# EUROPEAN PATENT APPLICATION

(11) **EP 1 440 981 A2**
(43) Date of publication of application: **28.07.2004**
(21) Application number: 04001196.7
(22) Date of filing: 21.01.2004
(51) Int. Cl.: C07K 14/47, C07K 14/705, C07K 16/18, G01N 33/53, C12Q 1/68, C12P 21/00, C12N 15/11, C12N 15/12, C12N 9/00, G06F 19/00

(54) **Full-length human cdna**

(30) Priority: 21.01.2003 JP 2003102206; 09.05.2003 JP 2003131392
(71) Applicant: Research Association for Biotechnology, Tokyo 105-0003 (JP)
(72) Inventor: Isogai, Takao, Inashiki-gun Ibaraki 300-0303 (JP); Sugiyama, Tomoyasu, Tokyo 130-0003 (JP); Otsuki, Tetsuji, Nishitokyo -shi Tokyo 188-0001 (JP); Wakamatsu, Ai, Kisarazu-shi Chiba 292-0014 (JP); Sato, Hiroyuki, Toyonaka-shi Osaka 560-0021 (JP); Ishii, Shizuko, Shiraoi-gun Hokkaido 059-0914 (JP); Yamamoto, Junichi, Kisarazu-shi Chiba 292-0014 (JP); Isono, Yuko, Kisarazu-shi Chiba 292-0014 (JP); Nagai, Keiichi, Higashiyamato-shi Tokyo 207-0022 (JP); Irie, Ryotaro, Saitama-shi Saitama 336-0936 (JP)
(74) Representative: VOSSIUS & PARTNER

(57) **Abstract**

Novel full-length cDNAs are provided.

2,495 cDNA derived from human have been isolated. The full-length nucleotide sequences of the cDNA and amino acid sequences encoded by the nucleotide sequences have been determined. Because the cDNA of the present invention are full-length and contain the translation start site, they provide information useful for analyzing the functions of the polypeptide.

## Description

### FIELD OF THE INVENTION

The present invention relates to polynucleotides encoding novel polypeptides, the polypeptides encoded by these polynucleotides, and new uses of these.

### BACKGROUND OF THE INVENTION

The genomic DNAs of various living organisms are currently being sequenced and analyzed all over the world. The entire genomic sequences of more than 40 species of prokaryotes, a lower eukaryote, yeast, a multicellular eukaryote, *C. elegans*, and a higher plant, *arabidopsis*, and such have already been determined. Analysis of the human genome, presumed to have three billion base pairs, was advanced under global cooperative organization, and a draft sequence was disclosed in 2001. In 2003 the complete structure had been elucidated and publically disclosed. A genome is a blueprint for highly complicated living organisms. The aim in determining a genomic sequence is to reveal the function and regulation of all genes, and to understand living organisms as a network of interactions between genes, proteins, cells or individuals. Understanding living organisms through the genomic information of various species is not only academically important, but also socially significant from the viewpoint of industrial application.

However, simply determining a genomic sequence will not reveal the function of all genes. For example, in the case of yeast, the function of only approximately half of the 6000 genes predicted on the basis of genomic sequence have been deduced. The human genome has been estimated to contain about 30,000 to 40,000 genes. Further, 100,000 or more types of mRNAs are said to exist when variants produced by alternative splicing are taken into consideration. Therefore, it is desirable to establish "a high throughput system for analysis of gene functions" which allows rapid and efficient identification of the functions of vast amounts of genes obtained by genomic sequencing.

Many genes in the eukaryotic genome are split by introns into multiple exons. Thus, it is difficult to correctly predict the structure of an encoded protein based solely on genomic information. In contrast, cDNA, which is produced from mRNA that lacks introns, encodes a protein as a single continuous amino acid sequence and allows easy identification of the protein's primary structure. In human cDNA research to date, more than three million ESTs (Expression Sequence Tags) are publicly available, which presumably covers no less than 80% of all human genes.

EST information is utilized in a variety of ways, for example in analyzing the structure of the human genome, or in predicting exon regions of genomic sequences or their expression profiles. However, most human ESTs have been derived from regions proximal to the cDNA 3'-end, and little information is available from around the mRNA 5'-end. In human cDNAs, the full-length protein sequence of approximately 15,000 corresponding mRNAs have been deduced.

It is possible to identify the mRNA transcription start site on the genomic sequence based on the 5'-end sequence of a full-length cDNA, and to analyze factors involved in the stability of mRNA contained in that sequence, or in the regulation of its expression at the translation stage. Also, since the atg codon, or translation start site, is contained in the 5'-region of a full-length cDNA, translation to proteins will occur in the correct frame. Therefore, it is possible to produce a large amount of the protein encoded by the cDNA, or to analyze the biological activity of the expressed protein by utilizing an appropriate expression system. Thus, analysis of full-length cDNA provides valuable information which complements genome sequencing information. Also, full-length cDNA clones that can be expressed are extremely valuable in empirical analysis of gene function and in industrial application.

Therefore, if a novel human full-length cDNA can be isolated, it can be used for developing medicines for diseases in which its gene is involved. A protein encoded by such a gene can be used as a drug by itself. Thus, obtaining full-length cDNAs encoding novel human proteins is of great significance.

In particular, human secretory proteins or membrane proteins would be useful used as medicines in the same manner as tissue plasminogen activator (TPA), or as target proteins for medicines like membrane receptors. In addition, genes for signal transduction-related proteins (protein kinases, etc.), glycoprotein-related proteins, transcription-related proteins, and such, are genes whose relationships to human diseases have been elucidated. Moreover, genes for disease-related proteins form a gene group rich in genes whose relationships to human diseases have been elucidated.

Isolating novel full-length human cDNA clones, only a few of which have been isolated, is of great significance. The isolation of novel cDNA clones encoding secretory proteins or membrane proteins is especially desired since such proteins would be useful in themselves as medicines, and also their clones would potentially include genes involved in disease. In addition, genes encoding proteins involved in signal transduction, glycoprotein, transcription, or disease, are expected to be useful as target molecules for therapy, or as medicines in themselves. These genes form a gene group predicted to be strongly involved in disease. Thus, identification of full-length cDNA clones encoding these proteins has great significance.

### SUMMARY OF THE INVENTION

An objective of the present invention is to provide polynucleotides encoding novel polypeptides, polypeptides encoded by the polynucleotides, and novel usages of these.

The inventors have developed a method for efficiently cloning, from a cDNA library having a very high fullness-ratio, human full-length cDNAs predicted to be full-length cDNA clones, where that cDNA library is synthesized by an improved method (WO 01/04286) of oligo-capping (K. Maruyama and S. Sugano, Gene, 138: 171-174 (1994); Y. Suzuki et al., Gene, 200: 149-156 (1997)). The nucleotide sequences of cDNA clones whose fullness ratio is high, obtained by this method, were determined mainly from their 5'-end, and, if required, from their 3'-end.

Among the clones obtained, representative clones estimated to be novel and full-length were analyzed for their full-length nucleotide sequence. The determined full-length nucleotide sequences were analyzed using a BLAST homology search of the databases shown below. Homology searches of the present invention were carried out based on full-length cDNA information, including the entire coding region, and thus homology to every part of a polypeptide could be analyzed. Therefore, in the present invention, the reliability of homology searches has been greatly improved.
[1] SwissProt (http://www.ebi.ac.uk/ebi docsSwissProt db/swisshome.html),
[2] GenBank (http://www.ncbi.nlm.nih.gov/web/GenBank),
[3] UniGene (Human) (http://www.ncbi.nlm.nih.gov/UniGene),
[4] nr (a protein database, which has been constructed by combining data of coding sequences (CDS) in nucleotide sequences deposited in GenBank, and data of SwissProt, PDB (http://www.rcsb.org/pdb/index.html), PIR (http://pir.georgetown.edu/pirwww/pirhome.shtml), and PRF (http://www.prf.or.jp/en/); overlapping sequences have been removed.), and
[5] RefSeq (http://www.ncbi.nlm.nih.gov/LocusLink/refseq.html).

The gene expression profiles of cDNA clones whose full-length nucleotide sequence had been determined were studied by analyzing the large-scale cDNA database constructed based on the 5'-end nucleotide sequences of cDNAs obtained. The present inventors revealed the usefulness of the genes of the present invention based on these analysis results.

In the present invention, gene functions were revealed by analysis of expression profiles *in silico*, based on full-length nucleotide sequence information. The expression profiles used in the expression frequency analysis were studied based on databases containing a sufficient amount of fragment sequence data. Expression frequency analysis was carried out by referring, for these expression profiles, to the full-length nucleotide sequences of the many cDNA clones obtained in the present invention. Thus, highly reliable analysis was achieved by referring to the full-length nucleotide sequences of a wide variety of genes in a sufficiently large population for analysis (expression profiles). Namely, the results of expression frequency analysis using the full-length sequences of the present invention more precisely reflect gene expression frequency in tissues and cells from which a certain cDNA library was derived. Thus, the full-length cDNA nucleotide sequence information of the present invention made it possible to achieve highly reliable expression frequency analysis.

The full-length cDNA clones of the present invention were obtained by a method comprising the steps of [1] preparing libraries containing cDNAs with a high fullness ratio using oligo-capping, and [2] assembling 5'-end sequences and selecting those with the highest probability of completeness in length in the cluster formed (there are many clones longer in the 5'-end direction). The use of primers designed based on the 5'- and 3'-end sequences of polynucleotides provided by the present invention enable full-length cDNAs to be readily obtained without using such special techniques. Primers, which are designed for use in obtaining cDNAs capable of being expressed, are not limited to the 5'- and 3'-end sequences of a polynucleotide.

Specifically, the present invention relates to polynucleotides and proteins encoded by the polypeptides as follows.
[1] A polynucleotide selected from the group consisting of:
   (a) a polynucleotide comprising a protein-coding region of the nucleotide sequence according to any one of SEQ ID NOs: 1-2188 and SEQ ID NOs: 4377-4683;
   (b) a polynucleotide comprising the nucleotide sequence encoding a polypeptide that comprises the amino acid sequence of any one of SEQ ID NOs: 2189-4376 and SEQ ID NOs: 4684-4990;
   (c) a polynucleotide comprising a nucleotide sequence encoding a polypeptide, which comprises the amino acid sequence selected from SEQ ID NO: SEQ ID NOs: 2189-4376 and SEQ ID NOs: 4684-4990 wherein one or more amino acids have been substituted, deleted, inserted, and/or added, and which is functionally equivalent to the polypeptide comprising the selected amino acid sequence as described above;
   (d) a polynucleotide which hybridizes to a polynucleotide comprising the nucleotide sequence selected from SEQ ID NOs: 1-2188 and SEQ ID NOs: 4377-4683, and which comprises the nucleotide sequence encoding a polypeptide functionally equivalent to a polypeptide encoded by the selected nucleotide sequence as described above;
   (e) a polynucleotide comprising a nucleotide sequence encoding a partial amino acid sequence of a polypeptide encoded by the polynucleotides according to any one of (a)-(d);
   (f) a polynucleotide comprising a nucleotide sequence having at least 70% identity to the nucleotide sequence of any one of SEQ ID NOs: 1-2188 and SEQ ID NOs: 4377-4683; and
   (g) a polynucleotide comprising a nucleotide sequence having at least 90% identity to the nucleotide sequence of any one of SEQ ID NOs: 1-2188 and SEQ ID NOs: 4377-4683.
[2] A polypeptide encoded by the polynucleotide according to [1], or a partial peptide thereof.
[3] An antibody which binds to the polypeptide or the peptide according to [2].
[4] An immunoassay method for the polypeptide or the peptide according to [2], which comprises the steps of:
   (a) contacting the polypeptide or the peptide according to [2] with the antibody according to [3]; and
   (b) observing the binding between the two.
[5] A vector comprising the polynucleotide according to [1].
[6] A transformant comprising the polynucleotide according to [1] or the vector according to [5].
[7] A transformant which comprises the polynucleotide according to [1] or vector according to [5] in an expressible manner.
[8] A method for producing the polypeptide or the peptide according to [2], which comprises the steps of:
   (a) culturing the transformant according to [7]; and
   (b) recovering the expression product.
[9] An oligonucleotide comprising 15 or more nucleotides, which comprises the nucleotide sequence according to any one of SEQ ID NOs: 1-2188 and SEQ ID NOs: 4377-4683, or a nucleotide sequence complementary to the complementary strand thereof.
[10] A primer for synthesizing a polynucleotide, which comprises the oligonucleotide according to [9].
[11] A probe for detecting a polynucleotide, which comprises the oligonucleotide according to [9].
[12] A polynucleotide according to any one of:
   (a) an antisense polynucleotide comprising a nucleotide sequence complementary to the transcript of the polynucleotide according to [1];
   (b) a polynucleotide with the ribozyme activity for specifically cleaving the transcript of the polynucleotide according to [1]; and
   (c) a polynucleotide which downregulates the expression of the polynucleotide of [1] due to RNAi activity in a host cell.
[13] A method for detecting the polynucleotide according to [1], which comprises the steps of:
   (a) incubating a target polynucleotide with the oligonucleotide according to [9] under conditions ensuring hybridization; and
   (b) detecting the hybridization between the target polynucleotide and the oligonucleotide according to [9].
[14] A database of polynucleotides and/or polypeptides, which comprises information on at least one of the nucleotide sequences of SEQ ID NOs: 1-2188 and SEQ ID NOs: 4377-4683 and/or on at least one of the amino acid sequences of SEQ ID NOs: 2189-4376 and SEQ ID NOs: 4684-4990.

Herein, "polynucleotide" is defined as a molecule, such as a DNA or RNA, in which multiple nucleotides are polymerized. There is no limitation as to the number of polymerized nucleotides. If a polymer contains a relatively low number of nucleotides, it is also described as an "oligonucleotide", which is included in the "polynucleotide" of the present invention. The polynucleotides or oligonucleotides of the present invention can be natural or chemically synthesized. Alternatively, they can be synthesized using a template polynucleotide in an enzymatic reaction such as PCR. Furthermore, the polynucleotides of the present invention may be modified chemically. Single-stranded and double-stranded polynucleotides are included in the present invention. In this specification, especially in the claims, when the polynucleotides are described merely as "polynucleotide", it means not only single-stranded polynucleotides but also double-stranded polynucleotides. When it means a double-stranded polynucleotide, the nucleotide sequence of only one chain is indicated. However, based on the nucleotide sequence of a sense chain, the nucleotide sequence of the corresponding complementary strand is essentially determined.

All the cDNAs provided by the present invention are full-length cDNAs. "Full-length cDNAs" herein means cDNAs containing the ATG codon, which is the start point of translation therein. Untranslated regions upstream and downstream of the protein-coding region are both naturally contained in natural mRNAs and are not essential. It is preferable that the full-length cDNAs of the present invention contain a stop codon.

### BRIEF DESCRIPTION OF THE DRAWINGS

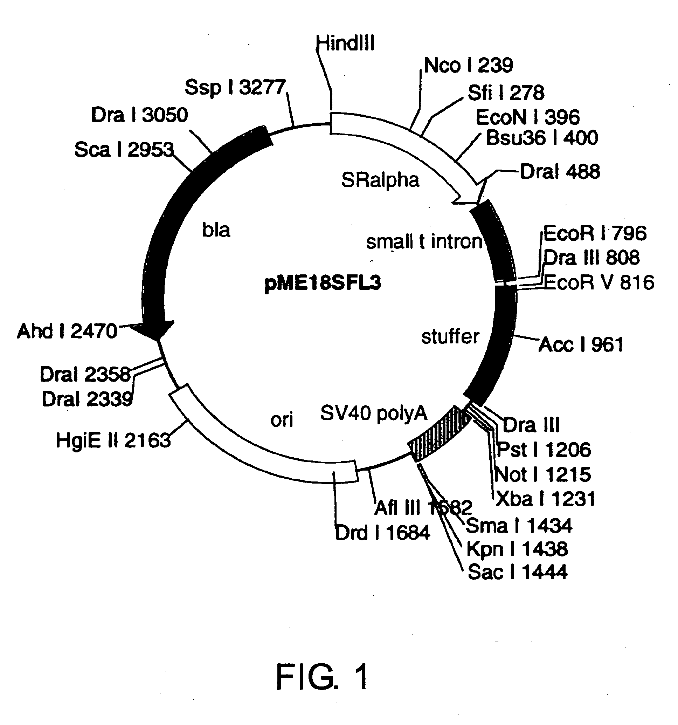

Fig. 1 shows the restriction map of the vector pME18SFL3.

### DETAILED DESCRIPTION OF THE INVENTION

All of the clones (2495 clones) of the present invention are novel and encode full-length polypeptides. Further, all of the clones are cDNAs with a high fullness ratio, and which were obtained by oligo-capping method. None of the clones are identical to any known human mRNAs (namely, they are novel clones) selected by searching 5'-end sequences and mRNA sequences with the annotation of "complete cds" in the GenBank and UniGene databases using BLAST homology [S. F. Altschul, W. Gish, W. Miller, E. W. Myers & D. J. Lipman, J. Mol. Biol., 215: 403-410 (1990); W. Gish & D. J. States, Nature Genet., 3: 266-272 (1993)]. They are also clones that were assumed to have a higher fullness ratio among members in assembled clusters. Most of the clones with a high fullness ratio in a cluster had nucleotide sequences longer in the 5'-end direction.

All of the full-length cDNAs of the present invention can be synthesized by a method such as PCR (Current protocols in Molecular Biology edit. Ausubel et al. (1987) Publish. John Wiley & Sons Section 6.1-6.4) using primer sets designed based on 5'-end and 3'-end sequences, or using primer sets of primers designed based on 5'-end sequences and a primer of oligo dT sequence corresponding to poly A sequence. Table 1 contains the clone names of 2,495 full-length cDNA clones of the present invention, SEQ ID NOs of the full-length nucleotide sequences, CDS portions deduced from the full-length nucleotide sequences, and SEQ ID NOs of the translated amino acids. The positions of CDS are shown according to the rules set out in "DDBJ/EMBL/GenBank Feature Table Definition" (http://www.ncbi.nlm.nih.gov/collab/FT/index.html). The start position number corresponds to the first letter of "ATG", the nucleotide triplet encoding methionine; the termination position number corresponds to the third letter of the stop codon. These are indicated by flanking with the mark "..". However, in clones without a stop codon, the termination position is indicated by the mark ">", according to the above rules.

**Table 1**

| | | | |
|---|---|---|---|
| 3NB692002685 | 1 | 777..1496 | 2189 |
| 3NB692002806 | 2 | 33.. 674 | 2190 |
| 3NB692008729 | 3 | 455..1189 | 2191 |
| ACTVT2000380 | 4 | 593.. 919 | 2192 |
| ADIPS2000088 | 5 | 59..1486 | 2193 |
| ADRGL2000172 | 6 | 237.. 638 | 2194 |
| ADRGL2003329 | 7 | 640..1014 | 2195 |
| ADRGL2009146 | 8 | 313.. 732 | 2196 |
| ADRGL2009691 | 9 | 294.. 767 | 2197 |
| ADRGL2009755 | 10 | 87..1265 | 2198 |
| ADRGL2012038 | 11 | 342.. 686 | 2199 |
| ADRGL2012179 | 12 | 254.. 625 | 2200 |
| ASTRO1000009 | 13 | 458..1552 | 2201 |
| ASTRO2002842 | 14 | 150..1490 | 2202 |
| ASTRO2003960 | 15 | 422.. 895 | 2203 |
| ASTRO2014923 | 16 | 65..1357 | 2204 |
| ASTRO2018373 | 17 | 123.. 512 | 2205 |
| ASTRO3000172 | 18 | 2582..2890 | 2206 |
| ASTRO3000177 | 19 | 2311..3978 | 2207 |
| ASTRO3000301 | 20 | 125..3832 | 2208 |
| ASTRO3000482 | 21 | 90.. 557 | 2209 |
| BLADE1000176 | 22 | 1787..2101 | 2210 |
| BLADE2001371 | 23 | 1009..1323 | 2211 |
| BLADE2001987 | 24 | 186.. 548 | 2212 |
| BLADE2002073 | 25 | 481.. 801 | 2213 |
| BLADE2002782 | 26 | 1341..1667 | 2214 |
| BLADE2002947 | 27 | 7.. 336 | 2215 |
| BLADE2003474 | 28 | 967..1272 | 2216 |
| BLADE2004089 | 29 | 60..>2486 | 2217 |
| BLADE2004462 | 30 | 96.. 512 | 2218 |
| BLADE2004670 | 31 | 2037..>2574 | 2219 |
| BLADE2005036 | 32 | 1293..1838 | 2220 |
| BLADE2005459 | 33 | 306.. 977 | 2221 |
| BLADE2007666 | 34 | 2289..2612 | 2222 |
| BLADE2007958 | 35 | 1147..1530 | 2223 |
| BLADE2008281 | 36 | 26.. 370 | 2224 |
| BLADE2008398 | 37 | 2742..3422 | 2225 |
| BLADE2008539 | 38 | 2857..4245 | 2226 |
| BNGH42003570 | 39 | 98.. 802 | 2227 |
| BNGH42007788 | 40 | 472..1782 | 2228 |
| BRACE1000186 | 41 | 333.. 821 | 2229 |
| BRACE1000258 | 42 | 10..2538 | 2230 |
| BRACE1000533 | 43 | 1483..1908 | 2231 |
| BRACE1000572 | 44 | 16..3435 | 2232 |
| BRACE2003639 | 45 | 481..1065 | 2233 |
| BRACE2005457 | 46 | 128..1237 | 2234 |
| BRACE2006319 | 47 | 603..1517 | 2235 |
| BRACE2008594 | 48 | 1221..1871 | 2236 |
| BRACE2010489 | 49 | 262..>1693 | 2237 |
| BRACE2011747 | 50 | 983..1438 | 2238 |
| BRACE2014306 | 51 | 32..1402 | 2239 |
| BRACE2014475 | 52 | 598.. 906 | 2240 |
| BRACE2014657 | 53 | 404.. 814 | 2241 |
| BRACE2015058 | 54 | 670..1116 | 2242 |
| BRACE2015314 | 55 | 203..1822 | 2243 |
| BRACE2016981 | 56 | 51..1355 | 2244 |
| BRACE2018762 | 57 | 654..1739 | 2245 |
| BRACE2024627 | 58 | 438.. 950 | 2246 |
| BRACE2026836 | 59 | 134..1087 | 2247 |
| BRACE2027258 | 60 | 164..>1851 | 2248 |
| BRACE2027970 | 61 | 1538..1990 | 2249 |
| BRACE2028970 | 62 | 2257..2685 | 2250 |
| BRACE2029112 | 63 | 832..1191 | 2251 |
| BRACE2029849 | 64 | 1039..1341 | 2252 |
| BRACE2030326 | 65 | 344.. 688 | 2253 |
| BRACE2030341 | 66 | 1209..1694 | 2254 |
| BRACE2030884 | 67 | 1248..1601 | 2255 |
| BRACE2031154 | 68 | 2.. 367 | 2256 |
| BRACE2031389 | 69 | 690..1271 | 2257 |
| BRACE2031527 | 70 | 367.. 684 | 2258 |
| BRACE2031531 | 71 | 120.. 635 | 2259 |
| BRACE2031899 | 72 | 46.. 381 | 2260 |
| BRACE2032044 | 73 | 403.. 732 | 2261 |
| BRACE2032329 | 74 | 1330..1761 | 2262 |
| BRACE2032385 | 75 | 20.. 613 | 2263 |
| BRACE2032538 | 76 | 189.. 515 | 2264 |
| BRACE2032823 | 77 | 1910..2212 | 2265 |
| BRACE2033720 | 78 | 28.. 537 | 2266 |
| BRACE2035381 | 79 | 1310..2758 | 2267 |
| BRACE2035441 | 80 | 98..1660 | 2268 |
| BRACE2036005 | 81 | 1584..1913 | 2269 |
| BRACE2036096 | 82 | 1173..1484 | 2270 |
| BRACE2036830 | 83 | 391.. 702 | 2271 |
| BRACE2036834 | 84 | 1436..1894 | 2272 |
| BRACE2037847 | 85 | 122.. 616 | 2273 |
| BRACE2038114 | 86 | 171.. 536 | 2274 |
| BRACE2038329 | 87 | 335.. 928 | 2275 |
| BRACE2038551 | 88 | 1913..2239 | 2276 |
| BRACE2039249 | 89 | 1085..1726 | 2277 |
| BRACE2039327 | 90 | 361..1419 | 2278 |
| BRACE2039475 | 91 | 307.. 753 | 2279 |
| BRACE2039734 | 92 | 16..>1730 | 2280 |
| BRACE2040138 | 93 | 797..1216 | 2281 |
| BRACE2040325 | 94 | 356.. 757 | 2282 |
| BRACE2041009 | 95 | 29..1390 | 2283 |
| BRACE2041200 | 96 | 304.. 945 | 2284 |
| BRACE2041264 | 97 | 530..1105 | 2285 |
| BRACE2042550 | 98 | 15..1007 | 2286 |
| BRACE2043142 | 99 | 156.. 908 | 2287 |
| BRACE2043248 | 100 | 1099..1581 | 2288 |
| BRACE2043349 | 101 | 202.. 531 | 2289 |
| BRACE2043665 | 102 | 858..1565 | 2290 |
| BRACE2044286 | 103 | 125..2011 | 2291 |
| BRACE2044816 | 104 | 188.. 670 | 2292 |
| BRACE2044949 | 105 | 24.. 725 | 2293 |
| BRACE2045300 | 106 | 1174..1863 | 2294 |
| BRACE2045428 | 107 | 209.. 625 | 2295 |
| BRACE2045596 | 108 | 906..1583 | 2296 |
| BRACE2045772 | 109 | 31.. 777 | 2297 |
| BRACE2045947 | 110 | 499..1164 | 2298 |
| BRACE2045954 | 111 | 1558..1863 | 2299 |
| BRACE2046251 | 112 | 527..1360 | 2300 |
| BRACE2046295 | 113 | 436..1428 | 2301 |
| BRACE2047011 | 114 | 1453..1794 | 2302 |
| BRACE2047350 | 115 | 2840..3487 | 2303 |
| BRACE2047377 | 116 | 133.. 456 | 2304 |
| BRACE2047385 | 117 | 75.. 458 | 2305 |
| BRACE3000071 | 118 | 1729..2124 | 2306 |
| BRACE3000697 | 119 | 131.. 703 | 2307 |
| BRACE3000787 | 120 | 2208..2750 | 2308 |
| BRACE3000840 | 121 | 564..3611 | 2309 |
| BRACE3000973 | 122 | 887..1666 | 2310 |
| BRACE3001002 | 123 | 292.. 624 | 2311 |
| BRACE3001217 | 124 | 2924..3241 | 2312 |
| BRACE3001391 | 125 | 1803..3506 | 2313 |
| BRACE3001595 | 126 | 8.. 952 | 2314 |
| BRACE3001754 | 127 | 586..1188 | 2315 |
| BRACE3002298 | 128 | 67.. 375 | 2316 |
| BRACE3002390 | 129 | 136.. 537 | 2317 |
| BRACE3002508 | 130 | 3430..4104 | 2318 |
| BRACE3003004 | 131 | 692..1027 | 2319 |
| BRACE3003192 | 132 | 1044..3245 | 2320 |
| BRACE3003595 | 133 | 1654..4179 | 2321 |
| BRACE3003698 | 134 | 506.. 847 | 2322 |
| BRACE3004058 | 135 | 2267..3040 | 2323 |
| BRACE3004113 | 136 | 1887..2189 | 2324 |
| BRACE3004150 | 137 | 1537..2886 | 2325 |
| BRACE3004358 | 138 | 222.. 551 | 2326 |
| BRACE3004435 | 139 | 2782..3240 | 2327 |
| BRACE3004772 | 140 | 888..1577 | 2328 |
| BRACE3004783 | 141 | 132.. 644 | 2329 |
| BRACE3004843 | 142 | 183.. 500 | 2330 |
| BRACE3004880 | 143 | 885..1655 | 2331 |
| BRACE3005145 | 144 | 717..1484 | 2332 |
| BRACE3005225 | 145 | 76.. 495 | 2333 |
| BRACE3005430 | 146 | 3120..3524 | 2334 |
| BRACE3005499 | 147 | 205.. 531 | 2335 |
| BRACE3006185 | 148 | 220.. 648 | 2336 |
| BRACE3006226 | 149 | 580.. 951 | 2337 |
| BRACE3006462 | 150 | 1851..2213 | 2338 |
| BRACE3006872 | 151 | 1136..1957 | 2339 |
| BRACE3007322 | 152 | 2918..>3227 | 2340 |
| BRACE3007472 | 153 | 190.. 573 | 2341 |
| BRACE3007480 | 154 | 93.. 962 | 2342 |
| BRACE3007559 | 155 | 1236..1592 | 2343 |
| BRACE3007625 | 156 | 36..3053 | 2344 |
| BRACE3007642 | 157 | 1857..2294 | 2345 |
| BRACE3007767 | 158 | 237.. 758 | 2346 |
| BRACE3008036 | 159 | 162.. 512 | 2347 |
| BRACE3008092 | 160 | 3356..3724 | 2348 |
| BRACE3008137 | 161 | 208..3699 | 2349 |
| BRACE3008384 | 162 | 123..1043 | 2350 |
| BRACE3008720 | 163 | 3014..3550 | 2351 |
| BRACE3008772 | 164 | 3900..4331 | 2352 |
| BRACE3009090 | 165 | 70.. 585 | 2353 |
| BRACE3009237 | 166 | 406.. 714 | 2354 |
| BRACE3009297 | 167 | 2724..3107 | 2355 |
| BRACE3009377 | 168 | 1359..1790 | 2356 |
| BRACE3009574 | 169 | 180.. 527 | 2357 |
| BRACE3009701 | 170 | 1011..1550 | 2358 |
| BRACE3009708 | 171 | 105..2867 | 2359 |
| BRACE3009724 | 172 | 2401..3282 | 2360 |
| BRACE3009747 | 173 | 2650..3171 | 2361 |
| BRACE3010397 | 174 | 1848..2660 | 2362 |
| BRACE3010428 | 175 | 269.. 628 | 2363 |
| BRACE3011271 | 176 | 689..1417 | 2364 |
| BRACE3011421 | 177 | 372..3209 | 2365 |
| BRACE3011505 | 178 | 688..1035 | 2366 |
| BRACE3012364 | 179 | 1980..2633 | 2367 |
| BRACE3012930 | 180 | 1128..1718 | 2368 |
| BRACE3013119 | 181 | 66.. 395 | 2369 |
| BRACE3013576 | 182 | 2120..2971 | 2370 |
| BRACE3013740 | 183 | 118.. 447 | 2371 |
| BRACE3013780 | 184 | 6..1124 | 2372 |
| BRACE3014005 | 185 | 1460..2374 | 2373 |
| BRACE3014068 | 186 | 29.. 373 | 2374 |
| BRACE3014231 | 187 | 928..1347 | 2375 |
| BRACE3014317 | 188 | 44.. 550 | 2376 |
| BRACE3014807 | 189 | 144..1202 | 2377 |
| BRACE3015027 | 190 | 1..1656 | 2378 |
| BRACE3015121 | 191 | 3019..4404 | 2379 |
| BRACE3015262 | 192 | 27..2195 | 2380 |
| BRACE3015521 | 193 | 2117..2827 | 2381 |
| BRACE3015894 | 194 | 285.. 773 | 2382 |
| BRACE3016884 | 195 | 303..2633 | 2383 |
| BRACE3018308 | 196 | 106.. 612 | 2384 |
| BRACE3018963 | 197 | 332.. 676 | 2385 |
| BRACE3019055 | 198 | 1065..1940 | 2386 |
| BRACE3019084 | 199 | 1644..2369 | 2387 |
| BRACE3020194 | 200 | 1727..2161 | 2388 |
| BRACE3020286 | 201 | 85.. 426 | 2389 |
| BRACE3020594 | 202 | 250.. 624 | 2390 |
| BRACE3022769 | 203 | 299.. 790 | 2391 |
| BRACE3023912 | 204 | 12.. 539 | 2392 |
| BRACE3024073 | 205 | 86.. 655 | 2393 |
| BRACE3024659 | 206 | 14.. 586 | 2394 |
| BRACE3024662 | 207 | 401.. 925 | 2395 |
| BRACE3025153 | 208 | 98.. 523 | 2396 |
| BRACE3025457 | 209 | 1204..2541 | 2397 |
| BRACE3025531 | 210 | 1319..2338 | 2398 |
| BRACE3025630 | 211 | 1329..1919 | 2399 |
| BRACE3026008 | 212 | 250.. 558 | 2400 |
| BRACE3026075 | 213 | 130.. 483 | 2401 |
| BRACE3026735 | 214 | 324.. 635 | 2402 |
| BRACE3027242 | 215 | 273.. 791 | 2403 |
| BRACE3027326 | 216 | 237..2372 | 2404 |
| BRACE3027478 | 217 | 32.. 529 | 2405 |
| BRACE3030103 | 218 | 320.. 682 | 2406 |
| BRACE3031838 | 219 | 1661..1981 | 2407 |
| BRACE3032983 | 220 | 155.. 484 | 2408 |
| BRACE3040856 | 221 | 187.. 585 | 2409 |
| BRACE3045033 | 222 | 24.. 566 | 2410 |
| BRALZ2011796 | 223 | 132..1361 | 2411 |
| BRALZ2012183 | 224 | 2172..2741 | 2412 |
| BRALZ2012848 | 225 | 218.. 754 | 2413 |
| BRALZ2014484 | 226 | 80..1411 | 2414 |
| BRALZ2016085 | 227 | 217..>1672 | 2415 |
| BRALZ2016498 | 228 | 402.. 893 | 2416 |
| BRALZ2017359 | 229 | 47.. 973 | 2417 |
| BRAMY2001473 | 230 | 112..1701 | 2418 |
| BRAMY2003008 | 231 | 236.. 961 | 2419 |
| BRAMY2004771 | 232 | 240..2108 | 2420 |
| BRAMY2005052 | 233 | 211..1434 | 2421 |
| BRAMY2017528 | 234 | 447..1076 | 2422 |
| BRAMY2019300 | 235 | 338..2110 | 2423 |
| BRAMY2019963 | 236 | 206.. 640 | 2424 |
| BRAMY2019985 | 237 | 271.. 573 | 2425 |
| BRAMY2020058 | 238 | 1537..1962 | 2426 |
| BRAMY2020270 | 239 | 98.. 976 | 2427 |
| BRAMY2021498 | 240 | 71..1870 | 2428 |
| BRAMY2028856 | 241 | 16.. 333 | 2429 |
| BRAMY2028914 | 242 | 34.. 534 | 2430 |
| BRAMY2029602 | 243 | 216.. 731 | 2431 |
| BRAMY2030098 | 244 | 960..1298 | 2432 |
| BRAMY2030109 | 245 | 271..1296 | 2433 |
| BRAMY2030702 | 246 | 1008..1316 | 2434 |
| BRAMY2030703 | 247 | 132.. 611 | 2435 |
| BRAMY2030799 | 248 | 85.. 480 | 2436 |
| BRAMY2031317 | 249 | 313..2334 | 2437 |
| BRAMY2031377 | 250 | 214.. 642 | 2438 |
| BRAMY2031442 | 251 | 1492..1854 | 2439 |
| BRAMY2032014 | 252 | 1227..2054 | 2440 |
| BRAMY2032242 | 253 | 404..1150 | 2441 |
| BRAMY2032317 | 254 | 1649..1987 | 2442 |
| BRAMY2033003 | 255 | 736..1068 | 2443 |
| BRAMY2033116 | 256 | 1008..1721 | 2444 |
| BRAMY2033267 | 257 | 609.. 923 | 2445 |
| BRAMY2033594 | 258 | 2121..2447 | 2446 |
| BRAMY2034185 | 259 | 47.. 400 | 2447 |
| BRAMY2034920 | 260 | 222.. 620 | 2448 |
| BRAMY2034993 | 261 | 1613..2041 | 2449 |
| BRAMY2036387 | 262 | 1858..2160 | 2450 |
| BRAMY2036396 | 263 | 837..1169 | 2451 |
| BRAMY2036567 | 264 | 859..1338 | 2452 |
| BRAMY2036699 | 265 | 60.. 374 | 2453 |
| BRAMY2036913 | 266 | 86.. 433 | 2454 |
| BRAMY2037823 | 267 | 2163..2549 | 2455 |
| BRAMY2038100 | 268 | 515.. 997 | 2456 |
| BRAMY2038484 | 269 | 95.. 709 | 2457 |
| BRAMY2038846 | 270 | 1709..2356 | 2458 |
| BRAMY2038904 | 271 | 118..2274 | 2459 |
| BRAMY2039872 | 272 | 663..1148 | 2460 |
| BRAMY2040478 | 273 | 1754..2083 | 2461 |
| BRAMY2040592 | 274 | 954..1943 | 2462 |
| BRAMY2041261 | 275 | 124.. 441 | 2463 |
| BRAMY2041378 | 276 | 199.. 831 | 2464 |
| BRAMY2041542 | 277 | 2098..2412 | 2465 |
| BRAMY2042612 | 278 | 1319..1621 | 2466 |
| BRAMY2042641 | 279 | 271.. 672 | 2467 |
| BRAMY2042760 | 280 | 1306..2115 | 2468 |
| BRAMY2042918 | 281 | 956..1354 | 2469 |
| BRAMY2044078 | 282 | 123.. 569 | 2470 |
| BRAMY2044246 | 283 | 83.. 436 | 2471 |
| BRAMY2045036 | 284 | 2746..3069 | 2472 |
| BRAMY2046478 | 285 | 51.. 359 | 2473 |
| BRAMY2046742 | 286 | 819..1148 | 2474 |
| BRAMY2046989 | 287 | 47..1594 | 2475 |
| BRAMY2047169 | 288 | 1622..2083 | 2476 |
| BRAMY2047420 | 289 | 573..1055 | 2477 |
| BRAMY2047676 | 290 | 30.. 677 | 2478 |
| BRAMY2047746 | 291 | 109..1566 | 2479 |
| BRAMY2047751 | 292 | 859..2040 | 2480 |
| BRAMY2047765 | 293 | 2247..2549 | 2481 |
| BRAMY2047884 | 294 | 873..1199 | 2482 |
| BRAMY3000206 | 295 | 1072..1689 | 2483 |
| BRAMY3000213 | 296 | 2109..2888 | 2484 |
| BRAMY3001401 | 297 | 109.. 594 | 2485 |
| BRAMY3001794 | 298 | 51..1322 | 2486 |
| BRAMY3002312 | 299 | 58.. 372 | 2487 |
| BRAMY3002620 | 300 | 113..2752 | 2488 |
| BRAMY3002803 | 301 | 913..2823 | 2489 |
| BRAMY3002805 | 302 | 2036..2371 | 2490 |
| BRAMY3004224 | 303 | 414..1526 | 2491 |
| BRAMY3004672 | 304 | 1926..2402 | 2492 |
| BRAMY3004900 | 305 | 165.. 635 | 2493 |
| BRAMY3004919 | 306 | 539..3298 | 2494 |
| BRAMY3005091 | 307 | 592..1311 | 2495 |
| BRAMY3005932 | 308 | 2455..3075 | 2496 |
| BRAMY3006297 | 309 | 76.. 456 | 2497 |
| BRAMY3007206 | 310 | 720..1901 | 2498 |
| BRAMY3007609 | 311 | 744..1745 | 2499 |
| BRAMY3008466 | 312 | 1458..2342 | 2500 |
| BRAMY3008505 | 313 | 2733..3125 | 2501 |
| BRAMY3008650 | 314 | 656.. 958 | 2502 |
| BRAMY3009811 | 315 | 1842..2321 | 2503 |
| BRAMY3010411 | 316 | 707..1066 | 2504 |
| BRAMY4000095 | 317 | 736..2487 | 2505 |
| BRAMY4000229 | 318 | 1214..1957 | 2506 |
| BRAMY4000277 | 319 | 1027..2064 | 2507 |
| BRASW1000053 | 320 | 1315..1632 | 2508 |
| BRASW1000125 | 321 | 1099..1443 | 2509 |
| BRAWH1000127 | 322 | 622..1353 | 2510 |
| BRAWH2001395 | 323 | 696..1130 | 2511 |
| BRAWH2001671 | 324 | 1559..2077 | 2512 |
| BRAWH2001940 | 325 | 172..2208 | 2513 |
| BRAWH2001973 | 326 | 133.. 498 | 2514 |
| BRAWH2002560 | 327 | 176..2476 | 2515 |
| BRAWH2002761 | 328 | 555..1079 | 2516 |
| BRAWH2005315 | 329 | 161..1615 | 2517 |
| BRAWH2007658 | 330 | 45..1148 | 2518 |
| BRAWH2010000 | 331 | 275..2155 | 2519 |
| BRAWH2010084 | 332 | 1682..2191 | 2520 |
| BRAWH2010536 | 333 | 448..1284 | 2521 |
| BRAWH2012162 | 334 | 512..2068 | 2522 |
| BRAWH2012326 | 335 | 115.. 426 | 2523 |
| BRAWH2013294 | 336 | 762..1148 | 2524 |
| BRAWH2013871 | 337 | 756..1151 | 2525 |
| BRAWH2014414 | 338 | 266..2794 | 2526 |
| BRAWH2014645 | 339 | 940..1632 | 2527 |
| BRAWH2014662 | 340 | 285..1706 | 2528 |
| BRAWH2014876 | 341 | 148.. 840 | 2529 |
| BRAWH2014954 | 342 | 377..>2183 | 2530 |
| BRAWH2016221 | 343 | 1235..1891 | 2531 |
| BRAWH2016439 | 344 | 2332..2754 | 2532 |
| BRAWH2016702 | 345 | 1328..2347 | 2533 |
| BRAWH2016724 | 346 | 846..1292 | 2534 |
| BRAWH3000078 | 347 | 290.. 805 | 2535 |
| BRAWH3000100 | 348 | 3063..5708 | 2536 |
| BRAWH3000314 | 349 | 1322..3064 | 2537 |
| BRAWH3000345 | 350 | 1135..1452 | 2538 |
| BRAWH3000491 | 351 | 590.. 913 | 2539 |
| BRAWH3001326 | 352 | 73.. 987 | 2540 |
| BRAWH3001475 | 353 | 75.. 377 | 2541 |
| BRAWH3001891 | 354 | 11..1141 | 2542 |
| BRAWH3002574 | 355 | 1585..2217 | 2543 |
| BRAWH3002600 | 356 | 104..1795 | 2544 |
| BRAWH3002819 | 357 | 2589..3233 | 2545 |
| BRAWH3002821 | 358 | 383..3064 | 2546 |
| BRAWH3003522 | 359 | 69.. 554 | 2547 |
| BRAWH3003555 | 360 | 1048..1545 | 2548 |
| BRAWH3003727 | 361 | 1386..1694 | 2549 |
| BRAWH3003801 | 362 | 2985..3470 | 2550 |
| BRAWH3003992 | 363 | 2123..2677 | 2551 |
| BRAWH3004453 | 364 | 1891..2346 | 2552 |
| BRAWH3004666 | 365 | 1158..1562 | 2553 |
| BRAWH3005132 | 366 | 2935..3333 | 2554 |
| BRAWH3005422 | 367 | 14.. 535 | 2555 |
| BRAWH3005912 | 368 | 1384..3516 | 2556 |
| BRAWH3005981 | 369 | 160..2685 | 2557 |
| BRAWH3006548 | 370 | 2431..2760 | 2558 |
| BRAWH3006792 | 371 | 681..1313 | 2559 |
| BRAWH3007221 | 372 | 854..1345 | 2560 |
| BRAWH3007506 | 373 | 986..1459 | 2561 |
| BRAWH3007592 | 374 | 2933..3331 | 2562 |
| BRAWH3007726 | 375 | 352.. 780 | 2563 |
| BRAWH3007783 | 376 | 779..1435 | 2564 |
| BRAWH3008341 | 377 | 1608..2198 | 2565 |
| BRAWH3008634 | 378 | 1099..1515 | 2566 |
| BRAWH3008697 | 379 | 605..1294 | 2567 |
| BRAWH3008931 | 380 | 11.. 469 | 2568 |
| BRAWH3009297 | 381 | 1581..2141 | 2569 |
| BRCAN2002562 | 382 | 215.. 880 | 2570 |
| BRCAN2002856 | 383 | 380..1114 | 2571 |
| BRCAN2002944 | 384 | 22.. 609 | 2572 |
| BRCAN2002948 | 385 | 42.. 431 | 2573 |
| BRCAN2003703 | 386 | 579..1157 | 2574 |
| BRCAN2003746 | 387 | 1773..2543 | 2575 |
| BRCAN2003987 | 388 | 142.. 447 | 2576 |
| BRCAN2004355 | 389 | 722.. 1024 | 2577 |
| BRCAN2005436 | 390 | 45.. 764 | 2578 |
| BRCAN2006063 | 391 | 547..1278 | 2579 |
| BRCAN2006290 | 392 | 117.. 419 | 2580 |
| BRCAN2006297 | 393 | 490.. 999 | 2581 |
| BRCAN2006450 | 394 | 3.. 320 | 2582 |
| BRCAN2007144 | 395 | 94.. 636 | 2583 |
| BRCAN2007409 | 396 | 2280..>2620 | 2584 |
| BRCAN2007426 | 397 | 62.. 601 | 2585 |
| BRCAN2008528 | 398 | 311..1231 | 2586 |
| BRCAN2009203 | 399 | 1077..2408 | 2587 |
| BRCAN2009432 | 400 | 95.. 646 | 2588 |
| BRCAN2010376 | 401 | 227.. 571 | 2589 |
| BRCAN2011254 | 402 | 252.. 557 | 2590 |
| BRCAN2011602 | 403 | 224.. 748 | 2591 |
| BRCAN2012355 | 404 | 467..1009 | 2592 |
| BRCAN2012481 | 405 | 240.. 566 | 2593 |
| BRCAN2013655 | 406 | 268.. 606 | 2594 |
| BRCAN2013660 | 407 | 216.. 641 | 2595 |
| BRCAN2014143 | 408 | 15.. 584 | 2596 |
| BRCAN2014602 | 409 | 94.. 870 | 2597 |
| BRCAN2014881 | 410 | 279.. 758 | 2598 |
| BRCAN2015371 | 411 | 49.. 432 | 2599 |
| BRCAN2015464 | 412 | 491.. 955 | 2600 |
| BRCAN2016433 | 413 | 1679..1993 | 2601 |
| BRCAN2016619 | 414 | 77..2743 | 2602 |
| BRCAN2017442 | 415 | 82.. 492 | 2603 |
| BRCAN2017717 | 416 | 63.. 377 | 2604 |
| BRCAN2017905 | 417 | 38.. 532 | 2605 |
| BRCAN2018935 | 418 | 156.. 719 | 2606 |
| BRCAN2019387 | 419 | 9.. 314 | 2607 |
| BRCAN2020710 | 420 | 1738..2043 | 2608 |
| BRCAN2021028 | 421 | 94..1734 | 2609 |
| BRCAN2024451 | 422 | 184..1227 | 2610 |
| BRCAN2024563 | 423 | 1.. 384 | 2611 |
| BRCAN2025712 | 424 | 780..1187 | 2612 |
| BRCAN2028355 | 425 | 78..1805 | 2613 |
| BRCOC2000670 | 426 | 326.. 628 | 2614 |
| BRCOC2001505 | 427 | 1470..1904 | 2615 |
| BRCOC2003213 | 428 | 1821..3143 | 2616 |
| BRCOC2007034 | 429 | 404.. 931 | 2617 |
| BRCOC2014033 | 430 | 359.. 709 | 2618 |
| BRCOC2016525 | 431 | 153..1997 | 2619 |
| BRCOC2019934 | 432 | 5.. 385 | 2620 |
| BRCOC2020142 | 433 | 741..1130 | 2621 |
| BRHIP2000691 | 434 | 1545..1910 | 2622 |
| BRHIP2000819 | 435 | 1030..1611 | 2623 |
| BRHIP2000826 | 436 | 137.. 442 | 2624 |
| BRHIP2000920 | 437 | 836..1567 | 2625 |
| BRHIP2001074 | 438 | 553..1581 | 2626 |
| BRHIP2001805 | 439 | 70.. 528 | 2627 |
| BRHIP2001927 | 440 | 2.. 340 | 2628 |
| BRHIP2002122 | 441 | 530..1153 | 2629 |
| BRHIP2002172 | 442 | 673..2031 | 2630 |
| BRHIP2002346 | 443 | 159..1097 | 2631 |
| BRHIP2003242 | 444 | 72.. 575 | 2632 |
| BRHIP2003786 | 445 | 199..2013 | 2633 |
| BRHIP2003917 | 446 | 1870..2316 | 2634 |
| BRHIP2004312 | 447 | 1748..2200 | 2635 |
| BRHIP2004359 | 448 | 663..2027 | 2636 |
| BRHIP2004814 | 449 | 642..1778 | 2637 |
| BRHIP2004883 | 450 | 221.. 526 | 2638 |
| BRHIP2005236 | 451 | 709..1860 | 2639 |
| BRHIP2005354 | 452 | 569..1057 | 2640 |
| BRHIP2005600 | 453 | 492..1187 | 2641 |
| BRHIP2005719 | 454 | 445.. 909 | 2642 |
| BRHIP2005752 | 455 | 876..1919 | 2643 |
| BRHIP2005932 | 456 | 212.. 652 | 2644 |
| BRHIP2006800 | 457 | 178.. 774 | 2645 |
| BRHIP2007616 | 458 | 136..1614 | 2646 |
| BRHIP2007741 | 459 | 811..1122 | 2647 |
| BRHIP2009340 | 460 | 72.. 374 | 2648 |
| BRHIP2009414 | 461 | 828..1541 | 2649 |
| BRHIP2009474 | 462 | 38.. 469 | 2650 |
| BRHIP2013699 | 463 | 816..1328 | 2651 |
| BRHIP2014228 | 464 | 444.. 869 | 2652 |
| BRHIP2021615 | 465 | 282..1310 | 2653 |
| BRHIP2022221 | 466 | 2101..2478 | 2654 |
| BRHIP2024146 | 467 | 177.. 539 | 2655 |
| BRHIP2024165 | 468 | 923..1294 | 2656 |
| BRHIP2026061 | 469 | 282..1022 | 2657 |
| BRHIP2026288 | 470 | 668..1681 | 2658 |
| BRHIP2029176 | 471 | 778..1224 | 2659 |
| BRHIP2029393 | 472 | 2401..2931 | 2660 |
| BRHIP3000339 | 473 | 2793..3227 | 2661 |
| BRHIP3000526 | 474 | 107..2830 | 2662 |
| BRHIP3001283 | 475 | 626..1645 | 2663 |
| BRHIP3006683 | 476 | 292.. 630 | 2664 |
| BRHIP3007483 | 477 | 2669..3082 | 2665 |
| BRHIP3007586 | 478 | 1409..2047 | 2666 |
| BRHIP3008183 | 479 | 607..3861 | 2667 |
| BRHIP3008313 | 480 | 1604..2353 | 2668 |
| BRHIP3008344 | 481 | 1878..3719 | 2669 |
| BRHIP3008405 | 482 | 2679..3668 | 2670 |
| BRHIP3008565 | 483 | 1696..2055 | 2671 |
| BRHIP3008598 | 484 | 612..1079 | 2672 |
| BRHIP3008997 | 485 | 1196..1579 | 2673 |
| BRHIP3009099 | 486 | 74.. 892 | 2674 |
| BRHIP3009448 | 487 | 2785..3354 | 2675 |
| BRHIP3011241 | 488 | 220.. 642 | 2676 |
| BRHIP3013765 | 489 | 91.. 486 | 2677 |
| BRHIP3013897 | 490 | 2340..2843 | 2678 |
| BRHIP3015751 | 491 | 2103..2516 | 2679 |
| BRHIP3016213 | 492 | 402..1202 | 2680 |
| BRHIP3018797 | 493 | 2687..3004 | 2681 |
| BRHIP3020182 | 494 | 1515..2087 | 2682 |
| BRHIP3024118 | 495 | 67..1131 | 2683 |
| BRHIP3024533 | 496 | 236..>3741 | 2684 |
| BRHIP3024725 | 497 | 1840..4176 | 2685 |
| BRHIP3025161 | 498 | 225..4517 | 2686 |
| BRHIP3025702 | 499 | 3307..3609 | 2687 |
| BRHIP3026097 | 500 | 1387..1773 | 2688 |
| BRHIP3027137 | 501 | 261..1673 | 2689 |
| BRHIP3027854 | 502 | 2503..3213 | 2690 |
| BRSSN2000684 | 503 | 166..>1937 | 2691 |
| BRSSN2003086 | 504 | 1.. 342 | 2692 |
| BRSSN2004496 | 505 | 303..1469 | 2693 |
| BRSSN2004719 | 506 | 62.. 784 | 2694 |
| BRSSN2006892 | 507 | 1899..2396 | 2695 |
| BRSSN2008549 | 508 | 563..1564 | 2696 |
| BRSSN2008797 | 509 | 57..1571 | 2697 |
| BRSSN2011262 | 510 | 326.. 694 | 2698 |
| BRSSN2011738 | 511 | 1587..1928 | 2699 |
| BRSSN2013874 | 512 | 1430..1894 | 2700 |
| BRSSN2014299 | 513 | 641..1855 | 2701 |
| BRSSN2014424 | 514 | 861..2201 | 2702 |
| BRSSN2014556 | 515 | 359.. 679 | 2703 |
| BRSSN2018581 | 516 | 1440..1757 | 2704 |
| BRSSN2018925 | 517 | 31.. 372 | 2705 |
| BRSTN2000872 | 518 | 537..1649 | 2706 |
| BRSTN2001067 | 519 | 698..1048 | 2707 |
| BRSTN2001613 | 520 | 98.. 754 | 2708 |
| BRSTN2002400 | 521 | 82..1170 | 2709 |
| BRSTN2003835 | 522 | 25.. 420 | 2710 |
| BRSTN2004863 | 523 | 438..2264 | 2711 |
| BRSTN2004987 | 524 | 11.. 1093 | 2712 |
| BRSTN2005721 | 525 | 415..1302 | 2713 |
| BRSTN2006865 | 526 | 693..1559 | 2714 |
| BRSTN2007000 | 527 | 115.. 492 | 2715 |
| BRSTN2007284 | 528 | 96.. 608 | 2716 |
| BRSTN2008052 | 529 | 133.. 483 | 2717 |
| BRSTN2008283 | 530 | 179.. 484 | 2718 |
| BRSTN2008418 | 531 | 468..1013 | 2719 |
| BRSTN2008457 | 532 | 942..1331 | 2720 |
| BRSTN2009899 | 533 | 707..1135 | 2721 |
| BRSTN2010363 | 534 | 1125..1901 | 2722 |
| BRSTN2010500 | 535 | 703..1134 | 2723 |
| BRSTN2010750 | 536 | 247..1290 | 2724 |
| BRSTN2012320 | 537 | 300.. 686 | 2725 |
| BRSTN2012380 | 538 | 1068..1439 | 2726 |
| BRSTN2013741 | 539 | 648..1274 | 2727 |
| BRSTN2015015 | 540 | 1290..1610 | 2728 |
| BRSTN2016470 | 541 | 479.. 814 | 2729 |
| BRSTN2016678 | 542 | 97.. 486 | 2730 |
| BRSTN2017084 | 543 | 53.. 397 | 2731 |
| BRSTN2017110 | 544 | 128.. 562 | 2732 |
| BRSTN2017237 | 545 | 42..1466 | 2733 |
| BRSTN2017771 | 546 | 1018..1332 | 2734 |
| BRSTN2018083 | 547 | 309.. 641 | 2735 |
| BRSTN2019129 | 548 | 1004..1459 | 2736 |
| BRTHA1000311 | 549 | 210.. 680 | 2737 |
| BRTHA2000855 | 550 | 149..>2176 | 2738 |
| BRTHA2001462 | 551 | 710..1555 | 2739 |
| BRTHA2002115 | 552 | 947..1267 | 2740 |
| BRTHA2002281 | 553 | 856..1767 | 2741 |
| BRTHA2002376 | 554 | 124.. 513 | 2742 |
| BRTHA2002442 | 555 | 28.. 630 | 2743 |
| BRTHA2002493 | 556 | 400.. 735 | 2744 |
| BRTHA2002608 | 557 | 1100..1495 | 2745 |
| BRTHA2002808 | 558 | 1010..1456 | 2746 |
| BRTHA2003030 | 559 | 501.. 875 | 2747 |
| BRTHA2003110 | 560 | 1429..2010 | 2748 |
| BRTHA2003116 | 561 | 78.. 476 | 2749 |
| BRTHA2003461 | 562 | 2179..2709 | 2750 |
| BRTHA2004821 | 563 | 1764..2273 | 2751 |
| BRTHA2004978 | 564 | 125.. 937 | 2752 |
| BRTHA2005579 | 565 | 44..2488 | 2753 |
| BRTHA2005956 | 566 | 1466..1882 | 2754 |
| BRTHA2006075 | 567 | 591.. 995 | 2755 |
| BRTHA2006146 | 568 | 24.. 569 | 2756 |
| BRTHA2006194 | 569 | 129.. 506 | 2757 |
| BRTHA2007122 | 570 | 297..2267 | 2758 |
| BRTHA2007422 | 571 | 114.. 632 | 2759 |
| BRTHA2007603 | 572 | 534.. 962 | 2760 |
| BRTHA2008316 | 573 | 124.. 429 | 2761 |
| BRTHA2008335 | 574 | 1205..1747 | 2762 |
| BRTHA2008527 | 575 | 54.. 680 | 2763 |
| BRTHA2008535 | 576 | 2076..2597 | 2764 |
| BRTHA2008955 | 577 | 427..1395 | 2765 |
| BRTHA2009311 | 578 | 47.. 502 | 2766 |
| BRTHA2009846 | 579 | 492.. 881 | 2767 |
| BRTHA2009972 | 580 | 134.. 493 | 2768 |
| BRTHA2010073 | 581 | 1064..1399 | 2769 |
| BRTHA2010608 | 582 | 926..1354 | 2770 |
| BRTHA2010884 | 583 | 76..1305 | 2771 |
| BRTHA2010907 | 584 | 78.. 806 | 2772 |
| BRTHA2011194 | 585 | 2370..2720 | 2773 |
| BRTHA2011351 | 586 | 18.. 905 | 2774 |
| BRTHA2011500 | 587 | 123.. 476 | 2775 |
| BRTHA2011641 | 588 | 47.. 562 | 2776 |
| BRTHA2012392 | 589 | 1425..2021 | 2777 |
| BRTHA2012562 | 590 | 1649..1963 | 2778 |
| BRTHA2012980 | 591 | 610..1290 | 2779 |
| BRTHA2013262 | 592 | 138.. 794 | 2780 |
| BRTHA2013460 | 593 | 379.. 732 | 2781 |
| BRTHA2013707 | 594 | 244.. 657 | 2782 |
| BRTHA2014792 | 595 | 221..1663 | 2783 |
| BRTHA2014828 | 596 | 667..1158 | 2784 |
| BRTHA2015406 | 597 | 162..1583 | 2785 |
| BRTHA2015478 | 598 | 111.. 419 | 2786 |
| BRTHA2015696 | 599 | 1109..1657 | 2787 |
| BRTHA2015878 | 600 | 433..1902 | 2788 |
| BRTHA2016215 | 601 | 1831..2280 | 2789 |
| BRTHA2016496 | 602 | 76..1527 | 2790 |
| BRTHA2016543 | 603 | 310.. 636 | 2791 |
| BRTHA2017353 | 604 | 68..1027 | 2792 |
| BRTHA2017985 | 605 | 166.. 474 | 2793 |
| BRTHA2018165 | 606 | 1224..1550 | 2794 |
| BRTHA2018344 | 607 | 1064..1402 | 2795 |
| BRTHA2018591 | 608 | 515..1195 | 2796 |
| BRTHA2018624 | 609 | 1253..2293 | 2797 |
| BRTHA2018707 | 610 | 1333..1791 | 2798 |
| BRTHA2019014 | 611 | 134.. 451 | 2799 |
| BRTHA2019022 | 612 | 189.. 494 | 2800 |
| BRTHA2019048 | 613 | 52.. 549 | 2801 |
| BRTHA3000273 | 614 | 128.. 694 | 2802 |
| BRTHA3000297 | 615 | 1553..2083 | 2803 |
| BRTHA3000633 | 616 | 40.. 858 | 2804 |
| BRTHA3001721 | 617 | 382.. 963 | 2805 |
| BRTHA3002401 | 618 | 944..1396 | 2806 |
| BRTHA3002427 | 619 | 871..2589 | 2807 |
| BRTHA3002933 | 620 | 835..1908 | 2808 |
| BRTHA3003074 | 621 | 545..2050 | 2809 |
| BRTHA3003343 | 622 | 1938..2639 | 2810 |
| BRTHA3003449 | 623 | 1281..2612 | 2811 |
| BRTHA3003474 | 624 | 6.. 410 | 2812 |
| BRTHA3003490 | 625 | 22..3783 | 2813 |
| BRTHA3004475 | 626 | 1777..2196 | 2814 |
| BRTHA3005046 | 627 | 14.. 415 | 2815 |
| BRTHA3006856 | 628 | 241.. 624 | 2816 |
| BRTHA3007113 | 629 | 656..1657 | 2817 |
| BRTHA3007148 | 630 | 70.. 960 | 2818 |
| BRTHA3007319 | 631 | 1348..1995 | 2819 |
| BRTHA3007769 | 632 | 17..4579 | 2820 |
| BRTHA3008143 | 633 | 1649..1987 | 2821 |
| BRTHA3008310 | 634 | 1333..2673 | 2822 |
| BRTHA3008386 | 635 | 599..1921 | 2823 |
| BRTHA3008520 | 636 | 1670..2659 | 2824 |
| BRTHA3008778 | 637 | 1533..3353 | 2825 |
| BRTHA3009037 | 638 | 175..3441 | 2826 |
| BRTHA3009090 | 639 | 188..4141 | 2827 |
| BRTHA3009291 | 640 | 174.. 782 | 2828 |
| BRTHA3010366 | 641 | 1998..3029 | 2829 |
| BRTHA3013884 | 642 | 1085..3331 | 2830 |
| BRTHA3015815 | 643 | 1172..2122 | 2831 |
| BRTHA3015910 | 644 | 944..2899 | 2832 |
| BRTHA3016845 | 645 | 403.. 771 | 2833 |
| BRTHA3016917 | 646 | 1674..3179 | 2834 |
| BRTHA3017047 | 647 | 146..1060 | 2835 |
| BRTHA3017589 | 648 | 14.. 793 | 2836 |
| BRTHA3017848 | 649 | 218..1342 | 2837 |
| BRTHA3018514 | 650 | 416..1567 | 2838 |
| BRTHA3018617 | 651 | 28.. 522 | 2839 |
| BRTHA3018656 | 652 | 247..1338 | 2840 |
| BRTHA3019105 | 653 | 266.. 682 | 2841 |
| CERVX1000042 | 654 | 3.. 536 | 2842 |
| CERVX2002006 | 655 | 874..1257 | 2843 |
| COLON1000030 | 656 | 607..1170 | 2844 |
| COLON2000470 | 657 | 9.. 656 | 2845 |
| COLON2000568 | 658 | 73..1506 | 2846 |
| COLON2001721 | 659 | 1274..1981 | 2847 |
| COLON2002443 | 660 | 13.. 447 | 2848 |
| COLON2002520 | 661 | 1243..>3130 | 2849 |
| COLON2003043 | 662 | 3.. 311 | 2850 |
| COLON2004478 | 663 | 81..1565 | 2851 |
| COLON2005126 | 664 | 244.. 747 | 2852 |
| COLON2005772 | 665 | 286.. 816 | 2853 |
| COLON2006282 | 666 | 200.. 505 | 2854 |
| COLON2009499 | 667 | 13.. 345 | 2855 |
| CORDB1000140 | 668 | 346.. 897 | 2856 |
| CORDB2000061 | 669 | 12.. 329 | 2857 |
| CORDB2000541 | 670 | 495..1277 | 2858 |
| CTONG1000087 | 671 | 194..2326 | 2859 |
| CTONG1000088 | 672 | 2902..3252 | 2860 |
| CTONG1000288 | 673 | 1315.. 1875 | 2861 |
| CTONG1000302 | 674 | 168.. 485 | 2862 |
| CTONG1000341 | 675 | 29..1702 | 2863 |
| CTONG1000467 | 676 | 90..2312 | 2864 |
| CTONG1000488 | 677 | 2227..2892 | 2865 |
| CTONG1000508 | 678 | 1182..2615 | 2866 |
| CTONG1000540 | 679 | 778..1428 | 2867 |
| CTONG2000042 | 680 | 75..2603 | 2868 |
| CTONG2001877 | 681 | 11.. 721 | 2869 |
| CTONG2004062 | 682 | 121..2268 | 2870 |
| CTONG2006798 | 683 | 423..>2466 | 2871 |
| CTONG2008233 | 684 | 655..1893 | 2872 |
| CTONG2009423 | 685 | 1433..1774 | 2873 |
| CTONG2009531 | 686 | 49.. 543 | 2874 |
| CTONG2010803 | 687 | 1352..2464 | 2875 |
| CTONG2013178 | 688 | 50..1366 | 2876 |
| CTONG2017500 | 689 | 57.. 878 | 2877 |
| CTONG2019248 | 690 | 3528..3938 | 2878 |
| CTONG2019652 | 691 | 229.. 540 | 2879 |
| CTONG2019704 | 692 | 176.. 799 | 2880 |
| CTONG2019788 | 693 | 1985..2311 | 2881 |
| CTONG2019833 | 694 | 1026.. 1328 | 2882 |
| CTONG2020026 | 695 | 152..2845 | 2883 |
| CTONG2020127 | 696 | 1343..1774 | 2884 |
| CTONG2020522 | 697 | 191.. 586 | 2885 |
| CTONG2020638 | 698 | 159.. 986 | 2886 |
| CTONG2020806 | 699 | 436.. 933 | 2887 |
| CTONG2021132 | 700 | 1883..2233 | 2888 |
| CTONG2022153 | 701 | 341.. 787 | 2889 |
| CTONG2022601 | 702 | 1301..1621 | 2890 |
| CTONG2023021 | 703 | 2277..2840 | 2891 |
| CTONG2023512 | 704 | 311..1624 | 2892 |
| CTONG2024206 | 705 | 322..2499 | 2893 |
| CTONG2024749 | 706 | 129..2198 | 2894 |
| CTONG2025496 | 707 | 151..3015 | 2895 |
| CTONG2025516 | 708 | 459.. 773 | 2896 |
| CTONG2025900 | 709 | 135..2399 | 2897 |
| CTONG2026920 | 710 | 363.. 689 | 2898 |
| CTONG2027327 | 711 | 396.. 719 | 2899 |
| CTONG2028124 | 712 | 761..2683 | 2900 |
| CTONG2028687 | 713 | 654..1967 | 2901 |
| CTONG3000084 | 714 | 1..3111 | 2902 |
| CTONG3000657 | 715 | 31.. 732 | 2903 |
| CTONG3000686 | 716 | 2605..3294 | 2904 |
| CTONG3000707 | 717 | 2912..3265 | 2905 |
| CTONG3000896 | 718 | 3245..3676 | 2906 |
| CTONG3001123 | 719 | 285..3392 | 2907 |
| CTONG3001370 | 720 | 26..3922 | 2908 |
| CTONG3001420 | 721 | 536.. 850 | 2909 |
| CTONG3001560 | 722 | 19.. 450 | 2910 |
| CTONG3002020 | 723 | 242.. 673 | 2911 |
| CTONG3002127 | 724 | 1264..2604 | 2912 |
| CTONG3002412 | 725 | 1782..>3812 | 2913 |
| CTONG3002674 | 726 | 258..>4088 | 2914 |
| CTONG3003179 | 727 | 2650..3144 | 2915 |
| CTONG3003483 | 728 | 3006..3332 | 2916 |
| CTONG3003652 | 729 | 229..3372 | 2917 |
| CTONG3003654 | 730 | 1865..2263 | 2918 |
| CTONG3003737 | 731 | 87..1832 | 2919 |
| CTONG3003905 | 732 | 75..3833 | 2920 |
| CTONG3003972 | 733 | 203..2167 | 2921 |
| CTONG3004072 | 734 | 110..3784 | 2922 |
| CTONG3004712 | 735 | 3159..3890 | 2923 |
| CTONG3005325 | 736 | 210..3044 | 2924 |
| CTONG3005648 | 737 | 180..1061 | 2925 |
| CTONG3005713 | 738 | 42.. 902 | 2926 |
| CTONG3005813 | 739 | 173.. 991 | 2927 |
| CTONG3006067 | 740 | 194.. 583 | 2928 |
| CTONG3006186 | 741 | 208..1869 | 2929 |
| CTONG3006650 | 742 | 299.. 649 | 2930 |
| CTONG3007444 | 743 | 1638..2039 | 2931 |
| CTONG3007528 | 744 | 177.. 962 | 2932 |
| CTONG3007586 | 745 | 27..3089 | 2933 |
| CTONG3007870 | 746 | 1402..2064 | 2934 |
| CTONG3008252 | 747 | 216.. 572 | 2935 |
| CTONG3008258 | 748 | 194..2320 | 2936 |
| CTONG3008496 | 749 | 1412..1774 | 2937 |
| CTONG3008566 | 750 | 2577..3113 | 2938 |
| CTONG3008639 | 751 | 102..>4092 | 2939 |
| CTONG3008831 | 752 | 198..3077 | 2940 |
| CTONG3008894 | 753 | 1230..2411 | 2941 |
| CTONG3008951 | 754 | 3010..3438 | 2942 |
| CTONG3009028 | 755 | 25..3954 | 2943 |
| CTONG3009227 | 756 | 1948..2337 | 2944 |
| CTONG3009239 | 757 | 2907..3275 | 2945 |
| CTONG3009328 | 758 | 101..1291 | 2946 |
| CTONG3009385 | 759 | 795..2390 | 2947 |
| D3OST2002182 | 760 | 79..1308 | 2948 |
| D3OST2002648 | 761 | 215..1225 | 2949 |
| D3OST3000169 | 762 | 83..1204 | 2950 |
| DFNES1000107 | 763 | 651.. 986 | 2951 |
| DFNES2000146 | 764 | 56.. 520 | 2952 |
| DFNES2001108 | 765 | 435.. 854 | 2953 |
| DFNES2005266 | 766 | 285.. 926 | 2954 |
| DFNES2010502 | 767 | 108.. 479 | 2955 |
| DFNES2011239 | 768 | 80..1081 | 2956 |
| DFNES2011499 | 769 | 206.. 595 | 2957 |
| ERLTF2000324 | 770 | 66.. 641 | 2958 |
| FCBBF1000297 | 771 | 1409..>2811 | 2959 |
| FCBBF2001183 | 772 | 951..1559 | 2960 |
| FCBBF2007510 | 773 | 474..1082 | 2961 |
| FCBBF3001977 | 774 | 1167..1535 | 2962 |
| FCBBF3002163 | 775 | 86..2905 | 2963 |
| FCBBF3003435 | 776 | 618.. 926 | 2964 |
| FCBBF3004502 | 777 | 145..2565 | 2965 |
| FCBBF3004847 | 778 | 378.. 830 | 2966 |
| FCBBF3006171 | 779 | 599.. 913 | 2967 |
| FCBBF3007242 | 780 | 93.. 416 | 2968 |
| FCBBF3007540 | 781 | 307..1269 | 2969 |
| FCBBF3008944 | 782 | 772..1377 | 2970 |
| FCBBF3009888 | 783 | 103.. 597 | 2971 |
| FCBBF3012170 | 784 | 89..1054 | 2972 |
| FCBBF3012288 | 785 | 1881..2906 | 2973 |
| FCBBF3013307 | 786 | 300..>2766 | 2974 |
| FCBBF3013846 | 787 | 2050..2673 | 2975 |
| FCBBF3021576 | 788 | 304.. 678 | 2976 |
| FCBBF3021940 | 789 | 353.. 772 | 2977 |
| FCBBF3023443 | 790 | 1109..1495 | 2978 |
| FCBBF3023895 | 791 | 702..1409 | 2979 |
| FCBBF3025730 | 792 | 333.. 941 | 2980 |
| FCBBF3027717 | 793 | 218..>4477 | 2981 |
| FCBBF4000076 | 794 | 225.. 566 | 2982 |
| FEBRA1000030 | 795 | 735..1358 | 2983 |
| FEBRA2000253 | 796 | 1692..2822 | 2984 |
| FEBRA2006396 | 797 | 442..1521 | 2985 |
| FEBRA2007544 | 798 | 321..1874 | 2986 |
| FEBRA2007708 | 799 | 356..1981 | 2987 |
| FEBRA2007793 | 800 | 407.. 973 | 2988 |
| FEBRA2007801 | 801 | 362..2185 | 2989 |
| FEBRA2008287 | 802 | 1110..2531 | 2990 |
| FEBRA2008311 | 803 | 80..1339 | 2991 |
| FEBRA2008360 | 804 | 428.. 997 | 2992 |
| FEBRA2008468 | 805 | 291..1496 | 2993 |
| FEBRA2010719 | 806 | 348.. 995 | 2994 |
| FEBRA2014213 | 807 | 407..1483 | 2995 |
| FEBRA2015588 | 808 | 1131..1916 | 2996 |
| FEBRA2020484 | 809 | 1160..1615 | 2997 |
| FEBRA2020582 | 810 | 289.. 729 | 2998 |
| FEBRA2020668 | 811 | 787..1839 | 2999 |
| FEBRA2020886 | 812 | 1749..2342 | 3000 |
| FEBRA2021339 | 813 | 441.. 914 | 3001 |
| FEBRA2021571 | 814 | 566..1591 | 3002 |
| FEBRA2021908 | 815 | 181.. 588 | 3003 |
| FEBRA2021966 | 816 | 102.. 476 | 3004 |
| FEBRA2024136 | 817 | 2261..2638 | 3005 |
| FEBRA2024150 | 818 | 15.. 950 | 3006 |
| FEBRA2024343 | 819 | 2284..3036 | 3007 |
| FEBRA2024744 | 820 | 918..1781 | 3008 |
| FEBRA2025427 | 821 | 201.. 548 | 3009 |
| FEBRA2026984 | 822 | 910..2496 | 3010 |
| FEBRA2027082 | 823 | 174.. 509 | 3011 |
| FEBRA2027297 | 824 | 1040..1396 | 3012 |
| FEBRA2027352 | 825 | 604..1065 | 3013 |
| FEBRA2028366 | 826 | 5.. 994 | 3014 |
| FEBRA2028477 | 827 | 2176..2685 | 3015 |
| FEBRA2028618 | 828 | 587..1054 | 3016 |
| HCASM2001301 | 829 | 628..1050 | 3017 |
| HCASM2002502 | 830 | 112.. 942 | 3018 |
| HCASM2002918 | 831 | 292..>1851 | 3019 |
| HCASM2003212 | 832 | 304.. 6 | 3020 |
| HCASM2003415 | 833 | 23.. 3 | 3021 |
| HCASM2007047 | 834 | 79..2250 | 3022 |
| HCASM2007737 | 835 | 54.. 548 | 3023 |
| HCHON2000028 | 836 | 32..2239 | 3024 |
| HCHON2000212 | 837 | 1713..2087 | 3025 |
| HCHON2000244 | 838 | 698..1528 | 3026 |
| HCHON2000418 | 839 | 1185..1487 | 3027 |
| HCHON2000626 | 840 | 1168..1578 | 3028 |
| HCHON2001084 | 841 | 164..2017 | 3029 |
| HCHON2001217 | 842 | 169..2856 | 3030 |
| HCHON2001548 | 843 | 1460..1942 | 3031 |
| HCHON2001577 | 844 | 14..1726 | 3032 |
| HCHON2001712 | 845 | 143..1624 | 3033 |
| HCHON2002676 | 846 | 42..2069 | 3034 |
| HCHON2003532 | 847 | 614..1618 | 3035 |
| HCHON2004007 | 848 | 181..1917 | 3036 |
| HCHON2004531 | 849 | 299..1528 | 3037 |
| HCHON2004776 | 850 | 158..1723 | 3038 |
| HCHON2005921 | 851 | 174.. 860 | 3039 |
| HCHON2006250 | 852 | 517..2151 | 3040 |
| HCHON2006714 | 853 | 51.. 371 | 3041 |
| HCHON2007881 | 854 | 77..1801 | 3042 |
| HCHON2008112 | 855 | 929..1462 | 3043 |
| HCHON2008444 | 856 | 1810..2112 | 3044 |
| HEART1000010 | 857 | 967..1296 | 3045 |
| HEART1000074 | 858 | 151..1626 | 3046 |
| HEART1000088 | 859 | 1567..2016 | 3047 |
| HEART1000139 | 860 | 1408..2106 | 3048 |
| HEART2001680 | 861 | 59..1549 | 3049 |
| HEART2001756 | 862 | 155..1795 | 3050 |
| HEART2006131 | 863 | 8..1717 | 3051 |
| HEART2006909 | 864 | 4.. 927 | 3052 |
| HEART2007031 | 865 | 106..1374 | 3053 |
| HEART2010391 | 866 | 619..1173 | 3054 |
| HEART2010492 | 867 | 91..1617 | 3055 |
| HEART2010495 | 868 | 148..>2295 | 3056 |
| HHDPC1000118 | 869 | 258..2489 | 3057 |
| HHDPC2001337 | 870 | 210.. 566 | 3058 |
| HLUNG1000017 | 871 | 227..1054 | 3059 |
| HLUNG2000014 | 872 | 175.. 816 | 3060 |
| HLUNG2001996 | 873 | 990..1562 | 3061 |
| HLUNG2002465 | 874 | 72..1796 | 3062 |
| HLUNG2002958 | 875 | 123..2234 | 3063 |
| HLUNG2003003 | 876 | 459.. 848 | 3064 |
| HLUNG2003872 | 877 | 542..1366 | 3065 |
| HLUNG2010464 | 878 | 256.. 972 | 3066 |
| HLUNG2011041 | 879 | 721..1422 | 3067 |
| HLUNG2011298 | 880 | 1669..1983 | 3068 |
| HLUNG2012049 | 881 | 264.. 755 | 3069 |
| HLUNG2012287 | 882 | 789..1139 | 3070 |
| HLUNG2012727 | 883 | 198..1046 | 3071 |
| HLUNG2013204 | 884 | 1196..1513 | 3072 |
| HLUNG2013304 | 885 | 329.. 649 | 3073 |
| HLUNG2013622 | 886 | 123.. 440 | 3074 |
| HLUNG2013851 | 887 | 224.. 787 | 3075 |
| HLUNG2014262 | 888 | 189..1886 | 3076 |
| HLUNG2014288 | 889 | 192..1106 | 3077 |
| HLUNG2014449 | 890 | 2235..>2713 | 3078 |
| HLUNG2015617 | 891 | 1630..1968 | 3079 |
| HLUNG2017350 | 892 | 879..1766 | 3080 |
| HLUNG2017546 | 893 | 1666..2070 | 3081 |
| HLUNG2017806 | 894 | 29.. 577 | 3082 |
| HLUNG2019058 | 895 | 239.. 568 | 3083 |
| HSYRA2004858 | 896 | 150.. 467 | 3084 |
| HSYRA2005456 | 897 | 61..2415 | 3085 |
| HSYRA2005496 | 898 | 36..1211 | 3086 |
| HSYRA2006873 | 899 | 590.. 910 | 3087 |
| HSYRA2007667 | 900 | 424..1281 | 3088 |
| HSYRA2008376 | 901 | 329..2422 | 3089 |
| HSYRA2008714 | 902 | 249..1487 | 3090 |
| HSYRA2009075 | 903 | 237..2204 | 3091 |
| HSYRA2009102 | 904 | 119..1357 | 3092 |
| IMR322000127 | 905 | 847..2271 | 3093 |
| IMR322000917 | 906 | 287..1000 | 3094 |
| IMR322001380 | 907 | 14.. 790 | 3095 |
| IMR322002035 | 908 | 2228..2620 | 3096 |
| IMR322002110 | 909 | 176.. 553 | 3097 |
| IMR322003675 | 910 | 202.. 978 | 3098 |
| IMR322006222 | 911 | 516.. 845 | 3099 |
| IMR322006495 | 912 | 63.. 749 | 3100 |
| IMR322006886 | 913 | 565..1224 | 3101 |
| IMR322007225 | 914 | 24.. 467 | 3102 |
| IMR322016146 | 915 | 112.. 426 | 3103 |
| IMR322018117 | 916 | 37.. 528 | 3104 |
| KIDNE1000064 | 917 | 302..1963 | 3105 |
| KIDNE2000665 | 918 | 379.. 735 | 3106 |
| KIDNE2000722 | 919 | 227.. 571 | 3107 |
| KIDNE2000832 | 920 | 103..1554 | 3108 |
| KIDNE2000846 | 921 | 23.. 700 | 3109 |
| KIDNE2001361 | 922 | 953..2056 | 3110 |
| KIDNE2001847 | 923 | 86..1240 | 3111 |
| KIDNE2002252 | 924 | 96..2543 | 3112 |
| KIDNE2002991 | 925 | 644.. 952 | 3113 |
| KIDNE2003837 | 926 | 1732..2073 | 3114 |
| KIDNE2005543 | 927 | 2011..2484 | 3115 |
| KIDNE2006580 | 928 | 40..1617 | 3116 |
| KIDNE2010264 | 929 | 640..1158 | 3117 |
| KIDNE2011314 | 930 | 796..1098 | 3118 |
| KIDNE2011532 | 931 | 1097..1636 | 3119 |
| KIDNE2011635 | 932 | 148..2376 | 3120 |
| KIDNE2012945 | 933 | 301..1407 | 3121 |
| KIDNE2013095 | 934 | 162.. 749 | 3122 |
| LIVER2007415 | 935 | 308..1144 | 3123 |
| LYMPB1000141 | 936 | 85.. 396 | 3124 |
| LYMPB2000083 | 937 | 35..1084 | 3125 |
| MESAN2001979 | 938 | 1199..1855 | 3126 |
| MESAN2006563 | 939 | 264..1670 | 3127 |
| MESAN2012054 | 940 | 24..1892 | 3128 |
| MESAN2014295 | 941 | 1349..1687 | 3129 |
| MESAN2015515 | 942 | 381.. 803 | 3130 |
| MESAN2018576 | 943 | 65.. 370 | 3131 |
| MESTC1000042 | 944 | 235.. 738 | 3132 |
| MESTC2000153 | 945 | 350.. 820 | 3133 |
| NB9N41000340 | 946 | 625..1032 | 3134 |
| NCRRP1000129 | 947 | 147.. 521 | 3135 |
| NESOP2000744 | 948 | 652..1020 | 3136 |
| NESOP2001433 | 949 | 81..1580 | 3137 |
| NESOP2001656 | 950 | 369.. 707 | 3138 |
| NESOP2001694 | 951 | 240.. 731 | 3139 |
| NESOP2001752 | 952 | 624.. 935 | 3140 |
| NESOP2002738 | 953 | 1342..1890 | 3141 |
| NHNPC2000606 | 954 | 1635..1949 | 3142 |
| NHNPC2000877 | 955 | 27.. 443 | 3143 |
| NHNPC2001223 | 956 | 2476..2790 | 3144 |
| NHNPC2001816 | 957 | 1053..1421 | 3145 |
| NHNPC2002565 | 958 | 463..1134 | 3146 |
| NHNPC2002749 | 959 | 81.. 605 | 3147 |
| NOVAR2000136 | 960 | 174..1481 | 3148 |
| NOVAR2000710 | 961 | 59.. 466 | 3149 |
| NOVAR2000962 | 962 | 25.. 393 | 3150 |
| NOVAR2001108 | 963 | 49..1482 | 3151 |
| NOVAR2001783 | 964 | 44.. 490 | 3152 |
| NT2NE2003252 | 965 | 889..1899 | 3153 |
| NT2NE2005890 | 966 | 435.. 740 | 3154 |
| NT2NE2006531 | 967 | 295..1812 | 3155 |
| NT2NE2006909 | 968 | 661..1647 | 3156 |
| NT2NE2008060 | 969 | 1027..1404 | 3157 |
| NT2RI2003993 | 970 | 600..1136 | 3158 |
| NT2RI2004618 | 971 | 495..1253 | 3159 |
| NT2RI2005166 | 972 | 60..1436 | 3160 |
| NT2RI2006686 | 973 | 14..1909 | 3161 |
| NT2RI2008724 | 974 | 289..1917 | 3162 |
| NT2RI2009855 | 975 | 1615..2283 | 3163 |
| NT2RI2011422 | 976 | 110..1543 | 3164 |
| NT2RI2011683 | 977 | 206.. 754 | 3165 |
| NT2RI2012659 | 978 | 100.. 528 | 3166 |
| NT2RI2012990 | 979 | 85.. 561 | 3167 |
| NT2RI2013357 | 980 | 287.. 877 | 3168 |
| NT2RI2014247 | 981 | 1608..1967 | 3169 |
| NT2RI2014551 | 982 | 52..>2076 | 3170 |
| NT2RI2014733 | 983 | 638..1204 | 3171 |
| NT2RI2016128 | 984 | 173.. 589 | 3172 |
| NT2RI2018311 | 985 | 680..1345 | 3173 |
| NT2RI2018883 | 986 | 316.. 846 | 3174 |
| NT2RI2019751 | 987 | 357.. 743 | 3175 |
| NT2RI2023303 | 988 | 1041..1451 | 3176 |
| NT2RI2025909 | 989 | 208..1119 | 3177 |
| NT2RI2025957 | 990 | 348..2432 | 3178 |
| NT2RI2027081 | 991 | 1254..1586 | 3179 |
| NT2RI2027396 | 992 | 109.. 426 | 3180 |
| NT2RI3000622 | 993 | 305.. 964 | 3181 |
| NT2RI3001263 | 994 | 2825..3241 | 3182 |
| NT2RI3001515 | 995 | 857..3373 | 3183 |
| NT2RI3002303 | 996 | 2269..2613 | 3184 |
| NT2RI3002842 | 997 | 50.. 934 | 3185 |
| NT2RI3002892 | 998 | 3.. 311 | 3186 |
| NT2RI3003031 | 999 | 111.. 413 | 3187 |
| NT2RI3003095 | 1000 | 1032..1412 | 3188 |
| NT2RI3003162 | 1001 | 865..1353 | 3189 |
| NT2RI3003382 | 1002 | 600..2642 | 3190 |
| NT2RI3003409 | 1003 | 2744..3340 | 3191 |
| NT2RI3004381 | 1004 | 2637..3314 | 3192 |
| NT2RI3004510 | 1005 | 178..1950 | 3193 |
| NT2RI3005202 | 1006 | 132.. 659 | 3194 |
| NT2RI3005403 | 1007 | 366..1073 | 3195 |
| NT2RI3005724 | 1008 | 194.. 637 | 3196 |
| NT2RI3006132 | 1009 | 82.. 684 | 3197 |
| NT2RI3006171 | 1010 | 300..1211 | 3198 |
| NT2RI3006284 | 1011 | 154..1911 | 3199 |
| NT2RI3006340 | 1012 | 335..5392 | 3200 |
| NT2RI3006376 | 1013 | 152..4081 | 3201 |
| NT2RI3006673 | 1014 | 197..3997 | 3202 |
| NT2RI3006796 | 1015 | 1047..1427 | 3203 |
| NT2RI3007065 | 1016 | 1707..2096 | 3204 |
| NT2RI3007158 | 1017 | 656..>4499 | 3205 |
| NT2RI3007291 | 1018 | 10..1614 | 3206 |
| NT2RI3007543 | 1019 | 54..4181 | 3207 |
| NT2RI3007757 | 1020 | 225..>5460 | 3208 |
| NT2RI3007978 | 1021 | 745..2505 | 3209 |
| NT2RI3008055 | 1022 | 2452..2838 | 3210 |
| NT2RI3008162 | 1023 | 81..2354 | 3211 |
| NT2RI3008652 | 1024 | 179..2323 | 3212 |
| NT2RI3008697 | 1025 | 334..1515 | 3213 |
| NT2RI3008974 | 1026 | 949..1434 | 3214 |
| NT2RI3009158 | 1027 | 228..1286 | 3215 |
| NT2RP7000359 | 1028 | 48..3902 | 3216 |
| NT2RP7000466 | 1029 | 72..2987 | 3217 |
| NT2RP7004027 | 1030 | 507..1787 | 3218 |
| NT2RP7004123 | 1031 | 638..1516 | 3219 |
| NT2RP7005118 | 1032 | 108..3179 | 3220 |
| NT2RP7005529 | 1033 | 156..2531 | 3221 |
| NT2RP7005846 | 1034 | 1536..2180 | 3222 |
| NT2RP7009030 | 1035 | 732..1199 | 3223 |
| NT2RP7009147 | 1036 | 434..>2686 | 3224 |
| NT2RP7009867 | 1037 | 880..1317 | 3225 |
| NT2RP7010128 | 1038 | 264.. 755 | 3226 |
| NT2RP7010599 | 1039 | 988..2160 | 3227 |
| NT2RP7011570 | 1040 | 1694..2017 | 3228 |
| NT2RP7013795 | 1041 | 284..1117 | 3229 |
| NT2RP7014005 | 1042 | 375..2135 | 3230 |
| NT2RP7015512 | 1043 | 2441..2863 | 3231 |
| NT2RP7017365 | 1044 | 5.. 349 | 3232 |
| NT2RP7017474 | 1045 | 234..1598 | 3233 |
| NT2RP7017546 | 1046 | 182..1018 | 3234 |
| NT2RP8000137 | 1047 | 1335..1673 | 3235 |
| NT2RP8000296 | 1048 | 911..2878 | 3236 |
| NT2RP8000483 | 1049 | 2903..4729 | 3237 |
| NTONG2000413 | 1050 | 331..1863 | 3238 |
| NTONG2003852 | 1051 | 341..1087 | 3239 |
| NTONG2005277 | 1052 | 293..2059 | 3240 |
| NTONG2005969 | 1053 | 1128..1436 | 3241 |
| NTONG2006354 | 1054 | 96.. 674 | 3242 |
| NTONG2007249 | 1055 | 439..1386 | 3243 |
| NTONG2007517 | 1056 | 34.. 930 | 3244 |
| NTONG2008088 | 1057 | 133.. 468 | 3245 |
| NTONG2008672 | 1058 | 37..2067 | 3246 |
| OCBBF1000254 | 1059 | 203.. 514 | 3247 |
| OCBBF2001794 | 1060 | 23.. 454 | 3248 |
| OCBBF2002124 | 1061 | 2496..2807 | 3249 |
| OCBBF2003819 | 1062 | 1788..>2890 | 3250 |
| OCBBF2004826 | 1063 | 304..>4404 | 3251 |
| OCBBF2004883 | 1064 | 1087..1803 | 3252 |
| OCBBF2005428 | 1065 | 1010..1369 | 3253 |
| OCBBF2006005 | 1066 | 107..2752 | 3254 |
| OCBBF2006058 | 1067 | 143.. 568 | 3255 |
| OCBBF2006151 | 1068 | 53.. 817 | 3256 |
| OCBBF2006567 | 1069 | 2568..2924 | 3257 |
| OCBBF2006764 | 1070 | 157..2571 | 3258 |
| OCBBF2007028 | 1071 | 1589..2572 | 3259 |
| OCBBF2007068 | 1072 | 108..3338 | 3260 |
| OCBBF2007114 | 1073 | 589.. 963 | 3261 |
| OCBBF2007428 | 1074 | 177.. 527 | 3262 |
| OCBBF2007478 | 1075 | 2014..2361 | 3263 |
| OCBBF2007610 | 1076 | 1229..1963 | 3264 |
| OCBBF2008770 | 1077 | 224..2677 | 3265 |
| OCBBF2009788 | 1078 | 18.. 371 | 3266 |
| OCBBF2009926 | 1079 | 734..1177 | 3267 |
| OCBBF2010140 | 1080 | 66..2459 | 3268 |
| OCBBF2010416 | 1081 | 1243..>3531 | 3269 |
| OCBBF2017516 | 1082 | 459.. 860 | 3270 |
| OCBBF2019327 | 1083 | 221.. 553 | 3271 |
| OCBBF2019823 | 1084 | 798..1796 | 3272 |
| OCBBF2020343 | 1085 | 293.. 859 | 3273 |
| OCBBF2020453 | 1086 | 168.. 488 | 3274 |
| OCBBF2020639 | 1087 | 907..1248 | 3275 |
| OCBBF2020741 | 1088 | 962..1555 | 3276 |
| OCBBF2020801 | 1089 | 1544..2017 | 3277 |
| OCBBF2020838 | 1090 | 593..1819 | 3278 |
| OCBBF2021020 | 1091 | 1232..2206 | 3279 |
| OCBBF2021286 | 1092 | 86.. 787 | 3280 |
| OCBBF2021323 | 1093 | 8.. 538 | 3281 |
| OCBBF2021788 | 1094 | 19..2793 | 3282 |
| OCBBF2022351 | 1095 | 157..1443 | 3283 |
| OCBBF2022574 | 1096 | 362.. 769 | 3284 |
| OCBBF2023162 | 1097 | 222.. 536 | 3285 |
| OCBBF2023643 | 1098 | 1236..1658 | 3286 |
| OCBBF2024719 | 1099 | 658.. 999 | 3287 |
| OCBBF2024781 | 1100 | 358.. 987 | 3288 |
| OCBBF2024850 | 1101 | 329.. 652 | 3289 |
| OCBBF2025028 | 1102 | 431..1504 | 3290 |
| OCBBF2025458 | 1103 | 10.. 486 | 3291 |
| OCBBF2025527 | 1104 | 627.. 980 | 3292 |
| OCBBF2025730 | 1105 | 614.. 949 | 3293 |
| OCBBF2026645 | 1106 | 1905..2216 | 3294 |
| OCBBF2027423 | 1107 | 25.. 360 | 3295 |
| OCBBF2027478 | 1108 | 1362..1796 | 3296 |
| OCBBF2028173 | 1109 | 764..1723 | 3297 |
| OCBBF2028935 | 1110 | 76.. 432 | 3298 |
| OCBBF2029901 | 1111 | 357.. 836 | 3299 |
| OCBBF2030354 | 1112 | 261..1646 | 3300 |
| OCBBF2030517 | 1113 | 1378..1842 | 3301 |
| OCBBF2030574 | 1114 | 54.. 605 | 3302 |
| OCBBF2030708 | 1115 | 409..2361 | 3303 |
| OCBBF2031167 | 1116 | 31..>2709 | 3304 |
| OCBBF2031366 | 1117 | 1696..2055 | 3305 |
| OCBBF2032590 | 1118 | 395..1021 | 3306 |
| OCBBF2032599 | 1119 | 30.. 371 | 3307 |
| OCBBF2032611 | 1120 | 1.. 354 | 3308 |
| OCBBF2032671 | 1121 | 960..1355 | 3309 |
| OCBBF2033869 | 1122 | 882..1355 | 3310 |
| OCBBF2035110 | 1123 | 1534..3426 | 3311 |
| OCBBF2035214 | 1124 | 342.. 644 | 3312 |
| OCBBF2035564 | 1125 | 321..2228 | 3313 |
| OCBBF2035885 | 1126 | 76.. 420 | 3314 |
| OCBBF2035916 | 1127 | 1859..2524 | 3315 |
| OCBBF2036476 | 1128 | 1103..1873 | 3316 |
| OCBBF2036743 | 1129 | 526..2445 | 3317 |
| OCBBF2037068 | 1130 | 318..1067 | 3318 |
| OCBBF2037340 | 1131 | 526..>3404 | 3319 |
| OCBBF2037398 | 1132 | 286.. 651 | 3320 |
| OCBBF2037547 | 1133 | 54..3860 | 3321 |
| OCBBF2037598 | 1134 | 1515..>3057 | 3322 |
| OCBBF2037638 | 1135 | 754..2043 | 3323 |
| OCBBF2038317 | 1136 | 183..3575 | 3324 |
| OCBBF3000296 | 1137 | 1505..2095 | 3325 |
| OCBBF3000483 | 1138 | 37.. 471 | 3326 |
| OCBBF3002553 | 1139 | 27.. 581 | 3327 |
| OCBBF3002600 | 1140 | 2661..3005 | 3328 |
| OCBBF3003320 | 1141 | 20..1000 | 3329 |
| OCBBF3003592 | 1142 | 86..2827 | 3330 |
| OCBBF3004314 | 1143 | 687..1187 | 3331 |
| OCBBF3006802 | 1144 | 236.. 625 | 3332 |
| OCBBF3007516 | 1145 | 1479..1829 | 3333 |
| OCBBF3008230 | 1146 | 2923..>3428 | 3334 |
| OCBBF3009279 | 1147 | 34..1065 | 3335 |
| PEBLM2000170 | 1148 | 434.. 811 | 3336 |
| PEBLM2000338 | 1149 | 1419..1865 | 3337 |
| PEBLM2001465 | 1150 | 856..1374 | 3338 |
| PEBLM2001488 | 1151 | 325.. 732 | 3339 |
| PEBLM2002594 | 1152 | 688..2121 | 3340 |
| PEBLM2002749 | 1153 | 275.. 820 | 3341 |
| PEBLM2002887 | 1154 | 948..1301 | 3342 |
| PEBLM2004497 | 1155 | 502.. 888 | 3343 |
| PEBLM2004666 | 1156 | 139..2397 | 3344 |
| PEBLM2005183 | 1157 | 9..2426 | 3345 |
| PEBLM2005697 | 1158 | 408.. 734 | 3346 |
| PEBLM2006113 | 1159 | 29.. 421 | 3347 |
| PEBLM2007112 | 1160 | 243.. 704 | 3348 |
| PEBLM2007140 | 1161 | 267.. 629 | 3349 |
| PEBLM2007834 | 1162 | 177.. 539 | 3350 |
| PERIC1000147 | 1163 | 83..>2669 | 3351 |
| PERIC2000889 | 1164 | 1483..2079 | 3352 |
| PERIC2000914 | 1165 | 1557..1901 | 3353 |
| PERIC2001227 | 1166 | 936..1253 | 3354 |
| PERIC2001228 | 1167 | 2.. 754 | 3355 |
| PERIC2002766 | 1168 | 64.. 384 | 3356 |
| PERIC2003090 | 1169 | 158.. 802 | 3357 |
| PERIC2003452 | 1170 | 1555..1890 | 3358 |
| PERIC2003699 | 1171 | 100.. 435 | 3359 |
| PERIC2003720 | 1172 | 640..1512 | 3360 |
| PERIC2003834 | 1173 | 103.. 531 | 3361 |
| PERIC2004028 | 1174 | 751..1212 | 3362 |
| PERIC2004259 | 1175 | 1686..2042 | 3363 |
| PERIC2004379 | 1176 | 55.. 483 | 3364 |
| PERIC2004429 | 1177 | 12.. 377 | 3365 |
| PERIC2004909 | 1178 | 32.. 334 | 3366 |
| PERIC2005347 | 1179 | 26.. 499 | 3367 |
| PERIC2005370 | 1180 | 13..1041 | 3368 |
| PERIC2006035 | 1181 | 1..1134 | 3369 |
| PERIC2007914 | 1182 | 1492..2073 | 3370 |
| PERIC2008385 | 1183 | 348.. 722 | 3371 |
| PERIC2009086 | 1184 | 202..1749 | 3372 |
| PLACE5000001 | 1185 | 2536..3036 | 3373 |
| PLACE5000171 | 1186 | 334..2940 | 3374 |
| PLACE5000260 | 1187 | 242.. 742 | 3375 |
| PLACE5000282 | 1188 | 60..1739 | 3376 |
| PLACE6001185 | 1189 | 765..1337 | 3377 |
| PLACE6009006 | 1190 | 1018..1518 | 3378 |
| PLACE6012574 | 1191 | 368..1984 | 3379 |
| PLACE6019385 | 1192 | 617..1360 | 3380 |
| PLACE6019932 | 1193 | 20.. 847 | 3381 |
| PLACE6020031 | 1194 | 228..1586 | 3382 |
| PLACE7000514 | 1195 | 17..1246 | 3383 |
| PLACE7001022 | 1196 | 1733..2197 | 3384 |
| PLACE7001936 | 1197 | 272.. 697 | 3385 |
| PLACE7002641 | 1198 | 1254..2264 | 3386 |
| PLACE7006051 | 1199 | 145..>3409 | 3387 |
| PLACE7008431 | 1200 | 223..1281 | 3388 |
| PLACE7008623 | 1201 | 1304..1762 | 3389 |
| PROST1000184 | 1202 | 734.. 1123 | 3390 |
| PROST1000528 | 1203 | 428.. 838 | 3391 |
| PROST1000559 | 1204 | 149.. 856 | 3392 |
| PROST2003428 | 1205 | 1031..1387 | 3393 |
| PROST2008993 | 1206 | 362..2008 | 3394 |
| PROST2015243 | 1207 | 1401..1802 | 3395 |
| PROST2016462 | 1208 | 435..2081 | 3396 |
| PROST2017367 | 1209 | 72.. 428 | 3397 |
| PROST2017413 | 1210 | 1163..1510 | 3398 |
| PROST2017700 | 1211 | 1337..1645 | 3399 |
| PROST2018030 | 1212 | 884..1255 | 3400 |
| PROST2018090 | 1213 | 117..1448 | 3401 |
| PROST2018511 | 1214 | 136..1803 | 3402 |
| PROST2018902 | 1215 | 288.. 653 | 3403 |
| PROST2018922 | 1216 | 556.. 888 | 3404 |
| PROST2019296 | 1217 | 415.. 777 | 3405 |
| PROST2019781 | 1218 | 139.. 441 | 3406 |
| PUAEN2002489 | 1219 | 410..1186 | 3407 |
| PUAEN2002616 | 1220 | 1790..2389 | 3408 |
| PUAEN2003079 | 1221 | 438.. 812 | 3409 |
| PUAEN2005588 | 1222 | 134.. 580 | 3410 |
| PUAEN2005930 | 1223 | 1673..3256 | 3411 |
| PUAEN2006328 | 1224 | 166..2064 | 3412 |
| PUAEN2006701 | 1225 | 277.. 837 | 3413 |
| PUAEN2007044 | 1226 | 51.. 521 | 3414 |
| PUAEN2007785 | 1227 | 23..1144 | 3415 |
| PUAEN2009174 | 1228 | 4..2547 | 3416 |
| PUAEN2009655 | 1229 | 237..2198 | 3417 |
| PUAEN2009795 | 1230 | 121..1716 | 3418 |
| PUAEN2009852 | 1231 | 953.. 1291 | 3419 |
| RECTM2000433 | 1232 | 33.. 536 | 3420 |
| RECTM2001347 | 1233 | 638..1609 | 3421 |
| SKMUS2000757 | 1234 | 289.. 606 | 3422 |
| SKMUS2003074 | 1235 | 291.. 692 | 3423 |
| SKMUS2004047 | 1236 | 628..1047 | 3424 |
| SKMUS2006394 | 1237 | 111.. 1469 | 3425 |
| SKNMC1000124 | 1238 | 194..1390 | 3426 |
| SKNMC2002402 | 1239 | 107.. 466 | 3427 |
| SKNMC2004457 | 1240 | 1304..1750 | 3428 |
| SKNMC2004643 | 1241 | 26.. 697 | 3429 |
| SKNMC2005772 | 1242 | 1187..1585 | 3430 |
| SKNMC2006998 | 1243 | 97.. 558 | 3431 |
| SKNMC2007504 | 1244 | 27..1190 | 3432 |
| SKNMC2007961 | 1245 | 170.. 472 | 3433 |
| SKNMC2009450 | 1246 | 103.. 411 | 3434 |
| SKNSH2000482 | 1247 | 1184..1561 | 3435 |
| SKNSH2009991 | 1248 | 1073..1378 | 3436 |
| SKNSH2010015 | 1249 | 1308..1613 | 3437 |
| SMINT1000192 | 1250 | 49.. 810 | 3438 |
| SMINT2001818 | 1251 | 616..1158 | 3439 |
| SMINT2002743 | 1252 | 995..1375 | 3440 |
| SMINT2006641 | 1253 | 1820..2185 | 3441 |
| SMINT2007391 | 1254 | 229..1197 | 3442 |
| SMINT2009902 | 1255 | 98.. 901 | 3443 |
| SMINT2010076 | 1256 | 81..1574 | 3444 |
| SMINT2010897 | 1257 | 230.. 535 | 3445 |
| SMINT2011311 | 1258 | 594..1121 | 3446 |
| SMINT2011888 | 1259 | 80..1543 | 3447 |
| SMINT2015787 | 1260 | 48.. 551 | 3448 |
| SPLEN2001599 | 1261 | 20..1297 | 3449 |
| SPLEN2002147 | 1262 | 607..1068 | 3450 |
| SPLEN2002467 | 1263 | 256..1566 | 3451 |
| SPLEN2002707 | 1264 | 234.. 545 | 3452 |
| SPLEN2006122 | 1265 | 162..1121 | 3453 |
| SPLEN2009548 | 1266 | 349.. 723 | 3454 |
| SPLEN2010912 | 1267 | 1159..2286 | 3455 |
| SPLEN2011422 | 1268 | 79..1008 | 3456 |
| SPLEN2012624 | 1269 | 1077..1784 | 3457 |
| SPLEN2012889 | 1270 | 1647..2018 | 3458 |
| SPLEN2014946 | 1271 | 161.. 547 | 3459 |
| SPLEN2015158 | 1272 | 567.. 947 | 3460 |
| SPLEN2015267 | 1273 | 81..1646 | 3461 |
| SPLEN2015679 | 1274 | 517..>2171 | 3462 |
| SPLEN2016554 | 1275 | 200..3343 | 3463 |
| SPLEN2016863 | 1276 | 276..1352 | 3464 |
| SPLEN2017104 | 1277 | 264..1274 | 3465 |
| SPLEN2021701 | 1278 | 23.. 910 | 3466 |
| SPLEN2023733 | 1279 | 158..1264 | 3467 |
| SPLEN2023791 | 1280 | 144.. 746 | 3468 |
| SPLEN2024127 | 1281 | 198.. 584 | 3469 |
| SPLEN2025491 | 1282 | 1056..1466 | 3470 |
| SPLEN2027268 | 1283 | 1655..2161 | 3471 |
| SPLEN2028844 | 1284 | 271.. 840 | 3472 |
| SPLEN2028914 | 1285 | 253.. 855 | 3473 |
| SPLEN2029051 | 1286 | 234.. 596 | 3474 |
| SPLEN2029176 | 1287 | 531.. 998 | 3475 |
| SPLEN2029522 | 1288 | 1430..1771 | 3476 |
| SPLEN2029683 | 1289 | 1308..1682 | 3477 |
| SPLEN2029727 | 1290 | 2754..3068 | 3478 |
| SPLEN2029912 | 1291 | 216.. 653 | 3479 |
| SPLEN2030335 | 1292 | 382..1104 | 3480 |
| SPLEN2030479 | 1293 | 567..1061 | 3481 |
| SPLEN2031125 | 1294 | 24.. 407 | 3482 |
| SPLEN2031424 | 1295 | 641..1138 | 3483 |
| SPLEN2031547 | 1296 | 428..1645 | 3484 |
| SPLEN2031724 | 1297 | 385.. 765 | 3485 |
| SPLEN2031780 | 1298 | 78..1067 | 3486 |
| SPLEN2032154 | 1299 | 244.. 735 | 3487 |
| SPLEN2032321 | 1300 | 1893..2222 | 3488 |
| SPLEN2032813 | 1301 | 799..1134 | 3489 |
| SPLEN2033098 | 1302 | 1452..2051 | 3490 |
| SPLEN2033153 | 1303 | 109.. 471 | 3491 |
| SPLEN2033539 | 1304 | 1359..1682 | 3492 |
| SPLEN2033921 | 1305 | 2125..2478 | 3493 |
| SPLEN2034021 | 1306 | 147.. 482 | 3494 |
| SPLEN2034081 | 1307 | 536..1042 | 3495 |
| SPLEN2034678 | 1308 | 61.. 495 | 3496 |
| SPLEN2034781 | 1309 | 629..1618 | 3497 |
| SPLEN2036103 | 1310 | 1088..1627 | 3498 |
| SPLEN2036326 | 1311 | 1062..1973 | 3499 |
| SPLEN2036712 | 1312 | 179.. 529 | 3500 |
| SPLEN2036821 | 1313 | 932..1276 | 3501 |
| SPLEN2036932 | 1314 | 1724..2170 | 3502 |
| SPLEN2037194 | 1315 | 185..1984 | 3503 |
| SPLEN2037580 | 1316 | 1852..2301 | 3504 |
| SPLEN2037630 | 1317 | 1219..1542 | 3505 |
| SPLEN2037722 | 1318 | 71..1066 | 3506 |
| SPLEN2038055 | 1319 | 1738..2220 | 3507 |
| SPLEN2038180 | 1320 | 8.. 496 | 3508 |
| SPLEN2038345 | 1321 | 356.. 793 | 3509 |
| SPLEN2038407 | 1322 | 185..2005 | 3510 |
| SPLEN2039697 | 1323 | 791..1105 | 3511 |
| SPLEN2039936 | 1324 | 71.. 382 | 3512 |
| SPLEN2040222 | 1325 | 106.. 840 | 3513 |
| SPLEN2041304 | 1326 | 72.. 398 | 3514 |
| SPLEN2041310 | 1327 | 1402..1734 | 3515 |
| SPLEN2041645 | 1328 | 1624..1998 | 3516 |
| SPLEN2041720 | 1329 | 57.. 494 | 3517 |
| SPLEN2041977 | 1330 | 72.. 374 | 3518 |
| SPLEN2042303 | 1331 | 480.. 791 | 3519 |
| SPLEN2042598 | 1332 | 70.. 387 | 3520 |
| STOMA1000189 | 1333 | 1050..1436 | 3521 |
| STOMA2003444 | 1334 | 1664..1975 | 3522 |
| STOMA2004294 | 1335 | 38.. 607 | 3523 |
| STOMA2004925 | 1336 | 854..1285 | 3524 |
| STOMA2008546 | 1337 | 119..1060 | 3525 |
| SYNOV1000374 | 1338 | 204.. 659 | 3526 |
| SYNOV2005216 | 1339 | 1201..>2283 | 3527 |
| SYNOV2005448 | 1340 | 1261..1653 | 3528 |
| SYNOV2005817 | 1341 | 412..1395 | 3529 |
| SYNOV2006430 | 1342 | 232.. 780 | 3530 |
| SYNOV2007965 | 1343 | 83..1375 | 3531 |
| SYNOV2012326 | 1344 | 30.. 476 | 3532 |
| SYNOV2014400 | 1345 | 389.. 988 | 3533 |
| SYNOV2016124 | 1346 | 1174..1476 | 3534 |
| SYNOV2017055 | 1347 | 981..1511 | 3535 |
| SYNOV2018921 | 1348 | 126.. 737 | 3536 |
| SYNOV2021320 | 1349 | 182..1792 | 3537 |
| SYNOV3000231 | 1350 | 81..1499 | 3538 |
| SYNOV3000302 | 1351 | 80..1423 | 3539 |
| SYNOV4000472 | 1352 | 103.. 540 | 3540 |
| SYNOV4000706 | 1353 | 121..2538 | 3541 |
| SYNOV4001326 | 1354 | 196..1086 | 3542 |
| SYNOV4001395 | 1355 | 959..2092 | 3543 |
| SYNOV4002346 | 1356 | 150..3797 | 3544 |
| SYNOV4002392 | 1357 | 148.. 951 | 3545 |
| SYNOV4002883 | 1358 | 1686..2240 | 3546 |
| SYNOV4003322 | 1359 | 2461..2859 | 3547 |
| SYNOV4004184 | 1360 | 2542..2889 | 3548 |
| SYNOV4004741 | 1361 | 776..1237 | 3549 |
| SYNOV4004823 | 1362 | 3332..3715 | 3550 |
| SYNOV4004914 | 1363 | 2753..3106 | 3551 |
| SYNOV4006256 | 1364 | 182.. 493 | 3552 |
| SYNOV4007012 | 1365 | 546..1007 | 3553 |
| SYNOV4007215 | 1366 | 78..>3375 | 3554 |
| SYNOV4007360 | 1367 | 567..1364 | 3555 |
| SYNOV4007430 | 1368 | 47.. 448 | 3556 |
| SYNOV4007521 | 1369 | 6.. 935 | 3557 |
| SYNOV4007553 | 1370 | 1405..3759 | 3558 |
| SYNOV4007671 | 1371 | 548..1417 | 3559 |
| SYNOV4008336 | 1372 | 825..1256 | 3560 |
| SYNOV4008440 | 1373 | 115..3804 | 3561 |
| T1ESE2000116 | 1374 | 1455..1769 | 3562 |
| TBAES2001171 | 1375 | 871..1845 | 3563 |
| TBAES2001220 | 1376 | 155.. 547 | 3564 |
| TBAES2001229 | 1377 | 1496..1861 | 3565 |
| TBAES2001258 | 1378 | 369.. 677 | 3566 |
| TBAES2001492 | 1379 | 630..1241 | 3567 |
| TBAES2001751 | 1380 | 185.. 592 | 3568 |
| TBAES2002197 | 1381 | 5..1609 | 3569 |
| TBAES2003550 | 1382 | 1130..1549 | 3570 |
| TBAES2004055 | 1383 | 898..1935 | 3571 |
| TBAES2005157 | 1384 | 119..1573 | 3572 |
| TBAES2005543 | 1385 | 1197..1694 | 3573 |
| TBAES2006568 | 1386 | 169.. 996 | 3574 |
| TBAES2007964 | 1387 | 72.. 449 | 3575 |
| TCERX2000613 | 1388 | 427..1410 | 3576 |
| TCOLN2002278 | 1389 | 1064..1432 | 3577 |
| TESOP1000127 | 1390 | 358.. 708 | 3578 |
| TESOP2000801 | 1391 | 707..1216 | 3579 |
| TESOP2001122 | 1392 | 207.. 998 | 3580 |
| TESOP2001166 | 1393 | 290..1612 | 3581 |
| TESOP2001345 | 1394 | 33.. 350 | 3582 |
| TESOP2001605 | 1395 | 746..1651 | 3583 |
| TESOP2001818 | 1396 | 491..1456 | 3584 |
| TESOP2001849 | 1397 | 17.. 853 | 3585 |
| TESOP2001865 | 1398 | 6.. 368 | 3586 |
| TESOP2001953 | 1399 | 372.. 1871 | 3587 |
| TESOP2002273 | 1400 | 163.. 558 | 3588 |
| TESOP2002451 | 1401 | 99..1157 | 3589 |
| TESOP2002489 | 1402 | 18.. 365 | 3590 |
| TESOP2002539 | 1403 | 76.. 459 | 3591 |
| TESOP2002950 | 1404 | 1262..1747 | 3592 |
| TESOP2003273 | 1405 | 691..1032 | 3593 |
| TESOP2003753 | 1406 | 97.. 399 | 3594 |
| TESOP2004114 | 1407 | 510..1766 | 3595 |
| TESOP2005285 | 1408 | 510..1181 | 3596 |
| TESOP2005485 | 1409 | 62.. 994 | 3597 |
| TESOP2005579 | 1410 | 755..1729 | 3598 |
| TESOP2006041 | 1411 | 315..1538 | 3599 |
| TESOP2006060 | 1412 | 606..1040 | 3600 |
| TESOP2006068 | 1413 | 930..1382 | 3601 |
| TESOP2006670 | 1414 | 744..1235 | 3602 |
| TESOP2006746 | 1415 | 1158..1655 | 3603 |
| TESOP2007052 | 1416 | 28.. 351 | 3604 |
| TESOP2007262 | 1417 | 870..2078 | 3605 |
| TESOP2007636 | 1418 | 1053..1403 | 3606 |
| TESOP2007688 | 1419 | 210.. 863 | 3607 |
| TESOP2009121 | 1420 | 39..>2370 | 3608 |
| TESOP2009555 | 1421 | 741..1565 | 3609 |
| TESTI1000257 | 1422 | 377..1870 | 3610 |
| TESTI1000319 | 1423 | 107..2773 | 3611 |
| TESTI1000330 | 1424 | 271.. 576 | 3612 |
| TESTI1000348 | 1425 | 802..1251 | 3613 |
| TESTI1000390 | 1426 | 1642..3522 | 3614 |
| TESTI1000491 | 1427 | 33.. 428 | 3615 |
| TESTI1000545 | 1428 | 836..3625 | 3616 |
| TESTI2000443 | 1429 | 682..2265 | 3617 |
| TESTI2000644 | 1430 | 864..1211 | 3618 |
| TESTI2002036 | 1431 | 114..1700 | 3619 |
| TESTI2002618 | 1432 | 42.. 881 | 3620 |
| TESTI2002928 | 1433 | 272.. 757 | 3621 |
| TESTI2003347 | 1434 | 285..1829 | 3622 |
| TESTI2003573 | 1435 | 326..1363 | 3623 |
| TESTI2004215 | 1436 | 300..2456 | 3624 |
| TESTI2004700 | 1437 | 614..1273 | 3625 |
| TESTI2005376 | 1438 | 412..2103 | 3626 |
| TESTI2005610 | 1439 | 426..1964 | 3627 |
| TESTI2005739 | 1440 | 514..1848 | 3628 |
| TESTI2005986 | 1441 | 204.. 551 | 3629 |
| TESTI2006041 | 1442 | 697..1047 | 3630 |
| TESTI2006643 | 1443 | 812..1882 | 3631 |
| TESTI2006648 | 1444 | 204..1919 | 3632 |
| TESTI2009474 | 1445 | 283..1536 | 3633 |
| TESTI2009477 | 1446 | 1176..1649 | 3634 |
| TESTI2009511 | 1447 | 154.. 663 | 3635 |
| TESTI2009812 | 1448 | 910..1308 | 3636 |
| TESTI2010400 | 1449 | 1814..2224 | 3637 |
| TESTI2013381 | 1450 | 351.. 695 | 3638 |
| TESTI2013382 | 1451 | 1332..1826 | 3639 |
| TESTI2014716 | 1452 | 64..1443 | 3640 |
| TESTI2014843 | 1453 | 148..1866 | 3641 |
| TESTI2016046 | 1454 | 152..2173 | 3642 |
| TESTI2017727 | 1455 | 83.. 541 | 3643 |
| TESTI2018838 | 1456 | 647..1147 | 3644 |
| TESTI2019042 | 1457 | 130..1011 | 3645 |
| TESTI2019648 | 1458 | 1044..1463 | 3646 |
| TESTI2023254 | 1459 | 206..1972 | 3647 |
| TESTI2023599 | 1460 | 200.. 565 | 3648 |
| TESTI2024567 | 1461 | 1072..>1878 | 3649 |
| TESTI2026505 | 1462 | 69..1691 | 3650 |
| TESTI2027019 | 1463 | 608..1342 | 3651 |
| TESTI2031529 | 1464 | 386..1819 | 3652 |
| TESTI2034520 | 1465 | 59..1519 | 3653 |
| TESTI2034749 | 1466 | 973..1503 | 3654 |
| TESTI2034767 | 1467 | 374..1786 | 3655 |
| TESTI2034953 | 1468 | 703..1113 | 3656 |
| TESTI2034997 | 1469 | 179.. 547 | 3657 |
| TESTI2035107 | 1470 | 126.. 533 | 3658 |
| TESTI2035997 | 1471 | 826..1140 | 3659 |
| TESTI2036513 | 1472 | 1569..1895 | 3660 |
| TESTI2036684 | 1473 | 297.. 839 | 3661 |
| TESTI2037643 | 1474 | 3.. 599 | 3662 |
| TESTI2040018 | 1475 | 441..>1825 | 3663 |
| TESTI2042450 | 1476 | 212.. 544 | 3664 |
| TESTI2044796 | 1477 | 913..1692 | 3665 |
| TESTI2044833 | 1478 | 265.. 633 | 3666 |
| TESTI2045920 | 1479 | 15.. 758 | 3667 |
| TESTI2045983 | 1480 | 103.. 609 | 3668 |
| TESTI2046347 | 1481 | 465..1022 | 3669 |
| TESTI2047071 | 1482 | 596..1012 | 3670 |
| TESTI2048465 | 1483 | 755..1315 | 3671 |
| TESTI2048603 | 1484 | 122.. 490 | 3672 |
| TESTI2048898 | 1485 | 6.. 374 | 3673 |
| TESTI2049206 | 1486 | 677..1003 | 3674 |
| TESTI2049246 | 1487 | 296.. 658 | 3675 |
| TESTI2049277 | 1488 | 765..1130 | 3676 |
| TESTI2049422 | 1489 | 69.. 941 | 3677 |
| TESTI2049452 | 1490 | 28.. 381 | 3678 |
| TESTI2049469 | 1491 | 313..1341 | 3679 |
| TESTI2049576 | 1492 | 1030..1773 | 3680 |
| TESTI2049857 | 1493 | 816..2057 | 3681 |
| TESTI2050137 | 1494 | 6..1283 | 3682 |
| TESTI2050681 | 1495 | 186.. 605 | 3683 |
| TESTI2050987 | 1496 | 313..1104 | 3684 |
| TESTI2051279 | 1497 | 338.. 922 | 3685 |
| TESTI2051488 | 1498 | 665..1171 | 3686 |
| TESTI2051543 | 1499 | 925..1491 | 3687 |
| TESTI2051767 | 1500 | 568.. 945 | 3688 |
| TESTI2051806 | 1501 | 60..1091 | 3689 |
| TESTI2051867 | 1502 | 779..1780 | 3690 |
| TESTI2052211 | 1503 | 264..1358 | 3691 |
| TESTI2052693 | 1504 | 929..1954 | 3692 |
| TESTI2052698 | 1505 | 451..1011 | 3693 |
| TESTI2052822 | 1506 | 103.. 459 | 3694 |
| TESTI2053242 | 1507 | 86.. 574 | 3695 |
| TESTI2053399 | 1508 | 81.. 503 | 3696 |
| TESTI2053526 | 1509 | 116.. 502 | 3697 |
| TESTI2053621 | 1510 | 977..1582 | 3698 |
| TESTI4000014 | 1511 | 30.. 4214 | 3699 |
| TESTI4000068 | 1512 | 1820..2359 | 3700 |
| TESTI4000079 | 1513 | 1263..3458 | 3701 |
| TESTI4000209 | 1514 | 24..1049 | 3702 |
| TESTI4000215 | 1515 | 230.. 922 | 3703 |
| TESTI4000250 | 1516 | 56.. 496 | 3704 |
| TESTI4000288 | 1517 | 546..1082 | 3705 |
| TESTI4000349 | 1518 | 1259..3469 | 3706 |
| TESTI4000462 | 1519 | 886..1497 | 3707 |
| TESTI4000530 | 1520 | 346.. 879 | 3708 |
| TESTI4000724 | 1521 | 110..1639 | 3709 |
| TESTI4000970 | 1522 | 458..2476 | 3710 |
| TESTI4001100 | 1523 | 55.. 723 | 3711 |
| TESTI4001106 | 1524 | 453..2180 | 3712 |
| TESTI4001148 | 1525 | 94..1947 | 3713 |
| TESTI4001176 | 1526 | 636..1178 | 3714 |
| TESTI4001201 | 1527 | 106.. 441 | 3715 |
| TESTI4001206 | 1528 | 500.. 916 | 3716 |
| TESTI4001527 | 1529 | 710..1468 | 3717 |
| TESTI4001561 | 1530 | 2780..>3955 | 3718 |
| TESTI4001665 | 1531 | 4092..4538 | 3719 |
| TESTI4001923 | 1532 | 200.. 658 | 3720 |
| TESTI4002290 | 1533 | 1054..1497 | 3721 |
| TESTI4002491 | 1534 | 1553..1873 | 3722 |
| TESTI4002552 | 1535 | 2112..3710 | 3723 |
| TESTI4002647 | 1536 | 190..>3607 | 3724 |
| TESTI4002703 | 1537 | 1531..2484 | 3725 |
| TESTI4002754 | 1538 | 157.. 600 | 3726 |
| TESTI4002878 | 1539 | 150.. 650 | 3727 |
| TESTI4004200 | 1540 | 4.. 498 | 3728 |
| TESTI4005628 | 1541 | 158.. 487 | 3729 |
| TESTI4005805 | 1542 | 4.. 468 | 3730 |
| TESTI4005857 | 1543 | 3018..3860 | 3731 |
| TESTI4005961 | 1544 | 3600..3911 | 3732 |
| TESTI4006053 | 1545 | 163.. 480 | 3733 |
| TESTI4006079 | 1546 | 1652..3514 | 3734 |
| TESTI4006112 | 1547 | 799..1635 | 3735 |
| TESTI4006137 | 1548 | 235.. 798 | 3736 |
| TESTI4006148 | 1549 | 358..1035 | 3737 |
| TESTI4006219 | 1550 | 138.. 479 | 3738 |
| TESTI4006326 | 1551 | 1194..2537 | 3739 |
| TESTI4006393 | 1552 | 2691..3194 | 3740 |
| TESTI4006412 | 1553 | 83.. 427 | 3741 |
| TESTI4006420 | 1554 | 1850..2674 | 3742 |
| TESTI4006546 | 1555 | 286..3543 | 3743 |
| TESTI4006802 | 1556 | 95..3277 | 3744 |
| TESTI4006819 | 1557 | 1355..1681 | 3745 |
| TESTI4007064 | 1558 | 5..4678 | 3746 |
| TESTI4007163 | 1559 | 2933..3799 | 3747 |
| TESTI4007203 | 1560 | 79.. 420 | 3748 |
| TESTI4007239 | 1561 | 36..4373 | 3749 |
| TESTI4007373 | 1562 | 77.. 493 | 3750 |
| TESTI4007382 | 1563 | 32..2785 | 3751 |
| TESTI4007404 | 1564 | 82..4038 | 3752 |
| TESTI4007489 | 1565 | 3867..4589 | 3753 |
| TESTI4007775 | 1566 | 1493..1870 | 3754 |
| TESTI4007778 | 1567 | 665..3337 | 3755 |
| TESTI4007799 | 1568 | 1200..2039 | 3756 |
| TESTI4007810 | 1569 | 1972..2739 | 3757 |
| TESTI4008007 | 1570 | 1375..1983 | 3758 |
| TESTI4008018 | 1571 | 2012..2338 | 3759 |
| TESTI4008050 | 1572 | 968..2101 | 3760 |
| TESTI4008219 | 1573 | 2148..2618 | 3761 |
| TESTI4008401 | 1574 | 288.. 731 | 3762 |
| TESTI4008429 | 1575 | 2906..4009 | 3763 |
| TESTI4008573 | 1576 | 199.. 528 | 3764 |
| TESTI4008797 | 1577 | 2518..3342 | 3765 |
| TESTI4008816 | 1578 | 661..3057 | 3766 |
| TESTI4008935 | 1579 | 2086..3030 | 3767 |
| TESTI4008993 | 1580 | 224..1048 | 3768 |
| TESTI4009022 | 1581 | 2341..2706 | 3769 |
| TESTI4009034 | 1582 | 4067..4510 | 3770 |
| TESTI4009123 | 1583 | 33.. 374 | 3771 |
| TESTI4009160 | 1584 | 51..2603 | 3772 |
| TESTI4009215 | 1585 | 1159..1467 | 3773 |
| TESTI4009283 | 1586 | 1816..2547 | 3774 |
| TESTI4009286 | 1587 | 4041..4886 | 3775 |
| TESTI4009374 | 1588 | 180..1592 | 3776 |
| TESTI4009406 | 1589 | 2151..3299 | 3777 |
| TESTI4009457 | 1590 | 364.. 858 | 3778 |
| TESTI4009563 | 1591 | 2555..3313 | 3779 |
| TESTI4009608 | 1592 | 531.. 908 | 3780 |
| TESTI4009638 | 1593 | 85.. 624 | 3781 |
| TESTI4009881 | 1594 | 583..3669 | 3782 |
| TESTI4010211 | 1595 | 46.. 405 | 3783 |
| TESTI4010377 | 1596 | 1057..1476 | 3784 |
| TESTI4010713 | 1597 | 106..3780 | 3785 |
| TESTI4010789 | 1598 | 2218..2778 | 3786 |
| TESTI4010817 | 1599 | 1062..>3380 | 3787 |
| TESTI4010831 | 1600 | 486..3395 | 3788 |
| TESTI4010851 | 1601 | 92..>4555 | 3789 |
| TESTI4010928 | 1602 | 197.. 769 | 3790 |
| TESTI4011118 | 1603 | 4088..4399 | 3791 |
| TESTI4011161 | 1604 | 1397..3091 | 3792 |
| TESTI4011246 | 1605 | 1723..2106 | 3793 |
| TESTI4011484 | 1606 | 201..2336 | 3794 |
| TESTI4011505 | 1607 | 1357..1815 | 3795 |
| TESTI4011745 | 1608 | 2319..>4990 | 3796 |
| TESTI4011956 | 1609 | 162..2786 | 3797 |
| TESTI4012086 | 1610 | 177.. 731 | 3798 |
| TESTI4012329 | 1611 | 524.. 907 | 3799 |
| TESTI4012406 | 1612 | 141.. 611 | 3800 |
| TESTI4012448 | 1613 | 1666..3051 | 3801 |
| TESTI4012505 | 1614 | 1119..3005 | 3802 |
| TESTI4012556 | 1615 | 1928..2431 | 3803 |
| TESTI4012679 | 1616 | 1037..2071 | 3804 |
| TESTI4012702 | 1617 | 72..1529 | 3805 |
| TESTI4013369 | 1618 | 3026..3454 | 3806 |
| TESTI4013667 | 1619 | 351.. 659 | 3807 |
| TESTI4013675 | 1620 | 287.. 592 | 3808 |
| TESTI4013685 | 1621 | 2650..3045 | 3809 |
| TESTI4013735 | 1622 | 2964..3386 | 3810 |
| TESTI4013817 | 1623 | 2764..3237 | 3811 |
| TESTI4013830 | 1624 | 163..4938 | 3812 |
| TESTI4013924 | 1625 | 168..2039 | 3813 |
| TESTI4014159 | 1626 | 2462..2929 | 3814 |
| TESTI4014175 | 1627 | 836..2569 | 3815 |
| TESTI4014306 | 1628 | 2600..3031 | 3816 |
| TESTI4014392 | 1629 | 2506..2877 | 3817 |
| TESTI4014445 | 1630 | 23.. 364 | 3818 |
| TESTI4014694 | 1631 | 65.. 424 | 3819 |
| TESTI4014818 | 1632 | 1881..2957 | 3820 |
| TESTI4014924 | 1633 | 131..3898 | 3821 |
| TESTI4015263 | 1634 | 246.. 746 | 3822 |
| TESTI4015293 | 1635 | 2214..4253 | 3823 |
| TESTI4015471 | 1636 | 3317..3808 | 3824 |
| TESTI4015600 | 1637 | 2050..2508 | 3825 |
| TESTI4015646 | 1638 | 1674..2030 | 3826 |
| TESTI4015681 | 1639 | 2551..3006 | 3827 |
| TESTI4015688 | 1640 | 37.. 432 | 3828 |
| TESTI4016110 | 1641 | 2559..3257 | 3829 |
| TESTI4016238 | 1642 | 12.. 371 | 3830 |
| TESTI4016551 | 1643 | 2731..3495 | 3831 |
| TESTI4016812 | 1644 | 53..3097 | 3832 |
| TESTI4016822 | 1645 | 203.. 688 | 3833 |
| TESTI4016882 | 1646 | 131.. 958 | 3834 |
| TESTI4016925 | 1647 | 49..4704 | 3835 |
| TESTI4017001 | 1648 | 1..1251 | 3836 |
| TESTI4017137 | 1649 | 1822..2391 | 3837 |
| TESTI4017254 | 1650 | 295.. 645 | 3838 |
| TESTI4017543 | 1651 | 30..3824 | 3839 |
| TESTI4017575 | 1652 | 32..3679 | 3840 |
| TESTI4017848 | 1653 | 170.. 544 | 3841 |
| TESTI4017901 | 1654 | 50.. 361 | 3842 |
| TESTI4017961 | 1655 | 2224..2679 | 3843 |
| TESTI4018152 | 1656 | 1279..2082 | 3844 |
| TESTI4018208 | 1657 | 114.. 461 | 3845 |
| TESTI4018382 | 1658 | 819..1208 | 3846 |
| TESTI4018555 | 1659 | 980.. 1633 | 3847 |
| TESTI4018806 | 1660 | 156.. 587 | 3848 |
| TESTI4018835 | 1661 | 1223..3052 | 3849 |
| TESTI4018881 | 1662 | 1303..2754 | 3850 |
| TESTI4018886 | 1663 | 1235..2332 | 3851 |
| TESTI4019140 | 1664 | 622..2322 | 3852 |
| TESTI4019299 | 1665 | 122..1195 | 3853 |
| TESTI4019417 | 1666 | 247.. 618 | 3854 |
| TESTI4019566 | 1667 | 35..2755 | 3855 |
| TESTI4019843 | 1668 | 165..2381 | 3856 |
| TESTI4020092 | 1669 | 1074..1418 | 3857 |
| TESTI4020102 | 1670 | 1262..1693 | 3858 |
| TESTI4020806 | 1671 | 367.. 753 | 3859 |
| TESTI4020920 | 1672 | 846..2930 | 3860 |
| TESTI4021294 | 1673 | 1..3249 | 3861 |
| TESTI4021456 | 1674 | 445.. 900 | 3862 |
| TESTI4021478 | 1675 | 116..2476 | 3863 |
| TESTI4021491 | 1676 | 1532..1972 | 3864 |
| TESTI4022716 | 1677 | 577..3141 | 3865 |
| TESTI4022873 | 1678 | 166..3783 | 3866 |
| TESTI4022936 | 1679 | 54.. 854 | 3867 |
| TESTI4023546 | 1680 | 150..2882 | 3868 |
| TESTI4023555 | 1681 | 1769..2470 | 3869 |
| TESTI4023722 | 1682 | 729..1112 | 3870 |
| TESTI4023762 | 1683 | 471..2999 | 3871 |
| TESTI4023942 | 1684 | 47.. 361 | 3872 |
| TESTI4024344 | 1685 | 3.. 548 | 3873 |
| TESTI4024420 | 1686 | 96..3140 | 3874 |
| TESTI4024874 | 1687 | 418.. 918 | 3875 |
| TESTI4024890 | 1688 | 2189..2623 | 3876 |
| TESTI4024907 | 1689 | 1280..1729 | 3877 |
| TESTI4025731 | 1690 | 266.. 610 | 3878 |
| TESTI4025797 | 1691 | 56..2869 | 3879 |
| TESTI4025920 | 1692 | 76..3117 | 3880 |
| TESTI4026079 | 1693 | 3.. 323 | 3881 |
| TESTI4026192 | 1694 | 1.. 390 | 3882 |
| TESTI4026295 | 1695 | 145.. 498 | 3883 |
| TESTI4026456 | 1696 | 117.. 443 | 3884 |
| TESTI4026510 | 1697 | 1733..3232 | 3885 |
| TESTI4026524 | 1698 | 744..2759 | 3886 |
| TESTI4026700 | 1699 | 242..2626 | 3887 |
| TESTI4026762 | 1700 | 392..4093 | 3888 |
| TESTI4026785 | 1701 | 107.. 508 | 3889 |
| TESTI4027516 | 1702 | 3747..4265 | 3890 |
| TESTI4027557 | 1703 | 1469..2440 | 3891 |
| TESTI4027821 | 1704 | 1226..1855 | 3892 |
| TESTI4028059 | 1705 | 1786..3183 | 3893 |
| TESTI4028062 | 1706 | 53.. 529 | 3894 |
| TESTI4028429 | 1707 | 1.. 306 | 3895 |
| TESTI4028612 | 1708 | 357..4304 | 3896 |
| TESTI4028809 | 1709 | 1672..2172 | 3897 |
| TESTI4028823 | 1710 | 119..>2529 | 3898 |
| TESTI4028880 | 1711 | 758..2320 | 3899 |
| TESTI4028983 | 1712 | 283.. 924 | 3900 |
| TESTI4029370 | 1713 | 79.. 447 | 3901 |
| TESTI4029671 | 1714 | 59..1408 | 3902 |
| TESTI4029836 | 1715 | 76..2982 | 3903 |
| TESTI4030069 | 1716 | 219.. 650 | 3904 |
| TESTI4030159 | 1717 | 34.. 621 | 3905 |
| TESTI4030505 | 1718 | 142..3492 | 3906 |
| TESTI4030603 | 1719 | 37..1011 | 3907 |
| TESTI4030669 | 1720 | 3..3041 | 3908 |
| TESTI4032895 | 1721 | 212..2701 | 3909 |
| TESTI4033433 | 1722 | 875..1519 | 3910 |
| TESTI4033690 | 1723 | 206..>3057 | 3911 |
| TESTI4034172 | 1724 | 97.. 435 | 3912 |
| TESTI4034212 | 1725 | 296..1093 | 3913 |
| TESTI4034432 | 1726 | 4035..>4449 | 3914 |
| TESTI4034632 | 1727 | 1306..2199 | 3915 |
| TESTI4034912 | 1728 | 201..3137 | 3916 |
| TESTI4035063 | 1729 | 894..3518 | 3917 |
| TESTI4035065 | 1730 | 1418..1927 | 3918 |
| TESTI4035498 | 1731 | 39.. 704 | 3919 |
| TESTI4035602 | 1732 | 39.. 389 | 3920 |
| TESTI4035637 | 1733 | 1..2937 | 3921 |
| TESTI4035649 | 1734 | 19.. 441 | 3922 |
| TESTI4036042 | 1735 | 3085..3465 | 3923 |
| TESTI4036909 | 1736 | 130..2910 | 3924 |
| TESTI4037066 | 1737 | 1285..4461 | 3925 |
| TESTI4037156 | 1738 | 1119..2738 | 3926 |
| TESTI4037188 | 1739 | 142..2280 | 3927 |
| TESTI4037244 | 1740 | 52.. 354 | 3928 |
| TESTI4037727 | 1741 | 250..3051 | 3929 |
| TESTI4038156 | 1742 | 163.. 489 | 3930 |
| TESTI4038223 | 1743 | 112.. 654 | 3931 |
| TESTI4038258 | 1744 | 83.. 403 | 3932 |
| TESTI4038339 | 1745 | 458..2428 | 3933 |
| TESTI4038492 | 1746 | 415.. 960 | 3934 |
| TESTI4038818 | 1747 | 1053..1442 | 3935 |
| TESTI4039038 | 1748 | 1270..2871 | 3936 |
| TESTI4039086 | 1749 | 1902..2606 | 3937 |
| TESTI4039659 | 1750 | 237.. 935 | 3938 |
| TESTI4040363 | 1751 | 255.. 677 | 3939 |
| TESTI4040800 | 1752 | 59..1144 | 3940 |
| TESTI4040939 | 1753 | 23.. 823 | 3941 |
| TESTI4040956 | 1754 | 1316..1630 | 3942 |
| TESTI4041053 | 1755 | 89..3331 | 3943 |
| TESTI4041099 | 1756 | 3010..3369 | 3944 |
| TESTI4041143 | 1757 | 318.. 644 | 3945 |
| TESTI4041519 | 1758 | 4.. 345 | 3946 |
| TESTI4041624 | 1759 | 6.. 458 | 3947 |
| TESTI4041903 | 1760 | 69.. 458 | 3948 |
| TESTI4041954 | 1761 | 130.. 498 | 3949 |
| TESTI4042098 | 1762 | 1523..2296 | 3950 |
| TESTI4042444 | 1763 | 1808..2239 | 3951 |
| TESTI4042711 | 1764 | 220.. 561 | 3952 |
| TESTI4043129 | 1765 | 1129..1629 | 3953 |
| TESTI4043203 | 1766 | 84.. 437 | 3954 |
| TESTI4043551 | 1767 | 2185..2754 | 3955 |
| TESTI4043947 | 1768 | 14..2920 | 3956 |
| TESTI4044035 | 1769 | 140.. 682 | 3957 |
| TESTI4044084 | 1770 | 6.. 491 | 3958 |
| TESTI4044123 | 1771 | 227.. 637 | 3959 |
| TESTI4044186 | 1772 | 263.. 844 | 3960 |
| TESTI4044234 | 1773 | 184.. 987 | 3961 |
| TESTI4044296 | 1774 | 1968..2684 | 3962 |
| TESTI4044682 | 1775 | 36.. 491 | 3963 |
| TESTI4045312 | 1776 | 375.. 734 | 3964 |
| TESTI4046253 | 1777 | 151.. 456 | 3965 |
| TESTI4046282 | 1778 | 79..3672 | 3966 |
| TESTI4046487 | 1779 | 1351..2883 | 3967 |
| TESTI4046819 | 1780 | 299..3535 | 3968 |
| TESTI4046884 | 1781 | 99..>3063 | 3969 |
| TESTI4047069 | 1782 | 1554..1895 | 3970 |
| THYMU1000496 | 1783 | 324..1295 | 3971 |
| THYMU1000600 | 1784 | 390.. 809 | 3972 |
| THYMU2000932 | 1785 | 1767..2492 | 3973 |
| THYMU2001053 | 1786 | 1669..2070 | 3974 |
| THYMU2001090 | 1787 | 1738..2067 | 3975 |
| THYMU2003397 | 1788 | 1100..1543 | 3976 |
| THYMU2003632 | 1789 | 1483..1812 | 3977 |
| THYMU2003760 | 1790 | 304.. 612 | 3978 |
| THYMU2004693 | 1791 | 1445..1792 | 3979 |
| THYMU2005003 | 1792 | 1245..1697 | 3980 |
| THYMU2005190 | 1793 | 133.. 531 | 3981 |
| THYMU2005303 | 1794 | 909..1367 | 3982 |
| THYMU2005321 | 1795 | 81.. 473 | 3983 |
| THYMU2006420 | 1796 | 335..1147 | 3984 |
| THYMU2007060 | 1797 | 786..>2283 | 3985 |
| THYMU2007179 | 1798 | 310..>1233 | 3986 |
| THYMU2007658 | 1799 | 452.. 913 | 3987 |
| THYMU2008282 | 1800 | 1301..1741 | 3988 |
| THYMU2008725 | 1801 | 47..1876 | 3989 |
| THYMU2009134 | 1802 | 147.. 674 | 3990 |
| THYMU2009157 | 1803 | 1691..2095 | 3991 |
| THYMU2009425 | 1804 | 4.. 525 | 3992 |
| THYMU2011548 | 1805 | 70..1044 | 3993 |
| THYMU2011736 | 1806 | 1019..1939 | 3994 |
| THYMU2013386 | 1807 | 421..1773 | 3995 |
| THYMU2014353 | 1808 | 765..1100 | 3996 |
| THYMU2016204 | 1809 | 880..1923 | 3997 |
| THYMU2016523 | 1810 | 2076..2411 | 3998 |
| THYMU2019210 | 1811 | 55..1359 | 3999 |
| THYMU2019587 | 1812 | 1196..1633 | 4000 |
| THYMU2023711 | 1813 | 80..1486 | 4001 |
| THYMU2023967 | 1814 | 1000..1479 | 4002 |
| THYMU2025707 | 1815 | 18.. 527 | 4003 |
| THYMU2027497 | 1816 | 84.. 884 | 4004 |
| THYMU2027695 | 1817 | 52..1611 | 4005 |
| THYMU2027734 | 1818 | 581..1141 | 4006 |
| THYMU2028978 | 1819 | 283.. 642 | 4007 |
| THYMU2029676 | 1820 | 1209..1868 | 4008 |
| THYMU2029688 | 1821 | 45.. 368 | 4009 |
| THYMU2030068 | 1822 | 91.. 477 | 4010 |
| THYMU2030226 | 1823 | 207.. 710 | 4011 |
| THYMU2030264 | 1824 | 152..1756 | 4012 |
| THYMU2030637 | 1825 | 147..1865 | 4013 |
| THYMU2030796 | 1826 | 328.. 642 | 4014 |
| THYMU2031046 | 1827 | 23.. 703 | 4015 |
| THYMU2031218 | 1828 | 563.. 925 | 4016 |
| THYMU2031258 | 1829 | 127.. 681 | 4017 |
| THYMU2031341 | 1830 | 2050..2376 | 4018 |
| THYMU2031368 | 1831 | 498.. 821 | 4019 |
| THYMU2031579 | 1832 | 258.. 578 | 4020 |
| THYMU2031847 | 1833 | 145.. 492 | 4021 |
| THYMU2031890 | 1834 | 4.. 348 | 4022 |
| THYMU2032014 | 1835 | 880..1371 | 4023 |
| THYMU2032035 | 1836 | 186.. 533 | 4024 |
| THYMU2032080 | 1837 | 366.. 674 | 4025 |
| THYMU2032358 | 1838 | 230.. 604 | 4026 |
| THYMU2032437 | 1839 | 665.. 1090 | 4027 |
| THYMU2032655 | 1840 | 360.. 701 | 4028 |
| THYMU2032696 | 1841 | 1133..2215 | 4029 |
| THYMU2032825 | 1842 | 189.. 818 | 4030 |
| THYMU2033070 | 1843 | 22.. 441 | 4031 |
| THYMU2033079 | 1844 | 1706..2023 | 4032 |
| THYMU2033104 | 1845 | 1963..2298 | 4033 |
| THYMU2033308 | 1846 | 1067..1369 | 4034 |
| THYMU2033787 | 1847 | 1808..2656 | 4035 |
| THYMU2033816 | 1848 | 1947..2336 | 4036 |
| THYMU2034314 | 1849 | 1836..2189 | 4037 |
| THYMU2034374 | 1850 | 1119..1637 | 4038 |
| THYMU2034647 | 1851 | 1557..2051 | 4039 |
| THYMU2035064 | 1852 | 24.. 428 | 4040 |
| THYMU2035101 | 1853 | 17.. 334 | 4041 |
| THYMU2035319 | 1854 | 641..2161 | 4042 |
| THYMU2035388 | 1855 | 37.. 408 | 4043 |
| THYMU2035400 | 1856 | 96.. 494 | 4044 |
| THYMU2035735 | 1857 | 99..1619 | 4045 |
| THYMU2036058 | 1858 | 1381..1785 | 4046 |
| THYMU2036085 | 1859 | 2311..2922 | 4047 |
| THYMU2036252 | 1860 | 22.. 459 | 4048 |
| THYMU2036265 | 1861 | 1260..1568 | 4049 |
| THYMU2036459 | 1862 | 347..3121 | 4050 |
| THYMU2036653 | 1863 | 422.. 754 | 4051 |
| THYMU2037081 | 1864 | 78.. 533 | 4052 |
| THYMU2037208 | 1865 | 1156..1608 | 4053 |
| THYMU2037226 | 1866 | 375..4157 | 4054 |
| THYMU2037233 | 1867 | 2777..3235 | 4055 |
| THYMU2037348 | 1868 | 115.. 423 | 4056 |
| THYMU2037965 | 1869 | 167.. 598 | 4057 |
| THYMU2038189 | 1870 | 84.. 452 | 4058 |
| THYMU2038301 | 1871 | 1019..1417 | 4059 |
| THYMU2038369 | 1872 | 11.. 826 | 4060 |
| THYMU2038615 | 1873 | 597..1100 | 4061 |
| THYMU2038636 | 1874 | 19.. 387 | 4062 |
| THYMU2038739 | 1875 | 1446..1874 | 4063 |
| THYMU2038772 | 1876 | 50.. 532 | 4064 |
| THYMU2038797 | 1877 | 75..1304 | 4065 |
| THYMU2039305 | 1878 | 1607..1969 | 4066 |
| THYMU2039315 | 1879 | 26..1549 | 4067 |
| THYMU2039350 | 1880 | 86..1891 | 4068 |
| THYMU2039411 | 1881 | 423.. 800 | 4069 |
| THYMU2039780 | 1882 | 292.. 831 | 4070 |
| THYMU2039989 | 1883 | 1651..2124 | 4071 |
| THYMU2040140 | 1884 | 72.. 437 | 4072 |
| THYMU2040412 | 1885 | 660..1049 | 4073 |
| THYMU2040824 | 1886 | 13.. 693 | 4074 |
| THYMU2040975 | 1887 | 174..1262 | 4075 |
| THYMU2041007 | 1888 | 54.. 401 | 4076 |
| THYMU2041015 | 1889 | 251..1798 | 4077 |
| THYMU2041252 | 1890 | 301.. 798 | 4078 |
| THYMU3000028 | 1891 | 883..1701 | 4079 |
| THYMU3000036 | 1892 | 480.. 881 | 4080 |
| THYMU3000133 | 1893 | 312..2012 | 4081 |
| THYMU3000655 | 1894 | 148.. 774 | 4082 |
| THYMU3000826 | 1895 | 225.. 779 | 4083 |
| THYMU3001083 | 1896 | 2429..2761 | 4084 |
| THYMU3001234 | 1897 | 1340..2329 | 4085 |
| THYMU3001379 | 1898 | 599..2251 | 4086 |
| THYMU3001472 | 1899 | 1556..1870 | 4087 |
| THYMU3001991 | 1900 | 2453..2782 | 4088 |
| THYMU3002452 | 1901 | 1314..1697 | 4089 |
| THYMU3002661 | 1902 | 818..1348 | 4090 |
| THYMU3003212 | 1903 | 1292..1726 | 4091 |
| THYMU3003309 | 1904 | 2336..2866 | 4092 |
| THYMU3003763 | 1905 | 1011..1991 | 4093 |
| THYMU3004157 | 1906 | 579.. 899 | 4094 |
| THYMU3004835 | 1907 | 77..1282 | 4095 |
| THYMU3004866 | 1908 | 703..2709 | 4096 |
| THYMU3005696 | 1909 | 2267..2569 | 4097 |
| THYMU3006118 | 1910 | 924..1331 | 4098 |
| THYMU3006132 | 1911 | 237..1703 | 4099 |
| THYMU3006168 | 1912 | 2025..2813 | 4100 |
| THYMU3006172 | 1913 | 240..1823 | 4101 |
| THYMU3006371 | 1914 | 1600..2007 | 4102 |
| THYMU3006485 | 1915 | 1386..1796 | 4103 |
| THYMU3006811 | 1916 | 27.. 395 | 4104 |
| THYMU3006963 | 1917 | 386.. 784 | 4105 |
| THYMU3007137 | 1918 | 404..2878 | 4106 |
| THYMU3007368 | 1919 | 2076..2516 | 4107 |
| THYMU3007845 | 1920 | 1862..2281 | 4108 |
| THYMU3008171 | 1921 | 1204..1773 | 4109 |
| THYMU3008436 | 1922 | 225..2780 | 4110 |
| THYMU3009255 | 1923 | 186.. 524 | 4111 |
| TKIDN2000701 | 1924 | 1716..2306 | 4112 |
| TKIDN2002424 | 1925 | 42.. 377 | 4113 |
| TKIDN2002632 | 1926 | 350.. 730 | 4114 |
| TKIDN2003044 | 1927 | 1520..1840 | 4115 |
| TKIDN2004386 | 1928 | 1489..2130 | 4116 |
| TKIDN2005934 | 1929 | 158.. 571 | 4117 |
| TKIDN2005947 | 1930 | 144.. 533 | 4118 |
| TKIDN2006525 | 1931 | 613..1590 | 4119 |
| TKIDN2006852 | 1932 | 665..1294 | 4120 |
| TKIDN2007667 | 1933 | 1640..>2145 | 4121 |
| TKIDN2009092 | 1934 | 665..1021 | 4122 |
| TKIDN2009641 | 1935 | 538.. 852 | 4123 |
| TKIDN2009889 | 1936 | 164.. 580 | 4124 |
| TKIDN2010934 | 1937 | 516..1220 | 4125 |
| TKIDN2012824 | 1938 | 434.. 745 | 4126 |
| TKIDN2013287 | 1939 | 331.. 663 | 4127 |
| TKIDN2014757 | 1940 | 223.. 537 | 4128 |
| TKIDN2014771 | 1941 | 244.. 642 | 4129 |
| TKIDN2015263 | 1942 | 43.. 396 | 4130 |
| TKIDN2015788 | 1943 | 841..1272 | 4131 |
| TKIDN2016309 | 1944 | 212.. 634 | 4132 |
| TKIDN2019116 | 1945 | 14.. 640 | 4133 |
| TLIVE2000023 | 1946 | 110.. 589 | 4134 |
| TLIVE2001327 | 1947 | 90..2918 | 4135 |
| TLIVE2001828 | 1948 | 155.. 589 | 4136 |
| TLIVE2001927 | 1949 | 557.. 895 | 4137 |
| TLIVE2002336 | 1950 | 3..1598 | 4138 |
| TLIVE2002338 | 1951 | 1005..1817 | 4139 |
| TLIVE2002690 | 1952 | 210..1490 | 4140 |
| TLIVE2003197 | 1953 | 88.. 474 | 4141 |
| TLIVE2003225 | 1954 | 60..1220 | 4142 |
| TLIVE2003381 | 1955 | 603..1097 | 4143 |
| TLIVE2003970 | 1956 | 230.. 640 | 4144 |
| TLIVE2004110 | 1957 | 68.. 424 | 4145 |
| TLIVE2004320 | 1958 | 1436..2650 | 4146 |
| TLIVE2004601 | 1959 | 242.. 586 | 4147 |
| TLIVE2005180 | 1960 | 1180..1518 | 4148 |
| TLIVE2006236 | 1961 | 59.. 409 | 4149 |
| TLIVE2006529 | 1962 | 572..1492 | 4150 |
| TLIVE2007132 | 1963 | 70.. 444 | 4151 |
| TLIVE2007528 | 1964 | 715..1110 | 4152 |
| TLIVE2007816 | 1965 | 214.. 669 | 4153 |
| TLIVE2008083 | 1966 | 1670..2002 | 4154 |
| TLIVE2008229 | 1967 | 17..1786 | 4155 |
| TLIVE2009541 | 1968 | 343..1413 | 4156 |
| TOVAR2000649 | 1969 | 192.. 605 | 4157 |
| TOVAR2001281 | 1970 | 71.. 403 | 4158 |
| TOVAR2001730 | 1971 | 94.. 672 | 4159 |
| TOVAR2002247 | 1972 | 317..1375 | 4160 |
| TOVAR2002549 | 1973 | 1301..1657 | 4161 |
| TRACH1000205 | 1974 | 89.. 928 | 4162 |
| TRACH2001443 | 1975 | 284.. 949 | 4163 |
| TRACH2001549 | 1976 | 190..1566 | 4164 |
| TRACH2001684 | 1977 | 1190..1684 | 4165 |
| TRACH2003070 | 1978 | 358.. 855 | 4166 |
| TRACH2004170 | 1979 | 349.. 795 | 4167 |
| TRACH2005066 | 1980 | 136.. 597 | 4168 |
| TRACH2005811 | 1981 | 1125..2234 | 4169 |
| TRACH2006049 | 1982 | 927..1247 | 4170 |
| TRACH2006387 | 1983 | 245..1258 | 4171 |
| TRACH2007059 | 1984 | 244..2031 | 4172 |
| TRACH2007834 | 1985 | 3.. 311 | 4173 |
| TRACH2008300 | 1986 | 414.. 752 | 4174 |
| TRACH2009310 | 1987 | 275..2413 | 4175 |
| TRACH2019248 | 1988 | 42.. 395 | 4176 |
| TRACH2019473 | 1989 | 179..1552 | 4177 |
| TRACH2020525 | 1990 | 1340..1912 | 4178 |
| TRACH2021398 | 1991 | 225.. 872 | 4179 |
| TRACH2021964 | 1992 | 105..>2454 | 4180 |
| TRACH2022042 | 1993 | 985..1332 | 4181 |
| TRACH2022425 | 1994 | 80..1573 | 4182 |
| TRACH2022553 | 1995 | 38..1744 | 4183 |
| TRACH2022649 | 1996 | 55..1491 | 4184 |
| TRACH2023299 | 1997 | 24..1670 | 4185 |
| TRACH2023306 | 1998 | 157..1071 | 4186 |
| TRACH2025344 | 1999 | 1616..2086 | 4187 |
| TRACH2025507 | 2000 | 1483..1848 | 4188 |
| TRACH2025535 | 2001 | 561..1253 | 4189 |
| TRACH2025749 | 2002 | 655..1017 | 4190 |
| TRACH2025911 | 2003 | 189.. 680 | 4191 |
| TRACH2025932 | 2004 | 201.. 530 | 4192 |
| TRACH3000014 | 2005 | 33..2963 | 4193 |
| TRACH3000342 | 2006 | 3259..3672 | 4194 |
| TRACH3000558 | 2007 | 3058..>3576 | 4195 |
| TRACH3000586 | 2008 | 1117..1662 | 4196 |
| TRACH3000926 | 2009 | 127..4203 | 4197 |
| TRACH3001427 | 2010 | 26..1021 | 4198 |
| TRACH3002064 | 2011 | 1457..1777 | 4199 |
| TRACH3002168 | 2012 | 730..2313 | 4200 |
| TRACH3002192 | 2013 | 24.. 464 | 4201 |
| TRACH3002650 | 2014 | 3122..3475 | 4202 |
| TRACH3002866 | 2015 | 90..1199 | 4203 |
| TRACH3002871 | 2016 | 1866..2183 | 4204 |
| TRACH3003379 | 2017 | 84..1916 | 4205 |
| TRACH3004068 | 2018 | 787..1104 | 4206 |
| TRACH3004537 | 2019 | 1535..2935 | 4207 |
| TRACH3004721 | 2020 | 1009..2847 | 4208 |
| TRACH3004786 | 2021 | 2629..3258 | 4209 |
| TRACH3004840 | 2022 | 79.. 714 | 4210 |
| TRACH3005294 | 2023 | 3984..4352 | 4211 |
| TRACH3005479 | 2024 | 90..3338 | 4212 |
| TRACH3005549 | 2025 | 81.. 845 | 4213 |
| TRACH3006038 | 2026 | 2846..3157 | 4214 |
| TRACH3006149 | 2027 | 2186..2494 | 4215 |
| TRACH3006228 | 2028 | 1016..3304 | 4216 |
| TRACH3006412 | 2029 | 1681..2196 | 4217 |
| TRACH3006470 | 2030 | 157..1983 | 4218 |
| TRACH3006889 | 2031 | 2712..3470 | 4219 |
| TRACH3007391 | 2032 | 3.. 308 | 4220 |
| TRACH3007479 | 2033 | 571..1602 | 4221 |
| TRACH3008093 | 2034 | 184.. 813 | 4222 |
| TRACH3008535 | 2035 | 2148..2456 | 4223 |
| TRACH3008629 | 2036 | 80..2737 | 4224 |
| TRACH3008713 | 2037 | 2044..2364 | 4225 |
| TRACH3009455 | 2038 | 1507..2718 | 4226 |
| TRACH3034731 | 2039 | 124..1233 | 4227 |
| TRACH3034762 | 2040 | 1177..1527 | 4228 |
| TRACH3035199 | 2041 | 234..1211 | 4229 |
| TRACH3035235 | 2042 | 3.. 350 | 4230 |
| TRACH3035482 | 2043 | 1879..2346 | 4231 |
| TRACH3035526 | 2044 | 17..1489 | 4232 |
| TRACH3036193 | 2045 | 172..4803 | 4233 |
| TRACH3036207 | 2046 | 268..1368 | 4234 |
| TRACH3036309 | 2047 | 50.. 691 | 4235 |
| TRACH3036456 | 2048 | 1214..1591 | 4236 |
| TRACH3036609 | 2049 | 3159..4154 | 4237 |
| TSTOM1000135 | 2050 | 201..1097 | 4238 |
| TSTOM2000442 | 2051 | 80..1633 | 4239 |
| TSTOM2000553 | 2052 | 170..1450 | 4240 |
| TSTOM2002672 | 2053 | 1088..1432 | 4241 |
| TUTER1000122 | 2054 | 10.. 363 | 4242 |
| TUTER2000425 | 2055 | 216.. 572 | 4243 |
| TUTER2000904 | 2056 | 18.. 773 | 4244 |
| TUTER2000916 | 2057 | 149.. 568 | 4245 |
| TUTER2001387 | 2058 | 1525..1908 | 4246 |
| TUTER2002729 | 2059 | 20.. 868 | 4247 |
| UTERU1000024 | 2060 | 15..1190 | 4248 |
| UTERU1000031 | 2061 | 86..1609 | 4249 |
| UTERU1000148 | 2062 | 1553..2005 | 4250 |
| UTERU1000249 | 2063 | 349..2499 | 4251 |
| UTERU1000337 | 2064 | 1062..1922 | 4252 |
| UTERU1000339 | 2065 | 18.. 341 | 4253 |
| UTERU2000649 | 2066 | 213.. 902 | 4254 |
| UTERU2001409 | 2067 | 236.. 553 | 4255 |
| UTERU2002410 | 2068 | 2655..2987 | 4256 |
| UTERU2002841 | 2069 | 1185..2021 | 4257 |
| UTERU2004688 | 2070 | 1487..2470 | 4258 |
| UTERU2004929 | 2071 | 2106..2462 | 4259 |
| UTERU2005004 | 2072 | 1795..2388 | 4260 |
| UTERU2005621 | 2073 | 98..1483 | 4261 |
| UTERU2006115 | 2074 | 128..1018 | 4262 |
| UTERU2006137 | 2075 | 1721..2068 | 4263 |
| UTERU2006568 | 2076 | 535..1458 | 4264 |
| UTERU2007444 | 2077 | 1104..1502 | 4265 |
| UTERU2007520 | 2078 | 1644..2126 | 4266 |
| UTERU2007724 | 2079 | 53.. 865 | 4267 |
| UTERU2008347 | 2080 | 123..1355 | 4268 |
| UTERU2014678 | 2081 | 1232..1585 | 4269 |
| UTERU2017762 | 2082 | 427..2319 | 4270 |
| UTERU2019491 | 2083 | 379.. 849 | 4271 |
| UTERU2019681 | 2084 | 1424..1840 | 4272 |
| UTERU2019706 | 2085 | 88..1656 | 4273 |
| UTERU2019940 | 2086 | 653..1228 | 4274 |
| UTERU2020491 | 2087 | 2042..2437 | 4275 |
| UTERU2020718 | 2088 | 355..1428 | 4276 |
| UTERU2021163 | 2089 | 1279..1620 | 4277 |
| UTERU2021380 | 2090 | 358.. 666 | 4278 |
| UTERU2022020 | 2091 | 1448..1789 | 4279 |
| UTERU2022981 | 2092 | 263.. 571 | 4280 |
| UTERU2023039 | 2093 | 172.. 681 | 4281 |
| UTERU2023175 | 2094 | 1231..2088 | 4282 |
| UTERU2023651 | 2095 | 647..1012 | 4283 |
| UTERU2023712 | 2096 | 399.. 758 | 4284 |
| UTERU2024002 | 2097 | 517..1026 | 4285 |
| UTERU2024656 | 2098 | 28.. 534 | 4286 |
| UTERU2025025 | 2099 | 814..2301 | 4287 |
| UTERU2025645 | 2100 | 263..1021 | 4288 |
| UTERU2025891 | 2101 | 1446..1787 | 4289 |
| UTERU2026025 | 2102 | 172.. 540 | 4290 |
| UTERU2026090 | 2103 | 65..1183 | 4291 |
| UTERU2026203 | 2104 | 612..1304 | 4292 |
| UTERU2027591 | 2105 | 427.. 747 | 4293 |
| UTERU2029953 | 2106 | 1415..1870 | 4294 |
| UTERU2030213 | 2107 | 1261..1950 | 4295 |
| UTERU2030280 | 2108 | 1134..1511 | 4296 |
| UTERU2031084 | 2109 | 162.. 728 | 4297 |
| UTERU2031268 | 2110 | 429..1985 | 4298 |
| UTERU2031521 | 2111 | 209.. 700 | 4299 |
| UTERU2031703 | 2112 | 1190..1654 | 4300 |
| UTERU2031851 | 2113 | 1192..1659 | 4301 |
| UTERU2033375 | 2114 | 887..2218 | 4302 |
| UTERU2033382 | 2115 | 1144..1614 | 4303 |
| UTERU2035114 | 2116 | 96.. 614 | 4304 |
| UTERU2035323 | 2117 | 1015..1467 | 4305 |
| UTERU2035328 | 2118 | 28..>2438 | 4306 |
| UTERU2035331 | 2119 | 656..1255 | 4307 |
| UTERU2035452 | 2120 | 1393..1899 | 4308 |
| UTERU2035469 | 2121 | 261.. 707 | 4309 |
| UTERU2035503 | 2122 | 1539..1859 | 4310 |
| UTERU2035745 | 2123 | 1964..2272 | 4311 |
| UTERU2036089 | 2124 | 1131..2297 | 4312 |
| UTERU2037361 | 2125 | 1427..1849 | 4313 |
| UTERU2037577 | 2126 | 384.. 725 | 4314 |
| UTERU2038251 | 2127 | 76..1215 | 4315 |
| UTERU3000226 | 2128 | 1353..1691 | 4316 |
| UTERU3000645 | 2129 | 2681..3310 | 4317 |
| UTERU3000665 | 2130 | 1945..3624 | 4318 |
| UTERU3000828 | 2131 | 98..2986 | 4319 |
| UTERU3000899 | 2132 | 26.. 871 | 4320 |
| UTERU3001059 | 2133 | 2772..4715 | 4321 |
| UTERU3001240 | 2134 | 762..2021 | 4322 |
| UTERU3001542 | 2135 | 2792..3097 | 4323 |
| UTERU3001571 | 2136 | 2792..3466 | 4324 |
| UTERU3001572 | 2137 | 412..3990 | 4325 |
| UTERU3001585 | 2138 | 40..1551 | 4326 |
| UTERU3001652 | 2139 | 115..1590 | 4327 |
| UTERU3001766 | 2140 | 2128..2478 | 4328 |
| UTERU3001988 | 2141 | 28.. 801 | 4329 |
| UTERU3002209 | 2142 | 2047..2406 | 4330 |
| UTERU3002218 | 2143 | 221..3025 | 4331 |
| UTERU3002383 | 2144 | 248.. 571 | 4332 |
| UTERU3002667 | 2145 | 3011..3322 | 4333 |
| UTERU3002731 | 2146 | 161.. 472 | 4334 |
| UTERU3002768 | 2147 | 98.. 445 | 4335 |
| UTERU3002786 | 2148 | 1292..2065 | 4336 |
| UTERU3002993 | 2149 | 4072..4425 | 4337 |
| UTERU3003116 | 2150 | 3428..4126 | 4338 |
| UTERU3003135 | 2151 | 3370..3795 | 4339 |
| UTERU3003178 | 2152 | 1019..2077 | 4340 |
| UTERU3003465 | 2153 | 2851..3189 | 4341 |
| UTERU3003523 | 2154 | 176.. 3937 | 4342 |
| UTERU3003776 | 2155 | 711..1025 | 4343 |
| UTERU3004523 | 2156 | 1109..3427 | 4344 |
| UTERU3004616 | 2157 | 2480..3187 | 4345 |
| UTERU3004709 | 2158 | 718..1095 | 4346 |
| UTERU3004992 | 2159 | 2565..3077 | 4347 |
| UTERU3005049 | 2160 | 1561..2505 | 4348 |
| UTERU3005205 | 2161 | 101..1342 | 4349 |
| UTERU3005230 | 2162 | 1038..1445 | 4350 |
| UTERU3005460 | 2163 | 1872..2489 | 4351 |
| UTERU3005585 | 2164 | 1145..2395 | 4352 |
| UTERU3005907 | 2165 | 819..1709 | 4353 |
| UTERU3005970 | 2166 | 1.. 393 | 4354 |
| UTERU3006008 | 2167 | 2595..3236 | 4355 |
| UTERU3006308 | 2168 | 1490..2491 | 4356 |
| UTERU3007134 | 2169 | 2990..3532 | 4357 |
| UTERU3007419 | 2170 | 124..3792 | 4358 |
| UTERU3007640 | 2171 | 2823..3248 | 4359 |
| UTERU3007913 | 2172 | 138..1775 | 4360 |
| UTERU3008660 | 2173 | 3524..4030 | 4361 |
| UTERU3008671 | 2174 | 1920..2246 | 4362 |
| UTERU3009259 | 2175 | 2426..2917 | 4363 |
| UTERU3009490 | 2176 | 3200..3556 | 4364 |
| UTERU3009517 | 2177 | 2118..2786 | 4365 |
| UTERU3009690 | 2178 | 2674..2991 | 4366 |
| UTERU3009871 | 2179 | 111..1877 | 4367 |
| UTERU3009979 | 2180 | 1630..3504 | 4368 |
| UTERU3011063 | 2181 | 28..1614 | 4369 |
| UTERU3015086 | 2182 | 3216..3713 | 4370 |
| UTERU3015500 | 2183 | 985..2088 | 4371 |
| UTERU3016789 | 2184 | 553..1956 | 4372 |
| UTERU3018081 | 2185 | 180..3194 | 4373 |
| UTERU3018154 | 2186 | 1534..3186 | 4374 |
| UTERU3018616 | 2187 | 267.. 710 | 4375 |
| UTERU3018711 | 2188 | 3597..4106 | 4376 |
| 3NB692004724 | 4377 | 8..1486 | 4684 |
| ADRGL2000042 | 4378 | 133.. 996 | 4685 |
| ADRGL2000056 | 4379 | 158.. 748 | 4686 |
| BLADE2000579 | 4380 | 816..1703 | 4687 |
| BLADE2006830 | 4381 | 88..1872 | 4688 |
| BRACE2002589 | 4382 | 972..1334 | 4689 |
| BRACE2003609 | 4383 | 510..1895 | 4690 |
| BRACE2009318 | 4384 | 874..1242 | 4691 |
| BRACE2011677 | 4385 | 79.. 450 | 4692 |
| BRACE2029396 | 4386 | 38.. 421 | 4693 |
| BRACE2037299 | 4387 | 1170..1754 | 4694 |
| BRACE2039823 | 4388 | 153.. 866 | 4695 |
| BRACE2039832 | 4389 | 125.. 460 | 4696 |
| BRACE2043105 | 4390 | 96.. 494 | 4697 |
| BRACE3001058 | 4391 | 915..2912 | 4698 |
| BRACE3001113 | 4392 | 236..3196 | 4699 |
| BRACE3003026 | 4393 | 128..1453 | 4700 |
| BRACE3003053 | 4394 | 44..>4212 | 4701 |
| BRACE3005107 | 4395 | 54.. 458 | 4702 |
| BRACE3009127 | 4396 | 151..2382 | 4703 |
| BRACE3010076 | 4397 | 1095..2828 | 4704 |
| BRACE3015829 | 4398 | 220.. 585 | 4705 |
| BRACE3021148 | 4399 | 29.. 637 | 4706 |
| BRALZ2017844 | 4400 | 173..1192 | 4707 |
| BRAMY2019111 | 4401 | 989..1768 | 4708 |
| BRAMY2035070 | 4402 | 287..2455 | 4709 |
| BRAMY2035449 | 4403 | 150..1418 | 4710 |
| BRAMY2035718 | 4404 | 353..1693 | 4711 |
| BRAMY2038516 | 4405 | 369..1835 | 4712 |
| BRAMY2039341 | 4406 | 127..1083 | 4713 |
| BRAMY2040159 | 4407 | 209..2440 | 4714 |
| BRAMY2041434 | 4408 | 125.. 550 | 4715 |
| BRAMY2045471 | 4409 | 148..2295 | 4716 |
| BRAMY3004800 | 4410 | 181..2736 | 4717 |
| BRAWH1000369 | 4411 | 412..1350 | 4718 |
| BRAWH2006207 | 4412 | 88.. 459 | 4719 |
| BRAWH2006395 | 4413 | 70.. 975 | 4720 |
| BRAWH2008993 | 4414 | 756..1061 | 4721 |
| BRAWH2009393 | 4415 | 179.. 529 | 4722 |
| BRAWH2010552 | 4416 | 72.. 572 | 4723 |
| BRAWH3007441 | 4417 | 2034..2531 | 4724 |
| BRAWH3009017 | 4418 | 733.. 1158 | 4725 |
| BRCAN2002473 | 4419 | 80..1060 | 4726 |
| BRCAN2002854 | 4420 | 36.. 842 | 4727 |
| BRCAN2003070 | 4421 | 1937..2254 | 4728 |
| BRCAN2014229 | 4422 | 249..1259 | 4729 |
| BRCOC2019841 | 4423 | 275..1591 | 4730 |
| BRHIP2002722 | 4424 | 411..2234 | 4731 |
| BRHIP2003272 | 4425 | 18.. 464 | 4732 |
| BRHIP2005271 | 4426 | 108..1286 | 4733 |
| BRHIP2005724 | 4427 | 107..1180 | 4734 |
| BRHIP2006617 | 4428 | 1568..2641 | 4735 |
| BRHIP2008389 | 4429 | 128.. 781 | 4736 |
| BRHIP2012360 | 4430 | 74..2800 | 4737 |
| BRHIP2017553 | 4431 | 362..2275 | 4738 |
| BRHIP2026877 | 4432 | 430.. 942 | 4739 |
| BRHIP3000017 | 4433 | 37..1515 | 4740 |
| BRHIP3000240 | 4434 | 290..1537 | 4741 |
| BRHIP3008314 | 4435 | 1622..2005 | 4742 |
| BRHIP3026052 | 4436 | 10..1632 | 4743 |
| BRSTN2013354 | 4437 | 286..1371 | 4744 |
| BRTHA2002133 | 4438 | 1448..1957 | 4745 |
| BRTHA2002702 | 4439 | 269.. 817 | 4746 |
| BRTHA2007060 | 4440 | 106..2979 | 4747 |
| BRTHA2010033 | 4441 | 823..1356 | 4748 |
| BRTHA2011321 | 4442 | 32.. 373 | 4749 |
| BRTHA2013426 | 4443 | 980..1588 | 4750 |
| BRTHA2013610 | 4444 | 71..1609 | 4751 |
| BRTHA2016318 | 4445 | 748..1581 | 4752 |
| BRTHA2017364 | 4446 | 52..1638 | 4753 |
| BRTHA2017972 | 4447 | 92.. 808 | 4754 |
| BRTHA2018011 | 4448 | 6.. 929 | 4755 |
| BRTHA2018443 | 4449 | 23..1774 | 4756 |
| BRTHA3000296 | 4450 | 2006..2521 | 4757 |
| BRTHA3003000 | 4451 | 1170..2510 | 4758 |
| BRTHA3008826 | 4452 | 2082..2402 | 4759 |
| CERVX2002013 | 4453 | 978..1301 | 4760 |
| CTONG1000113 | 4454 | 538..2541 | 4761 |
| CTONG2003348 | 4455 | 363..1745 | 4762 |
| CTONG2004000 | 4456 | 538..1869 | 4763 |
| CTONG2008721 | 4457 | 797..1909 | 4764 |
| CTONG2015596 | 4458 | 351..1490 | 4765 |
| CTONG2015633 | 4459 | 838..1305 | 4766 |
| CTONG2016942 | 4460 | 122..1387 | 4767 |
| CTONG2019822 | 4461 | 194..1681 | 4768 |
| CTONG2020374 | 4462 | 191..3052 | 4769 |
| CTONG2020378 | 4463 | 1693..2688 | 4770 |
| CTONG2020411 | 4464 | 23..3322 | 4771 |
| CTONG2020974 | 4465 | 968..1711 | 4772 |
| CTONG2024031 | 4466 | 256..2373 | 4773 |
| CTONG2028758 | 4467 | 268..>2988 | 4774 |
| CTONG3001501 | 4468 | 233..1471 | 4775 |
| CTONG3002552 | 4469 | 3007..>3950 | 4776 |
| CTONG3003598 | 4470 | 2111..2905 | 4777 |
| CTONG3004550 | 4471 | 310..2868 | 4778 |
| CTONG3004726 | 4472 | 84..2681 | 4779 |
| CTONG3009287 | 4473 | 745..2388 | 4780 |
| DFNES2011192 | 4474 | 209..1540 | 4781 |
| FCBBF1000509 | 4475 | 407..2356 | 4782 |
| FCBBF3010361 | 4476 | 253..1266 | 4783 |
| FCBBF3027854 | 4477 | 435..>2218 | 4784 |
| FEBRA2000790 | 4478 | 483.. 839 | 4785 |
| FEBRA2001990 | 4479 | 57..1532 | 4786 |
| FEBRA2006519 | 4480 | 1794..3041 | 4787 |
| FEBRA2008692 | 4481 | 170..3094 | 4788 |
| FEBRA2014122 | 4482 | 216..2600 | 4789 |
| FEBRA2027609 | 4483 | 91..2169 | 4790 |
| FEBRA2028256 | 4484 | 365..2983 | 4791 |
| FEBRA2028516 | 4485 | 62..3109 | 4792 |
| HCASM2002754 | 4486 | 171..1508 | 4793 |
| HCASM2003018 | 4487 | 115..>2321 | 4794 |
| HCASM2003099 | 4488 | 383..2119 | 4795 |
| HCASM2003357 | 4489 | 165.. 491 | 4796 |
| HCASM2008536 | 4490 | 201.. 629 | 4797 |
| HCASM2009424 | 4491 | 121.. 582 | 4798 |
| HCHON2000508 | 4492 | 59..2422 | 4799 |
| HCHON2000743 | 4493 | 218.. 883 | 4800 |
| HCHON2004858 | 4494 | 154..3285 | 4801 |
| HEART2009680 | 4495 | 63..1331 | 4802 |
| HLUNG2013350 | 4496 | 367..1299 | 4803 |
| HLUNG2015418 | 4497 | 73..2691 | 4804 |
| HLUNG2015548 | 4498 | 6..1598 | 4805 |
| HLUNG2016862 | 4499 | 295.. 699 | 4806 |
| HSYRA2005628 | 4500 | 454..1971 | 4807 |
| IMR322001879 | 4501 | 147.. 467 | 4808 |
| IMR322007078 | 4502 | 747..1979 | 4809 |
| IMR322008651 | 4503 | 145..1167 | 4810 |
| IMR322013396 | 4504 | 142..2238 | 4811 |
| IMR322013731 | 4505 | 81..>1713 | 4812 |
| LIVER2000247 | 4506 | 1187..2191 | 4813 |
| MESAN2001770 | 4507 | 415..1770 | 4814 |
| MESAN2005303 | 4508 | 278..2362 | 4815 |
| MESAN2014412 | 4509 | 1589..3538 | 4816 |
| MESAN2015501 | 4510 | 553..2979 | 4817 |
| NT2RI2005772 | 4511 | 227..2035 | 4818 |
| NT2RI2008952 | 4512 | 187..1683 | 4819 |
| NT2RI2009583 | 4513 | 1127..2587 | 4820 |
| NT2RI2018448 | 4514 | 125..1030 | 4821 |
| NT2RI2027157 | 4515 | 603..2483 | 4822 |
| NT2RI3000174 | 4516 | 18..2501 | 4823 |
| NT2RI3001132 | 4517 | 281..3265 | 4824 |
| NT2RI3002557 | 4518 | 3660..>3975 | 4825 |
| NT2RI3005928 | 4519 | 1784..2194 | 4826 |
| NT2RI3007167 | 4520 | 205.. 957 | 4827 |
| NT2RI3007443 | 4521 | 1024..3270 | 4828 |
| NT2RP7008435 | 4522 | 719..2437 | 4829 |
| NT2RP8000521 | 4523 | 635..1039 | 4830 |
| NTONG2008093 | 4524 | 81.. 635 | 4831 |
| OCBBF2003327 | 4525 | 35..1516 | 4832 |
| OCBBF2005433 | 4526 | 154..2565 | 4833 |
| OCBBF2006987 | 4527 | 447..3125 | 4834 |
| OCBBF2008144 | 4528 | 375..>3049 | 4835 |
| OCBBF2009583 | 4529 | 443..1567 | 4836 |
| OCBBF2011669 | 4530 | 656..3346 | 4837 |
| OCBBF2019684 | 4531 | 161..1555 | 4838 |
| OCBBF2020048 | 4532 | 109..1152 | 4839 |
| OCBBF2024284 | 4533 | 70..3063 | 4840 |
| OCBBF2030116 | 4534 | 445..2574 | 4841 |
| OCBBF2032274 | 4535 | 46..1053 | 4842 |
| OCBBF2034637 | 4536 | 380..1897 | 4843 |
| OCBBF3000167 | 4537 | 139..1365 | 4844 |
| OCBBF3002654 | 4538 | 494..2284 | 4845 |
| OCBBF3003761 | 4539 | 316..2025 | 4846 |
| OCBBF3004972 | 4540 | 2394..3302 | 4847 |
| PERIC2007068 | 4541 | 174..1523 | 4848 |
| PLACE7000333 | 4542 | 1736..2470 | 4849 |
| PLACE7000502 | 4543 | 2490..4577 | 4850 |
| PROST2000452 | 4544 | 1462..2097 | 4851 |
| PROST2009320 | 4545 | 1640..2221 | 4852 |
| PROST2019487 | 4546 | 745..1149 | 4853 |
| PUAEN2006335 | 4547 | 123..1829 | 4854 |
| SKMUS2003194 | 4548 | 70..1317 | 4855 |
| SPLEN2004611 | 4549 | 1058..1651 | 4856 |
| SPLEN2016135 | 4550 | 70.. 699 | 4857 |
| SPLEN2016781 | 4551 | 1665..2444 | 4858 |
| SPLEN2016932 | 4552 | 597..1079 | 4859 |
| SPLEN2030847 | 4553 | 101.. 931 | 4860 |
| SPLEN2033490 | 4554 | 1418..1897 | 4861 |
| SPLEN2036702 | 4555 | 122..2467 | 4862 |
| SPLEN2037319 | 4556 | 20.. 364 | 4863 |
| SPLEN2039311 | 4557 | 343.. 768 | 4864 |
| SPLEN2039379 | 4558 | 4..2199 | 4865 |
| STOMA2003158 | 4559 | 593..1459 | 4866 |
| STOMA2004893 | 4560 | 1137..>1566 | 4867 |
| SYNOV1000256 | 4561 | 2082..2927 | 4868 |
| SYNOV2001660 | 4562 | 917..1510 | 4869 |
| SYNOV2006620 | 4563 | 1036..1692 | 4870 |
| SYNOV2013637 | 4564 | 36..1085 | 4871 |
| SYNOV2021953 | 4565 | 375..1640 | 4872 |
| SYNOV4002744 | 4566 | 469..1302 | 4873 |
| SYNOV4003981 | 4567 | 36..2837 | 4874 |
| SYNOV4005739 | 4568 | 1425..2075 | 4875 |
| SYNOV4005889 | 4569 | 79..2793 | 4876 |
| TBAES2000932 | 4570 | 1943..2437 | 4877 |
| TESOP2000390 | 4571 | 218..1651 | 4878 |
| TESOP2001796 | 4572 | 129..1589 | 4879 |
| TESOP2005199 | 4573 | 108..1586 | 4880 |
| TESOP2006398 | 4574 | 2557..>3253 | 4881 |
| TESOP2006865 | 4575 | 256.. 732 | 4882 |
| TESOP2007384 | 4576 | 168..1058 | 4883 |
| TESTI1000266 | 4577 | 346.. 807 | 4884 |
| TESTI2008901 | 4578 | 432..2120 | 4885 |
| TESTI2015626 | 4579 | 448..1605 | 4886 |
| TESTI2025924 | 4580 | 163..2028 | 4887 |
| TESTI2026647 | 4581 | 753..1556 | 4888 |
| TESTI2029252 | 4582 | 191..2293 | 4889 |
| TESTI2032643 | 4583 | 1810..2334 | 4890 |
| TESTI2034251 | 4584 | 188..>1827 | 4891 |
| TESTI2035981 | 4585 | 814..1155 | 4892 |
| TESTI2036288 | 4586 | 86.. 475 | 4893 |
| TESTI2037830 | 4587 | 911..1228 | 4894 |
| TESTI2039060 | 4588 | 140..2080 | 4895 |
| TESTI2049956 | 4589 | 438..1856 | 4896 |
| TESTI2050780 | 4590 | 70..1470 | 4897 |
| TESTI4000137 | 4591 | 396..2435 | 4898 |
| TESTI4000155 | 4592 | 72..3518 | 4899 |
| TESTI4000183 | 4593 | 258..1160 | 4900 |
| TESTI4000214 | 4594 | 60..3710 | 4901 |
| TESTI4000319 | 4595 | 1609..2916 | 4902 |
| TESTI4001984 | 4596 | 651..1166 | 4903 |
| TESTI4005317 | 4597 | 607..2406 | 4904 |
| TESTI4006473 | 4598 | 166..4188 | 4905 |
| TESTI4008058 | 4599 | 849..2573 | 4906 |
| TESTI4008302 | 4600 | 933..2537 | 4907 |
| TESTI4010382 | 4601 | 160..4062 | 4908 |
| TESTI4011070 | 4602 | 978..>3811 | 4909 |
| TESTI4011072 | 4603 | 255..>3737 | 4910 |
| TESTI4011829 | 4604 | 2254..>4540 | 4911 |
| TESTI4013365 | 4605 | 858..2717 | 4912 |
| TESTI4013602 | 4606 | 3496..3990 | 4913 |
| TESTI4013894 | 4607 | 367..1077 | 4914 |
| TESTI4014801 | 4608 | 1383..2555 | 4915 |
| TESTI4015012 | 4609 | 1699..3558 | 4916 |
| TESTI4015442 | 4610 | 12..3323 | 4917 |
| TESTI4017714 | 4611 | 318..2501 | 4918 |
| TESTI4019657 | 4612 | 1789..2925 | 4919 |
| TESTI4021482 | 4613 | 1.. 507 | 4920 |
| TESTI4024387 | 4614 | 404..1063 | 4921 |
| TESTI4025268 | 4615 | 3..1262 | 4922 |
| TESTI4025494 | 4616 | 282..2381 | 4923 |
| TESTI4025547 | 4617 | 5.. 964 | 4924 |
| TESTI4025865 | 4618 | 45..1421 | 4925 |
| TESTI4026207 | 4619 | 96..2540 | 4926 |
| TESTI4028938 | 4620 | 200..1711 | 4927 |
| TESTI4028958 | 4621 | 1336..2502 | 4928 |
| TESTI4029348 | 4622 | 116.. 613 | 4929 |
| TESTI4029528 | 4623 | 46..2544 | 4930 |
| TESTI4029690 | 4624 | 165.. 917 | 4931 |
| TESTI4031745 | 4625 | 1636..4002 | 4932 |
| TESTI4032090 | 4626 | 198.. 998 | 4933 |
| TESTI4032112 | 4627 | 815..1654 | 4934 |
| TESTI4036767 | 4628 | 62..1039 | 4935 |
| TESTI4038721 | 4629 | 2156..3292 | 4936 |
| TESTI4041086 | 4630 | 1946..3313 | 4937 |
| TESTI4046240 | 4631 | 981..1469 | 4938 |
| THYMU2004139 | 4632 | 407..>2108 | 4939 |
| THYMU2004284 | 4633 | 922..1344 | 4940 |
| THYMU2006001 | 4634 | 230..1663 | 4941 |
| THYMU2028739 | 4635 | 408..1925 | 4942 |
| THYMU2030462 | 4636 | 1324..1725 | 4943 |
| THYMU2031139 | 4637 | 382..1890 | 4944 |
| THYMU2031249 | 4638 | 643..1713 | 4945 |
| THYMU2032976 | 4639 | 102.. 482 | 4946 |
| THYMU2033401 | 4640 | 221.. 646 | 4947 |
| THYMU2034279 | 4641 | 1718..2176 | 4948 |
| THYMU2035078 | 4642 | 396.. 902 | 4949 |
| THYMU2035710 | 4643 | 988..1443 | 4950 |
| THYMU2040925 | 4644 | 179.. 727 | 4951 |
| THYMU3000269 | 4645 | 1966..2742 | 4952 |
| THYMU3000360 | 4646 | 792..1241 | 4953 |
| THYMU3001428 | 4647 | 486..2294 | 4954 |
| TKIDN2008778 | 4648 | 1512..1862 | 4955 |
| TKIDN2012771 | 4649 | 2185..3315 | 4956 |
| TKIDN2018926 | 4650 | 59.. 388 | 4957 |
| TLIVE2001684 | 4651 | 1046..2137 | 4958 |
| TLIVE2002046 | 4652 | 255..1334 | 4959 |
| TLIVE2007607 | 4653 | 220..1746 | 4960 |
| TRACH1000212 | 4654 | 32..3826 | 4961 |
| TRACH2000862 | 4655 | 259..2160 | 4962 |
| TRACH2007483 | 4656 | 756..3095 | 4963 |
| TRACH2019672 | 4657 | 289..1350 | 4964 |
| TRACH2024408 | 4658 | 392..>2211 | 4965 |
| TRACH2024559 | 4659 | 1450..1905 | 4966 |
| TRACH3000134 | 4660 | 293..2488 | 4967 |
| TRACH3000420 | 4661 | 17..3577 | 4968 |
| TRACH3002561 | 4662 | 2181..2603 | 4969 |
| TRACH3003683 | 4663 | 1157..1690 | 4970 |
| TRACH3003832 | 4664 | 6..2798 | 4971 |
| TRACH3007866 | 4665 | 183..2450 | 4972 |
| TUTER2000057 | 4666 | 27.. 833 | 4973 |
| UTERU2004299 | 4667 | 452.. 934 | 4974 |
| UTERU2008040 | 4668 | 286..1521 | 4975 |
| UTERU2011220 | 4669 | 453.. 842 | 4976 |
| UTERU2019534 | 4670 | 551..1021 | 4977 |
| UTERU2021820 | 4671 | 1545..2096 | 4978 |
| UTERU2028734 | 4672 | 217..1956 | 4979 |
| UTERU2032279 | 4673 | 1252..2037 | 4980 |
| UTERU2033577 | 4674 | 164..1009 | 4981 |
| UTERU2035978 | 4675 | 56.. 436 | 4982 |
| UTERU3000402 | 4676 | 798..1598 | 4983 |
| UTERU3000738 | 4677 | 792..1547 | 4984 |
| UTERU3001053 | 4678 | 2485..>3535 | 4985 |
| UTERU3014791 | 4679 | 2452..3027 | 4986 |
| UTERU3015069 | 4680 | 2538..3986 | 4987 |
| UTERU3015412 | 4681 | 67..1464 | 4988 |
| UTERU3017176 | 4682 | 3512..>3913 | 4989 |
| TESTI4038779 | 4683 | 202..1971 | 4990 |

Primers used to synthesize polynucleotides can be designed based on the nucleotide sequences of polynucleotides of the present invention, shown in SEQ ID NOs in Table 1 above. When synthesizing full-length cDNAs, an oligo dT primer can be used as the 3'-end primer. The length of the primer is usually 15-100 bp, and favorably between 15-35 bp. In the case of LA PCR, described below, a primer length of 25-35 bp provides a good result.

Methods for designing a primer that enables specific amplification based on a target nucleotide sequence are known to those skilled in the art (Current Protocols in Molecular Biology, Ausubel et al. edit, (1987) John Wiley & Sons, Section 6.1-6.4). In principle, primers based on 5'-end sequences are designed such that amplification products will include the translation start site. Accordingly, for example, when the 5'-end primer is designed based on the nucleotide sequence of the 5' untranslated region (5'UTR), any part of the 5'-end, which ensures specificity to the cDNA of interest, can be selected as the primer.

When synthesizing a full-length cDNA, the target nucleotide sequence to be amplified can extend to several thousand bp in some cDNA. Such long nucleotides can be amplified using methods such as LA PCR (Long and Accurate PCR). The use of LA PCR is advantageous when synthesizing long DNA. In LA PCR, in which a special DNA polymerase having 3' -> 5' exonuclease activity is used, misincorporated nucleotides can be removed. Accordingly, accurate synthesis of the complementary strand can be achieved even with a long nucleotide sequence. By using LA PCR, amplification of nucleotides 20 kb or longer can be achieved under desirable conditions (Takeshi Hayashi (1996) Jikken-Igaku Bessatsu, "Advanced Technologies in PCR" Youdo-sha).

Template DNAs for synthesizing the full-length cDNAs of the present invention can be obtained by using cDNA libraries prepared by various methods. The full-length cDNA clones of the present invention are clones with a high probability of completeness in length, obtained by a method comprising the steps of [1] preparing libraries containing cDNAs with a very high fullness ratio using oligo-capping, and [2] assembling 5'-end sequences and selecting those with the highest probability of completeness in length in clusters formed (there are many clones longer in the 5'-end direction).

However, the use of primers designed based on the full-length nucleotide sequences provided by the present invention enable full-length cDNAs to be easily obtained without using such a special technique.

The problem with commercially available cDNA libraries or those prepared by known methods is that mRNA contained in these libraries has a very low fullness ratio. Thus, it is difficult to screen full-length cDNA clones directly from the library using ordinary cloning methods. The present invention has revealed nucleotide sequences of novel full-length cDNA. If such a full-length nucleotide sequence is provided, it is possible to synthesize a target full-length cDNA by using enzymatic reactions such as PCR. In particular, a full-length-enriched cDNA library, synthesized by methods such as oligo-capping, is desirable to more reliably synthesize a full-length cDNA.

The present invention provides isolated polynucleotides comprising the nucleotide sequences of SEQ ID NO: 1 as shown in Table 1, or homologs thereof. As used herein, an "isolated polynucleotide" is a polynucleotide whose structure is not identical to that of any naturally occurring polynucleotide or to that of any fragment of a naturally occurring genomic polynucleotide spanning more than three separate genes. The term therefore includes, for example, (a) a DNA which comprises the sequence of part of a naturally occurring genomic DNA molecule in the genome of the organism in which it naturally occurs; (b) a polynucleotide incorporated into a vector or into the genomic DNA of a prokaryote or eukaryote such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion polypeptide. Specifically excluded from this definition are polynucleotides of DNA molecules present in mixtures of different (i) DNA molecules, (ii) transfected cells, or (iii) cell clones; e.g., as these occur in DNA libraries such as cDNA or genomic DNA libraries.

The 5'-end sequence of the full-length cDNA clones of this invention can be used to isolate the regulatory elements of transcription, including the promoter on the genome. A rough draft of the human genome (an analysis of the human genomic sequence with lower accuracy), which covers 90% of the genome, has been reported (Nature, Vol.409, 814-823, 2001), and by the year 2003, analysis of the entire human genomic sequence will be finished. However, using software to analyze transcription start sites on the human genome, in which long introns exist, is difficult. In contrast, it is easy to specify transcription start sites in the genomic sequence using nucleotide sequences which include the 5'-end of the full-length cDNA clones of the present invention, and thus it is easy to obtain genomic regions involved in transcription regulation, which include promoters contained upstream of the transcription start site.

The polypeptides encoded by the full-length cDNAs of the invention can be prepared as recombinant polypeptides or as natural polypeptides. For example, a recombinant polypeptide can be prepared by inserting a polynucleotide encoding a polypeptide of the present invention into a vector, introducing the vector into an appropriate host cell, and purifying the polypeptide expressed within that transformed host cell, as described below. In contrast, a natural polypeptide can be prepared, for example, by utilizing an affinity column to which is attached an antibody against a polypeptide of the present invention (Current Protocols in Molecular Biology (1987) Ausubel et al. edit, John Wiley & Sons, Section 16.1-16.19). The antibody used for affinity purification may be either a polyclonal antibody, or a monoclonal antibody. Alternatively, in vitro translation (see, for example, "On the fidelity of mRNA translation in the nuclease-treated rabbit reticulocyte lysate system." Dasso M.C., and Jackson R.J. (1989) Nucleic Acids Res. 17: 3129-3144) may be used for preparing a polypeptide of the invention.

The present invention provides substantially pure proteins encoded by the full-length cDNAs of the present invention. The term "substantially pure" herein used in reference to a given protein or polypeptide means that the protein or polypeptide is substantially free from other biological macromolecules. For example, the substantially pure protein or polypeptide is at least 75%, 80%, 85%, 95%, or 99% pure by dry weight. Purity can be measured by any appropriate standard method known in the art, for example, by column chromatography, polyacrylamide gel electrophoresis, or HPLC analysis.

Polypeptides functionally equivalent to the polypeptides of the present invention can be prepared based on the activities of the polypeptides of the present invention, clarified in the above-mentioned manner. Whether or not a particular polypeptide is functionally equivalent to a polypeptide of the present invention can be verified by using the biological activity of the polypeptide of the present invention as an index to examine whether that polypeptide has the said activity.

Polypeptides functionally equivalent to polypeptides of the present invention can be prepared by those skilled in the art, for example, by using a method for introducing mutations into a polypeptide amino acid sequence (for example, site-directed mutagenesis (Current Protocols in Molecular Biology, edit, Ausubel et al., (1987) John Wiley & Sons, Section 8.1-8.5). Such polypeptides can also be generated by spontaneous mutations. The present invention also includes polypeptides comprising the amino acid sequences shown in Table 1 in which one or more amino acids are substituted, deleted, inserted, and/or added, as long as the polypeptides have a function equivalent to that of a polypeptide identified in the Examples of the present invention, described later.

There are no limitations as to the number and site of amino acid mutation, as long as polypeptide function is maintained. The number of mutations typically corresponds to 30% or less, or 20% or less, or 10% or less, preferably 5% or less or 3% or less of the total amino acids, more preferably 2% or less or 1% or less of the total amino acids. Alternatively, herein, substitution of one or more amino acids includes substitution of several amino acids. As used herein, the term "several amino acids" means, for example, five amino acids, preferably four or three amino acids, more preferably two amino acids, and further preferably one amino acid.

Herein "conservative amino acid substitution" refers to substitution of an amino acid residue belonging to a group comprising a chemically similar side chain, with another amino acid in the same group. Groups of amino acid residues having similar side chains have been defined in the art. These groups include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

In addition, polypeptides functionally equivalent to the polypeptides of the present invention can be isolated by using techniques of hybridization or gene amplification known to those skilled in the art. Specifically, using hybridization (Current Protocols in Molecular Biology, edit, Ausubel et al., (1987) John Wiley & Sons, Section 6.3-6.4)), those skilled in the art can usually isolate a polynucleotide highly homologous to a polynucleotide encoding the polypeptide identified in the present Example, based on the identified nucleotide sequence (Table 1) or a portion thereof, and obtain a functionally equivalent polypeptide from the isolated polynucleotide. The present invention includes polypeptides encoded by polynucleotides which hybridize with the polynucleotides encoding the polypeptides identified in the present Example, as long as the polypeptides are functionally equivalent to those identified in the present Example. Organisms from which the functionally equivalent polypeptides are isolated include but are not limited to vertebrates such as humans, mice, rats, rabbits, pigs and cows.

Washing conditions for hybridization for polynucleotide isolation encoding functionally equivalent polypeptides are usually "1x SSC, 0.1% SDS, 37°C"; more stringent conditions are "0.5x SSC, 0.1% SDS, 42°C"; and still more stringent conditions are "0.1x SSC, 0.1% SDS, 65°C". Alternatively, the following conditions can be given as hybridization conditions of the present invention. Namely, conditions in which hybridization is performed at "6x SSC, 40% Formamide, 25°C", and washing at "1x SSC, 55°C" can be given. More preferable conditions are those in which hybridization is performed at "6x SSC, 40% Formamide, 37°C", and washing at "0.2x SSC, 55°C". Even more preferable are those in which hybridization is performed at "6x SSC, 50% Formamide, 37°C", and washing at "0.1x SSC, 62°C". The more stringent the hybridization conditions, the more frequently polynucleotides highly homologous to the probe sequence are isolated. Therefore, it is preferable to conduct hybridization under stringent conditions. Examples of stringent conditions in the present invention are, washing conditions of "0.5x SSC, 0.1% SDS, 42°C", or alternatively, hybridization conditions of "6x SSC, 40% Formamide, 37°C", and washing at "0.2x SSC, 55°C".

One skilled in the art can suitably select various conditions, such as SSC dilution ratio, formamide concentration, and temperature, to accomplish a similar stringency.

The above-mentioned combinations of SSC, SDS, and temperature conditions are indicated just as examples. Those skilled in the art can select hybridization conditions with similar stringency to those mentioned above by properly combining the above-mentioned or other factors that determine hybridization stringency (for example, probe concentration, probe length, and duration of hybridization reaction).

The amino acid sequences of polypeptides isolated by using hybridization techniques usually have high identity to those of the polypeptides of the present invention, shown in Table 1. The present invention encompasses polynucleotides comprising a nucleotide sequence with high identity to the nucleotide sequence of claim 1 (a). Furthermore, the present invention encompasses peptides, or polypeptides comprising an amino acid sequence with high identity to the amino acid sequence encoded by the polynucleotide of claim 1 (b). The term "high identity" indicates sequence identity of at least 40% or more; preferably 60% or more; and more preferably 70% or more. Even more preferable is identity of 90% or more, 93% or more, or 95% or more. Further more preferable is 97% or more, or 99% or more. Identity can be determined using the BLAST search algorithm.

As used herein, "percent identity" of amino acid sequences or nucleic acids is determined using the BLAST algorithm of Karlin and Altschul (Proc. Natl. Acad. Sci. USA 90:5873-5877, 1993). Such an algorithm is incorporated into the BLASTN and BLASTX programs of Altschul et al. (J. Mol. Biol.215:403-410, 1990). BLAST nucleotide searches are performed with the BLASTN program, using for example, score = 100, wordlength = 12. BLAST protein searches are performed with the BLASTX program, using for example, score = 50, wordlength = 3. When utilizing the BLAST and Gapped BLAST programs, the default parameters of each program are used. See http://www.ncbi.nlm.nih.gov.

Using the gene amplification technique (PCR) (Current Protocols in Molecular Biology, edit, Ausubel et al., (1987) John Wiley & Sons, Section 6.1-6.4)) and primers designed based on the nucleotide sequences (Table 1) or portions thereof as identified in the present Example, it is possible to isolate polynucleotide fragments highly homologous to the polynucleotide sequences or portions thereof, and to obtain polypeptides functionally equivalent to a particular polypeptide identified in the present Example, based on the isolated polynucleotide fragment.

The present invention also provides polynucleotides containing at least 15 nucleotides complementary to a polynucleotide comprising a nucleotide sequence of SEQ ID NOs shown in Table 1, or the complementary strand thereof. Herein, the term "complementary strand" is defined as the other strand to one strand of a double stranded DNA composed of A:T and G:C base pairs. In addition, "complementary" is not only defined as sequences completely matching a continuous region of at least 15 nucleotides, but is also defined to include sequences comprising identity of at least 70%, favorably 80% or higher, more favorably 90% or higher, and most favorably 95% or higher within that region. Identity may be determined using the algorithm described herein.

Such polynucleotides includes probes and primers used for the detection and amplification of polynucleotides encoding the inventive polypeptides. When used as a primer, such a polynucleotide usually comprises 15 to 100 bp, and preferably of 15 to 35 bp. When used as a probe, such a polynucleotide comprises the whole or a part of the sequence of a polynucleotide of the present invention, and comprises at least 15 bp. When used as a primer, such a polynucleotide is complementary at the 3'-end, and restriction enzyme recognition sequences or tags can be added to the 5'-end.

Furthermore, polynucleotides of the present invention include antisense polynucleotides for suppressing the expression of a polypeptide of the present invention, which comprises an amino acid sequence of SEQ ID NOs shown in Table 1. To exert an antisense effect, an antisense polynucleotide has at least 15 bp or more, for example 50 bp or more, preferably 100 bp or more, and more preferably 500 bp or more, and usually has 3000 bp or less, and preferably 2000 bp or less. Antisense polynucleotides can be used in gene therapy of diseases caused by abnormalities of the polypeptides of the invention (abnormal function or abnormal expression). An antisense polynucleotide can be prepared, for example, using the phosphorothioate method ("Physicochemical properties of phosphorothioate oligodeoxynucleotides." Stein (1988) Nucleic Acids Res. 16: 3209-3221) based on the sequence information of polynucleotides encoding a polypeptide of this invention (for example, the nucleotide sequences of SEQ ID NOs: 1 to 2188 and SEQ ID NOs: 4377 to 4683).

The present invention also includes polynucleotides that can use ribozyme or RNA interference (RNAi) activity to downregulate expression of a polynucleotide of the present invention, where such a polynucleotide can be designed based on the nucleotide sequence of the polynucleotide of the present invention.

A ribozyme is a polynucleotide that comprises 1) an antisense sequence of a polynucleotide of the present invention, and 2) the nucleotide sequence of a catalytic unit required for catalytic action. The antisense sequence constituting the ribozyme can be appropriately selected to be compatible with the structure of the ribozyme's catalytic unit. The ribozome's catalytic unit is well known in the art. For example, the hammer-head ribozyme (Rossi et al. (1991) Pharmac. Ther. 50: 245-254) and hairpin ribozyme (Hampel et al. (1990) Nucl. Acids Res. 18: 299-304, and U.S. Pat. No. 5,254,678) are known to have nucleotide sequence-specific cleaving activity. These ribozymes use this catalytic activity to cleave, at a specific position, polynucleotides which hybridize to the antisense sequence.

For example, the autolytic domain of a hammer-head ribozyme cleaves on the 3' side of C15 in the sequence G13U14C15. Base pairing between U14 and A9 plays an important role in this activity, and A15 or U15 can be cleaved instead of C15 (Koizumi M, et al: FEBS Lett 228: 228, 1988). A restriction enzyme-like RNA-cleaving ribozyme that recognizes the target RNA sequences UC, UU, or UA can be produced by designing the ribozyme such that the substrate binding site complements the RNA sequence near the target site (Koizumi, M. et al., FEBS Lett, 239:285, 1988; Koizumi, M. and Otsuka, E., Protein, Nucleic acid, and Enzyme, 35:2191, 1990; Koizumi, M. et al., Nucl Acids Res, 17:7059, 1989). For example, the polynucleotides of the present invention (SEQ ID NOs: 1-2188 and SEQ ID NOs: 4377-4683) contain a number of potential target sites. The polynucleotides of the present invention can be cleaved at desired positions with a ribozyme which contains an appropriately selected antisense sequence.

The ribozyme preferably comprises RNA and may be synthesized chemically or produced by enzymatic reaction. Methods of chemical synthesizing RNA are known in the art. Alternatively, the ribozyme can be produced by using RNA polymerase to transcribe a polynucleotide encoding the ribozyme. To produce a ribozyme by transcription, a polynucleotide encoding the ribozyme is arranged downstream of a promotor recognized by an RNA polymerase. Such RNA polymerases include T7 RNA polymerase and SP6 RNA polymerase. Alternatively, a ribozyme can be expressed in a host cell by inserting a polynucleotide encoding the ribozyme into an appropriate vector, and then introducing the vector into a host cell. The vector contains a promotor that can direct the expression of the gene in the host cell.

The present invention also provides siRNA (small interfering RNA) that downregulates the expression of a polynucleotide of the present invention. siRNA is a technique for controlling gene expression that inhibits protein synthesis from an mRNA by using a double-stranded RNA which comprises the same nucleotide sequence as that mRNA (Fire et al. (1998) Nature 391: 806-811). The effect of downregulating gene expression using double-stranded RNA is called "the RNAi effect". siRNA has been reported to effectively control gene expression in mice (Zamore et al. (2000) Cell 101:25-33; Gura (2000) Nature 404: 804-808). Thus the introduction of such a double-stranded RNA into cells can result in selective downregulation of target gene expression.

There is no limitation as to the length of the siRNAs. The double-stranded RNAs introduced into cells are enzymatically digested inside these cells, starting from their original 3' end and forming fragments of 21 bp to 23 bp. The enzyme that digests the double-stranded RNA is called 'dicer'. The resulting double-stranded RNA fragments recognize and bind to target nucleotide sequences which comprise the same sequence. The nucleotide sequence is then cleaved by the activity of RNase III-like nuclease (Hammond et al. (2000) Nature, 404: 293-298; Zamore et al. (2000) Cell 101: 25-33).

siRNA is introduced into cells to downregulate gene expression using RNAi activity. siRNA can be introduced into cells using the same methods as for ribozymes. Specifically, chemically-synthesized, double-stranded RNA can be introduced into cells. When synthesized RNA, including antisense RNA and siRNA, is intended for introduction into cells, it can be premodified to prevent degradation by nuclease. For example, thiolated RNA is protected from nuclease degradation.

Alternatively, siRNA can be expressed in cells. For example, siRNA can be expressed in cells by inserting a sense sequence and its corresponding antisense sequence into a vector, and then transforming cells with that vector. When the two strands are adjacent, the expressed double-stranded RNA will have a hairpin-loop structure. When the two strands are expressed under the control of different promotors, the resulting double-stranded RNA will comprise two separate strands. Promotors generally used for the expression of siRNA include the U6 promotor.

The nucleotide sequence of an antisense polynucleotide, ribozyme, or siRNA of the present invention may be completely identical or complementary to any one of the nucleotide sequences of SEQ ID NOs: 1-2188 and SEQ ID NOs: 4377-4683, or may have high homology to these nucleotide sequences. The phrase "high homology" to an antisense polynucleotide, ribozyme, or siRNA nucleotide sequence typically means 90% or higher homology, preferably 95% or higher homology, more preferably 98% or higher homology, and still more preferably 99% or higher homology. The homology of a nucleotide sequence can be estimated, for example, by a method described herein.

One skilled in the art can design siRNA based on the nucleotide sequence of a gene whose expression is to be downregulated. The typical methods for designing siRNA include, for example, those described below. To begin with, it is advantageous to avoid using as target sequences: 1) 5'- or 3'-untranslated regions, and 2) regions adjacent to the start codon.

These regions often serve as binding regions for transcriptional regulatory proteins. In addition, these regions may also contain nucleotide sequences conserved among various mRNAs, and thus they may act to inhibit the expression of genes other than the gene of interest.

Thus, it may be advantageous to arrange the target sequence, for example, within the ORF downstream of the start codon. It is preferable to adjust the number of nucleotides between the start codon and the target sequence, for example, to 50 nucleotides or more. Typically, the nucleotide sequence of an siRNA is designed so that it starts from an aa sequence and comprises 19-21 consecutive nucleotides. A dinucleotide overhang is added to one end of siRNA. The nucleotide sequence of such an overhang may include doublets, dTdT or UU sequences. The GC content of a nucleotide sequence constituting siRNA is preferably about 50%. G and C nucleotide residues are preferably uniformly distributed throughout the siRNA.

The action of siRNA is based on sequence-specific mRNA hybridization. Thus, to achieve downregulation specific to a particular gene, it is essential to make the target nucleotide sequence as specific as possible to that gene. It is thus preferable to use homology searches to confirm that the proposed target nucleotide sequence exhibits negligible homology with other genes. Nucleotide sequence homology can be determined using established algorithms.

As long as an siRNA of the present invention downregulates the expression of a polynucleotide of the present invention, it is not limited to nucleotide sequences obtained using the typical design method described above. For example, even if the target sequence is not specific to the nucleotide sequence of a particular gene, it can specifically downregulate the expression of a gene of interest in cells which do not express genes comprising homologous nucleotide sequences. Furthermore, double-stranded RNA having RNAi activity can be obtained without using the above-described methods typically used to select a target sequence.

The polynucleotides or antisense polynucleotides, ribozymes, and siRNAs of the present invention can be used in, for example, gene therapy. Preferable target diseases may be, for example, cancers or various inflammatory diseases. Such molecules can be used for gene therapy, for example, by administrating them to patients in vivo or ex vivo using viral vectors such as retroviral vectors, adenoviral vectors, and adeno-related viral vectors, or non-viral vectors such as liposomes.

The present invention also includes partial peptides of the polypeptides of the invention. Such a partial peptide comprises a polypeptide generated as a result of removing a signal peptide from a secretory protein. If a polypeptide of the present invention has activity as a receptor or ligand, the partial peptide may function as a competitive inhibitor of the polypeptide, and may bind to the receptor (or ligand). In addition, the present invention includes an antigen peptide for raising antibodies. For the peptides to be specific to a polypeptide of the present invention, the peptides comprise at least seven amino acids, preferably eight amino acids or more, more preferably nine amino acids or more, and even more preferably ten amino acids or more. The peptide can be used to prepare an antibody against or competitive inhibitor of a polypeptide of the present invention, and can also be used to screen for a receptor that binds to the polypeptide of this invention. The partial peptides of this invention can be produced, for example, by genetic engineering methods, known methods for synthesizing peptides, or by digesting a polypeptide of the invention with an appropriate peptidase.

The present invention also relates to a vector into which a polynucleotide of the invention is inserted. Vectors of the present invention are not limited as long as they can contain the inserted polynucleotide stably. For example, if E. coli is used as a host, vectors such as pBluescript vector (Stratagene) are preferred cloning vectors. To produce a polypeptide of the invention, expression vectors are especially useful. Any expression vector can be used as long as it is capable of expressing the polypeptide in vitro, in *E. coli*, in cultured cells, or *in vivo*. For example, pBEST vector (Promega) is preferable for *in vitro* expression, pET vector (Invitrogen) for *E. coli*, pME18S-FL3 vector (GenBank Accession No. AB009864) for cultured cells, and pME18S vector (Mol. Cell. Biol. (1988) 8: 466-472) for *in vivo* expression. To insert a polynucleotide of the present invention, ligation utilizing restriction sites can be performed according to standard methods (Current Protocols in Molecular Biology (1987) Ausubel et al. edit, John Wiley & Sons, Section 11.4-11.11).

The GATEWAY™ system (Invitrogen), which is an expression vector construction system for polypeptide expression, can also be used (Experimental Medicine, Vol. 18, No. 19 (December), p2716-2717, 2000). This system includes two types of site-specific recombinases (BP CLONASE™ and LR CLONASE™) derived from lambda phage and uses BP CLONASE™-specific recombination sites for the Entry Vector, and LR CLONASE™-specific recombination sites for the Destination Vector, which may comprise a tag useful for polypeptide purification. With this system, an expression vector can be obtained by using homologous recombination.

First, a polynucleotide fragment of interest is inserted into the entry vector using the first recombination. Then, a second recombination is allowed to take place between the entry vector, where the polynucleotide fragment of interest has been inserted, and the destination vector. Thus, the expression vector can be prepared rapidly and efficiently. Using the above-mentioned typical restriction enzyme/ligase reaction method, expression vector construction and expression of a polypeptide of interest takes about seven to ten days. However, using the GATEWAY™ system, the polypeptide of interest can be expressed and prepared in only three to four days. Thus, the system ensures a high-throughput functional analysis for expressed polypeptides (http://biotech.nikkeibp.co.jp/netlink/lto/gateway/).

The present invention also relates to a transformant carrying a vector of the present invention. Any cell can be used as a host into which a vector of this invention is inserted, and various kinds of host cells can be used depending on the purpose. For example, COS cells or CHO cells can be used for strong expression of the polypeptide in eukaryotic cells.

Introduction of such a vector into host cells can be performed, for example, by calcium phosphate precipitation, electroporation (Current Protocols in Molecular Biology (1987) Ausubel et al. edit, John Wiley & Sons, Section 9.1-9.9), lipofectamine method (GIBCO-BRL), or microinjection, etc.

Further, a polynucleotide containing at least 15 nucleotides comprising a nucleotide sequence of any one of the polynucleotides comprising the nucleotide sequences of SEQ ID NOs shown in Table 1, or the complementary strand thereof, can be used not only as a primer for synthesizing full-length cDNAs, but also for testing and diagnosing abnormalities of the polypeptide encoded by the full-length cDNA of the present invention. For example, by utilizing polymerase chain reaction (genomic DNA-PCR, or RT-PCR) using a polynucleotide of this invention as a primer, a polynucleotide encoding a polypeptide of the invention can be amplified. It is also possible to obtain the regulatory region of expression in the 5'-upstream by using PCR or hybridization, since the transcription start site within the genomic sequence can be easily specified based on the 5'-end sequence of the full-length cDNA. The obtained genomic region can be used for detection and/or diagnosis of sequence abnormality using RFLP analysis, SSCP, or sequencing. Where expression of an mRNA of the present invention varies according to a specific disease, analysis of the amount of mRNA expression using a polynucleotide of the present invention as a probe or a primer enables detection and diagnosis of that disease.

The present invention also relates to antibodies that bind to a polypeptide of the present invention. There are no limitations as to the form of the antibodies of this invention. They include polyclonal antibodies, monoclonal antibodies, or portions thereof that can bind to an antigen. They also include antibodies of all classes. Furthermore, special antibodies such as humanized antibodies and chimeric antibodies are also included.

A polyclonal antibody of this invention can be obtained according to the standard method of synthesizing an oligopeptide corresponding to an amino acid sequence, and immunizing rabbits with that peptide (Current Protocols in Molecular Biology (1987) Ausubel et al. edit, John Wiley & Sons, Section 11.12-11.13). A monoclonal antibody of the present invention can be obtained according to the standard method of purifying a polypeptide expressed in E. coli, immunizing mice with that polypeptide, and producing a hybridoma cell by fusing spleen cells and myeloma cells (Current Protocols in Molecular Biology (1987) Ausubel et al. edit, John Wiley & Sons, Section 11.4-11.11).

An antibody binding to a polypeptide of the present invention can be used for purification of the polypeptide of the invention, and also for detection and/or diagnosis of abnormalities of the expression and structure of that polypeptide. Specifically, polypeptides can be extracted, for example, from tissues, blood, or cells, and a polypeptide of this invention can then be detected for the above purpose using Western blotting, immunoprecipitation, or ELISA.

Furthermore, an antibody binding to a polypeptide of the present invention can be utilized for treating a disease associated with that polypeptide. If the antibody is used to treat patients, human antibodies, humanized antibodies, or chimeric antibodies are preferred due to their low antigenicity. Human antibodies can be prepared by immunizing a mouse whose immune system is replaced with that of a human (e.g., see "Functional transplant of megabase human immunoglobulin loci recapitulates human antibody response in mice" Mendez, M.J. et al. (1997) Nat. Genet. 15: 146-156). These humanized antibodies can be prepared by recombination of the hypervariable region of a monoclonal antibody (Methods in Enzymology (1991) 203: 99-121).

The cDNAs (clones) of the present invention include polypeptide sequences encoding proteins whose function can be predicted, such as, for example, secretory and/or membrane proteins, glycoprotein-related proteins, signal transduction-related proteins, transcription-related proteins, disease-related proteins, enzyme and/or metabolism-related proteins, cell division- and/or cell proliferation-related proteins, cytoskeleton-related proteins, nuclear protein and/or RNA synthesis-related proteins, protein synthesis and/or transport-related proteins, cellular defense-related proteins, development and/or differentiation-related proteins, DNA- and/or RNA-binding proteins, ATP- and/or GTP-binding proteins. The results of cDNA homology searches can be used to estimate whether a cDNA sequence comprises the function of an above-described protein. Specifically, the function of a polypeptide encoded by a cDNA of the present invention can be inferred by (a) searching for a known gene or protein which is homologous to the complete or partial nucleotide sequence of the full-length cDNA of the present invention, and (b) comparing the function of the gene and that of the protein encoded by the gene.

Alternatively, the function of a polypeptide encoded by a cDNA of the present invention can be predicted when a signal sequence, transmembrane domain, nuclear translocation signal, glycosylation signal, phosphorylation site, zinc-finger motif, SH3 domain, or such is found in the amino acid sequence. In particular, partial sequence structures such as motif and domain structures are commonly found in a number of proteins, and comprise a minimal functional protein structure. The Pfam database identifies a total of 4,832 types of motifs and domains, including both those whose functions have been clarified and those whose functions remain unclear (http://www.sanger.ac.uk/Software/Pfam/index.shtml) Version 7.7 (the latest version as of December 2002).

A specific example of motif/domain function is shown below. The ITAM motif (immunoreceptor tyrosine-based activation motif) is found in the intracellular region of the T cell receptor which is expressed on the cell membrane of T cells participating in an immune response (Flaswinkel, H. et al. Semin Immunol 1995 Feb,7(1):21-7). The ITAM motif has a tandem YXXL structure (tyrosine-arbitrary amino acid-arbitrary amino acid-leucine). On extracellular stimulus by an antigen or antibody, the tyrosine in the motif is phosphorylated by an enzyme (LCK) with a kinase domain. Then, ZAP70 binds to the phosphorylated tyrosine via the SH2 domain, resulting in downstream signal transduction (Bu, J.Y. et al., Proc Natl Acad Sci U S A 1995 May 23, 92(11):5106-10; Neumeister, E.N. et al., Mol Cell Biol 1995 June, 15 (6) :3171-8).

A similar phenomenon has been found in mast cells as well as in T cells (Chen, T. et al., J Biol Chem 1996 Oct 11, 271(41):25308-15). Thus, at the molecular level, such a phenomenon is the first step in the activation of immune cells in immunologic diseases such as allergies, atopic dermatitis, and asthma.

Even in a simple exemplary scheme such as the one described above, there are three major motif/domain structures - ITAM, the SH2 domain, and the protein kinase domain - each of which play an important role. The mechanism of this scheme can be interpreted using these three structures. Thus, collecting, categorizing and elucidating the function of molecules that comprise common motif/domain structures is exceedingly important in understanding the molecular-based mechanisms of various cellular functions, including and in addition to the immune response described herein. Searching for motif/domain structures is highly important as the first step in elucidating the functions of unknown polypeptides. It is also understood that an entire polypeptide structure is comprised by minimal structures such as motifs and domains, thus providing the overall function of an entire polypeptide.

The overall function of a polypeptide in cells can be accurately predicted at the molecular level using data obtained by domain and motif structure analysis. In addition, a fusion polypeptide comprising a partial amino acid sequence and a GFP protein or the like may be prepared, and then introduced into cultured cells. For example, if a polypeptide is localized on the cell membrane, it may function as a receptor or ion channel. Alternatively, if a polypeptide is localized in the nucleus, it can be predicted to serve as a polynucleotide-binding protein or to participate in transcription. Thus, the function of a polypeptide can also be predicted by determining its localization.

The function of a full-length cDNA obtained in the present invention can be predicted by carrying out the above-described analysis using its entire nucleotide sequence and the amino acid sequence it encodes. Even when the full-length cDNA nucleotide sequence is not available, a partial sequence thereof (preferably 300 nucleotides or more) often enables function to be predicted. However, function predicted based on information yielded in a partial sequence homology search will not necessarily be the same as that based on a full-length sequence. Functional prediction based on a full-length nucleotide sequence is obviously preferable.

A more specific method for predicting function involves homology searches of databases such as GenBank, Swiss-Prot, UniGene, nr and RefSeq, using BLAST or FASTA. The functions of polypeptides encoded by the cDNAs of the present invention can be predicted based on hit genes and the function of polypeptides encoded by these genes. Polypeptide functions can be predicted from the amino acid sequences deduced from the structure of the full-length nucleotide sequences. In this way, signal sequences and transmembrane domains can be predicted from amino acid sequences using PSORT [K. Nakai & M. Kanehisa, Genomics, 14: 897-911 (1992)], SOSUI [T. Hirokawa et al., Bioinformatics, 14, 378-379 (1998)] (Mitsui Knowledge Industry Co., Ltd.), MEMSAT [D. T. Jones, W. R. Taylor & J. M. Thornton, Biochemistry, 33, 3038-3049 (1994)], and the like. Alternatively, motifs and domains can be predicted from amino acid sequences by carrying out searches using Pfam, PROSITE (http://www.expasy.ch/prosite/), or such. The above-described procedures facilitate more accurate prediction of polypeptide function.

The databases GenBank, Swiss-Prot, UniGene, nr and RefSeq were searched as described above for homology to the 2,495 full-length clone sequences of the present invention whose full-length nucleotide had been determined (see Example 4 and the results of homology searches). In addition, the amino acid sequences deduced from the full-length nucleotide sequences were analyzed by database searches for signal sequences and transmembrane domains using PSORT and SOSUI (see Example 5). The clones were categorized into the fourteen functional categories shown below, based on 1) the results of annotation-based functional prediction (by referring to keywords in the hit data of Swiss-Prot, or to Definitions and Reference information in the hit data of GenBank, UniGene, nr or RefSeq), 2) the results of PSORT searches for signal sequences using the deduced ORFs and 3) the results of SOSUI searches for transmembrane domains using the deduced ORFs. As a result, 1,229 clones were estimated to encode proteins belonging to the categories described below.
Secretory and/or membrane protein (741 clones)
Glycoprotein-related protein (130 clones)
Signal transduction-related protein (111 clones)
Transcription-related protein (102 clones)
Disease-related protein (426 clones)
Enzyme and/or metabolism-related protein (230 clones)
Cell division- and/or cell proliferation-related protein (52 clones)
Cytoskeleton-related protein (61 clones)
Nuclear protein and/or RNA synthesis-related protein (58 clones) Protein synthesis- and/or transport-related protein (72 clones)
Cellular defense-related protein (seven clones)
Development and/or differentiation-related protein (14 clones)
DNA- and/or RNA-binding protein (129 clones)
ATP- and/or GTP-binding protein (92 clones)

The clones predicted to belong to the category of secretory protein and/or membrane protein are the following 659 clones.
ACTVT2000380, ADIPS2000088, ADRGL2000172, ADRGL2003329, ADRGL2009146, ASTRO2014923, ASTRO3000301, BLADE1000176, BLADE2002073, BLADE2002947, BLADE2004462, BLADE2004670, BLADE2005036, BLADE2008539, BNGH42003570, BRACE1000186, BRACE2005457, BRACE2014306, BRACE2016981, BRACE2029112, BRACE2030884, BRACE2031527, BRACE2031531, BRACE2031899, BRACE2032385, BRACE2036005, BRACE2039249, BRACE2039327, BRACE2040138, BRACE2041200, BRACE2043142, BRACE2043665, BRACE2046295, BRACE3000697, BRACE3001391, BRACE3002298, BRACE3003004, BRACE3003595, BRACE3004058, BRACE3004113, BRACE3004772, BRACE3004843, BRACE3006462, BRACE3008137, BRACE3008384, BRACE3009574, BRACE3009708, BRACE3010397, BRACE3011271, BRACE3011505, BRACE3013740, BRACE3014005, BRACE3014068, BRACE3014807, BRACE3016884, BRACE3018963, BRACE3019084, BRACE3020286, BRACE3020594, BRACE3024662, BRACE3025531, BRACE3025630, BRACE3026008, BRACE3026735, BRACE3027326, BRACE3031838, BRACE3040856, BRALZ2016085, BRAMY2001473, BRAMY2004771, BRAMY200.5052, BRAMY2017528, BRAMY2019300, BRAMY2019963, BRAMY2021498, BRAMY2028856, BRAMY2033003, BRAMY2033116, BRAMY2033594, BRAMY2036396, BRAMY2039872, BRAMY2040592, BRAMY2041542, BRAMY2045036, BRAMY2047420, BRAMY2047751, BRAMY2047765, BRAMY3002312, BRAMY3004224, BRAMY3004919, BRAMY3007206, BRAMY3007609, BRAMY3008505, BRAMY4000095, BRASW1000125, BRAWH1000127, BRAWH2002560, BRAWH2002761, BRAWH2007658, BRAWH2014414, BRAWH2014954, BRAWH2016221, BRAWH2016439, BRAWH2016702, BRAWH3000078, BRAWH3000314, BRAWH3001475, BRAWH3001891, BRAWH3002600, BRAWH3003555, BRAWH3003727, BRAWH3003992, BRAWH3004453, BRAWH3004666, BRAWH3005132, BRAWH3005912, BRAWH3006548, BRAWH3007221, BRAWH3007506, BRAWH3007592, BRAWH3008634, BRCAN2002948, BRCAN2006063, BRCAN2009203, BRCAN2010376, BRCAN2012355, BRCAN2012481, BRCAN2013655, BRCAN2014143, BRCAN2016619, BRCAN2024451, BRCOC2007034, BRCOC2019934, BRHIP2000691, BRHIP2001805, BRHIP2002172, BRHIP2004814, BRHIP2004883, BRHIP2005236, BRHIP2005752, BRHIP2009414, BRHIP2013699, BRHIP2026288, BRHIP3000526, BRHIP3007483, BRHIP3007586, BRHIP3008598, BRHIP3009448, BRHIP3015751, BRHIP3024118, BRHIP3026097, BRSSN2003086, BRSSN2004496, BRSSN2008549, BRSSN2011738, BRSSN2014424, BRSSN2018925, BRSTN2000872, BRSTN2003835, BRSTN2007000, BRSTN2010363, BRSTN2012380, BRSTN2015015, BRSTN2016470, BRSTN2016678, BRSTN2017110, BRTHA2002376, BRTHA2002493, BRTHA2002608, BRTHA2002808, BRTHA2003110, BRTHA2003461, BRTHA2005579, BRTHA2006075, BRTHA2008527, BRTHA2011194, BRTHA2012980, BRTHA2013460, BRTHA2015696, BRTHA2015878, BRTHA2016215, BRTHA2016496, BRTHA2017985, BRTHA2018344, BRTHA2018624, BRTHA3000633, BRTHA3002427, BRTHA3003474, BRTHA3007148, BRTHA3008386, BRTHA3008778, BRTHA3009037, BRTHA3009090, BRTHA3009291, BRTHA3016845, BRTHA3017047, BRTHA3017589, BRTHA3017848, BRTHA3018656, CERVX2002006, COLON2000568, COLON2002443, COLON2004478, COLON2005126, COLON2005772, CTONG1000302, CTONG1000341, CTONG1000488, CTONG1000508, CTONG2000042, CTONG2004062, CTONG2008233, CTONG2009423, CTONG2009531, CTONG2010803, CTONG2013178, CTONG2019652, CTONG2019788, CTONG2020127, CTONG2020522, CTONG2020638, CTONG2022601, CTONG2023512, CTONG2024749, CTONG2025496, CTONG2026920, CTONG2027327, CTONG2028124, CTONG2028687, CTONG3000707, CTONG3001370, CTONG3001560, CTONG3002020, CTONG3003179, CTONG3003483, CTONG3003737, CTONG3005648, CTONG3008252, CTONG3008258, CTONG3008496, CTONG3008566, CTONG3008951, CTONG3009227, CTONG3009239, CTONG3009328, CTONG3009385, D3OST2002182, D3OST2002648, DFNES1000107, DFNES2000146, DFNES2005266, DFNES2010502, FCBBF2001183, FCBBF2007510, FCBBF3003435, FCBBF3004502, FCBBF3009888, FCBBF3012170, FCBBF3021576, FCBBF3023895, FCBBF4000076, FEBRA1000030, FEBRA2007708, FEBRA2008311, FEBRA2008468, FEBRA2020668, FEBRA2025427, FEBRA2027082, HCASM2002502, HCASM2003212, HCASM2007047, HCHON2000212, HCHON2001084, HCHON2001548, HCHON2001577, HCHON2001712, HCHON2002676, HCHON2004007, HCHON2004776, HCHON2005921, HEART1000010, HEART2001680, HEART2010492, HLUNG2000014, HLUNG2003872, HLUNG2010464, HLUNG2015617, HLUNG2017350, HSYRA2005496, HSYRA2006873, HSYRA2008714, HSYRA2009102, IMR322002110, IMR322006222, KIDNE1000064, KIDNE2000832, KIDNE2000846, KIDNE2006580, KIDNE2010264, KIDNE2011635, KIDNE2012945, KIDNE2013095, LIVER2007415, LYMPB2000083, MESAN2001979, MESAN2012054, MESTC1000042, NHNPC2000606, NHNPC2001223, NOVAR2000136, NOVAR2001108, NT2RI2008724, NT2RI2009855, NT2RI2025909, NT2RI3001263, NT2RI3003095, NT2RI3003382, NT2RI3003409, NT2RI3005403, NT2RI3006171, NT2RI3006673, NT2RI3007065, NT2RI3007543, NT2RI3007978, NT2RP7000359, NT2RP7000466, NT2RP7004027, NT2RP7009030, NT2RP7014005, NTONG2000413, OCBBF2006151, OCBBF2006567, OCBBF2006764, OCBBF2007114, OCBBF2007428, OCBBF2009926, OCBBF2010140, OCBBF2017516, OCBBF2021788, OCBBF2024719, OCBBF2025458, OCBBF2030517, OCBBF2030574, OCBBF2031167, OCBBF2032590, OCBBF2033869, OCBBF2037598, OCBBF2038317, OCBBF3000483, OCBBF3003320, OCBBF3004314, PEBLM2000170, PEBLM2000338, PEBLM2002594, PEBLM2006113, PEBLM2007834, PERIC2001227, PERIC2003452, PERIC2003720, PERIC2004909, PERIC2005347, PERIC2006035, PERIC2007914, PLACE5000171, PLACE5000260, PLACE5000282, PLACE6012574, PLACE6019932, PLACE6020031, PLACE7000514, PLACE7001022, PROST1000184, PROST1000528, PROST1000559, PROST2003428, PROST2018090, PROST2018902, PROST2018922, PUAEN2002489, PUAEN2005588, PUAEN2006701, PUAEN2009174, PUAEN2009795, RECTM2000433, RECTM2001347, SKMUS2000757, SKNMC2002402, SMINT2002743, SMINT2009902, SMINT2011888, SMINT2015787, SPLEN2001599, SPLEN2009548, SPLEN2012889, SPLEN2015158, SPLEN2015267, SPLEN2015679, SPLEN2021701, SPLEN2023733, SPLEN2023791, SPLEN2025491, SPLEN2029522, SPLEN2029683, SPLEN2030335, SPLEN2030479, SPLEN2031125, SPLEN2031424, SPLEN2031547, SPLEN2031724, SPLEN2031780, SPLEN2032813, SPLEN2033098, SPLEN2034021, SPLEN2034781, SPLEN2036326, SPLEN2036821, SPLEN2037722, SPLEN2038180, SPLEN2038345, SPLEN2038407, SPLEN2040222, SPLEN2041304, SPLEN2042598, STOMA2004294, STOMA2008546, SYNOV2005817, SYNOV2012326, SYNOV2014400, SYNOV2016124, SYNOV4002883, SYNOV4003322, SYNOV4004184, SYNOV4004741, SYNOV4004914, SYNOV4006256, SYNOV4007430, SYNOV4007553, SYNOV4007671, SYNOV4008336, SYNOV4008440, TBAES2001258, TCERX2000613, TESOP2001345, TESOP2001865, TESOP2002273, TESOP2002539, TESOP2004114, TESOP2005485, TESOP2005579, TESOP2006041, TESOP2007052, TESOP2007262, TESOP2007636, TESTI1000257, TESTI1000348, TESTI2000644, TESTI2002036, TESTI2002618, TESTI2002928, TESTI2003347, TESTI2005610, TESTI2006648, TESTI2013382, TESTI2024567, TESTI2027019, TESTI2034767, TESTI2034953, TESTI2034997, TESTI2035997, TESTI2036684, TESTI2042450, TESTI2047071, TESTI2048898, TESTI2051767, TESTI2052822, TESTI4000215, TESTI4000724, TESTI4001100, TESTI4001527, TESTI4001561, TESTI4001665, TESTI4001923, TESTI4002552, TESTI4002754, TESTI4005805, TESTI4005961, TESTI4006053, TESTI4006137, TESTI4007064, TESTI4007163, TESTI4007239, TESTI4007382, TESTI4008050, TESTI4008401, TESTI4008429, TESTI4008797, TESTI4009608, TESTI4012448, TESTI4013369, TESTI4013667, TESTI4013830, TESTI4014392, TESTI4016238, TESTI4017575, TESTI4017901, TESTI4018835, TESTI4019566, TESTI4020092, TESTI4020102, TESTI4021478, TESTI4023722, TESTI4024420, TESTI4024874, TESTI4024890, TESTI4025797, TESTI4026456, TESTI4026785, TESTI4027821, TESTI4028062, TESTI4028429, TESTI4028823, TESTI4028880, TESTI4029836, TESTI4030159, TESTI4030505, TESTI4034172, TESTI4035065, TESTI4035649, TESTI4037244, TESTI4041053, TESTI4042711, TESTI4046487, TESTI4046819, THYMU2001053, THYMU2003632, THYMU2003760, THYMU2005003, THYMU2005303, THYMU2005321, THYMU2007658, THYMU2008725, THYMU2009425, THYMU2011548, THYMU2013386, THYMU2014353, THYMU2019210, THYMU2023711, THYMU2027497, THYMU2027695, THYMU2029676, THYMU2030068, THYMU2032035, THYMU2032437, THYMU2032655, THYMU2033079, THYMU2033308, THYMU2033816, THYMU2034314, THYMU2035064, THYMU2036085, THYMU2036459, THYMU2037226, THYMU2037348, THYMU2038772, THYMU2038797, THYMU2039780, THYMU2040412, THYMU2041015, THYMU3000028, THYMU3000036, THYMU3004835, THYMU3005696, THYMU3006168, THYMU3006811, THYMU3007137, THYMU3007368, THYMU3007845, TKIDN2002424, TKIDN2002632, TKIDN2006525, TKIDN2009092, TKIDN2009889, TKIDN2014771, TKIDN2019116, TLIVE2000023, TLIVE2001828, TLIVE2001927, TLIVE2002336, TLIVE2002690, TLIVE2003381, TLIVE2004110, TLIVE2008229, TOVAR2001281, TRACH1000205, TRACH2001549, TRACH2001684, TRACH2006387, TRACH2007059, TRACH2007834, TRACH2008300, TRACH2020525, TRACH2021964, TRACH2022553, TRACH2025535, TRACH2025911, TRACH3000014, TRACH3002064, TRACH3002168, TRACH3002650, TRACH3004786, TRACH3005294, TRACH3005549, TRACH3006149, TRACH3007391, TRACH3008629, TRACH3035199, TRACH3035526, TRACH3036193, TSTOM2000442, TSTOM2000553, TUTER2000916, UTERU1000339, UTERU2004688, UTERU2004929, UTERU2006137, UTERU2006568, UTERU2007444, UTERU2017762, UTERU2020718, UTERU2022020, UTERU2025025, UTERU2025645, UTERU2025891, UTERU2026090, UTERU2026203, UTERU2027591, UTERU2029953, UTERU2031851, UTERU2035323, UTERU2035469, UTERU3000645, UTERU3000899, UTERU3001240, UTERU3001571, UTERU3001585, UTERU3001652, UTERU3001988, UTERU3002209, UTERU3002383, UTERU3002786, UTERU3003116, UTERU3003776, UTERU3006308, UTERU3008671, UTERU3009690, UTERU3009979, UTERU3011063, UTERU3015500, UTERU3016789

The following 82 clones are also predicted to belong to the category of secretory protein and/or membrane protein. BLADE2006830, BRACE2002589, BRACE2009318, BRACE2011677, BRACE2029396, BRACE2039823, BRACE2039832, BRAMY2019111, BRAMY2038516, BRAMY2045471, BRAWH2006395, BRAWH2008993, BRCOC2019841, BRHIP2003272, BRHIP2005271, BRHIP2005724, BRHIP2008389, BRHIP2026877, BRHIP3000240, BRTHA2011321, BRTHA2018011, BRTHA2018443, BRTHA3008826, CTONG2015633, CTONG2016942, CTONG2019822, FEBRA2000790, FEBRA2006519, FEBRA2028256, FEBRA2028516, HCASM2002754, HEART2009680, HLUNG2013350, HLUNG2015418, IMR322013396, LIVER2000247, NT2RI2009583, NT2RI2027157, NT2RP7008435, OCBBF2003327, OCBBF2030116, PLACE7000502, PROST2000452, PROST2019487, SPLEN2016932, SPLEN2037319, SYNOV2001660, SYNOV2013637, SYNOV4003981, SYNOV4005889, TBAES2000932, TESTI2015626, TESTI2029252, TESTI2032643, TESTI2039060, TESTI2050780, TESTI4000137, TESTI4000155, TESTI4006473, TESTI4011070, TESTI4013365, TESTI4013894, TESTI4014801, TESTI4032090, TESTI4041086, THYMU2004284, THYMU2030462, THYMU2033401, THYMU2034279, THYMU2035710, THYMU2040925, TKIDN2008778, TKIDN2012771, TKIDN2018926, TLIVE2007607, TRACH2019672, TRACH3000420, TRACH3003683, UTERU2011220, UTERU2021820, UTERU2032279, UTERU3015069

The clones predicted to belong to the category of glycoprotein-related protein are the following 115 clones.
ADIPS2000088, BNGH42003570, BRACE2005457, BRACE2014306, BRACE2029112, BRACE2039249, BRACE2046295, BRACE3001391, BRACE3011271, BRACE3016884, BRAMY2005052, BRAMY3004919, BRAMY4000095, BRAMY4000277, BRAWH1000127, BRAWH2007658, BRAWH2014414, BRAWH2016221, BRAWH3002600, BRCAN2006063, BRSSN2004496, BRTHA2008527, BRTHA2012980, BRTHA2016496, BRTHA3002427, BRTHA3017848, COLON2000568, COLON2004478, COLON2005772, CTONG1000341, CTONG2000042, CTONG2009423, CTONG2023512, CTONG2024749, CTONG2025496, CTONG3001370, CTONG3003737, D3OST2002648, DFNES2000146, DFNES2005266, FCBBF3012170, FEBRA1000030, FEBRA2008311, FEBRA2008468, HCHON2001712, HEART1000010, HEART2001680, HSYRA2005496, KIDNE2012945, LYMPB2000083, NESOP2001433, NOVAR2000136, NOVAR2001108, NT2RI3006171, NT2RI3006673, NT2RP7004027, OCBBF2033869, PLACE5000171, PROST1000184, PUAEN2009795, SMINT2010076, SMINT2011888, SMINT2015787, SPLEN2015267, SPLEN2021701, SPLEN2030335, SYNOV2005817, SYNOV2014400, SYNOV3000231, SYNOV3000302, TESOP2004114, TESOP2005485, TESTI1000257, TESTI2002036, TESTI2002618, TESTI2024567, TESTI2027019, TESTI4001527, TESTI4007163, TESTI4012406, TESTI4013830, TESTI4020092, TESTI4023546, TESTI4028823, TESTI4028880, TESTI4046819, THYMU2005303, THYMU2008725, THYMU2009425, THYMU2011548, THYMU2019210, THYMU2023711, THYMU2027497, THYMU2027695, THYMU2038797, THYMU3004835, TLIVE2003381, TRACH2006387, TRACH2007059, TRACH2022425, TRACH2022553, TRACH2022649, TRACH3002168, TRACH3008629, TRACH3035526, TSTOM2000442, UTERU2008347, UTERU2025025, UTERU2035469, UTERU3000899, UTERU3001240, UTERU3003116, UTERU3006308, UTERU3008671, UTERU3015500

The following 15 clones are also predicted to belong to the category of glycoprotein-related protein.
BRAMY2019111, BRHIP2026877, BRTHA2018011, FEBRA2028256, HEART2009680, HLUNG2015418, NT2RI2009583, NT2RP7008435, OCBBF2003327, TESTI2032643, TESTI2039060, TESTI4011070, THYMU2035710, TRACH3003683, UTERU2032279

The clones predicted to belong to the category of signal transduction-related protein are the following 80 clones.
BNGH42007788, BRACE2008594, BRACE2030341, BRACE2044286, BRACE3002508, BRACE3003595, BRACE3006872, BRACE3011421, BRACE3015027, BRACE3027326, BRAMY2036567, BRAMY2038904, BRAMY3000213, BRAMY3002803, BRAMY3005091, BRAMY3005932, BRAMY4000095, BRAMY4000229, BRCAN2003703, BRCAN2014602, BRCAN2016619, BRCAN2028355, BRHIP2000819, BRHIP3025161, BRSSN2004719, BRSTN2008418, BRTHA2002281, BRTHA2015406, CTONG2006798, CTONG3000084, CTONG3002412, D3OST3000169, FCBBF3007540, HCASM2001301, HCHON2006250, HCHON2008112, HLUNG2002465, KIDNE2001847, NESOP2001694, NT2NE2003252, NT2RI2005166, NT2RI3007757, NT2RI3008652, NT2RP7005529, NT2RP7009147, NT2RP7013795, NT2RP8000483, OCBBF2004826, OCBBF2007028, OCBBF2022351, OCBBF2030354, OCBBF2037547, PLACE6019385, PLACE7008431, PROST2016462, PROST2018511, PUAEN2009852, SPLEN2036932, SYNOV2021320, TESOP2000801, TESOP2001166, TESTI2005739, TESTI2026505, TESTI2050137, TESTI4011745, TESTI4012505, TESTI4018208, TESTI4028059, THYMU2007060, THYMU2031046, THYMU2032014, THYMU2039305, THYMU3008436, TLIVE2001327, TRACH2009310, TRACH2025535, TRACH3009455, UTERU2025025, UTERU2036089, UTERU3016789

The following 31 clones are also predicted to belong to the category of signal transduction-related protein.
BRAMY3004800, BRAWH3009017, BRHIP2026877, BRTHA2013610, BRTHA2017972, BRTHA3003000, CTONG2020974, FEBRA2001990, FEBRA2008692, NT2RI2005772, NT2RI3007443, NTONG2008093, OCBBF2005433, OCBBF2024284, OCBBF2034637, OCBBF3002654, SPLEN2036702, SPLEN2039379, TESOP2000390, TESTI2025924, TESTI2049956, TESTI4000319, TESTI4019657, TESTI4021482, TESTI4024387, TESTI4025268, TESTI4031745, THYMU2004139, THYMU2031249, UTERU2008040, UTERU3000738

The clones predicted to belong to the category of transcription-related protein are the following 38 clones.
BRACE2030326, BRACE3001002, BRACE3045033, BRHIP3025161, BRSSN2014299, BRTHA2014792, BRTHA3001721, CTONG2025516, FEBRA2007544, FEBRA2007801, HEART1000074, IMR322000127, IMR322000917, NT2NE2006531, NT2RI2006686, NT2RI3009158, OCBBF2020838, OCBBF2036743, PEBLM2002887, SKNMC2007504, SPLEN2012624, TESTI2026505, TESTI2040018, TESTI2044796, TESTI2050987, TESTI4001176, TESTI4007810, TESTI4014175, TESTI4017543, TESTI4026524, TESTI4036909, THYMU2006420, THYMU2037233, THYMU3004866, TRACH3000558, TUTER2000425, UTERU2035328, UTERU3009490

The following 64 clones are also predicted to belong to the category of transcription-related protein.
BRACE2003609, BRACE3001058, BRACE3001113, BRALZ2017844, BRAMY2035070, BRAMY2035449, BRAMY2035718, BRAMY2039341, BRAWH2006207, BRHIP2017553, CERVX2002013, CTONG1000113, CTONG2003348, CTONG2008721, CTONG2020378, CTONG2020411, CTONG2028758, CTONG3004726, DFNES2011192, FCBBF3010361, FEBRA2014122, FEBRA2027609, HCASM2003018, HCHON2004858, HSYRA2005628, MESAN2005303, MESAN2014412, MESAN2015501, NT2RI2008952, NT2RI2018448, NT2RI3001132, OCBBF2008144, OCBBF2009583, OCBBF2011669, OCBBF2019684, OCBBF2032274, OCBBF3000167, SPLEN2004611, SPLEN2016135, SPLEN2016781, SYNOV2021953, SYNOV4002744, TESOP2001796, TESOP2005199, TESOP2006398, TESOP2006865, TESTI2034251, TESTI4000183, TESTI4000214, TESTI4008302, TESTI4015442, TESTI4025494, TESTI4025547, TESTI4028938, TESTI4032112, THYMU2006001, THYMU2028739, TRACH2007483, TRACH3000134, TRACH3003832, TUTER2000057, UTERU2033577, UTERU3001053, TESTI4038779

The clones predicted to belong to the category of disease-related protein are the following 342 clones.
3NB692002806, ADIPS2000088, BLADE2005036, BRACE2005457, BRACE2008594, BRACE2014306, BRACE2016981, BRACE2018762, BRACE2035381, BRACE2038551, BRACE2039249, BRACE2045300, BRACE3000840, BRACE3001002, BRACE3001391, BRACE3001754, BRACE3002508, BRACE3003595, BRACE3004058, BRACE3004150, BRACE3004772, BRACE3008137, BRACE3008384, BRACE3009708, BRACE3010397, BRACE3011271, BRACE3011421, BRACE3014807, BRACE3015027, BRACE3015521, BRACE3018963, BRACE3020594, BRACE3027326, BRALZ2017359, BRAMY2005052, BRAMY2038904, BRAMY2047751, BRAMY3000213, BRAMY3005091, BRAMY3007609, BRAMY4000095, BRAMY4000229, BRAMY4000277, BRAWH2001395, BRAWH2002560, BRAWH2010000, BRAWH2010536, BRAWH2014414, BRAWH3000100, BRAWH3000491, BRAWH3001326, BRAWH3002574, BRAWH3005912, BRAWH3008341, BRCAN2002562, BRCAN2002856, BRCAN2002948, BRCAN2003746, BRCAN2006063, BRCAN2009203, BRCAN2014602, BRCAN2016619, BRCAN2017442, BRCAN2024451, BRCOC2001505, BRCOC2003213, BRHIP2000819, BRHIP2001805, BRHIP2009414, BRHIP2024165, BRHIP2026288, BRHIP3000339, BRHIP3008405, BRHIP3009448, BRHIP3027137, BRHIP3027854, BRSSN2000684, BRSSN2004719, BRSSN2014424, BRSTN2001613, BRSTN2004987, BRSTN2008418, BRTHA2002608, BRTHA2003110, BRTHA2007122, BRTHA2007603, BRTHA2008527, BRTHA2012980, BRTHA2014792, BRTHA3001721, BRTHA3002427, BRTHA3003074, BRTHA3003449, BRTHA3008778, BRTHA3009037, BRTHA3009090, BRTHA3015815, BRTHA3016917, BRTHA3017848, COLON2000568, COLON2002520, CTONG1000341, CTONG2000042, CTONG2009423, CTONG2010803, CTONG2017500, CTONG2023021, CTONG2025496, CTONG2025516, CTONG3000084, CTONG3002412, CTONG3008639, D3OST2002182, D3OST2002648, DFNES2001108, FCBBF3009888, FEBRA2007708, FEBRA2008468, FEBRA2024744, HCASM2001301, HCASM2007737, HCHON2001712, HCHON2002676, HCHON2003532, HCHON2004007, HCHON2004531, HCHON2008112, HCHON2008444, HEART1000010, HEART1000139, HEART2001680, HEART2010495, HLUNG2002465, HSYRA2005496, IMR322000127, IMR322001380, IMR322006495, KIDNE2001847, KIDNE2012945, NESOP2001694, NOVAR2001108, NT2NE2003252, NT2NE2006531, NT2NE2006909, NT2RI2006686, NT2RI2025909, NT2RI3001515, NT2RI3006171, NT2RI3006340, NT2RI3006673, NT2RI3007757, NT2RI3008652, NT2RP7000359, NT2RP7005118, NT2RP7005529, NT2RP7010599, NTONG2000413, OCBBF2006058, OCBBF2020801, OCBBF2021788, OCBBF2031167, OCBBF2033869, OCBBF2036743, OCBBF2037068, OCBBF2037340, OCBBF3003320, PEBLM2000170, PEBLM2002887, PERIC2003720, PERIC2007914, PERIC2008385, PERIC2009086, PLACE5000282, PLACE6019385, PROST1000184, PROST2003428, PROST2016462, PROST2017367, PROST2018090, PROST2018511, PUAEN2002489, PUAEN2009795, SKNMC2007504, SMINT2010076, SPLEN2002467, SPLEN2006122, SPLEN2011422, SPLEN2012624, SPLEN2021701, SPLEN2031547, SPLEN2033098, SPLEN2036326, SPLEN2036821, SPLEN2036932, SYNOV2005817, SYNOV2012326, SYNOV2014400, SYNOV2021320, SYNOV3000231, SYNOV3000302, SYNOV4002883, SYNOV4004741, SYNOV4007360, SYNOV4007521, SYNOV4007553, SYNOV4007671, SYNOV4008440, TBAES2001229, TBAES2001258, TESOP2004114, TESOP2005485, TESOP2009121, TESTI1000257, TESTI1000319, TESTI2000644, TESTI2002618, TESTI2005610, TESTI2024567, TESTI2026505, TESTI2050987, TESTI2051867, TESTI2053399, TESTI2053621, TESTI4000014, TESTI4000079, TESTI4000288, TESTI4000349, TESTI4000724, TESTI4001148, TESTI4001176, TESTI4001527, TESTI4001561, TESTI4002491, TESTI4006420, TESTI4006819, TESTI4007163, TESTI4007778, TESTI4007810, TESTI4008050, TESTI4008429, TESTI4009160, TESTI4009457, TESTI4009881, TESTI4010851, TESTI4011745, TESTI4011956, TESTI4012406, TESTI4012448, TESTI4012505, TESTI4012679, TESTI4013369, TESTI4013924, TESTI4014175, TESTI4016110, TESTI4016822, TESTI4016925, TESTI4017901, TESTI4018835, TESTI4018881, TESTI4018886, TESTI4020092, TESTI4021478, TESTI4022873, TESTI4023546, TESTI4026524, TESTI4027557, TESTI4028059, TESTI4028429, TESTI4028880, TESTI4030069, TESTI4034632, TESTI4034912, TESTI4035063, TESTI4035498, TESTI4036909, TESTI4037156, TESTI4040363, THYMU1000496, THYMU2005303, THYMU2008725, THYMU2019210, THYMU2027497, THYMU2027695, THYMU2027734, THYMU2031046, THYMU2033104, THYMU2035319, THYMU2037233, THYMU2041015, THYMU3001083, THYMU3001234, THYMU3001379, THYMU3003309, THYMU3004835, THYMU3006118, THYMU3007137, THYMU3008436, TKIDN2000701, TKIDN2006852, TLIVE2001327, TRACH2001549, TRACH2007059, TRACH2022425, TRACH2022649, TRACH3000558, TRACH3002168, TRACH3004721, TRACH3004786, TRACH3005549, TRACH3007479, TRACH3008629, TRACH3009455, TRACH3035526, TSTOM2000442, TUTER2000904, UTERU1000337, UTERU2005621, UTERU2007724, UTERU2017762, UTERU2019491, UTERU2019706, UTERU2025025, UTERU2026090, UTERU2027591, UTERU2035328, UTERU3000645, UTERU3000828, UTERU3000899, UTERU3001240, UTERU3001572, UTERU3001585, UTERU3001652, UTERU3003116, UTERU3003135, UTERU3005907, UTERU3007640, UTERU3008671, UTERU3009490, UTERU3009690, UTERU3009979, UTERU3015500, UTERU3016789

The following 84 clones are also predicted to belong to the category of disease-related protein.
BRACE3001113, BRACE3010076, BRAMY2039341, BRAMY3004800, BRAWH3009017, BRCAN2002473, BRCAN2002854, BRCAN2003070, BRHIP2005271, BRHIP2017553, BRHIP2026877, BRHIP3000240, BRHIP3008314, BRHIP3026052, BRSTN2013354, BRTHA2016318, BRTHA2017972, BRTHA3003000, CERVX2002013, CTONG1000113, CTONG2008721, CTONG2020411, CTONG3004550, FCBBF1000509, FEBRA2008692, HCASM2008536, HCHON2004858, HEART2009680, HLUNG2015548, HSYRA2005628, IMR322008651, IMR322013396, MESAN2001770, NT2RI2009583, NT2RI3007443, OCBBF2003327, OCBBF2009583, OCBBF2011669, OCBBF2024284, OCBBF2032274, OCBBF3000167, OCBBF3002654, PLACE7000502, PROST2000452, PROST2009320, SPLEN2004611, STOMA2003158, SYNOV1000256, SYNOV4002744, SYNOV4003981, TBAES2000932, TESOP2000390, TESOP2001796, TESOP2005199, TESTI2015626, TESTI2025924, TESTI2026647, TESTI2039060, TESTI4000183, TESTI4006473, TESTI4011070, TESTI4017714, TESTI4019657, TESTI4021482, TESTI4024387, TESTI4025494, TESTI4025547, TESTI4028938, TESTI4031745, TESTI4032112, THYMU2004284, THYMU2028739, THYMU2031139, THYMU2031249, THYMU2035710, THYMU3000269, TLIVE2001684, TLIVE2002046, TRACH2024408, TRACH3003683, UTERU2021820, UTERU2032279, UTERU2033577, UTERU3000738

In particular, hit data of the following 338 clones for Swiss-Prot, or GenBank, UniGene, nr or RefSeq corresponded to genes or proteins which had been deposited in the Online Mendelian Inheritance in Man (OMIM), which is the human gene and disease database (the OMIM Number is shown in the parenthesis after the Clone Name).
3NB692002806 (261630), ADIPS2000088 (147120), BLADE2005036 (114850), BRACE2005457 (274600;603545;600791), BRACE2008594 (601959), BRACE2014306 (193002), BRACE2016981 (602701), BRACE2018762 (604800), BRACE2035381 (606088), BRACE2038551 (601961),
BRACE2039249 (602273), BRACE2045300 (601442), BRACE3000840 (600355), BRACE3001002 (300236), BRACE3001391 (601313;173900), BRACE3001754 (185641), BRACE3002508 (606417), BRACE3003595 (602941), BRACE3004058 (250800), BRACE3004150 (601035), BRACE3004772 (603143), BRACE3008137 (602187), BRACE3008384 (603264), BRACE3009708 (182340), BRACE3010397 (602187), BRACE3011271 (602187), BRACE3011421 (602187), BRACE3014807 (605784), BRACE3015027 (602187), BRACE3015521 (605888), BRACE3018963 (605744), BRACE3020594 (400023), BRACE3027326 (602187), BRALZ2017359 (604331), BRAMY2005052 (602621), BRAMY2038904 (605671), BRAMY2047751 (602512), BRAMY3000213 (605448), BRAMY3005091 (600286), BRAMY3007609 (300315), BRAMY4000095 (602187), BRAMY4000229 (602159), BRAMY4000277 (602187), BRAWH2001395 (159430), BRAWH2002560 (602865), BRAWH2010000 (602581), BRAWH2010536 (604010), BRAWH2014414 (603006), BRAWH3000100 (601403), BRAWH3000491 (602187), BRAWH3001326 (602187), BRAWH3002574 (602187), BRAWH3005912 (602187), BRAWH3008341 (602187), BRCAN2002562 (602187), BRCAN2002856 (602712), BRCAN2002948 (603534), BRCAN2003746 (311870), BRCAN2006063 (603196;601369), BRCAN2009203 (603143), BRCAN2014602 (601441), BRCAN2016619 (602187), BRCAN2017442 (604455), BRCAN2024451 (602513), BRCOC2001505 (159430), BRCOC2003213 (602187), BRHIP2000819 (605000), BRHIP2001805 (603219), BRHIP2009414 (602187), BRHIP2024165 (604402), BRHIP2026288 (602187), BRHIP3000339 (159430), BRHIP3008405 (602187), BRHIP3009448 (602187), BRHIP3027137 (600249), BRHIP3027854 (601060), BRSSN2000684 (603505), BRSSN2004719 (600560), BRSSN2014424 (606105), BRSTN2001613 (164020), BRSTN2004987 (604733), BRSTN2008418 (602187), BRTHA2002608 (600463), BRTHA2003110 (602187), BRTHA2007122 (106410), BRTHA2007603 (605846), BRTHA2008527 (152790;176410), BRTHA2012980 (300119), BRTHA2014792 (601674), BRTHA3001721 (604902),
BRTHA3002427 (602187), BRTHA3003074 (605367), BRTHA3003449 (160745), BRTHA3008778 (602187), BRTHA3009037 (602187), BRTHA3009090 (603197), BRTHA3015815 (600902), BRTHA3016917 (604137), BRTHA3017848 (603377;212140), COLON2000568 (147000), COLON2002520 (602187), CTONG1000341 (188040), CTONG2000042 (103950), CTONG2009423 (182137), CTONG2010803 (602189), CTONG2023021 (602498), CTONG2025496 (103950), CTONG2025516 (601679), CTONG3000084 (600888), CTONG3002412 (601403), CTONG3008639 (601797), D3OST2002182 (603590), D3OST2002648 (603071), DFNES2001108 (603560), FCBBF3009888 (602470), FEBRA2007708 (126650;214700), FEBRA2008468 (278000), HCASM2001301 (602399), HCASM2007737 (601504), HCHON2001712 (109190) ,
HCHON2002676 (252800), HCHON2003532 (172490), HCHON2004007 (605866), HCHON2004531 (602187), HCHON2008112 (605837), HCHON2008444 (602187), HEART1000010 (602187), HEART1000139 (191045;115195), HEART2001680 (146900), HEART2010495 (157132), HLUNG2002465 (605216), HSYRA2005496 (131195;187300), IMR322000127 (604077), IMR322001380 (605652), IMR322006495 (605607), KIDNE2012945 (600270), NOVAR2001108 (147120), NT2NE2003252 (602913), NT2NE2006531 (602277), NT2NE2006909 (602187),
NT2RI2006686 (602700), NT2RI2025909 (212138), NT2RI3001515 (300362), NT2RI3006171 (114890), NT2RI3006340 (602187), NT2RI3006673 (602187), NT2RI3007757 (605396), NT2RI3008652 (602654), NT2RP7000359 (603271), NT2RP7005118 (603379), NT2RP7005529 (600888), NT2RP7010599 (603684), NTONG2000413 (602262), OCBBF2006058 (604773), OCBBF2020801 (602187), OCBBF2021788 (602597), OCBBF2031167 (603709), OCBBF2033869 (600270), OCBBF2036743 (604075), OCBBF2037068 (602187), OCBBF2037340 (602187), OCBBF3003320 (605868), PEBLM2000170 (602187), PEBLM2002887 (602187), PERIC2003720 (600381), PERIC2007914 (400009), PERIC2008385 (604455), PERIC2009086 (600134;605158), PLACE5000282 (130160), PLACE6019385 (602448), PROST1000184 (192321), PROST2003428 (602187), PROST2016462 (602187), PROST2017367 (600585), PROST2018090 (312610), PROST2018511 (602187), PUAEN2002489 (604658), PUAEN2009795 (601456), SKNMC2007504 (602187), SMINT2010076 (146900), SPLEN2002467 (605652), SPLEN2006122 (604739), SPLEN2011422 (114213), SPLEN2012624 (602187), SPLEN2021701 (142800), SPLEN2031547 (602187), SPLEN2033098 (602746), SPLEN2036326 (602101), SPLEN2036821 (212138), SPLEN2036932 (605577), SYNOV2005817 (123889), SYNOV2012326 (604336), SYNOV2014400 (135820), SYNOV2021320 (602104), SYNOV3000231 (147100), SYNOV3000302 (147100), SYNOV4002883 (602187), SYNOV4004741 (602187), SYNOV4007360 (602187), SYNOV4007521 (605830), SYNOV4007553 (603028), SYNOV4007671 (602187), SYNOV4008440 (602187), TBAES2001229 (602187), TBAES2001258 (142440), TESOP2004114 (601865), TESOP2005485 (147170), TESOP2009121 (117143), TESTI1000257 (138170), TESTI1000319 (602187), TESTI2000644 (601392), TESTI2002618 (601533), TESTI2005610 (601040), TESTI2024567 (601116), TESTI2026505 (305400), TESTI2050987 (605968), TESTI2051867 (180479), TESTI2053399 (605819), TESTI2053621 (600364;602093), TESTI4000014 (602187), TESTI4000079 (603560), TESTI4000288 (602187), TESTI4000349 (604506), TESTI4000724 (603878), TESTI4001148 (602187), TESTI4001176 (601430), TESTI4001527 (602187), TESTI4001561 (602187), TESTI4002491 (602187), TESTI4006420 (605612), TESTI4006819 (602187), TESTI4007163 (602187), TESTI4007778 (602187), TESTI4007810 (600940), TESTI4008050 (602187), TESTI4008429 (602187), TESTI4009160 (602187), TESTI4009457 (606185), TESTI4009881 (602187), TESTI4010851 (602187), TESTI4011745 (602187), TESTI4011956 (602187), TESTI4012406 (602187), TESTI4012448 (185261), TESTI4012505 (602143), TESTI4012679 (601933), TESTI4013369 (602187), TESTI4013924 (602187), TESTI4014175 (602187), TESTI4016110 (602187), TESTI4016822 (601792), TESTI4016925 (602187), TESTI4017901 (104221), TESTI4018835 (602187), TESTI4018881 (605070), TESTI4018886 (602187), TESTI4020092 (156225), TESTI4021478 (605868), TESTI4022873 (602187), TESTI4023546 (602187), TESTI4026524 (603277), TESTI4027557 (602187), TESTI4028059 (232800;171850), TESTI4028429 (602187), TESTI4028880 (138170), TESTI4030069 (604603), TESTI4034632 (606251), TESTI4034912 (602187), TESTI4035063 (602187), TESTI4035498 (602187), TESTI4036909 (602187), TESTI4037156 (606026), TESTI4040363 (185641), THYMU1000496 (603060), THYMU2005303 (186910), THYMU2008725 (176882), THYMU2019210 (142830), THYMU2027497 (182139), THYMU2027695 (147100), THYMU2027734 (145505), THYMU2031046 (604207), THYMU2033104 (605349), THYMU2035319 (604739), THYMU2037233 (605121), THYMU2041015 (602187), THYMU3001083 (602187), THYMU3001234 (602187), THYMU3001379 (602187), THYMU3003309 (300359), THYMU3004835 (602187), THYMU3006118 (603708), THYMU3007137 (602187), THYMU3008436 (602187), TKIDN2000701 (600465), TKIDN2006852 (603602), TLIVE2001327 (601403), TRACH2001549 (603197), TRACH2007059 (602187), TRACH2022425 (146900), TRACH2022649 (147100), TRACH3000558 (600140), TRACH3002168 (155735), TRACH3004721 (602187), TRACH3004786 (602187), TRACH3005549 (602187), TRACH3007479 (602308), TRACH3008629 (600976), TRACH3009455 (171833), TRACH3035526 (147000), TSTOM2000442 (147100), TUTER2000904 (602187), UTERU1000337 (602187), UTERU2005621 (603505), UTERU2007724 (602373), UTERU2017762 (601053), UTERU2019491 (603762), UTERU2019706 (600114), UTERU2025025 (191315;164970;256000), UTERU2026090 (605497), UTERU2027591 (600150),
UTERU2035328 (605409), UTERU3000645 (602909), UTERU3000828 (602187), UTERU3000899 (603062), UTERU3001240 (602187), UTERU3001572 (602187), UTERU3001585 (602187), UTERU3001652 (602715), UTERU3003116 (602187), UTERU3003135 (602187), UTERU3005907 (190196), UTERU3007640 (603215), UTERU3008671 (182120), UTERU3009490 (604585), UTERU3009690 (104221), UTERU3009979 (600441), UTERU3015500 (606667), UTERU3016789 (602104)

Additionally, hit data of the following 84 clones for Swiss-Prot, nr or RefSeq corresponded to genes or proteins which had been deposited in the Online Mendelian Inheritance in Man (OMIM), which is the human gene and disease database (the OMIM Number is shown in the parenthesis after the Clone Name).
BRACE3001113 (603971), BRACE3010076 (142695), BRAMY2039341 (604077), BRAMY3004800 (602187), BRAWH3009017 (602187), BRCAN2002473 (602187), BRCAN2002854 (602895), BRCAN2003070 (605574), BRHIP2005271 (600267), BRHIP2017553 (602187), BRHIP2026877 (600341), BRHIP3000240 (601142), BRHIP3008314 (604480), BRHIP3026052 (601645), BRSTN2013354 (602187), BRTHA2016318 (605442), BRTHA2017972 (602932), BRTHA3003000 (605276), CERVX2002013 (602903), CTONG1000113 (602277), CTONG2008721 (605317), CTONG2020411 (601930), CTONG3004550 (605611), FCBBF1000509 (601933), FEBRA2008692 (604034), HCASM2008536 (194360), HCHON2004858 (602187), HEART2009680 (601970), HLUNG2015548 (146690), HSYRA2005628 (602187), IMR322008651 (179617), IMR322013396 (600053), MESAN2001770 (600495), NT2RI2009583 (605949), NT2RI3007443 (602448), OCBBF2003327 (605008), OCBBF2009583 (602277), OCBBF2011669 (602187), OCBBF2024284 (176981), OCBBF2032274 (603975), OCBBF3000167 (194558), OCBBF3002654 (601893), PLACE7000502 (164951), PROST2000452 (602060), PROST2009320 (605903), SPLEN2004611 (602228), STOMA2003158 (602244), SYNOV1000256 (606021), SYNOV4002744 (602187), SYNOV4003981 (604283), TBAES2000932 (606212), TESOP2000390 (602187), TESOP2001796 (602187), TESOP2005199 (194531), TESTI2015626 (601249), TESTI2025924 (600863), TESTI2026647 (601235), TESTI2039060 (154360), TESTI4000183 (601276), TESTI4006473 (602187), TESTI4011070 (602187), TESTI4017714 (602187), TESTI4019657 (602052), TESTI4021482 (164730), TESTI4024387 (602187), TESTI4025494 (602187), TESTI4025547 (605308), TESTI4028938 (603899), TESTI4031745 (602448), TESTI4032112 (603246), THYMU2004284 (314370), THYMU2028739 (604191), THYMU2031139 (605009), THYMU2031249 (311550), THYMU2035710 (601890), THYMU3000269 (600857), TLIVE2001684 (120700), TLIVE2002046 (125270), TRACH2024408 (106410), TRACH3003683 (150205), UTERU2021820 (126141), UTERU2032279 (600942), UTERU2033577 (603397), UTERU3000738 (602187)

The clones predicted to belong to the category of enzyme and/or metabolism-related protein are the following 171 clones.
3NB692002806, ASTRO2002842, BLADE2005036, BRACE2008594, BRACE2030341, BRACE2035381, BRACE2038551, BRACE2039249, BRACE2041200, BRACE2045772, BRACE3004058, BRACE3009708, BRACE3011421, BRACE3016884, BRACE3024073, BRACE3025630, BRAMY2033267, BRAMY2039872, BRAMY3002803, BRAMY3004919, BRAMY3005091, BRAMY3005932, BRAMY4000095, BRAWH3002574, BRAWH3008341, BRCAN2003703, BRCAN2003746, BRCAN2009432, BRCAN2014602, BRCAN2017442, BRCAN2028355, BRCOC2003213, BRHIP2024165, BRHIP3008405, BRHIP3027137, BRHIP3027854, BRSTN2000872, BRSTN2004863, BRSTN2004987, BRSTN2008418, BRTHA2002608, BRTHA2009311, BRTHA2015406, BRTHA2016496, BRTHA3008778, BRTHA3009090, BRTHA3015815, BRTHA3016917, CTONG2004062, CTONG2006798, CTONG2013178, CTONG2028124, CTONG3009028, D3OST2002182, DFNES2001108, DFNES2005266, FCBBF3013307, FCBBF3023895, FEBRA2008468, FEBRA2026984, HCASM2001301, HCHON2002676, HCHON2003532, HCHON2004007, HEART2006131, HEART2010492, HHDPC1000118, HLUNG2011298, HLUNG2013204, HSYRA2008714, KIDNE2001361, KIDNE2006580, NT2NE2003252, NT2NE2006909, NT2RI2004618, NT2RI2025909, NT2RI3006673, NT2RI3007978, NT2RI3008974, NT2RP7000359, NT2RP7004027, NT2RP7010599, NT2RP7014005, NTONG2000413, NTONG2008672, OCBBF2006005, OCBBF2006058, OCBBF2006151, OCBBF2019823, OCBBF2025527, OCBBF2030354, OCBBF2031167, OCBBF3003320, PEBLM2005183, PERIC2000889, PERIC2008385, PLACE6019385, PLACE7008431, PROST2017367, PUAEN2007044, PUAEN2009655, PUAEN2009852, SKNMC2006998, SKNMC2007504, SMINT1000192, SPLEN2010912, SYNOV2012326, SYNOV4002883, TBAES2001258, TESOP2000801, TESOP2004114, TESTI2005610, TESTI2005739, TESTI2016046, TESTI4000079, TESTI4000209, TESTI4000288, TESTI4000349, TESTI4001176, TESTI4001527, TESTI4001561, TESTI4002552, TESTI4006148, TESTI4006819, TESTI4007810, TESTI4008429, TESTI4010851, TESTI4012406, TESTI4012448, TESTI4013369, TESTI4013817, TESTI4014175, TESTI4016822, TESTI4018152, TESTI4018835, TESTI4019566, TESTI4021478, TESTI4022716, TESTI4023546, TESTI4026510, TESTI4026524, TESTI4028059, TESTI4029836, TESTI4034632, TESTI4036909, TESTI4046819, THYMU2008725, THYMU2027734, THYMU2031046, THYMU2031258, THYMU3001234, THYMU3003212, THYMU3004157, THYMU3004835, THYMU3006118, THYMU3008436, TKIDN2006852, TLIVE2002336, TRACH2001549, TRACH2009310, TRACH3007479, TRACH3036193, UTERU1000337, UTERU2019491, UTERU2025025, UTERU2026203, UTERU3000665, UTERU3001240, UTERU3001585, UTERU3003116, UTERU3005907

The following 59 clones are also predicted to belong to the category of enzyme and/or metabolism-related protein.
BRACE2039823, BRACE3010076, BRAMY2038516, BRAWH1000369, BRCAN2003070, BRHIP2005271, BRHIP2012360, BRHIP2026877, BRHIP3008314, BRTHA2013610, BRTHA2017364, BRTHA2017972, BRTHA2018011, BRTHA2018443, BRTHA3003000, CTONG2016942, FCBBF1000509, FEBRA2008692, HCASM2003099, HLUNG2015548, MESAN2005303, NT2RI3000174, NT2RI3007443, NT2RP7008435, NTONG2008093, OCBBF2003327, OCBBF2034637, OCBBF3002654, PROST2000452, SPLEN2039311, SPLEN2039379, STOMA2003158, TESOP2000390, TESTI2015626, TESTI2025924, TESTI2026647, TESTI2032643, TESTI2036288, TESTI2039060, TESTI4006473, TESTI4011070, TESTI4014801, TESTI4017714, TESTI4019657, TESTI4021482, TESTI4031745, THYMU2004139, THYMU2004284, THYMU2031139, THYMU2031249, THYMU2040925, THYMU3000269, TLIVE2002046, TLIVE2007607, TRACH2024559, TRACH3003683, TRACH3007866, UTERU2021820, UTERU3000738

The clones predicted to belong to the category of cell division and/or cell proliferation-related protein are the following 42 clones.
BLADE2002782, BRACE2042550, BRACE2043248, BRACE3000840, BRALZ2017359, BRAMY2038484, BRAMY2046989, BRAWH2010536, BRAWH2014954, BRAWH3000100, BRHIP2000819, BRHIP2001927, BRHIP2009414, BRSSN2000684, CTONG3002412, CTONG3008258, CTONG3008639, FCBBF3002163, HCASM2001301, IMR322006495, NT2RI2006686, OCBBF2021020, OCBBF2037068, OCBBF3004314, PLACE5000282, PLACE6019385, PLACE7002641, PUAEN2006328, SPLEN2033098, TESOP2009121, TESTI1000545, TESTI2003573, TESTI2005610, TESTI4007810, TESTI4017901, THYMU2034374, THYMU2039315, TLIVE2001327, TRACH2025507, UTERU2005621, UTERU3009690, UTERU3009979

The following ten clones are also predicted to belong to the category of cell division and/or cell proliferation-related protein. BRCAN2003070, BRTHA3003000, NT2RI3007443, PLACE7000502, SPLEN2004611, STOMA2003158, SYNOV4003981, TESTI4031745, THYMU2004139, THYMU2035078

The clones predicted to belong to the category of cytoskeleton-related protein are the following 55 clones.
ASTRO1000009, BLADE2004089, BRACE2026836, BRACE2045300, BRACE3006872, BRAMY3008466, BRAWH2001395, BRAWH2005315, BRAWH3002600, BRCOC2001505, BRHIP2000819, BRHIP3000339, BRHIP3008405, BRTHA2007122, BRTHA3003449, COLON2002520, CORDB2000541, FCBBF3021940, HCHON2001577, HEART1000139, HEART2010495, NT2RI3006340, NT2RP7000359, NTONG2005277, OCBBF2007068, OCBBF3003592, PERIC2000889, PLACE5000282, PROST1000559, SKMUS2006394, SPLEN2011422, SPLEN2015679, TESTI2049857, TESTI4000288, TESTI4001148, TESTI4007778, TESTI4009160, TESTI4009881, TESTI4011956, TESTI4013924, TESTI4016925, TESTI4018886, TESTI4022873, TESTI4034912, TESTI4035063, TESTI4037727, THYMU1000496, THYMU2035735, THYMU3001083, THYMU3001234, TKIDN2000701, UTERU2007724, UTERU2008347, UTERU2035745, UTERU3003178

The following six clones are also predicted to belong to the category of cytoskeleton-related protein.
HLUNG2015418, SPLEN2030847, SPLEN2036702, TESTI4025268, TESTI4026207, TRACH2024408

The clones predicted to belong to the category of nuclear protein and/or RNA synthesis-related protein are the following 42 clones.
BLADE2007958, BRACE2010489, BRACE2045300, BRACE3004150, BRACE3005430, BRACE3011421, BRAMY2046989, BRAMY3005932, BRCAN2002562, BRHIP2021615, BRSTN2001613, BRSTN2004987, COLON2000470, CTONG3009028, FCBBF3013307, HCHON2004531, IMR322006495, OCBBF2020801, PEBLM2005183, PUAEN2007044, SKNMC1000124, SMINT1000192, SPLEN2006122, SPLEN2010912, TESOP2009121, TESTI4009374, TESTI4009457, TESTI4013830, TESTI4019566, TESTI4022716, THYMU2033104, THYMU2035319, THYMU2038301, THYMU2040975, THYMU3001379, TRACH3004721, TRACH3036609, UTERU2026025, UTERU3000828, UTERU3001572, UTERU3003135, UTERU3004992

The following 16 clones are also predicted to belong to the category of nuclear protein and/or RNA synthesis-related protein.
BRACE3010076, BRCAN2002854, BRHIP2006617, BRHIP2012360, BRHIP3026052, BRSTN2013354, BRTHA2017364, HCASM2003099, HCASM2008536, IMR322008651, NT2RI3000174, STOMA2003158, TESTI2026647, TESTI4006473, TESTI4021482, THYMU2035078

The clones predicted to belong to the category of protein synthesis and/or transport-related protein are the following 57 clones.
ASTRO2002842, BLADE2005036, BRACE3025630, BRAMY2033003, BRAMY3007609, BRAWH3000491, BRAWH3002574, BRAWH3008341, BRCAN2002856, BRCAN2002948, BRCOC2003213, BRSTN2004987, BRTHA2016496, BRTHA3013884, BRTHA3016917, CTONG2000042, CTONG2013178, CTONG2023512, CTONG2024749, CTONG2025496, CTONG3001370, DFNES2005266, FEBRA2026984, HCASM2007737, HCHON2008444, HEART1000010, KIDNE2000846, NT2NE2006909, NT2RI2011422, NT2RP7004027, NTONG2000413, OCBBF2031167, TBAES2001229, TBAES2001258, TESTI1000319, TESTI2005610, TESTI2051867, TESTI4000209, TESTI4000349, TESTI4001106, TESTI4002491, TESTI4008050, TESTI4010851, TESTI4012406, TESTI4012448, TESTI4013924, TESTI4028429, TESTI4034912, THYMU2009157, TLIVE2008229, TRACH3007479, TRACH3008713, TRACH3036193, UTERU2019940, UTERU3001988, UTERU3003116, UTERU3007419

The following 15 clones are also predicted to belong to the category of protein synthesis and/or transport-related protein.
BRTHA2007060, BRTHA2018011, CTONG2016942, MESAN2001770, MESAN2005303, NT2RP7008435, OCBBF2003327, PROST2000452, TESOP2001796, TESTI4017714, THYMU2004284, THYMU2031139, TRACH2024559, TRACH3007866, UTERU2021820

The clones predicted to belong to the category of cellular defense-related protein are the following three clones.
BRACE3005430, HCHON2004531, TESTI4007810

The following four clones are also predicted to belong to the category of cellular defense-related protein.
BRHIP2012360, FCBBF3027854, HCASM2008536, UTERU2032279

The clones predicted to belong to the category of development and/or differentiation-related protein are the following nine clones.
BRACE3009747, BRTHA2005579, BRTHA3003343, IMR322000917, PEBLM2000170, TESOP2001122, TESOP2001953, TESTI2040018, UTERU3006308

The following five clones are also predicted to belong to the category of development and/or differentiation-related protein.
BRALZ2017844, CTONG2020378, HCHON2004858, OCBBF2019684, THYMU2006001

The clones predicted to belong to the category of DNA-binding and/or RNA-binding protein are the following 55 clones.
3NB692002685, BLADE2007958, BRACE2030326, BRACE2045596, BRACE3001002, BRACE3004150, BRACE3009747, BRACE3045033, BRCAN2002562, BRHIP2021615, BRSSN2014299, BRSTN2001613, BRSTN2004987, BRTHA2014792, BRTHA3001721, BRTHA3003343, CTONG2025516, CTONG3008831, CTONG3009028, FCBBF3013307, FEBRA2007544, FEBRA2007801, HEART1000074, IMR322000127, IMR322000917, NT2NE2006531, NT2RI3009158, OCBBF2020838, OCBBF2036743, PEBLM2002887, PEBLM2005183, SKNMC2007504, SMINT1000192, SPLEN2006122, TBAES2001229, TESTI2014716, TESTI2040018, TESTI2044796, TESTI4009374, TESTI4012679, TESTI4014175, TESTI4017543, TESTI4026510, TESTI4026524, THYMU2006420, THYMU2035319, THYMU2037233, THYMU2040975, THYMU3004866, TLIVE2008229, TRACH3036609, TUTER2000425, UTERU2026025, UTERU2035328, UTERU3009490

The following 74 clones are also predicted to belong to the category of DNA-binding and/or RNA-binding protein.
BRACE2003609, BRACE3001058, BRACE3001113, BRACE3010076, BRALZ2017844, BRAMY2035070, BRAMY2035449, BRAMY2035718, BRAMY2039341, BRAWH1000369, BRAWH2006207, BRCAN2002854, BRHIP2012360, BRHIP2017553, BRSTN2013354, BRTHA2017364, CERVX2002013, CTONG1000113, CTONG2008721, CTONG2020378, CTONG2020411, CTONG2028758, CTONG3004726, DFNES2011192, FEBRA2014122, FEBRA2027609, HCASM2003018, HCASM2009424, HCHON2004858, HSYRA2005628, IMR322008651, MESAN2001770, MESAN2005303, MESAN2014412, MESAN2015501, NT2RI2008952, NT2RI2018448, NT2RI3000174, NT2RI3001132, OCBBF2008144, OCBBF2009583, OCBBF2011669, OCBBF2019684, OCBBF2032274, OCBBF3000167, SPLEN2004611, SPLEN2016135, SPLEN2016781, SYNOV2021953, SYNOV4002744, TESOP2005199, TESOP2006398, TESOP2006865, TESTI2026647, TESTI2034251, TESTI4000183, TESTI4000214, TESTI4006473, TESTI4008302, TESTI4015442, TESTI4025494, TESTI4025547, TESTI4028938, TESTI4032112, THYMU2006001, THYMU2035078, TRACH2007483, TRACH3000134, TRACH3002561, TRACH3003832, TUTER2000057, UTERU2033577, UTERU3001053, TESTI4038779

The clones predicted to belong to the category of ATP binding and/or GTP-binding protein are the following 68 clones.
BNGH42007788, BRACE2008594, BRACE2047377, BRACE3005430, BRACE3008720, BRACE3009708, BRACE3015521, BRACE3024073, BRAMY4000095, BRCAN2009432, BRCOC2003213, BRHIP3008405, BRSTN2013741, BRTHA3003449, BRTHA3015815, BRTHA3016917, COLON2002520, FEBRA2026984, HCASM2001301, HCHON2004007, HSYRA2008714, KIDNE2001361, KIDNE2001847, NESOP2001694, NT2RI2005166, NT2RP7013795, OCBBF3003320, OCBBF3003592, PEBLM2002594, PERIC2000889, PLACE6019385, SMINT1000192, SPLEN2037194, TESOP2000801, TESTI2006648, TESTI4000288, TESTI4001148, TESTI4001176, TESTI4002552, TESTI4007810, TESTI4008429, TESTI4009160, TESTI4009881, TESTI4011956, TESTI4013817, TESTI4014175, TESTI4016925, TESTI4018208, TESTI4018835, TESTI4019566, TESTI4021478, TESTI4022873, TESTI4026524, TESTI4029836, TESTI4035498, TESTI4036909, TESTI4037727, THYMU1000496, THYMU2033079, THYMU3001083, THYMU3001234, THYMU3001379, TRACH2009310, UTERU2019706, UTERU2025025, UTERU2035745, UTERU3000665, UTERU3000828

The following 24 clones are also predicted to belong to the category of ATP binding and/or GTP-binding protein.
BRHIP2026877, BRTHA2017364, BRTHA2018443, IMR322008651, IMR322013731, NT2RI3007443, NTONG2008093, OCBBF3002654, TESOP2000390, TESOP2007384, TESTI2025924, TESTI2026647, TESTI2049956, TESTI4005317, TESTI4006473, TESTI4021482, TESTI4026207, TESTI4031745, THYMU2004139, THYMU2031249, TRACH2000862, TRACH2024559, TRACH3000420, UTERU3000738

The 119 clones shown below are clones which were unassignable to any of the above-mentioned categories, but have been predicted to have some function based on homology search using their full-length nucleotide sequences. Clone Name and Definition in the result of homology search, demarcated by a double slash mark (//), are shown below.
ADRGL2009691// Mus musculus D11lgp1 mRNA, complete cds.
ADRGL2009755// Homo sapiens brain and reproductive organ-expressed protein (BRE) mRNA, complete
ASTRO3000177// Drosophila melanogaster BcDNA.GH03694 (BcDNA.GH03694) mRNA, complete cds.
BLADE2008398// Homo sapiens LRR FLI-I interacting protein 2 (LRRFIP2) mRNA, complete cds.
BRACE2006319// Homo sapiens mRNA for Fln29, complete cds.
BRACE2027258// Homo sapiens E2a-Pbx1-associated protein (EB-1) mRNA, partial cds.
BRACE2038329// Rattus norvegicus CBL-B (Cbl-b) mRNA, partial cds.
BRACE2046251// Homo sapiens hucep-10 mRNA for cerebral protein-10, complete cds.
BRACE3003192// latent transforming growth factor beta binding protein 3 [Homo sapiens]
BRACE3007625// espin [Rattus norvegicus]
BRACE3009297// mdgl-1 [Mus musculus]
BRACE3015262// espin [Mus musculus]
BRACE3025457// testis-specific protein TSP-NY [Homo sapiens]
BRALZ2016498// Homo sapiens FKSG76 (FKSG76) mRNA, complete cds.
BRAMY2030109// Homo sapiens hucep-4 mRNA for cerebral protein-4, complete cds.
BRAMY2031317// Mus musculus semaphorin cytoplasmic domain-associated protein 3A (Semcap3) mRNA, complete cds.
BRAMY2047746// nasopharyngeal carcinoma susceptibility protein [Homo sapiens]
BRAMY3001794// Rattus norvegicus Circadian Oscillatory Protein (SCOP) (Scop)
BRAWH2001940// H.sapiens gene from PAC 1026E2, partial.
BRAWH2012162// KE03 protein [Homo sapiens]
BRAWH2016724// MAP2=HMW-MAP2 {alternatively spliced} [rats, brain, mRNA Partial, 267 nt].
BRAWH3002821// synaptotagmin-like 2 [Mus musculus]
BRCAN2002944// Mus musculus huntington yeast partner C (Hypc) mRNA, complete cds.
BRCAN2013660// Arabidopsis thaliana putative protein (F4F15.330) mRNA, complete cds.
BRHIP2002122// Homo sapiens B aggressive lymphoma long isoform (BAL) mRNA, complete cds.
BRHIP2003786// CCA3 [Rattus norvegicus]
BRHIP2004359// ELAC PROTEIN.
BRHIP2007616// plexin 2
BRHIP2029393// COBW-like protein [Homo sapiens]
BRHIP3008313// testis specific ankyrin-like protein 1 [Homo sapiens]
BRSSN2013874// TEMO [Rattus norvegicus]
BRSTN2017771// Homo sapiens putative BTK-binding protein mRNA, complete cds.
BRTHA2012392// Homo sapiens HCDI (HCDI) mRNA, complete cds.
BRTHA3002933// uroplakin 3 [Homo sapiens]
BRTHA3008310// Mus musculus mRNA for iroquois homeobox protein 6 (Irx6 gene).
BRTHA3008520// sporulation-induced transcript 4-associated protein; hypothetical protein FLJ11058 [Homo sapiens]
COLON2001721// GLUT4 vesicle protein [Mus musculus]
CTONG1000467// Mus musculus mRNA for Deltex3, complete cds.
CTONG2020026// Drosophila melanogaster BcDNA.GH09358 (BcDNA.GH09358) mRNA, complete cds.
CTONG3001123// Mus musculus Pax transcription activation domain interacting protein PTIP mRNA, complete cds.
CTONG3002127// granuphilin [Mus musculus]
CTONG3004072// GL002 protein [Homo sapiens]
CTONG3006186// syntaxin binding protein 4 [Mus musculus]
CTONG3008894// Mus musculus SH3-domain binding protein 5
FCBBF1000297// Human protein immuno-reactive with anti-PTH polyclonal antibodies mRNA, partial cds.
HCHON2000028// Homo sapiens 7h3 protein mRNA, partial cds.
HCHON2000626// X-linked protein STS1769.
HCHON2001217// Homo sapiens cullin CUL4B (CUL4B) mRNA, complete cds.
HEART2006909// Hemolysin C.
HLUNG2011041// basic proline-rich peptide IB-8a - human (fragments)
HLUNG2014288// Mus musculus RP42 mRNA, complete cds.
IMR322006886// Homo sapiens hepatocellular carcinoma-associated antigen 127 (HCA127) mRNA, complete cds.
KIDNE2002252// Drosophila melanogaster BcDNA.GH09358 (BcDNA.GH09358) mRNA, complete cds.
KIDNE2011532// similar to melanoma-associated chondroitin sulfate proteoglycan 4
NT2RI2012990// 76.5 KDA PROTEIN C21ORF13.
NT2RI2025957// LU1 protein [Homo sapiens]
NT2RI3006284// Homo sapiens chorea-acanthocytosis (CHAC) mRNA, complete cds.
NT2RI3008697// erythroblast macrophage protein [Mus musculus]
NT2RP8000296// similar to Kelch proteins
NTONG2007517// RING CANAL PROTEIN (KELCH PROTEIN).
OCBBF2002124// p40 [Homo sapiens]
OCBBF2007610// PSD-95/SAP90-associated protein-4 [Rattus norvegicus].
OCBBF2021323// Mus musculus GTRGEO22 (Gtrgeo22) mRNA, complete cds.
OCBBF2028173// JM11 protein [Homo sapiens]
PEBLM2001465// diphthamide biosynthesis; Dph5p [Saccharomyces cerevisiae]
PERIC2004028// Mus musculus erythroblast macrophage protein EMP mRNA, complete cds.
PLACE7006051// cytoplasmic dynein heavy chain 2 [Rattus norvegicus]
PROST2008993// Mus musculus Pax transcription activation domain interacting protein PTIP mRNA, complete cds.
PUAEN2003079// nasopharyngeal carcinoma susceptibility protein [Homo sapiens]
SPLEN2002147// Halocynthia roretzi mRNA for HrPET-3, complete cds.
SPLEN2032154// NDRG1 PROTEIN (DIFFERENTIATION-RELATED GENE 1 PROTEIN) (DRG1) (REDUCING AGENTS AND TUNICAMYCIN-RESPONSIVE PROTEIN) (RTP) (NICKEL- SPECIFIC INDUCTION PROTEIN CAP43).
SYNOV2005216// Homo sapiens laryngeal carcinoma related protein 1 mRNA, complete cds.
SYNOV2007965// Homo sapiens mRNA for H-1(3)mbt-like protein, alternative variant a.
SYNOV4000706// B cell phosphoinositide 3-kinase adaptor [Mus musculus]
TBAES2004055// NY-REN-50 antigen
TESOP2001605// Homo sapiens laryngeal carcinoma related protein 1 mRNA, complete cds.
TESOP2005285// Homo sapiens partial mRNA for chr2 synaptotagmin (CHR2SYT gene).
TESTI2004215// Maackia amurensis early nodulin (ENOD2) mRNA, partial cds.
TESTI2009477// TRICHOHYALIN.
TESTI2034520// Rattus norvegicus SMC (segregation of mitotic chromosomes 1)-like 1 (yeast) (Smc111), mRNA
TESTI2052693// brk kinase substrate [Homo sapiens].
TESTI4006079// MUF1 protein; likely ortholog of mouse MUF1; elongin BC-interacting leucine-rich repeat protein [Homo sapiens]
TESTI4006393// neural specific sr protein NSSR 2 [Mus musculus]
TESTI4006546// colon cancer antigen NY-CO-45 [Homo sapiens].
TESTI4006802// mesothelin; megakaryocyte potentiating factor [Mus musculus]
TESTI4008018// DAZ associated protein 2; KIAA0058 gene product [Homo sapiens]
TESTI4009286// Homo sapiens HOTTL protein mRNA, complete cds
TESTI4009563// testis specific ankyrin-like protein 1 [Homo sapiens]
TESTI4010831// yeast Sec31p homolog; ABP125 [Homo sapiens]
TESTI4011484// Sec23-interacting protein p125 [Homo sapiens]
TESTI4014818// AD-012 protein [Homo sapiens]
TESTI4014924// selective hybridizing clone [Mus musculus]
TESTI4019140// Mi-2 histone deacetylase complex protein 66 [Xenopus laevis]
TESTI4019843// Rattus norvegicus huntingtin-associated protein interacting protein (duo) (Hapip), mRNA.
TESTI4023762// Trichohyalin.
TESTI4025920// B29 protein [Homo sapiens]
TESTI4039659// DnaJ homolog subfamily B member 8 (mDJ6).
TESTI4044186// leucine-rich, glioma inactivated 1 [Mus musculus]
THYMU2011736// latent transforming growth factor beta binding protein 3
THYMU2032825// Mus musculus mRNA for Drctnnb1a, complete cds.
THYMU2038369// Mus musculus GTRGEO22 (Gtrgeo22) mRNA, complete cds.
THYMU3001991// ART-4 protein [Homo sapiens]
THYMU3006172// membrane bound C2 domain containing protein [Rattus norvegicus]
TLIVE2003225// CUB and Sushi multiple domains 1 [Homo sapiens]
TLIVE2004320// Homo sapiens PC2-glutamine-rich-associated protein (PCQAP) mRNA, complete cds.
TOVAR2002247// Homo sapiens partial partial mRNA for NICE-4 protein, clone 3114f17.
TRACH2023299// growth factor receptor bound protein 2-associated protein 2 [Mus musculus]
TRACH3000926// cardiac morphogenesis [Mus musculus]
TRACH3001427// p47 [Homo sapiens]
TRACH3006412// Homo sapiens COP9 constitutive photomorphogenic homolog subunit 7B
TRACH3034731// Ras association (RalGDS/AF-6) domain family 2
TUTER2002729// D6MM5E protein [Mus musculus]
UTERU1000031// G.gallus mRNA for tom-1B protein.
UTERU2006115// ALPHA-ADAPTIN A (CLATHRIN ASSEMBLY PROTEIN COMPLEX 2 ALPHA-A LARGE CHAIN) (100 KDA COATED VESICLE PROTEIN A) (PLASMA MEMBRANE ADAPTOR HA2/AP2 ADAPTIN ALPHA A SUBUNIT).
UTERU2031268// NY-REN-25 antigen [Homo sapiens].
UTERU2035452// NG3 [Homo sapiens]
UTERU3001059// ABC1 protein homolog, mitochondrial precursor.
UTERU3005585// rhophilin-like protein [Homo sapiens]
UTERU3009871// feminization 1 homolog a (C. elegans)

The 14 clones shown below are clones which were unassignable to any of the above-mentioned categories, but have been predicted to have some function based on homology search using their full-length nucleotide sequences. Clone Name and Definition in the result of homology search, demarcated by a double slash mark (//), are shown below.
ADRGL2000042//Homo sapiens CTCL tumor antigen se20-4 mRNA, complete cds.
BRACE3009127//oxysterol binding protein 2; oxysterol binding protein-like 1 [Homo sapiens]
BRACE3021148//DC12 protein [Homo sapiens]
BRAMY2040159//Homo sapiens MRIP-1 mRNA, complete cds.
BRAWH3007441//CAT56 protein [Homo sapiens]
CTONG3001501//Mus musculus glucocorticoid-induced gene 1 mRNA, complete cds.
HCHON2000508//Homo sapiens prostate antigen PARIS-1 mRNA, complete cds.
OCBBF2020048// 95 kDa retinoblastoma protein binding protein; KIAA0661 gene product
PERIC2007068//Mus musculus mRNA for 1A13 protein.
TESTI4010382//cytoplasmic dynein heavy chain 2 [Rattus norvegicus]
TESTI4011072//tudor domain containing 1 [Mus musculus]
TESTI4046240//sirtuin 7
UTERU2019534//Golgi apparatus protein 1 [Homo sapiens]
UTERU2028734//Mus musculus slp2-a mRNA for synaptotagmin-like protein 2-a delta 2S-III, complete cds.

Further, a polypeptide will not always belong solely to a single category of the above-described functional categories, and therefore, a polypeptide may belong to any of the predicted functional categories. Further analyses may yield additional functions for clones classified into these functional categories.

Detailed descriptions concerning each domain or motif can be found in websites linked from the websites of Pfam, InterPro (http://www.ebi.ac.uk/interpro/), PROSITE (http://www.expasy.ch/cgi-bin/prosite-list.pl), or such. This information can be found based on domain/motif names, and accession numbers of hit data obtained through domain searches of Pfam (http://www.sanger.ac.uk/Software/Pfam/index.shtml) (see Example 5) for amino acid sequences deduced from the 2,495 full-length clones of the present invention whose full-length nucleotide sequences have been determined. PROSITE in particular enables comparison of unique functional categories. The functions of polypeptides encoded by the 914 clones with hit data in Pfam were predicted and classified into the 13 functional categories described below. As a result, 661 clones were estimated to encode proteins belonging to these categories.
Secretory and/or membrane protein (87 clones)
Glycoprotein-related protein (85 clones)
Signal transduction-related protein (154 clones)
Transcription-related protein (115 clones)
Enzyme and/or metabolism-related protein (265 clones)
Cell division- and/or cell proliferation-related protein (13 clones)
Cytoskeleton-related protein (40 clones)
Nuclear protein and/or RNA synthesis-related protein (31 clones)
Protein synthesis- and/or transport-related protein (46 clones)
Cellular defense-related protein (seven clones)
Development and/or differentiation-related protein (two clones)
DNA- and/or RNA-binding protein (179 clones)
ATP- and/or GTP-binding protein (36 clones)

The clones predicted to belong to the category of secretory protein and/or membrane protein are the following 64 clones.
ASTRO2014923, ASTRO3000301, BRACE2005457, BRACE2014306, BRACE3001391, BRACE3014005, BRALZ2016085, BRAMY2040592, BRAWH2014662, BRHIP2004814, BRHIP3024118, BRTHA3002427, BRTHA3017848, BRTHA3018656, CTONG2009423, CTONG2013178, D3OST2002648, FEBRA2007708, FEBRA2008311, HCHON2001084, HCHON2001712, HCHON2004531, HCHON2005921, HSYRA2009102, KIDNE1000064, KIDNE2000832, NT2RI3006376, OCBBF2031167, OCBBF2035110, OCBBF2038317, PEBLM2002594, PERIC1000147, PERIC2009086, PROST1000184, SPLEN2012624, SPLEN2031547, SPLEN2033098, SPLEN2036326, TESTI1000257, TESTI1000390, TESTI2000644, TESTI2002036, TESTI2002928, TESTI2006648, TESTI2024567, TESTI2034520, TESTI4000014, TESTI4000724, TESTI4007163, TESTI4009881, TESTI4028880, THYMU2009425, THYMU2011548, THYMU2033079, THYMU2041015, TLIVE2000023, TLIVE2003381, TLIVE2007132, TRACH2006387, TRACH2007059, TRACH3004786, UTERU3000645, UTERU3004616, UTERU3006308

The following 23 clones are also predicted to belong to the category of secretory protein and/or membrane protein.
BRACE2029396, BRACE3005107, BRACE3010076, BRAMY2019111, BRAMY3004800, BRHIP3000017, FCBBF1000509, HCHON2000508, HEART2009680, IMR322013396, NT2RI2009583, NT2RI3000174, NT2RP8000521, OCBBF2030116, TESTI2029252, TESTI4013894, TESTI4032112, TESTI4041086, THYMU2035710, TKIDN2012771, TRACH3000420, UTERU2004299, TESTI4038779

The clones predicted to belong to the category of glycoprotein-related protein are the following 77 clones.
ADIPS2000088, BRACE2043142, BRACE2046295, BRACE3014005, BRAMY2005052, BRAMY4000277, BRAWH2007658, BRCAN2006063, BRSTN2004863, BRTHA3017589, BRTHA3017848, COLON2000568, COLON2004478, CTONG2000042, CTONG2013178, CTONG2024206, CTONG2024749, CTONG2025496, CTONG3001370, CTONG3003737, D3OST2002182, FEBRA2007708, HCHON2001084, HCHON2002676, HCHON2004531, HEART2001680, HLUNG2014262, LYMPB2000083, NESOP2001433, NOVAR2001108, NT2RI3006171, NT2RI3006340, NT2RI3007978, NT2RP7014005, OCBBF2010140, OCBBF2037598, PLACE5000171, PLACE6012574, PLACE7006051, PUAEN2009174, SMINT2002743, SMINT2010076, SMINT2011888, SMINT2015787, SPLEN2001599, SPLEN2015267, SPLEN2021701, SPLEN2037722, STOMA2004294, SYNOV3000231, SYNOV3000302, SYNOV4007521, SYNOV4007671, TBAES2003550, TESOP2005485, TESTI2005610, TESTI4006326, TESTI4021294, THYMU2005303, THYMU2019210, THYMU2023711, THYMU2027695, TRACH2007059, TRACH2022425, TRACH2022553, TRACH2022649, TRACH3002168, TRACH3005479, TRACH3005549, TRACH3006470, TRACH3035526, TRACH3036609, TSTOM2000442, UTERU2026090, UTERU3004616, UTERU3004992, UTERU3006308

The following eight clones are also predicted to belong to the category of glycoprotein-related protein.
BRAWH2006395, BRHIP3000017, NT2RI3007443, OCBBF3002654, TESTI2039060, TESTI4013894, TESTI4031745, TLIVE2001684

The clones predicted to belong to the category of signal transduction-related protein are the following 116 clones.
BLADE2007958, BNGH42007788, BRACE1000258, BRACE2008594, BRACE2041009, BRACE3001391, BRACE3006872, BRACE3011421, BRACE3024073, BRACE3027326, BRALZ2014484, BRAMY2001473, BRAMY2036567, BRAMY2042760, BRAMY2047751, BRAMY3001794, BRAMY3002803, BRAMY3005091, BRAMY3008466, BRAMY4000095, BRAWH3001326, BRAWH3002821, BRAWH3005912, BRCAN2002856, BRCAN2009432, BRCAN2016619, BRCAN2024451, BRCAN2028355, BRHIP2000819, BRHIP2005932, BRHIP3008405, BRHIP3025161, BRSSN2000684, BRSSN2004719, BRSTN2008418, BRSTN2013741, BRTHA3009037, BRTHA3013884, COLON2001721, CTONG2006798, CTONG3000084, CTONG3000657, CTONG3002127, D3OST3000169, DFNES2001108, DFNES2011499, FCBBF3007540, HCASM2001301, HCHON2000028, HCHON2006250, HHDPC1000118, HLUNG2001996, HLUNG2002465, KIDNE2001847, MESAN2006563, NHNPC2001816, NT2NE2003252, NT2RI2005166, NT2RI3000622, NT2RI3006673, NT2RP7005118, NT2RP7005529, NT2RP7009147, NT2RP7013795, NT2RP8000483, NTONG2003852, OCBBF2004826, OCBBF2004883, OCBBF2007028, OCBBF2008770, OCBBF2022351, OCBBF2037340, OCBBF2037547, PEBLM2004666, PLACE7008431, PROST2016462, PROST2018511, PUAEN2002616, PUAEN2005930, PUAEN2006328, PUAEN2009852, SYNOV2021320, TESOP2000801, TESOP2001166, TESTI2006648, TESTI2026505, TESTI2050137, TESTI2052693, TESTI4000079, TESTI4010713, TESTI4010831, TESTI4011956, TESTI4016882, TESTI4019843, TESTI4028059, THYMU2032014, THYMU2037226, THYMU2038615, THYMU3001234, THYMU3006172, THYMU3008436, TLIVE2009541, TRACH2009310, TRACH2021398, TRACH2023299, TRACH2025535, TRACH3009455, TRACH3034731, TSTOM2000553, UTERU1000337, UTERU2005621, UTERU2025025, UTERU2036089, UTERU2038251, UTERU3003523, UTERU3007419

The following 38 clones are also predicted to belong to the category of signal transduction-related protein.
BLADE2000579, BRACE3001058, BRACE3003053, BRACE3009127, BRAMY2040159, BRAMY3004800, BRAWH3009017, BRCAN2014229, BRHIP2026877, BRTHA2013610, CTONG3004550, FEBRA2001990, FEBRA2008692, HCHON2000508, MESAN2001770, NT2RI2005772, NT2RI3007443, NTONG2008093, OCBBF2005433, OCBBF2024284, OCBBF2034637, OCBBF3002654, TESOP2000390, TESTI2025924, TESTI2049956, TESTI4000319, TESTI4005317, TESTI4021482, TESTI4025268, TESTI4031745, THYMU2004139, THYMU2031249, TRACH2024408, UTERU2008040, UTERU2028734, UTERU3000402, UTERU3000738, UTERU3015412

The clones predicted to belong to the category of transcription-related protein are the following 27 clones.
BRACE2006319, BRACE3013576, BRAMY2030109, BRAWH3005912, BRHIP3025161, CORDB1000140, CTONG1000467, HEART2001756, IMR322000127, IMR322000917, KIDNE1000064, NOVAR2000136, NT2NE2006531, NT2RI3007158, NT2RP7000466, OCBBF2036743, OCBBF3009279, PLACE6019385, TESTI2026505, TESTI2044796, TESTI2050987, TESTI4017001, TESTI4019140, TESTI4034912, THYMU2035735, TRACH2025749, TRACH3004840

The following 88 clones are also predicted to belong to the category of transcription-related protein.
BRACE2003609, BRACE3001058, BRACE3001113, BRACE3003026, BRAMY2035070, BRAMY2035449, BRAMY2035718, BRAMY2039341, BRAMY2045471, BRAWH3007441, BRHIP2017553, BRSTN2013354, CERVX2002013, CTONG1000113, CTONG2003348, CTONG2020374, CTONG2020378, CTONG2020411, CTONG2024031, CTONG2028758, CTONG3001501, CTONG3004726, DFNES2011192, FCBBF3027854, FEBRA2014122, FEBRA2027609, HCASM2003018, HCASM2003099, HCHON2000508, HCHON2000743, HCHON2004858, HSYRA2005628, MESAN2014412, MESAN2015501, NT2RI2008952, NT2RI2018448, NT2RI3000174, NT2RI3001132, NT2RI3002557, NT2RI3007167, NT2RI3007443, OCBBF2008144, OCBBF2009583, OCBBF2011669, OCBBF2019684, OCBBF2020048, OCBBF2024284, OCBBF2032274, OCBBF3000167, OCBBF3003761, SPLEN2016135, SPLEN2016781, SPLEN2036702, SYNOV2021953, SYNOV4002744, TESOP2001796, TESOP2005199, TESOP2006398, TESTI2008901, TESTI2034251, TESTI2037830, TESTI4000183, TESTI4000214, TESTI4006473, TESTI4008058, TESTI4008302, TESTI4013365, TESTI4014801, TESTI4015442, TESTI4017714, TESTI4025494, TESTI4025547, TESTI4028938, TESTI4029348, TESTI4031745, TESTI4032090, THYMU2006001, THYMU2028739, THYMU2031139, THYMU3001428, TRACH2007483, TRACH3000134, TRACH3003832, TRACH3007866, UTERU3001053, UTERU3014791, UTERU3017176, TESTI4038779

The clones predicted to belong to the category of enzyme and/or metabolism-related protein are the following 176 clones.
3NB692002806, ASTRO1000009, BLADE2005036, BLADE2008539, BRACE2005457, BRACE2008594, BRACE2014475, BRACE2018762, BRACE2035381, BRACE2043142, BRACE2047011, BRACE3004058, BRACE3007625, BRACE3009708, BRACE3011421, BRACE3015262, BRACE3024073, BRACE3025630, BRACE3027478, BRAMY2047746, BRAMY2047751, BRAMY3002803, BRAMY3004919, BRAMY3005091, BRAMY4000095, BRAWH2010000, BRAWH2014414, BRAWH2014662, BRAWH2016702, BRAWH3002821, BRAWH3003727, BRCAN2021028, BRCAN2024451, BRCAN2028355, BRCOC2003213, BRHIP2004359, BRHIP2026288, BRHIP3008183, BRHIP3025161, BRHIP3027137, BRSSN2000684, BRSTN2000872, BRSTN2004863, BRSTN2004987, BRTHA2012980, BRTHA3002401, BRTHA3008778, BRTHA3009037, BRTHA3009090, BRTHA3015815, BRTHA3016917, BRTHA3017848, BRTHA3018656, COLON2001721, CTONG2004062, CTONG2006798, CTONG2013178, CTONG2028124, CTONG3002127, CTONG3005325, CTONG3005648, D3OST2002182, FCBBF3004502, FCBBF3013307, FEBRA2007708, FEBRA2008468, FEBRA2026984, HCASM2001301, HCASM2002918, HCHON2002676, HCHON2004007, HCHON2004531, HEART2006131, HHDPC1000118, HLUNG1000017, KIDNE2000832, KIDNE2006580, MESAN2012054, NOVAR2000136, NT2NE2003252, NT2NE2006909, NT2RI2004618, NT2RI3004510, NT2RI3006673, NT2RI3007978, NT2RI3008652, NT2RP7010599, NT2RP7014005, NT2RP7017474, NTONG2000413, OCBBF2004826, OCBBF2006058, OCBBF2019823, OCBBF2025527, OCBBF2031167, OCBBF2037340, OCBBF2037547, OCBBF2037638, PERIC2009086, PLACE7002641, PLACE7008431, PROST2017367, PUAEN2007044, PUAEN2009795, PUAEN2009852, SPLEN2010912, SPLEN2015679, SPLEN2030335, SYNOV4002392, SYNOV4002883, TBAES2003550, TESOP2000801, TESOP2004114, TESOP2009121, TESTI1000257, TESTI1000545, TESTI2002618, TESTI2006648, TESTI2040018, TESTI2049469, TESTI2053621, TESTI4000288, TESTI4000349, TESTI4001148, TESTI4001527, TESTI4001561, TESTI4002552, TESTI4006819, TESTI4007382, TESTI4007810, TESTI4008429, TESTI4010713, TESTI4010851, TESTI4012448, TESTI4012679, TESTI4013369, TESTI4016925, TESTI4018835, TESTI4020920, TESTI4021478, TESTI4022716, TESTI4026510, TESTI4028059, TESTI4029836, TESTI4032895, TESTI4034432, TESTI4036909, THYMU2006420, THYMU3000133, THYMU3001379, THYMU3004835, THYMU3006172, THYMU3008436, TLIVE2002336, TRACH2006387, TRACH2009310, TRACH2019473, TRACH2022425, TRACH2023299, TRACH3005479, TRACH3006470, TRACH3007479, TRACH3008093, TRACH3008629, TRACH3036193, TSTOM2000553, UTERU2005621, UTERU2017762, UTERU2025025, UTERU2033375, UTERU3000828, UTERU3001240, UTERU3001585, UTERU3003116, UTERU3005460, UTERU3005907

The following 89 clones are also predicted to belong to the category of enzyme and/or metabolism-related protein.
BLADE2000579, BRACE2039823, BRACE3003053, BRAMY2038516, BRAMY2040159, BRAWH1000369, BRCAN2003070, BRCAN2014229, BRCOC2019841, BRHIP2005724, BRHIP2008389, BRHIP2026877, BRHIP3000240, BRHIP3026052, BRTHA2002133, BRTHA2002702, BRTHA2007060, BRTHA2010033, BRTHA2013426, BRTHA2013610, BRTHA2017364, BRTHA2018011, BRTHA3000296, CTONG2004000, CTONG2016942, CTONG2020374, CTONG2024031, CTONG3002552, CTONG3003598, CTONG3004550, FCBBF1000509, FEBRA2008692, HCASM2002754, HCASM2003099, HCASM2003357, HLUNG2015418, HLUNG2015548, IMR322013731, MESAN2005303, NT2RI2005772, NT2RI2008952, NT2RI3000174, NT2RI3007443, NT2RP7008435, NTONG2008093, OCBBF2006987, OCBBF2034637, OCBBF3002654, PLACE7000333, PLACE7000502, PROST2000452, SPLEN2039311, STOMA2003158, SYNOV2013637, TESOP2000390, TESTI2015626, TESTI2025924, TESTI2026647, TESTI2035981, TESTI2036288, TESTI2039060, TESTI2049956, TESTI4000155, TESTI4001984, TESTI4006473, TESTI4010382, TESTI4011072, TESTI4014801, TESTI4017714, TESTI4021482, TESTI4025547, TESTI4025865, TESTI4026207, TESTI4028958, TESTI4029690, TESTI4031745, TESTI4032090, THYMU2004139, THYMU2031139, THYMU2031249, THYMU2040925, TKIDN2012771, TLIVE2002046, TLIVE2007607, TRACH3000420, TRACH3007866, UTERU2019534, UTERU2028734, UTERU3000738

The clones predicted to belong to the category of cell division and/or cell proliferation-related protein are the following ten clones.
BRAWH2001940, CTONG3001123, HCHON2001217, PROST2008993, TBAES2001171, TESTI4021294, TESTI4035498, UTERU1000024, UTERU3002993, UTERU3003523

The following three clones are also predicted to belong to the category of cell division and/or cell proliferation-related protein.
BRACE2029396, BRAWH2010552, TESTI4013365

The clones predicted to belong to the category of cytoskeleton-related protein are the following 36 clones.
BRACE2026836, BRACE2045300, BRAWH3000314, BRSTN2004863, BRTHA2004978, BRTHA3003449, BRTHA3005046, COLON2002520, CORDB2000541, CTONG3002674, FCBBF3012288, HCHON2001577, HLUNG2017350, HSYRA2005456, HSYRA2009075, NT2RI3006340, NT2RI3006673, NT2RI3007291, OCBBF2037598, PLACE5000282, TESTI2003347, TESTI2034767, TESTI4000288, TESTI4007778, TESTI4009160, TESTI4018886, TESTI4030603, TESTI4034632, TESTI4035063, THYMU1000496, THYMU2008725, TRACH2005811, TRACH2007059, UTERU2007724, UTERU2035745, UTERU3004616

The following four clones are also predicted to belong to the category of cytoskeleton-related protein.
NT2RI2005772, OCBBF2006987, SPLEN2030847, TESTI4026207

The clones predicted to belong to the category of nuclear protein and/or RNA synthesis-related protein are the following 20 clones.
BRACE3024073, BRAWH2001940, BRCOC2003213, BRSTN2004987, BRTHA3016917, CTONG3009028, FCBBF3013307, FEBRA2026984, SPLEN2010912, TBAES2001171, TESTI2040018, TESTI4019566, TESTI4022716, TESTI4026510, TESTI4036909, THYMU3000133, TRACH2023299, TRACH3036193, UTERU1000024, UTERU3002993

The following eleven clones are also predicted to belong to the category of nuclear protein and/or RNA synthesis-related protein.
BRACE3003053, BRCAN2002473, BRTHA2017364, NT2RI2008952, NT2RI3000174, TESTI2026647, TESTI2035981, TESTI4000155, TESTI4006473, TESTI4010382, TESTI4025547

The clones predicted to belong to the category of protein synthesis and/or transport-related protein are the following 29 clones.
BRACE2014306, BRACE3008720, BRAWH3000491, BRCAN2009432, BRHIP2000920, BRTHA3013884, CTONG2013178, HCHON2004531, HLUNG1000017, HLUNG2013851, HSYRA2005496, NT2NE2006909, NT2RI3006340, OCBBF2007068, OCBBF2031167, PUAEN2009795, TBAES2001229, TBAES2004055, TESTI2051867, TESTI4000014, TESTI4000349, TESTI4009608, TESTI4010851, TESTI4034632, TRACH3007479, TRACH3036193, UTERU2017762, UTERU2019940, UTERU2033375

The following 17 clones are also predicted to belong to the category of protein synthesis and/or transport-related protein.
BLADE2000579, BRACE3003053, BRCAN2003070, BRTHA2018011, BRTHA3000296, CTONG2016942, MESAN2005303, NT2RI3002557, NT2RP7008435, PERIC2007068, PLACE7000502, PROST2000452, TESTI4001984, TESTI4017714, THYMU2004284, TRACH3000420, TRACH3007866

The clones predicted to belong to the category of cellular defense-related protein are the following four clones.
BRTHA2015878, CTONG3000084, NT2RI3002842, PEBLM2004666

The following three clones are also predicted to belong to the category of cellular defense-related protein.
BRCAN2002473, NT2RI3007167, TRACH3002561

The clones predicted to belong to the category of development and/or differentiation-related protein are the following one clone.
TESTI4014924

The clones predicted to belong to the category of DNA-binding and/or RNA-binding protein are the following 67 clones.
BRACE2006319, BRACE2047011, BRACE3004150, BRACE3013576, BRACE3024073, BRAMY2030109, BRAWH3005912, BRCAN2002562, BRCOC2003213, BRHIP2021615, BRHIP3008183, BRHIP3025161, BRSTN2004987, BRTHA2018707, BRTHA3016917, CORDB1000140, CTONG1000467, CTONG3000084, CTONG3003972, CTONG3008831, CTONG3009028, FCBBF3013307, FEBRA2026984, HEART2001756, HLUNG2013851, IMR322000127, IMR322000917, KIDNE1000064, NT2NE2006531, NT2RI3003382, NT2RI3007158, NT2RP7000466, NT2RP7004123, OCBBF2036743, OCBBF3009279, PLACE6019385, SPLEN2006122, SPLEN2010912, TESOP2009121, TESTI1000390, TESTI2014716, TESTI2026505, TESTI2040018, TESTI2044796, TESTI2050987, TESTI4007810, TESTI4009374, TESTI4011745, TESTI4012679, TESTI4017001, TESTI4019140, TESTI4019566, TESTI4022716, TESTI4026510, TESTI4034432, TESTI4034912, TESTI4036909, THYMU2035319, THYMU2035735, THYMU3000133, TLIVE2002336, TRACH2023299, TRACH2025749, TRACH3004840, TRACH3036193, UTERU2026025, UTERU3009490

The following 112 clones are also predicted to belong to the category of DNA-binding and/or RNA-binding protein.
BLADE2006830, BRACE2003609, BRACE3001058, BRACE3001113, BRACE3003026, BRACE3003053, BRACE3010076, BRAMY2035070, BRAMY2035449, BRAMY2039341, BRAMY2045471, BRAWH1000369, BRAWH3007441, BRHIP2017553, BRSTN2013354, BRTHA2002133, BRTHA2002702, BRTHA2017364, BRTHA2017972, CERVX2002013, CTONG1000113, CTONG2003348, CTONG2015596, CTONG2020374, CTONG2020378, CTONG2020411, CTONG2024031, CTONG2028758, CTONG3001501, CTONG3004726, DFNES2011192, FCBBF1000509, FCBBF3027854, FEBRA2014122, FEBRA2027609, HCASM2003018, HCASM2003099, HCASM2009424, HCHON2000508, HCHON2000743, HCHON2004858, HSYRA2005628, IMR322013731, MESAN2014412, MESAN2015501, NT2RI2008952, NT2RI2018448, NT2RI2027157, NT2RI3000174, NT2RI3001132, NT2RI3002557, NT2RI3007167, NT2RI3007443, OCBBF2006987, OCBBF2008144, OCBBF2009583, OCBBF2011669, OCBBF2019684, OCBBF2020048, OCBBF2024284, OCBBF2032274, OCBBF2034637, OCBBF3000167, OCBBF3003761, PERIC2007068, SPLEN2016135, SPLEN2016781, SPLEN2036702, STOMA2003158, SYNOV2021953, SYNOV4002744, TESOP2001796, TESOP2005199, TESOP2006398, TESTI2008901, TESTI2026647, TESTI2034251, TESTI2035981, TESTI2037830, TESTI4000155, TESTI4000183, TESTI4000214, TESTI4006473, TESTI4008058, TESTI4008302, TESTI4010382, TESTI4013365, TESTI4014801, TESTI4015442, TESTI4017714, TESTI4025494, TESTI4025547, TESTI4026207, TESTI4028938, TESTI4028958, TESTI4029348, TESTI4031745, TESTI4032090, THYMU2006001, THYMU2028739, THYMU2031139, THYMU3001428, TKIDN2012771, TLIVE2007607, TRACH2007483, TRACH3000134, TRACH3003832, TRACH3007866, UTERU3001053, UTERU3014791, UTERU3017176, TESTI4038779

The clones predicted to belong to the category of ATP binding and/or GTP-binding protein are the following 28 clones.
BRACE3008720, BRACE3009708, BRAMY2047746, BRAMY3004919, BRAWH2014662, BRAWH2016702, BRCAN2009432, BRCAN2024451, BRSTN2013741, BRTHA3008778, BRTHA3009090, CTONG2004062, CTONG2028124, HCHON2004007, OCBBF2037340, SPLEN2030335, TESTI4000288, TESTI4001148, TESTI4002552, TESTI4008429, TESTI4018835, TESTI4021478, TESTI4029836, THYMU2036459, THYMU3001379, TRACH2001549, UTERU3000828, UTERU3001240

The following eight clones are also predicted to belong to the category of ATP binding and/or GTP-binding protein.
BRCAN2014229, BRHIP2008389, CTONG3004550, FEBRA2001990, IMR322013396, IMR322013731, MESAN2001770, TESTI4000319

The following 208 clones have hit data in Pfam (see Example 5), and each has a functional domain or motif. It is currently unclear as to which of the above-described categories each of these clones belong. However, if data on polypeptides with a similar domain or motif can be accumulated, and their functions clarified in more detail, they may be classified into any of the above-described categories.
3NB692002685, 3NB692008729, ASTRO2003960, BNGH42003570, BRACE2010489, BRACE2015314, BRACE2016981, BRACE2027258, BRACE2030341, BRACE2035441, BRACE2038329, BRACE2042550, BRACE2044286, BRACE3000071, BRACE3000973, BRACE3001002, BRACE3003192, BRACE3004772, BRACE3004880, BRACE3008137, BRACE3008384, BRACE3009090, BRACE3010397, BRACE3015521, BRACE3016884, BRACE3019084, BRAMY2004771, BRAMY2019300, BRAMY2021498, BRAMY2031317, BRAMY2039872, BRAMY2046989, BRAMY3004224, BRAMY3005932, BRAWH1000127, BRAWH2001395, BRAWH2014954, BRAWH3000078, BRAWH3001891, BRAWH3002574, BRAWH3002600, BRAWH3008341, BRCAN2002948, BRCAN2009203, BRCAN2015464, BRCAN2017717, BRCOC2001505, BRCOC2016525, BRHIP2003786, BRHIP2005236, BRHIP2007616, BRHIP2009414, BRHIP3000339, BRHIP3008313, BRSTN2001067, BRTHA2000855, BRTHA2005579, BRTHA2007122, BRTHA2008527, BRTHA2009311, BRTHA2010884, BRTHA2013262, BRTHA2014792, BRTHA2015406, BRTHA2016496, BRTHA2018591, BRTHA2018624, BRTHA2019048, BRTHA3003074, BRTHA3008310, CTONG1000341, CTONG2001877, CTONG2008233, CTONG2017500, CTONG2020026, CTONG2028687, CTONG3000686, CTONG3004072, CTONG3006067, CTONG3006186, CTONG3009385, DFNES2000146, DFNES2005266, FCBBF3009888, FCBBF3012170, FEBRA2000253, FEBRA2007801, FEBRA2021571, FEBRA2024150, HCHON2004776, HEART1000139, HEART2006909, HEART2010495, HLUNG2000014, HLUNG2002958, HLUNG2011298, IMR322006495, KIDNE2000846, KIDNE2001361, KIDNE2011635, KIDNE2012945, NESOP2001656, NT2RI2008724, NT2RI2025909, NT2RI2025957, NT2RI3007543, NT2RP7000359, NT2RP7004027, NT2RP7011570, NT2RP8000296, NTONG2005277, NTONG2006354, NTONG2007517, OCBBF2006764, OCBBF2010416, OCBBF2020838, OCBBF2021323, OCBBF2033869, PERIC2001228, PERIC2003720, PLACE6020031, PLACE7000514, PROST2018090, RECTM2000433, SKMUS2006394, SMINT1000192, SPLEN2002147, SPLEN2002467, SPLEN2031780, SPLEN2034081, SPLEN2036821, SYNOV2005448, SYNOV2005817, SYNOV2006430, SYNOV2014400, SYNOV4007553, SYNOV4008440, TESOP2001953, TESTI2000443, TESTI2004700, TESTI2027019, TESTI4000462, TESTI4000970, TESTI4002491, TESTI4006546, TESTI4007064, TESTI4011484, TESTI4012406, TESTI4015471, TESTI4016110, TESTI4017137, TESTI4017575, TESTI4018152, TESTI4018555, TESTI4020092, TESTI4023555, TESTI4025920, TESTI4026192, TESTI4027557, TESTI4028429, TESTI4028612, TESTI4028983, TESTI4030505, TESTI4038492, TESTI4039659, TESTI4041053, TESTI4044084, TESTI4046487, TESTI4046819, THYMU2004693, THYMU2011736, THYMU2016204, THYMU2027734, THYMU2038369, THYMU2038797, THYMU3000028, THYMU3003212, THYMU3003763, THYMU3007137, THYMU3008171, TLIVE2002338, TLIVE2002690, TLIVE2003225, TLIVE2008229, TRACH2001443, TRACH3001427, TRACH3003379, TRACH3008713, TRACH3035235, TUTER2000425, UTERU1000031, UTERU2006115, UTERU2006568, UTERU2019706, UTERU2035328, UTERU2035331, UTERU2035452, UTERU3001652, UTERU3001766, UTERU3001988, UTERU3002667, UTERU3003178, UTERU3005585, UTERU3007640, UTERU3008660, UTERU3009871, UTERU3009979, UTERU3015500

Likewise, the following 45 clones also had hit data in Pfam (see Example 5), although it remains unclear as to which of the above-described categories each clone belongs. When data on polypeptides with a similar domain or motif are accumulated, and their functions are clarified in more detail, these clones may also be classified into any of the above-described categories.
3NB692004724// KRAB box// Integrase core domain
ADRGL2000042// Nucleosome assembly protein (NAP)
BRACE2037299// Integrase core domain
BRALZ2017844// Homeobox domain
BRAWH2006207// KRAB box
BRCAN2002854// SAP domain
BRHIP2006617// TPR Domain// TPR Domain
BRHIP2012360// XPG N-terminal domain// XPG I-region
BRHIP3008314// Sir2 family
BRTHA2016318// KE2 family protein
CTONG2019822// Hepatitis C virus core protein
FCBBF3010361// Fork head domain
FEBRA2006519// Thrombospondin type 1 domain// Thrombospondin type 1 domain
FEBRA2028256// EGF-like domain// EGF-like domain// EGF-like
domain// EGF-like domain// EGF-like domain// TB domain// EGF-like domain// EGF-like domain// EGF-like domain// EGF-like domain// EB module// Squash family of serine protease inhibitors// EGF-like domain// EGF-like domain
FEBRA2028516// GRIP domain
HCASM2008536// XRCC1 N terminal domain
IMR322007078// UBA domain
IMR322008651// Helix-hairpin-helix motif.
LIVER2000247// Sodium
OCBBF2003327// Thrombospondin type 1 domain// Thrombospondin type 1 domain// Thrombospondin type 1 domain
PROST2009320// LIM domain containing proteins// LIM domain containing proteins
PUAEN2006335// Formin Homology 2 Domain
SKMUS2003194// SAP domain
SPLEN2039379// Transthyretin precursor (formerly prealbumin)
SYNOV1000256// Leucine Rich Repeat// BAH domain// Leucine Rich
Repeat// Leucine Rich Repeat// Leucine Rich Repeat
SYNOV2006620// Nuclear transition protein 2
SYNOV4003981// Somatomedin B domain// WAP-type (Whey Acidic Protein) 'four-disulfide core'// Hemopexin// Hemopexin
SYNOV4005889// Apolipoprotein A1/A4/E family
TESOP2006865// KRAB box
TESTI1000266// Integrase core domain
TESTI2050780// Kazal-type serine protease inhibitor domain
TESTI4000137// Domain of unknown function
TESTI4024387// GDP dissociation inhibitor
TESTI4029528// RanBP1 domain.
TESTI4038721// Squash family of serine protease inhibitors
TESTI4046240// Sir2 family
THYMU2035078// Domain of unknown function DUF27
THYMU3000269// FAD binding domain
THYMU3000360// Integrase core domain
TRACH1000212// TSC-22/dip/bun family
TRACH2000862// Guanylate-binding protein
TRACH2019672// CRAL/TRIO domain.
TRACH2024559// IQ calmodulin-binding motif// IQ calmodulin-binding motif
UTERU2032279// Serpins (serine protease inhibitors)
UTERU2033577// KRAB box

The function of a motif or domain may sometimes belong to more than one of the above-described functional categories, and there is also the possibility that such a motif or domain may be predicted to belong to every functional category. As new polypeptide data are accumulated and novel domains and motifs are found, a new functional domain or motif may be identified by re-analyzing deduced amino acid sequences in homology searches using updated databases. Thus in the future, the remaining clones, for which there are currently no hit data, may be classified into any of the above-described categories.

Since the polypeptides encoded by clones of the present invention contain full-length amino acid sequences, it is possible to analyze their biological activity and effect on cellular conditions such as cell proliferation and differentiation, by expressing the polypeptides as recombinant polypeptides using an appropriate expression system, injecting the recombinant into a cell, or raising a specific antibody against that polypeptide.

The biological activities of respective polypeptides can be analyzed by the methods as shown below.

### Secretory protein, transmembrane protein:

"Ion Channels" (Ed., R. H. Ashley, 1995) of "The Practical Approach Series" (IRL PRESS),
"Growth Factors" (Eds., I. McKay, I. Leigh, 1993),
"Extracellular Matrix" (Eds., M. A. Haralson, J. R. Hassell, 1995) ;

### Glycoprotein-related protein:

"Glycobiology" (Eds., M. Fukuda, A. Kobata, 1993) of "The Practical Approach Series" (IRL PRESS),
"Glycoprotein Analysis in Biomedicine" (Ed., Elizabeth F. Hounsell, 1993) of "Method in Molecular Biology" (Humana Press) series;

### Signal transduction-related protein:

"Signal Transduction" (Ed., G. Milligan, 1992) of "The Practical Approach Series" (IRL PRESS),
"Protein Phosphorylation" (Ed., D. G. Hardie, 1993), or
"Signal Transduction Protocols" (Eds., David A. Kendall, Stephen J. Hill, 1995) of "Method in Molecular Biology" (Humana Press) series;

### Transcription-related protein:

"Gene Transcription" (Eds., B. D. Hames, S. J. Higgins, 1993) of "The Practical Approach Series" (IRL PRESS),
"Transcription Factors" (Ed., D. S. Latchman, 1993); Enzyme and/or metabolism-related protein:
"Enzyme Assays" (Eds., ROBERT EISENTHAL and MICHAEL J. DANSON, 1992) of "The Practical Approach Series" (IRL PRESS);

### Cell division and/or cell proliferation-related protein:

"Cell Growth, Differentiation and Senescence" (Ed., GEORGE STUDZINSKI, 2000) of "The Practical Approach Series" (IRL PRESS) ;

### Cytoskeleton-related protein:

"Cytoskeleton: Signalling and Cell Regulation" (Eds., KERMIT L. CARRAWAY and CAROLIE A. CAROTHERS CARRAWAY, 2000) of "The Practical Approach Series" (IRL PRESS),
"Cytoskeleton Methods and Protocols" (Ed., Gavin, Ray H., 2000) of "Method in Molecular Biology" (Humana Press) series;

### Nuclear protein and/or RNA synthesis-related protein:

"Nuclear Receptors" (Ed., DIDIER PICARD, 1999) of "The Practical Approach Series" (IRL PRESS),
"RNA Processing" (Eds., STEPHEN J. HIGGINS and B. DAVID HAMES, 1994);

### Protein synthesis and/or transport-related protein:

"Membrane Transport" (Ed., STEPHEN A. BALDWIN, 2000) of "The Practical Approach Series" (IRL PRESS),
"Protein Synthesis Methods and Protocols" (Eds., Martin, Robin, 1998) of "Method in Molecular Biology" (Humana Press) series;

### Cellular defense-related protein:

"DNA Repair Protocols" (Henderson, Daryl S., 1999) of "Method in Molecular Biology" (Humana Press) series,
"Chaperonin Protocols" (Eds., Schneider, Christine, 2000);

### Development and/or differentiation-related protein:

"Developmental Biology Protocols" (Eds., ROBERT EISENTHAL and MICHAEL J. DANSON, 1992) of "Method in Molecular Biology" (Humana Press) series;

### DNA- and/or RNA-binding protein:

"DNA-Protein Interactions Principles and Protocols" (Eds., Kneale, G. Geoff, 1994) of "Method in Molecular Biology" (Humana Press) series,
"RNA-Protein Interaction Protocols" (Eds., Haynes, Susan R., 1999) ;

### ATP- and/or GTP-binding protein:

"Signal Transduction Protocols" (Eds., David A. Kendall, Stephen J. Hill, 1995) of "Method in Molecular Biology" (Humana Press)
series.

When other techniques are used, the activity of a polypeptide can be analyzed according to the description in Methods in Enzymology (Academic Press).

In the above-described categorization, a clone predicted to belong to the secretory and/or membrane protein category refers to a clone having hit data in a homology search with some annotation to suggest that the clone encodes a secretory and/or membrane protein, such as a growth factor, cytokine, hormone, signal, transmembrane, membrane, extracellular matrix, receptor, G-protein coupled receptor, ionic channel, voltage-gated channel, calcium channel, cell adhesion, collagen, and connective tissue protein; or a clone in which the results of PSORT and SOSUI analyses for deduced ORF suggest the presence of a nucleotide sequence encoding a signal sequence or transmembrane region; or a clone in which the results of a domain/motif search with Pfam suggest the presence of domains/motifs that suggest receptors, ion channels, hormones, or growth factors, for example, seven-transmembrane receptors, pancreatic hormone peptides, ion transport proteins, or fibroblast growth factors.

A clone predicted to belong to the glycoprotein-related protein category means a clone having hit data in a homology search with some annotation, such as glycoprotein, suggesting that the clone encodes a glycoprotein-related protein; or a clone in which the results of a domain/motif search with Pfam indicate the presence of domains and motifs such as a glycoprotein or glycosyltransferase that suggest the involvement of glycobiology, for example, immunoglobulin domain or glycosyl transferases group 1.

A clone predicted to belong to the signal transduction-related protein category means a clone having hit data in a homology search with some annotation, such as serine/threonine-protein kinase, tyrosine-protein kinase, SH3 domain, and SH2 domain, suggesting that the clone encodes a signal transduction-related protein; or a clone in which the results of a domain/motif search with Pfam suggest the presence of domains and motifs that suggest a protein kinase, dephosphoenzyme, SH2 domain, or small G protein, for example, eukaryotic protein kinase domain, protein phosphatase 2C, or Ras family.

A clone predicted to belong to the transcription-related protein category means a clone having hit data in a homology search with some annotation, such as transcription regulation, zinc finger, and homeobox, suggesting that the clone encodes a transcription-related protein; or a clone in which the results of a domain/motif search with Pfam suggest the presence of domains and motifs that suggest a transcription factor or transcription-controlling protein, for example, bZIP transcription factor, Zinc finger, or C2H2 type.

A clone predicted to belong to the category of disease-related protein means a clone having hit data in a homology search with some annotation, such as disease mutation and syndrome, suggesting that the clone encodes a disease-related protein; or a clone whose full-length nucleotide sequence has hit data in Swiss-Prot, GenBank, UniGene, nr or RefSeq, where that hit data corresponds to genes or polypeptides which have been deposited in the Online Mendelian Inheritance in Man (OMIM) (http://www.ncbi.nlm.nih.gov/Omim/), the human gene and disease database described later; or a clone in which the results of a domain/motif search with Pfam suggest the presence of domains and motifs, that suggest proteins with disease-specific expression or proteins involved in increasing or decreasing expression (depending on the disease), for example, Wilm's tumor protein or von Hippel-Lindau disease tumor suppressor protein.

A clone predicted to belong to the category of enzyme and/or metabolism-related protein means a clone having hit data in a homology search with some annotation, such as metabolism, oxidoreductase, and E. C. No. (Enzyme commission number), suggesting that the clone encodes an enzyme and/or metabolism-related protein; or a clone in which the results of a domain/motif search with Pfam suggests the presence of domains and motifs that suggest transferase, synthase, or hydrolase, for example, aldehyde dehydrogenase family, chitin synthase, or glucose-6-phosphate dehydrogenase.

A clone predicted to belong to the category of cell division and/or cell proliferation-related protein means a clone having hit data in a homology search with some annotation, such as cell division, cell cycle, mitosis, chromosomal protein, cell growth, and apoptosis, suggesting that the clone encodes a cell division and/or cell proliferation-related protein; or a clone in which the results of a domain/motif search with Pfam suggest the presence of domains and motifs that suggest cyclin or cell proliferation-controlling protein, for example, cyclin or cell division protein.

A clone predicted to belong to the category of cytoskeleton-related protein means a clone having hit data in a homology search with some annotation, such as structural protein, cytoskeleton, actin-binding, and microtubles, suggesting that the clone encodes a cytoskeleton-related protein; or a clone in which the results of a domain/motif search with Pfam suggest the presence of domains and motifs that suggest actin, kinesin, or fibronectin, for example, actin, fibronectin type I domain, or kinesin motor domain.

A clone predicted to belong to the category of nuclear protein and/or RNA synthesis-related protein means a clone having hit data in a homology search with some annotation, such as nuclear protein, RNA splicing, RNA processing, RNA helicase, and polyadenylation, suggesting that the clone encodes a nuclear protein and/or RNA synthesis-related protein; or a clone in which the results of a domain/motif search with Pfam suggest the presence of domains and motifs that suggest splicing factor, RNA synthase, or helicase, for example, hepatitis C virus RNA dependent RNA polymerase or DEAD/DEAH box helicase.

A clone predicted to belong to the category of protein synthesis and/or transport-related protein means a clone having hit data in a homology search with some annotation, such as translation regulation, protein biosynthesis, amino-acid biosynthesis, ribosomal protein, protein transport, and signal recognition particle, suggesting that the clone encodes a protein synthesis and/or transport-related protein; or a clone in which the results of a domain/motif search with Pfam suggest the presence of domains and motifs that suggest translation-relating protein, ubiquitin-relating protein, or ribosomal protein, for example, translation initiation factor SUI1, ubiquitin family, or ribosomal protein L16.

A clone predicted to belong to the category of cellular defense-related protein means a clone having hit data in a homology search with some annotation, such as heat shock, DNA repair, and DNA damage, suggesting that the clone encodes a cellular defense-related protein; or a clone in which the results of a domain/motif search with Pfam suggest the presence of domains and motifs that suggest chaperonin or DNA repair protein, for example, HSP90 protein or DNA mismatch repair protein.

A clone predicted to belong to the category of development and/or differentiation-related proteins means a clone having hit data in a homology search with some annotation, such as developmental protein, suggesting that the clone encodes a development and/or differentiation-related protein; or a clone in which the results of a domain/motif search with Pfam suggest the presence of domains and motifs that suggest organogenesis-relating protein, for example, floricaula/leafy protein.

A clone predicted to belong to the category of DNA- and/or RNA-binding protein means a clone having hit data in a homology search with some annotation, such as DNA-binding, RNA-binding, and such; or a clone in which the results of a domain/motif search with Pfam suggest the presence of domains and motifs that suggest DNA/RNA-relating enzyme group including transcription factor and DNA ligase or Zinc-finger relating protein, for example, transcription factor WhiB, B-box zinc finger, or tRNA synthetases class I (C).

A clone predicted to belong to the category of ATP- and/or GTP-binding protein means a clone having hit data in a homology search with some annotation, such as ATP-binding, GTP-binding, and such; or a clone in which the results of a domain/motif search with Pfam suggest the presence of domains and motifs that suggest ATP/GTP-relating enzyme group including ATPase or G protein, for example, E1-E2 ATPase or Ras family.

It is possible to perform functional analysis of a protein involved in a disease as described above. It is also possible to analyze correlation between a protein's expression or activity and a certain disease by using a specific antibody obtained by using the expressed protein. Alternatively, it is possible to utilize the OMIM database, which is a database of human genes and diseases, to analyze the protein. New information is constantly being deposited into this database. Therefore, it is possible that one skilled in the art will find a new relationship between a particular disease and a gene of the present invention by using the most up-to-date database. Proteins involved in diseases are useful for developing diagnostic markers or medicines for regulation of their expression and activity, or as gene therapy targets.

The proteins may have a variety of functions, including but not limited to the above 14 categories, such as secretory proteins, membrane proteins, signal transduction-related proteins, glycoprotein-related proteins, or transcription-related proteins. When searching OMIM using these keywords, the proteins are revealed to be involved in a great number of diseases (the results of the OMIM search for secretory and membrane proteins are shown below as an Example). Associations between proteins related to signal transduction or transcription and diseases are reported in "Transcription Factor Research-1999" (Fujii, Tamura, Morohashi, Kageyama, and Satake edit, (1999) Jikken-Igaku Zoukan, Vol.17, No.3), and "Gene Medicine" (1999) Vol.3, No.2). As another example and as described in "Biology of Cancer", many proteins are involved in cancers, including enzymes and/or metabolism-related proteins, cytoskeleton-related proteins, cell division and/or cell proliferation-related proteins as well as secretory proteins, membrane proteins, signal transduction-related proteins, glycoprotein-related proteins, transcription-related proteins, (S. Matsubara, 1992) of Life Science series (Shokabo). As the above example clearly demonstrates, not only disease-related proteins but also secretory proteins, membrane proteins, signal transduction-related proteins, glycoprotein-related proteins, and transcription-related proteins are often involved in diseases, and thus such proteins can be useful targets in the field of medical industry.

The results of the OMIM search for secretory and membrane proteins are shown below. The keywords used were:
(1) secretion protein,
(2) membrane protein,
(3) channel, and
(4) extracellular matrix.

Only the OMIM accession numbers are shown in the search results. The first 50 accession numbers displayed in the search results are provided. Using this number, data showing the relationship between a disease and a gene or protein can be seen. OMIM data is renewed daily.
1) Secretion protein
   When searching under these keywords, 436 genes were registered as being associated with disease. The OMIM numbers for 50 of these genes are as follows.
   *604667, *104760, *176860, *139320, *118910, *151675, *107400, *604029, #200100, *177061, *600946, *601693, *139250, *176880, *600998, *603850, *605083, *147572, *179513, *606055, *604028, *125950, *157147, *246700, *602926, *600560, *602421, *603215, 185860, *600174, *179512, *109270, *179511, *179510, *179509, *601146, *604710, *177020, *138120, *170280, *600626, *164160, *168470, *154545, *603831, *601652, *104311, *601489, *603062, *102720
2) Membrane protein
   When searching under these keywords, 1873 genes were registered as being associated with disease. The OMIM numbers for 50 of these genes are as follows.
   *130500, *605704, *305360, *153330, *109270, *173610, *170995, *120920, *170993, *309060, *104776, *602333, *605703, *602690, *605943, *159430, *600897, *606867, *133090, *601178, *602413, *602003, *604405, *605940, *603237, *109280, *606958, *600378, *606959, *602173, *107776, *602334, *125305, *602335, *309845, *601134, *605731, *606795, *185881, *607178, *603177, *154045, *603214, *603718, *606909, *600594, *603241, *606629, *603657, *600182
3) Channel (member of membrane proteins)
   When searching under these keywords, 449 genes were registered as being associated with disease. The OMIM numbers for 50 of these genes are as follows.
   *176266, *600724, *605427, *182390, *123825, *114208, *114206, *114205, *176267, *600053, *601784, *603749, *182392, *600937, *603415, *114204, *114209, *114207, *607370, *604528, *604527, *601011, *600760, *192500, *118425, *600228, *600359, *176261, *602235, *600761, *182389, *300008, *600877, *605692, *300338, *602232, *603537, *182391, *176263, *602343, *601328, *605874, *604385, *603939, *602208, *601534, *601958, *603220, *600504, *607368
4) Extracellular matrix
   When searching under these keywords, 267 genes were registered as being associated with disease. The OMIM numbers for 50 of these genes are as follows.
   *605912, *602201, *603479, *604633, *601418, *601548, *115437, *154870, *120361, *602285, *600754, *602262, *134797, *602261, *603320, *603321, *604871, *604629, *601807, #154700, *128239, *600310, *605470, *185250, *178990, *603767, *120360, *185261, *116935, *607056, *253700, *190180, *600985, *188826, *193300, *276901, *308700, *120150, *602109, *120324, *600514, #177170, #247100, #116920, #200610, *605127, *601313, *601652, *120180, *154790

In addition to these, various keywords shown in the above-mentioned categorizations or others can be used in an OMIM search to reveal involvement in disease.

Further, the use of nucleotide sequences of cDNAs of the present invention enables the expression frequency of genes corresponding to those cDNAs to be analyzed. Gene function can be predicted based on information obtained by expression frequency analysis.

There are several methods for analyzing the expression level of genes involved in disease. Differences in gene expression levels between diseased and normal tissues can be studied by analytical methods using, for example, Northern blotting, RT-PCR, DNA microarrays, etc. (Experimental Medicine, Vol.17, No. 8, 980-1056 (1999); Cell Engineering (additional volume) DNA Microarray and Advanced PCR Methods, Muramatsu & Nawa (eds.), Shujunsya (2000)). In addition to these analysis methods, computer analysis can be used to compare the nucleotide sequences of expressed genes, and hence to analyze expression frequency. For example, in the "BODYMAP" database, gene clones are randomly extracted from cDNA libraries of various tissues and/or cells, clones homologous to each other are assigned to a single cluster based on 3'-end nucleotide sequence homology information, genes are then classified into clusters, and the number of clones in each cluster is compared to gain information on expression frequency (http://bodymap.ims.u-tokyo.ac.jp/).

When these analytical methods result in observation of an explicit difference between gene expression levels in diseased tissues and normal tissues, it can be concluded that the gene is closely involved in the disease or disorder. When gene expression is explicitly different between normal cells and cells reproducing specific disease-associated features, even if they are not diseased tissues, it can be concluded that the gene is closely involved in a disease or disorder.

Of the 2,495 clones whose full-length nucleotide sequences were revealed, genes involved in a particular pathology or function were selected using the database shown below (see Example 7; "Expression frequency analysis in *silico*"). The database used in the analyses of the present invention contains the nucleotide sequences of 1,402,069 clones, a sufficiently large population for analysis. Sequence information in the database was obtained by randomly selecting cDNA clones from cDNA libraries derived from the various tissues and cells shown in Example 1, and determining the 5'-end sequences thereof.

The nucleotide sequence of each clone in this database was then categorized (clustered) based on nucleotide sequence homology determined with a search program. The number of clones belonging to each cluster of each library was determined and normalized; and the ratio of a certain gene in a cDNA library was determined. This analysis provided information on the expression frequency of a gene in the tissue or cell that was the source of the cDNA library.

In order to analyze the expression of genes corresponding to the nucleotide sequences of cDNAs of the present invention in tissues and cells, the libraries from the tissues or cells, which had been used in large-scale cDNA analyses, were taken as subjects for comparison of expression levels between different tissues or cells. Namely, expression frequency was analyzed by comparing the previously normalized values between tissues or cells from which were derived the 600 or more cDNA clones whose nucleotide sequences had been analyzed. This analysis showed that the cDNA clones corresponded to the genes involved in the pathologies and functions indicated below. Each value in Tables 2 to 24 indicated below represents a relative expression frequency; a higher value indicates a higher expression level. Genes included in these Tables do not indicate such a big difference between compared libraries, but when compared with other tissue- or gene-derived libraries based on Example 9, they indicate a significant difference. Thus, these genes are specific to a tissue or cell, and can be considered useful diagnostic markers for disease, as well as useful for analyzing molecular mechanisms.

### Osteoporosis-related genes

Osteoporosis is a pathology in which bones are easily broken owing to an overall decrease in bone components. The onset involves the balance between the functions of osteoblast producing bone and osteoclast absorbing bone, namely bone metabolism. Genes involved in the increase of osteoclasts differentiating from precursor cells of monocyte/macrophage line (Molecular Medicine 38. 642-648. (2001)) are thus genes involved in osteoporosis relevant to bone metabolism.

Nucleotide sequence-based analysis was carried out to identify genes whose expression frequencies were higher or lower in CD34+ cells (cells expressing glycoprotein CD34) treated with osteoclast differentiation factor (Molecular Medicine 38. 642-648. (2001)), compared to untreated CD34+ cells, which are precursor cells in monocyte/macrophage lines. The result of comparative analysis of frequency between the cDNA libraries prepared from the RNA of CD34+ cells (CD34C), and from the RNA of CD34+ cells treated with the osteoclast differentiation factor (D3OST, D60ST or D9OST) (Table 2), showed that the genes whose expression levels differed between the two were 15 and two clones indicated below.
BRACE3013780, BRAMY2047420, BRSTN2016470, CTONG3008894, D3OST2002182, D3OST2002648, D3OST3000169, PEBLM2005183, PUAEN2009655, TESTI4000014, TESTI4010851, TRACH2023299, TRACH2025535, TRACH3001427, UTERU2006137
HCHON2000508, TESTI2015626

These clones are involved in osteoporosis.

### Genes involved in neural cell differentiation

Genes involved in neural cell differentiation are useful for treating neurological disease. Genes with varying expression levels in response to induction of cellular differentiation in neural cells are thought to be involved in neurological disease.

A survey was performed for genes whose expression levels varied in response to induction of differentiation (stimulation by retinoic acid (RA) or growth inhibitor treatment after RA stimulation) in cultured cells of a neural strain, NT2. The result of comparative analysis of cDNA libraries derived from undifferentiated NT2 cells (NT2RM) and cells subjected to differentiation treatment (NT2RP, NT2RI or NT2NE) (Table 3) showed that the genes whose expression levels differed between the two were 174 and 30 clones indicated below.
BNGH42007788, BRACE1000186, BRACE2006319, BRACE2014306, BRACE2015058, BRACE2044286, BRACE3010428, BRAMY2044078, BRAWH2014645, BRAWH2014662, BRAWH3002574, BRAWH3003992, BRAWH3005981, BRAWH3007592, BRCAN2009432, BRCAN2016619, BRCAN2028355, BRHIP2001074, BRHIP2007741, BRHIP2014228, BRHIP2024146, BRHIP3007586, BRHIP3018797, BRTHA2003461, BRTHA3000633, BRTHA3003490, COLON2001721, CTONG1000087, CTONG2008233, CTONG2020638, CTONG2028124, CTONG3003905, CTONG3008894, CTONG3009028, CTONG3009239, DFNES2011499, FCBBF3001977, FEBRA1000030, FEBRA2006396, FEBRA2007801, HCHON2000028, HCHON2000244, HCHON2001084, HCHON2001217, HCHON2001548, HCHON2006250, HEART1000074, HHDPC1000118, HSYRA2009075, IMR322000127, IMR322001380, KIDNE2000665, KIDNE2002252, MESAN2006563, MESAN2012054, MESAN2015515, NT2NE2003252, NT2NE2005890, NT2NE2006531, NT2NE2006909, NT2NE2008060, NT2RI2003993, NT2RI2004618, NT2RI2005166, NT2RI2006686, NT2RI2008724, NT2RI2009855, NT2RI2011422, NT2RI2011683, NT2RI2012659, NT2RI2012990, NT2RI2013357, NT2RI2014247, NT2RI2014551, NT2RI2014733, NT2RI2016128, NT2RI2018311, NT2RI2018883, NT2RI2019751, NT2RI2023303, NT2RI2025909, NT2RI2025957, NT2RI2027081, NT2RI2027396, NT2RI3000622, NT2RI3001263, NT2RI3001515, NT2RI3002303, NT2RI3002842, NT2RI3002892, NT2RI3003031, NT2RI3003095, NT2RI3003162, NT2RI3003382, NT2RI3003409, NT2RI3004381, NT2RI3004510, NT2RI3005202, NT2RI3005403, NT2RI3005724, NT2RI3006132, NT2RI3006171, NT2RI3006284, NT2RI3006340, NT2RI3006376, NT2RI3006673, NT2RI3006796, NT2RI3007065, NT2RI3007158, NT2RI3007291, NT2RI3007543, NT2RI3007757, NT2RI3007978, NT2RI3008055, NT2RI3008162, NT2RI3008652, NT2RI3008697, NT2RI3008974, NT2RI3009158, NT2RP7000359, NT2RP7000466, NT2RP7004027, NT2RP7004123, NT2RP7005118, NT2RP7005529, NT2RP7005846, NT2RP7009030, NT2RP7009147, NT2RP7009867, NT2RP7010128, NT2RP7010599, NT2RP7011570, NT2RP7013795, NT2RP7014005, NT2RP7015512, NT2RP7017365, NT2RP7017474, NT2RP7017546, NT2RP8000137, NT2RP8000296, NT2RP8000483, NTONG2005969, OCBBF2007028, OCBBF2037068, PLACE7000514, PUAEN2007044, SPLEN2002467, SPLEN2006122, SPLEN2028914, SPLEN2031547, SYNOV4002346, SYNOV4007671, SYNOV4008440, TESOP2002273, TESTI2003573, TESTI4000014, TESTI4009286, TESTI4010851, TESTI4012702, TESTI4029671, TESTI4037156, THYMU3000133, TRACH1000205, TRACH2005811, TRACH2007834, TRACH2025535, TRACH3001427, TRACH3002192, TRACH3004721, TRACH3008093, TRACH3008535, TRACH3008713, UTERU2002410, UTERU2023175
ADRGL2000042, BRACE2003609, BRACE3003026, BRHIP3000017, CTONG2020411, FCBBF1000509, FCBBF3027854, FEBRA2028516, HCHON2000508, IMR322001879, NT2RI2005772, NT2RI2008952, NT2RI2009583, NT2RI2018448, NT2RI2027157, NT2RI3000174, NT2RI3001132, NT2RI3002557, NT2RI3005928, NT2RI3007167, NT2RI3007443, NT2RP7008435, NT2RP8000521, OCBBF2006987, PERIC2007068, TESTI2015626, TESTI4015442, TLIVE2002046, TRACH3000134, TUTER2000057

These genes are neurological disease-related genes.

### Genes involved in Alzheimer's disease

Alzheimer's disease is a cranial neurological disease characterized by memory loss. As the disease advances, patients can no longer support themselves and require nursing. Alzheimer's disease eventually leads to brain atrophication. Environmental factors such as stress, and vascular factors such as hypertension and cholesterolemia, are assumed but not confirmed to contribute to the onset of Alzheimer's disease. Genes whose expression levels differ between normal brain tissues and tissues affected with Alzheimer's disease are expected to be involved in Alzheimer's disease. Such genes can be used to elucidate the disease's onset mechanism and in genetic diagnosis. cDNA libraries derived from the cerebral cortex of Alzheimer patients (BRALZ and BRASW) and a library derived from the whole tissues of a normal brain (BRAWH) were analyzed and compared (Table 4). Genes whose expression levels differed between the two were the 250 clones and 41 clones listed below.
ASTRO1000009, BLADE2008398, BRACE1000186, BRACE1000258, BRACE1000533, BRACE2005457, BRACE2010489, BRACE2014657, BRACE2035381, BRACE2044286, BRACE2045954, BRACE3000787, BRACE3003192, BRACE3005499, BRACE3007480, BRACE3009237, BRACE3009724, BRACE3009747, BRACE3010428, BRACE3011271, BRACE3011421, BRACE3012364, BRACE3022769, BRACE3026735, BRACE3031838, BRALZ2011796, BRALZ2012183, BRALZ2012848, BRALZ2014484, BRALZ2016085, BRALZ2016498, BRALZ2017359, BRAMY2003008, BRAMY2005052, BRAMY2019300, BRAMY2019963, BRAMY2036567, BRAMY2037823, BRAMY2040592, BRAMY3002803, BRAMY3004224, BRAMY3005091, BRASW1000053, BRASW1000125, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCOC2003213, BRCOC2014033, BRCOC2020142, BRHIP2000920, BRHIP2005719, BRHIP2007741, BRHIP2014228, BRHIP2024146, BRHIP2026288, BRHIP3000339, BRHIP3006683, BRHIP3007586, BRHIP3008405, BRHIP3018797, BRSSN2000684, BRSSN2011738, BRSSN2014299, BRSTN2008052, BRSTN2015015, BRSTN2016470, BRTHA1000311, BRTHA2008335, BRTHA3002427, BRTHA3003490, BRTHA3008520, BRTHA3017848, COLON2001721, CTONG2017500, CTONG2028124, CTONG3000657, CTONG3001123, CTONG3009328, FCBBF2001183, FCBBF3001977, FEBRA2007544, FEBRA2007801, FEBRA2020886, FEBRA2028618, HCASM2007047, HCHON2000244, HCHON2000626, HCHON2001217, HCHON2002676, HCHON2006250, HEART1000074, HHDPC1000118, HLUNG2002465, IMR322000127, IMR322001380, IMR322002035, KIDNE2006580, MESAN2006563, MESAN2012054, MESTC1000042, NOVAR2001783, NT2NE2006909, NT2RI2008724, NT2RI2012659, NT2RI2014733, NT2RI3002892, NT2RI3006284, NT2RI3006673, NT2RI3007543, NT2RI3008055, NT2RP7005529, NT2RP7009147, NT2RP7014005, NT2RP7017474, NTONG2005969, OCBBF2001794, OCBBF2006005, OCBBF2006764, OCBBF2007028, OCBBF2007114, OCBBF2010140, OCBBF2021286, OCBBF2023162, OCBBF2024850, OCBBF2028935, OCBBF2036743, OCBBF2038317, OCBBF3000483, OCBBF3008230, PEBLM2004666, PLACE6001185, PUAEN2005930, PUAEN2006701, PUAEN2007044, PUAEN2009655, SMINT2001818, SPLEN2028914, SPLEN2031424, SPLEN2031547, SPLEN2034781, SPLEN2036932, SYNOV2014400, SYNOV4002346, SYNOV4002883, SYNOV4007430, SYNOV4007671, SYNOV4008440, TESOP2002273, TESOP2002451, TESTI4000014, TESTI4000209, TESTI4001100, TESTI4006137, TESTI4008797, TESTI4009286, TESTI4010851, TESTI4013817, TESTI4014694, TESTI4021478, TESTI4022936, TESTI4024420, TESTI4027821, THYMU2001090, THYMU2033308, THYMU2035735, THYMU2039315, THYMU3001234, THYMU3008171, TKIDN2009641, TKIDN2009889, TKIDN2015788, TRACH1000205, TRACH2001549, TRACH2005811, TRACH2006049, TRACH2007834, TRACH2008300, TRACH2025535, TRACH3001427, TRACH3002192, TRACH3004068, TRACH3004721, TRACH3005294, TRACH3007479, TRACH3008093, TRACH3009455, UTERU2005621, UTERU2006115, UTERU2019706, UTERU2023039, UTERU2026203, UTERU3005230, UTERU3007640, UTERU3009871
ADRGL2000042, BLADE2006830, BRACE2003609, BRALZ2017844, BRAMY3004800, BRAWH1000369, BRAWH2006207, BRAWH2006395, BRAWH2008993, BRAWH2009393, BRAWH2010552, BRAWH3007441, BRAWH3009017, BRHIP2005271, BRHIP3000017, BRHIP3026052, BRTHA2018443, BRTHA3003000, CTONG2020374, CTONG2020378, CTONG2024031, FCBBF1000509, FEBRA2001990, FEBRA2006519, FEBRA2028516, HCHON2000743, IMR322001879, NT2RI2009583, OCBBF2008144, PERIC2007068, PUAEN2006335, SPLEN2039379, TESTI4001984, TESTI4008058, TESTI4025268, TESTI4032090, THYMU3000360, TLIVE2002046, TRACH3000134, UTERU2021820, UTERU2028734

These genes are involved in Alzheimer's disease.

### Genes involved in Parkinson's disease

Parkinson's disease is a cranial neurological disease characterized by impaired production of the neurotransmitter dopamine in the substantia nigra in the brain. This results in dyskinesia, such as hand tremors, and impaired body movement due to muscular rigidity. Normally, the number of brain neurons gradually decreases with age. However, compared to healthy people, patients with Parkinson's disease experience a rapid and marked decrease in the number of neurons in their substantia nigra. Genes whose expression levels differ between tissues of the whole brain and the nigra are expected to be involved in Parkinson's disease. These genes exhibit nigra-specific alterations in their expression levels, and can be used to elucidate the disease onset mechanism and in gene diagnosis. cDNA libraries derived from the substantia nigra (BRSSN) and a library derived from whole tissues of a normal brain (BRAWH) were analyzed and compared (Table 5). Genes whose expression levels differed between the two were the 250 clones and 40 clones listed below.
ASTRO1000009, BLADE2008398, BRACE1000186, BRACE1000258, BRACE1000533, BRACE2005457, BRACE2010489, BRACE2014657, BRACE2035381, BRACE2044286, BRACE2045954, BRACE3000787, BRACE3003192, BRACE3005499, BRACE3007480, BRACE3009237, BRACE3009724, BRACE3009747, BRACE3010428, BRACE3011271, BRACE3011421, BRACE3012364, BRACE3013780, BRACE3022769, BRACE3026735, BRACE3031838, BRALZ2011796, BRAMY2003008, BRAMY2005052, BRAMY2019300, BRAMY2019963, BRAMY2036567, BRAMY2037823, BRAMY2040592, BRAMY2047420, BRAMY3002803, BRAMY3004224, BRAMY3005091, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCOC2003213, BRCOC2014033, BRCOC2020142, BRHIP2000920, BRHIP2005719, BRHIP2007741, BRHIP2014228, BRHIP2024146, BRHIP3000339, BRHIP3006683, BRHIP3007586, BRHIP3008405, BRHIP3018797, BRSSN2000684, BRSSN2003086, BRSSN2004496, BRSSN2004719, BRSSN2006892, BRSSN2008549, BRSSN2008797, BRSSN2011262, BRSSN2011738, BRSSN2013874, BRSSN2014299, BRSSN2014424, BRSSN2014556, BRSSN2018581, BRSSN2018925, BRSTN2008052, BRSTN2015015, BRSTN2016470, BRTHA1000311, BRTHA2003461, BRTHA2008335, BRTHA3002427, BRTHA3003490, BRTHA3008520, BRTHA3017848, COLON2001721, CTONG2017500, CTONG2028124, CTONG3000657, CTONG3001123, CTONG3009328, FCBBF2001183, FCBBF3001977, FEBRA2007544, FEBRA2007801, FEBRA2020886, FEBRA2024136, FEBRA2025427, FEBRA2028618, HCASM2007047, HCHON2000244, HCHON2000626, HCHON2001217, HCHON2002676, HCHON2006250, HEART1000074, HHDPC1000118, HLUNG2002465, IMR322000127, IMR322002035, KIDNE2006580, MESAN2006563, MESAN2012054, MESTC1000042, NOVAR2001783, NT2NE2006909, NT2RI2008724, NT2RI2012659, NT2RI2014733, NT2RI3002892, NT2RI3006284, NT2RI3006673, NT2RI3007543, NT2RI3008055, NT2RP7005529, NT2RP7009147, NT2RP7014005, NT2RP7017474, OCBBF2001794, OCBBF2006005, OCBBF2006764, OCBBF2007028, OCBBF2010140, OCBBF2021286, OCBBF2024850, OCBBF2028935, OCBBF2036743, OCBBF2038317, OCBBF3000483, OCBBF3008230, PEBLM2004666, PLACE6001185, PUAEN2005930, PUAEN2006701, PUAEN2007044, PUAEN2009655, SPLEN2028914, SPLEN2031424, SPLEN2031547, SPLEN2034781, SPLEN2036932, SYNOV2014400, SYNOV4002346, SYNOV4002883, SYNOV4007430, SYNOV4008440, TESOP2002451, TESTI4000014, TESTI4000209, TESTI4001100, TESTI4006137, TESTI4008797, TESTI4009286, TESTI4010851, TESTI4013817, TESTI4014694, TESTI4021478, TESTI4022936, TESTI4024420, TESTI4027821, TESTI4037156, THYMU2001090, THYMU2033308, THYMU2035735, THYMU2039315, THYMU3001234, THYMU3008171, TKIDN2009641, TKIDN2009889, TKIDN2015788, TRACH1000205, TRACH2001549, TRACH2005811, TRACH2006049, TRACH2007834, TRACH2008300, TRACH2025535, TRACH3001427, TRACH3002192, TRACH3004721, TRACH3005294, TRACH3007479, TRACH3008093, TRACH3009455, UTERU2006115, UTERU2019706, UTERU2023039, UTERU2026203, UTERU3005230, UTERU3007640, UTERU3009871
ADRGL2000042, BLADE2006830, BRACE2003609, BRAMY3004800, BRAWH1000369, BRAWH2006207, BRAWH2006395, BRAWH2008993, BRAWH2009393, BRAWH2010552, BRAWH3007441, BRAWH3009017, BRHIP2005271, BRHIP3000017, BRTHA2018443, BRTHA3003000, CTONG2020374, CTONG2020378, CTONG2024031, FCBBF1000509, FEBRA2001990, FEBRA2006519, FEBRA2028516, HCHON2000743, IMR322001879, NT2RI2009583, OCBBF2008144, PERIC2007068, PUAEN2006335, SPLEN2039379, TESTI2015626, TESTI4001984, TESTI4008058, TESTI4025268, TESTI4032090, THYMU3000360, TLIVE2002046, TRACH3000134, UTERU2021820, UTERU2028734

These genes are involved in Parkinson's disease.

### Genes involved in short-term memory and dementia

In the brain, the hippocampus is a highly important memory-related area. The hippocampus functions to establish a memory by judging whether acquired information is necessary, and then accumulating the memory in another area of the brain. According to clinical findings, patients can retain a new memory for only about five minutes with an abnormal, or at worst without a hippocampus. Some dementia patients are presumed to have hippocampus abnormalities. Thus, genes whose expression levels differ between tissues of the whole brain and the hippocampus are expected to be involved in memory or dementia. Such genes can be used to elucidate the mechanism underlying memory, and in gene diagnosis. cDNA libraries derived from the hippocampus (BRHIP) and from the whole tissues of a normal brain (BRAWH) were analyzed and compared (Table 6). Genes whose expression levels differed between the two were the 370 clones and 59 clones listed below.
ASTRO1000009, BLADE2001371, BLADE2008398, BNGH42007788, BRACE1000186, BRACE1000258, BRACE1000533, BRACE2005457, BRACE2010489, BRACE2014657, BRACE2015058, BRACE2018762, BRACE2030341, BRACE2035381, BRACE2044286, BRACE2045954, BRACE3000787, BRACE3003192, BRACE3005499, BRACE3007480, BRACE3009237, BRACE3009724, BRACE3009747, BRACE3010428, BRACE3011271, BRACE3011421, BRACE3012364, BRACE3018963, BRACE3022769, BRACE3026735, BRACE3031838, BRALZ2011796, BRAMY2003008, BRAMY2005052, BRAMY2019300, BRAMY2019963, BRAMY2031317, BRAMY2036567, BRAMY2037823, BRAMY2040592, BRAMY2044078, BRAMY3002803, BRAMY3004224, BRAMY3005091, BRAMY3009811, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2020710, BRCAN2028355, BRCOC2003213, BRCOC2014033, BRCOC2020142, BRHIP2000691, BRHIP2000819, BRHIP2000826, BRHIP2000920, BRHIP2001074, BRHIP2001805, BRHIP2001927, BRHIP2002122, BRHIP2002172, BRHIP2002346, BRHIP2003242, BRHIP2003786, BRHIP2003917, BRHIP2004312, BRHIP2004359, BRHIP2004814, BRHIP2004883, BRHIP2005236, BRHIP2005354, BRHIP2005600, BRHIP2005719, BRHIP2005752, BRHIP2005932, BRHIP2006800, BRHIP2007616, BRHIP2007741, BRHIP2009340, BRHIP2009414, BRHIP2009474, BRHIP2013699, BRHIP2014228, BRHIP2021615, BRHIP2022221, BRHIP2024146, BRHIP2024165, BRHIP2026061, BRHIP2026288, BRHIP2029176, BRHIP2029393, BRHIP3000339, BRHIP3000526, BRHIP3001283, BRHIP3006683, BRHIP3007483, BRHIP3007586, BRHIP3008183, BRHIP3008313, BRHIP3008344, BRHIP3008405, BRHIP3008565, BRHIP3008598, BRHIP3008997, BRHIP3009099, BRHIP3009448, BRHIP3011241, BRHIP3013765, BRHIP3013897, BRHIP3015751, BRHIP3016213, BRHIP3018797, BRHIP3020182, BRHIP3024118, BRHIP3024533, BRHIP3024725, BRHIP3025161, BRHIP3025702, BRHIP3026097, BRHIP3027137, BRHIP3027854, BRSSN2000684, BRSSN2004719, BRSSN2008549, BRSSN2011738, BRSSN2014299, BRSTN2008052, BRSTN2015015, BRSTN2016470, BRSTN2018083, BRTHA1000311, BRTHA2002442, BRTHA2008335, BRTHA3000297, BRTHA3001721, BRTHA3002427, BRTHA3003490, BRTHA3005046, BRTHA3008520, BRTHA3008778, BRTHA3009090, BRTHA3015910, BRTHA3017848, COLON2001721, CTONG1000087, CTONG1000088, CTONG1000467, CTONG2000042, CTONG2008233, CTONG2009423, CTONG2017500, CTONG2019788, CTONG2028124, CTONG3000657, CTONG3001123, CTONG3001370, CTONG3002412, CTONG3004072, CTONG3008894, CTONG3009239, CTONG3009328, DFNES2011499, FCBBF2001183, FCBBF3001977, FEBRA2000253, FEBRA2007544, FEBRA2007801, FEBRA2008287, FEBRA2010719, FEBRA2020886, FEBRA2028618, HCASM2007047, HCHON2000028, HCHON2000244, HCHON2000626, HCHON2001217, HCHON2002676, HCHON2005921, HCHON2006250, HEART1000074, HEART2007031, HHDPC1000118, HLUNG2002465, HLUNG2003003, IMR322000127, IMR322001380, IMR322002035, KIDNE2005543, KIDNE2006580, MESAN2006563, MESAN2012054, MESTC1000042, NOVAR2001783, NT2NE2006909, NT2RI2008724, NT2RI2012659, NT2RI2014733, NT2RI2018311, NT2RI3001515, NT2RI3002892, NT2RI3004510, NT2RI3005724, NT2RI3006284, NT2RI3006673, NT2RI3007291, NT2RI3007543, NT2RI3008055, NT2RP7005529, NT2RP7009147, NT2RP7014005, NT2RP7017474, OCBBF2001794, OCBBF2003819, OCBBF2006005, OCBBF2006151, OCBBF2006764, OCBBF2007028, OCBBF2007068, OCBBF2010140, OCBBF2020741, OCBBF2021286, OCBBF2024719, OCBBF2024850, OCBBF2028935, OCBBF2036743, OCBBF2038317, OCBBF3000296, OCBBF3000483, OCBBF3008230, PEBLM2004666, PLACE6001185, PUAEN2005930, PUAEN2006701, PUAEN2007044, PUAEN2009655, SPLEN2010912, SPLEN2012624, SPLEN2028914, SPLEN2031424, SPLEN2031547, SPLEN2034781, SPLEN2036932, SYNOV2014400, SYNOV4002346, SYNOV4002883, SYNOV4007430, SYNOV4008440, TESOP2002451, TESTI2049246, TESTI4000014, TESTI4000209, TESTI4001100, TESTI4006137, TESTI4008797, TESTI4009286, TESTI4010377, TESTI4010851, TESTI4010928, TESTI4011161, TESTI4013817, TESTI4014159, TESTI4014694, TESTI4014818, TESTI4021478, TESTI4022936, TESTI4024420, TESTI4027821, TESTI4037156, THYMU2001090, THYMU2023967, THYMU2025707, THYMU2031341, THYMU2033308, THYMU2035735, THYMU2037226, THYMU2039315, THYMU3001234, THYMU3001379, THYMU3004835, THYMU3007137, THYMU3008171, TKIDN2009641, TKIDN2009889, TKIDN2015788, TRACH1000205, TRACH2001549, TRACH2005811, TRACH2006049, TRACH2007834, TRACH2008300, TRACH2025535, TRACH3000014, TRACH3001427, TRACH3002192, TRACH3004721, TRACH3005294, TRACH3007479, TRACH3008093, TRACH3009455, TUTER1000122, TUTER2000904, UTERU2004929, UTERU2006115, UTERU2019706, UTERU2021163, UTERU2023039, UTERU2026203, UTERU2030213, UTERU3001572, UTERU3003135, UTERU3005230, UTERU3007640, UTERU3009259, UTERU3009871
ADRGL2000042, BLADE2006830, BRACE2003609, BRAMY3004800, BRAWH1000369, BRAWH2006207, BRAWH2006395, BRAWH2008993, BRAWH2009393, BRAWH2010552, BRAWH3007441, BRAWH3009017, BRHIP2002722, BRHIP2003272, BRHIP2005271, BRHIP2005724, BRHIP2006617, BRHIP2008389, BRHIP2012360, BRHIP2017553, BRHIP2026877, BRHIP3000017, BRHIP3000240, BRHIP3008314, BRHIP3026052, BRTHA2018443, BRTHA3003000, CTONG2020374, CTONG2020378, CTONG2024031, CTONG3004726, FCBBF1000509, FEBRA2001990, FEBRA2006519, FEBRA2028516, HCHON2000743, IMR322001879, NT2RI2009583, OCBBF2006987, OCBBF2008144, OCBBF2030116, PERIC2007068, PUAEN2006335, SPLEN2039379, TESTI2015626, TESTI4000214, TESTI4001984, TESTI4008058, TESTI4013894, TESTI4025268, TESTI4025547, TESTI4026207, TESTI4032090, THYMU3000360, TLIVE2002046, TRACH3000134, UTERU2008040, UTERU2021820, UTERU2028734

These genes are involved in memory and dementia.

### Genes involved in equilibrium sense and movement function

The cerebellum is the center of equilibrium sense, muscular movement, and motor learning. This area is thought to be involved in motor control, and smooth movements are achieved unconsciously due to cerebellum action. Recent studies have elucidated that the cerebellum participates in not only simple movements but also in establishing higher-order movements such as reading and writing. Thus, genes whose expression levels differ between tissues of the whole brain and the cerebellum are expected to be involved in equilibrium sense or motor function, which can be useful for elucidating the molecular mechanism controlled by the brain. cDNA libraries derived from the cerebellum (BRACE) and from the whole tissues of a normal brain (BRAWH) were analyzed and compared (Table 7). Genes whose expression levels differed between the two were the 488 clones and 66 clones listed below.
ADRGL2009146, ADRGL2012038, ASTRO1000009, ASTRO2003960, BLADE1000176, BLADE2004089, BLADE2008398, BRACE1000186, BRACE1000258, BRACE1000533, BRACE1000572, BRACE2003639, BRACE2005457, BRACE2006319, BRACE2008594, BRACE2010489, BRACE2011747, BRACE2014306, BRACE2014475, BRACE2014657, BRACE2015058, BRACE2015314, BRACE2016981, BRACE2018762, BRACE2024627, BRACE2026836, BRACE2027258, BRACE2027970, BRACE2028970, BRACE2029112, BRACE2029849, BRACE2030326, BRACE2030341, BRACE2030884, BRACE2031154, BRACE2031389, BRACE2031527, BRACE2031531, BRACE2031899, BRACE2032044, BRACE2032329, BRACE2032385, BRACE2032538, BRACE2032823, BRACE2033720, BRACE2035381, BRACE2035441, BRACE2036005, BRACE2036096, BRACE2036830, BRACE2036834, BRACE2037847, BRACE2038114, BRACE2038329, BRACE2038551, BRACE2039249, BRACE2039327, BRACE2039475, BRACE2039734, BRACE2040138, BRACE2040325, BRACE2041009, BRACE2041200, BRACE2041264, BRACE2042550, BRACE2043142, BRACE2043248, BRACE2043349, BRACE2043665, BRACE2044286, BRACE2044816, BRACE2044949, BRACE2045300, BRACE2045428, BRACE2045596, BRACE2045772, BRACE2045947, BRACE2045954, BRACE2046251, BRACE2046295, BRACE2047011, BRACE2047350, BRACE2047377, BRACE2047385, BRACE3000071, BRACE3000697, BRACE3000787, BRACE3000840, BRACE3000973, BRACE3001002, BRACE3001217, BRACE3001391, BRACE3001595, BRACE3001754, BRACE3002298, BRACE3002390, BRACE3002508, BRACE3003004, BRACE3003192, BRACE3003595, BRACE3003698, BRACE3004058, BRACE3004113, BRACE3004150, BRACE3004358, BRACE3004435, BRACE3004772, BRACE3004783, BRACE3004843, BRACE3004880, BRACE3005145, BRACE3005225, BRACE3005430, BRACE3005499, BRACE3006185, BRACE3006226, BRACE3006462, BRACE3006872, BRACE3007322, BRACE3007472, BRACE3007480, BRACE3007559, BRACE3007625, BRACE3007642, BRACE3007767, BRACE3008036, BRACE3008092, BRACE3008137, BRACE3008384, BRACE3008720, BRACE3008772, BRACE3009090, BRACE3009237, BRACE3009297, BRACE3009377, BRACE3009574, BRACE3009701, BRACE3009708, BRACE3009724, BRACE3009747, BRACE3010397, BRACE3010428, BRACE3011271, BRACE3011421, BRACE3011505, BRACE3012364, BRACE3012930, BRACE3013119, BRACE3013576, BRACE3013740, BRACE3013780, BRACE3014005, BRACE3014068, BRACE3014231, BRACE3014317, BRACE3014807, BRACE3015027, BRACE3015121, BRACE3015262, BRACE3015521, BRACE3015894, BRACE3016884, BRACE3018308, BRACE3018963, BRACE3019055, BRACE3019084, BRACE3020194, BRACE3020286, BRACE3020594, BRACE3022769, BRACE3023912, BRACE3024073, BRACE3024659, BRACE3024662, BRACE3025153, BRACE3025457, BRACE3025531, BRACE3025630, BRACE3026008, BRACE3026075, BRACE3026735, BRACE3027242, BRACE3027326, BRACE3027478, BRACE3030103, BRACE3031838, BRACE3032983, BRACE3040856, BRACE3045033, BRALZ2011796, BRAMY2003008, BRAMY2005052, BRAMY2019300, BRAMY2019963, BRAMY2020058, BRAMY2030098, BRAMY2031317, BRAMY2036567, BRAMY2037823, BRAMY2039872, BRAMY2040592, BRAMY2044078, BRAMY2047420, BRAMY3002620, BRAMY3002803, BRAMY3004224, BRAMY3005091, BRAMY3005932, BRAMY4000229, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2009432, BRCAN2010376, BRCAN2015371, BRCAN2020710, BRCOC2003213, BRCOC2007034, BRCOC2014033, BRCOC2020142, BRHIP2000920, BRHIP2004359, BRHIP2005719, BRHIP2005752, BRHIP2007741, BRHIP2013699, BRHIP2014228, BRHIP2024146, BRHIP3000339, BRHIP3006683, BRHIP3007586, BRHIP3008313, BRHIP3008405, BRHIP3018797, BRSSN2000684, BRSSN2006892, BRSSN2011262, BRSSN2011738, BRSSN2014299, BRSTN2008052, BRSTN2010750, BRSTN2015015, BRSTN2016470, BRTHA1000311, BRTHA2008335, BRTHA2008955, BRTHA2011194, BRTHA3001721, BRTHA3002427, BRTHA3003490, BRTHA3008520, BRTHA3009090, BRTHA3017848, COLON2001721, CTONG2008233, CTONG2017500, CTONG2028124, CTONG3000657, CTONG3001123, CTONG3005813, CTONG3008894, CTONG3009328, DFNES2011499, FCBBF2001183, FCBBF3001977, FEBRA2006396, FEBRA2007544, FEBRA2007708, FEBRA2007801, FEBRA2008287, FEBRA2020886, FEBRA2021966, FEBRA2026984, FEBRA2028618, HCASM2007047, HCHON2000244, HCHON2000626, HCHON2001217, HCHON2002676, HCHON2005921, HCHON2006250, HEART1000074, HHDPC1000118, HLUNG2002465, IMR322000127, IMR322001380, IMR322002035, KIDNE2000665, KIDNE2006580, MESAN2006563, MESAN2012054, MESTC1000042, NB9N41000340, NESOP2001752, NOVAR2001783, NT2NE2006909, NT2RI2005166, NT2RI2008724, NT2RI2012659, NT2RI2014733, NT2RI2019751, NT2RI3002892, NT2RI3003382, NT2RI3004510, NT2RI3005724, NT2RI3006284, NT2RI3006673, NT2RI3007291, NT2RI3007543, NT2RI3008055, NT2RP7004123, NT2RP7005529, NT2RP7009147, NT2RP7010599, NT2RP7014005, NT2RP7017474, NTONG2005969, OCBBF2001794, OCBBF2003819, OCBBF2006005, OCBBF2006151, OCBBF2006764, OCBBF2007028, OCBBF2010140, OCBBF2020343, OCBBF2020741, OCBBF2021286, OCBBF2022351, OCBBF2024850, OCBBF2025527, OCBBF2028935, OCBBF2036743, OCBBF2038317, OCBBF3000483, OCBBF3007516, OCBBF3008230, PEBLM2004666, PERIC2000889, PLACE6001185, PUAEN2002489, PUAEN2005930, PUAEN2006701, PUAEN2007044, PUAEN2009655, SPLEN2010912, SPLEN2012624, SPLEN2027268, SPLEN2028914, SPLEN2031424, SPLEN2031547, SPLEN2034781, SPLEN2036932, SPLEN2037194, SYNOV2014400, SYNOV4002346, SYNOV4002883, SYNOV4007430, SYNOV4007671, SYNOV4008440, TESOP2002273, TESOP2002451, TESOP2002950, TESTI1000330, TESTI4000014, TESTI4000209, TESTI4000349, TESTI4001100, TESTI4001561, TESTI4006137, TESTI4008797, TESTI4009286, TESTI4010851, TESTI4011161, TESTI4013675, TESTI4013817, TESTI4014159, TESTI4014306, TESTI4014694, TESTI4021478, TESTI4022936, TESTI4024420, TESTI4027821, TESTI4037156, TESTI4046819, THYMU2001090, THYMU2016523, THYMU2023967, THYMU2030264, THYMU2033308, THYMU2035735, THYMU2039315, THYMU2039780, THYMU3001083, THYMU3001234, THYMU3003309, THYMU3006485, THYMU3008171, TKIDN2009641, TKIDN2009889, TKIDN2015788, TRACH1000205, TRACH2001549, TRACH2005811, TRACH2006049, TRACH2007834, TRACH2008300, TRACH2025535, TRACH3001427, TRACH3002192, TRACH3004721, TRACH3005294, TRACH3006038, TRACH3006412, TRACH3007479, TRACH3008093, TRACH3009455, TUTER2000904, UTERU2002410, UTERU2006115, UTERU2007520, UTERU2019706, UTERU2023039, UTERU2026203, UTERU3000226, UTERU3001572, UTERU3005230, UTERU3005460, UTERU3005970, UTERU3006308, UTERU3007419, UTERU3007640, UTERU3007913, UTERU3009871 ADRGL2000042, BLADE2006830, BRACE2002589, BRACE2003609, BRACE2009318, BRACE2011677, BRACE2029396, BRACE2037299, BRACE2039823, BRACE2039832, BRACE2043105, BRACE3001058, BRACE3001113, BRACE3003026, BRACE3003053, BRACE3009127, BRACE3010076, BRACE3015829, BRACE3021148, BRAMY3004800, BRAWH1000369, BRAWH2006207, BRAWH2006395, BRAWH2008993, BRAWH2009393, BRAWH2010552, BRAWH3007441, BRAWH3009017, BRCOC2019841, BRHIP2005271, BRHIP3000017, BRHIP3000240, BRTHA2018443, BRTHA3003000, CTONG2020374, CTONG2020378, CTONG2024031, FCBBF1000509, FEBRA2001990, FEBRA2006519, FEBRA2028516, HCHON2000743, IMR322001879, NT2RI2009583, NT2RP8000521, OCBBF2008144, OCBBF2011669, PERIC2007068, PUAEN2006335, SPLEN2039379, SYNOV2021953, TESTI2015626, TESTI4001984, TESTI4008058, TESTI4013894, TESTI4025268, TESTI4032090, THYMU2004284, THYMU2040925, THYMU3000360, TLIVE2002046, TRACH3000134, UTERU2008040, UTERU2011220, UTERU2021820, UTERU2028734

These genes are involved in equilibrium sense or motor function.

### Genes involved in signaling from sensory organs

The thalamus is an area which comprises many neurons strongly connected to the cerebrum, and which transmits sensory information from the spinal cord or such to the responsible area of the cerebrum. The thalamus also controls the direction of movement from the cerebrum. For example, the thalamus resolves vision into the elements of size, shape, and color, and resolves sound into volume and sweetness or harshness to the ear, and then transmits this information to the sensory area of the cerebral cortex. Thus, genes whose expression levels differ between tissues of the whole brain and the thalamus are expected to be involved in signaling from sensory organs. These genes can be used to elucidate the molecular mechanism underlying signaling controlled by the brain. cDNA libraries derived from the thalamus (BRTHA) and from whole tissues of a normal brain (BRAWH) were analyzed and compared (Table 8). Genes whose expression levels differed between the two were the 412 clones and 56 clones listed below.
ASTRO1000009, ASTRO3000482, BLADE2008398, BRACE1000186, BRACE1000258, BRACE1000533, BRACE2005457, BRACE2010489, BRACE2014306, BRACE2014657, BRACE2015058, BRACE2031154, BRACE2035381, BRACE2044286, BRACE2045954, BRACE3000787, BRACE3003192, BRACE3005499, BRACE3007480, BRACE3008384, BRACE3009237, BRACE3009724, BRACE3009747,.BRACE3010397, BRACE3010428, BRACE3011271, BRACE3011421, BRACE3012364, BRACE3022769, BRACE3026735, BRACE3027478, BRACE3031838, BRALZ2011796, BRAMY2003008, BRAMY2005052, BRAMY2019300, BRAMY2019963, BRAMY2028914, BRAMY2031317, BRAMY2036567, BRAMY2037823, BRAMY2040592, BRAMY2044078, BRAMY3002803, BRAMY3004224, BRAMY3005091, BRAMY4000229, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2006297, BRCOC2003213, BRCOC2014033, BRCOC2020142, BRHIP2000819, BRHIP2000920, BRHIP2005719, BRHIP2007741, BRHIP2009474, BRHIP2013699, BRHIP2014228, BRHIP2022221, BRHIP2024146, BRHIP3000339, BRHIP3006683, BRHIP3007586, BRHIP3008405, BRHIP3018797, BRSSN2000684, BRSSN2008549, BRSSN2008797, BRSSN2011738, BRSSN2014299, BRSTN2004863, BRSTN2008052, BRSTN2015015, BRSTN2016470, BRTHA1000311, BRTHA2000855, BRTHA2001462, BRTHA2002115, BRTHA2002281, BRTHA2002376, BRTHA2002442, BRTHA2002493, BRTHA2002608, BRTHA2002808, BRTHA2003030, BRTHA2003110, BRTHA2003116, BRTHA2003461, BRTHA2004821, BRTHA2004978, BRTHA2005579, BRTHA2005956, BRTHA2006075, BRTHA2006146, BRTHA2006194, BRTHA2007122, BRTHA2007422, BRTHA2007603, BRTHA2008316, BRTHA2008335, BRTHA2008527, BRTHA2008535, BRTHA2008955, BRTHA2009311, BRTHA2009846, BRTHA2009972, BRTHA2010073, BRTHA2010608, BRTHA2010884, BRTHA2010907, BRTHA2011194, BRTHA2011351, BRTHA2011500, BRTHA2011641, BRTHA2012392, BRTHA2012562, BRTHA2012980, BRTHA2013262, BRTHA2013460, BRTHA2013707, BRTHA2014792, BRTHA2014828, BRTHA2015406, BRTHA2015478, BRTHA2015696, BRTHA2015878, BRTHA2016215, BRTHA2016496, BRTHA2016543, BRTHA2017353, BRTHA2017985, BRTHA2018165, BRTHA2018344, BRTHA2018591, BRTHA2018624, BRTHA2018707, BRTHA2019014, BRTHA2019022, BRTHA2019048, BRTHA3000273, BRTHA3000297, BRTHA3000633, BRTHA3001721, BRTHA3002401, BRTHA3002427, BRTHA3002933, BRTHA3003074, BRTHA3003343, BRTHA3003449, BRTHA3003474, BRTHA3003490, BRTHA3004475, BRTHA3005046, BRTHA3006856, BRTHA3007113, BRTHA3007148, BRTHA3007319, BRTHA3007769, BRTHA3008143, BRTHA3008310, BRTHA3008386, BRTHA3008520, BRTHA3008778, BRTHA3009037, BRTHA3009090, BRTHA3009291, BRTHA3010366, BRTHA3013884, BRTHA3015815, BRTHA3015910, BRTHA3016845, BRTHA3016917, BRTHA3017047, BRTHA3017589, BRTHA3017848, BRTHA3018514, BRTHA3018617, BRTHA3018656, BRTHA3019105, COLON2001721, CTONG1000087, CTONG2008233, CTONG2017500, CTONG2019788, CTONG2023021, CTONG2028124, CTONG3000657, CTONG3001123, CTONG3008894, CTONG3009028, CTONG3009239, CTONG3009328, FCBBF2001183, FCBBF3001977, FCBBF3021576, FEBRA2007544, FEBRA2007801, FEBRA2008287, FEBRA2008360, FEBRA2020886, FEBRA2028618, HCASM2007047, HCHON2000028, HCHON2000212, HCHON2000244, HCHON2000626, HCHON2001084, HCHON2001217, HCHON2002676, HCHON2005921, HCHON2006250, HEART1000074, HEART2007031, HHDPC1000118, HLUNG2001996, HLUNG2002465, IMR322000127, IMR322001380, IMR322002035, KIDNE2002252, KIDNE2005543, KIDNE2006580, KIDNE2011314, MESAN2006563, MESAN2012054, MESTC1000042, NOVAR2001783, NT2NE2006909, NT2RI2008724, NT2RI2012659, NT2RI2014733, NT2RI3002842, NT2RI3002892, NT2RI3005403, NT2RI3006284, NT2RI3006673, NT2RI3007543, NT2RI3008055, NT2RP7004123, NT2RP7005529, NT2RP7009147, NT2RP7014005, NT2RP7017474, NTONG2005969, NTONG2008088, OCBBF2001794, OCBBF2006005, OCBBF2006764, OCBBF2007028, OCBBF2010140, OCBBF2020639, OCBBF2021286, OCBBF2024719, OCBBF2024850, OCBBF2028935, OCBBF2036743, OCBBF2038317, OCBBF3000483, OCBBF3008230, PEBLM2004666, PLACE6001185, PUAEN2002489, PUAEN2005930, PUAEN2006701, PUAEN2007044, PUAEN2009655, RECTM2001347, SKMUS2000757, SPLEN2006122, SPLEN2010912, SPLEN2025491, SPLEN2028914, SPLEN2031424, SPLEN2031547, SPLEN2032154, SPLEN2034781, SPLEN2036821, SPLEN2036932, SYNOV1000374, SYNOV2014400, SYNOV4002346, SYNOV4002883, SYNOV4007430, SYNOV4007671, SYNOV4008440, TESOP2002451, TESTI2049246, TESTI4000014, TESTI4000209, TESTI4001100, TESTI4002290, TESTI4006137, TESTI4008797, TESTI4009286, TESTI4010851, TESTI4012702, TESTI4013817, TESTI4014159, TESTI4014694, TESTI4021478, TESTI4022936, TESTI4024420, TESTI4027821, TESTI4037156, THYMU2001090, THYMU2025707, THYMU2032825, THYMU2033308, THYMU2033787, THYMU2035735, THYMU2039315, THYMU2040975, THYMU3001234, THYMU3001379, THYMU3004835, THYMU3008171, TKIDN2009641, TKIDN2009889, TKIDN2015788, TLIVE2001327, TRACH1000205, TRACH2001549, TRACH2005811, TRACH2006049, TRACH2007834, TRACH2008300, TRACH2023299, TRACH2025535, TRACH3001427, TRACH3002192, TRACH3004068, TRACH3004721, TRACH3005294, TRACH3007479, TRACH3008093, TRACH3009455, TSTOM1000135, TUTER2000904, UTERU2002410, UTERU2006115, UTERU2019706, UTERU2019940, UTERU2023039, UTERU2023175, UTERU2026203, UTERU2030280, UTERU3000899, UTERU3001571, UTERU3001572, UTERU3004709, UTERU3005230, UTERU3005907, UTERU3007640, UTERU3009871 ADRGL2000042, BLADE2006830, BRACE2003609, BRAMY3004800, BRAWH1000369, BRAWH2006207, BRAWH2006395, BRAWH2008993, BRAWH2009393, BRAWH2010552, BRAWH3007441, BRAWH3009017, BRHIP2005271, BRHIP3000017, BRTHA2002133, BRTHA2002702, BRTHA2007060, BRTHA2010033, BRTHA2011321, BRTHA2013426, BRTHA2013610, BRTHA2016318, BRTHA2017364, BRTHA2017972, BRTHA2018011, BRTHA2018443, BRTHA3000296, BRTHA3003000, BRTHA3008826, CTONG2008721, CTONG2020374, CTONG2020378, CTONG2024031, FCBBF1000509, FEBRA2001990, FEBRA2006519, FEBRA2028516, HCHON2000743, HSYRA2005628, IMR322001879, NT2RI2009583, OCBBF2008144, PERIC2007068, PUAEN2006335, SPLEN2016932, SPLEN2039379, SYNOV2006620, TESTI4001984, TESTI4008058, TESTI4025268, TESTI4032090, THYMU3000360, TLIVE2002046, TRACH3000134, UTERU2021820, UTERU2028734

These genes are involved in signaling from sensory organs.

### Genes involved in emotional reaction

The amygdala is the center of emotion in the brain. Information passing through the amygdala induces an emotional reaction, for example, panic or fear. When a strong fear reaction is produced due to the emotional evaluation of stimulus in the amygdala, the amygdala transmits an alert signal to each area of the brain. This results in various reactions such as sweating palms, palpitation, elevated blood pressure, and rapid secretion of adrenaline. In other words, the amygdala transmits signals which cause the body to be on the alert and is a tissue involved in a kind of defense instinct. Thus, genes whose expression levels differ between tissues of the whole brain and the amygdala are expected to be involved in emotional reaction. Such genes can be used to elucidate the molecular mechanism underlying emotional reaction, fear, or panic. cDNA libraries derived from the amygdale (BRAMY) and from whole tissues of a normal brain (BRAWH) were analyzed and compared (Table 9). Genes whose expression levels differed between the two were the 383 clones and 55 clones listed below.
ASTRO1000009, BLADE2008398, BRACE1000186, BRACE1000258, BRACE1000533, BRACE2005457, BRACE2006319, BRACE2010489, BRACE2014657, BRACE2015058, BRACE2027258, BRACE2030341, BRACE2031389, BRACE2035381, BRACE2044286, BRACE2045954, BRACE3000787, BRACE3000973, BRACE3003192, BRACE3005499, BRACE3007480, BRACE3008036, BRACE3009237, BRACE3009724, BRACE3009747, BRACE3010428, BRACE3011271, BRACE3011421, BRACE3012364, BRACE3013780, BRACE3022769, BRACE3026735, BRACE3027478, BRACE3031838, BRALZ2011796, BRAMY2001473, BRAMY2003008, BRAMY2004771, BRAMY2005052, BRAMY2017528, BRAMY2019300, BRAMY2019963, BRAMY2019985, BRAMY2020058, BRAMY2020270, BRAMY2021498, BRAMY2028856, BRAMY2028914, BRAMY2029602, BRAMY2030098, BRAMY2030109, BRAMY2030702, BRAMY2030703, BRAMY2030799, BRAMY2031317, BRAMY2031377, BRAMY2031442, BRAMY2032014, BRAMY2032242, BRAMY2032317, BRAMY2033003, BRAMY2033116, BRAMY2033267, BRAMY2033594, BRAMY2034185, BRAMY2034920, BRAMY2034993, BRAMY2036387, BRAMY2036396, BRAMY2036567, BRAMY2036699, BRAMY2036913, BRAMY2037823, BRAMY2038100, BRAMY2038484, BRAMY2038846, BRAMY2038904, BRAMY2039872, BRAMY2040478, BRAMY2040592, BRAMY2041261, BRAMY2041378, BRAMY2041542, BRAMY2042612, BRAMY2042641, BRAMY2042760, BRAMY2042918, BRAMY2044078, BRAMY2044246, BRAMY2045036, BRAMY2046478, BRAMY2046742, BRAMY2046989, BRAMY2047169, BRAMY2047420, BRAMY2047676, BRAMY2047746, BRAMY2047751, BRAMY2047765, BRAMY2047884, BRAMY3000206, BRAMY3000213, BRAMY3001401, BRAMY3001794, BRAMY3002312, BRAMY3002620, BRAMY3002803, BRAMY3002805, BRAMY3004224, BRAMY3004672, BRAMY3004900, BRAMY3004919, BRAMY3005091, BRAMY3005932, BRAMY3006297, BRAMY3007206, BRAMY3007609, BRAMY3008466, BRAMY3008505, BRAMY3008650, BRAMY3009811, BRAMY3010411, BRAMY4000095, BRAMY4000229, BRAMY4000277, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2014881, BRCAN2017717, BRCOC2000670, BRCOC2003213, BRCOC2014033, BRCOC2020142, BRHIP2000920, BRHIP2005719, BRHIP2007741, BRHIP2014228, BRHIP2024146, BRHIP2026061, BRHIP3000339, BRHIP3001283, BRHIP3006683, BRHIP3007586, BRHIP3008405, BRHIP3018797, BRSSN2000684, BRSSN2004496, BRSSN2011738, BRSSN2014299, BRSTN2008052, BRSTN2010750, BRSTN2015015, BRSTN2016470, BRTHA1000311, BRTHA2008335, BRTHA2011641, BRTHA3001721, BRTHA3002427, BRTHA3003490, BRTHA3004475, BRTHA3008520, BRTHA3009090, BRTHA3017848, COLON2001721, CTONG1000087, CTONG2008233, CTONG2017500, CTONG2028124, CTONG3000657, CTONG3001123, CTONG3008894, CTONG3009239, CTONG3009328, FCBBF2001183, FCBBF3001977, FEBRA2007544, FEBRA2007801, FEBRA2008287, FEBRA2010719, FEBRA2020886, FEBRA2025427, FEBRA2028618, HCASM2007047, HCHON2000244, HCHON2000626, HCHON2001217, HCHON2002676, HCHON2006250, HCHON2008112, HEART1000074, HHDPC1000118, HLUNG2002465, HSYRA2009075, IMR322000127, IMR322001380, IMR322002035, KIDNE2000665, KIDNE2006580, MESAN2006563, MESAN2012054, MESAN2015515, MESTC1000042, NOVAR2001783, NT2NE2005890, NT2NE2006909, NT2RI2008724, NT2RI2012659, NT2RI2014733, NT2RI3001515, NT2RI3002892, NT2RI3005724, NT2RI3006284, NT2RI3006673, NT2RI3007543, NT2RI3008055, NT2RP7005529, NT2RP7009147, NT2RP7014005, NT2RP7017474, NTONG2005969, OCBBF1000254, OCBBF2001794, OCBBF2006005, OCBBF2006764, OCBBF2007028, OCBBF2007114, OCBBF2010140, OCBBF2021286, OCBBF2023162, OCBBF2024850, OCBBF2028935, OCBBF2035214, OCBBF2036743, OCBBF2038317, OCBBF3000483, OCBBF3008230, PEBLM2004666, PERIC2000889, PERIC2003720, PLACE6001185, PUAEN2005930, PUAEN2006701, PUAEN2007044, PUAEN2009174, PUAEN2009655, SKNMC2002402, SKNSH2000482, SPLEN2001599, SPLEN2002467, SPLEN2028914, SPLEN2029912, SPLEN2031424, SPLEN2031547, SPLEN2034781, SPLEN2036932, SPLEN2038345, SYNOV2014400, SYNOV4002346, SYNOV4002883, SYNOV4007430, SYNOV4007671, SYNOV4008440, TESOP2002451, TESTI2009474, TESTI4000014, TESTI4000209, TESTI4001100, TESTI4006137, TESTI4008797, TESTI4009286, TESTI4010851, TESTI4013817, TESTI4014159, TESTI4014694, TESTI4021478, TESTI4022936, TESTI4024420, TESTI4027821, TESTI4029836, TESTI4037156, TESTI4037188, THYMU2001090, THYMU2014353, THYMU2033308, THYMU2035735, THYMU2037226, THYMU2039315, THYMU3001234, THYMU3001379, THYMU3004835, THYMU3008171, TKIDN2009641, TKIDN2009889, TKIDN2015788, TLIVE2004320, TRACH1000205, TRACH2001549, TRACH2001684, TRACH2005811, TRACH2006049, TRACH2007834, TRACH2008300, TRACH2025344, TRACH2025535, TRACH2025911, TRACH3001427, TRACH3002192, TRACH3004068, TRACH3004721, TRACH3005294, TRACH3007479, TRACH3008093, TRACH3009455, TUTER2000904, UTERU2002410, UTERU2004929, UTERU2006115, UTERU2007520, UTERU2019706, UTERU2023039, UTERU2026203, UTERU3001572, UTERU3001766, UTERU3005230, UTERU3007640, UTERU3009517, UTERU3009871
ADRGL2000042, BLADE2006830, BRACE2003609, BRACE2039823, BRAMY2019111, BRAMY2035070, BRAMY2035449, BRAMY2035718, BRAMY2038516, BRAMY2039341, BRAMY2040159, BRAMY2041434, BRAMY2045471, BRAMY3004800, BRAWH1000369, BRAWH2006207, BRAWH2006395, BRAWH2008993, BRAWH2009393, BRAWH2010552, BRAWH3007441, BRAWH3009017, BRHIP2005271, BRHIP3000017, BRTHA2018443, BRTHA3003000, CTONG2020374, CTONG2020378, CTONG2024031, FCBBF1000509, FEBRA2001990, FEBRA2006519, FEBRA2028516, HCHON2000508, HCHON2000743, IMR322001879, NT2RI2009583, OCBBF2008144, PERIC2007068, PUAEN2006335, SPLEN2039379, TESTI2015626, TESTI2026647, TESTI4001984, TESTI4008058, TESTI4013894, TESTI4025268, TESTI4032090, THYMU3000360, TKIDN2018926, TLIVE2002046, TRACH3000134, UTERU2008040, UTERU2021820, UTERU2028734

These genes are involved in emotional reaction.

### Cancer-related genes

Cancer tissues are assumed to express a distinct set of genes distinct from normal tissues, and thus expression of these genes can contribute to carcinogenesis in tissues and cells. Thus, genes whose expression patterns in cancer tissues differ from those in normal tissues are cancer-related genes. A search was carried out for genes whose expression levels in cancer tissues differed from those in normal tissues.

The result of comparative analysis of cDNA libraries derived from breast tumor (TBAES) and normal breast (BEAST) (Table 10) showed that the genes whose expression levels differed between the two were the 35 and four clones listed below.
ASTRO2002842, BRACE3016884, BRSSN2011262, BRTHA2008335, HCHON2000244, HCHON2006250, HEART1000010, MESAN2012054, NT2RP7000466, NT2RP7009147, OCBBF2021020, PEBLM2002749, PEBLM2004666, SPLEN2001599, SPLEN2031547, STOMA1000189, TBAES2001171, TBAES2001220, TBAES2001229, TBAES2001258, TBAES2001492, TBAES2001751, TBAES2002197, TBAES2003550, TBAES2004055, TBAES2005157, TBAES2005543, TBAES2006568, TBAES2007964, TESTI4000014, TESTI4037156, TRACH3002192, TRACH3004068, TSTOM2000553, UTERU2002410
BRAWH2006395, NT2RI2009583, STOMA2004893, TBAES2000932

The result of comparative analysis of cDNA libraries derived from cervical tumor (TCERX) and normal cervical duct (CERVX) (Table 11) showed that the genes whose expression levels differed between the two were twelve and two clones listed below.
BLADE2007666, BRAMY2047420, BRCAN2007409, BRSTN2016470, CERVX1000042, CERVX2002006, MESAN2006563, PROST2018090, TCERX2000613, TESTI4037156, THYMU2031341, UTERU2004688 CERVX2002013, NT2RI2009583

The result of comparative analysis of cDNA libraries derived from colon tumor (TCOLN) and normal colon (COLON) (Table 12) showed that the genes whose expression levels differed between the two were the 24 and four clones listed below.
BRACE3015027, BRAMY2040592, BRSTN2016470, COLON1000030, COLON2000470, COLON2000568, COLON2001721, COLON2002443, COLON2002520, COLON2003043, COLON2004478, COLON2005126, COLON2005772, COLON2006282, COLON2009499, OCBBF2028935, PLACE7000514, RECTM2000433, SYNOV4007671, TCOLN2002278, TESTI2052693, TESTI4037156, THYMU2031368, TRACH2025535 CTONG1000113, NT2RI2009583, NT2RI2018448, TESTI2015626

The result of comparative analysis of cDNA libraries derived from esophageal tumor (TESOP) and normal esophagus (NESOP) (Table 13) showed that the genes whose expression levels differed between the two were the 56 and ten clones listed below.
BRACE2030341, BRAMY2047420, BRHIP2003917, BRTHA2003461, CTONG2013178, D3OST3000169, FEBRA2025427, HCHON2000244, HHDPC1000118, NESOP2000744, NESOP2001433, NESOP2001656, NESOP2001694, NESOP2001752, NESOP2002738, NT2RI3006284, NT2RP7009147, PLACE6019932, SYNOV2005216, TESOP1000127, TESOP2000801, TESOP2001122, TESOP2001166, TESOP2001345, TESOP2001605, TESOP2001818, TESOP2001849, TESOP2001865, TESOP2001953, TESOP2002273, TESOP2002451, TESOP2002489, TESOP2002539, TESOP2002950, TESOP2003273, TESOP2003753, TESOP2004114, TESOP2005285, TESOP2005485, TESOP2005579, TESOP2006041, TESOP2006060, TESOP2006068, TESOP2006670, TESOP2006746, TESOP2007052, TESOP2007262, TESOP2007636, TESOP2007688, TESOP2009121, TESOP2009555, TESTI4009286, TESTI4010851, THYMU2040975, TRACH2005811, UTERU2023175 CTONG2016942, NT2RI2009583, TESOP2000390, TESOP2001796, TESOP2005199, TESOP2006398, TESOP2006865, TESOP2007384, TESTI2015626, TRACH2000862

The result of comparative analysis of cDNA libraries derived from kidney tumor (TKIDN) and normal kidney (KIDNE) (Table 14) showed that the genes whose expression levels differed between the two were the 96 and 13 clones listed below.
ASTRO2018373, BRACE1000186, BRACE2014306, BRACE2015058, BRACE2016981, BRACE2043665, BRACE3008036, BRACE3010428, BRACE3022769, BRAMY2019963, BRAMY2044078, BRAWH1000127, BRAWH2001395, BRAWH2001671, BRAWH2013294, BRAWH2014645, BRHIP2024146, BRHIP3000339, BRSSN2000684, BRSSN2004719, BRSSN2018581, BRSTN2016470, BRTHA1000311, BRTHA3002427, CTONG1000087, CTONG2028124, CTONG3000657, CTONG3008894, FCBBF2001183, FEBRA2008287, HCASM2001301, HCHON2000028, HCHON2000244, HEART1000074, HHDPC1000118, HSYRA2008376, KIDNE1000064, KIDNE2000665, KIDNE2000722, KIDNE2000832, KIDNE2000846, KIDNE2001361, KIDNE2001847, KIDNE2002252, KIDNE2002991, KIDNE2003837, KIDNE2005543, KIDNE2006580, KIDNE2010264, KIDNE2011314, KIDNE2011532, KIDNE2011635, KIDNE2012945, KIDNE2013095, NESOP2001656, NTONG2005969, PEBLM2004666, SKMUS2000757, STOMA1000189, SYNOV4007671, TBAES2001258, TESTI4000014, TESTI4001100, TESTI4012702, TESTI4046819, THYMU2032014, TKIDN2000701, TKIDN2002424, TKIDN2002632, TKIDN2003044, TKIDN2004386, TKIDN2005934, TKIDN2005947, TKIDN2006525, TKIDN2006852, TKIDN2007667, TKIDN2009092, TKIDN2009641, TKIDN2009889, TKIDN2010934, TKIDN2012824, TKIDN2013287, TKIDN2014757, TKIDN2014771, TKIDN2015263, TKIDN2015788, TKIDN2016309, TKIDN2019116, TRACH2001443, TRACH2001684, TRACH2007834, TRACH2008300, TRACH3001427, UTERU2002410, UTERU2023175, UTERU3001572 BLADE2006830, BRALZ2017844, CTONG2028758, FCBBF1000509, FEBRA2001990, FEBRA2028516, HCHON2000508, MESAN2005303, NT2RI2009583, TESTI2015626, TKIDN2008778, TKIDN2012771, TKIDN2018926

The result of comparative analysis of cDNA libraries derived from liver tumor (TLIVE) and normal liver (LIVER) (Table 15) showed that the genes whose expression levels differed between the two were the 35 and six clones listed below.
BRCAN2018935, BRSTN2016470, BRTHA2012980, BRTHA3002427, CTONG2028124, LIVER2007415, NT2RI2008724, SPLEN2012624, SPLEN2033098, TESOP2002451, TLIVE2000023, TLIVE2001327, TLIVE2001828, TLIVE2001927, TLIVE2002336, TLIVE2002338, TLIVE2002690, TLIVE2003197, TLIVE2003225, TLIVE2003381, TLIVE2003970, TLIVE2004110, TLIVE2004320, TLIVE2004601, TLIVE2005180, TLIVE2006236, TLIVE2006529, TLIVE2007132, TLIVE2007528, TLIVE2007816, TLIVE2008083, TLIVE2008229, TLIVE2009541, UTERU2002410, UTERU2005621
LIVER2000247, NT2RI2009583, TESTI2015626, TLIVE2001684, TLIVE2002046, TLIVE2007607

The result of comparative analysis of cDNA libraries derived from lung tumor (TLUNG) and normal lung (HLUNG) (Table 16) showed that the genes whose expression levels differed between the two were the 47 and nine clones listed below.
BRCAN2021028, BRHIP2000819, BRSTN2016470, CTONG1000087, CTONG2028124, HCHON2006250, HEART1000074, HLUNG1000017, HLUNG2000014, HLUNG2001996, HLUNG2002465, HLUNG2002958, HLUNG2003003, HLUNG2003872, HLUNG2010464, HLUNG2011041, HLUNG2011298, HLUNG2012049, HLUNG2012287, HLUNG2012727, HLUNG2013204, HLUNG2013304, HLUNG2013622, HLUNG2013851, HLUNG2014262, HLUNG2014288, HLUNG2014449, HLUNG2015617, HLUNG2017350, HLUNG2017546, HLUNG2017806, HLUNG2019058, HSYRA2008376, KIDNE2012945, NT2RI2003993, NT2RP7013795, OCBBF3000483, SPLEN2028914, SPLEN2031547, SYNOV4007671, TESOP1000127, TESTI2003573, TESTI4000014, TESTI4037156, TRACH2005811, TRACH3004068, UTERU2005621
FEBRA2028516, HCHON2000508, HLUNG2013350, HLUNG2015418, HLUNG2015548, HLUNG2016862, NT2RI2009583, TESTI2015626, TRACH2019672

The result of comparative analysis of cDNA libraries derived from ovary tumor (TOVER) and normal ovary (NOVER) (Table 17A) showed the genes whose expression levels differed between the two were the 16 clones listed below.
CTONG2019788, FEBRA2014213, HLUNG2017546, NOVAR2000136, NOVAR2000710, NOVAR2000962, NOVAR2001108, NOVAR2001783, OCBBF3007516, TESTI2052693, TOVAR2000649, TOVAR2001281, TOVAR2001730, TOVAR2002247, TOVAR2002549, TRACH3004068

The result of comparative analysis of cDNA libraries derived from ovary tumor (TOVER) and normal ovary (NOVER) (Table 17B) showed the gene whose expression level was different between the two was the one clone described below. There was no difference in expression levels of this gene between normal and diseased ovaries, however there was a significant difference in the expression level in both tumorous and normal ovaries when compared with in other tissues. Thus, this gene is an ovary-specific gene and can be used as a diagnostic marker due to its association with disease.
TESTI2015626

The result of comparative analysis of cDNA libraries derived from stomach tumor (TSTOM) and normal stomach (STOMA) (Table 18) showed that the genes whose expression levels differed between the two were the 31 and five clones listed below.
BRACE2024627, BRAWH2014645, BRCAN2028355, BRHIP2000819, BRSTN2016470, BRTHA3003490, COLON2002443, HEART1000010, HLUNG2002465, KIDNE2001847, NT2RP7000466, PUAEN2006328, SMINT2001818, STOMA1000189, STOMA2003444, STOMA2004294, STOMA2004925, STOMA2008546, SYNOV4007671, TESTI4000014, TESTI4010851, THYMU2035735, TRACH2001549, TRACH2005811, TRACH2025535, TSTOM1000135, TSTOM2000442, TSTOM2000553, TSTOM2002672, UTERU2006115, UTERU3001572
FEBRA2008692, NT2RI2009583, STOMA2003158, STOMA2004893, TESTI2015626

The result of comparative analysis of cDNA libraries derived from uterine tumor (TUTER) and normal uterus (UTERU) (Table 19) showed that the genes whose expression levels differed between the two were the 244 and 34 clones listed below.
BNGH42007788, BRACE1000186, BRACE2030341, BRACE3008772, BRACE3009747, BRACE3010428, BRACE3027478, BRALZ2017359, BRAWH2014645, BRAWH3000314, BRAWH3001326, BRAWH3002574, BRAWH3002821, BRAWH3003727, BRAWH3007592, BRCAN2009432, BRCAN2028355, BRHIP3007586, BRHIP3008344, BRHIP3008565, BRSSN2006892, BRSTN2001067, BRSTN2016470, BRTHA2010608, BRTHA3003074, CTONG1000087, CTONG1000467, CTONG2028124, CTONG3001123, CTONG3008894, CTONG3009028, CTONG3009239, FCBBF3004847, FEBRA2026984, FEBRA2028618, HCHON2000244, HCHON2000418, HCHON2000626, HCHON2001084, HCHON2001217, HCHON2005921, HCHON2006250, HCHON2008444, HLUNG2003003, HSYRA2008376, KIDNE2002252, MESAN2014295, NOVAR2000710, NT2RI2008724, NT2RI2014247, NT2RI2014733, NT2RI3002892, NT2RI3005724, NT2RI3006284, NT2RI3006340, NT2RI3006673, NT2RI3007291, NT2RI3007543, NT2RP7004123, NT2RP7005529, NT2RP7009147, NT2RP7017474, OCBBF2007028, OCBBF2020741, OCBBF2024850, OCBBF2036743, OCBBF3000483, PLACE6001185, PLACE7000514, PUAEN2007044, PUAEN2009655, SKNSH2000482, SPLEN2006122, SPLEN2016554, SPLEN2031547, SPLEN2036932, STOMA1000189, STOMA2004925, SYNOV2017055, SYNOV4001395, SYNOV4002346, SYNOV4008440, TCERX2000613, TESOP2002273, TESTI4000014, TESTI4008797, TESTI4009286, TESTI4012702, TESTI4013675, TESTI4014159, TESTI4018886, TESTI4029671, TESTI4037156, THYMU2008725, THYMU2031890, THYMU2033070, THYMU2035735, THYMU3001472, TRACH1000205, TRACH2001443, TRACH2001549, TRACH2005811, TRACH2007834, TRACH2008300, TRACH3002192, TRACH3003379, TRACH3004068, TRACH3004721, TRACH3007479, TUTER1000122, TUTER2000425, TUTER2000904, TUTER2000916, TUTER2001387, TUTER2002729, UTERU1000024, UTERU1000031, UTERU1000148, UTERU1000249, UTERU1000337, UTERU1000339, UTERU2000649, UTERU2001409, UTERU2002410, UTERU2002841, UTERU2004688, UTERU2004929, UTERU2005004, UTERU2005621, UTERU2006115, UTERU2006137, UTERU2006568, UTERU2007444, UTERU2007520, UTERU2007724, UTERU2008347, UTERU2014678, UTERU2017762, UTERU2019491, UTERU2019681, UTERU2019706, UTERU2019940, UTERU2020491, UTERU2020718, UTERU2021163, UTERU2021380, UTERU2022020, UTERU2022981, UTERU2023039, UTERU2023175, UTERU2023651, UTERU2023712, UTERU2024002, UTERU2024656, UTERU2025025, UTERU2025645, UTERU2025891, UTERU2026025, UTERU2026090, UTERU2026203, UTERU2027591, UTERU2029953, UTERU2030213, UTERU2030280, UTERU2031084, UTERU2031268, UTERU2031521, UTERU2031703, UTERU2031851, UTERU2033375, UTERU2033382, UTERU2035114, UTERU2035323, UTERU2035328, UTERU2035331, UTERU2035452, UTERU2035469, UTERU2035503, UTERU2035745, UTERU2036089, UTERU2037361, UTERU2037577, UTERU2038251, UTERU3000226, UTERU3000645, UTERU3000665, UTERU3000828, UTERU3000899, UTERU3001059, UTERU3001240, UTERU3001542, UTERU3001571, UTERU3001572, UTERU3001585, UTERU3001652, UTERU3001766, UTERU3001988, UTERU3002209, UTERU3002218, UTERU3002383, UTERU3002667, UTERU3002731, UTERU3002768, UTERU3002786, UTERU3002993, UTERU3003116, UTERU3003135, UTERU3003178, UTERU3003465, UTERU3003523, UTERU3003776, UTERU3004523, UTERU3004616, UTERU3004709, UTERU3004992, UTERU3005049, UTERU3005205, UTERU3005230, UTERU3005460, UTERU3005585, UTERU3005907, UTERU3005970, UTERU3006008, UTERU3006308, UTERU3007134, UTERU3007419, UTERU3007640, UTERU3007913, UTERU3008660, UTERU3008671, UTERU3009259, UTERU3009490, UTERU3009517, UTERU3009690, UTERU3009871, UTERU3009979, UTERU3011063, UTERU3015086, UTERU3015500, UTERU3016789, UTERU3018081, UTERU3018154, UTERU3018616, UTERU3018711 ADRGL2000042, BRHIP3000017, CTONG2003348, CTONG2019822, CTONG2020378, CTONG2020411, CTONG2024031, FEBRA2028516, HCASM2008536, HCHON2000743, IMR322001879, MESAN2005303, NT2RI2009583, OCBBF2008144, PERIC2007068, SPLEN2039379, TESTI2015626, TESTI4013894, TUTER2000057, UTERU2004299, UTERU2008040, UTERU2011220, UTERU2019534, UTERU2021820, UTERU2028734, UTERU2032279, UTERU2033577, UTERU2035978, UTERU3000402, UTERU3000738, UTERU3001053, UTERU3014791, UTERU3015412, UTERU3017176

The result of comparative analysis of cDNA libraries derived from tongue cancer (CTONG) and normal tongue (NTONG) (Table 20) showed that the genes whose expression levels differed between the two were the 166 and 31 clones listed below.
BNGH42007788, BRACE1000186, BRACE2006319, BRACE3010428, BRACE3012364, BRAMY2020058, BRAMY3002803, BRAWH2001671, BRAWH2014645, BRAWH3002574, BRCAN2009432, BRCAN2015371, BRCAN2020710, BRHIP2004814, BRHIP3018797, BRTHA2003461, BRTHA3003490, CTONG1000087, CTONG1000088, CTONG1000288, CTONG1000302, CTONG1000341, CTONG1000467, CTONG1000488, CTONG1000508, CTONG1000540, CTONG2000042, CTONG2001877, CTONG2004062, CTONG2006798, CTONG2008233, CTONG2009423, CTONG2009531, CTONG2010803, CTONG2013178, CTONG2017500, CTONG2019248, CTONG2019652, CTONG2019704, CTONG2019788, CTONG2019833, CTONG2020127, CTONG2020522, CTONG2020638, CTONG2020806, CTONG2021132, CTONG2022153, CTONG2022601, CTONG2023021, CTONG2023512, CTONG2024206, CTONG2024749, CTONG2025496, CTONG2025516, CTONG2025900, CTONG2026920, CTONG2027327, CTONG2028124, CTONG2028687, CTONG3000084, CTONG3000657, CTONG3000686, CTONG3000707, CTONG3000896, CTONG3001123, CTONG3001370, CTONG3001420, CTONG3001560, CTONG3002020, CTONG3002127, CTONG3002412, CTONG3002674, CTONG3003179, CTONG3003483, CTONG3003652, CTONG3003654, CTONG3003737, CTONG3003905, CTONG3003972, CTONG3004072, CTONG3004712, CTONG3005325, CTONG3005648, CTONG3005713, CTONG3005813, CTONG3006067, CTONG3006186, CTONG3006650, CTONG3007444, CTONG3007528, CTONG3007586, CTONG3007870, CTONG3008252, CTONG3008258, CTONG3008496, CTONG3008566, CTONG3008639, CTONG3008831, CTONG3008894, CTONG3008951, CTONG3009028, CTONG3009227, CTONG3009239, CTONG3009328, CTONG3009385, FEBRA2007544, FEBRA2007801, FEBRA2021966, FEBRA2025427, HCHON2000028, HCHON2001217, HHDPC1000118, HSYRA2008376, KIDNE2001847, KIDNE2002252, MESAN2006563, NT2RI2008724, NT2RI2018883, NT2RI3000622, NT2RI3006284, NT2RI3006673, NT2RI3007543, NT2RI3007757, NT2RP7004123, NT2RP7009147, NT2RP7014005, NTONG2000413, NTONG2003852, NTONG2005277, NTONG2005969, NTONG2006354, NTONG2007249, NTONG2007517, NTONG2008088, NTONG2008672, OCBBF2001794, OCBBF2006151, PEBLM2004666, PEBLM2005183, SPLEN2002467, SPLEN2029912, SPLEN2031547, SYNOV4007671, SYNOV4008440, TBAES2002197, TESOP2002273, TESTI2009474, TESTI4000014, TESTI4000209, TESTI4008018, TESTI4009286, TESTI4010851, TESTI4012702, TESTI4013675, THYMU2031847, THYMU2033308, TLIVE2002690, TRACH2005811, TRACH2007059, TRACH2025535, TRACH3001427, TSTOM2000553, UTERU2005621, UTERU2017762, UTERU2023175, UTERU3001572
BLADE2006830, BRHIP3000017, CTONG1000113, CTONG2003348, CTONG2004000, CTONG2008721, CTONG2015596, CTONG2015633, CTONG2016942, CTONG2019822, CTONG2020374, CTONG2020378, CTONG2020411, CTONG2020974, CTONG2024031, CTONG2028758, CTONG3001501, CTONG3002552, CTONG3003598, CTONG3004550, CTONG3004726, CTONG3009287, FEBRA2008692, FEBRA2028516, HCHON2000508, NT2RI2009583, NTONG2008093, PERIC2007068, TESOP2007384, TLIVE2002046, TRACH2000862

These genes are involved in cancer.

Expression frequency analysis is used when searching for genes involved in development and differentiation, where the expression levels of genes in developing and/or differentiating tissues and/or cells are compared with those in adult tissues and/or cells. Genes involved in tissue development and/or differentiation are genes which participate in tissue construction and function expression. These genes are thus useful genes available for medicine aiming at regeneration of injured tissues.

By using information on gene expression frequency gained from the database of the nucleotide sequences of the 1,402,069 clones as described above, genes whose expression frequencies differed between developing and/or differentiating tissues and/or cells, and adult tissues and/or cells, were analyzed.

The result of comparative analysis of cDNA libraries derived from fetal brain (FCBBF, FEBRA or OCBBF) and adult brain (BRACE, BRALZ, BRAMY, BRAWH, BRCAN, BRCOC, BRHIP, BRSSN, BRSTN or BRTHA) (Table 21) showed that the genes whose expression levels differed between the two were the 1,035 and 139 clones listed below.
ADRGL2009146, ADRGL2012038, ADRGL2012179, ASTRO1000009, ASTRO2003960, ASTRO3000482, BLADE1000176, BLADE2001371, BLADE2004089, BLADE2008398, BNGH42007788, BRACE1000186, BRACE1000258, BRACE1000533, BRACE1000572, BRACE2003639, BRACE2005457, BRACE2006319, BRACE2008594, BRACE2010489, BRACE2011747, BRACE2014306, BRACE2014475, BRACE2014657, BRACE2015058, BRACE2015314, BRACE2016981, BRACE2018762, BRACE2024627, BRACE2026836, BRACE2027258, BRACE2027970, BRACE2028970, BRACE2029112, BRACE2029849, BRACE2030326, BRACE2030341, BRACE2030884, BRACE2031154, BRACE2031389, BRACE2031527, BRACE2031531, BRACE2031899, BRACE2032044, BRACE2032329, BRACE2032385, BRACE2032538, BRACE2032823, BRACE2033720, BRACE2035381, BRACE2035441, BRACE2036005, BRACE2036096, BRACE2036830, BRACE2036834, BRACE2037847, BRACE2038114, BRACE2038329, BRACE2038551, BRACE2039249, BRACE2039327, BRACE2039475, BRACE2039734, BRACE2040138, BRACE2040325, BRACE2041009, BRACE2041200, BRACE2041264, BRACE2042550, BRACE2043142, BRACE2043248, BRACE2043349, BRACE2043665, BRACE2044286, BRACE2044816, BRACE2044949, BRACE2045300, BRACE2045428, BRACE2045596, BRACE2045772, BRACE2045947, BRACE2045954, BRACE2046251, BRACE2046295, BRACE2047011, BRACE2047350, BRACE2047377, BRACE2047385, BRACE3000071, BRACE3000697, BRACE3000787, BRACE3000840, BRACE3000973, BRACE3001002, BRACE3001217, BRACE3001391, BRACE3001595, BRACE3001754, BRACE3002298, BRACE3002390, BRACE3002508, BRACE3003004, BRACE3003192, BRACE3003595, BRACE3003698, BRACE3004058, BRACE3004113, BRACE3004150, BRACE3004358, BRACE3004435, BRACE3004772, BRACE3004783, BRACE3004843, BRACE3004880, BRACE3005145, BRACE3005225, BRACE3005430, BRACE3005499, BRACE3006185, BRACE3006226, BRACE3006462, BRACE3006872, BRACE3007322, BRACE3007472, BRACE3007480, BRACE3007559, BRACE3007625, BRACE3007642, BRACE3007767, BRACE3008036, BRACE3008092, BRACE3008137, BRACE3008384, BRACE3008720, BRACE3008772, BRACE3009090, BRACE3009237, BRACE3009297, BRACE3009377, BRACE3009574, BRACE3009701, BRACE3009708, BRACE3009724, BRACE3009747, BRACE3010397, BRACE3010428, BRACE3011271, BRACE3011421, BRACE3011505, BRACE3012364, BRACE3012930, BRACE3013119, BRACE3013576, BRACE3013740, BRACE3013780, BRACE3014005, BRACE3014068, BRACE3014231, BRACE3014317, BRACE3014807, BRACE3015027, BRACE3015121, BRACE3015262, BRACE3015521, BRACE3015894, BRACE3016884, BRACE3018308, BRACE3018963, BRACE3019055, BRACE3019084, BRACE3020194, BRACE3020286, BRACE3020594, BRACE3022769, BRACE3023912, BRACE3024073, BRACE3024659, BRACE3024662, BRACE3025153, BRACE3025457, BRACE3025531, BRACE3025630, BRACE3026008, BRACE3026075, BRACE3026735, BRACE3027242, BRACE3027326, BRACE3027478, BRACE3030103, BRACE3031838, BRACE3032983, BRACE3040856, BRACE3045033, BRALZ2011796, BRALZ2012183, BRALZ2012848, BRALZ2014484, BRALZ2016085, BRALZ2016498, BRALZ2017359, BRAMY2001473, BRAMY2003008, BRAMY2004771, BRAMY2005052, BRAMY2017528, BRAMY2019300, BRAMY2019963, BRAMY2019985, BRAMY2020058, BRAMY2020270, BRAMY2021498, BRAMY2028856, BRAMY2028914, BRAMY2029602, BRAMY2030098, BRAMY2030109, BRAMY2030702, BRAMY2030703, BRAMY2030799, BRAMY2031317, BRAMY2031377, BRAMY2031442, BRAMY2032014, BRAMY2032242, BRAMY2032317, BRAMY2033003, BRAMY2033116, BRAMY2033267, BRAMY2033594, BRAMY2034185, BRAMY2034920, BRAMY2034993, BRAMY2036387, BRAMY2036396, BRAMY2036567, BRAMY2036699, BRAMY2036913, BRAMY2037823, BRAMY2038100, BRAMY2038484, BRAMY2038846, BRAMY2038904, BRAMY2039872, BRAMY2040478, BRAMY2040592, BRAMY2041261, BRAMY2041378, BRAMY2041542, BRAMY2042612, BRAMY2042641, BRAMY2042760, BRAMY2042918, BRAMY2044078, BRAMY2044246, BRAMY2045036, BRAMY2046478, BRAMY2046742, BRAMY2046989, BRAMY2047169, BRAMY2047420, BRAMY2047676, BRAMY2047746, BRAMY2047751, BRAMY2047765, BRAMY2047884, BRAMY3000206, BRAMY3000213, BRAMY3001401, BRAMY3001794, BRAMY3002312, BRAMY3002620, BRAMY3002803, BRAMY3002805, BRAMY3004224, BRAMY3004672, BRAMY3004900, BRAMY3004919, BRAMY3005091, BRAMY3005932, BRAMY3006297, BRAMY3007206, BRAMY3007609, BRAMY3008466, BRAMY3008505, BRAMY3008650, BRAMY3009811, BRAMY3010411, BRAMY4000095, BRAMY4000229, BRAMY4000277, BRASW1000125, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2002562, BRCAN2002856, BRCAN2002944, BRCAN2002948, BRCAN2003703, BRCAN2003746, BRCAN2003987, BRCAN2004355, BRCAN2005436, BRCAN2006063, BRCAN2006290, BRCAN2006297, BRCAN2006450, BRCAN2007144, BRCAN2007409, BRCAN2007426, BRCAN2008528, BRCAN2009203, BRCAN2009432, BRCAN2010376, BRCAN2011254, BRCAN2011602, BRCAN2012355, BRCAN2012481, BRCAN2013655, BRCAN2013660, BRCAN2014143, BRCAN2014602, BRCAN2014881, BRCAN2015371, BRCAN2015464, BRCAN2016433, BRCAN2016619, BRCAN2017442, BRCAN2017717, BRCAN2017905, BRCAN2018935, BRCAN2019387, BRCAN2020710, BRCAN2021028, BRCAN2024451, BRCAN2024563, BRCAN2025712, BRCAN2028355, BRCOC2000670, BRCOC2003213, BRCOC2007034, BRCOC2014033, BRCOC2016525, BRCOC2019934, BRCOC2020142, BRHIP2000691, BRHIP2000819, BRHIP2000826, BRHIP2000920, BRHIP2001074, BRHIP2001805, BRHIP2001927, BRHIP2002122, BRHIP2002172, BRHIP2002346, BRHIP2003242, BRHIP2003786, BRHIP2003917, BRHIP2004312, BRHIP2004359, BRHIP2004814, BRHIP2004883, BRHIP2005236, BRHIP2005354, BRHIP2005600, BRHIP2005719, BRHIP2005752, BRHIP2005932, BRHIP2006800, BRHIP2007616, BRHIP2007741, BRHIP2009340, BRHIP2009414, BRHIP2009474, BRHIP2013699, BRHIP2014228, BRHIP2021615, BRHIP2022221, BRHIP2024146, BRHIP2024165, BRHIP2026061, BRHIP2026288, BRHIP2029176, BRHIP2029393, BRHIP3000339, BRHIP3000526, BRHIP3001283, BRHIP3006683, BRHIP3007483, BRHIP3007586, BRHIP3008183, BRHIP3008313, BRHIP3008344, BRHIP3008405, BRHIP3008565, BRHIP3008598, BRHIP3008997, BRHIP3009099, BRHIP3009448, BRHIP3011241, BRHIP3013765, BRHIP3013897, BRHIP3015751, BRHIP3016213, BRHIP3018797, BRHIP3020182, BRHIP3024118, BRHIP3024533, BRHIP3024725, BRHIP3025161, BRHIP3025702, BRHIP3026097, BRHIP3027137, BRHIP3027854, BRSSN2000684, BRSSN2003086, BRSSN2004496, BRSSN2004719, BRSSN2006892, BRSSN2008549, BRSSN2008797, BRSSN2011262, BRSSN2011738, BRSSN2013874, BRSSN2014299, BRSSN2014424, BRSSN2014556, BRSSN2018581, BRSSN2018925, BRSTN2000872, BRSTN2001067, BRSTN2001613, BRSTN2002400, BRSTN2003835, BRSTN2004863, BRSTN2004987, BRSTN2005721, BRSTN2006865, BRSTN2007000, BRSTN2007284, BRSTN2008052, BRSTN2008283, BRSTN2008418, BRSTN2008457, BRSTN2010363, BRSTN2010500, BRSTN2010750, BRSTN2012320, BRSTN2012380, BRSTN2015015, BRSTN2016470, BRSTN2016678, BRSTN2017237, BRSTN2017771, BRSTN2018083, BRSTN2019129, BRTHA1000311, BRTHA2000855, BRTHA2001462, BRTHA2002115, BRTHA2002281, BRTHA2002376, BRTHA2002442, BRTHA2002493, BRTHA2002608, BRTHA2002808, BRTHA2003030, BRTHA2003110, BRTHA2003116, BRTHA2003461, BRTHA2004821, BRTHA2004978, BRTHA2005579, BRTHA2005956, BRTHA2006075, BRTHA2006146, BRTHA2006194, BRTHA2007122, BRTHA2007422, BRTHA2007603, BRTHA2008316, BRTHA2008335, BRTHA2008527, BRTHA2008535, BRTHA2008955, BRTHA2009311, BRTHA2009846, BRTHA2009972, BRTHA2010073, BRTHA2010608, BRTHA2010884, BRTHA2010907, BRTHA2011194, BRTHA2011351, BRTHA2011500, BRTHA2011641, BRTHA2012392, BRTHA2012562, BRTHA2012980, BRTHA2013262, BRTHA2013460, BRTHA2013707, BRTHA2014792, BRTHA2014828, BRTHA2015406, BRTHA2015478, BRTHA2015696, BRTHA2015878, BRTHA2016215, BRTHA2016496, BRTHA2016543, BRTHA2017353, BRTHA2017985, BRTHA2018165, BRTHA2018344, BRTHA2018591, BRTHA2018624, BRTHA2018707, BRTHA2019014, BRTHA2019022, BRTHA2019048, BRTHA3000273, BRTHA3000297, BRTHA3000633, BRTHA3001721, BRTHA3002401, BRTHA3002427, BRTHA3002933, BRTHA3003074, BRTHA3003343, BRTHA3003449, BRTHA3003474, BRTHA3003490, BRTHA3004475, BRTHA3005046, BRTHA3006856, BRTHA3007113, BRTHA3007148, BRTHA3007319, BRTHA3007769, BRTHA3008143, BRTHA3008310, BRTHA3008386, BRTHA3008520, BRTHA3008778, BRTHA3009037, BRTHA3009090, BRTHA3009291, BRTHA3010366, BRTHA3013884, BRTHA3015815, BRTHA3015910, BRTHA3016845, BRTHA3016917, BRTHA3017047, BRTHA3017589, BRTHA3017848, BRTHA3018514, BRTHA3018617, BRTHA3018656, BRTHA3019105, COLON2001721, CTONG1000087, CTONG1000088, CTONG1000467, CTONG2000042, CTONG2008233, CTONG2009423, CTONG2017500, CTONG2019248, CTONG2019788, CTONG2020522, CTONG2023021, CTONG2028124, CTONG3000657, CTONG3001123, CTONG3001370, CTONG3002412, CTONG3004072, CTONG3005813, CTONG3008894, CTONG3009028, CTONG3009239, CTONG3009328, DFNES2000146, DFNES2011239, DFNES2011499, FCBBF1000297, FCBBF2001183, FCBBF3001977, FCBBF3002163, FCBBF3003435, FCBBF3004502, FCBBF3004847, FCBBF3006171, FCBBF3007242, FCBBF3007540, FCBBF3008944, FCBBF3009888, FCBBF3012170, FCBBF3012288, FCBBF3013307, FCBBF3013846, FCBBF3021576, FCBBF3021940, FCBBF3023443, FCBBF3023895, FCBBF3025730, FCBBF3027717, FCBBF4000076, FEBRA1000030, FEBRA2000253, FEBRA2006396, FEBRA2007544, FEBRA2007708, FEBRA2007793, FEBRA2007801, FEBRA2008287, FEBRA2008311, FEBRA2008360, FEBRA2008468, FEBRA2010719, FEBRA2014213, FEBRA2015588, FEBRA2020484, FEBRA2020582, FEBRA2020668, FEBRA2020886, FEBRA2021339, FEBRA2021571, FEBRA2021908, FEBRA2021966, FEBRA2024136, FEBRA2024150, FEBRA2024343, FEBRA2024744, FEBRA2025427, FEBRA2026984, FEBRA2027082, FEBRA2027297, FEBRA2027352, FEBRA2028366, FEBRA2028477, FEBRA2028618, HCASM2007047, HCHON2000028, HCHON2000212, HCHON2000244, HCHON2000626, HCHON2001084, HCHON2001217, HCHON2002676, HCHON2005921, HCHON2006250, HCHON2007881, HCHON2008112, HEART1000074, HEART2007031, HHDPC1000118, HLUNG2001996, HLUNG2002465, HLUNG2003003, HSYRA2009075, IMR322000127, IMR322000917, IMR322001380, IMR322002035, KIDNE2000665, KIDNE2002252, KIDNE2005543, KIDNE2006580, KIDNE2011314, MESAN2006563, MESAN2012054, MESAN2015515, MESTC1000042, NB9N41000340, NESOP2001752, NHNPC2001223, NOVAR2001783, NT2NE2005890, NT2NE2006909, NT2NE2008060, NT2RI2003993, NT2RI2005166, NT2RI2008724, NT2RI2012659, NT2RI2014733, NT2RI2018311, NT2RI2019751, NT2RI3000622, NT2RI3001515, NT2RI3002842, NT2RI3002892, NT2RI3003382, NT2RI3004510, NT2RI3005403, NT2RI3005724, NT2RI3006284, NT2RI3006673, NT2RI3007291, NT2RI3007543, NT2RI3008055, NT2RP7004123, NT2RP7005529, NT2RP7009030, NT2RP7009147, NT2RP7010599, NT2RP7014005, NT2RP7015512, NT2RP7017474, NTONG2000413, NTONG2005969, NTONG2008088, OCBBF1000254, OCBBF2001794, OCBBF2002124, OCBBF2003819, OCBBF2004826, OCBBF2004883, OCBBF2005428, OCBBF2006005, OCBBF2006058, OCBBF2006151, OCBBF2006567, OCBBF2006764, OCBBF2007028, OCBBF2007068, OCBBF2007114, OCBBF2007428, OCBBF2007478, OCBBF2007610, OCBBF2008770, OCBBF2009788, OCBBF2009926, OCBBF2010140, OCBBF2010416, OCBBF2017516, OCBBF2019327, OCBBF2019823, OCBBF2020343, OCBBF2020453, OCBBF2020639, OCBBF2020741, OCBBF2020801, OCBBF2020838, OCBBF2021020, OCBBF2021286, OCBBF2021323, OCBBF2021788, OCBBF2022351, OCBBF2022574, OCBBF2023162, OCBBF2023643, OCBBF2024719, OCBBF2024781, OCBBF2024850, OCBBF2025028, OCBBF2025458, OCBBF2025527, OCBBF2025730, OCBBF2026645, OCBBF2027423, OCBBF2027478, OCBBF2028173, OCBBF2028935, OCBBF2029901, OCBBF2030354, OCBBF2030517, OCBBF2030574, OCBBF2030708, OCBBF2031167, OCBBF2031366, OCBBF2032590, OCBBF2032599, OCBBF2032611, OCBBF2032671, OCBBF2033869, OCBBF2035110, OCBBF2035214, OCBBF2035564, OCBBF2035885, OCBBF2035916, OCBBF2036476, OCBBF2036743, OCBBF2037068, OCBBF2037340, OCBBF2037398, OCBBF2037547, OCBBF2037598, OCBBF2037638, OCBBF2038317, OCBBF3000296, OCBBF3000483, OCBBF3002553, OCBBF3002600, OCBBF3003320, OCBBF3003592, OCBBF3004314, OCBBF3006802, OCBBF3007516, OCBBF3008230, OCBBF3009279, PEBLM2004666, PERIC2000889, PERIC2002766, PERIC2003720, PLACE6001185, PLACE6019385, PUAEN2002489, PUAEN2005930, PUAEN2006701, PUAEN2007044, PUAEN2009174, PUAEN2009655, RECTM2001347, SKMUS2000757, SKNMC2002402, SKNSH2000482, SMINT2001818, SPLEN2001599, SPLEN2002467, SPLEN2006122, SPLEN2010912, SPLEN2012624, SPLEN2025491, SPLEN2027268, SPLEN2028914, SPLEN2029912, SPLEN2031424, SPLEN2031547, SPLEN2032154, SPLEN2034781, SPLEN2036821, SPLEN2036932, SPLEN2037194, SPLEN2038345, SPLEN2042303, SYNOV1000374, SYNOV2005216, SYNOV2014400, SYNOV4002346, SYNOV4002883, SYNOV4007430, SYNOV4007671, SYNOV4008440, TESOP2001605, TESOP2002273, TESOP2002451, TESOP2002950, TESTI1000330, TESTI2003573, TESTI2009474, TESTI2049246, TESTI4000014, TESTI4000209, TESTI4000349, TESTI4001100, TESTI4001561, TESTI4002290, TESTI4002647, TESTI4005857, TESTI4006137, TESTI4006326, TESTI4008797, TESTI4009286, TESTI4010377, TESTI4010851, TESTI4010928, TESTI4011161, TESTI4012702, TESTI4013675, TESTI4013817, TESTI4014159, TESTI4014175, TESTI4014306, TESTI4014694, TESTI4014818, TESTI4019843, TESTI4021478, TESTI4022936, TESTI4024420, TESTI4027821, TESTI4029836, TESTI4037156, TESTI4037188, TESTI4046819, THYMU2001090, THYMU2011736, THYMU2014353, THYMU2016204, THYMU2016523, THYMU2023967, THYMU2025707, THYMU2030264, THYMU2031341, THYMU2031890, THYMU2032696, THYMU2032825, THYMU2033308, THYMU2033787, THYMU2034374, THYMU2035735, THYMU2037226, THYMU2039315, THYMU2039780, THYMU2040975, THYMU3001083, THYMU3001234, THYMU3001379, THYMU3003309, THYMU3004835, THYMU3006485, THYMU3007137, THYMU3008171, TKIDN2009641, TKIDN2009889, TKIDN2010934, TKIDN2013287, TKIDN2015788, TLIVE2001327, TLIVE2004320, TRACH1000205, TRACH2001443, TRACH2001549, TRACH2001684, TRACH2005811, TRACH2006049, TRACH2007834, TRACH2008300, TRACH2023299, TRACH2025344, TRACH2025535, TRACH2025911, TRACH3000014, TRACH3001427, TRACH3002192, TRACH3004068, TRACH3004721, TRACH3005294, TRACH3006038, TRACH3006412, TRACH3007479, TRACH3008093, TRACH3009455, TSTOM1000135, TUTER1000122, TUTER2000904, UTERU2002410, UTERU2004929, UTERU2005621, UTERU2006115, UTERU2007520, UTERU2014678, UTERU2019706, UTERU2019940, UTERU2021163, UTERU2023039, UTERU2023175, UTERU2026203, UTERU2030213, UTERU2030280, UTERU3000226, UTERU3000899, UTERU3001571, UTERU3001572, UTERU3001766, UTERU3003135, UTERU3004709, UTERU3005230, UTERU3005460, UTERU3005907, UTERU3005970, UTERU3006308, UTERU3007419, UTERU3007640, UTERU3007913, UTERU3009259, UTERU3009517, UTERU3009871
ADRGL2000042, BLADE2006830, BRACE2002589, BRACE2003609, BRACE2009318, BRACE2011677, BRACE2029396, BRACE2037299, BRACE2039823, BRACE2039832, BRACE2043105, BRACE3001058, BRACE3001113, BRACE3003026, BRACE3003053, BRACE3009127, BRACE3010076, BRACE3015829, BRACE3021148, BRALZ2017844, BRAMY2019111, BRAMY2035070, BRAMY2035449, BRAMY2035718, BRAMY2038516, BRAMY2039341, BRAMY2040159, BRAMY2041434, BRAMY2045471, BRAMY3004800, BRAWH1000369, BRAWH2006207, BRAWH2006395, BRAWH2008993, BRAWH2009393, BRAWH2010552, BRAWH3007441, BRAWH3009017, BRCAN2002473, BRCAN2002854, BRCAN2003070, BRCAN2014229, BRCOC2019841, BRHIP2002722, BRHIP2003272, BRHIP2005271, BRHIP2005724, BRHIP2006617, BRHIP2008389, BRHIP2012360, BRHIP2017553, BRHIP2026877, BRHIP3000017, BRHIP3000240, BRHIP3008314, BRHIP3026052, BRSTN2013354, BRTHA2002133, BRTHA2002702, BRTHA2007060, BRTHA2010033, BRTHA2011321, BRTHA2013426, BRTHA2013610, BRTHA2016318, BRTHA2017364, BRTHA2017972, BRTHA2018011, BRTHA2018443, BRTHA3000296, BRTHA3003000, BRTHA3008826, CTONG2008721, CTONG2020374, CTONG2020378, CTONG2020411, CTONG2024031, CTONG3004726, FCBBF1000509, FCBBF3010361, FCBBF3027854, FEBRA2000790, FEBRA2001990, FEBRA2006519, FEBRA2008692, FEBRA2014122, FEBRA2027609, FEBRA2028516, HCASM2003018, HCHON2000508, HCHON2000743, HCHON2004858, HSYRA2005628, IMR322001879, NT2RI2009583, NT2RP8000521, OCBBF2003327, OCBBF2005433, OCBBF2006987, OCBBF2008144, OCBBF2009583, OCBBF2011669, OCBBF2019684, OCBBF2020048, OCBBF2030116, OCBBF2032274, OCBBF2034637, OCBBF3002654, OCBBF3003761, OCBBF3004972, PERIC2007068, PUAEN2006335, SPLEN2016932, SPLEN2039379, SYNOV2006620, SYNOV2021953, TESTI1000266, TESTI2015626, TESTI2026647, TESTI4000214, TESTI4001984, TESTI4008058, TESTI4013894, TESTI4015442, TESTI4017714, TESTI4025268, TESTI4025547, TESTI4026207, TESTI4032090, THYMU2004284, THYMU2040925, THYMU3000360, TKIDN2018926, TLIVE2002046, TRACH3000134, UTERU2008040, UTERU2011220, UTERU2021820, UTERU2028734

The result of comparative analysis of cDNA libraries derived from fetal heart (FEHRT) and adult heart (HEART) (Table 22) showed that the genes whose expression levels differed between the two were the 34 and two clones listed below.
BRAMY2040592, BRAWH2001671, BRSTN2016470, CTONG2017500, CTONG2028124, CTONG3000657, D3OST3000169, FEBRA2008287, HCHON2000244, HCHON2000626, HEART1000010, HEART1000074, HEART1000088, HEART1000139, HEART2001680, HEART2001756, HEART2006131, HEART2006909, HEART2007031, HEART2010391, HEART2010492, HEART2010495, KIDNE2000665, NB9N41000340, NT2RI2003993, NT2RI3002892, OCBBF2024850, SKMUS2006394, SMINT2001818, TESTI4000209, TKIDN2015788, TRACH3002192, TRACH3005294, TRACH3007479
HEART2009680, THYMU2004284

The result of comparative analysis of cDNA libraries derived from fetal kidney (FEKID) and adult kidney (KIDNE) (Table 23) showed that the genes whose expression levels differed between the two were the 40 and two clones listed below.
BRACE2043665, BRACE3010428, BRSTN2016470, CTONG1000087, CTONG2028124, CTONG3008894, HCASM2003415, HCHON2000244, HEART1000074, HHDPC1000118, KIDNE1000064, KIDNE2000665, KIDNE2000722, KIDNE2000832, KIDNE2000846, KIDNE2001361, KIDNE2001847, KIDNE2002252, KIDNE2002991, KIDNE2003837, KIDNE2005543, KIDNE2006580, KIDNE2010264, KIDNE2011314, KIDNE2011532, KIDNE2011635, KIDNE2012945, KIDNE2013095, PEBLM2004666, PLACE6019385, STOMA1000189, SYNOV4007671, TBAES2001258, TESOP2002451, TESTI4000014, TESTI4012702, THYMU2032014, TRACH2001684, TRACH2007834, UTERU2023175 NT2RI2009583, OCBBF2008144

The result of comparative analysis of cDNA libraries derived from fetal lung (FELNG) and adult lung (HLUNG) (Table 24) showed that the genes whose expression levels differed between the two were the 51 and eight clones listed below.
BRAWH3007592, BRCAN2021028, BRHIP2000819, BRSTN2016470, CTONG1000087, CTONG2028124, HCASM2007047, HEART1000074, HLUNG1000017, HLUNG2000014, HLUNG2001996, HLUNG2002465, HLUNG2002958, HLUNG2003003, HLUNG2003872, HLUNG2010464, HLUNG2011041, HLUNG2011298, HLUNG2012049, HLUNG2012287, HLUNG2012727, HLUNG2013204, HLUNG2013304, HLUNG2013622, HLUNG2013851, HLUNG2014262, HLUNG2014288, HLUNG2014449, HLUNG2015617, HLUNG2017350, HLUNG2017546, HLUNG2017806, HLUNG2019058, HSYRA2008376, KIDNE2012945, NT2RI2003993, NT2RI3007543, OCBBF3000483, SMINT1000192, SPLEN2028914, SPLEN2031547, STOMA1000189, SYNOV4007671, TESOP1000127, TESTI2003573, TESTI4000014, TESTI4037156, TRACH2005811, TRACH3004068, UTERU2005621, UTERU2023175
FEBRA2028516, HCHON2000508, HLUNG2013350, HLUNG2015418, HLUNG2015548, HLUNG2016862, TESTI2015626, TRACH2019672

These genes are involved in regeneration of tissues and/or cells.

For example, if a polypeptide encoded by a cDNA of the present invention is a regulatory factor for cellular conditions such as growth and differentiation, it can be used for developing medicines as follows. The polypeptide or antibody provided by the present invention is injected into cells using microinjection, and then low molecular weight compounds and such can be screened by using, as an index, change in cellular conditions (such as growth and differentiation), or activation/inhibition of a particular gene in the cell. The screening can be performed as follows.

A polypeptide of the present invention is first expressed and purified as a recombinant. This purified polypeptide is then microinjected into cells such as various cell lines, or primary culture cells, and cellular changes such as growth and differentiation are examined. Alternatively, induction of a gene whose expression is known to be involved in the change of a particular cellular condition may be detected using mRNA or polypeptide amounts. Alternatively, the amount of an intracellular molecule (low molecular weight compounds, etc.) may be detected, where that amount is changed by the function of a gene product (polypeptide) known to influence change in a particular cellular condition. The compounds to be screened (including both low and high molecular compounds) can be added to culture media and screened using, as an index, their ability to change a cellular condition.

Cell lines introduced with a gene of the present invention can be used for screening, even without microinjection. If the product of a gene of the present invention is revealed to be involved in a particular change in cellular conditions, the change in that product can be used as an index for screening. Once a compound which can activate or inhibit the function of a polypeptide of the present invention has been developed using this screening, such a compound can be practically applied in medicines.

If a polypeptide encoded by a cDNA of the present invention is a secretory or membrane protein, or a protein involved in signal transduction, glycoproteins, transcription, or disease, it can be used in functional assays for developing medicines as described below.

In case of a membrane protein, the polypeptide is very likely to function as a receptor or ligand on the cell surface. Therefore, it is possible to reveal new ligand-receptor relationships by screening membrane proteins of the present invention, based on binding activity with known or new ligands or receptors. Screening can be performed according to known methods.

For example, a ligand against a polypeptide of the present invention can be screened in the following manner. Namely, a ligand that binds to a specific polypeptide can be screened using a method comprising the steps of: (a) contacting a test sample with a polypeptide of the present invention, or partial peptide thereof, or cells expressing such, and (b) selecting a test sample that binds to said polypeptide, said partial peptide, or said cells.

Screening using cells expressing receptor polypeptides of the present invention can also be performed, for example, as follows. Receptors capable of binding to a specific polypeptide can be screened by (a) attaching sample cells to a polypeptide of the present invention or its partial peptide, and (b) selecting cells that can bind to the said polypeptide or its partial peptide.

For example, screening can be carried out as follows: a polypeptide of the present invention is first expressed, the recombinant polypeptide is purified and labeled, a binding assay is performed using various cell lines or primary cultured cells, and cells that express the receptor are selected (Growth and differentiation factors and their receptors, Shin-Seikagaku Jikken Kouza Vol.7 (1991) Honjyo, Arai, Taniguchi, and Muramatsu edit, p203-236, Tokyo-Kagaku-Doujin). A polypeptide of the present invention can be labeled with RI such as ¹²⁵I, and enzyme (alkaline phosphatase etc.).

Alternatively, a polypeptide of the present invention may be used without labeling and then detected by using a labeled antibody against that polypeptide. Cells expressing a receptor polypeptide of the present invention, and selected using the above screening methods, can be applied as mentioned below to screen agonists or antagonists of that receptor.

Once a ligand binding to a polypeptide of the present invention, a receptor of that polypeptide, or cells expressing that receptor have been obtained by screening as described above, a compound binding to that ligand or receptor can be screened. It is also possible to screen a compound that can inhibit both bindings (agonists or antagonists of the receptor, for example) by using this binding activity as an index.

When a polypeptide of the present invention is a receptor, the screening method comprises the steps of (a) contacting the ligand with a polypeptide of the present invention, or cells expressing that polypeptide, in the presence of a test sample, (b) detecting binding activity between that polypeptide or cells expressing that polypeptide and the ligand, and (c) selecting a compound that can reduce that binding activity when compared to activity in the absence of the test sample. Furthermore, when a polypeptide of the present invention is a ligand, the screening method comprises the steps of (a) contacting the polypeptide of the present invention with its receptor or cells expressing that receptor, in the presence of a test sample, (b) detecting binding activity between the polypeptide and its receptor, or cells expressing that receptor, and (c) selecting a compound that can reduce that binding activity compared to activity in the absence of the test sample.

Examples of test samples to screen include, but are not limited to, cell extracts, expressed gene library products, synthesized low molecular compounds, synthesized peptides, and natural compounds. A compound that is isolated by the above screening can also be used as a test sample, using binding activity with a polypeptide of the present invention as an index.

A compound isolated using this screening may be a candidate agonist or antagonist of a polypeptide or polypeptide receptor of the present invention. By monitoring changes in intracellular signals, such as phosphorylation due to reduced binding between a polypeptide and its receptor, it is possible to identify whether the obtained compound is an agonist or antagonist of the polypeptide receptor of the present invention. Also, the screened compound may be a candidate for a compound that can inhibit interaction between a polypeptide and its associated molecules (including receptors) *in vivo.* The polypeptides of this invention, recepters that bind to those polypeptides or ligands, and compounds thereof, can be applied in the development of preventative, therapeutic or testing agents for diseases in which the polypeptides of the present invention are involved.

Secretory proteins may regulate cellular conditions such as growth and differentiation. A novel factor that regulates cellular conditions can be found by i) adding a secretory protein of the present invention to a certain kind of cell, and ii) screening using as an index cellular changes in growth or differentiation, or activation of a particular gene.

Screening can be performed, for example, as follows. First, a polypeptide of the present invention is expressed and purified in a recombinant form. Then, the purified polypeptide is added to various cell lines or primary cultured cells, and changes in cell growth and differentiation are monitored. The induction of a particular gene known to be involved in a certain cellular change is detected using mRNA and polypeptide amounts. Alternatively, detection can be carried out using the amount of an intracellular molecule (low-molecular-weight compounds, etc.) changed by the action of a gene product (polypeptide) which influences a certain cellular change.

If screening reveals that a polypeptide of the present invention can regulate cellular conditions or functions, it is possible to apply that polypeptide as a pharmaceutical and diagnostic medicine for related diseases, either directly or by altering a part of it into an appropriate composition.

As for membrane proteins as described above, a secretory protein provided by the present invention may be used to explore a novel ligand-receptor interaction by screening based on binding activity to a known or new ligand or receptor. A similar method can be used to identify an agonist or antagonist. Compounds obtained by these methods are candidate compounds for inhibiting the interaction between the polypeptide of the present invention and an interacting molecule (including receptors). These compounds may be applied as preventive, therapeutic, or testing agents for diseases in which the polypeptide plays a role.

Proteins involved in signal transduction or transcription may be factors that affect a certain polypeptide or gene in response to intra- or extra-cellular stimuli. A novel factor able to affect a polypeptide or gene can be found by expressing a polypeptide provided by the present invention in a certain type of cell, and screening using as an index the activation of a certain intracellular polypeptide or gene.

Screening may be performed as follows. First, a transformed cell line which expresses a polypeptide of the present invention is obtained. Then, changes in a certain gene between the transformed and the original untransformed cell lines are detected using mRNA or polypeptide amounts. Alternatively, detection may be carried out using the amount of an intracellular molecule (low molecular weight compounds, etc.) changed by the function of a certain gene product (polypeptide). Furthermore, change in expression of a certain gene can be detected by estimating the activity of a marker gene product (polypeptide), where the polypeptide of the present invention is expressed in a cell that has been introduced with a fusion gene comprising a regulatory region of the certain gene and a marker gene (luciferase, β-galactosidase, etc.).

If the polypeptide or gene influenced by a protein of the present invention is involved in disease, it is possible to screen a gene or compound that can regulate that polypeptide or gene's expression and/or activity, either directly or indirectly, by utilizing a polypeptide of the present invention.

For example, a polypeptide of the present invention is expressed and purified as a recombinant polypeptide, and a polypeptide or gene that interacts with that polypeptide is purified and screened based on binding. Alternatively, changes in binding activity can be monitored after adding a candidate inhibitor compound. In another method, the 5'-upstream transcription regulatory region of a gene that encodes a polypeptide of the present invention and that can regulate the expression of another gene, is obtained. The gene is fused with a marker gene and then introduced into a cell, compounds and the like are added, and a factor which can regulate expression of that gene can be discovered.

A compound obtained by this screening can be used for developing pharmaceutical medicines for a disease in which a polypeptide of the present invention is involved. Similarly, if a regulatory factor obtained by screening turns out to be a polypeptide, compounds that can newly affect the expression or activity of this polypeptide may be used as a medicine for diseases in which the polypeptide of the present invention is involved.

If a polypeptide of the present invention has enzymatic activity, regardless of whether it is a secretory protein, membrane protein, or a protein involved in signal transduction, glycoprotein, transcription, or disease, screening may be performed by adding a compound to the polypeptide of the present invention under suitable conditions, and monitoring that compound's change. This enzymatic activity may also be used as an index to screen a compound that inhibits the polypeptide activity.

In an example of this screening, a polypeptide of the present invention is expressed and the recombinant polypeptide is purified. Then, compounds are contacted with the purified polypeptide, and the amount of compound and reaction product is examined. Alternatively, inhibitor candidate compounds are added, then a compound (substrate) that reacts with the purified polypeptide is added, and change in the amount of substrate and reaction product is examined.

A compound obtained by screening may be used as a medicine for diseases in which a polypeptide of the present invention is involved. Also such a compound can be applied in tests that examine, for example, whether a polypeptide of the invention functions normally *in vivo*.

Whether a secretory protein, membrane protein, signal transduction-related protein, glycoprotein-related protein, or transcription-related protein of the present invention is a novel protein involved in disease or not is determined using different method to that described above. In this method, a specific antibody against a polypeptide of the present invention is obtained, and the relationship between the expression or activity of the polypeptide and a certain disease is examined. Alternatively, analysis can use the methods in "Molecular Diagnosis of Genetic Diseases" as a reference (Elles R. edit, (1996) in the series of "Method in Molecular Biology" (Humana Press).

Proteins involved in disease are very useful in developing drugs which regulate their expression and activity, and become targets of the above-mentioned screenings. They are also useful in the medicinal industry as diagnostic markers for their related disease, or as gene therapy targets.

A compound isolated as mentioned above can be administered to patients as is, or after being formulated into a pharmaceutical composition according to known methods. Specific examples of pharmaceutically acceptable carriers or vehicles include sterilized water, saline, plant oils, emulsifiers, suspending agents and the like, where they are mixed with the compound appropriately. The pharmaceutical compositions can be administered to patients by a method known to those skilled in the art, such as intraarterial, intravenous, or subcutaneous injection. Dosage may vary depending on the weight or age of a patient, or the method of administration, but those skilled in the art can properly choose an appropriate dosage. If a compound is encoded by a polynucleotide, the polynucleotide can be cloned into a gene therapy vector, and used for gene therapy. Those skilled in the art can properly choose the dosage of the polynucleotide and the method of its administration, and these may vary depending on the weight, age or symptoms of a patient.

The present invention further relates to databases comprising at least one sequence of a polynucleotide and/or polypeptide, or a medium recorded in such databases, selected from the sequence data of the nucleotides and/or amino acids indicated in Table 1. The term "database" means a set of accumulated, machine-searchable and readable nucleotide sequence information. The databases of the present invention comprise at least one of the novel nucleotide sequences of the polynucleotides provided by the present invention. The databases of the present invention can consist only of the sequence data of the novel polynucleotides provided by the present invention, or can comprise other information on known nucleotide sequences of full-length cDNAs or ESTs. The databases of the present invention can be comprised of not only information on nucleotide sequences, but also information on gene functions as revealed by the present invention. Additional information such as the names of DNA clones carrying the full-length cDNAs can be recorded or linked together with the sequence data in the databases.

The database of the present invention is useful for gaining complete gene sequence information from the partial sequence information of a gene of interest. The database of the present invention comprises nucleotide sequence information of full-length cDNAs. Consequently, by comparing information in this database with the nucleotide sequence of a partial gene fragment yielded by differential display method or subtraction method, information on the full-length nucleotide sequence of interest can be gained from the sequence of the partial fragment as a starting clue.

Sequence information of the full-length cDNAs constituting the database of the present invention contains information on not only complete sequences, but also on gene expression frequency and gene homology to known genes and polypeptides. This extra information facilitates rapid functional analyses of partial gene fragments. Further, information on human genes is accumulated in the database of the present invention, and therefore, the database is useful for isolating a human homologue of a gene originating from another species. The human homologue can be isolated based on the nucleotide sequence of the gene from the original species.

At present, information on a wide variety of gene fragments can be obtained by differential display method and subtraction method. In general, these gene fragments are utilized as tools for isolating the full-length sequences thereof. When the gene fragment corresponds to a known gene, the full-length sequence is easily obtained by comparing the partial sequence with the information in known databases. However, when there is no information corresponding to the partial sequence of interest in known databases, cDNA cloning should be carried out for the full-length cDNA. It is often difficult to obtain the full-length nucleotide sequence using partial sequence information as an initial clue. If the full-length gene is not available, the amino acid sequence of the polypeptide encoded by that gene remains unidentified. Thus the database of the present invention can contribute to the identification of full-length cDNAs corresponding to gene fragments, which cannot be revealed using databases of known genes.

The present invention has provided 2,495 polynucleotides. In humans, where isolation of full-length cDNA has not progressed, the provision of new full-length cDNAs has great significance. Secretory proteins, membrane proteins, signal transduction-related proteins, glycoprotein-related proteins, transcription-related proteins, and so on are known to be involved in many diseases. The genes and proteins involved in diseases are useful for developing diagnostic markers or medicines for regulation of their expression and activity, or as a gene therapy target.

In particular, cDNA encoding secretory proteins of the present invention are extremely important to the industry since these proteins are expected to be useful as pharmaceutical agents, and many disease-related genes can be included with them. In addition, membrane proteins, signal transduction-related proteins, transcription-related proteins, disease-related proteins, and genes encoding these proteins can be used as disease indexes, etc. These cDNAs are also very important to the industry, and are expected to be effective in treating diseases and the like by regulating the activity or expression of the proteins they encode.

Human cDNAs involved in various diseases, morbid states, or functions were isolated in the present invention. The cDNAs of the present invention include, for example, genes that can be useful as diagnostic markers or therapeutic targets for the diseases or morbid states shown below. These diseases receive widespread attention, and require the development of new technology for treatment and diagnosis.
- osteoporosis
- neurologic diseases
- Alzheimer's disease
- Parkinson's disease
- dementia
- various cancers

In addition, human cDNAs involved in the functions shown below were isolated in the present invention. These genes can be used to elucidate the mechanisms of various functions, and in therapeutic methods which enhance or repress those functions. For example, genes participating in tissue generation and functional expression can be used in regenerative medicines.
- emotional reaction
- tissue generation and functional expression
- motor function controlled by the brain, signaling function controlled by the brain
- emotional reaction, fear response, and panic

Furthermore, information on the nucleotide sequence of the full-length cDNAs or their full-length amino acid sequences as provided by the present invention can be used to isolate human genes based on partial sequence information obtained by functional analysis of various genes, or from sequence information of genes from non-human organisms.

Any patents, patent applications, and publications cited herein are incorporated by reference.

The present invention is illustrated more specifically with reference to the following examples, but is not to be construed as being limited thereto.

### EXAMPLE 1

### Preparation of cDNA library by oligo-capping

### (1) Extraction and purchase of mRNA

Total RNAs as mRNA sources were extracted from human tissues (shown below) by the method described in the reference (J. Sambrook, E. F. Fritsch & T. Maniatis, Molecular Cloning Second edition, Cold Spring harbor Laboratory Press, 1989). Further, by the method described in the reference (J. Sambrook, E. F. Fritsch & T. Maniatis, Molecular Cloning Second edition, Cold Spring harbor Laboratory Press, 1989), total RNAs as mRNA sources were extracted from human culture cells and human primary culture cells (shown below) cultivated by the methods described in the catalogs.

The library names and origins are indicated below in the order of "Library name: Origin". When a library was prepared by the subtraction method, the item is followed by a description of how to prepare the subtracted library.

### <Extraction of mRNA from human tissues>

NTONG: Normal tongue;
CTONG: Tongue cancer;
FCBBF: Fetal brain;
OCBBF: Fetal brain;
PLACE: Placenta;
SYNOV: Synovial membrane tissue (from rheumatioid arthritis);
CORDB: Cord blood.

### <Extraction of mRNA from culture cells>

BNGH4: H4 cells (ATCC #HTB-148);
IMR32: IMR32 cells (ATCC #CCL-127);
SKNMC: SK-N-MC cells (ATCC #HTB-10);
3NB69: NB69 cells (RCB #RCB0480);
BGGI1: GI1 cells (RCB #RCB0763);
NB9N4: NB9 cells (RCB #RCB0477);
SKNSH: SK-N-SH cells (RCB #RCB0426);
AHMSC: Human mesenchymal cells (HMSC);
CHONS: Chondrocytes;
ERLTF: TF-1 cells (Erythroleukemia);
HELAC: HeLa cells;
JCMLC: Myelogenous leukemia cells;
MESTC: Mesenchyme stem cells;
N1ESE: Mesenchymal stem cells;
NCRRM: Embryonal carcinomas;
NCRRP: Embryonal carcinomas treated with retinoic acid (RA) to induce the differentiation;
T1ESE: Mesenchymal stem cells treated with trichostatin and 5-azacytidine to induce the differentiation;
NT2RM: NT2 cells (STARATAGENE #204101);
NT2RP: NT2 cells treated with RA for 5 weeks to induce the differentiation;
NT2RI: NT2 cells treated with RA for 5 weeks to induce the differentiation, followed by the treatment with the growth inhibitor for 2 weeks;
NT2NE: NT2 cells were treated with RA and the growth inhibitor for the neuronal differentiation, and the resultant neurons were concentrated and harvested (NT2 Neuron);
NTISM: NT2 cells (STARATAGENE #204101) were treated with RA for five weeks to induce the differentiation, and then treated with the growth inhibitor for two weeks; mRNA was prepared from the cells and a cDNA library was constructed from the mRNA; the cDNA libraries whose nucleotide sequences were shared by those of mRNAs from undifferentiated NT2 cells were subtracted by using a Subtract Kit (Invitrogen #K4320-01); the subtracted library (NT2RI-NT2RM) was provided by this procedure.

RCB indicates that the cell was provided by the Cell Bank, RIKEN GENE BANK, The Institute of Physical and Chemical Research; ATCC indicates that the cell was provided by American Type Culture Collection.

### <Extraction of mRNA from primary culture cells>

ASTRO: Normal human astrocyte NHA5732, Takara Shuzo #CC2565;
DFNES: Normal human dermal fibroblast (neonatal skin); NHDF-Neo NHDF2564, Takara Shuzo #CC2509;
MESAN: Normal human mesangial cell NHMC56046-2, Takara Shuzo #CC2559;
NHNPC: Normal human neural progenitor cell NHNP5958, Takara Shuzo #CC2599;
PEBLM: Normal human peripheral blood mononuclear cell HPBMC5939, Takara Shuzo #CC2702;
HSYRA: Human synoviocyte HS-RA (from rheumatoid arthritis), Toyobo #T404K-05;
PUAEN: Normal human pulmonary artery endothelial cells, Toyobo #T302K-05;
UMVEN: Normal human umbilical vein endothelial cell HUVEC, Toyobo #T200K-05;
HCASM: Normal human coronary artery smooth muscle cell HCASMC, Toyobo #T305K-05;
HCHON: Normal human chondrocyte HC, Toyobo #T402K-05;
HHDPC: Normal human dermal papilla cell HDPC, Toyobo #THPCK-001;
CD34C: CD34+ cells (AllCells, LLC #CB14435M);
D3OST: CD34+ cells treated with the osteoclast differentiation factor (ODF) for three days to induce the differentiation;
D6OST: CD34+ cells treated with ODF for six days to induce the differentiation;
D9OST: CD34+ cells treated with ODF for nine days to induce the differentiation;
ACTVT: Activated T-cells;
LYMPB: Lymphoblasts (EB virus transferred B cells);
NETRP: Neutrophils.

Total RNAs extracted from the following human tissues were then purchased and used as mRNA sources. Library names and the origins are indicated below in the order of "Library name: Origin". When a library was prepared by the subtraction method, the item is followed by a description of how to prepare the subtracted library.

### <Purchase of total RNA containing mRNA extracted from human tissues>

ADRGL: Adrenal gland, CLONTECH #64016-1;
BRACE: Brain (cerebellum), CLONTECH #64035-1;
BRAWH: Whole brain, CLONTECH #64020-1;
FEBRA: Fetal brain, CLONTECH #64019-1;
FELIV: Fetal liver, CLONTECH #64018-1;
HEART: Heart, CLONTECH #64025-1;
HLUNG: Lung, CLONTECH #64023-1;
KIDNE: Kidney, CLONTECH #64030-1;
LIVER: Liver, CLONTECH #64022-1;
MAMGL: Mammary Gland, CLONTECH #64037-1;
PANCR: Pancreas, CLONTECH #64031-1;
PROST: Prostate, CLONTECH #64038-1;
SALGL: Salivary Gland, CLONTECH #64026-1;
SKMUS: Skeletal Muscle, CLONTECH #64033-1;
SMINT: Small Intestine, CLONTECH #64039-1;
SPLEN: Spleen, CLONTECH #64034-1;
STOMA: Stomach, CLONTECH #64090-1;
TBAES: Breast (Tumor), CLONTECH #64015-1;
TCERX: Cervix (Tumor), CLONTECH #64010-1;
TCOLN: Colon (Tumor), CLONTECH #64014-1;
TESTI: Testis, CLONTECH #64027-1;
THYMU: Thymus, CLONTECH #64028-1;
TLUNG: Lung (Tumor), CLONTECH #64013-1;
TOVAR: Ovary (Tumor), CLONTECH #64011-1;
TRACH: Trachea, CLONTECH #64091-1;
TUTER: Uterus (Tumor), CLONTECH #64008-1;
UTERU: Uterus, CLONTECH #64029-1;
ADIPS: Adipose, Invitrogen #D6005-01;
BLADE: Bladder, Invitrogen #D6020-01;
BRALZ: Cerebral cortex from an Alzheimer patient (Brain, cortex, Alzheimer), Invitrogen #D6830-01;
CERVX: Cervix, Invitrogen #D6047-01;
COLON: Colon, Invitrogen #D6050-0;
NESOP: Esophagus, Invitrogen #D6060-01;
PERIC: Pericardium, Invitrogen #D6105-01;
RECTM: Rectum, Invitrogen #D6110-01;
TESOP: Esophageal (Tumor), Invitrogen #D6860-01;
TKIDN: Kidney (Tumor), Invitrogen #D6870-01;
TLIVE: Liver (Tumor), Invitrogen #D6880-01;
TSTOM: Stomach (Tumor), Invitrogen #D6920-01;
BEAST: Adult breast, STARATAGENE #735044;
FEHRT: Fetal heart, STARATAGENE #738012;
FEKID: Fetal kidney, STARATAGENE #738014;
FELNG: Fetal lung, STARATAGENE #738020;
NOVAR: Adult ovary, STARATAGENE #735260;
BRASW: subtracted library (BRALZ-BRAWH). A cDNA library was constructed from mRNA prepared from tissues of cerebral cortex obtained from an Alzheimer patient [BRALZ: Cerebral cortex from an Alzheimer patient (Brain, cortex, Alzheimer), Invitrogen #D6830-01]; the cDNA libraries whose nucleotide sequences were shared by those of mRNAs from whole brain tissue [BRAWH: Whole brain, CLONTECH #64020-1] were subtracted using a Subtract Kit (Invitrogen #K4320-01).

Further, mRNAs extracted and purified as poly A(+) RNAs from the human tissues shown below were purchased. A cDNA library was prepared from an RNA mixture in which the poly A(+) RNA from each tissue was combined with poly A(-) RNA. The poly A(-) RNA was prepared by removing poly A(+) RNA from the total RNA of whole brain tissue (CLONTECH #64020-1) by using oligo dT cellulose. The library names and origins are indicated below in the order of "Library name: Origin".

### <Purchase of mRNAs of human tissues as poly A(+) RNAs>

BRAMY: Brain (amygdala), CLONTECH #6574-1;
BRCAN: Brain (caudate nucleus), CLONTECH #6575-1;
BRCOC: Brain (corpus callosum), CLONTECH #6577-1;
BRHIP: Brain (hippocampus), CLONTECH #6578-1;
BRSSN: Brain (substantia nigra), CLONTECH #6580-1;
BRSTN: Brain (subthalamic nucleus), CLONTECH #6581-1;
BRTHA: Brain (thalamus), CLONTECH #6582-1.

### (2) Preparation of cDNA libraries

A cDNA library was prepared from each RNA using the improved method (WO 01/04286) of oligo capping [M. Maruyama and S. Sugano, Gene, 138: 171-174 (1994)]. A series of procedures, BAP (Bacterial Alkaline Phosphatase) treatment, TAP (Tobacco Acid Pyrophosphatase) treatment, RNA ligation, first strand cDNA synthesis and RNA removal, were carried out using the oligo-cap linker (agcaucgagu cggccuuguu ggccuacugg/ SEQ ID NO: 4991) and oligo dT primer (gcggctgaag acggcctatg tggccttttt tttttttttt tt/ SEQ ID NO: 4992), as described in WO 01/04286. The single-stranded cDNA was then converted to a double-stranded cDNA by PCR (polymerase chain reaction) using 5' (agcatcgagt cggccttgtt g/ SEQ ID NO: 4993) and 3' (gcggctgaag acggcctatg t/ SEQ ID NO: 4994) PCR primers, and then digested with SfiI. Then, a fraction of cDNA fragments, typically 2-kb or longer (3-kb or longer in some cases), was unidirectionally cloned into a *Dra*III-digested pME18SFL3 vector (Figure 1) (GenBank AB009864, Expression vector); and the cDNA library was thus prepared.

Shown below are the names of cDNA libraries used in the analysis of full-length cDNA sequences, and their origins. The Library Name is provided with the Type, Origin, and such of the library source, demarcated by a slash mark (/) within parentheses.
3NB69 (culture cells / NB69 cells (RCB #RCB0480))
ACTVT (primary culture cells / Activated T-cells(Activated T-cell))
ADIPS (Tissues / Adipose (Invitrogen #D6005-01))
ADRGL (Tissues / Adrenal gland (CLONTECH #64016-1))
ASTRO (primary culture cells / Normal Human Astrocyte NHA5732 (Takara Shuzo #CC2565))
BLADE (Tissues / Bladder (Invitrogen #D6020-01))
BNGH4 (culture cells / H4 cells (ATCC #HTB-148))
BRACE (Tissues / Brain (cerebellum) (CLONTECH #64035-1))
BRALZ (Tissues / Cerebral cortex from an Alzheimer patient (Brain, cortex, Alzheimer) (Invitrogen #D6830-01))
BRAMY (Tissues / Brain (amygdala) (CLONTECH #6574-1 ))
BRASW (Tissues / subtracted library (BRALZ-BRAWH). The cDNAs from tissues of cerebral cortex obtained from an Alzheimer patient whose nucleotide sequences were shared by those of mRNAs from whole brain tissue were subtracted.
BRAWH (Tissues / Whole brain (CLONTECH #64020-1))
BRCAN (Tissues / Brain (caudate nucleus) (CLONTECH #6575-1))
BRCOC (Tissues / Brain (corpus callosum) (CLONTECH #6577-1))
BRHIP (Tissues / Brain (hippocampus) (CLONTECH #6578-1))
BRSSN (Tissues / Brain (substantia nigra) (CLONTECH #6580-1))
BRSTN (Tissues / Brain (subthalamic nucleus) (CLONTECH #6581-1))
BRTHA (Tissues / Brain (thalamus) (CLONTECH #6582-1))
CERVX (Tissues / Cervix (Invitrogen #D6047-01))
COLON (Tissues / Colon (Invitrogen #D6050-0))
CORDB (Tissues / Cord blood)
CTONG (Tissues / Tongue Cancer)
D3OST (primary culture cells / CD34+ cells (treated with ODF for three days to induce the differentiation))
DFNES (primary culture cells / Normal human dermal fibroblasts (neonatal skin); NHDF-Neo NHDF2564 (Takara Shuzo #CC2509))
ERLTF (culture cells / TF-1 cells (Erythroleukemia))
FCBBF (Tissues / Fetal brain)
FEBRA (Tissues / Fetal brain (CLONTECH #64019-1))
HCASM (primary culture cells / Normal human coronary artery smooth muscle cell HCASMC (Toyobo #T305K-05))
HCHON (primary culture cells / Normal human chondrocyte HC (Toyobo #T402K-05))
HEART (Tissues / Heart (CLONTECH #64025-1))
HHDPC (primary culture cells / Normal human dermal papilla cell HDPC (Toyobo #THPCK-001))
HLUNG (Tissues / Lung (CLONTECH #64023-1))
HSYRA (primary culture cells / Human synoviocyte HS-RA (from rheumatoid arthritis)(Toyobo #T404K-05))
IMR32 (culture cells / IMR32 cells (ATCC #CCL-127))
KIDNE (Tissues / Kidney (CLONTECH #64030-1))
LIVER (Tissues / Liver (CLONTECH #64022-1))
LYMPB (primary culture cells / Lymphoblasts (EB virus transferred B cells))
MESAN (primary culture cells / Normal human mesangial cell NHMC56046-2 (Takara Shuzo #CC2559))
MESTC (culture cells / Mesenchyme stem cells)
NB9N4 (culture cells / NB9 cells (RCB #RCB0477))
NCRRP (culture cells / Embryonal carcinomas treated with RA to induce the differentiation)
NESOP (Tissues / Esophagus (Invitrogen #D6060-01))
NHNPC (primary culture cells / Normal human neural progenitor cell NHNP5958 (Takara Shuzo #CC2599))
NOVAR (Tissues / Adult ovary (STARATAGENE #735260))
NT2NE (culture cells / NT2 cells were treated with RA and the growth inhibitor for the neuronal differentiation, and the resultant neurons were concentrated and harvested (NT2 Neuron)) NT2RI (culture cells / NT2 cells treated with RA for five weeks to induce the differentiation, followed by the treatment with the growth inhibitor for 2 weeks)
NT2RP (culture cells / NT2 cells treated with RA for five weeks to induce the differentiation)
NTONG (Tissues / Normal tongue)
OCBBF (Tissues / Fetal brain)
PEBLM (primary culture cells / Normal human peripheral blood mononuclear cell HPBMC5939 (Takara Shuzo #CC2702))
PERIC (Tissues / Pericardium (Invitrogen #D6105-01))
PLACE (Tissues / Placenta)
PROST (Tissues / Prostate (CLONTECH #64038-1))
PUAEN (primary culture cells / Normal human pulmonary artery endothelial cells (Toyobo #T302K-05))
RECTM (Tissues / Rectum (Invitrogen #D6110-01))
SKMUS (Tissues / Skeletal Muscle (CLONTECH #64033-1))
SKNMC (culture cells / SK-N-MC cells (ATCC #HTB-10))
SKNSH (culture cells / SK-N-SH cells (RCB #RCB0426))
SMINT (Tissues / Small Intestine (CLONTECH #64039-1))
SPLEN (Tissues / Spleen (CLONTECH #64034-1))
STOMA (Tissues / Stomach (CLONTECH #64090-1))
SYNOV (Tissues / Synovial membrane tissue (from rheumatoid arthritis))
T1ESE (culture cells / Mesenchymal stem cell (treated with trichostatin and 5-azacytidine to induce the differentiation))
TBAES (Tissues / Breast (Tumor) (CLONTECH #64015-1))
TCERX (Tissues / Cervix (Tumor) (CLONTECH #64010-1))
TCOLN (Tissues / Colon (Tumor) (CLONTECH #64014-1))
TESOP (Tissues / Esophageal (Tumor) (Invitrogen #D6860-01))
TESTI (Tissues / Testis (CLONTECH #64027-1))
THYMU (Tissues / Thymus (CLONTECH #64028-1))
TKIDN (Tissues / Kidney (Tumor) (Invitrogen #D6870-01))
TLIVE (Tissues / Liver (Tumor) (Invitrogen #D6880-01))
TOVAR (Tissues / Ovary (Tumor) (CLONTECH #64011-1))
TRACH (Tissues / Trachea (CLONTECH #64091-1))
TSTOM (Tissues / Stomach (Tumor) (Invitrogen #D6920-01))
TUTER (Tissues / Uterus (Tumor) (CLONTECH #64008-1))
UTERU (Tissues / Uterus (CLONTECH #64029-1))

cDNA libraries with a high fullness ratio (the fullness ratio of 5'-end was 90% on average, calculated for each cDNA library using the protein coding region found in known mRNA species as an index) prepared by the improved oligo-capping method were constructed using a eukaryotic expression vector pMEI8SFL3. The vector contained SRα promoter and SV40 small t intron upstream of the cloning site, and SV40 polyA added signal sequence site downstream. As the cloning site of pME18SFL3 has asymmetrical DraIII sites, and the ends of cDNA fragments contain SfiI sites complementary to the DraIII sites, the cloned cDNA fragments can be inserted downstream of the SRα promoter unidirectionally. Therefore, clones containing full-length cDNA can be expressed transiently by introducing the obtained plasmid directly into COS cells, etc. Thus, clones can be analyzed very easily using the clone gene product proteins, or those proteins' biological activities.

### (3) Assessment of 5'-end completeness of clones derived from a cDNA library prepared by oligo-capping

With respect to the plasmid DNAs of clones derived from the libraries, the nucleotide sequences of cDNA 5'-ends (3'-ends as well in some cases) were determined in a DNA sequencer (ABI PRISM 3700, PE Biosystems), after sequencing reaction were conducted using a DNA sequencing reagent (BigDye Terminator Cycle Sequencing FS Ready Reaction Kit, PE Biosystems) according to the manual. A database was constructed based on the obtained data.

The 5'-end completeness of about 1,110,000 clones derived from the human cDNA libraries prepared by the improved oligo-capping method was determined using the following method. The clones whose 5'-end sequences were consistent with those of known human mRNA in the public database were judged to be "full-length" if they had a longer 5'-end sequence than that of the known human mRNA. Even if the 5'-end sequence was shorter, clones containing the translation initiation codon were judged to comprise the "full-length" sequence. Clones which did not comprise the translation initiation codon were judged to be "not-full-length". The fullness ratio ((the number of full-length clones)/(the number of full-length and not-full-length clones)) at the 5'-end of the cDNA clones was determined by comparison with known human mRNA. The fullness ratio of the 5'-ends was found to be 90%. The result indicates that the fullness ratio at the 5'-end sequence was extremely high in human cDNA clones obtained by the oligo-capping method.

### EXAMPLE 2

### Sequencing analysis of cDNA ends and selection of full-length clones

With respect to the plasmid DNAs of clones obtained from each cDNA library, the 5'-end nucleotide sequences of the cDNAs were determined in a DNA sequencer (ABI PRISM 3700, PE Biosystems), after sequencing reaction was conducted by using a DNA sequencing reagent (Dye Terminator Cycle Sequencing FS Ready Reaction Kit, dRhodamine Terminator Cycle Sequencing FS Ready Reaction Kit or BigDye Terminator Cycle Sequencing FS Ready Reaction Kit, PE Biosystems) according to the manual. A database was constructed using the data obtained.

For the analyzed 5'-end sequences of cDNA clones, the data with the annotation of "complete cds" in the GenBank and UniGene were searched by BLAST homology search. When identical to certain human mRNA sequences, such cDNA clones were excluded. Then, clustering was carried out. When the identity was 90% or higher, and the length of consensus sequence was 50 base pairs or longer, the cDNA clones were assumed to belong to an identical cluster, and thus clustered. cDNA clones longer in the 5' direction were selected from the members belonging to a cluster; if required, the 3'-end sequences of the selected clones were determined by the same analysis method as used to determine the 5'-end sequences. The data of the end sequences obtained were analyzed, and then the clones forming a sequence contig at 5'- and 3'-ends were excluded. Further, as mentioned above, the data was analyzed again by BLAST homology search; when identical to certain human mRNA sequences (including sequences patented and applied for), the cDNA clones were excluded. Thus, the cDNAs clones to be analyzed for their nucleotide sequence were obtained.

### EXAMPLE 3

### Analysis of the full-length nucleotide sequences

The full-length nucleotide sequences of the selected clones were determined. The nucleotide sequence determination was mainly performed by primer walking method comprising the dideoxy terminator method using custom-made synthetic DNA primers. Namely, the nucleotide sequences of the DNAs were determined in a sequencer from PE Biosystems, after sequencing reaction was carried out with a DNA sequencing reagent from the same supplier using the custom-made synthetic DNA primers according to the manual. A part of the clones were analyzed with a DNA sequencer from Licor.

Further, the nucleotide sequences of a part of the clones were determined by the shotgun method where the plasmids containing the cDNAs were digested at random were used, instead of the use of custom-made primers, by the same method in the DNA sequencer. The full-length nucleotide sequences were finally determined by completely assembling the partial nucleotide sequences obtained by the above method.

Then, the regions translatable to proteins were deduced from the determined full-length nucleotide sequences, and thereby the amino acid sequences were determined. SEQ ID NOs corresponding to the respective sequences are shown in Table 1.

### EXAMPLE 4

### Functional prediction by homology search

GenBank, SwissProt, UniGene, RefSeq, and nr were searched using BLAST for the determined nucleotide sequences (SEQ ID NOs: 1-2188) and the ORF amino acid sequences deduced to encode the polypeptides (SEQ ID NOs: 2189-4376). SwissProt, RefSeq, and nr were searched using BLAST for the nucleotide sequences of SEQ ID NOs: 4377-4683, and the amino acid sequences of SEQ ID NOs: 4684-4990. Of the hit data which met the criteria described below, representative hit data were selected. This representative data is hit data with a higher homology and that enables relatively easy functional prediction for nucleotide sequences and deduced amino acid sequences. The results of the homology search are shown at the end of this specification.
· Hit data whose P value or E value is 10⁻⁴ or less
· For analysis using an amino acid database, hit data whose P value or E value is 10⁻⁴ or less, where the length of consensus sequence x homology = 30 or higher,

Thus, only representative data are indicated and molecules exhibiting homology to each clone are not limited thereto. For some clones, hit data that do not meet the above-described criteria in BLAST search are not shown.

### EXAMPLE 5

### Search for signal sequence, transmembrane domain and other functional domains in the deduced amino acid sequences

With respect to the amino acid sequences deduced from the full-length nucleotide sequences, the prediction was made for the presence of signal sequence at the amino terminus, the presence of transmembrane domain, and the presence of functional protein domains (motifs). The signal sequence at the amino terminus was searched for by PSORT [K. Nakai & M. Kanehisa, Genomics, 14: 897-911 (1992)]; the transmembrane domain, by SOSUI [T. Hirokawa et al., Bioinformatics, 14: 378-379 (1998)] (Mitsui Knowledge Industry); the function domain, by Pfam (Version 5.5) (http://www.sanger.ac.uk/Software/Pfam/index.shtml). The amino acid sequence in which the signal sequence at the amino terminus or transmembrane domain had been predicted to be present by PSORT or SOSUI were assumed to be a secretory or membrane protein. Further, when the amino acid sequence hit a certain functional domain by the Pfam functional domain search, the protein function can be predicted based on the hit data, for example, by referring to the function categories on the PROSITE (http://www.expasy.ch/cgi-bin/prosite-list.pl). In addition, the functional domain search can also be carried out on the PROSITE.

The search results obtained with the respective programs are shown below.

The 161 clones whose deduced amino acid sequences were detected to have the signal sequences by PSORT are as follows.
ADIPS2000088, ADRGL2000172, ADRGL2009146, BNGH42003570, BRACE2030341, BRACE2031531, BRACE2039327, BRACE2041200, BRACE2043142, BRACE3004113, BRACE3004843, BRACE3010397, BRACE3011505, BRACE3026735, BRACE3040856, BRAMY2004771, BRAMY2005052, BRAMY2019300, BRAMY2019963, BRAMY3007206, BRAMY3007609, BRAMY3008505, BRAWH2002560, BRAWH3001475, BRAWH3003992, BRAWH3004666, BRAWH3006548, BRCAN2002948, BRCAN2010376, BRCAN2012481, BRHIP2005236, BRHIP2026288, BRHIP3008598, BRSSN2014424, BRSTN2007000, BRSTN2010363, BRSTN2016470, BRTHA2002608, BRTHA2005579, BRTHA2016496, BRTHA2018344, BRTHA3000633, BRTHA3017047, COLON2000568, COLON2002443, COLON2005126, CTONG1000302, CTONG1000488, CTONG1000508, CTONG2000042, CTONG2008233, CTONG3000707, CTONG3001560, CTONG3003179, CTONG3003483, CTONG3003737, CTONG3005648, CTONG3008258, CTONG3009385, D3OST2002182, D3OST2002648, FCBBF2001183, FCBBF2007510, FCBBF3009888, FCBBF4000076, FEBRA2025427, HCASM2002502, HCHON2001577, HCHON2002676, HEART2001680, HSYRA2009102, IMR322002110, KIDNE2000846, KIDNE2006580, KIDNE2013095, LYMPB2000083, NOVAR2000136, NOVAR2001108, NT2RI2008724, NT2RI3005403, NT2RI3007065, NT2RP7000359, NT2RP7000466, NTONG2000413, OCBBF2006764, PLACE5000171, PLACE5000282, PLACE6012574, PROST2018090, PUAEN2002489, PUAEN2009795, RECTM2000433, SKMUS2000757, SKNMC2002402, SMINT2015787, SPLEN2009548, SPLEN2012624, SPLEN2012889, SPLEN2021701, SPLEN2030479, SPLEN2031125, SPLEN2034021, SPLEN2034781, SPLEN2037722, SPLEN2040222, STOMA2004294, SYNOV4002883, SYNOV4008336, TESOP2002273, TESOP2005485, TESOP2007636, TESTI2002618, TESTI2036684, TESTI2048898, TESTI4001561, TESTI4001665, TESTI4008401, TESTI4008797, TESTI4014392, TESTI4020102, TESTI4025797, TESTI4028429, TESTI4035065, TESTI4035649, TESTI4042711, TESTI4046487, THYMU2001053, THYMU2003632, THYMU2005321, THYMU2013386, THYMU2014353, THYMU2019210, THYMU2027497, THYMU2027695, THYMU2029676, THYMU2039780, THYMU2040412, THYMU3004835, THYMU3005696, THYMU3007845, TKIDN2002424, TKIDN2019116, TLIVE2002690, TRACH2007834, TRACH3002650, TRACH3004786, TRACH3006149, TRACH3035199, TRACH3035526, UTERU1000339, UTERU2025645, UTERU2026090, UTERU3000645, UTERU3000899, UTERU3001585, UTERU3002209, UTERU3002383, UTERU3003776, UTERU3009690, UTERU3009979, UTERU3015500

Deduced amino acid sequences of following 20 clones were also detected to have the signal sequences by PSORT.
BRACE2002589, BRACE2009318, BRACE2039823, BRAWH2006395, BRAWH2008993, BRCOC2019841, BRHIP2005271, BRTHA2011321, FEBRA2028256, HCASM2003099, PROST2000452, PROST2019487, SPLEN2016932, STOMA2003158, SYNOV2001660, SYNOV4003981, TESTI2015626, TESTI4000319, TKIDN2018926, UTERU2032279

The 523 clones whose deduced amino acid sequences were detected to have the transmembrane domains by SOSUI are as follows. Numerals indicate the numbers of transmembrane domains detected in the deduced amino acid sequences. Of the search result, the Clone Name and the Number of transmembrane domains are demarcated by a double slash mark (//).
ACTVT2000380//1, ADRGL2003329//2, ASTRO2014923//6, ASTRO3000301//1, BLADE1000176//1, BLADE2002073//1, BLADE2002947//2, BLADE2004462//2, BLADE2004670//1, BLADE2008539//1, BNGH42003570//1, BRACE1000186//1, BRACE2005457//8, BRACE2014306//11, BRACE2016981//1, BRACE2030341//1, BRACE2030884//3, BRACE2031527//1, BRACE2031899//2, BRACE2032385//3,
BRACE2036005//1, BRACE2040138//2, BRACE2043142//4, BRACE2043665//1, BRACE3000697//4, BRACE3001391//3, BRACE3002298//1, BRACE3003004//1, BRACE3004113//1, BRACE3004843//1, BRACE3006462//3, BRACE3008384//6, BRACE3009574//1, BRACE3009708//6, BRACE3010397//2, BRACE3011505//2, BRACE3013740//3, BRACE3014005//8, BRACE3014068//3, BRACE3014807//3,
BRACE3020286//3, BRACE3020594//2, BRACE3024662//1, BRACE3025531//1, BRACE3026008//2, BRACE3031838//1, BRACE3040856//3, BRALZ2016085//10, BRAMY2004771//2, BRAMY2005052//2, BRAMY2017528//1, BRAMY2019300//2, BRAMY2019963//1, BRAMY2028856//2, BRAMY2033003//2, BRAMY2033116//2, BRAMY2033594//1, BRAMY2036396//2, BRAMY2039872//2, BRAMY2040592//2,
BRAMY2041542//1, BRAMY2045036//1, BRAMY2047420//1, BRAMY2047765//3, BRAMY3002312//1, BRAMY3004224//2, BRAMY3004919//5, BRAMY3008505//2, BRASW1000125//2, BRAWH2002560//1, BRAWH2002761//3, BRAWH2007658//2, BRAWH2014414//1, BRAWH2016439//1, BRAWH2016702//3, BRAWH3000078//2, BRAWH3000314//1, BRAWH3001891//6, BRAWH3002600//1, BRAWH3003555//2,
BRAWH3003727//1, BRAWH3004453//1, BRAWH3004666//2, BRAWH3005132//1, BRAWH3005912//1, BRAWH3006548//2, BRAWH3007221//2, BRAWH3007506//2, BRAWH3007592//1, BRAWH3008634//1, BRCAN2012355//5, BRCAN2012481//1, BRCAN2013655//3, BRCAN2014143//5, BRCAN2028355//4, BRCOC2007034//2, BRCOC2019934//2, BRHIP2000691//1, BRHIP2001805//1, BRHIP2002172//8,
BRHIP2004814//1, BRHIP2004883//2, BRHIP2005752//2, BRHIP2009414//7, BRHIP2013699//1, BRHIP2026288//2, BRHIP3000526//1, BRHIP3007483//2, BRHIP3007586//1, BRHIP3008598//3, BRHIP3015751//1, BRHIP3024118//7, BRHIP3026097//1, BRSSN2003086//1, BRSSN2008549//1, BRSSN2011738//2, BRSSN2014424//9, BRSSN2018925//2, BRSTN2003835//1, BRSTN2007000//1,
BRSTN2012380//1, BRSTN2015015//2, BRSTN2016678//3, BRSTN2017110//2, BRTHA2002281//2, BRTHA2002376//2, BRTHA2002493//1, BRTHA2002608//2, BRTHA2002808//1, BRTHA2003110//2, BRTHA2003461//3, BRTHA2006075//2, BRTHA2011194//1, BRTHA2012980//2, BRTHA2013460//2, BRTHA2015696//2, BRTHA2015878//2, BRTHA2016215//1, BRTHA2017985//1, BRTHA2018344//3,
BRTHA2018624//1, BRTHA3000633//2, BRTHA3002427//12, BRTHA3003474//2, BRTHA3007148//3, BRTHA3008386//4, BRTHA3008778//1, BRTHA3009090//1, BRTHA3009291//2, BRTHA3016845//2, BRTHA3017047//2, BRTHA3017589//2, BRTHA3017848//8, BRTHA3018656//9, CERVX2002006//1, COLON2002443//1, COLON2005126//2, CTONG1000302//1, CTONG1000341//1, CTONG2004062//4,
CTONG2008233//2, CTONG2009423//3, CTONG2009531//1, CTONG2013178//1, CTONG2019652//1, CTONG2019788//1, CTONG2020127//1, CTONG2020522//1, CTONG2020638//6, CTONG2022601//2, CTONG2023512//2, CTONG2026920//1, CTONG2027327//1, CTONG2028124//3, CTONG2028687//2, CTONG3001560//4, CTONG3002020//2, CTONG3002412//3, CTONG3003483//2, CTONG3003737//1,
CTONG3008252//1, CTONG3008496//2, CTONG3008566//1, CTONG3008951//2, CTONG3009227//1, CTONG3009239//3, CTONG3009328//5, CTONG3009385//4, D3OST2002648//6, DFNES1000107//1, DFNES2010502//3, FCBBF2001183//2, FCBBF2007510//2, FCBBF3003435//1, FCBBF3004502//10, FCBBF3009888//1, FCBBF3012170//1, FCBBF3021576//1, FCBBF3023895//1, FEBRA2007544//1,
FEBRA2007708//13, FEBRA2008311//7, FEBRA2020668//2, FEBRA2025427//1, FEBRA2027082//1, HCASM2003212//1, HCASM2007047//9, HCHON2000212//1, HCHON2001084//12, HCHON2001548//1, HCHON2001712//9, HCHON2004007//3, HCHON2005921//4, HLUNG2000014//1, HLUNG2003872//5, HLUNG2010464//4, HLUNG2015617//2, HLUNG2017350//4, HSYRA2005496//2, HSYRA2006873//1,
HSYRA2008714//6, HSYRA2009102//10, IMR322000127//2, IMR322002110//1, IMR322006222//1, KIDNE1000064//10, KIDNE2000832//10, KIDNE2000846//5, KIDNE2006580//1, KIDNE2010264//1, KIDNE2011635//12, KIDNE2012945//1, KIDNE2013095//3, LIVER2007415//1, LYMPB2000083//3, MESAN2001979//3, MESAN2012054//2, MESTC1000042//1, NHNPC2000606//1, NHNPC2001223//1,
NT2RI2008724//1, NT2RI2009855//3, NT2RI3001263//1, NT2RI3003095//3, NT2RI3003382//1, NT2RI3003409//1, NT2RI3005403//2, NT2RI3006673//1, NT2RI3007065//3, NT2RI3007543//2, NT2RI3007978//2, NT2RP7000466//1, NT2RP7009030//1, NT2RP7014005//2, NTONG2000413//1, OCBBF2006151//5, OCBBF2006567//1, OCBBF2006764//1, OCBBF2007114//1, OCBBF2007428//1,
OCBBF2009926//2, OCBBF2010140//13, OCBBF2017516//2, OCBBF2021788//1, OCBBF2024719//1, OCBBF2025458//2, OCBBF2030517//2, OCBBF2030574//3, OCBBF2031167//1, OCBBF2033869//2, OCBBF2038317//2, OCBBF3000483//1, OCBBF3003320//6, OCBBF3004314//1, PEBLM2000170//1, PEBLM2000338//2, PEBLM2002594//2, PEBLM2006113//1, PEBLM2007834//1, PERIC2001227//1,
PERIC2003452//3, PERIC2004909//2, PERIC2006035//7, PERIC2007914//3, PLACE5000171//1, PLACE5000260//2, PLACE6012574//2, PLACE7000514//1, PLACE7001022//1, PROST1000184//2, PROST1000528//2, PROST1000559//1, PROST2018902//1, PROST2018922//1, PUAEN2002489//4, PUAEN2005588//1, PUAEN2006701//3, PUAEN2009174//1, PUAEN2009852//1, RECTM2001347//2,
SMINT1000192//1, SMINT2002743//2, SMINT2009902//4, SMINT2015787//2, SPLEN2001599//1, SPLEN2009548//2, SPLEN2012889//3, SPLEN2015158//1, SPLEN2015679//1, SPLEN2021701//2, SPLEN2023733//7, SPLEN2023791//1, SPLEN2025491//1, SPLEN2029522//1, SPLEN2029683//2, SPLEN2030335//1, SPLEN2030479//1, SPLEN2031125//2, SPLEN2031424//2, SPLEN2031547//6,
SPLEN2031724//3, SPLEN2031780//2, SPLEN2032813//2, SPLEN2033098//1, SPLEN2036326//4, SPLEN2037722//2, SPLEN2038180//2, SPLEN2038345//1, SPLEN2040222//4, SPLEN2041304//1, SPLEN2042598//3, STOMA2008546//3, SYNOV2005817//2, SYNOV2012326//2, SYNOV2016124//1, SYNOV2021320//2, SYNOV4003322//3, SYNOV4004184//1, SYNOV4004741//3, SYNOV4004914//1,
SYNOV4006256//2, SYNOV4007430//1, SYNOV4007553//2, SYNOV4007671//1, SYNOV4008336//2, SYNOV4008440//4, TCERX2000613//1, TESOP2000801//1, TESOP2001345//2, TESOP2001865//2, TESOP2002273//2, TESOP2002539//3, TESOP2005579//1, TESOP2006041//1, TESOP2007052//1, TESOP2007262//1, TESOP2007636//2, TESTI1000257//11, TESTI1000348//3, TESTI2002036//6,
TESTI2002618//2, TESTI2002928//1, TESTI2003347//2, TESTI2005610//1, TESTI2006648//6, TESTI2013382//3, TESTI2024567//5, TESTI2034953//1, TESTI2034997//1, TESTI2035997//1, TESTI2042450//1, TESTI2047071//2, TESTI2048898//2, TESTI2051767//3, TESTI2052822//1, TESTI4000215//2, TESTI4000724//11, TESTI4001176//1, TESTI4001561//2, TESTI4001923//1,
TESTI4002552//4, TESTI4002754//3, TESTI4005805//1, TESTI4005961//1, TESTI4006053//1, TESTI4006137//2, TESTI4007064//3, TESTI4007163//3, TESTI4007239//1, TESTI4007382//1, TESTI4008401//1, TESTI4009608//1, TESTI4013369//3, TESTI4013667//2, TESTI4013830//3, TESTI4016238//2, TESTI4017575//2, TESTI4017901//2, TESTI4018835//2, TESTI4019566//2,
TESTI4020092//1, TESTI4020102//2, TESTI4021478//7, TESTI4023722//2, TESTI4024420//1, TESTI4024874//3, TESTI4024890//2, TESTI4026456//1, TESTI4026785//1, TESTI4027821//1, TESTI4028062//1, TESTI4028429//1, TESTI4028823//4, TESTI4028880//11, TESTI4029836//7, TESTI4030159//3, TESTI4030505//2, TESTI4034172//3, TESTI4035649//2, TESTI4037244//1,
TESTI4041053//2, TESTI4042711//2, TESTI4046487//1, THYMU2003632//4, THYMU2003760//1, THYMU2005003//2, THYMU2005303//1, THYMU2007658//2, THYMU2009425//3, THYMU2011548//6, THYMU2013386//2, THYMU2014353//2, THYMU2019210//2, THYMU2030068//3, THYMU2032035//2, THYMU2032437//1, THYMU2032655//1, THYMU2033308//1, THYMU2033816//4, THYMU2034314//1,
THYMU2035064//2, THYMU2036085//6, THYMU2037226//3, THYMU2037233//1, THYMU2037348//2, THYMU2038772//1, THYMU2038797//1, THYMU2040412//1, THYMU2041015//12, THYMU3000028//1, THYMU3000036//2, THYMU3004835//1, THYMU3006168//8, THYMU3006811//2, THYMU3007368//1, TKIDN2002424//2, TKIDN2002632//1, TKIDN2006525//2, TKIDN2009092//1, TKIDN2009889//1,
TKIDN2014771//2, TKIDN2019116//4, TLIVE2000023//5, TLIVE2001828//2, TLIVE2001927//2, TLIVE2002336//1, TLIVE2002690//2, TLIVE2003381//4, TLIVE2004110//1, TOVAR2001281//1, TRACH1000205//6, TRACH2001549//1, TRACH2001684//2, TRACH2006387//6, TRACH2007059//1, TRACH2008300//1, TRACH2020525//4, TRACH2021964//2, TRACH2022553//2, TRACH2025535//1,
TRACH2025911//1, TRACH3000014//1, TRACH3002064//1, TRACH3002650//2, TRACH3004786//4, TRACH3005294//1, TRACH3006149//1, TRACH3007391//1, TRACH3008629//2, TRACH3035199//3, TRACH3036193//1, TSTOM2000442//2, TUTER2000916//1, UTERU2004688//1, UTERU2004929//1, UTERU2006137//1, UTERU2006568//1, UTERU2007444//1, UTERU2020718//2, UTERU2022020//1,
UTERU2025025//1, UTERU2025891//2, UTERU2026090//1, UTERU2026203//3, UTERU2027591//1, UTERU2029953//3, UTERU2031851//2, UTERU2035323//3, UTERU2035469//1, UTERU3000645//4, UTERU3000899//2, UTERU3001240//4, UTERU3001571//2, UTERU3001585//2, UTERU3002209//3, UTERU3002786//1, UTERU3003116//1, UTERU3003776//1, UTERU3006308//3, UTERU3008671//1,
UTERU3009690//1, UTERU3011063//10, UTERU3016789//2

Deduced amino acid sequences of following 70 clones were also detected to have the transmembrane domains by SOSUI.
BLADE2006830//8, BRACE2002589//1, BRACE2011677//2, BRACE2029396//2, BRACE2039823//3, BRACE2039832//1, BRAMY2019111//5, BRAMY2045471//2, BRAWH2008993//1, BRHIP2003272//1, BRHIP2005724//1, BRHIP2008389//3, BRTHA2011321//2, BRTHA2017972//1, BRTHA2018011//2, BRTHA2018443//6, BRTHA3008826//1, CTONG2003348//1, CTONG2015633//2, CTONG2016942//1,
CTONG2019822//9, CTONG2020974//1, FEBRA2000790//1, FEBRA2006519//1, FEBRA2008692//1, FEBRA2028516//2, HCASM2002754//4, HCASM2003099//3, HEART2009680//7, HLUNG2013350//1, HLUNG2015418//3, IMR322013396//2, LIVER2000247//4, NT2RI2009583//8, NT2RI2027157//6, OCBBF2030116//2, PLACE7000502//2, PROST2019487//2, SPLEN2016932//1, SPLEN2037319//2,
SYNOV2001660//1, SYNOV2013637//4, SYNOV4003981//1, SYNOV4005889//1, TBAES2000932//1, TESOP2001796//2, TESOP2006865//1, TESTI2029252//9, TESTI2032643//3, TESTI2050780//6, TESTI4000137//3, TESTI4000155//1, TESTI4006473//1, TESTI4013894//4, TESTI4014801//1, TESTI4032090//2, TESTI4041086//10, THYMU2004284//1, THYMU2030462//1, THYMU2033401//4,
THYMU2034279//1, THYMU2035710//1, THYMU2040925//3, TKIDN2008778//1, TKIDN2012771//4, TRACH3000420//7, UTERU2011220//1, UTERU2021820//2, UTERU2032279//2, UTERU3015069//2

The 664 clones whose deduced amino acid sequences were detected to have functional domains with Pfam are as follows. The search result is indicated as "Clone Name//Functional Domain Name". When the clone has Multiple Hit Functional Domains, they are listed and demarcated by a double slash mark (//). When the clone has multiple hits of an identical functional domain, each is listed without abridgment.
3NB692002685// R3H domain
3NB692002806// short chain dehydrogenase
3NB692008729// Hrl repeat motif
ADIPS2000088// Immunoglobulin domain// Immunoglobulin domain//
Immunoglobulin domain// Immunoglobulin domain
ASTRO1000009// Delta-aminolevulinic acid dehydratase// FERM domain (Band 4.1 family)
ASTRO2003960// F-box domain.
ASTRO2014923// Ion transport protein
ASTRO3000301// Transmembrane 4 family// Adenovirus E3 region protein CR2
BLADE2005036// Zinc carboxypeptidase
BLADE2007958// WD domain, G-beta repeat// WD domain, G-beta repeat// WD domain, G-beta repeat
BLADE2008539// Carbohydrate phosphorylases
BNGH42003570// EB module// Furin-like cysteine rich region// Thrombospondin type 1 domain
BNGH42007788// WD domain, G-beta repeat// WD domain, G-beta repeat// WD domain, G-beta repeat// WD domain, G-beta repeat// WD domain, G-beta repeat// WD domain, G-beta repeat
BRACE1000258// PH domain// PH domain
BRACE2005457// Sulfate transporter family// Xanthine/uracil permeases family
BRACE2006319// TRAF-type zinc finger// Squash family of serine protease inhibitors// TRAF-type zinc finger
BRACE2008594// Eukaryotic protein kinase domain
BRACE2010489// LysM domain
BRACE2014306// Vesicular monoamine transporter// Ribosomal protein L23// Sugar (and other) transporter// LacY proton/sugar symporter
BRACE2014475// Amidase
BRACE2015314// Bacterial mutT protein
BRACE2016981// Fanconi anaemia group C protein// Bacterial flagellin N-terminus
BRACE2018762// WH1 domain// RanBP1 domain.// Streptomyces extracellular neutral protein// Formyl transferase// K-box region// Tropomyosins
BRACE2026836// Calponin homology (CH) domain
BRACE2027258// Ank repeat// Ank repeat// Ank repeat// Ank repeat// Ank repeat
BRACE2030341// Kinase associated domain 1
BRACE2035381// Lysophospholipase catalytic domain
BRACE2035441// Spectrin repeat// Spectrin repeat// Spectrin repeat
BRACE2038329// TS-N domain// UBA domain
BRACE2041009// TBC domain
BRACE2042550// Thrombospondin type 1 domain// Trypsin Inhibitor like cysteine rich domain// von Willebrand factor type C domain// Thrombospondin type 1 domain
BRACE2043142// Glucose-6-phosphate dehydrogenase
BRACE2044286// CRAL/TRIO domain.// Spectrin repeat
BRACE2045300// Cofilin/tropomyosin-type actin-binding proteins
BRACE2046295// Immunoglobulin domain// EGF-like domain
BRACE2047011// DNA polymerase family B
BRACE3000071// Ank repeat// Ank repeat// Ank repeat
BRACE3000973// Leucine Rich Repeat
BRACE3001002// Lipoprotein
BRACE3001391// Latrophilin/CL-1-like GPS domain// PLAT/LH2 domain// Regulator of G protein signaling domain
BRACE3003192// EGF-like domain// EGF-like domain// EGF-like domain// EGF-like domain// Metallothionein// Keratin, high sulfur B2 protein// EGF-like domain// EGF-like domain// EGF-like domain// EGF-like domain// TB domain// EGF-like domain// EGF-like domain// EGF-like domain// TB domain// EGF-like domain// EGF-like domain
BRACE3004058// FAD/NAD-binding Cytochrome reductase// Oxidoreductase FAD/NAD-binding domain
BRACE3004150// RNA recognition motif. (a.k.a. RRM, RBD, or RNP domain)// RNA recognition motif. (a.k.a. RRM, RBD, or RNP domain)// RNA recognition motif. (a.k.a. RRM, RBD, or RNP domain)
BRACE3004772// SAM domain (Sterile alpha motif)
BRACE3004880// GLTT repeat (12 copies)// GLTT repeat (12 copies)// GLTT repeat (12 copies)// Keratin, high sulfur B2 protein
BRACE3006872// WD domain, G-beta repeat// WD domain, G-beta repeat// WD domain, G-beta repeat
BRACE3007625// Ank repeat// Ank repeat// Ank repeat// Ank repeat// Ank repeat// Ank repeat// Ank repeat// Cytochrome P450
BRACE3008137// PDZ domain (Also known as DHR or GLGF).// PDZ domain (Also known as DHR or GLGF).// PDZ domain (Also known as DHR or GLGF).// PDZ domain (Also known as DHR or GLGF).
BRACE3008384// Rhomboid family
BRACE3008720// GTP1/OBG family// ADP-ribosylation factor family
BRACE3009090// Beige/BEACH domain
BRACE3009708// E1-E2 ATPase// Na+/K+ ATPase C-terminus
BRACE3010397// SCP-like extracellular protein
BRACE3011421// Phorbol esters/diacylglycerol binding dom// Diacylglycerol kinase catalytic domain (presumed)/ Diacylglycerol kinase accessory domain (presumed)// Ank repeat// Ank repeat
BRACE3013576// Hemagglutinin// SPRY domain// Zinc finger, C3HC4 type (RING finger)
BRACE3014005// Putative integral membrane protein
BRACE3015262// Cytochrome P450
BRACE3015521// EF hand
BRACE3016884// Keratin, high sulfur B2 protein// Flagellar L-ring protein
BRACE3019084// SAM domain (Sterile alpha motif)
BRACE3024073// 4Fe-4S iron sulfur cluster binding protein// lactate/malate dehydrogenase// Viral (Superfamily 1) RNA helicase// Ras family
BRACE3025630// Subtilase family
BRACE3027326// LGN motif, putative GEF specific for G-alpha// Rap/ran-GAP
BRACE3027478// Permeases for cytosine/purines, uracil
BRALZ2014484// PH domain
BRALZ2016085// Presenilin// Sugar (and other) transporter// Monocarboxylate transporter
BRAMY2001473// Death domain// ZU5 domain
BRAMY2004771// Leucine Rich Repeat// Leucine rich repeat C-terminal domain// Leucine rich repeat N-terminal domain
BRAMY2005052// Immunoglobulin domain
BRAMY2019300// Leucine Rich Repeat// Leucine rich repeat C-terminal domain// Leucine rich repeat N-terminal domain
BRAMY2021498// Thrombospondin type 1 domain// DnaJ central domain (4 repeats)// Thrombospondin type 1 domain//
Thrombospondin type 1 domain// Thrombospondin type 1 domain//
Thrombospondin type 1 domain
BRAMY2030109// Phorbol esters/diacylglycerol binding domain (C1 domain)// PHD-finger
BRAMY2031317// PDZ domain (Also known as DHR or GLGF).
BRAMY2036567// SH3 domain
BRAMY2039872// Interferon alpha/beta domain
BRAMY2040592// Transient receptor
BRAMY2042760// PH domain// PH domain
BRAMY2046989// TPR Domain// TPR Domain// TPR Domain// TPR Domain// TPR Domain// TPR Domain
BRAMY2047746// Sodium and potassium ATPases// Ank repeat// Ank repeat// Ank repeat
BRAMY2047751// Regulator of G protein signaling domain// Raf-like Ras-binding domain// Raf-like Ras-binding domain
BRAMY3001794// Geminivirus coat protein// PH domain// Leucine Rich Repeat// Leucine Rich Repeat// Leucine Rich Repeat// Leucine Rich Repeat// Leucine Rich Repeat
BRAMY3002803// P21-Rho-binding domain// Eukaryotic protein kinase domain// Eukaryotic protein kinase domain
BRAMY3004224// Leucine rich repeat N-terminal domain// Leucine Rich Repeat// Leucine Rich Repeat// Leucine Rich Repeat// Leucine Rich Repeat// Leucine Rich Repeat// Leucine rich repeat C-terminal domain
BRAMY3004919// Copper/zinc superoxide dismutase (SODC)// Adenylate and Guanylate cyclase catalytic domain// Adenylate and Guanylate cyclase catalytic domain
BRAMY3005091// Phosphatidylinositol 3- and 4-kinases
BRAMY3005932// Ank repeat
BRAMY3008466// WD domain, G-beta repeat// WD domain, G-beta repeat// WD domain, G-beta repeat// WD domain, G-beta repeat// WD domain, G-beta repeat// WD domain, G-beta repeat
BRAMY4000095// Eukaryotic protein kinase domain
BRAMY4000277// Immunoglobulin domain// Immunoglobulin domain
BRAWH1000127// Plexin repeat// Thrombospondin type 1 domain
BRAWH2001395// Myelin basic protein
BRAWH2001940// NOL1/NOP2/sun family
BRAWH2007658// Immunoglobulin domain
BRAWH2010000// Xylose isomerase
BRAWH2014414// Cadherin domain// Cadherin domain// Cadherin domain// Fructose-bisphosphate aldolase class-// Cadherin domain// Cadherin domain// Cadherin cytoplasmic region
BRAWH2014662// K+ channel tetramerisation domain// ATP synthase Alpha chain, C terminal
BRAWH2014954// PDZ domain (Also known as DHR or GLGF).// PDZ domain (Also known as DHR or GLGF).
BRAWH2016702// AMP-binding enzyme
BRAWH3000078// Adaptin N terminal region// Activin types I and II receptor domain
BRAWH3000314// Fibronectin type III domain
BRAWH3000491// Ribosomal protein S12e
BRAWH3001326// Protein phosphatase 2C
BRAWH3001891// YCF9
BRAWH3002574// Calpain large subunit, domain III// EF hand
BRAWH3002600// Cadherin domain// Cadherin domain// Cadherin domain
BRAWH3002821// C2 domain// C2 domain
BRAWH3003727// ribonuclease.
BRAWH3005912// bZIP transcription factor// bZIP transcription factor// Troponin// TBC domain
BRAWH3008341// Pentaxin family
BRCAN2002562// RNA recognition motif. (a.k.a. RRM, RBD, or RNP domain)
BRCAN2002856// Phosphotyrosine interaction domain (PTB/PID).
BRCAN2002948// Adaptin N terminal region
BRCAN2006063// von Willebrand factor type A domain
BRCAN2009203// SAM domain (Sterile alpha motif)
BRCAN2009432// ADP-ribosylation factor family// Ras family
BRCAN2015464// Gag P30 core shell protein
BRCAN2016619// SH3 domain
BRCAN2017717// Squash family of serine protease inhibitors
BRCAN2021028// Aminopeptidase I zinc metalloprotease (M18)
BRCAN2024451// Raf-like Ras-binding domain// Leptin// Raf-like Ras-binding domain// LGN motif, putative GEF specific for G-alpha GTPase
BRCAN2028355// Eukaryotic protein kinase domain
BRCOC2001505// Myelin basic protein
BRCOC2003213// ATP synthase, Delta/Epsilon chain// tRNA synthetase class II core domain (G, H, P, S and T)
BRCOC2016525// Ank repeat// Ank repeat// Ank repeat// Ank repeat// Ank repeat// Ank repeat// Ank repeat
BRHIP2000819// WD domain, G-beta repeat
BRHIP2000920// Ribosomal protein S9/S16
BRHIP2003786// Ank repeat// Ank repeat// Ank repeat// BTB/POZ domain
BRHIP2004359// Metallo-beta-lactamase superfamily
BRHIP2004814// Phosphate transporter family
BRHIP2005236// Galactose binding lectin domain// Latrophilin Cytoplasmic C-terminal region
BRHIP2005932// PH domain
BRHIP2007616// Sema domain
BRHIP2009414// Uncharacterized protein family
BRHIP2021615// RNA recognition motif. (a.k.a. RRM, RBD, or RNP domain)// RNA recognition motif. (a.k.a. RRM, RBD, or RNP domain)
BRHIP2026288// Prolyl oligopeptidase family// alpha/beta hydrolase fold
BRHIP3000339// Myelin basic protein
BRHIP3008183// Adaptin N terminal region// tRNA (Guanine-1)-methyltransferase
BRHIP3008313// Ank repeat
BRHIP3008405// PH domain
BRHIP3024118// Sodium:galactoside symporter family// Monocarboxylate transporter
BRHIP3025161// Phosphotriesterase family// RhoGEF domain// PH domain// Thaumatin family// GATA zinc finger// FYVE zinc finger// PH domain
BRHIP3027137// Aldehyde dehydrogenase family
BRSSN2000684// Protein-tyrosine phosphatase// Dual specificity phosphatase, catalytic domain
BRSSN2004719// Src homology domain 2
BRSTN2000872// Thioredoxin// Thioredoxin
BRSTN2001067// Rifin/stevor family
BRSTN2004863// Chitin synthase// Glycosyl transferases// Similarity to lectin domain of ricin beta-chain, 3 copies.
BRSTN2004987// tRNA synthetases class I (W and Y)
BRSTN2008418// RhoGAP domain
BRSTN2013741// Ras family
BRTHA2000855// Extracellular link domain
BRTHA2004978// Collagen triple helix repeat (20 copies)
BRTHA2005579// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain
BRTHA2007122// Ank repeat// Ank repeat// Ank repeat// Ank repeat// SAM domain (Sterile alpha motif)
BRTHA2008527// Leucine Rich Repeat// Leucine Rich Repeat// Leucine Rich Repeat// Leucine Rich Repeat// Leucine Rich Repeat
BRTHA2009311// Vertebrate galactoside-binding lectins
BRTHA2010884// Thrombospondin type 1 domain// CUB domain
BRTHA2012980// Cytochrome P450
BRTHA2013262// Keratin, high sulfur B2 protein
BRTHA2014792// SET domain
BRTHA2015406// UBA domain
BRTHA2015878// Gram-negative pili assembly chaperone
BRTHA2016496// Peptidase C13 family
BRTHA2018591// GTPase of unknown function
BRTHA2018624// Galactose binding lectin domain// Activin types I and II receptor domain// Galactose binding lectin domain
BRTHA2018707// RNA recognition motif. (a.k.a. RRM, RBD, or RNP domain)
BRTHA2019048// Domain of unknown function DUF71
BRTHA3002401// Ornithine decarboxylase antizyme
BRTHA3002427// Sulfate transporter family// Sodium:neurotransmitter symporter family
BRTHA3003074// Fanconi anaemia group C protein
BRTHA3003449// Myosin head (motor domain)
BRTHA3005046// Collagen triple helix repeat (20 copies)
BRTHA3008310// Homeobox domain
BRTHA3008778// AMP-binding enzyme
BRTHA3009037// C2 domain// PDZ domain (Also known as DHR or GLGF).// Regulator of G protein signaling domain// Regulator of G protein signaling domain
BRTHA3009090// Cyclic nucleotide-binding domain// Cyclic nucleotide-binding domain// Cyclic nucleotide-binding domain// Glutathione S-transferases.// Uncharacterized protein family UPF0028
BRTHA3013884// Domain associated with PX domains// PX domain// 60s Acidic ribosomal protein
BRTHA3015815// AIR synthase related protein
BRTHA3016917// tRNA synthetases class I (C)// tRNA synthetases class I (I, L, M and V)
BRTHA3017589// Immunoglobulin domain// Immunoglobulin domain// Hantavirus glycoprotein G2
BRTHA3017848// Glucose-6-phosphate dehydrogenase// Sugar (and other) transporter
BRTHA3018656// Divalent cation transporter// Divalent cation transporter
COLON2000568// Immunoglobulin domain// Cellulose binding domain// Immunoglobulin domain// Immunoglobulin domain// Immunoglobulin domain// Immunoglobulin domain
COLON2001721// C2 domain
COLON2002520// Myosin head (motor domain)// IQ calmodulin-binding motif
COLON2004478// Immunoglobulin domain// Immunoglobulin domain// Immunoglobulin domain// Immunoglobulin domain// Immunoglobulin domain
CORDB1000140// TRAF-type zinc finger
CORDB2000541// F-actin capping protein, beta subunit
CTONG1000341// EGF-like domain// EGF-like domain// Metallothionein// EGF-like domain// EB module// EGF-like domain// EGF-like domain// EGF-like domain
CTONG1000467// Zinc finger, C3HC4 type (RING finger)
CTONG2000042// Bacterial regulatory proteins, gntR family// Alpha-2-macroglobulin family N-terminal region// Alpha-2-macroglobulin family
CTONG2001877// MutT-like domain
CTONG2004062// E1-E2 ATPase
CTONG2006798// Eukaryotic protein kinase domain// Eukaryotic protein kinase domain
CTONG2008233// DnaJ domain
CTONG2009423// 7 transmembrane receptor (rhodopsin family) CTONG2013178// SEA domain// Trypsin
CTONG2017500// F-box domain.
CTONG2020026// Herpesvirus VP23 like capsid protein
CTONG2024206// Neuregulin family// von Willebrand factor type A domain// EGF-like domain// Response regulator receiver domain// von Willebrand factor type A domain// von Willebrand factor type A domain
CTONG2024749// Alpha-2-macroglobulin family
CTONG2025496// Alpha-2-macroglobulin family N-terminal region// Alpha-2-macroglobulin family
CTONG2028124// AMP-binding enzyme
CTONG2028687// TPR Domain// TPR Domain
CTONG3000084// DNA mismatch repair protein// RhoGEF domain// PH domain// SH3 domain
CTONG3000657// SH3 domain
CTONG3000686// TPR Domain// TPR Domain// TPR Domain// TPR Domain
CTONG3001123// BRCA1 C Terminus (BRCT) domain// BRCA1 C Terminus (BRCT) domain// BRCA1 C Terminus (BRCT) domain// BRCA1 C Terminus (BRCT) domain// BRCA1 C Terminus (BRCT) domain
CTONG3001370// Alpha-2-macroglobulin family N-terminal region// Alpha-2-macroglobulin family
CTONG3002127// C2 domain// C2 domain
CTONG3002674// Calponin homology (CH) domain
CTONG3003737// Leucine rich repeat N-terminal domain// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine rich repeat C-terminal domain// Fusion glycoprotein F0.
CTONG3003972// RNA recognition motif. (a.k.a. RRM, RBD, or RNP domain)
CTONG3004072// Beta type Zein// Keratin, high sulfur B2 protein
CTONG3005325// TS-N domain// UBA domain// Transposase
CTONG3005648// Putative undecaprenyl diphosphate synthase
CTONG3006067// DnaJ central domain (4 repeats)
CTONG3006186// PDZ domain (Also known as DHR or GLGF)// Apolipoprotein A1/A4/E family// WW domain
CTONG3008831// RNA recognition motif. (a.k.a. RRM, RBD, or RNP domain)
CTONG3009028// Helicases conserved C-terminal domain
CTONG3009385// TPR Domain// TPR Domain// TPR Domain// TPR Domain// TPR Domain// TPR Domain// TPR Domain// TPR Domain// TPR Domain// TPR Domain
D3OST2002182// Glycosyl transferase family 8
D3OST2002648// 7 transmembrane receptor (rhodopsin family)// 7 transmembrane receptor (rhodopsin family)
D3OST3000169// SH3 domain// SAM domain (Sterile alpha motif)
DFNES2000146// Plexin repeat// Thrombospondin type 1 domain
DFNES2001108// PH domain
DFNES2005266// Thrombospondin type 1 domain
DFNES2011499// WD domain, G-beta repeat
FCBBF3004502// Terpene synthase family// YCF9
FCBBF3007540// RhoGEF domain// PH domain
FCBBF3009888// Keratin, high sulfur B2 protein// u-PAR/Ly-6 domain
FCBBF3012170// Thrombospondin type 1 domain
FCBBF3012288// Fibronectin type III domain
FCBBF3013307// DEAD/DEAH box helicase// Helicases conserved C-terminal domain
FEBRA2000253// Flagellar L-ring protein
FEBRA2007708// Fusion glycoprotein F0.// Xanthine/uracil permeases family// Sulfate transporter family
FEBRA2007801// IBR domain
FEBRA2008311// 7 transmembrane receptor (rhodopsin family)// 7 transmembrane receptor (rhodopsin family)
FEBRA2008468// alpha/beta hydrolase fold
FEBRA2021571// von Willebrand factor type D domain
FEBRA2024150// DENN (AEX-3) domain
FEBRA2026984// tRNA synthetases class I (W and Y)// Putative tRNA binding domain
HCASM2001301// Eukaryotic protein kinase domain
HCASM2002918// ATP synthase Alpha chain, C terminal
HCHON2000028// RhoGAP domain
HCHON2001084// FecCD transport family// Sugar (and other) transporter
HCHON2001217// Cullin family
HCHON2001577// Collagen triple helix repeat (20 copies)// Heavy-metal-associated domain
HCHON2001712// Sodium:dicarboxylate symporter family
HCHON2002676// Glycosyl hydrolases family 39
HCHON2004007// E1-E2 ATPase
HCHON2004531// Ubiquitin family// UBA domain// Integrins, beta chain// UBA domain
HCHON2004776// Protein of unknown function DUF93
HCHON2005921// PMP-22/EMP/MP20/Claudin family
HCHON2006250// WD domain, G-beta repeat// WD domain, G-beta repeat// WD domain, G-beta repeat// WD domain, G-beta repeat
HEART1000139// Troponin
HEART2001680// Immunoglobulin domain// Immunoglobulin domain// Immunoglobulin domain// Immunoglobulin domain// Immunoglobulin domain
HEART2001756// Bacterial transcriptional regulator
HEART2006131// Thiamine pyrophosphate enzymes
HEART2006909// CBS domain// CBS domain
HEART2010495// Tau and MAP proteins, tubulin-binding
HHDPC1000118// Adenylate kinase// Shikimate kinase// Deoxynucleoside kinase// Pyridoxal-phosphate dependent enzyme
HLUNG1000017// Reprolysin family propeptide
HLUNG2000014// Lectin C-type domain
HLUNG2001996// SH3 domain
HLUNG2002465// PH domain// RhoGEF domain// SH3 domain
HLUNG2002958// EF hand
HLUNG2011298// Oxidoreductase FAD/NAD-binding domain
HLUNG2013851// Pumilio-family RNA binding domains (aka PUM-HD, Pumilio homology domain)// Pumilio-family RNA binding domains (aka PUM-HD, Pumilio homology domain)
HLUNG2014262// von Willebrand factor type A domain// von Willebrand factor type A domain
HLUNG2017350// Connexin
HSYRA2005456// Fibronectin type III domain
HSYRA2005496// emp24/gp25L/p24 family
HSYRA2009075// Fibronectin type III domain
HSYRA2009102// Integral membrane protein DUF6
IMR322000127// Zinc finger, C2H2 type
IMR322000917// Zinc finger, C2H2 type
IMR322006495// Tropomyosins
KIDNE1000064// Integral membrane protein DUF7// Sugar (and other) transporter// Transmembrane 4 family// Zn-finger in Ran binding protein and others.
KIDNE2000832// Amino acid permease// Transmembrane amino acid transporter protein// Sodium/hydrogen exchanger family
KIDNE2000846// Sodium:neurotransmitter symporter family
KIDNE2001361// Domain of unknown function DUF19
KIDNE2001847// RhoGAP domain// SH3 domain
KIDNE2006580// Cytochrome P450
KIDNE2011635// Sodium:solute symporter family
KIDNE2012945// CUB domain// Pentaxin family
LYMPB2000083// Class I Histocompatibility antigen, domains alpha 1 and 2// Class I Histocompatibility antigen, domains alpha 1 and 2// Immunoglobulin domain
MESAN2006563// PH domain
MESAN2012054// PQQ enzyme repeat// PQQ enzyme repeat
NESOP2001433// Immunoglobulin domain// Immunoglobulin domain// Immunoglobulin domain// Immunoglobulin domain// Immunoglobulin domain
NESOP2001656// Polyomavirus coat protein
NHNPC2001816// Regulator of G protein signaling domain
NOVAR2000136// Thioredoxin// CTF/NF-I family// Calsequestrin
NOVAR2001108// Immunoglobulin domain// Immunoglobulin domain// Immunoglobulin domain// Immunoglobulin domain
NT2NE2003252// Eukaryotic protein kinase domain
NT2NE2006531// KRAB box// PHD-finger// Transcription factor S-II (TFIIS)// Zinc finger, C2H2 type
NT2NE2006909// Influenza Matrix protein (M1)// metallopeptidase family M24
NT2RI2004618// Cytosolic long-chain acyl-CoA thioestease
NT2RI2005166// F-box domain.// WD domain, G-beta repeat
NT2RI2008724// GGL domain
NT2RI2025909// Mitochondrial carrier proteins// Mitochondrial carrier proteins// Mitochondrial carrier proteins
NT2RI2025957// PDZ domain (Also known as DHR or GLGF).
NT2RI3000622// TBC domain
NT2RI3002842// Hsp20/alpha crystallin family
NT2RI3003382// Rotavirus RNA-binding Protein 53 (NS53)
NT2RI3004510// Pyridine nucleotide-disulphide oxidoreductase// FAD binding domain// Flavin containing amine oxidase// Phytoene dehydrogenase related enzyme
NT2RI3006171// Immunoglobulin domain// Immunoglobulin domain// Immunoglobulin domain// Immunoglobulin domain
NT2RI3006340// Immunoglobulin domain// Immunoglobulin domain//
Fibronectin type III domain// Fibronectin type III domain//
Fibronectin type III domain// Fibronectin type III domain// Fibronectin type III domain// Immunoglobulin domain// Ribosomal protein S14p/S29e // Immunoglobulin domain// Immunoglobulin domain
NT2RI3006376// DENN (AEX-3) domain// PLAT/LH2 domain
NT2RI3006673// Fibronectin type III domain// Fibronectin type III domain// Fibronectin type III domain// Protein-tyrosine phosphatase// Dual specificity phosphatase, catalytic domain// Protein-tyrosine phosphatase
NT2RI3007158// FYVE zinc finger
NT2RI3007291// Collagen triple helix repeat (20 copies)
NT2RI3007543// DnaJ domain
NT2RI3007978// Glutamine amidotransferase class-I
NT2RI3008652// FERM domain (Band 4.1 family)// Uncharacterised protein family UPF0058// Biopterin-dependent aromatic amino acid hydroxylase
NT2RP7000359// FERM domain (Band 4.1 family)// Insulin-like growth factor binding proteins// PDZ domain (Also known as DHR or GLGF).
NT2RP7000466// CUB domain// CXXC zinc finger// EGF-like domain// Granulins// Keratin, high sulfur B2 protein// Trypsin Inhibitor like cysteine rich domain
NT2RP7004027// CUB domain// Sushi domain (SCR repeat)
NT2RP7004123// Hepatitis delta virus delta antigen
NT2RP7005118// GTPase-activator protein for Ras-like GTPase// IQ calmodulin-binding motif// WW domain
NT2RP7005529// PH domain// RhoGEF domain
NT2RP7009147// WD domain, G-beta repeat// WD domain, G-beta repeat// WD domain, G-beta repeat
NT2RP7010599// Lipase
NT2RP7011570// Gag P30 core shell protein
NT2RP7013795// WD domain, G-beta repeat// WD domain, G-beta repeat
NT2RP7014005// Glutamine amidotransferase class-I
NT2RP7017474// Phosphoglucose isomerase
NT2RP8000296// BTB/POZ domain// Kelch motif// Kelch motif// Kelch motif// Kelch motif// Kelch motif// Kelch motif
NT2RP8000483// RhoGAP domain
NTONG2000413// Astacin (Peptidase family M12A)// Hemopexin// Matrixin
NTONG2003852// Phosphotyrosine interaction domain (PTB/PID).
NTONG2005277// Ank repeat// Ank repeat// Ank repeat// Ank repeat// Ank repeat// Ank repeat// Ank repeat// Ank repeat
NTONG2006354// Ank repeat
NTONG2007517// BTB/POZ domain
OCBBF2004826// PH domain// Raf-like Ras-binding domain// Transaldolase// PDZ domain (Also known as DHR or GLGF).// RhoGEF domain
OCBBF2004883// WD domain, G-beta repeat// WD domain, G-beta repeat// WD domain, G-beta repeat// WD domain, G-beta repeat// WD domain, G-beta repeat
OCBBF2006058// Acyl-CoA dehydrogenase
OCBBF2006764// Sushi domain (SCR repeat)// CUB domain// Sushi domain (SCR repeat)// CUB domain// Sushi domain (SCR repeat)
OCBBF2007028// SH3 domain
OCBBF2007068// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ribosomal protein L34// Ank repeat// Ank repeat// Ank repeat// Ank repeat
OCBBF2008770// TBC domain
OCBBF2010140// Alphavirus E1 glycoprotein
OCBBF2010416// Major intrinsic protein
OCBBF2019823// lactate/malate dehydrogenase
OCBBF2020838// Fork head domain
OCBBF2021323// Regulatory subunit of type II PKA R-subunit
OCBBF2022351// WD domain, G-beta repeat// WD domain, G-beta repeat// WD domain, G-beta repeat// WD domain, G-beta repeat// WD domain, G-beta repeat// WD domain, G-beta repeat
OCBBF2025527// NAD-dependent glycerol-3-phosphate dehydrogenase
OCBBF2031167// Reprolysin family propeptide// Pancreatic hormone peptides// Reprolysin (M12B) family zinc metalloprotease// Disintegrin// Beta defensin// Radical activating enzymes// EB module// EGF-like domain// Delta serrate ligand
OCBBF2033869// CUB domain
OCBBF2035110// PLAT/LH2 domain
OCBBF2036743// KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type
OCBBF2037340// Alpha crystallin A chain, N terminal// Archaeal ATPase// Dual specificity phosphatase, catalytic domain OCBBF2037547// PH domain// Raf-like Ras-binding domain// Transaldolase// PDZ domain (Also known as DHR or GLGF).// RhoGEF domain// PH domain
OCBBF2037598// Immunoglobulin domain// Immunoglobulin domain// Immunoglobulin domain// Fibronectin type III domain// Fibronectin type III domain
OCBBF2037638// Pyridine nucleotide-disulphide oxidoreductase// Pyridine nucleotide-disulphide oxidoreductase
OCBBF2038317// Syndecan domain// BNR repeat// BNR repeat// BNR repeat// BNR repeat// BNR repeat// PKD domain
OCBBF3009279// KH domain// Zinc finger, C3HC4 type (RING finger)
PEBLM2002594// ABC transporter// Aldehyde oxidase and xanthine dehydrogenase, C terminus
PEBLM2004666// WD domain, G-beta repeat// Gram-negative pili assembly chaperone// WD domain, G-beta repeat// WD domain, G-beta repeat// WD domain, G-beta repeat// WD domain, G-beta repeat// WD domain, G-beta repeat
PERIC1000147// Syndecan domain
PERIC2001228// Leucine Rich Repeat// Leucine Rich Repeat// Leucine Rich Repeat// Leucine Rich Repeat// Leucine Rich Repeat// Leucine Rich Repeat// Leucine Rich Repeat
PERIC2003720// Ezrin/radixin/moesin family
PERIC2009086// 7 transmembrane receptor (rhodopsin family)// Peroxidase
PLACE5000171// Chitin binding Peritrophin-A domain// HYR domain// Plant PEC family metallothionein// Sushi domain (SCR repeat)// von Willebrand factor type A domain
PLACE5000282// Collagen triple helix repeat (20 copies)// Heavy-metal-associated domain
PLACE6012574// ENV polyprotein (coat polyprotein)
PLACE6019385// REV protein (anti-repression trans-activator protein)
PLACE6020031// Ank repeat// Ank repeat
PLACE7000514// Filamin/ABP280 repeat.
PLACE7002641// LPP20 lipoprotein precursor// HRDC domain// Dihydrodipicolinate synthetase family
PLACE7006051// ENV polyprotein (coat polyprotein)
PLACE7008431// Phosphatidylinositol-4-phosphate 5-Kinase
PROST1000184// 7 transmembrane receptor (Secretin family)
PROST2008993// BRCA1 C Terminus (BRCT) domain
PROST2016462// WW domain// PH domain// RhoGAP domain
PROST2017367// Transglutaminase family
PROST2018090// Sushi domain (SCR repeat)// Sushi domain (SCR repeat)// Chitin binding Peritrophin-A domain// HYR domain// Sushi domain (SCR repeat)
PROST2018511// Ras association (RalGDS/AF-6) domain// PH domain// Src homology domain 2
PUAEN2002616// Src homology domain 2
PUAEN2005930// Extracellular link domain// PH domain
PUAEN2006328// TBC domain
PUAEN2007044// TruB family pseudouridylate synthase (N terminal domain)
PUAEN2009174// L1 (late) protein// Alpha-2-macroglobulin family
PUAEN2009795// Ribosomal protein S3, C-terminal domain// EGF-like domain// Clq domain
PUAEN2009852// Eukaryotic protein kinase domain
RECTM2000433// Jacalin-like lectin domain
SKMUS2006394// Ank repeat// Ank repeat// Ank repeat// Ank repeat// Ank repeat// Ank repeat// Ank repeat
SMINT1000192// Small hydrophilic plant seed proteins
SMINT2002743// ENV polyprotein (coat polyprotein)
SMINT2010076// Immunoglobulin domain// Immunoglobulin domain// Immunoglobulin domain// Immunoglobulin domain// Immunoglobulin domain
SMINT2011888// Immunoglobulin domain// Cellulose binding domain// Immunoglobulin domain// Immunoglobulin domain// Immunoglobulin domain// Immunoglobulin domain
SMINT2015787// Serum amyloid A protein// Immunoglobulin domain
SPLEN2001599// Immunoglobulin domain
SPLEN2002147// Phosphatidylinositol transfer protein
SPLEN2002467// DB module// F-box domain.// Leucine Rich Repeat
SPLEN2006122// RNA recognition motif. (a.k.a. RRM, RBD, or RNP domain)// RNA recognition motif. (a.k.a. RRM, RBD, or RNP domain)
SPLEN2010912// DEAD/DEAH box helicase// Helicases conserved C-terminal domain
SPLEN2012624// Ank repeat// Ank repeat// Ank repeat// Sodium:neurotransmitter symporter family
SPLEN2015267// Immunoglobulin domain// Immunoglobulin domain// Immunoglobulin domain// Immunoglobulin domain
SPLEN2015679// ATP synthase delta (OSCP) subunit
SPLEN2021701// Class I Histocompatibility antigen, domains alpha 1 and 2// Class I Histocompatibility antigen, domains alpha 1 and 2// Immunoglobulin domain
SPLEN2030335// AMP-binding enzyme
SPLEN2031547// Integral membrane protein// Integral membrane protein
SPLEN2031780// Domain of unknown function DUF139// Domain of unknown function DUF139
SPLEN2033098// TNFR/NGFR cysteine-rich region
SPLEN2034081// Insulin-like growth factor binding proteins
SPLEN2036326// GPR1/FUN34/yaaH family// PMP-22/EMP/MP20/Claudin family
SPLEN2036821// Mitochondrial carrier proteins
SPLEN2037722// Immunoglobulin domain// Immunoglobulin domain
STOMA2004294// Immunoglobulin domain
SYNOV2005448// Apidaecin
SYNOV2005817// Domain of unknown function DUF19// Tissue factor
SYNOV2006430// Nitrogen regulatory protein P-II
SYNOV2014400// EGF-like domain// Granulins// Granulins// EGF-like domain
SYNOV2021320// Src homology domain 2
SYNOV3000231// Immunoglobulin domain// Immunoglobulin domain// Immunoglobulin domain// Immunoglobulin domain
SYNOV3000302// Immunoglobulin domain// Immunoglobulin domain// Immunoglobulin domain// Immunoglobulin domain
SYNOV4002392// lactate/malate dehydrogenase
SYNOV4002883// Adenosylmethionine decarboxylase
SYNOV4007521// Immunoglobulin domain// Immunoglobulin domain
SYNOV4007553// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine rich repeat C-terminal domain// TIR domain
SYNOV4007671// Syntaxin// Fusion glycoprotein F0.
SYNOV4008440// Adaptin N terminal region
TBAES2001171// NOL1/NOP2/sun family
TBAES2001229// Ribosomal protein L23
TBAES2003550// Glucose-6-phosphate dehydrogenase
TBAES2004055// Ribosomal protein S11
TESOP2000801// Eukaryotic protein kinase domain
TESOP2001166// Src homology domain 2
TESOP2001953// Leucine Rich Repeat// Leucine Rich Repeat// Leucine Rich Repeat// Leucine Rich Repeat// Leucine Rich Repeat// Leucine Rich Repeat// Leucine Rich Repeat
TESOP2004114// Lysyl hydrolase// Lysyl hydrolase
TESOP2005485// Immunoglobulin domain// Immunoglobulin domain
TESOP2009121// DNA polymerase (viral) C-terminal domain
TESTI1000257// GntP family permease// Sugar (and other) transporter
TESTI1000390// Bromodomain// Atrial natriuretic peptide
TESTI1000545// TPR Domain// TPR Domain// TPR Domain// LPP20 lipoprotein precursor// HRDC domain// Adaptin N terminal region// Dihydrodipicolinate synthetase family
TESTI2000443// Leucine Rich Repeat// Leucine Rich Repeat// Leucine Rich Repeat
TESTI2000644// Small cytokines (intecrine/chemokine), interleukin-8 like
TESTI2002036// Ion transport protein// Transmembrane region cyclic Nucleotide Gated Channel
TESTI2002618// Reprolysin (M12B) family zinc metalloprotease// Reprolysin family propeptide
TESTI2002928// Syndecan domain
TESTI2003347// Connexin// Cytochrome b559, alpha (gene psbE) and beta (gene psbF)subunits.
TESTI2004700// Leucine Rich Repeat// Leucine Rich Repeat// Leucine Rich Repeat// Leucine Rich Repeat
TESTI2005610// CD36 family
TESTI2006648// Ion transport protein// ABC transporter transmembrane region.// PEP-utilizing enzymes// Phosphoribulokinase// Elongation factor Tu family
TESTI2014716// RNA recognition motif. (a.k.a. RRM, RBD, or RNP domain)// RNA recognition motif. (a.k.a. RRM, RBD, or RNP domain)// RNA recognition motif. (a.k.a. RRM, RBD, or RNP domain)
TESTI2024567// 7 transmembrane receptor (metabotropic glutamate family)
TESTI2026505// Domain of unknown function DUF123// FYVE zinc finger// PH domain// RhoGEF domain
TESTI2027019// Leucine Rich Repeat
TESTI2034520// ABC transporter
TESTI2034767// Collagen triple helix repeat (20 copies)// Collagen triple helix repeat (20 copies)// Collagen triple helix repeat (20 copies)// Collagen triple helix repeat (20 copies)// Collagen triple helix repeat (20 copies)// Collagen triple helix repeat (20 copies)// Collagen triple helix repeat (20 copies)
TESTI2040018// Hepatitis C virus RNA dependent RNA polymerase
TESTI2044796// Zinc finger, C3HC4 type (RING finger)
TESTI2049469// Chitinases class I
TESTI2050137// Phosphotyrosine interaction domain (PTB/PID)// Src homology domain 2
TESTI2050987// Zinc finger, C3HC4 type (RING finger)// SPRY domain
TESTI2051867// Ribosomal protein L4/L1 family
TESTI2052693// Src homology domain 2
TESTI2053621// EF hand// EF hand// Glutathione peroxidases// EF hand
TESTI4000014// PPR repeat// PPR repeat// PPR repeat// PPR repeat// PPR repeat// ENTH domain// PPR repeat// PPR repeat// PPR repeat// Ribosomal protein L22p/L17e// Interleukin 10// PPR repeat
TESTI4000079// Phosphopantetheine attachment site// PH domain
TESTI4000288// Dynamin GTPase effector domain
TESTI4000349// HECT-domain (ubiquitin-transferase).
TESTI4000462// Keratin, high sulfur B2 protein
TESTI4000724// Vesicular monoamine transporter// Sugar (and other) transporter// Monocarboxylate transporter
TESTI4000970// Ezrin/radixin/moesin family
TESTI4001148// Enol-ase// ATP synthase delta (OSCP) subunit
TESTI4001527// UDP-glucoronosyl and UDP-glucosyl transferase
TESTI4001561// Acyltransferase
TESTI4002491// NSF attachment protein
TESTI4002552// E1-E2 ATPase// Na+/K+ ATPase C-terminus
TESTI4006326// von Willebrand factor type A domain
TESTI4006546// Tudor domain// Tudor domain// Tudor domain
TESTI4006819// Enol-ase
TESTI4007064// DENN (AEX-3) domain// PPR repeat// LIM domain containing proteins
TESTI4007163// Sodium:neurotransmitter symporter family
TESTI4007382// Nickel-dependent hydrogenases
TESTI4007778// Calponin homology (CH) domain// Calponin homology (CH) domain// Spectrin repeat// Spectrin repeat// Spectrin repeat// Spectrin repeat// EF hand// EF hand
TESTI4007810// DNA ligase
TESTI4008429// E1-E2 ATPase// Domain of unknown function
TESTI4009160// Kinesin motor domain// Kinesin motor domain
TESTI4009374// RNA recognition motif. (a.k.a. RRM, RBD, or RNP domain)// Ham1 family// RNA recognition motif. (a.k.a. RRM, RBD, or RNP domain)// RNA recognition motif. (a.k.a. RRM, RBD, or RNP domain)// Double-stranded RNA binding motif
TESTI4009608// emp24/gp25L/p24 family
TESTI4009881// Bacterial type II secretion system protein
TESTI4010713// C2 domain
TESTI4010831// WD domain, G-beta repeat
TESTI4010851// Ubiquitin carboxyl-terminal hydrolases// Ubiquitin carboxyl-terminal hydrolase f// eIF4-gamma/eIF5/eIF2-epsilon// Rifin/stevor family
TESTI4011484// SAM domain (Sterile alpha motif)
TESTI4011745// Bromodomain
TESTI4011956// PH domain
TESTI4012406// Kringle domain
TESTI4012448// Matrixin// Hemopexin// Hemopexin// Hemopexin// Hemopexin
TESTI4012679// DNA photolyase
TESTI4013369// ATP synthase subunit C
TESTI4014924// Floricaula / Leafy protein
TESTI4015471// Tropomyosins
TESTI4016110// DnaJ domain
TESTI4016882// SH3 domain// SH3 domain
TESTI4016925// Aminotransferases class-III// Pyridoxal-phosphate dependent enzyme
TESTI4017001// bZIP transcription factor
TESTI4017137// Keratin, high sulfur B2 protein
TESTI4017575// MSP (Major sperm protein) domain
TESTI4018152// FERM domain (Band 4.1 family)
TESTI4018555// Granulins
TESTI4018835// E1-E2 ATPase// E1-E2 ATPase
TESTI4018886// Fibronectin type III domain// Fibronectin type III domain// Fibronectin type III domain
TESTI4019140// GATA zinc finger
TESTI4019566// Helicases conserved C-terminal domain// Tudor domain
TESTI4019843// SH3 domain// RhoGEF domain// PH domain
TESTI4020092// Laminin G domain
TESTI4020920// D-isomer specific 2-hydroxyacid dehydrogenase, catalytic domain
TESTI4021294// Cyclin// Immunoglobulin domain
TESTI4021478// E1-E2 ATPase// E1-E2 ATPase// E1-E2 ATPase
TESTI4022716// DEAD/DEAH box helicase// Helicases conserved C-terminal domain
TESTI4023555// Lectin C-type domain
TESTI4025920// Adaptin N terminal region
TESTI4026192// Domain of unknown function
TESTI4026510// DEAD/DEAH box helicase// Helicases conserved C-terminal domain
TESTI4027557// Vertebrate galactoside-binding lectins// Vertebrate galactoside-binding lectins
TESTI4028059// Phosphofructokinase// Phosphofructokinase
TESTI4028429// WAP-type (Whey Acidic Protein) 'four-disulfide core'
TESTI4028612// Major intrinsic protein
TESTI4028880// Sugar (and other) transporter//
Sodium:galactoside symporter family
TESTI4028983// Serum amyloid A protein
TESTI4029836// E1-E2 ATPase// E1-E2 ATPase// Neuraxin and MAP1B proteins// E1-E2 ATPase// Cof family
TESTI4030505// Metallothionein family 5
TESTI4030603// Collagen triple helix repeat (20 copies)
TESTI4032895// ATP synthase, Delta/Epsilon chain//
Tropomyosins// Protein of unknown function
TESTI4034432// Peptidyl-tRNA hydrolase domain
TESTI4034632// Ribosomal protein S3, C-terminal domain//
Similarity to lectin domain of ricin beta-chain, 3 copies
TESTI4034912// Adhesin lipoprotein// Vesiculovirus phosphoprotein
TESTI4035063// Myosin tail// CAP-Gly domain
TESTI4035498// Cell division protein
TESTI4036909// Viral (Superfamily 1) RNA helicase// Heavy-metal-associated domain// Viral (Superfamily 1) RNA helicase
TESTI4038492// Serum amyloid A protein
TESTI4039659// DnaJ domain
TESTI4041053// Ank repeat// Ank repeat// Ank repeat// Ank repeat// Armadillo/beta-catenin-like repeats// Armadillo/beta-catenin-like repeats// Armadillo/beta-catenin-like repeats// Armadillo/beta-catenin-like repeats// Armadillo/beta-catenin-like repeats// Armadillo/beta-catenin-like repeats
TESTI4044084// Domain of unknown function
TESTI4046487// Hantavirus nucleocapsid protein
TESTI4046819// Metallothionein// PTS HPr component phosphorylation sites
THYMU1000496// Kinesin motor domain
THYMU2004693// CX module
THYMU2005303// Immunoglobulin domain
THYMU2006420// NAD(P) transhydrogenase beta subunit
THYMU2008725// Similarity to lectin domain of ricin beta-chain, 3 copies.// Fibronectin type III domain// Fibronectin type III domain// Fibronectin type III domain// Fibronectin type III domain
THYMU2009425// 7 transmembrane receptor (rhodopsin family)
THYMU2011548// 7 transmembrane receptor (rhodopsin family)
THYMU2011736// EGF-like domain// EGF-like domain// EB module// EGF-like domain// TB domain// EGF-like domain// EGF-like domain
THYMU2016204// Metallothionein
THYMU2019210// Class I Histocompatibility antigen, domains alpha 1 and 2// Class I Histocompatibility antigen, domains alpha 1 and 2// Immunoglobulin domain
THYMU2023711// Immunoglobulin domain// Immunoglobulin domain// Immunoglobulin domain// Immunoglobulin domain
THYMU2027695// Immunoglobulin domain// Immunoglobulin domain// Immunoglobulin domain// Immunoglobulin domain
THYMU2027734// Parvovirus coat protein VP2
THYMU2032014// SH3 domain
THYMU2033079// ABC transporter
THYMU2035319// RNA recognition motif. (a.k.a. RRM, RBD, or RNP domain)// RNA recognition motif. (a.k.a. RRM, RBD, or RNP domain)// RNA recognition motif. (a.k.a. RRM, RBD, or RNP domain)
THYMU2035735// FHA domain// SNAP-25 family// Borrelia ORF-A
THYMU2036459// GTP1/OBG family
THYMU2037226// SH3 domain// TPR Domain// TPR Domain// TPR Domain// TPR Domain
THYMU2038369// Regulatory subunit of type II PKA R-subunit
THYMU2038615// PH domain
THYMU2038797// Lectin C-type domain
THYMU2041015// Sodium:galactoside symporter family// LacY proton/sugar symporter// Domain of unknown function// Monocarboxylate transporter// Polysaccharide biosynthesis protein// Sugar (and other) transporter
THYMU3000028// Zona pellucida-like domain
THYMU3000133// Viral (Superfamily 1) RNA helicase
THYMU3001234// PH domain
THYMU3001379// 3'5'-cyclic nucleotide phosphodiesterase// Elongation factor Tu family
THYMU3003212// Cytidine and deoxycytidylate deaminase zinc-binding region
THYMU3003763// Leucine rich repeat N-terminal domain// Polyomavirus coat protein
THYMU3004835// Galactosyltransferase
THYMU3006172// C2 domain// C2 domain
THYMU3007137// PDZ domain (Also known as DHR or GLGF).// PDZ domain (Also known as DHR or GLGF).
THYMU3008171// TPR Domain
THYMU3008436// Phosphofructokinase// Phosphofructokinase
TLIVE2000023// Integral membrane protein
TLIVE2002336// Metalloenzyme superfamily// Sulfatase// Type I phosphodiesterase / nucleotide pyrophosphatase
TLIVE2002338// Transforming growth factor beta like domain
TLIVE2002690// von Willebrand factor type D domain
TLIVE2003225// CUB domain// Sushi domain (SCR repeat)// CUB domain// Sushi domain (SCR repeat)
TLIVE2003381// 7 transmembrane receptor (metabotropic glutamate family)
TLIVE2007132// Syndecan domain
TLIVE2008229// TPR Domain// TPR Domain
TLIVE2009541// TBC domain
TRACH2001443// TIR domain
TRACH2001549// Cyclic nucleotide-binding domain
TRACH2005811// Kinesin motor domain
TRACH2006387// NADH-ubiquinone oxidoreductase chain 4,// 7 transmembrane receptor (rhodopsin family)
TRACH2007059// DnaJ domain// Integrins, beta chain// PA domain
TRACH2009310// Armadillo/beta-catenin-like repeats// Eukaryotic protein kinase domain// RIO1/ZK632.3/MJ0444 family
TRACH2019473// Iron/manganese superoxide dismutases (SODM)
TRACH2021398// RhoGAP domain
TRACH2022425// Immunoglobulin domain// Subtilase family// Immunoglobulin domain// Immunoglobulin domain// Immunoglobulin domain// Immunoglobulin domain
TRACH2022553// Immunoglobulin domain// Immunoglobulin domain// Immunoglobulin domain// Immunoglobulin domain
TRACH2022649// Immunoglobulin domain// Immunoglobulin domain// Immunoglobulin domain// Immunoglobulin domain
TRACH2023299// Squalene and phytoene synthases// PH domain// tRNA synthetases class I (E and Q)
TRACH2025535// PH domain
TRACH2025749// Zinc finger, C3HC4 type (RING finger)
TRACH3001427// UBX domain
TRACH3002168// Immunoglobulin domain// Immunoglobulin domain// Immunoglobulin domain
TRACH3003379// Protein phosphatase 2A regulatory B subunit
TRACH3004786// PMP-22/EMP/MP20/Claudin family
TRACH3004840// Rop protein
TRACH3005479// Glycosyl transferases group 1
TRACH3005549// Immunoglobulin domain// Immunoglobulin domain
TRACH3006470// Glycosyl transferases group 1
TRACH3007479// WW domain// HECT-domain (ubiquitin-transferase).
TRACH3008093// Putative undecaprenyl diphosphate synthase
TRACH3008629// Cadherin domain// Cadherin domain// Cadherin domain// Cadherin domain// Cadherin domain// PQQ enzyme repeat
TRACH3008713// NSF attachment protein
TRACH3009455// Src homology domain 2// FERM domain (Band 4.1 family)// Src homology domain 2
TRACH3034731// Ras association (RalGDS/AF-6) domain
TRACH3035235// S-100/ICaBP type calcium binding domain
TRACH3035526// Immunoglobulin domain// Cellulose binding domain// Immunoglobulin domain// Immunoglobulin domain// Immunoglobulin domain// Immunoglobulin domain
TRACH3036193// picornavirus capsid protein// Thaumatin family// Picornavirus core protein 2A// Picornavirus 2B protein// Extracellular link domain// RNA helicase// 3C cysteine protease (picornain 3C)// RNA dependent RNA polymerase
TRACH3036609// Immunoglobulin domain
TSTOM2000442// Immunoglobulin domain// Immunoglobulin domain// Immunoglobulin domain// Immunoglobulin domain
TSTOM2000553// C2 domain
TUTER2000425// KRAB box
UTERU1000024// NOL1/NOP2/sun family// NOL1/NOP2/sun family
UTERU1000031// ENTH domain// VHS domain
UTERU1000337// Protein phosphatase 2C
UTERU2005621// Protein-tyrosine phosphatase// Dual specificity phosphatase, catalytic domain
UTERU2006115// Adaptin N terminal region
UTERU2006568// IBR domain
UTERU2007724// Calponin homology (CH) domain// Calponin family// Calponin family// Calponin family
UTERU2017762// Ubiquitin family
UTERU2019706// TCP-1/cpn60 chaperonin family// TCP-1/cpn60 chaperonin family
UTERU2019940// Ribosomal protein L30p/L7e
UTERU2025025// Eukaryotic protein kinase domain
UTERU2026025// RNA recognition motif. (a.k.a. RRM, RBD, or RNP domain)
UTERU2026090// Lectin (probable mannose binding)
UTERU2033375// Ubiquitin carboxyl-terminal hydrolase family 2
UTERU2035328// WW domain// WW domain// WW domain// FF domain// FF domain// FF domain
UTERU2035331// Fibrillar collagen C-terminal domain
UTERU2035452// EGF-like domain// Metallothionein// EGF-like domain
UTERU2035745// Myosin head (motor domain)// Aldehyde oxidase and xanthine dehydrogenase, C terminus
UTERU2036089// RhoGAP domain
UTERU2038251// PH domain
UTERU3000645// PMP-22/EMP/MP20/Claudin family
UTERU3000828// 3'5'-cyclic nucleotide phosphodiesterase// Elongation factor Tu family// Elongation factor G C-terminus
UTERU3001240// Copper/zinc superoxide dismutase (SODC)// Adenylate and Guanylate cyclase catalytic domain
UTERU3001585// Cytochrome P450
UTERU3001652// Wiskott Aldrich syndrome homology region 2
UTERU3001766// Apidaecin
UTERU3001988// TPR Domain
UTERU3002667// Polyomavirus coat protein
UTERU3002993// NOL1/NOP2/sun family
UTERU3003116// Urease// EGF-like domain
UTERU3003178// TPR Domain// TPR Domain// TPR Domain// TPR Domain// PPR repeat
UTERU3003523// PH domain// Fibroblast growth factor
UTERU3004616// Disintegrin
UTERU3004992// Immunoglobulin domain
UTERU3005460// Penicillin amidase// Bacterial regulatory proteins, lacI family
UTERU3005585// PDZ domain (Also known as DHR or GLGF).
UTERU3005907// Transglutaminase family
UTERU3006308// Integrins, beta chain// Plexin repeat// Immunoglobulin domain
UTERU3007419// PH domain
UTERU3007640// NSF attachment protein
UTERU3008660// TPR Domain// TPR Domain
UTERU3009490// Bromodomain
UTERU3009871// Ank repeat// Ank repeat// Ank repeat// Ank repeat// TPR Domain// Ank repeat// Ank repeat
UTERU3009979// EGF-like domain// EGF-like domain// EGF-like domain// Trypsin Inhibitor like cysteine rich domain// EGF-like domain// Laminin G domain// Thrombospondin N-terminal -like domains// Laminin G domain
UTERU3015500// Leucine rich repeat N-terminal domain// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat

Deduced amino acid sequences of following 250 clones were also detected to have functional domains with Pfam.
3NB692004724// KRAB box// Integrase core domain
ADRGL2000042// Nucleosome assembly protein (NAP)
BLADE2000579// Src homology domain 2// Peptidase family C9
BLADE2006830// HSF-type DNA-binding domain
BRACE2003609// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type// Zinc finger, C2H2 type
BRACE2029396// Somatotropin hormone family
BRACE2037299// Integrase core domain
BRACE2039823// CDP-alcohol phosphatidyltransferase
BRACE3001058// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Putative zinc finger in N-recognin// Zinc finger, C2H2 type// Zinc finger, C2H2 type// Transcription factor S-II (TFIIS)// Src homology domain 2// Zinc finger, C2H2 type// Zinc finger, C2H2 type// Src homology domain 2// Zinc finger, C2H2 type
BRACE3001113// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Putative zinc finger in N-recognin// Zinc finger, C2H2 type// PHD-finger// Zinc finger, C2H2 type
BRACE3003026// Phorbol esters/diacylglycerol binding domain (C1 domain)// Zinc finger, C3HC4 type (RING finger)// PHD-finger
BRACE3003053// Influenza RNA-dependent RNA polymerase subunit// Reprolysin family propeptide// Leptin
BRACE3005107// Small cytokines (intecrine/chemokine), interleukin-8 like
BRACE3009127// PH domain// Oxysterol-binding protein
BRACE3010076// KH domain// KH domain// Domain of unknown function// KH domain// KH domain// KH domain// Small cytokines (intecrine/chemokine), interleukin-8 like// Fanconi anaemia group C protein// KH domain// KH domain
BRALZ2017844// Homeobox domain
BRAMY2019111// Ion transport protein
BRAMY2035070// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
BRAMY2035449// KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
BRAMY2035718// HMG (high mobility group) box// CTF/NF-I family
BRAMY2038516// Thioredoxin// Thioredoxin
BRAMY2039341// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type
BRAMY2040159// Chalcone and stilbene synthases// Adaptor complexes medium subunit family// PH domain// Putative GTP-ase activating protein for Arf// Ank repeat// Ank repeat
BRAMY2045471// DHHC zinc finger domain
BRAMY3004800// Synaptobrevin// RhoGEF domain
BRAWH1000369// DNA polymerase family A
BRAWH2006207// KRAB box
BRAWH2006395// Immunoglobulin domain// Thrombospondin type 1 domain
BRAWH2010552// Cyclin
BRAWH3007441// Zinc finger C-x8-C-x5-C-x3-H type (and similar)
BRAWH3009017// WD domain, G-beta repeat// WD domain, G-beta repeat
BRCAN2002473// Tropomyosins// Tropomyosins// UvrB/uvrC motif// Tropomyosins
BRCAN2002854// SAP domain
BRCAN2003070// Ubiquitin-conjugating enzyme
BRCAN2014229// SRP54-type protein// SRP54-type protein// Shikimate kinase// Adenylate kinase// ATPases associated with various cellular activities (AAA)
BRCOC2019841// Purple acid phosphatase
BRHIP2005724// alpha/beta hydrolase fold
BRHIP2006617// TPR Domain// TPR Domain
BRHIP2008389// Adenylate and Guanylate cyclase catalytic domain
BRHIP2012360// XPG N-terminal domain// XPG I-region
BRHIP2017553// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
BRHIP2026877// Eukaryotic protein kinase domain
BRHIP3000017// Integrins, beta chain// Uncharacterized protein family UPF0004
BRHIP3000240// Aldo/keto reductase family// Aldo/keto reductase family
BRHIP3008314// Sir2 family
BRHIP3026052// Protein phosphatase 2A regulatory B subunit (B56 family)
BRSTN2013354// Ets-domain
BRTHA2002133// Reverse transcriptase (RNA-dependent DNA polymerase)
BRTHA2002702// RNase H
BRTHA2007060// Transposase
BRTHA2010033// AP endonuclease family 1
BRTHA2013426// AP endonuclease family 1
BRTHA2013610// Deoxynucleoside kinase
BRTHA2016318// KE2 family protein
BRTHA2017364// DEAD/DEAH box helicase// Helicases conserved C-terminal domain
BRTHA2017972// Dwarfin
BRTHA2018011// Trypsin
BRTHA3000296// Peptidase family M20/M25/M40
CERVX2002013// Zinc finger, C2H2 type// Zinc finger, C2H2 type CTONG1000113// KRAB box// Zinc finger, C2H2 type// Zinc finger, C2H2 type// Integrase core domain// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
CTONG2003348// bZIP transcription factor//Importin beta binding domain
CTONG2004000// Acyl-CoA dehydrogenase
CTONG2015596// Myb-like DNA-binding domain
CTONG2016942// Trypsin
CTONG2019822// Hepatitis C virus core protein
CTONG2020374// MORN motif// MORN motif// MORN motif// MORN motif// MORN motif// MORN motif// MORN motif// Penicillin amidase// Bacterial regulatory proteins, lacI family// Vacuolar sorting protein 9 (VPS9) domain
CTONG2020378// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
CTONG2020411// Zinc finger, C2H2 type// Zinc finger, C2H2 type// Zinc finger, C2H2 type// Zinc finger, C2H2 type// Zinc finger, C2H2 type// Zinc finger, C2H2 type
CTONG2024031// MORN motif// MORN motif// MORN motif// MORN motif// MORN motif// MORN motif// MORN motif// Penicillin amidase// Bacterial regulatory proteins, lacI family
CTONG2028758// Zinc finger, C2H2 type// Zinc finger, C2H2 type// Homeobox domain// Zinc finger, C2H2 type// Homeobox domain// Zinc finger, C2H2 type
CTONG3001501// Zinc finger, C2H2 type
CTONG3002552// AP endonuclease family 1
CTONG3003598// AP endonuclease family 1
CTONG3004550// Phosphoribosyl-ATP pyrophosphohydrolase// Death domain
CTONG3004726// KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type// Zinc finger, C2H2 type
DFNES2011192// KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
FCBBF1000509// SAM domain (Sterile alpha motif)// SURF4 family// SAM domain (Sterile alpha motif)// DNA photolyase
FCBBF3010361// Fork head domain
FCBBF3027854// Zinc finger, C2H2 type
FEBRA2001990// RasGEF domain
FEBRA2006519// Thrombospondin type 1 domain// Thrombospondin type 1 domain
FEBRA2008692// PQQ enzyme repeat// wnt family of developmental signaling proteins// Eukaryotic protein kinase domain// Ribulose bisphosphate carboxylase, large chain
FEBRA2014122// Zinc finger, C2H2 type// Zinc finger, C2H2 type// Zinc finger, C2H2 type// Sodium// Zinc finger, C2H2 type
FEBRA2027609// Zinc finger, C2H2 type// Rubredoxin// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Putative zinc finger in N-recognin// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
FEBRA2028256// EGF-like domain// EGF-like domain// EGF-like domain// EGF-like domain// EGF-like domain// TB domain// EGF-like domain// EGF-like domain// EGF-like domain// EGF-like domain// EB module// Squash family of serine protease inhibitors// EGF-like domain// EGF-like domain
FEBRA2028516// GRIP domain
HCASM2002754// Sterol desaturase
HCASM2003018// PHD-finger// Zinc finger// Zinc finger// Zinc finger// Zinc finger// Zinc finger// Zinc finger// Zinc finger// Zinc finger// Zinc finger// Zinc finger// Zinc finger// Zinc finger
HCASM2003099// Histone deacetylase family// Zn-finger in ubiquitin-hydrolases and other proteins
HCASM2003357// Coproporphyrinogen III oxidase
HCASM2008536// XRCC1 N terminal domain
HCASM2009424// RFX DNA-binding domain
HCHON2000508// PH domain// bZIP transcription factor// bZIP transcription factor// Outer membrane efflux protein// Troponin
HCHON2000743// SCAN domain
HCHON2004858// SCAN domain// Myb-like DNA-binding domain// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type
HEART2009680// Bacteriorhodopsin// 7 transmembrane receptor (Secretin family)
HLUNG2015418// Cadherin domain// Cadherin domain// Cadherin domain// Cadherin domain// PQQ enzyme repeat
HLUNG2015548// IMP dehydrogenase / GMP reductase N terminus// CBS domain// CBS domain// Dihydroorotate dehydrogenase// Histidine biosynthesis protein// FMN-dependent dehydrogenase// Conserved region in glutamate synthase// IMP dehydrogenase / GMP reductase C terminus
HSYRA2005628// KRAB box// Zinc finger, C2H2 type// PHD-finger// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
IMR322007078// UBA domain
IMR322008651// Helix-hairpin-helix motif.
IMR322013396// Transmembrane region cyclic Nucleotide Gated Channel// Cyclic nucleotide-binding domain
IMR322013731// ATPases associated with various cellular activities (AAA)// Bromodomain
LIVER2000247// Sodium
MESAN2001770// Regulatory subunit of type II PKA R-subunit// eIF4-gamma/eIF5/eIF2-epsilon
MESAN2005303// Ank repeat//HECT-domain (ubiquitin-transferase).
MESAN2014412// Zinc finger, C2H2 type// Zinc finger, C2H2 type// Zinc finger, C2H2 type// Zinc finger, C2H2 type// Zinc finger, C2H2 type// Zinc finger, C2H2 type
MESAN2015501// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
NT2RI2005772// Guanylate kinase//I/LWEQ domain//PDZ domain (Also known as DHR or GLGF).//SH3 domain
NT2RI2008952// FYVE zinc finger//PHD-finger//Plant PEC family metallothionein//RNA polymerases M/15 Kd subunits//TRAF-type zinc finger//Transcription factor S-II (TFIIS)//Zinc finger, C2H2 type
NT2RI2009583// 7 transmembrane receptor (metabotropic glutamate family)//GPR1/FUN34/yaaH family
NT2RI2018448// Zinc finger, C2H2 type// Zinc finger, C2H2 type// Zinc finger, C2H2 type
NT2RI2027157// Reeler domain// HSF-type DNA-binding domain
NT2RI3000174// SNF2 and others N-terminal domain// Small cytokines (intecrine/chemokine), interleukin-8 like// Helicases conserved C-terminal domain// Zn-finger in Ran binding protein and others.// HNH endonuclease
NT2RI3001132// Zinc finger, C3HC4 type (RING finger)// PHD-finger
NT2RI3002557// Ribosomal protein L36// bZIP transcription factor
NT2RI3007167// Cold-shock' DNA-binding domain// Zinc knuckle// Zinc knuckle
NT2RI3007443// Alpha-2-macroglobulin family// Eukaryotic protein kinase domain// REV protein (anti-repression trans-activator protein)
NT2RP7008435// Anenome neurotoxin//CUB domain//Low-density lipoprotein receptor domain class A//Trypsin
NT2RP8000521// Small cytokines (intecrine/chemokine), interleukin-8 like
NTONG2008093// Adenylylsulfate kinase// 6-phosphofructo-2-kinase
OCBBF2003327// Thrombospondin type 1 domain// Thrombospondin type 1 domain// Thrombospondin type 1 domain
OCBBF2005433// SH3 domain// WW domain// PH domain// RhoGAP domain
OCBBF2006987// Collagen triple helix repeat (20 copies)// Eukaryotic DNA topoisomerase I
OCBBF2008144// KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
OCBBF2009583// KRAB box// Zinc finger, C2H2 type// Zinc finger, C2H2 type// Zinc finger, C2H2 type// Zinc finger, C2H2 type// Zinc finger, C2H2 type
OCBBF2011669// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Putative zinc finger in N-recognin// Zinc finger, C2H2 type// PHD-finger// Zinc finger, C2H2 type
OCBBF2019684// Zinc finger, C2H2 type// Zinc finger, C2H2 type// Transcription factor S-II (TFIIS)// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
OCBBF2020048// Protein of unknown function DUF98// Zinc finger, C3HC4 type (RING finger)
OCBBF2024284// Hemagglutinin// PHD-finger// Zinc finger// Zinc finger// Zinc finger// Zinc finger// Zinc finger// Zinc finger// Zinc finger// Zinc finger// Zinc finger// Zinc finger// Zinc finger// Zinc finger// WD domain, G-beta repeat// WD domain, G-beta repeat// WD domain, G-beta repeat
OCBBF2030116// Hr1 repeat motif// Transthyretin precursor (formerly prealbumin)// Tau and MAP proteins, tubulin-binding// Transient receptor// Syntaxin
OCBBF2032274// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type
OCBBF2034637// Prokaryotic DNA topoisomerase// Protein of unknown function// Eukaryotic protein kinase domain
OCBBF3000167// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
OCBBF3002654// SH3 domain// Immunoglobulin domain// Eukaryotic protein kinase domain
OCBBF3003761// KH domain// KH domain// Zinc finger, C3HC4 type (RING finger)
PERIC2007068// ELM2 domain// Myb-like DNA-binding domain// Eukaryotic initiation factor 1A// Myb-like DNA-binding domain
PLACE7000333// AP endonuclease family 1
PLACE7000502// Ank repeat// Ank repeat// Ank repeat// Ank repeat// Ank repeat// Peptidase family C9
PROST2000452// Trypsin
PROST2009320// LIM domain containing proteins// LIM domain containing proteins
PUAEN2006335// Formin Homology 2 Domain
SKMUS2003194// SAP domain
SPLEN2016135// Zinc finger, C2H2 type// Zinc finger, C2H2 type// Phorbol esters/diacylglycerol binding domain (C1 domain)// Zinc finger, C2H2 type
SPLEN2016781// Transcription factor S-II (TFIIS)// Zinc finger, C2H2 type// Zinc finger, C2H2 type// Zinc finger, C2H2 type
SPLEN2030847// Kinesin motor domain// Kinesin motor domain// GGL domain
SPLEN2036702// REJ domain// Phorbol esters/diacylglycerol binding domain (C1 domain)// PHD-finger
SPLEN2039311// dUTPase
SPLEN2039379// Transthyretin precursor (formerly prealbumin)
STOMA2003158// Deoxyribonuclease I (DNase I)
SYNOV1000256// Leucine Rich Repeat// BAH domain// Leucine Rich Repeat// Leucine Rich Repeat// Leucine Rich Repeat
SYNOV2006620// Nuclear transition protein 2
SYNOV2013637// Chalcone and stilbene synthases
SYNOV2021953// Zinc finger, C2H2 type// Zinc finger, C2H2 type// Zinc finger, C2H2 type// Zinc finger, C2H2 type
SYNOV4002744// Zinc finger, C2H2 type// Zinc finger, C2H2 type// Zinc finger, C2H2 type// Zinc finger, C2H2 type
SYNOV4003981// Somatomedin B domain// WAP-type (Whey Acidic Protein) 'four-disulfide core'// Hemopexin// Hemopexin
SYNOV4005889// Apolipoprotein A1/A4/E family
TESOP2000390// Eukaryotic protein kinase domain
TESOP2001796// Zinc finger, C3HC4 type (RING finger)// PHD-finger// IBR domain
TESOP2005199// KRAB box// Zinc finger, C2H2 type// Zinc finger, C2H2 type// Zinc finger, C2H2 type// Transcription factor S-II (TFIIS)// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// TRAF-type zinc finger// Zinc finger, C2H2 type
TESOP2006398// Zinc finger, C2H2 type
TESOP2006865// KRAB box
TESTI1000266// Integrase core domain
TESTI2008901// Transcription factor WhiB
TESTI2015626// Phosphoribosyl transferase domain
TESTI2025924// Eukaryotic protein kinase domain
TESTI2026647// DEAD/DEAH box helicase// Helicases conserved C-terminal domain
TESTI2029252// Ion transport protein// Polysaccharide biosynthesis protein
TESTI2034251// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
TESTI2035981// RNA polymerase alpha subunit
TESTI2036288// Aldo/keto reductase family
TESTI2037830// Zinc finger, C2H2 type// Zinc finger, C2H2 type
TESTI2039060// D-ala D-ala ligase// Glycosyl hydrolases family 31
TESTI2049956// WD domain, G-beta repeat// WD domain, G-beta repeat// PQQ enzyme repeat// WD domain, G-beta repeat// WD domain, G-beta repeat// WD domain, G-beta repeat// WD domain, G-beta repeat
TESTI2050780// Kazal-type serine protease inhibitor domain
TESTI4000137// Domain of unknown function
TESTI4000155// Viral RNA dependent RNA polymerase
TESTI4000183// KRAB box// Zinc finger, C2H2 type// Zinc finger, C2H2 type// Zinc finger, C2H2 type
TESTI4000214// Zinc finger, C3HC4 type (RING finger)// DENN (AEX-3) domain
TESTI4000319// RasGEF domain
TESTI4001984// Retroviral aspartyl protease// G-patch domain
TESTI4005317// Bacterial flagellin C-terminus// Phosphotyrosine interaction domain (PTB/PID)
TESTI4006473// Sigma-54 transcription factors// DEAD/DEAH box helicase// DEAD/DEAH box helicase// Ank repeat// Ank repeat// Helicases conserved C-terminal domain
TESTI4008058// Zn-finger in Ran binding protein and others.// Zinc finger, CCHC class
TESTI4008302// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// Zinc finger, C2H2 type
TESTI4010382// Luteovirus (ORF3) RNA-directed RNA-polymerase// Ezrin/radixin/moesin family
TESTI4011072// Tudor domain// Tudor domain// Staphylococcal nuclease homologues// Tudor domain// Tudor domain
TESTI4013365// MYND finger
TESTI4013894// Synaptophysin / synaptoporin
TESTI4014801// Zinc finger, C2H2 type// N2,N2-dimethylguanosine tRNA methyltransferase
TESTI4015442// Homeobox domain// Zinc finger, C2H2 type// Homeobox domain// Zinc finger, C2H2 type// Zinc finger, C2H2 type// Zinc finger, C2H2 type
TESTI4017714// Transcriptional regulatory protein, C terminal// Prolyl oligopeptidase family
TESTI4021482// Eukaryotic protein kinase domain
TESTI4024387// GDP dissociation inhibitor
TESTI4025268// WD domain, G-beta repeat// WD domain, G-beta repeat
TESTI4025494// KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// TRAF-type zinc finger// Zinc finger, C2H2 type// Zinc finger, C2H2 type// Zinc finger, C2H2 type// Zinc finger, C2H2 type// Zinc finger, C2H2 type
TESTI4025547// Type II intron maturase// Zinc finger, C2H2 type// Zinc finger, C2H2 type// Zinc finger, C2H2 type
TESTI4025865// Lipoate-protein ligase B// KE2 family protein
TESTI4026207// Kinesin motor domain// DNA gyrase/topoisomerase IV, subunit A
TESTI4028938// KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// DM DNA binding domain// Zinc finger, C2H2 type// Zinc finger, C2H2 type
TESTI4028958// DNA gyrase/topoisomerase IV, subunit A// Apolipoprotein A1/A4/E family
TESTI4029348// Trans-activation protein X
TESTI4029528// RanBP1 domain.
TESTI4029690// Leucine Rich Repeat// Leucine Rich Repeat// Leucine Rich Repeat// Cytochrome c oxidase subunit VIa// IQ calmodulin-binding motif
TESTI4031745// Alpha-2-macroglobulin family// Eukaryotic protein kinase domain// REV protein (anti-repression trans-activator protein)
TESTI4032090// RNase H// Integrase Zinc binding domain// Integrase core domain
TESTI4032112// Syndecan domain
TESTI4038721// Squash family of serine protease inhibitors
TESTI4041086// Transmembrane amino acid transporter protein
TESTI4046240// Sir2 family
THYMU2004139// Eukaryotic protein kinase domain
THYMU2004284// Repeat in ubiquitin-activating (UBA) proteins
THYMU2006001// Zinc finger, C3HC4 type (RING finger)// CONSTANS family zinc finger// B-box zinc finger.// SPRY domain
THYMU2028739// SCAN domain// KRAB box// Myb-like DNA-binding domain
THYMU2031139// Reprolysin (M12B) family zinc metalloprotease// Thrombospondin type 1 domain// EB module// Plant PEC family metallothionein// A20-like zinc finger
THYMU2031249// C-type lysozyme/alpha-lactalbumin family// Eukaryotic protein kinase domain
THYMU2035078// Domain of unknown function DUF27
THYMU2035710// ATP1G1/PLM/MAT8 family
THYMU2040925// CDP-alcohol phosphatidyltransferase
THYMU3000269// FAD binding domain
THYMU3000360// Integrase core domain
THYMU3001428// Zinc finger, C3HC4 type (RING finger)// PHD-finger
TKIDN2012771// DNA polymerase (viral) C-terminal domain// MttB family UPF0032
TLIVE2001684// Alpha-2-macroglobulin family// Alpha-2-macroglobulin family
TLIVE2002046// HMG (high mobility group) box// Uroporphyrinogen decarboxylase (URO-D)// Delta-aminolevulinic acid dehydratase
TLIVE2007607// DNA polymerase (viral) C-terminal domain// Cytochrome P450
TRACH1000212// TSC-22/dip/bun family
TRACH2000862// Guanylate-binding protein
TRACH2007483// Zinc finger, C2H2 type// Zinc finger, C2H2 type// Zinc finger, C2H2 type// Zinc finger, C2H2 type// Zinc finger, C2H2 type
TRACH2019672// CRAL/TRIO domain.
TRACH2024408// Death domain
TRACH2024559// IQ calmodulin-binding motif// IQ calmodulin-binding motif
TRACH3000134// KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// DnaJ central domain (4 repeats)// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
TRACH3000420// ABC transporter// Papain family cysteine protease// ABC transporter
TRACH3002561// 'Cold-shock' DNA-binding domain
TRACH3003832// PHD-finger
TRACH3007866// Transcriptional regulatory protein, C terminal// PAC motif// Dipeptidyl peptidase IV (DPP IV) N-terminal region// Prolyl oligopeptidase family
UTERU2004299// ATP P2X receptor
UTERU2008040// Phorbol esters/diacylglycerol binding domain (C1 domain)// SH3 domain
UTERU2019534// Cysteine rich repeat// Cysteine rich repeat// Carboxylesterases
UTERU2028734// C2 domain// C2 domain
UTERU2032279// Serpins (serine protease inhibitors)
UTERU2033577// KRAB box
UTERU3000402// WD domain, G-beta repeat// WD domain, G-beta repeat
UTERU3000738// Eukaryotic protein kinase domain
UTERU3001053// Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type
UTERU3014791// LIM domain containing proteins// PHD-finger// LIM domain containing proteins
UTERU3015412// WD domain, G-beta repeat// WD domain, G-beta repeat// WD domain, G-beta repeat// Keratin, high sulfur B2 protein
UTERU3017176// K-box region// bZIP transcription factor
TESTI4038779// K+ channel tetramerisation domain// BTB/POZ domain// Zinc finger, C2H2 type// Zinc finger, C2H2 type// Rubredoxin// PHD-finger// Zinc finger, C2H2 type

### EXAMPLE 6

### Functional categorization based on homology search of the full-length nucleotide sequences and deduced amino acid sequences

The functional prediction and categorization of the proteins encoded by the clones were carried out based on the result of homology search of the databases of GenBank, Swiss-Prot, UniGene, nr, and RefSeq (see the Homology Search Result Data) for the full-length nucleotide sequences and deduced amino acid sequences.

A clone predicted to belong to the category of secretory protein/membrane protein means a clone having hit data with some annotation, such as growth factor, cytokine, hormone, signal, transmembrane, membrane, extracellular matrix, receptor, G-protein coupled receptor, ionic channel, voltage-gated channel, calcium channel, cell adhesion, collagen, and connective tissue, suggesting that it is a secretory or membrane protein, or means a clone in which the presence of nucleotide sequence encoding a signal sequence or transmembrane domain was suggested by the results of PSORT and SOSUI analyses for deduced ORF.

A clone predicted to belong to the category of glycoprotein-related protein means a clone having hit data with some annotation, such as glycoprotein, suggesting that the clone encodes a glycoprotein-related protein.

A clone predicted to belong to the category of signal transduction-related protein means a clone having hit data with some annotation, such as serine/threonine-protein kinase, tyrosine-protein kinase, SH3 domain, and SH2 domain, suggesting that the clone encodes a signal transduction-related protein.

A clone predicted to belong to the category of transcription-related protein means a clone having hit data with some annotation, such as transcription regulation, zinc finger, and homeobox, suggesting that the clone encodes a transcription-related protein.

A clone predicted to belong to the category of disease-related protein means a clone having hit data with some annotation, such as disease mutation and syndrome, suggesting that the clone encodes a disease-related protein, or means a clone whose full-length nucleotide sequence has hit data for Swiss-Prot, GenBank, or UniGene, where the hit data corresponds to genes or proteins which have been deposited in the Online Mendelian Inheritance in Man (OMIM) (http://www.ncbi.nlm.nih.gov/Omim/), which is the human gene and disease database.

A clone predicted to belong to the category of enzyme and/or metabolism-related protein means a clone having hit data with some annotation, such as metabolism, oxidoreductase, and E. C. No. (Enzyme commission number), suggesting that the clone encodes an enzyme and/or metabolism-related protein.

A clone predicted to belong to the category of cell division and/or cell proliferation-related protein means a clone having hit data with some annotation, such as cell division, cell cycle, mitosis, chromosomal protein, cell growth, and apoptosis, suggesting that the clone encodes a cell division and/or cell proliferation-related protein.

A clone predicted to belong to the category of cytoskeleton-related protein means a clone having hit data with some annotation, such as structural protein, cytoskeleton, actin-binding, and microtubles, suggesting that the clone encodes a cytoskeleton-related protein.

A clone which is predicted to belong to the category of nuclear protein and/or RNA synthesis-related protein means a clone having hit data with some annotation, such as nuclear protein, RNA splicing, RNA processing, RNA helicase, and polyadenylation, suggesting that the clone encodes a nuclear protein and/or RNA synthesis-related protein.

A clone predicted to belong to the category of protein synthesis and/or transport-related protein means a clone having hit data with some annotation, such as translation regulation, protein biosynthesis, amino-acid biosynthesis, ribosomal protein, protein transport, and signal recognition particle, suggesting that the clone encodes a protein synthesis and/or transport-related protein.

A clone predicted to belong to the category of cellular defense-related protein means a clone having hit data with some annotation, such as heat shock, DNA repair, and DNA damage, suggesting that the clone encodes a cellular defense-related protein.

A clone predicted to belong to the category of development and/or differentiation-related proteins means a clone having hit data with some annotation, such as developmental protein, suggesting that the clone encodes a development and/or differentiation-related protein.

A clone predicted to belong to the category of DNA-binding and/or RNA-binding protein means a clone having hit data with some annotation, such as DNA-binding, RNA-binding, etc.

A clone predicted to belong to the category of ATP-binding and/or GTP-binding protein means a clone having hit data with some annotation, such as ATP-binding, GTP-binding, etc.

In this functional categorization, when a single clone corresponded to multiple categories of those shown above, the clone was assigned to the multiple categories. However, the function of a protein is not restricted to the functional categories in this classification, and there is the possibility that other functions are newly assigned to the protein.

The clones predicted to belong to the category of secretory protein and/or membrane protein are the following 659 clones.
ACTVT2000380, ADIPS2000088, ADRGL2000172, ADRGL2003329, ADRGL2009146, ASTRO2014923, ASTRO3000301, BLADE1000176, BLADE2002073, BLADE2002947, BLADE2004462, BLADE2004670, BLADE2005036, BLADE2008539, BNGH42003570, BRACE1000186, BRACE2005457, BRACE2014306, BRACE2016981, BRACE2029112, BRACE2030884, BRACE2031527, BRACE2031531, BRACE2031899, BRACE2032385, BRACE2036005, BRACE2039249, BRACE2039327, BRACE2040138, BRACE2041200, BRACE2043142, BRACE2043665, BRACE2046295, BRACE3000697, BRACE3001391, BRACE3002298, BRACE3003004, BRACE3003595, BRACE3004058, BRACE3004113, BRACE3004772, BRACE3004843, BRACE3006462, BRACE3008137, BRACE3008384, BRACE3009574, BRACE3009708, BRACE3010397, BRACE3011271, BRACE3011505, BRACE3013740, BRACE3014005, BRACE3014068, BRACE3014807, BRACE3016884, BRACE3018963, BRACE3019084, BRACE3020286, BRACE3020594, BRACE3024662, BRACE3025531, BRACE3025630, BRACE3026008, BRACE3026735, BRACE3027326, BRACE3031838, BRACE3040856, BRALZ2016085, BRAMY2001473, BRAMY2004771, BRAMY2005052, BRAMY2017528, BRAMY2019300, BRAMY2019963, BRAMY2021498, BRAMY2028856, BRAMY2033003, BRAMY2033116, BRAMY2033594, BRAMY2036396, BRAMY2039872, BRAMY2040592, BRAMY2041542, BRAMY2045036, BRAMY2047420, BRAMY2047751, BRAMY2047765, BRAMY3002312, BRAMY3004224, BRAMY3004919, BRAMY3007206, BRAMY3007609, BRAMY3008505, BRAMY4000095, BRASW1000125, BRAWH1000127, BRAWH2002560, BRAWH2002761, BRAWH2007658, BRAWH2014414, BRAWH2014954, BRAWH2016221, BRAWH2016439, BRAWH2016702, BRAWH3000078, BRAWH3000314, BRAWH3001475, BRAWH3001891, BRAWH3002600, BRAWH3003555, BRAWH3003727, BRAWH3003992, BRAWH3004453, BRAWH3004666, BRAWH3005132, BRAWH3005912, BRAWH3006548, BRAWH3007221, BRAWH3007506, BRAWH3007592, BRAWH3008634, BRCAN2002948, BRCAN2006063, BRCAN2009203, BRCAN2010376, BRCAN2012355, BRCAN2012481, BRCAN2013655, BRCAN2014143, BRCAN2016619, BRCAN2024451, BRCOC2007034, BRCOC2019934, BRHIP2000691, BRHIP2001805, BRHIP2002172, BRHIP2004814, BRHIP2004883, BRHIP2005236, BRHIP2005752, BRHIP2009414, BRHIP2013699, BRHIP2026288, BRHIP3000526, BRHIP3007483, BRHIP3007586, BRHIP3008598, BRHIP3009448, BRHIP3015751, BRHIP3024118, BRHIP3026097, BRSSN2003086, BRSSN2004496, BRSSN2008549, BRSSN2011738, BRSSN2014424, BRSSN2018925, BRSTN2000872, BRSTN2003835, BRSTN2007000, BRSTN2010363, BRSTN2012380, BRSTN2015015, BRSTN2016470, BRSTN2016678, BRSTN2017110, BRTHA2002376, BRTHA2002493, BRTHA2002608, BRTHA2002808, BRTHA2003110, BRTHA2003461, BRTHA2005579, BRTHA2006075, BRTHA2008527, BRTHA2011194, BRTHA2012980, BRTHA2013460, BRTHA2015696, BRTHA2015878, BRTHA2016215, BRTHA2016496, BRTHA2017985, BRTHA2018344, BRTHA2018624, BRTHA3000633, BRTHA3002427, BRTHA3003474, BRTHA3007148, BRTHA3008386, BRTHA3008778, BRTHA3009037, BRTHA3009090, BRTHA3009291, BRTHA3016845, BRTHA3017047, BRTHA3017589, BRTHA3017848, BRTHA3018656, CERVX2002006, COLON2000568, COLON2002443, COLON2004478, COLON2005126, COLON2005772, CTONG1000302, CTONG1000341, CTONG1000488, CTONG1000508, CTONG2000042, CTONG2004062, CTONG2008233, CTONG2009423, CTONG2009531, CTONG2010803, CTONG2013178, CTONG2019652, CTONG2019788, CTONG2020127, CTONG2020522, CTONG2020638, CTONG2022601, CTONG2023512, CTONG2024749, CTONG2025496, CTONG2026920, CTONG2027327, CTONG2028124, CTONG2028687, CTONG3000707, CTONG3001370, CTONG3001560, CTONG3002020, CTONG3003179, CTONG3003483, CTONG3003737, CTONG3005648, CTONG3008252, CTONG3008258, CTONG3008496, CTONG3008566, CTONG3008951, CTONG3009227, CTONG3009239, CTONG3009328, CTONG3009385, D3OST2002182, D3OST2002648, DFNES1000107, DFNES2000146, DFNES2005266, DFNES2010502, FCBBF2001183, FCBBF2007510, FCBBF3003435, FCBBF3004502, FCBBF3009888, FCBBF3012170, FCBBF3021576, FCBBF3023895, FCBBF4000076, FEBRA1000030, FEBRA2007708, FEBRA2008311, FEBRA2008468, FEBRA2020668, FEBRA2025427, FEBRA2027082, HCASM2002502, HCASM2003212, HCASM2007047, HCHON2000212, HCHON2001084, HCHON2001548, HCHON2001577, HCHON2001712, HCHON2002676, HCHON2004007, HCHON2004776, HCHON2005921, HEART1000010, HEART2001680, HEART2010492, HLUNG2000014, HLUNG2003872, HLUNG2010464, HLUNG2015617, HLUNG2017350, HSYRA2005496, HSYRA2006873, HSYRA2008714, HSYRA2009102, IMR322002110, IMR322006222, KIDNE1000064, KIDNE2000832, KIDNE2000846, KIDNE2006580, KIDNE2010264, KIDNE2011635, KIDNE2012945, KIDNE2013095, LIVER2007415, LYMPB2000083, MESAN2001979, MESAN2012054, MESTC1000042, NHNPC2000606, NHNPC2001223, NOVAR2000136, NOVAR2001108, NT2RI2008724, NT2RI2009855, NT2RI2025909, NT2RI3001263, NT2RI3003095, NT2RI3003382, NT2RI3003409, NT2RI3005403, NT2RI3006171, NT2RI3006673, NT2RI3007065, NT2RI3007543, NT2RI3007978, NT2RP7000359, NT2RP7000466, NT2RP7004027, NT2RP7009030, NT2RP7014005, NTONG2000413, OCBBF2006151, OCBBF2006567, OCBBF2006764, OCBBF2007114, OCBBF2007428, OCBBF2009926, OCBBF2010140, OCBBF2017516, OCBBF2021788, OCBBF2024719, OCBBF2025458, OCBBF2030517, OCBBF2030574, OCBBF2031167, OCBBF2032590, OCBBF2033869, OCBBF2037598, OCBBF2038317, OCBBF3000483, OCBBF3003320, OCBBF3004314, PEBLM2000170, PEBLM2000338, PEBLM2002594, PEBLM2006113, PEBLM2007834, PERIC2001227, PERIC2003452, PERIC2003720, PERIC2004909, PERIC2005347, PERIC2006035, PERIC2007914, PLACE5000171, PLACE5000260, PLACE5000282, PLACE6012574, PLACE6019932, PLACE6020031, PLACE7000514, PLACE7001022, PROST1000184, PROST1000528, PROST1000559, PROST2003428, PROST2018090, PROST2018902, PROST2018922, PUAEN2002489, PUAEN2005588, PUAEN2006701, PUAEN2009174, PUAEN2009795, RECTM2000433, RECTM2001347, SKMUS2000757, SKNMC2002402, SMINT2002743, SMINT2009902, SMINT2011888, SMINT2015787, SPLEN2001599, SPLEN2009548, SPLEN2012889, SPLEN2015158, SPLEN2015267, SPLEN2015679, SPLEN2021701, SPLEN2023733, SPLEN2023791, SPLEN2025491, SPLEN2029522, SPLEN2029683, SPLEN2030335, SPLEN2030479, SPLEN2031125, SPLEN2031424, SPLEN2031547, SPLEN2031724, SPLEN2031780, SPLEN2032813, SPLEN2033098, SPLEN2034021, SPLEN2034781, SPLEN2036326, SPLEN2036821, SPLEN2037722, SPLEN2038180, SPLEN2038345, SPLEN2038407, SPLEN2040222, SPLEN2041304, SPLEN2042598, STOMA2004294, STOMA2008546, SYNOV2005817, SYNOV2012326, SYNOV2014400, SYNOV2016124, SYNOV4002883, SYNOV4003322, SYNOV4004184, SYNOV4004741, SYNOV4004914, SYNOV4006256, SYNOV4007430, SYNOV4007553, SYNOV4007671, SYNOV4008336, SYNOV4008440, TBAES2001258, TCERX2000613, TESOP2001345, TESOP2001865, TESOP2002273, TESOP2002539, TESOP2004114, TESOP2005485, TESOP2005579, TESOP2006041, TESOP2007052, TESOP2007262, TESOP2007636, TESTI1000257, TESTI1000348, TESTI2000644, TESTI2002036, TESTI2002618, TESTI2002928, TESTI2003347, TESTI2005610, TESTI2006648, TESTI2013382, TESTI2024567, TESTI2027019, TESTI2034767, TESTI2034953, TESTI2034997, TESTI2035997, TESTI2036684, TESTI2042450, TESTI2047071, TESTI2048898, TESTI2051767, TESTI2052822, TESTI4000215, TESTI4000724, TESTI4001100, TESTI4001527, TESTI4001561, TESTI4001665, TESTI4001923, TESTI4002552, TESTI4002754, TESTI4005805, TESTI4005961, TESTI4006053, TESTI4006137, TESTI4007064, TESTI4007163, TESTI4007239, TESTI4007382, TESTI4008050, TESTI4008401, TESTI4008429, TESTI4008797, TESTI4009608, TESTI4012448, TESTI4013369, TESTI4013667, TESTI4013830, TESTI4014392, TESTI4016238, TESTI4017575, TESTI4017901, TESTI4018835, TESTI4019566, TESTI4020092, TESTI4020102, TESTI4021478, TESTI4023722, TESTI4024420, TESTI4024874, TESTI4024890, TESTI4025797, TESTI4026456, TESTI4026785, TESTI4027821, TESTI4028062, TESTI4028429, TESTI4028823, TESTI4028880, TESTI4029836, TESTI4030159, TESTI4030505, TESTI4034172, TESTI4035065, TESTI4035649, TESTI4037244, TESTI4041053, TESTI4042711, TESTI4046487, TESTI4046819, THYMU2001053, THYMU2003632, THYMU2003760, THYMU2005003, THYMU2005303, THYMU2005321, THYMU2007658, THYMU2008725, THYMU2009425, THYMU2011548, THYMU2013386, THYMU2014353, THYMU2019210, THYMU2023711, THYMU2027497, THYMU2027695, THYMU2029676, THYMU2030068, THYMU2032035, THYMU2032437, THYMU2032655, THYMU2033079, THYMU2033308, THYMU2033816, THYMU2034314, THYMU2035064, THYMU2036085, THYMU2036459, THYMU2037226, THYMU2037348, THYMU2038772, THYMU2038797, THYMU2039780, THYMU2040412, THYMU2041015, THYMU3000028, THYMU3000036, THYMU3004835, THYMU3005696, THYMU3006168, THYMU3006811, THYMU3007137, THYMU3007368, THYMU3007845, TKIDN2002424, TKIDN2002632, TKIDN2006525, TKIDN2009092, TKIDN2009889, TKIDN2014771, TKIDN2019116, TLIVE2000023, TLIVE2001828, TLIVE2001927, TLIVE2002336, TLIVE2002690, TLIVE2003381, TLIVE2004110, TLIVE2008229, TOVAR2001281, TRACH1000205, TRACH2001549, TRACH2001684, TRACH2006387, TRACH2007059, TRACH2007834, TRACH2008300, TRACH2020525, TRACH2021964, TRACH2022553, TRACH2025535, TRACH2025911, TRACH3000014, TRACH3002064, TRACH3002168, TRACH3002650, TRACH3004786, TRACH3005294, TRACH3005549, TRACH3006149, TRACH3007391, TRACH3008629, TRACH3035199, TRACH3035526, TRACH3036193, TSTOM2000442, TSTOM2000553, TUTER2000916, UTERU1000339, UTERU2004688, UTERU2004929, UTERU2006137, UTERU2006568, UTERU2007444, UTERU2017762, UTERU2020718, UTERU2022020, UTERU2025025, UTERU2025645, UTERU2025891, UTERU2026090, UTERU2026203, UTERU2027591, UTERU2029953, UTERU2031851, UTERU2035323, UTERU2035469, UTERU3000645, UTERU3000899, UTERU3001240, UTERU3001571, UTERU3001585, UTERU3001652, UTERU3001988, UTERU3002209, UTERU3002383, UTERU3002786, UTERU3003116, UTERU3003776, UTERU3006308, UTERU3008671, UTERU3009690, UTERU3009979, UTERU3011063, UTERU3015500, UTERU3016789

The following 82 clones are also predicted to belong to the category of secretory protein and/or membrane protein.
BLADE2006830, BRACE2002589, BRACE2009318, BRACE2011677, BRACE2029396, BRACE2039823, BRACE2039832, BRAMY2019111, BRAMY2038516, BRAMY2045471, BRAWH2006395, BRAWH2008993, BRCOC2019841, BRHIP2003272, BRHIP2005271, BRHIP2005724, BRHIP2008389, BRHIP2026877, BRHIP3000240, BRTHA2011321, BRTHA2018011, BRTHA2018443, BRTHA3008826, CTONG2015633, CTONG2016942, CTONG2019822, FEBRA2000790, FEBRA2006519, FEBRA2028256, FEBRA2028516, HCASM2002754, HEART2009680, HLUNG2013350, HLUNG2015418, IMR322013396, LIVER2000247, NT2RI2009583, NT2RI2027157, NT2RP7008435, OCBBF2003327, OCBBF2030116, PLACE7000502, PROST2000452, PROST2019487, SPLEN2016932, SPLEN2037319, SYNOV2001660, SYNOV2013637, SYNOV4003981, SYNOV4005889, TBAES2000932, TESTI2015626, TESTI2029252, TESTI2032643, TESTI2039060, TESTI2050780, TESTI4000137, TESTI4000155, TESTI4006473, TESTI4011070, TESTI4013365, TESTI4013894, TESTI4014801, TESTI4032090, TESTI4041086, THYMU2004284, THYMU2030462, THYMU2033401, THYMU2034279, THYMU2035710, THYMU2040925, TKIDN2008778, TKIDN2012771, TKIDN2018926, TLIVE2007607, TRACH2019672, TRACH3000420, TRACH3003683, UTERU2011220, UTERU2021820, UTERU2032279, UTERU3015069

The clones predicted to belong to the category of glycoprotein-related protein are the following 115 clones.
ADIPS2000088, BNGH42003570, BRACE2005457, BRACE2014306, BRACE2029112, BRACE2039249, BRACE2046295, BRACE3001391, BRACE3011271, BRACE3016884, BRAMY2005052, BRAMY3004919, BRAMY4000095, BRAMY4000277, BRAWH1000127, BRAWH2007658, BRAWH2014414, BRAWH2016221, BRAWH3002600, BRCAN2006063, BRSSN2004496, BRTHA2008527, BRTHA2012980, BRTHA2016496, BRTHA3002427, BRTHA3017848, COLON2000568, COLON2004478, COLON2005772, CTONG1000341, CTONG2000042, CTONG2009423, CTONG2023512, CTONG2024749, CTONG2025496, CTONG3001370, CTONG3003737, D3OST2002648, DFNES2000146, DFNES2005266, FCBBF3012170, FEBRA1000030, FEBRA2008311, FEBRA2008468, HCHON2001712, HEART1000010, HEART2001680, HSYRA2005496, KIDNE2012945, LYMPB2000083, NESOP2001433, NOVAR2000136, NOVAR2001108, NT2RI3006171, NT2RI3006673, NT2RP7004027, OCBBF2033869, PLACE5000171, PROST1000184, PUAEN2009795, SMINT2010076, SMINT2011888, SMINT2015787, SPLEN2015267, SPLEN2021701, SPLEN2030335, SYNOV2005817, SYNOV2014400, SYNOV3000231, SYNOV3000302, TESOP2004114, TESOP2005485, TESTI1000257, TESTI2002036, TESTI2002618, TESTI2024567, TESTI2027019, TESTI4001527, TESTI4007163, TESTI4012406, TESTI4013830, TESTI4020092, TESTI4023546, TESTI4028823, TESTI4028880, TESTI4046819, THYMU2005303, THYMU2008725, THYMU2009425, THYMU2011548, THYMU2019210, THYMU2023711, THYMU2027497, THYMU2027695, THYMU2038797, THYMU3004835, TLIVE2003381, TRACH2006387, TRACH2007059, TRACH2022425, TRACH2022553, TRACH2022649, TRACH3002168, TRACH3008629, TRACH3035526, TSTOM2000442, UTERU2008347, UTERU2025025, UTERU2035469, UTERU3000899, UTERU3001240, UTERU3003116, UTERU3006308, UTERU3008671, UTERU3015500

The following 15 clones are also predicted to belong to the category of glycoprotein-related protein.
BRAMY2019111, BRHIP2026877, BRTHA2018011, FEBRA2028256, HEART2009680, HLUNG2015418, NT2RI2009583, NT2RP7008435, OCBBF2003327, TESTI2032643, TESTI2039060, TESTI4011070, THYMU2035710, TRACH3003683, UTERU2032279

The clones predicted to belong to the category of signal transduction-related protein are the following 80 clones.
BNGH42007788, BRACE2008594, BRACE2030341, BRACE2044286, BRACE3002508, BRACE3003595, BRACE3006872, BRACE3011421, BRACE3015027, BRACE3027326, BRAMY2036567, BRAMY2038904, BRAMY3000213, BRAMY3002803, BRAMY3005091, BRAMY3005932, BRAMY4000095, BRAMY4000229, BRCAN2003703, BRCAN2014602, BRCAN2016619, BRCAN2028355, BRHIP2000819, BRHIP3025161, BRSSN2004719, BRSTN2008418, BRTHA2002281, BRTHA2015406, CTONG2006798, CTONG3000084, CTONG3002412, D3OST3000169, FCBBF3007540, HCASM2001301, HCHON2006250, HCHON2008112, HLUNG2002465, KIDNE2001847, NESOP2001694, NT2NE2003252, NT2RI2005166, NT2RI3007757, NT2RI3008652, NT2RP7005529, NT2RP7009147, NT2RP7013795, NT2RP8000483, OCBBF2004826, OCBBF2007028, OCBBF2022351, OCBBF2030354, OCBBF2037547, PLACE6019385, PLACE7008431, PROST2016462, PROST2018511, PUAEN2009852, SPLEN2036932, SYNOV2021320, TESOP2000801, TESOP2001166, TESTI2005739, TESTI2026505, TESTI2050137, TESTI4011745, TESTI4012505, TESTI4018208, TESTI4028059, THYMU2007060, THYMU2031046, THYMU2032014, THYMU2039305, THYMU3008436, TLIVE2001327, TRACH2009310, TRACH2025535, TRACH3009455, UTERU2025025, UTERU2036089, UTERU3016789

The following 31 clones are also predicted to belong to the category of signal transduction-related protein.
BRAMY3004800, BRAWH3009017, BRHIP2026877, BRTHA2013610, BRTHA2017972, BRTHA3003000, CTONG2020974, FEBRA2001990, FEBRA2008692, NT2RI2005772, NT2RI3007443, NTONG2008093, OCBBF2005433, OCBBF2024284, OCBBF2034637, OCBBF3002654, SPLEN2036702, SPLEN2039379, TESOP2000390, TESTI2025924, TESTI2049956, TESTI4000319, TESTI4019657, TESTI4021482, TESTI4024387, TESTI4025268, TESTI4031745, THYMU2004139, THYMU2031249, UTERU2008040, UTERU3000738

The clones predicted to belong to the category of transcription-related protein are the following 38 clones.
BRACE2030326, BRACE3001002, BRACE3045033, BRHIP3025161, BRSSN2014299, BRTHA2014792, BRTHA3001721, CTONG2025516, FEBRA2007544, FEBRA2007801, HEART1000074, IMR322000127, IMR322000917, NT2NE2006531, NT2RI2006686, NT2RI3009158, OCBBF2020838, OCBBF2036743, PEBLM2002887, SKNMC2007504, SPLEN2012624, TESTI2026505, TESTI2040018, TESTI2044796, TESTI2050987, TESTI4001176, TESTI4007810, TESTI4014175, TESTI4017543, TESTI4026524, TESTI4036909, THYMU2006420, THYMU2037233, THYMU3004866, TRACH3000558, TUTER2000425, UTERU2035328, UTERU3009490

The following 64 clones are also predicted to belong to the category of transcription-related protein.
BRACE2003609, BRACE3001058, BRACE3001113, BRALZ2017844, BRAMY2035070, BRAMY2035449, BRAMY2035718, BRAMY2039341, BRAWH2006207, BRHIP2017553, CERVX2002013, CTONG1000113, CTONG2003348, CTONG2008721, CTONG2020378, CTONG2020411, CTONG2028758, CTONG3004726, DFNES2011192, FCBBF3010361, FEBRA2014122, FEBRA2027609, HCASM2003018, HCHON2004858, HSYRA2005628, MESAN2005303, MESAN2014412, MESAN2015501, NT2RI2008952, NT2RI2018448, NT2RI3001132, OCBBF2008144, OCBBF2009583, OCBBF2011669, OCBBF2019684, OCBBF2032274, OCBBF3000167, SPLEN2004611, SPLEN2016135, SPLEN2016781, SYNOV2021953, SYNOV4002744, TESOP2001796, TESOP2005199, TESOP2006398, TESOP2006865, TESTI2034251, TESTI4000183, TESTI4000214, TESTI4008302, TESTI4015442, TESTI4025494, TESTI4025547, TESTI4028938, TESTI4032112, THYMU2006001, THYMU2028739, TRACH2007483, TRACH3000134, TRACH3003832, TUTER2000057, UTERU2033577, UTERU3001053, TESTI4038779

The clones predicted to belong to the category of disease-related protein are the following 342 clones.
3NB692002806, ADIPS2000088, BLADE2005036, BRACE2005457, BRACE2008594, BRACE2014306, BRACE2016981, BRACE2018762, BRACE2035381, BRACE2038551, BRACE2039249, BRACE2045300, BRACE3000840, BRACE3001002, BRACE3001391, BRACE3001754, BRACE3002508, BRACE3003595, BRACE3004058, BRACE3004150, BRACE3004772, BRACE3008137, BRACE3008384, BRACE3009708, BRACE3010397, BRACE3011271, BRACE3011421, BRACE3014807, BRACE3015027, BRACE3015521, BRACE3018963, BRACE3020594, BRACE3027326, BRALZ2017359, BRAMY2005052, BRAMY2038904, BRAMY2047751, BRAMY3000213, BRAMY3005091, BRAMY3007609, BRAMY4000095, BRAMY4000229, BRAMY4000277, BRAWH2001395, BRAWH2002560, BRAWH2010000, BRAWH2010536, BRAWH2014414, BRAWH3000100, BRAWH3000491, BRAWH3001326, BRAWH3002574, BRAWH3005912, BRAWH3008341, BRCAN2002562, BRCAN2002856, BRCAN2002948, BRCAN2003746, BRCAN2006063, BRCAN2009203, BRCAN2014602, BRCAN2016619, BRCAN2017442, BRCAN2024451, BRCOC2001505, BRCOC2003213, BRHIP2000819, BRHIP2001805, BRHIP2009414, BRHIP2024165, BRHIP2026288, BRHIP3000339, BRHIP3008405, BRHIP3009448, BRHIP3027137, BRHIP3027854, BRSSN2000684, BRSSN2004719, BRSSN2014424, BRSTN2001613, BRSTN2004987, BRSTN2008418, BRTHA2002608, BRTHA2003110, BRTHA2007122, BRTHA2007603, BRTHA2008527, BRTHA2012980, BRTHA2014792, BRTHA3001721, BRTHA3002427, BRTHA3003074, BRTHA3003449, BRTHA3008778, BRTHA3009037, BRTHA3009090, BRTHA3015815, BRTHA3016917, BRTHA3017848, COLON2000568, COLON2002520, CTONG1000341, CTONG2000042, CTONG2009423, CTONG2010803, CTONG2017500, CTONG2023021, CTONG2025496, CTONG2025516, CTONG3000084, CTONG3002412, CTONG3008639, D3OST2002182 D3OST2002648, DFNES2001108, FCBBF3009888, FEBRA2007708, FEBRA2008468, FEBRA2024744, HCASM2001301, HCASM2007737, HCHON2001712, HCHON2002676, HCHON2003532, HCHON2004007, HCHON2004531, HCHON2008112, HCHON2008444, HEART1000010, HEART1000139, HEART2001680, HEART2010495, HLUNG2002465, HSYRA2005496, IMR322000127, IMR322001380, IMR322006495, KIDNE2001847, KIDNE2012945, NESOP2001694, NOVAR2001108, NT2NE2003252, NT2NE2006531, NT2NE2006909, NT2RI2006686, NT2RI2025909, NT2RI3001515, NT2RI3006171, NT2RI3006340, NT2RI3006673, NT2RI3007757, NT2RI3008652, NT2RP7000359, NT2RP7005118, NT2RP7005529, NT2RP7010599, NTONG2000413, OCBBF2006058, OCBBF2020801, OCBBF2021788, OCBBF2031167, OCBBF2033869, OCBBF2036743, OCBBF2037068, OCBBF2037340, OCBBF3003320, PEBLM2000170, PEBLM2002887, PERIC2003720, PERIC2007914, PERIC2008385, PERIC2009086, PLACE5000282, PLACE6019385, PROST1000184, PROST2003428, PROST2016462, PROST2017367, PROST2018090, PROST2018511, PUAEN2002489, PUAEN2009795, SKNMC2007504, SMINT2010076, SPLEN2002467, SPLEN2006122, SPLEN2011422, SPLEN2012624, SPLEN2021701, SPLEN2031547, SPLEN2033098, SPLEN2036326, SPLEN2036821, SPLEN2036932, SYNOV2005817, SYNOV2012326, SYNOV2014400, SYNOV2021320, SYNOV3000231, SYNOV3000302, SYNOV4002883, SYNOV4004741, SYNOV4007360, SYNOV4007521, SYNOV4007553, SYNOV4007671, SYNOV4008440, TBAES2001229, TBAES2001258, TESOP2004114, TESOP2005485, TESOP2009121, TESTI1000257, TESTI1000319, TESTI2000644, TESTI2002618, TESTI2005610, TESTI2024567, TESTI2026505, TESTI2050987, TESTI2051867, TESTI2053399, TESTI2053621, TESTI4000014, TESTI4000079, TESTI4000288, TESTI4000349, TESTI4000724, TESTI4001148, TESTI4001176, TESTI4001527, TESTI4001561, TESTI4002491, TESTI4006420, TESTI4006819, TESTI4007163, TESTI4007778, TESTI4007810, TESTI4008050, TESTI4008429, TESTI4009160, TESTI4009457, TESTI4009881, TESTI4010851, TESTI4011745, TESTI4011956, TESTI4012406, TESTI4012448, TESTI4012505, TESTI4012679, TESTI4013369, TESTI4013924, TESTI4014175, TESTI4016110, TESTI4016822, TESTI4016925, TESTI4017901, TESTI4018835, TESTI4018881, TESTI4018886, TESTI4020092, TESTI4021478, TESTI4022873, TESTI4023546, TESTI4026524, TESTI4027557, TESTI4028059, TESTI4028429, TESTI4028880, TESTI4030069, TESTI4034632, TESTI4034912, TESTI4035063, TESTI4035498, TESTI4036909, TESTI4037156, TESTI4040363, THYMU1000496, THYMU2005303, THYMU2008725, THYMU2019210, THYMU2027497, THYMU2027695, THYMU2027734, THYMU2031046, THYMU2033104, THYMU2035319, THYMU2037233, THYMU2041015, THYMU3001083, THYMU3001234, THYMU3001379, THYMU3003309, THYMU3004835, THYMU3006118, THYMU3007137, THYMU3008436, TKIDN2000701, TKIDN2006852, TLIVE2001327, TRACH2001549, TRACH2007059, TRACH2022425, TRACH2022649, TRACH3000558, TRACH3002168, TRACH3004721, TRACH3004786, TRACH3005549, TRACH3007479, TRACH3008629, TRACH3009455, TRACH3035526, TSTOM2000442, TUTER2000904, UTERU1000337, UTERU2005621, UTERU2007724, UTERU2017762, UTERU2019491, UTERU2019706, UTERU2025025, UTERU2026090, UTERU2027591, UTERU2035328, UTERU3000645, UTERU3000828, UTERU3000899, UTERU3001240, UTERU3001572, UTERU3001585, UTERU3001652, UTERU3003116, UTERU3003135, UTERU3005907, UTERU3007640, UTERU3008671, UTERU3009490, UTERU3009690, UTERU3009979, UTERU3015500, UTERU3016789

The following 84 clones are also predicted to belong to the category of disease-related protein.
BRACE3001113, BRACE3010076, BRAMY2039341, BRAMY3004800, BRAWH3009017, BRCAN2002473, BRCAN2002854, BRCAN2003070, BRHIP2005271, BRHIP2017553, BRHIP2026877, BRHIP3000240, BRHIP3008314, BRHIP3026052, BRSTN2013354, BRTHA2016318, BRTHA2017972, BRTHA3003000, CERVX2002013, CTONG1000113, CTONG2008721, CTONG2020411, CTONG3004550, FCBBF1000509, FEBRA2008692, HCASM2008536, HCHON2004858, HEART2009680, HLUNG2015548, HSYRA2005628, IMR322008651, IMR322013396, MESAN2001770, NT2RI2009583, NT2RI3007443, OCBBF2003327, OCBBF2009583, OCBBF2011669, OCBBF2024284, OCBBF2032274, OCBBF3000167, OCBBF3002654, PLACE7000502, PROST2000452, PROST2009320, SPLEN2004611, STOMA2003158, SYNOV1000256, SYNOV4002744, SYNOV4003981, TBAES2000932, TESOP2000390, TESOP2001796, TESOP2005199, TESTI2015626, TESTI2025924, TESTI2026647, TESTI2039060, TESTI4000183, TESTI4006473, TESTI4011070, TESTI4017714, TESTI4019657, TESTI4021482, TESTI4024387, TESTI4025494, TESTI4025547, TESTI4028938, TESTI4031745, TESTI4032112, THYMU2004284, THYMU2028739, THYMU2031139, THYMU2031249, THYMU2035710, THYMU3000269, TLIVE2001684, TLIVE2002046, TRACH2024408, TRACH3003683, UTERU2021820, UTERU2032279, UTERU2033577, UTERU3000738

In particular, hit data of the following 338 clones for Swiss-Prot, or GenBank, UniGene, nr, or RefSeq corresponded to genes or proteins which had been deposited in the Online Mendelian Inheritance in Man (OMIM), which is the human gene and disease database (the OMIM Number is shown in the parenthesis after the Clone Name).
3NB692002806 (261630), ADIPS2000088 (147120), BLADE2005036 (114850), BRACE2005457 (274600;603545;600791), BRACE2008594 (601959), BRACE2014306 (193002), BRACE2016981 (602701), BRACE2018762 (604800), BRACE2035381 (606088), BRACE2038551 (601961),
BRACE2039249 (602273), BRACE2045300 (601442), BRACE3000840 (600355), BRACE3001002 (300236), BRACE3001391 (601313;173900), BRACE3001754 (185641), BRACE3002508 (606417), BRACE3003595 (602941), BRACE3004058 (250800), BRACE3004150 (601035), BRACE3004772 (603143), BRACE3008137 (602187), BRACE3008384 (603264), BRACE3009708 (182340), BRACE3010397 (602187), BRACE3011271 (602187), BRACE3011421 (602187), BRACE3014807 (605784), BRACE3015027 (602187), BRACE3015521 (605888), BRACE3018963 (605744), BRACE3020594 (400023), BRACE3027326 (602187), BRALZ2017359 (604331), BRAMY2005052 (602621), BRAMY2038904 (605671), BRAMY2047751 (602512), BRAMY3000213 (605448), BRAMY3005091 (600286), BRAMY3007609 (300315), BRAMY4000095 (602187), BRAMY4000229 (602159), BRAMY4000277 (602187), BRAWH2001395 (159430), BRAWH2002560 (602865), BRAWH2010000 (602581), BRAWH2010536 (604010), BRAWH2014414 (603006), BRAWH3000100 (601403), BRAWH3000491 (602187), BRAWH3001326 (602187), BRAWH3002574 (602187), BRAWH3005912 (602187), BRAWH3008341 (602187), BRCAN2002562 (602187), BRCAN2002856 (602712), BRCAN2002948 (603534), BRCAN2003746 (311870), BRCAN2006063 (603196;601369), BRCAN2009203 (603143), BRCAN2014602 (601441), BRCAN2016619 (602187), BRCAN2017442 (604455), BRCAN2024451 (602513), BRCOC2001505 (159430), BRCOC2003213 (602187), BRHIP2000819 (605000), BRHIP2001805 (603219), BRHIP2009414 (602187), BRHIP2024165 (604402), BRHIP2026288 (602187), BRHIP3000339 (159430), BRHIP3008405 (602187), BRHIP3009448 (602187), BRHIP3027137 (600249), BRHIP3027854 (601060), BRSSN2000684 (603505), BRSSN2004719 (600560), BRSSN2014424 (606105), BRSTN2001613 (164020), BRSTN2004987 (604733), BRSTN2008418 (602187), BRTHA2002608 (600463), BRTHA2003110 (602187), BRTHA2007122 (106410), BRTHA2007603 (605846), BRTHA2008527 (152790;176410), BRTHA2012980 (300119), BRTHA2014792 (601674), BRTHA3001721 (604902),
BRTHA3002427 (602187), BRTHA3003074 (605367), BRTHA3003449 (160745), BRTHA3008778 (602187), BRTHA3009037 (602187), BRTHA3009090 (603197), BRTHA3015815 (600902), BRTHA3016917 (604137), BRTHA3017848 (603377;212140), COLON2000568 (147000), COLON2002520 (602187), CTONG1000341 (188040), CTONG2000042 (103950), CTONG2009423 (182137), CTONG2010803 (602189), CTONG2023021 (602498), CTONG2025496 (103950), CTONG2025516 (601679), CTONG3000084 (600888), CTONG3002412 (601403), CTONG3008639 (601797), D3OST2002182 (603590), D3OST2002648 (603071), DFNES2001108 (603560), FCBBF3009888 (602470), FEBRA2007708 (126650;214700), FEBRA2008468 (278000), HCASM2001301 (602399), HCASM2007737 (601504), HCHON2001712 (109190) ,
HCHON2002676 (252800), HCHON2003532 (172490), HCHON2004007 (605866), HCHON2004531 (602187), HCHON2008112 (605837), HCHON2008444 (602187), HEART1000010 (602187), HEART1000139 (191045;115195), HEART2001680 (146900), HEART2010495 (157132), HLUNG2002465 (605216), HSYRA2005496 (131195;187300), IMR322000127 (604077), IMR322001380 (605652), IMR322006495 (605607), KIDNE2012945 (600270), NOVAR2001108 (147120), NT2NE2003252 (602913), NT2NE2006531 (602277), NT2NE2006909 (602187),
NT2RI2006686 (602700), NT2RI2025909 (212138), NT2RI3001515 (300362), NT2RI3006171 (114890), NT2RI3006340 (602187), NT2RI3006673 (602187), NT2RI3007757 (605396), NT2RI3008652 (602654), NT2RP7000359 (603271), NT2RP7005118 (603379), NT2RP7005529 (600888), NT2RP7010599 (603684), NTONG2000413 (602262), OCBBF2006058 (604773), OCBBF2020801 (602187), OCBBF2021788 (602597), OCBBF2031167 (603709), OCBBF2033869 (600270), OCBBF2036743 (604075), OCBBF2037068 (602187), OCBBF2037340 (602187), OCBBF3003320 (605868), PEBLM2000170 (602187), PEBLM2002887 (602187), PERIC2003720 (600381), PERIC2007914 (400009), PERIC2008385 (604455), PERIC2009086 (600134;605158), PLACE5000282 (130160), PLACE6019385 (602448), PROST1000184 (192321), PROST2003428 (602187), PROST2016462 (602187), PROST2017367 (600585), PROST2018090 (312610), PROST2018511 (602187), PUAEN2002489 (604658), PUAEN2009795 (601456), SKNMC2007504 (602187), SMINT2010076 (146900), SPLEN2002467 (605652), SPLEN2006122 (604739), SPLEN2011422 (114213), SPLEN2012624 (602187), SPLEN2021701 (142800), SPLEN2031547 (602187), SPLEN2033098 (602746), SPLEN2036326 (602101), SPLEN2036821 (212138), SPLEN2036932 (605577), SYNOV2005817 (123889), SYNOV2012326 (604336), SYNOV2014400 (135820), SYNOV2021320 (602104), SYNOV3000231 (147100), SYNOV3000302 (147100), SYNOV4002883 (602187), SYNOV4004741 (602187), SYNOV4007360 (602187), SYNOV4007521 (605830), SYNOV4007553 (603028), SYNOV4007671 (602187), SYNOV4008440 (602187), TBAES2001229 (602187), TBAES2001258 (142440), TESOP2004114 (601865), TESOP2005485 (147170), TESOP2009121 (117143), TESTI1000257 (138170), TESTI1000319 (602187), TESTI2000644 (601392), TESTI2002618 (601533), TESTI2005610 (601040), TESTI2024567 (601116), TESTI2026505 (305400), TESTI2050987 (605968), TESTI2051867 (180479), TESTI2053399 (605819), TESTI2053621 (600364;602093), TESTI4000014 (602187), TESTI4000079 (603560), TESTI4000288 (602187), TESTI4000349 (604506), TESTI4000724 (603878), TESTI4001148 (602187), TESTI4001176 (601430), TESTI4001527 (602187), TESTI4001561 (602187), TESTI4002491 (602187), TESTI4006420 (605612), TESTI4006819 (602187), TESTI4007163 (602187), TESTI4007778 (602187), TESTI4007810 (600940), TESTI4008050 (602187), TESTI4008429 (602187), TESTI4009160 (602187), TESTI4009457 (606185), TESTI4009881 (602187), TESTI4010851 (602187), TESTI4011745 (602187), TESTI4011956 (602187), TESTI4012406 (602187), TESTI4012448 (185261), TESTI4012505 (602143), TESTI4012679 (601933), TESTI4013369 (602187), TESTI4013924 (602187), TESTI4014175 (602187), TESTI4016110 (602187), TESTI4016822 (601792), TESTI4016925 (602187), TESTI4017901 (104221), TESTI4018835 (602187), TESTI4018881 (605070), TESTI4018886 (602187), TESTI4020092 (156225), TESTI4021478 (605868), TESTI4022873 (602187), TESTI4023546 (602187), TESTI4026524 (603277), TESTI4027557 (602187), TESTI4028059 (232800;171850), TESTI4028429 (602187), TESTI4028880 (138170), TESTI4030069 (604603), TESTI4034632 (606251), TESTI4034912 (602187), TESTI4035063 (602187), TESTI4035498 (602187), TESTI4036909 (602187), TESTI4037156 (606026), TESTI4040363 (185641), THYMU1000496 (603060), THYMU2005303 (186910), THYMU2008725 (176882), THYMU2019210 (142830), THYMU2027497 (182139), THYMU2027695 (147100), THYMU2027734 (145505), THYMU2031046 (604207), THYMU2033104 (605349), THYMU2035319 (604739), THYMU2037233 (605121), THYMU2041015 (602187), THYMU3001083 (602187), THYMU3001234 (602187), THYMU3001379 (602187), THYMU3003309 (300359), THYMU3004835 (602187), THYMU3006118 (603708), THYMU3007137 (602187), THYMU3008436 (602187), TKIDN2000701 (600465), TKIDN2006852 (603602), TLIVE2001327 (601403), TRACH2001549 (603197), TRACH2007059 (602187), TRACH2022425 (146900), TRACH2022649 (147100), TRACH3000558 (600140), TRACH3002168 (155735), TRACH3004721 (602187), TRACH3004786 (602187), TRACH3005549 (602187), TRACH3007479 (602308), TRACH3008629 (600976), TRACH3009455 (171833), TRACH3035526 (147000), TSTOM2000442 (147100), TUTER2000904 (602187), UTERU1000337 (602187), UTERU2005621 (603505), UTERU2007724 (602373), UTERU2017762 (601053), UTERU2019491 (603762), UTERU2019706 (600114), UTERU2025025 (191315;164970;256000), UTERU2026090 (605497), UTERU2027591 (600150),
UTERU2035328 (605409), UTERU3000645 (602909), UTERU3000828 (602187), UTERU3000899 (603062), UTERU3001240 (602187), UTERU3001572 (602187), UTERU3001585 (602187), UTERU3001652 (602715), UTERU3003116 (602187), UTERU3003135 (602187), UTERU3005907 (190196), UTERU3007640 (603215), UTERU3008671 (182120), UTERU3009490 (604585), UTERU3009690 (104221), UTERU3009979 (600441), UTERU3015500 (606667), UTERU3016789 (602104)

Additionally, hit data of the following 84 clones for Swiss-Prot, or nr or RefSeq corresponded to genes or proteins which had been deposited in the Online Mendelian Inheritance in Man (OMIM), which is the human gene and disease database (the OMIM Number is shown in the parenthesis after the Clone Name).
BRACE3001113 (603971), BRACE3010076 (142695), BRAMY2039341 (604077), BRAMY3004800 (602187), BRAWH3009017 (602187), BRCAN2002473 (602187), BRCAN2002854 (602895), BRCAN2003070 (605574), BRHIP2005271 (600267), BRHIP2017553 (602187), BRHIP2026877 (600341), BRHIP3000240 (601142), BRHIP3008314 (604480), BRHIP3026052 (601645), BRSTN2013354 (602187), BRTHA2016318 (605442), BRTHA2017972 (602932), BRTHA3003000 (605276), CERVX2002013 (602903), CTONG1000113 (602277), CTONG2008721 (605317), CTONG2020411 (601930), CTONG3004550 (605611), FCBBF1000509 (601933), FEBRA2008692 (604034), HCASM2008536 (194360), HCHON2004858 (602187), HEART2009680 (601970), HLUNG2015548 (146690), HSYRA2005628 (602187), IMR322008651 (179617), IMR322013396 (600053), MESAN2001770 (600495), NT2RI2009583 (605949), NT2RI3007443 (602448), OCBBF2003327 (605008), OCBBF2009583 (602277), OCBBF2011669 (602187), OCBBF2024284 (176981), OCBBF2032274 (603975), OCBBF3000167 (194558), OCBBF3002654 (601893), PLACE7000502 (164951), PROST2000452 (602060), PROST2009320 (605903), SPLEN2004611 (602228), STOMA2003158 (602244), SYNOV1000256 (606021), SYNOV4002744 (602187), SYNOV4003981 (604283), TBAES2000932 (606212), TESOP2000390 (602187), TESOP2001796 (602187), TESOP2005199 (194531), TESTI2015626 (601249), TESTI2025924 (600863), TESTI2026647 (601235), TESTI2039060 (154360), TESTI4000183 (601276), TESTI4006473 (602187), TESTI4011070 (602187), TESTI4017714 (602187), TESTI4019657 (602052), TESTI4021482 (164730), TESTI4024387 (602187), TESTI4025494 (602187), TESTI4025547 (605308), TESTI4028938 (603899), TESTI4031745 (602448), TESTI4032112 (603246), THYMU2004284 (314370), THYMU2028739 (604191), THYMU2031139 (605009), THYMU2031249 (311550), THYMU2035710 (601890), THYMU3000269 (600857), TLIVE2001684 (120700), TLIVE2002046 (125270), TRACH2024408 (106410), TRACH3003683 (150205), UTERU2021820 (126141), UTERU2032279 (600942), UTERU2033577 (603397), UTERU3000738 (602187)

The clones predicted to belong to the category of enzyme and/or metabolism-related protein are the following 171 clones.
3NB692002806, ASTRO2002842, BLADE2005036, BRACE2008594, BRACE2030341, BRACE2035381, BRACE2038551, BRACE2039249, BRACE2041200, BRACE2045772, BRACE3004058, BRACE3009708, BRACE3011421, BRACE3016884, BRACE3024073, BRACE3025630, BRAMY2033267, BRAMY2039872, BRAMY3002803, BRAMY3004919, BRAMY3005091, BRAMY3005932, BRAMY4000095, BRAWH3002574, BRAWH3008341, BRCAN2003703, BRCAN2003746, BRCAN2009432, BRCAN2014602, BRCAN2017442, BRCAN2028355, BRCOC2003213, BRHIP2024165, BRHIP3008405, BRHIP3027137, BRHIP3027854, BRSTN2000872, BRSTN2004863, BRSTN2004987, BRSTN2008418, BRTHA2002608, BRTHA2009311, BRTHA2015406, BRTHA2016496, BRTHA3008778, BRTHA3009090, BRTHA3015815, BRTHA3016917, CTONG2004062, CTONG2006798, CTONG2013178, CTONG2028124, CTONG3009028, D3OST2002182, DFNES2001108, DFNES2005266, FCBBF3013307, FCBBF3023895, FEBRA2008468, FEBRA2026984, HCASM2001301, HCHON2002676, HCHON2003532, HCHON2004007, HEART2006131, HEART2010492, HHDPC1000118, HLUNG2011298, HLUNG2013204, HSYRA2008714, KIDNE2001361, KIDNE2006580, NT2NE2003252, NT2NE2006909, NT2RI2004618, NT2RI2025909, NT2RI3006673, NT2RI3007978, NT2RI3008974, NT2RP7000359, NT2RP7004027, NT2RP7010599, NT2RP7014005, NTONG2000413, NTONG2008672, OCBBF2006005, OCBBF2006058, OCBBF2006151, OCBBF2019823, OCBBF2025527, OCBBF2030354, OCBBF2031167, OCBBF3003320, PEBLM2005183, PERIC2000889, PERIC2008385, PLACE6019385, PLACE7008431, PROST2017367, PUAEN2007044, PUAEN2009655, PUAEN2009852, SKNMC2006998, SKNMC2007504, SMINT1000192, SPLEN2010912, SYNOV2012326, SYNOV4002883, TBAES2001258, TESOP2000801, TESOP2004114, TESTI2005610, TESTI2005739, TESTI2016046, TESTI4000079, TESTI4000209, TESTI4000288, TESTI4000349, TESTI4001176, TESTI4001527, TESTI4001561, TESTI4002552, TESTI4006148, TESTI4006819, TESTI4007810, TESTI4008429, TESTI4010851, TESTI4012406, TESTI4012448, TESTI4013369, TESTI4013817, TESTI4014175, TESTI4016822, TESTI4018152, TESTI4018835, TESTI4019566, TESTI4021478, TESTI4022716, TESTI4023546, TESTI4026510, TESTI4026524, TESTI4028059, TESTI4029836, TESTI4034632, TESTI4036909, TESTI4046819, THYMU2008725, THYMU2027734, THYMU2031046, THYMU2031258, THYMU3001234, THYMU3003212, THYMU3004157, THYMU3004835, THYMU3006118, THYMU3008436, TKIDN2006852, TLIVE2002336, TRACH2001549, TRACH2009310, TRACH3007479, TRACH3036193, UTERU1000337, UTERU2019491, UTERU2025025, UTERU2026203, UTERU3000665, UTERU3001240, UTERU3001585, UTERU3003116, UTERU3005907

The following 59 clones are also predicted to belong to the category of enzyme and/or metabolism-related protein.
BRACE2039823, BRACE3010076, BRAMY2038516, BRAWH1000369, BRCAN2003070, BRHIP2005271, BRHIP2012360, BRHIP2026877, BRHIP3008314, BRTHA2013610, BRTHA2017364, BRTHA2017972, BRTHA2018011, BRTHA2018443, BRTHA3003000, CTONG2016942, FCBBF1000509, FEBRA2008692, HCASM2003099, HLUNG2015548, MESAN2005303, NT2RI3000174, NT2RI3007443, NT2RP7008435, NTONG2008093, OCBBF2003327, OCBBF2034637, OCBBF3002654, PROST2000452, SPLEN2039311, SPLEN2039379, STOMA2003158, TESOP2000390, TESTI2015626, TESTI2025924, TESTI2026647, TESTI2032643, TESTI2036288, TESTI2039060, TESTI4006473, TESTI4011070, TESTI4014801, TESTI4017714, TESTI4019657, TESTI4021482, TESTI4031745, THYMU2004139, THYMU2004284, THYMU2031139, THYMU2031249, THYMU2040925, THYMU3000269, TLIVE2002046, TLIVE2007607, TRACH2024559, TRACH3003683, TRACH3007866, UTERU2021820, UTERU3000738

The clones predicted to belong to the category of cell division and/or cell proliferation-related protein are the following 42 clones.
BLADE2002782, BRACE2042550, BRACE2043248, BRACE3000840, BRALZ2017359, BRAMY2038484, BRAMY2046989, BRAWH2010536, BRAWH2014954, BRAWH3000100, BRHIP2000819, BRHIP2001927, BRHIP2009414, BRSSN2000684, CTONG3002412, CTONG3008258, CTONG3008639, FCBBF3002163, HCASM2001301, IMR322006495, NT2RI2006686, OCBBF2021020, OCBBF2037068, OCBBF3004314, PLACE5000282, PLACE6019385, PLACE7002641, PUAEN2006328, SPLEN2033098, TESOP2009121, TESTI1000545, TESTI2003573, TESTI2005610, TESTI4007810, TESTI4017901, THYMU2034374, THYMU2039315, TLIVE2001327, TRACH2025507, UTERU2005621, UTERU3009690, UTERU3009979

The following ten clones are also predicted to belong to the category of cell division and/or cell proliferation-related protein.
BRCAN2003070, BRTHA3003000, NT2RI3007443, PLACE7000502, SPLEN2004611, STOMA2003158, SYNOV4003981, TESTI4031745, THYMU2004139, THYMU2035078

The clones predicted to belong to the category of cytoskeleton-related protein are the following 55 clones.
ASTRO1000009, BLADE2004089, BRACE2026836, BRACE2045300, BRACE3006872, BRAMY3008466, BRAWH2001395, BRAWH2005315, BRAWH3002600, BRCOC2001505, BRHIP2000819, BRHIP3000339, BRHIP3008405, BRTHA2007122, BRTHA3003449, COLON2002520, CORDB2000541, FCBBF3021940, HCHON2001577, HEART1000139, HEART2010495, NT2RI3006340, NT2RP7000359, NTONG2005277, OCBBF2007068, OCBBF3003592, PERIC2000889, PLACE5000282, PROST1000559, SKMUS2006394, SPLEN2011422, SPLEN2015679, TESTI2049857, TESTI4000288, TESTI4001148, TESTI4007778, TESTI4009160, TESTI4009881, TESTI4011956, TESTI4013924, TESTI4016925, TESTI4018886, TESTI4022873, TESTI4034912, TESTI4035063, TESTI4037727, THYMU1000496, THYMU2035735, THYMU3001083, THYMU3001234, TKIDN2000701, UTERU2007724, UTERU2008347, UTERU2035745, UTERU3003178

The following six clones are also predicted to belong to the category of cytoskeleton-related protein.
HLUNG2015418, SPLEN2030847, SPLEN2036702, TESTI4025268, TESTI4026207, TRACH2024408

The clones predicted to belong to the category of nuclear protein and/or RNA synthesis-related protein are the following 42 clones.
BLADE2007958, BRACE2010489, BRACE2045300, BRACE3004150, BRACE3005430, BRACE3011421, BRAMY2046989, BRAMY3005932, BRCAN2002562, BRHIP2021615, BRSTN2001613, BRSTN2004987, COLON2000470, CTONG3009028, FCBBF3013307, HCHON2004531, IMR322006495, OCBBF2020801, PEBLM2005183, PUAEN2007044, SKNMC1000124, SMINT1000192, SPLEN2006122, SPLEN2010912, TESOP2009121, TESTI4009374, TESTI4009457, TESTI4013830, TESTI4019566, TESTI4022716, THYMU2033104, THYMU2035319, THYMU2038301, THYMU2040975, THYMU3001379, TRACH3004721, TRACH3036609, UTERU2026025, UTERU3000828, UTERU3001572, UTERU3003135, UTERU3004992

The following 16 clones are also predicted to belong to the category of nuclear protein and/or RNA synthesis-related protein.
BRACE3010076, BRCAN2002854, BRHIP2006617, BRHIP2012360, BRHIP3026052, BRSTN2013354, BRTHA2017364, HCASM2003099, HCASM2008536, IMR322008651, NT2RI3000174, STOMA2003158, TESTI2026647, TESTI4006473, TESTI4021482, THYMU2035078

The clones predicted to belong to the category of protein synthesis and/or transport-related protein are the following 57 clones.
ASTRO2002842, BLADE2005036, BRACE3025630, BRAMY2033003, BRAMY3007609, BRAWH3000491, BRAWH3002574, BRAWH3008341, BRCAN2002856, BRCAN2002948, BRCOC2003213, BRSTN2004987, BRTHA2016496, BRTHA3013884, BRTHA3016917, CTONG2000042, CTONG2013178, CTONG2023512, CTONG2024749, CTONG2025496, CTONG3001370, DFNES2005266, FEBRA2026984, HCASM2007737, HCHON2008444, HEART1000010, KIDNE2000846, NT2NE2006909, NT2RI2011422, NT2RP7004027, NTONG2000413, OCBBF2031167, TBAES2001229, TBAES2001258, TESTI1000319, TESTI2005610, TESTI2051867, TESTI4000209, TESTI4000349, TESTI4001106, TESTI4002491, TESTI4008050, TESTI4010851, TESTI4012406, TESTI4012448, TESTI4013924, TESTI4028429, TESTI4034912, THYMU2009157, TLIVE2008229, TRACH3007479, TRACH3008713, TRACH3036193, UTERU2019940, UTERU3001988, UTERU3003116, UTERU3007419

The following 15 clones are also predicted to belong to the category of protein synthesis and/or transport-related protein.
BRTHA2007060, BRTHA2018011, CTONG2016942, MESAN2001770, MESAN2005303, NT2RP7008435, OCBBF2003327, PROST2000452, TESOP2001796, TESTI4017714, THYMU2004284, THYMU2031139, TRACH2024559, TRACH3007866, UTERU2021820

The clones predicted to belong to the category of cellular defense-related protein are the following three clones.
BRACE3005430, HCHON2004531, TESTI4007810

The following four clones are also predicted to belong to the category of cellular defense-related protein.
BRHIP2012360, FCBBF3027854, HCASM2008536, UTERU2032279

The clones predicted to belong to the category of development and/or differentiation-related protein are the following nine clones.
BRACE3009747, BRTHA2005579, BRTHA3003343, IMR322000917, PEBLM2000170, TESOP2001122, TESOP2001953, TESTI2040018, UTERU3006308

The following five clones are also predicted to belong to the category of development and/or differentiation-related protein.
BRALZ2017844, CTONG2020378, HCHON2004858, OCBBF2019684, THYMU2006001

The clones predicted to belong to the category of DNA-binding and/or RNA-binding protein are the following 55 clones.
3NB692002685, BLADE2007958, BRACE2030326, BRACE2045596, BRACE3001002, BRACE3004150, BRACE3009747, BRACE3045033, BRCAN2002562, BRHIP2021615, BRSSN2014299, BRSTN2001613, BRSTN2004987, BRTHA2014792, BRTHA3001721, BRTHA3003343, CTONG2025516, CTONG3008831, CTONG3009028, FCBBF3013307, FEBRA2007544, FEBRA2007801, HEART1000074, IMR322000127, IMR322000917, NT2NE2006531, NT2RI3009158, OCBBF2020838, OCBBF2036743, PEBLM2002887, PEBLM2005183, SKNMC2007504, SMINT1000192, SPLEN2006122, TBAES2001229, TESTI2014716, TESTI2040018, TESTI2044796, TESTI4009374, TESTI4012679, TESTI4014175, TESTI4017543, TESTI4026510, TESTI4026524, THYMU2006420, THYMU2035319, THYMU2037233, THYMU2040975, THYMU3004866, TLIVE2008229, TRACH3036609, TUTER2000425, UTERU2026025, UTERU2035328, UTERU3009490

The following 74 clones are also predicted to belong to the category of DNA-binding and/or RNA-binding protein.
BRACE2003609, BRACE3001058, BRACE3001113, BRACE3010076, BRALZ2017844, BRAMY2035070, BRAMY2035449, BRAMY2035718, BRAMY2039341, BRAWH1000369, BRAWH2006207, BRCAN2002854, BRHIP2012360, BRHIP2017553, BRSTN2013354, BRTHA2017364, CERVX2002013, CTONG1000113, CTONG2008721, CTONG2020378, CTONG2020411, CTONG2028758, CTONG3004726, DFNES2011192, FEBRA2014122, FEBRA2027609, HCASM2003018, HCASM2009424, HCHON2004858, HSYRA2005628, IMR322008651, MESAN2001770, MESAN2005303, MESAN2014412, MESAN2015501, NT2RI2008952, NT2RI2018448, NT2RI3000174, NT2RI3001132, OCBBF2008144, OCBBF2009583, OCBBF2011669, OCBBF2019684, OCBBF2032274, OCBBF3000167, SPLEN2004611, SPLEN2016135, SPLEN2016781, SYNOV2021953, SYNOV4002744, TESOP2005199, TESOP2006398, TESOP2006865, TESTI2026647, TESTI2034251, TESTI4000183, TESTI4000214, TESTI4006473, TESTI4008302, TESTI4015442, TESTI4025494, TESTI4025547, TESTI4028938, TESTI4032112, THYMU2006001, THYMU2035078, TRACH2007483, TRACH3000134, TRACH3002561, TRACH3003832, TUTER2000057, UTERU2033577, UTERU3001053, TESTI4038779

The clones predicted to belong to the category of ATP binding and/or GTP-binding protein are the following 68 clones.
BNGH42007788, BRACE2008594, BRACE2047377, BRACE3005430, BRACE3008720, BRACE3009708, BRACE3015521, BRACE3024073, BRAMY4000095, BRCAN2009432, BRCOC2003213, BRHIP3008405, BRSTN2013741, BRTHA3003449, BRTHA3015815, BRTHA3016917, COLON2002520, FEBRA2026984, HCASM2001301, HCHON2004007, HSYRA2008714, KIDNE2001361, KIDNE2001847, NESOP2001694, NT2RI2005166, NT2RP7013795, OCBBF3003320, OCBBF3003592, PEBLM2002594, PERIC2000889, PLACE6019385, SMINT1000192, SPLEN2037194, TESOP2000801, TESTI2006648, TESTI4000288, TESTI4001148, TESTI4001176, TESTI4002552, TESTI4007810, TESTI4008429, TESTI4009160, TESTI4009881, TESTI4011956, TESTI4013817, TESTI4014175, TESTI4016925, TESTI4018208, TESTI4018835, TESTI4019566, TESTI4021478, TESTI4022873, TESTI4026524, TESTI4029836, TESTI4035498, TESTI4036909, TESTI4037727, THYMU1000496, THYMU2033079, THYMU3001083, THYMU3001234, THYMU3001379, TRACH2009310, UTERU2019706, UTERU2025025, UTERU2035745, UTERU3000665, UTERU3000828

The following 24 clones are also predicted to belong to the category of ATP binding and/or GTP-binding protein.
BRHIP2026877, BRTHA2017364, BRTHA2018443, IMR322008651, IMR322013731, NT2RI3007443, NTONG2008093, OCBBF3002654, TESOP2000390, TESOP2007384, TESTI2025924, TESTI2026647, TESTI2049956, TESTI4005317, TESTI4006473, TESTI4021482, TESTI4026207, TESTI4031745, THYMU2004139, THYMU2031249, TRACH2000862, TRACH2024559, TRACH3000420, UTERU3000738

The 119 clones shown below are clones which were unassignable to any of the above-mentioned categories, but have been predicted to have some functions based on homology search using their full-length nucleotide sequences. Clone Name and Definition in the result of homology search, demarcated by a double slash mark (//), are shown below.
ADRGL2009691// Mus musculus D11lgp1 mRNA, complete cds.
ADRGL2009755// Homo sapiens brain and reproductive organ-expressed protein (BRE) mRNA, complete cds.
ASTRO3000177// Drosophila melanogaster BcDNA.GH03694 (BcDNA.GH03694) mRNA, complete cds.
BLADE2008398// Homo sapiens LRR FLI-I interacting protein 2 (LRRFIP2) mRNA, complete cds.
BRACE2006319// Homo sapiens mRNA for Fln29, complete cds.
BRACE2027258// Homo sapiens E2a-Pbx1-associated protein (EB-1) mRNA, partial cds.
BRACE2038329// Rattus norvegicus CBL-B (Cbl-b) mRNA, partial cds.
BRACE2046251// Homo sapiens hucep-10 mRNA for cerebral protein-10, complete cds.
BRACE3003192// latent transforming growth factor beta binding protein 3 [Homo sapiens]
BRACE3007625// espin [Rattus norvegicus]
BRACE3009297// mdgl-1 [Mus musculus]
BRACE3015262// espin [Mus musculus]
BRACE3025457// testis-specific protein TSP-NY [Homo sapiens]
BRALZ2016498// Homo sapiens FKSG76 (FKSG76) mRNA, complete cds.
BRAMY2030109// Homo sapiens hucep-4 mRNA for cerebral protein-4, complete cds.
BRAMY2031317// Mus musculus semaphorin cytoplasmic domain-associated protein 3A (Semcap3) mRNA, complete cds.
BRAMY2047746// nasopharyngeal carcinoma susceptibility protein [Homo sapiens]
BRAMY3001794// Rattus norvegicus Circadian Oscillatory Protein (SCOP) (Scop)
BRAWH2001940// H.sapiens gene from PAC 1026E2, partial.
BRAWH2012162// KE03 protein [Homo sapiens]
BRAWH2016724// MAP2=HMW-MAP2 {alternatively spliced} [rats, brain, mRNA Partial, 267 nt].
BRAWH3002821// synaptotagmin-like 2 [Mus musculus]
BRCAN2002944// Mus musculus huntington yeast partner C (Hypc) mRNA, complete cds.
BRCAN2013660// Arabidopsis thaliana putative protein (F4F15.330) mRNA, complete cds.
BRHIP2002122// Homo sapiens B aggressive lymphoma long isoform (BAL) mRNA, complete cds.
BRHIP2003786// CCA3 [Rattus norvegicus]
BRHIP2004359// ELAC PROTEIN.
BRHIP2007616// plexin 2
BRHIP2029393// COBW-like protein [Homo sapiens]
BRHIP3008313// testis specific ankyrin-like protein 1 [Homo sapiens]
BRSSN2013874// TEMO [Rattus norvegicus]
BRSTN2017771// Homo sapiens putative BTK-binding protein mRNA, complete cds.
BRTHA2012392// Homo sapiens HCDI (HCDI) mRNA, complete cds.
BRTHA3002933// uroplakin 3 [Homo sapiens]
BRTHA3008310// Mus musculus mRNA for iroquois homeobox protein 6 (Irx6 gene).
BRTHA3008520// sporulation-induced transcript 4-associated protein; hypothetical protein FLJ11058 [Homo sapiens]
COLON2001721// GLUT4 vesicle protein [Mus musculus]
CTONG1000467// Mus musculus mRNA for Deltex3, complete cds.
CTONG2020026// Drosophila melanogaster BcDNA.GH09358 (BcDNA.GH09358) mRNA, complete cds.
CTONG3001123// Mus musculus Pax transcription activation domain interacting protein PTIP mRNA, complete cds.
CTONG3002127// granuphilin [Mus musculus]
CTONG3004072// GL002 protein [Homo sapiens]
CTONG3006186// syntaxin binding protein 4 [Mus musculus]
CTONG3008894// Mus musculus SH3-domain binding protein 5
FCBBF1000297// Human protein immuno-reactive with anti-PTH polyclonal antibodies mRNA, partial cds.
HCHON2000028// Homo sapiens 7h3 protein mRNA, partial cds.
HCHON2000626// X-linked protein STS1769.
HCHON2001217// Homo sapiens cullin CUL4B (CUL4B) mRNA, complete cds.
HEART2006909// Hemolysin C.
HLUNG2011041// basic proline-rich peptide IB-8a - human (fragments)
HLUNG2014288// Mus musculus RP42 mRNA, complete cds.
IMR322006886// Homo sapiens hepatocellular carcinoma-associated antigen 127 (HCA127) mRNA, complete cds.
KIDNE2002252// Drosophila melanogaster BcDNA.GH09358 (BcDNA.GH09358) mRNA, complete cds.
KIDNE2011532// similar to melanoma-associated chondroitin sulfate proteoglycan 4
NT2RI2012990// 76.5 KDA PROTEIN C210RF13.
NT2RI2025957// LU1 protein [Homo sapiens]
NT2RI3006284// Homo sapiens chorea-acanthocytosis (CHAC) mRNA, complete cds.
NT2RI3008697// erythroblast macrophage protein [Mus musculus]
NT2RP8000296// similar to Kelch proteins
NTONG2007517// RING CANAL PROTEIN (KELCH PROTEIN).
OCBBF2002124// p40 [Homo sapiens]
OCBBF2007610// PSD-95/SAP90-associated protein-4 [Rattus norvegicus].
OCBBF2021323// Mus musculus GTRGEO22 (Gtrgeo22) mRNA, complete cds.
OCBBF2028173// JM11 protein [Homo sapiens]
PEBLM2001465// diphthamide biosynthesis; Dph5p [Saccharomyces cerevisiae]
PERIC2004028// Mus musculus erythroblast macrophage protein EMP mRNA, complete cds.
PLACE7006051// cytoplasmic dynein heavy chain 2 [Rattus norvegicus]
PROST2008993// Mus musculus Pax transcription activation domain interacting protein PTIP mRNA, complete cds.
PUAEN2003079// nasopharyngeal carcinoma susceptibility protein [Homo sapiens]
SPLEN2002147// Halocynthia roretzi mRNA for HrPET-3, complete cds.
SPLEN2032154// NDRG1 PROTEIN (DIFFERENTIATION-RELATED GENE 1 PROTEIN) (DRG1) (REDUCING AGENTS AND TUNICAMYCIN-RESPONSIVE PROTEIN) (RTP) (NICKEL- SPECIFIC INDUCTION PROTEIN CAP43).
SYNOV2005216// Homo sapiens laryngeal carcinoma related protein 1 mRNA, complete cds.
SYNOV2007965// Homo sapiens mRNA for H-1(3)mbt-like protein, alternative variant a.
SYNOV4000706// B cell phosphoinositide 3-kinase adaptor [Mus musculus]
TBAES2004055// NY-REN-50 antigen
TESOP2001605// Homo sapiens laryngeal carcinoma related protein 1 mRNA, complete cds.
TESOP2005285// Homo sapiens partial mRNA for chr2 synaptotagmin (CHR2SYT gene).
TESTI2004215// Maackia amurensis early nodulin (ENOD2) mRNA, partial cds.
TESTI2009477// TRICHOHYALIN.
TESTI2034520// Rattus norvegicus SMC (segregation of mitotic chromosomes 1)-like 1 (yeast) (Smc1l1), mRNA
TESTI2052693// brk kinase substrate [Homo sapiens].
TESTI4006079// MUF1 protein; likely ortholog of mouse MUF1; elongin BC-interacting leucine-rich repeat protein [Homo sapiens]
TESTI4006393// neural specific sr protein NSSR 2 [Mus musculus]
TESTI4006546// colon cancer antigen NY-CO-45 [Homo sapiens].
TESTI4006802// mesothelin; megakaryocyte potentiating factor [Mus musculus]
TESTI4008018// DAZ associated protein 2; KIAA0058 gene product [Homo sapiens]
TESTI4009286// Homo sapiens HOTTL protein mRNA, complete cds
TESTI4009563// testis specific ankyrin-like protein 1 [Homo sapiens]
TESTI4010831// yeast Sec31p homolog; ABP125 [Homo sapiens]
TESTI4011484// Sec23-interacting protein p125 [Homo sapiens]
TESTI4014818// AD-012 protein [Homo sapiens]
TESTI4014924// selective hybridizing clone [Mus musculus]
TESTI4019140// Mi-2 histone deacetylase complex protein 66 [Xenopus laevis]
TESTI4019843// Rattus norvegicus huntingtin-associated protein interacting protein (duo) (Hapip), mRNA.
TESTI4023762// Trichohyalin.
TESTI4025920// B29 protein [Homo sapiens]
TESTI4039659// DnaJ homolog subfamily B member 8 (mDJ6).
TESTI4044186// leucine-rich, glioma inactivated 1 [Mus musculus]
THYMU2011736// latent transforming growth factor beta binding protein 3
THYMU2032825// Mus musculus mRNA for Drctnnbla, complete cds.
THYMU2038369// Mus musculus GTRGEO22 (Gtrgeo22) mRNA, complete cds.
THYMU3001991// ART-4 protein [Homo sapiens]
THYMU3006172// membrane bound C2 domain containing protein [Rattus norvegicus]
TLIVE2003225// CUB and Sushi multiple domains 1 [Homo sapiens]
TLIVE2004320// Homo sapiens PC2-glutamine-rich-associated protein (PCQAP) mRNA, complete cds.
TOVAR2002247// Homo sapiens partial partial mRNA for NICE-4 protein, clone 3114f17.
TRACH2023299// growth factor receptor bound protein 2-associated protein 2 [Mus musculus]
TRACH3000926// cardiac morphogenesis [Mus musculus]
TRACH3001427// p47 [Homo sapiens]
TRACH3006412// Homo sapiens COP9 constitutive photomorphogenic homolog subunit 7B
TRACH3034731// Ras association (RalGDS/AF-6) domain family 2
TUTER2002729// D6MM5E protein [Mus musculus]
UTERU1000031// G.gallus mRNA for tom-1B protein.
UTERU2006115// ALPHA-ADAPTIN A (CLATHRIN ASSEMBLY PROTEIN COMPLEX 2 ALPHA-A LARGE CHAIN) (100 KDA COATED VESICLE PROTEIN A) (PLASMA MEMBRANE ADAPTOR HA2/AP2 ADAPTIN ALPHA A SUBUNIT).
UTERU2031268// NY-REN-25 antigen [Homo sapiens].
UTERU2035452// NG3 [Homo sapiens]
UTERU3001059// ABC1 protein homolog, mitochondrial precursor.
UTERU3005585// rhophilin-like protein [Homo sapiens]
UTERU3009871// feminization 1 homolog a (C. elegans)
   Similarly, the 14 clones shown below are clones which were unassignable to any of the above-mentioned categories, but have been predicted to have some functions based on homology search using their full-length nucleotide sequences. Clone Name and Definition in the result of homology search, demarcated by a double slash mark (//), are shown below.
ADRGL2000042//Homo sapiens CTCL tumor antigen se20-4 mRNA, complete cds.
BRACE3009127//oxysterol binding protein 2; oxysterol binding protein-like 1 [Homo sapiens]
BRACE3021148//DC12 protein [Homo sapiens]
BRAMY2040159//Homo sapiens MRIP-1 mRNA, complete cds.
BRAWH3007441//CAT56 protein [Homo sapiens]
CTONG3001501//Mus musculus glucocorticoid-induced gene 1 mRNA, complete cds.
HCHON2000508//Homo sapiens prostate antigen PARIS-1 mRNA, complete cds.
OCBBF2020048// 95 kDa retinoblastoma protein binding protein; KIAA0661 gene product
PERIC2007068//Mus musculus mRNA for 1A13 protein.
TESTI4010382//cytoplasmic dynein heavy chain 2 [Rattus norvegicus]
TESTI4011072//tudor domain containing 1 [Mus musculus]
TESTI4046240//sirtuin 7
UTERU2019534//Golgi apparatus protein 1 [Homo sapiens]
UTERU2028734//Mus musculus slp2-a mRNA for synaptotagmin-like protein 2-a delta 2S-III, complete cds.

So far no information suggesting the function of the remaining 1,061 clones has been provided by the homology search. The functions of these clones may be clarified when an updated database becomes available in future. Clone names are shown below.
3NB692008729, ADRGL2012038, ADRGL2012179, ASTRO2003960, ASTRO2018373, ASTRO3000172, ASTRO3000482, BLADE2001371, BLADE2001987, BLADE2003474, BLADE2005459, BLADE2007666, BLADE2008281, BRACE1000258, BRACE1000533, BRACE1000572, BRACE2003639, BRACE2011747, BRACE2014475, BRACE2014657, BRACE2015058, BRACE2015314, BRACE2024627, BRACE2027970, BRACE2028970, BRACE2029849, BRACE2031154, BRACE2031389, BRACE2032044, BRACE2032329, BRACE2032538, BRACE2032823, BRACE2033720, BRACE2035441, BRACE2036096, BRACE2036830, BRACE2036834, BRACE2037847, BRACE2038114, BRACE2039475, BRACE2039734, BRACE2040325, BRACE2041009, BRACE2041264, BRACE2043349, BRACE2044816, BRACE2044949, BRACE2045428, BRACE2045947, BRACE2045954, BRACE2047011, BRACE2047350, BRACE2047385, BRACE3000071, BRACE3000787, BRACE3000973, BRACE3001217, BRACE3001595, BRACE3002390, BRACE3003698, BRACE3004358, BRACE3004435, BRACE3004783, BRACE3004880, BRACE3005145, BRACE3005225, BRACE3005499, BRACE3006185, BRACE3006226, BRACE3007322, BRACE3007472, BRACE3007480, BRACE3007559, BRACE3007642, BRACE3007767, BRACE3008036, BRACE3008092, BRACE3008772, BRACE3009090, BRACE3009237, BRACE3009377, BRACE3009701, BRACE3009724, BRACE3010428, BRACE3012364, BRACE3012930, BRACE3013119, BRACE3013576, BRACE3013780, BRACE3014231, BRACE3014317, BRACE3015121, BRACE3015894, BRACE3018308, BRACE3019055, BRACE3020194, BRACE3022769, BRACE3023912, BRACE3024659, BRACE3025153, BRACE3026075, BRACE3027242, BRACE3027478, BRACE3030103, BRACE3032983, BRALZ2011796, BRALZ2012183, BRALZ2012848, BRALZ2014484, BRAMY2003008, BRAMY2019985, BRAMY2020058, BRAMY2020270, BRAMY2028914, BRAMY2029602, BRAMY2030098, BRAMY2030702, BRAMY2030703, BRAMY2030799, BRAMY2031377, BRAMY2031442, BRAMY2032014, BRAMY2032242, BRAMY2032317, BRAMY2034185, BRAMY2034920, BRAMY2034993, BRAMY2036387, BRAMY2036699, BRAMY2036913, BRAMY2037823, BRAMY2038100, BRAMY2038846, BRAMY2040478, BRAMY2041261, BRAMY2041378, BRAMY2042612, BRAMY2042641, BRAMY2042760, BRAMY2042918, BRAMY2044078, BRAMY2044246, BRAMY2046478, BRAMY2046742, BRAMY2047169, BRAMY2047676, BRAMY2047884, BRAMY3000206, BRAMY3001401, BRAMY3002620, BRAMY3002805, BRAMY3004672, BRAMY3004900, BRAMY3006297, BRAMY3008650, BRAMY3009811, BRAMY3010411, BRASW1000053, BRAWH2001671, BRAWH2001973, BRAWH2010084, BRAWH2012326, BRAWH2013294, BRAWH2013871, BRAWH2014645, BRAWH2014662, BRAWH2014876, BRAWH3000345, BRAWH3002819, BRAWH3003522, BRAWH3003801, BRAWH3005422, BRAWH3005981, BRAWH3006792, BRAWH3007726, BRAWH3007783, BRAWH3008697, BRAWH3008931, BRAWH3009297, BRCAN2003987, BRCAN2004355, BRCAN2005436, BRCAN2006290, BRCAN2006297, BRCAN2006450, BRCAN2007144, BRCAN2007409, BRCAN2007426, BRCAN2008528, BRCAN2011254, BRCAN2011602, BRCAN2014881, BRCAN2015371, BRCAN2015464, BRCAN2016433, BRCAN2017717, BRCAN2017905, BRCAN2018935, BRCAN2019387, BRCAN2020710, BRCAN2021028, BRCAN2024563, BRCAN2025712, BRCOC2000670, BRCOC2014033, BRCOC2016525, BRCOC2020142, BRHIP2000826, BRHIP2000920, BRHIP2001074, BRHIP2002346, BRHIP2003242, BRHIP2003917, BRHIP2004312, BRHIP2005354, BRHIP2005600, BRHIP2005719, BRHIP2005932, BRHIP2006800, BRHIP2007741, BRHIP2009340, BRHIP2009474, BRHIP2014228, BRHIP2022221, BRHIP2024146, BRHIP2026061, BRHIP2029176, BRHIP3001283, BRHIP3006683, BRHIP3008183, BRHIP3008344, BRHIP3008565, BRHIP3008997, BRHIP3009099, BRHIP3011241, BRHIP3013765, BRHIP3013897, BRHIP3016213, BRHIP3018797, BRHIP3020182, BRHIP3024533, BRHIP3024725, BRHIP3025702, BRSSN2006892, BRSSN2008797, BRSSN2011262, BRSSN2014556, BRSSN2018581, BRSTN2001067, BRSTN2002400, BRSTN2005721, BRSTN2006865, BRSTN2007284, BRSTN2008052, BRSTN2008283, BRSTN2008457, BRSTN2009899, BRSTN2010500, BRSTN2010750, BRSTN2012320, BRSTN2017084, BRSTN2017237, BRSTN2018083, BRSTN2019129, BRTHA1000311, BRTHA2000855, BRTHA2001462, BRTHA2002115, BRTHA2002442, BRTHA2003030, BRTHA2003116, BRTHA2004821, BRTHA2004978, BRTHA2005956, BRTHA2006146, BRTHA2006194, BRTHA2007422, BRTHA2008316, BRTHA2008335, BRTHA2008535, BRTHA2008955, BRTHA2009846, BRTHA2009972, BRTHA2010073, BRTHA2010608, BRTHA2010884, BRTHA2010907, BRTHA2011351, BRTHA2011500, BRTHA2011641, BRTHA2012562, BRTHA2013262, BRTHA2013707, BRTHA2014828, BRTHA2015478, BRTHA2016543, BRTHA2017353, BRTHA2018165, BRTHA2018591, BRTHA2018707, BRTHA2019014, BRTHA2019022, BRTHA2019048, BRTHA3000273, BRTHA3000297, BRTHA3002401, BRTHA3003490, BRTHA3004475, BRTHA3005046, BRTHA3006856, BRTHA3007113, BRTHA3007319, BRTHA3007769, BRTHA3008143, BRTHA3010366, BRTHA3015910, BRTHA3018514, BRTHA3018617, BRTHA3019105, CERVX1000042, COLON1000030, COLON2003043, COLON2006282, COLON2009499, CORDB1000140, CORDB2000061, CTONG1000087, CTONG1000088, CTONG1000288, CTONG1000540, CTONG2001877, CTONG2019248, CTONG2019704, CTONG2019833, CTONG2020806, CTONG2021132, CTONG2022153, CTONG2024206, CTONG2025900, CTONG3000657, CTONG3000686, CTONG3000896, CTONG3001420, CTONG3002674, CTONG3003652, CTONG3003654, CTONG3003905, CTONG3003972, CTONG3004712, CTONG3005325, CTONG3005713, CTONG3005813, CTONG3006067, CTONG3006650, CTONG3007444, CTONG3007528, CTONG3007586, CTONG3007870, DFNES2011239, DFNES2011499, ERLTF2000324, FCBBF3001977, FCBBF3004847, FCBBF3006171, FCBBF3007242, FCBBF3008944, FCBBF3012288, FCBBF3013846, FCBBF3023443, FCBBF3025730, FCBBF3027717, FEBRA2000253, FEBRA2006396, FEBRA2007793, FEBRA2008287, FEBRA2008360, FEBRA2010719, FEBRA2014213, FEBRA2015588, FEBRA2020484, FEBRA2020582, FEBRA2020886, FEBRA2021339, FEBRA2021571, FEBRA2021908, FEBRA2021966, FEBRA2024136, FEBRA2024150, FEBRA2024343, FEBRA2027297, FEBRA2027352, FEBRA2028366, FEBRA2028477, FEBRA2028618, HCASM2002918, HCASM2003415, HCHON2000244, HCHON2000418, HCHON2006714, HCHON2007881, HEART1000088, HEART2001756, HEART2007031, HEART2010391, HHDPC2001337, HLUNG1000017, HLUNG2001996, HLUNG2002958, HLUNG2003003, HLUNG2012049, HLUNG2012287, HLUNG2012727, HLUNG2013304, HLUNG2013622, HLUNG2013851, HLUNG2014262, HLUNG2014449, HLUNG2017546, HLUNG2017806, HLUNG2019058, HSYRA2004858, HSYRA2005456, HSYRA2007667, HSYRA2008376, HSYRA2009075, IMR322002035, IMR322003675, IMR322007225, IMR322016146, IMR322018117, KIDNE2000665, KIDNE2000722, KIDNE2002991, KIDNE2003837, KIDNE2005543, KIDNE2011314, LYMPB1000141, MESAN2006563, MESAN2014295, MESAN2015515, MESAN2018576, MESTC2000153, NB9N41000340, NCRRP1000129, NESOP2000744, NESOP2001656, NESOP2001752, NESOP2002738, NHNPC2000877, NHNPC2001816, NHNPC2002565, NHNPC2002749, NOVAR2000710, NOVAR2000962, NOVAR2001783, NT2NE2005890, NT2NE2008060, NT2RI2003993, NT2RI2011683, NT2RI2012659, NT2RI2013357, NT2RI2014247, NT2RI2014551, NT2RI2014733, NT2RI2016128, NT2RI2018311, NT2RI2018883, NT2RI2019751, NT2RI2023303, NT2RI2027081, NT2RI2027396, NT2RI3000622, NT2RI3002303, NT2RI3002842, NT2RI3002892, NT2RI3003031, NT2RI3003162, NT2RI3004381, NT2RI3004510, NT2RI3005202, NT2RI3005724, NT2RI3006132, NT2RI3006376, NT2RI3006796, NT2RI3007158, NT2RI3007291, NT2RI3008055, NT2RI3008162, NT2RP7004123, NT2RP7005846, NT2RP7009867, NT2RP7010128, NT2RP7011570, NT2RP7015512, NT2RP7017365, NT2RP7017474, NT2RP7017546, NT2RP8000137, NTONG2003852, NTONG2005969, NTONG2006354, NTONG2007249, NTONG2008088, OCBBF1000254, OCBBF2001794, OCBBF2003819, OCBBF2004883, OCBBF2005428, OCBBF2007478, OCBBF2008770, OCBBF2009788, OCBBF2010416, OCBBF2019327, OCBBF2020343, OCBBF2020453, OCBBF2020639, OCBBF2020741, OCBBF2021286, OCBBF2022574, OCBBF2023162, OCBBF2023643, OCBBF2024781, OCBBF2024850, OCBBF2025028, OCBBF2025730, OCBBF2026645, OCBBF2027423, OCBBF2027478, OCBBF2028935, OCBBF2029901, OCBBF2030708, OCBBF2031366, OCBBF2032599, OCBBF2032611, OCBBF2032671, OCBBF2035110, OCBBF2035214, OCBBF2035564, OCBBF2035885, OCBBF2035916, OCBBF2036476, OCBBF2037398, OCBBF2037638, OCBBF3000296, OCBBF3002553, OCBBF3002600, OCBBF3006802, OCBBF3007516, OCBBF3008230, OCBBF3009279, PEBLM2001488, PEBLM2002749, PEBLM2004497, PEBLM2004666, PEBLM2005697, PEBLM2007112, PEBLM2007140, PERIC1000147, PERIC2000914, PERIC2001228, PERIC2002766, PERIC2003090, PERIC2003699, PERIC2003834, PERIC2004259, PERIC2004379, PERIC2004429, PERIC2005370, PLACE5000001, PLACE6001185, PLACE6009006, PLACE7001936, PLACE7008623, PROST2015243, PROST2017413, PROST2017700, PROST2018030, PROST2019296, PROST2019781, PUAEN2002616, PUAEN2005930, PUAEN2007785, SKMUS2003074, SKMUS2004047, SKNMC2004457, SKNMC2004643, SKNMC2005772, SKNMC2007961, SKNMC2009450, SKNSH2000482, SKNSH2009991, SKNSH2010015, SMINT2001818, SMINT2006641, SMINT2007391, SMINT2010897, SMINT2011311, SPLEN2002707, SPLEN2014946, SPLEN2016554, SPLEN2016863, SPLEN2017104, SPLEN2024127, SPLEN2027268, SPLEN2028844, SPLEN2028914, SPLEN2029051, SPLEN2029176, SPLEN2029727, SPLEN2029912, SPLEN2032321, SPLEN2033153, SPLEN2033539, SPLEN2033921, SPLEN2034081, SPLEN2034678, SPLEN2036103, SPLEN2036712, SPLEN2037580, SPLEN2037630, SPLEN2038055, SPLEN2039697, SPLEN2039936, SPLEN2041310, SPLEN2041645, SPLEN2041720, SPLEN2041977, SPLEN2042303, STOMA1000189, STOMA2003444, STOMA2004925, SYNOV1000374, SYNOV2005448, SYNOV2006430, SYNOV2017055, SYNOV2018921, SYNOV4000472, SYNOV4001326, SYNOV4001395, SYNOV4002346, SYNOV4002392, SYNOV4004823, SYNOV4007012, SYNOV4007215, T1ESE2000116, TBAES2001171, TBAES2001220, TBAES2001492, TBAES2001751, TBAES2002197, TBAES2003550, TBAES2005157, TBAES2005543, TBAES2006568, TBAES2007964, TCOLN2002278, TESOP1000127, TESOP2001818, TESOP2001849, TESOP2002451, TESOP2002489, TESOP2002950, TESOP2003273, TESOP2003753, TESOP2006060, TESOP2006068, TESOP2006670, TESOP2006746, TESOP2007688, TESOP2009555, TESTI1000330, TESTI1000390, TESTI1000491, TESTI2000443, TESTI2004700, TESTI2005376, TESTI2005986, TESTI2006041, TESTI2006643, TESTI2009474, TESTI2009511, TESTI2009812, TESTI2010400, TESTI2013381, TESTI2014843, TESTI2017727, TESTI2018838, TESTI2019042, TESTI2019648, TESTI2023254, TESTI2023599, TESTI2031529, TESTI2034749, TESTI2035107, TESTI2036513, TESTI2037643, TESTI2044833, TESTI2045920, TESTI2045983, TESTI2046347, TESTI2048465, TESTI2048603, TESTI2049206, TESTI2049246, TESTI2049277, TESTI2049422, TESTI2049452, TESTI2049469, TESTI2049576, TESTI2050681, TESTI2051279, TESTI2051488, TESTI2051543, TESTI2051806, TESTI2052211, TESTI2052698, TESTI2053242, TESTI2053526, TESTI4000068, TESTI4000250, TESTI4000462, TESTI4000530, TESTI4000970, TESTI4001201, TESTI4001206, TESTI4002290, TESTI4002647, TESTI4002703, TESTI4002878, TESTI4004200, TESTI4005628, TESTI4005857, TESTI4006112, TESTI4006219, TESTI4006326, TESTI4006412, TESTI4007203, TESTI4007373, TESTI4007404, TESTI4007489, TESTI4007775, TESTI4007799, TESTI4008007, TESTI4008219, TESTI4008573, TESTI4008816, TESTI4008935, TESTI4008993, TESTI4009022, TESTI4009034, TESTI4009123, TESTI4009215, TESTI4009283, TESTI4009406, TESTI4009638, TESTI4010211, TESTI4010377, TESTI4010713, TESTI4010789, TESTI4010817, TESTI4010928, TESTI4011118, TESTI4011161, TESTI4011246, TESTI4011505, TESTI4012086, TESTI4012329, TESTI4012556, TESTI4012702, TESTI4013675, TESTI4013685, TESTI4013735, TESTI4014159, TESTI4014306, TESTI4014445, TESTI4014694, TESTI4015263, TESTI4015293, TESTI4015471, TESTI4015600, TESTI4015646, TESTI4015681, TESTI4015688, TESTI4016551, TESTI4016812, TESTI4016882, TESTI4017001, TESTI4017137, TESTI4017254, TESTI4017848, TESTI4017961, TESTI4018382, TESTI4018555, TESTI4018806, TESTI4019299, TESTI4019417, TESTI4020806, TESTI4020920, TESTI4021294, TESTI4021456, TESTI4021491, TESTI4022936, TESTI4023555, TESTI4023942, TESTI4024344, TESTI4024907, TESTI4025731, TESTI4026079, TESTI4026192, TESTI4026295, TESTI4026700, TESTI4026762, TESTI4027516, TESTI4028612, TESTI4028809, TESTI4028983, TESTI4029370, TESTI4029671, TESTI4030603, TESTI4030669, TESTI4032895, TESTI4033433, TESTI4033690, TESTI4034212, TESTI4034432, TESTI4035602, TESTI4035637, TESTI4036042, TESTI4037066, TESTI4037188, TESTI4038156, TESTI4038223, TESTI4038258, TESTI4038339, TESTI4038492, TESTI4038818, TESTI4039038, TESTI4039086, TESTI4040800, TESTI4040939, TESTI4040956, TESTI4041099, TESTI4041143, TESTI4041519, TESTI4041624, TESTI4041903, TESTI4041954, TESTI4042098, TESTI4042444, TESTI4043129, TESTI4043203, TESTI4043551, TESTI4043947, TESTI4044035, TESTI4044084, TESTI4044123, TESTI4044234, TESTI4044296, TESTI4044682, TESTI4045312, TESTI4046253, TESTI4046282, TESTI4046884, TESTI4047069, THYMU1000600, THYMU2000932, THYMU2001090, THYMU2003397, THYMU2004693, THYMU2005190, THYMU2007179, THYMU2008282, THYMU2009134, THYMU2016204, THYMU2016523, THYMU2019587, THYMU2023967, THYMU2025707, THYMU2028978, THYMU2029688, THYMU2030226, THYMU2030264, THYMU2030637, THYMU2030796, THYMU2031218, THYMU2031341, THYMU2031368, THYMU2031579, THYMU2031847, THYMU2031890, THYMU2032080, THYMU2032358, THYMU2032696, THYMU2033070, THYMU2033787, THYMU2034647, THYMU2035101, THYMU2035388, THYMU2035400, THYMU2036058, THYMU2036252, THYMU2036265, THYMU2036653, THYMU2037081, THYMU2037208, THYMU2037965, THYMU2038189, THYMU2038615, THYMU2038636, THYMU2038739, THYMU2039350, THYMU2039411, THYMU2039989, THYMU2040140, THYMU2040824, THYMU2041007, THYMU2041252, THYMU3000133, THYMU3000655, THYMU3000826, THYMU3001472, THYMU3002452, THYMU3002661, THYMU3003763, THYMU3006132, THYMU3006371, THYMU3006485, THYMU3006963, THYMU3008171, THYMU3009255, TKIDN2003044, TKIDN2004386, TKIDN2005934, TKIDN2005947, TKIDN2007667, TKIDN2009641, TKIDN2010934, TKIDN2012824, TKIDN2013287, TKIDN2014757, TKIDN2015263, TKIDN2015788, TKIDN2016309, TLIVE2002338, TLIVE2003197, TLIVE2003970, TLIVE2004601, TLIVE2005180, TLIVE2006236, TLIVE2006529, TLIVE2007132, TLIVE2007528, TLIVE2007816, TLIVE2008083, TLIVE2009541, TOVAR2000649, TOVAR2001730, TOVAR2002549, TRACH2001443, TRACH2003070, TRACH2004170, TRACH2005066, TRACH2005811, TRACH2006049, TRACH2019248, TRACH2019473, TRACH2021398, TRACH2022042, TRACH2023306, TRACH2025344, TRACH2025749, TRACH2025932, TRACH3000342, TRACH3000586, TRACH3002192, TRACH3002866, TRACH3002871, TRACH3003379, TRACH3004068, TRACH3004537, TRACH3004840, TRACH3005479, TRACH3006038, TRACH3006228, TRACH3006470, TRACH3006889, TRACH3008093, TRACH3008535, TRACH3034762, TRACH3035235, TRACH3035482, TRACH3036207, TRACH3036309, TRACH3036456, TSTOM1000135, TSTOM2002672, TUTER1000122, TUTER2001387, UTERU1000024, UTERU1000148, UTERU1000249, UTERU2000649, UTERU2001409, UTERU2002410, UTERU2002841, UTERU2005004, UTERU2007520, UTERU2014678, UTERU2019681, UTERU2020491, UTERU2021163, UTERU2021380, UTERU2022981, UTERU2023039, UTERU2023175, UTERU2023651, UTERU2023712, UTERU2024002, UTERU2024656, UTERU2030213, UTERU2030280, UTERU2031084, UTERU2031521, UTERU2031703, UTERU2033375, UTERU2033382, UTERU2035114, UTERU2035331, UTERU2035503, UTERU2037361, UTERU2037577, UTERU2038251, UTERU3000226, UTERU3001542, UTERU3001766, UTERU3002218, UTERU3002667, UTERU3002731, UTERU3002768, UTERU3002993, UTERU3003465, UTERU3003523, UTERU3004523, UTERU3004616, UTERU3004709, UTERU3005049, UTERU3005205, UTERU3005230, UTERU3005460, UTERU3005970, UTERU3006008, UTERU3007134, UTERU3007913, UTERU3008660, UTERU3009259, UTERU3009517, UTERU3015086, UTERU3018081, UTERU3018154, UTERU3018616, UTERU3018711

Likewise, so far no information suggesting the function of the 72 clones shown below has been provided by the homology search. The functions of these clones may be clarified when an updated database becomes available in future. Clone names are shown below.
3NB692004724, ADRGL2000056, BLADE2000579, BRACE2037299, BRACE2043105, BRACE3003026, BRACE3003053, BRACE3005107, BRACE3015829, BRAMY2041434, BRAWH2009393, BRAWH2010552, BRCAN2014229, BRHIP2002722, BRHIP3000017, BRTHA2002133, BRTHA2002702, BRTHA2010033, BRTHA2013426, BRTHA3000296, CTONG2004000, CTONG2015596, CTONG2020374, CTONG2024031, CTONG3002552, CTONG3003598, CTONG3009287, HCASM2003357, HCHON2000743, HLUNG2016862, IMR322001879, IMR322007078, NT2RI3002557, NT2RI3005928, NT2RI3007167, NT2RP8000521, OCBBF2006987, OCBBF3003761, OCBBF3004972, PLACE7000333, PUAEN2006335, SKMUS2003194, SPLEN2033490, STOMA2004893, SYNOV2006620, SYNOV4005739, TESTI1000266, TESTI2008901, TESTI2035981, TESTI2037830, TESTI4001984, TESTI4008058, TESTI4011829, TESTI4013602, TESTI4015012, TESTI4025865, TESTI4028958, TESTI4029348, TESTI4029528, TESTI4029690, TESTI4036767, TESTI4038721, THYMU2032976, THYMU3000360, THYMU3001428, TRACH1000212, UTERU2004299, UTERU2035978, UTERU3000402, UTERU3014791, UTERU3015412, UTERU3017176

### EXAMPLE 7

### Functional categorization based on a functional domain search for deduced amino acid sequences

Domains and motifs are minimal functional structures of polypeptides. The structure of a polypeptide is constituted by a collection of such minimal structures, and thus the overall function of a polypeptide is ensured by the resulting structure. Thus, the overall function of a polypeptide can be predicted relatively accurately using data obtained by analysis of domain and motif structures. Furthermore, classifying these results into functional categories in a database allows clones comprising specific functions to be easily selected. Thus, such databases are highly useful in the functional analysis of each clone.

Pfam was used to undertake a domain search for the amino acid sequences deduced from the full-length nucleotide sequences (see Example 5). Based on these results, the proteins encoded by clones 664 and 250 were categorized and their functions predicted. This was performed by referring to domain and motif names, accession numbers for hit data, and detailed descriptions in Pfam (http://www.sanger.ac.uk/Software/Pfam/index.shtml) as well as functional categorizations in PROSITE (http://www.expasy.ch/cgi-bin/prosite-list.pl).

A clone predicted to belong to the category of secretory and/or membrane protein means a clone having domains and motifs, for example, seven-transmembrane receptor, pancreatic hormone peptides, ion transport protein, or fibroblast growth factor, which suggest receptor, ion channel, hormone, or growth factor.

A clone predicted to belong to the category of glycoprotein-related protein means a clone having domains and motifs, for example, immunoglobulin domain or glycosyl transferases group 1, which suggest involvement in glycobiology, such as glycoprotein or glycosyltransferase.

A clone predicted to belong to the category of signal transduction-related protein means a clone having domains and motifs, for example, eukaryotic protein kinase domain, protein phosphatase 2C, or Ras family, which suggest protein kinase, dephosphoenzyme, SH2 domain, or small G protein.

A clone predicted to belong to the category of transcription-related protein means a clone having domains and motifs, for example, bZIP transcription factor, Zinc finger, or C2H2 type, which suggest transcription factor or transcription-controlling protein.

A clone predicted to belong to the category of disease-related protein means a clone having domains and motifs, for example, Wilm's tumor protein or von Hippel-Lindau disease tumor suppressor protein, which suggest proteins with disease-specific expression or that promote or suppress expression, depending on the disease.

A clone predicted to belong to the category of enzyme and/or metabolism-related protein means a clone having domains and motifs, for example, aldehyde dehydrogenase family, chitin synthase, or glucose-6-phosphate dehydrogenase, which suggest transferase, synthase, or hydrolase.

A clone predicted to belong to the category of cell division- and/or cell proliferation-related protein means a clone having domains and motifs, for example, cyclin or cell division protein, which suggest cyclin or cell proliferation-controlling protein.

A clone predicted to belong to the category of cytoskeleton-related protein means a clone having domains and motifs, for example, actin, fibronectin type I domain, or kinesin motor domain, which suggest actin, kinesin, or fibronectin.

A clone predicted to belong to the category of nuclear protein and/or RNA synthesis-related protein means a clone having domains and motifs, for example, hepatitis C virus RNA dependent RNA polymerase or DEAD/DEAH box helicase, which suggest splicing factor, RNA synthase, or helicase.

A clone predicted to belong to the category of protein synthesis and/or transport-related protein means a clone having domains and motifs, for example, translation initiation factor SUI1, ubiquitin family, or ribosomal protein L16, which suggest translation-related protein, ubiquitin-related protein, or ribosomal protein.

A clone predicted to belong to the category of cellular defense-related protein means a clone having domains and motifs, for example, HSP90 protein or DNA mismatch repair protein, which suggest chaperonin or DNA repair protein.

A clone predicted to belong to the category of development- and/or differentiation-related proteins means a clone having domains and motifs, for example, floricaula / leafy protein, which suggest organogenesis-related protein.

A clone predicted to belong to the category of DNA- and/or RNA-binding protein means a clone having domains and motifs, for example, transcription factor WhiB, B-box zinc finger, or tRNA synthetases class I (C), which suggest DNA/RNA-relating enzyme group including transcription factor and DNA ligase or Zinc-finger related protein.

A clone predicted to belong to the category of ATP- and/or GTP-binding protein means a clone having domains and motifs, for example, E1-E2 ATPase or Ras family, which suggest ATP/GTP-related enzyme group including ATPase or G protein.

During this functional categorization, if a clone met every criterion of multiple categories as described above, it was grouped into multiple categories. However, the function of a polypeptide is not limited to these functional categories.

The clones predicted to belong to the category of secretory protein and/or membrane protein are the following 64 clones.
ASTRO2014923, ASTRO3000301, BRACE2005457, BRACE2014306, BRACE3001391, BRACE3014005, BRALZ2016085, BRAMY2040592, BRAWH2014662, BRHIP2004814, BRHIP3024118, BRTHA3002427, BRTHA3017848, BRTHA3018656, CTONG2009423, CTONG2013178, D3OST2002648, FEBRA2007708, FEBRA2008311, HCHON2001084, HCHON2001712, HCHON2004531, HCHON2005921, HSYRA2009102, KIDNE1000064, KIDNE2000832, NT2RI3006376, OCBBF2031167, OCBBF2035110, OCBBF2038317, PEBLM2002594, PERIC1000147, PERIC2009086, PROST1000184, SPLEN2012624, SPLEN2031547, SPLEN2033098, SPLEN2036326, TESTI1000257, TESTI1000390, TESTI2000644, TESTI2002036, TESTI2002928, TESTI2006648, TESTI2024567, TESTI2034520, TESTI4000014, TESTI4000724, TESTI4007163, TESTI4009881, TESTI4028880, THYMU2009425, THYMU2011548, THYMU2033079, THYMU2041015, TLIVE2000023, TLIVE2003381, TLIVE2007132, TRACH2006387, TRACH2007059, TRACH3004786, UTERU3000645, UTERU3004616, UTERU3006308

The following 23 clones are also predicted to belong to the category of secretory protein and/or membrane protein.
BRACE2029396, BRACE3005107, BRACE3010076, BRAMY2019111, BRAMY3004800, BRHIP3000017, FCBBF1000509, HCHON2000508, HEART2009680, IMR322013396, NT2RI2009583, NT2RI3000174, NT2RP8000521, OCBBF2030116, TESTI2029252, TESTI4013894, TESTI4032112, TESTI4041086, THYMU2035710, TKIDN2012771, TRACH3000420, UTERU2004299, TESTI4038779

The clones predicted to belong to the category of glycoprotein-related protein are the following 77 clones.
ADIPS2000088, BRACE2043142, BRACE2046295, BRACE3014005, BRAMY2005052, BRAMY4000277, BRAWH2007658, BRCAN2006063, BRSTN2004863, BRTHA3017589, BRTHA3017848, COLON2000568, COLON2004478, CTONG2000042, CTONG2013178, CTONG2024206, CTONG2024749, CTONG2025496, CTONG3001370, CTONG3003737, D3OST2002182, FEBRA2007708, HCHON2001084, HCHON2002676, HCHON2004531, HEART2001680, HLUNG2014262, LYMPB2000083, NESOP2001433, NOVAR2001108, NT2RI3006171, NT2RI3006340, NT2RI3007978, NT2RP7014005, OCBBF2010140, OCBBF2037598, PLACE5000171, PLACE6012574, PLACE7006051, PUAEN2009174, SMINT2002743, SMINT2010076, SMINT2011888, SMINT2015787, SPLEN2001599, SPLEN2015267, SPLEN2021701, SPLEN2037722, STOMA2004294, SYNOV3000231, SYNOV3000302, SYNOV4007521, SYNOV4007671, TBAES2003550, TESOP2005485, TESTI2005610, TESTI4006326, TESTI4021294, THYMU2005303, THYMU2019210, THYMU2023711, THYMU2027695, TRACH2007059, TRACH2022425, TRACH2022553, TRACH2022649, TRACH3002168, TRACH3005479, TRACH3005549, TRACH3006470, TRACH3035526, TRACH3036609, TSTOM2000442, UTERU2026090, UTERU3004616, UTERU3004992, UTERU3006308

The following eight clones are also predicted to belong to the category of glycoprotein-related protein.
BRAWH2006395, BRHIP3000017, NT2RI3007443, OCBBF3002654, TESTI2039060, TESTI4013894, TESTI4031745, TLIVE2001684

The clones predicted to belong to the category of signal transduction-related protein are the following 116 clones.
BLADE2007958, BNGH42007788, BRACE1000258, BRACE2008594, BRACE2041009, BRACE3001391, BRACE3006872, BRACE3011421, BRACE3024073, BRACE3027326, BRALZ2014484, BRAMY2001473, BRAMY2036567, BRAMY2042760, BRAMY2047751, BRAMY3001794, BRAMY3002803, BRAMY3005091, BRAMY3008466, BRAMY4000095, BRAWH3001326, BRAWH3002821, BRAWH3005912, BRCAN2002856, BRCAN2009432, BRCAN2016619, BRCAN2024451, BRCAN2028355, BRHIP2000819, BRHIP2005932, BRHIP3008405, BRHIP3025161, BRSSN2000684, BRSSN2004719, BRSTN2008418, BRSTN2013741, BRTHA3009037, BRTHA3013884, COLON2001721, CTONG2006798, CTONG3000084, CTONG3000657, CTONG3002127, D3OST3000169, DFNES2001108, DFNES2011499, FCBBF3007540, HCASM2001301, HCHON2000028, HCHON2006250, HHDPC1000118, HLUNG2001996, HLUNG2002465, KIDNE2001847, MESAN2006563, NHNPC2001816, NT2NE2003252, NT2RI2005166, NT2RI3000622, NT2RI3006673, NT2RP7005118, NT2RP7005529, NT2RP7009147, NT2RP7013795, NT2RP8000483, NTONG2003852, OCBBF2004826, OCBBF2004883, OCBBF2007028, OCBBF2008770, OCBBF2022351, OCBBF2037340, OCBBF2037547, PEBLM2004666, PLACE7008431, PROST2016462, PROST2018511, PUAEN2002616, PUAEN2005930, PUAEN2006328, PUAEN2009852, SYNOV2021320, TESOP2000801, TESOP2001166, TESTI2006648, TESTI2026505, TESTI2050137, TESTI2052693, TESTI4000079, TESTI4010713, TESTI4010831, TESTI4011956, TESTI4016882, TESTI4019843, TESTI4028059, THYMU2032014, THYMU2037226, THYMU2038615, THYMU3001234, THYMU3006172, THYMU3008436, TLIVE2009541, TRACH2009310, TRACH2021398, TRACH2023299, TRACH2025535, TRACH3009455, TRACH3034731, TSTOM2000553, UTERU1000337, UTERU2005621, UTERU2025025, UTERU2036089, UTERU2038251, UTERU3003523, UTERU3007419

The following 38 clones are also predicted to belong to the category of signal transduction-related protein.
BLADE2000579, BRACE3001058, BRACE3003053, BRACE3009127, BRAMY2040159, BRAMY3004800, BRAWH3009017, BRCAN2014229, BRHIP2026877, BRTHA2013610, CTONG3004550, FEBRA2001990, FEBRA2008692, HCHON2000508, MESAN2001770, NT2RI2005772, NT2RI3007443, NTONG2008093, OCBBF2005433, OCBBF2024284, OCBBF2034637, OCBBF3002654, TESOP2000390, TESTI2025924, TESTI2049956, TESTI4000319, TESTI4005317, TESTI4021482, TESTI4025268, TESTI4031745, THYMU2004139, THYMU2031249, TRACH2024408, UTERU2008040, UTERU2028734, UTERU3000402, UTERU3000738, UTERU3015412

The clones predicted to belong to the category of transcription-related protein are the following 27 clones.
BRACE2006319, BRACE3013576, BRAMY2030109, BRAWH3005912, BRHIP3025161, CORDB1000140, CTONG1000467, HEART2001756, IMR322000127, IMR322000917, KIDNE1000064, NOVAR2000136, NT2NE2006531, NT2RI3007158, NT2RP7000466, OCBBF2036743, OCBBF3009279, PLACE6019385, TESTI2026505, TESTI2044796, TESTI2050987, TESTI4017001, TESTI4019140, TESTI4034912, THYMU2035735, TRACH2025749, TRACH3004840

The following 88 clones are also predicted to belong to the category of transcription-related protein.
BRACE2003609, BRACE3001058, BRACE3001113, BRACE3003026, BRAMY2035070, BRAMY2035449, BRAMY2035718, BRAMY2039341, BRAMY2045471, BRAWH3007441, BRHIP2017553, BRSTN2013354, CERVX2002013, CTONG1000113, CTONG2003348, CTONG2020374, CTONG2020378, CTONG2020411, CTONG2024031, CTONG2028758, CTONG3001501, CTONG3004726, DFNES2011192, FCBBF3027854, FEBRA2014122, FEBRA2027609, HCASM2003018, HCASM2003099, HCHON2000508, HCHON2000743, HCHON2004858, HSYRA2005628, MESAN2014412, MESAN2015501, NT2RI2008952, NT2RI2018448, NT2RI3000174, NT2RI3001132, NT2RI3002557, NT2RI3007167, NT2RI3007443, OCBBF2008144, OCBBF2009583, OCBBF2011669, OCBBF2019684, OCBBF2020048, OCBBF2024284, OCBBF2032274, OCBBF3000167, OCBBF3003761, SPLEN2016135, SPLEN2016781, SPLEN2036702, SYNOV2021953, SYNOV4002744, TESOP2001796, TESOP2005199, TESOP2006398, TESTI2008901, TESTI2034251, TESTI2037830, TESTI4000183, TESTI4000214, TESTI4006473, TESTI4008058, TESTI4008302, TESTI4013365, TESTI4014801, TESTI4015442, TESTI4017714, TESTI4025494, TESTI4025547, TESTI4028938, TESTI4029348, TESTI4031745, TESTI4032090, THYMU2006001, THYMU2028739, THYMU2031139, THYMU3001428, TRACH2007483, TRACH3000134, TRACH3003832, TRACH3007866, UTERU3001053, UTERU3014791, UTERU3017176, TESTI4038779

The clones predicted to belong to the category of enzyme and/or metabolism-related protein are the following 176 clones.
3NB692002806, ASTRO1000009, BLADE2005036, BLADE2008539, BRACE2005457, BRACE2008594, BRACE2014475, BRACE2018762, BRACE2035381, BRACE2043142, BRACE2047011, BRACE3004058, BRACE3007625, BRACE3009708, BRACE3011421, BRACE3015262, BRACE3024073, BRACE3025630, BRACE3027478, BRAMY2047746, BRAMY2047751, BRAMY3002803, BRAMY3004919, BRAMY3005091, BRAMY4000095, BRAWH2010000, BRAWH2014414, BRAWH2014662, BRAWH2016702, BRAWH3002821, BRAWH3003727, BRCAN2021028, BRCAN2024451, BRCAN2028355, BRCOC2003213, BRHIP2004359, BRHIP2026288, BRHIP3008183, BRHIP3025161, BRHIP3027137, BRSSN2000684, BRSTN2000872, BRSTN2004863, BRSTN2004987, BRTHA2012980, BRTHA3002401, BRTHA3008778, BRTHA3009037, BRTHA3009090, BRTHA3015815, BRTHA3016917, BRTHA3017848, BRTHA3018656, COLON2001721, CTONG2004062, CTONG2006798, CTONG2013178, CTONG2028124, CTONG3002127, CTONG3005325, CTONG3005648, D3OST2002182, FCBBF3004502, FCBBF3013307, FEBRA2007708, FEBRA2008468, FEBRA2026984, HCASM2001301, HCASM2002918, HCHON2002676, HCHON2004007, HCHON2004531, HEART2006131, HHDPC1000118, HLUNG1000017, KIDNE2000832, KIDNE2006580, MESAN2012054, NOVAR2000136, NT2NE2003252, NT2NE2006909, NT2RI2004618, NT2RI3004510, NT2RI3006673, NT2RI3007978, NT2RI3008652, NT2RP7010599, NT2RP7014005, NT2RP7017474, NTONG2000413, OCBBF2004826, OCBBF2006058, OCBBF2019823, OCBBF2025527, OCBBF2031167, OCBBF2037340, OCBBF2037547, OCBBF2037638, PERIC2009086, PLACE7002641, PLACE7008431, PROST2017367, PUAEN2007044, PUAEN2009795, PUAEN2009852, SPLEN2010912, SPLEN2015679, SPLEN2030335, SYNOV4002392, SYNOV4002883, TBAES2003550, TESOP2000801, TESOP2004114, TESOP2009121, TESTI1000257, TESTI1000545, TESTI2002618, TESTI2006648, TESTI2040018, TESTI2049469, TESTI2053621, TESTI4000288, TESTI4000349, TESTI4001148, TESTI4001527, TESTI4001561, TESTI4002552, TESTI4006819, TESTI4007382, TESTI4007810, TESTI4008429, TESTI4010713, TESTI4010851, TESTI4012448, TESTI4012679, TESTI4013369, TESTI4016925, TESTI4018835, TESTI4020920, TESTI4021478, TESTI4022716, TESTI4026510, TESTI4028059, TESTI4029836, TESTI4032895, TESTI4034432, TESTI4036909, THYMU2006420, THYMU3000133, THYMU3001379, THYMU3004835, THYMU3006172, THYMU3008436, TLIVE2002336, TRACH2006387, TRACH2009310, TRACH2019473, TRACH2022425, TRACH2023299, TRACH3005479, TRACH3006470, TRACH3007479, TRACH3008093, TRACH3008629, TRACH3036193, TSTOM2000553, UTERU2005621, UTERU2017762, UTERU2025025, UTERU2033375, UTERU3000828, UTERU3001240, UTERU3001585, UTERU3003116, UTERU3005460, UTERU3005907

The following 89 clones are also predicted to belong to the category of enzyme and/or metabolism-related protein.
BLADE2000579, BRACE2039823, BRACE3003053, BRAMY2038516, BRAMY2040159, BRAWH1000369, BRCAN2003070, BRCAN2014229, BRCOC2019841, BRHIP2005724, BRHIP2008389, BRHIP2026877, BRHIP3000240, BRHIP3026052, BRTHA2002133, BRTHA2002702, BRTHA2007060, BRTHA2010033, BRTHA2013426, BRTHA2013610, BRTHA2017364, BRTHA2018011, BRTHA3000296, CTONG2004000, CTONG2016942, CTONG2020374, CTONG2024031, CTONG3002552, CTONG3003598, CTONG3004550, FCBBF1000509, FEBRA2008692, HCASM2002754, HCASM2003099, HCASM2003357, HLUNG2015418, HLUNG2015548, IMR322013731, MESAN2005303, NT2RI2005772, NT2RI2008952, NT2RI3000174, NT2RI3007443, NT2RP7008435, NTONG2008093, OCBBF2006987, OCBBF2034637, OCBBF3002654, PLACE7000333, PLACE7000502, PROST2000452, SPLEN2039311, STOMA2003158, SYNOV2013637, TESOP2000390, TESTI2015626, TESTI2025924, TESTI2026647, TESTI2035981, TESTI2036288, TESTI2039060, TESTI2049956, TESTI4000155, TESTI4001984, TESTI4006473, TESTI4010382, TESTI4011072, TESTI4014801, TESTI4017714, TESTI4021482, TESTI4025547, TESTI4025865, TESTI4026207, TESTI4028958, TESTI4029690, TESTI4031745, TESTI4032090, THYMU2004139, THYMU2031139, THYMU2031249, THYMU2040925, TKIDN2012771, TLIVE2002046, TLIVE2007607, TRACH3000420, TRACH3007866, UTERU2019534, UTERU2028734, UTERU3000738

The clones predicted to belong to the category of cell division and/or cell proliferation-related protein are the following ten clones.
BRAWH2001940, CTONG3001123, HCHON2001217, PROST2008993, TBAES2001171, TESTI4021294, TESTI4035498, UTERU1000024, UTERU3002993, UTERU3003523

The following three clones are also predicted to belong to the category of cell division and/or cell proliferation-related protein.
BRACE2029396, BRAWH2010552, TESTI4013365

The clones predicted to belong to the category of cytoskeleton-related protein are the following 36 clones.
BRACE2026836, BRACE2045300, BRAWH3000314, BRSTN2004863, BRTHA2004978, BRTHA3003449, BRTHA3005046, COLON2002520, CORDB2000541, CTONG3002674, FCBBF3012288, HCHON2001577, HLUNG2017350, HSYRA2005456, HSYRA2009075, NT2RI3006340, NT2RI3006673, NT2RI3007291, OCBBF2037598, PLACE5000282, TESTI2003347, TESTI2034767, TESTI4000288, TESTI4007778, TESTI4009160, TESTI4018886, TESTI4030603, TESTI4034632, TESTI4035063, THYMU1000496, THYMU2008725, TRACH2005811, TRACH2007059, UTERU2007724, UTERU2035745, UTERU3004616

The following four clones are also predicted to belong to the category of cytoskeleton-related protein.
NT2RI2005772, OCBBF2006987, SPLEN2030847, TESTI4026207

The clones predicted to belong to the category of nuclear protein and/or RNA synthesis-related protein are the following 20 clones.
BRACE3024073, BRAWH2001940, BRCOC2003213, BRSTN2004987, BRTHA3016917, CTONG3009028, FCBBF3013307, FEBRA2026984, SPLEN2010912, TBAES2001171, TESTI2040018, TESTI4019566, TESTI4022716, TESTI4026510, TESTI4036909, THYMU3000133, TRACH2023299, TRACH3036193, UTERU1000024, UTERU3002993

The following eleven clones are also predicted to belong to the category of nuclear protein and/or RNA synthesis-related protein.
BRACE3003053, BRCAN2002473, BRTHA2017364, NT2RI2008952, NT2RI3000174, TESTI2026647, TESTI2035981, TESTI4000155, TESTI4006473, TESTI4010382, TESTI4025547

The clones predicted to belong to the category of protein synthesis and/or transport-related protein are the following 29 clones.
BRACE2014306, BRACE3008720, BRAWH3000491, BRCAN2009432, BRHIP2000920, BRTHA3013884, CTONG2013178, HCHON2004531, HLUNG1000017, HLUNG2013851, HSYRA2005496, NT2NE2006909, NT2RI3006340, OCBBF2007068, OCBBF2031167, PUAEN2009795, TBAES2001229, TBAES2004055, TESTI2051867, TESTI4000014, TESTI4000349, TESTI4009608, TESTI4010851, TESTI4034632, TRACH3007479, TRACH3036193, UTERU2017762, UTERU2019940, UTERU2033375

The following 17 clones are also predicted to belong to the category of protein synthesis and/or transport-related protein.
BLADE2000579, BRACE3003053, BRCAN2003070, BRTHA2018011, BRTHA3000296, CTONG2016942, MESAN2005303, NT2RI3002557, NT2RP7008435, PERIC2007068, PLACE7000502, PROST2000452, TESTI4001984, TESTI4017714, THYMU2004284, TRACH3000420, TRACH3007866

The clones predicted to belong to the category of cellular defense-related protein are the following four clones.
BRTHA2015878, CTONG3000084, NT2RI3002842, PEBLM2004666

The following three clones are also predicted to belong to the category of cellular defense-related protein.
BRCAN2002473, NT2RI3007167, TRACH3002561

The clone predicted to belong to the category of development and/or differentiation-related protein is the following one clone.
TESTI4014924

The clones predicted to belong to the category of DNA-binding and/or RNA-binding protein are the following 67 clones.
BRACE2006319, BRACE2047011, BRACE3004150, BRACE3013576, BRACE3024073, BRAMY2030109, BRAWH3005912, BRCAN2002562, BRCOC2003213, BRHIP2021615, BRHIP3008183, BRHIP3025161, BRSTN2004987, BRTHA2018707, BRTHA3016917, CORDB1000140, CTONG1000467, CTONG3000084, CTONG3003972, CTONG3008831, CTONG3009028, FCBBF3013307, FEBRA2026984, HEART2001756, HLUNG2013851, IMR322000127, IMR322000917, KIDNE1000064, NT2NE2006531, NT2RI3003382, NT2RI3007158, NT2RP7000466, NT2RP7004123, OCBBF2036743, OCBBF3009279, PLACE6019385, SPLEN2006122, SPLEN2010912, TESOP2009121, TESTI1000390, TESTI2014716, TESTI2026505, TESTI2040018, TESTI2044796, TESTI2050987, TESTI4007810, TESTI4009374, TESTI4011745, TESTI4012679, TESTI4017001, TESTI4019140, TESTI4019566, TESTI4022716, TESTI4026510, TESTI4034432, TESTI4034912, TESTI4036909, THYMU2035319, THYMU2035735, THYMU3000133, TLIVE2002336, TRACH2023299, TRACH2025749, TRACH3004840, TRACH3036193, UTERU2026025, UTERU3009490

The following 112 clones are also predicted to belong to the category of DNA-binding and/or RNA-binding protein.
BLADE2006830, BRACE2003609, BRACE3001058, BRACE3001113, BRACE3003026, BRACE3003053, BRACE3010076, BRAMY2035070, BRAMY2035449, BRAMY2039341, BRAMY2045471, BRAWH1000369, BRAWH3007441, BRHIP2017553, BRSTN2013354, BRTHA2002133, BRTHA2002702, BRTHA2017364, BRTHA2017972, CERVX2002013, CTONG1000113, CTONG2003348, CTONG2015596, CTONG2020374, CTONG2020378, CTONG2020411, CTONG2024031, CTONG2028758, CTONG3001501, CTONG3004726, DFNES2011192, FCBBF1000509, FCBBF3027854, FEBRA2014122, FEBRA2027609, HCASM2003018, HCASM2003099, HCASM2009424, HCHON2000508, HCHON2000743, HCHON2004858, HSYRA2005628, IMR322013731, MESAN2014412, MESAN2015501, NT2RI2008952, NT2RI2018448, NT2RI2027157, NT2RI3000174, NT2RI3001132, NT2RI3002557, NT2RI3007167, NT2RI3007443, OCBBF2006987, OCBBF2008144, OCBBF2009583, OCBBF2011669, OCBBF2019684, OCBBF2020048, OCBBF2024284, OCBBF2032274, OCBBF2034637, OCBBF3000167, OCBBF3003761, PERIC2007068, SPLEN2016135, SPLEN2016781, SPLEN2036702, STOMA2003158, SYNOV2021953, SYNOV4002744, TESOP2001796, TESOP2005199, TESOP2006398, TESTI2008901, TESTI2026647, TESTI2034251, TESTI2035981, TESTI2037830, TESTI4000155, TESTI4000183, TESTI4000214, TESTI4006473, TESTI4008058, TESTI4008302, TESTI4010382, TESTI4013365, TESTI4014801, TESTI4015442, TESTI4017714, TESTI4025494, TESTI4025547, TESTI4026207, TESTI4028938, TESTI4028958, TESTI4029348, TESTI4031745, TESTI4032090, THYMU2006001, THYMU2028739, THYMU2031139, THYMU3001428, TKIDN2012771, TLIVE2007607, TRACH2007483, TRACH3000134, TRACH3003832, TRACH3007866, UTERU3001053, UTERU3014791, UTERU3017176, TESTI4038779

The clones predicted to belong to the category of ATP binding and/or GTP-binding protein are the following 28 clones.
BRACE3008720, BRACE3009708, BRAMY2047746, BRAMY3004919, BRAWH2014662, BRAWH2016702, BRCAN2009432, BRCAN2024451, BRSTN2013741, BRTHA3008778, BRTHA3009090, CTONG2004062, CTONG2028124, HCHON2004007, OCBBF2037340, SPLEN2030335, TESTI4000288, TESTI4001148, TESTI4002552, TESTI4008429, TESTI4018835, TESTI4021478, TESTI4029836, THYMU2036459, THYMU3001379, TRACH2001549, UTERU3000828, UTERU3001240

The following eight clones are also predicted to belong to the category of ATP binding and/or GTP-binding protein.
BRCAN2014229, BRHIP2008389, CTONG3004550, FEBRA2001990, IMR322013396, IMR322013731, MESAN2001770, TESTI4000319

Although the 208 clones described below have hit data in Pfam, it remains unclear as to which of the above-described categories each of these clones belong. However, if data for proteins having a similar domain or motif are accumulated and their functions clarified in more detail, in the future these clones can be classified into any of the above-described categories. The Clone Name and Functional Domain Name are indicated as "Clone Name//Functional Domain Name". When a clone had hit data in multiple functional domains, all data were represented, with each marked with a double slash (//).

In addition, even when a clone had multiple hit data in an identical functional domain, these data are fully represented without abridgment.
3NB692002685 //R3H domain
3NB692008729 //Hr1 repeat motif
ASTRO2003960 //F-box domain.
BNGH42003570 //EB module// Furin-like cysteine rich region// Thrombospondin type 1 domain
BRACE2010489 //LysM domain
BRACE2015314 //Bacterial mutT protein
BRACE2016981 //Fanconi anaemia group C protein// Bacterial flagellin N-terminus
BRACE2027258 //Ank repeat// Ank repeat// Ank repeat// Ank repeat// Ank repeat
BRACE2030341 //Kinase associated domain 1
BRACE2035441 //Spectrin repeat// Spectrin repeat// Spectrin repeat
BRACE2038329 //TS-N domain// UBA domain
BRACE2042550 //Thrombospondin type 1 domain// Trypsin Inhibitor like cysteine rich domain// von Willebrand factor type C domain// Thrombospondin type 1 domain
BRACE2044286 //CRAL/TRIO domain.// Spectrin repeat
BRACE3000071 //Ank repeat// Ank repeat// Ank repeat
BRACE3000973 //Leucine Rich Repeat
BRACE3001002 //Lipoprotein
BRACE3003192 //EGF-like domain// EGF-like domain// EGF-like domain// EGF-like domain// Metallothionein// Keratin, high sulfur B2 protein// EGF-like domain// EGF-like domain// EGF-like domain// EGF-like domain// TB domain// EGF-like domain// EGF-like domain// EGF-like domain// TB domain// EGF-like domain// EGF-like domain
BRACE3004772 //SAM domain (Sterile alpha motif)
BRACE3004880 //GLTT repeat (12 copies)// GLTT repeat (12 copies)// GLTT repeat (12 copies)// Keratin, high sulfur B2 protein
BRACE3008137 //PDZ domain (Also known as DHR or GLGF).// PDZ domain (Also known as DHR or GLGF).// PDZ domain (Also known as DHR or GLGF).// PDZ domain (Also known as DHR or GLGF).
BRACE3008384 //Rhomboid family
BRACE3009090 //Beige/BEACH domain
BRACE3010397 //SCP-like extracellular protein
BRACE3015521 //EF hand
BRACE3016884 //Keratin, high sulfur B2 protein// Flagellar L-ring protein
BRACE3019084 //SAM domain (Sterile alpha motif)
BRAMY2004771 //Leucine Rich Repeat// Leucine rich repeat C-terminal domain// Leucine rich repeat N-terminal domain
BRAMY2019300 //Leucine Rich Repeat// Leucine rich repeat C-terminal domain// Leucine rich repeat N-terminal domain BRAMY2021498 //Thrombospondin type 1 domain// DnaJ central domain (4 repeats)// Thrombospondin type 1 domain// Thrombospondin type 1 domain// Thrombospondin type 1 domain// Thrombospondin type 1 domain
BRAMY2031317 //PDZ domain (Also known as DHR or GLGF).
BRAMY2039872 //Interferon alpha/beta domain
BRAMY2046989 //TPR Domain// TPR Domain// TPR Domain// TPR Domain// TPR Domain// TPR Domain
BRAMY3004224 //Leucine rich repeat N-terminal domain// Leucine Rich Repeat// Leucine Rich Repeat// Leucine Rich Repeat// Leucine Rich Repeat// Leucine Rich Repeat// Leucine rich repeat C-terminal domain
BRAMY3005932 //Ank repeat
BRAWH1000127 //Plexin repeat// Thrombospondin type 1 domain
BRAWH2001395 //Myelin basic protein
BRAWH2014954 //PDZ domain (Also known as DHR or GLGF).// PDZ domain (Also known as DHR or GLGF).
BRAWH3000078 //Adaptin N terminal region// Activin types I and II receptor domain
BRAWH3001891 //YCF9
BRAWH3002574 //Calpain large subunit, domain III// EF hand
BRAWH3002600 //Cadherin domain// Cadherin domain// Cadherin domain
BRAWH3008341 //Pentaxin family
BRCAN2002948 //Adaptin N terminal region
BRCAN2009203 //SAM domain (Sterile alpha motif)
BRCAN2015464 //Gag P30 core shell protein
BRCAN2017717 //Squash family of serine protease inhibitors
BRCOC2001505 //Myelin basic protein
BRCOC2016525 //Ank repeat// Ank repeat// Ank repeat// Ank repeat// Ank repeat// Ank repeat// Ank repeat
BRHIP2003786 //Ank repeat// Ank repeat// Ank repeat// BTB/POZ domain
BRHIP2005236 //Galactose binding lectin domain// Latrophilin Cytoplasmic C-terminal region
BRHIP2007616 //Sema domain
BRHIP2009414 //Uncharacterized protein family
BRHIP3000339 //Myelin basic protein
BRHIP3008313 //Ank repeat
BRSTN2001067 //Rifin/stevor family
BRTHA2000855 //Extracellular link domain
BRTHA2005579 //von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain// von Willebrand factor type C domain
BRTHA2007122 //Ank repeat// Ank repeat// Ank repeat// Ank repeat// SAM domain (Sterile alpha motif)
BRTHA2008527 //Leucine Rich Repeat// Leucine Rich Repeat// Leucine Rich Repeat// Leucine Rich Repeat// Leucine Rich Repeat
BRTHA2009311 //Vertebrate galactoside-binding lectins
BRTHA2010884 //Thrombospondin type 1 domain// CUB domain
BRTHA2013262 //Keratin, high sulfur B2 protein
BRTHA2014792 //SET domain
BRTHA2015406 //UBA domain
BRTHA2016496 //Peptidase C13 family
BRTHA2018591 //GTPase of unknown function
BRTHA2018624 //Galactose binding lectin domain// Activin types I and II receptor domain// Galactose binding lectin domain
BRTHA2019048 //Domain of unknown function DUF71
BRTHA3003074 //Fanconi anaemia group C protein
BRTHA3008310 //Homeobox domain
CTONG1000341 //EGF-like domain// EGF-like domain// Metallothionein// EGF-like domain// EB module// EGF-like domain// EGF-like domain// EGF-like domain
CTONG2001877 //MutT-like domain
CTONG2008233 //DnaJ domain
CTONG2017500 //F-box domain.
CTONG2020026 //Herpesvirus VP23 like capsid protein
CTONG2028687 //TPR Domain// TPR Domain
CTONG3000686 //TPR Domain// TPR Domain// TPR Domain// TPR Domain CTONG3004072 //Beta type Zein// Keratin, high sulfur B2 protein
CTONG3006067 //DnaJ central domain (4 repeats)
CTONG3006186 //PDZ domain (Also known as DHR or GLGF)// Apolipoprotein A1/A4/E family// WW domain
CTONG3009385 //TPR Domain// TPR Domain// TPR Domain// TPR Domain// TPR Domain// TPR Domain// TPR Domain// TPR Domain// TPR Domain// TPR Domain
DFNES2000146 //Plexin repeat// Thrombospondin type 1 domain
DFNES2005266 //Thrombospondin type 1 domain
FCBBF3009888 //Keratin, high sulfur B2 protein// u-PAR/Ly-6 domain
FCBBF3012170 //Thrombospondin type 1 domain
FEBRA2000253 //Flagellar L-ring protein
FEBRA2007801 //IBR domain
FEBRA2021571 //von Willebrand factor type D domain
FEBRA2024150 //DENN (AEX-3) domain
HCHON2004776 //Protein of unknown function DUF93
HEART1000139 //Troponin
HEART2006909 //CBS domain// CBS domain
HEART2010495 //Tau and MAP proteins, tubulin-binding
HLUNG2000014 //Lectin C-type domain
HLUNG2002958 //EF hand
HLUNG2011298 //Oxidoreductase FAD/NAD-binding domain
IMR322006495 //Tropomyosins
KIDNE2000846 //Sodium:neurotransmitter symporter family
KIDNE2001361 //Domain of unknown function DUF19
KIDNE2011635 //Sodium:solute symporter family
KIDNE2012945 //CUB domain// Pentaxin family
NESOP2001656 //Polyomavirus coat protein
NT2RI2008724 //GGL domain
NT2RI2025909 //Mitochondrial carrier proteins// Mitochondrial carrier proteins// Mitochondrial carrier proteins
NT2RI2025957 //PDZ domain (Also known as DHR or GLGF).
NT2RI3007543 //DnaJ domain
NT2RP7000359 //FERM domain (Band 4.1 family)// Insulin-like growth factor binding proteins// PDZ domain (Also known as DHR or GLGF).
NT2RP7004027 //CUB domain// Sushi domain (SCR repeat)
NT2RP7011570 //Gag P30 core shell protein
NT2RP8000296 //BTB/POZ domain// Kelch motif// Kelch motif// Kelch motif// Kelch motif// Kelch motif// Kelch motif
NTONG2005277 //Ank repeat// Ank repeat// Ank repeat// Ank repeat// Ank repeat// Ank repeat// Ank repeat// Ank repeat
NTONG2006354 //Ank repeat
NTONG2007517 //BTB/POZ domain
OCBBF2006764 //Sushi domain (SCR repeat)// CUB domain// Sushi domain (SCR repeat)// CUB domain// Sushi domain (SCR repeat)
OCBBF2010416 //Major intrinsic protein
OCBBF2020838 //Fork head domain
OCBBF2021323 //Regulatory subunit of type II PKA R-subunit
OCBBF2033869 //CUB domain
PERIC2001228 //Leucine Rich Repeat// Leucine Rich Repeat// Leucine Rich Repeat// Leucine Rich Repeat// Leucine Rich Repeat// Leucine Rich Repeat// Leucine Rich Repeat
PERIC2003720 //Ezrin/radixin/moesin family
PLACE6020031 //Ank repeat// Ank repeat
PLACE7000514 //Filamin/ABP280 repeat.
PROST2018090 //Sushi domain (SCR repeat)// Sushi domain (SCR repeat)// Chitin binding Peritrophin-A domain// HYR domain// Sushi domain (SCR repeat)
RECTM2000433 //Jacalin-like lectin domain
SKMUS2006394 //Ank repeat// Ank repeat// Ank repeat// Ank repeat// Ank repeat// Ank repeat// Ank repeat
SMINT1000192 //Small hydrophilic plant seed proteins
SPLEN2002147 //Phosphatidylinositol transfer protein
SPLEN2002467 //DB module// F-box domain.// Leucine Rich Repeat
SPLEN2031780 //Domain of unknown function DUF139// Domain of unknown function DUF139
SPLEN2034081 //Insulin-like growth factor binding proteins
SPLEN2036821 //Mitochondrial carrier proteins
SYNOV2005448 //Apidaecin
SYNOV2005817 //Domain of unknown function DUF19// Tissue factor
SYNOV2006430 //Nitrogen regulatory protein P-II
SYNOV2014400 //EGF-like domain// Granulins// Granulins// EGF-like domain
SYNOV4007553 //Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine rich repeat C-terminal domain// TIR domain
SYNOV4008440 //Adaptin N terminal region
TESOP2001953 //Leucine Rich Repeat// Leucine Rich Repeat// Leucine Rich Repeat// Leucine Rich Repeat// Leucine Rich Repeat// Leucine Rich Repeat// Leucine Rich Repeat TESTI2000443 //Leucine Rich Repeat// Leucine Rich Repeat// Leucine Rich Repeat
TESTI2004700 //Leucine Rich Repeat// Leucine Rich Repeat// Leucine Rich Repeat// Leucine Rich Repeat
TESTI2027019 //Leucine Rich Repeat
TESTI4000462 //Keratin, high sulfur B2 protein
TESTI4000970 //Ezrin/radixin/moesin family
TESTI4002491 //NSF attachment protein
TESTI4006546 //Tudor domain// Tudor domain// Tudor domain
TESTI4007064 //DENN (AEX-3) domain// PPR repeat// LIM domain containing proteins
TESTI4011484 //SAM domain (Sterile alpha motif)
TESTI4012406 //Kringle domain
TESTI4015471 //Tropomyosins
TESTI4016110 //DnaJ domain
TESTI4017137 //Keratin, high sulfur B2 protein
TESTI4017575 //MSP (Major sperm protein) domain
TESTI4018152 //FERM domain (Band 4.1 family)
TESTI4018555 //Granulins
TESTI4020092 //Laminin G domain
TESTI4023555 //Lectin C-type domain
TESTI4025920 //Adaptin N terminal region
TESTI4026192 //Domain of unknown function
TESTI4027557 //Vertebrate galactoside-binding lectins// Vertebrate galactoside-binding lectins
TESTI4028429 //WAP-type (Whey Acidic Protein) 'four-disulfide core'
TESTI4028612 //Major intrinsic protein
TESTI4028983 //Serum amyloid A protein
TESTI4030505 //Metallothionein family 5
TESTI4038492 //Serum amyloid A protein
TESTI4039659 //DnaJ domain
TESTI4041053 //Ank repeat// Ank repeat// Ank repeat// Ank repeat// Armadillo/beta-catenin-like repeats// Armadillo/beta-catenin-like repeats// Armadillo/beta-catenin-like repeats// Armadillo/beta-catenin-like repeats// Armadillo/beta-catenin-like repeats// Armadillo/beta-catenin-like repeats
TESTI4044084 //Domain of unknown function
TESTI4046487 //Hantavirus nucleocapsid protein
TESTI4046819 //Metallothionein// PTS HPr component phosphorylation sites
THYMU2004693 //CX module
THYMU2011736 //EGF-like domain// EGF-like domain// EB module// EGF-like domain// TB domain// EGF-like domain// EGF-like domain
THYMU2016204 //Metallothionein
THYMU2027734 //Parvovirus coat protein VP2
THYMU2038369 //Regulatory subunit of type II PKA R-subunit
THYMU2038797 //Lectin C-type domain
THYMU3000028 //Zona pellucida-like domain
THYMU3003212 //Cytidine and deoxycytidylate deaminase zinc-binding region
THYMU3003763 //Leucine rich repeat N-terminal domain// Polyomavirus coat protein
THYMU3007137 //PDZ domain (Also known as DHR or GLGF).// PDZ domain (Also known as DHR or GLGF).
THYMU3008171 //TPR Domain
TLIVE2002338 //Transforming growth factor beta like domain
TLIVE2002690 //von Willebrand factor type D domain
TLIVE2003225 //CUB domain// Sushi domain (SCR repeat)// CUB domain// Sushi domain (SCR repeat)
TLIVE2008229 //TPR Domain// TPR Domain
TRACH2001443 //TIR domain
TRACH3001427 //UBX domain
TRACH3003379 //Protein phosphatase 2A regulatory B subunit
TRACH3008713 //NSF attachment protein
TRACH3035235 //S-100/ICaBP type calcium binding domain
TUTER2000425 //KRAB box
UTERU1000031 //ENTH domain// VHS domain
UTERU2006115 //Adaptin N terminal region
UTERU2006568 //IBR domain
UTERU2019706 //TCP-1/cpn60 chaperonin family// TCP-1/cpn60 chaperonin family
UTERU2035328 //WW domain// WW domain// WW domain// FF domain// FF domain// FF domain
UTERU2035331 //Fibrillar collagen C-terminal domain
UTERU2035452 //EGF-like domain// Metallothionein// EGF-like domain
UTERU3001652 //Wiskott Aldrich syndrome homology region 2
UTERU3001766 //Apidaecin
UTERU3001988 //TPR Domain
UTERU3002667 //Polyomavirus coat protein
UTERU3003178 //TPR Domain// TPR Domain// TPR Domain// TPR Domain// PPR repeat
UTERU3005585 //PDZ domain (Also known as DHR or GLGF).
UTERU3007640 //NSF attachment protein
UTERU3008660 //TPR Domain// TPR Domain
UTERU3009871 //Ank repeat// Ank repeat// Ank repeat// Ank repeat// TPR Domain// Ank repeat// Ank repeat
UTERU3009979 //EGF-like domain// EGF-like domain// EGF-like domain// Trypsin Inhibitor like cysteine rich domain// EGF-like domain// Laminin G domain// Thrombospondin N-terminal -like domains// Laminin G domain
UTERU3015500 //Leucine rich repeat N-terminal domain// Leucine Rich Repeat// Leucine Rich Repeat// Leucine Rich Repeat//
Leucine Rich Repeat// Leucine Rich Repeat// Leucine Rich Repeat// Leucine Rich Repeat// Leucine Rich Repeat// Leucine Rich Repeat

Likewise, although the 45 clones described below have hit data in Pfam (see Example 5), it remains unclear as to which of the above-described categories each of the clones belong. However, if data for proteins comprising a similar domain or motif are accumulated and their functions are clarified in more detail, in the future these clones can be classified into any of the categories described above.
3NB692004724// KRAB box// Integrase core domain
ADRGL2000042// Nucleosome assembly protein (NAP)
BRACE2037299// Integrase core domain
BRALZ2017844// Homeobox domain
BRAWH2006207// KRAB box
BRCAN2002854// SAP domain
BRHIP2006617// TPR Domain// TPR Domain
BRHIP2012360// XPG N-terminal domain// XPG 1-region
BRHIP3008314// Sir2 family
BRTHA2016318// KE2 family protein
CTONG2019822// Hepatitis C virus core protein
FCBBF3010361// Fork head domain
FEBRA2006519// Thrombospondin type 1 domain// Thrombospondin type 1 domain
FEBRA2028256// EGF-like domain// EGF-like domain// EGF-like domain// EGF-like domain// EGF-like domain// TB domain// EGF-like domain// EGF-like domain// EGF-like domain// EGF-like domain// EB module// Squash family of serine protease inhibitors// EGF-like domain// EGF-like domain
FEBRA2028516// GRIP domain
HCASM2008536// XRCC1 N terminal domain
IMR322007078// UBA domain
IMR322008651// Helix-hairpin-helix motif.
LIVER2000247// Sodium
OCBBF2003327// Thrombospondin type 1 domain// Thrombospondin type 1 domain// Thrombospondin type 1 domain
PROST2009320// LIM domain containing proteins// LIM domain containing proteins
PUAEN2006335// Formin Homology 2 Domain
SKMUS2003194// SAP domain
SPLEN2039379// Transthyretin precursor (formerly prealbumin)
SYNOV1000256// Leucine Rich Repeat// BAH domain// Leucine Rich Repeat// Leucine Rich Repeat// Leucine Rich Repeat
SYNOV2006620// Nuclear transition protein 2
SYNOV4003981// Somatomedin B domain// WAP-type (Whey Acidic Protein) 'four-disulfide core'// Hemopexin// Hemopexin
SYNOV4005889// Apolipoprotein A1/A4/E family
TESOP2006865// KRAB box
TESTI1000266// Integrase core domain
TESTI2050780// Kazal-type serine protease inhibitor domain
TESTI4000137// Domain of unknown function
TESTI4024387// GDP dissociation inhibitor
TESTI4029528// RanBP1 domain.
TESTI4038721// Squash family of serine protease inhibitors
TESTI4046240// Sir2 family
THYMU2035078// Domain of unknown function DUF27
THYMU3000269// FAD binding domain
THYMU3000360// Integrase core domain
TRACH1000212// TSC-22/dip/bun family
TRACH2000862// Guanylate-binding protein
TRACH2019672// CRAL/TRIO domain.
TRACH2024559// IQ calmodulin-binding motif// IQ calmodulin-binding motif
UTERU2032279// Serpins (serine protease inhibitors)
UTERU2033577// KRAB box

In addition, when data for proteins are accumulated and novel domains and motifs are found, in the future the remaining clones, which had no hit data in the search with Pfam, can be classified into any of the above-described categories if a new functional domain or motif is identified by re-analyzing the deduced amino acid sequences of the clones using a homology search against an updated database.

### EXAMPLE 8

### Expression frequency analysis in silico

The cDNA libraries derived from various tissues and cells as indicated in Example 1 were prepared, and cDNA clones were selected from each library at random. The 5'-end sequences were determined and the database was constructed based on the data. The database was constructed based on the nucleotide sequences of 1,402,069 clones, and thus the population of the database is large enough for the analysis.

Then, clones having a homologous sequence are categorized into a single cluster (clustering) by searching the nucleotide sequences of respective clones in this database with the program of nucleotide sequence homology search; the number of clones belonging to each cluster was determined and normalized for every library; thus, the ratio of a certain gene in each cDNA library was determined. This analysis gave the information of the expression frequency of genes in tissues and cells which were sources of the cDNA libraries.

Then, in order to analyze the expression of a gene containing the nucleotide sequence of the cDNA of the present invention in tissues and cells, the library derived from a tissue or a cell used in the large-scale cDNA analysis was subjected to the comparison of the expression levels between tissues or cells. Namely, the expression frequency was analyzed by comparing the previously normalized values between tissues and/or cells for which the nucleotide sequences of 600 or more cDNA clones had been analyzed. By this analysis, some of the genes were revealed to be involved in the pathology and functions indicated below. Each value in Tables 2 to 24 shown below represents a relative expression frequency; the higher the value, the higher the expression level. The genes which are included a part of the Tables indicate not so big difference between compared libraries, but when compared with other libraries from another tissue or cell based on Example 9, they indicate significant difference. Thus, the genes are specific in each tissue or cell, and can be considered to be useful as diagnosing markers for the disease as well as useful for analyzing molecular mechanisms.

### Osteoporosis-related genes

Osteoporosis is a pathology in which bones are easily broken owing to overall decrease in components of bone. The onset involves the balance between the functions of osteoblast producing bone and osteoclast absorbing bone, namely bone metabolism. Thus, the genes involved in the increase of osteoclasts differentiating from precursor cells of monocyte/macrophage line (Molecular Medicine 38. 642-648. (2001)) are genes involved in osteoporosis relevant to bone metabolism.

A nucleotide sequence information-based analysis was carried out to identify the genes whose expression frequencies are higher or lower in CD34+ cell (cell expressing a glycoprotein CD34) treated with the osteoclast differentiation factor (Molecular Medicine 38. 642-648. (2001)) than in the untreated CD34+ cell, which is the precursor cell of monocyte/macrophage line. The result of comparative analysis for the frequency between the two cDNA libraries prepared from the RNA of CD34+ cells (CD34C) and from the RNA of CD34+ cells treated with the osteoclast differentiation factor (D3OST, D60ST or D9OST) showed that the genes whose expression levels were different between the two were the following 15 and 2 clones (Table 2).
BRACE3013780, BRAMY2047420, BRSTN2016470, CTONG3008894, D3OST2002182, D3OST2002648, D3OST3000169, PEBLM2005183, PUAEN2009655, TESTI4000014, TESTI4010851, TRACH2023299, TRACH2025535, TRACH3001427, UTERU2006137
HCHON2000508, TESTI2015626

These genes are involved in osteoporosis.

### Genes involved in neural cell differentiation

Genes involved in neural cell differentiation are useful for treating neurological diseases. Genes with varying expression levels in response to induction of cellular differentiation in neural cells are thought to be involved in neurological diseases.

A survey was performed for genes whose expression levels are varied in response to induction of differentiation (stimulation by retinoic acid (RA) or growth inhibitor treatment after RA stimulation) in cultured cells of a neural strain, NT2. The result of comparative analysis of cDNA libraries derived from undifferentiated NT2 cells (NT2RM) and the cells subjected to the differentiation treatment (NT2RP, NT2RI or NT2NE) showed that the genes whose expression levels were different between the two were the following 174 and 30 clones (Table 3).
BNGH42007788, BRACE1000186, BRACE2006319, BRACE2014306, BRACE2015058, BRACE2044286, BRACE3010428, BRAMY2044078, BRAWH2014645, BRAWH2014662, BRAWH3002574, BRAWH3003992, BRAWH3005981, BRAWH3007592, BRCAN2009432, BRCAN2016619, BRCAN2028355, BRHIP2001074, BRHIP2007741, BRHIP2014228, BRHIP2024146, BRHIP3007586, BRHIP3018797, BRTHA2003461, BRTHA3000633, BRTHA3003490, COLON2001721, CTONG1000087, CTONG2008233, CTONG2020638, CTONG2028124, CTONG3003905, CTONG3008894, CTONG3009028, CTONG3009239, DFNES2011499, FCBBF3001977, FEBRA1000030, FEBRA2006396, FEBRA2007801, HCHON2000028, HCHON2000244, HCHON2001084, HCHON2001217, HCHON2001548, HCHON2006250, HEART1000074, HHDPC1000118, HSYRA2009075, IMR322000127, IMR322001380, KIDNE2000665, KIDNE2002252, MESAN2006563, MESAN2012054, MESAN2015515, NT2NE2003252, NT2NE2005890, NT2NE2006531, NT2NE2006909, NT2NE2008060, NT2RI2003993, NT2RI2004618, NT2RI2005166, NT2RI2006686, NT2RI2008724, NT2RI2009855, NT2RI2011422, NT2RI2011683, NT2RI2012659, NT2RI2012990, NT2RI2013357, NT2RI2014247, NT2RI2014551, NT2RI2014733, NT2RI2016128, NT2RI2018311, NT2RI2018883, NT2RI2019751, NT2RI2023303, NT2RI2025909, NT2RI2025957, NT2RI2027081, NT2RI2027396, NT2RI3000622, NT2RI3001263, NT2RI3001515, NT2RI3002303, NT2RI3002842, NT2RI3002892, NT2RI3003031, NT2RI3003095, NT2RI3003162, NT2RI3003382, NT2RI3003409, NT2RI3004381, NT2RI3004510, NT2RI3005202, NT2RI3005403, NT2RI3005724, NT2RI3006132, NT2RI3006171, NT2RI3006284, NT2RI3006340, NT2RI3006376, NT2RI3006673, NT2RI3006796, NT2RI3007065, NT2RI3007158, NT2RI3007291, NT2RI3007543, NT2RI3007757, NT2RI3007978, NT2RI3008055, NT2RI3008162, NT2RI3008652, NT2RI3008697, NT2RI3008974, NT2RI3009158, NT2RP7000359, NT2RP7000466, NT2RP7004027, NT2RP7004123, NT2RP7005118, NT2RP7005529, NT2RP7005846, NT2RP7009030, NT2RP7009147, NT2RP7009867, NT2RP7010128, NT2RP7010599, NT2RP7011570, NT2RP7013795, NT2RP7014005, NT2RP7015512, NT2RP7017365, NT2RP7017474, NT2RP7017546, NT2RP8000137, NT2RP8000296, NT2RP8000483, NTONG2005969, OCBBF2007028, OCBBF2037068, PLACE7000514, PUAEN2007044, SPLEN2002467, SPLEN2006122, SPLEN2028914, SPLEN2031547, SYNOV4002346, SYNOV4007671, SYNOV4008440, TESOP2002273, TESTI2003573, TESTI4000014, TESTI4009286, TESTI4010851, TESTI4012702, TESTI4029671, TESTI4037156, THYMU3000133, TRACH1000205, TRACH2005811, TRACH2007834, TRACH2025535, TRACH3001427, TRACH3002192, TRACH3004721, TRACH3008093, TRACH3008535, TRACH3008713, UTERU2002410, UTERU2023175
ADRGL2000042, BRACE2003609, BRACE3003026, BRHIP3000017, CTONG2020411, FCBBF1000509, FCBBF3027854, FEBRA2028516, HCHON2000508, IMR322001879, NT2RI2005772, NT2RI2008952, NT2RI2009583, NT2RI2018448, NT2RI2027157, NT2RI3000174, NT2RI3001132, NT2RI3002557, NT2RI3005928, NT2RI3007167, NT2RI3007443, NT2RP7008435, NT2RP8000521, OCBBF2006987, PERIC2007068, TESTI2015626, TESTI4015442, TLIVE2002046, TRACH3000134, TUTER2000057

These genes are neurological disease-related genes.

### Genes involved in Alzheimer's disease

Alzheimer's disease is a cranial neurological disease that is characterized by memory loss. As the disease advances, patients can no longer support themselves and require nursing. Alzheimer's disease eventually leads to atrophication of the brain itself. Environmental factors such as stress, and vascular factors such as hypertension and cholesterolemia, are assumed, but not confirmed, to contribute to the onset of Alzheimer's disease. Genes whose expression levels differ between normal brain tissues and tissues affected with Alzheimer's disease are expected to be involved in Alzheimer's disease. Such genes can be used to elucidate the disease's onset mechanism and in genetic diagnosis. cDNA libraries derived from the cerebral cortex of Alzheimer patients (BRALZ and BRASW), and a library derived from whole tissues of a normal brain (BRAWH) were analyzed and compared (Table 4). The results showed that genes whose expression levels differed between the two are the following 250 and 41 clones listed below.
ASTRO1000009, BLADE2008398, BRACE1000186, BRACE1000258, BRACE1000533, BRACE2005457, BRACE2010489, BRACE2014657, BRACE2035381, BRACE2044286, BRACE2045954, BRACE3000787, BRACE3003192, BRACE3005499, BRACE3007480, BRACE3009237, BRACE3009724, BRACE3009747, BRACE3010428, BRACE3011271, BRACE3011421, BRACE3012364, BRACE3022769, BRACE3026735, BRACE3031838, BRALZ2011796, BRALZ2012183, BRALZ2012848, BRALZ2014484, BRALZ2016085, BRALZ2016498, BRALZ2017359, BRAMY2003008, BRAMY2005052, BRAMY2019300, BRAMY2019963, BRAMY2036567, BRAMY2037823, BRAMY2040592, BRAMY3002803, BRAMY3004224, BRAMY3005091, BRASW1000053, BRASW1000125, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCOC2003213, BRCOC2014033, BRCOC2020142, BRHIP2000920, BRHIP2005719, BRHIP2007741, BRHIP2014228, BRHIP2024146, BRHIP2026288, BRHIP3000339, BRHIP3006683, BRHIP3007586, BRHIP3008405, BRHIP3018797, BRSSN2000684, BRSSN2011738, BRSSN2014299, BRSTN2008052, BRSTN2015015, BRSTN2016470, BRTHA1000311, BRTHA2008335, BRTHA3002427, BRTHA3003490, BRTHA3008520, BRTHA3017848, COLON2001721, CTONG2017500, CTONG2028124, CTONG3000657, CTONG3001123, CTONG3009328, FCBBF2001183, FCBBF3001977, FEBRA2007544, FEBRA2007801, FEBRA2020886, FEBRA2028618, HCASM2007047, HCHON2000244, HCHON2000626, HCHON2001217, HCHON2002676, HCHON2006250, HEART1000074, HHDPC1000118, HLUNG2002465, IMR322000127, IMR322001380, IMR322002035, KIDNE2006580, MESAN2006563, MESAN2012054, MESTC1000042, NOVAR2001783, NT2NE2006909, NT2RI2008724, NT2RI2012659, NT2RI2014733, NT2RI3002892, NT2RI3006284, NT2RI3006673, NT2RI3007543, NT2RI3008055, NT2RP7005529, NT2RP7009147, NT2RP7014005, NT2RP7017474, NTONG2005969, OCBBF2001794, OCBBF2006005, OCBBF2006764, OCBBF2007028, OCBBF2007114, OCBBF2010140, OCBBF2021286, OCBBF2023162, OCBBF2024850, OCBBF2028935, OCBBF2036743, OCBBF2038317, OCBBF3000483, OCBBF3008230, PEBLM2004666, PLACE6001185, PUAEN2005930, PUAEN2006701, PUAEN2007044, PUAEN2009655, SMINT2001818, SPLEN2028914, SPLEN2031424, SPLEN2031547, SPLEN2034781, SPLEN2036932, SYNOV2014400, SYNOV4002346, SYNOV4002883, SYNOV4007430, SYNOV4007671, SYNOV4008440, TESOP2002273, TESOP2002451, TESTI4000014, TESTI4000209, TESTI4001100, TESTI4006137, TESTI4008797, TESTI4009286, TESTI4010851, TESTI4013817, TESTI4014694, TESTI4021478, TESTI4022936, TESTI4024420, TESTI4027821, THYMU2001090, THYMU2033308, THYMU2035735, THYMU2039315, THYMU3001234, THYMU3008171, TKIDN2009641, TKIDN2009889, TKIDN2015788, TRACH1000205, TRACH2001549, TRACH2005811, TRACH2006049, TRACH2007834, TRACH2008300, TRACH2025535, TRACH3001427, TRACH3002192, TRACH3004068, TRACH3004721, TRACH3005294, TRACH3007479, TRACH3008093, TRACH3009455, UTERU2005621, UTERU2006115, UTERU2019706, UTERU2023039, UTERU2026203, UTERU3005230, UTERU3007640, UTERU3009871
ADRGL2000042, BLADE2006830, BRACE2003609, BRALZ2017844, BRAMY3004800, BRAWH1000369, BRAWH2006207, BRAWH2006395, BRAWH2008993, BRAWH2009393, BRAWH2010552, BRAWH3007441, BRAWH3009017, BRHIP2005271, BRHIP3000017, BRHIP3026052, BRTHA2018443, BRTHA3003000, CTONG2020374, CTONG2020378, CTONG2024031, FCBBF1000509, FEBRA2001990, FEBRA2006519, FEBRA2028516, HCHON2000743, IMR322001879, NT2RI2009583, OCBBF2008144, PERIC2007068, PUAEN2006335, SPLEN2039379, TESTI4001984, TESTI4008058, TESTI4025268, TESTI4032090, THYMU3000360, TLIVE2002046, TRACH3000134, UTERU2021820, UTERU2028734

These genes are involved in Alzheimer's disease.

### Genes involved in Parkinson's disease

Parkinson's disease is a cranial neurological disease characterized by impaired production of the neurotransmitter dopamine in the substantia nigra in the brain. This results in dyskinesia, such as hand tremors, and impaired body movement due to muscular rigidity. Normally, the number of brain neurons gradually decreases with age. However, compared to healthy people, patients with Parkinson's disease experience a rapid and marked decrease in the number of neurons in their substantia nigra. Genes whose expression levels differ between tissues of the whole brain and the nigra are expected to be involved in Parkinson's disease. These genes exhibit nigra-specific alterations in their expression levels, and can be used to elucidate the disease onset mechanism and in gene diagnosis. cDNA libraries derived from the substantia nigra (BRSSN) and a library derived from whole tissues of a normal brain (BRAWH) were analyzed and compared (Table 5). Genes whose expression levels differed between the two were the 250 clones and 40 clones listed below.
ASTRO1000009, BLADE2008398, BRACE1000186, BRACE1000258, BRACE1000533, BRACE2005457, BRACE2010489, BRACE2014657, BRACE2035381, BRACE2044286, BRACE2045954, BRACE3000787, BRACE3003192, BRACE3005499, BRACE3007480, BRACE3009237, BRACE3009724, BRACE3009747, BRACE3010428, BRACE3011271, BRACE3011421, BRACE3012364, BRACE3013780, BRACE3022769, BRACE3026735, BRACE3031838, BRALZ2011796, BRAMY2003008, BRAMY2005052, BRAMY2019300, BRAMY2019963, BRAMY2036567, BRAMY2037823, BRAMY2040592, BRAMY2047420, BRAMY3002803, BRAMY3004224, BRAMY3005091, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCOC2003213, BRCOC2014033, BRCOC2020142, BRHIP2000920, BRHIP2005719, BRHIP2007741, BRHIP2014228, BRHIP2024146, BRHIP3000339, BRHIP3006683, BRHIP3007586, BRHIP3008405, BRHIP3018797, BRSSN2000684, BRSSN2003086, BRSSN2004496, BRSSN2004719, BRSSN2006892, BRSSN2008549, BRSSN2008797, BRSSN2011262, BRSSN2011738, BRSSN2013874, BRSSN2014299, BRSSN2014424, BRSSN2014556, BRSSN2018581, BRSSN2018925, BRSTN2008052, BRSTN2015015, BRSTN2016470, BRTHA1000311, BRTHA2003461, BRTHA2008335, BRTHA3002427, BRTHA3003490, BRTHA3008520, BRTHA3017848, COLON2001721, CTONG2017500, CTONG2028124, CTONG3000657, CTONG3001123, CTONG3009328, FCBBF2001183, FCBBF3001977, FEBRA2007544, FEBRA2007801, FEBRA2020886, FEBRA2024136, FEBRA2025427, FEBRA2028618, HCASM2007047, HCHON2000244, HCHON2000626, HCHON2001217, HCHON2002676, HCHON2006250, HEART1000074, HHDPC1000118, HLUNG2002465, IMR322000127, IMR322002035, KIDNE2006580, MESAN2006563, MESAN2012054, MESTC1000042, NOVAR2001783, NT2NE2006909, NT2RI2008724, NT2RI2012659, NT2RI2014733, NT2RI3002892, NT2RI3006284, NT2RI3006673, NT2RI3007543, NT2RI3008055, NT2RP7005529, NT2RP7009147, NT2RP7014005, NT2RP7017474, OCBBF2001794, OCBBF2006005, OCBBF2006764, OCBBF2007028, OCBBF2010140, OCBBF2021286, OCBBF2024850, OCBBF2028935, OCBBF2036743, OCBBF2038317, OCBBF3000483, OCBBF3008230, PEBLM2004666, PLACE6001185, PUAEN2005930, PUAEN2006701, PUAEN2007044, PUAEN2009655, SPLEN2028914, SPLEN2031424, SPLEN2031547, SPLEN2034781, SPLEN2036932, SYNOV2014400, SYNOV4002346, SYNOV4002883, SYNOV4007430, SYNOV4008440, TESOP2002451, TESTI4000014, TESTI4000209, TESTI4001100, TESTI4006137, TESTI4008797, TESTI4009286, TESTI4010851, TESTI4013817, TESTI4014694, TESTI4021478, TESTI4022936, TESTI4024420, TESTI4027821, TESTI4037156, THYMU2001090, THYMU2033308, THYMU2035735, THYMU2039315, THYMU3001234, THYMU3008171, TKIDN2009641, TKIDN2009889, TKIDN2015788, TRACH1000205, TRACH2001549, TRACH2005811, TRACH2006049, TRACH2007834, TRACH2008300, TRACH2025535, TRACH3001427, TRACH3002192, TRACH3004721, TRACH3005294, TRACH3007479, TRACH3008093, TRACH3009455, UTERU2006115, UTERU2019706, UTERU2023039, UTERU2026203, UTERU3005230, UTERU3007640, UTERU3009871
ADRGL2000042, BLADE2006830, BRACE2003609, BRAMY3004800, BRAWH1000369, BRAWH2006207, BRAWH2006395, BRAWH2008993, BRAWH2009393, BRAWH2010552, BRAWH3007441, BRAWH3009017, BRHIP2005271, BRHIP3000017, BRTHA2018443, BRTHA3003000, CTONG2020374, CTONG2020378, CTONG2024031, FCBBF1000509, FEBRA2001990, FEBRA2006519, FEBRA2028516, HCHON2000743, IMR322001879, NT2RI2009583, OCBBF2008144, PERIC2007068, PUAEN2006335, SPLEN2039379, TESTI2015626, TESTI4001984, TESTI4008058, TESTI4025268, TESTI4032090, THYMU3000360, TLIVE2002046, TRACH3000134, UTERU2021820, UTERU2028734

These genes are involved in Parkinson's disease.

### Genes involved in short-term memory and dementia

In the brain, the hippocampus is a highly important memory-related area. The hippocampus functions to establish a memory by judging whether acquired information is necessary, and then accumulating the memory in another area of the brain. According to clinical findings, patients can retain a new memory for only about five minutes with an abnormal, or at the worst without a hippocampus. Some dementia patients are presumed to have hippocampus abnormalities. Thus, genes whose expression levels differ between tissues of the whole brain and the hippocampus are expected to be involved in memory or dementia. Such genes can be used to elucidate the mechanism underlying the memory and in gene diagnosis. cDNA libraries derived from the hippocampus (BRHIP) and from the whole tissues of a normal brain (BRAWH) were analyzed and compared (Table 6). Genes whose expression levels differed between the two were the 370 clones and 59 clones listed below. ASTRO1000009, BLADE2001371, BLADE2008398, BNGH42007788, BRACE1000186, BRACE1000258, BRACE1000533, BRACE2005457, BRACE2010489, BRACE2014657, BRACE2015058, BRACE2018762, BRACE2030341, BRACE2035381, BRACE2044286, BRACE2045954, BRACE3000787, BRACE3003192, BRACE3005499, BRACE3007480, BRACE3009237, BRACE3009724, BRACE3009747, BRACE3010428, BRACE3011271, BRACE3011421, BRACE3012364, BRACE3018963, BRACE3022769, BRACE3026735, BRACE3031838, BRALZ2011796, BRAMY2003008, BRAMY2005052, BRAMY2019300, BRAMY2019963, BRAMY2031317, BRAMY2036567, BRAMY2037823, BRAMY2040592, BRAMY2044078, BRAMY3002803, BRAMY3004224, BRAMY3005091, BRAMY3009811, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2020710, BRCAN2028355, BRCOC2003213, BRCOC2014033, BRCOC2020142, BRHIP2000691, BRHIP2000819, BRHIP2000826, BRHIP2000920, BRHIP2001074, BRHIP2001805, BRHIP2001927, BRHIP2002122, BRHIP2002172, BRHIP2002346, BRHIP2003242, BRHIP2003786, BRHIP2003917, BRHIP2004312, BRHIP2004359, BRHIP2004814, BRHIP2004883, BRHIP2005236, BRHIP2005354, BRHIP2005600, BRHIP2005719, BRHIP2005752, BRHIP2005932, BRHIP2006800, BRHIP2007616, BRHIP2007741, BRHIP2009340, BRHIP2009414, BRHIP2009474, BRHIP2013699, BRHIP2014228, BRHIP2021615, BRHIP2022221, BRHIP2024146, BRHIP2024165, BRHIP2026061, BRHIP2026288, BRHIP2029176, BRHIP2029393, BRHIP3000339, BRHIP3000526, BRHIP3001283, BRHIP3006683, BRHIP3007483, BRHIP3007586, BRHIP3008183, BRHIP3008313, BRHIP3008344, BRHIP3008405, BRHIP3008565, BRHIP3008598, BRHIP3008997, BRHIP3009099, BRHIP3009448, BRHIP3011241, BRHIP3013765, BRHIP3013897, BRHIP3015751, BRHIP3016213, BRHIP3018797, BRHIP3020182, BRHIP3024118, BRHIP3024533, BRHIP3024725, BRHIP3025161, BRHIP3025702, BRHIP3026097, BRHIP3027137, BRHIP3027854, BRSSN2000684, BRSSN2004719, BRSSN2008549, BRSSN2011738, BRSSN2014299, BRSTN2008052, BRSTN2015015, BRSTN2016470, BRSTN2018083, BRTHA1000311, BRTHA2002442, BRTHA2008335, BRTHA3000297, BRTHA3001721, BRTHA3002427, BRTHA3003490, BRTHA3005046, BRTHA3008520, BRTHA3008778, BRTHA3009090, BRTHA3015910, BRTHA3017848, COLON2001721, CTONG1000087, CTONG1000088, CTONG1000467, CTONG2000042, CTONG2008233, CTONG2009423, CTONG2017500, CTONG2019788, CTONG2028124, CTONG3000657, CTONG3001123, CTONG3001370, CTONG3002412, CTONG3004072, CTONG3008894, CTONG3009239, CTONG3009328, DFNES2011499, FCBBF2001183, FCBBF3001977, FEBRA2000253, FEBRA2007544, FEBRA2007801, FEBRA2008287, FEBRA2010719, FEBRA2020886, FEBRA2028618, HCASM2007047, HCHON2000028, HCHON2000244, HCHON2000626, HCHON2001217, HCHON2002676, HCHON2005921, HCHON2006250, HEART1000074, HEART2007031, HHDPC1000118, HLUNG2002465, HLUNG2003003, IMR322000127, IMR322001380, IMR322002035, KIDNE2005543, KIDNE2006580, MESAN2006563, MESAN2012054, MESTC1000042, NOVAR2001783, NT2NE2006909, NT2RI2008724, NT2RI2012659, NT2RI2014733, NT2RI2018311, NT2RI3001515, NT2RI3002892, NT2RI3004510, NT2RI3005724, NT2RI3006284, NT2RI3006673, NT2RI3007291, NT2RI3007543, NT2RI3008055, NT2RP7005529, NT2RP7009147, NT2RP7014005, NT2RP7017474, OCBBF2001794, OCBBF2003819, OCBBF2006005, OCBBF2006151, OCBBF2006764, OCBBF2007028, OCBBF2007068, OCBBF2010140, OCBBF2020741, OCBBF2021286, OCBBF2024719, OCBBF2024850, OCBBF2028935, OCBBF2036743, OCBBF2038317, OCBBF3000296, OCBBF3000483, OCBBF3008230, PEBLM2004666, PLACE6001185, PUAEN2005930, PUAEN2006701, PUAEN2007044, PUAEN2009655, SPLEN2010912, SPLEN2012624, SPLEN2028914, SPLEN2031424, SPLEN2031547, SPLEN2034781, SPLEN2036932, SYNOV2014400, SYNOV4002346, SYNOV4002883, SYNOV4007430, SYNOV4008440, TESOP2002451, TESTI2049246, TESTI4000014, TESTI4000209, TESTI4001100, TESTI4006137, TESTI4008797, TESTI4009286, TESTI4010377, TESTI4010851, TESTI4010928, TESTI4011161, TESTI4013817, TESTI4014159, TESTI4014694, TESTI4014818, TESTI4021478, TESTI4022936, TESTI4024420, TESTI4027821, TESTI4037156, THYMU2001090, THYMU2023967, THYMU2025707, THYMU2031341, THYMU2033308, THYMU2035735, THYMU2037226, THYMU2039315, THYMU3001234, THYMU3001379, THYMU3004835, THYMU3007137, THYMU3008171, TKIDN2009641, TKIDN2009889, TKIDN2015788, TRACH1000205, TRACH2001549, TRACH2005811, TRACH2006049, TRACH2007834, TRACH2008300, TRACH2025535, TRACH3000014, TRACH3001427, TRACH3002192, TRACH3004721, TRACH3005294, TRACH3007479, TRACH3008093, TRACH3009455, TUTER1000122, TUTER2000904, UTERU2004929, UTERU2006115, UTERU2019706, UTERU2021163, UTERU2023039, UTERU2026203, UTERU2030213, UTERU3001572, UTERU3003135, UTERU3005230, UTERU3007640, UTERU3009259, UTERU3009871
ADRGL2000042, BLADE2006830, BRACE2003609, BRAMY3004800, BRAWH1000369, BRAWH2006207, BRAWH2006395, BRAWH2008993, BRAWH2009393, BRAWH2010552, BRAWH3007441, BRAWH3009017, BRHIP2002722, BRHIP2003272, BRHIP2005271, BRHIP2005724, BRHIP2006617, BRHIP2008389, BRHIP2012360, BRHIP2017553, BRHIP2026877, BRHIP3000017, BRHIP3000240, BRHIP3008314, BRHIP3026052, BRTHA2018443, BRTHA3003000, CTONG2020374, CTONG2020378, CTONG2024031, CTONG3004726, FCBBF1000509, FEBRA2001990, FEBRA2006519, FEBRA2028516, HCHON2000743, IMR322001879, NT2RI2009583, OCBBF2006987, OCBBF2008144, OCBBF2030116, PERIC2007068, PUAEN2006335, SPLEN2039379, TESTI2015626, TESTI4000214, TESTI4001984, TESTI4008058, TESTI4013894, TESTI4025268, TESTI4025547, TESTI4026207, TESTI4032090, THYMU3000360, TLIVE2002046, TRACH3000134, UTERU2008040, UTERU2021820, UTERU2028734

These genes are involved in memory and dementia.

### Genes involved in equilibrium sense and movement function

The cerebellum is the center of equilibrium sense, muscular movement, and motor learning. This area is thought to be involved in motor control, and smooth movements are achieved unconsciously due to cerebellum action. Recent studies have elucidated that the cerebellum participates in not only simple movements but also in establishing higher-order movements such as reading and writing. Thus, genes whose expression levels differ between tissues of the whole brain and the cerebellum are expected to be involved in equilibrium sense or motor function, which can be useful for elucidating the molecular mechanism controlled by the brain. cDNA libraries derived from the cerebellum (BRACE) and from the whole tissues of a normal brain (BRAWH) were analyzed and compared (Table 7). Genes whose expression levels differed between the two were the 488 clones and 66 clones listed below.
ADRGL2009146, ADRGL2012038, ASTRO1000009, ASTRO2003960, BLADE1000176, BLADE2004089, BLADE2008398, BRACE1000186, BRACE1000258, BRACE1000533, BRACE1000572, BRACE2003639, BRACE2005457, BRACE2006319, BRACE2008594, BRACE2010489, BRACE2011747, BRACE2014306, BRACE2014475, BRACE2014657, BRACE2015058, BRACE2015314, BRACE2016981, BRACE2018762, BRACE2024627, BRACE2026836, BRACE2027258, BRACE2027970, BRACE2028970, BRACE2029112, BRACE2029849, BRACE2030326, BRACE2030341, BRACE2030884, BRACE2031154, BRACE2031389, BRACE2031527, BRACE2031531, BRACE2031899, BRACE2032044, BRACE2032329, BRACE2032385, BRACE2032538, BRACE2032823, BRACE2033720, BRACE2035381, BRACE2035441, BRACE2036005, BRACE2036096, BRACE2036830, BRACE2036834, BRACE2037847, BRACE2038114, BRACE2038329, BRACE2038551, BRACE2039249, BRACE2039327, BRACE2039475, BRACE2039734, BRACE2040138, BRACE2040325, BRACE2041009, BRACE2041200, BRACE2041264, BRACE2042550, BRACE2043142, BRACE2043248, BRACE2043349, BRACE2043665, BRACE2044286, BRACE2044816, BRACE2044949, BRACE2045300, BRACE2045428, BRACE2045596, BRACE2045772, BRACE2045947, BRACE2045954, BRACE2046251, BRACE2046295, BRACE2047011, BRACE2047350, BRACE2047377, BRACE2047385, BRACE3000071, BRACE3000697, BRACE3000787, BRACE3000840, BRACE3000973, BRACE3001002, BRACE3001217, BRACE3001391, BRACE3001595, BRACE3001754, BRACE3002298, BRACE3002390, BRACE3002508, BRACE3003004, BRACE3003192, BRACE3003595, BRACE3003698, BRACE3004058, BRACE3004113, BRACE3004150, BRACE3004358, BRACE3004435, BRACE3004772, BRACE3004783, BRACE3004843, BRACE3004880, BRACE3005145, BRACE3005225, BRACE3005430, BRACE3005499, BRACE3006185, BRACE3006226, BRACE3006462, BRACE3006872, BRACE3007322, BRACE3007472, BRACE3007480, BRACE3007559, BRACE3007625, BRACE3007642, BRACE3007767, BRACE3008036, BRACE3008092, BRACE3008137, BRACE3008384, BRACE3008720, BRACE3008772, BRACE3009090, BRACE3009237, BRACE3009297, BRACE3009377, BRACE3009574, BRACE3009701, BRACE3009708, BRACE3009724, BRACE3009747, BRACE3010397, BRACE3010428, BRACE3011271, BRACE3011421, BRACE3011505, BRACE3012364, BRACE3012930, BRACE3013119, BRACE3013576, BRACE3013740, BRACE3013780, BRACE3014005, BRACE3014068, BRACE3014231, BRACE3014317, BRACE3014807, BRACE3015027, BRACE3015121, BRACE3015262, BRACE3015521, BRACE3015894, BRACE3016884, BRACE3018308, BRACE3018963, BRACE3019055, BRACE3019084, BRACE3020194, BRACE3020286, BRACE3020594, BRACE3022769, BRACE3023912, BRACE3024073, BRACE3024659, BRACE3024662, BRACE3025153, BRACE3025457, BRACE3025531, BRACE3025630, BRACE3026008, BRACE3026075, BRACE3026735, BRACE3027242, BRACE3027326, BRACE3027478, BRACE3030103, BRACE3031838, BRACE3032983, BRACE3040856, BRACE3045033, BRALZ2011796, BRAMY2003008, BRAMY2005052, BRAMY2019300, BRAMY2019963, BRAMY2020058, BRAMY2030098, BRAMY2031317, BRAMY2036567, BRAMY2037823, BRAMY2039872, BRAMY2040592, BRAMY2044078, BRAMY2047420, BRAMY3002620, BRAMY3002803, BRAMY3004224, BRAMY3005091, BRAMY3005932, BRAMY4000229, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2009432, BRCAN2010376, BRCAN2015371, BRCAN2020710, BRCOC2003213, BRCOC2007034, BRCOC2014033, BRCOC2020142, BRHIP2000920, BRHIP2004359, BRHIP2005719, BRHIP2005752, BRHIP2007741, BRHIP2013699, BRHIP2014228, BRHIP2024146, BRHIP3000339, BRHIP3006683, BRHIP3007586, BRHIP3008313, BRHIP3008405, BRHIP3018797, BRSSN2000684, BRSSN2006892, BRSSN2011262, BRSSN2011738, BRSSN2014299, BRSTN2008052, BRSTN2010750, BRSTN2015015, BRSTN2016470, BRTHA1000311, BRTHA2008335, BRTHA2008955, BRTHA2011194, BRTHA3001721, BRTHA3002427, BRTHA3003490, BRTHA3008520, BRTHA3009090, BRTHA3017848, COLON2001721, CTONG2008233, CTONG2017500, CTONG2028124, CTONG3000657, CTONG3001123, CTONG3005813, CTONG3008894, CTONG3009328, DFNES2011499, FCBBF2001183, FCBBF3001977, FEBRA2006396, FEBRA2007544, FEBRA2007708, FEBRA2007801, FEBRA2008287, FEBRA2020886, FEBRA2021966, FEBRA2026984, FEBRA2028618, HCASM2007047, HCHON2000244, HCHON2000626, HCHON2001217, HCHON2002676, HCHON2005921, HCHON2006250, HEART1000074, HHDPC1000118, HLUNG2002465, IMR322000127, IMR322001380, IMR322002035, KIDNE2000665, KIDNE2006580, MESAN2006563, MESAN2012054, MESTC1000042, NB9N41000340, NESOP2001752, NOVAR2001783, NT2NE2006909, NT2RI2005166, NT2RI2008724, NT2RI2012659, NT2RI2014733, NT2RI2019751, NT2RI3002892, NT2RI3003382, NT2RI3004510, NT2RI3005724, NT2RI3006284, NT2RI3006673, NT2RI3007291, NT2RI3007543, NT2RI3008055, NT2RP7004123, NT2RP7005529, NT2RP7009147, NT2RP7010599, NT2RP7014005, NT2RP7017474, NTONG2005969, OCBBF2001794, OCBBF2003819, OCBBF2006005, OCBBF2006151, OCBBF2006764, OCBBF2007028, OCBBF2010140, OCBBF2020343, OCBBF2020741, OCBBF2021286, OCBBF2022351, OCBBF2024850, OCBBF2025527, OCBBF2028935, OCBBF2036743, OCBBF2038317, OCBBF3000483, OCBBF3007516, OCBBF3008230, PEBLM2004666, PERIC2000889, PLACE6001185, PUAEN2002489, PUAEN2005930, PUAEN2006701, PUAEN2007044, PUAEN2009655, SPLEN2010912, SPLEN2012624, SPLEN2027268, SPLEN2028914, SPLEN2031424, SPLEN2031547, SPLEN2034781, SPLEN2036932, SPLEN2037194, SYNOV2014400, SYNOV4002346, SYNOV4002883, SYNOV4007430, SYNOV4007671, SYNOV4008440, TESOP2002273, TESOP2002451, TESOP2002950, TESTI1000330, TESTI4000014, TESTI4000209, TESTI4000349, TESTI4001100, TESTI4001561, TESTI4006137, TESTI4008797, TESTI4009286, TESTI4010851, TESTI4011161, TESTI4013675, TESTI4013817, TESTI4014159, TESTI4014306, TESTI4014694, TESTI4021478, TESTI4022936, TESTI4024420, TESTI4027821, TESTI4037156, TESTI4046819, THYMU2001090, THYMU2016523, THYMU2023967, THYMU2030264, THYMU2033308, THYMU2035735, THYMU2039315, THYMU2039780, THYMU3001083, THYMU3001234, THYMU3003309, THYMU3006485, THYMU3008171, TKIDN2009641, TKIDN2009889, TKIDN2015788, TRACH1000205, TRACH2001549, TRACH2005811, TRACH2006049, TRACH2007834, TRACH2008300, TRACH2025535, TRACH3001427, TRACH3002192, TRACH3004721, TRACH3005294, TRACH3006038, TRACH3006412, TRACH3007479, TRACH3008093, TRACH3009455, TUTER2000904, UTERU2002410, UTERU2006115, UTERU2007520, UTERU2019706, UTERU2023039, UTERU2026203, UTERU3000226, UTERU3001572, UTERU3005230, UTERU3005460, UTERU3005970, UTERU3006308, UTERU3007419, UTERU3007640, UTERU3007913, UTERU3009871 ADRGL2000042, BLADE2006830, BRACE2002589, BRACE2003609, BRACE2009318, BRACE2011677, BRACE2029396, BRACE2037299, BRACE2039823, BRACE2039832, BRACE2043105, BRACE3001058, BRACE3001113, BRACE3003026, BRACE3003053, BRACE3009127, BRACE3010076, BRACE3015829, BRACE3021148, BRAMY3004800, BRAWH1000369, BRAWH2006207, BRAWH2006395, BRAWH2008993, BRAWH2009393, BRAWH2010552, BRAWH3007441, BRAWH3009017, BRCOC2019841, BRHIP2005271, BRHIP3000017, BRHIP3000240, BRTHA2018443, BRTHA3003000, CTONG2020374, CTONG2020378, CTONG2024031, FCBBF1000509, FEBRA2001990, FEBRA2006519, FEBRA2028516, HCHON2000743, IMR322001879, NT2RI2009583, NT2RP8000521, OCBBF2008144, OCBBF2011669, PERIC2007068, PUAEN2006335, SPLEN2039379, SYNOV2021953, TESTI2015626, TESTI4001984, TESTI4008058, TESTI4013894, TESTI4025268, TESTI4032090, THYMU2004284, THYMU2040925, THYMU3000360, TLIVE2002046, TRACH3000134, UTERU2008040, UTERU2011220, UTERU2021820, UTERU2028734

These genes are involved in equilibrium sense or motor function.

### Genes involved in signaling from sensory organs

The thalamus is an area which comprises many neurons strongly connected to the cerebrum, and which transmits sensory information from the spinal cord or such to the responsible area of the cerebrum. The thalamus also controls the direction of movement from the cerebrum. For example, the thalamus resolves vision into the elements of size, shape, and color, and resolves sound into volume and sweetness or harshness to the ear, and then transmits this information to the sensory area of the cerebral cortex. Thus, genes whose expression levels differ between tissues of the whole brain and the thalamus are expected to be involved in signaling from sensory organs. These genes can be used to elucidate the molecular mechanism underlying signaling controlled by the brain. cDNA libraries derived from the thalamus (BRTHA) and from whole tissues of a normal brain (BRAWH) were analyzed and compared (Table 8). Genes whose expression levels differed between the two were the 412 clones and 56 clones listed below.
ASTRO1000009, ASTRO3000482, BLADE2008398, BRACE1000186, BRACE1000258, BRACE1000533, BRACE2005457, BRACE2010489, BRACE2014306, BRACE2014657, BRACE2015058, BRACE2031154, BRACE2035381, BRACE2044286, BRACE2045954, BRACE3000787, BRACE3003192, BRACE3005499, BRACE3007480, BRACE3008384, BRACE3009237, BRACE3009724, BRACE3009747, BRACE3010397, BRACE3010428, BRACE3011271, BRACE3011421, BRACE3012364, BRACE3022769, BRACE3026735, BRACE3027478, BRACE3031838, BRALZ2011796, BRAMY2003008, BRAMY2005052, BRAMY2019300, BRAMY2019963, BRAMY2028914, BRAMY2031317, BRAMY2036567, BRAMY2037823, BRAMY2040592, BRAMY2044078, BRAMY3002803, BRAMY3004224, BRAMY3005091, BRAMY4000229, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2006297, BRCOC2003213, BRCOC2014033, BRCOC2020142, BRHIP2000819, BRHIP2000920, BRHIP2005719, BRHIP2007741, BRHIP2009474, BRHIP2013699, BRHIP2014228, BRHIP2022221, BRHIP2024146, BRHIP3000339, BRHIP3006683, BRHIP3007586, BRHIP3008405, BRHIP3018797, BRSSN2000684, BRSSN2008549, BRSSN2008797, BRSSN2011738, BRSSN2014299, BRSTN2004863, BRSTN2008052, BRSTN2015015, BRSTN2016470, BRTHA1000311, BRTHA2000855, BRTHA2001462, BRTHA2002115, BRTHA2002281, BRTHA2002376, BRTHA2002442, BRTHA2002493, BRTHA2002608, BRTHA2002808, BRTHA2003030, BRTHA2003110, BRTHA2003116, BRTHA2003461, BRTHA2004821, BRTHA2004978, BRTHA2005579, BRTHA2005956, BRTHA2006075, BRTHA2006146, BRTHA2006194, BRTHA2007122, BRTHA2007422, BRTHA2007603, BRTHA2008316, BRTHA2008335, BRTHA2008527, BRTHA2008535, BRTHA2008955, BRTHA2009311, BRTHA2009846, BRTHA2009972, BRTHA2010073, BRTHA2010608, BRTHA2010884, BRTHA2010907, BRTHA2011194, BRTHA2011351, BRTHA2011500, BRTHA2011641, BRTHA2012392, BRTHA2012562, BRTHA2012980, BRTHA2013262, BRTHA2013460, BRTHA2013707, BRTHA2014792, BRTHA2014828, BRTHA2015406, BRTHA2015478, BRTHA2015696, BRTHA2015878, BRTHA2016215, BRTHA2016496, BRTHA2016543, BRTHA2017353, BRTHA2017985, BRTHA2018165, BRTHA2018344, BRTHA2018591, BRTHA2018624, BRTHA2018707, BRTHA2019014, BRTHA2019022, BRTHA2019048, BRTHA3000273, BRTHA3000297, BRTHA3000633, BRTHA3001721, BRTHA3002401, BRTHA3002427, BRTHA3002933, BRTHA3003074, BRTHA3003343, BRTHA3003449, BRTHA3003474, BRTHA3003490, BRTHA3004475, BRTHA3005046, BRTHA3006856, BRTHA3007113, BRTHA3007148, BRTHA3007319, BRTHA3007769, BRTHA3008143, BRTHA3008310, BRTHA3008386, BRTHA3008520, BRTHA3008778, BRTHA3009037, BRTHA3009090, BRTHA3009291, BRTHA3010366, BRTHA3013884, BRTHA3015815, BRTHA3015910, BRTHA3016845, BRTHA3016917, BRTHA3017047, BRTHA3017589, BRTHA3017848, BRTHA3018514, BRTHA3018617, BRTHA3018656, BRTHA3019105, COLON2001721, CTONG1000087, CTONG2008233, CTONG2017500, CTONG2019788, CTONG2023021, CTONG2028124, CTONG3000657, CTONG3001123, CTONG3008894, CTONG3009028, CTONG3009239, CTONG3009328, FCBBF2001183, FCBBF3001977, FCBBF3021576, FEBRA2007544, FEBRA2007801, FEBRA2008287, FEBRA2008360, FEBRA2020886, FEBRA2028618, HCASM2007047, HCHON2000028, HCHON2000212, HCHON2000244, HCHON2000626, HCHON2001084, HCHON2001217, HCHON2002676, HCHON2005921, HCHON2006250, HEART1000074, HEART2007031, HHDPC1000118, HLUNG2001996, HLUNG2002465, IMR322000127, IMR322001380, IMR322002035, KIDNE2002252, KIDNE2005543, KIDNE2006580, KIDNE2011314, MESAN2006563, MESAN2012054, MESTC1000042, NOVAR2001783, NT2NE2006909, NT2RI2008724, NT2RI2012659, NT2RI2014733, NT2RI3002842, NT2RI3002892, NT2RI3005403, NT2RI3006284, NT2RI3006673, NT2RI3007543, NT2RI3008055, NT2RP7004123, NT2RP7005529, NT2RP7009147, NT2RP7014005, NT2RP7017474, NTONG2005969, NTONG2008088, OCBBF2001794, OCBBF2006005, OCBBF2006764, OCBBF2007028, OCBBF2010140, OCBBF2020639, OCBBF2021286, OCBBF2024719, OCBBF2024850, OCBBF2028935, OCBBF2036743, OCBBF2038317, OCBBF3000483, OCBBF3008230, PEBLM2004666, PLACE6001185, PUAEN2002489, PUAEN2005930, PUAEN2006701, PUAEN2007044, PUAEN2009655, RECTM2001347, SKMUS2000757, SPLEN2006122, SPLEN2010912, SPLEN2025491, SPLEN2028914, SPLEN2031424, SPLEN2031547, SPLEN2032154, SPLEN2034781, SPLEN2036821, SPLEN2036932, SYNOV1000374, SYNOV2014400, SYNOV4002346, SYNOV4002883, SYNOV4007430, SYNOV4007671, SYNOV4008440, TESOP2002451, TESTI2049246, TESTI4000014, TESTI4000209, TESTI4001100, TESTI4002290, TESTI4006137, TESTI4008797, TESTI4009286, TESTI4010851, TESTI4012702, TESTI4013817, TESTI4014159, TESTI4014694, TESTI4021478, TESTI4022936, TESTI4024420, TESTI4027821, TESTI4037156, THYMU2001090, THYMU2025707, THYMU2032825, THYMU2033308, THYMU2033787, THYMU2035735, THYMU2039315, THYMU2040975, THYMU3001234, THYMU3001379, THYMU3004835, THYMU3008171, TKIDN2009641, TKIDN2009889, TKIDN2015788, TLIVE2001327, TRACH1000205, TRACH2001549, TRACH2005811, TRACH2006049, TRACH2007834, TRACH2008300, TRACH2023299, TRACH2025535, TRACH3001427, TRACH3002192, TRACH3004068, TRACH3004721, TRACH3005294, TRACH3007479, TRACH3008093, TRACH3009455, TSTOM1000135, TUTER2000904, UTERU2002410, UTERU2006115, UTERU2019706, UTERU2019940, UTERU2023039, UTERU2023175, UTERU2026203, UTERU2030280, UTERU3000899, UTERU3001571, UTERU3001572, UTERU3004709, UTERU3005230, UTERU3005907, UTERU3007640, UTERU3009871 ADRGL2000042, BLADE2006830, BRACE2003609, BRAMY3004800, BRAWH1000369, BRAWH2006207, BRAWH2006395, BRAWH2008993, BRAWH2009393, BRAWH2010552, BRAWH3007441, BRAWH3009017, BRHIP2005271, BRHIP3000017, BRTHA2002133, BRTHA2002702, BRTHA2007060, BRTHA2010033, BRTHA2011321, BRTHA2013426, BRTHA2013610, BRTHA2016318, BRTHA2017364, BRTHA2017972, BRTHA2018011, BRTHA2018443, BRTHA3000296, BRTHA3003000, BRTHA3008826, CTONG2008721, CTONG2020374, CTONG2020378, CTONG2024031, FCBBF1000509, FEBRA2001990, FEBRA2006519, FEBRA2028516, HCHON2000743, HSYRA2005628, IMR322001879, NT2RI2009583, OCBBF2008144, PERIC2007068, PUAEN2006335, SPLEN2016932, SPLEN2039379, SYNOV2006620, TESTI4001984, TESTI4008058, TESTI4025268, TESTI4032090, THYMU3000360, TLIVE2002046, TRACH3000134, UTERU2021820, UTERU2028734

These genes are involved in signaling from sensory organs.

### Genes involved in emotional reaction

The amygdala is the center of emotion in the brain. Information passing through the amygdala induces an emotional reaction, for example, panic or fear. When a strong fear reaction is produced due to the emotional evaluation of stimulus in the amygdala, the amygdala transmits an alert signal to each area of the brain. This results in various reactions such as sweating palms, palpitation, elevated blood pressure, and rapid secretion of adrenaline. In other words, the amygdala transmits signals which cause the body to be on the alert and is a tissue involved in a kind of defense instinct. Thus, genes whose expression levels differ between tissues of the whole brain and the amygdala are expected to be involved in emotional reaction. Such genes can be used to elucidate the molecular mechanism underlying emotional reaction, fear, or panic. cDNA libraries derived from the amygdale (BRAMY) and from whole tissues of a normal brain (BRAWH) were analyzed and compared (Table 9). Genes whose expression levels differed between the two were the 383 clones and 55 clones listed below.
ASTRO1000009, BLADE2008398, BRACE1000186, BRACE1000258, BRACE1000533, BRACE2005457, BRACE2006319, BRACE2010489, BRACE2014657, BRACE2015058, BRACE2027258, BRACE2030341, BRACE2031389, BRACE2035381, BRACE2044286, BRACE2045954, BRACE3000787, BRACE3000973, BRACE3003192, BRACE3005499, BRACE3007480, BRACE3008036, BRACE3009237, BRACE3009724, BRACE3009747, BRACE3010428, BRACE3011271, BRACE3011421, BRACE3012364, BRACE3013780, BRACE3022769, BRACE3026735, BRACE3027478, BRACE3031838, BRALZ2011796, BRAMY2001473, BRAMY2003008, BRAMY2004771, BRAMY2005052, BRAMY2017528, BRAMY2019300, BRAMY2019963, BRAMY2019985, BRAMY2020058, BRAMY2020270, BRAMY2021498, BRAMY2028856, BRAMY2028914, BRAMY2029602, BRAMY2030098, BRAMY2030109, BRAMY2030702, BRAMY2030703, BRAMY2030799, BRAMY2031317, BRAMY2031377, BRAMY2031442, BRAMY2032014, BRAMY2032242, BRAMY2032317, BRAMY2033003, BRAMY2033116, BRAMY2033267, BRAMY2033594, BRAMY2034185, BRAMY2034920, BRAMY2034993, BRAMY2036387, BRAMY2036396, BRAMY2036567, BRAMY2036699, BRAMY2036913, BRAMY2037823, BRAMY2038100, BRAMY2038484, BRAMY2038846, BRAMY2038904, BRAMY2039872, BRAMY2040478, BRAMY2040592, BRAMY2041261, BRAMY2041378, BRAMY2041542, BRAMY2042612, BRAMY2042641, BRAMY2042760, BRAMY2042918, BRAMY2044078, BRAMY2044246, BRAMY2045036, BRAMY2046478, BRAMY2046742, BRAMY2046989, BRAMY2047169, BRAMY2047420, BRAMY2047676, BRAMY2047746, BRAMY2047751, BRAMY2047765, BRAMY2047884, BRAMY3000206, BRAMY3000213, BRAMY3001401, BRAMY3001794, BRAMY3002312, BRAMY3002620, BRAMY3002803, BRAMY3002805, BRAMY3004224, BRAMY3004672, BRAMY3004900, BRAMY3004919, BRAMY3005091, BRAMY3005932, BRAMY3006297, BRAMY3007206, BRAMY3007609, BRAMY3008466, BRAMY3008505, BRAMY3008650, BRAMY3009811, BRAMY3010411, BRAMY4000095, BRAMY4000229, BRAMY4000277, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2014881, BRCAN2017717, BRCOC2000670, BRCOC2003213, BRCOC2014033, BRCOC2020142, BRHIP2000920, BRHIP2005719, BRHIP2007741, BRHIP2014228, BRHIP2024146, BRHIP2026061, BRHIP3000339, BRHIP3001283, BRHIP3006683, BRHIP3007586, BRHIP3008405, BRHIP3018797, BRSSN2000684, BRSSN2004496, BRSSN2011738, BRSSN2014299, BRSTN2008052, BRSTN2010750, BRSTN2015015, BRSTN2016470, BRTHA1000311, BRTHA2008335, BRTHA2011641, BRTHA3001721, BRTHA3002427, BRTHA3003490, BRTHA3004475, BRTHA3008520, BRTHA3009090, BRTHA3017848, COLON2001721, CTONG1000087, CTONG2008233, CTONG2017500, CTONG2028124, CTONG3000657, CTONG3001123, CTONG3008894, CTONG3009239, CTONG3009328, FCBBF2001183, FCBBF3001977, FEBRA2007544, FEBRA2007801, FEBRA2008287, FEBRA2010719, FEBRA2020886, FEBRA2025427, FEBRA2028618, HCASM2007047, HCHON2000244, HCHON2000626, HCHON2001217, HCHON2002676, HCHON2006250, HCHON2008112, HEART1000074, HHDPC1000118, HLUNG2002465, HSYRA2009075, IMR322000127, IMR322001380, IMR322002035, KIDNE2000665, KIDNE2006580, MESAN2006563, MESAN2012054, MESAN2015515, MESTC1000042, NOVAR2001783, NT2NE2005890, NT2NE2006909, NT2RI2008724, NT2RI2012659, NT2RI2014733, NT2RI3001515, NT2RI3002892, NT2RI3005724, NT2RI3006284, NT2RI3006673, NT2RI3007543, NT2RI3008055, NT2RP7005529, NT2RP7009147, NT2RP7014005, NT2RP7017474, NTONG2005969, OCBBF1000254, OCBBF2001794, OCBBF2006005, OCBBF2006764, OCBBF2007028, OCBBF2007114, OCBBF2010140, OCBBF2021286, OCBBF2023162, OCBBF2024850, OCBBF2028935, OCBBF2035214, OCBBF2036743, OCBBF2038317, OCBBF3000483, OCBBF3008230, PEBLM2004666, PERIC2000889, PERIC2003720, PLACE6001185, PUAEN2005930, PUAEN2006701, PUAEN2007044, PUAEN2009174, PUAEN2009655, SKNMC2002402, SKNSH2000482, SPLEN2001599, SPLEN2002467, SPLEN2028914, SPLEN2029912, SPLEN2031424, SPLEN2031547, SPLEN2034781, SPLEN2036932, SPLEN2038345, SYNOV2014400, SYNOV4002346, SYNOV4002883, SYNOV4007430, SYNOV4007671, SYNOV4008440, TESOP2002451, TESTI2009474, TESTI4000014, TESTI4000209, TESTI4001100, TESTI4006137, TESTI4008797, TESTI4009286, TESTI4010851, TESTI4013817, TESTI4014159, TESTI4014694, TESTI4021478, TESTI4022936, TESTI4024420, TESTI4027821, TESTI4029836, TESTI4037156, TESTI4037188, THYMU2001090, THYMU2014353, THYMU2033308, THYMU2035735, THYMU2037226, THYMU2039315, THYMU3001234, THYMU3001379, THYMU3004835, THYMU3008171, TKIDN2009641, TKIDN2009889, TKIDN2015788, TLIVE2004320, TRACH1000205, TRACH2001549, TRACH2001684, TRACH2005811, TRACH2006049, TRACH2007834, TRACH2008300, TRACH2025344, TRACH2025535, TRACH2025911, TRACH3001427, TRACH3002192, TRACH3004068, TRACH3004721, TRACH3005294, TRACH3007479, TRACH3008093, TRACH3009455, TUTER2000904, UTERU2002410, UTERU2004929, UTERU2006115, UTERU2007520, UTERU2019706, UTERU2023039, UTERU2026203, UTERU3001572, UTERU3001766, UTERU3005230, UTERU3007640, UTERU3009517, UTERU3009871
ADRGL2000042, BLADE2006830, BRACE2003609, BRACE2039823, BRAMY2019111, BRAMY2035070, BRAMY2035449, BRAMY2035718, BRAMY2038516, BRAMY2039341, BRAMY2040159, BRAMY2041434, BRAMY2045471, BRAMY3004800, BRAWH1000369, BRAWH2006207, BRAWH2006395, BRAWH2008993, BRAWH2009393, BRAWH2010552, BRAWH3007441, BRAWH3009017, BRHIP2005271, BRHIP3000017, BRTHA2018443, BRTHA3003000, CTONG2020374, CTONG2020378, CTONG2024031, FCBBF1000509, FEBRA2001990, FEBRA2006519, FEBRA2028516, HCHON2000508, HCHON2000743, IMR322001879, NT2RI2009583, OCBBF2008144, PERIC2007068, PUAEN2006335, SPLEN2039379, TESTI2015626, TESTI2026647, TESTI4001984, TESTI4008058, TESTI4013894, TESTI4025268, TESTI4032090, THYMU3000360, TKIDN2018926, TLIVE2002046, TRACH3000134, UTERU2008040, UTERU2021820, UTERU2028734

These genes are involved in emotional reaction.

### Cancer-related genes

Cancer tissues are assumed to express a distinct set of genes distinct from normal tissues, and thus expression of these genes can contribute to carcinogenesis in tissues and cells. Thus, genes whose expression patterns in cancer tissues differ from those in normal tissues are cancer-related genes. A search was carried out for genes whose expression levels in cancer tissues differed from those in normal tissues.

The result of comparative analysis of cDNA libraries derived from breast tumor (TBAES) and normal breast (BEAST) (Table 10) showed that the genes whose expression levels differed between the two were 35 and four clones as described below.
ASTRO2002842, BRACE3016884, BRSSN2011262, BRTHA2008335, HCHON2000244, HCHON2006250, HEART1000010, MESAN2012054, NT2RP7000466, NT2RP7009147, OCBBF2021020, PEBLM2002749, PEBLM2004666, SPLEN2001599, SPLEN2031547, STOMA1000189, TBAES2001171, TBAES2001220, TBAES2001229, TBAES2001258, TBAES2001492, TBAES2001751, TBAES2002197, TBAES2003550, TBAES2004055, TBAES2005157, TBAES2005543, TBAES2006568, TBAES2007964, TESTI4000014, TESTI4037156, TRACH3002192, TRACH3004068, TSTOM2000553, UTERU2002410
BRAWH2006395, NT2RI2009583, STOMA2004893, TBAES2000932

The result of comparative analysis of cDNA libraries derived from cervical tumor (TCERX) and normal cervical duct (CERVX) (Table 11) showed that the genes whose expression levels differed between the two were twelve and two clones as described below.
BLADE2007666, BRAMY2047420, BRCAN2007409, BRSTN2016470, CERVX1000042, CERVX2002006, MESAN2006563, PROST2018090, TCERX2000613, TESTI4037156, THYMU2031341, UTERU2004688 CERVX2002013, NT2RI2009583

The result of comparative analysis of cDNA libraries derived from colon tumor (TCOLN) and normal colon (COLON) (Table 12) showed that the genes whose expression levels were different between the two were 24 and four clones as described below.
BRACE3015027, BRAMY2040592, BRSTN2016470, COLON1000030, COLON2000470, COLON2000568, COLON2001721, COLON2002443, COLON2002520, COLON2003043, COLON2004478, COLON2005126, COLON2005772, COLON2006282, COLON2009499, OCBBF2028935, PLACE7000514, RECTM2000433, SYNOV4007671, TCOLN2002278, TESTI2052693, TESTI4037156, THYMU2031368, TRACH2025535 CTONG1000113, NT2RI2009583, NT2RI2018448, TESTI2015626

The result of comparative analysis of cDNA libraries derived from esophageal tumor (TESOP) and normal esophagus (NESOP) (Table 13) showed that the genes whose expression levels were different between the two were 56 and ten clones as described below.
BRACE2030341, BRAMY2047420, BRHIP2003917, BRTHA2003461, CTONG2013178, D3OST3000169, FEBRA2025427, HCHON2000244, HHDPC1000118, NESOP2000744, NESOP2001433, NESOP2001656, NESOP2001694, NESOP2001752, NESOP2002738, NT2RI3006284, NT2RP7009147, PLACE6019932, SYNOV2005216, TESOP1000127, TESOP2000801, TESOP2001122, TESOP2001166, TESOP2001345, TESOP2001605, TESOP2001818, TESOP2001849, TESOP2001865, TESOP2001953, TESOP2002273, TESOP2002451, TESOP2002489, TESOP2002539, TESOP2002950, TESOP2003273, TESOP2003753, TESOP2004114, TESOP2005285, TESOP2005485, TESOP2005579, TESOP2006041, TESOP2006060, TESOP2006068, TESOP2006670, TESOP2006746, TESOP2007052, TESOP2007262, TESOP2007636, TESOP2007688, TESOP2009121, TESOP2009555, TESTI4009286, TESTI4010851, THYMU2040975, TRACH2005811, UTERU2023175 CTONG2016942, NT2RI2009583, TESOP2000390, TESOP2001796, TESOP2005199, TESOP2006398, TESOP2006865, TESOP2007384, TESTI2015626, TRACH2000862

The result of comparative analysis of cDNA libraries derived from kidney tumor (TKIDN) and normal kidney (KIDNE) (Table 14) showed that the genes whose expression levels were different between the two were 96 and 13 clones as described below.
ASTRO2018373, BRACE1000186, BRACE2014306, BRACE2015058, BRACE2016981, BRACE2043665, BRACE3008036, BRACE3010428, BRACE3022769, BRAMY2019963, BRAMY2044078, BRAWH1000127, BRAWH2001395, BRAWH2001671, BRAWH2013294, BRAWH2014645, BRHIP2024146, BRHIP3000339, BRSSN2000684, BRSSN2004719, BRSSN2018581, BRSTN2016470, BRTHA1000311, BRTHA3002427, CTONG1000087, CTONG2028124, CTONG3000657, CTONG3008894, FCBBF2001183, FEBRA2008287, HCASM2001301, HCHON2000028, HCHON2000244, HEART1000074, HHDPC1000118, HSYRA2008376, KIDNE1000064, KIDNE2000665, KIDNE2000722, KIDNE2000832, KIDNE2000846, KIDNE2001361, KIDNE2001847, KIDNE2002252, KIDNE2002991, KIDNE2003837, KIDNE2005543, KIDNE2006580, KIDNE2010264, KIDNE2011314, KIDNE2011532, KIDNE2011635, KIDNE2012945, KIDNE2013095, NESOP2001656, NTONG2005969, PEBLM2004666, SKMUS2000757, STOMA1000189, SYNOV4007671, TBAES2001258, TESTI4000014, TESTI4001100, TESTI4012702, TESTI4046819, THYMU2032014, TKIDN2000701, TKIDN2002424, TKIDN2002632, TKIDN2003044, TKIDN2004386, TKIDN2005934, TKIDN2005947, TKIDN2006525, TKIDN2006852, TKIDN2007667, TKIDN2009092, TKIDN2009641, TKIDN2009889, TKIDN2010934, TKIDN2012824, TKIDN2013287, TKIDN2014757, TKIDN2014771, TKIDN2015263, TKIDN2015788, TKIDN2016309, TKIDN2019116, TRACH2001443, TRACH2001684, TRACH2007834, TRACH2008300, TRACH3001427, UTERU2002410, UTERU2023175, UTERU3001572 BLADE2006830, BRALZ2017844, CTONG2028758, FCBBF1000509, FEBRA2001990, FEBRA2028516, HCHON2000508, MESAN2005303, NT2RI2009583, TESTI2015626, TKIDN2008778, TKIDN2012771, TKIDN2018926

The result of comparative analysis of cDNA libraries derived from liver tumor (TLIVE) and normal liver (LIVER) (Table 15) showed that the genes whose expression levels were different between the two were 35 and six clones as described below.
BRCAN2018935, BRSTN2016470, BRTHA2012980, BRTHA3002427, CTONG2028124, LIVER2007415, NT2RI2008724, SPLEN2012624, SPLEN2033098, TESOP2002451, TLIVE2000023, TLIVE2001327, TLIVE2001828, TLIVE2001927, TLIVE2002336, TLIVE2002338, TLIVE2002690, TLIVE2003197, TLIVE2003225, TLIVE2003381, TLIVE2003970, TLIVE2004110, TLIVE2004320, TLIVE2004601, TLIVE2005180, TLIVE2006236, TLIVE2006529, TLIVE2007132, TLIVE2007528, TLIVE2007816, TLIVE2008083, TLIVE2008229, TLIVE2009541, UTERU2002410, UTERU2005621
LIVER2000247, NT2RI2009583, TESTI2015626, TLIVE2001684, TLIVE2002046, TLIVE2007607

The result of comparative analysis of cDNA libraries derived from lung tumor (TLUNG) and normal lung (HLUNG) (Table 16) showed that the genes whose expression levels were different between the two were 47 and nine clones as described below.
BRCAN2021028, BRHIP2000819, BRSTN2016470, CTONG1000087, CTONG2028124, HCHON2006250, HEART1000074, HLUNG1000017, HLUNG2000014, HLUNG2001996, HLUNG2002465, HLUNG2002958, HLUNG2003003, HLUNG2003872, HLUNG2010464, HLUNG2011041, HLUNG2011298, HLUNG2012049, HLUNG2012287, HLUNG2012727, HLUNG2013204, HLUNG2013304, HLUNG2013622, HLUNG2013851, HLUNG2014262, HLUNG2014288, HLUNG2014449, HLUNG2015617, HLUNG2017350, HLUNG2017546, HLUNG2017806, HLUNG2019058, HSYRA2008376, KIDNE2012945, NT2RI2003993, NT2RP7013795, OCBBF3000483, SPLEN2028914, SPLEN2031547, SYNOV4007671, TESOP1000127, TESTI2003573, TESTI4000014, TESTI4037156, TRACH2005811, TRACH3004068, UTERU2005621
FEBRA2028516, HCHON2000508, HLUNG2013350, HLUNG2015418, HLUNG2015548, HLUNG2016862, NT2RI2009583, TESTI2015626, TRACH2019672

The result of comparative analysis of cDNA libraries derived from ovary tumor (TOVER) and normal ovary (NOVER) (Table 17A) showed the genes whose expression levels were different between the two were 16 clones as described below.
CTONG2019788, FEBRA2014213, HLUNG2017546, NOVAR2000136, NOVAR2000710, NOVAR2000962, NOVAR2001108, NOVAR2001783, OCBBF3007516, TESTI2052693, TOVAR2000649, TOVAR2001281, TOVAR2001730, TOVAR2002247, TOVAR2002549, TRACH3004068

The result of comparative analysis of cDNA libraries derived from ovary tumor (TOVER) and normal ovary (NOVER) (Table 17B) showed the gene whose expression levels were different between the two was one clone as described below. The gene has no different expression levels between normal and diseased ovary. However, the gene showed significantly different expression level in both ovary tumor and normal ovary, compared with other tissues. Thus, the gene are ovary-specific gene and can be used as diagnostic marker because its association with the disease.
TESTI2015626

The result of comparative analysis of cDNA libraries derived from stomach tumor (TSTOM) and normal stomach (STOMA) (Table 18) showed that the genes whose expression levels were different between the two were 31 and five clones as described below.
BRACE2024627, BRAWH2014645, BRCAN2028355, BRHIP2000819, BRSTN2016470, BRTHA3003490, COLON2002443, HEART1000010, HLUNG2002465, KIDNE2001847, NT2RP7000466, PUAEN2006328, SMINT2001818, STOMA1000189, STOMA2003444, STOMA2004294, STOMA2004925, STOMA2008546, SYNOV4007671, TESTI4000014, TESTI4010851, THYMU2035735, TRACH2001549, TRACH2005811, TRACH2025535, TSTOM1000135, TSTOM2000442, TSTOM2000553, TSTOM2002672, UTERU2006115, UTERU3001572

The following five clones also had different expression levels between the two.
FEBRA2008692, NT2RI2009583, STOMA2003158, STOMA2004893, TESTI2015626

The result of comparative analysis of cDNA libraries derived from uterine tumor (TUTER) and normal uterus (UTERU) (Table 19) showed that the genes whose expression levels were different between the two were 244 and 34 clones as described below.
BNGH42007788, BRACE1000186, BRACE2030341, BRACE3008772, BRACE3009747, BRACE3010428, BRACE3027478, BRALZ2017359, BRAWH2014645, BRAWH3000314, BRAWH3001326, BRAWH3002574, BRAWH3002821, BRAWH3003727, BRAWH3007592, BRCAN2009432, BRCAN2028355, BRHIP3007586, BRHIP3008344, BRHIP3008565, BRSSN2006892, BRSTN2001067, BRSTN2016470, BRTHA2010608, BRTHA3003074, CTONG1000087, CTONG1000467, CTONG2028124, CTONG3001123, CTONG3008894, CTONG3009028, CTONG3009239, FCBBF3004847, FEBRA2026984, FEBRA2028618, HCHON2000244, HCHON2000418, HCHON2000626, HCHON2001084, HCHON2001217, HCHON2005921, HCHON2006250, HCHON2008444, HLUNG2003003, HSYRA2008376, KIDNE2002252, MESAN2014295, NOVAR2000710, NT2RI2008724, NT2RI2014247, NT2RI2014733, NT2RI3002892, NT2RI3005724, NT2RI3006284, NT2RI3006340, NT2RI3006673, NT2RI3007291, NT2RI3007543, NT2RP7004123, NT2RP7005529, NT2RP7009147, NT2RP7017474, OCBBF2007028, OCBBF2020741, OCBBF2024850, OCBBF2036743, OCBBF3000483, PLACE6001185, PLACE7000514, PUAEN2007044, PUAEN2009655, SKNSH2000482, SPLEN2006122, SPLEN2016554, SPLEN2031547, SPLEN2036932, STOMA1000189, STOMA2004925, SYNOV2017055, SYNOV4001395, SYNOV4002346, SYNOV4008440, TCERX2000613, TESOP2002273, TESTI4000014, TESTI4008797, TESTI4009286, TESTI4012702, TESTI4013675, TESTI4014159, TESTI4018886, TESTI4029671, TESTI4037156, THYMU2008725, THYMU2031890, THYMU2033070, THYMU2035735, THYMU3001472, TRACH1000205, TRACH2001443, TRACH2001549, TRACH2005811, TRACH2007834, TRACH2008300, TRACH3002192, TRACH3003379, TRACH3004068, TRACH3004721, TRACH3007479, TUTER1000122, TUTER2000425, TUTER2000904, TUTER2000916, TUTER2001387, TUTER2002729, UTERU1000024, UTERU1000031, UTERU1000148, UTERU1000249, UTERU1000337, UTERU1000339, UTERU2000649, UTERU2001409, UTERU2002410, UTERU2002841, UTERU2004688, UTERU2004929, UTERU2005004, UTERU2005621, UTERU2006115, UTERU2006137, UTERU2006568, UTERU2007444, UTERU2007520, UTERU2007724, UTERU2008347, UTERU2014678, UTERU2017762, UTERU2019491, UTERU2019681, UTERU2019706, UTERU2019940, UTERU2020491, UTERU2020718, UTERU2021163, UTERU2021380, UTERU2022020, UTERU2022981, UTERU2023039, UTERU2023175, UTERU2023651, UTERU2023712, UTERU2024002, UTERU2024656, UTERU2025025, UTERU2025645, UTERU2025891, UTERU2026025, UTERU2026090, UTERU2026203, UTERU2027591, UTERU2029953, UTERU2030213, UTERU2030280, UTERU2031084, UTERU2031268, UTERU2031521, UTERU2031703, UTERU2031851, UTERU2033375, UTERU2033382, UTERU2035114, UTERU2035323, UTERU2035328, UTERU2035331, UTERU2035452, UTERU2035469, UTERU2035503, UTERU2035745, UTERU2036089, UTERU2037361, UTERU2037577, UTERU2038251, UTERU3000226, UTERU3000645, UTERU3000665, UTERU3000828, UTERU3000899, UTERU3001059, UTERU3001240, UTERU3001542, UTERU3001571, UTERU3001572, UTERU3001585, UTERU3001652, UTERU3001766, UTERU3001988, UTERU3002209, UTERU3002218, UTERU3002383, UTERU3002667, UTERU3002731, UTERU3002768, UTERU3002786, UTERU3002993, UTERU3003116, UTERU3003135, UTERU3003178, UTERU3003465, UTERU3003523, UTERU3003776, UTERU3004523, UTERU3004616, UTERU3004709, UTERU3004992, UTERU3005049, UTERU3005205, UTERU3005230, UTERU3005460, UTERU3005585, UTERU3005907, UTERU3005970, UTERU3006008, UTERU3006308, UTERU3007134, UTERU3007419, UTERU3007640, UTERU3007913, UTERU3008660, UTERU3008671, UTERU3009259, UTERU3009490, UTERU3009517, UTERU3009690, UTERU3009871, UTERU3009979, UTERU3011063, UTERU3015086, UTERU3015500, UTERU3016789, UTERU3018081, UTERU3018154, UTERU3018616, UTERU3018711 ADRGL2000042, BRHIP3000017, CTONG2003348, CTONG2019822, CTONG2020378, CTONG2020411, CTONG2024031, FEBRA2028516, HCASM2008536, HCHON2000743, IMR322001879, MESAN2005303, NT2RI2009583, OCBBF2008144, PERIC2007068, SPLEN2039379, TESTI2015626, TESTI4013894, TUTER2000057, UTERU2004299, UTERU2008040, UTERU2011220, UTERU2019534, UTERU2021820, UTERU2028734, UTERU2032279, UTERU2033577, UTERU2035978, UTERU3000402, UTERU3000738, UTERU3001053, UTERU3014791, UTERU3015412, UTERU3017176

The result of comparative analysis of cDNA libraries derived from tongue cancer (CTONG) and normal tongue (NTONG) (Table 20) showed that the genes whose expression levels were different between the two were 166 and 31 clones as described below.
BNGH42007788, BRACE1000186, BRACE2006319, BRACE3010428, BRACE3012364, BRAMY2020058, BRAMY3002803, BRAWH2001671, BRAWH2014645, BRAWH3002574, BRCAN2009432, BRCAN2015371, BRCAN2020710, BRHIP2004814, BRHIP3018797, BRTHA2003461, BRTHA3003490, CTONG1000087, CTONG1000088, CTONG1000288, CTONG1000302, CTONG1000341, CTONG1000467, CTONG1000488, CTONG1000508, CTONG1000540, CTONG2000042, CTONG2001877, CTONG2004062, CTONG2006798, CTONG2008233, CTONG2009423, CTONG2009531, CTONG2010803, CTONG2013178, CTONG2017500, CTONG2019248, CTONG2019652, CTONG2019704, CTONG2019788, CTONG2019833, CTONG2020127, CTONG2020522, CTONG2020638, CTONG2020806, CTONG2021132, CTONG2022153, CTONG2022601, CTONG2023021, CTONG2023512, CTONG2024206, CTONG2024749, CTONG2025496, CTONG2025516, CTONG2025900, CTONG2026920, CTONG2027327, CTONG2028124, CTONG2028687, CTONG3000084, CTONG3000657, CTONG3000686, CTONG3000707, CTONG3000896, CTONG3001123, CTONG3001370, CTONG3001420, CTONG3001560, CTONG3002020, CTONG3002127, CTONG3002412, CTONG3002674, CTONG3003179, CTONG3003483, CTONG3003652, CTONG3003654, CTONG3003737, CTONG3003905, CTONG3003972, CTONG3004072, CTONG3004712, CTONG3005325, CTONG3005648, CTONG3005713, CTONG3005813, CTONG3006067, CTONG3006186, CTONG3006650, CTONG3007444, CTONG3007528, CTONG3007586, CTONG3007870, CTONG3008252, CTONG3008258, CTONG3008496, CTONG3008566, CTONG3008639, CTONG3008831, CTONG3008894, CTONG3008951, CTONG3009028, CTONG3009227, CTONG3009239, CTONG3009328, CTONG3009385, FEBRA2007544, FEBRA2007801, FEBRA2021966, FEBRA2025427, HCHON2000028, HCHON2001217, HHDPC1000118, HSYRA2008376, KIDNE2001847, KIDNE2002252, MESAN2006563, NT2RI2008724, NT2RI2018883, NT2RI3000622, NT2RI3006284, NT2RI3006673, NT2RI3007543, NT2RI3007757, NT2RP7004123, NT2RP7009147, NT2RP7014005, NTONG2000413, NTONG2003852, NTONG2005277, NTONG2005969, NTONG2006354, NTONG2007249, NTONG2007517, NTONG2008088, NTONG2008672, OCBBF2001794, OCBBF2006151, PEBLM2004666, PEBLM2005183, SPLEN2002467, SPLEN2029912, SPLEN2031547, SYNOV4007671, SYNOV4008440, TBAES2002197, TESOP2002273, TESTI2009474, TESTI4000014, TESTI4000209, TESTI4008018, TESTI4009286, TESTI4010851, TESTI4012702, TESTI4013675, THYMU2031847, THYMU2033308, TLIVE2002690, TRACH2005811, TRACH2007059, TRACH2025535, TRACH3001427, TSTOM2000553, UTERU2005621, UTERU2017762, UTERU2023175, UTERU3001572
BLADE2006830, BRHIP3000017, CTONG1000113, CTONG2003348, CTONG2004000, CTONG2008721, CTONG2015596, CTONG2015633, CTONG2016942, CTONG2019822, CTONG2020374, CTONG2020378, CTONG2020411, CTONG2020974, CTONG2024031, CTONG2028758, CTONG3001501, CTONG3002552, CTONG3003598, CTONG3004550, CTONG3004726, CTONG3009287, FEBRA2008692, FEBRA2028516, HCHON2000508, NT2RI2009583, NTONG2008093, PERIC2007068, TESOP2007384, TLIVE2002046, TRACH2000862

These genes are involved in cancer.

Further, there is a method to search for genes involved in development and differentiation, which is the expression frequency analysis in which the expression levels of genes are compared between developing and/or differentiating tissues and/or cells and adult tissues and/or cells. The genes involved in tissue development and/or differentiation are genes participating in tissue construction and expression of function, and thus are useful genes, which are available for regenerative medicine aiming at convenient regeneration of injured tissues.

By using the information of gene expression frequency gained from the database of nucleotide sequences of 1,402,069 clones as described above, genes whose expression frequencies were different between developing and/or differentiating tissues and/or cells and adult tissues and/or cells were analyzed.

The result of comparative analysis of cDNA libraries derived from fetal brain (FCBBF, FEBRA or OCBBF) and adult brain (BRACE, BRALZ, BRAMY, BRAWH, BRCAN, BRCOC, BRHIP, BRSSN, BRSTN or BRTHA) (Table 21) showed that the genes whose expression levels were different between the two were 1,035 and 139 clones as described below.
ADRGL2009146, ADRGL2012038, ADRGL2012179, ASTRO1000009, ASTRO2003960, ASTRO3000482, BLADE1000176, BLADE2001371, BLADE2004089, BLADE2008398, BNGH42007788, BRACE1000186, BRACE1000258, BRACE1000533, BRACE1000572, BRACE2003639, BRACE2005457, BRACE2006319, BRACE2008594, BRACE2010489, BRACE2011747, BRACE2014306, BRACE2014475, BRACE2014657, BRACE2015058, BRACE2015314, BRACE2016981, BRACE2018762, BRACE2024627, BRACE2026836, BRACE2027258, BRACE2027970, BRACE2028970, BRACE2029112, BRACE2029849, BRACE2030326, BRACE2030341, BRACE2030884, BRACE2031154, BRACE2031389, BRACE2031527, BRACE2031531, BRACE2031899, BRACE2032044, BRACE2032329, BRACE2032385, BRACE2032538, BRACE2032823, BRACE2033720, BRACE2035381, BRACE2035441, BRACE2036005, BRACE2036096, BRACE2036830, BRACE2036834, BRACE2037847, BRACE2038114, BRACE2038329, BRACE2038551, BRACE2039249, BRACE2039327, BRACE2039475, BRACE2039734, BRACE2040138, BRACE2040325, BRACE2041009, BRACE2041200, BRACE2041264, BRACE2042550, BRACE2043142, BRACE2043248, BRACE2043349, BRACE2043665, BRACE2044286, BRACE2044816, BRACE2044949, BRACE2045300, BRACE2045428, BRACE2045596, BRACE2045772, BRACE2045947, BRACE2045954, BRACE2046251, BRACE2046295, BRACE2047011, BRACE2047350, BRACE2047377, BRACE2047385, BRACE3000071, BRACE3000697, BRACE3000787, BRACE3000840, BRACE3000973, BRACE3001002, BRACE3001217, BRACE3001391, BRACE3001595, BRACE3001754, BRACE3002298, BRACE3002390, BRACE3002508, BRACE3003004, BRACE3003192, BRACE3003595, BRACE3003698, BRACE3004058, BRACE3004113, BRACE3004150, BRACE3004358, BRACE3004435, BRACE3004772, BRACE3004783, BRACE3004843, BRACE3004880, BRACE3005145, BRACE3005225, BRACE3005430, BRACE3005499, BRACE3006185, BRACE3006226, BRACE3006462, BRACE3006872, BRACE3007322, BRACE3007472, BRACE3007480, BRACE3007559, BRACE3007625, BRACE3007642, BRACE3007767, BRACE3008036, BRACE3008092, BRACE3008137, BRACE3008384, BRACE3008720, BRACE3008772, BRACE3009090, BRACE3009237, BRACE3009297, BRACE3009377, BRACE3009574, BRACE3009701, BRACE3009708, BRACE3009724, BRACE3009747, BRACE3010397, BRACE3010428, BRACE3011271, BRACE3011421, BRACE3011505, BRACE3012364, BRACE3012930, BRACE3013119, BRACE3013576, BRACE3013740, BRACE3013780, BRACE3014005, BRACE3014068, BRACE3014231, BRACE3014317, BRACE3014807, BRACE3015027, BRACE3015121, BRACE3015262, BRACE3015521, BRACE3015894, BRACE3016884, BRACE3018308, BRACE3018963, BRACE3019055, BRACE3019084, BRACE3020194, BRACE3020286, BRACE3020594, BRACE3022769, BRACE3023912, BRACE3024073, BRACE3024659, BRACE3024662, BRACE3025153, BRACE3025457, BRACE3025531, BRACE3025630, BRACE3026008, BRACE3026075, BRACE3026735, BRACE3027242, BRACE3027326, BRACE3027478, BRACE3030103, BRACE3031838, BRACE3032983, BRACE3040856, BRACE3045033, BRALZ2011796, BRALZ2012183, BRALZ2012848, BRALZ2014484, BRALZ2016085, BRALZ2016498, BRALZ2017359, BRAMY2001473, BRAMY2003008, BRAMY2004771, BRAMY2005052, BRAMY2017528, BRAMY2019300, BRAMY2019963, BRAMY2019985, BRAMY2020058, BRAMY2020270, BRAMY2021498, BRAMY2028856, BRAMY2028914, BRAMY2029602, BRAMY2030098, BRAMY2030109, BRAMY2030702, BRAMY2030703, BRAMY2030799, BRAMY2031317, BRAMY2031377, BRAMY2031442, BRAMY2032014, BRAMY2032242, BRAMY2032317, BRAMY2033003, BRAMY2033116, BRAMY2033267, BRAMY2033594, BRAMY2034185, BRAMY2034920, BRAMY2034993, BRAMY2036387, BRAMY2036396, BRAMY2036567, BRAMY2036699, BRAMY2036913, BRAMY2037823, BRAMY2038100, BRAMY2038484, BRAMY2038846, BRAMY2038904, BRAMY2039872, BRAMY2040478, BRAMY2040592, BRAMY2041261, BRAMY2041378, BRAMY2041542, BRAMY2042612, BRAMY2042641, BRAMY2042760, BRAMY2042918, BRAMY2044078, BRAMY2044246, BRAMY2045036, BRAMY2046478, BRAMY2046742, BRAMY2046989, BRAMY2047169, BRAMY2047420, BRAMY2047676, BRAMY2047746, BRAMY2047751, BRAMY2047765, BRAMY2047884, BRAMY3000206, BRAMY3000213, BRAMY3001401, BRAMY3001794, BRAMY3002312, BRAMY3002620, BRAMY3002803, BRAMY3002805, BRAMY3004224, BRAMY3004672, BRAMY3004900, BRAMY3004919, BRAMY3005091, BRAMY3005932, BRAMY3006297, BRAMY3007206, BRAMY3007609, BRAMY3008466, BRAMY3008505, BRAMY3008650, BRAMY3009811, BRAMY3010411, BRAMY4000095, BRAMY4000229, BRAMY4000277, BRASW1000125, BRAWH1000127, BRAWH2001395, BRAWH2001671, BRAWH2001940, BRAWH2001973, BRAWH2002560, BRAWH2002761, BRAWH2005315, BRAWH2007658, BRAWH2010000, BRAWH2010084, BRAWH2010536, BRAWH2012162, BRAWH2012326, BRAWH2013294, BRAWH2013871, BRAWH2014414, BRAWH2014645, BRAWH2014662, BRAWH2014876, BRAWH2014954, BRAWH2016221, BRAWH2016439, BRAWH2016702, BRAWH2016724, BRAWH3000078, BRAWH3000100, BRAWH3000314, BRAWH3000491, BRAWH3001326, BRAWH3001475, BRAWH3001891, BRAWH3002574, BRAWH3002600, BRAWH3002819, BRAWH3002821, BRAWH3003522, BRAWH3003555, BRAWH3003727, BRAWH3003801, BRAWH3003992, BRAWH3004453, BRAWH3004666, BRAWH3005132, BRAWH3005422, BRAWH3005912, BRAWH3005981, BRAWH3006548, BRAWH3006792, BRAWH3007221, BRAWH3007506, BRAWH3007592, BRAWH3007726, BRAWH3007783, BRAWH3008341, BRAWH3008697, BRAWH3008931, BRAWH3009297, BRCAN2002562, BRCAN2002856, BRCAN2002944, BRCAN2002948, BRCAN2003703, BRCAN2003746, BRCAN2003987, BRCAN2004355, BRCAN2005436, BRCAN2006063, BRCAN2006290, BRCAN2006297, BRCAN2006450, BRCAN2007144, BRCAN2007409, BRCAN2007426, BRCAN2008528, BRCAN2009203, BRCAN2009432, BRCAN2010376, BRCAN2011254, BRCAN2011602, BRCAN2012355, BRCAN2012481, BRCAN2013655, BRCAN2013660, BRCAN2014143, BRCAN2014602, BRCAN2014881, BRCAN2015371, BRCAN2015464, BRCAN2016433, BRCAN2016619, BRCAN2017442, BRCAN2017717, BRCAN2017905, BRCAN2018935, BRCAN2019387, BRCAN2020710, BRCAN2021028, BRCAN2024451, BRCAN2024563, BRCAN2025712, BRCAN2028355, BRCOC2000670, BRCOC2003213, BRCOC2007034, BRCOC2014033, BRCOC2016525, BRCOC2019934, BRCOC2020142, BRHIP2000691, BRHIP2000819, BRHIP2000826, BRHIP2000920, BRHIP2001074, BRHIP2001805, BRHIP2001927, BRHIP2002122, BRHIP2002172, BRHIP2002346, BRHIP2003242, BRHIP2003786, BRHIP2003917, BRHIP2004312, BRHIP2004359, BRHIP2004814, BRHIP2004883, BRHIP2005236, BRHIP2005354, BRHIP2005600, BRHIP2005719, BRHIP2005752, BRHIP2005932, BRHIP2006800, BRHIP2007616, BRHIP2007741, BRHIP2009340, BRHIP2009414, BRHIP2009474, BRHIP2013699, BRHIP2014228, BRHIP2021615, BRHIP2022221, BRHIP2024146, BRHIP2024165, BRHIP2026061, BRHIP2026288, BRHIP2029176, BRHIP2029393, BRHIP3000339, BRHIP3000526, BRHIP3001283, BRHIP3006683, BRHIP3007483, BRHIP3007586, BRHIP3008183, BRHIP3008313, BRHIP3008344, BRHIP3008405, BRHIP3008565, BRHIP3008598, BRHIP3008997, BRHIP3009099, BRHIP3009448, BRHIP3011241, BRHIP3013765, BRHIP3013897, BRHIP3015751, BRHIP3016213, BRHIP3018797, BRHIP3020182, BRHIP3024118, BRHIP3024533, BRHIP3024725, BRHIP3025161, BRHIP3025702, BRHIP3026097, BRHIP3027137, BRHIP3027854, BRSSN2000684, BRSSN2003086, BRSSN2004496, BRSSN2004719, BRSSN2006892, BRSSN2008549, BRSSN2008797, BRSSN2011262, BRSSN2011738, BRSSN2013874, BRSSN2014299, BRSSN2014424, BRSSN2014556, BRSSN2018581, BRSSN2018925, BRSTN2000872, BRSTN2001067, BRSTN2001613, BRSTN2002400, BRSTN2003835, BRSTN2004863, BRSTN2004987, BRSTN2005721, BRSTN2006865, BRSTN2007000, BRSTN2007284, BRSTN2008052, BRSTN2008283, BRSTN2008418, BRSTN2008457, BRSTN2010363, BRSTN2010500, BRSTN2010750, BRSTN2012320, BRSTN2012380, BRSTN2015015, BRSTN2016470, BRSTN2016678, BRSTN2017237, BRSTN2017771, BRSTN2018083, BRSTN2019129, BRTHA1000311, BRTHA2000855, BRTHA2001462, BRTHA2002115, BRTHA2002281, BRTHA2002376, BRTHA2002442, BRTHA2002493, BRTHA2002608, BRTHA2002808, BRTHA2003030, BRTHA2003110, BRTHA2003116, BRTHA2003461, BRTHA2004821, BRTHA2004978, BRTHA2005579, BRTHA2005956, BRTHA2006075, BRTHA2006146, BRTHA2006194, BRTHA2007122, BRTHA2007422, BRTHA2007603, BRTHA2008316, BRTHA2008335, BRTHA2008527, BRTHA2008535, BRTHA2008955, BRTHA2009311, BRTHA2009846, BRTHA2009972, BRTHA2010073, BRTHA2010608, BRTHA2010884, BRTHA2010907, BRTHA2011194, BRTHA2011351, BRTHA2011500, BRTHA2011641, BRTHA2012392, BRTHA2012562, BRTHA2012980, BRTHA2013262, BRTHA2013460, BRTHA2013707, BRTHA2014792, BRTHA2014828, BRTHA2015406, BRTHA2015478, BRTHA2015696, BRTHA2015878, BRTHA2016215, BRTHA2016496, BRTHA2016543, BRTHA2017353, BRTHA2017985, BRTHA2018165, BRTHA2018344, BRTHA2018591, BRTHA2018624, BRTHA2018707, BRTHA2019014, BRTHA2019022, BRTHA2019048, BRTHA3000273, BRTHA3000297, BRTHA3000633, BRTHA3001721, BRTHA3002401, BRTHA3002427, BRTHA3002933, BRTHA3003074, BRTHA3003343, BRTHA3003449, BRTHA3003474, BRTHA3003490, BRTHA3004475, BRTHA3005046, BRTHA3006856, BRTHA3007113, BRTHA3007148, BRTHA3007319, BRTHA3007769, BRTHA3008143, BRTHA3008310, BRTHA3008386, BRTHA3008520, BRTHA3008778, BRTHA3009037, BRTHA3009090, BRTHA3009291, BRTHA3010366, BRTHA3013884, BRTHA3015815, BRTHA3015910, BRTHA3016845, BRTHA3016917, BRTHA3017047, BRTHA3017589, BRTHA3017848, BRTHA3018514, BRTHA3018617, BRTHA3018656, BRTHA3019105, COLON2001721, CTONG1000087, CTONG1000088, CTONG1000467, CTONG2000042, CTONG2008233, CTONG2009423, CTONG2017500, CTONG2019248, CTONG2019788, CTONG2020522, CTONG2023021, CTONG2028124, CTONG3000657, CTONG3001123, CTONG3001370, CTONG3002412, CTONG3004072, CTONG3005813, CTONG3008894, CTONG3009028, CTONG3009239, CTONG3009328, DFNES2000146, DFNES2011239, DFNES2011499, FCBBF1000297, FCBBF2001183, FCBBF3001977, FCBBF3002163, FCBBF3003435, FCBBF3004502, FCBBF3004847, FCBBF3006171, FCBBF3007242, FCBBF3007540, FCBBF3008944, FCBBF3009888, FCBBF3012170, FCBBF3012288, FCBBF3013307, FCBBF3013846, FCBBF3021576, FCBBF3021940, FCBBF3023443, FCBBF3023895, FCBBF3025730, FCBBF3027717, FCBBF4000076, FEBRA1000030, FEBRA2000253, FEBRA2006396, FEBRA2007544, FEBRA2007708, FEBRA2007793, FEBRA2007801, FEBRA2008287, FEBRA2008311, FEBRA2008360, FEBRA2008468, FEBRA2010719, FEBRA2014213, FEBRA2015588, FEBRA2020484, FEBRA2020582, FEBRA2020668, FEBRA2020886, FEBRA2021339, FEBRA2021571, FEBRA2021908, FEBRA2021966, FEBRA2024136, FEBRA2024150, FEBRA2024343, FEBRA2024744, FEBRA2025427, FEBRA2026984, FEBRA2027082, FEBRA2027297, FEBRA2027352, FEBRA2028366, FEBRA2028477, FEBRA2028618, HCASM2007047, HCHON2000028, HCHON2000212, HCHON2000244, HCHON2000626, HCHON2001084, HCHON2001217, HCHON2002676, HCHON2005921, HCHON2006250, HCHON2007881, HCHON2008112, HEART1000074, HEART2007031, HHDPC1000118, HLUNG2001996, HLUNG2002465, HLUNG2003003, HSYRA2009075, IMR322000127, IMR322000917, IMR322001380, IMR322002035, KIDNE2000665, KIDNE2002252, KIDNE2005543, KIDNE2006580, KIDNE2011314, MESAN2006563, MESAN2012054, MESAN2015515, MESTC1000042, NB9N41000340, NESOP2001752, NHNPC2001223, NOVAR2001783, NT2NE2005890, NT2NE2006909, NT2NE2008060, NT2RI2003993, NT2RI2005166, NT2RI2008724, NT2RI2012659, NT2RI2014733, NT2RI2018311, NT2RI2019751, NT2RI3000622, NT2RI3001515, NT2RI3002842, NT2RI3002892, NT2RI3003382, NT2RI3004510, NT2RI3005403, NT2RI3005724, NT2RI3006284, NT2RI3006673, NT2RI3007291, NT2RI3007543, NT2RI3008055, NT2RP7004123, NT2RP7005529, NT2RP7009030, NT2RP7009147, NT2RP7010599, NT2RP7014005, NT2RP7015512, NT2RP7017474, NTONG2000413, NTONG2005969, NTONG2008088, OCBBF1000254, OCBBF2001794, OCBBF2002124, OCBBF2003819, OCBBF2004826, OCBBF2004883, OCBBF2005428, OCBBF2006005, OCBBF2006058, OCBBF2006151, OCBBF2006567, OCBBF2006764, OCBBF2007028, OCBBF2007068, OCBBF2007114, OCBBF2007428, OCBBF2007478, OCBBF2007610, OCBBF2008770, OCBBF2009788, OCBBF2009926, OCBBF2010140, OCBBF2010416, OCBBF2017516, OCBBF2019327, OCBBF2019823, OCBBF2020343, OCBBF2020453, OCBBF2020639, OCBBF2020741, OCBBF2020801, OCBBF2020838, OCBBF2021020, OCBBF2021286, OCBBF2021323, OCBBF2021788, OCBBF2022351, OCBBF2022574, OCBBF2023162, OCBBF2023643, OCBBF2024719, OCBBF2024781, OCBBF2024850, OCBBF2025028, OCBBF2025458, OCBBF2025527, OCBBF2025730, OCBBF2026645, OCBBF2027423, OCBBF2027478, OCBBF2028173, OCBBF2028935, OCBBF2029901, OCBBF2030354, OCBBF2030517, OCBBF2030574, OCBBF2030708, OCBBF2031167, OCBBF2031366, OCBBF2032590, OCBBF2032599, OCBBF2032611, OCBBF2032671, OCBBF2033869, OCBBF2035110, OCBBF2035214, OCBBF2035564, OCBBF2035885, OCBBF2035916, OCBBF2036476, OCBBF2036743, OCBBF2037068, OCBBF2037340, OCBBF2037398, OCBBF2037547, OCBBF2037598, OCBBF2037638, OCBBF2038317, OCBBF3000296, OCBBF3000483, OCBBF3002553, OCBBF3002600, OCBBF3003320, OCBBF3003592, OCBBF3004314, OCBBF3006802, OCBBF3007516, OCBBF3008230, OCBBF3009279, PEBLM2004666, PERIC2000889, PERIC2002766, PERIC2003720, PLACE6001185, PLACE6019385, PUAEN2002489, PUAEN2005930, PUAEN2006701, PUAEN2007044, PUAEN2009174, PUAEN2009655, RECTM2001347, SKMUS2000757, SKNMC2002402, SKNSH2000482, SMINT2001818, SPLEN2001599, SPLEN2002467, SPLEN2006122, SPLEN2010912, SPLEN2012624, SPLEN2025491, SPLEN2027268, SPLEN2028914, SPLEN2029912, SPLEN2031424, SPLEN2031547, SPLEN2032154, SPLEN2034781, SPLEN2036821, SPLEN2036932, SPLEN2037194, SPLEN2038345, SPLEN2042303, SYNOV1000374, SYNOV2005216, SYNOV2014400, SYNOV4002346, SYNOV4002883, SYNOV4007430, SYNOV4007671, SYNOV4008440, TESOP2001605, TESOP2002273, TESOP2002451, TESOP2002950, TESTI1000330, TESTI2003573, TESTI2009474, TESTI2049246, TESTI4000014, TESTI4000209, TESTI4000349, TESTI4001100, TESTI4001561, TESTI4002290, TESTI4002647, TESTI4005857, TESTI4006137, TESTI4006326, TESTI4008797, TESTI4009286, TESTI4010377, TESTI4010851, TESTI4010928, TESTI4011161, TESTI4012702, TESTI4013675, TESTI4013817, TESTI4014159, TESTI4014175, TESTI4014306, TESTI4014694, TESTI4014818, TESTI4019843, TESTI4021478, TESTI4022936, TESTI4024420, TESTI4027821, TESTI4029836, TESTI4037156, TESTI4037188, TESTI4046819, THYMU2001090, THYMU2011736, THYMU2014353, THYMU2016204, THYMU2016523, THYMU2023967, THYMU2025707, THYMU2030264, THYMU2031341, THYMU2031890, THYMU2032696, THYMU2032825, THYMU2033308, THYMU2033787, THYMU2034374, THYMU2035735, THYMU2037226, THYMU2039315, THYMU2039780, THYMU2040975, THYMU3001083, THYMU3001234, THYMU3001379, THYMU3003309, THYMU3004835, THYMU3006485, THYMU3007137, THYMU3008171, TKIDN2009641, TKIDN2009889, TKIDN2010934, TKIDN2013287, TKIDN2015788, TLIVE2001327, TLIVE2004320, TRACH1000205, TRACH2001443, TRACH2001549, TRACH2001684, TRACH2005811, TRACH2006049, TRACH2007834, TRACH2008300, TRACH2023299, TRACH2025344, TRACH2025535, TRACH2025911, TRACH3000014, TRACH3001427, TRACH3002192, TRACH3004068, TRACH3004721, TRACH3005294, TRACH3006038, TRACH3006412, TRACH3007479, TRACH3008093, TRACH3009455, TSTOM1000135, TUTER1000122, TUTER2000904, UTERU2002410, UTERU2004929, UTERU2005621, UTERU2006115, UTERU2007520, UTERU2014678, UTERU2019706, UTERU2019940, UTERU2021163, UTERU2023039, UTERU2023175, UTERU2026203, UTERU2030213, UTERU2030280, UTERU3000226, UTERU3000899, UTERU3001571, UTERU3001572, UTERU3001766, UTERU3003135, UTERU3004709, UTERU3005230, UTERU3005460, UTERU3005907, UTERU3005970, UTERU3006308, UTERU3007419, UTERU3007640, UTERU3007913, UTERU3009259, UTERU3009517, UTERU3009871
ADRGL2000042, BLADE2006830, BRACE2002589, BRACE2003609, BRACE2009318, BRACE2011677, BRACE2029396, BRACE2037299, BRACE2039823, BRACE2039832, BRACE2043105, BRACE3001058, BRACE3001113, BRACE3003026, BRACE3003053, BRACE3009127, BRACE3010076, BRACE3015829, BRACE3021148, BRALZ2017844, BRAMY2019111, BRAMY2035070, BRAMY2035449, BRAMY2035718, BRAMY2038516, BRAMY2039341, BRAMY2040159, BRAMY2041434, BRAMY2045471, BRAMY3004800, BRAWH1000369, BRAWH2006207, BRAWH2006395, BRAWH2008993, BRAWH2009393, BRAWH2010552, BRAWH3007441, BRAWH3009017, BRCAN2002473, BRCAN2002854, BRCAN2003070, BRCAN2014229, BRCOC2019841, BRHIP2002722, BRHIP2003272, BRHIP2005271, BRHIP2005724, BRHIP2006617, BRHIP2008389, BRHIP2012360, BRHIP2017553, BRHIP2026877, BRHIP3000017, BRHIP3000240, BRHIP3008314, BRHIP3026052, BRSTN2013354, BRTHA2002133, BRTHA2002702, BRTHA2007060, BRTHA2010033, BRTHA2011321, BRTHA2013426, BRTHA2013610, BRTHA2016318, BRTHA2017364, BRTHA2017972, BRTHA2018011, BRTHA2018443, BRTHA3000296, BRTHA3003000, BRTHA3008826, CTONG2008721, CTONG2020374, CTONG2020378, CTONG2020411, CTONG2024031, CTONG3004726, FCBBF1000509, FCBBF3010361, FCBBF3027854, FEBRA2000790, FEBRA2001990, FEBRA2006519, FEBRA2008692, FEBRA2014122, FEBRA2027609, FEBRA2028516, HCASM2003018, HCHON2000508, HCHON2000743, HCHON2004858, HSYRA2005628, IMR322001879, NT2RI2009583, NT2RP8000521, OCBBF2003327, OCBBF2005433, OCBBF2006987, OCBBF2008144, OCBBF2009583, OCBBF2011669, OCBBF2019684, OCBBF2020048, OCBBF2030116, OCBBF2032274, OCBBF2034637, OCBBF3002654, OCBBF3003761, OCBBF3004972, PERIC2007068, PUAEN2006335, SPLEN2016932, SPLEN2039379, SYNOV2006620, SYNOV2021953, TESTI1000266, TESTI2015626, TESTI2026647, TESTI4000214, TESTI4001984, TESTI4008058, TESTI4013894, TESTI4015442, TESTI4017714, TESTI4025268, TESTI4025547, TESTI4026207, TESTI4032090, THYMU2004284, THYMU2040925, THYMU3000360, TKIDN2018926, TLIVE2002046, TRACH3000134, UTERU2008040, UTERU2011220, UTERU2021820, UTERU2028734

The result of comparative analysis of cDNA libraries derived from fetal heart (FEHRT) and adult heart (HEART) (Table 22) showed that the genes whose expression levels were different between the two were 34 and two clones as described below.
BRAMY2040592, BRAWH2001671, BRSTN2016470, CTONG2017500, CTONG2028124, CTONG3000657, D3OST3000169, FEBRA2008287, HCHON2000244, HCHON2000626, HEART1000010, HEART1000074, HEART1000088, HEART1000139, HEART2001680, HEART2001756, HEART2006131, HEART2006909, HEART2007031, HEART2010391, HEART2010492, HEART2010495, KIDNE2000665, NB9N41000340, NT2RI2003993, NT2RI3002892, OCBBF2024850, SKMUS2006394, SMINT2001818, TESTI4000209, TKIDN2015788, TRACH3002192, TRACH3005294, TRACH3007479
HEART2009680, THYMU2004284

The result of comparative analysis of cDNA libraries derived from fetal kidney (FEKID) and adult kidney (KIDNE) (Table 23) showed that the genes whose expression levels were different between the two were 40 and two clones as described below.
BRACE2043665, BRACE3010428, BRSTN2016470, CTONG1000087, CTONG2028124, CTONG3008894, HCASM2003415, HCHON2000244, HEART1000074, HHDPC1000118, KIDNE1000064, KIDNE2000665, KIDNE2000722, KIDNE2000832, KIDNE2000846, KIDNE2001361, KIDNE2001847, KIDNE2002252, KIDNE2002991, KIDNE2003837, KIDNE2005543, KIDNE2006580, KIDNE2010264, KIDNE2011314, KIDNE2011532, KIDNE2011635, KIDNE2012945, KIDNE2013095, PEBLM2004666, PLACE6019385, STOMA1000189, SYNOV4007671, TBAES2001258, TESOP2002451, TESTI4000014, TESTI4012702, THYMU2032014, TRACH2001684, TRACH2007834, UTERU2023175 NT2RI2009583, OCBBF2008144

The result of comparative analysis of cDNA libraries derived from fetal lung (FELNG) and adult lung (HLUNG) (Table 24) showed that the genes whose expression levels were different between the two were 51 and eight clones as described below.
BRAWH3007592, BRCAN2021028, BRHIP2000819, BRSTN2016470, CTONG1000087, CTONG2028124, HCASM2007047, HEART1000074, HLUNG1000017, HLUNG2000014, HLUNG2001996, HLUNG2002465, HLUNG2002958, HLUNG2003003, HLUNG2003872, HLUNG2010464, HLUNG2011041, HLUNG2011298, HLUNG2012049, HLUNG2012287, HLUNG2012727, HLUNG2013204, HLUNG2013304, HLUNG2013622, HLUNG2013851, HLUNG2014262, HLUNG2014288, HLUNG2014449, HLUNG2015617, HLUNG2017350, HLUNG2017546, HLUNG2017806, HLUNG2019058, HSYRA2008376, KIDNE2012945, NT2RI2003993, NT2RI3007543, OCBBF3000483, SMINT1000192, SPLEN2028914, SPLEN2031547, STOMA1000189, SYNOV4007671, TESOP1000127, TESTI2003573, TESTI4000014, TESTI4037156, TRACH2005811, TRACH3004068, UTERU2005621, UTERU2023175
FEBRA2028516, HCHON2000508, HLUNG2013350, HLUNG2015418, HLUNG2015548, HLUNG2016862, TESTI2015626, TRACH2019672

These genes are involved in regeneration of tissues and/or cells.

A nucleotide sequence information-based analysis was carried out to identify the genes whose expression frequencies are higher or lower in CD34+ cell (cell expressing a glycoprotein CD34) treated with the osteoclast differentiation factor (Molecular Medicine 38. 642-648. (2001)) than in the untreated CD34+ cell, which is the precursor cell of monocyte/macrophage line. The result of comparative analysis for the frequency between the two cDNA libraries prepared from the RNA of CD34+ cells (CD34C) and from the RNA of CD34+ cells treated with the osteoclast differentiation factor (D3OST, D60ST or D9OST) showed following genes whose expression levels were different between the two.

**Table 2**

| Clone ID | CD34C | D3OST | D6OST | D9OST |
|---|---|---|---|---|
| BRACE3013780 | 0.000 | 55.996 | 0.000 | 0.000 |
| BRAMY2047420 | 42.545 | 0.000 | 0.000 | 0.000 |
| BRSTN2016470 | 0.000 | 2.555 | 0.000 | 0.000 |
| CTONG3008894 | 0.000 | 8.487 | 0.000 | 0.000 |
| D3OST2002182 | 0.000 | 86.773 | 0.000 | 0.000 |
| D3OST2002648 | 0.000 | 17.515 | 0.000 | 0.000 |
| D3OST3000169 | 20.553 | 28.566 | 0.000 | 19.796 |
| PEBLM2005183 | 0.000 | 0.000 | 0.000 | 50.747 |
| PUAEN2009655 | 0.000 | 0.000 | 0.000 | 49.285 |
| TESTI4000014 | 7.500 | 0.000 | 0.000 | 0.000 |
| TESTI4010851 | 0.000 | 0.000 | 0.000 | 7.372 |
| TRACH2023299 | 0.000 | 74.521 | 0.000 | 0.000 |
| TRACH2025535 | 0.000 | 6.778 | 0.000 | 0.000 |
| TRACH3001427 | 0.000 | 0.000 | 0.000 | 12.519 |
| UTERU2006137 | 0.000 | 90.731 | 0.000 | 0.000 |
| HCHON2000508 | 0.000 | 4.360 | 50.138 | 0.000 |
| TESTI2015626 | 0.000 | 0.000 | 0.000 | 4.435 |

A survey was performed for genes whose expression levels are varied in response to induction of differentiation (stimulation by retinoic acid (RA) or growth inhibitor treatment after RA stimulation) in cultured cells of a neural strain, NT2. The result of comparative analysis of cDNA libraries derived from undifferentiated NT2 cells (NT2RM) and the cells subjected to the differentiation treatment (NT2RP, NT2RI or NT2NE) showed following genes whose expression levels were different between the two.

**Table 3**

| Clone ID | NT2RM | NT2RP | NT2RI | NT2NE |
|---|---|---|---|---|
| BNGH42007788 | 0.000 | 7.419 | 0.000 | 0.000 |
| BRACE1000186 | 0.000 | 5.211 | 0.000 | 7.836 |
| BRACE2006319 | 0.000 | 8.450 | 0.000 | 0.000 |
| BRACE2014306 | 0.000 | 0.000 | 0.000 | 17.640 |
| BRACE2015058 | 0.000 | 10.567 | 0.000 | 0.000 |
| BRACE2044286 | 0.000 | 0.000 | 33.923 | 0.000 |
| BRACE3010428 | 0.000 | 0.000 | 2.080 | 0.000 |
| BRAMY2044078 | 0.000 | 10.567 | 0.000 | 0.000 |
| BRAWH2014645 | 0.000 | 7.643 | 1.921 | 0.000 |
| BRAWH2014662 | 0.000 | 0.000 | 0.000 | 56.250 |
| BRAWH3002574 | 0.000 | 0.000 | 12.014 | 0.000 |
| BRAWH3003992 | 0.000 | 34.956 | 0.000 | 0.000 |
| BRAWH3005981 | 0.000 | 70.676 | 0.000 | 0.000 |
| BRAWH3007592 | 0.000 | 8.644 | 3.259 | 0.000 |
| BRCAN2009432 | 0.000 | 3.173 | 0.000 | 0.000 |
| BRCAN2016619 | 0.000 | 0.000 | 23.940 | 0.000 |
| BRCAN2028355 | 0.000 | 0.000 | 0.000 | 5.354 |
| BRHIP2001074 | 0.000 | 47.218 | 0.000 | 0.000 |
| BRHIP2007741 | 0.000 | 0.000 | 6.309 | 6.291 |
| BRHIP2014228 | 0.000 | 35.475 | 0.000 | 0.000 |
| BRHIP2024146 | 0. 000 | 0.000 | 1.106 | 0.000 |
| BRHIP3007586 | 0.000 | 0.000 | 0.000 | 12.507 |
| BRHIP3018797 | 0.000 | 0.000 | 4.869 | 0.000 |
| BRTHA2003461 | 0.000 | 0.000 | 3.989 | 0.000 |
| BRTHA3000633 | 0.000 | 0.000 | 62.102 | 0.000 |
| BRTHA3003490 | 0.000 | 3.913 | 0.000 | 0.000 |
| COLON2001721 | 0.000 | 0.000 | 10.053 | 0.000 |
| CTONG1000087 | 0.000 | 5.041 | 5.701 | 3.790 |
| CTONG2008233 | 0.000 | 8.275 | 2.496 | 7.466 |
| CTONG2020638 | 0.000 | 0.000 | 22.499 | 0.000 |
| CTONG2028124 | 0.000 | 1.211 | 0.913 | 0.000 |
| CTONG3003905 | 0.000 | 47.197 | 0.000 | 0.000 |
| CTONG3008894 | 0.000 | 7.008 | 9.247 | 2.634 |
| CTONG3009028 | 0.000 | 0.000 | 2.924 | 0.000 |
| CTONG3009239 | 0.000 | 0.000 | 2.624 | 0.000 |
| DFNES2011499 | 0.000 | 0.000 | 22.548 | 0.000 |
| FCBBF3001977 | 0.000 | 17.952 | 13.536 | 0.000 |
| FEBRA1000030 | 0.000 | 0.000 | 0.000 | 59.247 |
| FEBRA2006396 | 0.000 | 0.000 | 14.606 | 0.000 |
| FEBRA2007801 | 0.000 | 5.950 | 0.000 | 0.000 |
| HCHON2000028 | 0.000 | 0.000 | 5.766 | 0.000 |
| HCHON2000244 | 0.000 | 3.171 | 1.195 | 0.000 |
| HCHON2001084 | 0.000 | 0.000 | 4.173 | 0.000 |
| HCHON2001217 | 0.000 | 2.509 | 5.674 | 0.000 |
| HCHON2001548 | 0.000 | 27.583 | 0.000 | 0.000 |
| HCHON2006250 | 0.000 | 3.771 | 0.000 | 0.000 |
| HEART1000074 | 0.000 | 1.830 | 0.000 | 0.000 |
| HHDPC1000118 | 0.000 | 10.102 | 3.809 | 15.191 |
| HSYRA2009075 | 0.000 | 2.899 | 0.000 | 0.000 |
| IMR322000127 | 0.000 | 3.733 | 0.000 | 0.000 |
| IMR322001380 | 0.000 | 0.000 | 3.602 | 7.184 |
| KIDNE2000665 | 0.000 | 0.000 | 0.000 | 7.016 |
| KIDNE2002252 | 0.000 | 0.000 | 3.961 | 0.000 |
| MESAN2006563 | 0.000 | 1.664 | 2.510 | 0.000 |
| MESAN2012054 | 0.000 | 0.000 | 4.636 | 0.000 |
| MESAN2015515 | 0.000 | 6.402 | 0.000 | 0.000 |
| NT2NE2003252 | 0.000 | 0.000 | 0.000 | 100.000 |
| NT2NE2005890 | 0.000 | 0.000 | 0.000 | 64.265 |
| NT2NE2006531 | 0.000 | 0.000 | 0.000 | 100.000 |
| NT2NE2006909 | 0.000 | 0.000 | 0.000 | 1.034 |
| NT2NE2008060 | 0.000 | 0.000 | 0.000 | 74.472 |
| NT2RI2003993 | 0.000 | 0.000 | 6.899 | 0.000 |
| NT2RI2004618 | 0.000 | 0.000 | 100.000 | 0.000 |
| NT2RI2005166 | 0.000 | 0.000 | 47.393 | 0.000 |
| NT2RI2006686 | 0.000 | 0.000 | 21.246 | 0.000 |
| NT2RI2008724 | 0.000 | 0.000 | 3.337 | 0. 000 |
| NT2RI2009855 | 0. 000 | 0. 000 | 100. 000 | 0. 000 |
| NT2RI2011422 | 0.000 | 0.000 | 100.000 | 0. 000 |
| NT2RI2011683 | 0.000 | 0.000 | 100.000 | 0.000 |
| NT2RI2012659 | 0.000 | 0. 000 | 24.645 | 0.000 |
| NT2RI2012990 | 0.000 | 0.000 | 24.360 | 0.000 |
| NT2RI2013357 | 0.000 | 0.000 | 73.545 | 0.000 |
| NT2RI2014247 | 0.000 | 0.000 | 27.718 | 0.000 |
| NT2RI2014551 | 0.000 | 0.000 | 100.000 | 0.000 |
| NT2RI2014733 | 0.000 | 0.000 | 45.319 | 0.000 |
| NT2RI2016128 | 0.000 | 0.000 | 100.000 | 0.000 |
| NT2RI2018311 | 0.000 | 0.000 | 78.082 | 0.000 |
| NT2RI2018883 | 0.000 | 0.000 | 49.384 | 0.000 |
| NT2RI2019751 | 0.000 | 0.000 | 71.913 | 0.000 |
| NT2RI2023303 | 0.000 | 0.000 | 100.000 | 0.000 |
| NT2RI2025909 | 0. 000 | 0. 000 | 100. 000 | 0. 000 |
| NT2RI2025957 | 0.000 | 0.000 | 100.000 | 0.000 |
| NT2RI2027081 | 0.000 | 0.000 | 100.000 | 0.000 |
| NT2RI2027396 | 0.000 | 0.000 | 100.000 | 0.000 |
| NT2RI3000622 | 0.000 | 0.000 | 15.558 | 0.000 |
| NT2RI3001263 | 0.000 | 0.000 | 100.000 | 0.000 |
| NT2RI3001515 | 0.000 | 0.000 | 19.682 | 0.000 |
| NT2RI3002303 | 0. 000 | 0. 000 | 100. 000 | 0. 000 |
| NT2RI3002842 | 0.000 | 45. 164 | 34.054 | 0.000 |
| NT2RI3002892 | 0.000 | 4.241 | 3. 198 | 0.000 |
| NT2RI3003031 | 0.000 | 0.000 | 100.000 | 0.000 |
| NT2RI3003095 | 0. 000 | 0. 000 | 100. 000 | 0. 000 |
| NT2RI3003162 | 0.000 | 0.000 | 100.000 | 0.000 |
| NT2RI3003382 | 0.000 | 0.000 | 71.913 | 0.000 |
| NT2RI3003409 | 0. 000 | 0. 000 | 100.000 | 0. 000 |
| NT2RI3004381 | 0.000 | 0.000 | 100.000 | 0.000 |
| NT2RI3004510 | 0.000 | 0.000 | 51.230 | 0.000 |
| NT2RI3005202 | 0.000 | 0.000 | 100.000 | 0.000 |
| NT2RI3005403 | 0.000 | 0.000 | 62. 102 | 0.000 |
| NT2RI3005724 | 0.000 | 14.284 | 10.770 | 0.000 |
| NT2RI3006132 | 0.000 | 0.000 | 100.000 | 0.000 |
| NT2RI3006171 | 0. 000 | 0. 000 | 100. 000 | 0. 000 |
| NT2RI3006284 | 0. 000 | 0. 000 | 3. 760 | 0. 000 |
| NT2RI3006340 | 0. 000 | 0. 000 | 14. 839 | 0. 000 |
| NT2RI3006376 | 0.000 | 0.000 | 100.000 | 0.000 |
| NT2RI3006673 | 0.000 | 0.000 | 18. 135 | 0.000 |
| NT2RI3006796 | 0. 000 | 0. 000 | 100. 000 | 0. 000 |
| NT2RI3007065 | 0.000 | 0.000 | 100.000 | 0.000 |
| NT2RI3007158 | 0.000 | 0.000 | 100.000 | 0.000 |
| NT2RI3007291 | 0.000 | 0.000 | 22.746 | 0.000 |
| NT2RI3007543 | 0.000 | 0.000 | 1.542 | 6.150 |
| NT2RI3007757 | 0. 000 | 30. 480 | 45. 964 | 0. 000 |
| NT2RI3007978 | 0. 000 | 0. 000 | 100. 000 | 0. 000 |
| NT2RI3008055 | 0.000 | 0.000 | 40.142 | 0.000 |
| NT2RI3008162 | 0.000 | 0.000 | 100.000 | 0.000 |
| NT2RI3008652 | 0.000 | 0.000 | 100.000 | 0.000 |
| NT2RI3008697 | 0.000 | 0.000 | 100.000 | 0.000 |
| NT2RI3008974 | 0.000 | 0.000 | 100.000 | 0.000 |
| NT2RI3009158 | 0.000 | 0.000 | 45.727 | 0.000 |
| NT2RP7000359 | 0.000 | 100.000 | 0.000 | 0.000 |
| NT2RP7000466 | 0.000 | 2.098 | 3.164 | 0.000 |
| NT2RP7004027 | 0.000 | 100.000 | 0.000 | 0.000 |
| NT2RP7004123 | 0.000 | 4.625 | 0.000 | 0.000 |
| NT2RP7005118 | 0.000 | 100.000 | 0.000 | 0.000 |
| NT2RP7005529 | 0.000 | 35.588 | 0.000 | 0.000 |
| NT2RP7005846 | 0.000 | 100.000 | 0.000 | 0.000 |
| NT2RP7009030 | 0.000 | 46.373 | 0.000 | 0.000 |
| NT2RP7009147 | 0.000 | 7.679 | 2.895 | 0.000 |
| NT2RP7009867 | 0.000 | 100.000 | 0.000 | 0.000 |
| NT2RP7010128 | 0.000 | 100.000 | 0.000 | 0.000 |
| NT2RP7010599 | 0.000 | 77.250 | 0.000 | 0.000 |
| NT2RP7011570 | 0.000 | 100.000 | 0.000 | 0.000 |
| NT2RP7013795 | 0.000 | 10.432 | 0.000 | 0.000 |
| NT2RP7014005 | 0.000 | 14.022 | 21.145 | 0.000 |
| NT2RP7015512 | 0.000 | 31.156 | 0.000 | 0.000 |
| NT2RP7017365 | 0.000 | 100.000 | 0.000 | 0.000 |
| NT2RP7017474 | 0.000 | 45.366 | 0.000 | 0.000 |
| NT2RP7017546 | 0.000 | 100.000 | 0.000 | 0.000 |
| NT2RP8000137 | 0.000 | 100.000 | 0.000 | 0.000 |
| NT2RP8000296 | 0.000 | 100.000 | 0.000 | 0.000 |
| NT2RP8000483 | 0.000 | 100.000 | 0.000 | 0.000 |
| NTONG2005969 | 0.000 | 15.484 | 0.000 | 0.000 |
| OCBBF2007028 | 0.000 | 0.000 | 2.509 | 0.000 |
| OCBBF2037068 | 0.000 | 0.000 | 27.189 | 54.224 |
| PLACE7000514 | 0.000 | 0.000 | 8.644 | 0.000 |
| PUAEN2007044 | 0.000 | 3.455 | 13.024 | 0.000 |
| SPLEN2002467 | 0.000 | 7.852 | 0.000 | 0.000 |
| SPLEN2006122 | 0.000 | 0.000 | 1.530 | 0.000 |
| SPLEN2028914 | 0.000 | 6.730 | 10.149 | 0.000 |
| SPLEN2031547 | 0.000 | 0.000 | 2.861 | 0.000 |
| SYNOV4002346 | 0.000 | 0.000 | 10.899 | 0.000 |
| SYNOV4007671 | 42.189 | 0.000 | 0.000 | 2.657 |
| SYNOV4008440 | 0.000 | 0.000 | 2.681 | 0.000 |
| TESOP2002273 | 0.000 | 9.782 | 0.000 | 0.000 |
| TESTI2003573 | 0.000 | 0.000 | 13.573 | 0.000 |
| TESTI4000014 | 0.000 | 3.443 | 0.974 | 0.000 |
| TESTI4009286 | 0.000 | 1.747 | 0.000 | 0.000 |
| TESTI4010851 | 0.000 | 5.270 | 1.987 | 0.000 |
| TESTI4012702 | 0.000 | 0.000 | 3.961 | 0.000 |
| TESTI4029671 | 0.000 | 44.826 | 0.000 | 0.000 |
| TESTI4037156 | 0.000 | 4.669 | 1.509 | 2.006 |
| THYMU3000133 | 0.000 | 34.767 | 8.738 | 0.000 |
| TRACH1000205 | 0.000 | 0.000 | 7.152 | 0.000 |
| TRACH2005811 | 0.000 | 0.000 | 0.000 | 3.822 |
| TRACH2007834 | 0.000 | 3.647 | 1.833 | 0.000 |
| TRACH2025535 | 0.000 | 1.399 | 3.165 | 4.208 |
| TRACH3001427 | 0.000 | 8.951 | 1.687 | 3.365 |
| TRACH3002192 | 0.000 | 0.000 | 2.267 | 4.520 |
| TRACH3004721 | 0.000 | 14.013 | 5.283 | 10.536 |
| TRACH3008093 | 0.000 | 0.000 | 8.902 | 0.000 |
| TRACH3008535 | 0.000 | 68.270 | 0.000 | 0.000 |
| TRACH3008713 | 0.000 | 68.270 | 0.000 | 0.000 |
| UTERU2002410 | 0.000 | 0.000 | 1.067 | 0.000 |
| UTERU2023175 | 0.000 | 7.176 | 0.000 | 5.396 |
| ADRGL2000042 | 0.000 | 0.000 | 0.000 | 9.204 |
| BRACE2003609 | 0.000 | 21.907 | 0.000 | 32.942 |
| BRACE3003026 | 0.000 | 0.000 | 59.349 | 0.000 |
| BRHIP3000017 | 0.000 | 0.000 | 8.013 | 0.000 |
| CTONG2020411 | 0.000 | 16.593 | 25.022 | 0.000 |
| FCBBF1000509 | 0.000 | 0.000 | 0.000 | 6.762 |
| FCBBF3027854 | 0.000 | 0.000 | 28.447 | 0.000 |
| FEBRA2028516 | 0.000 | 11.027 | 6.236 | 0.000 |
| HCHON2000508 | 0.000 | 0.900 | 0.000 | 0.000 |
| IMR322001879 | 0.000 | 0.000 | 35.028 | 0.000 |
| NT2RI2005772 | 0.000 | 0.000 | 100.000 | 0.000 |
| NT2RI2008952 | 0.000 | 0.000 | 100.000 | 0.000 |
| NT2RI2009583 | 0.000 | 0.000 | 0.813 | 0.811 |
| NT2RI2018448 | 0.000 | 15.176 | 11.442 | 0.000 |
| NT2RI2027157 | 0.000 | 0.000 | 100.000 | 0.000 |
| NT2RI3000174 | 0.000 | 0.000 | 61.866 | 0.000 |
| NT2RI3001132 | 0.000 | 0.000 | 100.000 | 0.000 |
| NT2RI3002557 | 0.000 | 0.000 | 100.000 | 0.000 |
| NT2RI3005928 | 0.000 | 0.000 | 100.000 | 0.000 |
| NT2RI3007167 | 0.000 | 0.000 | 100.000 | 0.000 |
| NT2RI3007443 | 0.000 | 0.000 | 100.000 | 0.000 |
| NT2RP7008435 | 0.000 | 100.000 | 0.000 | 0.000 |
| NT2RP8000521 | 0.000 | 62.933 | 0.000 | 0.000 |
| OCBBF2006987 | 0.000 | 62.306 | 0.000 | 0.000 |
| PERIC2007068 | 0.000 | 3.719 | 2.804 | 0.000 |
| TESTI2015626 | 9.463 | 0.000 | 0.000 | 2.384 |
| TESTI4015442 | 0.000 | 48.593 | 0.000 | 0.000 |
| TLIVE2002046 | 0.000 | 0.000 | 3.298 | 0.000 |
| TRACH3000134 | 0.000 | 43.581 | 0.000 | 0.000 |
| TUTER2000057 | 0.000 | 0.000 | 7.539 | 0.000 |

The result of comparative analysis of cDNA libraries derived from the cerebral cortex of Alzheimer patients (BRALZ and BRASW), and from whole tissues of a normal brain (BRAWH) showed the following genes whose expression levels differed between the two.

**Table 4**

| Clone ID | BRAWH | BRALZ | BRASW |
|---|---|---|---|
| ASTRO1000009 | 2.611 | 0.000 | 0.000 |
| BLADE2008398 | 12.401 | 0.000 | 0.000 |
| BRACE1000186 | 4.324 | 0.000 | 0.000 |
| BRACE1000258 | 31.956 | 0.000 | 0.000 |
| BRACE1000533 | 11.795 | 0.000 | 0.000 |
| BRACE2005457 | 58.488 | 0.000 | 0.000 |
| BRACE2010489 | 63.510 | 0.000 | 0.000 |
| BRACE2014657 | 15.451 | 0.000 | 0.000 |
| BRACE2035381 | 10.177 | 0.000 | 0.000 |
| BRACE2044286 | 18.667 | 0.000 | 0.000 |
| BRACE2045954 | 27.309 | 0.000 | 0.000 |
| BRACE3000787 | 32.844 | 0.000 | 0.000 |
| BRACE3003192 | 58.488 | 0.000 | 0.000 |
| BRACE3005499 | 31. 276 | 0.000 | 0.000 |
| BRACE3007480 | 19.471 | 0.000 | 0.000 |
| BRACE3009237 | 18.139 | 0.000 | 0.000 |
| BRACE3009724 | 58.488 | 0.000 | 0.000 |
| BRACE3009747 | 2.237 | 0.000 | 0.000 |
| BRACE3010428 | 6.868 | 0.000 | 0.000 |
| BRACE3011271 | 11.036 | 0.000 | 0.000 |
| BRACE3011421 | 28.251 | 0.000 | 0.000 |
| BRACE3012364 | 8.506 | 0.000 | 0.000 |
| BRACE3022769 | 4.285 | 0.000 | 0.000 |
| BRACE3026735 | 24.173 | 0.000 | 0.000 |
| BRACE3031838 | 58.488 | 0.000 | 0.000 |
| BRALZ2011796 | 5.511 | 39.830 | 0.000 |
| BRALZ2012183 | 0.000 | 100.000 | 0.000 |
| BRALZ2012848 | 0.000 | 100.000 | 0.000 |
| BRALZ2014484 | 0. 000 | 1 00. 000 | 0.000 |
| BRALZ2016085 | 0.000 | 100.000 | 0.000 |
| BRALZ2016498 | 0.000 | 100.000 | 0.000 |
| BRALZ2017359 | 0.000 | 75.184 | 0.000 |
| BRAMY2003008 | 26.445 | 0.000 | 0.000 |
| BRAMY2005052 | 11.612 | 0.000 | 0.000 |
| BRAMY2019300 | 49.811 | 0.000 | 0.000 |
| BRAMY2019963 | 20.428 | 0.000 | 0.000 |
| BRAMY2036567 | 7.474 | 0.000 | 0.000 |
| BRAMY2037823 | 29.664 | 0.000 | 0.000 |
| BRAMY2040592 | 3.482 | 12.582 | 0.000 |
| BRAMY3002803 | 14.428 | 0.000 | 0.000 |
| BRAMY3004224 | 33.027 | 0.000 | 0.000 |
| BRAMY3005091 | 19.193 | 0.000 | 0.000 |
| BRASW1000053 | 0.000 | 0.000 | 100.000 |
| BRASW1000125 | 0.000 | 0.000 | 99.054 |
| BRAWH1000127 | 15.983 | 0.000 | 0.000 |
| BRAWH2001395 | 14.290 | 3.037 | 0.000 |
| BRAWH2001671 | 7.605 | 0.000 | 0.000 |
| BRAWH2001940 | 37.398 | 0.000 | 0.000 |
| BRAWH2001973 | 37.398 | 0.000 | 0.000 |
| BRAWH2002560 | 6.454 | 0.000 | 0.000 |
| BRAWH2002761 | 100.000 | 0.000 | 0.000 |
| BRAWH2005315 | 100.000 | 0.000 | 0.000 |
| BRAWH2007658 | 58.101 | 0.000 | 0.000 |
| BRAWH2010000 | 18.745 | 0.000 | 0.000 |
| BRAWH2010084 | 100.000 | 0.000 | 0.000 |
| BRAWH2010536 | 14.718 | 0.000 | 0.000 |
| BRAWH2012162 | 36.060 | 0.000 | 0.000 |
| BRAWH2012326 | 100.000 | 0.000 | 0.000 |
| BRAWH2013294 | 39.442 | 0.000 | 0.000 |
| BRAWH2013871 | 37.485 | 0.000 | 0.000 |
| BRAWH2014414 | 17.865 | 0.000 | 0.000 |
| BRAWH2014645 | 4.228 | 0.000 | 0.000 |
| BRAWH2014662 | 15.521 | 0.000 | 0.000 |
| BRAWH2014876 | 10.473 | 0.000 | 0.000 |
| BRAWH2014954 | 58.488 | 0.000 | 0.000 |
| BRAWH2016221 | 47.417 | 0.000 | 0.000 |
| BRAWH2016439 | 100.000 | 0.000 | 0.000 |
| BRAWH2016702 | 73.807 | 0.000 | 0.000 |
| BRAWH2016724 | 35.119 | 0.000 | 0.000 |
| BRAWH3000078 | 100.000 | 0.000 | 0.000 |
| BRAWH3000100 | 100.000 | 0.000 | 0.000 |
| BRAWH3000314 | 71.553 | 0.000 | 0.000 |
| BRAWH3000491 | 100.000 | 0.000 | 0.000 |
| BRAWH3001326 | 45.606 | 0.000 | 0.000 |
| BRAWH3001475 | 100.000 | 0.000 | 0.000 |
| BRAWH3001891 | 34.539 | 0.000 | 0.000 |
| BRAWH3002574 | 13.222 | 0.000 | 0.000 |
| BRAWH3002600 | 36.800 | 0.000 | 0.000 |
| BRAWH3002819 | 100.000 | 0.000 | 0.000 |
| BRAWH3002821 | 21.953 | 0.000 | 0.000 |
| BRAWH3003522 | 100.000 | 0.000 | 0.000 |
| BRAWH3003555 | 15.229 | 0.000 | 0.000 |
| BRAWH3003727 | 10.055 | 0.000 | 0.000 |
| BRAWH3003801 | 100.000 | 0.000 | 0.000 |
| BRAWH3003992 | 29.008 | 0.000 | 0.000 |
| BRAWH3004453 | 100.000 | 0.000 | 0.000 |
| BRAWH3004666 | 49.499 | 0.000 | 0.000 |
| BRAWH3005132 | 49.811 | 0.000 | 0.000 |
| BRAWH3005422 | 100.000 | 0.000 | 0.000 |
| BRAWH3005912 | 100.000 | 0.000 | 0.000 |
| BRAWH3005981 | 29.324 | 0.000 | 0.000 |
| BRAWH3006548 | 71.018 | 0.000 | 0.000 |
| BRAWH3006792 | 49.499 | 0.000 | 0.000 |
| BRAWH3007221 | 100.000 | 0.000 | 0.000 |
| BRAWH3007506 | 100.000 | 0.000 | 0.000 |
| BRAWH3007592 | 8.966 | 0.000 | 0.000 |
| BRAWH3007726 | 54.530 | 0.000 | 0.000 |
| BRAWH3007783 | 100.000 | 0.000 | 0.000 |
| BRAWH3008341 | 100.000 | 0.000 | 0.000 |
| BRAWH3008697 | 100.000 | 0.000 | 0.000 |
| BRAWH3008931 | 3.463 | 0.000 | 0.000 |
| BRAWH3009297 | 58.488 | 0.000 | 0.000 |
| BRCOC2003213 | 10.381 | 0.000 | 0.000 |
| BRCOC2014033 | 15.633 | 0.000 | 0.000 |
| BRCOC2020142 | 22.014 | 0.000 | 0.000 |
| BRHIP2000920 | 36.630 | 0.000 | 0.000 |
| BRHIP2005719 | 49.499 | 0.000 | 0.000 |
| BRHIP2007741 | 6.943 | 0.000 | 0.000 |
| BRHIP2014228 | 29.439 | 0.000 | 0.000 |
| BRHIP2024146 | 3.042 | 12.091 | 0.000 |
| BRHIP2026288 | 0.000 | 77.982 | 0.000 |
| BRHIP3000339 | 14.290 | 3.037 | 0.000 |
| BRHIP3006683 | 24.100 | 0.000 | 0.000 |
| BRHIP3007586 | 17.255 | 0.000 | 0.000 |
| BRHIP3008405 | 35.187 | 0.000 | 0.000 |
| BRHIP3018797 | 30.810 | 4.840 | 0.000 |
| BRSSN2000684 | 23.433 | 0.000 | 0.000 |
| BRSSN2011738 | 31.553 | 0.000 | 0.000 |
| BRSSN2014299 | 3.695 | 0.000 | 0.000 |
| BRSTN2008052 | 32.844 | 0.000 | 0.000 |
| BRSTN2015015 | 14.017 | 0.000 | 0.000 |
| BRSTN2016470 | 0.438 | 7.909 | 0.000 |
| BRTHA1000311 | 11.803 | 0.000 | 0.000 |
| BRTHA2008335 | 16.281 | 0.000 | 0.000 |
| BRTHA3002427 | 8.577 | 0.000 | 0.000 |
| BRTHA3003490 | 1.623 | 0.000 | 0.000 |
| BRTHA3008520 | 47.417 | 0.000 | 0.000 |
| BRTHA3017848 | 47.417 | 0.000 | 0.000 |
| COLON2001721 | 11.065 | 0.000 | 0.000 |
| CTONG2017500 | 2.649 | 0.000 | 0.000 |
| CTONG2028124 | 0.503 | 0.000 | 0.000 |
| CTONG3000657 | 3.880 | 0.000 | 0.000 |
| CTONG3001123 | 7.847 | 0.000 | 0.000 |
| CTONG3009328 | 11.993 | 43.334 | 0.000 |
| FCBBF2001183 | 16.537 | 5.975 | 0.000 |
| FCBBF3001977 | 7.448 | 0.000 | 0.000 |
| FEBRA2007544 | 14.689 | 0.000 | 0.000 |
| FEBRA2007801 | 4.937 | 0.000 | 0.000 |
| FEBRA2020886 | 12.124 | 0.000 | 0.000 |
| FEBRA2028618 | 5.082 | 0.000 | 0.000 |
| HCASM2007047 | 3.431 | 0.000 | 0.000 |
| HCHON2000244 | 0.658 | 0.000 | 0.000 |
| HCHON2000626 | 2.351 | 0.000 | 0.000 |
| HCHON2001217 | 3.123 | 0.000 | 0.000 |
| HCHON2002676 | 13.647 | 0.000 | 0.000 |
| HCHON2006250 | 1.565 | 0.000 | 0.000 |
| HEART1000074 | 0.759 | 0.000 | 0.000 |
| HHDPC1000118 | 2.096 | 0.000 | 0.000 |
| HLUNG2002465 | 1.209 | 0.000 | 0.000 |
| IMR322000127 | 3.098 | 5.597 | 0.000 |
| IMR322001380 | 0.000 | 7.163 | 0.000 |
| IMR322002035 | 36.176 | 0.000 | 0.000 |
| KIDNE2006580 | 7.013 | 0.000 | 0.000 |
| MESAN2006563 | 0.691 | 0.000 | 0.000 |
| MESAN2012054 | 12.754 | 0.000 | 0.000 |
| MESTC1000042 | 2.245 | 0.000 | 0.000 |
| NOVAR2001783 | 4.027 | 0.000 | 0.000 |
| NT2NE2006909 | 0.285 | 0.000 | 0.000 |
| NT2RI2008724 | 1.836 | 0.000 | 0.000 |
| NT2RI2012659 | 13.562 | 0.000 | 0.000 |
| NT2RI2014733 | 24.938 | 0.000 | 0.000 |
| NT2RI3002892 | 8.799 | 0.000 | 0.000 |
| NT2RI3006284 | 4.138 | 0.000 | 0.000 |
| NT2RI3006673 | 19.959 | 0.000 | 0.000 |
| NT2RI3007543 | 1.697 | 0.000 | 0.000 |
| NT2RI3008055 | 44.179 | 0.000 | 0.000 |
| NT2RP7005529 | 14.766 | 0.000 | 0.000 |
| NT2RP7009147 | 14.337 | 0.000 | 0.000 |
| NT2RP7014005 | 5.818 | 0.000 | 0.000 |
| NT2RP7017474 | 18.823 | 0.000 | 0.000 |
| NTONG2005969 | 0.000 | 11.607 | 0.000 |
| OCBBF2001794 | 4.728 | 0.000 | 0.000 |
| OCBBF2006005 | 9.535 | 0.000 | 0.000 |
| OCBBF2006764 | 15.345 | 0.000 | 0.000 |
| OCBBF2007028 | 9.665 | 0.000 | 0.000 |
| OCBBF2007114 | 0.000 | 38.623 | 0.000 |
| OCBBF2010140 | 32.508 | 0.000 | 0.000 |
| OCBBF2021286 | 18.456 | 0.000 | 0.000 |
| OCBBF2023162 | 0.000 | 37.152 | 0.000 |
| OCBBF2024850 | 4.445 | 0.000 | 0.000 |
| OCBBF2028935 | 5.789 | 4.183 | 0.000 |
| OCBBF2036743 | 11.053 | 0.000 | 0.000 |
| OCBBF2038317 | 19.713 | 0.000 | 0.000 |
| OCBBF3000483 | 11.973 | 0.000 | 0.000 |
| OCBBF3008230 | 29.840 | 0.000 | 0.000 |
| PEBLM2004666 | 3.715 | 0.000 | 0.000 |
| PLACE6001185 | 21.358 | 0.000 | 0.000 |
| PUAEN2005930 | 18.362 | 0.000 | 0.000 |
| PUAEN2006701 | 2.249 | 8.128 | 0.000 |
| PUAEN2007044 | 8.600 | 0.000 | 0.000 |
| PUAEN2009655 | 18.275 | 0.000 | 0.000 |
| SMINT2001818 | 0.000 | 3.387 | 0.000 |
| SPLEN2028914 | 2.792 | 0.000 | 0.000 |
| SPLEN2031424 | 15.229 | 0.000 | 0.000 |
| SPLEN2031547 | 1.574 | 5.689 | 0.000 |
| SPLEN2034781 | 27.984 | 0.000 | 0.000 |
| SPLEN2036932 | 2.932 | 0.000 | 0.000 |
| SYNOV2014400 | 12.977 | 0.000 | 0.000 |
| SYNOV4002346 | 5.997 | 0.000 | 0.000 |
| SYNOV4002883 | 23.940 | 0.000 | 0.000 |
| SYNOV4007430 | 31.677 | 0.000 | 0.000 |
| SYNOV4007671 | 0.000 | 2.649 | 0.000 |
| SYNOV4008440 | 1.475 | 0.000 | 0.000 |
| TESOP2002273 | 0.000 | 14.666 | 0.000 |
| TESOP2002451 | 2.375 | 0.000 | 0.000 |
| TESTI4000014 | 1.964 | 0.000 | 0.000 |
| TESTI4000209 | 2. 649 | 0. 000 | 0. 000 |
| TESTI4001100 | 4.098 | 0.000 | 0.000 |
| TESTI4006137 | 25.755 | 0.000 | 0.000 |
| TESTI4008797 | 12.429 | 0. 000 | 0. 000 |
| TESTI4009286 | 1.450 | 0. 000 | 0. 000 |
| TESTI4010851 | 3.280 | 0.000 | 0.000 |
| TESTI4013817 | 27.163 | 0.000 | 0.000 |
| TESTI4014694 | 2.229 | 0.000 | 0.000 |
| TESTI4021478 | 22.098 | 0.000 | 0.000 |
| TESTI4022936 | 26.445 | 0. 000 | 0. 000 |
| TESTI4024420 | 37.398 | 0. 000 | 0. 000 |
| TESTI4027821 | 60.471 | 0. 000 | 0. 000 |
| THYMU2001090 | 21.252 | 0.000 | 0.000 |
| THYMU2033308 | 13.964 | 0.000 | 0.000 |
| THYMU2035735 | 1.319 | 0.000 | 0.000 |
| THYMU2039315 | 54.530 | 0.000 | 0.000 |
| THYMU3001234 | 11.085 | 0.000 | 0.000 |
| THYMU3008171 | 20.170 | 0.000 | 0.000 |
| TKIDN2009641 | 5.782 | 0.000 | 0.000 |
| TKIDN2009889 | 35.077 | 0.000 | 0.000 |
| TKIDN2015788 | 5.261 | 9.505 | 0.000 |
| TRACH1000205 | 19.677 | 0.000 | 0.000 |
| TRACH2001549 | 8.457 | 0.000 | 0.000 |
| TRACH2005811 | 2.109 | 0.000 | 0.000 |
| TRACH2006049 | 47.167 | 0.000 | 0.000 |
| TRACH2007834 | 0.504 | 0.000 | 0.000 |
| TRACH2008300 | 10.186 | 6.135 | 0.000 |
| TRACH2025535 | 5.806 | 0.000 | 0.000 |
| TRACH3001427 | 5.571 | 3.355 | 0.000 |
| TRACH3002192 | 4.989 | 2.253 | 0.000 |
| TRACH3004068 | 0.000 | 5.150 | 0.000 |
| TRACH3004721 | 8.721 | 0.000 | 0.000 |
| TRACH3005294 | 7.428 | 0.000 | 0.000 |
| TRACH3007479 | 1.075 | 0.000 | 0.000 |
| TRACH3008093 | 2.449 | 0.000 | 0.000 |
| TRACH3009455 | 47.167 | 0.000 | 0.000 |
| UTERU2005621 | 0.000 | 8.145 | 0.000 |
| UTERU2006115 | 7.837 | 0.000 | 0.000 |
| UTERU2019706 | 45.606 | 0.000 | 0.000 |
| UTERU2023039 | 45.606 | 0.000 | 0.000 |
| UTERU2026203 | 45.606 | 0.000 | 0.000 |
| UTERU3005230 | 24.419 | 0.000 | 0.000 |
| UTERU3007640 | 45.606 | 0.000 | 0.000 |
| UTERU3009871 | 36.230 | 0.000 | 0.000 |
| ADRGL2000042 | 2.540 | 0.000 | 0.000 |
| BLADE2006830 | 1.681 | 0.000 | 0.000 |
| BRACE2003609 | 9.090 | 0.000 | 0.000 |
| BRALZ2017844 | 0.000 | 49.396 | 0.000 |
| BRAMY3004800 | 38.061 | 0.000 | 0.000 |
| BRAWH1000369 | 100.000 | 0.000 | 0.000 |
| BRAWH2006207 | 12.943 | 0.000 | 0.000 |
| BRAWH2006395 | 12.446 | 0.000 | 0.000 |
| BRAWH2008993 | 49.811 | 0.000 | 0.000 |
| BRAWH2009393 | 100.000 | 0.000 | 0.000 |
| BRAWH2010552 | 58.488 | 0.000 | 0.000 |
| BRAWH3007441 | 100.000 | 0.000 | 0.000 |
| BRAWH3009017 | 100.000 | 0.000 | 0.000 |
| BRHIP2005271 | 7.083 | 0.000 | 0.000 |
| BRHIP3000017 | 8.819 | 0.000 | 0.000 |
| BRHIP3026052 | 0.000 | 54.140 | 0.000 |
| BRTHA2018443 | 22.098 | 0.000 | 0.000 |
| BRTHA3003000 | 17.150 | 0.000 | 0.000 |
| CTONG2020374 | 31.081 | 0.000 | 0.000 |
| CTONG2020378 | 16.140 | 0.000 | 0.000 |
| CTONG2024031 | 2.584 | 0.000 | 0.000 |
| FCBBF1000509 | 3.732 | 0.000 | 0.000 |
| FEBRA2001990 | 18.144 | 0.000 | 0.000 |
| FEBRA2006519 | 11.891 | 0.000 | 0.000 |
| FEBRA2028516 | 8.007 | 0.000 | 0.000 |
| HCHON2000743 | 6.105 | 0.000 | 0.000 |
| IMR322001879 | 9.638 | 0.000 | 0.000 |
| NT2RI2009583 | 0.224 | 0.808 | 0.000 |
| OCBBF2008144 | 5.768 | 0.000 | 0.000 |
| PERIC2007068 | 3.086 | 0.000 | 0.000 |
| PUAEN2006335 | 12.682 | 0.000 | 0.000 |
| SPLEN2039379 | 5.792 | 0.000 | 0.000 |
| TESTI4001984 | 60.471 | 0. 000 | 0. 000 |
| TESTI4008058 | 8.814 | 0. 000 | 0. 000 |
| TESTI4025268 | 60.471 | 0. 000 | 0. 000 |
| TESTI4032090 | 60.471 | 0. 000 | 0. 000 |
| THYMU3000360 | 39.314 | 0.000 | 0.000 |
| TLIVE2002046 | 5.445 | 0.000 | 0.000 |
| TRACH3000134 | 36.165 | 0.000 | 0.000 |
| UTERU2021820 | 24.929 | 0.000 | 0.000 |
| UTERU2028734 | 21.953 | 0.000 | 0.000 |

The result of comparative analysis of cDNA libraries derived from the substantia nigra (BRSSN), and from whole tissues of a normal brain (BRAWH) showed following genes whose expression levels differed between the two.

**Table 5**

| Clone ID | BRAWH | BRSSN |
|---|---|---|
| ASTRO1000009 | 2.611 | 0.000 |
| BLADE2008398 | 12.401 | 0.000 |
| BRACE1000186 | 4.324 | 0.000 |
| BRACE1000258 | 31.956 | 0.000 |
| BRACE1000533 | 11.795 | 8.780 |
| BRACE2005457 | 58.488 | 0.000 |
| BRACE2010489 | 63.510 | 0.000 |
| BRACE2014657 | 15.451 | 0.000 |
| BRACE2035381 | 10.177 | 0.000 |
| BRACE2044286 | 18.667 | 0.000 |
| BRACE2045954 | 27.309 | 0.000 |
| BRACE3000787 | 32.844 | 0.000 |
| BRACE3003192 | 58.488 | 0.000 |
| BRACE3005499 | 31.276 | 0.000 |
| BRACE3007480 | 19.471 | 0.000 |
| BRACE3009237 | 18.139 | 0.000 |
| BRACE3009724 | 58.488 | 0.000 |
| BRACE3009747 | 2.237 | 8.327 |
| BRACE3010428 | 6.868 | 4.261 |
| BRACE3011271 | 11.036 | 0.000 |
| BRACE3011421 | 28.251 | 0.000 |
| BRACE3012364 | 8.506 | 0.000 |
| BRACE3013780 | 0.000 | 17.852 |
| BRACE3022769 | 4.285 | 5.316 |
| BRACE3026735 | 24.173 | 0.000 |
| BRACE3031838 | 58.488 | 0.000 |
| BRALZ2011796 | 5.511 | 20.514 |
| BRAMY2003008 | 26.445 | 0.000 |
| BRAMY2005052 | 11.612 | 0.000 |
| BRAMY2019300 | 49.811 | 0.000 |
| BRAMY2019963 | 20.428 | 0.000 |
| BRAMY2036567 | 7.474 | 0.000 |
| BRAMY2037823 | 29.664 | 0.000 |
| BRAMY2040592 | 3.482 | 0.000 |
| BRAMY2047420 | 0.000 | 3.770 |
| BRAMY3002803 | 14.428 | 0.000 |
| BRAMY3004224 | 33.027 | 0.000 |
| BRAMY3005091 | 19.193 | 0.000 |
| BRAWH1000127 | 15.983 | 35.693 |
| BRAWH2001395 | 14.290 | 12.514 |
| BRAWH2001671 | 7.605 | 0.000 |
| BRAWH2001940 | 37.398 | 0.000 |
| BRAWH2001973 | 37.398 | 0.000 |
| BRAWH2002560 | 6.454 | 12.010 |
| BRAWH2002761 | 100.000 | 0.000 |
| BRAWH2005315 | 100.000 | 0.000 |
| BRAWH2007658 | 58.101 | 0.000 |
| BRAWH2010000 | 18.745 | 0.000 |
| BRAWH2010084 | 100.000 | 0.000 |
| BRAWH2010536 | 14.718 | 0.000 |
| BRAWH2012162 | 36.060 | 0.000 |
| BRAWH2012326 | 100.000 | 0.000 |
| BRAWH2013294 | 39.442 | 0.000 |
| BRAWH2013871 | 37.485 | 0.000 |
| BRAWH2014414 | 17.865 | 0.000 |
| BRAWH2014645 | 4.228 | 0.000 |
| BRAWH2014662 | 15.521 | 0.000 |
| BRAWH2014876 | 10.473 | 0.000 |
| BRAWH2014954 | 58.488 | 0.000 |
| BRAWH2016221 | 47.417 | 0.000 |
| BRAWH2016439 | 100.000 | 0.000 |
| BRAWH2016702 | 73.807 | 0.000 |
| BRAWH2016724 | 35.119 | 0.000 |
| BRAWH3000078 | 100.000 | 0.000 |
| BRAWH3000100 | 100.000 | 0. 000 |
| BRAWH3000314 | 71.553 | 0.000 |
| BRAWH3000491 | 100.000 | 0.000 |
| BRAWH3001326 | 45.606 | 0.000 |
| BRAWH3001475 | 100.000 | 0.000 |
| BRAWH3001891 | 34.539 | 0.000 |
| BRAWH3002574 | 13.222 | 0.000 |
| BRAWH3002600 | 36.800 | 0.000 |
| BRAWH3002819 | 100.000 | 0.000 |
| BRAWH3002821 | 21.953 | 0.000 |
| BRAWH3003522 | 100.000 | 0.000 |
| BRAWH3003555 | 15.229 | 0.000 |
| BRAWH3003727 | 10.055 | 0.000 |
| BRAWH3003801 | 100.000 | 0.000 |
| BRAWH3003992 | 29.008 | 0.000 |
| BRAWH3004453 | 100.000 | 0.000 |
| BRAWH3004666 | 49.499 | 0.000 |
| BRAWH3005132 | 49.811 | 0.000 |
| BRAWH3005422 | 100.000 | 0.000 |
| BRAWH3005912 | 100.000 | 0.000 |
| BRAWH3005981 | 29.324 | 0.000 |
| BRAWH3006548 | 71.018 | 0.000 |
| BRAWH3006792 | 49.499 | 0.000 |
| BRAWH3007221 | 100.000 | 0.000 |
| BRAWH3007506 | 100.000 | 0.000 |
| BRAWH3007592 | 8.966 | 0.000 |
| BRAWH3007726 | 54.530 | 0.000 |
| BRAWH3007783 | 100.000 | 0.000 |
| BRAWH3008341 | 100.000 | 0.000 |
| BRAWH3008697 | 100.000 | 0.000 |
| BRAWH3008931 | 3.463 | 12.891 |
| BRAWH3009297 | 58.488 | 0.000 |
| BRCOC2003213 | 10.381 | 0.000 |
| BRCOC2014033 | 15.633 | 0.000 |
| BRCOC2020142 | 22.014 | 0.000 |
| BRHIP2000920 | 36.630 | 0.000 |
| BRHIP2005719 | 49.499 | 0.000 |
| BRHIP2007741 | 6.943 | 0.000 |
| BRHIP2014228 | 29.439 | 0.000 |
| BRHIP2024146 | 3.042 | 10.190 |
| BRHIP3000339 | 14.290 | 12.514 |
| BRHIP3006683 | 24.100 | 0.000 |
| BRHIP3007586 | 17.255 | 0.000 |
| BRHIP3008405 | 35.187 | 0.000 |
| BRHIP3018797 | 30.810 | 0.000 |
| BRSSN2000684 | 23.433 | 17.444 |
| BRSSN2003086 | 0.000 | 100.000 |
| BRSSN2004496 | 0.000 | 78.696 |
| BRSSN2004719 | 0.000 | 39.002 |
| BRSSN2006892 | 0.000 | 57.631 |
| BRSSN2008549 | 0.000 | 63.611 |
| BRSSN2008797 | 0.000 | 77.045 |
| BRSSN2011262 | 0.000 | 10.489 |
| BRSSN2011738 | 31.553 | 39.146 |
| BRSSN2013874 | 0.000 | 100.000 |
| BRSSN2014299 | 3.695 | 13.753 |
| BRSSN2014424 | 0.000 | 61.866 |
| BRSSN2014556 | 0.000 | 100.000 |
| BRSSN2018581 | 0.000 | 50.137 |
| BRSSN2018925 | 0.000 | 100.000 |
| BRSTN2008052 | 32.844 | 0.000 |
| BRSTN2015015 | 14.017 | 0.000 |
| BRSTN2016470 | 0.438 | 0.000 |
| BRTHA1000311 | 11.803 | 10.982 |
| BRTHA2003461 | 0.000 | 8.169 |
| BRTHA2008335 | 16.281 | 0.000 |
| BRTHA3002427 | 8.577 | 10.641 |
| BRTHA3003490 | 1.623 | 0.000 |
| BRTHA3008520 | 47.417 | 0.000 |
| BRTHA3017848 | 47.417 | 0.000 |
| COLON2001721 | 11.065 | 0.000 |
| CTONG2017500 | 2.649 | 0.000 |
| CTONG2028124 | 0.503 | 0.000 |
| CTONG3000657 | 3.880 | 0.000 |
| CTONG3001123 | 7.847 | 0.000 |
| CTONG3009328 | 11.993 | 0.000 |
| FCBBF2001183 | 16.537 | 6.155 |
| FCBBF3001977 | 7.448 | 0.000 |
| FEBRA2007544 | 14.689 | 0.000 |
| FEBRA2007801 | 4.937 | 0.000 |
| FEBRA2020886 | 12.124 | 0.000 |
| FEBRA2024136 | 0.000 | 42.701 |
| FEBRA2025427 | 0.000 | 9.226 |
| FEBRA2028618 | 5.082 | 0.000 |
| HCASM2007047 | 3.431 | 0.000 |
| HCHON2000244 | 0.658 | 0.000 |
| HCHON2000626 | 2.351 | 4.375 |
| HCHON2001217 | 3.123 | 0.000 |
| HCHON2002676 | 13.647 | 0.000 |
| HCHON2006250 | 1.565 | 0.000 |
| HEART1000074 | 0.759 | 0.000 |
| HHDPC1000118 | 2.096 | 0.000 |
| HLUNG2002465 | 1.209 | 0.000 |
| IMR322000127 | 3.098 | 0.000 |
| IMR322002035 | 36.176 | 0.000 |
| KIDNE2006580 | 7.013 | 0.000 |
| MESAN2006563 | 0.691 | 2.570 |
| MESAN2012054 | 12.754 | 0.000 |
| MESTC1000042 | 2.245 | 0.000 |
| NOVAR2001783 | 4.027 | 0.000 |
| NT2NE2006909 | 0.285 | 0.000 |
| NT2RI2008724 | 1.836 | 0.000 |
| NT2RI2012659 | 13.562 | 0.000 |
| NT2RI2014733 | 24.938 | 0.000 |
| NT2RI3002892 | 8.799 | 0.000 |
| NT2RI3006284 | 4.138 | 0.000 |
| NT2RI3006673 | 19.959 | 0.000 |
| NT2RI3007543 | 1.697 | 0.000 |
| NT2RI3008055 | 44.179 | 0.000 |
| NT2RP7005529 | 14.766 | 0.000 |
| NT2RP7009147 | 14.337 | 0.000 |
| NT2RP7014005 | 5.818 | 0.000 |
| NT2RP7017474 | 18.823 | 0.000 |
| OCBBF2001794 | 4.728 | 0.000 |
| OCBBF2006005 | 9.535 | 0.000 |
| OCBBF2006764 | 15.345 | 0.000 |
| OCBBF2007028 | 9.665 | 0.000 |
| OCBBF2010140 | 32.508 | 0.000 |
| OCBBF2021286 | 18.456 | 0.000 |
| OCBBF2024850 | 4.445 | 0.000 |
| OCBBF2028935 | 5.789 | 8.618 |
| OCBBF2036743 | 11.053 | 0.000 |
| OCBBF2038317 | 19.713 | 0.000 |
| OCBBF3000483 | 11.973 | 0.000 |
| OCBBF3008230 | 29.840 | 0.000 |
| PEBLM2004666 | 3.715 | 0.000 |
| PLACE6001185 | 21.358 | 0.000 |
| PUAEN2005930 | 18.362 | 0.000 |
| PUAEN2006701 | 2.249 | 0.000 |
| PUAEN2007044 | 8.600 | 0.000 |
| PUAEN2009655 | 18.275 | 0.000 |
| SPLEN2028914 | 2.792 | 0.000 |
| SPLEN2031424 | 15.229 | 0.000 |
| SPLEN2031547 | 1.574 | 0.000 |
| SPLEN2034781 | 27.984 | 0.000 |
| SPLEN2036932 | 2.932 | 0.000 |
| SYNOV2014400 | 12.977 | 0.000 |
| SYNOV4002346 | 5.997 | 0.000 |
| SYNOV4002883 | 23.940 | 0.000 |
| SYNOV4007430 | 31.677 | 0.000 |
| SYNOV4008440 | 1.475 | 0.000 |
| TESOP2002451 | 2.375 | 0.000 |
| TESTI4000014 | 1.964 | 0. 665 |
| TESTI4000209 | 2. 649 | 0. 000 |
| TESTI4001100 | 4.098 | 0.000 |
| TESTI4006137 | 25.755 | 0. 000 |
| TESTI4008797 | 12.429 | 0. 000 |
| TESTI4009286 | 1.450 | 0. 000 |
| TESTI4010851 | 3.280 | 2.035 |
| TESTI4013817 | 27.163 | 0.000 |
| TESTI4014694 | 2. 229 | 0. 000 |
| TESTI4021478 | 22. 098 | 0. 000 |
| TESTI4022936 | 26.445 | 0. 000 |
| TESTI4024420 | 37.398 | 0. 000 |
| TESTI4027821 | 60.471 | 0. 000 |
| TESTI4037156 | 0.000 | 2.060 |
| THYMU2001090 | 21.252 | 0.000 |
| THYMU2033308 | 13.964 | 0.000 |
| THYMU2035735 | 1.319 | 0.000 |
| THYMU2039315 | 54.530 | 0.000 |
| THYMU3001234 | 11.085 | 0.000 |
| THYMU3008171 | 20.170 | 0.000 |
| TKIDN2009641 | 5.782 | 21.519 |
| TKIDN2009889 | 35.077 | 0.000 |
| TKIDN2015788 | 5.261 | 0.000 |
| TRACH1000205 | 19.677 | 0.000 |
| TRACH2001549 | 8.457 | 0.000 |
| TRACH2005811 | 2.109 | 0.000 |
| TRACH2006049 | 47.167 | 0.000 |
| TRACH2007834 | 0.504 | 1.877 |
| TRACH2008300 | 10.186 | 0.000 |
| TRACH2025535 | 5.806 | 0.000 |
| TRACH3001427 | 5.571 | 0.000 |
| TRACH3002192 | 4.989 | 2.321 |
| TRACH3004721 | 8.721 | 0.000 |
| TRACH3005294 | 7.428 | 0.000 |
| TRACH3007479 | 1.075 | 0.000 |
| TRACH3008093 | 2.449 | 0.000 |
| TRACH3009455 | 47.167 | 0.000 |
| UTERU2006115 | 7.837 | 0.000 |
| UTERU2019706 | 45.606 | 0.000 |
| UTERU2023039 | 45.606 | 0.000 |
| UTERU2026203 | 45.606 | 0.000 |
| UTERU3005230 | 24.419 | 0.000 |
| UTERU3007640 | 45.606 | 0.000 |
| UTERU3009871 | 36.230 | 0.000 |
| ADRGL2000042 | 2.540 | 18.905 |
| BLADE2006830 | 1.681 | 0.000 |
| BRACE2003609 | 9.090 | 0.000 |
| BRAMY3004800 | 38.061 | 0.000 |
| BRAWH1000369 | 100.000 | 0.000 |
| BRAWH2006207 | 12.943 | 48.175 |
| BRAWH2006395 | 12.446 | 0.000 |
| BRAWH2008993 | 49.811 | 0.000 |
| BRAWH2009393 | 100.000 | 0.000 |
| BRAWH2010552 | 58.488 | 0.000 |
| BRAWH3007441 | 100.000 | 0.000 |
| BRAWH3009017 | 100.000 | 0.000 |
| BRHIP2005271 | 7.083 | 0.000 |
| BRHIP3000017 | 8.819 | 0.000 |
| BRTHA2018443 | 22.098 | 0.000 |
| BRTHA3003000 | 17.150 | 63.832 |
| CTONG2020374 | 31.081 | 0.000 |
| CTONG2020378 | 16.140 | 0.000 |
| CTONG2024031 | 2.584 | 0.000 |
| FCBBF1000509 | 3.732 | 6.945 |
| FEBRA2001990 | 18.144 | 0.000 |
| FEBRA2006519 | 11.891 | 0.000 |
| FEBRA2028516 | 8.007 | 0.000 |
| HCHON2000743 | 6.105 | 0.000 |
| IMR322001879 | 9.638 | 0.000 |
| NT2RI2009583 | 0.224 | 1.665 |
| OCBBF2008144 | 5.768 | 0.000 |
| PERIC2007068 | 3.086 | 0.000 |
| PUAEN2006335 | 12.682 | 0.000 |
| SPLEN2039379 | 5.792 | 0.000 |
| TESTI2015626 | 0.000 | 1.224 |
| TESTI4001984 | 60.471 | 0.000 |
| TESTI4008058 | 8.814 | 0. 000 |
| TESTI4025268 | 60.471 | 0. 000 |
| TESTI4032090 | 60.471 | 0. 000 |
| THYMU3000360 | 39.314 | 0.000 |
| TLIVE2002046 | 5.445 | 0.000 |
| TRACH3000134 | 36.165 | 0.000 |
| UTERU2021820 | 24.929 | 0.000 |
| UTERU2028734 | 21.953 | 0.000 |

The result of comparative analysis of cDNA libraries derived from the hippocampus (BRHIP), and from whole tissues of a normal brain (BRAWH) showed following genes whose expression levels differed between the two.

**Table 6**

| Clone ID | BRAWH | BRHIP |
|---|---|---|
| ASTRO1000009 | 2.611 | 0.000 |
| BLADE2001371 | 0.000 | 12.691 |
| BLADE2008398 | 12.401 | 18.978 |
| BNGH42007788 | 0.000 | 3.141 |
| BRACE1000186 | 4.324 | 2.206 |
| BRACE1000258 | 31.956 | 0.000 |
| BRACE1000533 | 11.795 | 9.627 |
| BRACE2005457 | 58.488 | 0.000 |
| BRACE2010489 | 63.510 | 10.799 |
| BRACE2014657 | 15.451 | 0.000 |
| BRACE2015058 | 0.000 | 8.947 |
| BRACE2018762 | 0.000 | 58.973 |
| BRACE2030341 | 0.000 | 7.057 |
| BRACE2035381 | 10.177 | 20.766 |
| BRACE2044286 | 18.667 | 0.000 |
| BRACE2045954 | 27.309 | 0.000 |
| BRACE3000787 | 32.844 | 0.000 |
| BRACE3003192 | 58.488 | 0.000 |
| BRACE3005499 | 31.276 | 0.000 |
| BRACE3007480 | 19.471 | 52.973 |
| BRACE3009237 | 18.139 | 37.013 |
| BRACE3009724 | 58.488 | 0.000 |
| BRACE3009747 | 2.237 | 2.283 |
| BRACE3010428 | 6.868 | 2.336 |
| BRACE3011271 | 11.036 | 11.259 |
| BRACE3011421 | 28.251 | 0.000 |
| BRACE3012364 | 8.506 | 4.339 |
| BRACE3018963 | 0.000 | 58.973 |
| BRACE3022769 | 4.285 | 4.372 |
| BRACE3026735 | 24.173 | 0.000 |
| BRACE3031838 | 58.488 | 0.000 |
| BRALZ2011796 | 5.511 | 0.000 |
| BRAMY2003008 | 26.445 | 0.000 |
| BRAMY2005052 | 11.612 | 0.000 |
| BRAMY2019300 | 49.811 | 0.000 |
| BRAMY2019963 | 20.428 | 6.947 |
| BRAMY2031317 | 0.000 | 16.004 |
| BRAMY2036567 | 7.474 | 7.626 |
| BRAMY2037823 | 29.664 | 0.000 |
| BRAMY2040592 | 3.482 | 7.105 |
| BRAMY2044078 | 0.000 | 8.947 |
| BRAMY3002803 | 14.428 | 44.161 |
| BRAMY3004224 | 33.027 | 33.695 |
| BRAMY3005091 | 19.193 | 0.000 |
| BRAMY3009811 | 0.000 | 66.943 |
| BRAWH1000127 | 15.983 | 3.261 |
| BRAWH2001395 | 14.290 | 22.297 |
| BRAWH2001671 | 7.605 | 11.638 |
| BRAWH2001940 | 37.398 | 38.155 |
| BRAWH2001973 | 37.398 | 38.155 |
| BRAWH2002560 | 6.454 | 3.292 |
| BRAWH2002761 | 100.000 | 0.000 |
| BRAWH2005315 | 100.000 | 0.000 |
| BRAWH2007658 | 58.101 | 0.000 |
| BRAWH2010000 | 18.745 | 38.249 |
| BRAWH2010084 | 100.000 | 0.000 |
| BRAWH2010536 | 14.718 | 0.000 |
| BRAWH2012162 | 36.060 | 0.000 |
| BRAWH2012326 | 100.000 | 0.000 |
| BRAWH2013294 | 39.442 | 10.060 |
| BRAWH2013871 | 37.485 | 0.000 |
| BRAWH2014414 | 17.865 | 18.227 |
| BRAWH2014645 | 4.228 | 1.078 |
| BRAWH2014662 | 15.521 | 0.000 |
| BRAWH2014876 | 10.473 | 0.000 |
| BRAWH2014954 | 58.488 | 0.000 |
| BRAWH2016221 | 47.417 | 0.000 |
| BRAWH2016439 | 100.000 | 0.000 |
| BRAWH2016702 | 73.807 | 0.000 |
| BRAWH2016724 | 35.119 | 0.000 |
| BRAWH3000078 | 100.000 | 0.000 |
| BRAWH3000100 | 100.000 | 0.000 |
| BRAWH3000314 | 71.553 | 0.000 |
| BRAWH3000491 | 100.000 | 0.000 |
| BRAWH3001326 | 45.606 | 0.000 |
| BRAWH3001475 | 100.000 | 0.000 |
| BRAWH3001891 | 34.539 | 0.000 |
| BRAWH3002574 | 13.222 | 0.000 |
| BRAWH3002600 | 36.800 | 0.000 |
| BRAWH3002819 | 100.000 | 0.000 |
| BRAWH3002821 | 21.953 | 0.000 |
| BRAWH3003522 | 100.000 | 0.000 |
| BRAWH3003555 | 15.229 | 0.000 |
| BRAWH3003727 | 10.055 | 10.259 |
| BRAWH3003801 | 100.000 | 0.000 |
| BRAWH3003992 | 29.008 | 0.000 |
| BRAWH3004453 | 100.000 | 0.000 |
| BRAWH3004666 | 49.499 | 50.501 |
| BRAWH3005132 | 49.811 | 0.000 |
| BRAWH3005422 | 100.000 | 0.000 |
| BRAWH3005912 | 100.000 | 0.000 |
| BRAWH3005981 | 29.324 | 0.000 |
| BRAWH3006548 | 71.018 | 28.982 |
| BRAWH3006792 | 49.499 | 50.501 |
| BRAWH3007221 | 100.000 | 0.000 |
| BRAWH3007506 | 100.000 | 0.000 |
| BRAWH3007592 | 8.966 | 3.659 |
| BRAWH3007726 | 54.530 | 0.000 |
| BRAWH3007783 | 100.000 | 0.000 |
| BRAWH3008341 | 100.000 | 0.000 |
| BRAWH3008697 | 100.000 | 0.000 |
| BRAWH3008931 | 3.463 | 10.601 |
| BRAWH3009297 | 58.488 | 0.000 |
| BRCAN2020710 | 0.000 | 22.176 |
| BRCAN2028355 | 0.000 | 1.507 |
| BRCOC2003213 | 10.381 | 0.000 |
| BRCOC2014033 | 15.633 | 15.950 |
| BRCOC2020142 | 22.014 | 0.000 |
| BRHIP2000691 | 0.000 | 100.000 |
| BRHIP2000819 | 0.000 | 2.204 |
| BRHIP2000826 | 0.000 | 100.000 |
| BRHIP2000920 | 36.630 | 37.371 |
| BRHIP2001074 | 0.000 | 39.976 |
| BRHIP2001805 | 0.000 | 14.757 |
| BRHIP2001927 | 0.000 | 100.000 |
| BRHIP2002122 | 0.000 | 100.000 |
| BRHIP2002172 | 0.000 | 100.000 |
| BRHIP2002346 | 0.000 | 100.000 |
| BRHIP2003242 | 0.000 | 100.000 |
| BRHIP2003786 | 0.000 | 100.000 |
| BRHIP2003917 | 0.000 | 11.981 |
| BRHIP2004312 | 0.000 | 100.000 |
| BRHIP2004359 | 0.000 | 24.839 |
| BRHIP2004814 | 0.000 | 35.391 |
| BRHIP2004883 | 0.000 | 100.000 |
| BRHIP2005236 | 0.000 | 100.000 |
| BRHIP2005354 | 0.000 | 100.000 |
| BRHIP2005600 | 0.000 | 100.000 |
| BRHIP2005719 | 49. 499 | 50. 501 |
| BRHIP2005752 | 0.000 | 58.973 |
| BRHIP2005932 | 0.000 | 100.000 |
| BRHIP2006800 | 0.000 | 100.000 |
| BRHIP2007616 | 0.000 | 100.000 |
| BRHIP2007741 | 6.943 | 3.542 |
| BRHIP2009340 | 0.000 | 100.000 |
| BRHIP2009414 | 0.000 | 100.000 |
| BRHIP2009474 | 0.000 | 47.917 |
| BRHIP2013699 | 0.000 | 21.770 |
| BRHIP2014228 | 29.439 | 15.017 |
| BRHIP2021615 | 0.000 | 100.000 |
| BRHIP2022221 | 0.000 | 47.917 |
| BRHIP2024146 | 3.042 | 5.897 |
| BRHIP2024165 | 0.000 | 100.000 |
| BRHIP2026061 | 0.000 | 31.199 |
| BRHIP2026288 | 0.000 | 22.018 |
| BRHIP202.9176 | 0.000 | 100.000 |
| BRHIP2029393 | 0.000 | 100.000 |
| BRHIP3000339 | 14. 290 | 22.297 |
| BRHIP3000526 | 0.000 | 100.000 |
| BRHIP3001283 | 0.000 | 50.312 |
| BRHIP3006683 | 24.100 | 49.175 |
| BRHIP3007483 | 0.000 | 100.000 |
| BRHIP3007586 | 17.255 | 24.645 |
| BRHIP3008183 | 0.000 | 100.000 |
| BRHIP3008313 | 0.000 | 29.118 |
| BRHIP3008344 | 0.000 | 46.104 |
| BRHIP3008405 | 35.187 | 17.949 |
| BRHIP3008565 | 0.000 | 46.104 |
| BRHIP3008598 | 0.000 | 100.000 |
| BRHIP3008997 | 0.000 | 100.000 |
| BRHIP3009099 | 0.000 | 35.577 |
| BRHIP3009448 | 0.000 | 100.000 |
| BRHIP3011241 | 0.000 | 100.000 |
| BRHIP3013765 | 0.000 | 100.000 |
| BRHIP3013897 | 0.000 | 100.000 |
| BRHIP3015751 | 0.000 | 100.000 |
| BRHIP3016213 | 0.000 | 100.000 |
| BRHIP3018797 | 30.810 | 9.567 |
| BRHIP3020182 | 0.000 | 100.000 |
| BRHIP3024118 | 0.000 | 100.000 |
| BRHIP3024533 | 0.000 | 100.000 |
| BRHIP3024725 | 0.000 | 100.000 |
| BRHIP3025161 | 0.000 | 100.000 |
| BRHIP3025702 | 0.000 | 100.000 |
| BRHIP3026097 | 0.000 | 100.000 |
| BRHIP3027137 | 0.000 | 100.000 |
| BRHIP3027854 | 0.000 | 100.000 |
| BRSSN2000684 | 23.433 | 19.126 |
| BRSSN2004719 | 0.000 | 10.691 |
| BRSSN2008549 | 0.000 | 17.436 |
| BRSSN2011738 | 31.553 | 0.000 |
| BRSSN2014299 | 3.695 | 3.770 |
| BRSTN2008052 | 32.844 | 0.000 |
| BRSTN2015015 | 14.017 | 0.000 |
| BRSTN2016470 | 0.438 | 0.447 |
| BRSTN2018083 | 0.000 | 22.055 |
| BRTHA1000311 | 11.803 | 6.021 |
| BRTHA2002442 | 0.000 | 31.507 |
| BRTHA2008335 | 16.281 | 8.305 |
| BRTHA3000297 | 0.000 | 47.917 |
| BRTHA3001721 | 0.000 | 12. 017 |
| BRTHA3002427 | 8.577 | 2.917 |
| BRTHA3003490 | 1.623 | 0.000 |
| BRTHA3005046 | 0.000 | 47.917 |
| BRTHA3008520 | 47.417 | 0.000 |
| BRTHA3008778 | 0.000 | 25.346 |
| BRTHA3009090 | 0.000 | 12.008 |
| BRTHA3015910 | 0.000 | 15.221 |
| BRTHA3017848 | 47.417 | 0.000 |
| COLON2001721 | 11.065 | 5.644 |
| CTONG1000087 | 0.000 | 1.067 |
| CTONG1000088 | 0.000 | 2.981 |
| CTONG1000467 | 0.000 | 8.766 |
| CTONG2000042 | 0.000 | 3.574 |
| CTONG2008233 | 0.000 | 0.701 |
| CTONG2009423 | 0.000 | 35.391 |
| CTONG2017500 | 2.649 | 0.000 |
| CTONG2019788 | 0.000 | 3.120 |
| CTONG2028124 | 0.503 | 1.026 |
| CTONG3000657 | 3.880 | 11.875 |
| CTONG3001123 | 7.847 | 0.000 |
| CTONG3001370 | 0.000 | 3.574 |
| CTONG3002412 | 0.000 | 10.918 |
| CTONG3004072 | 0.000 | 19.915 |
| CTONG3008894 | 0.000 | 2.966 |
| CTONG3009239 | 0.000 | 2.946 |
| CTONG3009328 | 11.993 | 0.000 |
| DFNES2011499 | 0.000 | 6.330 |
| FCBBF2001183 | 16.537 | 11.810 |
| FCBBF3001977 | 7.448 | 7.599 |
| FEBRA2000253 | 0.000 | 14.934 |
| FEBRA2007544 | 14.689 | 4.995 |
| FEBRA2007801 | 4.937 | 7.556 |
| FEBRA2008287 | 0.000 | 3.007 |
| FEBRA2010719 | 0.000 | 17.703 |
| FEBRA2020886 | 12.124 | 12.369 |
| FEBRA2028618 | 5.082 | 0.000 |
| HCASM2007047 | 3.431 | 0.000 |
| HCHON2000028 | 0.000 | 1.619 |
| HCHON2000244 | 0.658 | 0.671 |
| HCHON2000626 | 2.351 | 0.000 |
| HCHON2001217 | 3.123 | 2.124 |
| HCHON2002676 | 13.647 | 0.000 |
| HCHON2005921 | 0.000 | 12.694 |
| HCHON2006250 | 1.565 | 0.000 |
| HEART1000074 | 0.759 | 0.000 |
| HEART2007031 | 0.000 | 11.593 |
| HHDPC1000118 | 2.096 | 6.415 |
| HLUNG2002465 | 1.209 | 3.702 |
| HLUNG2003003 | 0.000 | 16.306 |
| IMR322000127 | 3.098 | 3.161 |
| IMR322001380 | 0.000 | 2.022 |
| IMR322002035 | 36.176 | 0.000 |
| KIDNE2005543 | 0.000 | 18.168 |
| KIDNE2006580 | 7.013 | 14.310 |
| MESAN2006563 | 0.691 | 0.000 |
| MESAN2012054 | 12. 754 | 15.615 |
| MESTC1000042 | 2.245 | 0.000 |
| NOVAR2001783 | 4.027 | 0.000 |
| NT2NE2006909 | 0.285 | 0.000 |
| NT2RI2008724 | 1.836 | 1.874 |
| NT2RI2012659 | 13.562 | 0. 000 |
| NT2RI2014733 | 24.938 | 0.000 |
| NT2RI2018311 | 0.000 | 21.918 |
| NT2RI3001515 | 0.000 | 11.050 |
| NT2RI3002892 | 8.799 | 16.159 |
| NT2RI3004510 | 0. 000 | 28.761 |
| NT2RI3005724 | 0. 000 | 12. 093 |
| NT2RI3006284 | 4.138 | 0.000 |
| NT2RI3006673 | 19.959 | 10.182 |
| NT2RI3007291 | 0.000 | 12.770 |
| NT2RI3007543 | 1.697 | 0.866 |
| NT2RI3008055 | 44.179 | 0.000 |
| NT2RP7005529 | 14.766 | 15.065 |
| NT2RP7009147 | 14.337 | 1.625 |
| NT2RP7014005 | 5.818 | 0.000 |
| NT2RP7017474 | 18.823 | 0.000 |
| OCBBF2001794 | 4.728 | 0.000 |
| OCBBF2003819 | 0.000 | 21.496 |
| OCBBF2006005 | 9.535 | 9.728 |
| OCBBF2006151 | 0.000 | 11.764 |
| OCBBF2006764 | 15.345 | 0.000 |
| OCBBF2007028 | 9.665 | 11.269 |
| OCBBF2007068 | 0.000 | 45.093 |
| OCBBF2010140 | 32.508 | 0.000 |
| OCBBF2020741 | 0.000 | 28.789 |
| OCBBF2021286 | 18.456 | 18.829 |
| OCBBF2024719 | 0.000 | 30.261 |
| OCBBF2024850 | 4.445 | 4.535 |
| OCBBF2028935 | 5.789 | 7.087 |
| OCBBF2036743 | 11.053 | 0.000 |
| OCBBF2038317 | 19.713 | 0.000 |
| OCBBF3000296 | 0.000 | 19.328 |
| OCBBF3000483 | 11.973 | 8.143 |
| OCBBF3008230 | 29.840 | 0.000 |
| PEBLM2004666 | 3.715 | 0.000 |
| PLACE6001185 | 21.358 | 0.000 |
| PUAEN2005930 | 18.362 | 9.367 |
| PUAEN2006701 | 2.249 | 2.295 |
| PUAEN2007044 | 8.600 | 0.000 |
| PUAEN2009655 | 18.275 | 7.458 |
| SPLEN2010912 | 0.000 | 12.636 |
| SPLEN2012624 | 0.000 | 10.317 |
| SPLEN2028914 | 2.792 | 0.000 |
| SPLEN2031424 | 15.229 | 0.000 |
| SPLEN2031547 | 1.574 | 11.244 |
| SPLEN2034781 | 27.984 | 0.000 |
| SPLEN2036932 | 2.932 | 5.982 |
| SYNOV2014400 | 12.977 | 13.240 |
| SYNOV4002346 | 5.997 | 0.000 |
| SYNOV4002883 | 23.940 | 24.425 |
| SYNOV4007430 | 31.677 | 0.000 |
| SYNOV4008440 | 1.475 | 3.010 |
| TESOP2002451 | 2.375 | 0.000 |
| TESTI2049246 | 0. 000 | 53. 653 |
| TESTI4000014 | 1.964 | 2.551 |
| TESTI4000209 | 2. 649 | 0. 000 |
| TESTI4001100 | 4.098 | 4.180 |
| TESTI4006137 | 25.755 | 0.000 |
| TESTI4008797 | 12.429 | 12. 681 |
| TESTI4009286 | 1.450 | 1.479 |
| TESTI4010377 | 0.000 | 60.949 |
| TESTI4010851 | 3.280 | 3.904 |
| TESTI4010928 | 0. 000 | 34. 221 |
| TESTI4011161 | 0.000 | 21.496 |
| TESTI4013817 | 27.163 | 27. 712 |
| TESTI4014159 | 0.000 | 23.916 |
| TESTI4014694 | 2. 229 | 0. 000 |
| TESTI4014818 | 0. 000 | 60.949 |
| TESTI4021478 | 22.098 | 0.000 |
| TESTI4022936 | 26.445 | 0. 000 |
| TESTI4024420 | 37.398 | 38.155 |
| TESTI4027821 | 60.471 | 0. 000 |
| TESTI4037156 | 0.000 | 0.282 |
| THYMU2001090 | 21.252 | 0.000 |
| THYMU2023967 | 0.000 | 31.165 |
| THYMU2025707 | 0.000 | 9.440 |
| THYMU2031341 | 0.000 | 7.040 |
| THYMU2033308 | 13.964 | 0.000 |
| THYMU2035735 | 1.319 | 2.692 |
| THYMU2037226 | 0.000 | 34.964 |
| THYMU2039315 | 54.530 | 0.000 |
| THYMU3001234 | 11.085 | 0.000 |
| THYMU3001379 | 0.000 | 25.695 |
| THYMU3004835 | 0.000 | 20.041 |
| THYMU3007137 | 0.000 | 37.956 |
| THYMU3008171 | 20.170 | 20.579 |
| TKIDN2009641 | 5.782 | 0.000 |
| TKIDN2009889 | 35.077 | 0.000 |
| TKIDN2015788 | 5.261 | 2.684 |
| TRACH1000205 | 19.677 | 4.015 |
| TRACH2001549 | 8.457 | 21.569 |
| TRACH2005811 | 2.109 | 2.152 |
| TRACH2006049 | 47.167 | 0.000 |
| TRACH2007834 | 0.504 | 0.515 |
| TRACH2008300 | 10.186 | 13.857 |
| TRACH2025535 | 5.806 | 2.369 |
| TRACH3000014 | 0.000 | 18.547 |
| TRACH3001427 | 5.571 | 2.842 |
| TRACH3002192 | 4.989 | 6.999 |
| TRACH3004721 | 8.721 | 2.966 |
| TRACH3005294 | 7.428 | 0.000 |
| TRACH3007479 | 1.075 | 0.000 |
| TRACH3008093 | 2.449 | 0.000 |
| TRACH3009455 | 47.167 | 0.000 |
| TUTER1000122 | 0.000 | 3.330 |
| TUTER2000904 | 0.000 | 5.697 |
| UTERU2004929 | 0.000 | 11.843 |
| UTERU2006115 | 7.837 | 0.000 |
| UTERU2019706 | 45.606 | 0.000 |
| UTERU2021163 | 0.000 | 46.104 |
| UTERU2023039 | 45.606 | 0.000 |
| UTERU2026203 | 45.606 | 0.000 |
| UTERU2030213 | 0.000 | 25.133 |
| UTERU3001572 | 0.000 | 1.862 |
| UTERU3003135 | 0.000 | 33.486 |
| UTERU3005230 | 24.419 | 0.000 |
| UTERU3007640 | 45.606 | 0.000 |
| UTERU3009259 | 0.000 | 46.104 |
| UTERU3009871 | 36.230 | 18.482 |
| ADRGL2000042 | 2.540 | 0.000 |
| BLADE2006830 | 1.681 | 0.000 |
| BRACE2003609 | 9.090 | 0.000 |
| BRAMY3004800 | 38.061 | 46.598 |
| BRAWH1000369 | 100.000 | 0.000 |
| BRAWH2006207 | 12.943 | 0.000 |
| BRAWH2006395 | 12.446 | 0.000 |
| BRAWH2008993 | 49.811 | 0.000 |
| BRAWH2009393 | 100.000 | 0.000 |
| BRAWH2010552 | 58.488 | 0.000 |
| BRAWH3007441 | 100.000 | 0.000 |
| BRAWH3009017 | 100.000 | 0.000 |
| BRHIP2002722 | 0.000 | 100.000 |
| BRHIP2003272 | 0.000 | 100.000 |
| BRHIP2005271 | 7.083 | 14.453 |
| BRHIP2005724 | 0.000 | 100.000 |
| BRHIP2006617 | 0.000 | 100.000 |
| BRHIP2008389 | 0.000 | 100.000 |
| BRHIP2012360 | 0.000 | 100.000 |
| BRHIP2017553 | 0.000 | 100.000 |
| BRHIP2026877 | 0.000 | 30.781 |
| BRHIP3000017 | 8.819 | 4.499 |
| BRHIP3000240 | 0.000 | 32.393 |
| BRHIP3008314 | 0.000 | 100.000 |
| BRHIP3026052 | 0.000 | 45.860 |
| BRTHA2018443 | 22.098 | 0.000 |
| BRTHA3003000 | 17.150 | 0.000 |
| CTONG2020374 | 31.081 | 0.000 |
| CTONG2020378 | 16.140 | 16.467 |
| CTONG2024031 | 2.584 | 0.000 |
| CTONG3004726 | 0.000 | 17.278 |
| FCBBF1000509 | 3.732 | 5.711 |
| FEBRA2001990 | 18.144 | 7.404 |
| FEBRA2006519 | 11.891 | 0.000 |
| FEBRA2028516 | 8.007 | 4.668 |
| HCHON2000743 | 6.105 | 0.000 |
| IMR322001879 | 9.638 | 9.833 |
| NT2RI2009583 | 0.224 | 0.228 |
| OCBBF2006987 | 0.000 | 13.187 |
| OCBBF2008144 | 5.768 | 2.942 |
| OCBBF2030116 | 0.000 | 45.093 |
| PERIC2007068 | 3.086 | 4.723 |
| PUAEN2006335 | 12.682 | 0.000 |
| SPLEN2039379 | 5.792 | 8.864 |
| TESTI2015626 | 0.000 | 0.336 |
| TESTI4000214 | 0.000 | 7.979 |
| TESTI4001984 | 60.471 | 0.000 |
| TESTI4008058 | 8.814 | 0.000 |
| TESTI4013894 | 0.000 | 14.289 |
| TESTI4025268 | 60.471 | 0.000 |
| TESTI4025547 | 0.000 | 60.949 |
| TESTI4026207 | 0.000 | 60.949 |
| TESTI4032090 | 60.471 | 0.000 |
| THYMU3000360 | 39.314 | 0.000 |
| TLIVE2002046 | 5.445 | 1.852 |
| TRACH3000134 | 36.165 | 0.000 |
| UTERU2008040 | 0.000 | 24.014 |
| UTERU2021820 | 24.929 | 0.000 |
| UTERU2028734 | 21.953 | 0.000 |

The result of comparative analysis of cDNA libraries derived from the cerebellum (BRACE), and from whole tissues of a normal brain (BRAWH) showed following genes whose expression levels differed between the two.

**Table 7**

| Clone ID | BRAWH | BRACE |
|---|---|---|
| ADRGL2009146 | 0.000 | 10.913 |
| ADRGL2012038 | 0.000 | 1.459 |
| ASTRO1000009 | 2.611 | 0.000 |
| ASTRO2003960 | 0.000 | 17.003 |
| BLADE1000176 | 0.000 | 16.822 |
| BLADE2004089 | 0.000 | 7.034 |
| BLADE2008398 | 12.401 | 4.401 |
| BRACE1000186 | 4.324 | 1.535 |
| BRACE1000258 | 31.956 | 68.044 |
| BRACE1000533 | 11.795 | 11.720 |
| BRACE1000572 | 0.000 | 100.000 |
| BRACE2003639 | 0.000 | 100.000 |
| BRACE2005457 | 58.488 | 41.512 |
| BRACE2006319 | 0.000 | 4.977 |
| BRACE2008594 | 0.000 | 100.000 |
| BRACE2010489 | 63.510 | 15.026 |
| BRACE2011747 | 0.000 | 100.000 |
| BRACE2014306 | 0.000 | 6.909 |
| BRACE2014475 | 0.000 | 100.000 |
| BRACE2014657 | 15.451 | 76.765 |
| BRACE2015058 | 0.000 | 6.224 |
| BRACE2015314 | 0.000 | 100.000 |
| BRACE2016981 | 0.000 | 16.089 |
| BRACE2018762 | 0.000 | 41.027 |
| BRACE2024627 | 0.000 | 8.689 |
| BRACE2026836 | 0.000 | 4.898 |
| BRACE2027258 | 0.000 | 23.981 |
| BRACE2027970 | 0.000 | 45.981 |
| BRACE2028970 | 0.000 | 100.000 |
| BRACE2029112 | 0.000 | 100.000 |
| BRACE2029849 | 0.000 | 100.000 |
| BRACE2030326 | 0.000 | 100.000 |
| BRACE2030341 | 0.000 | 4.909 |
| BRACE2030884 | 0.000 | 100.000 |
| BRACE2031154 | 0.000 | 39.025 |
| BRACE2031389 | 0.000 | 7.220 |
| BRACE2031527 | 0.000 | 100.000 |
| BRACE2031531 | 0.000 | 100.000 |
| BRACE2031899 | 0.000 | 100.000 |
| BRACE2032044 | 0.000 | 100.000 |
| BRACE2032329 | 0.000 | 100.000 |
| BRACE2032385 | 0.000 | 100.000 |
| BRACE2032538 | 0.000 | 100.000 |
| BRACE2032823 | 0.000 | 100.000 |
| BRACE2033720 | 0.000 | 100.000 |
| BRACE2035381 | 10.177 | 36.117 |
| BRACE2035441 | 0.000 | 100.000 |
| BRACE2036005 | 0.000 | 100.000 |
| BRACE2036096 | 0.000 | 100.000 |
| BRACE2036830 | 0.000 | 100.000 |
| BRACE2036834 | 0.000 | 100.000 |
| BRACE2037847 | 0.000 | 100.000 |
| BRACE2038114 | 0.000 | 100.000 |
| BRACE2038329 | 0.000 | 100.000 |
| BRACE2038551 | 0.000 | 100.000 |
| BRACE2039249 | 0.000 | 100.000 |
| BRACE2039327 | 0.000 | 100.000 |
| BRACE2039475 | 0.000 | 100.000 |
| BRACE2039734 | 0.000 | 100.000 |
| BRACE2040138 | 0.000 | 100.000 |
| BRACE2040325 | 0.000 | 100.000 |
| BRACE2041009 | 0.000 | 100.000 |
| BRACE2041200 | 0.000 | 38.787 |
| BRACE2041264 | 0.000 | 100.000 |
| BRACE2042550 | 0.000 | 100.000 |
| BRACE2043142 | 0.000 | 52.057 |
| BRACE2043248 | 0.000 | 100.000 |
| BRACE2043349 | 0.000 | 100.000 |
| BRACE2043665 | 0.000 | 16.915 |
| BRACE2044286 | 18.667 | 26.499 |
| BRACE2044816 | 0.000 | 100.000 |
| BRACE2044949 | 0.000 | 2.766 |
| BRACE2045300 | 0.000 | 100.000 |
| BRACE2045428 | 0.000 | 100.000 |
| BRACE2045596 | 0.000 | 100.000 |
| BRACE2045772 | 0.000 | 2.055 |
| BRACE2045947 | 0.000 | 100.000 |
| BRACE2045954 | 27.309 | 38.766 |
| BRACE2046251 | 0.000 | 100.000 |
| BRACE2046295 | 0.000 | 100.000 |
| BRACE2047011 | 0.000 | 100.000 |
| BRACE2047350 | 0.000 | 45.981 |
| BRACE2047377 | 0.000 | 100.000 |
| BRACE2047385 | 0.000 | 100.000 |
| BRACE3000071 | 0.000 | 9.532 |
| BRACE3000697 | 0.000 | 100.000 |
| BRACE3000787 | 32.844 | 15.541 |
| BRACE3000840 | 0.000 | 100.000 |
| BRACE3000973 | 0.000 | 28.699 |
| BRACE3001002 | 0.000 | 100.000 |
| BRACE3001217 | 0.000 | 100.000 |
| BRACE3001391 | 0.000 | 100.000 |
| BRACE3001595 | 0.000 | 100.000 |
| BRACE3001754 | 0.000 | 38.787 |
| BRACE3002298 | 0.000 | 100.000 |
| BRACE3002390 | 0.000 | 100.000 |
| BRACE3002508 | 0.000 | 100.000 |
| BRACE3003004 | 0.000 | 100.000 |
| BRACE3003192 | 58.488 | 41.512 |
| BRACE3003595 | 0.000 | 100.000 |
| BRACE3003698 | 0.000 | 45.981 |
| BRACE3004058 | 0.000 | 100.000 |
| BRACE3004113 | 0.000 | 100.000 |
| BRACE3004150 | 0.000 | 2.757 |
| BRACE3004358 | 0.000 | 100.000 |
| BRACE3004435 | 0.000 | 100.000 |
| BRACE3004772 | 0.000 | 100.000 |
| BRACE3004783 | 0.000 | 100.000 |
| BRACE3004843 | 0.000 | 22.581 |
| BRACE3004880 | 0.000 | 100.000 |
| BRACE3005145 | 0.000 | 100.000 |
| BRACE3005225 | 0.000 | 100.000 |
| BRACE3005430 | 0.000 | 100.000 |
| BRACE3005499 | 31.276 | 22.199 |
| BRACE3006185 | 0.000 | 100.000 |
| BRACE3006226 | 0.000 | 100.000 |
| BRACE3006462 | 0.000 | 100.000 |
| BRACE3006872 | 0.000 | 100.000 |
| BRACE3007322 | 0.000 | 100.000 |
| BRACE3007472 | 0.000 | 100.000 |
| BRACE3007480 | 19.471 | 13.820 |
| BRACE3007559 | 0.000 | 100.000 |
| BRACE3007625 | 0.000 | 100.000 |
| BRACE3007642 | 0.000 | 100.000 |
| BRACE3007767 | 0.000 | 100.000 |
| BRACE3008036 | 0.000 | 13.098 |
| BRACE3008092 | 0.000 | 100.000 |
| BRACE3008137 | 0.000 | 100.000 |
| BRACE3008384 | 0.000 | 56.141 |
| BRACE3008720 | 0.000 | 100.000 |
| BRACE3008772 | 0.000 | 37.308 |
| BRACE3009090 | 0.000 | 100.000 |
| BRACE3009237 | 18.139 | 12.875 |
| BRACE3009297 | 0.000 | 100.000 |
| BRACE3009377 | 0.000 | 100.000 |
| BRACE3009574 | 0.000 | 100.000 |
| BRACE3009701 | 0.000 | 100.000 |
| BRACE3009708 | 0.000 | 100.000 |
| BRACE3009724 | 58.488 | 41.512 |
| BRACE3009747 | 2.237 | 1.588 |
| BRACE3010397 | 0.000 | 24.243 |
| BRACE3010428 | 6.868 | 3.250 |
| BRACE3011271 | 11.036 | 39.165 |
| BRACE3011421 | 28.251 | 40.103 |
| BRACE3011505 | 0.000 | 100.000 |
| BRACE3012364 | 8.506 | 6.038 |
| BRACE3012930 | 0.000 | 38.787 |
| BRACE3013119 | 0.000 | 100.000 |
| BRACE3013576 | 0.000 | 100.000 |
| BRACE3013740 | 0.000 | 100.000 |
| BRACE3013780 | 0.000 | 3.404 |
| BRACE3014005 | 0.000 | 100.000 |
| BRACE3014068 | 0.000 | 100.000 |
| BRACE3014231 | 0.000 | 100.000 |
| BRACE3014317 | 0.000 | 100.000 |
| BRACE3014807 | 0.000 | 100.000 |
| BRACE3015027 | 0.000 | 9.209 |
| BRACE3015121 | 0.000 | 100.000 |
| BRACE3015262 | 0.000 | 100. 000 |
| BRACE3015521 | 0.000 | 100.000 |
| BRACE3015894 | 0.000 | 100.000 |
| BRACE3016884 | 0.000 | 9.165 |
| BRACE3018308 | 0.000 | 100.000 |
| BRACE3018963 | 0.000 | 41.027 |
| BRACE3019055 | 0.000 | 100.000 |
| BRACE3019084 | 0.000 | 100.000 |
| BRACE3020194 | 0.000 | 100.000 |
| BRACE3020286 | 0.000 | 100.000 |
| BRACE3020594 | 0.000 | 100.000 |
| BRACE3022769 | 4.285 | 9.124 |
| BRACE3023912 | 0.000 | 100.000 |
| BRACE3024073 | 0.000 | 100.000 |
| BRACE3024659 | 0.000 | 100.000 |
| BRACE3024662 | 0.000 | 100.000 |
| BRACE3025153 | 0.000 | 100.000 |
| BRACE3025457 | 0.000 | 100.000 |
| BRACE3025531 | 0.000 | 100.000 |
| BRACE3025630 | 0.000 | 100.000 |
| BRACE3026008 | 0.000 | 100.000 |
| BRACE3026075 | 0.000 | 100.000 |
| BRACE3026735 | 24.173 | 51.471 |
| BRACE3027242 | 0.000 | 100.000 |
| BRACE3027326 | 0.000 | 100.000 |
| BRACE3027478 | 0.000 | 11.360 |
| BRACE3030103 | 0.000 | 100.000 |
| BRACE3031838 | 58.488 | 41.512 |
| BRACE3032983 | 0.000 | 100.000 |
| BRACE3040856 | 0.000 | 100.000 |
| BRACE3045033 | 0.000 | 100.000 |
| BRALZ2011796 | 5.511 | 3.912 |
| BRAMY2003008 | 26.445 | 0.000 |
| BRAMY2005052 | 11.612 | 8.242 |
| BRAMY2019300 | 49.811 | 0.000 |
| BRAMY2019963 | 20.428 | 0.000 |
| BRAMY2020058 | 0.000 | 19.826 |
| BRAMY2030098 | 0.000 | 29.935 |
| BRAMY2031317 | 0.000 | 33.400 |
| BRAMY2036567 | 7.474 | 26.525 |
| BRAMY2037823 | 29.664 | 21.055 |
| BRAMY2039872 | 0.000 | 14.071 |
| BRAMY2040592 | 3.482 | 0.000 |
| BRAMY2044078 | 0.000 | 6.224 |
| BRAMY2047420 | 0.000 | 0.719 |
| BRAMY3002620 | 0.000 | 41.329 |
| BRAMY3002803 | 14.428 | 0.000 |
| BRAMY3004224 | 33.027 | 0.000 |
| BRAMY3005091 | 19.193 | 13.622 |
| BRAMY3005932 | 0.000 | 41.329 |
| BRAMY4000229 | 0.000 | 25.113 |
| BRAWH1000127 | 15.983 | 4.538 |
| BRAWH2001395 | 14.290 | 3.580 |
| BRAWH2001671 | 7.605 | 8.097 |
| BRAWH2001940 | 37.398 | 0.000 |
| BRAWH2001973 | 37.398 | 0.000 |
| BRAWH2002560 | 6.454 | 0.000 |
| BRAWH2002761 | 100.000 | 0.000 |
| BRAWH2005315 | 100.000 | 0.000 |
| BRAWH2007658 | 58.101 | 0.000 |
| BRAWH2010000 | 18.745 | 0.000 |
| BRAWH2010084 | 100.000 | 0.000 |
| BRAWH2010536 | 14.718 | 0.000 |
| BRAWH2012162 | 36.060 | 0.000 |
| BRAWH2012326 | 100.000 | 0.000 |
| BRAWH2013294 | 39.442 | 13.997 |
| BRAWH2013871 | 37.485 | 0.000 |
| BRAWH2014414 | 17.865 | 0.000 |
| BRAWH2014645 | 4.228 | 3.751 |
| BRAWH2014662 | 15.521 | 11.016 |
| BRAWH2014876 | 10.473 | 7.433 |
| BRAWH2014954 | 58.488 | 41.512 |
| BRAWH2016221 | 47.417 | 0.000 |
| BRAWH2016439 | 100.000 | 0.000 |
| BRAWH2016702 | 73.807 | 26.193 |
| BRAWH2016724 | 35.119 | 0.000 |
| BRAWH3000078 | 100.000 | 0.000 |
| BRAWH3000100 | 100.000 | 0.000 |
| BRAWH3000314 | 71.553 | 0.000 |
| BRAWH3000491 | 100.000 | 0.000 |
| BRAWH3001326 | 45.606 | 0.000 |
| BRAWH3001475 | 100.000 | 0.000 |
| BRAWH3001891 | 34.539 | 36.772 |
| BRAWH3002574 | 13.222 | 0.000 |
| BRAWH3002600 | 36.800 | 26.120 |
| BRAWH3002819 | 100.000 | 0.000 |
| BRAWH3002821 | 21.953 | 0.000 |
| BRAWH3003522 | 100.000 | 0.000 |
| BRAWH3003555 | 15.229 | 5.405 |
| BRAWH3003727 | 10.055 | 21.411 |
| BRAWH3003801 | 100.000 | 0.000 |
| BRAWH3003992 | 29.008 | 0.000 |
| BRAWH3004453 | 100.000 | 0.000 |
| BRAWH3004666 | 49.499 | 0.000 |
| BRAWH3005132 | 49.811 | 0.000 |
| BRAWH3005422 | 100.000 | 0.000 |
| BRAWH3005912 | 100.000 | 0.000 |
| BRAWH3005981 | 29.324 | 0.000 |
| BRAWH3006548 | 71.018 | 0.000 |
| BRAWH3006792 | 49.499 | 0.000 |
| BRAWH3007221 | 100.000 | 0.000 |
| BRAWH3007506 | 100.000 | 0.000 |
| BRAWH3007592 | 8.966 | 6.364 |
| BRAWH3007726 | 54.530 | 0.000 |
| BRAWH3007783 | 100.000 | 0.000 |
| BRAWH3008341 | 100.000 | 0.000 |
| BRAWH3008697 | 100.000 | 0.000 |
| BRAWH3008931 | 3.463 | 4.916 |
| BRAWH3009297 | 58.488 | 41.512 |
| BRCAN2009432 | 0.000 | 1.869 |
| BRCAN2010376 | 0.000 | 23.627 |
| BRCAN2015371 | 0.000 | 15.560 |
| BRCAN2020710 | 0.000 | 7.714 |
| BRCOC2003213 | 10.381 | 7.368 |
| BRCOC2007034 | 0.000 | 16.691 |
| BRCOC2014033 | 15.633 | 0.000 |
| BRCOC2020142 | 22.014 | 0.000 |
| BRHIP2000920 | 36.630 | 25.999 |
| BRHIP2004359 | 0.000 | 34.560 |
| BRHIP2005719 | 49. 499 | 0.000 |
| BRHIP2005752 | 0.000 | 41.027 |
| BRHIP2007741 | 6.943 | 3.696 |
| BRHIP2013699 | 0.000 | 15.145 |
| BRHIP2014228 | 29.439 | 10.447 |
| BRHIP2024146 | 3.042 | 0.864 |
| BRHIP3000339 | 14.290 | 3.580 |
| BRHIP3006683 | 24.100 | 0.000 |
| BRHIP3007586 | 17.255 | 7.348 |
| BRHIP3008313 | 0.000 | 20.257 |
| BRHIP3008405 | 35.187 | 12.487 |
| BRHIP3018797 | 30.810 | 3.803 |
| BRSSN2000684 | 23.433 | 0.000 |
| BRSSN2006892 | 0.000 | 10.990 |
| BRSSN2011262 | 0.000 | 2.000 |
| BRSSN2011738 | 31.553 | 7.465 |
| BRSSN2014299 | 3.695 | 2.623 |
| BRSTN2008052 | 32.844 | 15.541 |
| BRSTN2010750 | 0.000 | 7.220 |
| BRSTN2015015 | 14.017 | 19.897 |
| BRSTN2016470 | 0.438 | 7.146 |
| BRTHA1000311 | 11.803 | 10.472 |
| BRTHA2008335 | 16.281 | 0.000 |
| BRTHA2008955 | 0.000 | 39.025 |
| BRTHA2011194 | 0.000 | 39.025 |
| BRTHA3001721 | 0.000 | 8.360 |
| BRTHA3002427 | 8.577 | 2.029 |
| BRTHA3003490 | 1.623 | 2.304 |
| BRTHA3008520 | 47.417 | 0.000 |
| BRTHA3009090 | 0.000 | 8.354 |
| BRTHA3017848 | 47.417 | 0.000 |
| COLON2001721 | 11.065 | 19.633 |
| CTONG2008233 | 0.000 | 3.412 |
| CTONG2017500 | 2.649 | 0.000 |
| CTONG2028124 | 0.503 | 2.854 |
| CTONG3000657 | 3.880 | 5.508 |
| CTONG3001123 | 7.847 | 0.000 |
| CTONG3005813 | 0.000 | 27.592 |
| CTONG3008894 | 0.000 | 1.032 |
| CTONG3009328 | 11.993 | 0.000 |
| DFNES2011499 | 0.000 | 4.403 |
| FCBBF2001183 | 16.537 | 5.869 |
| FCBBF3001977 | 7.448 | 10.573 |
| FEBRA2006396 | 0.000 | 5.705 |
| FEBRA2007544 | 14.689 | 41.703 |
| FEBRA2007708 | 0.000 | 6.066 |
| FEBRA2007801 | 4.937 | 1.752 |
| FEBRA2008287 | 0.000 | 4.184 |
| FEBRA2020886 | 12.124 | 0.000 |
| FEBRA2021966 | 0.000 | 3.062 |
| FEBRA2026984 | 0.000 | 12.446 |
| FEBRA2028618 | 5.082 | 10.821 |
| HCASM2007047 | 3.431 | 0.000 |
| HCHON2000244 | 0.658 | 0.934 |
| HCHON2000626 | 2.351 | 0.834 |
| HCHON2001217 | 3.123 | 2.216 |
| HCHON2002676 | 13. 647 | 0.000 |
| HCHON2005921 | 0.000 | 2.944 |
| HCHON2006250 | 1.565 | 0.000 |
| HEART1000074 | 0.759 | 1.078 |
| HHDPC1000118 | 2.096 | 0.000 |
| HLUNG2002465 | 1.209 | 0.000 |
| IMR322000127 | 3.098 | 4.398 |
| IMR322001380 | 0.000 | 2.814 |
| IMR322002035 | 36.176 | 0.000 |
| KIDNE2000665 | 0.000 | 1.374 |
| KIDNE2006580 | 7.013 | 14.933 |
| MESAN2006563 | 0.691 | 1.470 |
| MESAN2012054 | 12.754 | 5.432 |
| MESTC1000042 | 2.245 | 0.797 |
| NB9N41000340 | 0.000 | 2.059 |
| NESOP2001752 | 0.000 | 3.228 |
| NOVAR2001783 | 4.027 | 0.000 |
| NT2NE2006909 | 0.285 | 0.000 |
| NT2RI2005166 | 0.000 | 18.511 |
| NT2RI2008724 | 1.836 | 0. 000 |
| NT2RI2012659 | 13.562 | 48.128 |
| NT2RI2014733 | 24.938 | 0.000 |
| NT2RI2019751 | 0.000 | 28.087 |
| NT2RI3002892 | 8.799 | 9.992 |
| NT2RI3003382 | 0. 000 | 28. 087 |
| NT2RI3004510 | 0.000 | 20.009 |
| NT2RI3005724 | 0. 000 | 42. 065 |
| NT2RI3006284 | 4.138 | 1.469 |
| NT2RI3006673 | 19.959 | 0.000 |
| NT2RI3007291 | 0. 000 | 35.535 |
| NT2RI3007543 | 1.697 | 1.204 |
| NT2RI3008055 | 44.179 | 15.678 |
| NT2RP7004123 | 0.000 | 1.362 |
| NT2RP7005529 | 14.766 | 5.240 |
| NT2RP7009147 | 14.337 | 0.000 |
| NT2RP7010599 | 0.000 | 22.750 |
| NT2RP7014005 | 5.818 | 0.000 |
| NT2RP7017474 | 18.823 | 13.360 |
| NTONG2005969 | 0.000 | 4.560 |
| OCBBF2001794 | 4.728 | 0.000 |
| OCBBF2003819 | 0.000 | 14.955 |
| OCBBF2006005 | 9.535 | 0.000 |
| OCBBF2006151 | 0.000 | 4.092 |
| OCBBF2006764 | 15.345 | 21.782 |
| OCBBF2007028 | 9.665 | 8.820 |
| OCBBF2010140 | 32.508 | 0.000 |
| OCBBF2020343 | 0.000 | 36.360 |
| OCBBF2020741 | 0.000 | 20.028 |
| OCBBF2021286 | 18.456 | 6.550 |
| OCBBF2022351 | 0.000 | 36.360 |
| OCBBF2024850 | 4.445 | 1.577 |
| OCBBF2025527 | 0.000 | 36.360 |
| OCBBF2028935 | 5.789 | 1.643 |
| OCBBF2036743 | 11.053 | 15.690 |
| OCBBF2038317 | 19.713 | 0.000 |
| OCBBF3000483 | 11.973 | 14.163 |
| OCBBF3007516 | 0.000 | 2.684 |
| OCBBF3008230 | 29.840 | 0.000 |
| PEBLM2004666 | 3.715 | 0.000 |
| PERIC2000889 | 0.000 | 6.314 |
| PLACE6001185 | 21.358 | 15.159 |
| PUAEN2002489 | 0.000 | 9.262 |
| PUAEN2005930 | 18.362 | 0.000 |
| PUAEN2006701 | 2.249 | 0.000 |
| PUAEN2007044 | 8.600 | 5.087 |
| PUAEN2009655 | 18.275 | 0.000 |
| SPLEN2010912 | 0.000 | 26.371 |
| SPLEN2012624 | 0.000 | 7.178 |
| SPLEN2027268 | 0.000 | 14.361 |
| SPLEN2028914 | 2.792 | 0.000 |
| SPLEN2031424 | 15.229 | 5.405 |
| SPLEN2031547 | 1.574 | 5.587 |
| SPLEN2034781 | 27.984 | 0.000 |
| SPLEN2036932 | 2.932 | 2.081 |
| SPLEN2037194 | 0.000 | 28.977 |
| SYNOV2014400 | 12.977 | 0.000 |
| SYNOV4002346 | 5.997 | 0.000 |
| SYNOV4002883 | 23.940 | 0.000 |
| SYNOV4007430 | 31.677 | 0.000 |
| SYNOV4007671 | 0.000 | 0.520 |
| SYNOV4008440 | 1.475 | 2.094 |
| TESOP2002273 | 0.000 | 2.881 |
| TESOP2002451 | 2.375 | 3.372 |
| TESOP2002950 | 0.000 | 9.399 |
| TESTI1000330 | 0.000 | 52.057 |
| TESTI4000014 | 1.964 | 2. 028 |
| TESTI4000209 | 2. 649 | 0. 000 |
| TESTI4000349 | 0. 000 | 36. 899 |
| TESTI4001100 | 4.098 | 8.725 |
| TESTI4001561 | 0. 000 | 28. 578 |
| TESTI4006137 | 25.755 | 0.000 |
| TESTI4008797 | 12. 429 | 0. 000 |
| TESTI4009286 | 1. 450 | 0. 000 |
| TESTI4010851 | 3. 280 | 2. 716 |
| TESTI4011161 | 0.000 | 14.955 |
| TESTI4013675 | 0.000 | 14.015 |
| TESTI4013817 | 27. 163 | 0.000 |
| TESTI4014159 | 0. 000 | 16. 638 |
| TESTI4014306 | 0.000 | 52.057 |
| TESTI4014694 | 2. 229 | 0. 791 |
| TESTI4021478 | 22. 098 | 0. 000 |
| TESTI4022936 | 26.445 | 0.000 |
| TESTI4024420 | 37.398 | 0. 000 |
| TESTI4027821 | 60.471 | 0. 000 |
| TESTI4037156 | 0.000 | 0.982 |
| TESTI4046819 | 0. 000 | 14.013 |
| THYMU2001090 | 21.252 | 0.000 |
| THYMU2016523 | 0.000 | 23.416 |
| THYMU2023967 | 0.000 | 43.363 |
| THYMU2030264 | 0.000 | 45.981 |
| THYMU2033308 | 13.964 | 0.000 |
| THYMU2035735 | 1.319 | 0.936 |
| THYMU2039315 | 54.530 | 0.000 |
| THYMU2039780 | 0.000 | 45.981 |
| THYMU3001083 | 0.000 | 45.981 |
| THYMU3001234 | 11.085 | 7.868 |
| THYMU3003309 | 0.000 | 20.635 |
| THYMU3006485 | 0.000 | 45.981 |
| THYMU3008171 | 20.170 | 14.316 |
| TKIDN2009641 | 5. 782 | 4.104 |
| TKIDN2009889 | 35.077 | 0.000 |
| TKIDN2015788 | 5.261 | 0.000 |
| TRACH1000205 | 19.677 | 8.380 |
| TRACH2001549 | 8.457 | 0.000 |
| TRACH2005811 | 2.109 | 0.000 |
| TRACH2006049 | 47.167 | 0.000 |
| TRACH2007834 | 0.504 | 0.000 |
| TRACH2008300 | 10.186 | 3.615 |
| TRACH2025535 | 5.806 | 2.472 |
| TRACH3001427 | 5.571 | 3.295 |
| TRACH3002192 | 4.989 | 3.541 |
| TRACH3004721 | 8. 721 | 4.127 |
| TRACH3005294 | 7.428 | 0.000 |
| TRACH3006038 | 0.000 | 38.787 |
| TRACH3006412 | 0.000 | 38.787 |
| TRACH3007479 | 1.075 | 0.000 |
| TRACH3008093 | 2.449 | 3.477 |
| TRACH3009455 | 47.167 | 0.000 |
| TUTER2000904 | 0.000 | 5.944 |
| UTERU2002410 | 0.000 | 0.417 |
| UTERU2006115 | 7.837 | 5.563 |
| UTERU2007520 | 0.000 | 4.761 |
| UTERU2019706 | 45.606 | 0.000 |
| UTERU2023039 | 45.606 | 0.000 |
| UTERU2026203 | 45.606 | 0.000 |
| UTERU3000226 | 0.000 | 37.308 |
| UTERU3001572 | 0.000 | 2.591 |
| UTERU3005230 | 24.419 | 17.332 |
| UTERU3005460 | 0.000 | 37.308 |
| UTERU3005970 | 0.000 | 37.308 |
| UTERU3006308 | 0.000 | 37.308 |
| UTERU3007419 | 0.000 | 37.308 |
| UTERU3007640 | 45.606 | 0.000 |
| UTERU3007913 | 0.000 | 12.951 |
| UTERU3009871 | 36.230 | 0.000 |
| ADRGL2000042 | 2.540 | 7.210 |
| BLADE2006830 | 1.681 | 2.386 |
| BRACE2002589 | 0.000 | 100.000 |
| BRACE2003609 | 9.090 | 6.452 |
| BRACE2009318 | 0.000 | 100.000 |
| BRACE2011677 | 0.000 | 100.000 |
| BRACE2029396 | 0.000 | 100.000 |
| BRACE2037299 | 0.000 | 100.000 |
| BRACE2039823 | 0.000 | 41.329 |
| BRACE2039832 | 0.000 | 100.000 |
| BRACE2043105 | 0.000 | 100.000 |
| BRACE3001058 | 0.000 | 100.000 |
| BRACE3001113 | 0.000 | 18.680 |
| BRACE3003026 | 0.000 | 11.590 |
| BRACE3003053 | 0.000 | 36.360 |
| BRACE3009127 | 0.000 | 100.000 |
| BRACE3010076 | 0.000 | 100.000 |
| BRACE3015829 | 0.000 | 100.000 |
| BRACE3021148 | 0.000 | 100.000 |
| BRAMY3004800 | 38.061 | 0.000 |
| BRAWH1000369 | 100.000 | 0.000 |
| BRAWH2006207 | 12.943 | 9.187 |
| BRAWH2006395 | 12.446 | 0.000 |
| BRAWH2008993 | 49.811 | 0.000 |
| BRAWH2009393 | 100.000 | 0.000 |
| BRAWH2010552 | 58.488 | 41.512 |
| BRAWH3007441 | 100.000 | 0.000 |
| BRAWH3009017 | 100.000 | 0.000 |
| BRCOC2019841 | 0.000 | 16.691 |
| BRHIP2005271 | 7.083 | 0.000 |
| BRHIP3000017 | 8.819 | 0.000 |
| BRHIP3000240 | 0.000 | 67.607 |
| BRTHA2018443 | 22.098 | 0.000 |
| BRTHA3003000 | 17.150 | 0.000 |
| CTONG2020374 | 31.081 | 11.030 |
| CTONG2020378 | 16.140 | 0.000 |
| CTONG2024031 | 2.584 | 3.669 |
| FCBBF1000509 | 3.732 | 0.000 |
| FEBRA2001990 | 18.144 | 15.453 |
| FEBRA2006519 | 11.891 | 25.320 |
| FEBRA2028516 | 8.007 | 1.624 |
| HCHON2000743 | 6.105 | 8.667 |
| IMR322001879 | 9.638 | 0.000 |
| NT2RI2009583 | 0.224 | 5.399 |
| NT2RP8000521 | 0.000 | 37.067 |
| OCBBF2008144 | 5.768 | 4.094 |
| OCBBF2011669 | 0.000 | 36.360 |
| PERIC2007068 | 3.086 | 8.761 |
| PUAEN2006335 | 12.682 | 0.000 |
| SPLEN2039379 | 5.792 | 10.278 |
| SYNOV2021953 | 0.000 | 6.793 |
| TESTI2015626 | 0.000 | 0.467 |
| TESTI4001984 | 60.471 | 0.000 |
| TESTI4008058 | 8.814 | 0.000 |
| TESTI4013894 | 0.000 | 19.881 |
| TESTI4025268 | 60.471 | 0.000 |
| TESTI4032090 | 60.471 | 0.000 |
| THYMU2004284 | 0.000 | 4.768 |
| THYMU2040925 | 0.000 | 45.981 |
| THYMU3000360 | 39.314 | 27.904 |
| TLIVE2002046 | 5.445 | 7.729 |
| TRACH3000134 | 36.165 | 0.000 |
| UTERU2008040 | 0.000 | 8.353 |
| UTERU2011220 | 0.000 | 1.389 |
| UTERU2021820 | 24.929 | 17.694 |
| UTERU2028734 | 21.953 | 0.000 |

The result of comparative analysis of cDNA libraries derived from the thalamus (BRTHA), and from whole tissues of a normal brain (BRAWH) showed following genes whose expression levels differed between the two.

**Table 8**

| Clone ID | BRAWH | BRTHA |
|---|---|---|
| ASTRO1000009 | 2.611 | 0.000 |
| ASTRO3000482 | 0.000 | 24.247 |
| BLADE2008398 | 12.401 | 13.752 |
| BRACE1000186 | 4.324 | 0.000 |
| BRACE1000258 | 31.956 | 0.000 |
| BRACE1000533 | 11.795 | 5.232 |
| BRACE2005457 | 58.488 | 0.000 |
| BRACE2010489 | 63.510 | 0.000 |
| BRACE2014306 | 0.000 | 5.398 |
| BRACE2014657 | 15.451 | 0.000 |
| BRACE2015058 | 0.000 | 19.449 |
| BRACE2031154 | 0.000 | 60.975 |
| BRACE2035381 | 10.177 | 11.286 |
| BRACE2044286 | 18.667 | 0.000 |
| BRACE2045954 | 27.309 | 0.000 |
| BRACE3000787 | 32.844 | 12.141 |
| BRACE3003192 | 58.488 | 0.000 |
| BRACE3005499 | 31.276 | 0.000 |
| BRACE3007480 | 19.471 | 7.197 |
| BRACE3008384 | 0.000 | 43.859 |
| BRACE3009237 | 18.139 | 20.116 |
| BRACE3009724 | 58.488 | 0.000 |
| BRACE3009747 | 2.237 | 2.481 |
| BRACE3010397 | 0.000 | 75.757 |
| BRACE3010428 | 6.868 | 2.539 |
| BRACE3011271 | 11.036 | 0.000 |
| BRACE3011421 | 28.251 | 0.000 |
| BRACE3012364 | 8.506 | 9.433 |
| BRACE3022769 | 4.285 | 4.752 |
| BRACE3026735 | 24.173 | 0.000 |
| BRACE3027478 | 0.000 | 35.497 |
| BRACE3031838 | 58.488 | 0.000 |
| BRALZ2011796 | 5.511 | 6.112 |
| BRAMY2003008 | 26.445 | 0.000 |
| BRAMY2005052 | 11.612 | 25.755 |
| BRAMY2019300 | 49.811 | 0.000 |
| BRAMY2019963 | 20.428 | 7.551 |
| BRAMY2028914 | 0.000 | 19.645 |
| BRAMY2031317 | 0.000 | 34.791 |
| BRAMY2036567 | 7.474 | 0.000 |
| BRAMY2037823 | 29.664 | 0.000 |
| BRAMY2040592 | 3.482 | 0.000 |
| BRAMY2044078 | 0.000 | 19.449 |
| BRAMY3002803 | 14.428 | 0.000 |
| BRAMY3004224 | 33.027 | 0.000 |
| BRAMY3005091 | 19.193 | 0.000 |
| BRAMY4000229 | 0.000 | 39.237 |
| BRAWH1000127 | 15.983 | 7.090 |
| BRAWH2001395 | 14.290 | 11.652 |
| BRAWH2001671 | 7.605 | 12.650 |
| BRAWH2001940 | 37.398 | 0.000 |
| BRAWH2001973 | 37.398 | 0.000 |
| BRAWH2002560 | 6.454 | 0.000 |
| BRAWH2002761 | 100.000 | 0.000 |
| BRAWH2005315 | 100.000 | 0.000 |
| BRAWH2007658 | 58.101 | 0.000 |
| BRAWH2010000 | 18.745 | 0.000 |
| BRAWH2010084 | 100.000 | 0.000 |
| BRAWH2010536 | 14.718 | 0.000 |
| BRAWH2012162 | 36.060 | 0.000 |
| BRAWH2012326 | 100.000 | 0.000 |
| BRAWH2013294 | 39.442 | 0.000 |
| BRAWH2013871 | 37.485 | 0.000 |
| BRAWH2014414 | 17.865 | 9.906 |
| BRAWH2014645 | 4.228 | 1.172 |
| BRAWH2014662 | 15.521 | 17.212 |
| BRAWH2014876 | 10.473 | 58.068 |
| BRAWH2014954 | 58.488 | 0.000 |
| BRAWH2016221 | 47.417 | 52.583 |
| BRAWH2016439 | 100.000 | 0.000 |
| BRAWH2016702 | 73.807 | 0.000 |
| BRAWH2016724 | 35.119 | 0.000 |
| BRAWH3000078 | 100.000 | 0.000 |
| BRAWH3000100 | 100.000 | 0.000 |
| BRAWH3000314 | 71.553 | 0.000 |
| BRAWH3000491 | 100.000 | 0.000 |
| BRAWH3001326 | 45.606 | 0.000 |
| BRAWH3001475 | 100.000 | 0.000 |
| BRAWH3001891 | 34.539 | 0.000 |
| BRAWH3002574 | 13.222 | 0.000 |
| BRAWH3002600 | 36.800 | 0.000 |
| BRAWH3002819 | 100.000 | 0.000 |
| BRAWH3002821 | 21.953 | 0.000 |
| BRAWH3003522 | 100.000 | 0.000 |
| BRAWH3003555 | 15.229 | 8.444 |
| BRAWH3003727 | 10.055 | 0.000 |
| BRAWH3003801 | 100.000 | 0.000 |
| BRAWH3003992 | 29.008 | 0.000 |
| BRAWH3004453 | 100.000 | 0.000 |
| BRAWH3004666 | 49.499 | 0.000 |
| BRAWH3005132 | 49.811 | 0.000 |
| BRAWH3005422 | 100.000 | 0.000 |
| BRAWH3005912 | 100.000 | 0.000 |
| BRAWH3005981 | 29.324 | 0.000 |
| BRAWH3006548 | 71.018 | 0.000 |
| BRAWH3006792 | 49.499 | 0.000 |
| BRAWH3007221 | 100.000 | 0.000 |
| BRAWH3007506 | 100.000 | 0.000 |
| BRAWH3007592 | 8.966 | 0.000 |
| BRAWH3007726 | 54.530 | 0.000 |
| BRAWH3007783 | 100.000 | 0.000 |
| BRAWH3008341 | 100.000 | 0.000 |
| BRAWH3008697 | 100.000 | 0.000 |
| BRAWH3008931 | 3.463 | 0.000 |
| BRAWH3009297 | 58.488 | 0.000 |
| BRCAN2006297 | 0.000 | 15.966 |
| BRCOC2003213 | 10.381 | 0.000 |
| BRCOC2014033 | 15.633 | 0.000 |
| BRCOC2020142 | 22.014 | 0.000 |
| BRHIP2000819 | 0.000 | 4.791 |
| BRHIP2000920 | 36.630 | 0.000 |
| BRHIP2005719 | 49.499 | 0.000 |
| BRHIP2007741 | 6.943 | 5.775 |
| BRHIP2009474 | 0.000 | 52.083 |
| BRHIP2013699 | 0.000 | 23.663 |
| BRHIP2014228 | 29.439 | 0.000 |
| BRHIP2022221 | 0.000 | 52.083 |
| BRHIP2024146 | 3.042 | 7.759 |
| BRHIP3000339 | 14.290 | 11.652 |
| BRHIP3006683 | 24.100 | 26.726 |
| BRHIP3007586 | 17.255 | 11.481 |
| BRHIP3008405 | 35.187 | 0.000 |
| BRHIP3018797 | 30.810 | 10.399 |
| BRSSN2000684 | 23.433 | 10.395 |
| BRSSN2008549 | 0.000 | 18.953 |
| BRSSN2008797 | 0.000 | 22.955 |
| BRSSN2011738 | 31.553 | 0.000 |
| BRSSN2014299 | 3.695 | 0.000 |
| BRSTN2004863 | 0.000 | 11.049 |
| BRSTN2008052 | 32.844 | 12.141 |
| BRSTN2015015 | 14.017 | 15.544 |
| BRSTN2016470 | 0.438 | 0.000 |
| BRTHA1000311 | 11.803 | 9.817 |
| BRTHA2000855 | 0.000 | 100.000 |
| BRTHA2001462 | 0.000 | 100.000 |
| BRTHA2002115 | 0.000 | 100.000 |
| BRTHA2002281 | 0.000 | 100.000 |
| BRTHA2002376 | 0.000 | 100.000 |
| BRTHA2002442 | 0.000 | 68.493 |
| BRTHA2002493 | 0.000 | 100.000 |
| BRTHA2002608 | 0.000 | 100.000 |
| BRTHA2002808 | 0.000 | 12.857 |
| BRTHA2003030 | 0.000 | 100.000 |
| BRTHA2003110 | 0.000 | 100.000 |
| BRTHA2003116 | 0.000 | 100.000 |
| BRTHA2003461 | 0.000 | 2.434 |
| BRTHA2004821 | 0.000 | 100.000 |
| BRTHA2004978 | 0.000 | 100.000 |
| BRTHA2005579 | 0.000 | 100.000 |
| BRTHA2005956 | 0.000 | 100.000 |
| BRTHA2006075 | 0.000 | 100.000 |
| BRTHA2006146 | 0.000 | 100.000 |
| BRTHA2006194 | 0.000 | 100.000 |
| BRTHA2007122 | 0.000 | 100.000 |
| BRTHA2007422 | 0.000 | 100.000 |
| BRTHA2007603 | 0.000 | 100.000 |
| BRTHA2008316 | 0.000 | 100.000 |
| BRTHA2008335 | 16.281 | 9.028 |
| BRTHA2008527 | 0.000 | 100.000 |
| BRTHA2008535 | 0.000 | 100.000 |
| BRTHA2008955 | 0.000 | 60.975 |
| BRTHA2009311 | 0.000 | 100.000 |
| BRTHA2009846 | 0.000 | 100.000 |
| BRTHA2009972 | 0.000 | 100.000 |
| BRTHA2010073 | 0.000 | 100.000 |
| BRTHA2010608 | 0.000 | 48.181 |
| BRTHA2010884 | 0.000 | 100.000 |
| BRTHA2010907 | 0.000 | 100.000 |
| BRTHA2011194 | 0.000 | 60.975 |
| BRTHA2011351 | 0.000 | 100.000 |
| BRTHA2011500 | 0.000 | 100.000 |
| BRTHA2011641 | 0.000 | 52.395 |
| BRTHA2012392 | 0.000 | 38.390 |
| BRTHA2012562 | 0.000 | 100.000 |
| BRTHA2012980 | 0.000 | 13.890 |
| BRTHA2013262 | 0.000 | 100.000 |
| BRTHA2013460 | 0.000 | 100.000 |
| BRTHA2013707 | 0.000 | 100.000 |
| BRTHA2014792 | 0.000 | 100.000 |
| BRTHA2014828 | 0.000 | 100.000 |
| BRTHA2015406 | 0.000 | 100.000 |
| BRTHA2015478 | 0.000 | 100.000 |
| BRTHA2015696 | 0.000 | 100.000 |
| BRTHA2015878 | 0.000 | 100.000 |
| BRTHA2016215 | 0.000 | 38.930 |
| BRTHA2016496 | 0.000 | 100.000 |
| BRTHA2016543 | 0.000 | 100.000 |
| BRTHA2017353 | 0.000 | 100.000 |
| BRTHA2017985 | 0.000 | 49.749 |
| BRTHA2018165 | 0.000 | 100.000 |
| BRTHA2018344 | 0.000 | 100.000 |
| BRTHA2018591 | 0.000 | 100.000 |
| BRTHA2018624 | 0.000 | 100.000 |
| BRTHA2018707 | 0.000 | 57.080 |
| BRTHA2019014 | 0.000 | 100.000 |
| BRTHA2019022 | 0.000 | 100.000 |
| BRTHA2019048 | 0.000 | 100.000 |
| BRTHA3000273 | 0.000 | 57.080 |
| BRTHA3000297 | 0.000 | 52.083 |
| BRTHA3000633 | 0.000 | 37.898 |
| BRTHA3001721 | 0.000 | 13.062 |
| BRTHA3002401 | 0.000 | 100.000 |
| BRTHA3002427 | 8.577 | 15.852 |
| BRTHA3002933 | 0.000 | 100.000 |
| BRTHA3003074 | 0.000 | 37.459 |
| BRTHA3003343 | 0.000 | 100.000 |
| BRTHA3003449 | 0.000 | 100.000 |
| BRTHA3003474 | 0.000 | 100.000 |
| BRTHA3003490 | 1.623 | 1.800 |
| BRTHA3004475 | 0.000 | 52.395 |
| BRTHA3005046 | 0.000 | 52.083 |
| BRTHA3006856 | 0.000 | 100.000 |
| BRTHA3007113 | 0.000 | 100.000 |
| BRTHA3007148 | 0.000 | 100.000 |
| BRTHA3007319 | 0.000 | 100.000 |
| BRTHA3007769 | 0.000 | 100.000 |
| BRTHA3008143 | 0.000 | 100.000 |
| BRTHA3008310 | 0.000 | 100.000 |
| BRTHA3008386 | 0.000 | 100.000 |
| BRTHA3008520 | 47.417 | 52.583 |
| BRTHA3008778 | 0.000 | 27.551 |
| BRTHA3009037 | 0.000 | 100.000 |
| BRTHA3009090 | 0.000 | 13.052 |
| BRTHA3009291 | 0.000 | 100.000 |
| BRTHA3010366 | 0.000 | 100.000 |
| BRTHA3013884 | 0.000 | 100.000 |
| BRTHA3015815 | 0.000 | 100.000 |
| BRTHA3015910 | 0.000 | 33.089 |
| BRTHA3016845 | 0.000 | 100.000 |
| BRTHA3016917 | 0.000 | 100.000 |
| BRTHA3017047 | 0.000 | 100.000 |
| BRTHA3017589 | 0.000 | 100.000 |
| BRTHA3017848 | 47.417 | 52.583 |
| BRTHA3018514 | 0.000 | 100.000 |
| BRTHA3018617 | 0.000 | 100.000 |
| BRTHA3018656 | 0.000 | 100.000 |
| BRTHA3019105 | 0.000 | 100.000 |
| COLON2001721 | 11.065 | 0.000 |
| CTONG1000087 | 0.000 | 3.479 |
| CTONG2008233 | 0.000 | 3.046 |
| CTONG2017500 | 2.649 | 2.938 |
| CTONG2019788 | 0.000 | 3.391 |
| CTONG2023021 | 0.000 | 37.320 |
| CTONG2028124 | 0.503 | 1.672 |
| CTONG3000657 | 3.880 | 8.605 |
| CTONG3001123 | 7.847 | 0.000 |
| CTONG3008894 | 0.000 | 2.418 |
| CTONG3009028 | 0.000 | 1.785 |
| CTONG3009239 | 0.000 | 1.601 |
| CTONG3009328 | 11.993 | 0.000 |
| FCBBF2001183 | 16.537 | 9.169 |
| FCBBF3001977 | 7.448 | 0.000 |
| FCBBF3021576 | 0.000 | 19.062 |
| FEBRA2007544 | 14.689 | 2.715 |
| FEBRA2007801 | 4.937 | 5.475 |
| FEBRA2008287 | 0.000 | 6.537 |
| FEBRA2008360 | 0.000 | 11.338 |
| FEBRA2020886 | 12.124 | 0.000 |
| FEBRA2028618 | 5.082 | 0.000 |
| HCASM2007047 | 3.431 | 0.000 |
| HCHON2000028 | 0.000 | 5.278 |
| HCHON2000212 | 0.000 | 19.062 |
| HCHON2000244 | 0.658 | 0.000 |
| HCHON2000626 | 2.351 | 1.304 |
| HCHON2001084 | 0.000 | 2.546 |
| HCHON2001217 | 3.123 | 5.771 |
| HCHON2002676 | 13.647 | 0.000 |
| HCHON2005921 | 0.000 | 9.198 |
| HCHON2006250 | 1.565 | 1.735 |
| HEART1000074 | 0.759 | 0.000 |
| HEART2007031 | 0.000 | 12.601 |
| HHDPC1000118 | 2.096 | 0.000 |
| HLUNG2001996 | 0.000 | 23.216 |
| HLUNG2002465 | 1.209 | 1.341 |
| IMR322000127 | 3.098 | 0.000 |
| IMR322001380 | 0.000 | 2.198 |
| IMR322002035 | 36.176 | 0.000 |
| KIDNE2002252 | 0.000 | 2.417 |
| KIDNE2005543 | 0.000 | 19.748 |
| KIDNE2006580 | 7.013 | 7.777 |
| KIDNE2011314 | 0.000 | 24.133 |
| MESAN2006563 | 0.691 | 1.532 |
| MESAN2012054 | 12.754 | 0.000 |
| MESTC1000042 | 2.245 | 0.000 |
| NOVAR2001783 | 4.027 | 0.000 |
| NT2NE2006909 | 0.285 | 0.000 |
| NT2RI2008724 | 1.836 | 4.073 |
| NT2RI2012659 | 13.562 | 0.000 |
| NT2RI2014733 | 24.938 | 0.000 |
| NT2RI3002842 | 0. 000 | 20.781 |
| NT2RI3002892 | 8.799 | 13. 661 |
| NT2RI3005403 | 0.000 | 37.898 |
| NT2RI3006284 | 4.138 | 0. 000 |
| NT2RI3006673 | 19.959 | 0. 000 |
| NT2RI3007543 | 1. 697 | 0. 000 |
| NT2RI3008055 | 44.179 | 0.000 |
| NT2RP7004123 | 0.000 | 2.128 |
| NT2RP7005529 | 14.766 | 0.000 |
| NT2RP7009147 | 14.337 | 3.533 |
| NT2RP7014005 | 5.818 | 0.000 |
| NT2RP7017474 | 18.823 | 0.000 |
| NTONG2005969 | 0.000 | 3.562 |
| NTONG2008088 | 0.000 | 11.332 |
| OCBBF2001794 | 4.728 | 0.000 |
| OCBBF2006005 | 9.535 | 21.147 |
| OCBBF2006764 | 15.345 | 0.000 |
| OCBBF2007028 | 9.665 | 18.374 |
| OCBBF2010140 | 32.508 | 0.000 |
| OCBBF2020639 | 0.000 | 25.048 |
| OCBBF2021286 | 18.456 | 10.233 |
| OCBBF2024719 | 0.000 | 32.892 |
| OCBBF2024850 | 4.445 | 7.393 |
| OCBBF2028935 | 5.789 | 2.568 |
| OCBBF2036743 | 11.053 | 12.257 |
| OCBBF2038317 | 19.713 | 10.930 |
| OCBBF3000483 | 11.973 | 0.000 |
| OCBBF3008230 | 29.840 | 33.091 |
| PEBLM2004666 | 3.715 | 0.000 |
| PLACE6001185 | 21.358 | 0.000 |
| PUAEN2002489 | 0.000 | 21.707 |
| PUAEN2005930 | 18.362 | 10.181 |
| PUAEN2006701 | 2.249 | 0.000 |
| PUAEN2007044 | 8.600 | 1.590 |
| PUAEN2009655 | 18.275 | 0.000 |
| RECTM2001347 | 0.000 | 4.600 |
| SKMUS2000757 | 0.000 | 5.480 |
| SPLEN2006122 | 0.000 | 0.934 |
| SPLEN2010912 | 0.000 | 13.734 |
| SPLEN2025491 | 0.000 | 38.930 |
| SPLEN2028914 | 2.792 | 0.000 |
| SPLEN2031424 | 15.229 | 8.444 |
| SPLEN2031547 | 1.574 | 1.746 |
| SPLEN2032154 | 0.000 | 38.930 |
| SPLEN2034781 | 27.984 | 0.000 |
| SPLEN2036821 | 0.000 | 38.930 |
| SPLEN2036932 | 2.932 | 0.000 |
| SYNOV1000374 | 0.000 | 22.005 |
| SYNOV2014400 | 12.977 | 0.000 |
| SYNOV4002346 | 5.997 | 0.000 |
| SYNOV4002883 | 23.940 | 0.000 |
| SYNOV4007430 | 31.677 | 0.000 |
| SYNOV4007671 | 0.000 | 0.813 |
| SYNOV4008440 | 1.475 | 3.272 |
| TESOP2002451 | 2.375 | 0.000 |
| TESTI2049246 | 0.000 | 29.159 |
| TESTI4000014 | 1.964 | 1.188 |
| TESTI4000209 | 2. 649 | 2.938 |
| TESTI4001100 | 4.098 | 4.544 |
| TESTI4002290 | 0. 000 | 62.915 |
| TESTI4006137 | 25.755 | 28.561 |
| TESTI4008797 | 12.429 | 27.567 |
| TESTI4009286 | 1.450 | 0.804 |
| TESTI4010851 | 3. 280 | 3. 031 |
| TESTI4012702 | 0.000 | 2.417 |
| TESTI4013817 | 27.163 | 0.000 |
| TESTI4014159 | 0.000 | 25.996 |
| TESTI4014694 | 2. 229 | 0. 000 |
| TESTI4021478 | 22.098 | 49.012 |
| TESTI4022936 | 26.445 | 0. 000 |
| TESTI4024420 | 37.398 | 0. 000 |
| TESTI4027821 | 60.471 | 0. 000 |
| TESTI4037156 | 0.000 | 0.307 |
| THYMU2001090 | 21.252 | 47.135 |
| THYMU2025707 | 0.000 | 20.521 |
| THYMU2032825 | 0.000 | 20.620 |
| THYMU2033308 | 13.964 | 0.000 |
| THYMU2033787 | 0.000 | 57.080 |
| THYMU2035735 | 1.319 | 0.000 |
| THYMU2039315 | 54.530 | 0.000 |
| THYMU2040975 | 0.000 | 1.948 |
| THYMU3001234 | 11.085 | 12.293 |
| THYMU3001379 | 0.000 | 27.929 |
| THYMU3004835 | 0.000 | 21.784 |
| THYMU3008171 | 20.170 | 0.000 |
| TKIDN2009641 | 5.782 | 12.823 |
| TKIDN2009889 | 35.077 | 0.000 |
| TKIDN2015788 | 5.261 | 2.917 |
| TLIVE2001327 | 0.000 | 5.201 |
| TRACH1000205 | 19.677 | 0.000 |
| TRACH2001549 | 8.457 | 4.689 |
| TRACH2005811 | 2.109 | 3.508 |
| TRACH2006049 | 47.167 | 0.000 |
| TRACH2007834 | 0.504 | 0.559 |
| TRACH2008300 | 10.186 | 11.296 |
| TRACH2023299 | 0.000 | 14.157 |
| TRACH2025535 | 5.806 | 1.288 |
| TRACH3001427 | 5.571 | 1.030 |
| TRACH3002192 | 4.989 | 2.766 |
| TRACH3004068 | 0.000 | 1.581 |
| TRACH3004721 | 8.721 | 3.224 |
| TRACH3005294 | 7.428 | 0.000 |
| TRACH3007479 | 1.075 | 0.000 |
| TRACH3008093 | 2.449 | 2.716 |
| TRACH3009455 | 47.167 | 0.000 |
| TSTOM1000135 | 0.000 | 9.361 |
| TUTER2000904 | 0.000 | 3.096 |
| UTERU2002410 | 0.000 | 1.302 |
| UTERU2006115 | 7.837 | 17.383 |
| UTERU2019706 | 45.606 | 0.000 |
| UTERU2019940 | 0.000 | 48.181 |
| UTERU2023039 | 45.606 | 0.000 |
| UTERU2023175 | 0.000 | 1.651 |
| UTERU2026203 | 45.606 | 0.000 |
| UTERU2030280 | 0.000 | 48.181 |
| UTERU3000899 | 0.000 | 31.292 |
| UTERU3001571 | 0.000 | 48.181 |
| UTERU3001572 | 0.000 | 6.073 |
| UTERU3004709 | 0.000 | 48.181 |
| UTERU3005230 | 24.419 | 0.000 |
| UTERU3005907 | 0.000 | 14.514 |
| UTERU3007640 | 45.606 | 0.000 |
| UTERU3009871 | 36.230 | 0.000 |
| ADRGL2000042 | 2.540 | 0.000 |
| BLADE2006830 | 1.681 | 0.000 |
| BRACE2003609 | 9.090 | 0.000 |
| BRAMY3004800 | 38.061 | 0.000 |
| BRAWH1000369 | 100.000 | 0.000 |
| BRAWH2006207 | 12.943 | 0.000 |
| BRAWH2006395 | 12.446 | 0.000 |
| BRAWH2008993 | 49.811 | 0.000 |
| BRAWH2009393 | 100.000 | 0.000 |
| BRAWH2010552 | 58.488 | 0.000 |
| BRAWH3007441 | 100.000 | 0.000 |
| BRAWH3009017 | 100.000 | 0.000 |
| BRHIP2005271 | 7.083 | 7.855 |
| BRHIP3000017 | 8.819 | 0.000 |
| BRTHA2002133 | 0.000 | 100.000 |
| BRTHA2002702 | 0.000 | 100.000 |
| BRTHA2007060 | 0.000 | 100.000 |
| BRTHA2010033 | 0.000 | 100.000 |
| BRTHA2011321 | 0.000 | 100.000 |
| BRTHA2013426 | 0.000 | 100.000 |
| BRTHA2013610 | 0.000 | 100.000 |
| BRTHA2016318 | 0.000 | 100.000 |
| BRTHA2017364 | 0.000 | 100.000 |
| BRTHA2017972 | 0.000 | 37.427 |
| BRTHA2018011 | 0.000 | 100.000 |
| BRTHA2018443 | 22.098 | 49.012 |
| BRTHA3000296 | 0.000 | 100.000 |
| BRTHA3003000 | 17.150 | 19.019 |
| BRTHA3008826 | 0.000 | 100.000 |
| CTONG2008721 | 0.000 | 37.320 |
| CTONG2020374 | 31.081 | 0.000 |
| CTONG2020378 | 16.140 | 0.000 |
| CTONG2024031 | 2.584 | 0.000 |
| FCBBF1000509 | 3.732 | 6.208 |
| FEBRA2001990 | 18.144 | 8.048 |
| FEBRA2006519 | 11.891 | 0.000 |
| FEBRA2028516 | 8.007 | 1.268 |
| HCHON2000743 | 6.105 | 6.771 |
| HSYRA2005628 | 0.000 | 7.437 |
| IMR322001879 | 9.638 | 0.000 |
| NT2RI2009583 | 0.224 | 1.489 |
| OCBBF2008144 | 5.768 | 3.198 |
| PERIC2007068 | 3.086 | 1.711 |
| PUAEN2006335 | 12.682 | 0.000 |
| SPLEN2016932 | 0.000 | 38.930 |
| SPLEN2039379 | 5.792 | 3.212 |
| SYNOV2006620 | 0.000 | 33.957 |
| TESTI4001984 | 60.471 | 0.000 |
| TESTI4008058 | 8.814 | 0.000 |
| TESTI4025268 | 60.471 | 0.000 |
| TESTI4032090 | 60.471 | 0.000 |
| THYMU3000360 | 39.314 | 0.000 |
| TLIVE2002046 | 5.445 | 12.076 |
| TRACH3000134 | 36.165 | 0.000 |
| UTERU2021820 | 24.929 | 27.645 |
| UTERU2028734 | 21.953 | 0.000 |

The result of comparative analysis of cDNA libraries derived from the amygdale (BRAMY), and from whole tissues of a normal brain (BRAWH) showed following genes whose expression levels differed between the two.

**Table 9**

| Clone ID | BRAWH | BRAMY |
|---|---|---|
| ASTRO1000009 | 2.611 | 0.000 |
| BLADE2008398 | 12.401 | 0.000 |
| BRACE1000186 | 4.324 | 0.000 |
| BRACE1000258 | 31.956 | 0.000 |
| BRACE1000533 | 11.795 | 4.754 |
| BRACE2005457 | 58.488 | 0.000 |
| BRACE2006319 | 0.000 | 3.532 |
| BRACE2010489 | 63.510 | 10.665 |
| BRACE2014657 | 15.451 | 7.784 |
| BRACE2015058 | 0.000 | 8.836 |
| BRACE2027258 | 0.000 | 34.044 |
| BRACE2030341 | 0.000 | 6.969 |
| BRACE2031389 | 0.000 | 30.747 |
| BRACE2035381 | 10. 177 | 10. 254 |
| BRACE2044286 | 18.667 | 0.000 |
| BRACE2045954 | 27.309 | 0.000 |
| BRACE3000787 | 32.844 | 0.000 |
| BRACE3000973 | 0.000 | 13.581 |
| BRACE3003192 | 58.488 | 0.000 |
| BRACE3005499 | 31.276 | 0.000 |
| BRACE3007480 | 19.471 | 6.540 |
| BRACE3008036 | 0.000 | 18.594 |
| BRACE3009237 | 18.139 | 0.000 |
| BRACE3009724 | 58.488 | 0.000 |
| BRACE3009747 | 2.237 | 0.000 |
| BRACE3010428 | 6.868 | 3.460 |
| BRACE3011271 | 11.036 | 11.120 |
| BRACE3011421 | 28.251 | 0.000 |
| BRACE3012364 | 8.506 | 0.000 |
| BRACE3013780 | 0.000 | 4.833 |
| BRACE3022769 | 4.285 | 4.318 |
| BRACE3026735 | 24.173 | 24.356 |
| BRACE3027478 | 0.000 | 16.126 |
| BRACE3031838 | 58.488 | 0.000 |
| BRALZ2011796 | 5.511 | 0.000 |
| BRAMY2001473 | 0.000 | 100.000 |
| BRAMY2003008 | 26.445 | 26.646 |
| BRAMY2004771 | 0.000 | 100.000 |
| BRAMY2005052 | 11.612 | 46.801 |
| BRAMY2017528 | 0.000 | 100.000 |
| BRAMY2019300 | 49.811 | 50.189 |
| BRAMY2019963 | 20.428 | 6.861 |
| BRAMY2019985 | 0.000 | 100.000 |
| BRAMY2020058 | 0.000 | 28.146 |
| BRAMY2020270 | 0.000 | 100.000 |
| BRAMY2021498 | 0.000 | 100.000 |
| BRAMY2028856 | 0.000 | 100.000 |
| BRAMY2028914 | 0.000 | 17.849 |
| BRAMY2029602 | 0.000 | 100.000 |
| BRAMY2030098 | 0.000 | 42.496 |
| BRAMY2030109 | 0.000 | 100.000 |
| BRAMY2030702 | 0.000 | 100.000 |
| BRAMY2030703 | 0.000 | 100.000 |
| BRAMY2030799 | 0.000 | 100.000 |
| BRAMY2031317 | 0.000 | 15.805 |
| BRAMY2031377 | 0.000 | 100.000 |
| BRAMY2031442 | 0.000 | 100.000 |
| BRAMY2032014 | 0.000 | 100.000 |
| BRAMY2032242 | 0.000 | 100.000 |
| BRAMY2032317 | 0.000 | 60.652 |
| BRAMY2033003 | 0.000 | 100.000 |
| BRAMY2033116 | 0.000 | 100.000 |
| BRAMY2033267 | 0.000 | 100.000 |
| BRAMY2033594 | 0.000 | 100.000 |
| BRAMY2034185 | 0.000 | 100.000 |
| BRAMY2034920 | 0.000 | 100.000 |
| BRAMY2034993 | 0.000 | 100.000 |
| BRAMY2036387 | 0.000 | 13.490 |
| BRAMY2036396 | 0.000 | 100.000 |
| BRAMY2036567 | 7.474 | 22.594 |
| BRAMY2036699 | 0.000 | 100.000 |
| BRAMY2036913 | 0.000 | 100.000 |
| BRAMY2037823 | 29.664 | 29.890 |
| BRAMY2038100 | 0.000 | 100.000 |
| BRAMY2038484 | 0.000 | 100.000 |
| BRAMY2038846 | 0.000 | 47.355 |
| BRAMY2038904 | 0.000 | 60.652 |
| BRAMY2039872 | 0.000 | 19.976 |
| BRAMY2040478 | 0.000 | 100.000 |
| BRAMY2040592 | 3.482 | 7.017 |
| BRAMY2041261 | 0.000 | 100.000 |
| BRAMY2041378 | 0.000 | 100.000 |
| BRAMY2041542 | 0.000 | 100.000 |
| BRAMY2042612 | 0.000 | 100.000 |
| BRAMY2042641 | 0.000 | 100.000 |
| BRAMY2042760 | 0.000 | 100.000 |
| BRAMY2042918 | 0.000 | 100.000 |
| BRAMY2044078 | 0.000 | 8.836 |
| BRAMY2044246 | 0.000 | 100.000 |
| BRAMY2045036 | 0.000 | 100.000 |
| BRAMY2046478 | 0.000 | 100.000 |
| BRAMY2046742 | 0.000 | 100.000 |
| BRAMY2046989 | 0.000 | 100.000 |
| BRAMY2047169 | 0.000 | 100.000 |
| BRAMY2047420 | 0.000 | 2.041 |
| BRAMY2047676 | 0.000 | 100.000 |
| BRAMY2047746 | 0.000 | 100.000 |
| BRAMY2047751 | 0.000 | 100.000 |
| BRAMY2047765 | 0.000 | 100.000 |
| BRAMY2047884 | 0.000 | 100.000 |
| BRAMY3000206 | 0.000 | 100.000 |
| BRAMY3000213 | 0.000 | 100.000 |
| BRAMY3001401 | 0.000 | 100.000 |
| BRAMY3001794 | 0.000 | 100.000 |
| BRAMY3002312 | 0.000 | 100.000 |
| BRAMY3002620 | 0.000 | 58.671 |
| BRAMY3002803 | 14.428 | 14.538 |
| BRAMY3002805 | 0.000 | 100.000 |
| BRAMY3004224 | 33.027 | 33.278 |
| BRAMY3004672 | 0.000 | 100.000 |
| BRAMY3004900 | 0.000 | 100.000 |
| BRAMY3004919 | 0.000 | 100.000 |
| BRAMY3005091 | 19.193 | 19.339 |
| BRAMY3005932 | 0.000 | 58.671 |
| BRAMY3006297 | 0.000 | 100.000 |
| BRAMY3007206 | 0.000 | 100.000 |
| BRAMY3007609 | 0.000 | 100.000 |
| BRAMY3008466 | 0.000 | 100.000 |
| BRAMY3008505 | 0.000 | 100.000 |
| BRAMY3008650 | 0.000 | 100.000 |
| BRAMY3009811 | 0.000 | 33.057 |
| BRAMY3010411 | 0.000 | 100.000 |
| BRAMY4000095 | 0.000 | 14.721 |
| BRAMY4000229 | 0.000 | 35.650 |
| BRAMY4000277 | 0.000 | 100.000 |
| BRAWH1000127 | 15.983 | 9.663 |
| BRAWH2001395 | 14.290 | 2.541 |
| BRAWH2001671 | 7.605 | 0.000 |
| BRAWH2001940 | 37.398 | 0.000 |
| BRAWH2001973 | 37.398 | 0.000 |
| BRAWH2002560 | 6.454 | 6.503 |
| BRAWH2002761 | 100.000 | 0.000 |
| BRAWH2005315 | 100.000 | 0.000 |
| BRAWH2007658 | 58.101 | 0.000 |
| BRAWH2010000 | 18.745 | 0.000 |
| BRAWH2010084 | 100.000 | 0.000 |
| BRAWH2010536 | 14.718 | 0.000 |
| BRAWH2012162 | 36.060 | 0.000 |
| BRAWH2012326 | 100.000 | 0.000 |
| BRAWH2013294 | 39.442 | 0.000 |
| BRAWH2013871 | 37.485 | 0.000 |
| BRAWH2014414 | 17.865 | 54.002 |
| BRAWH2014645 | 4.228 | 2.130 |
| BRAWH2014662 | 15.521 | 0.000 |
| BRAWH2014876 | 10.473 | 0.000 |
| BRAWH2014954 | 58.488 | 0.000 |
| BRAWH2016221 | 47.417 | 0.000 |
| BRAWH2016439 | 100.000 | 0.000 |
| BRAWH2016702 | 73.807 | 0.000 |
| BRAWH2016724 | 35.119 | 0.000 |
| BRAWH3000078 | 100.000 | 0.000 |
| BRAWH3000100 | 100.000 | 0.000 |
| BRAWH3000314 | 71.553 | 0.000 |
| BRAWH3000491 | 100.000 | 0.000 |
| BRAWH3001326 | 45.606 | 0.000 |
| BRAWH3001475 | 100.000 | 0.000 |
| BRAWH3001891 | 34.539 | 17.401 |
| BRAWH3002574 | 13.222 | 0.000 |
| BRAWH3002600 | 36.800 | 37.080 |
| BRAWH3002819 | 100.000 | 0.000 |
| BRAWH3002821 | 21.953 | 0.000 |
| BRAWH3003522 | 100.000 | 0.000 |
| BRAWH3003555 | 15.229 | 0.000 |
| BRAWH3003727 | 10.055 | 0.000 |
| BRAWH3003801 | 100.000 | 0.000 |
| BRAWH3003992 | 29.008 | 0.000 |
| BRAWH3004453 | 100.000 | 0.000 |
| BRAWH3004666 | 49.499 | 0.000 |
| BRAWH3005132 | 49.811 | 50.189 |
| BRAWH3005422 | 100.000 | 0.000 |
| BRAWH3005912 | 100.000 | 0.000 |
| BRAWH3005981 | 29.324 | 0.000 |
| BRAWH3006548 | 71.018 | 0.000 |
| BRAWH3006792 | 49.499 | 0.000 |
| BRAWH3007221 | 100.000 | 0.000 |
| BRAWH3007506 | 100.000 | 0.000 |
| BRAWH3007592 | 8.966 | 1.807 |
| BRAWH3007726 | 54.530 | 0.000 |
| BRAWH3007783 | 100.000 | 0.000 |
| BRAWH3008341 | 100.000 | 0.000 |
| BRAWH3008697 | 100.000 | 0.000 |
| BRAWH3008931 | 3.463 | 6.980 |
| BRAWH3009297 | 58.488 | 0.000 |
| BRCAN2014881 | 0.000 | 30.516 |
| BRCAN2017717 | 0.000 | 10.719 |
| BRCOC2000670 | 0.000 | 22.144 |
| BRCOC2003213 | 10.381 | 0.000 |
| BRCOC2014033 | 15.633 | 0.000 |
| BRCOC2020142 | 22.014 | 0.000 |
| BRHIP2000920 | 36.630 | 0.000 |
| BRHIP2005719 | 49.499 | 0.000 |
| BRHIP2007741 | 6.943 | 8.745 |
| BRHIP2014228 | 29.439 | 0.000 |
| BRHIP2024146 | 3.042 | 2.759 |
| BRHIP2026061 | 0.000 | 30.812 |
| BRHIP3000339 | 14.290 | 2.541 |
| BRHIP3001283 | 0.000 | 49.688 |
| BRHIP3006683 | 24.100 | 0.000 |
| BRHIP3007586 | 17.255 | 3.477 |
| BRHIP3008405 | 35.187 | 0.000 |
| BRHIP3018797 | 30.810 | 9.448 |
| BRSSN2000684 | 23.433 | 0.000 |
| BRSSN2004496 | 0.000 | 21.304 |
| BRSSN2011738 | 31.553 | 0.000 |
| BRSSN2014299 | 3.695 | 0.000 |
| BRSTN2008052 | 32.844 | 0.000 |
| BRSTN2010750 | 0.000 | 30.747 |
| BRSTN2015015 | 14.017 | 0.000 |
| BRSTN2016470 | 0.438 | 0.441 |
| BRTHA1000311 | 11.803 | 14.866 |
| BRTHA2008335 | 16.281 | 0.000 |
| BRTHA2011641 | 0.000 | 47.605 |
| BRTHA3001721 | 0.000 | 23.737 |
| BRTHA3002427 | 8.577 | 5.761 |
| BRTHA3003490 | 1.623 | 0.000 |
| BRTHA3004475 | 0.000 | 47.605 |
| BRTHA3008520 | 47.417 | 0.000 |
| BRTHA3009090 | 0.000 | 11.859 |
| BRTHA3017848 | 47.417 | 0.000 |
| COLON2001721 | 11.065 | 11.149 |
| CTONG1000087 | 0.000 | 3.161 |
| CTONG2008233 | 0.000 | 0.692 |
| CTONG2017500 | 2.649 | 2.669 |
| CTONG2028124 | 0.503 | 0.506 |
| CTONG3000657 | 3.880 | 0.000 |
| CTONG3001123 | 7.847 | 0.000 |
| CTONG3008894 | 0.000 | 4.395 |
| CTONG3009239 | 0.000 | 4.364 |
| CTONG3009328 | 11.993 | 0.000 |
| FCBBF2001183 | 16.537 | 9.998 |
| FCBBF3001977 | 7.448 | 15.010 |
| FEBRA2007544 | 14.689 | 0.000 |
| FEBRA2007801 | 4.937 | 0.000 |
| FEBRA2008287 | 0.000 | 2.970 |
| FEBRA2010719 | 0.000 | 17.484 |
| FEBRA2020886 | 12.124 | 0.000 |
| FEBRA2025427 | 0.000 | 4.995 |
| FEBRA2028618 | 5.082 | 5.121 |
| HCASM2007047 | 3.431 | 0.000 |
| HCHON2000244 | 0.658 | 0.663 |
| HCHON2000626 | 2.351 | 0.000 |
| HCHON2001217 | 3.123 | 0.000 |
| HCHON2002676 | 13.647 | 0.000 |
| HCHON2006250 | 1.565 | 0.000 |
| HCHON2008112 | 0.000 | 13.736 |
| HEART1000074 | 0.759 | 0.000 |
| HHDPC1000118 | 2.096 | 0.000 |
| HLUNG2002465 | 1.209 | 4.875 |
| HSYRA2009075 | 0.000 | 1.212 |
| IMR322000127 | 3.098 | 10.926 |
| IMR322001380 | 0.000 | 11.984 |
| IMR322002035 | 36.176 | 0.000 |
| KIDNE2000665 | 0.000 | 1.951 |
| KIDNE2006580 | 7.013 | 7.066 |
| MESAN2006563 | 0.691 | 0.000 |
| MESAN2012054 | 12.754 | 0.000 |
| MESAN2015515 | 0.000 | 2.677 |
| MESTC1000042 | 2.245 | 0.000 |
| NOVAR2001783 | 4.027 | 0.000 |
| NT2NE2005890 | 0.000 | 35.735 |
| NT2NE2006909 | 0.285 | 0.287 |
| NT2RI2008724 | 1.836 | 1.850 |
| NT2RI2012659 | 13.562 | 13.665 |
| NT2RI2014733 | 24.938 | 0.000 |
| NT2RI3001515 | 0.000 | 21.825 |
| NT2RI3002892 | 8.799 | 1.773 |
| NT2RI3005724 | 0.000 | 5.972 |
| NT2RI3006284 | 4.138 | 0.000 |
| NT2RI3006673 | 19.959 | 10.055 |
| NT2RI3007543 | 1.697 | 0.855 |
| NT2RI3008055 | 44.179 | 0.000 |
| NT2RP7005529 | 14.766 | 7.439 |
| NT2RP7009147 | 14.337 | 0.000 |
| NT2RP7014005 | 5.818 | 0.000 |
| NT2RP7017474 | 18.823 | 0.000 |
| NTONG2005969 | 0.000 | 6.473 |
| OCBBF1000254 | 0.000 | 44.784 |
| OCBBF2001794 | 4.728 | 4.764 |
| OCBBF2006005 | 9.535 | 0.000 |
| OCBBF2006764 | 15.345 | 15.461 |
| OCBBF2007028 | 9.665 | 6.956 |
| OCBBF2007114 | 0.000 | 21.540 |
| OCBBF2010140 | 32.508 | 0.000 |
| OCBBF2021286 | 18.456 | 0.000 |
| OCBBF2023162 | 0.000 | 31.080 |
| OCBBF2024850 | 4.445 | 2.239 |
| OCBBF2028935 | 5.789 | 1.167 |
| OCBBF2035214 | 0.000 | 44.784 |
| OCBBF2036743 | 11.053 | 11.137 |
| OCBBF2038317 | 19.713 | 0.000 |
| OCBBF3000483 | 11.973 | 4.021 |
| OCBBF3008230 | 29.840 | 0.000 |
| PEBLM2004666 | 3.715 | 3.744 |
| PERIC2000889 | 0.000 | 8.963 |
| PERIC2003720 | 0.000 | 13.012 |
| PLACE6001185 | 21.358 | 0.000 |
| PUAEN2005930 | 18.362 | 0.000 |
| PUAEN2006701 | 2.249 | 0.000 |
| PUAEN2007044 | 8.600 | 1.444 |
| PUAEN2009174 | 0.000 | 2.856 |
| PUAEN2009655 | 18.275 | 0.000 |
| SKNMC2002402 | 0.000 | 7.428 |
| SKNSH2000482 | 0.000 | 9.821 |
| SPLEN2001599 | 0.000 | 7.190 |
| SPLEN2002467 | 0.000 | 6.565 |
| SPLEN2028914 | 2.792 | 0.000 |
| SPLEN2029912 | 0.000 | 1.646 |
| SPLEN2031424 | 15.229 | 0.000 |
| SPLEN2031547 | 1.574 | 4.759 |
| SPLEN2034781 | 27.984 | 0.000 |
| SPLEN2036932 | 2.932 | 0.000 |
| SPLEN2038345 | 0.000 | 29.627 |
| SYNOV2014400 | 12.977 | 0.000 |
| SYNOV4002346 | 5.997 | 0.000 |
| SYNOV4002883 | 23.940 | 0.000 |
| SYNOV4007430 | 31.677 | 0.000 |
| SYNOV4007671 | 0.000 | 0.739 |
| SYNOV4008440 | 1.475 | 0.000 |
| TESOP2002451 | 2.375 | 0.000 |
| TESTI2009474 | 0.000 | 2.036 |
| TESTI4000014 | 1.964 | 1.799 |
| TESTI4000209 | 2.649 | 2.669 |
| TESTI4001100 | 4.098 | 4.129 |
| TESTI4006137 | 25.755 | 0.000 |
| TESTI4008797 | 12.429 | 0.000 |
| TESTI4009286 | 1.450 | 0.000 |
| TESTI4010851 | 3.280 | 2.203 |
| TESTI4013817 | 27.163 | 27.369 |
| TESTI4014159 | 0.000 | 11.810 |
| TESTI4014694 | 2.229 | 0.000 |
| TESTI4021478 | 22.098 | 0.000 |
| TESTI4022936 | 26.445 | 26.646 |
| TESTI4024420 | 37.398 | 0.000 |
| TESTI4027821 | 60.471 | 0.000 |
| TESTI4029836 | 0.000 | 60.652 |
| TESTI4037156 | 0.000 | 1.394 |
| TESTI4037188 | 0.000 | 60.652 |
| THYMU2001090 | 21.252 | 0.000 |
| THYMU2014353 | 0.000 | 37.663 |
| THYMU2033308 | 13.964 | 0.000 |
| THYMU2035735 | 1.319 | 0.000 |
| THYMU2037226 | 0.000 | 17.265 |
| THYMU2039315 | 54.530 | 0.000 |
| THYMU3001234 | 11.085 | 0.000 |
| THYMU3001379 | 0.000 | 25.376 |
| THYMU3004835 | 0.000 | 19.793 |
| THYMU3008171 | 20.170 | 0.000 |
| TKIDN2009641 | 5.782 | 5.826 |
| TKIDN2009889 | 35.077 | 0.000 |
| TKIDN2015788 | 5.261 | 5.301 |
| TLIVE2004320 | 0.000 | 12.783 |
| TRACH1000205 | 19.677 | 3.965 |
| TRACH2001549 | 8.457 | 4.260 |
| TRACH2001684 | 0.000 | 17.948 |
| TRACH2005811 | 2.109 | 0.000 |
| TRACH2006049 | 47.167 | 0.000 |
| TRACH2007834 | 0.504 | 1.016 |
| TRACH2008300 | 10.186 | 11.974 |
| TRACH2025344 | 0.000 | 19.846 |
| TRACH2025535 | 5.806 | 0.000 |
| TRACH2025911 | 0.000 | 47.355 |
| TRACH3001427 | 5.571 | 3.742 |
| TRACH3002192 | 4.989 | 1.885 |
| TRACH3004068 | 0.000 | 1.436 |
| TRACH3004721 | 8.721 | 5.858 |
| TRACH3005294 | 7.428 | 0.000 |
| TRACH3007479 | 1.075 | 1.084 |
| TRACH3008093 | 2.449 | 0.000 |
| TRACH3009455 | 47.167 | 0.000 |
| TUTER2000904 | 0.000 | 2.813 |
| UTERU2002410 | 0.000 | 0.592 |
| UTERU2004929 | 0.000 | 11.697 |
| UTERU2006115 | 7.837 | 0.000 |
| UTERU2007520 | 0.000 | 6.758 |
| UTERU2019706 | 45.606 | 0.000 |
| UTERU2023039 | 45.606 | 0.000 |
| UTERU2026203 | 45.606 | 0.000 |
| UTERU3001572 | 0.000 | 1.839 |
| UTERU3001766 | 0.000 | 45.794 |
| UTERU3005230 | 24.419 | 0.000 |
| UTERU3007640 | 45.606 | 0.000 |
| UTERU3009517 | 0.000 | 45.794 |
| UTERU3009871 | 36.230 | 0.000 |
| ADRGL2000042 | 2.540 | 5.118 |
| BLADE2006830 | 1.681 | 0.000 |
| BRACE2003609 | 9.090 | 18.317 |
| BRACE2039823 | 0.000 | 58.671 |
| BRAMY2019111 | 0.000 | 30.516 |
| BRAMY2035070 | 0.000 | 54.718 |
| BRAMY2035449 | 0.000 | 100.000 |
| BRAMY2035718 | 0.000 | 100.000 |
| BRAMY2038516 | 0.000 | 12.770 |
| BRAMY2039341 | 0.000 | 100.000 |
| BRAMY2040159 | 0.000 | 100.000 |
| BRAMY2041434 | 0.000 | 100.000 |
| BRAMY2045471 | 0.000 | 100.000 |
| BRAMY3004800 | 38.061 | 15.340 |
| BRAWH1000369 | 100.000 | 0.000 |
| BRAWH2006207 | 12.943 | 0.000 |
| BRAWH2006395 | 12.446 | 0.000 |
| BRAWH2008993 | 49.811 | 50.189 |
| BRAWH2009393 | 100.000 | 0.000 |
| BRAWH2010552 | 58.488 | 0.000 |
| BRAWH3007441 | 100.000 | 0.000 |
| BRAWH3009017 | 100.000 | 0.000 |
| BRHIP2005271 | 7.083 | 7.137 |
| BRHIP3000017 | 8.819 | 0.000 |
| BRTHA2018443 | 22.098 | 0.000 |
| BRTHA3003000 | 17.150 | 0.000 |
| CTONG2020374 | 31.081 | 0.000 |
| CTONG2020378 | 16.140 | 0.000 |
| CTONG2024031 | 2.584 | 0.000 |
| FCBBF1000509 | 3.732 | 13.160 |
| FEBRA2001990 | 18.144 | 7.313 |
| FEBRA2006519 | 11.891 | 11.982 |
| FEBRA2028516 | 8.007 | 2.305 |
| HCHON2000508 | 0.000 | 0.376 |
| HCHON2000743 | 6.105 | 0.000 |
| IMR322001879 | 9.638 | 0.000 |
| NT2RI2009583 | 0.224 | 0.902 |
| OCBBF2008144 | 5.768 | 2.906 |
| PERIC2007068 | 3.086 | 0.000 |
| PUAEN2006335 | 12.682 | 0.000 |
| SPLEN2039379 | 5.792 | 11.672 |
| TESTI2015626 | 0.000 | 0.331 |
| TESTI2026647 | 0.000 | 23.811 |
| TESTI4001984 | 60.471 | 0.000 |
| TESTI4008058 | 8.814 | 0.000 |
| TESTI4013894 | 0.000 | 14.112 |
| TESTI4025268 | 60.471 | 0.000 |
| TESTI4032090 | 60.471 | 0.000 |
| THYMU3000360 | 39.314 | 0.000 |
| TKIDN2018926 | 0.000 | 21.396 |
| TLIVE2002046 | 5.445 | 10.972 |
| TRACH3000134 | 36.165 | 0.000 |
| UTERU2008040 | 0.000 | 11.858 |
| UTERU2021820 | 24.929 | 0.000 |
| UTERU2028734 | 21.953 | 0.000 |

The result of comparative analysis of cDNA libraries derived from breast tumor (TBAES), and normal breast (BEAST) showed the following genes whose expression levels differed between the two.

**Table 10**

| Clone ID | BEAST | TBAES |
|---|---|---|
| ASTRO2002842 | 0.000 | 67.001 |
| BRACE3016884 | 0.000 | 90.835 |
| BRSSN2011262 | 61.476 | 0.000 |
| BRTHA2008335 | 0.000 | 57.267 |
| HCHON2000244 | 0.000 | 4.627 |
| HCHON2006250 | 0.000 | 11.007 |
| HEART1000010 | 0.000 | 24.034 |
| MESAN2012054 | 0.000 | 35.889 |
| NT2RP7000466 | 0.000 | 85.728 |
| NT2RP7009147 | 0.000 | 11.206 |
| OCBBF2021020 | 0.000 | 18.756 |
| PEBLM2002749 | 0.000 | 48.344 |
| PEBLM2004666 | 0.000 | 26.137 |
| SPLEN2001599 | 0.000 | 50.196 |
| SPLEN2031547 | 0.000 | 11.076 |
| STOMA1000189 | 0.000 | 22.002 |
| TBAES2001171 | 0.000 | 100.000 |
| TBAES2001220 | 0.000 | 100.000 |
| TBAES2001229 | 0.000 | 100.000 |
| TBAES2001258 | 0.000 | 66.863 |
| TBAES2001492 | 0.000 | 100.000 |
| TBAES2001751 | 0.000 | 100.000 |
| TBAES2002197 | 0.000 | 54.528 |
| TBAES2003550 | 0.000 | 100.000 |
| TBAES2004055 | 0.000 | 100.000 |
| TBAES2005157 | 0.000 | 100.000 |
| TBAES2005543 | 0.000 | 100.000 |
| TBAES2006568 | 0.000 | 100.000 |
| TBAES2007964 | 0.000 | 100.000 |
| TEST 14000014 | 0.000 | 1.256 |
| TESTI4037156 | 0.000 | 3.893 |
| TRACH3002192 | 0.000 | 4.387 |
| TRACH3004068 | 0.000 | 10.026 |
| TSTOM2000553 | 0.000 | 11.859 |
| UTERU2002410 | 0.000 | 8.261 |
| BRAWH2006395 | 0.000 | 87.554 |
| NT2RI2009583 | 4.881 | 0.000 |
| STOMA2004893 | 0.000 | 50.829 |
| TBAES2000932 | 0.000 | 100.000 |

The result of comparative analysis of cDNA libraries derived cervical tumor (TCERX), and normal cervical duct (CERVX) showed the following genes whose expression levels differed between the two.

**Table 11**

| Clone ID | CERVX | TCERX |
|---|---|---|
| BLADE2007666 | 70.405 | 0.000 |
| BRAMY2047420 | 0.000 | 21.378 |
| BRCAN2007409 | 0.000 | 63.318 |
| BRSTN2016470 | 0.000 | 9.238 |
| CERVX1000042 | 100.000 | 0.000 |
| CERVX2002006 | 100.000 | 0.000 |
| MESAN2006563 | 14.270 | 0.000 |
| PROST2018090 | 44.955 | 0.000 |
| TCERX2000613 | 0.000 | 85.503 |
| TESTI4037156 | 0. 000 | 5.840 |
| THYMU2031341 | 71.304 | 0.000 |
| UTERU2004688 | 22.977 | 70.393 |
| CERVX2002013 | 100.000 | 0.000 |
| NT2RI2009583 | 0. 000 | 9.443 |

The result of comparative analysis of cDNA libraries derived from colon tumor (TCOLN), and normal colon (COLON) showed the following genes whose expression levels differed between the two.

**Table 12**

| Clone ID | COLON | TCOLN |
|---|---|---|
| BRACE3015027 | 0.000 | 55.019 |
| BRAMY2040592 | 24.447 | 0.000 |
| BRSTN2016470 | 6.147 | 9.281 |
| COLON1000030 | 100.000 | 0.000 |
| COLON2000470 | 50.006 | 0.000 |
| COLON2000568 | 100.000 | 0.000 |
| COLON2001721 | 38.840 | 0.000 |
| COLON2002443 | 31.658 | 0.000 |
| COLON2002520 | 100.000 | 0.000 |
| COLON2003043 | 100.000 | 0.000 |
| COLON2004478 | 100.000 | 0.000 |
| COLON2005126 | 100.000 | 0.000 |
| COLON2005772 | 100.000 | 0.000 |
| COLON2006282 | 100.000 | 0.000 |
| COLON2009499 | 100.000 | 0.000 |
| OCBBF2028935 | 8.128 | 0.000 |
| PLACE7000514 | 33.393 | 0.000 |
| RECTM2000433 | 24.395 | 0.000 |
| SYNOV4007671 | 5.146 | 0.000 |
| TCOLN2002278 | 0.000 | 100.000 |
| TESTI2052693 | 23.507 | 35.495 |
| TESTI4037156 | 1.943 | 0.000 |
| THYMU2031368 | 0.000 | 96.216 |
| TRACH2025535 | 16.304 | 0.000 |
| CTONG1000113 | 79.033 | 0.000 |
| NT2RI2009583 | 0.000 | 4.744 |
| NT2RI2018448 | 44.206 | 0.000 |
| TESTI2015626 | 2.309 | 0.000 |

The result of comparative analysis of cDNA libraries derived from esophageal tumor (TESOP), and normal esophagus (NESOP) showed the following genes whose expression levels differed between the two.

**Table 13**

| Clone ID | NESOP | TESOP |
|---|---|---|
| BRACE2030341 | 0.000 | 47.323 |
| BRAMY2047420 | 21.553 | 0.000 |
| BRHIP2003917 | 0.000 | 80.343 |
| BRTHA2003461 | 0.000 | 15.017 |
| CTONG2013178 | 0.000 | 64.748 |
| D3OST3000169 | 0.000 | 6.696 |
| FEBRA2025427 | 0.000 | 16.960 |
| HCHON2000244 | 0.000 | 4.501 |
| HHDPC1000118 | 0.000 | 14.339 |
| NESOP2000744 | 100.000 | 0.000 |
| NESOP2001433 | 100.000 | 0.000 |
| NESOP2001656 | 85.181 | 0.000 |
| NESOP2001694 | 100.000 | 0.000 |
| NESOP2001752 | 96.772 | 0.000 |
| NESOP2002738 | 100.000 | 0.000 |
| NT2RI3006284 | 0.000 | 14.156 |
| NT2RP7009147 | 33.894 | 0.000 |
| PLACE6019932 | 92.281 | 0.000 |
| SYNOV2005216 | 0.000 | 66.809 |
| TESOP1000127 | 0.000 | 65.101 |
| TESOP2000801 | 0.000 | 100.000 |
| TESOP2001122 | 0.000 | 100.000 |
| TESOP2001166 | 0.000 | 100.000 |
| TESOP2001345 | 0.000 | 100.000 |
| TESOP2001605 | 0.000 | 66.809 |
| TESOP2001818 | 0.000 | 100.000 |
| TESOP2001849 | 0.000 | 100.000 |
| TESOP2001865 | 0.000 | 100.000 |
| TESOP2001953 | 0.000 | 89.137 |
| TESOP2002273 | 0.000 | 27.770 |
| TESOP2002451 | 0.000 | 16.251 |
| TESOP2002489 | 0.000 | 100.000 |
| TESOP2002539 | 0.000 | 100.000 |
| TESOP2002950 | 0.000 | 90.601 |
| TESOP2003273 | 0.000 | 100.000 |
| TESOP2003753 | 0.000 | 100.000 |
| TESOP2004114 | 0.000 | 100.000 |
| TESOP2005285 | 0.000 | 100.000 |
| TESOP2005485 | 0.000 | 100.000 |
| TESOP2005579 | 0.000 | 100.000 |
| TESOP2006041 | 0.000 | 100.000 |
| TESOP2006060 | 0.000 | 100.000 |
| TESOP2006068 | 0.000 | 100.000 |
| TESOP2006670 | 0.000 | 79.728 |
| TESOP2006746 | 0.000 | 100.000 |
| TESOP2007052 | 0.000 | 100.000 |
| TESOP2007262 | 0.000 | 100.000 |
| TESOP2007636 | 0.000 | 100.000 |
| TESOP2007688 | 0.000 | 100.000 |
| TESOP2009121 | 0.000 | 100.000 |
| TESOP2009555 | 0.000 | 100.000 |
| TESTI4009286 | 15.424 | 9.920 |
| TESTI4010851 | 0.000 | 3.740 |
| THYMU2040975 | 0.000 | 12.019 |
| TRACH2005811 | 0.000 | 14.431 |
| UTERU2023175 | 0.000 | 10.186 |
| CTONG2016942 | 0.000 | 78.602 |
| NT2RI2009583 | 0.000 | 1.531 |
| TESOP2000390 | 0.000 | 100.000 |
| TESOP2001796 | 0.000 | 100.000 |
| TESOP2005199 | 0.000 | 100.000 |
| TESOP2006398 | 0.000 | 100.000 |
| TESOP2006865 | 0.000 | 100.000 |
| TESOP2007384 | 0.000 | 13.734 |
| TESTI2015626 | 0.000 | 2.250 |
| TRACH2000862 | 0.000 | 39.606 |

The result of comparative analysis of cDNA libraries derived from kidney tumor (TKIDN), and normal kidney (KIDNE) showed the following genes whose expression levels differed between the two.

**Table 14**

| Clone ID | KIDNE | TKIDN |
|---|---|---|
| ASTRO2018373 | 0.000 | 51.654 |
| BRACE1000186 | 0.000 | 16.007 |
| BRACE2014306 | 0.000 | 18.017 |
| BRACE2015058 | 0.000 | 16.230 |
| BRACE2016981 | 0.000 | 83.911 |
| BRACE2043665 | 83.085 | 0.000 |
| BRACE3008036 | 0.000 | 68.309 |
| BRACE3010428 | 3.991 | 0.000 |
| BRACE3022769 | 0.000 | 5.287 |
| BRAMY2019963 | 0.000 | 25.206 |
| BRAMY2044078 | 0.000 | 16.230 |
| BRAWH1000127 | 0.000 | 11.833 |
| BRAWH2001395 | 0.000 | 9.335 |
| BRAWH2001671 | 0.000 | 14.075 |
| BRAWH2013294 | 0.000 | 36.500 |
| BRAWH2014645 | 0.000 | 3.913 |
| BRHIP2024146 | 0.000 | 7.882 |
| BRHIP3000339 | 0.000 | 9.335 |
| BRSSN2000684 | 0.000 | 17.348 |
| BRSSN2004719 | 0.000 | 19.395 |
| BRSSN2018581 | 0.000 | 49.863 |
| BRSTN2016470 | 0.763 | 0.810 |
| BRTHA1000311 | 0.000 | 10.923 |
| BRTHA3002427 | 0.000 | 10. 582 |
| CTONG1000087 | 3.646 | 0.000 |
| CTONG2028124 | 3.504 | 0.000 |
| CTONG3000657 | 0.000 | 14.362 |
| CTONG3008894 | 2.534 | 0.000 |
| FCBBF2001183 | 0.000 | 12.243 |
| FEBRA2008287 | 0.000 | 10.911 |
| HCASM2001301 | 0.000 | 35.796 |
| HCHON2000028 | 0.000 | 5.873 |
| HCHON2000244 | 4.587 | 0.000 |
| HEART1000074 | 2.646 | 0.000 |
| HHDPC1000118 | 7.307 | 0.000 |
| HSYRA2008376 | 0.000 | 5.788 |
| KIDNE1000064 | 100.000 | 0.000 |
| KIDNE2000665 | 13.498 | 0.000 |
| KIDNE2000722 | 100.000 | 0.000 |
| KIDNE2000832 | 32.592 | 0.000 |
| KIDNE2000846 | 100.000 | 0.000 |
| KIDNE2001361 | 80.699 | 0.000 |
| KIDNE2001847 | 12.687 | 0.000 |
| KIDNE2002252 | 7.600 | 0.000 |
| KIDNE2002991 | 100.000 | 0.000 |
| KIDNE2003837 | 100.000 | 0.000 |
| KIDNE2005543 | 62.084 | 0.000 |
| KIDNE2006580 | 48.900 | 0.000 |
| KIDNE2010264 | 100.000 | 0.000 |
| KIDNE2011314 | 75.867 | 0.000 |
| KIDNE2011532 | 100.000 | 0.000 |
| KIDNE2011635 | 48.502 | 51.498 |
| KIDNE2012945 | 48.732 | 0.000 |
| KIDNE2013095 | 100.000 | 0.000 |
| NESOP2001656 | 0.000 | 14.819 |
| NTONG2005969 | 0.000 | 11.891 |
| PEBLM2004666 | 12.953 | 0.000 |
| SKMUS2000757 | 0.000 | 18.292 |
| STOMA1000189 | 16.356 | 0.000 |
| SYNOV4007671 | 2.556 | 5.427 |
| TBAES2001258 | 33.137 | 0.000 |
| TESTI4000014 | 1. 245 | 0. 000 |
| TESTI4001100 | 0.000 | 15.168 |
| TESTI4012702 | 7.600 | 0.000 |
| TESTI4046819 | 0. 000 | 73. 082 |
| THYMU2032014 | 57.532 | 0.000 |
| TKIDN2000701 | 0.000 | 84.991 |
| TKIDN2002424 | 0.000 | 100.000 |
| TKIDN2002632 | 0.000 | 100.000 |
| TKIDN2003044 | 0.000 | 100.000 |
| TKIDN2004386 | 0.000 | 100.000 |
| TKIDN2005934 | 0.000 | 100.000 |
| TKIDN2005947 | 0.000 | 100.000 |
| TKIDN2006525 | 0.000 | 100.000 |
| TKIDN2006852 | 0.000 | 100.000 |
| TKIDN2007667 | 0.000 | 100.000 |
| TKIDN2009092 | 0.000 | 100.000 |
| TKIDN2009641 | 0.000 | 21.402 |
| TKIDN2009889 | 0.000 | 64.923 |
| TKIDN2010934 | 0.000 | 74.873 |
| TKIDN2012824 | 0.000 | 100.000 |
| TKIDN2013287 | 0.000 | 64.067 |
| TKIDN2014757 | 0.000 | 100.000 |
| TKIDN2014771 | 0.000 | 100.000 |
| TKIDN2015263 | 0.000 | 100.000 |
| TKIDN2015788 | 0.000 | 9.737 |
| TKIDN2016309 | 0.000 | 100.000 |
| TKIDN2019116 | 0.000 | 100.000 |
| TRACH2001443 | 0.000 | 34.396 |
| TRACH2001684 | 62.100 | 0.000 |
| TRACH2007834 | 1.758 | 3.734 |
| TRACH2008300 | 0.000 | 6.284 |
| TRACH3001427 | 0.000 | 3.437 |
| UTERU2002410 | 0.000 | 2.173 |
| UTERU2023175 | 5.190 | 0.000 |
| UTERU3001572 | 0.000 | 6.758 |
| BLADE2006830 | 0.000 | 6.222 |
| BRALZ2017844 | 0.000 | 50.604 |
| CTONG2028758 | 0.000 | 59.532 |
| FCBBF1000509 | 0.000 | 6.907 |
| FEBRA2001990 | 0.000 | 13.433 |
| FEBRA2028516 | 0.000 | 4.234 |
| HCHON2000508 | 0.000 | 1.382 |
| MESAN2005303 | 0.000 | 29.326 |
| NT2RI2009583 | 10.920 | 0.828 |
| TESTI2015626 | 0.000 | 4.869 |
| TKIDN2008778 | 0.000 | 100.000 |
| TKIDN2012771 | 0.000 | 100.000 |
| TKIDN2018926 | 0.000 | 78.604 |

The result of compar derived from liver tumor showed the following genes between the two.

**Table 15**

| Clone ID | LIVER | TLIVE |
|---|---|---|
| BRCAN2018935 | 79.072 | 0.000 |
| BRSTN2016470 | 1.897 | 0.000 |
| BRTHA2012980 | 0.000 | 86.110 |
| BRTHA3002427 | 0.000 | 19.655 |
| CTONG2028124 | 0.000 | 3.455 |
| LIVER2007415 | 100.000 | 0.000 |
| NT2RI2008724 | 0.000 | 12.626 |
| SPLEN2012624 | 43.831 | 0.000 |
| SPLEN2033098 | 0.000 | 34.211 |
| TESOP2002451 | 0.000 | 16.330 |
| TLIVE2000023 | 0.000 | 100.000 |
| TLIVE2001327 | 0.000 | 64.491 |
| TLIVE2001828 | 0.000 | 89.183 |
| TLIVE2001927 | 0.000 | 100.000 |
| TLIVE2002336 | 0.000 | 100.000 |
| TLIVE2002338 | 0.000 | 100.000 |
| TLIVE2002690 | 0.000 | 41.431 |
| TLIVE2003197 | 0.000 | 100.000 |
| TLIVE2003225 | 0.000 | 100.000 |
| TLIVE2003381 | 0.000 | 100.000 |
| TLIVE2003970 | 0.000 | 15.901 |
| TLIVE2004110 | 0.000 | 70.982 |
| TLIVE2004320 | 0.000 | 87.217 |
| TLIVE2004601 | 0.000 | 100.000 |
| TLIVE2005180 | 0.000 | 100.000 |
| TLIVE2006236 | 0.000 | 100.000 |
| TLIVE2006529 | 0.000 | 100.000 |
| TLIVE2007132 | 0.000 | 100.000 |
| TLIVE2007528 | 0.000 | 100.000 |
| TLIVE2007816 | 0.000 | 100.000 |
| TLIVE2008083 | 0.000 | 100.000 |
| TLIVE2008229 | 0.000 | 91.317 |
| TLIVE2009541 | 0.000 | 100.000 |
| UTERU2002410 | 0.000 | 4.037 |
| UTERU2005621 | 19.540 | 0.000 |
| LIVER2000247 | 100.000 | 0.000 |
| NT2RI2009583 | 1.939 | 4.615 |
| TESTI2015626 | 0.000 | 2.261 |
| TLIVE2001684 | 0.000 | 100.000 |
| TLIVE2002046 | 0.000 | 12.478 |
| TLIVE2007607 | 0.000 | 100.000 |

The result of comparative analysis of cDNA libraries derived from lung tumor (TLUNG), and normal lung (HLUNG) showed the following genes whose expression levels differed between the two.

**Table 16**

| Clone ID | HLUNG | TLUNG |
|---|---|---|
| BRCAN2021028 | 38.589 | 0.000 |
| BRHIP2000819 | 7.923 | 0.000 |
| BRSTN2016470 | 0.803 | 0.000 |
| CTONG1000087 | 3.835 | 0.000 |
| CTONG2028124 | 9.217 | 0.000 |
| HCHON2006250 | 0.000 | 32.381 |
| HEART1000074 | 2.784 | 0.000 |
| HLUNG1000017 | 100.000 | 0.000 |
| HLUNG2000014 | 100.000 | 0.000 |
| HLUNG2001996 | 76.784 | 0.000 |
| HLUNG2002465 | 4.436 | 0.000 |
| HLUNG2002958 | 100.000 | 0.000 |
| HLUNG2003003 | 29.311 | 0.000 |
| HLUNG2003872 | 100.000 | 0.000 |
| HLUNG2010464 | 100.000 | 0.000 |
| HLUNG2011041 | 100.000 | 0.000 |
| HLUNG2011298 | 35.254 | 0.000 |
| HLUNG2012049 | 100.000 | 0.000 |
| HLUNG2012287 | 100.000 | 0.000 |
| HLUNG2012727 | 100.000 | 0.000 |
| HLUNG2013204 | 100.000 | 0.000 |
| HLUNG2013304 | 100.000 | 0.000 |
| HLUNG2013622 | 100.000 | 0.000 |
| HLUNG2013851 | 100.000 | 0.000 |
| HLUNG2014262 | 100.000 | 0.000 |
| HLUNG2014288 | 100.000 | 0.000 |
| HLUNG2014449 | 100.000 | 0.000 |
| HLUNG2015617 | 100.000 | 0.000 |
| HLUNG2017350 | 100.000 | 0.000 |
| HLUNG2017546 | 12.944 | 0.000 |
| HLUNG2017806 | 100.000 | 0.000 |
| HLUNG2019058 | 100.000 | 0.000 |
| HSYRA2008376 | 11.470 | 0.000 |
| KIDNE2012945 | 51.268 | 0.000 |
| NT2RI2003993 | 13.924 | 0.000 |
| NT2RP7013795 | 0.000 | 89.568 |
| OCBBF3000483 | 14.638 | 0.000 |
| SPLEN2028914 | 10.242 | 0.000 |
| SPLEN2031547 | 5.775 | 0.000 |
| SYNOV4007671 | 2.689 | 0.000 |
| TESOP1000127 | 34.899 | 0.000 |
| TESTI2003573 | 27.394 | 0.000 |
| TESTI4000014 | 0.655 | 0.000 |
| TESTI4037156 | 2.030 | 0.000 |
| TRACH2005811 | 3.868 | 0.000 |
| TRACH3004068 | 5.227 | 0.000 |
| UTERU2005621 | 8.268 | 0.000 |
| FEBRA2028516 | 4.195 | 0.000 |
| HCHON2000508 | 1.370 | 0.000 |
| HLUNG2013350 | 100.000 | 0.000 |
| HLUNG2015418 | 76.605 | 0.000 |
| HLUNG2015548 | 100.000 | 0.000 |
| HLUNG2016862 | 100.000 | 0.000 |
| NT2RI2009583 | 0.000 | 13.890 |
| TESTI2015626 | 2.412 | 0.000 |
| TRACH2019672 | 76.605 | 0.000 |

The result of comparative analysis of cDNA libraries derived from ovary tumor (TOVER), and normal ovary (NOVER) showed the following genes whose expression levels differed between the two.

**Table 17A**

| Clone ID | NOVAR | TOVAR |
|---|---|---|
| CTONG2019788 | 72.878 | 0.000 |
| FEBRA2014213 | 0.000 | 85.773 |
| HLUNG2017546 | 84.114 | 0.000 |
| NOVAR2000136 | 100.000 | 0.000 |
| NOVAR2000710 | 83.961 | 0.000 |
| NOVAR2000962 | 100.000 | 0.000 |
| NOVAR2001108 | 100.000 | 0.000 |
| NOVAR2001783 | 95.973 | 0.000 |
| OCBBF3007516 | 90.145 | 0.000 |
| TESTI2052693 | 39.903 | 0.000 |
| TOVAR2000649 | 0.000 | 100.000 |
| TOVAR2001281 | 0.000 | 100.000 |
| TOVAR2001730 | 0.000 | 100.000 |
| TOVAR2002247 | 0.000 | 100.000 |
| TOVAR2002549 | 0.000 | 100.000 |
| TRACH3004068 | 0.000 | 31.044 |

The result of comparative analysis of cDNA libraries derived from ovary tumor (TOVER), and normal ovary (NOVER) showed the following genes whose expression levels differed between the two.

**Table 17B**

| Clone ID | NOVAR | TOVAR |
|---|---|---|
| TESTI2015626 | 7.838 | 7.163 |

The gene has no different expression levels between normal and diseased ovary. However, the gene showed significantly different expression level in both ovary tumor and normal ovary, compared with other tissues (as in Example 9). Thus, the gene are ovary-specific gene and can be used as diagnostic marker because its association with the disease.

The result of comparative analysis of cDNA libraries derived from stomach tumor (TSTOM) and normal stomach (STOMA) showed the following genes whose expression levels differed between the two.

**Table 18**

| Clone ID | STOMA | TSTOM |
|---|---|---|
| BRACE2024627 | 83.309 | 0.000 |
| BRAWH2014645 | 0.000 | 22.702 |
| BRCAN2028355 | 0.000 | 31.728 |
| BRHIP2000819 | 14.701 | 46.392 |
| BRSTN2016470 | 1.490 | 0.000 |
| BRTHA3003490 | 0.000 | 34.864 |
| COLON2002443 | 30.687 | 0.000 |
| HEART1000010 | 23.250 | 0.000 |
| HLUNG2002465 | 8.231 | 0.000 |
| KIDNE2001847 | 0.000 | 78.156 |
| NT2RP7000466 | 5.924 | 0.000 |
| PUAEN2006328 | 0.000 | 79.193 |
| SMINT2001818 | 12.758 | 0.000 |
| STOMA1000189 | 10.642 | 0.000 |
| STOMA2003444 | 91.236 | 0.000 |
| STOMA2004294 | 100.000 | 0.000 |
| STOMA2004925 | 85.088 | 0.000 |
| STOMA2008546 | 100.000 | 0.000 |
| SYNOV4007671 | 4.989 | 0.000 |
| TESTI4000014 | 0. 000 | 3.835 |
| TESTI4010851 | 3.720 | 0.000 |
| THYMU2035735 | 0.000 | 28.332 |
| TRACH2001549 | 28.775 | 0.000 |
| TRACH2005811 | 0.000 | 22.648 |
| TRACH2025535 | 0.000 | 12.468 |
| TSTOM1000135 | 0.000 | 90.639 |
| TSTOM2000442 | 0.000 | 100.000 |
| TSTOM2000553 | 0.000 | 36.203 |
| TSTOM2002672 | 0.000 | 100.000 |
| UTERU2006115 | 53.335 | 0.000 |
| UTERU3001572 | 12.423 | 0.000 |
| FEBRA2008692 | 0.000 | 52.692 |
| NT2RI2009583 | 7. 613 | 0. 000 |
| STOMA2003158 | 41.655 | 0.000 |
| STOMA2004893 | 49.171 | 0.000 |
| TESTI2015626 | 2.238 | 0.000 |

The result of comparative analysis of cDNA libraries derived from uterine tumor (TUTER) and normal uterus (UTERU) showed the following genes whose expression levels differed between the two.

**Table 19**

| Clone ID | UTERU | TUTER |
|---|---|---|
| BNGH42007788 | 3.672 | 0.000 |
| BRACE1000186 | 2.579 | 0.000 |
| BRACE2030341 | 16.499 | 0.000 |
| BRACE3008772 | 62.692 | 0.000 |
| BRACE3009747 | 2.668 | 0.000 |
| BRACE3010428 | 5.461 | 0.000 |
| BRACE3027478 | 19.089 | 0.000 |
| BRALZ2017359 | 24.816 | 0.000 |
| BRAWH2014645 | 2.522 | 0.000 |
| BRAWH3000314 | 28.447 | 0.000 |
| BRAWH3001326 | 54.394 | 0.000 |
| BRAWH3002574 | 7.885 | 0.000 |
| BRAWH3002821 | 26.183 | 0.000 |
| BRAWH3003727 | 11.993 | 0.000 |
| BRAWH3007592 | 4.277 | 0.000 |
| BRCAN2009432 | 3.141 | 29.345 |
| BRCAN2028355 | 1.762 | 0.000 |
| BRHIP3007586 | 4.116 | 0.000 |
| BRHIP3008344 | 53.896 | 0.000 |
| BRHIP3008565 | 53.896 | 0.000 |
| BRSSN2006892 | 18.468 | 0.000 |
| BRSTN2001067 | 7.384 | 0.000 |
| BRSTN2016470 | 0.522 | 0.000 |
| BRTHA2010608 | 51.819 | 0.000 |
| BRTHA3003074 | 13.429 | 0.000 |
| CTONG1000087 | 2.494 | 0.000 |
| CTONG1000467 | 10.248 | 0.000 |
| CTONG2028124 | 2.997 | 0.000 |
| CTONG3001123 | 9.359 | 0.000 |
| CTONG3008894 | 0.867 | 0.000 |
| CTONG3009028 | 3.839 | 0.000 |
| CTONG3009239 | 1.722 | 0.000 |
| FCBBF3004847 | 39.231 | 0.000 |
| FEBRA2026984 | 20.914 | 0.000 |
| FEBRA2028618 | 6.061 | 0.000 |
| HCHON2000244 | 1.569 | 0.000 |
| HCHON2000418 | 15.860 | 0.000 |
| HCHON2000626 | 5.608 | 0.000 |
| HCHON2001084 | 2.739 | 0.000 |
| HCHON2001217 | 4.966 | 0.000 |
| HCHON2005921 | 24.732 | 0.000 |
| HCHON2006250 | 0.000 | 34.872 |
| HCHON2008444 | 15.860 | 0.000 |
| HLUNG2003003 | 28.594 | 0.000 |
| HSYRA2008376 | 1.865 | 0.000 |
| KIDNE2002252 | 2.600 | 0.000 |
| MESAN2014295 | 24.337 | 0.000 |
| NOVAR2000710 | 4.201 | 0.000 |
| NT2RI2008724 | 2.190 | 0. 000 |
| NT2RI2014247 | 18.191 | 0.000 |
| NT2RI2014733 | 29.743 | 0.000 |
| NT2RI3002892 | 18.890 | 0.000 |
| NT2RI3005724 | 7.069 | 0.000 |
| NT2RI3006284 | 0.000 | 46.111 |
| NT2RI3006340 | 19.479 | 0.000 |
| NT2RI3006673 | 11.902 | 0. 000 |
| NT2RI3007291 | 14.928 | 0. 000 |
| NT2RI3007543 | 1.012 | 0.000 |
| NT2RP7004123 | 2.289 | 0.000 |
| NT2RP7005529 | 8.806 | 0.000 |
| NT2RP7009147 | 1.900 | 0.000 |
| NT2RP7017474 | 22.450 | 0.000 |
| OCBBF2007028 | 8.234 | 0.000 |
| OCBBF2020741 | 33.655 | 0.000 |
| OCBBF2024850 | 26.505 | 0.000 |
| OCBBF2036743 | 13.183 | 0.000 |
| OCBBF3000483 | 9.520 | 0.000 |
| PLACE6001185 | 25.473 | 0.000 |
| PLACE7000514 | 5.673 | 0.000 |
| PUAEN2007044 | 1.710 | 0.000 |
| PUAEN2009655 | 4.359 | 0.000 |
| SKNSH2000482 | 23.251 | 0.000 |
| SPLEN2006122 | 0.000 | 18.769 |
| SPLEN2016554 | 22.228 | 0.000 |
| SPLEN2031547 | 5.633 | 0.000 |
| SPLEN2036932 | 3.497 | 0.000 |
| STOMA1000189 | 1.865 | 0.000 |
| STOMA2004925 | 14.912 | 0.000 |
| SYNOV2017055 | 20.136 | 0.000 |
| SYNOV4001395 | 21.660 | 0.000 |
| SYNOV4002346 | 7.153 | 0.000 |
| SYNOV4008440 | 1.759 | 0.000 |
| TCERX2000613 | 14.497 | 0.000 |
| TESOP2002273 | 4.841 | 0.000 |
| TESTI4000014 | 0.639 | 0.000 |
| TESTI4008797 | 14.824 | 0. 000 |
| TESTI4009286 | 2.594 | 0.000 |
| TESTI4012702 | 2.600 | 0.000 |
| TESTI4013675 | 35.326 | 0.000 |
| TESTI4014159 | 13.979 | 0.000 |
| TESTI4018886 | 64.596 | 0.000 |
| TESTI4029671 | 22.183 | 0.000 |
| TESTI4037156 | 1.320 | 0.000 |
| THYMU2008725 | 15.552 | 0.000 |
| THYMU2031890 | 21.176 | 0.000 |
| THYMU2033070 | 58.853 | 0.000 |
| THYMU2035735 | 11.014 | 0.000 |
| THYMU3001472 | 20.097 | 0.000 |
| TRACH1000205 | 4.694 | 0.000 |
| TRACH2001443 | 11.083 | 0.000 |
| TRACH2001549 | 5.043 | 0.000 |
| TRACH2005811 | 1.258 | 0.000 |
| TRACH2007834 | 0.602 | 0.000 |
| TRACH2008300 | 2.025 | 0.000 |
| TRACH3002192 | 4.463 | 0.000 |
| TRACH3003379 | 29.185 | 0.000 |
| TRACH3004068 | 1.700 | 0.000 |
| TRACH3004721 | 3.467 | 0.000 |
| TRACH3007479 | 3.848 | 0.000 |
| TUTER1000122 | 0.000 | 72.738 |
| TUTER2000425 | 0.000 | 100.000 |
| TUTER2000904 | 3.330 | 62.217 |
| TUTER2000916 | 0.000 | 100.000 |
| TUTER2001387 | 0.000 | 100.000 |
| TUTER2002729 | 0.000 | 100.000 |
| UTERU1000024 | 100.000 | 0.000 |
| UTERU1000031 | 100.000 | 0.000 |
| UTERU1000148 | 100.000 | 0.000 |
| UTERU1000249 | 100.000 | 0.000 |
| UTERU1000337 | 100.000 | 0.000 |
| UTERU1000339 | 100.000 | 0.000 |
| UTERU2000649 | 100.000 | 0.000 |
| UTERU2001409 | 100.000 | 0.000 |
| UTERU2002410 | 0.700 | 13.085 |
| UTERU2002841 | 64.596 | 0.000 |
| UTERU2004688 | 6.630 | 0.000 |
| UTERU2004929 | 13.845 | 0.000 |
| UTERU2005004 | 41.697 | 0.000 |
| UTERU2005621 | 5.377 | 0.000 |
| UTERU2006115 | 9.347 | 0.000 |
| UTERU2006137 | 9.269 | 0.000 |
| UTERU2006568 | 100.000 | 0.000 |
| UTERU2007444 | 64.596 | 0.000 |
| UTERU2007520 | 15.999 | 0.000 |
| UTERU2007724 | 15.672 | 0.000 |
| UTERU2008347 | 100.000 | 0.000 |
| UTERU2014678 | 48.981 | 0.000 |
| UTERU2017762 | 39.037 | 0.000 |
| UTERU2019491 | 100.000 | 0.000 |
| UTERU2019681 | 100.000 | 0.000 |
| UTERU2019706 | 54.394 | 0.000 |
| UTERU2019940 | 51.819 | 0.000 |
| UTERU2020491 | 100.000 | 0.000 |
| UTERU2020718 | 100.000 | 0.000 |
| UTERU2021163 | 53.896 | 0.000 |
| UTERU2021380 | 100.000 | 0.000 |
| UTERU2022020 | 100.000 | 0.000 |
| UTERU2022981 | 100.000 | 0.000 |
| UTERU2023039 | 54.394 | 0.000 |
| UTERU2023175 | 7.103 | 0.000 |
| UTERU2023651 | 29.963 | 0.000 |
| UTERU2023712 | 100.000 | 0.000 |
| UTERU2024002 | 100.000 | 0.000 |
| UTERU2024656 | 51.568 | 0.000 |
| UTERU2025025 | 100.000 | 0.000 |
| UTERU2025645 | 100.000 | 0.000 |
| UTERU2025891 | 100.000 | 0.000 |
| UTERU2026025 | 100.000 | 0.000 |
| UTERU2026090 | 100.000 | 0.000 |
| UTERU2026203 | 54.394 | 0.000 |
| UTERU2027591 | 100.000 | 0.000 |
| UTERU2029953 | 100.000 | 0.000 |
| UTERU2030213 | 58.763 | 0.000 |
| UTERU2030280 | 51.819 | 0.000 |
| UTERU2031084 | 25.928 | 0.000 |
| UTERU2031268 | 100.000 | 0.000 |
| UTERU2031521 | 100.000 | 0.000 |
| UTERU2031703 | 100.000 | 0.000 |
| UTERU2031851 | 100.000 | 0.000 |
| UTERU2033375 | 1.385 | 0.000 |
| UTERU2033382 | 100.000 | 0.000 |
| UTERU2035114 | 51.568 | 0.000 |
| UTERU2035323 | 100.000 | 0.000 |
| UTERU2035328 | 100.000 | 0.000 |
| UTERU2035331 | 100.000 | 0.000 |
| UTERU2035452 | 100.000 | 0.000 |
| UTERU2035469 | 100.000 | 0.000 |
| UTERU2035503 | 100.000 | 0.000 |
| UTERU2035745 | 100.000 | 0.000 |
| UTERU2036089 | 100.000 | 0.000 |
| UTERU2037361 | 100.000 | 0.000 |
| UTERU2037577 | 100.000 | 0.000 |
| UTERU2038251 | 100.000 | 0.000 |
| UTERU3000226 | 62.692 | 0.000 |
| UTERU3000645 | 34.742 | 0.000 |
| UTERU3000665 | 100.000 | 0.000 |
| UTERU3000828 | 100.000 | 0.000 |
| UTERU3000899 | 33.654 | 0.000 |
| UTERU3001059 | 100.000 | 0.000 |
| UTERU3001240 | 100.000 | 0.000 |
| UTERU3001542 | 58.853 | 0.000 |
| UTERU3001571 | 51.819 | 0.000 |
| UTERU3001572 | 19.596 | 0.000 |
| UTERU3001585 | 100.000 | 0.000 |
| UTERU3001652 | 100.000 | 0.000 |
| UTERU3001766 | 54.206 | 0.000 |
| UTERU3001988 | 100.000 | 0.000 |
| UTERU3002209 | 100.000 | 0.000 |
| UTERU3002218 | 51.568 | 0.000 |
| UTERU3002383 | 100.000 | 0.000 |
| UTERU3002667 | 100.000 | 0.000 |
| UTERU3002731 | 100.000 | 0.000 |
| UTERU3002768 | 100.000 | 0.000 |
| UTERU3002786 | 51.568 | 0.000 |
| UTERU3002993 | 100.000 | 0.000 |
| UTERU3003116 | 100.000 | 0.000 |
| UTERU3003135 | 39.146 | 0.000 |
| UTERU3003178 | 100.000 | 0.000 |
| UTERU3003465 | 100.000 | 0.000 |
| UTERU3003523 | 100.000 | 0.000 |
| UTERU3003776 | 100.000 | 0.000 |
| UTERU3004523 | 100.000 | 0.000 |
| UTERU3004616 | 100.000 | 0.000 |
| UTERU3004709 | 51.819 | 0.000 |
| UTERU3004992 | 100.000 | 0.000 |
| UTERU3005049 | 58.853 | 0.000 |
| UTERU3005205 | 100.000 | 0.000 |
| UTERU3005230 | 58.249 | 0.000 |
| UTERU3005460 | 62.692 | 0.000 |
| UTERU3005585 | 100.000 | 0.000 |
| UTERU3005907 | 15.610 | 0.000 |
| UTERU3005970 | 62.692 | 0.000 |
| UTERU3006008 | 100.000 | 0.000 |
| UTERU3006308 | 62.692 | 0.000 |
| UTERU3007134 | 100.000 | 0.000 |
| UTERU3007419 | 62.692 | 0.000 |
| UTERU3007640 | 54.394 | 0.000 |
| UTERU3007913 | 87.049 | 0.000 |
| UTERU3008660 | 100.000 | 0.000 |
| UTERU3008671 | 100.000 | 0.000 |
| UTERU3009259 | 53.896 | 0.000 |
| UTERU3009490 | 100.000 | 0.000 |
| UTERU3009517 | 54.206 | 0.000 |
| UTERU3009690 | 100.000 | 0.000 |
| UTERU3009871 | 21.605 | 0.000 |
| UTERU3009979 | 100.000 | 0.000 |
| UTERU3011063 | 100.000 | 0.000 |
| UTERU3015086 | 100.000 | 0.000 |
| UTERU3015500 | 100.000 | 0.000 |
| UTERU3016789 | 100.000 | 0.000 |
| UTERU3018081 | 40.205 | 0.000 |
| UTERU3018154 | 100.000 | 0.000 |
| UTERU3018616 | 25.167 | 0.000 |
| UTERU3018711 | 86.466 | 0.000 |
| ADRGL2000042 | 3.029 | 0.000 |
| BRHIP3000017 | 15.777 | 0.000 |
| CTONG2003348 | 39.037 | 0.000 |
| CTONG2019822 | 7.755 | 0.000 |
| CTONG2020378 | 19.250 | 0.000 |
| CTONG2020411 | 32.844 | 0.000 |
| CTONG2024031 | 6.165 | 0.000 |
| FEBRA2028516 | 1.364 | 0.000 |
| HCASM2008536 | 15.228 | 0.000 |
| HCHON2000743 | 7.282 | 0.000 |
| IMR322001879 | 11.495 | 0.000 |
| MESAN2005303 | 9.449 | 0.000 |
| NT2RI2009583 | 0.267 | 0.000 |
| OCBBF2008144 | 3.440 | 0.000 |
| PERIC2007068 | 5.521 | 0.000 |
| SPLEN2039379 | 10.362 | 0.000 |
| TESTI2015626 | 0.784 | 7.329 |
| TESTI4013894 | 33.408 | 0.000 |
| TUTER2000057 | 0.000 | 92.461 |
| UTERU2004299 | 100.000 | 0.000 |
| UTERU2008040 | 14.037 | 0.000 |
| UTERU2011220 | 4.669 | 0.000 |
| UTERU2019534 | 100.000 | 0.000 |
| UTERU2021820 | 29.732 | 0.000 |
| UTERU2028734 | 26.183 | 0.000 |
| UTERU2032279 | 100.000 | 0.000 |
| UTERU2033577 | 100.000 | 0.000 |
| UTERU2035978 | 100.000 | 0.000 |
| UTERU3000402 | 100.000 | 0.000 |
| UTERU3000738 | 41.697 | 0.000 |
| UTERU3001053 | 100.000 | 0.000 |
| UTERU3014791 | 100.000 | 0.000 |
| UTERU3015412 | 100.000 | 0.000 |
| UTERU3017176 | 100.000 | 0.000 |

The result of comparative analysis of cDNA libraries derived from tongue cancer (CTONG) and normal tongue (NTONG) showed the following genes whose expression levels differed between the two.

**Table 20**

| Clone ID | NTONG | CTONG |
|---|---|---|
| BNGH42007788 | 0.000 | 5.734 |
| BRACE1000186 | 15.935 | 0.000 |
| BRACE2006319 | 0.000 | 6.530 |
| BRACE3010428 | 0.000 | 6.396 |
| BRACE3012364 | 0.000 | 7.922 |
| BRAMY2020058 | 0.000 | 52.028 |
| BRAMY3002803 | 0.000 | 26.873 |
| BRAWH2001671 | 0.000 | 7.082 |
| BRAWH2014645 | 0.000 | 1.969 |
| BRAWH3002574 | 0.000 | 12.314 |
| BRCAN2009432 | 0.000 | 4.905 |
| BRCAN2015371 | 0.000 | 20.417 |
| BRCAN2020710 | 0.000 | 20.242 |
| BRHIP2004814 | 0.000 | 64.609 |
| BRHIP3018797 | 0.000 | 2.495 |
| BRTHA2003461 | 0.000 | 4.088 |
| BRTHA3003490 | 11.964 | 0.000 |
| CTONG1000087 | 0.000 | 3.895 |
| CTONG1000088 | 0.000 | 5.442 |
| CTONG1000288 | 11.209 | 84.986 |
| CTONG1000302 | 0.000 | 100.000 |
| CTONG1000341 | 0.000 | 51.706 |
| CTONG1000467 | 0.000 | 16.004 |
| CTONG1000488 | 0.000 | 100.000 |
| CTONG1000508 | 0.000 | 100.000 |
| CTONG1000540 | 0.000 | 100.000 |
| CTONG2000042 | 0.000 | 65.252 |
| CTONG2001877 | 0.000 | 100.000 |
| CTONG2004062 | 0.000 | 100.000 |
| CTONG2006798 | 0.000 | 17.972 |
| CTONG2008233 | 0.000 | 8.953 |
| CTONG2009423 | 0.000 | 64.609 |
| CTONG2009531 | 0.000 | 100.000 |
| CTONG2010803 | 0.000 | 20.971 |
| CTONG2013178 | 0.000 | 35.252 |
| CTONG2017500 | 0.000 | 4.934 |
| CTONG2019248 | 0.000 | 28.179 |
| CTONG2019652 | 0.000 | 100.000 |
| CTONG2019704 | 0.000 | 62.446 |
| CTONG2019788 | 0.000 | 11.390 |
| CTONG2019833 | 0.000 | 100.000 |
| CTONG2020127 | 0.000 | 100.000 |
| CTONG2020522 | 0.000 | 42.683 |
| CTONG2020638 | 0.000 | 23.060 |
| CTONG2020806 | 0.000 | 100.000 |
| CTONG2021132 | 0.000 | 100.000 |
| CTONG2022153 | 0.000 | 100.000 |
| CTONG2022601 | 0.000 | 100.000 |
| CTONG2023021 | 0.000 | 62.680 |
| CTONG2023512 | 0.000 | 100.000 |
| CTONG2024206 | 0.000 | 100.000 |
| CTONG2024749 | 0.000 | 100.000 |
| CTONG2025496 | 0.000 | 100.000 |
| CTONG2025516 | 0.000 | 100.000 |
| CTONG2025900 | 0.000 | 100.000 |
| CTONG2026920 | 0.000 | 100.000 |
| CTONG2027327 | 0.000 | 52.760 |
| CTONG2028124 | 3.704 | 0.936 |
| CTONG2028687 | 0.000 | 100.000 |
| CTONG3000084 | 0.000 | 51.585 |
| CTONG3000657 | 0.000 | 7.226 |
| CTONG3000686 | 0.000 | 100.000 |
| CTONG3000707 | 0.000 | 100.000 |
| CTONG3000896 | 0.000 | 100.000 |
| CTONG3001123 | 0.000 | 14.616 |
| CTONG3001370 | 0.000 | 65.252 |
| CTONG3001420 | 0.000 | 51.138 |
| CTONG3001560 | 0.000 | 100.000 |
| CTONG3002020 | 0.000 | 100.000 |
| CTONG3002127 | 0.000 | 62.446 |
| CTONG3002412 | 0.000 | 19.932 |
| CTONG3002674 | 0.000 | 41.611 |
| CTONG3003179 | 0.000 | 100.000 |
| CTONG3003483 | 0.000 | 100.000 |
| CTONG3003652 | 0.000 | 100.000 |
| CTONG3003654 | 0.000 | 100.000 |
| CTONG3003737 | 0.000 | 100.000 |
| CTONG3003905 | 0.000 | 36.474 |
| CTONG3003972 | 0.000 | 51.706 |
| CTONG3004072 | 0.000 | 36.356 |
| CTONG3004712 | 0.000 | 100.000 |
| CTONG3005325 | 0.000 | 100.000 |
| CTONG3005648 | 0.000 | 100.000 |
| CTONG3005713 | 0.000 | 100.000 |
| CTONG3005813 | 0.000 | 72.408 |
| CTONG3006067 | 0.000 | 74.021 |
| CTONG3006186 | 0.000 | 100.000 |
| CTONG3006650 | 0.000 | 100.000 |
| CTONG3007444 | 0.000 | 100.000 |
| CTONG3007528 | 0.000 | 100.000 |
| CTONG3007586 | 0.000 | 100.000 |
| CTONG3007870 | 0.000 | 100.000 |
| CTONG3008252 | 0.000 | 100.000 |
| CTONG3008258 | 0.000 | 100.000 |
| CTONG3008496 | 0.000 | 100.000 |
| CTONG3008566 | 0.000 | 100.000 |
| CTONG3008639 | 0.000 | 100.000 |
| CTONG3008831 | 0.000 | 100.000 |
| CTONG3008894 | 0.000 | 2.708 |
| CTONG3008951 | 0.000 | 100.000 |
| CTONG3009028 | 0.000 | 5.995 |
| CTONG3009227 | 0.000 | 100.000 |
| CTONG3009239 | 0.000 | 5.378 |
| CTONG3009328 | 0.000 | 44.674 |
| CTONG3009385 | 0.000 | 100.000 |
| FEBRA2007544 | 0.000 | 4.560 |
| FEBRA2007801 | 0.000 | 4.598 |
| FEBRA2021966 | 31.791 | 0.000 |
| FEBRA2025427 | 0.000 | 9.234 |
| HCHON2000028 | 0.000 | 2.955 |
| HCHON2001217 | 0.000 | 3.877 |
| HHDPC1000118 | 0.000 | 3.904 |
| HSYRA2008376 | 11.524 | 0.000 |
| KIDNE2001847 | 0.000 | 6.778 |
| KIDNE2002252 | 0.000 | 4.060 |
| MESAN2006563 | 0.000 | 2.572 |
| NT2RI2008724 | 0.000 | 3.421 |
| NT2RI2018883 | 0.000 | 50.616 |
| NT2RI3000622 | 63.099 | 0.000 |
| NT2RI3006284 | 0.000 | 3.854 |
| NT2RI3006673 | 0.000 | 18.588 |
| NT2RI3007543 | 6.253 | 7.901 |
| NT2RI3007757 | 0.000 | 23.555 |
| NT2RP7004123 | 0.000 | 3.574 |
| NT2RP7009147 | 0.000 | 2.967 |
| NT2RP7014005 | 0.000 | 10.836 |
| NTONG2000413 | 79.538 | 0.000 |
| NTONG2003852 | 49.507 | 0.000 |
| NTONG2005277 | 100.000 | 0.000 |
| NTONG2005969 | 23.675 | 0.000 |
| NTONG2006354 | 100.000 | 0.000 |
| NTONG2007249 | 100.000 | 0.000 |
| NTONG2007517 | 100.000 | 0.000 |
| NTONG2008088 | 75.309 | 0.000 |
| NTONG2008672 | 100.000 | 0.000 |
| OCBBF2001794 | 34.843 | 0.000 |
| OCBBF2006151 | 0.000 | 10.738 |
| PEBLM2004666 | 0.000 | 6.920 |
| PEBLM2005183 | 0.000 | 7.010 |
| SPLEN2002467 | 0.000 | 6.068 |
| SPLEN2029912 | 0.000 | 6.085 |
| SPLEN2031547 | 0.000 | 2.932 |
| SYNOV4007671 | 0.000 | 1.365 |
| SYNOV4008440 | 0.000 | 2.748 |
| TBAES2002197 | 0.000 | 28.875 |
| TESOP2002273 | 0.000 | 7.560 |
| TESTI2009474 | 0. 000 | 3.763 |
| TESTI4000014 | 2. 632 | 0. 665 |
| TESTI4000209 | 0. 000 | 4.934 |
| TESTI4008018 | 0. 000 | 74. 021 |
| TESTI4009286 | 0.000 | 1.350 |
| TESTI4010851 | 0.000 | 1. 018 |
| TESTI4012702 | 0. 000 | 4. 060 |
| TESTI4013675 | 0.000 | 18.389 |
| THYMU2031847 | 0.000 | 69.076 |
| THYMU2033308 | 0.000 | 26.010 |
| TLIVE2002690 | 44.414 | 0.000 |
| TRACH2005811 | 0.000 | 3.928 |
| TRACH2007059 | 0.000 | 62.446 |
| TRACH2025535 | 0.000 | 2.163 |
| TRACH3001427 | 0.000 | 1.729 |
| TSTOM2000553 | 0.000 | 12.559 |
| UTERU2005621 | 0.000 | 8.397 |
| UTERU2017762 | 0.000 | 60.963 |
| UTERU2023175 | 0.000 | 2.773 |
| UTERU3001572 | 0.000 | 6.800 |
| BLADE2006830 | 24.778 | 0.000 |
| BRHIP3000017 | 0.000 | 8.213 |
| CTONG1000113 | 0.000 | 20.967 |
| CTONG2003348 | 0.000 | 60.963 |
| CTONG2004000 | 0.000 | 100.000 |
| CTONG2008721 | 0.000 | 62.680 |
| CTONG2015596 | 0.000 | 100.000 |
| CTONG2015633 | 0.000 | 100.000 |
| CTONG2016942 | 0.000 | 21.398 |
| CTONG2019822 | 0.000 | 12.111 |
| CTONG2020374 | 0.000 | 57.889 |
| CTONG2020378 | 0.000 | 30.063 |
| CTONG2020411 | 0.000 | 12.823 |
| CTONG2020974 | 0.000 | 74.021 |
| CTONG2024031 | 0.000 | 19.255 |
| CTONG2028758 | 0.000 | 29.955 |
| CTONG3001501 | 0.000 | 100.000 |
| CTONG3002552 | 0.000 | 100.000 |
| CTONG3003598 | 0.000 | 100.000 |
| CTONG3004550 | 0.000 | 100.000 |
| CTONG3004726 | 0.000 | 31.543 |
| CTONG3009287 | 0.000 | 100.000 |
| FEBRA2008692 | 0.000 | 13.709 |
| FEBRA2028516 | 0.000 | 4.261 |
| HCHON2000508 | 2.752 | 9.042 |
| NT2RI2009583 | 4.947 | 0.833 |
| NTONG2008093 | 100.000 | 0.000 |
| PERIC2007068 | 0.000 | 2.874 |
| TESOP2007384 | 44.382 | 37.388 |
| TLIVE2002046 | 0.000 | 3.380 |
| TRACH2000862 | 0.000 | 53.910 |

The result of comparative analysis of cDNA libraries derived from fetal brain (FCBBF, FEBRA, or OCBBF) and adult brain (BRACE, BRALZ, BRAMY, BRAWH, BRCAN, BRCOC, BRHIP, BRSSN, BRSTN, or BRTHA) showed the following genes whose expression levels differed between the two.

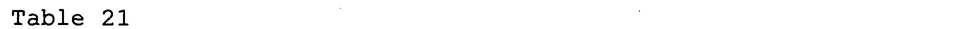

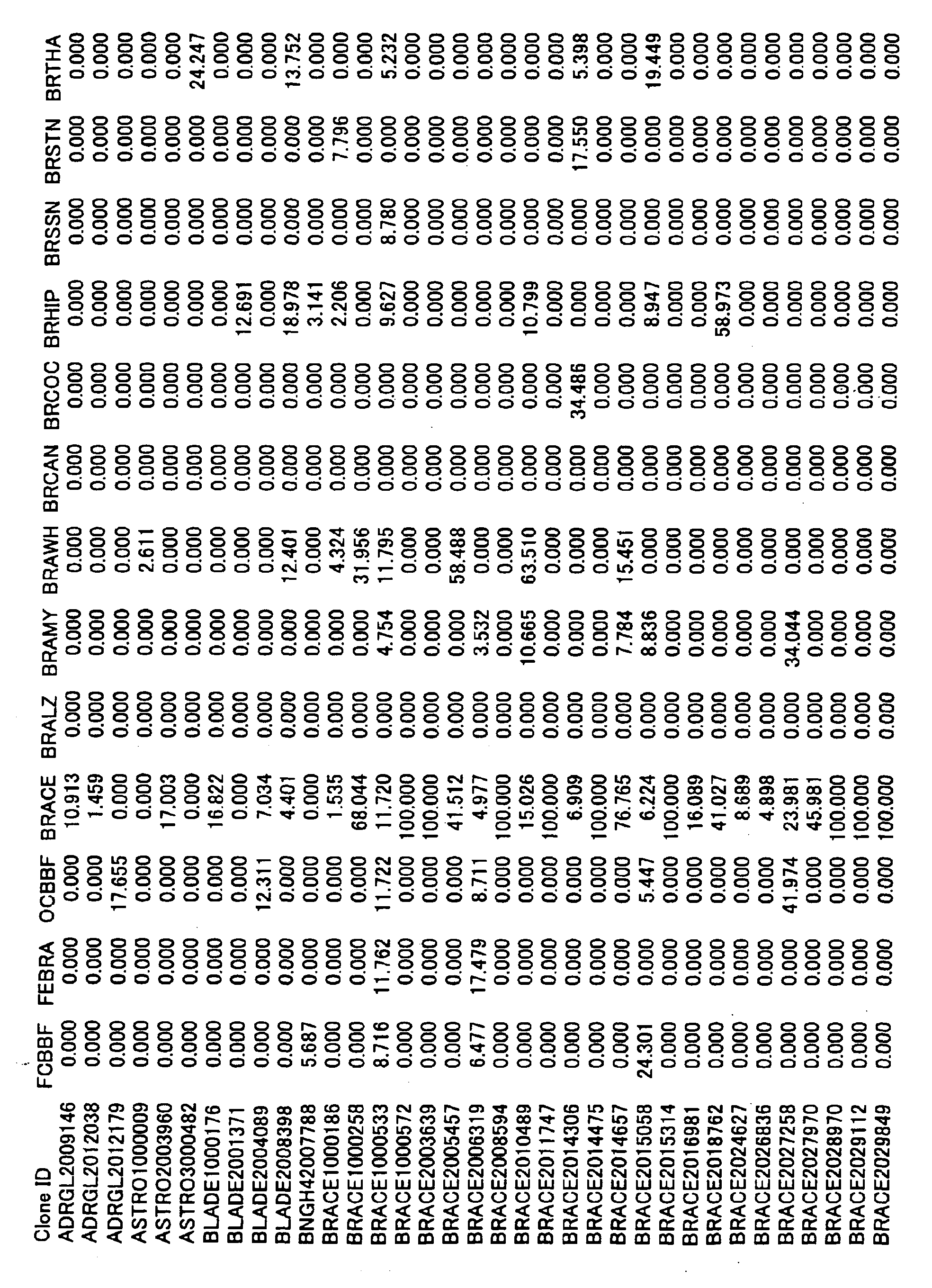

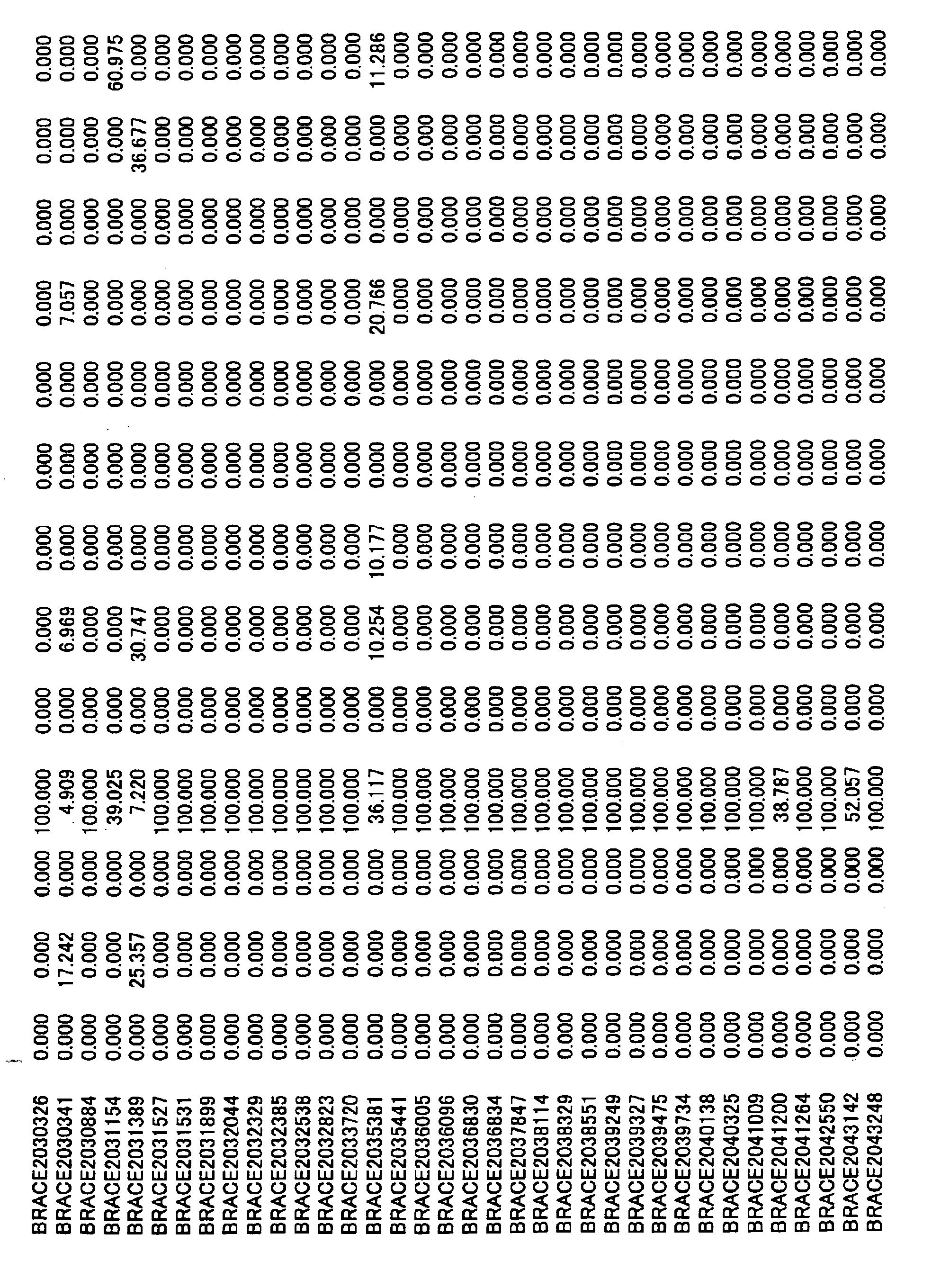

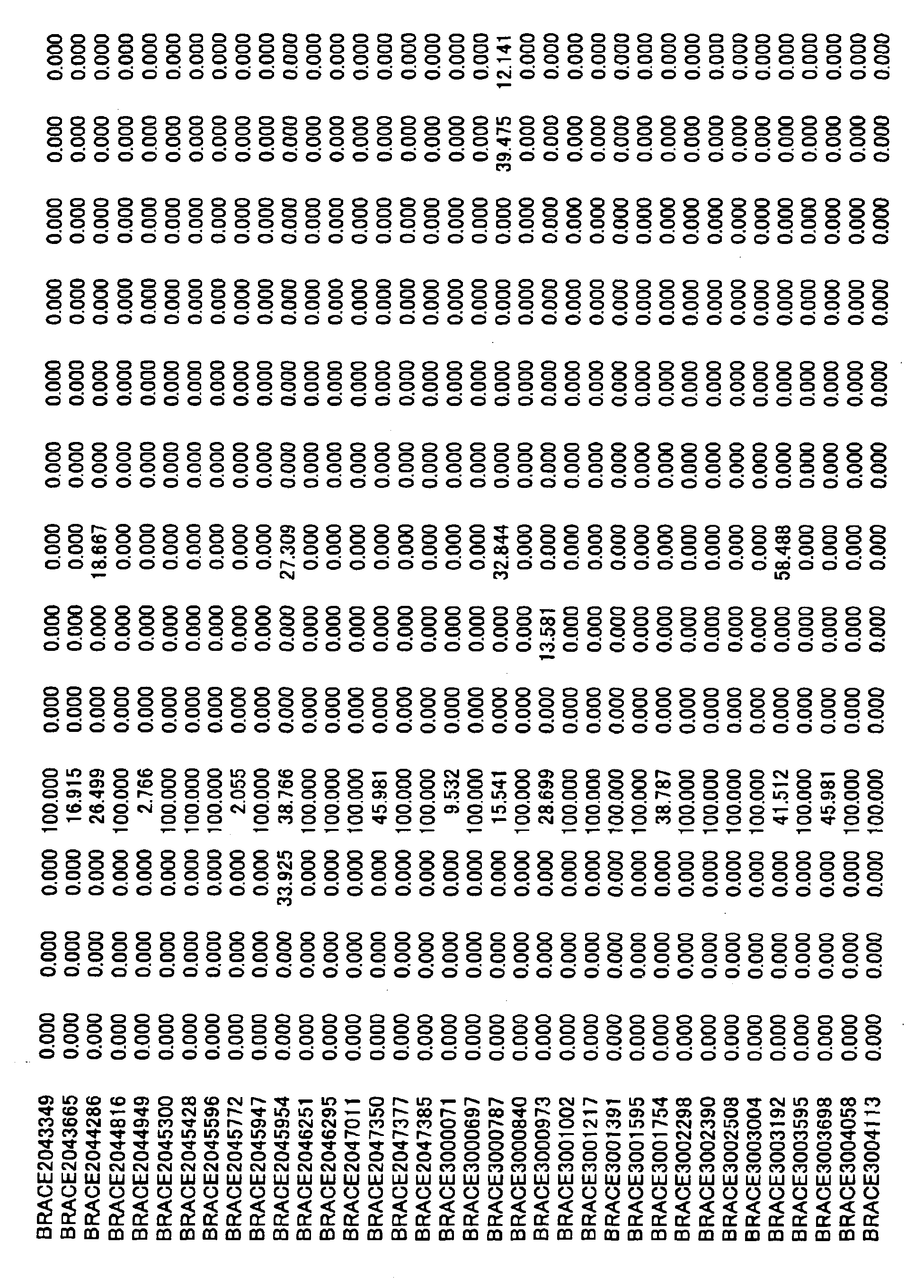

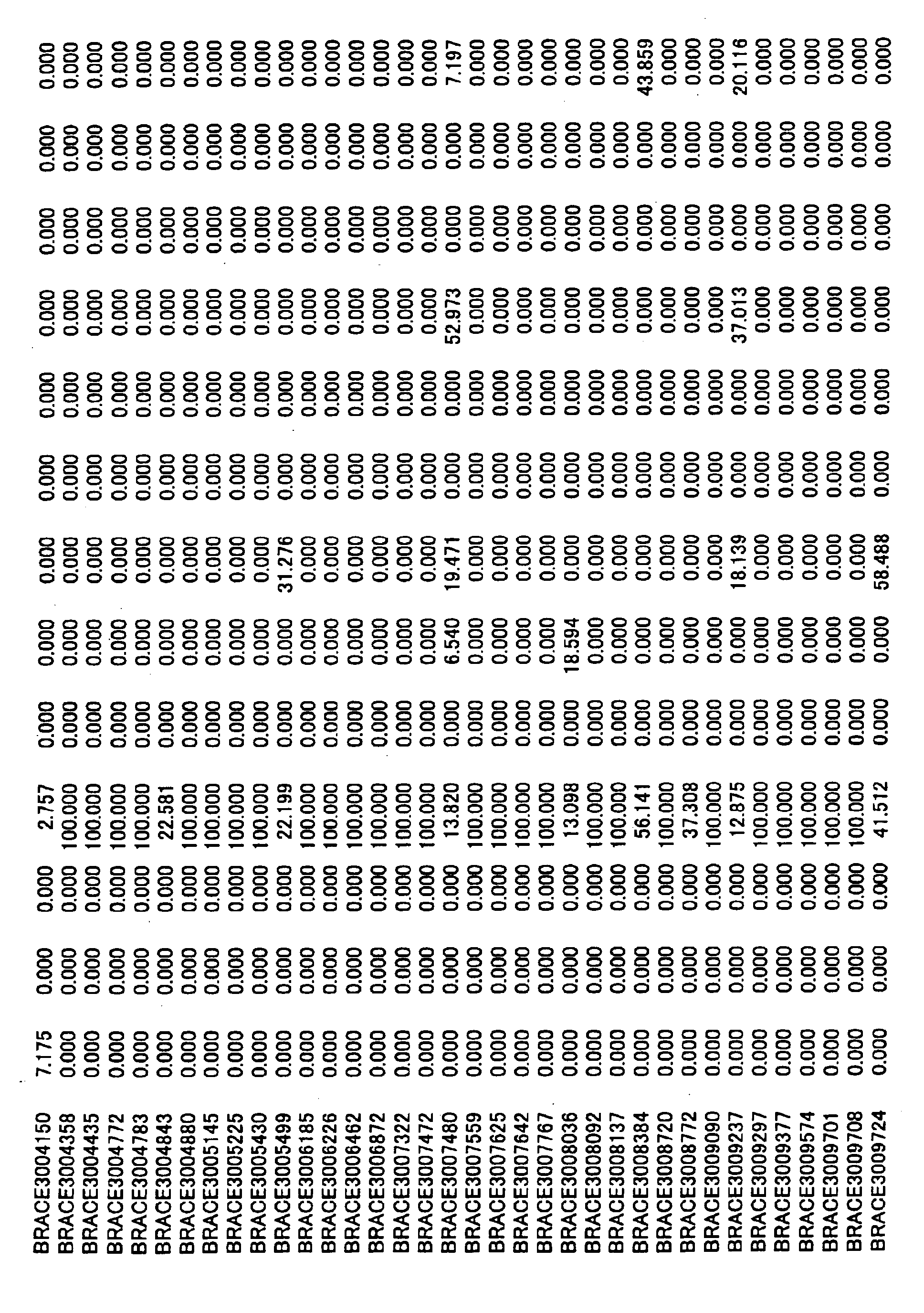

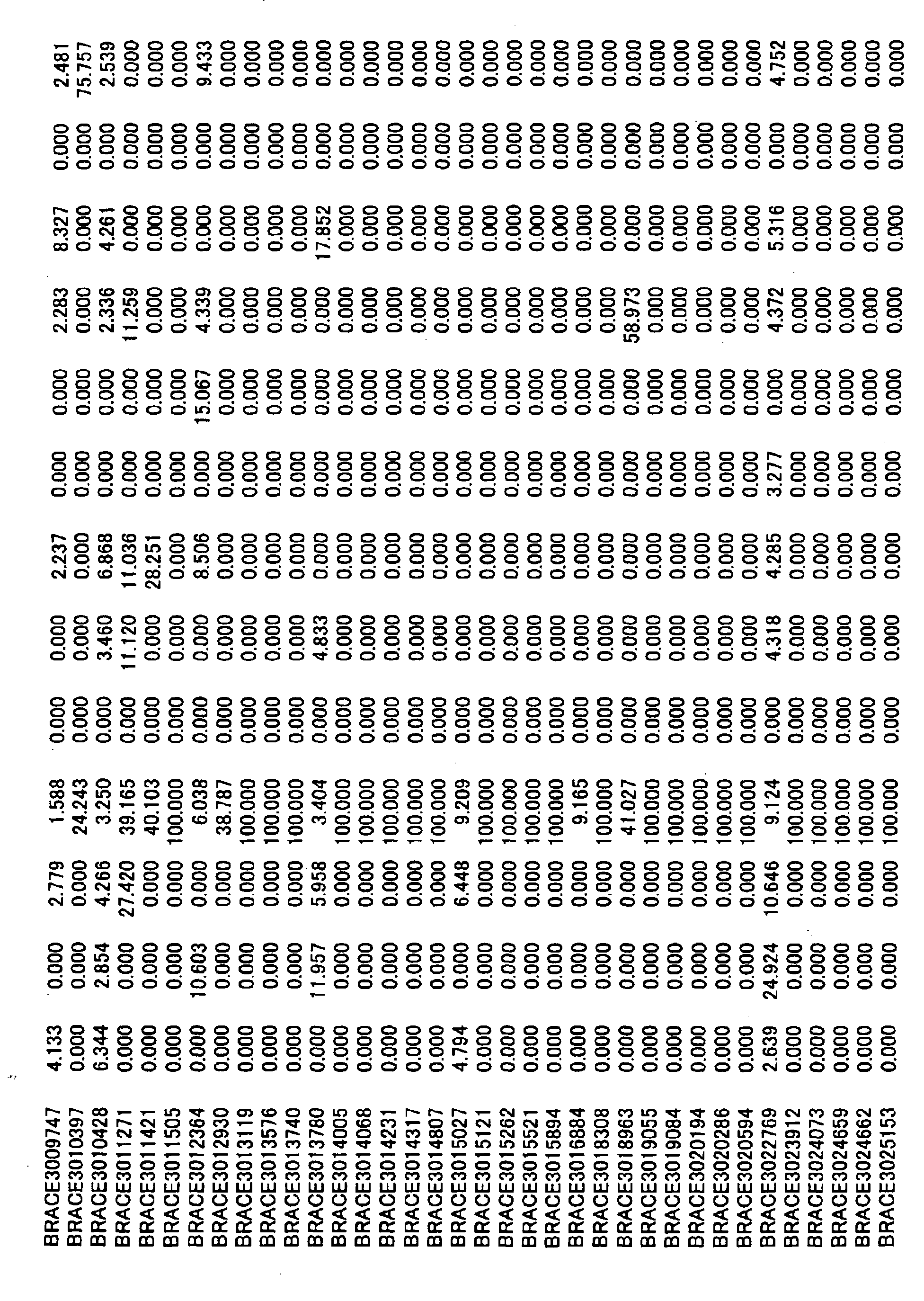

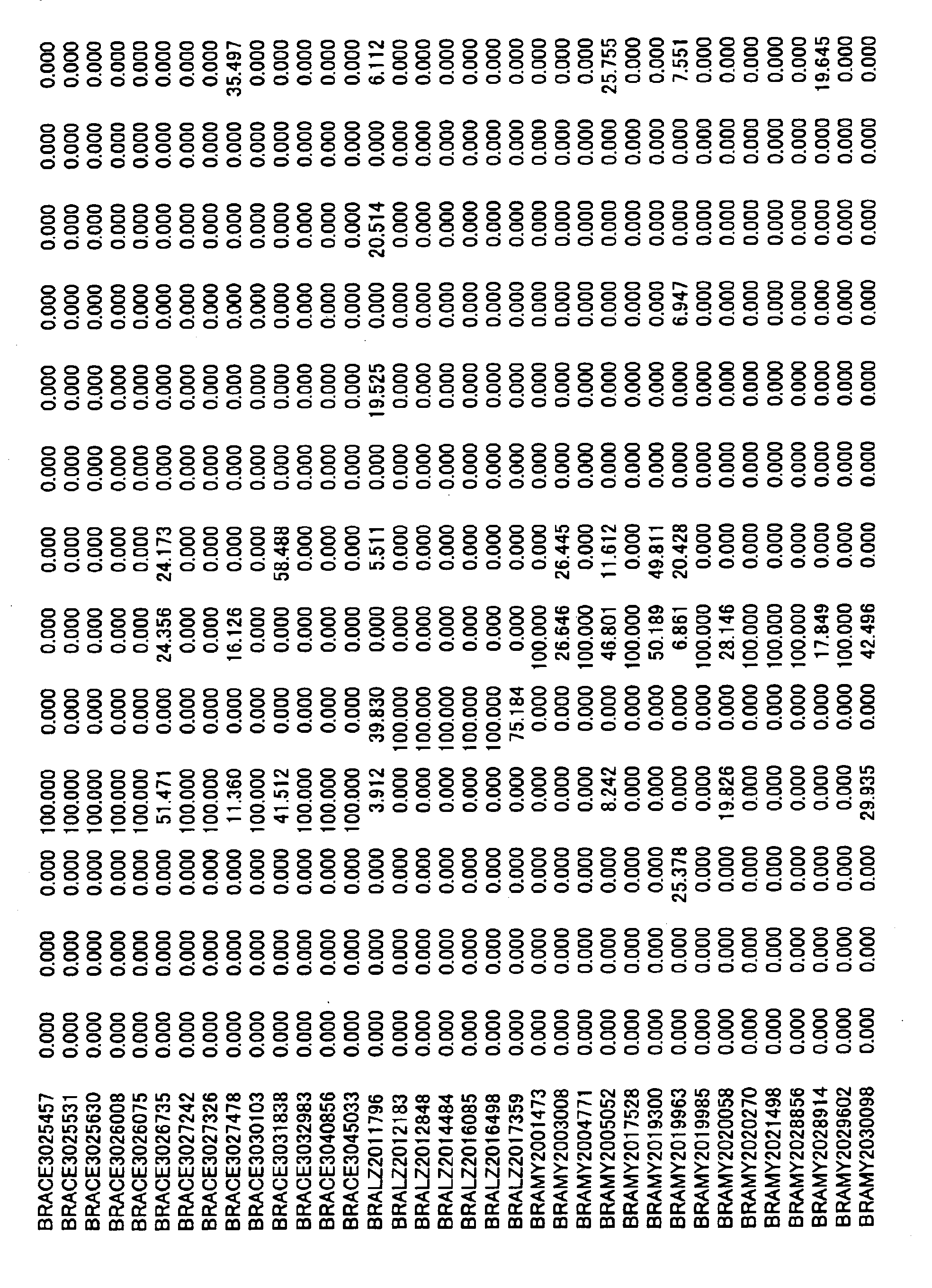

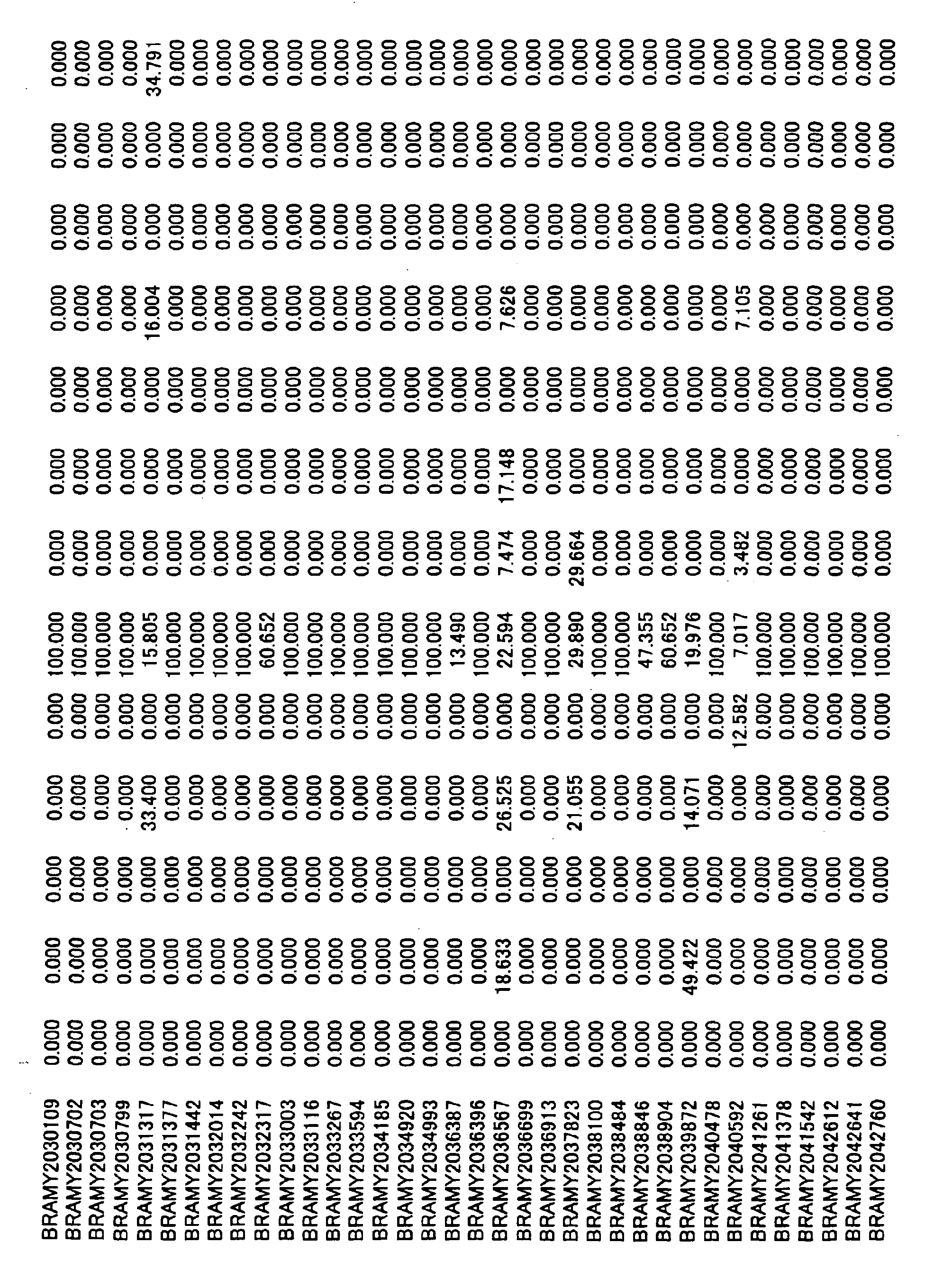

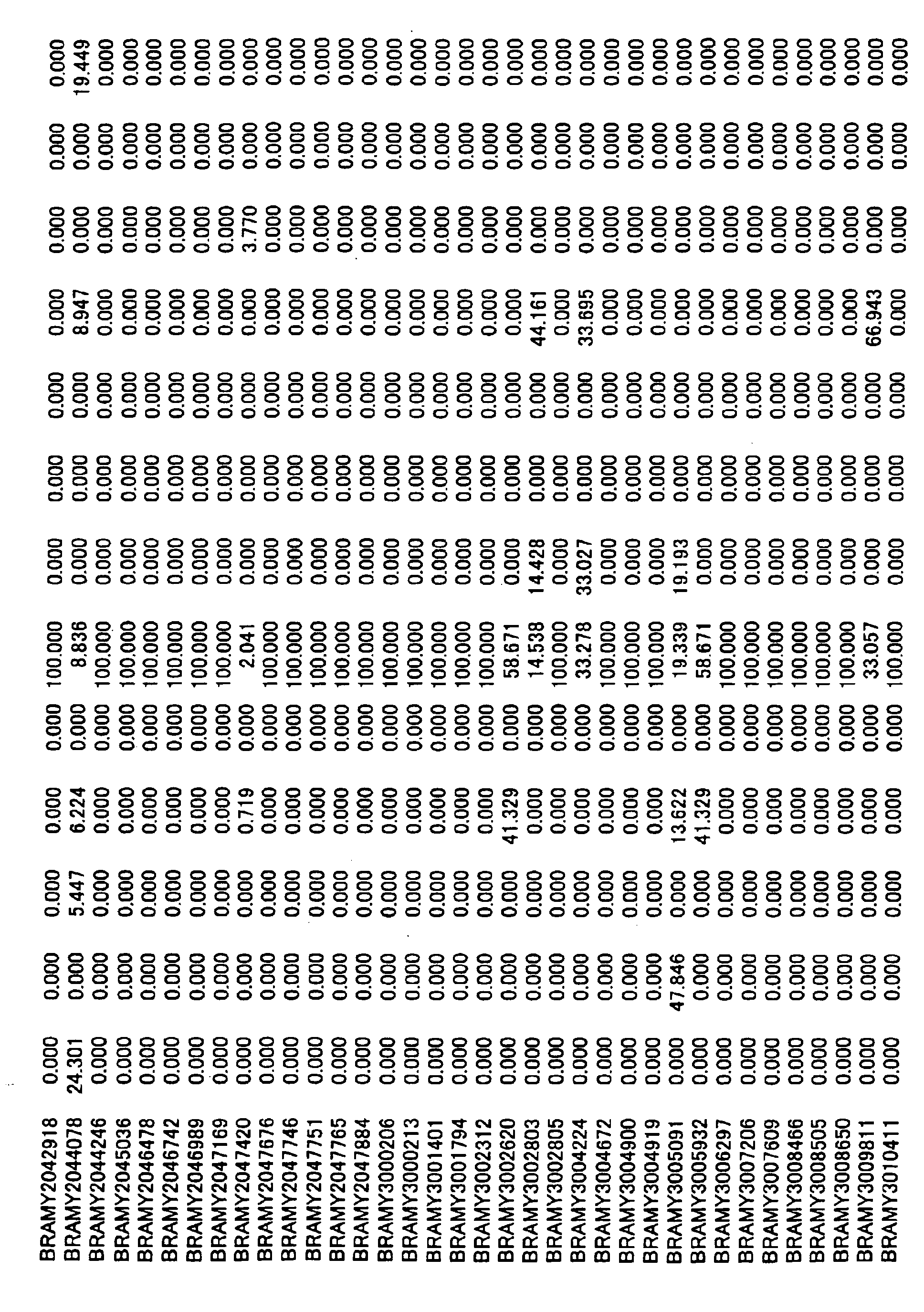

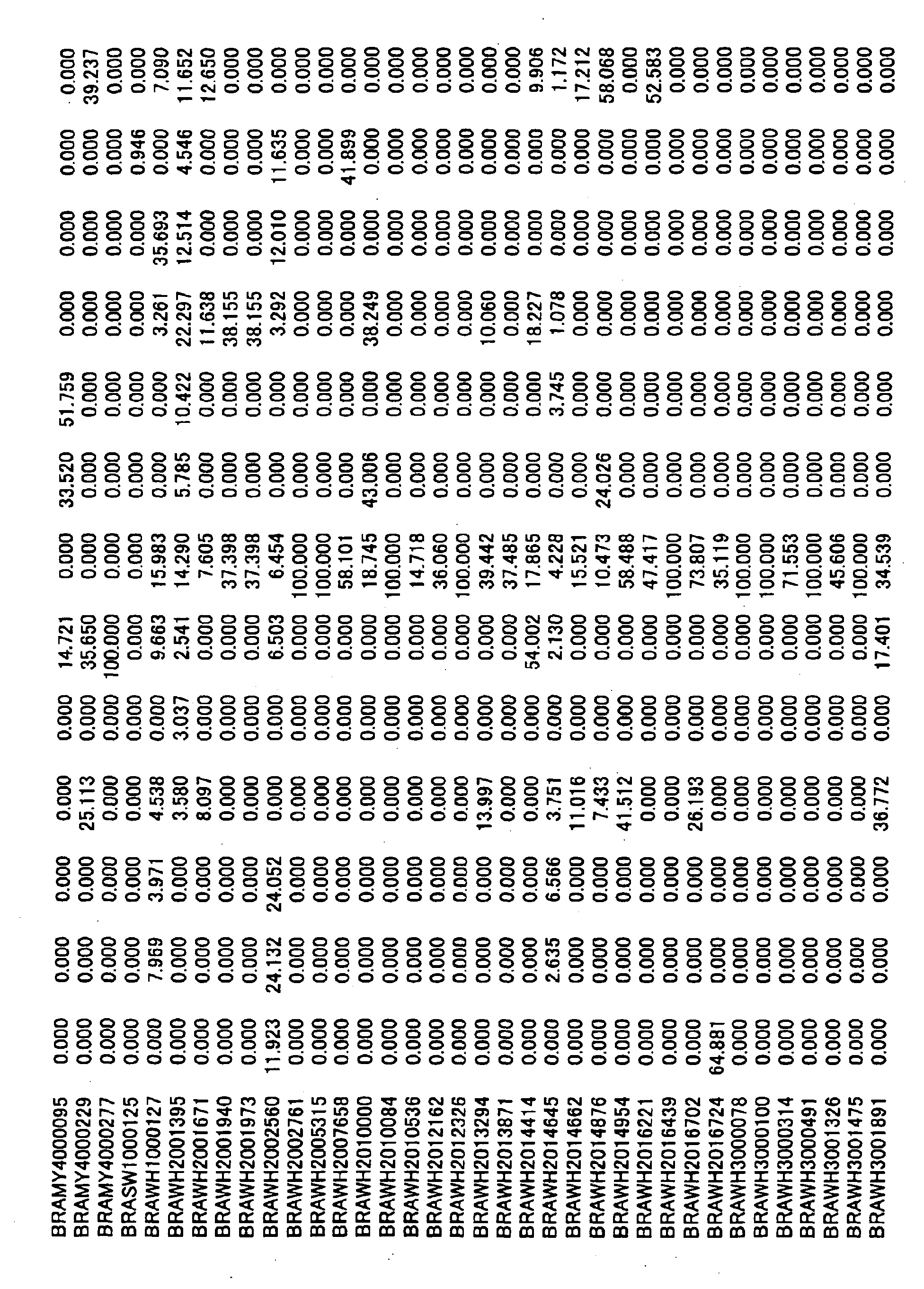

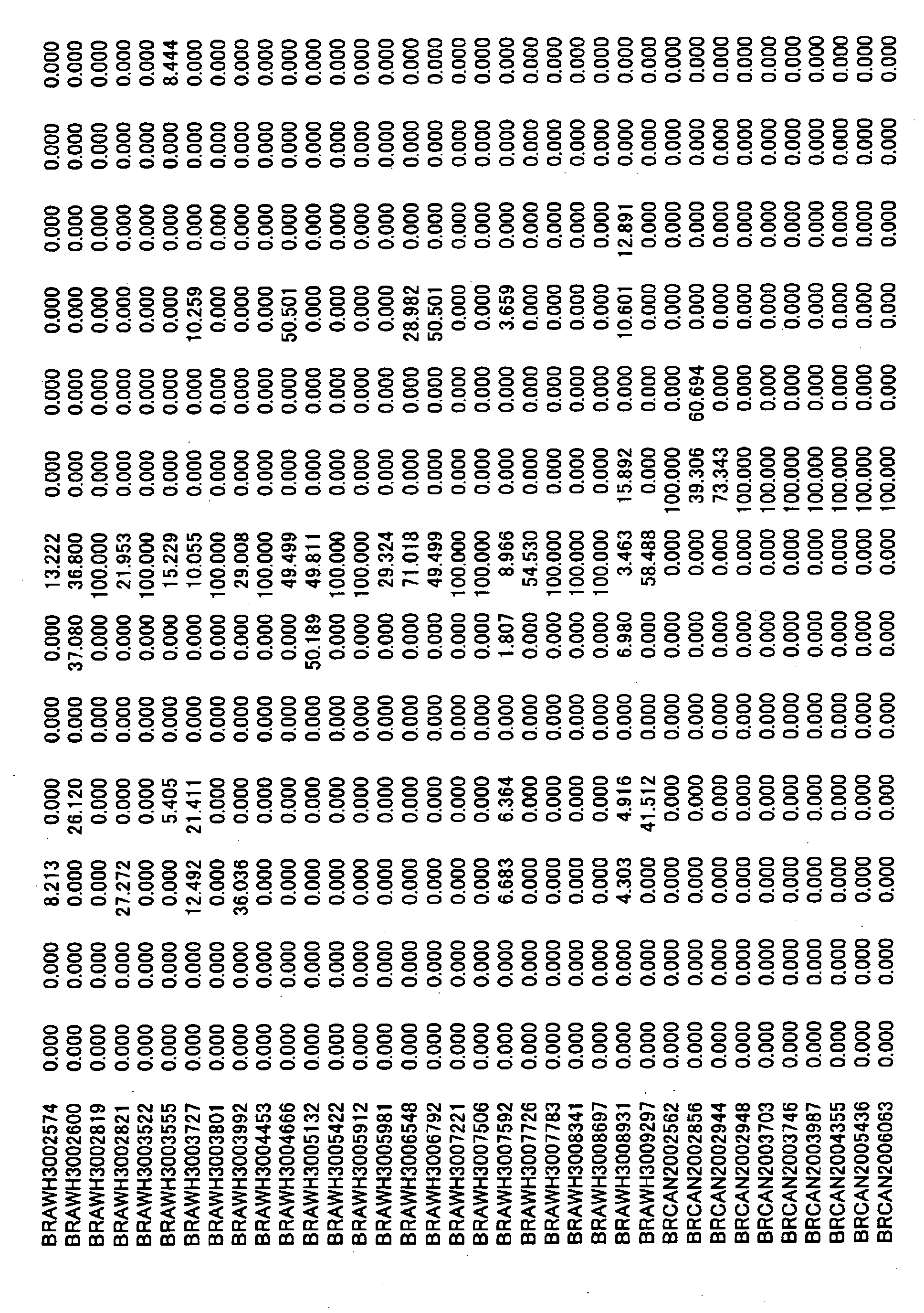

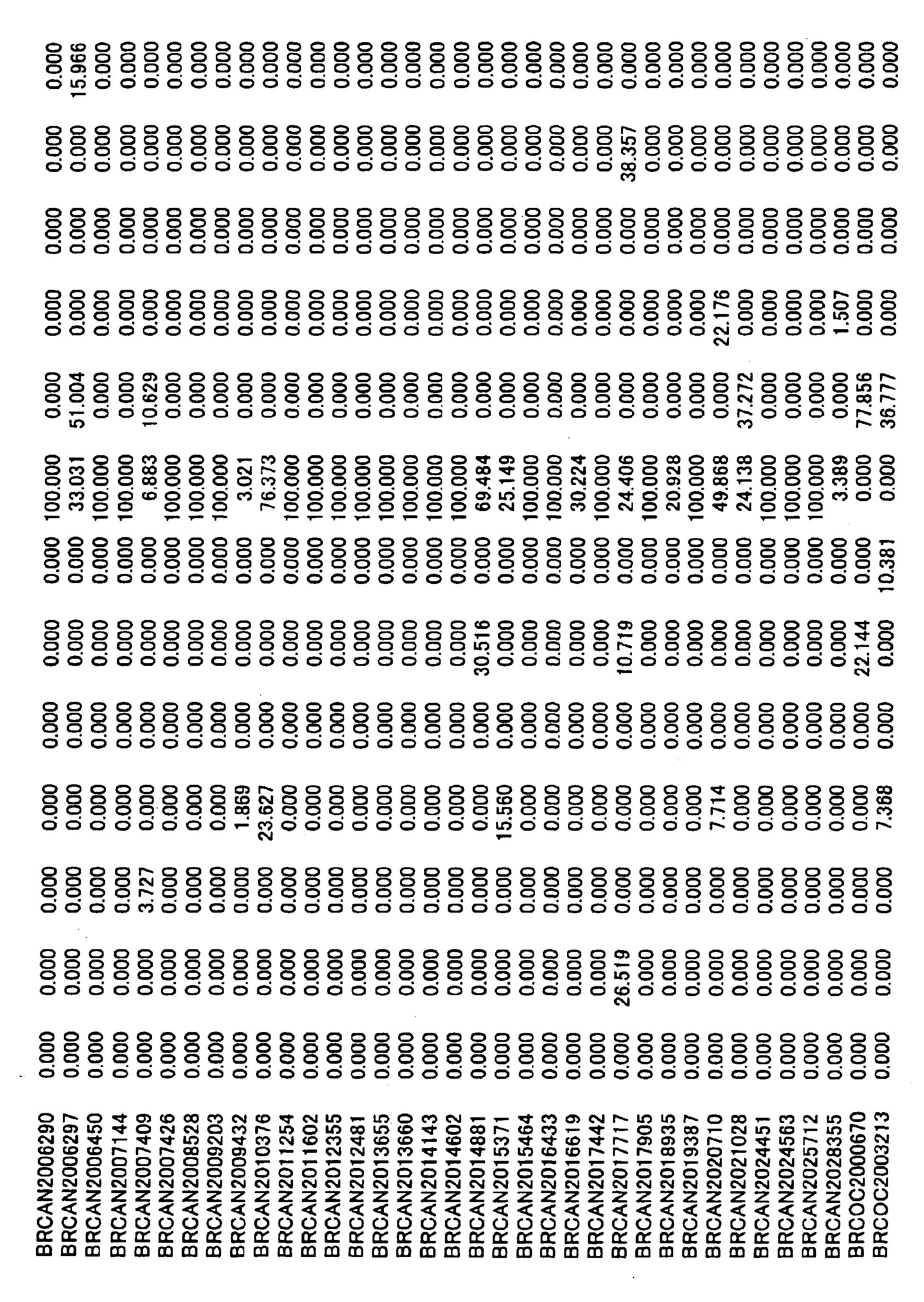

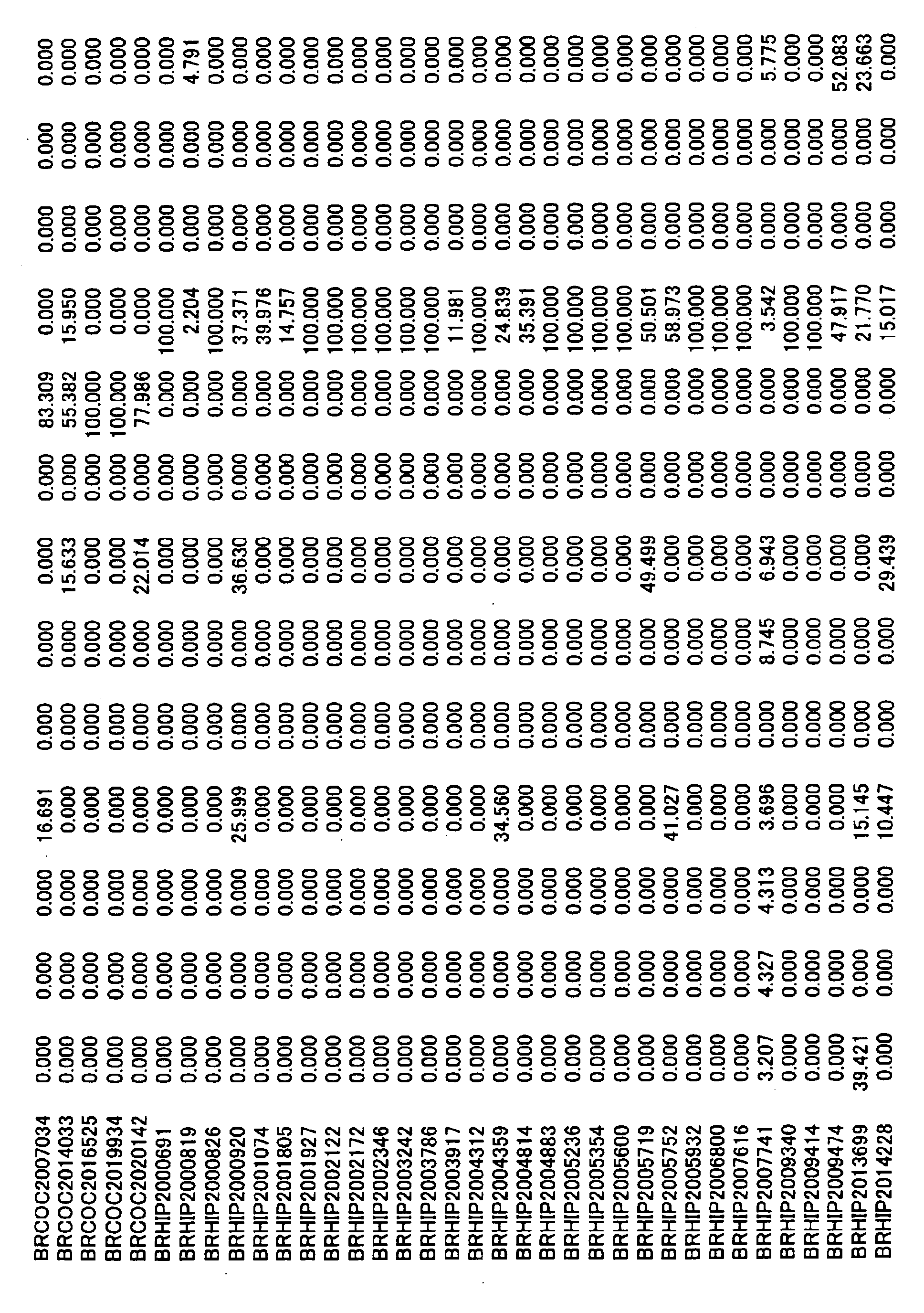

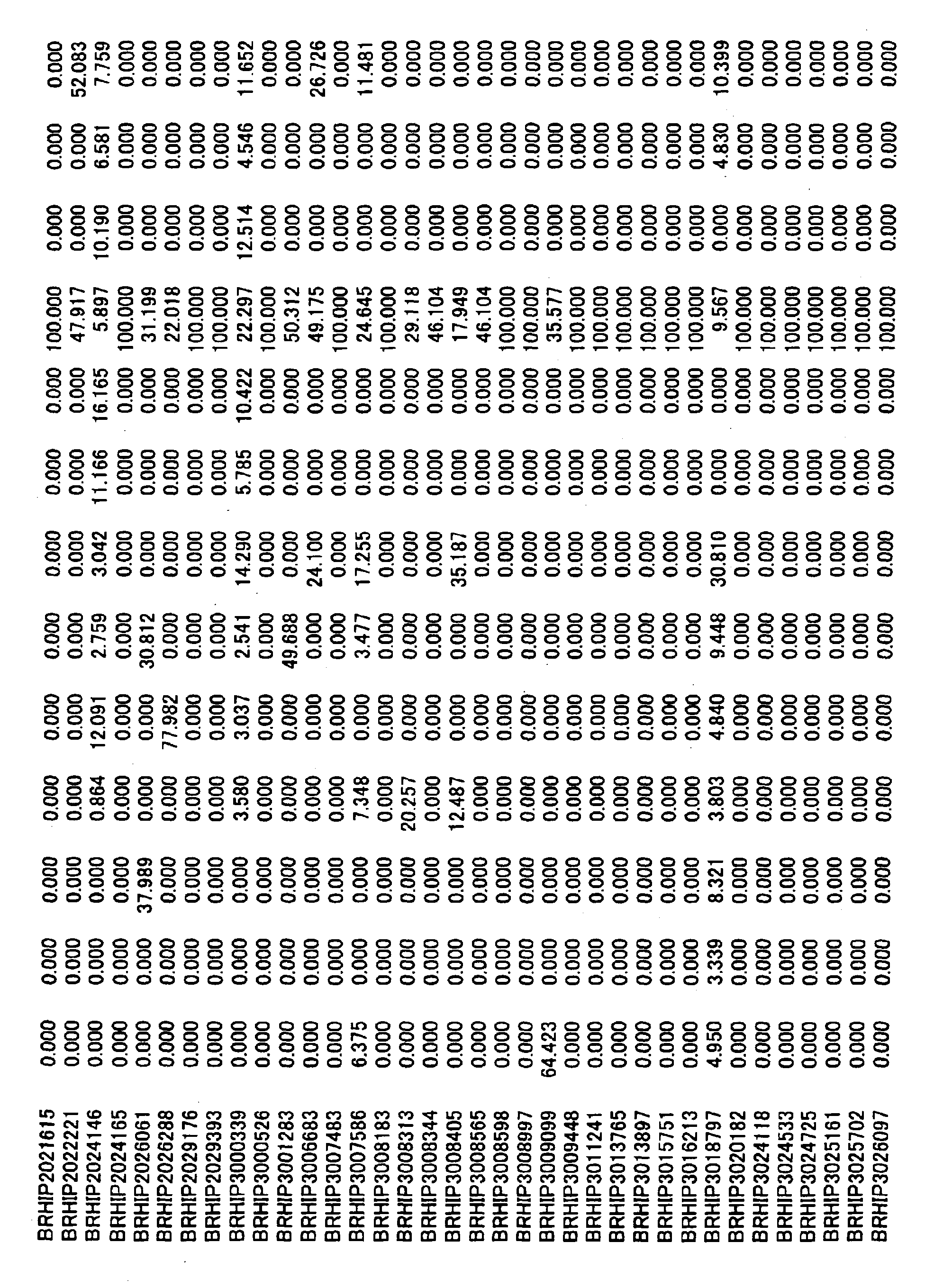

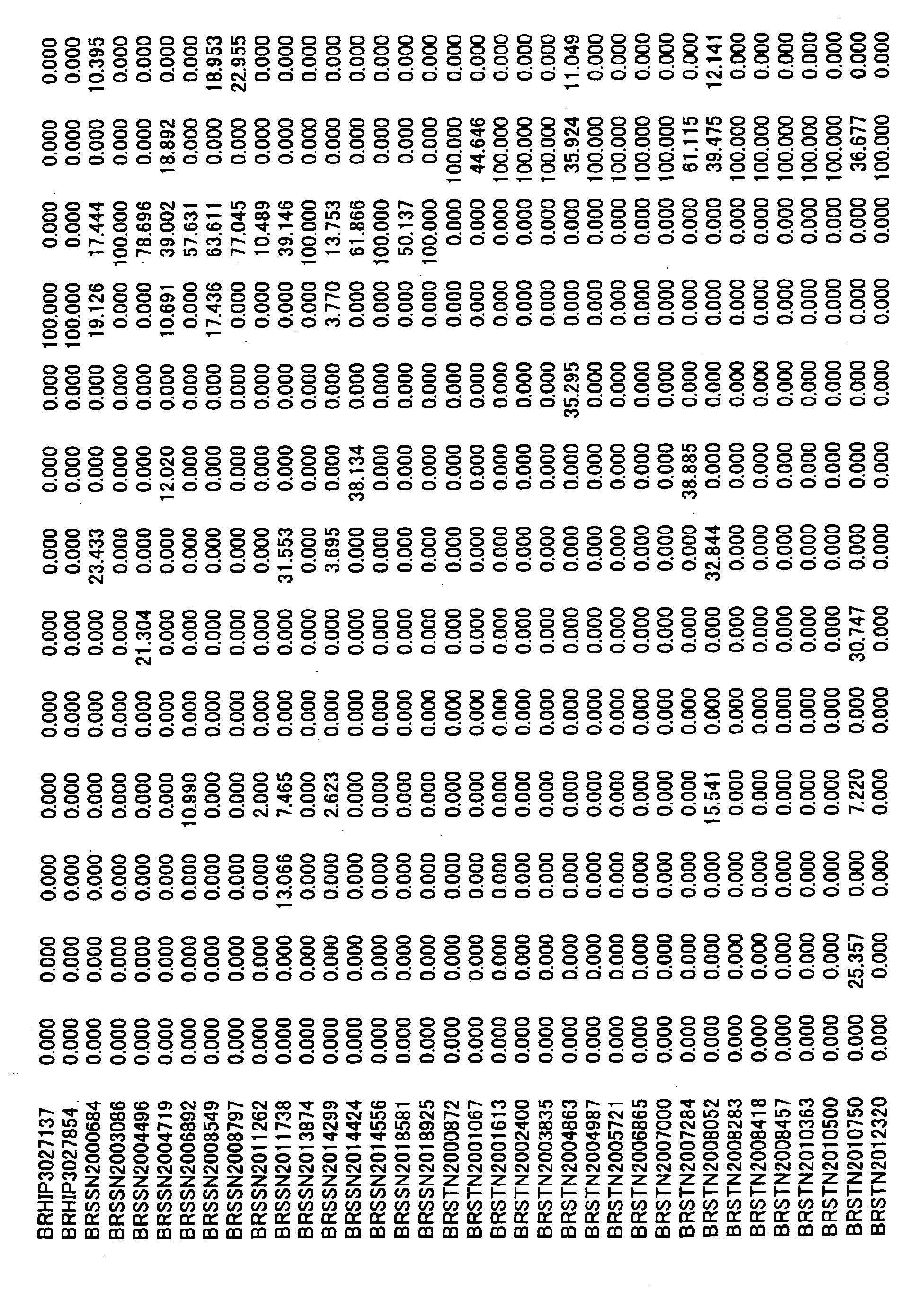

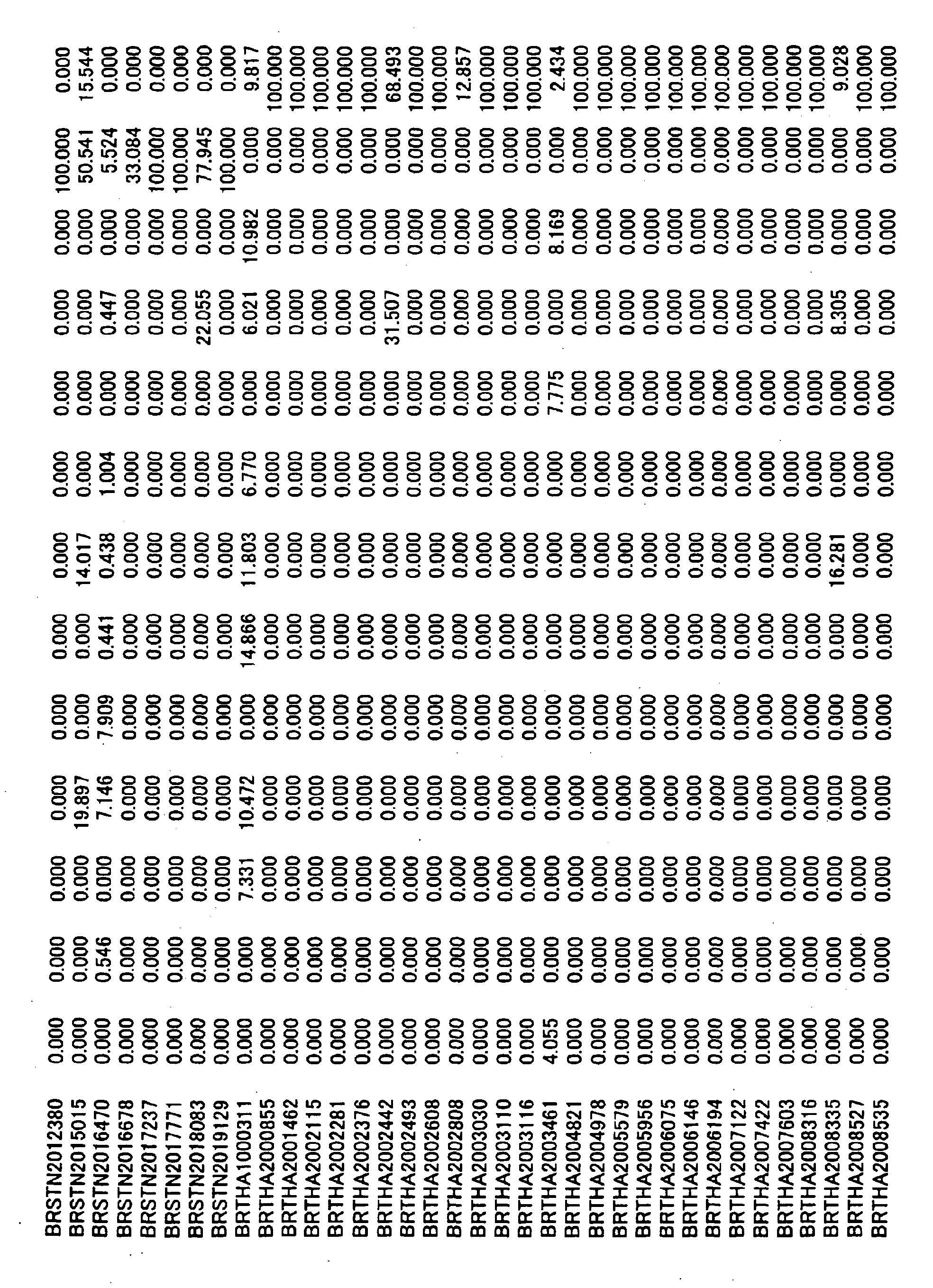

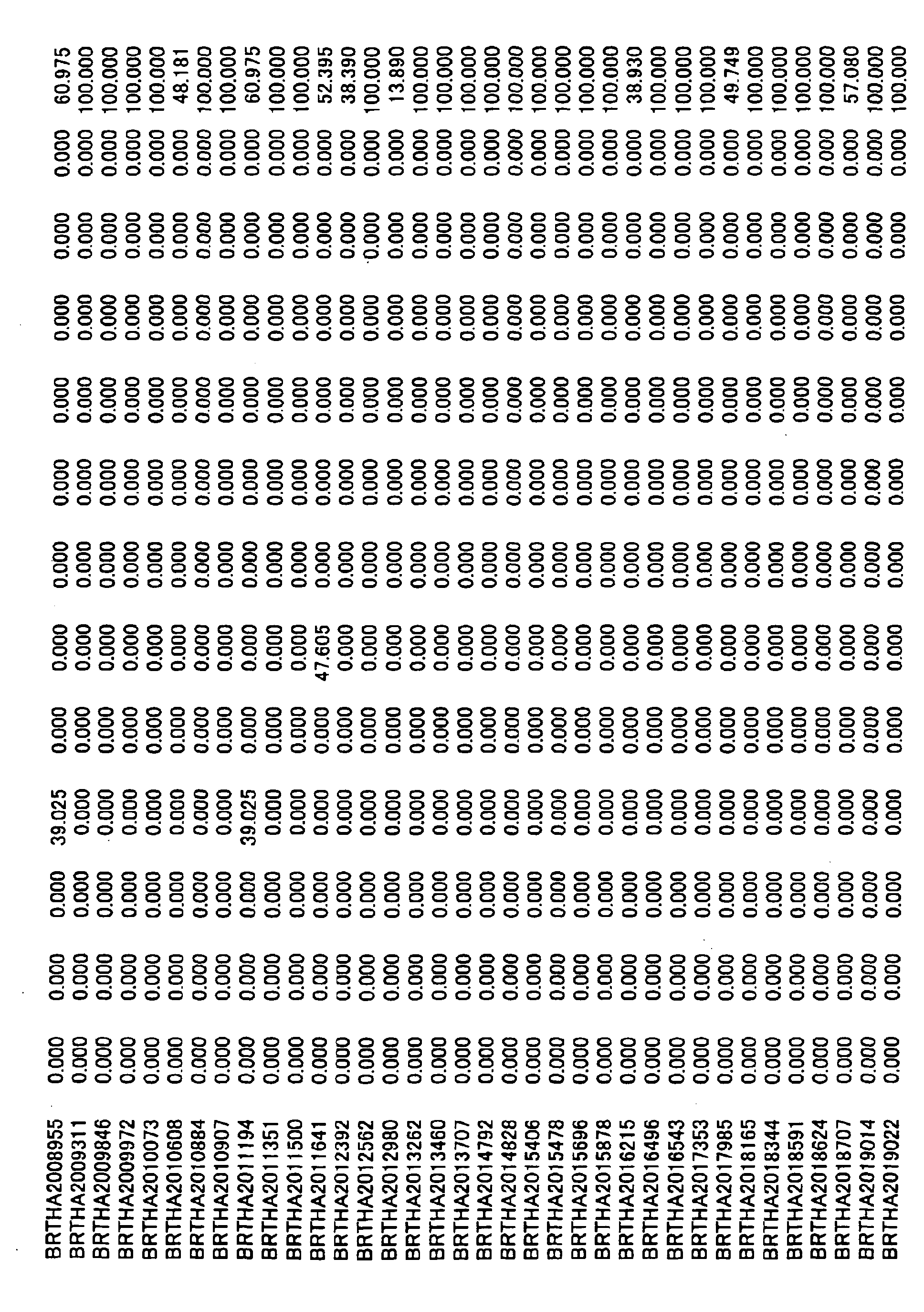

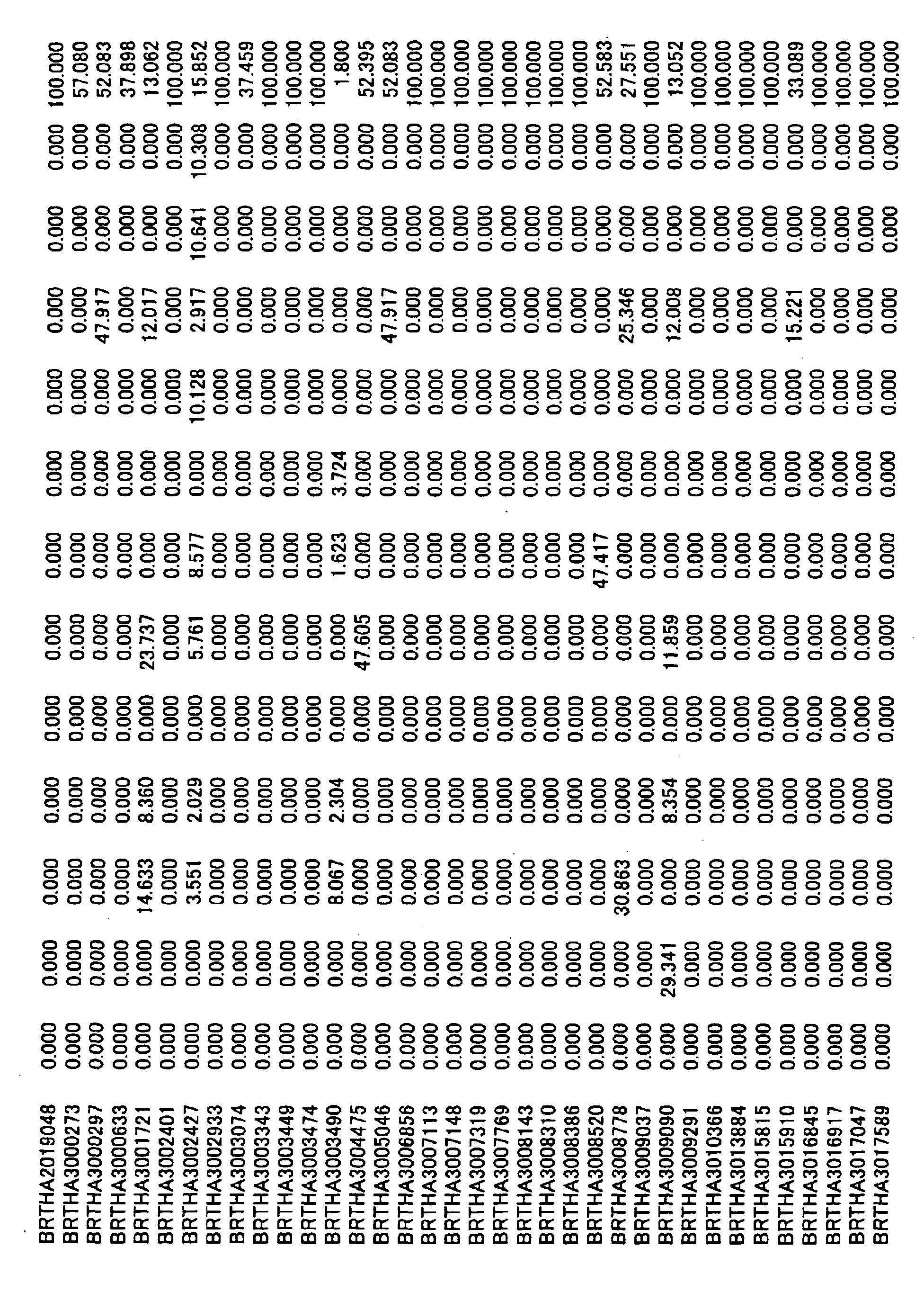

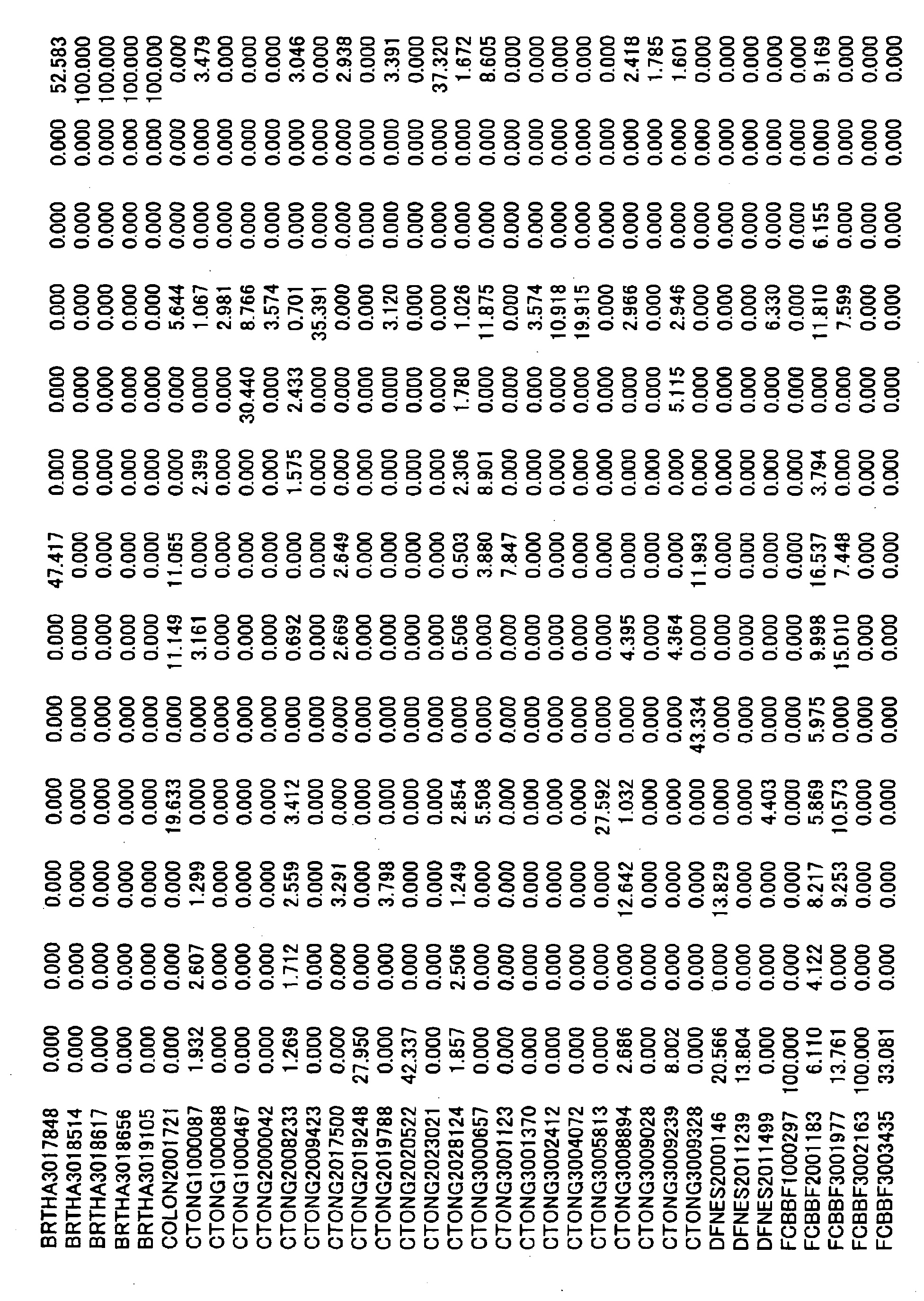

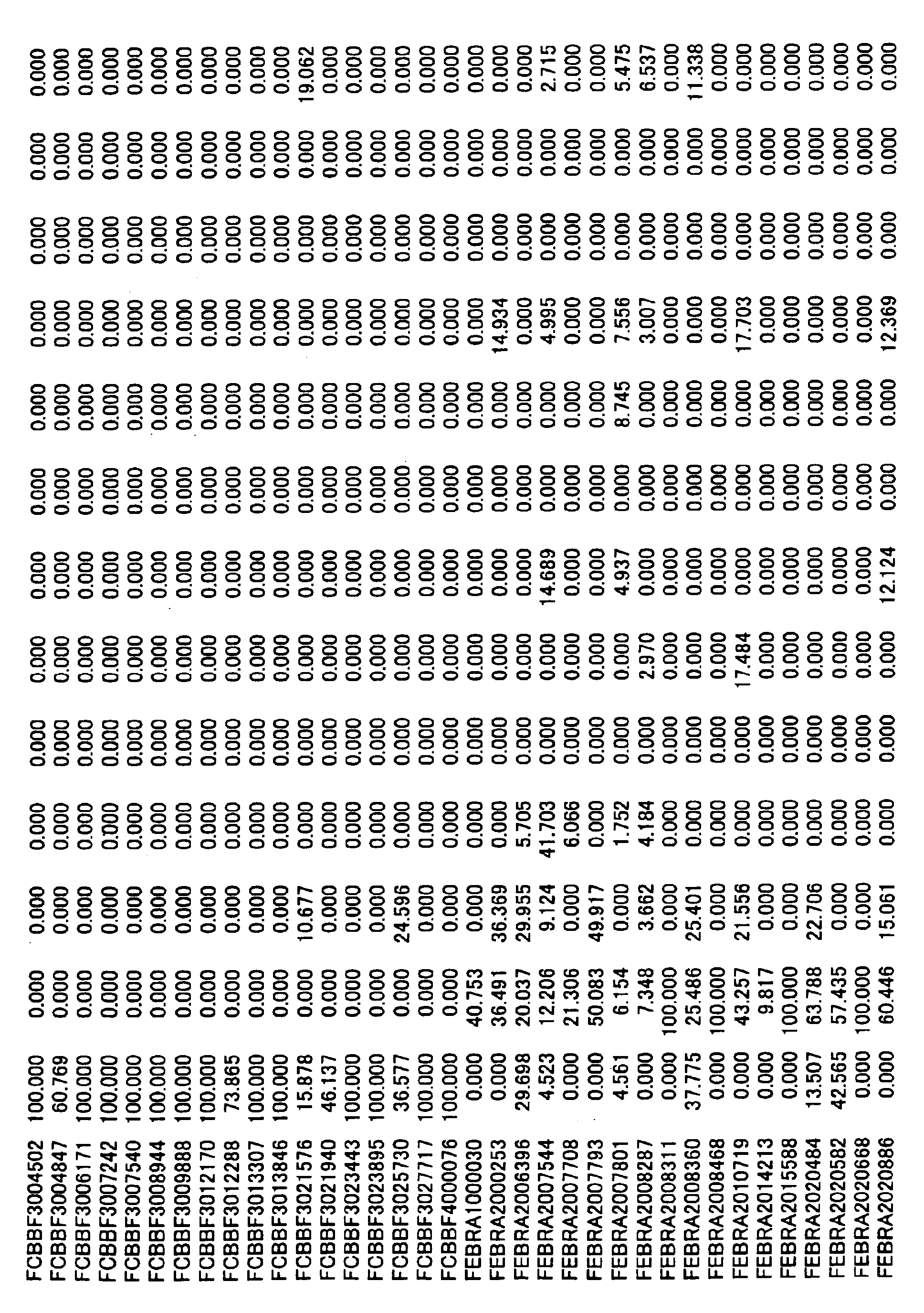

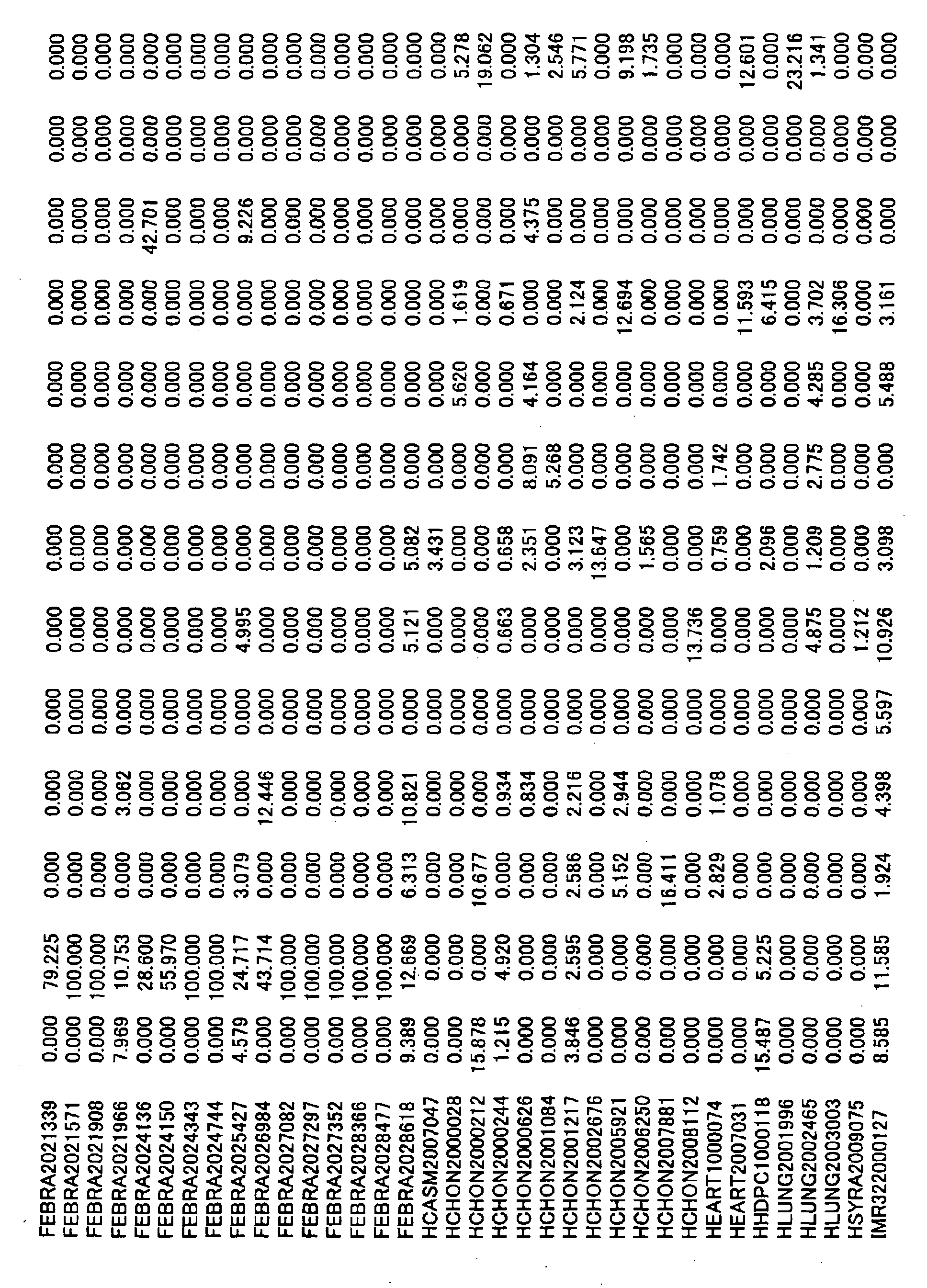

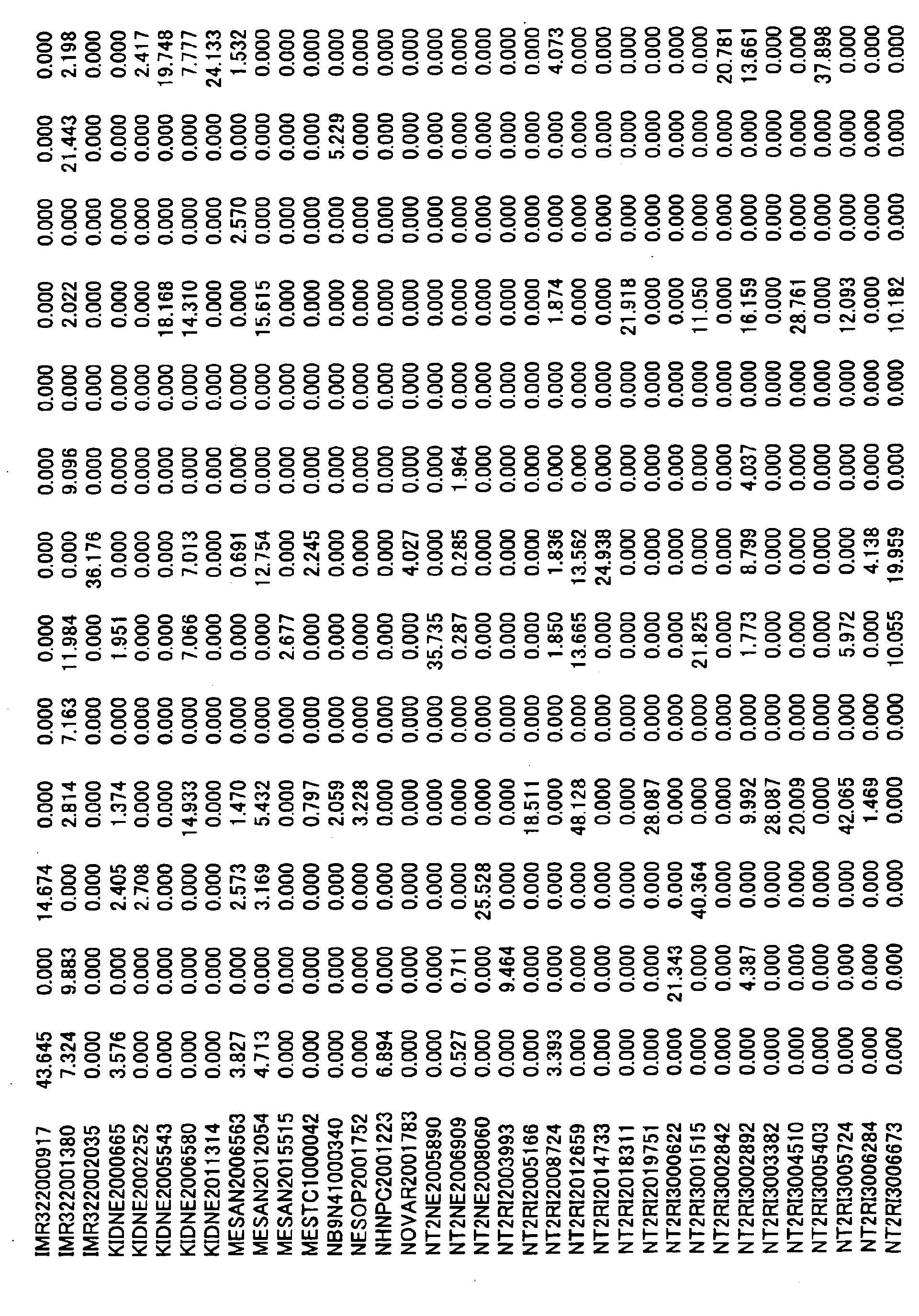

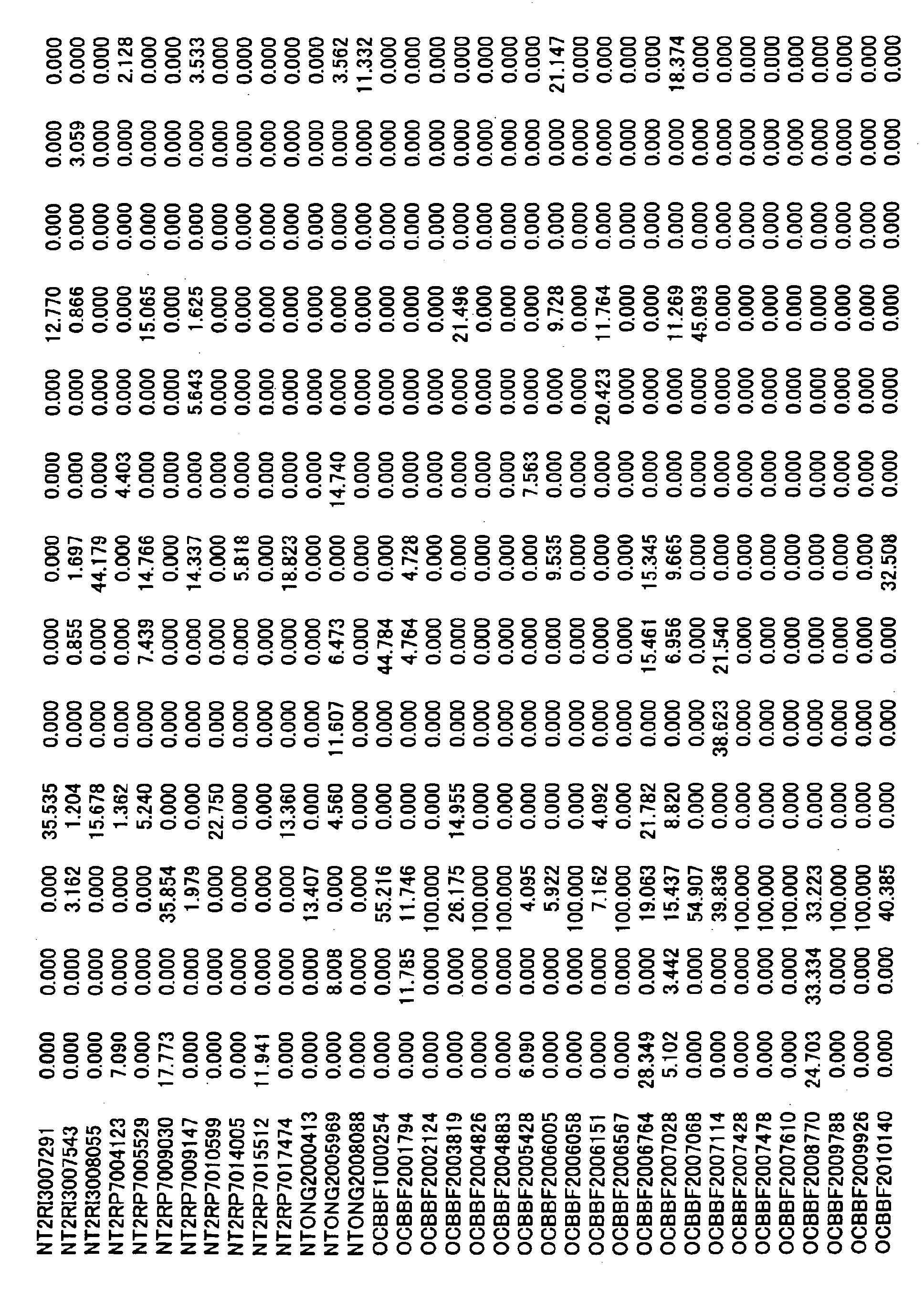

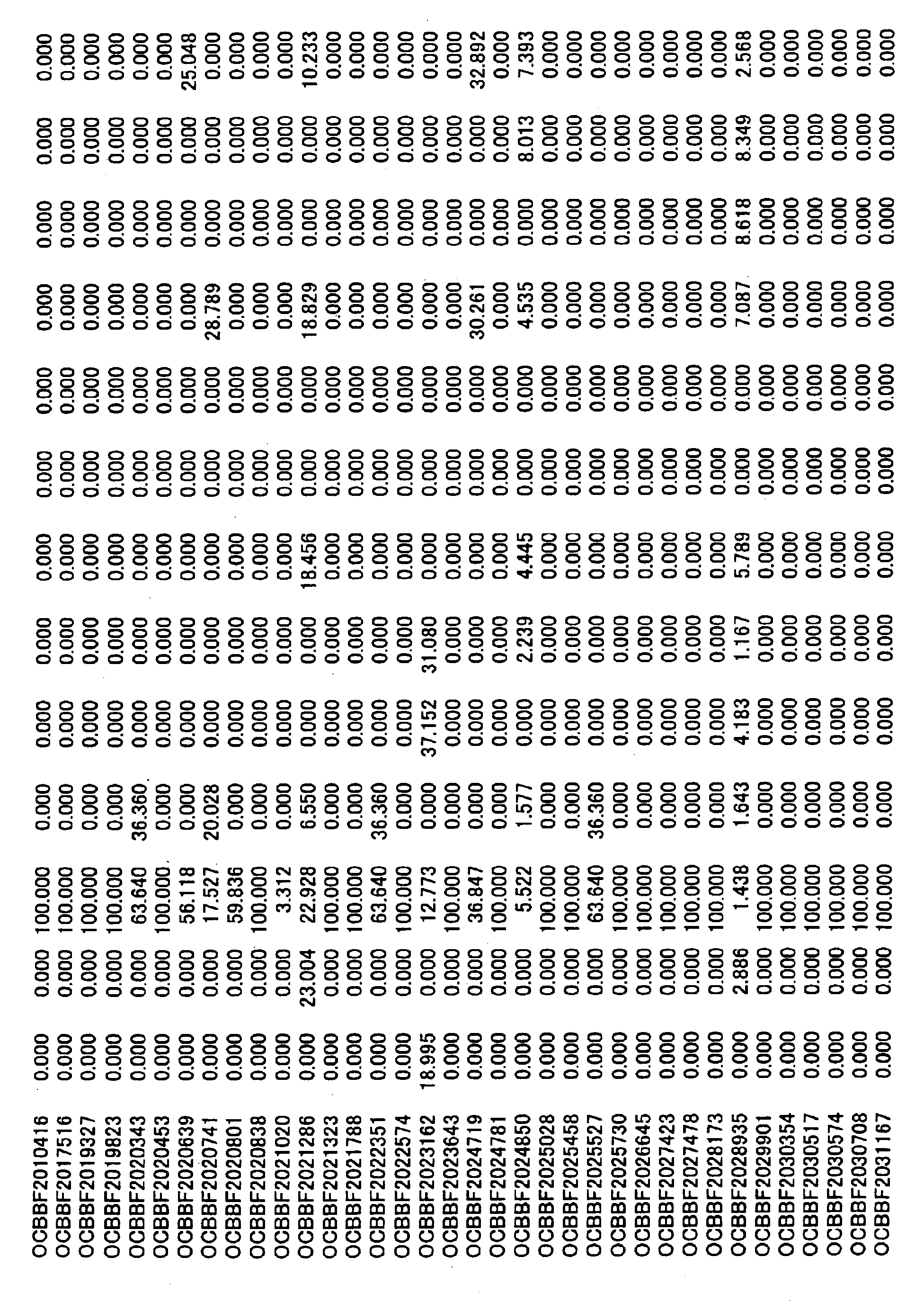

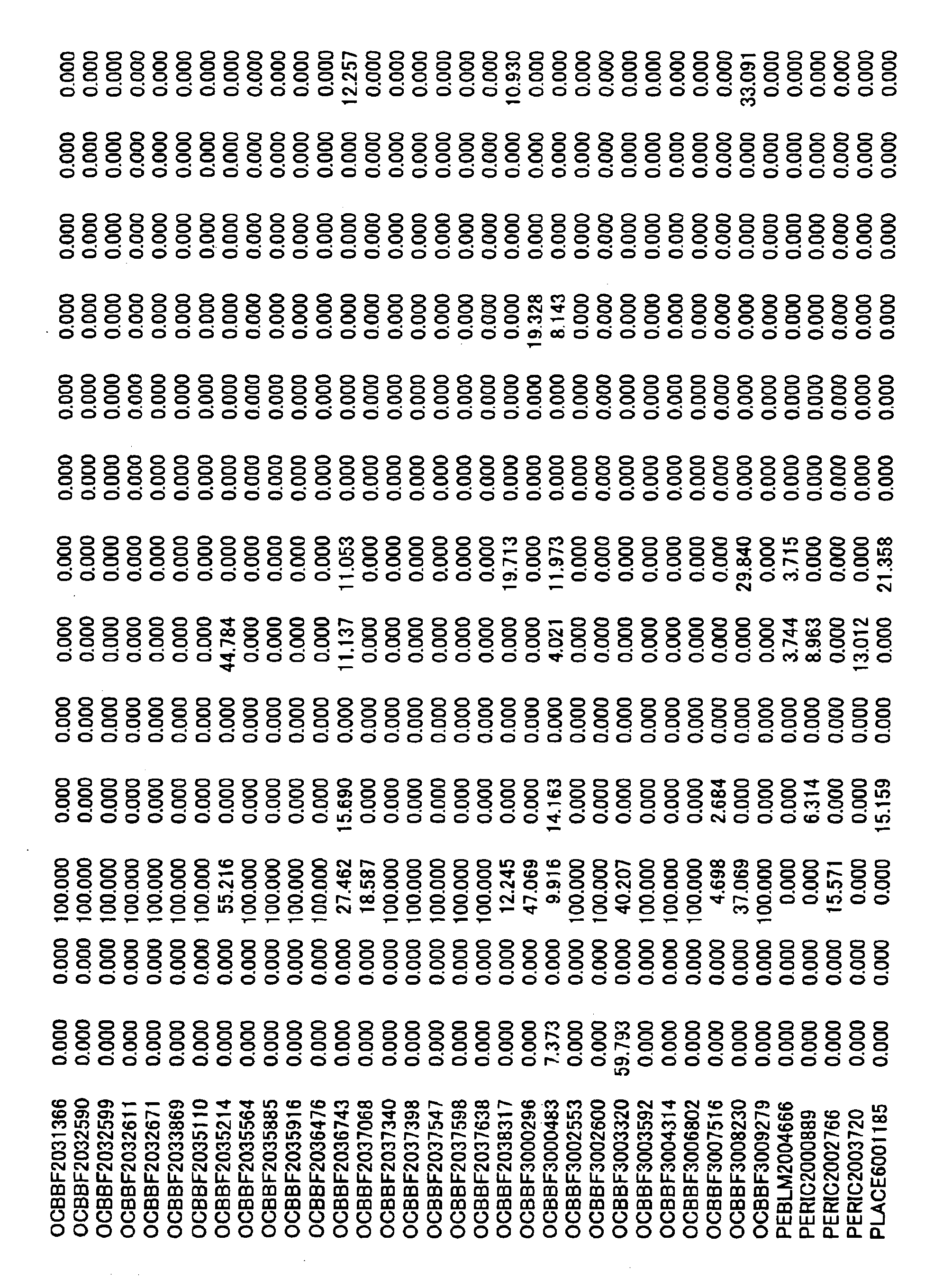

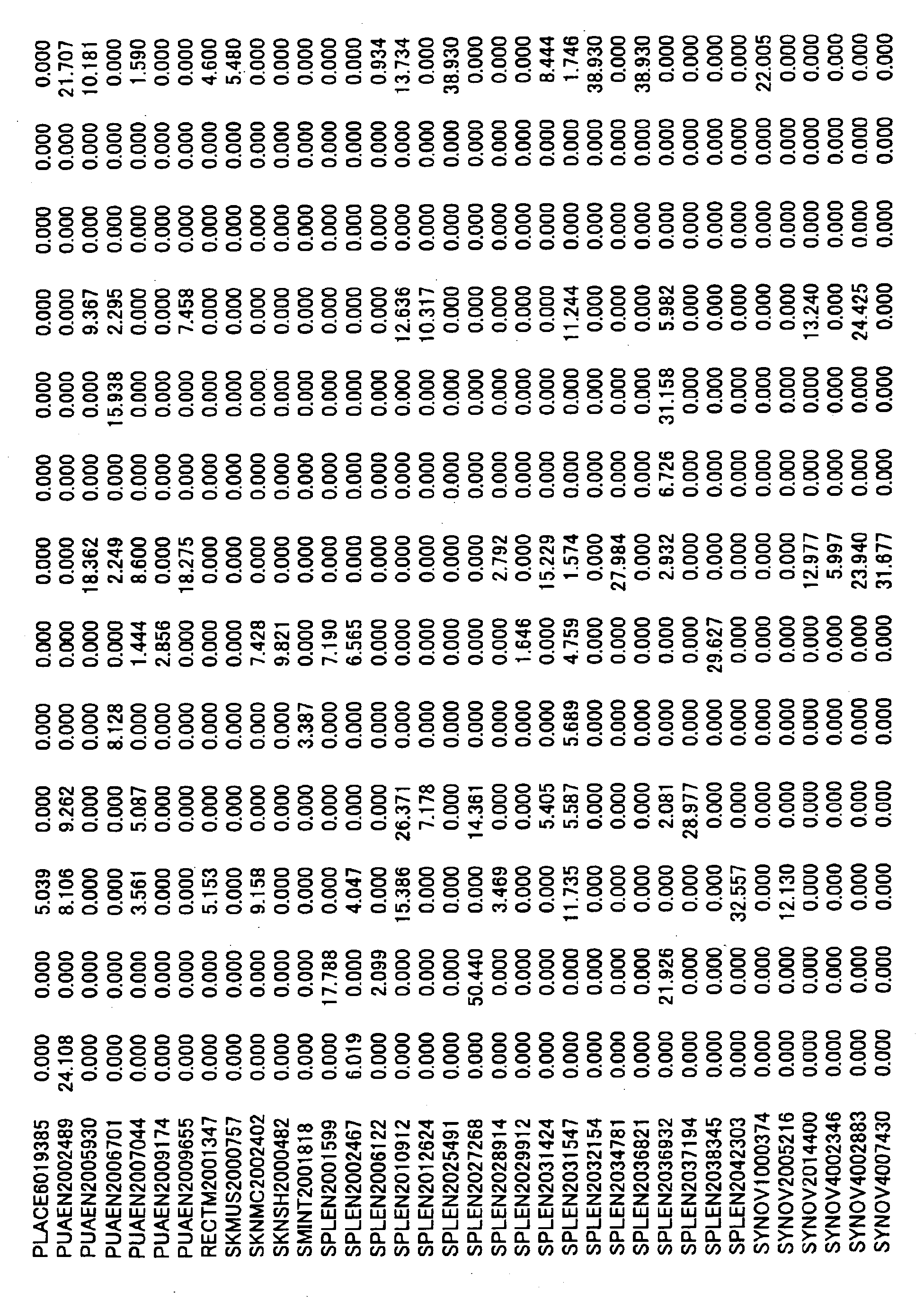

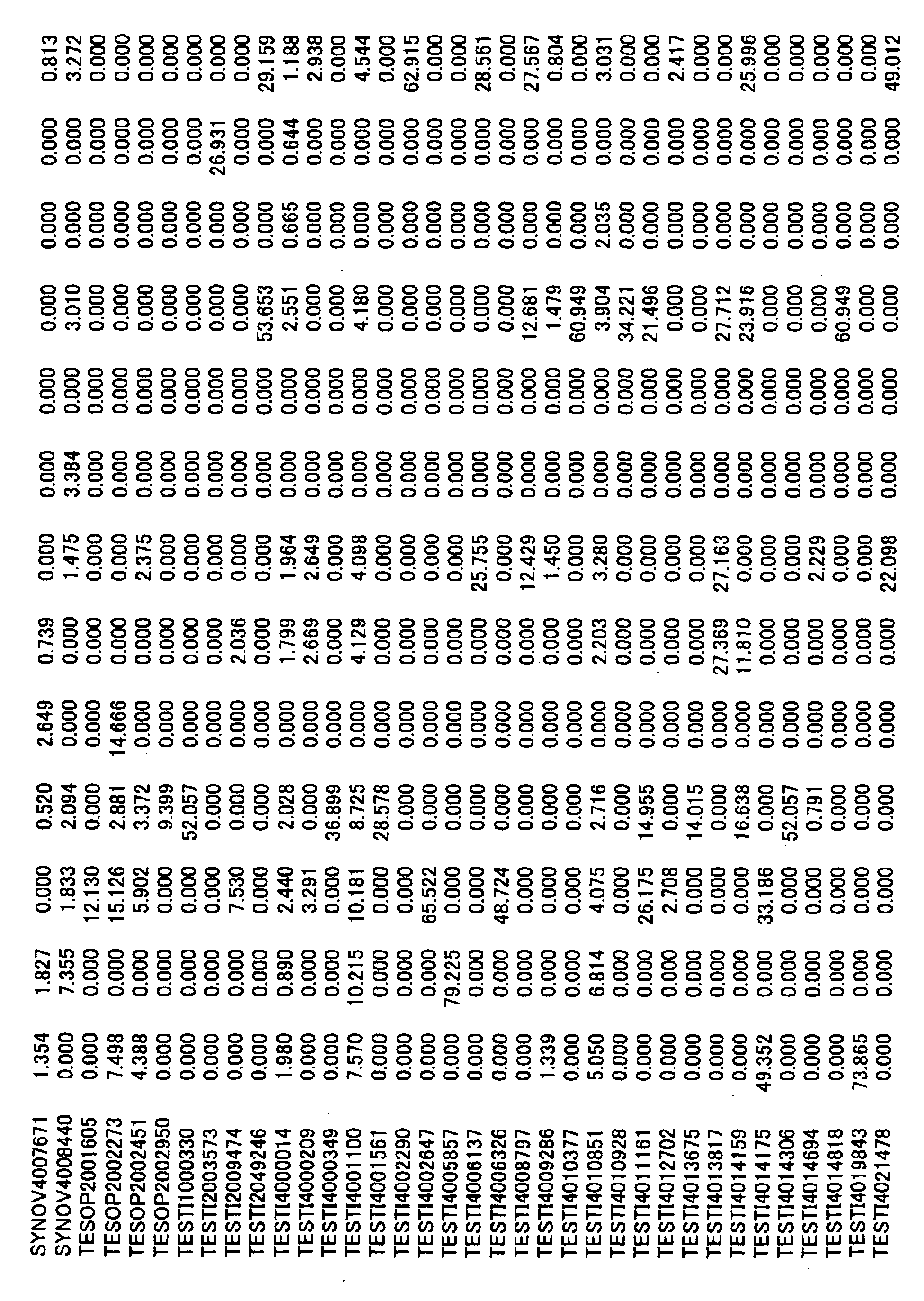

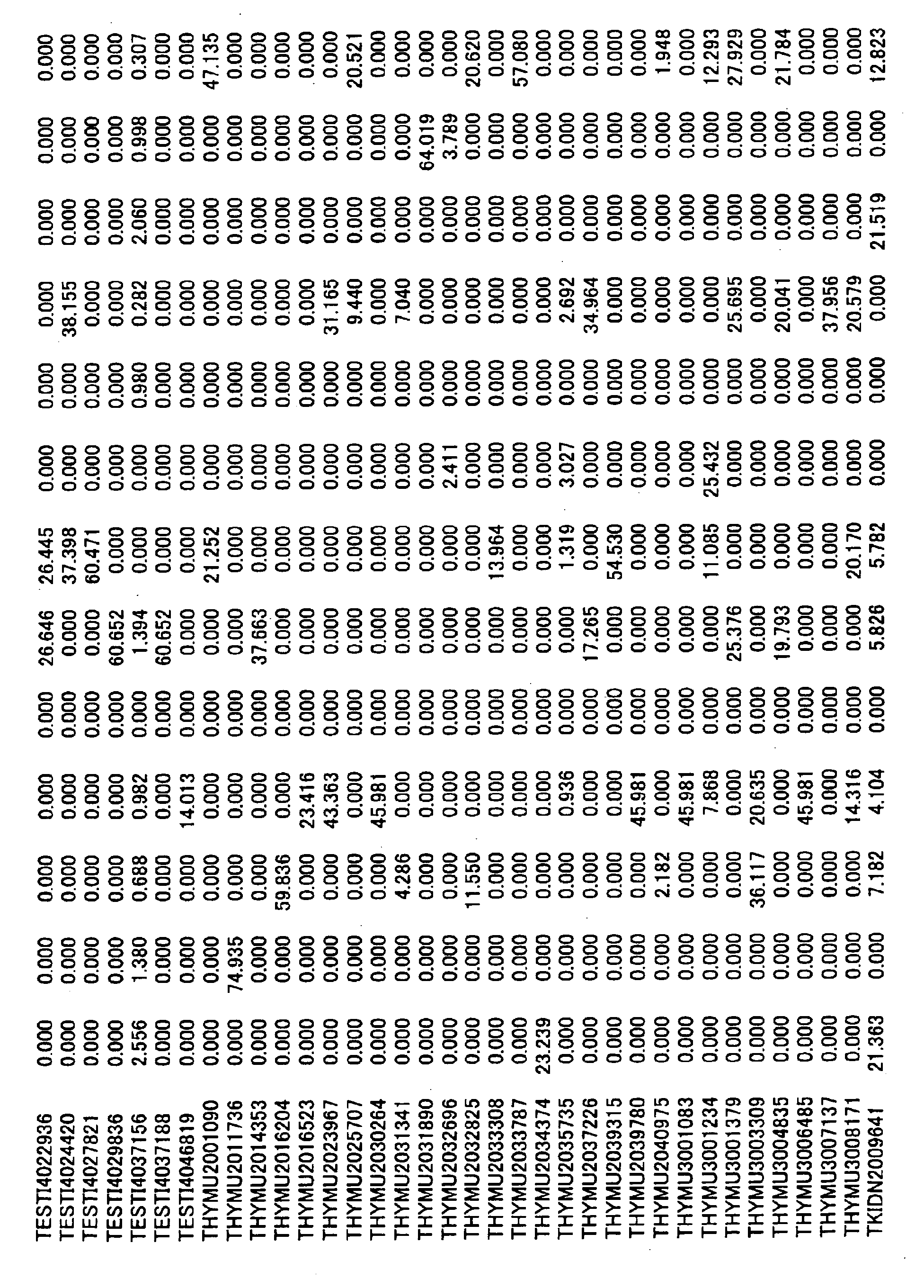

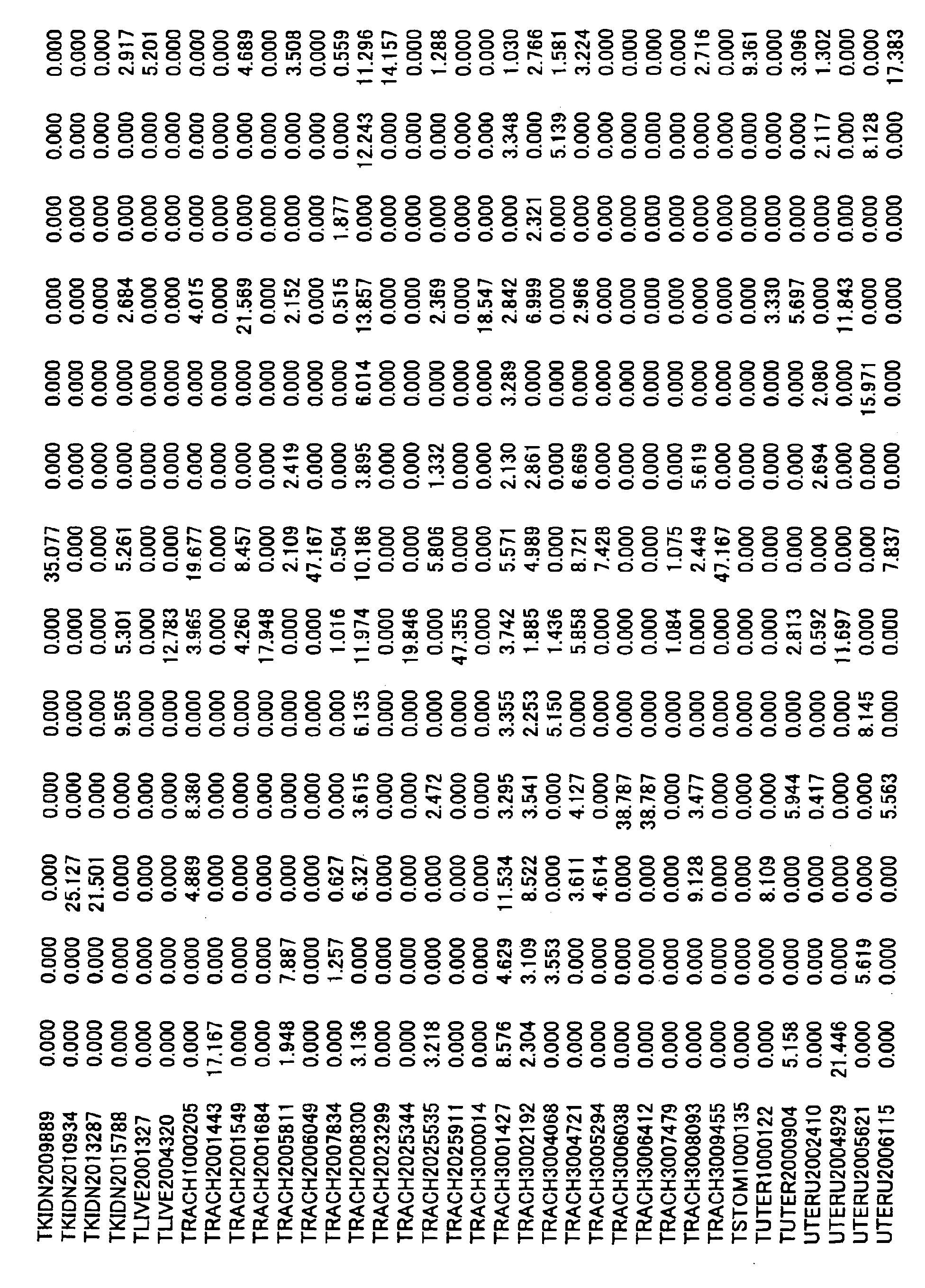

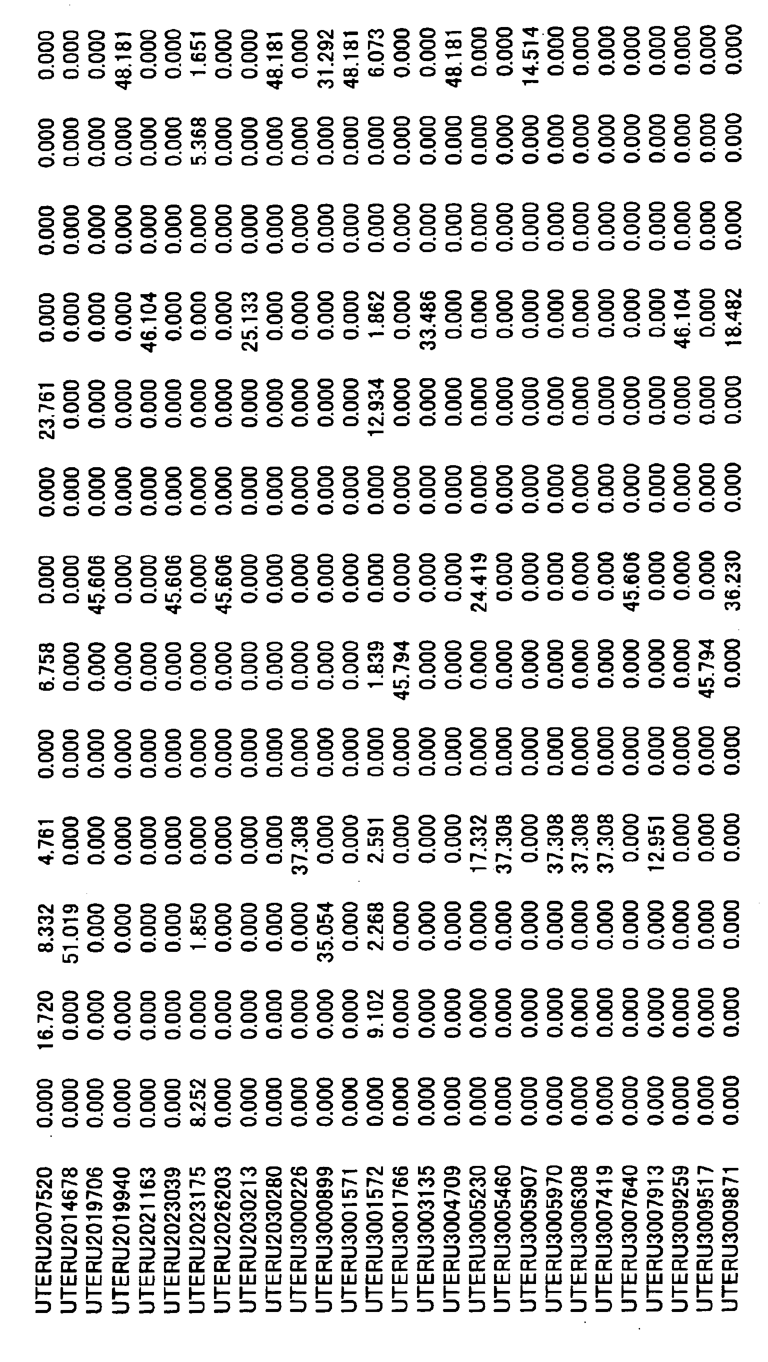

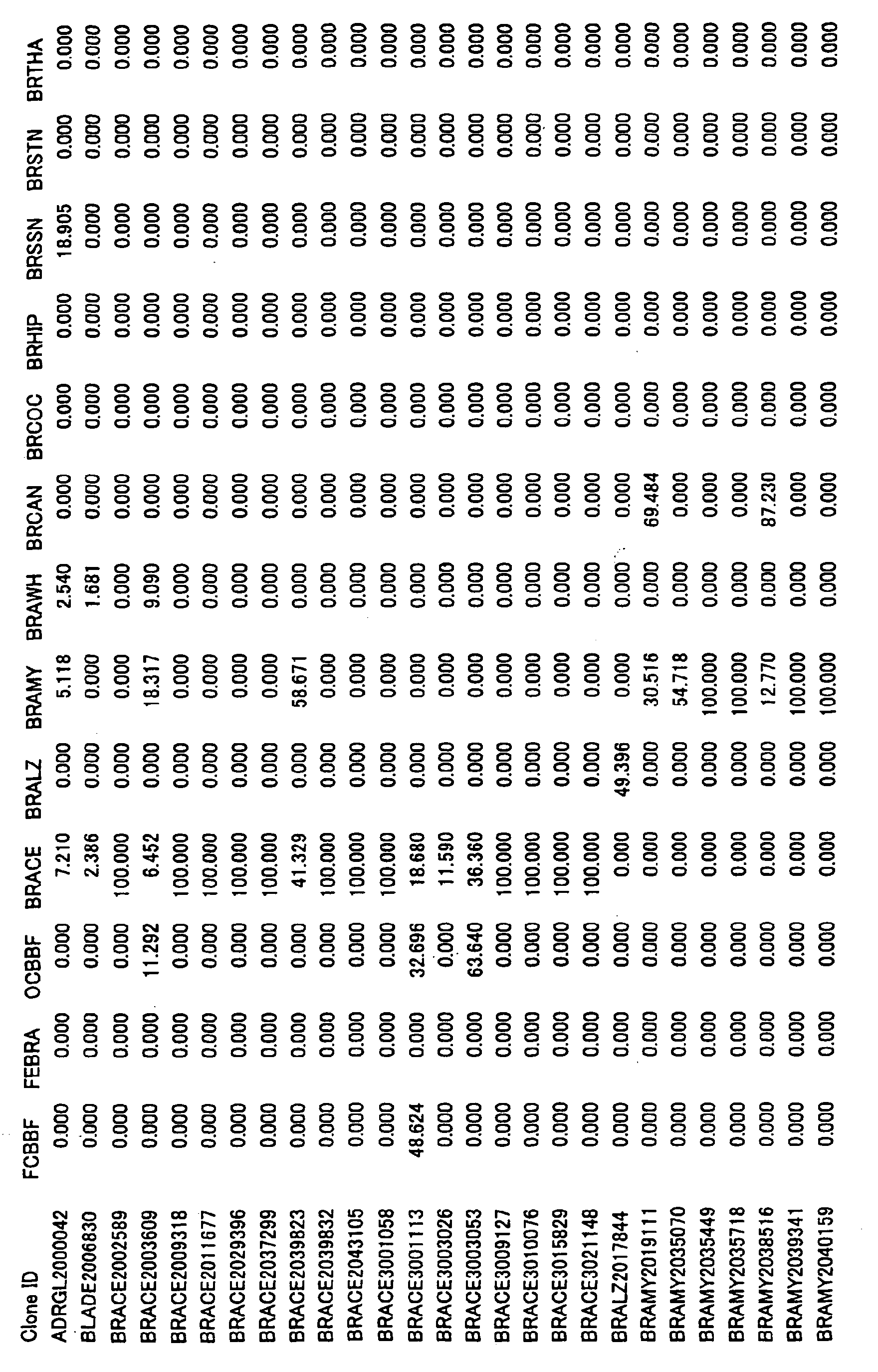

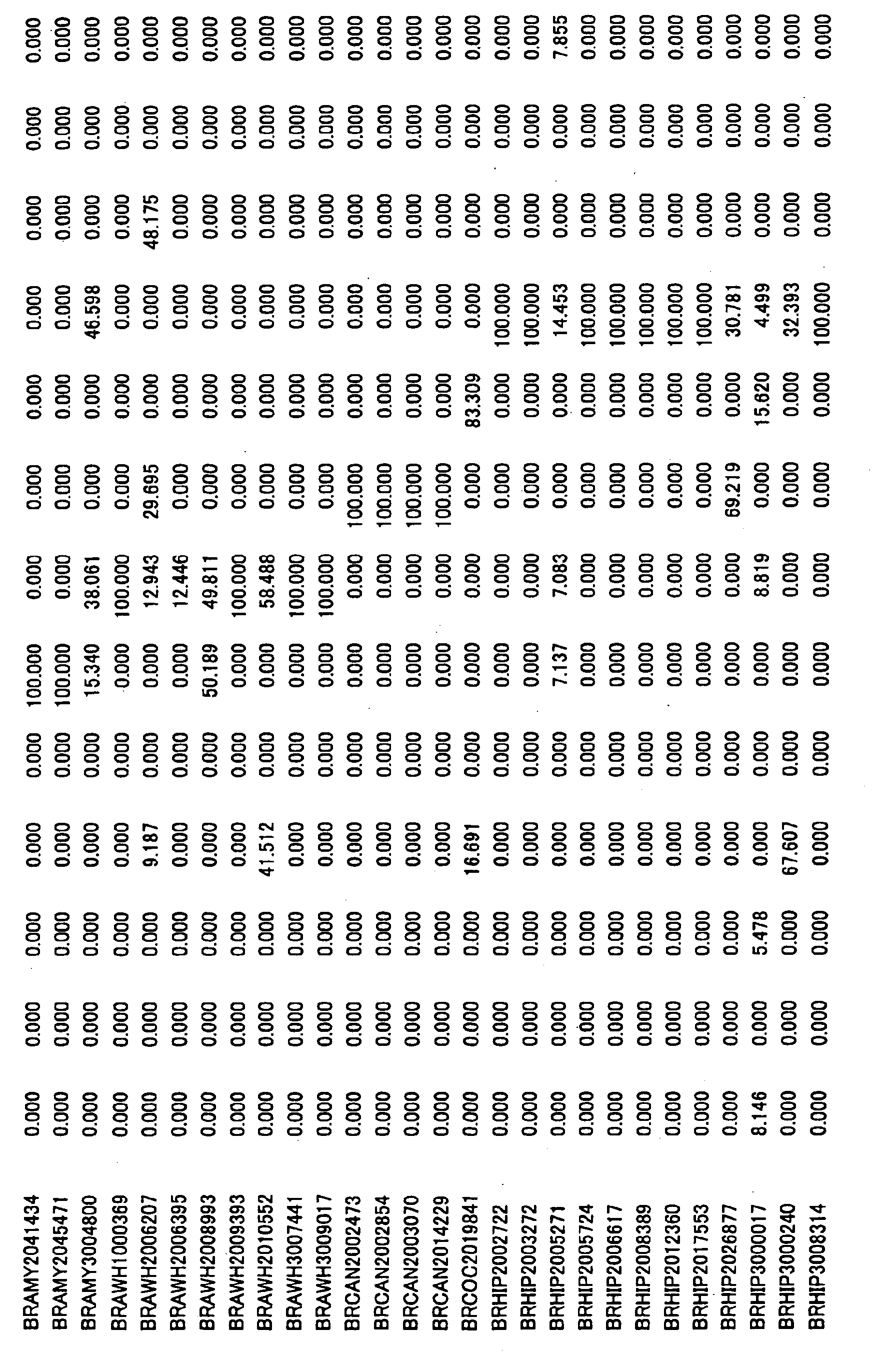

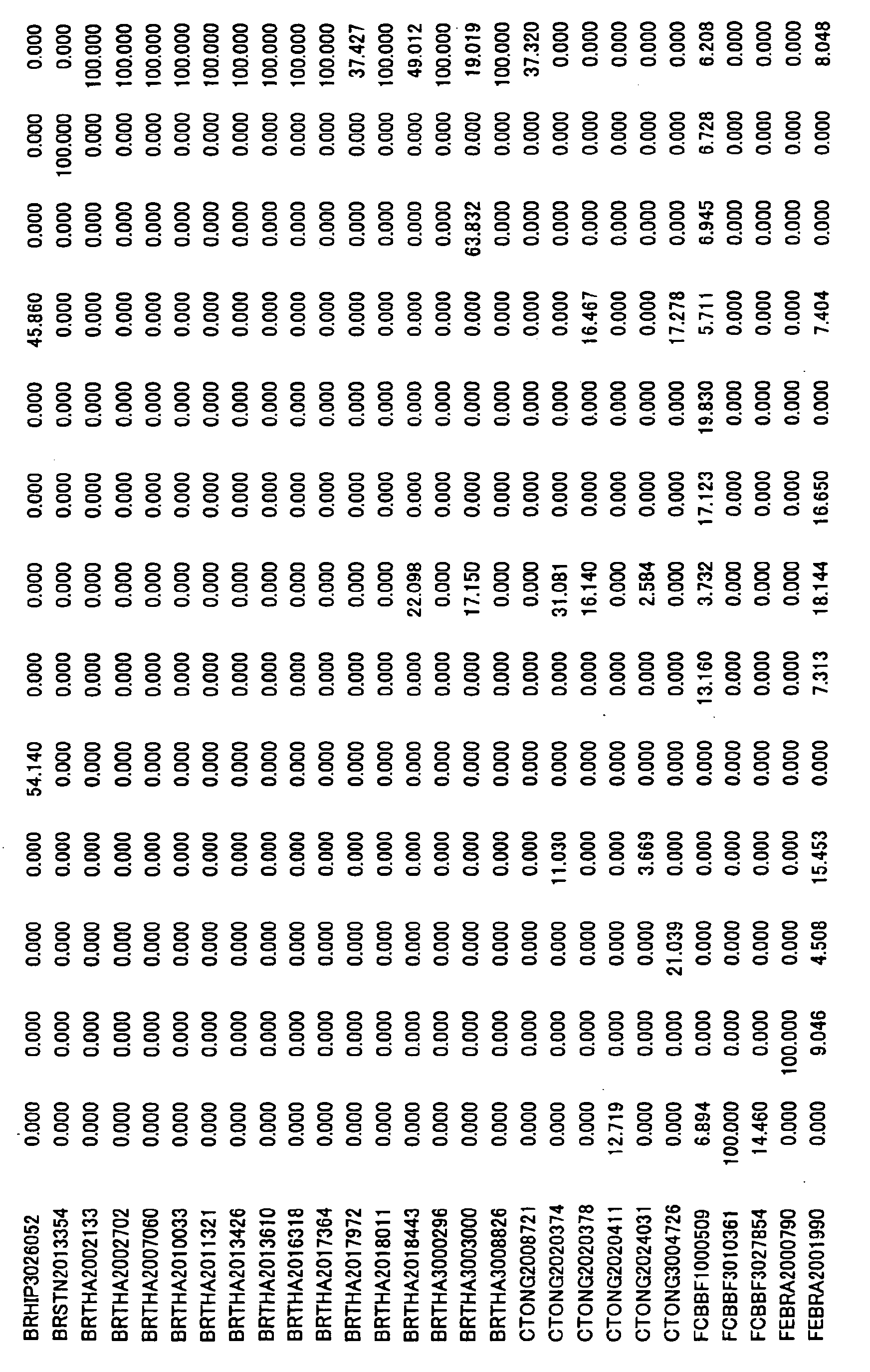

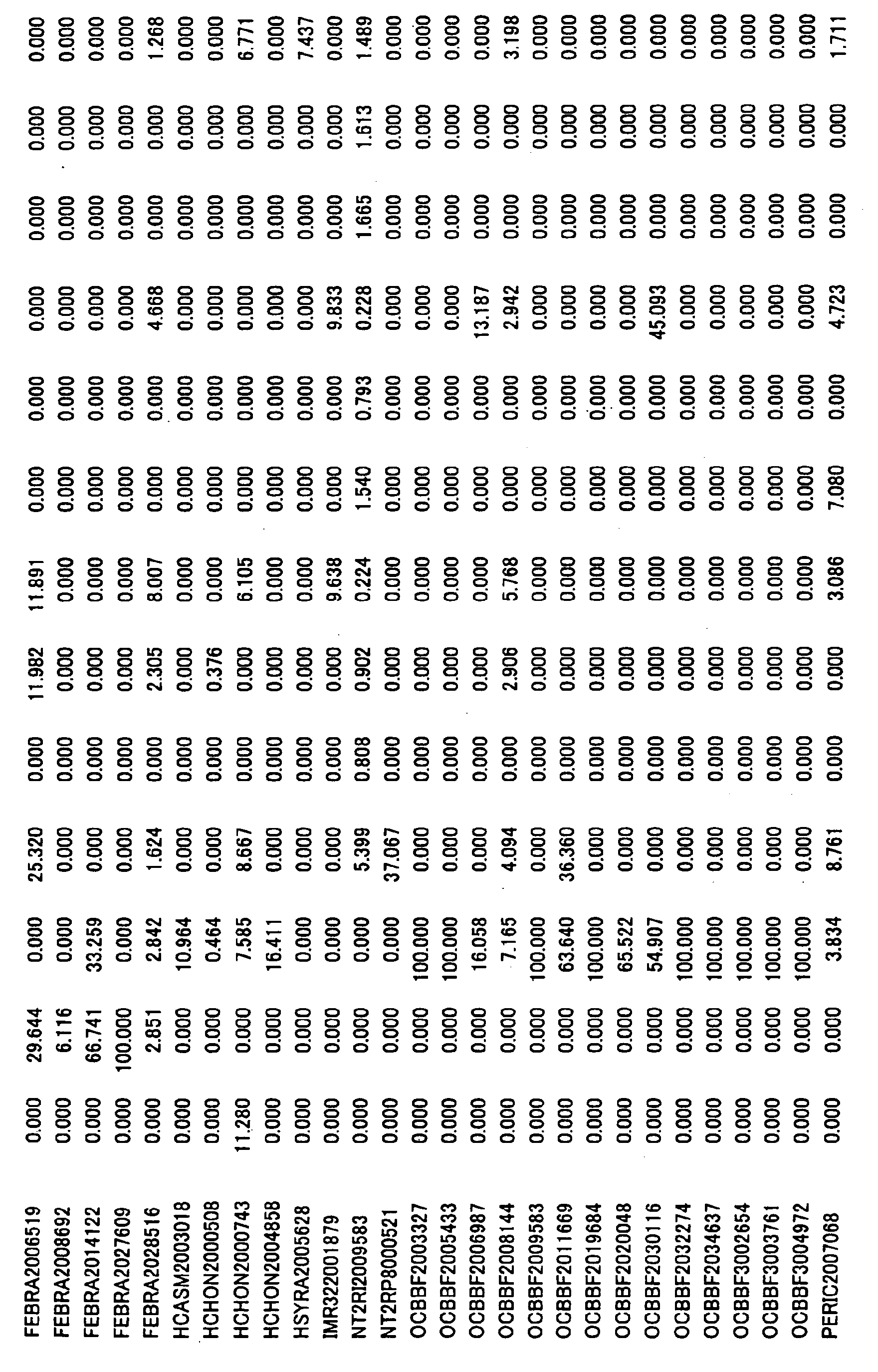

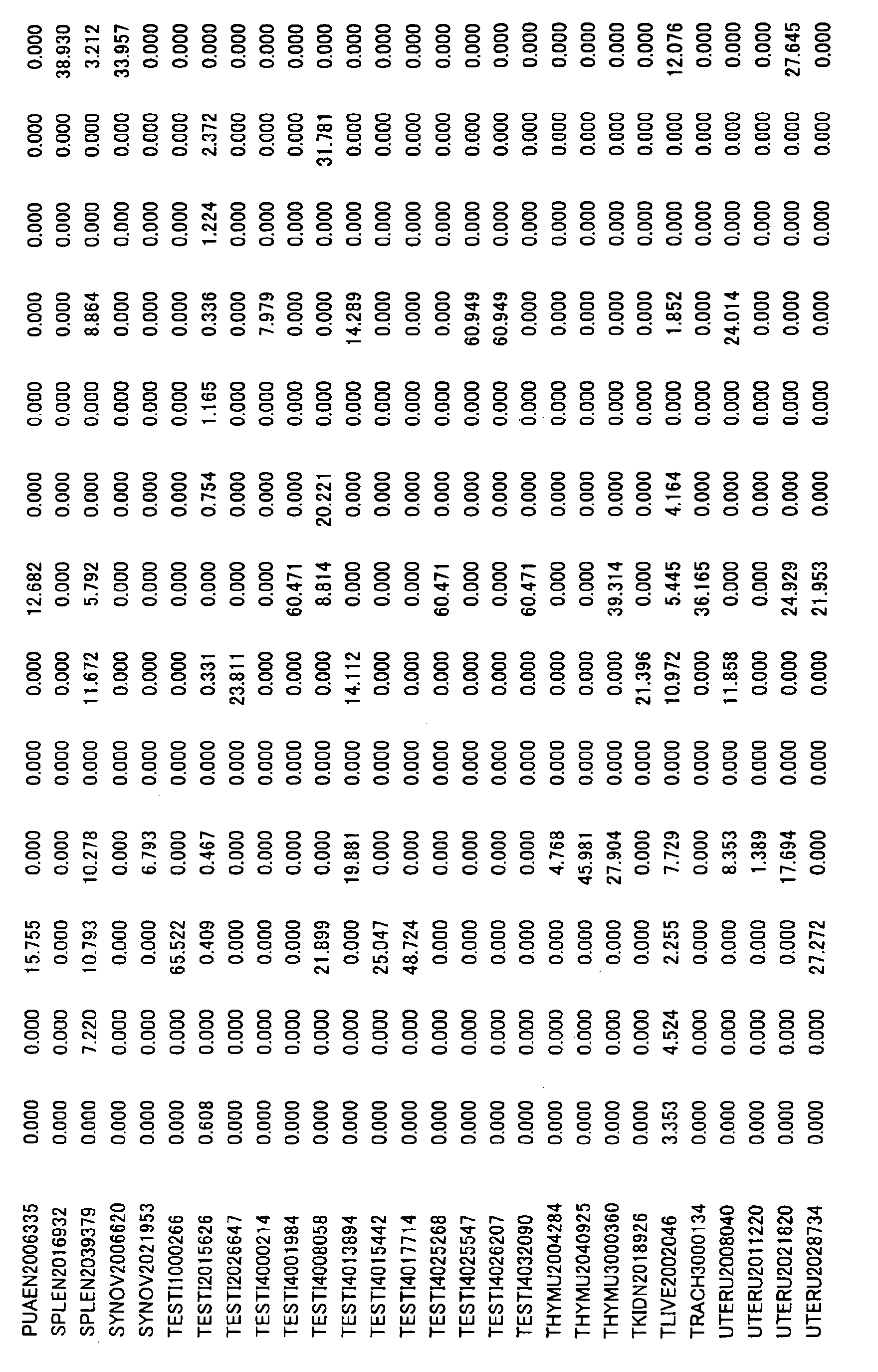

The result of comparative analysis of cDNA libraries derived from fetal heart (FEHRT) and adult heart (HEART) showed the following genes whose expression levels differed between the two.

**Table 22**

| Clone ID | FEHRT | HEART |
|---|---|---|
| BRAMY2040592 | 0.000 | 23.231 |
| BRAWH2001671 | 0.000 | 25.367 |
| BRSTN2016470 | 0.000 | 1.460 |
| CTONG2017500 | 55.163 | 0.000 |
| CTONG2028124 | 0.000 | 3.353 |
| CTONG3000657 | 0.000 | 25.884 |
| D3OST3000169 | 10.191 | 0.000 |
| FEBRA2008287 | 61.381 | 0.000 |
| HCHON2000244 | 13.698 | 0.000 |
| HCHON2000626 | 0.000 | 7.842 |
| HEART1000010 | 0.000 | 22.793 |
| HEART1000074 | 0.000 | 5.064 |
| HEART1000088 | 0.000 | 49.759 |
| HEART1000139 | 0.000 | 66.452 |
| HEART2001680 | 0.000 | 100.000 |
| HEART2001756 | 0.000 | 100.000 |
| HEART2006131 | 0.000 | 100.000 |
| HEART2006909 | 0.000 | 100.000 |
| HEART2007031 | 0.000 | 75.806 |
| HEART2010391 | 0.000 | 100.000 |
| HEART2010492 | 0.000 | 100.000 |
| HEART2010495 | 0.000 | 49.759 |
| KIDNE2000665 | 0.000 | 12.914 |
| NB9N41000340 | 0.000 | 9.675 |
| NT2RI2003993 | 0.000 | 25.326 |
| NT2RI3002892 | 0. 000 | 11.740 |
| OCBBF2024850 | 0.000 | 14.826 |
| SKMUS2006394 | 0.000 | 32.130 |
| SMINT2001818 | 0.000 | 6.254 |
| TESTI4000209 | 55.163 | 0. 000 |
| TKIDN2015788 | 54.776 | 0.000 |
| TRACH3002192 | 12.987 | 0.000 |
| TRACH3005294 | 77.338 | 0.000 |
| TRACH3007479 | 0.000 | 7.175 |
| HEART2009680 | 100.000 | 0.000 |
| THYMU2004284 | 89.631 | 0.000 |

The result of comparative analysis of cDNA libraries derived from fetal kidney (FEKID) and adult kidney (KIDNE) showed the following genes whose expression levels differed between the two.

**Table 23**

| Clone ID | FEKID | KIDNE |
|---|---|---|
| BRACE2043665 | 0.000 | 83.085 |
| BRACE3010428 | 0.000 | 3.991 |
| BRSTN2016470 | 0.000 | 0.763 |
| CTONG1000087 | 0.000 | 3.646 |
| CTONG2028124 | 0.000 | 3.504 |
| CTONG3008894 | 0.000 | 2.534 |
| HCASM2003415 | 36.285 | 0.000 |
| HCHON2000244 | 0.000 | 4.587 |
| HEART1000074 | 0.000 | 2.646 |
| HHDPC1000118 | 0.000 | 7.307 |
| KIDNE1000064 | 0.000 | 100.000 |
| KIDNE2000665 | 41.875 | 13.498 |
| KIDNE2000722 | 0.000 | 100.000 |
| KIDNE2000832 | 67.408 | 32.592 |
| KIDNE2000846 | 0.000 | 100.000 |
| KIDNE2001361 | 0.000 | 80.699 |
| KIDNE2001847 | 0.000 | 12.687 |
| KIDNE2002252 | 0.000 | 7.600 |
| KIDNE2002991 | 0.000 | 100.000 |
| KIDNE2003837 | 0.000 | 100.000 |
| KIDNE2005543 | 0.000 | 62.084 |
| KIDNE2006580 | 0.000 | 48.900 |
| KIDNE2010264 | 0.000 | 100.000 |
| KIDNE2011314 | 0.000 | 75.867 |
| KIDNE2011532 | 0.000 | 100.000 |
| KIDNE2011635 | 0.000 | 48.502 |
| KIDNE2012945 | 0.000 | 48.732 |
| KIDNE2013095 | 0.000 | 100.000 |
| PEBLM2004666 | 0.000 | 12.953 |
| PLACE6019385 | 87.742 | 0.000 |
| STOMA1000189 | 0.000 | 16.356 |
| SYNOV4007671 | 0.000 | 2.556 |
| TBAES2001258 | 0.000 | 33.137 |
| TESOP2002451 | 51.382 | 0.000 |
| TESTI4000014 | 0.000 | 1.245 |
| TESTI4012702 | 0.000 | 7.600 |
| THYMU2032014 | 0.000 | 57.532 |
| TRACH2001684 | 0.000 | 62.100 |
| TRACH2007834 | 0.000 | 1.758 |
| UTERU2023175 | 0.000 | 5.190 |
| NT2RI2009583 | 10.920 | 4.840 |
| OCBBF2008144 | 0.000 | 62.383 |

The result of comparative analysis of cDNA libraries derived from fetal lung (FELNG) and adult lung (HLUNG) showed the following genes whose expression levels differed between the two.

**Table 24**

| Clone ID | FELNG | HLUNG |
|---|---|---|
| BRAWH3007592 | 38.566 | 0.000 |
| BRCAN2021028 | 0.000 | 38.589 |
| BRHIP2000819 | 0.000 | 7.923 |
| BRSTN2016470 | 4.707 | 0.803 |
| CTONG1000087 | 0.000 | 3.835 |
| CTONG2028124 | 10.809 | 9.217 |
| HCASM2007047 | 73.789 | 0.000 |
| HEART1000074 | 32.652 | 2.784 |
| HLUNG1000017 | 0.000 | 100.000 |
| HLUNG2000014 | 0.000 | 100.000 |
| HLUNG2001996 | 0.000 | 76.784 |
| HLUNG2002465 | 26.011 | 4.436 |
| HLUNG2002958 | 0.000 | 100.000 |
| HLUNG2003003 | 0.000 | 29.311 |
| HLUNG2003872 | 0.000 | 100.000 |
| HLUNG2010464 | 0.000 | 100.000 |
| HLUNG2011041 | 0.000 | 100.000 |
| HLUNG2011298 | 0.000 | 35.254 |
| HLUNG2012049 | 0.000 | 100.000 |
| HLUNG2012287 | 0.000 | 100.000 |
| HLUNG2012727 | 0.000 | 100.000 |
| HLUNG2013204 | 0.000 | 100.000 |
| HLUNG2013304 | 0.000 | 100.000 |
| HLUNG2013622 | 0.000 | 100.000 |
| HLUNG2013851 | 0.000 | 100.000 |
| HLUNG2014262 | 0.000 | 100.000 |
| HLUNG2014288 | 0.000 | 100.000 |
| HLUNG2014449 | 0.000 | 100.000 |
| HLUNG2015617 | 0.000 | 100.000 |
| HLUNG2017350 | 0.000 | 100.000 |
| HLUNG2017546 | 0.000 | 12.944 |
| HLUNG2017806 | 0.000 | 100.000 |
| HLUNG2019058 | 0.000 | 100.000 |
| HSYRA2008376 | 0.000 | 11.470 |
| KIDNE2012945 | 0.000 | 51.268 |
| NT2RI2003993 | 0.000 | 13.924 |
| NT2RI3007543 | 18.247 | 0.000 |
| OCBBF3000483 | 0.000 | 14.638 |
| SMINT1000192 | 64.044 | 0.000 |
| SPLEN2028914 | 0.000 | 10.242 |
| SPLEN2031547 | 0.000 | 5.775 |
| STOMA1000189 | 33.633 | 0.000 |
| SYNOV4007671 | 0.000 | 2.689 |
| TESOP1000127 | 0.000 | 34.899 |
| TESTI2003573 | 0.000 | 27.394 |
| TESTI4000014 | 0.000 | 0.655 |
| TESTI4037156 | 0.000 | 2.030 |
| TRACH2005811 | 0.000 | 3.868 |
| TRACH3004068 | 0.000 | 5.227 |
| UTERU2005621 | 0.000 | 8.268 |
| UTERU2023175 | 32.020 | 0.000 |
| FEBRA2028516 | 4.195 | 0.000 |
| HCHON2000508 | 1.370 | 0.000 |
| HLUNG2013350 | 100.000 | 0.000 |
| HLUNG2015418 | 76.605 | 0.000 |
| HLUNG2015548 | 100.000 | 0.000 |
| HLUNG2016862 | 100.000 | 0.000 |
| TESTI2015626 | 2.412 | 0.000 |
| TRACH2019672 | 76.605 | 0.000 |

### EXAMPLE 9

### In-silico expression frequency analysis based on large-scale analysis data for 5'-end sequences

Analyzing the expression level of a gene in various organs, tissues or cells is an exceedingly important step in clarifying the function of that organ, tissue, or cell, as well as in clarifying gene networks and in studying a causative gene of a morbid state. For example, if the expression level of a gene differs between a normal tissue and a malignant tissue, there is a possibility that that gene is involved in cancer. If tissue canceration can be suppressed by downregulating the expression of that gene, a compound comprising such downregulating activity can be used as an anticancer drug.

Various methods for analyzing gene expression frequency have been developed. For example, wet-type experiment-based methods include Northern blotting and RT-PCR, and of the use of gene chips and microarrays, where target samples synthesized from tissue or cell-derived RNA are hybridized to polynucleotides that comprise partial gene sequences synthesized on a base, or cDNA clones attached as plasmids directly to a base, and then signals are detected (Experiment Medicine, Vol. 17, No. 8, 980-1056 (1999), Eds., Muramatsu and Nawa, Cell Technology, Suppl. "DNA Microarray and New PCR Methods" (Shujunsha, 2000)). A method called "ATAC-PCR" is also available (Kato. K (1997) Nucleic Acids Res. 25, 4694-6), which comprises the steps of cleaving cDNA synthesized from tissue or cell-derived RNA, attaching adapters of different length depending on the type of tissue or cell, carrying out competitive PCR using a primer which contains a fluorescent dye and a sequence complementary to the adapter, and a primer specific to the gene, and then analyzing the expression level of the gene. In addition, an in-silico analysis-based method using sequence data is available. A database called BODYMAP (http://bodymap.ims.u-tokyo.ac.jp/) has been constructed by randomly extracting gene clones from cDNA libraries of various tissues and cells, combining clones homologous to one another as a cluster, classifying the genes in each cluster unit based on homology information on the nucleotide sequences of cDNA 3' ends, and then obtaining information on gene expression frequency by comparing the number of clones in respective clusters.

Both experiment-based and in-silico analysis-based methods are widely accepted methods of gene expression analysis. These methods, and almost all other established methods for analyzing the frequency of gene expression, are based on analysis of 3'-end gene sequences. In human genes the 3'-untranslated regions (3'-UTR) are usually long, and thus there is controversy as to whether the results of such methods directly correspond to gene expression frequency and polypeptide expression pattern. However, the analysis method for gene expression frequency of the present invention is based on 5'-end gene sequences, and thus the expression pattern of each gene is a more accurate and faithful reproduction of the original in vivo pattern.

cDNA libraries in which the probability of cDNA completeness is exceedingly high were prepared from various tissues and cells as described in Example 1. cDNA clones were randomly selected from each library, the cDNA 5'-end sequences were determined, and a sequence database was constructed using this data. This database contains the nucleotide sequences of 1,402,069 clones, a sufficiently large population for analysis. The libraries used in the analysis, the number of cDNA clones whose 5'-end sequences were determined, and the weighted value for a single sequence in each library (100 divided by the number of sequences analyzed in each library) are described below.

**Table 25**

| Library Names | Number of clones whose 5' ends were analyzed | Weighted values for single sequences (100/Number of sequences) |
|---|---|---|
| 3NB69 | 8182 | 0.012221951 |
| ACTVT | 684 | 0.14619883 |
| ADIPS | 614 | 0.16286645 |
| ADRGL | 10300 | 0.009708738 |
| AHMSC | 671 | 0.149031297 |
| ASTRO | 17226 | 0.005805178 |
| BEAST | 2736 | 0.036549708 |
| BGGI1 | 1904 | 0.052521008 |
| BLADE | 8503 | 0.011760555 |
| BNGH4 | 7739 | 0.012921566 |
| BRACE | 84087 | 0.001189244 |
| BRALZ | 16517 | 0.006054368 |
| BRAMY | 59232 | 0.001688277 |
| BRASW | 158 | 0.632911392 |
| BRAWH | 59682 | 0.001675547 |
| BRCAN | 26014 | 0.003844084 |
| BRCOC | 16847 | 0.005935775 |
| BRHIP | 58498 | 0.00170946 |
| BRSSN | 16035 | 0.006236358 |
| BRSTN | 16552 | 0.006041566 |
| BRTHA | 53818 | 0.001858114 |
| CD34C | 1421 | 0.070372977 |
| CERVX | 2888 | 0.034626039 |
| CHONS | 2694 | 0.037119525 |
| COLON | 8501 | 0.011763322 |
| CORDB | 711 | 0.140646976 |
| CTONG | 32043 | 0.003120806 |
| D3OST | 5112 | 0.019561815 |
| D60ST | 889 | 0.112485939 |
| D9OST | 4426 | 0.022593764 |
| DFNES | 10126 | 0.009874593 |
| ERLTF | 2178 | 0.045913682 |
| FCBBF | 32305 | 0.003095496 |
| FEBRA | 23941 | 0.004176935 |
| FEHRT | 2866 | 0.034891835 |
| FEKID | 2759 | 0.036245016 |
| FELIV | 186 | 0.537634409 |
| FELNG | 2775 | 0.036036036 |
| HCASM | 8989 | 0.011124708 |
| HCHON | 9432 | 0.010602205 |
| HEART | 8946 | 0.01117818 |
| HELAC | 680 | 0.147058824 |
| HHDPC | 8476 | 0.011798018 |
| HLUNG | 16272 | 0.006145526 |
| HSYRA | 7985 | 0.012523482 |
| IMR32 | 16914 | 0.005912262 |
| JCMLC | 2171 | 0.046061723 |
| KIDNE | 17119 | 0.005841463 |
| LIVER | 6885 | 0.014524328 |
| LYMPB | 2630 | 0.038022814 |
| MAMGL | 184 | 0.543478261 |
| MESAN | 16095 | 0.00621311 |
| MESTC | 691 | 0.1447178 |
| N1ESE | 2628 | 0.03805175 |
| NB9N4 | 1764 | 0.056689342 |
| NCRRM | 703 | 0.142247511 |
| NCRRP | 699 | 0.143061516 |
| NESOP | 2805 | 0.035650624 |
| NETRP | 9236 | 0.010827198 |
| NHNPC | 2392 | 0.04180602 |
| NOVAR | 2504 | 0.039936102 |
| NT2NE | 16468 | 0.006072383 |
| NT2RI | 32842 | 0.003044882 |
| NT2RM | 2074 | 0.048216008 |
| NT2RP | 24763 | 0.004038283 |
| NTISM | 181 | 0.552486188 |
| NTONG | 8098 | 0.012348728 |
| OCBBF | 48042 | 0.002081512 |
| PANCR | 182 | 0.549450549 |
| PEBLM | 7940 | 0.012594458 |
| PERIC | 8860 | 0.011286682 |
| PLACE | 33535 | 0.002981959 |
| PROST | 16829 | 0.005942124 |
| PUAEN | 10577 | 0.009454477 |
| RECTM | 2743 | 0.036456435 |
| SALGL | 185 | 0.540540541 |
| SKMUS | 8470 | 0.011806375 |
| SKNMC | 7656 | 0.013061651 |
| SKNSH | 8692 | 0.011504832 |
| SMINT | 16913 | 0.005912612 |
| SPLEN | 34307 | 0.002914857 |
| STOMA | 8770 | 0.011402509 |
| SYNOV | 27671 | 0.003613892 |
| T1ESE | 2687 | 0.037216226 |
| TBAES | 8484 | 0.011786893 |
| TCERX | 2828 | 0.035360679 |
| TCOLN | 2815 | 0.035523979 |
| TESOP | 8723 | 0.011463946 |
| TESTI | 91301 | 0.001095278 |
| THYMU | 71574 | 0.001397155 |
| TKIDN | 16123 | 0.00620232 |
| TLIVE | 8681 | 0.01151941 |
| TLUNG | 2884 | 0.034674064 |
| TOVAR | 2740 | 0.03649635 |
| TRACH | 53281 | 0.001876842 |
| TSTOM | 2779 | 0.035984167 |
| TUTER | 2678 | 0.037341299 |
| UMVEN | 633 | 0.157977883 |
| UTERU | 50040 | 0.001998401 |

Using a nucleotide sequence homology search program, the nucleotide sequences of respective clones in this database were categorized (clustered) into groups, each of which contained homologous sequences. Cluster members (the number of clones whose 5' ends were analyzed) were totalized and normalized for each library, resulting in an abundance ratio of genes in the cDNA library. In addition, 17,547 genes, including full-length cDNAs of the present invention, were assigned to a cluster using a nucleotide sequence homology search program.

In addition to the abundance ratio of the assigned genes in cDNA libraries, the sums of the weighted values for single sequences in each library were also calculated. For example, the expression of the clone BRACE2000753 was detectable in the BRACE, SPLEN, and THYMU libraries, based on expression analysis results. The single-sequence weighted values for these libraries are 0.001189244, 0.002914857, and 0.001397155 respectively, as seen in Table 1. Thus, their sum is 0.005501257. This parameter serves as an index for gene expression frequency in entire libraries. The higher this value, the greater the gene expression level. Listed below are the clone names of the 17,176 genes assigned to clusters, the number of cDNA clones whose 5' end sequences were determined, and the sum of the weighted values for single sequences, cluster names, and the number of clones whose 5' ends were analyzed.

**Table 26**

| Names of clones assigned to clusters | Sums of weighted values for single sequences | Cluster names | Number of clones constituting clusters |
|---|---|---|---|
| 3NB691000018 | 0.018027129 | 76695 | 2 |
| 3NB691000085 | 0.019353319 | 56523 | 3 |
| 3NB691000113 | 0.024745432 | 48667 | 2 |
| 3NB691000116 | 0.103029787 | 73878 | 17 |
| 3NB691000129 | 0.066363755 | 74681 | 5 |
| 3NB691000173 | 0.212194846 | 38917 | 27 |
| 3NB691000191 | 0.02737028 | 77141 | 4 |
| 3NB692000012 | 0.012221951 | 168462 | 1 |
| 3NB692000029 | 0.012221951 | 271188 | 1 |
| 3NB692000102 | 0.012221951 | 110443 | 1 |
| 3NB692000154 | 0.012221951 | 201888 | 1 |
| 3NB692000276 | 0.012221951 | 268979 | 1 |
| 3NB692000281 | 0.761173312 | 35400 | 45 |
| 3NB692000305 | 0.012221951 | 265781 | 1 |
| 3NB692000330 | 0.379866613 | 53361 | 33 |
| 3NB692000374 | 0.154501611 | 57823 | 29 |
| 3NB692000429 | 0.012221951 | 249303 | 1 |
| 3NB692000484 | 0.016398886 | 123962 | 2 |
| 3NB692000529 | 0.015026689 | 223096 | 3 |
| 3NB692000545 | 0.059391668 | 134996 | 14 |
| 3NB692000912 | 0.356619986 | 48215 | 43 |
| 3NB692000973 | 0.023726783 | 201722 | 2 |
| 3NB692001002 | 0.085645793 | 67876 | 7 |
| 3NB692001022 | 0.134669151 | 41083 | 19 |
| 3NB692001034 | 0.012221951 | 221723 | 1 |
| 3NB692001040 | 0.012221951 | 115756 | 1 |
| 3NB692001123 | 0.012221951 | 266961 | 1 |
| 3NB692001267 | 0.012221951 | 265274 | 1 |
| 3NB692001288 | 0.1118352 | 117265 | 23 |
| 3NB692001334 | 0.624221602 | 49741 | 81 |
| 3NB692001339 | 0.071442272 | 47481 | 4 |
| 3NB692001349 | 0.012221951 | 175232 | 1 |
| 3NB692001366 | 0.013317229 | 3969 | 2 |
| 3NB692001408 | 0.012221951 | 126880 | 1 |
| 3NB692001433 | 0.048706104 | 130520 | 3 |
| 3NB692001442 | 0.138711778 | 127766 | 17 |
| 3NB692001459 | 0.029639045 | 200852 | 3 |
| 3NB692001471 | 0.012221951 | 75788 | 1 |
| 3NB692001496 | 0.021768101 | 84831 | 5 |
| 3NB692001501 | 0.012221951 | 268316 | 1 |
| 3NB692001507 | 0.012221951 | 268061 | 1 |
| 3NB692001511 | 0.019462755 | 167015 | 3 |
| 3NB692001519 | 0.012221951 | 271957 | 1 |
| 3NB692001520 | 0.013619106 | 104334 | 2 |
| 3NB692001528 | 0.071227801 | 139135 | 6 |
| 3NB692001538 | 0.084340651 | 122218 | 7 |
| 3NB692001548 | 0.012221951 | 96322 | 1 |
| 3NB692001557 | 0.027621147 | 91468 | 4 |
| 3NB692001637 | 0.036967383 | 168210 | 3 |
| 3NB692001719 | 0.024327187 | 94876 | 6 |
| 3NB692001853 | 0.063988222 | 34169 | 5 |
| 3NB692001861 | 0.012221951 | 193198 | 1 |
| 3NB692002051 | 0.019613515 | 178660 | 3 |
| 3NB692002365 | 0.012221951 | 4137 | 1 |
| 3NB692002685 | 0.012221951 | 217529 | 1 |
| 3NB692002806 | 0.012221951 | 154266 | 1 |
| 3NB692003538 | 0.012221951 | 208889 | 1 |
| 3NB692004045 | 0.012221951 | 284307 | 1 |
| 3NB692004724 | 0.012221951 | 196462 | 1 |
| 3NB692005439 | 0.012221951 | 185399 | 1 |
| 3NB692006952 | 0.028620836 | 68822 | 3 |
| 3NB692008178 | 0.013317229 | 46324 | 2 |
| 3NB692008729 | 0.012221951 | 135639 | 1 |
| ACTVT2000380 | 0.14619883 | 240854 | 1 |
| ADIPS1000064 | 0.16286645 | 41464 | 1 |
| ADIPS2000069 | 0.16286645 | 278077 | 1 |
| ADIPS2000088 | 0.325732899 | 273369 | 2 |
| ADIPS2000245 | 0.16286645 | 260321 | 1 |
| ADIPS2000425 | 0.215847182 | 124522 | 9 |
| ADRGL1000002 | 0.101756095 | 64755 | 44 |
| ADRGL1000018 | 0.034647887 | 46754 | 15 |
| ADRGL1000033 | 0.043883526 | 34724 | 5 |
| ADRGL1000038 | 0.009708738 | 66512 | 1 |
| ADRGL1000065 | 0.02971239 | 34887 | 11 |
| ADRGL1000067 | 0.009708738 | 8956 | 1 |
| ADRGL1000111 | 0.011105893 | 55238 | 2 |
| ADRGL1000144 | 0.05013397 | 52060 | 8 |
| ADRGL1000147 | 0.019417476 | 53719 | 2 |
| ADRGL1000160 | 0.077669903 | 10013 | 8 |
| ADRGL1000165 | 0.009708738 | 73605 | 1 |
| ADRGL1000182 | 0.009708738 | 65035 | 1 |
| ADRGL2000006 | 0.161178621 | 129178 | 41 |
| ADRGL2000042 | 0.065977455 | 94764 | 17 |
| ADRGL2000056 | 0.011105893 | 208582 | 2 |
| ADRGL2000064 | 0.009708738 | 184566 | 1 |
| ADRGL2000074 | 0.011397014 | 157554 | 2 |
| ADRGL2000085 | 0.009708738 | 184572 | 1 |
| ADRGL2000097 | 0.075366581 | 171199 | 10 |
| ADRGL2000117 | 0.019631485 | 114323 | 4 |
| ADRGL2000142 | 0.009708738 | 141891 | 1 |
| ADRGL2000172 | 0.009708738 | 182717 | 1 |
| ADRGL2000248 | 0.009708738 | 223982 | 1 |
| ADRGL2000261 | 0.009708738 | 207317 | 1 |
| ADRGL2000323 | 0.012623595 | 236322 | 2 |
| ADRGL2000328 | 0.024889644 | 175865 | 6 |
| ADRGL2000353 | 0.009708738 | 215172 | 1 |
| ADRGL2000384 | 0.067961165 | 12648 | 7 |
| ADRGL2000428 | 0.009708738 | 234148 | 1 |
| ADRGL2000636 | 0.009708738 | 75522 | 1 |
| ADRGL2000644 | 0.015763106 | 214709 | 2 |
| ADRGL2000968 | 0.009708738 | 196722 | 1 |
| ADRGL2001119 | 0.009708738 | 112417 | 1 |
| ADRGL2001229 | 0.009708738 | 215864 | 1 |
| ADRGL2001287 | 0.009708738 | 197023 | 1 |
| ADRGL2001301 | 0.009708738 | 241888 | 1 |
| ADRGL2001352 | 0.019587871 | 192434 | 4 |
| ADRGL2001354 | 0.009708738 | 223064 | 1 |
| ADRGL2001554 | 0.009708738 | 224065 | 1 |
| ADRGL2001651 | 0.009708738 | 222162 | 1 |
| ADRGL2001756 | 0.009708738 | 85903 | 1 |
| ADRGL2001830 | 0.014018789 | 65424 | 3 |
| ADRGL2001836 | 0.009708738 | 245756 | 1 |
| ADRGL2001854 | 0.009708738 | 243277 | 1 |
| ADRGL2002013 | 0.018625967 | 119719 | 7 |
| ADRGL2002029 | 0.009708738 | 78836 | 1 |
| ADRGL2002191 | 0.013106475 | 63260 | 3 |
| ADRGL2002260 | 0.011105893 | 188977 | 2 |
| ADRGL2002392 | 0.009708738 | 94613 | 1 |
| ADRGL2002477 | 0.098491284 | 97270 | 26 |
| ADRGL2002679 | 0.013465797 | 130441 | 3 |
| ADRGL2002753 | 0.019417476 | 231793 | 2 |
| ADRGL2002857 | 0.009708738 | 127516 | 1 |
| ADRGL2003017 | 0.009708738 | 46472 | 1 |
| ADRGL2003329 | 0.009708738 | 152935 | 1 |
| ADRGL2003552 | 0.009708738 | 283156 | 1 |
| ADRGL2003585 | 0.009708738 | 15181 | 1 |
| ADRGL2003638 | 0.009708738 | 134901 | 1 |
| ADRGL2003684 | 0.009708738 | 155321 | 1 |
| ADRGL2003773 | 0.009708738 | 223161 | 1 |
| ADRGL2003785 | 0.011418198 | 69967 | 2 |
| ADRGL2004031 | 0.009708738 | 164853 | 1 |
| ADRGL2004077 | 0.009708738 | 22835 | 1 |
| ADRGL2004451 | 0.019417476 | 72403 | 2 |
| ADRGL2004459 | 0.019417476 | 189693 | 2 |
| ADRGL2004676 | 0.038439333 | 112371 | 8 |
| ADRGL2004708 | 0.009708738 | 163374 | 1 |
| ADRGL2004724 | 0.009708738 | 174384 | 1 |
| ADRGL2004749 | 0.009708738 | 192682 | 1 |
| ADRGL2004770 | 0.009708738 | 200652 | 1 |
| ADRGL2004777 | 0.009708738 | 131595 | 1 |
| ADRGL2004833 | 0.009708738 | 80679 | 1 |
| ADRGL2005336 | 0.009708738 | 17560 | 1 |
| ADRGL2005564 | 0.009708738 | 24035 | 1 |
| ADRGL2005756 | 0.009708738 | 187567 | 1 |
| ADRGL2005838 | 0.015854264 | 5613 | 2 |
| ADRGL2005926 | 0.038834951 | 145927 | 4 |
| ADRGL2005961 | 0.009708738 | 3196 | 1 |
| ADRGL2006034 | 0.009708738 | 101275 | 1 |
| ADRGL2006124 | 0.009708738 | 32168 | 1 |
| ADRGL2006177 | 0.009708738 | 149214 | 1 |
| ADRGL2006193 | 0.009708738 | 146749 | 1 |
| ADRGL2006233 | 0.016534781 | 47690 | 3 |
| ADRGL2006377 | 0.009708738 | 77853 | 1 |
| ADRGL2006677 | 0.009708738 | 186902 | 1 |
| ADRGL2006732 | 0.009708738 | 101903 | 1 |
| ADRGL2006767 | 0.009708738 | 275340 | 1 |
| ADRGL2006817 | 0.009708738 | 213653 | 1 |
| ADRGL2006846 | 0.009708738 | 187510 | 1 |
| ADRGL2006886 | 0.009708738 | 176480 | 1 |
| ADRGL2007080 | 0.009708738 | 190702 | 1 |
| ADRGL2007125 | 0.009708738 | 45966 | 1 |
| ADRGL2007283 | 0.009708738 | 167805 | 1 |
| ADRGL2007313 | 0.017100302 | 168347 | 3 |
| ADRGL2007357 | 0.009708738 | 186500 | 1 |
| ADRGL2007636 | 0.009708738 | 155823 | 1 |
| ADRGL2007651 | 0.009708738 | 144684 | 1 |
| ADRGL2007810 | 0.009708738 | 99151 | 1 |
| ADRGL2007877 | 0.009708738 | 197625 | 1 |
| ADRGL2007906 | 0.018592193 | 81944 | 5 |
| ADRGL2007974 | 0.011397014 | 174372 | 2 |
| ADRGL2008023 | 0.009708738 | 122769 | 1 |
| ADRGL2008331 | 0.009708738 | 223423 | 1 |
| ADRGL2008337 | 0.009708738 | 261136 | 1 |
| ADRGL2008535 | 0.011105893 | 189976 | 2 |
| ADRGL2008966 | 0.011397014 | 148609 | 2 |
| ADRGL2009146 | 0.010897982 | 200721 | 2 |
| ADRGL2009273 | 0.120619855 | 138546 | 30 |
| ADRGL2009324 | 0.009708738 | 195085 | 1 |
| ADRGL2009489 | 0.009708738 | 149020 | 1 |
| ADRGL2009533 | 0.009708738 | 42576 | 1 |
| ADRGL2009691 | 0.009708738 | 89392 | 1 |
| ADRGL2009755 | 0.009708738 | 30025 | 1 |
| ADRGL2010152 | 0.02892579 | 15777 | 5 |
| ADRGL2010315 | 0.009708738 | 199715 | 1 |
| ADRGL2010594 | 0.009708738 | 242848 | 1 |
| ADRGL2010860 | 0.009708738 | 235358 | 1 |
| ADRGL2010974 | 0.009708738 | 202322 | 1 |
| ADRGL2011190 | 0.009708738 | 98377 | 1 |
| ADRGL2011330 | 0.011105893 | 203020 | 2 |
| ADRGL2012038 | 0.081507962 | 90763 | 5 |
| ADRGL2012179 | 0.01179025 | 104156 | 2 |
| AHMSC1000138 | 0.149031297 | 255416 | 1 |
| ASTRO1000009 | 0.064170068 | 77684 | 3 |
| ASTRO1000018 | 0.005805178 | 111744 | 1 |
| ASTRO1000029 | 0.005805178 | 31508 | 1 |
| ASTRO1000096 | 0.016632376 | 110634 | 2 |
| ASTRO1000165 | 0.075597651 | 69043 | 13 |
| ASTRO2000011 | 0.005805178 | 260808 | 1 |
| ASTRO2000014 | 0.408013258 | 131485 | 22 |
| ASTRO2000046 | 0.410629945 | 81809 | 138 |
| ASTRO2000095 | 0.005805178 | 228929 | 1 |
| ASTRO2000141 | 0.11032761 | 28048 | 10 |
| ASTRO2000191 | 0.005805178 | 205634 | 1 |
| ASTRO2000278 | 0.008599489 | 146123 | 3 |
| ASTRO2000372 | 0.007514638 | 33799 | 2 |
| ASTRO2000381 | 0.02668074 | 125948 | 6 |
| ASTRO2000403 | 0.005805178 | 235134 | 1 |
| ASTRO2000417 | 0.005805178 | 32092 | 1 |
| ASTRO2000480 | 0.059182171 | 97447 | 15 |
| ASTRO2000482 | 0.007480725 | 103504 | 2 |
| ASTRO2000533 | 0.005805178 | 109282 | 1 |
| ASTRO2000653 | 0.005805178 | 77531 | 1 |
| ASTRO2000662 | 0.02077397 | 164888 | 3 |
| ASTRO2000725 | 0.005805178 | 224690 | 1 |
| ASTRO2000801 | 0.164247184 | 18747 | 6 |
| ASTRO2000914 | 0.086079069 | 36093 | 9 |
| ASTRO2001001 | 0.005805178 | 137036 | 1 |
| ASTRO2001008 | 0.005805178 | 135634 | 1 |
| ASTRO2001029 | 0.133620252 | 162823 | 25 |
| ASTRO2001076 | 0.117885646 | 168112 | 17 |
| ASTRO2001088 | 0.005805178 | 168622 | 1 |
| ASTRO2001107 | 0.022938731 | 113427 | 11 |
| ASTRO2001122 | 0.005805178 | 139390 | 1 |
| ASTRO2001227 | 0.005805178 | 194451 | 1 |
| ASTRO2001249 | 0.017611554 | 142448 | 2 |
| ASTRO2001458 | 0.055081272 | 130929 | 13 |
| ASTRO2001480 | 0.005805178 | 273731 | 1 |
| ASTRO2001806 | 0.117118444 | 51065 | 21 |
| ASTRO2001823 | 0.115432411 | 148627 | 22 |
| ASTRO2002024 | 0.012780199 | 136960 | 6 |
| ASTRO2002035 | 0.015437208 | 14714 | 4 |
| ASTRO2002064 | 0.012007498 | 161710 | 2 |
| ASTRO2002202 | 0.072708957 | 107156 | 11 |
| ASTRO2002459 | 0.191410161 | 160202 | 7 |
| ASTRO2002571 | 0.005805178 | 153931 | 1 |
| ASTRO2002632 | 0.005805178 | 184466 | 1 |
| ASTRO2002659 | 0.011610356 | 128564 | 2 |
| ASTRO2002693 | 0.023638116 | 58270 | 3 |
| ASTRO2002720 | 0.006900456 | 45986 | 2 |
| ASTRO2002733 | 0.010891192 | 52101 | 4 |
| ASTRO2002743 | 0.005805178 | 152270 | 1 |
| ASTRO2002842 | 0.017592071 | 190999 | 2 |
| ASTRO2003024 | 0.176782467 | 101801 | 29 |
| ASTRO2003212 | 0.011185197 | 183619 | 4 |
| ASTRO2003316 | 0.056801159 | 119269 | 19 |
| ASTRO2003461 | 0.028128472 | 97123 | 10 |
| ASTRO2003574 | 0.011230867 | 93166 | 4 |
| ASTRO2003581 | 0.005805178 | 152157 | 1 |
| ASTRO2003632 | 0.403324214 | 58701 | 38 |
| ASTRO2003740 | 0.009649262 | 146059 | 2 |
| ASTRO2003840 | 0.027050092 | 133906 | 8 |
| ASTRO2003925 | 0.007803579 | 74962 | 2 |
| ASTRO2003960 | 0.006994423 | 206956 | 2 |
| ASTRO2004032 | 0.027492808 | 76671 | 8 |
| ASTRO2004051 | 0.005805178 | 115862 | 1 |
| ASTRO2004177 | 0.006994423 | 174394 | 2 |
| ASTRO2004217 | 0.005805178 | 200913 | 1 |
| ASTRO2004297 | 0.005805178 | 118848 | 1 |
| ASTRO2004584 | 0.005805178 | 576 | 1 |
| ASTRO2004628 | 0.018824826 | 147998 | 7 |
| ASTRO2004751 | 0.005805178 | 210722 | 1 |
| ASTRO2004877 | 0.005805178 | 128553 | 1 |
| ASTRO2004974 | 0.086235482 | 104154 | 13 |
| ASTRO2005008 | 0.005805178 | 104924 | 1 |
| ASTRO2005081 | 0.005805178 | 145748 | 1 |
| ASTRO2005096 | 0.005805178 | 198881 | 1 |
| ASTRO2005242 | 0.018149557 | 123226 | 5 |
| ASTRO2005273 | 0.005805178 | 168450 | 1 |
| ASTRO2005343 | 0.005805178 | 77687 | 1 |
| ASTRO2005360 | 0.028086918 | 149445 | 8 |
| ASTRO2005413 | 0.005805178 | 281753 | 1 |
| ASTRO2005423 | 0.048662157 | 122393 | 22 |
| ASTRO2005553 | 0.064661953 | 106837 | 7 |
| ASTRO2005557 | 0.005805178 | 20738 | 1 |
| ASTRO2005575 | 0.005805178 | 37609 | 1 |
| ASTRO2005593 | 0.012007498 | 90378 | 2 |
| ASTRO2005687 | 0.150323004 | 93161 | 20 |
| ASTRO2005726 | 0.005805178 | 168471 | 1 |
| ASTRO2005863 | 0.044075179 | 110378 | 14 |
| ASTRO2005896 | 0.018866829 | 189722 | 2 |
| ASTRO2005902 | 0.005805178 | 104669 | 1 |
| ASTRO2006356 | 0.005805178 | 187764 | 1 |
| ASTRO2006475 | 0.022496365 | 133653 | 5 |
| ASTRO2006732 | 0.010924386 | 144077 | 3 |
| ASTRO2006747 | 0.005805178 | 214900 | 1 |
| ASTRO2006920 | 0.027928558 | 206896 | 5 |
| ASTRO2006952 | 0.005805178 | 190210 | 1 |
| ASTRO2007047 | 0.080176463 | 113453 | 14 |
| ASTRO2007221 | 0.008925985 | 143785 | 2 |
| ASTRO2007377 | 0.005805178 | 165596 | 1 |
| ASTRO2007515 | 0.005805178 | 215382 | 1 |
| ASTRO2007578 | 0.005805178 | 165593 | 1 |
| ASTRO2007643 | 0.005805178 | 224456 | 1 |
| ASTRO2007666 | 0.005805178 | 217428 | 1 |
| ASTRO2007948 | 0.066903155 | 139726 | 18 |
| ASTRO2007956 | 0.005805178 | 74035 | 1 |
| ASTRO2007962 | 0.005805178 | 156422 | 1 |
| ASTRO2008040 | 0.013986539 | 149402 | 5 |
| ASTRO2008216 | 0.052509128 | 88347 | 10 |
| ASTRO2008239 | 0.005805178 | 217321 | 1 |
| ASTRO2008409 | 0.012888343 | 127528 | 3 |
| ASTRO2008425 | 0.005805178 | 150223 | 1 |
| ASTRO2008508 | 0.018630777 | 138166 | 3 |
| ASTRO2008777 | 0.005805178 | 141331 | 1 |
| ASTRO2008895 | 0.005805178 | 46303 | 1 |
| ASTRO2008960 | 0.005805178 | 177732 | 1 |
| ASTRO2008972 | 0.010758789 | 160000 | 3 |
| ASTRO2009068 | 0.02961118 | 128613 | 7 |
| ASTRO2009118 | 0.010726901 | 149916 | 3 |
| ASTRO2009177 | 0.005805178 | 190477 | 1 |
| ASTRO2009333 | 0.005805178 | 75248 | 1 |
| ASTRO2009458 | 0.005805178 | 159002 | 1 |
| ASTRO2009526 | 0.005805178 | 116040 | 1 |
| ASTRO2009879 | 0.005805178 | 280536 | 1 |
| ASTRO2010072 | 0.009649262 | 145536 | 2 |
| ASTRO2010582 | 0.009680421 | 81044 | 3 |
| ASTRO2010615 | 0.005805178 | 125076 | 1 |
| ASTRO2010799 | 0.011740953 | 63502 | 2 |
| ASTRO2010819 | 0.249018954 | 17947 | 96 |
| ASTRO2010962 | 0.005805178 | 206950 | 1 |
| ASTRO2010996 | 0.00885006 | 164323 | 2 |
| ASTRO2011149 | 0.005805178 | 161025 | 1 |
| ASTRO2011422 | 0.023096182 | 101511 | 8 |
| ASTRO2011426 | 0.005805178 | 241244 | 1 |
| ASTRO2011437 | 0.005805178 | 60299 | 1 |
| ASTRO2011461 | 0.005805178 | 253416 | 1 |
| ASTRO2011834 | 0.005805178 | 130341 | 1 |
| ASTRO2011893 | 0.005805178 | 190191 | 1 |
| ASTRO2012552 | 0.113357236 | 70566 | 17 |
| ASTRO2013050 | 0.065204083 | 112505 | 24 |
| ASTRO2013124 | 0.005805178 | 147155 | 1 |
| ASTRO2013585 | 0.343711979 | 31391 | 53 |
| ASTRO2013671 | 0.007202334 | 46333 | 2 |
| ASTRO2013802 | 0.005805178 | 168328 | 1 |
| ASTRO2014135 | 0.048237303 | 45846 | 3 |
| ASTRO2014174 | 0.007514638 | 135847 | 2 |
| ASTRO2014211 | 0.005805178 | 255616 | 1 |
| ASTRO2014363 | 0.005805178 | 75297 | 1 |
| ASTRO2014392 | 0.011337539 | 167372 | 3 |
| ASTRO2014498 | 0.009372912 | 129794 | 4 |
| ASTRO2014561 | 0.019012865 | 58366 | 3 |
| ASTRO2014576 | 0.014825091 | 115245 | 4 |
| ASTRO2014863 | 0.04959715 | 53993 | 9 |
| ASTRO2014923 | 0.005805178 | 166494 | 1 |
| ASTRO2014961 | 0.005805178 | 157854 | 1 |
| ASTRO2015162 | 0.005805178 | 20020 | 1 |
| ASTRO2015185 | 0.005805178 | 95607 | 1 |
| ASTRO2015214 | 0.057020452 | 185848 | 4 |
| ASTRO2015295 | 0.327013605 | 137956 | 20 |
| ASTRO2015328 | 0.044027039 | 113878 | 11 |
| ASTRO2015529 | 0.090436131 | 165708 | 13 |
| ASTRO2015987 | 0.063798417 | 36693 | 9 |
| ASTRO2016044 | 0.005805178 | 146106 | 1 |
| ASTRO2016114 | 0.011557963 | 18679 | 4 |
| ASTRO2016148 | 0.00788669 | 106373 | 2 |
| ASTRO2016313 | 0.050855316 | 164367 | 12 |
| ASTRO2016491 | 0.33272971 | 33255 | 78 |
| ASTRO2016681 | 0.005805178 | 236880 | 1 |
| ASTRO2016819 | 0.025966357 | 59150 | 6 |
| ASTRO2016847 | 0.005805178 | 156242 | 1 |
| ASTRO2016892 | 0.005805178 | 50889 | 1 |
| ASTRO2017348 | 0.005805178 | 241546 | 1 |
| ASTRO2017627 | 0.005805178 | 163656 | 1 |
| ASTRO2018169 | 0.177397925 | 117098 | 12 |
| ASTRO2018373 | 0.012007498 | 163438 | 2 |
| ASTRO2018540 | 0.107746655 | 28370 | 9 |
| ASTRO2019039 | 0.005805178 | 183074 | 1 |
| ASTRO2019171 | 0.009372912 | 130262 | 4 |
| ASTRO3000154 | 0.005805178 | 270258 | 1 |
| ASTRO3000172 | 0.005805178 | 257814 | 1 |
| ASTRO3000177 | 0.005805178 | 247423 | 1 |
| ASTRO3000301 | 0.005805178 | 266756 | 1 |
| ASTRO3000474 | 0.005805178 | 252434 | 1 |
| ASTRO3000482 | 0.007663293 | 260423 | 2 |
| BEAST2000454 | 0.036549708 | 29585 | 1 |
| BGGI11000123 | 0.239085915 | 62672 | 18 |
| BGGI11000193 | 0.0561349 | 78986 | 2 |
| BGGI11000285 | 0.052521008 | 35193 | 1 |
| BGGI12000022 | 0.052521008 | 263509 | 1 |
| BGGI12000054 | 0.711429859 | 113109 | 92 |
| BGGI12000067 | 0.052521008 | 73635 | 1 |
| BGGI12000161 | 0.052521008 | 23642 | 1 |
| BGGI12000533 | 0.074219504 | 8036 | 5 |
| BGGI12000544 | 0.052521008 | 120857 | 1 |
| BGGI12000616 | 0.187405138 | 102543 | 15 |
| BGGI12000684 | 0.067914618 | 51818 | 3 |
| BGGI12000693 | 0.088766025 | 145987 | 2 |
| BGGI12000839 | 0.227220843 | 27656 | 23 |
| BGGI12001002 | 0.094386136 | 131716 | 8 |
| BGGI12001075 | 0.08464501 | 17107 | 12 |
| BGGI12001097 | 0.149100363 | 142097 | 13 |
| BGGI12001241 | 0.052521008 | 184267 | 1 |
| BGGI12001247 | 0.052521008 | 150339 | 1 |
| BGGI12001682 | 0.162245467 | 139773 | 32 |
| BGGI12001714 | 0.065701387 | 38725 | 3 |
| BLADE1000176 | 0.014139044 | 51068 | 3 |
| BLADE2000181 | 0.011760555 | 185326 | 1 |
| BLADE2000256 | 0.014675412 | 12223 | 2 |
| BLADE2000340 | 0.011760555 | 130145 | 1 |
| BLADE2000389 | 0.011760555 | 108104 | 1 |
| BLADE2000463 | 0.011760555 | 56291 | 1 |
| BLADE2000492 | 0.047744722 | 136666 | 2 |
| BLADE2000579 | 0.011760555 | 121609 | 1 |
| BLADE2000640 | 0.011760555 | 280554 | 1 |
| BLADE2000866 | 0.011760555 | 234026 | 1 |
| BLADE2000938 | 0.011760555 | 192101 | 1 |
| BLADE2001031 | 0.011760555 | 257809 | 1 |
| BLADE2001133 | 0.011760555 | 245054 | 1 |
| BLADE2001138 | 0.011760555 | 177041 | 1 |
| BLADE2001141 | 0.011760555 | 76703 | 1 |
| BLADE2001371 | 0.013470015 | 52824 | 2 |
| BLADE2001458 | 0.011760555 | 177047 | 1 |
| BLADE2001575 | 0.017673167 | 183403 | 2 |
| BLADE2001753 | 0.011760555 | 205923 | 1 |
| BLADE2001935 | 0.011760555 | 248845 | 1 |
| BLADE2001940 | 0.011760555 | 238112 | 1 |
| BLADE2001987 | 0.011760555 | 188797 | 1 |
| BLADE2002073 | 0.011760555 | 153257 | 1 |
| BLADE2002310 | 0.011760555 | 238186 | 1 |
| BLADE2002730 | 0.012855833 | 211742 | 2 |
| BLADE2002738 | 0.013618669 | 202912 | 2 |
| BLADE2002744 | 0.068649476 | 194471 | 3 |
| BLADE2002782 | 0.011760555 | 273692 | 1 |
| BLADE2002947 | 0.011760555 | 276859 | 1 |
| BLADE2002972 | 0.011760555 | 50579 | 1 |
| BLADE2003474 | 0.011760555 | 182842 | 1 |
| BLADE2003916 | 0.011760555 | 276589 | 1 |
| BLADE2004089 | 0.016908153 | 73797 | 4 |
| BLADE2004345 | 0.011760555 | 278121 | 1 |
| BLADE2004389 | 0.011760555 | 209756 | 1 |
| BLADE2004462 | 0.011760555 | 155020 | 1 |
| BLADE2004628 | 0.011760555 | 188402 | 1 |
| BLADE2004670 | 0.011760555 | 218339 | 1 |
| BLADE2004722 | 0.016740916 | 114161 | 3 |
| BLADE2004849 | 0.022605909 | 85424 | 4 |
| BLADE2005036 | 0.011760555 | 170294 | 1 |
| BLADE2005038 | 0.011760555 | 278268 | 1 |
| BLADE2005229 | 0.011760555 | 168257 | 1 |
| BLADE2005459 | 0.011760555 | 172713 | 1 |
| BLADE2005792 | 0.011760555 | 192229 | 1 |
| BLADE2006043 | 0.011760555 | 161191 | 1 |
| BLADE2006412 | 0.027596903 | 160606 | 9 |
| BLADE2006415 | 0.011760555 | 184028 | 1 |
| BLADE2006607 | 0.011760555 | 241968 | 1 |
| BLADE2006826 | 0.011760555 | 233655 | 1 |
| BLADE2006830 | 0.099676546 | 87087 | 10 |
| BLADE2006849 | 0.011760555 | 277868 | 1 |
| BLADE2006947 | 0.02352111 | 148312 | 2 |
| BLADE2007033 | 0.011760555 | 273120 | 1 |
| BLADE2007043 | 0.013436102 | 149106 | 2 |
| BLADE2007589 | 0.033894588 | 45920 | 9 |
| BLADE2007666 | 0.049180905 | 212513 | 4 |
| BLADE2007722 | 0.011760555 | 212999 | 1 |
| BLADE2007735 | 0.011760555 | 231094 | 1 |
| BLADE2007744 | 0.011760555 | 235449 | 1 |
| BLADE2007799 | 0.011760555 | 270841 | 1 |
| BLADE2007923 | 0.011760555 | 160628 | 1 |
| BLADE2007935 | 0.013470015 | 226601 | 2 |
| BLADE2007958 | 0.011760555 | 149323 | 1 |
| BLADE2008281 | 0.011760555 | 197770 | 1 |
| BLADE2008398 | 0.027022345 | 110886 | 10 |
| BLADE2008454 | 0.011760555 | 279749 | 1 |
| BLADE2008461 | 0.067592263 | 20287 | 7 |
| BLADE2008479 | 0.011760555 | 210912 | 1 |
| BLADE2008539 | 0.011760555 | 162493 | 1 |
| BLADE2008809 | 0.011760555 | 192230 | 1 |
| BLADE2008995 | 0.011760555 | 141691 | 1 |
| BLADE2009452 | 0.011760555 | 198602 | 1 |
| BLADE2009479 | 0.011760555 | 211142 | 1 |
| BLADE2009593 | 0.011760555 | 265180 | 1 |
| BNGH41000098 | 0.066102669 | 52545 | 19 |
| BNGH41000104 | 0.022376043 | 48987 | 2 |
| BNGH41000118 | 0.012921566 | 29364 | 1 |
| BNGH41000137 | 0.038618962 | 76227 | 15 |
| BNGH41000153 | 0.035657499 | 54613 | 7 |
| BNGH41000169 | 0.012921566 | 66533 | 1 |
| BNGH41000177 | 0.024099746 | 8028 | 2 |
| BNGH41000190 | 0.066215467 | 66513 | 18 |
| BNGH41000198 | 0.080650037 | 72493 | 16 |
| BNGH41000216 | 0.012921566 | 87914 | 1 |
| BNGH41000235 | 0.025707724 | 10945 | 8 |
| BNGH42000130 | 0.15998039 | 123106 | 2 |
| BNGH42000132 | 0.012921566 | 194610 | 1 |
| BNGH42000360 | 1.065255609 | 56156 | 47 |
| BNGH42000405 | 0.012921566 | 203887 | 1 |
| BNGH42000532 | 0.01771792 | 97272 | 3 |
| BNGH42000675 | 0.012921566 | 35195 | 1 |
| BNGH42000739 | 0.269301405 | 83698 | 129 |
| BNGH42000791 | 0.012921566 | 261436 | 1 |
| BNGH42000815 | 0.019134676 | 87815 | 2 |
| BNGH42000994 | 0.012921566 | 135858 | 1 |
| BNGH42001247 | 0.03184986 | 78838 | 5 |
| BNGH42001406 | 0.024385512 | 260626 | 2 |
| BNGH42001576 | 0.034339999 | 189891 | 7 |
| BNGH42001724 | 0.012921566 | 39677 | 1 |
| BNGH42002168 | 0.125933709 | 68433 | 13 |
| BNGH42002244 | 0.012921566 | 146543 | 1 |
| BNGH42002387 | 0.015874959 | 99741 | 3 |
| BNGH42002487 | 0.017901927 | 123500 | 3 |
| BNGH42002489 | 0.012921566 | 245750 | 1 |
| BNGH42003529 | 0.012921566 | 151167 | 1 |
| BNGH42003570 | 0.012921566 | 239401 | 1 |
| BNGH42003641 | 0.012921566 | 236561 | 1 |
| BNGH42004076 | 0.012921566 | 223271 | 1 |
| BNGH42004291 | 0.012921566 | 92653 | 1 |
| BNGH42004538 | 0.012921566 | 171161 | 1 |
| BNGH42004679 | 0.014016844 | 35257 | 2 |
| BNGH42005017 | 0.019134676 | 159594 | 2 |
| BNGH42005235 | 0.012921566 | 165878 | 1 |
| BNGH42005504 | 0.012921566 | 34753 | 1 |
| BNGH42005968 | 0.072950516 | 131341 | 13 |
| BNGH42006135 | 0.253789888 | 84562 | 87 |
| BNGH42006226 | 0.014609843 | 134037 | 2 |
| BNGH42006234 | 0.012921566 | 139293 | 1 |
| BNGH42007037 | 0.02693841 | 109167 | 3 |
| BNGH42007366 | 0.026200916 | 125945 | 3 |
| BNGH42007460 | 0.166281879 | 99772 | 37 |
| BNGH42007594 | 0.012921566 | 250143 | 1 |
| BNGH42007709 | 0.727928246 | 66128 | 75 |
| BNGH42007788 | 0.054428793 | 120895 | 11 |
| BNGH42007798 | 0.017061726 | 100817 | 3 |
| BNGH42008199 | 0.012921566 | 226838 | 1 |
| BNGH42008510 | 0.012921566 | 161476 | 1 |
| BNGH42008603 | 0.11584507 | 133073 | 35 |
| BNGH42008649 | 0.012921566 | 278044 | 1 |
| BNGH42008743 | 0.10884126 | 126361 | 14 |
| BNGH42008832 | 0.012921566 | 33390 | 1 |
| BNGH42008850 | 0.015903525 | 160114 | 2 |
| BNGH42008943 | 0.020165179 | 29169 | 3 |
| BNGH42009021 | 0.012921566 | 129251 | 1 |
| BNGH42009025 | 0.120186589 | 150229 | 17 |
| BRACE1000020 | 0.014886212 | 54636 | 5 |
| BRACE1000042 | 0.110072803 | 53847 | 30 |
| BRACE1000047 | 0.001189244 | 64934 | 1 |
| BRACE1000051 | 0.009347382 | 75149 | 6 |
| BRACE1000065 | 0.004236603 | 52286 | 3 |
| BRACE1000070 | 0.093726205 | 39812 | 17 |
| BRACE1000073 | 0.0397401 | 49943 | 9 |
| BRACE1000084 | 0.001189244 | 52494 | 1 |
| BRACE1000087 | 0.01141852 | 73907 | 4 |
| BRACE1000092 | 0.001189244 | 66811 | 1 |
| BRACE1000093 | 0.004284741 | 36501 | 2 |
| BRACE1000101 | 0.014973455 | 73673 | 4 |
| BRACE1000114 | 0.001189244 | 69287 | 1 |
| BRACE1000115 | 0.895124541 | 5165 | 65 |
| BRACE1000124 | 0.001189244 | 64539 | 1 |
| BRACE1000131 | 0.16086513 | 62338 | 32 |
| BRACE1000132 | 0.011664658 | 76367 | 4 |
| BRACE1000159 | 0.090748029 | 52820 | 15 |
| BRACE1000166 | 0.0025864 | 87629 | 2 |
| BRACE1000169 | 0.008264884 | 41745 | 4 |
| BRACE1000186 | 0.077496146 | 73042 | 14 |
| BRACE1000187 | 0.062081235 | 57824 | 29 |
| BRACE1000239 | 0.008455944 | 143594 | 4 |
| BRACE1000258 | 0.00524328 | 88300 | 4 |
| BRACE1000451 | 0.001189244 | 251199 | 1 |
| BRACE1000475 | 0.001189244 | 258814 | 1 |
| BRACE1000533 | 0.071027019 | 89830 | 30 |
| BRACE1000572 | 0.001189244 | 278488 | 1 |
| BRACE2000061 | 0.004274676 | 172422 | 3 |
| BRACE2000077 | 0.003473767 | 158305 | 3 |
| BRACE2000078 | 0.037434261 | 199344 | 2 |
| BRACE2000100 | 0.001189244 | 215188 | 1 |
| BRACE2000109 | 0.003047359 | 166740 | 2 |
| BRACE2000141 | 0.009647536 | 85923 | 6 |
| BRACE2000148 | 0.046934018 | 190688 | 3 |
| BRACE2000183 | 0.036549923 | 167435 | 2 |
| BRACE2000229 | 0.012976137 | 130666 | 2 |
| BRACE2000232 | 0.001189244 | 204443 | 1 |
| BRACE2000245 | 0.018945752 | 97358 | 9 |
| BRACE2000256 | 0.001189244 | 207459 | 1 |
| BRACE2000266 | 0.001189244 | 251079 | 1 |
| BRACE2000267 | 0.012536959 | 102199 | 5 |
| BRACE2000280 | 0.008940271 | 112431 | 3 |
| BRACE2000300 | 0.031521611 | 95671 | 11 |
| BRACE2000307 | 0.142252985 | 132485 | 49 |
| BRACE2000331 | 0.002864792 | 18972 | 2 |
| BRACE2000332 | 0.061728861 | 126335 | 12 |
| BRACE2000347 | 0.064814986 | 182381 | 17 |
| BRACE2000351 | 0.001189244 | 281897 | 1 |
| BRACE2000368 | 0.002378489 | 206430 | 2 |
| BRACE2000392 | 0.001189244 | 214545 | 1 |
| BRACE2000421 | 0.043236157 | 82742 | 25 |
| BRACE2000441 | 0.001189244 | 146945 | 1 |
| BRACE2000487 | 0.020419338 | 165327 | 4 |
| BRACE2000489 | 0.001189244 | 64699 | 1 |
| BRACE2000505 | 0.013712726 | 184139 | 2 |
| BRACE2000525 | 0.103126365 | 93675 | 35 |
| BRACE2000526 | 0.001189244 | 216121 | 1 |
| BRACE2000531 | 0.001189244 | 142381 | 1 |
| BRACE2000545 | 0.001189244 | 106138 | 1 |
| BRACE2000553 | 0.001189244 | 91501 | 1 |
| BRACE2000565 | 0.023362848 | 153227 | 8 |
| BRACE2000577 | 0.002284523 | 71681 | 2 |
| BRACE2000640 | 0.001189244 | 270201 | 1 |
| BRACE2000650 | 0.001189244 | 238334 | 1 |
| BRACE2000676 | 0.155624815 | 48302 | 44 |
| BRACE2000677 | 0.001189244 | 266766 | 1 |
| BRACE2000698 | 0.019473747 | 144048 | 3 |
| BRACE2000718 | 0.001189244 | 208548 | 1 |
| BRACE2000733 | 0.009507114 | 176333 | 3 |
| BRACE2000743 | 0.001189244 | 249263 | 1 |
| BRACE2000750 | 0.008141256 | 57101 | 4 |
| BRACE2000753 | 0.005501257 | 154521 | 3 |
| BRACE2000815 | 0.001189244 | 233427 | 1 |
| BRACE2000864 | 0.31749202 | 137308 | 41 |
| BRACE2000885 | 0.002284523 | 127717 | 2 |
| BRACE2000893 | 0.037076472 | 150848 | 17 |
| BRACE2000905 | 0.001189244 | 227967 | 1 |
| BRACE2000936 | 0.001189244 | 273068 | 1 |
| BRACE2000947 | 0.001189244 | 237436 | 1 |
| BRACE2000965 | 0.001189244 | 158015 | 1 |
| BRACE2000969 | 0.007334771 | 149537 | 2 |
| BRACE2000975 | 0.261569543 | 75529 | 133 |
| BRACE2000988 | 0.001189244 | 279611 | 1 |
| BRACE2001017 | 0.001189244 | 247496 | 1 |
| BRACE2001065 | 0.025233658 | 88814 | 7 |
| BRACE2001070 | 0.007425602 | 100127 | 2 |
| BRACE2001094 | 0.001189244 | 281360 | 1 |
| BRACE2001107 | 0.011383773 | 230024 | 4 |
| BRACE2001117 | 0.060920807 | 55893 | 10 |
| BRACE2001143 | 0.112794231 | 98802 | 15 |
| BRACE2001151 | 0.025957605 | 49097 | 5 |
| BRACE2001154 | 0.015770393 | 72571 | 6 |
| BRACE2001188 | 0.059357034 | 94051 | 6 |
| BRACE2001191 | 0.001189244 | 273710 | 1 |
| BRACE2001197 | 0.002898705 | 44154 | 2 |
| BRACE2001236 | 0.009312322 | 181678 | 3 |
| BRACE2001239 | 0.001189244 | 227737 | 1 |
| BRACE2001269 | 0.001189244 | 230267 | 1 |
| BRACE2001312 | 0.002378489 | 212099 | 2 |
| BRACE2001313 | 0.005501257 | 166260 | 3 |
| BRACE2001330 | 0.001189244 | 41898 | 1 |
| BRACE2001340 | 0.001189244 | 134905 | 1 |
| BRACE2001352 | 0.001189244 | 185672 | 1 |
| BRACE2001353 | 0.001189244 | 197676 | 1 |
| BRACE2001374 | 0.002877521 | 109292 | 2 |
| BRACE2001375 | 0.001189244 | 192284 | 1 |
| BRACE2001423 | 0.001189244 | 189404 | 1 |
| BRACE2001424 | 0.001189244 | 29307 | 1 |
| BRACE2001445 | 0.001189244 | 20354 | 1 |
| BRACE2001453 | 0.001189244 | 220137 | 1 |
| BRACE2001455 | 0.001189244 | 30719 | 1 |
| BRACE2001477 | 0.048719392 | 115129 | 20 |
| BRACE2001478 | 0.094622909 | 106856 | 32 |
| BRACE2001492 | 0.001189244 | 95633 | 1 |
| BRACE2001496 | 0.034537152 | 11508 | 8 |
| BRACE2001497 | 0.089919331 | 130162 | 27 |
| BRACE2001508 | 0.01901123 | 220066 | 4 |
| BRACE2001534 | 0.054321394 | 173923 | 6 |
| BRACE2001543 | 0.001189244 | 244558 | 1 |
| BRACE2001564 | 0.003473767 | 111429 | 3 |
| BRACE2001588 | 0.001189244 | 232704 | 1 |
| BRACE2001607 | 0.001189244 | 200443 | 1 |
| BRACE2001626 | 0.001189244 | 245180 | 1 |
| BRACE2001673 | 0.714683906 | 90925 | 23 |
| BRACE2001677 | 0.001189244 | 235166 | 1 |
| BRACE2001692 | 0.276719989 | 87056 | 17 |
| BRACE2001705 | 0.001189244 | 247141 | 1 |
| BRACE2001709 | 0.05162969 | 96776 | 14 |
| BRACE2001711 | 0.043073912 | 41456 | 7 |
| BRACE2001726 | 0.004735635 | 64631 | 3 |
| BRACE2001733 | 0.001189244 | 247168 | 1 |
| BRACE2001737 | 0.001189244 | 229602 | 1 |
| BRACE2001742 | 0.002378489 | 223664 | 2 |
| BRACE2001777 | 0.001189244 | 243124 | 1 |
| BRACE2001779 | 0.001189244 | 239647 | 1 |
| BRACE2001785 | 0.162822998 | 105745 | 26 |
| BRACE2001834 | 0.044147361 | 108730 | 17 |
| BRACE2001855 | 0.145620135 | 74488 | 15 |
| BRACE2001859 | 0.012313952 | 69588 | 2 |
| BRACE2001865 | 0.001189244 | 159266 | 1 |
| BRACE2001872 | 0.001189244 | 235994 | 1 |
| BRACE2001881 | 0.007261627 | 174173 | 2 |
| BRACE2001892 | 0.553136526 | 79977 | 240 |
| BRACE2001898 | 0.001189244 | 245875 | 1 |
| BRACE2001923 | 0.001189244 | 90201 | 1 |
| BRACE2001942 | 0.0025864 | 165068 | 2 |
| BRACE2001944 | 0.026710106 | 152439 | 8 |
| BRACE2001954 | 0.001189244 | 155522 | 1 |
| BRACE2001971 | 0.074703155 | 15916 | 17 |
| BRACE2001972 | 0.001189244 | 170824 | 1 |
| BRACE2001973 | 0.005946222 | 208320 | 5 |
| BRACE2002026 | 0.001189244 | 221386 | 1 |
| BRACE2002034 | 0.001189244 | 220242 | 1 |
| BRACE2002050 | 0.057781435 | 128655 | 26 |
| BRACE2002081 | 0.008731926 | 180908 | 3 |
| BRACE2002091 | 0.079344087 | 128918 | 19 |
| BRACE2002139 | 0.001189244 | 114525 | 1 |
| BRACE2002142 | 0.001189244 | 150126 | 1 |
| BRACE2002151 | 0.001189244 | 155744 | 1 |
| BRACE2002157 | 0.001189244 | 143410 | 1 |
| BRACE2002176 | 0.003187646 | 180777 | 2 |
| BRACE2002202 | 0.004663012 | 181084 | 4 |
| BRACE2002206 | 0.001189244 | 114072 | 1 |
| BRACE2002227 | 0.001189244 | 121343 | 1 |
| BRACE2002260 | 0.001189244 | 180781 | 1 |
| BRACE2002297 | 0.001189244 | 208054 | 1 |
| BRACE2002304 | 0.001189244 | 154933 | 1 |
| BRACE2002356 | 0.00922151 | 42663 | 5 |
| BRACE2002392 | 0.097211361 | 103454 | 25 |
| BRACE2002394 | 0.001189244 | 285151 | 1 |
| BRACE2002409 | 0.003187646 | 130594 | 2 |
| BRACE2002431 | 0.001189244 | 54577 | 1 |
| BRACE2002441 | 0.148775007 | 111346 | 21 |
| BRACE2002444 | 0.001189244 | 41749 | 1 |
| BRACE2002458 | 0.001189244 | 175336 | 1 |
| BRACE2002467 | 0.025633146 | 155379 | 3 |
| BRACE2002468 | 0.001189244 | 99366 | 1 |
| BRACE2002478 | 0.011560335 | 175363 | 7 |
| BRACE2002495 | 0.00723081 | 168524 | 2 |
| BRACE2002582 | 0.025645851 | 101505 | 9 |
| BRACE2002585 | 0.006721605 | 110818 | 3 |
| BRACE2002589 | 0.001189244 | 272237 | 1 |
| BRACE2002590 | 0.001189244 | 140923 | 1 |
| BRACE2002606 | 0.031262135 | 114772 | 5 |
| BRACE2002613 | 0.001189244 | 108603 | 1 |
| BRACE2002614 | 0.001189244 | 107029 | 1 |
| BRACE2002617 | 0.001189244 | 265212 | 1 |
| BRACE2002623 | 0.001189244 | 114765 | 1 |
| BRACE2002635 | 0.002877521 | 140061 | 2 |
| BRACE2002649 | 0.015825326 | 131524 | 9 |
| BRACE2002661 | 0.058181693 | 124597 | 9 |
| BRACE2002676 | 0.001189244 | 49196 | 1 |
| BRACE2002677 | 0.086792471 | 103900 | 15 |
| BRACE2002682 | 0.008751187 | 132202 | 4 |
| BRACE2002685 | 0.048883651 | 201820 | 4 |
| BRACE2002695 | 0.002378489 | 139887 | 2 |
| BRACE2002707 | 0.001189244 | 111285 | 1 |
| BRACE2002736 | 0.001189244 | 128665 | 1 |
| BRACE2002752 | 0.010897982 | 242273 | 2 |
| BRACE2002755 | 0.003066086 | 171219 | 2 |
| BRACE2002762 | 0.001189244 | 238001 | 1 |
| BRACE2002772 | 0.001189244 | 173638 | 1 |
| BRACE2002792 | 0.001189244 | 145752 | 1 |
| BRACE2002796 | 0.003270756 | 132488 | 2 |
| BRACE2002803 | 0.009195636 | 88035 | 6 |
| BRACE2002807 | 0.00519938 | 123075 | 3 |
| BRACE2002812 | 0.028512311 | 60834 | 13 |
| BRACE2002834 | 0.002378489 | 175252 | 2 |
| BRACE2002843 | 0.22660084 | 33605 | 58 |
| BRACE2002849 | 0.001189244 | 264019 | 1 |
| BRACE2002857 | 0.001189244 | 160288 | 1 |
| BRACE2002860 | 0.066552329 | 5635 | 16 |
| BRACE2002861 | 0.064566514 | 117326 | 12 |
| BRACE2002884 | 0.001189244 | 111155 | 1 |
| BRACE2002896 | 0.011179055 | 23341 | 5 |
| BRACE2002948 | 0.001189244 | 245402 | 1 |
| BRACE2003037 | 0.077599497 | 71466 | 25 |
| BRACE2003078 | 0.010351617 | 105424 | 3 |
| BRACE2003097 | 0.002898705 | 164178 | 2 |
| BRACE2003110 | 0.012438635 | 186504 | 4 |
| BRACE2003168 | 0.001189244 | 259248 | 1 |
| BRACE2003172 | 0.515557623 | 94268 | 154 |
| BRACE2003199 | 0.001189244 | 106524 | 1 |
| BRACE2003210 | 0.002378489 | 169936 | 2 |
| BRACE2003217 | 0.038871874 | 68900 | 9 |
| BRACE2003254 | 0.185073124 | 86751 | 35 |
| BRACE2003269 | 0.001189244 | 117238 | 1 |
| BRACE2003285 | 0.001189244 | 68395 | 1 |
| BRACE2003304 | 0.076642407 | 8482 | 16 |
| BRACE2003319 | 0.073132035 | 10304 | 4 |
| BRACE2003398 | 0.007243613 | 267224 | 2 |
| BRACE2003420 | 0.001189244 | 198564 | 1 |
| BRACE2003428 | 0.001189244 | 15191 | 1 |
| BRACE2003431 | 0.001189244 | 171176 | 1 |
| BRACE2003449 | 0.067608748 | 117850 | 22 |
| BRACE2003512 | 0.005186047 | 62441 | 3 |
| BRACE2003516 | 0.003187646 | 89609 | 2 |
| BRACE2003527 | 0.065002078 | 47270 | 13 |
| BRACE2003539 | 0.003047359 | 100355 | 2 |
| BRACE2003540 | 0.001189244 | 104535 | 1 |
| BRACE2003594 | 0.014349722 | 226521 | 3 |
| BRACE2003598 | 0.006094718 | 115732 | 4 |
| BRACE2003609 | 0.018433522 | 97813 | 7 |
| BRACE2003614 | 0.001189244 | 113504 | 1 |
| BRACE2003628 | 0.024560067 | 154466 | 7 |
| BRACE2003639 | 0.001189244 | 165240 | 1 |
| BRACE2003700 | 0.012200138 | 113523 | 8 |
| BRACE2003713 | 0.007243613 | 253583 | 2 |
| BRACE2003766 | 0.001189244 | 185696 | 1 |
| BRACE2003785 | 0.17242849 | 108323 | 45 |
| BRACE2003788 | 0.00429586 | 175370 | 3 |
| BRACE2003800 | 0.006113445 | 136299 | 4 |
| BRACE2003802 | 0.002378489 | 165262 | 2 |
| BRACE2003825 | 0.012313952 | 118486 | 2 |
| BRACE2003827 | 0.191244224 | 63595 | 42 |
| BRACE2003845 | 0.036775553 | 112087 | 12 |
| BRACE2003846 | 0.257097629 | 111157 | 31 |
| BRACE2003847 | 0.001189244 | 283219 | 1 |
| BRACE2003848 | 0.003066086 | 39370 | 2 |
| BRACE2003873 | 0.001189244 | 199346 | 1 |
| BRACE2003885 | 0.001189244 | 107600 | 1 |
| BRACE2003887 | 0.001189244 | 257294 | 1 |
| BRACE2003892 | 0.076773409 | 62724 | 27 |
| BRACE2003904 | 0.06463287 | 64974 | 14 |
| BRACE2003905 | 0.010595549 | 157872 | 7 |
| BRACE2003928 | 0.001189244 | 124346 | 1 |
| BRACE2003944 | 0.323677952 | 108195 | 40 |
| BRACE2003954 | 0.007101856 | 55642 | 2 |
| BRACE2003982 | 0.040716444 | 89008 | 10 |
| BRACE2003987 | 0.001189244 | 43206 | 1 |
| BRACE2005034 | 0.001189244 | 270218 | 1 |
| BRACE2005047 | 0.001189244 | 228652 | 1 |
| BRACE2005063 | 0.001189244 | 191153 | 1 |
| BRACE2005083 | 0.00696547 | 65336 | 5 |
| BRACE2005087 | 0.161528991 | 34376 | 69 |
| BRACE2005090 | 0.002284523 | 165711 | 2 |
| BRACE2005099 | 0.126048051 | 118147 | 40 |
| BRACE2005118 | 0.004756978 | 92217 | 4 |
| BRACE2005124 | 0.001189244 | 151453 | 1 |
| BRACE2005138 | 0.001189244 | 278805 | 1 |
| BRACE2005151 | 0.079742189 | 90872 | 13 |
| BRACE2005160 | 0.001189244 | 194376 | 1 |
| BRACE2005169 | 0.001189244 | 192616 | 1 |
| BRACE2005189 | 0.006612615 | 159288 | 4 |
| BRACE2005193 | 0.002898705 | 110026 | 2 |
| BRACE2005196 | 0.005269158 | 180812 | 3 |
| BRACE2005198 | 0.001189244 | 179517 | 1 |
| BRACE2005216 | 0.005946222 | 149522 | 5 |
| BRACE2005231 | 0.052483553 | 53758 | 20 |
| BRACE2005243 | 0.001189244 | 94059 | 1 |
| BRACE2005251 | 0.01585274 | 129793 | 10 |
| BRACE2005253 | 0.003047359 | 192462 | 2 |
| BRACE2005290 | 0.006585383 | 133765 | 4 |
| BRACE2005298 | 0.005501257 | 238111 | 3 |
| BRACE2005328 | 0.001189244 | 157538 | 1 |
| BRACE2005329 | 0.001189244 | 250935 | 1 |
| BRACE2005337 | 0.001189244 | 263896 | 1 |
| BRACE2005348 | 0.001189244 | 14925 | 1 |
| BRACE2005363 | 0.01179145 | 69313 | 2 |
| BRACE2005395 | 0.017771209 | 136896 | 9 |
| BRACE2005408 | 0.017616261 | 126124 | 7 |
| BRACE2005433 | 0.014508876 | 33267 | 4 |
| BRACE2005434 | 0.243038258 | 159387 | 22 |
| BRACE2005448 | 0.02464078 | 114059 | 6 |
| BRACE2005450 | 0.001189244 | 196801 | 1 |
| BRACE2005453 | 0.012952566 | 179221 | 2 |
| BRACE2005457 | 0.002864792 | 148694 | 2 |
| BRACE2005460 | 0.001189244 | 191165 | 1 |
| BRACE2005489 | 0.001189244 | 251214 | 1 |
| BRACE2005518 | 0.001189244 | 64745 | 1 |
| BRACE2005556 | 0.001189244 | 145382 | 1 |
| BRACE2005562 | 0.001189244 | 179357 | 1 |
| BRACE2005624 | 0.001189244 | 168474 | 1 |
| BRACE2005642 | 0.022931482 | 74158 | 9 |
| BRACE2005661 | 0.001189244 | 171453 | 1 |
| BRACE2005667 | 0.001189244 | 148123 | 1 |
| BRACE2005681 | 0.167842894 | 40549 | 87 |
| BRACE2005719 | 0.056417443 | 115048 | 16 |
| BRACE2005731 | 0.002877521 | 169088 | 2 |
| BRACE2005742 | 0.001189244 | 139089 | 1 |
| BRACE2005762 | 0.001189244 | 147495 | 1 |
| BRACE2005773 | 0.001189244 | 168467 | 1 |
| BRACE2005787 | 0.001189244 | 187705 | 1 |
| BRACE2005795 | 0.001189244 | 252383 | 1 |
| BRACE2005800 | 0.001189244 | 150044 | 1 |
| BRACE2005858 | 0.016215826 | 84377 | 10 |
| BRACE2005869 | 0.001189244 | 216537 | 1 |
| BRACE2005875 | 0.03892138 | 153328 | 3 |
| BRACE2005881 | 0.001189244 | 165965 | 1 |
| BRACE2005898 | 0.002378489 | 165487 | 2 |
| BRACE2005904 | 0.001189244 | 161930 | 1 |
| BRACE2005906 | 0.001189244 | 142179 | 1 |
| BRACE2005907 | 0.001189244 | 171454 | 1 |
| BRACE2005911 | 0.001189244 | 160120 | 1 |
| BRACE2005937 | 0.027739619 | 175663 | 8 |
| BRACE2005949 | 0.001189244 | 144908 | 1 |
| BRACE2005981 | 0.001189244 | 111483 | 1 |
| BRACE2005991 | 0.001189244 | 155307 | 1 |
| BRACE2006055 | 0.083760892 | 155424 | 18 |
| BRACE2006072 | 0.008500825 | 213122 | 4 |
| BRACE2006075 | 0.010326307 | 217884 | 3 |
| BRACE2006084 | 0.054589823 | 184305 | 7 |
| BRACE2006089 | 0.041095851 | 30416 | 24 |
| BRACE2006102 | 0.003983555 | 209616 | 3 |
| BRACE2006105 | 0.007897763 | 77099 | 5 |
| BRACE2006122 | 0.001189244 | 234826 | 1 |
| BRACE2006137 | 0.002877521 | 204780 | 2 |
| BRACE2006143 | 0.001189244 | 244251 | 1 |
| BRACE2006162 | 0.108285945 | 160645 | 9 |
| BRACE2006167 | 0.079483894 | 34093 | 31 |
| BRACE2006174 | 0.001189244 | 262976 | 1 |
| BRACE2006240 | 0.001189244 | 271255 | 1 |
| BRACE2006245 | 0.001189244 | 252264 | 1 |
| BRACE2006249 | 0.0025864 | 203093 | 2 |
| BRACE2006258 | 0.204556781 | 67510 | 34 |
| BRACE2006264 | 0.019852288 | 188999 | 7 |
| BRACE2006274 | 0.005755042 | 99934 | 4 |
| BRACE2006281 | 0.055446818 | 70046 | 3 |
| BRACE2006298 | 0.001189244 | 255370 | 1 |
| BRACE2006319 | 0.047792784 | 49721 | 19 |
| BRACE2006344 | 0.046874944 | 145391 | 5 |
| BRACE2006354 | 0.026926489 | 99305 | 10 |
| BRACE2006363 | 0.018078792 | 126430 | 8 |
| BRACE2006378 | 0.003066086 | 201430 | 2 |
| BRACE2006380 | 0.004087949 | 159761 | 3 |
| BRACE2006393 | 0.001189244 | 257566 | 1 |
| BRACE2006397 | 0.567652044 | 94125 | 50 |
| BRACE2006413 | 0.001189244 | 250113 | 1 |
| BRACE2006417 | 0.011892445 | 90652 | 10 |
| BRACE2006453 | 0.001189244 | 171125 | 1 |
| BRACE2006459 | 0.004104101 | 251803 | 2 |
| BRACE2006488 | 0.003567733 | 1822 | 3 |
| BRACE2006514 | 0.192867899 | 11048 | 61 |
| BRACE2006534 | 0.001189244 | 99015 | 1 |
| BRACE2006537 | 0.155686962 | 95917 | 33 |
| BRACE2006540 | 0.001189244 | 255246 | 1 |
| BRACE2006547 | 0.011198766 | 152427 | 5 |
| BRACE2006564 | 0.003983555 | 160946 | 3 |
| BRACE2006587 | 0.001189244 | 222032 | 1 |
| BRACE2006598 | 0.005792378 | 110900 | 3 |
| BRACE2006636 | 0.01222174 | 118499 | 7 |
| BRACE2006685 | 0.001189244 | 261600 | 1 |
| BRACE2006703 | 0.001189244 | 262684 | 1 |
| BRACE2006743 | 0.037684663 | 103447 | 15 |
| BRACE2006753 | 0.027249027 | 128172 | 6 |
| BRACE2006783 | 0.001189244 | 154308 | 1 |
| BRACE2006821 | 0.090743342 | 104649 | 22 |
| BRACE2006833 | 0.004460001 | 171316 | 3 |
| BRACE2006859 | 0.016491365 | 153508 | 4 |
| BRACE2006871 | 0.167574939 | 116102 | 9 |
| BRACE2006900 | 0.325644347 | 29047 | 36 |
| BRACE2006909 | 0.001189244 | 230630 | 1 |
| BRACE2006911 | 0.009023047 | 155213 | 3 |
| BRACE2006924 | 0.001189244 | 236896 | 1 |
| BRACE2006935 | 0.007243613 | 155118 | 2 |
| BRACE2006937 | 0.125196352 | 121760 | 31 |
| BRACE2006944 | 0.014365826 | 42390 | 3 |
| BRACE2006951 | 0.001189244 | 153398 | 1 |
| BRACE2006982 | 0.12916629 | 6982 | 26 |
| BRACE2006986 | 0.001189244 | 234274 | 1 |
| BRACE2007013 | 0.016341915 | 78490 | 8 |
| BRACE2007064 | 0.103117687 | 50384 | 38 |
| BRACE2007068 | 0.006690501 | 172630 | 4 |
| BRACE2007087 | 0.001189244 | 246196 | 1 |
| BRACE2007089 | 0.197885429 | 69728 | 14 |
| BRACE2007109 | 0.17553833 | 108689 | 65 |
| BRACE2007138 | 0.001189244 | 230550 | 1 |
| BRACE2007153 | 0.224251358 | 125514 | 49 |
| BRACE2007174 | 0.010725179 | 118448 | 5 |
| BRACE2007191 | 0.243938443 | 131102 | 38 |
| BRACE2007197 | 0.001189244 | 285656 | 1 |
| BRACE2007201 | 0.002898705 | 247830 | 2 |
| BRACE2007203 | 0.001189244 | 234245 | 1 |
| BRACE2007232 | 0.001189244 | 180360 | 1 |
| BRACE2007254 | 0.022508864 | 153140 | 6 |
| BRACE2007262 | 0.001189244 | 126993 | 1 |
| BRACE2007274 | 0.001189244 | 241721 | 1 |
| BRACE2007281 | 0.001189244 | 99385 | 1 |
| BRACE2007295 | 0.017818629 | 175248 | 5 |
| BRACE2007358 | 0.001189244 | 242533 | 1 |
| BRACE2007396 | 0.001189244 | 248147 | 1 |
| BRACE2007398 | 0.001189244 | 52678 | 1 |
| BRACE2007401 | 0.091683993 | 88738 | 26 |
| BRACE2007411 | 0.003567733 | 176323 | 3 |
| BRACE2007447 | 0.001189244 | 247769 | 1 |
| BRACE2007477 | 0.001189244 | 242604 | 1 |
| BRACE2007502 | 0.002378489 | 134480 | 2 |
| BRACE2007503 | 0.001189244 | 217246 | 1 |
| BRACE2007518 | 0.073827438 | 102551 | 32 |
| BRACE2007522 | 0.002898705 | 154500 | 2 |
| BRACE2007527 | 0.001189244 | 113455 | 1 |
| BRACE2007538 | 0.001189244 | 40271 | 1 |
| BRACE2007563 | 0.001189244 | 243885 | 1 |
| BRACE2007567 | 0.120753346 | 124417 | 16 |
| BRACE2007577 | 0.171121036 | 72310 | 13 |
| BRACE2007621 | 0.001189244 | 244840 | 1 |
| BRACE2007640 | 0.003567733 | 88485 | 3 |
| BRACE2007641 | 0.106885695 | 21293 | 46 |
| BRACE2007646 | 0.002877521 | 110136 | 2 |
| BRACE2007663 | 0.005277194 | 149782 | 4 |
| BRACE2007671 | 0.108374936 | 90530 | 18 |
| BRACE2007682 | 0.001189244 | 230535 | 1 |
| BRACE2007685 | 0.001189244 | 64181 | 1 |
| BRACE2007708 | 0.078848286 | 154775 | 15 |
| BRACE2007727 | 0.001189244 | 244060 | 1 |
| BRACE2007749 | 0.001189244 | 128479 | 1 |
| BRACE2007760 | 0.012784712 | 74217 | 10 |
| BRACE2007761 | 0.001189244 | 267719 | 1 |
| BRACE2007764 | 0.002877521 | 4420 | 2 |
| BRACE2007767 | 0.05381986 | 144915 | 5 |
| BRACE2007768 | 0.017511022 | 146332 | 6 |
| BRACE2007769 | 0.001189244 | 221669 | 1 |
| BRACE2007784 | 0.001189244 | 86887 | 1 |
| BRACE2007786 | 0.001189244 | 180262 | 1 |
| BRACE2007798 | 0.161988061 | 32099 | 36 |
| BRACE2007799 | 0.001189244 | 173702 | 1 |
| BRACE2007801 | 0.002284523 | 102202 | 2 |
| BRACE2007832 | 0.013350897 | 175572 | 7 |
| BRACE2007836 | 0.001189244 | 217903 | 1 |
| BRACE2007868 | 0.001189244 | 39294 | 1 |
| BRACE2007882 | 0.025441469 | 161035 | 14 |
| BRACE2007888 | 0.001189244 | 133348 | 1 |
| BRACE2007902 | 0.005227527 | 168485 | 2 |
| BRACE2007920 | 0.001189244 | 191167 | 1 |
| BRACE2007937 | 0.065970832 | 84509 | 29 |
| BRACE2007944 | 0.001189244 | 218029 | 1 |
| BRACE2007952 | 0.069106987 | 108548 | 31 |
| BRACE2007953 | 0.104236124 | 154196 | 6 |
| BRACE2008008 | 0.001189244 | 54881 | 1 |
| BRACE2008015 | 0.001189244 | 56521 | 1 |
| BRACE2008021 | 0.001189244 | 129663 | 1 |
| BRACE2008097 | 0.034899573 | 159484 | 6 |
| BRACE2008102 | 0.007054456 | 146187 | 3 |
| BRACE2008114 | 0.001189244 | 213249 | 1 |
| BRACE2008144 | 0.001189244 | 199209 | 1 |
| BRACE2008172 | 0.001189244 | 133693 | 1 |
| BRACE2008216 | 0.0126554 | 156162 | 4 |
| BRACE2008218 | 0.001189244 | 191398 | 1 |
| BRACE2008251 | 0.012368099 | 160350 | 4 |
| BRACE2008276 | 0.001189244 | 139106 | 1 |
| BRACE2008295 | 0.001189244 | 159895 | 1 |
| BRACE2008308 | 0.001189244 | 168480 | 1 |
| BRACE2008336 | 0.001189244 | 183863 | 1 |
| BRACE2008358 | 0.073941797 | 92379 | 20 |
| BRACE2008361 | 0.002378489 | 56309 | 2 |
| BRACE2008380 | 0.085760918 | 142497 | 19 |
| BRACE2008385 | 0.080224922 | 121227 | 13 |
| BRACE2008399 | 0.030273708 | 68382 | 5 |
| BRACE2008401 | 0.001189244 | 189130 | 1 |
| BRACE2008402 | 0.039956833 | 72031 | 8 |
| BRACE2008410 | 0.001189244 | 206913 | 1 |
| BRACE2008420 | 0.001189244 | 259891 | 1 |
| BRACE2008443 | 0.001189244 | 147720 | 1 |
| BRACE2008473 | 0.001189244 | 239082 | 1 |
| BRACE2008480 | 0.001189244 | 210791 | 1 |
| BRACE2008481 | 0.060022429 | 52249 | 8 |
| BRACE2008488 | 0.001189244 | 90930 | 1 |
| BRACE2008495 | 0.007334771 | 42391 | 2 |
| BRACE2008500 | 0.001189244 | 41129 | 1 |
| BRACE2008553 | 0.040287278 | 185004 | 5 |
| BRACE2008594 | 0.001189244 | 75334 | 1 |
| BRACE2008604 | 0.001189244 | 139728 | 1 |
| BRACE2008615 | 0.001189244 | 235013 | 1 |
| BRACE2008622 | 0.001189244 | 222015 | 1 |
| BRACE2008629 | 0.001189244 | 210096 | 1 |
| BRACE2008653 | 0.317700554 | 22887 | 122 |
| BRACE2008655 | 0.001189244 | 82033 | 1 |
| BRACE2008656 | 0.001189244 | 90862 | 1 |
| BRACE2008706 | 0.050994595 | 2457 | 12 |
| BRACE2008708 | 0.011987788 | 154887 | 6 |
| BRACE2008710 | 0.001189244 | 244142 | 1 |
| BRACE2008754 | 0.001189244 | 110483 | 1 |
| BRACE2008797 | 0.013499711 | 113154 | 8 |
| BRACE2008847 | 0.001189244 | 212662 | 1 |
| BRACE2008857 | 0.002864792 | 166051 | 2 |
| BRACE2008861 | 0.001189244 | 196343 | 1 |
| BRACE2008867 | 0.002378489 | 47837 | 2 |
| BRACE2008881 | 0.012087227 | 120538 | 3 |
| BRACE2008883 | 0.001189244 | 232501 | 1 |
| BRACE2008917 | 0.001189244 | 271030 | 1 |
| BRACE2008919 | 0.001189244 | 218058 | 1 |
| BRACE2008941 | 0.016765604 | 130467 | 7 |
| BRACE2008948 | 0.001189244 | 20474 | 1 |
| BRACE2008960 | 0.003567733 | 209967 | 3 |
| BRACE2008968 | 0.288163224 | 121926 | 34 |
| BRACE2008999 | 0.023806966 | 143537 | 7 |
| BRACE2009014 | 0.001189244 | 95156 | 1 |
| BRACE2009016 | 0.001189244 | 242650 | 1 |
| BRACE2009031 | 0.001189244 | 100079 | 1 |
| BRACE2009037 | 0.573817419 | 4525 | 63 |
| BRACE2009044 | 0.016699921 | 166211 | 7 |
| BRACE2009045 | 0.027828217 | 83640 | 14 |
| BRACE2009053 | 0.001189244 | 229251 | 1 |
| BRACE2009122 | 0.001189244 | 81982 | 1 |
| BRACE2009131 | 0.001189244 | 15612 | 1 |
| BRACE2009185 | 0.001189244 | 257741 | 1 |
| BRACE2009188 | 0.001189244 | 66571 | 1 |
| BRACE2009212 | 0.001189244 | 103113 | 1 |
| BRACE2009221 | 0.001189244 | 262082 | 1 |
| BRACE2009235 | 0.001189244 | 37733 | 1 |
| BRACE2009238 | 0.001189244 | 244505 | 1 |
| BRACE2009255 | 0.007054456 | 221652 | 3 |
| BRACE2009270 | 0.001189244 | 262765 | 1 |
| BRACE2009272 | 0.001189244 | 256547 | 1 |
| BRACE2009274 | 0.001189244 | 259886 | 1 |
| BRACE2009275 | 0.001189244 | 246818 | 1 |
| BRACE2009291 | 0.126668675 | 116526 | 14 |
| BRACE2009293 | 0.001189244 | 261750 | 1 |
| BRACE2009307 | 0.01131341 | 72313 | 8 |
| BRACE2009311 | 0.002877521 | 236956 | 2 |
| BRACE2009318 | 0.001189244 | 254503 | 1 |
| BRACE2009323 | 0.001189244 | 228794 | 1 |
| BRACE2009361 | 0.034447498 | 106221 | 13 |
| BRACE2009366 | 0.046375375 | 168064 | 5 |
| BRACE2009421 | 0.001189244 | 256680 | 1 |
| BRACE2009428 | 0.001189244 | 234105 | 1 |
| BRACE2009434 | 0.001189244 | 239220 | 1 |
| BRACE2009437 | 0.001189244 | 244925 | 1 |
| BRACE2009483 | 0.019069861 | 93738 | 6 |
| BRACE2009517 | 0.061776874 | 106292 | 16 |
| BRACE2009533 | 0.001189244 | 262712 | 1 |
| BRACE2009558 | 0.058274451 | 117558 | 12 |
| BRACE2009566 | 0.00883448 | 199057 | 3 |
| BRACE2009591 | 0.001189244 | 233334 | 1 |
| BRACE2009620 | 0.008731926 | 79065 | 3 |
| BRACE2009654 | 0.001189244 | 216624 | 1 |
| BRACE2009721 | 0.006994423 | 182629 | 2 |
| BRACE2009732 | 0.001189244 | 197086 | 1 |
| BRACE2009754 | 0.001189244 | 74772 | 1 |
| BRACE2009828 | 0.004565798 | 141115 | 3 |
| BRACE2009886 | 0.001189244 | 189096 | 1 |
| BRACE2009907 | 0.001189244 | 125581 | 1 |
| BRACE2009957 | 0.004284741 | 37521 | 2 |
| BRACE2010170 | 0.001189244 | 168616 | 1 |
| BRACE2010171 | 0.003567733 | 173067 | 3 |
| BRACE2010203 | 0.001189244 | 14948 | 1 |
| BRACE2010208 | 0.001189244 | 168617 | 1 |
| BRACE2010336 | 0.012313952 | 155347 | 2 |
| BRACE2010348 | 0.001189244 | 168611 | 1 |
| BRACE2010440 | 0.001189244 | 14807 | 1 |
| BRACE2010444 | 0.001189244 | 100861 | 1 |
| BRACE2010477 | 0.001189244 | 163301 | 1 |
| BRACE2010489 | 0.015829508 | 14377 | 10 |
| BRACE2010535 | 0.001189244 | 158388 | 1 |
| BRACE2010550 | 0.001189244 | 143937 | 1 |
| BRACE2010598 | 0.001189244 | 95651 | 1 |
| BRACE2010632 | 0.001189244 | 133217 | 1 |
| BRACE2010642 | 0.010897982 | 169237 | 2 |
| BRACE2010651 | 0.009067111 | 94884 | 3 |
| BRACE2010669 | 0.026283838 | 134883 | 15 |
| BRACE2010684 | 0.003066086 | 82176 | 2 |
| BRACE2010753 | 0.008983134 | 239625 | 3 |
| BRACE2010813 | 0.001189244 | 113253 | 1 |
| BRACE2010828 | 0.004284741 | 128309 | 2 |
| BRACE2010837 | 0.001189244 | 78307 | 1 |
| BRACE2010888 | 0.001189244 | 160783 | 1 |
| BRACE2010937 | 0.001189244 | 207883 | 1 |
| BRACE2010983 | 0.001189244 | 49965 | 1 |
| BRACE2011007 | 0.001189244 | 205132 | 1 |
| BRACE2011025 | 0.001189244 | 191252 | 1 |
| BRACE2011183 | 0.026065865 | 132980 | 13 |
| BRACE2011199 | 0.001189244 | 200222 | 1 |
| BRACE2011265 | 0.045167185 | 131887 | 4 |
| BRACE2011389 | 0.001189244 | 166287 | 1 |
| BRACE2011478 | 0.001189244 | 220741 | 1 |
| BRACE2011545 | 0.001189244 | 183842 | 1 |
| BRACE2011592 | 0.001189244 | 131526 | 1 |
| BRACE2011611 | 0.001189244 | 211435 | 1 |
| BRACE2011646 | 0.001189244 | 199613 | 1 |
| BRACE2011677 | 0.001189244 | 199638 | 1 |
| BRACE2011703 | 0.004104101 | 67945 | 2 |
| BRACE2011747 | 0.002378489 | 92459 | 2 |
| BRACE2011838 | 0.001189244 | 42924 | 1 |
| BRACE2011892 | 0.001189244 | 114304 | 1 |
| BRACE2011904 | 0.001189244 | 284895 | 1 |
| BRACE2011947 | 0.001189244 | 191309 | 1 |
| BRACE2012061 | 0.009945564 | 61436 | 3 |
| BRACE2012062 | 0.001189244 | 32297 | 1 |
| BRACE2012185 | 0.001189244 | 189272 | 1 |
| BRACE2012222 | 0.007852474 | 198 | 5 |
| BRACE2012232 | 0.002877521 | 122011 | 2 |
| BRACE2012291 | 0.004284741 | 161196 | 2 |
| BRACE2012317 | 0.244180507 | 78598 | 143 |
| BRACE2012510 | 0.001189244 | 199134 | 1 |
| BRACE2012528 | 0.353320111 | 30663 | 153 |
| BRACE2012625 | 0.001189244 | 194923 | 1 |
| BRACE2012653 | 0.002877521 | 172764 | 2 |
| BRACE2012814 | 0.109265947 | 50905 | 26 |
| BRACE2012833 | 0.001189244 | 93506 | 1 |
| BRACE2012838 | 0.001189244 | 129303 | 1 |
| BRACE2012936 | 0.001189244 | 232255 | 1 |
| BRACE2012947 | 0.001189244 | 161055 | 1 |
| BRACE2013009 | 0.001189244 | 206931 | 1 |
| BRACE2013010 | 0.001189244 | 206754 | 1 |
| BRACE2013126 | 0.001189244 | 259480 | 1 |
| BRACE2013132 | 0.001189244 | 229262 | 1 |
| BRACE2013149 | 0.001189244 | 217457 | 1 |
| BRACE2013369 | 0.005366179 | 161049 | 2 |
| BRACE2013624 | 0.00931331 | 115882 | 5 |
| BRACE2013806 | 0.001189244 | 165551 | 1 |
| BRACE2013855 | 0.020889599 | 169975 | 5 |
| BRACE2013911 | 0.022290047 | 2207 | 8 |
| BRACE2014108 | 0.002378489 | 165542 | 2 |
| BRACE2014161 | 0.001189244 | 33385 | 1 |
| BRACE2014232 | 0.001189244 | 209561 | 1 |
| BRACE2014257 | 0.013511576 | 133947 | 9 |
| BRACE2014268 | 0.001189244 | 76843 | 1 |
| BRACE2014306 | 0.034424422 | 49096 | 8 |
| BRACE2014475 | 0.001189244 | 271557 | 1 |
| BRACE2014657 | 0.021688793 | 138948 | 17 |
| BRACE2014746 | 0.024011621 | 146076 | 7 |
| BRACE2014780 | 0.001189244 | 219837 | 1 |
| BRACE2014818 | 0.004104101 | 187936 | 2 |
| BRACE2014821 | 0.001189244 | 120300 | 1 |
| BRACE2014824 | 0.001189244 | 114075 | 1 |
| BRACE2014850 | 0.022340617 | 48270 | 8 |
| BRACE2014854 | 0.001189244 | 78522 | 1 |
| BRACE2014917 | 0.011278791 | 80941 | 3 |
| BRACE2015031 | 0.001189244 | 83466 | 1 |
| BRACE2015058 | 0.038215023 | 161719 | 16 |
| BRACE2015132 | 0.010169901 | 148690 | 3 |
| BRACE2015262 | 0.001189244 | 45481 | 1 |
| BRACE2015270 | 0.001189244 | 173298 | 1 |
| BRACE2015287 | 0.072963499 | 46157 | 10 |
| BRACE2015314 | 0.001189244 | 276534 | 1 |
| BRACE2015352 | 0.001189244 | 260718 | 1 |
| BRACE2015368 | 0.002864792 | 133626 | 2 |
| BRACE2015412 | 0.013762774 | 126249 | 4 |
| BRACE2015436 | 0.001189244 | 220738 | 1 |
| BRACE2015741 | 0.001189244 | 62074 | 1 |
| BRACE2015756 | 0.001189244 | 268259 | 1 |
| BRACE2015969 | 0.004104101 | 158181 | 2 |
| BRACE2015995 | 0.001189244 | 21734 | 1 |
| BRACE2016058 | 0.001189244 | 73899 | 1 |
| BRACE2016194 | 0.005033328 | 135278 | 2 |
| BRACE2016315 | 0.001189244 | 169518 | 1 |
| BRACE2016325 | 0.001189244 | 129040 | 1 |
| BRACE2016335 | 0.024032885 | 46661 | 4 |
| BRACE2016362 | 0.01086925 | 70860 | 7 |
| BRACE2016366 | 0.019719539 | 146884 | 4 |
| BRACE2016465 | 0.002378489 | 154306 | 2 |
| BRACE2016516 | 0.001189244 | 92554 | 1 |
| BRACE2016583 | 0.001189244 | 193615 | 1 |
| BRACE2016708 | 0.001189244 | 202712 | 1 |
| BRACE2016821 | 0.001189244 | 169580 | 1 |
| BRACE2016896 | 0.001189244 | 97035 | 1 |
| BRACE2016981 | 0.007391564 | 238972 | 2 |
| BRACE2017108 | 0.001189244 | 27536 | 1 |
| BRACE2017124 | 0.001189244 | 160394 | 1 |
| BRACE2017298 | 0.001189244 | 162794 | 1 |
| BRACE2017359 | 0.004087949 | 119629 | 3 |
| BRACE2017397 | 0.071040729 | 131411 | 16 |
| BRACE2017410 | 0.002378489 | 124055 | 2 |
| BRACE2017438 | 0.004066766 | 41203 | 3 |
| BRACE2017574 | 0.032704414 | 87099 | 11 |
| BRACE2017580 | 0.001189244 | 160421 | 1 |
| BRACE2017587 | 0.001189244 | 163031 | 1 |
| BRACE2017720 | 0.001189244 | 141397 | 1 |
| BRACE2017844 | 0.032605046 | 40595 | 15 |
| BRACE2017872 | 0.001189244 | 278301 | 1 |
| BRACE2017929 | 0.001189244 | 160272 | 1 |
| BRACE2017934 | 0.018128793 | 152472 | 4 |
| BRACE2017985 | 0.003066086 | 135671 | 2 |
| BRACE2017992 | 0.001189244 | 201597 | 1 |
| BRACE2018337 | 0.070348372 | 127875 | 16 |
| BRACE2018443 | 0.0025864 | 135620 | 2 |
| BRACE2018448 | 0.001189244 | 90106 | 1 |
| BRACE2018568 | 0.001189244 | 84752 | 1 |
| BRACE2018700 | 0.001189244 | 56190 | 1 |
| BRACE2018727 | 0.001189244 | 212341 | 1 |
| BRACE2018736 | 0.004142637 | 96058 | 3 |
| BRACE2018759 | 0.001189244 | 159274 | 1 |
| BRACE2018762 | 0.002898705 | 146432 | 2 |
| BRACE2018782 | 0.001189244 | 160483 | 1 |
| BRACE2018847 | 0.00858901 | 154407 | 6 |
| BRACE2019004 | 0.003047359 | 151363 | 2 |
| BRACE2019044 | 0.001189244 | 187459 | 1 |
| BRACE2019147 | 0.078238458 | 113092 | 11 |
| BRACE2019244 | 0.004104101 | 168591 | 2 |
| BRACE2019258 | 0.001189244 | 228132 | 1 |
| BRACE2019327 | 0.018697818 | 102356 | 4 |
| BRACE2019348 | 0.014419429 | 30414 | 11 |
| BRACE2019371 | 0.011205958 | 90760 | 4 |
| BRACE2019467 | 0.001189244 | 64827 | 1 |
| BRACE2019510 | 0.001189244 | 193968 | 1 |
| BRACE2019519 | 0.009001861 | 28846 | 3 |
| BRACE2019528 | 0.001189244 | 45078 | 1 |
| BRACE2019618 | 0.001189244 | 119997 | 1 |
| BRACE2019696 | 0.002378489 | 160119 | 2 |
| BRACE2019703 | 0.001189244 | 127804 | 1 |
| BRACE2019843 | 0.001189244 | 201979 | 1 |
| BRACE2019865 | 0.001189244 | 164406 | 1 |
| BRACE2019872 | 0.001189244 | 262882 | 1 |
| BRACE2019877 | 0.02758694 | 92970 | 9 |
| BRACE2020028 | 0.001189244 | 223748 | 1 |
| BRACE2020077 | 0.001189244 | 134143 | 1 |
| BRACE2020097 | 0.001189244 | 226284 | 1 |
| BRACE2020157 | 0.001189244 | 163688 | 1 |
| BRACE2020278 | 0.001189244 | 148215 | 1 |
| BRACE2020358 | 0.001189244 | 182740 | 1 |
| BRACE2020467 | 0.002378489 | 58401 | 2 |
| BRACE2020584 | 0.003187646 | 161548 | 2 |
| BRACE2020652 | 0.001189244 | 210120 | 1 |
| BRACE2020709 | 0.001189244 | 275578 | 1 |
| BRACE2020742 | 0.001189244 | 91497 | 1 |
| BRACE2020756 | 0.001189244 | 156306 | 1 |
| BRACE2020863 | 0.001189244 | 210160 | 1 |
| BRACE2020982 | 0.001189244 | 186808 | 1 |
| BRACE2021014 | 0.028283424 | 118744 | 16 |
| BRACE2021143 | 0.021173272 | 72230 | 5 |
| BRACE2021148 | 0.001189244 | 220825 | 1 |
| BRACE2021245 | 0.001189244 | 122693 | 1 |
| BRACE2021254 | 0.001189244 | 92219 | 1 |
| BRACE2021264 | 0.001189244 | 235318 | 1 |
| BRACE2021344 | 0.005366179 | 199345 | 2 |
| BRACE2021433 | 0.001189244 | 114004 | 1 |
| BRACE2021478 | 0.012943319 | 74993 | 3 |
| BRACE2021541 | 0.010525536 | 179192 | 5 |
| BRACE2021670 | 0.001189244 | 164138 | 1 |
| BRACE2021695 | 0.001189244 | 6298 | 1 |
| BRACE2021708 | 0.005659102 | 115797 | 4 |
| BRACE2021721 | 0.001189244 | 198280 | 1 |
| BRACE2021932 | 0.002898705 | 110700 | 2 |
| BRACE2021936 | 0.001189244 | 238478 | 1 |
| BRACE2022030 | 0.001189244 | 191358 | 1 |
| BRACE2022158 | 0.001189244 | 269452 | 1 |
| BRACE2022196 | 0.001189244 | 163330 | 1 |
| BRACE2022270 | 0.001189244 | 179030 | 1 |
| BRACE2022328 | 0.001189244 | 158372 | 1 |
| BRACE2022333 | 0.018873269 | 103103 | 11 |
| BRACE2022389 | 0.015768543 | 204228 | 4 |
| BRACE2022394 | 0.001189244 | 279838 | 1 |
| BRACE2022435 | 0.001189244 | 179090 | 1 |
| BRACE2022448 | 0.002378489 | 168411 | 2 |
| BRACE2022450 | 0.001189244 | 163304 | 1 |
| BRACE2022549 | 0.003270756 | 186844 | 2 |
| BRACE2022633 | 0.001189244 | 169407 | 1 |
| BRACE2022638 | 0.224805206 | 93790 | 48 |
| BRACE2022693 | 0.001189244 | 192635 | 1 |
| BRACE2022848 | 0.001189244 | 254317 | 1 |
| BRACE2022928 | 0.001189244 | 131721 | 1 |
| BRACE2022990 | 0.002877521 | 123595 | 2 |
| BRACE2023069 | 0.001189244 | 23405 | 1 |
| BRACE2023070 | 0.001189244 | 159145 | 1 |
| BRACE2023223 | 0.004586981 | 79703 | 3 |
| BRACE2023268 | 0.001189244 | 172956 | 1 |
| BRACE2023284 | 0.001189244 | 200569 | 1 |
| BRACE2023404 | 0.001189244 | 182641 | 1 |
| BRACE2023451 | 0.001189244 | 204095 | 1 |
| BRACE2023540 | 0.004756978 | 73500 | 4 |
| BRACE2023633 | 0.001189244 | 214818 | 1 |
| BRACE2023718 | 0.010440573 | 133875 | 6 |
| BRACE2023727 | 0.008486842 | 10631 | 3 |
| BRACE2023744 | 0.001189244 | 143952 | 1 |
| BRACE2023800 | 0.001189244 | 143338 | 1 |
| BRACE2023801 | 0.004463242 | 123312 | 3 |
| BRACE2024074 | 0.0025864 | 122064 | 2 |
| BRACE2024222 | 0.001189244 | 209929 | 1 |
| BRACE2024610 | 0.002378489 | 158064 | 2 |
| BRACE2024627 | 0.013687031 | 143575 | 3 |
| BRACE2024781 | 0.002864792 | 122515 | 2 |
| BRACE2024826 | 0.001189244 | 113971 | 1 |
| BRACE2025018 | 0.001189244 | 215208 | 1 |
| BRACE2025316 | 0.001189244 | 196890 | 1 |
| BRACE2025333 | 0.002378489 | 34335 | 2 |
| BRACE2025437 | 0.054881851 | 34042 | 8 |
| BRACE2025452 | 0.002378489 | 140862 | 2 |
| BRACE2025492 | 0.006366253 | 117709 | 3 |
| BRACE2025710 | 0.001189244 | 71332 | 1 |
| BRACE2025796 | 0.005366179 | 129806 | 2 |
| BRACE2025992 | 0.002378489 | 120107 | 2 |
| BRACE2026054 | 0.007329622 | 44424 | 3 |
| BRACE2026131 | 0.002284523 | 139515 | 2 |
| BRACE2026141 | 0.004284741 | 173483 | 2 |
| BRACE2026142 | 0.007425602 | 150672 | 2 |
| BRACE2026293 | 0.001189244 | 210903 | 1 |
| BRACE2026294 | 0.001189244 | 53157 | 1 |
| BRACE2026404 | 0.001189244 | 197793 | 1 |
| BRACE2026675 | 0.001189244 | 165634 | 1 |
| BRACE2026725 | 0.009138469 | 167647 | 3 |
| BRACE2026836 | 0.024282302 | 192680 | 3 |
| BRACE2026920 | 0.001189244 | 168423 | 1 |
| BRACE2026971 | 0.001189244 | 59027 | 1 |
| BRACE2027018 | 0.007125019 | 216761 | 2 |
| BRACE2027221 | 0.001189244 | 84704 | 1 |
| BRACE2027258 | 0.004959033 | 194107 | 3 |
| BRACE2027312 | 0.001189244 | 247720 | 1 |
| BRACE2027382 | 0.001189244 | 232038 | 1 |
| BRACE2027389 | 0.001189244 | 227058 | 1 |
| BRACE2027408 | 0.001189244 | 106881 | 1 |
| BRACE2027643 | 0.011632802 | 98024 | 5 |
| BRACE2027767 | 0.005729583 | 149324 | 4 |
| BRACE2027770 | 0.001189244 | 158066 | 1 |
| BRACE2027879 | 0.001189244 | 231993 | 1 |
| BRACE2027896 | 0.006551469 | 32851 | 4 |
| BRACE2027915 | 0.004460001 | 171316 | 3 |
| BRACE2027970 | 0.0025864 | 166086 | 2 |
| BRACE2028171 | 0.010897982 | 203768 | 2 |
| BRACE2028392 | 0.032870112 | 155946 | 7 |
| BRACE2028410 | 0.001189244 | 35686 | 1 |
| BRACE2028636 | 0.012520806 | 18826 | 6 |
| BRACE2028741 | 0.001189244 | 180711 | 1 |
| BRACE2028956 | 0.001189244 | 156858 | 1 |
| BRACE2028970 | 0.001189244 | 170565 | 1 |
| BRACE2029112 | 0.001189244 | 73421 | 1 |
| BRACE2029356 | 0.0025864 | 215157 | 2 |
| BRACE2029396 | 0.001189244 | 203977 | 1 |
| BRACE2029581 | 0.001189244 | 143923 | 1 |
| BRACE2029644 | 0.0025864 | 177295 | 2 |
| BRACE2029849 | 0.001189244 | 270322 | 1 |
| BRACE2030039 | 0.001189244 | 262525 | 1 |
| BRACE2030096 | 0.001189244 | 234442 | 1 |
| BRACE2030323 | 0.01069797 | 109078 | 7 |
| BRACE2030326 | 0.001189244 | 170455 | 1 |
| BRACE2030341 | 0.024224665 | 150448 | 7 |
| BRACE2030345 | 0.0025864 | 152729 | 2 |
| BRACE2030697 | 0.079484867 | 8648 | 23 |
| BRACE2030736 | 0.001189244 | 223746 | 1 |
| BRACE2030883 | 0.001189244 | 282592 | 1 |
| BRACE2030884 | 0.001189244 | 219424 | 1 |
| BRACE2031154 | 0.003047359 | 181094 | 2 |
| BRACE2031232 | 0.001189244 | 163185 | 1 |
| BRACE2031389 | 0.016472575 | 153391 | 6 |
| BRACE2031527 | 0.001189244 | 173342 | 1 |
| BRACE2031531 | 0.001189244 | 173341 | 1 |
| BRACE2031553 | 0.001189244 | 273894 | 1 |
| BRACE2031899 | 0.001189244 | 169575 | 1 |
| BRACE2032044 | 0.001189244 | 143019 | 1 |
| BRACE2032090 | 0.001189244 | 61887 | 1 |
| BRACE2032134 | 0.001189244 | 197812 | 1 |
| BRACE2032148 | 0.001189244 | 183268 | 1 |
| BRACE2032182 | 0.001189244 | 193781 | 1 |
| BRACE2032329 | 0.002378489 | 147651 | 2 |
| BRACE2032385 | 0.001189244 | 145368 | 1 |
| BRACE2032436 | 0.001189244 | 186471 | 1 |
| BRACE2032477 | 0.030068076 | 139630 | 11 |
| BRACE2032502 | 0.003047359 | 158563 | 2 |
| BRACE2032538 | 0.001189244 | 193647 | 1 |
| BRACE2032584 | 0.001189244 | 173439 | 1 |
| BRACE2032823 | 0.001189244 | 159076 | 1 |
| BRACE2033128 | 0.001189244 | 191725 | 1 |
| BRACE2033394 | 0.001189244 | 173417 | 1 |
| BRACE2033478 | 0.001189244 | 189015 | 1 |
| BRACE2033720 | 0.001189244 | 200989 | 1 |
| BRACE2033786 | 0.001189244 | 173409 | 1 |
| BRACE2034434 | 0.001189244 | 270663 | 1 |
| BRACE2034523 | 0.001189244 | 277470 | 1 |
| BRACE2034616 | 0.001189244 | 221896 | 1 |
| BRACE2034622 | 0.001189244 | 203209 | 1 |
| BRACE2034870 | 0.001189244 | 255002 | 1 |
| BRACE2035003 | 0.001189244 | 237866 | 1 |
| BRACE2035120 | 0.001189244 | 97700 | 1 |
| BRACE2035124 | 0.001189244 | 135999 | 1 |
| BRACE2035191 | 0.001189244 | 181140 | 1 |
| BRACE2035278 | 0.004460001 | 193209 | 3 |
| BRACE2035381 | 0.016463922 | 140683 | 11 |
| BRACE2035441 | 0.001189244 | 196939 | 1 |
| BRACE2035485 | 0.001189244 | 156997 | 1 |
| BRACE2036005 | 0.001189244 | 279947 | 1 |
| BRACE2036096 | 0.001189244 | 283323 | 1 |
| BRACE2036743 | 0.001189244 | 55962 | 1 |
| BRACE2036830 | 0.001189244 | 154543 | 1 |
| BRACE2036834 | 0.001189244 | 160118 | 1 |
| BRACE2036900 | 0.001189244 | 173343 | 1 |
| BRACE2037132 | 0.002864792 | 161142 | 2 |
| BRACE2037294 | 0.001189244 | 275703 | 1 |
| BRACE2037295 | 0.05107896 | 138332 | 8 |
| BRACE2037299 | 0.001189244 | 255744 | 1 |
| BRACE2037310 | 0.008539914 | 116022 | 5 |
| BRACE2037431 | 0.001189244 | 280273 | 1 |
| BRACE2037692 | 0.001189244 | 278363 | 1 |
| BRACE2037847 | 0.001189244 | 261549 | 1 |
| BRACE2038114 | 0.001189244 | 187272 | 1 |
| BRACE2038269 | 0.002284523 | 185857 | 2 |
| BRACE2038271 | 0.002378489 | 233254 | 2 |
| BRACE2038329 | 0.003567733 | 106366 | 3 |
| BRACE2038492 | 0.001189244 | 276491 | 1 |
| BRACE2038551 | 0.001189244 | 186443 | 1 |
| BRACE2038618 | 0.001189244 | 253910 | 1 |
| BRACE2039009 | 0.001189244 | 197781 | 1 |
| BRACE2039128 | 0.001189244 | 206396 | 1 |
| BRACE2039170 | 0.001189244 | 176476 | 1 |
| BRACE2039249 | 0.001189244 | 217790 | 1 |
| BRACE2039286 | 0.001189244 | 159894 | 1 |
| BRACE2039297 | 0.001189244 | 271657 | 1 |
| BRACE2039327 | 0.001189244 | 120710 | 1 |
| BRACE2039362 | 0.001189244 | 266478 | 1 |
| BRACE2039475 | 0.001189244 | 162948 | 1 |
| BRACE2039600 | 0.001189244 | 156205 | 1 |
| BRACE2039607 | 0.001189244 | 248814 | 1 |
| BRACE2039624 | 0.17318819 | 110639 | 34 |
| BRACE2039734 | 0.001189244 | 180966 | 1 |
| BRACE2039823 | 0.002877521 | 34063 | 2 |
| BRACE2039832 | 0.001189244 | 160250 | 1 |
| BRACE2039893 | 0.001189244 | 90770 | 1 |
| BRACE2039986 | 0.015799087 | 95628 | 3 |
| BRACE2040138 | 0.001189244 | 256291 | 1 |
| BRACE2040325 | 0.001189244 | 264272 | 1 |
| BRACE2040514 | 0.001189244 | 69404 | 1 |
| BRACE2040640 | 0.001189244 | 178480 | 1 |
| BRACE2040987 | 0.001189244 | 151322 | 1 |
| BRACE2041009 | 0.001189244 | 194083 | 1 |
| BRACE2041095 | 0.001189244 | 240601 | 1 |
| BRACE2041200 | 0.003066086 | 127048 | 2 |
| BRACE2041264 | 0.001189244 | 194076 | 1 |
| BRACE2041890 | 0.001189244 | 165880 | 1 |
| BRACE2041898 | 0.001189244 | 162801 | 1 |
| BRACE2042394 | 0.001189244 | 207506 | 1 |
| BRACE2042398 | 0.001189244 | 176238 | 1 |
| BRACE2042541 | 0.005946222 | 149522 | 5 |
| BRACE2042550 | 0.001189244 | 280151 | 1 |
| BRACE2042620 | 0.001189244 | 173528 | 1 |
| BRACE2042628 | 0.001189244 | 208427 | 1 |
| BRACE2042873 | 0.001189244 | 221630 | 1 |
| BRACE2043036 | 0.001189244 | 200931 | 1 |
| BRACE2043105 | 0.001189244 | 202711 | 1 |
| BRACE2043142 | 0.002284523 | 217179 | 2 |
| BRACE2043248 | 0.001189244 | 215841 | 1 |
| BRACE2043349 | 0.001189244 | 132431 | 1 |
| BRACE2043381 | 0.015568047 | 32764 | 3 |
| BRACE2043665 | 0.007030707 | 218663 | 2 |
| BRACE2043774 | 0.001189244 | 281174 | 1 |
| BRACE2043942 | 0.001189244 | 269821 | 1 |
| BRACE2044057 | 0.001189244 | 233553 | 1 |
| BRACE2044105 | 0.008326089 | 182136 | 3 |
| BRACE2044263 | 0.001189244 | 148866 | 1 |
| BRACE2044286 | 0.008975759 | 107828 | 5 |
| BRACE2044304 | 0.001189244 | 272371 | 1 |
| BRACE2044473 | 0.001189244 | 276278 | 1 |
| BRACE2044510 | 0.001189244 | 275695 | 1 |
| BRACE2044640 | 0.001189244 | 228568 | 1 |
| BRACE2044816 | 0.001189244 | 187746 | 1 |
| BRACE2044946 | 0.001189244 | 184533 | 1 |
| BRACE2044949 | 0.042995265 | 275421 | 2 |
| BRACE2045300 | 0.001189244 | 162489 | 1 |
| BRACE2045328 | 0.007852474 | 198 | 5 |
| BRACE2045428 | 0.001189244 | 228314 | 1 |
| BRACE2045445 | 0.003187646 | 53580 | 2 |
| BRACE2045596 | 0.001189244 | 246679 | 1 |
| BRACE2045772 | 0.057878587 | 178675 | 2 |
| BRACE2045947 | 0.001189244 | 205141 | 1 |
| BRACE2045954 | 0.006135548 | 152963 | 4 |
| BRACE2046251 | 0.001189244 | 28876 | 1 |
| BRACE2046295 | 0.001189244 | 205392 | 1 |
| BRACE2046976 | 0.001189244 | 274104 | 1 |
| BRACE2047011 | 0.001189244 | 181440 | 1 |
| BRACE2047232 | 0.001189244 | 151494 | 1 |
| BRACE2047350 | 0.0025864 | 262844 | 2 |
| BRACE2047377 | 0.001189244 | 156767 | 1 |
| BRACE2047385 | 0.001189244 | 192935 | 1 |
| BRACE2047492 | 0.002877521 | 226506 | 2 |
| BRACE2047558 | 0.001189244 | 134717 | 1 |
| BRACE2047573 | 0.001189244 | 46721 | 1 |
| BRACE2047835 | 0.001189244 | 181494 | 1 |
| BRACE2047893 | 0.001189244 | 211781 | 1 |
| BRACE2047975 | 0.001189244 | 225885 | 1 |
| BRACE3000071 | 0.012475926 | 220026 | 2 |
| BRACE3000404 | 0.001189244 | 194754 | 1 |
| BRACE3000520 | 0.001189244 | 233671 | 1 |
| BRACE3000697 | 0.001189244 | 281964 | 1 |
| BRACE3000787 | 0.015304811 | 163440 | 7 |
| BRACE3000840 | 0.001189244 | 271497 | 1 |
| BRACE3000973 | 0.012431434 | 42734 | 10 |
| BRACE3001002 | 0.001189244 | 152370 | 1 |
| BRACE3001058 | 0.001189244 | 239739 | 1 |
| BRACE3001066 | 0.004310051 | 131463 | 2 |
| BRACE3001113 | 0.006366253 | 70834 | 3 |
| BRACE3001217 | 0.001189244 | 226468 | 1 |
| BRACE3001299 | 0.001189244 | 281001 | 1 |
| BRACE3001384 | 0.001189244 | 278808 | 1 |
| BRACE3001391 | 0.001189244 | 3627 | 1 |
| BRACE3001403 | 0.001189244 | 276971 | 1 |
| BRACE3001595 | 0.001189244 | 280871 | 1 |
| BRACE3001754 | 0.003066086 | 106962 | 2 |
| BRACE3001948 | 0.001189244 | 144921 | 1 |
| BRACE3001973 | 0.003270756 | 270727 | 2 |
| BRACE3002184 | 0.006252716 | 18869 | 3 |
| BRACE3002298 | 0.001189244 | 171941 | 1 |
| BRACE3002344 | 0.001189244 | 98555 | 1 |
| BRACE3002390 | 0.001189244 | 79450 | 1 |
| BRACE3002420 | 0.001189244 | 233592 | 1 |
| BRACE3002508 | 0.001189244 | 274398 | 1 |
| BRACE3002541 | 0.001189244 | 223843 | 1 |
| BRACE3002756 | 0.001189244 | 237062 | 1 |
| BRACE3003004 | 0.001189244 | 241547 | 1 |
| BRACE3003026 | 0.010260967 | 44231 | 4 |
| BRACE3003053 | 0.003270756 | 212426 | 2 |
| BRACE3003192 | 0.002864792 | 125573 | 2 |
| BRACE3003413 | 0.0025864 | 224239 | 2 |
| BRACE3003595 | 0.007135467 | 109650 | 6 |
| BRACE3003698 | 0.0025864 | 234028 | 2 |
| BRACE3003866 | 0.001189244 | 211465 | 1 |
| BRACE3003920 | 0.001189244 | 176605 | 1 |
| BRACE3004046 | 0.002898705 | 279350 | 2 |
| BRACE3004058 | 0.001189244 | 159838 | 1 |
| BRACE3004113 | 0.001189244 | 164089 | 1 |
| BRACE3004150 | 0.043140332 | 22395 | 7 |
| BRACE3004205 | 0.003473767 | 226065 | 3 |
| BRACE3004358 | 0.001189244 | 228022 | 1 |
| BRACE3004371 | 0.044600234 | 39521 | 18 |
| BRACE3004435 | 0.001189244 | 201680 | 1 |
| BRACE3004477 | 0.001189244 | 176719 | 1 |
| BRACE3004767 | 0.002864792 | 236073 | 2 |
| BRACE3004772 | 0.001189244 | 229092 | 1 |
| BRACE3004783 | 0.002378489 | 233556 | 2 |
| BRACE3004843 | 0.005266482 | 170248 | 3 |
| BRACE3004862 | 0.003066086 | 83676 | 2 |
| BRACE3004880 | 0.001189244 | 222589 | 1 |
| BRACE3004887 | 0.001189244 | 190917 | 1 |
| BRACE3004981 | 0.001189244 | 187202 | 1 |
| BRACE3004996 | 0.001189244 | 232376 | 1 |
| BRACE3004998 | 0.001189244 | 129051 | 1 |
| BRACE3005103 | 0.001189244 | 50996 | 1 |
| BRACE3005107 | 0.006416772 | 199019 | 3 |
| BRACE3005127 | 0.001189244 | 241318 | 1 |
| BRACE3005145 | 0.001189244 | 223316 | 1 |
| BRACE3005217 | 0.001189244 | 274015 | 1 |
| BRACE3005225 | 0.001189244 | 150216 | 1 |
| BRACE3005430 | 0.001189244 | 229360 | 1 |
| BRACE3005499 | 0.005357225 | 161806 | 4 |
| BRACE3005760 | 0.007439043 | 207400 | 5 |
| BRACE3005870 | 0.001189244 | 208337 | 1 |
| BRACE3005903 | 0.001189244 | 232736 | 1 |
| BRACE3005938 | 0.152151783 | 127032 | 36 |
| BRACE3005989 | 0.716731118 | 91573 | 19 |
| BRACE3006113 | 0.001189244 | 208623 | 1 |
| BRACE3006185 | 0.001189244 | 187232 | 1 |
| BRACE3006226 | 0.001189244 | 142659 | 1 |
| BRACE3006462 | 0.001189244 | 252312 | 1 |
| BRACE3006463 | 0.001189244 | 234664 | 1 |
| BRACE3006553 | 0.013017557 | 50414 | 11 |
| BRACE3006872 | 0.001189244 | 255765 | 1 |
| BRACE3006917 | 0.001189244 | 235972 | 1 |
| BRACE3007058 | 0.002284523 | 135281 | 2 |
| BRACE3007084 | 0.001189244 | 160259 | 1 |
| BRACE3007085 | 0.001189244 | 172323 | 1 |
| BRACE3007258 | 0.012875282 | 6581 | 8 |
| BRACE3007322 | 0.001189244 | 275240 | 1 |
| BRACE3007472 | 0.001189244 | 258709 | 1 |
| BRACE3007480 | 0.025816447 | 128624 | 16 |
| BRACE3007559 | 0.001189244 | 229380 | 1 |
| BRACE3007625 | 0.008324711 | 131927 | 7 |
| BRACE3007642 | 0.001189244 | 161691 | 1 |
| BRACE3007649 | 0.002378489 | 249540 | 2 |
| BRACE3007767 | 0.001189244 | 247436 | 1 |
| BRACE3007869 | 0.001189244 | 199684 | 1 |
| BRACE3008036 | 0.009079841 | 191467 | 3 |
| BRACE3008066 | 0.001189244 | 2300 | 1 |
| BRACE3008092 | 0.001189244 | 194640 | 1 |
| BRACE3008095 | 0.0025864 | 101779 | 2 |
| BRACE3008137 | 0.001189244 | 64407 | 1 |
| BRACE3008238 | 0.036215441 | 100290 | 21 |
| BRACE3008255 | 0.001189244 | 174065 | 1 |
| BRACE3008298 | 0.038198657 | 124033 | 19 |
| BRACE3008337 | 0.001189244 | 228948 | 1 |
| BRACE3008384 | 0.004236603 | 188789 | 3 |
| BRACE3008491 | 0.107321308 | 43537 | 34 |
| BRACE3008720 | 0.001189244 | 180099 | 1 |
| BRACE3008772 | 0.003187646 | 207047 | 2 |
| BRACE3009044 | 0.001189244 | 153369 | 1 |
| BRACE3009075 | 0.001189244 | 238861 | 1 |
| BRACE3009090 | 0.001189244 | 218553 | 1 |
| BRACE3009127 | 0.001189244 | 91612 | 1 |
| BRACE3009197 | 0.001189244 | 238873 | 1 |
| BRACE3009237 | 0.009237104 | 182663 | 6 |
| BRACE3009265 | 0.001189244 | 277243 | 1 |
| BRACE3009297 | 0.001189244 | 116554 | 1 |
| BRACE3009377 | 0.001189244 | 244308 | 1 |
| BRACE3009392 | 0.001189244 | 251948 | 1 |
| BRACE3009416 | 0.005870825 | 229667 | 4 |
| BRACE3009539 | 0.001189244 | 184910 | 1 |
| BRACE3009574 | 0.001189244 | 199561 | 1 |
| BRACE3009701 | 0.001189244 | 252788 | 1 |
| BRACE3009708 | 0.001189244 | 78624 | 1 |
| BRACE3009724 | 0.002864792 | 164137 | 2 |
| BRACE3009747 | 0.074889021 | 89002 | 14 |
| BRACE3009993 | 0.001189244 | 242139 | 1 |
| BRACE3010076 | 0.001189244 | 251989 | 1 |
| BRACE3010079 | 0.001189244 | 240749 | 1 |
| BRACE3010086 | 0.001189244 | 232264 | 1 |
| BRACE3010221 | 0.002378489 | 234204 | 2 |
| BRACE3010283 | 0.003066086 | 122361 | 2 |
| BRACE3010315 | 0.001189244 | 246165 | 1 |
| BRACE3010397 | 0.004905473 | 206862 | 3 |
| BRACE3010416 | 0.001189244 | 277833 | 1 |
| BRACE3010428 | 0.146373937 | 116230 | 44 |
| BRACE3010435 | 0.001189244 | 236289 | 1 |
| BRACE3010438 | 0.093572816 | 44400 | 21 |
| BRACE3010702 | 0.001189244 | 244257 | 1 |
| BRACE3010862 | 0.001189244 | 250450 | 1 |
| BRACE3011271 | 0.01518253 | 35201 | 10 |
| BRACE3011421 | 0.005930878 | 82580 | 4 |
| BRACE3011447 | 0.001189244 | 226423 | 1 |
| BRACE3011502 | 0.064024495 | 110806 | 23 |
| BRACE3011505 | 0.001189244 | 206641 | 1 |
| BRACE3011634 | 0.001189244 | 250490 | 1 |
| BRACE3011774 | 0.001189244 | 167245 | 1 |
| BRACE3012043 | 0.009338148 | 133794 | 5 |
| BRACE3012357 | 0.002378489 | 143987 | 2 |
| BRACE3012364 | 0.039395015 | 121642 | 19 |
| BRACE3012574 | 0.001189244 | 152013 | 1 |
| BRACE3012806 | 0.0025864 | 139436 | 2 |
| BRACE3012930 | 0.003066086 | 158250 | 2 |
| BRACE3013119 | 0.001189244 | 274622 | 1 |
| BRACE3013418 | 0.002378489 | 118255 | 2 |
| BRACE3013576 | 0.001189244 | 283925 | 1 |
| BRACE3013740 | 0.001189244 | 139616 | 1 |
| BRACE3013780 | 0.034934141 | 107332 | 6 |
| BRACE3013874 | 0.001189244 | 279648 | 1 |
| BRACE3013936 | 0.005499295 | 105973 | 3 |
| BRACE3013986 | 0.001189244 | 258980 | 1 |
| BRACE3014005 | 0.001189244 | 258982 | 1 |
| BRACE3014068 | 0.001189244 | 240010 | 1 |
| BRACE3014231 | 0.001189244 | 250592 | 1 |
| BRACE3014290 | 0.002378489 | 197547 | 2 |
| BRACE3014317 | 0.001189244 | 102335 | 1 |
| BRACE3014523 | 0.001189244 | 61325 | 1 |
| BRACE3014714 | 0.001189244 | 140386 | 1 |
| BRACE3014807 | 0.001189244 | 175809 | 1 |
| BRACE3014942 | 0.001189244 | 148478 | 1 |
| BRACE3015027 | 0.064566514 | 117326 | 12 |
| BRACE3015090 | 0.001189244 | 129069 | 1 |
| BRACE3015121 | 0.001189244 | 138155 | 1 |
| BRACE3015262 | 0.002378489 | 257963 | 2 |
| BRACE3015521 | 0.001189244 | 258322 | 1 |
| BRACE3015808 | 0.001189244 | 228723 | 1 |
| BRACE3015829 | 0.001189244 | 237508 | 1 |
| BRACE3015894 | 0.001189244 | 148524 | 1 |
| BRACE3015898 | 0.001189244 | 228760 | 1 |
| BRACE3016020 | 0.001189244 | 144653 | 1 |
| BRACE3016122 | 0.002864792 | 118093 | 2 |
| BRACE3016167 | 0.001189244 | 244536 | 1 |
| BRACE3016170 | 0.001189244 | 244270 | 1 |
| BRACE3016580 | 0.001189244 | 227829 | 1 |
| BRACE3016788 | 0.001189244 | 248860 | 1 |
| BRACE3016810 | 0.001189244 | 249727 | 1 |
| BRACE3016862 | 0.001189244 | 229470 | 1 |
| BRACE3016884 | 0.025952275 | 211347 | 4 |
| BRACE3017083 | 0.001189244 | 235213 | 1 |
| BRACE3017253 | 0.004255331 | 234499 | 3 |
| BRACE3018083 | 0.001189244 | 235175 | 1 |
| BRACE3018308 | 0.001189244 | 222009 | 1 |
| BRACE3018963 | 0.002898705 | 136132 | 2 |
| BRACE3019055 | 0.002378489 | 166429 | 2 |
| BRACE3019071 | 0.00591215 | 205684 | 4 |
| BRACE3019084 | 0.001189244 | 199688 | 1 |
| BRACE3019570 | 0.001189244 | 124762 | 1 |
| BRACE3019611 | 0.001189244 | 258177 | 1 |
| BRACE3019817 | 0.001189244 | 135429 | 1 |
| BRACE3019828 | 0.001189244 | 136479 | 1 |
| BRACE3019941 | 0.057350561 | 101871 | 13 |
| BRACE3020151 | 0.001189244 | 164354 | 1 |
| BRACE3020194 | 0.001189244 | 118031 | 1 |
| BRACE3020286 | 0.001189244 | 276135 | 1 |
| BRACE3020356 | 0.001189244 | 241825 | 1 |
| BRACE3020594 | 0.001189244 | 227580 | 1 |
| BRACE3020669 | 0.003066086 | 117372 | 2 |
| BRACE3020671 | 0.001189244 | 273251 | 1 |
| BRACE3020890 | 0.001189244 | 246702 | 1 |
| BRACE3021148 | 0.001189244 | 229888 | 1 |
| BRACE3021430 | 0.014045078 | 121125 | 3 |
| BRACE3021517 | 0.001189244 | 183991 | 1 |
| BRACE3021805 | 0.001189244 | 258470 | 1 |
| BRACE3022051 | 0.001189244 | 238809 | 1 |
| BRACE3022303 | 0.001189244 | 247697 | 1 |
| BRACE3022312 | 0.004924201 | 137816 | 3 |
| BRACE3022340 | 0.002864792 | 124763 | 2 |
| BRACE3022769 | 0.117309152 | 99020 | 46 |
| BRACE3022847 | 0.001189244 | 214850 | 1 |
| BRACE3023604 | 0.001189244 | 269992 | 1 |
| BRACE3023912 | 0.001189244 | 189694 | 1 |
| BRACE3024073 | 0.001189244 | 82623 | 1 |
| BRACE3024359 | 0.001189244 | 175638 | 1 |
| BRACE3024379 | 0.001189244 | 283649 | 1 |
| BRACE3024444 | 0.001189244 | 217297 | 1 |
| BRACE3024497 | 0.001189244 | 235096 | 1 |
| BRACE3024537 | 0.001189244 | 79454 | 1 |
| BRACE3024659 | 0.001189244 | 74718 | 1 |
| BRACE3024662 | 0.001189244 | 198906 | 1 |
| BRACE3024879 | 0.001189244 | 164449 | 1 |
| BRACE3025153 | 0.001189244 | 154384 | 1 |
| BRACE3025302 | 0.001189244 | 137650 | 1 |
| BRACE3025457 | 0.001189244 | 145581 | 1 |
| BRACE3025531 | 0.001189244 | 126933 | 1 |
| BRACE3025627 | 0.001189244 | 149321 | 1 |
| BRACE3025630 | 0.003567733 | 148647 | 3 |
| BRACE3025719 | 0.009302444 | 53572 | 3 |
| BRACE3026008 | 0.001189244 | 122419 | 1 |
| BRACE3026075 | 0.001189244 | 126932 | 1 |
| BRACE3026161 | 0.001189244 | 171340 | 1 |
| BRACE3026290 | 0.001189244 | 124589 | 1 |
| BRACE3026345 | 0.001189244 | 246787 | 1 |
| BRACE3026456 | 0.002378489 | 214257 | 2 |
| BRACE3026735 | 0.006931557 | 163414 | 5 |
| BRACE3026802 | 0.009804091 | 64897 | 4 |
| BRACE3026844 | 0.001189244 | 246661 | 1 |
| BRACE3026947 | 0.001189244 | 256532 | 1 |
| BRACE3026993 | 0.002378489 | 158252 | 2 |
| BRACE3027242 | 0.001189244 | 214578 | 1 |
| BRACE3027256 | 0.001189244 | 205914 | 1 |
| BRACE3027326 | 0.001189244 | 171329 | 1 |
| BRACE3027478 | 0.010468993 | 119584 | 6 |
| BRACE3027480 | 0.001189244 | 211484 | 1 |
| BRACE3027931 | 0.004980217 | 110747 | 3 |
| BRACE3027976 | 0.0025864 | 108599 | 2 |
| BRACE3028214 | 0.001189244 | 115909 | 1 |
| BRACE3028241 | 0.001189244 | 161249 | 1 |
| BRACE3028360 | 0.001189244 | 181059 | 1 |
| BRACE3028895 | 0.003681678 | 28758 | 3 |
| BRACE3028998 | 0.001189244 | 134522 | 1 |
| BRACE3029005 | 0.001189244 | 141654 | 1 |
| BRACE3029021 | 0.004608165 | 91068 | 3 |
| BRACE3029205 | 0.001189244 | 134452 | 1 |
| BRACE3029447 | 0.001189244 | 148048 | 1 |
| BRACE3030057 | 0.001189244 | 69400 | 1 |
| BRACE3030103 | 0.001189244 | 180187 | 1 |
| BRACE3030538 | 0.003270756 | 102592 | 2 |
| BRACE3030748 | 0.001189244 | 145766 | 1 |
| BRACE3030866 | 0.002864792 | 82596 | 2 |
| BRACE3031161 | 0.001189244 | 145480 | 1 |
| BRACE3031184 | 0.001189244 | 132373 | 1 |
| BRACE3031185 | 0.002864792 | 125436 | 2 |
| BRACE3031315 | 0.002877521 | 244358 | 2 |
| BRACE3031372 | 0.001189244 | 249159 | 1 |
| BRACE3031579 | 0.001189244 | 109237 | 1 |
| BRACE3031728 | 0.001189244 | 241878 | 1 |
| BRACE3031743 | 0.030714937 | 71298 | 12 |
| BRACE3031838 | 0.002864792 | 252119 | 2 |
| BRACE3031843 | 0.001189244 | 221115 | 1 |
| BRACE3032385 | 0.024506169 | 132139 | 14 |
| BRACE3032427 | 0.002864792 | 195440 | 2 |
| BRACE3032455 | 0.001189244 | 261202 | 1 |
| BRACE3032537 | 0.001189244 | 240808 | 1 |
| BRACE3032538 | 0.001189244 | 164141 | 1 |
| BRACE3032631 | 0.013285472 | 129386 | 5 |
| BRACE3032771 | 0.001189244 | 283486 | 1 |
| BRACE3032980 | 0.001189244 | 260415 | 1 |
| BRACE3032983 | 0.001189244 | 187643 | 1 |
| BRACE3033525 | 0.001189244 | 266837 | 1 |
| BRACE3034159 | 0.001189244 | 175485 | 1 |
| BRACE3034183 | 0.001189244 | 184775 | 1 |
| BRACE3034389 | 0.001189244 | 6570 | 1 |
| BRACE3034964 | 0.001189244 | 35141 | 1 |
| BRACE3034993 | 0.001189244 | 250584 | 1 |
| BRACE3035168 | 0.003983555 | 139342 | 3 |
| BRACE3035227 | 0.001189244 | 123021 | 1 |
| BRACE3036156 | 0.007655524 | 30705 | 5 |
| BRACE3036271 | 0.001189244 | 113895 | 1 |
| BRACE3036283 | 0.022448721 | 104123 | 8 |
| BRACE3037612 | 0.001189244 | 187772 | 1 |
| BRACE3037637 | 0.001189244 | 222659 | 1 |
| BRACE3037803 | 0.001189244 | 177702 | 1 |
| BRACE3038012 | 0.001189244 | 214713 | 1 |
| BRACE3038030 | 0.001189244 | 248786 | 1 |
| BRACE3038569 | 0.001189244 | 264527 | 1 |
| BRACE3038570 | 0.001189244 | 263137 | 1 |
| BRACE3038687 | 0.001189244 | 279931 | 1 |
| BRACE3038760 | 0.001189244 | 269558 | 1 |
| BRACE3039288 | 0.001189244 | 220816 | 1 |
| BRACE3039358 | 0.008107913 | 118354 | 5 |
| BRACE3039378 | 0.002378489 | 90659 | 2 |
| BRACE3039454 | 0.001189244 | 284195 | 1 |
| BRACE3040012 | 0.001189244 | 260452 | 1 |
| BRACE3040239 | 0.001189244 | 279874 | 1 |
| BRACE3040504 | 0.001189244 | 200637 | 1 |
| BRACE3040644 | 0.001189244 | 262790 | 1 |
| BRACE3040856 | 0.001189244 | 264498 | 1 |
| BRACE3040863 | 0.009366964 | 155117 | 6 |
| BRACE3041059 | 0.001189244 | 273044 | 1 |
| BRACE3041162 | 0.001189244 | 231432 | 1 |
| BRACE3041827 | 0.001189244 | 256379 | 1 |
| BRACE3042046 | 0.001189244 | 165416 | 1 |
| BRACE3042210 | 0.001189244 | 260938 | 1 |
| BRACE3042326 | 0.004775546 | 63088 | 3 |
| BRACE3042409 | 0.001189244 | 227217 | 1 |
| BRACE3042432 | 0.002864792 | 260685 | 2 |
| BRACE3042594 | 0.001189244 | 263963 | 1 |
| BRACE3043597 | 0.001189244 | 225982 | 1 |
| BRACE3044090 | 0.001189244 | 282897 | 1 |
| BRACE3044172 | 0.001189244 | 272396 | 1 |
| BRACE3044247 | 0.001189244 | 262335 | 1 |
| BRACE3044377 | 0.001189244 | 256842 | 1 |
| BRACE3044389 | 0.001189244 | 252401 | 1 |
| BRACE3044495 | 0.001189244 | 280557 | 1 |
| BRACE3045011 | 0.001189244 | 231521 | 1 |
| BRACE3045033 | 0.001189244 | 127483 | 1 |
| BRACE3045078 | 0.004087949 | 192835 | 3 |
| BRACE3045145 | 0.001189244 | 284077 | 1 |
| BRACE3045424 | 0.001189244 | 269869 | 1 |
| BRACE3045708 | 0.001189244 | 241816 | 1 |
| BRACE3045981 | 0.006338559 | 71048 | 5 |
| BRACE3046049 | 0.001189244 | 278286 | 1 |
| BRACE3046152 | 0.001189244 | 214332 | 1 |
| BRACE3046294 | 0.001189244 | 203297 | 1 |
| BRACE3046450 | 0.007621769 | 64401 | 6 |
| BRACE3046466 | 0.001189244 | 270869 | 1 |
| BRACE3046491 | 0.001189244 | 79387 | 1 |
| BRACE3046609 | 0.001189244 | 250690 | 1 |
| BRACE3046762 | 0.001189244 | 278219 | 1 |
| BRACE3046837 | 0.001189244 | 123388 | 1 |
| BRACE3046855 | 0.001189244 | 223302 | 1 |
| BRACE3046966 | 0.001189244 | 283072 | 1 |
| BRACE3047018 | 0.001189244 | 262974 | 1 |
| BRACE3047482 | 0.001189244 | 284339 | 1 |
| BRACE3047801 | 0.001189244 | 91325 | 1 |
| BRALZ1000047 | 0.006054368 | 209425 | 1 |
| BRALZ1000103 | 0.006054368 | 208787 | 1 |
| BRALZ2000189 | 0.006054368 | 37983 | 1 |
| BRALZ2000263 | 0.007243613 | 128577 | 2 |
| BRALZ2000988 | 0.03228223 | 116246 | 16 |
| BRALZ2001038 | 0.010309699 | 78090 | 4 |
| BRALZ2001279 | 0.012108736 | 99816 | 2 |
| BRALZ2001350 | 0.012108736 | 58272 | 2 |
| BRALZ2001445 | 0.006054368 | 101234 | 1 |
| BRALZ2001559 | 0.006054368 | 181345 | 1 |
| BRALZ2001743 | 0.006054368 | 65342 | 1 |
| BRALZ2001834 | 0.007912483 | 100135 | 2 |
| BRALZ2001966 | 0.012215794 | 183591 | 4 |
| BRALZ2002174 | 0.006054368 | 84725 | 1 |
| BRALZ2002418 | 0.007742645 | 124647 | 2 |
| BRALZ2002477 | 0.006054368 | 200027 | 1 |
| BRALZ2003119 | 0.006054368 | 97588 | 1 |
| BRALZ2003330 | 0.006054368 | 262294 | 1 |
| BRALZ2003453 | 0.006054368 | 236852 | 1 |
| BRALZ2003525 | 0.006054368 | 152942 | 1 |
| BRALZ2004397 | 0.006054368 | 172185 | 1 |
| BRALZ2005467 | 0.006054368 | 121905 | 1 |
| BRALZ2005471 | 0.006054368 | 79624 | 1 |
| BRALZ2005888 | 0.006054368 | 45044 | 1 |
| BRALZ2005950 | 0.006054368 | 205321 | 1 |
| BRALZ2005992 | 0.006054368 | 237256 | 1 |
| BRALZ2006288 | 0.015364717 | 130927 | 4 |
| BRALZ2006474 | 0.006054368 | 225106 | 1 |
| BRALZ2006560 | 0.006054368 | 227174 | 1 |
| BRALZ2006734 | 0.006054368 | 227194 | 1 |
| BRALZ2006800 | 0.012108736 | 249789 | 2 |
| BRALZ2006976 | 0.006054368 | 252208 | 1 |
| BRALZ2007376 | 0.006054368 | 244791 | 1 |
| BRALZ2007545 | 0.006054368 | 248296 | 1 |
| BRALZ2007576 | 0.006054368 | 136757 | 1 |
| BRALZ2007661 | 0.006054368 | 232125 | 1 |
| BRALZ2007790 | 0.006054368 | 7312 | 1 |
| BRALZ2007793 | 0.006054368 | 132826 | 1 |
| BRALZ2007952 | 0.006054368 | 203925 | 1 |
| BRALZ2008031 | 0.006054368 | 263450 | 1 |
| BRALZ2008166 | 0.009149864 | 100033 | 2 |
| BRALZ2008462 | 0.006054368 | 116596 | 1 |
| BRALZ2008484 | 0.015675457 | 144794 | 6 |
| BRALZ2008617 | 0.020679715 | 78975 | 4 |
| BRALZ2008696 | 0.007729915 | 116307 | 2 |
| BRALZ2008763 | 0.006054368 | 248650 | 1 |
| BRALZ2008869 | 0.006054368 | 251314 | 1 |
| BRALZ2008930 | 0.006054368 | 253441 | 1 |
| BRALZ2009446 | 0.035935332 | 95658 | 6 |
| BRALZ2009482 | 0.450889643 | 88477 | 55 |
| BRALZ2009486 | 0.01595282 | 90054 | 3 |
| BRALZ2010186 | 0.006054368 | 224258 | 1 |
| BRALZ2010842 | 0.006054368 | 202363 | 1 |
| BRALZ2011337 | 0.006054368 | 215681 | 1 |
| BRALZ2011796 | 0.030400931 | 42915 | 8 |
| BRALZ2011880 | 0.054955421 | 135349 | 10 |
| BRALZ2012050 | 0.006054368 | 256724 | 1 |
| BRALZ2012183 | 0.006054368 | 239539 | 1 |
| BRALZ2012848 | 0.006054368 | 244734 | 1 |
| BRALZ2013621 | 0.006054368 | 95352 | 1 |
| BRALZ2013690 | 0.006054368 | 112266 | 1 |
| BRALZ2014054 | 0.015763106 | 65654 | 2 |
| BRALZ2014316 | 0.006054368 | 233292 | 1 |
| BRALZ2014484 | 0.006054368 | 235211 | 1 |
| BRALZ2014951 | 0.006054368 | 202173 | 1 |
| BRALZ2015168 | 0.006054368 | 235217 | 1 |
| BRALZ2015811 | 0.006054368 | 224629 | 1 |
| BRALZ2016085 | 0.006054368 | 118047 | 1 |
| BRALZ2016498 | 0.006054368 | 224685 | 1 |
| BRALZ2017359 | 0.00805277 | 101670 | 2 |
| BRALZ2017531 | 0.006054368 | 76811 | 1 |
| BRALZ2017607 | 0.006054368 | 251769 | 1 |
| BRALZ2017620 | 0.006054368 | 256462 | 1 |
| BRALZ2017844 | 0.012256688 | 163079 | 2 |
| BRALZ2018342 | 0.006054368 | 214159 | 1 |
| BRALZ2018492 | 0.007243613 | 133939 | 2 |
| BRALZ2018834 | 0.026851826 | 172207 | 5 |
| BRALZ2019047 | 0.007912483 | 237762 | 2 |
| BRAMY1000095 | 0.001688277 | 111454 | 1 |
| BRAMY1000098 | 0.001688277 | 278339 | 1 |
| BRAMY1000130 | 0.013910227 | 174420 | 2 |
| BRAMY1000157 | 0.001688277 | 148531 | 1 |
| BRAMY1000173 | 0.015844282 | 102816 | 8 |
| BRAMY2000021 | 0.001688277 | 262947 | 1 |
| BRAMY2000025 | 0.010618374 | 142852 | 5 |
| BRAMY2000052 | 0.015472487 | 148977 | 4 |
| BRAMY2000076 | 0.003376553 | 157164 | 2 |
| BRAMY2000086 | 0.001688277 | 276108 | 1 |
| BRAMY2000151 | 0.007600539 | 66132 | 2 |
| BRAMY2000181 | 0.004783773 | 101609 | 2 |
| BRAMY2000196 | 0.001688277 | 95538 | 1 |
| BRAMY2000231 | 0.004603134 | 273607 | 2 |
| BRAMY2000277 | 0.001688277 | 205638 | 1 |
| BRAMY2000354 | 0.121936009 | 52892 | 18 |
| BRAMY2000388 | 0.027386151 | 127133 | 9 |
| BRAMY2000411 | 0.027034061 | 124231 | 8 |
| BRAMY2000508 | 0.004760979 | 2414 | 3 |
| BRAMY2000562 | 0.087191067 | 97390 | 26 |
| BRAMY2000585 | 0.068220966 | 148164 | 7 |
| BRAMY2000653 | 0.001688277 | 151864 | 1 |
| BRAMY2000814 | 0.007890596 | 131263 | 2 |
| BRAMY2000893 | 0.110106451 | 110629 | 9 |
| BRAMY2000946 | 0.001688277 | 153309 | 1 |
| BRAMY2000981 | 0.001688277 | 226304 | 1 |
| BRAMY2000987 | 0.009871944 | 8481 | 4 |
| BRAMY2001068 | 0.001688277 | 77225 | 1 |
| BRAMY2001114 | 0.001688277 | 94481 | 1 |
| BRAMY2001203 | 0.001688277 | 174295 | 1 |
| BRAMY2001282 | 0.001688277 | 85700 | 1 |
| BRAMY2001367 | 0.001688277 | 91656 | 1 |
| BRAMY2001427 | 0.001688277 | 149801 | 1 |
| BRAMY2001473 | 0.001688277 | 158269 | 1 |
| BRAMY2001678 | 0.004482587 | 91076 | 3 |
| BRAMY2002005 | 0.001688277 | 184946 | 1 |
| BRAMY2002044 | 0.003376553 | 101735 | 2 |
| BRAMY2002072 | 0.001688277 | 120931 | 1 |
| BRAMY2002158 | 0.001688277 | 162620 | 1 |
| BRAMY2002311 | 0.001688277 | 169939 | 1 |
| BRAMY2002339 | 0.001688277 | 274751 | 1 |
| BRAMY2002364 | 0.001688277 | 79012 | 1 |
| BRAMY2002369 | 0.020538448 | 66030 | 11 |
| BRAMY2002479 | 0.007729843 | 159751 | 2 |
| BRAMY2002493 | 0.001688277 | 166922 | 1 |
| BRAMY2002525 | 0.002877521 | 132126 | 2 |
| BRAMY2002584 | 0.001688277 | 168278 | 1 |
| BRAMY2002604 | 0.001688277 | 129321 | 1 |
| BRAMY2002621 | 0.004735635 | 153424 | 3 |
| BRAMY2002638 | 0.001688277 | 48990 | 1 |
| BRAMY2002739 | 0.001688277 | 276200 | 1 |
| BRAMY2002799 | 0.003363824 | 157940 | 2 |
| BRAMY2002853 | 0.001688277 | 138334 | 1 |
| BRAMY2002862 | 0.001688277 | 176836 | 1 |
| BRAMY2002956 | 0.001688277 | 181661 | 1 |
| BRAMY2003008 | 0.006335944 | 102377 | 4 |
| BRAMY2003037 | 0.006753106 | 147423 | 4 |
| BRAMY2003117 | 0.001688277 | 21320 | 1 |
| BRAMY2003287 | 0.001688277 | 181687 | 1 |
| BRAMY2003325 | 0.013494652 | 174414 | 2 |
| BRAMY2003529 | 0.001688277 | 188521 | 1 |
| BRAMY2003538 | 0.004565798 | 161463 | 3 |
| BRAMY2003583 | 0.130254954 | 84775 | 43 |
| BRAMY2003585 | 0.001688277 | 180079 | 1 |
| BRAMY2003634 | 0.001688277 | 40418 | 1 |
| BRAMY2003653 | 0.003376553 | 71471 | 2 |
| BRAMY2003681 | 0.001688277 | 278791 | 1 |
| BRAMY2003893 | 0.001688277 | 154086 | 1 |
| BRAMY2003897 | 0.001688277 | 39871 | 1 |
| BRAMY2003898 | 0.001688277 | 7501 | 1 |
| BRAMY2003904 | 0.001688277 | 273224 | 1 |
| BRAMY2003926 | 0.001688277 | 207241 | 1 |
| BRAMY2003929 | 0.007879269 | 175730 | 3 |
| BRAMY2004031 | 0.013177596 | 22981 | 4 |
| BRAMY2004058 | 0.001688277 | 235378 | 1 |
| BRAMY2004183 | 0.001688277 | 30938 | 1 |
| BRAMY2004335 | 0.001688277 | 194915 | 1 |
| BRAMY2004351 | 0.001688277 | 128355 | 1 |
| BRAMY2004352 | 0.001688277 | 188782 | 1 |
| BRAMY2004363 | 0.081959587 | 57294 | 38 |
| BRAMY2004387 | 0.005532361 | 213336 | 2 |
| BRAMY2004492 | 0.001688277 | 117969 | 1 |
| BRAMY2004521 | 0.070690827 | 118887 | 21 |
| BRAMY2004524 | 0.007729843 | 104031 | 2 |
| BRAMY2004537 | 0.001688277 | 190262 | 1 |
| BRAMY2004542 | 0.001688277 | 181836 | 1 |
| BRAMY2004756 | 0.001688277 | 86339 | 1 |
| BRAMY2004771 | 0.001688277 | 114280 | 1 |
| BRAMY2005052 | 0.014429405 | 122494 | 9 |
| BRAMY2005064 | 0.001688277 | 252478 | 1 |
| BRAMY2005094 | 0.001688277 | 171186 | 1 |
| BRAMY2005151 | 0.001688277 | 195123 | 1 |
| BRAMY2005182 | 0.001688277 | 126136 | 1 |
| BRAMY2005244 | 0.001688277 | 145878 | 1 |
| BRAMY2005391 | 0.001688277 | 89031 | 1 |
| BRAMY2005488 | 0.014703466 | 153630 | 7 |
| BRAMY2005576 | 0.007890596 | 131572 | 2 |
| BRAMY2005648 | 0.001688277 | 203292 | 1 |
| BRAMY2005662 | 0.001688277 | 76598 | 1 |
| BRAMY2005684 | 0.022570023 | 133371 | 10 |
| BRAMY2005708 | 0.001688277 | 185498 | 1 |
| BRAMY2005711 | 0.001688277 | 203299 | 1 |
| BRAMY2006092 | 0.001688277 | 229856 | 1 |
| BRAMY2006118 | 0.001688277 | 168650 | 1 |
| BRAMY2006149 | 0.001688277 | 181621 | 1 |
| BRAMY2006366 | 0.018188047 | 129951 | 4 |
| BRAMY2006375 | 0.007068987 | 72288 | 5 |
| BRAMY2006397 | 0.059666056 | 125572 | 6 |
| BRAMY2006411 | 0.001688277 | 125831 | 1 |
| BRAMY2006459 | 0.001688277 | 95693 | 1 |
| BRAMY2006466 | 0.001688277 | 146568 | 1 |
| BRAMY2006659 | 0.001688277 | 237010 | 1 |
| BRAMY2006779 | 0.001688277 | 19868 | 1 |
| BRAMY2006809 | 0.001688277 | 36409 | 1 |
| BRAMY2006879 | 0.001688277 | 87546 | 1 |
| BRAMY2007043 | 0.001688277 | 126414 | 1 |
| BRAMY2007078 | 0.044062731 | 126642 | 8 |
| BRAMY2007110 | 0.001688277 | 208844 | 1 |
| BRAMY2007185 | 0.001688277 | 223397 | 1 |
| BRAMY2007244 | 0.014296277 | 135445 | 6 |
| BRAMY2007249 | 0.001688277 | 198536 | 1 |
| BRAMY2007255 | 0.00848375 | 83492 | 5 |
| BRAMY2007287 | 0.015637282 | 84744 | 4 |
| BRAMY2007308 | 0.001688277 | 204726 | 1 |
| BRAMY2007411 | 0.001688277 | 118549 | 1 |
| BRAMY2007458 | 0.001688277 | 149654 | 1 |
| BRAMY2007486 | 0.001688277 | 266661 | 1 |
| BRAMY2007546 | 0.001688277 | 49131 | 1 |
| BRAMY2007567 | 0.021593198 | 22941 | 12 |
| BRAMY2007594 | 0.005404505 | 38055 | 3 |
| BRAMY2007610 | 0.001688277 | 114780 | 1 |
| BRAMY2007613 | 0.125082366 | 84438 | 62 |
| BRAMY2007653 | 0.001688277 | 149732 | 1 |
| BRAMY2007753 | 0.001688277 | 132915 | 1 |
| BRAMY2007859 | 0.001688277 | 263143 | 1 |
| BRAMY2008333 | 0.001688277 | 213557 | 1 |
| BRAMY2008382 | 0.010805541 | 134302 | 3 |
| BRAMY2008563 | 0.003397737 | 88603 | 2 |
| BRAMY2008583 | 0.001688277 | 11191 | 1 |
| BRAMY2008977 | 0.033139054 | 126239 | 6 |
| BRAMY2008979 | 0.001688277 | 99011 | 1 |
| BRAMY2009013 | 0.027510006 | 107963 | 13 |
| BRAMY2009023 | 0.012638846 | 49221 | 5 |
| BRAMY2009034 | 0.001688277 | 241671 | 1 |
| BRAMY2009105 | 0.001688277 | 222178 | 1 |
| BRAMY2009123 | 0.003686678 | 43124 | 2 |
| BRAMY2009192 | 0.001688277 | 213779 | 1 |
| BRAMY2009344 | 0.001688277 | 82133 | 1 |
| BRAMY2009349 | 0.001688277 | 124273 | 1 |
| BRAMY2009394 | 0.001688277 | 18397 | 1 |
| BRAMY2009489 | 0.009322617 | 129119 | 6 |
| BRAMY2009508 | 0.022346414 | 74704 | 14 |
| BRAMY2009528 | 0.001688277 | 63123 | 1 |
| BRAMY2009557 | 0.003397737 | 153062 | 2 |
| BRAMY2009612 | 0.012379628 | 55151 | 5 |
| BRAMY2009693 | 0.001688277 | 14706 | 1 |
| BRAMY2009934 | 0.028343935 | 165950 | 7 |
| BRAMY2010068 | 0.001688277 | 207845 | 1 |
| BRAMY2010135 | 0.001688277 | 149065 | 1 |
| BRAMY2010145 | 0.001688277 | 159445 | 1 |
| BRAMY2010171 | 0.001688277 | 149965 | 1 |
| BRAMY2010208 | 0.009218016 | 174692 | 3 |
| BRAMY2010239 | 0.001688277 | 159075 | 1 |
| BRAMY2010272 | 0.003363824 | 18745 | 2 |
| BRAMY2010290 | 0.001688277 | 185818 | 1 |
| BRAMY2010317 | 0.142290854 | 110977 | 54 |
| BRAMY2010357 | 0.011221561 | 105629 | 7 |
| BRAMY2010366 | 0.001688277 | 15618 | 1 |
| BRAMY2010395 | 0.001688277 | 222421 | 1 |
| BRAMY2010441 | 0.001688277 | 91575 | 1 |
| BRAMY2010442 | 0.003546391 | 215612 | 2 |
| BRAMY2010464 | 0.015208629 | 123638 | 8 |
| BRAMY2010581 | 0.001688277 | 179317 | 1 |
| BRAMY2010798 | 0.001688277 | 181265 | 1 |
| BRAMY2010808 | 0.001688277 | 165804 | 1 |
| BRAMY2010919 | 0.001688277 | 179619 | 1 |
| BRAMY2011064 | 0.001688277 | 147688 | 1 |
| BRAMY2011105 | 0.055312338 | 155765 | 5 |
| BRAMY2011178 | 0.001688277 | 126580 | 1 |
| BRAMY2011196 | 0.001688277 | 270166 | 1 |
| BRAMY2011216 | 0.001688277 | 78961 | 1 |
| BRAMY2011253 | 0.001688277 | 185381 | 1 |
| BRAMY2011280 | 0.10132157 | 79371 | 45 |
| BRAMY2011297 | 0.408013258 | 131485 | 22 |
| BRAMY2011446 | 0.004735635 | 99446 | 3 |
| BRAMY2011480 | 0.001688277 | 86064 | 1 |
| BRAMY2011639 | 0.001688277 | 231626 | 1 |
| BRAMY2011679 | 0.001688277 | 70054 | 1 |
| BRAMY2011767 | 0.080407312 | 97605 | 30 |
| BRAMY2011811 | 0.001688277 | 207642 | 1 |
| BRAMY2011841 | 0.01518463 | 154555 | 3 |
| BRAMY2011846 | 0.001688277 | 207572 | 1 |
| BRAMY2011849 | 0.003397737 | 207406 | 2 |
| BRAMY2011872 | 0.001688277 | 146966 | 1 |
| BRAMY2011961 | 0.001688277 | 159663 | 1 |
| BRAMY2012017 | 0.001688277 | 182088 | 1 |
| BRAMY2012091 | 0.028216948 | 159513 | 5 |
| BRAMY2012119 | 0.001688277 | 183683 | 1 |
| BRAMY2012162 | 0.001688277 | 134104 | 1 |
| BRAMY2012163 | 0.021192046 | 162715 | 5 |
| BRAMY2012277 | 0.001688277 | 70995 | 1 |
| BRAMY2012340 | 0.003397737 | 84398 | 2 |
| BRAMY2012426 | 0.02710763 | 61961 | 4 |
| BRAMY2012497 | 0.001688277 | 204093 | 1 |
| BRAMY2012517 | 0.001688277 | 112434 | 1 |
| BRAMY2012536 | 0.290288676 | 36444 | 21 |
| BRAMY2012555 | 0.003376553 | 185592 | 2 |
| BRAMY2012568 | 0.001688277 | 198471 | 1 |
| BRAMY2012581 | 0.007334907 | 39769 | 4 |
| BRAMY2012691 | 0.001688277 | 179817 | 1 |
| BRAMY2012721 | 0.001688277 | 263161 | 1 |
| BRAMY2012731 | 0.001688277 | 266298 | 1 |
| BRAMY2012776 | 0.001688277 | 143687 | 1 |
| BRAMY2012880 | 0.003376553 | 113376 | 2 |
| BRAMY2012881 | 0.003546391 | 65904 | 2 |
| BRAMY2013169 | 0.014735555 | 53312 | 6 |
| BRAMY2013200 | 0.001688277 | 203810 | 1 |
| BRAMY2013216 | 0.001688277 | 210436 | 1 |
| BRAMY2013405 | 0.001688277 | 216963 | 1 |
| BRAMY2013414 | 0.003376553 | 61615 | 2 |
| BRAMY2013572 | 0.001688277 | 254396 | 1 |
| BRAMY2013590 | 0.007755668 | 35084 | 4 |
| BRAMY2013621 | 0.00572656 | 182111 | 2 |
| BRAMY2013659 | 0.00576819 | 155141 | 3 |
| BRAMY2013722 | 0.001688277 | 143226 | 1 |
| BRAMY2013736 | 0.003546391 | 111232 | 2 |
| BRAMY2013756 | 0.008873318 | 149120 | 4 |
| BRAMY2013944 | 0.001688277 | 181579 | 1 |
| BRAMY2013975 | 0.001688277 | 268746 | 1 |
| BRAMY2014019 | 0.001688277 | 177887 | 1 |
| BRAMY2014205 | 0.011694423 | 126460 | 4 |
| BRAMY2014387 | 0.001688277 | 172312 | 1 |
| BRAMY2014462 | 0.003397737 | 108055 | 2 |
| BRAMY2014469 | 0.003376553 | 145453 | 2 |
| BRAMY2014541 | 0.001688277 | 210401 | 1 |
| BRAMY2014754 | 0.119621588 | 6885 | 51 |
| BRAMY2014813 | 0.017368167 | 87665 | 4 |
| BRAMY2015211 | 0.012899034 | 152308 | 4 |
| BRAMY2015251 | 0.05472127 | 94033 | 18 |
| BRAMY2015311 | 0.001688277 | 189178 | 1 |
| BRAMY2015420 | 0.007904162 | 131835 | 5 |
| BRAMY2015503 | 0.001688277 | 92331 | 1 |
| BRAMY2015516 | 0.001688277 | 279553 | 1 |
| BRAMY2015550 | 0.004943546 | 163106 | 3 |
| BRAMY2015551 | 0.001688277 | 69419 | 1 |
| BRAMY2015559 | 0.001688277 | 106643 | 1 |
| BRAMY2015688 | 0.001688277 | 272346 | 1 |
| BRAMY2015705 | 0.00572656 | 128885 | 2 |
| BRAMY2015782 | 0.042787761 | 206855 | 4 |
| BRAMY2015833 | 0.036348251 | 109722 | 12 |
| BRAMY2015855 | 0.002877521 | 148057 | 2 |
| BRAMY2015890 | 0.001688277 | 242770 | 1 |
| BRAMY2015911 | 0.011245968 | 6281 | 4 |
| BRAMY2015925 | 0.001688277 | 230468 | 1 |
| BRAMY2016002 | 0.001688277 | 105854 | 1 |
| BRAMY2016009 | 0.032040691 | 133660 | 16 |
| BRAMY2016020 | 0.003376553 | 225348 | 2 |
| BRAMY2016070 | 0.001688277 | 277068 | 1 |
| BRAMY2016251 | 0.023298868 | 152393 | 10 |
| BRAMY2016325 | 0.044469609 | 82943 | 9 |
| BRAMY2016327 | 0.001688277 | 180047 | 1 |
| BRAMY2016386 | 0.003085432 | 155444 | 2 |
| BRAMY2016611 | 0.261569543 | 75529 | 133 |
| BRAMY2016706 | 0.033805049 | 173354 | 6 |
| BRAMY2016771 | 0.001688277 | 212914 | 1 |
| BRAMY2016892 | 0.011539563 | 97158 | 7 |
| BRAMY2016938 | 0.001688277 | 159919 | 1 |
| BRAMY2016953 | 0.001688277 | 102459 | 1 |
| BRAMY2017014 | 0.001688277 | 150224 | 1 |
| BRAMY2017249 | 0.003376553 | 149613 | 2 |
| BRAMY2017348 | 0.001688277 | 171936 | 1 |
| BRAMY2017450 | 0.001688277 | 205652 | 1 |
| BRAMY2017455 | 0.001688277 | 42527 | 1 |
| BRAMY2017528 | 0.001688277 | 7948 | 1 |
| BRAMY2017864 | 0.012126528 | 118454 | 5 |
| BRAMY2018027 | 0.003376553 | 174496 | 2 |
| BRAMY2018106 | 0.001688277 | 134667 | 1 |
| BRAMY2018122 | 0.013207687 | 17774 | 2 |
| BRAMY2018126 | 0.001688277 | 30015 | 1 |
| BRAMY2018272 | 0.001688277 | 36167 | 1 |
| BRAMY2018273 | 0.001688277 | 190282 | 1 |
| BRAMY2018279 | 0.001688277 | 172766 | 1 |
| BRAMY2018295 | 0.009333512 | 127568 | 3 |
| BRAMY2018308 | 0.001688277 | 36470 | 1 |
| BRAMY2018396 | 0.001688277 | 181631 | 1 |
| BRAMY2018467 | 0.001688277 | 39335 | 1 |
| BRAMY2018635 | 0.006168562 | 124266 | 4 |
| BRAMY2018785 | 0.024638944 | 145238 | 8 |
| BRAMY2019055 | 0.001688277 | 85000 | 1 |
| BRAMY2019111 | 0.005532361 | 186675 | 2 |
| BRAMY2019277 | 0.001688277 | 213756 | 1 |
| BRAMY2019300 | 0.003363824 | 160704 | 2 |
| BRAMY2019468 | 0.044112636 | 116019 | 28 |
| BRAMY2019509 | 0.001688277 | 210006 | 1 |
| BRAMY2019546 | 0.027828217 | 83640 | 14 |
| BRAMY2019554 | 0. 209966321 | 34085 | 59 |
| BRAMY2019600 | 0.020752286 | 149067 | 9 |
| BRAMY2019643 | 0.002783555 | 94113 | 2 |
| BRAMY2019710 | 0.003546391 | 164923 | 2 |
| BRAMY2019963 | 0.02460619 | 120171 | 11 |
| BRAMY2019985 | 0.001688277 | 186853 | 1 |
| BRAMY2019989 | 0.173767891 | 97829 | 61 |
| BRAMY2020050 | 0.001688277 | 158682 | 1 |
| BRAMY2020058 | 0.005998327 | 155162 | 3 |
| BRAMY2020106 | 0.014988363 | 171974 | 5 |
| BRAMY2020270 | 0.001688277 | 271061 | 1 |
| BRAMY2020354 | 0.002783555 | 140088 | 2 |
| BRAMY2020355 | 0.001688277 | 222464 | 1 |
| BRAMY2020412 | 0.015608914 | 141693 | 3 |
| BRAMY2020427 | 0.282979705 | 33258 | 37 |
| BRAMY2020445 | 0.001688277 | 145725 | 1 |
| BRAMY2020466 | 0.001688277 | 176243 | 1 |
| BRAMY2020520 | 0.001688277 | 88406 | 1 |
| BRAMY2020574 | 0.001688277 | 188203 | 1 |
| BRAMY2020634 | 0.001688277 | 135463 | 1 |
| BRAMY2020713 | 0.006491413 | 105032 | 3 |
| BRAMY2021040 | 0.001688277 | 164848 | 1 |
| BRAMY2021063 | 0.001688277 | 194124 | 1 |
| BRAMY2021098 | 0.001688277 | 237169 | 1 |
| BRAMY2021139 | 0.006547077 | 126566 | 3 |
| BRAMY2021142 | 0.006257322 | 133382 | 5 |
| BRAMY2021276 | 0.003546391 | 119252 | 2 |
| BRAMY2021310 | 0.013486295 | 164889 | 2 |
| BRAMY2021388 | 0.001688277 | 142648 | 1 |
| BRAMY2021498 | 0.001688277 | 184636 | 1 |
| BRAMY2021523 | 0.007901386 | 202729 | 2 |
| BRAMY2021710 | 0.0222763 | 70935 | 4 |
| BRAMY2021732 | 0.001688277 | 258058 | 1 |
| BRAMY2021746 | 0.001688277 | 4424 | 1 |
| BRAMY2021825 | 0.005086013 | 133624 | 3 |
| BRAMY2021867 | 0.004735635 | 97376 | 3 |
| BRAMY2021962 | 0. 001688277 | 67422 | 1 |
| BRAMY2021982 | 0.001688277 | 275972 | 1 |
| BRAMY2022052 | 0.001688277 | 234255 | 1 |
| BRAMY2022160 | 0.013299826 | 45362 | 8 |
| BRAMY2022168 | 0.012386364 | 105685 | 7 |
| BRAMY2022263 | 0.001688277 | 269301 | 1 |
| BRAMY2022301 | 0.001688277 | 239395 | 1 |
| BRAMY2022320 | 0.001688277 | 100685 | 1 |
| BRAMY2022383 | 0.004565798 | 159773 | 3 |
| BRAMY2022445 | 0.003565118 | 176556 | 2 |
| BRAMY2022454 | 0.001688277 | 64167 | 1 |
| BRAMY2022493 | 0.063159019 | 122241 | 13 |
| BRAMY2022525 | 0.00759795 | 122571 | 4 |
| BRAMY2022532 | 0.001688277 | 176617 | 1 |
| BRAMY2022723 | 0.004735635 | 196520 | 3 |
| BRAMY2022786 | 0.134278042 | 6170 | 34 |
| BRAMY2022796 | 0.001688277 | 197503 | 1 |
| BRAMY2022929 | 0.001688277 | 213309 | 1 |
| BRAMY2022980 | 0.010975146 | 148033 | 4 |
| BRAMY2022984 | 0.001688277 | 145187 | 1 |
| BRAMY2023060 | 0.001688277 | 171657 | 1 |
| BRAMY2023110 | 0.001688277 | 109918 | 1 |
| BRAMY2023115 | 0.003397737 | 149720 | 2 |
| BRAMY2023161 | 0.001688277 | 91707 | 1 |
| BRAMY2023172 | 0.001688277 | 279892 | 1 |
| BRAMY2023238 | 0.001688277 | 128693 | 1 |
| BRAMY2023327 | 0.001688277 | 151849 | 1 |
| BRAMY2023358 | 0.001688277 | 143766 | 1 |
| BRAMY2023406 | 0.02537982 | 196749 | 4 |
| BRAMY2023482 | 0.001688277 | 55187 | 1 |
| BRAMY2023514 | 0.001688277 | 130981 | 1 |
| BRAMY2023719 | 0.02802479 | 175044 | 4 |
| BRAMY2023729 | 0.003565118 | 127398 | 2 |
| BRAMY2023786 | 0.001688277 | 18496 | 1 |
| BRAMY2023852 | 0.001688277 | 65047 | 1 |
| BRAMY2023863 | 0.004733158 | 38792 | 2 |
| BRAMY2023939 | 0.027413512 | 149393 | 10 |
| BRAMY2024004 | 0.060577217 | 89481 | 6 |
| BRAMY2024073 | 0.001688277 | 165041 | 1 |
| BRAMY2024247 | 0.015634486 | 152461 | 9 |
| BRAMY2024312 | 0.003397737 | 67516 | 2 |
| BRAMY2024374 | 0.001688277 | 160278 | 1 |
| BRAMY2024390 | 0.004794892 | 121402 | 3 |
| BRAMY2024416 | 0.037029711 | 83172 | 18 |
| BRAMY2024449 | 0.001688277 | 160352 | 1 |
| BRAMY2024489 | 0.003376553 | 169551 | 2 |
| BRAMY2024497 | 0.001688277 | 131172 | 1 |
| BRAMY2024514 | 0.001688277 | 164794 | 1 |
| BRAMY2024530 | 0.001688277 | 14950 | 1 |
| BRAMY2024535 | 0.001688277 | 273806 | 1 |
| BRAMY2024545 | 0.001688277 | 147641 | 1 |
| BRAMY2024576 | 0.001688277 | 144663 | 1 |
| BRAMY2024593 | 0.012997918 | 65991 | 5 |
| BRAMY2024711 | 0.013665617 | 139275 | 3 |
| BRAMY2024715 | 0.013152222 | 153107 | 2 |
| BRAMY2024728 | 0.001688277 | 60144 | 1 |
| BRAMY2024827 | 0.083989576 | 158591 | 8 |
| BRAMY2024849 | 0.001688277 | 175106 | 1 |
| BRAMY2024923 | 0.020081166 | 141389 | 9 |
| BRAMY2025024 | 0.001688277 | 212856 | 1 |
| BRAMY2025032 | 0.001688277 | 190156 | 1 |
| BRAMY2025121 | 0.010119342 | 165766 | 5 |
| BRAMY2025175 | 0.001688277 | 12504 | 1 |
| BRAMY2025218 | 0.001688277 | 152965 | 1 |
| BRAMY2025230 | 0.017089279 | 117181 | 4 |
| BRAMY2025272 | 0.003546391 | 115562 | 2 |
| BRAMY2025277 | 0.001688277 | 112572 | 1 |
| BRAMY2025495 | 0.001688277 | 280148 | 1 |
| BRAMY2025812 | 0.001688277 | 162669 | 1 |
| BRAMY2025996 | 0.001688277 | 230575 | 1 |
| BRAMY2026009 | 0.011267149 | 192537 | 4 |
| BRAMY2026091 | 0.001688277 | 113738 | 1 |
| BRAMY2026168 | 0.005221938 | 76858 | 3 |
| BRAMY2026300 | 0.001688277 | 104670 | 1 |
| BRAMY2026347 | 0.001688277 | 147417 | 1 |
| BRAMY2026395 | 0.001688277 | 157733 | 1 |
| BRAMY2026405 | 0.001688277 | 88728 | 1 |
| BRAMY2026533 | 0.008951281 | 154491 | 4 |
| BRAMY2026538 | 0.003376553 | 91404 | 2 |
| BRAMY2026685 | 0.067607046 | 116703 | 18 |
| BRAMY2026713 | 0.001688277 | 264346 | 1 |
| BRAMY2026778 | 0.034583959 | 148047 | 6 |
| BRAMY2026824 | 0.001688277 | 160595 | 1 |
| BRAMY2026899 | 0.001688277 | 193505 | 1 |
| BRAMY2026902 | 0.001688277 | 87919 | 1 |
| BRAMY2026904 | 0.001688277 | 73642 | 1 |
| BRAMY2026976 | 0.001688277 | 150599 | 1 |
| BRAMY2027073 | 0.001688277 | 229549 | 1 |
| BRAMY2027114 | 0.009748711 | 145186 | 3 |
| BRAMY2027140 | 0.00649736 | 171273 | 3 |
| BRAMY2027242 | 0.001688277 | 16413 | 1 |
| BRAMY2027396 | 0.001688277 | 131400 | 1 |
| BRAMY2027451 | 0.001688277 | 45694 | 1 |
| BRAMY2027461 | 0.001688277 | 28464 | 1 |
| BRAMY2027467 | 0.001688277 | 133720 | 1 |
| BRAMY2027587 | 0.012548236 | 118907 | 8 |
| BRAMY2027714 | 0.001688277 | 278718 | 1 |
| BRAMY2027717 | 0.04069848 | 105997 | 23 |
| BRAMY2027739 | 0.12450995 | 188594 | 7 |
| BRAMY2027752 | 0.001688277 | 164376 | 1 |
| BRAMY2027782 | 0.001688277 | 39225 | 1 |
| BRAMY2028072 | 0.001688277 | 274286 | 1 |
| BRAMY2028183 | 0.008919087 | 228892 | 3 |
| BRAMY2028188 | 0.001688277 | 175384 | 1 |
| BRAMY2028269 | 0.010265519 | 204213 | 4 |
| BRAMY2028282 | 0.006865285 | 48781 | 3 |
| BRAMY2028472 | 0.001688277 | 172264 | 1 |
| BRAMY2028491 | 0.004670236 | 206029 | 2 |
| BRAMY2028516 | 0.042482865 | 144522 | 5 |
| BRAMY2028565 | 0.001688277 | 273002 | 1 |
| BRAMY2028593 | 0.001688277 | 141401 | 1 |
| BRAMY2028682 | 0.007890596 | 167525 | 2 |
| BRAMY2028740 | 0.179971651 | 87730 | 48 |
| BRAMY2028783 | 0.001688277 | 255679 | 1 |
| BRAMY2028796 | 0.112720242 | 130292 | 13 |
| BRAMY2028856 | 0.001688277 | 203646 | 1 |
| BRAMY2028914 | 0.009458653 | 119461 | 3 |
| BRAMY2029204 | 0.001688277 | 225248 | 1 |
| BRAMY2029602 | 0.001688277 | 200985 | 1 |
| BRAMY2029988 | 0.01422502 | 117928 | 8 |
| BRAMY2030098 | 0.003972799 | 126180 | 3 |
| BRAMY2030109 | 0.001688277 | 143999 | 1 |
| BRAMY2030206 | 0.001688277 | 169340 | 1 |
| BRAMY2030477 | 0.001688277 | 281314 | 1 |
| BRAMY2030545 | 0.007493455 | 107263 | 2 |
| BRAMY2030607 | 0.001688277 | 155855 | 1 |
| BRAMY2030702 | 0.001688277 | 171192 | 1 |
| BRAMY2030703 | 0.001688277 | 171190 | 1 |
| BRAMY2030768 | 0.001688277 | 223983 | 1 |
| BRAMY2030799 | 0.001688277 | 171201 | 1 |
| BRAMY2030859 | 0.001688277 | 221030 | 1 |
| BRAMY2030915 | 0.001688277 | 226837 | 1 |
| BRAMY2031317 | 0.010681699 | 109314 | 7 |
| BRAMY2031377 | 0.001688277 | 163712 | 1 |
| BRAMY2031442 | 0.001688277 | 256075 | 1 |
| BRAMY2031516 | 0.022684724 | 104298 | 9 |
| BRAMY2032014 | 0.001688277 | 213503 | 1 |
| BRAMY2032087 | 0.138356563 | 5993 | 40 |
| BRAMY2032231 | 0.001688277 | 260203 | 1 |
| BRAMY2032242 | 0.001688277 | 235481 | 1 |
| BRAMY2032290 | 0.001688277 | 263255 | 1 |
| BRAMY2032311 | 0.001688277 | 37344 | 1 |
| BRAMY2032317 | 0.002783555 | 264191 | 2 |
| BRAMY2032344 | 0.001688277 | 264072 | 1 |
| BRAMY2032589 | 0.001688277 | 178171 | 1 |
| BRAMY2032678 | 0.001688277 | 195279 | 1 |
| BRAMY2032696 | 0.007890596 | 49991 | 2 |
| BRAMY2032777 | 0.001688277 | 235150 | 1 |
| BRAMY2032994 | 0.001688277 | 179152 | 1 |
| BRAMY2033003 | 0.001688277 | 184119 | 1 |
| BRAMY2033116 | 0.001688277 | 228101 | 1 |
| BRAMY2033240 | 0.001688277 | 261444 | 1 |
| BRAMY2033267 | 0.001688277 | 262038 | 1 |
| BRAMY2033316 | 0.003085432 | 32684 | 2 |
| BRAMY2033332 | 0.001688277 | 177678 | 1 |
| BRAMY2033506 | 0.001688277 | 230160 | 1 |
| BRAMY2033594 | 0.001688277 | 212078 | 1 |
| BRAMY2033895 | 0.001688277 | 212849 | 1 |
| BRAMY2033914 | 0.003363824 | 145115 | 2 |
| BRAMY2034072 | 0.001688277 | 206061 | 1 |
| BRAMY2034185 | 0.001688277 | 230205 | 1 |
| BRAMY2034187 | 0.01347517 | 202857 | 2 |
| BRAMY2034305 | 0.001688277 | 202786 | 1 |
| BRAMY2034561 | 0.001688277 | 284412 | 1 |
| BRAMY2034920 | 0.001688277 | 171583 | 1 |
| BRAMY2034993 | 0.001688277 | 171654 | 1 |
| BRAMY2035070 | 0.003085432 | 18903 | 2 |
| BRAMY2035449 | 0.001688277 | 206535 | 1 |
| BRAMY2035545 | 0.002877521 | 221477 | 2 |
| BRAMY2035606 | 0.001688277 | 281517 | 1 |
| BRAMY2035718 | 0.001688277 | 282089 | 1 |
| BRAMY2035719 | 0.01103125 | 62268 | 4 |
| BRAMY2035801 | 0.001688277 | 276513 | 1 |
| BRAMY2035869 | 0.002783555 | 1045 | 2 |
| BRAMY2035923 | 0.001688277 | 164296 | 1 |
| BRAMY2036076 | 0.015491023 | 86250 | 7 |
| BRAMY2036079 | 0.001688277 | 257310 | 1 |
| BRAMY2036254 | 0.001688277 | 142325 | 1 |
| BRAMY2036266 | 0.001688277 | 148962 | 1 |
| BRAMY2036387 | 0.012515475 | 139077 | 2 |
| BRAMY2036396 | 0.001688277 | 227369 | 1 |
| BRAMY2036459 | 0.088906521 | 164382 | 11 |
| BRAMY2036567 | 0.022417078 | 84976 | 12 |
| BRAMY2036615 | 0.001688277 | 224550 | 1 |
| BRAMY2036699 | 0.001688277 | 213126 | 1 |
| BRAMY2036913 | 0.001688277 | 168297 | 1 |
| BRAMY2036918 | 0.001688277 | 271232 | 1 |
| BRAMY2036968 | 0.001688277 | 279454 | 1 |
| BRAMY2037147 | 0.004670236 | 166258 | 2 |
| BRAMY2037328 | 0.001688277 | 188389 | 1 |
| BRAMY2037609 | 0.001688277 | 158193 | 1 |
| BRAMY2037629 | 0.001688277 | 168259 | 1 |
| BRAMY2037800 | 0.001688277 | 265412 | 1 |
| BRAMY2037823 | 0.005648346 | 84476 | 4 |
| BRAMY2037971 | 0.001688277 | 266427 | 1 |
| BRAMY2038100 | 0.001688277 | 259330 | 1 |
| BRAMY2038128 | 0.001688277 | 255693 | 1 |
| BRAMY2038317 | 0.001688277 | 220493 | 1 |
| BRAMY2038475 | 0.001688277 | 62040 | 1 |
| BRAMY2038484 | 0.003376553 | 220482 | 2 |
| BRAMY2038516 | 0.013220528 | 42714 | 4 |
| BRAMY2038832 | 0.013746414 | 28808 | 3 |
| BRAMY2038846 | 0.003565118 | 248903 | 2 |
| BRAMY2038887 | 0.001688277 | 90556 | 1 |
| BRAMY2038904 | 0.002783555 | 182255 | 2 |
| BRAMY2039287 | 0.001688277 | 210992 | 1 |
| BRAMY2039300 | 0.001688277 | 192922 | 1 |
| BRAMY2039314 | 0.001688277 | 184107 | 1 |
| BRAMY2039341 | 0.001688277 | 182222 | 1 |
| BRAMY2039630 | 0.019306977 | 126235 | 4 |
| BRAMY2039695 | 0.001688277 | 192889 | 1 |
| BRAMY2039728 | 0.004586981 | 222030 | 3 |
| BRAMY2039872 | 0.008451611 | 29553 | 4 |
| BRAMY2040051 | 0.001688277 | 277330 | 1 |
| BRAMY2040095 | 0.490173259 | 23879 | 83 |
| BRAMY2040159 | 0.001688277 | 56644 | 1 |
| BRAMY2040214 | 0.011552293 | 91006 | 7 |
| BRAMY2040478 | 0.001688277 | 278794 | 1 |
| BRAMY2040592 | 0.048118185 | 125029 | 11 |
| BRAMY2040915 | 0.001688277 | 284131 | 1 |
| BRAMY2041261 | 0.001688277 | 277177 | 1 |
| BRAMY2041347 | 0.001688277 | 210516 | 1 |
| BRAMY2041378 | 0.001688277 | 258600 | 1 |
| BRAMY2041384 | 0.001688277 | 27916 | 1 |
| BRAMY2041434 | 0.001688277 | 215608 | 1 |
| BRAMY2041507 | 0.001688277 | 200277 | 1 |
| BRAMY2041542 | 0.001688277 | 282740 | 1 |
| BRAMY2041738 | 0.001688277 | 283696 | 1 |
| BRAMY2042122 | 0.001688277 | 172570 | 1 |
| BRAMY2042131 | 0.001688277 | 146223 | 1 |
| BRAMY2042178 | 0.001688277 | 271806 | 1 |
| BRAMY2042195 | 0.005532361 | 195676 | 2 |
| BRAMY2042244 | 0.001688277 | 230001 | 1 |
| BRAMY2042549 | 0.001688277 | 211224 | 1 |
| BRAMY2042596 | 0.001688277 | 279119 | 1 |
| BRAMY2042612 | 0.001688277 | 280056 | 1 |
| BRAMY2042641 | 0.001688277 | 219803 | 1 |
| BRAMY2042711 | 0.001688277 | 199781 | 1 |
| BRAMY2042760 | 0.001688277 | 241025 | 1 |
| BRAMY2042804 | 0.002877521 | 144442 | 2 |
| BRAMY2042899 | 0.001688277 | 211083 | 1 |
| BRAMY2042918 | 0.001688277 | 178328 | 1 |
| BRAMY2043069 | 0.007890596 | 158421 | 2 |
| BRAMY2043103 | 0.001688277 | 115759 | 1 |
| BRAMY2043314 | 0.001688277 | 264884 | 1 |
| BRAMY2043345 | 0.001688277 | 163385 | 1 |
| BRAMY2044078 | 0.038215023 | 161719 | 16 |
| BRAMY2044246 | 0.001688277 | 263050 | 1 |
| BRAMY2044317 | 0.001688277 | 261445 | 1 |
| BRAMY2044349 | 0.001688277 | 259554 | 1 |
| BRAMY2044686 | 0.001688277 | 276005 | 1 |
| BRAMY2044838 | 0.005404505 | 252610 | 3 |
| BRAMY2045036 | 0.001688277 | 281392 | 1 |
| BRAMY2045299 | 0.001688277 | 148569 | 1 |
| BRAMY2045471 | 0.003376553 | 218376 | 2 |
| BRAMY2045530 | 0.017760844 | 5408 | 4 |
| BRAMY2046109 | 0.001688277 | 98014 | 1 |
| BRAMY2046478 | 0.001688277 | 276394 | 1 |
| BRAMY2046489 | 0.001688277 | 215808 | 1 |
| BRAMY2046537 | 0.001688277 | 240114 | 1 |
| BRAMY2046667 | 0.001688277 | 220865 | 1 |
| BRAMY2046742 | 0.001688277 | 195740 | 1 |
| BRAMY2046871 | 0.0050521 | 213129 | 3 |
| BRAMY2046984 | 0.001688277 | 238975 | 1 |
| BRAMY2046989 | 0.001688277 | 117879 | 1 |
| BRAMY2047169 | 0.001688277 | 251188 | 1 |
| BRAMY2047280 | 0.001688277 | 179986 | 1 |
| BRAMY2047420 | 0.165408115 | 117426 | 10 |
| BRAMY2047676 | 0.001688277 | 170182 | 1 |
| BRAMY2047692 | 0.001688277 | 150911 | 1 |
| BRAMY2047746 | 0.001688277 | 160869 | 1 |
| BRAMY2047751 | 0.001688277 | 240602 | 1 |
| BRAMY2047765 | 0.001688277 | 204252 | 1 |
| BRAMY2047884 | 0.001688277 | 145301 | 1 |
| BRAMY2047948 | 0.001688277 | 260960 | 1 |
| BRAMY3000206 | 0.001688277 | 191074 | 1 |
| BRAMY3000210 | 0.001688277 | 243176 | 1 |
| BRAMY3000213 | 0.001688277 | 208662 | 1 |
| BRAMY3000224 | 0.005865212 | 214564 | 2 |
| BRAMY3000254 | 0.001688277 | 274173 | 1 |
| BRAMY3000307 | 0.001688277 | 14805 | 1 |
| BRAMY3000692 | 0.001688277 | 238277 | 1 |
| BRAMY3001356 | 0.001688277 | 240026 | 1 |
| BRAMY3001401 | 0.001688277 | 282047 | 1 |
| BRAMY3001409 | 0.001688277 | 122846 | 1 |
| BRAMY3001794 | 0.001688277 | 278756 | 1 |
| BRAMY3002120 | 0.003085432 | 274920 | 2 |
| BRAMY3002312 | 0.001688277 | 212407 | 1 |
| BRAMY3002329 | 0.024733489 | 88376 | 15 |
| BRAMY3002458 | 0.013496638 | 50360 | 8 |
| BRAMY3002508 | 0.001688277 | 187663 | 1 |
| BRAMY3002620 | 0.002877521 | 263532 | 2 |
| BRAMY3002803 | 0.011613011 | 6275 | 6 |
| BRAMY3002805 | 0.001688277 | 187027 | 1 |
| BRAMY3002886 | 0.001688277 | 283709 | 1 |
| BRAMY3002991 | 0.042733411 | 156256 | 6 |
| BRAMY3003026 | 0.003769789 | 171950 | 2 |
| BRAMY3003109 | 0.001688277 | 235868 | 1 |
| BRAMY3003205 | 0.006964265 | 22122 | 5 |
| BRAMY3003310 | 0.003085432 | 29786 | 2 |
| BRAMY3003566 | 0.001688277 | 1038 | 1 |
| BRAMY3003723 | 0.001688277 | 273171 | 1 |
| BRAMY3003935 | 0.01069797 | 109078 | 7 |
| BRAMY3004126 | 0.003363824 | 269938 | 2 |
| BRAMY3004224 | 0.005073284 | 90383 | 3 |
| BRAMY3004364 | 0.024665517 | 167502 | 7 |
| BRAMY3004377 | 0.202027897 | 108797 | 44 |
| BRAMY3004672 | 0.001688277 | 198821 | 1 |
| BRAMY3004800 | 0.022011049 | 46315 | 13 |
| BRAMY3004900 | 0.001688277 | 195490 | 1 |
| BRAMY3004919 | 0.001688277 | 110243 | 1 |
| BRAMY3005091 | 0.008730003 | 70985 | 4 |
| BRAMY3005184 | 0.00747765 | 83059 | 4 |
| BRAMY3005656 | 0.001688277 | 260511 | 1 |
| BRAMY3005912 | 0.007823825 | 177921 | 5 |
| BRAMY3005932 | 0.002877521 | 82603 | 2 |
| BRAMY3006032 | 0.001688277 | 186399 | 1 |
| BRAMY3006297 | 0.001688277 | 253799 | 1 |
| BRAMY3006761 | 0.015262219 | 60379 | 9 |
| BRAMY3007078 | 0.003397737 | 211502 | 2 |
| BRAMY3007206 | 0.001688277 | 283003 | 1 |
| BRAMY3007311 | 0.021327119 | 144415 | 9 |
| BRAMY3007350 | 0.001688277 | 27494 | 1 |
| BRAMY3007449 | 0.001688277 | 266907 | 1 |
| BRAMY3007471 | 0.001688277 | 261690 | 1 |
| BRAMY3007609 | 0.001688277 | 256744 | 1 |
| BRAMY3007723 | 0.001688277 | 257850 | 1 |
| BRAMY3007968 | 0.001688277 | 138565 | 1 |
| BRAMY3008044 | 0.093406814 | 60946 | 38 |
| BRAMY3008088 | 0.001688277 | 277370 | 1 |
| BRAMY3008096 | 0.002877521 | 128660 | 2 |
| BRAMY3008335 | 0.009432945 | 77458 | 5 |
| BRAMY3008436 | 0.119621588 | 6885 | 51 |
| BRAMY3008466 | 0.001688277 | 249700 | 1 |
| BRAMY3008505 | 0.001688277 | 259721 | 1 |
| BRAMY3008650 | 0.001688277 | 255619 | 1 |
| BRAMY3008937 | 0.001688277 | 265634 | 1 |
| BRAMY3009158 | 0.001688277 | 265210 | 1 |
| BRAMY3009207 | 0.001688277 | 260172 | 1 |
| BRAMY3009491 | 0.005369955 | 117745 | 4 |
| BRAMY3009556 | 0.003546391 | 251503 | 2 |
| BRAMY3009782 | 0.003546391 | 185685 | 2 |
| BRAMY3009811 | 0.005107197 | 87425 | 3 |
| BRAMY3009904 | 0.001688277 | 259738 | 1 |
| BRAMY3010008 | 0.287891807 | 117489 | 14 |
| BRAMY3010264 | 0.003363824 | 234713 | 2 |
| BRAMY3010321 | 0.001688277 | 232211 | 1 |
| BRAMY3010411 | 0.001688277 | 211338 | 1 |
| BRAMY3010492 | 0.02909303 | 367 | 10 |
| BRAMY3010603 | 0.001688277 | 220417 | 1 |
| BRAMY3010654 | 0.003546391 | 87638 | 2 |
| BRAMY4000089 | 0.032925039 | 111406 | 17 |
| BRAMY4000095 | 0.011468135 | 70851 | 3 |
| BRAMY4000229 | 0.004735635 | 261921 | 3 |
| BRAMY4000277 | 0.001688277 | 260995 | 1 |
| BRAMY4000915 | 0.003397737 | 122639 | 2 |
| BRAMY4000962 | 0.001688277 | 283461 | 1 |
| BRAMY4001173 | 0.001688277 | 210285 | 1 |
| BRAMY4001200 | 0.001688277 | 201028 | 1 |
| BRAMY4001234 | 0.003085432 | 177802 | 2 |
| BRAMY4001449 | 0.001688277 | 221870 | 1 |
| BRAMY4001652 | 0.001688277 | 210095 | 1 |
| BRAMY4001863 | 0.011185159 | 109043 | 5 |
| BRAMY4001913 | 0.001688277 | 125997 | 1 |
| BRAMY4002575 | 0.001688277 | 238638 | 1 |
| BRAMY4002628 | 0.023589801 | 141596 | 11 |
| BRASW1000053 | 0.632911392 | 218746 | 1 |
| BRASW1000125 | 0.638952958 | 187197 | 2 |
| BRAWH1000001 | 0.050352377 | 61329 | 21 |
| BRAWH1000002 | 0.001675547 | 66770 | 1 |
| BRAWH1000007 | 0.018646974 | 6612 | 5 |
| BRAWH1000037 | 0.091889673 | 54702 | 24 |
| BRAWH1000040 | 0.24351653 | 11924 | 49 |
| BRAWH1000045 | 0.167388547 | 79272 | 64 |
| BRAWH1000083 | 0.036981447 | 9715 | 10 |
| BRAWH1000093 | 0.36328603 | 48128 | 52 |
| BRAWH1000094 | 0.039797585 | 39402 | 14 |
| BRAWH1000116 | 0.001675547 | 43181 | 1 |
| BRAWH1000127 | 0.052416584 | 37367 | 19 |
| BRAWH1000130 | 0.001675547 | 67297 | 1 |
| BRAWH1000157 | 0.001675547 | 64749 | 1 |
| BRAWH1000162 | 0.091810869 | 30660 | 32 |
| BRAWH1000164 | 0.001675547 | 75232 | 1 |
| BRAWH1000167 | 0.092619948 | 51003 | 33 |
| BRAWH1000168 | 0.001675547 | 128265 | 1 |
| BRAWH1000174 | 0.058484446 | 75043 | 25 |
| BRAWH1000180 | 0.001675547 | 40597 | 1 |
| BRAWH1000369 | 0.001675547 | 165088 | 1 |
| BRAWH2000034 | 0.001675547 | 273204 | 1 |
| BRAWH2000048 | 0.001675547 | 259623 | 1 |
| BRAWH2000071 | 0.001675547 | 69516 | 1 |
| BRAWH2000082 | 0.436810495 | 55088 | 158 |
| BRAWH2000093 | 0.001675547 | 228500 | 1 |
| BRAWH2000109 | 0.001675547 | 136811 | 1 |
| BRAWH2000131 | 0.007729915 | 206593 | 2 |
| BRAWH2000142 | 0.010996329 | 117660 | 4 |
| BRAWH2000169 | 0.015895751 | 114160 | 3 |
| BRAWH2000177 | 0.020956106 | 23588 | 7 |
| BRAWH2000218 | 0.003533661 | 45764 | 2 |
| BRAWH2000232 | 0.001675547 | 122780 | 1 |
| BRAWH2000248 | 0.001675547 | 169569 | 1 |
| BRAWH2000256 | 0.001675547 | 245732 | 1 |
| BRAWH2000274 | 0.08487909 | 126191 | 44 |
| BRAWH2000323 | 0.001675547 | 106322 | 1 |
| BRAWH2000353 | 0.002864792 | 87260 | 2 |
| BRAWH2000370 | 0.001675547 | 195714 | 1 |
| BRAWH2000409 | 0.001675547 | 221617 | 1 |
| BRAWH2000417 | 0.271182045 | 85514 | 92 |
| BRAWH2000443 | 0.029573251 | 105194 | 11 |
| BRAWH2000460 | 0.274541089 | 10111 | 76 |
| BRAWH2000471 | 0.001675547 | 267212 | 1 |
| BRAWH2000476 | 0.006952582 | 93782 | 4 |
| BRAWH2000482 | 0.110393093 | 13399 | 26 |
| BRAWH2000488 | 0.016619307 | 133749 | 6 |
| BRAWH2000494 | 0.018430826 | 140072 | 6 |
| BRAWH2000500 | 0.002864792 | 192393 | 2 |
| BRAWH2000503 | 0.008076427 | 131331 | 4 |
| BRAWH2000522 | 0.09244356 | 39525 | 18 |
| BRAWH2000554 | 0.001675547 | 265277 | 1 |
| BRAWH2000588 | 0.133555816 | 79239 | 29 |
| BRAWH2000595 | 0.013436102 | 119870 | 2 |
| BRAWH2000633 | 0.126392578 | 31367 | 18 |
| BRAWH2000636 | 0.317081402 | 80819 | 122 |
| BRAWH2000643 | 0.001675547 | 231904 | 1 |
| BRAWH2000651 | 0.001675547 | 144148 | 1 |
| BRAWH2000682 | 0.001675547 | 95684 | 1 |
| BRAWH2000686 | 0.137074917 | 38929 | 51 |
| BRAWH2000697 | 0.165643883 | 125094 | 44 |
| BRAWH2000752 | 0.001675547 | 130443 | 1 |
| BRAWH2000839 | 0.407966855 | 38528 | 119 |
| BRAWH2000859 | 0.003552389 | 214221 | 2 |
| BRAWH2000866 | 0.001675547 | 255369 | 1 |
| BRAWH2000892 | 0.001675547 | 219414 | 1 |
| BRAWH2000901 | 0.001675547 | 280013 | 1 |
| BRAWH2000924 | 0.583840525 | 60800 | 159 |
| BRAWH2000926 | 0.039727297 | 109375 | 2 |
| BRAWH2000944 | 0.032118304 | 103162 | 11 |
| BRAWH2000984 | 0.001675547 | 122613 | 1 |
| BRAWH2001019 | 0.073467058 | 4310 | 21 |
| BRAWH2001037 | 0.041779669 | 170115 | 15 |
| BRAWH2001052 | 0.001675547 | 270537 | 1 |
| BRAWH2001092 | 0.953353157 | 65081 | 148 |
| BRAWH2001103 | 0.025497494 | 217133 | 5 |
| BRAWH2001129 | 0.003552389 | 143882 | 2 |
| BRAWH2001137 | 0.001675547 | 178414 | 1 |
| BRAWH2001141 | 0.032970596 | 75233 | 13 |
| BRAWH2001159 | 0.001675547 | 80868 | 1 |
| BRAWH2001166 | 0.05308009 | 96586 | 15 |
| BRAWH2001171 | 0.271297828 | 45061 | 101 |
| BRAWH2001186 | 0.058806701 | 67320 | 6 |
| BRAWH2001203 | 0.001675547 | 44253 | 1 |
| BRAWH2001238 | 0.013858127 | 121814 | 7 |
| BRAWH2001239 | 0.022965193 | 151246 | 5 |
| BRAWH2001255 | 0.001675547 | 239935 | 1 |
| BRAWH2001321 | 0.195185305 | 40551 | 78 |
| BRAWH2001355 | 0.001675547 | 104096 | 1 |
| BRAWH2001378 | 0.001675547 | 128073 | 1 |
| BRAWH2001395 | 0.398667125 | 6827 | 161 |
| BRAWH2001406 | 0.049495121 | 106201 | 7 |
| BRAWH2001412 | 0.011143487 | 197652 | 4 |
| BRAWH2001418 | 0.064965414 | 99926 | 17 |
| BRAWH2001424 | 0.0329093 | 72850 | 7 |
| BRAWH2001436 | 0.001675547 | 260418 | 1 |
| BRAWH2001438 | 0.001675547 | 260234 | 1 |
| BRAWH2001439 | 0.162847305 | 86450 | 59 |
| BRAWH2001459 | 0.068509233 | 76563 | 29 |
| BRAWH2001461 | 0.047136806 | 76709 | 6 |
| BRAWH2001484 | 0.015770289 | 125985 | 5 |
| BRAWH2001492 | 0.001675547 | 139006 | 1 |
| BRAWH2001502 | 1.267697555 | 27207 | 213 |
| BRAWH2001503 | 0.001675547 | 285113 | 1 |
| BRAWH2001519 | 0.001675547 | 275735 | 1 |
| BRAWH2001530 | 0.021950701 | 131534 | 6 |
| BRAWH2001535 | 0.339353143 | 83177 | 102 |
| BRAWH2001589 | 0.003351094 | 222414 | 2 |
| BRAWH2001662 | 0.03644306 | 104160 | 11 |
| BRAWH2001666 | 0.003351094 | 145143 | 2 |
| BRAWH2001671 | 0.044064981 | 135772 | 15 |
| BRAWH2001679 | 0.001675547 | 43070 | 1 |
| BRAWH2001686 | 0.001675547 | 208551 | 1 |
| BRAWH2001701 | 0.003351094 | 185782 | 2 |
| BRAWH2001842 | 0.001675547 | 177855 | 1 |
| BRAWH2001862 | 0.049891555 | 50559 | 2 |
| BRAWH2001873 | 0.001675547 | 199204 | 1 |
| BRAWH2001940 | 0.004480285 | 104286 | 3 |
| BRAWH2001973 | 0.004480285 | 84575 | 3 |
| BRAWH2002047 | 0.002864792 | 163894 | 2 |
| BRAWH2002060 | 0.002864792 | 110855 | 2 |
| BRAWH2002062 | 0.003072702 | 168588 | 2 |
| BRAWH2002125 | 0.001675547 | 33389 | 1 |
| BRAWH2002191 | 0.001675547 | 155312 | 1 |
| BRAWH2002318 | 0.006278681 | 6005 | 3 |
| BRAWH2002333 | 0.001675547 | 90149 | 1 |
| BRAWH2002352 | 0.001675547 | 182411 | 1 |
| BRAWH2002383 | 0.001675547 | 203207 | 1 |
| BRAWH2002549 | 0.009299599 | 168333 | 3 |
| BRAWH2002560 | 0.051925901 | 24106 | 18 |
| BRAWH2002601 | 0.033688345 | 132020 | 13 |
| BRAWH2002623 | 0.001675547 | 195435 | 1 |
| BRAWH2002630 | 0.001675547 | 205437 | 1 |
| BRAWH2002635 | 0.001675547 | 167440 | 1 |
| BRAWH2002662 | 0.001675547 | 198736 | 1 |
| BRAWH2002679 | 0.017698113 | 38968 | 4 |
| BRAWH2002725 | 0.001675547 | 262605 | 1 |
| BRAWH2002761 | 0.001675547 | 276467 | 1 |
| BRAWH2002811 | 0.019428385 | 140433 | 8 |
| BRAWH2002861 | 0.001675547 | 144857 | 1 |
| BRAWH2002911 | 0.007041727 | 109926 | 3 |
| BRAWH2002963 | 0.014042972 | 145002 | 3 |
| BRAWH2002967 | 0.003757059 | 143402 | 2 |
| BRAWH2002976 | 0.001675547 | 34620 | 1 |
| BRAWH2003000 | 0.002864792 | 121508 | 2 |
| BRAWH2003025 | 0.055260238 | 40082 | 12 |
| BRAWH2003135 | 0.004796353 | 75904 | 2 |
| BRAWH2003240 | 0.021350649 | 80779 | 4 |
| BRAWH2003307 | 0.001675547 | 227712 | 1 |
| BRAWH2003355 | 0.007729915 | 232894 | 2 |
| BRAWH2003366 | 0.001675547 | 173285 | 1 |
| BRAWH2003662 | 0.027035576 | 142685 | 13 |
| BRAWH2003689 | 0.003552389 | 129639 | 2 |
| BRAWH2003693 | 0.048975235 | 55559 | 16 |
| BRAWH2003818 | 0.05536172 | 39487 | 11 |
| BRAWH2003832 | 0.001675547 | 168336 | 1 |
| BRAWH2003948 | 0.001675547 | 46769 | 1 |
| BRAWH2003964 | 0.003385007 | 48760 | 2 |
| BRAWH2004044 | 0.001675547 | 161936 | 1 |
| BRAWH2004068 | 0.001675547 | 60350 | 1 |
| BRAWH2004078 | 0.031797678 | 215344 | 7 |
| BRAWH2004095 | 0.141739371 | 91975 | 53 |
| BRAWH2004136 | 0.010530678 | 204838 | 4 |
| BRAWH2004217 | 0.003072702 | 79530 | 2 |
| BRAWH2004580 | 0.009214721 | 149244 | 4 |
| BRAWH2004731 | 0.001675547 | 172111 | 1 |
| BRAWH2004779 | 0.003363824 | 172110 | 2 |
| BRAWH2004842 | 0.042036641 | 125533 | 22 |
| BRAWH2004884 | 0.001675547 | 148802 | 1 |
| BRAWH2004923 | 0.001675547 | 251058 | 1 |
| BRAWH2005008 | 0.010148342 | 92454 | 5 |
| BRAWH2005068 | 0.416566647 | 145643 | 23 |
| BRAWH2005074 | 0.004771043 | 99951 | 2 |
| BRAWH2005225 | 0.028905305 | 140646 | 10 |
| BRAWH2005315 | 0.001675547 | 117854 | 1 |
| BRAWH2005517 | 0.003757059 | 47957 | 2 |
| BRAWH2005524 | 0.001675547 | 150027 | 1 |
| BRAWH2005533 | 0.001675547 | 275384 | 1 |
| BRAWH2005578 | 0.012503372 | 158656 | 7 |
| BRAWH2005661 | 0.104812185 | 103899 | 29 |
| BRAWH2005812 | 0.001675547 | 92953 | 1 |
| BRAWH2005974 | 0.001675547 | 154422 | 1 |
| BRAWH2005998 | 0.005519631 | 165543 | 2 |
| BRAWH2006044 | 0.009523565 | 68176 | 4 |
| BRAWH2006083 | 0.001675547 | 144284 | 1 |
| BRAWH2006207 | 0.012945233 | 150670 | 4 |
| BRAWH2006301 | 0.002864792 | 102326 | 2 |
| BRAWH2006395 | 0.01346244 | 245071 | 2 |
| BRAWH2006405 | 0.012911511 | 98799 | 4 |
| BRAWH2006450 | 0.033369032 | 128986 | 13 |
| BRAWH2006493 | 0.032942226 | 142646 | 13 |
| BRAWH2006526 | 0.003072702 | 192799 | 2 |
| BRAWH2006543 | 0.038198657 | 124033 | 19 |
| BRAWH2006622 | 0.010126419 | 112961 | 4 |
| BRAWH2006760 | 0.001675547 | 70390 | 1 |
| BRAWH2006960 | 0.001675547 | 42476 | 1 |
| BRAWH2006989 | 0.006708875 | 161487 | 3 |
| BRAWH2006996 | 0.049260868 | 113942 | 11 |
| BRAWH2007069 | 0.001675547 | 173997 | 1 |
| BRAWH2007308 | 0.003351094 | 108276 | 2 |
| BRAWH2007406 | 0.001675547 | 196415 | 1 |
| BRAWH2007550 | 0.001675547 | 161434 | 1 |
| BRAWH2007570 | 0.001675547 | 78177 | 1 |
| BRAWH2007591 | 0.0533923 | 82609 | 32 |
| BRAWH2007605 | 0.081686424 | 109624 | 37 |
| BRAWH2007658 | 0.014419301 | 158117 | 6 |
| BRAWH2007664 | 0.016375786 | 30706 | 5 |
| BRAWH2007800 | 0.071331673 | 110609 | 8 |
| BRAWH2007808 | 0.004469858 | 158139 | 3 |
| BRAWH2007825 | 0.002864792 | 90359 | 2 |
| BRAWH2007862 | 0.001675547 | 156457 | 1 |
| BRAWH2007890 | 0.001675547 | 33980 | 1 |
| BRAWH2008058 | 0.007152616 | 157433 | 4 |
| BRAWH2008128 | 0.007588159 | 141840 | 2 |
| BRAWH2008219 | 0.011627806 | 2127 | 4 |
| BRAWH2008255 | 0.039660178 | 118528 | 7 |
| BRAWH2008292 | 0.004590404 | 23747 | 2 |
| BRAWH2008297 | 0.001675547 | 116427 | 1 |
| BRAWH2008366 | 0.013139493 | 32578 | 2 |
| BRAWH2008380 | 0.001675547 | 221636 | 1 |
| BRAWH2008540 | 0.003072702 | 168580 | 2 |
| BRAWH2008577 | 0.047128784 | 119324 | 3 |
| BRAWH2008706 | 0.058270128 | 83391 | 25 |
| BRAWH2008790 | 0.001675547 | 169218 | 1 |
| BRAWH2008903 | 0.039628131 | 86047 | 18 |
| BRAWH2008956 | 0.010761144 | 116317 | 6 |
| BRAWH2008993 | 0.003363824 | 110730 | 2 |
| BRAWH2009112 | 0.001675547 | 195764 | 1 |
| BRAWH2009227 | 0.010601532 | 157980 | 3 |
| BRAWH2009238 | 0.03214183 | 66051 | 20 |
| BRAWH2009261 | 0.001675547 | 134582 | 1 |
| BRAWH2009263 | 0.001675547 | 190993 | 1 |
| BRAWH2009298 | 0.001675547 | 180316 | 1 |
| BRAWH2009304 | 0.042719991 | 71017 | 11 |
| BRAWH2009307 | 0.084153786 | 134573 | 13 |
| BRAWH2009360 | 0.019362756 | 149798 | 4 |
| BRAWH2009393 | 0.001675547 | 126256 | 1 |
| BRAWH2009485 | 0.001675547 | 72162 | 1 |
| BRAWH2009490 | 0.049523133 | 160072 | 3 |
| BRAWH2009558 | 0.001675547 | 152234 | 1 |
| BRAWH2009590 | 0.001675547 | 103984 | 1 |
| BRAWH2009626 | 0.003757059 | 157183 | 2 |
| BRAWH2009678 | 0.007877867 | 206807 | 2 |
| BRAWH2009695 | 0.006262528 | 94573 | 4 |
| BRAWH2009907 | 0.001675547 | 143767 | 1 |
| BRAWH2010000 | 0.008938551 | 110619 | 4 |
| BRAWH2010069 | 0.001675547 | 133838 | 1 |
| BRAWH2010084 | 0.001675547 | 169125 | 1 |
| BRAWH2010136 | 0.006345783 | 148720 | 3 |
| BRAWH2010318 | 0.001675547 | 161941 | 1 |
| BRAWH2010329 | 0.066964303 | 84969 | 40 |
| BRAWH2010354 | 0.012911195 | 134398 | 4 |
| BRAWH2010364 | 0.009723566 | 75150 | 7 |
| BRAWH2010536 | 0.011384285 | 150103 | 2 |
| BRAWH2010552 | 0.002864792 | 161059 | 2 |
| BRAWH2010584 | 0.005519631 | 174573 | 2 |
| BRAWH2010618 | 0.001675547 | 162035 | 1 |
| BRAWH2010619 | 0.001675547 | 160987 | 1 |
| BRAWH2010754 | 0.01497874 | 146640 | 4 |
| BRAWH2010980 | 0.023711758 | 8032 | 4 |
| BRAWH2011066 | 0.007911905 | 97161 | 2 |
| BRAWH2011079 | 0.001675547 | 34587 | 1 |
| BRAWH2011096 | 0.003072702 | 41070 | 2 |
| BRAWH2011155 | 0.001675547 | 53735 | 1 |
| BRAWH2011166 | 0.001675547 | 169015 | 1 |
| BRAWH2011204 | 0.003363824 | 133840 | 2 |
| BRAWH2011286 | 0.018775481 | 169769 | 3 |
| BRAWH2011294 | 0.001675547 | 84560 | 1 |
| BRAWH2011343 | 0.023885229 | 3696 | 12 |
| BRAWH2011400 | 0.001675547 | 76793 | 1 |
| BRAWH2011762 | 0.001675547 | 160760 | 1 |
| BRAWH2011795 | 0.001675547 | 159903 | 1 |
| BRAWH2011796 | 0.001675547 | 125184 | 1 |
| BRAWH2011812 | 0.012371582 | 138479 | 4 |
| BRAWH2011823 | 0.00774793 | 81130 | 2 |
| BRAWH2011860 | 0.001675547 | 15493 | 1 |
| BRAWH2011920 | 0.008843072 | 102818 | 5 |
| BRAWH2011958 | 0.001675547 | 145201 | 1 |
| BRAWH2012054 | 0.001675547 | 274598 | 1 |
| BRAWH2012077 | 0.013373031 | 108446 | 3 |
| BRAWH2012161 | 0.043517163 | 109813 | 4 |
| BRAWH2012162 | 0.009293218 | 134272 | 3 |
| BRAWH2012164 | 0.0080418 | 127327 | 4 |
| BRAWH2012258 | 0.001675547 | 149606 | 1 |
| BRAWH2012277 | 0.005852482 | 162426 | 2 |
| BRAWH2012326 | 0.001675547 | 132676 | 1 |
| BRAWH2012538 | 0.033030933 | 156117 | 6 |
| BRAWH2012540 | 0.001675547 | 190796 | 1 |
| BRAWH2012619 | 0.001675547 | 206260 | 1 |
| BRAWH2012698 | 0.008738778 | 81784 | 5 |
| BRAWH2012749 | 0.001675547 | 150056 | 1 |
| BRAWH2012827 | 0.018631453 | 156309 | 5 |
| BRAWH2012866 | 0.078824191 | 92308 | 33 |
| BRAWH2012963 | 0.001675547 | 156447 | 1 |
| BRAWH2013047 | 0.00891916 | 168647 | 3 |
| BRAWH2013219 | 0.017296059 | 150185 | 5 |
| BRAWH2013294 | 0.016992457 | 82020 | 8 |
| BRAWH2013748 | 0.062780603 | 140432 | 10 |
| BRAWH2013866 | 0.001675547 | 184419 | 1 |
| BRAWH2013871 | 0.004469858 | 121957 | 3 |
| BRAWH2013914 | 0.003533661 | 169064 | 2 |
| BRAWH2013941 | 0.001675547 | 117299 | 1 |
| BRAWH2013955 | 0.001675547 | 200899 | 1 |
| BRAWH2014053 | 0.00721534 | 49117 | 5 |
| BRAWH2014188 | 0.234037348 | 89493 | 75 |
| BRAWH2014234 | 0.001675547 | 169657 | 1 |
| BRAWH2014379 | 0.008071523 | 156404 | 4 |
| BRAWH2014414 | 0.018757788 | 144394 | 11 |
| BRAWH2014473 | 0.003673948 | 107630 | 2 |
| BRAWH2014511 | 0.038995427 | 74990 | 11 |
| BRAWH2014645 | 0.158504425 | 5893 | 47 |
| BRAWH2014662 | 0.010795289 | 130749 | 4 |
| BRAWH2014717 | 0.001675547 | 130227 | 1 |
| BRAWH2014729 | 0.001675547 | 135718 | 1 |
| BRAWH2014876 | 0.015999447 | 79596 | 8 |
| BRAWH2014934 | 0.004657506 | 196562 | 2 |
| BRAWH2014954 | 0.008594375 | 64398 | 6 |
| BRAWH2015247 | 0.016049487 | 51796 | 6 |
| BRAWH2015349 | 0.015234123 | 133657 | 4 |
| BRAWH2015375 | 0.001675547 | 73565 | 1 |
| BRAWH2015385 | 0.002864792 | 145887 | 2 |
| BRAWH2015595 | 0.001675547 | 168501 | 1 |
| BRAWH2015728 | 0.003363824 | 232459 | 2 |
| BRAWH2015853 | 0.001675547 | 191340 | 1 |
| BRAWH2015866 | 0.001675547 | 281697 | 1 |
| BRAWH2016028 | 0.003363824 | 162496 | 2 |
| BRAWH2016106 | 0.004590404 | 55835 | 2 |
| BRAWH2016160 | 0.001675547 | 80028 | 1 |
| BRAWH2016166 | 0.022155807 | 94358 | 12 |
| BRAWH2016209 | 0.020172424 | 184569 | 6 |
| BRAWH2016221 | 0.003533661 | 213257 | 2 |
| BRAWH2016223 | 0.001675547 | 201440 | 1 |
| BRAWH2016269 | 0.127095772 | 109168 | 15 |
| BRAWH2016305 | 0.038891773 | 244464 | 2 |
| BRAWH2016439 | 0.001675547 | 88084 | 1 |
| BRAWH2016446 | 0.019259288 | 204380 | 4 |
| BRAWH2016514 | 0.004782163 | 234421 | 3 |
| BRAWH2016562 | 0.003385007 | 132251 | 2 |
| BRAWH2016655 | 0.001675547 | 22329 | 1 |
| BRAWH2016679 | 0.01738912 | 187261 | 3 |
| BRAWH2016702 | 0.004540339 | 145426 | 3 |
| BRAWH2016724 | 0.004771043 | 155420 | 2 |
| BRAWH2016785 | 0.003351094 | 81447 | 2 |
| BRAWH2016792 | 0.003351094 | 86017 | 2 |
| BRAWH2016800 | 0.001675547 | 274840 | 1 |
| BRAWH2017002 | 0.0673329 | 57826 | 23 |
| BRAWH2017103 | 0.001675547 | 224531 | 1 |
| BRAWH2017250 | 0.001675547 | 127741 | 1 |
| BRAWH2017294 | 0.003533661 | 106107 | 2 |
| BRAWH2017304 | 0.01009072 | 137379 | 5 |
| BRAWH2017305 | 0.001675547 | 198548 | 1 |
| BRAWH2017379 | 0.011027116 | 161551 | 4 |
| BRAWH2017407 | 0.001675547 | 10980 | 1 |
| BRAWH2017433 | 0.003351094 | 138889 | 2 |
| BRAWH2017523 | 0.001675547 | 152193 | 1 |
| BRAWH2017534 | 0.001675547 | 185738 | 1 |
| BRAWH2017542 | 0.004771043 | 161709 | 2 |
| BRAWH2017635 | 0.001675547 | 15543 | 1 |
| BRAWH2017685 | 0.330158685 | 120529 | 61 |
| BRAWH2017913 | 0.001675547 | 155897 | 1 |
| BRAWH2018206 | 0.01516167 | 101278 | 6 |
| BRAWH2018267 | 0.008107913 | 143028 | 5 |
| BRAWH2018282 | 0.001675547 | 75552 | 1 |
| BRAWH2018317 | 0.001675547 | 195763 | 1 |
| BRAWH2018506 | 0.001675547 | 231106 | 1 |
| BRAWH2018514 | 0.001675547 | 221793 | 1 |
| BRAWH2018526 | 0.544470089 | 129133 | 44 |
| BRAWH2018527 | 0.019684242 | 185060 | 3 |
| BRAWH2018601 | 0.001675547 | 196414 | 1 |
| BRAWH2018729 | 0.056231978 | 123237 | 5 |
| BRAWH2018745 | 0.003673948 | 48669 | 2 |
| BRAWH2018875 | 0.012648252 | 84089 | 8 |
| BRAWH2019053 | 0.001675547 | 89882 | 1 |
| BRAWH2019055 | 0.001675547 | 98551 | 1 |
| BRAWH2019198 | 0.001675547 | 77683 | 1 |
| BRAWH3000078 | 0.001675547 | 88230 | 1 |
| BRAWH3000088 | 0.144845796 | 124777 | 54 |
| BRAWH3000100 | 0.001675547 | 279359 | 1 |
| BRAWH3000166 | 0.001675547 | 242460 | 1 |
| BRAWH3000314 | 0.007025042 | 166457 | 4 |
| BRAWH3000329 | 0.001675547 | 216881 | 1 |
| BRAWH3000410 | 0.001675547 | 272484 | 1 |
| BRAWH3000446 | 0.001675547 | 205765 | 1 |
| BRAWH3000491 | 0.001675547 | 281787 | 1 |
| BRAWH3000604 | 0.003072702 | 233470 | 2 |
| BRAWH3000884 | 0.001675547 | 269906 | 1 |
| BRAWH3001053 | 0.003351094 | 179568 | 2 |
| BRAWH3001319 | 0.006138789 | 183216 | 4 |
| BRAWH3001326 | 0.003673948 | 232595 | 2 |
| BRAWH3001475 | 0.001675547 | 257569 | 1 |
| BRAWH3001636 | 0.001675547 | 262464 | 1 |
| BRAWH3001638 | 0.001675547 | 282938 | 1 |
| BRAWH3001712 | 0.003385007 | 270855 | 2 |
| BRAWH3001760 | 0.001675547 | 99303 | 1 |
| BRAWH3001783 | 0.001675547 | 143864 | 1 |
| BRAWH3001833 | 0.001675547 | 261258 | 1 |
| BRAWH3001879 | 0.001675547 | 255948 | 1 |
| BRAWH3001891 | 0.009702382 | 179526 | 7 |
| BRAWH3001918 | 0.012066426 | 89585 | 8 |
| BRAWH3002433 | 0.003533661 | 234665 | 2 |
| BRAWH3002467 | 0.009180808 | 191510 | 4 |
| BRAWH3002513 | 0.001675547 | 517 | 1 |
| BRAWH3002574 | 0.025343997 | 69483 | 8 |
| BRAWH3002600 | 0.004553068 | 135831 | 3 |
| BRAWH3002819 | 0.001675547 | 259427 | 1 |
| BRAWH3002821 | 0.007632302 | 166780 | 4 |
| BRAWH3002853 | 0.226526584 | 32608 | 60 |
| BRAWH3003019 | 0.001675547 | 238681 | 1 |
| BRAWH3003136 | 0.001675547 | 228170 | 1 |
| BRAWH3003244 | 0.001675547 | 238643 | 1 |
| BRAWH3003343 | 0.001675547 | 247864 | 1 |
| BRAWH3003411 | 0.007240683 | 190039 | 5 |
| BRAWH3003522 | 0.001675547 | 248808 | 1 |
| BRAWH3003555 | 0.022004007 | 42729 | 7 |
| BRAWH3003573 | 0.008310618 | 65784 | 6 |
| BRAWH3003598 | 0.001675547 | 242182 | 1 |
| BRAWH3003727 | 0.016663179 | 116935 | 10 |
| BRAWH3003801 | 0.003351094 | 131635 | 2 |
| BRAWH3003975 | 0.003351094 | 199152 | 2 |
| BRAWH3003992 | 0.011552401 | 131464 | 5 |
| BRAWH3004222 | 0.003757059 | 194775 | 2 |
| BRAWH3004335 | 0.001675547 | 263186 | 1 |
| BRAWH3004350 | 0.010106008 | 114768 | 6 |
| BRAWH3004453 | 0.001675547 | 261011 | 1 |
| BRAWH3004530 | 0.009498452 | 54899 | 6 |
| BRAWH3004666 | 0.003385007 | 244416 | 2 |
| BRAWH3005037 | 0.001675547 | 248294 | 1 |
| BRAWH3005047 | 0.001675547 | 258477 | 1 |
| BRAWH3005132 | 0.003363824 | 188326 | 2 |
| BRAWH3005146 | 0.033092275 | 69861 | 13 |
| BRAWH3005292 | 0.001675547 | 282073 | 1 |
| BRAWH3005300 | 0.003552389 | 82196 | 2 |
| BRAWH3005422 | 0.001675547 | 279719 | 1 |
| BRAWH3005534 | 0.001675547 | 280748 | 1 |
| BRAWH3005886 | 0.001675547 | 261047 | 1 |
| BRAWH3005892 | 0.001675547 | 280937 | 1 |
| BRAWH3005896 | 0.001675547 | 256349 | 1 |
| BRAWH3005912 | 0.001675547 | 271411 | 1 |
| BRAWH3005981 | 0.00571383 | 277327 | 2 |
| BRAWH3006547 | 0.001675547 | 276488 | 1 |
| BRAWH3006548 | 0.011796656 | 148518 | 7 |
| BRAWH3006761 | 0.007878022 | 166828 | 5 |
| BRAWH3006792 | 0.003385007 | 246720 | 2 |
| BRAWH3007129 | 0.017027073 | 160039 | 8 |
| BRAWH3007221 | 0.003351094 | 235246 | 2 |
| BRAWH3007441 | 0.001675547 | 264171 | 1 |
| BRAWH3007455 | 0.001675547 | 251894 | 1 |
| BRAWH3007506 | 0.001675547 | 233101 | 1 |
| BRAWH3007592 | 0.093440803 | 64972 | 30 |
| BRAWH3007726 | 0.006145405 | 217388 | 4 |
| BRAWH3007783 | 0.001675547 | 279927 | 1 |
| BRAWH3008167 | 0.001675547 | 138566 | 1 |
| BRAWH3008341 | 0.001675547 | 200684 | 1 |
| BRAWH3008559 | 0.001675547 | 283607 | 1 |
| BRAWH3008697 | 0.003351094 | 86255 | 2 |
| BRAWH3008867 | 0.001675547 | 254090 | 1 |
| BRAWH3008931 | 0.048377823 | 106919 | 17 |
| BRAWH3009013 | 0.02559117 | 50260 | 11 |
| BRAWH3009017 | 0.001675547 | 223932 | 1 |
| BRAWH3009285 | 0.004540339 | 119388 | 3 |
| BRAWH3009297 | 0.002864792 | 227623 | 2 |
| BRAWH3009546 | 0.007108153 | 133866 | 4 |
| BRAWH3009961 | 0.003533661 | 81921 | 2 |
| BRAWH3010461 | 0.081558458 | 71514 | 41 |
| BRAWH3010560 | 0.001675547 | 164700 | 1 |
| BRAWH3010602 | 0.001675547 | 158258 | 1 |
| BRAWH3010657 | 0.001675547 | 114193 | 1 |
| BRAWH3010726 | 0.001675547 | 112837 | 1 |
| BRAWH3010833 | 0.001675547 | 237791 | 1 |
| BRAWH3011101 | 0.001675547 | 184506 | 1 |
| BRAWH3011331 | 0.001675547 | 207193 | 1 |
| BRAWH3011402 | 0.001675547 | 145263 | 1 |
| BRAWH3011577 | 0.001675547 | 248955 | 1 |
| BRAWH3011623 | 0.001675547 | 251784 | 1 |
| BRAWH3011637 | 0.00542923 | 122745 | 3 |
| BRAWH3011685 | 0.001675547 | 237702 | 1 |
| BRAWH3011907 | 0.003533661 | 207282 | 2 |
| BRAWH3011929 | 0.001675547 | 277704 | 1 |
| BRAWH3012005 | 0.001675547 | 231751 | 1 |
| BRAWH3012662 | 0.014796535 | 99528 | 6 |
| BRAWH3012779 | 0.001675547 | 282146 | 1 |
| BRAWH3013009 | 0.001675547 | 238671 | 1 |
| BRAWH3013049 | 0.001675547 | 228700 | 1 |
| BRAWH3013197 | 0.001675547 | 265568 | 1 |
| BRAWH3013264 | 0.003351094 | 150921 | 2 |
| BRAWH3013508 | 0.001675547 | 283882 | 1 |
| BRAWH3014609 | 0.003757059 | 160705 | 2 |
| BRAWH3014639 | 0.001675547 | 274081 | 1 |
| BRAWH3015017 | 0.001675547 | 102597 | 1 |
| BRAWH3015175 | 0.001675547 | 256586 | 1 |
| BRAWH3015260 | 0.001675547 | 177813 | 1 |
| BRAWH3015610 | 0.001675547 | 262371 | 1 |
| BRAWH3015825 | 0.001675547 | 48227 | 1 |
| BRAWH3016123 | 0.001675547 | 267069 | 1 |
| BRAWH3016271 | 0.031064887 | 120891 | 8 |
| BRAWH3016715 | 0.001675547 | 84691 | 1 |
| BRAWH3017180 | 0.005930878 | 82580 | 4 |
| BRAWH3017259 | 0.023943624 | 34784 | 8 |
| BRAWH3017260 | 0.003351094 | 83107 | 2 |
| BRAWH3017477 | 0.001675547 | 268033 | 1 |
| BRAWH3017537 | 0.001675547 | 267063 | 1 |
| BRAWH3017980 | 0.003363824 | 160032 | 2 |
| BRAWH3018063 | 0.06348498 | 144822 | 20 |
| BRAWH3018326 | 0.013764262 | 145370 | 4 |
| BRAWH3018369 | 0.001675547 | 217040 | 1 |
| BRAWH3018548 | 0.001675547 | 164946 | 1 |
| BRAWH3018969 | 0.001675547 | 60794 | 1 |
| BRAWH3019026 | 0.001675547 | 97288 | 1 |
| BRAWH3019529 | 0.001675547 | 257347 | 1 |
| BRAWH3019594 | 0.001675547 | 210334 | 1 |
| BRAWH3019629 | 0.001675547 | 276435 | 1 |
| BRAWH3019820 | 0.001675547 | 268523 | 1 |
| BRAWH3020200 | 0.001675547 | 81634 | 1 |
| BRAWH3020318 | 0.001675547 | 84995 | 1 |
| BRAWH3020486 | 0.001675547 | 213436 | 1 |
| BRAWH3020884 | 0.001675547 | 23422 | 1 |
| BRAWH3020928 | 0.003385007 | 152654 | 2 |
| BRAWH3021012 | 0.001675547 | 207349 | 1 |
| BRAWH3021545 | 0.04592879 | 50396 | 22 |
| BRAWH3021574 | 0.035651665 | 61311 | 13 |
| BRAWH3021580 | 0.003351094 | 74031 | 2 |
| BRAWH3021641 | 0.001675547 | 110504 | 1 |
| BRAWH3021643 | 0.001675547 | 89504 | 1 |
| BRAWH3021724 | 0.003351094 | 81943 | 2 |
| BRAWH3022347 | 0.001675547 | 213075 | 1 |
| BRAWH3022431 | 0.001675547 | 195705 | 1 |
| BRAWH3022459 | 0.001675547 | 218788 | 1 |
| BRAWH3022542 | 0.001675547 | 258254 | 1 |
| BRAWH3022651 | 0.10228708 | 119632 | 35 |
| BRAWH3022719 | 0.001675547 | 106275 | 1 |
| BRAWH3022866 | 0.001675547 | 276578 | 1 |
| BRAWH3022900 | 0.001675547 | 111018 | 1 |
| BRAWH3023156 | 0.001675547 | 266869 | 1 |
| BRAWH3023168 | 0.001675547 | 226668 | 1 |
| BRAWH3023172 | 0.182954113 | 86062 | 10 |
| BRAWH3023274 | 0.001675547 | 109669 | 1 |
| BRAWH3023421 | 0.001675547 | 264367 | 1 |
| BRAWH3024186 | 0.016789762 | 65471 | 8 |
| BRAWH3024231 | 0.001675547 | 267850 | 1 |
| BRAWH3024242 | 0.005261849 | 157813 | 3 |
| BRAWH3024469 | 0.001675547 | 19232 | 1 |
| BRAWH3024506 | 0.001675547 | 156888 | 1 |
| BRAWH3024613 | 0.001675547 | 197765 | 1 |
| BRAWH3024989 | 0.07072506 | 120140 | 29 |
| BRAWH3026349 | 0.001675547 | 251374 | 1 |
| BRAWH3026394 | 0.001675547 | 259578 | 1 |
| BRAWH3026529 | 0.003673948 | 138720 | 2 |
| BRAWH3026938 | 0.072918291 | 109313 | 27 |
| BRAWH3027420 | 0.001675547 | 212385 | 1 |
| BRAWH3027440 | 0.002864792 | 178730 | 2 |
| BRAWH3027533 | 0.015221005 | 130150 | 3 |
| BRAWH3027574 | 0.003533661 | 147319 | 2 |
| BRAWH3027607 | 0.001675547 | 132187 | 1 |
| BRAWH3027616 | 0.001675547 | 184622 | 1 |
| BRAWH3027675 | 0.001675547 | 244944 | 1 |
| BRAWH3027806 | 0.001675547 | 201378 | 1 |
| BRAWH3027880 | 0.001675547 | 183033 | 1 |
| BRAWH3027927 | 0.001675547 | 3799 | 1 |
| BRAWH3028202 | 0.001675547 | 186937 | 1 |
| BRAWH3028223 | 0.001675547 | 234808 | 1 |
| BRAWH3028306 | 0.001675547 | 61779 | 1 |
| BRAWH3028461 | 0.001675547 | 201279 | 1 |
| BRAWH3028645 | 0.001675547 | 213562 | 1 |
| BRAWH3028754 | 0.001675547 | 262333 | 1 |
| BRAWH3028796 | 0.001675547 | 210470 | 1 |
| BRAWH3029254 | 0.001675547 | 204569 | 1 |
| BRAWH3029313 | 0.001675547 | 195640 | 1 |
| BRAWH3029385 | 0.009523796 | 157043 | 6 |
| BRAWH3029538 | 0.003351094 | 83210 | 2 |
| BRAWH3029556 | 0.008606945 | 116664 | 5 |
| BRAWH3029806 | 0.018801856 | 83334 | 11 |
| BRAWH3030613 | 0.001675547 | 156978 | 1 |
| BRAWH3030772 | 0.001675547 | 125907 | 1 |
| BRAWH3030810 | 0.015742571 | 125856 | 12 |
| BRAWH3030892 | 0.001675547 | 147829 | 1 |
| BRAWH3030910 | 0.001675547 | 125981 | 1 |
| BRAWH3030950 | 0.001675547 | 174483 | 1 |
| BRAWH3031054 | 0.001675547 | 146636 | 1 |
| BRAWH3031342 | 0.03086749 | 18696 | 13 |
| BRAWH3031710 | 0.001675547 | 152867 | 1 |
| BRAWH3031902 | 0.001675547 | 132138 | 1 |
| BRAWH3032298 | 0.001675547 | 159392 | 1 |
| BRAWH3032340 | 0.001675547 | 147921 | 1 |
| BRAWH3032571 | 0.001675547 | 159231 | 1 |
| BRAWH3032620 | 0.001675547 | 94379 | 1 |
| BRAWH3032930 | 0.001675547 | 211300 | 1 |
| BRAWH3033117 | 0.001675547 | 169507 | 1 |
| BRAWH3033293 | 0.001675547 | 177964 | 1 |
| BRAWH3033448 | 0.001675547 | 214377 | 1 |
| BRAWH3033513 | 0.001675547 | 216441 | 1 |
| BRAWH3034097 | 0.001675547 | 139494 | 1 |
| BRAWH3034114 | 0.001675547 | 134289 | 1 |
| BRAWH3034134 | 0.001675547 | 120881 | 1 |
| BRAWH3034668 | 0.001675547 | 237898 | 1 |
| BRAWH3034743 | 0.040224064 | 56904 | 21 |
| BRAWH3034775 | 0.001675547 | 73162 | 1 |
| BRAWH3034890 | 0.001675547 | 73172 | 1 |
| BRAWH3035154 | 0.001675547 | 156807 | 1 |
| BRAWH3035403 | 0.004741633 | 124099 | 3 |
| BRAWH3035904 | 0.001675547 | 166356 | 1 |
| BRAWH3035914 | 0.001675547 | 103310 | 1 |
| BRAWH3035936 | 0.00474825 | 134375 | 3 |
| BRAWH3036077 | 0.001675547 | 153135 | 1 |
| BRAWH3036247 | 0.003072702 | 156760 | 2 |
| BRAWH3036270 | 0.001675547 | 174326 | 1 |
| BRAWH3036334 | 0.026846921 | 18694 | 13 |
| BRAWH3036561 | 0.001675547 | 165613 | 1 |
| BRAWH3036738 | 0.001675547 | 202007 | 1 |
| BRAWH3036951 | 0.001675547 | 140590 | 1 |
| BRAWH3037265 | 0.003757059 | 166320 | 2 |
| BRAWH3037394 | 0.001675547 | 128028 | 1 |
| BRAWH3037428 | 0.028512311 | 60834 | 13 |
| BRAWH3037533 | 0.001675547 | 118159 | 1 |
| BRAWH3037979 | 0.001675547 | 116724 | 1 |
| BRAWH3038055 | 0.003351094 | 149091 | 2 |
| BRAWH3038230 | 0.001675547 | 239909 | 1 |
| BRAWH3038252 | 0.001675547 | 113692 | 1 |
| BRAWH3038324 | 0.003351094 | 13645 | 2 |
| BRCAN1000065 | 0.003844084 | 135287 | 1 |
| BRCAN1000076 | 0.056927504 | 71942 | 6 |
| BRCAN1000105 | 0.003844084 | 91601 | 1 |
| BRCAN1000149 | 0.006758941 | 122109 | 2 |
| BRCAN1000168 | 0.118776685 | 82827 | 42 |
| BRCAN2000148 | 0.003844084 | 244740 | 1 |
| BRCAN2000149 | 0.003844084 | 248714 | 1 |
| BRCAN2000209 | 0.010046404 | 163728 | 2 |
| BRCAN2000239 | 0.003844084 | 137382 | 1 |
| BRCAN2000346 | 0.003844084 | 147935 | 1 |
| BRCAN2000364 | 0.003844084 | 54885 | 1 |
| BRCAN2000383 | 0.003844084 | 261105 | 1 |
| BRCAN2000418 | 0.003844084 | 276541 | 1 |
| BRCAN2000523 | 0.003844084 | 220782 | 1 |
| BRCAN2000541 | 0.003844084 | 99442 | 1 |
| BRCAN2000563 | 0.012741448 | 80080 | 4 |
| BRCAN2000577 | 0.003844084 | 187454 | 1 |
| BRCAN2000620 | 0.003844084 | 112571 | 1 |
| BRCAN2000639 | 0.00998961 | 132003 | 2 |
| BRCAN2000763 | 0.003844084 | 189621 | 1 |
| BRCAN2000832 | 0.003844084 | 85128 | 1 |
| BRCAN2000923 | 0.14449106 | 143876 | 2 |
| BRCAN2001223 | 0.007241821 | 50291 | 3 |
| BRCAN2001866 | 0.003844084 | 99717 | 1 |
| BRCAN2002173 | 0.084263573 | 78105 | 12 |
| BRCAN2002416 | 0.003844084 | 98836 | 1 |
| BRCAN2002473 | 0.003844084 | 54001 | 1 |
| BRCAN2002562 | 0.003844084 | 3398 | 1 |
| BRCAN2002662 | 0.003844084 | 161777 | 1 |
| BRCAN2002826 | 0.003844084 | 249654 | 1 |
| BRCAN2002854 | 0.003844084 | 63625 | 1 |
| BRCAN2002856 | 0.019559718 | 8075 | 4 |
| BRCAN2002892 | 0.005702198 | 102492 | 2 |
| BRCAN2002944 | 0.005241239 | 187798 | 2 |
| BRCAN2002948 | 0.003844084 | 139985 | 1 |
| BRCAN2003070 | 0.003844084 | 275253 | 1 |
| BRCAN2003269 | 0.003844084 | 177054 | 1 |
| BRCAN2003389 | 0.055104973 | 42048 | 3 |
| BRCAN2003628 | 0.003844084 | 161812 | 1 |
| BRCAN2003703 | 0.003844084 | 141346 | 1 |
| BRCAN2003746 | 0.003844084 | 190406 | 1 |
| BRCAN2003814 | 0.038735919 | 137987 | 2 |
| BRCAN2003944 | 0.594971919 | 95673 | 58 |
| BRCAN2003987 | 0.003844084 | 151203 | 1 |
| BRCAN2004173 | 0.003844084 | 160496 | 1 |
| BRCAN2004355 | 0.003844084 | 84673 | 1 |
| BRCAN2004371 | 0.003844084 | 152864 | 1 |
| BRCAN2004653 | 0.004939362 | 138924 | 2 |
| BRCAN2004699 | 0.003844084 | 179615 | 1 |
| BRCAN2005436 | 0.003844084 | 190248 | 1 |
| BRCAN2005449 | 0.003844084 | 28712 | 1 |
| BRCAN2006019 | 0.04460076 | 104549 | 10 |
| BRCAN2006051 | 0.003844084 | 163958 | 1 |
| BRCAN2006063 | 0.003844084 | 58312 | 1 |
| BRCAN2006117 | 0.006721605 | 169303 | 3 |
| BRCAN2006174 | 0.003844084 | 160469 | 1 |
| BRCAN2006290 | 0.003844084 | 212382 | 1 |
| BRCAN2006297 | 0.011637973 | 170416 | 3 |
| BRCAN2006323 | 0.003844084 | 42504 | 1 |
| BRCAN2006401 | 0.007390475 | 110847 | 3 |
| BRCAN2006411 | 0.003844084 | 41795 | 1 |
| BRCAN2006450 | 0.003844084 | 263743 | 1 |
| BRCAN2006955 | 0.003844084 | 268784 | 1 |
| BRCAN2007119 | 0.00988565 | 36558 | 2 |
| BRCAN2007144 | 0.003844084 | 36656 | 1 |
| BRCAN2007409 | 0.055846075 | 59873 | 8 |
| BRCAN2007426 | 0.003844084 | 146743 | 1 |
| BRCAN2007525 | 0.003844084 | 195523 | 1 |
| BRCAN2007700 | 0.0137779 | 188624 | 5 |
| BRCAN2008494 | 0.003844084 | 149957 | 1 |
| BRCAN2008528 | 0.003844084 | 144220 | 1 |
| BRCAN2008701 | 0.003844084 | 161671 | 1 |
| BRCAN2008894 | 0.022007189 | 165687 | 4 |
| BRCAN2009156 | 0.003844084 | 39419 | 1 |
| BRCAN2009168 | 0.003844084 | 225031 | 1 |
| BRCAN2009203 | 0.003844084 | 34820 | 1 |
| BRCAN2009432 | 0.127250354 | 23822 | 14 |
| BRCAN2010374 | 0.005532361 | 103405 | 2 |
| BRCAN2010376 | 0.005033328 | 141036 | 2 |
| BRCAN2010547 | 0.003844084 | 230039 | 1 |
| BRCAN2010581 | 0.003844084 | 195474 | 1 |
| BRCAN2010665 | 0.051267018 | 79370 | 21 |
| BRCAN2011254 | 0.003844084 | 174425 | 1 |
| BRCAN2011316 | 0.003844084 | 225201 | 1 |
| BRCAN2011602 | 0.007688168 | 167458 | 2 |
| BRCAN2011946 | 0.026496751 | 28461 | 6 |
| BRCAN2012110 | 0.003844084 | 108708 | 1 |
| BRCAN2012355 | 0.003844084 | 195653 | 1 |
| BRCAN2012402 | 0.003844084 | 275590 | 1 |
| BRCAN2012408 | 0.003844084 | 146031 | 1 |
| BRCAN2012481 | 0.003844084 | 206235 | 1 |
| BRCAN2012613 | 0.003844084 | 198686 | 1 |
| BRCAN2012713 | 0.003844084 | 201852 | 1 |
| BRCAN2012929 | 0.013729734 | 169420 | 3 |
| BRCAN2013655 | 0.003844084 | 209395 | 1 |
| BRCAN2013660 | 0.003844084 | 271438 | 1 |
| BRCAN2014143 | 0.003844084 | 160591 | 1 |
| BRCAN2014229 | 0.003844084 | 160592 | 1 |
| BRCAN2014370 | 0.003844084 | 202940 | 1 |
| BRCAN2014602 | 0.003844084 | 82595 | 1 |
| BRCAN2014652 | 0.003844084 | 107243 | 1 |
| BRCAN2014788 | 0.003844084 | 220642 | 1 |
| BRCAN2014881 | 0.005532361 | 8661 | 2 |
| BRCAN2014952 | 0.003844084 | 174930 | 1 |
| BRCAN2015371 | 0.015285503 | 152047 | 5 |
| BRCAN2015402 | 0.003844084 | 267367 | 1 |
| BRCAN2015464 | 0.003844084 | 241390 | 1 |
| BRCAN2015719 | 0.055495027 | 109738 | 12 |
| BRCAN2015757 | 0.003844084 | 232947 | 1 |
| BRCAN2015886 | 0.003844084 | 232952 | 1 |
| BRCAN2016025 | 0.003844084 | 209149 | 1 |
| BRCAN2016046 | 0.005532361 | 154590 | 2 |
| BRCAN2016174 | 0.015410936 | 168562 | 3 |
| BRCAN2016433 | 0.003844084 | 54131 | 1 |
| BRCAN2016619 | 0.01271868 | 109646 | 4 |
| BRCAN2016762 | 0.003844084 | 138231 | 1 |
| BRCAN2016814 | 0.005702198 | 149212 | 2 |
| BRCAN2017442 | 0.003844084 | 195360 | 1 |
| BRCAN2017717 | 0.015750862 | 131671 | 4 |
| BRCAN2017905 | 0.003844084 | 213650 | 1 |
| BRCAN2018240 | 0.003844084 | 114116 | 1 |
| BRCAN2018269 | 0.003844084 | 147416 | 1 |
| BRCAN2018490 | 0.003844084 | 218477 | 1 |
| BRCAN2018566 | 0.006758941 | 221911 | 2 |
| BRCAN2018667 | 0.003844084 | 272519 | 1 |
| BRCAN2018748 | 0.003844084 | 21173 | 1 |
| BRCAN2018935 | 0.018368412 | 275378 | 2 |
| BRCAN2019002 | 0.003844084 | 231098 | 1 |
| BRCAN2019387 | 0.003844084 | 158055 | 1 |
| BRCAN2019653 | 0.026866142 | 137400 | 7 |
| BRCAN2019773 | 0.003844084 | 261413 | 1 |
| BRCAN2019907 | 0.003844084 | 61114 | 1 |
| BRCAN2019953 | 0.017616261 | 126124 | 7 |
| BRCAN2020234 | 0.006797477 | 151391 | 3 |
| BRCAN2020331 | 0.003844084 | 74998 | 1 |
| BRCAN2020412 | 0.015363494 | 19743 | 2 |
| BRCAN2020467 | 0.003844084 | 7574 | 1 |
| BRCAN2020710 | 0.015417139 | 126295 | 6 |
| BRCAN2020880 | 0.003844084 | 209759 | 1 |
| BRCAN2021024 | 0.016339278 | 57384 | 8 |
| BRCAN2021028 | 0.015925385 | 219211 | 3 |
| BRCAN2021325 | 0.003844084 | 18495 | 1 |
| BRCAN2021452 | 0.003844084 | 262828 | 1 |
| BRCAN2021669 | 0.003844084 | 252453 | 1 |
| BRCAN2021718 | 0.003844084 | 209683 | 1 |
| BRCAN2022126 | 0.034508627 | 126374 | 12 |
| BRCAN2022191 | 0.003844084 | 252624 | 1 |
| BRCAN2022472 | 0.003844084 | 121747 | 1 |
| BRCAN2022474 | 0.003844084 | 172625 | 1 |
| BRCAN2023347 | 0.003844084 | 216974 | 1 |
| BRCAN2023724 | 0.003844084 | 283958 | 1 |
| BRCAN2024259 | 0.095037005 | 31692 | 41 |
| BRCAN2024451 | 0.003844084 | 179715 | 1 |
| BRCAN2024563 | 0.003844084 | 276224 | 1 |
| BRCAN2024572 | 0.061349936 | 69558 | 17 |
| BRCAN2025009 | 0.003844084 | 276509 | 1 |
| BRCAN2025093 | 0.007710188 | 139096 | 4 |
| BRCAN2025349 | 0.003844084 | 242581 | 1 |
| BRCAN2025368 | 0.019796904 | 136756 | 4 |
| BRCAN2025712 | 0.003844084 | 172914 | 1 |
| BRCAN2025982 | 0.005553544 | 67879 | 2 |
| BRCAN2026117 | 0.003844084 | 280910 | 1 |
| BRCAN2026197 | 0.107321308 | 43537 | 34 |
| BRCAN2026340 | 0.011087697 | 198787 | 3 |
| BRCAN2026711 | 0.003844084 | 86354 | 1 |
| BRCAN2027150 | 0.016438542 | 32203 | 2 |
| BRCAN2027310 | 0.003844084 | 172371 | 1 |
| BRCAN2027334 | 0.005532361 | 131917 | 2 |
| BRCAN2027355 | 0.003844084 | 74301 | 1 |
| BRCAN2027364 | 0.056419347 | 28163 | 18 |
| BRCAN2027513 | 0.003844084 | 150618 | 1 |
| BRCAN2027593 | 0.003844084 | 281663 | 1 |
| BRCAN2027970 | 0.003844084 | 197634 | 1 |
| BRCAN2028021 | 0.003844084 | 142751 | 1 |
| BRCAN2028036 | 0.069399133 | 37105 | 14 |
| BRCAN2028040 | 0.006336518 | 179367 | 3 |
| BRCAN2028319 | 0.012874753 | 118570 | 4 |
| BRCAN2028338 | 0.003844084 | 202521 | 1 |
| BRCAN2028355 | 0.113416185 | 34589 | 7 |
| BRCAN2028378 | 0.003844084 | 190610 | 1 |
| BRCAN2028460 | 0.003844084 | 221046 | 1 |
| BRCAN2028545 | 0.00988565 | 249974 | 2 |
| BRCAN2028634 | 0.005702198 | 230114 | 2 |
| BRCAN2028702 | 0.003844084 | 222760 | 1 |
| BRCOC1000040 | 0.056815748 | 91819 | 23 |
| BRCOC1000087 | 0.005935775 | 98530 | 1 |
| BRCOC1000094 | 0.175974203 | 90967 | 41 |
| BRCOC2000004 | 0.093083084 | 117659 | 26 |
| BRCOC2000047 | 0.011990143 | 186127 | 2 |
| BRCOC2000152 | 0.008017287 | 126617 | 2 |
| BRCOC2000184 | 0.019993963 | 153536 | 3 |
| BRCOC2000186 | 0.056330024 | 61789 | 5 |
| BRCOC2000333 | 0.013988544 | 57918 | 3 |
| BRCOC2000360 | 0.009320782 | 13758 | 3 |
| BRCOC2000404 | 0.005935775 | 71087 | 1 |
| BRCOC2000487 | 0.578350283 | 183430 | 8 |
| BRCOC2000637 | 0.005935775 | 33321 | 1 |
| BRCOC2000670 | 0.007624052 | 157803 | 2 |
| BRCOC2000816 | 0.007611322 | 47698 | 2 |
| BRCOC2000850 | 0.005935775 | 251661 | 1 |
| BRCOC2001355 | 0.005935775 | 168470 | 1 |
| BRCOC2002085 | 0.005935775 | 227864 | 1 |
| BRCOC2002155 | 0.007812617 | 85141 | 2 |
| BRCOC2002323 | 0.005935775 | 208816 | 1 |
| BRCOC2002659 | 0.005935775 | 60687 | 1 |
| BRCOC2002664 | 0.005935775 | 223483 | 1 |
| BRCOC2002686 | 0.005935775 | 227218 | 1 |
| BRCOC2002751 | 0.005935775 | 152273 | 1 |
| BRCOC2002756 | 0.005935775 | 236262 | 1 |
| BRCOC2002777 | 0.005935775 | 236888 | 1 |
| BRCOC2002835 | 0.005935775 | 271611 | 1 |
| BRCOC2003100 | 0.007125019 | 189182 | 2 |
| BRCOC2003125 | 0.02948599 | 170652 | 3 |
| BRCOC2003187 | 0.213061185 | 136280 | 21 |
| BRCOC2003213 | 0.016139846 | 176666 | 5 |
| BRCOC2003513 | 0.005935775 | 222059 | 1 |
| BRCOC2003732 | 0.007812617 | 72397 | 2 |
| BRCOC2003740 | 0.005935775 | 118759 | 1 |
| BRCOC2004036 | 0.011990143 | 84666 | 2 |
| BRCOC2004175 | 0.017222457 | 14318 | 2 |
| BRCOC2004354 | 0.005935775 | 71847 | 1 |
| BRCOC2004455 | 0.005935775 | 187920 | 1 |
| BRCOC2004950 | 0.005935775 | 222129 | 1 |
| BRCOC2005542 | 0.009482166 | 137824 | 3 |
| BRCOC2005903 | 0.019087997 | 182454 | 3 |
| BRCOC2005951 | 0.005935775 | 113101 | 1 |
| BRCOC2006164 | 0.011528845 | 67154 | 4 |
| BRCOC2006189 | 0.05047861 | 87356 | 17 |
| BRCOC2006243 | 0.005935775 | 245025 | 1 |
| BRCOC2006270 | 0.011990143 | 5012 | 2 |
| BRCOC2006639 | 0.005935775 | 83473 | 1 |
| BRCOC2006942 | 0.005935775 | 161969 | 1 |
| BRCOC2007034 | 0.007125019 | 197343 | 2 |
| BRCOC2007476 | 0.020811331 | 129777 | 7 |
| BRCOC2007593 | 0.005935775 | 200862 | 1 |
| BRCOC2007733 | 0.005935775 | 143874 | 1 |
| BRCOC2007769 | 0.005935775 | 46881 | 1 |
| BRCOC2007864 | 0.165643883 | 125094 | 44 |
| BRCOC2008064 | 0.005935775 | 272829 | 1 |
| BRCOC2008225 | 0.005935775 | 160323 | 1 |
| BRCOC2008642 | 0.005935775 | 174077 | 1 |
| BRCOC2008993 | 0.005935775 | 128520 | 1 |
| BRCOC2009052 | 0.005935775 | 156579 | 1 |
| BRCOC2009196 | 0.005935775 | 130823 | 1 |
| BRCOC2009380 | 0.005935775 | 208446 | 1 |
| BRCOC2009638 | 0.005935775 | 160277 | 1 |
| BRCOC2009937 | 0.01042879 | 49779 | 4 |
| BRCOC2010115 | 0.005935775 | 274106 | 1 |
| BRCOC2010123 | 0.152952001 | 135859 | 14 |
| BRCOC2010510 | 0.013953062 | 173796 | 3 |
| BRCOC2010730 | 0.015501458 | 52063 | 6 |
| BRCOC2010980 | 0.005935775 | 115520 | 1 |
| BRCOC2010994 | 0.005935775 | 239211 | 1 |
| BRCOC2011010 | 0.005935775 | 149962 | 1 |
| BRCOC2011398 | 0.005935775 | 193068 | 1 |
| BRCOC2011418 | 0.005935775 | 167478 | 1 |
| BRCOC2011506 | 0.176080525 | 152205 | 14 |
| BRCOC2011544 | 0.117885646 | 168112 | 17 |
| BRCOC2011769 | 0.005935775 | 215804 | 1 |
| BRCOC2011996 | 0.005935775 | 208943 | 1 |
| BRCOC2012172 | 0.059567813 | 141939 | 29 |
| BRCOC2012229 | 0.005935775 | 86581 | 1 |
| BRCOC2012386 | 0.014694486 | 136577 | 4 |
| BRCOC2012551 | 0.023231153 | 123831 | 11 |
| BRCOC2012813 | 0.009779859 | 42927 | 2 |
| BRCOC2013448 | 0.005935775 | 17689 | 1 |
| BRCOC2013573 | 0.005935775 | 258775 | 1 |
| BRCOC2013675 | 0.005935775 | 125334 | 1 |
| BRCOC2013780 | 0.011990143 | 168066 | 2 |
| BRCOC2014013 | 0.030896123 | 156531 | 5 |
| BRCOC2014033 | 0.010717938 | 192261 | 4 |
| BRCOC2014178 | 0.083115518 | 88587 | 28 |
| BRCOC2014400 | 0.009974058 | 37726 | 2 |
| BRCOC2014748 | 0.005935775 | 153159 | 1 |
| BRCOC2014833 | 0.005935775 | 159685 | 1 |
| BRCOC2015597 | 0.007934176 | 248379 | 2 |
| BRCOC2015824 | 0.005935775 | 133223 | 1 |
| BRCOC2015908 | 0.008850632 | 128500 | 2 |
| BRCOC2015944 | 0.005935775 | 271718 | 1 |
| BRCOC2016525 | 0.005935775 | 199258 | 1 |
| BRCOC2016661 | 0.005935775 | 273678 | 1 |
| BRCOC2017061 | 0.005935775 | 78265 | 1 |
| BRCOC2017652 | 0.012138095 | 18378 | 2 |
| BRCOC2017856 | 0.005935775 | 96918 | 1 |
| BRCOC2018775 | 0.01653798 | 142231 | 2 |
| BRCOC2018877 | 0.008850632 | 49368 | 2 |
| BRCOC2019255 | 0.019587861 | 128892 | 5 |
| BRCOC2019549 | 0.005935775 | 175751 | 1 |
| BRCOC2019841 | 0.007125019 | 171607 | 2 |
| BRCOC2019896 | 0.007624052 | 66373 | 2 |
| BRCOC2019903 | 0.010453737 | 104768 | 3 |
| BRCOC2019910 | 0.268110198 | 82318 | 140 |
| BRCOC2019934 | 0.005935775 | 197632 | 1 |
| BRCOC2020122 | 0.005935775 | 137297 | 1 |
| BRCOC2020142 | 0.007611322 | 152952 | 2 |
| BRHIP1000058 | 0.00170946 | 230471 | 1 |
| BRHIP1000072 | 0.00170946 | 146589 | 1 |
| BRHIP1000104 | 0.25633749 | 99527 | 75 |
| BRHIP1000108 | 0.038879618 | 85252 | 15 |
| BRHIP1000129 | 0.003397737 | 93924 | 2 |
| BRHIP1000174 | 0.033048593 | 122269 | 11 |
| BRHIP1000258 | 0.006480503 | 87401 | 3 |
| BRHIP2000021 | 0.014771111 | 83122 | 2 |
| BRHIP2000079 | 0.00170946 | 60877 | 1 |
| BRHIP2000087 | 0.025458357 | 141764 | 7 |
| BRHIP2000163 | 0.004480285 | 107084 | 3 |
| BRHIP2000210 | 0.002898705 | 180589 | 2 |
| BRHIP2000239 | 0.003586302 | 219053 | 2 |
| BRHIP2000245 | 0.003106616 | 36877 | 2 |
| BRHIP2000312 | 0.025838097 | 13600 | 14 |
| BRHIP2000359 | 0.00170946 | 110633 | 1 |
| BRHIP2000445 | 0.00170946 | 74655 | 1 |
| BRHIP2000506 | 0.00170946 | 265273 | 1 |
| BRHIP2000532 | 0.00170946 | 242287 | 1 |
| BRHIP2000534 | 0.489558345 | 54252 | 52 |
| BRHIP2000553 | 0.065204083 | 112505 | 24 |
| BRHIP2000621 | 0.00170946 | 135060 | 1 |
| BRHIP2000691 | 0.00170946 | 91747 | 1 |
| BRHIP2000819 | 0.077565447 | 63996 | 15 |
| BRHIP2000826 | 0.00170946 | 195964 | 1 |
| BRHIP2000920 | 0.004574252 | 1721 | 3 |
| BRHIP2001074 | 0.008552481 | 141443 | 4 |
| BRHIP2001099 | 0.024171057 | 18466 | 9 |
| BRHIP2001686 | 0.00170946 | 168052 | 1 |
| BRHIP2001782 | 0.00170946 | 147120 | 1 |
| BRHIP2001802 | 0.00170946 | 13741 | 1 |
| BRHIP2001805 | 0.011584053 | 130721 | 2 |
| BRHIP2001886 | 0.003397737 | 156599 | 2 |
| BRHIP2001927 | 0.00170946 | 103096 | 1 |
| BRHIP2002122 | 0.00170946 | 202996 | 1 |
| BRHIP2002143 | 0.002898705 | 152747 | 2 |
| BRHIP2002172 | 0.00170946 | 156585 | 1 |
| BRHIP2002339 | 0.021350394 | 130120 | 10 |
| BRHIP2002346 | 0.00170946 | 158808 | 1 |
| BRHIP2002510 | 0.00170946 | 283979 | 1 |
| BRHIP2002664 | 0.00170946 | 161947 | 1 |
| BRHIP2002722 | 0.00170946 | 154342 | 1 |
| BRHIP2002929 | 0.00170946 | 96456 | 1 |
| BRHIP2003048 | 0.00170946 | 147259 | 1 |
| BRHIP2003062 | 0.017638961 | 67501 | 10 |
| BRHIP2003242 | 0.00170946 | 160902 | 1 |
| BRHIP2003272 | 0.00170946 | 216050 | 1 |
| BRHIP2003748 | 0.00170946 | 207793 | 1 |
| BRHIP2003786 | 0.00170946 | 113029 | 1 |
| BRHIP2003828 | 0.00170946 | 219454 | 1 |
| BRHIP2003917 | 0.014268684 | 147330 | 3 |
| BRHIP2004312 | 0.00170946 | 40614 | 1 |
| BRHIP2004355 | 0.00170946 | 33474 | 1 |
| BRHIP2004359 | 0.00688226 | 14940 | 5 |
| BRHIP2004814 | 0.004830267 | 206824 | 2 |
| BRHIP2004883 | 0.00170946 | 203046 | 1 |
| BRHIP2004902 | 0.00170946 | 196516 | 1 |
| BRHIP2005010 | 0.01062862 | 144510 | 4 |
| BRHIP2005236 | 0.00170946 | 234942 | 1 |
| BRHIP2005271 | 0.023655592 | 131024 | 13 |
| BRHIP2005354 | 0.00170946 | 183924 | 1 |
| BRHIP2005583 | 0.048140878 | 101969 | 13 |
| BRHIP2005600 | 0.00170946 | 168223 | 1 |
| BRHIP2005719 | 0.003385007 | 191287 | 2 |
| BRHIP2005724 | 0.00170946 | 30696 | 1 |
| BRHIP2005752 | 0.002898705 | 108690 | 2 |
| BRHIP2005932 | 0.00170946 | 50190 | 1 |
| BRHIP2006617 | 0.00170946 | 161005 | 1 |
| BRHIP2006782 | 0.00170946 | 163891 | 1 |
| BRHIP2006800 | 0.00170946 | 163869 | 1 |
| BRHIP2006819 | 0.00170946 | 242608 | 1 |
| BRHIP2006921 | 0.00170946 | 260663 | 1 |
| BRHIP2007305 | 0.148012957 | 130789 | 29 |
| BRHIP2007307 | 0.005886395 | 143844 | 2 |
| BRHIP2007616 | 0.00170946 | 163973 | 1 |
| BRHIP2007690 | 0.00170946 | 213796 | 1 |
| BRHIP2007741 | 0.096527316 | 158769 | 27 |
| BRHIP2007928 | 0.00170946 | 154976 | 1 |
| BRHIP2007969 | 0.00170946 | 179793 | 1 |
| BRHIP2008269 | 0.006021473 | 40089 | 3 |
| BRHIP2008389 | 0.00170946 | 189559 | 1 |
| BRHIP2008607 | 0.009331071 | 109657 | 6 |
| BRHIP2008756 | 0.00170946 | 21599 | 1 |
| BRHIP2008909 | 0.00170946 | 211728 | 1 |
| BRHIP2009019 | 0.002898705 | 232461 | 2 |
| BRHIP2009177 | 0.00170946 | 228601 | 1 |
| BRHIP2009340 | 0.00170946 | 217726 | 1 |
| BRHIP2009351 | 0.00170946 | 239323 | 1 |
| BRHIP2009414 | 0.00170946 | 30608 | 1 |
| BRHIP2009474 | 0.003567575 | 170450 | 2 |
| BRHIP2009617 | 0.00170946 | 149177 | 1 |
| BRHIP2009685 | 0.047037149 | 151845 | 8 |
| BRHIP2010217 | 0.030001638 | 126118 | 9 |
| BRHIP2010309 | 0.00170946 | 222788 | 1 |
| BRHIP2010444 | 0.003567575 | 102257 | 2 |
| BRHIP2010487 | 0.040739036 | 105303 | 3 |
| BRHIP2010571 | 0.010881243 | 132250 | 7 |
| BRHIP2010593 | 0.047847882 | 118687 | 3 |
| BRHIP2010610 | 0.00170946 | 111747 | 1 |
| BRHIP2010723 | 0.003397737 | 140170 | 2 |
| BRHIP2010744 | 0.00170946 | 143992 | 1 |
| BRHIP2011080 | 0.004804956 | 108959 | 2 |
| BRHIP2011120 | 0.021830116 | 129498 | 12 |
| BRHIP2011199 | 0.003106616 | 165737 | 2 |
| BRHIP2011491 | 0.202710934 | 146691 | 17 |
| BRHIP2011508 | 0.007759041 | 88948 | 5 |
| BRHIP2011576 | 0.008740167 | 182175 | 3 |
| BRHIP2011616 | 0.003707861 | 155037 | 2 |
| BRHIP2011783 | 0.00170946 | 236020 | 1 |
| BRHIP2011838 | 0.00170946 | 175556 | 1 |
| BRHIP2011891 | 0.002804738 | 148563 | 2 |
| BRHIP2011933 | 0.00170946 | 27376 | 1 |
| BRHIP2012054 | 0.005886395 | 72314 | 2 |
| BRHIP2012059 | 0.00170946 | 156034 | 1 |
| BRHIP2012141 | 0.003567575 | 159032 | 2 |
| BRHIP2012314 | 0.00170946 | 179267 | 1 |
| BRHIP2012360 | 0.00341892 | 162676 | 2 |
| BRHIP2012545 | 0.00170946 | 183243 | 1 |
| BRHIP2012580 | 0.00170946 | 122018 | 1 |
| BRHIP2012627 | 0.010666604 | 175420 | 3 |
| BRHIP2012642 | 0.00170946 | 189468 | 1 |
| BRHIP2012750 | 0.00170946 | 133293 | 1 |
| BRHIP2012922 | 0.00341892 | 173974 | 2 |
| BRHIP2012923 | 0.005553544 | 70357 | 2 |
| BRHIP2012972 | 0.003567575 | 143538 | 2 |
| BRHIP2013286 | 0.009786026 | 132197 | 3 |
| BRHIP2013447 | 0.00170946 | 153478 | 1 |
| BRHIP2013510 | 0.00170946 | 89582 | 1 |
| BRHIP2013699 | 0.007852315 | 137064 | 4 |
| BRHIP2013723 | 0.00170946 | 38026 | 1 |
| BRHIP2013958 | 0.004662853 | 122647 | 3 |
| BRHIP2013972 | 0.00170946 | 34651 | 1 |
| BRHIP2014063 | 0.004624317 | 29547 | 2 |
| BRHIP2014228 | 0.01138336 | 151704 | 6 |
| BRHIP2014285 | 0.00170946 | 153370 | 1 |
| BRHIP2014291 | 0.00170946 | 54956 | 1 |
| BRHIP2014373 | 0.00496473 | 112679 | 3 |
| BRHIP2014386 | 0.003106616 | 102740 | 2 |
| BRHIP2014387 | 0.00170946 | 84258 | 1 |
| BRHIP2014747 | 0.146698192 | 36550 | 40 |
| BRHIP2014954 | 0.00170946 | 157461 | 1 |
| BRHIP2015153 | 0.016723757 | 125355 | 10 |
| BRHIP2015224 | 0.03683202 | 86319 | 18 |
| BRHIP2015245 | 0.070578263 | 8049 | 39 |
| BRHIP2015356 | 0.00170946 | 165697 | 1 |
| BRHIP2015360 | 0.00170946 | 157497 | 1 |
| BRHIP2015679 | 0.066794406 | 86476 | 14 |
| BRHIP2016005 | 0.00170946 | 189760 | 1 |
| BRHIP2016125 | 0.00170946 | 180928 | 1 |
| BRHIP2016788 | 0.00170946 | 150426 | 1 |
| BRHIP2016968 | 0.00170946 | 180531 | 1 |
| BRHIP2016990 | 0.00170946 | 165673 | 1 |
| BRHIP2017010 | 0.012188947 | 166776 | 7 |
| BRHIP2017315 | 0.00170946 | 140634 | 1 |
| BRHIP2017385 | 0.00170946 | 277604 | 1 |
| BRHIP2017404 | 0.00170946 | 98389 | 1 |
| BRHIP2017542 | 0.00170946 | 236099 | 1 |
| BRHIP2017553 | 0.00170946 | 241426 | 1 |
| BRHIP2017642 | 0.012973736 | 148973 | 6 |
| BRHIP2017714 | 0.00170946 | 127630 | 1 |
| BRHIP2017780 | 0.00170946 | 140992 | 1 |
| BRHIP2017945 | 0.051986696 | 163768 | 4 |
| BRHIP2018014 | 0.00170946 | 5665 | 1 |
| BRHIP2018369 | 0.00170946 | 198515 | 1 |
| BRHIP2018612 | 0.00170946 | 218737 | 1 |
| BRHIP2018635 | 0.002804738 | 18726 | 2 |
| BRHIP2018650 | 0.00341892 | 158261 | 2 |
| BRHIP2018712 | 0.08273766 | 112751 | 28 |
| BRHIP2018719 | 0.003397737 | 139645 | 2 |
| BRHIP2018805 | 0.00170946 | 149646 | 1 |
| BRHIP2018998 | 0.00170946 | 134678 | 1 |
| BRHIP2019007 | 0.00170946 | 146177 | 1 |
| BRHIP2019047 | 0.00170946 | 139731 | 1 |
| BRHIP2019149 | 0.015171807 | 124148 | 3 |
| BRHIP2019177 | 0.00170946 | 110480 | 1 |
| BRHIP2019186 | 0.084900897 | 33675 | 11 |
| BRHIP2019291 | 0.00170946 | 281799 | 1 |
| BRHIP2019494 | 0.004608165 | 129121 | 3 |
| BRHIP2019589 | 0.00170946 | 68568 | 1 |
| BRHIP2019641 | 0.00341892 | 187186 | 2 |
| BRHIP2019819 | 0.019324521 | 134898 | 10 |
| BRHIP2019884 | 0.00170946 | 249651 | 1 |
| BRHIP2019972 | 0.00341892 | 192726 | 2 |
| BRHIP2020182 | 0.00170946 | 23948 | 1 |
| BRHIP2020509 | 0.008948078 | 59863 | 3 |
| BRHIP2020573 | 0.01851457 | 94284 | 9 |
| BRHIP2020622 | 0.007241821 | 151154 | 3 |
| BRHIP2020695 | 0.003385007 | 166153 | 2 |
| BRHIP2020743 | 0.00170946 | 187389 | 1 |
| BRHIP2020799 | 0.033107653 | 44218 | 18 |
| BRHIP2020827 | 0.026065865 | 132980 | 13 |
| BRHIP2020842 | 0.00341892 | 143639 | 2 |
| BRHIP2020859 | 0.00170946 | 216464 | 1 |
| BRHIP2020930 | 0.00170946 | 190847 | 1 |
| BRHIP2021102 | 0.00170946 | 166796 | 1 |
| BRHIP2021289 | 0.00170946 | 173214 | 1 |
| BRHIP2021495 | 0.026123956 | 118824 | 7 |
| BRHIP2021615 | 0.00341892 | 189293 | 2 |
| BRHIP2021744 | 0.00170946 | 187775 | 1 |
| BRHIP2021762 | 0.060142531 | 89523 | 21 |
| BRHIP2021858 | 0.050070972 | 99622 | 9 |
| BRHIP2021870 | 0.037738375 | 57100 | 11 |
| BRHIP2021929 | 0.00170946 | 115180 | 1 |
| BRHIP2022006 | 0.00170946 | 116182 | 1 |
| BRHIP2022054 | 0.068041549 | 117359 | 10 |
| BRHIP2022221 | 0.003567575 | 205217 | 2 |
| BRHIP2022228 | 0.018336685 | 18904 | 9 |
| BRHIP2022298 | 0.00170946 | 146534 | 1 |
| BRHIP2022326 | 0.196957662 | 123190 | 39 |
| BRHIP2022467 | 0.011789902 | 157907 | 3 |
| BRHIP2022708 | 0.00170946 | 136370 | 1 |
| BRHIP2022986 | 0.058886507 | 33672 | 16 |
| BRHIP2023071 | 0.003397737 | 172433 | 2 |
| BRHIP2023229 | 0.011163937 | 101098 | 2 |
| BRHIP2023272 | 0.049644331 | 52563 | 4 |
| BRHIP2023292 | 0.004804956 | 182910 | 2 |
| BRHIP2023309 | 0.014362873 | 168908 | 6 |
| BRHIP2023369 | 0.009587327 | 96335 | 3 |
| BRHIP2023438 | 0.006568261 | 86980 | 3 |
| BRHIP2023695 | 0.004691419 | 152755 | 2 |
| BRHIP2023735 | 0.00170946 | 210654 | 1 |
| BRHIP2023762 | 0.070211287 | 67922 | 10 |
| BRHIP2023773 | 0.005886395 | 86378 | 2 |
| BRHIP2023860 | 0.00170946 | 206783 | 1 |
| BRHIP2023869 | 0.004662853 | 228725 | 3 |
| BRHIP2023888 | 0.455384502 | 77486 | 118 |
| BRHIP2024046 | 0.005463143 | 150099 | 3 |
| BRHIP2024146 | 0.550803718 | 46185 | 133 |
| BRHIP2024165 | 0.00170946 | 279107 | 1 |
| BRHIP2024201 | 0.00170946 | 137328 | 1 |
| BRHIP2024347 | 0.00170946 | 241176 | 1 |
| BRHIP2024398 | 0.019900997 | 98223 | 9 |
| BRHIP2024549 | 0.00170946 | 118391 | 1 |
| BRHIP2024742 | 0.134347487 | 50167 | 50 |
| BRHIP2024789 | 0.00341892 | 189370 | 2 |
| BRHIP2024911 | 0.018077944 | 114397 | 8 |
| BRHIP2024941 | 0.00170946 | 282810 | 1 |
| BRHIP2025245 | 0.003586302 | 51623 | 2 |
| BRHIP2025366 | 0.00170946 | 149074 | 1 |
| BRHIP2025448 | 0.005479249 | 203737 | 3 |
| BRHIP2025679 | 0.002898705 | 119403 | 2 |
| BRHIP2025739 | 0.00170946 | 227691 | 1 |
| BRHIP2025797 | 0.00170946 | 78509 | 1 |
| BRHIP2026021 | 0.00170946 | 62680 | 1 |
| BRHIP2026061 | 0.005479249 | 205812 | 3 |
| BRHIP2026155 | 0.00170946 | 174215 | 1 |
| BRHIP2026214 | 0.004804956 | 157216 | 2 |
| BRHIP2026274 | 0.018249654 | 218809 | 5 |
| BRHIP2026288 | 0.007763828 | 116278 | 2 |
| BRHIP2026346 | 0.00170946 | 144555 | 1 |
| BRHIP2026557 | 0.00170946 | 173254 | 1 |
| BRHIP2026723 | 0.00170946 | 229414 | 1 |
| BRHIP2026877 | 0.005553544 | 162037 | 2 |
| BRHIP2027017 | 0.014555181 | 134600 | 6 |
| BRHIP2027054 | 0.00170946 | 278982 | 1 |
| BRHIP2027077 | 0.082954585 | 126331 | 13 |
| BRHIP2027563 | 0.00170946 | 221371 | 1 |
| BRHIP2027685 | 0.003397737 | 272625 | 2 |
| BRHIP2027762 | 0.00170946 | 187405 | 1 |
| BRHIP2028187 | 0.004608165 | 156486 | 3 |
| BRHIP2028303 | 0.00170946 | 15203 | 1 |
| BRHIP2028330 | 0.00170946 | 189077 | 1 |
| BRHIP2028480 | 0.00170946 | 165857 | 1 |
| BRHIP2028583 | 0.013470015 | 142951 | 2 |
| BRHIP2028593 | 0.00170946 | 150512 | 1 |
| BRHIP2029176 | 0.00170946 | 195499 | 1 |
| BRHIP2029283 | 0.00170946 | 249330 | 1 |
| BRHIP2029393 | 0.00170946 | 224694 | 1 |
| BRHIP2029529 | 0.00170946 | 27646 | 1 |
| BRHIP2029643 | 0.011216575 | 155782 | 4 |
| BRHIP2029663 | 0.003790972 | 175923 | 2 |
| BRHIP3000017 | 0.038000524 | 75906 | 14 |
| BRHIP3000060 | 0.003567575 | 148628 | 2 |
| BRHIP3000070 | 0.00170946 | 210344 | 1 |
| BRHIP3000081 | 0.00512838 | 118437 | 3 |
| BRHIP3000111 | 0.163667808 | 24064 | 26 |
| BRHIP3000131 | 0.045627167 | 103307 | 19 |
| BRHIP3000240 | 0.005277194 | 60392 | 4 |
| BRHIP3000339 | 0.398667125 | 6827 | 161 |
| BRHIP3000377 | 0.029087086 | 174714 | 5 |
| BRHIP3000457 | 0.006169461 | 204613 | 4 |
| BRHIP3000473 | 0.034647887 | 46754 | 15 |
| BRHIP3000475 | 0.007749326 | 252971 | 4 |
| BRHIP3000488 | 0.00341892 | 149552 | 2 |
| BRHIP3000526 | 0.00170946 | 240924 | 1 |
| BRHIP3000626 | 0.00170946 | 251484 | 1 |
| BRHIP3000859 | 0.00170946 | 224748 | 1 |
| BRHIP3000907 | 0.00170946 | 235965 | 1 |
| BRHIP3001076 | 0.003707861 | 85300 | 2 |
| BRHIP3001079 | 0.00170946 | 276480 | 1 |
| BRHIP3001141 | 0.00170946 | 260374 | 1 |
| BRHIP3001283 | 0.003397737 | 197725 | 2 |
| BRHIP3001338 | 0.00170946 | 245641 | 1 |
| BRHIP3001360 | 0.002898705 | 118453 | 2 |
| BRHIP3001481 | 0.008895312 | 125404 | 7 |
| BRHIP3001573 | 0.020538448 | 66030 | 11 |
| BRHIP3001878 | 0.00170946 | 278106 | 1 |
| BRHIP3001964 | 0.00170946 | 260235 | 1 |
| BRHIP3002000 | 0.223425027 | 89444 | 65 |
| BRHIP3002114 | 0.00429586 | 177731 | 3 |
| BRHIP3002124 | 0.00170946 | 210949 | 1 |
| BRHIP3002141 | 0.010679107 | 124253 | 5 |
| BRHIP3002363 | 0.00170946 | 253318 | 1 |
| BRHIP3002691 | 0.020202753 | 122270 | 8 |
| BRHIP3002920 | 0.005277035 | 151611 | 3 |
| BRHIP3002931 | 0.00170946 | 223015 | 1 |
| BRHIP3003063 | 0.015287678 | 50029 | 9 |
| BRHIP3003126 | 0.004608165 | 80595 | 3 |
| BRHIP3003306 | 0.002804738 | 259995 | 2 |
| BRHIP3003340 | 0.00170946 | 247561 | 1 |
| BRHIP3003395 | 0.00170946 | 254955 | 1 |
| BRHIP3003484 | 0.00170946 | 264212 | 1 |
| BRHIP3003644 | 0.00170946 | 242845 | 1 |
| BRHIP3003688 | 0.00170946 | 229131 | 1 |
| BRHIP3003795 | 0.003790972 | 266307 | 2 |
| BRHIP3003845 | 0.015866389 | 91566 | 3 |
| BRHIP3003916 | 0.027035576 | 142685 | 13 |
| BRHIP3003961 | 0.008834056 | 155207 | 5 |
| BRHIP3003984 | 0.012195862 | 118711 | 8 |
| BRHIP3004193 | 0.00170946 | 165881 | 1 |
| BRHIP3004215 | 0.012341912 | 70616 | 6 |
| BRHIP3004416 | 0.00170946 | 75491 | 1 |
| BRHIP3004710 | 0.00170946 | 104512 | 1 |
| BRHIP3004725 | 0.00170946 | 197711 | 1 |
| BRHIP3004774 | 0.008479475 | 136828 | 5 |
| BRHIP3004786 | 0.00170946 | 212343 | 1 |
| BRHIP3004863 | 0.00170946 | 228898 | 1 |
| BRHIP3004968 | 0.004980217 | 168537 | 3 |
| BRHIP3005037 | 0.006223659 | 40997 | 4 |
| BRHIP3005137 | 0.00170946 | 260046 | 1 |
| BRHIP3005142 | 0.002804738 | 252493 | 2 |
| BRHIP3005231 | 0.00170946 | 104514 | 1 |
| BRHIP3005307 | 0.003707861 | 186763 | 2 |
| BRHIP3005574 | 0.011891968 | 152925 | 7 |
| BRHIP3005673 | 0.006504352 | 116820 | 4 |
| BRHIP3005768 | 0.003567575 | 205685 | 2 |
| BRHIP3005801 | 0.008940271 | 43547 | 3 |
| BRHIP3005944 | 0.008547301 | 124239 | 5 |
| BRHIP3006279 | 0.00170946 | 208068 | 1 |
| BRHIP3006294 | 0.004201894 | 94675 | 3 |
| BRHIP3006390 | 0.003385007 | 159603 | 2 |
| BRHIP3006449 | 0.00170946 | 116740 | 1 |
| BRHIP3006683 | 0.006952582 | 92246 | 4 |
| BRHIP3006717 | 0.098443203 | 87795 | 35 |
| BRHIP3006786 | 0.00170946 | 49040 | 1 |
| BRHIP3006950 | 0.00170946 | 265026 | 1 |
| BRHIP3007172 | 0.00170946 | 246962 | 1 |
| BRHIP3007183 | 0.012223166 | 17467 | 6 |
| BRHIP3007195 | 0.004514199 | 140090 | 3 |
| BRHIP3007223 | 0.083103968 | 39599 | 19 |
| BRHIP3007239 | 0.00170946 | 217039 | 1 |
| BRHIP3007291 | 0.00170946 | 202817 | 1 |
| BRHIP3007409 | 0.005243122 | 118506 | 3 |
| BRHIP3007424 | 0.00170946 | 140000 | 1 |
| BRHIP3007483 | 0.00170946 | 217103 | 1 |
| BRHIP3007586 | 0.048553699 | 136122 | 23 |
| BRHIP3007609 | 0.00170946 | 280652 | 1 |
| BRHIP3007640 | 0.007506869 | 48333 | 5 |
| BRHIP3007834 | 0.048662157 | 122393 | 22 |
| BRHIP3007960 | 0.00170946 | 240423 | 1 |
| BRHIP3008082 | 0.003790972 | 189488 | 2 |
| BRHIP3008183 | 0.00170946 | 82140 | 1 |
| BRHIP3008313 | 0.005870825 | 229667 | 4 |
| BRHIP3008314 | 0.00170946 | 51827 | 1 |
| BRHIP3008320 | 0.007175979 | 114378 | 4 |
| BRHIP3008344 | 0.003707861 | 221258 | 2 |
| BRHIP3008405 | 0.009523796 | 157043 | 6 |
| BRHIP3008565 | 0.003707861 | 174931 | 2 |
| BRHIP3008598 | 0.00170946 | 133153 | 1 |
| BRHIP3008606 | 0.00170946 | 143870 | 1 |
| BRHIP3008691 | 0.003385007 | 150203 | 2 |
| BRHIP3008714 | 0.00170946 | 221000 | 1 |
| BRHIP3008997 | 0.00170946 | 78492 | 1 |
| BRHIP3009099 | 0.004804956 | 212905 | 2 |
| BRHIP3009140 | 0.00170946 | 258695 | 1 |
| BRHIP3009181 | 0.00170946 | 258703 | 1 |
| BRHIP3009318 | 0.00170946 | 264633 | 1 |
| BRHIP3009427 | 0.00170946 | 240851 | 1 |
| BRHIP3009448 | 0.00170946 | 269727 | 1 |
| BRHIP3009672 | 0.00170946 | 273146 | 1 |
| BRHIP3009753 | 0.143956971 | 259646 | 2 |
| BRHIP3010135 | 0.00170946 | 232047 | 1 |
| BRHIP3010289 | 0.00170946 | 186967 | 1 |
| BRHIP3010529 | 0.00170946 | 232037 | 1 |
| BRHIP3010916 | 0.003790972 | 94696 | 2 |
| BRHIP3011082 | 0.00170946 | 83359 | 1 |
| BRHIP3011241 | 0.00170946 | 212394 | 1 |
| BRHIP3011269 | 0.00341892 | 93536 | 2 |
| BRHIP3011460 | 0.00170946 | 141687 | 1 |
| BRHIP3011567 | 0.00170946 | 269883 | 1 |
| BRHIP3011831 | 0.00170946 | 271999 | 1 |
| BRHIP3012185 | 0.00170946 | 107345 | 1 |
| BRHIP3012289 | 0.00170946 | 266237 | 1 |
| BRHIP3012357 | 0.00170946 | 271523 | 1 |
| BRHIP3012736 | 0.005086013 | 132938 | 3 |
| BRHIP3012745 | 0.00170946 | 210226 | 1 |
| BRHIP3012868 | 0.00170946 | 272345 | 1 |
| BRHIP3012997 | 0.180797099 | 37543 | 9 |
| BRHIP3013078 | 0.00170946 | 245492 | 1 |
| BRHIP3013271 | 0.00170946 | 276773 | 1 |
| BRHIP3013588 | 0.002804738 | 107102 | 2 |
| BRHIP3013698 | 0.00170946 | 245591 | 1 |
| BRHIP3013765 | 0.00170946 | 94917 | 1 |
| BRHIP3013897 | 0.00170946 | 222369 | 1 |
| BRHIP3014237 | 0.00170946 | 195548 | 1 |
| BRHIP3014675 | 0.008836199 | 131462 | 6 |
| BRHIP3015620 | 0.00170946 | 101894 | 1 |
| BRHIP3015751 | 0.00170946 | 270376 | 1 |
| BRHIP3015854 | 0.00170946 | 186322 | 1 |
| BRHIP3016032 | 0.00170946 | 173390 | 1 |
| BRHIP3016213 | 0.00170946 | 149338 | 1 |
| BRHIP3016302 | 0.00170946 | 157213 | 1 |
| BRHIP3016421 | 0.00170946 | 105308 | 1 |
| BRHIP3017109 | 0.00170946 | 208100 | 1 |
| BRHIP3017146 | 0.005107197 | 149132 | 3 |
| BRHIP3017256 | 0.00170946 | 206318 | 1 |
| BRHIP3017325 | 0.00170946 | 282488 | 1 |
| BRHIP3017558 | 0.00170946 | 110149 | 1 |
| BRHIP3017637 | 0.00170946 | 213396 | 1 |
| BRHIP3017855 | 0.043759235 | 14626 | 26 |
| BRHIP3018278 | 0.00429586 | 171897 | 3 |
| BRHIP3018784 | 0.048353746 | 138992 | 10 |
| BRHIP3018797 | 0.125082366 | 84438 | 62 |
| BRHIP3019643 | 0.00170946 | 247653 | 1 |
| BRHIP3019824 | 0.00170946 | 279665 | 1 |
| BRHIP3019880 | 0.00170946 | 237470 | 1 |
| BRHIP3019956 | 0.00170946 | 210038 | 1 |
| BRHIP3020046 | 0.165417468 | 124270 | 15 |
| BRHIP3020155 | 0.00170946 | 150487 | 1 |
| BRHIP3020182 | 0.00170946 | 258319 | 1 |
| BRHIP3020733 | 0.003106616 | 274810 | 2 |
| BRHIP3021019 | 0.021635132 | 94036 | 9 |
| BRHIP3021499 | 0.00170946 | 228855 | 1 |
| BRHIP3021534 | 0.00170946 | 275436 | 1 |
| BRHIP3021778 | 0.00170946 | 243641 | 1 |
| BRHIP3021987 | 0.004691419 | 102623 | 2 |
| BRHIP3022656 | 0.00170946 | 87459 | 1 |
| BRHIP3023922 | 0.00170946 | 110544 | 1 |
| BRHIP3024118 | 0.00170946 | 269579 | 1 |
| BRHIP3024533 | 0.00170946 | 277394 | 1 |
| BRHIP3024703 | 0.00170946 | 271798 | 1 |
| BRHIP3024725 | 0.00170946 | 224006 | 1 |
| BRHIP3024820 | 0.00170946 | 214661 | 1 |
| BRHIP3025161 | 0.00170946 | 226124 | 1 |
| BRHIP3025702 | 0.00170946 | 231785 | 1 |
| BRHIP3025795 | 0.00170946 | 94107 | 1 |
| BRHIP3025844 | 0.00170946 | 220907 | 1 |
| BRHIP3026052 | 0.011182749 | 90953 | 4 |
| BRHIP3026097 | 0.00170946 | 267330 | 1 |
| BRHIP3026231 | 0.00170946 | 278214 | 1 |
| BRHIP3026335 | 0.00170946 | 80649 | 1 |
| BRHIP3026651 | 0.00170946 | 228916 | 1 |
| BRHIP3027105 | 0.003385007 | 190048 | 2 |
| BRHIP3027137 | 0.00170946 | 210699 | 1 |
| BRHIP3027160 | 0.00341892 | 213769 | 2 |
| BRHIP3027191 | 0.00170946 | 72949 | 1 |
| BRHIP3027501 | 0.00170946 | 269793 | 1 |
| BRHIP3027594 | 0.008619675 | 235362 | 5 |
| BRHIP3027651 | 0.00170946 | 80535 | 1 |
| BRHIP3027854 | 0.00170946 | 243843 | 1 |
| BRHIP3027947 | 0.00341892 | 251125 | 2 |
| BRHIP3028246 | 0.007565717 | 154250 | 5 |
| BRHIP3028570 | 0.005243122 | 139192 | 3 |
| BRHIP3028742 | 0.00170946 | 236522 | 1 |
| BRSSN1000056 | 0.027656288 | 186429 | 3 |
| BRSSN1000059 | 0.012449468 | 88729 | 2 |
| BRSSN1000092 | 0.006236358 | 192844 | 1 |
| BRSSN1000153 | 0.006236358 | 187396 | 1 |
| BRSSN2000097 | 0.024199233 | 125506 | 3 |
| BRSSN2000175 | 0.006236358 | 181614 | 1 |
| BRSSN2000197 | 0.007633513 | 146613 | 2 |
| BRSSN2000244 | 0.012438678 | 131125 | 2 |
| BRSSN2000293 | 0.051094821 | 134194 | 11 |
| BRSSN2000295 | 0.024193313 | 145070 | 8 |
| BRSSN2000312 | 0.015573199 | 150566 | 6 |
| BRSSN2000498 | 0.153123455 | 87722 | 34 |
| BRSSN2000518 | 0.006236358 | 145645 | 1 |
| BRSSN2000561 | 0.026721366 | 139273 | 9 |
| BRSSN2000566 | 0.034447498 | 106221 | 13 |
| BRSSN2000684 | 0.035751595 | 97160 | 17 |
| BRSSN2000715 | 0.006236358 | 43038 | 1 |
| BRSSN2000832 | 0.013917225 | 159332 | 6 |
| BRSSN2001213 | 0.008234759 | 116356 | 2 |
| BRSSN2001275 | 0.010080442 | 164175 | 2 |
| BRSSN2001342 | 0.006236358 | 64260 | 1 |
| BRSSN2001426 | 0.006236358 | 137157 | 1 |
| BRSSN2001503 | 0.006236358 | 275065 | 1 |
| BRSSN2001579 | 0.012438678 | 74274 | 2 |
| BRSSN2001758 | 0.017823087 | 101268 | 4 |
| BRSSN2001810 | 0.006236358 | 197379 | 1 |
| BRSSN2001869 | 0.055510128 | 115027 | 20 |
| BRSSN2002160 | 0.009507114 | 176333 | 3 |
| BRSSN2002857 | 0.006236358 | 272943 | 1 |
| BRSSN2002859 | 0.006236358 | 273660 | 1 |
| BRSSN2003012 | 0.038733392 | 71520 | 7 |
| BRSSN2003086 | 0.006236358 | 144135 | 1 |
| BRSSN2003093 | 0.006236358 | 144275 | 1 |
| BRSSN2003820 | 0.006236358 | 148131 | 1 |
| BRSSN2003841 | 0.006236358 | 246032 | 1 |
| BRSSN2003937 | 0.006236358 | 184655 | 1 |
| BRSSN2004142 | 0.006236358 | 159642 | 1 |
| BRSSN2004303 | 0.006236358 | 167085 | 1 |
| BRSSN2004304 | 0.006236358 | 69822 | 1 |
| BRSSN2004496 | 0.007924635 | 165569 | 2 |
| BRSSN2004657 | 0.007331636 | 92659 | 2 |
| BRSSN2004679 | 0.011009329 | 123585 | 3 |
| BRSSN2004686 | 0.006236358 | 49979 | 1 |
| BRSSN2004710 | 0.006236358 | 187801 | 1 |
| BRSSN2004719 | 0.031979606 | 141965 | 7 |
| BRSSN2005847 | 0.007633513 | 151978 | 2 |
| BRSSN2006133 | 0.006236358 | 154482 | 1 |
| BRSSN2006390 | 0.006236358 | 203026 | 1 |
| BRSSN2006575 | 0.006236358 | 136185 | 1 |
| BRSSN2006611 | 0.007924635 | 131281 | 2 |
| BRSSN2006644 | 0.006236358 | 148457 | 1 |
| BRSSN2006892 | 0.010821159 | 143769 | 4 |
| BRSSN2007076 | 0.006236358 | 142064 | 1 |
| BRSSN2007168 | 0.006236358 | 155527 | 1 |
| BRSSN2007202 | 0.010080442 | 150100 | 2 |
| BRSSN2007338 | 0.022669981 | 140230 | 4 |
| BRSSN2007464 | 0.040944704 | 65759 | 4 |
| BRSSN2007504 | 0.006236358 | 243470 | 1 |
| BRSSN2007819 | 0.006236358 | 201862 | 1 |
| BRSSN2008125 | 0.006236358 | 50333 | 1 |
| BRSSN2008419 | 0.006236358 | 175644 | 1 |
| BRSSN2008464 | 0.006236358 | 246347 | 1 |
| BRSSN2008549 | 0.009803933 | 130361 | 3 |
| BRSSN2008797 | 0.008094472 | 176121 | 2 |
| BRSSN2009119 | 0.009151215 | 49353 | 2 |
| BRSSN2009244 | 0.194213688 | 79808 | 4 |
| BRSSN2009378 | 0.006236358 | 191776 | 1 |
| BRSSN2009389 | 0.006236358 | 186636 | 1 |
| BRSSN2009395 | 0.006236358 | 211859 | 1 |
| BRSSN2009518 | 0.09727873 | 61927 | 23 |
| BRSSN2009627 | 0.006236358 | 112261 | 1 |
| BRSSN2009630 | 0.006236358 | 132259 | 1 |
| BRSSN2009702 | 0.006236358 | 158373 | 1 |
| BRSSN2010019 | 0.014437079 | 132968 | 3 |
| BRSSN2010110 | 0.006236358 | 159520 | 1 |
| BRSSN2010587 | 0.007425602 | 131033 | 2 |
| BRSSN2010596 | 0.013979003 | 217318 | 3 |
| BRSSN2010830 | 0.006236358 | 199624 | 1 |
| BRSSN2011262 | 0.059453458 | 36357 | 7 |
| BRSSN2011653 | 0.033815049 | 52320 | 8 |
| BRSSN2011738 | 0.015930911 | 134521 | 7 |
| BRSSN2011799 | 0.060054739 | 141469 | 10 |
| BRSSN2011843 | 0.013999766 | 135118 | 5 |
| BRSSN2012081 | 0.006236358 | 98673 | 1 |
| BRSSN2012103 | 0.006236358 | 123195 | 1 |
| BRSSN2012157 | 0.006236358 | 206979 | 1 |
| BRSSN2012198 | 0.006236358 | 155352 | 1 |
| BRSSN2012254 | 0.006236358 | 31976 | 1 |
| BRSSN2012439 | 0.007633513 | 76028 | 2 |
| BRSSN2012622 | 0.006236358 | 183629 | 1 |
| BRSSN2012691 | 0.006236358 | 168893 | 1 |
| BRSSN2012838 | 0.006236358 | 228888 | 1 |
| BRSSN2013095 | 0.007945818 | 47694 | 2 |
| BRSSN2013544 | 0.006236358 | 281374 | 1 |
| BRSSN2013696 | 0.046913263 | 50403 | 19 |
| BRSSN2013702 | 0.017662866 | 224348 | 4 |
| BRSSN2013733 | 0.007924635 | 125854 | 2 |
| BRSSN2013753 | 0.011362752 | 169855 | 3 |
| BRSSN2013874 | 0.006236358 | 133218 | 1 |
| BRSSN2014112 | 0.006236358 | 121880 | 1 |
| BRSSN2014294 | 0.017996913 | 137087 | 2 |
| BRSSN2014299 | 0.045344114 | 51217 | 10 |
| BRSSN2014424 | 0.010080442 | 43088 | 2 |
| BRSSN2014513 | 0.006236358 | 245942 | 1 |
| BRSSN2014556 | 0.006236358 | 162834 | 1 |
| BRSSN2014573 | 0.006236358 | 262723 | 1 |
| BRSSN2014610 | 0.046931868 | 114219 | 19 |
| BRSSN2014636 | 0.006236358 | 227598 | 1 |
| BRSSN2014685 | 0.011799878 | 117723 | 4 |
| BRSSN2015199 | 0.007425602 | 128489 | 2 |
| BRSSN2015238 | 0.048945168 | 137301 | 17 |
| BRSSN2015369 | 0.006236358 | 129318 | 1 |
| BRSSN2015493 | 0.045840127 | 58109 | 5 |
| BRSSN2015497 | 0.023428368 | 104848 | 16 |
| BRSSN2015554 | 0.012172133 | 83197 | 2 |
| BRSSN2015707 | 0.006236358 | 70611 | 1 |
| BRSSN2015773 | 0.006236358 | 145073 | 1 |
| BRSSN2015907 | 0.006236358 | 159863 | 1 |
| BRSSN2015982 | 0.010313595 | 35466 | 3 |
| BRSSN2016589 | 0.006236358 | 143329 | 1 |
| BRSSN2016768 | 0.012472716 | 208455 | 2 |
| BRSSN2016905 | 0.006236358 | 167075 | 1 |
| BRSSN2017297 | 0.006236358 | 210890 | 1 |
| BRSSN2017422 | 0.01165368 | 214642 | 4 |
| BRSSN2017530 | 0.006236358 | 176547 | 1 |
| BRSSN2017682 | 0.006236358 | 176557 | 1 |
| BRSSN2017757 | 0.006236358 | 220412 | 1 |
| BRSSN2018218 | 0.006236358 | 130807 | 1 |
| BRSSN2018440 | 0.00831787 | 72053 | 2 |
| BRSSN2018447 | 0.025638602 | 119395 | 10 |
| BRSSN2018581 | 0.012438678 | 147007 | 2 |
| BRSSN2018731 | 0.006236358 | 125747 | 1 |
| BRSSN2018771 | 0.006236358 | 176447 | 1 |
| BRSSN2018925 | 0.006236358 | 181854 | 1 |
| BRSTN1000083 | 0.262421535 | 94790 | 41 |
| BRSTN1000097 | 0.006041566 | 65953 | 1 |
| BRSTN2000058 | 0.069460404 | 49960 | 16 |
| BRSTN2000070 | 0.007751026 | 163797 | 2 |
| BRSTN2000220 | 0.048836214 | 143433 | 18 |
| BRSTN2000312 | 0.006041566 | 238650 | 1 |
| BRSTN2000367 | 0.007136844 | 171047 | 2 |
| BRSTN2000536 | 0.007751026 | 132193 | 2 |
| BRSTN2000872 | 0.006041566 | 51603 | 1 |
| BRSTN2001067 | 0.027064268 | 29260 | 6 |
| BRSTN2001613 | 0.006041566 | 148979 | 1 |
| BRSTN2001801 | 0.006041566 | 244595 | 1 |
| BRSTN2002105 | 0.006041566 | 190995 | 1 |
| BRSTN2002400 | 0.006041566 | 169593 | 1 |
| BRSTN2002532 | 0.006041566 | 2576 | 1 |
| BRSTN2002691 | 0.006041566 | 242370 | 1 |
| BRSTN2003590 | 0.006041566 | 195725 | 1 |
| BRSTN2003835 | 0.006041566 | 152669 | 1 |
| BRSTN2004863 | 0.016817414 | 91234 | 4 |
| BRSTN2004869 | 0.007918408 | 79994 | 2 |
| BRSTN2004987 | 0.006041566 | 155975 | 1 |
| BRSTN2005721 | 0.006041566 | 53315 | 1 |
| BRSTN2006134 | 0.008956423 | 208265 | 2 |
| BRSTN2006306 | 0.006041566 | 214754 | 1 |
| BRSTN2006466 | 0.006041566 | 271293 | 1 |
| BRSTN2006580 | 0.00723081 | 189831 | 2 |
| BRSTN2006583 | 0.00789968 | 81364 | 2 |
| BRSTN2006638 | 0.006041566 | 284114 | 1 |
| BRSTN2006865 | 0.006041566 | 147851 | 1 |
| BRSTN2007000 | 0.006041566 | 219546 | 1 |
| BRSTN2007118 | 0.006041566 | 240062 | 1 |
| BRSTN2007284 | 0.00988565 | 102381 | 2 |
| BRSTN2007765 | 0.006041566 | 252330 | 1 |
| BRSTN2008052 | 0.015304811 | 163440 | 7 |
| BRSTN2008283 | 0.006041566 | 237711 | 1 |
| BRSTN2008367 | 0.006041566 | 189295 | 1 |
| BRSTN2008418 | 0.006041566 | 81302 | 1 |
| BRSTN2008457 | 0.006041566 | 232356 | 1 |
| BRSTN2008475 | 0.006041566 | 232370 | 1 |
| BRSTN2008930 | 0.006041566 | 174103 | 1 |
| BRSTN2009247 | 0.006041566 | 166679 | 1 |
| BRSTN2010089 | 0.025396583 | 4319 | 8 |
| BRSTN2010107 | 0.500891046 | 1671 | 135 |
| BRSTN2010363 | 0.006041566 | 51123 | 1 |
| BRSTN2010416 | 0.006041566 | 283711 | 1 |
| BRSTN2010500 | 0.006041566 | 275957 | 1 |
| BRSTN2010569 | 0.006041566 | 27001 | 1 |
| BRSTN2010750 | 0.016472575 | 153391 | 6 |
| BRSTN2010923 | 0.006041566 | 215119 | 1 |
| BRSTN2011211 | 0.006041566 | 204863 | 1 |
| BRSTN2011597 | 0.00723081 | 189486 | 2 |
| BRSTN2011688 | 0.006041566 | 159393 | 1 |
| BRSTN2011799 | 0.006041566 | 167121 | 1 |
| BRSTN2011961 | 0.57856361 | 4537 | 64 |
| BRSTN2012069 | 17.71546276 | 31469 | 1260 |
| BRSTN2012089 | 0.028886371 | 83043 | 8 |
| BRSTN2012174 | 0.828439861 | 34274 | 85 |
| BRSTN2012284 | 0.006041566 | 219502 | 1 |
| BRSTN2012320 | 0.006041566 | 79763 | 1 |
| BRSTN2012380 | 0.006041566 | 268752 | 1 |
| BRSTN2012451 | 0.20286663 | 163701 | 7 |
| BRSTN2013171 | 0.048353119 | 99506 | 8 |
| BRSTN2013354 | 0.012083132 | 62736 | 2 |
| BRSTN2013502 | 0.068941636 | 116848 | 14 |
| BRSTN2013931 | 0.17342111 | 87169 | 14 |
| BRSTN2014490 | 0.018186009 | 234542 | 3 |
| BRSTN2014633 | 0.006041566 | 169243 | 1 |
| BRSTN2014751 | 0.006041566 | 201389 | 1 |
| BRSTN2014770 | 0.006041566 | 144964 | 1 |
| BRSTN2015015 | 0.011953716 | 139496 | 5 |
| BRSTN2015889 | 0.043256602 | 142464 | 15 |
| BRSTN2015978 | 5.522814481 | 6913 | 1008 |
| BRSTN2016066 | 0.006041566 | 229573 | 1 |
| BRSTN2016335 | 0.006041566 | 208565 | 1 |
| BRSTN2016470 | 0.765521402 | 78918 | 124 |
| BRSTN2016678 | 0.018261552 | 6374 | 3 |
| BRSTN2016892 | 0.056151978 | 99323 | 5 |
| BRSTN2016918 | 0.006041566 | 137234 | 1 |
| BRSTN2016992 | 0.340483632 | 93672 | 83 |
| BRSTN2017104 | 0.085935761 | 146119 | 12 |
| BRSTN2017151 | 0.006041566 | 133409 | 1 |
| BRSTN2017237 | 0.006041566 | 173095 | 1 |
| BRSTN2017379 | 0.006041566 | 1843 | 1 |
| BRSTN2017761 | 0.007918408 | 120371 | 2 |
| BRSTN2017771 | 0.006041566 | 173745 | 1 |
| BRSTN2017995 | 0.006041566 | 269660 | 1 |
| BRSTN2018077 | 0.00988565 | 128207 | 2 |
| BRSTN2018083 | 0.007751026 | 186991 | 2 |
| BRSTN2018596 | 0.006041566 | 173603 | 1 |
| BRSTN2018692 | 0.006041566 | 136281 | 1 |
| BRSTN2018712 | 0.006041566 | 275897 | 1 |
| BRSTN2019079 | 0.006041566 | 243556 | 1 |
| BRSTN2019129 | 0.006041566 | 235661 | 1 |
| BRTHA1000021 | 0.009756234 | 154304 | 5 |
| BRTHA1000063 | 0.001858114 | 142993 | 1 |
| BRTHA1000311 | 0.056784799 | 131833 | 27 |
| BRTHA2000057 | 0.003856516 | 191151 | 2 |
| BRTHA2000474 | 0.001858114 | 132639 | 1 |
| BRTHA2000514 | 0.001858114 | 231066 | 1 |
| BRTHA2000574 | 0.001858114 | 198864 | 1 |
| BRTHA2000855 | 0.001858114 | 195311 | 1 |
| BRTHA2000969 | 0.001858114 | 253804 | 1 |
| BRTHA2000972 | 0.001858114 | 253807 | 1 |
| BRTHA2001304 | 0.001858114 | 133019 | 1 |
| BRTHA2001370 | 0.001858114 | 103288 | 1 |
| BRTHA2001462 | 0.001858114 | 103371 | 1 |
| BRTHA2001717 | 0.001858114 | 237101 | 1 |
| BRTHA2001812 | 0.019660235 | 157418 | 3 |
| BRTHA2001953 | 0.017503822 | 174353 | 5 |
| BRTHA2002091 | 0.001858114 | 114392 | 1 |
| BRTHA2002115 | 0.001858114 | 231102 | 1 |
| BRTHA2002133 | 0.001858114 | 231132 | 1 |
| BRTHA2002281 | 0.001858114 | 240089 | 1 |
| BRTHA2002376 | 0.001858114 | 201758 | 1 |
| BRTHA2002442 | 0.005425689 | 209697 | 3 |
| BRTHA2002493 | 0.001858114 | 218275 | 1 |
| BRTHA2002565 | 0.001858114 | 225816 | 1 |
| BRTHA2002608 | 0.001858114 | 196607 | 1 |
| BRTHA2002702 | 0.001858114 | 103255 | 1 |
| BRTHA2002721 | 0.001858114 | 109357 | 1 |
| BRTHA2002726 | 0.007800238 | 101738 | 2 |
| BRTHA2002741 | 0.001858114 | 279940 | 1 |
| BRTHA2002808 | 0.014452573 | 103836 | 2 |
| BRTHA2002896 | 0.001858114 | 264368 | 1 |
| BRTHA2002903 | 0.001858114 | 264384 | 1 |
| BRTHA2003030 | 0.001858114 | 163926 | 1 |
| BRTHA2003110 | 0.001858114 | 249079 | 1 |
| BRTHA2003116 | 0.001858114 | 254352 | 1 |
| BRTHA2003436 | 0.001858114 | 95937 | 1 |
| BRTHA2003461 | 0.076340879 | 104645 | 11 |
| BRTHA2003759 | 0.001858114 | 189664 | 1 |
| BRTHA2003794 | 0.001858114 | 62315 | 1 |
| BRTHA2004350 | 0.041138741 | 14663 | 17 |
| BRTHA2004361 | 0.003856516 | 244165 | 2 |
| BRTHA2004371 | 0.001858114 | 241049 | 1 |
| BRTHA2004582 | 0.001858114 | 257605 | 1 |
| BRTHA2004629 | 0.001858114 | 69587 | 1 |
| BRTHA2004639 | 0.001858114 | 195374 | 1 |
| BRTHA2004642 | 0.001858114 | 211464 | 1 |
| BRTHA2004812 | 0.001858114 | 226051 | 1 |
| BRTHA2004821 | 0.001858114 | 175218 | 1 |
| BRTHA2004978 | 0.001858114 | 267648 | 1 |
| BRTHA2004992 | 0.001858114 | 252121 | 1 |
| BRTHA2005005 | 0.007432458 | 102370 | 4 |
| BRTHA2005013 | 0.015015825 | 102359 | 3 |
| BRTHA2005448 | 0.007432458 | 99099 | 4 |
| BRTHA2005579 | 0.001858114 | 259845 | 1 |
| BRTHA2005831 | 0.003856516 | 245772 | 2 |
| BRTHA2005864 | 0.103266523 | 134399 | 27 |
| BRTHA2005942 | 0.001858114 | 175483 | 1 |
| BRTHA2005956 | 0.001858114 | 219073 | 1 |
| BRTHA2006075 | 0.001858114 | 216815 | 1 |
| BRTHA2006146 | 0.001858114 | 228679 | 1 |
| BRTHA2006194 | 0.001858114 | 225960 | 1 |
| BRTHA2006232 | 0.001858114 | 211410 | 1 |
| BRTHA2006720 | 0.001858114 | 228104 | 1 |
| BRTHA2006735 | 0.001858114 | 250988 | 1 |
| BRTHA2006974 | 0.001858114 | 181051 | 1 |
| BRTHA2006975 | 0.073700379 | 181050 | 3 |
| BRTHA2007060 | 0.001858114 | 261826 | 1 |
| BRTHA2007122 | 0.001858114 | 144211 | 1 |
| BRTHA2007422 | 0.001858114 | 271107 | 1 |
| BRTHA2007603 | 0.001858114 | 207172 | 1 |
| BRTHA2007665 | 0.001858114 | 229738 | 1 |
| BRTHA2007998 | 0.194298765 | 63084 | 15 |
| BRTHA2008316 | 0.001858114 | 211650 | 1 |
| BRTHA2008335 | 0.020582403 | 94910 | 6 |
| BRTHA2008502 | 0.00774451 | 92286 | 3 |
| BRTHA2008513 | 0.003567575 | 177419 | 2 |
| BRTHA2008527 | 0.001858114 | 229687 | 1 |
| BRTHA2008535 | 0.001858114 | 185415 | 1 |
| BRTHA2008598 | 0.001858114 | 255151 | 1 |
| BRTHA2008669 | 0.001858114 | 267658 | 1 |
| BRTHA2008955 | 0.003047359 | 144589 | 2 |
| BRTHA2009311 | 0.001858114 | 166999 | 1 |
| BRTHA2009349 | 0.001858114 | 211638 | 1 |
| BRTHA2009486 | 0.01532813 | 157623 | 3 |
| BRTHA2009803 | 0.001858114 | 280740 | 1 |
| BRTHA2009846 | 0.001858114 | 200423 | 1 |
| BRTHA2009886 | 0.008060434 | 185690 | 2 |
| BRTHA2009972 | 0.001858114 | 93029 | 1 |
| BRTHA2010008 | 0.001858114 | 98357 | 1 |
| BRTHA2010033 | 0.001858114 | 96595 | 1 |
| BRTHA2010073 | 0.001858114 | 282860 | 1 |
| BRTHA2010097 | 0.039909865 | 105637 | 2 |
| BRTHA2010191 | 0.001858114 | 102568 | 1 |
| BRTHA2010397 | 0.001858114 | 266931 | 1 |
| BRTHA2010608 | 0.003856516 | 164610 | 2 |
| BRTHA2010672 | 0.001858114 | 193092 | 1 |
| BRTHA2010884 | 0.001858114 | 264909 | 1 |
| BRTHA2010907 | 0.001858114 | 555 | 1 |
| BRTHA2011062 | 0.001858114 | 192653 | 1 |
| BRTHA2011138 | 0.603047235 | 155783 | 12 |
| BRTHA2011194 | 0.003047359 | 217167 | 2 |
| BRTHA2011321 | 0.001858114 | 217136 | 1 |
| BRTHA2011351 | 0.001858114 | 102058 | 1 |
| BRTHA2011500 | 0.003716229 | 179680 | 2 |
| BRTHA2011641 | 0.003546391 | 195266 | 2 |
| BRTHA2011691 | 0.00325527 | 145364 | 2 |
| BRTHA2012129 | 0.001858114 | 142810 | 1 |
| BRTHA2012183 | 0.003856516 | 203312 | 2 |
| BRTHA2012189 | 0.001858114 | 214683 | 1 |
| BRTHA2012351 | 0.001858114 | 177092 | 1 |
| BRTHA2012392 | 0.004840074 | 112107 | 2 |
| BRTHA2012466 | 0.003716229 | 93930 | 2 |
| BRTHA2012555 | 0.001858114 | 252381 | 1 |
| BRTHA2012562 | 0.001858114 | 243552 | 1 |
| BRTHA2012714 | 0.133679029 | 5064 | 22 |
| BRTHA2012980 | 0.013377525 | 185533 | 2 |
| BRTHA2013054 | 0.027893359 | 48870 | 19 |
| BRTHA2013262 | 0.001858114 | 266515 | 1 |
| BRTHA2013275 | 0.003734956 | 241994 | 2 |
| BRTHA2013426 | 0.001858114 | 247578 | 1 |
| BRTHA2013460 | 0.001858114 | 252305 | 1 |
| BRTHA2013515 | 0.003533661 | 119126 | 2 |
| BRTHA2013540 | 0.004943546 | 197960 | 3 |
| BRTHA2013595 | 0.001858114 | 247598 | 1 |
| BRTHA2013610 | 0.001858114 | 243614 | 1 |
| BRTHA2013707 | 0.001858114 | 247505 | 1 |
| BRTHA2013942 | 0.001858114 | 77541 | 1 |
| BRTHA2014287 | 0.001858114 | 95391 | 1 |
| BRTHA2014334 | 0.001858114 | 242123 | 1 |
| BRTHA2014381 | 0.001858114 | 254585 | 1 |
| BRTHA2014578 | 0.001858114 | 246118 | 1 |
| BRTHA2014647 | 0.001858114 | 272576 | 1 |
| BRTHA2014663 | 0.009457589 | 167559 | 3 |
| BRTHA2014792 | 0.001858114 | 258150 | 1 |
| BRTHA2014828 | 0.001858114 | 258121 | 1 |
| BRTHA2014909 | 0.001858114 | 102542 | 1 |
| BRTHA2014975 | 0.001858114 | 97542 | 1 |
| BRTHA2014997 | 0.00325527 | 101663 | 2 |
| BRTHA2015406 | 0.001858114 | 230120 | 1 |
| BRTHA2015478 | 0.001858114 | 170434 | 1 |
| BRTHA2015696 | 0.001858114 | 218651 | 1 |
| BRTHA2015700 | 0.001858114 | 236623 | 1 |
| BRTHA2015853 | 0.001858114 | 214472 | 1 |
| BRTHA2015878 | 0.001858114 | 138376 | 1 |
| BRTHA2016179 | 0.001858114 | 214927 | 1 |
| BRTHA2016215 | 0.004772971 | 151443 | 2 |
| BRTHA2016267 | 0.001858114 | 202409 | 1 |
| BRTHA2016318 | 0.001858114 | 182204 | 1 |
| BRTHA2016496 | 0.001858114 | 247417 | 1 |
| BRTHA2016543 | 0.001858114 | 99657 | 1 |
| BRTHA2016552 | 0.001858114 | 271066 | 1 |
| BRTHA2016577 | 0.071012737 | 94909 | 12 |
| BRTHA2016765 | 0.014240074 | 231452 | 3 |
| BRTHA2016918 | 0.017093753 | 102338 | 4 |
| BRTHA2017178 | 0.003856516 | 202308 | 2 |
| BRTHA2017353 | 0.001858114 | 171429 | 1 |
| BRTHA2017364 | 0.001858114 | 197914 | 1 |
| BRTHA2017972 | 0.009929338 | 177613 | 3 |
| BRTHA2017985 | 0.003734956 | 92516 | 2 |
| BRTHA2018011 | 0.003716229 | 138317 | 2 |
| BRTHA2018129 | 0.001858114 | 50830 | 1 |
| BRTHA2018165 | 0.001858114 | 100289 | 1 |
| BRTHA2018304 | 0.001858114 | 96986 | 1 |
| BRTHA2018344 | 0.001858114 | 202403 | 1 |
| BRTHA2018420 | 0.001858114 | 231424 | 1 |
| BRTHA2018443 | 0.007582332 | 144708 | 5 |
| BRTHA2018504 | 0.004930817 | 220141 | 3 |
| BRTHA2018558 | 0.001858114 | 270957 | 1 |
| BRTHA2018591 | 0.001858114 | 59186 | 1 |
| BRTHA2018624 | 0.001858114 | 180293 | 1 |
| BRTHA2018706 | 0.001858114 | 209675 | 1 |
| BRTHA2018707 | 0.00325527 | 136620 | 2 |
| BRTHA2019014 | 0.001858114 | 101362 | 1 |
| BRTHA2019022 | 0.001858114 | 103155 | 1 |
| BRTHA2019048 | 0.001858114 | 97789 | 1 |
| BRTHA2019073 | 0.00325527 | 101754 | 2 |
| BRTHA2019726 | 0.001858114 | 266333 | 1 |
| BRTHA2019743 | 0.001858114 | 201797 | 1 |
| BRTHA2020400 | 0.001858114 | 164964 | 1 |
| BRTHA2020566 | 0.003716229 | 164957 | 2 |
| BRTHA2020642 | 0.001858114 | 128505 | 1 |
| BRTHA2020695 | 0.001858114 | 150695 | 1 |
| BRTHA2020721 | 0.001858114 | 270933 | 1 |
| BRTHA2020781 | 0.001858114 | 147171 | 1 |
| BRTHA2020811 | 0.001858114 | 280153 | 1 |
| BRTHA2020910 | 0.001858114 | 105888 | 1 |
| BRTHA2021212 | 0.001858114 | 196532 | 1 |
| BRTHA2021440 | 0.00325527 | 254163 | 2 |
| BRTHA2021450 | 0.001858114 | 201953 | 1 |
| BRTHA2022074 | 0.001858114 | 150658 | 1 |
| BRTHA2022914 | 0.001858114 | 130738 | 1 |
| BRTHA2022968 | 0.001858114 | 33602 | 1 |
| BRTHA2023402 | 0.001858114 | 136956 | 1 |
| BRTHA2023437 | 0.001858114 | 140780 | 1 |
| BRTHA2024177 | 0.001858114 | 130641 | 1 |
| BRTHA2024354 | 0.001858114 | 126649 | 1 |
| BRTHA2024712 | 0.001858114 | 124242 | 1 |
| BRTHA2025869 | 0.001858114 | 10842 | 1 |
| BRTHA2026071 | 0.013650469 | 31734 | 6 |
| BRTHA2026290 | 0.001858114 | 269857 | 1 |
| BRTHA2026311 | 0.001858114 | 42711 | 1 |
| BRTHA2027227 | 0.001858114 | 163735 | 1 |
| BRTHA2027229 | 0.183063644 | 135880 | 10 |
| BRTHA2027250 | 0.001858114 | 227498 | 1 |
| BRTHA2027546 | 0.001858114 | 274421 | 1 |
| BRTHA2028297 | 0.001858114 | 155839 | 1 |
| BRTHA2029969 | 0.001858114 | 118071 | 1 |
| BRTHA2030036 | 0.001858114 | 67404 | 1 |
| BRTHA2030213 | 0.001858114 | 192840 | 1 |
| BRTHA2031517 | 0.003567575 | 152024 | 2 |
| BRTHA2031800 | 0.001858114 | 151996 | 1 |
| BRTHA2031917 | 0.001858114 | 148553 | 1 |
| BRTHA2032763 | 0.001858114 | 188359 | 1 |
| BRTHA2032845 | 0.001858114 | 130948 | 1 |
| BRTHA2033122 | 0.001858114 | 119146 | 1 |
| BRTHA2033155 | 0.039396899 | 67281 | 16 |
| BRTHA2033264 | 0.005702198 | 146347 | 2 |
| BRTHA2033320 | 0.001858114 | 122715 | 1 |
| BRTHA2033469 | 0.004772971 | 149299 | 2 |
| BRTHA2033683 | 0.001858114 | 71947 | 1 |
| BRTHA2034281 | 0.001858114 | 165261 | 1 |
| BRTHA2034576 | 0.001858114 | 69618 | 1 |
| BRTHA2034874 | 0.001858114 | 48881 | 1 |
| BRTHA2035448 | 0.001858114 | 129756 | 1 |
| BRTHA2035743 | 0.024691211 | 92447 | 14 |
| BRTHA2036055 | 0.001858114 | 155229 | 1 |
| BRTHA2036295 | 0.001858114 | 255162 | 1 |
| BRTHA2036660 | 0.001858114 | 118512 | 1 |
| BRTHA2037247 | 0.001858114 | 150668 | 1 |
| BRTHA2038279 | 0.001858114 | 129561 | 1 |
| BRTHA2038345 | 0.001858114 | 126099 | 1 |
| BRTHA2038353 | 0.001858114 | 278798 | 1 |
| BRTHA2038396 | 0.001858114 | 169118 | 1 |
| BRTHA3000252 | 0.003716229 | 23295 | 2 |
| BRTHA3000273 | 0.00325527 | 195619 | 2 |
| BRTHA3000296 | 0.001858114 | 164811 | 1 |
| BRTHA3000297 | 0.007135149 | 152696 | 4 |
| BRTHA3000325 | 0.001858114 | 206242 | 1 |
| BRTHA3000456 | 0.002953393 | 78216 | 2 |
| BRTHA3000633 | 0.004902996 | 30816 | 2 |
| BRTHA3000789 | 0.004756819 | 123818 | 3 |
| BRTHA3001221 | 0.001858114 | 167348 | 1 |
| BRTHA3001623 | 0.001858114 | 163468 | 1 |
| BRTHA3001721 | 0.01422502 | 117928 | 8 |
| BRTHA3002265 | 0.001858114 | 246225 | 1 |
| BRTHA3002401 | 0.001858114 | 190010 | 1 |
| BRTHA3002411 | 0.001858114 | 255497 | 1 |
| BRTHA3002427 | 0.058609412 | 82080 | 18 |
| BRTHA3002864 | 0.00325527 | 181358 | 2 |
| BRTHA3002933 | 0.001858114 | 248080 | 1 |
| BRTHA3002955 | 0.001858114 | 210602 | 1 |
| BRTHA3003000 | 0.009770019 | 152207 | 3 |
| BRTHA3003023 | 0.082905216 | 124881 | 13 |
| BRTHA3003074 | 0.014881132 | 14957 | 6 |
| BRTHA3003225 | 0.003939626 | 227824 | 2 |
| BRTHA3003339 | 0.001858114 | 215609 | 1 |
| BRTHA3003343 | 0.001858114 | 256394 | 1 |
| BRTHA3003417 | 0.005444416 | 161511 | 3 |
| BRTHA3003449 | 0.001858114 | 249570 | 1 |
| BRTHA3003474 | 0.001858114 | 276685 | 1 |
| BRTHA3003490 | 0.103213241 | 113322 | 22 |
| BRTHA3003704 | 0.003047359 | 130304 | 2 |
| BRTHA3003736 | 0.07789303 | 98288 | 21 |
| BRTHA3004307 | 0.055621142 | 108282 | 33 |
| BRTHA3004432 | 0.005221938 | 131871 | 3 |
| BRTHA3004475 | 0.003546391 | 224738 | 2 |
| BRTHA3004502 | 0.001858114 | 273993 | 1 |
| BRTHA3004610 | 0.001858114 | 273198 | 1 |
| BRTHA3005046 | 0.003567575 | 173422 | 2 |
| BRTHA3005735 | 0.001858114 | 23404 | 1 |
| BRTHA3005988 | 0.005391776 | 145838 | 3 |
| BRTHA3006318 | 0.001858114 | 179015 | 1 |
| BRTHA3006593 | 0.001858114 | 257082 | 1 |
| BRTHA3006856 | 0.001858114 | 129740 | 1 |
| BRTHA3007113 | 0.001858114 | 262617 | 1 |
| BRTHA3007130 | 0.001858114 | 234521 | 1 |
| BRTHA3007148 | 0.001858114 | 243016 | 1 |
| BRTHA3007319 | 0.001858114 | 239823 | 1 |
| BRTHA3007469 | 0.001858114 | 263316 | 1 |
| BRTHA3007472 | 0.042940532 | 130274 | 12 |
| BRTHA3007532 | 0.001858114 | 89120 | 1 |
| BRTHA3007662 | 0.005277035 | 176725 | 3 |
| BRTHA3007685 | 0.003047359 | 237059 | 2 |
| BRTHA3007769 | 0.001858114 | 270404 | 1 |
| BRTHA3007922 | 0.010736471 | 253856 | 4 |
| BRTHA3008143 | 0.001858114 | 249767 | 1 |
| BRTHA3008293 | 0.001858114 | 264217 | 1 |
| BRTHA3008310 | 0.001858114 | 158621 | 1 |
| BRTHA3008386 | 0.001858114 | 8934 | 1 |
| BRTHA3008520 | 0.003533661 | 239799 | 2 |
| BRTHA3008608 | 0.001858114 | 165175 | 1 |
| BRTHA3008694 | 0.001858114 | 205546 | 1 |
| BRTHA3008778 | 0.006744365 | 84754 | 4 |
| BRTHA3008826 | 0.001858114 | 29110 | 1 |
| BRTHA3009037 | 0.001858114 | 145094 | 1 |
| BRTHA3009090 | 0.014235922 | 111161 | 6 |
| BRTHA3009291 | 0.001858114 | 271541 | 1 |
| BRTHA3009858 | 0.007224294 | 107617 | 3 |
| BRTHA3010135 | 0.008373735 | 65474 | 4 |
| BRTHA3010212 | 0.030329007 | 18697 | 10 |
| BRTHA3010366 | 0.001858114 | 218438 | 1 |
| BRTHA3010469 | 0.003716229 | 175354 | 2 |
| BRTHA3010530 | 0.011984525 | 115899 | 7 |
| BRTHA3010540 | 0.049777951 | 123221 | 3 |
| BRTHA3010717 | 0.001858114 | 71531 | 1 |
| BRTHA3011149 | 0.001858114 | 138502 | 1 |
| BRTHA3011187 | 0.001858114 | 141701 | 1 |
| BRTHA3011194 | 0.047405969 | 38798 | 15 |
| BRTHA3011229 | 0.001858114 | 124896 | 1 |
| BRTHA3011265 | 0.019638355 | 87098 | 9 |
| BRTHA3011306 | 0.013605416 | 187293 | 3 |
| BRTHA3011361 | 0.002953393 | 109699 | 2 |
| BRTHA3011510 | 0.001858114 | 276094 | 1 |
| BRTHA3011892 | 0.001858114 | 188733 | 1 |
| BRTHA3011998 | 0.004236603 | 188789 | 3 |
| BRTHA3012265 | 0.005277035 | 188376 | 3 |
| BRTHA3013860 | 0.001858114 | 256911 | 1 |
| BRTHA3013882 | 0.001858114 | 281554 | 1 |
| BRTHA3013884 | 0.001858114 | 252473 | 1 |
| BRTHA3013981 | 0.001858114 | 258272 | 1 |
| BRTHA3014000 | 0.001858114 | 242794 | 1 |
| BRTHA3014105 | 0.001858114 | 280016 | 1 |
| BRTHA3014507 | 0.001858114 | 128803 | 1 |
| BRTHA3014547 | 0.001858114 | 145850 | 1 |
| BRTHA3014691 | 0.001858114 | 186262 | 1 |
| BRTHA3014835 | 0.001858114 | 203108 | 1 |
| BRTHA3014854 | 0.001858114 | 226205 | 1 |
| BRTHA3014908 | 0.001858114 | 80719 | 1 |
| BRTHA3014920 | 0.001858114 | 79620 | 1 |
| BRTHA3015815 | 0.001858114 | 195542 | 1 |
| BRTHA3015910 | 0.011230867 | 93166 | 4 |
| BRTHA3016616 | 0.001858114 | 240126 | 1 |
| BRTHA3016835 | 0.001858114 | 231788 | 1 |
| BRTHA3016845 | 0.001858114 | 240138 | 1 |
| BRTHA3016917 | 0.001858114 | 84707 | 1 |
| BRTHA3017047 | 0.001858114 | 224085 | 1 |
| BRTHA3017589 | 0.001858114 | 60549 | 1 |
| BRTHA3017791 | 0.141739371 | 91975 | 53 |
| BRTHA3017848 | 0.003533661 | 148953 | 2 |
| BRTHA3018409 | 0.001858114 | 97049 | 1 |
| BRTHA3018514 | 0.001858114 | 84924 | 1 |
| BRTHA3018617 | 0.001858114 | 281297 | 1 |
| BRTHA3018623 | 0.001858114 | 228742 | 1 |
| BRTHA3018656 | 0.001858114 | 154137 | 1 |
| BRTHA3019048 | 0.001858114 | 207399 | 1 |
| BRTHA3019105 | 0.001858114 | 222076 | 1 |
| BRTHA3019183 | 0.001858114 | 228721 | 1 |
| BRTHA3020000 | 0.001858114 | 174228 | 1 |
| BRTHA3020314 | 0.016455223 | 108610 | 9 |
| BRTHA3020369 | 0.001858114 | 238482 | 1 |
| BRTHA3020771 | 0.005243122 | 139192 | 3 |
| BRTHA3021569 | 0.001858114 | 226080 | 1 |
| BRTHA3021708 | 0.003047359 | 259965 | 2 |
| BRTHA3021786 | 0.001858114 | 247601 | 1 |
| BRTHA3021971 | 0.086324188 | 118853 | 12 |
| BRTHA3022641 | 0.009471572 | 132297 | 4 |
| BRTHA3023403 | 0.022556145 | 103660 | 12 |
| BRTHA3023523 | 0.001858114 | 161537 | 1 |
| BRTHA3023590 | 0.001858114 | 236396 | 1 |
| BRTHA3023929 | 0.001858114 | 216919 | 1 |
| BRTHA3024233 | 0.001858114 | 167329 | 1 |
| BRTHA3024600 | 0.001858114 | 216463 | 1 |
| BRTHA3025073 | 0.001858114 | 258890 | 1 |
| BRTHA3025789 | 0.001858114 | 221043 | 1 |
| BRTHA3025826 | 0.001858114 | 240471 | 1 |
| BRTHA3026161 | 0.001858114 | 262193 | 1 |
| BRTHA3026180 | 0.001858114 | 222750 | 1 |
| BRTHA3026507 | 0.047956841 | 76565 | 24 |
| BRTHA3026556 | 0.001858114 | 234235 | 1 |
| BRTHA3026916 | 0.003546391 | 104037 | 2 |
| BRTHA3027171 | 0.001858114 | 263889 | 1 |
| BRTHA3027318 | 0.001858114 | 197580 | 1 |
| BRTHA3027638 | 0.001858114 | 234184 | 1 |
| BRTHA3027820 | 0.001858114 | 258849 | 1 |
| BRTHA3027879 | 0.003856516 | 19874 | 2 |
| BRTHA3027957 | 0.001858114 | 218841 | 1 |
| BRTHA3028339 | 0.001858114 | 77129 | 1 |
| BRTHA3028505 | 0.001858114 | 60728 | 1 |
| BRTHA3028782 | 0.001858114 | 253394 | 1 |
| CD34C2000051 | 0.070372977 | 2976 | 1 |
| CD34C2000152 | 0.070372977 | 147255 | 1 |
| CD34C2000175 | 0.070372977 | 113756 | 1 |
| CD34C2000443 | 0.070372977 | 53395 | 1 |
| CD34C3000125 | 0.072082437 | 158107 | 2 |
| CD34C3000252 | 0.204145114 | 118638 | 22 |
| CD34C3000314 | 0.070372977 | 154623 | 1 |
| CD34C3000411 | 0.219156642 | 71428 | 31 |
| CD34C3000424 | 0.073177715 | 77507 | 3 |
| CD34C3000494 | 0.070372977 | 167343 | 1 |
| CERVX1000042 | 0.034626039 | 67344 | 1 |
| CERVX2000812 | 0.034626039 | 276282 | 1 |
| CERVX2000968 | 0.034626039 | 195678 | 1 |
| CERVX2002006 | 0.034626039 | 174407 | 1 |
| CERVX2002013 | 0.034626039 | 169904 | 1 |
| CERVX2002430 | 0.034626039 | 189997 | 1 |
| CERVX2002770 | 0.071082473 | 181132 | 2 |
| CHONS1000058 | 0.037119525 | 260326 | 1 |
| CHONS2000172 | 0.04484944 | 146038 | 3 |
| CHONS2000797 | 0.03851668 | 213245 | 2 |
| CHONS2001287 | 0.037119525 | 12902 | 1 |
| CHONS2001382 | 0.037119525 | 214482 | 1 |
| CHONS2001797 | 0.037119525 | 195567 | 1 |
| CHONS2001834 | 0.037119525 | 100947 | 1 |
| CHONS2002419 | 0.037119525 | 36130 | 1 |
| CHONS2002829 | 0.102559495 | 102447 | 32 |
| COLON1000030 | 0.011763322 | 9531 | 1 |
| COLON1000084 | 0.011763322 | 196656 | 1 |
| COLON1000135 | 0.011763322 | 271740 | 1 |
| COLON2000470 | 0.023523877 | 172195 | 2 |
| COLON2000557 | 0.011763322 | 204534 | 1 |
| COLON2000568 | 0.011763322 | 274367 | 1 |
| COLON2001721 | 0.030286812 | 187376 | 13 |
| COLON2001829 | 0.011763322 | 104654 | 1 |
| COLON2001866 | 0.011763322 | 203953 | 1 |
| COLON2002443 | 0.037157444 | 90793 | 4 |
| COLON2002520 | 0.011763322 | 195730 | 1 |
| COLON2003043 | 0.011763322 | 247917 | 1 |
| COLON2003529 | 0.011763322 | 241903 | 1 |
| COLON2004318 | 0.011763322 | 176203 | 1 |
| COLON2004351 | 0.011763322 | 124325 | 1 |
| COLON2004478 | 0.011763322 | 274317 | 1 |
| COLON2004911 | 0.011763322 | 231909 | 1 |
| COLON2005126 | 0.023526644 | 176286 | 2 |
| COLON2005623 | 0.011763322 | 231849 | 1 |
| COLON2005735 | 0.011763322 | 188221 | 1 |
| COLON2005772 | 0.011763322 | 28843 | 1 |
| COLON2006263 | 0.011763322 | 226701 | 1 |
| COLON2006282 | 0.011763322 | 219407 | 1 |
| COLON2006417 | 0.011763322 | 44351 | 1 |
| COLON2007274 | 0.011763322 | 212155 | 1 |
| COLON2007733 | 0.013438869 | 216968 | 2 |
| COLON2008105 | 0.011763322 | 178748 | 1 |
| COLON2009337 | 0.023526644 | 176434 | 2 |
| COLON2009499 | 0.011763322 | 208506 | 1 |
| CORDB1000140 | 0.140646976 | 215444 | 1 |
| CORDB2000061 | 0.140646976 | 246593 | 1 |
| CORDB2000127 | 0.140646976 | 249465 | 1 |
| CORDB2000541 | 0.140646976 | 249151 | 1 |
| CTONG1000009 | 0.034496405 | 62385 | 13 |
| CTONG1000010 | 0.025844691 | 77899 | 7 |
| CTONG1000020 | 0.003120806 | 77966 | 1 |
| CTONG1000022 | 0.003120806 | 72470 | 1 |
| CTONG1000040 | 0.003120806 | 7962 | 1 |
| CTONG1000052 | 0.277307012 | 16876 | 89 |
| CTONG1000056 | 0.003120806 | 43501 | 1 |
| CTONG1000062 | 0.003120806 | 59249 | 1 |
| CTONG1000086 | 0.003120806 | 62390 | 1 |
| CTONG1000087 | 0.160228428 | 21020 | 44 |
| CTONG1000088 | 0.057351275 | 79448 | 3 |
| CTONG1000093 | 0.004216085 | 70893 | 2 |
| CTONG1000094 | 0.430230536 | 55221 | 85 |
| CTONG1000097 | 0.003120806 | 141332 | 1 |
| CTONG1000113 | 0.014884128 | 77814 | 2 |
| CTONG1000137 | 0.005119208 | 74841 | 2 |
| CTONG1000165 | 0.003120806 | 6634 | 1 |
| CTONG1000175 | 0.099518905 | 67270 | 21 |
| CTONG1000180 | 0.006977322 | 40124 | 3 |
| CTONG1000181 | 0.003120806 | 22626 | 1 |
| CTONG1000218 | 0.347974452 | 40191 | 42 |
| CTONG1000220 | 0.012575283 | 62125 | 2 |
| CTONG1000241 | 0.078984935 | 69883 | 31 |
| CTONG1000277 | 0.008101167 | 71091 | 3 |
| CTONG1000283 | 0.003120806 | 14595 | 1 |
| CTONG1000288 | 0.110164387 | 63549 | 34 |
| CTONG1000302 | 0.003120806 | 180451 | 1 |
| CTONG1000341 | 0.006035663 | 94998 | 2 |
| CTONG1000467 | 0.019500085 | 169148 | 7 |
| CTONG1000476 | 0.003120806 | 167184 | 1 |
| CTONG1000488 | 0.003120806 | 271982 | 1 |
| CTONG1000508 | 0.003120806 | 186524 | 1 |
| CTONG1000540 | 0.003120806 | 175577 | 1 |
| CTONG2000031 | 0.004796353 | 86537 | 2 |
| CTONG2000034 | 0.018590341 | 162325 | 3 |
| CTONG2000042 | 0.04782732 | 121139 | 16 |
| CTONG2000063 | 0.078268289 | 105397 | 28 |
| CTONG2000201 | 0.086441422 | 112247 | 21 |
| CTONG2000218 | 0.003120806 | 255618 | 1 |
| CTONG2000279 | 0.003120806 | 278930 | 1 |
| CTONG2000303 | 0.006241613 | 197275 | 2 |
| CTONG2000330 | 0.003120806 | 90705 | 1 |
| CTONG2000395 | 0.003120806 | 265176 | 1 |
| CTONG2000411 | 0.029115702 | 150428 | 11 |
| CTONG2000452 | 0.057530151 | 35838 | 10 |
| CTONG2000465 | 0.004517962 | 35886 | 2 |
| CTONG2000469 | 0.05737883 | 103841 | 12 |
| CTONG2000480 | 0.003120806 | 263293 | 1 |
| CTONG2000508 | 0.212174642 | 84844 | 73 |
| CTONG2000561 | 0.046175455 | 124179 | 15 |
| CTONG2000589 | 0.015026848 | 22042 | 4 |
| CTONG2000766 | 0.003120806 | 106143 | 1 |
| CTONG2000771 | 0.003120806 | 267049 | 1 |
| CTONG2000819 | 0.003120806 | 167101 | 1 |
| CTONG2000827 | 0.004978921 | 92558 | 2 |
| CTONG2000846 | 0.003120806 | 85683 | 1 |
| CTONG2000855 | 0.003120806 | 112142 | 1 |
| CTONG2000977 | 0.003120806 | 13589 | 1 |
| CTONG2001139 | 0.006216302 | 181502 | 2 |
| CTONG2001226 | 0.022074238 | 13354 | 4 |
| CTONG2001320 | 0.03178173 | 130660 | 5 |
| CTONG2001366 | 0.021616218 | 126438 | 6 |
| CTONG2001413 | 0.003120806 | 124385 | 1 |
| CTONG2001428 | 0.004997648 | 240236 | 2 |
| CTONG2001513 | 0.006102766 | 39666 | 2 |
| CTONG2001524 | 0.003120806 | 258587 | 1 |
| CTONG2001533 | 0.004830267 | 204421 | 2 |
| CTONG2001598 | 0.004310051 | 168941 | 2 |
| CTONG2001681 | 0.003120806 | 33244 | 1 |
| CTONG2001724 | 0.003120806 | 183725 | 1 |
| CTONG2001748 | 0.080158054 | 3135 | 26 |
| CTONG2001749 | 0.003120806 | 108180 | 1 |
| CTONG2001800 | 0.005925545 | 124246 | 3 |
| CTONG2001820 | 0.006165688 | 109341 | 2 |
| CTONG2001858 | 0.022703207 | 3778 | 5 |
| CTONG2001877 | 0.003120806 | 281661 | 1 |
| CTONG2001911 | 0.011088714 | 117968 | 4 |
| CTONG2001932 | 0.016218342 | 62274 | 6 |
| CTONG2001955 | 0.003120806 | 47686 | 1 |
| CTONG2002066 | 0.014884128 | 155537 | 2 |
| CTONG2002073 | 0.114392808 | 119881 | 20 |
| CTONG2002095 | 0.023924858 | 76669 | 9 |
| CTONG2002143 | 0.004216085 | 106850 | 2 |
| CTONG2002270 | 0.315884954 | 80131 | 174 |
| CTONG2002395 | 0.004517962 | 133635 | 2 |
| CTONG2002417 | 0.003120806 | 187183 | 1 |
| CTONG2002418 | 0.003120806 | 63629 | 1 |
| CTONG2002453 | 0.021583041 | 179222 | 4 |
| CTONG2002520 | 0.056918342 | 53994 | 7 |
| CTONG2002558 | 0.006035663 | 99482 | 2 |
| CTONG2002590 | 0.003120806 | 152778 | 1 |
| CTONG2002709 | 0.077331872 | 77951 | 6 |
| CTONG2002721 | 0.004517962 | 189962 | 2 |
| CTONG2002744 | 0.284516459 | 79890 | 72 |
| CTONG2002766 | 0.014181268 | 69565 | 9 |
| CTONG2002803 | 0.113796531 | 77877 | 37 |
| CTONG2002816 | 0.003120806 | 119481 | 1 |
| CTONG2002820 | 0.014897288 | 103972 | 6 |
| CTONG2002832 | 0.014946393 | 179335 | 7 |
| CTONG2002841 | 0.015818152 | 126198 | 9 |
| CTONG2002903 | 0.054395942 | 138939 | 13 |
| CTONG2002965 | 0.018724838 | 149389 | 6 |
| CTONG2002999 | 0.003120806 | 206341 | 1 |
| CTONG2003028 | 0.003120806 | 95144 | 1 |
| CTONG2003094 | 0.09145026 | 126067 | 29 |
| CTONG2003100 | 0.039104973 | 187360 | 2 |
| CTONG2003115 | 0.003120806 | 199174 | 1 |
| CTONG2003189 | 0.004216085 | 162326 | 2 |
| CTONG2003245 | 0.003120806 | 204581 | 1 |
| CTONG2003293 | 0.018126706 | 108714 | 6 |
| CTONG2003298 | 0.230892881 | 121073 | 48 |
| CTONG2003348 | 0.005119208 | 137769 | 2 |
| CTONG2003350 | 0.003120806 | 141148 | 1 |
| CTONG2003361 | 0.007130942 | 87892 | 3 |
| CTONG2003372 | 0.003120806 | 181168 | 1 |
| CTONG2003375 | 0.003120806 | 101229 | 1 |
| CTONG2003427 | 0.003120806 | 157347 | 1 |
| CTONG2003517 | 0.014946209 | 155548 | 4 |
| CTONG2003524 | 0.103986045 | 75516 | 28 |
| CTONG2003599 | 0.003120806 | 412 | 1 |
| CTONG2003680 | 0.016182457 | 83753 | 2 |
| CTONG2003699 | 0.004809083 | 110024 | 2 |
| CTONG2003764 | 0.012829544 | 152578 | 2 |
| CTONG2003782 | 0.003120806 | 115291 | 1 |
| CTONG2003889 | 0.003120806 | 160486 | 1 |
| CTONG2003937 | 0.008962269 | 179141 | 2 |
| CTONG2003945 | 0.015680031 | 41700 | 3 |
| CTONG2004000 | 0.003120806 | 87224 | 1 |
| CTONG2004062 | 0.068657741 | 140112 | 22 |
| CTONG2004115 | 0.014018789 | 138170 | 3 |
| CTONG2004126 | 0.003120806 | 61376 | 1 |
| CTONG2004264 | 0.003120806 | 112602 | 1 |
| CTONG2004305 | 0.004796353 | 183331 | 2 |
| CTONG2004397 | 0.006035663 | 126844 | 2 |
| CTONG2004423 | 0.01458127 | 38348 | 7 |
| CTONG2004454 | 0.003120806 | 115355 | 1 |
| CTONG2004487 | 0.177140902 | 118959 | 79 |
| CTONG2004550 | 0.008962269 | 122165 | 2 |
| CTONG2004669 | 0.003120806 | 144841 | 1 |
| CTONG2004716 | 0.003120806 | 18409 | 1 |
| CTONG2004912 | 0.003120806 | 253234 | 1 |
| CTONG2004941 | 0.003120806 | 106992 | 1 |
| CTONG2004948 | 0.003120806 | 93041 | 1 |
| CTONG2005028 | 0.024477841 | 67324 | 10 |
| CTONG2005049 | 0.006241613 | 155344 | 2 |
| CTONG2005110 | 0.003120806 | 50728 | 1 |
| CTONG2005145 | 0.006828668 | 96127 | 3 |
| CTONG2005265 | 0.008962269 | 167518 | 2 |
| CTONG2005278 | 0.058139526 | 54224 | 17 |
| CTONG2005290 | 0.007297741 | 30737 | 2 |
| CTONG2005374 | 0.003120806 | 167274 | 1 |
| CTONG2005399 | 0.003120806 | 76298 | 1 |
| CTONG2005468 | 0.011239261 | 141850 | 4 |
| CTONG2005553 | 0.23397385 | 86681 | 40 |
| CTONG2005567 | 0.003120806 | 222692 | 1 |
| CTONG2005585 | 0.059810149 | 32632 | 2 |
| CTONG2005615 | 0.035237193 | 99667 | 19 |
| CTONG2005635 | 0.003120806 | 103368 | 1 |
| CTONG2005775 | 0.003120806 | 213226 | 1 |
| CTONG2005795 | 0.004830267 | 125171 | 2 |
| CTONG2005890 | 0.003120806 | 251063 | 1 |
| CTONG2005904 | 0.003120806 | 246498 | 1 |
| CTONG2005913 | 0.037549133 | 138354 | 9 |
| CTONG2006004 | 0.158747049 | 60248 | 22 |
| CTONG2006129 | 0.003120806 | 237474 | 1 |
| CTONG2006223 | 0.003120806 | 257390 | 1 |
| CTONG2006235 | 0.10228708 | 119632 | 35 |
| CTONG2006273 | 0.003120806 | 142600 | 1 |
| CTONG2006310 | 0.003120806 | 155452 | 1 |
| CTONG2006377 | 0.014272056 | 131941 | 9 |
| CTONG2006393 | 0.004796353 | 82547 | 2 |
| CTONG2006412 | 0.026992367 | 145607 | 10 |
| CTONG2006449 | 0.019060663 | 57104 | 5 |
| CTONG2006515 | 0.003120806 | 185387 | 1 |
| CTONG2006524 | 0.003120806 | 160618 | 1 |
| CTONG2006562 | 0.119676012 | 115921 | 29 |
| CTONG2006568 | 0.003120806 | 1118 | 1 |
| CTONG2006611 | 0.023203382 | 114381 | 6 |
| CTONG2006620 | 0.003120806 | 141233 | 1 |
| CTONG2006666 | 0.006394803 | 148234 | 3 |
| CTONG2006691 | 0.003120806 | 94651 | 1 |
| CTONG2006709 | 0.064792528 | 66027 | 25 |
| CTONG2006798 | 0.017364674 | 116923 | 5 |
| CTONG2006836 | 0.003120806 | 112541 | 1 |
| CTONG2006932 | 0.152263897 | 113947 | 16 |
| CTONG2006942 | 0.012483226 | 116396 | 4 |
| CTONG2006964 | 0.003120806 | 199107 | 1 |
| CTONG2007007 | 0.179986723 | 3690 | 50 |
| CTONG2007009 | 0.003120806 | 152526 | 1 |
| CTONG2007072 | 0.077886267 | 17742 | 18 |
| CTONG2007078 | 0.131115217 | 119962 | 31 |
| CTONG2007091 | 0.003120806 | 92658 | 1 |
| CTONG2007104 | 0.026939526 | 114315 | 9 |
| CTONG2007168 | 0.027717208 | 30085 | 16 |
| CTONG2007175 | 0.003120806 | 35654 | 1 |
| CTONG2007259 | 0.003120806 | 241536 | 1 |
| CTONG2007272 | 0.003120806 | 96165 | 1 |
| CTONG2007293 | 0.004978921 | 148996 | 2 |
| CTONG2007297 | 0.003120806 | 241542 | 1 |
| CTONG2007399 | 0.010272745 | 150737 | 7 |
| CTONG2007400 | 0.014561073 | 152008 | 6 |
| CTONG2007417 | 0.007773232 | 142891 | 4 |
| CTONG2007441 | 0.008118454 | 137131 | 3 |
| CTONG2007474 | 0.015773997 | 75185 | 3 |
| CTONG2007500 | 0.222801919 | 93241 | 51 |
| CTONG2007586 | 0.027878218 | 146137 | 8 |
| CTONG2007613 | 0.019142568 | 106401 | 5 |
| CTONG2007623 | 0.003120806 | 174951 | 1 |
| CTONG2007681 | 0.00906687 | 72577 | 5 |
| CTONG2007776 | 0.039395015 | 121642 | 19 |
| CTONG2007779 | 0.004978921 | 160578 | 2 |
| CTONG2007834 | 0.177140902 | 118959 | 79 |
| CTONG2007898 | 0.003120806 | 187129 | 1 |
| CTONG2007959 | 0.00921057 | 117951 | 3 |
| CTONG2007970 | 0.003120806 | 67829 | 1 |
| CTONG2008014 | 0.011066625 | 155226 | 3 |
| CTONG2008184 | 0.003120806 | 143548 | 1 |
| CTONG2008233 | 0.243998186 | 121013 | 62 |
| CTONG2008262 | 0.003120806 | 6595 | 1 |
| CTONG2008269 | 0.024627562 | 274521 | 3 |
| CTONG2008321 | 0.011138093 | 189416 | 3 |
| CTONG2008343 | 0.005119208 | 87922 | 2 |
| CTONG2008398 | 0.219156642 | 71428 | 31 |
| CTONG2008402 | 0.003120806 | 134022 | 1 |
| CTONG2008405 | 0.007613821 | 164132 | 4 |
| CTONG2008466 | 0.021431248 | 125662 | 5 |
| CTONG2008506 | 0.178058455 | 124982 | 51 |
| CTONG2008518 | 0.043505258 | 80331 | 15 |
| CTONG2008521 | 0.011021259 | 67399 | 4 |
| CTONG2008562 | 0.003120806 | 122276 | 1 |
| CTONG2008577 | 0.003120806 | 132456 | 1 |
| CTONG2008595 | 0.003120806 | 62016 | 1 |
| CTONG2008621 | 0.004997648 | 122242 | 2 |
| CTONG2008689 | 0.008017912 | 171541 | 4 |
| CTONG2008720 | 0.006206238 | 104094 | 3 |
| CTONG2008721 | 0.004978921 | 174128 | 2 |
| CTONG2008773 | 0.003120806 | 190009 | 1 |
| CTONG2008792 | 0.02280339 | 59837 | 6 |
| CTONG2008989 | 0.003120806 | 189099 | 1 |
| CTONG2009033 | 0.006505814 | 140048 | 3 |
| CTONG2009108 | 0.007060433 | 109378 | 3 |
| CTONG2009132 | 0.003120806 | 82136 | 1 |
| CTONG2009156 | 0.006241613 | 155851 | 2 |
| CTONG2009181 | 0.006241613 | 63192 | 2 |
| CTONG2009257 | 0.003120806 | 39686 | 1 |
| CTONG2009258 | 0.003120806 | 113070 | 1 |
| CTONG2009268 | 0.003120806 | 121502 | 1 |
| CTONG2009270 | 0.003120806 | 99946 | 1 |
| CTONG2009395 | 0.003120806 | 102049 | 1 |
| CTONG2009423 | 0.004830267 | 167720 | 2 |
| CTONG2009440 | 0.003120806 | 266855 | 1 |
| CTONG2009527 | 0.003120806 | 104916 | 1 |
| CTONG2009529 | 0.02649962 | 45436 | 5 |
| CTONG2009531 | 0.003120806 | 100842 | 1 |
| CTONG2009534 | 0.021788234 | 92756 | 10 |
| CTONG2009570 | 0.003120806 | 60631 | 1 |
| CTONG2009572 | 0.006241613 | 20586 | 2 |
| CTONG2009604 | 0.003120806 | 82835 | 1 |
| CTONG2009643 | 0.003120806 | 66463 | 1 |
| CTONG2009675 | 0.003120806 | 112952 | 1 |
| CTONG2009689 | 0.003120806 | 237559 | 1 |
| CTONG2009725 | 0.003120806 | 231913 | 1 |
| CTONG2009766 | 0.003120806 | 217517 | 1 |
| CTONG2009768 | 0.003120806 | 265018 | 1 |
| CTONG2009844 | 0.009323126 | 198981 | 2 |
| CTONG2009871 | 0.003120806 | 255787 | 1 |
| CTONG2009887 | 0.003120806 | 235099 | 1 |
| CTONG2009891 | 0.003120806 | 113214 | 1 |
| CTONG2009923 | 0.005119208 | 143566 | 2 |
| CTONG2009938 | 0.008374477 | 107882 | 4 |
| CTONG2009955 | 0.003120806 | 6572 | 1 |
| CTONG2009958 | 0.003120806 | 83771 | 1 |
| CTONG2009963 | 0.008105674 | 95735 | 5 |
| CTONG2010018 | 0.003120806 | 160358 | 1 |
| CTONG2010024 | 0.006241613 | 112185 | 2 |
| CTONG2010116 | 0.003120806 | 175358 | 1 |
| CTONG2010133 | 0.003120806 | 47501 | 1 |
| CTONG2010148 | 0.003120806 | 147658 | 1 |
| CTONG2010276 | 0.003120806 | 144021 | 1 |
| CTONG2010330 | 0.003120806 | 178863 | 1 |
| CTONG2010348 | 0.094622909 | 106856 | 32 |
| CTONG2010408 | 0.019555713 | 10440 | 9 |
| CTONG2010508 | 0.040867652 | 142924 | 3 |
| CTONG2010566 | 0.01235668 | 89826 | 4 |
| CTONG2010623 | 0.003120806 | 200489 | 1 |
| CTONG2010649 | 0.107152979 | 147960 | 24 |
| CTONG2010652 | 0.010424747 | 60713 | 6 |
| CTONG2010803 | 0.014881362 | 67891 | 2 |
| CTONG2010821 | 0.234479482 | 47615 | 51 |
| CTONG2011188 | 0.007951073 | 124623 | 3 |
| CTONG2011429 | 0.003120806 | 72408 | 1 |
| CTONG2011474 | 0.003120806 | 217588 | 1 |
| CTONG2011676 | 0.003120806 | 274344 | 1 |
| CTONG2011716 | 0.006035663 | 35776 | 2 |
| CTONG2011765 | 0.003120806 | 273347 | 1 |
| CTONG2011779 | 0.003120806 | 120967 | 1 |
| CTONG2011801 | 0.056586757 | 105981 | 15 |
| CTONG2011815 | 0.003120806 | 74005 | 1 |
| CTONG2011822 | 0.003120806 | 145602 | 1 |
| CTONG2011825 | 0.019516612 | 14977 | 6 |
| CTONG2011920 | 0.003120806 | 79613 | 1 |
| CTONG2011985 | 0.003120806 | 150354 | 1 |
| CTONG2012001 | 0.023432238 | 139309 | 10 |
| CTONG2012029 | 0.008962269 | 196248 | 2 |
| CTONG2012050 | 0.023587306 | 121537 | 9 |
| CTONG2012077 | 0.003120806 | 21210 | 1 |
| CTONG2012101 | 0.010613531 | 176766 | 5 |
| CTONG2012123 | 0.003120806 | 153183 | 1 |
| CTONG2012158 | 0.018366985 | 83115 | 4 |
| CTONG2012230 | 0.003120806 | 164971 | 1 |
| CTONG2012401 | 0.039831185 | 150153 | 8 |
| CTONG2012422 | 0.004517962 | 80146 | 2 |
| CTONG2012425 | 0.006035663 | 176359 | 2 |
| CTONG2012447 | 0.006241613 | 100680 | 2 |
| CTONG2012452 | 0.470539281 | 86556 | 72 |
| CTONG2012453 | 0.022248538 | 114970 | 7 |
| CTONG2012473 | 0.003120806 | 140865 | 1 |
| CTONG2012476 | 0.003120806 | 93696 | 1 |
| CTONG2012533 | 0.003120806 | 266275 | 1 |
| CTONG2012554 | 0.003120806 | 176800 | 1 |
| CTONG2012564 | 0.042410704 | 123974 | 3 |
| CTONG2012607 | 0.096882531 | 121319 | 10 |
| CTONG2012661 | 0.007996629 | 150181 | 4 |
| CTONG2012724 | 0.006241613 | 167247 | 2 |
| CTONG2012738 | 0.003120806 | 153127 | 1 |
| CTONG2012745 | 0.030207963 | 53232 | 6 |
| CTONG2012758 | 0.003120806 | 143773 | 1 |
| CTONG2012843 | 0.018590341 | 113537 | 3 |
| CTONG2012847 | 0.097299276 | 64724 | 18 |
| CTONG2012879 | 0.098543947 | 60159 | 24 |
| CTONG2012901 | 0.003120806 | 53288 | 1 |
| CTONG2012996 | 0.057385388 | 69872 | 19 |
| CTONG2013128 | 0.005119208 | 69307 | 2 |
| CTONG2013149 | 0.012575283 | 144804 | 2 |
| CTONG2013156 | 0.008101167 | 135776 | 3 |
| CTONG2013178 | 0.017705559 | 115940 | 3 |
| CTONG2013222 | 0.044586909 | 123152 | 3 |
| CTONG2013284 | 0.012483226 | 18407 | 4 |
| CTONG2013339 | 0.003120806 | 126572 | 1 |
| CTONG2013348 | 0.03045737 | 21056 | 11 |
| CTONG2013352 | 0.006165688 | 141074 | 2 |
| CTONG2013381 | 0.053410589 | 132949 | 16 |
| CTONG2013393 | 0.003120806 | 213337 | 1 |
| CTONG2013511 | 0.003120806 | 164929 | 1 |
| CTONG2013630 | 0.003120806 | 144965 | 1 |
| CTONG2013803 | 0.003120806 | 186489 | 1 |
| CTONG2013907 | 0.077719633 | 126397 | 20 |
| CTONG2013934 | 0.024156494 | 136205 | 15 |
| CTONG2013985 | 0.02861187 | 105398 | 5 |
| CTONG2013986 | 0.003120806 | 127193 | 1 |
| CTONG2014032 | 0.003120806 | 60741 | 1 |
| CTONG2014058 | 0.003120806 | 136818 | 1 |
| CTONG2014119 | 0.020674126 | 31757 | 12 |
| CTONG2014165 | 0.034799554 | 150747 | 6 |
| CTONG2014191 | 0.003120806 | 160438 | 1 |
| CTONG2014206 | 0.003120806 | 14385 | 1 |
| CTONG2014234 | 0.003120806 | 52670 | 1 |
| CTONG2014264 | 0.003120806 | 92254 | 1 |
| CTONG2014280 | 0.013329953 | 74214 | 6 |
| CTONG2014369 | 0.040262756 | 149684 | 10 |
| CTONG2014389 | 0.06343209 | 135781 | 8 |
| CTONG2014491 | 0.004997648 | 81374 | 2 |
| CTONG2014630 | 0.003120806 | 254496 | 1 |
| CTONG2014635 | 0.003120806 | 230514 | 1 |
| CTONG2014646 | 0.003120806 | 258279 | 1 |
| CTONG2014697 | 0.10290126 | 128010 | 24 |
| CTONG2014705 | 0.008925985 | 244218 | 2 |
| CTONG2014898 | 0.029049444 | 146811 | 13 |
| CTONG2014946 | 0.003120806 | 163825 | 1 |
| CTONG2014954 | 0.01370457 | 142266 | 5 |
| CTONG2014959 | 0.003120806 | 188300 | 1 |
| CTONG2014966 | 0.003120806 | 145508 | 1 |
| CTONG2014995 | 0.003120806 | 206100 | 1 |
| CTONG2015091 | 0.206327196 | 56768 | 24 |
| CTONG2015204 | 0.011046569 | 165000 | 4 |
| CTONG2015316 | 0.003120806 | 133962 | 1 |
| CTONG2015330 | 0.005891632 | 22110 | 3 |
| CTONG2015345 | 0.019627211 | 31512 | 7 |
| CTONG2015358 | 0.004216085 | 132408 | 2 |
| CTONG2015434 | 0.003120806 | 134117 | 1 |
| CTONG2015518 | 0.012617427 | 84426 | 4 |
| CTONG2015540 | 0.003120806 | 153039 | 1 |
| CTONG2015596 | 0.003120806 | 145511 | 1 |
| CTONG2015633 | 0.003120806 | 184709 | 1 |
| CTONG2015678 | 0.004997648 | 176156 | 2 |
| CTONG2015717 | 0.003120806 | 230523 | 1 |
| CTONG2015804 | 0.003120806 | 131923 | 1 |
| CTONG2015815 | 0.019047249 | 236266 | 4 |
| CTONG2015866 | 0.003120806 | 110172 | 1 |
| CTONG2015914 | 0.006241613 | 171694 | 2 |
| CTONG2015953 | 0.003120806 | 173186 | 1 |
| CTONG2016056 | 0.084263661 | 50258 | 9 |
| CTONG2016185 | 0.064452745 | 112993 | 20 |
| CTONG2016217 | 0.003120806 | 123191 | 1 |
| CTONG2016355 | 0.003120806 | 159411 | 1 |
| CTONG2016408 | 0.04525627 | 48099 | 11 |
| CTONG2016499 | 0.004796353 | 149894 | 2 |
| CTONG2016505 | 0.009323126 | 183221 | 2 |
| CTONG2016559 | 0.003120806 | 181025 | 1 |
| CTONG2016575 | 0.337008866 | 34785 | 103 |
| CTONG2016658 | 0.003120806 | 132148 | 1 |
| CTONG2016775 | 0.003120806 | 176517 | 1 |
| CTONG2016797 | 0.003120806 | 206052 | 1 |
| CTONG2016824 | 0.039364238 | 79287 | 7 |
| CTONG2016846 | 0.003120806 | 145385 | 1 |
| CTONG2016869 | 0.063438942 | 17960 | 33 |
| CTONG2016904 | 0.003120806 | 116043 | 1 |
| CTONG2016931 | 0.05490659 | 88544 | 16 |
| CTONG2016942 | 0.014584752 | 155457 | 2 |
| CTONG2016953 | 0.003120806 | 243892 | 1 |
| CTONG2017021 | 0.003120806 | 188857 | 1 |
| CTONG2017094 | 0.074946426 | 130588 | 33 |
| CTONG2017292 | 0.003120806 | 175116 | 1 |
| CTONG2017409 | 0.004978921 | 112030 | 2 |
| CTONG2017429 | 0.023217322 | 123615 | 9 |
| CTONG2017444 | 0.045500795 | 132817 | 8 |
| CTONG2017458 | 0.003120806 | 162142 | 1 |
| CTONG2017500 | 0.06325171 | 153121 | 16 |
| CTONG2017604 | 0.013948004 | 185642 | 2 |
| CTONG2017650 | 0.003120806 | 164997 | 1 |
| CTONG2017798 | 0.008407551 | 47881 | 5 |
| CTONG2017804 | 0.009362419 | 137779 | 3 |
| CTONG2017814 | 0.003120806 | 133713 | 1 |
| CTONG2017910 | 0.003120806 | 144540 | 1 |
| CTONG2017939 | 0.003120806 | 171580 | 1 |
| CTONG2017989 | 0.003120806 | 8532 | 1 |
| CTONG2017998 | 0.066885703 | 65289 | 22 |
| CTONG2018062 | 0.003120806 | 204619 | 1 |
| CTONG2018069 | 0.003120806 | 230565 | 1 |
| CTONG2018135 | 0.010772981 | 117859 | 3 |
| CTONG2018147 | 0.003120806 | 199576 | 1 |
| CTONG2018211 | 0.049128914 | 143252 | 23 |
| CTONG2018217 | 0.003120806 | 178742 | 1 |
| CTONG2018279 | 0.003120806 | 198774 | 1 |
| CTONG2018334 | 0.022906496 | 175097 | 4 |
| CTONG2018343 | 0.011000075 | 27839 | 4 |
| CTONG2018379 | 0.003120806 | 149546 | 1 |
| CTONG2018383 | 0.037066396 | 140531 | 8 |
| CTONG2018413 | 0.006654468 | 445 | 3 |
| CTONG2018483 | 0.003120806 | 145199 | 1 |
| CTONG2018614 | 0.107152979 | 147960 | 24 |
| CTONG2018629 | 0.008118454 | 61273 | 3 |
| CTONG2018632 | 0.020949569 | 146791 | 6 |
| CTONG2018637 | 0.0255403 | 114676 | 13 |
| CTONG2018652 | 0.004216085 | 134374 | 2 |
| CTONG2018653 | 0.003120806 | 154748 | 1 |
| CTONG2018655 | 0.003120806 | 160945 | 1 |
| CTONG2018735 | 0.004517962 | 158753 | 2 |
| CTONG2018739 | 0.043236157 | 82742 | 25 |
| CTONG2018808 | 0.021273983 | 18560 | 5 |
| CTONG2018843 | 0.015092343 | 162030 | 6 |
| CTONG2018898 | 0.347911398 | 85618 | 143 |
| CTONG2018900 | 0.039156842 | 185001 | 2 |
| CTONG2018976 | 0.004517962 | 59482 | 2 |
| CTONG2019029 | 0.02070585 | 86408 | 8 |
| CTONG2019063 | 0.00789185 | 177974 | 3 |
| CTONG2019144 | 0.003120806 | 230543 | 1 |
| CTONG2019226 | 0.126812117 | 121445 | 44 |
| CTONG2019232 | 0.003120806 | 177074 | 1 |
| CTONG2019248 | 0.011075103 | 109409 | 4 |
| CTONG2019400 | 0.008488153 | 99779 | 5 |
| CTONG2019579 | 0.246421275 | 60996 | 32 |
| CTONG2019590 | 0.015571145 | 177140 | 5 |
| CTONG2019652 | 0.003120806 | 275526 | 1 |
| CTONG2019704 | 0.004997648 | 151418 | 2 |
| CTONG2019788 | 0.054798922 | 112077 | 8 |
| CTONG2019822 | 0.051535226 | 140011 | 7 |
| CTONG2019833 | 0.003120806 | 249441 | 1 |
| CTONG2020031 | 0.003120806 | 239538 | 1 |
| CTONG2020108 | 0.004517962 | 108592 | 2 |
| CTONG2020127 | 0.003120806 | 235025 | 1 |
| CTONG2020171 | 0.003120806 | 273578 | 1 |
| CTONG2020374 | 0.010781951 | 130107 | 5 |
| CTONG2020378 | 0.010381057 | 159348 | 5 |
| CTONG2020411 | 0.024337954 | 142074 | 9 |
| CTONG2020445 | 0.00796088 | 193716 | 3 |
| CTONG2020484 | 0.285038793 | 155470 | 28 |
| CTONG2020522 | 0.007311581 | 94151 | 3 |
| CTONG2020560 | 0.111693548 | 94701 | 39 |
| CTONG2020582 | 0.087326661 | 84741 | 38 |
| CTONG2020638 | 0.013533263 | 190625 | 5 |
| CTONG2020806 | 0.003120806 | 176801 | 1 |
| CTONG2020831 | 0.004978921 | 12747 | 2 |
| CTONG2020974 | 0.004216085 | 188736 | 2 |
| CTONG2021024 | 0.004997648 | 16554 | 2 |
| CTONG2021132 | 0.003120806 | 173441 | 1 |
| CTONG2021168 | 0.003120806 | 275834 | 1 |
| CTONG2021289 | 0.016284682 | 32489 | 9 |
| CTONG2022153 | 0.003120806 | 107309 | 1 |
| CTONG2022601 | 0.003120806 | 155433 | 1 |
| CTONG2022835 | 0.097666493 | 113041 | 7 |
| CTONG2023021 | 0.004978921 | 212634 | 2 |
| CTONG2023449 | 0.014584752 | 216175 | 2 |
| CTONG2023512 | 0.003120806 | 63452 | 1 |
| CTONG2024031 | 0.064831259 | 130106 | 13 |
| CTONG2024206 | 0.003120806 | 152314 | 1 |
| CTONG2024614 | 0.003120806 | 114796 | 1 |
| CTONG2024749 | 0.003120806 | 121142 | 1 |
| CTONG2025496 | 0.003120806 | 121138 | 1 |
| CTONG2025513 | 0.003120806 | 105636 | 1 |
| CTONG2025516 | 0.003120806 | 50392 | 1 |
| CTONG2025610 | 0.003120806 | 190580 | 1 |
| CTONG2025823 | 0.104812185 | 103899 | 29 |
| CTONG2025900 | 0.006241613 | 141228 | 2 |
| CTONG2026513 | 0.003120806 | 279437 | 1 |
| CTONG2026770 | 0.003120806 | 171895 | 1 |
| CTONG2026920 | 0.003120806 | 126518 | 1 |
| CTONG2026987 | 0.003120806 | 193881 | 1 |
| CTONG2027150 | 0.003120806 | 135723 | 1 |
| CTONG2027263 | 0.010174556 | 115873 | 6 |
| CTONG2027327 | 0.005915117 | 130565 | 3 |
| CTONG2027361 | 0.003120806 | 167504 | 1 |
| CTONG2027591 | 0.003120806 | 176258 | 1 |
| CTONG2027736 | 0.003120806 | 161070 | 1 |
| CTONG2027783 | 0.003120806 | 207067 | 1 |
| CTONG2027828 | 0.003120806 | 201528 | 1 |
| CTONG2027959 | 0.004310051 | 223767 | 2 |
| CTONG2028097 | 0.003120806 | 84216 | 1 |
| CTONG2028124 | 0.333389741 | 74106 | 72 |
| CTONG2028208 | 0.047316758 | 103024 | 7 |
| CTONG2028309 | 0.022827717 | 91737 | 13 |
| CTONG2028486 | 0.004830267 | 126502 | 2 |
| CTONG2028518 | 0.003120806 | 79299 | 1 |
| CTONG2028687 | 0.003120806 | 65547 | 1 |
| CTONG2028758 | 0.010418404 | 232620 | 3 |
| CTONG3000084 | 0.018149557 | 123226 | 5 |
| CTONG3000657 | 0.043186159 | 54434 | 13 |
| CTONG3000686 | 0.003120806 | 255501 | 1 |
| CTONG3000707 | 0.003120806 | 223771 | 1 |
| CTONG3000896 | 0.003120806 | 258070 | 1 |
| CTONG3001123 | 0.021352338 | 6973 | 8 |
| CTONG3001370 | 0.04782732 | 121139 | 16 |
| CTONG3001420 | 0.006102766 | 125067 | 2 |
| CTONG3001501 | 0.003120806 | 182219 | 1 |
| CTONG3001560 | 0.003120806 | 219139 | 1 |
| CTONG3001605 | 0.003120806 | 102594 | 1 |
| CTONG3001746 | 0.011712566 | 125383 | 6 |
| CTONG3002020 | 0.003120806 | 261829 | 1 |
| CTONG3002127 | 0.004997648 | 138614 | 2 |
| CTONG3002370 | 0.003120806 | 155186 | 1 |
| CTONG3002412 | 0.015657464 | 132482 | 3 |
| CTONG3002433 | 0.003120806 | 160967 | 1 |
| CTONG3002518 | 0.019857151 | 115778 | 5 |
| CTONG3002552 | 0.003120806 | 217505 | 1 |
| CTONG3002588 | 0.003120806 | 257921 | 1 |
| CTONG3002674 | 0.007499921 | 145272 | 3 |
| CTONG3002728 | 0.003120806 | 162301 | 1 |
| CTONG3002835 | 0.152511253 | 70898 | 51 |
| CTONG3002909 | 0.003120806 | 231263 | 1 |
| CTONG3002947 | 0.003120806 | 265335 | 1 |
| CTONG3002983 | 0.003120806 | 213712 | 1 |
| CTONG3003165 | 0.003120806 | 206650 | 1 |
| CTONG3003179 | 0.003120806 | 155199 | 1 |
| CTONG3003483 | 0.003120806 | 190414 | 1 |
| CTONG3003553 | 0.003120806 | 174419 | 1 |
| CTONG3003598 | 0.003120806 | 158758 | 1 |
| CTONG3003652 | 0.003120806 | 79140 | 1 |
| CTONG3003654 | 0.003120806 | 186697 | 1 |
| CTONG3003669 | 0.003120806 | 83233 | 1 |
| CTONG3003737 | 0.006241613 | 169505 | 2 |
| CTONG3003905 | 0.008556245 | 144878 | 3 |
| CTONG3003972 | 0.006035663 | 114203 | 2 |
| CTONG3004072 | 0.00858395 | 169053 | 4 |
| CTONG3004317 | 0.005119208 | 164909 | 2 |
| CTONG3004397 | 0.003120806 | 186737 | 1 |
| CTONG3004550 | 0.003120806 | 193373 | 1 |
| CTONG3004576 | 0.004997648 | 264550 | 2 |
| CTONG3004607 | 0.003120806 | 193312 | 1 |
| CTONG3004712 | 0.003120806 | 189679 | 1 |
| CTONG3004726 | 0.009893738 | 186753 | 4 |
| CTONG3005325 | 0.003120806 | 223681 | 1 |
| CTONG3005648 | 0.003120806 | 144963 | 1 |
| CTONG3005713 | 0.003120806 | 166765 | 1 |
| CTONG3005803 | 0.003120806 | 262194 | 1 |
| CTONG3005813 | 0.004310051 | 148009 | 2 |
| CTONG3006067 | 0.004216085 | 250341 | 2 |
| CTONG3006073 | 0.003120806 | 275889 | 1 |
| CTONG3006186 | 0.003120806 | 193154 | 1 |
| CTONG3006629 | 0.005202318 | 210397 | 2 |
| CTONG3006650 | 0.003120806 | 263678 | 1 |
| CTONG3006659 | 0.003120806 | 213612 | 1 |
| CTONG3007156 | 0.030068076 | 139630 | 11 |
| CTONG3007244 | 0.003120806 | 134002 | 1 |
| CTONG3007444 | 0.003120806 | 206812 | 1 |
| CTONG3007528 | 0.003120806 | 189773 | 1 |
| CTONG3007586 | 0.003120806 | 218484 | 1 |
| CTONG3007870 | 0.003120806 | 155788 | 1 |
| CTONG3008223 | 0.003120806 | 166729 | 1 |
| CTONG3008252 | 0.003120806 | 138752 | 1 |
| CTONG3008258 | 0.003120806 | 130160 | 1 |
| CTONG3008457 | 0.003120806 | 193532 | 1 |
| CTONG3008496 | 0.003120806 | 210268 | 1 |
| CTONG3008566 | 0.003120806 | 159106 | 1 |
| CTONG3008639 | 0.003120806 | 142559 | 1 |
| CTONG3008831 | 0.003120806 | 176161 | 1 |
| CTONG3008894 | 0.23050447 | 54222 | 65 |
| CTONG3008951 | 0.003120806 | 189767 | 1 |
| CTONG3008959 | 0.003120806 | 280325 | 1 |
| CTONG3009028 | 0.104117944 | 101576 | 17 |
| CTONG3009227 | 0.003120806 | 212143 | 1 |
| CTONG3009239 | 0.116049925 | 122198 | 20 |
| CTONG3009287 | 0.003120806 | 266067 | 1 |
| CTONG3009328 | 0.013971528 | 166252 | 4 |
| CTONG3009385 | 0.003120806 | 283717 | 1 |
| D3OST1000066 | 0.019561815 | 106133 | 1 |
| D3OST1000109 | 0.093873669 | 65300 | 5 |
| D3OST1000238 | 0.019561815 | 70264 | 1 |
| D3OST1000267 | 0.038801408 | 61539 | 6 |
| D3OST1000270 | 0.06484856 | 165871 | 10 |
| D3OST2000184 | 0.019561815 | 115674 | 1 |
| D3OST2000618 | 0.019561815 | 233011 | 1 |
| D3OST2000654 | 0.019561815 | 240422 | 1 |
| D3OST2000734 | 0.020958971 | 165082 | 2 |
| D3OST2001328 | 0.019561815 | 54994 | 1 |
| D3OST2001598 | 0.133620252 | 162823 | 25 |
| D3OST2001669 | 0.019561815 | 160748 | 1 |
| D3OST2001670 | 0.044906326 | 32003 | 16 |
| D3OST2001788 | 0.057455321 | 207448 | 3 |
| D3OST2002068 | 0.779927307 | 13188 | 36 |
| D3OST2002182 | 0.022543774 | 181875 | 2 |
| D3OST2002223 | 0.019561815 | 196249 | 1 |
| D3OST2002262 | 0.019561815 | 10981 | 1 |
| D3OST2002272 | 0.019561815 | 184492 | 1 |
| D3OST2002417 | 0.019561815 | 181802 | 1 |
| D3OST2002436 | 0.019561815 | 59507 | 1 |
| D3OST2002648 | 0.111685261 | 205579 | 3 |
| D3OST2003024 | 0.020657094 | 172212 | 2 |
| D3OST2003331 | 0.019561815 | 102138 | 1 |
| D3OST2003607 | 0.042155579 | 172299 | 2 |
| D3OST2003797 | 0.019561815 | 209937 | 1 |
| D3OST2003856 | 0.028522523 | 139468 | 4 |
| D3OST2004637 | 0.019561815 | 83434 | 1 |
| D3OST3000169 | 0.342393646 | 107384 | 16 |
| D3OST3000195 | 0.019561815 | 101848 | 1 |
| D3OST3000258 | 0.026954175 | 166297 | 5 |
| D3OST3000291 | 0.020958971 | 225614 | 2 |
| D3OST3000388 | 0.039123631 | 129867 | 2 |
| D3OST3000493 | 0.019561815 | 13211 | 1 |
| D60ST2000127 | 0.112485939 | 22207 | 1 |
| D60ST2000169 | 0.112485939 | 165157 | 1 |
| D60ST2000245 | 0.112485939 | 34544 | 1 |
| D60ST2000358 | 0.117909172 | 14321 | 4 |
| D60ST2000445 | 0.13116108 | 21045 | 7 |
| D60ST2000464 | 0.152933569 | 68417 | 10 |
| D60ST2000507 | 0.112485939 | 234407 | 1 |
| D9OST2000031 | 0.03546628 | 127947 | 4 |
| D9OST2000278 | 0.022593764 | 240111 | 1 |
| D9OST2000440 | 0.022593764 | 260948 | 1 |
| D9OST2000869 | 0.022593764 | 116154 | 1 |
| D9OST2001319 | 0.022593764 | 13197 | 1 |
| D9OST2001433 | 0.022593764 | 94735 | 1 |
| D9OST2001547 | 0.320836355 | 113852 | 56 |
| D9OST2002397 | 0.059713289 | 27384 | 2 |
| D9OST2002608 | 0.075207697 | 5633 | 24 |
| D9OST2002673 | 0.11471721 | 140952 | 3 |
| D9OST2003106 | 0.024269311 | 163466 | 2 |
| D9OST2003108 | 0.034354319 | 116037 | 2 |
| D9OST2003137 | 0.022593764 | 149663 | 1 |
| D9OST2003397 | 0.0586298 | 110749 | 2 |
| D9OST2003580 | 0.024282041 | 205287 | 2 |
| D9OST2003594 | 0.022593764 | 119273 | 1 |
| D9OST2003762 | 0.076873546 | 88189 | 4 |
| D9OST2003791 | 0.027386476 | 190681 | 4 |
| D9OST2003989 | 0.022593764 | 186515 | 1 |
| D9OST2004018 | 0.022593764 | 196472 | 1 |
| D9OST2004073 | 0.022593764 | 174322 | 1 |
| D9OST2004417 | 0.022593764 | 194901 | 1 |
| DFNES1000003 | 0.009874593 | 70308 | 1 |
| DFNES1000059 | 0.36328603 | 48128 | 52 |
| DFNES1000083 | 0.074487441 | 46150 | 11 |
| DFNES1000107 | 0.009874593 | 91562 | 1 |
| DFNES1000150 | 0.150038079 | 72887 | 26 |
| DFNES1000185 | 0.127043346 | 54022 | 4 |
| DFNES2000011 | 0.306413222 | 134223 | 35 |
| DFNES2000143 | 0.009874593 | 155119 | 1 |
| DFNES2000146 | 0.015051601 | 37368 | 3 |
| DFNES2000153 | 0.052333198 | 124873 | 7 |
| DFNES2000212 | 0.011562869 | 255753 | 2 |
| DFNES2000268 | 0.151739919 | 101980 | 12 |
| DFNES2000292 | 0.044645918 | 170876 | 6 |
| DFNES2000335 | 0.035367106 | 159474 | 10 |
| DFNES2000426 | 0.013270149 | 121257 | 3 |
| DFNES2000432 | 0.022469051 | 189201 | 2 |
| DFNES2000443 | 0.246835777 | 102486 | 13 |
| DFNES2000457 | 0.018864075 | 200670 | 4 |
| DFNES2000471 | 0.009874593 | 240352 | 1 |
| DFNES2000526 | 0.060630942 | 105554 | 14 |
| DFNES2000913 | 0.009874593 | 122514 | 1 |
| DFNES2001091 | 0.019523855 | 146980 | 3 |
| DFNES2001096 | 0.021864736 | 244363 | 3 |
| DFNES2001108 | 0.009874593 | 143613 | 1 |
| DFNES2001177 | 0.009874593 | 115344 | 1 |
| DFNES2001404 | 0.016076912 | 206200 | 2 |
| DFNES2001564 | 0.009874593 | 225231 | 1 |
| DFNES2001565 | 0.009874593 | 226983 | 1 |
| DFNES2001647 | 0.009874593 | 235448 | 1 |
| DFNES2001800 | 0.009874593 | 128303 | 1 |
| DFNES2001829 | 0.018156706 | 142272 | 6 |
| DFNES2002220 | 0.009874593 | 273079 | 1 |
| DFNES2002509 | 0.033000279 | 166587 | 3 |
| DFNES2002550 | 0.009874593 | 49135 | 1 |
| DFNES2002588 | 0.009874593 | 134598 | 1 |
| DFNES2002817 | 0.009874593 | 55052 | 1 |
| DFNES2002966 | 0.010969871 | 153176 | 2 |
| DFNES2003081 | 0.021277101 | 109172 | 2 |
| DFNES2003192 | 0.05593799 | 169863 | 5 |
| DFNES2003255 | 0.009874593 | 194431 | 1 |
| DFNES2003742 | 0.016076912 | 92268 | 2 |
| DFNES2004346 | 0.053538604 | 189798 | 4 |
| DFNES2004354 | 0.009874593 | 21250 | 1 |
| DFNES2004371 | 0.106203986 | 143173 | 24 |
| DFNES2004383 | 0.009874593 | 244456 | 1 |
| DFNES2004608 | 0.022223321 | 167623 | 2 |
| DFNES2004676 | 0.523525708 | 65148 | 65 |
| DFNES2004684 | 0.009874593 | 226924 | 1 |
| DFNES2005266 | 0.009874593 | 162205 | 1 |
| DFNES2005527 | 0.009874593 | 174374 | 1 |
| DFNES2005540 | 0.055428176 | 51301 | 6 |
| DFNES2005690 | 0.033003076 | 102213 | 13 |
| DFNES2005766 | 0.091393352 | 69311 | 7 |
| DFNES2005779 | 0.009874593 | 189676 | 1 |
| DFNES2005875 | 0.009874593 | 86363 | 1 |
| DFNES2006346 | 0.023718542 | 162010 | 7 |
| DFNES2006944 | 0.009874593 | 172325 | 1 |
| DFNES2007113 | 0.025073495 | 83012 | 4 |
| DFNES2007299 | 0.051018213 | 155151 | 3 |
| DFNES2007332 | 0.045147519 | 177409 | 5 |
| DFNES2007634 | 0.188510464 | 49606 | 58 |
| DFNES2007641 | 0.015679771 | 180029 | 2 |
| DFNES2007757 | 0.044500631 | 187106 | 2 |
| DFNES2008088 | 0.063735892 | 58501 | 10 |
| DFNES2008160 | 0.037401317 | 85928 | 16 |
| DFNES2008280 | 0.009874593 | 181779 | 1 |
| DFNES2008881 | 0.009874593 | 176061 | 1 |
| DFNES2009482 | 0.118139575 | 122834 | 10 |
| DFNES2010502 | 0.009874593 | 142265 | 1 |
| DFNES2011192 | 0.009874593 | 93746 | 1 |
| DFNES2011221 | 0.011751434 | 207725 | 2 |
| DFNES2011239 | 0.022424565 | 73183 | 3 |
| DFNES2011245 | 0.011584053 | 10966 | 2 |
| DFNES2011380 | 0.014333695 | 84318 | 4 |
| DFNES2011499 | 0.027007696 | 120899 | 10 |
| ERLTF1000021 | 0.045913682 | 244656 | 1 |
| ERLTF2000324 | 0.045913682 | 250347 | 1 |
| ERLTF2000465 | 0.045913682 | 142255 | 1 |
| FCBBF1000023 | 0.023819499 | 60177 | 4 |
| FCBBF1000024 | 0.076316914 | 49417 | 29 |
| FCBBF1000025 | 0.052359894 | 36915 | 11 |
| FCBBF1000027 | 0.45742453 | 62524 | 90 |
| FCBBF1000038 | 0.014914122 | 68397 | 6 |
| FCBBF1000053 | 0.061912034 | 35621 | 13 |
| FCBBF1000061 | 0.071243067 | 22766 | 26 |
| FCBBF1000063 | 0.028015417 | 14993 | 12 |
| FCBBF1000069 | 0.003095496 | 38028 | 1 |
| FCBBF1000077 | 0.206886551 | 62771 | 45 |
| FCBBF1000115 | 0.008243094 | 44497 | 4 |
| FCBBF1000117 | 0.003095496 | 102986 | 1 |
| FCBBF1000118 | 0.011049492 | 48994 | 5 |
| FCBBF1000121 | 0.016865074 | 9752 | 4 |
| FCBBF1000125 | 0.01075679 | 67675 | 5 |
| FCBBF1000131 | 0.003095496 | 52379 | 1 |
| FCBBF1000139 | 0.095848531 | 39474 | 37 |
| FCBBF1000155 | 0.003095496 | 21603 | 1 |
| FCBBF1000157 | 0.003095496 | 62763 | 1 |
| FCBBF1000171 | 0.003095496 | 60036 | 1 |
| FCBBF1000174 | 0.003095496 | 29641 | 1 |
| FCBBF1000182 | 0.019758782 | 11390 | 5 |
| FCBBF1000197 | 0.15685083 | 2587 | 55 |
| FCBBF1000198 | 0.003095496 | 33978 | 1 |
| FCBBF1000206 | 0.003095496 | 63982 | 1 |
| FCBBF1000220 | 0.003095496 | 22880 | 1 |
| FCBBF1000232 | 0.003095496 | 16449 | 1 |
| FCBBF1000233 | 0.003095496 | 68393 | 1 |
| FCBBF1000243 | 0.003095496 | 29523 | 1 |
| FCBBF1000270 | 0.003095496 | 48993 | 1 |
| FCBBF1000272 | 0.004804956 | 56068 | 2 |
| FCBBF1000280 | 0.029664149 | 41382 | 4 |
| FCBBF1000294 | 0.155624815 | 48302 | 44 |
| FCBBF1000297 | 0.003095496 | 74266 | 1 |
| FCBBF1000305 | 0.009340032 | 65758 | 4 |
| FCBBF1000314 | 0.003095496 | 1 | 1 |
| FCBBF1000322 | 0.003095496 | 32180 | 1 |
| FCBBF1000349 | 0.003095496 | 20926 | 1 |
| FCBBF1000367 | 0.110295219 | 4791 | 14 |
| FCBBF1000374 | 0.004190774 | 572 | 2 |
| FCBBF1000376 | 0.007866539 | 66556 | 3 |
| FCBBF1000377 | 0.015494321 | 46845 | 5 |
| FCBBF1000395 | 0.003095496 | 28170 | 1 |
| FCBBF1000397 | 0.003095496 | 2853 | 1 |
| FCBBF1000403 | 0.019977624 | 55364 | 5 |
| FCBBF1000412 | 0.086580986 | 58258 | 18 |
| FCBBF1000425 | 0.032095449 | 73402 | 11 |
| FCBBF1000437 | 0.013102145 | 55983 | 4 |
| FCBBF1000449 | 0.025062187 | 47334 | 7 |
| FCBBF1000466 | 0.263102133 | 55886 | 74 |
| FCBBF1000476 | 0.019293346 | 82143 | 9 |
| FCBBF1000506 | 0.009007758 | 68182 | 2 |
| FCBBF1000509 | 0.089799033 | 35365 | 28 |
| FCBBF1000526 | 0.080158054 | 3135 | 26 |
| FCBBF1000546 | 0.003095496 | 78873 | 1 |
| FCBBF1000550 | 0.003095496 | 76601 | 1 |
| FCBBF1000556 | 0.157181137 | 846 | 63 |
| FCBBF1000563 | 0.111559045 | 77935 | 35 |
| FCBBF1000574 | 0.080717809 | 69258 | 39 |
| FCBBF1000581 | 0.003095496 | 28339 | 1 |
| FCBBF1000582 | 0.003095496 | 83783 | 1 |
| FCBBF1000598 | 0.003095496 | 162089 | 1 |
| FCBBF1000604 | 0.003095496 | 100093 | 1 |
| FCBBF1000618 | 0.003095496 | 63094 | 1 |
| FCBBF1000627 | 0.003095496 | 74410 | 1 |
| FCBBF1000671 | 0.012238737 | 51017 | 6 |
| FCBBF1000675 | 0.013194834 | 69613 | 5 |
| FCBBF1000684 | 0.009331854 | 51019 | 2 |
| FCBBF1000686 | 0.007272431 | 70185 | 2 |
| FCBBF1000687 | 0.019396227 | 132528 | 8 |
| FCBBF1000691 | 0.003095496 | 69392 | 1 |
| FCBBF1000732 | 0.059837989 | 86412 | 9 |
| FCBBF1000748 | 0.00495361 | 11819 | 2 |
| FCBBF1000760 | 0.255865764 | 32709 | 29 |
| FCBBF2000038 | 0.124736983 | 28224 | 39 |
| FCBBF2000087 | 0.058081999 | 76337 | 7 |
| FCBBF2000094 | 0.003095496 | 117702 | 1 |
| FCBBF2000105 | 0.003095496 | 263895 | 1 |
| FCBBF2000123 | 0.003095496 | 265963 | 1 |
| FCBBF2000195 | 0.040311722 | 163377 | 2 |
| FCBBF2000199 | 0.059891866 | 79334 | 27 |
| FCBBF2000214 | 0.003095496 | 183947 | 1 |
| FCBBF2000232 | 0.003095496 | 54920 | 1 |
| FCBBF2000259 | 0.028019685 | 97114 | 9 |
| FCBBF2000276 | 0.003095496 | 129052 | 1 |
| FCBBF2000291 | 0.003095496 | 229153 | 1 |
| FCBBF2000362 | 0.020017388 | 101639 | 9 |
| FCBBF2000379 | 0.003095496 | 275965 | 1 |
| FCBBF2000473 | 0.003095496 | 132162 | 1 |
| FCBBF2000502 | 0.025398692 | 135290 | 3 |
| FCBBF2000566 | 0.045079387 | 136406 | 14 |
| FCBBF2000576 | 0.014882389 | 242007 | 2 |
| FCBBF2000591 | 0.584454109 | 94457 | 13 |
| FCBBF2000677 | 0.003095496 | 261367 | 1 |
| FCBBF2000678 | 0.003095496 | 283276 | 1 |
| FCBBF2000685 | 0.024628816 | 100828 | 5 |
| FCBBF2000733 | 0.021141239 | 108905 | 9 |
| FCBBF2000782 | 0.004492651 | 82708 | 2 |
| FCBBF2000808 | 0.003095496 | 263938 | 1 |
| FCBBF2000815 | 0.003095496 | 284071 | 1 |
| FCBBF2000825 | 0.022657311 | 238945 | 2 |
| FCBBF2000885 | 0.10915845 | 111627 | 42 |
| FCBBF2000940 | 0.00495361 | 201747 | 2 |
| FCBBF2000951 | 0.003095496 | 99499 | 1 |
| FCBBF2001001 | 0.017531439 | 111026 | 6 |
| FCBBF2001088 | 0.010334114 | 22044 | 3 |
| FCBBF2001105 | 0.055198885 | 22492 | 12 |
| FCBBF2001116 | 0.003095496 | 263718 | 1 |
| FCBBF2001183 | 0.10132157 | 79371 | 45 |
| FCBBF2001188 | 0.003095496 | 225291 | 1 |
| FCBBF2001211 | 0.026145652 | 134566 | 10 |
| FCBBF2001238 | 0.003095496 | 96304 | 1 |
| FCBBF2001243 | 0.003095496 | 225292 | 1 |
| FCBBF2001291 | 0.088074524 | 137034 | 18 |
| FCBBF2001299 | 0.007380237 | 189283 | 3 |
| FCBBF2001309 | 0.038511283 | 94283 | 13 |
| FCBBF2001400 | 0.008227607 | 82606 | 4 |
| FCBBF2001427 | 0.003095496 | 113787 | 1 |
| FCBBF2001480 | 0.00693958 | 183077 | 2 |
| FCBBF2001529 | 0.003095496 | 230424 | 1 |
| FCBBF2001538 | 0.21990932 | 118326 | 82 |
| FCBBF2001594 | 0.044059453 | 94350 | 25 |
| FCBBF2001672 | 0.003095496 | 44484 | 1 |
| FCBBF2001675 | 0.003095496 | 268010 | 1 |
| FCBBF2001718 | 0.003095496 | 259602 | 1 |
| FCBBF2001720 | 0.003095496 | 179421 | 1 |
| FCBBF2001817 | 0.00693958 | 18763 | 2 |
| FCBBF2001868 | 0.006190992 | 118121 | 2 |
| FCBBF2002044 | 0.003095496 | 183700 | 1 |
| FCBBF2002111 | 0.014559442 | 143975 | 2 |
| FCBBF2002281 | 0.010367927 | 110123 | 3 |
| FCBBF2002349 | 0.042442686 | 103204 | 13 |
| FCBBF2002370 | 0.003095496 | 109478 | 1 |
| FCBBF2002541 | 0.003095496 | 197892 | 1 |
| FCBBF2002626 | 0.004284741 | 3985 | 2 |
| FCBBF2002801 | 0.064558893 | 111089 | 19 |
| FCBBF2002897 | 0.003095496 | 141060 | 1 |
| FCBBF2002898 | 0.003095496 | 160955 | 1 |
| FCBBF2002904 | 0.003095496 | 22851 | 1 |
| FCBBF2002928 | 0.053850774 | 199720 | 4 |
| FCBBF2002953 | 0.003095496 | 43724 | 1 |
| FCBBF2003083 | 0.003095496 | 151747 | 1 |
| FCBBF2003102 | 0.008367709 | 80220 | 3 |
| FCBBF2003269 | 0.003095496 | 207147 | 1 |
| FCBBF2003293 | 0.009137062 | 167120 | 2 |
| FCBBF2003297 | 0.003095496 | 207159 | 1 |
| FCBBF2003336 | 0.262421535 | 94790 | 41 |
| FCBBF2003395 | 0.003095496 | 211024 | 1 |
| FCBBF2003528 | 0.003095496 | 52583 | 1 |
| FCBBF2003543 | 0.004771043 | 150811 | 2 |
| FCBBF2003549 | 0.014600328 | 45661 | 2 |
| FCBBF2003567 | 0.025703904 | 63614 | 10 |
| FCBBF2003636 | 0.003095496 | 190114 | 1 |
| FCBBF2003823 | 0.003095496 | 172921 | 1 |
| FCBBF2003895 | 0.003095496 | 199750 | 1 |
| FCBBF2004066 | 0.083115518 | 88587 | 28 |
| FCBBF2004090 | 0.003095496 | 49898 | 1 |
| FCBBF2004138 | 0.003095496 | 96860 | 1 |
| FCBBF2004217 | 0.003095496 | 2580 | 1 |
| FCBBF2004256 | 0.003095496 | 183624 | 1 |
| FCBBF2004373 | 0.007133779 | 24091 | 2 |
| FCBBF2004448 | 0.003095496 | 104546 | 1 |
| FCBBF2004671 | 0.003095496 | 230442 | 1 |
| FCBBF2004707 | 0.003095496 | 203865 | 1 |
| FCBBF2004788 | 0.003095496 | 139777 | 1 |
| FCBBF2004930 | 0.105565309 | 110213 | 33 |
| FCBBF2005069 | 0.003095496 | 132134 | 1 |
| FCBBF2005122 | 0.003095496 | 112227 | 1 |
| FCBBF2005245 | 0.23923172 | 104456 | 77 |
| FCBBF2005428 | 0.004284741 | 204446 | 2 |
| FCBBF2005439 | 0.006190992 | 96429 | 2 |
| FCBBF2005538 | 0.003095496 | 163134 | 1 |
| FCBBF2005637 | 0.06029586 | 155045 | 3 |
| FCBBF2005645 | 0.003095496 | 190701 | 1 |
| FCBBF2005658 | 0.021528592 | 109338 | 4 |
| FCBBF2005733 | 0.005177008 | 98294 | 2 |
| FCBBF2005909 | 0.026846921 | 18694 | 13 |
| FCBBF2005966 | 0.017691894 | 112189 | 5 |
| FCBBF2006131 | 0.003095496 | 106381 | 1 |
| FCBBF2006380 | 0.005093897 | 151005 | 2 |
| FCBBF2006418 | 0.003095496 | 121988 | 1 |
| FCBBF2006452 | 0.003095496 | 148978 | 1 |
| FCBBF2006634 | 0.035497262 | 143610 | 5 |
| FCBBF2006781 | 0.026479008 | 163248 | 6 |
| FCBBF2006882 | 0.048078306 | 18948 | 4 |
| FCBBF2007012 | 0.003095496 | 181784 | 1 |
| FCBBF2007080 | 0.039628131 | 86047 | 18 |
| FCBBF2007095 | 0.084263661 | 50258 | 9 |
| FCBBF2007186 | 0.003095496 | 175519 | 1 |
| FCBBF2007265 | 0.003095496 | 90437 | 1 |
| FCBBF2007302 | 0.007133779 | 155784 | 2 |
| FCBBF2007556 | 0.018334719 | 127067 | 4 |
| FCBBF2007610 | 0.006190992 | 98276 | 2 |
| FCBBF2007633 | 0.003095496 | 162796 | 1 |
| FCBBF2007840 | 0.013452619 | 42083 | 4 |
| FCBBF3000001 | 0.016389065 | 111079 | 6 |
| FCBBF3000102 | 0.013503056 | 105120 | 6 |
| FCBBF3000110 | 0.025944427 | 140529 | 8 |
| FCBBF3000115 | 0.041984388 | 80345 | 22 |
| FCBBF3000120 | 0.003095496 | 220253 | 1 |
| FCBBF3000168 | 0.020579852 | 95687 | 9 |
| FCBBF3000175 | 0.039903954 | 33268 | 7 |
| FCBBF3000184 | 0.011185197 | 183619 | 4 |
| FCBBF3000227 | 0.014901871 | 65144 | 2 |
| FCBBF3000228 | 0.018696377 | 107760 | 8 |
| FCBBF3000229 | 0.017352873 | 97121 | 4 |
| FCBBF3000233 | 0.003095496 | 234177 | 1 |
| FCBBF3000269 | 0.005473985 | 213191 | 3 |
| FCBBF3000361 | 0.003095496 | 128820 | 1 |
| FCBBF3000367 | 0.003095496 | 231682 | 1 |
| FCBBF3000388 | 0.067835682 | 50807 | 21 |
| FCBBF3000434 | 0.090504275 | 93919 | 35 |
| FCBBF3000462 | 0.003095496 | 196256 | 1 |
| FCBBF3000473 | 0.003095496 | 61548 | 1 |
| FCBBF3000501 | 0.003095496 | 264017 | 1 |
| FCBBF3000518 | 0.003095496 | 203242 | 1 |
| FCBBF3000536 | 0.062857826 | 119699 | 8 |
| FCBBF3000550 | 0.003095496 | 108008 | 1 |
| FCBBF3000768 | 0.027878218 | 146137 | 8 |
| FCBBF3000847 | 0.003095496 | 160555 | 1 |
| FCBBF3001018 | 0.003095496 | 144149 | 1 |
| FCBBF3001020 | 0.003095496 | 198381 | 1 |
| FCBBF3001081 | 0.063813822 | 18682 | 17 |
| FCBBF3001235 | 0.003095496 | 34590 | 1 |
| FCBBF3001281 | 0.008229057 | 155647 | 3 |
| FCBBF3001302 | 0.050406334 | 73062 | 3 |
| FCBBF3001377 | 0.016272096 | 186082 | 7 |
| FCBBF3001470 | 0.12343259 | 2956 | 15 |
| FCBBF3001594 | 0.003095496 | 277262 | 1 |
| FCBBF3001632 | 0.108539818 | 157195 | 21 |
| FCBBF3001657 | 0.071027019 | 89830 | 30 |
| FCBBF3001818 | 0.018394886 | 155304 | 8 |
| FCBBF3001855 | 0.059831107 | 52248 | 11 |
| FCBBF3001890 | 0.060915562 | 156540 | 14 |
| FCBBF3001912 | 0.003095496 | 33343 | 1 |
| FCBBF3001914 | 0.003095496 | 131677 | 1 |
| FCBBF3001918 | 0.003095496 | 12687 | 1 |
| FCBBF3001924 | 0.003095496 | 143587 | 1 |
| FCBBF3001977 | 0.0224955 | 161197 | 11 |
| FCBBF3002163 | 0.003095496 | 106152 | 1 |
| FCBBF3002188 | 0.003095496 | 197370 | 1 |
| FCBBF3002190 | 0.003095496 | 18929 | 1 |
| FCBBF3002268 | 0.003095496 | 103104 | 1 |
| FCBBF3002334 | 0.209966321 | 34085 | 59 |
| FCBBF3002475 | 0.003095496 | 71492 | 1 |
| FCBBF3002556 | 0.005177008 | 102832 | 2 |
| FCBBF3002592 | 0.003095496 | 166891 | 1 |
| FCBBF3002658 | 0.003095496 | 118928 | 1 |
| FCBBF3002782 | 0.003095496 | 142376 | 1 |
| FCBBF3002818 | 0.004771043 | 107319 | 2 |
| FCBBF3002925 | 0.003095496 | 105298 | 1 |
| FCBBF3003216 | 0.003095496 | 50836 | 1 |
| FCBBF3003305 | 0.003095496 | 96192 | 1 |
| FCBBF3003373 | 0.003095496 | 179753 | 1 |
| FCBBF3003435 | 0.018714474 | 207259 | 3 |
| FCBBF3003475 | 0.003095496 | 166878 | 1 |
| FCBBF3003481 | 0.003095496 | 84909 | 1 |
| FCBBF3003557 | 0.004771043 | 195270 | 2 |
| FCBBF3003787 | 0.017119771 | 128336 | 3 |
| FCBBF3003800 | 0.425362467 | 115654 | 125 |
| FCBBF3003902 | 0.007133779 | 179937 | 2 |
| FCBBF3004021 | 0.015689954 | 67962 | 2 |
| FCBBF3004041 | 0.044901516 | 165850 | 2 |
| FCBBF3004261 | 0.018522597 | 13249 | 7 |
| FCBBF3004323 | 0.030411669 | 80988 | 11 |
| FCBBF3004390 | 0.003095496 | 3900 | 1 |
| FCBBF3004409 | 0.005177008 | 71434 | 2 |
| FCBBF3004473 | 0.145841376 | 31452 | 30 |
| FCBBF3004502 | 0.003095496 | 81209 | 1 |
| FCBBF3004715 | 0.044746061 | 36441 | 5 |
| FCBBF3004842 | 0.003095496 | 144487 | 1 |
| FCBBF3004847 | 0.005093897 | 216184 | 2 |
| FCBBF3004955 | 0.021740844 | 80574 | 9 |
| FCBBF3005160 | 0.003095496 | 151590 | 1 |
| FCBBF3005218 | 0.003095496 | 138158 | 1 |
| FCBBF3005320 | 0.061422066 | 31211 | 16 |
| FCBBF3005330 | 0.241870702 | 56356 | 77 |
| FCBBF3005444 | 0.006190992 | 109183 | 2 |
| FCBBF3005497 | 0.213136262 | 78006 | 56 |
| FCBBF3005698 | 0.003095496 | 183684 | 1 |
| FCBBF3005729 | 0.003095496 | 110961 | 1 |
| FCBBF3006171 | 0.003095496 | 262531 | 1 |
| FCBBF3006249 | 0.003095496 | 138355 | 1 |
| FCBBF3006288 | 0.003095496 | 135436 | 1 |
| FCBBF3006399 | 0.003095496 | 162729 | 1 |
| FCBBF3006513 | 0.030648354 | 61532 | 12 |
| FCBBF3006628 | 0.003095496 | 9180 | 1 |
| FCBBF3006821 | 0.029311461 | 118609 | 18 |
| FCBBF3007077 | 0.003095496 | 5710 | 1 |
| FCBBF3007150 | 0.006190992 | 189818 | 2 |
| FCBBF3007152 | 0.003095496 | 180493 | 1 |
| FCBBF3007242 | 0.003095496 | 231705 | 1 |
| FCBBF3007244 | 0.021585909 | 157755 | 6 |
| FCBBF3007248 | 0.003095496 | 62467 | 1 |
| FCBBF3007453 | 0.003095496 | 194201 | 1 |
| FCBBF3007462 | 0.004804956 | 118823 | 2 |
| FCBBF3007540 | 0.003095496 | 230414 | 1 |
| FCBBF3007597 | 0.006709388 | 196496 | 2 |
| FCBBF3007604 | 0.003095496 | 220987 | 1 |
| FCBBF3007631 | 0.007669748 | 28265 | 4 |
| FCBBF3007829 | 0.014893514 | 105631 | 2 |
| FCBBF3007855 | 0.003095496 | 108031 | 1 |
| FCBBF3007859 | 0.025792706 | 69538 | 14 |
| FCBBF3007860 | 0.016559245 | 105628 | 9 |
| FCBBF3007977 | 0.044113724 | 132884 | 19 |
| FCBBF3008073 | 0.003095496 | 28057 | 1 |
| FCBBF3008100 | 0.011231376 | 120283 | 3 |
| FCBBF3008153 | 0.030625467 | 8804 | 15 |
| FCBBF3008159 | 0.008090791 | 8205 | 5 |
| FCBBF3008251 | 0.003095496 | 10075 | 1 |
| FCBBF3008311 | 0.006190992 | 155600 | 2 |
| FCBBF3008362 | 0.003095496 | 111566 | 1 |
| FCBBF3008382 | 0.015556263 | 207555 | 4 |
| FCBBF3008475 | 0.003095496 | 146780 | 1 |
| FCBBF3008556 | 0.004190774 | 155227 | 2 |
| FCBBF3008644 | 0.019447014 | 139467 | 4 |
| FCBBF3008689 | 0.017951547 | 143439 | 3 |
| FCBBF3008748 | 0.003095496 | 65274 | 1 |
| FCBBF3008870 | 0.003095496 | 215271 | 1 |
| FCBBF3008938 | 0.006010353 | 118747 | 2 |
| FCBBF3008944 | 0.003095496 | 285350 | 1 |
| FCBBF3009069 | 0.037415329 | 90386 | 21 |
| FCBBF3009101 | 0.004771043 | 120883 | 2 |
| FCBBF3009152 | 0.008809326 | 154077 | 3 |
| FCBBF3009256 | 0.004492651 | 254894 | 2 |
| FCBBF3009317 | 0.006190992 | 45274 | 2 |
| FCBBF3009428 | 0.003095496 | 80029 | 1 |
| FCBBF3009464 | 0.003095496 | 108257 | 1 |
| FCBBF3009479 | 0.016938605 | 147290 | 4 |
| FCBBF3009526 | 0.014182646 | 146947 | 6 |
| FCBBF3009541 | 0.406452685 | 87045 | 44 |
| FCBBF3009703 | 0.005177008 | 96427 | 2 |
| FCBBF3009717 | 0.003095496 | 250383 | 1 |
| FCBBF3009888 | 0.003095496 | 58275 | 1 |
| FCBBF3009949 | 0.003095496 | 102466 | 1 |
| FCBBF3009978 | 0.009031271 | 140827 | 2 |
| FCBBF3010008 | 0.003095496 | 133334 | 1 |
| FCBBF3010012 | 0.072918291 | 109313 | 27 |
| FCBBF3010041 | 0.092918664 | 130311 | 35 |
| FCBBF3010124 | 0.003095496 | 181016 | 1 |
| FCBBF3010130 | 0.003095496 | 180997 | 1 |
| FCBBF3010142 | 0.011609374 | 117093 | 6 |
| FCBBF3010149 | 0.003095496 | 155978 | 1 |
| FCBBF3010211 | 0.003095496 | 196702 | 1 |
| FCBBF3010361 | 0.003095496 | 101835 | 1 |
| FCBBF3010508 | 0.014856051 | 91710 | 2 |
| FCBBF3010544 | 0.003095496 | 91163 | 1 |
| FCBBF3010586 | 0.003095496 | 84520 | 1 |
| FCBBF3010601 | 0.003095496 | 200384 | 1 |
| FCBBF3010665 | 0.006369493 | 59284 | 3 |
| FCBBF3010695 | 0.067566706 | 15347 | 10 |
| FCBBF3010729 | 0.003095496 | 74635 | 1 |
| FCBBF3010733 | 0.003095496 | 174591 | 1 |
| FCBBF3011003 | 0.003095496 | 268668 | 1 |
| FCBBF3011183 | 0.013295058 | 99956 | 5 |
| FCBBF3011418 | 0.003095496 | 200677 | 1 |
| FCBBF3011485 | 0.008353798 | 19003 | 4 |
| FCBBF3011523 | 0.009297816 | 82316 | 2 |
| FCBBF3011570 | 0.003095496 | 126373 | 1 |
| FCBBF3011592 | 0.006190992 | 49168 | 2 |
| FCBBF3011867 | 0.003095496 | 85770 | 1 |
| FCBBF3011889 | 0.003095496 | 174970 | 1 |
| FCBBF3012170 | 0.003095496 | 158268 | 1 |
| FCBBF3012182 | 0.636006888 | 179857 | 2 |
| FCBBF3012288 | 0.004190774 | 226685 | 2 |
| FCBBF3012332 | 0.007866539 | 137006 | 3 |
| FCBBF3012347 | 0.076340879 | 104645 | 11 |
| FCBBF3012443 | 0.006140378 | 157133 | 2 |
| FCBBF3012546 | 0.067381772 | 51526 | 23 |
| FCBBF3012588 | 0.003095496 | 110940 | 1 |
| FCBBF3012667 | 0.006865285 | 75229 | 3 |
| FCBBF3012842 | 0.003095496 | 158323 | 1 |
| FCBBF3012901 | 0.003095496 | 97274 | 1 |
| FCBBF3013041 | 0.003095496 | 104712 | 1 |
| FCBBF3013058 | 0.003095496 | 237839 | 1 |
| FCBBF3013205 | 0.003095496 | 113182 | 1 |
| FCBBF3013266 | 0.00792573 | 6824 | 4 |
| FCBBF3013307 | 0.003095496 | 119687 | 1 |
| FCBBF3013341 | 0.003095496 | 275987 | 1 |
| FCBBF3013506 | 0.007055566 | 70754 | 4 |
| FCBBF3013589 | 0.02019543 | 57995 | 3 |
| FCBBF3013623 | 0.003095496 | 16629 | 1 |
| FCBBF3013800 | 0.004804956 | 69448 | 2 |
| FCBBF3013846 | 0.003095496 | 175736 | 1 |
| FCBBF3014229 | 0.003095496 | 190802 | 1 |
| FCBBF3014260 | 0.074209592 | 112722 | 23 |
| FCBBF3014355 | 0.003095496 | 1229 | 1 |
| FCBBF3014567 | 0.003095496 | 139758 | 1 |
| FCBBF3014770 | 0.017558968 | 120640 | 4 |
| FCBBF3015100 | 0.033580771 | 117707 | 16 |
| FCBBF3015119 | 0.003095496 | 217028 | 1 |
| FCBBF3015131 | 0.003095496 | 126166 | 1 |
| FCBBF3015317 | 0.003095496 | 222451 | 1 |
| FCBBF3015396 | 0.079742189 | 90872 | 13 |
| FCBBF3015727 | 0.004190774 | 175082 | 2 |
| FCBBF3015758 | 0.003095496 | 113156 | 1 |
| FCBBF3015809 | 0.003095496 | 124899 | 1 |
| FCBBF3015870 | 0.003095496 | 192464 | 1 |
| FCBBF3016018 | 0.003095496 | 237852 | 1 |
| FCBBF3016134 | 0.016096048 | 104871 | 4 |
| FCBBF3016178 | 0.003095496 | 239329 | 1 |
| FCBBF3016451 | 0.007133779 | 144397 | 2 |
| FCBBF3016618 | 0.003095496 | 120202 | 1 |
| FCBBF3016622 | 0.004771043 | 30459 | 2 |
| FCBBF3016644 | 0.003095496 | 192744 | 1 |
| FCBBF3016667 | 0.013088788 | 98301 | 4 |
| FCBBF3016928 | 0.191224014 | 78908 | 32 |
| FCBBF3016987 | 0.003095496 | 188489 | 1 |
| FCBBF3017024 | 0.032691387 | 91639 | 9 |
| FCBBF3017059 | 0.003095496 | 163553 | 1 |
| FCBBF3017071 | 0.003095496 | 1166 | 1 |
| FCBBF3017123 | 0.003095496 | 27872 | 1 |
| FCBBF3017233 | 0.003095496 | 100605 | 1 |
| FCBBF3017255 | 0.004804956 | 254544 | 2 |
| FCBBF3017288 | 0.005177008 | 105123 | 2 |
| FCBBF3017396 | 0.082074724 | 123824 | 20 |
| FCBBF3017531 | 0.104503607 | 80975 | 13 |
| FCBBF3017535 | 0.003095496 | 245940 | 1 |
| FCBBF3017681 | 0.003095496 | 278172 | 1 |
| FCBBF3017729 | 0.003095496 | 224411 | 1 |
| FCBBF3017873 | 0.003095496 | 142688 | 1 |
| FCBBF3017918 | 0.02559117 | 50260 | 11 |
| FCBBF3017974 | 0.003095496 | 180463 | 1 |
| FCBBF3018067 | 0.009137062 | 199895 | 2 |
| FCBBF3018173 | 0.004804956 | 102819 | 2 |
| FCBBF3018453 | 0.036272915 | 11172 | 12 |
| FCBBF3018591 | 0.003095496 | 87725 | 1 |
| FCBBF3018753 | 0.007272431 | 78046 | 2 |
| FCBBF3018796 | 0.05397134 | 134299 | 13 |
| FCBBF3018826 | 0.017175926 | 103781 | 5 |
| FCBBF3018949 | 0.011840079 | 142681 | 5 |
| FCBBF3019085 | 0.003095496 | 116314 | 1 |
| FCBBF3019320 | 0.008476207 | 53626 | 5 |
| FCBBF3019437 | 0.005177008 | 163552 | 2 |
| FCBBF3019455 | 0.020573785 | 162262 | 6 |
| FCBBF3019564 | 0.003095496 | 107210 | 1 |
| FCBBF3019569 | 0.004284741 | 180656 | 2 |
| FCBBF3019570 | 0.003095496 | 202160 | 1 |
| FCBBF3019663 | 0.003095496 | 189005 | 1 |
| FCBBF3019714 | 0.081803803 | 3716 | 8 |
| FCBBF3019716 | 0.003095496 | 154090 | 1 |
| FCBBF3019784 | 0.006140378 | 21322 | 2 |
| FCBBF3019839 | 0.044741637 | 132857 | 21 |
| FCBBF3019867 | 0.003095496 | 17746 | 1 |
| FCBBF3019961 | 0.003095496 | 249984 | 1 |
| FCBBF3020030 | 0.003095496 | 285116 | 1 |
| FCBBF3020138 | 0.003095496 | 126595 | 1 |
| FCBBF3020140 | 0.005889807 | 108327 | 3 |
| FCBBF3020163 | 0.003095496 | 201387 | 1 |
| FCBBF3020232 | 0.003095496 | 148608 | 1 |
| FCBBF3020599 | 0.006010353 | 247156 | 2 |
| FCBBF3021026 | 0.00693958 | 165540 | 2 |
| FCBBF3021191 | 0.00903762 | 57594 | 2 |
| FCBBF3021221 | 0.003095496 | 123739 | 1 |
| FCBBF3021501 | 0.003095496 | 83948 | 1 |
| FCBBF3021506 | 0.003095496 | 121039 | 1 |
| FCBBF3021524 | 0.003095496 | 157510 | 1 |
| FCBBF3021576 | 0.019495442 | 57915 | 5 |
| FCBBF3021807 | 0.038644985 | 161093 | 7 |
| FCBBF3021940 | 0.006709388 | 190587 | 2 |
| FCBBF3021985 | 0.003095496 | 187397 | 1 |
| FCBBF3022005 | 0.003095496 | 67389 | 1 |
| FCBBF3022291 | 0.004284741 | 132527 | 2 |
| FCBBF3022311 | 0.007380237 | 147872 | 3 |
| FCBBF3022321 | 0.022327869 | 111563 | 8 |
| FCBBF3022504 | 0.010751098 | 134416 | 4 |
| FCBBF3022566 | 0.004771043 | 162304 | 2 |
| FCBBF3022593 | 0.02418371 | 35373 | 7 |
| FCBBF3022765 | 0.004783773 | 283481 | 2 |
| FCBBF3022767 | 0.008227607 | 152022 | 4 |
| FCBBF3022894 | 0.037987331 | 121092 | 2 |
| FCBBF3023061 | 0.003095496 | 279182 | 1 |
| FCBBF3023078 | 0.039131532 | 193305 | 2 |
| FCBBF3023368 | 0.068291868 | 72083 | 14 |
| FCBBF3023372 | 0.003095496 | 266581 | 1 |
| FCBBF3023443 | 0.003095496 | 243362 | 1 |
| FCBBF3023599 | 0.003095496 | 185654 | 1 |
| FCBBF3023667 | 1.26175789 | 6796 | 104 |
| FCBBF3023704 | 0.003095496 | 173913 | 1 |
| FCBBF3023887 | 0.017679991 | 35952 | 7 |
| FCBBF3023895 | 0.003095496 | 255290 | 1 |
| FCBBF3024002 | 0.029523914 | 28589 | 16 |
| FCBBF3024096 | 0.003095496 | 93587 | 1 |
| FCBBF3024225 | 0.003095496 | 149394 | 1 |
| FCBBF3024266 | 0.017129488 | 3024 | 7 |
| FCBBF3024364 | 0.003095496 | 98834 | 1 |
| FCBBF3024623 | 0.059813931 | 45257 | 18 |
| FCBBF3024663 | 0.022806825 | 83294 | 11 |
| FCBBF3024793 | 0.01048706 | 40320 | 3 |
| FCBBF3024911 | 0.003095496 | 246119 | 1 |
| FCBBF3024935 | 0.105889362 | 102938 | 37 |
| FCBBF3025073 | 0.023163784 | 148824 | 8 |
| FCBBF3025098 | 0.003095496 | 1019 | 1 |
| FCBBF3025142 | 0.034580253 | 128442 | 15 |
| FCBBF3025252 | 0.003095496 | 50012 | 1 |
| FCBBF3025280 | 0.003095496 | 452 | 1 |
| FCBBF3025285 | 0.00693958 | 69856 | 2 |
| FCBBF3025417 | 0.019520132 | 119643 | 7 |
| FCBBF3025528 | 0.034705132 | 17097 | 7 |
| FCBBF3025568 | 0.010188365 | 82534 | 5 |
| FCBBF3025650 | 0.006663071 | 161974 | 3 |
| FCBBF3025674 | 0.003095496 | 122763 | 1 |
| FCBBF3025730 | 0.008462843 | 179377 | 5 |
| FCBBF3025737 | 0.051555398 | 106162 | 15 |
| FCBBF3025905 | 0.010474608 | 128816 | 6 |
| FCBBF3026021 | 0.003095496 | 147857 | 1 |
| FCBBF3026048 | 0.005177008 | 148775 | 2 |
| FCBBF3026236 | 0.01851457 | 94284 | 9 |
| FCBBF3026251 | 0.003095496 | 160066 | 1 |
| FCBBF3026308 | 0.013109707 | 18907 | 5 |
| FCBBF3026651 | 0.022961796 | 109189 | 14 |
| FCBBF3026678 | 0.003095496 | 223298 | 1 |
| FCBBF3026692 | 0.010326307 | 27253 | 3 |
| FCBBF3027104 | 0.003095496 | 83160 | 1 |
| FCBBF3027199 | 0.003095496 | 151981 | 1 |
| FCBBF3027328 | 0.003095496 | 141045 | 1 |
| FCBBF3027559 | 0.004771043 | 75587 | 2 |
| FCBBF3027717 | 0.003095496 | 165913 | 1 |
| FCBBF3027755 | 0.003095496 | 1220 | 1 |
| FCBBF3027768 | 0.003095496 | 91135 | 1 |
| FCBBF3027854 | 0.02140721 | 223399 | 4 |
| FCBBF3027863 | 0.003095496 | 177020 | 1 |
| FCBBF3027903 | 0.006190992 | 159232 | 2 |
| FCBBF3028102 | 0.003095496 | 265666 | 1 |
| FCBBF3028143 | 0.003095496 | 141680 | 1 |
| FCBBF3028188 | 0.003095496 | 158195 | 1 |
| FCBBF3028202 | 0.014583868 | 162303 | 5 |
| FCBBF3028244 | 0.180469591 | 11446 | 57 |
| FCBBF3028472 | 0.003095496 | 210384 | 1 |
| FCBBF3028528 | 0.003095496 | 121994 | 1 |
| FCBBF3028582 | 0.003095496 | 19310 | 1 |
| FCBBF3028593 | 0.041498893 | 78176 | 16 |
| FCBBF3028671 | 0.003095496 | 128279 | 1 |
| FCBBF3028794 | 0.003095496 | 161858 | 1 |
| FCBBF4000017 | 0.018864645 | 207011 | 5 |
| FCBBF4000061 | 0.003095496 | 85356 | 1 |
| FCBBF4000076 | 0.003095496 | 151467 | 1 |
| FCBBF4000142 | 0.045830797 | 66559 | 22 |
| FCBBF4000173 | 0.003095496 | 201938 | 1 |
| FCBBF4000192 | 0.003095496 | 260340 | 1 |
| FCBBF4000193 | 0.005177008 | 102821 | 2 |
| FCBBF4000268 | 0.004804956 | 148806 | 2 |
| FCBBF4000282 | 0.003095496 | 216578 | 1 |
| FCBBF4000399 | 0.003095496 | 226291 | 1 |
| FCBBF4000415 | 0.003095496 | 96302 | 1 |
| FCBBF4000446 | 0.223074046 | 129912 | 60 |
| FCBBF4000500 | 0.019117956 | 178642 | 4 |
| FCBBF4000529 | 0.003095496 | 264684 | 1 |
| FCBBF4000548 | 0.011463751 | 168269 | 5 |
| FCBBF5000041 | 0.003095496 | 84551 | 1 |
| FCBBF5000061 | 0.007998492 | 106949 | 3 |
| FCBBF5000165 | 0.050902363 | 103810 | 26 |
| FCBBF5000261 | 0.032271118 | 116453 | 10 |
| FCBBF5000325 | 0.016573675 | 157539 | 5 |
| FCBBF5000353 | 0.004783773 | 110781 | 2 |
| FCBBF5000384 | 0.009286488 | 250110 | 3 |
| FCBBF5000483 | 0.003095496 | 250092 | 1 |
| FCBBF5000495 | 0.003095496 | 190473 | 1 |
| FEBRA1000005 | 0.004176935 | 283841 | 1 |
| FEBRA1000008 | 0.007221817 | 51948 | 2 |
| FEBRA1000022 | 0.031835934 | 51520 | 10 |
| FEBRA1000030 | 0.010249318 | 41539 | 2 |
| FEBRA1000057 | 0.096516255 | 80034 | 46 |
| FEBRA1000088 | 0.039262423 | 76752 | 8 |
| FEBRA1000138 | 0.004176935 | 11952 | 1 |
| FEBRA1000148 | 0.004176935 | 69685 | 1 |
| FEBRA1000149 | 0.004176935 | 41492 | 1 |
| FEBRA1000183 | 0.004176935 | 10974 | 1 |
| FEBRA1000188 | 0.080062953 | 39146 | 30 |
| FEBRA1000189 | 0.008264884 | 73547 | 4 |
| FEBRA1000190 | 0.02108267 | 81248 | 3 |
| FEBRA2000007 | 0.00707564 | 1252 | 3 |
| FEBRA2000021 | 0.004176935 | 272023 | 1 |
| FEBRA2000035 | 0.004176935 | 165814 | 1 |
| FEBRA2000053 | 0.02375981 | 88848 | 10 |
| FEBRA2000105 | 0.233617833 | 65409 | 45 |
| FEBRA2000126 | 0.004176935 | 260702 | 1 |
| FEBRA2000129 | 0.064454281 | 29212 | 15 |
| FEBRA2000210 | 0.237030397 | 137341 | 17 |
| FEBRA2000220 | 0.004176935 | 69451 | 1 |
| FEBRA2000228 | 0.042419201 | 96185 | 12 |
| FEBRA2000253 | 0.011446575 | 112721 | 5 |
| FEBRA2000282 | 0.005272213 | 59705 | 2 |
| FEBRA2000286 | 0.004176935 | 151674 | 1 |
| FEBRA2000297 | 0.021128524 | 140830 | 6 |
| FEBRA2000311 | 0.016736159 | 79540 | 3 |
| FEBRA2000319 | 0.122339577 | 104010 | 39 |
| FEBRA2000321 | 0.004176935 | 139252 | 1 |
| FEBRA2000330 | 0.017511022 | 146332 | 6 |
| FEBRA2000333 | 0.006035049 | 173257 | 2 |
| FEBRA2000377 | 0.016749426 | 69207 | 7 |
| FEBRA2000378 | 0.004176935 | 174863 | 1 |
| FEBRA2000391 | 0.004176935 | 214772 | 1 |
| FEBRA2000394 | 0.004176935 | 212154 | 1 |
| FEBRA2000397 | 0.014412342 | 65918 | 4 |
| FEBRA2000399 | 0.004176935 | 141078 | 1 |
| FEBRA2000402 | 0.030231398 | 97450 | 7 |
| FEBRA2000404 | 0.016652861 | 10099 | 3 |
| FEBRA2000409 | 0.004176935 | 61638 | 1 |
| FEBRA2000415 | 0.004176935 | 115096 | 1 |
| FEBRA2000436 | 0.004176935 | 223385 | 1 |
| FEBRA2000438 | 0.018964506 | 121726 | 6 |
| FEBRA2000452 | 0.16706337 | 118037 | 51 |
| FEBRA2000454 | 0.00557409 | 18672 | 2 |
| FEBRA2000462 | 0.059357307 | 69079 | 36 |
| FEBRA2000468 | 0.004176935 | 182097 | 1 |
| FEBRA2000491 | 0.004176935 | 207236 | 1 |
| FEBRA2000504 | 0.004176935 | 61545 | 1 |
| FEBRA2000510 | 0.045411177 | 65267 | 10 |
| FEBRA2000532 | 0.052396457 | 151351 | 4 |
| FEBRA2000536 | 0.053194835 | 154038 | 20 |
| FEBRA2000538 | 0.010413293 | 133436 | 2 |
| FEBRA2000575 | 0.004176935 | 172903 | 1 |
| FEBRA2000581 | 0.210626844 | 82854 | 102 |
| FEBRA2000656 | 0.004176935 | 81282 | 1 |
| FEBRA2000659 | 0.004176935 | 62321 | 1 |
| FEBRA2000680 | 0.017827821 | 73496 | 10 |
| FEBRA2000690 | 0.004176935 | 41374 | 1 |
| FEBRA2000727 | 0.004176935 | 73716 | 1 |
| FEBRA2000733 | 0.994262346 | 65778 | 106 |
| FEBRA2000734 | 0.004176935 | 204792 | 1 |
| FEBRA2000740 | 0.148155923 | 126429 | 39 |
| FEBRA2000757 | 0.054716008 | 85619 | 27 |
| FEBRA2000762 | 0.026935129 | 103207 | 5 |
| FEBRA2000771 | 0.007723326 | 204285 | 3 |
| FEBRA2000772 | 0.019587871 | 192434 | 4 |
| FEBRA2000782 | 0.006555424 | 126095 | 3 |
| FEBRA2000787 | 0.084829388 | 125809 | 20 |
| FEBRA2000790 | 0.004176935 | 54713 | 1 |
| FEBRA2000793 | 0.043693537 | 146325 | 15 |
| FEBRA2000803 | 0.004176935 | 185525 | 1 |
| FEBRA2000805 | 0.004176935 | 122630 | 1 |
| FEBRA2000809 | 0.004176935 | 180145 | 1 |
| FEBRA2000815 | 0.046348201 | 65761 | 15 |
| FEBRA2000856 | 0.010556449 | 9963 | 6 |
| FEBRA2000862 | 0.004176935 | 101614 | 1 |
| FEBRA2000874 | 0.045830797 | 66559 | 22 |
| FEBRA2000877 | 0.011777362 | 100172 | 6 |
| FEBRA2000880 | 0.015811798 | 119896 | 6 |
| FEBRA2000881 | 0.052934384 | 156622 | 13 |
| FEBRA2000901 | 0.016130763 | 106445 | 3 |
| FEBRA2000909 | 0.004176935 | 223842 | 1 |
| FEBRA2000917 | 0.004176935 | 212227 | 1 |
| FEBRA2000928 | 0.004176935 | 117080 | 1 |
| FEBRA2000936 | 0.004176935 | 192445 | 1 |
| FEBRA2000937 | 0.004176935 | 181738 | 1 |
| FEBRA2000959 | 0.004176935 | 177077 | 1 |
| FEBRA2000972 | 0.011061583 | 118979 | 4 |
| FEBRA2001004 | 0.007574672 | 73453 | 3 |
| FEBRA2001012 | 0.004176935 | 471 | 1 |
| FEBRA2001020 | 0.004176935 | 214167 | 1 |
| FEBRA2001093 | 0.004176935 | 118043 | 1 |
| FEBRA2001124 | 0.004176935 | 32473 | 1 |
| FEBRA2001133 | 0.005272213 | 34757 | 2 |
| FEBRA2001134 | 0.212174642 | 84844 | 73 |
| FEBRA2001146 | 0.014983635 | 66459 | 8 |
| FEBRA2001161 | 0.004176935 | 214147 | 1 |
| FEBRA2001175 | 0.004176935 | 249403 | 1 |
| FEBRA2001197 | 0.01188635 | 165277 | 5 |
| FEBRA2001217 | 0.004176935 | 96908 | 1 |
| FEBRA2001227 | 0.045319674 | 88377 | 15 |
| FEBRA2001245 | 0.066428826 | 75508 | 28 |
| FEBRA2001267 | 0.004176935 | 41876 | 1 |
| FEBRA2001294 | 0.069335056 | 108604 | 12 |
| FEBRA2001334 | 0.029136712 | 58548 | 13 |
| FEBRA2001351 | 0.004176935 | 277232 | 1 |
| FEBRA2001383 | 0.016062214 | 141059 | 5 |
| FEBRA2001438 | 0.004176935 | 192423 | 1 |
| FEBRA2001440 | 0.014395436 | 96434 | 3 |
| FEBRA2001487 | 0.004176935 | 223847 | 1 |
| FEBRA2001492 | 0.051769295 | 91638 | 26 |
| FEBRA2001523 | 0.01970648 | 186423 | 4 |
| FEBRA2001529 | 0.049104411 | 110021 | 25 |
| FEBRA2001561 | 0.004176935 | 214799 | 1 |
| FEBRA2001571 | 0.079898853 | 12275 | 26 |
| FEBRA2001581 | 0.040864724 | 22680 | 11 |
| FEBRA2001584 | 0.004176935 | 149220 | 1 |
| FEBRA2001590 | 0.004176935 | 223518 | 1 |
| FEBRA2001591 | 0.016402976 | 148591 | 5 |
| FEBRA2001593 | 0.106885695 | 21293 | 46 |
| FEBRA2001610 | 0.004176935 | 77006 | 1 |
| FEBRA2001620 | 0.004176935 | 146214 | 1 |
| FEBRA2001659 | 0.004176935 | 35993 | 1 |
| FEBRA2001669 | 0.156407021 | 83699 | 77 |
| FEBRA2001698 | 0.013764262 | 145370 | 4 |
| FEBRA2001705 | 0.004176935 | 21251 | 1 |
| FEBRA2001706 | 0.005852482 | 139638 | 2 |
| FEBRA2001715 | 0.168037623 | 64622 | 68 |
| FEBRA2001745 | 0.133701175 | 71102 | 22 |
| FEBRA2001752 | 0.010638393 | 90140 | 4 |
| FEBRA2001772 | 0.004176935 | 242141 | 1 |
| FEBRA2001790 | 0.004176935 | 157502 | 1 |
| FEBRA2001814 | 0.12664823 | 95070 | 44 |
| FEBRA2001828 | 0.030797704 | 151754 | 10 |
| FEBRA2001867 | 0.045209688 | 119453 | 14 |
| FEBRA2001914 | 0.004176935 | 151811 | 1 |
| FEBRA2001974 | 0.014380448 | 108212 | 6 |
| FEBRA2001989 | 0.00557409 | 137054 | 2 |
| FEBRA2001990 | 0.046173839 | 118218 | 22 |
| FEBRA2002009 | 0.004176935 | 267335 | 1 |
| FEBRA2002027 | 0.066135695 | 58584 | 21 |
| FEBRA2002086 | 0.004176935 | 172253 | 1 |
| FEBRA2002109 | 0.004176935 | 280861 | 1 |
| FEBRA2002191 | 0.028522523 | 139468 | 4 |
| FEBRA2002194 | 0.138211456 | 118822 | 32 |
| FEBRA2002260 | 0.004176935 | 242171 | 1 |
| FEBRA2002352 | 0.048268514 | 117002 | 14 |
| FEBRA2002399 | 0.004176935 | 136412 | 1 |
| FEBRA2002410 | 0.006258447 | 185295 | 2 |
| FEBRA2002429 | 0.042790317 | 218135 | 3 |
| FEBRA2002442 | 0.008021019 | 177512 | 2 |
| FEBRA2002456 | 0.153620295 | 72543 | 66 |
| FEBRA2002508 | 0.004176935 | 64653 | 1 |
| FEBRA2002525 | 0.006258447 | 85737 | 2 |
| FEBRA2002527 | 0.058506177 | 111123 | 9 |
| FEBRA2002552 | 0.004176935 | 88046 | 1 |
| FEBRA2002574 | 0.00557409 | 103876 | 2 |
| FEBRA2002601 | 0.015696345 | 5562 | 2 |
| FEBRA2002611 | 0.007710596 | 92071 | 3 |
| FEBRA2002628 | 0.004176935 | 148620 | 1 |
| FEBRA2002662 | 0.004176935 | 238882 | 1 |
| FEBRA2002682 | 0.006258447 | 110880 | 2 |
| FEBRA2002707 | 0.046501045 | 27368 | 8 |
| FEBRA2002727 | 0.004176935 | 175605 | 1 |
| FEBRA2002781 | 0.024488495 | 93575 | 15 |
| FEBRA2002783 | 0.004176935 | 173916 | 1 |
| FEBRA2002792 | 0.125180907 | 83171 | 50 |
| FEBRA2002818 | 0.004176935 | 269467 | 1 |
| FEBRA2002858 | 0.004176935 | 264199 | 1 |
| FEBRA2002882 | 0.004176935 | 254902 | 1 |
| FEBRA2002897 | 0.004176935 | 212093 | 1 |
| FEBRA2002908 | 0.004176935 | 163856 | 1 |
| FEBRA2002931 | 0.153107618 | 104282 | 74 |
| FEBRA2002933 | 0.452774471 | 47338 | 38 |
| FEBRA2002962 | 0.004176935 | 83476 | 1 |
| FEBRA2002986 | 0.004176935 | 240045 | 1 |
| FEBRA2003054 | 0.004176935 | 62330 | 1 |
| FEBRA2003100 | 0.254417087 | 88710 | 39 |
| FEBRA2003115 | 0.007729324 | 75428 | 3 |
| FEBRA2003128 | 0.057602096 | 35368 | 29 |
| FEBRA2003155 | 0.004176935 | 235304 | 1 |
| FEBRA2003163 | 0.00835387 | 226188 | 2 |
| FEBRA2003181 | 0.00557409 | 259109 | 2 |
| FEBRA2003198 | 0.006258447 | 176644 | 2 |
| FEBRA2003253 | 0.004176935 | 94104 | 1 |
| FEBRA2003258 | 0.059223674 | 129836 | 13 |
| FEBRA2003275 | 0.004176935 | 261389 | 1 |
| FEBRA2003308 | 0.004176935 | 254043 | 1 |
| FEBRA2003327 | 0.004176935 | 126193 | 1 |
| FEBRA2003429 | 0.004176935 | 45084 | 1 |
| FEBRA2003436 | 0.030140426 | 59024 | 8 |
| FEBRA2003454 | 0.004176935 | 143803 | 1 |
| FEBRA2003468 | 0.00835387 | 213833 | 2 |
| FEBRA2003470 | 0.011480385 | 129650 | 5 |
| FEBRA2003520 | 0.004176935 | 42392 | 1 |
| FEBRA2003524 | 0.016398886 | 105446 | 2 |
| FEBRA2003541 | 0.109806822 | 105115 | 27 |
| FEBRA2003675 | 0.004176935 | 84330 | 1 |
| FEBRA2003707 | 0.008021019 | 175985 | 2 |
| FEBRA2003711 | 0.004176935 | 222118 | 1 |
| FEBRA2003726 | 0.168260867 | 52967 | 9 |
| FEBRA2003733 | 0.119522789 | 61527 | 44 |
| FEBRA2003750 | 0.091784057 | 141213 | 31 |
| FEBRA2003822 | 0.030464585 | 161581 | 3 |
| FEBRA2003833 | 0.096900985 | 70230 | 41 |
| FEBRA2003897 | 0.004176935 | 181699 | 1 |
| FEBRA2003907 | 0.005366179 | 174415 | 2 |
| FEBRA2003926 | 0.24002786 | 128692 | 35 |
| FEBRA2003930 | 0.093229135 | 120142 | 14 |
| FEBRA2004023 | 0.005272213 | 158238 | 2 |
| FEBRA2004026 | 0.004176935 | 74912 | 1 |
| FEBRA2004029 | 0.004176935 | 42428 | 1 |
| FEBRA2004042 | 0.078988859 | 44190 | 16 |
| FEBRA2004053 | 0.004176935 | 118450 | 1 |
| FEBRA2004056 | 0.004176935 | 139290 | 1 |
| FEBRA2004091 | 0.015855022 | 79489 | 6 |
| FEBRA2004099 | 0.066903155 | 139726 | 18 |
| FEBRA2004110 | 0.006258447 | 132401 | 2 |
| FEBRA2004191 | 0.008449003 | 190594 | 4 |
| FEBRA2004202 | 0.004176935 | 250783 | 1 |
| FEBRA2004219 | 0.005886395 | 128740 | 2 |
| FEBRA2004224 | 0.14472965 | 126539 | 32 |
| FEBRA2004237 | 0.008933913 | 202823 | 5 |
| FEBRA2004268 | 0.009543114 | 209948 | 3 |
| FEBRA2004293 | 0.063029631 | 75155 | 21 |
| FEBRA2004325 | 0.047571095 | 89569 | 10 |
| FEBRA2004329 | 0.004176935 | 244185 | 1 |
| FEBRA2004331 | 0.00557409 | 159910 | 2 |
| FEBRA2004412 | 0.057069716 | 154207 | 16 |
| FEBRA2004443 | 0.004176935 | 250767 | 1 |
| FEBRA2004485 | 0.024143038 | 164816 | 7 |
| FEBRA2004490 | 0.011887531 | 112414 | 4 |
| FEBRA2004538 | 0.004176935 | 152600 | 1 |
| FEBRA2004592 | 0.010413293 | 81949 | 2 |
| FEBRA2004620 | 0.017098501 | 91748 | 2 |
| FEBRA2004628 | 0.058739771 | 122287 | 14 |
| FEBRA2004651 | 0.004176935 | 216779 | 1 |
| FEBRA2004767 | 0.006555424 | 109708 | 3 |
| FEBRA2004813 | 0.004176935 | 246503 | 1 |
| FEBRA2004818 | 0.123048402 | 93677 | 31 |
| FEBRA2004840 | 0.004176935 | 242895 | 1 |
| FEBRA2004852 | 0.006175336 | 251770 | 2 |
| FEBRA2004867 | 0.004176935 | 161383 | 1 |
| FEBRA2004914 | 0.004176935 | 127069 | 1 |
| FEBRA2004933 | 0.004176935 | 213438 | 1 |
| FEBRA2004987 | 0.004176935 | 222662 | 1 |
| FEBRA2005014 | 0.004176935 | 57040 | 1 |
| FEBRA2005036 | 0.004176935 | 223872 | 1 |
| FEBRA2005079 | 0.010042147 | 94042 | 3 |
| FEBRA2005114 | 0.024970504 | 42183 | 7 |
| FEBRA2005216 | 0.004176935 | 272863 | 1 |
| FEBRA2005291 | 0.046202668 | 100772 | 3 |
| FEBRA2005361 | 0.23923172 | 104456 | 77 |
| FEBRA2005377 | 0.004176935 | 254136 | 1 |
| FEBRA2005380 | 0.004176935 | 255991 | 1 |
| FEBRA2005416 | 0.156027624 | 123623 | 20 |
| FEBRA2005427 | 0.005852482 | 208219 | 2 |
| FEBRA2005458 | 0.004176935 | 182098 | 1 |
| FEBRA2005476 | 0.064330012 | 161302 | 10 |
| FEBRA2005572 | 0.094039945 | 100147 | 23 |
| FEBRA2005701 | 0.004176935 | 262739 | 1 |
| FEBRA2005726 | 0.004176935 | 109196 | 1 |
| FEBRA2005734 | 0.198445894 | 40243 | 19 |
| FEBRA2005752 | 0.040255197 | 143138 | 9 |
| FEBRA2005778 | 0.055859713 | 156866 | 12 |
| FEBRA2005787 | 0.00557409 | 170802 | 2 |
| FEBRA2005788 | 0.033949775 | 120367 | 11 |
| FEBRA2005824 | 0.449223397 | 100968 | 136 |
| FEBRA2005995 | 0.119522789 | 61527 | 44 |
| FEBRA2005998 | 0.004176935 | 60173 | 1 |
| FEBRA2006055 | 0.063434366 | 179859 | 6 |
| FEBRA2006061 | 0.004176935 | 56171 | 1 |
| FEBRA2006092 | 0.269467869 | 37856 | 45 |
| FEBRA2006112 | 0.025385361 | 34360 | 13 |
| FEBRA2006150 | 0.004176935 | 270568 | 1 |
| FEBRA2006165 | 0.004176935 | 81741 | 1 |
| FEBRA2006255 | 0.128587489 | 91402 | 52 |
| FEBRA2006270 | 0.14333092 | 114889 | 24 |
| FEBRA2006315 | 0.004176935 | 266752 | 1 |
| FEBRA2006346 | 0.011278791 | 80941 | 3 |
| FEBRA2006354 | 0.004176935 | 167128 | 1 |
| FEBRA2006357 | 0.252967967 | 78596 | 135 |
| FEBRA2006368 | 0.053922974 | 99624 | 20 |
| FEBRA2006370 | 0.011727858 | 41237 | 3 |
| FEBRA2006372 | 0.124762606 | 18616 | 22 |
| FEBRA2006379 | 0.004176935 | 196938 | 1 |
| FEBRA2006396 | 0.020846589 | 175672 | 8 |
| FEBRA2006401 | 0.667575146 | 98766 | 29 |
| FEBRA2006409 | 0.004176935 | 202608 | 1 |
| FEBRA2006418 | 0.004176935 | 29177 | 1 |
| FEBRA2006427 | 0.004176935 | 181899 | 1 |
| FEBRA2006476 | 0.004176935 | 164491 | 1 |
| FEBRA2006485 | 0.055081272 | 130929 | 13 |
| FEBRA2006491 | 0.011283258 | 91401 | 5 |
| FEBRA2006519 | 0.014090451 | 81576 | 7 |
| FEBRA2006521 | 0.02190798 | 138761 | 10 |
| FEBRA2006553 | 0.042352188 | 117092 | 10 |
| FEBRA2006627 | 0.016240411 | 111564 | 6 |
| FEBRA2006664 | 0.005272213 | 18921 | 2 |
| FEBRA2006667 | 0.004176935 | 103319 | 1 |
| FEBRA2006726 | 0.004176935 | 274740 | 1 |
| FEBRA2006736 | 0.011278791 | 148018 | 3 |
| FEBRA2006793 | 0.004176935 | 196322 | 1 |
| FEBRA2006808 | 0.040818316 | 7695 | 12 |
| FEBRA2006817 | 0.010218501 | 110819 | 2 |
| FEBRA2006873 | 0.037313918 | 69447 | 8 |
| FEBRA2006890 | 0.007528029 | 152681 | 3 |
| FEBRA2006942 | 0.004176935 | 55987 | 1 |
| FEBRA2006977 | 0.004176935 | 213028 | 1 |
| FEBRA2007017 | 0.010231303 | 114664 | 2 |
| FEBRA2007176 | 0.005852482 | 161700 | 2 |
| FEBRA2007200 | 0.005272213 | 211510 | 2 |
| FEBRA2007212 | 0.042684601 | 94310 | 17 |
| FEBRA2007247 | 0.014051528 | 74992 | 2 |
| FEBRA2007280 | 0.004176935 | 177111 | 1 |
| FEBRA2007414 | 0.004176935 | 236821 | 1 |
| FEBRA2007436 | 0.004176935 | 64921 | 1 |
| FEBRA2007449 | 0.004176935 | 212459 | 1 |
| FEBRA2007458 | 0.020552719 | 126626 | 6 |
| FEBRA2007534 | 0.009284132 | 60262 | 4 |
| FEBRA2007544 | 0.068440576 | 18116 | 42 |
| FEBRA2007551 | 0.006035049 | 50243 | 2 |
| FEBRA2007566 | 0.004176935 | 229682 | 1 |
| FEBRA2007589 | 0.024506169 | 132139 | 14 |
| FEBRA2007613 | 0.050493173 | 87471 | 17 |
| FEBRA2007620 | 0.005272213 | 274280 | 2 |
| FEBRA2007622 | 0.162411089 | 113012 | 26 |
| FEBRA2007708 | 0.019604797 | 115093 | 15 |
| FEBRA2007714 | 0.020081166 | 141389 | 9 |
| FEBRA2007793 | 0.008339959 | 148844 | 3 |
| FEBRA2007801 | 0.067873593 | 40240 | 21 |
| FEBRA2007818 | 0.029964139 | 103573 | 5 |
| FEBRA2007823 | 0.005852482 | 163986 | 2 |
| FEBRA2007880 | 0.044518678 | 102314 | 15 |
| FEBRA2007900 | 0.02161745 | 108630 | 10 |
| FEBRA2007901 | 0.045209184 | 84151 | 11 |
| FEBRA2007931 | 0.004176935 | 131059 | 1 |
| FEBRA2007957 | 0.009203576 | 126442 | 4 |
| FEBRA2008009 | 0.004176935 | 223799 | 1 |
| FEBRA2008036 | 0.117309152 | 99020 | 46 |
| FEBRA2008081 | 0.23661529 | 56755 | 57 |
| FEBRA2008086 | 0.006035049 | 42736 | 2 |
| FEBRA2008087 | 0.934772397 | 43032 | 73 |
| FEBRA2008134 | 0.005865212 | 157061 | 2 |
| FEBRA2008135 | 0.004176935 | 212501 | 1 |
| FEBRA2008159 | 0.005366179 | 35865 | 2 |
| FEBRA2008165 | 0.004176935 | 163062 | 1 |
| FEBRA2008172 | 0.004176935 | 158014 | 1 |
| FEBRA2008201 | 0.069870416 | 108703 | 12 |
| FEBRA2008210 | 0.024981157 | 167548 | 8 |
| FEBRA2008255 | 0.033112123 | 89656 | 10 |
| FEBRA2008265 | 0.010231303 | 236535 | 2 |
| FEBRA2008266 | 0.018439689 | 138368 | 4 |
| FEBRA2008282 | 0.004176935 | 54789 | 1 |
| FEBRA2008287 | 0.056845056 | 24108 | 10 |
| FEBRA2008302 | 0.157446218 | 2665 | 33 |
| FEBRA2008311 | 0.004176935 | 53554 | 1 |
| FEBRA2008341 | 0.025507452 | 91475 | 6 |
| FEBRA2008360 | 0.016389065 | 111079 | 6 |
| FEBRA2008459 | 0.005865212 | 156347 | 2 |
| FEBRA2008468 | 0.004176935 | 1224 | 1 |
| FEBRA2008475 | 0.019858702 | 128443 | 3 |
| FEBRA2008481 | 0.004176935 | 164501 | 1 |
| FEBRA2008542 | 0.004176935 | 230774 | 1 |
| FEBRA2008583 | 1.267697555 | 27207 | 213 |
| FEBRA2008660 | 0.004176935 | 278426 | 1 |
| FEBRA2008662 | 0.105565309 | 110213 | 33 |
| FEBRA2008681 | 0.004176935 | 264225 | 1 |
| FEBRA2008692 | 0.068291938 | 134261 | 15 |
| FEBRA2008741 | 0.004176935 | 231739 | 1 |
| FEBRA2008755 | 0.102006309 | 66392 | 9 |
| FEBRA2008836 | 0.005272213 | 19098 | 2 |
| FEBRA2008859 | 0.005366179 | 253884 | 2 |
| FEBRA2008861 | 0.086031312 | 95688 | 37 |
| FEBRA2008866 | 0.004176935 | 226965 | 1 |
| FEBRA2008881 | 0.004176935 | 256972 | 1 |
| FEBRA2008928 | 0.005366179 | 63023 | 2 |
| FEBRA2008983 | 0.004176935 | 241847 | 1 |
| FEBRA2009016 | 0.004176935 | 111575 | 1 |
| FEBRA2009022 | 0.050172916 | 76242 | 12 |
| FEBRA2009029 | 0.004176935 | 196323 | 1 |
| FEBRA2009074 | 0.004176935 | 145066 | 1 |
| FEBRA2009117 | 0.004176935 | 210754 | 1 |
| FEBRA2009120 | 0.004176935 | 186096 | 1 |
| FEBRA2009162 | 0.004176935 | 110029 | 1 |
| FEBRA2009185 | 0.184420408 | 89752 | 80 |
| FEBRA2009240 | 0.004176935 | 238850 | 1 |
| FEBRA2009265 | 0.004176935 | 112418 | 1 |
| FEBRA2009276 | 0.007158894 | 96904 | 2 |
| FEBRA2009289 | 0.011940763 | 139044 | 3 |
| FEBRA2009327 | 0.004176935 | 222085 | 1 |
| FEBRA2009328 | 0.033813702 | 107117 | 6 |
| FEBRA2009352 | 0.005886395 | 216904 | 2 |
| FEBRA2009362 | 0.022006758 | 198442 | 5 |
| FEBRA2009385 | 0.004176935 | 173199 | 1 |
| FEBRA2009416 | 0.004176935 | 78048 | 1 |
| FEBRA2009419 | 0.126812117 | 121445 | 44 |
| FEBRA2009437 | 0.045982955 | 105449 | 2 |
| FEBRA2009478 | 0.004176935 | 192483 | 1 |
| FEBRA2009507 | 0.004176935 | 274534 | 1 |
| FEBRA2009514 | 0.050584741 | 114032 | 12 |
| FEBRA2009541 | 0.010073751 | 110374 | 3 |
| FEBRA2009567 | 0.004176935 | 101574 | 1 |
| FEBRA2009588 | 0.034580253 | 128442 | 15 |
| FEBRA2009731 | 0.004176935 | 32693 | 1 |
| FEBRA2009754 | 0.004176935 | 21279 | 1 |
| FEBRA2009780 | 0.006053777 | 66242 | 2 |
| FEBRA2009804 | 0.033048593 | 122269 | 11 |
| FEBRA2009846 | 0.008116561 | 23934 | 3 |
| FEBRA2009986 | 0.026068598 | 143534 | 13 |
| FEBRA2010141 | 0.004176935 | 179626 | 1 |
| FEBRA2010299 | 0.004176935 | 100899 | 1 |
| FEBRA2010484 | 0.004176935 | 216383 | 1 |
| FEBRA2010719 | 0.009656184 | 189228 | 4 |
| FEBRA2010746 | 0.019530799 | 109453 | 9 |
| FEBRA2010768 | 0.010218501 | 82636 | 2 |
| FEBRA2010802 | 0.004176935 | 263426 | 1 |
| FEBRA2010858 | 0.038439333 | 112371 | 8 |
| FEBRA2011063 | 0.004176935 | 158095 | 1 |
| FEBRA2011090 | 0.009654004 | 53618 | 4 |
| FEBRA2011356 | 0.004176935 | 66635 | 1 |
| FEBRA2011392 | 0.012350672 | 179765 | 4 |
| FEBRA2011414 | 0.004176935 | 54879 | 1 |
| FEBRA2011593 | 0.004176935 | 41044 | 1 |
| FEBRA2011665 | 0.004176935 | 182229 | 1 |
| FEBRA2012090 | 0.004176935 | 152453 | 1 |
| FEBRA2012120 | 0.010379255 | 75673 | 2 |
| FEBRA2012181 | 0.010148342 | 88797 | 5 |
| FEBRA2012195 | 0.004176935 | 210665 | 1 |
| FEBRA2012397 | 0.007158894 | 66332 | 2 |
| FEBRA2012418 | 0.004176935 | 117254 | 1 |
| FEBRA2012507 | 0.022453254 | 197553 | 3 |
| FEBRA2012625 | 0.004176935 | 219988 | 1 |
| FEBRA2013019 | 0.007272431 | 207383 | 2 |
| FEBRA2013069 | 0.004176935 | 276428 | 1 |
| FEBRA2013274 | 0.004176935 | 100846 | 1 |
| FEBRA2013465 | 0.004176935 | 98695 | 1 |
| FEBRA2013570 | 0.015004133 | 58831 | 2 |
| FEBRA2013699 | 0.023412536 | 106733 | 10 |
| FEBRA2013905 | 0.004176935 | 188051 | 1 |
| FEBRA2013951 | 0.010218501 | 48778 | 2 |
| FEBRA2014010 | 0.010379255 | 52372 | 2 |
| FEBRA2014122 | 0.006258447 | 211231 | 2 |
| FEBRA2014198 | 0.004176935 | 121977 | 1 |
| FEBRA2014213 | 0.042550127 | 198616 | 3 |
| FEBRA2014297 | 0.078398788 | 22094 | 14 |
| FEBRA2014417 | 0.310323648 | 123452 | 71 |
| FEBRA2014443 | 0.004176935 | 181650 | 1 |
| FEBRA2014578 | 0.007655602 | 199666 | 3 |
| FEBRA2014939 | 0.02005848 | 135477 | 9 |
| FEBRA2015042 | 0.162544289 | 118129 | 10 |
| FEBRA2015076 | 0.004176935 | 8597 | 1 |
| FEBRA2015085 | 0.109895262 | 46839 | 43 |
| FEBRA2015175 | 0.004176935 | 153648 | 1 |
| FEBRA2015292 | 0.004176935 | 223223 | 1 |
| FEBRA2015588 | 0.004176935 | 166658 | 1 |
| FEBRA2016112 | 0.004176935 | 108652 | 1 |
| FEBRA2016398 | 0.009696566 | 8638 | 3 |
| FEBRA2016572 | 0.008527483 | 213901 | 4 |
| FEBRA2016583 | 0.005886395 | 180408 | 2 |
| FEBRA2016621 | 0.004176935 | 168142 | 1 |
| FEBRA2016654 | 0.004176935 | 203372 | 1 |
| FEBRA2016739 | 0.004176935 | 17832 | 1 |
| FEBRA2017024 | 0.01736624 | 132873 | 6 |
| FEBRA2017098 | 0.007272431 | 145411 | 2 |
| FEBRA2017138 | 0.004176935 | 162604 | 1 |
| FEBRA2017154 | 0.004176935 | 210959 | 1 |
| FEBRA2017200 | 0.004176935 | 165288 | 1 |
| FEBRA2017223 | 0.004176935 | 134801 | 1 |
| FEBRA2017333 | 0.004176935 | 108289 | 1 |
| FEBRA2017441 | 0.330158685 | 120529 | 61 |
| FEBRA2017502 | 0.004176935 | 219556 | 1 |
| FEBRA2017533 | 0.030714937 | 71298 | 12 |
| FEBRA2017680 | 0.017621075 | 129041 | 5 |
| FEBRA2017736 | 0.004176935 | 190247 | 1 |
| FEBRA2017780 | 0.010367927 | 115664 | 3 |
| FEBRA2017811 | 0.004176935 | 199751 | 1 |
| FEBRA2017850 | 0.009353943 | 48773 | 3 |
| FEBRA2018051 | 0.004176935 | 223975 | 1 |
| FEBRA2018203 | 0.01104222 | 199198 | 4 |
| FEBRA2018407 | 0.010342623 | 181890 | 3 |
| FEBRA2018433 | 0.005366179 | 111538 | 2 |
| FEBRA2018476 | 0.153349683 | 103898 | 23 |
| FEBRA2018746 | 0.022404216 | 117454 | 5 |
| FEBRA2018868 | 0.109806822 | 105115 | 27 |
| FEBRA2019172 | 0.004176935 | 140282 | 1 |
| FEBRA2019242 | 0.020652321 | 110291 | 8 |
| FEBRA2019298 | 0.069220147 | 146588 | 4 |
| FEBRA2019378 | 0.028343935 | 165950 | 7 |
| FEBRA2019389 | 0.010231303 | 235384 | 2 |
| FEBRA2019457 | 0.004176935 | 127687 | 1 |
| FEBRA2019519 | 0.004176935 | 140063 | 1 |
| FEBRA2019582 | 0.004176935 | 152928 | 1 |
| FEBRA2019637 | 0.010249318 | 110294 | 2 |
| FEBRA2019663 | 0.019237825 | 119678 | 4 |
| FEBRA2019690 | 0.010379255 | 214054 | 2 |
| FEBRA2019711 | 0.07923718 | 28801 | 11 |
| FEBRA2019867 | 0.006035049 | 93839 | 2 |
| FEBRA2020400 | 0.045223526 | 103242 | 18 |
| FEBRA2020406 | 0.007272431 | 190803 | 2 |
| FEBRA2020484 | 0.045837097 | 22079 | 14 |
| FEBRA2020566 | 0.040212971 | 83643 | 2 |
| FEBRA2020582 | 0.007272431 | 115348 | 2 |
| FEBRA2020668 | 0.004176935 | 49133 | 1 |
| FEBRA2020801 | 0.004176935 | 137452 | 1 |
| FEBRA2020845 | 0.005852482 | 253224 | 2 |
| FEBRA2020886 | 0.013820389 | 43862 | 5 |
| FEBRA2020977 | 0.031797766 | 48839 | 7 |
| FEBRA2021032 | 0.031253286 | 3688 | 5 |
| FEBRA2021134 | 0.004176935 | 126153 | 1 |
| FEBRA2021171 | 0.015750862 | 131671 | 4 |
| FEBRA2021339 | 0.005272213 | 133147 | 2 |
| FEBRA2021431 | 0.004176935 | 123477 | 1 |
| FEBRA2021497 | 0.004176935 | 112425 | 1 |
| FEBRA2021550 | 0.005366179 | 112254 | 2 |
| FEBRA2021571 | 0.004176935 | 61967 | 1 |
| FEBRA2021636 | 0.008281036 | 72901 | 3 |
| FEBRA2021864 | 0.005366179 | 116323 | 2 |
| FEBRA2021892 | 0.004176935 | 107158 | 1 |
| FEBRA2021908 | 0.004176935 | 272994 | 1 |
| FEBRA2021966 | 0.038843984 | 110533 | 7 |
| FEBRA2022013 | 0.017821942 | 21906 | 3 |
| FEBRA2022055 | 0.314892511 | 96889 | 33 |
| FEBRA2022148 | 0.004176935 | 108629 | 1 |
| FEBRA2022204 | 0.004176935 | 104762 | 1 |
| FEBRA2022255 | 0.004176935 | 66090 | 1 |
| FEBRA2022322 | 0.004176935 | 49854 | 1 |
| FEBRA2022388 | 0.219398702 | 86661 | 51 |
| FEBRA2022504 | 0.102020016 | 144180 | 19 |
| FEBRA2022601 | 0.005366179 | 108100 | 2 |
| FEBRA2022696 | 0.008021019 | 255161 | 2 |
| FEBRA2022729 | 0.004176935 | 107507 | 1 |
| FEBRA2022787 | 0.004176935 | 29149 | 1 |
| FEBRA2022911 | 0.004176935 | 74986 | 1 |
| FEBRA2022956 | 0.004176935 | 184652 | 1 |
| FEBRA2022963 | 0.004176935 | 104928 | 1 |
| FEBRA2022972 | 0.004176935 | 48074 | 1 |
| FEBRA2023227 | 0.032715806 | 42324 | 5 |
| FEBRA2023285 | 0.004176935 | 269499 | 1 |
| FEBRA2023351 | 0.004176935 | 109927 | 1 |
| FEBRA2023377 | 0.021599684 | 82634 | 6 |
| FEBRA2023399 | 0.019089191 | 105396 | 4 |
| FEBRA2023498 | 0.004176935 | 250642 | 1 |
| FEBRA2023550 | 0.010231303 | 108011 | 2 |
| FEBRA2023721 | 0.004176935 | 108781 | 1 |
| FEBRA2023764 | 0.142252985 | 132485 | 49 |
| FEBRA2023927 | 0.041138741 | 14663 | 17 |
| FEBRA2023989 | 0.004176935 | 109770 | 1 |
| FEBRA2023990 | 0.004176935 | 106902 | 1 |
| FEBRA2024019 | 0.124941952 | 90732 | 35 |
| FEBRA2024136 | 0.014604759 | 33488 | 5 |
| FEBRA2024150 | 0.00746277 | 103517 | 4 |
| FEBRA2024343 | 0.004176935 | 104950 | 1 |
| FEBRA2024570 | 0.016649651 | 109698 | 3 |
| FEBRA2024693 | 0.004176935 | 108953 | 1 |
| FEBRA2024727 | 0.006258447 | 89739 | 2 |
| FEBRA2024744 | 0.004176935 | 108970 | 1 |
| FEBRA2024797 | 0.004176935 | 162537 | 1 |
| FEBRA2024987 | 0.017032768 | 112421 | 3 |
| FEBRA2025249 | 0.004176935 | 108593 | 1 |
| FEBRA2025427 | 0.067594876 | 113139 | 15 |
| FEBRA2025463 | 0.004176935 | 150904 | 1 |
| FEBRA2025477 | 0.004176935 | 107718 | 1 |
| FEBRA2025838 | 0.203971855 | 98825 | 23 |
| FEBRA2026474 | 0.004176935 | 169879 | 1 |
| FEBRA2026582 | 0.007221817 | 151672 | 2 |
| FEBRA2026629 | 0.004176935 | 261073 | 1 |
| FEBRA2026769 | 0.004176935 | 181728 | 1 |
| FEBRA2026879 | 0.004176935 | 221496 | 1 |
| FEBRA2026890 | 0.004176935 | 178116 | 1 |
| FEBRA2026977 | 0.004176935 | 229822 | 1 |
| FEBRA2026984 | 0.009555137 | 161905 | 5 |
| FEBRA2027082 | 0.004176935 | 195860 | 1 |
| FEBRA2027297 | 0.004176935 | 252510 | 1 |
| FEBRA2027352 | 0.004176935 | 126254 | 1 |
| FEBRA2027364 | 0.004176935 | 184588 | 1 |
| FEBRA2027609 | 0.004176935 | 18770 | 1 |
| FEBRA2027742 | 0.025838097 | 13600 | 14 |
| FEBRA2028158 | 0.006053777 | 246879 | 2 |
| FEBRA2028222 | 0.004176935 | 284042 | 1 |
| FEBRA2028256 | 0.02236498 | 181783 | 4 |
| FEBRA2028366 | 0.004176935 | 280117 | 1 |
| FEBRA2028457 | 0.004176935 | 14163 | 1 |
| FEBRA2028477 | 0.004176935 | 166823 | 1 |
| FEBRA2028503 | 0.023965831 | 148429 | 7 |
| FEBRA2028516 | 0.146491235 | 88510 | 41 |
| FEBRA2028618 | 0.032970596 | 75233 | 13 |
| FEHRT2000325 | 0.034891835 | 70992 | 1 |
| FEHRT2000364 | 0.037806692 | 57020 | 2 |
| FEKID1000018 | 0.042447336 | 117026 | 2 |
| FELIV1000101 | 0.539031564 | 44356 | 2 |
| FELIV1000137 | 0.540700495 | 56709 | 3 |
| FELIV1000153 | 1.015909407 | 54819 | 42 |
| FELIV1000164 | 0.576279927 | 46875 | 4 |
| FELNG2000241 | 0.036036036 | 282691 | 1 |
| HCASM1000021 | 0.011124708 | 126275 | 1 |
| HCASM1000050 | 0.011124708 | 98126 | 1 |
| HCASM1000061 | 0.011124708 | 19737 | 1 |
| HCASM1000115 | 0.16165611 | 52993 | 20 |
| HCASM1000130 | 0.011124708 | 56971 | 1 |
| HCASM2000016 | 0.224501738 | 98352 | 38 |
| HCASM2000138 | 0.039671728 | 19852 | 19 |
| HCASM2000156 | 0.012812985 | 119815 | 2 |
| HCASM2000202 | 0.011124708 | 232040 | 1 |
| HCASM2000214 | 0.017715981 | 67601 | 4 |
| HCASM2000266 | 0.011124708 | 156085 | 1 |
| HCASM2000307 | 0.011124708 | 114209 | 1 |
| HCASM2000363 | 0.246392643 | 31143 | 25 |
| HCASM2000534 | 0.011124708 | 179022 | 1 |
| HCASM2000536 | 0.011124708 | 111511 | 1 |
| HCASM2000560 | 0.011124708 | 253520 | 1 |
| HCASM2000871 | 0.011124708 | 205937 | 1 |
| HCASM2001285 | 0.011124708 | 201665 | 1 |
| HCASM2001301 | 0.017327028 | 113838 | 2 |
| HCASM2001810 | 0.011124708 | 246145 | 1 |
| HCASM2001866 | 0.011124708 | 14388 | 1 |
| HCASM2001890 | 0.013123109 | 245441 | 2 |
| HCASM2002148 | 0.011124708 | 224626 | 1 |
| HCASM2002502 | 0.011124708 | 55010 | 1 |
| HCASM2002754 | 0.011124708 | 270319 | 1 |
| HCASM2002918 | 0.011124708 | 210349 | 1 |
| HCASM2003018 | 0.018984488 | 193278 | 7 |
| HCASM2003076 | 0.02942649 | 23765 | 4 |
| HCASM2003099 | 0.011124708 | 227979 | 1 |
| HCASM2003212 | 0.011124708 | 280389 | 1 |
| HCASM2003237 | 0.011124708 | 112291 | 1 |
| HCASM2003357 | 0.011124708 | 274475 | 1 |
| HCASM2003415 | 0.099890733 | 140729 | 3 |
| HCASM2005388 | 0.047371899 | 122277 | 8 |
| HCASM2005484 | 0.012982822 | 111937 | 2 |
| HCASM2006131 | 0.011124708 | 205625 | 1 |
| HCASM2006338 | 0.011124708 | 172892 | 1 |
| HCASM2006359 | 0.011124708 | 185574 | 1 |
| HCASM2006632 | 0.011124708 | 245983 | 1 |
| HCASM2007047 | 0.048836291 | 170371 | 3 |
| HCASM2007317 | 0.011124708 | 220628 | 1 |
| HCASM2007737 | 0.011124708 | 148618 | 1 |
| HCASM2007773 | 0.011124708 | 120553 | 1 |
| HCASM2008154 | 0.011124708 | 15455 | 1 |
| HCASM2008536 | 0.013123109 | 130406 | 2 |
| HCASM2009405 | 0.011124708 | 273620 | 1 |
| HCASM2009424 | 0.011124708 | 185414 | 1 |
| HCASM2009463 | 0.011124708 | 220610 | 1 |
| HCASM2009588 | 0.160156005 | 136063 | 2 |
| HCHON1000015 | 0.107497299 | 5029 | 14 |
| HCHON1000030 | 0.923793667 | 28724 | 139 |
| HCHON1000131 | 0.040839417 | 65581 | 12 |
| HCHON1000142 | 0.012600607 | 35194 | 2 |
| HCHON1000166 | 0.055699267 | 64723 | 11 |
| HCHON1000176 | 0.010602205 | 67842 | 1 |
| HCHON1000191 | 0.346899111 | 12702 | 20 |
| HCHON2000028 | 0.105609731 | 88009 | 24 |
| HCHON2000038 | 0.010602205 | 98996 | 1 |
| HCHON2000056 | 0.590768361 | 33092 | 92 |
| HCHON2000062 | 0.039977658 | 35045 | 7 |
| HCHON2000087 | 0.270837225 | 71643 | 19 |
| HCHON2000100 | 0.010602205 | 153476 | 1 |
| HCHON2000156 | 0.010602205 | 58975 | 1 |
| HCHON2000160 | 0.166457755 | 90503 | 37 |
| HCHON2000199 | 0.010602205 | 193073 | 1 |
| HCHON2000212 | 0.019495442 | 57915 | 5 |
| HCHON2000226 | 0.415523562 | 76543 | 41 |
| HCHON2000244 | 0.254716517 | 126260 | 33 |
| HCHON2000265 | 0.082815681 | 145896 | 9 |
| HCHON2000271 | 0.010602205 | 114194 | 1 |
| HCHON2000295 | 0.382534307 | 44066 | 23 |
| HCHON2000315 | 0.010602205 | 271872 | 1 |
| HCHON2000322 | 0.015259711 | 220834 | 3 |
| HCHON2000323 | 0.090453209 | 142866 | 12 |
| HCHON2000344 | 0.010602205 | 62319 | 1 |
| HCHON2000364 | 0.37689031 | 12853 | 19 |
| HCHON2000373 | 0.165324137 | 60351 | 23 |
| HCHON2000395 | 0.010602205 | 175810 | 1 |
| HCHON2000417 | 0.010602205 | 30763 | 1 |
| HCHON2000418 | 0.012600607 | 86945 | 2 |
| HCHON2000473 | 0.028625077 | 107435 | 10 |
| HCHON2000475 | 0.125879405 | 106653 | 17 |
| HCHON2000503 | 0.010602205 | 115620 | 1 |
| HCHON2000508 | 0.448707369 | 126548 | 48 |
| HCHON2000626 | 0.142540221 | 92209 | 23 |
| HCHON2000660 | 0.010602205 | 110230 | 1 |
| HCHON2000663 | 0.019641786 | 225426 | 5 |
| HCHON2000673 | 0.010602205 | 81829 | 1 |
| HCHON2000676 | 0.056199156 | 11960 | 9 |
| HCHON2000698 | 0.058457997 | 141006 | 20 |
| HCHON2000699 | 0.011999361 | 126073 | 2 |
| HCHON2000705 | 0.016815315 | 99834 | 2 |
| HCHON2000738 | 0.056273021 | 122890 | 13 |
| HCHON2000743 | 0.027443448 | 36390 | 10 |
| HCHON2000751 | 0.046931294 | 15705 | 12 |
| HCHON2000781 | 0.062218274 | 17625 | 20 |
| HCHON2000815 | 0.059998228 | 101772 | 11 |
| HCHON2000817 | 0.010602205 | 269196 | 1 |
| HCHON2000818 | 0.010602205 | 267907 | 1 |
| HCHON2000819 | 0.022869683 | 56616 | 3 |
| HCHON2000826 | 0.23411552 | 87016 | 57 |
| HCHON2000832 | 0.047360549 | 159261 | 10 |
| HCHON2000851 | 0.010602205 | 267889 | 1 |
| HCHON2000898 | 0.016443668 | 199625 | 2 |
| HCHON2000935 | 0.010602205 | 159966 | 1 |
| HCHON2000942 | 0.01477914 | 58469 | 2 |
| HCHON2000956 | 0.010602205 | 98638 | 1 |
| HCHON2000994 | 0.021204411 | 12791 | 2 |
| HCHON2001039 | 0.150161876 | 78146 | 60 |
| HCHON2001050 | 0.11560155 | 145954 | 8 |
| HCHON2001084 | 0.072972628 | 87760 | 12 |
| HCHON2001099 | 0.061433165 | 112187 | 9 |
| HCHON2001108 | 0.078470402 | 20531 | 5 |
| HCHON2001110 | 0.020310943 | 125628 | 2 |
| HCHON2001116 | 0.010602205 | 129287 | 1 |
| HCHON2001161 | 0.015561238 | 105445 | 4 |
| HCHON2001200 | 0.387403789 | 34429 | 51 |
| HCHON2001214 | 0.010602205 | 269459 | 1 |
| HCHON2001217 | 0.160978591 | 5378 | 50 |
| HCHON2001233 | 0.010602205 | 45213 | 1 |
| HCHON2001269 | 0.010602205 | 67140 | 1 |
| HCHON2001359 | 0.1968093 | 76309 | 30 |
| HCHON2001407 | 0.212188264 | 117940 | 34 |
| HCHON2001434 | 0.10968658 | 48012 | 37 |
| HCHON2001450 | 0.010602205 | 47009 | 1 |
| HCHON2001453 | 0.010602205 | 268556 | 1 |
| HCHON2001497 | 0.02236276 | 154626 | 2 |
| HCHON2001505 | 0.037286861 | 12745 | 11 |
| HCHON2001519 | 0.011697484 | 257017 | 2 |
| HCHON2001523 | 0.010602205 | 83523 | 1 |
| HCHON2001535 | 0.018169384 | 66915 | 5 |
| HCHON2001546 | 0.023125687 | 52241 | 2 |
| HCHON2001548 | 0.014640488 | 101326 | 2 |
| HCHON2001577 | 0.010602205 | 37624 | 1 |
| HCHON2001598 | 0.068959654 | 132427 | 8 |
| HCHON2001604 | 0.010602205 | 12950 | 1 |
| HCHON2001607 | 0.010602205 | 285606 | 1 |
| HCHON2001646 | 0.010602205 | 269204 | 1 |
| HCHON2001665 | 0.108581659 | 101140 | 27 |
| HCHON2001712 | 0.011999361 | 77424 | 2 |
| HCHON2001768 | 0.010602205 | 89904 | 1 |
| HCHON2001801 | 0.01653798 | 180355 | 2 |
| HCHON2001853 | 0.029917426 | 117972 | 7 |
| HCHON2002123 | 0.010602205 | 12763 | 1 |
| HCHON2002194 | 0.010602205 | 138024 | 1 |
| HCHON2002241 | 0.010602205 | 173487 | 1 |
| HCHON2002247 | 0.059683268 | 59169 | 15 |
| HCHON2002354 | 0.013188605 | 82745 | 3 |
| HCHON2002496 | 0.021572076 | 46490 | 3 |
| HCHON2002676 | 0.012277752 | 212131 | 2 |
| HCHON2002770 | 0.010602205 | 176302 | 1 |
| HCHON2002964 | 0.021204411 | 12872 | 2 |
| HCHON2002971 | 0.011999361 | 178400 | 2 |
| HCHON2003131 | 0.010602205 | 92613 | 1 |
| HCHON2003134 | 0.010602205 | 28465 | 1 |
| HCHON2003327 | 0.010602205 | 22149 | 1 |
| HCHON2003513 | 0.010602205 | 105982 | 1 |
| HCHON2003532 | 0.010602205 | 207938 | 1 |
| HCHON2003642 | 0.013517062 | 93307 | 2 |
| HCHON2003659 | 0.010602205 | 965 | 1 |
| HCHON2003676 | 0.010602205 | 243165 | 1 |
| HCHON2004002 | 0.213419707 | 130575 | 37 |
| HCHON2004007 | 0.010602205 | 135584 | 1 |
| HCHON2004279 | 0.010602205 | 136665 | 1 |
| HCHON2004359 | 0.010602205 | 104086 | 1 |
| HCHON2004531 | 0.010602205 | 36374 | 1 |
| HCHON2004776 | 0.010602205 | 127547 | 1 |
| HCHON2004858 | 0.012683717 | 116759 | 2 |
| HCHON2005048 | 0.010602205 | 111303 | 1 |
| HCHON2005118 | 0.010602205 | 172354 | 1 |
| HCHON2005166 | 0.013697701 | 146621 | 2 |
| HCHON2005802 | 0.010602205 | 217253 | 1 |
| HCHON2005921 | 0.040400707 | 154965 | 16 |
| HCHON2005987 | 0.016804525 | 176993 | 2 |
| HCHON2006250 | 0.107081819 | 27447 | 10 |
| HCHON2006459 | 0.015523928 | 158722 | 3 |
| HCHON2006714 | 0.010602205 | 151914 | 1 |
| HCHON2006722 | 0.01653798 | 126183 | 2 |
| HCHON2006770 | 0.059368571 | 66629 | 4 |
| HCHON2006786 | 0.012311665 | 52239 | 2 |
| HCHON2006841 | 0.010602205 | 138680 | 1 |
| HCHON2006871 | 0.010602205 | 195906 | 1 |
| HCHON2007046 | 0.046638241 | 71519 | 2 |
| HCHON2007482 | 0.010602205 | 200988 | 1 |
| HCHON2007650 | 0.027514824 | 76325 | 5 |
| HCHON2007674 | 0.010602205 | 14156 | 1 |
| HCHON2007774 | 0.010602205 | 103138 | 1 |
| HCHON2007881 | 0.012683717 | 184857 | 2 |
| HCHON2008112 | 0.012290482 | 184362 | 2 |
| HCHON2008239 | 0.010602205 | 163399 | 1 |
| HCHON2008444 | 0.012600607 | 130366 | 2 |
| HCHON2008582 | 0.010602205 | 162830 | 1 |
| HCHON2008672 | 0.010602205 | 13028 | 1 |
| HCHON2008856 | 0.016804525 | 206860 | 2 |
| HCHON2008871 | 0.010602205 | 150290 | 1 |
| HCHON2008989 | 0.010602205 | 110588 | 1 |
| HCHON2009191 | 0.010602205 | 169572 | 1 |
| HCHON2009384 | 0.010602205 | 143708 | 1 |
| HCHON2009749 | 0.010602205 | 75588 | 1 |
| HCHON2009766 | 0.010602205 | 145551 | 1 |
| HCHON2009795 | 0.010602205 | 13046 | 1 |
| HCHON2010074 | 0.010602205 | 30481 | 1 |
| HCHON2010543 | 0.018677918 | 134404 | 5 |
| HEART1000010 | 0.049042994 | 21506 | 5 |
| HEART1000074 | 0.220726718 | 60269 | 31 |
| HEART1000088 | 0.022464862 | 49457 | 2 |
| HEART1000099 | 0.01117818 | 45227 | 1 |
| HEART1000102 | 0.01117818 | 35888 | 1 |
| HEART1000118 | 0.015117807 | 43130 | 3 |
| HEART1000132 | 0.057689644 | 43128 | 5 |
| HEART1000139 | 0.033643042 | 69689 | 3 |
| HEART1000142 | 0.093988249 | 51283 | 7 |
| HEART1000149 | 0.178424584 | 81037 | 32 |
| HEART1000151 | 0.01117818 | 45223 | 1 |
| HEART1000173 | 0.063840356 | 70047 | 5 |
| HEART1000185 | 0.211938906 | 57208 | 23 |
| HEART2000024 | 0.01117818 | 228339 | 1 |
| HEART2000035 | 0.01117818 | 104735 | 1 |
| HEART2000099 | 0.01117818 | 260986 | 1 |
| HEART2000298 | 0.01117818 | 229589 | 1 |
| HEART2000306 | 0.012367425 | 46612 | 2 |
| HEART2000309 | 0.01117818 | 213852 | 1 |
| HEART2000411 | 0.079417809 | 138027 | 7 |
| HEART2000418 | 0.01117818 | 241174 | 1 |
| HEART2000444 | 0.01989553 | 172999 | 4 |
| HEART2000448 | 0.02235636 | 69697 | 2 |
| HEART2000492 | 0.01117818 | 235982 | 1 |
| HEART2000506 | 0.095544218 | 148473 | 14 |
| HEART2000520 | 0.116199863 | 171346 | 11 |
| HEART2000541 | 0.125233313 | 154993 | 12 |
| HEART2000568 | 0.01117818 | 235971 | 1 |
| HEART2000611 | 0.02235636 | 109962 | 2 |
| HEART2000959 | 0.014225539 | 163957 | 3 |
| HEART2001680 | 0.01117818 | 273158 | 1 |
| HEART2001756 | 0.02235636 | 193083 | 2 |
| HEART2001773 | 0.075126312 | 91738 | 8 |
| HEART2001931 | 0.045147519 | 177409 | 5 |
| HEART2002129 | 0.01117818 | 90745 | 1 |
| HEART2002184 | 0.01117818 | 193412 | 1 |
| HEART2002220 | 0.01117818 | 58213 | 1 |
| HEART2002432 | 0.01117818 | 253027 | 1 |
| HEART2002531 | 0.01117818 | 268691 | 1 |
| HEART2002598 | 0.01117818 | 53797 | 1 |
| HEART2002717 | 0.01117818 | 146320 | 1 |
| HEART2003027 | 0.01117818 | 41622 | 1 |
| HEART2003168 | 0.013036295 | 42732 | 2 |
| HEART2003432 | 0.01117818 | 201533 | 1 |
| HEART2003440 | 0.057239903 | 152416 | 2 |
| HEART2003781 | 0.01117818 | 156417 | 1 |
| HEART2003836 | 0.081444128 | 143072 | 8 |
| HEART2003852 | 0.043005124 | 138957 | 6 |
| HEART2004570 | 0.01117818 | 120195 | 1 |
| HEART2004764 | 0.013368737 | 113879 | 3 |
| HEART2004931 | 0.024640527 | 119883 | 3 |
| HEART2004940 | 0.01117818 | 188184 | 1 |
| HEART2004941 | 0.035276163 | 202388 | 5 |
| HEART2004980 | 0.01117818 | 246209 | 1 |
| HEART2005244 | 0.01117818 | 267169 | 1 |
| HEART2005310 | 0.024034014 | 95327 | 3 |
| HEART2005581 | 0.01117818 | 193308 | 1 |
| HEART2005610 | 0.01117818 | 53804 | 1 |
| HEART2006131 | 0.01117818 | 178056 | 1 |
| HEART2006195 | 0.017679339 | 142370 | 4 |
| HEART2006310 | 0.01117818 | 159388 | 1 |
| HEART2006334 | 0.01117818 | 167816 | 1 |
| HEART2006487 | 0.017232548 | 155920 | 2 |
| HEART2006521 | 0.070054992 | 124825 | 15 |
| HEART2006557 | 0.01117818 | 230159 | 1 |
| HEART2006787 | 0.01117818 | 180547 | 1 |
| HEART2006789 | 0.01117818 | 172754 | 1 |
| HEART2006909 | 0.01117818 | 167824 | 1 |
| HEART2007031 | 0.014745755 | 124197 | 3 |
| HEART2007231 | 0.034590572 | 158945 | 11 |
| HEART2007390 | 0.290288676 | 36444 | 21 |
| HEART2007443 | 0.01117818 | 206096 | 1 |
| HEART2007696 | 0.033639989 | 4604 | 5 |
| HEART2007767 | 0.01117818 | 131539 | 1 |
| HEART2008108 | 0.01117818 | 72050 | 1 |
| HEART2008111 | 0.01117818 | 44305 | 1 |
| HEART2008257 | 0.01117818 | 53827 | 1 |
| HEART2008364 | 0.075460802 | 181167 | 7 |
| HEART2008509 | 0.028397926 | 164043 | 3 |
| HEART2008994 | 0.01117818 | 133477 | 1 |
| HEART2009000 | 0.016211509 | 97696 | 3 |
| HEART2009599 | 0.01117818 | 283388 | 1 |
| HEART2009680 | 0.01117818 | 178151 | 1 |
| HEART2010391 | 0.01117818 | 212941 | 1 |
| HEART2010492 | 0.01117818 | 123242 | 1 |
| HEART2010495 | 0.022464862 | 198410 | 2 |
| HELAC2000158 | 0.147058824 | 243951 | 1 |
| HELAC2000301 | 0.147058824 | 243318 | 1 |
| HHDPC1000001 | 0.011798018 | 62665 | 1 |
| HHDPC1000065 | 0.018323554 | 56037 | 5 |
| HHDPC1000083 | 0.17118191 | 5301 | 27 |
| HHDPC1000114 | 0.230602388 | 76468 | 33 |
| HHDPC1000118 | 0.079948571 | 61435 | 19 |
| HHDPC1000163 | 0.133241281 | 72507 | 21 |
| HHDPC2000055 | 0.091632272 | 121305 | 14 |
| HHDPC2000070 | 0.017411258 | 117037 | 4 |
| HHDPC2000095 | 0.220123573 | 66433 | 33 |
| HHDPC2000102 | 0.035288814 | 147613 | 6 |
| HHDPC2000104 | 0.090570063 | 116696 | 11 |
| HHDPC2000115 | 0.064051116 | 122929 | 10 |
| HHDPC2000149 | 0.037498931 | 69887 | 9 |
| HHDPC2000258 | 0.017733793 | 181248 | 2 |
| HHDPC2000315 | 0.011798018 | 126680 | 1 |
| HHDPC2000455 | 0.304913305 | 83824 | 31 |
| HHDPC2000456 | 0.107919863 | 48562 | 17 |
| HHDPC2000462 | 0.014842899 | 157121 | 2 |
| HHDPC2000572 | 0.025592632 | 123565 | 7 |
| HHDPC2000656 | 0.019601597 | 22695 | 3 |
| HHDPC2000692 | 0.011798018 | 181235 | 1 |
| HHDPC2001337 | 0.011798018 | 232626 | 1 |
| HHDPC2001432 | 0.011798018 | 122668 | 1 |
| HHDPC2001896 | 0.017870401 | 158517 | 2 |
| HHDPC2002963 | 0.011798018 | 8549 | 1 |
| HHDPC2003049 | 0.011798018 | 13118 | 1 |
| HHDPC2003113 | 0.011798018 | 219934 | 1 |
| HHDPC2003381 | 0.048043034 | 153643 | 2 |
| HHDPC2003439 | 0.658805628 | 68709 | 109 |
| HHDPC2003472 | 0.012893296 | 39729 | 2 |
| HHDPC2003820 | 0.044469609 | 82943 | 9 |
| HHDPC2003983 | 0.601712808 | 49467 | 110 |
| HHDPC2004757 | 0.011798018 | 182510 | 1 |
| HHDPC2005185 | 0.011798018 | 128495 | 1 |
| HHDPC2005513 | 0.011798018 | 163283 | 1 |
| HHDPC2005742 | 0.013195173 | 34044 | 2 |
| HHDPC2005794 | 0.021064004 | 141825 | 6 |
| HHDPC2006460 | 0.017627732 | 49614 | 3 |
| HHDPC2006862 | 0.022386969 | 62883 | 4 |
| HHDPC2006927 | 0.011798018 | 74694 | 1 |
| HHDPC2007267 | 0.343194637 | 57162 | 52 |
| HHDPC2007302 | 0.011798018 | 264356 | 1 |
| HHDPC2007775 | 0.015505879 | 108163 | 3 |
| HHDPC2008123 | 0.013195173 | 14188 | 2 |
| HHDPC2008185 | 0.011798018 | 78478 | 1 |
| HHDPC2008279 | 0.011798018 | 201628 | 1 |
| HHDPC2008297 | 0.022400223 | 157308 | 2 |
| HHDPC2008414 | 0.011798018 | 194567 | 1 |
| HHDPC2008474 | 0.011798018 | 12860 | 1 |
| HHDPC2008816 | 0.013656132 | 154642 | 2 |
| HHDPC2008843 | 0.011798018 | 263265 | 1 |
| HHDPC2009114 | 0.011798018 | 66636 | 1 |
| HHDPC2009178 | 0.016507188 | 143140 | 3 |
| HHDPC2009208 | 0.011798018 | 12769 | 1 |
| HHDPC2009272 | 0.01367486 | 94691 | 2 |
| HHDPC2009528 | 0.025780516 | 124939 | 9 |
| HLUNG1000017 | 0.006145526 | 72627 | 1 |
| HLUNG1000024 | 0.006145526 | 78128 | 1 |
| HLUNG1000030 | 0.006145526 | 76039 | 1 |
| HLUNG1000034 | 0.006145526 | 70805 | 1 |
| HLUNG1000037 | 0.01906854 | 65539 | 5 |
| HLUNG1000041 | 0.042734607 | 49300 | 15 |
| HLUNG1000053 | 0.006145526 | 71892 | 1 |
| HLUNG1000055 | 0.309541219 | 74617 | 56 |
| HLUNG1000064 | 0.024606665 | 67427 | 6 |
| HLUNG1000076 | 0.199173756 | 72867 | 29 |
| HLUNG1000084 | 0.006145526 | 67966 | 1 |
| HLUNG1000091 | 0.011322534 | 72763 | 3 |
| HLUNG1000099 | 0.015524287 | 46123 | 7 |
| HLUNG1000104 | 0.006145526 | 59147 | 1 |
| HLUNG1000105 | 0.015854264 | 74322 | 2 |
| HLUNG1000110 | 0.007240804 | 76890 | 2 |
| HLUNG1000151 | 0.006145526 | 59012 | 1 |
| HLUNG1000169 | 0.006145526 | 67046 | 1 |
| HLUNG2000004 | 0.066243039 | 115888 | 11 |
| HLUNG2000014 | 0.006145526 | 180932 | 1 |
| HLUNG2000027 | 0.006145526 | 41556 | 1 |
| HLUNG2000063 | 0.067937545 | 63277 | 16 |
| HLUNG2000068 | 0.006145526 | 103991 | 1 |
| HLUNG2000116 | 0.006145526 | 78124 | 1 |
| HLUNG2000125 | 0.046965116 | 116845 | 3 |
| HLUNG2000130 | 0.006145526 | 262864 | 1 |
| HLUNG2000142 | 0.012291052 | 100905 | 2 |
| HLUNG2000174 | 0.006145526 | 185033 | 1 |
| HLUNG2000176 | 0.007542681 | 103313 | 2 |
| HLUNG2000217 | 0.006145526 | 98761 | 1 |
| HLUNG2000232 | 0.013295058 | 99956 | 5 |
| HLUNG2000255 | 0.006145526 | 167263 | 1 |
| HLUNG2000281 | 0.006145526 | 147023 | 1 |
| HLUNG2000314 | 0.068300052 | 58776 | 9 |
| HLUNG2000315 | 0.0109856 | 123552 | 3 |
| HLUNG2000340 | 0.006145526 | 215325 | 1 |
| HLUNG2000362 | 0.006145526 | 159246 | 1 |
| HLUNG2000363 | 0.006145526 | 147846 | 1 |
| HLUNG2000412 | 0.062576097 | 146816 | 21 |
| HLUNG2000431 | 0.006145526 | 198509 | 1 |
| HLUNG2000480 | 0.006145526 | 270444 | 1 |
| HLUNG2000489 | 0.006145526 | 168597 | 1 |
| HLUNG2000501 | 0.195322804 | 122488 | 23 |
| HLUNG2000549 | 0.006145526 | 35872 | 1 |
| HLUNG2000557 | 0.006145526 | 282138 | 1 |
| HLUNG2000570 | 0.006145526 | 129597 | 1 |
| HLUNG2000751 | 0.009697915 | 242649 | 3 |
| HLUNG2000761 | 0.006145526 | 178685 | 1 |
| HLUNG2000777 | 0.006145526 | 225200 | 1 |
| HLUNG2000846 | 0.037087907 | 29445 | 19 |
| HLUNG2000870 | 0.006145526 | 240667 | 1 |
| HLUNG2000884 | 0.078279812 | 100688 | 17 |
| HLUNG2000926 | 0.006145526 | 210476 | 1 |
| HLUNG2000950 | 0.013553035 | 68684 | 4 |
| HLUNG2000955 | 0.006145526 | 236406 | 1 |
| HLUNG2000970 | 0.006145526 | 114929 | 1 |
| HLUNG2001013 | 0.006145526 | 139663 | 1 |
| HLUNG2001126 | 0.006145526 | 171876 | 1 |
| HLUNG2001144 | 0.006145526 | 212936 | 1 |
| HLUNG2001146 | 0.031964516 | 36493 | 13 |
| HLUNG2001214 | 0.006145526 | 272875 | 1 |
| HLUNG2001439 | 0.189995764 | 110658 | 12 |
| HLUNG2001459 | 0.025566028 | 125304 | 4 |
| HLUNG2001507 | 0.033460848 | 148830 | 7 |
| HLUNG2001518 | 0.153231919 | 112210 | 57 |
| HLUNG2001633 | 0.020926599 | 162390 | 7 |
| HLUNG2001677 | 0.047685159 | 151366 | 4 |
| HLUNG2001712 | 0.050819864 | 158995 | 9 |
| HLUNG2001814 | 0.006145526 | 96912 | 1 |
| HLUNG2001996 | 0.00800364 | 140256 | 2 |
| HLUNG2002006 | 0.006145526 | 232850 | 1 |
| HLUNG2002050 | 0.006145526 | 277250 | 1 |
| HLUNG2002059 | 0.061168426 | 124953 | 14 |
| HLUNG2002085 | 0.006145526 | 132761 | 1 |
| HLUNG2002145 | 0.012058138 | 122238 | 2 |
| HLUNG2002295 | 0.006145526 | 124756 | 1 |
| HLUNG2002303 | 0.006145526 | 132758 | 1 |
| HLUNG2002334 | 0.018228262 | 117124 | 4 |
| HLUNG2002405 | 0.006145526 | 137055 | 1 |
| HLUNG2002429 | 0.006145526 | 36838 | 1 |
| HLUNG2002465 | 0.138538996 | 109565 | 24 |
| HLUNG2002562 | 0.006145526 | 172863 | 1 |
| HLUNG2002648 | 0.046357932 | 6932 | 12 |
| HLUNG2002659 | 0.006145526 | 82510 | 1 |
| HLUNG2002707 | 0.006145526 | 132794 | 1 |
| HLUNG2002811 | 0.068764045 | 128957 | 16 |
| HLUNG2002916 | 0.019421838 | 79803 | 4 |
| HLUNG2002942 | 0.006145526 | 66941 | 1 |
| HLUNG2002949 | 0.009060383 | 254290 | 2 |
| HLUNG2002958 | 0.006145526 | 118783 | 1 |
| HLUNG2002982 | 0.006145526 | 136347 | 1 |
| HLUNG2003003 | 0.020966941 | 136346 | 9 |
| HLUNG2003042 | 0.070389005 | 74489 | 12 |
| HLUNG2003049 | 0.006145526 | 115207 | 1 |
| HLUNG2003061 | 0.006145526 | 143985 | 1 |
| HLUNG2003162 | 0.006145526 | 164928 | 1 |
| HLUNG2003246 | 0.006145526 | 30639 | 1 |
| HLUNG2003306 | 0.013085839 | 82150 | 4 |
| HLUNG2003331 | 0.020238563 | 121772 | 3 |
| HLUNG2003335 | 0.006145526 | 56828 | 1 |
| HLUNG2003378 | 0.018097956 | 157593 | 5 |
| HLUNG2003497 | 0.007542681 | 104940 | 2 |
| HLUNG2003691 | 0.006145526 | 175642 | 1 |
| HLUNG2003710 | 0.006145526 | 158435 | 1 |
| HLUNG2003714 | 0.006145526 | 249799 | 1 |
| HLUNG2003716 | 0.007334771 | 131110 | 2 |
| HLUNG2003778 | 0.032482039 | 276412 | 4 |
| HLUNG2003833 | 0.006145526 | 145569 | 1 |
| HLUNG2003872 | 0.006145526 | 150308 | 1 |
| HLUNG2003918 | 0.006145526 | 150282 | 1 |
| HLUNG2003991 | 0.006145526 | 134026 | 1 |
| HLUNG2004014 | 0.006145526 | 256664 | 1 |
| HLUNG2004037 | 0.010975793 | 120070 | 3 |
| HLUNG2004067 | 0.015217137 | 155154 | 4 |
| HLUNG2004154 | 0.016112319 | 130330 | 4 |
| HLUNG2004159 | 0.092417776 | 82653 | 28 |
| HLUNG2004170 | 0.027192647 | 88725 | 10 |
| HLUNG2004217 | 0.006145526 | 216197 | 1 |
| HLUNG2004273 | 0.006145526 | 100851 | 1 |
| HLUNG2004388 | 0.037525551 | 67635 | 6 |
| HLUNG2004416 | 0.006145526 | 122402 | 1 |
| HLUNG2004521 | 0.006145526 | 167872 | 1 |
| HLUNG2004534 | 0.057763737 | 40889 | 5 |
| HLUNG2004684 | 0.049632352 | 128944 | 3 |
| HLUNG2004707 | 0.006145526 | 145388 | 1 |
| HLUNG2004774 | 0.010322461 | 122122 | 2 |
| HLUNG2004775 | 0.006145526 | 116347 | 1 |
| HLUNG2005012 | 0.006145526 | 257417 | 1 |
| HLUNG2005030 | 0.006145526 | 284372 | 1 |
| HLUNG2005045 | 0.006145526 | 265170 | 1 |
| HLUNG2005076 | 0.006145526 | 88164 | 1 |
| HLUNG2005133 | 0.023653453 | 188485 | 7 |
| HLUNG2005203 | 0.008022368 | 219697 | 2 |
| HLUNG2005230 | 0.073302178 | 152388 | 12 |
| HLUNG2005479 | 0.006145526 | 234756 | 1 |
| HLUNG2005524 | 0.006145526 | 249278 | 1 |
| HLUNG2005656 | 0.007334771 | 222721 | 2 |
| HLUNG2005738 | 0.009127485 | 145313 | 2 |
| HLUNG2005774 | 0.006145526 | 164685 | 1 |
| HLUNG2005813 | 0.006145526 | 182087 | 1 |
| HLUNG2005924 | 0.006145526 | 52917 | 1 |
| HLUNG2006026 | 0.006145526 | 270806 | 1 |
| HLUNG2006067 | 0.006145526 | 148774 | 1 |
| HLUNG2006094 | 0.006145526 | 132742 | 1 |
| HLUNG2006370 | 0.013688208 | 135498 | 3 |
| HLUNG2006397 | 0.006145526 | 75269 | 1 |
| HLUNG2006570 | 0.025925218 | 128800 | 7 |
| HLUNG2006582 | 0.042754388 | 125879 | 19 |
| HLUNG2006599 | 0.006145526 | 87557 | 1 |
| HLUNG2006614 | 0.006145526 | 117771 | 1 |
| HLUNG2006671 | 0.006145526 | 114640 | 1 |
| HLUNG2006781 | 0.006145526 | 130438 | 1 |
| HLUNG2006812 | 0.056664505 | 93210 | 16 |
| HLUNG2006843 | 0.016143548 | 145481 | 4 |
| HLUNG2006852 | 0.006145526 | 142367 | 1 |
| HLUNG2006935 | 0.006145526 | 122209 | 1 |
| HLUNG2007041 | 0.006145526 | 162624 | 1 |
| HLUNG2007163 | 0.012436936 | 22585 | 4 |
| HLUNG2007210 | 0.08151364 | 52666 | 5 |
| HLUNG2007219 | 0.007542681 | 217068 | 2 |
| HLUNG2007245 | 0.048232005 | 198625 | 3 |
| HLUNG2007433 | 0.006145526 | 160830 | 1 |
| HLUNG2007931 | 0.006145526 | 170288 | 1 |
| HLUNG2008066 | 0.006145526 | 251045 | 1 |
| HLUNG2008139 | 0.014619794 | 150094 | 7 |
| HLUNG2008153 | 0.024615669 | 106811 | 7 |
| HLUNG2008225 | 0.010155661 | 232783 | 3 |
| HLUNG2008235 | 0.006145526 | 117325 | 1 |
| HLUNG2008247 | 0.006145526 | 78365 | 1 |
| HLUNG2008333 | 0.008143927 | 172882 | 2 |
| HLUNG2008348 | 0.013186071 | 10845 | 5 |
| HLUNG2008384 | 0.006145526 | 171465 | 1 |
| HLUNG2008396 | 0.006145526 | 154567 | 1 |
| HLUNG2008439 | 0.006145526 | 128850 | 1 |
| HLUNG2008479 | 0.009266332 | 53154 | 2 |
| HLUNG2008480 | 0.006145526 | 172886 | 1 |
| HLUNG2008521 | 0.006145526 | 54814 | 1 |
| HLUNG2008637 | 0.099191534 | 130778 | 12 |
| HLUNG2008833 | 0.006145526 | 62087 | 1 |
| HLUNG2008875 | 0.006145526 | 144491 | 1 |
| HLUNG2008885 | 0.015521841 | 43100 | 5 |
| HLUNG2008958 | 0.006145526 | 39490 | 1 |
| HLUNG2008959 | 0.006145526 | 157210 | 1 |
| HLUNG2009067 | 0.00800364 | 86169 | 2 |
| HLUNG2009215 | 0.01208765 | 100215 | 2 |
| HLUNG2009225 | 0.111100879 | 41575 | 42 |
| HLUNG2009253 | 0.006145526 | 154964 | 1 |
| HLUNG2009303 | 0.006145526 | 131693 | 1 |
| HLUNG2009413 | 0.006145526 | 106841 | 1 |
| HLUNG2009729 | 0.006145526 | 194036 | 1 |
| HLUNG2010181 | 0.006145526 | 166438 | 1 |
| HLUNG2010464 | 0.006145526 | 164109 | 1 |
| HLUNG2010562 | 0.006145526 | 178578 | 1 |
| HLUNG2010845 | 0.006145526 | 227112 | 1 |
| HLUNG2011041 | 0.006145526 | 246578 | 1 |
| HLUNG2011298 | 0.017432208 | 130706 | 2 |
| HLUNG2011461 | 0.007821073 | 189369 | 2 |
| HLUNG2011833 | 0.04081959 | 102116 | 2 |
| HLUNG2012049 | 0.006145526 | 274426 | 1 |
| HLUNG2012287 | 0.006145526 | 142907 | 1 |
| HLUNG2012315 | 0.006145526 | 220327 | 1 |
| HLUNG2012546 | 0.006145526 | 4680 | 1 |
| HLUNG2012600 | 0.006145526 | 284241 | 1 |
| HLUNG2012727 | 0.006145526 | 118456 | 1 |
| HLUNG2013097 | 0.006145526 | 3394 | 1 |
| HLUNG2013204 | 0.006145526 | 240482 | 1 |
| HLUNG2013304 | 0.006145526 | 178511 | 1 |
| HLUNG2013350 | 0.006145526 | 184878 | 1 |
| HLUNG2013622 | 0.006145526 | 176158 | 1 |
| HLUNG2013643 | 0.006145526 | 187907 | 1 |
| HLUNG2013784 | 0.047951546 | 62356 | 2 |
| HLUNG2013851 | 0.006145526 | 160035 | 1 |
| HLUNG2014262 | 0.006145526 | 203114 | 1 |
| HLUNG2014288 | 0.006145526 | 184962 | 1 |
| HLUNG2014449 | 0.006145526 | 188574 | 1 |
| HLUNG2014821 | 0.009899209 | 182831 | 3 |
| HLUNG2015184 | 0.012796353 | 119676 | 5 |
| HLUNG2015418 | 0.008022368 | 98339 | 2 |
| HLUNG2015548 | 0.006145526 | 8040 | 1 |
| HLUNG2015578 | 0.006145526 | 217563 | 1 |
| HLUNG2015617 | 0.006145526 | 170170 | 1 |
| HLUNG2016022 | 0.006145526 | 243719 | 1 |
| HLUNG2016862 | 0.006145526 | 248898 | 1 |
| HLUNG2017262 | 0.105658876 | 82144 | 50 |
| HLUNG2017286 | 0.006145526 | 193371 | 1 |
| HLUNG2017307 | 0.006145526 | 202242 | 1 |
| HLUNG2017350 | 0.006145526 | 268615 | 1 |
| HLUNG2017546 | 0.047478784 | 200901 | 3 |
| HLUNG2017806 | 0.006145526 | 169566 | 1 |
| HLUNG2018029 | 0.006145526 | 150492 | 1 |
| HLUNG2018282 | 0.006145526 | 277543 | 1 |
| HLUNG2018626 | 0.006145526 | 206788 | 1 |
| HLUNG2019058 | 0.006145526 | 221400 | 1 |
| HSYRA1000062 | 0.39118207 | 44116 | 62 |
| HSYRA1000119 | 0.3644211 | 78027 | 67 |
| HSYRA1000131 | 0.012523482 | 87609 | 1 |
| HSYRA1000137 | 0.044959562 | 43985 | 11 |
| HSYRA1000148 | 0.02540901 | 73950 | 4 |
| HSYRA1000152 | 0.140480179 | 48229 | 31 |
| HSYRA1000168 | 0.015505441 | 47014 | 2 |
| HSYRA1000178 | 0.066823107 | 18635 | 22 |
| HSYRA2000044 | 0.013920637 | 108021 | 2 |
| HSYRA2000135 | 0.042364748 | 118875 | 6 |
| HSYRA2000137 | 0.073922883 | 96221 | 11 |
| HSYRA2000159 | 1.389739123 | 9434 | 373 |
| HSYRA2000190 | 0.070547807 | 4816 | 12 |
| HSYRA2000221 | 0.01770049 | 89827 | 3 |
| HSYRA2000224 | 0.093183687 | 119952 | 18 |
| HSYRA2000232 | 0.22660084 | 33605 | 58 |
| HSYRA2000237 | 0.012523482 | 128212 | 1 |
| HSYRA2000248 | 0.246532535 | 78679 | 51 |
| HSYRA2000253 | 0.019832899 | 12156 | 3 |
| HSYRA2000255 | 0.373425306 | 60161 | 30 |
| HSYRA2000332 | 0.214826099 | 37573 | 17 |
| HSYRA2000347 | 0.234037348 | 89493 | 75 |
| HSYRA2000371 | 0.012523482 | 12968 | 1 |
| HSYRA2000424 | 0.014400323 | 105492 | 2 |
| HSYRA2000507 | 0.01361876 | 62084 | 2 |
| HSYRA2000510 | 0.012523482 | 240891 | 1 |
| HSYRA2000589 | 0.133555816 | 79239 | 29 |
| HSYRA2000605 | 0.028004799 | 135640 | 4 |
| HSYRA2000629 | 0.050203248 | 141543 | 7 |
| HSYRA2000640 | 0.196682364 | 29086 | 51 |
| HSYRA2000706 | 0.013712726 | 175272 | 2 |
| HSYRA2000743 | 0.216513216 | 79998 | 83 |
| HSYRA2000760 | 0.012523482 | 116092 | 1 |
| HSYRA2000787 | 0.05387047 | 7699 | 12 |
| HSYRA2000792 | 0.069187409 | 17868 | 3 |
| HSYRA2000828 | 0.11761207 | 120053 | 21 |
| HSYRA2000832 | 0.018669008 | 171140 | 2 |
| HSYRA2000925 | 0.756199735 | 94955 | 25 |
| HSYRA2000927 | 0.012523482 | 145681 | 1 |
| HSYRA2001003 | 0.019517904 | 107400 | 3 |
| HSYRA2001103 | 0.197559673 | 153858 | 17 |
| HSYRA2001105 | 0.060814345 | 98214 | 19 |
| HSYRA2001138 | 0.657241129 | 12270 | 103 |
| HSYRA2001142 | 0.012523482 | 262427 | 1 |
| HSYRA2001153 | 0.01832866 | 182301 | 2 |
| HSYRA2001225 | 0.226655906 | 146100 | 20 |
| HSYRA2001232 | 0.043312461 | 77421 | 5 |
| HSYRA2001255 | 0.012523482 | 283297 | 1 |
| HSYRA2001332 | 0.012523482 | 13018 | 1 |
| HSYRA2001353 | 0.033022593 | 183425 | 5 |
| HSYRA2001396 | 0.110290147 | 51644 | 21 |
| HSYRA2001420 | 0.030072353 | 128094 | 5 |
| HSYRA2001476 | 0.347074026 | 12734 | 58 |
| HSYRA2001484 | 0.018565048 | 223669 | 2 |
| HSYRA2001552 | 0.012523482 | 57036 | 1 |
| HSYRA2001567 | 0.074766449 | 134837 | 16 |
| HSYRA2001574 | 0.222687322 | 104873 | 21 |
| HSYRA2001580 | 0.030571812 | 184490 | 5 |
| HSYRA2001595 | 0.294133599 | 58519 | 49 |
| HSYRA2001615 | 0.080792807 | 111771 | 25 |
| HSYRA2001621 | 0.913125636 | 8427 | 57 |
| HSYRA2001631 | 0.028735737 | 18844 | 6 |
| HSYRA2001638 | 0.074083011 | 100148 | 16 |
| HSYRA2001983 | 0.034716479 | 45554 | 7 |
| HSYRA2002103 | 0.012523482 | 209450 | 1 |
| HSYRA2002590 | 0.012523482 | 221431 | 1 |
| HSYRA2002741 | 0.023810163 | 186938 | 2 |
| HSYRA2003168 | 0.012523482 | 12852 | 1 |
| HSYRA2004550 | 0.012523482 | 225056 | 1 |
| HSYRA2004858 | 0.012523482 | 202723 | 1 |
| HSYRA2005456 | 0.012523482 | 132446 | 1 |
| HSYRA2005496 | 0.012523482 | 91848 | 1 |
| HSYRA2005628 | 0.024983801 | 108705 | 3 |
| HSYRA2005970 | 0.012523482 | 212951 | 1 |
| HSYRA2006049 | 0.024745432 | 209870 | 2 |
| HSYRA2006873 | 0.012523482 | 135852 | 1 |
| HSYRA2007241 | 0.087372584 | 113081 | 10 |
| HSYRA2007338 | 0.012523482 | 184145 | 1 |
| HSYRA2007650 | 0.035226902 | 144430 | 6 |
| HSYRA2007667 | 0.018736591 | 60166 | 2 |
| HSYRA2008154 | 0.012523482 | 260439 | 1 |
| HSYRA2008376 | 0.107159275 | 79132 | 20 |
| HSYRA2008714 | 0.012523482 | 109472 | 1 |
| HSYRA2009075 | 0.139291562 | 132443 | 11 |
| HSYRA2009102 | 0.012523482 | 101249 | 1 |
| IMR321000102 | 0.122740821 | 55164 | 35 |
| IMR321000155 | 0.005912262 | 17127 | 1 |
| IMR321000158 | 0.220123573 | 66433 | 33 |
| IMR321000161 | 0.007309417 | 58575 | 2 |
| IMR321000165 | 0.005912262 | 281603 | 1 |
| IMR321000193 | 0.038175412 | 27719 | 6 |
| IMR321000207 | 0.011984645 | 62926 | 2 |
| IMR321000210 | 0.165308284 | 33089 | 32 |
| IMR321000219 | 0.596981765 | 61702 | 163 |
| IMR321000230 | 0.005912262 | 48009 | 1 |
| IMR321000242 | 0.029795622 | 51438 | 10 |
| IMR321000253 | 0.164511759 | 49580 | 16 |
| IMR321000266 | 0.059209506 | 54334 | 16 |
| IMR322000010 | 0.017699155 | 204132 | 2 |
| IMR322000072 | 0.208825484 | 101999 | 18 |
| IMR322000107 | 0.005912262 | 271654 | 1 |
| IMR322000121 | 0.150306421 | 120136 | 21 |
| IMR322000127 | 0.10816545 | 104430 | 34 |
| IMR322000144 | 0.102727316 | 142314 | 11 |
| IMR322000152 | 0.020683373 | 69863 | 3 |
| IMR322000164 | 0.005912262 | 269073 | 1 |
| IMR322000178 | 0.090092024 | 51443 | 6 |
| IMR322000223 | 0.246099782 | 72126 | 32 |
| IMR322000243 | 0.195299125 | 9027 | 34 |
| IMR322000302 | 0.028243819 | 145027 | 5 |
| IMR322000316 | 0.403797085 | 70462 | 140 |
| IMR322000374 | 0.030418644 | 114705 | 4 |
| IMR322000379 | 0.005912262 | 270234 | 1 |
| IMR322000383 | 0.005912262 | 92544 | 1 |
| IMR322000418 | 0.005912262 | 269496 | 1 |
| IMR322000518 | 0.054556436 | 120334 | 12 |
| IMR322000555 | 0.028085634 | 122224 | 8 |
| IMR322000604 | 0.007587809 | 95204 | 2 |
| IMR322000609 | 0.007587809 | 236913 | 2 |
| IMR322000656 | 0.005912262 | 83674 | 1 |
| IMR322000672 | 0.005912262 | 274780 | 1 |
| IMR322000730 | 1.222498247 | 29663 | 86 |
| IMR322000742 | 0.10118721 | 131842 | 13 |
| IMR322000775 | 0.040412625 | 71255 | 10 |
| IMR322000791 | 0.032295588 | 164874 | 4 |
| IMR322000811 | 0.011854386 | 56634 | 2 |
| IMR322000819 | 0.039310974 | 181455 | 5 |
| IMR322000838 | 0.007770376 | 153601 | 2 |
| IMR322000859 | 0.008290751 | 88163 | 3 |
| IMR322000863 | 0.024838293 | 36361 | 5 |
| IMR322000917 | 0.014184766 | 166215 | 4 |
| IMR322000918 | 0.057162195 | 124283 | 12 |
| IMR322000919 | 0.005912262 | 58220 | 1 |
| IMR322000935 | 0.111914818 | 130132 | 20 |
| IMR322000953 | 0.005912262 | 188928 | 1 |
| IMR322000973 | 0.207800666 | 168676 | 13 |
| IMR322000984 | 0.005912262 | 142377 | 1 |
| IMR322001018 | 0.005912262 | 269611 | 1 |
| IMR322001049 | 0.028924778 | 35412 | 7 |
| IMR322001167 | 0.005912262 | 102010 | 1 |
| IMR322001185 | 0.011953828 | 235571 | 2 |
| IMR322001218 | 0.149668632 | 174073 | 11 |
| IMR322001245 | 0.005912262 | 178089 | 1 |
| IMR322001317 | 0.212184704 | 99451 | 32 |
| IMR322001318 | 0.007600539 | 176795 | 2 |
| IMR322001322 | 0.007600539 | 260831 | 2 |
| IMR322001332 | 0.062933101 | 149208 | 14 |
| IMR322001380 | 0.084524886 | 101282 | 25 |
| IMR322001435 | 0.178350114 | 42954 | 43 |
| IMR322001438 | 0.020163157 | 31743 | 3 |
| IMR322001452 | 0.005912262 | 266043 | 1 |
| IMR322001491 | 0.017376208 | 145174 | 2 |
| IMR322001534 | 0.020278088 | 171468 | 4 |
| IMR322001541 | 0.099046094 | 141041 | 20 |
| IMR322001584 | 0.134229588 | 117797 | 21 |
| IMR322001600 | 1.040602874 | 60974 | 67 |
| IMR322001665 | 0.005912262 | 160858 | 1 |
| IMR322001670 | 0.036727557 | 165836 | 12 |
| IMR322001710 | 0.086393499 | 60954 | 22 |
| IMR322001724 | 0.005912262 | 275502 | 1 |
| IMR322001879 | 0.017385434 | 128107 | 6 |
| IMR322002035 | 0.009263356 | 142001 | 3 |
| IMR322002110 | 0.005912262 | 219548 | 1 |
| IMR322002470 | 0.005912262 | 241286 | 1 |
| IMR322002660 | 0.012371582 | 138479 | 4 |
| IMR322002760 | 0.031464993 | 141357 | 7 |
| IMR322002993 | 0.010764599 | 176882 | 4 |
| IMR322003675 | 0.005912262 | 201098 | 1 |
| IMR322004768 | 0.005912262 | 258479 | 1 |
| IMR322005293 | 0.005912262 | 116468 | 1 |
| IMR322005833 | 0.005912262 | 239102 | 1 |
| IMR322006012 | 0.005912262 | 244876 | 1 |
| IMR322006222 | 0.005912262 | 218415 | 1 |
| IMR322006495 | 0.005912262 | 158814 | 1 |
| IMR322006520 | 0.005912262 | 49736 | 1 |
| IMR322006886 | 0.005912262 | 31507 | 1 |
| IMR322006947 | 0.018134213 | 227599 | 2 |
| IMR322007078 | 0.005912262 | 240831 | 1 |
| IMR322007225 | 0.005912262 | 275698 | 1 |
| IMR322007704 | 0.005912262 | 126059 | 1 |
| IMR322008651 | 0.005912262 | 161927 | 1 |
| IMR322009710 | 0.007101507 | 169728 | 2 |
| IMR322009807 | 0.005912262 | 209191 | 1 |
| IMR322010295 | 0.005912262 | 227571 | 1 |
| IMR322010953 | 0.011824524 | 220657 | 2 |
| IMR322011659 | 0.039310974 | 181455 | 5 |
| IMR322011689 | 0.005912262 | 271209 | 1 |
| IMR322012314 | 0.005912262 | 177940 | 1 |
| IMR322012529 | 0.035581801 | 120080 | 7 |
| IMR322013053 | 0.005912262 | 278083 | 1 |
| IMR322013396 | 0.005912262 | 136777 | 1 |
| IMR322013731 | 0.005912262 | 204384 | 1 |
| IMR322013805 | 0.012403885 | 118755 | 5 |
| IMR322015523 | 0.005912262 | 169120 | 1 |
| IMR322016146 | 0.005912262 | 65914 | 1 |
| IMR322017049 | 0.005912262 | 168010 | 1 |
| IMR322018117 | 0.005912262 | 63289 | 1 |
| IMR322018192 | 0.005912262 | 2075 | 1 |
| IMR322019070 | 0.062625801 | 98019 | 19 |
| JCMLC2000273 | 0.054217819 | 130058 | 4 |
| KIDNE1000008 | 0.082976718 | 63106 | 9 |
| KIDNE1000012 | 0.005841463 | 60829 | 1 |
| KIDNE1000015 | 0.128512179 | 59968 | 22 |
| KIDNE1000028 | 0.005841463 | 51288 | 1 |
| KIDNE1000036 | 0.007839864 | 55820 | 2 |
| KIDNE1000050 | 0.67004871 | 54853 | 182 |
| KIDNE1000064 | 0.221975583 | 71795 | 38 |
| KIDNE1000104 | 0.01010341 | 46834 | 4 |
| KIDNE1000121 | 0.005841463 | 51641 | 1 |
| KIDNE1000143 | 0.251975035 | 59474 | 28 |
| KIDNE1000145 | 0.06059032 | 58772 | 18 |
| KIDNE1000152 | 0.005841463 | 17567 | 1 |
| KIDNE2000026 | 0.005841463 | 195182 | 1 |
| KIDNE2000041 | 0.005841463 | 97572 | 1 |
| KIDNE2000046 | 0.005841463 | 86608 | 1 |
| KIDNE2000051 | 0.005841463 | 124342 | 1 |
| KIDNE2000070 | 0.017524388 | 121155 | 3 |
| KIDNE2000085 | 0.162944329 | 113039 | 28 |
| KIDNE2000167 | 0.06425609 | 71798 | 11 |
| KIDNE2000174 | 0.005841463 | 257161 | 1 |
| KIDNE2000192 | 0.005841463 | 157877 | 1 |
| KIDNE2000227 | 0.005841463 | 150642 | 1 |
| KIDNE2000244 | 0.005841463 | 104614 | 1 |
| KIDNE2000245 | 0.005841463 | 97983 | 1 |
| KIDNE2000252 | 0.196056856 | 106109 | 53 |
| KIDNE2000266 | 0.039794587 | 79211 | 15 |
| KIDNE2000287 | 0.005841463 | 260665 | 1 |
| KIDNE2000315 | 0.024536653 | 128325 | 4 |
| KIDNE2000317 | 0.011777238 | 218754 | 2 |
| KIDNE2000328 | 0.005841463 | 65974 | 1 |
| KIDNE2000330 | 0.049079159 | 141919 | 4 |
| KIDNE2000335 | 0.005841463 | 231117 | 1 |
| KIDNE2000349 | 0.264932664 | 136134 | 21 |
| KIDNE2000375 | 0.005841463 | 103964 | 1 |
| KIDNE2000383 | 0.034022867 | 63503 | 11 |
| KIDNE2000394 | 0.011682925 | 141243 | 2 |
| KIDNE2000403 | 0.202819655 | 84424 | 25 |
| KIDNE2000422 | 0.00751701 | 249935 | 2 |
| KIDNE2000493 | 0.005841463 | 180462 | 1 |
| KIDNE2000497 | 0.05343476 | 225318 | 6 |
| KIDNE2000510 | 0.005841463 | 237100 | 1 |
| KIDNE2000513 | 0.005841463 | 97569 | 1 |
| KIDNE2000517 | 0.106986654 | 162158 | 19 |
| KIDNE2000519 | 0.017524388 | 126722 | 3 |
| KIDNE2000543 | 0.01940123 | 167445 | 4 |
| KIDNE2000555 | 0.005841463 | 212748 | 1 |
| KIDNE2000574 | 0.223844312 | 89428 | 32 |
| KIDNE2000624 | 0.005841463 | 112536 | 1 |
| KIDNE2000665 | 0.086555664 | 55127 | 11 |
| KIDNE2000678 | 0.005841463 | 93275 | 1 |
| KIDNE2000717 | 0.005841463 | 265465 | 1 |
| KIDNE2000721 | 0.007922975 | 114165 | 2 |
| KIDNE2000722 | 0.005841463 | 261395 | 1 |
| KIDNE2000777 | 0.087805408 | 138411 | 10 |
| KIDNE2000801 | 0.005841463 | 151482 | 1 |
| KIDNE2000832 | 0.053769404 | 112555 | 4 |
| KIDNE2000833 | 0.023258906 | 191590 | 3 |
| KIDNE2000846 | 0.011682925 | 143372 | 2 |
| KIDNE2000867 | 0.005841463 | 165319 | 1 |
| KIDNE2000909 | 0.081614836 | 120033 | 13 |
| KIDNE2000947 | 0.066295682 | 117622 | 17 |
| KIDNE2001117 | 0.00751701 | 208576 | 2 |
| KIDNE2001140 | 0.005841463 | 102580 | 1 |
| KIDNE2001162 | 0.358985708 | 38426 | 50 |
| KIDNE2001269 | 0.005841463 | 247228 | 1 |
| KIDNE2001361 | 0.007238618 | 113213 | 2 |
| KIDNE2001373 | 0.023517396 | 169434 | 3 |
| KIDNE2001467 | 0.642849001 | 82298 | 141 |
| KIDNE2001636 | 0.035458216 | 164793 | 9 |
| KIDNE2001713 | 0.0108138 | 159342 | 3 |
| KIDNE2001802 | 0.005841463 | 195821 | 1 |
| KIDNE2001840 | 0.005841463 | 44088 | 1 |
| KIDNE2001847 | 0.046041714 | 167204 | 4 |
| KIDNE2001857 | 0.005841463 | 59401 | 1 |
| KIDNE2001873 | 0.005841463 | 93271 | 1 |
| KIDNE2001897 | 0.005841463 | 136978 | 1 |
| KIDNE2001931 | 0.005841463 | 3831 | 1 |
| KIDNE2001979 | 0.028439911 | 103544 | 5 |
| KIDNE2002015 | 0.005841463 | 9882 | 1 |
| KIDNE2002162 | 0.005841463 | 147142 | 1 |
| KIDNE2002168 | 0.005841463 | 57156 | 1 |
| KIDNE2002191 | 0.0235069 | 528 | 9 |
| KIDNE2002198 | 0.005841463 | 154733 | 1 |
| KIDNE2002252 | 0.076863312 | 110087 | 22 |
| KIDNE2002262 | 0.005841463 | 144824 | 1 |
| KIDNE2002265 | 0.092128348 | 151505 | 13 |
| KIDNE2002438 | 0.008936959 | 82164 | 2 |
| KIDNE2002483 | 0.005841463 | 177668 | 1 |
| KIDNE2002725 | 0.016285294 | 139242 | 6 |
| KIDNE2002768 | 0.005841463 | 241326 | 1 |
| KIDNE2002795 | 0.005841463 | 76723 | 1 |
| KIDNE2002798 | 0.013845103 | 96858 | 3 |
| KIDNE2002839 | 0.022824821 | 65999 | 3 |
| KIDNE2002845 | 0.005841463 | 280387 | 1 |
| KIDNE2002872 | 0.29304986 | 181809 | 10 |
| KIDNE2002882 | 0.020365791 | 98344 | 2 |
| KIDNE2002883 | 0.073875916 | 121295 | 13 |
| KIDNE2002980 | 0.05316418 | 150533 | 3 |
| KIDNE2002991 | 0.005841463 | 237472 | 1 |
| KIDNE2003185 | 0.005841463 | 237586 | 1 |
| KIDNE2003188 | 0.005841463 | 59402 | 1 |
| KIDNE2003233 | 0.007030707 | 145793 | 2 |
| KIDNE2003305 | 0.199074898 | 136785 | 25 |
| KIDNE2003316 | 0.022146317 | 150501 | 4 |
| KIDNE2003335 | 0.005841463 | 241449 | 1 |
| KIDNE2003357 | 0.011332196 | 163340 | 3 |
| KIDNE2003373 | 0.0108138 | 109563 | 3 |
| KIDNE2003377 | 0.005841463 | 56207 | 1 |
| KIDNE2003507 | 0.049489618 | 170414 | 4 |
| KIDNE2003752 | 0.005841463 | 247329 | 1 |
| KIDNE2003764 | 0.015554722 | 99361 | 5 |
| KIDNE2003837 | 0.005841463 | 230814 | 1 |
| KIDNE2003941 | 0.332198807 | 88220 | 26 |
| KIDNE2003994 | 0.020779955 | 160572 | 3 |
| KIDNE2004034 | 0.005841463 | 164706 | 1 |
| KIDNE2004054 | 0.012835885 | 91568 | 3 |
| KIDNE2004084 | 0.005841463 | 155885 | 1 |
| KIDNE2004095 | 0.005841463 | 111437 | 1 |
| KIDNE2004115 | 0.007030707 | 94365 | 2 |
| KIDNE2004233 | 0.005841463 | 252100 | 1 |
| KIDNE2004262 | 0.005841463 | 147843 | 1 |
| KIDNE2004279 | 0.005841463 | 218219 | 1 |
| KIDNE2004294 | 0.005841463 | 164772 | 1 |
| KIDNE2004295 | 0.026245245 | 217115 | 5 |
| KIDNE2004305 | 0.008936959 | 16862 | 2 |
| KIDNE2004344 | 0.007238618 | 58414 | 2 |
| KIDNE2004394 | 0.005841463 | 246820 | 1 |
| KIDNE2004411 | 0.019662533 | 132354 | 5 |
| KIDNE2004475 | 0.065093671 | 157276 | 17 |
| KIDNE2004519 | 0.005841463 | 137793 | 1 |
| KIDNE2004520 | 0.005841463 | 246543 | 1 |
| KIDNE2004531 | 0.007238618 | 102911 | 2 |
| KIDNE2004534 | 0.044889885 | 141678 | 9 |
| KIDNE2004562 | 0.005841463 | 246808 | 1 |
| KIDNE2004579 | 0.005841463 | 255164 | 1 |
| KIDNE2004626 | 0.013000357 | 79706 | 3 |
| KIDNE2004681 | 0.005841463 | 238002 | 1 |
| KIDNE2004828 | 0.023365851 | 170845 | 4 |
| KIDNE2004846 | 0.025403278 | 34738 | 2 |
| KIDNE2004864 | 0.017161365 | 106686 | 5 |
| KIDNE2004879 | 0.005841463 | 89625 | 1 |
| KIDNE2004981 | 0.005841463 | 224981 | 1 |
| KIDNE2004985 | 0.005841463 | 224983 | 1 |
| KIDNE2004999 | 0.005841463 | 230960 | 1 |
| KIDNE2005038 | 0.005841463 | 238153 | 1 |
| KIDNE2005042 | 0.020811331 | 129777 | 7 |
| KIDNE2005062 | 0.005841463 | 224984 | 1 |
| KIDNE2005266 | 0.005841463 | 157721 | 1 |
| KIDNE2005296 | 0.005841463 | 183236 | 1 |
| KIDNE2005321 | 0.016957128 | 149152 | 4 |
| KIDNE2005327 | 0.029207314 | 194363 | 5 |
| KIDNE2005336 | 0.010018398 | 186730 | 2 |
| KIDNE2005400 | 0.005841463 | 244905 | 1 |
| KIDNE2005424 | 0.005841463 | 84682 | 1 |
| KIDNE2005477 | 0.005841463 | 166982 | 1 |
| KIDNE2005526 | 0.005841463 | 136844 | 1 |
| KIDNE2005543 | 0.009409037 | 39520 | 3 |
| KIDNE2005603 | 0.005841463 | 81671 | 1 |
| KIDNE2005629 | 0.023365851 | 116682 | 4 |
| KIDNE2005676 | 0.119099198 | 43143 | 45 |
| KIDNE2005781 | 0.005841463 | 36705 | 1 |
| KIDNE2005798 | 0.005841463 | 218140 | 1 |
| KIDNE2005854 | 0.005841463 | 138208 | 1 |
| KIDNE2005908 | 0.005841463 | 72328 | 1 |
| KIDNE2005937 | 0.030916774 | 133122 | 6 |
| KIDNE2006009 | 0.045448317 | 154557 | 10 |
| KIDNE2006014 | 0.056429638 | 149434 | 15 |
| KIDNE2006030 | 0.07851576 | 34026 | 25 |
| KIDNE2006039 | 0.010614434 | 124421 | 3 |
| KIDNE2006053 | 0.011682925 | 144826 | 2 |
| KIDNE2006062 | 0.059610867 | 165627 | 5 |
| KIDNE2006092 | 0.005841463 | 228204 | 1 |
| KIDNE2006145 | 0.005841463 | 258040 | 1 |
| KIDNE2006149 | 0.005841463 | 90614 | 1 |
| KIDNE2006210 | 0.023904876 | 178787 | 3 |
| KIDNE2006248 | 0.538918881 | 20243 | 49 |
| KIDNE2006299 | 0.005841463 | 109245 | 1 |
| KIDNE2006334 | 0.005841463 | 235919 | 1 |
| KIDNE2006353 | 0.042844954 | 118025 | 23 |
| KIDNE2006376 | 0.005841463 | 226494 | 1 |
| KIDNE2006378 | 0.005841463 | 162293 | 1 |
| KIDNE2006465 | 0.054150435 | 136539 | 21 |
| KJDNE2006580 | 0.023891517 | 88235 | 10 |
| KIDNE2006652 | 0.01940123 | 167445 | 4 |
| KIDNE2006687 | 0.005841463 | 95104 | 1 |
| KIDNE2006733 | 0.005841463 | 171215 | 1 |
| KIDNE2006760 | 0.024838834 | 152670 | 6 |
| KIDNE2006775 | 0.091455179 | 114362 | 13 |
| KIDNE2006811 | 0.051490468 | 101052 | 7 |
| KIDNE2006820 | 0.005841463 | 52561 | 1 |
| KIDNE2006880 | 0.052190513 | 81038 | 6 |
| KIDNE2007005 | 0.00875632 | 182657 | 2 |
| KIDNE2007040 | 0.007839864 | 219935 | 2 |
| KIDNE2007077 | 0.005841463 | 178790 | 1 |
| KIDNE2007186 | 0.00875632 | 141775 | 2 |
| KIDNE2007222 | 0.005841463 | 183153 | 1 |
| KIDNE2007328 | 0.189383507 | 101133 | 45 |
| KIDNE2007352 | 0.40306683 | 59267 | 83 |
| KIDNE2007356 | 0.011783586 | 252070 | 2 |
| KIDNE2007422 | 0.005841463 | 153769 | 1 |
| KIDNE2007569 | 0.011682925 | 152303 | 2 |
| KIDNE2007810 | 0.017524388 | 164707 | 3 |
| KIDNE2007811 | 0.019557654 | 170075 | 4 |
| KIDNE2007910 | 0.018488773 | 147451 | 4 |
| KIDNE2007944 | 0.016668661 | 132220 | 2 |
| KIDNE2007954 | 0.079484867 | 8648 | 23 |
| KIDNE2008022 | 0.006936741 | 41638 | 2 |
| KIDNE2008048 | 0.312345136 | 76422 | 86 |
| KIDNE2008069 | 0.14355133 | 99721 | 37 |
| KIDNE2008072 | 0.005841463 | 259485 | 1 |
| KIDNE2008116 | 0.144417246 | 32354 | 16 |
| KIDNE2008117 | 0.005841463 | 107715 | 1 |
| KIDNE2008218 | 0.005841463 | 262493 | 1 |
| KIDNE2008315 | 0.011682925 | 164689 | 2 |
| KIDNE2008362 | 0.036695152 | 36442 | 9 |
| KIDNE2008378 | 0.005841463 | 250677 | 1 |
| KIDNE2008403 | 0.123204427 | 89019 | 69 |
| KIDNE2008404 | 0.036134804 | 85611 | 4 |
| KIDNE2008446 | 0.005841463 | 89873 | 1 |
| KIDNE2008473 | 0.005841463 | 169529 | 1 |
| KIDNE2008480 | 0.005841463 | 261199 | 1 |
| KIDNE2008649 | 0.19005511 | 113533 | 33 |
| KIDNE2008666 | 0.00751701 | 261141 | 2 |
| KIDNE2008697 | 0.005841463 | 283080 | 1 |
| KIDNE2008758 | 0.007699577 | 198464 | 2 |
| KIDNE2008788 | 0.005841463 | 260591 | 1 |
| KIDNE2008795 | 0.005841463 | 260590 | 1 |
| KIDNE2008824 | 0.005841463 | 190329 | 1 |
| KIDNE2008832 | 0.005841463 | 92562 | 1 |
| KIDNE2008869 | 0.005841463 | 246472 | 1 |
| KIDNE2008914 | 0.009632435 | 140499 | 3 |
| KIDNE2008975 | 0.005841463 | 157597 | 1 |
| KIDNE2008987 | 0.058166891 | 36264 | 5 |
| KIDNE2009019 | 0.007922975 | 74230 | 2 |
| KIDNE2009109 | 0.005841463 | 192992 | 1 |
| KIDNE2009180 | 0.005841463 | 139249 | 1 |
| KIDNE2009191 | 0.005841463 | 39975 | 1 |
| KIDNE2009332 | 0.008886344 | 226666 | 2 |
| KIDNE2009367 | 0.222316502 | 89088 | 106 |
| KIDNE2009426 | 0.005841463 | 265033 | 1 |
| KIDNE2009467 | 0.005841463 | 101240 | 1 |
| KIDNE2009490 | 0.005841463 | 17577 | 1 |
| KIDNE2009553 | 0.005841463 | 253927 | 1 |
| KIDNE2009605 | 0.011095134 | 139982 | 4 |
| KIDNE2009628 | 0.005841463 | 159310 | 1 |
| KIDNE2009647 | 0.005841463 | 237873 | 1 |
| KIDNE2009676 | 0.005841463 | 98857 | 1 |
| KIDNE2009723 | 0.005841463 | 102830 | 1 |
| KIDNE2010007 | 0.005841463 | 44082 | 1 |
| KIDNE2010049 | 0.048733552 | 171247 | 5 |
| KIDNE2010052 | 0.005841463 | 186001 | 1 |
| KIDNE2010084 | 0.005841463 | 143367 | 1 |
| KIDNE2010137 | 0.005841463 | 274099 | 1 |
| KIDNE2010151 | 0.005841463 | 59998 | 1 |
| KIDNE2010264 | 0.005841463 | 143393 | 1 |
| KIDNE2010265 | 0.070921038 | 117677 | 7 |
| KIDNE2010271 | 0.005841463 | 162997 | 1 |
| KIDNE2010419 | 0.005841463 | 82090 | 1 |
| KIDNE2010430 | 0.017570502 | 149556 | 5 |
| KIDNE2010574 | 0.005841463 | 171107 | 1 |
| KIDNE2010674 | 0.005841463 | 153297 | 1 |
| KIDNE2010739 | 0.005841463 | 34571 | 1 |
| KIDNE2010750 | 0.015731596 | 132621 | 5 |
| KIDNE2010762 | 0.005841463 | 151481 | 1 |
| KIDNE2010863 | 0.005841463 | 201466 | 1 |
| KIDNE2010973 | 0.005841463 | 143373 | 1 |
| KIDNE2010989 | 0.005841463 | 167520 | 1 |
| KIDNE2011200 | 0.005841463 | 107238 | 1 |
| KIDNE2011314 | 0.007699577 | 101352 | 2 |
| KIDNE2011508 | 0.008962269 | 132593 | 2 |
| KIDNE2011532 | 0.005841463 | 177530 | 1 |
| KIDNE2011635 | 0.012043782 | 2290 | 2 |
| KIDNE2011752 | 0.007030707 | 157537 | 2 |
| KIDNE2011782 | 0.005841463 | 150504 | 1 |
| KIDNE2011858 | 0.005841463 | 81046 | 1 |
| KIDNE2012009 | 0.017602018 | 168369 | 2 |
| KIDNE2012025 | 0.071403765 | 53754 | 4 |
| KIDNE2012188 | 0.01876609 | 179530 | 4 |
| KIDNE2012291 | 0.007030707 | 197105 | 2 |
| KIDNE2012316 | 0.009879746 | 160552 | 2 |
| KIDNE2012361 | 0.011682925 | 179550 | 2 |
| KIDNE2012440 | 0.094659283 | 30801 | 14 |
| KIDNE2012453 | 0.005841463 | 179558 | 1 |
| KIDNE2012563 | 0.005841463 | 186041 | 1 |
| KIDNE2012601 | 0.005841463 | 162359 | 1 |
| KIDNE2012613 | 0.005841463 | 181549 | 1 |
| KIDNE2012710 | 0.017694873 | 153418 | 6 |
| KIDNE2012745 | 0.005841463 | 29961 | 1 |
| KIDNE2012775 | 0.005841463 | 171433 | 1 |
| KIDNE2012784 | 0.009838265 | 143652 | 3 |
| KIDNE2012945 | 0.011986989 | 162368 | 2 |
| KIDNE2013045 | 0.005841463 | 243287 | 1 |
| KIDNE2013095 | 0.005841463 | 152577 | 1 |
| KIDNE2013158 | 0.009237019 | 118760 | 3 |
| KIDNE2013218 | 0.005841463 | 143115 | 1 |
| KIDNE2013263 | 0.005841463 | 164843 | 1 |
| KIDNE2013346 | 0.008032019 | 13633 | 3 |
| KIDNE2013388 | 0.005841463 | 56929 | 1 |
| KIDNE2013413 | 0.008936959 | 170067 | 2 |
| KIDNE2013489 | 0.005841463 | 105329 | 1 |
| KIDNE2013639 | 0.005841463 | 279995 | 1 |
| KIDNE2013731 | 0.005841463 | 87918 | 1 |
| KIDNE2013734 | 0.005841463 | 162160 | 1 |
| KIDNE2013775 | 0.005841463 | 85851 | 1 |
| KIDNE2013801 | 0.005841463 | 153265 | 1 |
| KIDNE2013845 | 0.149912658 | 2957 | 39 |
| KIDNE2013866 | 0.006936741 | 123374 | 2 |
| KIDNE2014087 | 0.005841463 | 119807 | 1 |
| KIDNE2014112 | 0.005841463 | 167563 | 1 |
| KIDNE2014119 | 0.011754074 | 171320 | 2 |
| KIDNE2014170 | 0.005841463 | 197149 | 1 |
| KIDNE2014184 | 0.005841463 | 48528 | 1 |
| KIDNE2014268 | 0.005841463 | 164446 | 1 |
| KIDNE2014290 | 0.239007391 | 94988 | 60 |
| KIDNE2014298 | 0.005841463 | 103908 | 1 |
| KIDNE2014320 | 0.007699577 | 72871 | 2 |
| KIDNE2014325 | 0.044292984 | 76099 | 8 |
| KIDNE2014415 | 0.005841463 | 92025 | 1 |
| KIDNE2014489 | 0.005841463 | 185937 | 1 |
| KIDNE2014496 | 0.005841463 | 162202 | 1 |
| KIDNE2014661 | 0.005841463 | 55801 | 1 |
| KIDNE2014717 | 0.005841463 | 163430 | 1 |
| KIDNE2014789 | 0.005841463 | 82635 | 1 |
| KIDNE2014808 | 0.007718304 | 126721 | 2 |
| KIDNE2014890 | 0.005841463 | 137416 | 1 |
| KIDNE2014978 | 0.007529739 | 190402 | 2 |
| KIDNE2015073 | 0.005841463 | 127158 | 1 |
| KIDNE2015221 | 0.005841463 | 86813 | 1 |
| KIDNE2015244 | 0.005841463 | 94431 | 1 |
| KIDNE2015313 | 0.005841463 | 104623 | 1 |
| KIDNE2015433 | 0.018962576 | 52811 | 6 |
| KIDNE2015445 | 0.04877164 | 91753 | 9 |
| KIDNE2015483 | 0.005841463 | 172844 | 1 |
| KIDNE2015515 | 0.005841463 | 91705 | 1 |
| KIDNE2015546 | 0.012043782 | 141220 | 2 |
| KIDNE2015556 | 0.011682925 | 162166 | 2 |
| KIDNE2015598 | 0.007238618 | 69821 | 2 |
| KIDNE2015660 | 0.005841463 | 14900 | 1 |
| KIDNE2015710 | 0.089512404 | 152846 | 4 |
| KIDNE2015987 | 0.005841463 | 59983 | 1 |
| KIDNE2016000 | 0.005841463 | 62068 | 1 |
| KIDNE2016036 | 0.005841463 | 154599 | 1 |
| KIDNE2016096 | 0.005841463 | 143374 | 1 |
| KIDNE2016101 | 0.007030707 | 139888 | 2 |
| KIDNE2016142 | 0.005841463 | 59793 | 1 |
| KIDNE2016188 | 0.005841463 | 185453 | 1 |
| KIDNE2016327 | 0.009576419 | 223464 | 3 |
| KIDNE2016371 | 0.018597235 | 3640 | 7 |
| KIDNE2016388 | 0.005841463 | 141789 | 1 |
| KIDNE2016464 | 0.005841463 | 211929 | 1 |
| KIDNE2016539 | 0.011753725 | 132849 | 2 |
| KIDNE2016570 | 0.006936741 | 167578 | 2 |
| KIDNE2016918 | 0.005841463 | 59999 | 1 |
| KIDNE2017007 | 0.005841463 | 224923 | 1 |
| KIDNE2017040 | 0.049828212 | 152509 | 11 |
| KIDNE2017052 | 0.007238618 | 211940 | 2 |
| KIDNE2017151 | 0.005841463 | 204719 | 1 |
| KIDNE2017153 | 0.007030707 | 218663 | 2 |
| KIDNE2017315 | 0.005841463 | 116673 | 1 |
| KIDNE2017332 | 0.005841463 | 130116 | 1 |
| KIDNE2017343 | 0.050732526 | 101406 | 10 |
| KIDNE2017454 | 0.005841463 | 284919 | 1 |
| KIDNE2017603 | 0.005841463 | 230111 | 1 |
| KIDNE2017956 | 0.005841463 | 146072 | 1 |
| KIDNE2018071 | 0.011895831 | 234734 | 2 |
| KIDNE2018166 | 0.005841463 | 192198 | 1 |
| KIDNE2018167 | 0.005841463 | 156167 | 1 |
| KIDNE2018217 | 0.027611917 | 156173 | 5 |
| KIDNE2018254 | 0.005841463 | 215295 | 1 |
| KIDNE2018268 | 0.005841463 | 281773 | 1 |
| KIDNE2018269 | 0.005841463 | 268983 | 1 |
| KIDNE2018287 | 0.005841463 | 107290 | 1 |
| KIDNE2018294 | 0.005841463 | 154541 | 1 |
| KIDNE2018317 | 0.006936741 | 145953 | 2 |
| KIDNE2018352 | 0.046986847 | 151862 | 14 |
| KIDNE2018462 | 0.005841463 | 104999 | 1 |
| KIDNE2018493 | 0.047023149 | 102937 | 12 |
| KIDNE2018617 | 0.005841463 | 22470 | 1 |
| KIDNE2018678 | 0.005841463 | 170406 | 1 |
| KIDNE2018727 | 0.005841463 | 59992 | 1 |
| KIDNE2018863 | 0.005841463 | 198380 | 1 |
| KIDNE2018891 | 0.027475964 | 70931 | 8 |
| KIDNE2018989 | 0.005841463 | 49498 | 1 |
| KIDNE2018996 | 0.005841463 | 186076 | 1 |
| KIDNE2019074 | 0.005841463 | 170278 | 1 |
| KIDNE2019187 | 0.005841463 | 158649 | 1 |
| LIVER1000017 | 1.127224016 | 55076 | 70 |
| LIVER1000050 | 0.014524328 | 63808 | 1 |
| LIVER1000055 | 0.014524328 | 80879 | 1 |
| LIVER1000058 | 0.014524328 | 87072 | 1 |
| LIVER1000063 | 0.014524328 | 74134 | 1 |
| LIVER1000067 | 0.241381518 | 45313 | 14 |
| LIVER1000073 | 0.014524328 | 53701 | 1 |
| LIVER1000079 | 0.095625863 | 7710 | 18 |
| LIVER1000082 | 0.014524328 | 69867 | 1 |
| LIVER1000099 | 0.014524328 | 70122 | 1 |
| LIVER1000104 | 0.813015278 | 44077 | 87 |
| LIVER1000111 | 0.026531826 | 79172 | 3 |
| LIVER1000126 | 0.021855964 | 77137 | 3 |
| LIVER1000132 | 0.334841432 | 108373 | 38 |
| LIVER1000139 | 0.014524328 | 23707 | 1 |
| LIVER1000155 | 0.014524328 | 33623 | 1 |
| LIVER1000165 | 0.014524328 | 50213 | 1 |
| LIVER1000175 | 0.014524328 | 76038 | 1 |
| LIVER1000224 | 0.029048656 | 137479 | 2 |
| LIVER1000230 | 0.028892369 | 52005 | 6 |
| LIVER1000278 | 0.014524328 | 259488 | 1 |
| LIVER1000303 | 0.018715794 | 87074 | 4 |
| LIVER1000397 | 0.014524328 | 260977 | 1 |
| LIVER1000421 | 0.02043694 | 7985 | 2 |
| LIVER1000433 | 0.014524328 | 186415 | 1 |
| LIVER1000479 | 0.014524328 | 196881 | 1 |
| LIVER1000482 | 0.014524328 | 66532 | 1 |
| LIVER1000542 | 0.509239555 | 109803 | 51 |
| LIVER2000003 | 0.014524328 | 265627 | 1 |
| LIVER2000033 | 0.121122217 | 151526 | 10 |
| LIVER2000037 | 2.648325881 | 97070 | 100 |
| LIVER2000158 | 0.024233066 | 202224 | 2 |
| LIVER2000216 | 0.014524328 | 1971 | 1 |
| LIVER2000237 | 0.043572985 | 187971 | 3 |
| LIVER2000247 | 0.014524328 | 214426 | 1 |
| LIVER2000360 | 0.014524328 | 233230 | 1 |
| LIVER2000416 | 0.074052587 | 126378 | 8 |
| LIVER2000446 | 0.040733555 | 194458 | 4 |
| LIVER2000515 | 0.043572985 | 219997 | 3 |
| LIVER2000592 | 0.014524328 | 210687 | 1 |
| LIVER2000626 | 0.014524328 | 51706 | 1 |
| LIVER2000682 | 0.014524328 | 168557 | 1 |
| LIVER2000769 | 0.014524328 | 97593 | 1 |
| LIVER2000775 | 0.014524328 | 250788 | 1 |
| LIVER2001051 | 0.014524328 | 98689 | 1 |
| LIVER2001076 | 0.014524328 | 97553 | 1 |
| LIVER2001099 | 0.014524328 | 172545 | 1 |
| LIVER2001113 | 0.015619607 | 152117 | 2 |
| LIVER2001164 | 0.014524328 | 95457 | 1 |
| LIVER2001191 | 0.014524328 | 275525 | 1 |
| LIVER2001265 | 0.016212605 | 124333 | 2 |
| LIVER2001329 | 0.014524328 | 101528 | 1 |
| LIVER2001389 | 0.014524328 | 135987 | 1 |
| LIVER2001461 | 0.014524328 | 182285 | 1 |
| LIVER2001539 | 0.033646648 | 133742 | 7 |
| LIVER2001608 | 0.127587354 | 98471 | 15 |
| LIVER2001835 | 0.020460103 | 163420 | 2 |
| LIVER2002638 | 0.020565894 | 239093 | 2 |
| LIVER2002644 | 0.014524328 | 96584 | 1 |
| LIVER2002660 | 0.014524328 | 164147 | 1 |
| LIVER2002702 | 0.014524328 | 275619 | 1 |
| LIVER2002842 | 0.087770458 | 74323 | 24 |
| LIVER2003008 | 0.016212605 | 246153 | 2 |
| LIVER2003065 | 0.026043738 | 186110 | 2 |
| LIVER2003234 | 0.01652273 | 90853 | 2 |
| LIVER2003511 | 0.020365791 | 156137 | 2 |
| LIVER2003524 | 0.014524328 | 264507 | 1 |
| LIVER2003568 | 0.014524328 | 228163 | 1 |
| LIVER2003581 | 0.050048307 | 48527 | 2 |
| LIVER2003800 | 0.160723159 | 153720 | 5 |
| LIVER2003854 | 0.014524328 | 239331 | 1 |
| LIVER2004074 | 0.014524328 | 204293 | 1 |
| LIVER2004392 | 0.014524328 | 162596 | 1 |
| LIVER2004514 | 0.01640117 | 108285 | 2 |
| LIVER2004565 | 0.014524328 | 160461 | 1 |
| LIVER2005028 | 0.022175912 | 81176 | 3 |
| LIVER2005198 | 0.014524328 | 112292 | 1 |
| LIVER2005218 | 0.067937294 | 98474 | 5 |
| LIVER2005376 | 0.014524328 | 48121 | 1 |
| LIVER2005385 | 0.014524328 | 272916 | 1 |
| LIVER2005512 | 0.014524328 | 97330 | 1 |
| LIVER2005520 | 0.014524328 | 267175 | 1 |
| LIVER2005527 | 0.014524328 | 71251 | 1 |
| LIVER2005544 | 0.014524328 | 58825 | 1 |
| LIVER2005625 | 0.014524328 | 240295 | 1 |
| LIVER2005728 | 0.029048656 | 17782 | 2 |
| LIVER2005789 | 0.186122649 | 168945 | 15 |
| LIVER2005973 | 0.016233788 | 82267 | 2 |
| LIVER2005981 | 0.058518202 | 45805 | 3 |
| LIVER2006006 | 0.235617442 | 51513 | 19 |
| LIVER2006251 | 0.014524328 | 261194 | 1 |
| LIVER2006410 | 0.016382443 | 100019 | 2 |
| LIVER2006469 | 0.014524328 | 11714 | 1 |
| LIVER2006644 | 0.014524328 | 44089 | 1 |
| LIVER2006764 | 0.014524328 | 170276 | 1 |
| LIVER2007415 | 0.014524328 | 118556 | 1 |
| LIVER2007548 | 0.014524328 | 147039 | 1 |
| LIVER2007568 | 0.014524328 | 258669 | 1 |
| LIVER2007721 | 0.014524328 | 138529 | 1 |
| LIVER2007783 | 0.081661418 | 65980 | 10 |
| LIVER2008053 | 0.014524328 | 141188 | 1 |
| LIVER2008465 | 0.025317831 | 117099 | 6 |
| LIVER2008473 | 0.014524328 | 172573 | 1 |
| LIVER2008580 | 0.014524328 | 147325 | 1 |
| LIVER2008706 | 0.037563149 | 186228 | 3 |
| LIVER2008751 | 0.0192973 | 17050 | 3 |
| LIVER2008945 | 0.014524328 | 153028 | 1 |
| LIVER2008998 | 0.014524328 | 151693 | 1 |
| LIVER2009110 | 0.014524328 | 117175 | 1 |
| LIVER2009118 | 0.014524328 | 74451 | 1 |
| LIVER2009554 | 0.014524328 | 132207 | 1 |
| LYMPB1000141 | 0.039118092 | 131764 | 2 |
| LYMPB1000158 | 0.038022814 | 251564 | 1 |
| LYMPB2000083 | 0.038022814 | 103124 | 1 |
| MAMGL1000017 | 0.551111774 | 59524 | 3 |
| MAMGL1000032 | 1.039921212 | 30853 | 46 |
| MAMGL1000035 | 0.832075063 | 17419 | 37 |
| MAMGL1000056 | 0.584454109 | 94457 | 13 |
| MAMGL1000083 | 0.578386106 | 14366 | 20 |
| MAMGL1000096 | 0.556072719 | 57473 | 2 |
| MAMGL1000100 | 0.567554653 | 5632 | 6 |
| MAMGL1000132 | 0.543478261 | 32038 | 1 |
| MAMGL1000151 | 0.543478261 | 68645 | 1 |
| MAMGL1000173 | 0.632159969 | 44347 | 16 |
| MAMGL1000178 | 0.547790273 | 8955 | 3 |
| MAMGL1000182 | 0.543478261 | 37855 | 1 |
| MAMGL1000184 | 0.543478261 | 65284 | 1 |
| MESAN1000032 | 0.008071224 | 21005 | 2 |
| MESAN1000035 | 0.00621311 | 40530 | 1 |
| MESAN1000050 | 0.10437495 | 45654 | 23 |
| MESAN1000079 | 0.011657526 | 64979 | 4 |
| MESAN1000080 | 0.117354162 | 72243 | 12 |
| MESAN1000101 | 0.029028899 | 69446 | 12 |
| MESAN1000121 | 0.00621311 | 50003 | 1 |
| MESAN1000126 | 0.093829107 | 61776 | 20 |
| MESAN1000147 | 0.016341156 | 42348 | 4 |
| MESAN1000180 | 0.00621311 | 23908 | 1 |
| MESAN2000023 | 0.00621311 | 192936 | 1 |
| MESAN2000026 | 0.00621311 | 236371 | 1 |
| MESAN2000067 | 0.009308606 | 143501 | 2 |
| MESAN2000092 | 0.018935193 | 157449 | 7 |
| MESAN2000149 | 0.06820933 | 131401 | 16 |
| MESAN2000167 | 0.00621311 | 115003 | 1 |
| MESAN2000238 | 0.402506729 | 91756 | 67 |
| MESAN2000264 | 0.085027976 | 137712 | 11 |
| MESAN2000267 | 0.409174084 | 50495 | 78 |
| MESAN2000291 | 0.007308388 | 27545 | 2 |
| MESAN2000337 | 0.021131934 | 219460 | 3 |
| MESAN2000434 | 0.007308388 | 139268 | 2 |
| MESAN2000457 | 0.042308733 | 107271 | 12 |
| MESAN2000462 | 0.00621311 | 46273 | 1 |
| MESAN2000501 | 0.055850323 | 204130 | 7 |
| MESAN2000572 | 0.163888782 | 117236 | 41 |
| MESAN2000608 | 0.213075464 | 28732 | 29 |
| MESAN2000620 | 0.009195069 | 252269 | 2 |
| MESAN2000711 | 0.00621311 | 119795 | 1 |
| MESAN2000815 | 0.010731071 | 122318 | 3 |
| MESAN2000894 | 0.008211511 | 136814 | 2 |
| MESAN2000909 | 0.030336812 | 179017 | 5 |
| MESAN2001154 | 0.125760223 | 41668 | 14 |
| MESAN2001450 | 0.058100158 | 59450 | 8 |
| MESAN2001627 | 0.01281038 | 114857 | 4 |
| MESAN2001770 | 0.00621311 | 70800 | 1 |
| MESAN2001979 | 0.012426219 | 144971 | 2 |
| MESAN2001980 | 0.012358636 | 107695 | 2 |
| MESAN2002086 | 0.402506729 | 91756 | 67 |
| MESAN2002113 | 0.00621311 | 87821 | 1 |
| MESAN2002122 | 0.018890012 | 52964 | 8 |
| MESAN2002147 | 0.00621311 | 39642 | 1 |
| MESAN2002186 | 0.076436153 | 92223 | 6 |
| MESAN2002424 | 0.008211511 | 200502 | 2 |
| MESAN2002519 | 0.036128095 | 131882 | 9 |
| MESAN2002687 | 0.00621311 | 192739 | 1 |
| MESAN2002709 | 0.00621311 | 98698 | 1 |
| MESAN2002724 | 0.181696741 | 97935 | 35 |
| MESAN2002790 | 0.068509697 | 87165 | 14 |
| MESAN2002844 | 0.023123682 | 53028 | 7 |
| MESAN2002940 | 0.052685086 | 126143 | 18 |
| MESAN2002978 | 0.00621311 | 254679 | 1 |
| MESAN2003035 | 0.023379497 | 148936 | 7 |
| MESAN2003037 | 0.00621311 | 85891 | 1 |
| MESAN2003039 | 0.010293023 | 99445 | 3 |
| MESAN2003058 | 0.027977249 | 139201 | 8 |
| MESAN2003101 | 0.00621311 | 100388 | 1 |
| MESAN2003190 | 0.009468379 | 22818 | 3 |
| MESAN2003322 | 0.00621311 | 185360 | 1 |
| MESAN2003444 | 0.044978915 | 79440 | 15 |
| MESAN2003490 | 0.00621311 | 250337 | 1 |
| MESAN2003529 | 0.00621311 | 166489 | 1 |
| MESAN2003622 | 0.278812027 | 97643 | 28 |
| MESAN2003646 | 0.008071224 | 70254 | 2 |
| MESAN2003662 | 0.00621311 | 53111 | 1 |
| MESAN2003709 | 0.020201654 | 186592 | 4 |
| MESAN2003851 | 0.00621311 | 84220 | 1 |
| MESAN2003852 | 0.166281879 | 99772 | 37 |
| MESAN2004138 | 0.00621311 | 125779 | 1 |
| MESAN2004288 | 0.032244849 | 144871 | 4 |
| MESAN2004575 | 0.025962295 | 180208 | 3 |
| MESAN2005284 | 0.034553936 | 184030 | 8 |
| MESAN2005303 | 0.021149473 | 165010 | 6 |
| MESAN2005371 | 0.00621311 | 242346 | 1 |
| MESAN2005633 | 0.019521102 | 58808 | 8 |
| MESAN2005689 | 0.054008359 | 106448 | 18 |
| MESAN2005724 | 0.00621311 | 149657 | 1 |
| MESAN2005766 | 0.169915307 | 88288 | 40 |
| MESAN2005808 | 0.00621311 | 87668 | 1 |
| MESAN2005811 | 0.00621311 | 77594 | 1 |
| MESAN2005957 | 0.033936434 | 110107 | 13 |
| MESAN2005958 | 0.062218274 | 17625 | 20 |
| MESAN2006022 | 0.00621311 | 55165 | 1 |
| MESAN2006043 | 0.021037077 | 105300 | 6 |
| MESAN2006328 | 0.00621311 | 216829 | 1 |
| MESAN2006401 | 0.092619948 | 51003 | 33 |
| MESAN2006563 | 0.242649079 | 50179 | 33 |
| MESAN2006580 | 0.008211511 | 182782 | 2 |
| MESAN2006599 | 0.00621311 | 278390 | 1 |
| MESAN2006743 | 0.04346969 | 53998 | 10 |
| MESAN2006953 | 0.024435922 | 120366 | 6 |
| MESAN2006971 | 0.02788572 | 185376 | 3 |
| MESAN2007032 | 0.00621311 | 43348 | 1 |
| MESAN2007440 | 0.00621311 | 82340 | 1 |
| MESAN2007597 | 0.00621311 | 67163 | 1 |
| MESAN2007755 | 0.018315664 | 124281 | 5 |
| MESAN2008222 | 0.00621311 | 262155 | 1 |
| MESAN2008334 | 0.020381848 | 144118 | 5 |
| MESAN2008415 | 0.015667586 | 76296 | 2 |
| MESAN2008460 | 0.00621311 | 82850 | 1 |
| MESAN2008536 | 0.00621311 | 260114 | 1 |
| MESAN2008679 | 0.023912265 | 28821 | 3 |
| MESAN2008729 | 0.007402354 | 202280 | 2 |
| MESAN2008821 | 0.00621311 | 240842 | 1 |
| MESAN2008926 | 0.052485634 | 65393 | 6 |
| MESAN2008936 | 0.042118119 | 123560 | 15 |
| MESAN2009019 | 0.021448765 | 179018 | 5 |
| MESAN2009081 | 0.035597418 | 103309 | 8 |
| MESAN2009105 | 0.00621311 | 137709 | 1 |
| MESAN2009156 | 0.016112319 | 130330 | 4 |
| MESAN2009311 | 0.00621311 | 154516 | 1 |
| MESAN2009418 | 0.00621311 | 238318 | 1 |
| MESAN2009503 | 0.010057194 | 50552 | 2 |
| MESAN2009522 | 0.00621311 | 143649 | 1 |
| MESAN2009580 | 0.150876604 | 132500 | 13 |
| MESAN2010031 | 0.00621311 | 3477 | 1 |
| MESAN2010042 | 0.066319138 | 118392 | 17 |
| MESAN2010114 | 0.00621311 | 96491 | 1 |
| MESAN2010312 | 0.00621311 | 177125 | 1 |
| MESAN2010321 | 0.00621311 | 15928 | 1 |
| MESAN2010664 | 0.019306872 | 131585 | 5 |
| MESAN2010681 | 0.00621311 | 216654 | 1 |
| MESAN2011545 | 0.00621311 | 208549 | 1 |
| MESAN2011597 | 0.00621311 | 54585 | 1 |
| MESAN2011627 | 0.044522139 | 129431 | 4 |
| MESAN2011632 | 0.00792257 | 56483 | 2 |
| MESAN2011977 | 0.00621311 | 161310 | 1 |
| MESAN2011984 | 0.098214026 | 16810 | 22 |
| MESAN2012054 | 0.065685408 | 91419 | 23 |
| MESAN2012113 | 0.028399837 | 104578 | 7 |
| MESAN2012586 | 0.00621311 | 168500 | 1 |
| MESAN2012708 | 0.00621311 | 151405 | 1 |
| MESAN2012735 | 0.016815315 | 170857 | 2 |
| MESAN2013019 | 0.00621311 | 75146 | 1 |
| MESAN2013022 | 0.00621311 | 179640 | 1 |
| MESAN2013183 | 0.00621311 | 181032 | 1 |
| MESAN2013211 | 0.00621311 | 11286 | 1 |
| MESAN2013283 | 0.010057194 | 260206 | 2 |
| MESAN2013284 | 0.136426651 | 49064 | 49 |
| MESAN2013611 | 0.00621311 | 127163 | 1 |
| MESAN2013674 | 0.00621311 | 11974 | 1 |
| MESAN2013845 | 0.030029415 | 60353 | 4 |
| MESAN2013908 | 0.024284853 | 119331 | 4 |
| MESAN2013936 | 0.00621311 | 50439 | 1 |
| MESAN2014192 | 0.007610265 | 14817 | 2 |
| MESAN2014295 | 0.008211511 | 111348 | 2 |
| MESAN2014298 | 0.00621311 | 186159 | 1 |
| MESAN2014412 | 0.008705543 | 117617 | 3 |
| MESAN2014624 | 0.00621311 | 102347 | 1 |
| MESAN2015277 | 0.00621311 | 180796 | 1 |
| MESAN2015365 | 0.007402354 | 28539 | 2 |
| MESAN2015391 | 0.008294622 | 134048 | 2 |
| MESAN2015401 | 0.00621311 | 27944 | 1 |
| MESAN2015501 | 0.00621311 | 133000 | 1 |
| MESAN2015515 | 0.063076038 | 110366 | 6 |
| MESAN2015708 | 0.018011128 | 275419 | 2 |
| MESAN2016159 | 0.007308388 | 230963 | 2 |
| MESAN2016304 | 0.00621311 | 274014 | 1 |
| MESAN2016409 | 0.010057194 | 185888 | 2 |
| MESAN2016552 | 0.01377169 | 144925 | 5 |
| MESAN2016965 | 0.00621311 | 177672 | 1 |
| MESAN2017133 | 0.019097239 | 127923 | 6 |
| MESAN2017152 | 0.008211511 | 193140 | 2 |
| MESAN2017373 | 0.047837489 | 149079 | 3 |
| MESAN2017417 | 0.00621311 | 9337 | 1 |
| MESAN2018209 | 0.00621311 | 118478 | 1 |
| MESAN2018576 | 0.015921848 | 221004 | 2 |
| MESAN2018670 | 0.00621311 | 82391 | 1 |
| MESAN2018699 | 0.010883345 | 126608 | 3 |
| MESTC1000042 | 0.149258139 | 113402 | 4 |
| MESTC1000147 | 0.1447178 | 238409 | 1 |
| MESTC2000150 | 0.1447178 | 247832 | 1 |
| MESTC2000153 | 0.1447178 | 255090 | 1 |
| MESTC2000170 | 0.1447178 | 236425 | 1 |
| N1ESE2000698 | 0.039448906 | 15070 | 2 |
| NB9N41000011 | 0.078728801 | 8604 | 9 |
| NB9N41000047 | 0.063749775 | 65266 | 4 |
| NB9N41000121 | 0.223129215 | 67523 | 14 |
| NB9N41000135 | 0.075549107 | 80203 | 5 |
| NB9N41000142 | 0.06258574 | 34767 | 3 |
| NB9N41000146 | 0.056689342 | 66569 | 1 |
| NB9N41000340 | 0.115533323 | 49241 | 10 |
| NB9N42000042 | 0.086235482 | 104154 | 13 |
| NB9N42000104 | 0.202136096 | 145316 | 14 |
| NB9N42000122 | 0.056689342 | 216519 | 1 |
| NB9N42000196 | 0.165899693 | 250956 | 3 |
| NB9N42000281 | 0.087630223 | 125130 | 12 |
| NB9N42000314 | 0.056689342 | 218830 | 1 |
| NB9N42000342 | 0.232387732 | 103773 | 19 |
| NB9N42000430 | 0.060533426 | 100042 | 2 |
| NB9N42000495 | 0.056689342 | 67307 | 1 |
| NB9N42001300 | 0.061898551 | 52194 | 4 |
| NCRRP1000129 | 0.143061516 | 251354 | 1 |
| NESOP1000010 | 0.035650624 | 230824 | 1 |
| NESOP1000087 | 0.041563235 | 157486 | 2 |
| NESOP1000108 | 0.035650624 | 112218 | 1 |
| NESOP2000231 | 0.035650624 | 235751 | 1 |
| NESOP2000452 | 0.035650624 | 63439 | 1 |
| NESOP2000499 | 0.035650624 | 227862 | 1 |
| NESOP2000504 | 0.037047779 | 72164 | 2 |
| NESOP2000744 | 0.035650624 | 192316 | 1 |
| NESOP2001144 | 0.035650624 | 275693 | 1 |
| NESOP2001433 | 0.035650624 | 266209 | 1 |
| NESOP2001656 | 0.041852944 | 163725 | 2 |
| NESOP2001694 | 0.035650624 | 274555 | 1 |
| NESOP2001752 | 0.036839868 | 238005 | 2 |
| NESOP2002352 | 0.035650624 | 249061 | 1 |
| NESOP2002418 | 0.035650624 | 270810 | 1 |
| NESOP2002487 | 0.035650624 | 280908 | 1 |
| NESOP2002738 | 0.035650624 | 234571 | 1 |
| NESOP2002780 | 0.035650624 | 203339 | 1 |
| NETRP1000034 | 0.010827198 | 134951 | 1 |
| NETRP1000082 | 0.010827198 | 246140 | 1 |
| NETRP2000086 | 0.010827198 | 235690 | 1 |
| NETRP2000182 | 0.010827198 | 231597 | 1 |
| NETRP2000439 | 0.032481594 | 134784 | 3 |
| NHNPC1000034 | 0.043494297 | 133294 | 2 |
| NHNPC1000037 | 0.04180602 | 72536 | 1 |
| NHNPC1000084 | 0.04180602 | 97108 | 1 |
| NHNPC1000101 | 0.04180602 | 50677 | 1 |
| NHNPC1000124 | 0.04180602 | 71538 | 1 |
| NHNPC2000062 | 0.079954704 | 90681 | 10 |
| NHNPC2000187 | 0.370266431 | 100418 | 27 |
| NHNPC2000206 | 0.243998186 | 121013 | 62 |
| NHNPC2000212 | 0.048078306 | 18948 | 4 |
| NHNPC2000606 | 0.04180602 | 102204 | 1 |
| NHNPC2000877 | 0.04180602 | 185877 | 1 |
| NHNPC2001108 | 0.042901298 | 176597 | 2 |
| NHNPC2001223 | 0.044901516 | 102205 | 2 |
| NHNPC2001312 | 0.043887532 | 189476 | 2 |
| NHNPC2001816 | 0.04180602 | 269601 | 1 |
| NHNPC2001931 | 0.35109498 | 53308 | 28 |
| NHNPC2002565 | 0.04180602 | 170376 | 1 |
| NHNPC2002749 | 0.04180602 | 209207 | 1 |
| NOVAR1000015 | 0.055180045 | 160312 | 5 |
| NOVAR1000091 | 0.086526567 | 130741 | 10 |
| NOVAR1000102 | 0.120903585 | 143806 | 4 |
| NOVAR2000038 | 0.269744409 | 20263 | 50 |
| NOVAR2000136 | 0.039936102 | 282788 | 1 |
| NOVAR2000352 | 0.039936102 | 162230 | 1 |
| NOVAR2000412 | 0.039936102 | 71074 | 1 |
| NOVAR2000710 | 0.047565028 | 79451 | 5 |
| NOVAR2000783 | 0.044302137 | 196080 | 4 |
| NOVAR2000962 | 0.039936102 | 205724 | 1 |
| NOVAR2001108 | 0.039936102 | 275495 | 1 |
| NOVAR2001620 | 0.039936102 | 277910 | 1 |
| NOVAR2001783 | 0.041611649 | 192621 | 2 |
| NOVAR2001844 | 0.079018445 | 63416 | 8 |
| NOVAR2001876 | 0.039936102 | 267952 | 1 |
| NOVAR2002039 | 0.041333258 | 127798 | 2 |
| NT2NE1000004 | 0.020289133 | 65538 | 4 |
| NT2NE1000014 | 0.01927591 | 48558 | 7 |
| NT2NE1000018 | 0.067381772 | 51526 | 23 |
| NT2NE1000023 | 0.006072383 | 59163 | 1 |
| NT2NE1000024 | 0.043645394 | 50046 | 9 |
| NT2NE1000059 | 0.030978051 | 51277 | 4 |
| NT2NE1000063 | 0.012144766 | 52662 | 2 |
| NT2NE1000073 | 0.021042129 | 40425 | 5 |
| NT2NE1000083 | 0.006072383 | 48093 | 1 |
| NT2NE1000120 | 0.006072383 | 73851 | 1 |
| NT2NE1000122 | 0.007469538 | 53434 | 2 |
| NT2NE1000156 | 0.02649962 | 45436 | 5 |
| NT2NE1000163 | 0.413660566 | 49243 | 102 |
| NT2NE1000175 | 0.006072383 | 58574 | 1 |
| NT2NE1000181 | 0.009178998 | 30936 | 3 |
| NT2NE1000185 | 0.006072383 | 48982 | 1 |
| NT2NE2000038 | 0.006072383 | 88586 | 1 |
| NT2NE2000054 | 0.006072383 | 78336 | 1 |
| NT2NE2000056 | 0.426534229 | 37647 | 131 |
| NT2NE2000057 | 0.006072383 | 262434 | 1 |
| NT2NE2000064 | 0.006072383 | 146491 | 1 |
| NT2NE2000109 | 0.006072383 | 279204 | 1 |
| NT2NE2000137 | 0.006072383 | 265070 | 1 |
| NT2NE2000156 | 0.013155547 | 93786 | 3 |
| NT2NE2000174 | 0.439109139 | 77740 | 32 |
| NT2NE2000195 | 0.109001549 | 103469 | 15 |
| NT2NE2000214 | 0.131161861 | 116807 | 34 |
| NT2NE2000222 | 0.006072383 | 268088 | 1 |
| NT2NE2000259 | 0.05956676 | 158400 | 14 |
| NT2NE2000260 | 1.387073374 | 103993 | 89 |
| NT2NE2000284 | 0.006072383 | 114817 | 1 |
| NT2NE2000299 | 0.006072383 | 104697 | 1 |
| NT2NE2000327 | 0.066605006 | 22381 | 33 |
| NT2NE2000353 | 0.009167879 | 153808 | 2 |
| NT2NE2000356 | 0.007469538 | 176369 | 2 |
| NT2NE2000369 | 0.006072383 | 70435 | 1 |
| NT2NE2000374 | 0.006072383 | 187976 | 1 |
| NT2NE2000383 | 0.110267507 | 69725 | 32 |
| NT2NE2000384 | 0.072799264 | 83009 | 24 |
| NT2NE2000386 | 0.010843426 | 157388 | 3 |
| NT2NE2000390 | 0.006072383 | 270932 | 1 |
| NT2NE2000392 | 0.187681029 | 48071 | 66 |
| NT2NE2000455 | 0.008070784 | 134795 | 2 |
| NT2NE2000470 | 0.030704566 | 134793 | 7 |
| NT2NE2000488 | 0.006072383 | 273469 | 1 |
| NT2NE2000517 | 0.011798942 | 110083 | 3 |
| NT2NE2000536 | 0.019468635 | 186254 | 3 |
| NT2NE2000546 | 0.006072383 | 266324 | 1 |
| NT2NE2000548 | 0.007930497 | 160652 | 2 |
| NT2NE2000550 | 0.006072383 | 169321 | 1 |
| NT2NE2000575 | 0.696988405 | 17919 | 122 |
| NT2NE2000586 | 0.006072383 | 164805 | 1 |
| NT2NE2000612 | 0.006072383 | 147874 | 1 |
| NT2NE2000636 | 0.046404186 | 129610 | 11 |
| NT2NE2000658 | 0.006072383 | 222682 | 1 |
| NT2NE2000706 | 0.006072383 | 129087 | 1 |
| NT2NE2000707 | 0.500699743 | 52066 | 121 |
| NT2NE2000763 | 0.139619037 | 76345 | 33 |
| NT2NE2000787 | 0.169706805 | 109741 | 51 |
| NT2NE2000802 | 0.006072383 | 60033 | 1 |
| NT2NE2000808 | 0.006072383 | 265767 | 1 |
| NT2NE2000809 | 0.049432317 | 142817 | 11 |
| NT2NE2000864 | 0.007261627 | 182617 | 2 |
| NT2NE2000909 | 0.051185943 | 16713 | 16 |
| NT2NE2000913 | 0.022715338 | 13867 | 6 |
| NT2NE2000950 | 0.031968947 | 105203 | 7 |
| NT2NE2000951 | 0.006072383 | 163355 | 1 |
| NT2NE2000963 | 0.730406971 | 10433 | 69 |
| NT2NE2000980 | 0.010975379 | 102392 | 3 |
| NT2NE2001000 | 0.194695265 | 58103 | 29 |
| NT2NE2001005 | 0.007167661 | 166974 | 2 |
| NT2NE2001021 | 0.006072383 | 174056 | 1 |
| NT2NE2001040 | 0.010808018 | 128473 | 4 |
| NT2NE2001048 | 0.111946578 | 80591 | 26 |
| NT2NE2001049 | 0.019067089 | 18802 | 4 |
| NT2NE2001142 | 0.006072383 | 79591 | 1 |
| NT2NE2001156 | 0.029812719 | 96235 | 5 |
| NT2NE2001176 | 0.146698192 | 36550 | 40 |
| NT2NE2001180 | 0.012008158 | 206129 | 2 |
| NT2NE2001181 | 0.00898724 | 226151 | 2 |
| NT2NE2001188 | 0.006072383 | 68172 | 1 |
| NT2NE2001191 | 0.006072383 | 32216 | 1 |
| NT2NE2001247 | 0.006072383 | 269958 | 1 |
| NT2NE2001252 | 0.006072383 | 189199 | 1 |
| NT2NE2001324 | 0.007261627 | 41267 | 2 |
| NT2NE2001326 | 0.007949224 | 75722 | 2 |
| NT2NE2001337 | 0.036872043 | 74584 | 11 |
| NT2NE2001364 | 0.015672564 | 85122 | 4 |
| NT2NE2001372 | 0.006072383 | 27949 | 1 |
| NT2NE2001397 | 0.01273958 | 115606 | 4 |
| NT2NE2001403 | 0.032296527 | 122120 | 10 |
| NT2NE2001428 | 0.006072383 | 267723 | 1 |
| NT2NE2001432 | 0.006072383 | 32992 | 1 |
| NT2NE2001435 | 0.157605798 | 10147 | 69 |
| NT2NE2001442 | 0.033530102 | 93501 | 10 |
| NT2NE2001524 | 0.006072383 | 172655 | 1 |
| NT2NE2001530 | 0.023947856 | 57996 | 4 |
| NT2NE2001545 | 0.093514659 | 8806 | 31 |
| NT2NE2001598 | 0.013209227 | 154295 | 3 |
| NT2NE2001617 | 0.036932165 | 100046 | 7 |
| NT2NE2001623 | 0.006072383 | 225743 | 1 |
| NT2NE2001626 | 0.012144766 | 131707 | 2 |
| NT2NE2001634 | 0.006072383 | 85724 | 1 |
| NT2NE2001648 | 0.019305756 | 109733 | 4 |
| NT2NE2001655 | 0.119679354 | 11444 | 23 |
| NT2NE2001660 | 0.021393067 | 94956 | 7 |
| NT2NE2001666 | 0.006072383 | 9176 | 1 |
| NT2NE2001677 | 0.006072383 | 101330 | 1 |
| NT2NE2001697 | 0.030688551 | 159241 | 4 |
| NT2NE2001698 | 0.006072383 | 210391 | 1 |
| NT2NE2001723 | 0.006072383 | 208829 | 1 |
| NT2NE2001725 | 0.006072383 | 211817 | 1 |
| NT2NE2001793 | 0.161541388 | 60629 | 74 |
| NT2NE2001874 | 0.006072383 | 158706 | 1 |
| NT2NE2001889 | 0.006072383 | 77320 | 1 |
| NT2NE2001923 | 0.006072383 | 19176 | 1 |
| NT2NE2002100 | 0.007469538 | 152601 | 2 |
| NT2NE2002162 | 0.013705896 | 88624 | 3 |
| NT2NE2002186 | 0.006072383 | 259275 | 1 |
| NT2NE2002285 | 0.006072383 | 258902 | 1 |
| NT2NE2002311 | 0.006072383 | 86549 | 1 |
| NT2NE2002620 | 0.175174974 | 37247 | 3 |
| NT2NE2002651 | 0.006072383 | 148687 | 1 |
| NT2NE2002732 | 0.006072383 | 261036 | 1 |
| NT2NE2002768 | 0.006072383 | 243284 | 1 |
| NT2NE2002791 | 0.006072383 | 254786 | 1 |
| NT2NE2002856 | 0.006072383 | 261103 | 1 |
| NT2NE2002870 | 0.073402966 | 149096 | 7 |
| NT2NE2003150 | 0.006072383 | 264291 | 1 |
| NT2NE2003185 | 0.044201935 | 115411 | 13 |
| NT2NE2003252 | 0.006072383 | 181083 | 1 |
| NT2NE2003280 | 0.023023737 | 151479 | 7 |
| NT2NE2003308 | 0.006072383 | 194541 | 1 |
| NT2NE2003309 | 0.006072383 | 33587 | 1 |
| NT2NE2003315 | 0.006072383 | 171456 | 1 |
| NT2NE2003393 | 0.13035634 | 163132 | 3 |
| NT2NE2003408 | 0.006072383 | 193697 | 1 |
| NT2NE2003457 | 0.006072383 | 185775 | 1 |
| NT2NE2003485 | 0.071984942 | 106455 | 14 |
| NT2NE2003540 | 0.006072383 | 173634 | 1 |
| NT2NE2003569 | 0.096548347 | 139448 | 20 |
| NT2NE2003571 | 0.006072383 | 192465 | 1 |
| NT2NE2003705 | 0.006072383 | 259184 | 1 |
| NT2NE2003752 | 0.006072383 | 238693 | 1 |
| NT2NE2003887 | 0.026846204 | 94549 | 6 |
| NT2NE2003921 | 0.027465583 | 121717 | 6 |
| NT2NE2004255 | 0.028130935 | 121306 | 8 |
| NT2NE2004378 | 0.006072383 | 256633 | 1 |
| NT2NE2004490 | 0.006072383 | 241788 | 1 |
| NT2NE2004519 | 0.093506362 | 101732 | 15 |
| NT2NE2004606 | 0.006072383 | 235581 | 1 |
| NT2NE2004716 | 0.087297185 | 132046 | 6 |
| NT2NE2004740 | 0.007949224 | 251575 | 2 |
| NT2NE2004787 | 0.006072383 | 135224 | 1 |
| NT2NE2004916 | 0.006072383 | 51258 | 1 |
| NT2NE2005035 | 0.006072383 | 259213 | 1 |
| NT2NE2005223 | 0.006072383 | 93982 | 1 |
| NT2NE2005276 | 0.006072383 | 236380 | 1 |
| NT2NE2005317 | 0.013176752 | 176124 | 4 |
| NT2NE2005323 | 0.007469538 | 240074 | 2 |
| NT2NE2005358 | 0.008070784 | 97809 | 2 |
| NT2NE2005371 | 0.030388261 | 47610 | 6 |
| NT2NE2005395 | 0.012263375 | 67331 | 3 |
| NT2NE2005419 | 0.014471111 | 98619 | 6 |
| NT2NE2005441 | 0.053931326 | 102319 | 3 |
| NT2NE2005517 | 0.006072383 | 57515 | 1 |
| NT2NE2005537 | 0.006072383 | 249068 | 1 |
| NT2NE2005555 | 0.006072383 | 240742 | 1 |
| NT2NE2005588 | 0.009916467 | 193051 | 2 |
| NT2NE2005676 | 0.006072383 | 104634 | 1 |
| NT2NE2005688 | 0.224390279 | 112983 | 21 |
| NT2NE2005720 | 0.017835705 | 51662 | 2 |
| NT2NE2005784 | 0.006072383 | 263061 | 1 |
| NT2NE2005804 | 0.006072383 | 248927 | 1 |
| NT2NE2005821 | 0.006072383 | 260612 | 1 |
| NT2NE2005876 | 0.113589258 | 17299 | 31 |
| NT2NE2005890 | 0.009448936 | 200105 | 3 |
| NT2NE2005921 | 0.006072383 | 17703 | 1 |
| NT2NE2005968 | 0.006072383 | 42688 | 1 |
| NT2NE2006075 | 0.021270419 | 208725 | 5 |
| NT2NE2006087 | 0.016507765 | 124208 | 4 |
| NT2NE2006103 | 0.019351688 | 155724 | 4 |
| NT2NE2006217 | 0.006072383 | 249352 | 1 |
| NT2NE2006288 | 0.006072383 | 104497 | 1 |
| NT2NE2006382 | 0.006072383 | 259217 | 1 |
| NT2NE2006387 | 0.006072383 | 260624 | 1 |
| NT2NE2006427 | 0.009117264 | 69474 | 2 |
| NT2NE2006432 | 0.006072383 | 253576 | 1 |
| NT2NE2006458 | 0.118540551 | 154135 | 13 |
| NT2NE2006478 | 0.015594036 | 96791 | 6 |
| NT2NE2006531 | 0.006072383 | 257218 | 1 |
| NT2NE2006659 | 0.009260029 | 134874 | 3 |
| NT2NE2006784 | 0.006072383 | 254058 | 1 |
| NT2NE2006813 | 0.006072383 | 284911 | 1 |
| NT2NE2006894 | 0.006072383 | 258203 | 1 |
| NT2NE2006909 | 0.587286909 | 58210 | 14 |
| NT2NE2006942 | 0.006072383 | 249199 | 1 |
| NT2NE2006958 | 0.010249318 | 129910 | 2 |
| NT2NE2007052 | 0.006072383 | 235608 | 1 |
| NT2NE2007220 | 0.006072383 | 150744 | 1 |
| NT2NE2007425 | 0.006072383 | 161080 | 1 |
| NT2NE2007725 | 0.006072383 | 70715 | 1 |
| NT2NE2007727 | 0.027386554 | 151577 | 8 |
| NT2NE2007786 | 0.04205655 | 164480 | 2 |
| NT2NE2007877 | 0.012144766 | 151701 | 2 |
| NT2NE2007967 | 0.006072383 | 105635 | 1 |
| NT2NE2008017 | 0.006072383 | 264849 | 1 |
| NT2NE2008060 | 0.008153895 | 280285 | 2 |
| NT2NE2008077 | 0.017197091 | 224642 | 2 |
| NT2NE2008213 | 0.020134968 | 180367 | 4 |
| NT2NE2008260 | 0.006072383 | 150833 | 1 |
| NT2NE2008378 | 0.006072383 | 265591 | 1 |
| NT2NE2008607 | 0.006072383 | 258913 | 1 |
| NT2NE2008639 | 0.006072383 | 223459 | 1 |
| NT2NE2008727 | 0.006072383 | 161289 | 1 |
| NT2NE2008783 | 0.006072383 | 260596 | 1 |
| NT2NE2008785 | 0.017859276 | 282541 | 2 |
| NT2NE2008803 | 0.007261627 | 162623 | 2 |
| NT2NE2008867 | 0.006072383 | 277149 | 1 |
| NT2NE2008961 | 0.012144766 | 243285 | 2 |
| NT2NE2008997 | 0.006072383 | 19170 | 1 |
| NT2NE2009138 | 0.006072383 | 255513 | 1 |
| NT2NE2009295 | 0.083985743 | 46492 | 9 |
| NT2NE2009426 | 0.006072383 | 75879 | 1 |
| NT2NE2009523 | 0.021562885 | 149685 | 5 |
| NT2NE2009608 | 0.006072383 | 18833 | 1 |
| NT2NE2009691 | 0.006072383 | 167636 | 1 |
| NT2NE2010056 | 0.020909833 | 29945 | 9 |
| NT2NE2010105 | 0.006072383 | 150524 | 1 |
| NT2NE2010150 | 0.006072383 | 164442 | 1 |
| NT2NE2010400 | 0.006072383 | 175757 | 1 |
| NT2NE2010632 | 0.006072383 | 63318 | 1 |
| NT2NE2010633 | 0.006072383 | 154412 | 1 |
| NT2NE2010654 | 0.006072383 | 167651 | 1 |
| NT2NE2010688 | 0.00774793 | 143496 | 2 |
| NT2NE2010781 | 0.006072383 | 227047 | 1 |
| NT2NE2010842 | 0.177706809 | 109238 | 41 |
| NT2NE2010854 | 0.007167661 | 126577 | 2 |
| NT2NE2011033 | 0.009167879 | 112056 | 2 |
| NT2NE2011036 | 0.012144766 | 179262 | 2 |
| NT2NE2011107 | 0.013932163 | 158385 | 7 |
| NT2NE2011119 | 0.036186709 | 125008 | 7 |
| NT2NE2011154 | 0.006072383 | 54552 | 1 |
| NT2NE2011221 | 0.011646726 | 91779 | 4 |
| NT2NE2011411 | 0.006072383 | 102565 | 1 |
| NT2NE2011485 | 0.008843208 | 130445 | 3 |
| NT2NE2011650 | 0.009117264 | 23794 | 2 |
| NT2NE2011684 | 0.019134034 | 197356 | 2 |
| NT2NE2011687 | 0.006072383 | 185793 | 1 |
| NT2NE2011691 | 0.006072383 | 127863 | 1 |
| NT2NE2011758 | 0.006072383 | 102320 | 1 |
| NT2NE2011823 | 0.006072383 | 29687 | 1 |
| NT2NE2011896 | 0.006072383 | 59554 | 1 |
| NT2NE2011998 | 0.008153895 | 81516 | 2 |
| NT2NE2012113 | 0.006072383 | 161166 | 1 |
| NT2NE2012199 | 0.013833042 | 163215 | 3 |
| NT2NE2012243 | 0.007930497 | 193548 | 2 |
| NT2NE2012361 | 0.006072383 | 56528 | 1 |
| NT2NE2012448 | 0.006072383 | 55032 | 1 |
| NT2NE2012457 | 0.006072383 | 239597 | 1 |
| NT2NE2012493 | 0.006072383 | 48388 | 1 |
| NT2NE2012505 | 0.009138469 | 109045 | 3 |
| NT2NE2012533 | 0.006072383 | 109166 | 1 |
| NT2NE2012603 | 0.006072383 | 50635 | 1 |
| NT2NE2012747 | 0.006072383 | 19081 | 1 |
| NT2NE2012790 | 0.02793118 | 65056 | 5 |
| NT2NE2012847 | 0.006072383 | 210723 | 1 |
| NT2NE2012928 | 0.019245317 | 98772 | 4 |
| NT2NE2013019 | 0.009117264 | 156238 | 2 |
| NT2NE2013081 | 0.006072383 | 161376 | 1 |
| NT2NE2013189 | 0.006072383 | 93664 | 1 |
| NT2NE2013217 | 0.006072383 | 137391 | 1 |
| NT2NE2013501 | 0.110236555 | 92380 | 37 |
| NT2NE2013547 | 0.006072383 | 97970 | 1 |
| NT2NE2014013 | 0.006072383 | 75501 | 1 |
| NT2NE2014028 | 0.006072383 | 133098 | 1 |
| NT2NE2014104 | 0.099827368 | 52557 | 14 |
| NT2NE2014113 | 0.018465695 | 183524 | 4 |
| NT2NE2014176 | 0.012162146 | 109003 | 3 |
| NT2NE2014221 | 0.006072383 | 183651 | 1 |
| NT2NE2014525 | 0.006072383 | 3986 | 1 |
| NT2NE2014650 | 0.006072383 | 37833 | 1 |
| NT2NE2014651 | 0.006072383 | 207671 | 1 |
| NT2NE2014681 | 0.006072383 | 189721 | 1 |
| NT2NE2014758 | 0.006072383 | 33535 | 1 |
| NT2NE2014869 | 0.006072383 | 283898 | 1 |
| NT2NE2014950 | 0.006072383 | 192405 | 1 |
| NT2NE2015061 | 0.006072383 | 161172 | 1 |
| NT2NE2015262 | 0.007469538 | 151576 | 2 |
| NT2NE2015275 | 0.006072383 | 191366 | 1 |
| NT2NE2015362 | 0.009138469 | 116784 | 3 |
| NT2NE2015480 | 0.006072383 | 52825 | 1 |
| NT2NE2015511 | 0.006072383 | 237414 | 1 |
| NT2NE2015565 | 0.006072383 | 129902 | 1 |
| NT2NE2015626 | 0.006072383 | 127866 | 1 |
| NT2NE2015712 | 0.133701175 | 71102 | 22 |
| NT2NE2015747 | 0.006072383 | 54689 | 1 |
| NT2NE2015860 | 0.009117264 | 190403 | 2 |
| NT2NE2015885 | 0.006072383 | 151613 | 1 |
| NT2NE2015904 | 0.007781843 | 149472 | 2 |
| NT2NE2015974 | 0.021934332 | 54112 | 11 |
| NT2NE2016041 | 0.006072383 | 62695 | 1 |
| NT2NE2016387 | 0.00774793 | 51954 | 2 |
| NT2NE2016419 | 0.006072383 | 221100 | 1 |
| NT2NE2016519 | 0.006072383 | 104646 | 1 |
| NT2NE2016608 | 0.006072383 | 65079 | 1 |
| NT2NE2016766 | 0.006072383 | 173601 | 1 |
| NT2NE2016774 | 0.006072383 | 189704 | 1 |
| NT2NE2016971 | 0.012238071 | 54418 | 3 |
| NT2NE2017259 | 0.006072383 | 60055 | 1 |
| NT2NE2017332 | 0.006072383 | 141459 | 1 |
| NT2NE2017397 | 0.006072383 | 131867 | 1 |
| NT2NE2017480 | 0.010110666 | 167627 | 2 |
| NT2NE2017492 | 0.009928898 | 233573 | 3 |
| NT2NE2017508 | 0.006072383 | 159969 | 1 |
| NT2NE2017562 | 0.006072383 | 179655 | 1 |
| NT2NE2017590 | 0.006072383 | 194254 | 1 |
| NT2NE2017612 | 0.006072383 | 175656 | 1 |
| NT2NE2017721 | 0.006072383 | 161552 | 1 |
| NT2NE2017752 | 0.01427369 | 106435 | 4 |
| NT2NE2017792 | 0.008949904 | 173685 | 3 |
| NT2NE2017982 | 0.007167661 | 180775 | 2 |
| NT2NE2018165 | 0.006072383 | 126253 | 1 |
| NT2NE2018176 | 0.006072383 | 86243 | 1 |
| NT2NE2018180 | 0.044561852 | 167633 | 9 |
| NT2NE2018273 | 0.006072383 | 75823 | 1 |
| NT2NE2018376 | 0.006072383 | 173719 | 1 |
| NT2NE2018472 | 0.006072383 | 179874 | 1 |
| NT2NE2018490 | 0.007167661 | 130179 | 2 |
| NT2NE2018594 | 0.009916467 | 172593 | 2 |
| NT2NE2018739 | 0.006072383 | 124942 | 1 |
| NT2NE2018916 | 0.012144766 | 58507 | 2 |
| NT2NE2019056 | 0.006072383 | 175858 | 1 |
| NT2NE2019076 | 0.018595864 | 175854 | 2 |
| NT2NE2019091 | 0.088507346 | 126422 | 18 |
| NT2NE2019099 | 0.006072383 | 27233 | 1 |
| NT2RI1000016 | 0.006631183 | 63833 | 3 |
| NT2RI1000027 | 0.165718781 | 50916 | 17 |
| NT2RI1000035 | 0.00885006 | 61879 | 2 |
| NT2RI1000048 | 0.197336203 | 64352 | 17 |
| NT2RI1000127 | 0.092317238 | 76270 | 19 |
| NT2RI1000164 | 0.003044882 | 56827 | 1 |
| NT2RI1000172 | 0.013155547 | 78304 | 3 |
| NT2RI2000004 | 0.003044882 | 270754 | 1 |
| NT2RI2000007 | 0.005043283 | 134288 | 2 |
| NT2RI2000064 | 0.311298378 | 84067 | 46 |
| NT2RI2000107 | 0.072337994 | 110490 | 17 |
| NT2RI2000116 | 0.288108079 | 35941 | 124 |
| NT2RI2000117 | 0.003044882 | 52717 | 1 |
| NT2RI2000128 | 0.003044882 | 67613 | 1 |
| NT2RI2000133 | 0.065392303 | 134275 | 5 |
| NT2RI2000167 | 0.014503052 | 48732 | 5 |
| NT2RI2000173 | 0.007083164 | 262237 | 2 |
| NT2RI2000187 | 0.003044882 | 262236 | 1 |
| NT2RI2000255 | 0.139986191 | 34452 | 19 |
| NT2RI2000263 | 0.097952942 | 8609 | 20 |
| NT2RI2000270 | 0.003044882 | 139254 | 1 |
| NT2RI2000276 | 0.003044882 | 66761 | 1 |
| NT2RI2000282 | 0.529374764 | 79584 | 53 |
| NT2RI2000284 | 0.367548357 | 94056 | 35 |
| NT2RI2000294 | 0.088528718 | 107318 | 22 |
| NT2RI2000313 | 0.009164676 | 145861 | 3 |
| NT2RI2000341 | 0.003044882 | 19244 | 1 |
| NT2RI2000344 | 0.206956181 | 11149 | 15 |
| NT2RI2000348 | 0.018908463 | 72638 | 8 |
| NT2RI2000412 | 0.021738892 | 124256 | 10 |
| NT2RI2000421 | 0.055063444 | 108331 | 5 |
| NT2RI2000480 | 0.003044882 | 244831 | 1 |
| NT2RI2000575 | 0.003044882 | 149159 | 1 |
| NT2RI2000578 | 0.501074101 | 63367 | 31 |
| NT2RI2000588 | 0.003044882 | 129730 | 1 |
| NT2RI2000597 | 0.003044882 | 41065 | 1 |
| NT2RI2000669 | 0.013647087 | 122047 | 2 |
| NT2RI2000671 | 0.159298608 | 138197 | 20 |
| NT2RI2000685 | 0.169117153 | 30433 | 51 |
| NT2RI2000688 | 0.003044882 | 136901 | 1 |
| NT2RI2000689 | 0.10836064 | 134628 | 14 |
| NT2RI2000704 | 0.003044882 | 101241 | 1 |
| NT2RI2000727 | 0.647670214 | 18625 | 92 |
| NT2RI2000738 | 0.003044882 | 266131 | 1 |
| NT2RI2000775 | 0.009824719 | 207766 | 4 |
| NT2RI2000822 | 0.015728719 | 171711 | 3 |
| NT2RI2000829 | 0.043869045 | 133120 | 14 |
| NT2RI2000841 | 0.003044882 | 259233 | 1 |
| NT2RI2000865 | 0.059734224 | 157220 | 2 |
| NT2RI2000932 | 0.003044882 | 33427 | 1 |
| NT2RI2000974 | 0.07767935 | 144388 | 17 |
| NT2RI2000987 | 0.003044882 | 164859 | 1 |
| NT2RI2001010 | 0.004442037 | 155313 | 2 |
| NT2RI2001017 | 0.003044882 | 110452 | 1 |
| NT2RI2001030 | 0.005126394 | 127086 | 2 |
| NT2RI2001054 | 0.281610305 | 101025 | 26 |
| NT2RI2001083 | 0.003044882 | 57062 | 1 |
| NT2RI2001091 | 0.047658284 | 113969 | 8 |
| NT2RI2001167 | 0.00414016 | 240721 | 2 |
| NT2RI2001178 | 0.003044882 | 271855 | 1 |
| NT2RI2001230 | 0.217223438 | 79682 | 16 |
| NT2RI2001235 | 0.003044882 | 266711 | 1 |
| NT2RI2001244 | 0.003044882 | 179531 | 1 |
| NT2RI2001307 | 0.003044882 | 262280 | 1 |
| NT2RI2001342 | 0.106154394 | 12062 | 10 |
| NT2RI2001385 | 0.042528621 | 91061 | 7 |
| NT2RI2001409 | 0.014549714 | 151891 | 2 |
| NT2RI2001410 | 0.003044882 | 47821 | 1 |
| NT2RI2001422 | 0.045071428 | 10964 | 8 |
| NT2RI2001425 | 0.003044882 | 260083 | 1 |
| NT2RI2001449 | 0.029390737 | 61372 | 9 |
| NT2RI2001450 | 0.094565248 | 110620 | 38 |
| NT2RI2001474 | 0.003044882 | 265130 | 1 |
| NT2RI2001519 | 0.003044882 | 85438 | 1 |
| NT2RI2001540 | 2.943484976 | 18066 | 326 |
| NT2RI2001595 | 0.021101854 | 72422 | 5 |
| NT2RI2001621 | 0.054737539 | 171697 | 14 |
| NT2RI2001624 | 0.003044882 | 199443 | 1 |
| NT2RI2001657 | 0.31315763 | 34843 | 53 |
| NT2RI2001726 | 0.015868295 | 138336 | 7 |
| NT2RI2001731 | 0.088472215 | 106604 | 16 |
| NT2RI2001769 | 0.075374793 | 65315 | 7 |
| NT2RI2001782 | 0.003044882 | 266659 | 1 |
| NT2RI2001836 | 0.003044882 | 92674 | 1 |
| NT2RI2001846 | 0.034002249 | 127692 | 8 |
| NT2RI2001859 | 0.172666004 | 144954 | 42 |
| NT2RI2001866 | 0.011546406 | 108092 | 5 |
| NT2RI2001910 | 0.022433577 | 108147 | 7 |
| NT2RI2001952 | 0.007998492 | 146188 | 3 |
| NT2RI2002022 | 0.005126394 | 107272 | 2 |
| NT2RI2002041 | 0.003044882 | 266360 | 1 |
| NT2RI2002091 | 12.49272458 | 2604 | 1148 |
| NT2RI2002117 | 0.035566328 | 116219 | 7 |
| NT2RI2002120 | 0.021314171 | 169692 | 7 |
| NT2RI2002152 | 0.133240831 | 38406 | 19 |
| NT2RI2002153 | 0.003044882 | 264222 | 1 |
| NT2RI2002179 | 0.003044882 | 173643 | 1 |
| NT2RI2002211 | 0.403797085 | 70462 | 140 |
| NT2RI2002243 | 0.050938581 | 177528 | 6 |
| NT2RI2002252 | 0.003044882 | 214288 | 1 |
| NT2RI2002260 | 0.007083164 | 53369 | 2 |
| NT2RI2002266 | 0.066853669 | 97891 | 9 |
| NT2RI2002270 | 0.003044882 | 133802 | 1 |
| NT2RI2002316 | 0.208991284 | 114022 | 30 |
| NT2RI2002334 | 0.003044882 | 267590 | 1 |
| NT2RI2002359 | 0.020359769 | 133183 | 4 |
| NT2RI2002391 | 0.206616486 | 90765 | 34 |
| NT2RI2002447 | 0.003044882 | 276180 | 1 |
| NT2RI2002481 | 0.003044882 | 214460 | 1 |
| NT2RI2002507 | 0.003044882 | 274438 | 1 |
| NT2RI2002517 | 0.007083164 | 175210 | 2 |
| NT2RI2002530 | 0.02775887 | 50487 | 6 |
| NT2RI2002540 | 0.096926156 | 104804 | 29 |
| NT2RI2002541 | 0.095062189 | 112662 | 25 |
| NT2RI2002549 | 0.024319054 | 27726 | 6 |
| NT2RI2002554 | 0.092407576 | 15000 | 23 |
| NT2RI2002564 | 0.003044882 | 114625 | 1 |
| NT2RI2002585 | 0.012887308 | 128115 | 9 |
| NT2RI2002592 | 0.009086448 | 71443 | 2 |
| NT2RI2002602 | 0.021396088 | 162258 | 5 |
| NT2RI2002654 | 0.285223786 | 92848 | 41 |
| NT2RI2002699 | 0.014654697 | 136900 | 5 |
| NT2RI2002729 | 0.079664769 | 105954 | 6 |
| NT2RI2002802 | 0.005828436 | 176599 | 3 |
| NT2RI2002819 | 0.045005688 | 13987 | 11 |
| NT2RI2002822 | 0.033395273 | 113877 | 11 |
| NT2RI2002847 | 0.133480395 | 82109 | 35 |
| NT2RI2002852 | 0.00414016 | 251097 | 2 |
| NT2RI2002865 | 0.024710287 | 126630 | 6 |
| NT2RI2002923 | 0.003044882 | 266191 | 1 |
| NT2RI2002926 | 0.021231213 | 179949 | 4 |
| NT2RI2002958 | 0.17403637 | 82780 | 52 |
| NT2RI2002970 | 0.003044882 | 221862 | 1 |
| NT2RI2003011 | 0.048526964 | 188873 | 6 |
| NT2RI2003019 | 0.032037681 | 154019 | 9 |
| NT2RI2003051 | 0.16367109 | 78501 | 74 |
| NT2RI2003067 | 0.003044882 | 267074 | 1 |
| NT2RI2003154 | 0.008025098 | 150129 | 4 |
| NT2RI2003184 | 0.008886344 | 92677 | 2 |
| NT2RI2003205 | 0.052050932 | 70523 | 15 |
| NT2RI2003220 | 0.098656868 | 105048 | 44 |
| NT2RI2003222 | 0.006591273 | 144534 | 3 |
| NT2RI2003301 | 0.029521302 | 79538 | 13 |
| NT2RI2003304 | 0.638497477 | 73979 | 90 |
| NT2RI2003317 | 0.106297246 | 65520 | 35 |
| NT2RI2003338 | 0.165480193 | 75398 | 41 |
| NT2RI2003344 | 0.086041944 | 62148 | 55 |
| NT2RI2003360 | 0.003044882 | 988 | 1 |
| NT2RI2003361 | 0.033927008 | 28116 | 9 |
| NT2RI2003383 | 0.009235874 | 58084 | 3 |
| NT2RI2003392 | 0.003044882 | 265084 | 1 |
| NT2RI2003399 | 0.005235438 | 97734 | 3 |
| NT2RI2003407 | 0.138438197 | 108306 | 20 |
| NT2RI2003419 | 0.014842899 | 156419 | 2 |
| NT2RI2003420 | 0.108807576 | 73396 | 34 |
| NT2RI2003481 | 0.086771745 | 55408 | 21 |
| NT2RI2003487 | 0.044850902 | 223149 | 2 |
| NT2RI2003556 | 0.440412738 | 138145 | 29 |
| NT2RI2003655 | 0.008577242 | 97073 | 3 |
| NT2RI2003667 | 0.009117264 | 211893 | 2 |
| NT2RI2003678 | 0.088002754 | 136314 | 21 |
| NT2RI2003695 | 0.212783955 | 88709 | 47 |
| NT2RI2003738 | 0.957797157 | 77246 | 98 |
| NT2RI2003741 | 0.361548428 | 88362 | 93 |
| NT2RI2003751 | 0.12095225 | 98060 | 16 |
| NT2RI2003884 | 0.003044882 | 264344 | 1 |
| NT2RI2003921 | 0.35330779 | 57124 | 36 |
| NT2RI2003973 | 0.013348283 | 146329 | 5 |
| NT2RI2003993 | 0.044136676 | 168309 | 8 |
| NT2RI2004059 | 0.024589442 | 126823 | 5 |
| NT2RI2004078 | 0.004720429 | 175319 | 2 |
| NT2RI2004079 | 0.003044882 | 167611 | 1 |
| NT2RI2004093 | 0.006089763 | 69274 | 2 |
| NT2RI2004136 | 0.003044882 | 269521 | 1 |
| NT2RI2004157 | 0.005043283 | 102109 | 2 |
| NT2RI2004188 | 0.058353236 | 74415 | 9 |
| NT2RI2004190 | 0.003044882 | 159678 | 1 |
| NT2RI2004209 | 0.022822697 | 115204 | 5 |
| NT2RI2004230 | 0.1460284 | 100022 | 42 |
| NT2RI2004284 | 0.104236124 | 154196 | 6 |
| NT2RI2004290 | 0.007853965 | 86522 | 3 |
| NT2RI2004304 | 0.040236186 | 83227 | 21 |
| NT2RI2004312 | 0.011038656 | 160856 | 3 |
| NT2RI2004381 | 0.003044882 | 262480 | 1 |
| NT2RI2004398 | 0.033633816 | 94823 | 11 |
| NT2RI2004410 | 0.067181817 | 109096 | 24 |
| NT2RI2004442 | 0.003044882 | 89749 | 1 |
| NT2RI2004468 | 0.003044882 | 241362 | 1 |
| NT2RI2004504 | 0.596981765 | 61702 | 163 |
| NT2RI2004535 | 0.087535334 | 88770 | 28 |
| NT2RI2004606 | 0.004720429 | 73678 | 2 |
| NT2RI2004608 | 0.045288383 | 92709 | 10 |
| NT2RI2004618 | 0.003044882 | 277304 | 1 |
| NT2RI2004783 | 0.00776531 | 136794 | 3 |
| NT2RI2004818 | 0.003044882 | 137370 | 1 |
| NT2RI2004840 | 0.003044882 | 219054 | 1 |
| NT2RI2004884 | 0.216864488 | 98742 | 17 |
| NT2RI2004916 | 0.010128046 | 156110 | 3 |
| NT2RI2004962 | 0.003044882 | 101242 | 1 |
| NT2RI2004984 | 0.003044882 | 35529 | 1 |
| NT2RI2004985 | 0.003044882 | 130525 | 1 |
| NT2RI2005048 | 0.003044882 | 161917 | 1 |
| NT2RI2005061 | 0.03841027 | 148193 | 10 |
| NT2RI2005069 | 0.003044882 | 170390 | 1 |
| NT2RI2005087 | 0.039080918 | 141352 | 2 |
| NT2RI2005096 | 0.00414016 | 111223 | 2 |
| NT2RI2005115 | 0.027419703 | 105208 | 7 |
| NT2RI2005116 | 0.131078745 | 119430 | 26 |
| NT2RI2005130 | 0.012162146 | 167607 | 3 |
| NT2RI2005150 | 0.243160742 | 17820 | 42 |
| NT2RI2005166 | 0.006424683 | 180327 | 4 |
| NT2RI2005239 | 0.042487094 | 85121 | 15 |
| NT2RI2005246 | 0.01804796 | 15530 | 3 |
| NT2RI2005315 | 0.006165688 | 81983 | 2 |
| NT2RI2005335 | 0.432856271 | 23512 | 133 |
| NT2RI2005353 | 0.14545991 | 114168 | 22 |
| NT2RI2005358 | 0.008164089 | 157041 | 3 |
| NT2RI2005368 | 0.082926386 | 119652 | 16 |
| NT2RI2005382 | 0.00847057 | 77602 | 4 |
| NT2RI2005405 | 0.596878174 | 87082 | 73 |
| NT2RI2005473 | 0.051301787 | 243301 | 3 |
| NT2RI2005520 | 0.003044882 | 3204 | 1 |
| NT2RI2005564 | 0.037618515 | 61213 | 15 |
| NT2RI2005579 | 0.01785392 | 70182 | 6 |
| NT2RI2005621 | 0.003044882 | 189481 | 1 |
| NT2RI2005628 | 0.003044882 | 185344 | 1 |
| NT2RI2005647 | 0.253382024 | 4698 | 26 |
| NT2RI2005670 | 0.003044882 | 128365 | 1 |
| NT2RI2005710 | 0.032099741 | 53296 | 8 |
| NT2RI2005713 | 0.113302567 | 93611 | 26 |
| NT2RI2005723 | 0.333894126 | 113962 | 63 |
| NT2RI2005772 | 0.009134645 | 165778 | 3 |
| NT2RI2005811 | 0.109366709 | 159985 | 20 |
| NT2RI2005814 | 0.128650226 | 98883 | 11 |
| NT2RI2005816 | 0.026214124 | 32105 | 11 |
| NT2RI2005818 | 0.065786717 | 67172 | 20 |
| NT2RI2005851 | 0.044962203 | 121097 | 16 |
| NT2RI2005966 | 0.008987005 | 281478 | 2 |
| NT2RI2006070 | 0.08152819 | 134910 | 14 |
| NT2RI2006071 | 0.059355294 | 30415 | 42 |
| NT2RI2006072 | 0.218997109 | 166427 | 26 |
| NT2RI2006081 | 0.003044882 | 279262 | 1 |
| NT2RI2006126 | 0.003044882 | 151537 | 1 |
| NT2RI2006127 | 0.013872079 | 187634 | 2 |
| NT2RI2006183 | 0.017518546 | 186256 | 5 |
| NT2RI2006210 | 0.028243819 | 145027 | 5 |
| NT2RI2006257 | 0.003044882 | 277940 | 1 |
| NT2RI2006271 | 0.059990396 | 150039 | 6 |
| NT2RI2006294 | 0.003044882 | 61397 | 1 |
| NT2RI2006345 | 0.003044882 | 57811 | 1 |
| NT2RI2006403 | 0.003044882 | 191348 | 1 |
| NT2RI2006412 | 0.204407794 | 70407 | 65 |
| NT2RI2006445 | 0.003044882 | 12994 | 1 |
| NT2RI2006487 | 0.003044882 | 194241 | 1 |
| NT2RI2006506 | 0.012437974 | 161711 | 5 |
| NT2RI2006553 | 0.045302344 | 154796 | 7 |
| NT2RI2006565 | 0.028040899 | 145153 | 8 |
| NT2RI2006640 | 0.026659501 | 99387 | 10 |
| NT2RI2006647 | 0.036540964 | 135251 | 10 |
| NT2RI2006659 | 0.007083164 | 210389 | 2 |
| NT2RI2006662 | 0.003044882 | 95367 | 1 |
| NT2RI2006667 | 0.003044882 | 276968 | 1 |
| NT2RI2006679 | 0.003044882 | 71261 | 1 |
| NT2RI2006682 | 0.068092458 | 132383 | 18 |
| NT2RI2006686 | 0.014331563 | 193529 | 2 |
| NT2RI2006703 | 0.010338434 | 195054 | 4 |
| NT2RI2006716 | 0.003044882 | 234816 | 1 |
| NT2RI2006735 | 0.010645973 | 126312 | 5 |
| NT2RI2006762 | 0.003044882 | 277525 | 1 |
| NT2RI2006788 | 0.003044882 | 76851 | 1 |
| NT2RI2006825 | 0.061115679 | 112900 | 17 |
| NT2RI2006838 | 0.003044882 | 235587 | 1 |
| NT2RI2006853 | 0.074701306 | 152329 | 13 |
| NT2RI2006855 | 0.019436977 | 33831 | 5 |
| NT2RI2006856 | 0.357722292 | 10092 | 37 |
| NT2RI2006943 | 0.137764997 | 105442 | 29 |
| NT2RI2006973 | 0.003044882 | 66509 | 1 |
| NT2RI2007046 | 0.003044882 | 121712 | 1 |
| NT2RI2007048 | 0.003044882 | 178696 | 1 |
| NT2RI2007054 | 0.129060942 | 141191 | 27 |
| NT2RI2007084 | 0.154013668 | 134319 | 19 |
| NT2RI2007096 | 0.03232221 | 135052 | 13 |
| NT2RI2007116 | 0.003044882 | 277095 | 1 |
| NT2RI2007133 | 0.427118898 | 18766 | 23 |
| NT2RI2007148 | 0.177422997 | 122768 | 10 |
| NT2RI2007214 | 0.007083164 | 152627 | 2 |
| NT2RI2007254 | 0.212783955 | 88709 | 47 |
| NT2RI2007277 | 0.170915185 | 83693 | 51 |
| NT2RI2007303 | 0.003044882 | 269437 | 1 |
| NT2RI2007334 | 0.013280288 | 99955 | 4 |
| NT2RI2007377 | 0.143065284 | 15432 | 46 |
| NT2RI2007384 | 0.386921652 | 44065 | 46 |
| NT2RI2007386 | 0.003044882 | 222351 | 1 |
| NT2RI2007406 | 0.009117264 | 225482 | 2 |
| NT2RI2007439 | 0.009117264 | 166218 | 2 |
| NT2RI2007445 | 0.093447469 | 132737 | 15 |
| NT2RI2007468 | 0.040101278 | 118435 | 12 |
| NT2R12007469 | 0.005235438 | 166352 | 3 |
| NT2RI2007498 | 0.410872964 | 105083 | 43 |
| NT2RI2007507 | 0.003044882 | 274289 | 1 |
| NT2RI2007572 | 0.224251358 | 125514 | 49 |
| NT2RI2007589 | 0.156897572 | 106862 | 30 |
| NT2RI2007593 | 0.050855316 | 164367 | 12 |
| NT2RI2007629 | 0.083120959 | 104318 | 19 |
| NT2RI2007723 | 0.047362479 | 12494 | 9 |
| NT2RI2007729 | 0.014508827 | 174315 | 2 |
| NT2RI2007751 | 0.003044882 | 143596 | 1 |
| NT2RI2007754 | 0.017267943 | 146783 | 3 |
| NT2RI2007827 | 0.022319642 | 54119 | 3 |
| NT2RI2007853 | 0.07483736 | 121114 | 6 |
| NT2RI2007877 | 0.003044882 | 281851 | 1 |
| NT2RI2007879 | 0.105570783 | 7840 | 8 |
| NT2RI2007884 | 0.065999801 | 132268 | 17 |
| NT2RI2007891 | 0.003044882 | 104677 | 1 |
| NT2RI2007956 | 0.003044882 | 165126 | 1 |
| NT2RI2007965 | 0.007083164 | 123285 | 2 |
| NT2RI2007987 | 0.093058368 | 114503 | 27 |
| NT2RI2008007 | 0.008657175 | 31356 | 3 |
| NT2RI2008045 | 0.215801178 | 81138 | 33 |
| NT2RI2008050 | 0.003044882 | 238265 | 1 |
| NT2RI2008141 | 0.078359933 | 2764 | 20 |
| NT2RI2008171 | 0.037735551 | 145131 | 9 |
| NT2RI2008188 | 0.005828436 | 103711 | 3 |
| NT2RI2008204 | 0.026996023 | 156829 | 4 |
| NT2RI2008221 | 0.151134943 | 47257 | 5 |
| NT2RI2008233 | 0.00885006 | 124666 | 2 |
| NT2RI2008336 | 0.418255567 | 63559 | 57 |
| NT2RI2008396 | 0.380124981 | 54223 | 72 |
| NT2RI2008455 | 0.003044882 | 261425 | 1 |
| NT2RI2008481 | 0.226557321 | 63654 | 18 |
| NT2RI2008502 | 0.003044882 | 276425 | 1 |
| NT2RI2008526 | 0.218544249 | 12110 | 22 |
| NT2RI2008566 | 0.017814449 | 161829 | 8 |
| NT2RI2008598 | 0.019974768 | 77898 | 3 |
| NT2RI2008622 | 0.053094151 | 148594 | 9 |
| NT2RI2008656 | 0.061695007 | 172428 | 6 |
| NT2RI2008714 | 0.011419083 | 106996 | 5 |
| NT2RI2008724 | 0.091237502 | 70597 | 17 |
| NT2RI2008749 | 0.296348512 | 127582 | 27 |
| NT2RI2008791 | 0.143610146 | 66082 | 21 |
| NT2RI2008801 | 0.003044882 | 100350 | 1 |
| NT2RI2008808 | 0.036961263 | 90643 | 13 |
| NT2RI2008812 | 0.023144058 | 178312 | 5 |
| NT2RI2008841 | 0.003044882 | 280095 | 1 |
| NT2RI2008942 | 0.159783144 | 96198 | 59 |
| NT2RI2008952 | 0.003044882 | 184776 | 1 |
| NT2RI2009005 | 0.003044882 | 150884 | 1 |
| NT2RI2009037 | 0.198750868 | 102294 | 48 |
| NT2RI2009065 | 0.358761545 | 113793 | 52 |
| NT2RI2009066 | 0.061133341 | 123911 | 23 |
| NT2RI2009083 | 0.016341141 | 91711 | 9 |
| NT2RI2009101 | 0.003044882 | 271187 | 1 |
| NT2RI2009103 | 0.005235438 | 74366 | 3 |
| NT2RI2009144 | 0.003044882 | 173644 | 1 |
| NT2RI2009151 | 0.003044882 | 165498 | 1 |
| NT2RI2009173 | 0.150224077 | 132145 | 22 |
| NT2RI2009194 | 0.170496847 | 72698 | 29 |
| NT2RI2009215 | 0.003044882 | 197964 | 1 |
| NT2RI2009233 | 0.074284106 | 135421 | 23 |
| NT2RI2009239 | 0.119877738 | 49116 | 16 |
| NT2RI2009259 | 0.01431663 | 63363 | 3 |
| NT2RI2009269 | 0.169558506 | 123881 | 38 |
| NT2RI2009281 | 0.119052761 | 130866 | 21 |
| NT2RI2009289 | 0.003044882 | 109857 | 1 |
| NT2RI2009301 | 0.003044882 | 217643 | 1 |
| NT2RI2009402 | 0.053422391 | 86534 | 20 |
| NT2RI2009406 | 0.059555623 | 110874 | 17 |
| NT2RI2009517 | 0.003044882 | 84334 | 1 |
| NT2RI2009583 | 0.748889309 | 20637 | 112 |
| NT2RI2009585 | 0.003044882 | 146048 | 1 |
| NT2RI2009595 | 0.003044882 | 133190 | 1 |
| NT2RI2009855 | 0.006089763 | 194418 | 2 |
| NT2RI2010283 | 0.022640858 | 133082 | 4 |
| NT2RI2010508 | 0.003044882 | 67418 | 1 |
| NT2RI2010795 | 0.010158847 | 160033 | 5 |
| NT2RI2011422 | 0.003044882 | 150362 | 1 |
| NT2RI2011450 | 0.00659727 | 176848 | 3 |
| NT2RI2011683 | 0.003044882 | 180964 | 1 |
| NT2RI2011803 | 0.003044882 | 194297 | 1 |
| NT2RI2012350 | 0.418632789 | 144259 | 15 |
| NT2RI2012659 | 0.012354928 | 132403 | 8 |
| NT2RI2012726 | 0.036118115 | 115299 | 17 |
| NT2RI2012990 | 0.012499358 | 119171 | 2 |
| NT2RI2013345 | 0.01771657 | 127779 | 4 |
| NT2RI2013357 | 0.00414016 | 151758 | 2 |
| NT2RI2013373 | 0.003044882 | 211678 | 1 |
| NT2RI2014247 | 0.010985407 | 168213 | 3 |
| NT2RI2014551 | 0.003044882 | 134756 | 1 |
| NT2RI2014683 | 0.003044882 | 17687 | 1 |
| NT2RI2014733 | 0.00671883 | 191281 | 3 |
| NT2RI2015239 | 0.003044882 | 285005 | 1 |
| NT2RI2015342 | 0.111379131 | 57240 | 29 |
| NT2RI2015533 | 0.107813525 | 90441 | 43 |
| NT2RI2016128 | 0.003044882 | 182040 | 1 |
| NT2RI2016134 | 0.003044882 | 233335 | 1 |
| NT2RI2017529 | 0.172691052 | 140179 | 8 |
| NT2RI2018311 | 0.007799223 | 250765 | 3 |
| NT2RI2018363 | 0.003044882 | 126729 | 1 |
| NT2RI2018448 | 0.026610305 | 174603 | 7 |
| NT2RI2018883 | 0.006165688 | 169641 | 2 |
| NT2RI2018950 | 0.003044882 | 149010 | 1 |
| NT2RI2019751 | 0.004234126 | 163732 | 2 |
| NT2RI2019826 | 0.003044882 | 248705 | 1 |
| NT2RI2020390 | 0.003044882 | 226308 | 1 |
| NT2RI2020703 | 0.003044882 | 136587 | 1 |
| NT2RI2021463 | 0.006089763 | 143886 | 2 |
| NT2RI2021625 | 0.008792625 | 42350 | 3 |
| NT2RI2022468 | 0.024995459 | 144876 | 5 |
| NT2RI2022584 | 0.003044882 | 21601 | 1 |
| NT2RI2023303 | 0.003044882 | 233779 | 1 |
| NT2RI2023671 | 0.003044882 | 118766 | 1 |
| NT2RI2024008 | 0.004921723 | 149403 | 2 |
| NT2RI2024313 | 0.011435733 | 146362 | 7 |
| NT2RI2024460 | 0.003044882 | 151439 | 1 |
| NT2RI2024496 | 0.003044882 | 242707 | 1 |
| NT2RI2024935 | 0.03856886 | 144166 | 2 |
| NT2RI2024971 | 0.141887343 | 139080 | 14 |
| NT2RI2025075 | 0.009883213 | 125638 | 5 |
| NT2RI2025255 | 0.004902996 | 209782 | 2 |
| NT2RI2025564 | 0.003044882 | 243380 | 1 |
| NT2RI2025565 | 0.003044882 | 42513 | 1 |
| NT2RI2025909 | 0.003044882 | 69262 | 1 |
| NT2RI2025957 | 0.003044882 | 109316 | 1 |
| NT2RI2025976 | 0.003044882 | 158116 | 1 |
| NT2RI2026758 | 0.003044882 | 150895 | 1 |
| NT2RI2027081 | 0.003044882 | 229968 | 1 |
| NT2RI2027157 | 0.006089763 | 232592 | 2 |
| NT2RI2027224 | 0.004921723 | 270109 | 2 |
| NT2RI2027323 | 0.005043283 | 130031 | 2 |
| NT2RI2027396 | 0.003044882 | 182073 | 1 |
| NT2RI2027484 | 0.003044882 | 259189 | 1 |
| NT2RI2027975 | 0.004234126 | 228832 | 2 |
| NT2RI2028213 | 0.003044882 | 143927 | 1 |
| NT2RI2028222 | 0.003044882 | 283238 | 1 |
| NT2RI2028537 | 0.003044882 | 125227 | 1 |
| NT2RI2028642 | 0.003044882 | 183719 | 1 |
| NT2RI3000174 | 0.004921723 | 136817 | 2 |
| NT2RI3000213 | 0.003044882 | 269708 | 1 |
| NT2RI3000423 | 0.034845616 | 114406 | 15 |
| NT2RI3000622 | 0.019570545 | 77861 | 3 |
| NT2RI3001132 | 0.003044882 | 86569 | 1 |
| NT2RI3001263 | 0.003044882 | 235130 | 1 |
| NT2RI3001279 | 0.003044882 | 189135 | 1 |
| NT2RI3001295 | 0.011362752 | 169855 | 3 |
| NT2RI3001348 | 0.016032144 | 91744 | 3 |
| NT2RI3001445 | 0.026455484 | 184476 | 12 |
| NT2RI3001458 | 0.003044882 | 73885 | 1 |
| NT2RI3001515 | 0.015470709 | 39278 | 8 |
| NT2RI3001573 | 0.003044882 | 145584 | 1 |
| NT2RI3001967 | 0.003044882 | 279800 | 1 |
| NT2RI3001981 | 0.157446218 | 2665 | 33 |
| NT2RI3002303 | 0.003044882 | 198651 | 1 |
| NT2RI3002557 | 0.003044882 | 218158 | 1 |
| NT2RI3002842 | 0.008941279 | 139266 | 3 |
| NT2RI3002892 | 0.095212005 | 86533 | 46 |
| NT2RI3003027 | 0.003044882 | 174130 | 1 |
| NT2RI3003031 | 0.003044882 | 174131 | 1 |
| NT2RI3003095 | 0.003044882 | 152334 | 1 |
| NT2RI3003104 | 0.010711829 | 50141 | 8 |
| NT2RI3003162 | 0.003044882 | 132808 | 1 |
| NT2RI3003362 | 0.003044882 | 197726 | 1 |
| NT2RI3003382 | 0.004234126 | 121782 | 2 |
| NT2RI3003409 | 0.003044882 | 233343 | 1 |
| NT2RI3003738 | 0.00923839 | 125360 | 4 |
| NT2RI3003925 | 0.004754342 | 175982 | 2 |
| NT2RI3004133 | 0.003044882 | 38690 | 1 |
| NT2RI3004161 | 0.035628456 | 162731 | 13 |
| NT2RI3004381 | 0.003044882 | 164379 | 1 |
| NT2RI3004510 | 0.005943586 | 218272 | 3 |
| NT2RI3005202 | 0.003044882 | 130236 | 1 |
| NT2RI3005403 | 0.004902996 | 207252 | 2 |
| NT2RI3005416 | 0.003044882 | 69882 | 1 |
| NT2RI3005724 | 0.028271764 | 141725 | 18 |
| NT2RI3005861 | 0.003044882 | 284680 | 1 |
| NT2RI3005923 | 0.008595672 | 117247 | 4 |
| NT2RI3005928 | 0.006089763 | 67160 | 2 |
| NT2RI3006132 | 0.003044882 | 179692 | 1 |
| NT2RI3006171 | 0.003044882 | 219880 | 1 |
| NT2RI3006284 | 0.080982065 | 124928 | 16 |
| NT2RI3006340 | 0.020518922 | 22737 | 6 |
| NT2RI3006376 | 0.003044882 | 103518 | 1 |
| NT2RI3006666 | 0.008025242 | 173492 | 3 |
| NT2RI3006673 | 0.016789762 | 65471 | 8 |
| NT2RI3006796 | 0.006089763 | 202253 | 2 |
| NT2RI3007065 | 0.003044882 | 163412 | 1 |
| NT2RI3007095 | 0.003044882 | 194435 | 1 |
| NT2RI3007158 | 0.003044882 | 156763 | 1 |
| NT2RI3007167 | 0.003044882 | 114140 | 1 |
| NT2RI3007213 | 0.004234126 | 243335 | 2 |
| NT2RI3007291 | 0.013386563 | 142861 | 8 |
| NT2RI3007443 | 0.003044882 | 206758 | 1 |
| NT2RI3007543 | 0.197485233 | 119874 | 39 |
| NT2RI3007684 | 0.003044882 | 75412 | 1 |
| NT2RI3007757 | 0.013248852 | 139899 | 4 |
| NT2RI3007878 | 0.003044882 | 64176 | 1 |
| NT2RI3007978 | 0.003044882 | 283720 | 1 |
| NT2RI3008055 | 0.00758522 | 194446 | 4 |
| NT2RI3008162 | 0.003044882 | 109056 | 1 |
| NT2RI3008179 | 0.003044882 | 163400 | 1 |
| NT2RI3008228 | 0.003044882 | 281552 | 1 |
| NT2RI3008442 | 0.003044882 | 207022 | 1 |
| NT2RI3008652 | 0.003044882 | 271688 | 1 |
| NT2RI3008697 | 0.003044882 | 32350 | 1 |
| NT2RI3008974 | 0.003044882 | 209444 | 1 |
| NT2RI3009158 | 0.006658773 | 250563 | 2 |
| NT2RI3009480 | 0.004442037 | 136647 | 2 |
| NT2RI3009524 | 0.004720429 | 153188 | 2 |
| NT2RP6000005 | 0.076178169 | 15691 | 9 |
| NT2RP6000008 | 0.010455055 | 54556 | 4 |
| NT2RP6000017 | 0.111988015 | 61289 | 22 |
| NT2RP6000032 | 0.0213659 | 30083 | 4 |
| NT2RP6000033 | 0.040283299 | 61675 | 2 |
| NT2RP6000035 | 0.380124981 | 54223 | 72 |
| NT2RP6000039 | 0.093440803 | 64972 | 30 |
| NT2RP6000043 | 0.04411439 | 70295 | 10 |
| NT2RP6000059 | 0.007724961 | 29351 | 3 |
| NT2RP6000060 | 0.004038283 | 5023 | 1 |
| NT2RP6000072 | 0.10369656 | 43553 | 22 |
| NT2RP6000077 | 0.02606703 | 34972 | 9 |
| NT2RP6000078 | 0.466009153 | 63995 | 158 |
| NT2RP6000085 | 0.036228731 | 5879 | 21 |
| NT2RP6000086 | 0.005227527 | 67000 | 2 |
| NT2RP6000100 | 0.007652175 | 27365 | 2 |
| NT2RP6000109 | 0.087729946 | 67233 | 21 |
| NT2RP6000110 | 0.061189315 | 44496 | 28 |
| NT2RP6000119 | 0.004038283 | 6447 | 1 |
| NT2RP6000123 | 0.428032826 | 65407 | 72 |
| NT2RP6000127 | 0.004038283 | 30409 | 1 |
| NT2RP6000130 | 0.020932399 | 54818 | 4 |
| NT2RP6000140 | 0.004038283 | 868 | 1 |
| NT2RP6000150 | 0.004038283 | 49374 | 1 |
| NT2RP7000041 | 0.004038283 | 269761 | 1 |
| NT2RP7000069 | 0.049742589 | 113385 | 27 |
| NT2RP7000076 | 0.274938982 | 76237 | 55 |
| NT2RP7000086 | 0.013102697 | 52165 | 6 |
| NT2RP7000109 | 0.004038283 | 91775 | 1 |
| NT2RP7000112 | 0.010431807 | 46277 | 4 |
| NT2RP7000173 | 0.301387854 | 83961 | 96 |
| NT2RP7000238 | 0.129438892 | 111656 | 37 |
| NT2RP7000259 | 0.012530034 | 142332 | 5 |
| NT2RP7000271 | 0.05524676 | 120030 | 18 |
| NT2RP7000311 | 0.004038283 | 37611 | 1 |
| NT2RP7000359 | 0.004038283 | 131027 | 1 |
| NT2RP7000364 | 0.004038283 | 154609 | 1 |
| NT2RP7000380 | 0.004038283 | 220361 | 1 |
| NT2RP7000391 | 0.004038283 | 274012 | 1 |
| NT2RP7000425 | 0.008215218 | 185000 | 2 |
| NT2RP7000466 | 0.19248918 | 150521 | 19 |
| NT2RP7000477 | 0.084691945 | 74926 | 28 |
| NT2RP7000579 | 0.00926581 | 114563 | 3 |
| NT2RP7000586 | 0.015250128 | 116473 | 7 |
| NT2RP7000600 | 0.075614279 | 95942 | 38 |
| NT2RP7000624 | 0.038404074 | 113338 | 17 |
| NT2RP7000812 | 0.020998132 | 47819 | 3 |
| NT2RP7000876 | 0.074083011 | 100148 | 16 |
| NT2RP7000906 | 0.128445545 | 36763 | 50 |
| NT2RP7000926 | 0.040382829 | 139609 | 10 |
| NT2RP7001074 | 0.004038283 | 164447 | 1 |
| NT2RP7001080 | 0.050060872 | 52876 | 20 |
| NT2RP7001156 | 0.005133561 | 192119 | 2 |
| NT2RP7001166 | 0.224035746 | 113203 | 58 |
| NT2RP7001231 | 0.040572531 | 95153 | 18 |
| NT2RP7001283 | 0.29768157 | 22378 | 44 |
| NT2RP7001306 | 0.023035709 | 97659 | 3 |
| NT2RP7001319 | 0.004038283 | 99441 | 1 |
| NT2RP7001335 | 0.080201941 | 78877 | 22 |
| NT2RP7001364 | 0.00572656 | 279408 | 2 |
| NT2RP7001475 | 0.009537578 | 133602 | 4 |
| NT2RP7001532 | 0.004038283 | 163280 | 1 |
| NT2RP7001591 | 0.064447731 | 143106 | 7 |
| NT2RP7001602 | 0.004038283 | 266651 | 1 |
| NT2RP7001678 | 0.010772981 | 117859 | 3 |
| NT2RP7001745 | 0.108807576 | 73396 | 34 |
| NT2RP7001856 | 0.048889185 | 107453 | 3 |
| NT2RP7001962 | 0.063075103 | 104839 | 19 |
| NT2RP7002028 | 0.083379247 | 115298 | 15 |
| NT2RP7002064 | 0.007571944 | 55122 | 3 |
| NT2RP7002151 | 0.006119795 | 216146 | 2 |
| NT2RP7002163 | 0.004038283 | 222728 | 1 |
| NT2RP7002243 | 0.008201307 | 92650 | 3 |
| NT2RP7002282 | 0.010911494 | 65397 | 4 |
| NT2RP7002376 | 0.013809916 | 87666 | 6 |
| NT2RP7002379 | 0.332851147 | 91392 | 82 |
| NT2RP7002449 | 0.082940386 | 116018 | 34 |
| NT2RP7002450 | 0.06278006 | 110745 | 8 |
| NT2RP7002478 | 0.005747743 | 71529 | 2 |
| NT2RP7002554 | 0.004038283 | 269221 | 1 |
| NT2RP7002594 | 0.012266286 | 7603 | 5 |
| NT2RP7002603 | 0.004038283 | 275386 | 1 |
| NT2RP7002619 | 0.153785301 | 115895 | 21 |
| NT2RP7002650 | 0.004038283 | 270976 | 1 |
| NT2RP7002738 | 0.029107266 | 94672 | 13 |
| NT2RP7002779 | 0.005133561 | 87622 | 2 |
| NT2RP7002802 | 0.045849277 | 48683 | 17 |
| NT2RP7002829 | 0.019162507 | 112813 | 5 |
| NT2RP7002841 | 0.004038283 | 270005 | 1 |
| NT2RP7002875 | 0.181580261 | 45064 | 70 |
| NT2RP7002906 | 0.059763947 | 130515 | 21 |
| NT2RP7002978 | 0.004038283 | 67366 | 1 |
| NT2RP7002982 | 0.043102829 | 130158 | 11 |
| NT2RP7003025 | 0.004038283 | 184472 | 1 |
| NT2RP7003050 | 0.004038283 | 260561 | 1 |
| NT2RP7003055 | 0.015878856 | 153647 | 5 |
| NT2RP7003084 | 0.004038283 | 114420 | 1 |
| NT2RP7003091 | 0.004038283 | 134514 | 1 |
| NT2RP7003107 | 0.004038283 | 142608 | 1 |
| NT2RP7003134 | 0.004038283 | 190805 | 1 |
| NT2RP7003148 | 0.004038283 | 141947 | 1 |
| NT2RP7003203 | 0.090709641 | 59937 | 33 |
| NT2RP7003261 | 0.123329298 | 41599 | 20 |
| NT2RP7003304 | 0.118635564 | 90575 | 55 |
| NT2RP7003319 | 0.183647528 | 105733 | 64 |
| NT2RP7003439 | 0.004038283 | 232331 | 1 |
| NT2RP7003511 | 0.050705643 | 125339 | 16 |
| NT2RP7003629 | 0.014808438 | 77952 | 7 |
| NT2RP7003632 | 0.10290126 | 128010 | 24 |
| NT2RP7003647 | 0.142371141 | 85875 | 69 |
| NT2RP7003680 | 0.022461425 | 84281 | 9 |
| NT2RP7003688 | 0.226913699 | 119317 | 88 |
| NT2RP7003724 | 0.018144711 | 131537 | 9 |
| NT2RP7003960 | 0.10113201 | 144088 | 23 |
| NT2RP7004027 | 0.004038283 | 224197 | 1 |
| NT2RP7004080 | 0.004038283 | 109925 | 1 |
| NT2RP7004114 | 0.030048915 | 44516 | 11 |
| NT2RP7004123 | 0.087315579 | 56288 | 14 |
| NT2RP7004173 | 0.219398702 | 86661 | 51 |
| NT2RP7004196 | 0.127886066 | 104021 | 37 |
| NT2RP7004204 | 0.098263684 | 90300 | 21 |
| NT2RP7004233 | 0.01401255 | 150859 | 6 |
| NT2RP7004260 | 0.006530717 | 99380 | 3 |
| NT2RP7004348 | 0.074143863 | 18669 | 21 |
| NT2RP7004352 | 0.098656868 | 105048 | 44 |
| NT2RP7004358 | 0.004038283 | 73573 | 1 |
| NT2RP7004373 | 0.142361962 | 76852 | 49 |
| NT2RP7004396 | 0.004038283 | 142300 | 1 |
| NT2RP7004428 | 0.049683901 | 58797 | 22 |
| NT2RP7004481 | 0.031155526 | 93784 | 12 |
| NT2RP7004541 | 0.00695314 | 103974 | 2 |
| NT2RP7004559 | 0.019658121 | 101315 | 4 |
| NT2RP7004641 | 0.006530717 | 142071 | 3 |
| NT2RP7004656 | 0.007791966 | 100550 | 3 |
| NT2RP7004687 | 0.008887244 | 176653 | 4 |
| NT2RP7004722 | 0.004038283 | 281205 | 1 |
| NT2RP7004728 | 0.106603688 | 132891 | 36 |
| NT2RP7004751 | 0.357991542 | 21028 | 29 |
| NT2RP7004766 | 0.004038283 | 268800 | 1 |
| NT2RP7004790 | 0.021469442 | 105506 | 8 |
| NT2RP7004884 | 0.061033184 | 112079 | 25 |
| NT2RP7004911 | 0.095062189 | 112662 | 25 |
| NT2RP7004915 | 0.022570023 | 133371 | 10 |
| NT2RP7004925 | 0.132701628 | 63684 | 35 |
| NT2RP7004946 | 0.194637197 | 59625 | 68 |
| NT2RP7004961 | 0.009953407 | 150743 | 3 |
| NT2RP7004964 | 0.004038283 | 218051 | 1 |
| NT2RP7004975 | 0.004038283 | 220202 | 1 |
| NT2RP7005118 | 0.004038283 | 271306 | 1 |
| NT2RP7005205 | 0.004038283 | 218013 | 1 |
| NT2RP7005219 | 0.006416772 | 96869 | 3 |
| NT2RP7005323 | 0.004038283 | 236568 | 1 |
| NT2RP7005468 | 0.009164676 | 201673 | 3 |
| NT2RP7005493 | 0.013846334 | 148931 | 4 |
| NT2RP7005502 | 0.119877738 | 49116 | 16 |
| NT2RP7005513 | 0.099133722 | 31539 | 11 |
| NT2RP7005520 | 0.055859713 | 156866 | 12 |
| NT2RP7005529 | 0.022694623 | 141686 | 11 |
| NT2RP7005629 | 0.004038283 | 161150 | 1 |
| NT2RP7005631 | 0.004038283 | 208386 | 1 |
| NT2RP7005669 | 0.004038283 | 153070 | 1 |
| NT2RP7005675 | 0.057614967 | 75515 | 33 |
| NT2RP7005750 | 0.046827762 | 98076 | 16 |
| NT2RP7005846 | 0.004038283 | 188809 | 1 |
| NT2RP7006033 | 0.021795628 | 193708 | 8 |
| NT2RP7006075 | 0.063952128 | 136518 | 14 |
| NT2RP7006141 | 0.226889501 | 45902 | 26 |
| NT2RP7006160 | 0.004038283 | 197282 | 1 |
| NT2RP7006162 | 0.005227527 | 76680 | 2 |
| NT2RP7006188 | 0.064360101 | 88606 | 25 |
| NT2RP7006223 | 0.03965534 | 17718 | 19 |
| NT2RP7006263 | 0.04161189 | 76426 | 11 |
| NT2RP7006296 | 0.005133561 | 192274 | 2 |
| NT2RP7006304 | 0.005133561 | 27331 | 2 |
| NT2RP7006374 | 0.004038283 | 273470 | 1 |
| NT2RP7006395 | 0.009786026 | 35644 | 3 |
| NT2RP7006408 | 0.004038283 | 119602 | 1 |
| NT2RP7006457 | 0.008076566 | 161066 | 2 |
| NT2RP7006490 | 0.078060076 | 141871 | 14 |
| NT2RP7006527 | 0.165480193 | 75398 | 41 |
| NT2RP7006539 | 0.057592728 | 130971 | 13 |
| NT2RP7006547 | 0.005133561 | 5916 | 2 |
| NT2RP7006601 | 0.005915125 | 200548 | 2 |
| NT2RP7006619 | 0.075911455 | 35829 | 18 |
| NT2RP7006621 | 0.004038283 | 212913 | 1 |
| NT2RP7006636 | 0.004038283 | 51186 | 1 |
| NT2RP7006701 | 0.033326419 | 8826 | 9 |
| NT2RP7006717 | 0.254681494 | 47020 | 65 |
| NT2RP7006853 | 0.004038283 | 268915 | 1 |
| NT2RP7006886 | 0.01761676 | 18761 | 6 |
| NT2RP7006887 | 0.004038283 | 266775 | 1 |
| NT2RP7006980 | 0.009935099 | 113362 | 3 |
| NT2RP7006986 | 0.044602784 | 89686 | 12 |
| NT2RP7006995 | 0.007791966 | 221987 | 3 |
| NT2RP7007100 | 0.216513216 | 79998 | 83 |
| NT2RP7007114 | 0.010227459 | 37630 | 4 |
| NT2RP7007154 | 0.293831862 | 76905 | 29 |
| NT2RP7007177 | 0.123039235 | 51733 | 23 |
| NT2RP7007221 | 0.004038283 | 114433 | 1 |
| NT2RP7007226 | 0.004038283 | 122054 | 1 |
| NT2RP7007252 | 0.024400287 | 120769 | 14 |
| NT2RP7007269 | 0.007133779 | 222429 | 2 |
| NT2RP7007310 | 0.044993474 | 106402 | 3 |
| NT2RP7007359 | 0.004038283 | 194321 | 1 |
| NT2RP7007381 | 0.010684351 | 167370 | 5 |
| NT2RP7007387 | 0.081631537 | 118997 | 25 |
| NT2RP7007406 | 0.005435438 | 256496 | 2 |
| NT2RP7007480 | 0.008076566 | 88575 | 2 |
| NT2RP7007504 | 0.004038283 | 55564 | 1 |
| NT2RP7007524 | 0.049128914 | 143252 | 23 |
| NT2RP7007530 | 0.076428255 | 84475 | 15 |
| NT2RP7007537 | 0.136267014 | 50935 | 33 |
| NT2RP7007580 | 0.007010403 | 123339 | 3 |
| NT2RP7007594 | 0.004038283 | 179431 | 1 |
| NT2RP7007610 | 0.057895736 | 78252 | 21 |
| NT2RP7007617 | 0.010650739 | 89498 | 4 |
| NT2RP7007643 | 0.005133561 | 72414 | 2 |
| NT2RP7007766 | 0.005435438 | 187003 | 2 |
| NT2RP7007842 | 0.087359377 | 137047 | 20 |
| NT2RP7007925 | 0.004038283 | 195304 | 1 |
| NT2RP7007930 | 0.005133561 | 234959 | 2 |
| NT2RP7007975 | 0.122783079 | 98768 | 42 |
| NT2RP7008013 | 0.004038283 | 106409 | 1 |
| NT2RP7008015 | 0.166064989 | 135633 | 8 |
| NT2RP7008085 | 0.253789888 | 84562 | 87 |
| NT2RP7008133 | 0.004038283 | 62179 | 1 |
| NT2RP7008137 | 0.004038283 | 154411 | 1 |
| NT2RP7008142 | 0.004038283 | 266350 | 1 |
| NT2RP7008144 | 0.004038283 | 48898 | 1 |
| NT2RP7008161 | 0.026688097 | 102587 | 11 |
| NT2RP7008167 | 0.184617537 | 32039 | 46 |
| NT2RP7008190 | 0.126314021 | 69191 | 21 |
| NT2RP7008315 | 0.022327348 | 127190 | 6 |
| NT2RP7008360 | 0.010128046 | 42161 | 3 |
| NT2RP7008396 | 0.013611515 | 93862 | 5 |
| NT2RP7008406 | 0.004038283 | 93781 | 1 |
| NT2RP7008435 | 0.004038283 | 189554 | 1 |
| NT2RP7008441 | 0.008323023 | 117286 | 3 |
| NT2RP7008454 | 0.004038283 | 239197 | 1 |
| NT2RP7008487 | 0.010950062 | 23020 | 4 |
| NT2RP7008543 | 0.060904559 | 73412 | 18 |
| NT2RP7008544 | 0.004038283 | 271803 | 1 |
| NT2RP7008550 | 0.01515973 | 71600 | 4 |
| NT2RP7008557 | 0.004038283 | 108227 | 1 |
| NT2RP7008623 | 0.038930118 | 89603 | 2 |
| NT2RP7008657 | 0.004038283 | 233387 | 1 |
| NT2RP7008714 | 0.043205302 | 36975 | 17 |
| NT2RP7008720 | 0.007083164 | 175173 | 2 |
| NT2RP7008855 | 0.004038283 | 89344 | 1 |
| NT2RP7008863 | 0.004038283 | 90649 | 1 |
| NT2RP7009012 | 0.014778223 | 40000 | 6 |
| NT2RP7009019 | 0.004038283 | 19155 | 1 |
| NT2RP7009030 | 0.017416598 | 131055 | 6 |
| NT2RP7009087 | 0.068972966 | 111939 | 23 |
| NT2RP7009097 | 0.093197918 | 129012 | 11 |
| NT2RP7009108 | 0.188525251 | 24105 | 47 |
| NT2RP7009147 | 0.105182831 | 64980 | 28 |
| NT2RP7009149 | 0.14006429 | 87758 | 24 |
| NT2RP7009168 | 0.006119795 | 131468 | 2 |
| NT2RP7009215 | 0.004038283 | 235426 | 1 |
| NT2RP7009225 | 0.059569243 | 129171 | 16 |
| NT2RP7009236 | 0.004038283 | 238266 | 1 |
| NT2RP7009253 | 0.004038283 | 146725 | 1 |
| NT2RP7009259 | 0.298230805 | 74339 | 101 |
| NT2RP7009267 | 0.004038283 | 220500 | 1 |
| NT2RP7009322 | 0.015502229 | 182783 | 2 |
| NT2RP7009363 | 0.004038283 | 210048 | 1 |
| NT2RP7009370 | 0.005133561 | 136611 | 2 |
| NT2RP7009373 | 0.041709609 | 76469 | 18 |
| NT2RP7009394 | 0.138568949 | 82993 | 46 |
| NT2RP7009397 | 0.004038283 | 195193 | 1 |
| NT2RP7009429 | 0.004038283 | 178085 | 1 |
| NT2RP7009439 | 0.012114849 | 150342 | 3 |
| NT2RP7009466 | 0.005435438 | 111733 | 2 |
| NT2RP7009481 | 0.040912856 | 120940 | 16 |
| NT2RP7009482 | 0.361548428 | 88362 | 93 |
| NT2RP7009498 | 0.166273189 | 144037 | 10 |
| NT2RP7009502 | 0.004038283 | 206966 | 1 |
| NT2RP7009507 | 0.00571383 | 203782 | 2 |
| NT2RP7009577 | 0.007133779 | 7340 | 2 |
| NT2RP7009743 | 0.004038283 | 102207 | 1 |
| NT2RP7009867 | 0.004038283 | 135882 | 1 |
| NT2RP7010109 | 0.010662966 | 143053 | 4 |
| NT2RP7010110 | 0.072708957 | 107156 | 11 |
| NT2RP7010128 | 0.004038283 | 175182 | 1 |
| NT2RP7010235 | 0.019198013 | 179581 | 5 |
| NT2RP7010275 | 0.004038283 | 74820 | 1 |
| NT2RP7010521 | 0.004038283 | 134569 | 1 |
| NT2RP7010599 | 0.005227527 | 178225 | 2 |
| NT2RP7010612 | 0.007746144 | 141449 | 3 |
| NT2RP7010817 | 0.004038283 | 78314 | 1 |
| NT2RP7011086 | 0.004038283 | 201855 | 1 |
| NT2RP7011132 | 0.004038283 | 232449 | 1 |
| NT2RP7011268 | 0.004038283 | 202775 | 1 |
| NT2RP7011318 | 0.007133779 | 145973 | 2 |
| NT2RP7011452 | 0.004038283 | 208857 | 1 |
| NT2RP7011570 | 0.004038283 | 190769 | 1 |
| NT2RP7011615 | 0.028439911 | 103544 | 5 |
| NT2RP7011721 | 0.004038283 | 90109 | 1 |
| NT2RP7011909 | 0.59115051 | 161342 | 3 |
| NT2RP7012291 | 0.004038283 | 184850 | 1 |
| NT2RP7012516 | 0.004038283 | 205767 | 1 |
| NT2RP7012776 | 0.019935962 | 115000 | 4 |
| NT2RP7013002 | 0.007083164 | 157326 | 2 |
| NT2RP7013374 | 0.004038283 | 11400 | 1 |
| NT2RP7013499 | 0.004038283 | 219720 | 1 |
| NT2RP7013573 | 0.023252827 | 117327 | 9 |
| NT2RP7013729 | 0.004038283 | 205226 | 1 |
| NT2RP7013764 | 0.014758364 | 181221 | 5 |
| NT2RP7013795 | 0.038712347 | 173210 | 2 |
| NT2RP7013999 | 0.004038283 | 158185 | 1 |
| NT2RP7014005 | 0.028800602 | 92920 | 8 |
| NT2RP7014178 | 0.004038283 | 189229 | 1 |
| NT2RP7014348 | 0.004038283 | 179928 | 1 |
| NT2RP7014420 | 0.004038283 | 265682 | 1 |
| NT2RP7014583 | 0.010240603 | 183428 | 2 |
| NT2RP7014721 | 0.004038283 | 175200 | 1 |
| NT2RP7014743 | 0.004038283 | 2217 | 1 |
| NT2RP7014778 | 0.004038283 | 174196 | 1 |
| NT2RP7014906 | 0.004038283 | 218451 | 1 |
| NT2RP7014910 | 0.140138393 | 92250 | 33 |
| NT2RP7015080 | 0.004038283 | 200646 | 1 |
| NT2RP7015431 | 0.004038283 | 178181 | 1 |
| NT2RP7015503 | 0.052914656 | 184774 | 5 |
| NT2RP7015512 | 0.025923118 | 135904 | 7 |
| NT2RP7015676 | 0.004038283 | 223128 | 1 |
| NT2RP7015789 | 0.004038283 | 97395 | 1 |
| NT2RP7015996 | 0.004038283 | 117328 | 1 |
| NT2RP7016508 | 0.004038283 | 35406 | 1 |
| NT2RP7016574 | 0.007584674 | 76256 | 3 |
| NT2RP7016622 | 0.004038283 | 104981 | 1 |
| NT2RP7016911 | 0.004038283 | 117384 | 1 |
| NT2RP7017139 | 0.004038283 | 164222 | 1 |
| NT2RP7017284 | 0.004038283 | 101101 | 1 |
| NT2RP7017365 | 0.004038283 | 202699 | 1 |
| NT2RP7017474 | 0.008901476 | 137193 | 4 |
| NT2RP7017546 | 0.008076566 | 283109 | 2 |
| NT2RP7017567 | 0.004038283 | 164154 | 1 |
| NT2RP7017795 | 0.004038283 | 148488 | 1 |
| NT2RP7017971 | 0.004038283 | 132306 | 1 |
| NT2RP7018032 | 0.004038283 | 205 | 1 |
| NT2RP7018126 | 0.009950545 | 194781 | 2 |
| NT2RP7018197 | 0.004038283 | 183502 | 1 |
| NT2RP7018206 | 0.004038283 | 276028 | 1 |
| NT2RP7018340 | 0.004038283 | 202005 | 1 |
| NT2RP7018586 | 0.004038283 | 229410 | 1 |
| NT2RP7018802 | 0.008076566 | 195035 | 2 |
| NT2RP7018871 | 0.004038283 | 189361 | 1 |
| NT2RP7019064 | 0.013047083 | 196960 | 4 |
| NT2RP7019135 | 0.00571383 | 118773 | 2 |
| NT2RP7019273 | 0.004038283 | 1216 | 1 |
| NT2RP7019367 | 0.004038283 | 263250 | 1 |
| NT2RP7019401 | 0.016137788 | 33936 | 4 |
| NT2RP7019445 | 0.004038283 | 75302 | 1 |
| NT2RP7019543 | 0.015840394 | 146229 | 4 |
| NT2RP7019682 | 0.004038283 | 54346 | 1 |
| NT2RP7019835 | 0.160786693 | 58635 | 4 |
| NT2RP7020112 | 0.009879746 | 207374 | 2 |
| NT2RP7020123 | 0.039398962 | 144471 | 2 |
| NT2RP7020343 | 0.004038283 | 277655 | 1 |
| NT2RP7020379 | 0.004038283 | 246576 | 1 |
| NT2RP8000137 | 0.004038283 | 192623 | 1 |
| NT2RP8000296 | 0.004038283 | 77947 | 1 |
| NT2RP8000435 | 0.012557557 | 156683 | 5 |
| NT2RP8000483 | 0.004038283 | 281854 | 1 |
| NT2RP8000521 | 0.006416772 | 199019 | 3 |
| NT2RP8000633 | 0.004038283 | 275280 | 1 |
| NT2RP8001363 | 0.004038283 | 250513 | 1 |
| NT2RP8001407 | 0.004038283 | 257737 | 1 |
| NT2RP8001584 | 0.004038283 | 281882 | 1 |
| NT2RP8001604 | 0.024148442 | 181892 | 6 |
| NT2RP8001605 | 0.00571383 | 171246 | 2 |
| NT2RP8003490 | 0.004038283 | 222150 | 1 |
| NT2RP8003657 | 0.004038283 | 259812 | 1 |
| NT2RP8003787 | 0.004038283 | 186112 | 1 |
| NT2RP8004306 | 0.004038283 | 21976 | 1 |
| NT2RP8005546 | 0.006832594 | 114418 | 3 |
| NT2RP8006452 | 0.004038283 | 226920 | 1 |
| NT2RP8006521 | 0.004038283 | 276216 | 1 |
| NT2RP8007416 | 0.004038283 | 269019 | 1 |
| NT2RP8007503 | 0.004038283 | 260794 | 1 |
| NT2RP8007715 | 0.013335599 | 30421 | 8 |
| NT2RP8007920 | 0.08470369 | 156290 | 27 |
| NT2RP8008057 | 0.005915125 | 119986 | 2 |
| NT2RP8009119 | 0.036305904 | 102896 | 28 |
| NT2RP8009248 | 0.004038283 | 101043 | 1 |
| NTONG1000006 | 0.012348728 | 53037 | 1 |
| NTONG1000033 | 0.136758931 | 54910 | 53 |
| NTONG1000047 | 0.02788718 | 63430 | 4 |
| NTONG1000052 | 0.350823542 | 94424 | 31 |
| NTONG1000064 | 0.387403789 | 34429 | 51 |
| NTONG1000075 | 0.025148392 | 55312 | 3 |
| NTONG1000087 | 0.012348728 | 74103 | 1 |
| NTONG1000098 | 0.262993716 | 23511 | 95 |
| NTONG1000123 | 0.035984138 | 13134 | 3 |
| NTONG1000130 | 0.01422557 | 65535 | 2 |
| NTONG1000161 | 0.012348728 | 30236 | 1 |
| NTONG1000172 | 0.012348728 | 65517 | 1 |
| NTONG1000182 | 0.135343266 | 65504 | 23 |
| NTONG1000213 | 0.012348728 | 35234 | 1 |
| NTONG1000214 | 0.045275933 | 35396 | 7 |
| NTONG1000246 | 0.107159275 | 79132 | 20 |
| NTONG1000247 | 0.012348728 | 112253 | 1 |
| NTONG1000257 | 0.152254757 | 57465 | 19 |
| NTONG1000264 | 0.171910462 | 52878 | 57 |
| NTONG2000040 | 0.012348728 | 193180 | 1 |
| NTONG2000107 | 0.015712552 | 79459 | 3 |
| NTONG2000223 | 0.082907015 | 121274 | 15 |
| NTONG2000231 | 0.012348728 | 264438 | 1 |
| NTONG2000265 | 0.196548465 | 93345 | 75 |
| NTONG2000334 | 0.08195273 | 21695 | 11 |
| NTONG2000363 | 0.012348728 | 146685 | 1 |
| NTONG2000413 | 0.015525518 | 196061 | 3 |
| NTONG2000492 | 0.024697456 | 174181 | 2 |
| NTONG2000499 | 0.018153906 | 171241 | 2 |
| NTONG2000531 | 0.012348728 | 272314 | 1 |
| NTONG2000583 | 0.012348728 | 278144 | 1 |
| NTONG2000800 | 0.316441064 | 121153 | 19 |
| NTONG2000858 | 0.058015149 | 146317 | 5 |
| NTONG2000876 | 0.120334926 | 134760 | 17 |
| NTONG2000878 | 0.141887343 | 139080 | 14 |
| NTONG2000966 | 0.012348728 | 153836 | 1 |
| NTONG2000977 | 0.170446213 | 113626 | 15 |
| NTONG2000985 | 0.089875138 | 165062 | 8 |
| NTONG2001014 | 0.012348728 | 67982 | 1 |
| NTONG2001079 | 0.01443024 | 118831 | 2 |
| NTONG2001137 | 0.146407897 | 89033 | 13 |
| NTONG2001157 | 0.012348728 | 41655 | 1 |
| NTONG2001173 | 0.012348728 | 222018 | 1 |
| NTONG2001220 | 0.02911805 | 12118 | 3 |
| NTONG2001222 | 0.202062765 | 143035 | 11 |
| NTONG2001362 | 0.047999352 | 230795 | 2 |
| NTONG2001428 | 0.015895119 | 99341 | 3 |
| NTONG2001532 | 0.012348728 | 88521 | 1 |
| NTONG2001550 | 0.048140878 | 101969 | 13 |
| NTONG2001567 | 0.546437024 | 100109 | 48 |
| NTONG2001587 | 0.562851169 | 162441 | 26 |
| NTONG2001612 | 0.149263756 | 65582 | 23 |
| NTONG2001619 | 0.012348728 | 280445 | 1 |
| NTONG2001642 | 0.012348728 | 86736 | 1 |
| NTONG2001657 | 0.025270294 | 100024 | 2 |
| NTONG2001676 | 0.023488518 | 99714 | 4 |
| NTONG2001762 | 0.092918664 | 130311 | 35 |
| NTONG2002278 | 0.012348728 | 158242 | 1 |
| NTONG2002582 | 0.044549777 | 22394 | 14 |
| NTONG2002807 | 0.014206842 | 91287 | 2 |
| NTONG2002948 | 0.075460802 | 181167 | 7 |
| NTONG2002970 | 0.012348728 | 194985 | 1 |
| NTONG2002985 | 0.014024275 | 122207 | 2 |
| NTONG2003158 | 0.012348728 | 57219 | 1 |
| NTONG2003210 | 0.012348728 | 199293 | 1 |
| NTONG2003316 | 0.012348728 | 102292 | 1 |
| NTONG2003409 | 0.012348728 | 53319 | 1 |
| NTONG2003454 | 0.056203927 | 98363 | 22 |
| NTONG2003515 | 0.017121699 | 187810 | 3 |
| NTONG2003805 | 0.012348728 | 133262 | 1 |
| NTONG2003839 | 0.063906949 | 67108 | 10 |
| NTONG2003852 | 0.024943187 | 117128 | 2 |
| NTONG2003897 | 0.012348728 | 60491 | 1 |
| NTONG2003928 | 0.028763503 | 197631 | 5 |
| NTONG2004095 | 0.018249654 | 218809 | 5 |
| NTONG2004308 | 0.01422557 | 147480 | 2 |
| NTONG2004521 | 0.012348728 | 78495 | 1 |
| NTONG2004614 | 0.028294695 | 17896 | 5 |
| NTONG2004690 | 0.012348728 | 11320 | 1 |
| NTONG2004806 | 0.024155104 | 216592 | 2 |
| NTONG2004829 | 0.012348728 | 183710 | 1 |
| NTONG2004844 | 0.103390513 | 66619 | 11 |
| NTONG2004918 | 0.025874448 | 121520 | 5 |
| NTONG2004991 | 0.012348728 | 175391 | 1 |
| NTONG2005062 | 0.012348728 | 184823 | 1 |
| NTONG2005086 | 0.012348728 | 90027 | 1 |
| NTONG2005137 | 0.012348728 | 9910 | 1 |
| NTONG2005153 | 0.021176197 | 162608 | 3 |
| NTONG2005265 | 0.012348728 | 198322 | 1 |
| NTONG2005277 | 0.012348728 | 140262 | 1 |
| NTONG2005363 | 0.014347129 | 161085 | 2 |
| NTONG2005373 | 0.028432412 | 39609 | 4 |
| NTONG2005391 | 0.071874571 | 129330 | 31 |
| NTONG2005480 | 0.012348728 | 207976 | 1 |
| NTONG2005497 | 0.611408917 | 134823 | 4 |
| NTONG2005520 | 0.251713915 | 33660 | 54 |
| NTONG2005577 | 0.012348728 | 183800 | 1 |
| NTONG2005657 | 0.024697456 | 152462 | 2 |
| NTONG2005801 | 0.094050386 | 84743 | 31 |
| NTONG2005822 | 0.01826134 | 140970 | 2 |
| NTONG2005897 | 0.024410459 | 116472 | 5 |
| NTONG2005969 | 0.052160241 | 126188 | 13 |
| NTONG2006099 | 0.012348728 | 74368 | 1 |
| NTONG2006187 | 0.012348728 | 121156 | 1 |
| NTONG2006301 | 0.030486948 | 147488 | 3 |
| NTONG2006324 | 0.012348728 | 140332 | 1 |
| NTONG2006354 | 0.012348728 | 158120 | 1 |
| NTONG2006440 | 0.012348728 | 222450 | 1 |
| NTONG2006484 | 0.038763008 | 97254 | 6 |
| NTONG2006501 | 0.012348728 | 147494 | 1 |
| NTONG2006646 | 0.012348728 | 147476 | 1 |
| NTONG2006709 | 0.018489106 | 89616 | 3 |
| NTONG2006783 | 0.059183732 | 116666 | 6 |
| NTONG2007020 | 0.012348728 | 111926 | 1 |
| NTONG2007028 | 0.014024275 | 179445 | 2 |
| NTONG2007034 | 0.02363541 | 131415 | 2 |
| NTONG2007052 | 0.039364238 | 79287 | 7 |
| NTONG2007249 | 0.012348728 | 113152 | 1 |
| NTONG2007517 | 0.012348728 | 221997 | 1 |
| NTONG2007522 | 0.012348728 | 87900 | 1 |
| NTONG2007658 | 0.083728568 | 66678 | 10 |
| NTONG2007693 | 0.023812674 | 121145 | 2 |
| NTONG2007756 | 0.024824654 | 155771 | 3 |
| NTONG2008088 | 0.016397399 | 157349 | 4 |
| NTONG2008093 | 0.012348728 | 36460 | 1 |
| NTONG2008143 | 0.015421431 | 123769 | 3 |
| NTONG2008365 | 0.012348728 | 191045 | 1 |
| NTONG2008483 | 0.209724219 | 119804 | 44 |
| NTONG2008522 | 0.042756755 | 127388 | 5 |
| NTONG2008672 | 0.012348728 | 164712 | 1 |
| NTONG2008862 | 0.012348728 | 166531 | 1 |
| NTONG2008939 | 0.012348728 | 233372 | 1 |
| NTONG2008944 | 0.057384338 | 114730 | 22 |
| NTONG2009060 | 0.06013176 | 134574 | 3 |
| NTONG2009068 | 0.012348728 | 124960 | 1 |
| NTONG2009229 | 0.012348728 | 41389 | 1 |
| NTONG2009233 | 0.012348728 | 166519 | 1 |
| NTONG2009468 | 0.017642074 | 100811 | 4 |
| NTONG2009576 | 0.023581774 | 149978 | 7 |
| OCBBF1000016 | 0.002081512 | 36356 | 1 |
| OCBBF1000030 | 0.003790972 | 76364 | 2 |
| OCBBF1000042 | 0.002081512 | 68171 | 1 |
| OCBBF1000049 | 0.03371824 | 35934 | 15 |
| OCBBF1000054 | 0.002081512 | 108231 | 1 |
| OCBBF1000067 | 0.045837097 | 22079 | 14 |
| OCBBF1000080 | 0.00876331 | 38621 | 4 |
| OCBBF1000085 | 0.004163024 | 60213 | 2 |
| OCBBF1000086 | 0.218899346 | 73638 | 26 |
| OCBBF1000091 | 0.004163024 | 34976 | 2 |
| OCBBF1000104 | 0.016269141 | 78020 | 6 |
| OCBBF1000114 | 0.065727455 | 37092 | 10 |
| OCBBF1000118 | 0.002081512 | 77875 | 1 |
| OCBBF1000119 | 0.002081512 | 106812 | 1 |
| OCBBF1000122 | 0.004163024 | 94797 | 2 |
| OCBBF1000130 | 0.002081512 | 6405 | 1 |
| OCBBF1000138 | 0.002081512 | 106388 | 1 |
| OCBBF1000140 | 0.067835682 | 50807 | 21 |
| OCBBF1000145 | 0.003939626 | 69439 | 2 |
| OCBBF1000146 | 0.194247437 | 66341 | 59 |
| OCBBF1000153 | 0.019747592 | 37782 | 9 |
| OCBBF1000175 | 0.023262008 | 75110 | 7 |
| OCBBF1000185 | 0.03980012 | 45258 | 15 |
| OCBBF1000188 | 0.160904706 | 71218 | 26 |
| OCBBF1000192 | 0.002081512 | 78433 | 1 |
| OCBBF1000254 | 0.003769789 | 181531 | 2 |
| OCBBF1000315 | 0.019150674 | 86515 | 11 |
| OCBBF2000013 | 0.086109145 | 138184 | 22 |
| OCBBF2000015 | 0.211786145 | 55887 | 86 |
| OCBBF2000074 | 0.036401716 | 14609 | 14 |
| OCBBF2000126 | 0.043109901 | 134567 | 11 |
| OCBBF2000178 | 0.153683174 | 66291 | 58 |
| OCBBF2000231 | 0.04277493 | 123966 | 17 |
| OCBBF2000277 | 0.022806825 | 83294 | 11 |
| OCBBF2000287 | 0.007994124 | 225450 | 2 |
| OCBBF2000323 | 0.01104222 | 54174 | 4 |
| OCBBF2000452 | 0.002081512 | 222761 | 1 |
| OCBBF2000467 | 0.083073577 | 28185 | 22 |
| OCBBF2000522 | 0.066263823 | 42101 | 14 |
| OCBBF2000523 | 0.01785392 | 70182 | 6 |
| OCBBF2000677 | 0.010286152 | 118233 | 5 |
| OCBBF2000703 | 0.002081512 | 186946 | 1 |
| OCBBF2000712 | 0.1773015 | 2458 | 8 |
| OCBBF2000719 | 0.004163024 | 33520 | 2 |
| OCBBF2000824 | 0.02788448 | 85154 | 7 |
| OCBBF2000831 | 0.034801238 | 102289 | 18 |
| OCBBF2000846 | 0.228965121 | 103503 | 25 |
| OCBBF2000904 | 0.041454731 | 144127 | 11 |
| OCBBF2000982 | 0.192139698 | 121587 | 28 |
| OCBBF2000986 | 0.173870224 | 86050 | 32 |
| OCBBF2000998 | 0.002081512 | 34375 | 1 |
| OCBBF2001075 | 0.002081512 | 125228 | 1 |
| OCBBF2001101 | 0.002081512 | 126673 | 1 |
| OCBBF2001124 | 0.002081512 | 52260 | 1 |
| OCBBF2001140 | 0.029635973 | 82555 | 18 |
| OCBBF2001166 | 0.0305713 | 82890 | 17 |
| OCBBF2001176 | 0.002081512 | 83976 | 1 |
| OCBBF2001186 | 0.009465192 | 124096 | 4 |
| OCBBF2001196 | 0.010580523 | 130168 | 4 |
| OCBBF2001210 | 0.046211943 | 50387 | 24 |
| OCBBF2001252 | 0.036401716 | 14609 | 14 |
| OCBBF2001307 | 0.100739283 | 106222 | 50 |
| OCBBF2001323 | 0.019486196 | 131695 | 7 |
| OCBBF2001389 | 0.002081512 | 282111 | 1 |
| OCBBF2001402 | 0.005177008 | 109335 | 2 |
| OCBBF2001408 | 0.008677223 | 56975 | 5 |
| OCBBF2001416 | 0.008029128 | 48643 | 5 |
| OCBBF2001473 | 0.002081512 | 84922 | 1 |
| OCBBF2001492 | 0.244207829 | 34142 | 65 |
| OCBBF2001494 | 0.007922975 | 24094 | 2 |
| OCBBF2001527 | 0.002081512 | 278969 | 1 |
| OCBBF2001528 | 0.005053632 | 100761 | 3 |
| OCBBF2001586 | 0.046730494 | 146803 | 12 |
| OCBBF2001639 | 0.035631759 | 109810 | 11 |
| OCBBF2001681 | 0.002081512 | 16642 | 1 |
| OCBBF2001687 | 0.087108275 | 6874 | 22 |
| OCBBF2001706 | 0.003478667 | 235606 | 2 |
| OCBBF2001749 | 0.005177008 | 157261 | 2 |
| OCBBF2001794 | 0.035441514 | 32723 | 12 |
| OCBBF2001910 | 0.004163024 | 117932 | 2 |
| OCBBF2001931 | 0.010006147 | 133821 | 3 |
| OCBBF2001961 | 0.004079913 | 60998 | 2 |
| OCBBF2001983 | 0.023193627 | 60773 | 7 |
| OCBBF2002015 | 0.012114648 | 150985 | 8 |
| OCBBF2002083 | 0.015247912 | 82893 | 5 |
| OCBBF2002086 | 0.052007922 | 40767 | 22 |
| OCBBF2002124 | 0.002081512 | 183935 | 1 |
| OCBBF2002161 | 0.002081512 | 131222 | 1 |
| OCBBF2002290 | 0.016613995 | 120332 | 6 |
| OCBBF2002357 | 0.002081512 | 191004 | 1 |
| OCBBF2002376 | 0.022008448 | 147455 | 5 |
| OCBBF2002615 | 0.002081512 | 140519 | 1 |
| OCBBF2002626 | 0.198413943 | 106947 | 21 |
| OCBBF2002656 | 0.101296218 | 1664 | 24 |
| OCBBF2002663 | 0.005177008 | 148373 | 2 |
| OCBBF2002805 | 0.002081512 | 44235 | 1 |
| OCBBF2002865 | 0.00317679 | 74233 | 2 |
| OCBBF2002920 | 0.002081512 | 158048 | 1 |
| OCBBF2002980 | 0.003790972 | 146805 | 2 |
| OCBBF2003028 | 0.002081512 | 49688 | 1 |
| OCBBF2003052 | 0.044113724 | 132884 | 19 |
| OCBBF2003091 | 0.002081512 | 22155 | 1 |
| OCBBF2003246 | 0.017121104 | 114831 | 8 |
| OCBBF2003327 | 0.002081512 | 123760 | 1 |
| OCBBF2003518 | 0.162544289 | 118129 | 10 |
| OCBBF2003543 | 0.003478667 | 21586 | 2 |
| OCBBF2003593 | 0.003757059 | 48347 | 2 |
| OCBBF2003744 | 0.003958354 | 133138 | 2 |
| OCBBF2003819 | 0.007952337 | 82076 | 5 |
| OCBBF2003925 | 0.201100195 | 140007 | 14 |
| OCBBF2004038 | 0.006258447 | 48766 | 2 |
| OCBBF2004104 | 0.122339577 | 104010 | 39 |
| OCBBF2004147 | 0.002081512 | 200508 | 1 |
| OCBBF2004168 | 0.002081512 | 207583 | 1 |
| OCBBF2004235 | 0.014048492 | 195820 | 3 |
| OCBBF2004259 | 0.003939626 | 86978 | 2 |
| OCBBF2004273 | 0.186932187 | 130331 | 27 |
| OCBBF2004385 | 0.002081512 | 138045 | 1 |
| OCBBF2004418 | 0.01179025 | 169228 | 2 |
| OCBBF2004478 | 0.002081512 | 80025 | 1 |
| OCBBF2004482 | 0.002081512 | 53096 | 1 |
| OCBBF2004533 | 0.00831787 | 137466 | 2 |
| OCBBF2004567 | 0.002081512 | 67515 | 1 |
| OCBBF2004612 | 0.002081512 | 226334 | 1 |
| OCBBF2004647 | 0.002081512 | 118191 | 1 |
| OCBBF2004669 | 0.003790972 | 92758 | 2 |
| OCBBF2004757 | 0.067608748 | 117850 | 22 |
| OCBBF2004826 | 0.002081512 | 105278 | 1 |
| OCBBF2004866 | 0.002081512 | 180466 | 1 |
| OCBBF2004883 | 0.002081512 | 150946 | 1 |
| OCBBF2004889 | 0.003757059 | 146777 | 2 |
| OCBBF2004930 | 0.016144097 | 89689 | 4 |
| OCBBF2004984 | 0.007035122 | 87327 | 3 |
| OCBBF2005066 | 0.004163024 | 80840 | 2 |
| OCBBF2005077 | 0.002081512 | 134671 | 1 |
| OCBBF2005161 | 0.003790972 | 94696 | 2 |
| OCBBF2005189 | 0.23397385 | 86681 | 40 |
| OCBBF2005343 | 0.002081512 | 165812 | 1 |
| OCBBF2005349 | 0.002081512 | 42064 | 1 |
| OCBBF2005373 | 0.002081512 | 96263 | 1 |
| OCBBF2005420 | 0.007920083 | 141936 | 4 |
| OCBBF2005428 | 0.050827112 | 184980 | 4 |
| OCBBF2005433 | 0.002081512 | 109541 | 1 |
| OCBBF2005476 | 0.035841242 | 123024 | 9 |
| OCBBF2005546 | 0.003790972 | 265997 | 2 |
| OCBBF2005843 | 0.002081512 | 158114 | 1 |
| OCBBF2005901 | 0.002081512 | 129438 | 1 |
| OCBBF2005956 | 0.059023062 | 71573 | 19 |
| OCBBF2006005 | 0.035146236 | 86625 | 14 |
| OCBBF2006030 | 0.002081512 | 169451 | 1 |
| OCBBF2006058 | 0.002081512 | 30929 | 1 |
| OCBBF2006113 | 0.006366253 | 195137 | 3 |
| OCBBF2006148 | 0.024741948 | 96646 | 6 |
| OCBBF2006151 | 0.029063487 | 15823 | 8 |
| OCBBF2006154 | 0.002081512 | 198178 | 1 |
| OCBBF2006172 | 0.031956714 | 132460 | 8 |
| OCBBF2006214 | 0.002081512 | 181767 | 1 |
| OCBBF2006241 | 0.002081512 | 183886 | 1 |
| OCBBF2006313 | 0.029344902 | 152182 | 8 |
| OCBBF2006332 | 0.449223397 | 100968 | 136 |
| OCBBF2006388 | 0.002081512 | 164800 | 1 |
| OCBBF2006438 | 0.002081512 | 157278 | 1 |
| OCBBF2006567 | 0.002081512 | 150803 | 1 |
| OCBBF2006624 | 0.002081512 | 136500 | 1 |
| OCBBF2006639 | 0.004163024 | 45282 | 2 |
| OCBBF2006660 | 0.011100739 | 124091 | 4 |
| OCBBF2006764 | 0.010919321 | 21352 | 6 |
| OCBBF2006849 | 0.082907015 | 121274 | 15 |
| OCBBF2006859 | 0.02236498 | 181783 | 4 |
| OCBBF2006972 | 0.003757059 | 97909 | 2 |
| OCBBF2006987 | 0.012962816 | 157075 | 5 |
| OCBBF2007028 | 0.121351916 | 42184 | 62 |
| OCBBF2007039 | 0.002081512 | 284822 | 1 |
| OCBBF2007051 | 0.003790972 | 148068 | 2 |
| OCBBF2007068 | 0.003790972 | 210094 | 2 |
| OCBBF2007114 | 0.015675457 | 144794 | 6 |
| OCBBF2007121 | 0.003958354 | 131948 | 2 |
| OCBBF2007184 | 0.002081512 | 126574 | 1 |
| OCBBF2007194 | 0.002081512 | 117714 | 1 |
| OCBBF2007196 | 0.009340032 | 174052 | 4 |
| OCBBF2007224 | 0.017745425 | 101669 | 7 |
| OCBBF2007232 | 0.098693552 | 123744 | 43 |
| OCBBF2007238 | 0.002081512 | 83372 | 1 |
| OCBBF2007354 | 0.166783836 | 94307 | 25 |
| OCBBF2007414 | 0.003478667 | 123594 | 2 |
| OCBBF2007415 | 0.20924961 | 34419 | 63 |
| OCBBF2007428 | 0.002081512 | 206949 | 1 |
| OCBBF2007478 | 0.002081512 | 176475 | 1 |
| OCBBF2007485 | 0.002081512 | 164585 | 1 |
| OCBBF2007520 | 0.002081512 | 207064 | 1 |
| OCBBF2007555 | 0.004163024 | 238792 | 2 |
| OCBBF2007610 | 0.002081512 | 169189 | 1 |
| OCBBF2007622 | 0.045639803 | 92541 | 4 |
| OCBBF2007756 | 0.002081512 | 46166 | 1 |
| OCBBF2007762 | 0.010656257 | 148430 | 5 |
| OCBBF2007829 | 0.305167583 | 87150 | 99 |
| OCBBF2007892 | 0.039332193 | 108414 | 10 |
| OCBBF2007931 | 0.004163024 | 104496 | 2 |
| OCBBF2007946 | 0.002081512 | 66275 | 1 |
| OCBBF2008005 | 0.105527079 | 97111 | 35 |
| OCBBF2008041 | 0.080162019 | 111911 | 13 |
| OCBBF2008116 | 0.002081512 | 93596 | 1 |
| OCBBF2008138 | 0.002081512 | 130170 | 1 |
| OCBBF2008144 | 0.058101046 | 111940 | 13 |
| OCBBF2008283 | 0.002081512 | 436 | 1 |
| OCBBF2008330 | 0.002081512 | 228201 | 1 |
| OCBBF2008340 | 0.021705749 | 91002 | 10 |
| OCBBF2008366 | 0.013844834 | 189685 | 2 |
| OCBBF2008466 | 0.019586597 | 82533 | 5 |
| OCBBF2008483 | 0.019247786 | 228136 | 3 |
| OCBBF2008511 | 0.015176425 | 181663 | 6 |
| OCBBF2008520 | 0.002081512 | 147205 | 1 |
| OCBBF2008569 | 0.007993774 | 251999 | 2 |
| OCBBF2008586 | 0.002081512 | 223609 | 1 |
| OCBBF2008640 | 0.004163024 | 209824 | 2 |
| OCBBF2008691 | 0.002081512 | 101423 | 1 |
| OCBBF2008701 | 0.015460842 | 140231 | 7 |
| OCBBF2008724 | 0.002081512 | 181842 | 1 |
| OCBBF2008760 | 0.010075286 | 123810 | 3 |
| OCBBF2008768 | 0.002081512 | 220261 | 1 |
| OCBBF2008770 | 0.012530733 | 109191 | 5 |
| OCBBF2008775 | 0.003769789 | 155067 | 2 |
| OCBBF2008790 | 0.002081512 | 215323 | 1 |
| OCBBF2008814 | 0.004163024 | 174214 | 2 |
| OCBBF2008822 | 0.070290265 | 109405 | 3 |
| OCBBF2009095 | 0.025494519 | 135510 | 8 |
| OCBBF2009115 | 0.002081512 | 139247 | 1 |
| OCBBF2009242 | 0.059175937 | 54064 | 25 |
| OCBBF2009301 | 0.080831708 | 11165 | 18 |
| OCBBF2009352 | 0.029766781 | 7697 | 12 |
| OCBBF2009391 | 0.005177008 | 176458 | 2 |
| OCBBF2009424 | 0.023088978 | 106847 | 7 |
| OCBBF2009536 | 0.003939626 | 197400 | 2 |
| OCBBF2009571 | 0.002081512 | 27613 | 1 |
| OCBBF2009583 | 0.002081512 | 155953 | 1 |
| OCBBF2009603 | 0.005063471 | 6032 | 2 |
| OCBBF2009772 | 0.142290854 | 110977 | 54 |
| OCBBF2009788 | 0.002081512 | 191020 | 1 |
| OCBBF2009836 | 0.040891613 | 121038 | 17 |
| OCBBF2009920 | 0.003790972 | 191017 | 2 |
| OCBBF2009926 | 0.004163024 | 183545 | 2 |
| OCBBF2010040 | 0.002081512 | 150853 | 1 |
| OCBBF2010140 | 0.005154214 | 93818 | 3 |
| OCBBF2010281 | 0.003958354 | 170482 | 2 |
| OCBBF2010313 | 0.012215794 | 183591 | 4 |
| OCBBF2010404 | 0.002081512 | 160058 | 1 |
| OCBBF2010416 | 0.002081512 | 172400 | 1 |
| OCBBF2010420 | 0.004163024 | 96182 | 2 |
| OCBBF2010557 | 0.009144401 | 129884 | 5 |
| OCBBF2010604 | 0.002081512 | 102705 | 1 |
| OCBBF2010709 | 0.002081512 | 282266 | 1 |
| OCBBF2010792 | 0.002081512 | 140692 | 1 |
| OCBBF2010819 | 0.013357928 | 67332 | 6 |
| OCBBF2010830 | 0.190423858 | 4893 | 30 |
| OCBBF2010841 | 0.026956667 | 75402 | 11 |
| OCBBF2010843 | 0.011249391 | 98744 | 3 |
| OCBBF2010858 | 0.025153856 | 86788 | 12 |
| OCBBF2010863 | 0.003769789 | 179423 | 2 |
| OCBBF2010871 | 0.036657763 | 20959 | 18 |
| OCBBF2010931 | 0.002081512 | 130987 | 1 |
| OCBBF2010945 | 0.004079913 | 117005 | 2 |
| OCBBF2010978 | 0.002081512 | 156073 | 1 |
| OCBBF2010989 | 0.002081512 | 157266 | 1 |
| OCBBF2011021 | 0.115923486 | 95125 | 60 |
| OCBBF2011073 | 0.049104411 | 110021 | 25 |
| OCBBF2011137 | 0.002081512 | 66313 | 1 |
| OCBBF2011160 | 0.009598522 | 104681 | 3 |
| OCBBF2011177 | 0.002081512 | 188019 | 1 |
| OCBBF2011228 | 0.007836972 | 86215 | 4 |
| OCBBF2011232 | 0.020912567 | 84828 | 10 |
| OCBBF2011283 | 0.028288408 | 181857 | 14 |
| OCBBF2011311 | 0.002081512 | 176508 | 1 |
| OCBBF2011536 | 0.08399109 | 86213 | 16 |
| OCBBF2011625 | 0.006258447 | 136410 | 2 |
| OCBBF2011669 | 0.003270756 | 181745 | 2 |
| OCBBF2011685 | 0.002081512 | 14778 | 1 |
| OCBBF2011706 | 0.002081512 | 163278 | 1 |
| OCBBF2011722 | 0.008326048 | 140784 | 4 |
| OCBBF2011759 | 0.17929562 | 81197 | 101 |
| OCBBF2011767 | 0.005633901 | 163811 | 3 |
| OCBBF2011855 | 0.002081512 | 157236 | 1 |
| OCBBF2011872 | 0.002081512 | 115593 | 1 |
| OCBBF2011887 | 0.035628456 | 162731 | 13 |
| OCBBF2011897 | 0.009705564 | 196278 | 3 |
| OCBBF2011914 | 0.045952233 | 160238 | 11 |
| OCBBF2011960 | 0.002081512 | 158501 | 1 |
| OCBBF2011981 | 0.002081512 | 157275 | 1 |
| OCBBF2012001 | 0.006729179 | 130426 | 4 |
| OCBBF2012028 | 0.002081512 | 100637 | 1 |
| OCBBF2012039 | 0.109245667 | 86993 | 63 |
| OCBBF2012095 | 0.010949866 | 109152 | 4 |
| OCBBF2012139 | 0.002081512 | 94801 | 1 |
| OCBBF2012191 | 0.005177008 | 39488 | 2 |
| OCBBF2012262 | 0.011552401 | 131464 | 5 |
| OCBBF2012320 | 0.002081512 | 172330 | 1 |
| OCBBF2012436 | 0.003478667 | 102904 | 2 |
| OCBBF2012525 | 0.007433781 | 153756 | 4 |
| OCBBF2012553 | 0.142863832 | 56815 | 26 |
| OCBBF2012678 | 0.024291424 | 140891 | 13 |
| OCBBF2012704 | 0.008025098 | 150129 | 4 |
| OCBBF2012714 | 0.028866765 | 147541 | 5 |
| OCBBF2012755 | 0.002081512 | 152756 | 1 |
| OCBBF2012812 | 0.00813588 | 97614 | 2 |
| OCBBF2012821 | 0.066966268 | 138033 | 10 |
| OCBBF2012881 | 0.003769789 | 199285 | 2 |
| OCBBF2012908 | 0.05113242 | 96930 | 17 |
| OCBBF2012936 | 0.003757059 | 11689 | 2 |
| OCBBF2012990 | 0.002081512 | 159752 | 1 |
| OCBBF2013011 | 0.022191901 | 142051 | 8 |
| OCBBF2013049 | 0.002081512 | 128337 | 1 |
| OCBBF2013091 | 0.002081512 | 104565 | 1 |
| OCBBF2013123 | 0.007343361 | 28827 | 4 |
| OCBBF2013127 | 0.002081512 | 281651 | 1 |
| OCBBF2013149 | 0.108029691 | 87706 | 34 |
| OCBBF2013208 | 0.002081512 | 74373 | 1 |
| OCBBF2013285 | 0.003270756 | 75509 | 2 |
| OCBBF2013319 | 0.002081512 | 103246 | 1 |
| OCBBF2013324 | 0.004460001 | 87179 | 3 |
| OCBBF2013366 | 0.062073628 | 77068 | 9 |
| OCBBF2013481 | 0.030648354 | 61532 | 12 |
| OCBBF2013507 | 0.002081512 | 110930 | 1 |
| OCBBF2013721 | 0.002081512 | 209201 | 1 |
| OCBBF2013789 | 0.011102604 | 163465 | 4 |
| OCBBF2013843 | 0.313578061 | 44329 | 66 |
| OCBBF2013883 | 0.008153895 | 118909 | 2 |
| OCBBF2013926 | 0.013503056 | 181760 | 6 |
| OCBBF2014052 | 0.003958354 | 165252 | 2 |
| OCBBF2014064 | 0.122675426 | 117126 | 20 |
| OCBBF2014089 | 0.008490217 | 32775 | 4 |
| OCBBF2014292 | 0.161779207 | 27574 | 48 |
| OCBBF2014322 | 0.008116561 | 83650 | 3 |
| OCBBF2014386 | 0.009215291 | 139111 | 3 |
| OCBBF2014541 | 0.042734607 | 49300 | 15 |
| OCBBF2014576 | 0.045856326 | 173185 | 6 |
| OCBBF2014707 | 0.002081512 | 11378 | 1 |
| OCBBF2014745 | 0.003270756 | 169866 | 2 |
| OCBBF2014828 | 0.002081512 | 241112 | 1 |
| OCBBF2014873 | 0.011813937 | 114131 | 7 |
| OCBBF2014880 | 0.005177008 | 133168 | 2 |
| OCBBF2014928 | 0.005177008 | 43556 | 2 |
| OCBBF2015031 | 0.002081512 | 134973 | 1 |
| OCBBF2015081 | 0.002081512 | 162891 | 1 |
| OCBBF2015115 | 0.009390551 | 131549 | 4 |
| OCBBF2015232 | 0.002081512 | 153786 | 1 |
| OCBBF2015233 | 0.005352268 | 172085 | 3 |
| OCBBF2015285 | 0.002081512 | 187182 | 1 |
| OCBBF2015334 | 0.00317679 | 102309 | 2 |
| OCBBF2015335 | 0.002081512 | 62254 | 1 |
| OCBBF2015503 | 0.022327869 | 111563 | 8 |
| OCBBF2015506 | 0.006244536 | 128301 | 3 |
| OCBBF2015599 | 0.005177008 | 172068 | 2 |
| OCBBF2015645 | 0.002081512 | 132872 | 1 |
| OCBBF2015659 | 0.002081512 | 116698 | 1 |
| OCBBF2015751 | 0.059125739 | 177150 | 9 |
| OCBBF2015797 | 0.00317679 | 31555 | 2 |
| OCBBF2015931 | 0.00831787 | 187073 | 2 |
| OCBBF2015980 | 0.002081512 | 153022 | 1 |
| OCBBF2016038 | 0.002081512 | 5698 | 1 |
| OCBBF2016405 | 0.021722016 | 146920 | 8 |
| OCBBF2016467 | 0.002081512 | 135030 | 1 |
| OCBBF2016590 | 0.002081512 | 189851 | 1 |
| OCBBF2016591 | 0.002081512 | 91643 | 1 |
| OCBBF2016612 | 0.003958354 | 242051 | 2 |
| OCBBF2016689 | 0.005352268 | 45072 | 3 |
| OCBBF2016690 | 0.002081512 | 130138 | 1 |
| OCBBF2016729 | 0.01838075 | 102420 | 8 |
| OCBBF2016841 | 0.027428655 | 89112 | 4 |
| OCBBF2016928 | 0.061183899 | 166278 | 10 |
| OCBBF2017035 | 0.138211456 | 118822 | 32 |
| OCBBF2017055 | 0.009338148 | 133794 | 5 |
| OCBBF2017069 | 0.002081512 | 282867 | 1 |
| OCBBF2017306 | 0.003757059 | 172093 | 2 |
| OCBBF2017325 | 0.002081512 | 18917 | 1 |
| OCBBF2017398 | 0.130254954 | 84775 | 43 |
| OCBBF2017458 | 0.03009693 | 120303 | 11 |
| OCBBF2017489 | 0.002081512 | 83053 | 1 |
| OCBBF2017516 | 0.002081512 | 163975 | 1 |
| OCBBF2017665 | 0.002081512 | 209622 | 1 |
| OCBBF2017754 | 0.04462691 | 85474 | 22 |
| OCBBF2017791 | 0.130817703 | 70226 | 51 |
| OCBBF2017815 | 0.00317679 | 48226 | 2 |
| OCBBF2017882 | 0.023484027 | 93895 | 8 |
| OCBBF2017888 | 0.009834514 | 101169 | 4 |
| OCBBF2017893 | 0.096598963 | 72639 | 14 |
| OCBBF2017899 | 0.089875138 | 165062 | 8 |
| OCBBF2017920 | 0.013012814 | 80716 | 6 |
| OCBBF2018012 | 0.059697615 | 78102 | 11 |
| OCBBF2018014 | 0.002081512 | 52945 | 1 |
| OCBBF2018084 | 0.037998299 | 133834 | 10 |
| OCBBF2018167 | 0.002081512 | 110333 | 1 |
| OCBBF2018206 | 0.003790972 | 48642 | 2 |
| OCBBF2018229 | 0.002081512 | 149607 | 1 |
| OCBBF2018234 | 0.025868858 | 51553 | 13 |
| OCBBF2018362 | 0.00728383 | 95400 | 3 |
| OCBBF2018380 | 0.002081512 | 176886 | 1 |
| OCBBF2018563 | 0.008147311 | 94684 | 5 |
| OCBBF2018581 | 0.038946716 | 185391 | 10 |
| OCBBF2018618 | 0.010331586 | 144649 | 6 |
| OCBBF2018663 | 0.002081512 | 95263 | 1 |
| OCBBF2018687 | 0.002081512 | 173151 | 1 |
| OCBBF2018707 | 0.074503849 | 44141 | 14 |
| OCBBF2018827 | 0.034148765 | 133435 | 14 |
| OCBBF2018828 | 0.002081512 | 243418 | 1 |
| OCBBF2018873 | 0.120916169 | 74910 | 23 |
| OCBBF2018934 | 0.002081512 | 163061 | 1 |
| OCBBF2018956 | 0.099003332 | 131743 | 16 |
| OCBBF2019108 | 0.004875823 | 116958 | 3 |
| OCBBF2019195 | 0.002081512 | 66329 | 1 |
| OCBBF2019327 | 0.002081512 | 109165 | 1 |
| OCBBF2019684 | 0.002081512 | 148505 | 1 |
| OCBBF2019761 | 0.002081512 | 166324 | 1 |
| OCBBF2019823 | 0.002081512 | 268450 | 1 |
| OCBBF2019950 | 0.003790972 | 6731 | 2 |
| OCBBF2020048 | 0.00317679 | 76141 | 2 |
| OCBBF2020318 | 0.003478667 | 172434 | 2 |
| OCBBF2020343 | 0.003270756 | 84376 | 2 |
| OCBBF2020453 | 0.002081512 | 198562 | 1 |
| OCBBF2020639 | 0.007418294 | 127946 | 4 |
| OCBBF2020741 | 0.011875724 | 102282 | 7 |
| OCBBF2020801 | 0.003478667 | 167537 | 2 |
| OCBBF2020825 | 0.002081512 | 133139 | 1 |
| OCBBF2020838 | 0.002081512 | 280456 | 1 |
| OCBBF2021020 | 0.062844985 | 61109 | 4 |
| OCBBF2021214 | 0.002081512 | 264551 | 1 |
| OCBBF2021286 | 0.018157332 | 117157 | 9 |
| OCBBF2021323 | 0.002081512 | 172759 | 1 |
| OCBBF2021518 | 0.002081512 | 158404 | 1 |
| OCBBF2021788 | 0.002081512 | 282481 | 1 |
| OCBBF2021833 | 0.011553201 | 157205 | 6 |
| OCBBF2022351 | 0.003270756 | 67225 | 2 |
| OCBBF2022573 | 0.002081512 | 178307 | 1 |
| OCBBF2022574 | 0.004163024 | 90390 | 2 |
| OCBBF2023162 | 0.016296206 | 166299 | 6 |
| OCBBF2023545 | 0.02005848 | 135477 | 9 |
| OCBBF2023598 | 0.002081512 | 166295 | 1 |
| OCBBF2023643 | 0.002081512 | 197431 | 1 |
| OCBBF2024284 | 0.018984488 | 193278 | 7 |
| OCBBF2024463 | 0.002081512 | 154001 | 1 |
| OCBBF2024589 | 0.002081512 | 285660 | 1 |
| OCBBF2024719 | 0.005649087 | 144920 | 3 |
| OCBBF2024779 | 0.002081512 | 206643 | 1 |
| OCBBF2024781 | 0.002081512 | 275016 | 1 |
| OCBBF2024850 | 0.075396546 | 137903 | 33 |
| OCBBF2025028 | 0.002081512 | 144418 | 1 |
| OCBBF2025115 | 0.002081512 | 157707 | 1 |
| OCBBF2025198 | 0.002081512 | 143253 | 1 |
| OCBBF2025451 | 0.002081512 | 283335 | 1 |
| OCBBF2025458 | 0.002081512 | 285593 | 1 |
| OCBBF2025527 | 0.003270756 | 8698 | 2 |
| OCBBF2025631 | 0.002081512 | 277587 | 1 |
| OCBBF2025730 | 0.002081512 | 181776 | 1 |
| OCBBF2026025 | 0.002081512 | 278096 | 1 |
| OCBBF2026035 | 0.002081512 | 144199 | 1 |
| OCBBF2026144 | 0.002081512 | 271183 | 1 |
| OCBBF2026368 | 0.020277754 | 135511 | 9 |
| OCBBF2026645 | 0.002081512 | 150997 | 1 |
| OCBBF2026649 | 0.003270756 | 164049 | 2 |
| OCBBF2026690 | 0.006244536 | 153876 | 3 |
| OCBBF2026981 | 0.004163024 | 135734 | 2 |
| OCBBF2027148 | 0.00720072 | 36165 | 3 |
| OCBBF2027197 | 0.003270756 | 158730 | 2 |
| OCBBF2027354 | 0.002081512 | 145847 | 1 |
| OCBBF2027423 | 0.002081512 | 128117 | 1 |
| OCBBF2027478 | 0.002081512 | 156778 | 1 |
| OCBBF2027481 | 0.002081512 | 277571 | 1 |
| OCBBF2027511 | 0.002081512 | 270240 | 1 |
| OCBBF2027661 | 0.004460001 | 147148 | 3 |
| OCBBF2027728 | 0.002081512 | 173308 | 1 |
| OCBBF2027782 | 0.002081512 | 166305 | 1 |
| OCBBF2027868 | 0.002081512 | 159722 | 1 |
| OCBBF2028055 | 0.007514118 | 128278 | 4 |
| OCBBF2028173 | 0.002081512 | 181827 | 1 |
| OCBBF2028421 | 0.003958354 | 110875 | 2 |
| OCBBF2028935 | 0.14472965 | 126539 | 32 |
| OCBBF2029471 | 0.002081512 | 275248 | 1 |
| OCBBF2029901 | 0.002081512 | 88476 | 1 |
| OCBBF2030116 | 0.003790972 | 46886 | 2 |
| OCBBF2030329 | 0.003769789 | 95895 | 2 |
| OCBBF2030354 | 0.002081512 | 144343 | 1 |
| OCBBF2030517 | 0.002081512 | 206438 | 1 |
| OCBBF2030574 | 0.002081512 | 268848 | 1 |
| OCBBF2030708 | 0.002081512 | 246574 | 1 |
| OCBBF2030927 | 0.003769789 | 152312 | 2 |
| OCBBF2030963 | 0.002081512 | 281268 | 1 |
| OCBBF2031167 | 0.002081512 | 164204 | 1 |
| OCBBF2031198 | 0.002081512 | 254832 | 1 |
| OCBBF2031366 | 0.002081512 | 278251 | 1 |
| OCBBF2031561 | 0.005336782 | 87511 | 3 |
| OCBBF2032152 | 0.002081512 | 181495 | 1 |
| OCBBF2032274 | 0.002081512 | 269556 | 1 |
| OCBBF2032539 | 0.002081512 | 218009 | 1 |
| OCBBF2032590 | 0.002081512 | 222070 | 1 |
| OCBBF2032599 | 0.002081512 | 264341 | 1 |
| OCBBF2032611 | 0.002081512 | 276608 | 1 |
| OCBBF2032671 | 0.002081512 | 236779 | 1 |
| OCBBF2032968 | 0.007655602 | 199666 | 3 |
| OCBBF2033295 | 0.03186685 | 75220 | 15 |
| OCBBF2033413 | 0.002081512 | 262312 | 1 |
| OCBBF2033869 | 0.002081512 | 219482 | 1 |
| OCBBF2033948 | 0.002081512 | 246880 | 1 |
| OCBBF2034333 | 0.002081512 | 203604 | 1 |
| OCBBF2034529 | 0.002081512 | 13135 | 1 |
| OCBBF2034637 | 0.002081512 | 157900 | 1 |
| OCBBF2034823 | 0.026992367 | 145607 | 10 |
| OCBBF2034906 | 0.002081512 | 235119 | 1 |
| OCBBF2035110 | 0.002081512 | 194719 | 1 |
| OCBBF2035214 | 0.003769789 | 243761 | 2 |
| OCBBF2035226 | 0.285219087 | 127332 | 13 |
| OCBBF2035390 | 0.002081512 | 108071 | 1 |
| OCBBF2035564 | 0.002081512 | 194785 | 1 |
| OCBBF2035658 | 0.002081512 | 262840 | 1 |
| OCBBF2035823 | 0.004875823 | 178243 | 3 |
| OCBBF2035885 | 0.002081512 | 276141 | 1 |
| OCBBF2035916 | 0.002081512 | 280971 | 1 |
| OCBBF2036019 | 0.002081512 | 102327 | 1 |
| OCBBF2036031 | 0.002081512 | 256314 | 1 |
| OCBBF2036225 | 0.002081512 | 270805 | 1 |
| OCBBF2036476 | 0.002081512 | 235049 | 1 |
| OCBBF2036743 | 0.015159008 | 105049 | 9 |
| OCBBF2036752 | 0.002081512 | 280646 | 1 |
| OCBBF2037068 | 0.011198776 | 66296 | 3 |
| OCBBF2037340 | 0.002081512 | 272665 | 1 |
| OCBBF2037398 | 0.002081512 | 163268 | 1 |
| OCBBF2037464 | 0.002081512 | 280399 | 1 |
| OCBBF2037547 | 0.002081512 | 273560 | 1 |
| OCBBF2037598 | 0.002081512 | 139045 | 1 |
| OCBBF2037638 | 0.002081512 | 264271 | 1 |
| OCBBF2038238 | 0.002081512 | 251782 | 1 |
| OCBBF2038285 | 0.002081512 | 228649 | 1 |
| OCBBF2038317 | 0.016999458 | 138478 | 5 |
| OCBBF3000097 | 0.002081512 | 281772 | 1 |
| OCBBF3000167 | 0.008353798 | 19003 | 4 |
| OCBBF3000213 | 0.002081512 | 276009 | 1 |
| OCBBF3000296 | 0.008844604 | 161430 | 5 |
| OCBBF3000323 | 0.050379897 | 39254 | 19 |
| OCBBF3000372 | 0.002081512 | 217466 | 1 |
| OCBBF3000380 | 0.002081512 | 213957 | 1 |
| OCBBF3000483 | 0.041984388 | 80345 | 22 |
| OCBBF3000743 | 0.002081512 | 194802 | 1 |
| OCBBF3000830 | 0.002081512 | 209136 | 1 |
| OCBBF3001076 | 0.002081512 | 251737 | 1 |
| OCBBF3001202 | 0.002081512 | 252374 | 1 |
| OCBBF3001333 | 0.052150856 | 56997 | 22 |
| OCBBF3001616 | 0.002081512 | 191894 | 1 |
| OCBBF3002553 | 0.002081512 | 274593 | 1 |
| OCBBF3002600 | 0.002081512 | 277878 | 1 |
| OCBBF3002654 | 0.002081512 | 261718 | 1 |
| OCBBF3003103 | 0.003757059 | 31962 | 2 |
| OCBBF3003209 | 0.002081512 | 240784 | 1 |
| OCBBF3003320 | 0.005177008 | 148775 | 2 |
| OCBBF3003592 | 0.002081512 | 234918 | 1 |
| OCBBF3003745 | 0.002081512 | 267007 | 1 |
| OCBBF3003761 | 0.002081512 | 247541 | 1 |
| OCBBF3004264 | 0.002081512 | 262459 | 1 |
| OCBBF3004314 | 0.002081512 | 152663 | 1 |
| OCBBF3004487 | 0.036981384 | 19406 | 15 |
| OCBBF3004908 | 0.017518546 | 186256 | 5 |
| OCBBF3004972 | 0.002081512 | 245944 | 1 |
| OCBBF3005330 | 0.002081512 | 243877 | 1 |
| OCBBF3005597 | 0.002081512 | 251866 | 1 |
| OCBBF3005843 | 0.022896632 | 273128 | 11 |
| OCBBF3006802 | 0.004163024 | 176100 | 2 |
| OCBBF3006986 | 0.002081512 | 237690 | 1 |
| OCBBF3007078 | 0.004163024 | 136202 | 2 |
| OCBBF3007516 | 0.044302137 | 196080 | 4 |
| OCBBF3007704 | 0.002081512 | 237669 | 1 |
| OCBBF3008230 | 0.005615173 | 156207 | 3 |
| OCBBF3008392 | 0.002081512 | 41930 | 1 |
| OCBBF3008835 | 0.003939626 | 130734 | 2 |
| OCBBF3009192 | 0.002081512 | 229002 | 1 |
| OCBBF3009244 | 0.004865067 | 144341 | 3 |
| OCBBF3009279 | 0.002081512 | 68399 | 1 |
| PANCR1000012 | 0.549450549 | 281178 | 1 |
| PANCR1000021 | 0.549450549 | 6611 | 1 |
| PANCR1000081 | 0.560277747 | 79048 | 2 |
| PANCR1000086 | 0.549450549 | 10577 | 1 |
| PANCR1000091 | 0.556842114 | 46146 | 3 |
| PANCR1000185 | 0.549450549 | 38273 | 1 |
| PEBLM1000024 | 0.012594458 | 30084 | 1 |
| PEBLM1000029 | 0.012594458 | 33721 | 1 |
| PEBLM1000034 | 0.02409929 | 15430 | 2 |
| PEBLM1000062 | 0.012594458 | 52061 | 1 |
| PEBLM1000066 | 0.035356037 | 38785 | 6 |
| PEBLM1000068 | 0.012594458 | 73905 | 1 |
| PEBLM1000071 | 0.012594458 | 167585 | 1 |
| PEBLM1000083 | 0.012594458 | 39070 | 1 |
| PEBLM1000144 | 0.027938227 | 19967 | 7 |
| PEBLM1000147 | 0.012594458 | 62124 | 1 |
| PEBLM1000174 | 0.035036537 | 77263 | 7 |
| PEBLM1000180 | 0.012594458 | 15902 | 1 |
| PEBLM2000030 | 0.045526516 | 135158 | 24 |
| PEBLM2000112 | 0.012594458 | 145845 | 1 |
| PEBLM2000126 | 0.015509315 | 126919 | 2 |
| PEBLM2000147 | 0.131342074 | 133636 | 30 |
| PEBLM2000170 | 0.012594458 | 207155 | 1 |
| PEBLM2000180 | 0.012594458 | 275815 | 1 |
| PEBLM2000213 | 0.012594458 | 135244 | 1 |
| PEBLM2000222 | 0.288461073 | 97799 | 16 |
| PEBLM2000248 | 0.012594458 | 129607 | 1 |
| PEBLM2000267 | 0.119163955 | 133717 | 26 |
| PEBLM2000270 | 0.012594458 | 18974 | 1 |
| PEBLM2000308 | 0.084519324 | 102916 | 25 |
| PEBLM2000321 | 0.012594458 | 67784 | 1 |
| PEBLM2000326 | 0.03086749 | 18696 | 13 |
| PEBLM2000333 | 0.012594458 | 156538 | 1 |
| PEBLM2000338 | 0.017605506 | 143249 | 3 |
| PEBLM2000395 | 0.184092338 | 125656 | 30 |
| PEBLM2000479 | 0.035996212 | 126972 | 8 |
| PEBLM2000502 | 0.012594458 | 186313 | 1 |
| PEBLM2001072 | 0.092396545 | 85008 | 27 |
| PEBLM2001312 | 0.012594458 | 181594 | 1 |
| PEBLM2001465 | 0.025188917 | 48617 | 2 |
| PEBLM2001488 | 0.012594458 | 238124 | 1 |
| PEBLM2001803 | 0.01620835 | 126327 | 2 |
| PEBLM2002432 | 0.012594458 | 54344 | 1 |
| PEBLM2002455 | 0.018830816 | 189300 | 2 |
| PEBLM2002594 | 0.012594458 | 189923 | 1 |
| PEBLM2002749 | 0.024381351 | 257254 | 2 |
| PEBLM2002887 | 0.012594458 | 41861 | 1 |
| PEBLM2003426 | 0.012594458 | 142061 | 1 |
| PEBLM2003765 | 0.025188917 | 169706 | 2 |
| PEBLM2003914 | 0.078859739 | 96721 | 24 |
| PEBLM2003935 | 0.012594458 | 228274 | 1 |
| PEBLM2004015 | 0.012594458 | 189214 | 1 |
| PEBLM2004216 | 0.012594458 | 169716 | 1 |
| PEBLM2004290 | 0.012594458 | 225813 | 1 |
| PEBLM2004452 | 0.124147681 | 111666 | 56 |
| PEBLM2004497 | 0.012594458 | 255829 | 1 |
| PEBLM2004666 | 0.045096694 | 95135 | 10 |
| PEBLM2004733 | 0.017533821 | 136973 | 3 |
| PEBLM2005183 | 0.044522139 | 129431 | 4 |
| PEBLM2005282 | 0.014282735 | 149592 | 2 |
| PEBLM2005615 | 0.012594458 | 57736 | 1 |
| PEBLM2005632 | 0.012594458 | 62684 | 1 |
| PEBLM2005697 | 0.012594458 | 141511 | 1 |
| PEBLM2006031 | 0.012594458 | 21395 | 1 |
| PEBLM2006036 | 0.013991614 | 104483 | 2 |
| PEBLM2006049 | 0.012594458 | 48934 | 1 |
| PEBLM2006113 | 0.012594458 | 247663 | 1 |
| PEBLM2006135 | 0.012594458 | 225736 | 1 |
| PEBLM2006215 | 0.012594458 | 109761 | 1 |
| PEBLM2006283 | 0.014303919 | 167957 | 2 |
| PEBLM2006298 | 0.012594458 | 222370 | 1 |
| PEBLM2006366 | 0.060539691 | 9710 | 8 |
| PEBLM2006709 | 0.030116788 | 15102 | 5 |
| PEBLM2006912 | 0.012594458 | 281147 | 1 |
| PEBLM2007112 | 0.012594458 | 167786 | 1 |
| PEBLM2007140 | 0.012594458 | 157636 | 1 |
| PEBLM2007188 | 0.019193932 | 107228 | 4 |
| PEBLM2007296 | 0.096888444 | 69233 | 9 |
| PEBLM2007437 | 0.028103303 | 164651 | 3 |
| PEBLM2007447 | 0.013991614 | 17001 | 2 |
| PEBLM2007598 | 0.025188917 | 113957 | 2 |
| PEBLM2007774 | 0.556323895 | 32081 | 36 |
| PEBLM2007832 | 0.012594458 | 157434 | 1 |
| PEBLM2007834 | 0.012594458 | 280260 | 1 |
| PEBLM2008106 | 0.015715265 | 39307 | 2 |
| PEBLM2008576 | 0.012594458 | 83491 | 1 |
| PEBLM2008605 | 0.012594458 | 46058 | 1 |
| PEBLM2008826 | 0.018066465 | 88934 | 3 |
| PEBLM2008861 | 0.069609533 | 126855 | 4 |
| PERIC1000025 | 0.011286682 | 103613 | 1 |
| PERIC1000147 | 0.011286682 | 215907 | 1 |
| PERIC2000180 | 0.011286682 | 70562 | 1 |
| PERIC2000214 | 0.011286682 | 200235 | 1 |
| PERIC2000386 | 0.011286682 | 132677 | 1 |
| PERIC2000387 | 0.011286682 | 64701 | 1 |
| PERIC2000422 | 0.011286682 | 130524 | 1 |
| PERIC2000473 | 0.026691893 | 86448 | 5 |
| PERIC2000478 | 0.015390783 | 193727 | 3 |
| PERIC2000889 | 0.018835355 | 30504 | 6 |
| PERIC2000914 | 0.011286682 | 166331 | 1 |
| PERIC2001227 | 0.011286682 | 189726 | 1 |
| PERIC2001228 | 0.011286682 | 181564 | 1 |
| PERIC2001379 | 0.011286682 | 227518 | 1 |
| PERIC2001504 | 0.011286682 | 169499 | 1 |
| PERIC2001703 | 0.011286682 | 178937 | 1 |
| PERIC2001764 | 0.011286682 | 199841 | 1 |
| PERIC2002243 | 0.011286682 | 213513 | 1 |
| PERIC2002272 | 0.011286682 | 178940 | 1 |
| PERIC2002452 | 0.017198944 | 187738 | 2 |
| PERIC2002766 | 0.013368194 | 166329 | 2 |
| PERIC2003001 | 0.011286682 | 198035 | 1 |
| PERIC2003090 | 0.033860045 | 173088 | 3 |
| PERIC2003287 | 0.011286682 | 187046 | 1 |
| PERIC2003349 | 0.011286682 | 176351 | 1 |
| PERIC2003452 | 0.011286682 | 171552 | 1 |
| PERIC2003699 | 0.014268641 | 105281 | 2 |
| PERIC2003720 | 0.012974958 | 160208 | 2 |
| PERIC2003794 | 0.01238196 | 162130 | 2 |
| PERIC2003834 | 0.011286682 | 214823 | 1 |
| PERIC2003919 | 0.011286682 | 281469 | 1 |
| PERIC2003957 | 0.011286682 | 206183 | 1 |
| PERIC2004028 | 0.011286682 | 207520 | 1 |
| PERIC2004131 | 0.011286682 | 221197 | 1 |
| PERIC2004259 | 0.011286682 | 157909 | 1 |
| PERIC2004379 | 0.011286682 | 280422 | 1 |
| PERIC2004429 | 0.011286682 | 214091 | 1 |
| PERIC2004510 | 0.011286682 | 280236 | 1 |
| PERIC2004613 | 0.011286682 | 207755 | 1 |
| PERIC2004909 | 0.011286682 | 259979 | 1 |
| PERIC2005111 | 0.011286682 | 153626 | 1 |
| PERIC2005117 | 0.077998528 | 112140 | 7 |
| PERIC2005305 | 0.011286682 | 205154 | 1 |
| PERIC2005347 | 0.011286682 | 207648 | 1 |
| PERIC2005370 | 0.011286682 | 207645 | 1 |
| PERIC2005936 | 0.011286682 | 171566 | 1 |
| PERIC2006035 | 0.011286682 | 200478 | 1 |
| PERIC2006121 | 0.011286682 | 279172 | 1 |
| PERIC2006443 | 0.034500115 | 79859 | 4 |
| PERIC2006945 | 0.011286682 | 196826 | 1 |
| PERIC2006960 | 0.011286682 | 281970 | 1 |
| PERIC2007068 | 0.108591717 | 109548 | 42 |
| PERIC2007439 | 0.011286682 | 161406 | 1 |
| PERIC2007914 | 0.011286682 | 86598 | 1 |
| PERIC2008008 | 0.011286682 | 133478 | 1 |
| PERIC2008385 | 0.011286682 | 275402 | 1 |
| PERIC2009001 | 0.011286682 | 125305 | 1 |
| PERIC2009086 | 0.011286682 | 281601 | 1 |
| PERIC2009329 | 0.011286682 | 243202 | 1 |
| PERIC2009566 | 0.011286682 | 228471 | 1 |
| PLACE5000001 | 0.002981959 | 32814 | 1 |
| PLACE5000013 | 0.105555352 | 59531 | 32 |
| PLACE5000037 | 0.002981959 | 58436 | 1 |
| PLACE5000058 | 0.004840074 | 72392 | 2 |
| PLACE5000066 | 0.01831269 | 18212 | 5 |
| PLACE5000067 | 0.004077237 | 71744 | 2 |
| PLACE5000068 | 0.026680727 | 46897 | 5 |
| PLACE5000070 | 0.002981959 | 41653 | 1 |
| PLACE5000080 | 0.013584164 | 78840 | 2 |
| PLACE5000105 | 0.002981959 | 56014 | 1 |
| PLACE5000113 | 0.097388055 | 57624 | 45 |
| PLACE5000115 | 0.002981959 | 43408 | 1 |
| PLACE5000116 | 0.002981959 | 29697 | 1 |
| PLACE5000129 | 0.002981959 | 9718 | 1 |
| PLACE5000139 | 0.014268641 | 162829 | 2 |
| PLACE5000153 | 0.002981959 | 21397 | 1 |
| PLACE5000159 | 0.073725999 | 66100 | 32 |
| PLACE5000170 | 0.048192951 | 91940 | 17 |
| PLACE5000171 | 0.002981959 | 73763 | 1 |
| PLACE5000172 | 0.128909714 | 78101 | 44 |
| PLACE5000178 | 0.004657506 | 84669 | 2 |
| PLACE5000184 | 0.040642311 | 62231 | 14 |
| PLACE5000245 | 0.002981959 | 211938 | 1 |
| PLACE5000260 | 0.002981959 | 114167 | 1 |
| PLACE5000282 | 0.013584164 | 37600 | 2 |
| PLACE5000297 | 0.004691419 | 225360 | 2 |
| PLACE5000372 | 0.00498036 | 187595 | 2 |
| PLACE5000492 | 0.006826043 | 52889 | 2 |
| PLACE5000522 | 0.002981959 | 73488 | 1 |
| PLACE5000527 | 0.002981959 | 61350 | 1 |
| PLACE6000012 | 0.002981959 | 142402 | 1 |
| PLACE6000044 | 0.002981959 | 233552 | 1 |
| PLACE6000055 | 0.002981959 | 159292 | 1 |
| PLACE6000070 | 0.002981959 | 282621 | 1 |
| PLACE6000080 | 0.040733555 | 194458 | 4 |
| PLACE6000137 | 0.002981959 | 137073 | 1 |
| PLACE6000145 | 0.16167989 | 158642 | 9 |
| PLACE6000191 | 0.002981959 | 86527 | 1 |
| PLACE6000205 | 0.002981959 | 231541 | 1 |
| PLACE6000231 | 0.002981959 | 246390 | 1 |
| PLACE6000263 | 0.110207265 | 38307 | 27 |
| PLACE6000296 | 0.002981959 | 69473 | 1 |
| PLACE6000314 | 0.0109856 | 145628 | 3 |
| PLACE6000325 | 0.020683518 | 151732 | 5 |
| PLACE6000348 | 0.011769097 | 199737 | 3 |
| PLACE6000371 | 0.005896816 | 216226 | 2 |
| PLACE6000379 | 0.948638408 | 8957 | 184 |
| PLACE6000414 | 0.130761853 | 102490 | 34 |
| PLACE6000424 | 0.002981959 | 246212 | 1 |
| PLACE6000426 | 0.002981959 | 282680 | 1 |
| PLACE6000429 | 0.03578351 | 150571 | 12 |
| PLACE6000450 | 0.002981959 | 233544 | 1 |
| PLACE6000463 | 0.021474356 | 108850 | 7 |
| PLACE6000481 | 0.068082902 | 98528 | 25 |
| PLACE6000523 | 0.002981959 | 241171 | 1 |
| PLACE6000550 | 0.002981959 | 262030 | 1 |
| PLACE6000552 | 0.002981959 | 261505 | 1 |
| PLACE6000555 | 0.002981959 | 224941 | 1 |
| PLACE6000619 | 0.004670236 | 147238 | 2 |
| PLACE6000630 | 0.043424282 | 120712 | 4 |
| PLACE6000953 | 0.002981959 | 202422 | 1 |
| PLACE6001064 | 0.292054675 | 45807 | 103 |
| PLACE6001118 | 0.002981959 | 244197 | 1 |
| PLACE6001153 | 0.002981959 | 251398 | 1 |
| PLACE6001185 | 0.007845152 | 142257 | 4 |
| PLACE6001262 | 0.019175337 | 129776 | 4 |
| PLACE6001281 | 0.048671412 | 104937 | 15 |
| PLACE6001294 | 0.002981959 | 67215 | 1 |
| PLACE6001443 | 0.085304876 | 86362 | 19 |
| PLACE6001536 | 0.002981959 | 266076 | 1 |
| PLACE6001557 | 0.014909796 | 177257 | 5 |
| PLACE6001712 | 0.005963918 | 224885 | 2 |
| PLACE6001740 | 0.002981959 | 81387 | 1 |
| PLACE6001823 | 0.005880664 | 124934 | 3 |
| PLACE6001886 | 0.009893619 | 172644 | 4 |
| PLACE6001923 | 0.026657538 | 188536 | 4 |
| PLACE6001925 | 0.002981959 | 223800 | 1 |
| PLACE6001933 | 0.002981959 | 237523 | 1 |
| PLACE6002011 | 0.005896816 | 147840 | 2 |
| PLACE6002016 | 0.002981959 | 92029 | 1 |
| PLACE6002056 | 0.005896816 | 176255 | 2 |
| PLACE6002084 | 0.453700393 | 54927 | 62 |
| PLACE6002102 | 0.118635564 | 90575 | 55 |
| PLACE6002151 | 0.084519324 | 102916 | 25 |
| PLACE6002157 | 0.002981959 | 280333 | 1 |
| PLACE6002312 | 0.002981959 | 268063 | 1 |
| PLACE6002323 | 0.005963918 | 129204 | 2 |
| PLACE6002345 | 0.002981959 | 58982 | 1 |
| PLACE6002419 | 0.016072744 | 150462 | 3 |
| PLACE6002462 | 0.002981959 | 102861 | 1 |
| PLACE6002647 | 0.002981959 | 233924 | 1 |
| PLACE6002668 | 0.031047649 | 35038 | 7 |
| PLACE6002692 | 0.004670236 | 165921 | 2 |
| PLACE6002699 | 0.002981959 | 141477 | 1 |
| PLACE6002710 | 0.002981959 | 152059 | 1 |
| PLACE6002871 | 0.002981959 | 224996 | 1 |
| PLACE6002949 | 0.002981959 | 227915 | 1 |
| PLACE6002960 | 0.150965242 | 108462 | 38 |
| PLACE6003004 | 0.004840074 | 10286 | 2 |
| PLACE6003038 | 0.04642828 | 189643 | 7 |
| PLACE6003077 | 0.006595851 | 10590 | 2 |
| PLACE6003094 | 0.005963918 | 141265 | 2 |
| PLACE6003109 | 0.002981959 | 3992 | 1 |
| PLACE6003204 | 0.007639465 | 189843 | 3 |
| PLACE6003218 | 0.011514708 | 137767 | 5 |
| PLACE6003372 | 0.002981959 | 209685 | 1 |
| PLACE6003383 | 0.140382946 | 157299 | 20 |
| PLACE6003393 | 0.022065385 | 58710 | 4 |
| PLACE6003399 | 0.002981959 | 282920 | 1 |
| PLACE6003539 | 0.002981959 | 187678 | 1 |
| PLACE6003621 | 0.004171204 | 191249 | 2 |
| PLACE6003705 | 0.002981959 | 209018 | 1 |
| PLACE6003740 | 0.002981959 | 193349 | 1 |
| PLACE6003745 | 0.0318873 | 114630 | 10 |
| PLACE6003771 | 0.002981959 | 244586 | 1 |
| PLACE6003796 | 0.002981959 | 246821 | 1 |
| PLACE6003839 | 0.002981959 | 244603 | 1 |
| PLACE6003850 | 0.014936137 | 203681 | 3 |
| PLACE6004005 | 0.169706805 | 109741 | 51 |
| PLACE6004101 | 0.037607998 | 212276 | 2 |
| PLACE6004219 | 0.002981959 | 22188 | 1 |
| PLACE6004312 | 0.277824579 | 74045 | 42 |
| PLACE6004336 | 0.002981959 | 219713 | 1 |
| PLACE6004368 | 0.002981959 | 178791 | 1 |
| PLACE6004380 | 0.002981959 | 174613 | 1 |
| PLACE6004396 | 0.009127485 | 128856 | 2 |
| PLACE6004397 | 0.091161491 | 110015 | 21 |
| PLACE6004454 | 0.002981959 | 141920 | 1 |
| PLACE6004464 | 0.008878775 | 115119 | 3 |
| PLACE6004491 | 0.00498036 | 254838 | 2 |
| PLACE6004578 | 0.135172242 | 67455 | 19 |
| PLACE6004663 | 0.002981959 | 144252 | 1 |
| PLACE6004687 | 0.002981959 | 143498 | 1 |
| PLACE6004738 | 0.00796232 | 47684 | 3 |
| PLACE6004931 | 0.025588656 | 60981 | 3 |
| PLACE6004966 | 0.002981959 | 228624 | 1 |
| PLACE6004993 | 0.008787137 | 87962 | 2 |
| PLACE6005007 | 0.005963918 | 244655 | 2 |
| PLACE6005029 | 0.002981959 | 139585 | 1 |
| PLACE6005108 | 0.002981959 | 237247 | 1 |
| PLACE6005231 | 0.015170733 | 4887 | 9 |
| PLACE6005234 | 0.002981959 | 230943 | 1 |
| PLACE6005283 | 0.002981959 | 123008 | 1 |
| PLACE6005294 | 0.013915382 | 70074 | 4 |
| PLACE6005328 | 0.002981959 | 113499 | 1 |
| PLACE6005351 | 0.002981959 | 228275 | 1 |
| PLACE6005423 | 0.002981959 | 148085 | 1 |
| PLACE6005482 | 0.063366236 | 79410 | 29 |
| PLACE6005487 | 0.044787979 | 122090 | 2 |
| PLACE6005535 | 0.002981959 | 122731 | 1 |
| PLACE6005546 | 0.005963918 | 9510 | 2 |
| PLACE6005559 | 0.002981959 | 228221 | 1 |
| PLACE6005579 | 0.005963918 | 191997 | 2 |
| PLACE6005691 | 0.004670236 | 187091 | 2 |
| PLACE6005786 | 0.002981959 | 261980 | 1 |
| PLACE6006042 | 0.030779023 | 32799 | 7 |
| PLACE6006077 | 0.002981959 | 230941 | 1 |
| PLACE6006137 | 0.049056896 | 83677 | 20 |
| PLACE6006158 | 0.002981959 | 91461 | 1 |
| PLACE6006186 | 0.02316567 | 141263 | 7 |
| PLACE6006217 | 0.002981959 | 36239 | 1 |
| PLACE6006222 | 0.020053767 | 55170 | 6 |
| PLACE6006266 | 0.002981959 | 32020 | 1 |
| PLACE6006287 | 0.005963918 | 206102 | 2 |
| PLACE6006394 | 0.002981959 | 241272 | 1 |
| PLACE6006418 | 0.060438853 | 129737 | 11 |
| PLACE6006474 | 0.002981959 | 243949 | 1 |
| PLACE6006549 | 0.209837509 | 143567 | 24 |
| PLACE6006566 | 0.002981959 | 163855 | 1 |
| PLACE6006697 | 0.002981959 | 205332 | 1 |
| PLACE6006822 | 0.002981959 | 240828 | 1 |
| PLACE6006871 | 0.008945877 | 127180 | 3 |
| PLACE6006905 | 0.002981959 | 185464 | 1 |
| PLACE6006988 | 0.005063471 | 202659 | 2 |
| PLACE6007050 | 0.002981959 | 92951 | 1 |
| PLACE6007180 | 0.015634486 | 152461 | 9 |
| PLACE6007239 | 0.040642311 | 62231 | 14 |
| PLACE6007242 | 0.005963918 | 78475 | 2 |
| PLACE6007309 | 0.009218317 | 65055 | 2 |
| PLACE6007373 | 0.058550326 | 91565 | 4 |
| PLACE6007380 | 0.002981959 | 219634 | 1 |
| PLACE6007433 | 0.002981959 | 244442 | 1 |
| PLACE6007436 | 0.046638229 | 69178 | 13 |
| PLACE6007482 | 0.002981959 | 256521 | 1 |
| PLACE6007687 | 0.002981959 | 127517 | 1 |
| PLACE6007787 | 0.002981959 | 236738 | 1 |
| PLACE6007798 | 0.002981959 | 239802 | 1 |
| PLACE6007807 | 0.002981959 | 252721 | 1 |
| PLACE6007925 | 0.016534781 | 47690 | 3 |
| PLACE6008036 | 0.002981959 | 260248 | 1 |
| PLACE6008094 | 0.002981959 | 224813 | 1 |
| PLACE6008126 | 0.002981959 | 173845 | 1 |
| PLACE6008285 | 0.002981959 | 224811 | 1 |
| PLACE6008315 | 0.120334926 | 134760 | 17 |
| PLACE6008444 | 0.004670236 | 201715 | 2 |
| PLACE6008453 | 0.002981959 | 260252 | 1 |
| PLACE6008640 | 0.002981959 | 101822 | 1 |
| PLACE6008750 | 0.002981959 | 254292 | 1 |
| PLACE6008768 | 0.002981959 | 12946 | 1 |
| PLACE6008775 | 0.090907954 | 64913 | 40 |
| PLACE6008793 | 0.105679668 | 75205 | 10 |
| PLACE6008824 | 0.00498036 | 145425 | 2 |
| PLACE6008989 | 0.002981959 | 217252 | 1 |
| PLACE6009006 | 0.002981959 | 251955 | 1 |
| PLACE6009008 | 0.008924083 | 8038 | 2 |
| PLACE6009228 | 0.005896816 | 193730 | 2 |
| PLACE6009237 | 0.038966126 | 189299 | 2 |
| PLACE6009338 | 0.006668637 | 130112 | 3 |
| PLACE6009419 | 0.002981959 | 255777 | 1 |
| PLACE6009430 | 0.002981959 | 258442 | 1 |
| PLACE6009482 | 0.002981959 | 129207 | 1 |
| PLACE6009524 | 0.002981959 | 255553 | 1 |
| PLACE6009527 | 0.002981959 | 136976 | 1 |
| PLACE6009560 | 0.015505441 | 47014 | 2 |
| PLACE6009590 | 0.002981959 | 209325 | 1 |
| PLACE6009821 | 0.01088164 | 135568 | 3 |
| PLACE6009835 | 0.002981959 | 1907 | 1 |
| PLACE6009853 | 0.002981959 | 124518 | 1 |
| PLACE6010077 | 0.192446976 | 13832 | 21 |
| PLACE6010463 | 0.002981959 | 186685 | 1 |
| PLACE6010484 | 0.108402522 | 91463 | 21 |
| PLACE6010485 | 0.002981959 | 192009 | 1 |
| PLACE6010568 | 0.002981959 | 133287 | 1 |
| PLACE6010701 | 0.008924083 | 142837 | 2 |
| PLACE6010704 | 0.004657506 | 155333 | 2 |
| PLACE6010732 | 0.006826043 | 91093 | 2 |
| PLACE6010765 | 0.014268641 | 207183 | 2 |
| PLACE6010848 | 0.02047616 | 47531 | 5 |
| PLACE6010925 | 0.002981959 | 205614 | 1 |
| PLACE6010936 | 0.013616113 | 194062 | 5 |
| PLACE6010991 | 0.002981959 | 71143 | 1 |
| PLACE6011041 | 0.061584463 | 133852 | 10 |
| PLACE6011057 | 0.074343501 | 138019 | 22 |
| PLACE6011102 | 0.002981959 | 70787 | 1 |
| PLACE6011156 | 0.012867609 | 94430 | 3 |
| PLACE6011211 | 0.002981959 | 92518 | 1 |
| PLACE6011260 | 0.002981959 | 129293 | 1 |
| PLACE6011334 | 0.018642681 | 60769 | 10 |
| PLACE6011627 | 0.005063471 | 62845 | 2 |
| PLACE6011774 | 0.002981959 | 143073 | 1 |
| PLACE6011881 | 0.002981959 | 234564 | 1 |
| PLACE6011907 | 0.002981959 | 240727 | 1 |
| PLACE6011970 | 0.006460627 | 56455 | 3 |
| PLACE6011975 | 0.002981959 | 278157 | 1 |
| PLACE6012028 | 0.026692405 | 80766 | 7 |
| PLACE6012108 | 0.002981959 | 186848 | 1 |
| PLACE6012168 | 0.002981959 | 179133 | 1 |
| PLACE6012297 | 0.002981959 | 87637 | 1 |
| PLACE6012308 | 0.002981959 | 39673 | 1 |
| PLACE6012574 | 0.16393523 | 84610 | 8 |
| PLACE6012604 | 0.007293972 | 53766 | 3 |
| PLACE6012908 | 0.002981959 | 255215 | 1 |
| PLACE6012936 | 0.004379115 | 70979 | 2 |
| PLACE6012942 | 0.00498036 | 3342 | 2 |
| PLACE6013217 | 0.186122649 | 168945 | 15 |
| PLACE6013220 | 0.016584682 | 116953 | 9 |
| PLACE6013222 | 0.034004893 | 179146 | 11 |
| PLACE6013232 | 0.014501369 | 63902 | 2 |
| PLACE6013288 | 0.002981959 | 27684 | 1 |
| PLACE6013386 | 0.002981959 | 43528 | 1 |
| PLACE6013400 | 0.002981959 | 275480 | 1 |
| PLACE6013650 | 0.002981959 | 213359 | 1 |
| PLACE6013672 | 0.006067391 | 85882 | 3 |
| PLACE6013883 | 0.004840074 | 39591 | 2 |
| PLACE6013884 | 0.002981959 | 118409 | 1 |
| PLACE6013885 | 0.002981959 | 115819 | 1 |
| PLACE6014043 | 0.002981959 | 237579 | 1 |
| PLACE6014064 | 0.05474534 | 50978 | 5 |
| PLACE6014516 | 0.002981959 | 198933 | 1 |
| PLACE6014882 | 0.014106667 | 136335 | 2 |
| PLACE6015051 | 0.036347827 | 78385 | 6 |
| PLACE6015211 | 0.002981959 | 190099 | 1 |
| PLACE6015322 | 0.005896816 | 220766 | 2 |
| PLACE6015445 | 0.015077893 | 166201 | 3 |
| PLACE6015460 | 0.002981959 | 191974 | 1 |
| PLACE6015513 | 0.006595851 | 176199 | 2 |
| PLACE6015591 | 0.002981959 | 182279 | 1 |
| PLACE6015729 | 0.004670236 | 220756 | 2 |
| PLACE6015731 | 0.010916135 | 90857 | 3 |
| PLACE6015939 | 0.002981959 | 245924 | 1 |
| PLACE6016030 | 0.002981959 | 121465 | 1 |
| PLACE6016093 | 0.002981959 | 143645 | 1 |
| PLACE6016105 | 0.002981959 | 148330 | 1 |
| PLACE6016160 | 0.002981959 | 232923 | 1 |
| PLACE6016210 | 0.013231277 | 198754 | 3 |
| PLACE6016254 | 0.009170813 | 106930 | 4 |
| PLACE6016383 | 0.025917958 | 150264 | 5 |
| PLACE6016942 | 0.042831316 | 105440 | 10 |
| PLACE6016984 | 0.009070534 | 148094 | 4 |
| PLACE6017002 | 0.002981959 | 209451 | 1 |
| PLACE6017431 | 0.00498036 | 147771 | 2 |
| PLACE6017564 | 0.002981959 | 156714 | 1 |
| PLACE6017578 | 0.016566124 | 73676 | 3 |
| PLACE6017626 | 0.011805381 | 37993 | 3 |
| PLACE6017701 | 0.006826043 | 156072 | 2 |
| PLACE6017714 | 0.002981959 | 176249 | 1 |
| PLACE6017788 | 0.004077237 | 188015 | 2 |
| PLACE6017791 | 0.015255418 | 79365 | 3 |
| PLACE6018031 | 0.024920637 | 130792 | 7 |
| PLACE6018107 | 0.002981959 | 206119 | 1 |
| PLACE6018187 | 0.011927837 | 162369 | 4 |
| PLACE6018235 | 0.002981959 | 105345 | 1 |
| PLACE6018287 | 0.007059197 | 202180 | 3 |
| PLACE6018441 | 0.002981959 | 202821 | 1 |
| PLACE6018487 | 0.251080958 | 102855 | 6 |
| PLACE6018497 | 0.002981959 | 185011 | 1 |
| PLACE6018769 | 0.002981959 | 225171 | 1 |
| PLACE6018834 | 0.002981959 | 233027 | 1 |
| PLACE6018843 | 0.002981959 | 237222 | 1 |
| PLACE6018863 | 0.008823422 | 144823 | 2 |
| PLACE6018938 | 0.021839764 | 83044 | 8 |
| PLACE6019195 | 0.019746648 | 85025 | 8 |
| PLACE6019385 | 0.041308487 | 172131 | 3 |
| PLACE6019542 | 0.002981959 | 232568 | 1 |
| PLACE6019600 | 0.002981959 | 80534 | 1 |
| PLACE6019674 | 0.002981959 | 186850 | 1 |
| PLACE6019676 | 0.002981959 | 58232 | 1 |
| PLACE6019701 | 0.002981959 | 227715 | 1 |
| PLACE6019932 | 0.038632583 | 275162 | 2 |
| PLACE6020031 | 0.008945877 | 182352 | 3 |
| PLACE6020145 | 0.019965318 | 225193 | 3 |
| PLACE7000167 | 0.002981959 | 262219 | 1 |
| PLACE7000170 | 0.002981959 | 253467 | 1 |
| PLACE7000266 | 0.008945877 | 97952 | 3 |
| PLACE7000333 | 0.002981959 | 173394 | 1 |
| PLACE7000410 | 0.004840074 | 117459 | 2 |
| PLACE7000502 | 0.002981959 | 277703 | 1 |
| PLACE7000514 | 0.035226902 | 144430 | 6 |
| PLACE7000707 | 0.002981959 | 269925 | 1 |
| PLACE7001022 | 0.005954079 | 211334 | 3 |
| PLACE7001544 | 0.002981959 | 205948 | 1 |
| PLACE7001759 | 0.002981959 | 243868 | 1 |
| PLACE7001936 | 0.002981959 | 41454 | 1 |
| PLACE7002303 | 0.002981959 | 274816 | 1 |
| PLACE7002641 | 0.002981959 | 249853 | 1 |
| PLACE7003639 | 0.002981959 | 193910 | 1 |
| PLACE7003657 | 0.002981959 | 283762 | 1 |
| PLACE7003684 | 0.002981959 | 196750 | 1 |
| PLACE7003985 | 0.002981959 | 267452 | 1 |
| PLACE7004103 | 0.129563018 | 20704 | 50 |
| PLACE7004961 | 0.002981959 | 147425 | 1 |
| PLACE7005169 | 0.002981959 | 194095 | 1 |
| PLACE7005671 | 0.002981959 | 269594 | 1 |
| PLACE7005840 | 0.004379115 | 111366 | 2 |
| PLACE7006051 | 0.002981959 | 253074 | 1 |
| PLACE7006090 | 0.002981959 | 247503 | 1 |
| PLACE7006240 | 0.006655907 | 169060 | 3 |
| PLACE7006268 | 0.008924083 | 147886 | 2 |
| PLACE7006275 | 0.002981959 | 234796 | 1 |
| PLACE7006498 | 0.002981959 | 94118 | 1 |
| PLACE7006540 | 0.002981959 | 198954 | 1 |
| PLACE7007379 | 0.002981959 | 121060 | 1 |
| PLACE7007644 | 0.002981959 | 256302 | 1 |
| PLACE7007973 | 0.008945877 | 186973 | 3 |
| PLACE7008136 | 0.002981959 | 211294 | 1 |
| PLACE7008261 | 0.005880664 | 123305 | 3 |
| PLACE7008431 | 0.002981959 | 90795 | 1 |
| PLACE7008623 | 0.002981959 | 191577 | 1 |
| PLACE7008766 | 0.002981959 | 208298 | 1 |
| PLACE7009563 | 0.002981959 | 90811 | 1 |
| PROST1000033 | 0.015208456 | 68409 | 3 |
| PROST1000039 | 0.005942124 | 37168 | 1 |
| PROST1000065 | 0.005942124 | 54612 | 1 |
| PROST1000083 | 0.005942124 | 52758 | 1 |
| PROST1000085 | 0.024220018 | 60154 | 4 |
| PROST1000097 | 0.284962709 | 41964 | 74 |
| PROST1000110 | 0.011884247 | 72420 | 2 |
| PROST1000120 | 0.050810917 | 79126 | 9 |
| PROST1000123 | 0.005942124 | 11422 | 1 |
| PROST1000136 | 0.005942124 | 86583 | 1 |
| PROST1000144 | 0.106872442 | 23853 | 15 |
| PROST1000145 | 0.005942124 | 62838 | 1 |
| PROST1000152 | 0.005942124 | 63034 | 1 |
| PROST1000167 | 0.005942124 | 51368 | 1 |
| PROST1000184 | 0.005942124 | 65567 | 1 |
| PROST1000199 | 0.22932478 | 745 | 33 |
| PROST1000215 | 0.032759511 | 143590 | 10 |
| PROST1000217 | 0.005942124 | 268762 | 1 |
| PROST1000220 | 0.24678982 | 41658 | 31 |
| PROST1000226 | 0.093650062 | 139294 | 23 |
| PROST1000246 | 0.015250292 | 71608 | 4 |
| PROST1000262 | 0.005942124 | 96926 | 1 |
| PROST1000272 | 0.053103795 | 15833 | 5 |
| PROST1000298 | 0.005942124 | 82465 | 1 |
| PROST1000322 | 0.01401869 | 260802 | 3 |
| PROST1000334 | 0.005942124 | 60616 | 1 |
| PROST1000343 | 0.058985342 | 20205 | 9 |
| PROST1000362 | 0.005942124 | 171339 | 1 |
| PROST1000377 | 0.011884247 | 173352 | 2 |
| PROST1000383 | 0.007800238 | 235999 | 2 |
| PROST1000412 | 0.005942124 | 264154 | 1 |
| PROST1000419 | 0.005942124 | 256961 | 1 |
| PROST1000451 | 0.005942124 | 254669 | 1 |
| PROST1000480 | 0.008856981 | 247354 | 2 |
| PROST1000485 | 0.005942124 | 265300 | 1 |
| PROST1000493 | 0.005942124 | 258970 | 1 |
| PROST1000512 | 0.077839882 | 142555 | 8 |
| PROST1000526 | 0.066319138 | 118392 | 17 |
| PROST1000527 | 0.005942124 | 263236 | 1 |
| PROST1000528 | 0.005942124 | 251783 | 1 |
| PROST1000536 | 0.027867756 | 142100 | 5 |
| PROST1000559 | 0.005942124 | 226315 | 1 |
| PROST1000564 | 0.124646322 | 66986 | 21 |
| PROST1000575 | 0.005942124 | 279492 | 1 |
| PROST2000036 | 0.059603321 | 107170 | 4 |
| PROST2000053 | 0.005942124 | 132328 | 1 |
| PROST2000067 | 0.005942124 | 172345 | 1 |
| PROST2000079 | 0.055977201 | 115555 | 5 |
| PROST2000105 | 0.005942124 | 69501 | 1 |
| PROST2000138 | 1.933229642 | 4956 | 246 |
| PROST2000143 | 0.005942124 | 191485 | 1 |
| PROST2000162 | 0.005942124 | 184470 | 1 |
| PROST2000165 | 0.018349418 | 19562 | 5 |
| PROST2000176 | 0.066015708 | 113238 | 4 |
| PROST2000203 | 0.005942124 | 186293 | 1 |
| PROST2000206 | 0.007651584 | 118030 | 2 |
| PROST2000212 | 0.005942124 | 248980 | 1 |
| PROST2000241 | 0.027380781 | 11577 | 3 |
| PROST2000267 | 0.005942124 | 153872 | 1 |
| PROST2000273 | 0.007940525 | 98354 | 2 |
| PROST2000274 | 0.016236363 | 186300 | 5 |
| PROST2000277 | 0.007818965 | 167371 | 2 |
| PROST2000284 | 0.047848309 | 38032 | 14 |
| PROST2000325 | 0.023670143 | 89004 | 4 |
| PROST2000337 | 0.00903762 | 207257 | 2 |
| PROST2000432 | 0.012155233 | 43527 | 2 |
| PROST2000452 | 0.005942124 | 181251 | 1 |
| PROST2000463 | 0.009786208 | 157325 | 2 |
| PROST2000470 | 0.005942124 | 274241 | 1 |
| PROST2000505 | 0.005942124 | 45686 | 1 |
| PROST2000567 | 0.011697798 | 142858 | 5 |
| PROST2000572 | 0.005942124 | 143297 | 1 |
| PROST2000717 | 0.005942124 | 98172 | 1 |
| PROST2000760 | 0.059731529 | 101309 | 7 |
| PROST2000761 | 0.017702679 | 196925 | 2 |
| PROST2000835 | 0.007818965 | 217202 | 2 |
| PROST2000893 | 0.010119059 | 71562 | 2 |
| PROST2000961 | 0.005942124 | 260135 | 1 |
| PROST2001002 | 0.008528524 | 43003 | 3 |
| PROST2001116 | 0.007818965 | 253876 | 2 |
| PROST2001138 | 0.005942124 | 238843 | 1 |
| PROST2001180 | 0.067482781 | 56334 | 23 |
| PROST2001213 | 0.020099465 | 108990 | 4 |
| PROST2001283 | 0.078746926 | 165282 | 7 |
| PROST2001414 | 0.005942124 | 262600 | 1 |
| PROST2001415 | 0.005942124 | 267672 | 1 |
| PROST2001465 | 0.005942124 | 277146 | 1 |
| PROST2001521 | 0.137902849 | 56944 | 6 |
| PROST2001540 | 0.005942124 | 250616 | 1 |
| PROST2001552 | 0.19248918 | 150521 | 19 |
| PROST2001599 | 0.005942124 | 260119 | 1 |
| PROST2001611 | 0.005942124 | 255954 | 1 |
| PROST2001636 | 0.005942124 | 250617 | 1 |
| PROST2001676 | 0.277824579 | 74045 | 42 |
| PROST2001739 | 0.050689687 | 168573 | 6 |
| PROST2001796 | 0.005942124 | 67214 | 1 |
| PROST2001805 | 0.005942124 | 250560 | 1 |
| PROST2001823 | 0.00903762 | 183777 | 2 |
| PROST2001883 | 0.187602336 | 106283 | 7 |
| PROST2001899 | 0.005942124 | 195292 | 1 |
| PROST2001997 | 0.028739455 | 171146 | 9 |
| PROST2001998 | 0.005942124 | 260115 | 1 |
| PROST2002078 | 0.068946598 | 119990 | 4 |
| PROST2002101 | 0.005942124 | 19202 | 1 |
| PROST2002162 | 0.043281024 | 68508 | 3 |
| PROST2002212 | 0.005942124 | 51158 | 1 |
| PROST2002227 | 0.065918687 | 2930 | 3 |
| PROST2002338 | 0.005942124 | 162306 | 1 |
| PROST2002425 | 0.010545257 | 67345 | 3 |
| PROST2002488 | 0.005942124 | 4455 | 1 |
| PROST2002489 | 0.005942124 | 113822 | 1 |
| PROST2002527 | 0.005942124 | 258299 | 1 |
| PROST2002591 | 0.005942124 | 192943 | 1 |
| PROST2002602 | 0.005942124 | 265032 | 1 |
| PROST2002633 | 0.005942124 | 285163 | 1 |
| PROST2002651 | 0.056030105 | 153652 | 12 |
| PROST2002682 | 0.005942124 | 45850 | 1 |
| PROST2002685 | 0.005942124 | 256774 | 1 |
| PROST2002722 | 0.005942124 | 264712 | 1 |
| PROST2002736 | 0.005942124 | 273201 | 1 |
| PROST2002842 | 0.005942124 | 249187 | 1 |
| PROST2002897 | 0.007818965 | 185074 | 2 |
| PROST2002906 | 0.005942124 | 263237 | 1 |
| PROST2002927 | 0.015650862 | 119679 | 2 |
| PROST2002960 | 0.012465673 | 137655 | 4 |
| PROST2003102 | 0.005942124 | 260933 | 1 |
| PROST2003117 | 0.005942124 | 108224 | 1 |
| PROST2003210 | 0.005942124 | 112569 | 1 |
| PROST2003232 | 0.245575329 | 93213 | 41 |
| PROST2003302 | 0.005942124 | 255108 | 1 |
| PROST2003303 | 0.005942124 | 259127 | 1 |
| PROST2003324 | 0.009475785 | 159922 | 3 |
| PROST2003338 | 0.149695946 | 94357 | 35 |
| PROST2003340 | 0.005942124 | 18611 | 1 |
| PROST2003396 | 0.02804639 | 55143 | 12 |
| PROST2003428 | 0.008856981 | 152383 | 2 |
| PROST2003472 | 0.005942124 | 62749 | 1 |
| PROST2003517 | 0.063645034 | 95698 | 12 |
| PROST2003537 | 0.007818965 | 158815 | 2 |
| PROST2003563 | 0.005942124 | 238922 | 1 |
| PROST2003577 | 0.005942124 | 166410 | 1 |
| PROST2003583 | 0.005942124 | 91382 | 1 |
| PROST2003586 | 0.005942124 | 166577 | 1 |
| PROST2003628 | 0.005942124 | 254951 | 1 |
| PROST2003635 | 0.005942124 | 224705 | 1 |
| PROST2003674 | 0.005942124 | 146045 | 1 |
| PROST2003676 | 0.005942124 | 178577 | 1 |
| PROST2003725 | 0.007339279 | 110404 | 2 |
| PROST2003732 | 0.005942124 | 152808 | 1 |
| PROST2003775 | 0.005942124 | 54011 | 1 |
| PROST2003922 | 0.005942124 | 285074 | 1 |
| PROST2003930 | 0.005942124 | 284890 | 1 |
| PROST2004095 | 0.357992553 | 42362 | 118 |
| PROST2004115 | 0.005942124 | 71454 | 1 |
| PROST2004142 | 0.005942124 | 118631 | 1 |
| PROST2004146 | 0.005942124 | 157078 | 1 |
| PROST2004161 | 0.005942124 | 159965 | 1 |
| PROST2004251 | 0.187026454 | 228740 | 3 |
| PROST2004258 | 0.043916771 | 115981 | 10 |
| PROST2004270 | 0.284947649 | 53529 | 60 |
| PROST2004332 | 0.005942124 | 33031 | 1 |
| PROST2004416 | 0.246421275 | 60996 | 32 |
| PROST2004481 | 0.005942124 | 264549 | 1 |
| PROST2004526 | 0.005942124 | 75000 | 1 |
| PROST2004570 | 0.022353615 | 72423 | 5 |
| PROST2004727 | 0.005942124 | 135711 | 1 |
| PROST2004739 | 0.022532564 | 98637 | 12 |
| PROST2004742 | 0.005942124 | 137871 | 1 |
| PROST2004744 | 0.031694293 | 231522 | 4 |
| PROST2004817 | 0.005942124 | 35516 | 1 |
| PROST2005039 | 0.012158426 | 151600 | 3 |
| PROST2005067 | 0.010386638 | 96873 | 4 |
| PROST2005076 | 0.023270371 | 175251 | 3 |
| PROST2005093 | 0.007131368 | 133468 | 2 |
| PROST2005121 | 0.005942124 | 149591 | 1 |
| PROST2005131 | 0.005942124 | 7268 | 1 |
| PROST2005143 | 0.02469955 | 112012 | 8 |
| PROST2005228 | 0.005942124 | 158160 | 1 |
| PROST2005272 | 0.020403493 | 124137 | 6 |
| PROST2005285 | 0.022448721 | 104123 | 8 |
| PROST2005426 | 0.005942124 | 162545 | 1 |
| PROST2005466 | 0.007131368 | 183605 | 2 |
| PROST2005604 | 0.053867037 | 125604 | 19 |
| PROST2005630 | 0.005942124 | 264954 | 1 |
| PROST2005683 | 0.01841805 | 146611 | 3 |
| PROST2005788 | 0.005942124 | 257860 | 1 |
| PROST2005793 | 0.042726962 | 88935 | 10 |
| PROST2005880 | 0.005942124 | 247222 | 1 |
| PROST2005886 | 0.005942124 | 169519 | 1 |
| PROST2005904 | 0.005942124 | 258772 | 1 |
| PROST2005919 | 0.005942124 | 67219 | 1 |
| PROST2005943 | 0.005942124 | 247219 | 1 |
| PROST2006008 | 0.005942124 | 95067 | 1 |
| PROST2006020 | 0.005942124 | 264658 | 1 |
| PROST2006030 | 0.044283076 | 18674 | 20 |
| PROST2006060 | 0.025094982 | 90846 | 6 |
| PROST2006196 | 0.005942124 | 34444 | 1 |
| PROST2006201 | 0.005942124 | 263247 | 1 |
| PROST2006260 | 0.041148428 | 98945 | 15 |
| PROST2006282 | 0.018862625 | 147172 | 4 |
| PROST2006343 | 0.03789671 | 152612 | 5 |
| PROST2006450 | 0.005942124 | 253513 | 1 |
| PROST2006491 | 0.005942124 | 144216 | 1 |
| PROST2006510 | 0.005942124 | 35391 | 1 |
| PROST2006536 | 0.075507067 | 17279 | 19 |
| PROST2006579 | 0.005942124 | 58330 | 1 |
| PROST2006688 | 0.005942124 | 281706 | 1 |
| PROST2006737 | 0.069132358 | 95107 | 13 |
| PROST2006766 | 0.005942124 | 74271 | 1 |
| PROST2006782 | 0.005942124 | 257846 | 1 |
| PROST2006933 | 0.008856981 | 46348 | 2 |
| PROST2006988 | 0.011474484 | 178694 | 3 |
| PROST2007122 | 0.005942124 | 256014 | 1 |
| PROST2007200 | 0.014185061 | 177898 | 5 |
| PROST2007237 | 0.005942124 | 103378 | 1 |
| PROST2007248 | 0.008989483 | 166582 | 3 |
| PROST2007289 | 0.005942124 | 81524 | 1 |
| PROST2007317 | 0.007037402 | 117924 | 2 |
| PROST2007328 | 0.005942124 | 235208 | 1 |
| PROST2007382 | 0.005942124 | 269592 | 1 |
| PROST2007389 | 0.011884247 | 99275 | 2 |
| PROST2007416 | 0.011884247 | 173386 | 2 |
| PROST2007444 | 0.153231919 | 112210 | 57 |
| PROST2007528 | 0.005942124 | 162203 | 1 |
| PROST2007612 | 0.052470125 | 127075 | 12 |
| PROST2007818 | 0.005942124 | 241787 | 1 |
| PROST2007871 | 0.015191297 | 132816 | 4 |
| PROST2007950 | 0.005942124 | 143659 | 1 |
| PROST2007956 | 0.005942124 | 168227 | 1 |
| PROST2007974 | 0.005942124 | 158080 | 1 |
| PROST2007988 | 0.005942124 | 264140 | 1 |
| PROST2008079 | 0.005942124 | 13312 | 1 |
| PROST2008088 | 0.005942124 | 252945 | 1 |
| PROST2008243 | 0.005942124 | 131356 | 1 |
| PROST2008245 | 0.005942124 | 154052 | 1 |
| PROST2008268 | 0.00903762 | 170782 | 2 |
| PROST2008271 | 0.042754388 | 125879 | 19 |
| PROST2008360 | 0.093096573 | 120267 | 15 |
| PROST2008447 | 0.005942124 | 126269 | 1 |
| PROST2008468 | 0.005942124 | 250007 | 1 |
| PROST2008472 | 0.005942124 | 156943 | 1 |
| PROST2008489 | 0.258721502 | 59756 | 24 |
| PROST2008516 | 0.005942124 | 235806 | 1 |
| PROST2008724 | 0.005942124 | 236780 | 1 |
| PROST2008770 | 0.214802958 | 50355 | 66 |
| PROST2008835 | 0.005942124 | 68965 | 1 |
| PROST2008993 | 0.005942124 | 7528 | 1 |
| PROST2009022 | 0.005942124 | 264164 | 1 |
| PROST2009208 | 0.005942124 | 252939 | 1 |
| PROST2009222 | 0.005942124 | 256347 | 1 |
| PROST2009254 | 0.005942124 | 260282 | 1 |
| PROST2009273 | 0.005942124 | 62071 | 1 |
| PROST2009320 | 0.011854735 | 176817 | 2 |
| PROST2009347 | 0.008840828 | 167130 | 3 |
| PROST2009365 | 0.005942124 | 244082 | 1 |
| PROST2009388 | 0.005942124 | 113104 | 1 |
| PROST2009400 | 0.014050476 | 160196 | 4 |
| PROST2009470 | 0.005942124 | 254806 | 1 |
| PROST2009483 | 0.011884247 | 167995 | 2 |
| PROST2009571 | 0.005942124 | 262051 | 1 |
| PROST2009731 | 0.154504008 | 197671 | 3 |
| PROST2009736 | 0.005942124 | 36655 | 1 |
| PROST2009795 | 0.005942124 | 125611 | 1 |
| PROST2009901 | 0.005942124 | 164995 | 1 |
| PROST2009909 | 0.005942124 | 122661 | 1 |
| PROST2010046 | 0.029710619 | 142556 | 5 |
| PROST2010219 | 0.007818965 | 134050 | 2 |
| PROST2010250 | 0.005942124 | 173339 | 1 |
| PROST2010318 | 0.013882649 | 73588 | 3 |
| PROST2010326 | 0.005942124 | 93 | 1 |
| PROST2010382 | 0.053544083 | 181787 | 3 |
| PROST2010400 | 0.005942124 | 273838 | 1 |
| PROST2010545 | 0.012732224 | 201978 | 4 |
| PROST2010579 | 0.008914244 | 159160 | 3 |
| PROST2010606 | 0.005942124 | 17150 | 1 |
| PROST2010782 | 0.68901586 | 36006 | 21 |
| PROST2010885 | 0.005942124 | 116310 | 1 |
| PROST2011012 | 0.005942124 | 219780 | 1 |
| PROST2011038 | 0.013963143 | 69569 | 3 |
| PROST2011105 | 0.009415891 | 51105 | 4 |
| PROST2011297 | 0.005942124 | 110276 | 1 |
| PROST2011410 | 0.042256439 | 184207 | 3 |
| PROST2011439 | 0.005942124 | 196633 | 1 |
| PROST2011482 | 0.00903762 | 127791 | 2 |
| PROST2011577 | 0.162822998 | 105745 | 26 |
| PROST2011631 | 0.005942124 | 53421 | 1 |
| PROST2011660 | 0.007131368 | 219874 | 2 |
| PROST2012005 | 0.005942124 | 275100 | 1 |
| PROST2012007 | 0.147631125 | 114056 | 18 |
| PROST2012016 | 0.0076304 | 207999 | 2 |
| PROST2012088 | 0.005942124 | 125946 | 1 |
| PROST2012140 | 0.00906293 | 41975 | 2 |
| PROST2012157 | 0.005942124 | 148596 | 1 |
| PROST2012190 | 0.005942124 | 139665 | 1 |
| PROST2012249 | 0.005942124 | 259763 | 1 |
| PROST2012353 | 0.017825152 | 161319 | 3 |
| PROST2012400 | 0.005942124 | 210230 | 1 |
| PROST2012448 | 0.020536025 | 134964 | 9 |
| PROST2012504 | 0.007339279 | 15446 | 2 |
| PROST2012542 | 0.005942124 | 156960 | 1 |
| PROST2012550 | 0.005942124 | 70083 | 1 |
| PROST2012740 | 0.005942124 | 193803 | 1 |
| PROST2012745 | 0.043325874 | 9478 | 8 |
| PROST2012780 | 0.023032916 | 127933 | 3 |
| PROST2012888 | 0.009980407 | 176077 | 2 |
| PROST2012890 | 0.010985407 | 156961 | 3 |
| PROST2013032 | 0.005942124 | 131493 | 1 |
| PROST2013053 | 0.056571608 | 133025 | 4 |
| PROST2013121 | 0.015577881 | 11399 | 7 |
| PROST2013260 | 0.005942124 | 152122 | 1 |
| PROST2013327 | 0.005942124 | 89745 | 1 |
| PROST2013531 | 0.005942124 | 210251 | 1 |
| PROST2013605 | 0.005942124 | 242998 | 1 |
| PROST2013627 | 0.005942124 | 224677 | 1 |
| PROST2013873 | 0.005942124 | 191711 | 1 |
| PROST2013880 | 0.005942124 | 193328 | 1 |
| PROST2014422 | 0.005942124 | 159676 | 1 |
| PROST2014585 | 0.005942124 | 158165 | 1 |
| PROST2014601 | 0.005942124 | 133030 | 1 |
| PROST2014659 | 0.005942124 | 279819 | 1 |
| PROST2014862 | 0.010545257 | 83129 | 3 |
| PROST2014916 | 0.045992275 | 101313 | 3 |
| PROST2014925 | 0.011884247 | 276617 | 2 |
| PROST2015124 | 0.005942124 | 127654 | 1 |
| PROST2015137 | 0.009118914 | 19008 | 3 |
| PROST2015198 | 0.005942124 | 264123 | 1 |
| PROST2015243 | 0.005942124 | 278418 | 1 |
| PROST2015246 | 0.005942124 | 186494 | 1 |
| PROST2015251 | 0.007800238 | 74934 | 2 |
| PROST2015287 | 0.015608069 | 149479 | 4 |
| PROST2015332 | 0.065006855 | 116140 | 28 |
| PROST2015457 | 0.005942124 | 175300 | 1 |
| PROST2015464 | 0.097690609 | 124013 | 14 |
| PROST2015537 | 0.005942124 | 137696 | 1 |
| PROST2015545 | 0.005942124 | 191689 | 1 |
| PROST2015551 | 0.005942124 | 39570 | 1 |
| PROST2015636 | 0.005942124 | 196118 | 1 |
| PROST2015710 | 0.013605416 | 187293 | 3 |
| PROST2015756 | 0.005942124 | 156939 | 1 |
| PROST2015877 | 0.005942124 | 106718 | 1 |
| PROST2015900 | 0.007131368 | 162244 | 2 |
| PROST2015924 | 0.02472645 | 101372 | 6 |
| PROST2015932 | 0.005942124 | 237770 | 1 |
| PROST2016195 | 0.02865235 | 93703 | 4 |
| PROST2016246 | 0.005942124 | 256552 | 1 |
| PROST2016351 | 0.018917082 | 189305 | 3 |
| PROST2016379 | 0.005942124 | 223925 | 1 |
| PROST2016444 | 0.005942124 | 126609 | 1 |
| PROST2016462 | 0.005942124 | 153073 | 1 |
| PROST2016499 | 0.005942124 | 169652 | 1 |
| PROST2016512 | 0.005942124 | 90506 | 1 |
| PROST2016566 | 0.005942124 | 223036 | 1 |
| PROST2016582 | 0.017228805 | 59588 | 2 |
| PROST2016668 | 0.005942124 | 259973 | 1 |
| PROST2016684 | 0.005942124 | 130861 | 1 |
| PROST2016777 | 0.005942124 | 116474 | 1 |
| PROST2016829 | 0.005942124 | 188525 | 1 |
| PROST2016860 | 0.017826371 | 146566 | 3 |
| PROST2016918 | 0.026365287 | 109376 | 10 |
| PROST2016980 | 0.014799104 | 181108 | 3 |
| PROST2017098 | 0.005942124 | 188502 | 1 |
| PROST2017128 | 0.020793924 | 143705 | 4 |
| PROST2017203 | 0.129028512 | 75725 | 22 |
| PROST2017367 | 0.005942124 | 39562 | 1 |
| PROST2017413 | 0.005942124 | 196123 | 1 |
| PROST2017441 | 0.005942124 | 280815 | 1 |
| PROST2017529 | 0.066013596 | 73429 | 6 |
| PROST2017578 | 0.005942124 | 212267 | 1 |
| PROST2017612 | 0.007818965 | 30377 | 2 |
| PROST2017617 | 0.007940525 | 142022 | 2 |
| PROST2017692 | 0.227378107 | 56115 | 21 |
| PROST2017700 | 0.005942124 | 153278 | 1 |
| PROST2017701 | 0.005942124 | 146704 | 1 |
| PROST2017729 | 0.011854735 | 171611 | 2 |
| PROST2017836 | 0.005942124 | 18958 | 1 |
| PROST2017910 | 0.016034775 | 177825 | 3 |
| PROST2017972 | 0.013854029 | 151310 | 3 |
| PROST2018030 | 0.005942124 | 177929 | 1 |
| PROST2018090 | 0.077024597 | 77825 | 3 |
| PROST2018235 | 0.005942124 | 159943 | 1 |
| PROST2018437 | 0.005942124 | 284976 | 1 |
| PROST2018487 | 0.005942124 | 173469 | 1 |
| PROST2018511 | 0.005942124 | 69532 | 1 |
| PROST2018583 | 0.018129216 | 135109 | 3 |
| PROST2018588 | 0.012768167 | 64920 | 3 |
| PROST2018607 | 0.009980407 | 19012 | 2 |
| PROST2018788 | 0.005942124 | 233118 | 1 |
| PROST2018902 | 0.005942124 | 197268 | 1 |
| PROST2018922 | 0.005942124 | 144730 | 1 |
| PROST2018977 | 0.012894135 | 127478 | 4 |
| PROST2019164 | 0.045440065 | 112887 | 10 |
| PROST2019253 | 0.099728261 | 11949 | 32 |
| PROST2019296 | 0.029463234 | 179098 | 3 |
| PROST2019398 | 0.005942124 | 253328 | 1 |
| PROST2019487 | 0.005942124 | 220162 | 1 |
| PROST2019781 | 0.005942124 | 255810 | 1 |
| PUAEN1000039 | 0.009454477 | 67423 | 1 |
| PUAEN1000057 | 0.08161272 | 74496 | 24 |
| PUAEN1000065 | 0.250168757 | 9442 | 42 |
| PUAEN1000081 | 0.012575283 | 53305 | 2 |
| PUAEN1000085 | 0.009454477 | 128647 | 1 |
| PUAEN1000087 | 0.009454477 | 73847 | 1 |
| PUAEN1000121 | 0.009454477 | 21686 | 1 |
| PUAEN1000147 | 0.017969139 | 69830 | 6 |
| PUAEN1000161 | 0.02904803 | 43250 | 9 |
| PUAEN1000164 | 0.420960301 | 72471 | 40 |
| PUAEN1000175 | 0.009454477 | 43752 | 1 |
| PUAEN1000239 | 0.05404878 | 78338 | 14 |
| PUAEN1000275 | 0.009454477 | 281377 | 1 |
| PUAEN1000276 | 0.009454477 | 141955 | 1 |
| PUAEN1000288 | 0.02836343 | 36912 | 3 |
| PUAEN1000322 | 0.131066861 | 14558 | 13 |
| PUAEN2000080 | 0.009454477 | 141142 | 1 |
| PUAEN2000152 | 0.028232203 | 82523 | 8 |
| PUAEN2000175 | 0.04747729 | 103106 | 2 |
| PUAEN2000247 | 0.030327468 | 134137 | 8 |
| PUAEN2000312 | 0.018926049 | 92173 | 5 |
| PUAEN2000374 | 0.523525708 | 65148 | 65 |
| PUAEN2000394 | 0.009454477 | 120625 | 1 |
| PUAEN2000497 | 0.06499439 | 165430 | 5 |
| PUAEN2000535 | 0.208341386 | 87553 | 16 |
| PUAEN2000594 | 0.015667586 | 218383 | 2 |
| PUAEN2000684 | 0.018908953 | 87618 | 2 |
| PUAEN2000942 | 0.012549973 | 96188 | 2 |
| PUAEN2001188 | 0.048276743 | 117193 | 33 |
| PUAEN2001260 | 0.009454477 | 136511 | 1 |
| PUAEN2001345 | 0.057226273 | 103555 | 3 |
| PUAEN2001526 | 0.009454477 | 154880 | 1 |
| PUAEN2001586 | 0.058678854 | 67880 | 9 |
| PUAEN2001882 | 0.015259655 | 61560 | 2 |
| PUAEN2002473 | 0.009454477 | 223222 | 1 |
| PUAEN2002489 | 0.025679813 | 137028 | 9 |
| PUAEN2002552 | 0.009454477 | 245027 | 1 |
| PUAEN2002568 | 0.015367088 | 130005 | 2 |
| PUAEN2002616 | 0.009454477 | 204723 | 1 |
| PUAEN2002758 | 0.009454477 | 225331 | 1 |
| PUAEN2003018 | 0.04998634 | 130876 | 9 |
| PUAEN2003079 | 0.009454477 | 278188 | 1 |
| PUAEN2003116 | 0.009454477 | 172474 | 1 |
| PUAEN2003408 | 0.055636312 | 33679 | 8 |
| PUAEN2003517 | 0.009454477 | 135212 | 1 |
| PUAEN2003947 | 0.083379247 | 115298 | 15 |
| PUAEN2003954 | 0.015366739 | 169759 | 2 |
| PUAEN2004067 | 0.009454477 | 90043 | 1 |
| PUAEN2004083 | 0.009454477 | 112361 | 1 |
| PUAEN2004400 | 0.023257335 | 177058 | 4 |
| PUAEN2004511 | 0.037817907 | 175814 | 4 |
| PUAEN2004525 | 0.009454477 | 175807 | 1 |
| PUAEN2004875 | 0.009454477 | 88895 | 1 |
| PUAEN2005110 | 0.009454477 | 196908 | 1 |
| PUAEN2005247 | 0.009454477 | 133570 | 1 |
| PUAEN2005502 | 0.015655168 | 117317 | 5 |
| PUAEN2005588 | 0.009454477 | 105107 | 1 |
| PUAEN2005930 | 0.018249987 | 35647 | 6 |
| PUAEN2006029 | 0.009454477 | 109817 | 1 |
| PUAEN2006328 | 0.045438644 | 188717 | 2 |
| PUAEN2006335 | 0.013211536 | 28164 | 3 |
| PUAEN2006578 | 0.009454477 | 81690 | 1 |
| PUAEN2006639 | 0.009454477 | 214089 | 1 |
| PUAEN2006701 | 0.074487441 | 46150 | 11 |
| PUAEN2006723 | 0.117118444 | 51065 | 21 |
| PUAEN2006761 | 0.009454477 | 163250 | 1 |
| PUAEN2007044 | 0.116897286 | 113030 | 46 |
| PUAEN2007350 | 0.012499358 | 191416 | 2 |
| PUAEN2007785 | 0.009454477 | 107280 | 1 |
| PUAEN2007898 | 0.015508845 | 220203 | 2 |
| PUAEN2008123 | 0.02836343 | 153908 | 3 |
| PUAEN2008228 | 0.011452878 | 8315 | 2 |
| PUAEN2008314 | 0.015667586 | 51346 | 2 |
| PUAEN2008515 | 0.017258655 | 126473 | 6 |
| PUAEN2008815 | 0.01349276 | 164414 | 2 |
| PUAEN2008939 | 0.048822234 | 33700 | 7 |
| PUAEN2008980 | 0.009454477 | 65222 | 1 |
| PUAEN2008985 | 0.037762988 | 130790 | 13 |
| PUAEN2009174 | 0.05911391 | 158723 | 5 |
| PUAEN2009348 | 0.021217799 | 181211 | 2 |
| PUAEN2009534 | 0.009454477 | 101464 | 1 |
| PUAEN2009655 | 0.045843298 | 86624 | 10 |
| PUAEN2009795 | 0.009454477 | 116244 | 1 |
| PUAEN2009852 | 0.009454477 | 189853 | 1 |
| PUAEN2010346 | 0.016787407 | 30852 | 3 |
| PUAEN2010824 | 0.031983749 | 133594 | 15 |
| RECTM1000051 | 0.072595657 | 183914 | 4 |
| RECTM1000141 | 0.036456435 | 99349 | 1 |
| RECTM2000220 | 0.036456435 | 263986 | 1 |
| RECTM2000349 | 0.039551931 | 165918 | 2 |
| RECTM2000433 | 0.096439513 | 206849 | 4 |
| RECTM2000510 | 0.055590468 | 191599 | 3 |
| RECTM2001307 | 0.036456435 | 158680 | 1 |
| RECTM2001347 | 0.040396061 | 231729 | 3 |
| RECTM2001519 | 0.042398558 | 136175 | 2 |
| RECTM2001666 | 0.036456435 | 231640 | 1 |
| RECTM2001691 | 0.036456435 | 249519 | 1 |
| RECTM2001749 | 0.036456435 | 270399 | 1 |
| RECTM2002172 | 0.042658754 | 174941 | 2 |
| RECTM2002602 | 0.036456435 | 229358 | 1 |
| RECTM2002632 | 0.036456435 | 278074 | 1 |
| SALGL1000005 | 0.55252423 | 56519 | 3 |
| SALGL1000024 | 0.551367738 | 30407 | 2 |
| SALGL1000028 | 0.540540541 | 34449 | 1 |
| SALGL1000047 | 0.565613342 | 22067 | 13 |
| SALGL1000065 | 0.540540541 | 60866 | 1 |
| SALGL1000107 | 0.542731097 | 22721 | 3 |
| SALGL1000157 | 1.288535926 | 23950 | 76 |
| SALGL1000171 | 0.543334851 | 69545 | 3 |
| SKMUS1000014 | 0.022777922 | 44359 | 5 |
| SKMUS1000022 | 0.402562528 | 36477 | 33 |
| SKMUS1000030 | 0.011806375 | 51973 | 1 |
| SKMUS1000064 | 0.357021123 | 51790 | 32 |
| SKMUS1000067 | 0.011806375 | 61805 | 1 |
| SKMUS1000084 | 0.108797944 | 1627 | 27 |
| SKMUS1000102 | 0.017326006 | 72224 | 3 |
| SKMUS1000104 | 0.023612751 | 100625 | 2 |
| SKMUS1000110 | 0.022744331 | 62767 | 5 |
| SKMUS1000118 | 0.011806375 | 90271 | 1 |
| SKMUS1000124 | 0.011806375 | 36433 | 1 |
| SKMUS1000129 | 0.118063754 | 28908 | 10 |
| SKMUS1000138 | 1.744784769 | 78371 | 77 |
| SKMUS1000176 | 0.194213688 | 79808 | 4 |
| SKMUS1000177 | 0.070954434 | 27950 | 24 |
| SKMUS1000186 | 0.4174627 | 42726 | 50 |
| SKMUS2000020 | 0.045492571 | 174802 | 6 |
| SKMUS2000061 | 0.011806375 | 169612 | 1 |
| SKMUS2000074 | 0.032762615 | 94474 | 4 |
| SKMUS2000078 | 0.011806375 | 34531 | 1 |
| SKMUS2000117 | 0.019718858 | 184594 | 3 |
| SKMUS2000137 | 0.011806375 | 238540 | 1 |
| SKMUS2000198 | 0.106085694 | 153834 | 9 |
| SKMUS2000243 | 0.016609512 | 121970 | 3 |
| SKMUS2000271 | 0.026267996 | 170233 | 5 |
| SKMUS2000287 | 0.011806375 | 204543 | 1 |
| SKMUS2000317 | 0.011806375 | 106346 | 1 |
| SKMUS2000330 | 0.011806375 | 257597 | 1 |
| SKMUS2000339 | 0.011806375 | 274903 | 1 |
| SKMUS2000343 | 0.021746785 | 86654 | 7 |
| SKMUS2000361 | 0.156686231 | 118406 | 15 |
| SKMUS2000365 | 0.011806375 | 77154 | 1 |
| SKMUS2000380 | 0.077265157 | 56871 | 11 |
| SKMUS2000390 | 0.023612751 | 37149 | 2 |
| SKMUS2000416 | 0.011806375 | 147703 | 1 |
| SKMUS2000458 | 0.175319547 | 153833 | 15 |
| SKMUS2000467 | 0.014851257 | 175235 | 2 |
| SKMUS2000468 | 0.011806375 | 239334 | 1 |
| SKMUS2000484 | 0.095419817 | 152457 | 23 |
| SKMUS2000515 | 0.014788335 | 178432 | 2 |
| SKMUS2000648 | 0.382534307 | 44066 | 23 |
| SKMUS2000679 | 0.250105605 | 159248 | 20 |
| SKMUS2000690 | 0.03440014 | 190628 | 2 |
| SKMUS2000701 | 0.011806375 | 277491 | 1 |
| SKMUS2000705 | 0.011806375 | 266468 | 1 |
| SKMUS2000710 | 0.011806375 | 178160 | 1 |
| SKMUS2000724 | 0.011806375 | 119105 | 1 |
| SKMUS2000726 | 0.011806375 | 13136 | 1 |
| SKMUS2000757 | 0.033906374 | 118088 | 5 |
| SKMUS2000774 | 0.059031877 | 58750 | 5 |
| SKMUS2000780 | 0.017611554 | 107990 | 2 |
| SKMUS2000821 | 0.211093475 | 14972 | 16 |
| SKMUS2000847 | 0.011806375 | 276453 | 1 |
| SKMUS2000863 | 0.011806375 | 138727 | 1 |
| SKMUS2000873 | 0.30461113 | 53764 | 33 |
| SKMUS2000894 | 0.011806375 | 146381 | 1 |
| SKMUS2000898 | 0.011806375 | 173418 | 1 |
| SKMUS2000902 | 0.030979939 | 81845 | 6 |
| SKMUS2000931 | 0.307921717 | 4828 | 60 |
| SKMUS2000933 | 0.027005847 | 159968 | 4 |
| SKMUS2000945 | 0.023612751 | 234220 | 2 |
| SKMUS2000954 | 0.098584961 | 101802 | 13 |
| SKMUS2000961 | 0.025451383 | 91799 | 3 |
| SKMUS2000973 | 0.011806375 | 270204 | 1 |
| SKMUS2000980 | 0.011806375 | 275623 | 1 |
| SKMUS2001008 | 0.353604424 | 162422 | 31 |
| SKMUS2001014 | 0.011806375 | 272271 | 1 |
| SKMUS2001129 | 0.03294934 | 55098 | 7 |
| SKMUS2001147 | 0.060445442 | 122764 | 12 |
| SKMUS2001157 | 0.046705808 | 109083 | 4 |
| SKMUS2001164 | 0.011806375 | 193506 | 1 |
| SKMUS2001199 | 0.011806375 | 146323 | 1 |
| SKMUS2001201 | 0.094060505 | 178031 | 9 |
| SKMUS2001215 | 0.011806375 | 170024 | 1 |
| SKMUS2001247 | 0.011806375 | 272195 | 1 |
| SKMUS2001254 | 0.013494652 | 103250 | 2 |
| SKMUS2001271 | 0.011806375 | 265617 | 1 |
| SKMUS2001287 | 0.011806375 | 40044 | 1 |
| SKMUS2001323 | 0.095383062 | 54152 | 3 |
| SKMUS2001364 | 0.011806375 | 270303 | 1 |
| SKMUS2001398 | 0.011806375 | 201688 | 1 |
| SKMUS2001402 | 0.039745918 | 168538 | 5 |
| SKMUS2001412 | 0.013494652 | 109555 | 2 |
| SKMUS2001454 | 0.496448704 | 99423 | 80 |
| SKMUS2001492 | 0.011806375 | 131386 | 1 |
| SKMUS2001501 | 0.076873609 | 116061 | 10 |
| SKMUS2001528 | 0.133488985 | 131138 | 15 |
| SKMUS2001543 | 0.015374109 | 148340 | 4 |
| SKMUS2001580 | 0.011806375 | 276418 | 1 |
| SKMUS2001585 | 0.011806375 | 236373 | 1 |
| SKMUS2001608 | 0.011806375 | 110456 | 1 |
| SKMUS2001622 | 0.106085694 | 153834 | 9 |
| SKMUS2001631 | 0.011806375 | 231631 | 1 |
| SKMUS2001634 | 0.127981114 | 118943 | 27 |
| SKMUS2001662 | 0.011806375 | 185076 | 1 |
| SKMUS2001663 | 0.011806375 | 214394 | 1 |
| SKMUS2001668 | 0.339266725 | 173113 | 29 |
| SKMUS2001671 | 0.035419126 | 116067 | 3 |
| SKMUS2001683 | 0.011806375 | 141095 | 1 |
| SKMUS2001693 | 0.034899433 | 34319 | 3 |
| SKMUS2001695 | 0.185783845 | 109082 | 16 |
| SKMUS2001703 | 0.011806375 | 267090 | 1 |
| SKMUS2001740 | 0.011806375 | 40053 | 1 |
| SKMUS2001742 | 0.035419126 | 75473 | 3 |
| SKMUS2001823 | 0.011806375 | 253660 | 1 |
| SKMUS2001850 | 0.011806375 | 90275 | 1 |
| SKMUS2002077 | 0.023612751 | 159251 | 2 |
| SKMUS2002084 | 0.026835902 | 53859 | 4 |
| SKMUS2002153 | 0.011806375 | 49804 | 1 |
| SKMUS2002475 | 0.011806375 | 212934 | 1 |
| SKMUS2002602 | 0.011806375 | 116252 | 1 |
| SKMUS2002634 | 0.097794659 | 146131 | 9 |
| SKMUS2002693 | 0.011806375 | 248602 | 1 |
| SKMUS2002821 | 0.023612751 | 136885 | 2 |
| SKMUS2002840 | 0.011806375 | 212511 | 1 |
| SKMUS2002920 | 0.023612751 | 188014 | 2 |
| SKMUS2003034 | 0.023612751 | 181145 | 2 |
| SKMUS2003074 | 0.011806375 | 13142 | 1 |
| SKMUS2003168 | 0.017748499 | 205356 | 2 |
| SKMUS2003194 | 0.012901654 | 182700 | 2 |
| SKMUS2003744 | 0.011806375 | 240636 | 1 |
| SKMUS2004044 | 0.012901654 | 96617 | 2 |
| SKMUS2004047 | 0.011806375 | 175109 | 1 |
| SKMUS2004483 | 0.023612751 | 226782 | 2 |
| SKMUS2004667 | 0.017748499 | 142699 | 2 |
| SKMUS2004897 | 0.011806375 | 229383 | 1 |
| SKMUS2004903 | 0.023093057 | 109084 | 2 |
| SKMUS2004908 | 0.051011564 | 49066 | 20 |
| SKMUS2005428 | 0.011806375 | 175856 | 1 |
| SKMUS2006394 | 0.034790931 | 171345 | 3 |
| SKMUS2006481 | 0.011806375 | 203829 | 1 |
| SKMUS2006755 | 0.023093057 | 218561 | 2 |
| SKMUS2007315 | 0.011806375 | 166532 | 1 |
| SKMUS2007359 | 0.011806375 | 78060 | 1 |
| SKMUS2007568 | 0.011806375 | 209605 | 1 |
| SKMUS2007740 | 0.023612751 | 159250 | 2 |
| SKMUS2007816 | 0.011806375 | 7590 | 1 |
| SKMUS2007915 | 0.011806375 | 158009 | 1 |
| SKMUS2008338 | 0.011806375 | 167680 | 1 |
| SKMUS2008407 | 0.011806375 | 207212 | 1 |
| SKMUS2008474 | 0.011806375 | 13139 | 1 |
| SKMUS2008585 | 0.023612751 | 214395 | 2 |
| SKMUS2008607 | 0.011806375 | 188005 | 1 |
| SKMUS2009190 | 0.011806375 | 229207 | 1 |
| SKMUS2009232 | 0.011806375 | 187337 | 1 |
| SKMUS2009479 | 0.011806375 | 262641 | 1 |
| SKMUS2009557 | 0.011806375 | 215848 | 1 |
| SKNMC1000110 | 0.046280419 | 57453 | 8 |
| SKNMC1000123 | 0.013061651 | 69231 | 1 |
| SKNMC1000124 | 0.013061651 | 78246 | 1 |
| SKNMC1000137 | 0.158064039 | 54854 | 23 |
| SKNMC1000159 | 0.015866389 | 91566 | 3 |
| SKNMC1000168 | 0.200091079 | 62226 | 23 |
| SKNMC1000229 | 0.031872481 | 40682 | 7 |
| SKNMC1000251 | 0.023888065 | 70807 | 6 |
| SKNMC1000264 | 0.031945921 | 71841 | 5 |
| SKNMC2000065 | 0.155211407 | 81449 | 54 |
| SKNMC2000097 | 0.375933517 | 8504 | 37 |
| SKNMC2000224 | 0.026749288 | 133704 | 4 |
| SKNMC2000256 | 0.013061651 | 124132 | 1 |
| SKNMC2000305 | 0.047569604 | 83573 | 17 |
| SKNMC2000322 | 0.118647954 | 103785 | 14 |
| SKNMC2000356 | 0.018951458 | 146687 | 4 |
| SKNMC2000374 | 0.013061651 | 270474 | 1 |
| SKNMC2000583 | 0.013061651 | 267442 | 1 |
| SKNMC2000593 | 0.019207177 | 148705 | 2 |
| SKNMC2000612 | 0.039184953 | 167270 | 3 |
| SKNMC2000622 | 0.013061651 | 262601 | 1 |
| SKNMC2000635 | 0.934107208 | 77578 | 96 |
| SKNMC2000649 | 0.183657027 | 110797 | 32 |
| SKNMC2000698 | 0.081121106 | 159290 | 16 |
| SKNMC2000877 | 0.141402482 | 105490 | 31 |
| SKNMC2000966 | 0.020692051 | 52399 | 3 |
| SKNMC2001057 | 0.019047249 | 236266 | 4 |
| SKNMC2001113 | 0.017165752 | 32628 | 3 |
| SKNMC2001324 | 0.025947179 | 72405 | 4 |
| SKNMC2001503 | 0.054029779 | 132743 | 9 |
| SKNMC2001555 | 0.013061651 | 71150 | 1 |
| SKNMC2001596 | 0.013061651 | 87870 | 1 |
| SKNMC2001623 | 0.016675543 | 115479 | 2 |
| SKNMC2002402 | 0.022728256 | 105999 | 5 |
| SKNMC2003639 | 0.016182457 | 142789 | 2 |
| SKNMC2003924 | 0.013061651 | 213572 | 1 |
| SKNMC2003987 | 0.013061651 | 283391 | 1 |
| SKNMC2004457 | 0.013061651 | 234096 | 1 |
| SKNMC2004643 | 0.013061651 | 193536 | 1 |
| SKNMC2004651 | 0.013061651 | 225212 | 1 |
| SKNMC2005772 | 0.013061651 | 226517 | 1 |
| SKNMC2006173 | 0.021774346 | 167701 | 5 |
| SKNMC2006327 | 0.013061651 | 257564 | 1 |
| SKNMC2006998 | 0.013061651 | 197841 | 1 |
| SKNMC2007134 | 0.013061651 | 150488 | 1 |
| SKNMC2007502 | 0.013061651 | 175750 | 1 |
| SKNMC2007504 | 0.026123302 | 175749 | 2 |
| SKNMC2007961 | 0.013061651 | 154447 | 1 |
| SKNMC2009450 | 0.013061651 | 141873 | 1 |
| SKNSH1000002 | 0.116570504 | 74749 | 13 |
| SKNSH1000086 | 0.019623028 | 76655 | 4 |
| SKNSH1000101 | 0.260848272 | 63857 | 19 |
| SKNSH1000174 | 0.011504832 | 74273 | 1 |
| SKNSH1000301 | 0.011504832 | 73355 | 1 |
| SKNSH1000308 | 0.011504832 | 62403 | 1 |
| SKNSH1000416 | 0.011504832 | 67019 | 1 |
| SKNSH2000051 | 0.01983088 | 115662 | 5 |
| SKNSH2000101 | 0.025426757 | 80974 | 9 |
| SKNSH2000151 | 0.03079909 | 115194 | 9 |
| SKNSH2000163 | 0.015789573 | 145677 | 3 |
| SKNSH2000245 | 0.176626238 | 108761 | 31 |
| SKNSH2000250 | 0.118930558 | 75476 | 49 |
| SKNSH2000319 | 0.023364378 | 164426 | 3 |
| SKNSH2000347 | 0.074676678 | 125457 | 22 |
| SKNSH2000482 | 0.017189911 | 143153 | 4 |
| SKNSH2000499 | 0.045952233 | 160238 | 11 |
| SKNSH2000516 | 0.011504832 | 204000 | 1 |
| SKNSH2000550 | 0.011504832 | 259620 | 1 |
| SKNSH2000716 | 0.096677532 | 149478 | 13 |
| SKNSH2000819 | 0.067319187 | 124339 | 23 |
| SKNSH2000971 | 0.02330285 | 234478 | 2 |
| SKNSH2001222 | 0.025659598 | 158951 | 4 |
| SKNSH2001875 | 0.025803974 | 130530 | 4 |
| SKNSH2001931 | 0.032787618 | 68013 | 5 |
| SKNSH2002054 | 0.011504832 | 225472 | 1 |
| SKNSH2002325 | 0.043891577 | 127118 | 10 |
| SKNSH2002768 | 0.070767872 | 61033 | 14 |
| SKNSH2002866 | 0.011504832 | 274207 | 1 |
| SKNSH2003064 | 0.021403284 | 152142 | 3 |
| SKNSH2003174 | 0.011504832 | 160137 | 1 |
| SKNSH2003422 | 0.011504832 | 198245 | 1 |
| SKNSH2003466 | 0.011504832 | 240054 | 1 |
| SKNSH2003483 | 0.011504832 | 236918 | 1 |
| SKNSH2003528 | 0.055194048 | 131238 | 8 |
| SKNSH2003633 | 0.024980002 | 102593 | 3 |
| SKNSH2004039 | 0.011504832 | 96243 | 1 |
| SKNSH2005061 | 0.098840742 | 133269 | 12 |
| SKNSH2005120 | 0.011504832 | 217287 | 1 |
| SKNSH2005194 | 0.011504832 | 231893 | 1 |
| SKNSH2005240 | 0.01260011 | 98546 | 2 |
| SKNSH2005792 | 0.011504832 | 160331 | 1 |
| SKNSH2005816 | 0.011504832 | 219933 | 1 |
| SKNSH2005981 | 0.011504832 | 113222 | 1 |
| SKNSH2006234 | 0.047867269 | 199867 | 6 |
| SKNSH2006304 | 0.011504832 | 217391 | 1 |
| SKNSH2006432 | 0.011504832 | 163312 | 1 |
| SKNSH2006822 | 0.011504832 | 163652 | 1 |
| SKNSH2007142 | 0.011504832 | 74153 | 1 |
| SKNSH2007149 | 0.011504832 | 132810 | 1 |
| SKNSH2007429 | 0.011504832 | 78092 | 1 |
| SKNSH2007739 | 0.011504832 | 73330 | 1 |
| SKNSH2008043 | 0.016548115 | 162019 | 3 |
| SKNSH2008777 | 0.04267859 | 140574 | 9 |
| SKNSH2008940 | 0.087381065 | 47826 | 5 |
| SKNSH2008969 | 0.011504832 | 5599 | 1 |
| SKNSH2009197 | 0.011504832 | 49756 | 1 |
| SKNSH2009357 | 0.017539881 | 133546 | 3 |
| SKNSH2009435 | 0.011504832 | 126463 | 1 |
| SKNSH2009991 | 0.011504832 | 245728 | 1 |
| SKNSH2010015 | 0.011504832 | 242553 | 1 |
| SMINT1000012 | 0.005912612 | 17837 | 1 |
| SMINT1000016 | 0.068297746 | 22469 | 9 |
| SMINT1000039 | 0.01703732 | 58332 | 2 |
| SMINT1000042 | 0.062540386 | 15826 | 8 |
| SMINT1000048 | 0.021272859 | 60833 | 6 |
| SMINT1000051 | 0.005912612 | 57880 | 1 |
| SMINT1000054 | 0.008957493 | 62088 | 2 |
| SMINT1000057 | 0.009526503 | 67296 | 2 |
| SMINT1000071 | 0.005912612 | 53496 | 1 |
| SMINT1000075 | 0.005912612 | 37264 | 1 |
| SMINT1000078 | 0.017796859 | 39040 | 3 |
| SMINT1000091 | 0.005912612 | 68174 | 1 |
| SMINT1000100 | 0.06018931 | 168360 | 9 |
| SMINT1000103 | 0.005912612 | 60219 | 1 |
| SMINT1000117 | 0.127393401 | 39029 | 33 |
| SMINT1000118 | 0.005912612 | 40734 | 1 |
| SMINT1000131 | 0.182959568 | 58953 | 17 |
| SMINT1000137 | 0.008827469 | 53603 | 2 |
| SMINT1000178 | 0.070252924 | 41188 | 33 |
| SMINT1000192 | 0.056267369 | 74197 | 4 |
| SMINT2000007 | 0.027856852 | 103945 | 7 |
| SMINT2000018 | 0.039798556 | 109279 | 8 |
| SMINT2000032 | 0.005912612 | 238311 | 1 |
| SMINT2000040 | 0.005912612 | 116718 | 1 |
| SMINT2000060 | 0.005912612 | 263324 | 1 |
| SMINT2000073 | 0.019313987 | 17295 | 7 |
| SMINT2000084 | 0.005912612 | 266101 | 1 |
| SMINT2000145 | 0.005912612 | 171786 | 1 |
| SMINT2000159 | 0.030659106 | 91395 | 7 |
| SMINT2000176 | 0.011984994 | 189644 | 2 |
| SMINT2000185 | 0.005912612 | 37262 | 1 |
| SMINT2000227 | 0.005912612 | 267578 | 1 |
| SMINT2000229 | 0.005912612 | 152042 | 1 |
| SMINT2000232 | 0.011769768 | 276833 | 5 |
| SMINT2000239 | 0.019976545 | 125493 | 4 |
| SMINT2000267 | 0.007770726 | 171618 | 2 |
| SMINT2000277 | 0.092421108 | 121198 | 15 |
| SMINT2000396 | 0.136878017 | 38004 | 35 |
| SMINT2000400 | 0.025047457 | 29117 | 7 |
| SMINT2000441 | 0.371218566 | 37044 | 75 |
| SMINT2000454 | 0.018748294 | 58737 | 8 |
| SMINT2000468 | 0.073234454 | 104983 | 25 |
| SMINT2000518 | 0.005912612 | 268516 | 1 |
| SMINT2000529 | 0.005912612 | 277930 | 1 |
| SMINT2000530 | 0.005912612 | 123081 | 1 |
| SMINT2000537 | 0.007994124 | 110318 | 2 |
| SMINT2000538 | 0.005912612 | 141209 | 1 |
| SMINT2000541 | 0.005912612 | 154387 | 1 |
| SMINT2000545 | 0.070681319 | 115312 | 25 |
| SMINT2000558 | 0.005912612 | 277845 | 1 |
| SMINT2000562 | 0.005912612 | 267785 | 1 |
| SMINT2000568 | 0.005912612 | 63650 | 1 |
| SMINT2000602 | 0.304831045 | 145735 | 15 |
| SMINT2000609 | 0.008957493 | 206000 | 2 |
| SMINT2000629 | 0.062391264 | 165545 | 19 |
| SMINT2000659 | 0.603047235 | 155783 | 12 |
| SMINT2000694 | 0.140968152 | 10798 | 13 |
| SMINT2000747 | 0.010224624 | 157950 | 3 |
| SMINT2000811 | 0.005912612 | 155895 | 1 |
| SMINT2000824 | 0.005912612 | 193789 | 1 |
| SMINT2000933 | 0.164408747 | 150547 | 23 |
| SMINT2000984 | 0.005912612 | 225397 | 1 |
| SMINT2001114 | 0.005912612 | 97647 | 1 |
| SMINT2001158 | 0.005912612 | 178885 | 1 |
| SMINT2001183 | 0.018452584 | 84978 | 10 |
| SMINT2001195 | 0.019557654 | 170075 | 4 |
| SMINT2001199 | 0.01673981 | 102884 | 2 |
| SMINT2001222 | 0.04053865 | 235008 | 2 |
| SMINT2001348 | 0.018061496 | 116977 | 3 |
| SMINT2001397 | 0.005912612 | 156596 | 1 |
| SMINT2001458 | 0.005912612 | 218819 | 1 |
| SMINT2001461 | 0.005912612 | 225393 | 1 |
| SMINT2001535 | 0.005912612 | 37025 | 1 |
| SMINT2001559 | 0.005912612 | 161956 | 1 |
| SMINT2001609 | 0.005912612 | 24138 | 1 |
| SMINT2001615 | 0.009950895 | 198580 | 2 |
| SMINT2001683 | 0.005912612 | 46266 | 1 |
| SMINT2001731 | 0.005912612 | 181803 | 1 |
| SMINT2001812 | 0.020952521 | 161226 | 6 |
| SMINT2001818 | 0.178744123 | 72429 | 25 |
| SMINT2001950 | 0.015681739 | 136164 | 4 |
| SMINT2002126 | 0.005912612 | 208461 | 1 |
| SMINT2002159 | 0.024437993 | 188276 | 7 |
| SMINT2002202 | 0.005912612 | 156781 | 1 |
| SMINT2002210 | 0.005912612 | 2710 | 1 |
| SMINT2002224 | 0.013235363 | 133499 | 4 |
| SMINT2002281 | 0.011825223 | 267085 | 2 |
| SMINT2002311 | 0.031632486 | 135087 | 4 |
| SMINT2002314 | 0.138443826 | 100201 | 5 |
| SMINT2002328 | 0.009033418 | 135665 | 2 |
| SMINT2002414 | 0.005912612 | 206389 | 1 |
| SMINT2002457 | 0.005912612 | 2004 | 1 |
| SMINT2002620 | 0.073956527 | 109567 | 14 |
| SMINT2002689 | 0.142537791 | 53760 | 70 |
| SMINT2002743 | 0.005912612 | 155091 | 1 |
| SMINT2002778 | 0.007911013 | 20725 | 2 |
| SMINT2002880 | 0.005912612 | 118847 | 1 |
| SMINT2002882 | 0.057704881 | 111997 | 6 |
| SMINT2002884 | 0.045362008 | 127520 | 5 |
| SMINT2002899 | 0.005912612 | 170518 | 1 |
| SMINT2002974 | 0.005912612 | 109566 | 1 |
| SMINT2002976 | 0.032122891 | 23340 | 10 |
| SMINT2003003 | 0.008499011 | 163285 | 3 |
| SMINT2003074 | 0.122234053 | 110951 | 18 |
| SMINT2003128 | 0.005912612 | 193729 | 1 |
| SMINT2003150 | 0.007600888 | 95155 | 2 |
| SMINT2003169 | 0.356225718 | 47147 | 32 |
| SMINT2003317 | 0.005912612 | 171672 | 1 |
| SMINT2003340 | 0.057028111 | 171523 | 10 |
| SMINT2003386 | 0.005912612 | 22706 | 1 |
| SMINT2003423 | 0.015422967 | 110636 | 4 |
| SMINT2003505 | 0.053208096 | 109528 | 6 |
| SMINT2003551 | 0.0120783 | 138784 | 3 |
| SMINT2003569 | 0.005912612 | 150465 | 1 |
| SMINT2003641 | 0.005912612 | 174946 | 1 |
| SMINT2003644 | 0.005912612 | 108635 | 1 |
| SMINT2003866 | 0.005912612 | 56087 | 1 |
| SMINT2003905 | 0.216430435 | 40242 | 17 |
| SMINT2004023 | 0.109863374 | 111780 | 12 |
| SMINT2004037 | 0.008706922 | 39999 | 3 |
| SMINT2004047 | 0.005912612 | 24136 | 1 |
| SMINT2004086 | 0.005912612 | 76378 | 1 |
| SMINT2004144 | 0.369158109 | 19335 | 28 |
| SMINT2004280 | 0.01315123 | 120324 | 3 |
| SMINT2004299 | 0.018565802 | 33018 | 3 |
| SMINT2004339 | 0.005912612 | 136056 | 1 |
| SMINT2004350 | 0.012822826 | 120443 | 5 |
| SMINT2004414 | 0.007101856 | 278430 | 2 |
| SMINT2004473 | 0.005912612 | 101188 | 1 |
| SMINT2004547 | 0.005912612 | 193857 | 1 |
| SMINT2004583 | 0.043251512 | 30859 | 3 |
| SMINT2004589 | 0.041807918 | 124054 | 8 |
| SMINT2004729 | 0.013046391 | 149767 | 3 |
| SMINT2004781 | 0.005912612 | 150061 | 1 |
| SMINT2004872 | 0.079227914 | 152894 | 13 |
| SMINT2004891 | 0.005912612 | 29655 | 1 |
| SMINT2004909 | 0.005912612 | 229946 | 1 |
| SMINT2004972 | 0.021153384 | 99943 | 4 |
| SMINT2004992 | 0.005912612 | 199179 | 1 |
| SMINT2005075 | 0.005912612 | 193818 | 1 |
| SMINT2005161 | 0.005912612 | 192782 | 1 |
| SMINT2005174 | 0.005912612 | 193822 | 1 |
| SMINT2005213 | 0.005912612 | 190263 | 1 |
| SMINT2005330 | 0.01214897 | 131141 | 2 |
| SMINT2005368 | 0.14267833 | 63013 | 23 |
| SMINT2005387 | 0.007309767 | 231605 | 2 |
| SMINT2005402 | 0.007101856 | 218712 | 2 |
| SMINT2005405 | 0.005912612 | 94636 | 1 |
| SMINT2005621 | 0.005912612 | 225457 | 1 |
| SMINT2005623 | 0.005912612 | 251094 | 1 |
| SMINT2005624 | 0.005912612 | 250468 | 1 |
| SMINT2005688 | 0.067136107 | 72659 | 4 |
| SMINT2005800 | 0.005912612 | 191500 | 1 |
| SMINT2005956 | 0.063660854 | 82665 | 16 |
| SMINT2006078 | 0.005912612 | 231524 | 1 |
| SMINT2006205 | 0.005912612 | 30139 | 1 |
| SMINT2006331 | 0.005912612 | 170540 | 1 |
| SMINT2006596 | 0.005912612 | 173405 | 1 |
| SMINT2006641 | 0.005912612 | 173404 | 1 |
| SMINT2006648 | 0.005912612 | 215659 | 1 |
| SMINT2006708 | 0.005912612 | 139327 | 1 |
| SMINT2006716 | 0.014266482 | 104517 | 3 |
| SMINT2006801 | 0.010403447 | 142243 | 4 |
| SMINT2007062 | 0.007309767 | 111631 | 2 |
| SMINT2007101 | 0.007770726 | 135209 | 2 |
| SMINT2007140 | 0.005912612 | 228416 | 1 |
| SMINT2007187 | 0.02495963 | 14651 | 12 |
| SMINT2007219 | 0.005912612 | 17331 | 1 |
| SMINT2007294 | 0.005912612 | 219336 | 1 |
| SMINT2007365 | 0.005912612 | 277043 | 1 |
| SMINT2007391 | 0.005912612 | 248625 | 1 |
| SMINT2007433 | 0.020072751 | 1859 | 3 |
| SMINT2007508 | 0.010089547 | 100062 | 2 |
| SMINT2007526 | 0.005912612 | 15681 | 1 |
| SMINT2007579 | 0.005912612 | 165882 | 1 |
| SMINT2007647 | 0.007600888 | 152098 | 2 |
| SMINT2007729 | 0.029137113 | 34646 | 3 |
| SMINT2007790 | 0.005912612 | 151702 | 1 |
| SMINT2007792 | 0.020046584 | 83583 | 7 |
| SMINT2007796 | 0.005912612 | 212374 | 1 |
| SMINT2007867 | 0.011825223 | 179140 | 2 |
| SMINT2007932 | 0.005912612 | 198829 | 1 |
| SMINT2008042 | 0.005912612 | 217919 | 1 |
| SMINT2008054 | 0.008827469 | 198849 | 2 |
| SMINT2008133 | 0.007101856 | 127106 | 2 |
| SMINT2008277 | 0.005912612 | 197715 | 1 |
| SMINT2008329 | 0.005912612 | 257596 | 1 |
| SMINT2008455 | 0.005912612 | 257326 | 1 |
| SMINT2008491 | 0.005912612 | 104836 | 1 |
| SMINT2008531 | 0.005912612 | 176585 | 1 |
| SMINT2008545 | 0.005912612 | 264501 | 1 |
| SMINT2008558 | 0.005912612 | 164289 | 1 |
| SMINT2008590 | 0.017503822 | 174353 | 5 |
| SMINT2008625 | 0.005912612 | 203608 | 1 |
| SMINT2008672 | 0.012329383 | 125455 | 4 |
| SMINT2008690 | 0.005912612 | 172953 | 1 |
| SMINT2008715 | 0.011825223 | 165750 | 2 |
| SMINT2008720 | 0.005912612 | 201584 | 1 |
| SMINT2008817 | 0.005912612 | 105932 | 1 |
| SMINT2008844 | 0.005912612 | 170519 | 1 |
| SMINT2008869 | 0.011848387 | 128699 | 2 |
| SMINT2008917 | 0.014975542 | 171903 | 3 |
| SMINT2008921 | 0.151013325 | 90844 | 29 |
| SMINT2008960 | 0.244910965 | 11616 | 48 |
| SMINT2009119 | 0.005912612 | 150539 | 1 |
| SMINT2009121 | 0.005912612 | 19047 | 1 |
| SMINT2009212 | 0.005912612 | 149059 | 1 |
| SMINT2009216 | 0.030270392 | 195760 | 3 |
| SMINT2009233 | 0.007101856 | 217329 | 2 |
| SMINT2009259 | 0.005912612 | 60722 | 1 |
| SMINT2009272 | 0.005912612 | 160077 | 1 |
| SMINT2009292 | 0.047238991 | 84423 | 13 |
| SMINT2009363 | 0.005912612 | 224982 | 1 |
| SMINT2009415 | 0.007770726 | 99367 | 2 |
| SMINT2009468 | 0.005912612 | 155509 | 1 |
| SMINT2009505 | 0.005912612 | 190276 | 1 |
| SMINT2009529 | 0.005912612 | 103334 | 1 |
| SMINT2009832 | 0.018134562 | 125287 | 2 |
| SMINT2009895 | 0.005912612 | 198739 | 1 |
| SMINT2009902 | 0.005912612 | 264243 | 1 |
| SMINT2009973 | 0.005912612 | 27731 | 1 |
| SMINT2010015 | 0.005912612 | 206890 | 1 |
| SMINT2010068 | 0.005912612 | 221084 | 1 |
| SMINT2010076 | 0.005912612 | 216266 | 1 |
| SMINT2010084 | 0.048764241 | 20276 | 17 |
| SMINT2010144 | 0.007770726 | 75951 | 2 |
| SMINT2010200 | 0.017675934 | 89421 | 2 |
| SMINT2010278 | 0.005912612 | 280019 | 1 |
| SMINT2010324 | 0.007770726 | 98989 | 2 |
| SMINT2010326 | 0.005912612 | 140434 | 1 |
| SMINT2010369 | 0.007309767 | 109629 | 2 |
| SMINT2010426 | 0.030930367 | 154940 | 4 |
| SMINT2010500 | 0.005912612 | 251222 | 1 |
| SMINT2010533 | 0.011825223 | 38234 | 2 |
| SMINT2010629 | 0.005912612 | 120571 | 1 |
| SMINT2010639 | 0.061855819 | 102851 | 16 |
| SMINT2010672 | 0.005912612 | 270068 | 1 |
| SMINT2010753 | 0.005912612 | 65422 | 1 |
| SMINT2010853 | 0.005912612 | 205384 | 1 |
| SMINT2010897 | 0.005912612 | 152978 | 1 |
| SMINT2010901 | 0.047710153 | 38030 | 15 |
| SMINT2010959 | 0.01082587 | 40139 | 3 |
| SMINT2010997 | 0.005912612 | 148881 | 1 |
| SMINT2011033 | 0.007911013 | 59485 | 2 |
| SMINT2011066 | 0.005912612 | 98590 | 1 |
| SMINT2011273 | 0.005912612 | 271792 | 1 |
| SMINT2011311 | 0.008894571 | 159443 | 2 |
| SMINT2011406 | 0.005912612 | 171677 | 1 |
| SMINT2011509 | 0.005912612 | 203511 | 1 |
| SMINT2011567 | 0.017659914 | 23370 | 3 |
| SMINT2011588 | 0.009008108 | 142752 | 2 |
| SMINT2011656 | 0.007101856 | 159045 | 2 |
| SMINT2011888 | 0.005912612 | 176544 | 1 |
| SMINT2011958 | 0.005912612 | 135257 | 1 |
| SMINT2012040 | 0.005912612 | 127782 | 1 |
| SMINT2012122 | 0.047929182 | 105364 | 8 |
| SMINT2012179 | 0.009019227 | 31383 | 3 |
| SMINT2012195 | 0.007101856 | 135106 | 2 |
| SMINT2012285 | 0.005912612 | 228859 | 1 |
| SMINT2012291 | 0.036335971 | 162430 | 7 |
| SMINT2012501 | 0.021070903 | 12012 | 4 |
| SMINT2012734 | 0.005912612 | 128168 | 1 |
| SMINT2012735 | 0.020403493 | 124137 | 6 |
| SMINT2012793 | 0.005912612 | 272964 | 1 |
| SMINT2013031 | 0.053988011 | 64266 | 11 |
| SMINT2013032 | 0.014009091 | 108496 | 5 |
| SMINT2013129 | 0.005912612 | 66878 | 1 |
| SMINT2013170 | 0.015367088 | 162361 | 2 |
| SMINT2013181 | 0.007600888 | 197608 | 2 |
| SMINT2013228 | 0.005912612 | 170584 | 1 |
| SMINT2013613 | 0.005912612 | 171620 | 1 |
| SMINT2013626 | 0.005912612 | 176932 | 1 |
| SMINT2013695 | 0.007911013 | 18815 | 2 |
| SMINT2013833 | 0.005912612 | 132916 | 1 |
| SMINT2013890 | 0.01826134 | 149571 | 2 |
| SMINT2014166 | 0.005912612 | 158643 | 1 |
| SMINT2014282 | 0.005912612 | 92111 | 1 |
| SMINT2014443 | 0.005912612 | 261523 | 1 |
| SMINT2014480 | 0.105466368 | 57024 | 9 |
| SMINT2014489 | 0.005912612 | 185303 | 1 |
| SMINT2014721 | 0.005912612 | 124025 | 1 |
| SMINT2015006 | 0.005912612 | 139093 | 1 |
| SMINT2015294 | 0.021045914 | 49120 | 3 |
| SMINT2015306 | 0.01673981 | 164156 | 2 |
| SMINT2015326 | 0.005912612 | 49657 | 1 |
| SMINT2015353 | 0.005912612 | 164665 | 1 |
| SMINT2015454 | 0.005912612 | 128688 | 1 |
| SMINT2015518 | 0.005912612 | 36060 | 1 |
| SMINT2015745 | 0.005912612 | 196534 | 1 |
| SMINT2015787 | 0.005912612 | 166126 | 1 |
| SMINT2015810 | 0.005912612 | 154291 | 1 |
| SMINT2016122 | 0.005912612 | 112818 | 1 |
| SMINT2016146 | 0.009756696 | 93871 | 2 |
| SMINT2016286 | 0.005912612 | 175455 | 1 |
| SMINT2016313 | 0.005912612 | 74997 | 1 |
| SMINT2016396 | 0.011848387 | 174416 | 2 |
| SMINT2016412 | 0.088785792 | 131719 | 5 |
| SMINT2016440 | 0.005912612 | 101551 | 1 |
| SMINT2016477 | 0.005912612 | 66508 | 1 |
| SMINT2016544 | 0.005912612 | 48161 | 1 |
| SMINT2016857 | 0.005912612 | 181701 | 1 |
| SMINT2017134 | 0.007770726 | 187006 | 2 |
| SMINT2017319 | 0.005912612 | 182395 | 1 |
| SMINT2017324 | 0.005912612 | 178724 | 1 |
| SMINT2017410 | 0.005912612 | 214871 | 1 |
| SMINT2017431 | 0.005912612 | 187053 | 1 |
| SMINT2017432 | 0.005912612 | 3662 | 1 |
| SMINT2017436 | 0.005912612 | 149041 | 1 |
| SMINT2017599 | 0.040804447 | 158637 | 2 |
| SMINT2017659 | 0.005912612 | 160958 | 1 |
| SMINT2017736 | 0.005912612 | 3387 | 1 |
| SMINT2017781 | 0.005912612 | 256905 | 1 |
| SMINT2017855 | 0.005912612 | 166000 | 1 |
| SMINT2017884 | 0.007101856 | 176133 | 2 |
| SMINT2017964 | 0.034567508 | 90724 | 11 |
| SMINT2017974 | 0.005912612 | 263817 | 1 |
| SMINT2018343 | 0.005912612 | 22523 | 1 |
| SMINT2018353 | 0.017090792 | 36226 | 2 |
| SMINT2018592 | 0.005912612 | 124836 | 1 |
| SMINT2018681 | 0.040067287 | 11655 | 4 |
| SMINT2019017 | 0.005912612 | 270969 | 1 |
| SMINT2019105 | 0.005912612 | 27835 | 1 |
| SMINT2019142 | 0.011825223 | 122385 | 2 |
| SMINT2019153 | 0.005912612 | 175937 | 1 |
| SMINT2019200 | 0.005912612 | 171894 | 1 |
| SPLEN1000013 | 0.017218776 | 15244 | 3 |
| SPLEN1000041 | 0.004312012 | 74650 | 2 |
| SPLEN1000049 | 0.0076859 | 5000 | 3 |
| SPLEN1000056 | 0.004312012 | 74840 | 2 |
| SPLEN1000083 | 0.333649462 | 50383 | 87 |
| SPLEN1000087 | 0.002914857 | 63304 | 1 |
| SPLEN1000089 | 0.168663194 | 2591 | 12 |
| SPLEN1000091 | 0.002914857 | 72285 | 1 |
| SPLEN1000106 | 0.07198732 | 48973 | 32 |
| SPLEN1000116 | 0.338556398 | 73895 | 28 |
| SPLEN1000134 | 0.004791699 | 52904 | 2 |
| SPLEN1000139 | 0.005896816 | 13957 | 2 |
| SPLEN1000143 | 0.002914857 | 2277 | 1 |
| SPLEN1000144 | 0.032052177 | 59490 | 6 |
| SPLEN1000145 | 0.002914857 | 38633 | 1 |
| SPLEN1000156 | 0.002914857 | 79820 | 1 |
| SPLEN1000163 | 0.002914857 | 62664 | 1 |
| SPLEN1000165 | 0.03357555 | 59801 | 10 |
| SPLEN1000166 | 0.148223938 | 23861 | 43 |
| SPLEN1000178 | 0.002914857 | 242569 | 1 |
| SPLEN2000020 | 0.002914857 | 183944 | 1 |
| SPLEN2000047 | 0.010153475 | 177455 | 3 |
| SPLEN2000064 | 0.002914857 | 219170 | 1 |
| SPLEN2000072 | 0.027900558 | 170459 | 5 |
| SPLEN2000098 | 0.013910565 | 144282 | 8 |
| SPLEN2000126 | 0.002914857 | 213785 | 1 |
| SPLEN2000134 | 0.031982906 | 164686 | 7 |
| SPLEN2000143 | 0.007226869 | 101274 | 3 |
| SPLEN2000189 | 0.008744571 | 138974 | 3 |
| SPLEN2000197 | 0.004772971 | 12071 | 2 |
| SPLEN2000205 | 0.002914857 | 216742 | 1 |
| SPLEN2000210 | 0.012623595 | 164407 | 2 |
| SPLEN2000222 | 0.013717889 | 124147 | 4 |
| SPLEN2000242 | 0.017439185 | 141048 | 2 |
| SPLEN2000243 | 0.007707569 | 158484 | 4 |
| SPLEN2000247 | 0.002914857 | 146692 | 1 |
| SPLEN2000255 | 0.109103073 | 123372 | 34 |
| SPLEN2000258 | 0.038273399 | 71707 | 7 |
| SPLEN2000267 | 0.028700894 | 11452 | 15 |
| SPLEN2000270 | 0.002914857 | 115139 | 1 |
| SPLEN2000304 | 0.407966855 | 38528 | 119 |
| SPLEN2000307 | 0.002914857 | 219122 | 1 |
| SPLEN2000310 | 0.012623595 | 129027 | 2 |
| SPLEN2000341 | 0.01237386 | 67931 | 4 |
| SPLEN2000348 | 0.05989707 | 146825 | 12 |
| SPLEN2000350 | 0.002914857 | 128966 | 1 |
| SPLEN2000357 | 0.002914857 | 93707 | 1 |
| SPLEN2000364 | 0.002914857 | 285317 | 1 |
| SPLEN2000402 | 0.002914857 | 219110 | 1 |
| SPLEN2000414 | 0.012623595 | 127298 | 2 |
| SPLEN2000420 | 0.002914857 | 234978 | 1 |
| SPLEN2000443 | 0.007585093 | 185474 | 3 |
| SPLEN2000448 | 0.002914857 | 144581 | 1 |
| SPLEN2000450 | 0.002914857 | 136802 | 1 |
| SPLEN2000464 | 0.002914857 | 197263 | 1 |
| SPLEN2000496 | 0.002914857 | 197266 | 1 |
| SPLEN2000505 | 0.397595113 | 50221 | 57 |
| SPLEN2000515 | 0.002914857 | 215909 | 1 |
| SPLEN2000516 | 0.014017552 | 146888 | 6 |
| SPLEN2000537 | 0.004590404 | 134432 | 2 |
| SPLEN2000541 | 0.002914857 | 219141 | 1 |
| SPLEN2000607 | 0.002914857 | 125819 | 1 |
| SPLEN2000630 | 0.002914857 | 121423 | 1 |
| SPLEN2000695 | 0.002914857 | 136446 | 1 |
| SPLEN2000698 | 0.079018445 | 63416 | 8 |
| SPLEN2000839 | 0.018357265 | 97021 | 4 |
| SPLEN2000874 | 0.343764444 | 104953 | 13 |
| SPLEN2000882 | 0.095383222 | 127390 | 21 |
| SPLEN2001135 | 0.002914857 | 218565 | 1 |
| SPLEN2001141 | 0.177436641 | 59612 | 51 |
| SPLEN2001157 | 0.060917825 | 27211 | 16 |
| SPLEN2001176 | 0.002914857 | 81078 | 1 |
| SPLEN2001227 | 0.002914857 | 197240 | 1 |
| SPLEN2001245 | 0.149973681 | 23990 | 2 |
| SPLEN2001278 | 0.004312012 | 34574 | 2 |
| SPLEN2001354 | 0.002914857 | 124005 | 1 |
| SPLEN2001367 | 0.002914857 | 47344 | 1 |
| SPLEN2001383 | 0.002914857 | 124432 | 1 |
| SPLEN2001476 | 0.004312012 | 88714 | 2 |
| SPLEN2001503 | 0.016656912 | 146686 | 3 |
| SPLEN2001510 | 0.002914857 | 193264 | 1 |
| SPLEN2001599 | 0.023481819 | 119291 | 5 |
| SPLEN2001626 | 0.008956423 | 155295 | 2 |
| SPLEN2001650 | 0.002914857 | 154524 | 1 |
| SPLEN2001689 | 0.01200505 | 63113 | 4 |
| SPLEN2001710 | 0.043211036 | 43332 | 4 |
| SPLEN2001761 | 0.015746765 | 41068 | 5 |
| SPLEN2001781 | 0.002914857 | 178412 | 1 |
| SPLEN2001881 | 0.002914857 | 178421 | 1 |
| SPLEN2001912 | 0.002914857 | 126730 | 1 |
| SPLEN2001945 | 0.002914857 | 65178 | 1 |
| SPLEN2002007 | 0.008780069 | 115624 | 3 |
| SPLEN2002053 | 0.004590404 | 188235 | 2 |
| SPLEN2002133 | 0.004010135 | 165891 | 2 |
| SPLEN2002147 | 0.002914857 | 171784 | 1 |
| SPLEN2002166 | 0.002914857 | 106920 | 1 |
| SPLEN2002175 | 0.002914857 | 188139 | 1 |
| SPLEN2002223 | 0.004791699 | 231695 | 2 |
| SPLEN2002272 | 0.008956423 | 157958 | 2 |
| SPLEN2002314 | 0.002914857 | 253205 | 1 |
| SPLEN2002335 | 0.165480591 | 90306 | 44 |
| SPLEN2002343 | 0.002914857 | 203944 | 1 |
| SPLEN2002354 | 0.002914857 | 108707 | 1 |
| SPLEN2002385 | 0.002914857 | 58410 | 1 |
| SPLEN2002419 | 0.002914857 | 33084 | 1 |
| SPLEN2002451 | 0.004312012 | 146903 | 2 |
| SPLEN2002462 | 0.002914857 | 191105 | 1 |
| SPLEN2002463 | 0.022126491 | 89565 | 13 |
| SPLEN2002467 | 0.051432664 | 185854 | 10 |
| SPLEN2002477 | 0.103114815 | 109807 | 21 |
| SPLEN2002493 | 0.09680775 | 97695 | 51 |
| SPLEN2002662 | 0.002914857 | 45526 | 1 |
| SPLEN2002695 | 0.002914857 | 90699 | 1 |
| SPLEN2002707 | 0.002914857 | 245667 | 1 |
| SPLEN2002744 | 0.075126312 | 91738 | 8 |
| SPLEN2002917 | 0.002914857 | 221413 | 1 |
| SPLEN2002931 | 0.041086754 | 179234 | 3 |
| SPLEN2002957 | 0.005829714 | 178408 | 2 |
| SPLEN2003160 | 0.002914857 | 249846 | 1 |
| SPLEN2003204 | 0.002914857 | 198617 | 1 |
| SPLEN2003219 | 0.002914857 | 183601 | 1 |
| SPLEN2003297 | 0.006310414 | 159270 | 3 |
| SPLEN2003396 | 0.008956423 | 208265 | 2 |
| SPLEN2003678 | 0.004772971 | 234930 | 2 |
| SPLEN2003878 | 0.002914857 | 204112 | 1 |
| SPLEN2003918 | 0.002914857 | 101303 | 1 |
| SPLEN2003924 | 0.002914857 | 9892 | 1 |
| SPLEN2004060 | 0.002914857 | 180659 | 1 |
| SPLEN2004078 | 0.011212672 | 47273 | 4 |
| SPLEN2004124 | 0.002914857 | 22072 | 1 |
| SPLEN2004148 | 0.002914857 | 210540 | 1 |
| SPLEN2004181 | 0.021284736 | 157722 | 4 |
| SPLEN2004220 | 0.058259113 | 113792 | 7 |
| SPLEN2004343 | 0.00931331 | 115882 | 5 |
| SPLEN2004346 | 0.002914857 | 231171 | 1 |
| SPLEN2004368 | 0.002914857 | 147648 | 1 |
| SPLEN2004555 | 0.157417742 | 86802 | 39 |
| SPLEN2004611 | 0.004312012 | 113563 | 2 |
| SPLEN2004662 | 0.0088958 | 173909 | 4 |
| SPLEN2004773 | 0.235431317 | 1655 | 47 |
| SPLEN2004844 | 0.014675412 | 256461 | 2 |
| SPLEN2004880 | 0.568857651 | 31712 | 7 |
| SPLEN2004984 | 0.063790519 | 119307 | 12 |
| SPLEN2005009 | 0.019490293 | 133889 | 8 |
| SPLEN2005142 | 0.157417742 | 86802 | 39 |
| SPLEN2005227 | 0.002914857 | 125072 | 1 |
| SPLEN2005272 | 0.002914857 | 77774 | 1 |
| SPLEN2005342 | 0.031408064 | 92906 | 10 |
| SPLEN2005416 | 0.004791699 | 115677 | 2 |
| SPLEN2005429 | 0.045734728 | 96931 | 19 |
| SPLEN2005450 | 0.002914857 | 102480 | 1 |
| SPLEN2005474 | 0.002914857 | 43252 | 1 |
| SPLEN2005560 | 0.159183453 | 122274 | 5 |
| SPLEN2005727 | 0.002914857 | 227937 | 1 |
| SPLEN2005767 | 0.043916771 | 115981 | 10 |
| SPLEN2005783 | 0.025703173 | 62449 | 11 |
| SPLEN2005790 | 0.004312012 | 8060 | 2 |
| SPLEN2005806 | 0.274005729 | 74118 | 12 |
| SPLEN2005818 | 0.015032227 | 153072 | 7 |
| SPLEN2005838 | 0.004624317 | 140602 | 2 |
| SPLEN2005869 | 0.026090564 | 183059 | 3 |
| SPLEN2005927 | 0.038909785 | 140379 | 6 |
| SPLEN2005939 | 0.002914857 | 234216 | 1 |
| SPLEN2006122 | 0.198950286 | 52473 | 14 |
| SPLEN2006133 | 0.176626238 | 108761 | 31 |
| SPLEN2006143 | 0.074889021 | 89002 | 14 |
| SPLEN2006232 | 0.058412074 | 129454 | 12 |
| SPLEN2006268 | 0.002914857 | 266784 | 1 |
| SPLEN2006283 | 0.002914857 | 43327 | 1 |
| SPLEN2006305 | 0.265258593 | 111292 | 78 |
| SPLEN2006325 | 0.020111948 | 140126 | 3 |
| SPLEN2006374 | 0.110236555 | 92380 | 37 |
| SPLEN2006389 | 0.002914857 | 47968 | 1 |
| SPLEN2006425 | 0.002914857 | 263011 | 1 |
| SPLEN2006671 | 0.008969225 | 102925 | 2 |
| SPLEN2006701 | 0.002914857 | 124791 | 1 |
| SPLEN2006785 | 0.002914857 | 115943 | 1 |
| SPLEN2006875 | 0.002914857 | 122324 | 1 |
| SPLEN2007182 | 0.013109569 | 127097 | 4 |
| SPLEN2007276 | 0.063179234 | 112688 | 7 |
| SPLEN2007314 | 0.002914857 | 280002 | 1 |
| SPLEN2007326 | 0.004312012 | 17406 | 2 |
| SPLEN2007350 | 0.002914857 | 109816 | 1 |
| SPLEN2007388 | 0.006310414 | 73426 | 3 |
| SPLEN2007498 | 0.064024495 | 110806 | 23 |
| SPLEN2007619 | 0.002914857 | 74708 | 1 |
| SPLEN2007653 | 0.002914857 | 117250 | 1 |
| SPLEN2007689 | 0.002914857 | 116899 | 1 |
| SPLEN2007695 | 0.002914857 | 71541 | 1 |
| SPLEN2007739 | 0.027594397 | 34940 | 15 |
| SPLEN2007750 | 0.034632523 | 159116 | 5 |
| SPLEN2007794 | 0.016906471 | 139570 | 3 |
| SPLEN2007879 | 0.011621779 | 10093 | 4 |
| SPLEN2007926 | 0.002914857 | 51442 | 1 |
| SPLEN2007951 | 0.007091792 | 117347 | 2 |
| SPLEN2008007 | 0.002914857 | 13206 | 1 |
| SPLEN2008135 | 0.002914857 | 164479 | 1 |
| SPLEN2008164 | 0.002914857 | 132782 | 1 |
| SPLEN2008317 | 0.002914857 | 207510 | 1 |
| SPLEN2008460 | 0.127900964 | 117911 | 17 |
| SPLEN2008496 | 0.021641601 | 28078 | 3 |
| SPLEN2008591 | 0.004010135 | 48605 | 2 |
| SPLEN2008737 | 0.087450229 | 54463 | 31 |
| SPLEN2008786 | 0.002914857 | 72070 | 1 |
| SPLEN2009081 | 0.025888668 | 107596 | 5 |
| SPLEN2009088 | 0.002914857 | 92525 | 1 |
| SPLEN2009163 | 0.009117177 | 124897 | 2 |
| SPLEN2009315 | 0.004913258 | 222877 | 2 |
| SPLEN2009340 | 0.002914857 | 73613 | 1 |
| SPLEN2009512 | 0.010117191 | 141564 | 3 |
| SPLEN2009513 | 0.02702602 | 105252 | 4 |
| SPLEN2009541 | 0.002914857 | 35663 | 1 |
| SPLEN2009548 | 0.002914857 | 103115 | 1 |
| SPLEN2009555 | 0.019726366 | 97430 | 6 |
| SPLEN2009581 | 0.002914857 | 143353 | 1 |
| SPLEN2009733 | 0.002914857 | 135216 | 1 |
| SPLEN2009803 | 0.002914857 | 41978 | 1 |
| SPLEN2009884 | 0.002914857 | 154849 | 1 |
| SPLEN2009918 | 0.002914857 | 56743 | 1 |
| SPLEN2009970 | 0.032855635 | 142120 | 11 |
| SPLEN2010004 | 0.002914857 | 118013 | 1 |
| SPLEN2010015 | 0.010778292 | 122423 | 6 |
| SPLEN2010112 | 0.002914857 | 115375 | 1 |
| SPLEN2010119 | 0.002914857 | 54489 | 1 |
| SPLEN2010146 | 0.002914857 | 141650 | 1 |
| SPLEN2010187 | 0.002914857 | 115448 | 1 |
| SPLEN2010195 | 0.43188623 | 103198 | 98 |
| SPLEN2010350 | 0.002914857 | 113494 | 1 |
| SPLEN2010395 | 0.002914857 | 263561 | 1 |
| SPLEN2010415 | 0.002914857 | 122061 | 1 |
| SPLEN2010447 | 0.002914857 | 283351 | 1 |
| SPLEN2010469 | 0.006758941 | 14538 | 2 |
| SPLEN2010510 | 0.002914857 | 120657 | 1 |
| SPLEN2010530 | 0.002914857 | 139438 | 1 |
| SPLEN2010534 | 0.002914857 | 141408 | 1 |
| SPLEN2010588 | 0.002914857 | 169428 | 1 |
| SPLEN2010625 | 0.004312012 | 153873 | 2 |
| SPLEN2010800 | 0.002914857 | 42885 | 1 |
| SPLEN2010846 | 0.007293972 | 53766 | 3 |
| SPLEN2010847 | 0.01015847 | 51801 | 3 |
| SPLEN2010862 | 0.002914857 | 257653 | 1 |
| SPLEN2010912 | 0.013528832 | 121273 | 8 |
| SPLEN2010978 | 0.002914857 | 132321 | 1 |
| SPLEN2010986 | 0.008320186 | 50421 | 4 |
| SPLEN2011018 | 0.002914857 | 8437 | 1 |
| SPLEN2011021 | 0.010224624 | 57866 | 3 |
| SPLEN2011025 | 0.008827469 | 145110 | 2 |
| SPLEN2011086 | 0.002914857 | 129479 | 1 |
| SPLEN2011139 | 0.002914857 | 94065 | 1 |
| SPLEN2011145 | 0.002914857 | 135972 | 1 |
| SPLEN2011248 | 0.002914857 | 125718 | 1 |
| SPLEN2011252 | 0.002914857 | 246970 | 1 |
| SPLEN2011419 | 0.070119935 | 21021 | 12 |
| SPLEN2011422 | 0.002914857 | 138473 | 1 |
| SPLEN2011672 | 0.002914857 | 85403 | 1 |
| SPLEN2011678 | 0.147020482 | 115451 | 3 |
| SPLEN2011737 | 0.064312131 | 93659 | 24 |
| SPLEN2011758 | 0.031902823 | 65392 | 7 |
| SPLEN2011766 | 0.010254136 | 110461 | 3 |
| SPLEN2011805 | 0.002914857 | 38362 | 1 |
| SPLEN2011820 | 0.002914857 | 115381 | 1 |
| SPLEN2011830 | 0.005829714 | 110459 | 2 |
| SPLEN2011931 | 0.002914857 | 120907 | 1 |
| SPLEN2011981 | 0.002914857 | 114150 | 1 |
| SPLEN2012115 | 0.040937671 | 198255 | 2 |
| SPLEN2012175 | 0.002914857 | 32655 | 1 |
| SPLEN2012179 | 0.002914857 | 186778 | 1 |
| SPLEN2012185 | 0.002914857 | 45540 | 1 |
| SPLEN2012234 | 0.002914857 | 112345 | 1 |
| SPLEN2012453 | 0.004104101 | 142770 | 2 |
| SPLEN2012523 | 0.156930511 | 54625 | 9 |
| SPLEN2012524 | 0.002914857 | 39246 | 1 |
| SPLEN2012525 | 0.002914857 | 56713 | 1 |
| SPLEN2012571 | 0.052707355 | 137693 | 13 |
| SPLEN2012606 | 0.002914857 | 57108 | 1 |
| SPLEN2012611 | 0.011671177 | 118055 | 3 |
| SPLEN2012619 | 0.004590404 | 107825 | 2 |
| SPLEN2012624 | 0.033137229 | 120091 | 12 |
| SPLEN2012725 | 0.002914857 | 196965 | 1 |
| SPLEN2012743 | 0.002914857 | 58862 | 1 |
| SPLEN2012756 | 0.002914857 | 138971 | 1 |
| SPLEN2012800 | 0.490568327 | 127014 | 14 |
| SPLEN2012846 | 0.006188854 | 9927 | 3 |
| SPLEN2012889 | 0.004312012 | 72891 | 2 |
| SPLEN2012961 | 0.002914857 | 281990 | 1 |
| SPLEN2013108 | 0.004791699 | 125465 | 2 |
| SPLEN2013195 | 0.004104101 | 23933 | 2 |
| SPLEN2013503 | 0.066757206 | 147838 | 8 |
| SPLEN2013644 | 0.002914857 | 61762 | 1 |
| SPLEN2013648 | 0.002914857 | 67298 | 1 |
| SPLEN2013670 | 0.014289976 | 132837 | 6 |
| SPLEN2013673 | 0.002914857 | 113244 | 1 |
| SPLEN2013690 | 0.015515464 | 119263 | 3 |
| SPLEN2013714 | 0.002914857 | 73207 | 1 |
| SPLEN2013753 | 0.004312012 | 125588 | 2 |
| SPLEN2013821 | 0.022476672 | 112139 | 2 |
| SPLEN2013857 | 0.004590404 | 37671 | 2 |
| SPLEN2013860 | 0.002914857 | 39794 | 1 |
| SPLEN2013910 | 0.002914857 | 270087 | 1 |
| SPLEN2013923 | 0.006188854 | 85699 | 3 |
| SPLEN2013936 | 0.025112439 | 139079 | 5 |
| SPLEN2014041 | 0.004312012 | 150722 | 2 |
| SPLEN2014063 | 0.002914857 | 56758 | 1 |
| SPLEN2014073 | 0.014201539 | 35718 | 2 |
| SPLEN2014080 | 0.035364657 | 148087 | 9 |
| SPLEN2014119 | 0.010039876 | 148011 | 3 |
| SPLEN2014136 | 0.005829714 | 160302 | 2 |
| SPLEN2014199 | 0.002914857 | 134877 | 1 |
| SPLEN2014210 | 0.002914857 | 133008 | 1 |
| SPLEN2014293 | 0.002914857 | 124023 | 1 |
| SPLEN2014318 | 0.005829714 | 161544 | 2 |
| SPLEN2014381 | 0.011268727 | 106130 | 3 |
| SPLEN2014452 | 0.009117177 | 129789 | 2 |
| SPLEN2014454 | 0.002914857 | 129788 | 1 |
| SPLEN2014486 | 0.002914857 | 146475 | 1 |
| SPLEN2014543 | 0.002914857 | 138117 | 1 |
| SPLEN2014572 | 0.026923245 | 35256 | 6 |
| SPLEN2014645 | 0.009151215 | 145380 | 2 |
| SPLEN2014669 | 0.005829714 | 117142 | 2 |
| SPLEN2014711 | 0.063175608 | 111782 | 4 |
| SPLEN2014739 | 0.011538882 | 140868 | 5 |
| SPLEN2014860 | 0.005829714 | 113220 | 2 |
| SPLEN2014865 | 0.002914857 | 129901 | 1 |
| SPLEN2014911 | 0.158823404 | 97253 | 28 |
| SPLEN2014919 | 0.002914857 | 179198 | 1 |
| SPLEN2014920 | 0.002914857 | 59356 | 1 |
| SPLEN2014924 | 0.002914857 | 54173 | 1 |
| SPLEN2014937 | 0.002914857 | 147868 | 1 |
| SPLEN2014946 | 0.002914857 | 134758 | 1 |
| SPLEN2015031 | 0.002914857 | 51951 | 1 |
| SPLEN2015094 | 0.002914857 | 67845 | 1 |
| SPLEN2015121 | 0.004312012 | 122862 | 2 |
| SPLEN2015129 | 0.004590404 | 5661 | 2 |
| SPLEN2015158 | 0.002914857 | 150635 | 1 |
| SPLEN2015166 | 0.008827469 | 48798 | 2 |
| SPLEN2015216 | 0.002914857 | 101211 | 1 |
| SPLEN2015258 | 0.002914857 | 125238 | 1 |
| SPLEN2015261 | 0.002914857 | 134163 | 1 |
| SPLEN2015267 | 0.002914857 | 283801 | 1 |
| SPLEN2015276 | 0.002914857 | 142847 | 1 |
| SPLEN2015310 | 0.292333103 | 48032 | 31 |
| SPLEN2015415 | 0.002914857 | 126941 | 1 |
| SPLEN2015433 | 0.002914857 | 113232 | 1 |
| SPLEN2015593 | 0.002914857 | 113200 | 1 |
| SPLEN2015674 | 0.002914857 | 109737 | 1 |
| SPLEN2015679 | 0.002914857 | 17417 | 1 |
| SPLEN2015707 | 0.002914857 | 135155 | 1 |
| SPLEN2015730 | 0.01197524 | 146144 | 3 |
| SPLEN2015746 | 0.002914857 | 281810 | 1 |
| SPLEN2015788 | 0.034613688 | 115925 | 11 |
| SPLEN2015815 | 0.002914857 | 156260 | 1 |
| SPLEN2015890 | 0.004104101 | 72495 | 2 |
| SPLEN2015891 | 0.002914857 | 53226 | 1 |
| SPLEN2015899 | 0.002914857 | 99000 | 1 |
| SPLEN2016045 | 0.002914857 | 165479 | 1 |
| SPLEN2016053 | 0.002914857 | 273281 | 1 |
| SPLEN2016069 | 0.002914857 | 269710 | 1 |
| SPLEN2016098 | 0.009117177 | 100865 | 2 |
| SPLEN2016135 | 0.002914857 | 118167 | 1 |
| SPLEN2016139 | 0.130771767 | 99113 | 9 |
| SPLEN2016241 | 0.002914857 | 164252 | 1 |
| SPLEN2016250 | 0.002914857 | 12746 | 1 |
| SPLEN2016268 | 0.387829361 | 78383 | 48 |
| SPLEN2016304 | 0.002914857 | 110558 | 1 |
| SPLEN2016356 | 0.002914857 | 138736 | 1 |
| SPLEN2016421 | 0.080880372 | 49655 | 17 |
| SPLEN2016480 | 0.002914857 | 138399 | 1 |
| SPLEN2016531 | 0.002914857 | 271544 | 1 |
| SPLEN2016533 | 0.004772971 | 17471 | 2 |
| SPLEN2016541 | 0.002914857 | 217915 | 1 |
| SPLEN2016552 | 0.002914857 | 79500 | 1 |
| SPLEN2016554 | 0.008990496 | 79138 | 4 |
| SPLEN2016602 | 0.002914857 | 169370 | 1 |
| SPLEN2016627 | 0.002914857 | 151306 | 1 |
| SPLEN2016678 | 0.008503479 | 71061 | 5 |
| SPLEN2016743 | 0.029223877 | 72231 | 6 |
| SPLEN2016773 | 0.004104101 | 110853 | 2 |
| SPLEN2016781 | 0.004312012 | 138933 | 2 |
| SPLEN2016840 | 0.002914857 | 242181 | 1 |
| SPLEN2016863 | 0.002914857 | 275752 | 1 |
| SPLEN2016922 | 0.002914857 | 107663 | 1 |
| SPLEN2016932 | 0.004772971 | 153348 | 2 |
| SPLEN2016972 | 0.082631374 | 113807 | 13 |
| SPLEN2016973 | 0.002914857 | 17865 | 1 |
| SPLEN2017031 | 0.138538996 | 109565 | 24 |
| SPLEN2017043 | 0.016098905 | 119417 | 3 |
| SPLEN2017104 | 0.002914857 | 253301 | 1 |
| SPLEN2017121 | 0.021611965 | 135937 | 6 |
| SPLEN2017147 | 0.002914857 | 110292 | 1 |
| SPLEN2017187 | 0.002914857 | 115736 | 1 |
| SPLEN2017189 | 0.002914857 | 115047 | 1 |
| SPLEN2017212 | 0.002914857 | 162530 | 1 |
| SPLEN2017318 | 0.033021481 | 31715 | 16 |
| SPLEN2017351 | 0.055706502 | 135186 | 24 |
| SPLEN2017426 | 0.002914857 | 130108 | 1 |
| SPLEN2017452 | 0.002914857 | 176395 | 1 |
| SPLEN2017538 | 0.002914857 | 6188 | 1 |
| SPLEN2017592 | 0.004104101 | 109937 | 2 |
| SPLEN2017613 | 0.002914857 | 39284 | 1 |
| SPLEN2017620 | 0.032689027 | 60349 | 6 |
| SPLEN2017643 | 0.002914857 | 138421 | 1 |
| SPLEN2017687 | 0.002914857 | 270715 | 1 |
| SPLEN2017740 | 0.002914857 | 83086 | 1 |
| SPLEN2017781 | 0.004104101 | 30939 | 2 |
| SPLEN2017918 | 0.183776349 | 28073 | 21 |
| SPLEN2017981 | 0.051769295 | 91638 | 26 |
| SPLEN2017999 | 0.002914857 | 154787 | 1 |
| SPLEN2018098 | 0.004913258 | 47527 | 2 |
| SPLEN2018112 | 0.002914857 | 134989 | 1 |
| SPLEN2018157 | 0.069466112 | 97654 | 21 |
| SPLEN2018159 | 0.017692446 | 30104 | 4 |
| SPLEN2018180 | 0.002914857 | 36841 | 1 |
| SPLEN2018181 | 0.002914857 | 145736 | 1 |
| SPLEN2018280 | 0.002914857 | 267675 | 1 |
| SPLEN2018285 | 0.002914857 | 86331 | 1 |
| SPLEN2018299 | 0.008142793 | 129049 | 4 |
| SPLEN2018302 | 0.027555401 | 60772 | 4 |
| SPLEN2018364 | 0.002914857 | 154737 | 1 |
| SPLEN2018395 | 0.002914857 | 187543 | 1 |
| SPLEN2018643 | 0.004590404 | 30252 | 2 |
| SPLEN2018683 | 0.004312012 | 82417 | 2 |
| SPLEN2018732 | 0.002914857 | 104525 | 1 |
| SPLEN2018749 | 0.002914857 | 107273 | 1 |
| SPLEN2018933 | 0.002914857 | 186700 | 1 |
| SPLEN2019008 | 0.002914857 | 1122 | 1 |
| SPLEN2019015 | 0.257097629 | 111157 | 31 |
| SPLEN2019043 | 0.002914857 | 62255 | 1 |
| SPLEN2019077 | 0.03158465 | 192819 | 5 |
| SPLEN2019092 | 0.006035663 | 191999 | 2 |
| SPLEN2019102 | 0.002914857 | 178887 | 1 |
| SPLEN2019131 | 0.002914857 | 187019 | 1 |
| SPLEN2019169 | 0.029596594 | 130371 | 18 |
| SPLEN2019257 | 0.004104101 | 29121 | 2 |
| SPLEN2019311 | 0.00691166 | 150786 | 3 |
| SPLEN2019323 | 0.002914857 | 153382 | 1 |
| SPLEN2019349 | 0.004624317 | 206596 | 2 |
| SPLEN2019375 | 0.002914857 | 114599 | 1 |
| SPLEN2019379 | 0.014675412 | 42169 | 2 |
| SPLEN2019405 | 0.117003473 | 35419 | 33 |
| SPLEN2019446 | 0.016102262 | 136509 | 4 |
| SPLEN2019471 | 0.002914857 | 235419 | 1 |
| SPLEN2019480 | 0.002914857 | 184785 | 1 |
| SPLEN2019505 | 0.034178208 | 1156 | 5 |
| SPLEN2019571 | 0.002914857 | 94465 | 1 |
| SPLEN2019575 | 0.002914857 | 71649 | 1 |
| SPLEN2019709 | 0.002914857 | 219496 | 1 |
| SPLEN2019774 | 0.018859953 | 261630 | 3 |
| SPLEN2019793 | 0.002914857 | 130329 | 1 |
| SPLEN2019811 | 0.002914857 | 264766 | 1 |
| SPLEN2019839 | 0.002914857 | 138179 | 1 |
| SPLEN2019956 | 0.005501257 | 17638 | 3 |
| SPLEN2019985 | 0.002914857 | 247445 | 1 |
| SPLEN2020007 | 0.008969225 | 222123 | 2 |
| SPLEN2020034 | 0.006758941 | 106987 | 2 |
| SPLEN2020153 | 0.002914857 | 251920 | 1 |
| SPLEN2020154 | 0.002914857 | 39800 | 1 |
| SPLEN2020166 | 0.002914857 | 240411 | 1 |
| SPLEN2020183 | 0.002914857 | 38398 | 1 |
| SPLEN2020223 | 0.002914857 | 17760 | 1 |
| SPLEN2020359 | 0.002914857 | 149164 | 1 |
| SPLEN2020417 | 0.012003782 | 107233 | 4 |
| SPLEN2020427 | 0.002914857 | 211084 | 1 |
| SPLEN2020467 | 0.002914857 | 245115 | 1 |
| SPLEN2020512 | 0.002914857 | 37024 | 1 |
| SPLEN2020608 | 0.002914857 | 49071 | 1 |
| SPLEN2020777 | 0.002914857 | 32229 | 1 |
| SPLEN2020805 | 0.005829714 | 223978 | 2 |
| SPLEN2021057 | 0.002914857 | 34461 | 1 |
| SPLEN2021122 | 0.010273404 | 88683 | 5 |
| SPLEN2021157 | 0.002914857 | 243715 | 1 |
| SPLEN2021194 | 0.04967068 | 112526 | 12 |
| SPLEN2021231 | 0.002914857 | 229123 | 1 |
| SPLEN2021273 | 0.011361777 | 69503 | 5 |
| SPLEN2021295 | 0.002914857 | 99177 | 1 |
| SPLEN2021298 | 0.002914857 | 49036 | 1 |
| SPLEN2021383 | 0.002914857 | 147669 | 1 |
| SPLEN2021417 | 0.002914857 | 203080 | 1 |
| SPLEN2021440 | 0.002914857 | 178070 | 1 |
| SPLEN2021458 | 0.004772971 | 125487 | 2 |
| SPLEN2021463 | 0.002914857 | 136359 | 1 |
| SPLEN2021560 | 0.002914857 | 278460 | 1 |
| SPLEN2021701 | 0.002914857 | 254978 | 1 |
| SPLEN2021795 | 0.002914857 | 144403 | 1 |
| SPLEN2021983 | 0.002914857 | 223308 | 1 |
| SPLEN2021991 | 0.002914857 | 223312 | 1 |
| SPLEN2022040 | 0.002914857 | 212597 | 1 |
| SPLEN2022162 | 0.005829714 | 140706 | 2 |
| SPLEN2022227 | 0.002914857 | 70966 | 1 |
| SPLEN2022522 | 0.118200644 | 70961 | 13 |
| SPLEN2022591 | 0.002914857 | 237801 | 1 |
| SPLEN2022785 | 0.002914857 | 280669 | 1 |
| SPLEN2022920 | 0.002914857 | 226358 | 1 |
| SPLEN2023024 | 0.002914857 | 167543 | 1 |
| SPLEN2023118 | 0.015052952 | 50841 | 3 |
| SPLEN2023160 | 0.008524334 | 165524 | 5 |
| SPLEN2023423 | 0.002914857 | 164527 | 1 |
| SPLEN2023706 | 0.015521841 | 43100 | 5 |
| SPLEN2023710 | 0.002914857 | 154177 | 1 |
| SPLEN2023733 | 0.008322148 | 136308 | 4 |
| SPLEN2023791 | 0.005896816 | 232557 | 2 |
| SPLEN2023977 | 0.002914857 | 6565 | 1 |
| SPLEN2024070 | 0.002914857 | 105000 | 1 |
| SPLEN2024127 | 0.002914857 | 218242 | 1 |
| SPLEN2024232 | 0.025261465 | 146441 | 3 |
| SPLEN2024273 | 0.002914857 | 248214 | 1 |
| SPLEN2024383 | 0.002914857 | 100265 | 1 |
| SPLEN2024489 | 0.015335638 | 35425 | 6 |
| SPLEN2024530 | 0.002914857 | 32001 | 1 |
| SPLEN2024571 | 0.002914857 | 135218 | 1 |
| SPLEN2024577 | 0.002914857 | 173096 | 1 |
| SPLEN2024617 | 0.002914857 | 136055 | 1 |
| SPLEN2024737 | 0.002914857 | 162799 | 1 |
| SPLEN2024922 | 0.006501159 | 143618 | 3 |
| SPLEN2024956 | 0.002914857 | 209783 | 1 |
| SPLEN2024990 | 0.007226869 | 59340 | 3 |
| SPLEN2025012 | 0.002914857 | 243040 | 1 |
| SPLEN2025017 | 0.004996369 | 220204 | 2 |
| SPLEN2025039 | 0.004104101 | 45184 | 2 |
| SPLEN2025219 | 0.002914857 | 169452 | 1 |
| SPLEN2025491 | 0.004772971 | 199432 | 2 |
| SPLEN2025626 | 0.002914857 | 244518 | 1 |
| SPLEN2025820 | 0.002914857 | 229023 | 1 |
| SPLEN2026144 | 0.002914857 | 139012 | 1 |
| SPLEN2026411 | 0.01470175 | 144177 | 2 |
| SPLEN2026765 | 0.002914857 | 178984 | 1 |
| SPLEN2027113 | 0.102397727 | 132649 | 21 |
| SPLEN2027157 | 0.002914857 | 40822 | 1 |
| SPLEN2027268 | 0.008281036 | 72901 | 3 |
| SPLEN2027395 | 0.002914857 | 46679 | 1 |
| SPLEN2027488 | 0.002914857 | 28869 | 1 |
| SPLEN2027780 | 0.006035663 | 28390 | 2 |
| SPLEN2027852 | 0.002914857 | 102885 | 1 |
| SPLEN2027868 | 0.002914857 | 187751 | 1 |
| SPLEN2027995 | 0.002914857 | 23054 | 1 |
| SPLEN2028046 | 0.002914857 | 180839 | 1 |
| SPLEN2028066 | 0.002914857 | 141052 | 1 |
| SPLEN2028138 | 0.002914857 | 279635 | 1 |
| SPLEN2028365 | 0.048828539 | 239696 | 2 |
| SPLEN2028417 | 0.002914857 | 121117 | 1 |
| SPLEN2028424 | 0.006010353 | 119839 | 2 |
| SPLEN2028466 | 0.405050633 | 50829 | 79 |
| SPLEN2028593 | 0.002914857 | 215986 | 1 |
| SPLEN2028844 | 0.002914857 | 169538 | 1 |
| SPLEN2028914 | 0.060004578 | 56160 | 12 |
| SPLEN2028994 | 0.002914857 | 65100 | 1 |
| SPLEN2029051 | 0.002914857 | 224759 | 1 |
| SPLEN2029176 | 0.002914857 | 200061 | 1 |
| SPLEN2029295 | 0.004791699 | 189924 | 2 |
| SPLEN2029380 | 0.002914857 | 113901 | 1 |
| SPLEN2029522 | 0.002914857 | 180805 | 1 |
| SPLEN2029683 | 0.004791699 | 156689 | 2 |
| SPLEN2029727 | 0.005829714 | 210646 | 2 |
| SPLEN2029904 | 0.005829714 | 236507 | 2 |
| SPLEN2029912 | 0.102573432 | 134449 | 11 |
| SPLEN2030250 | 0.002914857 | 199513 | 1 |
| SPLEN2030335 | 0.002914857 | 74114 | 1 |
| SPLEN2030371 | 0.002914857 | 176014 | 1 |
| SPLEN2030397 | 0.002914857 | 243151 | 1 |
| SPLEN2030479 | 0.002914857 | 235231 | 1 |
| SPLEN2030495 | 0.002914857 | 97300 | 1 |
| SPLEN2030562 | 0.002914857 | 124194 | 1 |
| SPLEN2030736 | 0.009170813 | 106930 | 4 |
| SPLEN2030763 | 0.015910477 | 23741 | 3 |
| SPLEN2030847 | 0.002914857 | 149643 | 1 |
| SPLEN2031004 | 0.002914857 | 210166 | 1 |
| SPLEN2031125 | 0.002914857 | 243143 | 1 |
| SPLEN2031424 | 0.022004007 | 42729 | 7 |
| SPLEN2031547 | 0.106422675 | 86211 | 46 |
| SPLEN2031674 | 0.007567282 | 38472 | 4 |
| SPLEN2031724 | 0.002914857 | 139903 | 1 |
| SPLEN2031780 | 0.002914857 | 75151 | 1 |
| SPLEN2032036 | 0.002914857 | 233320 | 1 |
| SPLEN2032112 | 0.002914857 | 30137 | 1 |
| SPLEN2032154 | 0.004772971 | 30714 | 2 |
| SPLEN2032157 | 0.029322393 | 138209 | 5 |
| SPLEN2032321 | 0.002914857 | 168234 | 1 |
| SPLEN2032356 | 0.008720035 | 201270 | 2 |
| SPLEN2032677 | 0.005829714 | 149572 | 2 |
| SPLEN2032813 | 0.002914857 | 207006 | 1 |
| SPLEN2032924 | 0.002914857 | 264674 | 1 |
| SPLEN2033098 | 0.033671568 | 121865 | 10 |
| SPLEN2033153 | 0.002914857 | 238918 | 1 |
| SPLEN2033333 | 0.002914857 | 184481 | 1 |
| SPLEN2033490 | 0.002914857 | 163929 | 1 |
| SPLEN2033539 | 0.002914857 | 276312 | 1 |
| SPLEN2033921 | 0.002914857 | 254031 | 1 |
| SPLEN2033996 | 0.005829714 | 139283 | 2 |
| SPLEN2034021 | 0.002914857 | 198083 | 1 |
| SPLEN2034081 | 0.002914857 | 66499 | 1 |
| SPLEN2034252 | 0.002914857 | 168153 | 1 |
| SPLEN2034448 | 0.032316465 | 73005 | 12 |
| SPLEN2034551 | 0.002914857 | 266745 | 1 |
| SPLEN2034601 | 0.002914857 | 283931 | 1 |
| SPLEN2034678 | 0.002914857 | 222395 | 1 |
| SPLEN2034781 | 0.005987559 | 187945 | 3 |
| SPLEN2034864 | 0.002914857 | 220768 | 1 |
| SPLEN2034934 | 0.002914857 | 73802 | 1 |
| SPLEN2035318 | 0.002914857 | 75172 | 1 |
| SPLEN2035615 | 0.002914857 | 252961 | 1 |
| SPLEN2036076 | 0.101992709 | 98489 | 25 |
| SPLEN2036103 | 0.002914857 | 241156 | 1 |
| SPLEN2036326 | 0.002914857 | 216122 | 1 |
| SPLEN2036475 | 0.002914857 | 135081 | 1 |
| SPLEN2036501 | 0.004312012 | 153304 | 2 |
| SPLEN2036579 | 0.002914857 | 200967 | 1 |
| SPLEN2036608 | 0.002914857 | 198676 | 1 |
| SPLEN2036702 | 0.005829714 | 175948 | 2 |
| SPLEN2036712 | 0.002914857 | 163537 | 1 |
| SPLEN2036821 | 0.004772971 | 195486 | 2 |
| SPLEN2036932 | 0.057151022 | 92903 | 16 |
| SPLEN2036953 | 0.002914857 | 253831 | 1 |
| SPLEN2037077 | 0.005829714 | 235817 | 2 |
| SPLEN2037194 | 0.004104101 | 192686 | 2 |
| SPLEN2037319 | 0.002914857 | 254821 | 1 |
| SPLEN2037444 | 0.002914857 | 251799 | 1 |
| SPLEN2037580 | 0.002914857 | 141311 | 1 |
| SPLEN2037630 | 0.002914857 | 251678 | 1 |
| SPLEN2037678 | 0.002914857 | 89396 | 1 |
| SPLEN2037722 | 0.10169338 | 103496 | 11 |
| SPLEN2037891 | 0.002914857 | 232681 | 1 |
| SPLEN2038055 | 0.002914857 | 153266 | 1 |
| SPLEN2038180 | 0.002914857 | 228882 | 1 |
| SPLEN2038345 | 0.005698412 | 127873 | 3 |
| SPLEN2038407 | 0.002914857 | 131919 | 1 |
| SPLEN2038473 | 0.012181189 | 221740 | 3 |
| SPLEN2038834 | 0.036169319 | 115808 | 6 |
| SPLEN2039311 | 0.002914857 | 224424 | 1 |
| SPLEN2039379 | 0.057855456 | 70977 | 29 |
| SPLEN2039672 | 0.004312012 | 263345 | 2 |
| SPLEN2039697 | 0.002914857 | 228251 | 1 |
| SPLEN2039845 | 0.005829714 | 53204 | 2 |
| SPLEN2039936 | 0.002914857 | 162533 | 1 |
| SPLEN2040222 | 0.002914857 | 232621 | 1 |
| SPLEN2040237 | 0.004104101 | 234149 | 2 |
| SPLEN2040824 | 0.008124066 | 121828 | 4 |
| SPLEN2041304 | 0.002914857 | 232842 | 1 |
| SPLEN2041306 | 0.021755328 | 125816 | 10 |
| SPLEN2041310 | 0.002914857 | 105698 | 1 |
| SPLEN2041585 | 0.004913258 | 227340 | 2 |
| SPLEN2041645 | 0.002914857 | 256976 | 1 |
| SPLEN2041700 | 0.002914857 | 249112 | 1 |
| SPLEN2041720 | 0.002914857 | 221325 | 1 |
| SPLEN2041977 | 0.002914857 | 250662 | 1 |
| SPLEN2042051 | 0.002914857 | 182559 | 1 |
| SPLEN2042303 | 0.006393524 | 139498 | 3 |
| SPLEN2042521 | 0.002914857 | 169976 | 1 |
| SPLEN2042535 | 0.023534289 | 184674 | 8 |
| SPLEN2042598 | 0.002914857 | 256318 | 1 |
| SPLEN2042714 | 0.004913258 | 145619 | 2 |
| SPLEN2042920 | 0.061571038 | 84778 | 3 |
| STOMA1000013 | 0.011402509 | 31529 | 1 |
| STOMA1000047 | 0.052878615 | 53485 | 12 |
| STOMA1000052 | 0.011402509 | 49765 | 1 |
| STOMA1000074 | 0.011402509 | 60869 | 1 |
| STOMA1000091 | 0.024087821 | 77013 | 3 |
| STOMA1000117 | 0.011402509 | 11297 | 1 |
| STOMA1000133 | 0.011402509 | 56595 | 1 |
| STOMA1000175 | 0.011402509 | 21054 | 1 |
| STOMA1000186 | 0.01340091 | 156471 | 2 |
| STOMA1000189 | 0.107145355 | 69667 | 12 |
| STOMA1000190 | 0.011402509 | 8471 | 1 |
| STOMA1000191 | 0.011402509 | 65857 | 1 |
| STOMA2000032 | 0.011402509 | 115408 | 1 |
| STOMA2000055 | 0.288461073 | 97799 | 16 |
| STOMA2000072 | 0.011402509 | 266304 | 1 |
| STOMA2000088 | 0.011402509 | 171623 | 1 |
| STOMA2000104 | 0.011402509 | 147865 | 1 |
| STOMA2000121 | 0.137498035 | 3725 | 10 |
| STOMA2000148 | 0.014317366 | 236379 | 2 |
| STOMA2000168 | 0.193842645 | 19643 | 17 |
| STOMA2000183 | 0.011402509 | 269473 | 1 |
| STOMA2000188 | 0.011402509 | 128804 | 1 |
| STOMA2000257 | 0.084883416 | 64729 | 11 |
| STOMA2000279 | 0.011402509 | 54996 | 1 |
| STOMA2000289 | 0.061504591 | 98261 | 17 |
| STOMA2000383 | 0.014317366 | 260088 | 2 |
| STOMA2000386 | 0.011402509 | 104256 | 1 |
| STOMA2000395 | 0.078491497 | 28409 | 18 |
| STOMA2000396 | 0.032512699 | 141991 | 8 |
| STOMA2000478 | 0.011402509 | 119714 | 1 |
| STOMA2000482 | 0.043462414 | 152336 | 8 |
| STOMA2000529 | 0.023200526 | 220306 | 2 |
| STOMA2000539 | 0.034207526 | 191824 | 3 |
| STOMA2000567 | 0.011402509 | 267478 | 1 |
| STOMA2000640 | 0.011402509 | 249468 | 1 |
| STOMA2000678 | 0.011402509 | 89418 | 1 |
| STOMA2000686 | 0.011402509 | 101243 | 1 |
| STOMA2000888 | 0.011402509 | 117649 | 1 |
| STOMA2001025 | 0.011402509 | 94387 | 1 |
| STOMA2001389 | 0.02901198 | 193281 | 3 |
| STOMA2002052 | 0.03012564 | 137222 | 12 |
| STOMA2002141 | 0.011402509 | 232751 | 1 |
| STOMA2002166 | 0.286402306 | 49862 | 24 |
| STOMA2002367 | 0.011402509 | 60293 | 1 |
| STOMA2002688 | 0.011402509 | 51423 | 1 |
| STOMA2002828 | 0.03057301 | 149462 | 5 |
| STOMA2003158 | 0.027373949 | 109513 | 7 |
| STOMA2003289 | 0.011402509 | 86576 | 1 |
| STOMA2003444 | 0.012497787 | 215865 | 2 |
| STOMA2003477 | 0.022805017 | 189289 | 2 |
| STOMA2003646 | 0.011402509 | 234771 | 1 |
| STOMA2003716 | 0.014676506 | 7982 | 3 |
| STOMA2003781 | 0.01327935 | 215881 | 2 |
| STOMA2003894 | 0.011402509 | 195946 | 1 |
| STOMA2004194 | 0.011402509 | 220491 | 1 |
| STOMA2004294 | 0.011402509 | 229373 | 1 |
| STOMA2004663 | 0.023996967 | 194702 | 2 |
| STOMA2004668 | 0.011402509 | 99406 | 1 |
| STOMA2004852 | 0.011402509 | 253081 | 1 |
| STOMA2004873 | 0.011402509 | 202344 | 1 |
| STOMA2004884 | 0.011402509 | 176071 | 1 |
| STOMA2004893 | 0.023189402 | 260132 | 2 |
| STOMA2004925 | 0.01340091 | 205048 | 2 |
| STOMA2005120 | 0.013260623 | 220451 | 2 |
| STOMA2005664 | 0.011402509 | 280102 | 1 |
| STOMA2005667 | 0.011402509 | 273810 | 1 |
| STOMA2005746 | 0.011402509 | 16970 | 1 |
| STOMA2005782 | 0.012497787 | 79442 | 2 |
| STOMA2006181 | 0.011402509 | 148331 | 1 |
| STOMA2006213 | 0.011402509 | 206391 | 1 |
| STOMA2006229 | 0.027870128 | 149573 | 4 |
| STOMA2006325 | 0.011402509 | 227761 | 1 |
| STOMA2006330 | 0.011402509 | 236113 | 1 |
| STOMA2006398 | 0.011402509 | 240615 | 1 |
| STOMA2006447 | 0.022805017 | 184625 | 2 |
| STOMA2006780 | 0.019289199 | 189336 | 3 |
| STOMA2006904 | 0.011402509 | 199047 | 1 |
| STOMA2007269 | 0.011402509 | 20645 | 1 |
| STOMA2007680 | 0.011402509 | 221555 | 1 |
| STOMA2007745 | 0.011402509 | 282203 | 1 |
| STOMA2008050 | 0.011402509 | 122137 | 1 |
| STOMA2008361 | 0.011402509 | 270286 | 1 |
| STOMA2008546 | 0.011402509 | 96401 | 1 |
| STOMA2008614 | 0.011402509 | 69248 | 1 |
| STOMA2008638 | 0.022866454 | 146487 | 2 |
| STOMA2008838 | 0.011402509 | 276793 | 1 |
| STOMA2009250 | 0.011402509 | 90400 | 1 |
| STOMA2009253 | 0.011402509 | 280795 | 1 |
| STOMA2009256 | 0.011402509 | 183320 | 1 |
| STOMA2009289 | 0.029205793 | 218254 | 5 |
| SYNOV1000045 | 0.023852455 | 72118 | 4 |
| SYNOV1000049 | 0.007652175 | 65025 | 2 |
| SYNOV1000118 | 0.039591548 | 48625 | 9 |
| SYNOV1000124 | 0.301223048 | 9436 | 85 |
| SYNOV1000128 | 0.01141413 | 57783 | 3 |
| SYNOV1000164 | 0.043571914 | 59610 | 7 |
| SYNOV1000190 | 0.003613892 | 45988 | 1 |
| SYNOV1000256 | 0.003613892 | 199000 | 1 |
| SYNOV1000374 | 0.008444126 | 159157 | 4 |
| SYNOV2000152 | 0.003613892 | 237386 | 1 |
| SYNOV2000171 | 0.003613892 | 21680 | 1 |
| SYNOV2000173 | 0.003613892 | 274979 | 1 |
| SYNOV2000177 | 0.003613892 | 261581 | 1 |
| SYNOV2000178 | 0.003613892 | 64196 | 1 |
| SYNOV2000193 | 0.003613892 | 201160 | 1 |
| SYNOV2000251 | 0.003613892 | 279584 | 1 |
| SYNOV2000279 | 0.007227784 | 182793 | 2 |
| SYNOV2000291 | 0.005323352 | 144620 | 2 |
| SYNOV2000297 | 0.003613892 | 274374 | 1 |
| SYNOV2000397 | 0.007032812 | 101040 | 3 |
| SYNOV2000426 | 0.003613892 | 274010 | 1 |
| SYNOV2000601 | 0.003613892 | 236868 | 1 |
| SYNOV2000700 | 0.003613892 | 273768 | 1 |
| SYNOV2000820 | 0.004803136 | 191341 | 2 |
| SYNOV2000824 | 0.014455567 | 204721 | 4 |
| SYNOV2000923 | 0.010841675 | 189217 | 3 |
| SYNOV2001014 | 0.003613892 | 115317 | 1 |
| SYNOV2001033 | 0.279470536 | 132567 | 32 |
| SYNOV2001035 | 0.003613892 | 262977 | 1 |
| SYNOV2001088 | 0.003613892 | 278063 | 1 |
| SYNOV2001111 | 0.003613892 | 277402 | 1 |
| SYNOV2001144 | 0.118467456 | 102328 | 12 |
| SYNOV2001212 | 0.015377214 | 169597 | 2 |
| SYNOV2001239 | 0.534030075 | 67581 | 75 |
| SYNOV2001251 | 0.003613892 | 148739 | 1 |
| SYNOV2001262 | 0.080310646 | 127512 | 23 |
| SYNOV2001300 | 0.003613892 | 281048 | 1 |
| SYNOV2001356 | 0.003613892 | 275463 | 1 |
| SYNOV2001374 | 0.091479917 | 60111 | 12 |
| SYNOV2001390 | 0.003613892 | 273646 | 1 |
| SYNOV2001451 | 0.003613892 | 53495 | 1 |
| SYNOV2001457 | 0.003613892 | 43345 | 1 |
| SYNOV2001468 | 0.018359173 | 207870 | 3 |
| SYNOV2001581 | 0.063625364 | 133234 | 6 |
| SYNOV2001648 | 0.154620254 | 54138 | 11 |
| SYNOV2001660 | 0.003613892 | 274189 | 1 |
| SYNOV2001708 | 0.0561349 | 78986 | 2 |
| SYNOV2003326 | 0.003613892 | 9527 | 1 |
| SYNOV2005216 | 0.01715935 | 162569 | 3 |
| SYNOV2005448 | 0.003613892 | 90208 | 1 |
| SYNOV2005541 | 0.011703843 | 255833 | 3 |
| SYNOV2005673 | 0.003613892 | 249806 | 1 |
| SYNOV2005817 | 0.003613892 | 228847 | 1 |
| SYNOV2005962 | 0.003613892 | 75217 | 1 |
| SYNOV2006430 | 0.003613892 | 201271 | 1 |
| SYNOV2006620 | 0.005472006 | 32163 | 2 |
| SYNOV2007758 | 0.008563436 | 131445 | 4 |
| SYNOV2007965 | 0.003613892 | 136221 | 1 |
| SYNOV2008765 | 0.116099831 | 133235 | 2 |
| SYNOV2009172 | 0.321985384 | 124046 | 21 |
| SYNOV2011233 | 0.003613892 | 69147 | 1 |
| SYNOV2012326 | 0.003613892 | 101093 | 1 |
| SYNOV2013365 | 0.003613892 | 156832 | 1 |
| SYNOV2013637 | 0.003613892 | 197633 | 1 |
| SYNOV2014157 | 0.00470917 | 53366 | 2 |
| SYNOV2014364 | 0.003613892 | 183179 | 1 |
| SYNOV2014400 | 0.012911511 | 98799 | 4 |
| SYNOV2016124 | 0.003613892 | 190834 | 1 |
| SYNOV2016837 | 0.003613892 | 73068 | 1 |
| SYNOV2017055 | 0.009924305 | 151032 | 4 |
| SYNOV2017179 | 0.003613892 | 46364 | 1 |
| SYNOV2018921 | 0.003613892 | 214857 | 1 |
| SYNOV2019280 | 0.003613892 | 245355 | 1 |
| SYNOV2020085 | 0.003613892 | 138410 | 1 |
| SYNOV2020463 | 0.038239931 | 182997 | 2 |
| SYNOV2021320 | 0.003613892 | 211037 | 1 |
| SYNOV2021953 | 0.017507311 | 95051 | 9 |
| SYNOV3000184 | 0.003613892 | 279711 | 1 |
| SYNOV3000231 | 0.003613892 | 271871 | 1 |
| SYNOV3000302 | 0.007227784 | 200491 | 2 |
| SYNOV3000345 | 0.003613892 | 285038 | 1 |
| SYNOV4000249 | 0.007032812 | 201872 | 3 |
| SYNOV4000472 | 0.003613892 | 128609 | 1 |
| SYNOV4000598 | 0.005612293 | 71068 | 2 |
| SYNOV4000706 | 0.003613892 | 272086 | 1 |
| SYNOV4001153 | 0.072241522 | 114007 | 34 |
| SYNOV4001224 | 0.005612293 | 234263 | 2 |
| SYNOV4001326 | 0.005490733 | 226248 | 2 |
| SYNOV4001342 | 0.003613892 | 260053 | 1 |
| SYNOV4001395 | 0.009226185 | 266015 | 3 |
| SYNOV4002001 | 0.003613892 | 118215 | 1 |
| SYNOV4002346 | 0.027938227 | 19967 | 7 |
| SYNOV4002392 | 0.003613892 | 159677 | 1 |
| SYNOV4002744 | 0.082954585 | 126331 | 13 |
| SYNOV4002875 | 0.003613892 | 257134 | 1 |
| SYNOV4002883 | 0.006998899 | 65707 | 3 |
| SYNOV4003174 | 0.003613892 | 282533 | 1 |
| SYNOV4003322 | 0.003613892 | 252421 | 1 |
| SYNOV4003681 | 0.003613892 | 272846 | 1 |
| SYNOV4003981 | 0.003613892 | 64201 | 1 |
| SYNOV4004184 | 0.003613892 | 130099 | 1 |
| SYNOV4004210 | 0.177397925 | 117098 | 12 |
| SYNOV4004741 | 0.003613892 | 258543 | 1 |
| SYNOV4004823 | 0.007367575 | 137113 | 3 |
| SYNOV4004914 | 0.003613892 | 266928 | 1 |
| SYNOV4005256 | 0.00470917 | 32026 | 2 |
| SYNOV4005570 | 0.003613892 | 255282 | 1 |
| SYNOV4005739 | 0.003613892 | 252713 | 1 |
| SYNOV4005889 | 0.003613892 | 212673 | 1 |
| SYNOV4005981 | 0.003613892 | 245064 | 1 |
| SYNOV4005989 | 0.008117663 | 119949 | 4 |
| SYNOV4006256 | 0.003613892 | 251201 | 1 |
| SYNOV4006327 | 0.010840761 | 178438 | 4 |
| SYNOV4006542 | 0.045197226 | 86110 | 14 |
| SYNOV4007012 | 0.005011047 | 12308 | 2 |
| SYNOV4007215 | 0.003613892 | 260443 | 1 |
| SYNOV4007360 | 0.003613892 | 67035 | 1 |
| SYNOV4007430 | 0.005289439 | 199727 | 2 |
| SYNOV4007521 | 0.003613892 | 258386 | 1 |
| SYNOV4007553 | 0.005011047 | 127986 | 2 |
| SYNOV4007671 | 0.228572571 | 42872 | 29 |
| SYNOV4007711 | 0.003613892 | 231528 | 1 |
| SYNOV4008245 | 0.018136331 | 157123 | 10 |
| SYNOV4008336 | 0.003613892 | 244623 | 1 |
| SYNOV4008440 | 0.113584757 | 111121 | 28 |
| SYNOV4009129 | 0.021582962 | 137399 | 7 |
| SYNOV4009139 | 0.007227784 | 75216 | 2 |
| SYNOV4009295 | 0.015092343 | 162030 | 6 |
| SYNOV4009298 | 0.005695404 | 78315 | 2 |
| SYNOV4009575 | 0.034970012 | 95138 | 7 |
| T1ESE2000116 | 0.038613382 | 149178 | 2 |
| T1ESE2000609 | 0.073717682 | 34812 | 6 |
| T1ESE2000904 | 0.072576727 | 51440 | 9 |
| T1ESE2002665 | 0.040198185 | 189691 | 2 |
| TBAES2000004 | 0.011786893 | 205550 | 1 |
| TBAES2000059 | 0.111195391 | 152895 | 12 |
| TBAES2000116 | 0.014768852 | 155332 | 2 |
| TBAES2000255 | 0.011786893 | 64951 | 1 |
| TBAES2000315 | 0.023573786 | 153609 | 2 |
| TBAES2000352 | 0.023550215 | 84454 | 2 |
| TBAES2000377 | 0.131115217 | 119962 | 31 |
| TBAES2000932 | 0.011786893 | 252259 | 1 |
| TBAES2001171 | 0.011786893 | 63779 | 1 |
| TBAES2001220 | 0.011786893 | 110967 | 1 |
| TBAES2001229 | 0.011786893 | 237837 | 1 |
| TBAES2001258 | 0.017628356 | 186006 | 2 |
| TBAES2001296 | 0.014872325 | 227185 | 3 |
| TBAES2001492 | 0.011786893 | 169107 | 1 |
| TBAES2001553 | 0.056963783 | 176602 | 4 |
| TBAES2001751 | 0.011786893 | 256602 | 1 |
| TBAES2002197 | 0.021616218 | 126438 | 6 |
| TBAES2003435 | 0.011786893 | 284396 | 1 |
| TBAES2003492 | 0.011786893 | 144056 | 1 |
| TBAES2003550 | 0.011786893 | 267845 | 1 |
| TBAES2003702 | 0.013785294 | 135132 | 2 |
| TBAES2003917 | 0.011786893 | 282672 | 1 |
| TBAES2004055 | 0.011786893 | 271472 | 1 |
| TBAES2004105 | 0.011786893 | 283208 | 1 |
| TBAES2004939 | 0.011786893 | 211922 | 1 |
| TBAES2005157 | 0.011786893 | 123231 | 1 |
| TBAES2005361 | 0.011786893 | 247067 | 1 |
| TBAES2005543 | 0.011786893 | 249725 | 1 |
| TBAES2006568 | 0.011786893 | 245633 | 1 |
| TBAES2006881 | 0.011786893 | 226830 | 1 |
| TBAES2007379 | 0.011786893 | 150529 | 1 |
| TBAES2007428 | 0.031196233 | 8072 | 4 |
| TBAES2007481 | 0.011786893 | 282907 | 1 |
| TBAES2007504 | 0.011786893 | 182933 | 1 |
| TBAES2007548 | 0.011786893 | 169159 | 1 |
| TBAES2007697 | 0.011786893 | 261708 | 1 |
| TBAES2007862 | 0.011786893 | 230864 | 1 |
| TBAES2007964 | 0.023573786 | 259523 | 2 |
| TBAES2008133 | 0.011786893 | 139277 | 1 |
| TBAES2009387 | 0.313458544 | 7997 | 19 |
| TCERX2000171 | 0.035360679 | 140246 | 1 |
| TCERX2000431 | 0.079764438 | 63317 | 7 |
| TCERX2000613 | 0.041355883 | 199690 | 4 |
| TCERX2000998 | 0.035360679 | 281225 | 1 |
| TCERX2002644 | 0.048835849 | 216299 | 3 |
| TCOLN1000037 | 0.035523979 | 41662 | 1 |
| TCOLN1000180 | 0.068438917 | 153138 | 9 |
| TCOLN2000139 | 0.035523979 | 183290 | 1 |
| TCOLN2000236 | 0.035523979 | 30977 | 1 |
| TCOLN2000641 | 0.035523979 | 240028 | 1 |
| TCOLN2001268 | 0.035523979 | 221266 | 1 |
| TCOLN2002047 | 0.035523979 | 278678 | 1 |
| TCOLN2002278 | 0.035523979 | 259462 | 1 |
| TESOP1000035 | 0.037883681 | 208246 | 6 |
| TESOP1000127 | 0.017609472 | 237326 | 2 |
| TESOP1000160 | 0.011463946 | 101000 | 1 |
| TESOP2000083 | 0.011463946 | 184794 | 1 |
| TESOP2000090 | 0.011463946 | 203787 | 1 |
| TESOP2000205 | 0.011463946 | 191566 | 1 |
| TESOP2000312 | 0.034688447 | 41756 | 3 |
| TESOP2000390 | 0.011463946 | 141251 | 1 |
| TESOP2000400 | 0.011463946 | 155861 | 1 |
| TESOP2000510 | 0.011463946 | 42442 | 1 |
| TESOP2000527 | 0.016236917 | 134400 | 3 |
| TESOP2000569 | 0.011463946 | 72611 | 1 |
| TESOP2000739 | 0.011463946 | 261142 | 1 |
| TESOP2000801 | 0.011463946 | 254808 | 1 |
| TESOP2001122 | 0.011463946 | 276585 | 1 |
| TESOP2001166 | 0.011463946 | 204471 | 1 |
| TESOP2001345 | 0.011463946 | 198263 | 1 |
| TESOP2001474 | 0.011463946 | 159221 | 1 |
| TESOP2001605 | 0.01715935 | 162569 | 3 |
| TESOP2001796 | 0.011463946 | 129621 | 1 |
| TESOP2001818 | 0.011463946 | 146677 | 1 |
| TESOP2001849 | 0.011463946 | 189347 | 1 |
| TESOP2001865 | 0.011463946 | 179907 | 1 |
| TESOP2001901 | 0.011463946 | 199053 | 1 |
| TESOP2001953 | 0.012861101 | 268307 | 2 |
| TESOP2002005 | 0.011463946 | 150179 | 1 |
| TESOP2002110 | 0.011463946 | 138420 | 1 |
| TESOP2002150 | 0.011463946 | 272460 | 1 |
| TESOP2002273 | 0.041282319 | 106573 | 12 |
| TESOP2002451 | 0.070540928 | 79461 | 9 |
| TESOP2002489 | 0.011463946 | 135765 | 1 |
| TESOP2002539 | 0.011463946 | 204319 | 1 |
| TESOP2002545 | 0.011463946 | 170351 | 1 |
| TESOP2002804 | 0.011463946 | 190817 | 1 |
| TESOP2002950 | 0.01265319 | 280191 | 2 |
| TESOP2003273 | 0.011463946 | 250570 | 1 |
| TESOP2003308 | 0.011463946 | 162658 | 1 |
| TESOP2003553 | 0.011463946 | 190809 | 1 |
| TESOP2003753 | 0.011463946 | 261893 | 1 |
| TESOP2004110 | 0.011463946 | 279536 | 1 |
| TESOP2004114 | 0.011463946 | 271486 | 1 |
| TESOP2004173 | 0.011463946 | 29836 | 1 |
| TESOP2004214 | 0.011463946 | 249179 | 1 |
| TESOP2005199 | 0.011463946 | 169453 | 1 |
| TESOP2005285 | 0.011463946 | 140935 | 1 |
| TESOP2005485 | 0.011463946 | 183895 | 1 |
| TESOP2005498 | 0.011463946 | 170689 | 1 |
| TESOP2005579 | 0.011463946 | 211732 | 1 |
| TESOP2006041 | 0.011463946 | 102411 | 1 |
| TESOP2006053 | 0.022750628 | 196762 | 2 |
| TESOP2006060 | 0.011463946 | 268697 | 1 |
| TESOP2006068 | 0.011463946 | 272778 | 1 |
| TESOP2006110 | 0.011463946 | 267163 | 1 |
| TESOP2006398 | 0.011463946 | 170614 | 1 |
| TESOP2006670 | 0.014378803 | 93179 | 2 |
| TESOP2006704 | 0.011463946 | 280142 | 1 |
| TESOP2006746 | 0.011463946 | 264503 | 1 |
| TESOP2006865 | 0.011463946 | 134378 | 1 |
| TESOP2006893 | 0.019503913 | 39542 | 3 |
| TESOP2007041 | 0.011463946 | 200517 | 1 |
| TESOP2007052 | 0.022927892 | 211923 | 2 |
| TESOP2007262 | 0.011463946 | 194313 | 1 |
| TESOP2007384 | 0.083471878 | 63178 | 16 |
| TESOP2007636 | 0.011463946 | 239209 | 1 |
| TESOP2007674 | 0.011463946 | 205967 | 1 |
| TESOP2007688 | 0.011463946 | 248963 | 1 |
| TESOP2007725 | 0.011463946 | 73757 | 1 |
| TESOP2007912 | 0.014559442 | 151738 | 2 |
| TESOP2007978 | 0.011463946 | 265637 | 1 |
| TESOP2008127 | 0.011463946 | 191304 | 1 |
| TESOP2008552 | 0.011463946 | 204602 | 1 |
| TESOP2008556 | 0.011463946 | 252327 | 1 |
| TESOP2008563 | 0.011463946 | 272291 | 1 |
| TESOP2008820 | 0.017427864 | 88487 | 3 |
| TESOP2009121 | 0.011463946 | 251601 | 1 |
| TESOP2009201 | 0.011463946 | 160481 | 1 |
| TESOP2009247 | 0.011463946 | 175343 | 1 |
| TESOP2009555 | 0.011463946 | 102879 | 1 |
| TESTI1000018 | 0.033953626 | 21608 | 31 |
| TESTI1000019 | 0.00657167 | 62850 | 6 |
| TESTI1000023 | 0.199795094 | 29350 | 74 |
| TESTI1000025 | 0.005476391 | 73890 | 5 |
| TESTI1000029 | 0.001095278 | 43508 | 1 |
| TESTI1000030 | 0.001095278 | 69542 | 1 |
| TESTI1000042 | 0.016429174 | 68868 | 15 |
| TESTI1000045 | 0.001095278 | 47390 | 1 |
| TESTI1000051 | 0.032173191 | 65277 | 19 |
| TESTI1000055 | 0.005476391 | 74971 | 5 |
| TESTI1000064 | 0.034419358 | 1169 | 27 |
| TESTI1000070 | 0.046405709 | 66041 | 18 |
| TESTI1000085 | 0.002190557 | 68432 | 2 |
| TESTI1000088 | 0.001095278 | 48565 | 1 |
| TESTI1000093 | 0.001095278 | 17846 | 1 |
| TESTI1000094 | 0.016987423 | 77385 | 13 |
| TESTI1000096 | 0.009578075 | 61300 | 5 |
| TESTI1000101 | 0.039118604 | 68646 | 12 |
| TESTI1000109 | 0.001095278 | 34647 | 1 |
| TESTI1000125 | 0.001095278 | 73674 | 1 |
| TESTI1000127 | 0.01861973 | 57463 | 17 |
| TESTI1000128 | 0.002190557 | 27470 | 2 |
| TESTI1000131 | 0.085431704 | 60885 | 78 |
| TESTI1000138 | 0.00561324 | 78320 | 3 |
| TESTI1000142 | 0.024833461 | 69994 | 12 |
| TESTI1000157 | 0.001095278 | 76581 | 1 |
| TESTI1000163 | 0.040663925 | 75196 | 6 |
| TESTI1000168 | 0.053467984 | 46857 | 34 |
| TESTI1000169 | 0.003285835 | 59884 | 3 |
| TESTI1000179 | 0.001095278 | 72121 | 1 |
| TESTI1000183 | 0.001095278 | 73977 | 1 |
| TESTI1000191 | 0.044906408 | 45346 | 41 |
| TESTI1000257 | 0.003285835 | 12790 | 3 |
| TESTI1000266 | 0.00317679 | 197797 | 2 |
| TESTI1000319 | 0.001095278 | 204088 | 1 |
| TESTI1000330 | 0.002284523 | 52657 | 2 |
| TESTI1000348 | 0.001095278 | 152904 | 1 |
| TESTI1000390 | 0.001095278 | 204385 | 1 |
| TESTI1000391 | 0.001095278 | 259869 | 1 |
| TESTI1000459 | 0.001095278 | 281932 | 1 |
| TESTI1000491 | 0.001095278 | 260885 | 1 |
| TESTI1000545 | 0.012048061 | 170428 | 11 |
| TESTI2000006 | 0.016945006 | 32201 | 4 |
| TESTI2000018 | 0.001095278 | 239006 | 1 |
| TESTI2000044 | 0.001095278 | 191090 | 1 |
| TESTI2000100 | 0.008648766 | 98057 | 4 |
| TESTI2000117 | 0.00657167 | 158524 | 6 |
| TESTI2000120 | 0.001095278 | 7353 | 1 |
| TESTI2000147 | 0.001095278 | 203514 | 1 |
| TESTI2000154 | 0.00657167 | 150651 | 6 |
| TESTI2000172 | 0.019586134 | 104323 | 4 |
| TESTI2000177 | 0.006872137 | 91074 | 5 |
| TESTI2000179 | 0.004573946 | 188585 | 3 |
| TESTI2000184 | 0.148226826 | 28573 | 63 |
| TESTI2000207 | 0.001095278 | 142535 | 1 |
| TESTI2000208 | 0.001095278 | 134081 | 1 |
| TESTI2000221 | 0.001095278 | 215078 | 1 |
| TESTI2000238 | 0.005286053 | 53584 | 3 |
| TESTI2000253 | 0.037525172 | 79122 | 22 |
| TESTI2000272 | 0.017304703 | 122737 | 15 |
| TESTI2000278 | 0.001095278 | 188028 | 1 |
| TESTI2000349 | 0.007037402 | 87817 | 2 |
| TESTI2000356 | 0.009555137 | 150284 | 5 |
| TESTI2000358 | 0.001095278 | 9248 | 1 |
| TESTI2000372 | 0.001095278 | 276618 | 1 |
| TESTI2000427 | 0.001095278 | 271140 | 1 |
| TESTI2000431 | 0.005162676 | 131959 | 4 |
| TESTI2000435 | 0.008712949 | 20365 | 3 |
| TESTI2000443 | 0.001095278 | 243255 | 1 |
| TESTI2000452 | 0.001095278 | 183847 | 1 |
| TESTI2000462 | 0.002190557 | 221090 | 2 |
| TESTI2000489 | 0.010422679 | 105330 | 5 |
| TESTI2000520 | 0.044537665 | 150243 | 4 |
| TESTI2000571 | 0.001095278 | 211661 | 1 |
| TESTI2000591 | 0.091754732 | 130695 | 7 |
| TESTI2000598 | 0.033356304 | 143288 | 18 |
| TESTI2000600 | 0.004514199 | 83453 | 3 |
| TESTI2000616 | 0.001095278 | 269203 | 1 |
| TESTI2000627 | 0.007952337 | 130201 | 5 |
| TESTI2000644 | 0.001095278 | 130461 | 1 |
| TESTI2000671 | 0.008762226 | 191413 | 8 |
| TESTI2000683 | 0.004381113 | 136399 | 4 |
| TESTI2000689 | 0.002190557 | 204157 | 2 |
| TESTI2000695 | 0.047096965 | 104901 | 43 |
| TESTI2000699 | 0.017524452 | 102062 | 16 |
| TESTI2000707 | 0.007666948 | 227032 | 7 |
| TESTI2000745 | 0.001095278 | 258623 | 1 |
| TESTI2000756 | 0.001095278 | 207786 | 1 |
| TESTI2000762 | 0.002190557 | 115095 | 2 |
| TESTI2000784 | 0.073727461 | 72398 | 37 |
| TESTI2000808 | 0.007666948 | 139042 | 7 |
| TESTI2000819 | 0.001095278 | 224281 | 1 |
| TESTI2000830 | 0.009857504 | 120679 | 9 |
| TESTI2000840 | 0.001095278 | 196880 | 1 |
| TESTI2000849 | 0.006239227 | 166525 | 5 |
| TESTI2000850 | 0.001095278 | 240625 | 1 |
| TESTI2000874 | 0.013404019 | 40488 | 3 |
| TESTI2000883 | 0.022980289 | 35239 | 7 |
| TESTI2000909 | 0.012855833 | 144727 | 2 |
| TESTI2000951 | 0.001095278 | 179635 | 1 |
| TESTI2000970 | 0.034469079 | 161949 | 10 |
| TESTI2000981 | 0.002190557 | 232411 | 2 |
| TESTI2001008 | 0.001095278 | 104151 | 1 |
| TESTI2001049 | 0.001095278 | 153511 | 1 |
| TESTI2001082 | 0.001095278 | 228216 | 1 |
| TESTI2001099 | 0.341930539 | 32316 | 112 |
| TESTI2001120 | 0.173309652 | 93764 | 6 |
| TESTI2001134 | 0.001095278 | 94768 | 1 |
| TESTI2001141 | 0.017744117 | 119327 | 11 |
| TESTI2001153 | 0.42079305 | 57205 | 61 |
| TESTI2001180 | 0.001095278 | 237087 | 1 |
| TESTI2001208 | 0.019715009 | 146601 | 18 |
| TESTI2001229 | 0.001095278 | 173732 | 1 |
| TESTI2001236 | 0.001095278 | 216980 | 1 |
| TESTI2001237 | 0.001095278 | 114472 | 1 |
| TESTI2001269 | 0.306947036 | 91948 | 49 |
| TESTI2001306 | 0.001095278 | 191329 | 1 |
| TESTI2001313 | 0.002190557 | 63848 | 2 |
| TESTI2001330 | 0.001095278 | 22189 | 1 |
| TESTI2001339 | 0.004010135 | 45797 | 2 |
| TESTI2001345 | 0.001095278 | 112645 | 1 |
| TESTI2001352 | 0.001095278 | 145355 | 1 |
| TESTI2001364 | 0.002190557 | 148748 | 2 |
| TESTI2001412 | 0.007666948 | 158175 | 7 |
| TESTI2001420 | 0.00309368 | 203893 | 2 |
| TESTI2001498 | 0.001095278 | 150988 | 1 |
| TESTI2001511 | 0.09577017 | 166656 | 7 |
| TESTI2001512 | 0.001095278 | 49259 | 1 |
| TESTI2001518 | 0.001095278 | 211570 | 1 |
| TESTI2001556 | 0.001095278 | 111905 | 1 |
| TESTI2001593 | 0.001095278 | 255653 | 1 |
| TESTI2001613 | 0.012265241 | 176173 | 8 |
| TESTI2001621 | 0.002190557 | 234501 | 2 |
| TESTI2001661 | 0.006080146 | 86267 | 5 |
| TESTI2001665 | 0.001095278 | 139587 | 1 |
| TESTI2001671 | 0.001095278 | 115851 | 1 |
| TESTI2001697 | 0.033953626 | 130424 | 31 |
| TESTI2001725 | 0.002190557 | 164747 | 2 |
| TESTI2001758 | 0.001095278 | 192679 | 1 |
| TESTI2001766 | 0.00657167 | 111205 | 6 |
| TESTI2001792 | 0.001095278 | 254726 | 1 |
| TESTI2001793 | 0.001095278 | 247116 | 1 |
| TESTI2001795 | 0.002190557 | 179263 | 2 |
| TESTI2001812 | 0.001095278 | 222469 | 1 |
| TESTI2001815 | 0.00470917 | 123988 | 2 |
| TESTI2001823 | 0.001095278 | 285083 | 1 |
| TESTI2001826 | 0.014975336 | 135124 | 9 |
| TESTI2001827 | 0.001095278 | 108934 | 1 |
| TESTI2001829 | 0.197326509 | 99858 | 14 |
| TESTI2001852 | 0.004381113 | 86844 | 4 |
| TESTI2001862 | 0.063367423 | 149372 | 13 |
| TESTI2001869 | 0.007020874 | 144206 | 3 |
| TESTI2001879 | 0.00297212 | 103983 | 2 |
| TESTI2001894 | 0.002190557 | 175287 | 2 |
| TESTI2001898 | 0.002804738 | 161475 | 2 |
| TESTI2001915 | 0.001095278 | 40832 | 1 |
| TESTI2001950 | 0.00657167 | 139425 | 6 |
| TESTI2001959 | 0.001095278 | 138129 | 1 |
| TESTI2001968 | 0.001095278 | 121250 | 1 |
| TESTI2001985 | 0.028543138 | 32388 | 20 |
| TESTI2001991 | 0.001095278 | 157090 | 1 |
| TESTI2002002 | 0.001095278 | 159843 | 1 |
| TESTI2002012 | 0.001095278 | 184577 | 1 |
| TESTI2002036 | 0.001095278 | 51002 | 1 |
| TESTI2002048 | 0.001095278 | 196931 | 1 |
| TESTI2002057 | 0.005476391 | 175695 | 5 |
| TESTI2002081 | 0.001095278 | 112556 | 1 |
| TESTI2002090 | 0.003285835 | 125929 | 3 |
| TESTI2002105 | 0.012829624 | 87249 | 11 |
| TESTI2002149 | 0.100180728 | 4257 | 10 |
| TESTI2002216 | 0.001095278 | 275644 | 1 |
| TESTI2002223 | 0.006092926 | 114142 | 3 |
| TESTI2002245 | 0.001095278 | 261477 | 1 |
| TESTI2002251 | 0.004381113 | 48549 | 4 |
| TESTI2002256 | 0.004830234 | 226156 | 3 |
| TESTI2002264 | 0.001095278 | 129428 | 1 |
| TESTI2002282 | 0.003285835 | 47089 | 3 |
| TESTI2002293 | 0.003285835 | 32392 | 3 |
| TESTI2002294 | 0.001095278 | 5586 | 1 |
| TESTI2002326 | 0.001095278 | 209610 | 1 |
| TESTI2002351 | 0.001095278 | 232614 | 1 |
| TESTI2002361 | 0.001095278 | 209625 | 1 |
| TESTI2002365 | 0.153442253 | 58041 | 42 |
| TESTI2002369 | 0.011150563 | 121018 | 5 |
| TESTI2002406 | 0.001095278 | 247540 | 1 |
| TESTI2002415 | 0.001095278 | 273017 | 1 |
| TESTI2002423 | 0.001095278 | 62854 | 1 |
| TESTI2002453 | 0.001095278 | 232542 | 1 |
| TESTI2002461 | 0.001095278 | 116441 | 1 |
| TESTI2002465 | 0.028477235 | 149181 | 26 |
| TESTI2002467 | 0.007136844 | 123820 | 2 |
| TESTI2002498 | 0.004381113 | 87416 | 4 |
| TESTI2002516 | 0.001095278 | 234712 | 1 |
| TESTI2002544 | 0.001095278 | 122820 | 1 |
| TESTI2002578 | 0.001095278 | 272292 | 1 |
| TESTI2002580 | 0.001095278 | 233245 | 1 |
| TESTI2002618 | 0.005476391 | 171396 | 5 |
| TESTI2002632 | 0.015475564 | 49878 | 4 |
| TESTI2002676 | 0.004381113 | 135276 | 4 |
| TESTI2002690 | 0.001095278 | 171132 | 1 |
| TESTI2002696 | 0.012901654 | 175601 | 2 |
| TESTI2002698 | 0.001095278 | 90215 | 1 |
| TESTI2002700 | 0.002190557 | 80273 | 2 |
| TESTI2002707 | 0.101125978 | 126296 | 41 |
| TESTI2002724 | 0.001095278 | 252087 | 1 |
| TESTI2002729 | 0.005476391 | 123176 | 5 |
| TESTI2002752 | 0.001095278 | 269762 | 1 |
| TESTI2002753 | 0.001095278 | 169585 | 1 |
| TESTI2002789 | 0.001095278 | 92078 | 1 |
| TESTI2002802 | 0.002190557 | 41654 | 2 |
| TESTI2002806 | 0.001095278 | 245031 | 1 |
| TESTI2002840 | 0.004190774 | 165756 | 2 |
| TESTI2002866 | 0.015748053 | 72286 | 7 |
| TESTI2002877 | 0.004490835 | 135151 | 3 |
| TESTI2002910 | 0.001095278 | 146360 | 1 |
| TESTI2002912 | 0.024096122 | 151158 | 22 |
| TESTI2002928 | 0.003285835 | 145281 | 3 |
| TESTI2002937 | 0.001095278 | 220088 | 1 |
| TESTI2002965 | 0.020543223 | 110181 | 16 |
| TESTI2002993 | 0.002190557 | 50406 | 2 |
| TESTI2003005 | 0.055151874 | 175120 | 6 |
| TESTI2003020 | 0.003285835 | 140905 | 3 |
| TESTI2003031 | 0.001095278 | 79677 | 1 |
| TESTI2003037 | 0.00657167 | 92963 | 6 |
| TESTI2003044 | 0.018358563 | 141578 | 7 |
| TESTI2003059 | 0.001095278 | 111172 | 1 |
| TESTI2003061 | 0.012939161 | 105355 | 9 |
| TESTI2003071 | 0.048025803 | 101971 | 4 |
| TESTI2003074 | 0.00657167 | 150400 | 6 |
| TESTI2003089 | 0.001095278 | 256927 | 1 |
| TESTI2003104 | 0.001095278 | 219919 | 1 |
| TESTI2003109 | 0.014706466 | 127396 | 12 |
| TESTI2003117 | 0.013143339 | 152016 | 12 |
| TESTI2003127 | 0.015692265 | 144358 | 4 |
| TESTI2003130 | 0.001095278 | 142013 | 1 |
| TESTI2003131 | 0.002190557 | 11698 | 2 |
| TESTI2003139 | 0.001095278 | 49143 | 1 |
| TESTI2003141 | 0.029698302 | 169058 | 17 |
| TESTI2003152 | 0.007666948 | 149426 | 7 |
| TESTI2003181 | 0.003285835 | 169633 | 3 |
| TESTI2003193 | 0.001095278 | 261479 | 1 |
| TESTI2003196 | 0.001095278 | 207514 | 1 |
| TESTI2003228 | 0.001095278 | 151488 | 1 |
| TESTI2003236 | 0.001095278 | 198524 | 1 |
| TESTI2003255 | 0.001095278 | 183829 | 1 |
| TESTI2003258 | 0.001095278 | 177099 | 1 |
| TESTI2003277 | 0.002190557 | 158078 | 2 |
| TESTI2003280 | 0.040846566 | 143562 | 13 |
| TESTI2003299 | 0.001095278 | 271594 | 1 |
| TESTI2003311 | 0.002190557 | 102478 | 2 |
| TESTI2003321 | 0.001095278 | 189477 | 1 |
| TESTI2003325 | 0.039591907 | 126123 | 8 |
| TESTI2003327 | 0.001095278 | 114082 | 1 |
| TESTI2003330 | 0.001095278 | 203090 | 1 |
| TESTI2003347 | 0.007666948 | 114395 | 7 |
| TESTI2003354 | 0.001095278 | 217258 | 1 |
| TESTI2003356 | 0.023000843 | 129482 | 21 |
| TESTI2003372 | 0.001095278 | 241195 | 1 |
| TESTI2003376 | 0.001095278 | 275753 | 1 |
| TESTI2003413 | 0.064354769 | 119803 | 16 |
| TESTI2003418 | 0.001095278 | 179834 | 1 |
| TESTI2003419 | 0.001095278 | 52769 | 1 |
| TESTI2003432 | 0.001095278 | 173651 | 1 |
| TESTI2003454 | 0.031553162 | 115718 | 6 |
| TESTI2003475 | 0.001095278 | 128662 | 1 |
| TESTI2003476 | 0.001095278 | 222858 | 1 |
| TESTI2003498 | 0.001095278 | 145194 | 1 |
| TESTI2003512 | 0.017524452 | 125938 | 16 |
| TESTI2003533 | 0.193260242 | 78627 | 23 |
| TESTI2003539 | 0.007037402 | 146522 | 2 |
| TESTI2003541 | 0.005736948 | 93433 | 4 |
| TESTI2003545 | 0.001095278 | 204932 | 1 |
| TESTI2003551 | 0.023000843 | 101336 | 21 |
| TESTI2003573 | 0.022433577 | 108147 | 7 |
| TESTI2003574 | 0.028477235 | 150596 | 26 |
| TESTI2003579 | 0.014464015 | 143369 | 4 |
| TESTI2003580 | 0.001095278 | 227906 | 1 |
| TESTI2003589 | 0.003285835 | 128570 | 3 |
| TESTI2003596 | 0.040217458 | 145726 | 30 |
| TESTI2003598 | 0.001095278 | 15300 | 1 |
| TESTI2003615 | 0.001095278 | 224242 | 1 |
| TESTI2003625 | 0.021891242 | 106628 | 8 |
| TESTI2003638 | 0.001095278 | 115099 | 1 |
| TESTI2003649 | 0.001095278 | 169925 | 1 |
| TESTI2003692 | 0.002804738 | 9686 | 2 |
| TESTI2003718 | 0.001095278 | 170227 | 1 |
| TESTI2003727 | 0.001095278 | 252048 | 1 |
| TESTI2003756 | 0.015971147 | 117819 | 5 |
| TESTI2003768 | 0.001095278 | 265731 | 1 |
| TESTI2003781 | 0.001095278 | 104914 | 1 |
| TESTI2003787 | 0.001095278 | 241921 | 1 |
| TESTI2003824 | 0.001095278 | 179332 | 1 |
| TESTI2003827 | 0.001095278 | 264974 | 1 |
| TESTI2003894 | 0.001095278 | 175304 | 1 |
| TESTI2003911 | 0.001095278 | 55215 | 1 |
| TESTI2003914 | 0.010952783 | 157578 | 10 |
| TESTI2003940 | 0.001095278 | 111215 | 1 |
| TESTI2003962 | 0.001095278 | 221980 | 1 |
| TESTI2003998 | 0.001095278 | 194396 | 1 |
| TESTI2004000 | 0.023900295 | 150117 | 3 |
| TESTI2004031 | 0.41796801 | 94161 | 32 |
| TESTI2004073 | 0.002190557 | 221886 | 2 |
| TESTI2004080 | 0.034333085 | 2702 | 7 |
| TESTI2004085 | 0.001095278 | 123490 | 1 |
| TESTI2004089 | 0.001095278 | 196517 | 1 |
| TESTI2004111 | 0.001095278 | 232498 | 1 |
| TESTI2004119 | 0.001095278 | 113237 | 1 |
| TESTI2004122 | 0.054881643 | 76368 | 7 |
| TESTI2004153 | 0.038622179 | 146384 | 11 |
| TESTI2004163 | 0.014238617 | 159550 | 13 |
| TESTI2004169 | 0.007666948 | 161692 | 7 |
| TESTI2004207 | 0.013143339 | 133969 | 12 |
| TESTI2004215 | 0.054763913 | 103926 | 50 |
| TESTI2004227 | 0.001095278 | 85103 | 1 |
| TESTI2004229 | 0.002190557 | 199241 | 2 |
| TESTI2004243 | 0.002190557 | 196593 | 2 |
| TESTI2004255 | 0.001095278 | 241059 | 1 |
| TESTI2004287 | 0.001095278 | 234657 | 1 |
| TESTI2004295 | 0.020462353 | 138900 | 9 |
| TESTI2004313 | 0.001095278 | 241056 | 1 |
| TESTI2004318 | 0.001095278 | 157092 | 1 |
| TESTI2004322 | 0.00657167 | 63038 | 6 |
| TESTI2004391 | 0.039676011 | 110289 | 29 |
| TESTI2004399 | 0.001095278 | 210238 | 1 |
| TESTI2004423 | 0.036144182 | 144950 | 33 |
| TESTI2004431 | 0.020030803 | 104706 | 9 |
| TESTI2004432 | 0.00297212 | 149161 | 2 |
| TESTI2004452 | 0.002190557 | 101966 | 2 |
| TESTI2004459 | 0.001095278 | 160533 | 1 |
| TESTI2004490 | 0.001095278 | 153831 | 1 |
| TESTI2004539 | 0.008762226 | 138191 | 8 |
| TESTI2004552 | 0.012048061 | 104424 | 11 |
| TESTI2004560 | 0.001095278 | 192469 | 1 |
| TESTI2004574 | 0.004381113 | 139495 | 4 |
| TESTI2004601 | 0.001095278 | 275263 | 1 |
| TESTI2004611 | 0.001095278 | 244629 | 1 |
| TESTI2004649 | 0.004381113 | 16947 | 4 |
| TESTI2004654 | 0.001095278 | 140198 | 1 |
| TESTI2004675 | 0.001095278 | 254270 | 1 |
| TESTI2004687 | 0.002190557 | 116944 | 2 |
| TESTI2004689 | 0.01861973 | 124397 | 17 |
| TESTI2004700 | 0.001095278 | 167092 | 1 |
| TESTI2004706 | 0.012048061 | 164952 | 11 |
| TESTI2004712 | 0.148154102 | 253428 | 2 |
| TESTI2004734 | 0.00488625 | 179957 | 3 |
| TESTI2004737 | 0.094592715 | 113384 | 23 |
| TESTI2004783 | 0.002190557 | 197228 | 2 |
| TESTI2004791 | 0.001095278 | 228537 | 1 |
| TESTI2004793 | 0.001095278 | 83242 | 1 |
| TESTI2004860 | 0.001095278 | 244678 | 1 |
| TESTI2004906 | 0.00657167 | 66844 | 6 |
| TESTI2004920 | 0.002190557 | 1926 | 2 |
| TESTI2004929 | 0.05901212 | 105357 | 10 |
| TESTI2004936 | 0.015333896 | 49269 | 14 |
| TESTI2004941 | 0.001095278 | 91085 | 1 |
| TESTI2004971 | 0.002190557 | 122350 | 2 |
| TESTI2004982 | 0.002190557 | 220971 | 2 |
| TESTI2004993 | 0.001095278 | 99923 | 1 |
| TESTI2004994 | 0.003285835 | 141087 | 3 |
| TESTI2004999 | 0.007666948 | 161516 | 7 |
| TESTI2005017 | 0.050736982 | 126735 | 31 |
| TESTI2005025 | 0.001095278 | 126403 | 1 |
| TESTI2005040 | 0.005286053 | 140802 | 3 |
| TESTI2005060 | 0.001095278 | 260445 | 1 |
| TESTI2005072 | 0.036238149 | 122814 | 33 |
| TESTI2005085 | 0.001095278 | 235790 | 1 |
| TESTI2005112 | 0.008612288 | 118777 | 3 |
| TESTI2005120 | 0.014238617 | 173590 | 13 |
| TESTI2005153 | 0.001095278 | 240998 | 1 |
| TESTI2005155 | 0.001095278 | 243370 | 1 |
| TESTI2005173 | 0.011621917 | 117344 | 6 |
| TESTI2005267 | 0.001095278 | 84861 | 1 |
| TESTI2005268 | 0.013143339 | 104241 | 12 |
| TESTI2005280 | 0.001095278 | 209390 | 1 |
| TESTI2005307 | 0.028988398 | 143164 | 17 |
| TESTI2005326 | 0.007308388 | 224269 | 2 |
| TESTI2005363 | 0.001095278 | 150880 | 1 |
| TESTI2005376 | 0.001095278 | 210826 | 1 |
| TESTI2005378 | 0.001095278 | 102823 | 1 |
| TESTI2005380 | 0.004381113 | 101760 | 4 |
| TESTI2005391 | 0.001095278 | 143906 | 1 |
| TESTI2005395 | 0.004272069 | 132790 | 3 |
| TESTI2005396 | 0.011355233 | 151385 | 5 |
| TESTI2005408 | 0.094575798 | 80759 | 19 |
| TESTI2005415 | 0.001095278 | 227486 | 1 |
| TESTI2005470 | 0.010952783 | 150957 | 10 |
| TESTI2005492 | 0.001095278 | 148638 | 1 |
| TESTI2005564 | 0.0457525 | 124503 | 14 |
| TESTI2005568 | 0.005476391 | 92197 | 5 |
| TESTI2005584 | 0.005476391 | 93837 | 5 |
| TESTI2005588 | 0.00657167 | 152872 | 6 |
| TESTI2005590 | 0.001095278 | 95041 | 1 |
| TESTI2005603 | 0.001095278 | 45383 | 1 |
| TESTI2005609 | 0.001095278 | 235163 | 1 |
| TESTI2005610 | 0.001095278 | 167051 | 1 |
| TESTI2005630 | 0.001095278 | 235167 | 1 |
| TESTI2005672 | 0.001095278 | 34912 | 1 |
| TESTI2005683 | 0.003285835 | 207983 | 3 |
| TESTI2005690 | 0.012048061 | 110293 | 11 |
| TESTI2005692 | 0.00297212 | 158883 | 2 |
| TESTI2005703 | 0.001095278 | 182681 | 1 |
| TESTI2005720 | 0.001095278 | 127867 | 1 |
| TESTI2005731 | 0.050712559 | 96513 | 36 |
| TESTI2005739 | 0.007666948 | 109120 | 7 |
| TESTI2005742 | 0.008216161 | 88067 | 5 |
| TESTI2005743 | 0.076277759 | 176278 | 12 |
| TESTI2005759 | 0.179164756 | 148829 | 15 |
| TESTI2005767 | 0.06126452 | 136272 | 54 |
| TESTI2005775 | 0.001095278 | 224282 | 1 |
| TESTI2005784 | 0.056335086 | 152979 | 24 |
| TESTI2005788 | 0.036229542 | 127716 | 14 |
| TESTI2005827 | 0.001095278 | 240946 | 1 |
| TESTI2005835 | 0.013906175 | 108527 | 12 |
| TESTI2005860 | 0.013143339 | 138381 | 12 |
| TESTI2005892 | 0.00657167 | 105353 | 6 |
| TESTI2005908 | 0.106409059 | 134637 | 16 |
| TESTI2005911 | 0.001095278 | 254177 | 1 |
| TESTI2005933 | 0.001095278 | 236620 | 1 |
| TESTI2005937 | 0.036144182 | 111805 | 33 |
| TESTI2005948 | 0.001095278 | 189482 | 1 |
| TESTI2005979 | 0.001095278 | 217971 | 1 |
| TESTI2005981 | 0.005380019 | 104110 | 3 |
| TESTI2005982 | 0.002190557 | 179406 | 2 |
| TESTI2005984 | 0.001095278 | 215420 | 1 |
| TESTI2005986 | 0.002190557 | 114789 | 2 |
| TESTI2005987 | 0.002190557 | 175378 | 2 |
| TESTI2006007 | 0.012559224 | 241487 | 2 |
| TESTI2006008 | 0.007666948 | 161807 | 7 |
| TESTI2006015 | 0.00657167 | 122736 | 6 |
| TESTI2006016 | 0.004381113 | 88396 | 4 |
| TESTI2006035 | 0.019715009 | 119148 | 18 |
| TESTI2006040 | 0.048192243 | 133968 | 44 |
| TESTI2006041 | 0.00657167 | 159591 | 6 |
| TESTI2006045 | 0.001095278 | 214384 | 1 |
| TESTI2006051 | 0.395698054 | 83105 | 22 |
| TESTI2006067 | 0.003285835 | 113705 | 3 |
| TESTI2006083 | 0.042715852 | 138402 | 39 |
| TESTI2006097 | 0.015903283 | 143544 | 4 |
| TESTI2006109 | 0.001095278 | 251841 | 1 |
| TESTI2006111 | 0.008127008 | 133876 | 4 |
| TESTI2006120 | 0.034525396 | 17311 | 11 |
| TESTI2006125 | 0.001095278 | 236760 | 1 |
| TESTI2006179 | 0.001095278 | 129098 | 1 |
| TESTI2006209 | 0.001095278 | 241298 | 1 |
| TESTI2006210 | 0.019493236 | 111112 | 7 |
| TESTI2006212 | 0.001095278 | 167411 | 1 |
| TESTI2006218 | 0.00657167 | 119158 | 6 |
| TESTI2006238 | 0.009857504 | 148848 | 9 |
| TESTI2006255 | 0.001095278 | 229399 | 1 |
| TESTI2006258 | 0.016340114 | 46160 | 5 |
| TESTI2006278 | 0.001095278 | 222013 | 1 |
| TESTI2006333 | 0.001095278 | 229387 | 1 |
| TESTI2006341 | 0.008197134 | 119970 | 3 |
| TESTI2006360 | 0.001095278 | 59486 | 1 |
| TESTI2006383 | 0.041340921 | 124081 | 22 |
| TESTI2006409 | 0.002804738 | 186831 | 2 |
| TESTI2006425 | 0.001095278 | 243095 | 1 |
| TESTI2006437 | 0.004381113 | 112563 | 4 |
| TESTI2006453 | 0.19910624 | 102991 | 31 |
| TESTI2006457 | 0.001095278 | 282264 | 1 |
| TESTI2006460 | 0.005476391 | 158446 | 5 |
| TESTI2006465 | 0.001095278 | 122732 | 1 |
| TESTI2006483 | 0.002190557 | 73956 | 2 |
| TESTI2006499 | 0.001095278 | 236655 | 1 |
| TESTI2006523 | 0.003285835 | 223630 | 3 |
| TESTI2006543 | 0.005476391 | 189087 | 5 |
| TESTI2006565 | 0.001095278 | 112658 | 1 |
| TESTI2006572 | 0.023000843 | 147361 | 21 |
| TESTI2006588 | 0.309499013 | 58157 | 71 |
| TESTI2006615 | 0.036305904 | 102896 | 28 |
| TESTI2006617 | 0.002190557 | 217331 | 2 |
| TESTI2006624 | 0.002190557 | 150069 | 2 |
| TESTI2006628 | 0.013477238 | 90406 | 3 |
| TESTI2006633 | 0.006267794 | 182672 | 4 |
| TESTI2006643 | 0.001095278 | 227111 | 1 |
| TESTI2006648 | 0.002190557 | 158573 | 2 |
| TESTI2006659 | 0.001095278 | 221354 | 1 |
| TESTI2006660 | 0.001095278 | 239630 | 1 |
| TESTI2006665 | 0.004381113 | 131851 | 4 |
| TESTI2006667 | 0.001095278 | 102423 | 1 |
| TESTI2006677 | 0.002190557 | 262860 | 2 |
| TESTI2006720 | 0.002492434 | 220392 | 2 |
| TESTI2006735 | 0.001095278 | 154587 | 1 |
| TESTI2006744 | 0.002190557 | 102041 | 2 |
| TESTI2006748 | 0.048707779 | 126666 | 15 |
| TESTI2006774 | 0.002284523 | 20868 | 2 |
| TESTI2006853 | 0.001095278 | 246766 | 1 |
| TESTI2006866 | 0.001095278 | 229301 | 1 |
| TESTI2006872 | 0.030491939 | 151499 | 15 |
| TESTI2006877 | 0.012625446 | 180490 | 8 |
| TESTI2006879 | 0.001095278 | 221121 | 1 |
| TESTI2006894 | 0.002190557 | 173742 | 2 |
| TESTI2006940 | 0.004381113 | 136873 | 4 |
| TESTI2006955 | 0.001095278 | 118245 | 1 |
| TESTI2006978 | 0.001095278 | 249533 | 1 |
| TESTI2006979 | 0.001095278 | 237757 | 1 |
| TESTI2006990 | 0.001095278 | 229347 | 1 |
| TESTI2007040 | 0.003285835 | 13254 | 3 |
| TESTI2007074 | 0.001095278 | 171317 | 1 |
| TESTI2007078 | 0.001095278 | 55511 | 1 |
| TESTI2007113 | 0.001095278 | 111798 | 1 |
| TESTI2007152 | 0.001095278 | 224245 | 1 |
| TESTI2007163 | 0.002284523 | 198541 | 2 |
| TESTI2007183 | 0.001095278 | 64845 | 1 |
| TESTI2007211 | 0.050730246 | 45917 | 13 |
| TESTI2007220 | 0.001095278 | 217950 | 1 |
| TESTI2007234 | 0.001095278 | 227314 | 1 |
| TESTI2007311 | 0.043553326 | 42662 | 12 |
| TESTI2007320 | 0.001095278 | 208155 | 1 |
| TESTI2007346 | 0.001095278 | 117550 | 1 |
| TESTI2007358 | 0.001095278 | 116442 | 1 |
| TESTI2007402 | 0.001095278 | 232258 | 1 |
| TESTI2007405 | 0.001095278 | 258810 | 1 |
| TESTI2007407 | 0.05458856 | 155116 | 13 |
| TESTI2007422 | 0.005407291 | 3284 | 3 |
| TESTI2007452 | 0.001095278 | 191270 | 1 |
| TESTI2007464 | 0.041775597 | 51451 | 11 |
| TESTI2007466 | 0.001095278 | 141092 | 1 |
| TESTI2007480 | 0.005476391 | 127637 | 5 |
| TESTI2007490 | 0.001095278 | 94145 | 1 |
| TESTI2007502 | 0.001095278 | 200678 | 1 |
| TESTI2007524 | 0.001095278 | 125307 | 1 |
| TESTI2007533 | 0.001095278 | 121054 | 1 |
| TESTI2007576 | 0.001095278 | 175313 | 1 |
| TESTI2007613 | 0.002190557 | 84860 | 2 |
| TESTI2007657 | 0.004381113 | 62038 | 4 |
| TESTI2007685 | 0.001095278 | 260016 | 1 |
| TESTI2007692 | 0.001095278 | 106625 | 1 |
| TESTI2007749 | 0.001095278 | 244970 | 1 |
| TESTI2007750 | 0.001095278 | 141386 | 1 |
| TESTI2007808 | 0.002190557 | 104428 | 2 |
| TESTI2007814 | 0.001095278 | 173678 | 1 |
| TESTI2007829 | 0.001095278 | 245158 | 1 |
| TESTI2007864 | 0.002492434 | 105027 | 2 |
| TESTI2007867 | 0.001095278 | 105849 | 1 |
| TESTI2007872 | 0.001095278 | 102035 | 1 |
| TESTI2007906 | 0.001095278 | 193030 | 1 |
| TESTI2007922 | 0.042403927 | 141356 | 5 |
| TESTI2007951 | 0.001095278 | 32134 | 1 |
| TESTI2007972 | 0.001095278 | 254421 | 1 |
| TESTI2007973 | 0.001095278 | 258795 | 1 |
| TESTI2007998 | 0.105970221 | 89898 | 25 |
| TESTI2008014 | 0.002190557 | 158826 | 2 |
| TESTI2008020 | 0.043689936 | 48484 | 32 |
| TESTI2008032 | 0.001095278 | 237073 | 1 |
| TESTI2008033 | 0.001095278 | 240211 | 1 |
| TESTI2008046 | 0.001095278 | 203712 | 1 |
| TESTI2008081 | 0.003285835 | 116778 | 3 |
| TESTI2008099 | 0.001095278 | 111813 | 1 |
| TESTI2008139 | 0.007666948 | 149661 | 7 |
| TESTI2008144 | 0.001095278 | 227913 | 1 |
| TESTI2008161 | 0.001095278 | 224236 | 1 |
| TESTI2008189 | 0.001095278 | 141498 | 1 |
| TESTI2008204 | 0.001095278 | 210413 | 1 |
| TESTI2008219 | 0.001095278 | 201042 | 1 |
| TESTI2008233 | 0.012741189 | 179389 | 5 |
| TESTI2008234 | 0.001095278 | 204190 | 1 |
| TESTI2008237 | 0.001095278 | 37288 | 1 |
| TESTI2008240 | 0.67004871 | 54853 | 182 |
| TESTI2008278 | 0.013609072 | 148466 | 8 |
| TESTI2008320 | 0.001095278 | 167579 | 1 |
| TESTI2008331 | 0.001095278 | 215711 | 1 |
| TESTI2008343 | 0.001095278 | 142014 | 1 |
| TESTI2008387 | 0.013143339 | 161634 | 12 |
| TESTI2008389 | 0.001095278 | 209994 | 1 |
| TESTI2008394 | 0.004190774 | 181282 | 2 |
| TESTI2008405 | 0.032176504 | 193604 | 3 |
| TESTI2008420 | 0.001095278 | 181995 | 1 |
| TESTI2008425 | 0.00657167 | 91608 | 6 |
| TESTI2008440 | 0.001095278 | 220546 | 1 |
| TESTI2008567 | 0.003285835 | 138127 | 3 |
| TESTI2008595 | 0.002190557 | 121072 | 2 |
| TESTI2008621 | 0.016584682 | 116953 | 9 |
| TESTI2008636 | 0.001095278 | 140312 | 1 |
| TESTI2008657 | 0.002190557 | 202082 | 2 |
| TESTI2008684 | 0.001095278 | 213559 | 1 |
| TESTI2008699 | 0.017206957 | 134250 | 4 |
| TESTI2008715 | 0.001095278 | 175288 | 1 |
| TESTI2008762 | 0.007666948 | 143792 | 7 |
| TESTI2008774 | 0.001095278 | 65548 | 1 |
| TESTI2008786 | 0.001095278 | 196426 | 1 |
| TESTI2008822 | 0.001095278 | 19035 | 1 |
| TESTI2008835 | 0.015442408 | 180418 | 3 |
| TESTI2008840 | 0.008102819 | 172267 | 3 |
| TESTI2008847 | 0.003878833 | 134355 | 3 |
| TESTI2008884 | 0.002190557 | 185774 | 2 |
| TESTI2008901 | 0.001095278 | 176838 | 1 |
| TESTI2008929 | 0.001095278 | 87412 | 1 |
| TESTI2008946 | 0.001095278 | 205262 | 1 |
| TESTI2008960 | 0.002190557 | 232347 | 2 |
| TESTI2009018 | 0.00657167 | 78633 | 6 |
| TESTI2009091 | 0.001095278 | 224298 | 1 |
| TESTI2009097 | 0.001095278 | 193029 | 1 |
| TESTI2009136 | 0.001095278 | 202945 | 1 |
| TESTI2009185 | 0.001095278 | 219678 | 1 |
| TESTI2009190 | 0.001095278 | 150992 | 1 |
| TESTI2009217 | 0.002190557 | 180488 | 2 |
| TESTI2009239 | 0.002284523 | 156904 | 2 |
| TESTI2009325 | 0.001095278 | 240093 | 1 |
| TESTI2009388 | 0.008356906 | 103868 | 3 |
| TESTI2009390 | 0.001095278 | 197304 | 1 |
| TESTI2009400 | 0.001095278 | 229116 | 1 |
| TESTI2009402 | 0.018277016 | 174258 | 8 |
| TESTI2009412 | 0.004381113 | 166994 | 4 |
| TESTI2009423 | 0.010361611 | 1672 | 3 |
| TESTI2009447 | 0.001095278 | 170160 | 1 |
| TESTI2009462 | 0.001095278 | 167078 | 1 |
| TESTI2009474 | 0.082932325 | 151127 | 15 |
| TESTI2009477 | 0.004381113 | 111210 | 4 |
| TESTI2009497 | 0.00317679 | 64104 | 2 |
| TESTI2009502 | 0.037079445 | 10717 | 2 |
| TESTI2009511 | 0.002190557 | 237859 | 2 |
| TESTI2009520 | 0.223425027 | 89444 | 65 |
| TESTI2009544 | 0.001095278 | 102412 | 1 |
| TESTI2009545 | 0.002190557 | 229079 | 2 |
| TESTI2009551 | 0.001095278 | 213179 | 1 |
| TESTI2009577 | 0.094802209 | 112704 | 24 |
| TESTI2009585 | 0.001095278 | 233349 | 1 |
| TESTI2009588 | 0.001095278 | 21448 | 1 |
| TESTI2009683 | 0.002783555 | 49195 | 2 |
| TESTI2009727 | 0.001095278 | 142737 | 1 |
| TESTI2009739 | 0.001095278 | 194417 | 1 |
| TESTI2009785 | 0.116781786 | 97445 | 37 |
| TESTI2009812 | 0.003285835 | 173878 | 3 |
| TESTI2009835 | 0.023000843 | 132182 | 21 |
| TESTI2009853 | 0.001095278 | 264333 | 1 |
| TESTI2009935 | 0.002190557 | 157711 | 2 |
| TESTI2009977 | 0.006936741 | 145953 | 2 |
| TESTI2009987 | 0.032847288 | 107864 | 14 |
| TESTI2010009 | 0.002190557 | 74835 | 2 |
| TESTI2010154 | 0.007666948 | 32391 | 7 |
| TESTI2010180 | 0.004830234 | 46361 | 3 |
| TESTI2010239 | 0.001095278 | 190273 | 1 |
| TESTI2010280 | 0.001095278 | 127909 | 1 |
| TESTI2010354 | 0.001095278 | 134246 | 1 |
| TESTI2010369 | 0.048192243 | 133968 | 44 |
| TESTI2010396 | 0.004381113 | 114883 | 4 |
| TESTI2010400 | 0.001095278 | 139007 | 1 |
| TESTI2010409 | 0.001095278 | 144675 | 1 |
| TESTI2010513 | 0.42648461 | 20991 | 124 |
| TESTI2010572 | 0.001095278 | 127942 | 1 |
| TESTI2010587 | 0.001095278 | 102829 | 1 |
| TESTI2010591 | 0.001095278 | 139066 | 1 |
| TESTI2010617 | 0.024061147 | 133202 | 10 |
| TESTI2010682 | 0.001095278 | 37515 | 1 |
| TESTI2010724 | 0.044665455 | 146424 | 39 |
| TESTI2010732 | 0.001095278 | 120997 | 1 |
| TESTI2010734 | 0.005476391 | 119013 | 5 |
| TESTI2010755 | 0.001095278 | 260830 | 1 |
| TESTI2010793 | 0.001095278 | 263878 | 1 |
| TESTI2010806 | 0.001095278 | 11274 | 1 |
| TESTI2010833 | 0.001095278 | 248021 | 1 |
| TESTI2010872 | 0.001095278 | 227803 | 1 |
| TESTI2011009 | 0.001095278 | 97592 | 1 |
| TESTI2011020 | 0.001095278 | 112953 | 1 |
| TESTI2011028 | 0.001095278 | 248157 | 1 |
| TESTI2011033 | 0.001095278 | 245329 | 1 |
| TESTI2011254 | 0.001095278 | 104721 | 1 |
| TESTI2011286 | 0.012792762 | 160332 | 3 |
| TESTI2011294 | 0.001095278 | 233429 | 1 |
| TESTI2011314 | 0.001095278 | 214663 | 1 |
| TESTI2011315 | 0.001095278 | 232251 | 1 |
| TESTI2011394 | 0.001095278 | 154139 | 1 |
| TESTI2011407 | 0.002190557 | 205750 | 2 |
| TESTI2011448 | 0.002190557 | 99426 | 2 |
| TESTI2011480 | 0.001095278 | 116354 | 1 |
| TESTI2011549 | 0.002783555 | 138139 | 2 |
| TESTI2011575 | 0.007666948 | 14049 | 7 |
| TESTI2011605 | 0.046487668 | 78989 | 16 |
| TESTI2011612 | 0.001095278 | 11124 | 1 |
| TESTI2011665 | 0.001095278 | 183949 | 1 |
| TESTI2011680 | 0.022398562 | 91617 | 10 |
| TESTI2011683 | 0.005175192 | 86955 | 3 |
| TESTI2011712 | 0.008103168 | 135618 | 3 |
| TESTI2011750 | 0.001095278 | 141737 | 1 |
| TESTI2011840 | 0.003285835 | 212928 | 3 |
| TESTI2011846 | 0.003285835 | 119608 | 3 |
| TESTI2011915 | 0.001095278 | 107491 | 1 |
| TESTI2011928 | 0.002190557 | 224261 | 2 |
| TESTI2011971 | 0.003285835 | 131444 | 3 |
| TESTI2012019 | 0.043221937 | 143805 | 4 |
| TESTI2012050 | 0.001095278 | 262752 | 1 |
| TESTI2012090 | 0.001095278 | 188236 | 1 |
| TESTI2012095 | 0.002190557 | 163587 | 2 |
| TESTI2012104 | 0.017211705 | 166692 | 4 |
| TESTI2012152 | 0.001095278 | 60230 | 1 |
| TESTI2012155 | 0.026147931 | 119533 | 8 |
| TESTI2012171 | 0.017524452 | 53389 | 16 |
| TESTI2012207 | 0.002190557 | 88387 | 2 |
| TESTI2012231 | 0.001095278 | 272337 | 1 |
| TESTI2012244 | 0.001095278 | 65674 | 1 |
| TESTI2012289 | 0.019412667 | 122520 | 4 |
| TESTI2012308 | 0.001095278 | 143489 | 1 |
| TESTI2012356 | 0.057784621 | 145152 | 2 |
| TESTI2012387 | 0.001095278 | 138248 | 1 |
| TESTI2012394 | 0.006257955 | 115014 | 5 |
| TESTI2012444 | 0.003285835 | 141843 | 3 |
| TESTI2012511 | 0.039028571 | 111444 | 3 |
| TESTI2012528 | 0.001095278 | 117811 | 1 |
| TESTI2012544 | 0.001095278 | 134023 | 1 |
| TESTI2012550 | 0.003285835 | 83790 | 3 |
| TESTI2012571 | 0.016358454 | 122471 | 6 |
| TESTI2012592 | 0.001095278 | 53944 | 1 |
| TESTI2012617 | 0.001095278 | 109815 | 1 |
| TESTI2012628 | 0.008762226 | 134322 | 8 |
| TESTI2012778 | 0.90683466 | 65039 | 51 |
| TESTI2012812 | 0.002190557 | 104934 | 2 |
| TESTI2012834 | 0.001095278 | 119001 | 1 |
| TESTI2012835 | 0.001095278 | 105026 | 1 |
| TESTI2012861 | 0.001095278 | 101834 | 1 |
| TESTI2012900 | 0.001095278 | 106562 | 1 |
| TESTI2012915 | 0.001095278 | 92646 | 1 |
| TESTI2012922 | 0.001095278 | 103409 | 1 |
| TESTI2013001 | 0.001095278 | 45272 | 1 |
| TESTI2013012 | 0.001095278 | 197169 | 1 |
| TESTI2013053 | 0.002190557 | 131132 | 2 |
| TESTI2013144 | 0.001095278 | 59885 | 1 |
| TESTI2013163 | 0.001095278 | 129715 | 1 |
| TESTI2013169 | 0.001095278 | 126753 | 1 |
| TESTI2013212 | 0.001095278 | 134321 | 1 |
| TESTI2013231 | 0.084206555 | 114185 | 10 |
| TESTI2013257 | 0.001095278 | 122758 | 1 |
| TESTI2013264 | 0.006669369 | 100871 | 3 |
| TESTI2013268 | 0.002190557 | 142721 | 2 |
| TESTI2013295 | 0.062906448 | 86530 | 20 |
| TESTI2013322 | 0.001095278 | 120720 | 1 |
| TESTI2013381 | 0.001095278 | 141645 | 1 |
| TESTI2013382 | 0.001095278 | 191273 | 1 |
| TESTI2013401 | 0.001095278 | 78635 | 1 |
| TESTI2013427 | 0.001095278 | 114166 | 1 |
| TESTI2013468 | 0.00657167 | 117512 | 6 |
| TESTI2013497 | 0.002190557 | 112639 | 2 |
| TESTI2013545 | 0.001095278 | 67192 | 1 |
| TESTI2013546 | 0.001095278 | 182630 | 1 |
| TESTI2013566 | 0.001095278 | 283942 | 1 |
| TESTI2013601 | 0.004381113 | 150467 | 4 |
| TESTI2013604 | 0.001095278 | 154904 | 1 |
| TESTI2013607 | 0.001095278 | 152291 | 1 |
| TESTI2013610 | 0.001095278 | 161462 | 1 |
| TESTI2013671 | 0.005476391 | 127637 | 5 |
| TESTI2013691 | 0.002190557 | 233350 | 2 |
| TESTI2013699 | 0.002190557 | 173579 | 2 |
| TESTI2013737 | 0.001095278 | 161445 | 1 |
| TESTI2013767 | 0.001095278 | 139408 | 1 |
| TESTI2013832 | 0.001095278 | 108931 | 1 |
| TESTI2013873 | 0.012048061 | 120689 | 11 |
| TESTI2013889 | 0.001095278 | 119992 | 1 |
| TESTI2014007 | 0.001095278 | 50707 | 1 |
| TESTI2014036 | 0.001095278 | 45600 | 1 |
| TESTI2014097 | 0.001095278 | 143829 | 1 |
| TESTI2014160 | 0.001095278 | 241583 | 1 |
| TESTI2014248 | 0.001095278 | 87741 | 1 |
| TESTI2014254 | 0.001095278 | 141034 | 1 |
| TESTI2014269 | 0.001095278 | 42575 | 1 |
| TESTI2014274 | 0.001095278 | 5880 | 1 |
| TESTI2014318 | 0.005828436 | 152052 | 3 |
| TESTI2014324 | 0.001095278 | 148555 | 1 |
| TESTI2014339 | 0.001095278 | 134096 | 1 |
| TESTI2014362 | 0.001095278 | 143116 | 1 |
| TESTI2014385 | 0.001095278 | 135726 | 1 |
| TESTI2014415 | 0.001095278 | 139627 | 1 |
| TESTI2014439 | 0.001095278 | 111171 | 1 |
| TESTI2014474 | 0.001095278 | 120699 | 1 |
| TESTI2014505 | 0.008502787 | 56163 | 4 |
| TESTI2014577 | 0.001095278 | 155589 | 1 |
| TESTI2014578 | 0.001095278 | 134188 | 1 |
| TESTI2014716 | 0.001095278 | 75005 | 1 |
| TESTI2014780 | 0.005900234 | 159060 | 3 |
| TESTI2014800 | 0.00657167 | 161194 | 6 |
| TESTI2014838 | 0.126461488 | 108957 | 105 |
| TESTI2014843 | 0.001095278 | 180903 | 1 |
| TESTI2014979 | 0.001095278 | 159390 | 1 |
| TESTI2014988 | 0.001095278 | 108989 | 1 |
| TESTI2015042 | 0.003900017 | 129176 | 3 |
| TESTI2015092 | 0.05057616 | 93986 | 11 |
| TESTI2015105 | 0.002190557 | 158327 | 2 |
| TESTI2015180 | 0.001095278 | 218209 | 1 |
| TESTI2015213 | 0.326482171 | 56648 | 34 |
| TESTI2015246 | 0.014152782 | 29075 | 6 |
| TESTI2015249 | 0.002190557 | 219007 | 2 |
| TESTI2015327 | 0.001095278 | 68869 | 1 |
| TESTI2015331 | 0.003285835 | 112673 | 3 |
| TESTI2015335 | 0.00746277 | 104899 | 4 |
| TESTI2015350 | 0.001095278 | 122713 | 1 |
| TESTI2015375 | 0.001095278 | 250487 | 1 |
| TESTI2015437 | 0.04103138 | 103739 | 2 |
| TESTI2015553 | 0.053922974 | 99624 | 20 |
| TESTI2015590 | 0.003285835 | 104870 | 3 |
| TESTI2015610 | 0.00657167 | 111253 | 6 |
| TESTI2015626 | 0.509496239 | 72837 | 59 |
| TESTI2015647 | 0.001095278 | 101488 | 1 |
| TESTI2015670 | 0.040912856 | 120940 | 16 |
| TESTI2015675 | 0.004381113 | 77713 | 4 |
| TESTI2015710 | 0.001095278 | 90601 | 1 |
| TESTI2015752 | 0.019715009 | 156787 | 18 |
| TESTI2015830 | 0.001095278 | 142550 | 1 |
| TESTI2015914 | 0.001095278 | 94120 | 1 |
| TESTI2015938 | 0.001095278 | 83398 | 1 |
| TESTI2015947 | 0.001095278 | 163326 | 1 |
| TESTI2015962 | 0.042239159 | 124563 | 20 |
| TESTI2015987 | 0.001095278 | 163321 | 1 |
| TESTI2016033 | 0.096174573 | 105104 | 6 |
| TESTI2016046 | 0.001095278 | 105830 | 1 |
| TESTI2016101 | 0.038646991 | 142812 | 3 |
| TESTI2016162 | 0.001095278 | 171102 | 1 |
| TESTI2016197 | 0.001095278 | 194138 | 1 |
| TESTI2016278 | 0.002953393 | 153417 | 2 |
| TESTI2016298 | 0.004381113 | 159526 | 4 |
| TESTI2016331 | 0.003285835 | 197791 | 3 |
| TESTI2016337 | 0.001095278 | 46653 | 1 |
| TESTI2016410 | 0.001095278 | 247960 | 1 |
| TESTI2016421 | 0.002190557 | 116887 | 2 |
| TESTI2016509 | 0.001095278 | 100231 | 1 |
| TESTI2016568 | 0.001095278 | 135253 | 1 |
| TESTI2016583 | 0.009315576 | 151106 | 4 |
| TESTI2016599 | 0.001095278 | 122062 | 1 |
| TESTI2016629 | 0.001095278 | 151132 | 1 |
| TESTI2016663 | 0.001095278 | 128439 | 1 |
| TESTI2016667 | 0.001095278 | 102767 | 1 |
| TESTI2016688 | 0.003285835 | 191455 | 3 |
| TESTI2016701 | 0.002284523 | 8450 | 2 |
| TESTI2016758 | 0.001095278 | 135294 | 1 |
| TESTI2016848 | 0.001095278 | 169639 | 1 |
| TESTI2016863 | 0.001095278 | 173580 | 1 |
| TESTI2016888 | 0.001095278 | 81290 | 1 |
| TESTI2016896 | 0.003900017 | 47954 | 3 |
| TESTI2016922 | 0.001095278 | 235053 | 1 |
| TESTI2016942 | 0.002190557 | 173730 | 2 |
| TESTI2016950 | 0.001095278 | 153059 | 1 |
| TESTI2016996 | 0.001095278 | 222527 | 1 |
| TESTI2017017 | 0.003285835 | 120395 | 3 |
| TESTI2017028 | 0.001095278 | 283695 | 1 |
| TESTI2017035 | 0.003993983 | 195724 | 3 |
| TESTI2017069 | 0.001095278 | 232402 | 1 |
| TESTI2017089 | 0.001095278 | 163588 | 1 |
| TESTI2017102 | 0.001095278 | 251328 | 1 |
| TESTI2017107 | 0.002783555 | 48433 | 2 |
| TESTI2017113 | 0.001095278 | 133129 | 1 |
| TESTI2017125 | 0.002190557 | 234307 | 2 |
| TESTI2017256 | 0.001095278 | 215307 | 1 |
| TESTI2017305 | 0.001095278 | 107509 | 1 |
| TESTI2017311 | 0.001095278 | 111156 | 1 |
| TESTI2017396 | 0.004381113 | 111912 | 4 |
| TESTI2017507 | 0.001095278 | 48611 | 1 |
| TESTI2017537 | 0.001095278 | 101981 | 1 |
| TESTI2017582 | 0.001095278 | 171225 | 1 |
| TESTI2017584 | 0.001095278 | 147601 | 1 |
| TESTI2017645 | 0.005034905 | 159528 | 3 |
| TESTI2017727 | 0.002190557 | 191380 | 2 |
| TESTI2017740 | 0.001095278 | 167063 | 1 |
| TESTI2017775 | 0.001095278 | 111840 | 1 |
| TESTI2017816 | 0.002190557 | 175887 | 2 |
| TESTI2017832 | 0.001095278 | 113545 | 1 |
| TESTI2017923 | 0.00657167 | 150245 | 6 |
| TESTI2017932 | 0.001095278 | 173679 | 1 |
| TESTI2017951 | 0.001095278 | 140586 | 1 |
| TESTI2017954 | 0.252934652 | 65617 | 33 |
| TESTI2018014 | 0.043470488 | 83984 | 15 |
| TESTI2018031 | 0.001095278 | 155611 | 1 |
| TESTI2018035 | 0.001095278 | 19260 | 1 |
| TESTI2018060 | 0.001095278 | 171183 | 1 |
| TESTI2018083 | 0.001095278 | 191387 | 1 |
| TESTI2018178 | 0.001095278 | 166338 | 1 |
| TESTI2018221 | 0.001095278 | 259105 | 1 |
| TESTI2018276 | 0.001095278 | 139071 | 1 |
| TESTI2018290 | 0.002190557 | 164425 | 2 |
| TESTI2018335 | 0.005476391 | 160170 | 5 |
| TESTI2018337 | 0.001095278 | 184601 | 1 |
| TESTI2018368 | 0.001095278 | 149863 | 1 |
| TESTI2018421 | 0.007136844 | 163573 | 2 |
| TESTI2018428 | 0.001095278 | 245189 | 1 |
| TESTI2018462 | 0.059292988 | 152539 | 23 |
| TESTI2018475 | 0.001095278 | 141222 | 1 |
| TESTI2018482 | 0.001095278 | 155532 | 1 |
| TESTI2018521 | 0.001095278 | 86141 | 1 |
| TESTI2018565 | 0.002190557 | 152426 | 2 |
| TESTI2018581 | 0.002190557 | 163356 | 2 |
| TESTI2018611 | 0.159500105 | 46915 | 8 |
| TESTI2018687 | 0.001095278 | 167033 | 1 |
| TESTI2018697 | 0.001095278 | 151242 | 1 |
| TESTI2018838 | 0.001095278 | 194264 | 1 |
| TESTI2018867 | 0.058840224 | 119609 | 43 |
| TESTI2018941 | 0.003285835 | 124398 | 3 |
| TESTI2018957 | 0.003285835 | 183887 | 3 |
| TESTI2018974 | 0.001095278 | 58580 | 1 |
| TESTI2018975 | 0.001095278 | 201723 | 1 |
| TESTI2018997 | 0.080451144 | 157343 | 10 |
| TESTI2019042 | 0.001095278 | 116454 | 1 |
| TESTI2019077 | 0.002492434 | 173691 | 2 |
| TESTI2019228 | 0.001095278 | 173686 | 1 |
| TESTI2019257 | 0.001095278 | 180025 | 1 |
| TESTI2019280 | 0.001095278 | 153818 | 1 |
| TESTI2019282 | 0.001095278 | 164899 | 1 |
| TESTI2019308 | 0.001095278 | 70208 | 1 |
| TESTI2019336 | 0.001095278 | 161950 | 1 |
| TESTI2019352 | 0.001095278 | 159616 | 1 |
| TESTI2019406 | 0.001095278 | 99199 | 1 |
| TESTI2019430 | 0.001095278 | 175901 | 1 |
| TESTI2019455 | 0.001095278 | 23776 | 1 |
| TESTI2019481 | 0.001095278 | 180093 | 1 |
| TESTI2019488 | 0.001095278 | 182698 | 1 |
| TESTI2019501 | 0.001095278 | 128727 | 1 |
| TESTI2019648 | 0.001095278 | 129855 | 1 |
| TESTI2019669 | 0.001095278 | 120268 | 1 |
| TESTI2019697 | 0.001095278 | 9406 | 1 |
| TESTI2019729 | 0.004216085 | 106807 | 2 |
| TESTI2019760 | 0.003681678 | 54172 | 3 |
| TESTI2019787 | 0.001095278 | 194283 | 1 |
| TESTI2019794 | 0.001095278 | 123800 | 1 |
| TESTI2019854 | 0.001095278 | 169687 | 1 |
| TESTI2019859 | 0.001095278 | 46525 | 1 |
| TESTI2019860 | 0.001095278 | 151240 | 1 |
| TESTI2019872 | 0.001095278 | 151940 | 1 |
| TESTI2019911 | 0.003285835 | 175311 | 3 |
| TESTI2019917 | 0.001095278 | 180759 | 1 |
| TESTI2019975 | 0.001095278 | 216634 | 1 |
| TESTI2019998 | 0.003285835 | 166992 | 3 |
| TESTI2020012 | 0.007331636 | 77150 | 2 |
| TESTI2020026 | 0.012901654 | 188234 | 2 |
| TESTI2020029 | 0.001095278 | 204184 | 1 |
| TESTI2020071 | 0.002190557 | 13143 | 2 |
| TESTI2020084 | 0.001095278 | 79302 | 1 |
| TESTI2020176 | 0.007666948 | 109120 | 7 |
| TESTI2020265 | 0.001095278 | 224386 | 1 |
| TESTI2020283 | 0.001095278 | 239052 | 1 |
| TESTI2020316 | 0.001095278 | 224231 | 1 |
| TESTI2020344 | 0.001095278 | 199910 | 1 |
| TESTI2020379 | 0.001095278 | 245535 | 1 |
| TESTI2020426 | 0.001095278 | 131163 | 1 |
| TESTI2020445 | 0.001095278 | 133916 | 1 |
| TESTI2020510 | 0.023163784 | 148824 | 8 |
| TESTI2020515 | 0.001095278 | 237627 | 1 |
| TESTI2020525 | 0.005133561 | 81411 | 2 |
| TESTI2020579 | 0.004381113 | 170062 | 4 |
| TESTI2020717 | 0.001095278 | 214199 | 1 |
| TESTI2020840 | 0.010253348 | 101332 | 9 |
| TESTI2020871 | 0.00309368 | 179808 | 2 |
| TESTI2020905 | 0.001095278 | 119474 | 1 |
| TESTI2020906 | 0.004381113 | 32393 | 4 |
| TESTI2020946 | 0.013689737 | 234487 | 2 |
| TESTI2020956 | 0.03893756 | 210067 | 3 |
| TESTI2020981 | 0.001095278 | 240059 | 1 |
| TESTI2020999 | 0.001095278 | 185893 | 1 |
| TESTI2021003 | 0.001095278 | 30056 | 1 |
| TESTI2021038 | 0.00396007 | 82744 | 3 |
| TESTI2021057 | 0.001095278 | 204292 | 1 |
| TESTI2021077 | 0.001095278 | 174579 | 1 |
| TESTI2021112 | 0.001095278 | 171096 | 1 |
| TESTI2021114 | 0.002190557 | 134348 | 2 |
| TESTI2021116 | 0.001095278 | 109145 | 1 |
| TESTI2021118 | 0.015619607 | 85701 | 2 |
| TESTI2021122 | 0.001095278 | 96941 | 1 |
| TESTI2021124 | 0.029317298 | 149185 | 14 |
| TESTI2021138 | 0.003285835 | 161757 | 3 |
| TESTI2021297 | 0.02440723 | 175070 | 5 |
| TESTI2021315 | 0.008268012 | 166879 | 4 |
| TESTI2021358 | 0.004951794 | 137372 | 3 |
| TESTI2021425 | 0.097323164 | 102390 | 18 |
| TESTI2021447 | 0.006462625 | 153342 | 5 |
| TESTI2021463 | 0.001095278 | 149350 | 1 |
| TESTI2021531 | 0.010014365 | 23488 | 4 |
| TESTI2021599 | 0.001095278 | 132409 | 1 |
| TESTI2021637 | 0.001095278 | 121497 | 1 |
| TESTI2021654 | 0.017144414 | 107901 | 5 |
| TESTI2021667 | 0.001095278 | 174138 | 1 |
| TESTI2021736 | 0.001095278 | 49506 | 1 |
| TESTI2021785 | 0.001095278 | 196646 | 1 |
| TESTI2021819 | 0.001095278 | 258340 | 1 |
| TESTI2021851 | 0.008855938 | 141718 | 3 |
| TESTI2021895 | 0.001095278 | 106690 | 1 |
| TESTI2021911 | 0.001095278 | 71636 | 1 |
| TESTI2021939 | 0.001095278 | 223310 | 1 |
| TESTI2021999 | 0.001095278 | 213330 | 1 |
| TESTI2022010 | 0.001095278 | 264922 | 1 |
| TESTI2022023 | 0.001095278 | 223315 | 1 |
| TESTI2022065 | 0.001095278 | 223319 | 1 |
| TESTI2022086 | 0.001095278 | 206702 | 1 |
| TESTI2022179 | 0.015953069 | 133860 | 10 |
| TESTI2022203 | 0.001095278 | 52765 | 1 |
| TESTI2022246 | 0.001095278 | 171062 | 1 |
| TESTI2022323 | 0.001095278 | 182755 | 1 |
| TESTI2022338 | 0.001095278 | 31806 | 1 |
| TESTI2022400 | 0.001095278 | 114252 | 1 |
| TESTI2022462 | 0.001095278 | 203695 | 1 |
| TESTI2022495 | 0.001095278 | 196417 | 1 |
| TESTI2022623 | 0.001095278 | 153631 | 1 |
| TESTI2022649 | 0.001095278 | 183561 | 1 |
| TESTI2022652 | 0.003285835 | 105831 | 3 |
| TESTI2022738 | 0.001095278 | 108833 | 1 |
| TESTI2022798 | 0.001095278 | 49797 | 1 |
| TESTI2022812 | 0.002190557 | 129177 | 2 |
| TESTI2022814 | 0.001095278 | 123238 | 1 |
| TESTI2022874 | 0.001095278 | 89841 | 1 |
| TESTI2022940 | 0.001095278 | 167158 | 1 |
| TESTI2022952 | 0.001095278 | 17684 | 1 |
| TESTI2022960 | 0.018642681 | 60769 | 10 |
| TESTI2023025 | 0.013317229 | 175687 | 2 |
| TESTI2023033 | 0.012203146 | 45374 | 7 |
| TESTI2023053 | 0.001095278 | 167080 | 1 |
| TESTI2023085 | 0.017524452 | 152068 | 16 |
| TESTI2023192 | 0.001095278 | 136192 | 1 |
| TESTI2023194 | 0.001095278 | 143912 | 1 |
| TESTI2023214 | 0. 002190557 | 111200 | 2 |
| TESTI2023254 | 0.001095278 | 106460 | 1 |
| TESTI2023386 | 0.002190557 | 155436 | 2 |
| TESTI2023414 | 0.041164425 | 135462 | 18 |
| TESTI2023427 | 0.001095278 | 123050 | 1 |
| TESTI2023436 | 0.001095278 | 132838 | 1 |
| TESTI2023599 | 0.001095278 | 191367 | 1 |
| TESTI2023752 | 0.002190557 | 167409 | 2 |
| TESTI2023800 | 0.003285835 | 83118 | 3 |
| TESTI2023861 | 0.001095278 | 192415 | 1 |
| TESTI2023903 | 0.001095278 | 79123 | 1 |
| TESTI2023942 | 0.012048061 | 133907 | 11 |
| TESTI2023947 | 0.001095278 | 227045 | 1 |
| TESTI2023951 | 0.001095278 | 167756 | 1 |
| TESTI2023968 | 0.002190557 | 247379 | 2 |
| TESTI2024009 | 0.001095278 | 189770 | 1 |
| TESTI2024090 | 0.004077237 | 73492 | 2 |
| TESTI2024125 | 0.00470917 | 204928 | 2 |
| TESTI2024153 | 0.001095278 | 204475 | 1 |
| TESTI2024192 | 0.001095278 | 175641 | 1 |
| TESTI2024267 | 0.001095278 | 110644 | 1 |
| TESTI2024283 | 0.00891636 | 155749 | 6 |
| TESTI2024299 | 0.001095278 | 195929 | 1 |
| TESTI2024419 | 0.001095278 | 273933 | 1 |
| TESTI2024422 | 0.001095278 | 140255 | 1 |
| TESTI2024443 | 0.001095278 | 173581 | 1 |
| TESTI2024446 | 0.001095278 | 130697 | 1 |
| TESTI2024473 | 0.001095278 | 171364 | 1 |
| TESTI2024476 | 0.008762226 | 173577 | 8 |
| TESTI2024498 | 0.001095278 | 204291 | 1 |
| TESTI2024560 | 0.003285835 | 180181 | 3 |
| TESTI2024567 | 0.001095278 | 132071 | 1 |
| TESTI2024586 | 0.001095278 | 216631 | 1 |
| TESTI2024598 | 0.002804738 | 115255 | 2 |
| TESTI2024641 | 0.001095278 | 171370 | 1 |
| TESTI2024648 | 0.001095278 | 175796 | 1 |
| TESTI2024718 | 0.012048061 | 149593 | 11 |
| TESTI2024744 | 0.001095278 | 129439 | 1 |
| TESTI2024844 | 0.146257055 | 38285 | 11 |
| TESTI2024864 | 0.006067616 | 128611 | 3 |
| TESTI2024885 | 0.01260011 | 77395 | 2 |
| TESTI2024936 | 0.001095278 | 114388 | 1 |
| TESTI2024978 | 0.001095278 | 163403 | 1 |
| TESTI2024999 | 0.001095278 | 124874 | 1 |
| TESTI2025006 | 0.001095278 | 65057 | 1 |
| TESTI2025013 | 0.047072946 | 117261 | 3 |
| TESTI2025022 | 0.002190557 | 111212 | 2 |
| TESTI2025063 | 0.002190557 | 133970 | 2 |
| TESTI2025117 | 0.003285835 | 171381 | 3 |
| TESTI2025144 | 0.001095278 | 175765 | 1 |
| TESTI2025161 | 0.001095278 | 165601 | 1 |
| TESTI2025174 | 0.001095278 | 144627 | 1 |
| TESTI2025229 | 0.001095278 | 161885 | 1 |
| TESTI2025269 | 0.001095278 | 51834 | 1 |
| TESTI2025365 | 0.001095278 | 156562 | 1 |
| TESTI2025403 | 0.001095278 | 79870 | 1 |
| TESTI2025409 | 0.001095278 | 224291 | 1 |
| TESTI2025422 | 0.067506217 | 174323 | 6 |
| TESTI2025448 | 0.005476391 | 77390 | 5 |
| TESTI2025454 | 0.014876357 | 131140 | 5 |
| TESTI2025486 | 0.001095278 | 257479 | 1 |
| TESTI2025499 | 0.02693841 | 109167 | 3 |
| TESTI2025546 | 0.048276743 | 117193 | 33 |
| TESTI2025582 | 0.003285835 | 122865 | 3 |
| TESTI2025609 | 0.001095278 | 70036 | 1 |
| TESTI2025656 | 0.001095278 | 152240 | 1 |
| TESTI2025757 | 0.009881811 | 129887 | 5 |
| TESTI2025791 | 0.001095278 | 179882 | 1 |
| TESTI2025846 | 0.008230586 | 91610 | 6 |
| TESTI2025872 | 0.038646514 | 171066 | 4 |
| TESTI2025879 | 0.001095278 | 146300 | 1 |
| TESTI2025911 | 0.001095278 | 52035 | 1 |
| TESTI2025920 | 0.001095278 | 32336 | 1 |
| TESTI2025924 | 0.001095278 | 165985 | 1 |
| TESTI2026014 | 0.001095278 | 145731 | 1 |
| TESTI2026024 | 0.007037402 | 107454 | 2 |
| TESTI2026064 | 0.001095278 | 179390 | 1 |
| TESTI2026077 | 0.001095278 | 44170 | 1 |
| TESTI2026104 | 0.001095278 | 48494 | 1 |
| TESTI2026116 | 0.01123402 | 23005 | 6 |
| TESTI2026134 | 0.001095278 | 100191 | 1 |
| TESTI2026168 | 0.001095278 | 260944 | 1 |
| TESTI2026196 | 0.001095278 | 180712 | 1 |
| TESTI2026215 | 0.024190153 | 115720 | 6 |
| TESTI2026218 | 0.001095278 | 187766 | 1 |
| TESTI2026233 | 0.001095278 | 196891 | 1 |
| TESTI2026291 | 0.001095278 | 276472 | 1 |
| TESTI2026294 | 0.001095278 | 181622 | 1 |
| TESTI2026401 | 0.001095278 | 241995 | 1 |
| TESTI2026403 | 0.002190557 | 192368 | 2 |
| TESTI2026453 | 0.001095278 | 284444 | 1 |
| TESTI2026461 | 0.001095278 | 179353 | 1 |
| TESTI2026491 | 0.001095278 | 175725 | 1 |
| TESTI2026505 | 0.002190557 | 220206 | 2 |
| TESTI2026515 | 0.001095278 | 185796 | 1 |
| TESTI2026525 | 0.03851191 | 187000 | 9 |
| TESTI2026534 | 0.002804738 | 182822 | 2 |
| TESTI2026537 | 0.001095278 | 149806 | 1 |
| TESTI2026589 | 0.001095278 | 155025 | 1 |
| TESTI2026597 | 0.001095278 | 111069 | 1 |
| TESTI2026605 | 0.00657167 | 173774 | 6 |
| TESTI2026647 | 0.014180569 | 159741 | 4 |
| TESTI2026674 | 0.002190557 | 88778 | 2 |
| TESTI2026794 | 0.001095278 | 166957 | 1 |
| TESTI2026824 | 0.001095278 | 175703 | 1 |
| TESTI2026854 | 0.001095278 | 175710 | 1 |
| TESTI2026925 | 0.005476391 | 107906 | 5 |
| TESTI2026936 | 0.002783555 | 135310 | 2 |
| TESTI2026957 | 0.001095278 | 154688 | 1 |
| TESTI2027013 | 0.001095278 | 213630 | 1 |
| TESTI2027019 | 0.001095278 | 210487 | 1 |
| TESTI2027108 | 0.001095278 | 180804 | 1 |
| TESTI2027165 | 0.00309368 | 52326 | 2 |
| TESTI2027171 | 0.002190557 | 204407 | 2 |
| TESTI2027173 | 0.001095278 | 55277 | 1 |
| TESTI2027179 | 0.001095278 | 67759 | 1 |
| TESTI2027185 | 0.001095278 | 203424 | 1 |
| TESTI2027206 | 0.031763069 | 120175 | 29 |
| TESTI2027238 | 0.001095278 | 203035 | 1 |
| TESTI2027239 | 0.001095278 | 75184 | 1 |
| TESTI2027243 | 0.001095278 | 278341 | 1 |
| TESTI2027271 | 0.031732543 | 39383 | 6 |
| TESTI2027296 | 0.001095278 | 158781 | 1 |
| TESTI2027378 | 0.001095278 | 212458 | 1 |
| TESTI2027411 | 0.001095278 | 15291 | 1 |
| TESTI2027450 | 0.001095278 | 153670 | 1 |
| TESTI2027496 | 0.017834284 | 153894 | 7 |
| TESTI2027503 | 0.001095278 | 143165 | 1 |
| TESTI2027562 | 0.001095278 | 215240 | 1 |
| TESTI2027615 | 0.001095278 | 226842 | 1 |
| TESTI2027645 | 0.001095278 | 27887 | 1 |
| TESTI2027730 | 0.003285835 | 201524 | 3 |
| TESTI2027736 | 0.001095278 | 143147 | 1 |
| TESTI2027791 | 0.001095278 | 276017 | 1 |
| TESTI2027820 | 0.001095278 | 256536 | 1 |
| TESTI2027828 | 0.001095278 | 72623 | 1 |
| TESTI2027840 | 0.002190557 | 179260 | 2 |
| TESTI2027909 | 0.002492434 | 155575 | 2 |
| TESTI2028098 | 0.001095278 | 215392 | 1 |
| TESTI2028151 | 0.00657167 | 182680 | 6 |
| TESTI2028242 | 0.001095278 | 224287 | 1 |
| TESTI2028253 | 0.001095278 | 130573 | 1 |
| TESTI2028254 | 0.004381113 | 142422 | 4 |
| TESTI2028269 | 0.001095278 | 200642 | 1 |
| TESTI2028290 | 0.001095278 | 272910 | 1 |
| TESTI2028296 | 0.067948504 | 104221 | 8 |
| TESTI2028384 | 0.001095278 | 31634 | 1 |
| TESTI2028426 | 0.001095278 | 244211 | 1 |
| TESTI2028488 | 0.001095278 | 208414 | 1 |
| TESTI2028523 | 0.00657167 | 140902 | 6 |
| TESTI2028583 | 0.001095278 | 140579 | 1 |
| TESTI2028613 | 0.001095278 | 261525 | 1 |
| TESTI2028659 | 0.00555852 | 114257 | 4 |
| TESTI2028662 | 0.001095278 | 232286 | 1 |
| TESTI2028670 | 0.001095278 | 123155 | 1 |
| TESTI2028705 | 0.001095278 | 222330 | 1 |
| TESTI2028776 | 0.001095278 | 154075 | 1 |
| TESTI2028811 | 0.001095278 | 226149 | 1 |
| TESTI2028891 | 0.001095278 | 48445 | 1 |
| TESTI2028985 | 0.001095278 | 139159 | 1 |
| TESTI2028997 | 0.001095278 | 221766 | 1 |
| TESTI2029131 | 0.001095278 | 158596 | 1 |
| TESTI2029162 | 0.001095278 | 191158 | 1 |
| TESTI2029196 | 0.001095278 | 145689 | 1 |
| TESTI2029201 | 0.002190557 | 185802 | 2 |
| TESTI2029227 | 0.028226519 | 104191 | 13 |
| TESTI2029244 | 0.001095278 | 161994 | 1 |
| TESTI2029249 | 0.001095278 | 149249 | 1 |
| TESTI2029252 | 0.001095278 | 157399 | 1 |
| TESTI2029259 | 0.050627645 | 14429 | 9 |
| TESTI2029264 | 0.001095278 | 46713 | 1 |
| TESTI2029470 | 0.001095278 | 130184 | 1 |
| TESTI2029672 | 0.001095278 | 233339 | 1 |
| TESTI2029785 | 0.002492434 | 145330 | 2 |
| TESTI2029880 | 0.001095278 | 240165 | 1 |
| TESTI2030136 | 0.001095278 | 180810 | 1 |
| TESTI2030322 | 0.001095278 | 139047 | 1 |
| TESTI2030336 | 0.002492434 | 192451 | 2 |
| TESTI2030342 | 0.001095278 | 265648 | 1 |
| TESTI2030519 | 0.001095278 | 271936 | 1 |
| TESTI2030554 | 0.00468299 | 128614 | 4 |
| TESTI2030556 | 0.001095278 | 202871 | 1 |
| TESTI2030728 | 0.001095278 | 175724 | 1 |
| TESTI2030754 | 0.001095278 | 180868 | 1 |
| TESTI2030770 | 0.001095278 | 48386 | 1 |
| TESTI2030860 | 0.001095278 | 171343 | 1 |
| TESTI2030901 | 0.001095278 | 230690 | 1 |
| TESTI2030917 | 0.001095278 | 128961 | 1 |
| TESTI2030930 | 0.001095278 | 47069 | 1 |
| TESTI2031006 | 0.001095278 | 37087 | 1 |
| TESTI2031007 | 0.005162676 | 118782 | 4 |
| TESTI2031060 | 0.001095278 | 43865 | 1 |
| TESTI2031129 | 0.002190557 | 173639 | 2 |
| TESTI2031356 | 0.001095278 | 139063 | 1 |
| TESTI2031418 | 0.001095278 | 171355 | 1 |
| TESTI2031504 | 0.001095278 | 109724 | 1 |
| TESTI2031529 | 0.001095278 | 40060 | 1 |
| TESTI2031687 | 0.001095278 | 36807 | 1 |
| TESTI2031760 | 0.001095278 | 180445 | 1 |
| TESTI2031809 | 0.001095278 | 133461 | 1 |
| TESTI2031919 | 0.001095278 | 52042 | 1 |
| TESTI2031935 | 0.001095278 | 155727 | 1 |
| TESTI2031986 | 0.002190557 | 133668 | 2 |
| TESTI2032044 | 0.00657167 | 102026 | 6 |
| TESTI2032067 | 0.001095278 | 167010 | 1 |
| TESTI2032079 | 0.001095278 | 226167 | 1 |
| TESTI2032643 | 0.013779115 | 228702 | 3 |
| TESTI2032681 | 0.001095278 | 141395 | 1 |
| TESTI2032761 | 0.001095278 | 242921 | 1 |
| TESTI2032768 | 0.002190557 | 179579 | 2 |
| TESTI2032774 | 0.001095278 | 259279 | 1 |
| TESTI2032828 | 0.007666948 | 172468 | 7 |
| TESTI2033031 | 0.005445826 | 56053 | 4 |
| TESTI2033242 | 0.001095278 | 108152 | 1 |
| TESTI2033254 | 0.001095278 | 74269 | 1 |
| TESTI2033300 | 0.001095278 | 122498 | 1 |
| TESTI2033395 | 0.002190557 | 135127 | 2 |
| TESTI2033441 | 0.152694729 | 109611 | 29 |
| TESTI2033453 | 0.001095278 | 119141 | 1 |
| TESTI2033505 | 0.001095278 | 64173 | 1 |
| TESTI2033520 | 0.001095278 | 222311 | 1 |
| TESTI2033641 | 0.001095278 | 223615 | 1 |
| TESTI2033710 | 0.001095278 | 220552 | 1 |
| TESTI2033905 | 0.024096122 | 131838 | 22 |
| TESTI2034187 | 0.001095278 | 245631 | 1 |
| TESTI2034243 | 0.001095278 | 222485 | 1 |
| TESTI2034249 | 0.042877709 | 73491 | 33 |
| TESTI2034251 | 0.017834284 | 153894 | 7 |
| TESTI2034307 | 0.002492434 | 136584 | 2 |
| TESTI2034357 | 0.001095278 | 19626 | 1 |
| TESTI2034401 | 0.001095278 | 273751 | 1 |
| TESTI2034407 | 0.001095278 | 54245 | 1 |
| TESTI2034506 | 0.001095278 | 107914 | 1 |
| TESTI2034520 | 0.001095278 | 207784 | 1 |
| TESTI2034718 | 0.001095278 | 134249 | 1 |
| TESTI2034730 | 0.001095278 | 115798 | 1 |
| TESTI2034749 | 0.001095278 | 189661 | 1 |
| TESTI2034767 | 0.001095278 | 187767 | 1 |
| TESTI2034774 | 0.001095278 | 189625 | 1 |
| TESTI2034777 | 0.001095278 | 192439 | 1 |
| TESTI2034785 | 0.001095278 | 207622 | 1 |
| TESTI2034799 | 0.001095278 | 189618 | 1 |
| TESTI2034847 | 0.001095278 | 170706 | 1 |
| TESTI2034913 | 0.001095278 | 206879 | 1 |
| TESTI2034916 | 0.022042071 | 104310 | 8 |
| TESTI2034931 | 0.001095278 | 74922 | 1 |
| TESTI2034940 | 0.643197739 | 34012 | 81 |
| TESTI2034953 | 0.001095278 | 142667 | 1 |
| TESTI2034997 | 0.001095278 | 170695 | 1 |
| TESTI2035003 | 0.002284523 | 58322 | 2 |
| TESTI2035078 | 0.001095278 | 167320 | 1 |
| TESTI2035084 | 0.001095278 | 138239 | 1 |
| TESTI2035107 | 0.001095278 | 175290 | 1 |
| TESTI2035121 | 0.001095278 | 191210 | 1 |
| TESTI2035124 | 0.001095278 | 170712 | 1 |
| TESTI2035183 | 0.002190557 | 68871 | 2 |
| TESTI2035236 | 0.006499784 | 77802 | 4 |
| TESTI2035262 | 0.001095278 | 207770 | 1 |
| TESTI2035309 | 0.008232122 | 152686 | 3 |
| TESTI2035437 | 0.042403927 | 141356 | 5 |
| TESTI2035502 | 0.012741189 | 179389 | 5 |
| TESTI2035688 | 0.001095278 | 9973 | 1 |
| TESTI2035775 | 0.001095278 | 238937 | 1 |
| TESTI2035793 | 0.002190557 | 19057 | 2 |
| TESTI2035796 | 0.011116458 | 205584 | 6 |
| TESTI2035898 | 0.001095278 | 277946 | 1 |
| TESTI2035962 | 0.001095278 | 199700 | 1 |
| TESTI2035981 | 0.001095278 | 217343 | 1 |
| TESTI2035997 | 0.001095278 | 208280 | 1 |
| TESTI2036114 | 0.001095278 | 49273 | 1 |
| TESTI2036129 | 0.001095278 | 170707 | 1 |
| TESTI2036285 | 0.001095278 | 199673 | 1 |
| TESTI2036288 | 0.001095278 | 180139 | 1 |
| TESTI2036513 | 0.001095278 | 229885 | 1 |
| TESTI2036589 | 0.001095278 | 235625 | 1 |
| TESTI2036650 | 0.002783555 | 23897 | 2 |
| TESTI2036684 | 0.001095278 | 221277 | 1 |
| TESTI2036691 | 0.007136844 | 115567 | 2 |
| TESTI2036736 | 0.001095278 | 200647 | 1 |
| TESTI2036822 | 0.001095278 | 183034 | 1 |
| TESTI2036833 | 0.001095278 | 138314 | 1 |
| TESTI2036913 | 0.008256356 | 48491 | 6 |
| TESTI2036922 | 0.001095278 | 237809 | 1 |
| TESTI2036951 | 0.002190557 | 120405 | 2 |
| TESTI2036965 | 0.001095278 | 104134 | 1 |
| TESTI2036969 | 0.046634682 | 122870 | 22 |
| TESTI2037002 | 0.001095278 | 141140 | 1 |
| TESTI2037055 | 0.001095278 | 220318 | 1 |
| TESTI2037081 | 0.002190557 | 157400 | 2 |
| TESTI2037085 | 0.001095278 | 218010 | 1 |
| TESTI2037103 | 0.001095278 | 39845 | 1 |
| TESTI2037106 | 0.001095278 | 132156 | 1 |
| TESTI2037111 | 0.001095278 | 220289 | 1 |
| TESTI2037209 | 0.001095278 | 220295 | 1 |
| TESTI2037255 | 0.001095278 | 221426 | 1 |
| TESTI2037359 | 0.001095278 | 125665 | 1 |
| TESTI2037375 | 0.001095278 | 156597 | 1 |
| TESTI2037382 | 0.001095278 | 229818 | 1 |
| TESTI2037534 | 0.001095278 | 111203 | 1 |
| TESTI2037569 | 0.001095278 | 144342 | 1 |
| TESTI2037572 | 0.001095278 | 213320 | 1 |
| TESTI2037643 | 0.001095278 | 170851 | 1 |
| TESTI2037657 | 0.010952783 | 158273 | 10 |
| TESTI2037673 | 0.001095278 | 141842 | 1 |
| TESTI2037723 | 0.001095278 | 152070 | 1 |
| TESTI2037819 | 0.001095278 | 111918 | 1 |
| TESTI2037830 | 0.001095278 | 175531 | 1 |
| TESTI2037845 | 0.003285835 | 175262 | 3 |
| TESTI2037877 | 0.001095278 | 161682 | 1 |
| TESTI2037976 | 0.001095278 | 258240 | 1 |
| TESTI2038048 | 0.001095278 | 45046 | 1 |
| TESTI2038065 | 0.001095278 | 185710 | 1 |
| TESTI2038104 | 0.001095278 | 101689 | 1 |
| TESTI2038275 | 0.001095278 | 111829 | 1 |
| TESTI2038388 | 0.001095278 | 173886 | 1 |
| TESTI2038573 | 0.001095278 | 220445 | 1 |
| TESTI2038596 | 0.001095278 | 222681 | 1 |
| TESTI2038623 | 0.001095278 | 142700 | 1 |
| TESTI2038644 | 0.001095278 | 220472 | 1 |
| TESTI2038733 | 0.001095278 | 182610 | 1 |
| TESTI2038858 | 0.001095278 | 194534 | 1 |
| TESTI2038958 | 0.001095278 | 212921 | 1 |
| TESTI2039025 | 0.001095278 | 43642 | 1 |
| TESTI2039026 | 0.001095278 | 224310 | 1 |
| TESTI2039038 | 0.001095278 | 100266 | 1 |
| TESTI2039041 | 0.001095278 | 236560 | 1 |
| TESTI2039060 | 0.001095278 | 217562 | 1 |
| TESTI2039113 | 0.001095278 | 185855 | 1 |
| TESTI2039121 | 0.001095278 | 220499 | 1 |
| TESTI2039177 | 0.040429071 | 123408 | 25 |
| TESTI2039209 | 0.001095278 | 161758 | 1 |
| TESTI2039225 | 0.001095278 | 118795 | 1 |
| TESTI2039227 | 0.001095278 | 166589 | 1 |
| TESTI2039276 | 0.001095278 | 23784 | 1 |
| TESTI2039342 | 0.004811507 | 99594 | 3 |
| TESTI2039353 | 0.001095278 | 249300 | 1 |
| TESTI2039544 | 0.001095278 | 213203 | 1 |
| TESTI2039613 | 0.001095278 | 198340 | 1 |
| TESTI2039707 | 0.007297598 | 43760 | 2 |
| TESTI2039732 | 0.001095278 | 166678 | 1 |
| TESTI2039738 | 0.001095278 | 165065 | 1 |
| TESTI2039776 | 0.001095278 | 148658 | 1 |
| TESTI2039867 | 0.001095278 | 167960 | 1 |
| TESTI2039908 | 0.002190557 | 173907 | 2 |
| TESTI2040018 | 0.002190557 | 82392 | 2 |
| TESTI2040094 | 0.01361876 | 180254 | 2 |
| TESTI2040102 | 0.001095278 | 196367 | 1 |
| TESTI2040128 | 0.001095278 | 180736 | 1 |
| TESTI2040143 | 0.001095278 | 19137 | 1 |
| TESTI2040297 | 0.001095278 | 166671 | 1 |
| TESTI2040372 | 0.001095278 | 235885 | 1 |
| TESTI2040377 | 0.002190557 | 234525 | 2 |
| TESTI2040424 | 0.001095278 | 48663 | 1 |
| TESTI2040642 | 0.001095278 | 125806 | 1 |
| TESTI2040815 | 0.001095278 | 206869 | 1 |
| TESTI2040897 | 0.001095278 | 127194 | 1 |
| TESTI2040944 | 0.001095278 | 161674 | 1 |
| TESTI2040964 | 0.001095278 | 173889 | 1 |
| TESTI2040983 | 0.001095278 | 122349 | 1 |
| TESTI2040989 | 0.001095278 | 180744 | 1 |
| TESTI2041330 | 0.001095278 | 263218 | 1 |
| TESTI2041362 | 0.001095278 | 207802 | 1 |
| TESTI2041517 | 0.001095278 | 212840 | 1 |
| TESTI2041564 | 0.001095278 | 212980 | 1 |
| TESTI2041664 | 0.001095278 | 106672 | 1 |
| TESTI2041730 | 0.001095278 | 191229 | 1 |
| TESTI2041734 | 0.004272069 | 128030 | 3 |
| TESTI2041778 | 0.001095278 | 113289 | 1 |
| TESTI2041800 | 0.001095278 | 69935 | 1 |
| TESTI2041947 | 0.002190557 | 191391 | 2 |
| TESTI2041956 | 0.001095278 | 200171 | 1 |
| TESTI2041976 | 0.002190557 | 199164 | 2 |
| TESTI2042062 | 0.001095278 | 74575 | 1 |
| TESTI2042149 | 0.001095278 | 170073 | 1 |
| TESTI2042264 | 0.001095278 | 117549 | 1 |
| TESTI2042295 | 0.001095278 | 218024 | 1 |
| TESTI2042302 | 0.001095278 | 113463 | 1 |
| TESTI2042326 | 0.001095278 | 13365 | 1 |
| TESTI2042400 | 0.001095278 | 206278 | 1 |
| TESTI2042450 | 0.001095278 | 281398 | 1 |
| TESTI2042473 | 0.001095278 | 65975 | 1 |
| TESTI2042507 | 0.001095278 | 208148 | 1 |
| TESTI2042508 | 0.001095278 | 23236 | 1 |
| TESTI2042750 | 0.001095278 | 49432 | 1 |
| TESTI2042783 | 0.001095278 | 242809 | 1 |
| TESTI2042806 | 0.001095278 | 202323 | 1 |
| TESTI2042928 | 0.001095278 | 191234 | 1 |
| TESTI2042946 | 0.001095278 | 182670 | 1 |
| TESTI2042958 | 0.003285835 | 175797 | 3 |
| TESTI2043275 | 0.018936539 | 229966 | 3 |
| TESTI2043277 | 0.001095278 | 124130 | 1 |
| TESTI2043282 | 0.001095278 | 198169 | 1 |
| TESTI2043313 | 0.001095278 | 217286 | 1 |
| TESTI2043477 | 0.002190557 | 73800 | 2 |
| TESTI2043585 | 0.001095278 | 177670 | 1 |
| TESTI2043656 | 0.00297212 | 2682 | 2 |
| TESTI2043758 | 0.001095278 | 100820 | 1 |
| TESTI2043817 | 0.001095278 | 284823 | 1 |
| TESTI2043857 | 0.001095278 | 195055 | 1 |
| TESTI2043866 | 0.002190557 | 195951 | 2 |
| TESTI2043869 | 0.002190557 | 211069 | 2 |
| TESTI2044064 | 0.001095278 | 208637 | 1 |
| TESTI2044118 | 0.001095278 | 19320 | 1 |
| TESTI2044194 | 0.001095278 | 31478 | 1 |
| TESTI2044276 | 0.035334498 | 111254 | 16 |
| TESTI2044306 | 0.001095278 | 182666 | 1 |
| TESTI2044309 | 0.001095278 | 152986 | 1 |
| TESTI2044413 | 0.001095278 | 223607 | 1 |
| TESTI2044418 | 0.001095278 | 166930 | 1 |
| TESTI2044754 | 0.001095278 | 136190 | 1 |
| TESTI2044788 | 0.00309368 | 113426 | 2 |
| TESTI2044796 | 0.001095278 | 185715 | 1 |
| TESTI2044833 | 0.001095278 | 196044 | 1 |
| TESTI2044892 | 0.001095278 | 219603 | 1 |
| TESTI2044920 | 0.001095278 | 123073 | 1 |
| TESTI2044968 | 0.001095278 | 128490 | 1 |
| TESTI2045139 | 0.001095278 | 166945 | 1 |
| TESTI2045155 | 0.014546877 | 176527 | 3 |
| TESTI2045171 | 0.001095278 | 177741 | 1 |
| TESTI2045199 | 0.001095278 | 163612 | 1 |
| TESTI2045353 | 0.001095278 | 196591 | 1 |
| TESTI2045509 | 0.001095278 | 187731 | 1 |
| TESTI2045562 | 0.007136844 | 64524 | 2 |
| TESTI2045611 | 0.001095278 | 126797 | 1 |
| TESTI2045819 | 0.001095278 | 191193 | 1 |
| TESTI2045850 | 0.001095278 | 140324 | 1 |
| TESTI2045920 | 0.001095278 | 202494 | 1 |
| TESTI2045964 | 0.001095278 | 21254 | 1 |
| TESTI2045983 | 0.001095278 | 150606 | 1 |
| TESTI2046100 | 0.001095278 | 123870 | 1 |
| TESTI2046188 | 0.001095278 | 223197 | 1 |
| TESTI2046347 | 0.001095278 | 280511 | 1 |
| TESTI2046352 | 0.001095278 | 213684 | 1 |
| TESTI2046358 | 0.001095278 | 58017 | 1 |
| TESTI2046439 | 0.003285835 | 210508 | 3 |
| TESTI2046456 | 0.361985383 | 75965 | 82 |
| TESTI2046535 | 0.001095278 | 46211 | 1 |
| TESTI2046536 | 0.001095278 | 37068 | 1 |
| TESTI2046552 | 0.001095278 | 122071 | 1 |
| TESTI2046569 | 0.001095278 | 79570 | 1 |
| TESTI2046686 | 0.002190557 | 144123 | 2 |
| TESTI2046721 | 0.013689737 | 122896 | 2 |
| TESTI2046732 | 0.001095278 | 9151 | 1 |
| TESTI2046786 | 0.001095278 | 32053 | 1 |
| TESTI2046797 | 0.002190557 | 211229 | 2 |
| TESTI2046863 | 0.001095278 | 193133 | 1 |
| TESTI2046872 | 0.001095278 | 42985 | 1 |
| TESTI2047071 | 0.001095278 | 249934 | 1 |
| TESTI2047141 | 0.007666948 | 67646 | 7 |
| TESTI2047147 | 0.001095278 | 232760 | 1 |
| TESTI2047153 | 0.001095278 | 234778 | 1 |
| TESTI2047212 | 0.001095278 | 245491 | 1 |
| TESTI2047342 | 0.001095278 | 138514 | 1 |
| TESTI2047383 | 0.001095278 | 133979 | 1 |
| TESTI2047605 | 0.01149927 | 165372 | 5 |
| TESTI2047792 | 0.001095278 | 151700 | 1 |
| TESTI2047801 | 0.001095278 | 207856 | 1 |
| TESTI2047818 | 0.002190557 | 142677 | 2 |
| TESTI2047824 | 0.001095278 | 278834 | 1 |
| TESTI2047885 | 0.001095278 | 216740 | 1 |
| TESTI2047930 | 0.001095278 | 194556 | 1 |
| TESTI2048105 | 0.001095278 | 213331 | 1 |
| TESTI2048109 | 0.001095278 | 265230 | 1 |
| TESTI2048465 | 0.001095278 | 246943 | 1 |
| TESTI2048603 | 0.001095278 | 264089 | 1 |
| TESTI2048898 | 0.003285835 | 252442 | 3 |
| TESTI2049041 | 0.001095278 | 87253 | 1 |
| TESTI2049062 | 0.001095278 | 162294 | 1 |
| TESTI2049206 | 0.001095278 | 269564 | 1 |
| TESTI2049246 | 0.006372313 | 99342 | 4 |
| TESTI2049277 | 0.001095278 | 266096 | 1 |
| TESTI2049422 | 0.001095278 | 279530 | 1 |
| TESTI2049452 | 0.001095278 | 264249 | 1 |
| TESTI2049469 | 0.001095278 | 189312 | 1 |
| TESTI2049553 | 0.001095278 | 12055 | 1 |
| TESTI2049576 | 0.001095278 | 282334 | 1 |
| TESTI2049857 | 0.001095278 | 272463 | 1 |
| TESTI2049956 | 0.001095278 | 262002 | 1 |
| TESTI2050137 | 0.001095278 | 141968 | 1 |
| TESTI2050681 | 0.001095278 | 62563 | 1 |
| TESTI2050780 | 0.001095278 | 270964 | 1 |
| TESTI2050987 | 0.001095278 | 262730 | 1 |
| TESTI2051148 | 0.001095278 | 262795 | 1 |
| TESTI2051177 | 0.00700754 | 41869 | 2 |
| TESTI2051279 | 0.001095278 | 266567 | 1 |
| TESTI2051488 | 0.001095278 | 275569 | 1 |
| TESTI2051543 | 0.001095278 | 242256 | 1 |
| TESTI2051627 | 0.001095278 | 245246 | 1 |
| TESTI2051742 | 0.001095278 | 179082 | 1 |
| TESTI2051767 | 0.001095278 | 259239 | 1 |
| TESTI2051791 | 0.001095278 | 263829 | 1 |
| TESTI2051806 | 0.001095278 | 252533 | 1 |
| TESTI2051867 | 0.003285835 | 200798 | 3 |
| TESTI2051873 | 0.001095278 | 262760 | 1 |
| TESTI2052110 | 0.001095278 | 250638 | 1 |
| TESTI2052202 | 0.001095278 | 211683 | 1 |
| TESTI2052211 | 0.001095278 | 248949 | 1 |
| TESTI2052256 | 0.001095278 | 261859 | 1 |
| TESTI2052288 | 0.001095278 | 244427 | 1 |
| TESTI2052525 | 0.001095278 | 258269 | 1 |
| TESTI2052670 | 0.001095278 | 259276 | 1 |
| TESTI2052693 | 0.100082003 | 187540 | 5 |
| TESTI2052698 | 0.001095278 | 138340 | 1 |
| TESTI2052799 | 0.001095278 | 252496 | 1 |
| TESTI2052822 | 0.001095278 | 270804 | 1 |
| TESTI2052985 | 0.001095278 | 256967 | 1 |
| TESTI2053151 | 0.001095278 | 251816 | 1 |
| TESTI2053242 | 0.001095278 | 259085 | 1 |
| TESTI2053399 | 0.001095278 | 21602 | 1 |
| TESTI2053526 | 0.001095278 | 256794 | 1 |
| TESTI2053561 | 0.001095278 | 249163 | 1 |
| TESTI2053621 | 0.001095278 | 130026 | 1 |
| TESTI2053667 | 0.00297212 | 151109 | 2 |
| TESTI2053723 | 0.03701313 | 120236 | 13 |
| TESTI3000002 | 0.001095278 | 39924 | 1 |
| TESTI3000005 | 0.026286678 | 52760 | 24 |
| TESTI4000014 | 0.938263392 | 60637 | 166 |
| TESTI4000068 | 0.001095278 | 153102 | 1 |
| TESTI4000079 | 0.001095278 | 61247 | 1 |
| TESTI4000093 | 0.004381113 | 158843 | 4 |
| TESTI4000134 | 0.001095278 | 200245 | 1 |
| TESTI4000137 | 0.001095278 | 109195 | 1 |
| TESTI4000153 | 0.001095278 | 187359 | 1 |
| TESTI4000155 | 0.001095278 | 204925 | 1 |
| TESTI4000157 | 0.001095278 | 192960 | 1 |
| TESTI4000183 | 0.001095278 | 194326 | 1 |
| TESTI4000209 | 0.06325171 | 153121 | 16 |
| TESTI4000214 | 0.021424469 | 160960 | 19 |
| TESTI4000215 | 0.001095278 | 165944 | 1 |
| TESTI4000228 | 0.001095278 | 49078 | 1 |
| TESTI4000250 | 0.001095278 | 197001 | 1 |
| TESTI4000288 | 0.001095278 | 159819 | 1 |
| TESTI4000319 | 0.003285835 | 188030 | 3 |
| TESTI4000349 | 0.006445887 | 72087 | 5 |
| TESTI4000370 | 0.001095278 | 179356 | 1 |
| TESTI4000394 | 0.001095278 | 141931 | 1 |
| TESTI4000434 | 0.012814546 | 49772 | 10 |
| TESTI4000462 | 0.003285835 | 51147 | 3 |
| TESTI4000530 | 0.002190557 | 194577 | 2 |
| TESTI4000534 | 0.001095278 | 215053 | 1 |
| TESTI4000598 | 0.001095278 | 251449 | 1 |
| TESTI4000600 | 0.002190557 | 92886 | 2 |
| TESTI4000621 | 0.001095278 | 261595 | 1 |
| TESTI4000703 | 0.001095278 | 260353 | 1 |
| TESTI4000724 | 0.001095278 | 121180 | 1 |
| TESTI4000957 | 0.001095278 | 187323 | 1 |
| TESTI4000970 | 0.003285835 | 5741 | 3 |
| TESTI4001036 | 0.001095278 | 263559 | 1 |
| TESTI4001037 | 0.001095278 | 132551 | 1 |
| TESTI4001060 | 0.001095278 | 266434 | 1 |
| TESTI4001100 | 0.040891613 | 121038 | 17 |
| TESTI4001106 | 0.001095278 | 124278 | 1 |
| TESTI4001148 | 0.001095278 | 240884 | 1 |
| TESTI4001176 | 0.001095278 | 245516 | 1 |
| TESTI4001195 | 0.001095278 | 240608 | 1 |
| TESTI4001201 | 0.001095278 | 249981 | 1 |
| TESTI4001206 | 0.004010135 | 191516 | 2 |
| TESTI4001348 | 0.001095278 | 217118 | 1 |
| TESTI4001441 | 0.001095278 | 42952 | 1 |
| TESTI4001467 | 0.030264738 | 59805 | 7 |
| TESTI4001473 | 0.001095278 | 273886 | 1 |
| TESTI4001517 | 0.001095278 | 161876 | 1 |
| TESTI4001527 | 0.001095278 | 22471 | 1 |
| TESTI4001561 | 0.004161364 | 192332 | 3 |
| TESTI4001569 | 0.003900017 | 135865 | 3 |
| TESTI4001604 | 0.005758131 | 245334 | 4 |
| TESTI4001665 | 0.001095278 | 232605 | 1 |
| TESTI4001679 | 0.001095278 | 193021 | 1 |
| TESTI4001795 | 0.007666948 | 170563 | 7 |
| TESTI4001923 | 0.001095278 | 236152 | 1 |
| TESTI4001925 | 0.001095278 | 207691 | 1 |
| TESTI4001984 | 0.002770825 | 241338 | 2 |
| TESTI4002003 | 0.003285835 | 192128 | 3 |
| TESTI4002072 | 0.05017915 | 126605 | 27 |
| TESTI4002141 | 0.001095278 | 270506 | 1 |
| TESTI4002195 | 0.0250447 | 114499 | 6 |
| TESTI4002202 | 0.050541264 | 124222 | 14 |
| TESTI4002290 | 0.002953393 | 216458 | 2 |
| TESTI4002319 | 0.001095278 | 260838 | 1 |
| TESTI4002491 | 0.001095278 | 261542 | 1 |
| TESTI4002520 | 0.001095278 | 282980 | 1 |
| TESTI4002552 | 0.001095278 | 78622 | 1 |
| TESTI4002647 | 0.00317679 | 157852 | 2 |
| TESTI4002703 | 0.001095278 | 265261 | 1 |
| TESTI4002754 | 0.001095278 | 256679 | 1 |
| TESTI4002774 | 0.040839417 | 65581 | 12 |
| TESTI4002799 | 0.379400289 | 77233 | 51 |
| TESTI4002868 | 0.003285835 | 166368 | 3 |
| TESTI4002878 | 0.001095278 | 222020 | 1 |
| TESTI4002889 | 0.001095278 | 259192 | 1 |
| TESTI4002988 | 0.007726483 | 78207 | 5 |
| TESTI4003150 | 0.01128277 | 100256 | 7 |
| TESTI4003179 | 0.004067398 | 43813 | 3 |
| TESTI4003279 | 0.001095278 | 216493 | 1 |
| TESTI4003319 | 0.002190557 | 253395 | 2 |
| TESTI4003404 | 0.001095278 | 214633 | 1 |
| TESTI4003565 | 0.002190557 | 157103 | 2 |
| TESTI4003574 | 0.001095278 | 230059 | 1 |
| TESTI4003579 | 0.00297212 | 175467 | 2 |
| TESTI4003602 | 0.006452503 | 126936 | 5 |
| TESTI4003703 | 0.037866701 | 118926 | 19 |
| TESTI4003733 | 0.001095278 | 241415 | 1 |
| TESTI4003796 | 0.001095278 | 255051 | 1 |
| TESTI4003944 | 0.00480314 | 77460 | 3 |
| TESTI4004003 | 0.001095278 | 261558 | 1 |
| TESTI4004031 | 0.001095278 | 149332 | 1 |
| TESTI4004200 | 0.001095278 | 250967 | 1 |
| TESTI4004210 | 0.004647667 | 125473 | 3 |
| TESTI4004539 | 0.001095278 | 59137 | 1 |
| TESTI4004626 | 0.010177686 | 191821 | 5 |
| TESTI4004653 | 0.001095278 | 13268 | 1 |
| TESTI4004695 | 0.005143949 | 86266 | 4 |
| TESTI4004722 | 0.001095278 | 238982 | 1 |
| TESTI4004887 | 0.001095278 | 20521 | 1 |
| TESTI4004917 | 0.003285835 | 188177 | 3 |
| TESTI4005013 | 0.010253348 | 101332 | 9 |
| TESTI4005015 | 0.001095278 | 254907 | 1 |
| TESTI4005039 | 0.014238617 | 123537 | 13 |
| TESTI4005133 | 0.014051214 | 146299 | 9 |
| TESTI4005158 | 0.001095278 | 228107 | 1 |
| TESTI4005317 | 0.001095278 | 142461 | 1 |
| TESTI4005322 | 0.001095278 | 179829 | 1 |
| TESTI4005399 | 0.021489255 | 154134 | 14 |
| TESTI4005470 | 0.004381113 | 164571 | 4 |
| TESTI4005500 | 0.001095278 | 78218 | 1 |
| TESTI4005534 | 0.006008537 | 66733 | 3 |
| TESTI4005543 | 0.001095278 | 116823 | 1 |
| TESTI4005628 | 0.001095278 | 259280 | 1 |
| TESTI4005635 | 0.010272745 | 150737 | 7 |
| TESTI4005653 | 0.337008866 | 34785 | 103 |
| TESTI4005801 | 0.001095278 | 260835 | 1 |
| TESTI4005805 | 0.001095278 | 264025 | 1 |
| TESTI4005857 | 0.005272213 | 14686 | 2 |
| TESTI4005961 | 0.001095278 | 71049 | 1 |
| TESTI4006053 | 0.001095278 | 259289 | 1 |
| TESTI4006079 | 0.001095278 | 260348 | 1 |
| TESTI4006112 | 0.001095278 | 264771 | 1 |
| TESTI4006137 | 0.006505781 | 138977 | 4 |
| TESTI4006148 | 0.001095278 | 231523 | 1 |
| TESTI4006219 | 0.001095278 | 259178 | 1 |
| TESTI4006234 | 0.001095278 | 251254 | 1 |
| TESTI4006247 | 0.013932163 | 158385 | 7 |
| TESTI4006308 | 0.001095278 | 22166 | 1 |
| TESTI4006326 | 0.004272069 | 142505 | 3 |
| TESTI4006393 | 0.003285835 | 97456 | 3 |
| TESTI4006407 | 0.002953393 | 212390 | 2 |
| TESTI4006412 | 0.003285835 | 215151 | 3 |
| TESTI4006420 | 0.001095278 | 54230 | 1 |
| TESTI4006441 | 0.002770825 | 275980 | 2 |
| TESTI4006473 | 0.001095278 | 74239 | 1 |
| TESTI4006539 | 0.001095278 | 266692 | 1 |
| TESTI4006546 | 0.002190557 | 206213 | 2 |
| TESTI4006567 | 0.001095278 | 57055 | 1 |
| TESTI4006704 | 0.001095278 | 157830 | 1 |
| TESTI4006728 | 0.001095278 | 215677 | 1 |
| TESTI4006802 | 0.001095278 | 180893 | 1 |
| TESTI4006819 | 0.004067398 | 149915 | 3 |
| TESTI4007064 | 0.001095278 | 245587 | 1 |
| TESTI4007163 | 0.003285835 | 93467 | 3 |
| TESTI4007176 | 0.001095278 | 243841 | 1 |
| TESTI4007203 | 0.001095278 | 236230 | 1 |
| TESTI4007239 | 0.007666948 | 193028 | 7 |
| TESTI4007369 | 0.012855833 | 8208 | 2 |
| TESTI4007373 | 0.001095278 | 63092 | 1 |
| TESTI4007382 | 0.004381113 | 180442 | 4 |
| TESTI4007404 | 0.001095278 | 203957 | 1 |
| TESTI4007489 | 0.002190557 | 257961 | 2 |
| TESTI4007565 | 0.001095278 | 284452 | 1 |
| TESTI4007671 | 0.001095278 | 264174 | 1 |
| TESTI4007775 | 0.002190557 | 201476 | 2 |
| TESTI4007778 | 0.001095278 | 248467 | 1 |
| TESTI4007799 | 0.001095278 | 255217 | 1 |
| TESTI4007810 | 0.001095278 | 258571 | 1 |
| TESTI4007816 | 0.038684199 | 144528 | 3 |
| TESTI4007965 | 0.009284109 | 79512 | 6 |
| TESTI4008007 | 0.001095278 | 262093 | 1 |
| TESTI4008018 | 0.004216085 | 34035 | 2 |
| TESTI4008050 | 0.001095278 | 261748 | 1 |
| TESTI4008058 | 0.019010056 | 126677 | 8 |
| TESTI4008086 | 0.001095278 | 264856 | 1 |
| TESTI4008097 | 0.002770825 | 161715 | 2 |
| TESTI4008176 | 0.001095278 | 15372 | 1 |
| TESTI4008209 | 0.001095278 | 253718 | 1 |
| TESTI4008219 | 0.001095278 | 266870 | 1 |
| TESTI4008250 | 0.002190557 | 242479 | 2 |
| TESTI4008302 | 0.001095278 | 166280 | 1 |
| TESTI4008305 | 0.001095278 | 268497 | 1 |
| TESTI4008401 | 0.001095278 | 185692 | 1 |
| TESTI4008417 | 0.001095278 | 149779 | 1 |
| TESTI4008429 | 0.001095278 | 282235 | 1 |
| TESTI4008573 | 0.001095278 | 258324 | 1 |
| TESTI4008797 | 0.01348075 | 61664 | 9 |
| TESTI4008816 | 0.001095278 | 258200 | 1 |
| TESTI4008840 | 0.001095278 | 265030 | 1 |
| TESTI4008935 | 0.001095278 | 52296 | 1 |
| TESTI4008993 | 0.001095278 | 241438 | 1 |
| TESTI4009022 | 0.001095278 | 213560 | 1 |
| TESTI4009028 | 0.001095278 | 260812 | 1 |
| TESTI4009034 | 0.001095278 | 258458 | 1 |
| TESTI4009123 | 0.001095278 | 161874 | 1 |
| TESTI4009160 | 0.001095278 | 223104 | 1 |
| TESTI4009215 | 0.001095278 | 211837 | 1 |
| TESTI4009283 | 0.001095278 | 271702 | 1 |
| TESTI4009286 | 0.231137289 | 35424 | 40 |
| TESTI4009306 | 0.001095278 | 233916 | 1 |
| TESTI4009374 | 0.001095278 | 170997 | 1 |
| TESTI4009406 | 0.001095278 | 173756 | 1 |
| TESTI4009454 | 0.041689175 | 134363 | 14 |
| TESTI4009457 | 0.006257955 | 70902 | 5 |
| TESTI4009501 | 0.001095278 | 250496 | 1 |
| TESTI4009563 | 0.001095278 | 186154 | 1 |
| TESTI4009608 | 0.001095278 | 90338 | 1 |
| TESTI4009638 | 0.001095278 | 259231 | 1 |
| TESTI4009752 | 0.001095278 | 259300 | 1 |
| TESTI4009881 | 0.001095278 | 192460 | 1 |
| TESTI4009886 | 0.001095278 | 257087 | 1 |
| TESTI4009928 | 0.001095278 | 244272 | 1 |
| TESTI4009973 | 0.176782467 | 101801 | 29 |
| TESTI4010076 | 0.001095278 | 266938 | 1 |
| TESTI4010082 | 0.001095278 | 240485 | 1 |
| TESTI4010095 | 0.001095278 | 195931 | 1 |
| TESTI4010211 | 0.001095278 | 260383 | 1 |
| TESTI4010377 | 0.002804738 | 254442 | 2 |
| TESTI4010382 | 0.016272096 | 186082 | 7 |
| TESTI4010475 | 0.001095278 | 257010 | 1 |
| TESTI4010544 | 0.003285835 | 143857 | 3 |
| TESTI4010713 | 0.002190557 | 244611 | 2 |
| TESTI4010721 | 0.001095278 | 41193 | 1 |
| TESTI4010789 | 0.001095278 | 253923 | 1 |
| TESTI4010817 | 0.004381113 | 170437 | 4 |
| TESTI4010831 | 0.001095278 | 232300 | 1 |
| TESTI4010851 | 0.306486226 | 29256 | 90 |
| TESTI4010902 | 0.001095278 | 221968 | 1 |
| TESTI4010928 | 0.004995295 | 166362 | 4 |
| TESTI4010979 | 0.008527483 | 179607 | 4 |
| TESTI4011070 | 0.008762226 | 126026 | 8 |
| TESTI4011072 | 0.001095278 | 285513 | 1 |
| TESTI4011084 | 0.002804738 | 244139 | 2 |
| TESTI4011118 | 0.001095278 | 263136 | 1 |
| TESTI4011161 | 0.007952337 | 82076 | 5 |
| TESTI4011246 | 0.001095278 | 245854 | 1 |
| TESTI4011484 | 0.001095278 | 78263 | 1 |
| TESTI4011505 | 0.001095278 | 269537 | 1 |
| TESTI4011532 | 0.001095278 | 134326 | 1 |
| TESTI4011616 | 0.001095278 | 247898 | 1 |
| TESTI4011744 | 0.001095278 | 272566 | 1 |
| TESTI4011745 | 0.001095278 | 242070 | 1 |
| TESTI4011829 | 0.001095278 | 254628 | 1 |
| TESTI4011926 | 0.001095278 | 253338 | 1 |
| TESTI4011956 | 0.001095278 | 257599 | 1 |
| TESTI4012010 | 0.001095278 | 114228 | 1 |
| TESTI4012086 | 0.001095278 | 225766 | 1 |
| TESTI4012258 | 0.001095278 | 223569 | 1 |
| TESTI4012293 | 0.001095278 | 282037 | 1 |
| TESTI4012329 | 0.001095278 | 264163 | 1 |
| TESTI4012382 | 0.001095278 | 268140 | 1 |
| TESTI4012406 | 0.001095278 | 13070 | 1 |
| TESTI4012448 | 0.001095278 | 276934 | 1 |
| TESTI4012451 | 0.002953393 | 61924 | 2 |
| TESTI4012505 | 0.001095278 | 96659 | 1 |
| TESTI4012556 | 0.002190557 | 188194 | 2 |
| TESTI4012623 | 0.001095278 | 259204 | 1 |
| TESTI4012646 | 0.002770825 | 74897 | 2 |
| TESTI4012679 | 0.001095278 | 251963 | 1 |
| TESTI4012702 | 0.076863312 | 110087 | 22 |
| TESTI4012911 | 0.002190557 | 233430 | 2 |
| TESTI4012956 | 0.001095278 | 253382 | 1 |
| TESTI4012960 | 0.011253356 | 212425 | 4 |
| TESTI4013365 | 0.002190557 | 185163 | 2 |
| TESTI4013369 | 0.001095278 | 266716 | 1 |
| TESTI4013410 | 0.003285835 | 115097 | 3 |
| TESTI4013441 | 1.068316129 | 98128 | 81 |
| TESTI4013474 | 0.162165048 | 98488 | 26 |
| TESTI4013597 | 0.076598619 | 6825 | 26 |
| TESTI4013602 | 0.001095278 | 264914 | 1 |
| TESTI4013667 | 0.001095278 | 250425 | 1 |
| TESTI4013675 | 0.01697089 | 188529 | 11 |
| TESTI4013685 | 0.001095278 | 134025 | 1 |
| TESTI4013735 | 0.00297212 | 256504 | 2 |
| TESTI4013742 | 0.001095278 | 238119 | 1 |
| TESTI4013774 | 0.001095278 | 272781 | 1 |
| TESTI4013817 | 0.006168562 | 84751 | 4 |
| TESTI4013830 | 0.001095278 | 236696 | 1 |
| TESTI4013852 | 0.001095278 | 253280 | 1 |
| TESTI4013894 | 0.011963585 | 136773 | 8 |
| TESTI4013924 | 0.001095278 | 254302 | 1 |
| TESTI4013960 | 0.001095278 | 251888 | 1 |
| TESTI4013962 | 0.001095278 | 259273 | 1 |
| TESTI4014133 | 0.09727873 | 61927 | 23 |
| TESTI4014159 | 0.014295594 | 153848 | 9 |
| TESTI4014175 | 0.006272286 | 108415 | 3 |
| TESTI4014262 | 0.042877709 | 73491 | 33 |
| TESTI4014265 | 0.001095278 | 238115 | 1 |
| TESTI4014276 | 0.001095278 | 238113 | 1 |
| TESTI4014306 | 0.002284523 | 244580 | 2 |
| TESTI4014392 | 0.002190557 | 211692 | 2 |
| TESTI4014415 | 0.002190557 | 199374 | 2 |
| TESTI4014445 | 0.002190557 | 229547 | 2 |
| TESTI4014553 | 0.003473767 | 183813 | 3 |
| TESTI4014661 | 0.001095278 | 238087 | 1 |
| TESTI4014694 | 0.150353417 | 186725 | 5 |
| TESTI4014801 | 0.003285835 | 222939 | 3 |
| TESTI4014818 | 0.002804738 | 249997 | 2 |
| TESTI4014891 | 0.001095278 | 194526 | 1 |
| TESTI4014908 | 0.004647667 | 113477 | 3 |
| TESTI4014924 | 0.001095278 | 68375 | 1 |
| TESTI4014932 | 0.002190557 | 218144 | 2 |
| TESTI4014977 | 0.002190557 | 138350 | 2 |
| TESTI4015012 | 0.008762226 | 203956 | 8 |
| TESTI4015129 | 0.001095278 | 238860 | 1 |
| TESTI4015145 | 0.001095278 | 22049 | 1 |
| TESTI4015204 | 0.006381331 | 61487 | 4 |
| TESTI4015263 | 0.001095278 | 239895 | 1 |
| TESTI4015293 | 0.002190557 | 171444 | 2 |
| TESTI4015299 | 0.001095278 | 261326 | 1 |
| TESTI4015339 | 0.001095278 | 264707 | 1 |
| TESTI4015398 | 0.001095278 | 260813 | 1 |
| TESTI4015442 | 0.008310351 | 206677 | 4 |
| TESTI4015471 | 0.001095278 | 251510 | 1 |
| TESTI4015477 | 0.002190557 | 182384 | 2 |
| TESTI4015600 | 0.002190557 | 199200 | 2 |
| TESTI4015646 | 0.001095278 | 232483 | 1 |
| TESTI4015681 | 0.001095278 | 250757 | 1 |
| TESTI4015688 | 0.001095278 | 259106 | 1 |
| TESTI4015790 | 0.001095278 | 262645 | 1 |
| TESTI4016110 | 0.001095278 | 229649 | 1 |
| TESTI4016238 | 0.001095278 | 243749 | 1 |
| TESTI4016551 | 0.008762226 | 153589 | 8 |
| TESTI4016812 | 0.077764756 | 120203 | 71 |
| TESTI4016822 | 0.001095278 | 258312 | 1 |
| TESTI4016848 | 0.003285835 | 51147 | 3 |
| TESTI4016865 | 0.001095278 | 266111 | 1 |
| TESTI4016882 | 0.001095278 | 262826 | 1 |
| TESTI4016925 | 0.004848962 | 183298 | 3 |
| TESTI4017001 | 0.001095278 | 250724 | 1 |
| TESTI4017116 | 0.002190557 | 284487 | 2 |
| TESTI4017137 | 0.001095278 | 253987 | 1 |
| TESTI4017229 | 0.001095278 | 249141 | 1 |
| TESTI4017254 | 0.001095278 | 215658 | 1 |
| TESTI4017269 | 0.001095278 | 232514 | 1 |
| TESTI4017380 | 0.002190557 | 157332 | 2 |
| TESTI4017382 | 0.001095278 | 229488 | 1 |
| TESTI4017543 | 0.001095278 | 143102 | 1 |
| TESTI4017575 | 0.002190557 | 235480 | 2 |
| TESTI4017647 | 0.001095278 | 259018 | 1 |
| TESTI4017714 | 0.004272069 | 28615 | 3 |
| TESTI4017763 | 0.002190557 | 112201 | 2 |
| TESTI4017848 | 0.001095278 | 261053 | 1 |
| TESTI4017854 | 0.012882171 | 251424 | 2 |
| TESTI4017901 | 0.001095278 | 258424 | 1 |
| TESTI4017961 | 0.001095278 | 249809 | 1 |
| TESTI4017984 | 0.003379801 | 206412 | 3 |
| TESTI4018101 | 0.001095278 | 56150 | 1 |
| TESTI4018152 | 0.007779178 | 122861 | 6 |
| TESTI4018208 | 0.001095278 | 238296 | 1 |
| TESTI4018382 | 0.001095278 | 257236 | 1 |
| TESTI4018436 | 0.032196163 | 136438 | 5 |
| TESTI4018506 | 0.003285835 | 111248 | 3 |
| TESTI4018555 | 0.001095278 | 244990 | 1 |
| TESTI4018751 | 0.001095278 | 245120 | 1 |
| TESTI4018806 | 0.002190557 | 139589 | 2 |
| TESTI4018835 | 0.009857504 | 135580 | 9 |
| TESTI4018859 | 0.001095278 | 248308 | 1 |
| TESTI4018881 | 0.001095278 | 47576 | 1 |
| TESTI4018886 | 0.00309368 | 233647 | 2 |
| TESTI4018938 | 0.009676875 | 81707 | 7 |
| TESTI4019140 | 0.001095278 | 154241 | 1 |
| TESTI4019149 | 0.001095278 | 273650 | 1 |
| TESTI4019299 | 0.007666948 | 186168 | 7 |
| TESTI4019417 | 0.001095278 | 217693 | 1 |
| TESTI4019440 | 0.001095278 | 204512 | 1 |
| TESTI4019566 | 0.002190557 | 131150 | 2 |
| TESTI4019657 | 0.001095278 | 283873 | 1 |
| TESTI4019756 | 0.001095278 | 263749 | 1 |
| TESTI4019843 | 0.004190774 | 151971 | 2 |
| TESTI4020092 | 0.001095278 | 255673 | 1 |
| TESTI4020102 | 0.001095278 | 85070 | 1 |
| TESTI4020342 | 0.001095278 | 274907 | 1 |
| TESTI4020460 | 0.001095278 | 215871 | 1 |
| TESTI4020596 | 0.00317679 | 125073 | 2 |
| TESTI4020806 | 0.001095278 | 179738 | 1 |
| TESTI4020819 | 0.001095278 | 167983 | 1 |
| TESTI4020920 | 0.001095278 | 132886 | 1 |
| TESTI4021129 | 0.001095278 | 149202 | 1 |
| TESTI4021197 | 0.001095278 | 145096 | 1 |
| TESTI4021294 | 0.001095278 | 229706 | 1 |
| TESTI4021377 | 0.001095278 | 260709 | 1 |
| TESTI4021456 | 0.001095278 | 254568 | 1 |
| TESTI4021478 | 0.007582332 | 144708 | 5 |
| TESTI4021482 | 0.007897763 | 77099 | 5 |
| TESTI4021491 | 0.001095278 | 237860 | 1 |
| TESTI4021569 | 0.001095278 | 42538 | 1 |
| TESTI4021713 | 0.001095278 | 156665 | 1 |
| TESTI4021821 | 0.001095278 | 204425 | 1 |
| TESTI4022158 | 0.012554286 | 150104 | 8 |
| TESTI4022577 | 0.001095278 | 192419 | 1 |
| TESTI4022716 | 0.001095278 | 111688 | 1 |
| TESTI4022873 | 0.001095278 | 232363 | 1 |
| TESTI4022936 | 0.006335944 | 102377 | 4 |
| TESTI4023096 | 0.001095278 | 114511 | 1 |
| TESTI4023172 | 0.001095278 | 281426 | 1 |
| TESTI4023546 | 0.016429174 | 134897 | 15 |
| TESTI4023555 | 0.001095278 | 214574 | 1 |
| TESTI4023654 | 0.001095278 | 211754 | 1 |
| TESTI4023722 | 0.001095278 | 186109 | 1 |
| TESTI4023762 | 0.001095278 | 186101 | 1 |
| TESTI4023942 | 0.001095278 | 260233 | 1 |
| TESTI4024096 | 0.001095278 | 279195 | 1 |
| TESTI4024240 | 0.001095278 | 188183 | 1 |
| TESTI4024245 | 0.004970521 | 182565 | 3 |
| TESTI4024294 | 0.001095278 | 193075 | 1 |
| TESTI4024344 | 0.001095278 | 180251 | 1 |
| TESTI4024387 | 0.001095278 | 209217 | 1 |
| TESTI4024420 | 0.004480285 | 192379 | 3 |
| TESTI4024494 | 0.001095278 | 22811 | 1 |
| TESTI4024740 | 0.009314271 | 150671 | 5 |
| TESTI4024874 | 0.001095278 | 141952 | 1 |
| TESTI4024890 | 0.001095278 | 149248 | 1 |
| TESTI4024907 | 0.001095278 | 179945 | 1 |
| TESTI4024930 | 0.001095278 | 149850 | 1 |
| TESTI4025062 | 0.001095278 | 260999 | 1 |
| TESTI4025268 | 0.002770825 | 122913 | 2 |
| TESTI4025401 | 0.001095278 | 199332 | 1 |
| TESTI4025494 | 0.001095278 | 173798 | 1 |
| TESTI4025547 | 0.002804738 | 79327 | 2 |
| TESTI4025731 | 0.001095278 | 206414 | 1 |
| TESTI4025797 | 0.001095278 | 135933 | 1 |
| TESTI4025865 | 0.001095278 | 154298 | 1 |
| TESTI4025908 | 0.001095278 | 182532 | 1 |
| TESTI4025920 | 0.002190557 | 180294 | 2 |
| TESTI4026079 | 0.006586012 | 106872 | 3 |
| TESTI4026080 | 0.001095278 | 61461 | 1 |
| TESTI4026192 | 0.001095278 | 180299 | 1 |
| TESTI4026207 | 0.002804738 | 272038 | 2 |
| TESTI4026295 | 0.001095278 | 177098 | 1 |
| TESTI4026390 | 0.002284523 | 164381 | 2 |
| TESTI4026456 | 0.001095278 | 192386 | 1 |
| TESTI4026510 | 0.001095278 | 100986 | 1 |
| TESTI4026524 | 0.001095278 | 131678 | 1 |
| TESTI4026680 | 0.004381113 | 116436 | 4 |
| TESTI4026700 | 0.001095278 | 109058 | 1 |
| TESTI4026762 | 0.001095278 | 134344 | 1 |
| TESTI4026785 | 0.001095278 | 206441 | 1 |
| TESTI4027139 | 0.001095278 | 279426 | 1 |
| TESTI4027170 | 0.001095278 | 247835 | 1 |
| TESTI4027262 | 0.001095278 | 281562 | 1 |
| TESTI4027516 | 0.001095278 | 242249 | 1 |
| TESTI4027557 | 0.001095278 | 221398 | 1 |
| TESTI4027660 | 0.001095278 | 146701 | 1 |
| TESTI4027821 | 0.002770825 | 213184 | 2 |
| TESTI4027969 | 0.002190557 | 197820 | 2 |
| TESTI4028042 | 0.001095278 | 192194 | 1 |
| TESTI4028059 | 0.002190557 | 42826 | 2 |
| TESTI4028062 | 0.001095278 | 269612 | 1 |
| TESTI4028182 | 0.001095278 | 283473 | 1 |
| TESTI4028429 | 0.003285835 | 101925 | 3 |
| TESTI4028612 | 0.001095278 | 255236 | 1 |
| TESTI4028692 | 0.001095278 | 186281 | 1 |
| TESTI4028809 | 0.001095278 | 245690 | 1 |
| TESTI4028823 | 0.001095278 | 225843 | 1 |
| TESTI4028880 | 0.001095278 | 12859 | 1 |
| TESTI4028904 | 0.001095278 | 241329 | 1 |
| TESTI4028938 | 0.001095278 | 228668 | 1 |
| TESTI4028958 | 0.001095278 | 130557 | 1 |
| TESTI4028983 | 0.001095278 | 249716 | 1 |
| TESTI4029023 | 0.002190557 | 120394 | 2 |
| TESTI4029297 | 0.037866701 | 118926 | 19 |
| TESTI4029348 | 0.001095278 | 239232 | 1 |
| TESTI4029370 | 0.001095278 | 188035 | 1 |
| TESTI4029528 | 0.001095278 | 245597 | 1 |
| TESTI4029651 | 0.001095278 | 180629 | 1 |
| TESTI4029671 | 0.009008804 | 219783 | 4 |
| TESTI4029676 | 0.001095278 | 251292 | 1 |
| TESTI4029690 | 0.001095278 | 242520 | 1 |
| TESTI4029731 | 0.001095278 | 232287 | 1 |
| TESTI4029743 | 0.002190557 | 172493 | 2 |
| TESTI4029836 | 0.002783555 | 140088 | 2 |
| TESTI4030069 | 0.001095278 | 241219 | 1 |
| TESTI4030159 | 0.001095278 | 225940 | 1 |
| TESTI4030319 | 0.001095278 | 275319 | 1 |
| TESTI4030505 | 0.001095278 | 156700 | 1 |
| TESTI4030603 | 0.001095278 | 254817 | 1 |
| TESTI4030669 | 0.002190557 | 238967 | 2 |
| TESTI4030673 | 0.001095278 | 251257 | 1 |
| TESTI4030864 | 0.001095278 | 122070 | 1 |
| TESTI4031066 | 0.001095278 | 211782 | 1 |
| TESTI4031173 | 0.002190557 | 128307 | 2 |
| TESTI4031335 | 0.001095278 | 214580 | 1 |
| TESTI4031745 | 0.001095278 | 205992 | 1 |
| TESTI4031818 | 0.001095278 | 186273 | 1 |
| TESTI4032090 | 0.002770825 | 175498 | 2 |
| TESTI4032112 | 0.001095278 | 199306 | 1 |
| TESTI4032128 | 0.001095278 | 215321 | 1 |
| TESTI4032270 | 0.001095278 | 221282 | 1 |
| TESTI4032375 | 0.001095278 | 194277 | 1 |
| TESTI4032834 | 0.001095278 | 243607 | 1 |
| TESTI4032856 | 0.001095278 | 239404 | 1 |
| TESTI4032895 | 0.002190557 | 148207 | 2 |
| TESTI4032913 | 0.020672075 | 91743 | 7 |
| TESTI4033146 | 0.002190557 | 211697 | 2 |
| TESTI4033177 | 0.001095278 | 197836 | 1 |
| TESTI4033433 | 0.001095278 | 232260 | 1 |
| TESTI4033690 | 0.00297212 | 130642 | 2 |
| TESTI4034172 | 0.001095278 | 146288 | 1 |
| TESTI4034212 | 0.001095278 | 208305 | 1 |
| TESTI4034432 | 0.001095278 | 154533 | 1 |
| TESTI4034495 | 0.001095278 | 269704 | 1 |
| TESTI4034632 | 0.001095278 | 243364 | 1 |
| TESTI4034633 | 0.001095278 | 232482 | 1 |
| TESTI4034912 | 0.001095278 | 156069 | 1 |
| TESTI4034973 | 0.001095278 | 206152 | 1 |
| TESTI4035063 | 0.001095278 | 135437 | 1 |
| TESTI4035065 | 0.002190557 | 177081 | 2 |
| TESTI4035498 | 0.001095278 | 201060 | 1 |
| TESTI4035508 | 0.001095278 | 61455 | 1 |
| TESTI4035581 | 0.001095278 | 241996 | 1 |
| TESTI4035602 | 0.001095278 | 192094 | 1 |
| TESTI4035637 | 0.001095278 | 248492 | 1 |
| TESTI4035649 | 0.001095278 | 247937 | 1 |
| TESTI4035770 | 0.00317679 | 244389 | 2 |
| TESTI4035872 | 0.001095278 | 239976 | 1 |
| TESTI4035895 | 0.001095278 | 282194 | 1 |
| TESTI4035898 | 0.001095278 | 117895 | 1 |
| TESTI4035989 | 0.001095278 | 254379 | 1 |
| TESTI4036012 | 0.001095278 | 234842 | 1 |
| TESTI4036042 | 0.001095278 | 240025 | 1 |
| TESTI4036048 | 0.001095278 | 269260 | 1 |
| TESTI4036315 | 0.001095278 | 254350 | 1 |
| TESTI4036319 | 0.001095278 | 232367 | 1 |
| TESTI4036449 | 0.001095278 | 216173 | 1 |
| TESTI4036767 | 0.001095278 | 255550 | 1 |
| TESTI4036909 | 0.001095278 | 235074 | 1 |
| TESTI4037066 | 0.001095278 | 125935 | 1 |
| TESTI4037156 | 0.605504292 | 66227 | 79 |
| TESTI4037188 | 0.002783555 | 166357 | 2 |
| TESTI4037228 | 0.001095278 | 258145 | 1 |
| TESTI4037244 | 0.002190557 | 255818 | 2 |
| TESTI4037618 | 0.022532564 | 98637 | 12 |
| TESTI4037727 | 0.001095278 | 207484 | 1 |
| TESTI4037949 | 0.001095278 | 111330 | 1 |
| TESTI4038047 | 0.001095278 | 240753 | 1 |
| TESTI4038099 | 0.007595886 | 50411 | 5 |
| TESTI4038156 | 0.001095278 | 244899 | 1 |
| TESTI4038223 | 0.001095278 | 224171 | 1 |
| TESTI4038258 | 0.001095278 | 198921 | 1 |
| TESTI4038284 | 0.090980089 | 39991 | 29 |
| TESTI4038339 | 0.001095278 | 96909 | 1 |
| TESTI4038492 | 0.001095278 | 198910 | 1 |
| TESTI4038721 | 0.002190557 | 149050 | 2 |
| TESTI4038758 | 0.001095278 | 211918 | 1 |
| TESTI4038779 | 0.001095278 | 206960 | 1 |
| TESTI4038818 | 0.001095278 | 210845 | 1 |
| TESTI4039038 | 0.001095278 | 249909 | 1 |
| TESTI4039086 | 0.001095278 | 231191 | 1 |
| TESTI4039451 | 0.001095278 | 185954 | 1 |
| TESTI4039575 | 0.001095278 | 59556 | 1 |
| TESTI4039659 | 0.001095278 | 199768 | 1 |
| TESTI4039744 | 0.001095278 | 197328 | 1 |
| TESTI4039904 | 0.001095278 | 18953 | 1 |
| TESTI4040197 | 0.001095278 | 235500 | 1 |
| TESTI4040363 | 0.001095278 | 106961 | 1 |
| TESTI4040535 | 0.001095278 | 225793 | 1 |
| TESTI4040559 | 0.002190557 | 185320 | 2 |
| TESTI4040598 | 0.039147029 | 199530 | 2 |
| TESTI4040800 | 0.002190557 | 170359 | 2 |
| TESTI4040804 | 0.001095278 | 210697 | 1 |
| TESTI4040939 | 0.001095278 | 281109 | 1 |
| TESTI4040956 | 0.001095278 | 249496 | 1 |
| TESTI4041049 | 0.001095278 | 284852 | 1 |
| TESTI4041053 | 0.001095278 | 254785 | 1 |
| TESTI4041086 | 0.001095278 | 230544 | 1 |
| TESTI4041099 | 0.002190557 | 118090 | 2 |
| TESTI4041143 | 0.001095278 | 230829 | 1 |
| TESTI4041482 | 0.001095278 | 37801 | 1 |
| TESTI4041519 | 0.001095278 | 235446 | 1 |
| TESTI4041624 | 0.001095278 | 254663 | 1 |
| TESTI4041629 | 0.001095278 | 281176 | 1 |
| TESTI4041832 | 0.001095278 | 247573 | 1 |
| TESTI4041844 | 0.001095278 | 247548 | 1 |
| TESTI4041903 | 0.001095278 | 230867 | 1 |
| TESTI4041954 | 0.001095278 | 247608 | 1 |
| TESTI4041984 | 0.002190557 | 76745 | 2 |
| TESTI4041985 | 0.001095278 | 226807 | 1 |
| TESTI4041986 | 0.001095278 | 218600 | 1 |
| TESTI4042098 | 0.001095278 | 284723 | 1 |
| TESTI4042420 | 0.001095278 | 218571 | 1 |
| TESTI4042440 | 0.001095278 | 124401 | 1 |
| TESTI4042444 | 0.001095278 | 209142 | 1 |
| TESTI4042711 | 0.001095278 | 206775 | 1 |
| TESTI4042846 | 0.001095278 | 230935 | 1 |
| TESTI4043067 | 0.001095278 | 180443 | 1 |
| TESTI4043129 | 0.001095278 | 225818 | 1 |
| TESTI4043140 | 0.060428516 | 131310 | 17 |
| TESTI4043166 | 0.002190557 | 212598 | 2 |
| TESTI4043203 | 0.001095278 | 180026 | 1 |
| TESTI4043223 | 0.006054311 | 138492 | 4 |
| TESTI4043371 | 0.001095278 | 212595 | 1 |
| TESTI4043378 | 0.001095278 | 203763 | 1 |
| TESTI4043512 | 0.002190557 | 221950 | 2 |
| TESTI4043551 | 0.001095278 | 277418 | 1 |
| TESTI4043710 | 0.001095278 | 239618 | 1 |
| TESTI4043947 | 0.001095278 | 218542 | 1 |
| TESTI4044035 | 0.001095278 | 235563 | 1 |
| TESTI4044076 | 0.001095278 | 236416 | 1 |
| TESTI4044084 | 0.001095278 | 240444 | 1 |
| TESTI4044123 | 0.001095278 | 256132 | 1 |
| TESTI4044186 | 0.001095278 | 252354 | 1 |
| TESTI4044234 | 0.001095278 | 240637 | 1 |
| TESTI4044291 | 0.002190557 | 237908 | 2 |
| TESTI4044296 | 0.001095278 | 252319 | 1 |
| TESTI4044300 | 0.001095278 | 252320 | 1 |
| TESTI4044682 | 0.001095278 | 252417 | 1 |
| TESTI4044704 | 0.032874613 | 64394 | 9 |
| TESTI4044770 | 0.001095278 | 235568 | 1 |
| TESTI4045168 | 0.001095278 | 212690 | 1 |
| TESTI4045312 | 0.002190557 | 120404 | 2 |
| TESTI4045330 | 0.001095278 | 181281 | 1 |
| TESTI4045470 | 0.001095278 | 144924 | 1 |
| TESTI4045701 | 0.001095278 | 158326 | 1 |
| TESTI4045908 | 0.001095278 | 231426 | 1 |
| TESTI4045955 | 0.001095278 | 266764 | 1 |
| TESTI4045982 | 0.001095278 | 199723 | 1 |
| TESTI4046073 | 0.008825121 | 157549 | 3 |
| TESTI4046090 | 0.003285835 | 226844 | 3 |
| TESTI4046240 | 0.001095278 | 221953 | 1 |
| TESTI4046245 | 0.001095278 | 79139 | 1 |
| TESTI4046253 | 0.001095278 | 206709 | 1 |
| TESTI4046282 | 0.001095278 | 185412 | 1 |
| TESTI4046328 | 0.001095278 | 230649 | 1 |
| TESTI4046450 | 0.001095278 | 120184 | 1 |
| TESTI4046457 | 0.001095278 | 148584 | 1 |
| TESTI4046487 | 0.001095278 | 182480 | 1 |
| TESTI4046502 | 0.003866104 | 96815 | 3 |
| TESTI4046523 | 0.001095278 | 218154 | 1 |
| TESTI4046819 | 0.008486842 | 121011 | 3 |
| TESTI4046873 | 0.004077237 | 219380 | 2 |
| TESTI4046884 | 0.001095278 | 196134 | 1 |
| TESTI4046896 | 0.001095278 | 214282 | 1 |
| TESTI4046903 | 0.001095278 | 225700 | 1 |
| TESTI4046962 | 0.001095278 | 182857 | 1 |
| TESTI4047069 | 0.001095278 | 176966 | 1 |
| TESTI4047119 | 0.002190557 | 172789 | 2 |
| TESTI4047305 | 0.001095278 | 239962 | 1 |
| TESTI4047328 | 0.001095278 | 226854 | 1 |
| TESTI4047437 | 0.001095278 | 248013 | 1 |
| TESTI4047569 | 0.002190557 | 51155 | 2 |
| TESTI4047746 | 0.001095278 | 223052 | 1 |
| TESTI4047808 | 0.002190557 | 180518 | 2 |
| THYMU1000002 | 0.283081539 | 13295 | 31 |
| THYMU1000010 | 0.062156154 | 9982 | 7 |
| THYMU1000016 | 0.433589622 | 8513 | 43 |
| THYMU1000017 | 0.00557409 | 61283 | 2 |
| THYMU1000025 | 0.001397155 | 67971 | 1 |
| THYMU1000029 | 0.003106616 | 68402 | 2 |
| THYMU1000032 | 0.2146652 | 29347 | 91 |
| THYMU1000033 | 0.001397155 | 71495 | 1 |
| THYMU1000041 | 0.070464806 | 77450 | 30 |
| THYMU1000060 | 0.001397155 | 46511 | 1 |
| THYMU1000074 | 0.001397155 | 76856 | 1 |
| THYMU1000083 | 0.055878048 | 57909 | 21 |
| THYMU1000098 | 0.005588622 | 67676 | 4 |
| THYMU1000103 | 0.095037005 | 31692 | 41 |
| THYMU1000105 | 0.01116705 | 39932 | 4 |
| THYMU1000109 | 0.212893133 | 67579 | 24 |
| THYMU1000136 | 0.001397155 | 12463 | 1 |
| THYMU1000142 | 0.003395557 | 38666 | 2 |
| THYMU1000144 | 0.129554365 | 13387 | 68 |
| THYMU1000158 | 0.001397155 | 77621 | 1 |
| THYMU1000176 | 0.001397155 | 76265 | 1 |
| THYMU1000179 | 0.003273997 | 66210 | 2 |
| THYMU1000191 | 0.004191466 | 89223 | 3 |
| THYMU1000198 | 0.003085432 | 14759 | 2 |
| THYMU1000217 | 0.001397155 | 30221 | 1 |
| THYMU1000240 | 0.001397155 | 110687 | 1 |
| THYMU1000242 | 0.001397155 | 267162 | 1 |
| THYMU1000291 | 0.001397155 | 79043 | 1 |
| THYMU1000316 | 0.003395557 | 185601 | 2 |
| THYMU1000318 | 0.001397155 | 263995 | 1 |
| THYMU1000329 | 0.031439043 | 137063 | 7 |
| THYMU1000331 | 0.003681678 | 133321 | 3 |
| THYMU1000336 | 0.001397155 | 258167 | 1 |
| THYMU1000359 | 0.312579475 | 109518 | 78 |
| THYMU1000366 | 0.001397155 | 273762 | 1 |
| THYMU1000374 | 0.111870084 | 34316 | 36 |
| THYMU1000382 | 0.001397155 | 250667 | 1 |
| THYMU1000393 | 0.001397155 | 265725 | 1 |
| THYMU1000394 | 0.034085076 | 73770 | 9 |
| THYMU1000399 | 0.001397155 | 261134 | 1 |
| THYMU1000426 | 0.001397155 | 119256 | 1 |
| THYMU1000428 | 0.294780918 | 72160 | 29 |
| THYMU1000440 | 0.002794311 | 276993 | 2 |
| THYMU1000459 | 0.004191466 | 113570 | 3 |
| THYMU1000473 | 0.050373735 | 46668 | 3 |
| THYMU1000490 | 0.029220305 | 123055 | 11 |
| THYMU1000491 | 0.001397155 | 89146 | 1 |
| THYMU1000496 | 0.001397155 | 237850 | 1 |
| THYMU1000499 | 0.029356753 | 146535 | 5 |
| THYMU1000527 | 0.001397155 | 265406 | 1 |
| THYMU1000532 | 0.005588622 | 59728 | 4 |
| THYMU1000536 | 0.001397155 | 265405 | 1 |
| THYMU1000554 | 0.001397155 | 251027 | 1 |
| THYMU1000558 | 0.001397155 | 285168 | 1 |
| THYMU1000576 | 0.001397155 | 281822 | 1 |
| THYMU1000600 | 0.002794311 | 230476 | 2 |
| THYMU1000677 | 0.001397155 | 137997 | 1 |
| THYMU1000692 | 0.001397155 | 120913 | 1 |
| THYMU2000022 | 0.001397155 | 188729 | 1 |
| THYMU2000032 | 0.001397155 | 164054 | 1 |
| THYMU2000057 | 0.123541998 | 149586 | 19 |
| THYMU2000120 | 0.004312012 | 21197 | 2 |
| THYMU2000138 | 0.007907695 | 117960 | 5 |
| THYMU2000140 | 0.001397155 | 46673 | 1 |
| THYMU2000155 | 0.00325527 | 218203 | 2 |
| THYMU2000162 | 0.0025864 | 167097 | 2 |
| THYMU2000171 | 0.001397155 | 264286 | 1 |
| THYMU2000176 | 0.001397155 | 213541 | 1 |
| THYMU2000179 | 0.001397155 | 213542 | 1 |
| THYMU2000231 | 0.002794311 | 216378 | 2 |
| THYMU2000236 | 0.007633513 | 140097 | 2 |
| THYMU2000276 | 0.001397155 | 226351 | 1 |
| THYMU2000280 | 0.001397155 | 141333 | 1 |
| THYMU2000300 | 0.057353 | 113559 | 32 |
| THYMU2000317 | 0.001397155 | 214584 | 1 |
| THYMU2000368 | 0.001397155 | 17269 | 1 |
| THYMU2000369 | 0.00932013 | 157899 | 3 |
| THYMU2000382 | 0.276441727 | 126769 | 37 |
| THYMU2000418 | 0.001397155 | 218676 | 1 |
| THYMU2000436 | 0.18184934 | 102755 | 32 |
| THYMU2000440 | 0.001397155 | 38752 | 1 |
| THYMU2000489 | 0.003085432 | 158002 | 2 |
| THYMU2000492 | 0.001397155 | 271322 | 1 |
| THYMU2000519 | 0.001397155 | 205302 | 1 |
| THYMU2000570 | 0.001397155 | 167100 | 1 |
| THYMU2000602 | 0.001397155 | 5421 | 1 |
| THYMU2000660 | 0.001397155 | 170352 | 1 |
| THYMU2000671 | 0.001397155 | 236649 | 1 |
| THYMU2000672 | 0.007407508 | 113346 | 3 |
| THYMU2000684 | 0.040243433 | 91855 | 13 |
| THYMU2000702 | 0.053548641 | 85768 | 4 |
| THYMU2000767 | 0.001397155 | 104070 | 1 |
| THYMU2000775 | 0.01789626 | 21245 | 5 |
| THYMU2000800 | 0.001397155 | 211475 | 1 |
| THYMU2000879 | 0.001397155 | 226164 | 1 |
| THYMU2000932 | 0.001397155 | 245668 | 1 |
| THYMU2000946 | 0.115810319 | 99735 | 28 |
| THYMU2000950 | 0.007599475 | 18146 | 2 |
| THYMU2000971 | 0.00325527 | 132607 | 2 |
| THYMU2000982 | 0.001397155 | 230500 | 1 |
| THYMU2000990 | 0.001397155 | 226155 | 1 |
| THYMU2001007 | 0.061762722 | 78235 | 24 |
| THYMU2001018 | 0.018552145 | 174440 | 5 |
| THYMU2001053 | 0.001397155 | 281450 | 1 |
| THYMU2001071 | 0.001397155 | 231544 | 1 |
| THYMU2001090 | 0.007884209 | 161294 | 5 |
| THYMU2001139 | 0.001397155 | 213952 | 1 |
| THYMU2001195 | 0.004312012 | 17886 | 2 |
| THYMU2001202 | 0.001397155 | 235823 | 1 |
| THYMU2001203 | 0.001397155 | 226245 | 1 |
| THYMU2001256 | 0.001397155 | 231602 | 1 |
| THYMU2001270 | 0.001397155 | 230491 | 1 |
| THYMU2001291 | 0.001397155 | 167535 | 1 |
| THYMU2001314 | 0.005588622 | 151081 | 4 |
| THYMU2001325 | 0.001397155 | 81069 | 1 |
| THYMU2001357 | 0.001397155 | 113813 | 1 |
| THYMU2001381 | 0.024703459 | 113378 | 3 |
| THYMU2001422 | 0.01345275 | 174585 | 4 |
| THYMU2001443 | 0.001397155 | 102286 | 1 |
| THYMU2001515 | 0.011489548 | 69152 | 8 |
| THYMU2001521 | 0.042153599 | 145191 | 8 |
| THYMU2001556 | 0.001397155 | 226228 | 1 |
| THYMU2001622 | 0.001397155 | 118701 | 1 |
| THYMU2001727 | 0.001397155 | 249353 | 1 |
| THYMU2001819 | 0.007309767 | 159938 | 2 |
| THYMU2001825 | 0.002794311 | 66223 | 2 |
| THYMU2001839 | 0.010709459 | 103674 | 5 |
| THYMU2001868 | 0.005241239 | 223394 | 2 |
| THYMU2001898 | 0.001397155 | 136490 | 1 |
| THYMU2001900 | 0.001397155 | 203418 | 1 |
| THYMU2001916 | 0.001397155 | 211487 | 1 |
| THYMU2001926 | 0.008632811 | 18811 | 4 |
| THYMU2001989 | 0.001397155 | 230443 | 1 |
| THYMU2002023 | 0.012503049 | 59355 | 3 |
| THYMU2002037 | 0.001397155 | 238830 | 1 |
| THYMU2002080 | 0.01125891 | 63720 | 4 |
| THYMU2002109 | 0.001397155 | 165753 | 1 |
| THYMU2002147 | 0.007106323 | 69160 | 4 |
| THYMU2002154 | 0.004191466 | 230045 | 3 |
| THYMU2002157 | 0.001397155 | 235988 | 1 |
| THYMU2002162 | 0.01536891 | 16884 | 6 |
| THYMU2002177 | 0.001397155 | 269644 | 1 |
| THYMU2002203 | 0.001397155 | 127210 | 1 |
| THYMU2002229 | 0.015388769 | 242218 | 3 |
| THYMU2002242 | 0.001397155 | 107768 | 1 |
| THYMU2002338 | 0.002794311 | 39389 | 2 |
| THYMU2002356 | 0.001397155 | 96959 | 1 |
| THYMU2002376 | 0.001397155 | 249176 | 1 |
| THYMU2002384 | 0.001397155 | 218810 | 1 |
| THYMU2002450 | 0.007766648 | 145261 | 4 |
| THYMU2002490 | 0.001397155 | 41710 | 1 |
| THYMU2002548 | 0.001397155 | 209027 | 1 |
| THYMU2002583 | 0.323295964 | 88940 | 60 |
| THYMU2002695 | 0.001397155 | 156732 | 1 |
| THYMU2002745 | 0.043765852 | 149146 | 3 |
| THYMU2002756 | 0.022887652 | 14610 | 6 |
| THYMU2002789 | 0.001397155 | 255784 | 1 |
| THYMU2002792 | 0.001397155 | 127283 | 1 |
| THYMU2002815 | 0.005071104 | 132176 | 3 |
| THYMU2002841 | 0.057734742 | 172937 | 10 |
| THYMU2002852 | 0.001397155 | 235585 | 1 |
| THYMU2002910 | 0.001397155 | 235582 | 1 |
| THYMU2002950 | 0.001397155 | 85939 | 1 |
| THYMU2002983 | 0.055988425 | 77410 | 7 |
| THYMU2003012 | 0.191195817 | 6891 | 44 |
| THYMU2003046 | 0.087161916 | 100940 | 30 |
| THYMU2003069 | 0.037910844 | 98201 | 26 |
| THYMU2003107 | 0.001397155 | 135046 | 1 |
| THYMU2003124 | 0.001397155 | 114586 | 1 |
| THYMU2003133 | 0.048292691 | 131790 | 13 |
| THYMU2003166 | 0.001397155 | 172749 | 1 |
| THYMU2003232 | 0.001397155 | 226146 | 1 |
| THYMU2003242 | 0.001397155 | 205214 | 1 |
| THYMU2003249 | 0.001397155 | 200892 | 1 |
| THYMU2003270 | 0.001397155 | 226126 | 1 |
| THYMU2003282 | 0.001397155 | 206536 | 1 |
| THYMU2003287 | 0.0140921 | 124677 | 9 |
| THYMU2003336 | 0.001397155 | 235876 | 1 |
| THYMU2003366 | 0.001397155 | 11709 | 1 |
| THYMU2003397 | 0.011177243 | 110175 | 8 |
| THYMU2003419 | 0.333649462 | 50383 | 87 |
| THYMU2003440 | 0.007202334 | 73697 | 2 |
| THYMU2003446 | 0.070252924 | 41188 | 33 |
| THYMU2003470 | 0.001397155 | 194968 | 1 |
| THYMU2003479 | 0.001397155 | 180030 | 1 |
| THYMU2003499 | 0.011116458 | 205584 | 6 |
| THYMU2003542 | 0.077533087 | 129557 | 9 |
| THYMU2003632 | 0.001397155 | 276680 | 1 |
| THYMU2003650 | 0.001397155 | 123904 | 1 |
| THYMU2003700 | 0.001397155 | 211463 | 1 |
| THYMU2003760 | 0.001397155 | 280339 | 1 |
| THYMU2003803 | 0.001397155 | 282132 | 1 |
| THYMU2003855 | 0.001397155 | 123448 | 1 |
| THYMU2003891 | 1.148350684 | 17403 | 123 |
| THYMU2003907 | 0.01477468 | 103087 | 3 |
| THYMU2003932 | 0.00866256 | 98542 | 5 |
| THYMU2003981 | 0.320836355 | 113852 | 56 |
| THYMU2004003 | 0.001397155 | 241020 | 1 |
| THYMU2004008 | 0.002794311 | 247799 | 2 |
| THYMU2004042 | 0.001397155 | 226055 | 1 |
| THYMU2004108 | 0.012683837 | 193524 | 2 |
| THYMU2004111 | 0.001397155 | 250531 | 1 |
| THYMU2004123 | 0.001397155 | 51144 | 1 |
| THYMU2004139 | 0.001397155 | 226341 | 1 |
| THYMU2004152 | 0.168753016 | 120744 | 37 |
| THYMU2004169 | 0.001397155 | 135410 | 1 |
| THYMU2004284 | 0.02494276 | 163753 | 4 |
| THYMU2004320 | 0.010254136 | 130070 | 3 |
| THYMU2004336 | 0.002794311 | 122295 | 2 |
| THYMU2004344 | 0.131161861 | 116807 | 34 |
| THYMU2004356 | 0.001397155 | 89238 | 1 |
| THYMU2004410 | 0.087450229 | 54463 | 31 |
| THYMU2004452 | 0.007249637 | 99444 | 3 |
| THYMU2004508 | 0.0025864 | 116253 | 2 |
| THYMU2004512 | 0.001397155 | 205079 | 1 |
| THYMU2004635 | 0.003106616 | 32346 | 2 |
| THYMU2004639 | 0.001397155 | 123890 | 1 |
| THYMU2004668 | 0.001397155 | 283209 | 1 |
| THYMU2004677 | 0.001397155 | 198298 | 1 |
| THYMU2004688 | 0.001397155 | 40823 | 1 |
| THYMU2004693 | 0.001397155 | 195197 | 1 |
| THYMU2004733 | 0.029917801 | 88328 | 7 |
| THYMU2004835 | 0.001397155 | 218972 | 1 |
| THYMU2004843 | 0.001397155 | 105346 | 1 |
| THYMU2004906 | 0.013368594 | 3338 | 4 |
| THYMU2004916 | 0.107375141 | 47990 | 8 |
| THYMU2004986 | 0.002794311 | 130714 | 2 |
| THYMU2005001 | 0.001397155 | 195704 | 1 |
| THYMU2005003 | 0.011999361 | 120273 | 2 |
| THYMU2005046 | 0.001397155 | 219011 | 1 |
| THYMU2005134 | 0.001397155 | 162675 | 1 |
| THYMU2005156 | 0.004379115 | 195188 | 2 |
| THYMU2005190 | 0.001397155 | 198500 | 1 |
| THYMU2005225 | 0.001397155 | 216662 | 1 |
| THYMU2005240 | 0.001397155 | 272225 | 1 |
| THYMU2005246 | 0.003273997 | 68958 | 2 |
| THYMU2005270 | 0.001397155 | 82751 | 1 |
| THYMU2005283 | 0.001397155 | 240531 | 1 |
| THYMU2005303 | 0.001397155 | 32451 | 1 |
| THYMU2005321 | 0.001397155 | 280807 | 1 |
| THYMU2005356 | 0.001397155 | 85840 | 1 |
| THYMU2005369 | 0.001397155 | 223207 | 1 |
| THYMU2005439 | 0.030312595 | 48998 | 8 |
| THYMU2005480 | 0.001397155 | 226352 | 1 |
| THYMU2005482 | 0.013617142 | 185054 | 3 |
| THYMU2005507 | 0.004191466 | 163700 | 3 |
| THYMU2005545 | 0.001397155 | 240591 | 1 |
| THYMU2005546 | 0.306336962 | 129372 | 30 |
| THYMU2005574 | 0.0025864 | 209727 | 2 |
| THYMU2005576 | 0.001397155 | 228651 | 1 |
| THYMU2005748 | 0.001397155 | 225873 | 1 |
| THYMU2005759 | 0.001397155 | 252386 | 1 |
| THYMU2005807 | 0.0025864 | 171405 | 2 |
| THYMU2005815 | 0.001397155 | 249428 | 1 |
| THYMU2005855 | 0.001397155 | 245398 | 1 |
| THYMU2005881 | 0.001397155 | 240921 | 1 |
| THYMU2005948 | 0.001397155 | 148630 | 1 |
| THYMU2005980 | 0.020662708 | 73188 | 6 |
| THYMU2005997 | 0.010405633 | 119820 | 4 |
| THYMU2006001 | 0.001397155 | 160646 | 1 |
| THYMU2006048 | 0.001397155 | 197193 | 1 |
| THYMU2006098 | 0.001397155 | 135808 | 1 |
| THYMU2006160 | 0.002794311 | 236411 | 2 |
| THYMU2006170 | 0.002794311 | 216808 | 2 |
| THYMU2006252 | 0.001397155 | 207395 | 1 |
| THYMU2006261 | 0.005778268 | 171063 | 5 |
| THYMU2006277 | 0.144458672 | 216753 | 2 |
| THYMU2006303 | 0.001397155 | 97974 | 1 |
| THYMU2006324 | 0.001397155 | 211400 | 1 |
| THYMU2006350 | 0.001397155 | 231285 | 1 |
| THYMU2006357 | 0.001397155 | 118625 | 1 |
| THYMU2006365 | 0.001397155 | 27946 | 1 |
| THYMU2006420 | 0.001397155 | 10463 | 1 |
| THYMU2006468 | 0.001397155 | 131355 | 1 |
| THYMU2006505 | 0.122740821 | 55164 | 35 |
| THYMU2006610 | 0.012340254 | 122170 | 6 |
| THYMU2006666 | 0.001397155 | 85491 | 1 |
| THYMU2006712 | 0.001397155 | 243554 | 1 |
| THYMU2006813 | 0.105889362 | 102938 | 37 |
| THYMU2006913 | 0.001397155 | 250784 | 1 |
| THYMU2006946 | 0.001397155 | 201499 | 1 |
| THYMU2006965 | 0.001397155 | 219135 | 1 |
| THYMU2007025 | 0.001397155 | 37260 | 1 |
| THYMU2007036 | 0.021701029 | 185348 | 6 |
| THYMU2007060 | 0.039300517 | 121218 | 9 |
| THYMU2007065 | 0.041320358 | 154386 | 11 |
| THYMU2007095 | 0.001397155 | 222962 | 1 |
| THYMU2007112 | 0.036637237 | 141088 | 11 |
| THYMU2007146 | 0.00468299 | 193971 | 4 |
| THYMU2007179 | 0.001397155 | 76348 | 1 |
| THYMU2007258 | 0.001397155 | 252452 | 1 |
| THYMU2007307 | 0.001397155 | 239129 | 1 |
| THYMU2007308 | 0.001397155 | 241273 | 1 |
| THYMU2007339 | 0.005435438 | 255387 | 2 |
| THYMU2007350 | 0.001397155 | 61642 | 1 |
| THYMU2007384 | 0.001397155 | 253581 | 1 |
| THYMU2007415 | 0.069241641 | 84113 | 17 |
| THYMU2007467 | 0.041480917 | 99353 | 10 |
| THYMU2007493 | 0.001397155 | 124277 | 1 |
| THYMU2007521 | 0.001397155 | 184192 | 1 |
| THYMU2007528 | 0.001397155 | 166537 | 1 |
| THYMU2007530 | 0.001397155 | 168942 | 1 |
| THYMU2007532 | 0.010725521 | 14745 | 4 |
| THYMU2007561 | 0.001397155 | 184189 | 1 |
| THYMU2007571 | 0.001397155 | 161676 | 1 |
| THYMU2007635 | 0.002794311 | 61668 | 2 |
| THYMU2007658 | 0.001397155 | 201661 | 1 |
| THYMU2007667 | 0.001397155 | 172725 | 1 |
| THYMU2007694 | 0.008081801 | 97753 | 4 |
| THYMU2007725 | 0.001397155 | 113578 | 1 |
| THYMU2007729 | 0.034283517 | 152349 | 9 |
| THYMU2007802 | 0.00557409 | 149659 | 2 |
| THYMU2007824 | 0.001397155 | 241382 | 1 |
| THYMU2007854 | 0.001397155 | 219027 | 1 |
| THYMU2007886 | 0.121006836 | 97367 | 15 |
| THYMU2007952 | 0.001397155 | 197100 | 1 |
| THYMU2007969 | 0.001397155 | 245086 | 1 |
| THYMU2008035 | 0.003072702 | 120061 | 2 |
| THYMU2008036 | 0.003085432 | 83950 | 2 |
| THYMU2008049 | 0.007418294 | 127946 | 4 |
| THYMU2008072 | 0.001397155 | 192864 | 1 |
| THYMU2008111 | 0.004191466 | 96903 | 3 |
| THYMU2008145 | 0.001397155 | 126326 | 1 |
| THYMU2008149 | 0.02844597 | 131914 | 19 |
| THYMU2008185 | 0.058086498 | 14325 | 2 |
| THYMU2008191 | 0.001397155 | 164370 | 1 |
| THYMU2008207 | 0.001397155 | 158332 | 1 |
| THYMU2008226 | 0.001397155 | 180158 | 1 |
| THYMU2008282 | 0.001397155 | 97258 | 1 |
| THYMU2008321 | 0.057960839 | 80930 | 18 |
| THYMU2008339 | 0.001397155 | 166379 | 1 |
| THYMU2008350 | 0.001397155 | 192832 | 1 |
| THYMU2008351 | 0.002794311 | 58947 | 2 |
| THYMU2008383 | 0.002492434 | 118057 | 2 |
| THYMU2008452 | 0.002794311 | 250986 | 2 |
| THYMU2008643 | 0.001397155 | 279859 | 1 |
| THYMU2008686 | 0.013749135 | 100709 | 5 |
| THYMU2008691 | 0.001397155 | 124056 | 1 |
| THYMU2008714 | 0.001397155 | 190749 | 1 |
| THYMU2008725 | 0.012850033 | 145398 | 3 |
| THYMU2008727 | 0.011271748 | 148652 | 2 |
| THYMU2008781 | 0.006985777 | 75637 | 5 |
| THYMU2008799 | 0.001397155 | 133359 | 1 |
| THYMU2008876 | 0.001397155 | 141859 | 1 |
| THYMU2008910 | 0.001397155 | 132079 | 1 |
| THYMU2008917 | 0.001397155 | 177344 | 1 |
| THYMU2008928 | 0.001397155 | 274274 | 1 |
| THYMU2008990 | 0.001397155 | 31522 | 1 |
| THYMU2008994 | 0.001397155 | 280745 | 1 |
| THYMU2009023 | 0.001397155 | 9173 | 1 |
| THYMU2009046 | 0.001397155 | 154501 | 1 |
| THYMU2009101 | 0.001397155 | 167772 | 1 |
| THYMU2009104 | 0.001397155 | 172698 | 1 |
| THYMU2009134 | 0.001397155 | 164435 | 1 |
| THYMU2009157 | 0.002794311 | 169155 | 2 |
| THYMU2009181 | 0.001397155 | 172709 | 1 |
| THYMU2009186 | 0.004312012 | 173089 | 2 |
| THYMU2009338 | 0.00325527 | 39623 | 2 |
| THYMU2009425 | 0.001397155 | 216245 | 1 |
| THYMU2009516 | 0.001397155 | 171898 | 1 |
| THYMU2009526 | 0.009780088 | 128458 | 7 |
| THYMU2009546 | 0.001397155 | 268640 | 1 |
| THYMU2009592 | 0.006065067 | 56883 | 4 |
| THYMU2009596 | 0.001397155 | 170468 | 1 |
| THYMU2009658 | 0.478334266 | 50760 | 34 |
| THYMU2009792 | 0.059828212 | 145358 | 4 |
| THYMU2009835 | 0.001397155 | 192848 | 1 |
| THYMU2009906 | 0.001397155 | 272886 | 1 |
| THYMU2009948 | 0.001397155 | 244597 | 1 |
| THYMU2010030 | 0.001397155 | 167800 | 1 |
| THYMU2010041 | 0.008986358 | 131106 | 6 |
| THYMU2010046 | 0.001397155 | 177371 | 1 |
| THYMU2010059 | 0.001397155 | 161222 | 1 |
| THYMU2010082 | 0.001397155 | 203089 | 1 |
| THYMU2010094 | 0.028766841 | 119777 | 11 |
| THYMU2010161 | 0.011105893 | 197810 | 2 |
| THYMU2010192 | 0.00325527 | 180155 | 2 |
| THYMU2010448 | 0.031644047 | 101868 | 10 |
| THYMU2010519 | 0.021077877 | 167975 | 14 |
| THYMU2010637 | 0.001397155 | 51179 | 1 |
| THYMU2010671 | 0.001397155 | 172636 | 1 |
| THYMU2010699 | 0.001397155 | 4596 | 1 |
| THYMU2010705 | 0.001397155 | 184170 | 1 |
| THYMU2010779 | 0.003983555 | 152956 | 3 |
| THYMU2010816 | 0.01285287 | 143581 | 5 |
| THYMU2010831 | 0.003072702 | 38475 | 2 |
| THYMU2010848 | 0.001397155 | 137753 | 1 |
| THYMU2010917 | 0.022248538 | 114970 | 7 |
| THYMU2011053 | 0.003273997 | 195135 | 2 |
| THYMU2011063 | 0.031843421 | 22493 | 5 |
| THYMU2011072 | 0.001397155 | 102364 | 1 |
| THYMU2011096 | 0.001397155 | 180700 | 1 |
| THYMU2011118 | 0.001397155 | 123798 | 1 |
| THYMU2011142 | 0.004191466 | 172498 | 3 |
| THYMU2011183 | 0.006985777 | 98200 | 5 |
| THYMU2011257 | 0.001397155 | 192913 | 1 |
| THYMU2011259 | 0.001397155 | 79034 | 1 |
| THYMU2011295 | 0.001397155 | 183201 | 1 |
| THYMU2011308 | 0.039913836 | 116322 | 3 |
| THYMU2011390 | 0.001397155 | 29218 | 1 |
| THYMU2011431 | 0.001397155 | 172672 | 1 |
| THYMU2011447 | 0.001397155 | 153158 | 1 |
| THYMU2011482 | 0.054584543 | 71775 | 17 |
| THYMU2011538 | 0.001397155 | 168902 | 1 |
| THYMU2011548 | 0.001397155 | 204771 | 1 |
| THYMU2011551 | 0.001397155 | 127496 | 1 |
| THYMU2011564 | 0.001397155 | 40916 | 1 |
| THYMU2011573 | 0.001397155 | 155100 | 1 |
| THYMU2011585 | 0.004191466 | 264324 | 3 |
| THYMU2011621 | 0.001397155 | 154007 | 1 |
| THYMU2011666 | 0.011177243 | 163294 | 8 |
| THYMU2011736 | 0.00557409 | 72233 | 2 |
| THYMU2011785 | 0.001397155 | 180698 | 1 |
| THYMU2011806 | 0.001397155 | 148285 | 1 |
| THYMU2011852 | 0.001397155 | 152430 | 1 |
| THYMU2011875 | 0.007469538 | 199092 | 2 |
| THYMU2011881 | 0.016073126 | 156819 | 3 |
| THYMU2011937 | 0.001397155 | 279317 | 1 |
| THYMU2011939 | 0.0025864 | 203373 | 2 |
| THYMU2012002 | 0.006599474 | 14979 | 3 |
| THYMU2012024 | 0.056226295 | 114189 | 15 |
| THYMU2012073 | 0.001397155 | 61274 | 1 |
| THYMU2012104 | 0.006193509 | 38205 | 3 |
| THYMU2012113 | 0.001397155 | 113778 | 1 |
| THYMU2012273 | 0.001397155 | 3666 | 1 |
| THYMU2012401 | 0.007238618 | 195013 | 2 |
| THYMU2012492 | 0.012815491 | 112715 | 7 |
| THYMU2012507 | 0.001397155 | 172871 | 1 |
| THYMU2012565 | 0.001397155 | 158110 | 1 |
| THYMU2012574 | 0.001397155 | 118116 | 1 |
| THYMU2012625 | 0.161294528 | 124991 | 11 |
| THYMU2012631 | 0.002794311 | 172294 | 2 |
| THYMU2012665 | 0.001397155 | 148278 | 1 |
| THYMU2012690 | 0.043119814 | 68520 | 4 |
| THYMU2012701 | 0.001397155 | 188948 | 1 |
| THYMU2012807 | 0.001397155 | 129598 | 1 |
| THYMU2012826 | 0.007565717 | 154250 | 5 |
| THYMU2012882 | 0.001397155 | 158285 | 1 |
| THYMU2012891 | 0.001397155 | 155982 | 1 |
| THYMU2012902 | 0.001397155 | 159799 | 1 |
| THYMU2012919 | 0.001397155 | 108607 | 1 |
| THYMU2013023 | 0.001397155 | 177290 | 1 |
| THYMU2013047 | 0.001397155 | 172911 | 1 |
| THYMU2013051 | 0.007438721 | 76120 | 2 |
| THYMU2013089 | 0.001397155 | 32491 | 1 |
| THYMU2013136 | 0.204858728 | 36632 | 20 |
| THYMU2013170 | 0.007309417 | 177296 | 2 |
| THYMU2013364 | 0.001397155 | 165267 | 1 |
| THYMU2013386 | 0.001397155 | 133799 | 1 |
| THYMU2013426 | 0.001397155 | 167318 | 1 |
| THYMU2013578 | 0.001397155 | 221338 | 1 |
| THYMU2013648 | 0.013991614 | 77052 | 2 |
| THYMU2013705 | 0.001397155 | 71287 | 1 |
| THYMU2013757 | 0.001397155 | 150630 | 1 |
| THYMU2013779 | 0.001397155 | 3699 | 1 |
| THYMU2013863 | 0.001397155 | 32449 | 1 |
| THYMU2013916 | 0.151068608 | 41565 | 42 |
| THYMU2013950 | 0.001397155 | 108348 | 1 |
| THYMU2013981 | 0.001397155 | 158094 | 1 |
| THYMU2014051 | 0.059603321 | 107170 | 4 |
| THYMU2014067 | 0.011105893 | 111895 | 2 |
| THYMU2014068 | 0.001397155 | 274078 | 1 |
| THYMU2014167 | 0.030491603 | 20856 | 9 |
| THYMU2014183 | 0.017246884 | 99909 | 4 |
| THYMU2014204 | 0.001397155 | 165302 | 1 |
| THYMU2014297 | 0.001397155 | 68839 | 1 |
| THYMU2014323 | 0.003085432 | 181696 | 2 |
| THYMU2014327 | 0.001397155 | 49618 | 1 |
| THYMU2014353 | 0.004482587 | 170502 | 3 |
| THYMU2014492 | 0.008706922 | 93447 | 3 |
| THYMU2014500 | 0.001397155 | 167359 | 1 |
| THYMU2014599 | 0.005915117 | 42203 | 3 |
| THYMU2014762 | 0.001397155 | 177203 | 1 |
| THYMU2014777 | 0.001397155 | 269518 | 1 |
| THYMU2014801 | 0.001397155 | 167368 | 1 |
| THYMU2014901 | 0.001397155 | 167331 | 1 |
| THYMU2014923 | 0.001397155 | 183375 | 1 |
| THYMU2015019 | 0.012248787 | 154608 | 3 |
| THYMU2015161 | 0.001397155 | 118937 | 1 |
| THYMU2015316 | 0.001397155 | 123494 | 1 |
| THYMU2015321 | 0.003395557 | 102346 | 2 |
| THYMU2015409 | 0.103117687 | 50384 | 38 |
| THYMU2015439 | 0.001397155 | 157715 | 1 |
| THYMU2015441 | 0.007451524 | 41373 | 2 |
| THYMU2015479 | 0.001397155 | 153197 | 1 |
| THYMU2015762 | 0.169012346 | 57682 | 13 |
| THYMU2015775 | 0.007542681 | 61993 | 2 |
| THYMU2015825 | 0.001397155 | 143702 | 1 |
| THYMU2015918 | 0.003106616 | 97379 | 2 |
| THYMU2015943 | 0.001397155 | 155327 | 1 |
| THYMU2015984 | 0.001397155 | 207 | 1 |
| THYMU2016113 | 0.001397155 | 125341 | 1 |
| THYMU2016164 | 0.001397155 | 72624 | 1 |
| THYMU2016204 | 0.003478667 | 144129 | 2 |
| THYMU2016219 | 0.001397155 | 149802 | 1 |
| THYMU2016360 | 0.001397155 | 165066 | 1 |
| THYMU2016496 | 0.001397155 | 93566 | 1 |
| THYMU2016523 | 0.005078834 | 170568 | 4 |
| THYMU2016840 | 0.007451524 | 37090 | 2 |
| THYMU2016968 | 0.022668431 | 82586 | 3 |
| THYMU2017008 | 0.001397155 | 103669 | 1 |
| THYMU2017010 | 0.001397155 | 89229 | 1 |
| THYMU2017023 | 0.001397155 | 233892 | 1 |
| THYMU2017085 | 0.001397155 | 4204 | 1 |
| THYMU2017099 | 0.001397155 | 14548 | 1 |
| THYMU2017158 | 0.003478667 | 272717 | 2 |
| THYMU2017162 | 0.001397155 | 205058 | 1 |
| THYMU2017215 | 0.001397155 | 64019 | 1 |
| THYMU2017362 | 0.001397155 | 172453 | 1 |
| THYMU2017366 | 0.001397155 | 62106 | 1 |
| THYMU2017398 | 0.001397155 | 150631 | 1 |
| THYMU2017449 | 0.001397155 | 183255 | 1 |
| THYMU2017479 | 0.002794311 | 253512 | 2 |
| THYMU2017522 | 0.001397155 | 276059 | 1 |
| THYMU2017526 | 0.001397155 | 183254 | 1 |
| THYMU2017601 | 0.023726744 | 98543 | 9 |
| THYMU2017675 | 0.001397155 | 142928 | 1 |
| THYMU2017707 | 0.001397155 | 637 | 1 |
| THYMU2017831 | 0.002492434 | 125343 | 2 |
| THYMU2017844 | 0.001397155 | 264332 | 1 |
| THYMU2017878 | 0.0025864 | 88949 | 2 |
| THYMU2017948 | 0.003889589 | 15722 | 3 |
| THYMU2018028 | 0.00557409 | 13810 | 2 |
| THYMU2018071 | 0.001397155 | 141318 | 1 |
| THYMU2018126 | 0.230309261 | 51293 | 10 |
| THYMU2018189 | 0.001397155 | 123002 | 1 |
| THYMU2018197 | 0.001397155 | 15485 | 1 |
| THYMU2018384 | 0.001397155 | 113825 | 1 |
| THYMU2018455 | 0.001397155 | 47567 | 1 |
| THYMU2018547 | 0.001397155 | 172009 | 1 |
| THYMU2018565 | 0.001397155 | 108939 | 1 |
| THYMU2018639 | 0.004312012 | 134868 | 2 |
| THYMU2018673 | 0.001397155 | 148629 | 1 |
| THYMU2018721 | 0.001397155 | 172044 | 1 |
| THYMU2018772 | 0.001397155 | 47847 | 1 |
| THYMU2018819 | 0.001397155 | 47624 | 1 |
| THYMU2018841 | 0.001397155 | 170636 | 1 |
| THYMU2018997 | 0.001397155 | 152079 | 1 |
| THYMU2019021 | 0.03447783 | 103041 | 11 |
| THYMU2019054 | 0.001397155 | 150277 | 1 |
| THYMU2019197 | 0.001397155 | 150862 | 1 |
| THYMU2019210 | 0.001397155 | 73263 | 1 |
| THYMU2019364 | 0.003983555 | 74654 | 3 |
| THYMU2019436 | 0.001397155 | 117914 | 1 |
| THYMU2019442 | 0.001397155 | 122289 | 1 |
| THYMU2019587 | 0.001397155 | 281612 | 1 |
| THYMU2019599 | 0.001397155 | 229715 | 1 |
| THYMU2019686 | 0.002794311 | 158378 | 2 |
| THYMU2019813 | 0.001397155 | 214718 | 1 |
| THYMU2020198 | 0.001397155 | 145741 | 1 |
| THYMU2020289 | 0.001397155 | 108295 | 1 |
| THYMU2020416 | 0.012224353 | 29776 | 2 |
| THYMU2020491 | 0.001397155 | 67968 | 1 |
| THYMU2020499 | 0.001397155 | 251158 | 1 |
| THYMU2020560 | 0.001397155 | 130385 | 1 |
| THYMU2020667 | 0.004792712 | 86246 | 3 |
| THYMU2020830 | 0.001397155 | 269268 | 1 |
| THYMU2020883 | 0.00325527 | 181191 | 2 |
| THYMU2020959 | 0.015114984 | 88942 | 8 |
| THYMU2021361 | 0.001397155 | 34692 | 1 |
| THYMU2021509 | 0.001397155 | 238302 | 1 |
| THYMU2021597 | 0.001397155 | 57027 | 1 |
| THYMU2021684 | 0.003395557 | 127638 | 2 |
| THYMU2021714 | 0.023751642 | 103392 | 17 |
| THYMU2022213 | 0.025148797 | 114440 | 18 |
| THYMU2022289 | 0.004191466 | 73994 | 3 |
| THYMU2022660 | 0.007309417 | 172365 | 2 |
| THYMU2022854 | 0.001397155 | 278606 | 1 |
| THYMU2022922 | 0.001397155 | 116006 | 1 |
| THYMU2023209 | 0.001397155 | 116973 | 1 |
| THYMU2023264 | 0.003085432 | 41729 | 2 |
| THYMU2023576 | 0.010511424 | 85838 | 4 |
| THYMU2023711 | 0.001397155 | 274871 | 1 |
| THYMU2023900 | 0.001397155 | 94568 | 1 |
| THYMU2023943 | 0.003072702 | 5637 | 2 |
| THYMU2023967 | 0.005485104 | 101070 | 4 |
| THYMU2024071 | 0.005083833 | 100557 | 3 |
| THYMU2024121 | 0.005709168 | 41693 | 3 |
| THYMU2024185 | 0.001397155 | 164169 | 1 |
| THYMU2024207 | 0.001397155 | 238321 | 1 |
| THYMU2024616 | 0.001397155 | 96296 | 1 |
| THYMU2024684 | 0.001397155 | 192803 | 1 |
| THYMU2024748 | 0.004312012 | 108109 | 2 |
| THYMU2025042 | 0.001397155 | 266025 | 1 |
| THYMU2025189 | 0.001397155 | 282943 | 1 |
| THYMU2025319 | 0.005071104 | 100575 | 3 |
| THYMU2025325 | 0.012865291 | 144720 | 4 |
| THYMU2025406 | 0.002794311 | 243509 | 2 |
| THYMU2025557 | 0.002794311 | 221682 | 2 |
| THYMU2025572 | 0.001397155 | 199890 | 1 |
| THYMU2025707 | 0.018109526 | 131155 | 5 |
| THYMU2025849 | 0.001397155 | 221783 | 1 |
| THYMU2025909 | 0.001397155 | 197564 | 1 |
| THYMU2025939 | 0.010506348 | 82423 | 6 |
| THYMU2026182 | 0.001397155 | 232555 | 1 |
| THYMU2026276 | 0.034799554 | 150747 | 6 |
| THYMU2026350 | 0.003478667 | 8878 | 2 |
| THYMU2026530 | 0.001397155 | 141411 | 1 |
| THYMU2026531 | 0.001397155 | 266042 | 1 |
| THYMU2026835 | 0.003106616 | 60477 | 2 |
| THYMU2026904 | 0.001397155 | 41947 | 1 |
| THYMU2027053 | 0.001397155 | 200314 | 1 |
| THYMU2027125 | 0.936089559 | 75254 | 93 |
| THYMU2027168 | 0.001397155 | 63852 | 1 |
| THYMU2027249 | 0.001397155 | 263118 | 1 |
| THYMU2027282 | 0.001397155 | 285128 | 1 |
| THYMU2027497 | 0.001397155 | 227955 | 1 |
| THYMU2027695 | 0.001397155 | 277390 | 1 |
| THYMU2027734 | 0.001397155 | 177440 | 1 |
| THYMU2027894 | 0.001397155 | 92500 | 1 |
| THYMU2027975 | 0.04192001 | 34801 | 4 |
| THYMU2027996 | 0.001397155 | 67047 | 1 |
| THYMU2028041 | 0.001397155 | 204986 | 1 |
| THYMU2028368 | 0.001397155 | 261422 | 1 |
| THYMU2028379 | 0.001397155 | 262180 | 1 |
| THYMU2028394 | 0.002794311 | 160498 | 2 |
| THYMU2028412 | 0.001397155 | 247973 | 1 |
| THYMU2028629 | 0.009152086 | 94446 | 4 |
| THYMU2028632 | 0.002794311 | 208151 | 2 |
| THYMU2028724 | 0.004191466 | 183160 | 3 |
| THYMU2028739 | 0.002492434 | 194795 | 2 |
| THYMU2028942 | 0.001397155 | 196605 | 1 |
| THYMU2028978 | 0.001397155 | 205453 | 1 |
| THYMU2029134 | 0.001397155 | 220850 | 1 |
| THYMU2029578 | 0.024368135 | 8483 | 8 |
| THYMU2029676 | 0.001397155 | 184010 | 1 |
| THYMU2029688 | 0.001397155 | 208058 | 1 |
| THYMU2029788 | 0.001397155 | 233730 | 1 |
| THYMU2030068 | 0.001397155 | 229555 | 1 |
| THYMU2030226 | 0.001397155 | 244940 | 1 |
| THYMU2030264 | 0.0025864 | 121308 | 2 |
| THYMU2030462 | 0.001397155 | 233721 | 1 |
| THYMU2030543 | 0.002794311 | 216046 | 2 |
| THYMU2030637 | 0.001397155 | 138915 | 1 |
| THYMU2030655 | 0.007599475 | 184733 | 2 |
| THYMU2030796 | 0.005588622 | 95006 | 4 |
| THYMU2030912 | 0.001397155 | 272830 | 1 |
| THYMU2030982 | 0.001397155 | 244313 | 1 |
| THYMU2031046 | 0.001397155 | 197635 | 1 |
| THYMU2031139 | 0.004191466 | 177398 | 3 |
| THYMU2031218 | 0.002794311 | 17405 | 2 |
| THYMU2031249 | 0.001397155 | 4838 | 1 |
| THYMU2031258 | 0.001397155 | 114579 | 1 |
| THYMU2031341 | 0.048561028 | 17717 | 7 |
| THYMU2031368 | 0.036921134 | 140956 | 2 |
| THYMU2031579 | 0.001397155 | 259836 | 1 |
| THYMU2031847 | 0.004517962 | 123862 | 2 |
| THYMU2031890 | 0.009437123 | 212786 | 3 |
| THYMU2031994 | 0.001397155 | 229447 | 1 |
| THYMU2032014 | 0.010153475 | 68210 | 3 |
| THYMU2032035 | 0.001397155 | 251947 | 1 |
| THYMU2032080 | 0.001397155 | 167803 | 1 |
| THYMU2032150 | 0.001397155 | 192260 | 1 |
| THYMU2032155 | 0.023463307 | 177671 | 3 |
| THYMU2032358 | 0.001397155 | 270510 | 1 |
| THYMU2032437 | 0.001397155 | 252888 | 1 |
| THYMU2032601 | 0.001397155 | 250248 | 1 |
| THYMU2032655 | 0.001397155 | 100967 | 1 |
| THYMU2032696 | 0.159436907 | 73641 | 5 |
| THYMU2032715 | 0.002794311 | 167794 | 2 |
| THYMU2032732 | 0.001397155 | 131410 | 1 |
| THYMU2032825 | 0.018022094 | 93062 | 5 |
| THYMU2032976 | 0.001397155 | 92105 | 1 |
| THYMU2033053 | 0.002794311 | 12336 | 2 |
| THYMU2033059 | 0.007542681 | 183282 | 2 |
| THYMU2033070 | 0.003395557 | 184137 | 2 |
| THYMU2033079 | 0.001397155 | 36227 | 1 |
| THYMU2033085 | 0.001397155 | 184138 | 1 |
| THYMU2033104 | 0.001397155 | 73557 | 1 |
| THYMU2033227 | 0.001397155 | 262649 | 1 |
| THYMU2033263 | 0.015061645 | 226759 | 3 |
| THYMU2033308 | 0.011998687 | 161261 | 4 |
| THYMU2033401 | 0.001397155 | 184126 | 1 |
| THYMU2033583 | 0.003273997 | 254440 | 2 |
| THYMU2033755 | 0.001397155 | 222864 | 1 |
| THYMU2033787 | 0.00325527 | 227276 | 2 |
| THYMU2033816 | 0.001397155 | 172896 | 1 |
| THYMU2034182 | 0.001397155 | 274695 | 1 |
| THYMU2034279 | 0.001397155 | 255055 | 1 |
| THYMU2034314 | 0.001397155 | 220972 | 1 |
| THYMU2034338 | 0.001397155 | 238794 | 1 |
| THYMU2034374 | 0.01332012 | 126712 | 4 |
| THYMU2034647 | 0.004312012 | 172045 | 2 |
| THYMU2034781 | 0.010453737 | 104768 | 3 |
| THYMU2034917 | 0.008623084 | 125330 | 4 |
| THYMU2035064 | 0.001397155 | 199880 | 1 |
| THYMU2035078 | 0.001397155 | 172357 | 1 |
| THYMU2035101 | 0.001397155 | 193574 | 1 |
| THYMU2035319 | 0.001397155 | 178820 | 1 |
| THYMU2035321 | 0.001397155 | 188537 | 1 |
| THYMU2035388 | 0.001397155 | 208469 | 1 |
| THYMU2035400 | 0.001397155 | 184158 | 1 |
| THYMU2035554 | 0.001397155 | 238425 | 1 |
| THYMU2035710 | 0.027943108 | 71638 | 20 |
| THYMU2035735 | 0.127009852 | 96991 | 25 |
| THYMU2036058 | 0.002492434 | 185426 | 2 |
| THYMU2036085 | 0.037910844 | 98201 | 26 |
| THYMU2036105 | 0.001397155 | 193525 | 1 |
| THYMU2036207 | 0.001397155 | 199969 | 1 |
| THYMU2036215 | 0.007283551 | 179703 | 3 |
| THYMU2036252 | 0.001397155 | 229624 | 1 |
| THYMU2036265 | 0.001397155 | 188589 | 1 |
| THYMU2036366 | 0.001397155 | 254304 | 1 |
| THYMU2036459 | 0.001397155 | 121224 | 1 |
| THYMU2036461 | 0.011047141 | 11120 | 4 |
| THYMU2036604 | 0.001397155 | 199956 | 1 |
| THYMU2036653 | 0.001397155 | 153063 | 1 |
| THYMU2037081 | 0.001397155 | 209632 | 1 |
| THYMU2037208 | 0.001397155 | 20695 | 1 |
| THYMU2037226 | 0.009778349 | 66216 | 6 |
| THYMU2037233 | 0.001397155 | 239841 | 1 |
| THYMU2037234 | 0.002794311 | 220922 | 2 |
| THYMU2037348 | 0.001397155 | 241529 | 1 |
| THYMU2037406 | 0.001397155 | 267882 | 1 |
| THYMU2037965 | 0.001397155 | 214770 | 1 |
| THYMU2038058 | 0.001397155 | 231485 | 1 |
| THYMU2038189 | 0.001397155 | 21732 | 1 |
| THYMU2038199 | 0.001397155 | 204689 | 1 |
| THYMU2038220 | 0.008294641 | 137162 | 5 |
| THYMU2038301 | 0.001397155 | 182878 | 1 |
| THYMU2038369 | 0.001397155 | 172758 | 1 |
| THYMU2038389 | 0.001397155 | 184123 | 1 |
| THYMU2038615 | 0.001397155 | 193215 | 1 |
| THYMU2038636 | 0.001397155 | 272458 | 1 |
| THYMU2038739 | 0.001397155 | 270730 | 1 |
| THYMU2038772 | 0.001397155 | 188450 | 1 |
| THYMU2038797 | 0.001397155 | 266841 | 1 |
| THYMU2038906 | 0.001397155 | 198130 | 1 |
| THYMU2039305 | 0.002794311 | 39776 | 2 |
| THYMU2039315 | 0.003072702 | 141642 | 2 |
| THYMU2039334 | 0.147595986 | 233500 | 2 |
| THYMU2039350 | 0.004984867 | 165436 | 4 |
| THYMU2039411 | 0.001397155 | 225544 | 1 |
| THYMU2039634 | 0.003072702 | 138814 | 2 |
| THYMU2039719 | 0.001397155 | 242232 | 1 |
| THYMU2039780 | 0.0025864 | 254828 | 2 |
| THYMU2039788 | 0.001397155 | 124118 | 1 |
| THYMU2039989 | 0.001397155 | 58760 | 1 |
| THYMU2040029 | 0.004191466 | 154293 | 3 |
| THYMU2040110 | 0.001397155 | 256423 | 1 |
| THYMU2040114 | 0.005241239 | 59645 | 2 |
| THYMU2040140 | 0.001397155 | 172727 | 1 |
| THYMU2040412 | 0.001397155 | 164380 | 1 |
| THYMU2040427 | 0.0025864 | 157139 | 2 |
| THYMU2040824 | 0.001397155 | 192843 | 1 |
| THYMU2040925 | 0.0025864 | 34061 | 2 |
| THYMU2040975 | 0.095383222 | 127390 | 21 |
| THYMU2041007 | 0.001397155 | 253683 | 1 |
| THYMU2041015 | 0.001397155 | 274152 | 1 |
| THYMU2041252 | 0.001397155 | 240389 | 1 |
| THYMU3000028 | 0.004191466 | 89740 | 3 |
| THYMU3000036 | 0.001397155 | 281537 | 1 |
| THYMU3000037 | 0.002794311 | 279909 | 2 |
| THYMU3000082 | 0.002794311 | 255343 | 2 |
| THYMU3000133 | 0.034845616 | 114406 | 15 |
| THYMU3000194 | 0.001397155 | 283357 | 1 |
| THYMU3000224 | 0.002794311 | 136766 | 2 |
| THYMU3000269 | 0.001397155 | 282068 | 1 |
| THYMU3000306 | 0.001397155 | 242994 | 1 |
| THYMU3000360 | 0.004261947 | 128157 | 3 |
| THYMU3000390 | 0.001397155 | 272919 | 1 |
| THYMU3000420 | 0.004261947 | 76550 | 3 |
| THYMU3000490 | 0.001397155 | 249970 | 1 |
| THYMU3000504 | 0.001397155 | 209467 | 1 |
| THYMU3000542 | 0.001397155 | 231345 | 1 |
| THYMU3000655 | 0.003273997 | 110631 | 2 |
| THYMU3000776 | 0.010737936 | 110399 | 8 |
| THYMU3000826 | 0.001397155 | 245778 | 1 |
| THYMU3000841 | 0.001397155 | 242939 | 1 |
| THYMU3001077 | 0.00557409 | 216640 | 2 |
| THYMU3001082 | 0.001397155 | 238367 | 1 |
| THYMU3001083 | 0. 0025864 | 168993 | 2 |
| THYMU3001133 | 0.001397155 | 251623 | 1 |
| THYMU3001151 | 0.001397155 | 240225 | 1 |
| THYMU3001234 | 0.015114984 | 88942 | 8 |
| THYMU3001379 | 0.006653007 | 52793 | 4 |
| THYMU3001428 | 0.470539281 | 86556 | 72 |
| THYMU3001472 | 0.009943551 | 129060 | 6 |
| THYMU3001593 | 0.001397155 | 253783 | 1 |
| THYMU3001776 | 0.052878615 | 53485 | 12 |
| THYMU3001883 | 0.001397155 | 64251 | 1 |
| THYMU3001939 | 0.003478667 | 202620 | 2 |
| THYMU3001991 | 0.001397155 | 127266 | 1 |
| THYMU3002452 | 0.001397155 | 258017 | 1 |
| THYMU3002578 | 0.005477069 | 133316 | 3 |
| THYMU3002661 | 0.001397155 | 122970 | 1 |
| THYMU3002887 | 0.214802958 | 50355 | 66 |
| THYMU3003007 | 0.008815783 | 153762 | 5 |
| THYMU3003092 | 0.002794311 | 94638 | 2 |
| THYMU3003212 | 0.001397155 | 238933 | 1 |
| THYMU3003309 | 0.00576319 | 160112 | 4 |
| THYMU3003350 | 0.003072702 | 121425 | 2 |
| THYMU3003403 | 0.002794311 | 164217 | 2 |
| THYMU3003565 | 0.005709168 | 78023 | 3 |
| THYMU3003763 | 0.001397155 | 193299 | 1 |
| THYMU3003865 | 0.007968825 | 111888 | 7 |
| THYMU3003958 | 0.001397155 | 197699 | 1 |
| THYMU3003965 | 0.003106616 | 197687 | 2 |
| THYMU3004157 | 0.001397155 | 56980 | 1 |
| THYMU3004628 | 0.005588622 | 142583 | 4 |
| THYMU3004632 | 0.001397155 | 251461 | 1 |
| THYMU3004835 | 0.008529848 | 99747 | 5 |
| THYMU3004866 | 0.001397155 | 254944 | 1 |
| THYMU3005050 | 0.001397155 | 216432 | 1 |
| THYMU3005378 | 0.001397155 | 248901 | 1 |
| THYMU3005407 | 0.001397155 | 284284 | 1 |
| THYMU3005415 | 0.001397155 | 228278 | 1 |
| THYMU3005427 | 0.007099354 | 205639 | 3 |
| THYMU3005482 | 0.001397155 | 281535 | 1 |
| THYMU3005491 | 0.00325527 | 122750 | 2 |
| THYMU3005629 | 0.011089072 | 157105 | 4 |
| THYMU3005696 | 0.001397155 | 248935 | 1 |
| THYMU3006118 | 0.001397155 | 233478 | 1 |
| THYMU3006132 | 0.001397155 | 132151 | 1 |
| THYMU3006168 | 0.001397155 | 231366 | 1 |
| THYMU3006172 | 0.003273997 | 42739 | 2 |
| THYMU3006367 | 0.016874324 | 40087 | 11 |
| THYMU3006371 | 0.001397155 | 278659 | 1 |
| THYMU3006485 | 0.0025864 | 244910 | 2 |
| THYMU3006640 | 0.001397155 | 192871 | 1 |
| THYMU3006767 | 0.003273997 | 209531 | 2 |
| THYMU3006811 | 0.003273997 | 142631 | 2 |
| THYMU3006963 | 0.001397155 | 254993 | 1 |
| THYMU3007137 | 0.004503771 | 64399 | 3 |
| THYMU3007308 | 0.00429586 | 218367 | 3 |
| THYMU3007368 | 0.001397155 | 260613 | 1 |
| THYMU3007423 | 0.017041013 | 50722 | 11 |
| THYMU3007559 | 0.001397155 | 221683 | 1 |
| THYMU3007845 | 0.001397155 | 278117 | 1 |
| THYMU3008060 | 0.001397155 | 245851 | 1 |
| THYMU3008105 | 0.00827356 | 114480 | 5 |
| THYMU3008136 | 0.001397155 | 47542 | 1 |
| THYMU3008171 | 0.016613967 | 92879 | 11 |
| THYMU3008216 | 0.001397155 | 231250 | 1 |
| THYMU3008436 | 0.001397155 | 42825 | 1 |
| THYMU3008672 | 0.001397155 | 258715 | 1 |
| THYMU3008883 | 0.006887889 | 34707 | 3 |
| THYMU3008935 | 0.001397155 | 271592 | 1 |
| THYMU3009006 | 0.00903154 | 230206 | 5 |
| THYMU3009255 | 0.005588622 | 219921 | 4 |
| THYMU3009391 | 0.001397155 | 144778 | 1 |
| THYMU3009643 | 0.006873547 | 116956 | 6 |
| THYMU3009755 | 0.001397155 | 198404 | 1 |
| THYMU3010247 | 0.001397155 | 218601 | 1 |
| THYMU3011012 | 0.001397155 | 90214 | 1 |
| THYMU3011081 | 0.001397155 | 274837 | 1 |
| THYMU3011244 | 0.001397155 | 236663 | 1 |
| THYMU3011360 | 0.002794311 | 196120 | 2 |
| THYMU3011534 | 0.001397155 | 244009 | 1 |
| THYMU3011543 | 0.001397155 | 142963 | 1 |
| THYMU3011556 | 0.001397155 | 270144 | 1 |
| THYMU3011717 | 0.001397155 | 88461 | 1 |
| THYMU3011827 | 0.007213623 | 214964 | 4 |
| THYMU3012402 | 0.001397155 | 53213 | 1 |
| THYMU3012907 | 0.001397155 | 219975 | 1 |
| THYMU3012983 | 0.002492434 | 259506 | 2 |
| THYMU3013114 | 0.001397155 | 260644 | 1 |
| THYMU3013197 | 0.001397155 | 236768 | 1 |
| THYMU3013241 | 0.002794311 | 129264 | 2 |
| THYMU3013386 | 0.223840198 | 39993 | 27 |
| THYMU3013470 | 0.001397155 | 243210 | 1 |
| THYMU3013785 | 0.001397155 | 17361 | 1 |
| THYMU3013897 | 0.001397155 | 263083 | 1 |
| THYMU3014038 | 0.001397155 | 260643 | 1 |
| THYMU3014173 | 0.001397155 | 247110 | 1 |
| THYMU3014372 | 0.001397155 | 71015 | 1 |
| THYMU3014555 | 0.001397155 | 261227 | 1 |
| THYMU3014620 | 0.001397155 | 51142 | 1 |
| THYMU3014701 | 0.002794311 | 219505 | 2 |
| THYMU3015042 | 0.001397155 | 252403 | 1 |
| THYMU3015457 | 0.001397155 | 278317 | 1 |
| THYMU3015571 | 0.015952021 | 123613 | 4 |
| THYMU3015592 | 0.001397155 | 247487 | 1 |
| THYMU3015625 | 0.001397155 | 271811 | 1 |
| THYMU3015647 | 0.002794311 | 144757 | 2 |
| THYMU3015759 | 0.148820031 | 108232 | 27 |
| THYMU3015962 | 0.001397155 | 250559 | 1 |
| THYMU3016022 | 0.001397155 | 260638 | 1 |
| THYMU3016518 | 0.001397155 | 271631 | 1 |
| THYMU3016797 | 0.001397155 | 260637 | 1 |
| THYMU3016822 | 0.001397155 | 14922 | 1 |
| THYMU3016850 | 0.001397155 | 279641 | 1 |
| THYMU3017562 | 0.001397155 | 9018 | 1 |
| THYMU3017688 | 0.001397155 | 277462 | 1 |
| THYMU3017689 | 0.002794311 | 170289 | 2 |
| THYMU3017761 | 0.001397155 | 224947 | 1 |
| THYMU3018151 | 0.001397155 | 223138 | 1 |
| THYMU3018896 | 0.001397155 | 261995 | 1 |
| THYMU3019095 | 0.001397155 | 269072 | 1 |
| THYMU3019476 | 0.003085432 | 92851 | 2 |
| THYMU3019605 | 0.001397155 | 10308 | 1 |
| THYMU3019916 | 0.001397155 | 276273 | 1 |
| THYMU3020221 | 0.001397155 | 260925 | 1 |
| THYMU3020595 | 0.001397155 | 241191 | 1 |
| THYMU3020713 | 0.001397155 | 260917 | 1 |
| THYMU3020856 | 0.001397155 | 285613 | 1 |
| THYMU3020869 | 0.001397155 | 106746 | 1 |
| THYMU3020970 | 0.001397155 | 274340 | 1 |
| THYMU3021404 | 0.001397155 | 259469 | 1 |
| THYMU3021586 | 0.040568248 | 80135 | 22 |
| THYMU3021755 | 0.001397155 | 269248 | 1 |
| THYMU3021900 | 0.001397155 | 270538 | 1 |
| THYMU3022211 | 0.001397155 | 170307 | 1 |
| THYMU3022434 | 0.001397155 | 265498 | 1 |
| THYMU3022528 | 0.001397155 | 132499 | 1 |
| THYMU3022668 | 0.001397155 | 231465 | 1 |
| THYMU3022982 | 0.001397155 | 214108 | 1 |
| THYMU3023107 | 0.002794311 | 169106 | 2 |
| THYMU3023211 | 0.002794311 | 157013 | 2 |
| THYMU3023216 | 0.001397155 | 230218 | 1 |
| THYMU3023394 | 0.001397155 | 138438 | 1 |
| THYMU3023400 | 0.001397155 | 146064 | 1 |
| THYMU3023797 | 0.001397155 | 269007 | 1 |
| THYMU3024164 | 0.001397155 | 213683 | 1 |
| THYMU3024339 | 0.00577627 | 215668 | 3 |
| THYMU3024602 | 0.001397155 | 132919 | 1 |
| THYMU3024879 | 0.001397155 | 224994 | 1 |
| THYMU3025118 | 0.001397155 | 18456 | 1 |
| THYMU3025313 | 0.001397155 | 276374 | 1 |
| THYMU3025642 | 0.004379115 | 70496 | 2 |
| THYMU3025683 | 0.001397155 | 74463 | 1 |
| THYMU3025772 | 0.001397155 | 278490 | 1 |
| THYMU3025865 | 0.001397155 | 285103 | 1 |
| THYMU3026000 | 0.006829762 | 98173 | 4 |
| THYMU3026306 | 0.007240683 | 190039 | 5 |
| THYMU3026350 | 0.01172411 | 166294 | 7 |
| THYMU3026479 | 0.001397155 | 85839 | 1 |
| THYMU3026532 | 0.001397155 | 17882 | 1 |
| THYMU3026783 | 0.001397155 | 135389 | 1 |
| THYMU3026869 | 0.001397155 | 278722 | 1 |
| THYMU3027251 | 0.002794311 | 127981 | 2 |
| THYMU3027540 | 0.001397155 | 136129 | 1 |
| THYMU3027655 | 0.001397155 | 145781 | 1 |
| THYMU3027671 | 0.001397155 | 143757 | 1 |
| THYMU3028075 | 0.001397155 | 187873 | 1 |
| THYMU3028410 | 0.003395557 | 49146 | 2 |
| THYMU3028461 | 0.001397155 | 163347 | 1 |
| THYMU3028702 | 0.001397155 | 106155 | 1 |
| THYMU3029188 | 0.001397155 | 149629 | 1 |
| THYMU3029318 | 0.001397155 | 160083 | 1 |
| THYMU3029421 | 0.001397155 | 129683 | 1 |
| THYMU3029719 | 0.001397155 | 122082 | 1 |
| THYMU3029774 | 0.001397155 | 154357 | 1 |
| THYMU3029795 | 0.015748053 | 72286 | 7 |
| THYMU3029832 | 0.001397155 | 270238 | 1 |
| THYMU3030072 | 0.001397155 | 84183 | 1 |
| THYMU3030231 | 0.001397155 | 21751 | 1 |
| THYMU3030706 | 0.001397155 | 144069 | 1 |
| THYMU3030710 | 0.001397155 | 77815 | 1 |
| THYMU3030752 | 0.001397155 | 56810 | 1 |
| THYMU3031146 | 0.001397155 | 177001 | 1 |
| THYMU3031402 | 0.001397155 | 177004 | 1 |
| THYMU3031610 | 0.001397155 | 177049 | 1 |
| THYMU3031612 | 0.001397155 | 183171 | 1 |
| THYMU3031868 | 0.002794311 | 158871 | 2 |
| THYMU3031878 | 0.001397155 | 284935 | 1 |
| THYMU3032032 | 0.001397155 | 129852 | 1 |
| THYMU3032798 | 0.086652884 | 133051 | 35 |
| THYMU3032867 | 0.013018267 | 120560 | 5 |
| THYMU3033402 | 0.001397155 | 123558 | 1 |
| THYMU3033626 | 0.029810598 | 116011 | 10 |
| THYMU3033630 | 0.001397155 | 195868 | 1 |
| THYMU3033649 | 0.001397155 | 131517 | 1 |
| THYMU3033754 | 0.001397155 | 131916 | 1 |
| THYMU3033759 | 0.001397155 | 70779 | 1 |
| THYMU3034078 | 0.001397155 | 131197 | 1 |
| THYMU3034099 | 0.001397155 | 118889 | 1 |
| THYMU3034453 | 0.001397155 | 136730 | 1 |
| THYMU3034616 | 0.003395557 | 127270 | 2 |
| THYMU3034671 | 0.070255898 | 95008 | 29 |
| THYMU3034853 | 0.002794311 | 89354 | 2 |
| THYMU3034867 | 0.001397155 | 229912 | 1 |
| THYMU3034983 | 0.001397155 | 145517 | 1 |
| THYMU3036200 | 0.001397155 | 85883 | 1 |
| THYMU3036310 | 0.001397155 | 193684 | 1 |
| THYMU3036934 | 0.001397155 | 160193 | 1 |
| THYMU3036953 | 0.001397155 | 74270 | 1 |
| THYMU3037052 | 0.001397155 | 146848 | 1 |
| THYMU3037192 | 0.001397155 | 177752 | 1 |
| THYMU3037617 | 0.004191466 | 154344 | 3 |
| THYMU3037772 | 0.001397155 | 266207 | 1 |
| THYMU3037827 | 0.003072702 | 139321 | 2 |
| THYMU3037836 | 0.001397155 | 285085 | 1 |
| THYMU3037856 | 0.001397155 | 255912 | 1 |
| THYMU3037867 | 0.001397155 | 183027 | 1 |
| THYMU3037909 | 0.001397155 | 124163 | 1 |
| THYMU3037980 | 0.001397155 | 148801 | 1 |
| THYMU3038158 | 0.027208165 | 140556 | 3 |
| THYMU3038167 | 0.001397155 | 233991 | 1 |
| THYMU3038214 | 0.004261947 | 60776 | 3 |
| THYMU3038266 | 0.001397155 | 130507 | 1 |
| THYMU3038328 | 0.001397155 | 156764 | 1 |
| THYMU3038347 | 0.001397155 | 130763 | 1 |
| THYMU3038375 | 0.001397155 | 127530 | 1 |
| THYMU3038603 | 0.001397155 | 233997 | 1 |
| THYMU3038687 | 0.001397155 | 57684 | 1 |
| THYMU3038759 | 0.001397155 | 267875 | 1 |
| THYMU3038879 | 0.001397155 | 276171 | 1 |
| THYMU3038970 | 0.001397155 | 97478 | 1 |
| THYMU3039772 | 0.021240948 | 100742 | 8 |
| THYMU3039807 | 0.001397155 | 116444 | 1 |
| THYMU3039846 | 0.001397155 | 146174 | 1 |
| THYMU3040068 | 0.004312012 | 112312 | 2 |
| THYMU3040126 | 0.001397155 | 274276 | 1 |
| THYMU3040146 | 0.001397155 | 224857 | 1 |
| THYMU3040168 | 0.001397155 | 252069 | 1 |
| THYMU3040172 | 0.001397155 | 116297 | 1 |
| THYMU3040725 | 0.001397155 | 131903 | 1 |
| THYMU3040746 | 0.001397155 | 118726 | 1 |
| THYMU3040816 | 0.014458806 | 43541 | 2 |
| THYMU3040829 | 0.001397155 | 3719 | 1 |
| THYMU3040830 | 0.001397155 | 136652 | 1 |
| THYMU3040986 | 0.001397155 | 151752 | 1 |
| THYMU3041354 | 0.001397155 | 157035 | 1 |
| THYMU3041386 | 0.001397155 | 184312 | 1 |
| THYMU3041428 | 0.657483149 | 22293 | 8 |
| THYMU3041573 | 0.001397155 | 151887 | 1 |
| THYMU3041603 | 0.001397155 | 183090 | 1 |
| THYMU3041736 | 0.001397155 | 201763 | 1 |
| THYMU3041918 | 0.001397155 | 272277 | 1 |
| THYMU3042075 | 0.001397155 | 183111 | 1 |
| THYMU3042321 | 0.001397155 | 90204 | 1 |
| THYMU3042372 | 0.001397155 | 164318 | 1 |
| THYMU3042758 | 0.001397155 | 183047 | 1 |
| THYMU3042929 | 0.001397155 | 161461 | 1 |
| THYMU3043200 | 0.001397155 | 114621 | 1 |
| THYMU3043327 | 0.001397155 | 157126 | 1 |
| THYMU3043482 | 0.001397155 | 234573 | 1 |
| THYMU3043597 | 0.001397155 | 257792 | 1 |
| THYMU3043688 | 0.001397155 | 259521 | 1 |
| THYMU3043779 | 0.001397155 | 132990 | 1 |
| THYMU3043883 | 0.001397155 | 127344 | 1 |
| THYMU3043993 | 0.001397155 | 149288 | 1 |
| THYMU3044075 | 0.008836199 | 131462 | 6 |
| THYMU3044175 | 0.002794311 | 14683 | 2 |
| THYMU3044188 | 0.037433191 | 118200 | 2 |
| THYMU3044441 | 0.001397155 | 129615 | 1 |
| THYMU3044445 | 0.001397155 | 189960 | 1 |
| THYMU3045510 | 0.007106323 | 113478 | 4 |
| THYMU3045673 | 0.001397155 | 162132 | 1 |
| THYMU3045692 | 0.001397155 | 224840 | 1 |
| THYMU3045704 | 0.001397155 | 172359 | 1 |
| THYMU3046140 | 0.001397155 | 149532 | 1 |
| THYMU3046350 | 0.024256846 | 82081 | 6 |
| THYMU3046360 | 0.001397155 | 29998 | 1 |
| THYMU3046856 | 0.001397155 | 178900 | 1 |
| THYMU3047115 | 0.407756673 | 75470 | 37 |
| THYMU3047144 | 0.001397155 | 215565 | 1 |
| THYMU3047156 | 0.001397155 | 227560 | 1 |
| THYMU3047366 | 0.001397155 | 167763 | 1 |
| THYMU3047513 | 0.001397155 | 281819 | 1 |
| THYMU3047542 | 0.001397155 | 110020 | 1 |
| THYMU3047760 | 0.001397155 | 177694 | 1 |
| THYMU3047891 | 0.001397155 | 51202 | 1 |
| TKIDN1000001 | 0.015054857 | 60810 | 4 |
| TKIDN1000044 | 0.017892972 | 94496 | 5 |
| TKIDN1000062 | 0.00620232 | 47631 | 1 |
| TKIDN1000066 | 0.017489001 | 156289 | 2 |
| TKIDN1000164 | 0.027099988 | 113290 | 7 |
| TKIDN1000171 | 0.00620232 | 131961 | 1 |
| TKIDN1000192 | 0.030329007 | 18697 | 10 |
| TKIDN2000127 | 0.009247201 | 140638 | 2 |
| TKIDN2000240 | 0.00620232 | 121619 | 1 |
| TKIDN2000246 | 0.550803718 | 46185 | 133 |
| TKIDN2000319 | 0.00791178 | 146224 | 2 |
| TKIDN2000464 | 0.009117177 | 47879 | 2 |
| TKIDN2000521 | 0.011262874 | 75584 | 4 |
| TKIDN2000701 | 0.007297598 | 206043 | 2 |
| TKIDN2001529 | 0.008283832 | 198111 | 2 |
| TKIDN2001662 | 0.015363597 | 111377 | 6 |
| TKIDN2002318 | 0.03124095 | 122949 | 8 |
| TKIDN2002329 | 0.00620232 | 201319 | 1 |
| TKIDN2002424 | 0.00620232 | 198989 | 1 |
| TKIDN2002632 | 0.00620232 | 37890 | 1 |
| TKIDN2002738 | 0.399538298 | 153488 | 16 |
| TKIDN2003044 | 0.00620232 | 188868 | 1 |
| TKIDN2003059 | 0.00791178 | 202816 | 2 |
| TKIDN2003062 | 0.00620232 | 97689 | 1 |
| TKIDN2003078 | 0.012098717 | 222149 | 3 |
| TKIDN2003366 | 0.00620232 | 261303 | 1 |
| TKIDN2003396 | 0.00620232 | 152961 | 1 |
| TKIDN2003413 | 0.00620232 | 161507 | 1 |
| TKIDN2003597 | 0.021447808 | 58660 | 9 |
| TKIDN2003601 | 0.00878872 | 177417 | 3 |
| TKIDN2004185 | 0.042238356 | 180410 | 2 |
| TKIDN2004386 | 0.00620232 | 128375 | 1 |
| TKIDN2004458 | 0.012404639 | 114769 | 2 |
| TKIDN2004748 | 0.017327028 | 113838 | 2 |
| TKIDN2005487 | 0.233233081 | 132983 | 11 |
| TKIDN2005721 | 0.00620232 | 283957 | 1 |
| TKIDN2005934 | 0.00620232 | 147110 | 1 |
| TKIDN2005947 | 0.00620232 | 193356 | 1 |
| TKIDN2005956 | 0.00620232 | 110641 | 1 |
| TKIDN2006525 | 0.00620232 | 106071 | 1 |
| TKIDN2006761 | 0.007890596 | 233824 | 2 |
| TKIDN2006852 | 0.012404639 | 131268 | 2 |
| TKIDN2007605 | 0.013413037 | 74987 | 5 |
| TKIDN2007667 | 0.00620232 | 178842 | 1 |
| TKIDN2007828 | 0.025113053 | 157976 | 4 |
| TKIDN2008176 | 0.007890596 | 178917 | 2 |
| TKIDN2008778 | 0.00620232 | 271669 | 1 |
| TKIDN2009092 | 0.00620232 | 1703 | 1 |
| TKIDN2009330 | 0.008060434 | 147782 | 2 |
| TKIDN2009481 | 0.00620232 | 129039 | 1 |
| TKIDN2009641 | 0.028980479 | 143560 | 10 |
| TKIDN2009794 | 0.011612823 | 96189 | 4 |
| TKIDN2009889 | 0.009553414 | 142783 | 3 |
| TKIDN2010200 | 0.00620232 | 188293 | 1 |
| TKIDN2010232 | 0.00620232 | 199366 | 1 |
| TKIDN2010278 | 0.00620232 | 177480 | 1 |
| TKIDN2010602 | 0.00620232 | 194701 | 1 |
| TKIDN2010812 | 0.015716289 | 114231 | 6 |
| TKIDN2010934 | 0.008283832 | 160299 | 2 |
| TKIDN2011051 | 0.00620232 | 183324 | 1 |
| TKIDN2011160 | 0.00620232 | 245598 | 1 |
| TKIDN2011289 | 0.028028429 | 114038 | 7 |
| TKIDN2011324 | 0.00620232 | 215425 | 1 |
| TKIDN2012034 | 0.00620232 | 232588 | 1 |
| TKIDN2012771 | 0.00620232 | 48516 | 1 |
| TKIDN2012824 | 0.00620232 | 192579 | 1 |
| TKIDN2013134 | 0.00620232 | 259362 | 1 |
| TKIDN2013287 | 0.009680987 | 150912 | 3 |
| TKIDN2014570 | 0.007877867 | 234722 | 2 |
| TKIDN2014757 | 0.00620232 | 211344 | 1 |
| TKIDN2014771 | 0.00620232 | 222655 | 1 |
| TKIDN2014964 | 0.015288767 | 153329 | 3 |
| TKIDN2015025 | 0.00620232 | 188929 | 1 |
| TKIDN2015071 | 0.012917237 | 59938 | 5 |
| TKIDN2015161 | 0.00620232 | 207382 | 1 |
| TKIDN2015263 | 0.00620232 | 268949 | 1 |
| TKIDN2015285 | 0.016877602 | 198337 | 4 |
| TKIDN2015423 | 0.263199246 | 78702 | 135 |
| TKIDN2015788 | 0.063699701 | 124561 | 12 |
| TKIDN2016309 | 0.00620232 | 211313 | 1 |
| TKIDN2016399 | 0.01772173 | 188903 | 2 |
| TKIDN2016846 | 0.007391564 | 170791 | 2 |
| TKIDN2018926 | 0.007890596 | 199031 | 2 |
| TKIDN2019116 | 0.00620232 | 147919 | 1 |
| TLIVE2000023 | 0.01151941 | 142264 | 1 |
| TLIVE2000142 | 0.01151941 | 184600 | 1 |
| TLIVE2000292 | 0.01151941 | 43746 | 1 |
| TLIVE2000762 | 0.01151941 | 270153 | 1 |
| TLIVE2000979 | 0.015696345 | 112781 | 2 |
| TLIVE2001327 | 0.035724133 | 172673 | 4 |
| TLIVE2001616 | 0.01151941 | 210981 | 1 |
| TLIVE2001684 | 0.01151941 | 143407 | 1 |
| TLIVE2001828 | 0.012916566 | 217660 | 2 |
| TLIVE2001927 | 0.01151941 | 205909 | 1 |
| TLIVE2002046 | 0.092318972 | 41471 | 36 |
| TLIVE2002336 | 0.046077641 | 195878 | 4 |
| TLIVE2002338 | 0.01151941 | 170643 | 1 |
| TLIVE2002562 | 0.01151941 | 156517 | 1 |
| TLIVE2002690 | 0.08341079 | 159249 | 7 |
| TLIVE2002882 | 0.01151941 | 73608 | 1 |
| TLIVE2003197 | 0.01151941 | 221117 | 1 |
| TLIVE2003200 | 0.01322887 | 263530 | 2 |
| TLIVE2003225 | 0.01151941 | 232147 | 1 |
| TLIVE2003381 | 0.01151941 | 197238 | 1 |
| TLIVE2003737 | 0.01151941 | 190352 | 1 |
| TLIVE2003961 | 0.01151941 | 195044 | 1 |
| TLIVE2003970 | 0.072446202 | 127223 | 6 |
| TLIVE2004110 | 0.01622858 | 186237 | 3 |
| TLIVE2004320 | 0.013207687 | 186046 | 2 |
| TLIVE2004331 | 0.01151941 | 203974 | 1 |
| TLIVE2004601 | 0.01151941 | 137573 | 1 |
| TLIVE2005180 | 0.01151941 | 171074 | 1 |
| TLIVE2005390 | 0.01151941 | 162912 | 1 |
| TLIVE2005516 | 0.01151941 | 158841 | 1 |
| TLIVE2005731 | 0.01151941 | 149946 | 1 |
| TLIVE2005866 | 0.01151941 | 160852 | 1 |
| TLIVE2006236 | 0.01151941 | 151698 | 1 |
| TLIVE2006359 | 0.02303882 | 154405 | 2 |
| TLIVE2006529 | 0.01151941 | 182399 | 1 |
| TLIVE2006621 | 0.01151941 | 168164 | 1 |
| TLIVE2006733 | 0.01151941 | 185216 | 1 |
| TLIVE2007000 | 0.01151941 | 272444 | 1 |
| TLIVE2007016 | 0.01151941 | 14167 | 1 |
| TLIVE2007132 | 0.01151941 | 232895 | 1 |
| TLIVE2007192 | 0.01151941 | 215662 | 1 |
| TLIVE2007528 | 0.01151941 | 195857 | 1 |
| TLIVE2007607 | 0.02303882 | 100853 | 2 |
| TLIVE2007736 | 0.012708655 | 34334 | 2 |
| TLIVE2007816 | 0.01151941 | 156385 | 1 |
| TLIVE2007919 | 0.01151941 | 202926 | 1 |
| TLIVE2008083 | 0.01151941 | 264911 | 1 |
| TLIVE2008213 | 0.01151941 | 156360 | 1 |
| TLIVE2008221 | 0.02303882 | 147329 | 2 |
| TLIVE2008229 | 0.012614688 | 14540 | 2 |
| TLIVE2008712 | 0.01151941 | 144065 | 1 |
| TLIVE2008797 | 0.01151941 | 156389 | 1 |
| TLIVE2009087 | 0.01151941 | 170904 | 1 |
| TLIVE2009163 | 0.01151941 | 192482 | 1 |
| TLIVE2009489 | 0.01151941 | 141854 | 1 |
| TLIVE2009541 | 0.01151941 | 109193 | 1 |
| TLUNG2000179 | 0.035863308 | 111619 | 2 |
| TOVAR2000476 | 0.07383765 | 40429 | 2 |
| TOVAR2000575 | 0.037893506 | 17063 | 2 |
| TOVAR2000649 | 0.03649635 | 261104 | 1 |
| TOVAR2000904 | 0.038205811 | 166767 | 2 |
| TOVAR2001281 | 0.03649635 | 167847 | 1 |
| TOVAR2001556 | 0.03649635 | 170966 | 1 |
| TOVAR2001730 | 0.03649635 | 36671 | 1 |
| TOVAR2002247 | 0.03649635 | 68473 | 1 |
| TOVAR2002514 | 0.03649635 | 280288 | 1 |
| TOVAR2002549 | 0.03649635 | 160813 | 1 |
| TOVAR2002800 | 0.03649635 | 283966 | 1 |
| TRACH1000011 | 0.02276324 | 75422 | 7 |
| TRACH1000018 | 0.0081132 | 56050 | 2 |
| TRACH1000030 | 0.242877361 | 81581 | 82 |
| TRACH1000038 | 0.044487921 | 70270 | 15 |
| TRACH1000057 | 0.001876842 | 65038 | 1 |
| TRACH1000063 | 0.077907129 | 96395 | 11 |
| TRACH1000074 | 0.001876842 | 71895 | 1 |
| TRACH1000100 | 0.001876842 | 48143 | 1 |
| TRACH1000106 | 0.028223146 | 68326 | 5 |
| TRACH1000125 | 0.003273997 | 71896 | 2 |
| TRACH1000140 | 0.001876842 | 115480 | 1 |
| TRACH1000144 | 0.001876842 | 52961 | 1 |
| TRACH1000181 | 0.035367649 | 8421 | 10 |
| TRACH1000193 | 0.007713753 | 183820 | 5 |
| TRACH1000205 | 0.042576617 | 63085 | 18 |
| TRACH1000212 | 0.001876842 | 259947 | 1 |
| TRACH1000240 | 0.001876842 | 235796 | 1 |
| TRACH2000002 | 0.001876842 | 271670 | 1 |
| TRACH2000015 | 0.001876842 | 53856 | 1 |
| TRACH2000024 | 0.001876842 | 268675 | 1 |
| TRACH2000032 | 0.005150839 | 192694 | 3 |
| TRACH2000045 | 0.060933075 | 100123 | 30 |
| TRACH2000047 | 0.031284816 | 113471 | 14 |
| TRACH2000079 | 0.30930943 | 67177 | 52 |
| TRACH2000081 | 0.013163523 | 176354 | 2 |
| TRACH2000083 | 0.001876842 | 269548 | 1 |
| TRACH2000148 | 0.001876842 | 75993 | 1 |
| TRACH2000185 | 0.001876842 | 262542 | 1 |
| TRACH2000196 | 0.01270404 | 50423 | 2 |
| TRACH2000235 | 0.001876842 | 267088 | 1 |
| TRACH2000237 | 0.031164375 | 159027 | 9 |
| TRACH2000243 | 0.542417382 | 41893 | 2 |
| TRACH2000248 | 0.240163584 | 56800 | 50 |
| TRACH2000250 | 0.00911546 | 53151 | 3 |
| TRACH2000259 | 0.001876842 | 130854 | 1 |
| TRACH2000272 | 0.001876842 | 268966 | 1 |
| TRACH2000282 | 0.004791699 | 244101 | 2 |
| TRACH2000287 | 0.001876842 | 194550 | 1 |
| TRACH2000289 | 0.001876842 | 270078 | 1 |
| TRACH2000306 | 0.029624832 | 133844 | 5 |
| TRACH2000312 | 0.001876842 | 269781 | 1 |
| TRACH2000321 | 0.341032061 | 121365 | 68 |
| TRACH2000336 | 0.001876842 | 277535 | 1 |
| TRACH2000359 | 0.056186393 | 100850 | 11 |
| TRACH2000393 | 0.004924201 | 37670 | 3 |
| TRACH2000411 | 0.001876842 | 115636 | 1 |
| TRACH2000420 | 0.04996788 | 86495 | 20 |
| TRACH2000461 | 0.097726404 | 82743 | 49 |
| TRACH2000472 | 0.19622716 | 78653 | 33 |
| TRACH2000496 | 0.01126105 | 140260 | 6 |
| TRACH2000497 | 0.001876842 | 92546 | 1 |
| TRACH2000502 | 0.003552389 | 139492 | 2 |
| TRACH2000522 | 0.001876842 | 30404 | 1 |
| TRACH2000540 | 0.117612755 | 60835 | 15 |
| TRACH2000559 | 0.043024088 | 215095 | 3 |
| TRACH2000660 | 0.013211224 | 82315 | 8 |
| TRACH2000665 | 0.001876842 | 267158 | 1 |
| TRACH2000675 | 0.001876842 | 96417 | 1 |
| TRACH2000677 | 0.005943607 | 187195 | 4 |
| TRACH2000702 | 0.04267859 | 140574 | 9 |
| TRACH2000703 | 0.001876842 | 37984 | 1 |
| TRACH2000708 | 0.09145026 | 126067 | 29 |
| TRACH2000767 | 0.004671152 | 133191 | 3 |
| TRACH2000775 | 0.001876842 | 269017 | 1 |
| TRACH2000780 | 0.191708005 | 121982 | 61 |
| TRACH2000807 | 0.001876842 | 138063 | 1 |
| TRACH2000862 | 0.02894482 | 63177 | 7 |
| TRACH2000873 | 0.003565118 | 185084 | 2 |
| TRACH2000894 | 0.001876842 | 7567 | 1 |
| TRACH2000898 | 0.001876842 | 276954 | 1 |
| TRACH2000920 | 0.050541053 | 42080 | 19 |
| TRACH2000923 | 0.001876842 | 271648 | 1 |
| TRACH2000926 | 0.060054739 | 141469 | 10 |
| TRACH2000944 | 0.001876842 | 271064 | 1 |
| TRACH2000959 | 0.686273943 | 2455 | 22 |
| TRACH2000972 | 0.282727177 | 77265 | 30 |
| TRACH2000981 | 0.001876842 | 23469 | 1 |
| TRACH2000986 | 0.001876842 | 270545 | 1 |
| TRACH2001021 | 0.400160761 | 99504 | 39 |
| TRACH2001037 | 0.001876842 | 267477 | 1 |
| TRACH2001060 | 0.004997648 | 146531 | 2 |
| TRACH2001085 | 0.0081132 | 56506 | 2 |
| TRACH2001101 | 0.063036543 | 136261 | 17 |
| TRACH2001109 | 0.80060832 | 71463 | 390 |
| TRACH2001121 | 0.001876842 | 269250 | 1 |
| TRACH2001147 | 0.001876842 | 19995 | 1 |
| TRACH2001154 | 0.003753683 | 714 | 2 |
| TRACH2001188 | 0.001876842 | 268082 | 1 |
| TRACH2001192 | 0.004970521 | 83438 | 3 |
| TRACH2001249 | 0.018271584 | 165083 | 14 |
| TRACH2001275 | 0.001876842 | 270534 | 1 |
| TRACH2001289 | 0.49297219 | 80776 | 84 |
| TRACH2001314 | 0.091670121 | 115103 | 46 |
| TRACH2001335 | 0.001876842 | 271112 | 1 |
| TRACH2001339 | 0.005720926 | 79690 | 2 |
| TRACH2001341 | 0.026288736 | 48920 | 6 |
| TRACH2001347 | 0.001876842 | 45753 | 1 |
| TRACH2001395 | 0.001876842 | 271413 | 1 |
| TRACH2001400 | 0.001876842 | 271985 | 1 |
| TRACH2001403 | 0.001876842 | 23678 | 1 |
| TRACH2001416 | 0.001876842 | 269232 | 1 |
| TRACH2001428 | 0.132760152 | 14067 | 10 |
| TRACH2001432 | 0.150625572 | 139803 | 23 |
| TRACH2001443 | 0.018031859 | 147226 | 6 |
| TRACH2001463 | 0.054724518 | 125516 | 17 |
| TRACH2001474 | 0.001876842 | 109890 | 1 |
| TRACH2001478 | 0.288639963 | 106280 | 39 |
| TRACH2001523 | 0.098556086 | 155471 | 10 |
| TRACH2001525 | 0.001876842 | 270227 | 1 |
| TRACH2001532 | 0.001876842 | 156962 | 1 |
| TRACH2001549 | 0.039627059 | 111162 | 17 |
| TRACH2001592 | 0.001876842 | 135039 | 1 |
| TRACH2001596 | 0.008187255 | 8303 | 4 |
| TRACH2001607 | 0.166827778 | 91495 | 47 |
| TRACH2001612 | 0.041946256 | 28691 | 13 |
| TRACH2001621 | 0.001876842 | 62700 | 1 |
| TRACH2001646 | 0.007409202 | 239919 | 3 |
| TRACH2001651 | 0.01126105 | 100698 | 6 |
| TRACH2001684 | 0.009406581 | 237497 | 3 |
| TRACH2001810 | 0.215764907 | 81504 | 46 |
| TRACH2001898 | 0.164358759 | 148776 | 7 |
| TRACH2001933 | 0.001876842 | 245229 | 1 |
| TRACH2001968 | 0.007507367 | 144291 | 4 |
| TRACH2001996 | 0.005720926 | 248528 | 2 |
| TRACH2002043 | 0.158000009 | 98410 | 3 |
| TRACH2002054 | 0.003875243 | 190593 | 2 |
| TRACH2002092 | 0.059690957 | 84397 | 7 |
| TRACH2002100 | 0.009770005 | 100146 | 4 |
| TRACH2002138 | 0.007789453 | 110238 | 2 |
| TRACH2002333 | 0.011398249 | 179437 | 4 |
| TRACH2002537 | 0.18067368 | 85929 | 39 |
| TRACH2002664 | 0.110102803 | 97543 | 16 |
| TRACH2002784 | 0.003753683 | 253111 | 2 |
| TRACH2002803 | 0.080407312 | 97605 | 30 |
| TRACH2002840 | 0.001876842 | 215099 | 1 |
| TRACH2002847 | 0.036916451 | 228874 | 4 |
| TRACH2002853 | 0.001876842 | 280044 | 1 |
| TRACH2002862 | 0.001876842 | 273260 | 1 |
| TRACH2002954 | 0.001876842 | 267499 | 1 |
| TRACH2002960 | 0.001876842 | 117693 | 1 |
| TRACH2002988 | 0.003273997 | 130109 | 2 |
| TRACH2003070 | 0.003273997 | 89672 | 2 |
| TRACH2003272 | 0.045223526 | 103242 | 18 |
| TRACH2003323 | 0.094146793 | 67151 | 32 |
| TRACH2003347 | 0.105467684 | 91997 | 18 |
| TRACH2003484 | 0.003066086 | 114064 | 2 |
| TRACH2003516 | 0.001876842 | 121916 | 1 |
| TRACH2003605 | 0.003273997 | 111460 | 2 |
| TRACH2003736 | 0.001876842 | 132757 | 1 |
| TRACH2003759 | 0.001876842 | 167889 | 1 |
| TRACH2003941 | 0.001876842 | 55875 | 1 |
| TRACH2004039 | 0.00297212 | 84935 | 2 |
| TRACH2004054 | 0.001876842 | 36904 | 1 |
| TRACH2004087 | 0.001876842 | 158936 | 1 |
| TRACH2004109 | 0.004791699 | 174712 | 2 |
| TRACH2004170 | 0.001876842 | 266070 | 1 |
| TRACH2004183 | 0.001876842 | 37629 | 1 |
| TRACH2004251 | 0.001876842 | 2255 | 1 |
| TRACH2004292 | 0.001876842 | 167887 | 1 |
| TRACH2004336 | 0.00297212 | 120427 | 2 |
| TRACH2004399 | 0.005652486 | 80419 | 4 |
| TRACH2004428 | 0.001876842 | 37902 | 1 |
| TRACH2004458 | 0.00793121 | 90376 | 2 |
| TRACH2004499 | 0.057020452 | 185848 | 4 |
| TRACH2004521 | 0.001876842 | 51880 | 1 |
| TRACH2004570 | 0.001876842 | 156407 | 1 |
| TRACH2004845 | 0.001876842 | 134798 | 1 |
| TRACH2004887 | 0.005630525 | 145885 | 3 |
| TRACH2004950 | 0.001876842 | 175560 | 1 |
| TRACH2005004 | 0.003753683 | 125290 | 2 |
| TRACH2005017 | 0.001876842 | 112274 | 1 |
| TRACH2005066 | 0.001876842 | 273989 | 1 |
| TRACH2005159 | 0.001876842 | 169912 | 1 |
| TRACH2005210 | 0.055244213 | 147531 | 13 |
| TRACH2005314 | 0.001876842 | 108565 | 1 |
| TRACH2005444 | 0.001876842 | 170825 | 1 |
| TRACH2005541 | 0.017231309 | 71063 | 4 |
| TRACH2005666 | 0.001876842 | 190522 | 1 |
| TRACH2005698 | 0.003273997 | 43554 | 2 |
| TRACH2005710 | 0.012479047 | 63434 | 2 |
| TRACH2005720 | 0.001876842 | 73665 | 1 |
| TRACH2005769 | 0.001876842 | 189657 | 1 |
| TRACH2005796 | 0.003734956 | 168801 | 2 |
| TRACH2005800 | 0.139291562 | 132443 | 11 |
| TRACH2005811 | 0.158883397 | 92618 | 36 |
| TRACH2006015 | 0.001876842 | 190218 | 1 |
| TRACH2006049 | 0.003552389 | 96806 | 2 |
| TRACH2006097 | 0.003753683 | 162317 | 2 |
| TRACH2006194 | 0.001876842 | 170842 | 1 |
| TRACH2006327 | 0.001876842 | 137024 | 1 |
| TRACH2006331 | 0.001876842 | 68640 | 1 |
| TRACH2006378 | 0.018746459 | 174949 | 5 |
| TRACH2006387 | 0.001876842 | 146193 | 1 |
| TRACH2006528 | 0.001876842 | 95425 | 1 |
| TRACH2006670 | 0.001876842 | 17645 | 1 |
| TRACH2006762 | 0.007818965 | 84959 | 2 |
| TRACH2006866 | 0.168849307 | 151512 | 16 |
| TRACH2006870 | 0.001876842 | 98450 | 1 |
| TRACH2006918 | 0.001876842 | 273408 | 1 |
| TRACH2007059 | 0.004997648 | 170163 | 2 |
| TRACH2007324 | 0.001876842 | 201035 | 1 |
| TRACH2007358 | 0.01422557 | 4764 | 2 |
| TRACH2007399 | 0.355708299 | 94682 | 31 |
| TRACH2007483 | 0.001876842 | 123987 | 1 |
| TRACH2007542 | 0.001876842 | 207545 | 1 |
| TRACH2007577 | 0.001876842 | 130176 | 1 |
| TRACH2007674 | 0.001876842 | 129182 | 1 |
| TRACH2007676 | 0.001876842 | 130166 | 1 |
| TRACH2007688 | 0.003753683 | 180529 | 2 |
| TRACH2007733 | 0.001876842 | 278304 | 1 |
| TRACH2007754 | 0.11152409 | 87437 | 7 |
| TRACH2007816 | 0.016784271 | 131619 | 7 |
| TRACH2007834 | 0.332198807 | 88220 | 26 |
| TRACH2007894 | 0.003273997 | 101492 | 2 |
| TRACH2007969 | 0.066026047 | 157782 | 10 |
| TRACH2007988 | 0.001876842 | 194589 | 1 |
| TRACH2008081 | 0.13116108 | 21045 | 7 |
| TRACH2008112 | 0.001876842 | 180305 | 1 |
| TRACH2008113 | 0.009001861 | 161608 | 3 |
| TRACH2008127 | 0.001876842 | 174671 | 1 |
| TRACH2008130 | 0.003958354 | 152436 | 2 |
| TRACH2008278 | 0.001876842 | 113823 | 1 |
| TRACH2008300 | 0.098693552 | 123744 | 43 |
| TRACH2008472 | 0.106789876 | 155695 | 29 |
| TRACH2008478 | 0.009382103 | 119335 | 4 |
| TRACH2008540 | 0.021242825 | 163534 | 8 |
| TRACH2008583 | 0.001876842 | 157464 | 1 |
| TRACH2008597 | 0.003273997 | 10298 | 2 |
| TRACH2008716 | 0.033030077 | 158103 | 6 |
| TRACH2008820 | 0.001876842 | 18934 | 1 |
| TRACH2008891 | 0.007723592 | 18155 | 4 |
| TRACH2008921 | 0.003753683 | 170840 | 2 |
| TRACH2009006 | 0.047743908 | 100798 | 13 |
| TRACH2009060 | 0.001876842 | 233306 | 1 |
| TRACH2009107 | 0.013340788 | 61272 | 2 |
| TRACH2009123 | 0.001876842 | 157440 | 1 |
| TRACH2009268 | 0.001876842 | 223225 | 1 |
| TRACH2009291 | 0.008022368 | 167986 | 2 |
| TRACH2009310 | 0.009543789 | 85367 | 8 |
| TRACH2009340 | 0.016928499 | 48800 | 5 |
| TRACH2009348 | 0.001876842 | 140468 | 1 |
| TRACH2009358 | 0.001876842 | 222437 | 1 |
| TRACH2009661 | 0.00297212 | 2682 | 2 |
| TRACH2009730 | 0.005630525 | 227909 | 3 |
| TRACH2009743 | 0.001876842 | 148417 | 1 |
| TRACH2009754 | 0.001876842 | 145875 | 1 |
| TRACH2009851 | 0.011143174 | 150463 | 3 |
| TRACH2009862 | 0.001876842 | 283468 | 1 |
| TRACH2009934 | 0.003273997 | 98938 | 2 |
| TRACH2010159 | 0.001876842 | 110788 | 1 |
| TRACH2010218 | 0.001876842 | 264022 | 1 |
| TRACH2010251 | 0.001876842 | 148437 | 1 |
| TRACH2010272 | 0.001876842 | 136249 | 1 |
| TRACH2010317 | 0.003273997 | 16933 | 2 |
| TRACH2010419 | 0.001876842 | 163476 | 1 |
| TRACH2010451 | 0.001876842 | 148382 | 1 |
| TRACH2010505 | 0.001876842 | 154376 | 1 |
| TRACH2010587 | 0.001876842 | 145809 | 1 |
| TRACH2010600 | 0.004858801 | 92782 | 2 |
| TRACH2010634 | 0.009665945 | 27668 | 3 |
| TRACH2010639 | 0.001876842 | 156236 | 1 |
| TRACH2010677 | 0.001876842 | 132658 | 1 |
| TRACH2010698 | 0.001876842 | 51785 | 1 |
| TRACH2010771 | 0.004921723 | 56191 | 2 |
| TRACH2010824 | 0.009328365 | 113547 | 3 |
| TRACH2010883 | 0.001876842 | 199012 | 1 |
| TRACH2010965 | 0.001876842 | 158166 | 1 |
| TRACH2010974 | 0.003753683 | 125787 | 2 |
| TRACH2011057 | 0.001876842 | 262384 | 1 |
| TRACH2011093 | 0.001876842 | 137969 | 1 |
| TRACH2011113 | 0.035489678 | 114027 | 12 |
| TRACH2011290 | 0.003958354 | 103688 | 2 |
| TRACH2011293 | 0.037527466 | 125652 | 2 |
| TRACH2011302 | 0.01367486 | 71237 | 2 |
| TRACH2011500 | 0.012409369 | 96737 | 4 |
| TRACH2011574 | 0.001876842 | 175762 | 1 |
| TRACH2011894 | 0.018842558 | 86937 | 6 |
| TRACH2011956 | 0.001876842 | 263220 | 1 |
| TRACH2012000 | 0.001876842 | 153704 | 1 |
| TRACH2012138 | 0.001876842 | 179717 | 1 |
| TRACH2012242 | 0.001876842 | 183612 | 1 |
| TRACH2012298 | 0.118079062 | 117877 | 7 |
| TRACH2012370 | 0.001876842 | 190304 | 1 |
| TRACH2012376 | 0.062430239 | 97950 | 8 |
| TRACH2012387 | 0.00297212 | 126040 | 2 |
| TRACH2012497 | 0.025884721 | 176593 | 5 |
| TRACH2012536 | 0.004858801 | 166814 | 2 |
| TRACH2012562 | 0.001876842 | 171403 | 1 |
| TRACH2012647 | 0.025659598 | 158951 | 4 |
| TRACH2012742 | 0.001876842 | 9907 | 1 |
| TRACH2012811 | 0.021492274 | 150004 | 9 |
| TRACH2012823 | 0.001876842 | 253010 | 1 |
| TRACH2012825 | 0.667575146 | 98766 | 29 |
| TRACH2012918 | 0.001876842 | 174628 | 1 |
| TRACH2013081 | 0.014400323 | 154045 | 2 |
| TRACH2013123 | 0.013932344 | 124923 | 5 |
| TRACH2013265 | 0.001876842 | 277538 | 1 |
| TRACH2013369 | 0.003753683 | 117224 | 2 |
| TRACH2013450 | 0.001876842 | 157300 | 1 |
| TRACH2013495 | 0.014938493 | 158093 | 2 |
| TRACH2013552 | 0.016128461 | 98426 | 8 |
| TRACH2013585 | 0.007138691 | 18216 | 4 |
| TRACH2013671 | 0.001876842 | 210889 | 1 |
| TRACH2013726 | 0.001876842 | 167773 | 1 |
| TRACH2013902 | 0.001876842 | 138049 | 1 |
| TRACH2013928 | 0.001876842 | 140485 | 1 |
| TRACH2013982 | 0.001876842 | 14547 | 1 |
| TRACH2013991 | 0.013163523 | 130600 | 2 |
| TRACH2014000 | 0.355708299 | 94682 | 31 |
| TRACH2014018 | 0.001876842 | 147212 | 1 |
| TRACH2014045 | 0.001876842 | 128087 | 1 |
| TRACH2014046 | 0.001876842 | 39655 | 1 |
| TRACH2014077 | 0.049257269 | 34939 | 17 |
| TRACH2014082 | 0.001876842 | 154005 | 1 |
| TRACH2014124 | 0.162328919 | 131813 | 49 |
| TRACH2014268 | 0.001876842 | 256434 | 1 |
| TRACH2014284 | 0.001876842 | 154106 | 1 |
| TRACH2014337 | 0.001876842 | 157366 | 1 |
| TRACH2014356 | 0.001876842 | 49560 | 1 |
| TRACH2014371 | 0.001876842 | 40434 | 1 |
| TRACH2014442 | 0.074424533 | 4108 | 6 |
| TRACH2014483 | 0.001876842 | 123814 | 1 |
| TRACH2014495 | 0.001876842 | 144422 | 1 |
| TRACH2014544 | 0.001876842 | 179848 | 1 |
| TRACH2014573 | 0.001876842 | 162234 | 1 |
| TRACH2014725 | 0.001876842 | 61364 | 1 |
| TRACH2014760 | 0.001876842 | 136283 | 1 |
| TRACH2014815 | 0.001876842 | 14392 | 1 |
| TRACH2014839 | 0.001876842 | 158466 | 1 |
| TRACH2014938 | 0.001876842 | 159914 | 1 |
| TRACH2014950 | 0.001876842 | 154064 | 1 |
| TRACH2014972 | 0.001876842 | 144390 | 1 |
| TRACH2014974 | 0.001876842 | 146088 | 1 |
| TRACH2014997 | 0.047363123 | 23345 | 22 |
| TRACH2015180 | 0.001876842 | 48152 | 1 |
| TRACH2015243 | 0.001876842 | 123656 | 1 |
| TRACH2015316 | 0.001876842 | 160673 | 1 |
| TRACH2015381 | 0.001876842 | 160346 | 1 |
| TRACH2015486 | 0.001876842 | 68799 | 1 |
| TRACH2015654 | 0.008022368 | 78189 | 2 |
| TRACH2015823 | 0.076596152 | 100522 | 29 |
| TRACH2015824 | 0.072099206 | 125594 | 6 |
| TRACH2015939 | 0.029828108 | 21200 | 10 |
| TRACH2015987 | 0.001876842 | 36191 | 1 |
| TRACH2016080 | 0.001876842 | 268764 | 1 |
| TRACH2016131 | 0.005720926 | 134943 | 2 |
| TRACH2016286 | 0.001876842 | 125563 | 1 |
| TRACH2016317 | 0.021490469 | 21301 | 5 |
| TRACH2016347 | 0.001876842 | 127306 | 1 |
| TRACH2016380 | 0.001876842 | 142848 | 1 |
| TRACH2016384 | 0.001876842 | 99180 | 1 |
| TRACH2016410 | 0.001876842 | 175900 | 1 |
| TRACH2016481 | 0.003875243 | 13285 | 2 |
| TRACH2016498 | 0.001876842 | 87826 | 1 |
| TRACH2016533 | 0.011690111 | 32774 | 6 |
| TRACH2016551 | 0.001876842 | 272634 | 1 |
| TRACH2016554 | 0.001876842 | 144659 | 1 |
| TRACH2016651 | 0.001876842 | 152744 | 1 |
| TRACH2016690 | 0.001876842 | 92652 | 1 |
| TRACH2016709 | 0.001876842 | 272735 | 1 |
| TRACH2016722 | 0.001876842 | 127219 | 1 |
| TRACH2016835 | 0.009079175 | 45209 | 3 |
| TRACH2016980 | 0.001876842 | 130485 | 1 |
| TRACH2017086 | 0.001876842 | 71926 | 1 |
| TRACH2017368 | 0.038333276 | 157398 | 2 |
| TRACH2017498 | 0.001876842 | 110805 | 1 |
| TRACH2017609 | 0.005272398 | 103284 | 3 |
| TRACH2017949 | 0.005630525 | 168962 | 3 |
| TRACH2017965 | 0.001876842 | 91013 | 1 |
| TRACH2017980 | 0.001876842 | 166790 | 1 |
| TRACH2018034 | 0.003734956 | 200240 | 2 |
| TRACH2018084 | 0.001876842 | 114368 | 1 |
| TRACH2018262 | 0.001876842 | 52390 | 1 |
| TRACH2018273 | 0.001876842 | 131282 | 1 |
| TRACH2018278 | 0.001876842 | 157470 | 1 |
| TRACH2018317 | 0.057195045 | 56920 | 10 |
| TRACH2018345 | 0.001876842 | 182521 | 1 |
| TRACH2018380 | 0.001876842 | 182531 | 1 |
| TRACH2018446 | 0.040065241 | 66102 | 14 |
| TRACH2018449 | 0.011051281 | 51446 | 4 |
| TRACH2018512 | 0.001876842 | 269664 | 1 |
| TRACH2018646 | 0.001876842 | 77749 | 1 |
| TRACH2018663 | 0.001876842 | 65500 | 1 |
| TRACH2018718 | 0.001876842 | 183680 | 1 |
| TRACH2018835 | 0.001876842 | 169618 | 1 |
| TRACH2018882 | 0.003753683 | 41785 | 2 |
| TRACH2018914 | 0.001876842 | 171952 | 1 |
| TRACH2018950 | 0.022309481 | 152852 | 6 |
| TRACH2019024 | 0.001876842 | 49857 | 1 |
| TRACH2019046 | 0.001876842 | 67409 | 1 |
| TRACH2019080 | 0.005490733 | 84568 | 2 |
| TRACH2019151 | 0.001876842 | 9333 | 1 |
| TRACH2019154 | 0.001876842 | 24123 | 1 |
| TRACH2019176 | 0.001876842 | 144549 | 1 |
| TRACH2019248 | 0.001876842 | 276703 | 1 |
| TRACH2019309 | 0.001876842 | 264427 | 1 |
| TRACH2019425 | 0.014938493 | 183154 | 2 |
| TRACH2019473 | 0.001876842 | 249865 | 1 |
| TRACH2019602 | 0.001876842 | 153583 | 1 |
| TRACH2019661 | 0.001876842 | 114997 | 1 |
| TRACH2019672 | 0.008022368 | 122470 | 2 |
| TRACH2019673 | 0.001876842 | 272726 | 1 |
| TRACH2019844 | 0.001876842 | 253012 | 1 |
| TRACH2019879 | 0.001876842 | 140527 | 1 |
| TRACH2020048 | 0.001876842 | 235990 | 1 |
| TRACH2020336 | 0.001876842 | 243539 | 1 |
| TRACH2020525 | 0.001876842 | 284367 | 1 |
| TRACH2021398 | 0.001876842 | 260706 | 1 |
| TRACH2021622 | 0.001876842 | 277720 | 1 |
| TRACH2021635 | 0.001876842 | 249901 | 1 |
| TRACH2021964 | 0.001876842 | 267288 | 1 |
| TRACH2022042 | 0.001876842 | 282946 | 1 |
| TRACH2022113 | 0.001876842 | 261325 | 1 |
| TRACH2022253 | 0.001876842 | 277822 | 1 |
| TRACH2022425 | 0.001876842 | 274968 | 1 |
| TRACH2022553 | 0.001876842 | 71923 | 1 |
| TRACH2022649 | 0.001876842 | 206747 | 1 |
| TRACH2022839 | 0.003753683 | 220597 | 2 |
| TRACH2023246 | 0.001876842 | 241348 | 1 |
| TRACH2023299 | 0.026250164 | 207353 | 5 |
| TRACH2023306 | 0.001876842 | 255352 | 1 |
| TRACH2023416 | 0.001876842 | 268069 | 1 |
| TRACH2023479 | 0.001876842 | 140549 | 1 |
| TRACH2023684 | 0.001876842 | 259406 | 1 |
| TRACH2023860 | 0.001876842 | 256569 | 1 |
| TRACH2023876 | 0.001876842 | 253096 | 1 |
| TRACH2024139 | 0.001876842 | 268771 | 1 |
| TRACH2024187 | 0.001876842 | 274191 | 1 |
| TRACH2024271 | 0.003753683 | 94919 | 2 |
| TRACH2024293 | 0.01038032 | 87630 | 6 |
| TRACH2024408 | 0.01126105 | 148274 | 6 |
| TRACH2024512 | 0.001876842 | 199706 | 1 |
| TRACH2024559 | 0.001876842 | 280923 | 1 |
| TRACH2024647 | 0.001876842 | 282682 | 1 |
| TRACH2024651 | 0.001876842 | 235220 | 1 |
| TRACH2024730 | 0.001876842 | 269390 | 1 |
| TRACH2024735 | 0.001876842 | 217471 | 1 |
| TRACH2024749 | 0.001876842 | 273443 | 1 |
| TRACH2024821 | 0.001876842 | 265284 | 1 |
| TRACH2025057 | 0.001876842 | 276642 | 1 |
| TRACH2025084 | 0.001876842 | 257781 | 1 |
| TRACH2025155 | 0.001876842 | 245148 | 1 |
| TRACH2025224 | 0.001876842 | 228042 | 1 |
| TRACH2025344 | 0.01701395 | 109067 | 4 |
| TRACH2025379 | 0.001876842 | 275880 | 1 |
| TRACH2025507 | 0.001876842 | 245203 | 1 |
| TRACH2025535 | 0.288606486 | 4524 | 61 |
| TRACH2025569 | 0.001876842 | 253100 | 1 |
| TRACH2025705 | 0.001876842 | 265082 | 1 |
| TRACH2025749 | 0.001876842 | 249624 | 1 |
| TRACH2025753 | 0.001876842 | 283933 | 1 |
| TRACH2025810 | 0.001876842 | 256163 | 1 |
| TRACH2025853 | 0.001876842 | 261787 | 1 |
| TRACH2025911 | 0.003565118 | 283057 | 2 |
| TRACH2025932 | 0.001876842 | 224732 | 1 |
| TRACH3000014 | 0.009216827 | 186805 | 5 |
| TRACH3000017 | 0.003753683 | 245332 | 2 |
| TRACH3000043 | 0.001876842 | 217461 | 1 |
| TRACH3000134 | 0.009266219 | 84174 | 4 |
| TRACH3000180 | 0.001876842 | 228038 | 1 |
| TRACH3000342 | 0.001876842 | 201776 | 1 |
| TRACH3000420 | 0.001876842 | 257836 | 1 |
| TRACH3000515 | 0.001876842 | 280537 | 1 |
| TRACH3000530 | 0.003753683 | 127523 | 2 |
| TRACH3000548 | 0.001876842 | 211071 | 1 |
| TRACH3000558 | 0.001876842 | 202491 | 1 |
| TRACH3000586 | 0.001876842 | 282036 | 1 |
| TRACH3000692 | 0.035750286 | 8476 | 9 |
| TRACH3000780 | 0.003586302 | 271027 | 2 |
| TRACH3000892 | 0.001876842 | 284048 | 1 |
| TRACH3000926 | 0.001876842 | 280089 | 1 |
| TRACH3001119 | 0.007507367 | 32761 | 4 |
| TRACH3001427 | 0.180469591 | 11446 | 57 |
| TRACH3001443 | 0.005630525 | 189457 | 3 |
| TRACH3001550 | 0.003586302 | 236721 | 2 |
| TRACH3001759 | 0.001876842 | 258472 | 1 |
| TRACH3002064 | 0.001876842 | 148286 | 1 |
| TRACH3002107 | 0.001876842 | 234535 | 1 |
| TRACH3002168 | 0.001876842 | 215473 | 1 |
| TRACH3002188 | 0.001876842 | 223484 | 1 |
| TRACH3002192 | 0.268676242 | 60521 | 87 |
| TRACH3002245 | 0.001876842 | 251566 | 1 |
| TRACH3002293 | 0.001876842 | 269341 | 1 |
| TRACH3002533 | 0.001876842 | 243083 | 1 |
| TRACH3002561 | 0.001876842 | 4921 | 1 |
| TRACH3002567 | 0.007507367 | 140929 | 4 |
| TRACH3002605 | 0.003586302 | 266291 | 2 |
| TRACH3002650 | 0.001876842 | 277151 | 1 |
| TRACH3002752 | 0.032805351 | 119633 | 10 |
| TRACH3002866 | 0.001876842 | 215487 | 1 |
| TRACH3002871 | 0.001876842 | 148877 | 1 |
| TRACH3002876 | 0.001876842 | 265394 | 1 |
| TRACH3002890 | 0.001876842 | 269774 | 1 |
| TRACH3003009 | 0.001876842 | 280155 | 1 |
| TRACH3003037 | 0.005227936 | 109915 | 3 |
| TRACH3003357 | 0.001876842 | 276382 | 1 |
| TRACH3003379 | 0.006847363 | 158448 | 4 |
| TRACH3003547 | 0.039621581 | 86472 | 12 |
| TRACH3003586 | 0.001876842 | 251535 | 1 |
| TRACH3003683 | 0.001876842 | 43507 | 1 |
| TRACH3003780 | 0.001876842 | 260725 | 1 |
| TRACH3003805 | 0.001876842 | 264578 | 1 |
| TRACH3003832 | 0.013799318 | 132934 | 3 |
| TRACH3003872 | 0.061047945 | 75455 | 28 |
| TRACH3004068 | 0.117561666 | 74911 | 12 |
| TRACH3004113 | 0.001876842 | 243746 | 1 |
| TRACH3004288 | 0.001876842 | 249698 | 1 |
| TRACH3004303 | 0.003753683 | 155515 | 2 |
| TRACH3004412 | 0.001876842 | 268232 | 1 |
| TRACH3004424 | 0.00985254 | 18797 | 5 |
| TRACH3004456 | 0.001876842 | 229453 | 1 |
| TRACH3004537 | 0.001876842 | 127939 | 1 |
| TRACH3004596 | 0.001876842 | 253107 | 1 |
| TRACH3004651 | 0.001876842 | 49800 | 1 |
| TRACH3004652 | 0.001876842 | 218594 | 1 |
| TRACH3004671 | 0.001876842 | 30389 | 1 |
| TRACH3004721 | 0.057637274 | 70607 | 23 |
| TRACH3004747 | 0.003753683 | 195023 | 2 |
| TRACH3004760 | 0.001876842 | 260142 | 1 |
| TRACH3004783 | 0.001876842 | 233109 | 1 |
| TRACH3004786 | 0.001876842 | 255574 | 1 |
| TRACH3004840 | 0.001876842 | 4942 | 1 |
| TRACH3004931 | 0.001876842 | 235908 | 1 |
| TRACH3004934 | 0.003753683 | 239688 | 2 |
| TRACH3005072 | 0.001876842 | 243884 | 1 |
| TRACH3005085 | 0.001876842 | 248984 | 1 |
| TRACH3005102 | 0.001876842 | 273727 | 1 |
| TRACH3005173 | 0.001876842 | 219772 | 1 |
| TRACH3005191 | 0.001876842 | 271368 | 1 |
| TRACH3005211 | 0.009091915 | 225679 | 4 |
| TRACH3005274 | 0.001876842 | 266317 | 1 |
| TRACH3005294 | 0.04511614 | 131092 | 6 |
| TRACH3005479 | 0.00297212 | 146853 | 2 |
| TRACH3005549 | 0.001876842 | 245278 | 1 |
| TRACH3005699 | 0.017458248 | 155387 | 9 |
| TRACH3005702 | 0.003753683 | 244030 | 2 |
| TRACH3005808 | 0.001876842 | 244043 | 1 |
| TRACH3005852 | 0.001876842 | 210434 | 1 |
| TRACH3005896 | 0.007507367 | 135731 | 4 |
| TRACH3006038 | 0.003066086 | 243120 | 2 |
| TRACH3006149 | 0.001876842 | 243123 | 1 |
| TRACH3006228 | 0.001876842 | 236664 | 1 |
| TRACH3006264 | 0.001876842 | 277392 | 1 |
| TRACH3006336 | 0.003753683 | 208304 | 2 |
| TRACH3006379 | 0.001876842 | 275677 | 1 |
| TRACH3006392 | 0.007507367 | 135570 | 4 |
| TRACH3006395 | 0.001876842 | 277499 | 1 |
| TRACH3006397 | 0.001876842 | 271283 | 1 |
| TRACH3006412 | 0.003066086 | 246480 | 2 |
| TRACH3006470 | 0.001876842 | 194345 | 1 |
| TRACH3006589 | 0.001876842 | 190658 | 1 |
| TRACH3006626 | 0.007507367 | 122228 | 4 |
| TRACH3006699 | 0.001876842 | 265793 | 1 |
| TRACH3006717 | 0.045662706 | 74289 | 24 |
| TRACH3006800 | 0.005752085 | 212445 | 3 |
| TRACH3006889 | 0.001876842 | 233075 | 1 |
| TRACH3006985 | 0.001876842 | 280680 | 1 |
| TRACH3007097 | 0.001876842 | 280993 | 1 |
| TRACH3007268 | 0.001876842 | 273872 | 1 |
| TRACH3007274 | 0.001876842 | 270677 | 1 |
| TRACH3007277 | 0.001876842 | 281544 | 1 |
| TRACH3007391 | 0.001876842 | 281081 | 1 |
| TRACH3007479 | 0.155801381 | 87430 | 34 |
| TRACH3007541 | 0.003273997 | 141938 | 2 |
| TRACH3007595 | 0.001876842 | 280444 | 1 |
| TRACH3007625 | 0.142539258 | 115165 | 45 |
| TRACH3007627 | 0.001876842 | 134003 | 1 |
| TRACH3007689 | 0.007104777 | 151321 | 4 |
| TRACH3007766 | 0.001876842 | 268534 | 1 |
| TRACH3007866 | 0.001876842 | 76441 | 1 |
| TRACH3007995 | 0.003066086 | 251471 | 2 |
| TRACH3008042 | 0.001876842 | 271324 | 1 |
| TRACH3008061 | 0.007507367 | 150881 | 4 |
| TRACH3008093 | 0.06840904 | 144960 | 18 |
| TRACH3008201 | 0.003552389 | 52462 | 2 |
| TRACH3008508 | 0.008746003 | 118708 | 4 |
| TRACH3008535 | 0.005915125 | 113178 | 2 |
| TRACH3008629 | 0.03675527 | 98336 | 20 |
| TRACH3008632 | 0.019828308 | 140383 | 11 |
| TRACH3008688 | 0.001876842 | 245043 | 1 |
| TRACH3008697 | 0.001876842 | 243103 | 1 |
| TRACH3008713 | 0.005915125 | 189254 | 2 |
| TRACH3008887 | 0.001876842 | 247226 | 1 |
| TRACH3008902 | 0.001876842 | 219889 | 1 |
| TRACH3008990 | 0.003753683 | 174885 | 2 |
| TRACH3009001 | 0.00715142 | 225239 | 4 |
| TRACH3009008 | 0.034201874 | 78544 | 12 |
| TRACH3009059 | 0.003753683 | 224664 | 2 |
| TRACH3009061 | 0.003753683 | 131062 | 2 |
| TRACH3009148 | 0.018680989 | 13251 | 8 |
| TRACH3009455 | 0.003552389 | 15498 | 2 |
| TRACH3009701 | 0.001876842 | 258527 | 1 |
| TRACH3009715 | 0.001876842 | 257303 | 1 |
| TRACH3009894 | 0.001876842 | 261097 | 1 |
| TRACH3009956 | 0.004255331 | 154228 | 3 |
| TRACH3010079 | 0.006937396 | 37145 | 4 |
| TRACH3010167 | 0.001876842 | 194380 | 1 |
| TRACH3010331 | 0.001876842 | 49360 | 1 |
| TRACH3010342 | 0.001876842 | 103396 | 1 |
| TRACH3010382 | 0.005630525 | 125847 | 3 |
| TRACH3010757 | 0.001876842 | 224688 | 1 |
| TRACH3010901 | 0.001876842 | 189290 | 1 |
| TRACH3011004 | 0.001876842 | 189294 | 1 |
| TRACH3011082 | 0.001876842 | 195842 | 1 |
| TRACH3011184 | 0.001876842 | 212713 | 1 |
| TRACH3011282 | 0.001876842 | 74226 | 1 |
| TRACH3011313 | 0.001876842 | 53299 | 1 |
| TRACH3011454 | 0.001876842 | 190734 | 1 |
| TRACH3011485 | 0.001876842 | 219644 | 1 |
| TRACH3011503 | 0.001876842 | 131922 | 1 |
| TRACH3011538 | 0.001876842 | 235849 | 1 |
| TRACH3011542 | 0.007507367 | 204878 | 4 |
| TRACH3012106 | 0.001876842 | 18395 | 1 |
| TRACH3012161 | 0.001876842 | 276824 | 1 |
| TRACH3012232 | 0.001876842 | 251815 | 1 |
| TRACH3012261 | 0.003753683 | 116268 | 2 |
| TRACH3012445 | 0.001876842 | 281788 | 1 |
| TRACH3012460 | 0.001876842 | 112841 | 1 |
| TRACH3012659 | 0.001876842 | 272112 | 1 |
| TRACH3012718 | 0.001876842 | 239766 | 1 |
| TRACH3012864 | 0.001876842 | 163082 | 1 |
| TRACH3012891 | 0.001876842 | 179468 | 1 |
| TRACH3012942 | 0.001876842 | 196036 | 1 |
| TRACH3013043 | 0.004067398 | 75168 | 3 |
| TRACH3013072 | 0.003273997 | 128801 | 2 |
| TRACH3013426 | 0.001876842 | 179932 | 1 |
| TRACH3013454 | 0.001876842 | 127425 | 1 |
| TRACH3013558 | 0.003066086 | 189271 | 2 |
| TRACH3013684 | 0.001876842 | 241665 | 1 |
| TRACH3013693 | 0.001876842 | 251797 | 1 |
| TRACH3013700 | 0.001876842 | 78474 | 1 |
| TRACH3013766 | 0.001876842 | 241614 | 1 |
| TRACH3013900 | 0.001876842 | 135300 | 1 |
| TRACH3013926 | 0.001876842 | 168862 | 1 |
| TRACH3014063 | 0.001876842 | 166918 | 1 |
| TRACH3014183 | 0.001876842 | 180685 | 1 |
| TRACH3014215 | 0.001876842 | 190689 | 1 |
| TRACH3014316 | 0.003958354 | 225971 | 2 |
| TRACH3014415 | 0.001876842 | 233155 | 1 |
| TRACH3014580 | 0.001876842 | 229503 | 1 |
| TRACH3014641 | 0.001876842 | 153699 | 1 |
| TRACH3014885 | 0.001876842 | 218065 | 1 |
| TRACH3015136 | 0.001876842 | 236550 | 1 |
| TRACH3015336 | 0.001876842 | 19316 | 1 |
| TRACH3015346 | 0.001876842 | 95166 | 1 |
| TRACH3015354 | 0.001876842 | 87655 | 1 |
| TRACH3015467 | 0.001876842 | 189327 | 1 |
| TRACH3015626 | 0.001876842 | 34608 | 1 |
| TRACH3015951 | 0.001876842 | 243242 | 1 |
| TRACH3016051 | 0.001876842 | 236508 | 1 |
| TRACH3016120 | 0.001876842 | 234557 | 1 |
| TRACH3016264 | 0.003753683 | 222836 | 2 |
| TRACH3016277 | 0.001876842 | 223941 | 1 |
| TRACH3016368 | 0.001876842 | 221489 | 1 |
| TRACH3016455 | 0.001876842 | 213599 | 1 |
| TRACH3016614 | 0.001876842 | 138036 | 1 |
| TRACH3016805 | 0.037214621 | 91739 | 14 |
| TRACH3016885 | 0.042118119 | 123560 | 15 |
| TRACH3016992 | 0.007039518 | 150088 | 5 |
| TRACH3017171 | 0.001876842 | 223891 | 1 |
| TRACH3017409 | 0.001876842 | 165075 | 1 |
| TRACH3017934 | 0.001876842 | 189377 | 1 |
| TRACH3018108 | 0.001876842 | 262013 | 1 |
| TRACH3018191 | 0.001876842 | 138047 | 1 |
| TRACH3018240 | 0.001876842 | 284234 | 1 |
| TRACH3018261 | 0.001876842 | 148648 | 1 |
| TRACH3018519 | 0.001876842 | 56557 | 1 |
| TRACH3018524 | 0.001876842 | 145402 | 1 |
| TRACH3018606 | 0.001876842 | 233123 | 1 |
| TRACH3018783 | 0.001876842 | 166651 | 1 |
| TRACH3018907 | 0.001876842 | 114372 | 1 |
| TRACH3018933 | 0.001876842 | 160812 | 1 |
| TRACH3018943 | 0.001876842 | 166620 | 1 |
| TRACH3019018 | 0.001876842 | 154882 | 1 |
| TRACH3019058 | 0.001876842 | 169335 | 1 |
| TRACH3019142 | 0.001876842 | 169257 | 1 |
| TRACH3019290 | 0.006057552 | 18706 | 4 |
| TRACH3019347 | 0.001876842 | 174757 | 1 |
| TRACH3019370 | 0.001876842 | 142417 | 1 |
| TRACH3019447 | 0.001876842 | 243757 | 1 |
| TRACH3019598 | 0.001876842 | 235379 | 1 |
| TRACH3019621 | 0.001876842 | 160747 | 1 |
| TRACH3019662 | 0.001876842 | 175564 | 1 |
| TRACH3019807 | 0.001876842 | 179283 | 1 |
| TRACH3019924 | 0.001876842 | 117292 | 1 |
| TRACH3019977 | 0.003753683 | 229781 | 2 |
| TRACH3020137 | 0.001876842 | 169268 | 1 |
| TRACH3020410 | 0.001876842 | 246856 | 1 |
| TRACH3020563 | 0.001876842 | 128802 | 1 |
| TRACH3020605 | 0.001876842 | 262103 | 1 |
| TRACH3020750 | 0.001876842 | 166752 | 1 |
| TRACH3020769 | 0.001876842 | 84736 | 1 |
| TRACH3020928 | 0.001876842 | 264847 | 1 |
| TRACH3020930 | 0.001876842 | 277108 | 1 |
| TRACH3021023 | 0.001876842 | 71917 | 1 |
| TRACH3021066 | 0.001876842 | 189461 | 1 |
| TRACH3021212 | 0.005630525 | 194957 | 3 |
| TRACH3021335 | 0.001876842 | 237814 | 1 |
| TRACH3021373 | 0.001876842 | 20519 | 1 |
| TRACH3021544 | 0.003958354 | 155645 | 2 |
| TRACH3021778 | 0.003753683 | 175469 | 2 |
| TRACH3021834 | 0.001876842 | 85372 | 1 |
| TRACH3021883 | 0.001876842 | 150048 | 1 |
| TRACH3022109 | 0.003958354 | 110875 | 2 |
| TRACH3022170 | 0.001876842 | 121920 | 1 |
| TRACH3022198 | 0.020159652 | 62639 | 7 |
| TRACH3022296 | 0.001876842 | 141219 | 1 |
| TRACH3022482 | 0.001876842 | 237780 | 1 |
| TRACH3022732 | 0.001876842 | 119155 | 1 |
| TRACH3022758 | 0.001876842 | 138622 | 1 |
| TRACH3022875 | 0.001876842 | 207178 | 1 |
| TRACH3022910 | 0.003753683 | 140795 | 2 |
| TRACH3022960 | 0.001876842 | 138995 | 1 |
| TRACH3023063 | 0.001876842 | 119639 | 1 |
| TRACH3023203 | 0.001876842 | 129526 | 1 |
| TRACH3023242 | 0.001876842 | 117212 | 1 |
| TRACH3023373 | 0.001876842 | 124177 | 1 |
| TRACH3023516 | 0.001876842 | 45835 | 1 |
| TRACH3023752 | 0.001876842 | 138873 | 1 |
| TRACH3023945 | 0.003753683 | 129954 | 2 |
| TRACH3023960 | 0.001876842 | 250715 | 1 |
| TRACH3024020 | 0.001876842 | 154790 | 1 |
| TRACH3024081 | 0.001876842 | 123327 | 1 |
| TRACH3024342 | 0.003552389 | 116975 | 2 |
| TRACH3024423 | 0.001876842 | 115246 | 1 |
| TRACH3024428 | 0.001876842 | 119587 | 1 |
| TRACH3024512 | 0.001876842 | 132721 | 1 |
| TRACH3024671 | 0.003552389 | 153234 | 2 |
| TRACH3024823 | 0.005630525 | 163611 | 3 |
| TRACH3025302 | 0.001876842 | 55453 | 1 |
| TRACH3025316 | 0.005463143 | 51859 | 3 |
| TRACH3025346 | 0.001876842 | 198391 | 1 |
| TRACH3025520 | 0.001876842 | 260860 | 1 |
| TRACH3025828 | 0.246532535 | 78679 | 51 |
| TRACH3026148 | 0.001876842 | 139092 | 1 |
| TRACH3026283 | 0.001876842 | 86474 | 1 |
| TRACH3026299 | 0.001876842 | 119570 | 1 |
| TRACH3026303 | 0.029895056 | 90578 | 14 |
| TRACH3026542 | 0.001876842 | 242058 | 1 |
| TRACH3026650 | 0.001876842 | 249108 | 1 |
| TRACH3026676 | 0.014405426 | 89205 | 7 |
| TRACH3026744 | 0.001876842 | 250510 | 1 |
| TRACH3026949 | 0.001876842 | 111185 | 1 |
| TRACH3027229 | 0.030925498 | 127139 | 3 |
| TRACH3027527 | 0.001876842 | 148219 | 1 |
| TRACH3027681 | 0.001876842 | 174333 | 1 |
| TRACH3027701 | 0.001876842 | 123857 | 1 |
| TRACH3027885 | 0.001876842 | 174328 | 1 |
| TRACH3028031 | 0.001876842 | 161247 | 1 |
| TRACH3028164 | 0.001876842 | 138712 | 1 |
| TRACH3028180 | 0.001876842 | 148503 | 1 |
| TRACH3028195 | 0.001876842 | 130829 | 1 |
| TRACH3028441 | 0.001876842 | 280152 | 1 |
| TRACH3028597 | 0.001876842 | 8558 | 1 |
| TRACH3028678 | 0.005630525 | 155496 | 3 |
| TRACH3028837 | 0.001876842 | 53784 | 1 |
| TRACH3028847 | 0.001876842 | 161404 | 1 |
| TRACH3028855 | 0.007510742 | 86407 | 4 |
| TRACH3029139 | 0.001876842 | 196882 | 1 |
| TRACH3029329 | 0.001876842 | 262849 | 1 |
| TRACH3029451 | 0.001876842 | 283632 | 1 |
| TRACH3029462 | 0.001876842 | 47701 | 1 |
| TRACH3029520 | 0.001876842 | 207640 | 1 |
| TRACH3029592 | 0.001876842 | 189952 | 1 |
| TRACH3029629 | 0.001876842 | 161388 | 1 |
| TRACH3029665 | 0.001876842 | 167291 | 1 |
| TRACH3029670 | 0.001876842 | 180015 | 1 |
| TRACH3029800 | 0.001876842 | 163806 | 1 |
| TRACH3030111 | 0.001876842 | 83336 | 1 |
| TRACH3030130 | 0.001876842 | 220045 | 1 |
| TRACH3030176 | 0.001876842 | 180062 | 1 |
| TRACH3030252 | 0.001876842 | 140398 | 1 |
| TRACH3030725 | 0.001876842 | 244223 | 1 |
| TRACH3030855 | 0.001876842 | 174411 | 1 |
| TRACH3030883 | 0.001876842 | 260140 | 1 |
| TRACH3031316 | 0.001876842 | 130732 | 1 |
| TRACH3031400 | 0.001876842 | 136996 | 1 |
| TRACH3031463 | 0.001876842 | 212337 | 1 |
| TRACH3031479 | 0.001876842 | 264226 | 1 |
| TRACH3031518 | 0.001876842 | 272754 | 1 |
| TRACH3031660 | 0.001876842 | 129312 | 1 |
| TRACH3031678 | 0.001876842 | 87283 | 1 |
| TRACH3031936 | 0.001876842 | 279863 | 1 |
| TRACH3032044 | 0.001876842 | 172259 | 1 |
| TRACH3032065 | 0.003753683 | 142549 | 2 |
| TRACH3032150 | 0.001876842 | 128390 | 1 |
| TRACH3032372 | 0.001876842 | 187096 | 1 |
| TRACH3032480 | 0.001876842 | 260297 | 1 |
| TRACH3032570 | 0.003552389 | 174903 | 2 |
| TRACH3032755 | 0.001876842 | 279799 | 1 |
| TRACH3032827 | 0.001876842 | 194662 | 1 |
| TRACH3032873 | 0.001876842 | 152946 | 1 |
| TRACH3033535 | 0.001876842 | 281825 | 1 |
| TRACH3033680 | 0.001876842 | 123854 | 1 |
| TRACH3033868 | 0.001876842 | 127273 | 1 |
| TRACH3034145 | 0.001876842 | 200636 | 1 |
| TRACH3034414 | 0.001876842 | 269399 | 1 |
| TRACH3034488 | 0.001876842 | 167054 | 1 |
| TRACH3034680 | 0.001876842 | 71913 | 1 |
| TRACH3034731 | 0.003753683 | 141698 | 2 |
| TRACH3034745 | 0.001876842 | 155463 | 1 |
| TRACH3034762 | 0.001876842 | 164871 | 1 |
| TRACH3034903 | 0.001876842 | 185160 | 1 |
| TRACH3035187 | 0.001876842 | 151231 | 1 |
| TRACH3035199 | 0.001876842 | 115616 | 1 |
| TRACH3035235 | 0.001876842 | 163148 | 1 |
| TRACH3035451 | 0.001876842 | 159501 | 1 |
| TRACH3035482 | 0.001876842 | 192639 | 1 |
| TRACH3035526 | 0.001876842 | 189111 | 1 |
| TRACH3036004 | 0.001876842 | 127213 | 1 |
| TRACH3036103 | 0.001876842 | 146858 | 1 |
| TRACH3036193 | 0.001876842 | 184434 | 1 |
| TRACH3036207 | 0.003753683 | 90727 | 2 |
| TRACH3036278 | 0.001876842 | 128695 | 1 |
| TRACH3036309 | 0.001876842 | 174244 | 1 |
| TRACH3036456 | 0.001876842 | 142032 | 1 |
| TRACH3036609 | 0.001876842 | 159717 | 1 |
| TRACH3036638 | 0.001876842 | 165056 | 1 |
| TRACH3036683 | 0.003753683 | 166997 | 2 |
| TRACH3036750 | 0.001876842 | 146956 | 1 |
| TRACH3036792 | 0.001876842 | 154992 | 1 |
| TRACH3036843 | 0.001876842 | 190067 | 1 |
| TRACH3036897 | 0.001876842 | 163262 | 1 |
| TRACH3036932 | 0.011430014 | 125601 | 6 |
| TRACH3036942 | 0.686716771 | 136569 | 9 |
| TRACH3036997 | 0.001876842 | 133472 | 1 |
| TRACH3037063 | 0.001876842 | 137132 | 1 |
| TRACH3037067 | 0.104236998 | 128155 | 12 |
| TRACH3037267 | 0.001876842 | 142640 | 1 |
| TRACH3037288 | 0.001876842 | 49309 | 1 |
| TRACH3037505 | 0.001876842 | 135467 | 1 |
| TRACH3037573 | 0.001876842 | 133504 | 1 |
| TRACH3037696 | 0.001876842 | 137127 | 1 |
| TRACH3037897 | 0.001876842 | 136236 | 1 |
| TRACH3038086 | 0.001876842 | 114612 | 1 |
| TRACH3038399 | 0.013474237 | 133424 | 7 |
| TSTOM1000135 | 0.039700396 | 208608 | 3 |
| TSTOM1000186 | 0.035984167 | 221436 | 1 |
| TSTOM2000139 | 0.040061404 | 130365 | 3 |
| TSTOM2000235 | 0.037693627 | 136917 | 2 |
| TSTOM2000315 | 0.073523309 | 99167 | 12 |
| TSTOM2000442 | 0.035984167 | 273060 | 1 |
| TSTOM2000553 | 0.099395741 | 100797 | 21 |
| TSTOM2000569 | 0.035984167 | 70584 | 1 |
| TSTOM2000588 | 0.035984167 | 150490 | 1 |
| TSTOM2001195 | 0.035984167 | 231651 | 1 |
| TSTOM2001274 | 0.035984167 | 224127 | 1 |
| TSTOM2001571 | 0.035984167 | 196977 | 1 |
| TSTOM2001996 | 0.035984167 | 138448 | 1 |
| TSTOM2002265 | 0.035984167 | 207420 | 1 |
| TSTOM2002505 | 0.035984167 | 224138 | 1 |
| TSTOM2002561 | 0.035984167 | 199898 | 1 |
| TSTOM2002611 | 0.035984167 | 155921 | 1 |
| TSTOM2002672 | 0.035984167 | 278459 | 1 |
| TSTOM2002682 | 0.035984167 | 270648 | 1 |
| TUTER1000014 | 0.037341299 | 221698 | 1 |
| TUTER1000122 | 0.051336742 | 103372 | 9 |
| TUTER1000137 | 0.039016847 | 222374 | 2 |
| TUTER2000057 | 0.040386181 | 268032 | 2 |
| TUTER2000283 | 0.037341299 | 43096 | 1 |
| TUTER2000425 | 0.037341299 | 38750 | 1 |
| TUTER2000904 | 0.060017599 | 135875 | 14 |
| TUTER2000916 | 0.037341299 | 203159 | 1 |
| TUTER2001286 | 0.037341299 | 176751 | 1 |
| TUTER2001341 | 0.037341299 | 184704 | 1 |
| TUTER2001387 | 0.037341299 | 200612 | 1 |
| TUTER2001433 | 0.037341299 | 266343 | 1 |
| TUTER2001461 | 0.037341299 | 271545 | 1 |
| TUTER2002028 | 0.037341299 | 238559 | 1 |
| TUTER2002074 | 0.037341299 | 253334 | 1 |
| TUTER2002158 | 0.037341299 | 174532 | 1 |
| TUTER2002228 | 0.037341299 | 165834 | 1 |
| TUTER2002323 | 0.05144711 | 145377 | 4 |
| TUTER2002356 | 0.037341299 | 165069 | 1 |
| TUTER2002729 | 0.037341299 | 200723 | 1 |
| UMVEN1000122 | 0.171447898 | 70225 | 3 |
| UMVEN1000138 | 0.313578061 | 44329 | 66 |
| UMVEN1000143 | 0.2040239 | 29627 | 4 |
| UMVEN1000156 | 0.162154818 | 2310 | 2 |
| UMVEN1000186 | 0.634177857 | 17741 | 25 |
| UMVEN2000046 | 0.157977883 | 221603 | 1 |
| UMVEN2000069 | 0.157977883 | 143141 | 1 |
| UMVEN2000121 | 0.161712839 | 143359 | 3 |
| UMVEN2000133 | 0.285863748 | 118115 | 24 |
| UMVEN2000152 | 0.212724986 | 59628 | 12 |
| UMVEN2000354 | 0.203420643 | 156198 | 10 |
| UMVEN2000453 | 0.320907033 | 109109 | 47 |
| UTERU1000008 | 0.003395557 | 66387 | 2 |
| UTERU1000015 | 0.001998401 | 65373 | 1 |
| UTERU1000024 | 0.001998401 | 63730 | 1 |
| UTERU1000031 | 0.001998401 | 49244 | 1 |
| UTERU1000032 | 0.001998401 | 69926 | 1 |
| UTERU1000057 | 0.001998401 | 72104 | 1 |
| UTERU1000065 | 0.001998401 | 31345 | 1 |
| UTERU1000077 | 0.001998401 | 64540 | 1 |
| UTERU1000093 | 0.001998401 | 64580 | 1 |
| UTERU1000096 | 0.008234759 | 61603 | 2 |
| UTERU1000106 | 0.029076363 | 68024 | 7 |
| UTERU1000109 | 0.001998401 | 61936 | 1 |
| UTERU1000131 | 0.001998401 | 58954 | 1 |
| UTERU1000138 | 0.001998401 | 65283 | 1 |
| UTERU1000148 | 0.001998401 | 59066 | 1 |
| UTERU1000160 | 0.001998401 | 62014 | 1 |
| UTERU1000182 | 0.206560133 | 63305 | 18 |
| UTERU1000183 | 0.001998401 | 285325 | 1 |
| UTERU1000187 | 0.001998401 | 72182 | 1 |
| UTERU1000192 | 0.001998401 | 63618 | 1 |
| UTERU1000249 | 0.001998401 | 106727 | 1 |
| UTERU1000337 | 0.001998401 | 94863 | 1 |
| UTERU1000339 | 0.001998401 | 273538 | 1 |
| UTERU1000384 | 0.023509371 | 166464 | 10 |
| UTERU2000023 | 0.016367602 | 132806 | 10 |
| UTERU2000047 | 0.001998401 | 146975 | 1 |
| UTERU2000074 | 0.003187646 | 116970 | 2 |
| UTERU2000095 | 0.404833791 | 3101 | 68 |
| UTERU2000099 | 0.001998401 | 203684 | 1 |
| UTERU2000154 | 0.001998401 | 229 | 1 |
| UTERU2000197 | 0.005093897 | 174717 | 2 |
| UTERU2000218 | 0.001998401 | 134463 | 1 |
| UTERU2000238 | 0.001998401 | 186311 | 1 |
| UTERU2000243 | 0.001998401 | 172247 | 1 |
| UTERU2000260 | 0.008047982 | 41103 | 5 |
| UTERU2000263 | 0.327013605 | 137956 | 20 |
| UTERU2000300 | 0.033971044 | 127225 | 7 |
| UTERU2000329 | 0.005854917 | 239067 | 3 |
| UTERU2000332 | 0.035433658 | 135926 | 7 |
| UTERU2000338 | 0.050226674 | 122285 | 5 |
| UTERU2000349 | 0.001998401 | 147296 | 1 |
| UTERU2000377 | 0.001998401 | 84138 | 1 |
| UTERU2000393 | 0.001998401 | 231627 | 1 |
| UTERU2000418 | 0.005842485 | 254250 | 2 |
| UTERU2000424 | 0.197285034 | 27674 | 40 |
| UTERU2000465 | 0.001998401 | 223989 | 1 |
| UTERU2000485 | 0.013123109 | 170300 | 2 |
| UTERU2000517 | 0.001998401 | 50324 | 1 |
| UTERU2000524 | 0.001998401 | 200514 | 1 |
| UTERU2000537 | 0.032657348 | 131742 | 7 |
| UTERU2000539 | 0.001998401 | 223988 | 1 |
| UTERU2000541 | 0.026567306 | 142056 | 10 |
| UTERU2000542 | 0.018557296 | 73530 | 4 |
| UTERU2000546 | 0.001998401 | 103717 | 1 |
| UTERU2000550 | 0.001998401 | 215259 | 1 |
| UTERU2000569 | 0.001998401 | 189523 | 1 |
| UTERU2000607 | 0.001998401 | 266269 | 1 |
| UTERU2000629 | 0.001998401 | 121534 | 1 |
| UTERU2000649 | 0.001998401 | 268336 | 1 |
| UTERU2000663 | 0.001998401 | 263414 | 1 |
| UTERU2000696 | 0.058883424 | 144431 | 5 |
| UTERU2000794 | 0.003996803 | 199571 | 2 |
| UTERU2000830 | 0.001998401 | 263679 | 1 |
| UTERU2000844 | 0.001998401 | 80903 | 1 |
| UTERU2000922 | 0.001998401 | 75303 | 1 |
| UTERU2000925 | 0.016089745 | 73543 | 4 |
| UTERU2001024 | 0.001998401 | 3377 | 1 |
| UTERU2001110 | 0.008234759 | 96303 | 2 |
| UTERU2001176 | 0.001998401 | 276903 | 1 |
| UTERU2001281 | 0.001998401 | 131442 | 1 |
| UTERU2001389 | 0.001998401 | 99868 | 1 |
| UTERU2001409 | 0.001998401 | 261157 | 1 |
| UTERU2001412 | 0.001998401 | 98066 | 1 |
| UTERU2001504 | 0.001998401 | 223980 | 1 |
| UTERU2001607 | 0.113584757 | 111121 | 28 |
| UTERU2001658 | 0.018916318 | 48224 | 5 |
| UTERU2001747 | 0.001998401 | 153623 | 1 |
| UTERU2001876 | 0.001998401 | 166600 | 1 |
| UTERU2002001 | 0.00498036 | 55492 | 2 |
| UTERU2002011 | 0.001998401 | 96964 | 1 |
| UTERU2002176 | 0.001998401 | 215313 | 1 |
| UTERU2002198 | 0.00309368 | 134252 | 2 |
| UTERU2002294 | 0.092302324 | 17676 | 16 |
| UTERU2002332 | 0.001998401 | 226912 | 1 |
| UTERU2002410 | 0.285374963 | 70646 | 26 |
| UTERU2002473 | 0.001998401 | 280066 | 1 |
| UTERU2002547 | 0.001998401 | 221007 | 1 |
| UTERU2002662 | 0.094171422 | 108330 | 19 |
| UTERU2002693 | 0.001998401 | 56704 | 1 |
| UTERU2002733 | 0.001998401 | 278403 | 1 |
| UTERU2002736 | 0.001998401 | 58849 | 1 |
| UTERU2002737 | 0.001998401 | 272352 | 1 |
| UTERU2002826 | 0.045394069 | 203189 | 3 |
| UTERU2002841 | 0.00309368 | 219651 | 2 |
| UTERU2002964 | 0.007934176 | 169837 | 2 |
| UTERU2002993 | 0.00309368 | 141409 | 2 |
| UTERU2003035 | 0.001998401 | 40501 | 1 |
| UTERU2003057 | 0.06478483 | 125914 | 6 |
| UTERU2003126 | 0.001998401 | 185863 | 1 |
| UTERU2003135 | 0.001998401 | 60031 | 1 |
| UTERU2003321 | 0.004188958 | 142542 | 3 |
| UTERU2003399 | 0.001998401 | 253284 | 1 |
| UTERU2003411 | 0.554484589 | 103791 | 2 |
| UTERU2003456 | 0.015493053 | 41365 | 3 |
| UTERU2003577 | 0.001998401 | 13719 | 1 |
| UTERU2003704 | 0.009734723 | 123249 | 5 |
| UTERU2003926 | 0.00498036 | 124106 | 2 |
| UTERU2003973 | 0.001998401 | 202842 | 1 |
| UTERU2004015 | 0.001998401 | 271090 | 1 |
| UTERU2004037 | 0.076420451 | 150477 | 10 |
| UTERU2004039 | 0.001998401 | 161733 | 1 |
| UTERU2004061 | 0.001998401 | 150696 | 1 |
| UTERU2004073 | 0.008570071 | 136317 | 7 |
| UTERU2004163 | 0.001998401 | 74684 | 1 |
| UTERU2004197 | 0.083329792 | 51325 | 22 |
| UTERU2004299 | 0.001998401 | 159949 | 1 |
| UTERU2004461 | 0.006175336 | 50451 | 2 |
| UTERU2004520 | 0.005119208 | 140013 | 2 |
| UTERU2004564 | 0.001998401 | 23680 | 1 |
| UTERU2004664 | 0.098744332 | 66382 | 11 |
| UTERU2004688 | 0.150700082 | 166725 | 9 |
| UTERU2004698 | 0.003996803 | 193584 | 2 |
| UTERU2004807 | 0.166790628 | 104951 | 43 |
| UTERU2004861 | 0.007082235 | 183097 | 4 |
| UTERU2004929 | 0.014433758 | 100100 | 5 |
| UTERU2005004 | 0.004792712 | 43867 | 3 |
| UTERU2005050 | 0.001998401 | 179625 | 1 |
| UTERU2005069 | 0.00309368 | 143875 | 2 |
| UTERU2005074 | 0.014755553 | 148678 | 8 |
| UTERU2005179 | 0.001998401 | 109601 | 1 |
| UTERU2005292 | 0.053810396 | 138204 | 5 |
| UTERU2005346 | 0.001998401 | 83910 | 1 |
| UTERU2005354 | 0.076598619 | 6825 | 26 |
| UTERU2005446 | 0.00805277 | 154624 | 2 |
| UTERU2005449 | 0.010234672 | 98960 | 6 |
| UTERU2005450 | 0.001998401 | 237872 | 1 |
| UTERU2005533 | 0.001998401 | 222768 | 1 |
| UTERU2005548 | 0.048671412 | 104937 | 15 |
| UTERU2005593 | 0.149281574 | 83214 | 41 |
| UTERU2005601 | 0.065734037 | 115239 | 34 |
| UTERU2005621 | 0.074332556 | 29393 | 18 |
| UTERU2005664 | 0.014994543 | 187266 | 3 |
| UTERU2005822 | 0.001998401 | 8502 | 1 |
| UTERU2005903 | 0.001998401 | 104089 | 1 |
| UTERU2005905 | 0.001998401 | 118775 | 1 |
| UTERU2006103 | 0.001998401 | 95939 | 1 |
| UTERU2006115 | 0.021379086 | 143247 | 7 |
| UTERU2006137 | 0.021560217 | 102624 | 2 |
| UTERU2006182 | 0.003996803 | 147740 | 2 |
| UTERU2006400 | 0.013123109 | 138905 | 2 |
| UTERU2006412 | 0.001998401 | 118103 | 1 |
| UTERU2006429 | 0.001998401 | 64441 | 1 |
| UTERU2006486 | 0.003856516 | 174516 | 2 |
| UTERU2006524 | 0.003707861 | 151390 | 2 |
| UTERU2006547 | 0.008234759 | 79342 | 2 |
| UTERU2006568 | 0.001998401 | 161469 | 1 |
| UTERU2006593 | 0.113479648 | 132195 | 41 |
| UTERU2006643 | 0.019732194 | 59845 | 3 |
| UTERU2006651 | 0.001998401 | 271866 | 1 |
| UTERU2006705 | 0.001998401 | 221468 | 1 |
| UTERU2006899 | 0.001998401 | 148288 | 1 |
| UTERU2007004 | 0.008039967 | 241022 | 2 |
| UTERU2007075 | 0.001998401 | 135906 | 1 |
| UTERU2007081 | 0.011452878 | 167288 | 2 |
| UTERU2007128 | 0.001998401 | 51454 | 1 |
| UTERU2007253 | 0.016260617 | 112300 | 5 |
| UTERU2007267 | 0.001998401 | 153321 | 1 |
| UTERU2007444 | 0.00309368 | 158561 | 2 |
| UTERU2007499 | 0.001998401 | 108273 | 1 |
| UTERU2007520 | 0.024981157 | 167548 | 8 |
| UTERU2007639 | 0.001998401 | 193614 | 1 |
| UTERU2007724 | 0.025503558 | 15798 | 4 |
| UTERU2007924 | 0.062648093 | 140717 | 6 |
| UTERU2007942 | 0.001998401 | 104167 | 1 |
| UTERU2008018 | 0.008039967 | 204171 | 2 |
| UTERU2008019 | 0.001998401 | 213230 | 1 |
| UTERU2008027 | 0.043804421 | 42531 | 2 |
| UTERU2008040 | 0.014236966 | 215835 | 6 |
| UTERU2008077 | 0.001998401 | 218802 | 1 |
| UTERU2008085 | 1.855334107 | 34899 | 204 |
| UTERU2008130 | 0.003707861 | 204355 | 2 |
| UTERU2008302 | 0.125417087 | 97790 | 22 |
| UTERU2008347 | 0.001998401 | 269130 | 1 |
| UTERU2008426 | 0.007610199 | 132718 | 4 |
| UTERU2008516 | 0.001998401 | 82007 | 1 |
| UTERU2008561 | 0.001998401 | 218717 | 1 |
| UTERU2008653 | 0.004079913 | 134537 | 2 |
| UTERU2008705 | 0.001998401 | 224032 | 1 |
| UTERU2008707 | 0.001998401 | 5280 | 1 |
| UTERU2008747 | 0.018958528 | 207523 | 6 |
| UTERU2008785 | 0.001998401 | 115157 | 1 |
| UTERU2008845 | 0.001998401 | 19142 | 1 |
| UTERU2008901 | 0.001998401 | 14967 | 1 |
| UTERU2008930 | 0.001998401 | 272015 | 1 |
| UTERU2008938 | 0.068082902 | 98528 | 25 |
| UTERU2008939 | 0.011280225 | 94460 | 5 |
| UTERU2008962 | 0.00309368 | 173614 | 2 |
| UTERU2009094 | 0.001998401 | 162580 | 1 |
| UTERU2009120 | 0.001998401 | 260323 | 1 |
| UTERU2009131 | 0.001998401 | 133684 | 1 |
| UTERU2009147 | 0.001998401 | 125716 | 1 |
| UTERU2009206 | 0.001998401 | 263287 | 1 |
| UTERU2009283 | 0.001998401 | 228785 | 1 |
| UTERU2009335 | 0.055244213 | 147531 | 13 |
| UTERU2009414 | 0.001998401 | 5143 | 1 |
| UTERU2009435 | 0.031333709 | 101088 | 17 |
| UTERU2009483 | 0.001998401 | 183652 | 1 |
| UTERU2009510 | 0.011148266 | 142395 | 3 |
| UTERU2009538 | 0.004913258 | 145821 | 2 |
| UTERU2009540 | 0.001998401 | 32277 | 1 |
| UTERU2009776 | 0.001998401 | 149019 | 1 |
| UTERU2009904 | 0.001998401 | 189763 | 1 |
| UTERU2009951 | 0.001998401 | 123588 | 1 |
| UTERU2009972 | 0.047295514 | 146138 | 21 |
| UTERU2010115 | 0.001998401 | 157626 | 1 |
| UTERU2010124 | 0.001998401 | 152531 | 1 |
| UTERU2010164 | 0.001998401 | 6430 | 1 |
| UTERU2010226 | 0.021240948 | 100742 | 8 |
| UTERU2010231 | 0.001998401 | 137754 | 1 |
| UTERU2010304 | 0.001998401 | 6762 | 1 |
| UTERU2010320 | 0.007934176 | 139155 | 2 |
| UTERU2010417 | 0.531462578 | 36734 | 68 |
| UTERU2010431 | 0.001998401 | 70488 | 1 |
| UTERU2010525 | 0.014220352 | 215460 | 2 |
| UTERU2010651 | 0.001998401 | 81252 | 1 |
| UTERU2010724 | 0.001998401 | 70551 | 1 |
| UTERU2010747 | 0.003187646 | 148141 | 2 |
| UTERU2011195 | 0.001998401 | 107132 | 1 |
| UTERU2011199 | 0.001998401 | 182511 | 1 |
| UTERU2011220 | 0.085610789 | 107268 | 7 |
| UTERU2011261 | 0.001998401 | 97533 | 1 |
| UTERU2011287 | 0.005105017 | 66231 | 3 |
| UTERU2011410 | 0.001998401 | 92897 | 1 |
| UTERU2011574 | 0.001998401 | 23115 | 1 |
| UTERU2011621 | 0.016874324 | 40087 | 11 |
| UTERU2011657 | 0.001998401 | 79864 | 1 |
| UTERU2011741 | 0.014222755 | 107734 | 3 |
| UTERU2011806 | 0.01203677 | 80671 | 4 |
| UTERU2011811 | 0.001998401 | 164668 | 1 |
| UTERU2011897 | 0.003996803 | 134356 | 2 |
| UTERU2011906 | 0.024570185 | 47614 | 12 |
| UTERU2011962 | 0.001998401 | 213368 | 1 |
| UTERU2011968 | 0.001998401 | 60456 | 1 |
| UTERU2012031 | 0.003707861 | 88460 | 2 |
| UTERU2012101 | 0.001998401 | 72326 | 1 |
| UTERU2012114 | 0.001998401 | 90586 | 1 |
| UTERU2012230 | 0.095212005 | 86533 | 46 |
| UTERU2012252 | 0.007803579 | 128908 | 2 |
| UTERU2012286 | 0.066135103 | 52303 | 17 |
| UTERU2012333 | 0.001998401 | 8191 | 1 |
| UTERU2012407 | 0.006807484 | 138956 | 3 |
| UTERU2012526 | 0.018145421 | 198226 | 4 |
| UTERU2012581 | 0.001998401 | 247010 | 1 |
| UTERU2012610 | 0.001998401 | 2829 | 1 |
| UTERU2012615 | 0.001998401 | 146946 | 1 |
| UTERU2012688 | 0.003875243 | 129602 | 2 |
| UTERU2012703 | 0.008143927 | 85083 | 2 |
| UTERU2012715 | 0.005842485 | 41038 | 2 |
| UTERU2012741 | 0.009308168 | 94373 | 3 |
| UTERU2012767 | 0.031464993 | 141357 | 7 |
| UTERU2012786 | 0.001998401 | 235009 | 1 |
| UTERU2012856 | 0.047493293 | 161910 | 9 |
| UTERU2012890 | 0.001998401 | 163981 | 1 |
| UTERU2012938 | 0.004079913 | 70568 | 2 |
| UTERU2012976 | 0.001998401 | 135818 | 1 |
| UTERU2013048 | 0.001998401 | 4112 | 1 |
| UTERU2013078 | 0.012216902 | 11354 | 3 |
| UTERU2013231 | 0.001998401 | 133522 | 1 |
| UTERU2013262 | 0.009130364 | 135895 | 4 |
| UTERU2013280 | 0.001998401 | 82383 | 1 |
| UTERU2013322 | 0.001998401 | 38383 | 1 |
| UTERU2013483 | 0.001998401 | 149577 | 1 |
| UTERU2013491 | 0.014347129 | 55397 | 2 |
| UTERU2013502 | 0.009938926 | 147619 | 3 |
| UTERU2013586 | 0.025430779 | 62908 | 8 |
| UTERU2013926 | 0.001998401 | 29834 | 1 |
| UTERU2013976 | 0.001998401 | 78667 | 1 |
| UTERU2014001 | 0.001998401 | 160270 | 1 |
| UTERU2014024 | 0.012929125 | 31810 | 6 |
| UTERU2014167 | 0.183838856 | 155857 | 10 |
| UTERU2014223 | 0.001998401 | 82195 | 1 |
| UTERU2014398 | 0.001998401 | 239853 | 1 |
| UTERU2014464 | 0.012411694 | 56397 | 3 |
| UTERU2014548 | 0.005093897 | 6955 | 2 |
| UTERU2014601 | 0.024193577 | 197076 | 7 |
| UTERU2014631 | 0.001998401 | 232031 | 1 |
| UTERU2014668 | 0.005093897 | 190549 | 2 |
| UTERU2014678 | 0.004079913 | 85380 | 2 |
| UTERU2014728 | 0.003996803 | 168211 | 2 |
| UTERU2014898 | 0.11011669 | 28697 | 11 |
| UTERU2014998 | 0.001998401 | 239871 | 1 |
| UTERU2015062 | 0.011707139 | 171848 | 2 |
| UTERU2015087 | 0.001998401 | 169383 | 1 |
| UTERU2015108 | 0.026553347 | 132270 | 5 |
| UTERU2015190 | 0.004913258 | 206797 | 2 |
| UTERU2015198 | 0.001998401 | 116811 | 1 |
| UTERU2015202 | 0.001998401 | 146004 | 1 |
| UTERU2015405 | 0.001998401 | 109439 | 1 |
| UTERU2015640 | 0.001998401 | 16690 | 1 |
| UTERU2015653 | 0.287208797 | 97047 | 18 |
| UTERU2015830 | 0.003395557 | 72610 | 2 |
| UTERU2015880 | 0.001998401 | 31843 | 1 |
| UTERU2016147 | 0.001998401 | 81082 | 1 |
| UTERU2016157 | 0.001998401 | 94377 | 1 |
| UTERU2016426 | 0.013184237 | 162313 | 5 |
| UTERU2016464 | 0.001998401 | 269024 | 1 |
| UTERU2016669 | 0.020292735 | 215098 | 3 |
| UTERU2016757 | 0.003673948 | 32349 | 2 |
| UTERU2016761 | 0.001998401 | 171385 | 1 |
| UTERU2016799 | 0.057624333 | 101982 | 21 |
| UTERU2016822 | 0.083362439 | 84687 | 35 |
| UTERU2016896 | 0.001998401 | 162391 | 1 |
| UTERU2016902 | 0.001998401 | 187357 | 1 |
| UTERU2016979 | 0.01619994 | 8837 | 3 |
| UTERU2016981 | 0.001998401 | 47982 | 1 |
| UTERU2017123 | 0.001998401 | 143755 | 1 |
| UTERU2017303 | 0.001998401 | 55874 | 1 |
| UTERU2017421 | 0.001998401 | 197217 | 1 |
| UTERU2017492 | 0.038454836 | 52564 | 2 |
| UTERU2017613 | 0.016294395 | 39175 | 4 |
| UTERU2017623 | 0.00309368 | 122667 | 2 |
| UTERU2017632 | 0.00309368 | 58403 | 2 |
| UTERU2017715 | 0.007152616 | 157433 | 4 |
| UTERU2017761 | 0.003187646 | 141890 | 2 |
| UTERU2017762 | 0.005119208 | 73416 | 2 |
| UTERU2017810 | 0.001998401 | 85672 | 1 |
| UTERU2017988 | 0.001998401 | 165712 | 1 |
| UTERU2018127 | 0.001998401 | 236281 | 1 |
| UTERU2018180 | 0.001998401 | 78505 | 1 |
| UTERU2018200 | 0.006903875 | 149501 | 4 |
| UTERU2018333 | 0.001998401 | 215990 | 1 |
| UTERU2018364 | 0.003707861 | 75498 | 2 |
| UTERU2018514 | 0.001998401 | 190425 | 1 |
| UTERU2018522 | 0.001998401 | 114937 | 1 |
| UTERU2018523 | 0.00498036 | 86740 | 2 |
| UTERU2018544 | 0.001998401 | 90477 | 1 |
| UTERU2018566 | 0.009400755 | 122570 | 3 |
| UTERU2018609 | 0.003996803 | 236279 | 2 |
| UTERU2018674 | 0.001998401 | 188491 | 1 |
| UTERU2018712 | 0.003686678 | 43628 | 2 |
| UTERU2018784 | 0.001998401 | 139259 | 1 |
| UTERU2018789 | 0.003996803 | 210220 | 2 |
| UTERU2018811 | 0.001998401 | 102308 | 1 |
| UTERU2018867 | 0.001998401 | 151070 | 1 |
| UTERU2018881 | 0.001998401 | 268110 | 1 |
| UTERU2018884 | 0.108156749 | 127476 | 11 |
| UTERU2018955 | 0.001998401 | 17650 | 1 |
| UTERU2019005 | 0.02594312 | 20990 | 9 |
| UTERU2019038 | 0.001998401 | 127723 | 1 |
| UTERU2019096 | 0.072738751 | 76116 | 30 |
| UTERU2019163 | 0.001998401 | 223309 | 1 |
| UTERU2019257 | 0.006978762 | 47220 | 3 |
| UTERU2019453 | 0.001998401 | 165048 | 1 |
| UTERU2019491 | 0.001998401 | 234059 | 1 |
| UTERU2019534 | 0.001998401 | 151888 | 1 |
| UTERU2019681 | 0.001998401 | 199570 | 1 |
| UTERU2019706 | 0.003673948 | 17526 | 2 |
| UTERU2019710 | 0.001998401 | 155384 | 1 |
| UTERU2019940 | 0.003856516 | 136041 | 2 |
| UTERU2019959 | 0.001998401 | 143030 | 1 |
| UTERU2019964 | 0.001998401 | 278323 | 1 |
| UTERU2020226 | 0.005092081 | 175149 | 3 |
| UTERU2020242 | 0.001998401 | 212049 | 1 |
| UTERU2020292 | 0.001998401 | 143848 | 1 |
| UTERU2020351 | 0.001998401 | 160825 | 1 |
| UTERU2020491 | 0.001998401 | 119886 | 1 |
| UTERU2020718 | 0.001998401 | 176193 | 1 |
| UTERU2021163 | 0.003707861 | 115116 | 2 |
| UTERU2021380 | 0.001998401 | 129274 | 1 |
| UTERU2021649 | 0.001998401 | 127284 | 1 |
| UTERU2021820 | 0.006721307 | 127280 | 4 |
| UTERU2021917 | 0.078238458 | 113092 | 11 |
| UTERU2022020 | 0.001998401 | 182424 | 1 |
| UTERU2022773 | 0.001998401 | 196087 | 1 |
| UTERU2022955 | 0.001998401 | 283796 | 1 |
| UTERU2022981 | 0.001998401 | 149897 | 1 |
| UTERU2023039 | 0.003673948 | 105684 | 2 |
| UTERU2023045 | 0.018513218 | 100379 | 3 |
| UTERU2023175 | 0.112542976 | 125169 | 22 |
| UTERU2023262 | 0.013285083 | 186947 | 2 |
| UTERU2023550 | 0.020007096 | 128119 | 3 |
| UTERU2023651 | 0.006669554 | 129059 | 4 |
| UTERU2023687 | 0.001998401 | 127449 | 1 |
| UTERU2023712 | 0.001998401 | 127448 | 1 |
| UTERU2023941 | 0.001998401 | 278688 | 1 |
| UTERU2024002 | 0.001998401 | 145867 | 1 |
| UTERU2024042 | 0.001998401 | 131164 | 1 |
| UTERU2024141 | 0.001998401 | 131145 | 1 |
| UTERU2024481 | 0.001998401 | 120326 | 1 |
| UTERU2024656 | 0.003875243 | 131565 | 2 |
| UTERU2024758 | 0.001998401 | 141255 | 1 |
| UTERU2024820 | 0.001998401 | 135201 | 1 |
| UTERU2024881 | 0.001998401 | 268827 | 1 |
| UTERU2024969 | 0.052177104 | 10445 | 14 |
| UTERU2025025 | 0.001998401 | 145590 | 1 |
| UTERU2025041 | 0.001998401 | 161212 | 1 |
| UTERU2025301 | 0.038548325 | 20649 | 3 |
| UTERU2025366 | 0.001998401 | 186379 | 1 |
| UTERU2025415 | 0.001998401 | 158568 | 1 |
| UTERU2025579 | 0.001998401 | 158509 | 1 |
| UTERU2025645 | 0.001998401 | 144423 | 1 |
| UTERU2025891 | 0.001998401 | 141403 | 1 |
| UTERU2026025 | 0.001998401 | 3373 | 1 |
| UTERU2026090 | 0.001998401 | 36823 | 1 |
| UTERU2026142 | 0.001998401 | 2437 | 1 |
| UTERU2026203 | 0.003673948 | 48574 | 2 |
| UTERU2026775 | 0.001998401 | 184618 | 1 |
| UTERU2027023 | 0.001998401 | 253781 | 1 |
| UTERU2027369 | 0.005093897 | 146016 | 2 |
| UTERU2027591 | 0.001998401 | 134957 | 1 |
| UTERU2027616 | 0.001998401 | 153533 | 1 |
| UTERU2027941 | 0.001998401 | 100554 | 1 |
| UTERU2028377 | 0.001998401 | 196538 | 1 |
| UTERU2028734 | 0.007632302 | 166780 | 4 |
| UTERU2029503 | 0.001998401 | 175059 | 1 |
| UTERU2029660 | 0.001998401 | 175054 | 1 |
| UTERU2029742 | 0.001998401 | 147091 | 1 |
| UTERU2029953 | 0.001998401 | 234419 | 1 |
| UTERU2030213 | 0.006801541 | 133222 | 4 |
| UTERU2030270 | 0.001998401 | 219426 | 1 |
| UTERU2030280 | 0.003856516 | 144611 | 2 |
| UTERU2031060 | 0.001998401 | 91885 | 1 |
| UTERU2031084 | 0.007707569 | 165571 | 4 |
| UTERU2031268 | 0.001998401 | 134279 | 1 |
| UTERU2031295 | 0.001998401 | 254920 | 1 |
| UTERU2031521 | 0.001998401 | 234361 | 1 |
| UTERU2031611 | 0.001998401 | 234359 | 1 |
| UTERU2031703 | 0.005995204 | 210851 | 3 |
| UTERU2031834 | 0.001998401 | 193763 | 1 |
| UTERU2031851 | 0.001998401 | 196139 | 1 |
| UTERU2032075 | 0.001998401 | 281401 | 1 |
| UTERU2032220 | 0.001998401 | 214186 | 1 |
| UTERU2032279 | 0.001998401 | 242363 | 1 |
| UTERU2032726 | 0.005995204 | 2436 | 3 |
| UTERU2033172 | 0.001998401 | 261883 | 1 |
| UTERU2033375 | 0.144245912 | 241080 | 2 |
| UTERU2033382 | 0.001998401 | 248120 | 1 |
| UTERU2033420 | 0.001998401 | 143627 | 1 |
| UTERU2033530 | 0.001998401 | 197493 | 1 |
| UTERU2033577 | 0.001998401 | 96343 | 1 |
| UTERU2034053 | 0.001998401 | 254115 | 1 |
| UTERU2034147 | 0.003395557 | 254120 | 2 |
| UTERU2034695 | 0.003996803 | 237328 | 2 |
| UTERU2035114 | 0.003875243 | 203789 | 2 |
| UTERU2035187 | 0.001998401 | 257575 | 1 |
| UTERU2035231 | 0.001998401 | 33924 | 1 |
| UTERU2035306 | 0.001998401 | 211158 | 1 |
| UTERU2035323 | 0.001998401 | 193802 | 1 |
| UTERU2035328 | 0.001998401 | 119566 | 1 |
| UTERU2035331 | 0.001998401 | 69592 | 1 |
| UTERU2035452 | 0.001998401 | 280898 | 1 |
| UTERU2035469 | 0.001998401 | 158462 | 1 |
| UTERU2035503 | 0.001998401 | 60506 | 1 |
| UTERU2035745 | 0.001998401 | 277244 | 1 |
| UTERU2035908 | 0.001998401 | 258967 | 1 |
| UTERU2035926 | 0.001998401 | 282915 | 1 |
| UTERU2035978 | 0.001998401 | 251262 | 1 |
| UTERU2036089 | 0.001998401 | 200000 | 1 |
| UTERU2036347 | 0.005272398 | 223716 | 3 |
| UTERU2036507 | 0.001998401 | 143624 | 1 |
| UTERU2036512 | 0.001998401 | 269886 | 1 |
| UTERU2036530 | 0.001998401 | 223437 | 1 |
| UTERU2036623 | 0.001998401 | 188337 | 1 |
| UTERU2036690 | 0.001998401 | 250009 | 1 |
| UTERU2037224 | 0.001998401 | 182026 | 1 |
| UTERU2037361 | 0.001998401 | 263630 | 1 |
| UTERU2037423 | 0.001998401 | 282513 | 1 |
| UTERU2037577 | 0.001998401 | 277568 | 1 |
| UTERU2037674 | 0.001998401 | 260428 | 1 |
| UTERU2037791 | 0.001998401 | 200187 | 1 |
| UTERU2037843 | 0.001998401 | 269343 | 1 |
| UTERU2038171 | 0.001998401 | 232075 | 1 |
| UTERU2038251 | 0.001998401 | 213908 | 1 |
| UTERU3000226 | 0.003187646 | 176587 | 2 |
| UTERU3000298 | 0.036215441 | 100290 | 21 |
| UTERU3000402 | 0.001998401 | 190735 | 1 |
| UTERU3000645 | 0.005752085 | 190615 | 3 |
| UTERU3000665 | 0.001998401 | 257544 | 1 |
| UTERU3000670 | 0.007184448 | 73796 | 4 |
| UTERU3000727 | 0.007421772 | 95143 | 4 |
| UTERU3000738 | 0.004792712 | 162477 | 3 |
| UTERU3000828 | 0.001998401 | 52798 | 1 |
| UTERU3000844 | 0.00309368 | 40373 | 2 |
| UTERU3000899 | 0.005938028 | 2846 | 3 |
| UTERU3000959 | 0.001998401 | 284948 | 1 |
| UTERU3001029 | 0.001998401 | 102994 | 1 |
| UTERU3001053 | 0.001998401 | 175478 | 1 |
| UTERU3001059 | 0.001998401 | 280380 | 1 |
| UTERU3001158 | 0.00504576 | 197739 | 3 |
| UTERU3001240 | 0.001998401 | 177141 | 1 |
| UTERU3001394 | 0.001998401 | 254737 | 1 |
| UTERU3001542 | 0.003395557 | 19767 | 2 |
| UTERU3001558 | 0.001998401 | 236900 | 1 |
| UTERU3001571 | 0.003856516 | 40689 | 2 |
| UTERU3001572 | 0.091784057 | 141213 | 31 |
| UTERU3001585 | 0.001998401 | 88250 | 1 |
| UTERU3001632 | 0.001998401 | 84899 | 1 |
| UTERU3001652 | 0.001998401 | 60661 | 1 |
| UTERU3001766 | 0.003686678 | 55680 | 2 |
| UTERU3001946 | 0.005752085 | 212445 | 3 |
| UTERU3001988 | 0.001998401 | 128229 | 1 |
| UTERU3002209 | 0.001998401 | 257899 | 1 |
| UTERU3002218 | 0.003875243 | 37749 | 2 |
| UTERU3002383 | 0.001998401 | 230613 | 1 |
| UTERU3002600 | 0.001998401 | 151716 | 1 |
| UTERU3002620 | 0.001998401 | 279430 | 1 |
| UTERU3002667 | 0.001998401 | 106655 | 1 |
| UTERU3002701 | 0.001998401 | 226397 | 1 |
| UTERU3002731 | 0.001998401 | 231875 | 1 |
| UTERU3002768 | 0.001998401 | 237130 | 1 |
| UTERU3002786 | 0.003875243 | 268502 | 2 |
| UTERU3002993 | 0.001998401 | 63801 | 1 |
| UTERU3003116 | 0.001998401 | 284030 | 1 |
| UTERU3003135 | 0.005105017 | 165006 | 3 |
| UTERU3003178 | 0.001998401 | 255016 | 1 |
| UTERU3003465 | 0.001998401 | 111353 | 1 |
| UTERU3003495 | 0.001998401 | 215937 | 1 |
| UTERU3003523 | 0.003996803 | 139194 | 2 |
| UTERU3003660 | 0.02275735 | 114890 | 12 |
| UTERU3003774 | 0.001998401 | 215611 | 1 |
| UTERU3003776 | 0.001998401 | 210170 | 1 |
| UTERU3004384 | 0.003707861 | 228918 | 2 |
| UTERU3004418 | 0.00575546 | 180837 | 3 |
| UTERU3004477 | 0.006978762 | 14725 | 3 |
| UTERU3004523 | 0.001998401 | 260355 | 1 |
| UTERU3004616 | 0.001998401 | 122663 | 1 |
| UTERU3004635 | 0.001998401 | 106981 | 1 |
| UTERU3004709 | 0.003856516 | 261357 | 2 |
| UTERU3004938 | 0.001998401 | 237143 | 1 |
| UTERU3004992 | 0.001998401 | 88667 | 1 |
| UTERU3005049 | 0.003395557 | 177034 | 2 |
| UTERU3005159 | 0.00480314 | 123286 | 3 |
| UTERU3005205 | 0.001998401 | 259676 | 1 |
| UTERU3005230 | 0.006861594 | 232433 | 4 |
| UTERU3005264 | 0.001998401 | 128421 | 1 |
| UTERU3005422 | 0.007899328 | 132008 | 5 |
| UTERU3005460 | 0.003187646 | 130111 | 2 |
| UTERU3005536 | 0.003187646 | 172711 | 2 |
| UTERU3005585 | 0.001998401 | 178505 | 1 |
| UTERU3005767 | 0.140779323 | 2430 | 18 |
| UTERU3005907 | 0.025604786 | 86906 | 10 |
| UTERU3005970 | 0.003187646 | 234920 | 2 |
| UTERU3006008 | 0.001998401 | 253873 | 1 |
| UTERU3006228 | 0.001998401 | 197993 | 1 |
| UTERU3006308 | 0.003187646 | 258132 | 2 |
| UTERU3006538 | 0.001998401 | 269849 | 1 |
| UTERU3006640 | 0.001998401 | 270023 | 1 |
| UTERU3006687 | 0.001998401 | 253166 | 1 |
| UTERU3006720 | 0.001998401 | 88015 | 1 |
| UTERU3006798 | 0.001998401 | 259657 | 1 |
| UTERU3006884 | 0.001998401 | 257570 | 1 |
| UTERU3007021 | 0.005752085 | 224634 | 3 |
| UTERU3007104 | 0.003875243 | 84614 | 2 |
| UTERU3007108 | 0.001998401 | 138678 | 1 |
| UTERU3007134 | 0.001998401 | 168512 | 1 |
| UTERU3007419 | 0.003187646 | 23425 | 2 |
| UTERU3007640 | 0.003673948 | 78942 | 2 |
| UTERU3007731 | 0.001998401 | 5355 | 1 |
| UTERU3007913 | 0.00918285 | 83826 | 5 |
| UTERU3008280 | 0.005383408 | 241632 | 3 |
| UTERU3008463 | 0.001998401 | 245478 | 1 |
| UTERU3008660 | 0.001998401 | 241635 | 1 |
| UTERU3008671 | 0.001998401 | 222375 | 1 |
| UTERU3008722 | 0.001998401 | 199036 | 1 |
| UTERU3008917 | 0.001998401 | 257765 | 1 |
| UTERU3009259 | 0.003707861 | 253574 | 2 |
| UTERU3009490 | 0.001998401 | 251113 | 1 |
| UTERU3009517 | 0.003686678 | 247136 | 2 |
| UTERU3009690 | 0.001998401 | 242362 | 1 |
| UTERU3009775 | 0.001998401 | 226679 | 1 |
| UTERU3009871 | 0.009249512 | 116483 | 6 |
| UTERU3009979 | 0.001998401 | 237177 | 1 |
| UTERU3009987 | 0.001998401 | 250826 | 1 |
| UTERU3010029 | 0.001998401 | 250905 | 1 |
| UTERU3010409 | 0.048410381 | 88653 | 28 |
| UTERU3010526 | 0.001998401 | 274951 | 1 |
| UTERU3010604 | 0.00498036 | 82604 | 2 |
| UTERU3010892 | 0.001998401 | 248839 | 1 |
| UTERU3010919 | 0.001998401 | 119649 | 1 |
| UTERU3011063 | 0.001998401 | 51546 | 1 |
| UTERU3011092 | 0.001998401 | 275021 | 1 |
| UTERU3011273 | 0.001998401 | 122670 | 1 |
| UTERU3011398 | 0.001998401 | 251326 | 1 |
| UTERU3011558 | 0.001998401 | 248534 | 1 |
| UTERU3011579 | 0.005752085 | 36391 | 3 |
| UTERU3011795 | 0.001998401 | 248454 | 1 |
| UTERU3011837 | 0.001998401 | 250782 | 1 |
| UTERU3012293 | 0.001998401 | 198501 | 1 |
| UTERU3012414 | 0.003856516 | 94116 | 2 |
| UTERU3012476 | 0.001998401 | 231872 | 1 |
| UTERU3012599 | 0.001998401 | 242320 | 1 |
| UTERU3012999 | 0.001998401 | 117040 | 1 |
| UTERU3013167 | 0.003673948 | 239030 | 2 |
| UTERU3013302 | 0.003395557 | 122853 | 2 |
| UTERU3013781 | 0.001998401 | 161399 | 1 |
| UTERU3014274 | 0.001998401 | 212983 | 1 |
| UTERU3014446 | 0.001998401 | 274560 | 1 |
| UTERU3014611 | 0.003996803 | 276399 | 2 |
| UTERU3014647 | 0.001998401 | 253717 | 1 |
| UTERU3014791 | 0.001998401 | 258461 | 1 |
| UTERU3014906 | 0.001998401 | 285379 | 1 |
| UTERU3015011 | 0.023741785 | 136725 | 6 |
| UTERU3015069 | 0.026954175 | 166297 | 5 |
| UTERU3015086 | 0.001998401 | 127588 | 1 |
| UTERU3015299 | 0.001998401 | 285157 | 1 |
| UTERU3015412 | 0.001998401 | 98550 | 1 |
| UTERU3015500 | 0.001998401 | 231943 | 1 |
| UTERU3015647 | 0.001998401 | 120558 | 1 |
| UTERU3015844 | 0.001998401 | 245172 | 1 |
| UTERU3016070 | 0.001998401 | 138887 | 1 |
| UTERU3016273 | 0.003707861 | 136846 | 2 |
| UTERU3016274 | 0.001998401 | 249281 | 1 |
| UTERU3016308 | 0.001998401 | 53805 | 1 |
| UTERU3016789 | 0.001998401 | 274239 | 1 |
| UTERU3017176 | 0.001998401 | 197571 | 1 |
| UTERU3017441 | 0.001998401 | 34936 | 1 |
| UTERU3017626 | 0.001998401 | 231963 | 1 |
| UTERU3017995 | 0.015320462 | 179323 | 3 |
| UTERU3018081 | 0.004970521 | 182565 | 3 |
| UTERU3018154 | 0.001998401 | 235077 | 1 |
| UTERU3018172 | 0.005253671 | 89443 | 3 |
| UTERU3018255 | 0.001998401 | 162206 | 1 |
| UTERU3018616 | 0.007940525 | 199429 | 2 |
| UTERU3018711 | 0.013867249 | 106656 | 7 |
| UTERU3019078 | 0.001998401 | 162073 | 1 |

This analysis yielded information on the expression frequencies of the 17,176 genes that include the cDNAs of the present invention, in all tissues and cells used as sources of the respective cDNA libraries and described in Example 1.

Of the 17,176 genes that include the cDNAs of the present invention, genes whose expression levels were high enough to detect in each of the 3NB69, ACTVT, ADIPS, ADRGL, AHMSC, ASTRO, and BEAST libraries were analyzed for expression frequency. The analysis results are described below. Each is a relative value, where the expression level over all tissues is taken as 100. The higher this value, the greater the level of expression.

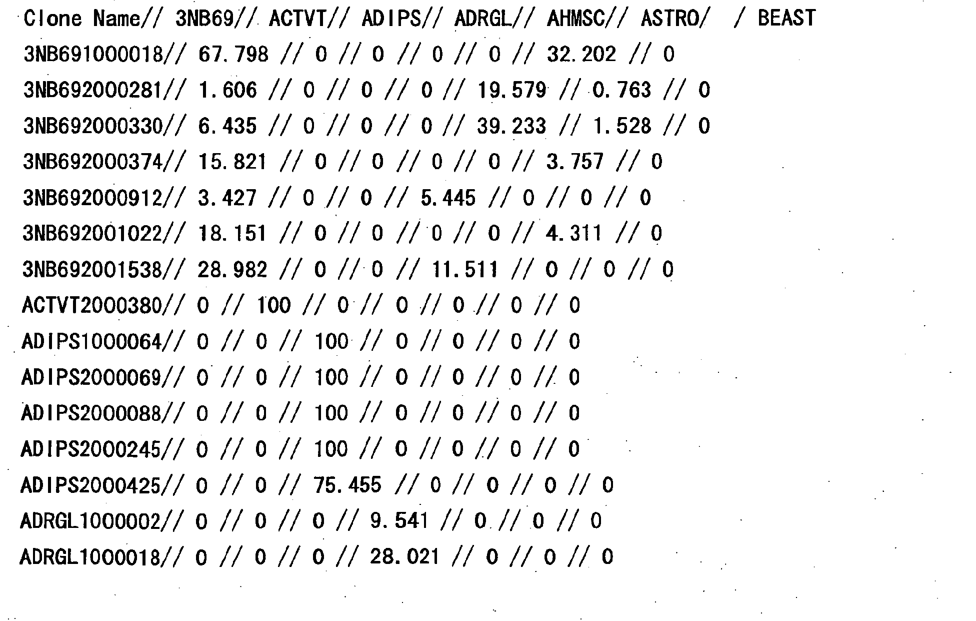

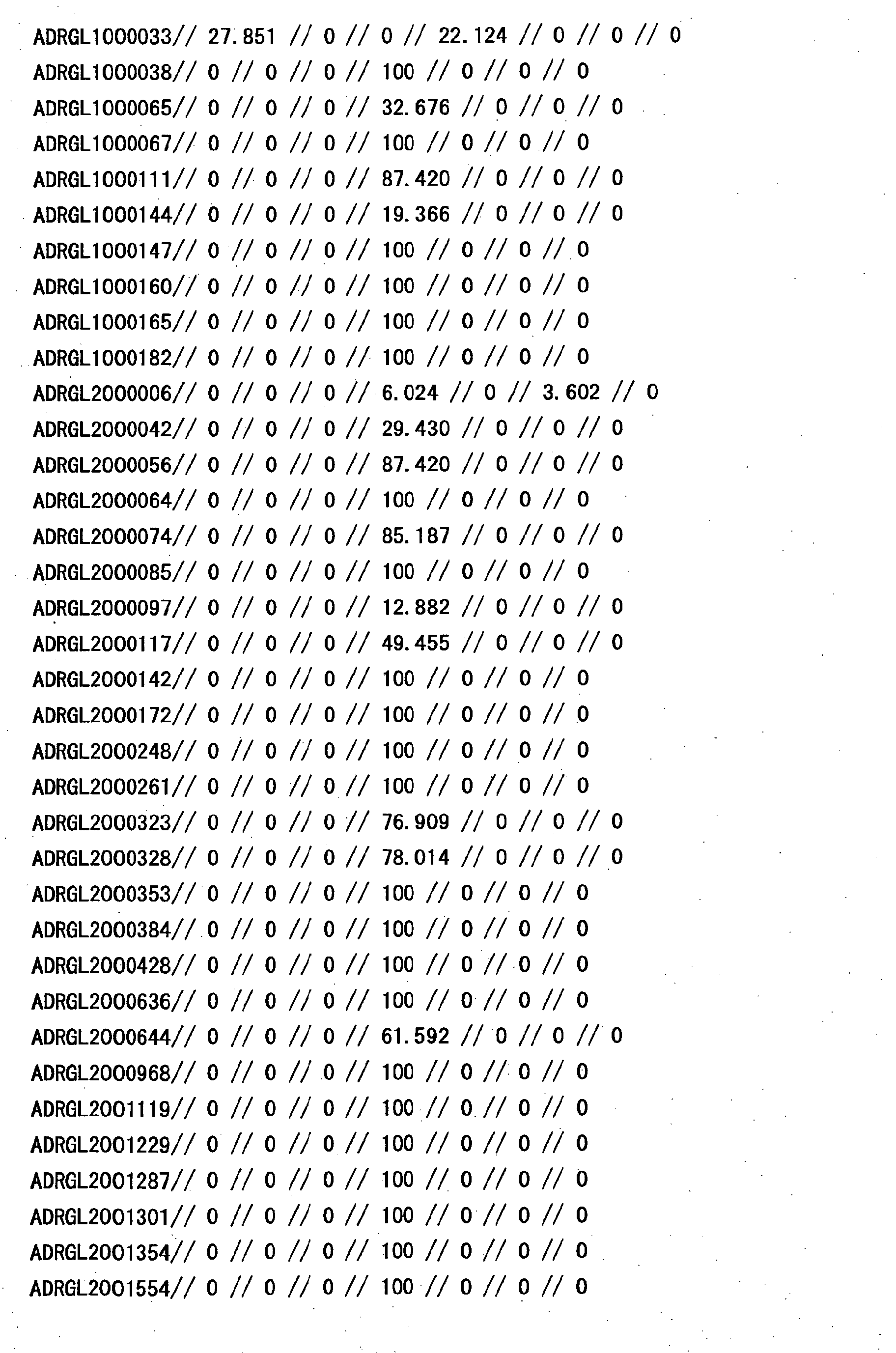

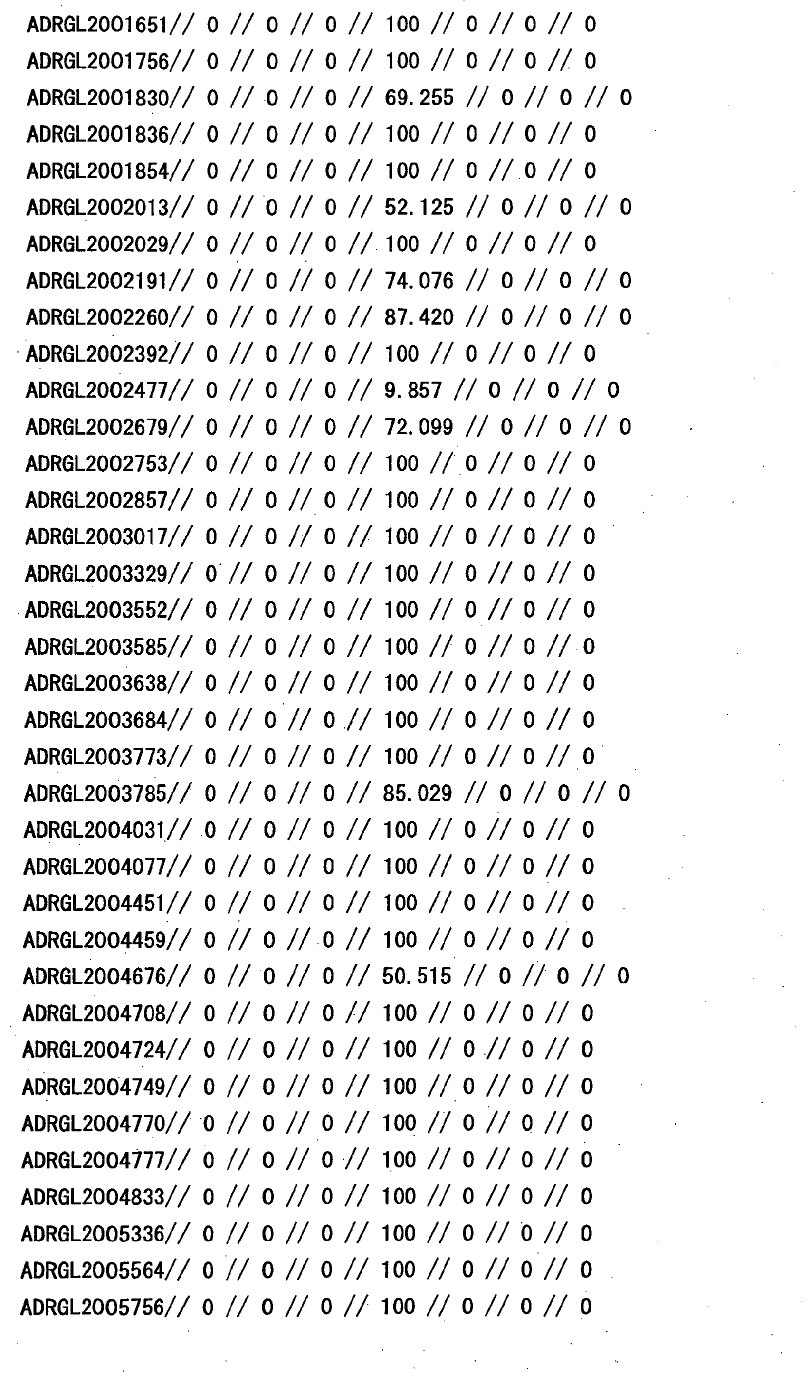

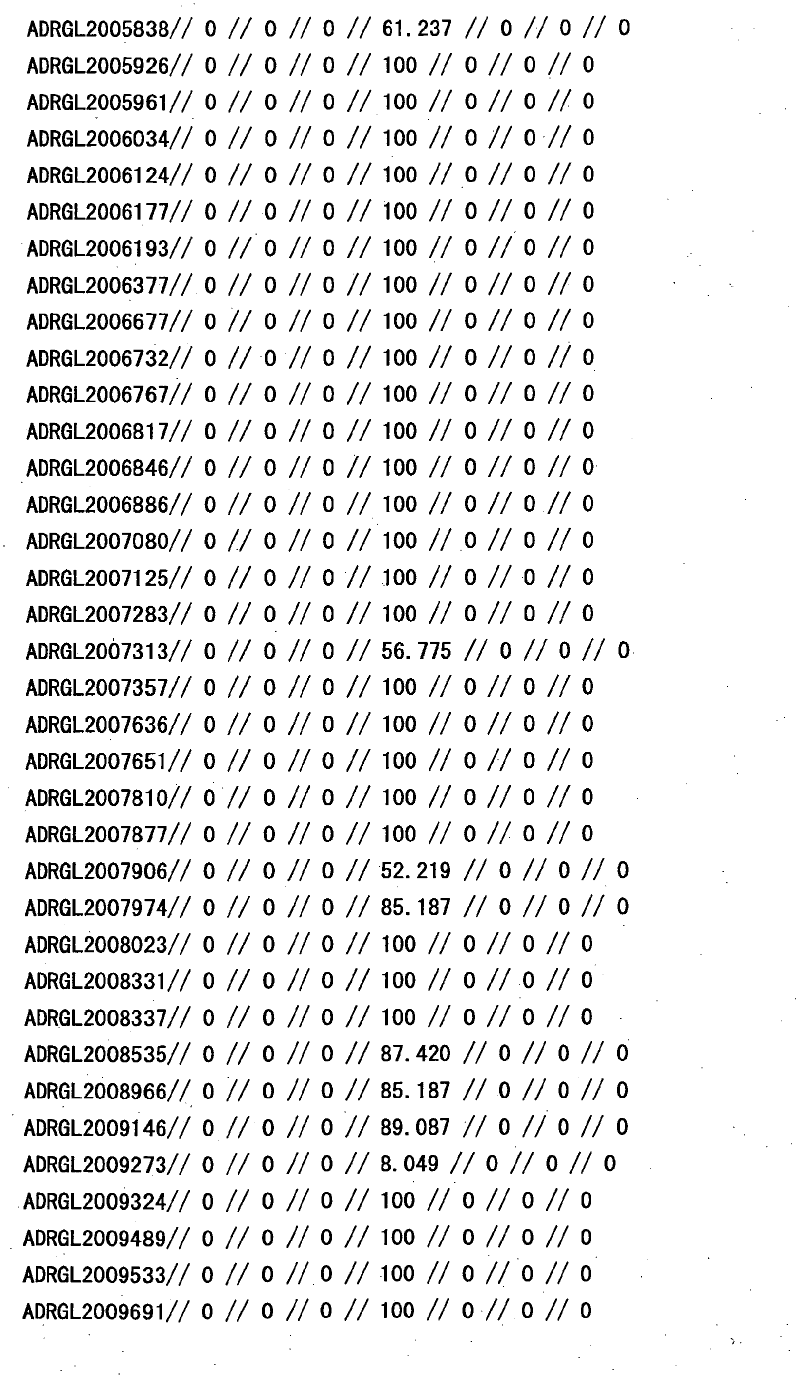

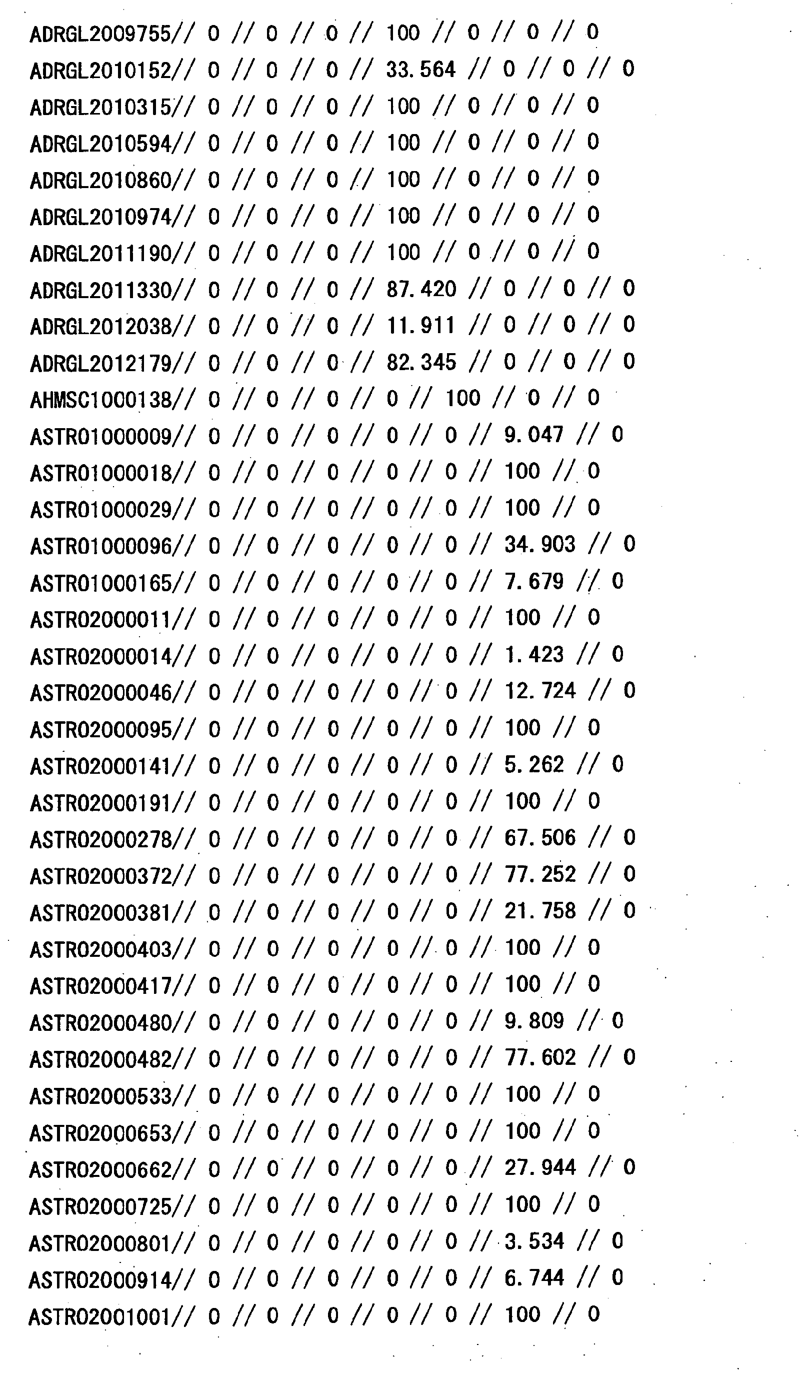

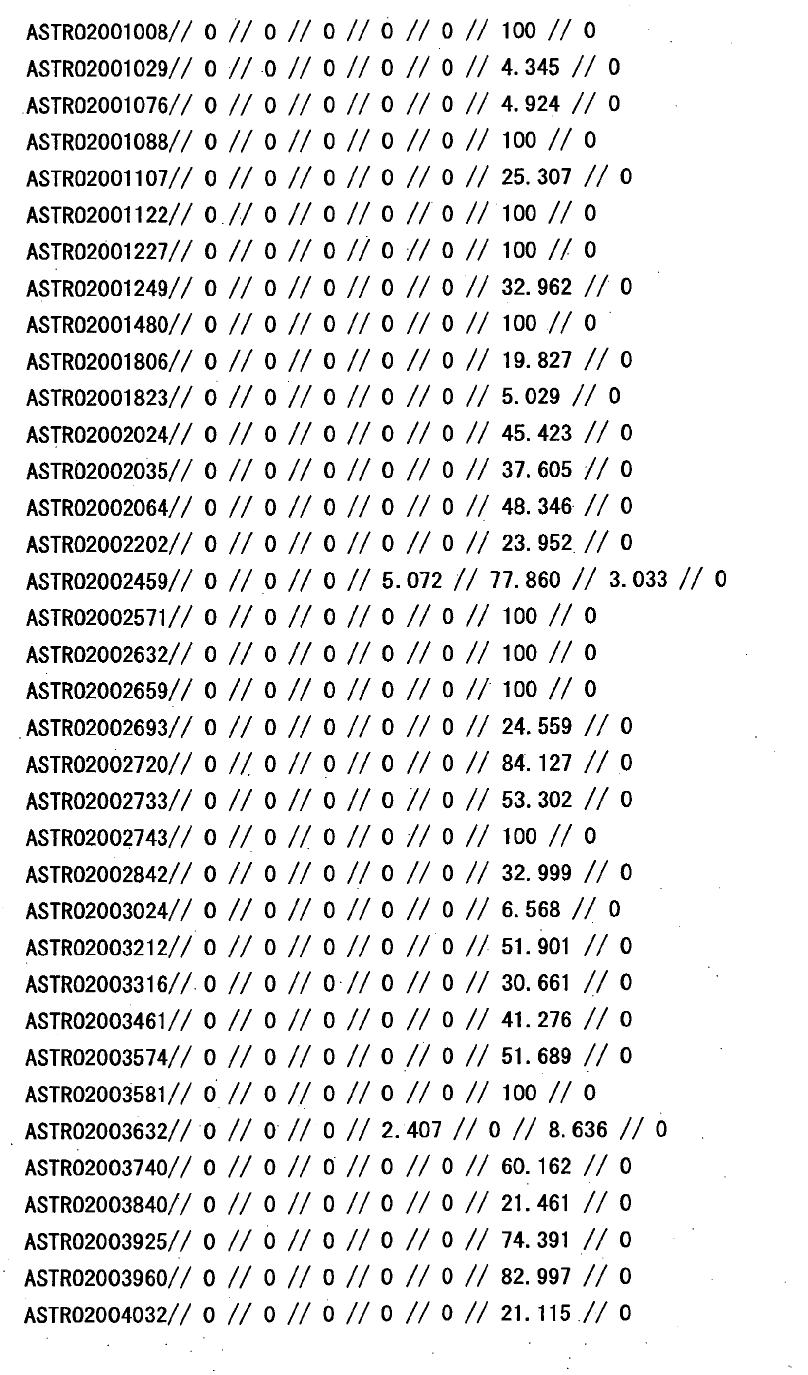

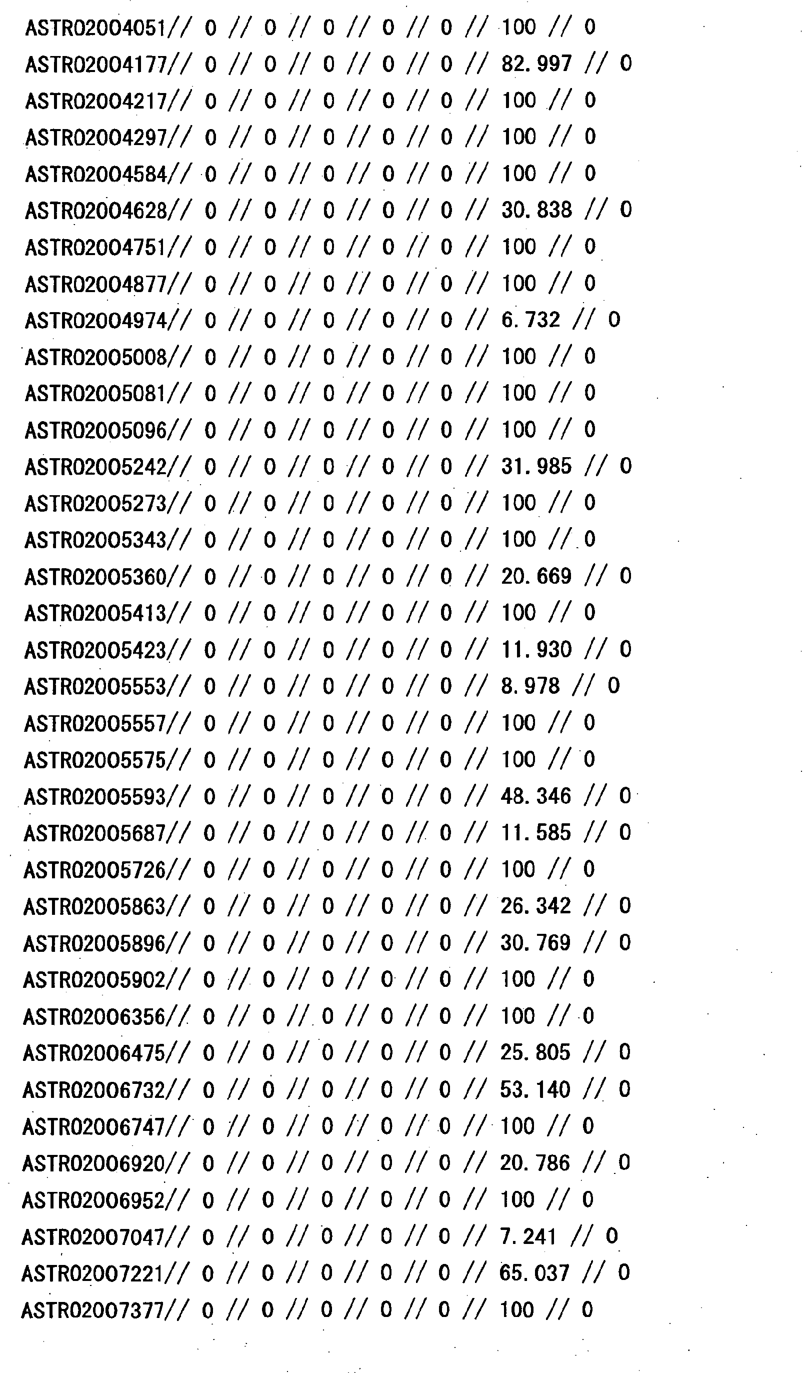

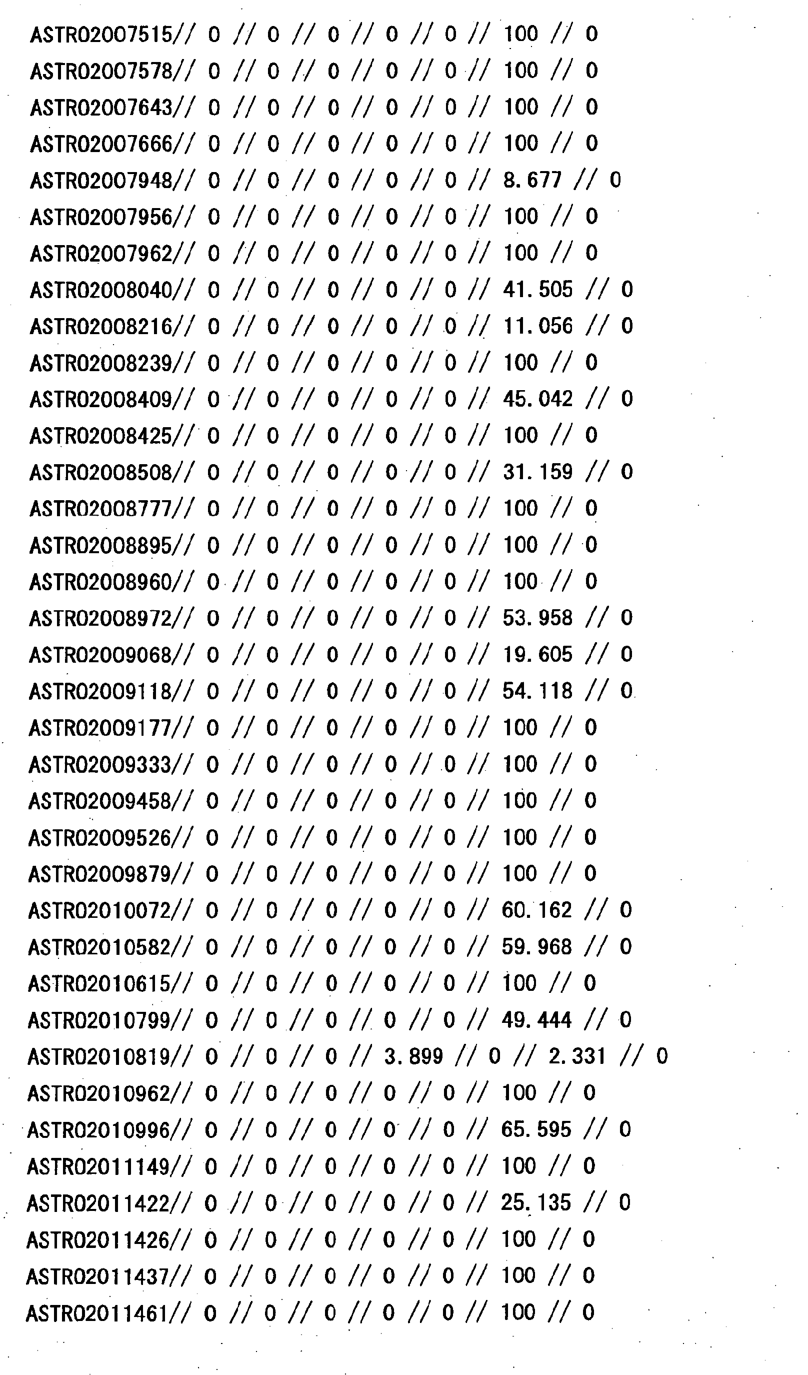

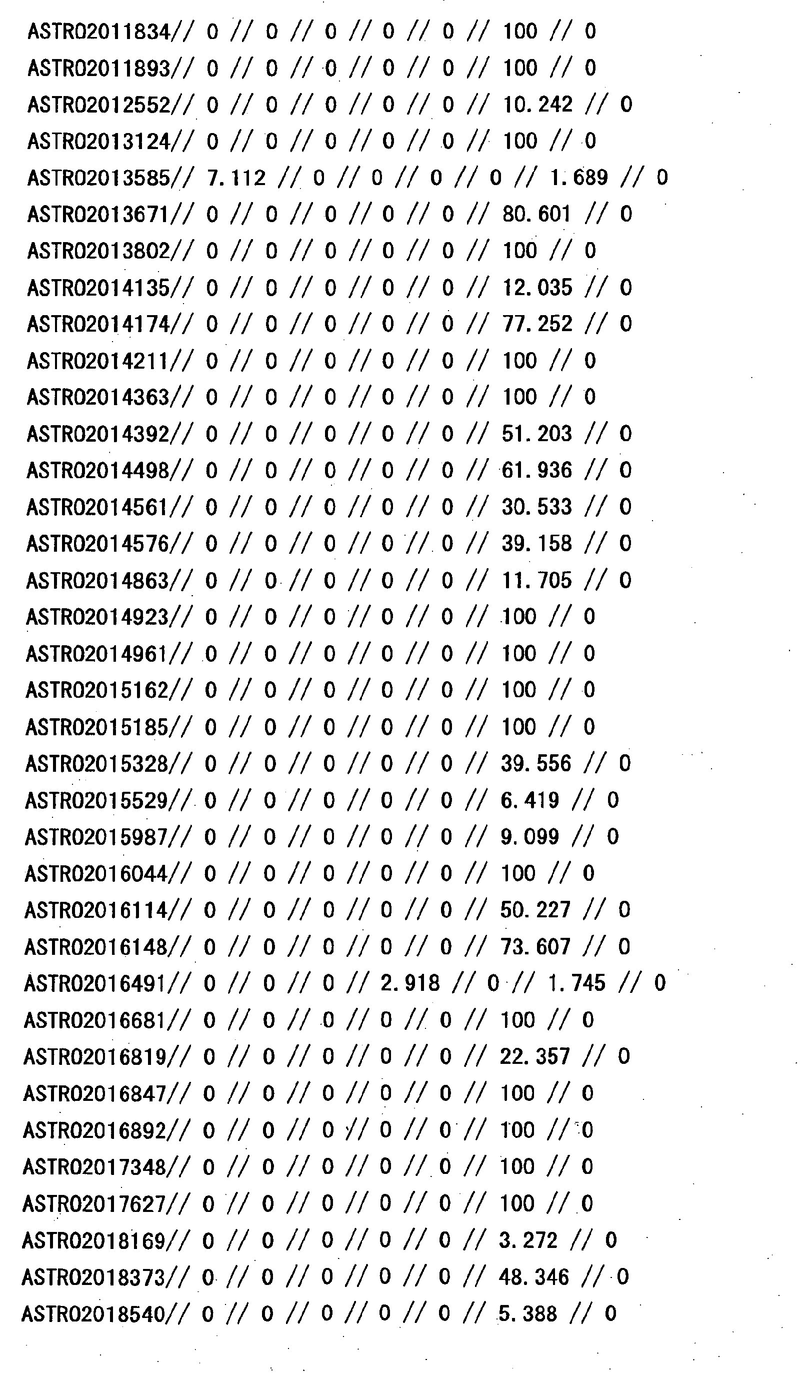

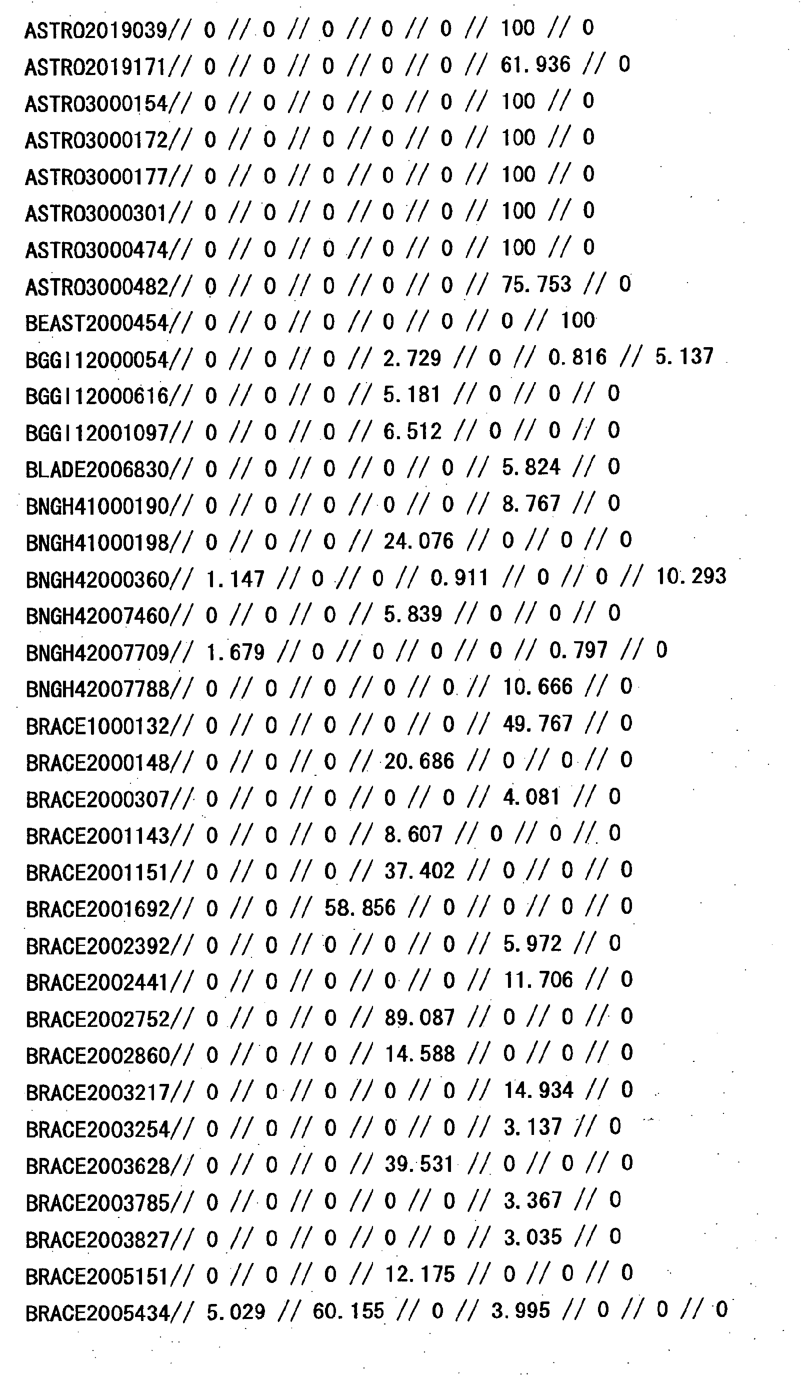

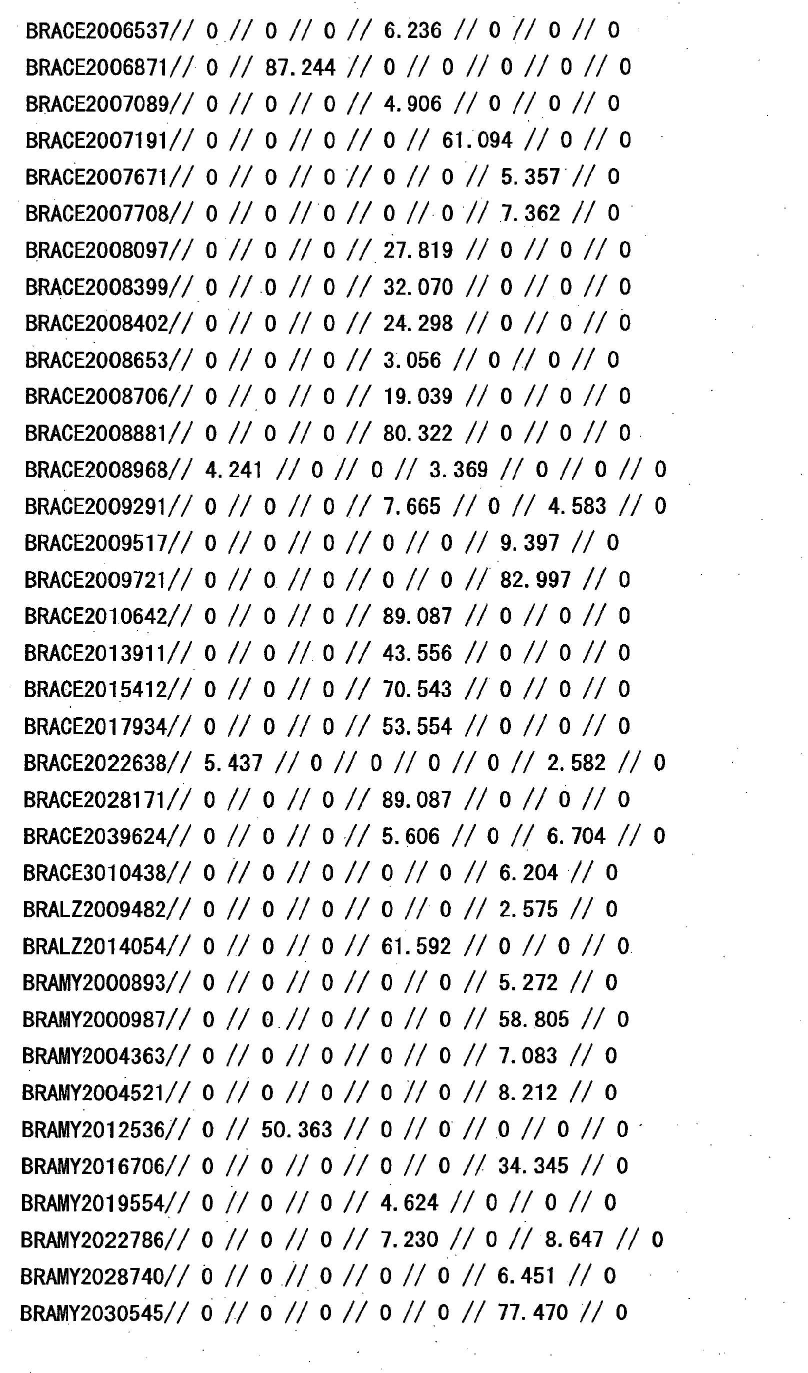

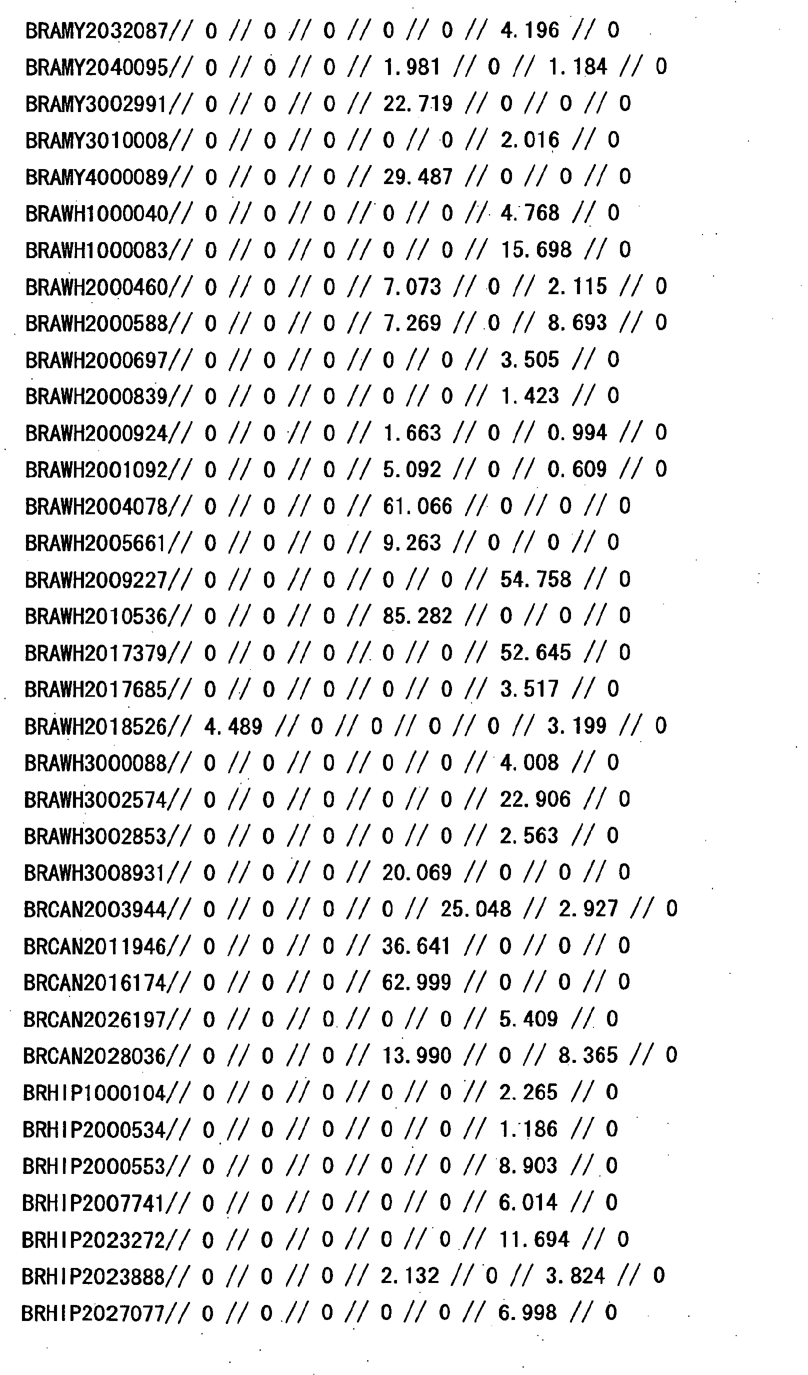

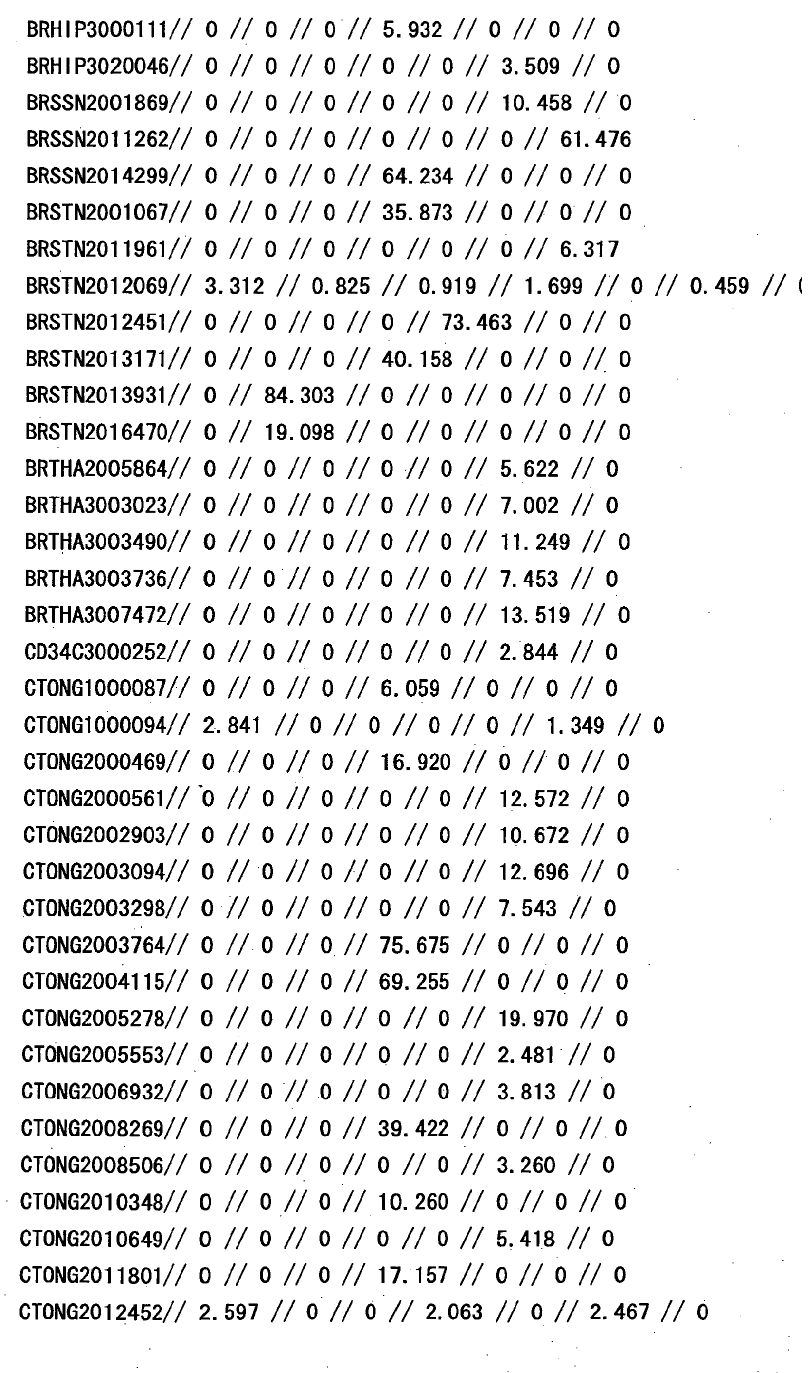

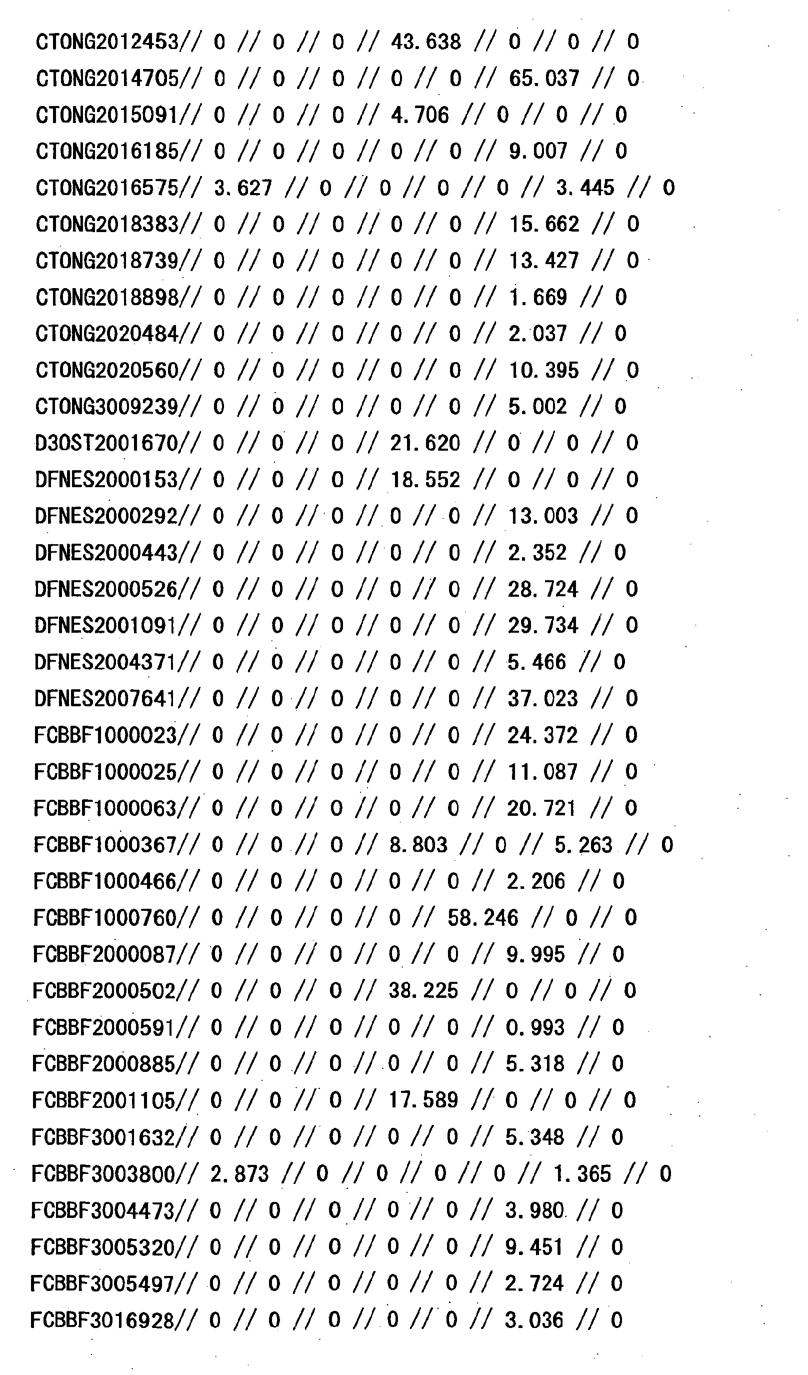

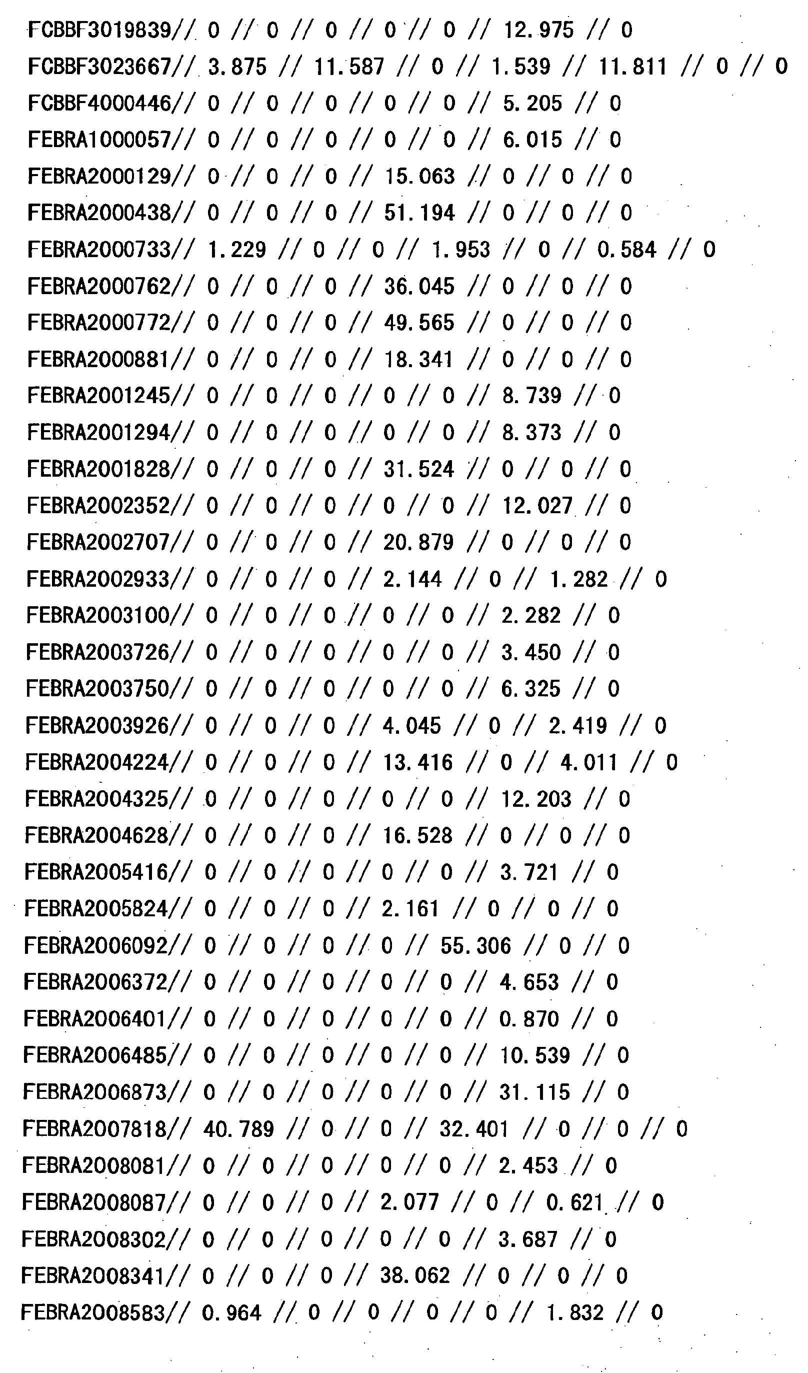

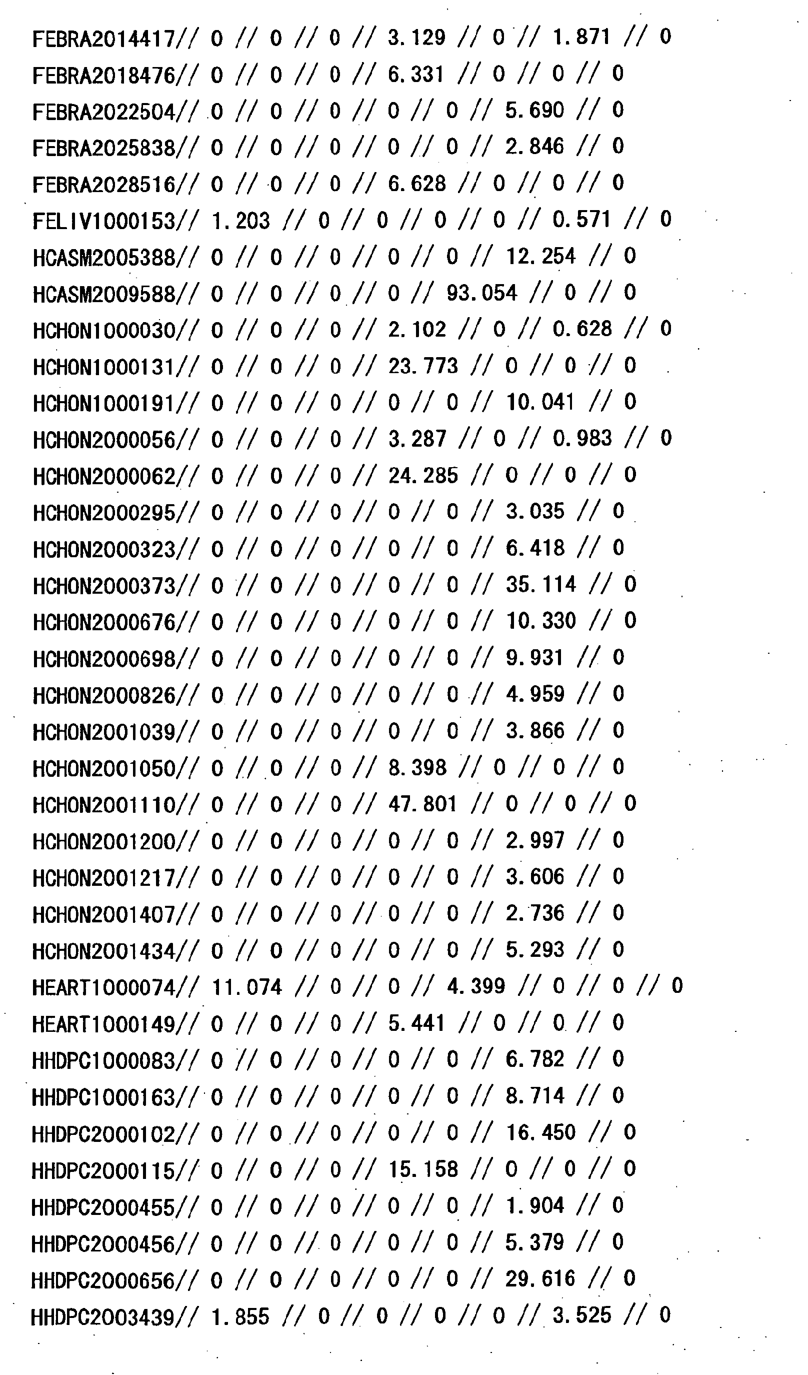

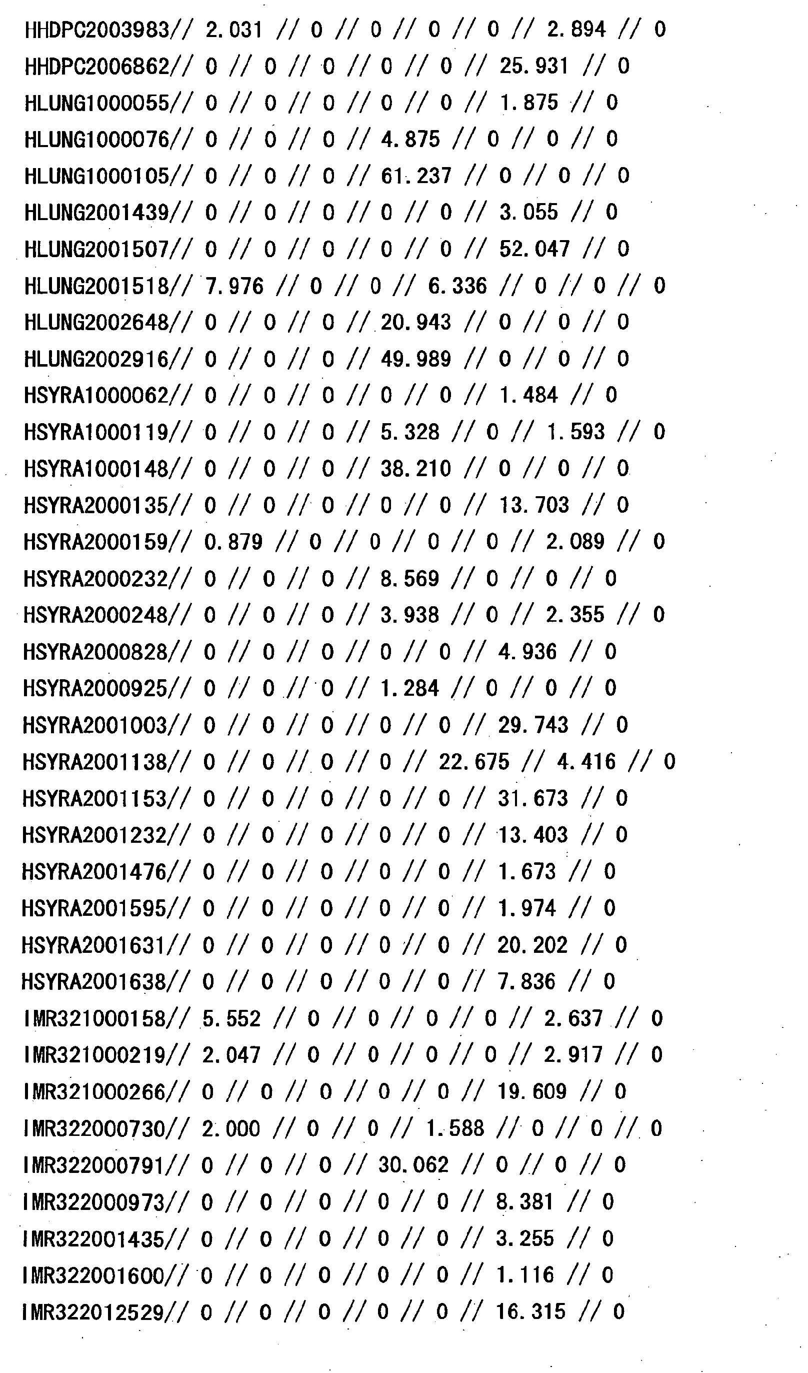

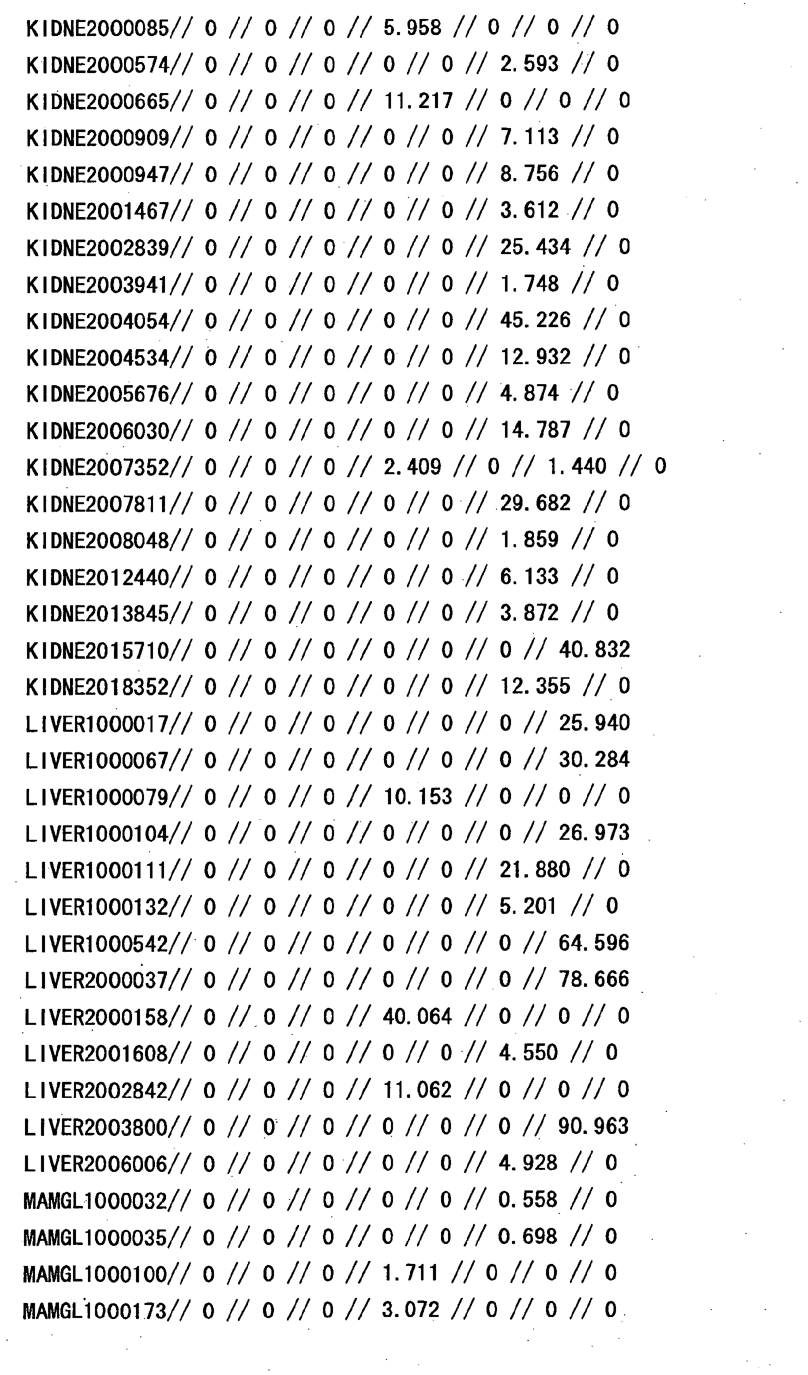

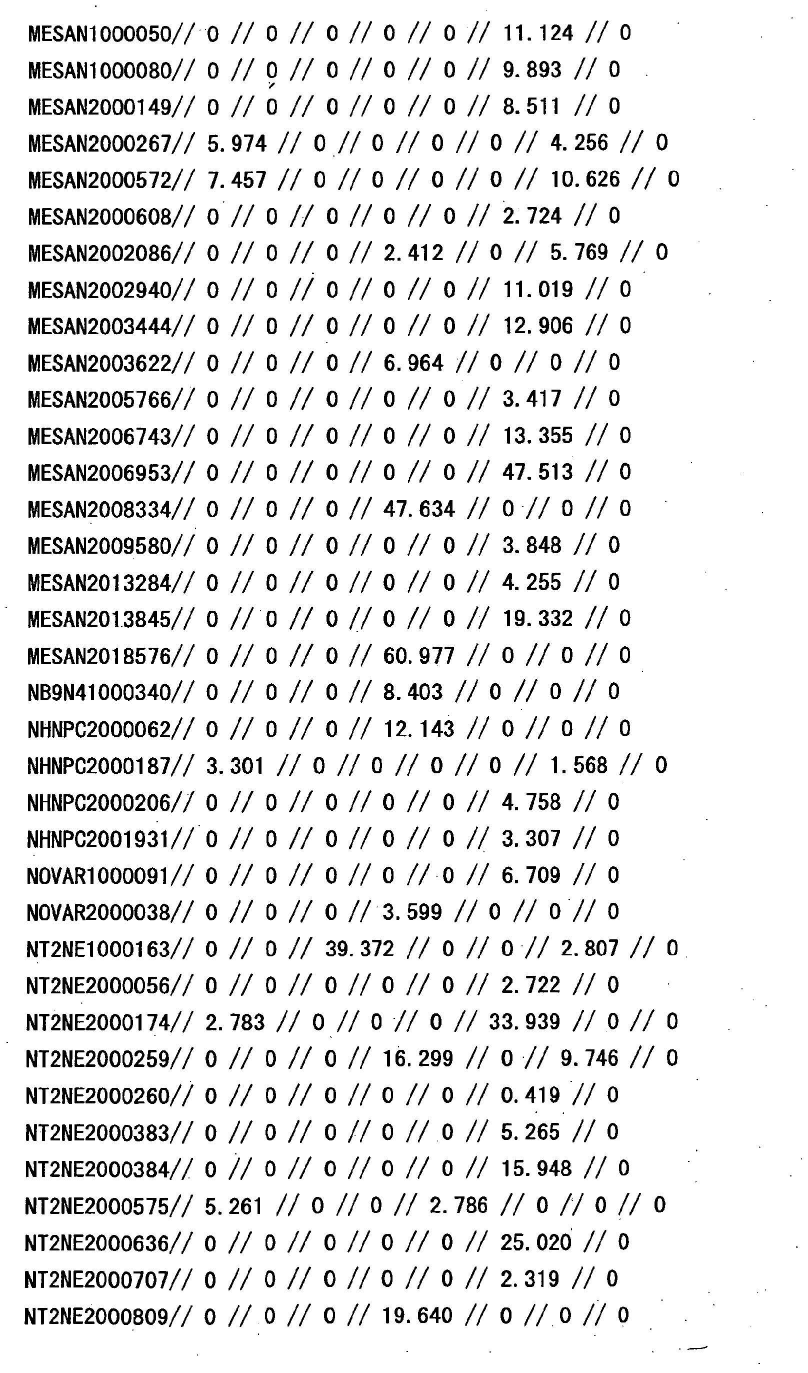

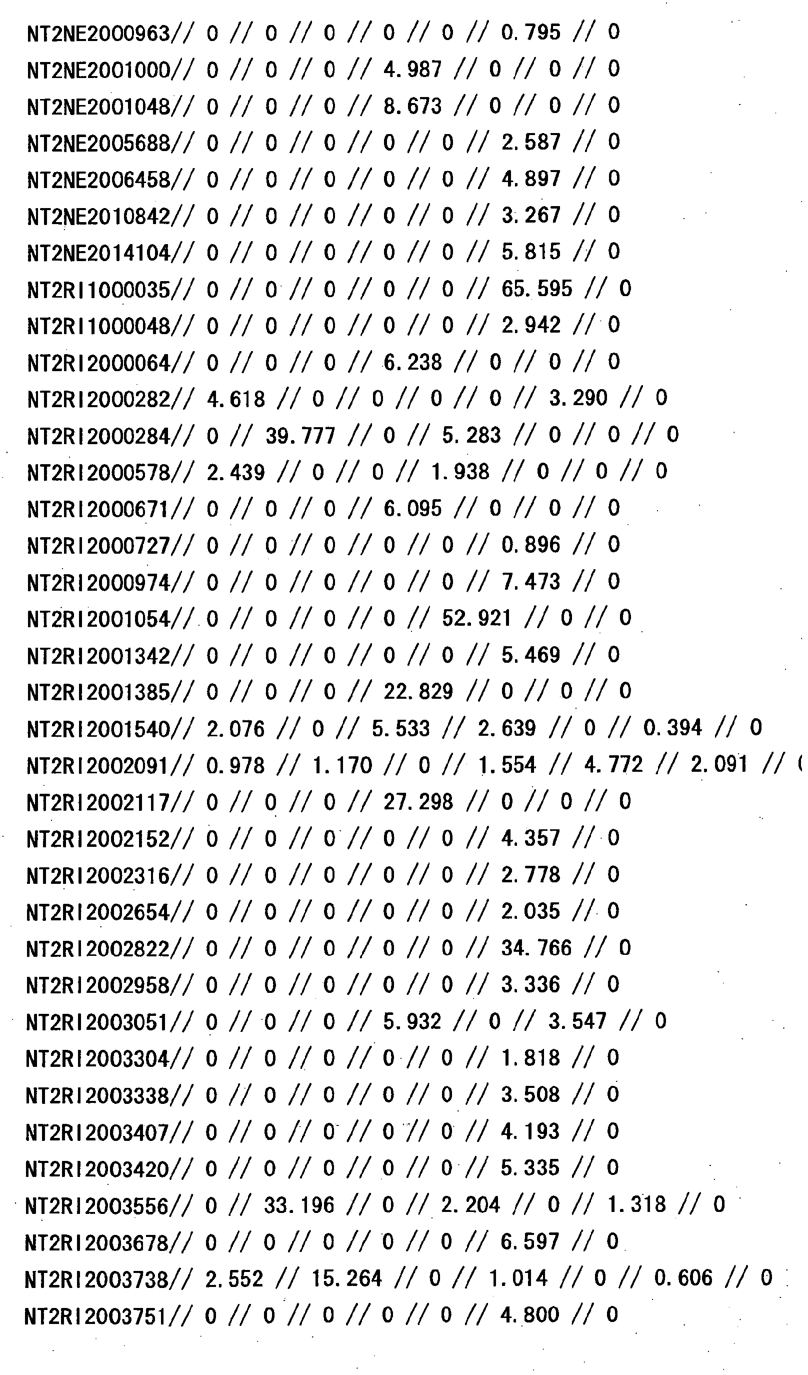

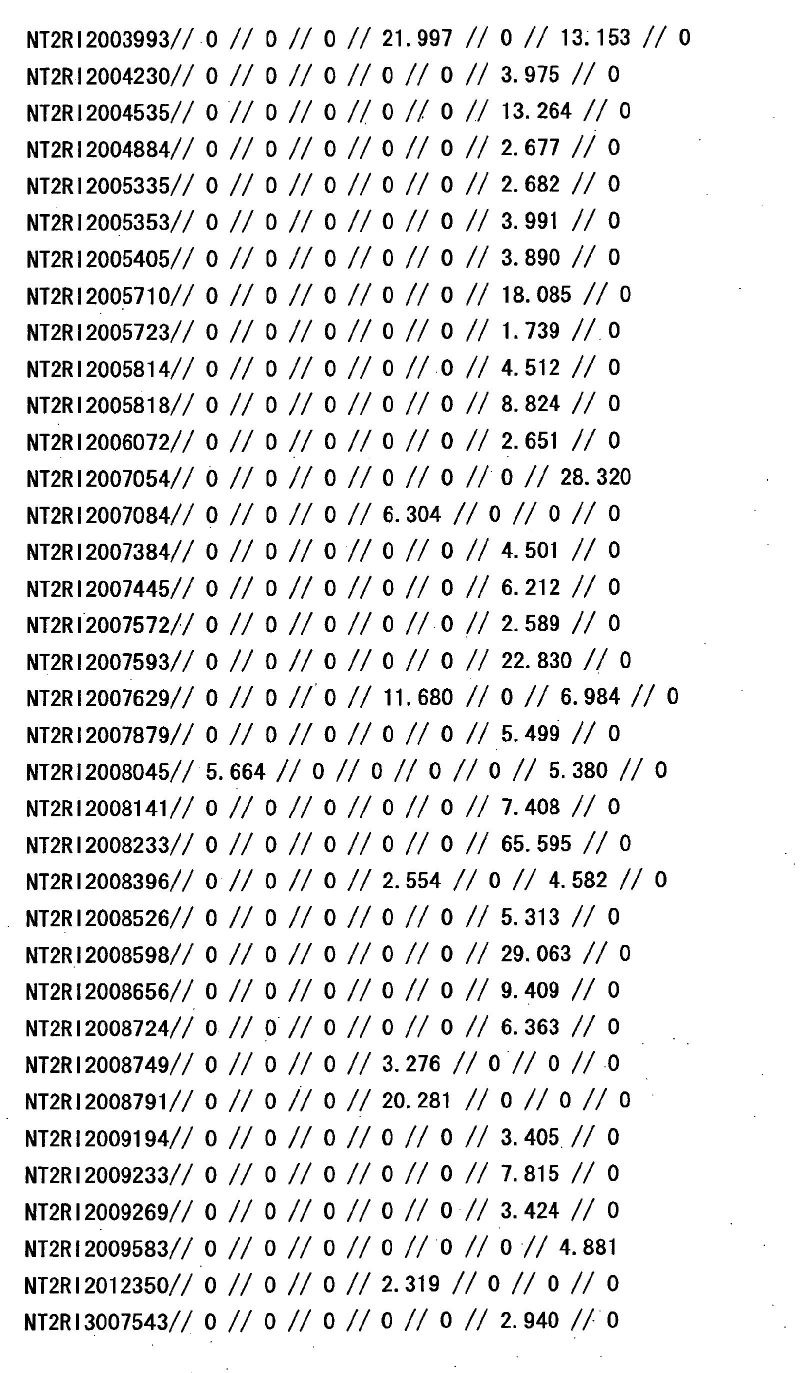

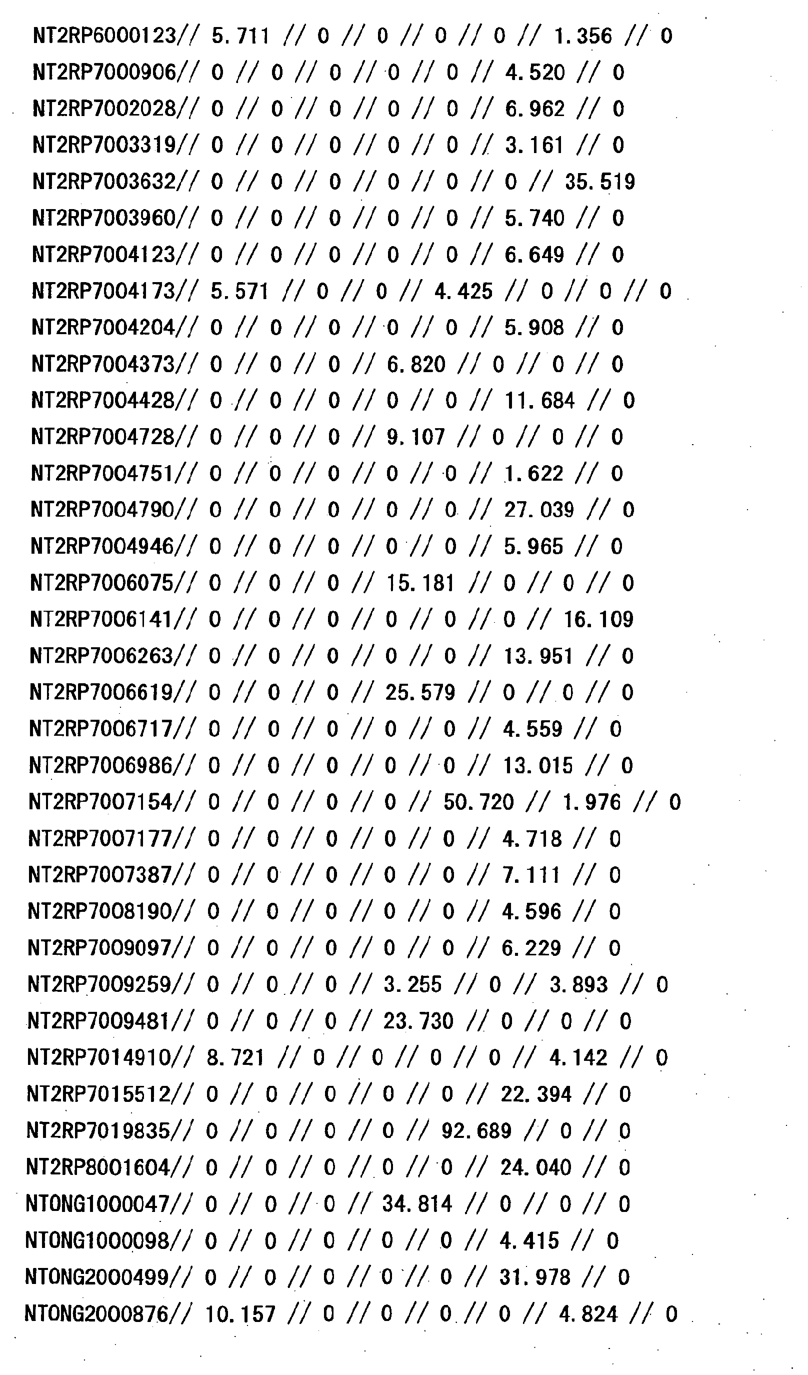

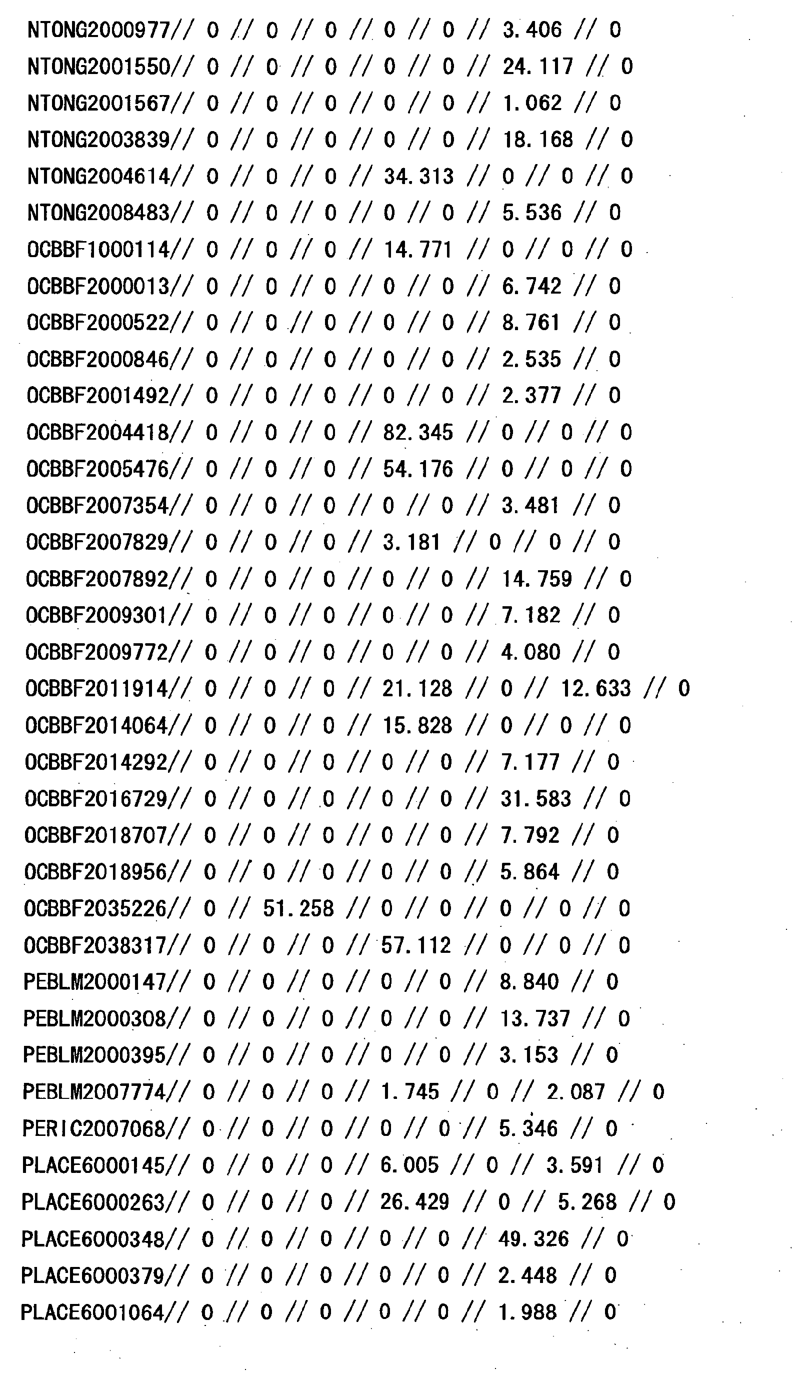

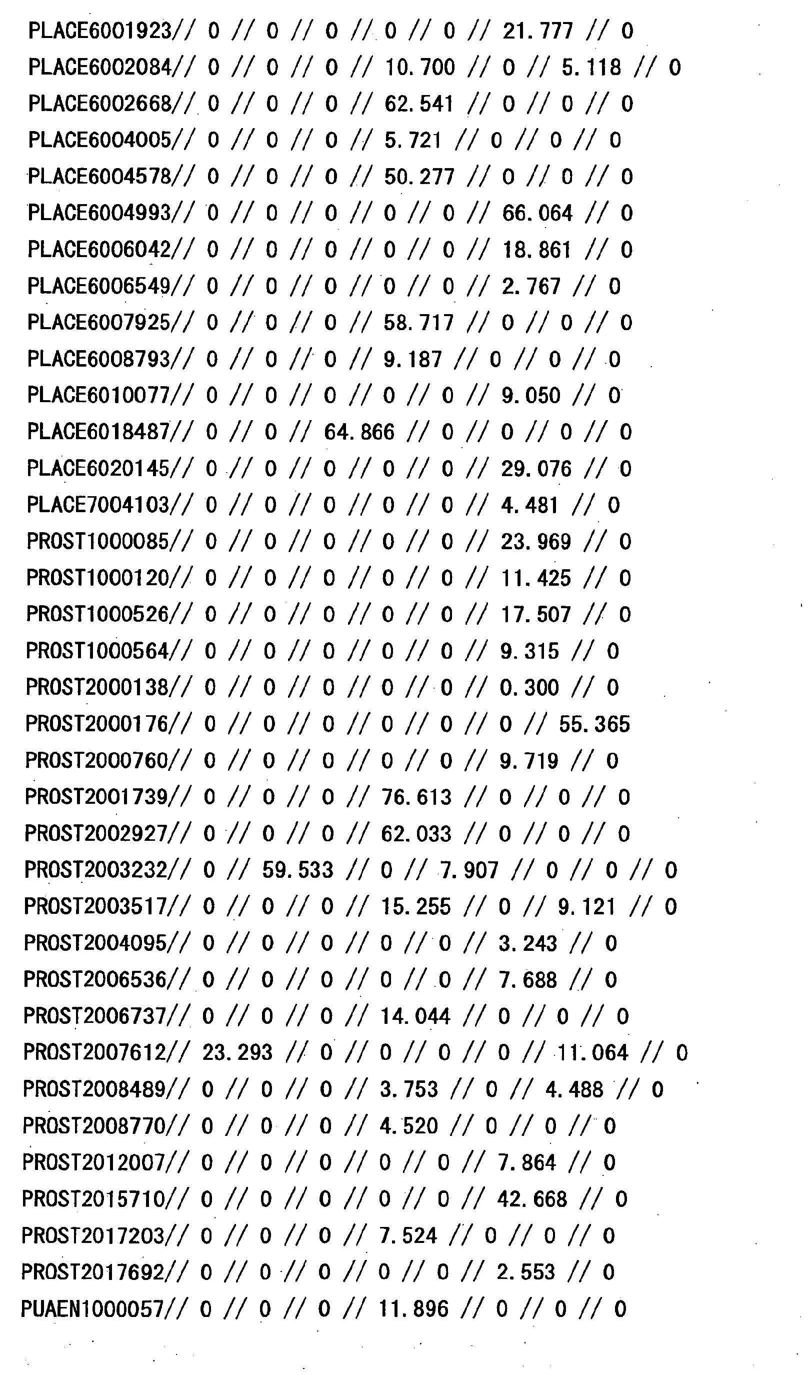

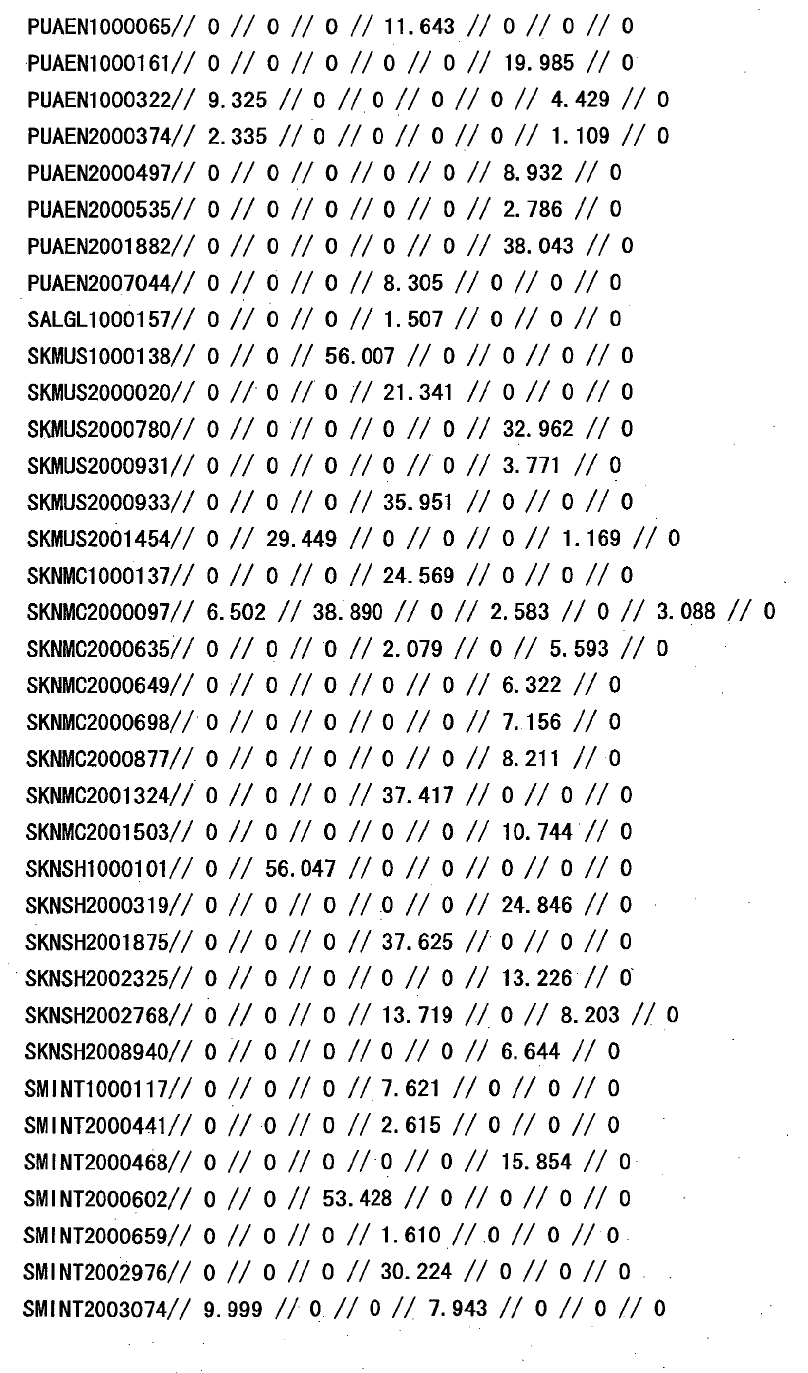

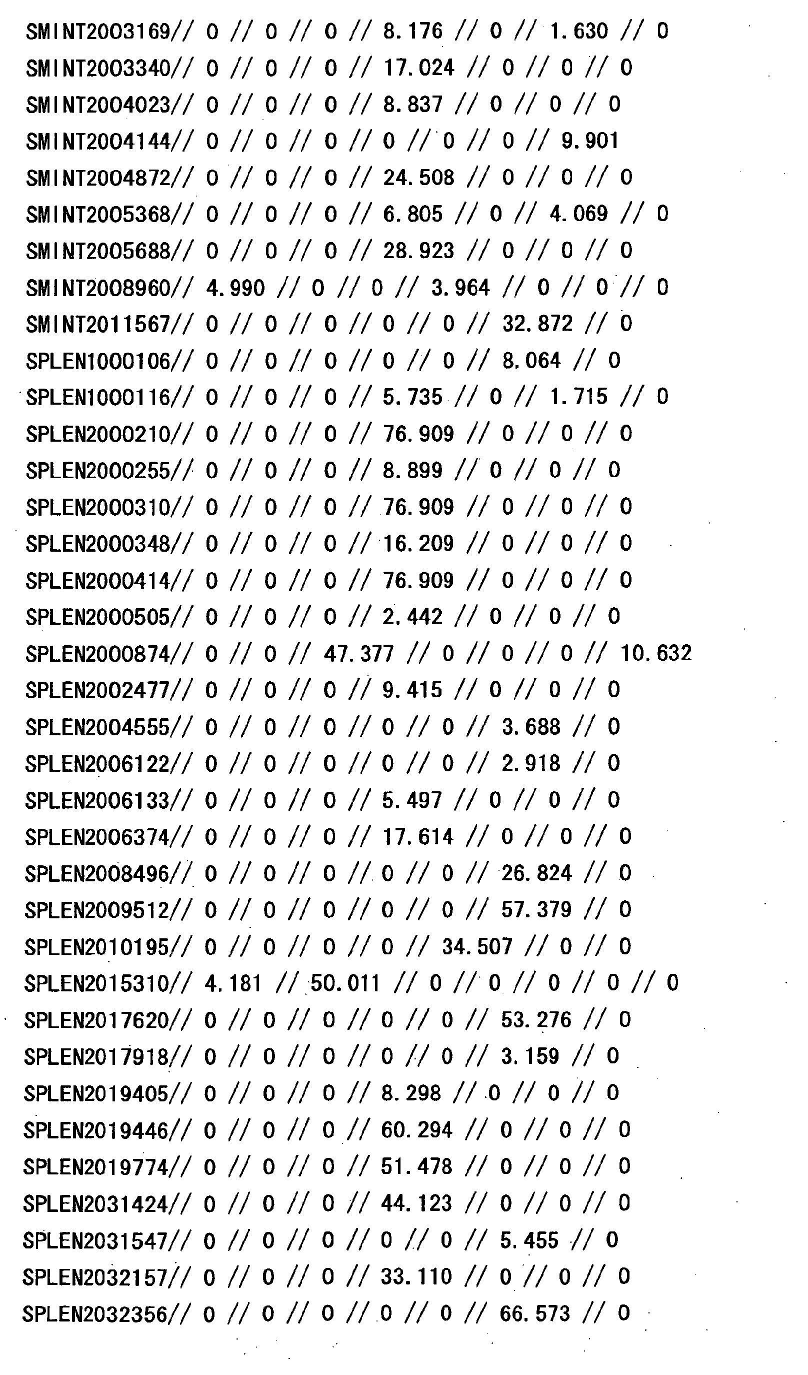

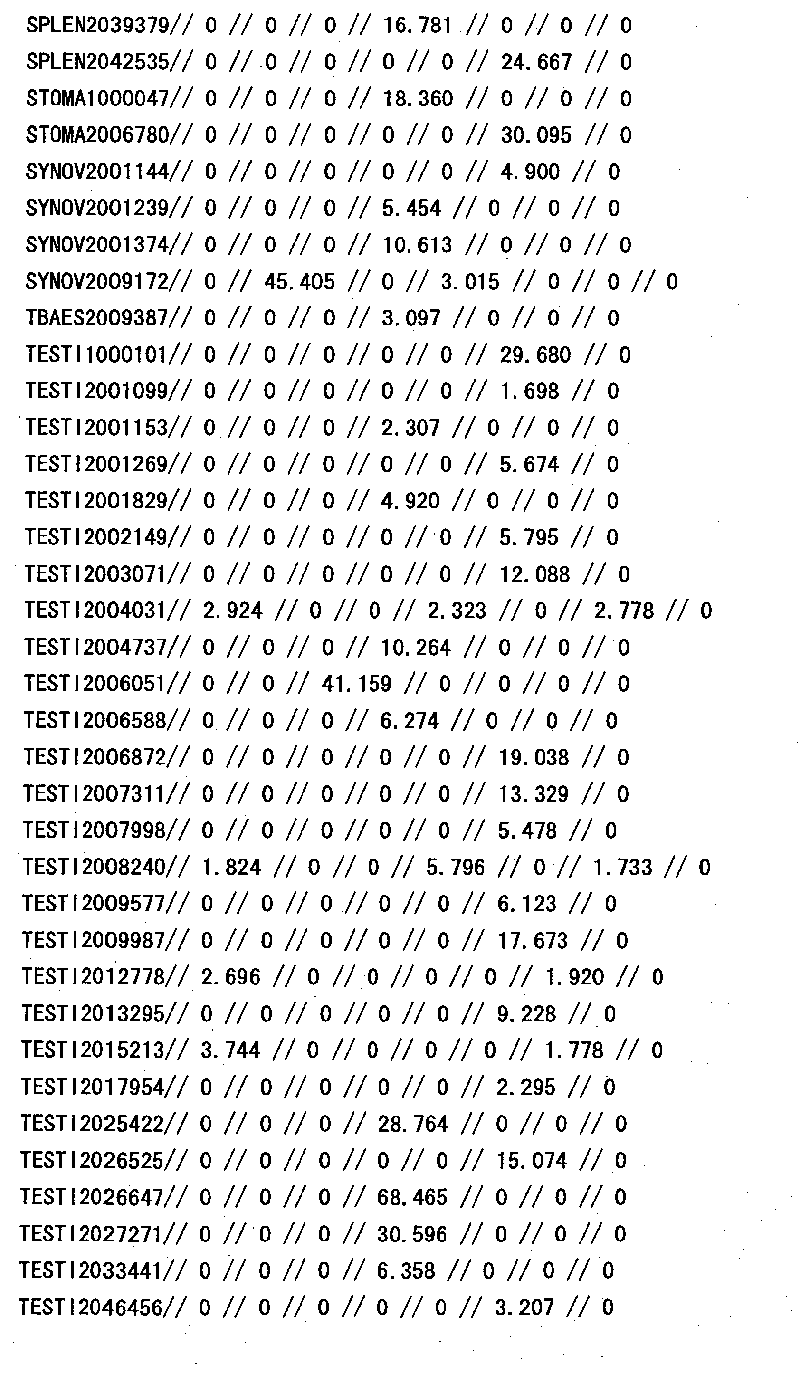

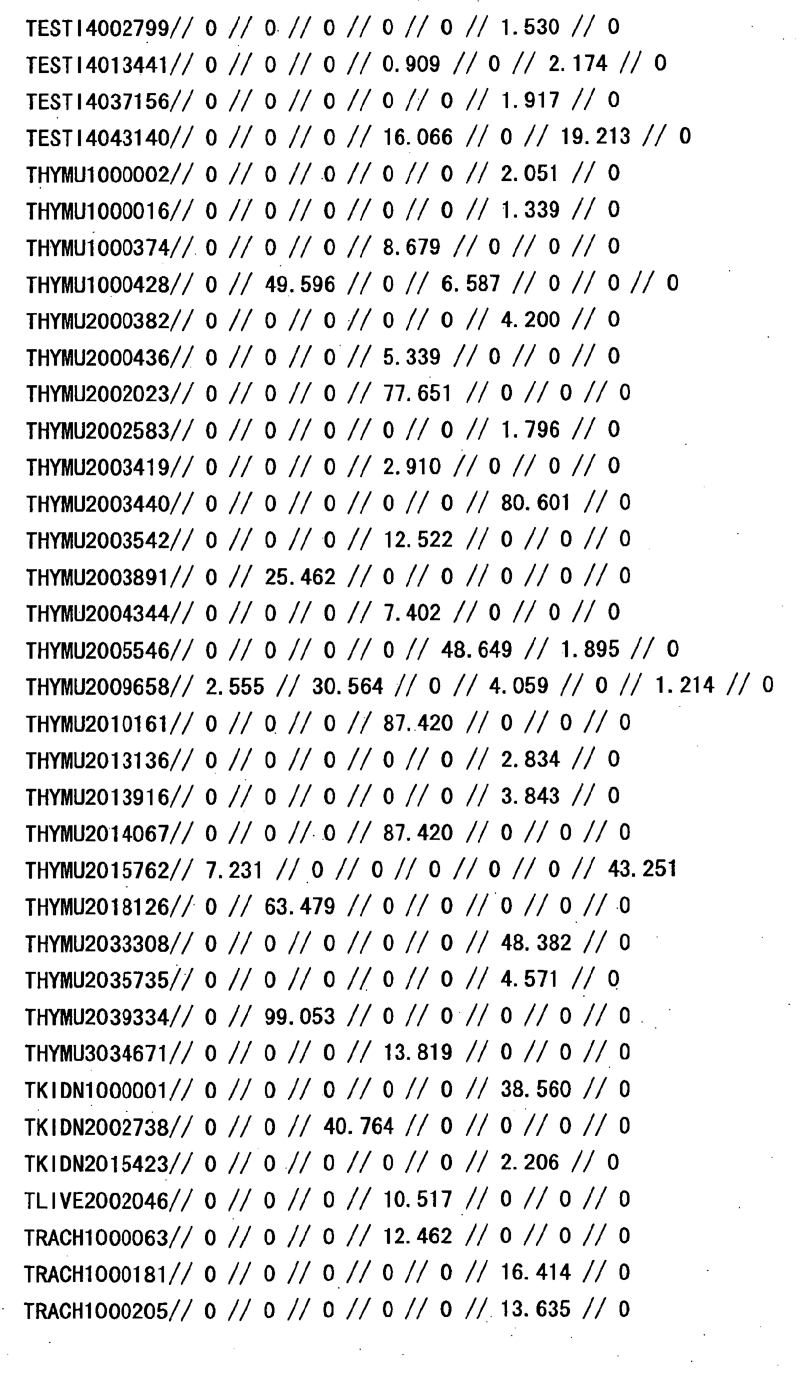

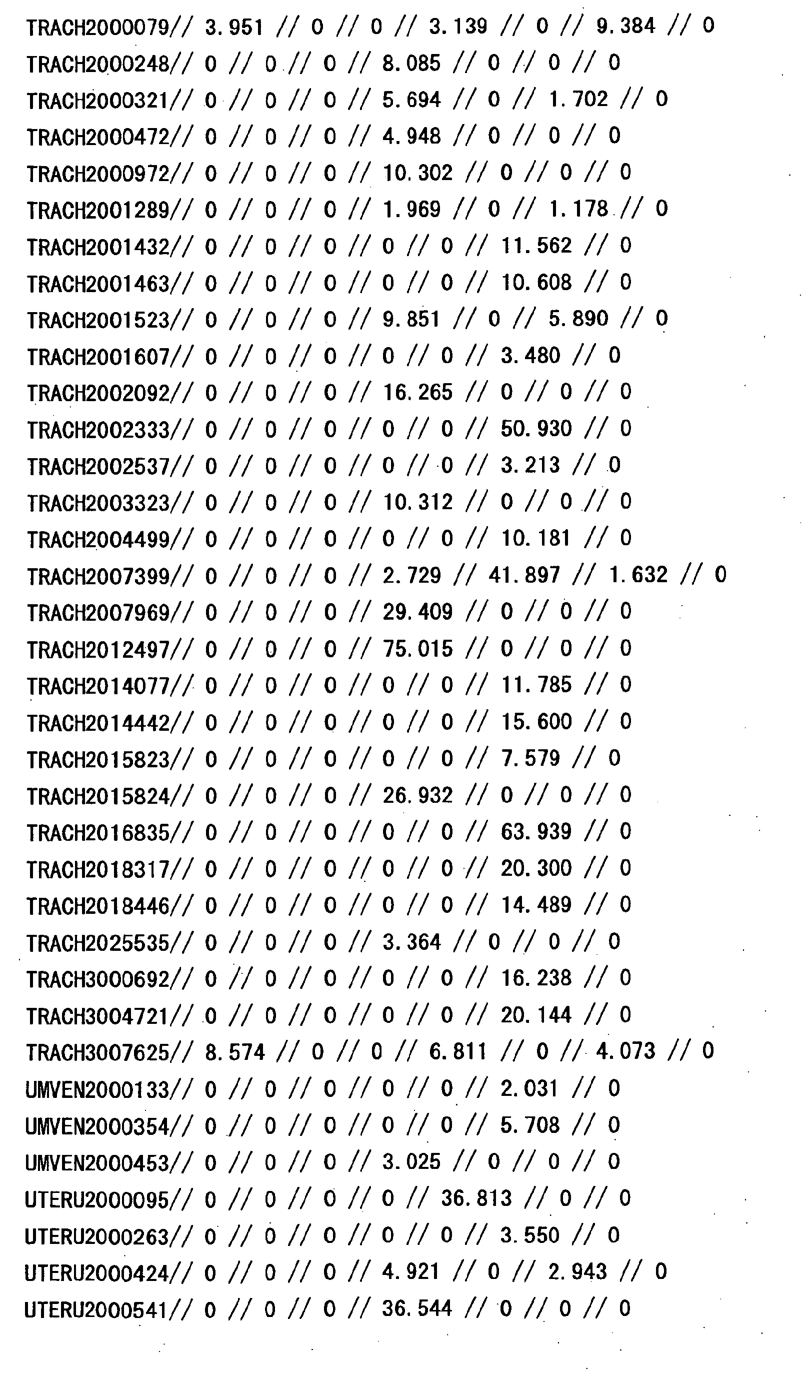

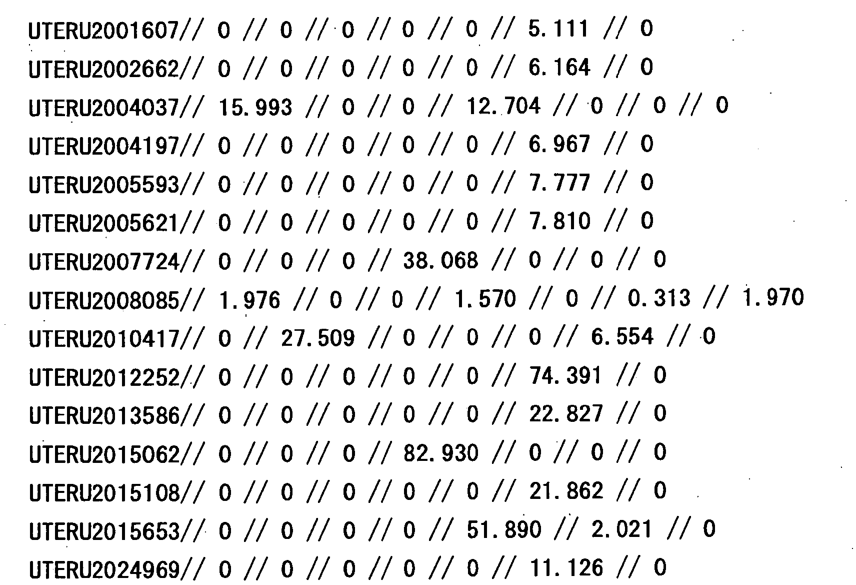

Genes whose expression levels were negligibly low in each of the above-described libraries are shown with their clone names below.
3NB691000085, 3NB691000113, 3NB691000116, 3NB691000129, 3NB691000173, 3NB691000191, 3NB692000012, 3NB692000029, 3NB692000102, 3NB692000154, 3NB692000276, 3NB692000305, 3NB692000429, 3NB692000484, 3NB692000529, 3NB692000545, 3NB692000973, 3NB692001002, 3NB692001034, 3NB692001040, 3NB692001123, 3NB692001267, 3NB692001288, 3NB692001334, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, BGGI11000123, BGGI11000193, BGGI11000285, BGGI12000022, BGGI12000067, BGGI12000161, BGGI12000533, BGGI12000544, BGGI12000684, BGGI12000693, BGGI12000839, BGGI12001002, BGGI12001075, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000153, BNGH41000169, BNGH41000177, BNGH41000216, BNGH41000235, BNGH42000130, BNGH42000132, BNGH42000405, BNGH42000532, BNGH42000675, BNGH42000739, BNGH42000791, BNGH42000815, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006135, BNGH42006226, BNGH42006234, BNGH42007037, BNGH42007366, BNGH42007594, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020, BRACE1000042, BRACE1000047, BRACE1000051, BRACE1000065, BRACE1000070, BRACE1000073, BRACE1000084, BRACE1000087, BRACE1000092, BRACE!000093, BRACE1000101, BRACE1000114, BRACE1000115, BRACE1000124, BRACE1000131, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000183, BRACE2000229, BRACE2000232, BRACE2000245, BRACE2000256, BRACE2000266, BRACE2000267, BRACE2000280, BRACE2000300, BRACE2000331, BRACE2000332, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972, BRACE2001973, BRACE2002026, BRACE2002034, BRACE2002050, BRACE2002081, BRACE2002091, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002682, BRACE2002685, BRACE2002695, BRACE2002707, BRACE2002736, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002861, BRACE2002884, BRACE2002896, BRACE2002948, BRACE2003037, BRACE2003078, BRACE2003097, BRACE2003110, BRACE2003168, BRACE2003172, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003639, BRACE2003700, BRACE2003713, BRACE2003766, BRACE2003788, BRACE2003800, BRACE2003802, BRACE2003825, BRACE2003845, BRACE2003846, BRACE2003847, BRACE2003848, BRACE2003873, BRACE2003885, BRACE2003887, BRACE2003892, BRACE2003904, BRACE2003905, BRACE2003928, BRACE2003944, BRACE2003954, BRACE2003982, BRACE2003987, BRACE2005034, BRACE2005047, BRACE2005063, BRACE2005083, BRACE2005087, BRACE2005090, BRACE2005099, BRACE2005118, BRACE2005124, BRACE2005138, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281, BRACE2006298, BRACE2006319, BRACE2006344, BRACE2006354, BRACE2006363, BRACE2006378, BRACE2006380, BRACE2006393, BRACE2006397, BRACE2006413, BRACE2006417, BRACE2006453, BRACE2006459, BRACE2006488, BRACE2006514, BRACE2006534, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006900, BRACE2006909, BRACE2006911, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007109, BRACE2007138, BRACE2007174, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007518, BRACE2007522, BRACE2007527, BRACE2007538, BRACE2007563, BRACE2007567, BRACE2007577, BRACE2007621, BRACE2007640, BRACE2007641, BRACE2007646, BRACE2007663, BRACE2007682, BRACE2007685, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008385, BRACE2008401, BRACE2008410, BRACE2008420, BRACE2008443, BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008708, BRACE2008710, BRACE2008754, BRACE2008797, BRACE2008847, BRACE2008857, BRACE2008861, BRACE2008867, BRACE2008883, BRACE,2008917, BRACE2008919, BRACE2008941, BRACE2008948, BRACE2008960, BRACE2008999, BRACE2009014, BRACE2009016, BRACE2009031, BRACE2009037, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587, BRACE2017720, BRACE2017844, BRACE2017872, BRACE2017929, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, ) BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE 2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217,. BRACE3005225, BRACE3005430, BRACE3005499, BRACE3005760, BRACE3005870, BRACE3005903, BRACE3005938, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010428, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, 5 BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728; BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000585, BRAMY2000653, BRAMY2000814, BRAMY2000946, BRAMY2000981, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2019989, BRAMY2020050, BRAMY2020058, BRAMY2020106, BRAMY2020270, BRAMY2020354, BRAMY2020355, BRAMY2020412, BRAMY2020427, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347; BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3003026, BRAMY3003109, BRAMY3003205, BRAMY3003310, BRAMY3003566, BRAMY3003723, BRAMY3004126, BRAMY3004224, BRAMY3004364, BRAMY3004377, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000037, BRAWH1000045, BRAWH1000093, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000471, BRAWH2000476, BRAWH2000482, BRAWH2000488, BRAWH2000494, BRAWH2000500, BRAWH2000503, BRAWH2000522, BRAWH2000554, BRAWH2000595, BRAWH2000633, BRAWH2000636, BRAWH2000643, BRAWH2000651, BRAWH2000682, BRAWH2000686, BRAWH2000752, BRAWH2000859, BRAWH2000866, BRAWH2000892, BRAWH2000901, BRAWH2000926, BRAWH2000944, BRAWH2000984, BRAWH2001019, BRAWH2001037, BRAWH2001052, BRAWH2001103, BRAWH2001129, BRAWH2001137, BRAWH2001141, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406, BRAWH2001412, BRAWH2001418, BRAWH2001424, BRAWH2001436, BRAWH2001438, BRAWH2001439, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025, BRAWH2003135, BRAWH2003240, BRAWH2003307, BRAWH2003355, BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380, BRAWH2008540, BRAWH2008577, BRAWH2008706, BRAWH2008790, BRAWH2008903, BRAWH2008956, BRAWH2008993, BRAWH2009112, BRAWH2009238, BRAWH2009261, BRAWH2009263, BRAWH2009298, BRAWH2009304, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014188, BRAWH2014234, BRAWH2014379, BRAWH2014414, BRAWH2014473, BRAWH2014511, BRAWH2014645, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017407, BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048; BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007305, BRHIP2007307, BRHIP2007616, BRHIP2007690, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022228, BRHIP2022298, BRHIP2022326, BRHIP2022467, BRHIP2022708, BRHIP2022986, BRHIP2023071, BRHIP2023229, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014424, BRSSN2014513, BRSSN2014556, BRSSN2014573, BRSSN2014610, BRSSN2014636, BRSSN2014685, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070, BRSTN2000220, BRSTN2000312, BRSTN2000367, BRSTN2000536, BRSTN2000872, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012174, BRSTN2012284, BRSTN2012320, BRSTN2012380, BRSTN2013354, BRSTN2013502, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016892, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003074, BRTHA3003225, BRTHA3003339, BRTHA3003343, BRTHA3003417, BRTHA3003449, BRTHA3003474, BRTHA3003704, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000052, CTONG1000056, CTONG1000062, CTONG1000086, CTONG1000088, CTONG1000093, CTONG1000097, CTONG1000113, CTONG1000137, CTONG1000165, CTONG1000175, CTONG1000180, CTONG1000181, CTONG1000218, CTONG1000220, CTONG1000241, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000411, CTONG2000452, CTONG2000465, CTONG2000480, CTONG2000508, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001932, CTONG2001955, CTONG2002066, CTONG2002073, CTONG2002095, CTONG2002143, CTONG2002270, CTONG2002395, CTONG2002417, CTONG2002418, CTONG2002453, CTONG2002520, CTONG2002558, CTONG2002590, CTONG2002709, CTONG2002721, CTONG2002744, CTONG2002766, CTONG2002803, CTONG2002816, CTONG2002820, CTONG2002832, CTONG2002841, CTONG2002965, CTONG2002999, CTONG2003028, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003524, CTONG2003599, CTONG2003680, CTONG2003699, CTONG2003782, CTONG2003889, CTONG2003937, CTONG2003945, CTONG2004000, CTONG2004062, CTONG2004126, CTONG2004264, CTONG2004305, CTONG2004397, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005290, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2005913, CTONG2006004, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006562, CTONG2006568, CTONG2006611, CTONG2006620, CTONG2006666, CTONG2006691, CTONG2006709, CTONG2006798, CTONG2006836, CTONG2006942, CTONG2006964, CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007500, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008321, CTONG2008343, CTONG2008398, CTONG2008402, CTONG2008405, CTONG2008466, CTONG2008518, CTONG200852-1, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010408, CTONG2010508, CTONG2010566, CTONG2010623, CTONG2010652, CTONG2010803, CTONG2010821, CTONG2011188, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012158, CTONG2012230, CTONG2012401, CTONG2012422, CTONG2012425, CTONG2012447, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012847, CTONG2012879, CTONG2012901, CTONG2012996, CTONG2013128, CTONG2013149, CTONG2013156, CTONG2013178, CTONG2013222, CTONG2013284, CTONG2013339, CTONG2013348, CTONG2013352, CTONG2013381, CTONG2013393, CTONG2013511, CTONG2013630, CTONG2013803, CTONG2013907, CTONG2013934, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016056, CTONG2016217, CTONG2016355, CTONG2016408, CTONG2016499, CTONG2016505, CTONG2016559, CTONG2016658, CTONG2016775, CTONG2016797, CTONG2016824, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017500, CTONG2017604, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018343, CTONG2018379, CTONG2018413, CTONG2018483, CTONG2018629, CTONG2018632, CTONG2018637, CTONG2018652, CTONG2018653, CTONG2018655, CTONG2018735, CTONG2018808, CTONG2018843, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020411, CTONG2020445, CTONG2020522, CTONG2020582, CTONG2020638, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024031, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028124, CTONG2028208, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008894, CTONG3008951, CTONG3008959, CTONG3009028, CTONG3009227, CTONG3009287, CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000358, D6OST2000464, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000083, DFNES1000107, DFNES1000150, DFNES1000185, DFNES2000011, DFNES2000143, DFNES2000146, DFNES2000212, DFNES2000268, DFNES2000335, DFNES2000426, DFNES2000432, DFNES2000457, DFNES2000471, DFNES2000913, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004383, DFNES2004608, DFNES2004684, DFNES2005266, DFNES2005527, DFNES2005540, DFNES2005690, DFNES2005766, DFNES2005779, DFNES2005875, DFNES2006346, DFNES2006944, DFNES2007113, DFNES2007299, DFNES2007332, DFNES2007634, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000024, FCBBF1000027, FCBBF1000038, FCBBF1000053, FCBBF1000061, FCBBF1000069, FCBBF1000077, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182, FCBBF1000197, FCBBF1000198, FCBBF1000206, FCBBF1000220, FCBBF1000232, FCBBF1000233, FCBBF1000243, FCBBF1000270, FCBBF1000272, FCBBF1000280, FCBBF1000294, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000476, FCBBF1000506, FCBBF1000509, FCBBF1000546, FCBBF1000550, FCBBF1000556, FCBBF1000563, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF2000038, FCBBF2000094, FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000566, FCBBF2000576, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001001, FCBBF2001088, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001470, FCBBF3001594, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005330, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009541, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000105, FEBRA2000126, FEBRA2000210, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000452, FEBRA2000454, FEBRA2000462, FEBRA2000468, FEBRA2000491, FEBRA2000504, FEBRA2000510, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000740, FEBRA2000757, FEBRA2000771, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001267, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002399, FEBRA2002410, FEBRA2002429, FEBRA2002442, FEBRA2002456, FEBRA2002508, FEBRA2002525, FEBRA2002527, FEBRA2002552, FEBRA2002574, FEBRA2002601, FEBRA2002611, FEBRA2002628, FEBRA2002662, FEBRA2002682, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003822, FEBRA2003833, FEBRA2003897, FEBRA2003907, FEBRA2003930, FEBRA2004023, FEBRA2004026, FEBRA2004029, FEBRA2004042, FEBRA2004053, FEBRA2004056, FEBRA2004091, FEBRA2004110, FEBRA2004191, FEBRA2004202, FEBRA2004219, FEBRA2004237, FEBRA2004268, FEBRA2004293, FEBRA2004329, FEBRA2004331, FEBRA2004412, FEBRA2004443, FEBRA2004485, FEBRA2004490, FEBRA2004538, FEBRA2004592, FEBRA2004620, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005427, FEBRA2005458, FEBRA2005476, FEBRA2005572, FEBRA2005701, FEBRA2005726, FEBRA2005734, FEBRA2005752, FEBRA2005778, FEBRA2005787, FEBRA2005788, FEBRA2005995, FEBRA2005998, FEBRA2006055, FEBRA2006061, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006370, FEBRA2006379, FEBRA2006396, FEBRA2006409, FEBRA2006418, FEBRA2006427, FEBRA2006476, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007622, FEBRA2007708, FEBRA2007714, FEBRA2007793, FEBRA2007801, FEBRA2007823, FEBRA2007880, FEBRA2007900, FEBRA2007901, FEBRA2007931, FEBRA2007957, FEBRA2008009, FEBRA2008036, FEBRA2008086, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008287, FEBRA2008311, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022055, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000363, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCHON1000015, HCHON1000142, HCHON1000166, HCHON1000176, HCHON2000028, HCHON2000038, HCHON2000087, HCHON2000100, HCHON2000156, HCHON2000160, HCHON2000199, HCHON2000212, HCHON2000226, HCHON2000244, HCHON2000265, HCHON2000271, HCHON2000315, HCHON2000322, HCHON2000344, HCHON2000364, HCHON2000395, HCHON2000417, HCHON2000418, HCHON2000473, HCHON2000475, HCHON2000503, HCHON2000508, HCHON2000626, HCHON2000660, HCHON2000663, HCHON2000673, HCHON2000699, HCHON2000705, HCHON2000738, HCHON2000743, HCHON2000751, HCHON2000815, HCHON2000817, HCHON2000818, HCHON2000819, HCHON2000832, HCHON2000851, HCHON2000898, HCHON2000935, HCHON2000942, HCHON2000956, HCHON2000994, HCHON2001084, HCHON2001099, HCHON2001108, HCHON2001116, HCHON2001161, HCHON2001214, HCHON2001233, HCHON2001269, HCHON2001359, HCHON2001450, HCHON2001453, HCHON2001497, HCHON2001505, HCHON2001519, HCHON2001523, HCHON2001535, HCHON2001546, HCHON2001548, HCHON2001577, HCHON2001598, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004002, HCHON2004007, HCHON2004279, HCHON2004359, HCHON2004531, HCHON2004776, HCHON2004858, HCHON2005048, HCHON2005118, HCHON2005166, HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000088, HEART1000099, HEART1000102, HEART1000118, HEART1000132, HEART1000139, HEART1000142, HEART1000151, HEART1000173, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000114, HHDPC1000118, HHDPC2000055, HHDPC2000070, HHDPC2000104, HHDPC2000149, HHDPC2000258, HHDPC2000315, HHDPC2000462, HHDPC2000572, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006927, HHDPC2007267, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000064, HLUNG1000084, HLUNG1000091, HLUNG1000099, HLUNG1000104, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000412, HLUNG2000431, HLUNG2000480, HLUNG2000489, HLUNG2000501, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013, HLUNG2001126, HLUNG2001144, HLUNG2001146, HLUNG2001214, HLUNG2001459, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, 5 HLUNG2002659, HLUNG2002707, HLUNG2002811, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, 0 HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004154, HLUNG2004159, HLUNG2004170, HLUNG2004217, HLUNG2004273, HLUNG2004388, HLUNG2004416, HLUNG2004521, HLUNG2004534, HLUNG2004684, 5 HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, 0 HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, 5 HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009067, HLUNG2009215, HLUNG2009225, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017262, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000152, HSYRA1000178, HSYRA2000044, HSYRA2000137, HSYRA2000190, HSYRA2000221, HSYRA2000224, HSYRA2000237, HSYRA2000253, HSYRA2000255, HSYRA2000332, HSYRA2000371, HSYRA2000424, HSYRA2000507, HSYRA2000510, HSYRA2000605, HSYRA2000629, HSYRA2000640, HSYRA2000706, HSYRA2000743, HSYRA2000760, HSYRA2000787, HSYRA2000792, HSYRA2000832, HSYRA2000927, HSYRA2001103, HSYRA2001105, HSYRA2001142, HSYRA2001225, HSYRA2001255, HSYRA2001332, HSYRA2001353, HSYRA2001396, HSYRA2001420, HSYRA2001484, HSYRA2001552, HSYRA2001567, HSYRA2001574, HSYRA2001580, HSYRA2001615, HSYRA2001621, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000161, IMR321000165, IMR321000193, IMR321000207, IMR321000210, IMR321000230, IMR321000242, IMR321000253, IMR322000010, IMR322000072, IMR322000107, IMR322000121, IMR322000127, IMR322000144, IMR322000152, IMR322000164, IMR322000178, IMR322000223, IMR322000243, IMR322000302, IMR322000316, IMR322000374, IMR322000379, IMR322000383, IMR322000418, IMR322000518, IMR322000555, IMR322000604, IMR322000609, IMR322000656, IMR322000672, IMR322000742, IMR322000775, IMR322000811, IMR322000819, IMR322000838, IMR322000859, IMR322000863, IMR322000917, IMR322000918, IMR322000919, IMR322000935, IMR322000953, IMR322000984, IMR322001018, IMR322001049, IMR322001167, IMR322001185, IMR322001218, IMR322001245, IMR322001317, IMR322001318, IMR322001322, IMR322001332, IMR322001380, IMR322001438, IMR322001452, IMR322001491, IMR322001534, IMR322001541, IMR322001584, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322013053, IMR322013396, IMR322013731, IMR322013805, IMR322015523, IMR322016146, IMR322017049, IMR322018117, IMR322018192, IMR322019070, JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000145, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000252, KIDNE2000266, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000403, KIDNE2000422, KIDNE2000493, KIDNE2000497, KIDNE2000510, KIDNE2000513, KIDNE2000517, KIDNE2000519, KIDNE2000555, KIDNE2000624, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2001117, KIDNE2001140, KIDNE2001162, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002265, KIDNE2002438, KIDNE2002483, KIDNE2002725, KIDNE2002768, KIDNE2002795, KIDNE2002798, KIDNE2002845, KIDNE2002872, KIDNE2002882, KIDNE2002883, KIDNE2002980, KIDNE2002991, KIDNE2003185, KIDNE2003188, KIDNE2003233, KIDNE2003305, KIDNE2003316, KIDNE2003335, KIDNE2003357, KIDNE2003373, KIDNE2003377, KIDNE2003507, KIDNE2003752, KIDNE2003764, KIDNE2003837, KIDNE2003994, KIDNE2004034, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004475, KIDNE2004519, KIDNE2004520, KIDNE2004531, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006014, KIDNE2006039, KIDNE2006053, KIDNE2006062, KIDNE2006092, KIDNE2006145, KIDNE2006149, KIDNE2006210, KIDNE2006248, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077, KIDNE2007186, KIDNE2007222, KIDNE2007328, KIDNE2007356, KIDNE2007422, KIDNE2007569, KIDNE2007810, KIDNE2007910, KIDNE2007944, KIDNE2007954, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014290, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000050, LIVER1000055, LIVER1000058, LIVER1000063, LIVER1000073, LIVER1000082, LIVER1000099, LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER2000003, LIVER2000033, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003581, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415, LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000132, MAMGL1000151, MAMGL1000178, MAMGL1000182, MAMGL1000184, MESAN1000032, MESAN1000035, MESAN1000079, MESAN1000101, MESAN1000121, MESAN1000126, MESAN1000147, MESAN1000180, MESAN2000023, MESAN2000026, MESAN2000067, MESAN2000092, MESAN2000167, MESAN2000264, MESAN2000291, MESAN2000337, MESAN2000434, MESAN2000457, MESAN2000462, MESAN2000501, MESAN2000620, MESAN2000711, MESAN2000815, MESAN2000894, MESAN2000909, MESAN2001154, MESAN2001450, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002724, MESAN2002790, MESAN2002844, MESAN2002978, MESAN2003035, MESAN2003037, MESAN2003039, MESAN2003058, MESAN2003101, MESAN2003190, MESAN2003322, MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005633, MESAN2005689, MESAN2005724, MESAN2005808, MESAN2005811, MESAN2005957, MESAN2005958, MESAN2006022, MESAN2006043, MESAN2006328, MESAN2006401, MESAN2006563, MESAN2006580, MESAN2006599, MESAN2006971, MESAN2007032, MESAN2007440, MESAN2007597, MESAN2007755, MESAN2008222, MESAN2008415, MESAN2008460, MESAN2008536, MESAN2008679, MESAN2008729, MESAN2008821, MESAN2008926, MESAN2008936, MESAN2009019, MESAN2009081, MESAN2009105, MESAN2009311, MESAN2009418, MESAN2009503, MESAN2009522, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018670, MESAN2018699, MESTC1000042, MESTC1000147, MESTC2000150, MESTC2000153, MESTC2000170, N1ESE2000698, NB9N41000011, NB9N41000047, NB9N41000121, NB9N41000135, NB9N41000142, NB9N41000146, NB9N42000104, NB9N42000122, NB9N42000196, NB9N42000281, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000102, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000195, NT2NE2000222, NT2NE2000284, NT2NE2000299, NT2NE2000327, NT2NE2000353, NT2NE2000356, NT2NE2000369, NT2NE2000374, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000658, NT2NE2000706, NT2NE2000763, NT2NE2000802, NT2NE2000808, NT2NE2000864, NT2NE2000909, NT2NE2000913, NT2NE2000950, NT2NE2000951, NT2NE2000980, NT2NE2001005, NT2NE2001021, NT2NE2001040, NT2NE2001049, NT2NE2001142, NT2NE2001156, NT2NE2001176, NT2NE2001180, NT2NE2001181, NT2NE2001188, NT2NE2001191, NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001617, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001793, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003185, NT2NE2003252, NT2NE2003280, NT2NE2003308, NT2NE2003309, NT2NE2003315, NT2NE2003393, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004519, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000027, NT2RI1000127, NT2RI1000164, NT2RI1000172, NT2RI2000004, NT2RI2000007, NT2RI2000107, NT2RI2000116, NT2RI2000117, NT2RI2000128, NT2RI2000133, NT2RI2000167, NT2RI2000173, NT2RI2000187, NT2RI2000255, NT2RI2000263, NT2RI2000270, NT2RI2000276, NT2RI2000294, NT2RI2000313, NT2RI2000341, NT2RI2000344, NT2RI2000348, NT2RI2000412, NT2RI2000421, NT2RI2000480, NT2RI2000575, NT2RI2000588, NT2RI2000597, NT2RI2000669, NT2RI2000685, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000987, NT2RI2001010, NT2RI2001017, NT2RI2001030, NT2RI2001083, NT2RI2001091, NT2RI2001167, NT2RI2001178, NT2RI2001230, NT2RI2001235, NT2RI2001244, NT2RI2001307, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001657, NT2RI2001726, NT2RI2001731, NT2RI2001769, NT2RI2001782, NT2RI2001836, NT2RI2001846, NT2RI2001859, NT2RI2001866, NT2RI2001910, NT2RI2001952, NT2RI2002022, NT2RI2002041, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002391, NT2RI2002447, NT2RI2002481, NT2RI2002507, NT2RI2002517, NT2RI2002530, NT2RI2002540, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819, NT2RI2002847, NT2RI2002852, NT2RI2002865, NT2RI2002923, NT2RI2002926, NT2RI2002970, NT2RI2003011, NT2RI2003019, NT2RI2003067, NT2RI2003184, NT2RI2003205, NT2RI2003222, NT2RI2003301, NT2RI2003317, NT2RI2003344, NT2RI2003360, NT2RI2003361, NT2RI2003383, NT2RI2003392, NT2RI2003399, NT2RI2003419, NT2RI2003481, NT2RI2003487, NT2RI2003655, NT2RI2003667, NT2RI2003695, NT2RI2003884, NT2RI2003921, NT2RI2003973, NT2RI2004059, NT2RI2004078, NT2RI2004079, NT2RI2004093, NT2RI2004136, NT2RI2004157, NT2RI2004188, NT2RI2004190, NT2RI2004209, NT2RI2004284, NT2RI2004290, NT2RI2004304, NT2RI2004312, NT2RI2004381, NT2RI2004398, NT2RI2004410, NT2RI2004442, NT2RI2004468, NT2RI2004606, NT2RI2004608, NT2RI2004618, NT2RI2004783, NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005116, NT2RI2005130, NT2RI2005150, NT2RI2005166, NT2RI2005239, NT2RI2005246, NT2RI2005315, NT2RI2005358, NT2RI2005368, NT2RI2005382, NT2RI2005473, NT2RI2005520, NT2RI2005564, NT2RI2005579, NT2RI2005621, NT2RI2005628, NT2RI2005647, NT2RI2005670, NT2RI2005713, NT2RI2005772, NT2RI2005811, NT2RI2005816, NT2RI2005851, NT2RI2005966, NT2RI2006070, NT2RI2006071, NT2RI2006081, NT2RI2006126, NT2RI2006127, NT2RI2006183, NT2RI2006257, NT2RI2006271, NT2RI2006294, NT2RI2006345, NT2RI2006403, NT2RI2006412, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006825, NT2RI2006838, NT2RI2006853, NT2RI2006855, NT2RI2006856, NT2RI2006943, NT2RI2006973, NT2RI2007046, NT2RI2007048, NT2RI2007096, NT2RI2007116, NT2RI2007133, NT2RI2007148, NT2RI2007214, NT2RI2007277, NT2RI2007303, NT2RI2007334, NT2RI2007377, NT2RI2007386, NT2RI2007406, NT2RI2007439, NT2RI2007468, NT2RI2007469, NT2RI2007498, NT2RI2007507, NT2RI2007589, NT2RI2007723, NT2RI2007729, NT2RI2007751, NT2RI2007754, NT2RI2007827, NT2RI2007853, NT2RI2007877, NT2RI2007884, NT2RI2007891, NT2RI2007956, NT2RI2007965, NT2RI2007987, NT2RI2008007, NT2RI2008050, NT2RI2008171, NT2RI2008188, NT2RI2008204, NT2RI2008221, NT2RI2008336, NT2RI2008455, NT2RI2008481, NT2RI2008502, NT2RI2008566, NT2RI2008622, NT2RI2008714, NT2RI2008801, NT2RI2008808, NT2RI2008812, NT2RI2008841, NT2RI2008942, NT2RI2008952, NT2RI2009005, NT2RI2009037, NT2RI2009065, NT2RI2009066, NT2RI2009083, NT2RI2009101, NT2RI2009103, NT2RI2009144, NT2RI2009151, NT2RI2009173, NT2RI2009215, NT2RI2009239, NT2RI2009259, NT2RI2009281, NT2RI2009289, NT2RI2009301, NT2RI2009402, NT2RI2009406, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2015342, NT2RI2015533, NT2RI2016128, NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000017, NT2RP6000032, NT2RP6000033, NT2RP6000039, NT2RP6000043, NT2RP6000059, NT2RP6000060, NT2RP6000072, NT2RP6000077, NT2RP6000078, NT2RP6000085, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000069, NT2RP7000076, NT2RP7000086, NT2RP7000109, NT2RP7000112, NT2RP7000173, NT2RP7000238, NT2RP7000259, NT2RP7000271, NT2RP7000311, NT2RP7000359, NT2RP7000364, NT2RP7000380, NT2RP7000391, NT2RP7000425, NT2RP7000477, NT2RP7000579, NT2RP7000586, NT2RP7000600, NT2RP7000624, NT2RP7000812, NT2RP7000926, NT2RP7001074, NT2RP7001080, NT2RP7001156, NT2RP7001166, NT2RP7001231, NT2RP7001283, NT2RP7001306, NT2RP7001319, NT2RP7001335, NT2RP7001364, NT2RP7001475, NT2RP7001532, NT2RP7001591, NT2RP7001602, NT2RP7001678, NT2RP7001856, NT2RP7001962, NT2RP7002064, NT2RP7002151, NT2RP7002163, NT2RP7002243, NT2RP7002282, NT2RP7002376, NT2RP7002379, NT2RP7002449, NT2RP7002450, NT2RP7002478, NT2RP7002554, NT2RP7002594, NT2RP7002603, NT2RP7002619, NT2RP7002650, NT2RP7002738, NT2RP7002779, NT2RP7002802, NT2RP7002829, NT2RP7002841, NT2RP7002875, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203, NT2RP7003261, NT2RP7003439, NT2RP7003511, NT2RP7003629, NT2RP7003647, NT2RP7003680, NT2RP7003688, NT2RP7003724, NT2RP7004027, NT2RP7004080, NT2RP7004114, NT2RP7004196, NT2RP7004233, NT2RP7004260, NT2RP7004348, NT2RP7004352, NT2RP7004358, NT2RP7004396, NT2RP7004481, NT2RP7004541, NT2RP7004559, NT2RP7004641, NT2RP7004656, NT2RP7004687, NT2RP7004722, NT2RP7004766, NT2RP7004884, NT2RP7004911, NT2RP7004915, NT2RP7004925, NT2RP7004961, NT2RP7004964, NT2RP7004975, NT2RP7005118, NT2RP7005205, NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005750, NT2RP7005846, NT2RP7006033, NT2RP7006160, NT2RP7006162, NT2RP7006188, NT2RP7006223, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006621, NT2RP7006636, NT2RP7006701, NT2RP7006853, NT2RP7006886, NT2RP7006887, NT2RP7006980, NT2RP7006995, NT2RP7007114, NT2RP7007221, NT2RP7007226, NT2RP7007252, NT2RP7007269, NT2RP7007310, NT2RP7007359, NT2RP7007381, NT2RP7007406, NT2RP7007480, NT2RP7007504, NT2RP7007530, NT2RP7007537, NT2RP7007580, NT2RP7007594, NT2RP7007610, NT2RP7007617, NT2RP7007643, NT2RP7007766, NT2RP7007842, NT2RP7007925, NT2RP7007930, NT2RP7007975, NT2RP7008013, NT2RP7008015, NT2RP7008133, NT2RP7008137, NT2RP7008142, NT2RP7008144, NT2RP7008161, NT2RP7008167, NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009108, NT2RP7009147, NT2RP7009149, NT2RP7009168, NT2RP7009215, NT2RP7009225, NT2RP7009236, NT2RP7009253, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009482, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000033, NTONG1000052, NTONG1000075, NTONG1000087, NTONG1000123, NTONG1000130, NTONG1000161, NTONG1000172, NTONG1000182, NTONG1000213, NTONG1000214, NTONG1000246, NTONG1000247, NTONG1000257, NTONG1000264, NTONG2000040, NTONG2000107, NTONG2000231, NTONG2000265, NTONG2000334, NTONG2000363, NTONG2000413, NTONG2000492, NTONG2000531, NTONG2000583, NTONG2000800, NTONG2000858, NTONG2000878, NTONG2000966, NTONG2000985, NTONG2001014, NTONG2001079, NTONG2001137, NTONG2001157, NTONG2001173, NTONG2001220, NTONG2001222, NTONG2001362, NTONG2001428, NTONG2001532, NTONG2001587, NTONG2001612, NTONG2001619, NTONG2001642, NTONG2001657, NTONG2001676, NTONG2001762, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805 NTONG2003852, NTONG2003897, NTONG2003928, NTONG2004095 NTONG2004308, NTONG2004521, NTONG2004690, NTONG2004806 NTONG2004829, NTONG2004844, NTONG2004918, NTONG2004991 NTONG2005062, NTONG2005086, NTONG2005137, NTONG2005153 NTONG2005265, NTONG2005277, NTONG2005363, NTONG2005373 NTONG2005391, NTONG2005480, NTONG2005497, NTONG2005520 NTONG2005577, NTONG2005657, NTONG2005801, NTONG2005822 NTONG2005897, NTONG2005969, NTONG2006099, NTONG2006187 NTONG2006301, NTONG2006324, NTONG2006354, NTONG2006440 NTONG2006484, NTONG2006501, NTONG2006646, NTONG2006709 NTONG2006783, NTONG2007020, NTONG2007028, NTONG2007034 NTONG2007249, NTONG2007517, NTONG2007522, NTONG2007658, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054, OCBBF1000067, OCBBF1000080, OCBBF1000085, OCBBF1000086, OCBBF1000091, OCBBF1000104, OCBBF1000118, OCBBF1000119, OCBBF1000122, OCBBF1000130, OCBBF1000138, OCBBF1000145, OCBBF1000146, OCBBF1000153, OCBBF1000175, OCBBF1000185, OCBBF1000188, OCBBF1000192, OCBBF1000254, OCBBF1000315, OCBBF2000015, OCBBF2000126, OCBBF2000178, OCBBF2000231, OCBBF2000277, OCBBF2000287, OCBBF2000323, OCBBF2000452, OCBBF2000467, OCBBF2000677, OCBBF2000703, OCBBF2000712, OCBBF2000719, OCBBF2000824, OCBBF2000831, OCBBF2000904, OCBBF2000982, OCBBF2000986, OCBBF2000998, OCBBF2001075, OCBBF2001101, OCBBF2001124, OCBBF2001140, OCBBF2001166, OCBBF2001176, OCBBF2001186, OCBBF2001196, OCBBF2001210, OCBBF2001252, OCBBF2001307, OCBBF2001323, OCBBF2001389, OCBBF2001402, OCBBF2001408, OCBBF2001416, OCBBF2001473, OCBBF2001494, OCBBF2001527, OCBBF2001528, OCBBF2001586, OCBBF2001639, OCBBF2001681, OCBBF2001687, OCBBF2001706, OCBBF2001749, OCBBF2001794, OCBBF2001910, OCBBF2001931, OCBBF2001961, OCBBF2001983, OCBBF2002015, OCBBF2002083, OCBBF2002086, OCBBF2002124, OCBBF2002161, OCBBF2002290, OCBBF2002357, OCBBF2002376, OCBBF2002615, OCBBF2002626, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038, OCBBF2004104, OCBBF2004147, OCBBF2004168, OCBBF2004235, OCBBF2004259, OCBBF2004273, OCBBF2004385, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007931, OCBBF2007946, OCBBF2008005, OCBBF2008041, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775, OCBBF2008790, OCBBF2008814, OCBBF2008822, OCBBF2009095, OCBBF2009115, OCBBF2009242, OCBBF2009352, OCBBF2009391, OCBBF2009424, OCBBF2009536, OCBBF2009571, OCBBF2009583, OCBBF2009603, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010830, OCBBF2010841, OCBBF2010843, OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011160, OCBBF2011177, OCBBF2011228, OCBBF2011232, OCBBF2011283, OCBBF2011311, OCBBF2011536, OCBBF2011625, OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012553, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013843, OCBBF2013883, OCBBF2013926, OCBBF2014052, OCBBF2014089, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016841, OCBBF2016928, OCBBF2017035, OCBBF2017055, OCBBF2017069, OCBBF2017306, OCBBF2017325, OCBBF2017398, OCBBF2017458, OCBBF2017489, OCBBF2017516, OCBBF2017665, OCBBF2017754, OCBBF2017791, OCBBF2017815, OCBBF2017882, OCBBF2017888, OCBBF2017893, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018827, OCBBF2018828, OCBBF2018873, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF3000097, OCBBF3000213, OCBBF3000296, OCBBF3000323, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000174, PEBLM1000180, PEBLM2000030, PEBLM2000112, PEBLM2000126, PEBLM2000170, PEBLM2000180, PEBLM2000213, PEBLM2000222, PEBLM2000248, PEBLM2000267, PEBLM2000270, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000080, PLACE5000105, PLACE5000113, PLACE5000115, PLACE5000116, PLACE5000129, PLACE5000139, PLACE5000153, PLACE5000159, PLACE5000170, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000371, PLACE6000414, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001118, PLACE6001153, PLACE6001185, PLACE6001262, PLACE6001281, PLACE6001294, PLACE6001443, PLACE6001536, PLACE6001557, PLACE6001712, PLACE6001740, PLACE6001823, PLACE6001886, PLACE6001925, PLACE6001933, PLACE6002011, PLACE6002016, PLACE6002056, PLACE6002102, PLACE6002157, PLACE6002312, PLACE6002323, PLACE6002345, PLACE6002419, PLACE6002462, PLACE6002647, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003383, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004101, PLACE6004219, PLACE6004312, PLACE6004336, PLACE6004368, PLACE6004380, PLACE6004396, PLACE6004397, PLACE6004454, PLACE6004464, PLACE6004491, PLACE6004663, PLACE6004687, PLACE6004738, PLACE6004931, PLACE6004966, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6008036, PLACE6008094, PLACE6008126, PLACE6008285, PLACE6008444, PLACE6008453, PLACE6008640, PLACE6008750, PLACE6008768, PLACE6008775, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011057, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016383, PLACE6016942, PLACE6016984, PLACE6017002, PLACE6017431, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000097, PROST1000110, PROST1000123, PROST1000136, PROST1000144, PROST1000145, PROST1000152, PROST1000167, PROST1000184, PROST1000199, PROST1000215, PROST1000217, PROST1000220, PROST1000226, PROST1000246, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000203, PROST2000206, PROST2000212, PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000761, PROST2000835, PROST2000893, PROST2000961, PROST2001002, PROST2001116, PROST2001138, PROST2001180, PROST2001213, PROST2001283, PROST2001414, PROST2001415, PROST2001465, PROST2001521, PROST2001540, PROST2001552, PROST2001599, PROST2001611, PROST2001636, PROST2001796, PROST2001805, PROST2001823, PROST2001883, PROST2001899, PROST2001997, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002960, PROST2003102, PROST2003117, PROST2003210, PROST2003302, PROST2003303, PROST2003324, PROST2003338, PROST2003340, PROST2003396, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004258, PROST2004270, PROST2004332, PROST2004416, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426, PROST2005466, PROST2005604, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006060, PROST2006196, PROST2006201, PROST2006260, PROST2006282, PROST2006343, PROST2006450, PROST2006491, PROST2006510, PROST2006579, PROST2006688, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008268, PROST2008271, PROST2008360, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010782, PROST2010885, PROST2011012, PROST2011038, PROST2011105, PROST2011297, PROST2011410, PROST2011439, PROST2011482, PROST2011577, PROST2011631, PROST2011660, PROST2012005, PROST2012016, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017367, PROST2017413, PROST2017441, PROST2017529, PROST2017578, PROST2017612, PROST2017617, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000081, PUAEN1000085, PUAEN1000087, PUAEN1000121, PUAEN1000147, PUAEN1000164, PUAEN1000175, PUAEN1000239, PUAEN1000275, PUAEN1000276, PUAEN1000288, PUAEN2000080, PUAEN2000152, PUAEN2000175, PUAEN2000247, PUAEN2000312, PUAEN2000394, PUAEN2000594, PUAEN2000684, PUAEN2000942, PUAEN2001260, PUAEN2001345, PUAEN2001526, PUAEN2001586, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000022, SKMUS1000030, SKMUS1000064, SKMUS1000067, SKMUS1000084, SKMUS1000102, SKMUS1000104, SKMUS1000110, SKMUS1000118, SKMUS1000124, SKMUS1000129, SKMUS1000176, SKMUS1000177, SKMUS1000186, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000361, SKMUS2000365, SKMUS2000380, SKMUS2000390, SKMUS2000416, SKMUS2000458, SKMUS2000467, SKMUS2000468, SKMUS2000484, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000821, SKMUS2000847, SKMUS2000863, SKMUS2000873, SKMUS2000894, SKMUS2000898, SKMUS2000902, SKMUS2000945, SKMUS2000954, SKMUS2000961, SKMUS2000973, SKMUS2000980, SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001528, SKMUS2001543, SKMUS2001580, SKMUS2001585, SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000159, SKNMC1000168, SKNMC1000229, SKNMC1000251, SKNMC1000264, SKNMC2000065, SKNMC2000224, SKNMC2000256, SKNMC2000305, SKNMC2000322, SKNMC2000356, SKNMC2000374, SKNMC2000583, SKNMC2000593, SKNMC2000612, SKNMC2000622, SKNMC2000966, SKNMC2001113, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000174, SKNSH1000301, SKNSH1000308, SKNSH1000416, SKNSH2000051, SKNSH2000101, SKNSH2000151, SKNSH2000163, SKNSH2000250, SKNSH2000347, SKNSH2000482, SKNSH2000516, SKNSH2000550, SKNSH2000716, SKNSH2000819, SKNSH2000971, SKNSH2001222, SKNSH2001931, SKNSH2002054, SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008969, SKNSH2009197, SKNSH2009357, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000118, SMINT1000131, SMINT1000137, SMINT1000192, SMINT2000007, SMINT2000018, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000396, SMINT2000400, SMINT2000454, SMINT2000518, SMINT2000529, SMINT2000530, SMINT2000537, SMINT2000538, SMINT2000541, SMINT2000545, SMINT2000558, SMINT2000562, SMINT2000568, SMINT2000609, SMINT2000629, SMINT2000694, SMINT2000747, SMINT2000811, SMINT2000824, SMINT2000933, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2001950, SMINT2002126, SMINT2002159, SMINT2002202, SMINT2002210, SMINT2002224, SMINT2002281, SMINT2002311, SMINT2002314, SMINT2002328, SMINT2002414, SMINT2002457, SMINT2002620, SMINT2002689, SMINT2002743, SMINT2002778, SMINT2002880, SMINT2002882, SMINT2002884, SMINT2002899, SMINT2002974, SMINT2003003, SMINT2003128, SMINT2003150, SMINT2003317, SMINT2003386, SMINT2003423, SMINT2003505, SMINT2003551, SMINT2003569, SMINT2003641, SMINT2003644, SMINT2003866, SMINT2003905, SMINT2004037, SMINT2004047, SMINT2004086, SMINT2004280, SMINT2004299, SMINT2004339, SMINT2004350, SMINT2004414, SMINT2004473, SMINT2004547, SMINT2004583, SMINT2004589, SMINT2004729, SMINT2004781, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011588, SMINT2011656, SMINT2011888, SMINT2011958, SMINT2012040, SMINT2012122, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000165, SPLEN1000166, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000222, SPLEN2000242, SPLEN2000243, SPLEN2000247, SPLEN2000258, SPLEN2000267, SPLEN2000270, SPLEN2000307, SPLEN2000341, SPLEN2000350, SPLEN2000357, SPLEN2000364, SPLEN2000402, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000698, SPLEN2000839, SPLEN2000882, SPLEN2001135, SPLEN2001141, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002467, SPLEN2002493, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004343, SPLEN2004346, SPLEN2004368, SPLEN2004611, SPLEN2004662, SPLEN2004773, SPLEN2004844, SPLEN2004880, SPLEN2004984, SPLEN2005009, SPLEN2005227, SPLEN2005272, SPLEN2005342, SPLEN2005416, SPLEN2005429, SPLEN2005450, SPLEN2005474, SPLEN2005560, SPLEN2005727, SPLEN2005783, SPLEN2005790, SPLEN2005806, SPLEN2005818, SPLEN2005838, SPLEN2005869, SPLEN2005927, SPLEN2005939, SPLEN2006143, SPLEN2006232, SPLEN2006268, SPLEN2006283, SPLEN2006305, SPLEN2006325, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008591, SPLEN2008786, SPLEN2009081, SPLEN2009088, SPLEN2009163, SPLEN2009315, SPLEN2009340, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012571, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016268, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017643, SPLEN2017687, SPLEN2017740, SPLEN2017781, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019471, SPLEN2019480, SPLEN2019505, SPLEN2019571, SPLEN2019575, SPLEN2019709, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027113, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028466, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031674, SPLEN2031724, SPLEN2031780, SPLEN2032036, SPLEN2032112, SPLEN2032154, SPLEN2032321, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042598, SPLEN2042714, SPLEN2042920, STOMA1000013, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000257, STOMA2000279, STOMA2000289, STOMA2000383, STOMA2000386, STOMA2000395, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002052, STOMA2002141, STOMA2002166, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000124, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001033, SYNOV2001035, SYNOV2001088, SYNOV2001111, SYNOV2001212, SYNOV2001251, SYNOV2001262, SYNOV2001300, SYNOV2001356, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007671, SYNOV4007711, SYNOV4008245, SYNOV4008336, SYNOV4009129, SYNOV4009139, SYNOV4009298, SYNOV4009575, T1ESE2000116, T1ESE2000609, T1ESE2000904, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000023, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179, TESTI2000184, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001120, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003413, TESTI2003418, TESTI2003419, TESTI2003432, TESTI2003454, TESTI2003475, TESTI2003476, TESTI2003498, TESTI2003512, TESTI2003533, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005759, TESTI2005767, TESTI2005775, TESTI2005784, TESTI2005788, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007320, TESTI2007346, TESTI2007358, TESTI2007402, TESTI2007405, TESTI2007407, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009520, TESTI2009544, TESTI2009545, TESTI2009551, TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2010009, TESTI2010154, TESTI2010180, TESTI2010239, TESTI2010280, TESTI2010354, TESTI2010396, TESTI2010400, TESTI2010409, TESTI2010513, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015626, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018462, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021124, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034940, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000014, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009286, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010851, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI40T1956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000029, THYMU1000032, THYMU1000033, THYMU1000041, THYMU1000060, THYMU1000074, THYMU1000083, THYMU1000098, THYMU1000103, THYMU1000105, THYMU1000109, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000359, THYMU1000366, THYMU1000382, THYMU1000393, THYMU1000394, THYMU1000399, THYMU1000426, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879, THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002841, THYMU2002852, THYMU2002910, THYMU2002950, THYMU2002983, THYMU2003012, THYMU2003046, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003446, THYMU2003470, THYMU2003479, THYMU2003499, THYMU2003632, THYMU2003650, THYMU2003700, THYMU2003760, THYMU2003803, THYMU2003855, THYMU2003907, THYMU2003932, THYMU2003981, THYMU2004003, THYMU2004008, THYMU2004042, THYMU2004108, THYMU2004111, THYMU2004123, THYMU2004139, THYMU2004152, THYMU2004169, THYMU2004284, THYMU2004320, THYMU2004336, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006505, THYMU2006610, THYMU2006666, THYMU2006712, THYMU2006813, THYMU2006913, THYMU2006946, THYMU2006965, THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010094, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027125, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032798, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000030, TRACH1000038, TRACH1000057, TRACH1000074, TRACH1000100, TRACH1000106, TRACH1000125, TRACH1000140, TRACH1000144, TRACH1000193, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000461, TRACH2000496, TRACH2000497, TRACH2000502, TRACH2000522, TRACH2000540, TRACH2000559, TRACH2000660, TRACH2000665, TRACH2000675, TRACH2000677, TRACH2000702, TRACH2000703, TRACH2000767, TRACH2000775, TRACH2000780, TRACH2000807, TRACH2000862, TRACH2000873, TRACH2000894, TRACH2000898, TRACH2000920, TRACH2000923, TRACH2000926, TRACH2000944, TRACH2000959, TRACH2000981, TRACH2000986, TRACH2001021, TRACH2001037, TRACH2001060, TRACH2001085, TRACH2001101, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001341, TRACH2001347, TRACH2001395, TRACH2001400, TRACH2001403, TRACH2001416, TRACH2001428, TRACH2001443, TRACH2001474, TRACH2001478, TRACH2001525, TRACH2001532, TRACH2001549, TRACH2001592, TRACH2001596, TRACH2001612, TRACH2001621, TRACH2001646, TRACH2001651, TRACH2001684, TRACH2001810, TRACH2001898, TRACH2001933, TRACH2001968, TRACH2001996, TRACH2002043, TRACH2002054, TRACH2002100, TRACH2002138, TRACH2002664, TRACH2002784, TRACH2002803, TRACH2002840, TRACH2002847, TRACH2002853, TRACH2002862, TRACH2002954, TRACH2002960, TRACH2002988, TRACH2003070, TRACH2003272, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2005811, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894, TRACH2007988, TRACH2008081, TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014124, TRACH2014268, TRACH2014284, TRACH2014337, TRACH2014356, TRACH2014371, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002192, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000152, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002294, UTERU2002332, UTERU2002410, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004039, UTERU2004061, UTERU2004073, UTERU2004163, UTERU2004299, UTERU2004461, UTERU2004520, UTERU2004564, UTERU2004664, UTERU2004688, UTERU2004698, UTERU2004807, UTERU2004861, UTERU2004929, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007924, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012286, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015087, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016799, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023175, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the BGGI1, BLADE, BNGH4, BRACE, BRALZ, BRAMY, and BRASW libraries were analyzed for expression frequency. The analysis results are described below. Each is a relative value, where the expression level over all tissues is taken as 100. The larger this value, the greater the level of expression.

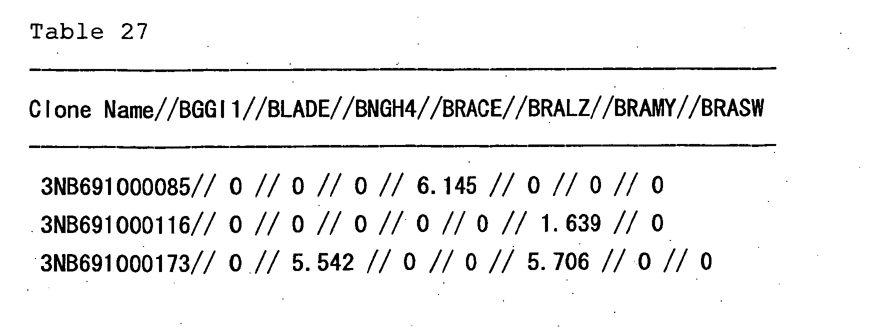

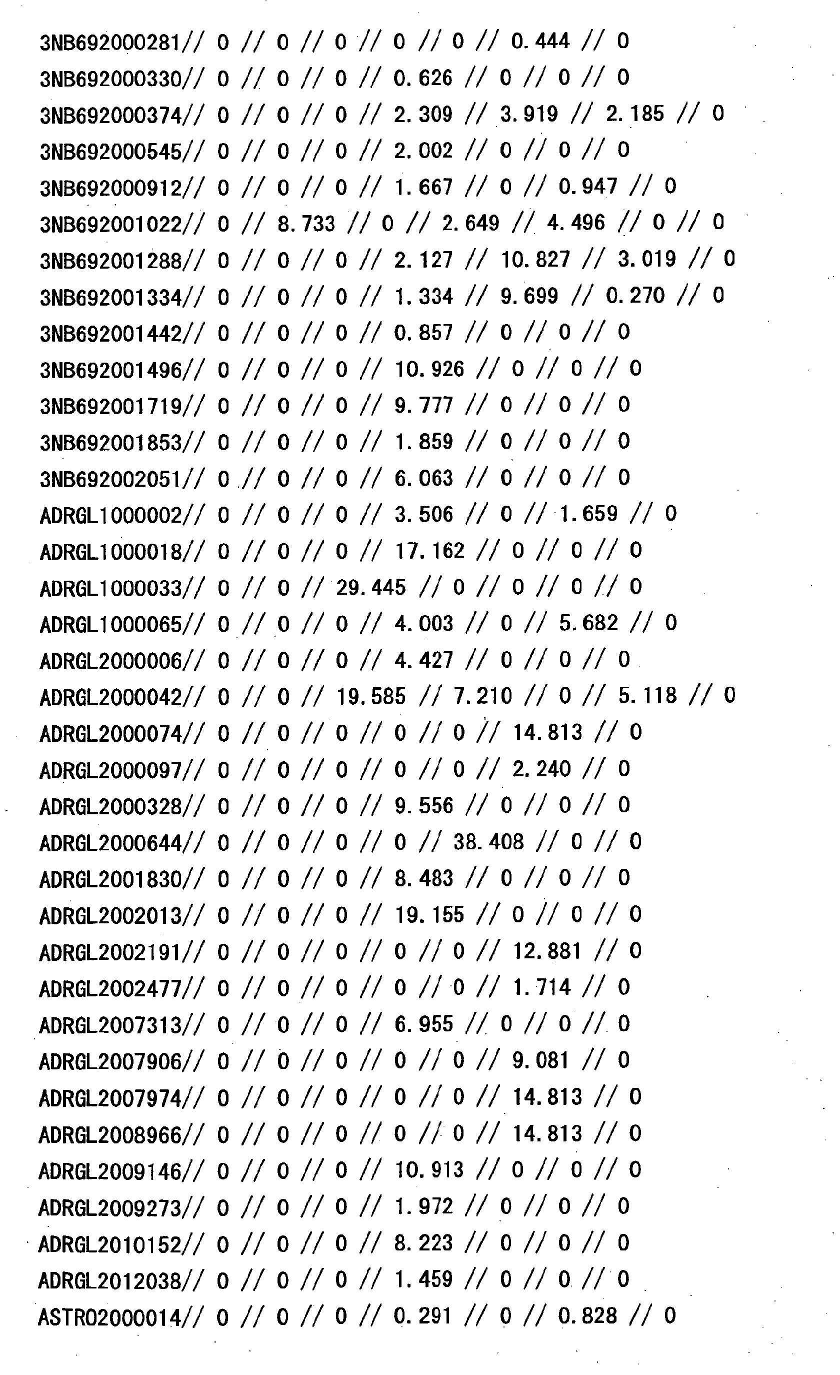

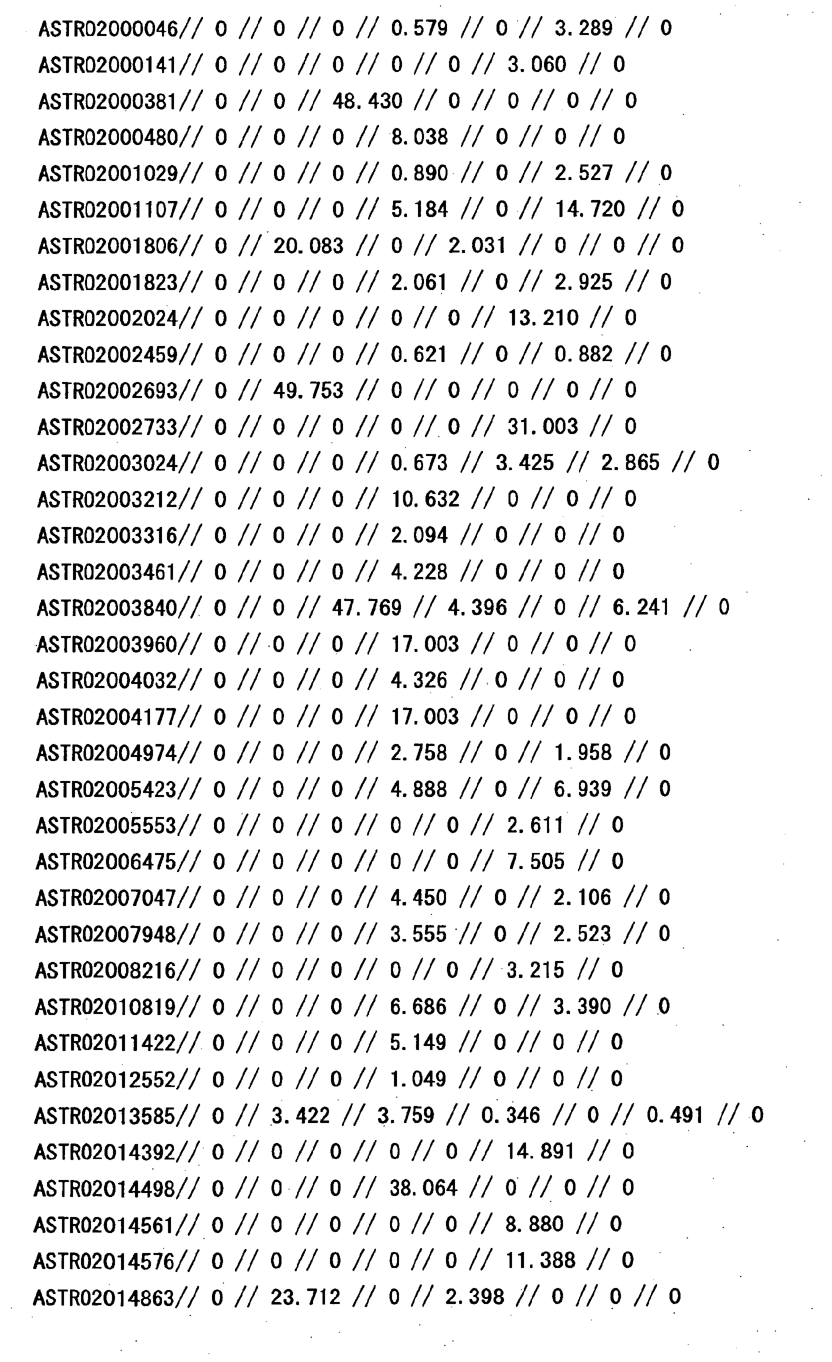

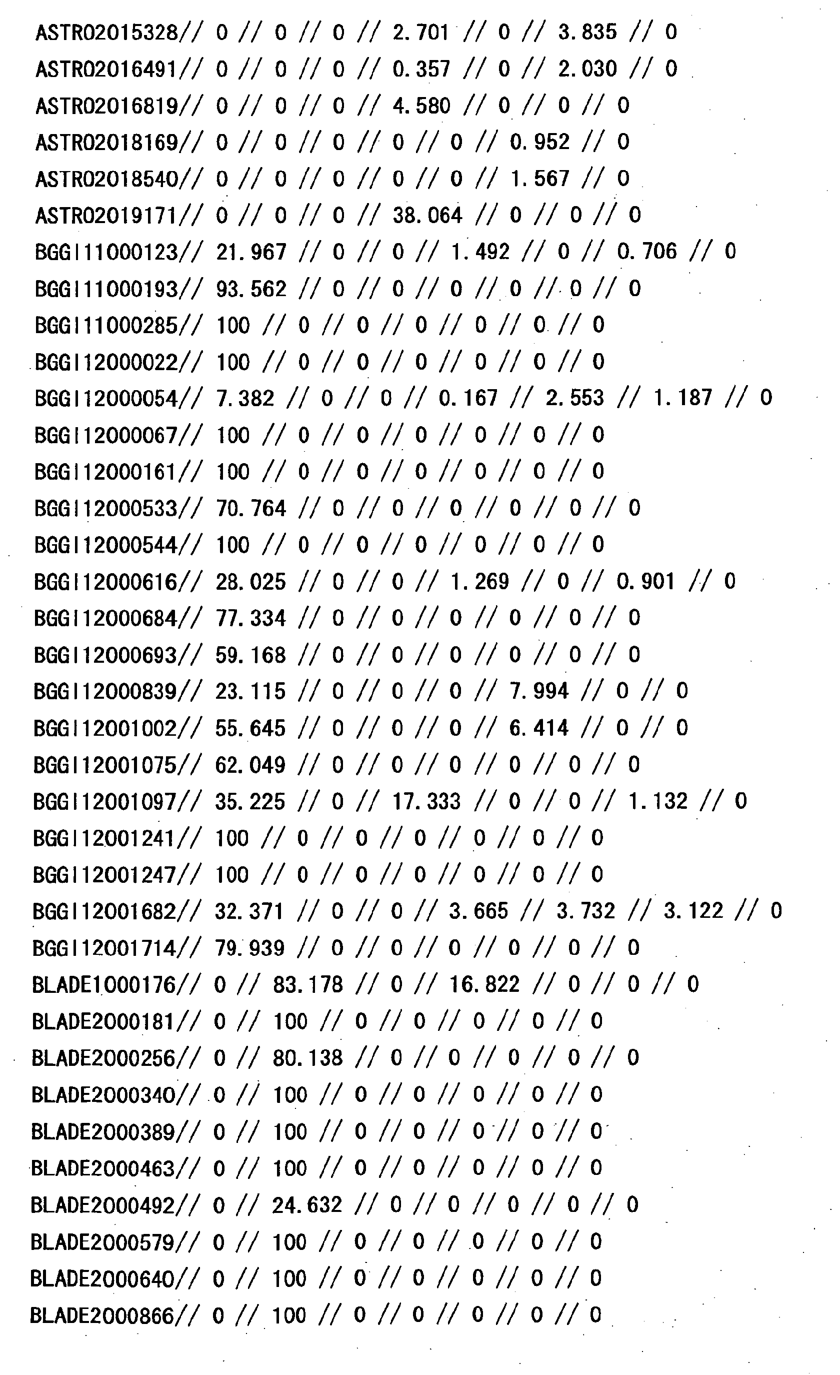

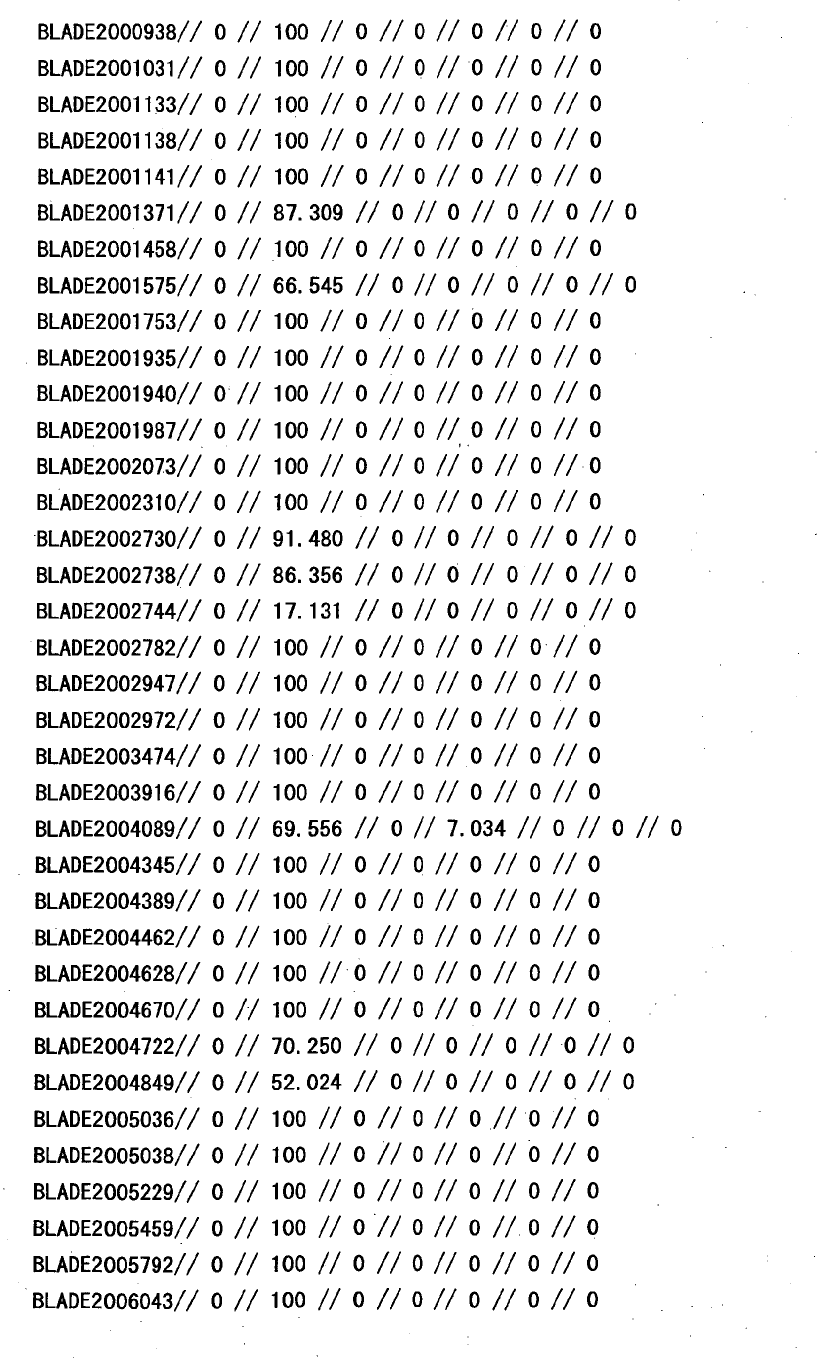

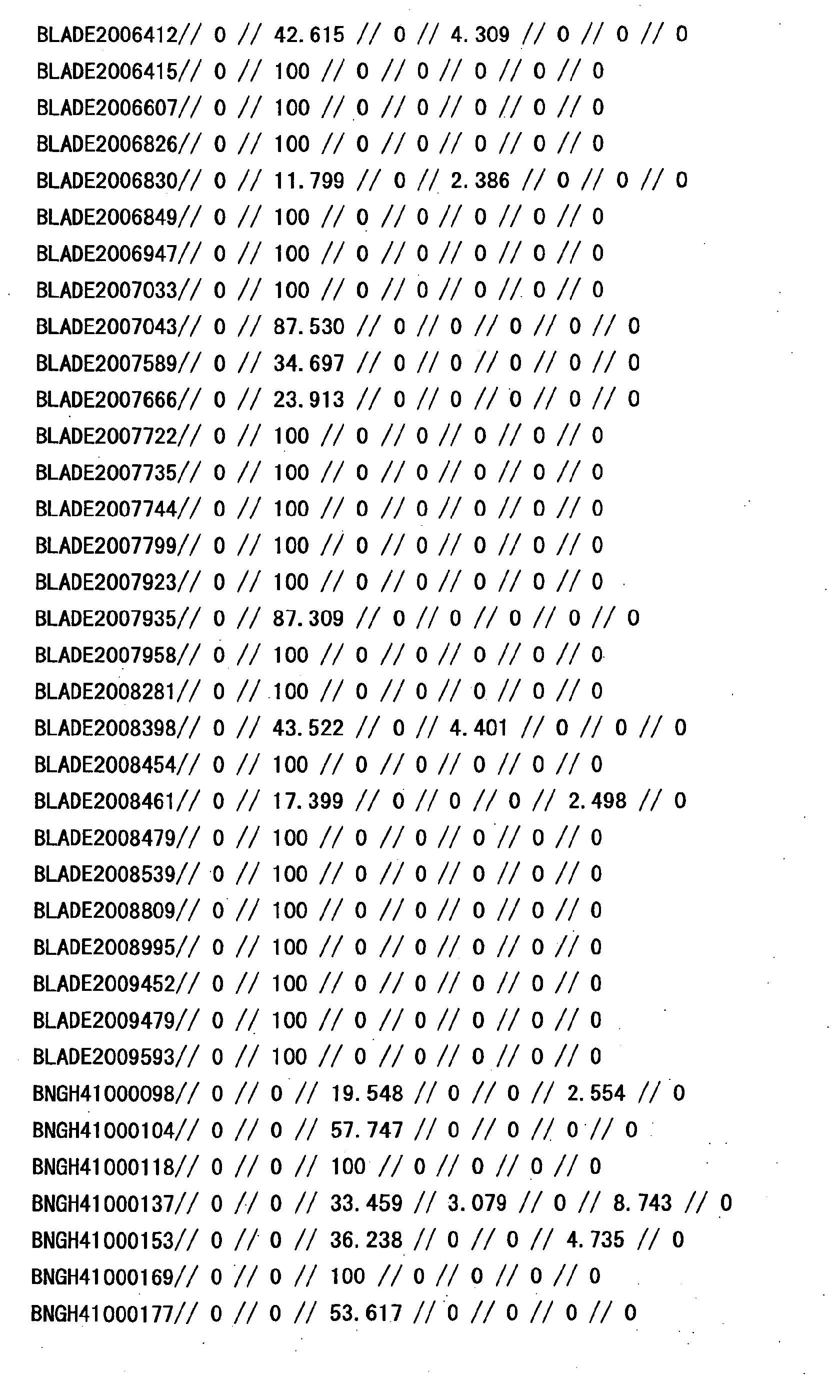

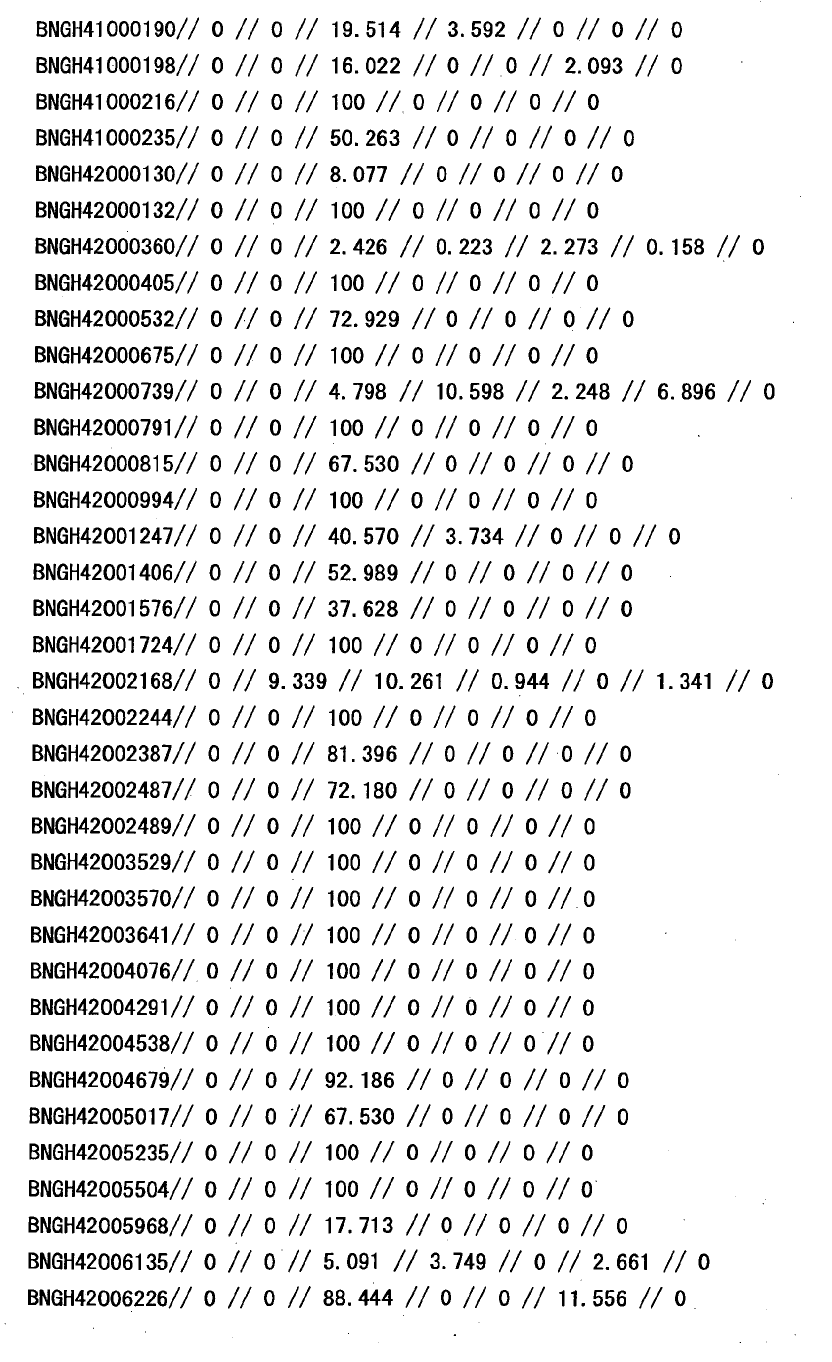

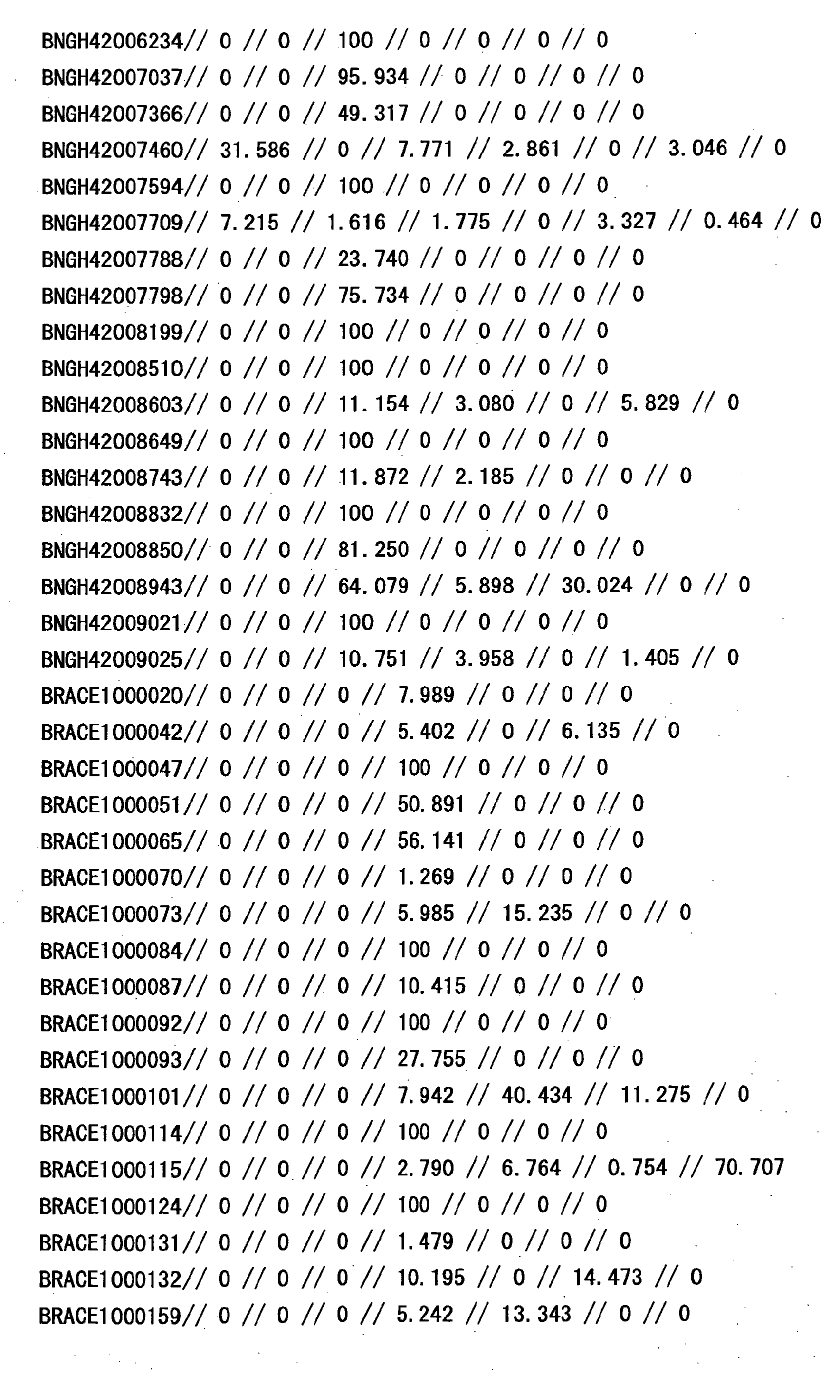

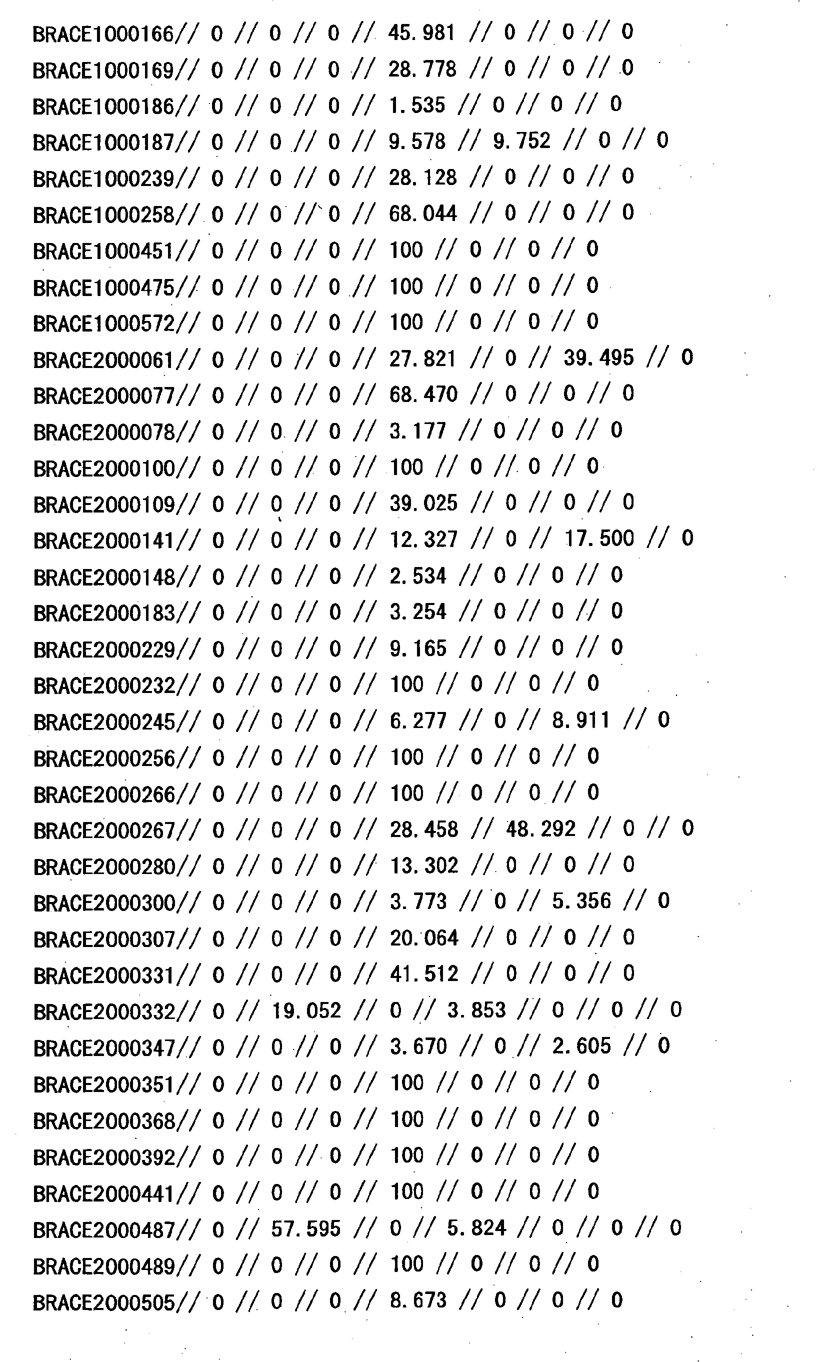

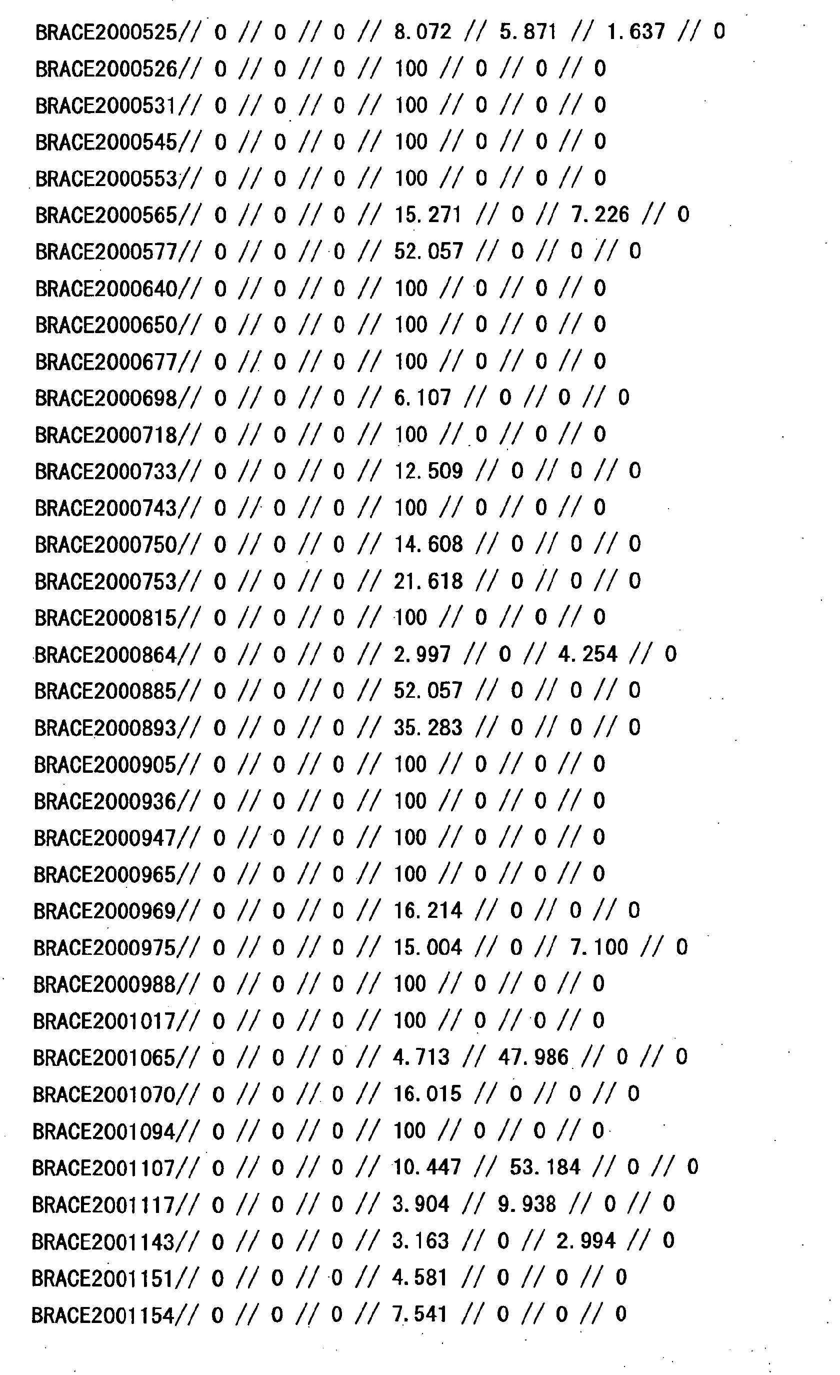

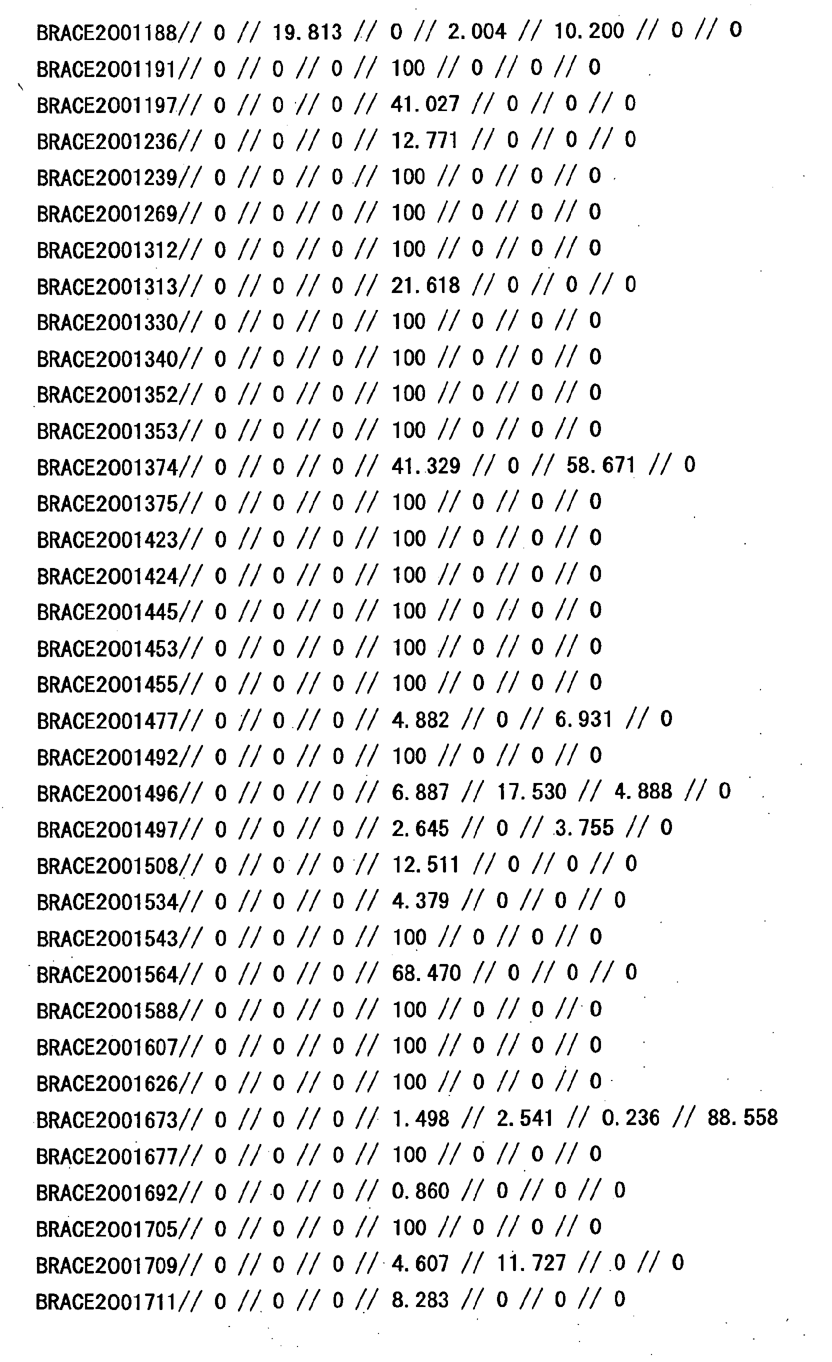

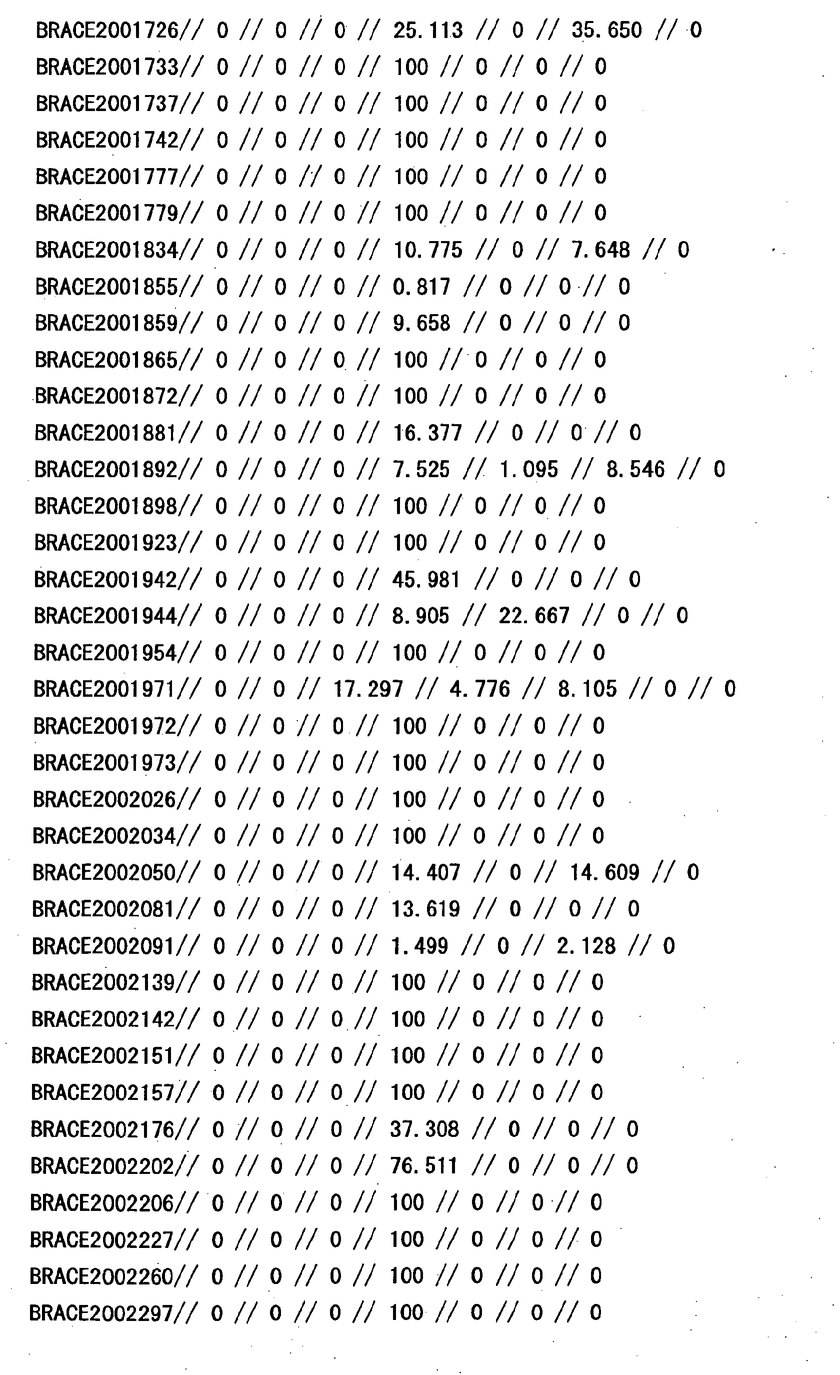

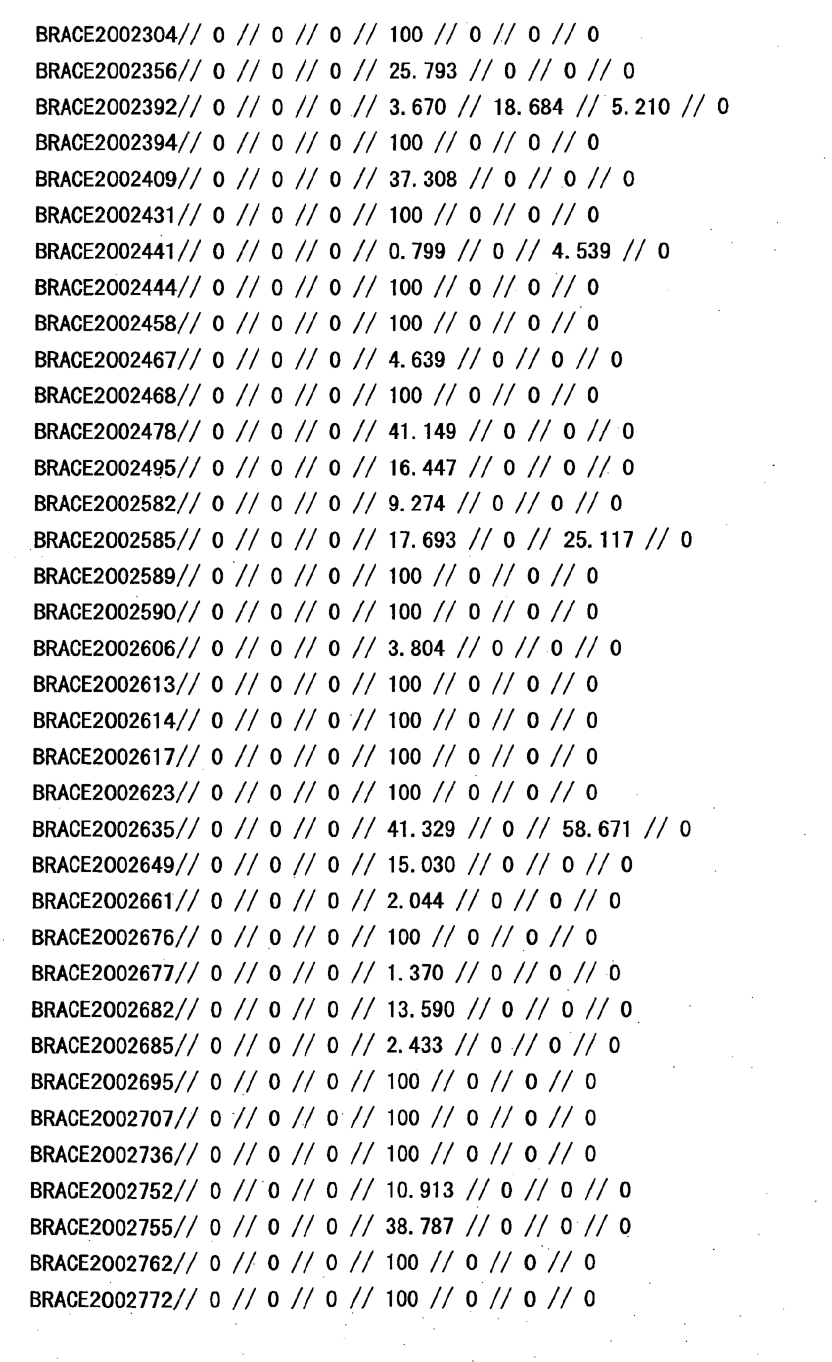

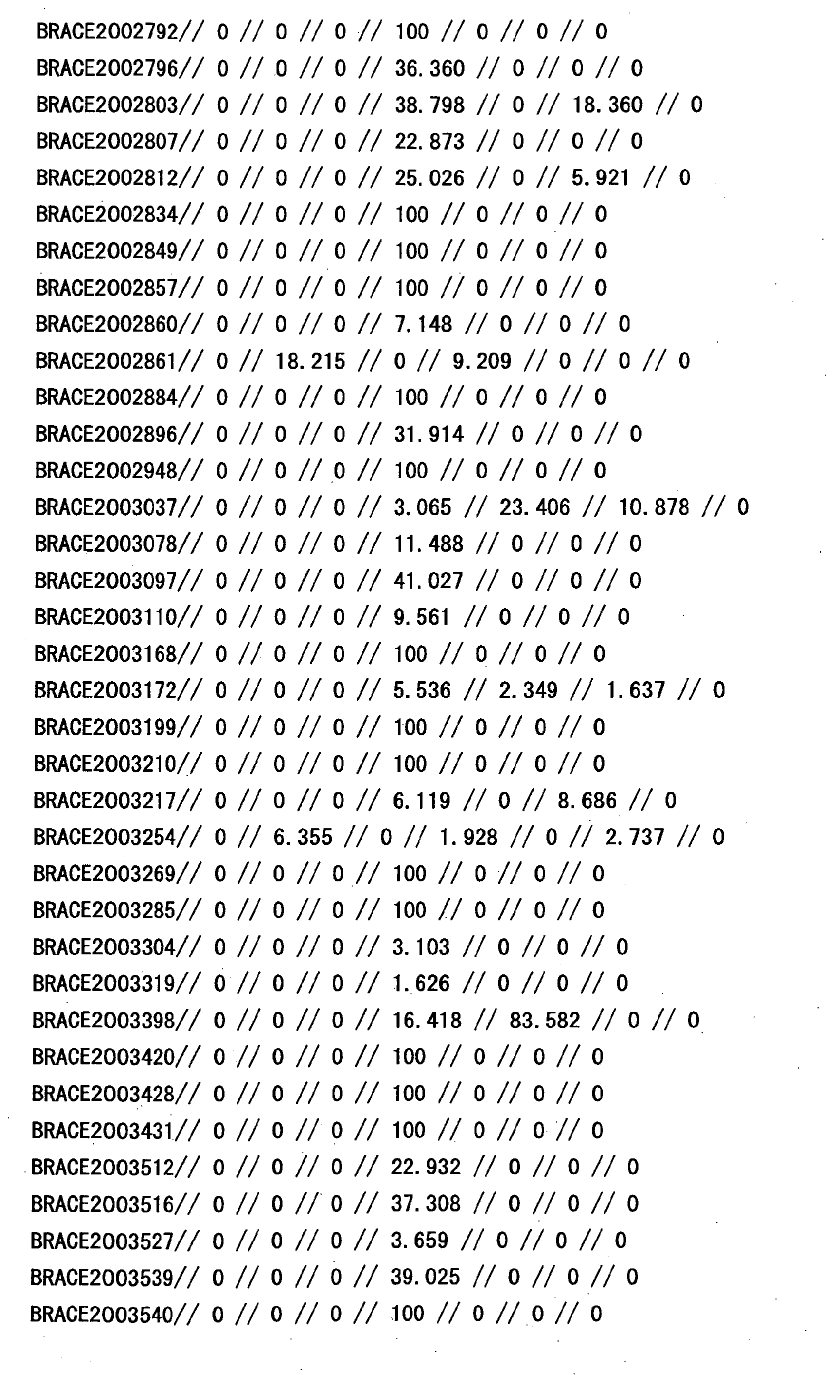

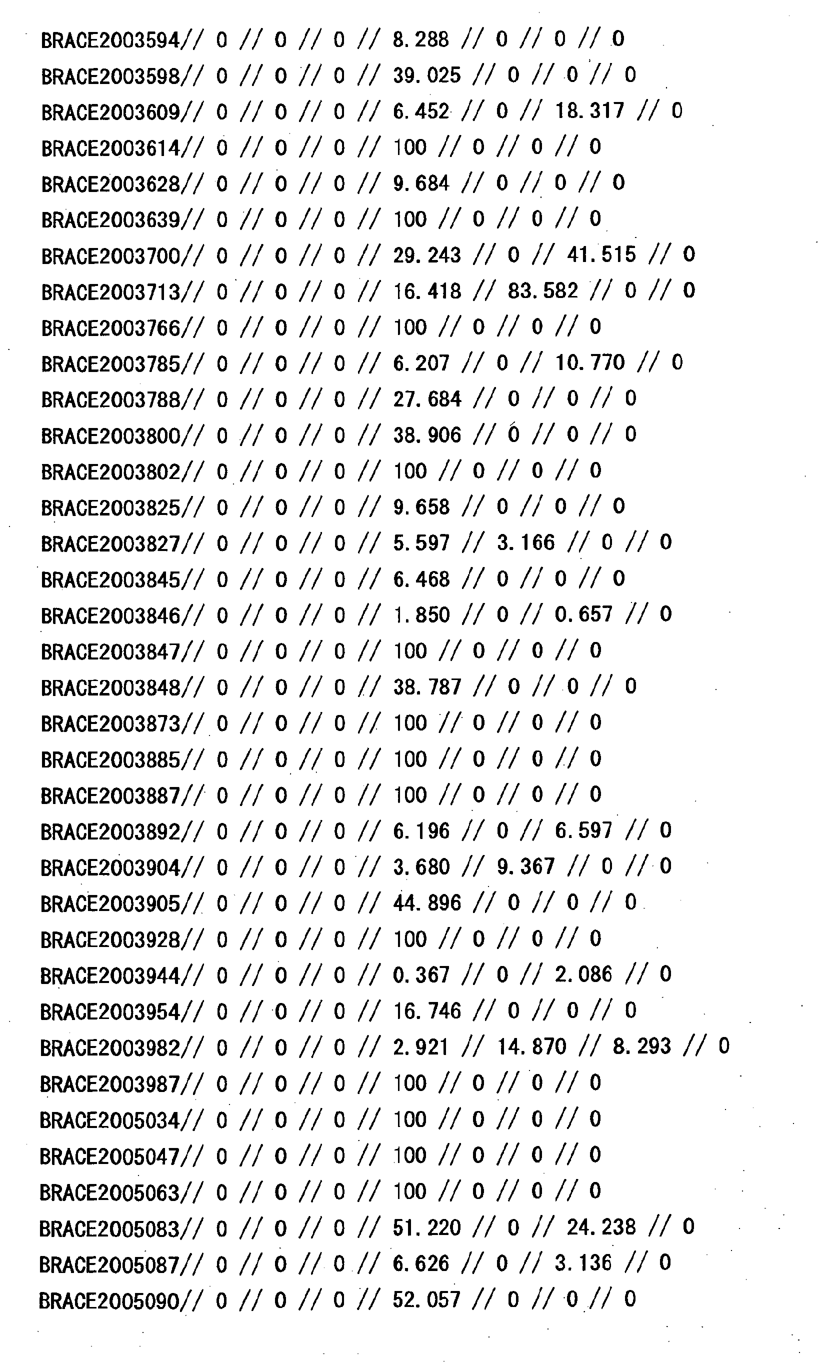

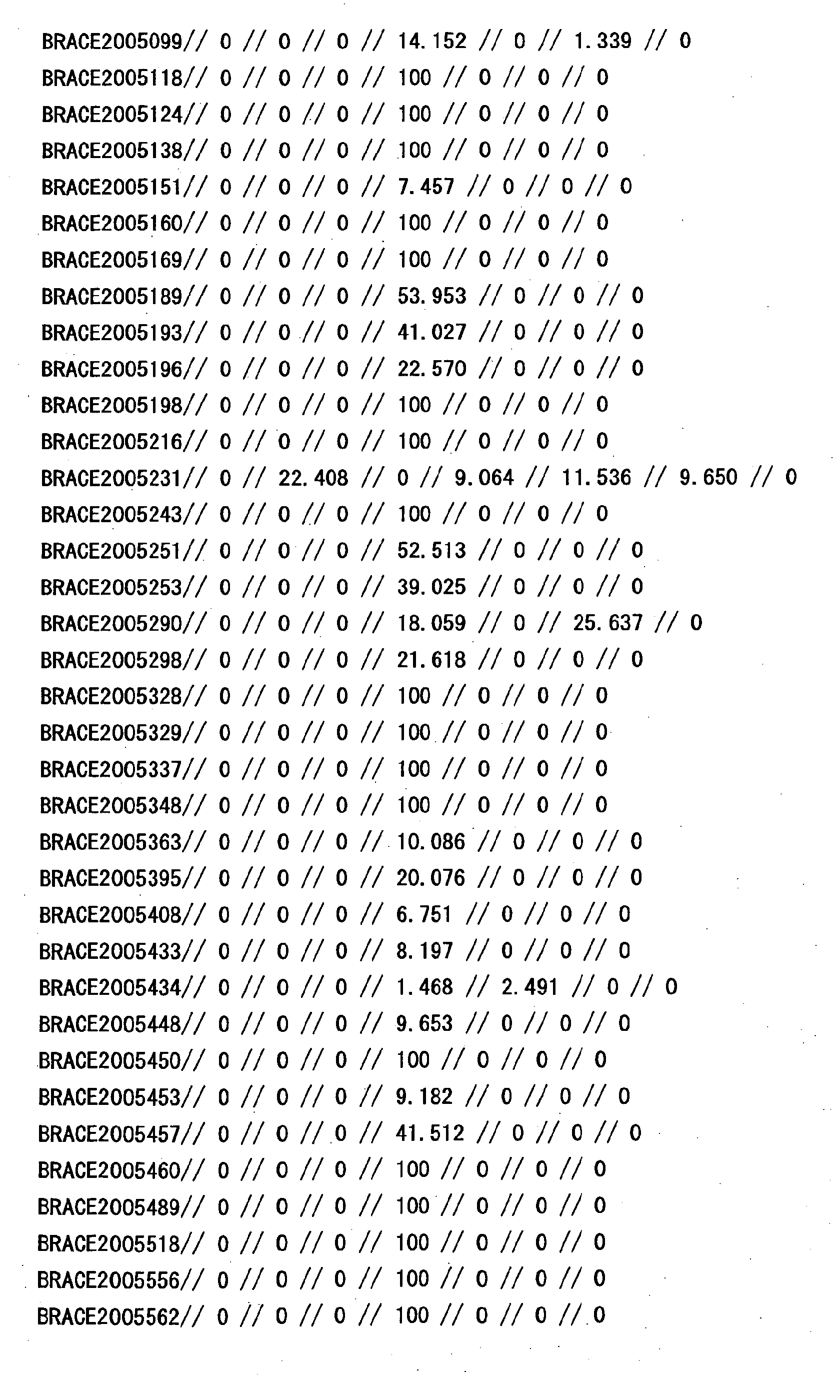

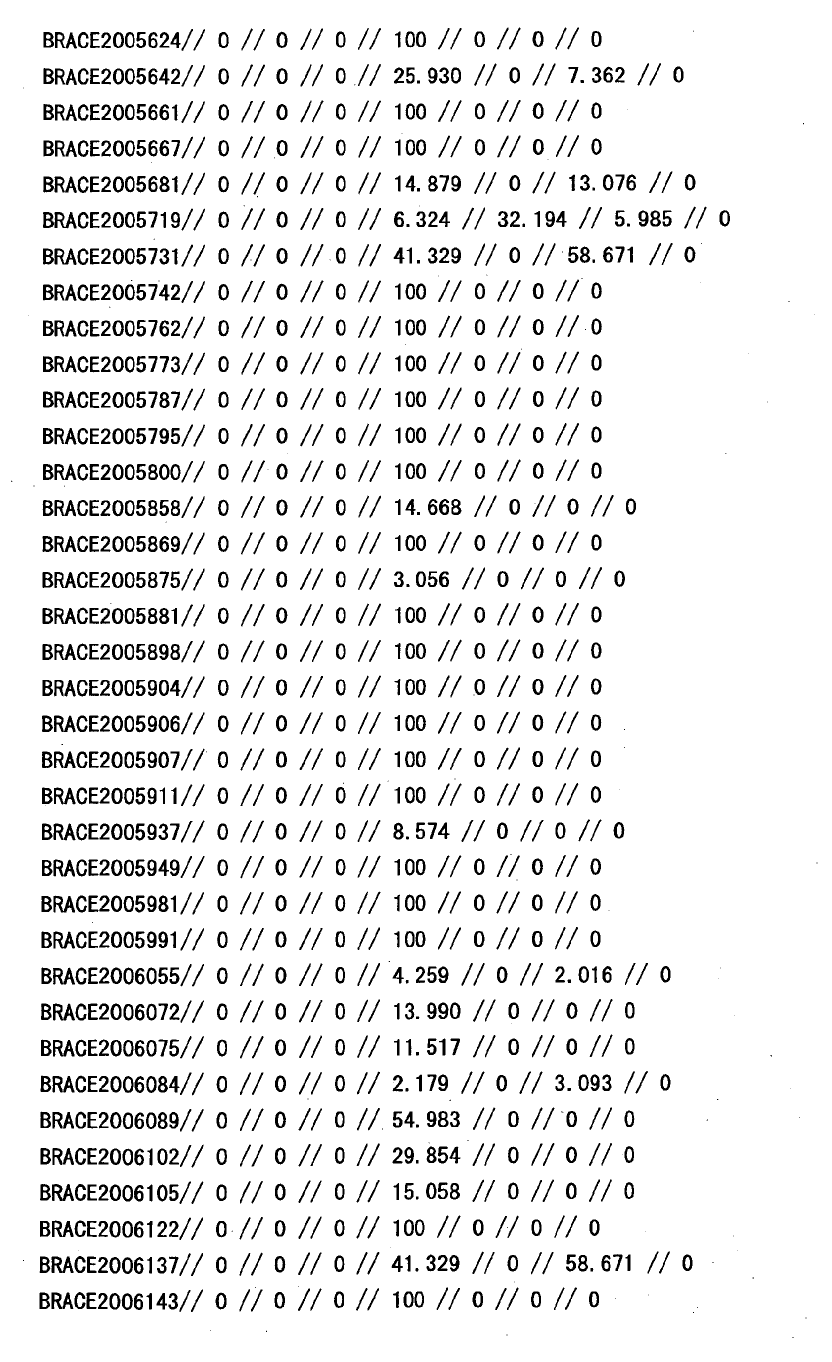

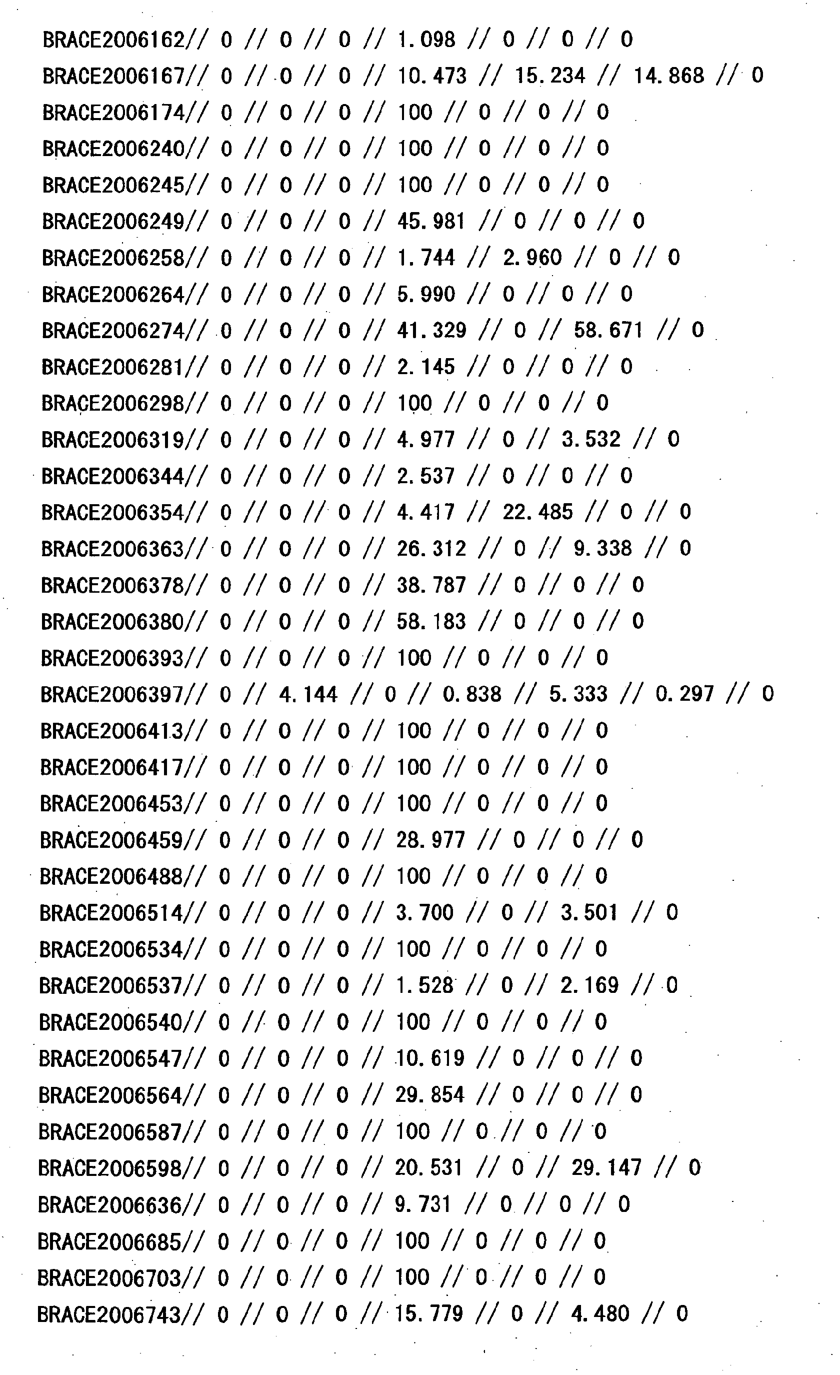

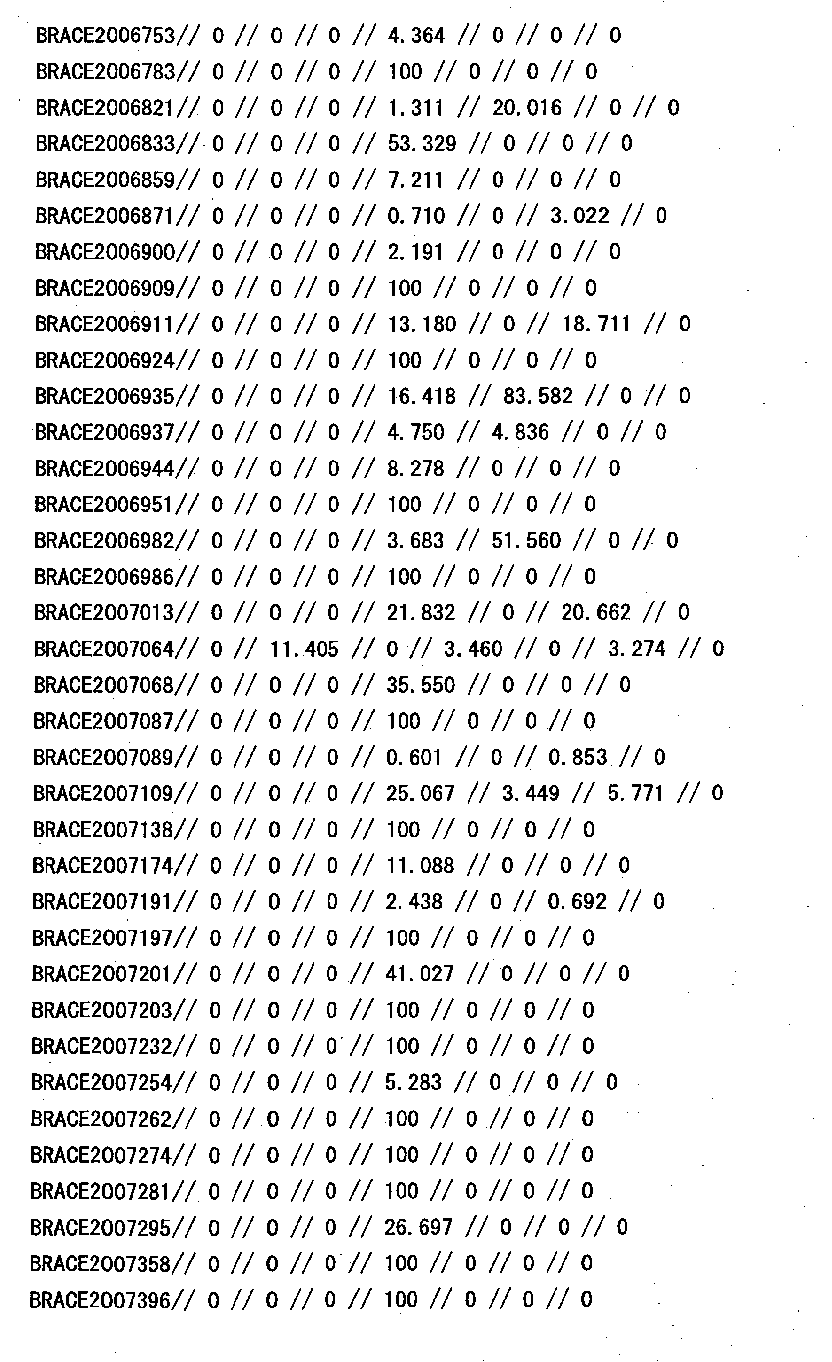

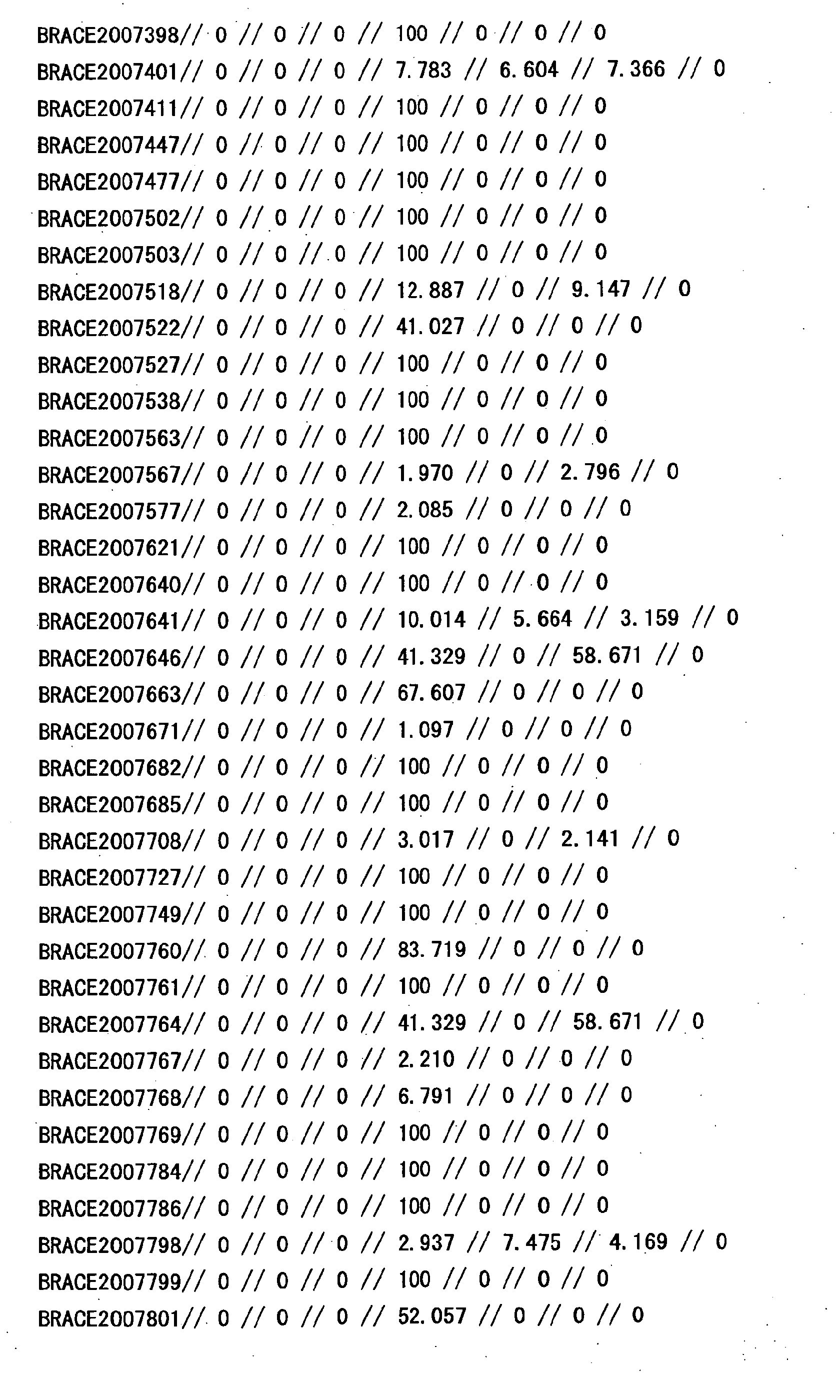

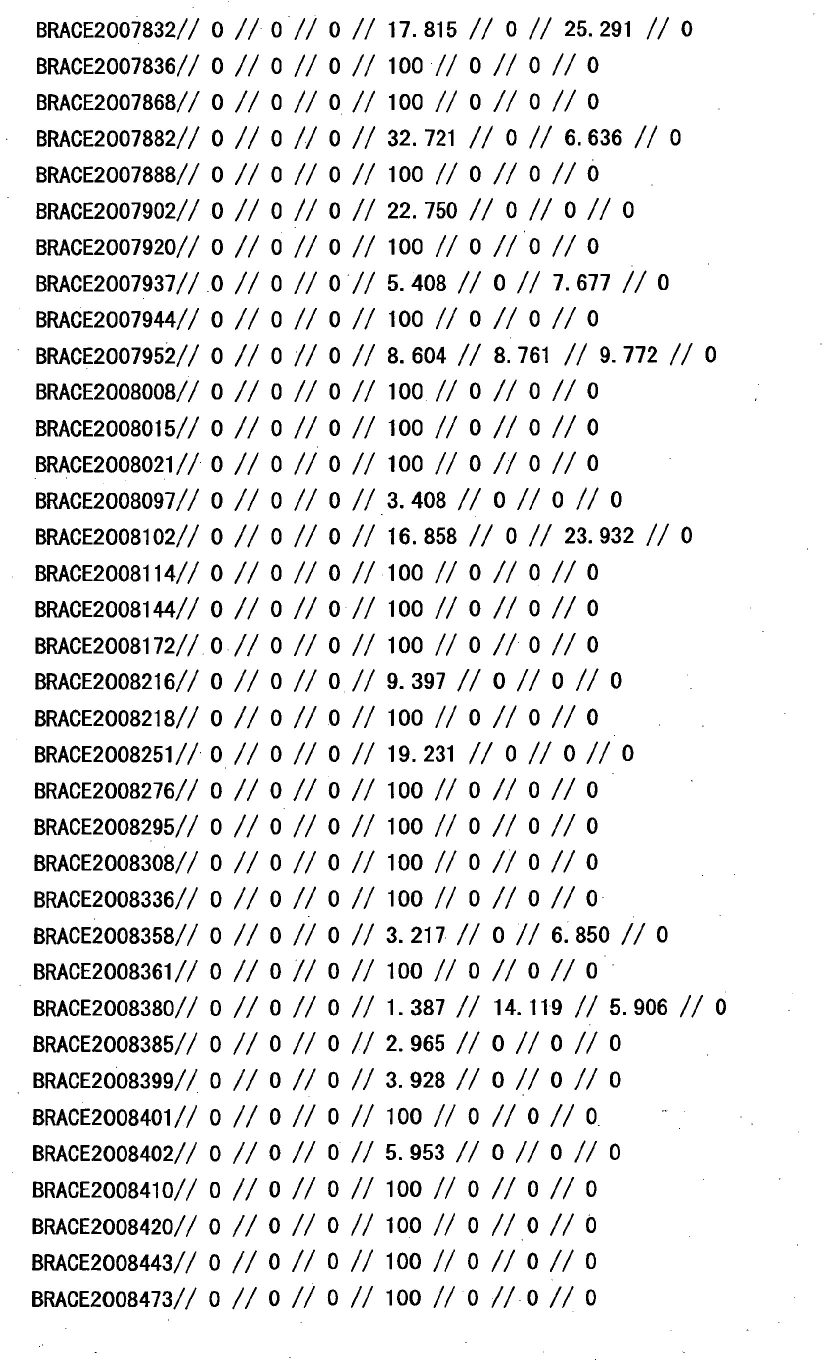

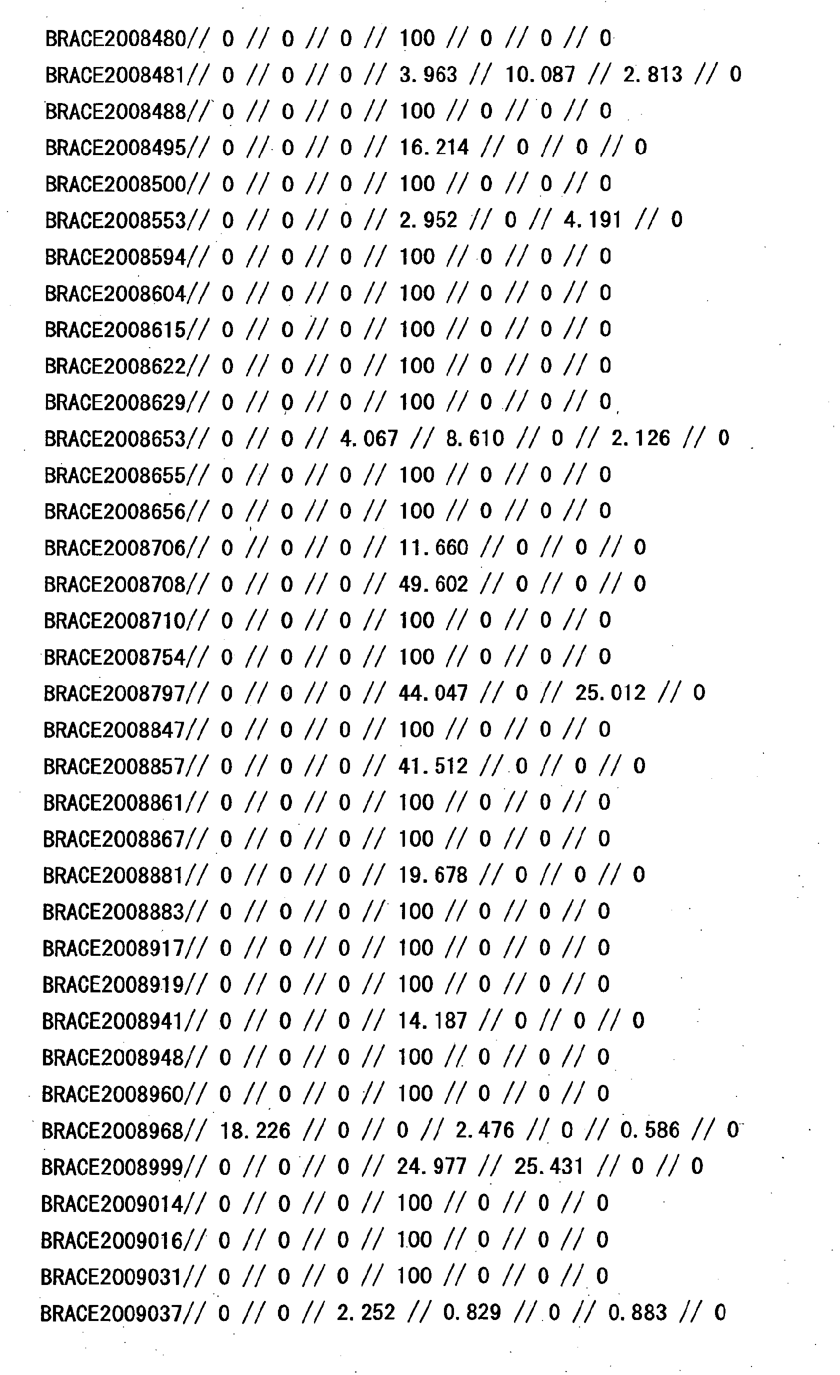

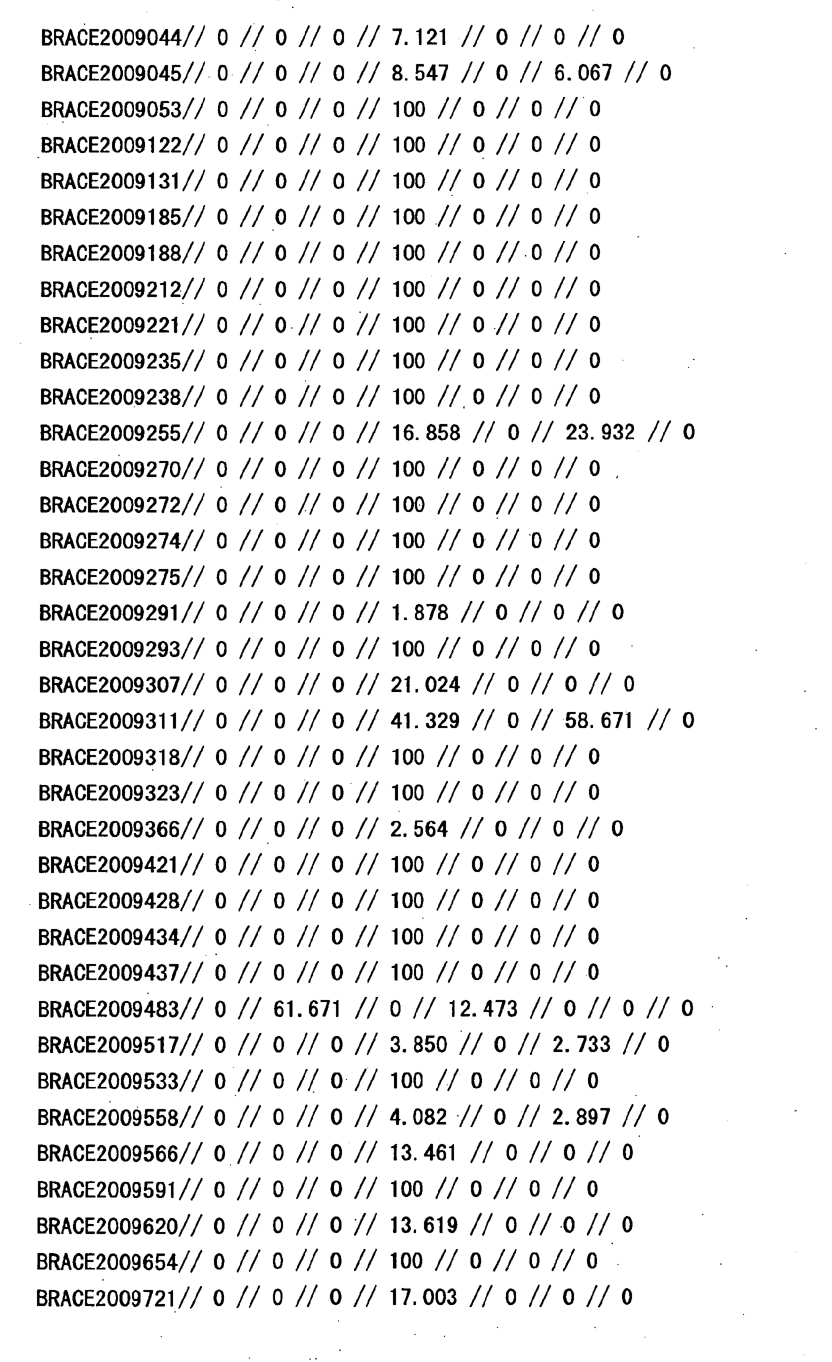

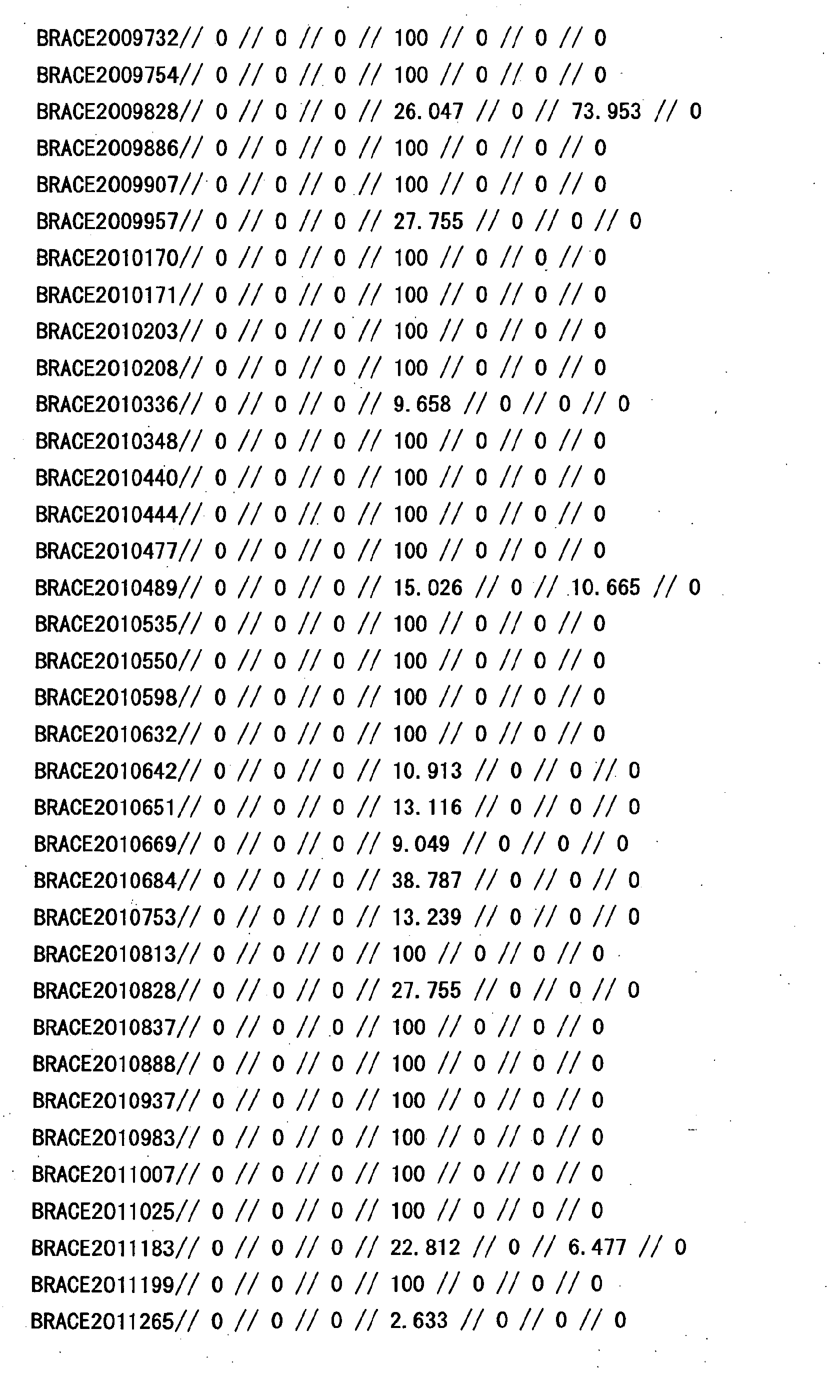

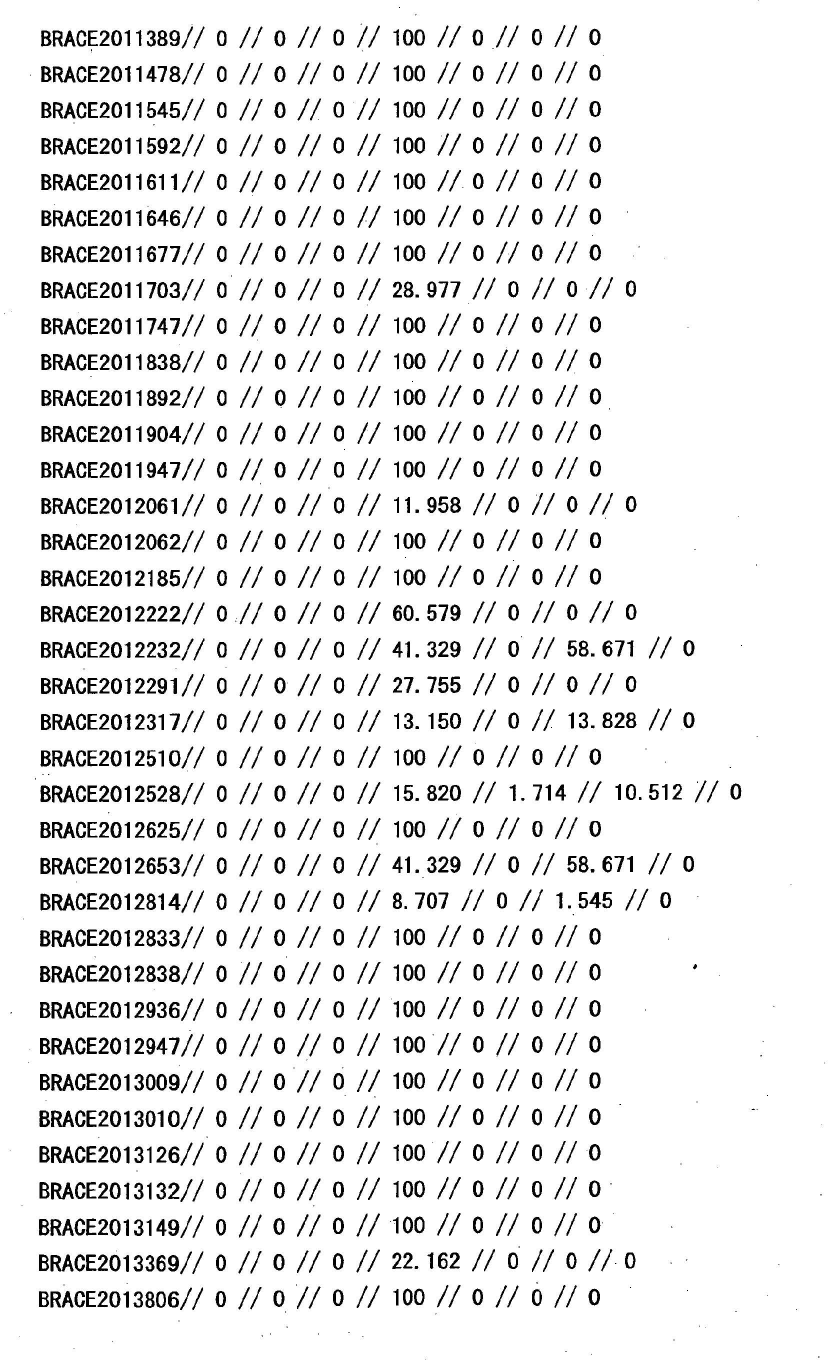

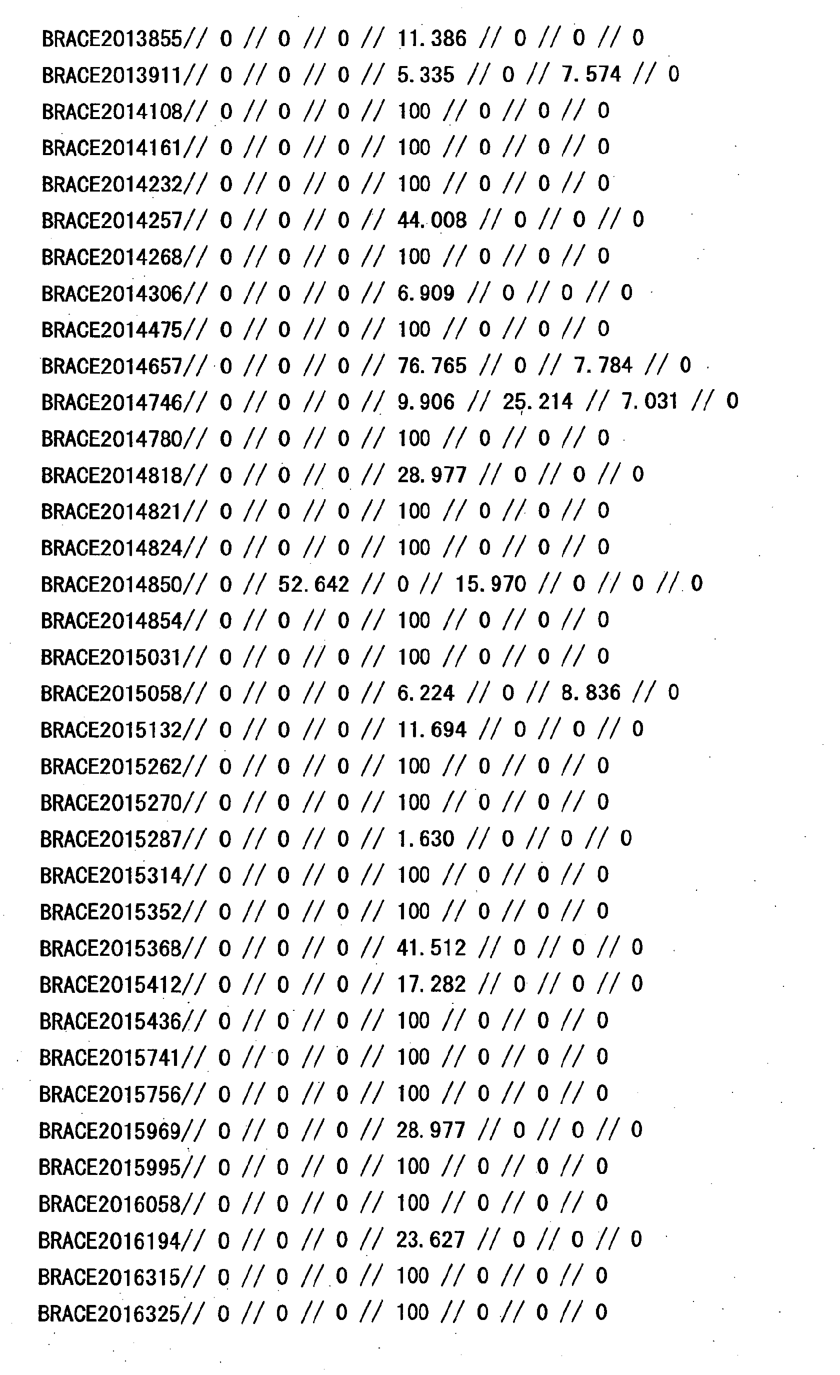

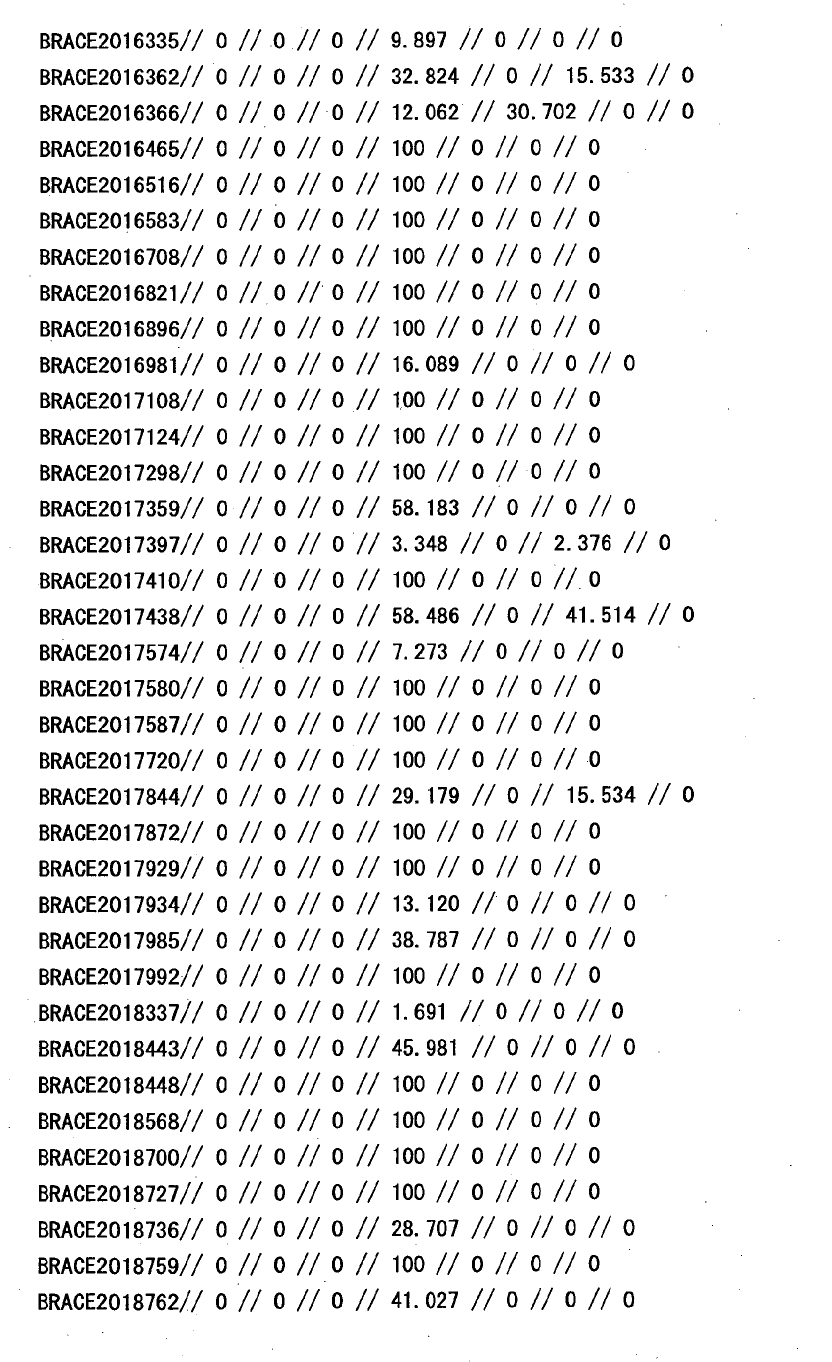

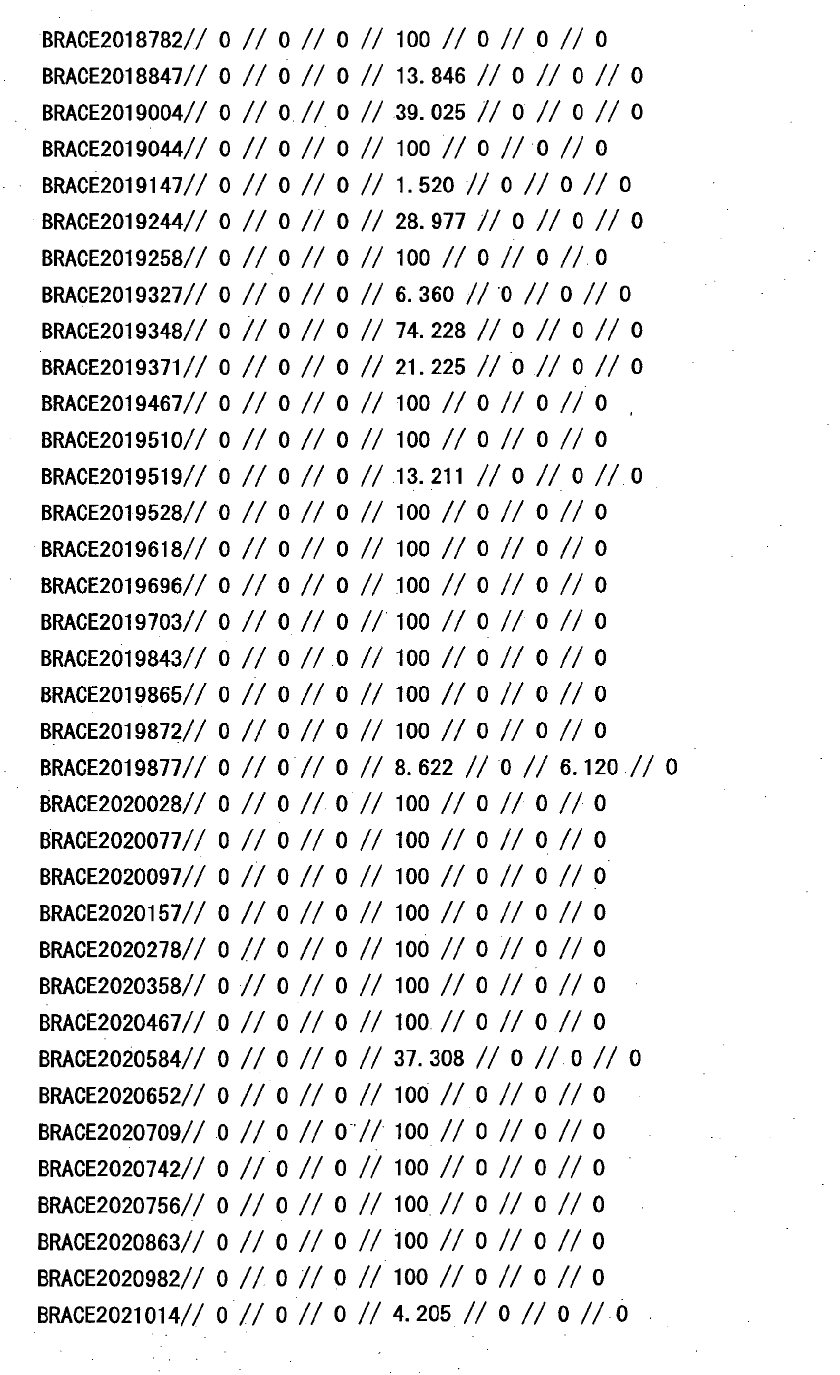

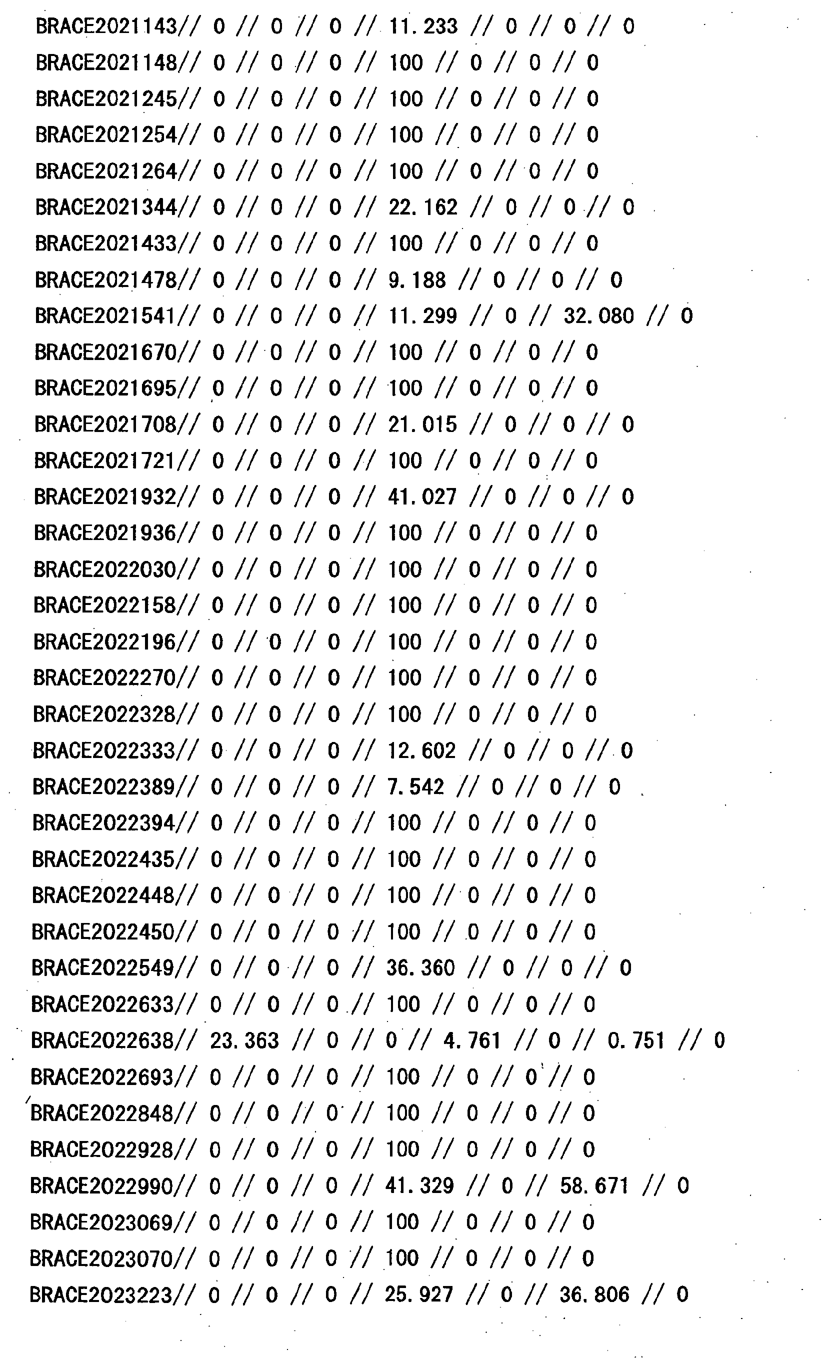

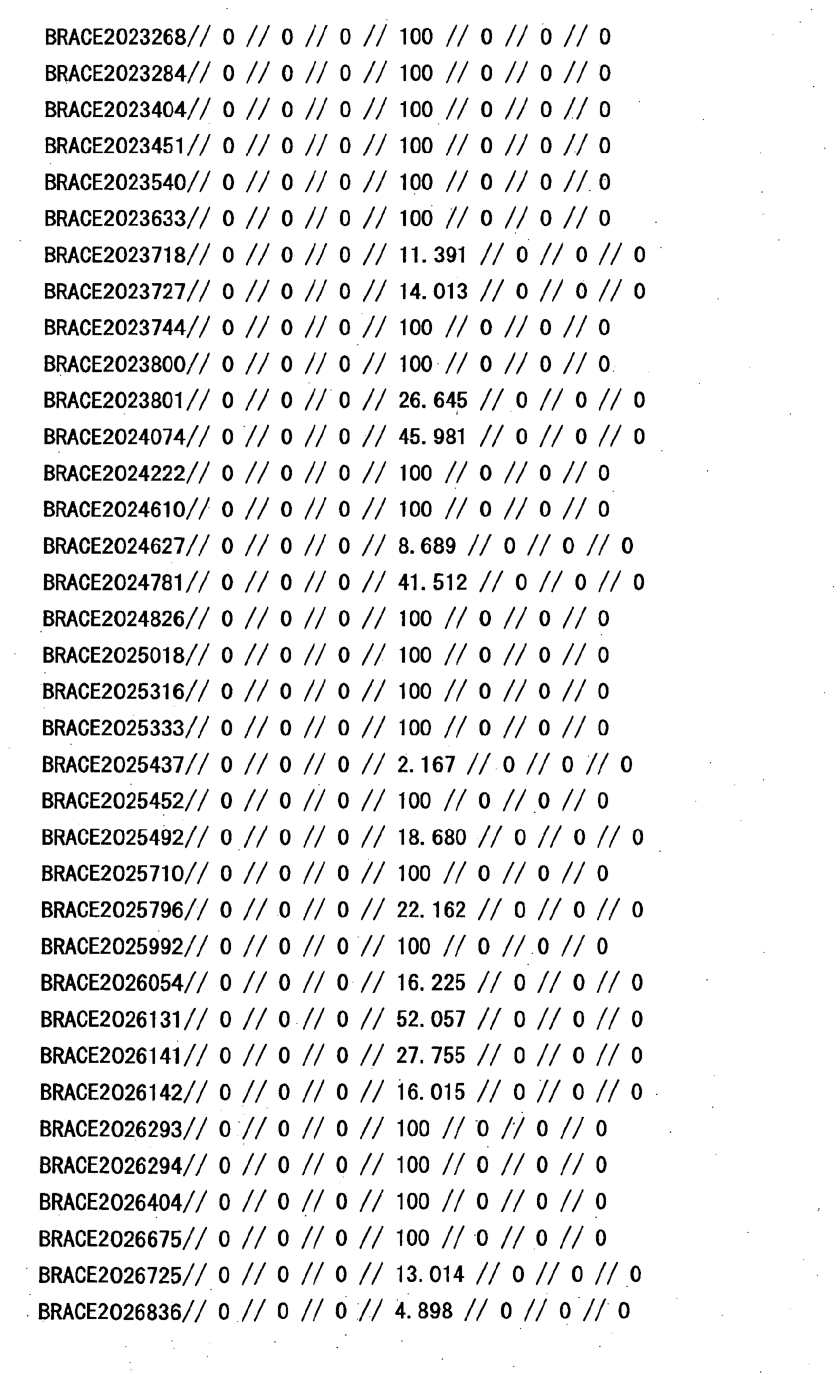

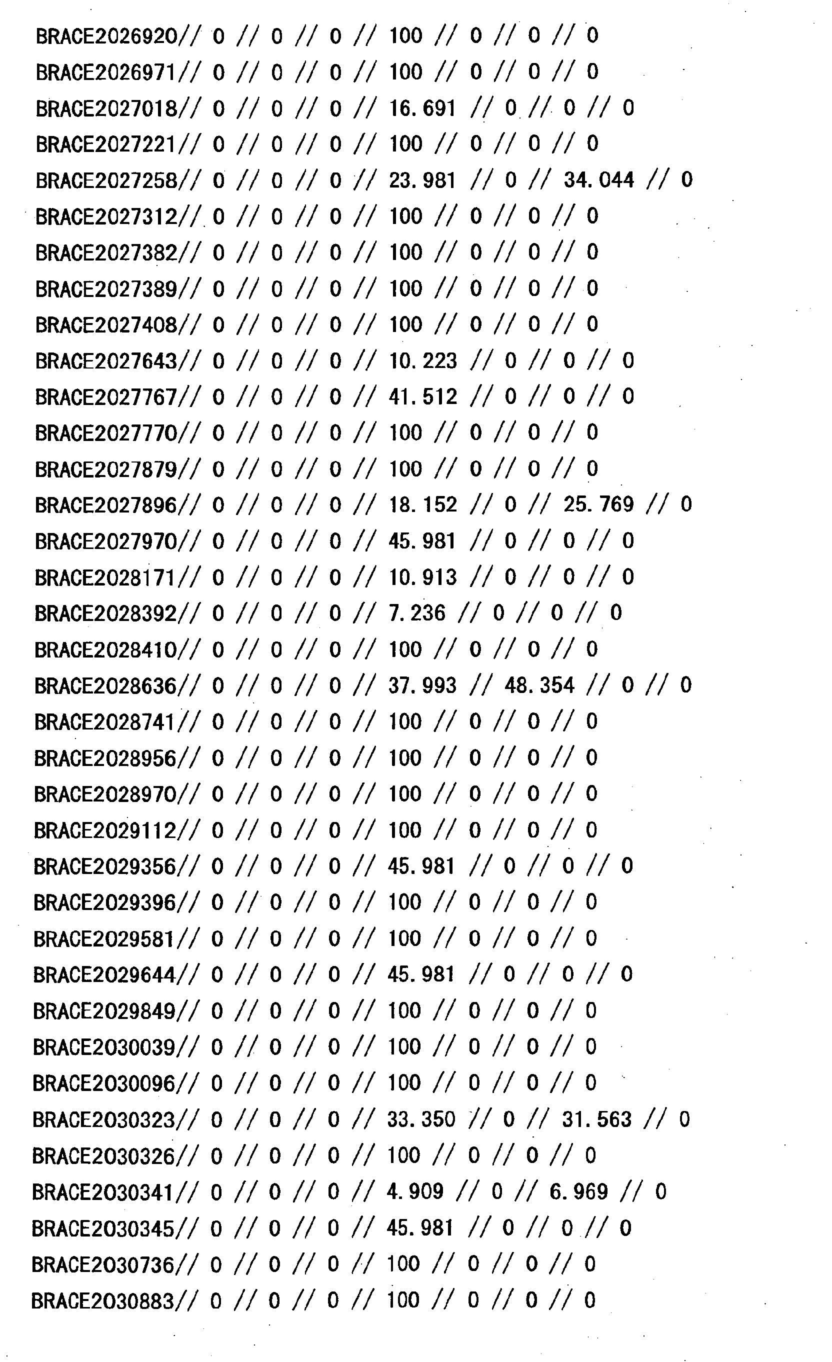

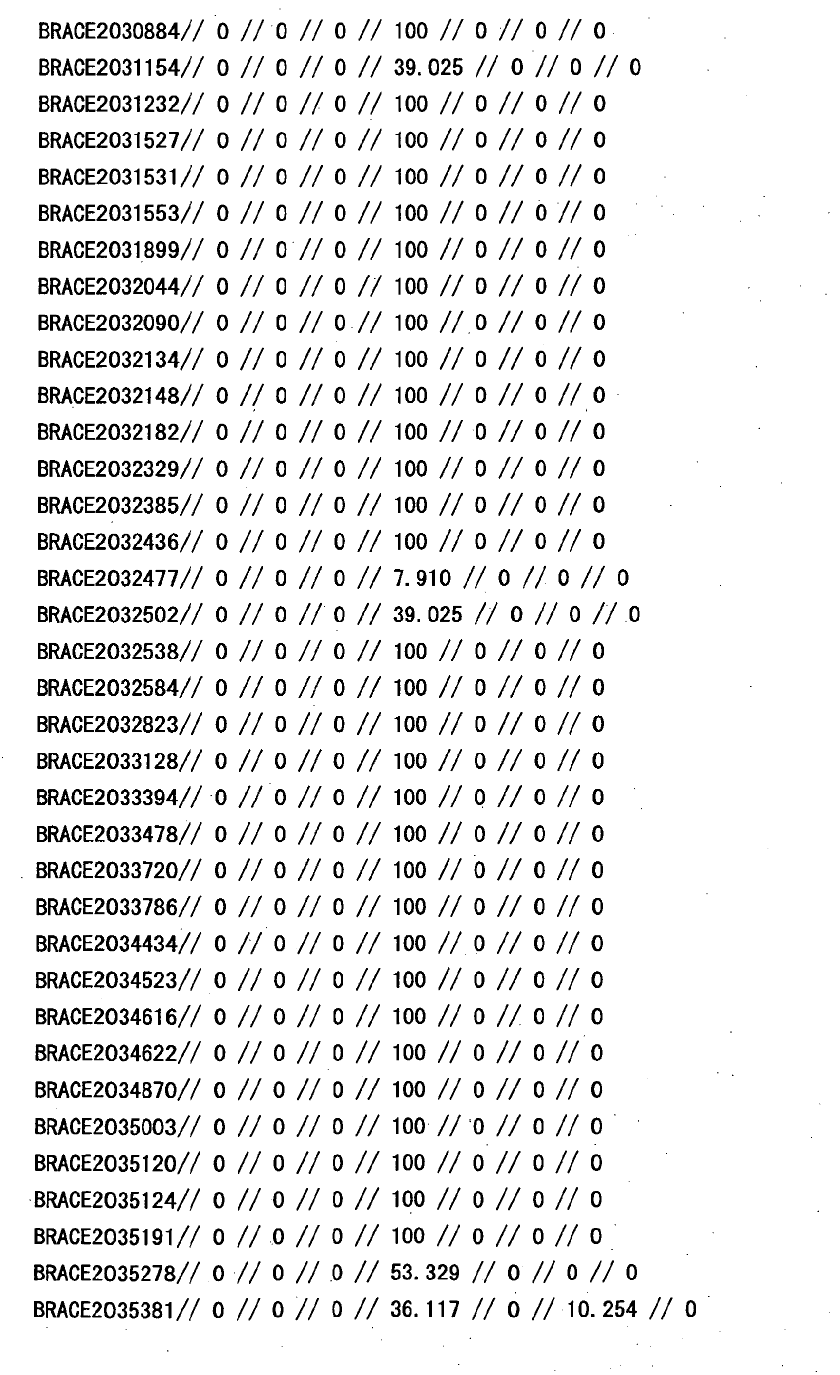

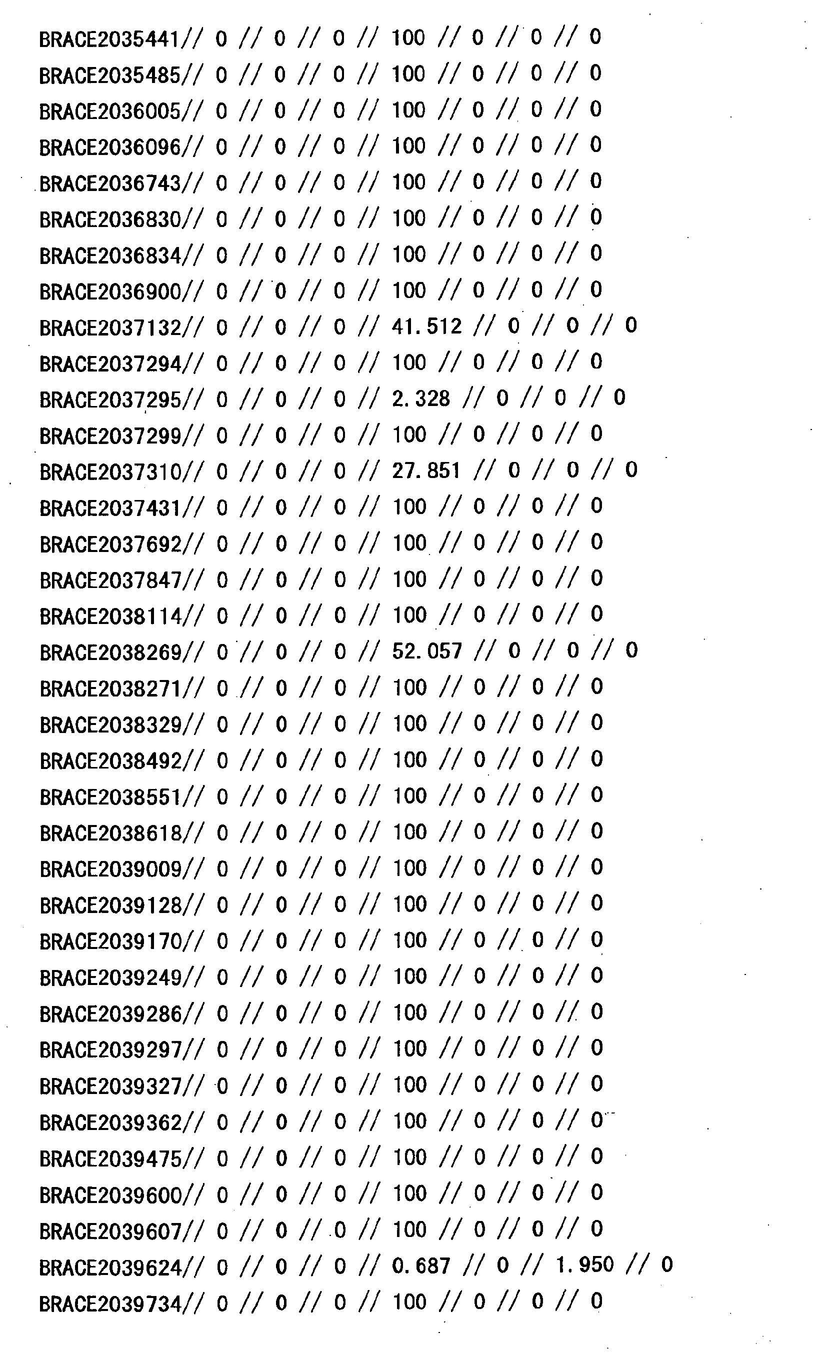

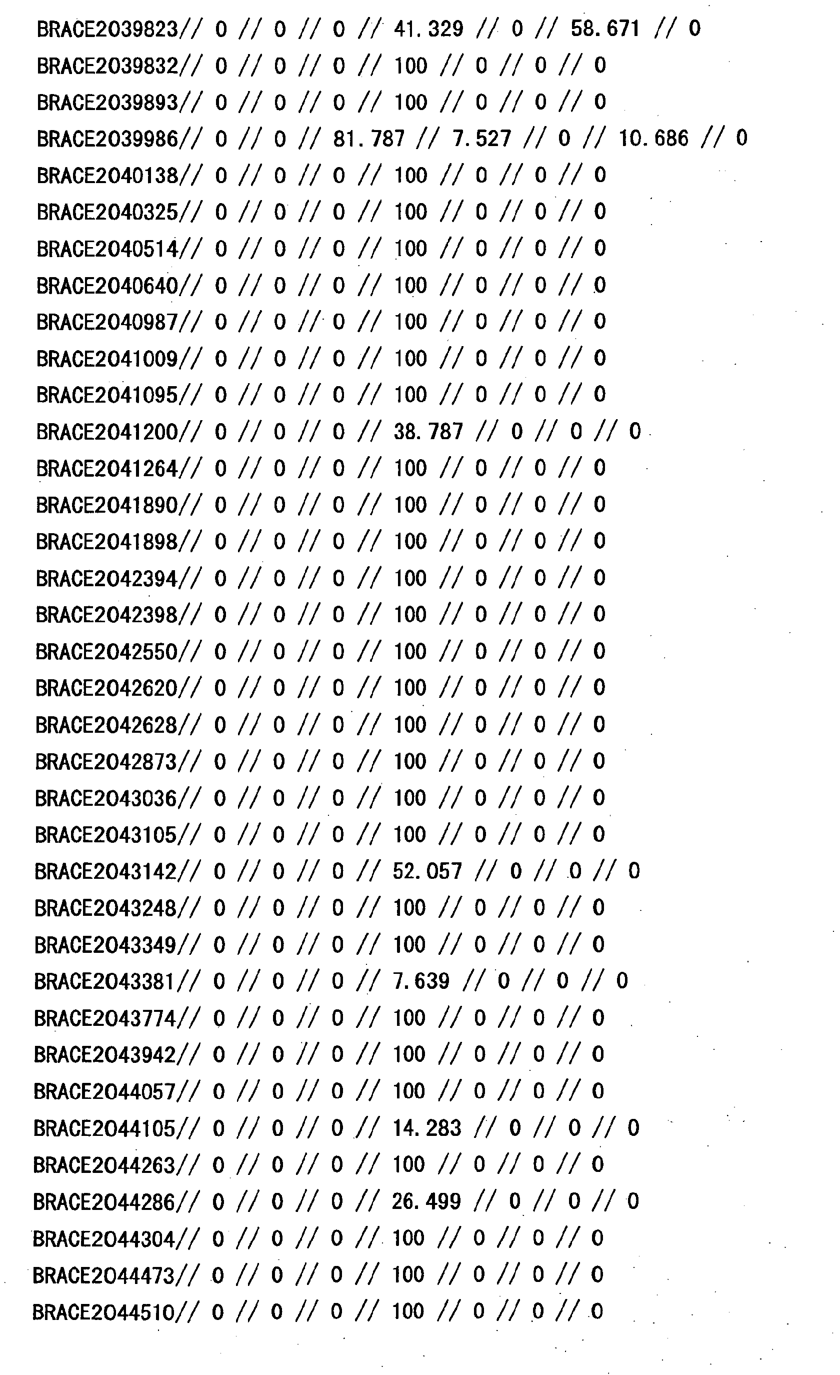

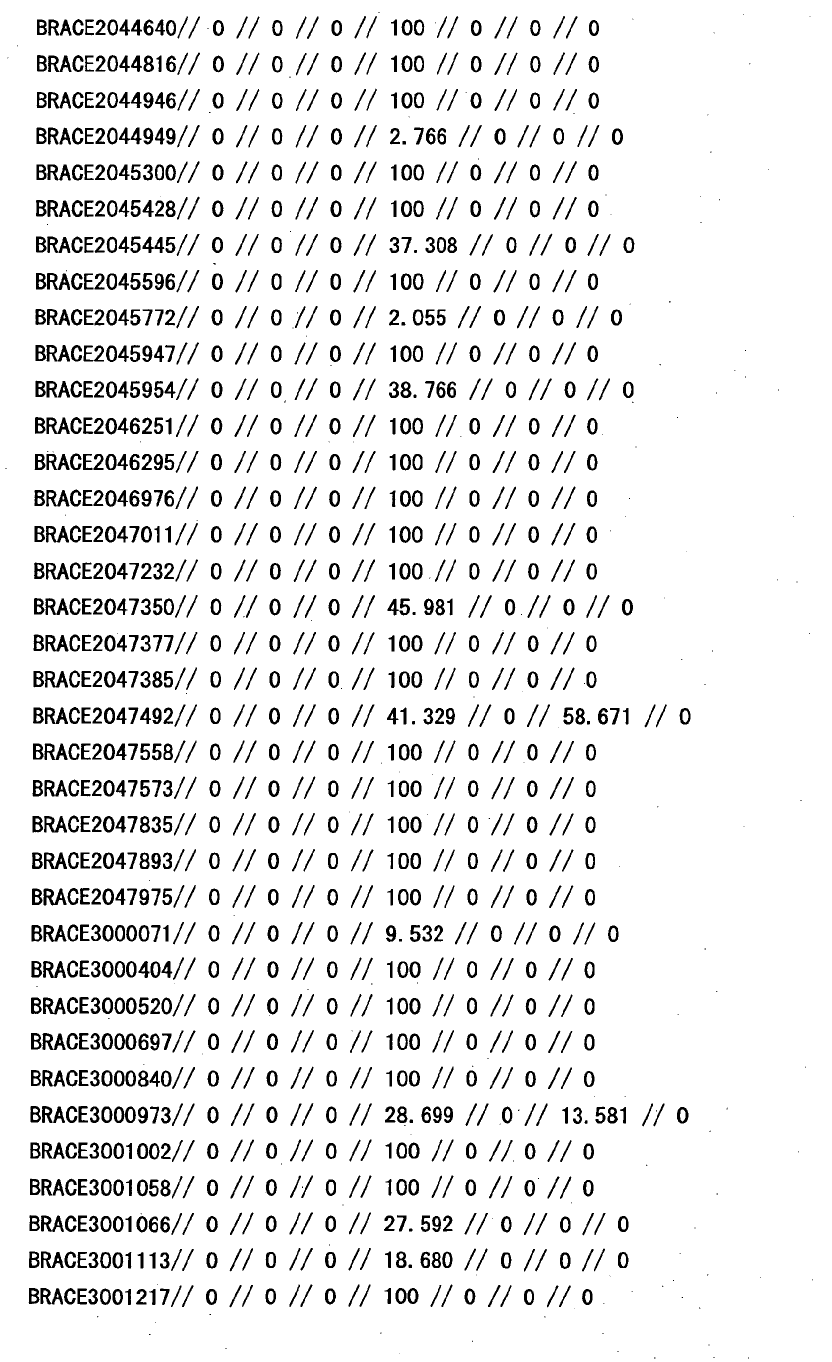

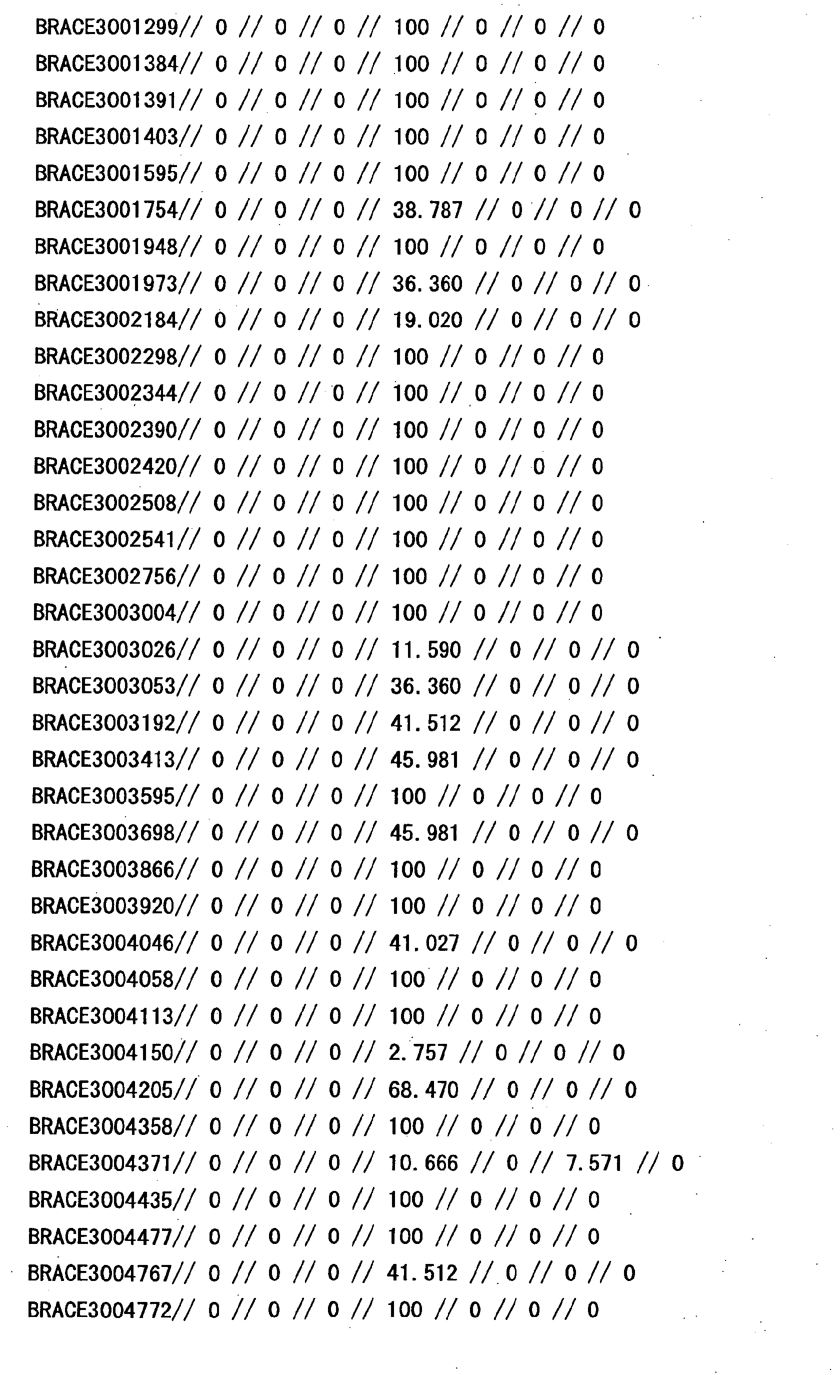

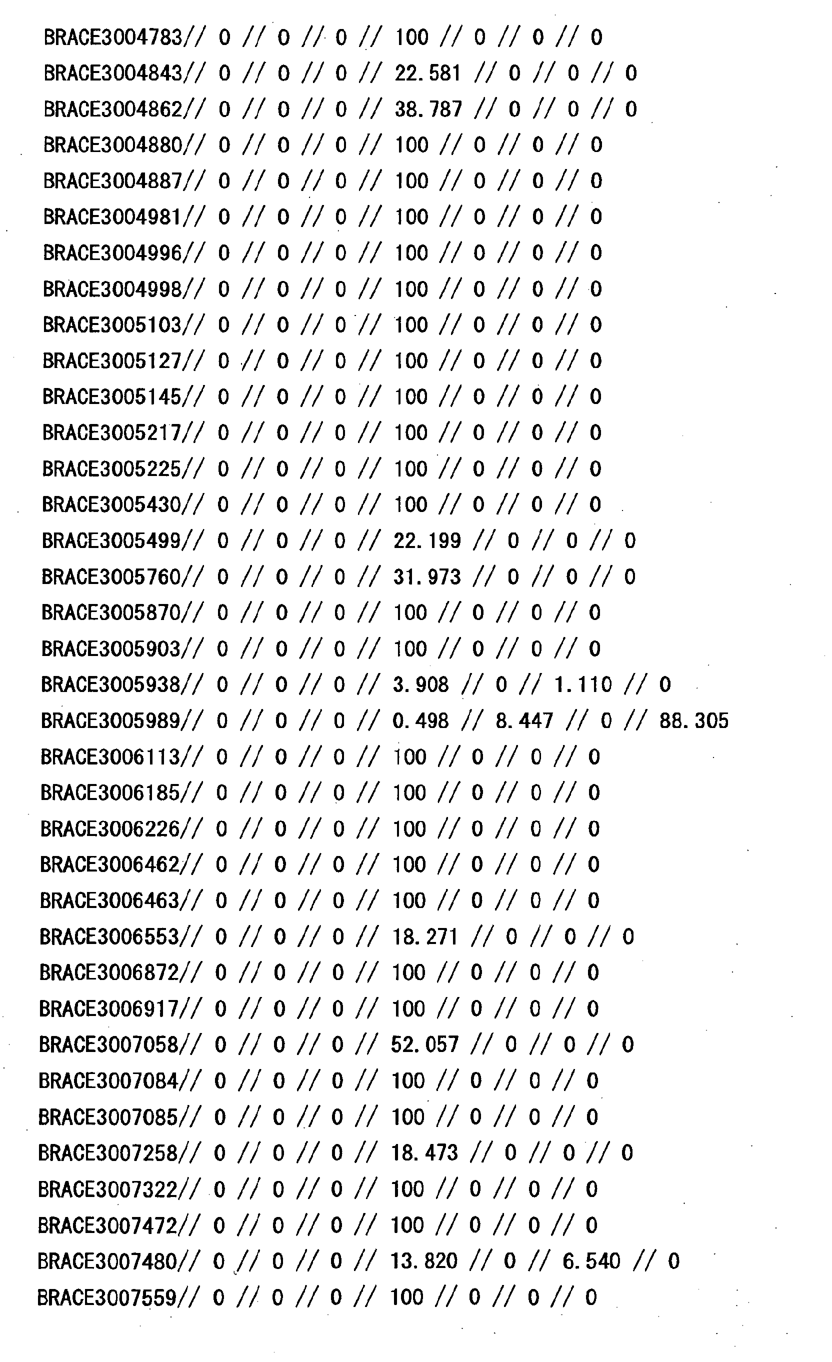

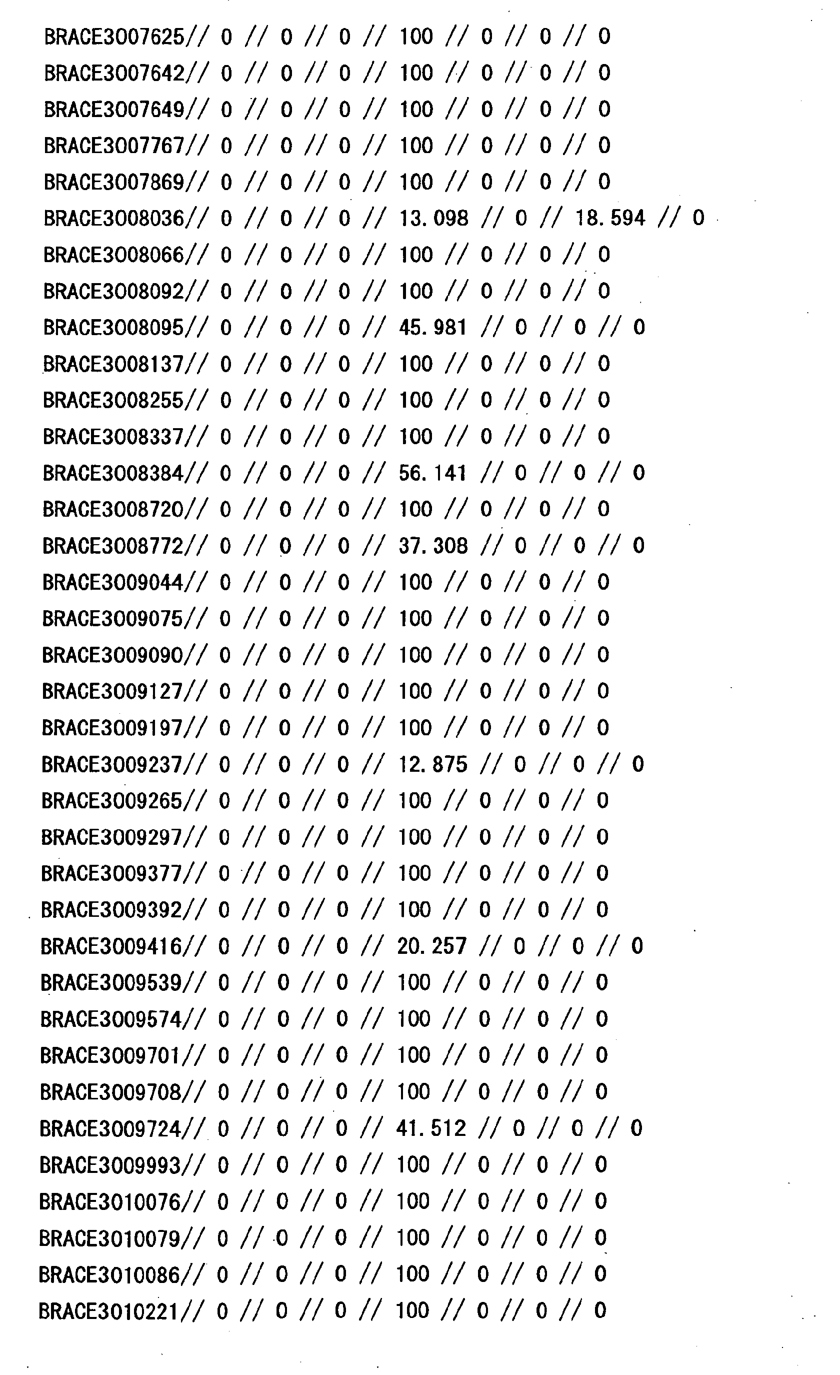

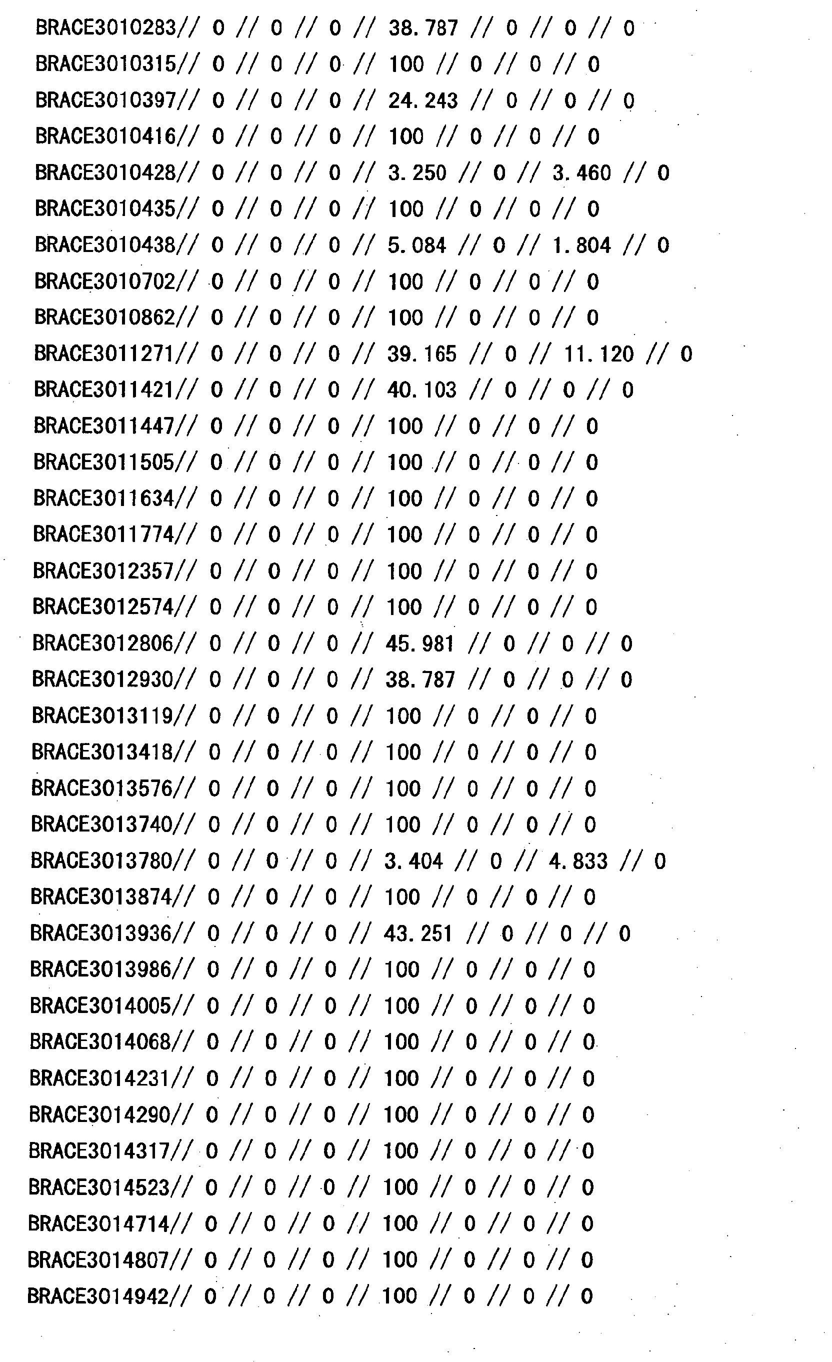

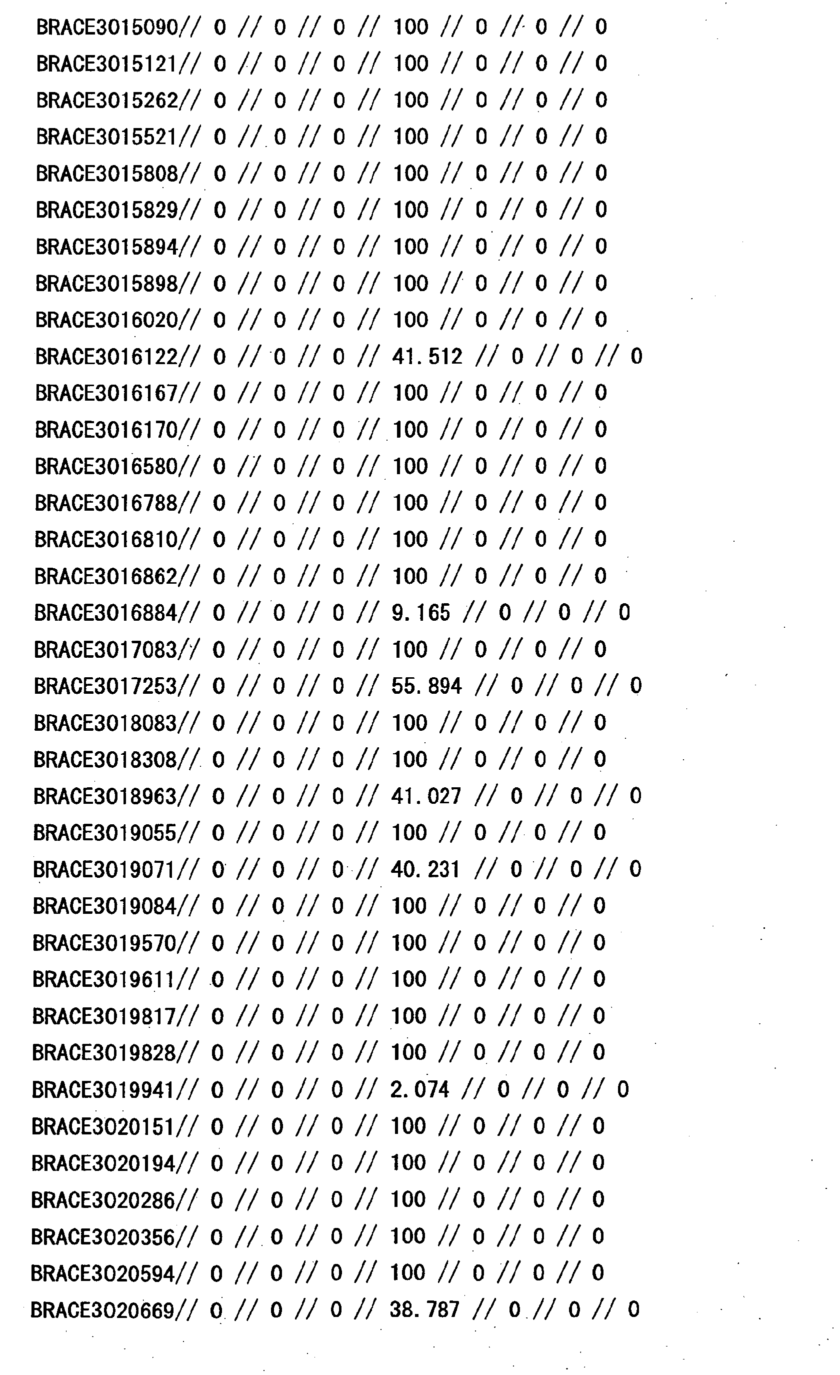

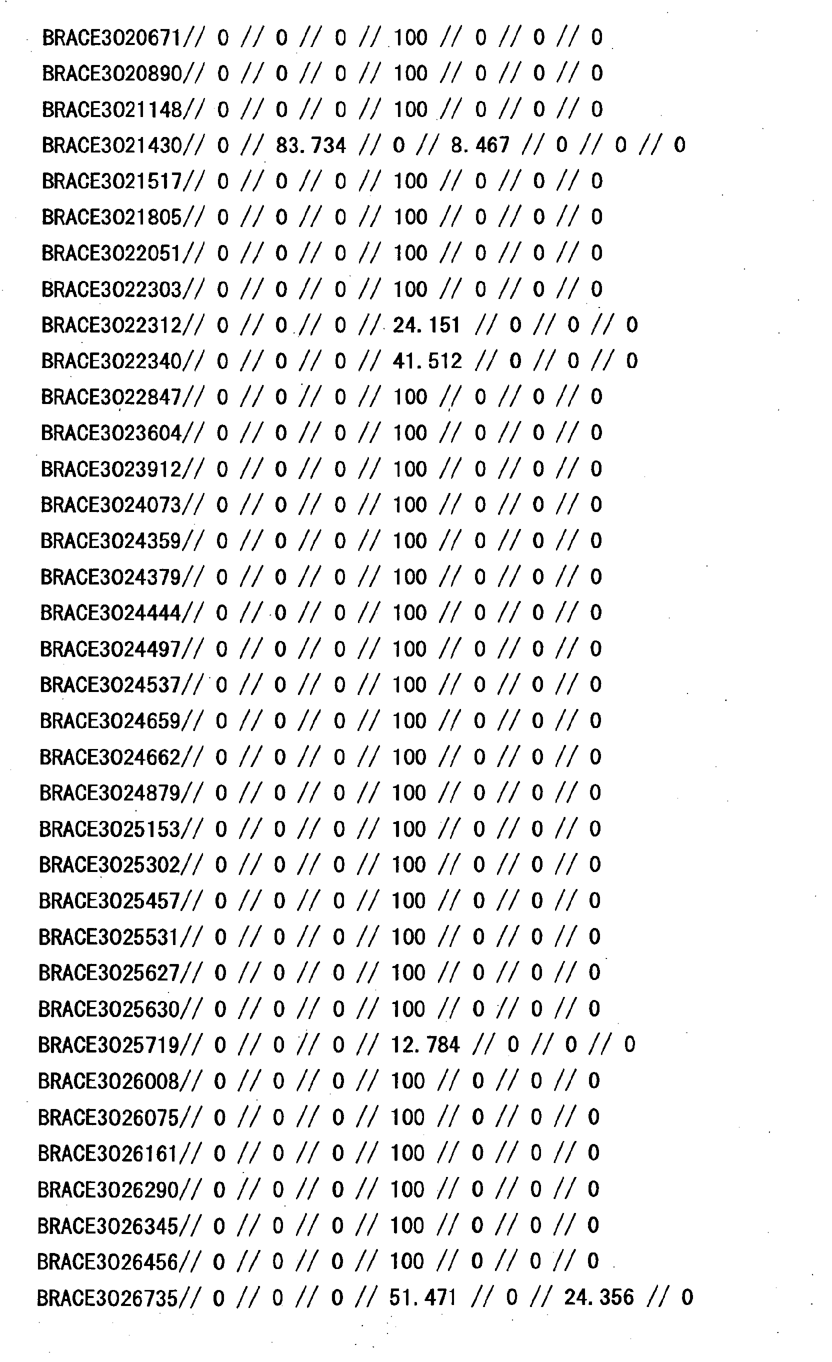

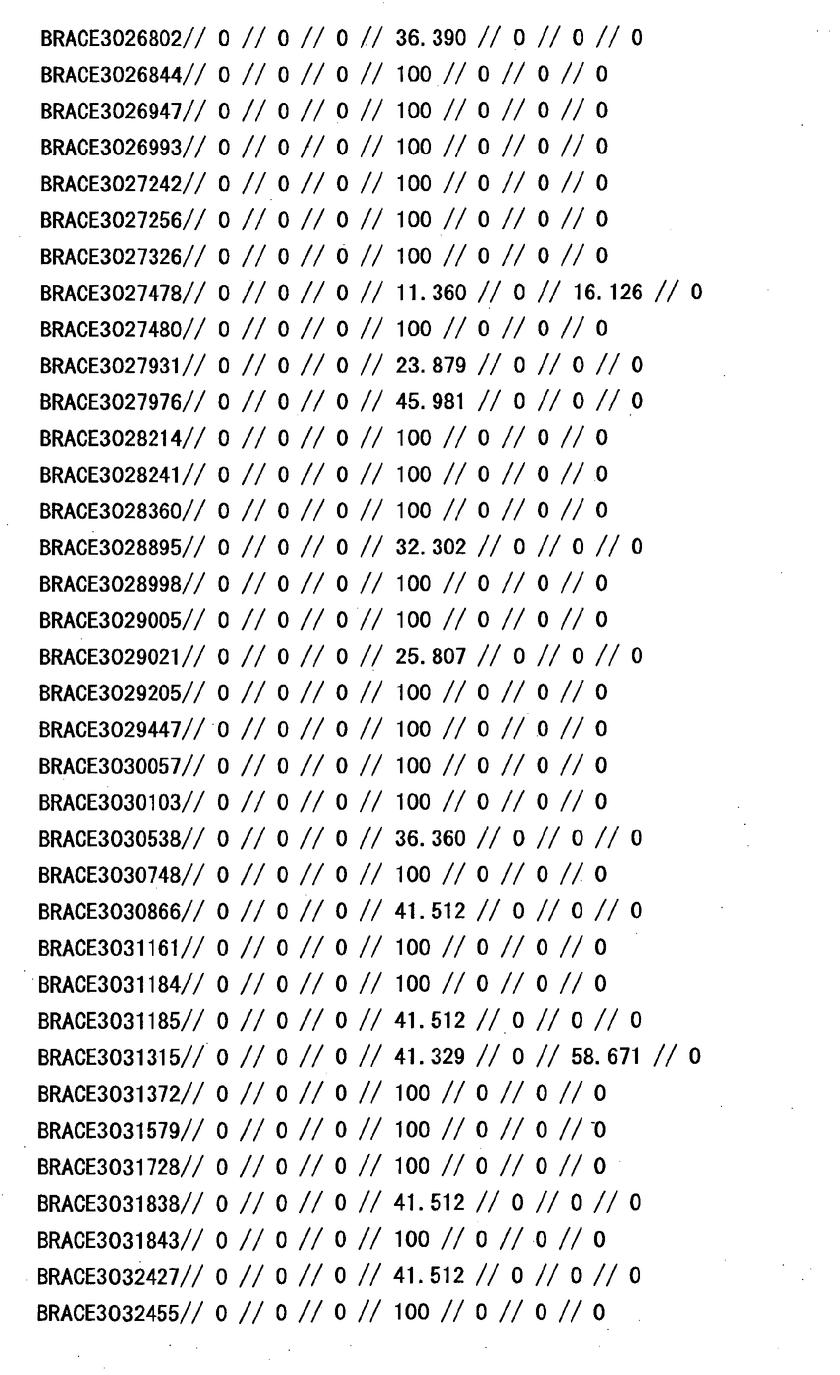

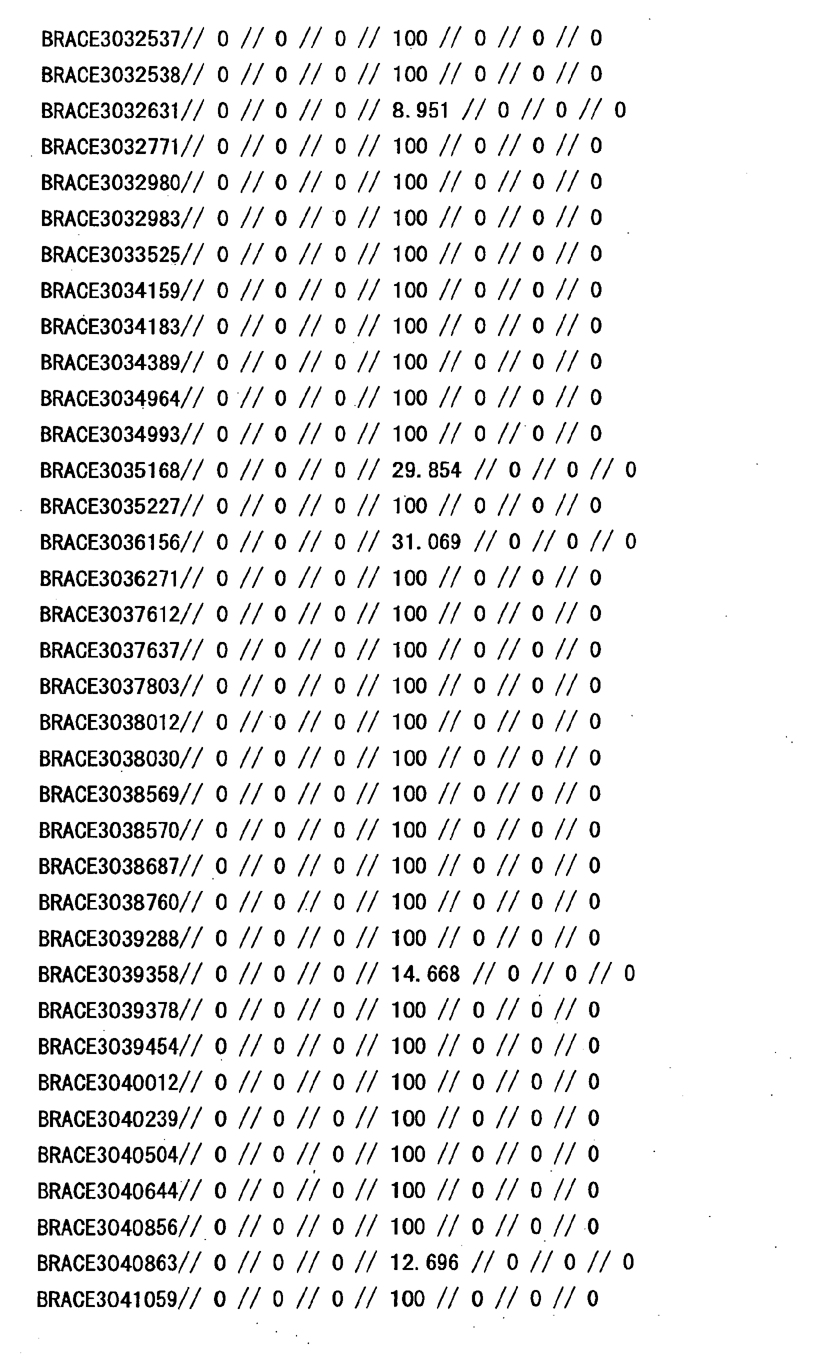

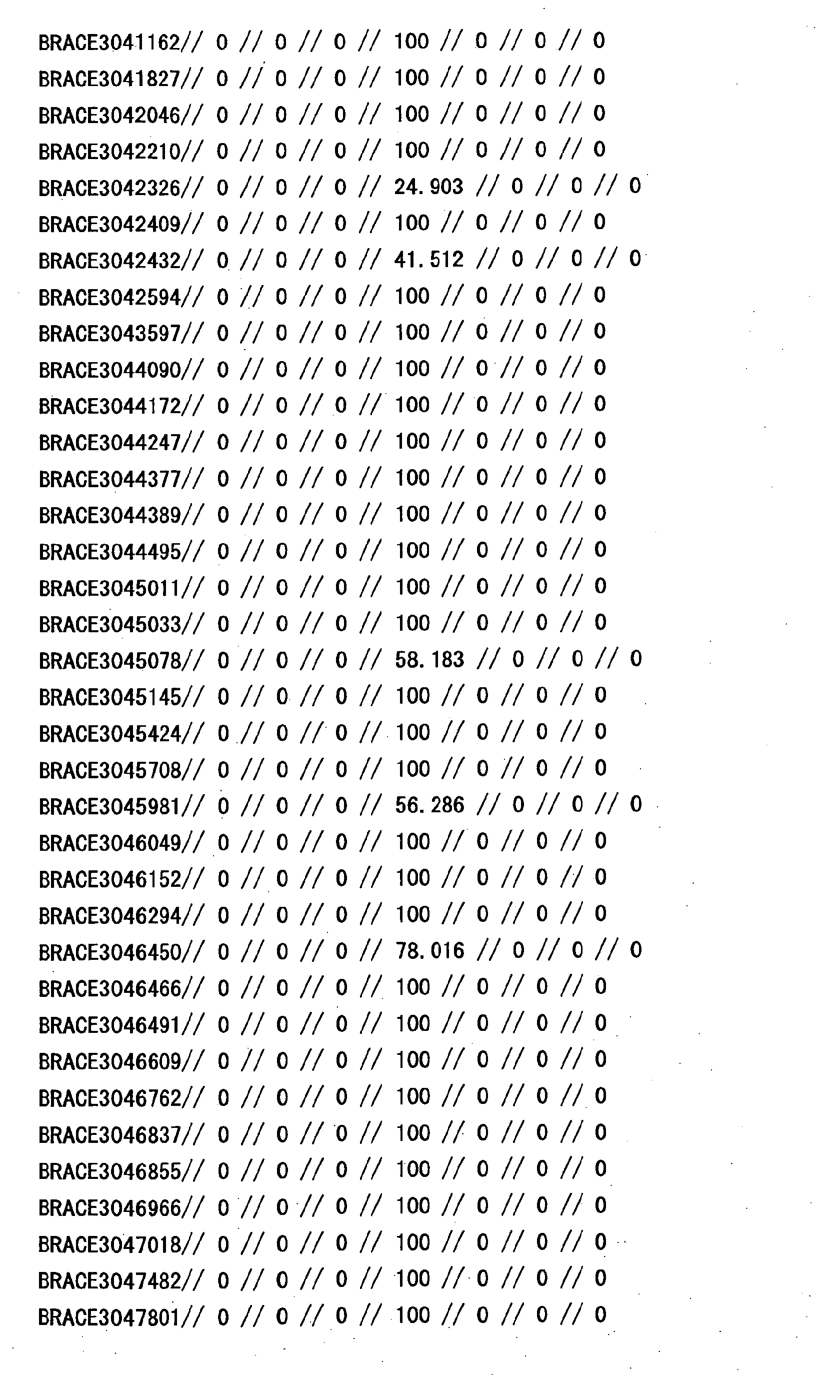

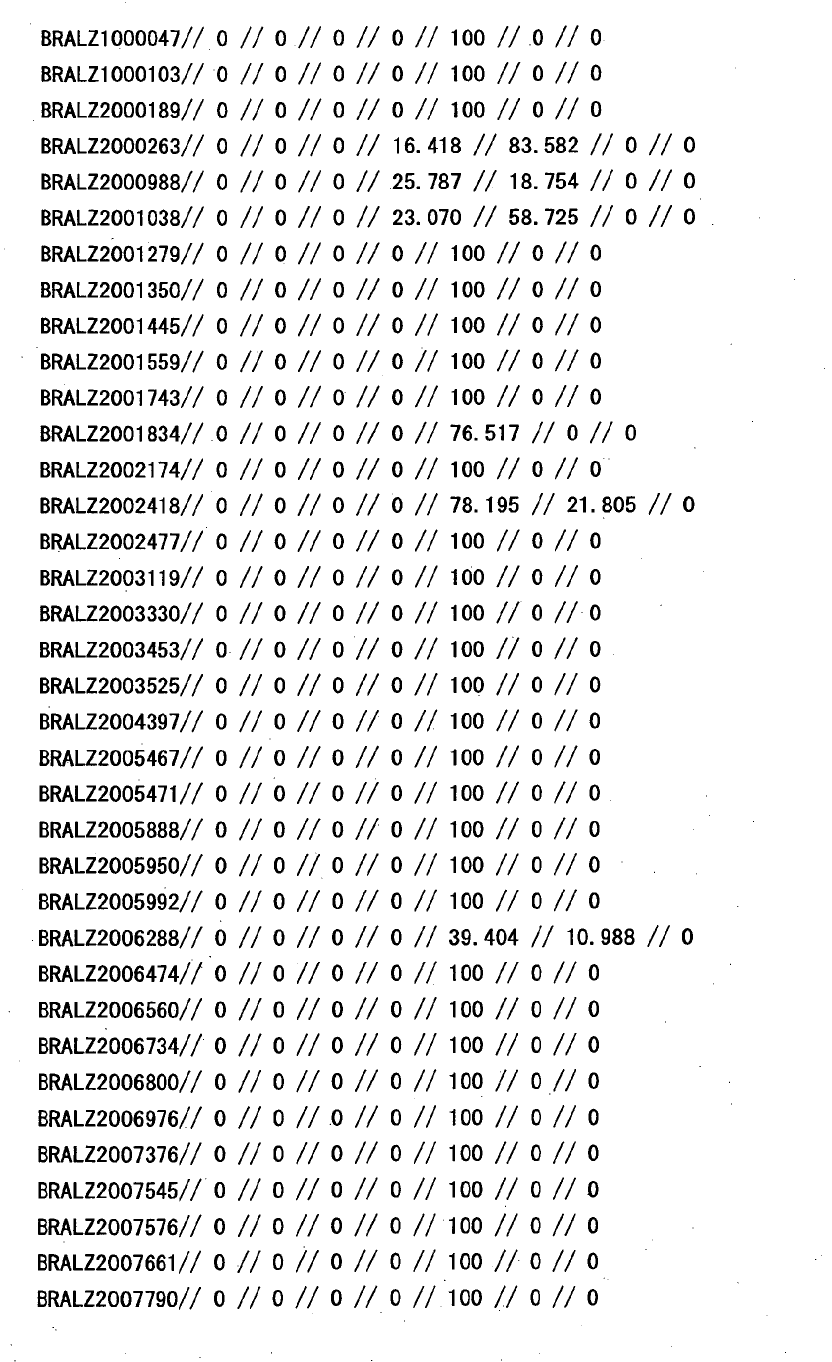

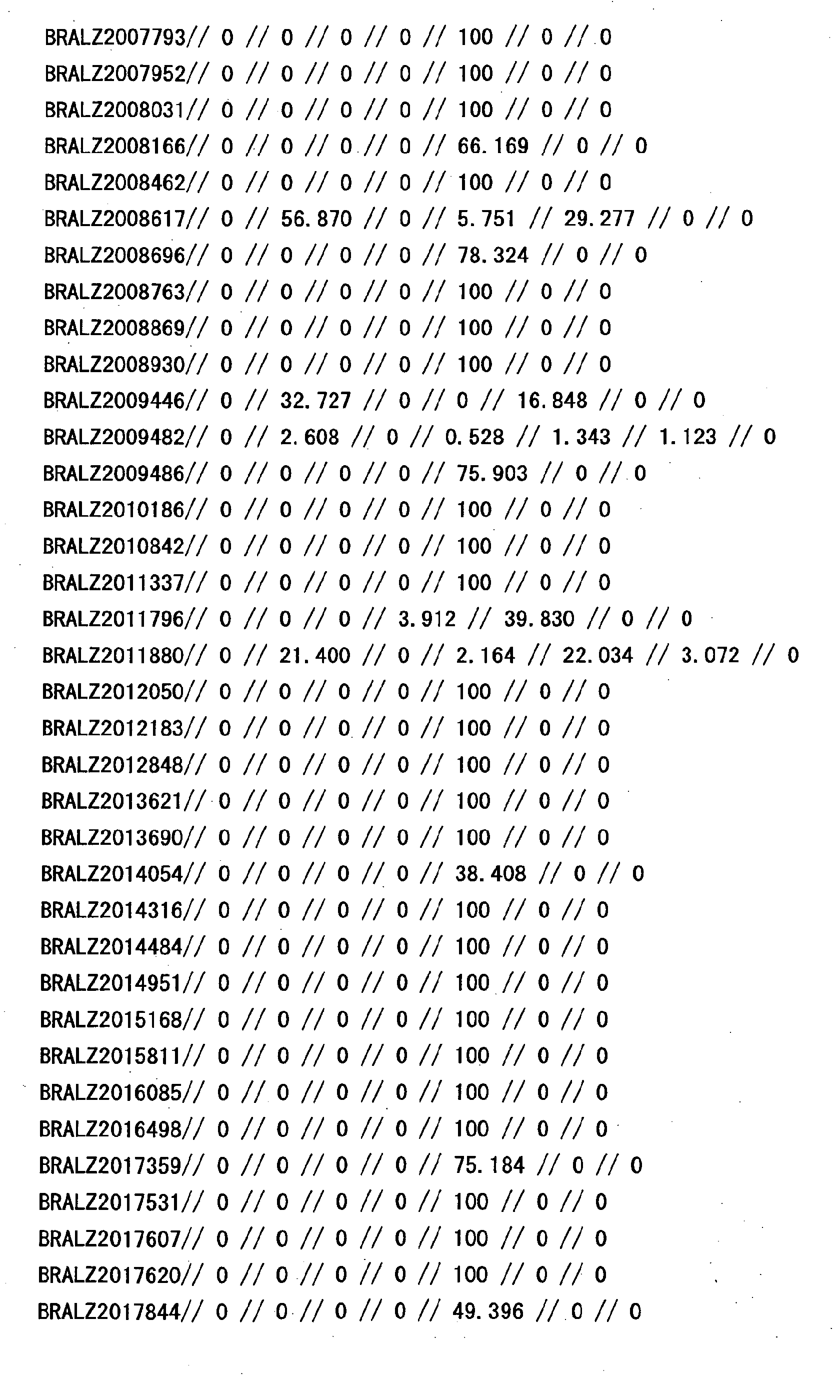

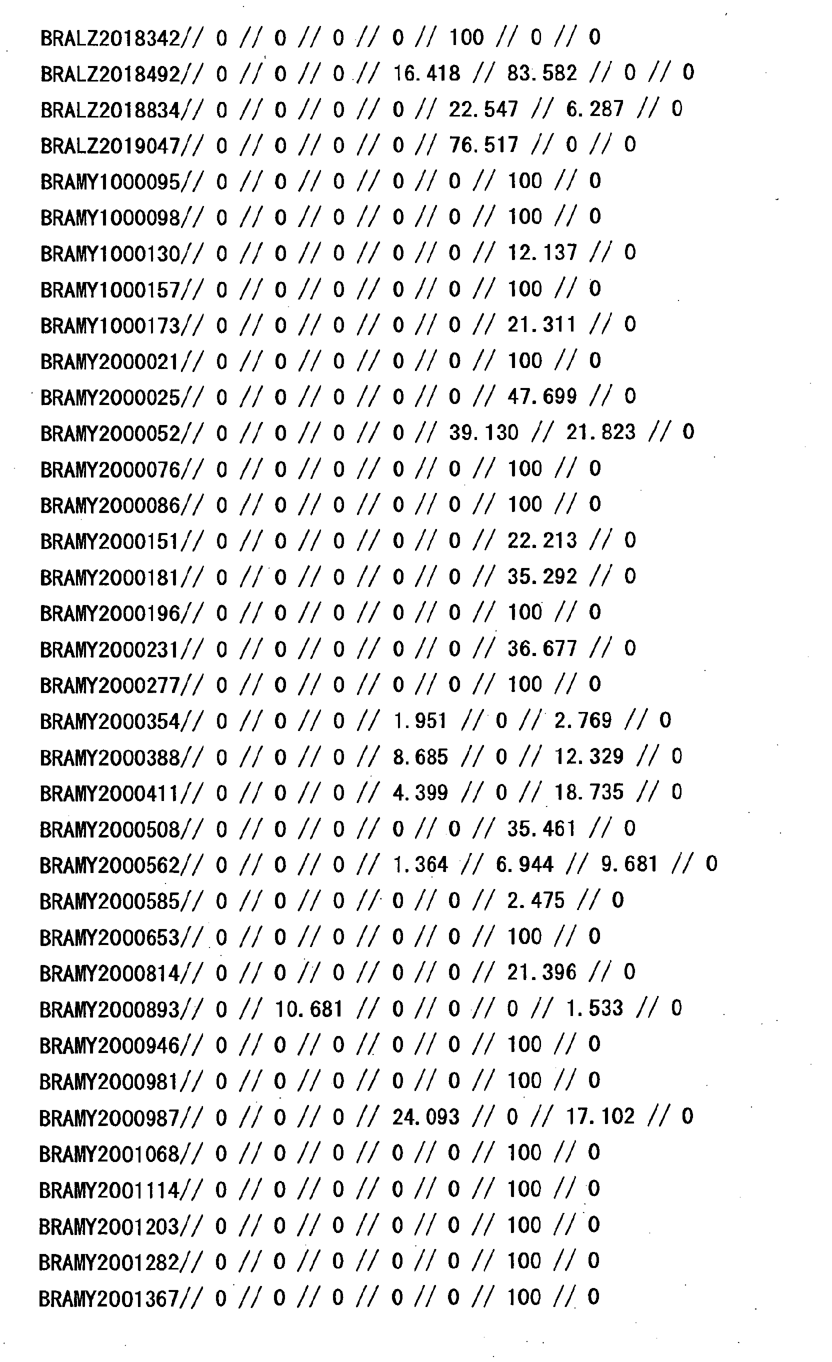

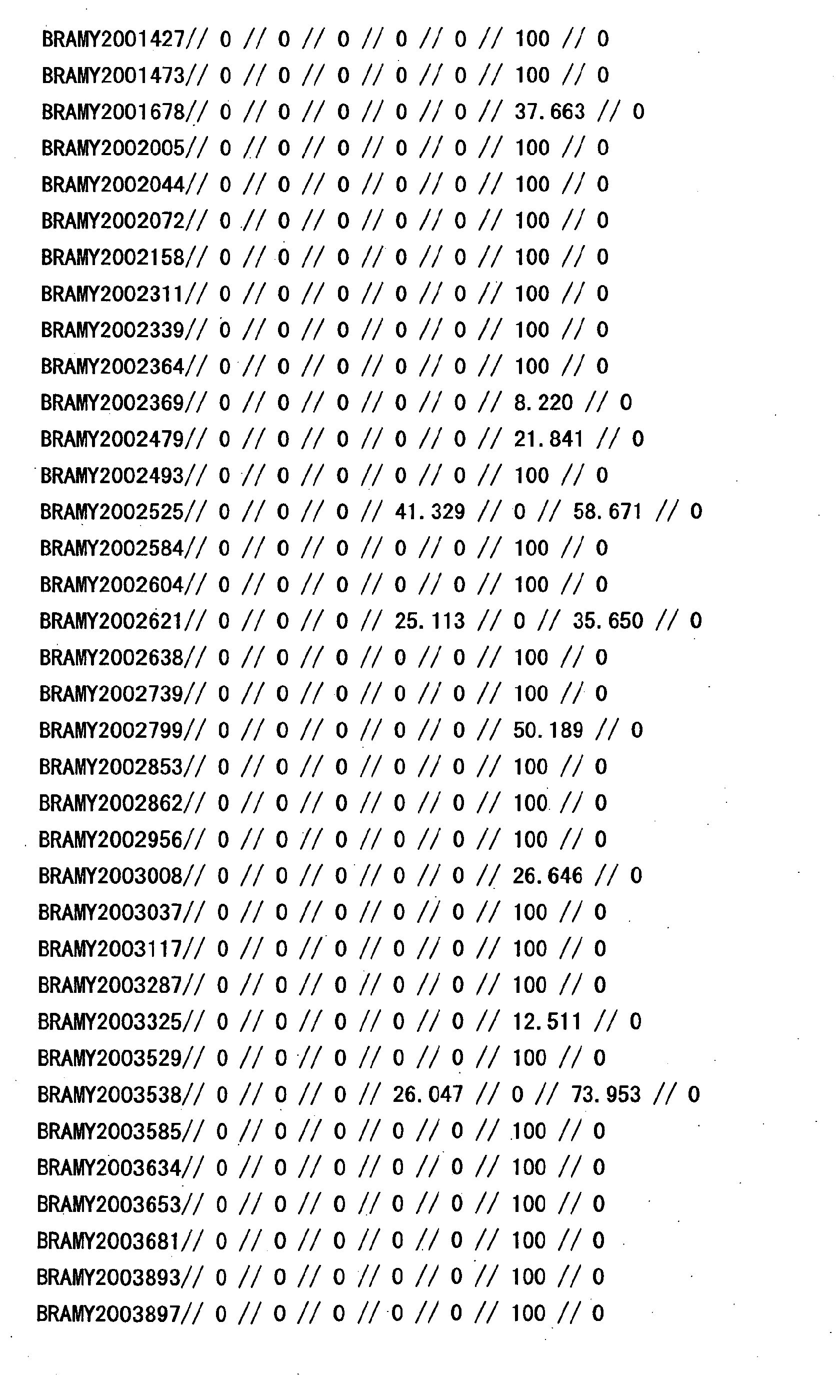

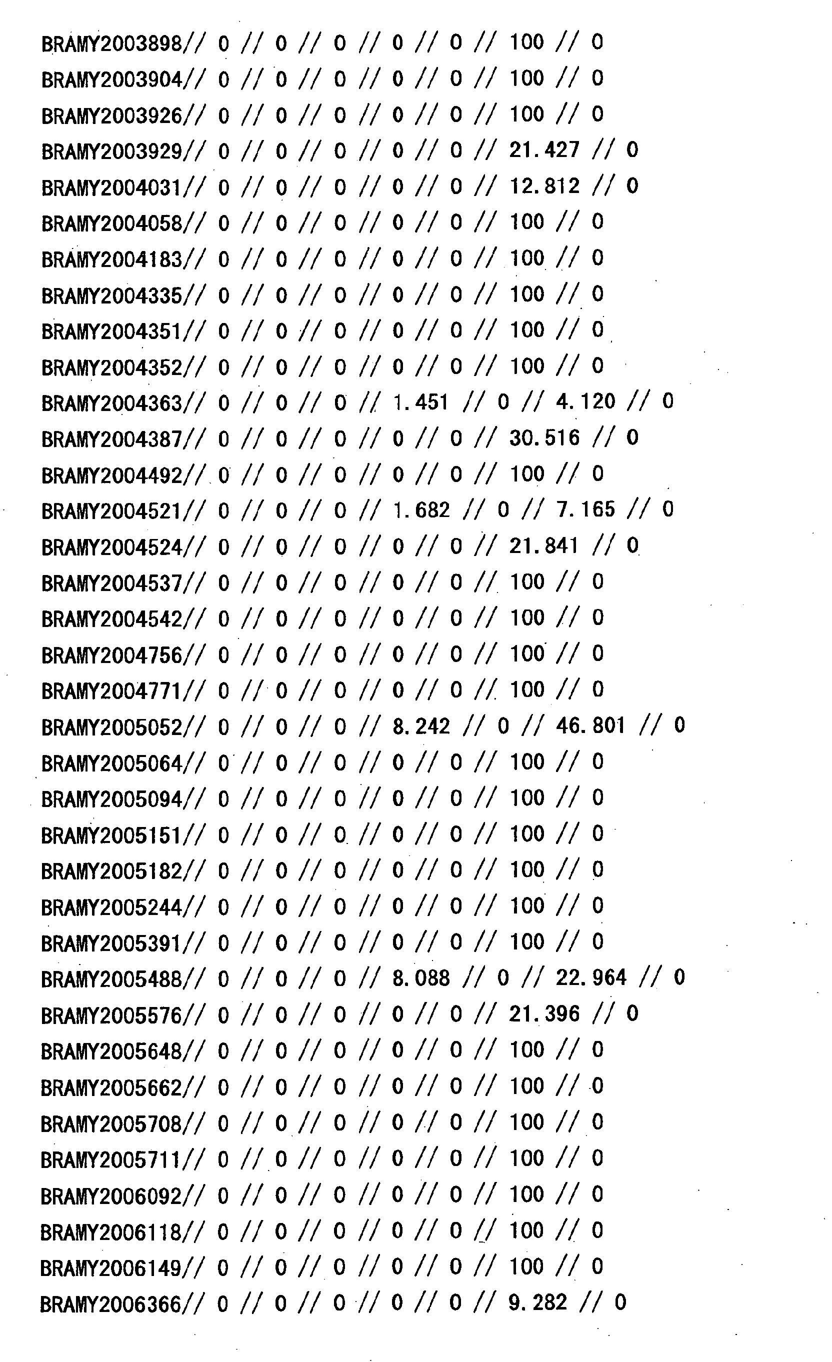

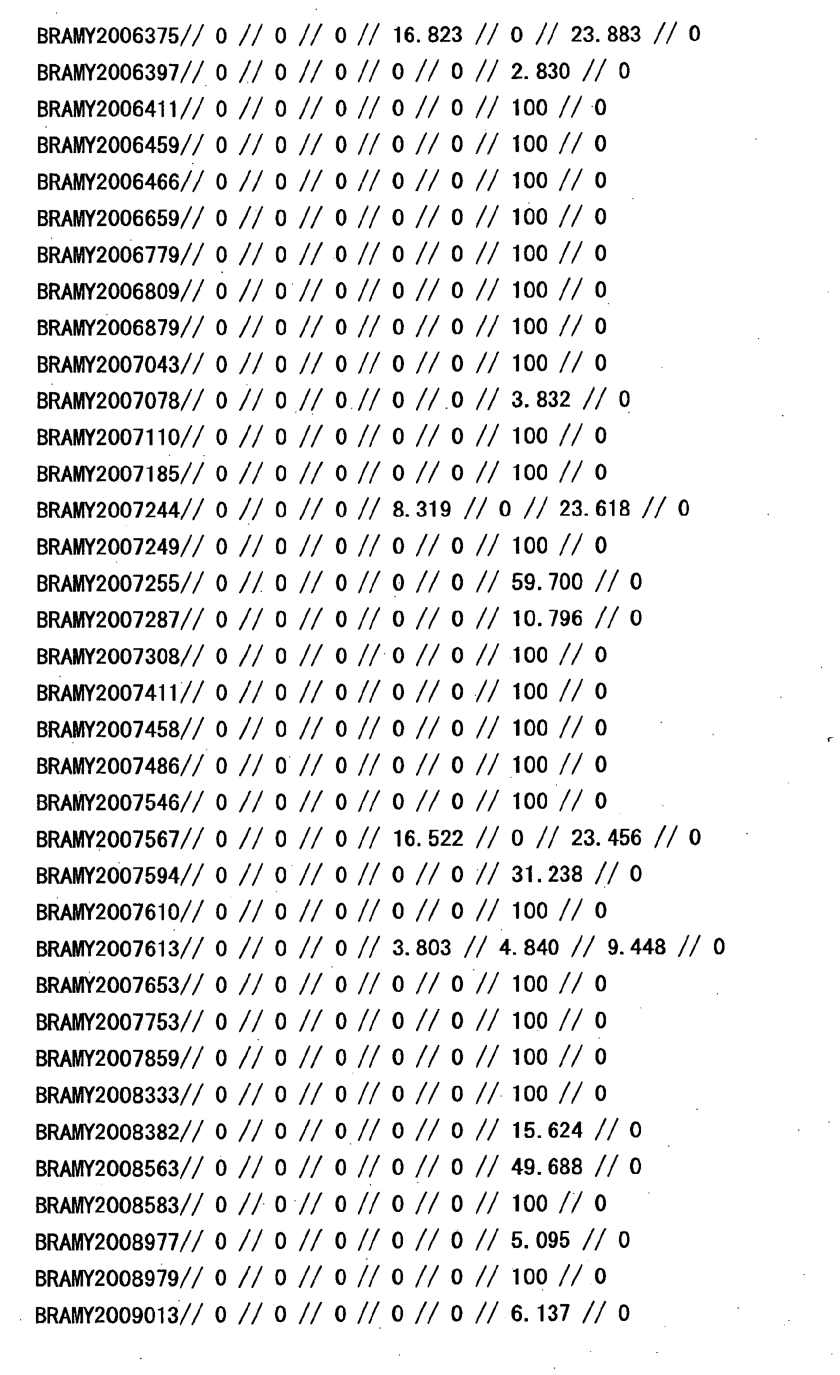

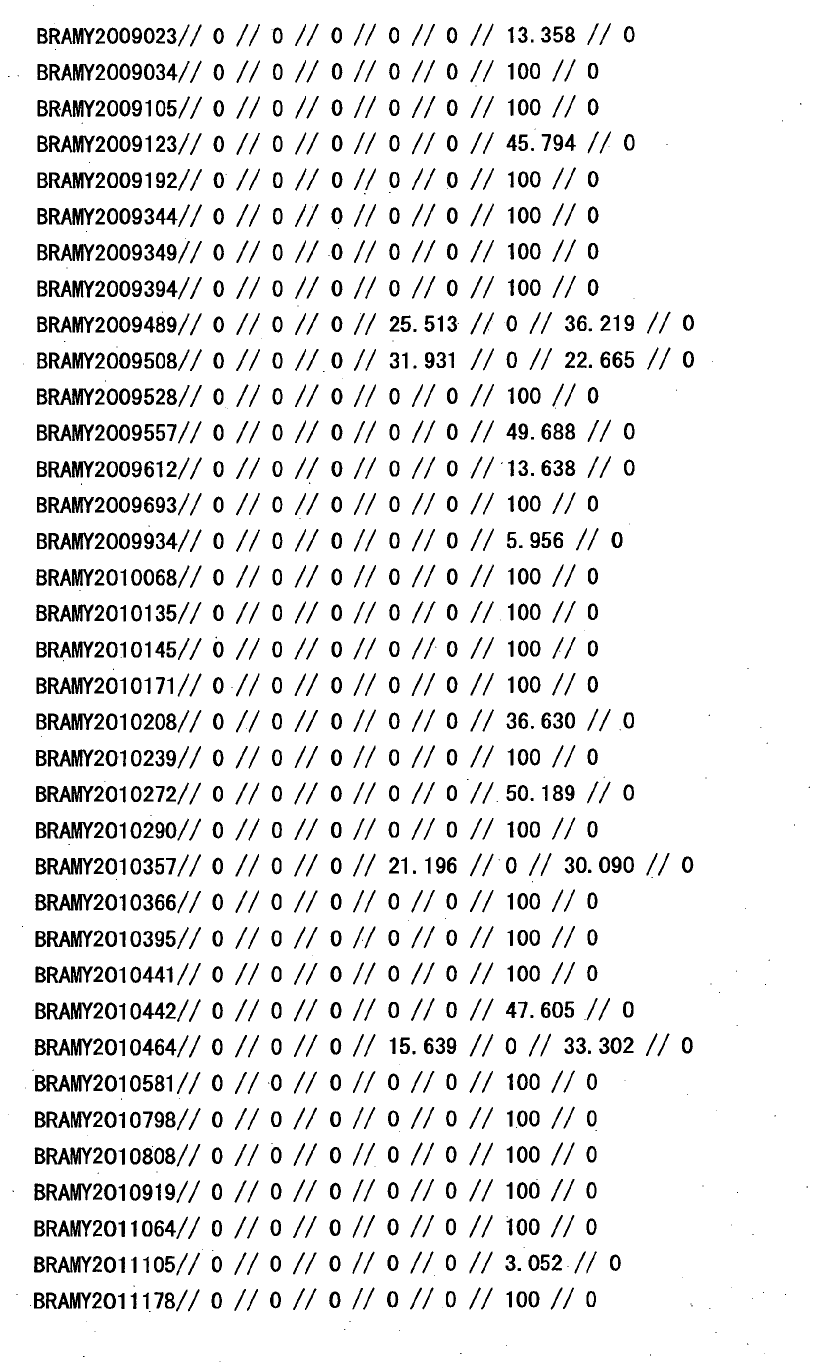

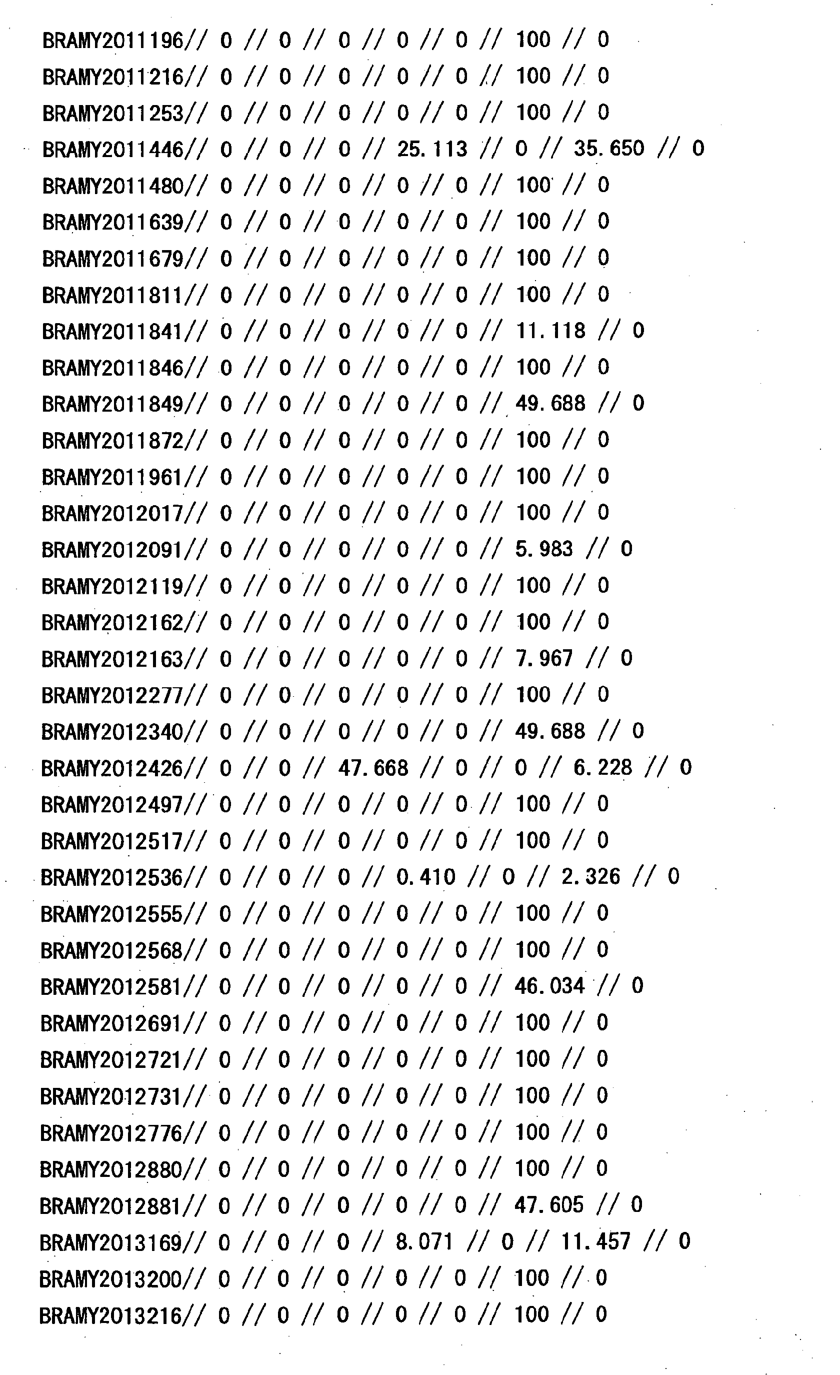

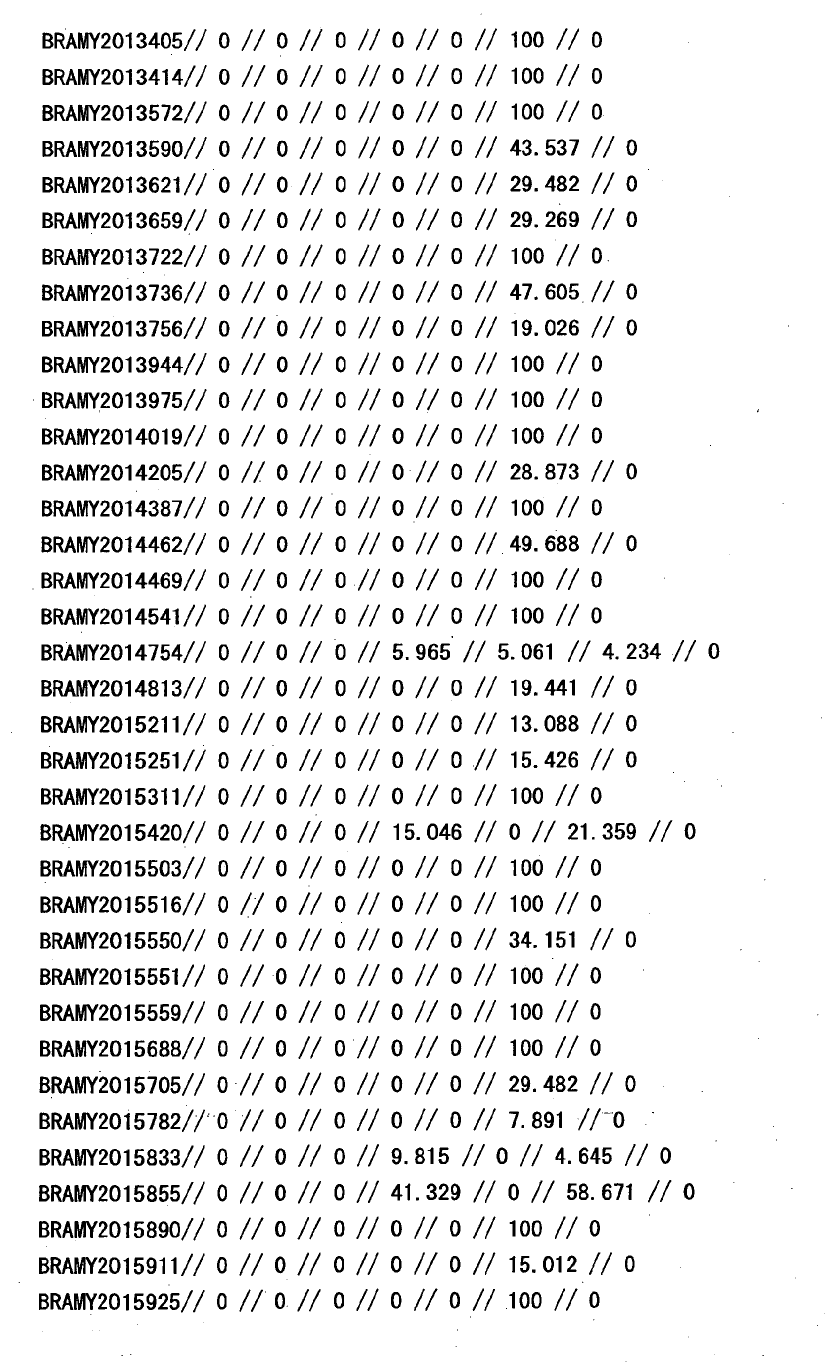

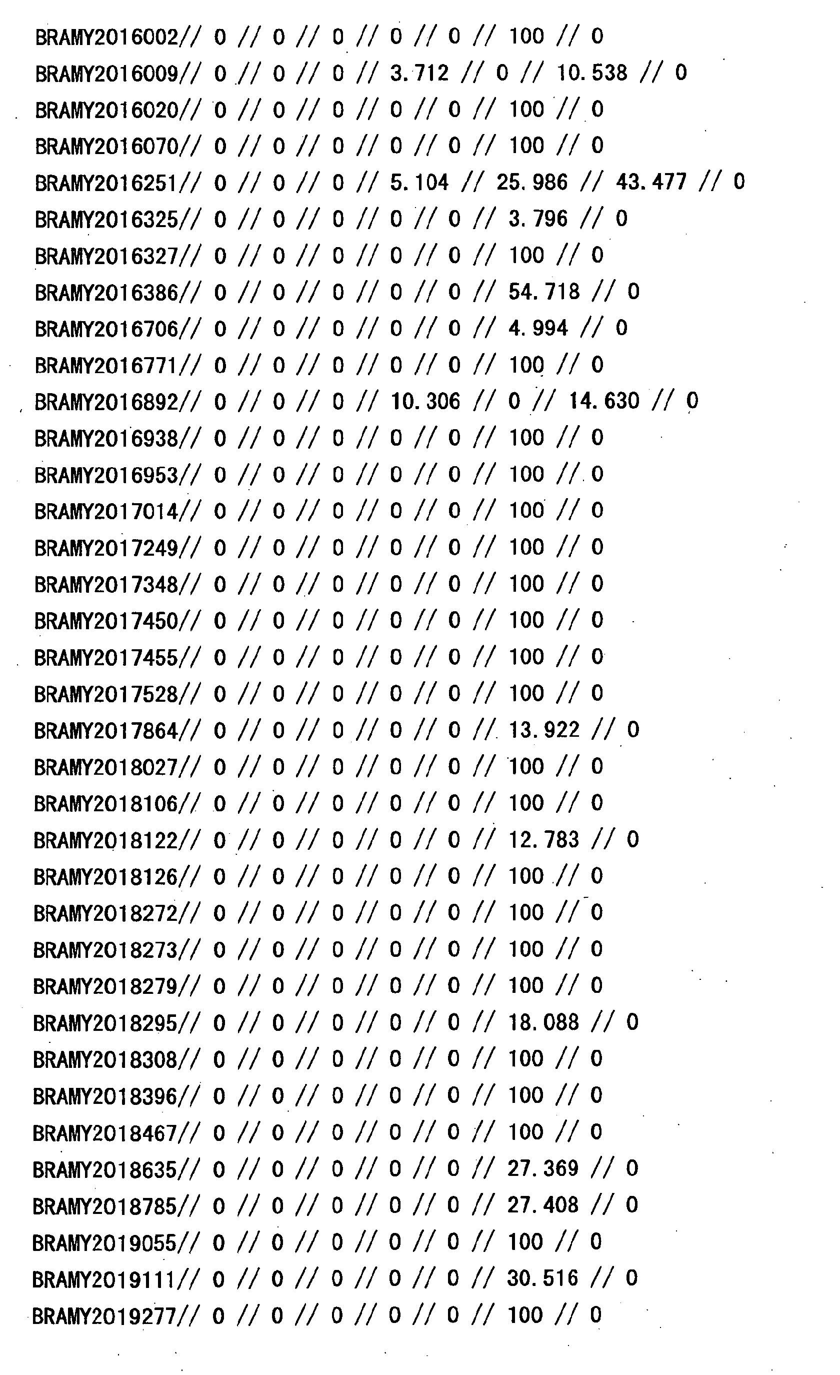

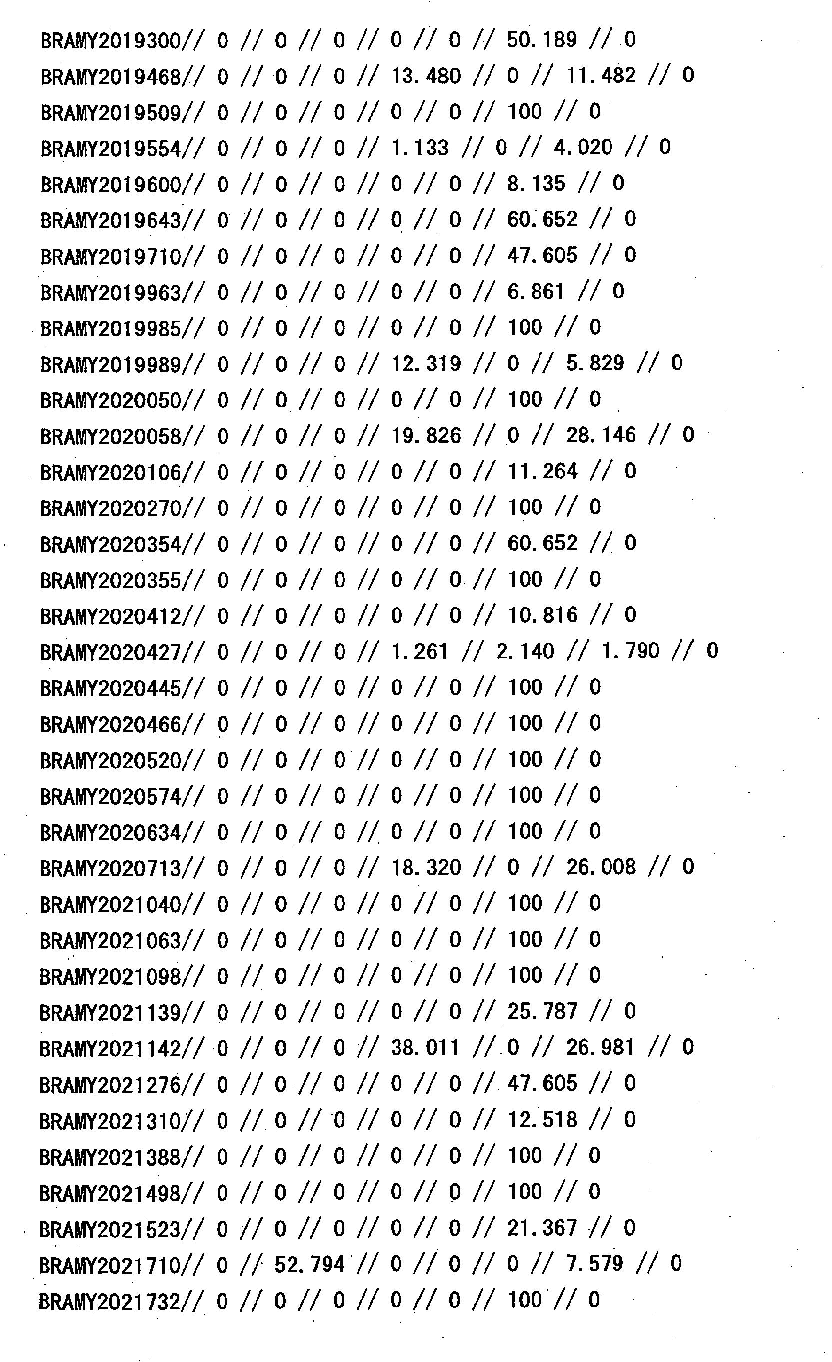

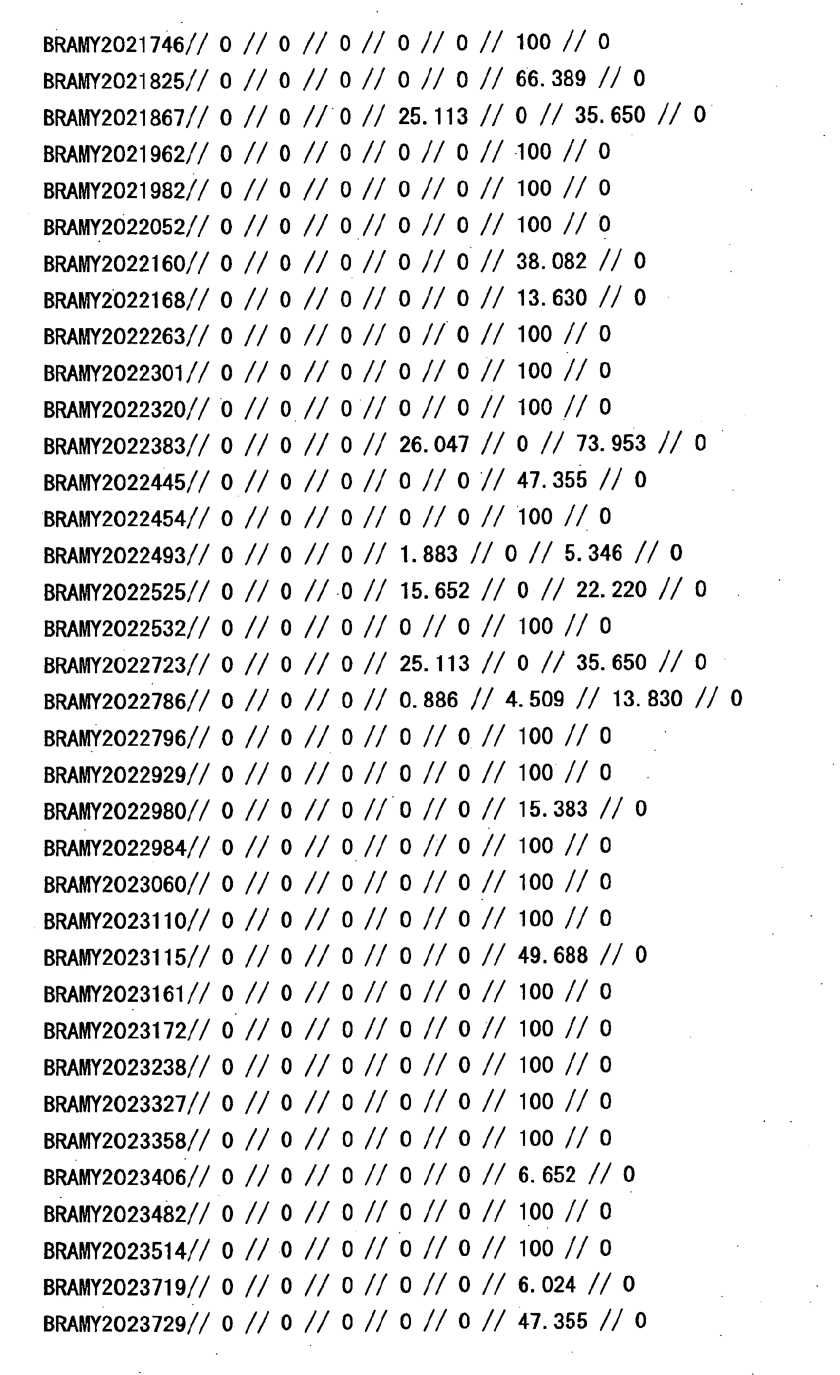

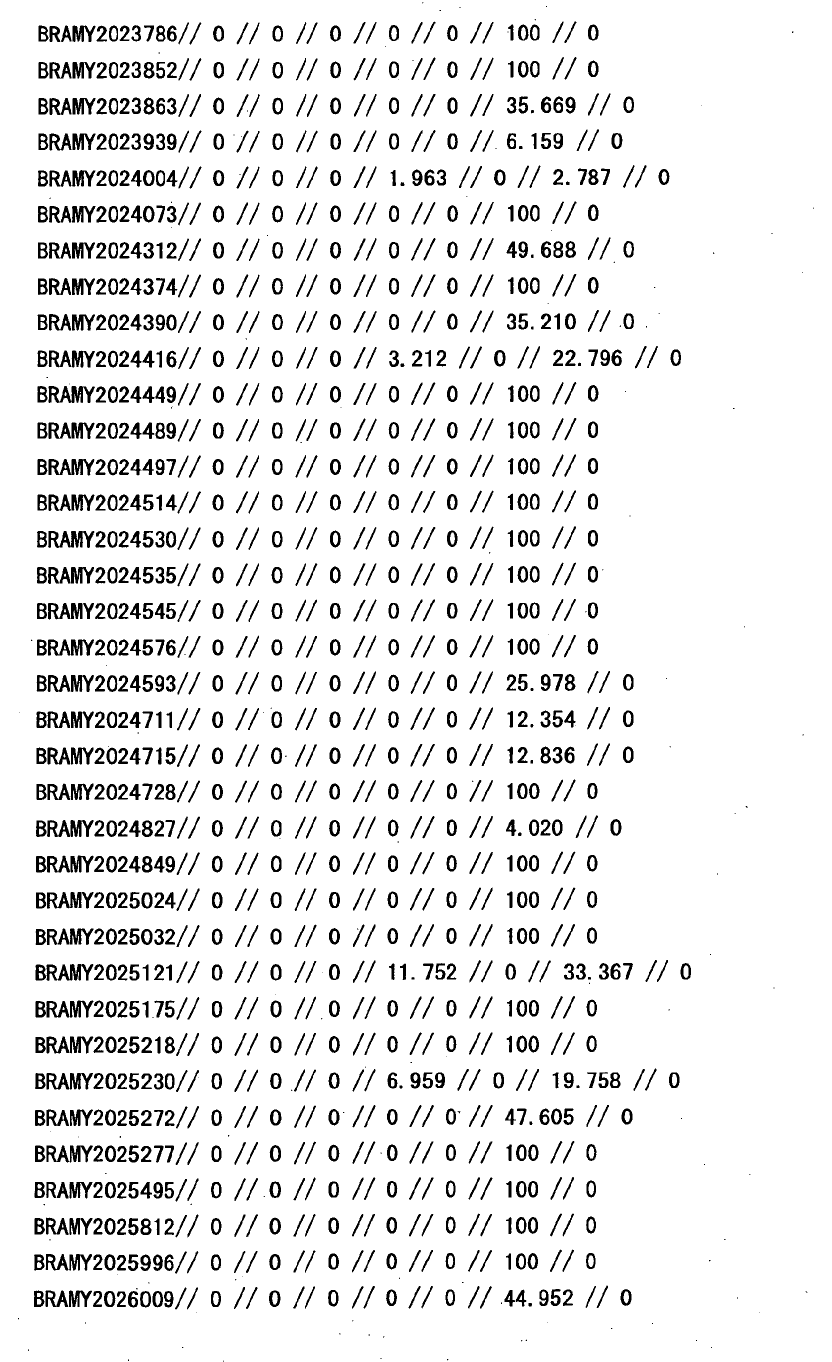

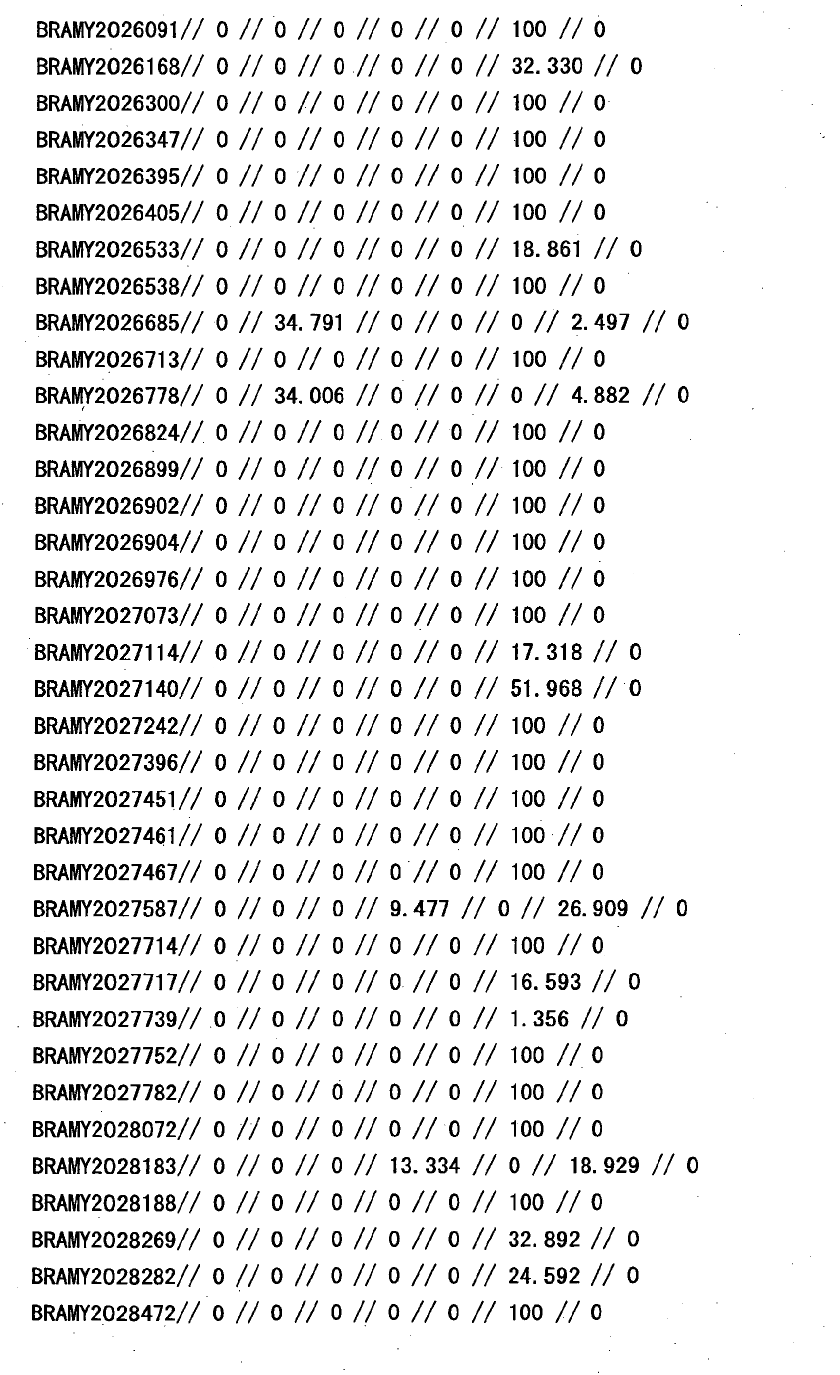

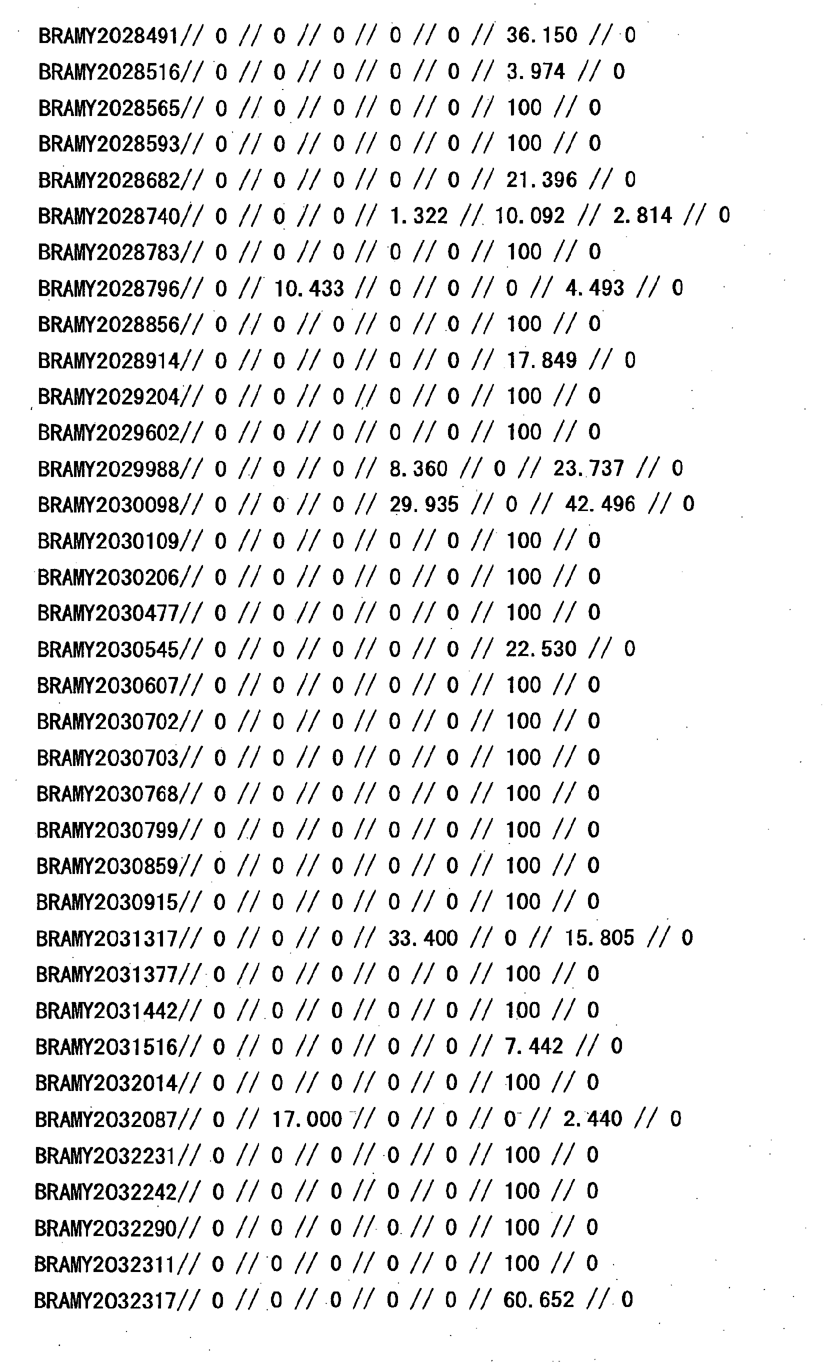

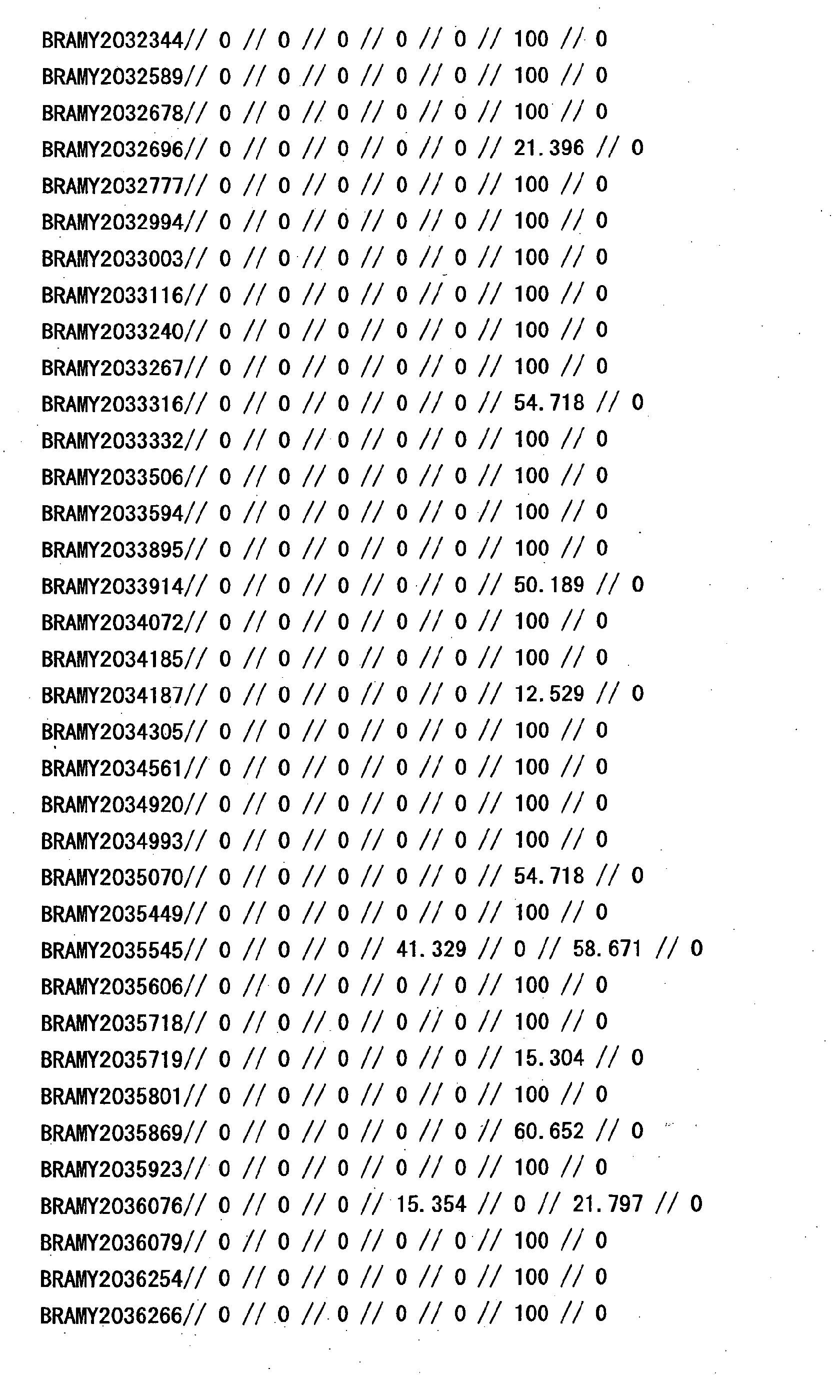

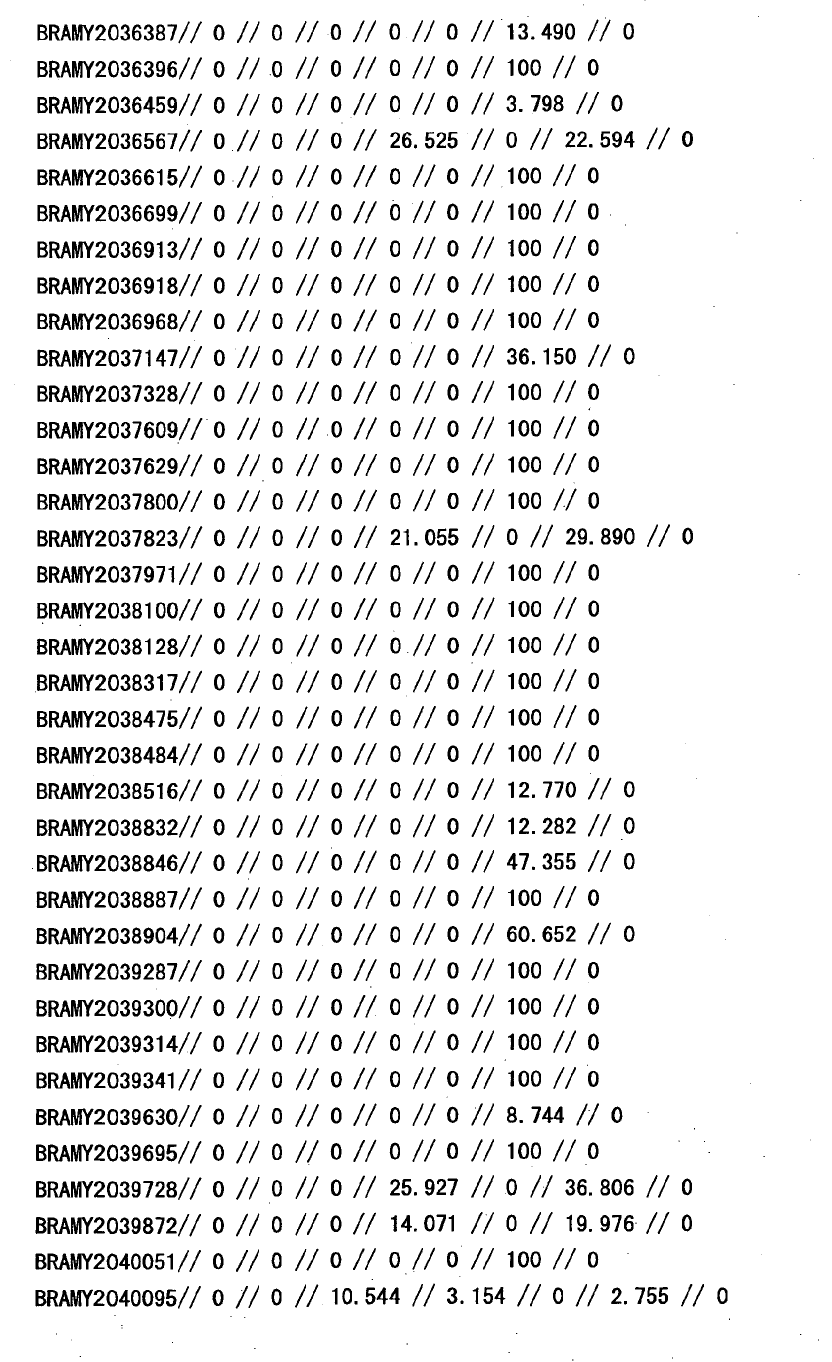

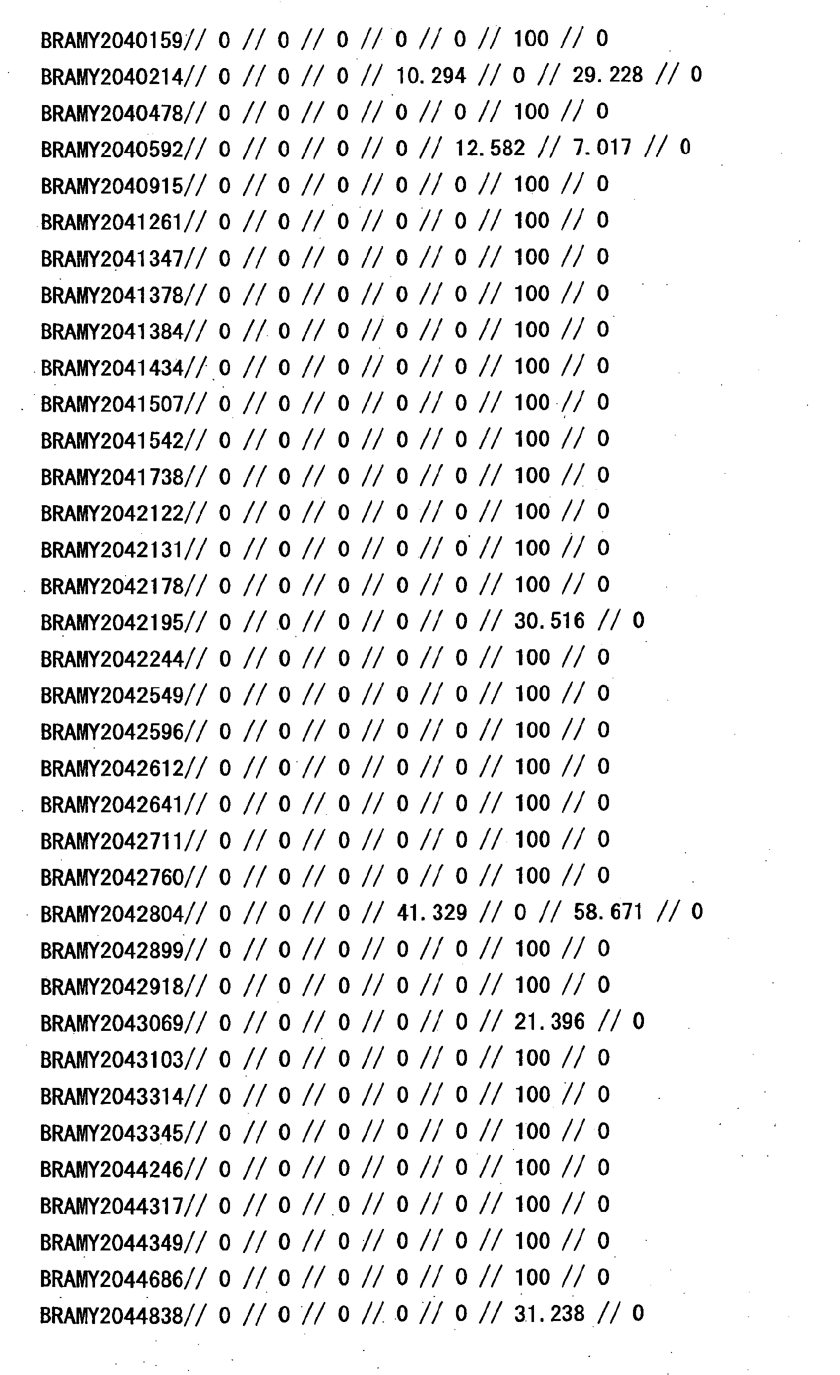

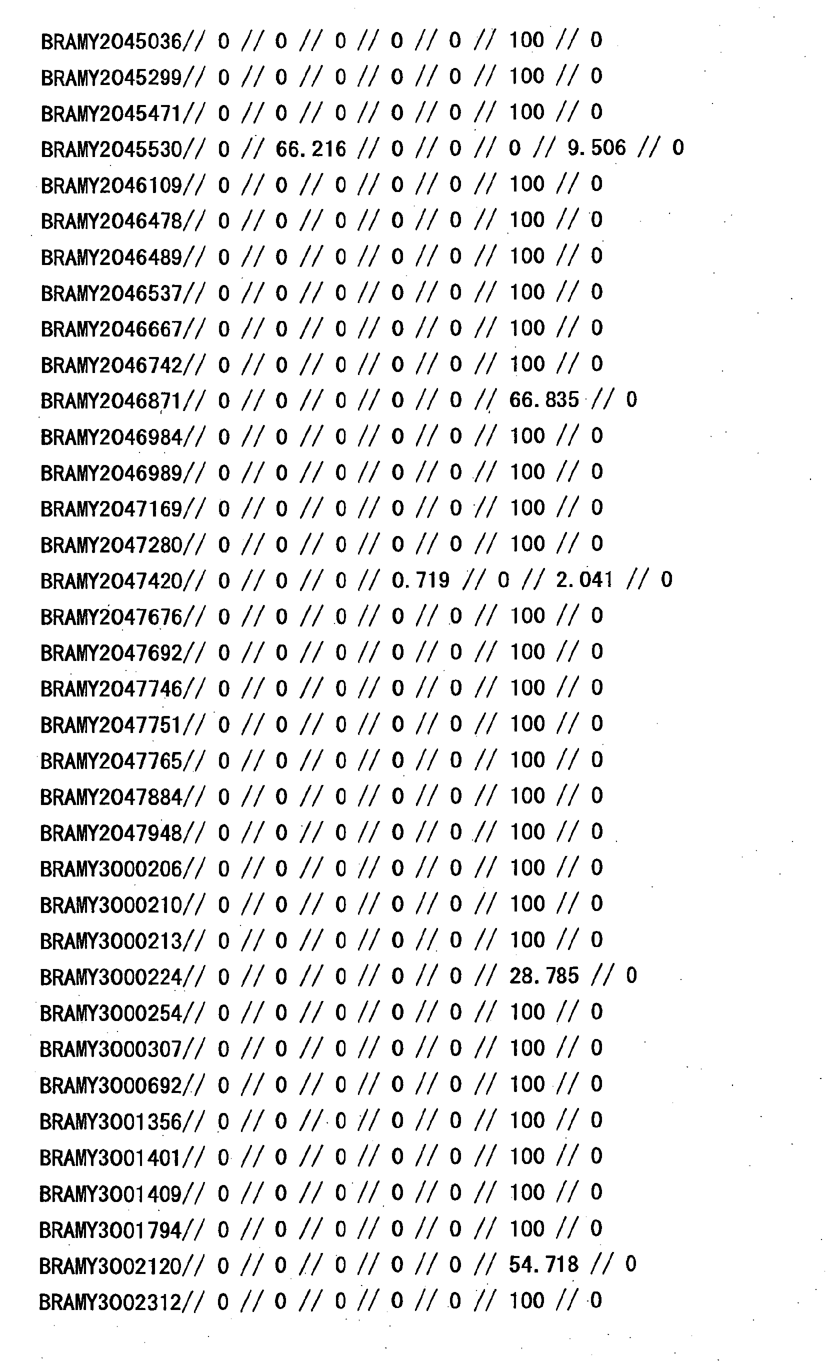

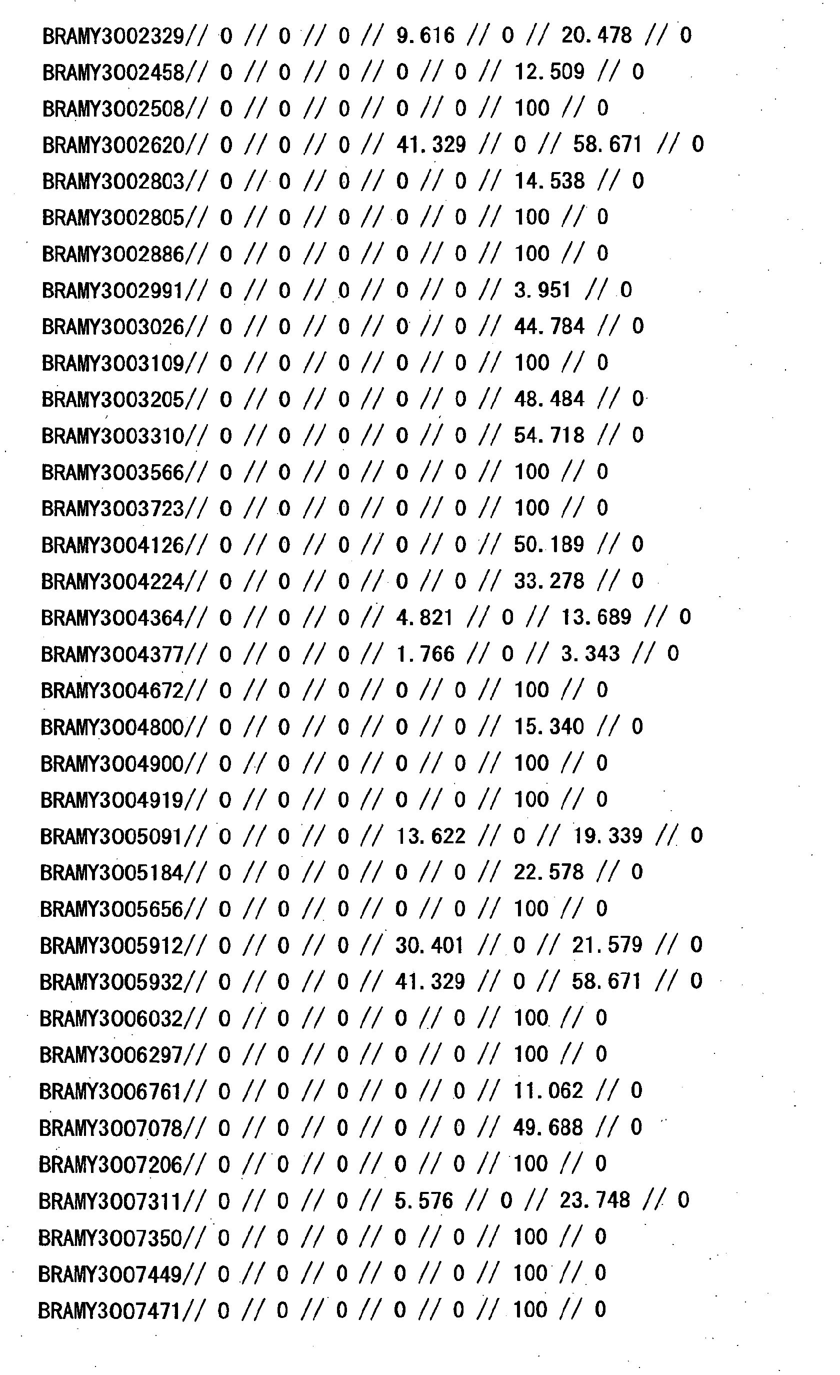

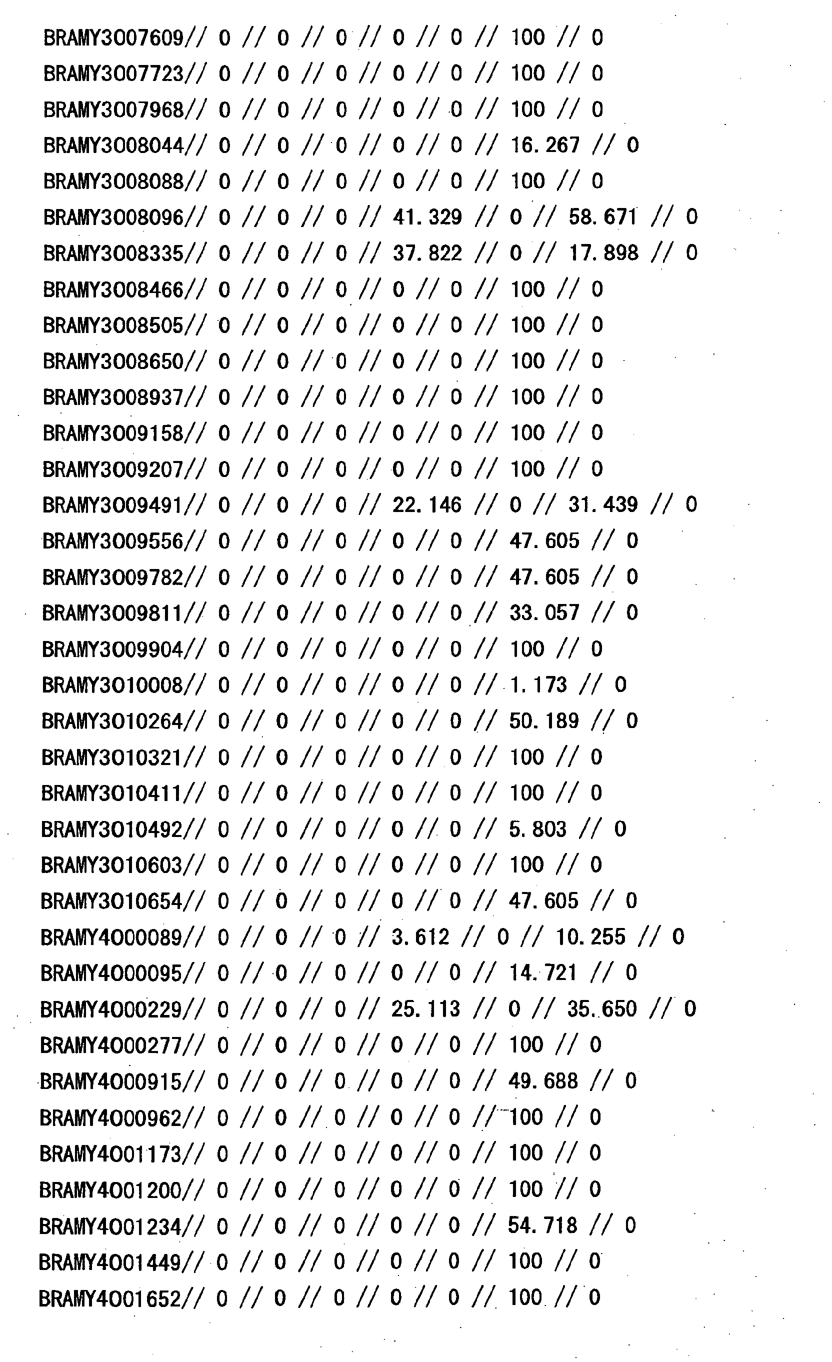

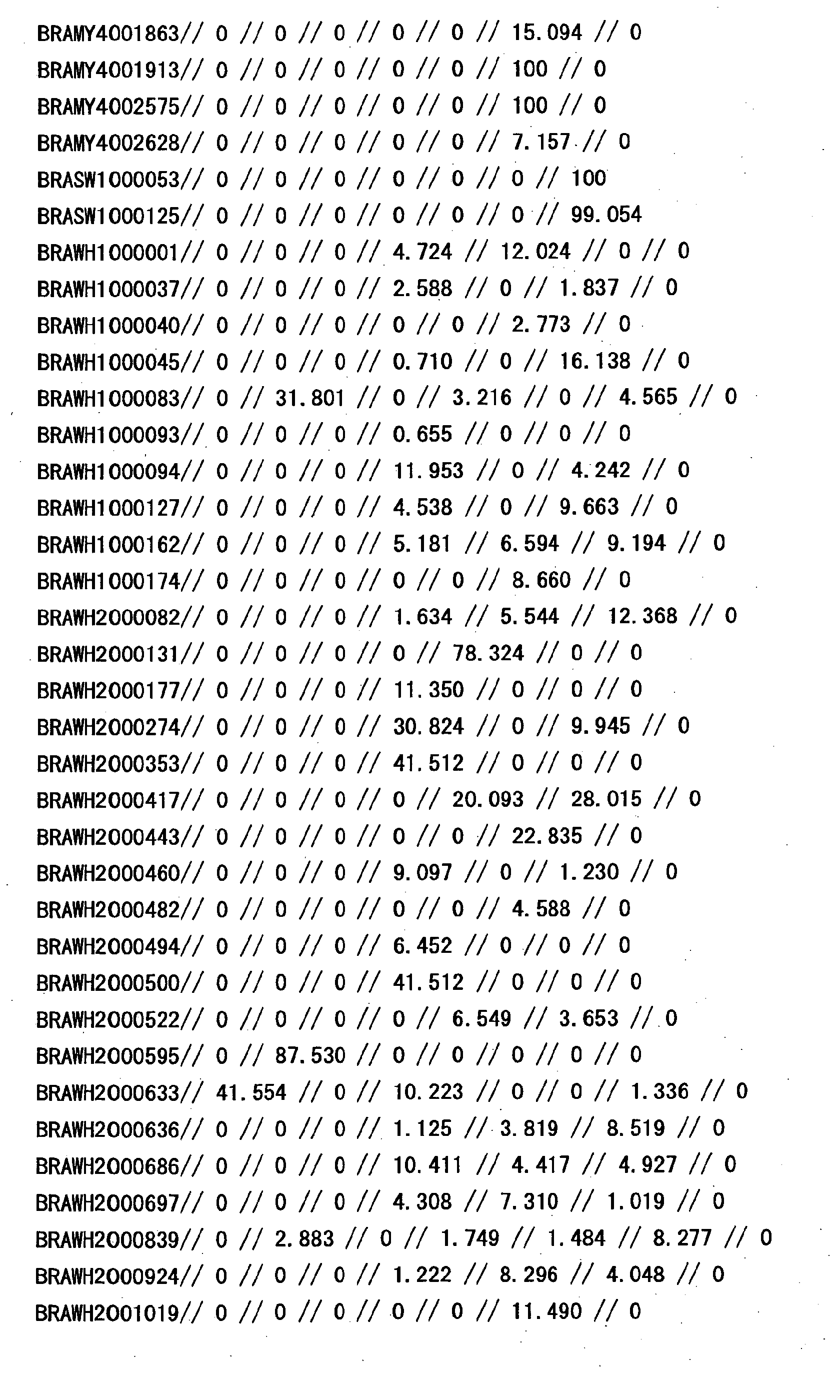

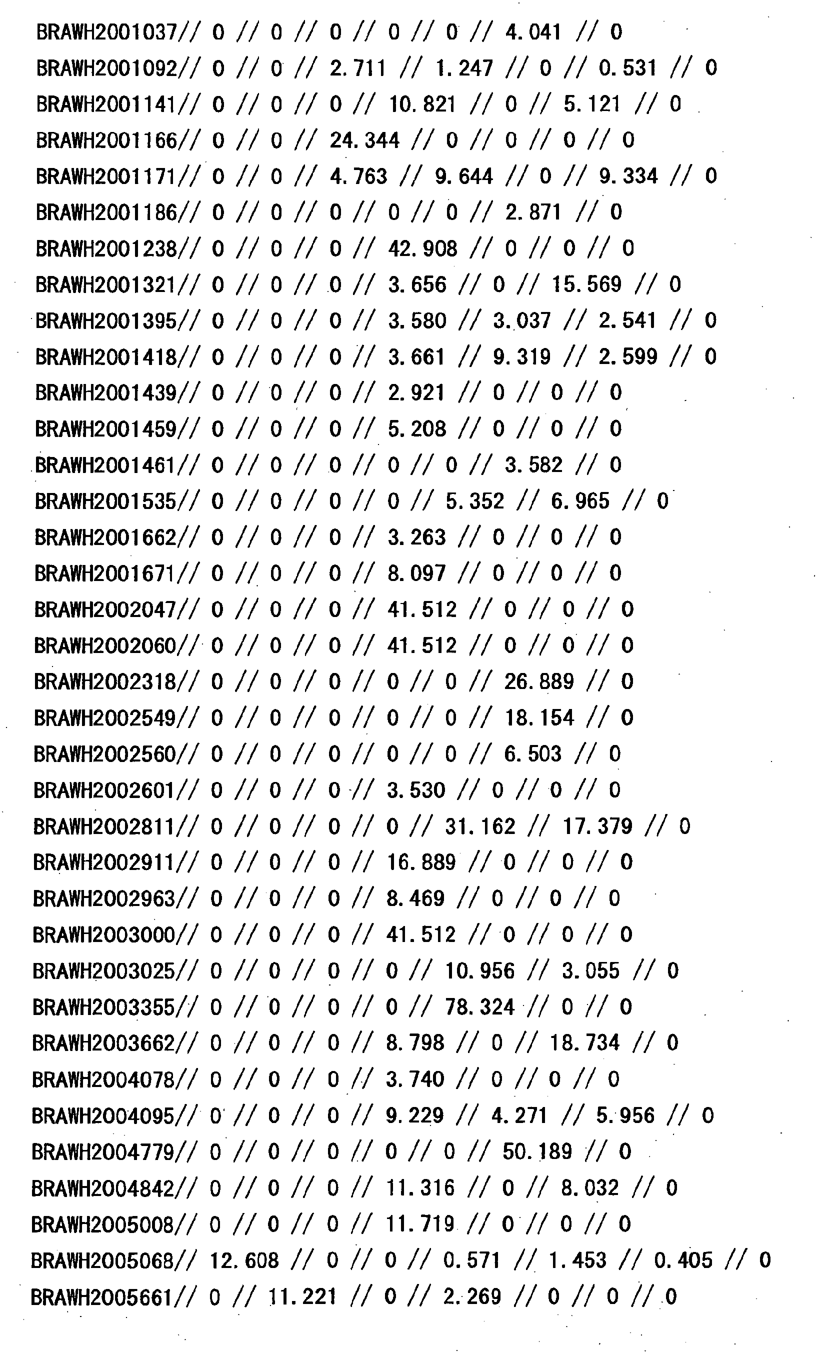

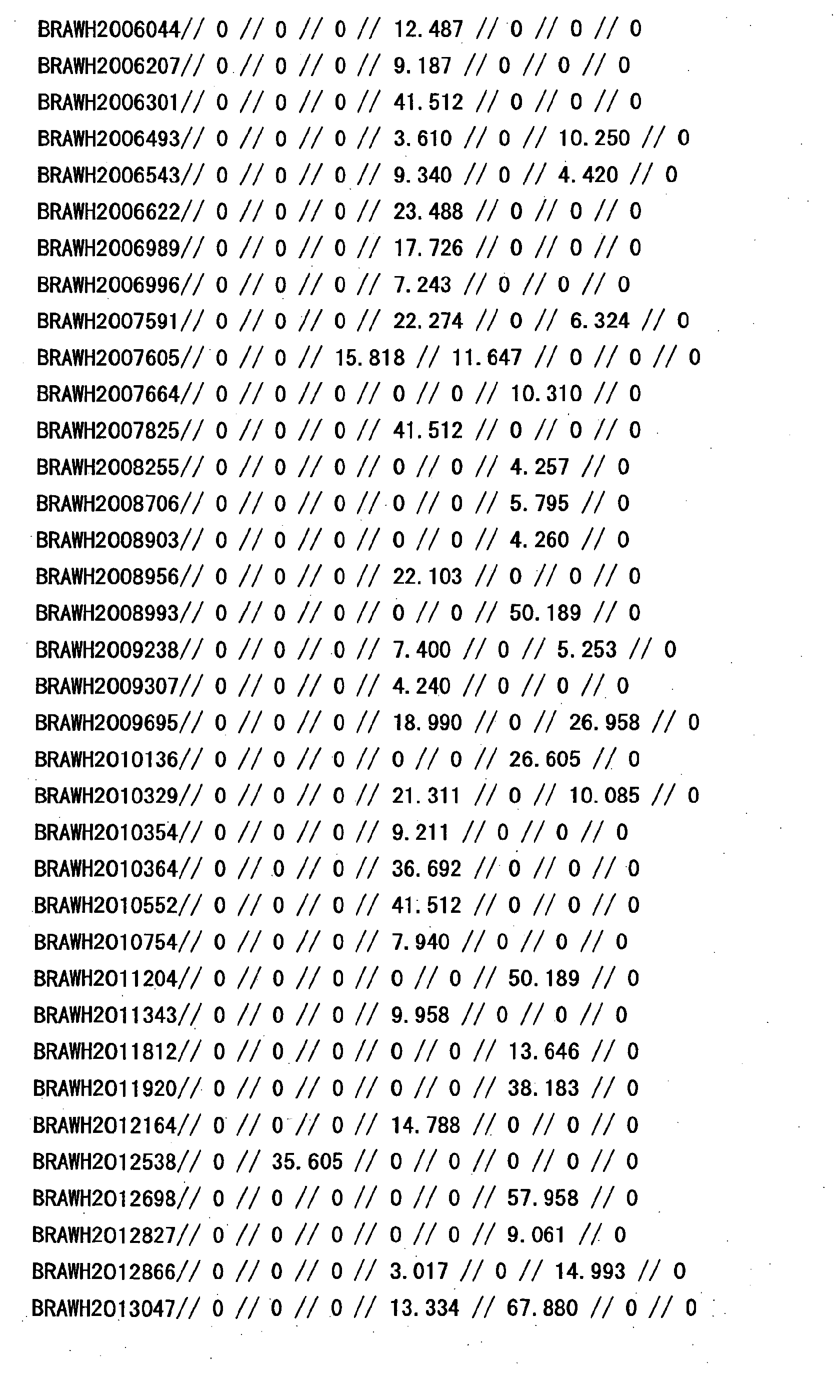

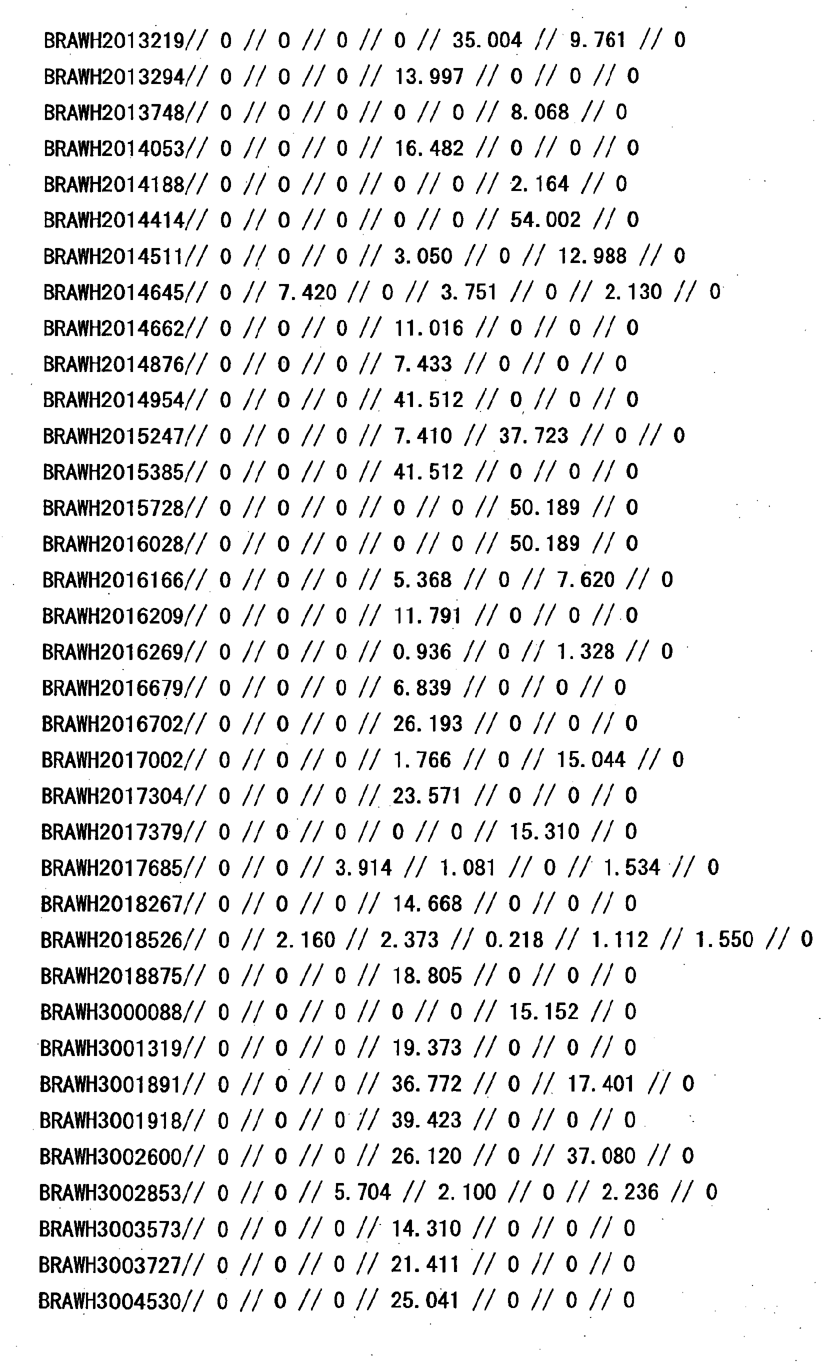

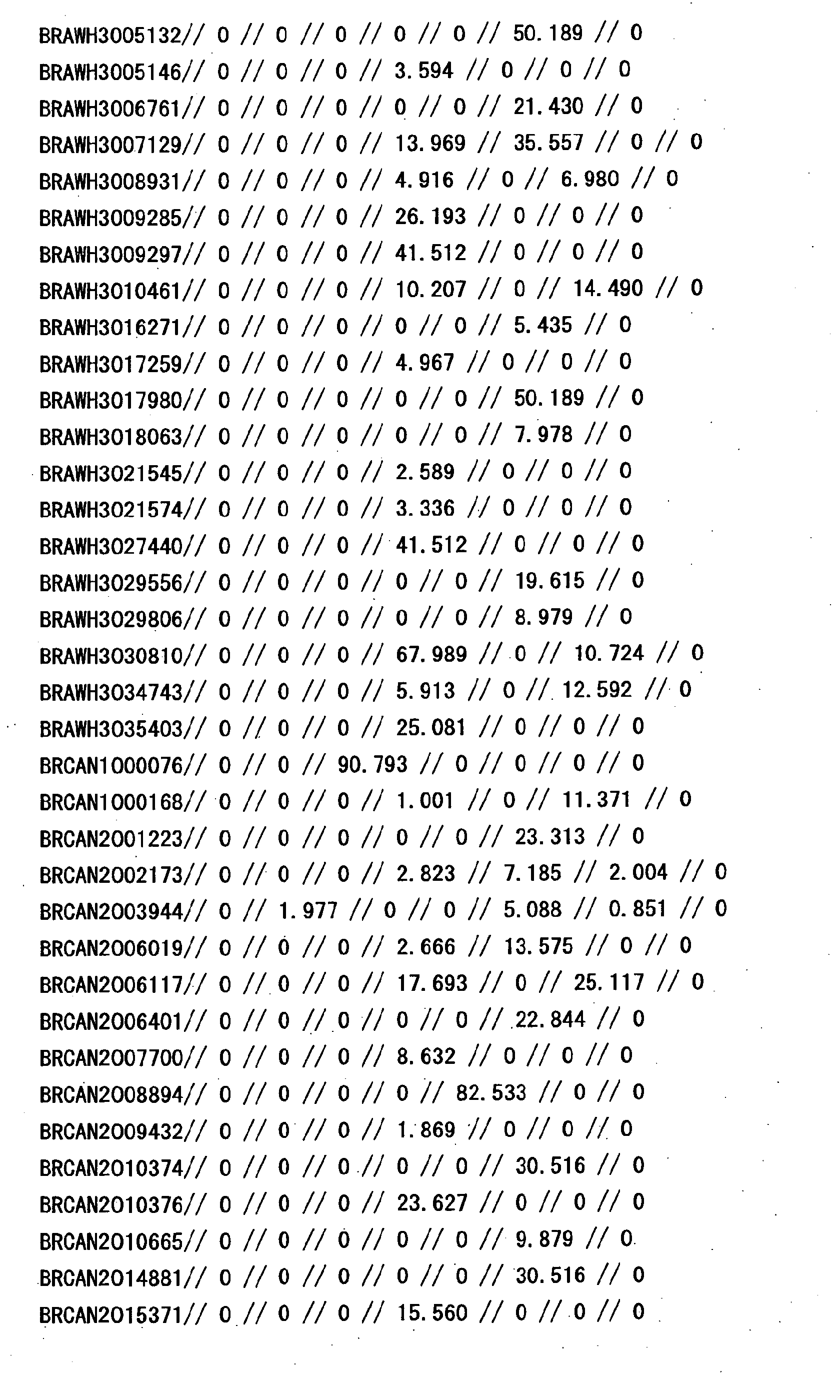

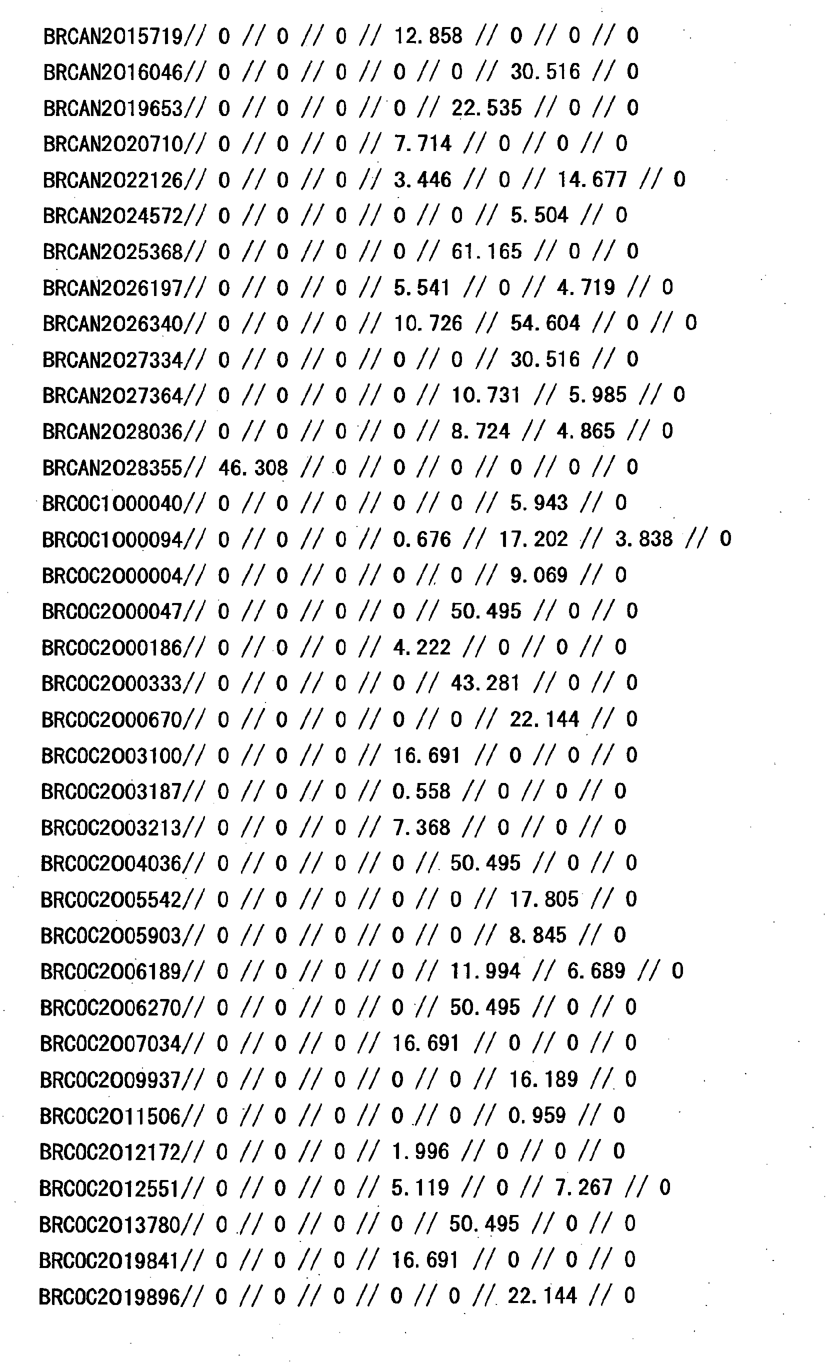

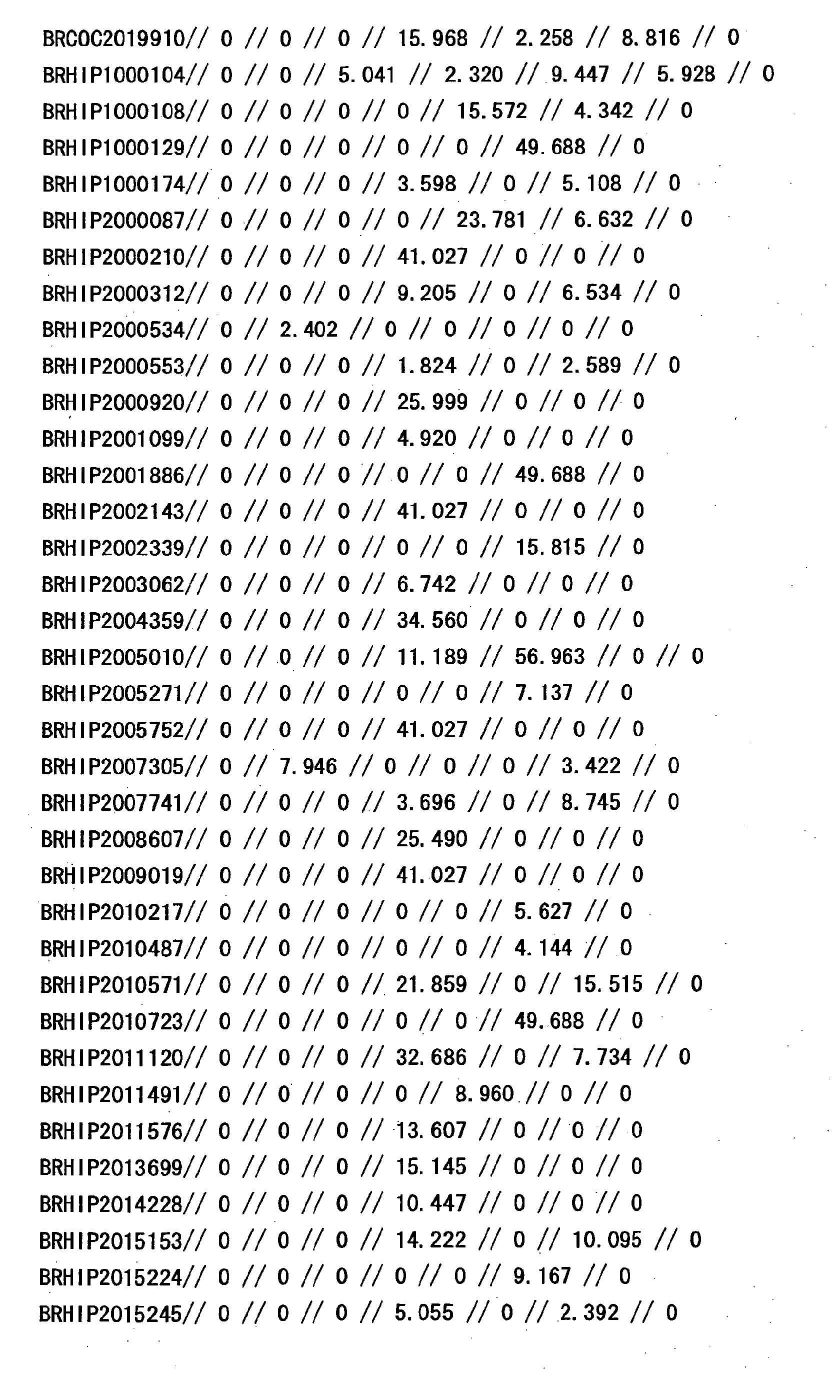

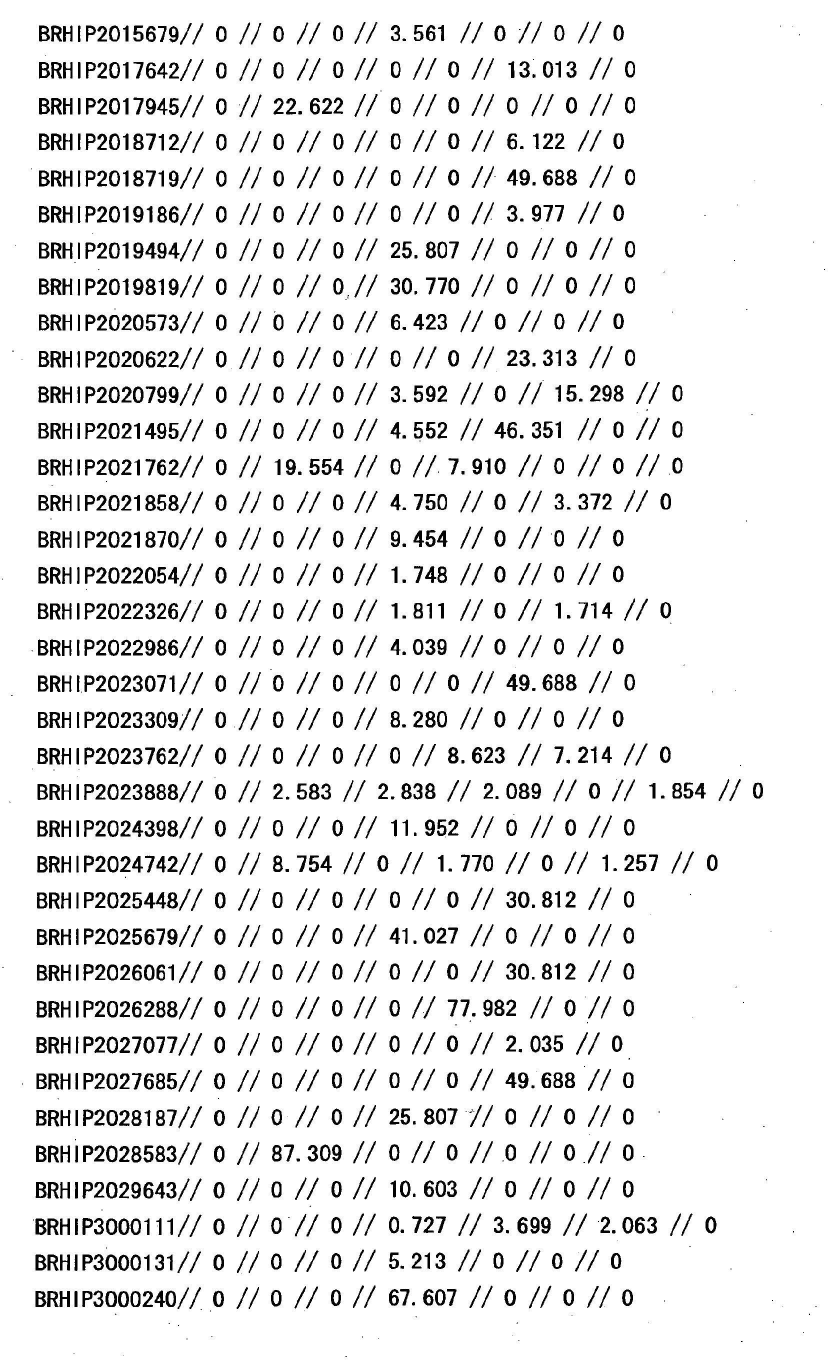

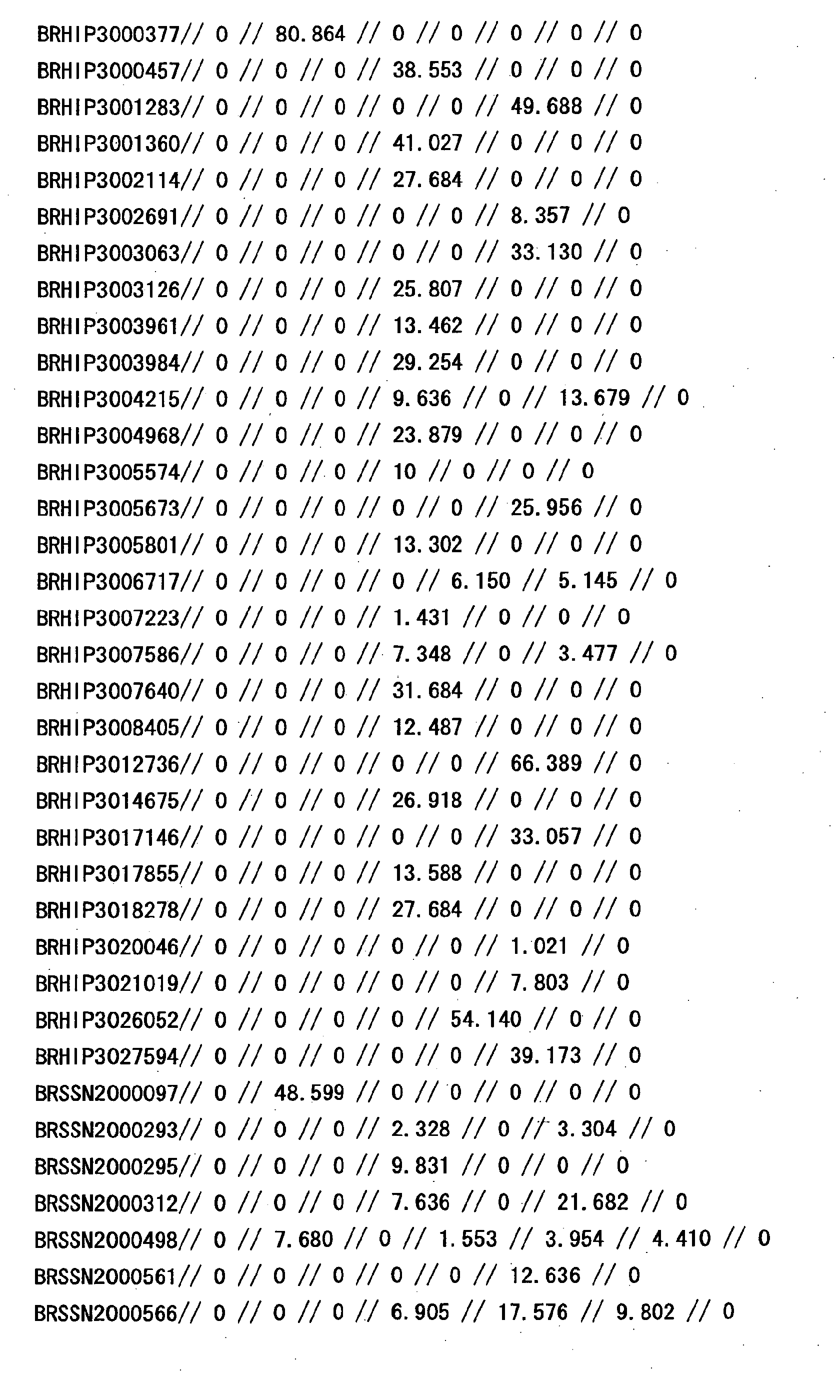

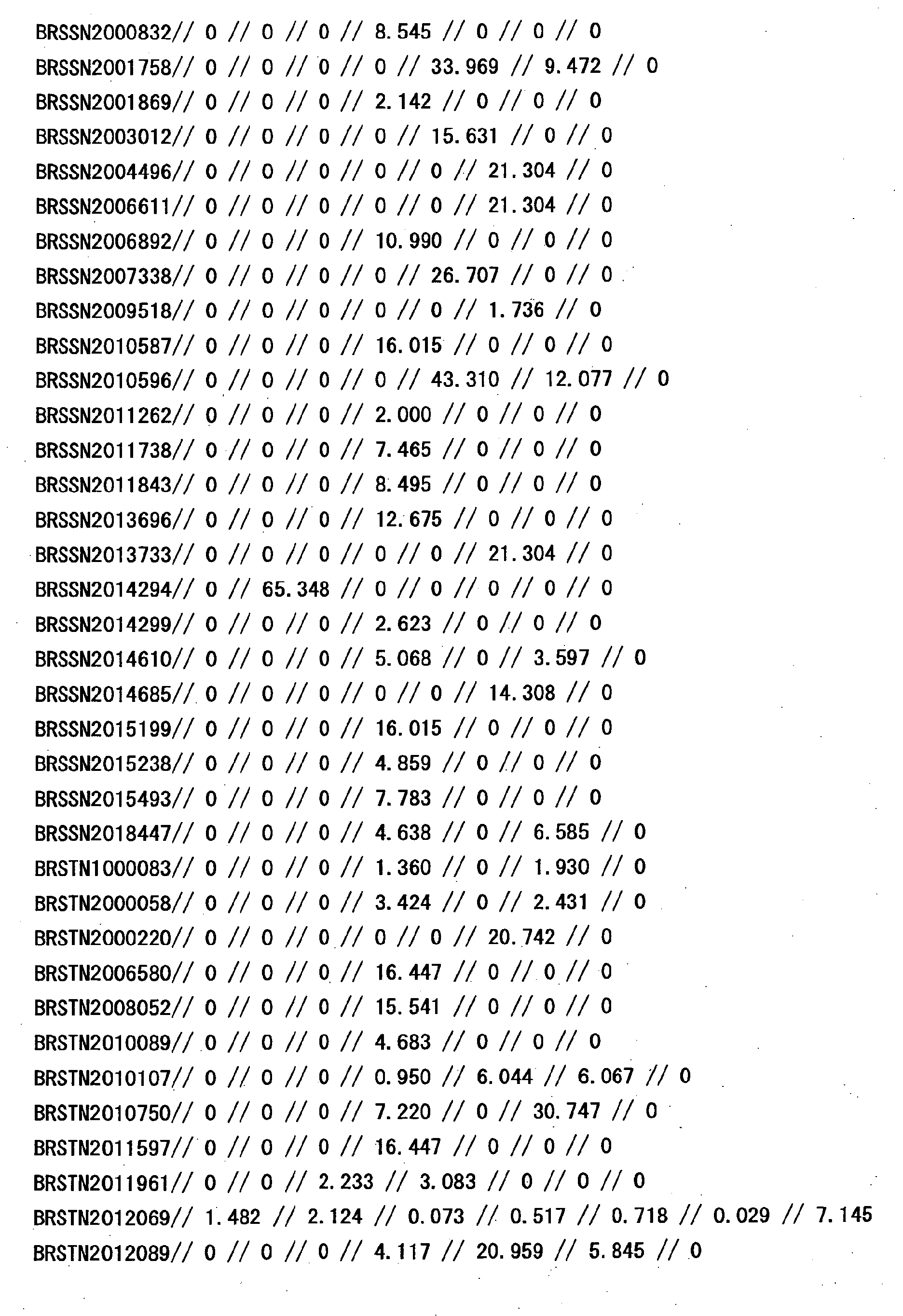

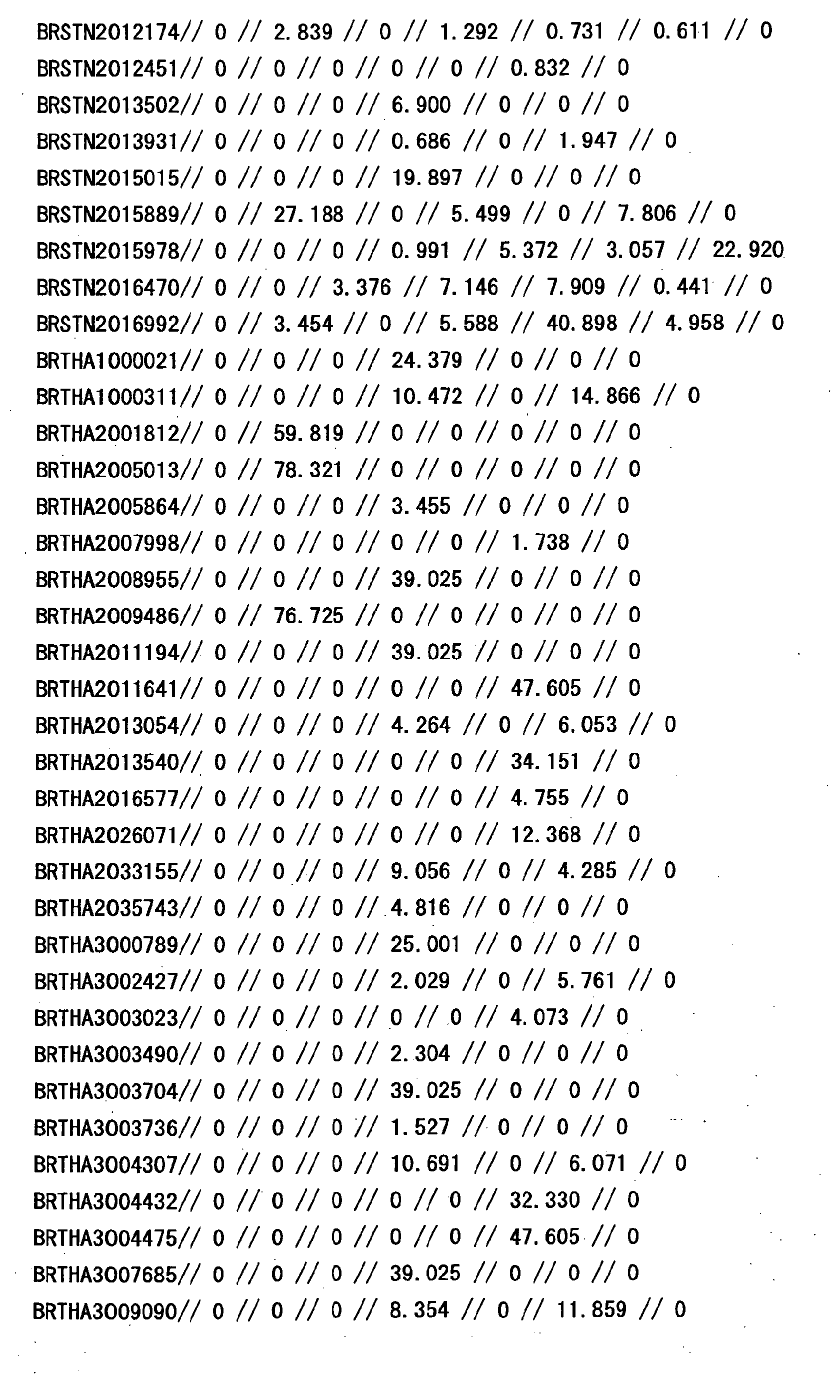

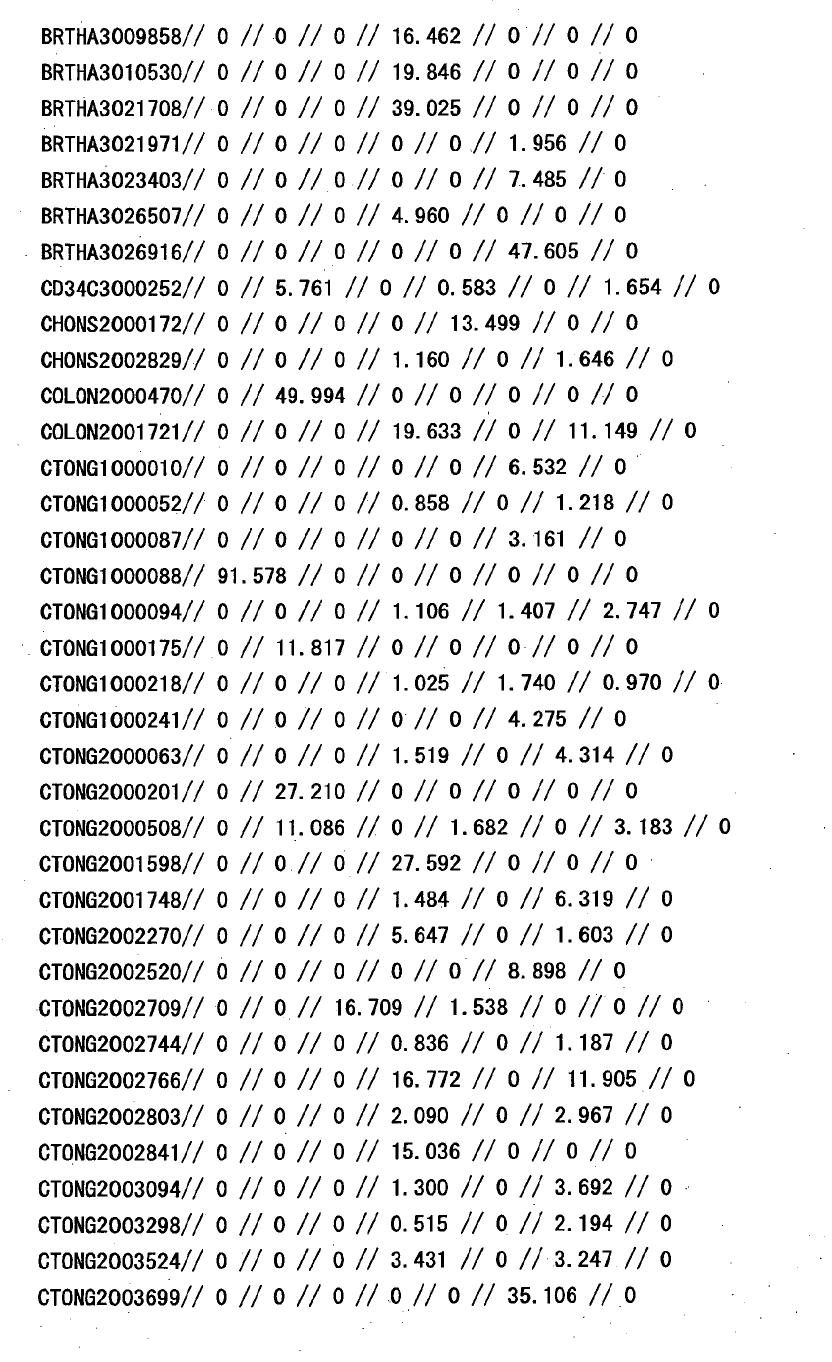

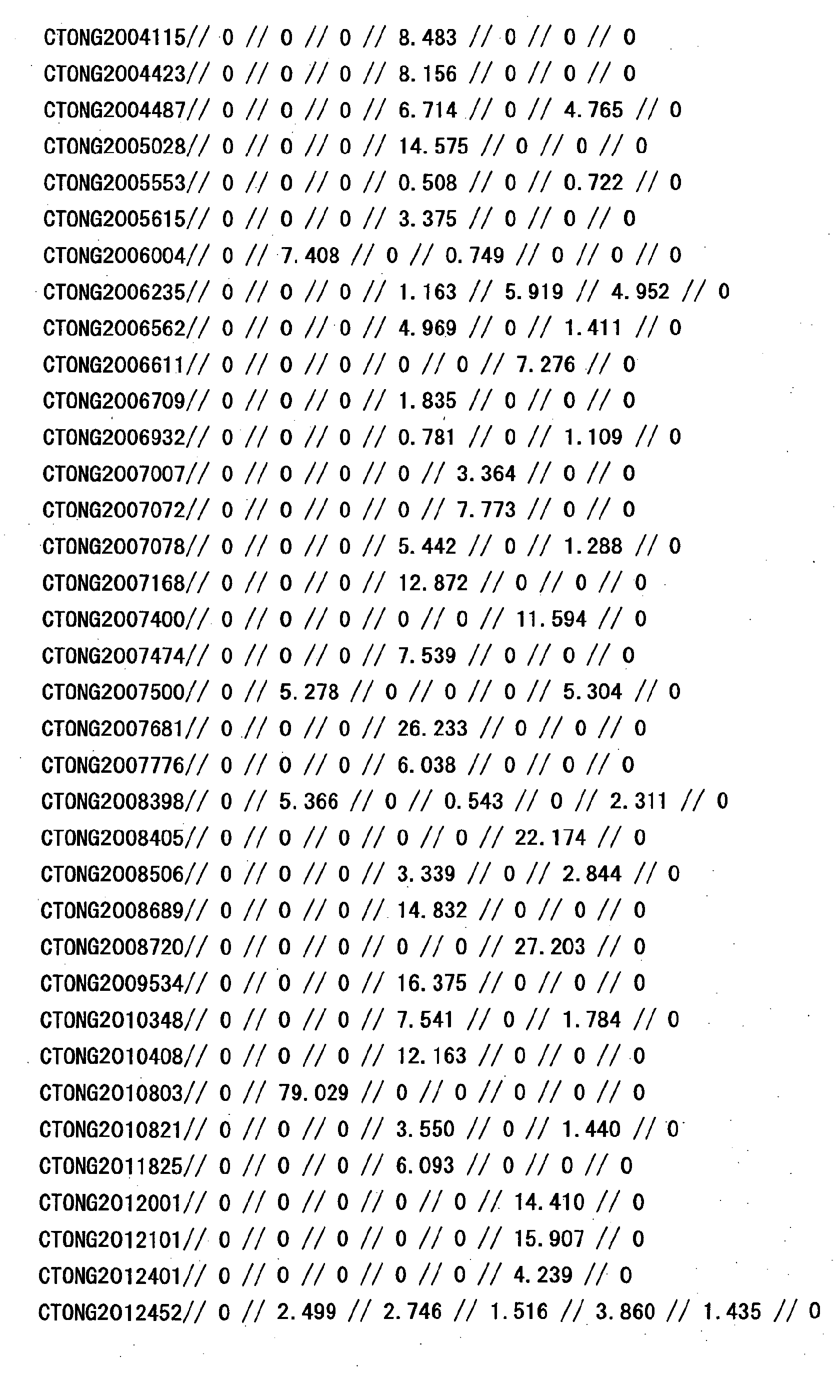

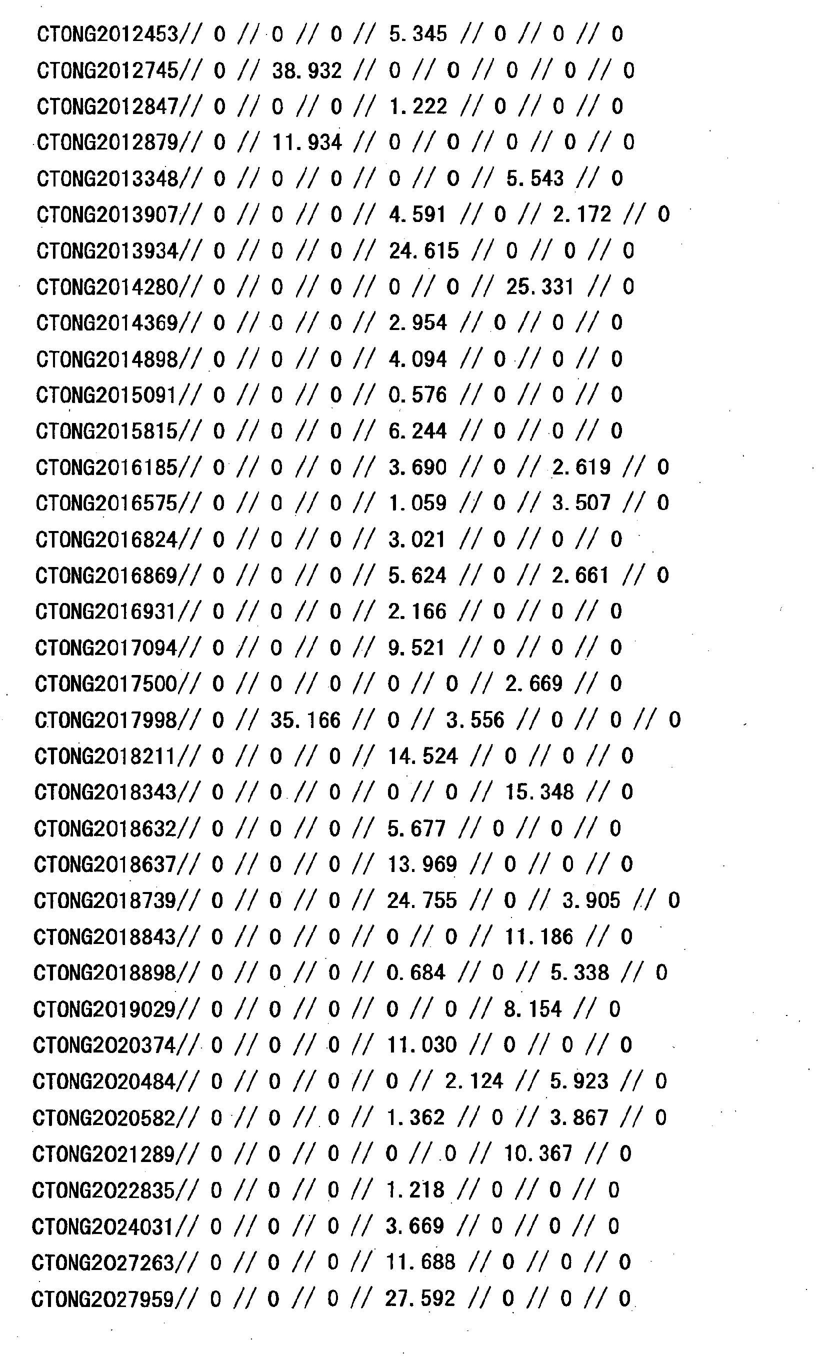

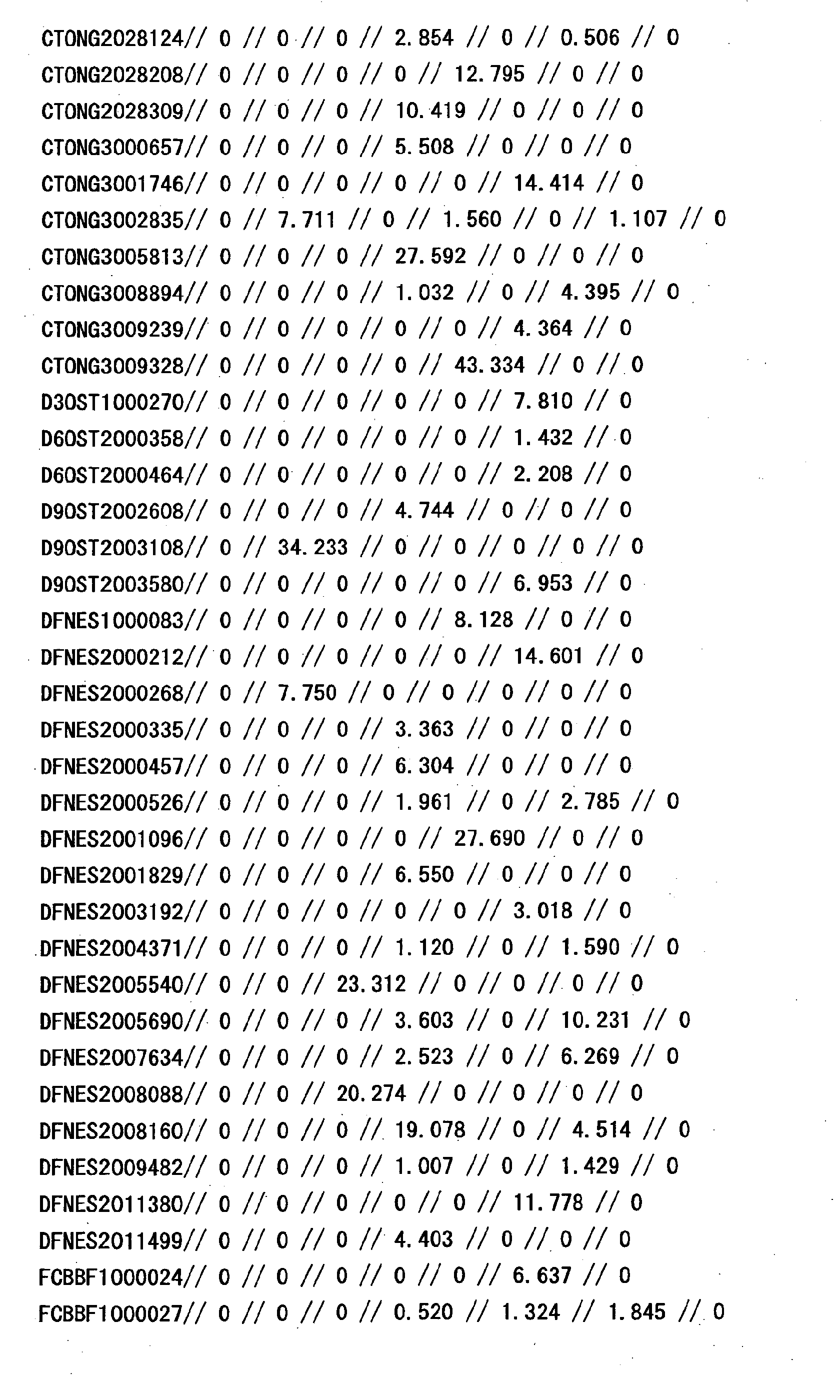

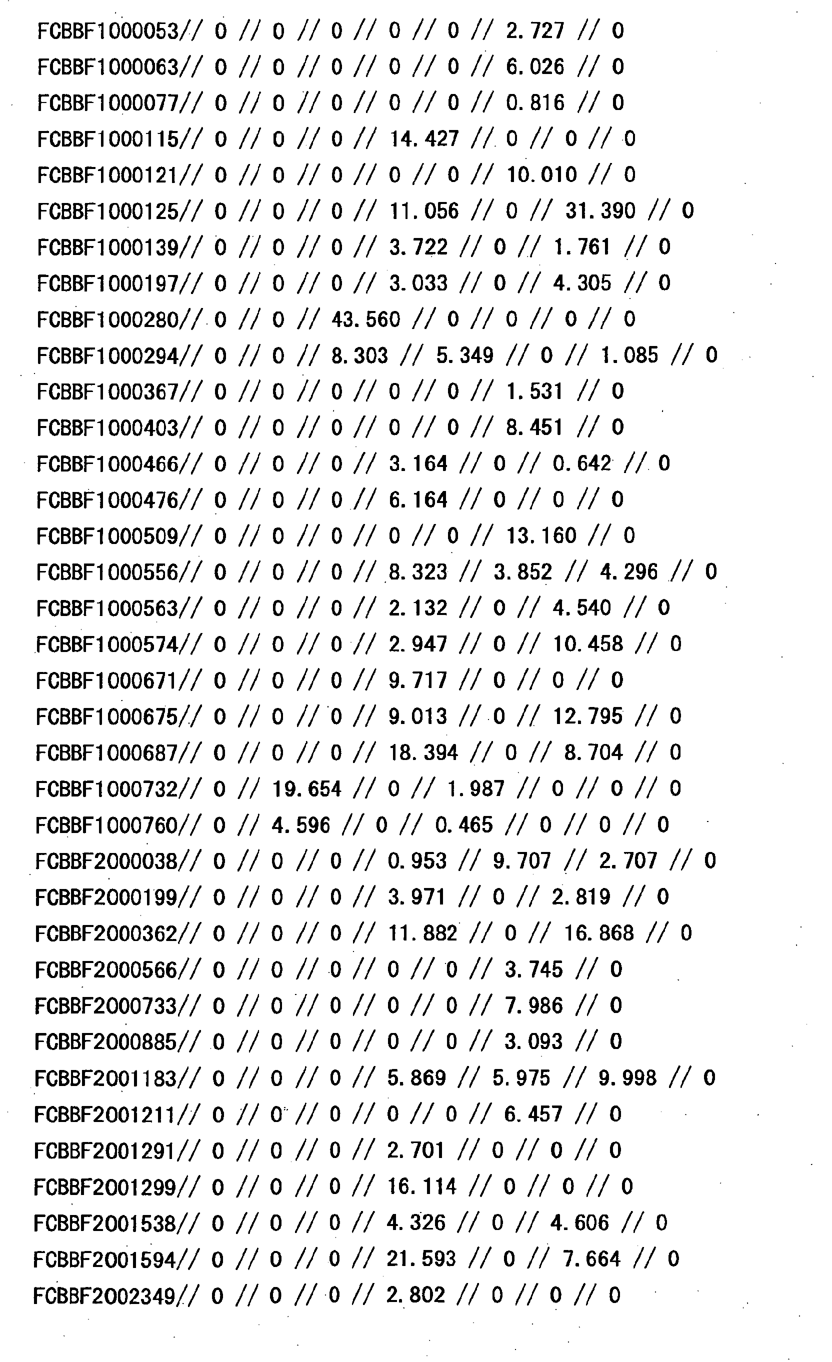

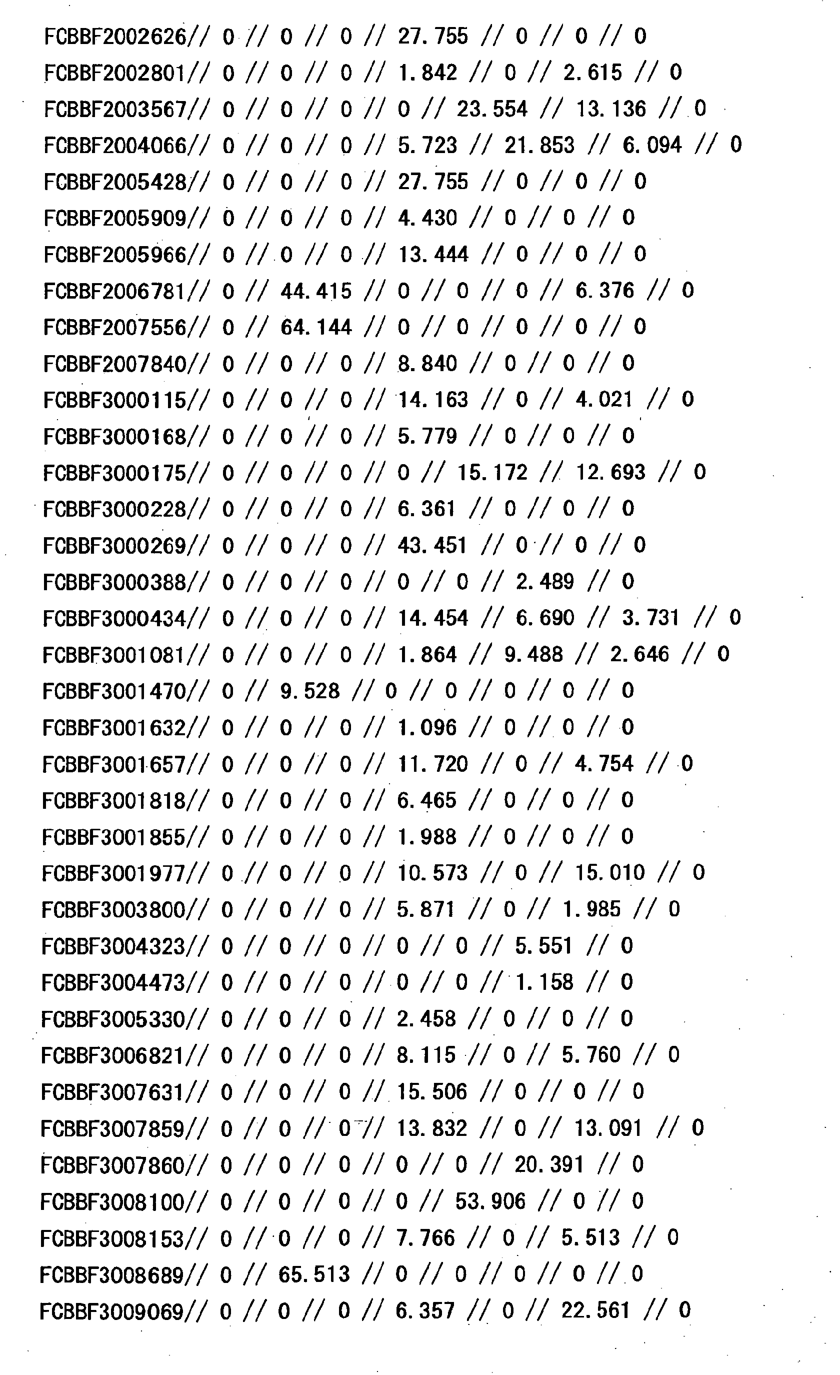

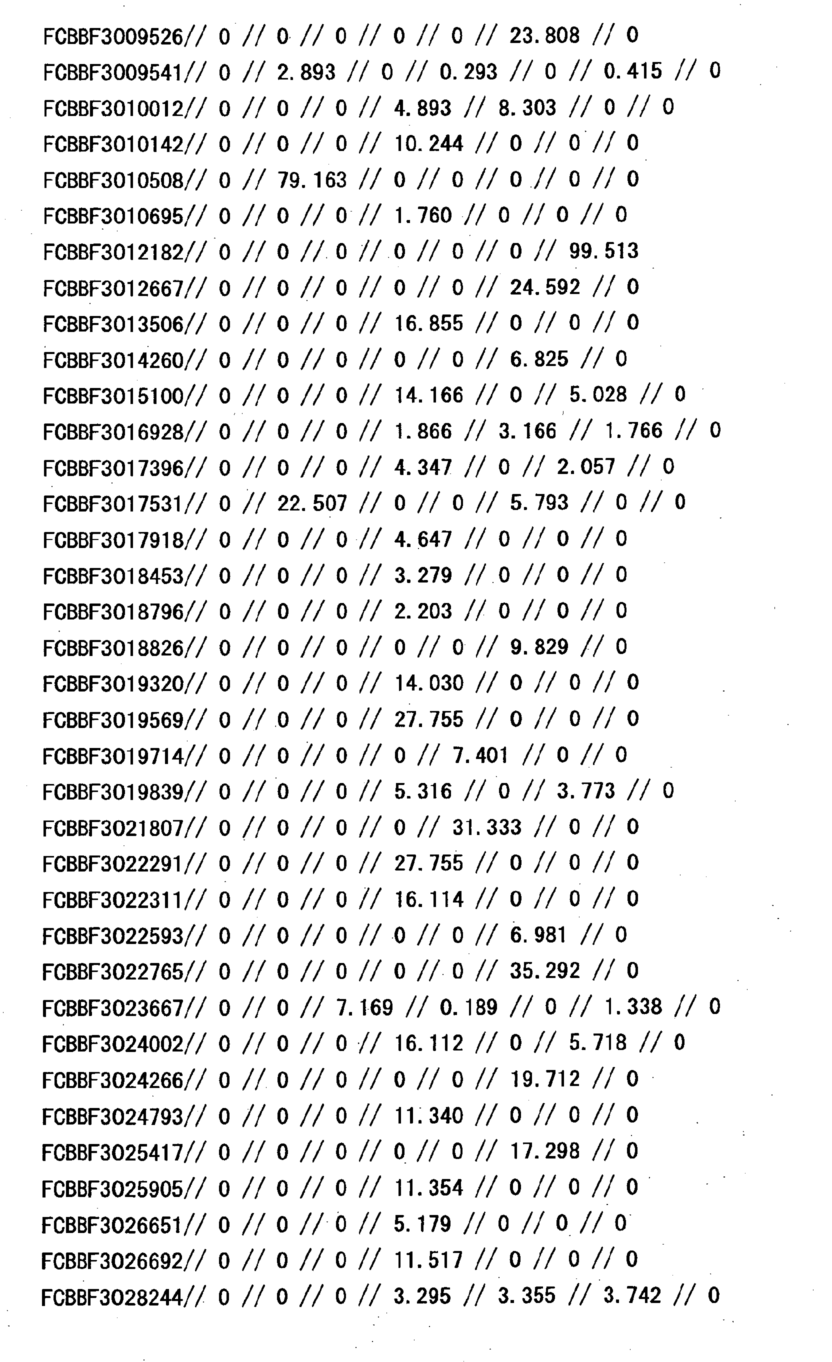

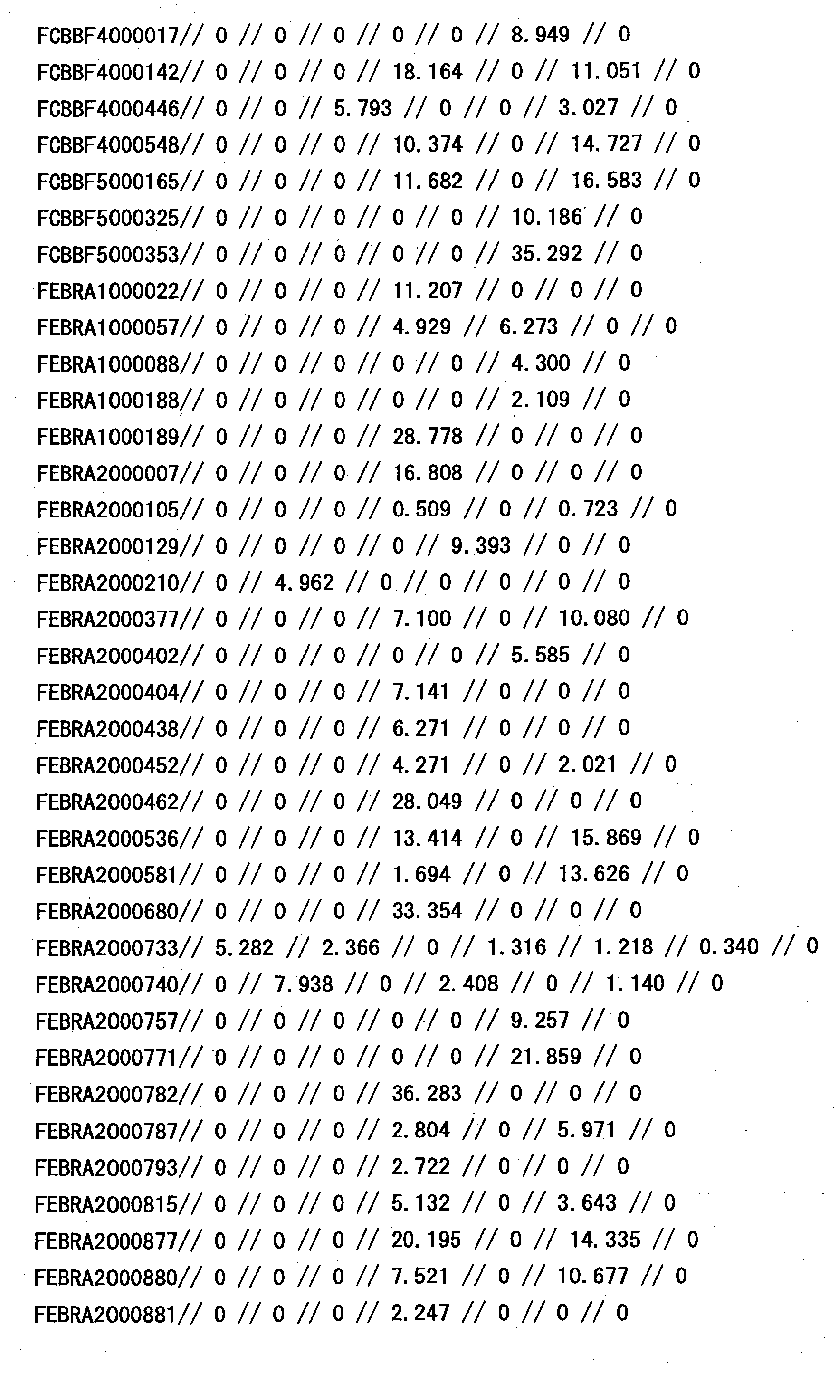

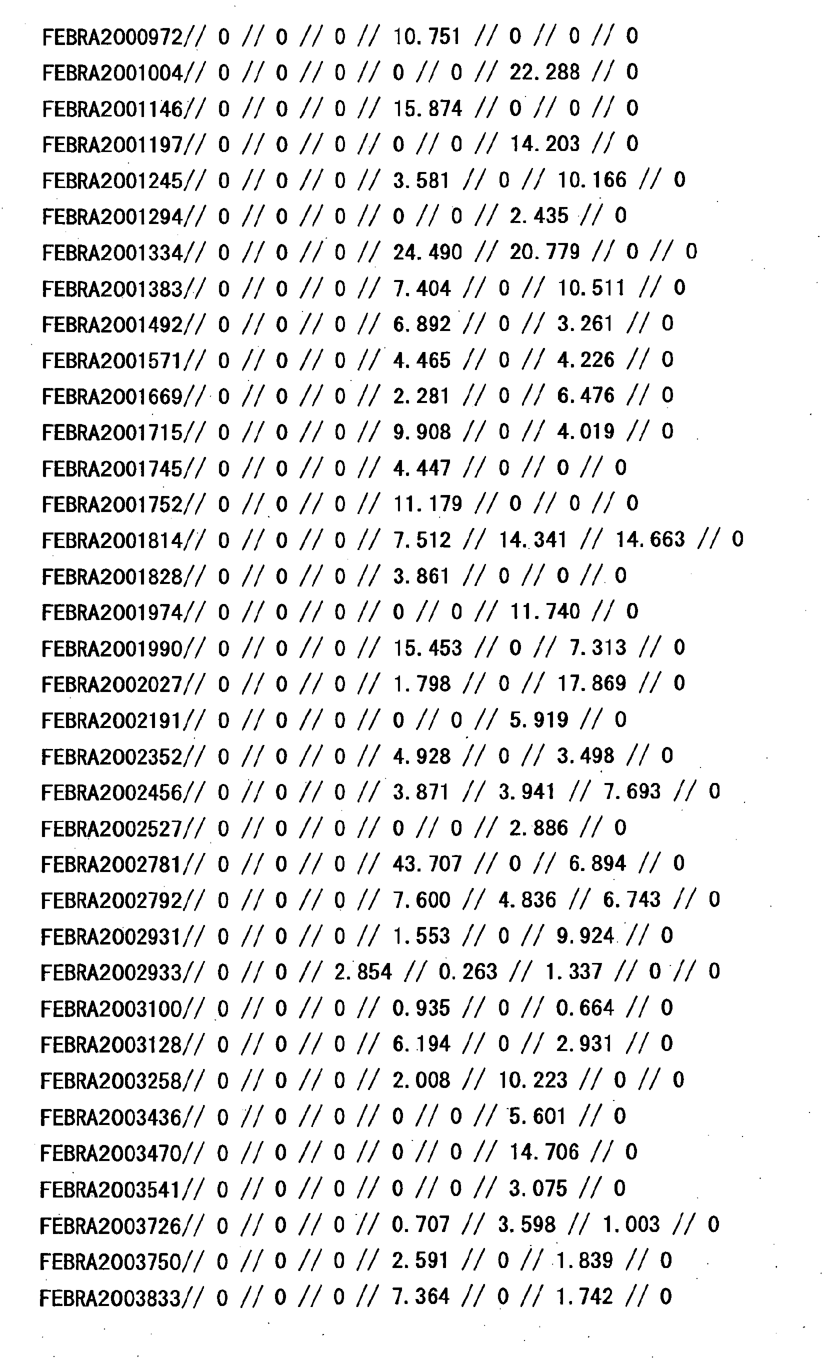

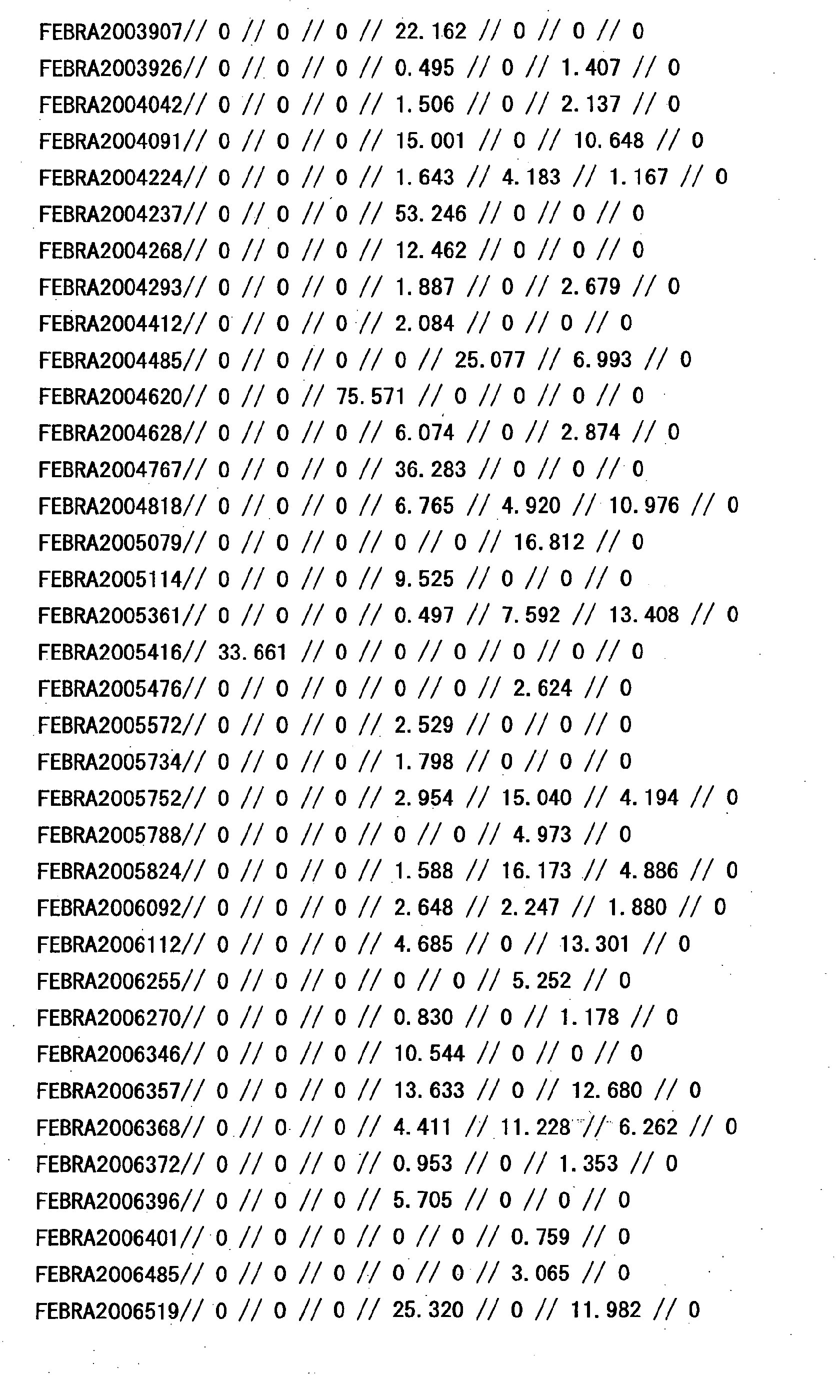

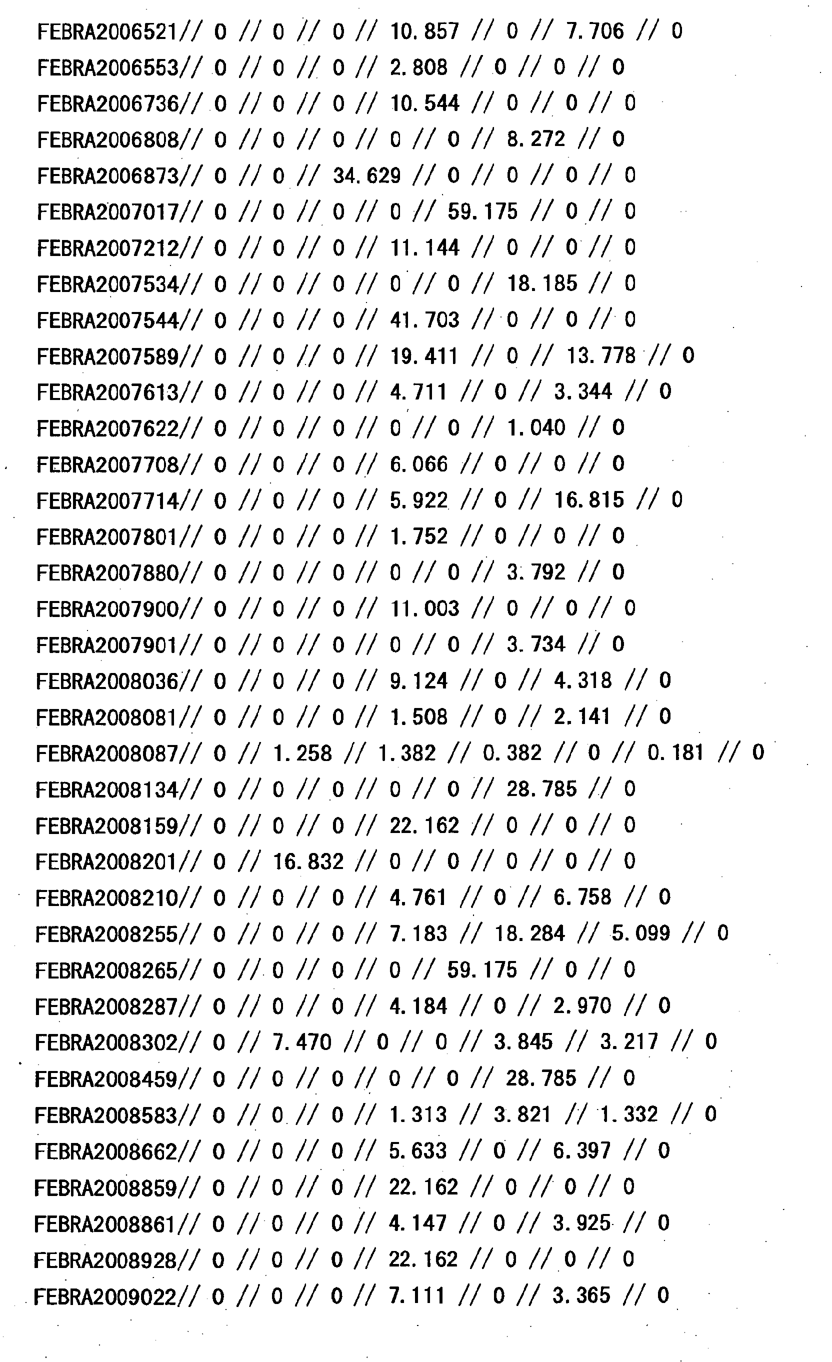

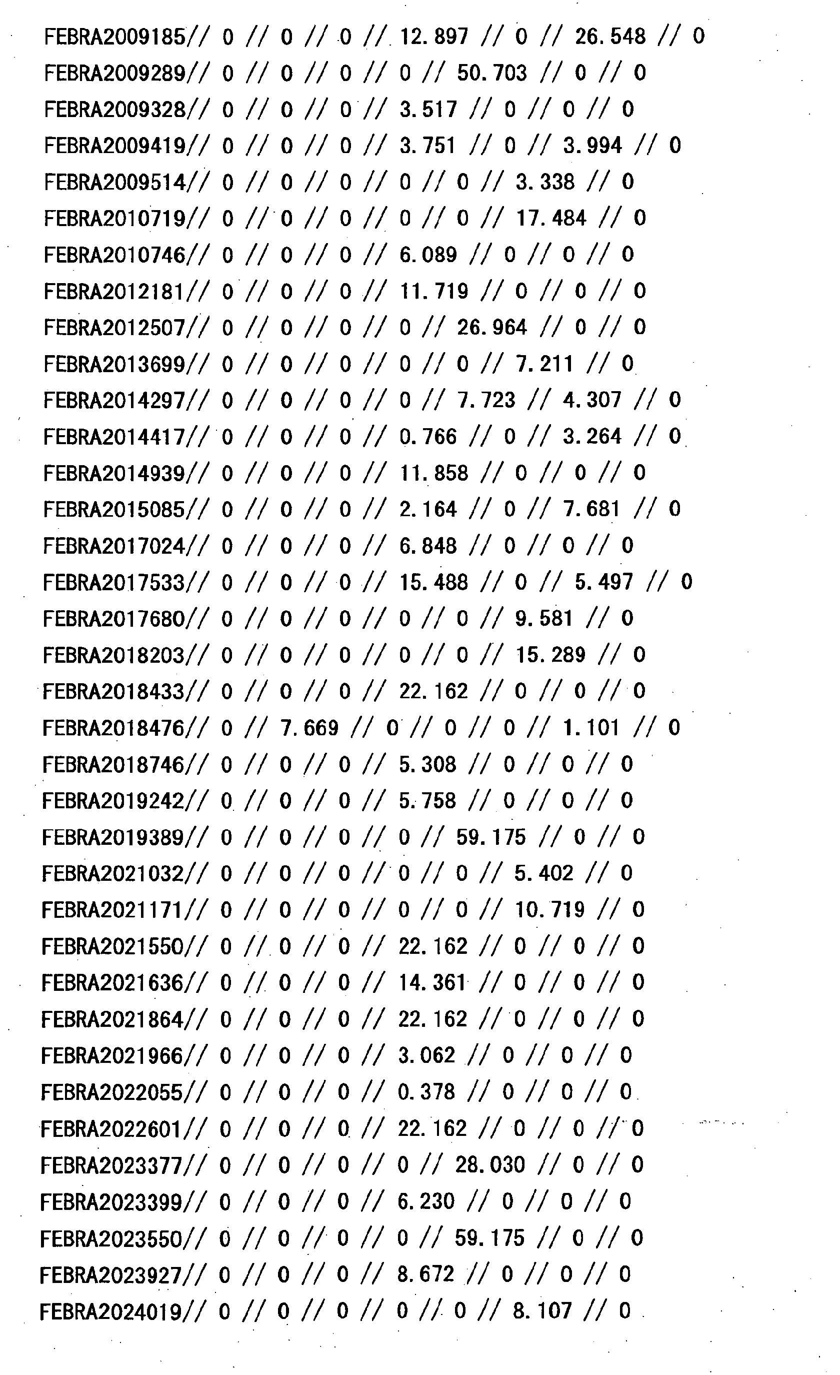

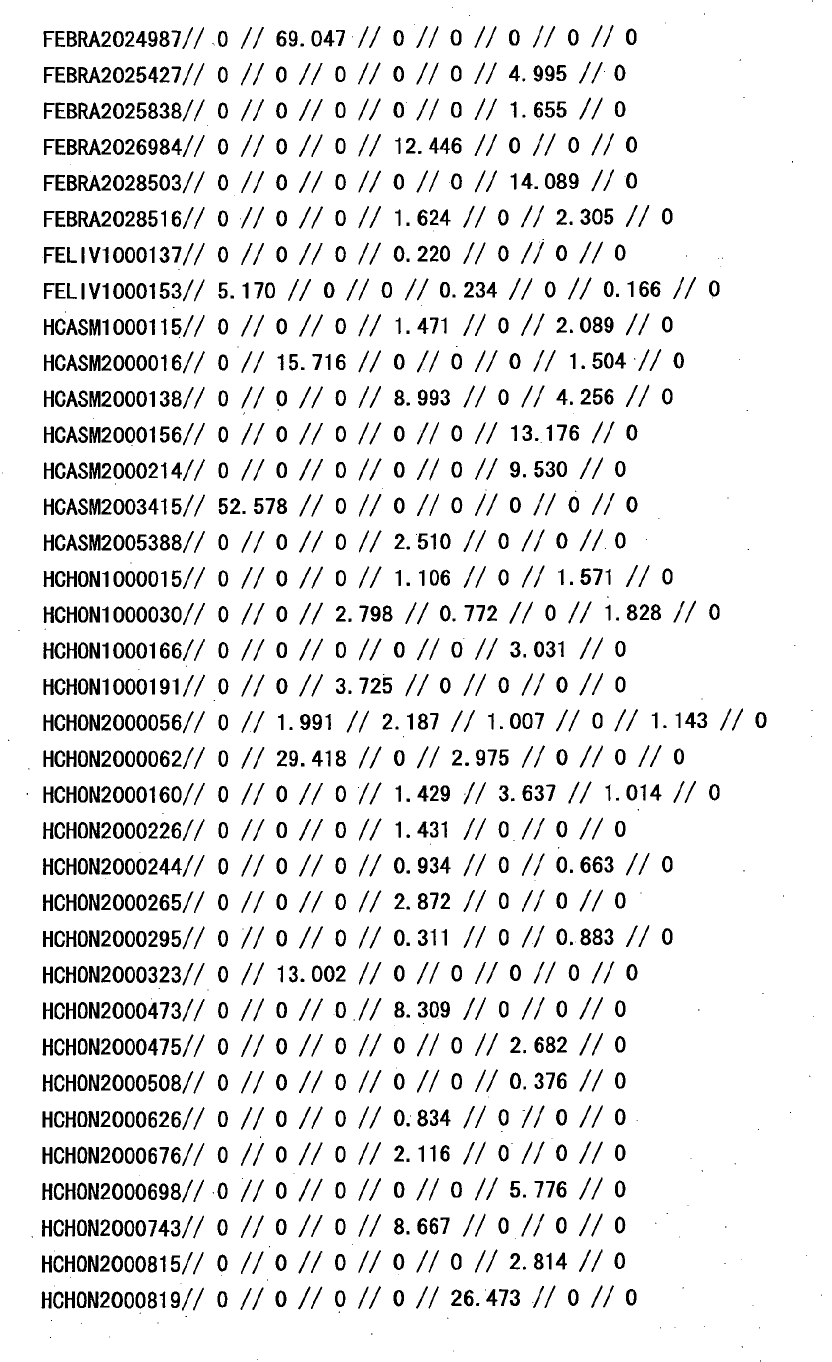

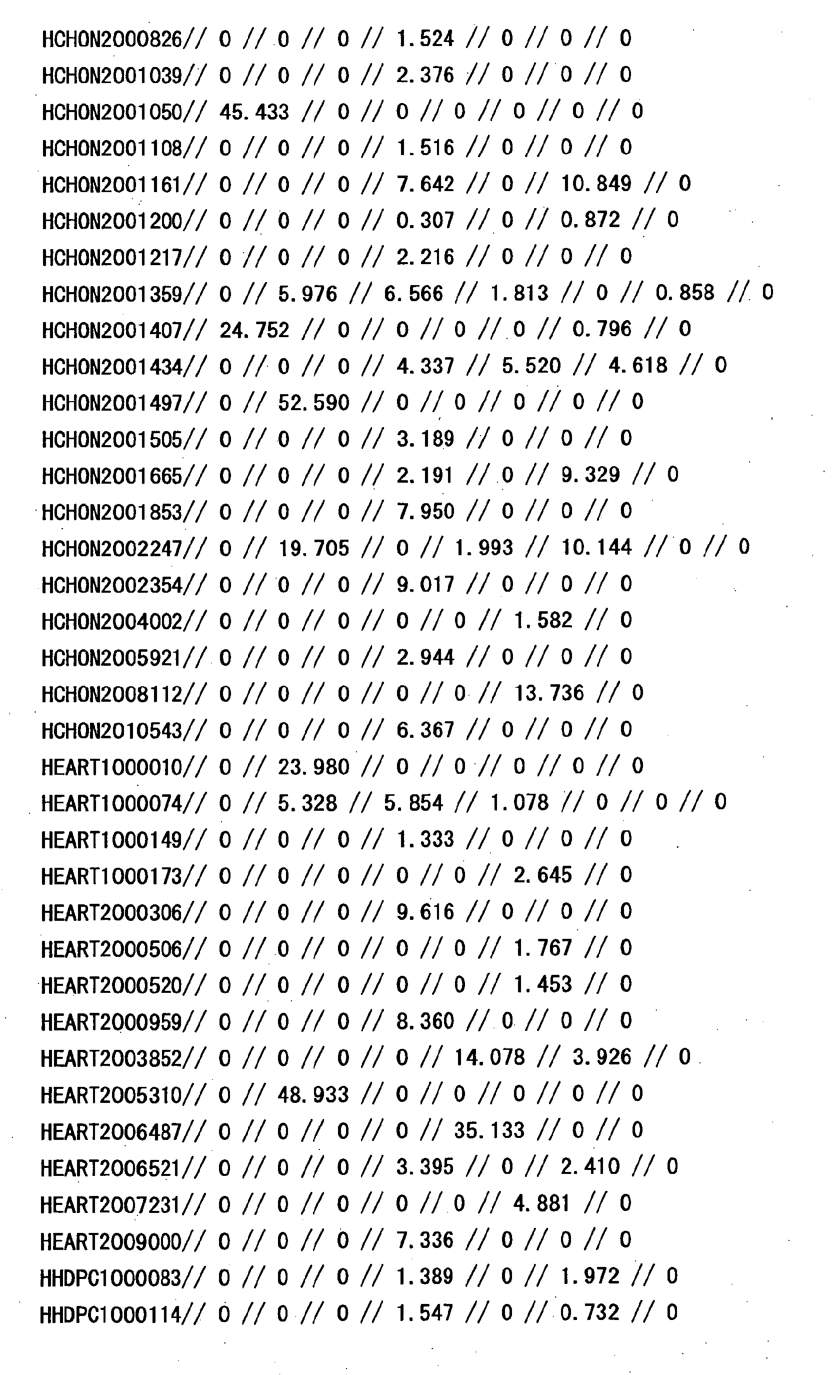

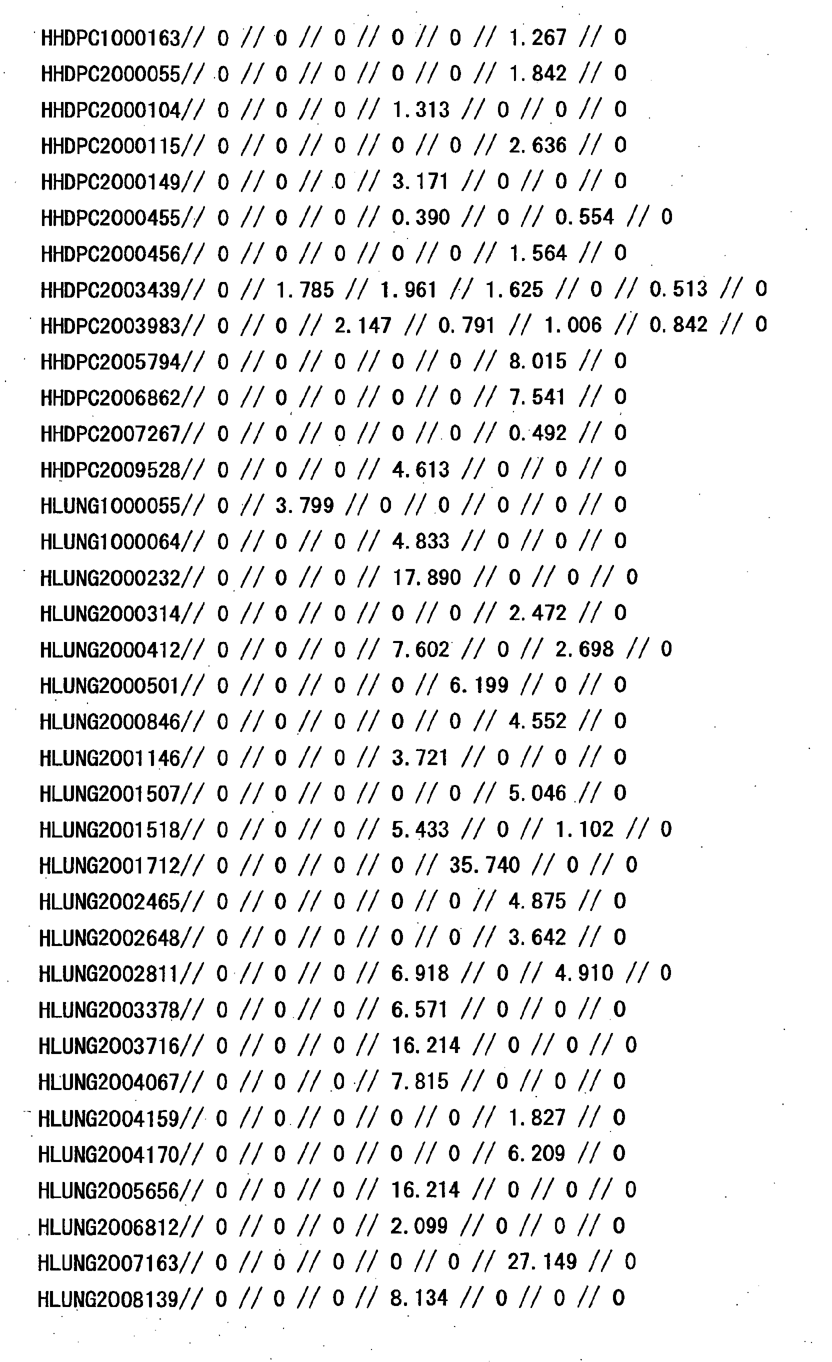

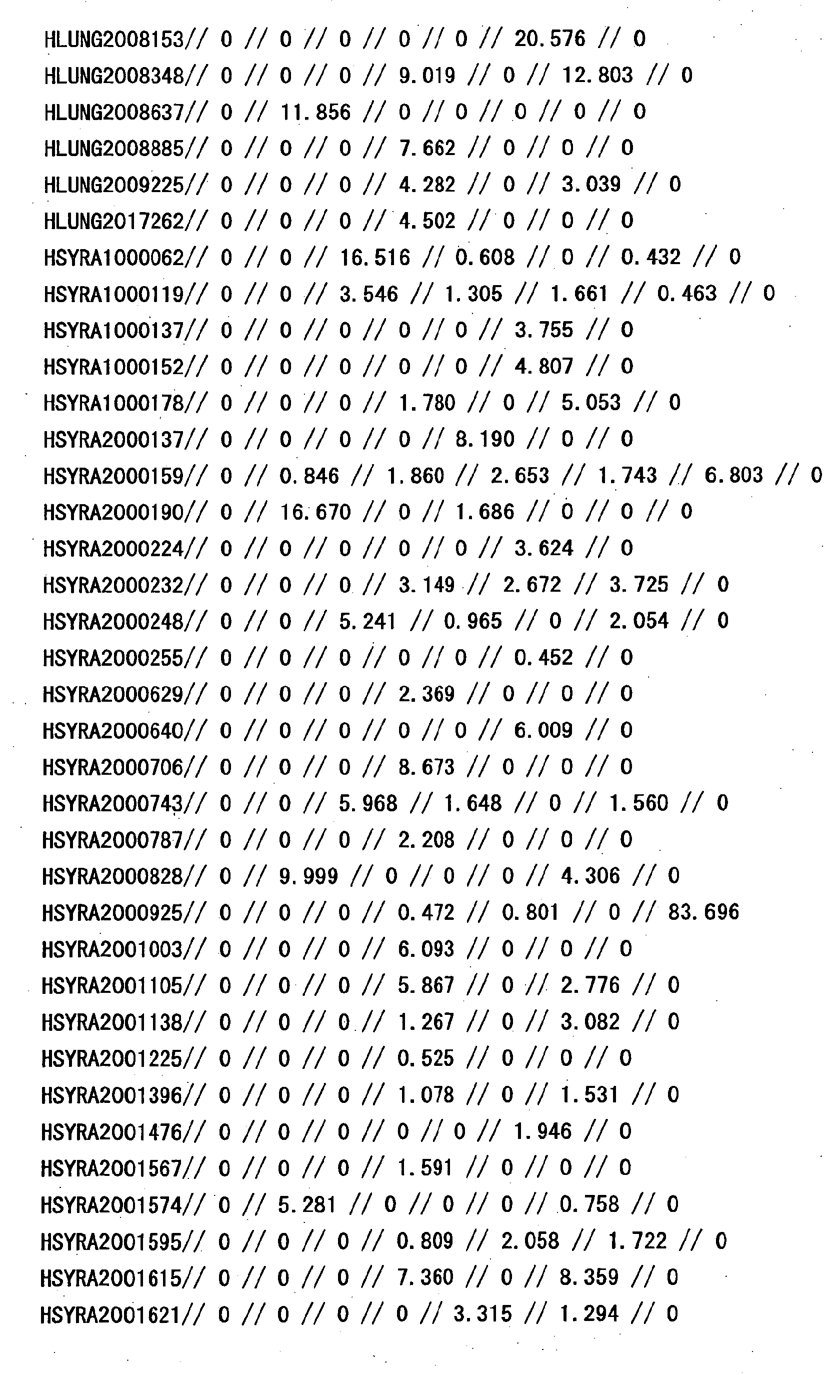

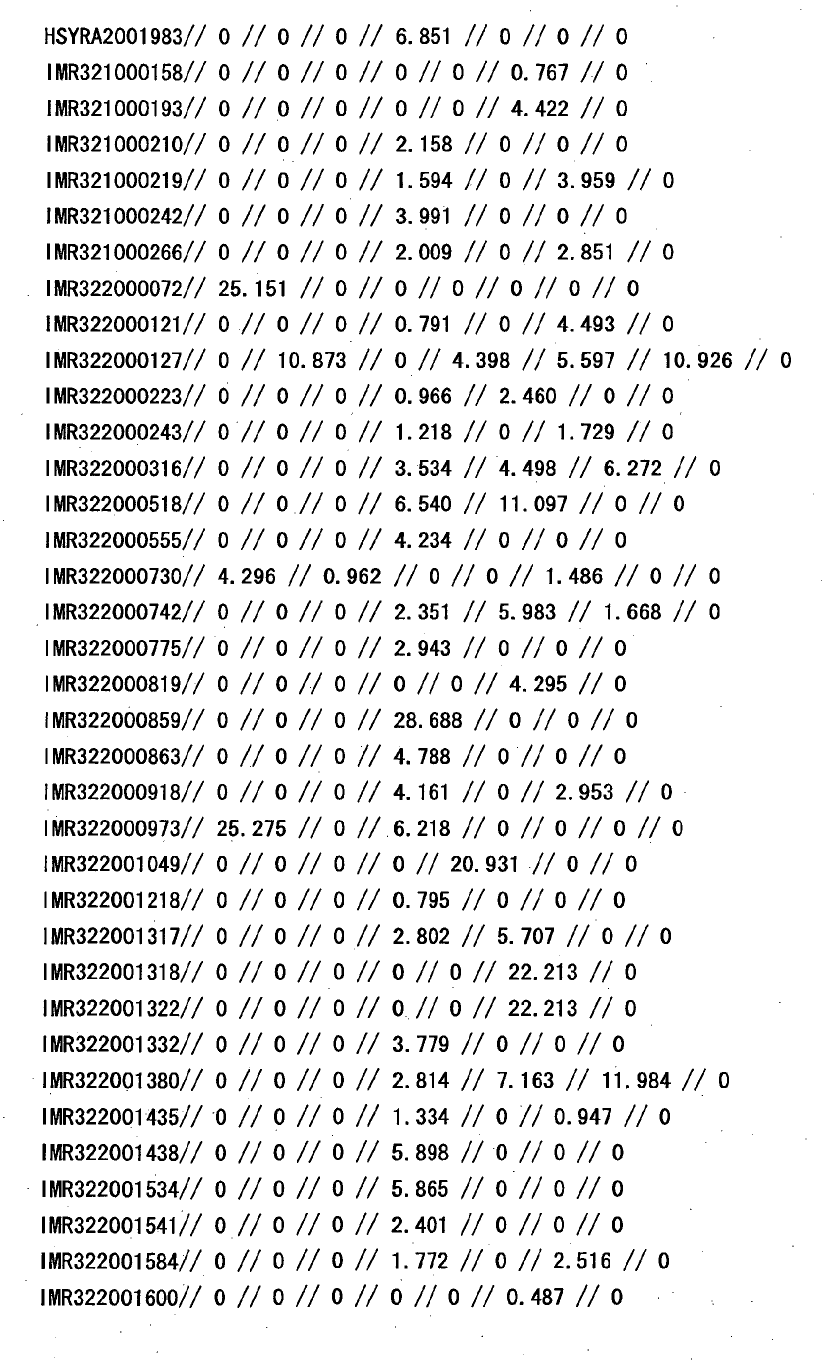

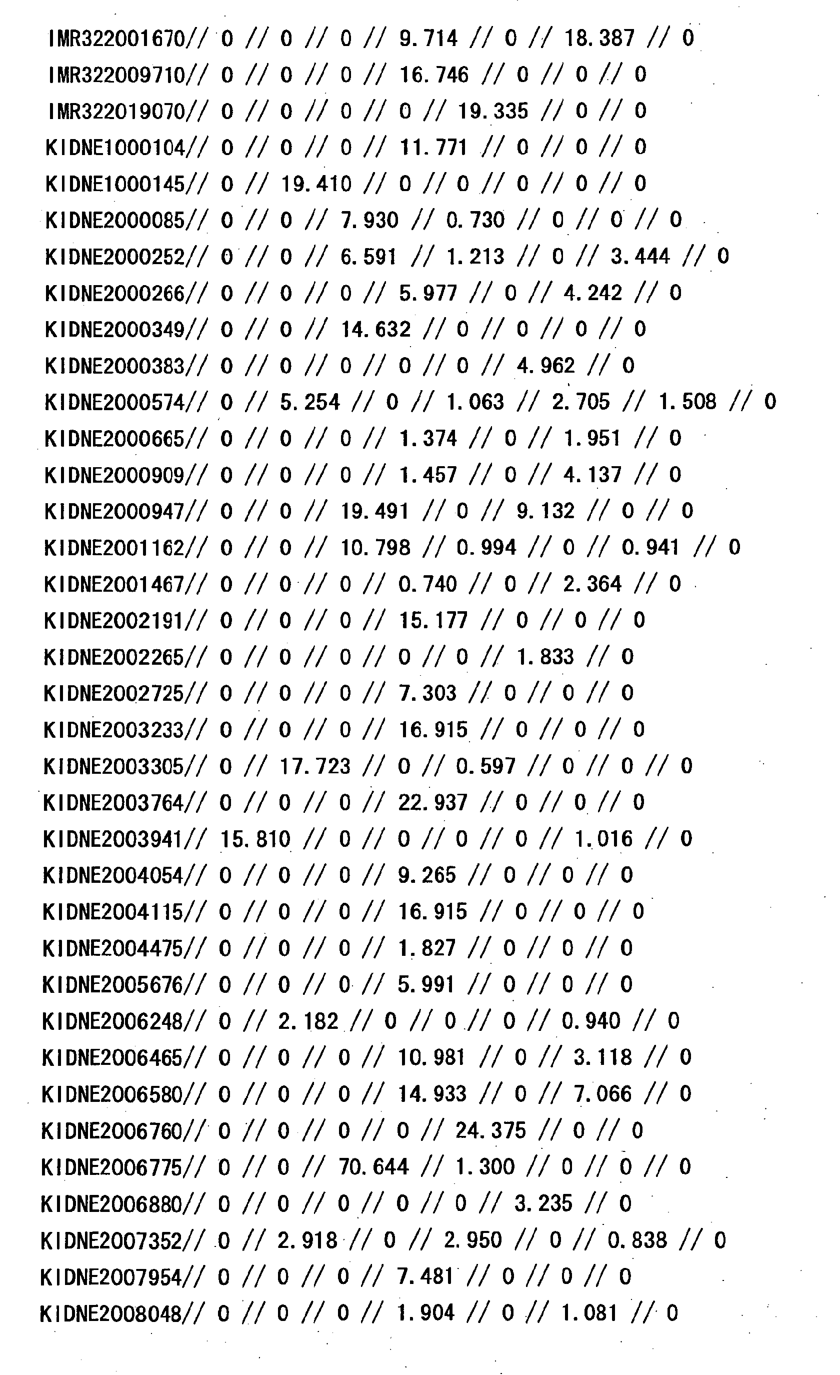

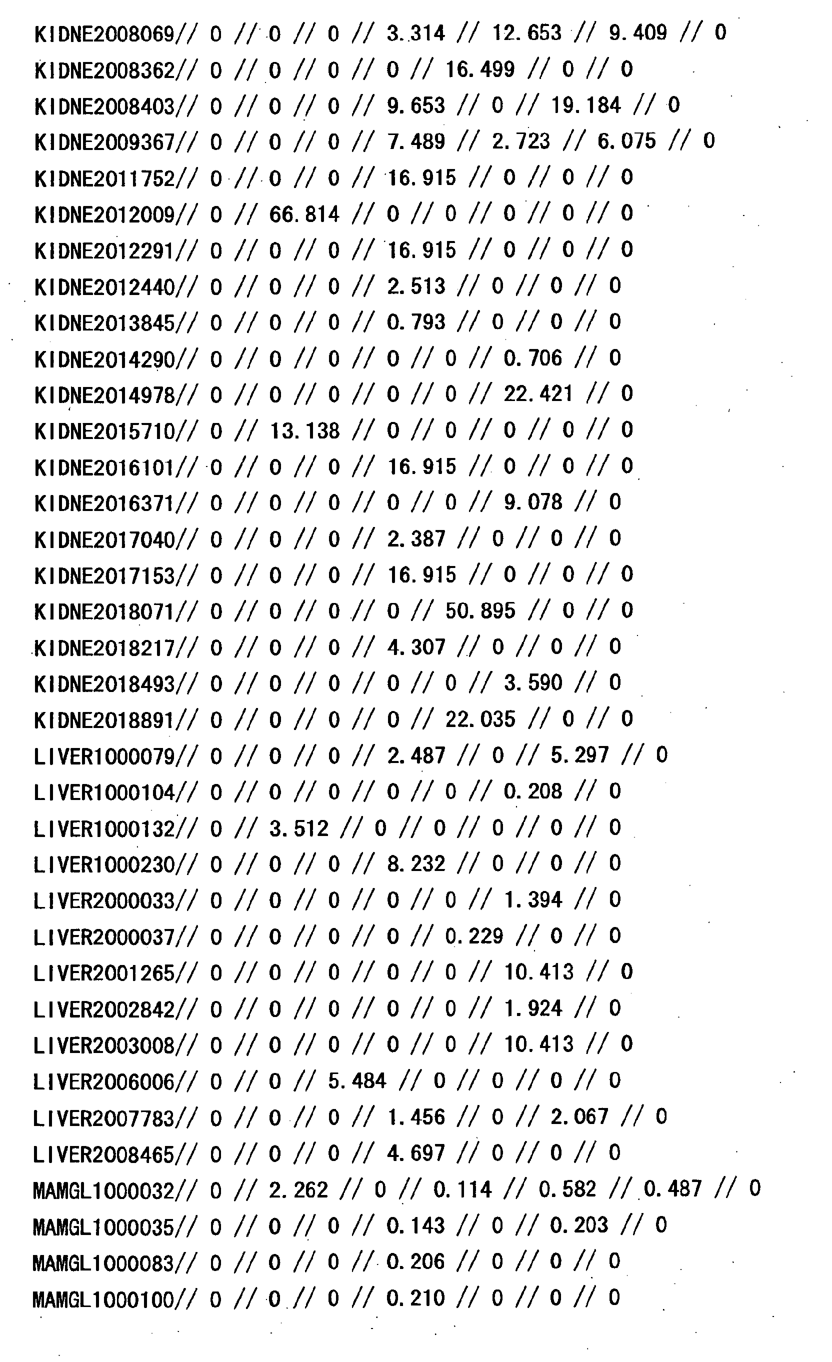

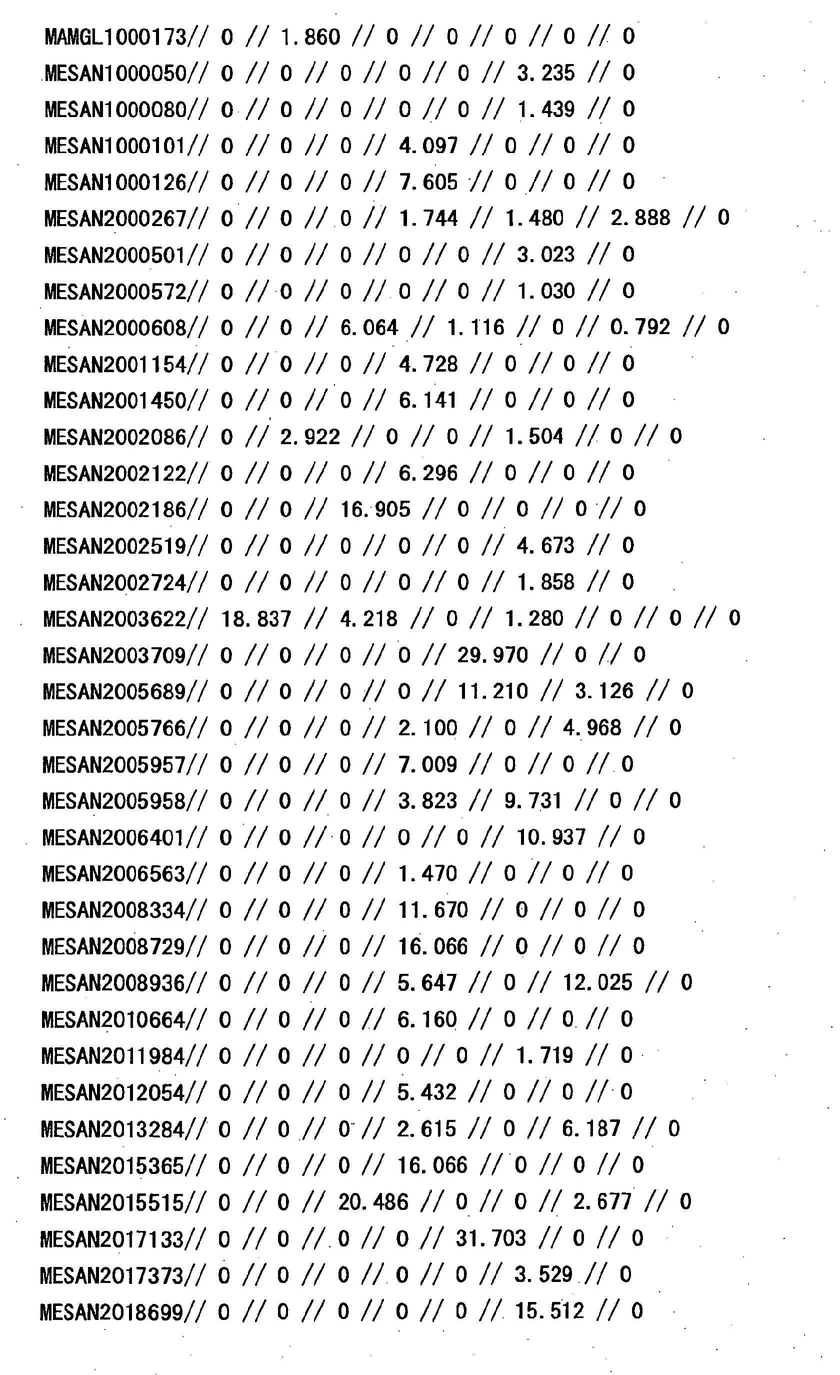

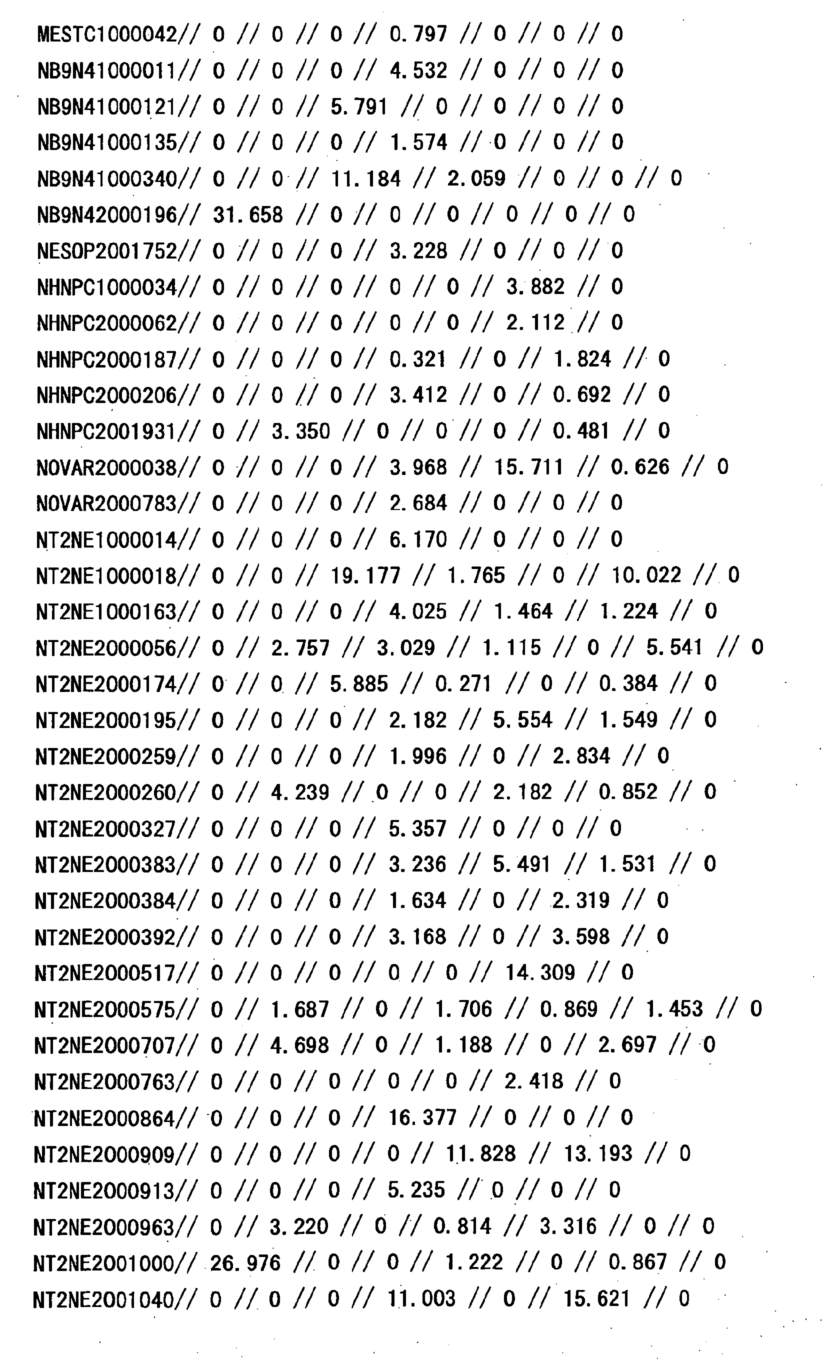

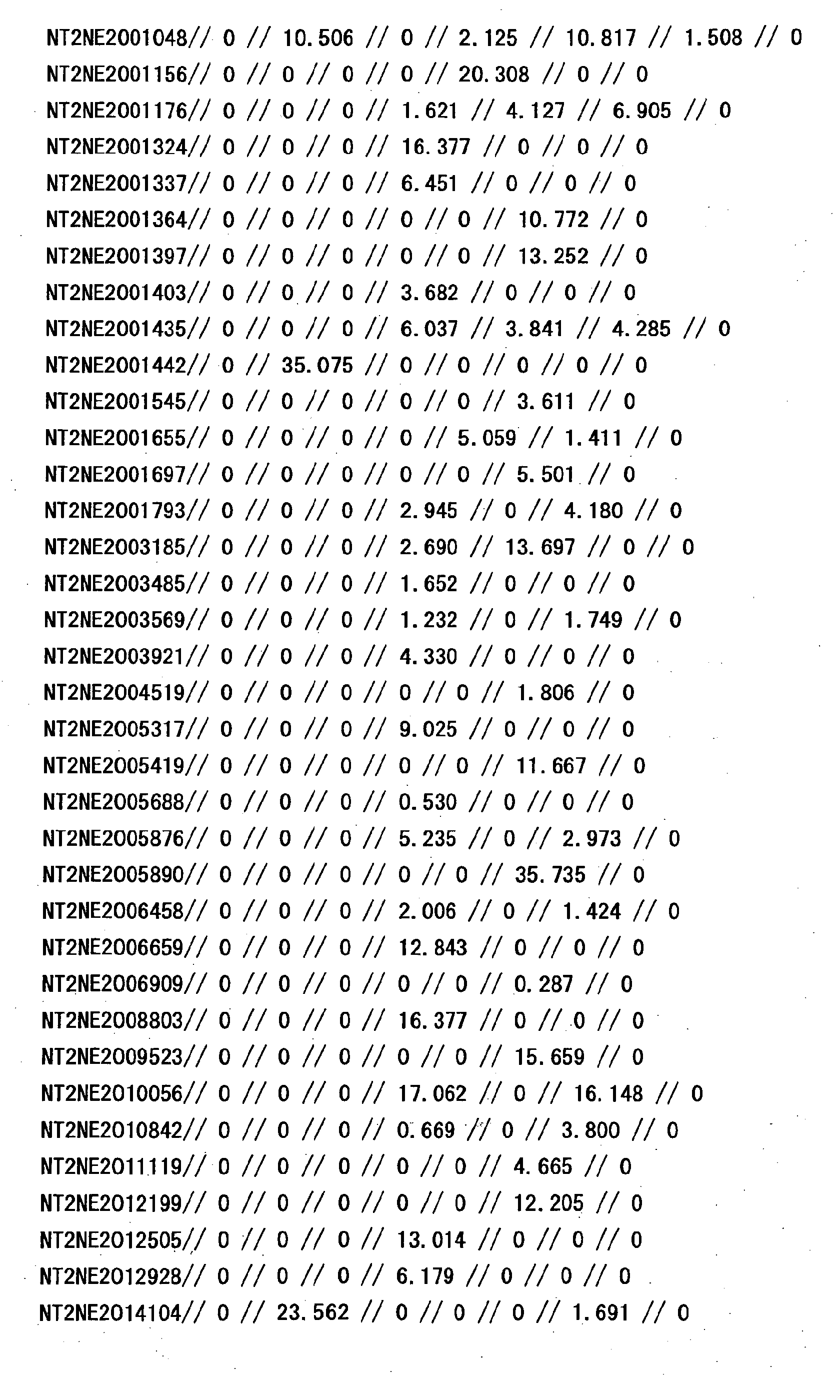

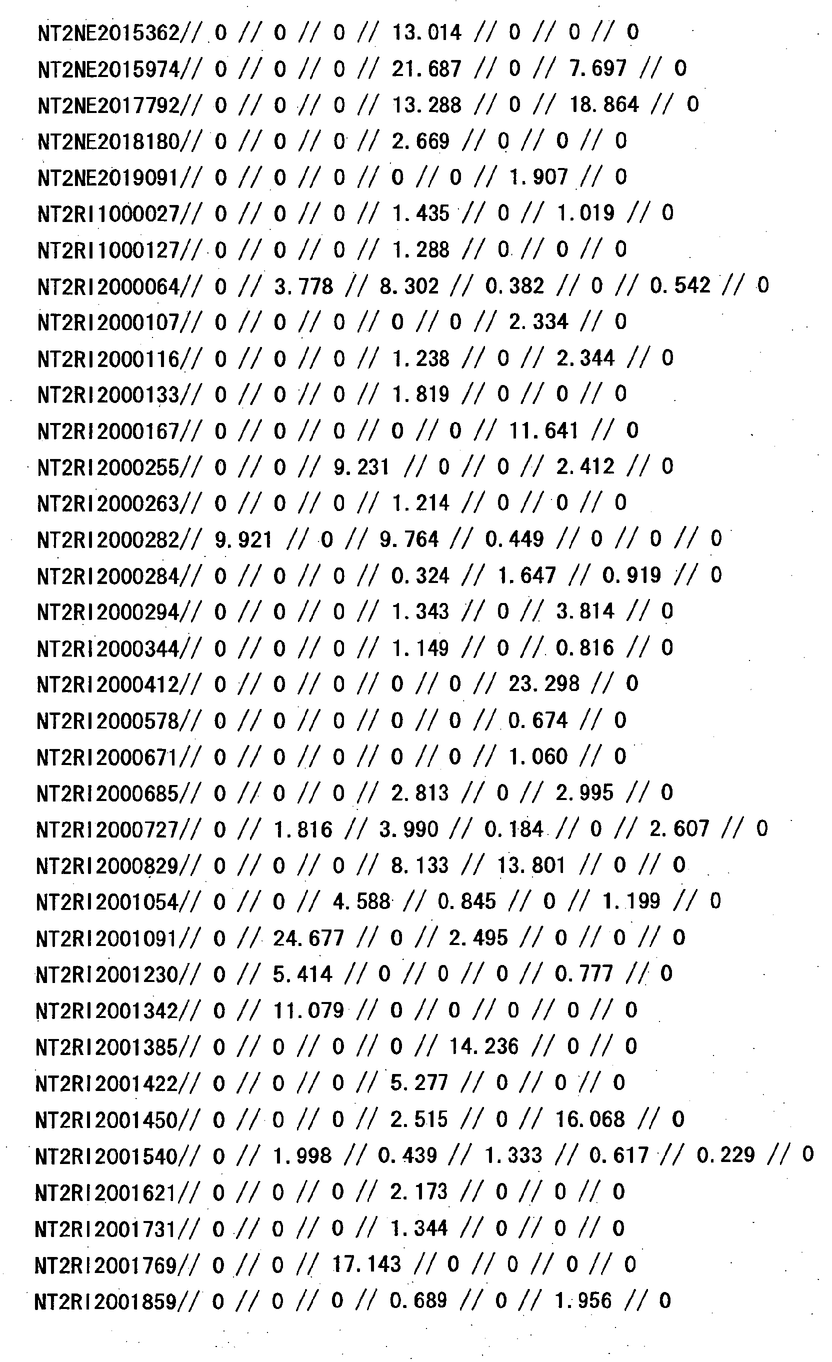

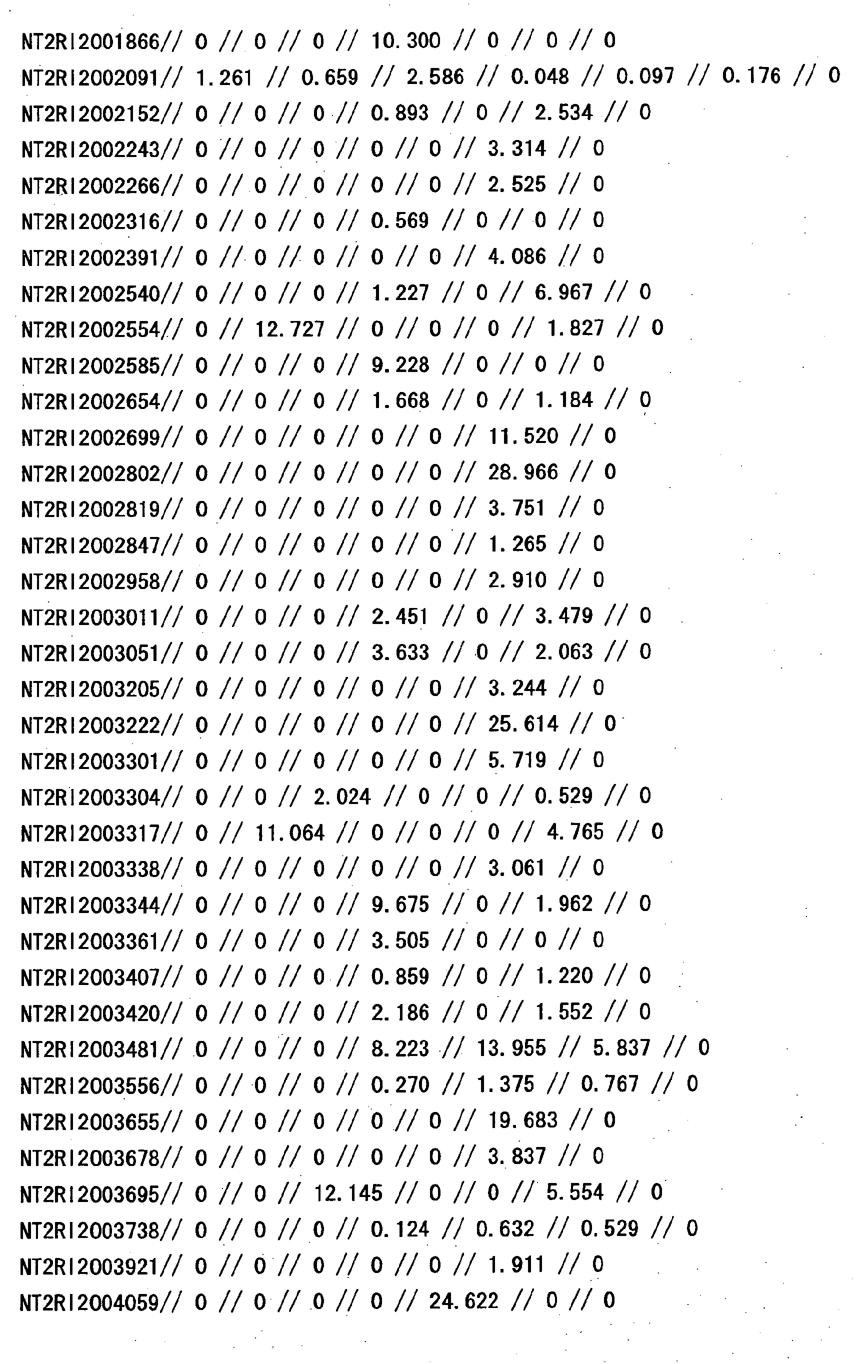

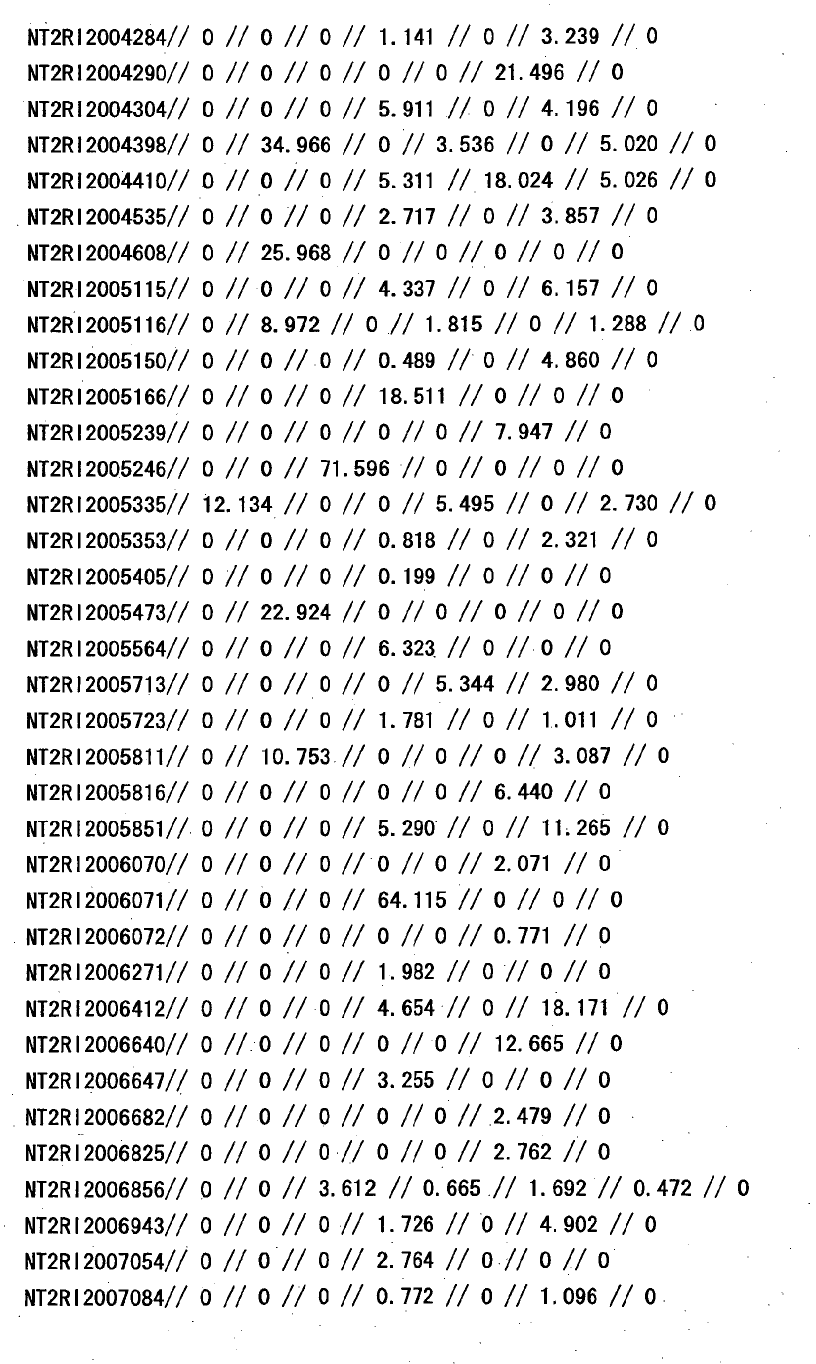

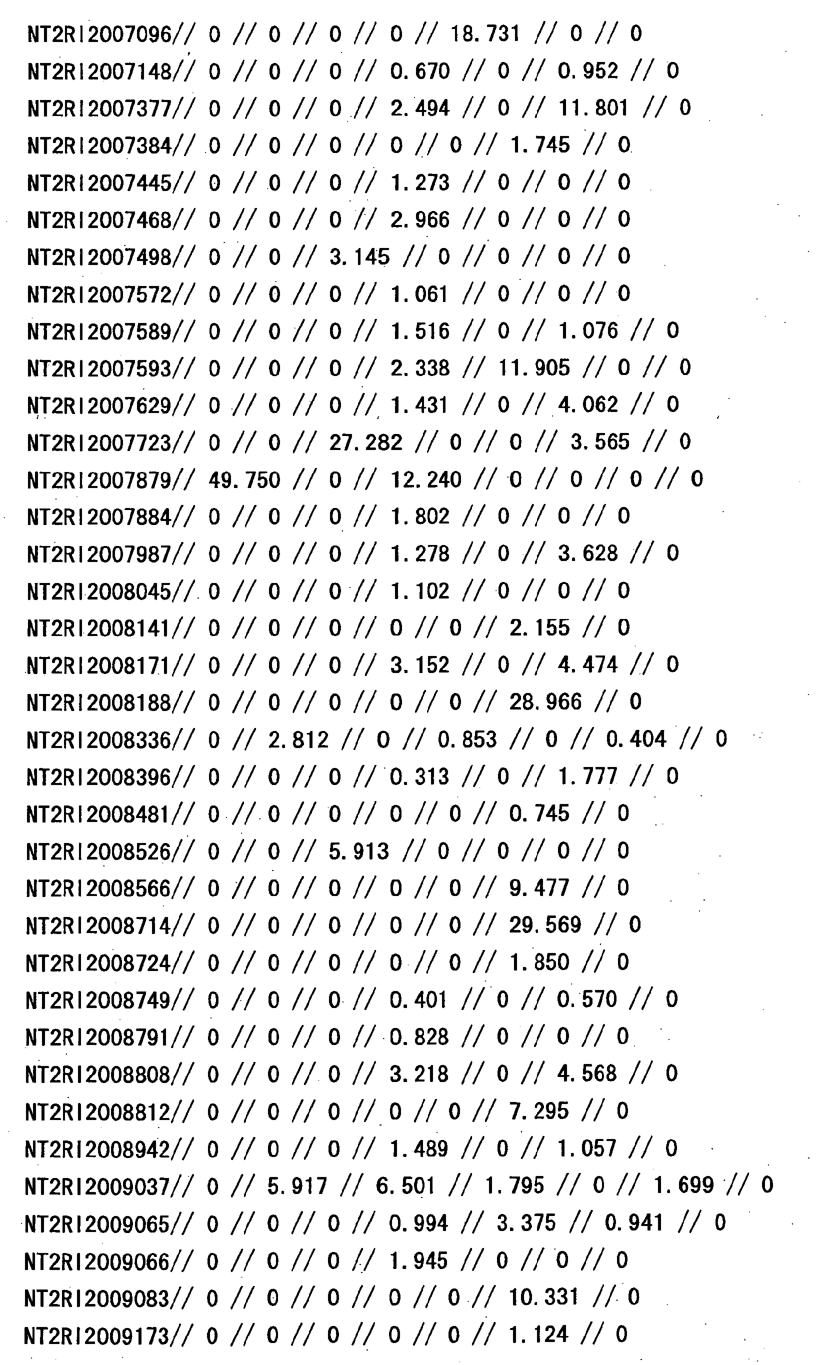

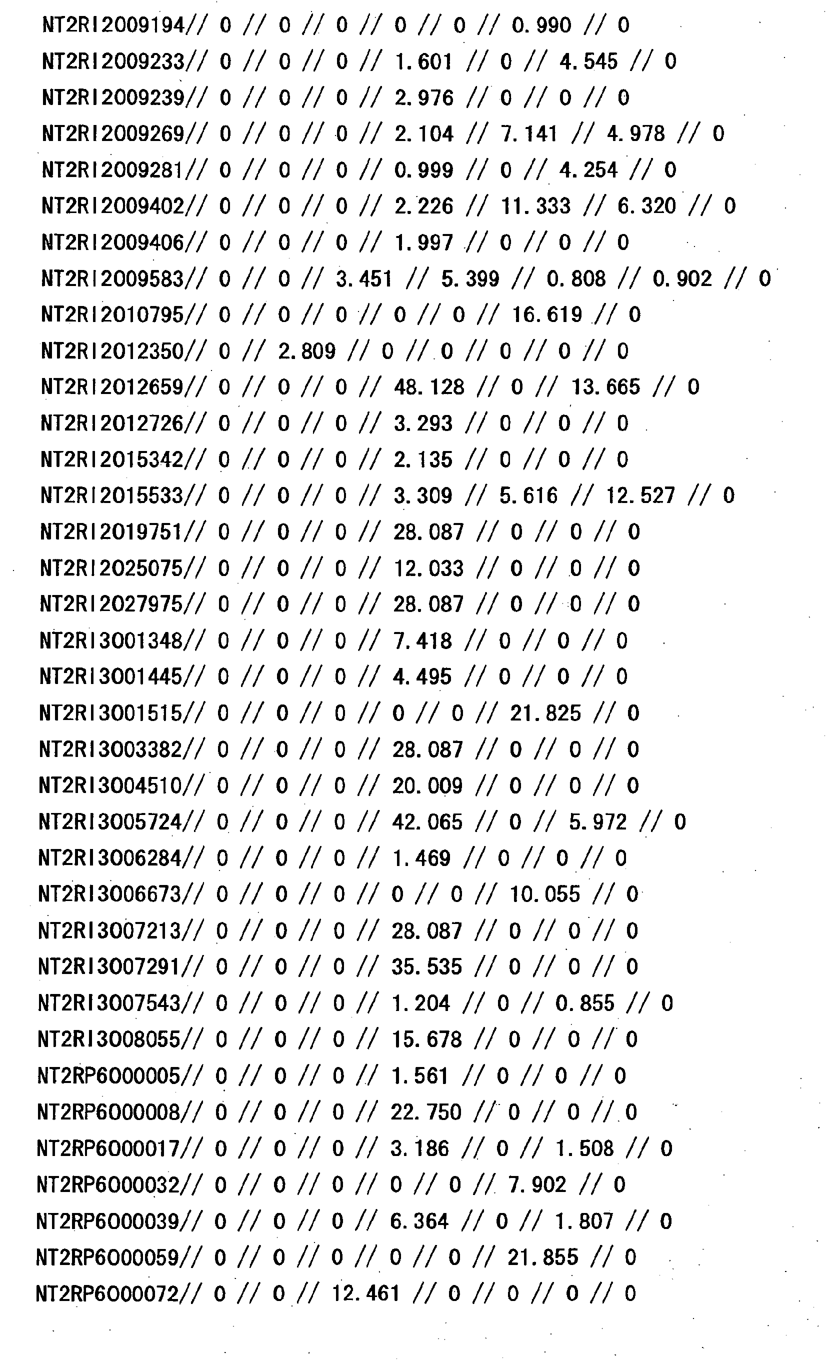

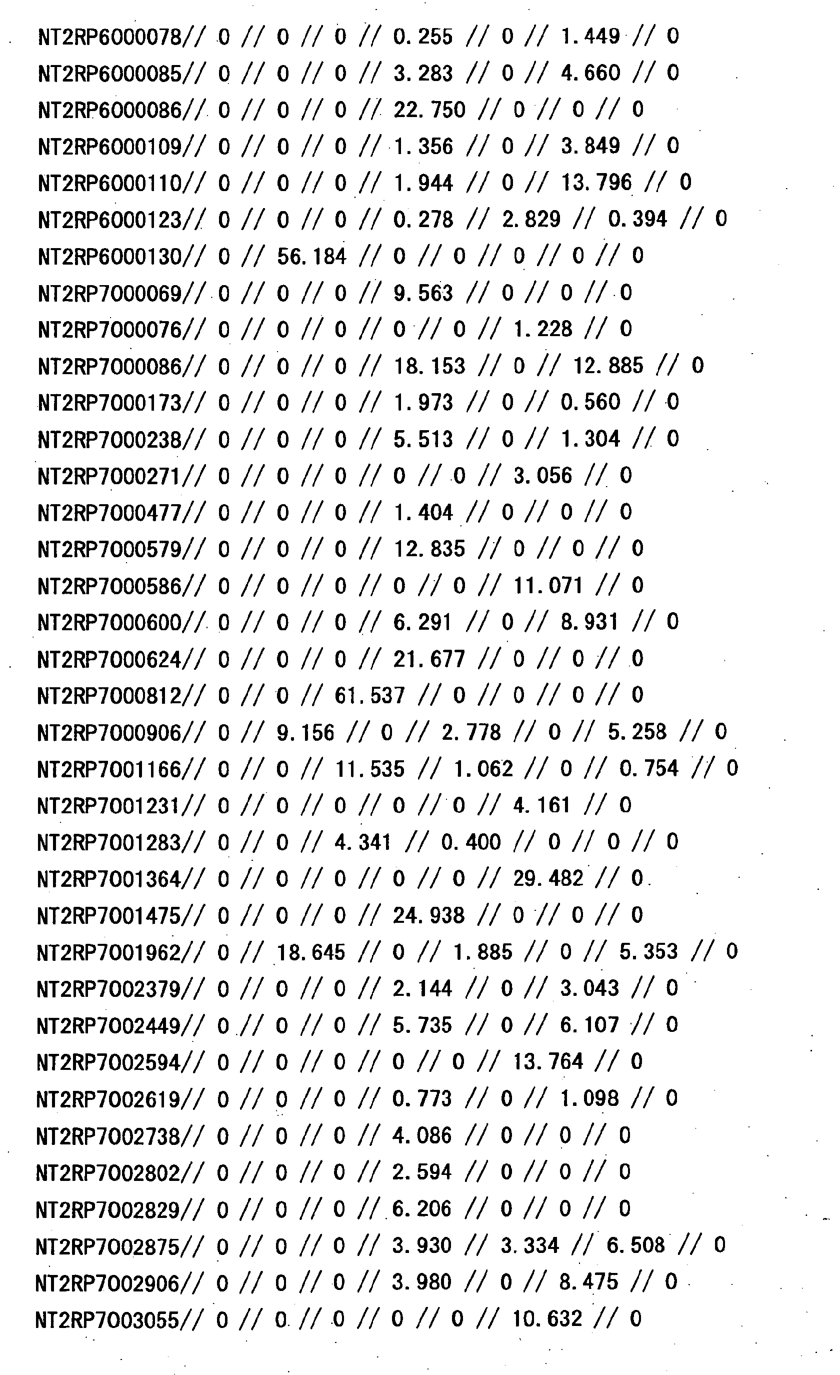

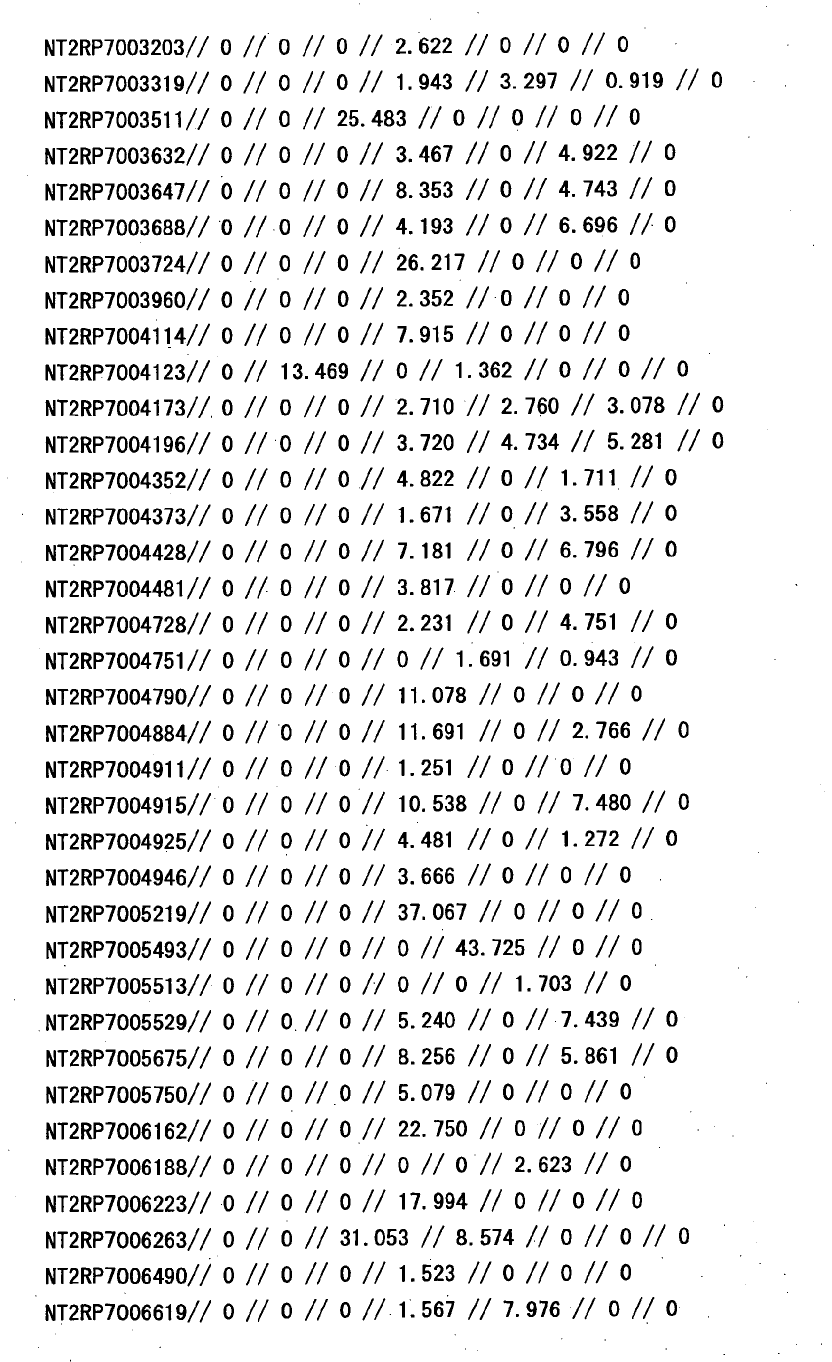

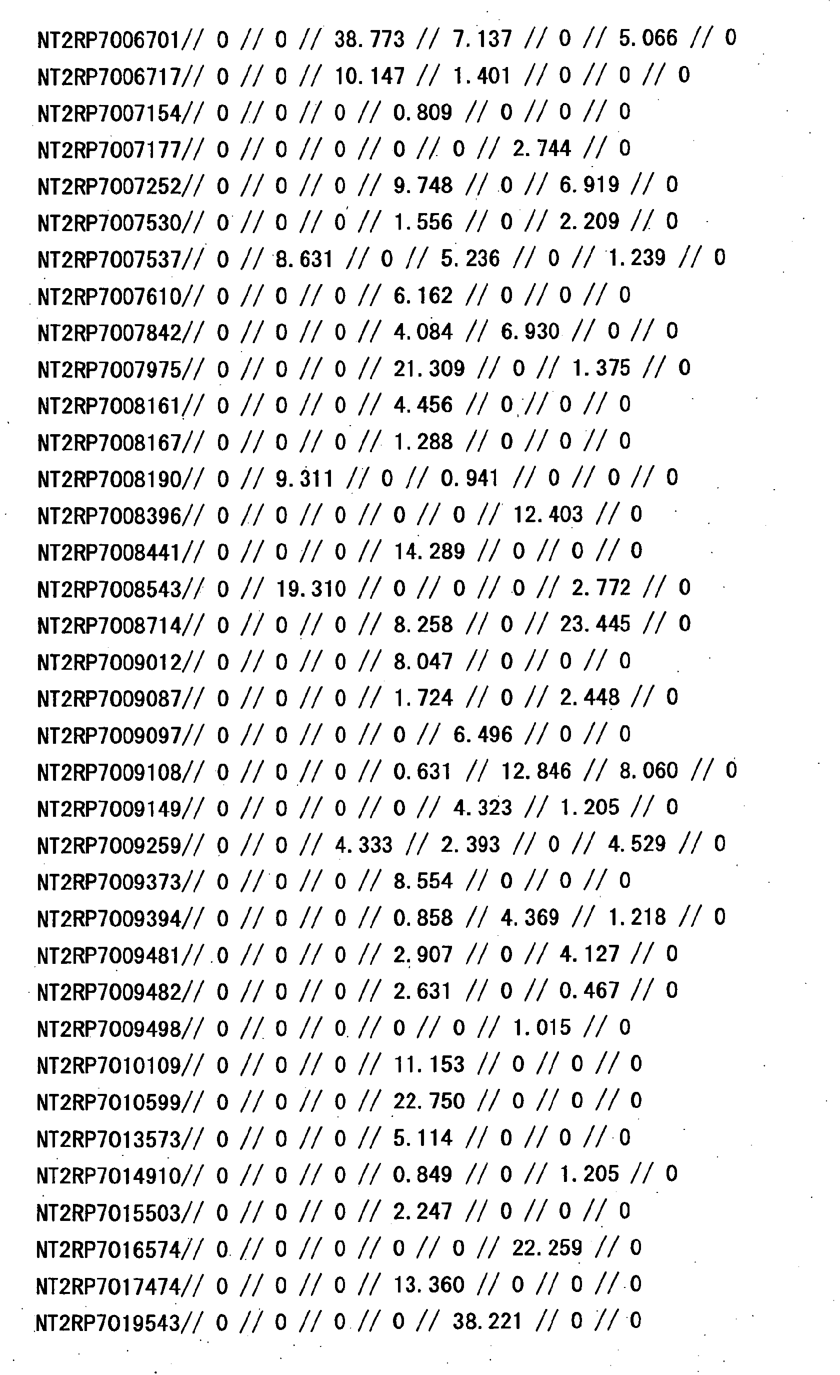

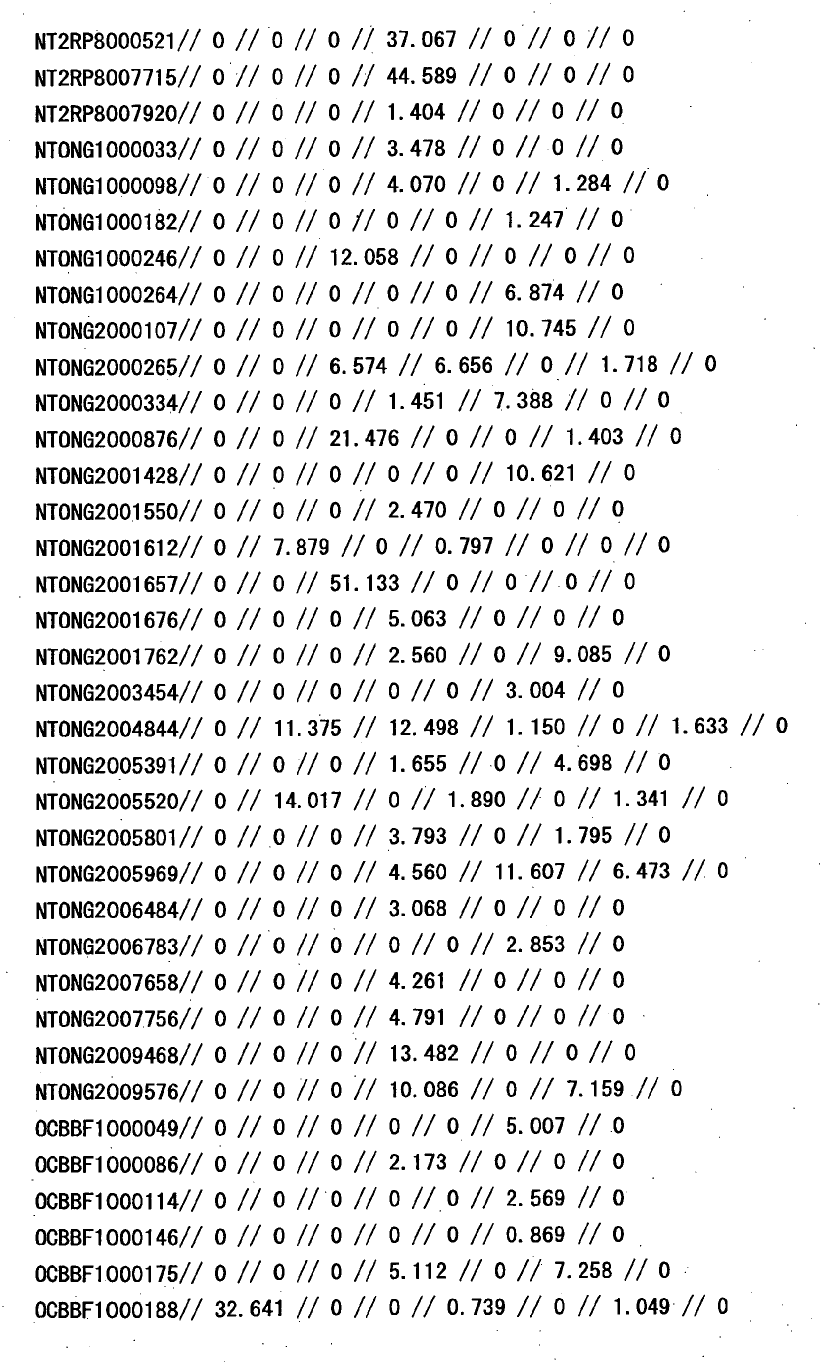

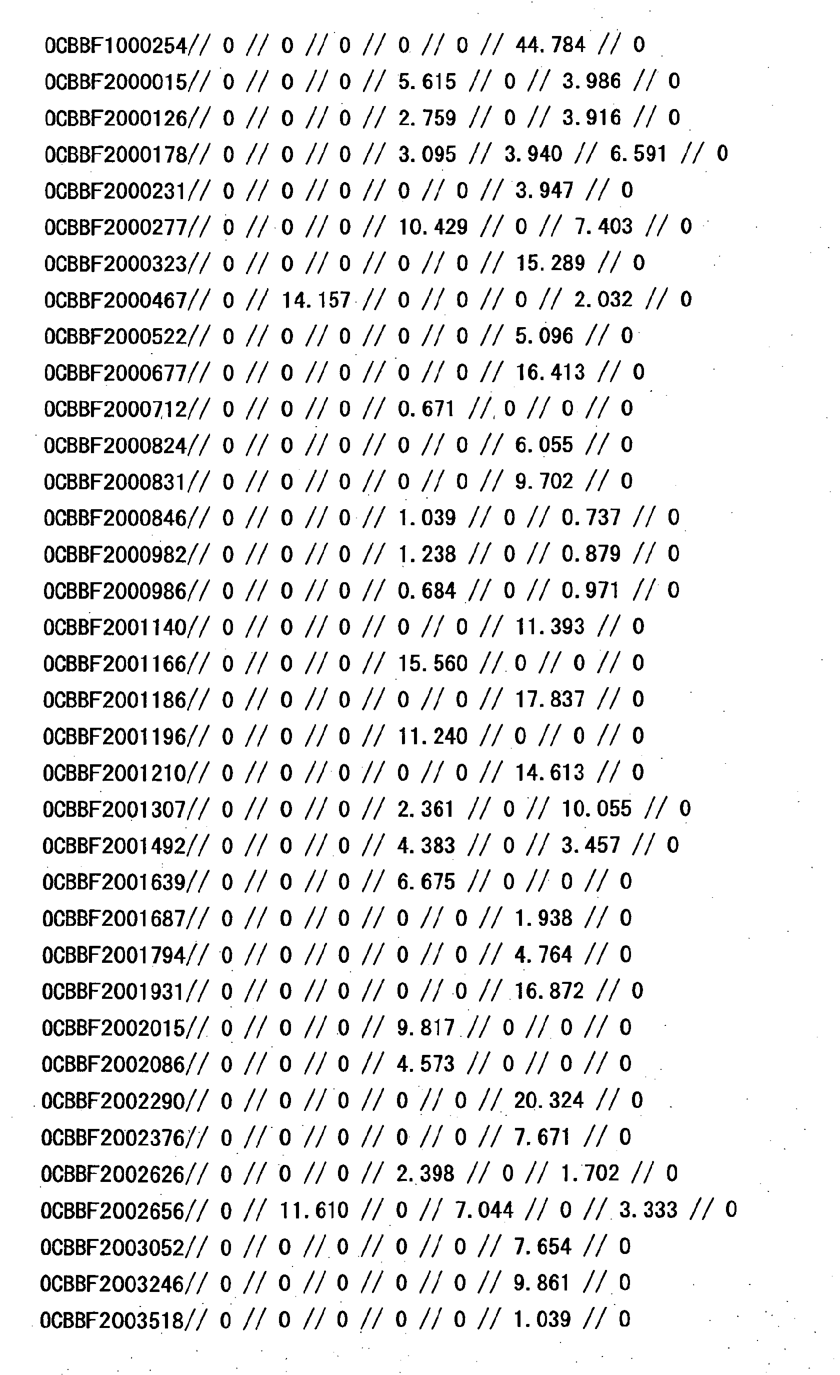

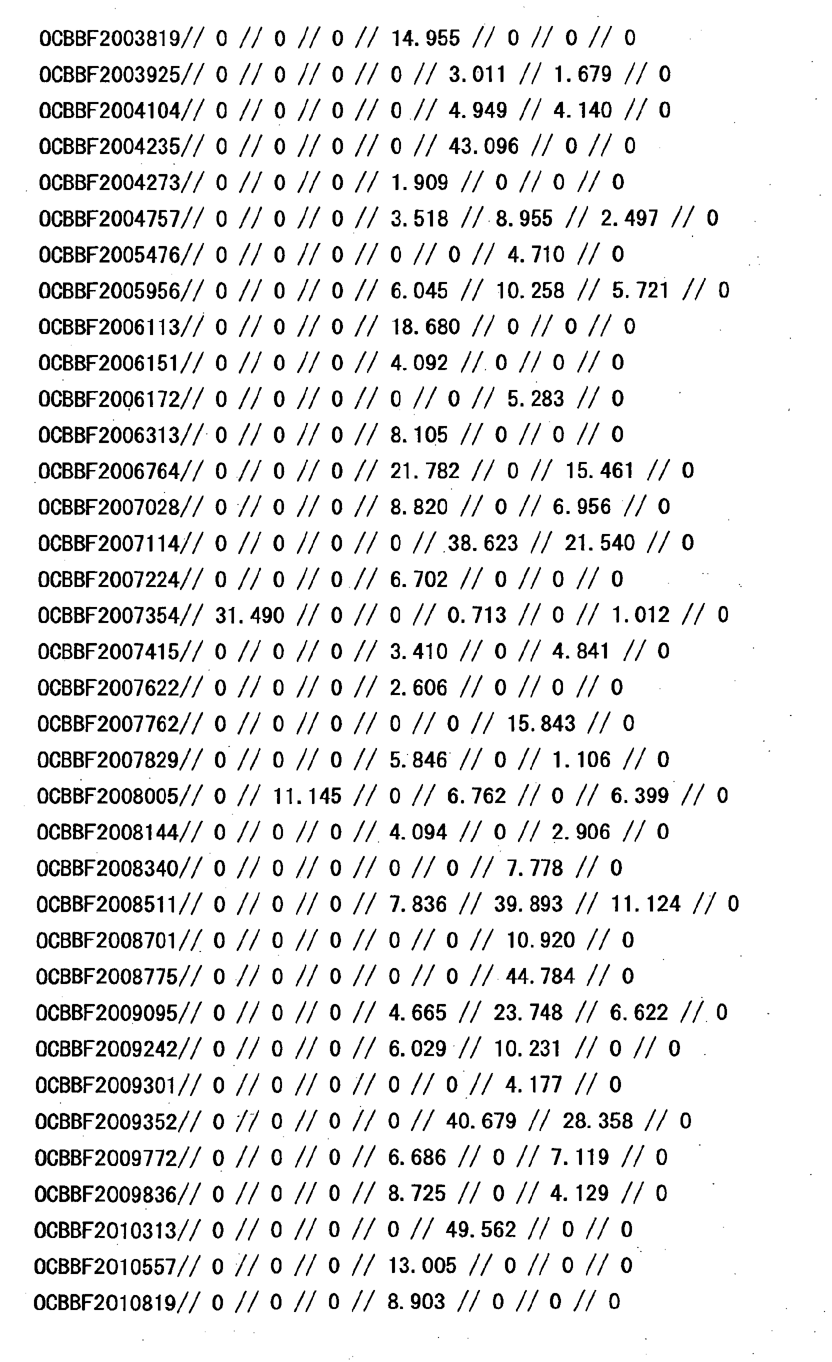

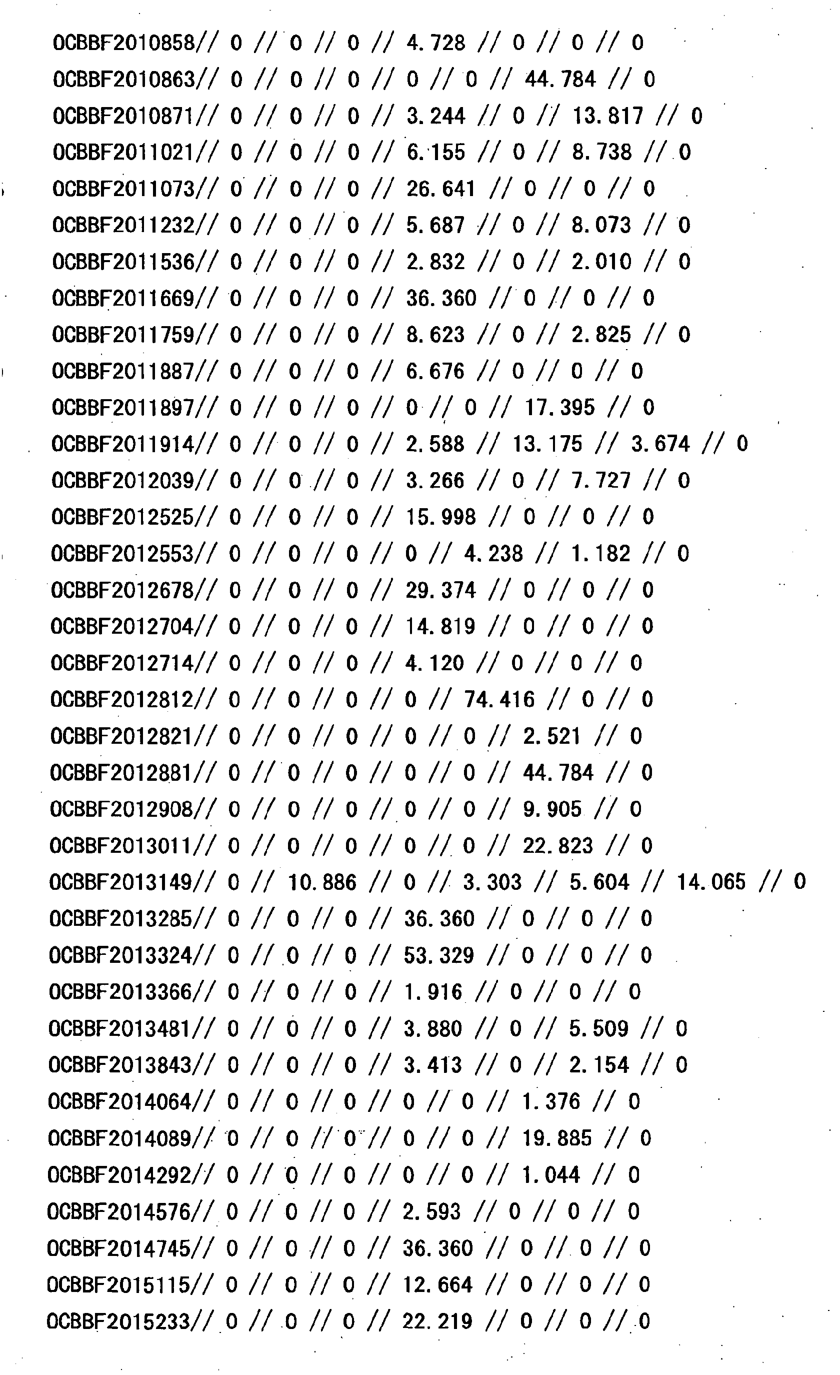

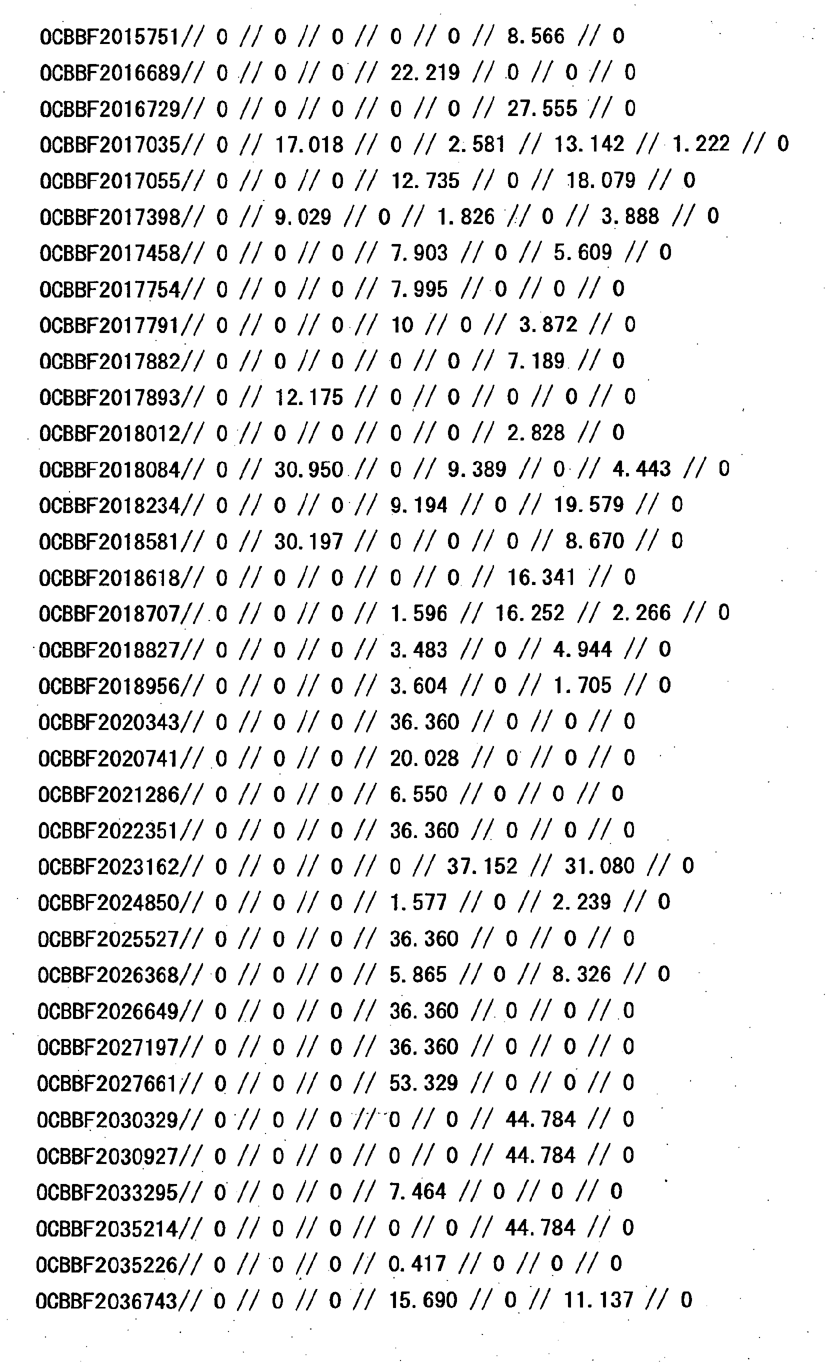

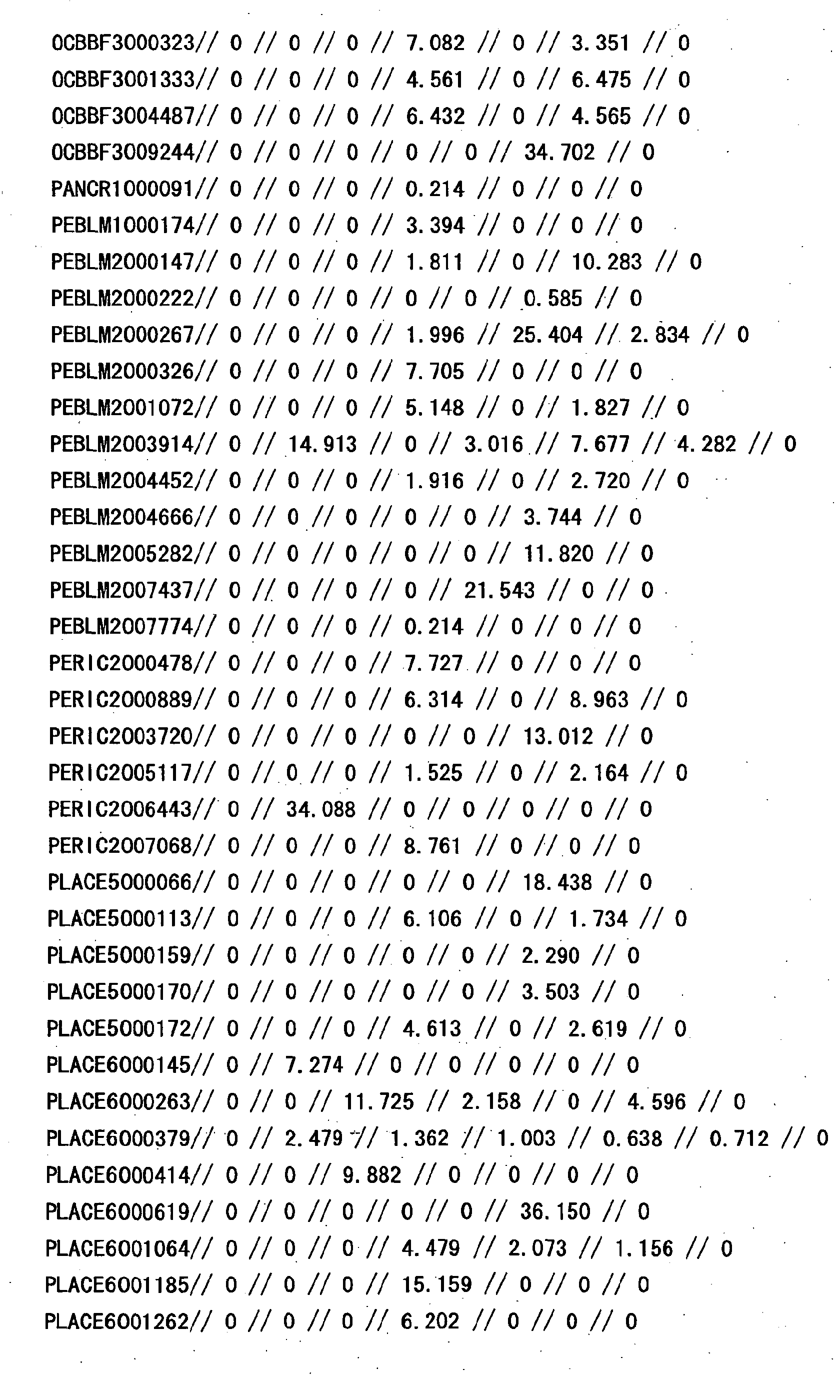

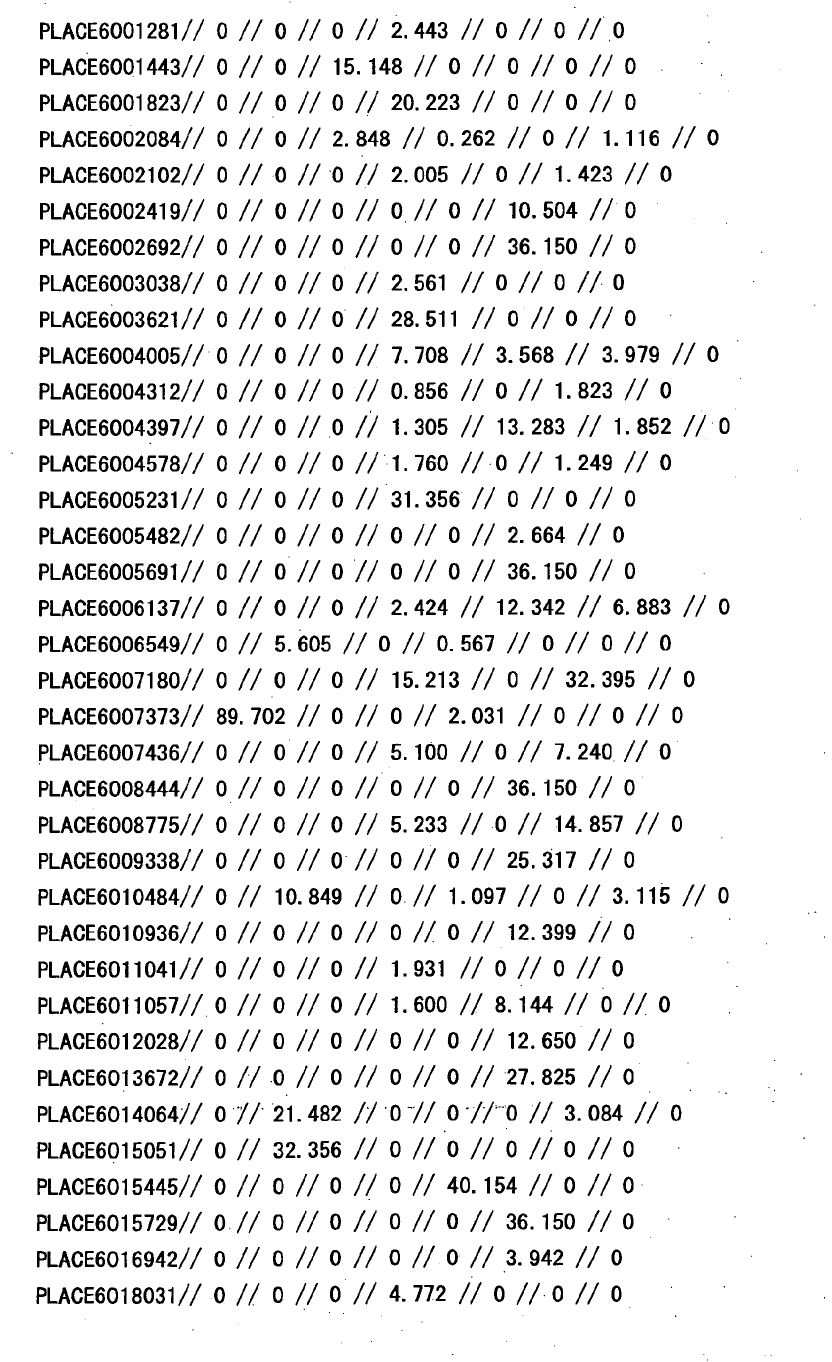

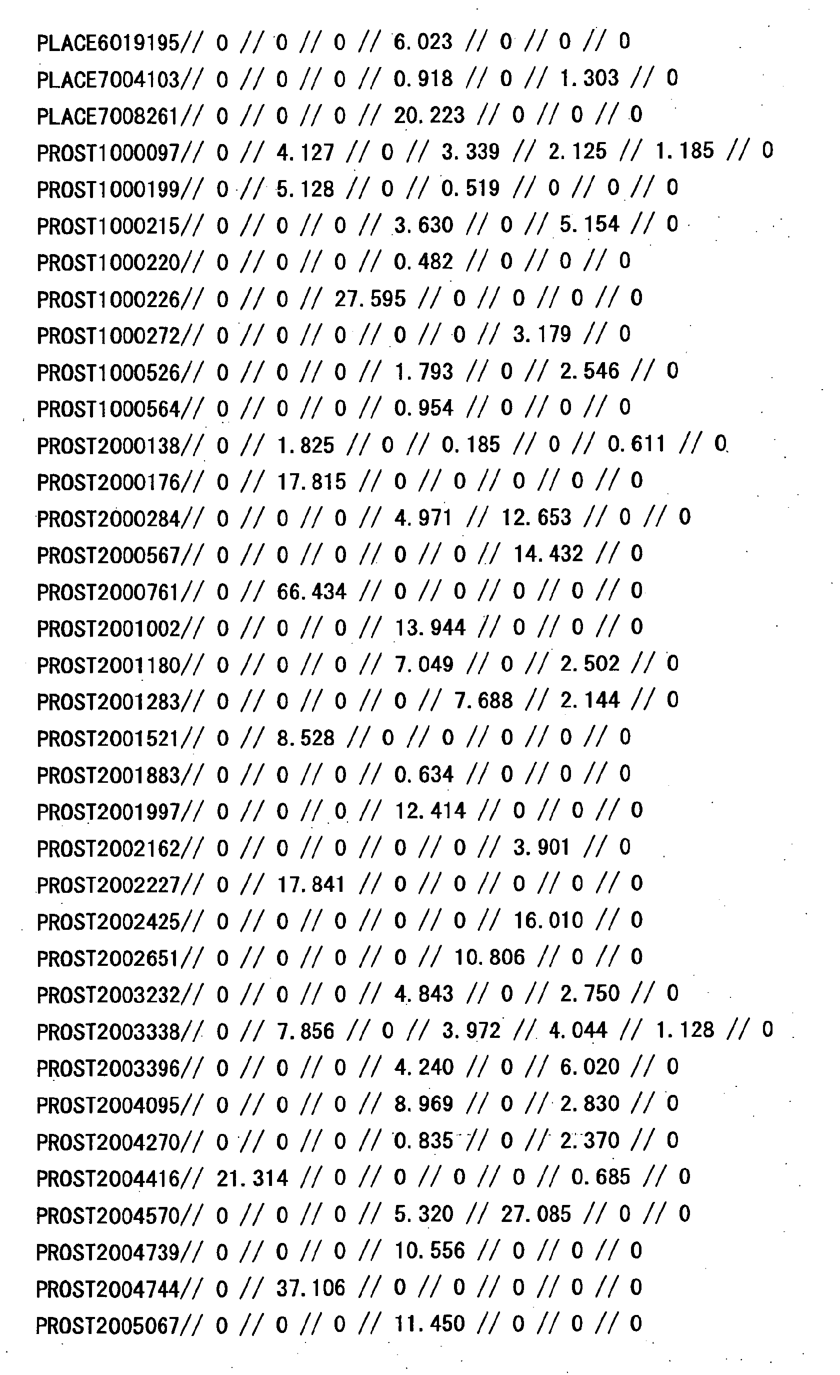

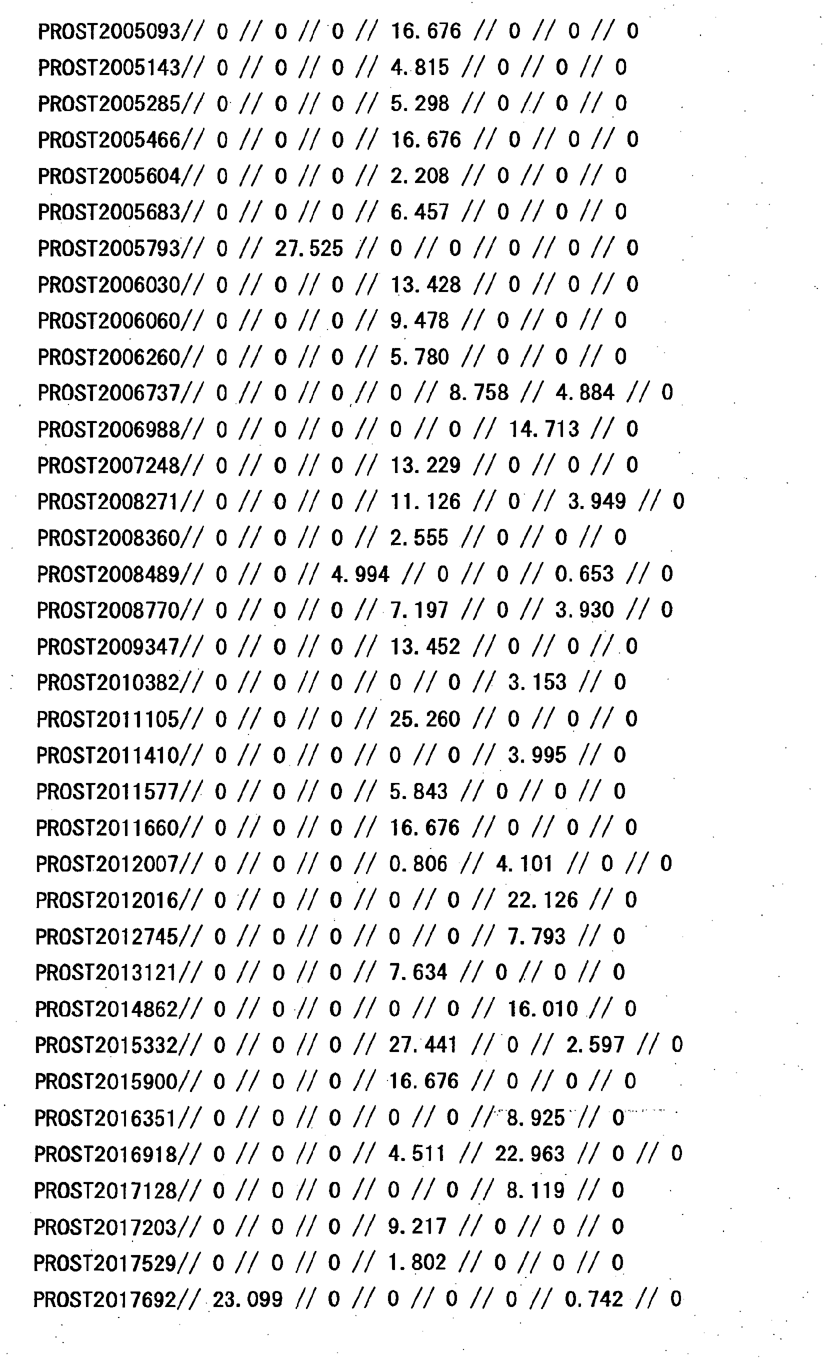

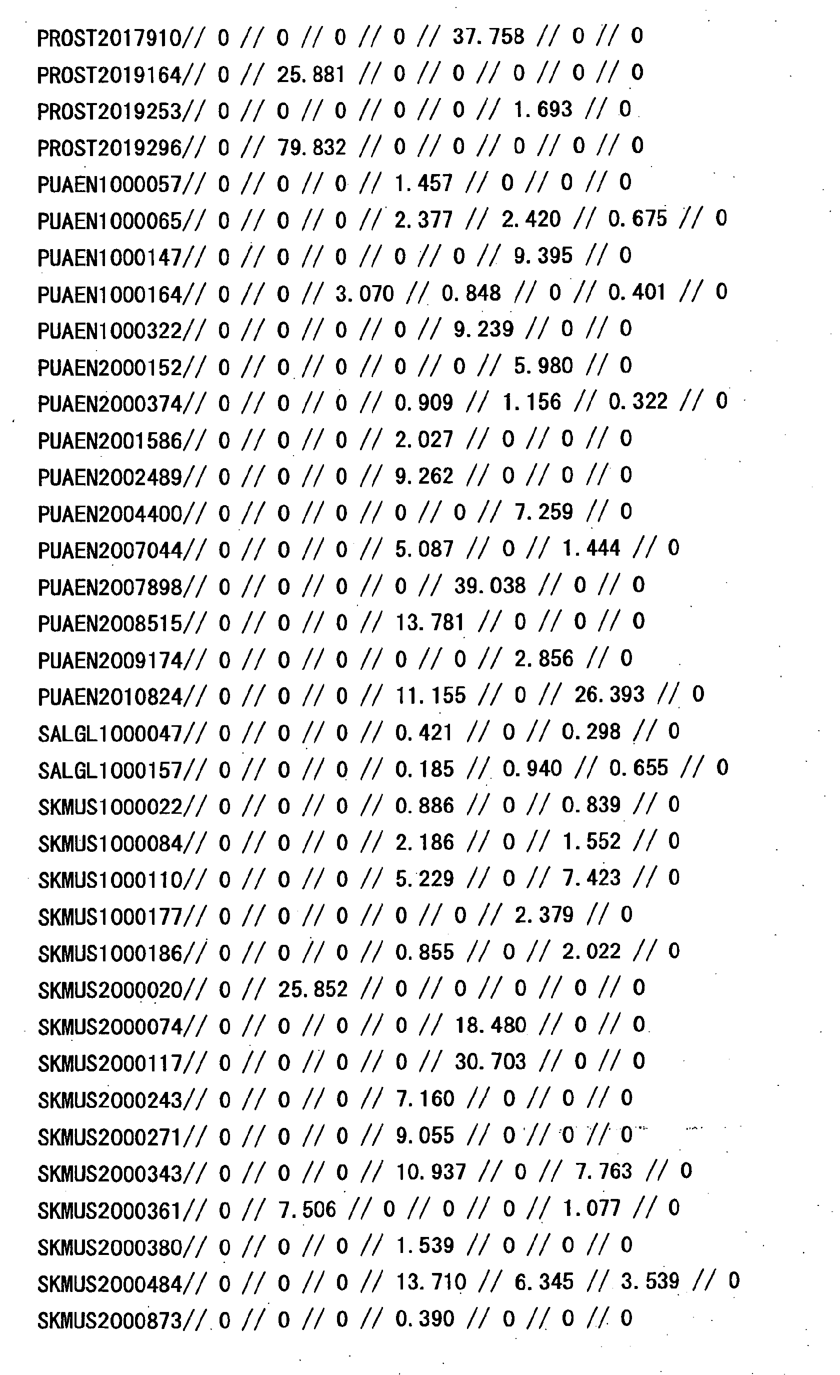

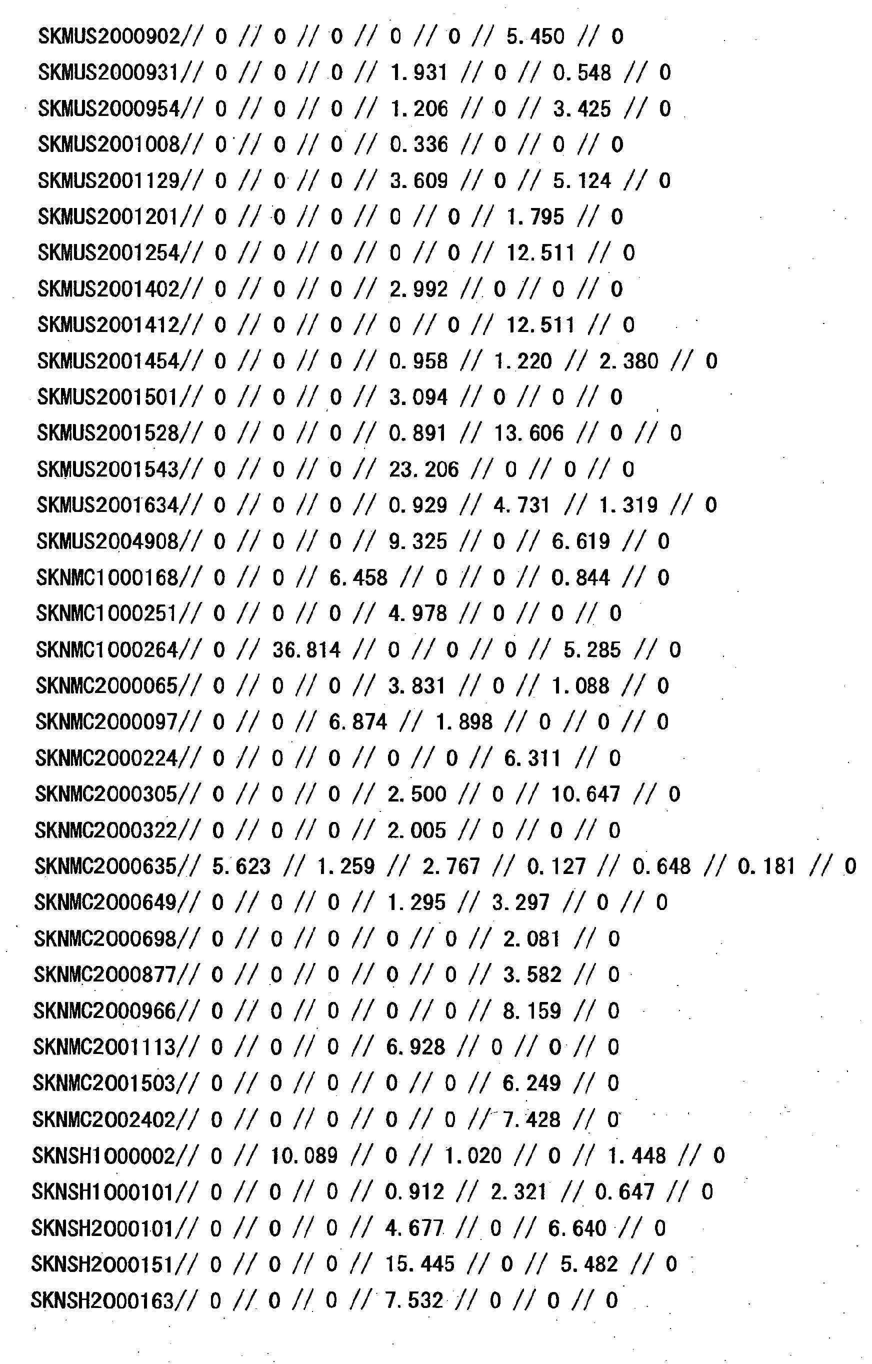

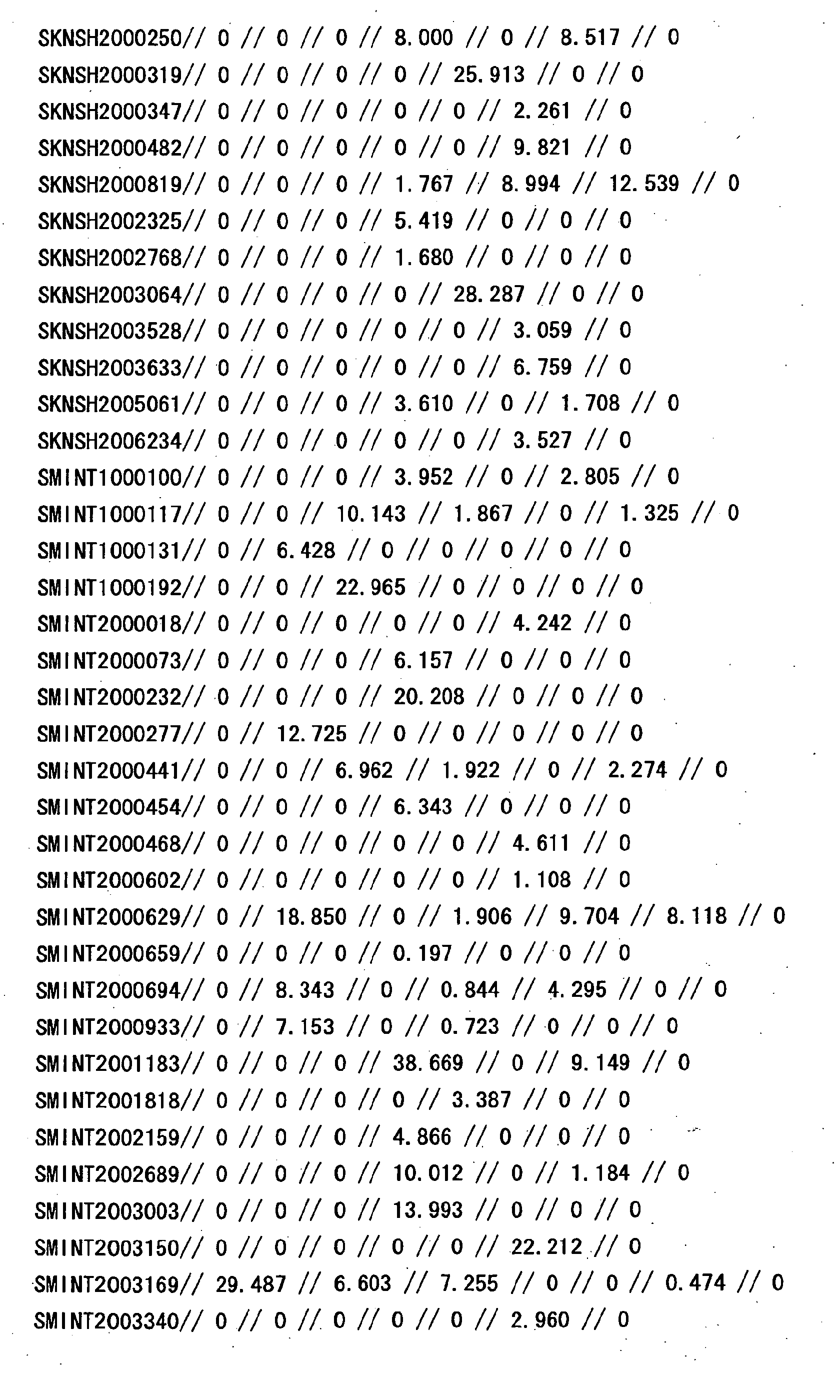

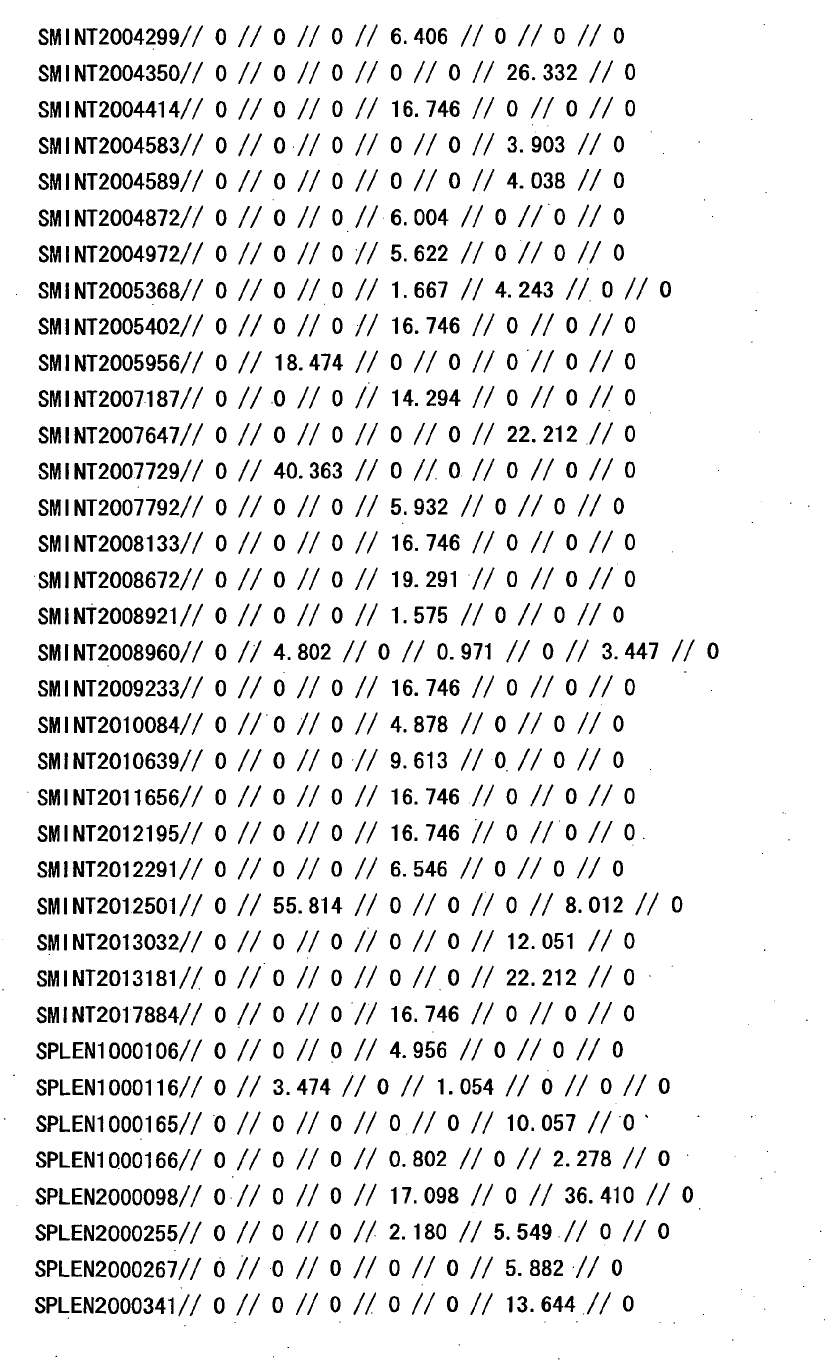

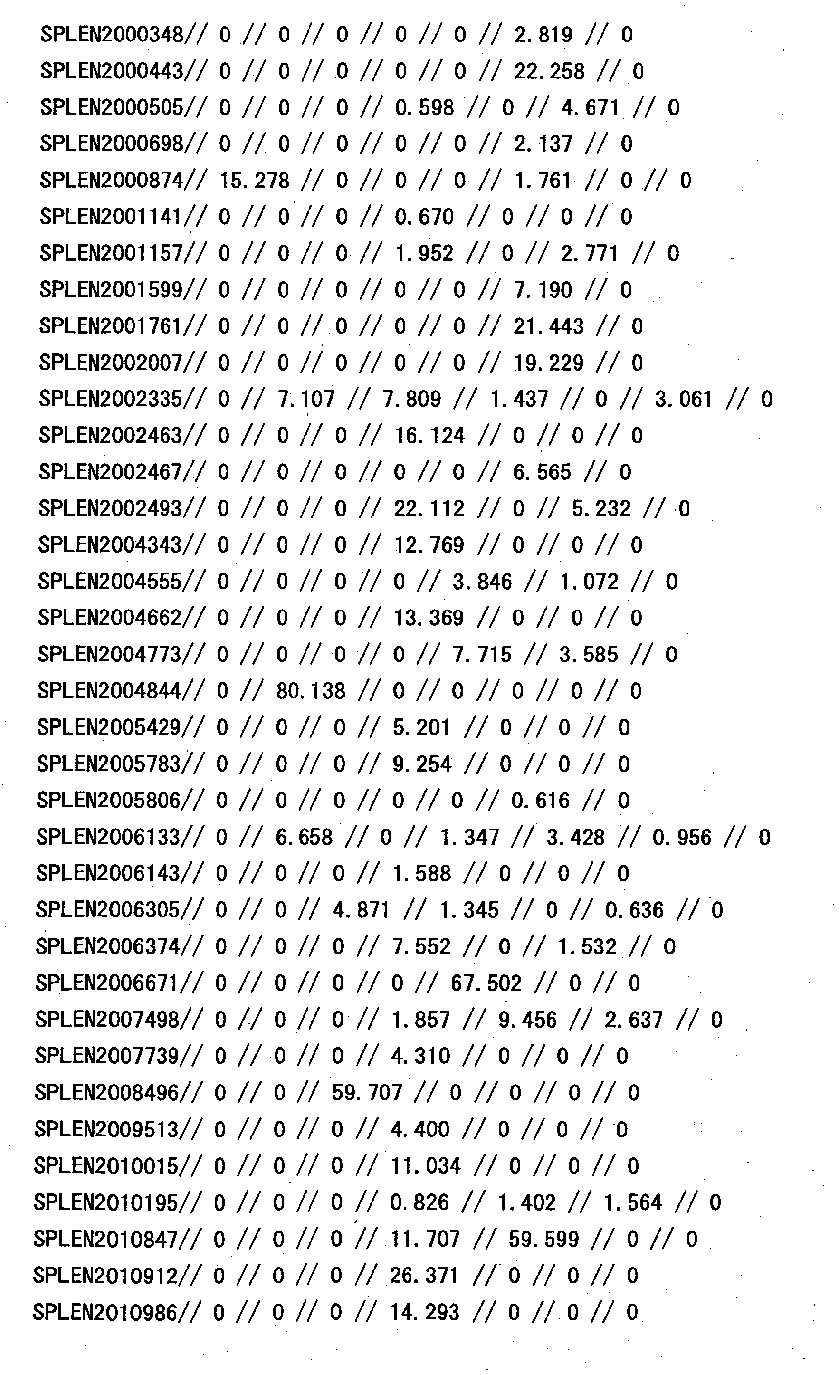

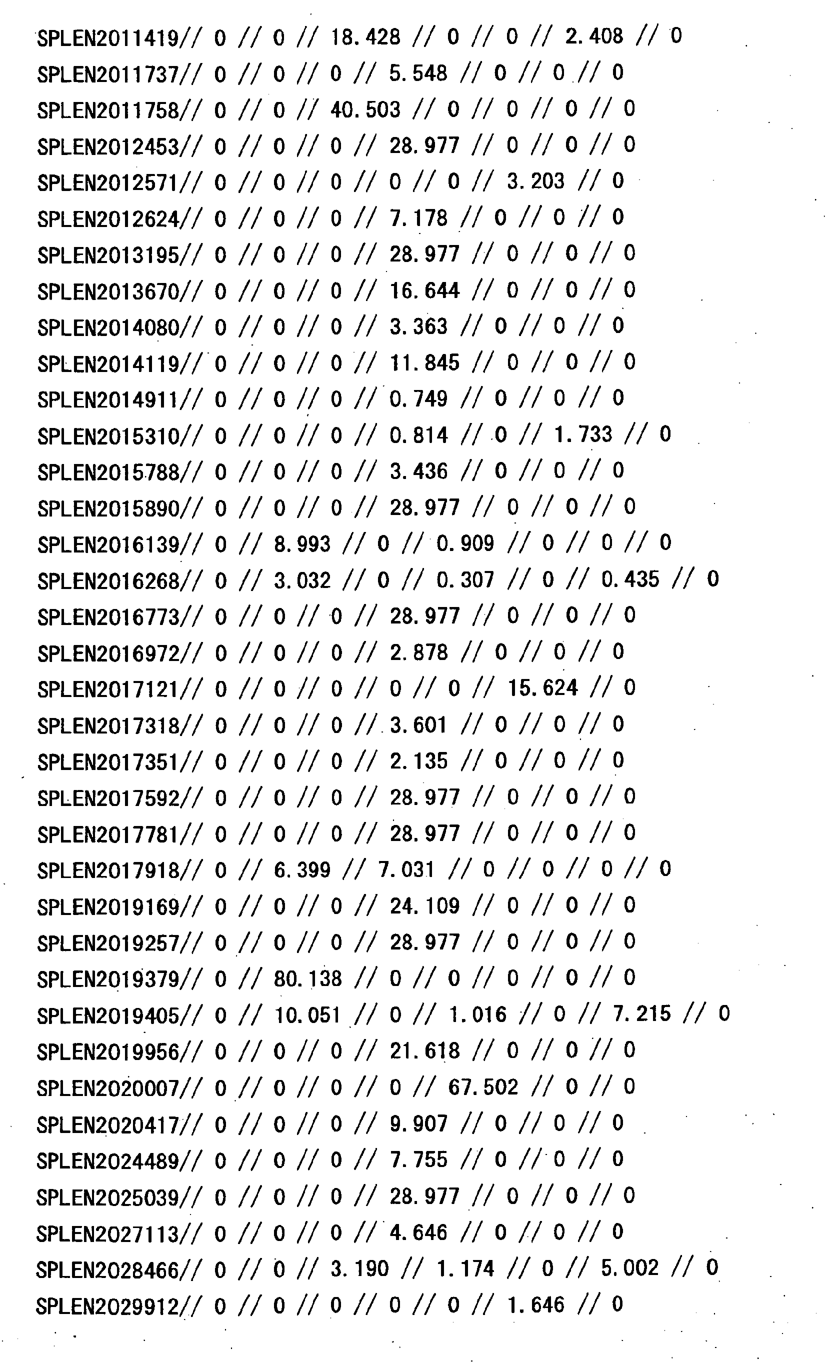

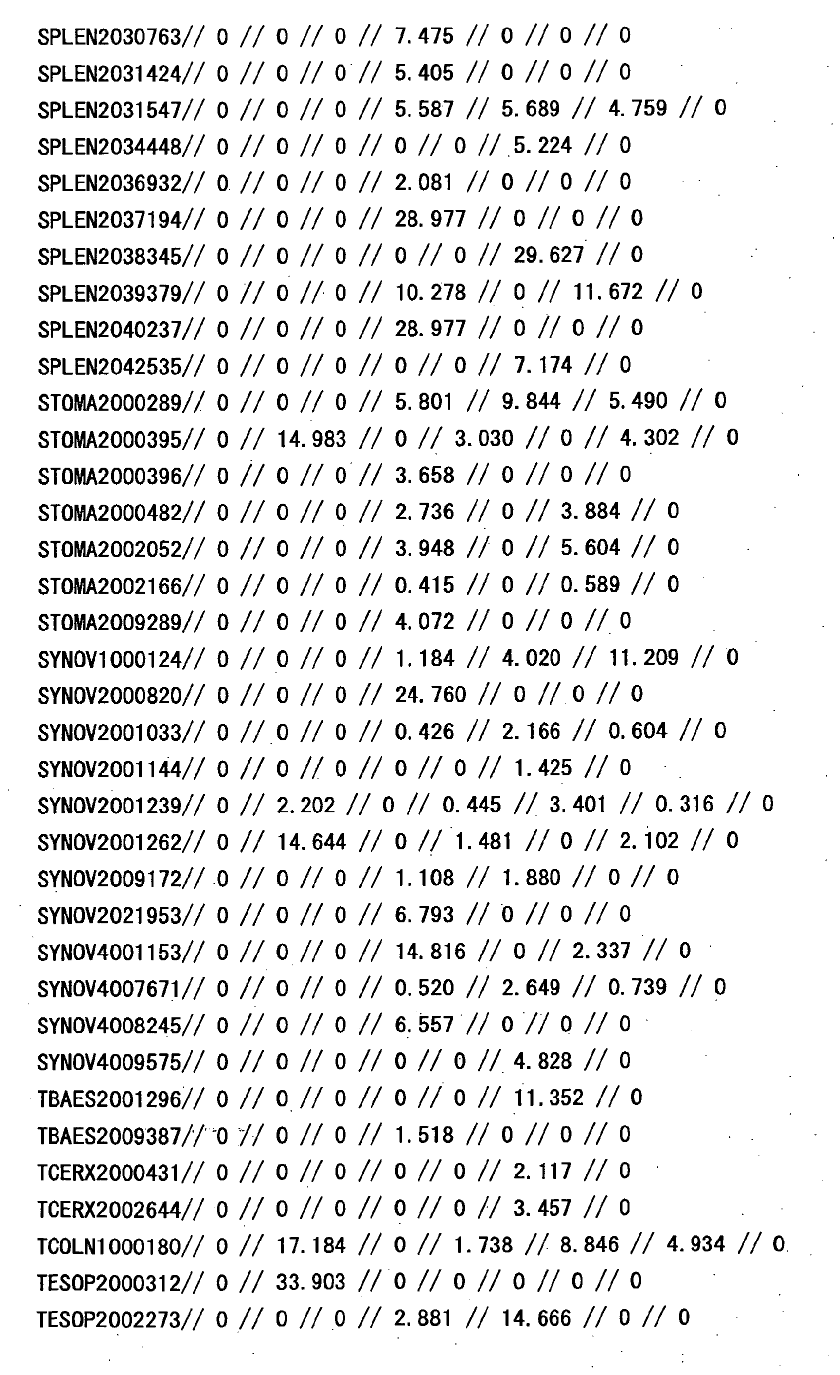

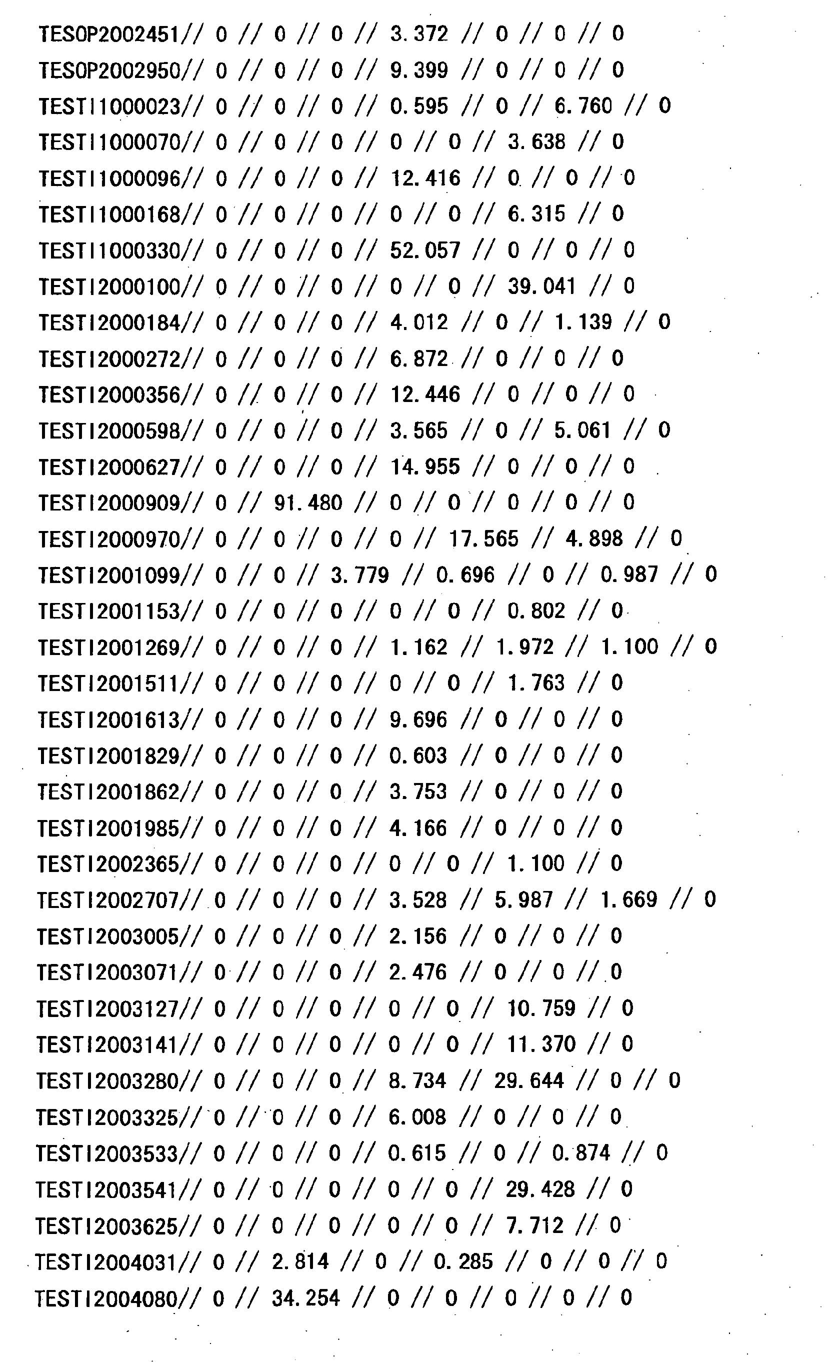

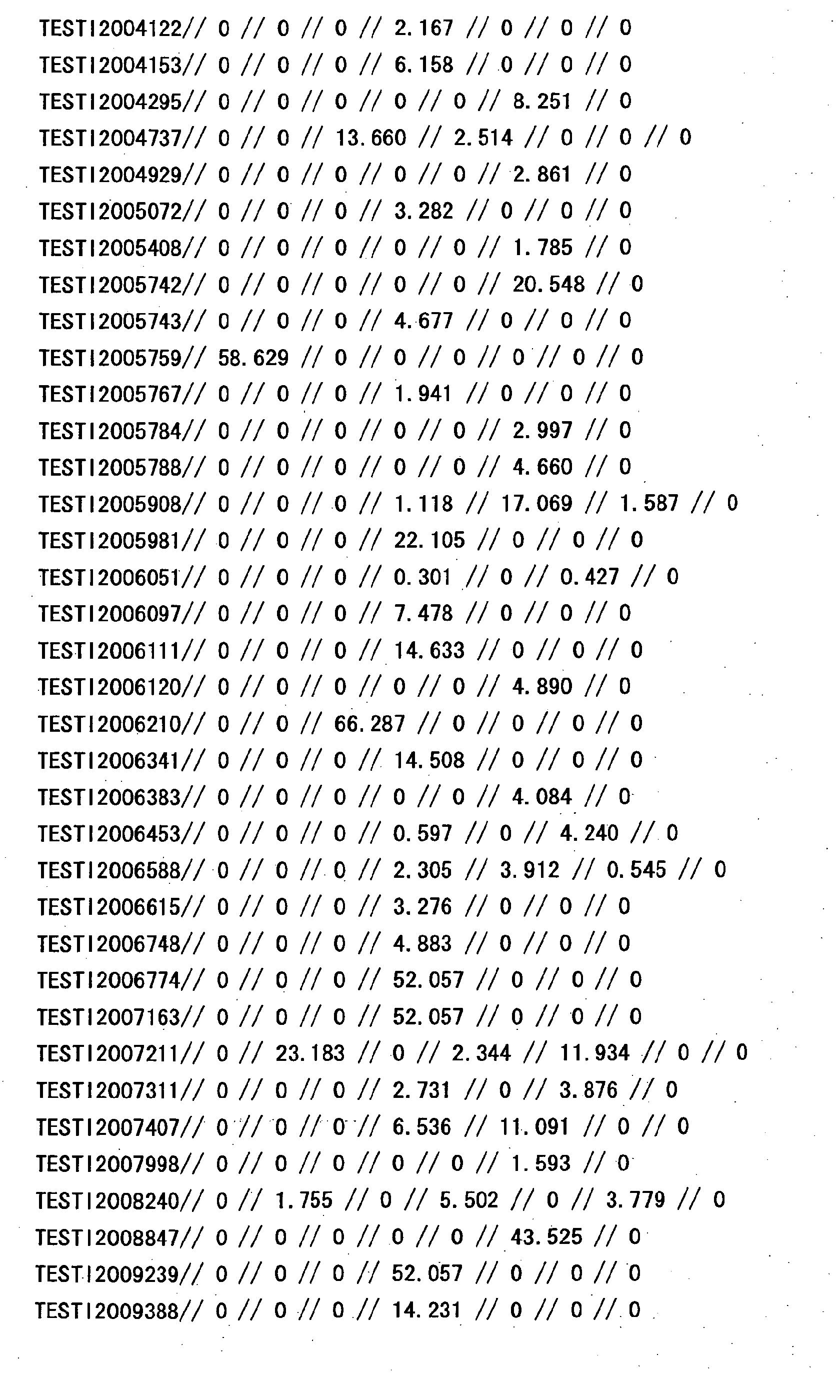

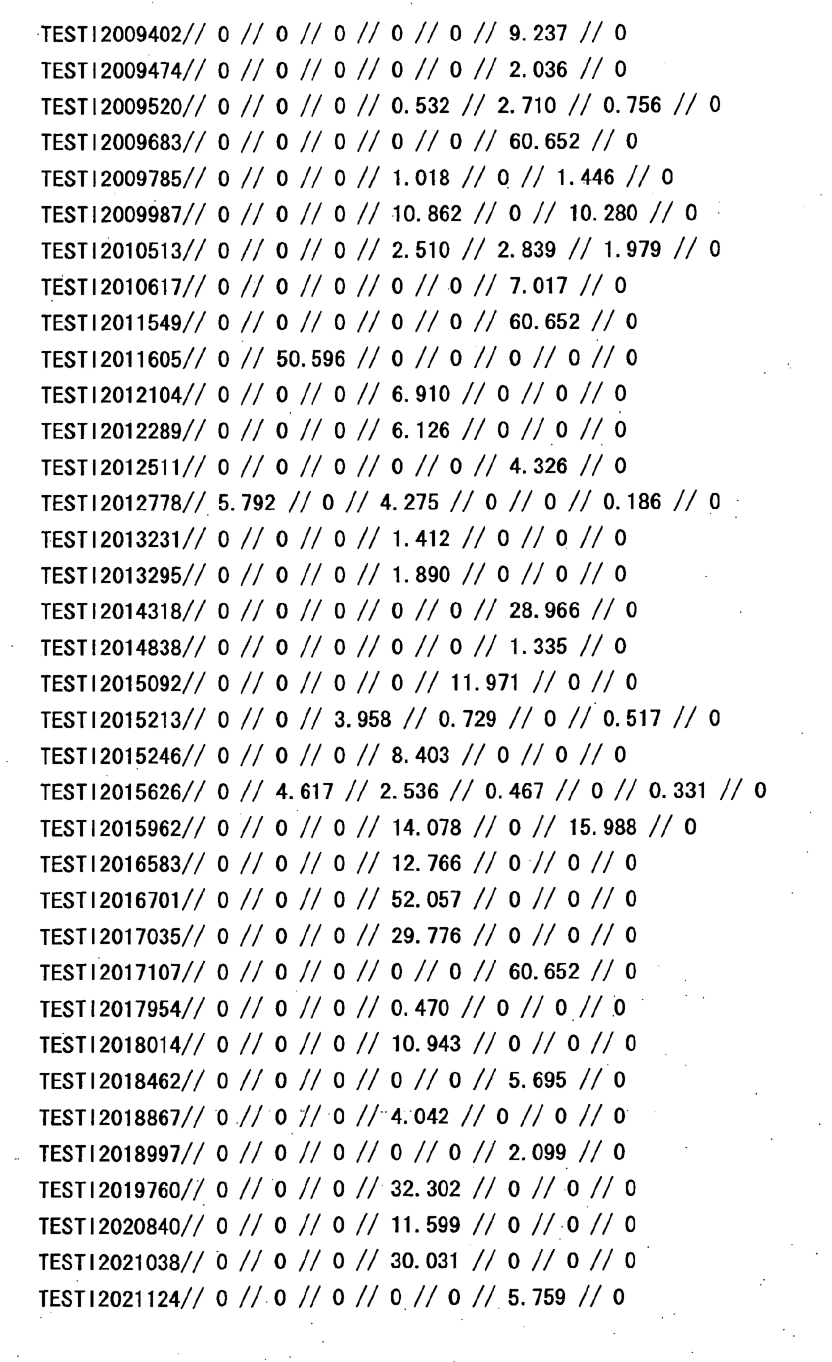

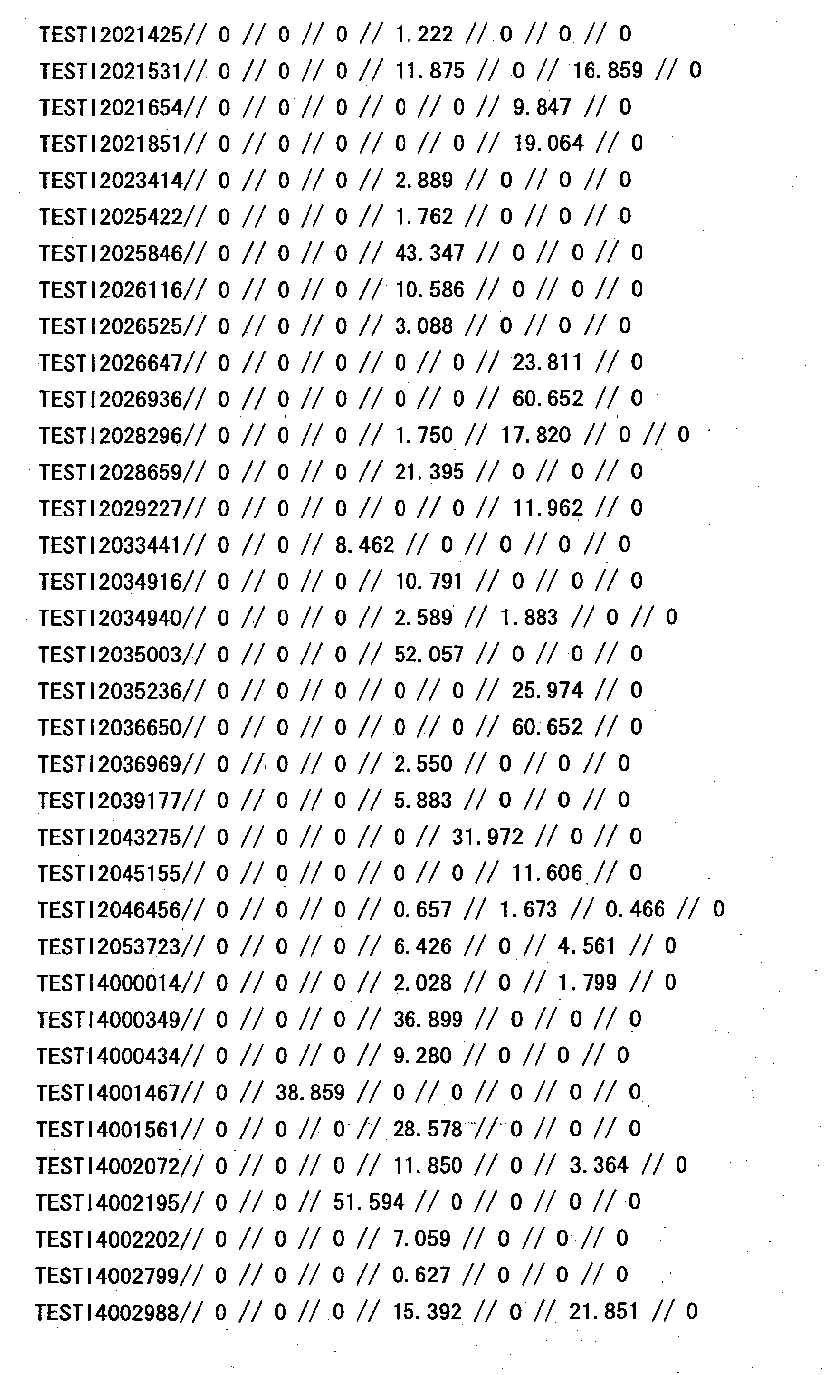

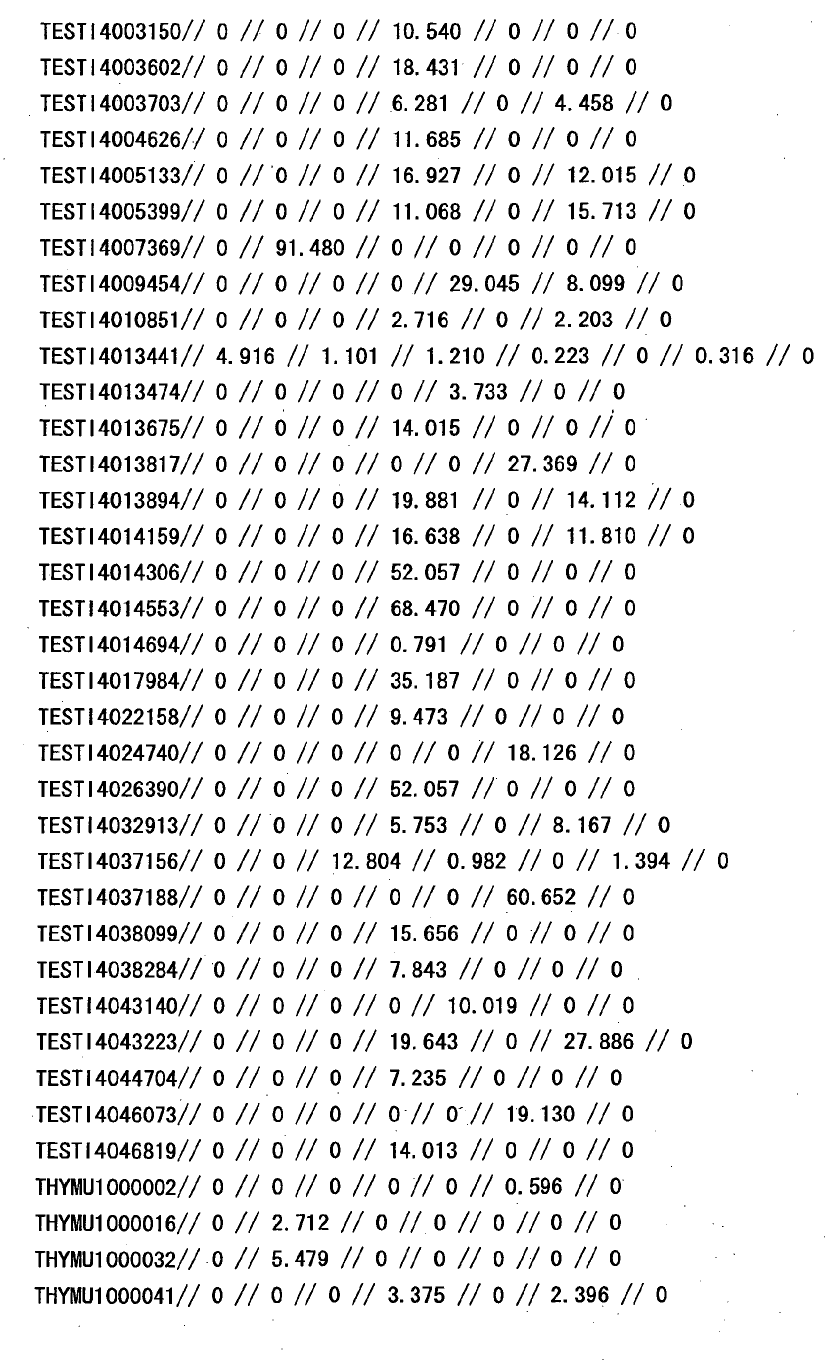

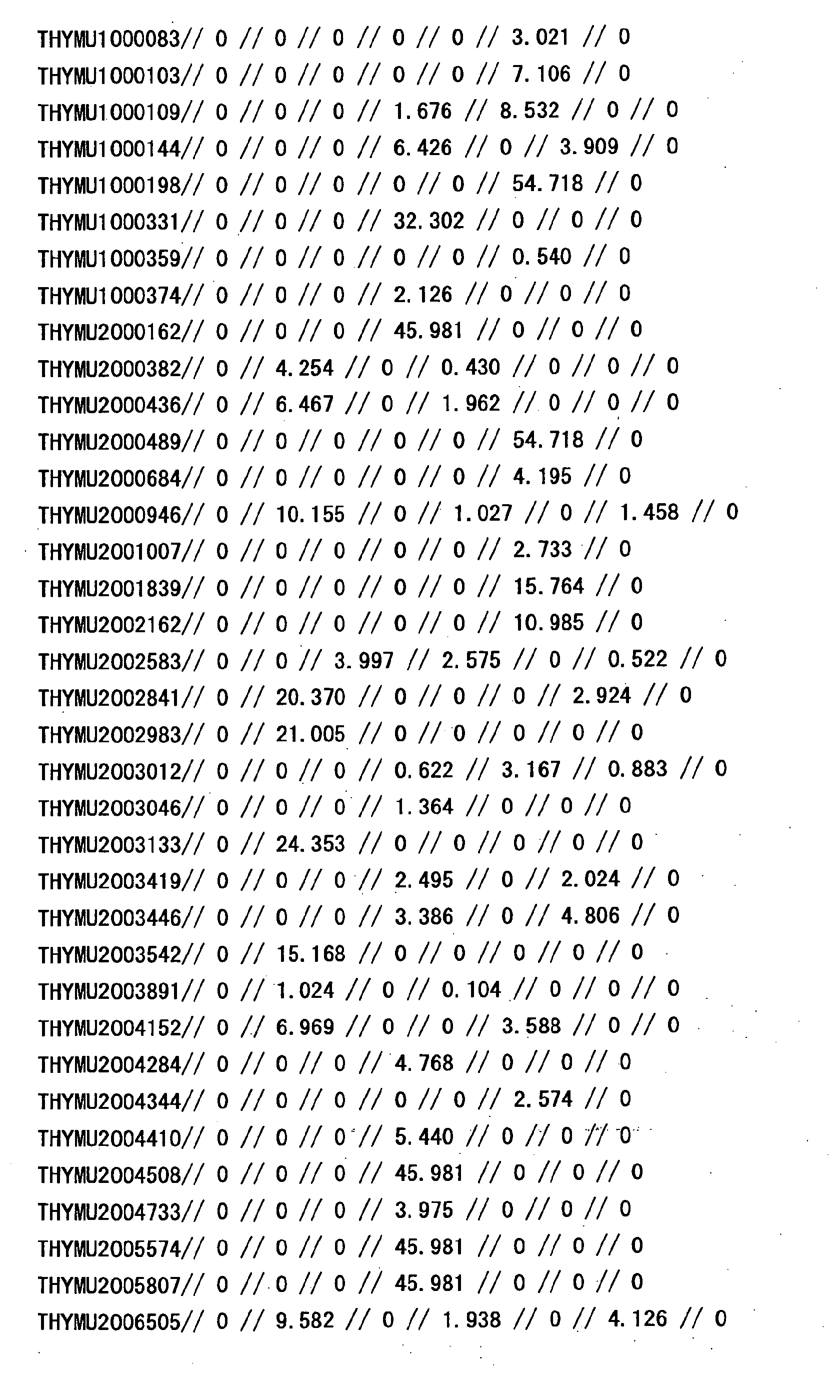

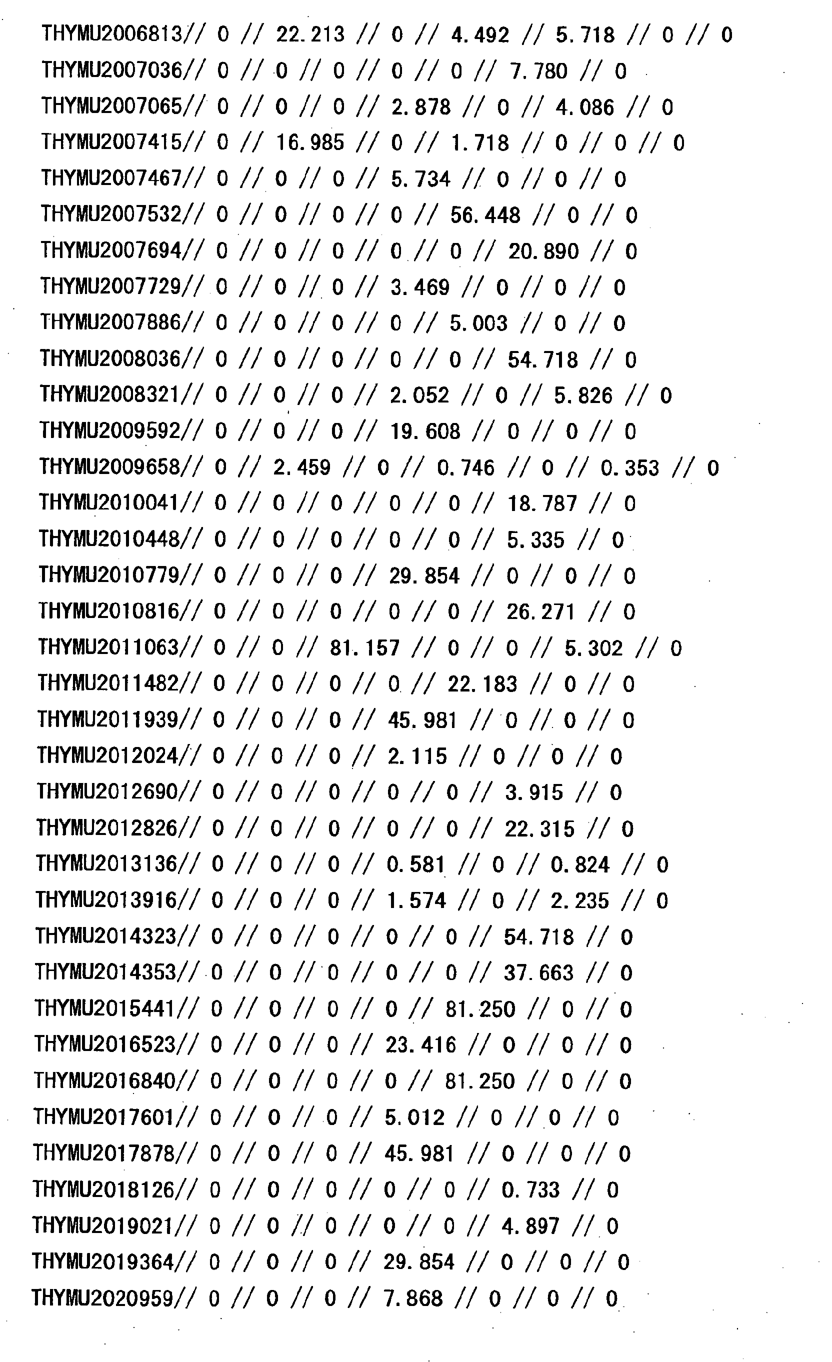

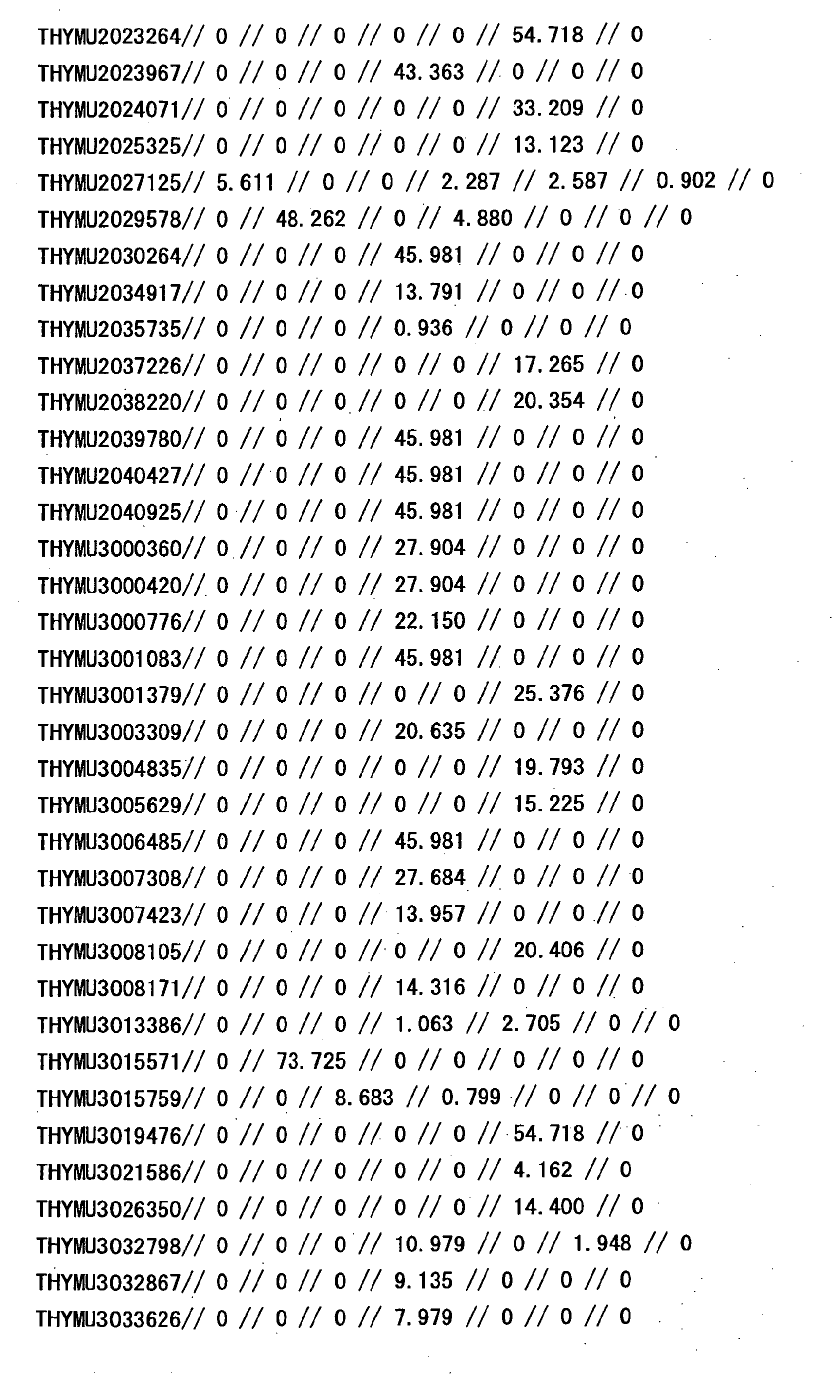

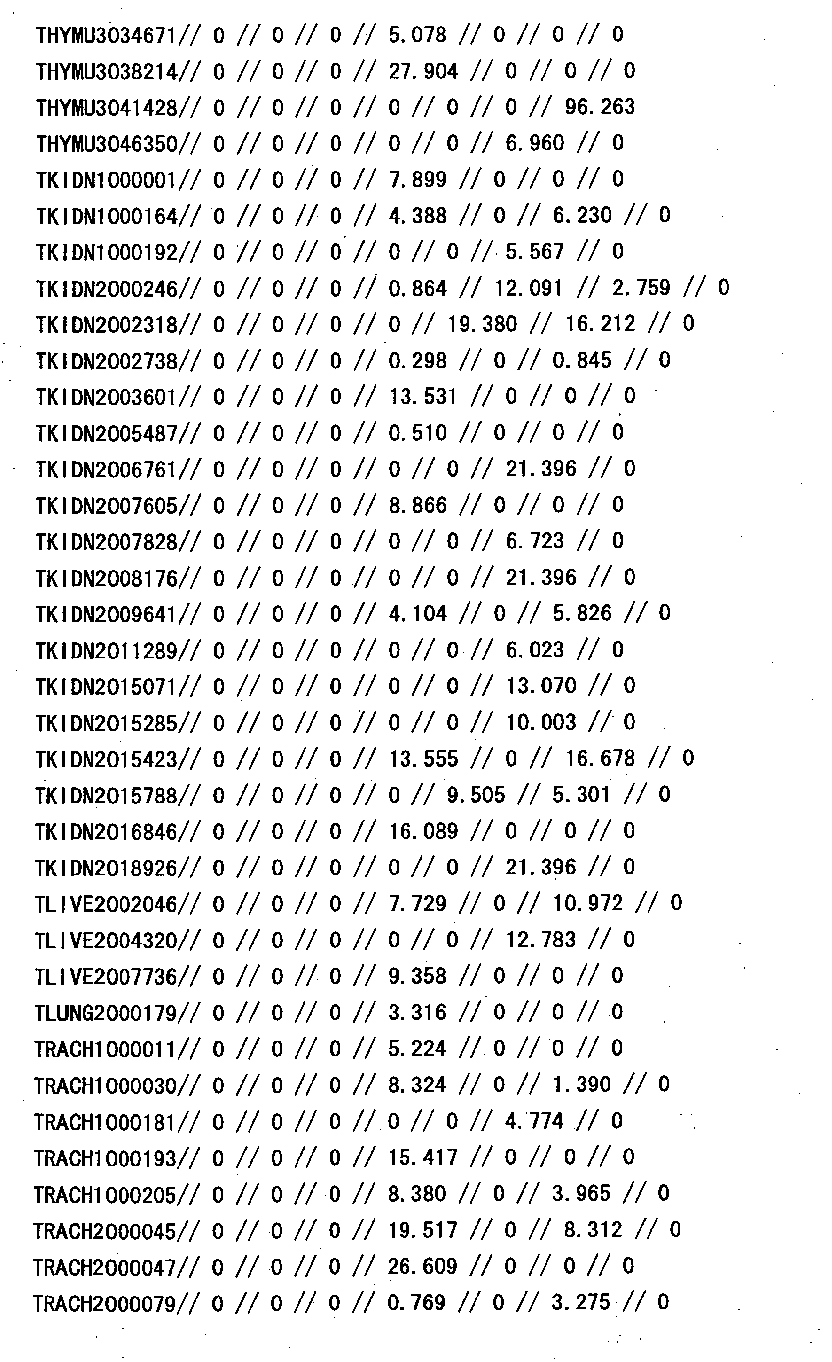

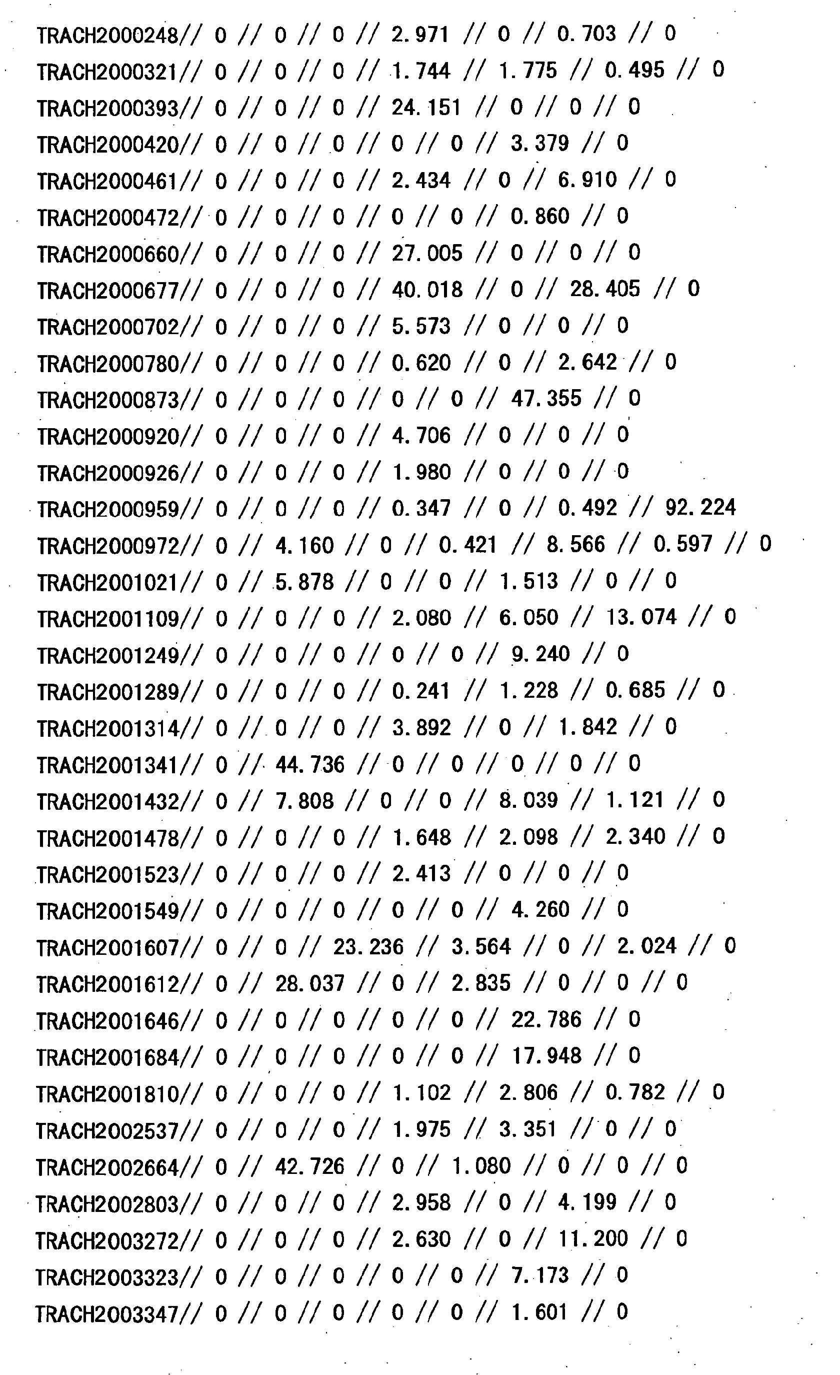

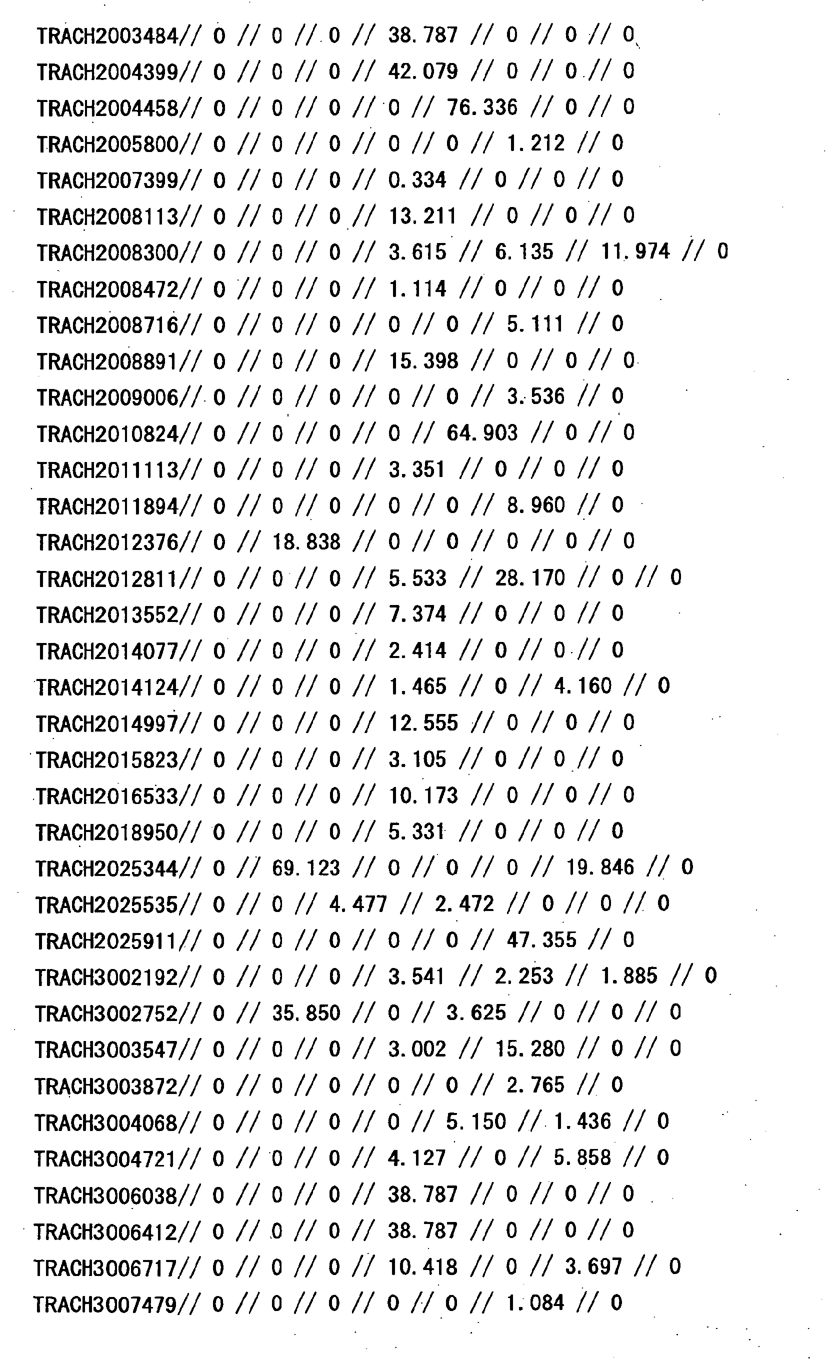

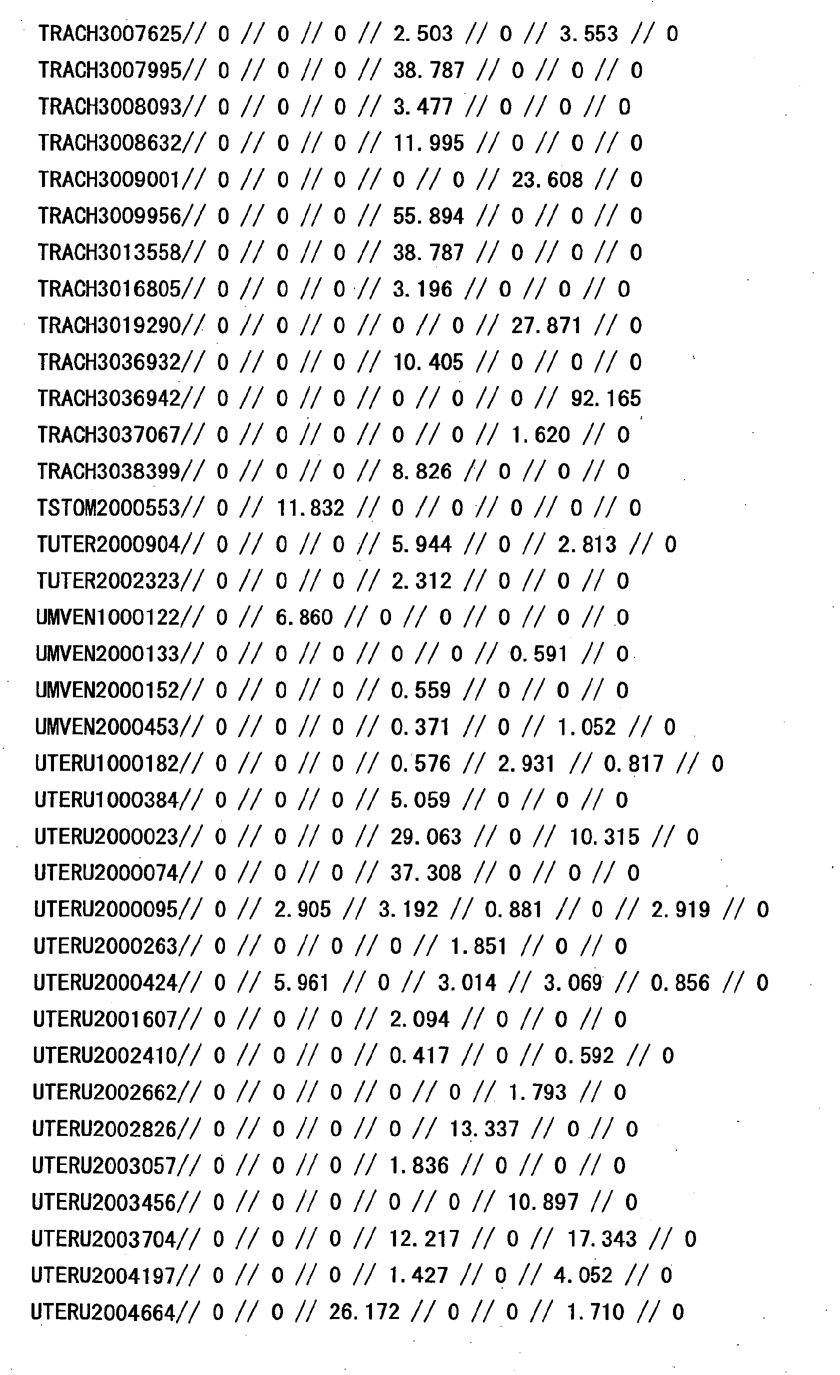

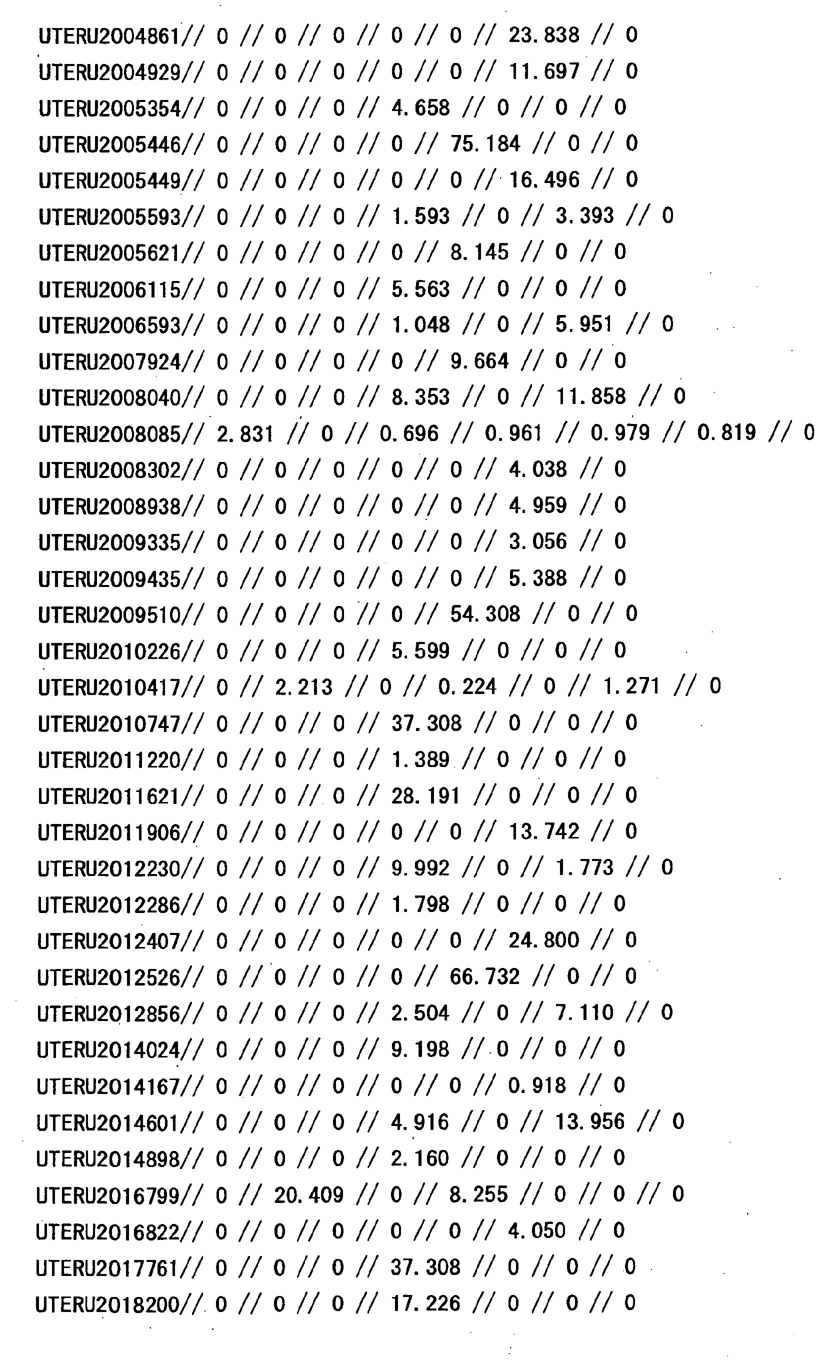

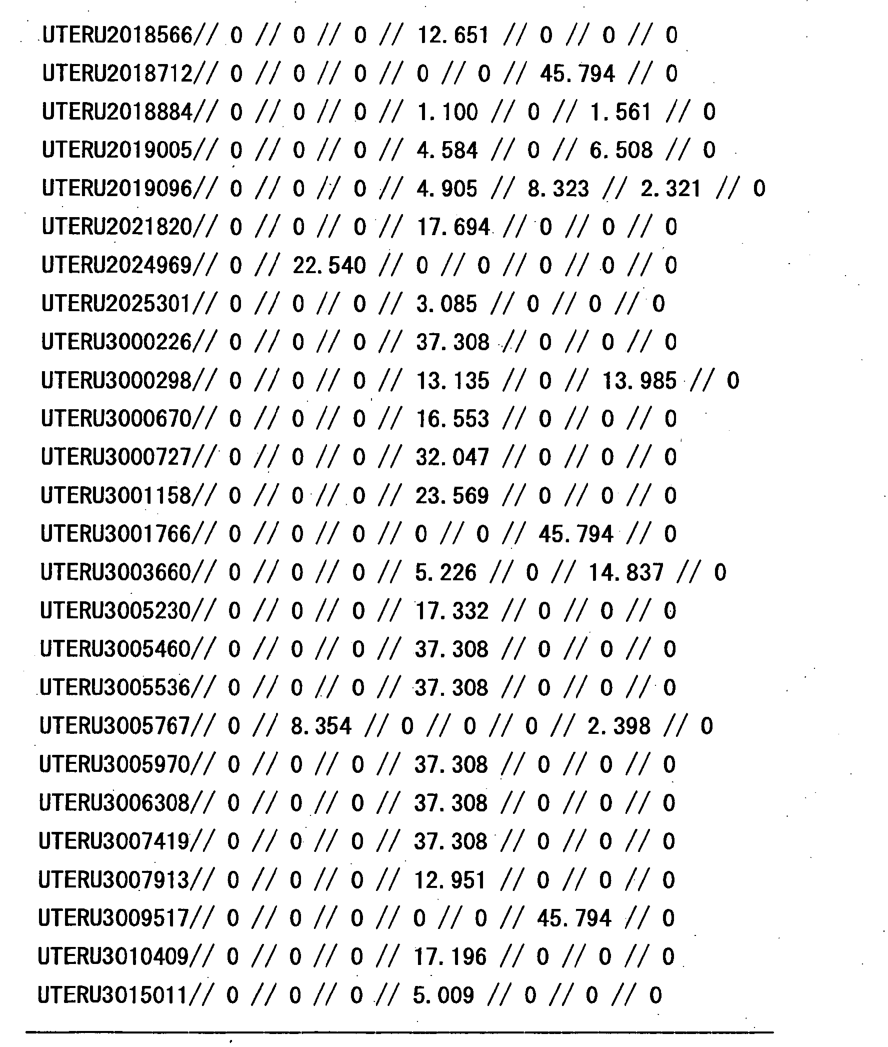

Genes whose expression levels were negligibly low in each of the above-described libraries are shown with their clone names below.
3NB691000018, 3NB691000113, 3NB691000129, 3NB691000191, 3NB692000012, 3NB692000029, 3NB692000102, 3NB692000154, 3NB692000276, 3NB692000305, 3NB692000429, 3NB692000484, 3NB692000529, 3NB692000973, 3NB692001002, 3NB692001034, 3NB692001040, 3NB692001123, 3NB692001267, 3NB692001339, 3NB692001349, 3NB692001366, 3NB692001408, 3NB692001433, 3NB692001459, 3NB692001471, 3NB692001501, 3NB692001507, 3NB692001511, 3NB692001519, 3NB692001520, 3NB692001528, 3NB692001538, 3NB692001548, 3NB692001557, 3NB692001637, 3NB692001861, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000038, ADRGL1000067, ADRGL1000111, ADRGL1000144, ADRGL1000147, ADRGL1000160, ADRGL1000165, ADRGL1000182, ADRGL2000056, ADRGL2000064, ADRGL2000085, ADRGL2000117, ADRGL2000142, ADRGL2000172, ADRGL2000248, ADRGL2000261, ADRGL2000323, ADRGL2000353, ADRGL2000384, ADRGL2000428, ADRGL2000636, ADRGL2000968, ADRGL2001119, ADRGL2001229, ADRGL2001287, ADRGL2001301, ADRGL2001354, ADRGL2001554, ADRGL2001651, ADRGL2001756, ADRGL2001836, ADRGL2001854, ADRGL2002029, ADRGL2002260, ADRGL2002392, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007357, ADRGL2007636, ADRGL2007651, ADRGL2007810, ADRGL2007877, ADRGL2008023, ADRGL2008331, ADRGL2008337, ADRGL2008535, ADRGL2009324, ADRGL2009489, ADRGL2009533, ADRGL2009691, ADRGL2009755, ADRGL2010315, ADRGL2010594, ADRGL2010860, ADRGL2010974, ADRGL2011190, ADRGL2011330, ADRGL2012179, AHMSC1000138, ASTRO1000009, ASTRO1000018, ASTRO1000029, ASTRO1000096, ASTRO1000165, ASTRO2000011, ASTRO2000095, ASTRO2000191, ASTRO2000278, ASTRO2000372, ASTRO2000403, ASTRO2000417, ASTRO2000482, ASTRO2000533, ASTRO2000653, ASTRO2000662, ASTRO2000725, ASTRO2000801, ASTRO2000914, ASTRO2001001, ASTRO2001008, ASTRO2001076, ASTRO2001088, ASTRO2001122, ASTRO2001227, ASTRO2001249, ASTRO2001480, ASTRO2002035, ASTRO2002064, ASTRO2002202, ASTRO2002571, ASTRO2002632, ASTRO2002659, ASTRO2002720, ASTRO2002743, ASTRO2002842, ASTRO2003574, ASTRO2003581, ASTRO2003632, ASTRO2003740, ASTRO2003925, ASTRO2004051, ASTRO2004217, ASTRO2004297, ASTRO2004584, ASTRO2004628, ASTRO2004751, ASTRO2004877, ASTRO2005008, ASTRO2005081, ASTRO2005096, ASTRO2005242, ASTRO2005273, ASTRO2005343, ASTRO2005360, ASTRO2005413, ASTRO2005557, ASTRO2005575, ASTRO2005593, ASTRO2005687, ASTRO2005726, ASTRO2005863, ASTRO2005896, ASTRO2005902, ASTRO2006356, ASTRO2006732, ASTRO2006747, ASTRO2006920, ASTRO2006952, ASTRO2007221, ASTRO2007377, ASTRO2007515, ASTRO2007578, ASTRO2007643, ASTRO2007666, ASTRO2007956, ASTRO2007962, ASTRO2008040, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014923, ASTRO2014961, ASTRO2015162, ASTRO2015185, ASTRO2015529, ASTRO2015987, ASTRO2016044, ASTRO2016114, ASTRO2016148, ASTRO2016681, ASTRO2016847, ASTRO2016892, ASTRO2017348, ASTRO2017627, ASTRO2018373, ASTRO2019039, ASTRO3000154, ASTRO3000172, ASTRO3000177, ASTRO3000301, ASTRO3000474, ASTRO3000482, BEAST2000454, BRAWH1000002, BRAWH1000007, BRAWH1000116, BRAWH1000130, BRAWH1000157, BRAWH1000164, BRAWH1000168, BRAWH1000180, BRAWH1000369, BRAWH2000034, BRAWH2000048, BRAWH2000071, BRAWH2000093, BRAWH2000109, BRAWH2000142, BRAWH2000169, BRAWH2000218, BRAWH2000232, BRAWH2000248, BRAWH2000256, BRAWH2000323, BRAWH2000370, BRAWH2000409, BRAWH2000471, BRAWH2000476, BRAWH2000488, BRAWH2000503, BRAWH2000554, BRAWH2000588, BRAWH2000643, BRAWH2000651, BRAWH2000682, BRAWH2000752, BRAWH2000859, BRAWH2000866, BRAWH2000892, BRAWH2000901, BRAWH2000926, BRAWH2000944, BRAWH2000984, BRAWH2001052, BRAWH2001103, BRAWH2001129, BRAWH2001137, BRAWH2001159, BRAWH2001203, BRAWH2001239, BRAWH2001255, BRAWH2001355, BRAWH2001378, BRAWH2001406, BRAWH2001412, BRAWH2001424, BRAWH2001436, BRAWH2001438, BRAWH2001484, BRAWH2001492, BRAWH2001503, BRAWH2001519, BRAWH2001530, BRAWH2001589, BRAWH2001666, BRAWH2001679, BRAWH2001686, BRAWH2001701, BRAWH2001842, BRAWH2001862, BRAWH2001873, BRAWH2001940, BRAWH2001973, BRAWH2002062, BRAWH2002125, BRAWH2002191, BRAWH2002333, BRAWH2002352, BRAWH2002383, BRAWH2002623, BRAWH2002630, BRAWH2002635, BRAWH2002662, BRAWH2002679, BRAWH2002725, BRAWH2002761, BRAWH2002861, BRAWH2002967, BRAWH2002976, BRAWH2003135, BRAWH2003240, BRAWH2003307, BRAWH2003366, BRAWH2003689, BRAWH2003693, BRAWH2003818, BRAWH2003832, BRAWH2003948, BRAWH2003964, BRAWH2004044, BRAWH2004068, BRAWH2004136, BRAWH2004217, BRAWH2004580, BRAWH2004731, BRAWH2004884, BRAWH2004923, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006083, BRAWH2006395, BRAWH2006405, BRAWH2006450, BRAWH2006526, BRAWH2006760, BRAWH2006960, BRAWH2007069, BRAWH2007308, BRAWH2007406, BRAWH2007550, BRAWH2007570, BRAWH2007658, BRAWH2007800, BRAWH2007808, BRAWH2007862, BRAWH2007890, BRAWH2008058, BRAWH2008128, BRAWH2008219, BRAWH2008292, BRAWH2008297, BRAWH2008366, BRAWH2008380, BRAWH2008540, BRAWH2008577, BRAWH2008790, BRAWH2009112, BRAWH2009227, BRAWH2009261, BRAWH2009263, BRAWH2009298, BRAWH2009304, BRAWH2009360, BRAWH2009393, BRAWH2009485, BRAWH2009490, BRAWH2009558, BRAWH2009590, BRAWH2009626, BRAWH2009678, BRAWH2009907, BRAWH2010000, BRAWH2010069, BRAWH2010084, BRAWH2010318, BRAWH2010536, BRAWH2010584, BRAWH2010618, BRAWH2010619, BRAWH2010980, BRAWH2011066, BRAWH2011079, BRAWH2011096, BRAWH2011155, BRAWH2011166, BRAWH2011286, BRAWH2011294, BRAWH2011400, BRAWH2011762, BRAWH2011795, BRAWH2011796, BRAWH2011823, BRAWH2011860, BRAWH2011958, BRAWH2012054, BRAWH2012077, BRAWH2012161, BRAWH2012162, BRAWH2012258, BRAWH2012277, BRAWH2012326, BRAWH2012540, BRAWH2012619, BRAWH2012749, BRAWH2012963, BRAWH2013866, BRAWH2013871, BRAWH2013914, BRAWH2013941, BRAWH2013955, BRAWH2014234, BRAWH2014379, BRAWH2014473, BRAWH2014717, BRAWH2014729, BRAWH2014934, BRAWH2015349, BRAWH2015375, BRAWH2015595, BRAWH2015853, BRAWH2015866, BRAWH2016106, BRAWH2016160, BRAWH2016221, BRAWH2016223, BRAWH2016305, BRAWH2016439, BRAWH2016446, BRAWH2016514, BRAWH2016562, BRAWH2016655, BRAWH2016724, BRAWH2016785, BRAWH2016792, BRAWH2016800, BRAWH2017103, BRAWH2017250, BRAWH2017294, BRAWH2017305, BRAWH2017407, BRAWH2017433, BRAWH2017523, BRAWH2017534, BRAWH2017542, BRAWH2017635, BRAWH2017913, BRAWH2018206, BRAWH2018282, BRAWH2018317, BRAWH2018506, BRAWH2018514, BRAWH2018527, BRAWH2018601, BRAWH2018729, BRAWH2018745, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001326, BRAWH3001475, BRAWH3001636, BRAWH3001638, BRAWH3001712, BRAWH3001760, BRAWH3001783, BRAWH3001833, BRAWH3001879, BRAWH3002433, BRAWH3002467, BRAWH3002513, BRAWH3002574, BRAWH3002819, BRAWH3003019, BRAWH3003136, BRAWH3003244, BRAWH3003343, BRAWH3003411, BRAWH3003522, BRAWH3003598, BRAWH3003801, BRAWH3003975, BRAWH3004222, BRAWH3004335, BRAWH3004350, BRAWH3004453, BRAWH3004666, BRAWH3005037, BRAWH3005047, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006792, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3009017, BRAWH3009546, BRAWH3009961, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017260, BRAWH3017477, BRAWH3017537, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3030613, BRAWH3030772, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034775, BRAWH3034890, BRAWH3035154, BRAWH3035904, BRAWH3035914, BRAWH3035936, BRAWH3036077, BRAWH3036247, BRAWH3036270, BRAWH3036561, BRAWH3036738, BRAWH3036951, BRAWH30372-65, BRAWH3037394, BRAWH3037533, BRAWH3037979, BRAWH3038055, BRAWH3038230, BRAWH3038252, BRAWH3038324, BRCAN1000065, BRCAN1000105, BRCAN1000149, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001866, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006051, BRCAN2006063, BRCAN2006174, BRCAN2006290, BRCAN2006297, BRCAN2006323, BRCAN2006411, BRCAN2006450, BRCAN2006955, BRCAN2007119, BRCAN2007144, BRCAN2007409, BRCAN2007426, BRCAN2007525, BRCAN2008494, BRCAN2008528, BRCAN2008701, BRCAN2009156, BRCAN2009168, BRCAN2009203, BRCAN2010547, BRCAN2010581, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014952, BRCAN2015402, BRCAN2015464, BRCAN2015757, BRCAN2015886, BRCAN2016025, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019773, BRCAN2019907, BRCAN2020234, BRCAN2020331, BRCAN2020412, BRCAN2020467, BRCAN2020880, BRCAN2021024, BRCAN2021028, BRCAN2021325, BRCAN2021452, BRCAN2021669, BRCAN2021718, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025712, BRCAN2025982, BRCAN2026117, BRCAN2026711, BRCAN2027150, BRCAN2027310, BRCAN2027355, BRCAN2027513, BRCAN2027593, BRCAN2027970, BRCAN2028021, BRCAN2028040, BRCAN2028319, BRCAN2028338, BRCAN2028378, BRCAN2028460, BRCAN2028545, BRCAN2028634, BRCAN2028702, BRCOC1000087, BRCOC2000152, BRCOC2000184, BRCOC2000360, BRCOC2000404, BRCOC2000487, BRCOC2000637, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003125, BRCOC2003513, BRCOC2003732, BRCOC2003740, BRCOC2004175, BRCOC2004354, BRCOC2004455, BRCOC2004950, BRCOC2005951, BRCOC2006164, BRCOC2006243, BRCOC2006639, BRCOC2006942, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2010115, BRCOC2010123, BRCOC2010510, BRCOC2010730, BRCOC2010980, BRCOC2010994, BRCOC2011010, BRCOC2011398, BRCOC2011418, BRCOC2011769, BRCOC2011996, BRCOC2012229, BRCOC2012386, BRCOC2012813, BRCOC2013448, BRCOC2013573, BRCOC2013675, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019903, BRCOC2019934, BRCOC2020122, BRCOC2020142, BRHIP1000058, BRHIP1000072, BRHIP1000258, BRHIP2000021, BRHIP2000079, BRHIP2000163, BRHIP2000239, BRHIP2000245, BRHIP2000359, BRHIP2000445, BRHIP2000506, BRHIP2000532, BRHIP2000621, BRHIP2000691, BRHIP2000819, BRHIP2000826, BRHIP2001074, BRHIP2001686, BRHIP2001782, BRHIP2001802, BRHIP2001805, BRHIP2001927, BRHIP2002122, BRHIP2002172, BRHIP2002346, BRHIP2002510, BRHIP2002664, BRHIP2002722, BRHIP2002929, BRHIP2003048, BRHIP2003242, BRHIP2003272, BRHIP2003748, BRHIP2003786, BRHIP2003828, BRHIP2003917, BRHIP2004312, BRHIP2004355, BRHIP2004814, BRHIP2004883, BRHIP2004902, BRHIP2005236, BRHIP2005354, BRHIP2005600, BRHIP2005719, BRHIP2005724, BRHIP2005932, BRHIP2006617, BRHIP2006782, BRHIP2006800, BRHIP2006819, BRHIP2006921, BRHIP2007307, BRHIP2007616, BRHIP2007690, BRHIP2007928, BRHIP2007969, BRHIP2008269, BRHIP2008389, BRHIP2008756, BRHIP2008909, BRHIP2009177, BRHIP2009340, BRHIP2009351, BRHIP2009414, BRHIP2009474, BRHIP2009617, BRHIP2009685, BRHIP2010309, BRHIP2010444, BRHIP2010593, BRHIP2010610, BRHIP2010744, BRHIP2011080, BRHIP2011199, BRHIP2011508, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013723, BRHIP2013958, BRHIP2013972, BRHIP2014063, BRHIP2014285, BRHIP2014291, BRHIP2014373, BRHIP2014386, BRHIP2014387, BRHIP2014954, BRHIP2015356, BRHIP2015360, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017714, BRHIP2017780, BRHIP2018014, BRHIP2018369, BRHIP2018612, BRHIP2018635, BRHIP2018650, BRHIP2018805, BRHIP2018998, BRHIP2019007, BRHIP2019047, BRHIP2019149, BRHIP2019177, BRHIP2019291, BRHIP2019589, BRHIP2019641, BRHIP2019884, BRHIP2019972, BRHIP2020182, BRHIP2020509, BRHIP2020695, BRHIP2020743, BRHIP2020842, BRHIP2020859, BRHIP2020930, BRHIP2021102, BRHIP2021289, BRHIP2021615, BRHIP2021744, BRHIP2021929, BRHIP2022006, BRHIP2022221, BRHIP2022228, BRHIP2022298, BRHIP2022467, BRHIP2022708, BRHIP2023229, BRHIP2023272, BRHIP2023292, BRHIP2023369, BRHIP2023438, BRHIP2023695, BRHIP2023735, BRHIP2023773, BRHIP2023860, BRHIP2023869, BRHIP2024046, BRHIP2024165, BRHIP2024201, BRHIP2024347, BRHIP2024549, BRHIP2024789, BRHIP2024911, BRHIP2024941, BRHIP2025245, BRHIP2025366, BRHIP2025739, BRHIP2025797, BRHIP2026021, BRHIP2026155, BRHIP2026214, BRHIP2026346, BRHIP2026557, BRHIP2026723, BRHIP2026877, BRHIP2027017, BRHIP2027054, BRHIP2027563, BRHIP2027762, BRHIP2028303, BRHIP2028330, BRHIP2028480, BRHIP2028593, BRHIP2029176, BRHIP2029283, BRHIP2029393, BRHIP2029529, BRHIP2029663, BRHIP3000017, BRHIP3000060, BRHIP3000070, BRHIP3000081, BRHIP3000475, BRHIP3000488, BRHIP3000526, BRHIP3000626, BRHIP3000859, BRHIP3000907, BRHIP3001076, BRHIP3001079, BRHIP3001141, BRHIP3001338, BRHIP3001481, BRHIP3001878, BRHIP3001964, BRHIP3002124, BRHIP3002141, BRHIP3002363, BRHIP3002920, BRHIP3002931, BRHIP3003306, BRHIP3003340, BRHIP3003395, BRHIP3003484, BRHIP3003644, BRHIP3003688, BRHIP3003795, BRHIP3004193, BRHIP3004416, BRHIP3004710, BRHIP3004725, BRHIP3004774, BRHIP3004786, BRHIP3004863, BRHIP3005037, BRHIP3005137, BRHIP3005142, BRHIP3005231, BRHIP3005307, BRHIP3005768, BRHIP3005944, BRHIP3006279, BRHIP3006294, BRHIP3006390, BRHIP3006449, BRHIP3006683, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007609, BRHIP3007960, BRHIP3008082, BRHIP3008183, BRHIP3008314, BRHIP3008320, BRHIP3008344, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3015620, BRHIP3015751, BRHIP3015854, BRHIP3016032, BRHIP3016213, BRHIP3016302, BRHIP3016421, BRHIP3017109, BRHIP3017256, BRHIP3017325, BRHIP3017558, BRHIP3017637, BRHIP3018784, BRHIP3019643, BRHIP3019824, BRHIP3019880, BRHIP3019956, BRHIP3020155, BRHIP3020182, BRHIP3020733, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026097, BRHIP3026231, BRHIP3026335, BRHIP3026651, BRHIP3027105, BRHIP3027137, BRHIP3027160, BRHIP3027191, BRHIP3027501, BRHIP3027651, BRHIP3027854, BRHIP3027947, BRHIP3028570, BRHIP3028742, BRSSN1000056, BRSSN1000059, BRSSN1000092, BRSSN1000153, BRSSN2000175, BRSSN2000197, BRSSN2000244, BRSSN2000518, BRSSN2000684, BRSSN2000715, BRSSN2001213, BRSSN2001275, BRSSN2001342, BRSSN2001426, BRSSN2001503, BRSSN2001579, BRSSN2001810, BRSSN2002857, BRSSN2002859, BRSSN2003086, BRSSN2003093, BRSSN2003820, BRSSN2003841, BRSSN2003937, BRSSN2004142, BRSSN2004303, BRSSN2004304, BRSSN2004657, BRSSN2004679, BRSSN2004686, BRSSN2004710, BRSSN2004719, BRSSN2005847, BRSSN2006133, BRSSN2006390, BRSSN2006575, BRSSN2006644, BRSSN2007076, BRSSN2007168, BRSSN2007202, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010019, BRSSN2010110, BRSSN2010830, BRSSN2011653, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013702, BRSSN2013753, BRSSN2013874, BRSSN2014112, BRSSN2014424, BRSSN2014513, BRSSN2014556, BRSSN2014573, BRSSN2014636, BRSSN2015369, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018581, BRSSN2018731, BRSSN2018771, BRSSN2018925, BRSTN1000097, BRSTN2000070, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006583, BRSTN2006638, BRSTN2006865, BRSTN2007000, BRSTN2007118, BRSTN2007284, BRSTN2007765, BRSTN2008283, BRSTN2008367, BRSTN2008418, BRSTN2008457, BRSTN2008475, BRSTN2008930, BRSTN2009247, BRSTN2010363, BRSTN2010416, BRSTN2010500, BRSTN2010569, BRSTN2010923, BRSTN2011211, BRSTN2011688, BRSTN2011799, BRSTN2012284, BRSTN2012320, BRSTN2012380, BRSTN2013171, BRSTN2013354, BRSTN2014490, BRSTN2014633, BRSTN2014751, BRSTN2014770, BRSTN2016066, BRSTN2016335, BRSTN2016678, BRSTN2016892, BRSTN2016918, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000063, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005448, BRTHA2005579, BRTHA2005831, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2009311, BRTHA2009349, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011321, BRTHA2011351, BRTHA2011500, BRTHA2011691, BRTHA2012129, BRTHA2012183, BRTHA2012189, BRTHA2012351, BRTHA2012392, BRTHA2012466, BRTHA2012555, BRTHA2012562, BRTHA2012714, BRTHA2012980, BRTHA2013262, BRTHA2013275, BRTHA2013426, BRTHA2013460, BRTHA2013515, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033264, BRTHA2033320, BRTHA2033469, BRTHA2033683, BRTHA2034281, BRTHA2034576, BRTHA2034874, BRTHA2035448, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3001221, BRTHA3001623, BRTHA3002265, BRTHA3002401, BRTHA3002411, BRTHA3002864, BRTHA3002933, BRTHA3002955, BRTHA3003000, BRTHA3003074, BRTHA3003225, BRTHA3003339, BRTHA3003343, BRTHA3003417, BRTHA3003449, BRTHA3003474, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009291, BRTHA3010135, BRTHA3010366, BRTHA3010469, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021786, BRTHA3022641, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026556, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000797, CHONS2001287, CHONS2001382, CHONS2001797, CHONS2001834, CHONS2002419, COLON1000030, COLON1000084, COLON1000135, COLON2000557, COLON2000568, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000020, CTONG1000022, CTONG1000040, CTONG1000056, CTONG1000062, CTONG1000086, CTONG1000093, CTONG1000097, CTONG1000113, CTONG1000137, CTONG1000165, CTONG1000180, CTONG1000181, CTONG1000220, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000218,. CTONG2000279, CTONG2000303, CTONG2000330, CTONG2000395, CTONG2000411, CTONG2000452, CTONG2000465, CTONG2000469, CTONG2000480, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001681, CTONG2001724, CTONG2001749, CTONG2001800, CTONG2001820, CTONG2001858, CTONG2001877, CTONG2001911, CTONG2001932, CTONG2001955, CTONG2002066, CTONG2002073, CTONG2002095, CTONG2002143, CTONG2002395, CTONG2002417, CTONG2002418, CTONG2002453, CTONG2002558, CTONG2002590, CTONG2002721, CTONG2002816, CTONG2002820, CTONG2002832, CTONG2002903, CTONG2002965, CTONG2002999, CTONG2003028, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003599, CTONG2003680, CTONG2003764, CTONG2003782, CTONG2003889, CTONG2003937, CTONG2003945, CTONG2004000, CTONG2004062, CTONG2004126, CTONG2004264, CTONG2004305, CTONG2004397, CTONG2004454, CTONG2004550, CTONG2004669, CTONG2004716, CTONG2004912, CTONG2004941, CTONG2004948, CTONG2005049, CTONG2005110, CTONG2005145, CTONG2005265, CTONG2005278, CTONG2005290, CTONG2005374, CTONG2005399, CTONG2005468, CTONG2005567, CTONG2005585, CTONG2005635, CTONG2005775, CTONG2005795, CTONG2005890, CTONG2005904, CTONG2005913, CTONG2006129, CTONG2006223, CTONG2006273, CTONG2006310, CTONG2006377, CTONG2006393, CTONG2006412, CTONG2006449, CTONG2006515, CTONG2006524, CTONG2006568, CTONG2006620, CTONG2006666, CTONG2006691, CTONG2006798, CTONG2006836, CTONG2006942, CTONG2006964, CTONG2007009, CTONG2007091, CTONG2007104, CTONG2007175, CTONG2007259, CTONG2007272, CTONG2007293, CTONG2007297, CTONG2007399, CTONG2007417, CTONG2007441, CTONG2007586, CTONG2007613, CTONG2007623, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008466, CTONG2008518, CTONG2008521, CTONG2008562, CTONG2008577, CTONG2008595, CTONG2008621, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010508, CTONG2010566, CTONG2010623, CTONG2010649, CTONG2010652, CTONG2011188, CTONG2011429, CTONG2011474, CTONG2011676, CTONG2011716, CTONG2011765, CTONG2011779, CTONG2011801, CTONG2011815, CTONG2011822, CTONG2011920, CTONG2011985, CTONG2012029, CTONG2012050, CTONG2012077, CTONG2012123, CTONG2012158, CTONG2012230, CTONG2012422, CTONG2012425, CTONG2012447, CTONG2012473, CTONG2012476, CTONG2012533, CTONG2012554, CTONG2012564, CTONG2012607, CTONG2012661, CTONG2012724, CTONG2012738, CTONG2012758, CTONG2012843, CTONG2012901, CTONG2012996, CTONG2013128, CTONG2013149, CTONG2013156, CTONG2013178, CTONG2013222, CTONG2013284, CTONG2013339, CTONG2013352, CTONG2013381, CTONG2013393, CTONG2013511, CTONG2013630, CTONG2013803, CTONG2013985, CTONG2013986, CTONG2014032, CTONG2014058, CTONG2014119, CTONG2014165, CTONG2014191, CTONG2014206, CTONG2014234, CTONG2014264, CTONG2014389, CTONG2014491, CTONG2014630, CTONG2014635, CTONG2014646, CTONG2014705, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015866, CTONG2015914, CTONG2015953, CTONG2016056, CTONG2016217, CTONG2016355, CTONG2016408, CTONG2016499, CTONG2016505, CTONG2016559, CTONG2016658, CTONG2016775, CTONG2016797, CTONG2016846, CTONG2016904, CTONG2016942, CTONG2016953, CTONG2017021, CTONG2017292, CTONG2017409, CTONG2017429, CTONG2017444, CTONG2017458, CTONG2017604, CTONG2017650, CTONG2017798, CTONG2017804, CTONG2017814, CTONG2017910, CTONG2017939, CTONG2017989, CTONG2018062, CTONG2018069, CTONG2018147, CTONG2018217, CTONG2018279, CTONG2018334, CTONG2018379, CTONG2018383, CTONG2018413, CTONG2018483, CTONG2018629, CTONG2018652, CTONG2018653, CTONG2018655, CTONG2018735, CTONG2018808, CTONG2018900, CTONG2018976, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020378, CTONG2020411, CTONG2020445, CTONG2020522, CTONG2020560, CTONG2020638, CTONG2020806, CTONG2020831, CTONG2020974, CTONG2021024, CTONG2021132, CTONG2021168, CTONG2022153, CTONG2022601, CTONG2023021, CTONG2023449, CTONG2023512, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027327, CTONG2027361, CTONG2027591, CTONG2027736, CTONG2027783, CTONG2027828, CTONG2028097, CTONG2028486, CTONG2028518, CTONG2028687, CTONG2028758, CTONG3000686, CTONG3000707, CTONG3000896, CTONG3001123, CTONG3001420, CTONG3001501, CTONG3001560, CTONG3001605, CTONG3002020, CTONG3002127, CTONG3002370, CTONG3002412, CTONG3002433, CTONG3002518, CTONG3002552, CTONG3002588, CTONG3002674, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009287, CTONG3009385, D3OST1000066, D3OST1000109, D3OST1000238, D3OST1000267, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D60ST2000127, D6OST2000169, D6OST2000245, D6OST2000507, D9OST2000031, D9OST2000278, D9OST2000440, D9OST2000869, D9OST2001319, D9OST2001433, D9OST2002397, D9OST2002673, D9OST2003106, D9OST2003137, D9OST2003397, D9OST2003594, D9OST2003762, D9OST2003791, D9OST2003989, D9OST2004018, D9OST2004073, D9OST2004417, DFNES1000003, DFNES1000107, DFNES1000150, DFNES1000185, DFNES2000011, DFNES2000143, DFNES2000146, DFNES2000153, DFNES2000292, DFNES2000426, DFNES2000432, DFNES2000443, DFNES2000471, DFNES2000913, DFNES2001091, DFNES2001108, DFNES2001177, DFNES2001404, DFNES2001564, DFNES2001565, DFNES2001647, DFNES2001800, DFNES2002220, DFNES2002509, DFNES2002550, DFNES2002588, DFNES2002817, DFNES2002966, DFNES2003081, DFNES2003255, DFNES2003742, DFNES2004346, DFNES2004354, DFNES2004383, DFNES2004608, DFNES2004684, DFNES2005266, DFNES2005527, DFNES2005766, DFNES2005779, DFNES2005875, DFNES2006346, DFNES2006944, DFNES2007113, DFNES2007299, DFNES2007332, DFNES2007641, DFNES2007757, DFNES2008280, DFNES2008881, DFNES2010502, DFNES2011192, DFNES2011221, DFNES2011239, DFNES2011245, ERLTF1000021, ERLTF2000324, ERLTF2000465, FCBBF1000023, FCBBF1000025, FCBBF1000038, FCBBF1000061, FCBBF1000069, FCBBF1000117, FCBBF1000118, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000412, FCBBF1000425, FCBBF1000437, FCBBF1000449, FCBBF1000506, FCBBF1000546, FCBBF1000550, FCBBF1000581, FCBBF1000582, FCBBF1000598, FCBBF1000604, FCBBF1000618, FCBBF1000627, FCBBF1000684, FCBBF1000686, FCBBF1000691, FCBBF1000748, FCBBF2000087, FCBBF2000094, FCBBF2000105, FCBBF2000123, FCBBF2000195, FCBBF2000214, FCBBF2000232, FCBBF2000259, FCBBF2000276, FCBBF2000291, FCBBF2000379, FCBBF2000473, FCBBF2000502, FCBBF2000576, FCBBF2000591, FCBBF2000677, FCBBF2000678, FCBBF2000685, FCBBF2000782, FCBBF2000808, FCBBF2000815, FCBBF2000825, FCBBF2000940, FCBBF2000951, FCBBF2001001, FCBBF2001088, FCBBF2001105, FCBBF2001116, FCBBF2001188, FCBBF2001238, FCBBF2001243, FCBBF2001309, FCBBF2001400, FCBBF2001427, FCBBF2001480, FCBBF2001529, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370, FCBBF2002541, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003636, FCBBF2003823, FCBBF2003895, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005439, FCBBF2005538, FCBBF2005637, FCBBF2005645, FCBBF2005658, FCBBF2005733, FCBBF2006131, FCBBF2006380, FCBBF2006418, FCBBF2006452, FCBBF2006634, FCBBF2007012, FCBBF2007186, FCBBF2007265, FCBBF2007302, FCBBF2007610, FCBBF2007633, FCBBF3000102, FCBBF3000110, FCBBF3000120, FCBBF3000227, FCBBF3000229, FCBBF3000233, FCBBF3000361, FCBBF3000367, FCBBF3000462, FCBBF3000473, FCBBF3000501, FCBBF3000518, FCBBF3000536, FCBBF3000550, FCBBF3000847, FCBBF3001018, FCBBF3001020, FCBBF3001235, FCBBF3001281, FCBBF3001302, FCBBF3001377, FCBBF3001594, FCBBF3001890, FCBBF3001912, FCBBF3001914, FCBBF3001918, FCBBF3001924, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004390, FCBBF3004409, FCBBF3004502, FCBBF3004715, FCBBF3004842, FCBBF3004847, FCBBF3004955, FCBBF3005160, FCBBF3005218, FCBBF3005320, FCBBF3005444, FCBBF3005497, FCBBF3005698, FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007829, FCBBF3007855, FCBBF3008073, FCBBF3008159, FCBBF3008251, FCBBF3008311, FCBBF3008362, FCBBF3008382, FCBBF3008475, FCBBF3008556, FCBBF3008644, FCBBF3008748, FCBBF3008870, FCBBF3008938, FCBBF3008944, FCBBF3009101, FCBBF3009152, FCBBF3009256, FCBBF3009317, FCBBF3009428, FCBBF3009464, FCBBF3009479, FCBBF3009703, FCBBF3009717, FCBBF3009888, FCBBF3009949, FCBBF3009978, FCBBF3010008, FCBBF3010124, FCBBF3010130, FCBBF3010149, FCBBF3010211, FCBBF3010361, FCBBF3010544, FCBBF3010586, FCBBF3010601, FCBBF3010665, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012288, FCBBF3012332, FCBBF3012347, FCBBF3012443, FCBBF3012588, FCBBF3012842, FCBBF3012901, FCBBF3013041, FCBBF3013058, FCBBF3013205, FCBBF3013266, FCBBF3013307, FCBBF3013341, FCBBF3013589, FCBBF3013623, FCBBF3013800, FCBBF3013846, FCBBF3014229, FCBBF3014355, FCBBF3014567, FCBBF3014770, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017535, FCBBF3017681, FCBBF3017729, FCBBF3017873, FCBBF3017974, FCBBF3018067, FCBBF3018173, FCBBF3018591, FCBBF3018753, FCBBF3018949, FCBBF3019085, FCBBF3019437, FCBBF3019455, FCBBF3019564, FCBBF3019570, FCBBF3019663, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021940, FCBBF3021985, FCBBF3022005, FCBBF3022504, FCBBF3022566, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024096, FCBBF3024225, FCBBF3024364, FCBBF3024623, FCBBF3024911, FCBBF3025098, FCBBF3025252, FCBBF3025280, FCBBF3025285, FCBBF3025528, FCBBF3025568, FCBBF3025650, FCBBF3025674, FCBBF3025730, FCBBF3025737, FCBBF3026021, FCBBF3026048, FCBBF3026251, FCBBF3026308, FCBBF3026678, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028472, FCBBF3028528, FCBBF3028582, FCBBF3028593, FCBBF3028671, FCBBF3028794, FCBBF4000061, FCBBF4000076, FCBBF4000173, FCBBF4000192, FCBBF4000193, FCBBF4000268, FCBBF4000282, FCBBF4000399, FCBBF4000415, FCBBF4000500, FCBBF4000529, FCBBF5000041, FCBBF5000061, FCBBF5000261, FCBBF5000384, FCBBF5000483, FCBBF5000495, FEBRA1000005, FEBRA1000008, FEBRA1000030, FEBRA1000138, FEBRA1000148, FEBRA1000149, FEBRA1000183, FEBRA1000190, FEBRA2000021, FEBRA2000035, FEBRA2000053, FEBRA2000126, FEBRA2000220, FEBRA2000228, FEBRA2000253, FEBRA2000282, FEBRA2000286, FEBRA2000297, FEBRA2000311, FEBRA2000321, FEBRA2000333, FEBRA2000378, FEBRA2000391, FEBRA2000394, FEBRA2000397, FEBRA2000399, FEBRA2000409, FEBRA2000415, FEBRA2000436, FEBRA2000454, FEBRA2000468, FEBRA2000491, FEBRA2000504, FEBRA2000510, FEBRA2000532, FEBRA2000538, FEBRA2000575, FEBRA2000656, FEBRA2000659, FEBRA2000690, FEBRA2000727, FEBRA2000734, FEBRA2000762, FEBRA2000772, FEBRA2000790, FEBRA2000803, FEBRA2000805, FEBRA2000809, FEBRA2000856, FEBRA2000862, FEBRA2000901, FEBRA2000909, FEBRA2000917, FEBRA2000928, FEBRA2000936, FEBRA2000937, FEBRA2000959, FEBRA2001012, FEBRA2001020, FEBRA2001093, FEBRA2001124, FEBRA2001133, FEBRA2001161, FEBRA2001175, FEBRA2001217, FEBRA2001227, FEBRA2001267, FEBRA2001351, FEBRA2001438, FEBRA2001440, FEBRA2001487, FEBRA2001523, FEBRA2001561, FEBRA2001581, FEBRA2001584, FEBRA2001590, FEBRA2001591, FEBRA2001610, FEBRA2001620, FEBRA2001659, FEBRA2001698, FEBRA2001705, FEBRA2001706, FEBRA2001772, FEBRA2001790, FEBRA2001867, FEBRA2001914, FEBRA2001989, FEBRA2002009, FEBRA2002086, FEBRA2002109, FEBRA2002260, FEBRA2002399, FEBRA2002410, FEBRA2002429, FEBRA2002442, FEBRA2002508, FEBRA2002525, FEBRA2002552, FEBRA2002574, FEBRA2002601, FEBRA2002611, FEBRA2002628, FEBRA2002662, FEBRA2002682, FEBRA2002707, FEBRA2002727, FEBRA2002783, FEBRA2002818, FEBRA2002858, FEBRA2002882, FEBRA2002897, FEBRA2002908, FEBRA2002962, FEBRA2002986, FEBRA2003054, FEBRA2003115, FEBRA2003155, FEBRA2003163, FEBRA2003181, FEBRA2003198, FEBRA2003253, FEBRA2003275, FEBRA2003308, FEBRA2003327, FEBRA2003429, FEBRA2003454, FEBRA2003468, FEBRA2003520, FEBRA2003524, FEBRA2003675, FEBRA2003707, FEBRA2003711, FEBRA2003822, FEBRA2003897, FEBRA2003930, FEBRA2004023, FEBRA2004026, FEBRA2004029, FEBRA2004053, FEBRA2004056, FEBRA2004110, FEBRA2004191, FEBRA2004202, FEBRA2004219, FEBRA2004325, FEBRA2004329, FEBRA2004331, FEBRA2004443, FEBRA2004490, FEBRA2004538, FEBRA2004592, FEBRA2004651, FEBRA2004813, FEBRA2004840, FEBRA2004852, FEBRA2004867, FEBRA2004914, FEBRA2004933, FEBRA2004987, FEBRA2005014, FEBRA2005036, FEBRA2005216, FEBRA2005291, FEBRA2005377, FEBRA2005380, FEBRA2005427, FEBRA2005458, FEBRA2005701, FEBRA2005726, FEBRA2005778, FEBRA2005787, FEBRA2005995, FEBRA2005998, FEBRA2006055, FEBRA2006061, FEBRA2006150, FEBRA2006165, FEBRA2006315, FEBRA2006354, FEBRA2006370, FEBRA2006379, FEBRA2006409, FEBRA2006418, FEBRA2006427, FEBRA2006476, FEBRA2006491, FEBRA2006627, FEBRA2006664, FEBRA2006667, FEBRA2006726, FEBRA2006793, FEBRA2006817, FEBRA2006890, FEBRA2006942, FEBRA2006977, FEBRA2007176, FEBRA2007200, FEBRA2007247, FEBRA2007280, FEBRA2007414, FEBRA2007436, FEBRA2007449, FEBRA2007458, FEBRA2007551, FEBRA2007566, FEBRA2007620, FEBRA2007793, FEBRA2007818, FEBRA2007823, FEBRA2007931, FEBRA2007957, FEBRA2008009, FEBRA2008086, FEBRA2008135, FEBRA2008165, FEBRA2008172, FEBRA2008266, FEBRA2008282, FEBRA2008311, FEBRA2008341, FEBRA2008360, FEBRA2008468, FEBRA2008475, FEBRA2008481, FEBRA2008542, FEBRA2008660, FEBRA2008681, FEBRA2008692, FEBRA2008741, FEBRA2008755, FEBRA2008836, FEBRA2008866, FEBRA2008881, FEBRA2008983, FEBRA2009016, FEBRA2009029, FEBRA2009074, FEBRA2009117, FEBRA2009120, FEBRA2009162, FEBRA2009240, FEBRA2009265, FEBRA2009276, FEBRA2009327, FEBRA2009352, FEBRA2009362, FEBRA2009385, FEBRA2009416, FEBRA2009437, FEBRA2009478, FEBRA2009507, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012195, FEBRA2012397, FEBRA2012418, FEBRA2012625, FEBRA2013019, FEBRA2013069, FEBRA2013274, FEBRA2013465, FEBRA2013570, FEBRA2013905, FEBRA2013951, FEBRA2014010, FEBRA2014122, FEBRA2014198, FEBRA2014213, FEBRA2014443, FEBRA2014578, FEBRA2015076, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017098, FEBRA2017138, FEBRA2017154, FEBRA2017200, FEBRA2017223, FEBRA2017333, FEBRA2017502, FEBRA2017736, FEBRA2017780, FEBRA2017811, FEBRA2017850, FEBRA2018051, FEBRA2018407, FEBRA2019172, FEBRA2019298, FEBRA2019457, FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021134, FEBRA2021339, FEBRA2021431, FEBRA2021497, FEBRA2021571, FEBRA2021892, FEBRA2021908, FEBRA2022013, FEBRA2022148, FEBRA2022204, FEBRA2022255, FEBRA2022322, FEBRA2022504, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023498, FEBRA2023721, FEBRA2023989, FEBRA2023990, FEBRA2024136, FEBRA2024150, FEBRA2024343, FEBRA2024570, FEBRA2024693, FEBRA2024727, FEBRA2024744, FEBRA2024797, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEHRT2000325, FEHRT2000364, FEKID1000018, FELIV1000101, FELIV1000164, FELNG2000241, HCASM1000021, HCASM1000050, HCASM1000061, HCASM1000130, HCASM2000202, HCASM2000266, HCASM2000307, HCASM2000363, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000131, HCHON1000142, HCHON1000176, HCHON2000028, HCHON2000038, HCHON2000087, HCHON2000100, HCHON2000156, HCHON2000199, HCHON2000212, HCHON2000271, HCHON2000315, HCHON2000322, HCHON2000344, HCHON2000364, HCHON2000373, HCHON2000395, HCHON2000417, HCHON2000418, HCHON2000503, HCHON2000660, HCHON2000663, HCHON2000673, HCHON2000699, HCHON2000705, HCHON2000738, HCHON2000751, HCHON2000817, HCHON2000818, HCHON2000832, HCHON2000851, HCHON2000898, HCHON2000935, HCHON2000942, HCHON2000956, HCHON2000994, HCHON2001084, HCHON2001099, HCHON2001110, HCHON2001116, HCHON2001214, HCHON2001233, HCHON2001269, HCHON2001450, HCHON2001453, HCHON2001519, HCHON2001523, HCHON2001535, HCHON2001546, HCHON2001548, HCHON2001577, HCHON2001598, HCHON2001604, HCHON2001607, HCHON2001646, HCHON2001712, HCHON2001768, HCHON2001801, HCHON2002123, HCHON2002194, HCHON2002241, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HEART1000088, HEART1000099, HEART1000102, HEART1000118, HEART1000132, HEART1000139, HEART1000142, HEART1000151, HEART1000185, HEART2000024, HEART2000035, HEART2000099, HEART2000298, HEART2000309, HEART2000411, HEART2000418, HEART2000444, HEART2000448, HEART2000492, HEART2000541, HEART2000568, HEART2000611, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2004570, HEART2004764, HEART2004931, HEART2004940, HEART2004941, HEART2004980, HEART2005244, HEART2005581, HEART2005610, HEART2006131, HEART2006195, HEART2006310, HEART2006334, HEART2006557, HEART2006787, HEART2006789, HEART2006909, HEART2007031, HEART2007443, HEART2007696, HEART2007767, HEART2008108, HEART2008111, HEART2008257, HEART2008509, HEART2008994, HEART2009599, HEART2009680, HEART2010391, HEART2010492, HEART2010495, HELAC2000158, HELAC2000301, HHDPC1000001, HHDPC1000065, HHDPC1000118, HHDPC2000070, HHDPC2000102, HHDPC2000258, HHDPC2000315, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2006460, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HLUNG1000017, HLUNG1000024, HLUNG1000030, HLUNG1000034, HLUNG1000037, HLUNG1000053, HLUNG1000076, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000255, HLUNG2000281, HLUNG2000315, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000870, HLUNG2000884, HLUNG2000926, HLUNG2000950, HLUNG2000955, HLUNG2000970, HLUNG2001013, HLUNG2001126, HLUNG2001144, HLUNG2001214, HLUNG2001439, HLUNG2001459, HLUNG2001633, HLUNG2001677, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002562, HLUNG2002659, HLUNG2002707, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003497, HLUNG2003691, HLUNG2003710, HLUNG2003714, HLUNG2003778, HLUNG2003833, HLUNG2003872, HLUNG2003918, HLUNG2003991, HLUNG2004014, HLUNG2004037, HLUNG2004154, HLUNG2004217, HLUNG2004273, HLUNG2004388, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006843, HLUNG2006852, HLUNG2006935, HLUNG2007041, HLUNG2007210, HLUNG2007219, HLUNG2007245, HLUNG2007433, HLUNG2007931, HLUNG2008066, HLUNG2008225, HLUNG2008235, HLUNG2008247, HLUNG2008333, HLUNG2008384, HLUNG2008396, HLUNG2008439, HLUNG2008479, HLUNG2008480, HLUNG2008521, HLUNG2008833, HLUNG2008875, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000148, HSYRA2000044, HSYRA2000135, HSYRA2000221, HSYRA2000237, HSYRA2000253, HSYRA2000332, HSYRA2000371, HSYRA2000424, HSYRA2000507, HSYRA2000510, HSYRA2000605, HSYRA2000760, HSYRA2000792, HSYRA2000832, HSYRA2000927, HSYRA2001103, HSYRA2001142, HSYRA2001153, HSYRA2001232, HSYRA2001255, HSYRA2001332, HSYRA2001353, HSYRA2001420, HSYRA2001484, HSYRA2001552, HSYRA2001580, HSYRA2001631, HSYRA2001638, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000161, IMR321000165, IMR321000207, IMR321000230, IMR321000253, IMR322000010, IMR322000107, IMR322000144, IMR322000152, IMR322000164, IMR322000178, IMR322000302, IMR322000374, IMR322000379, IMR322000383, IMR322000418, IMR322000604, IMR322000609, IMR322000656, IMR322000672, IMR322000791, IMR322000811, IMR322000838, IMR322000917, IMR322000919, IMR322000935, IMR322000953, IMR322000984, IMR322001018, IMR322001167, IMR322001185, IMR322001245, IMR322001452, IMR322001491, IMR322001665, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000036, KIDNE1000064, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000375, KIDNE2000394, KIDNE2000403, KIDNE2000422, KIDNE2000493, KIDNE2000497, KIDNE2000510, KIDNE2000513, KIDNE2000517, KIDNE2000519, KIDNE2000555, KIDNE2000624, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2001117, KIDNE2001140, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002198, KIDNE2002252, KIDNE2002262, KIDNE2002438, KIDNE2002483, KIDNE2002768, KIDNE2002795, KIDNE2002798, KIDNE2002839, KIDNE2002845, KIDNE2002872, KIDNE2002882, KIDNE2002883, KIDNE2002980, KIDNE2002991, KIDNE2003185, KIDNE2003188, KIDNE2003316, KIDNE2003335, KIDNE2003357, KIDNE2003373, KIDNE2003377, KIDNE2003507, KIDNE2003752, KIDNE2003837, KIDNE2003994, KIDNE2004034, KIDNE2004084, KIDNE2004095, KIDNE2004233, KIDNE2004262, KIDNE2004279, KIDNE2004294, KIDNE2004295, KIDNE2004305, KIDNE2004344, KIDNE2004394, KIDNE2004411, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006014, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006652, KIDNE2006687, KIDNE2006733, KIDNE2006811, KIDNE2006820, KIDNE2007005, KIDNE2007040, KIDNE2007077, KIDNE2007186, KIDNE2007222, KIDNE2007328, KIDNE2007356, KIDNE2007422, KIDNE2007569, KIDNE2007810, KIDNE2007811, KIDNE2007910, KIDNE2007944, KIDNE2008022, KIDNE2008072, KIDNE2008116, KIDNE2008117, KIDNE2008218, KIDNE2008315, KIDNE2008378, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011782, KIDNE2011858, KIDNE2012025, KIDNE2012188, KIDNE2012316, KIDNE2012361, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015987, KIDNE2016000, KIDNE2016036, KIDNE2016096, KIDNE2016142, KIDNE2016188, KIDNE2016327, KIDNE2016388, KIDNE2016464, KIDNE2016539, KIDNE2016570, KIDNE2016918, KIDNE2017007, KIDNE2017052, KIDNE2017151, KIDNE2017315, KIDNE2017332, KIDNE2017343, KIDNE2017454, KIDNE2017603, KIDNE2017956, KIDNE2018166, KIDNE2018167, KIDNE2018254, KIDNE2018268, KIDNE2018269, KIDNE2018287, KIDNE2018294, KIDNE2018352, KIDNE2018462, KIDNE2018617, KIDNE2018678, KIDNE2018727, KIDNE2018863, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099, LIVER1000111, LIVER1000126, LIVER1000139, LIVER1000155, LIVER1000165, LIVER1000175, LIVER1000224, LIVER1000278, LIVER1000303, LIVER1000397, LIVER1000421, LIVER1000433, LIVER1000479, LIVER1000482, LIVER1000542, LIVER2000003, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001329, LIVER2001389, LIVER2001461, LIVER2001539, LIVER2001608, LIVER2001835, LIVER2002638, LIVER2002644, LIVER2002660, LIVER2002702, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415, LIVER2007548, LIVER2007568, LIVER2007721, LIVER2008053, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000096, MAMGL1000132, MAMGL1000151, MAMGL1000178, MAMGL1000182, MAMGL1000184, MESAN1000032, MESAN1000035, MESAN1000079, MESAN1000121, MESAN1000147, MESAN1000180, MESAN2000023, MESAN2000026, MESAN2000067, MESAN2000092, MESAN2000149, MESAN2000167, MESAN2000264, MESAN2000291, MESAN2000337, MESAN2000434, MESAN2000457, MESAN2000462, MESAN2000620, MESAN2000711, MESAN2000815, MESAN2000894, MESAN2000909, MESAN2001627, MESAN2001770, MESAN2001979, MESAN2001980, MESAN2002113, MESAN2002147, MESAN2002424, MESAN2002687, MESAN2002709, MESAN2002790, MESAN2002844, MESAN2002940, MESAN2002978, MESAN2003035, MESAN2003037, MESAN2003039, MESAN2003058, MESAN2003101, MESAN2003190, MESAN2003322, MESAN2003444, MESAN2003490, MESAN2003529, MESAN2003646, MESAN2003662, MESAN2003851, MESAN2004138, MESAN2004288, MESAN2004575, MESAN2005284, MESAN2005303, MESAN2005371, MESAN2005633, MESAN2005724, MESAN2005808, MESAN2005811, MESAN2006022, MESAN2006043, MESAN2006328, MESAN2006580, MESAN2006599, MESAN2006743, MESAN2006953, MESAN2006971, MESAN2007032, MESAN2007440, MESAN2007597, MESAN2007755, MESAN2008222, MESAN2008415, MESAN2008460, MESAN2008536, MESAN2008679, MESAN2008821, MESAN2008926, MESAN2009019, MESAN2009081, MESAN2009105, MESAN2009311, MESAN2009418, MESAN2009503, MESAN2009522, MESAN2009580, MESAN2010031, MESAN2010114, MESAN2010312, MESAN2010321, MESAN2010681, MESAN2011545, MESAN2011597, MESAN2011627, MESAN2011632, MESAN2011977, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015391, MESAN2015401, MESAN2015501, MESAN2015708, MESAN2016159, MESAN2016304, MESAN2016409, MESAN2016552, MESAN2016965, MESAN2017152, MESAN2017417, MESAN2018209, MESAN2018576, MESAN2018670, MESTC1000147, MESTC2000150, MESTC2000153, MESTC2000170, N1ESE2000698, NB9N41000047, NB9N41000142, NB9N41000146, NB9N42000104, NB9N42000122, NB9N42000281, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2002352, NESOP2002418, NESOP2002487, NESOP2002738, NESOP2002780, NETRP1000034, NETRP1000082, NETRP2000086, NETRP2000182, NETRP2000439, NHNPC1000037, NHNPC1000084, NHNPC1000101, NHNPC1000124, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000091, NOVAR1000102, NOVAR2000136, NOVAR2000352, NOVAR2000412, NOVAR2000710, NOVAR2000962, NOVAR2001108, NOVAR2001620, NOVAR2001783, NOVAR2001876, NOVAR2002039, NT2NE1000004, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000222, NT2NE2000284, NT2NE2000299, NT2NE2000353, NT2NE2000356, NT2NE2000369, NT2NE2000374, NT2NE2000386, NT2NE2000390, NT2NE2000455, NT2NE2000470, NT2NE2000488, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000809, NT2NE2000950, NT2NE2000951, NT2NE2000980, NT2NE2001005, NT2NE2001021, NT2NE2001049, NT2NE2001142, NT2NE2001180, NT2NE2001181, NT2NE2001188, NT2NE2001191, NT2NE2001247, NT2NE2001252, NT2NE2001326, NT2NE2001372, NT2NE2001428, NT2NE2001432, NT2NE2001524, NT2NE2001530, NT2NE2001598, NT2NE2001617, NT2NE2001623, NT2NE2001626, NT2NE2001634, NT2NE2001648, NT2NE2001660, NT2NE2001666, NT2NE2001677, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003252, NT2NE2003280, NT2NE2003308, NT2NE2003309, NT2NE2003315, NT2NE2003393, NT2NE2003408, NT2NE2003457, NT2NE2003540, NT2NE2003571, NT2NE2003705, NT2NE2003752, NT2NE2003887, NT2NE2004255, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005323, NT2NE2005358, NT2NE2005371, NT2NE2005395, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006478, NT2NE2006531, NT2NE2006784, NT2NE2006813, NT2NE2006894, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008867, NT2NE2008961, NT2NE2008997, NT2NE2009138, NT2NE2009295, NT2NE2009426, NT2NE2009608, NT2NE2009691, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012243, NT2NE2012361, NT2NE2012448, NT2NE2012457, NT2NE2012493, NT2NE2012533, NT2NE2012603, NT2NE2012747, NT2NE2012790, NT2NE2012847, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015480, NT2NE2015511, NT2NE2015565, NT2NE2015626, NT2NE2015747, NT2NE2015860, NT2NE2015885, NT2NE2015904, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916, NT2NE2019056, NT2NE2019076, NT2NE2019099, NT2RI1000016, NT2RI1000035, NT2RI1000048, NT2RI1000164, NT2RI1000172, NT2RI2000004, NT2RI2000007, NT2RI2000117, NT2RI2000128, NT2RI2000173, NT2RI2000187, NT2RI2000270, NT2RI2000276, NT2RI2000313, NT2RI2000341, NT2RI2000348, NT2RI2000421, NT2RI2000480, NT2RI2000575, NT2RI2000588, NT2RI2000597, NT2RI2000669, NT2RI2000688, NT2RI2000689, NT2RI2000704, NT2RI2000738, NT2RI2000775, NT2RI2000822, NT2RI2000841, NT2RI2000865, NT2RI2000932, NT2RI2000974, NT2RI2000987, NT2RI2001010, NT2RI2001017, NT2RI2001030, NT2RI2001083, NT2RI2001167, NT2RI2001178, NT2RI2001235, NT2RI2001244, NT2RI2001307, NT2RI2001409, NT2RI2001410, NT2RI2001425, NT2RI2001449, NT2RI2001474, NT2RI2001519, NT2RI2001595, NT2RI2001624, NT2RI2001657, NT2RI2001726, NT2RI2001782, NT2RI2001836, NT2RI2001846, NT2RI2001910, NT2RI2001952, NT2RI2002022, NT2RI2002041, NT2RI2002117, NT2RI2002120, NT2RI2002153, NT2RI2002179, NT2RI2002252, NT2RI2002260, NT2RI2002270, NT2RI2002334, NT2RI2002359, NT2RI2002447, NT2RI2002481, NT2RI2002507, NT2RI2002517, NT2RI2002530, NT2RI2002549, NT2RI2002564, NT2RI2002592, NT2RI2002602, NT2RI2002729, NT2RI2002822, NT2RI2002852, NT2RI2002865, NT2RI2002923, NT2RI2002926, NT2RI2002970, NT2RI2003019, NT2RI2003067, NT2RI2003184, NT2RI2003360, NT2RI2003383, NT2RI2003392, NT2RI2003399, NT2RI2003419, NT2RI2003487, NT2RI2003667, NT2RI2003751, NT2RI2003884, NT2RI2003973, NT2RI2003993, NT2RI2004078, NT2RI2004079, NT2RI2004093, NT2RI2004136, NT2RI2004157, NT2RI2004188, NT2RI2004190, NT2RI2004209, NT2RI2004230, NT2RI2004312, NT2RI2004381, NT2RI2004442, NT2RI2004468, NT2RI2004606, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005087, NT2RI2005096, NT2RI2005130, NT2RI2005315, NT2RI2005358, NT2RI2005368, NT2RI2005382, NT2RI2005520, NT2RI2005579, NT2RI2005621, NT2RI2005628, NT2RI2005647, NT2RI2005670, NT2RI2005710, NT2RI2005772, NT2RI2005814, NT2RI2005818, NT2RI2005966, NT2RI2006081, NT2RI2006126, NT2RI2006127, NT2RI2006183, NT2RI2006257, NT2RI2006294, NT2RI2006345, NT2RI2006403, NT2RI2006445, NT2RI2006487, NT2RI2006506, NT2RI2006553, NT2RI2006565, NT2RI2006659, NT2RI2006662, NT2RI2006667, NT2RI2006679, NT2RI2006686, NT2RI2006703, NT2RI2006716, NT2RI2006735, NT2RI2006762, NT2RI2006788, NT2RI2006838, NT2RI2006853, NT2RI2006855, NT2RI2006973, NT2RI2007046, NT2RI2007048, NT2RI2007116, NT2RI2007133, NT2RI2007214, NT2RI2007277, NT2RI2007303, NT2RI2007334, NT2RI2007386, NT2RI2007406, NT2RI2007439, NT2RI2007469, NT2RI2007507, NT2RI2007729, NT2RI2007751, NT2RI2007754, NT2RI2007827, NT2RI2007853, NT2RI2007877, NT2RI2007891, NT2RI2007956, NT2RI2007965, NT2RI2008007, NT2RI2008050, NT2RI2008204, NT2RI2008221, NT2RI2008233, NT2RI2008455, NT2RI2008502, NT2RI2008598, NT2RI2008622, NT2RI2008656, NT2RI2008801, NT2RI2008841, NT2RI2008952, NT2RI2009005, NT2RI2009101, NT2RI2009103, NT2RI2009144, NT2RI2009151, NT2RI2009215, NT2RI2009259, NT2RI2009289, NT2RI2009301, NT2RI2009517, NT2RI2009585, NT2RI2009595, NT2RI2009855, NT2RI2010283, NT2RI2010508, NT2RI2011422, NT2RI2011450, NT2RI2011683, NT2RI2011803, NT2RI2012990, NT2RI2013345, NT2RI2013357, NT2RI2013373, NT2RI2014247, NT2RI2014551, NT2RI2014683, NT2RI2014733, NT2RI2015239, NT2RI2016128, NT2RI2016134, NT2RI2017529, NT2RI2018311, NT2RI2018363, NT2RI2018448, NT2RI2018883, NT2RI2018950, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001458, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003409, NT2RI3003738, NT2RI3003925, NT2RI3004133, NT2RI3004381, NT2RI3005202, NT2RI3005403, NT2RI3005416, NT2RI3005861, NT2RI3005923, NT2RI3005928, NT2RI3006132, NT2RI3006171, NT2RI3006340, NT2RI3006376, NT2RI3006666, NT2RI3006796, NT2RI3007065, NT2RI3007095, NT2RI3007158, NT2RI3007167, NT2RI3007443, NT2RI3007684, NT2RI3007757, NT2RI3007878, NT2RI3007978, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000033, NT2RP6000043, NT2RP6000060, NT2RP6000077, NT2RP6000100, NT2RP6000119, NT2RP6000127, NT2RP6000140, NT2RP6000150, NT2RP7000041, NT2RP7000109, NT2RP7000112, NT2RP7000259, NT2RP7000311, NT2RP7000359, NT2RP7000364, NT2RP7000380, NT2RP7000391, NT2RP7000425, NT2RP7000926, NT2RP7001074, NT2RP7001080, NT2RP7001156, NT2RP7001306, NT2RP7001319, NT2RP7001335, NT2RP7001532, NT2RP7001591, NT2RP7001602, NT2RP7001678, NT2RP7001856, NT2RP7002028, NT2RP7002064, NT2RP7002151, NT2RP7002163, NT2RP7002243, NT2RP7002282, NT2RP7002376, NT2RP7002450, NT2RP7002478, NT2RP7002554, NT2RP7002603, NT2RP7002650, NT2RP7002779, NT2RP7002841, NT2RP7002978, NT2RP7002982, NT2RP7003025, NT2RP7003050, NT2RP7003084, NT2RP7003091, NT2RP7003107, NT2RP7003134, NT2RP7003148, NT2RP7003261, NT2RP7003439, NT2RP7003629, NT2RP7003680, NT2RP7004027, NT2RP7004080, NT2RP7004204, NT2RP7004233, NT2RP7004260, NT2RP7004348, NT2RP7004358, NT2RP7004396, NT2RP7004541, NT2RP7004559, NT2RP7004641, NT2RP7004656, NT2RP7004687, NT2RP7004722, NT2RP7004766, NT2RP7004961, NT2RP7004964, NT2RP7004975, NT2RP7005118, NT2RP7005205, NT2RP7005323, NT2RP7005468, NT2RP7005629, NT2RP7005631, NT2RP7005669, NT2RP7005846, NT2RP7006033, NT2RP7006075, NT2RP7006141, NT2RP7006160, NT2RP7006296, NT2RP7006304, NT2RP7006374, NT2RP7006395, NT2RP7006408, NT2RP7006457, NT2RP7006539, NT2RP7006547, NT2RP7006601, NT2RP7006621, NT2RP7006636, NT2RP7006853, NT2RP7006886, NT2RP7006887, NT2RP7006980, NT2RP7006986, NT2RP7006995, NT2RP7007114, NT2RP7007221, NT2RP7007226, NT2RP7007269, NT2RP7007310, NT2RP7007359, NT2RP7007381, NT2RP7007387, NT2RP7007406, NT2RP7007480, NT2RP7007504, NT2RP7007580, NT2RP7007594, NT2RP7007617, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008315, NT2RP7008360, NT2RP7008406, NT2RP7008435, NT2RP7008454, NT2RP7008487, NT2RP7008544, NT2RP7008550, NT2RP7008557, NT2RP7008623, NT2RP7008657, NT2RP7008720, NT2RP7008855, NT2RP7008863, NT2RP7009019, NT2RP7009030, NT2RP7009147, NT2RP7009168, NT2RP7009215, NT2RP7009225, NT2RP7009236, NT2RP7009253, NT2RP7009267, NT2RP7009322, NT2RP7009363, NT2RP7009370, NT2RP7009397, NT2RP7009429, NT2RP7009439, NT2RP7009466, NT2RP7009502, NT2RP7009507, NT2RP7009577, NT2RP7009743, NT2RP7009867, NT2RP7010128, NT2RP7010235, NT2RP7010275, NT2RP7010521, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015512, NT2RP7015676, NT2RP7015789, NT2RP7015996, NT2RP7016508, NT2RP7016622, NT2RP7016911, NT2RP7017139, NT2RP7017284, NT2RP7017365, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000213, NTONG1000214, NTONG1000247, NTONG1000257, NTONG2000040, NTONG2000231, NTONG2000363, NTONG2000413, NTONG2000492, NTONG2000499, NTONG2000531, NTONG2000583, NTONG2000800, NTONG2000858, NTONG2000878, NTONG2000966, NTONG2000977, NTONG2000985, NTONG2001014, NTONG2001079, NTONG2001137, NTONG2001157, NTONG2001173, NTONG2001220, NTONG2001222, NTONG2001362, NTONG2001532, NTONG2001567, NTONG2001587, NTONG2001619, NTONG2001642, NTONG2002278, NTONG2002582, NTONG2002807, NTONG2002948, NTONG2002970, NTONG2002985, NTONG2003158, NTONG2003210, NTONG2003316, NTONG2003409, NTONG2003515, NTONG2003805, NTONG2003839, NTONG2003852, NTONG2003897, NTONG2003928, NTONG2004095, NTONG2004308, NTONG2004521, NTONG2004614, NTONG2004690, NTONG2004806, NTONG2004829, NTONG2004918, NTONG2004991, NTONG2005062, NTONG2005086, NTONG2005137, NTONG2005153, NTONG2005265, NTONG2005277, NTONG2005363, NTONG2005373, NTONG2005480, NTONG2005497, NTONG2005577, NTONG2005657, NTONG2005822, NTONG2005897, NTONG2006099, NTONG2006187, NTONG2006301, NTONG2006324, NTONG2006354, NTONG2006440, NTONG2006501, NTONG2006646, NTONG2006709, NTONG2007020, NTONG2007028, NTONG2007034, NTONG2007249, NTONG2007517, NTONG2007522, NTONG2007693, NTONG2008088, NTONG2008093, NTONG2008143, NTONG2008365, NTONG2008483, NTONG2008522, NTONG2008672, NTONG2008862, NTONG2008939, NTONG2008944, NTONG2009060, NTONG2009068, NTONG2009229, NTONG2009233, OCBBF1000016, OCBBF1000030, OCBBF1000042, OCBBF1000054, OCBBF1000067, OCBBF1000080, OCBBF1000085, OCBBF1000091, OCBBF1000104, OCBBF1000118, OCBBF1000119, OCBBF1000122, OCBBF1000130, OCBBF1000138, OCBBF1000145, OCBBF1000153, OCBBF1000185, OCBBF1000192, OCBBF1000315, OCBBF2000013, OCBBF2000287, OCBBF2000452, OCBBF2000703, OCBBF2000719, OCBBF2000904, OCBBF2000998, OCBBF2001075, OCBBF2001101, OCBBF2001124, OCBBF2001176, OCBBF2001252, OCBBF2001323, OCBBF2001389, OCBBF2001402, OCBBF2001408, OCBBF2001416, OCBBF2001473, OCBBF2001494, OCBBF2001527, OCBBF2001528, OCBBF2001586, OCBBF2001681, OCBBF2001706, OCBBF2001749, OCBBF2001910, OCBBF2001961, OCBBF2001983, OCBBF2002083, OCBBF2002124, OCBBF2002161, OCBBF2002357, OCBBF2002615, OCBBF2002663, OCBBF2002805, OCBBF2002865, OCBBF2002920, OCBBF2002980, OCBBF2003028, OCBBF2003091, OCBBF2003327, OCBBF2003543, OCBBF2003593, OCBBF2003744, OCBBF2004038, OCBBF2004147, OCBBF2004168, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005546, OCBBF2005843, OCBBF2005901, OCBBF2006005, OCBBF2006030, OCBBF2006058, OCBBF2006148, OCBBF2006154, OCBBF2006214, OCBBF2006241, OCBBF2006388, OCBBF2006438, OCBBF2006567, OCBBF2006624, OCBBF2006639, OCBBF2006660, OCBBF2006849, OCBBF2006859, OCBBF2006972, OCBBF2006987, OCBBF2007039, OCBBF2007051, OCBBF2007068, OCBBF2007121, OCBBF2007184, OCBBF2007194, OCBBF2007196, OCBBF2007238, OCBBF2007414, OCBBF2007428, OCBBF2007478, OCBBF2007485, OCBBF2007520, OCBBF2007555, OCBBF2007610, OCBBF2007756, OCBBF2007892, OCBBF2007931, OCBBF2007946, OCBBF2008041, OCBBF2008116, OCBBF2008138, OCBBF2008283, OCBBF2008330, OCBBF2008366, OCBBF2008466, OCBBF2008483, OCBBF2008520, OCBBF2008569, OCBBF2008586, OCBBF2008640, OCBBF2008691, OCBBF2008724, OCBBF2008760, OCBBF2008768, OCBBF2008770, OCBBF2008790, OCBBF2008814, OCBBF2008822, OCBBF2009115, OCBBF2009391, OCBBF2009424, OCBBF2009536, OCBBF2009571, OCBBF2009583, OCBBF2009603, OCBBF2009788, OCBBF2009920, OCBBF2009926, OCBBF2010040, OCBBF2010140, OCBBF2010281, OCBBF2010404, OCBBF2010416, OCBBF2010420, OCBBF2010604, OCBBF2010709, OCBBF2010792, OCBBF2010830, OCBBF2010841, OCBBF2010843, OCBBF2010931, OCBBF2010945, OCBBF2010978, OCBBF2010989, OCBBF2011137, OCBBF2011160, OCBBF2011177, OCBBF2011228, OCBBF2011283, OCBBF2011311, OCBBF2011625, OCBBF2011685, OCBBF2011706, OCBBF2011722, OCBBF2011767, OCBBF2011855, OCBBF2011872, OCBBF2011960, OCBBF2011981, OCBBF2012001, OCBBF2012028, OCBBF2012095, OCBBF2012139, OCBBF2012191, OCBBF2012262, OCBBF2012320, OCBBF2012436, OCBBF2012755, OCBBF2012936, OCBBF2012990, OCBBF2013049, OCBBF2013091, OCBBF2013123, OCBBF2013127, OCBBF2013208, OCBBF2013319, OCBBF2013507, OCBBF2013721, OCBBF2013789, OCBBF2013883, OCBBF2013926, OCBBF2014052, OCBBF2014322, OCBBF2014386, OCBBF2014541, OCBBF2014707, OCBBF2014828, OCBBF2014873, OCBBF2014880, OCBBF2014928, OCBBF2015031, OCBBF2015081, OCBBF2015232, OCBBF2015285, OCBBF2015334, OCBBF2015335, OCBBF2015503, OCBBF2015506, OCBBF2015599, OCBBF2015645, OCBBF2015659, OCBBF2015797, OCBBF2015931, OCBBF2015980, OCBBF2016038, OCBBF2016405, OCBBF2016467, OCBBF2016590, OCBBF2016591, OCBBF2016612, OCBBF2016690, OCBBF2016841, OCBBF2016928, OCBBF2017069, OCBBF2017306, OCBBF2017325, OCBBF2017489, OCBBF2017516, OCBBF2017665, OCBBF2017815, OCBBF2017888, OCBBF2017920, OCBBF2018014, OCBBF2018167, OCBBF2018206, OCBBF2018229, OCBBF2018362, OCBBF2018380, OCBBF2018563, OCBBF2018663, OCBBF2018687, OCBBF2018828, OCBBF2018873, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020453, OCBBF2020801, OCBBF2020825, OCBBF2020838, OCBBF2021020, OCBBF2021214, OCBBF2021323, OCBBF2021518, OCBBF2021788, OCBBF2021833, OCBBF2022573, OCBBF2022574, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025631, OCBBF2025730, OCBBF2026025, OCBBF2026035, OCBBF2026144, OCBBF2026645, OCBBF2026690, OCBBF2026981, OCBBF2027148, OCBBF2027354, OCBBF2027423, OCBBF2027478, OCBBF2027481, OCBBF2027511, OCBBF2027728, OCBBF2027782, OCBBF2027868, OCBBF2028055, OCBBF2028173, OCBBF2028421, OCBBF2029471, OCBBF2029901, OCBBF2030116, OCBBF2030354, OCBBF2030517, OCBBF2030574, OCBBF2030708, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033413, OCBBF2033869, OCBBF2033948, OCBBF2034333, OCBBF2034529, OCBBF2034637, OCBBF2034906, OCBBF2035110, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009279, PANCR1000012, PANCR1000021, PANCR1000081, PANCR1000086, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000180, PEBLM2000030, PEBLM2000112, PEBLM2000126, PEBLM2000170, PEBLM2000180, PEBLM2000213, PEBLM2000248, PEBLM2000270, PEBLM2000308, PEBLM2000321, PEBLM2000333, PEBLM2000338, PEBLM2000395, PEBLM2000479, PEBLM2000502, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003935, PEBLM2004015, PEBLM2004216, PEBLM2004290, PEBLM2004497, PEBLM2004733, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007188, PEBLM2007296, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005305, PERIC2005347, PERIC2005370, PERIC2005936, PERIC2006035, PERIC2006121, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000067, PLACE5000068, PLACE5000070, PLACE5000080, PLACE5000105, PLACE5000115, PLACE5000116, PLACE5000129, PLACE5000139, PLACE5000153, PLACE5000171, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000348, PLACE6000371, PLACE6000424, PLACE6000426, PLACE6000429, PLACE6000450, PLACE6000463, PLACE6000523, PLACE6000550, PLACE6000552, PLACE6000555, PLACE6000630, PLACE6000953, PLACE6001118, PLACE6001153, PLACE6001294, PLACE6001536, PLACE6001557, PLACE6001712, PLACE6001740, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002462, PLACE6002647, PLACE6002668, PLACE6002699, PLACE6002710, PLACE6002871, PLACE6002949, PLACE6002960, PLACE6003004, PLACE6003077, PLACE6003094, PLACE6003109, PLACE6003204, PLACE6003218, PLACE6003372, PLACE6003383, PLACE6003393, PLACE6003399, PLACE6003539, PLACE6003705, PLACE6003740, PLACE6003745, PLACE6003771, PLACE6003796, PLACE6003839, PLACE6003850, PLACE6004101, PLACE6004219, PLACE6004336, PLACE6004368, PLACE6004380, PLACE6004396, PLACE6004454, PLACE6004464, PLACE6004491, PLACE6004663, PLACE6004687, PLACE6004738, PLACE6004931, PLACE6004966, PLACE6004993, PLACE6005007, PLACE6005029, PLACE6005108, PLACE6005234, PLACE6005283, PLACE6005294, PLACE6005328, PLACE6005351, PLACE6005423, PLACE6005487, PLACE6005535, PLACE6005546, PLACE6005559, PLACE6005579, PLACE6005786, PLACE6006042, PLACE6006077, PLACE6006158, PLACE6006186, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007239, PLACE6007242, PLACE6007309, PLACE6007380, PLACE6007433, PLACE6007482, PLACE6007687, PLACE6007787, PLACE6007798, PLACE6007807, PLACE6007925, PLACE6008036, PLACE6008094, PLACE6008126, PLACE6008285, PLACE6008453, PLACE6008640, PLACE6008750, PLACE6008768, PLACE6008793, PLACE6008824, PLACE6008989, PLACE6009006, PLACE6009008, PLACE6009228, PLACE6009237, PLACE6009419, PLACE6009430, PLACE6009482, PLACE6009524, PLACE6009527, PLACE6009560, PLACE6009590, PLACE6009821, PLACE6009835, PLACE6009853, PLACE6010077, PLACE6010463, PLACE6010485, PLACE6010568, PLACE6010701, PLACE6010704, PLACE6010732, PLACE6010765, PLACE6010848, PLACE6010925, PLACE6010991, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013883, PLACE6013884, PLACE6013885, PLACE6014043, PLACE6014516, PLACE6014882, PLACE6015211, PLACE6015322, PLACE6015460, PLACE6015513, PLACE6015591, PLACE6015731, PLACE6015939, PLACE6016030, PLACE6016093, PLACE6016105, PLACE6016160, PLACE6016210, PLACE6016254, PLACE6016383, PLACE6016984, PLACE6017002, PLACE6017431, PLACE6017564, PLACE6017578, PLACE6017626, PLACE6017701, PLACE6017714, PLACE6017788, PLACE6017791, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008431, PLACE7008623, PLACE7008766, PLACE7009563, PROST1000033, PROST1000039, PROST1000065, PROST1000083, PROST1000085, PROST1000110, PROST1000120, PROST1000123, PROST1000136, PROST1000144, PROST1000145, PROST1000152, PROST1000167, PROST1000184, PROST1000217, PROST1000246, PROST1000262, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000203, PROST2000206, PROST2000212, PROST2000241, PROST2000267, PROST2000273, PROST2000274, PROST2000277, PROST2000325, PROST2000337, PROST2000432, PROST2000452, PROST2000463, PROST2000470, PROST2000505, PROST2000572, PROST2000717, PROST2000760, PROST2000835, PROST2000893, PROST2000961, PROST2001116, PROST2001138, PROST2001213, PROST2001414, PROST2001415, PROST2001465, PROST2001540, PROST2001552, PROST2001599, PROST2001611, PROST2001636, PROST2001739, PROST2001796, PROST2001805, PROST2001823, PROST2001899, PROST2001998, PROST2002078, PROST2002101, PROST2002212, PROST2002338, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003324, PROST2003340, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004258, PROST2004332, PROST2004481, PROST2004526, PROST2004727, PROST2004742, PROST2004817, PROST2005039, PROST2005076, PROST2005121, PROST2005131, PROST2005228, PROST2005272, PROST2005426, PROST2005630, PROST2005788, PROST2005880, PROST2005886, PROST2005904, PROST2005919, PROST2005943, PROST2006008, PROST2006020, PROST2006196, PROST2006201, PROST2006282, PROST2006343, PROST2006450, PROST2006491, PROST2006510, PROST2006536, PROST2006579, PROST2006688, PROST2006766, PROST2006782, PROST2006933, PROST2007122, PROST2007200, PROST2007237, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010400, PROST2010545, PROST2010579, PROST2010606, PROST2010782, PROST2010885, PROST2011012, PROST2011038, PROST2011297, PROST2011439, PROST2011482, PROST2011631, PROST2012005, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012780, PROST2012888, PROST2012890, PROST2013032, PROST2013053, PROST2013260, PROST2013327, PROST2013531, PROST2013605, PROST2013627, PROST2013873, PROST2013880, PROST2014422, PROST2014585, PROST2014601, PROST2014659, PROST2014916, PROST2014925, PROST2015124, PROST2015137, PROST2015198, PROST2015243, PROST2015246, PROST2015251, PROST2015287, PROST2015457, PROST2015464, PROST2015537, PROST2015545, PROST2015551, PROST2015636, PROST2015710, PROST2015756, PROST2015877, PROST2015924, PROST2015932, PROST2016195, PROST2016246, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016980, PROST2017098, PROST2017367, PROST2017413, PROST2017441, PROST2017578, PROST2017612, PROST2017617, PROST2017700, PROST2017701, PROST2017729, PROST2017836, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019398, PROST2019487, PROST2019781, PUAEN1000039, PUAEN1000081, PUAEN1000085, PUAEN1000087, PUAEN1000121, PUAEN1000161, PUAEN1000175, PUAEN1000239, PUAEN1000275, PUAEN1000276, PUAEN1000288, PUAEN2000080, PUAEN2000175, PUAEN2000247, PUAEN2000312, PUAEN2000394, PUAEN2000497, PUAEN2000535, PUAEN2000594, PUAEN2000684, PUAEN2000942, PUAEN2001260, PUAEN2001345, PUAEN2001526, PUAEN2001882, PUAEN2002473, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007350, PUAEN2007785, PUAEN2008123, PUAEN2008228, PUAEN2008314, PUAEN2008815, PUAEN2008939, PUAEN2008980, PUAEN2008985, PUAEN2009348, PUAEN2009534, PUAEN2009655, PUAEN2009795, PUAEN2009852, PUAEN2010346, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000065, SALGL1000107, SALGL1000171, SKMUS1000014, SKMUS1000030, SKMUS1000064, SKMUS1000067, SKMUS1000102, SKMUS1000104, SKMUS1000118, SKMUS1000124, SKMUS1000129, SKMUS1000138, SKMUS1000176, SKMUS2000061, SKMUS2000078, SKMUS2000137, SKMUS2000198, SKMUS2000287, SKMUS2000317, SKMUS2000330, SKMUS2000339, SKMUS2000365, SKMUS2000390, SKMUS2000416, SKMUS2000458, SKMUS2000467, SKMUS2000468, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000894, SKMUS2000898, SKMUS2000933, SKMUS2000945, SKMUS2000961, SKMUS2000973, SKMUS2000980, SKMUS2001014, SKMUS2001147, SKMUS2001157, SKMUS2001164, SKMUS2001199, SKMUS2001215, SKMUS2001247, SKMUS2001271, SKMUS2001287, SKMUS2001323, SKMUS2001364, SKMUS2001398, SKMUS2001492, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000137, SKNMC1000159, SKNMC1000229, SKNMC2000256, SKNMC2000356, SKNMC2000374, SKNMC2000583, SKNMC2000593, SKNMC2000612, SKNMC2000622, SKNMC2001324, SKNMC2001555, SKNMC2001596, SKNMC2001623, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000086, SKNSH1000174, SKNSH1000301, SKNSH1000308, SKNSH1000416, SKNSH2000051, SKNSH2000516, SKNSH2000550, SKNSH2000716, SKNSH2000971, SKNSH2001222, SKNSH2001875, SKNSH2001931, SKNSH2002054, SKNSH2002866, SKNSH2003174, SKNSH2003422, SKNSH2003466, SKNSH2003483, SKNSH2004039, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000103, SMINT1000118, SMINT1000137, SMINT2000007, SMINT2000032, SMINT2000040, SMINT2000060, SMINT2000084, SMINT2000145, SMINT2000159, SMINT2000176, SMINT2000185, SMINT2000227, SMINT2000229, SMINT2000239, SMINT2000267, SMINT2000396, SMINT2000400, SMINT2000518, SMINT2000529, SMINT2000530, SMINT2000537, SMINT2000538, SMINT2000541, SMINT2000545, SMINT2000558, SMINT2000562, SMINT2000568, SMINT2000609, SMINT2000747, SMINT2000811, SMINT2000824, SMINT2000984, SMINT2001114, SMINT2001158, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002202, SMINT2002210, SMINT2002224, SMINT2002281, SMINT2002311, SMINT2002314, SMINT2002328, SMINT2002414, SMINT2002457, SMINT2002620, SMINT2002743, SMINT2002778, SMINT2002880, SMINT2002882, SMINT2002884, SMINT2002899, SMINT2002974, SMINT2002976, SMINT2003074, SMINT2003128, SMINT2003317, SMINT2003386, SMINT2003423, SMINT2003505, SMINT2003551, SMINT2003569, SMINT2003641, SMINT2003644, SMINT2003866, SMINT2003905, SMINT2004023, SMINT2004037, SMINT2004047, SMINT2004086, SMINT2004144, SMINT2004280, SMINT2004339, SMINT2004473, SMINT2004547, SMINT2004729, SMINT2004781, SMINT2004891, SMINT2004909, SMINT2004992, SMINT2005075, SMINT2005161, SMINT2005174, SMINT2005213, SMINT2005330, SMINT2005387, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007219, SMINT2007294, SMINT2007365, SMINT2007391, SMINT2007433, SMINT2007508, SMINT2007526, SMINT2007579, SMINT2007790, SMINT2007796, SMINT2007867, SMINT2007932, SMINT2008042, SMINT2008054, SMINT2008277, SMINT2008329, SMINT2008455, SMINT2008491, SMINT2008531, SMINT2008545, SMINT2008558, SMINT2008590, SMINT2008625, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010144, SMINT2010200, SMINT2010278, SMINT2010324, SMINT2010326, SMINT2010369, SMINT2010426, SMINT2010500, SMINT2010533, SMINT2010629, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011888, SMINT2011958, SMINT2012040, SMINT2012122, SMINT2012179, SMINT2012285, SMINT2012734, SMINT2012793, SMINT2013031, SMINT2013129, SMINT2013170, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000178, SPLEN2000020, SPLEN2000047, SPLEN2000064, SPLEN2000072, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000258, SPLEN2000270, SPLEN2000307, SPLEN2000310, SPLEN2000350, SPLEN2000357, SPLEN2000364, SPLEN2000402, SPLEN2000414, SPLEN2000420, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000839, SPLEN2000882, SPLEN2001135, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001626, SPLEN2001650, SPLEN2001689, SPLEN2001710, SPLEN2001781, SPLEN2001881, SPLEN2001912, SPLEN2001945, SPLEN2002053, SPLEN2002133, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002477, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004346, SPLEN2004368, SPLEN2004611, SPLEN2004880, SPLEN2004984, SPLEN2005009, SPLEN2005227, SPLEN2005272, SPLEN2005342, SPLEN2005416, SPLEN2005450, SPLEN2005474, SPLEN2005560, SPLEN2005727, SPLEN2005790, SPLEN2005818, SPLEN2005838, SPLEN2005869, SPLEN2005927, SPLEN2005939, SPLEN2006122, SPLEN2006232, SPLEN2006268, SPLEN2006283, SPLEN2006325, SPLEN2006389, SPLEN2006425, SPLEN2006701, SPLEN2006785, SPLEN2006875, SPLEN2007182, SPLEN2007276, SPLEN2007314, SPLEN2007326, SPLEN2007350, SPLEN2007388, SPLEN2007619, SPLEN2007653, SPLEN2007689, SPLEN2007695, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008591, SPLEN2008786, SPLEN2009081, SPLEN2009088, SPLEN2009163, SPLEN2009315, SPLEN2009340, SPLEN2009512, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010862, SPLEN2010978, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012523, SPLEN2012524, SPLEN2012525, SPLEN2012606, SPLEN2012611, SPLEN2012619, SPLEN2012725, SPLEN2012743, SPLEN2012756, SPLEN2012800, SPLEN2012846, SPLEN2012889, SPLEN2012961, SPLEN2013108, SPLEN2013503, SPLEN2013644, SPLEN2013648, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015815, SPLEN2015891, SPLEN2015899, SPLEN2016045, SPLEN2016053, SPLEN2016069, SPLEN2016098, SPLEN2016135, SPLEN2016241, SPLEN2016250, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016781, SPLEN2016840, SPLEN2016863, SPLEN2016922, SPLEN2016932, SPLEN2016973, SPLEN2017043, SPLEN2017104, SPLEN2017147, SPLEN2017187, SPLEN2017189, SPLEN2017212, SPLEN2017426, SPLEN2017452, SPLEN2017538, SPLEN2017613, SPLEN2017620, SPLEN2017643, SPLEN2017687, SPLEN2017740, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019311, SPLEN2019323, SPLEN2019349, SPLEN2019375, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019985, SPLEN2020034, SPLEN2020153, SPLEN2020154, SPLEN2020166, SPLEN2020183, SPLEN2020223, SPLEN2020359, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030847, SPLEN2031004, SPLEN2031125, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036953, SPLEN2037077, SPLEN2037319, SPLEN2037444, SPLEN2037580, SPLEN2037630, SPLEN2037678, SPLEN2037722, SPLEN2037891, SPLEN2038055, SPLEN2038180, SPLEN2038407, SPLEN2038473, SPLEN2038834, SPLEN2039311, SPLEN2039672, SPLEN2039697, SPLEN2039845, SPLEN2039936, SPLEN2040222, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000257, STOMA2000279, STOMA2000383, STOMA2000386, STOMA2000478, STOMA2000529, STOMA2000539, STOMA2000567, STOMA2000640, STOMA2000678, STOMA2000686, STOMA2000888, STOMA2001025, STOMA2001389, STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, SYNOV1000045, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000824, SYNOV2000923, SYNOV2001014, SYNOV2001035, SYNOV2001088, SYNOV2001111, SYNOV2001212, SYNOV2001251, SYNOV2001300, SYNOV2001356, SYNOV2001374, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008336, SYNOV4009129, SYNOV4009139, SYNOV4009298, T1ESE2000116, T1ESE2000609, T1ESE2000904, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000613, TCERX2000998, TCOLN1000037, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002489, TESOP2002539, TESOP2002545, TESOP2002804, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000085, TESTI1000088, TESTI1000093, TESTI1000094, TESTI1000101, TESTI1000109, TESTI1000125, TESTI1000127, TESTI1000128, TESTI1000131, TESTI1000138, TESTI1000142, TESTI1000157, TESTI1000163, TESTI1000169, TESTI1000179, TESTI1000183, TESTI1000191, TESTI1000257, TESTI1000266, TESTI1000319, TESTI1000348, TESTI1000390, TESTI1000391, TESTI1000459, TESTI1000491, TESTI1000545, TESTI2000006, TESTI2000018, TESTI2000044, TESTI2000117, TESTI2000120, TESTI2000147, TESTI2000154, TESTI2000172, TESTI2000177, TESTI2000179, TESTI2000207, TESTI2000208, TESTI2000221, TESTI2000238, TESTI2000253, TESTI2000278, TESTI2000349, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000600, TESTI2000616, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000951, TESTI2000981, TESTI2001008, TESTI2001049, TESTI2001082, TESTI2001120, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518, TESTI2001556, TESTI2001593, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001869, TESTI2001879, TESTI2001894, TESTI2001898, TESTI2001915, TESTI2001950, TESTI2001959, TESTI2001968, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003074, TESTI2003089, TESTI2003104, TESTI2003109, TESTI2003117, TESTI2003130, TESTI2003131, TESTI2003139, TESTI2003152, TESTI2003181, TESTI2003193, TESTI2003196, TESTI2003228, TESTI2003236, TESTI2003255, TESTI2003258, TESTI2003277, TESTI2003299, TESTI2003311, TESTI2003321, TESTI2003327, TESTI2003330, TESTI2003347, TESTI2003354, TESTI2003356, TESTI2003372, TESTI2003376, TESTI2003413, TESTI2003418, TESTI2003419, TESTI2003432, TESTI2003454, TESTI2003475, TESTI2003476, TESTI2003498, TESTI2003512, TESTI2003539, TESTI2003545, TESTI2003551, TESTI2003574, TESTI2003579, TESTI2003580, TESTI2003589, TESTI2003596, TESTI2003598, TESTI2003615, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004085, TESTI2004089, TESTI2004111, TESTI2004119, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005775, TESTI2005827, TESTI2005835, TESTI2005860, TESTI2005892, TESTI2005911, TESTI2005933, TESTI2005937, TESTI2005948, TESTI2005979, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006109, TESTI2006125, TESTI2006179, TESTI2006209, TESTI2006212, TESTI2006218, TESTI2006238, TESTI2006255, TESTI2006258, TESTI2006278, TESTI2006333, TESTI2006360, TESTI2006409, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007183, TESTI2007220, TESTI2007234, TESTI2007320, TESTI2007346, TESTI2007358, TESTI2007402, TESTI2007405, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009325, TESTI2009390, TESTI2009400, TESTI2009412, TESTI2009423, TESTI2009447, TESTI2009462, TESTI2009477, TESTI2009497, TESTI2009502, TESTI2009511, TESTI2009544, TESTI2009545, TESTI2009551, TESTI2009577, TESTI2009585, TESTI2009588, TESTI2009727, TESTI2009739, TESTI2009812, TESTI2009835, TESTI2009853, TESTI2009935, TESTI2009977, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011575, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012152, TESTI2012155, TESTI2012171, TESTI2012207, TESTI2012231, TESTI2012244, TESTI2012308, TESTI2012356, TESTI2012387, TESTI2012394, TESTI2012444, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013264, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014843, TESTI2014979, TESTI2014988, TESTI2015042, TESTI2015105, TESTI2015180, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016599, TESTI2016629, TESTI2016663, TESTI2016667, TESTI2016688, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017069, TESTI2017089, TESTI2017102, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020871, TESTI2020905, TESTI2020906, TESTI2020946, TESTI2020956, TESTI2020981, TESTI2020999, TESTI2021003, TESTI2021057, TESTI2021077, TESTI2021112, TESTI2021114, TESTI2021116, TESTI2021118, TESTI2021122, TESTI2021138, TESTI2021297, TESTI2021315, TESTI2021358, TESTI2021447, TESTI2021463, TESTI2021599, TESTI2021637, TESTI2021667, TESTI2021736, TESTI2021785, TESTI2021819, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025448, TESTI2025454, TESTI2025486, TESTI2025546, TESTI2025582, TESTI2025609, TESTI2025656, TESTI2025757, TESTI2025791, TESTI2025872, TESTI2025879, TESTI2025911, TESTI2025920, TESTI2025924, TESTI2026014, TESTI2026024, TESTI2026064, TESTI2026077, TESTI2026104, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026674, TESTI2026794, TESTI2026824, TESTI2026854, TESTI2026925, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028384, TESTI2028426, TESTI2028488, TESTI2028523, TESTI2028583, TESTI2028613, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034931, TESTI2034953, TESTI2034997, TESTI2035078, TESTI2035084, TESTI2035107, TESTI2035121, TESTI2035124, TESTI2035183, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036684, TESTI2036691, TESTI2036736, TESTI2036822, TESTI2036833, TESTI2036913, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000370, TESTI4000394, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527, TESTI4001569, TESTI4001604, TESTI4001665, TESTI4001679, TESTI4001795, TESTI4001923, TESTI4001925, TESTI4001984, TESTI4002003, TESTI4002141, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4003179, TESTI4003279, TESTI4003319, TESTI4003404, TESTI4003565, TESTI4003574, TESTI4003579, TESTI4003733, TESTI4003796, TESTI4003944, TESTI4004003, TESTI4004031, TESTI4004200, TESTI4004210, TESTI4004539, TESTI4004653, TESTI4004695, TESTI4004722, TESTI4004887, TESTI4004917, TESTI4005015, TESTI4005039, TESTI4005158, TESTI4005317, TESTI4005322, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009286, TESTI4009306, TESTI4009374, TESTI4009406, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013685, TESTI4013735, TESTI4013742, TESTI4013774, TESTI4013830, TESTI4013852, TESTI4013924, TESTI4013960, TESTI4013962, TESTI4014175, TESTI4014265, TESTI4014276, TESTI4014392, TESTI4014415, TESTI4014445, TESTI4014661, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037228, TESTI4037244, TESTI4037727, TESTI4037949, TESTI4038047, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000029, THYMU1000033, THYMU1000060, THYMU1000074, THYMU1000098, THYMU1000105, THYMU1000136, THYMU1000142, THYMU1000158, THYMU1000176, THYMU1000179, THYMU1000191, THYMU1000217, THYMU1000240, THYMU1000242, THYMU1000291, THYMU1000316, THYMU1000318, THYMU1000329, THYMU1000336, THYMU1000366, THYMU1000382, THYMU1000393, THYMU1000394, THYMU1000399, THYMU1000426, THYMU1000428, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000492, THYMU2000519, THYMU2000570, THYMU2000602, THYMU2000660, THYMU2000671, THYMU2000672, THYMU2000702, THYMU2000767, THYMU2000775, THYMU2000800, THYMU2000879, THYMU2000932, THYMU2000950, THYMU2000971, THYMU2000982, THYMU2000990, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002852, THYMU2002910, THYMU2002950, THYMU2003069, THYMU2003107, THYMU2003124, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003632, THYMU2003650, THYMU2003700, THYMU2003760, THYMU2003803, THYMU2003855, THYMU2003907, THYMU2003932, THYMU2003981, THYMU2004003, THYMU2004008, THYMU2004042, THYMU2004108, THYMU2004111, THYMU2004123, THYMU2004139, THYMU2004169, THYMU2004320, THYMU2004336, THYMU2004356, THYMU2004452, THYMU2004512, THYMU2004635, THYMU2004639, THYMU2004668, THYMU2004677, THYMU2004688, THYMU2004693, THYMU2004835, THYMU2004843, THYMU2004906, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005546, THYMU2005576, THYMU2005748, THYMU2005759, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007060, THYMU2007095, THYMU2007112, THYMU2007146, THYMU2007179, THYMU2007258, THYMU2007307, THYMU2007308, THYMU2007339, THYMU2007350, THYMU2007384, THYMU2007493, THYMU2007521, THYMU2007528, THYMU2007530, THYMU2007561, THYMU2007571, THYMU2007635, THYMU2007658, THYMU2007667, THYMU2007725, THYMU2007802, THYMU2007824, THYMU2007854, THYMU2007952, THYMU2007969, THYMU2008035, THYMU2008049, THYMU2008072, THYMU2008111, THYMU2008145, THYMU2008149, THYMU2008185, THYMU2008191, THYMU2008207, THYMU2008226, THYMU2008282, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009596, THYMU2009792, THYMU2009835, THYMU2009906, THYMU2009948, THYMU2010030, THYMU2010046, THYMU2010059, THYMU2010082, THYMU2010094, THYMU2010161, THYMU2010192, THYMU2010519, THYMU2010637, THYMU2010671, THYMU2010699, THYMU2010705, THYMU2010831, THYMU2010848, THYMU2011053, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2012002, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012701, THYMU2012807, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014327, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017675, THYMU2017707, THYMU2017831, THYMU2017844, THYMU2017948, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023576, THYMU2023711, THYMU2023900, THYMU2023943, THYMU2024121, THYMU2024185, THYMU2024207, THYMU2024616, THYMU2024684, THYMU2024748, THYMU2025042, THYMU2025189, THYMU2025319, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029676, THYMU2029688, THYMU2029788, THYMU2030068, THYMU2030226, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037233, THYMU2037234, THYMU2037348, THYMU2037406, THYMU2037965, THYMU2038058, THYMU2038189, THYMU2038199, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039788, THYMU2039989, THYMU2040029, THYMU2040110, THYMU2040114, THYMU2040140, THYMU2040412, THYMU2040824, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000390, THYMU3000490, THYMU3000504, THYMU3000542, THYMU3000655, THYMU3000826, THYMU3000841, THYMU3001077, THYMU3001082, THYMU3001133, THYMU3001151, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003350, THYMU3003403, THYMU3003565, THYMU3003763, THYMU3003865, THYMU3003958, THYMU3003965, THYMU3004157, THYMU3004628, THYMU3004632, THYMU3004866, THYMU3005050, THYMU3005378, THYMU3005407, THYMU3005415, THYMU3005427, THYMU3005482, THYMU3005491, THYMU3005696, THYMU3006118, THYMU3006132, THYMU3006168, THYMU3006172, THYMU3006371, THYMU3006640, THYMU3006767, THYMU3006811, THYMU3006963, THYMU3007137, THYMU3007368, THYMU3007559, THYMU3007845, THYMU3008060, THYMU3008136, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3033402, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046360, THYMU3046856, THYMU3047115, THYMU3047144, THYMU3047156, THYMU3047366, THYMU3047513, THYMU3047542, THYMU3047760, THYMU3047891, TKIDN1000044, TKIDN1000062, TKIDN1000066, TKIDN1000171, TKIDN2000127, TKIDN2000240, TKIDN2000319, TKIDN2000464, TKIDN2000521, TKIDN2000701, TKIDN2001529, TKIDN2001662, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003597, TKIDN2004185, TKIDN2004386, TKIDN2004458, TKIDN2004748, TKIDN2005721, TKIDN2005934, TKIDN2005947, TKIDN2005956, TKIDN2006525, TKIDN2006852, TKIDN2007667, TKIDN2008778, TKIDN2009092, TKIDN2009330, TKIDN2009481, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015161, TKIDN2015263, TKIDN2016309, TKIDN2016399, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000018, TRACH1000038, TRACH1000057, TRACH1000063, TRACH1000074, TRACH1000100, TRACH1000106, TRACH1000125, TRACH1000140, TRACH1000144, TRACH1000212, TRACH1000240, TRACH2000002, TRACH2000015, TRACH2000024, TRACH2000032, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000411, TRACH2000496, TRACH2000497, TRACH2000502, TRACH2000522, TRACH2000540, TRACH2000559, TRACH2000665, TRACH2000675, TRACH2000703, TRACH2000767, TRACH2000775, TRACH2000807, TRACH2000862, TRACH2000894, TRACH2000898, TRACH2000923, TRACH2000944, TRACH2000981, TRACH2000986, TRACH2001037, TRACH2001060, TRACH2001085, TRACH2001101, TRACH2001121, TRACH2001147, TRACH2001154, TRACH2001188, TRACH2001192, TRACH2001275, TRACH2001335, TRACH2001339, TRACH2001347, TRACH2001395, TRACH2001400, TRACH2001403, TRACH2001416, TRACH2001428, TRACH2001443, TRACH2001463, TRACH2001474, TRACH2001525, TRACH2001532, TRACH2001592, TRACH2001596, TRACH2001621, TRACH2001651, TRACH2001898, TRACH2001933, TRACH2001968, TRACH2001996, TRACH2002043, TRACH2002054, TRACH2002092, TRACH2002100, TRACH2002138, TRACH2002333, TRACH2002784, TRACH2002840, TRACH2002847, TRACH2002853, TRACH2002862, TRACH2002954, TRACH2002960, TRACH2002988, TRACH2003070, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004428, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005811, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894, TRACH2007969, TRACH2007988, TRACH2008081, TRACH2008112, TRACH2008127, TRACH2008130, TRACH2008278, TRACH2008478, TRACH2008540, TRACH2008583, TRACH2008597, TRACH2008820, TRACH2008921, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010883, TRACH2010965, TRACH2010974, TRACH2011057, TRACH2011093, TRACH2011290, TRACH2011293, TRACH2011302, TRACH2011500, TRACH2011574, TRACH2011956, TRACH2012000, TRACH2012138, TRACH2012242, TRACH2012298, TRACH2012370, TRACH2012387, TRACH2012497, TRACH2012536, TRACH2012562, TRACH2012742, TRACH2012823, TRACH2012918, TRACH2013081, TRACH2013123, TRACH2013265, TRACH2013369, TRACH2013450, TRACH2013495, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, 5 TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025379, TRACH2025507, TRACH2025569, TRACH2025705, TRACH2025749, TRACH2025753, TRACH2025810, TRACH2025853, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002866, TRACH3002871, TRACH3002876, TRACH3002890, TRACH3003009, TRACH3003037, TRACH3003357, TRACH3003379, TRACH3003586, TRACH3003683, TRACH3003780, TRACH3003805, TRACH3003832, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006149, TRACH3006228, TRACH3006264, TRACH3006336, TRACH3006379, TRACH3006392, TRACH3006395, TRACH3006397, TRACH3006470, TRACH3006589, TRACH3006626, TRACH3006699, ) TRACH3006800, TRACH3006889, TRACH3006985, TRACH3007097, TRACH3007268, TRACH3007274, TRACH3007277, TRACH3007391, TRACH3007541, TRACH3007595, TRACH3007627, TRACH3007689, TRACH3007766, TRACH3007866, TRACH3008042, TRACH3008061, TRACH3008201, TRACH3008508, TRACH3008535, TRACH3008629, TRACH3008688, TRACH3008697, TRACH3008713, TRACH3008887, TRACH3008902, TRACH3008990, TRACH3009008, TRACH3009059, TRACH3009061, TRACH3009148, TRACH3009455, TRACH3009701, TRACH3009715, TRACH3009894, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036997, TRACH3037063, TRACH3037267, TRACH3037288, TRACH3037505, TRACH3037573, TRACH3037696, TRACH3037897, TRACH3038086, TSTOM1000135, TSTOM1000186, TSTOM2000139, TSTOM2000235, TSTOM2000315, TSTOM2000442, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002356, TUTER2002729, UMVEN1000143, UMVEN1000156, UMVEN1000186, UMVEN2000046, UMVEN2000069, UMVEN2000121, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU2000047, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002294, UTERU2002332, UTERU2002473, UTERU2002547, UTERU2002693, UTERU2002733, UTERU2002736, UTERU2002737, UTERU2002841, UTERU2002964, UTERU2002993, UTERU2003035, UTERU2003126, UTERU2003135, UTERU2003321, UTERU2003399, UTERU2003411, UTERU2003577, UTERU2003926, UTERU2003973, UTERU2004015, UTERU2004037, UTERU2004039, UTERU2004061, UTERU2004073, UTERU2004163, UTERU2004299, UTERU2004461, UTERU2004520, UTERU2004564, UTERU2004688, UTERU2004698, UTERU2004807, UTERU2005004, UTERU2005050, UTERU2005069, UTERU2005074, UTERU2005179, UTERU2005292, UTERU2005346, UTERU2005450, UTERU2005533, UTERU2005601, UTERU2005664, UTERU2005822, UTERU2005903, UTERU2005905, UTERU2006103, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007942, UTERU2008018, UTERU2008019, UTERU2008027, UTERU2008077, UTERU2008130, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008939, UTERU2008962, UTERU2009094, UTERU2009120, UTERU2009131, UTERU2009147, UTERU2009206, UTERU2009283, UTERU2009414, UTERU2009483, UTERU2009538, UTERU2009540, UTERU2009776, UTERU2009904, UTERU2009951, UTERU2009972, UTERU2010115, UTERU2010124, UTERU2010164, UTERU2010231, UTERU2010304, UTERU2010320, UTERU2010431, UTERU2010525, UTERU2010651, UTERU2010724, UTERU2011195, UTERU2011199, UTERU2011261, UTERU2011287, UTERU2011410, UTERU2011574, UTERU2011657, UTERU2011741, UTERU2011806, UTERU2011811, UTERU2011897, UTERU2011962, UTERU2011968, UTERU2012031, UTERU2012101, UTERU2012114, UTERU2012252, UTERU2012333, UTERU2012581, UTERU2012610, UTERU2012615, UTERU2012688, UTERU2012703, UTERU2012715, UTERU2012741, UTERU2012767, UTERU2012786, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014223, UTERU2014398, UTERU2014464, UTERU2014548, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757, UTERU2016761, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017762, UTERU2017810, UTERU2017988, UTERU2018127, UTERU2018180, UTERU2018333, UTERU2018364, UTERU2018514, UTERU2018522, UTERU2018523, UTERU2018544, UTERU2018609, UTERU2018674, UTERU2018784, UTERU2018789, UTERU2018811, UTERU2018867, UTERU2018881, UTERU2018955, UTERU2019038, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2022020, UTERU2022773, UTERU2022955, UTERU2022981, UTERU2023039, UTERU2023045, UTERU2023175, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000402, UTERU3000645, UTERU3000665, UTERU3000738, UTERU3000828, UTERU3000844, UTERU3000899, UTERU3000959, UTERU3001029, UTERU3001053, UTERU3001059, UTERU3001240, UTERU3001394, UTERU3001542, UTERU3001558, UTERU3001571, UTERU3001585, UTERU3001632, UTERU3001652, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005264, UTERU3005422, UTERU3005585, UTERU3005907, UTERU3006008, UTERU3006228, UTERU3006538, UTERU3006640, UTERU3006687, UTERU3006720, UTERU3006798, UTERU3006884, UTERU3007021, UTERU3007104, UTERU3007108, UTERU3007134, UTERU3007640, UTERU3007731, UTERU3008280, UTERU3008463, UTERU3008660, UTERU3008671, UTERU3008722, UTERU3008917, UTERU3009259, UTERU3009490, UTERU3009690, UTERU3009775, UTERU3009871, UTERU3009979, UTERU3009987, UTERU3010029, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the BRAWH, BRCAN, BRCOC, BRHIP, BRSSN, BRSTN, and BRTHA libraries were analyzed for expression frequency. The analysis results are described below. Each is a relative value, where the expression level over all tissues is taken as 100. The larger this value, the greater the level of expression.

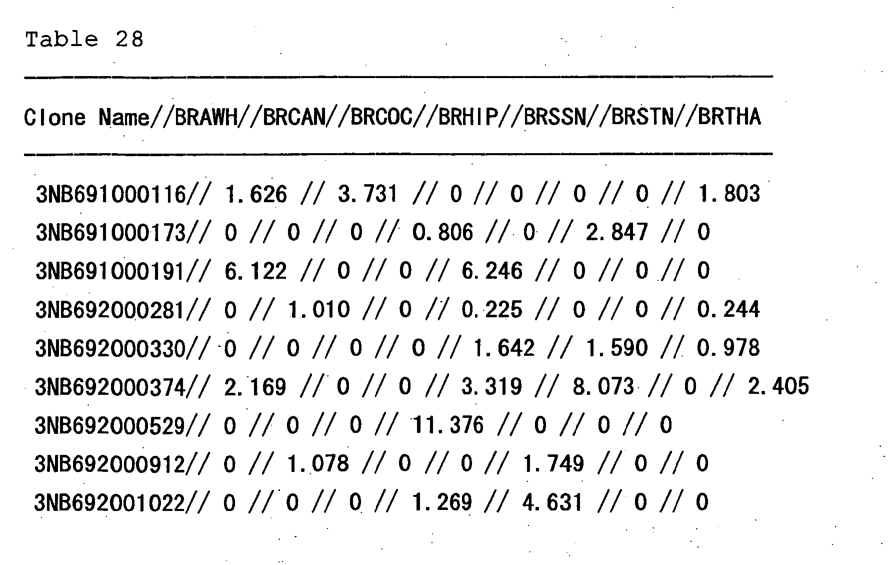

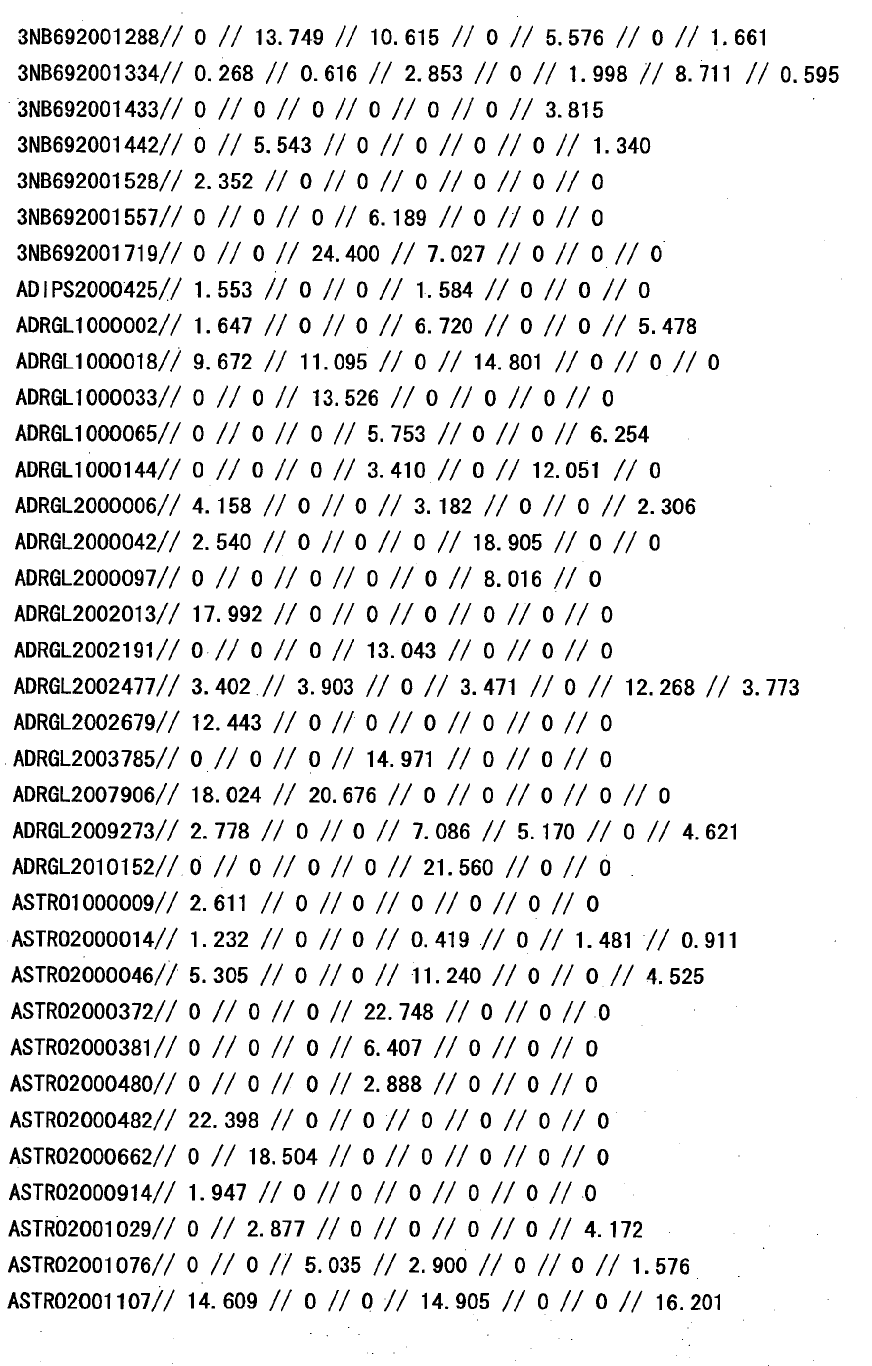

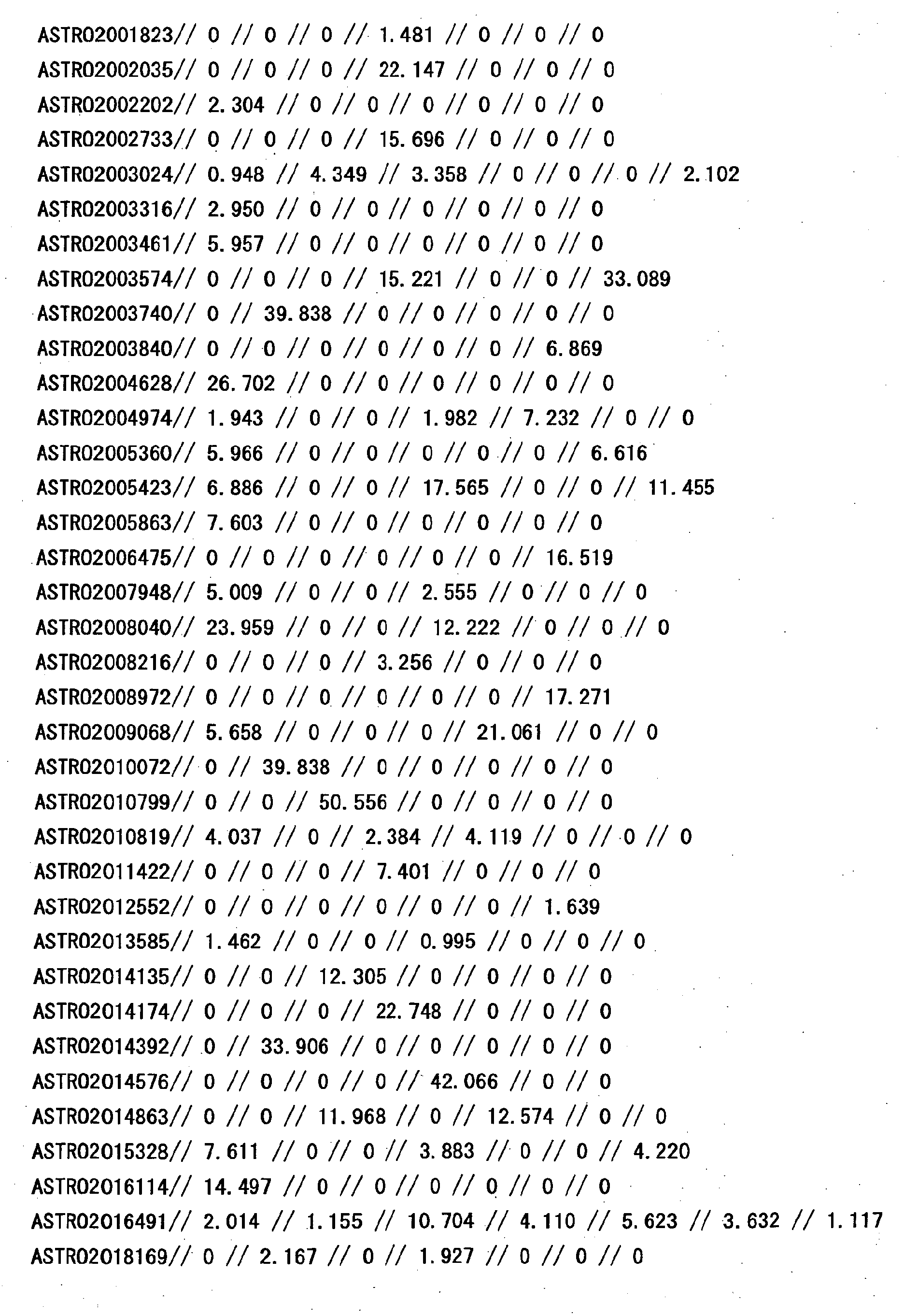

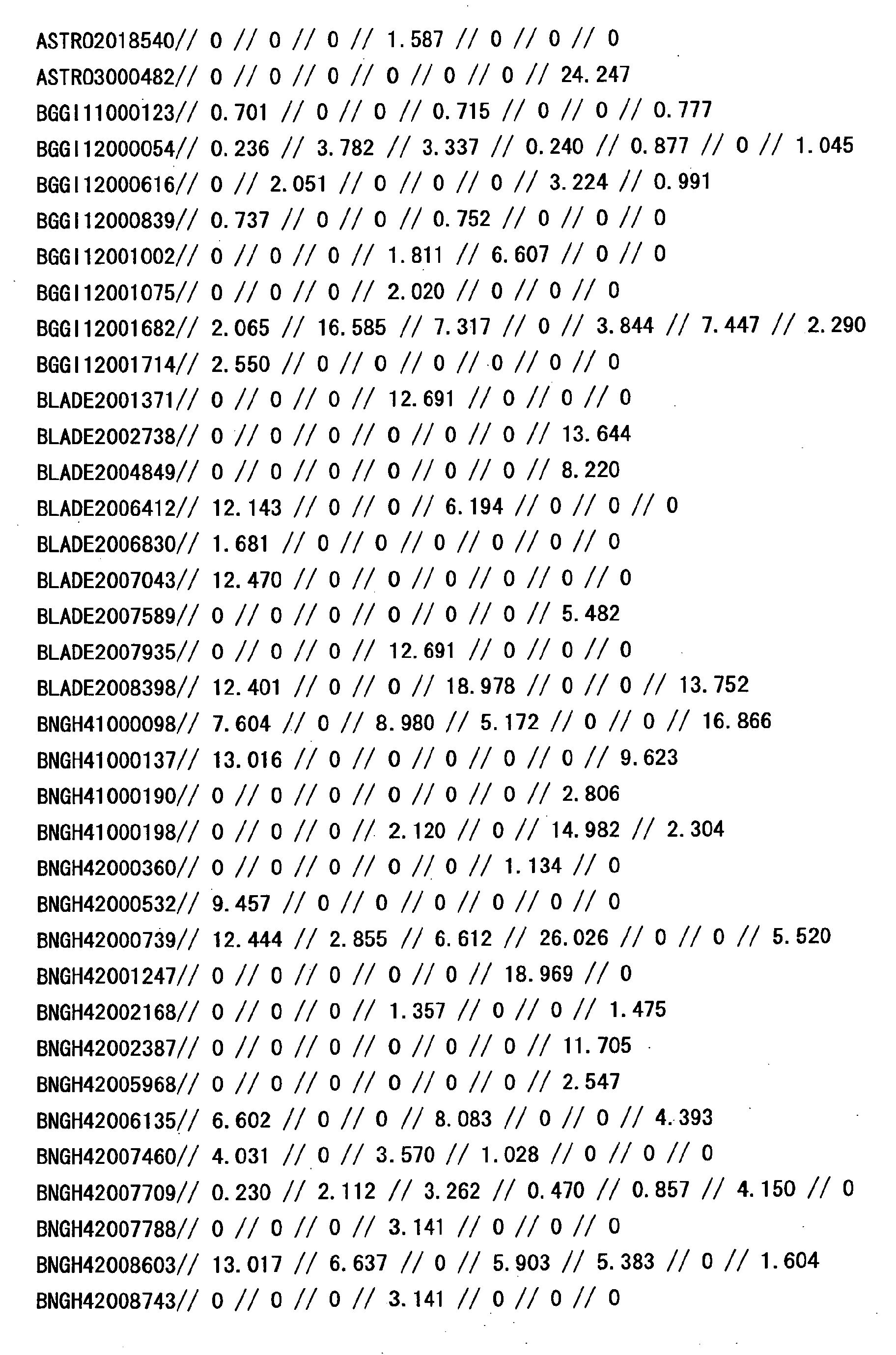

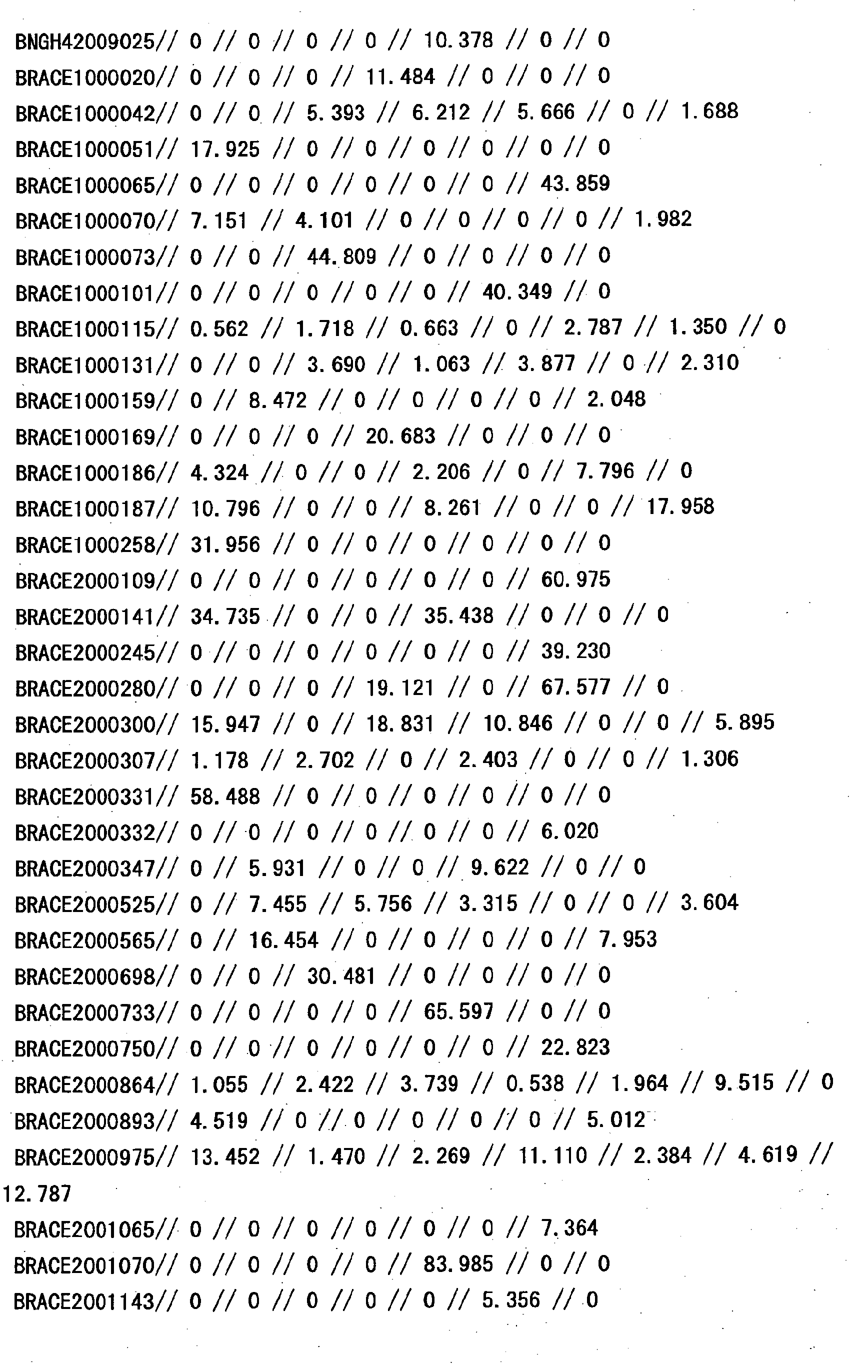

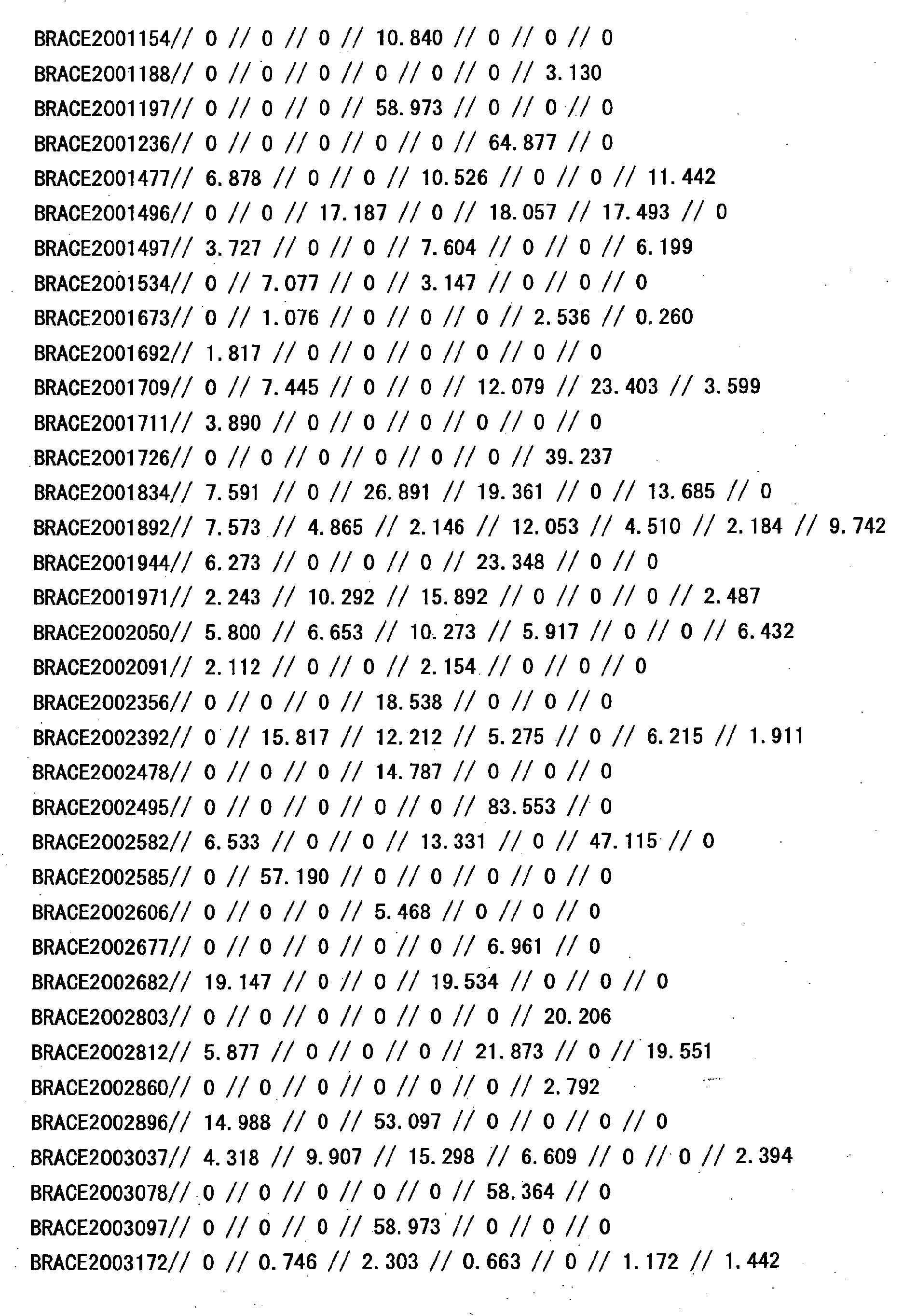

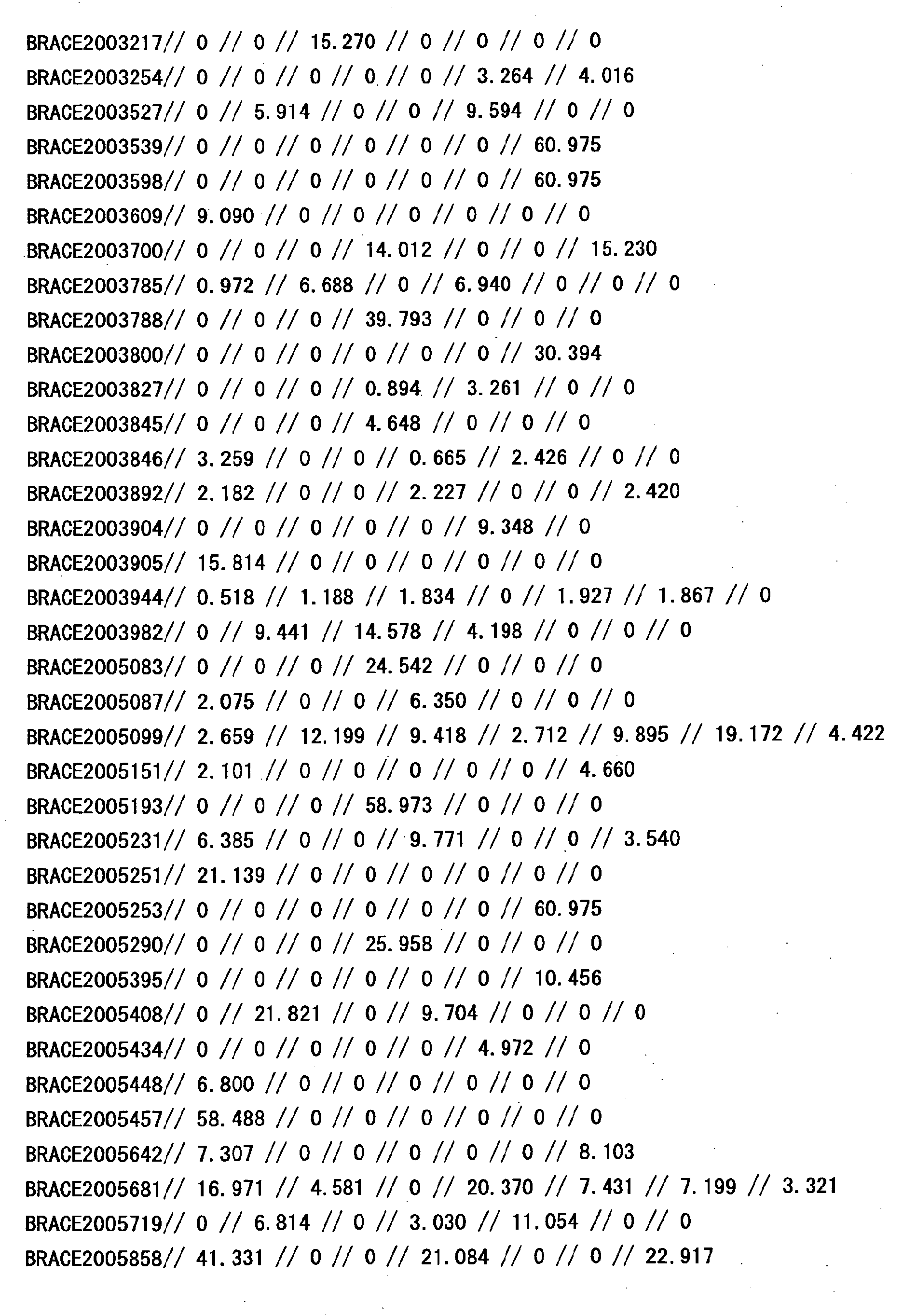

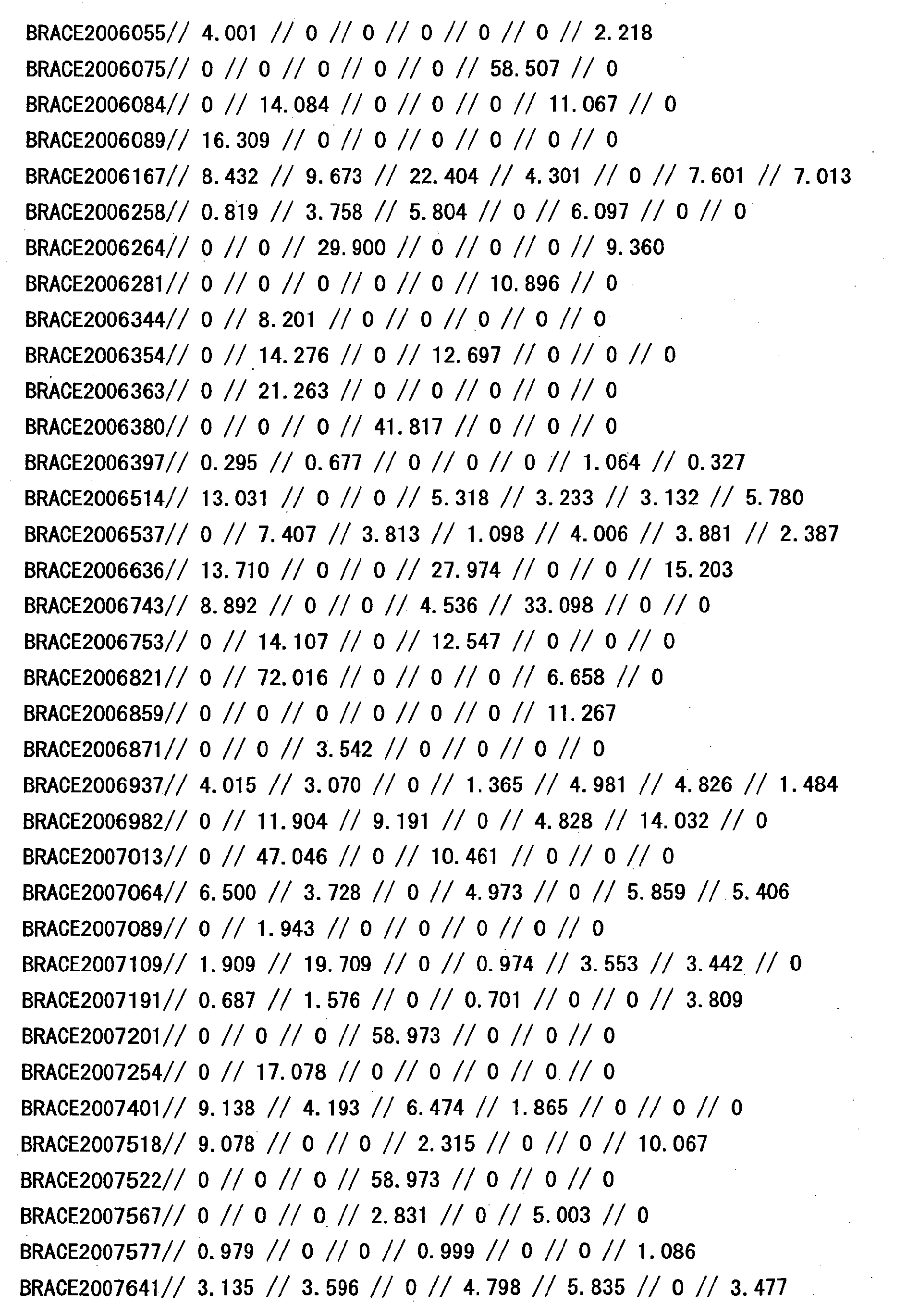

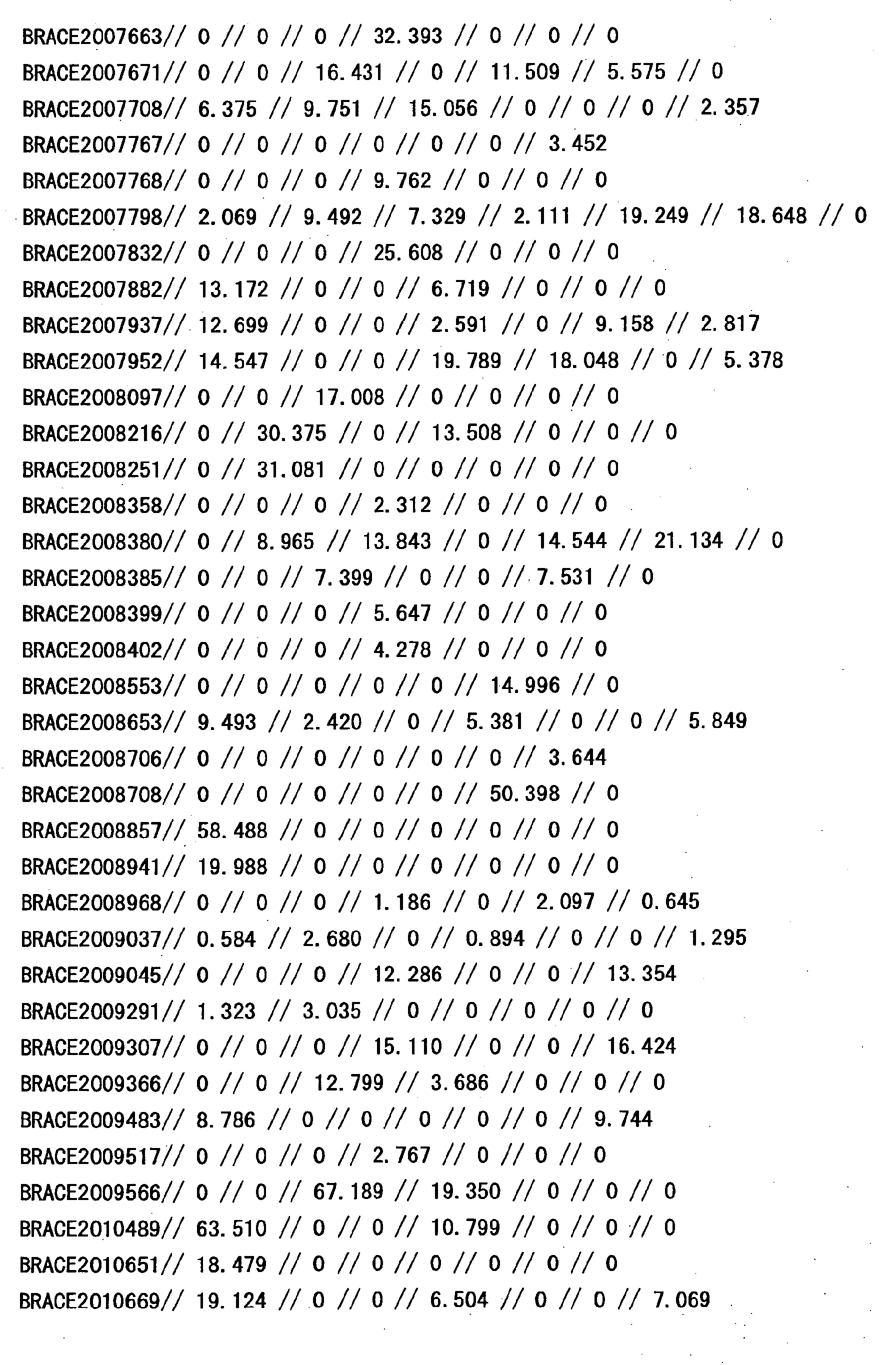

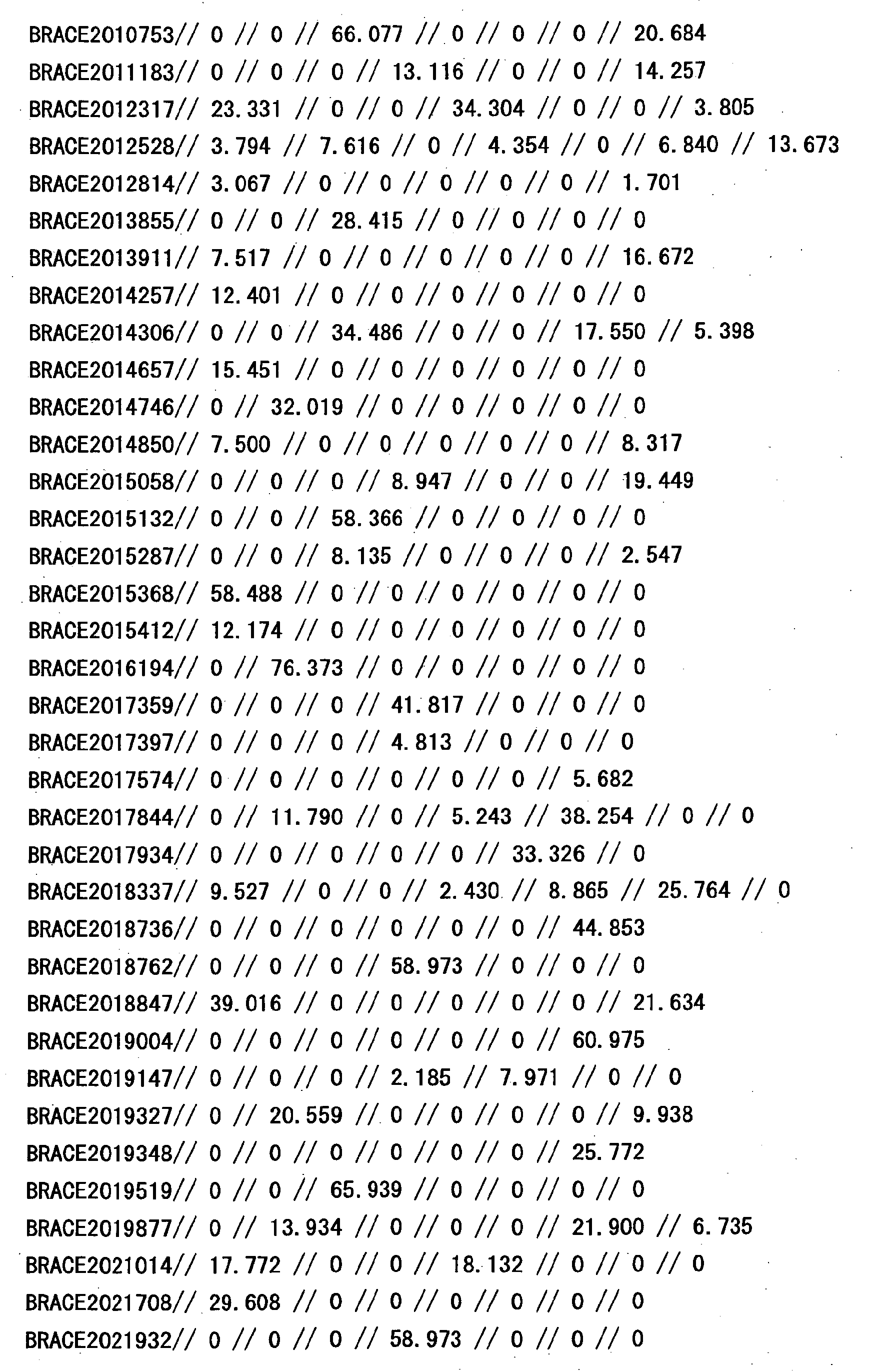

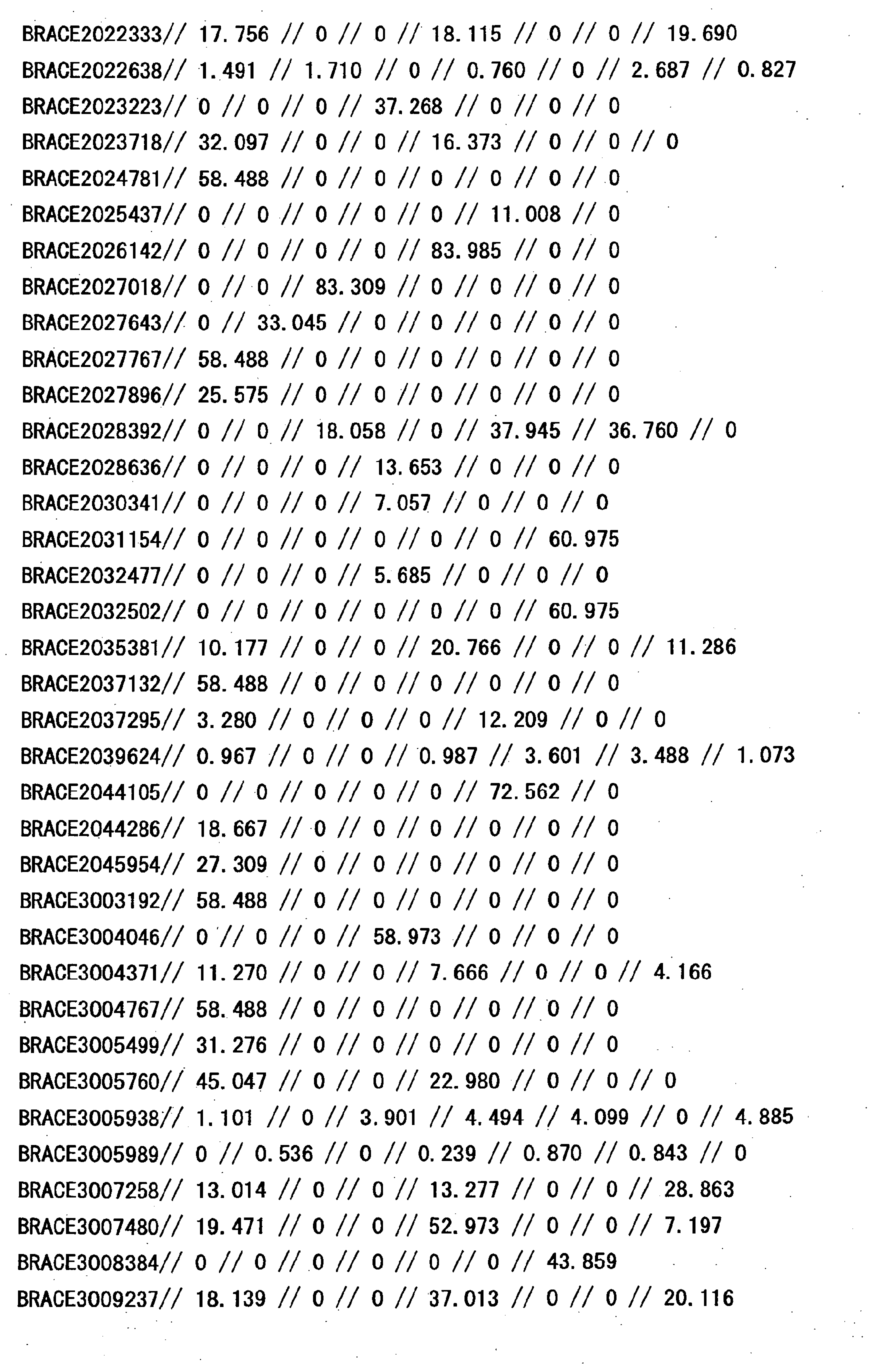

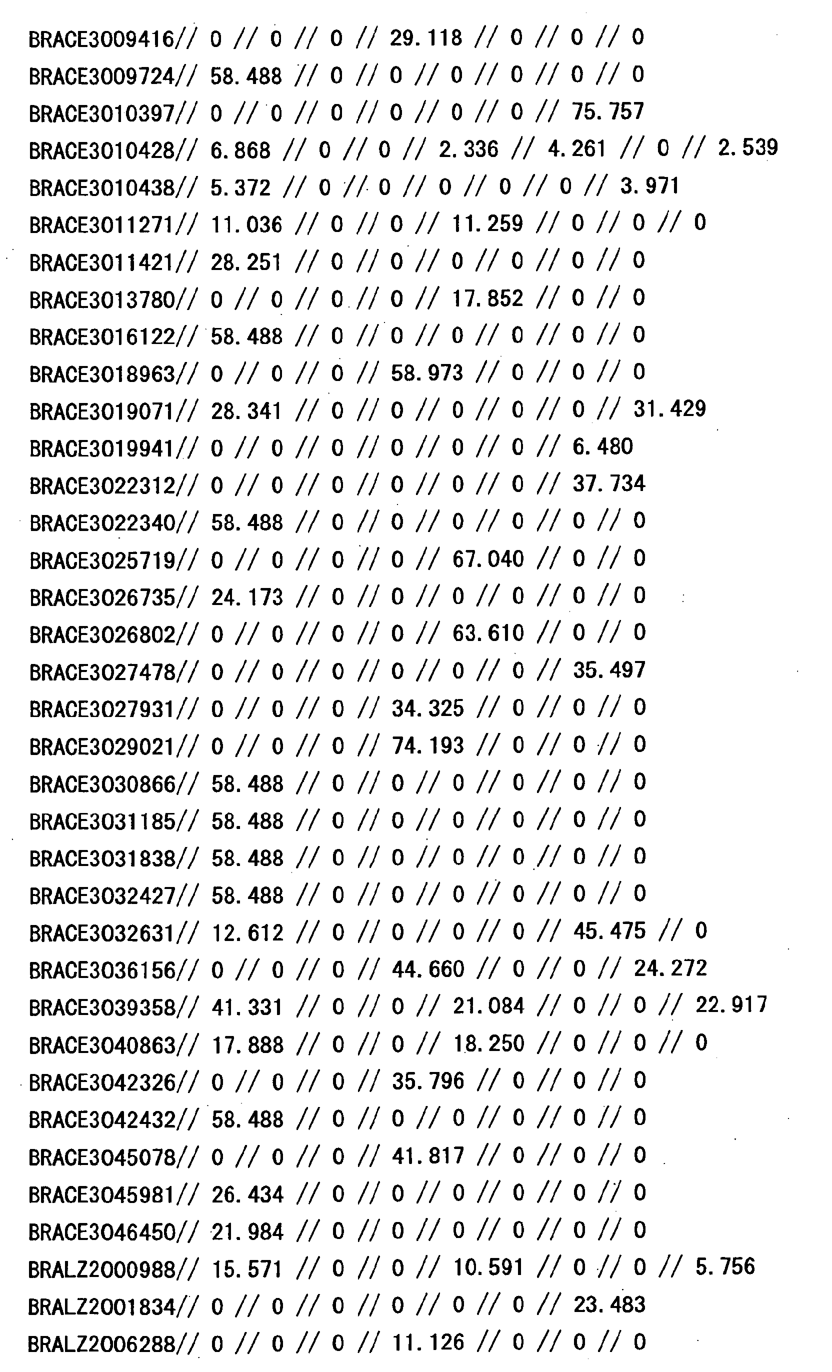

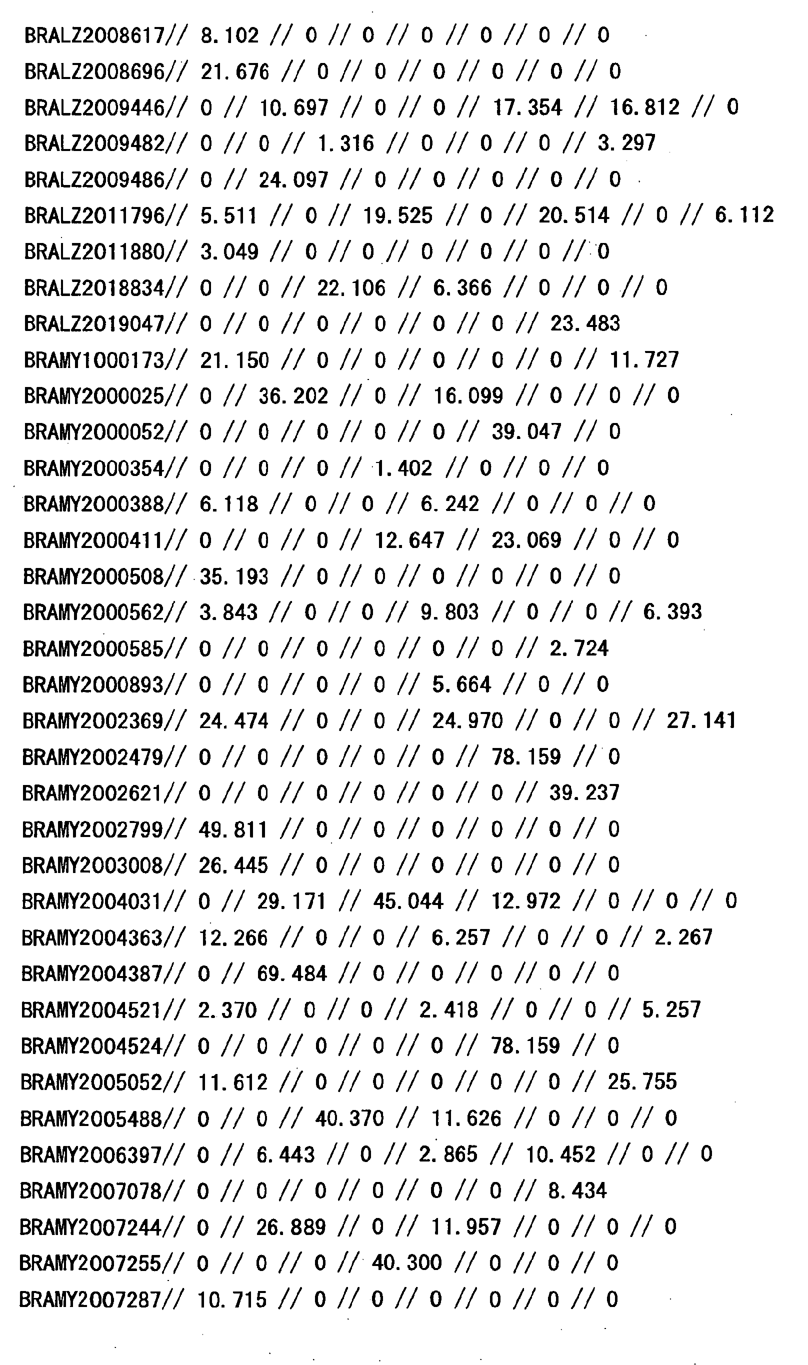

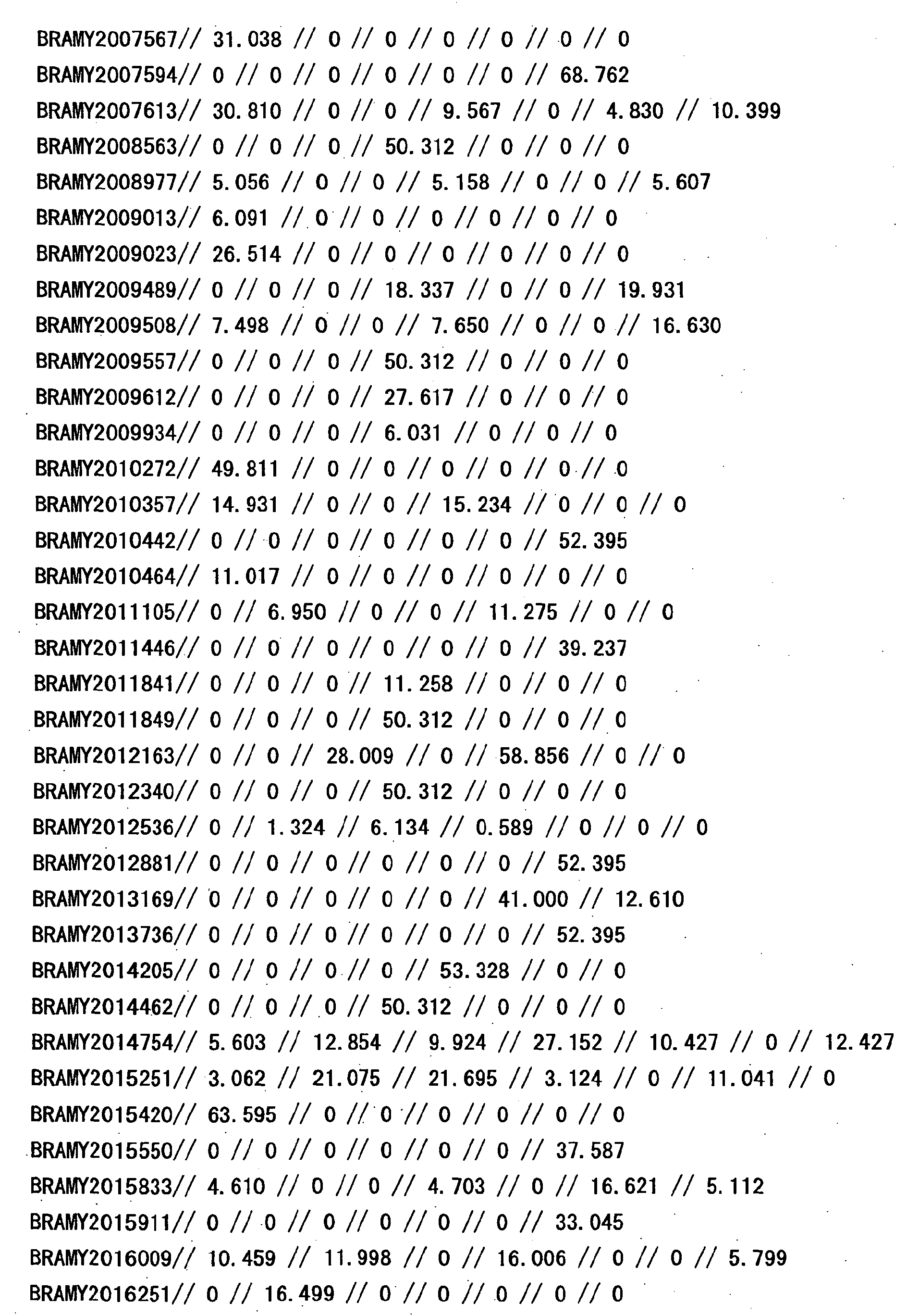

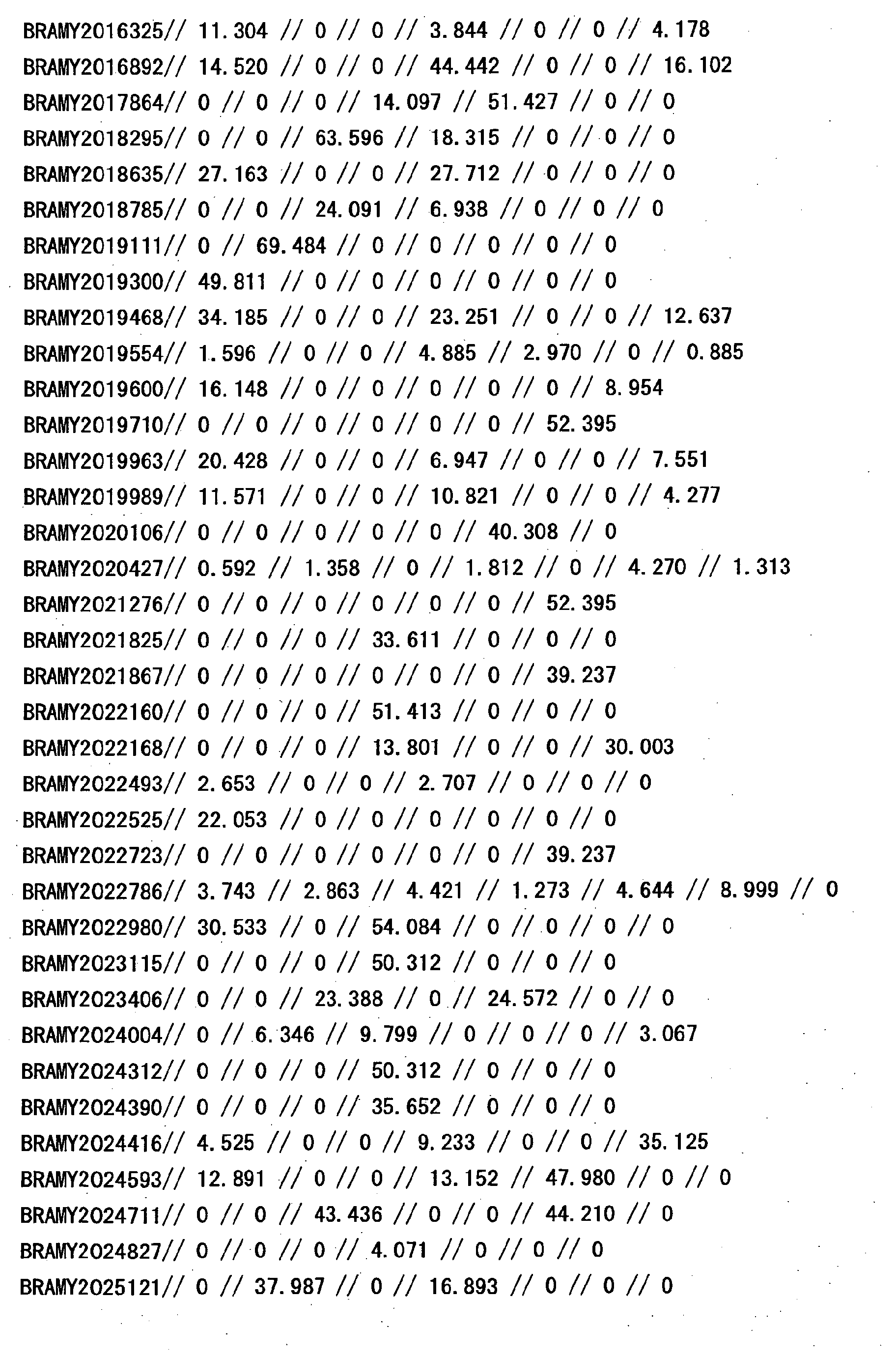

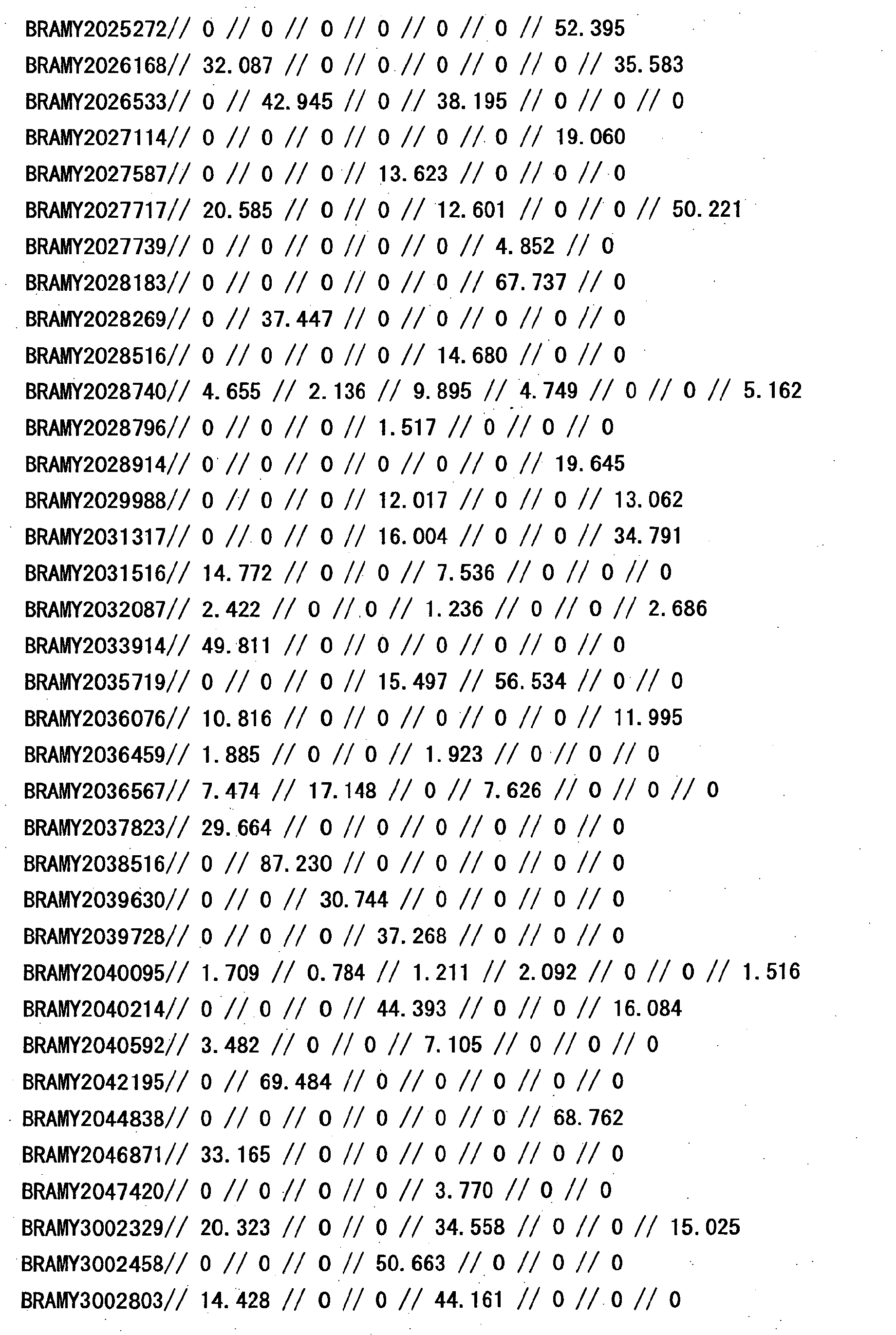

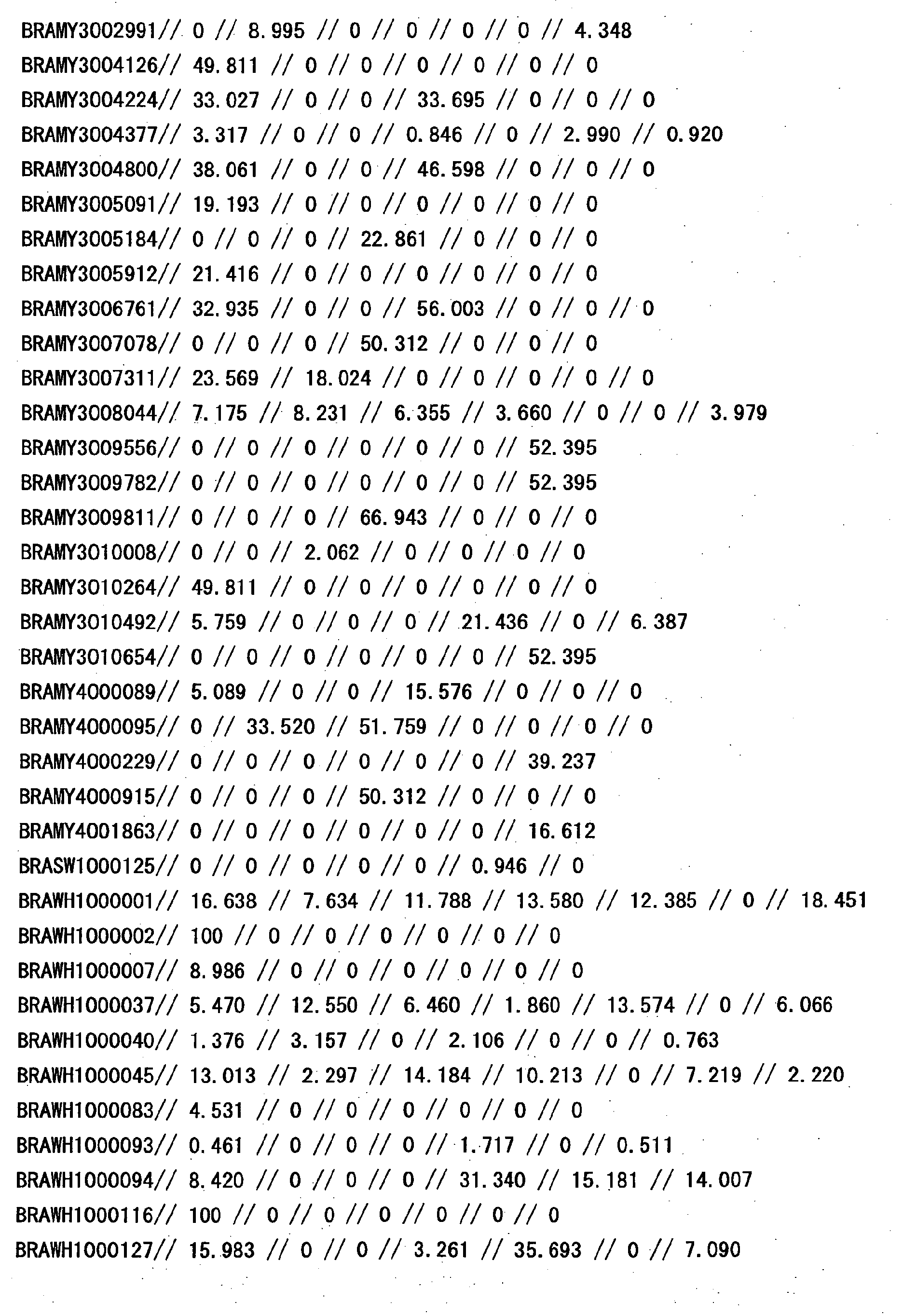

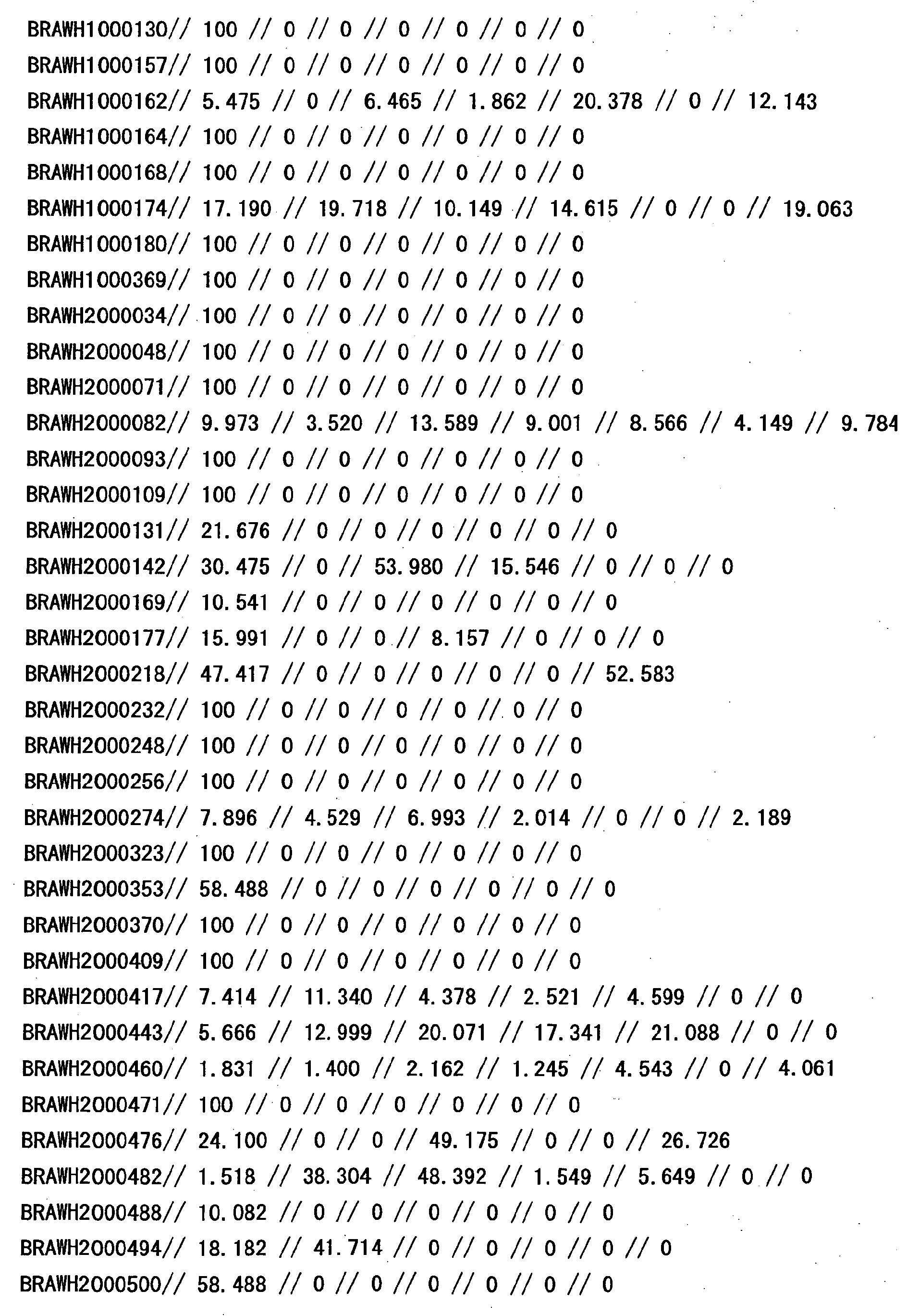

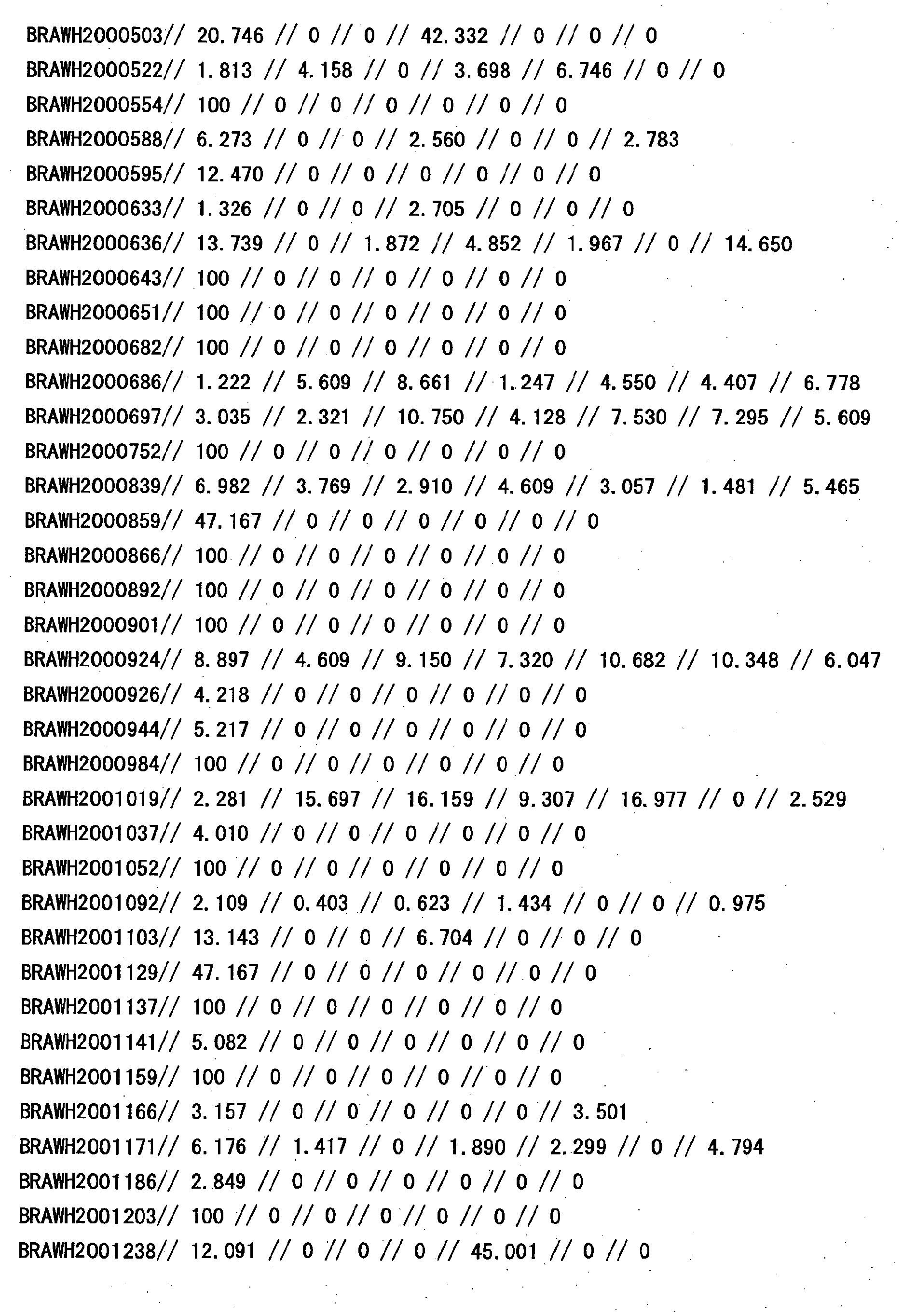

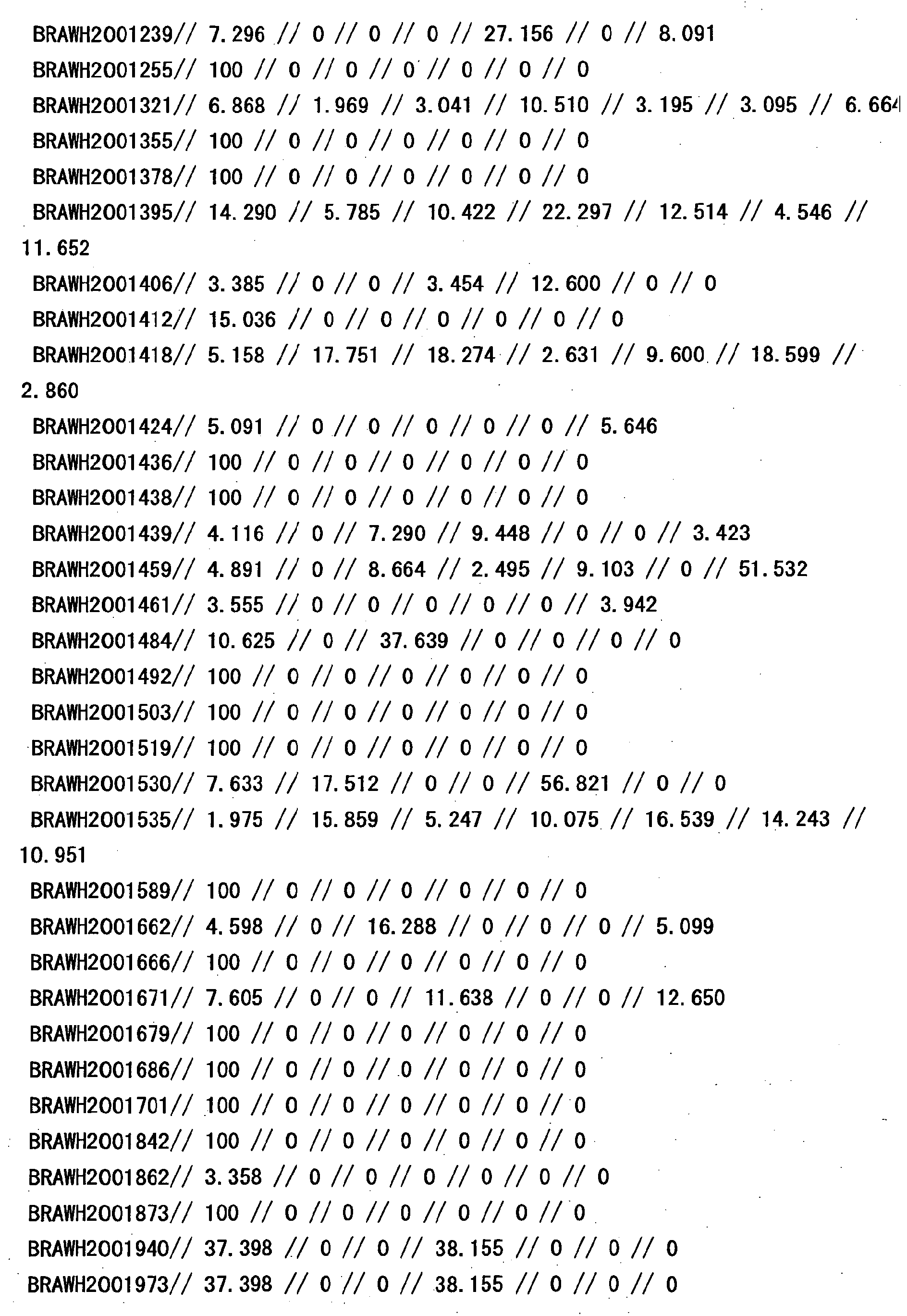

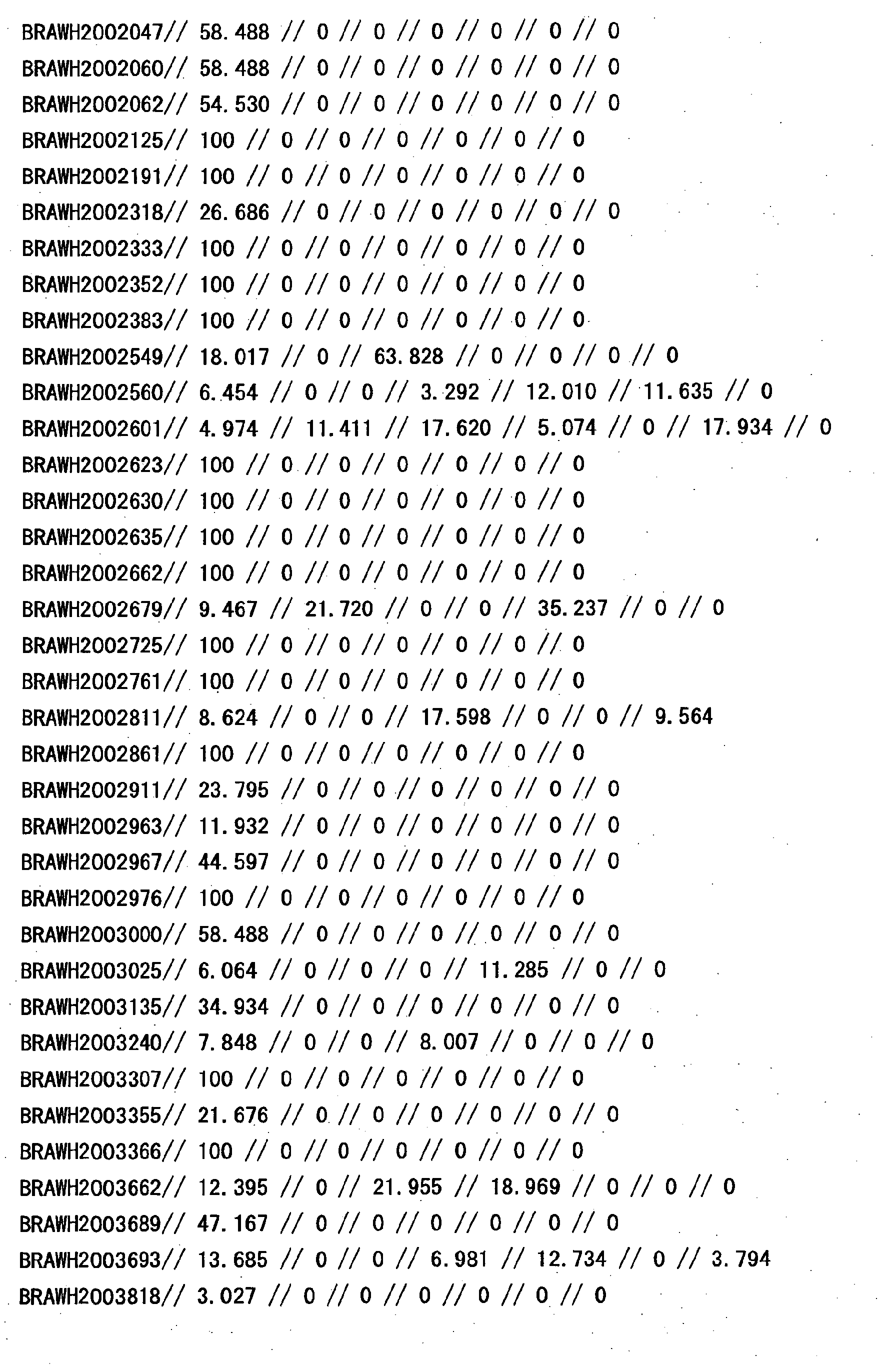

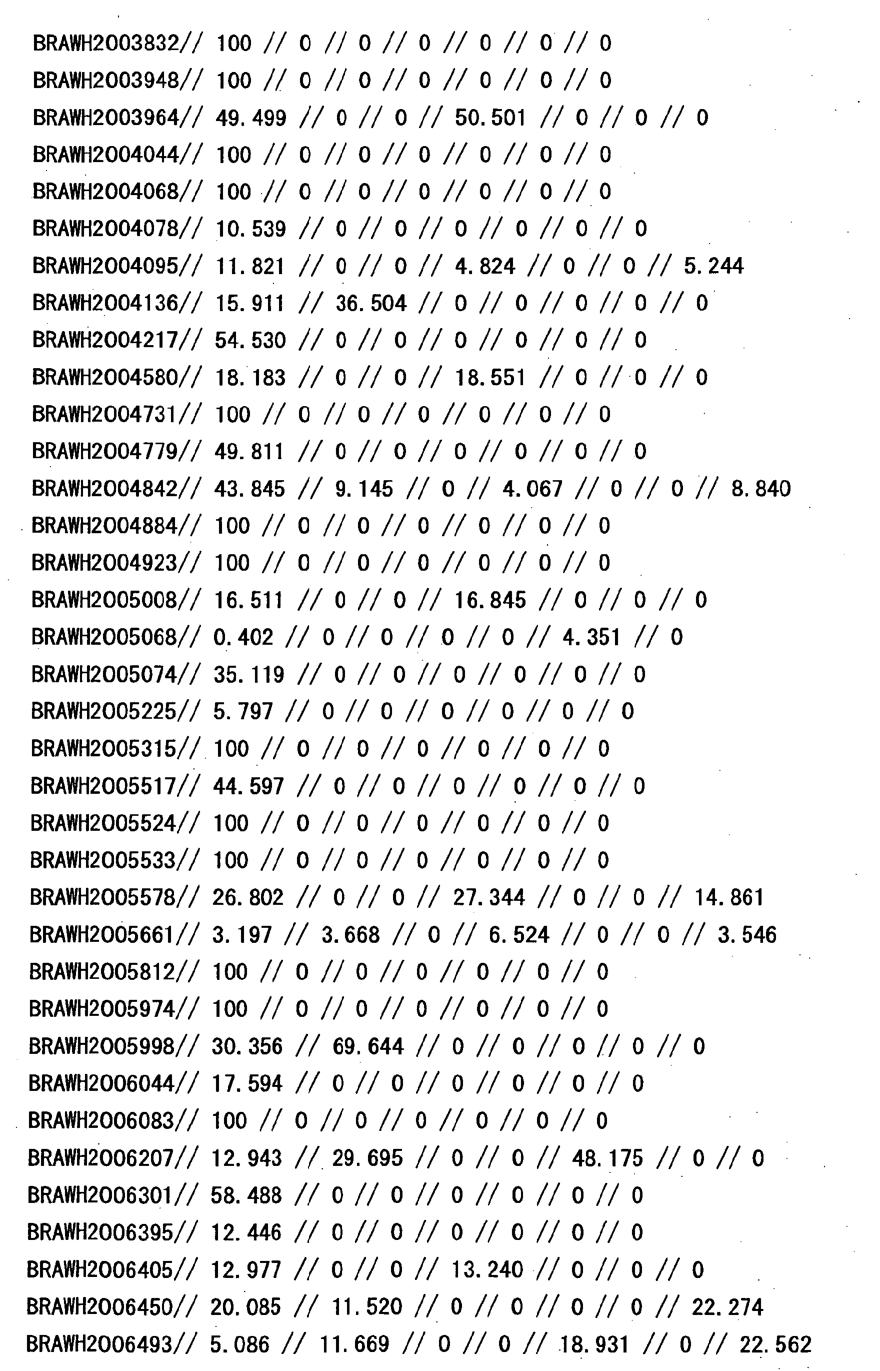

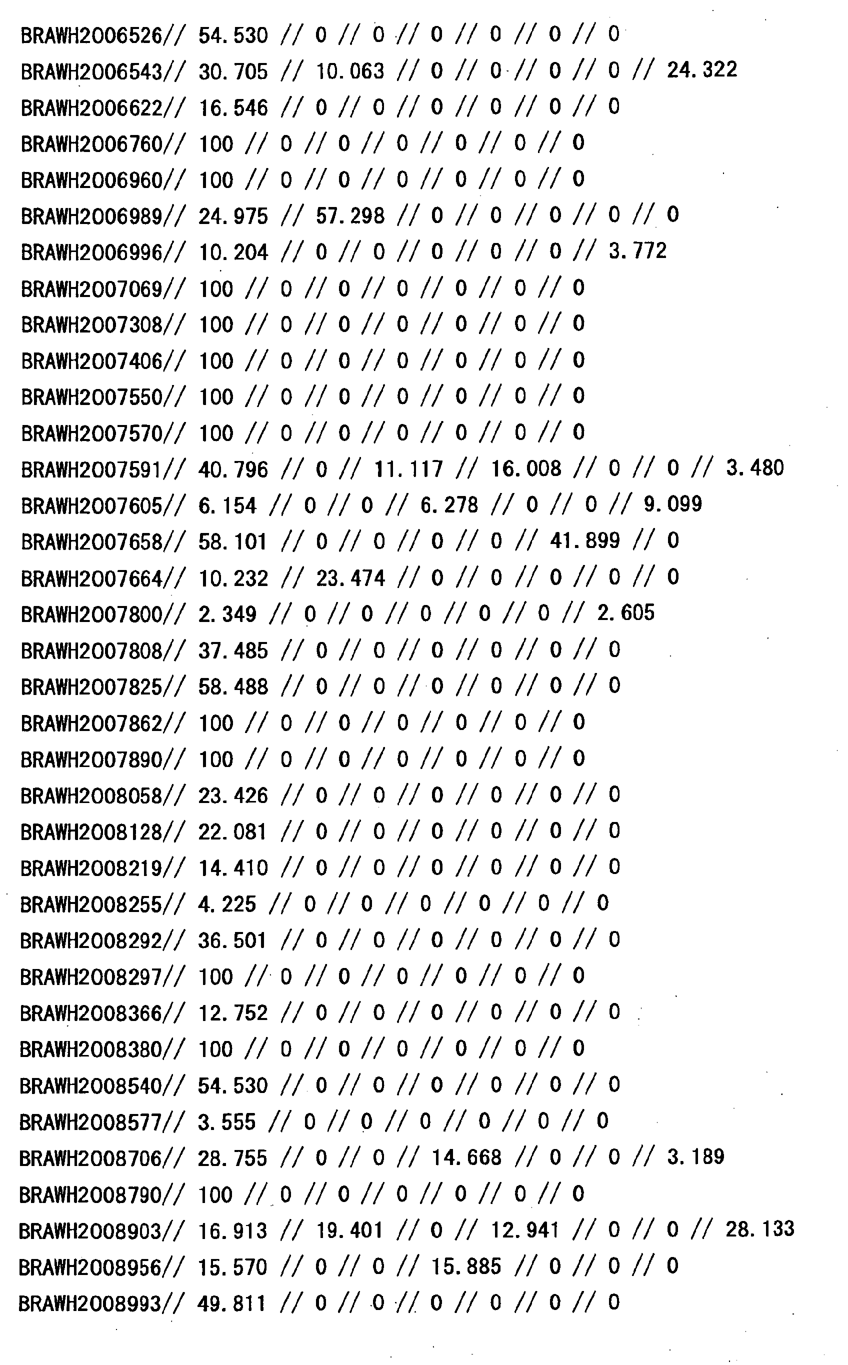

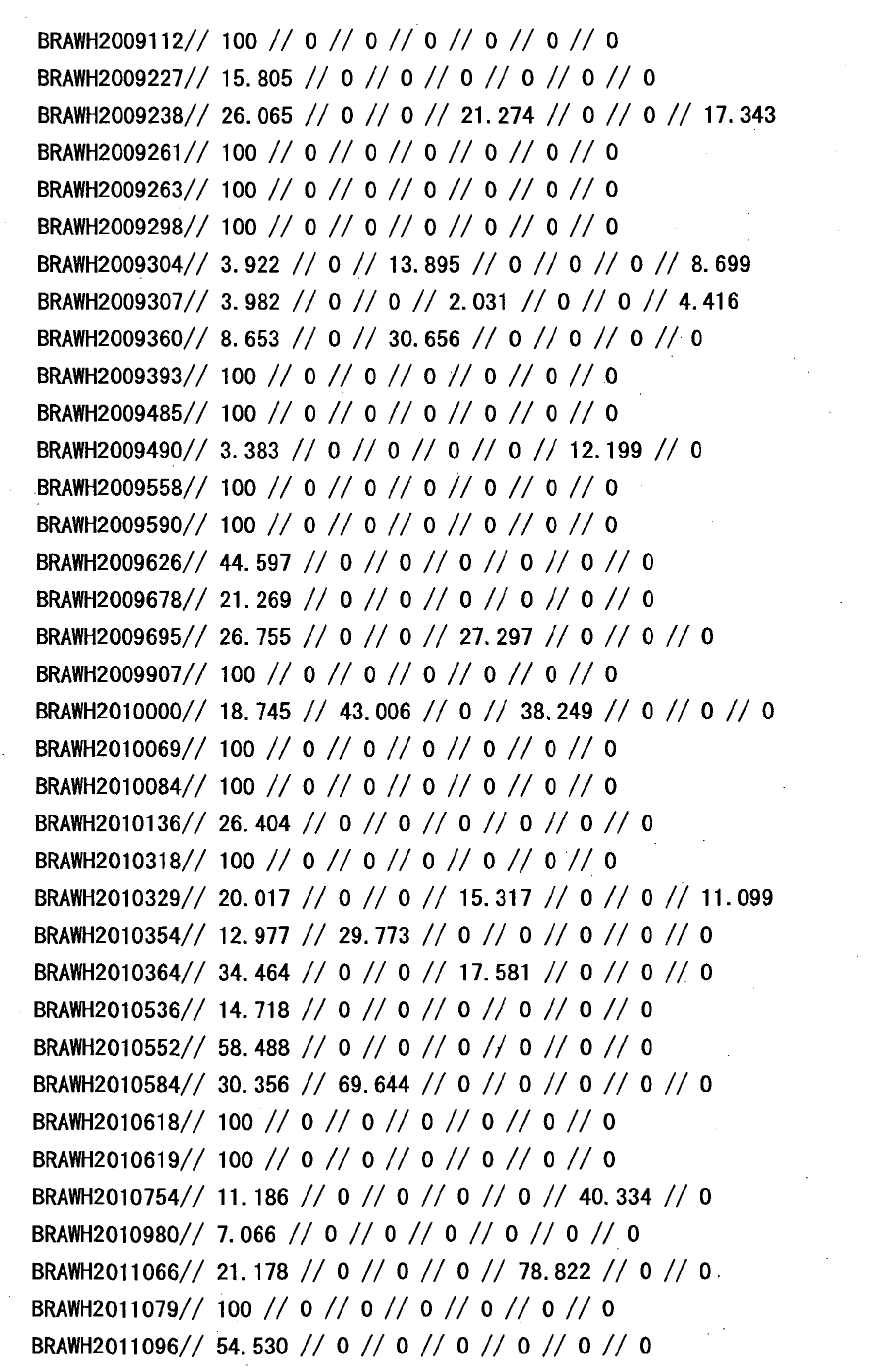

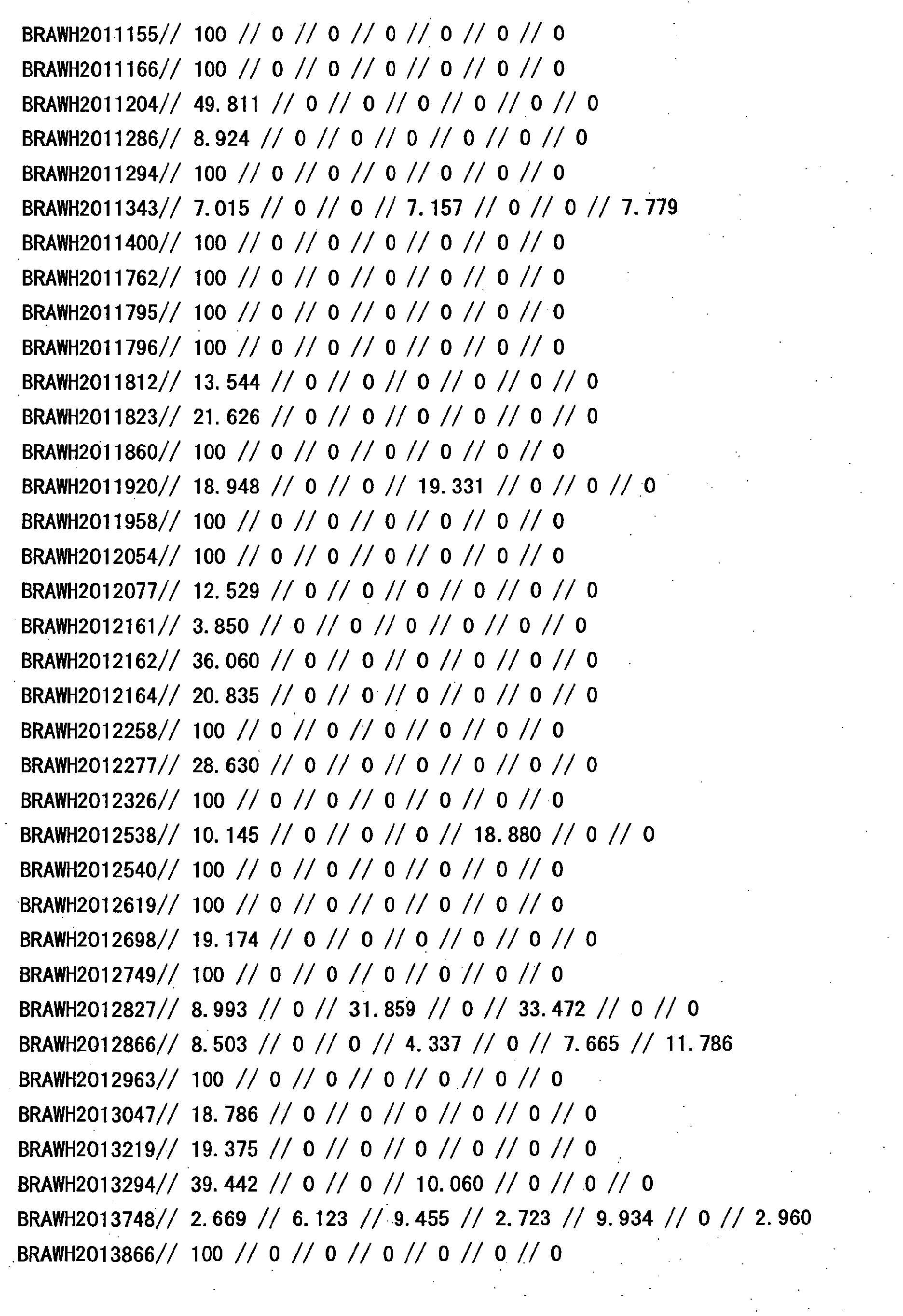

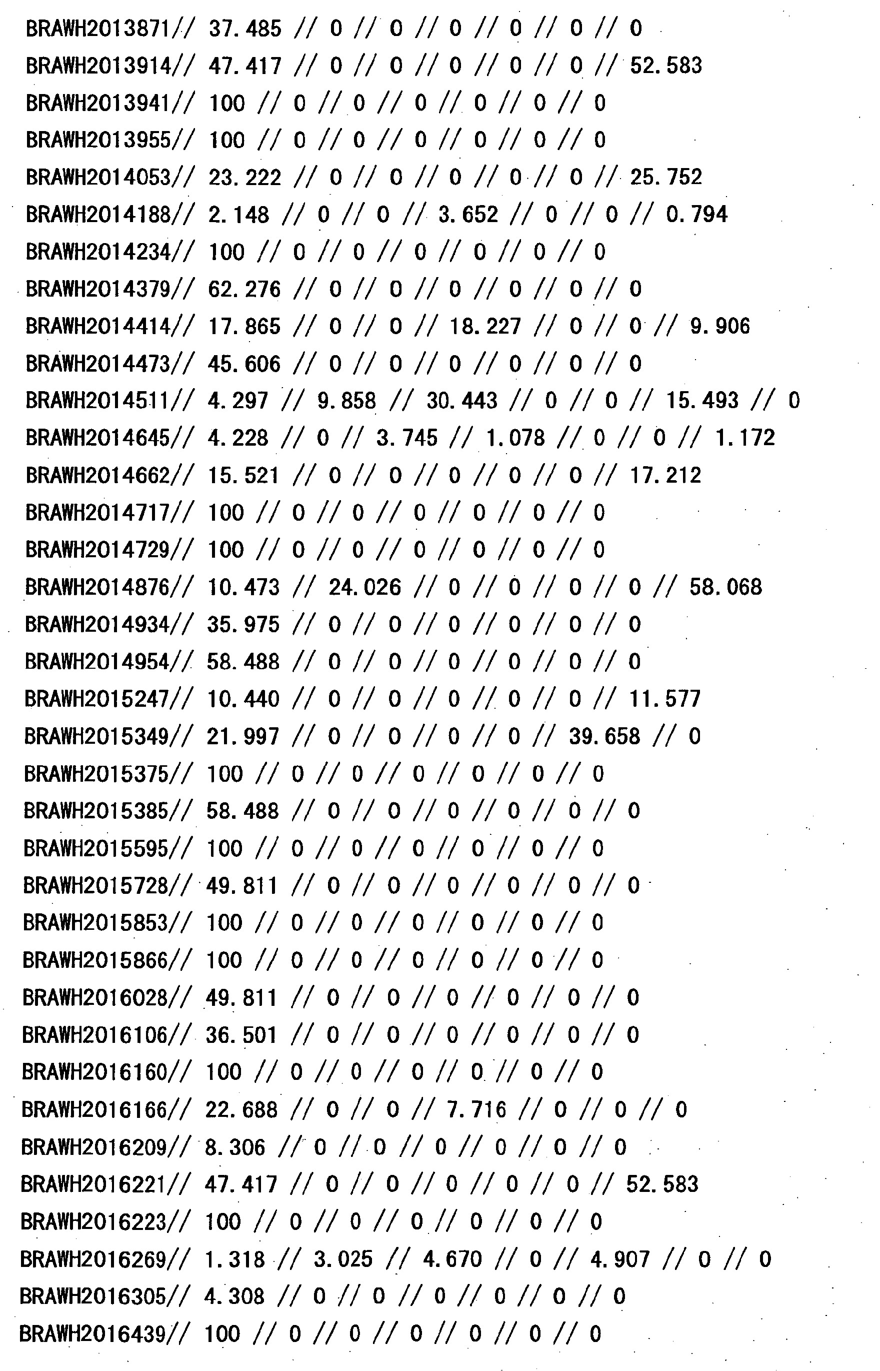

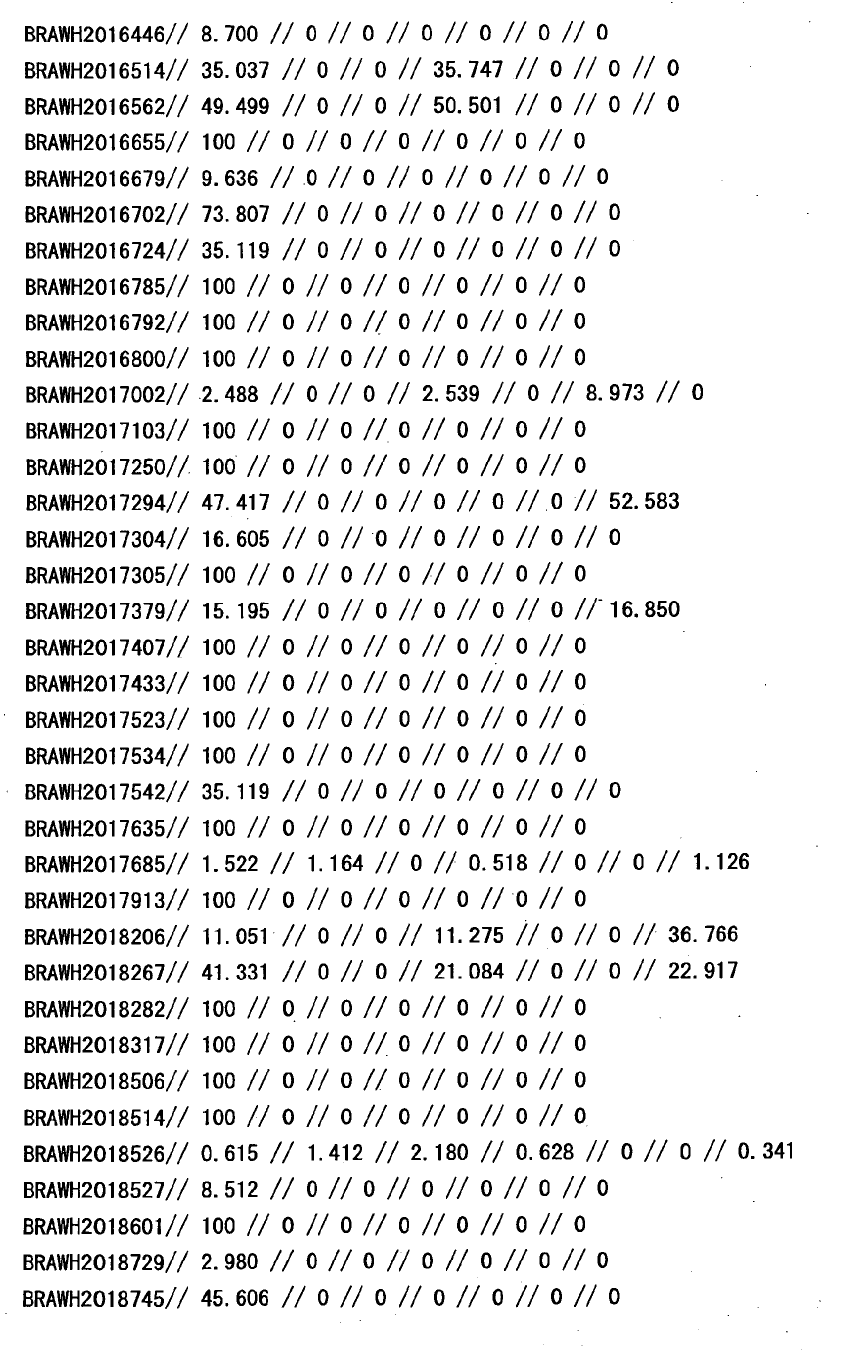

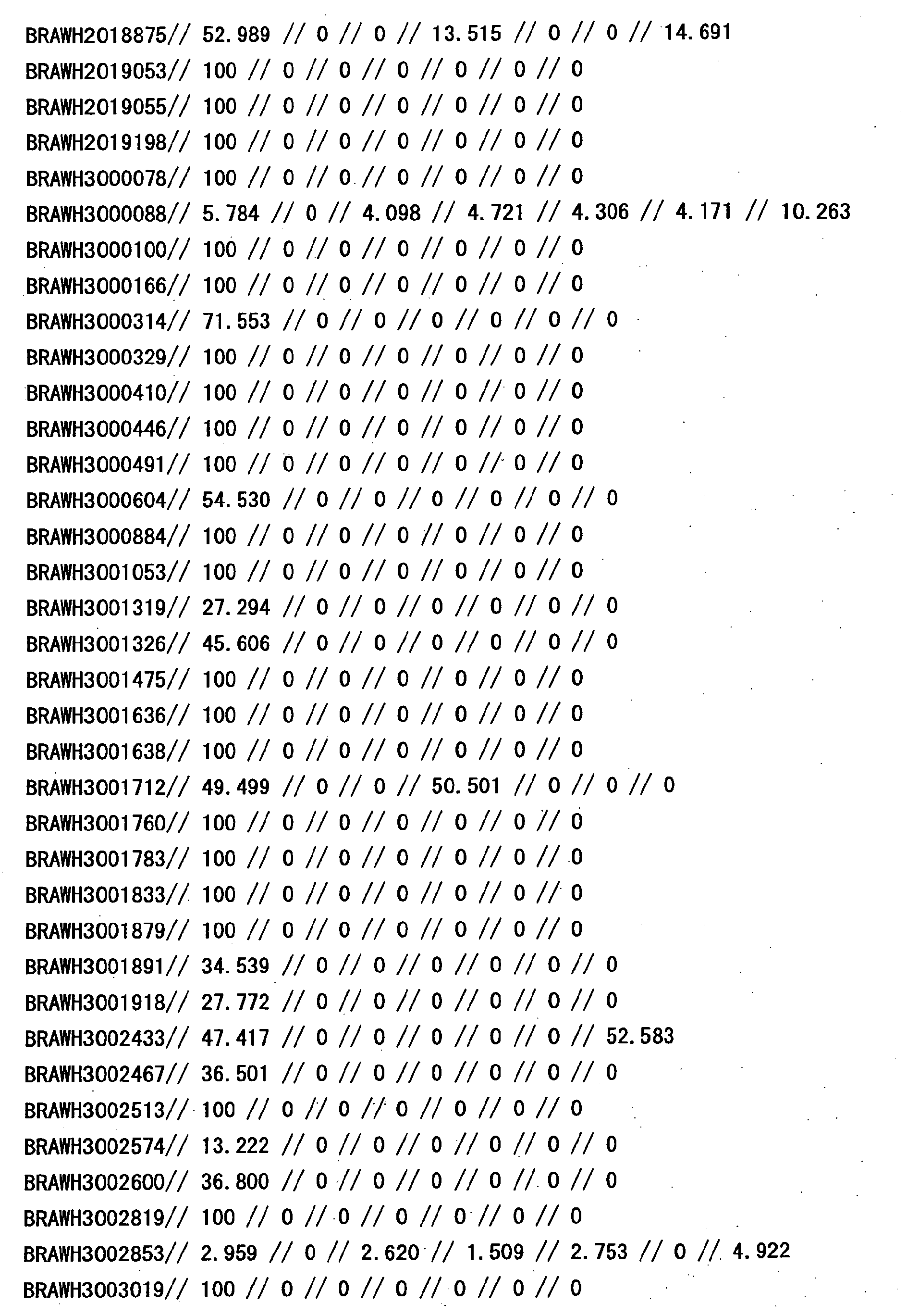

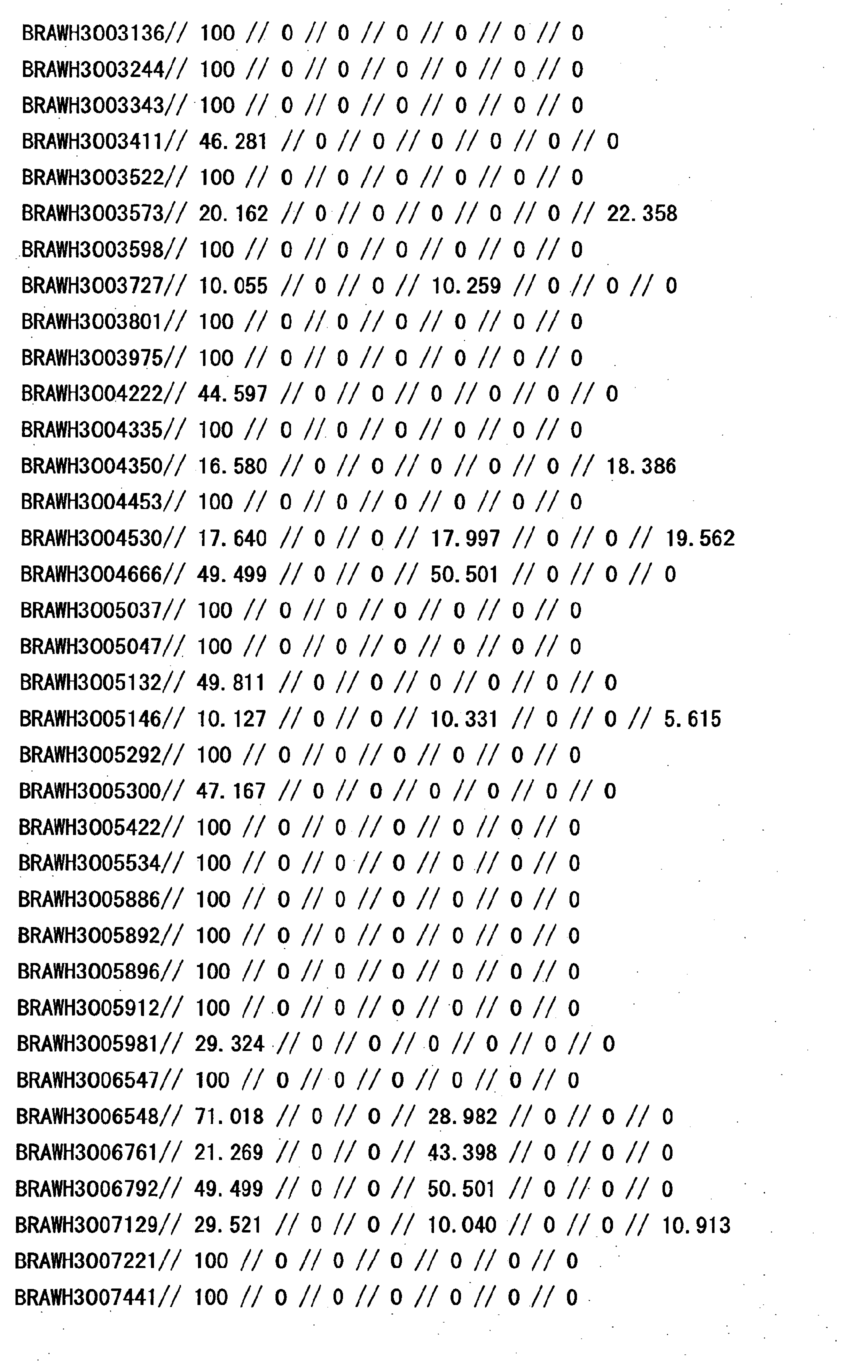

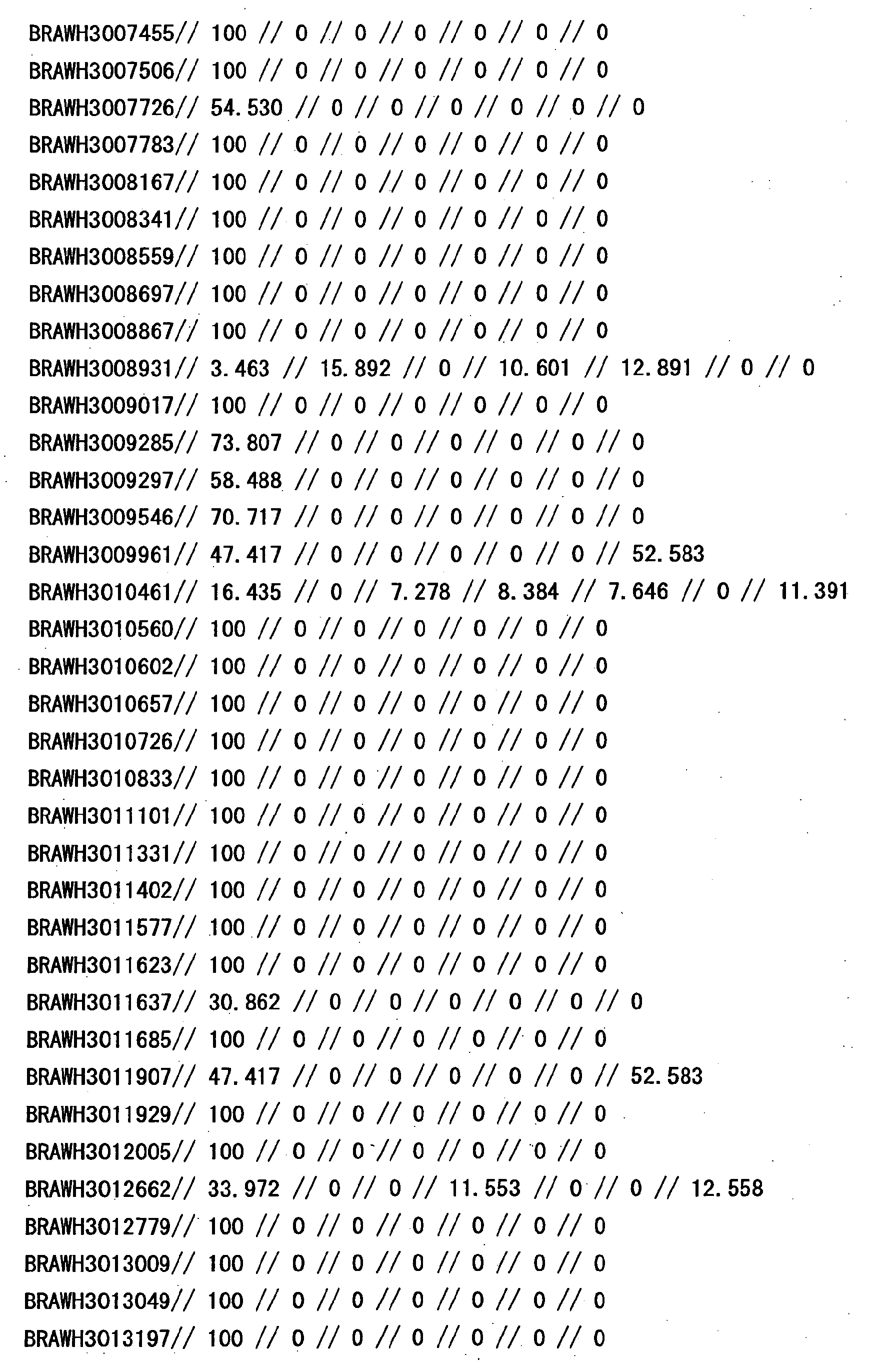

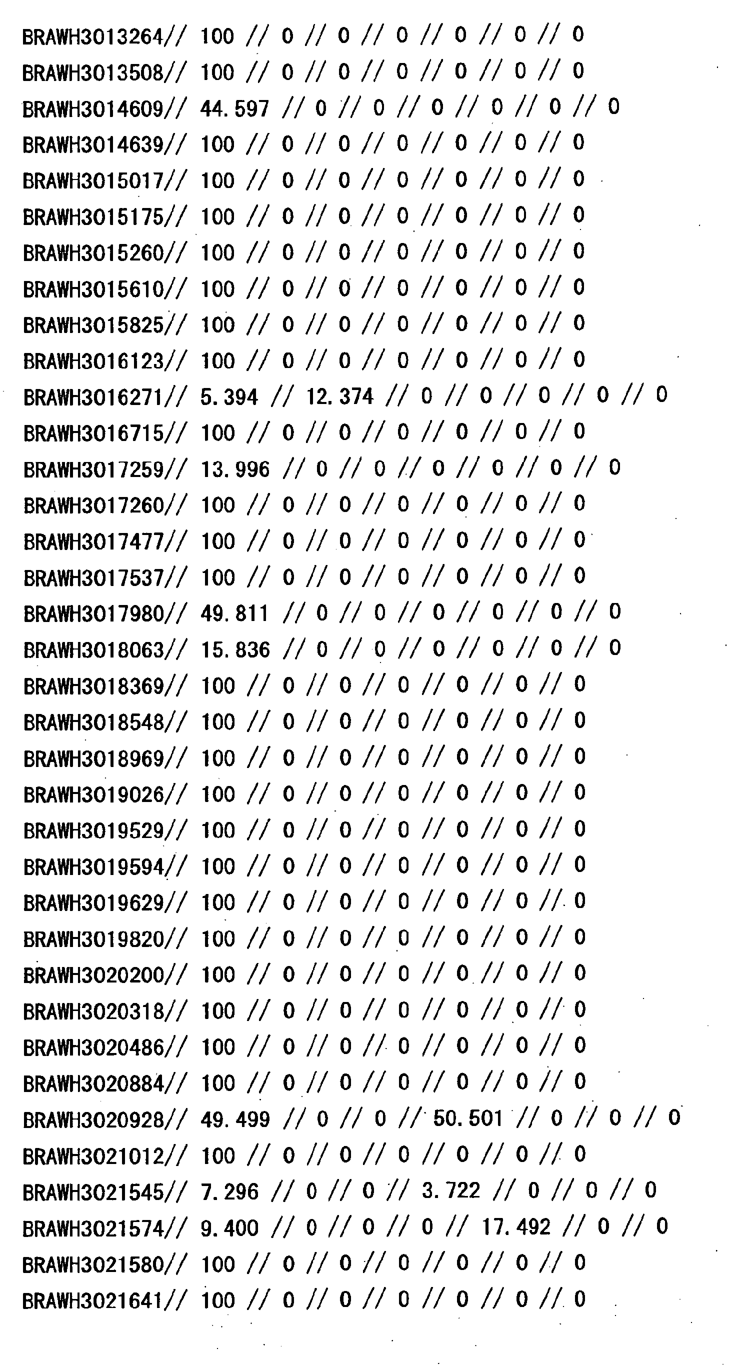

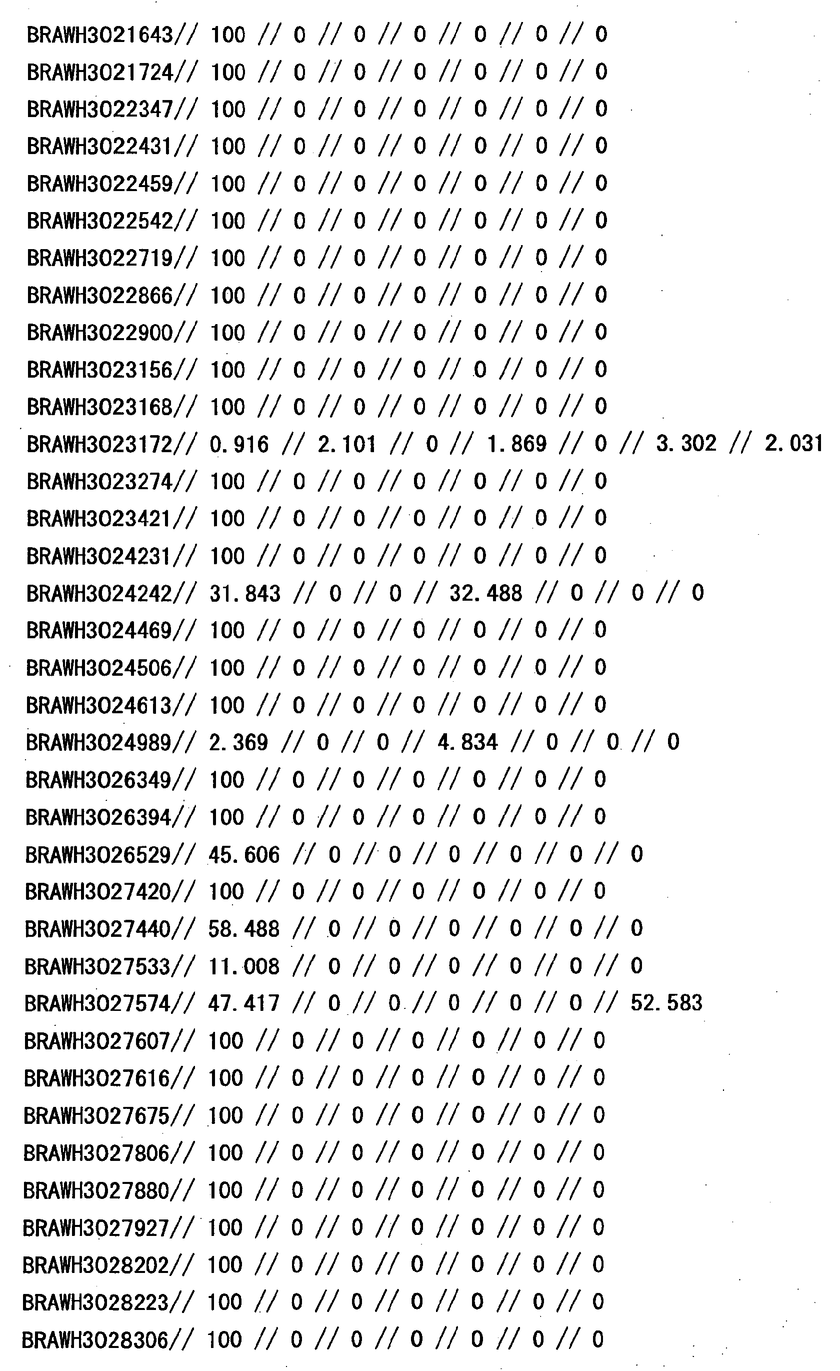

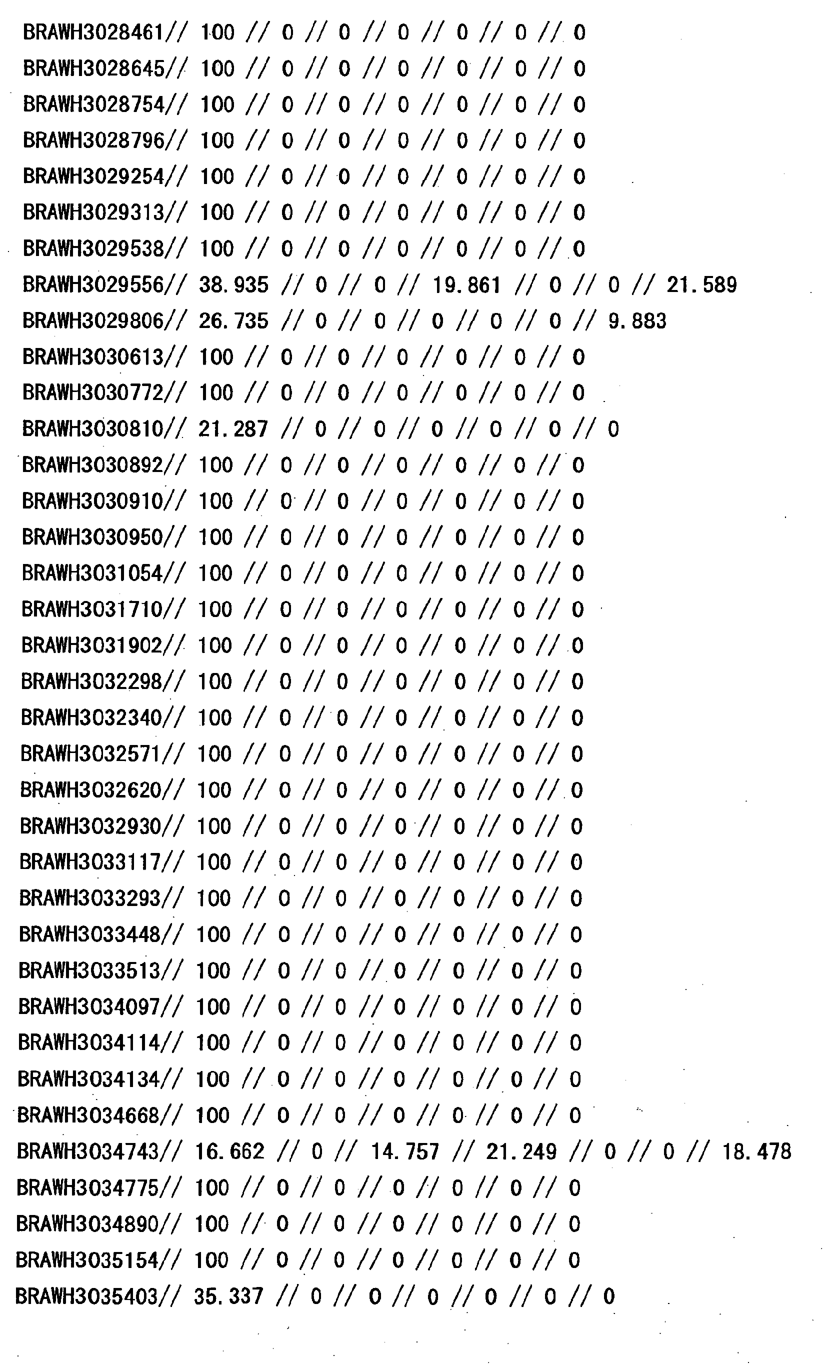

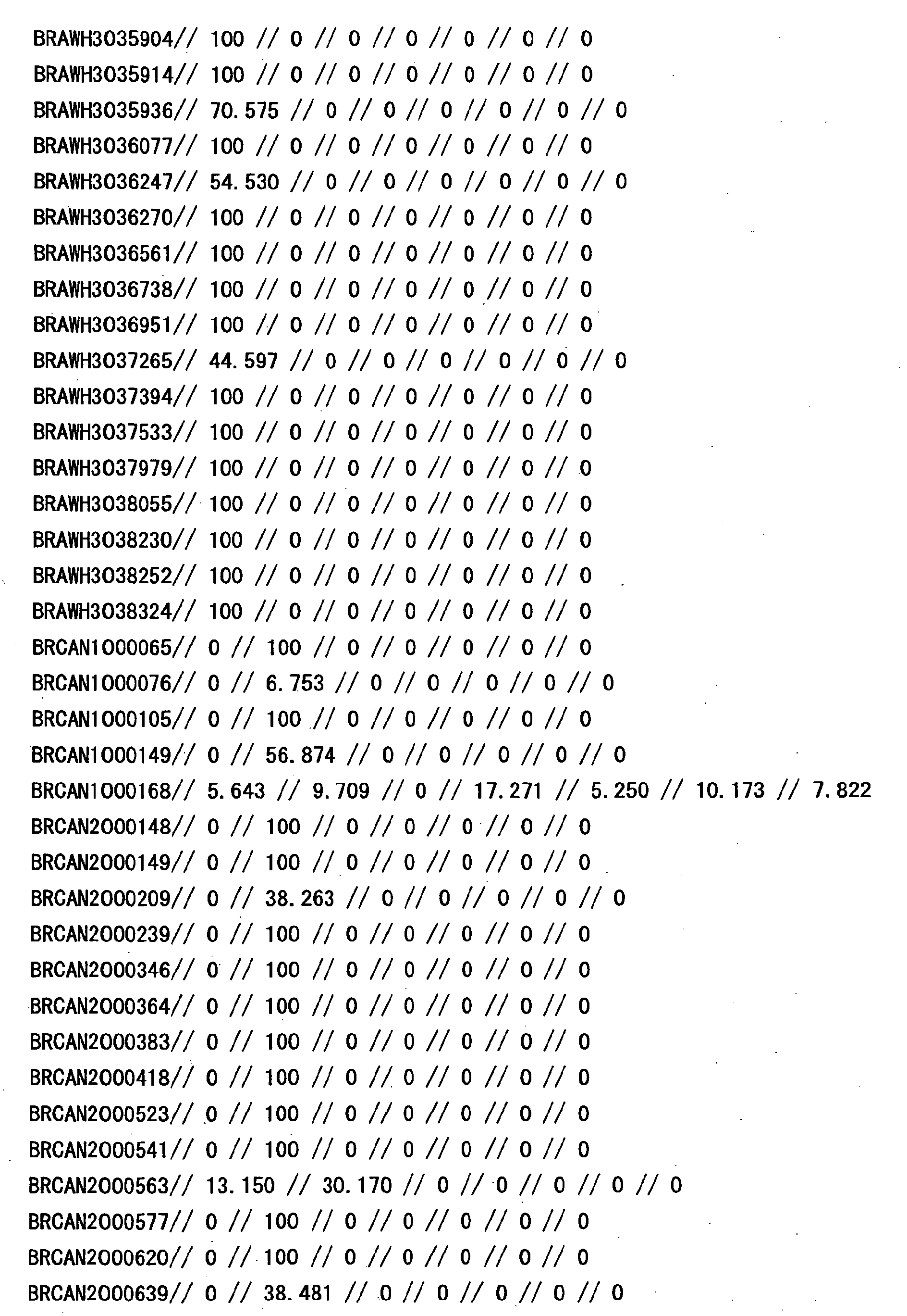

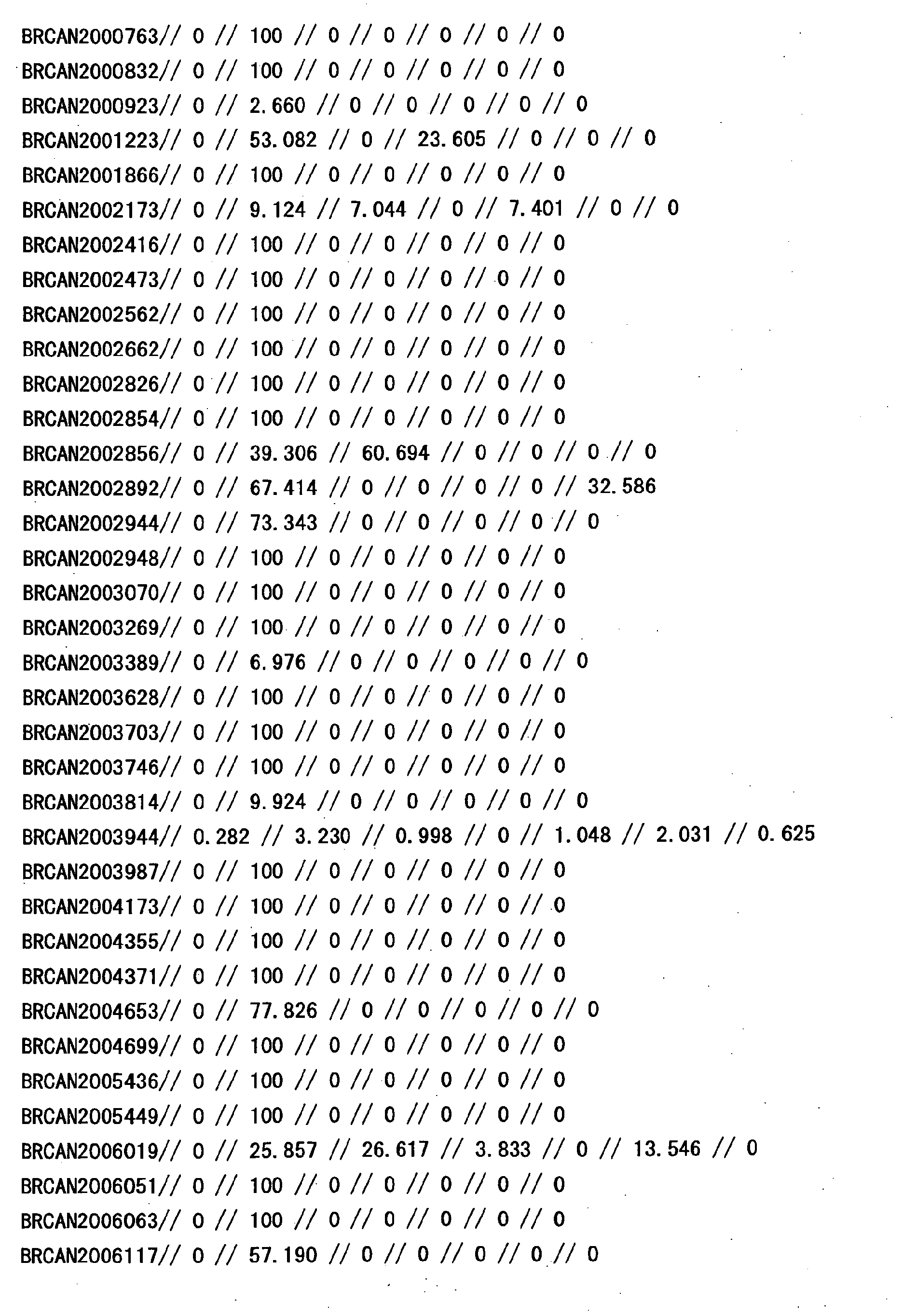

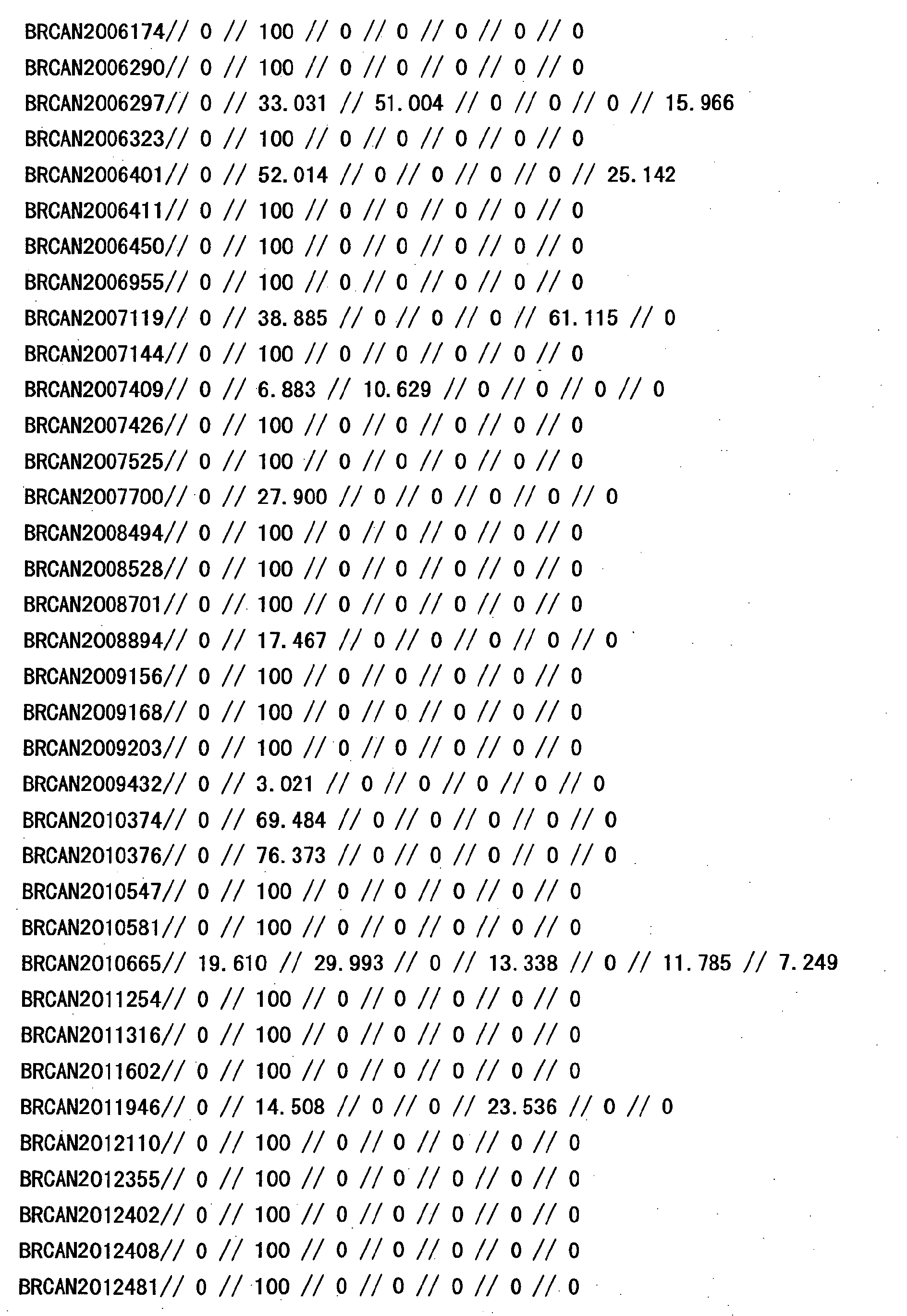

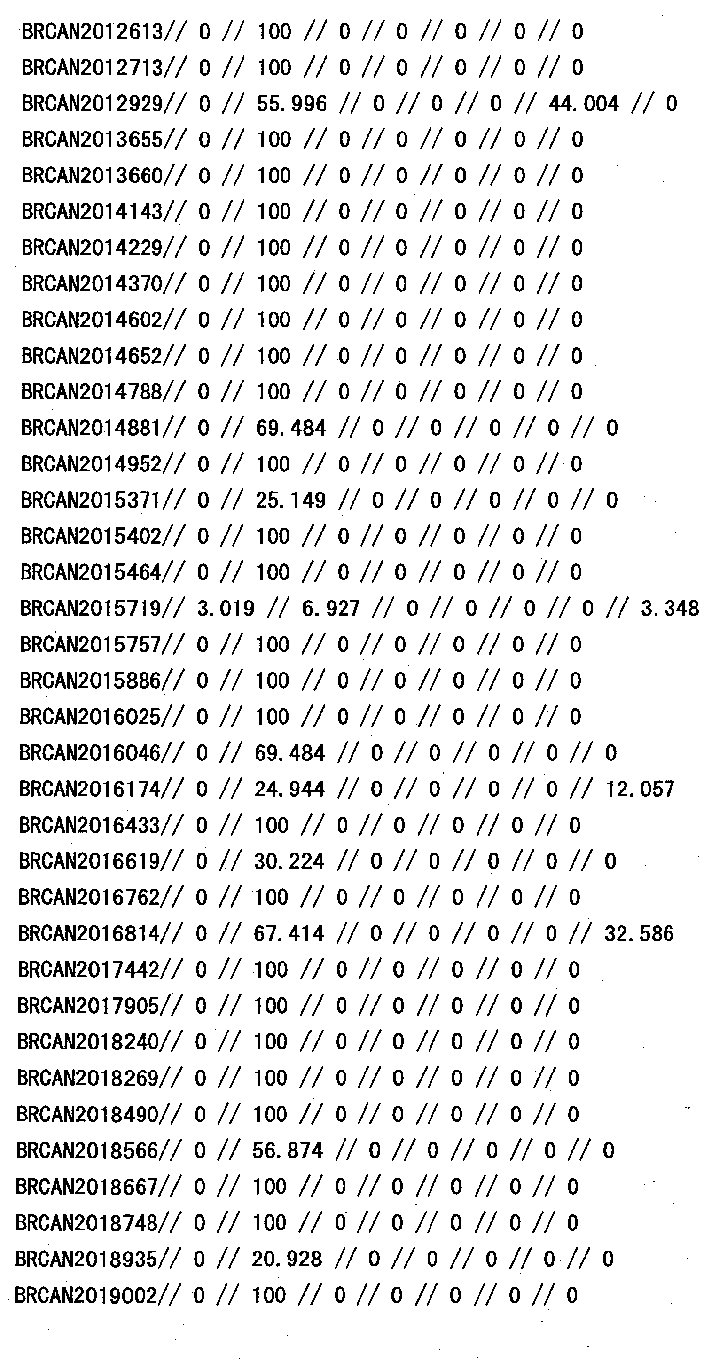

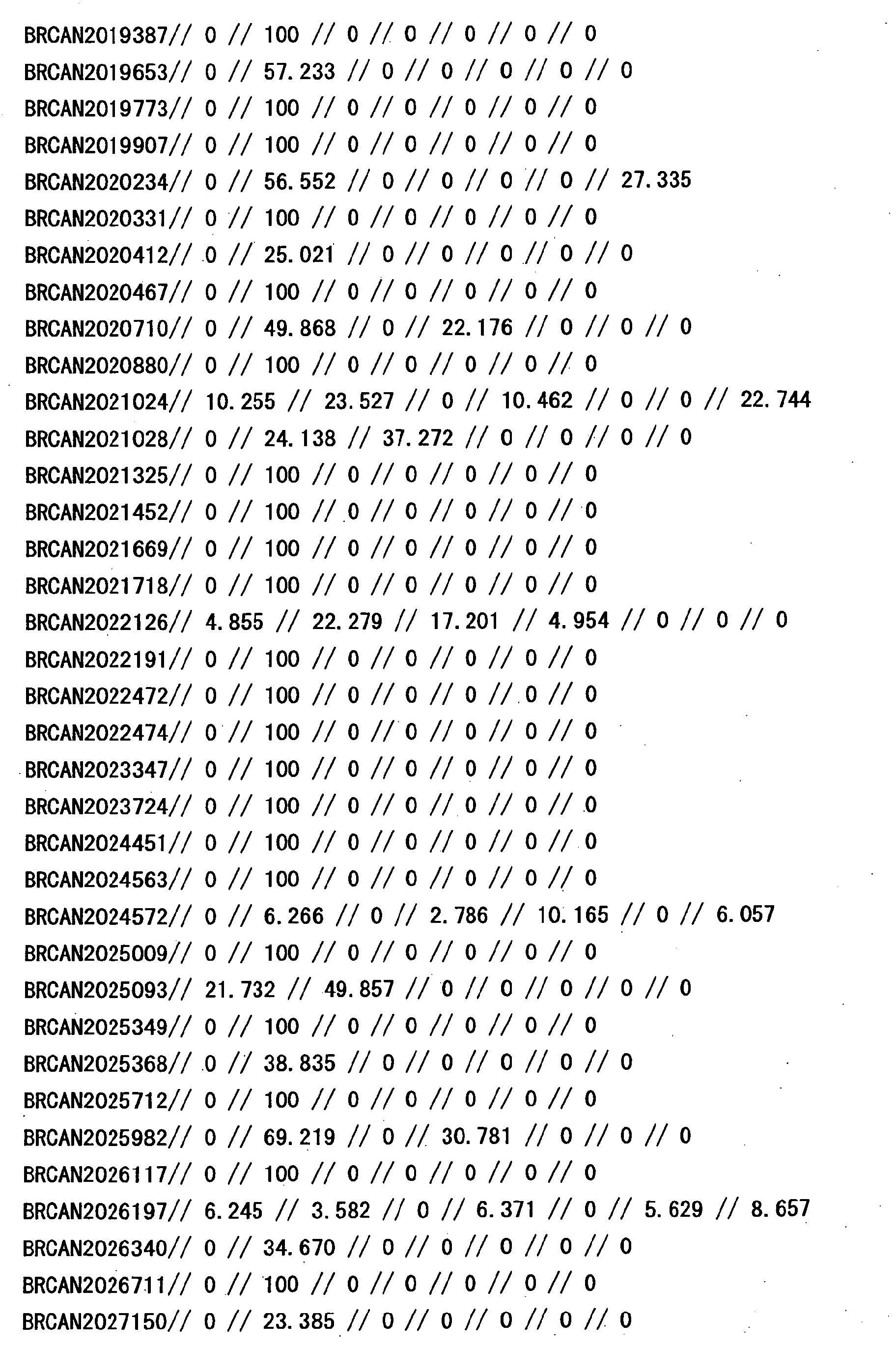

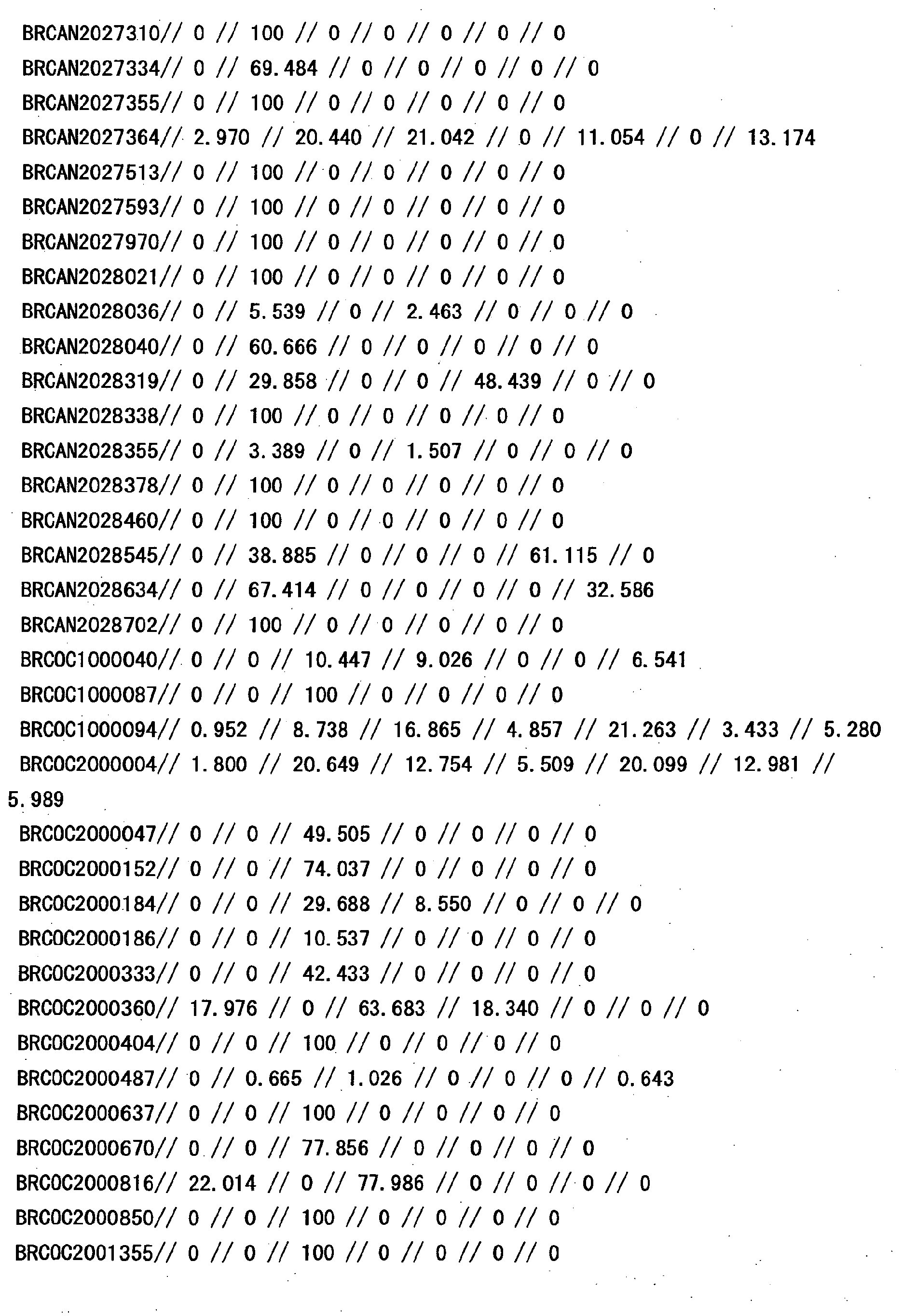

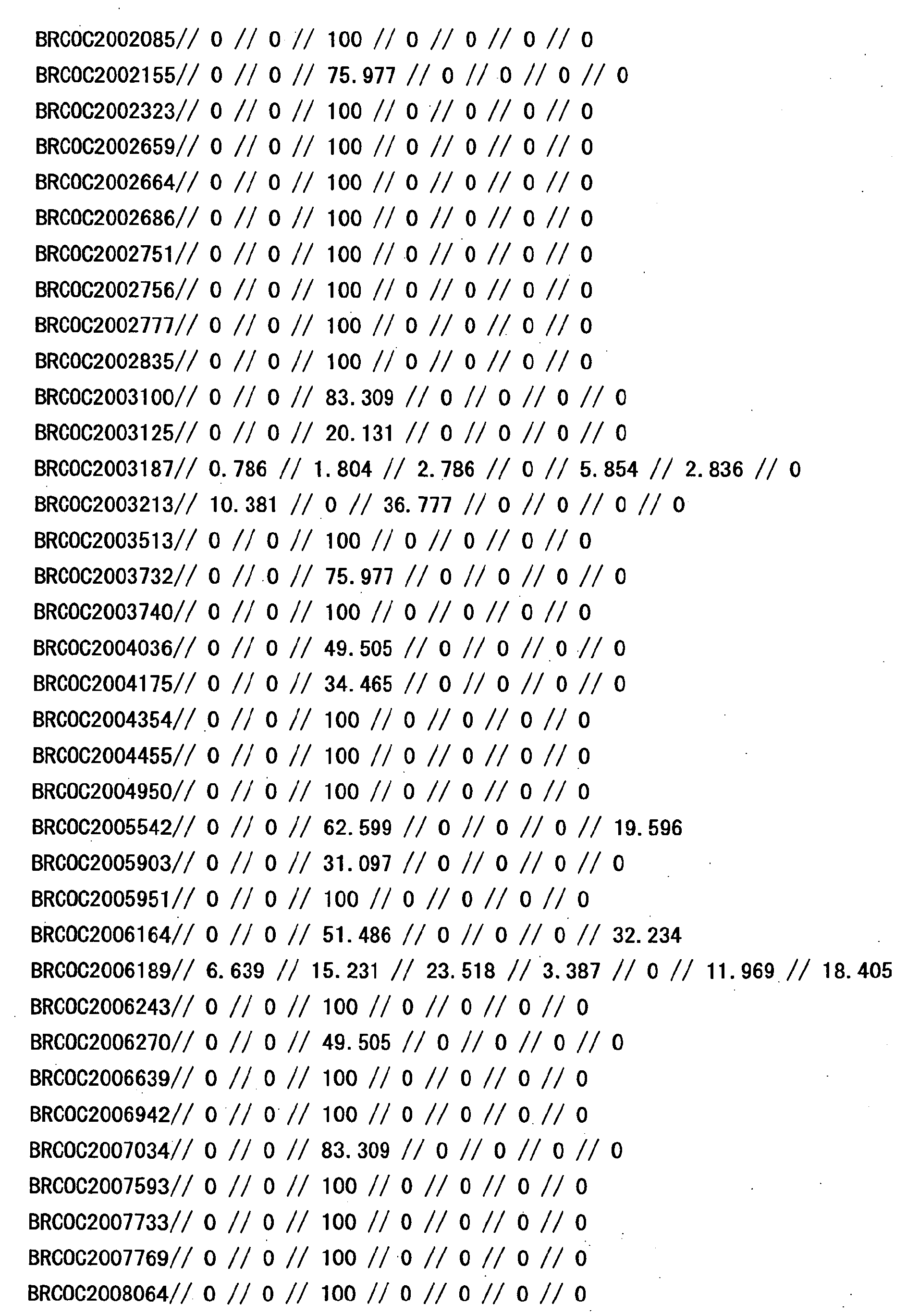

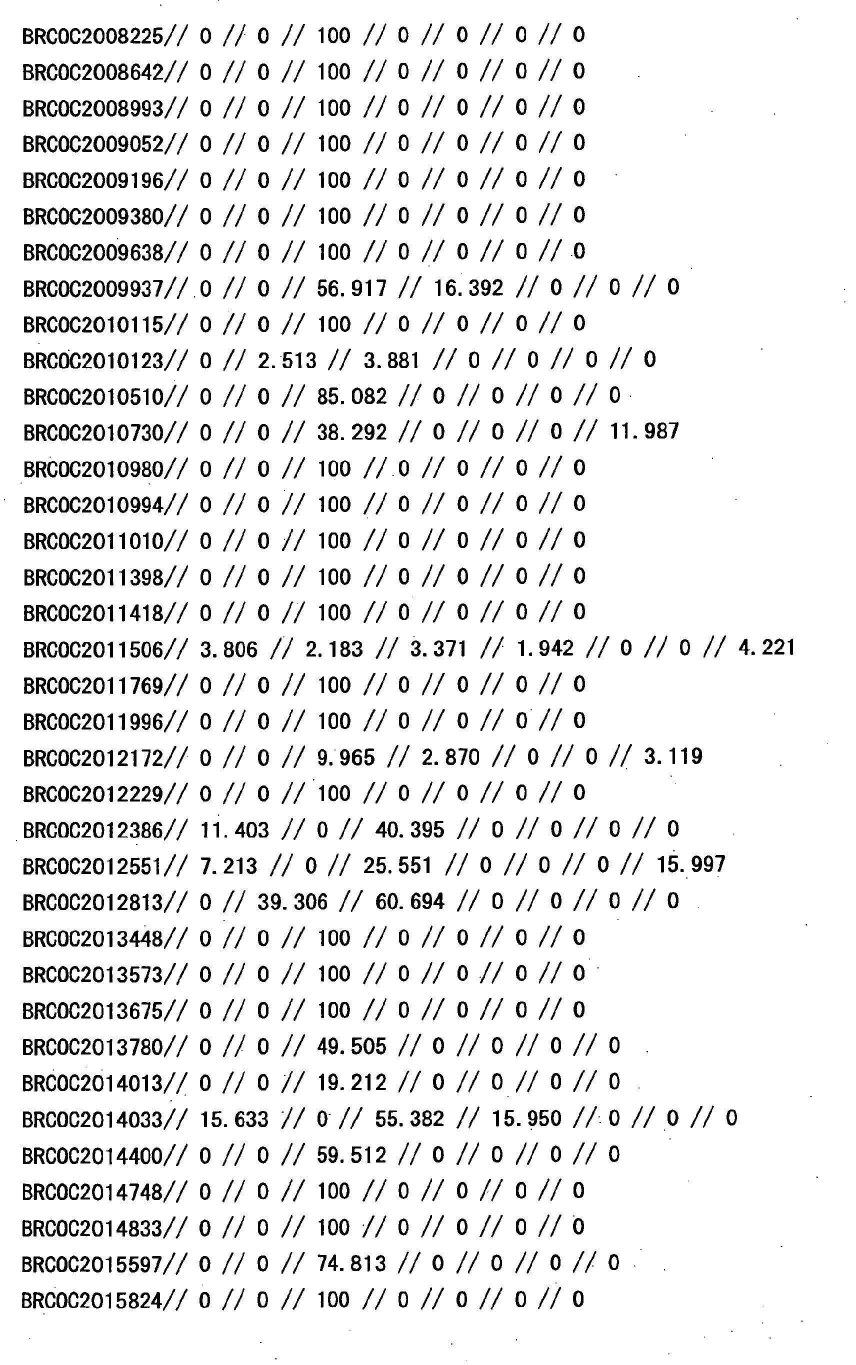

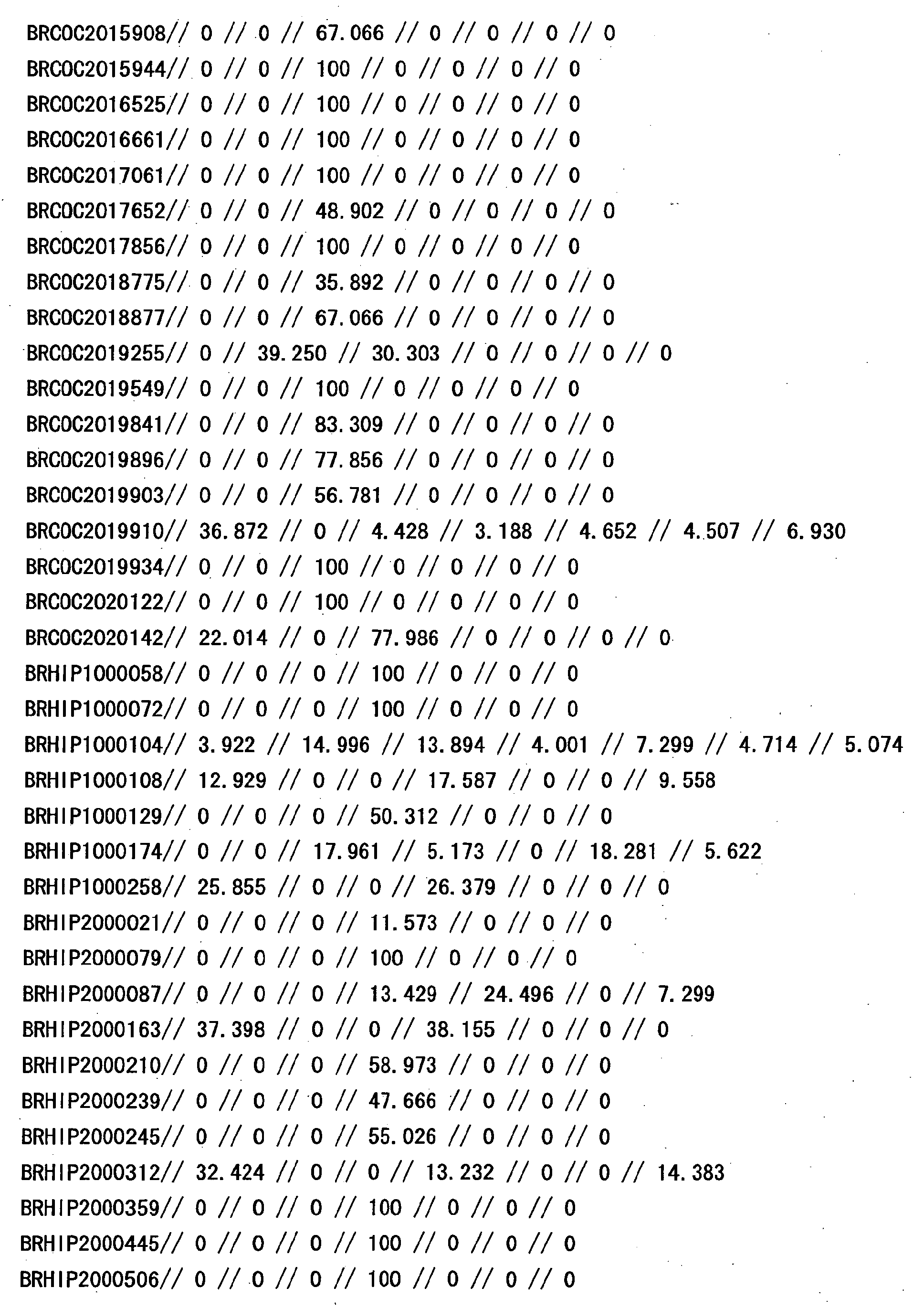

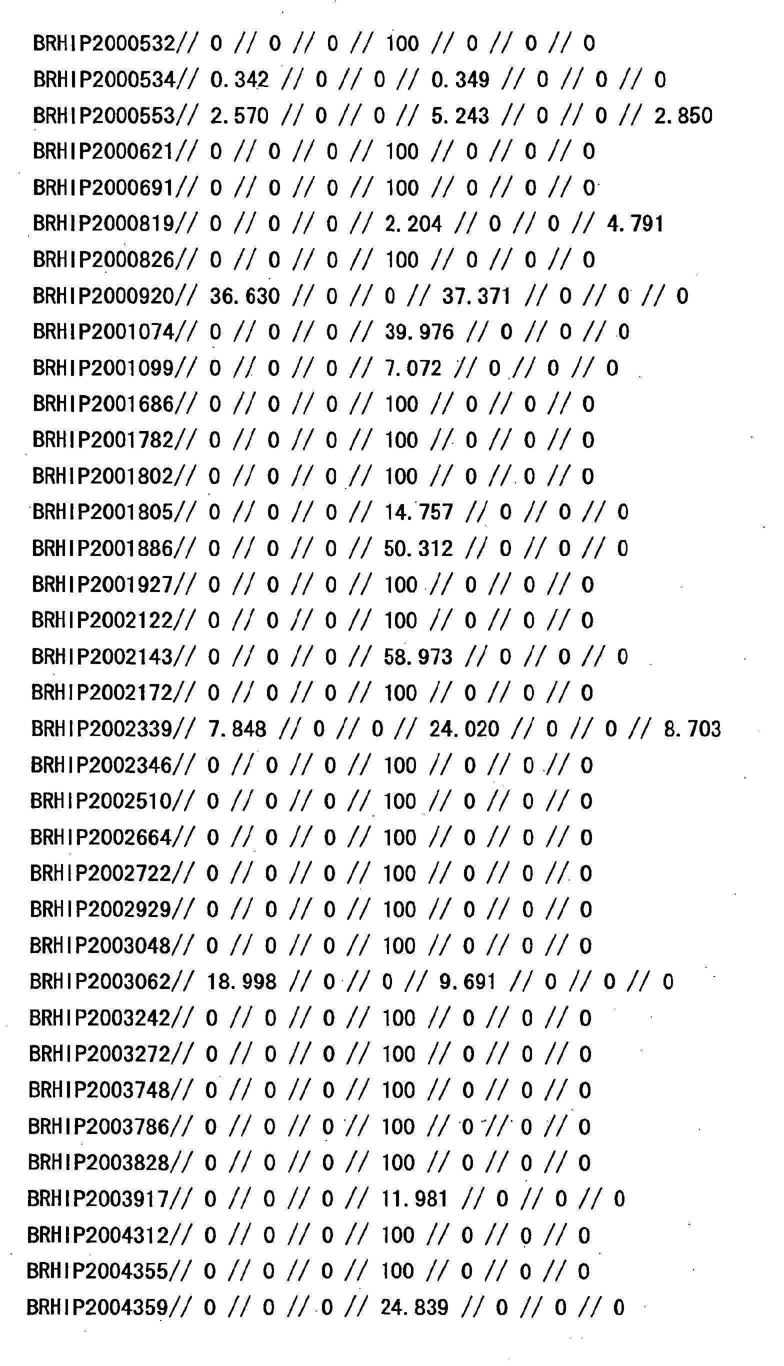

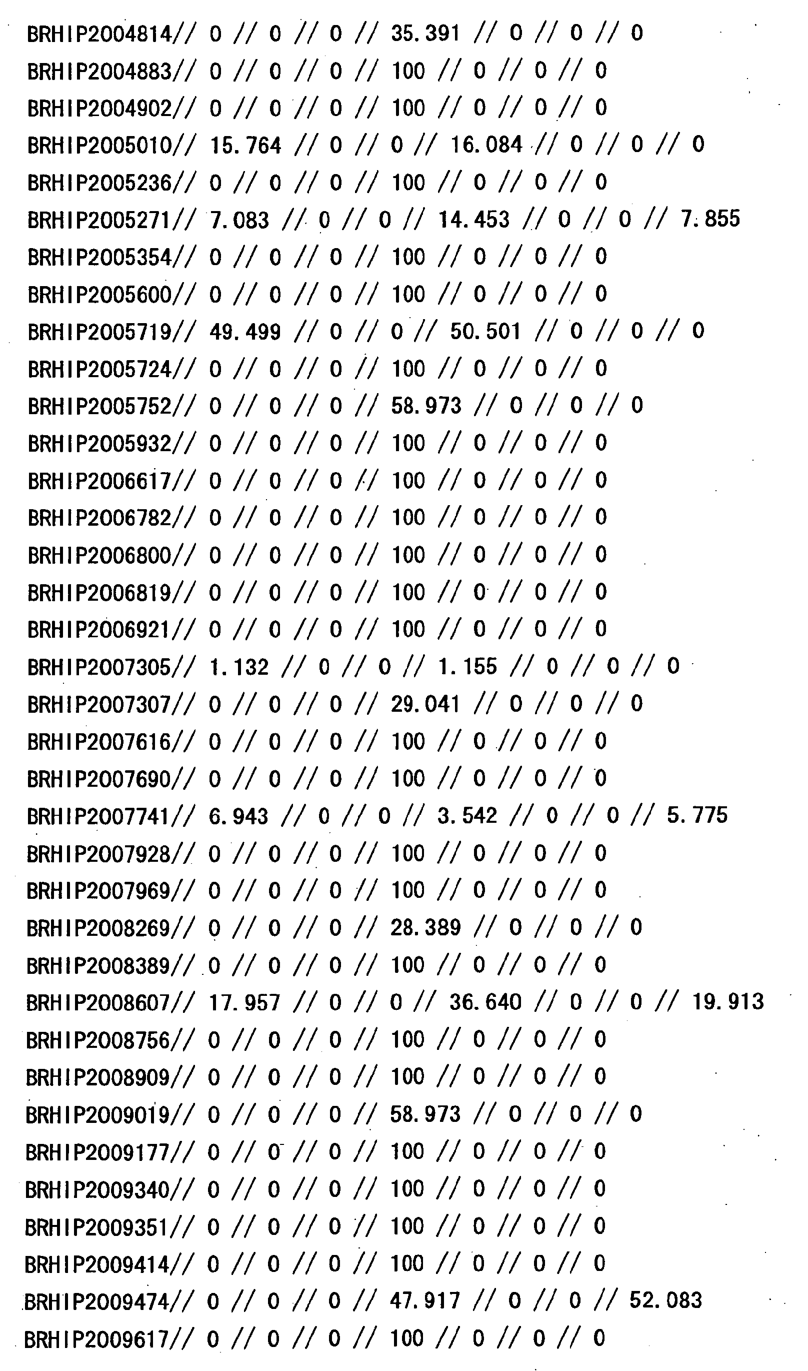

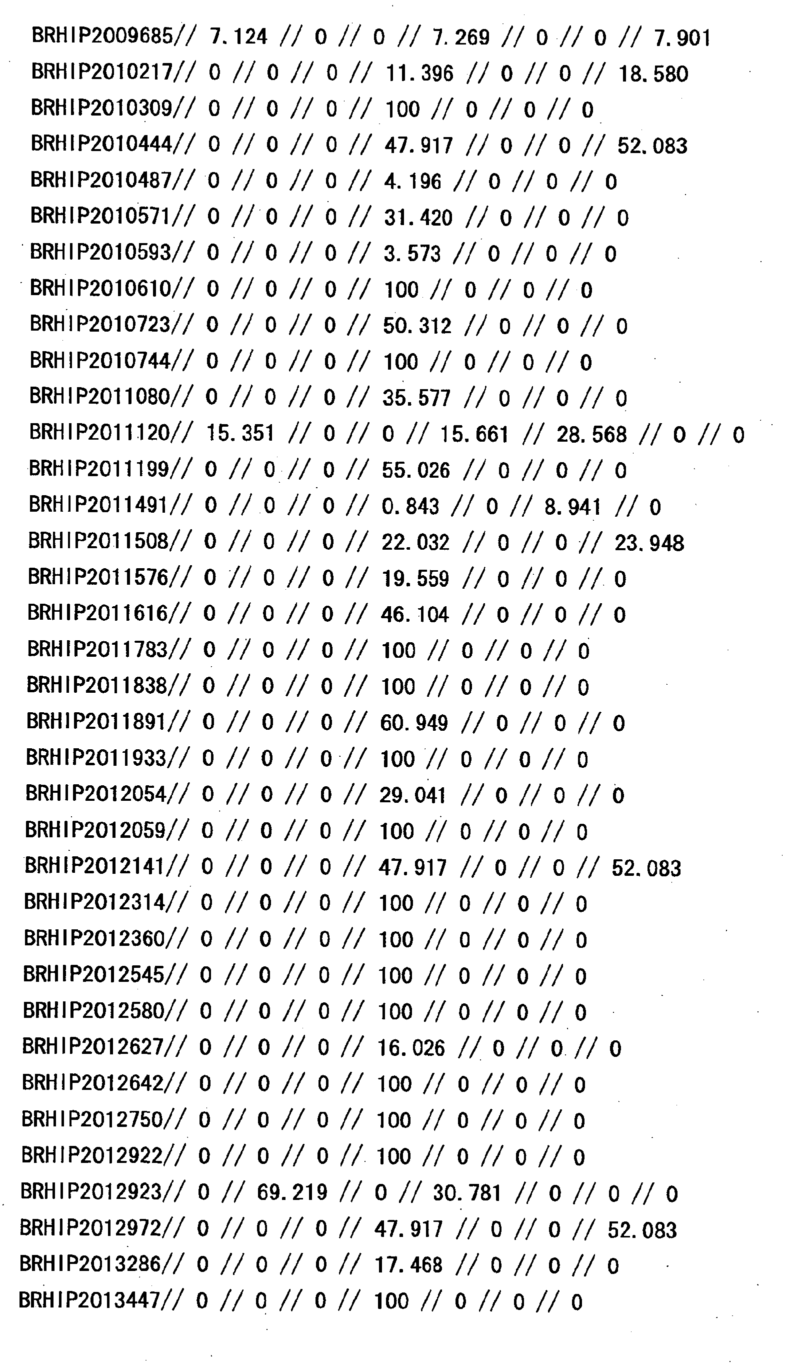

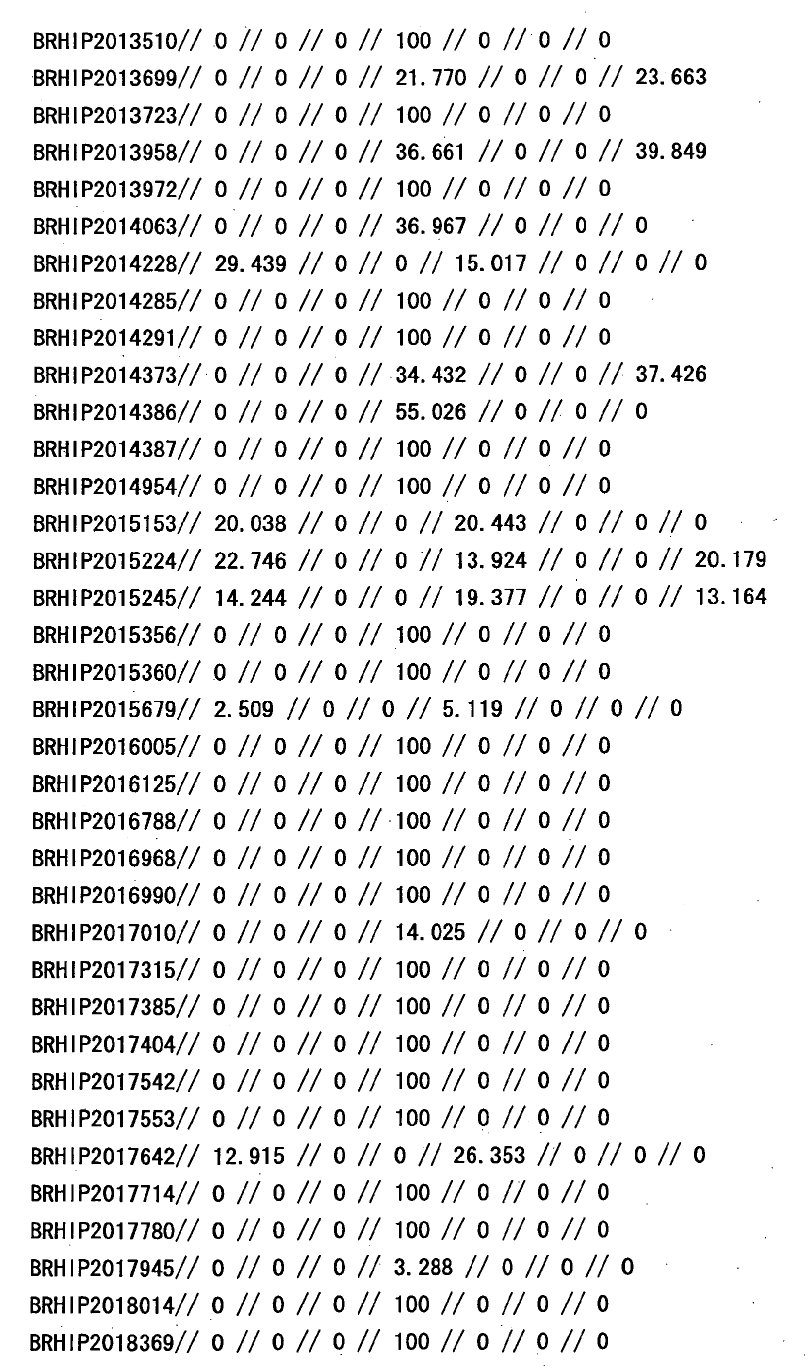

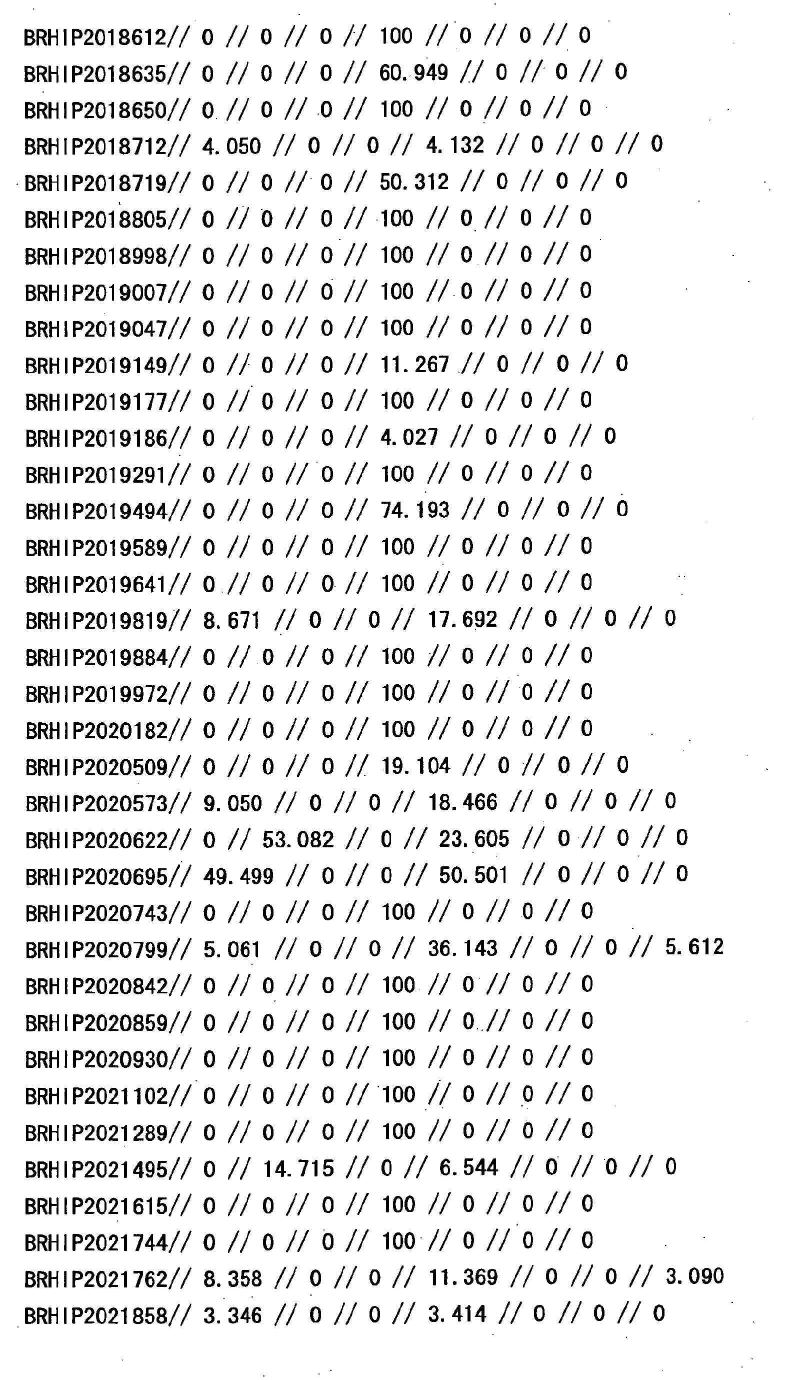

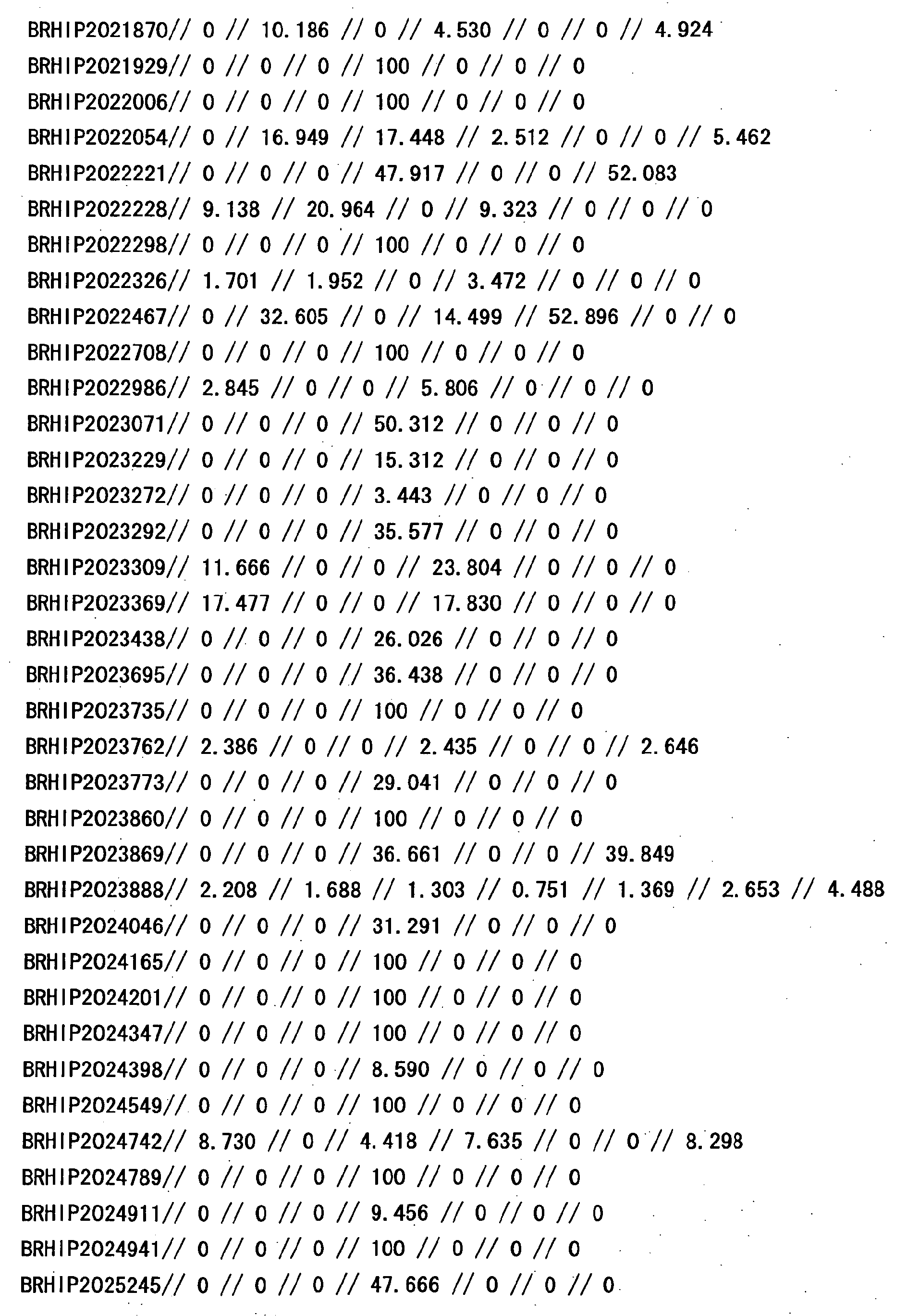

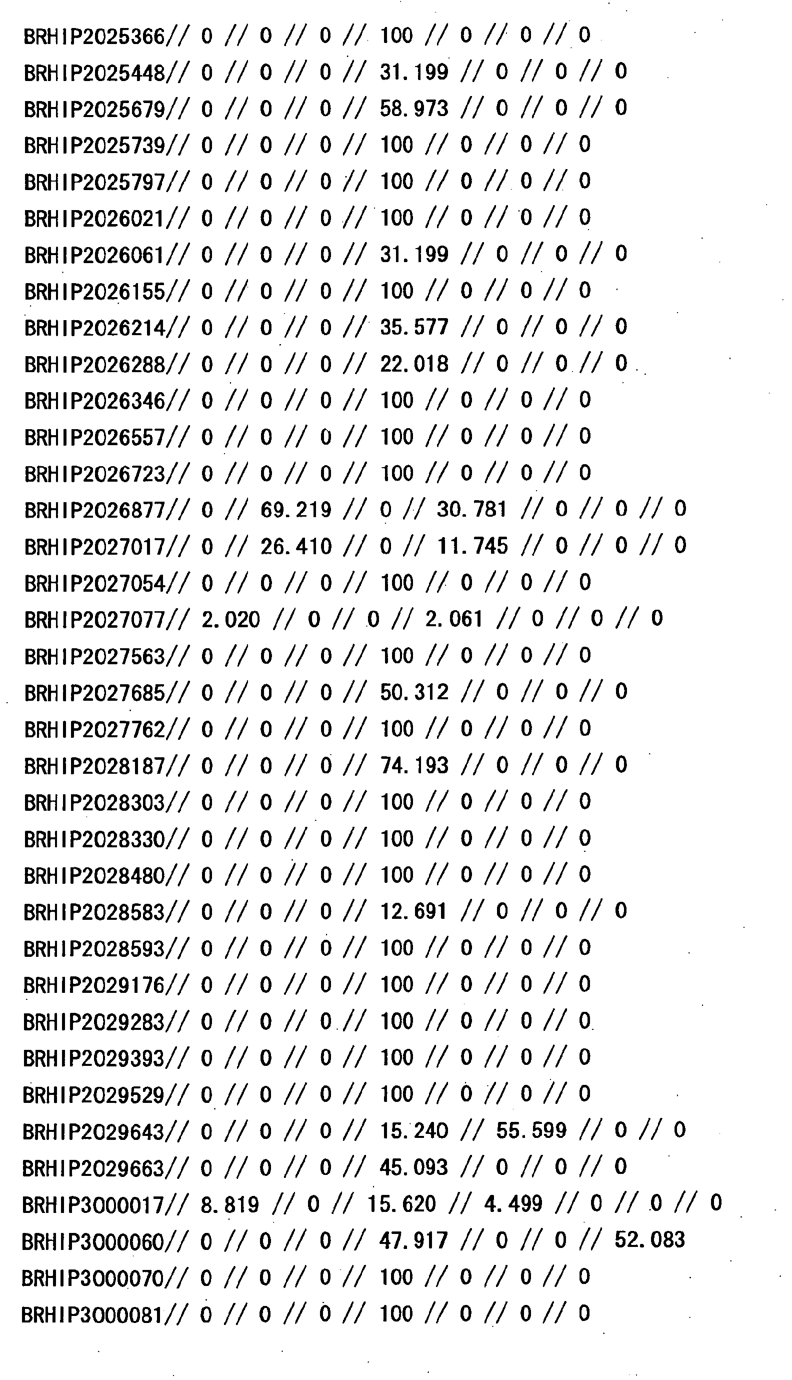

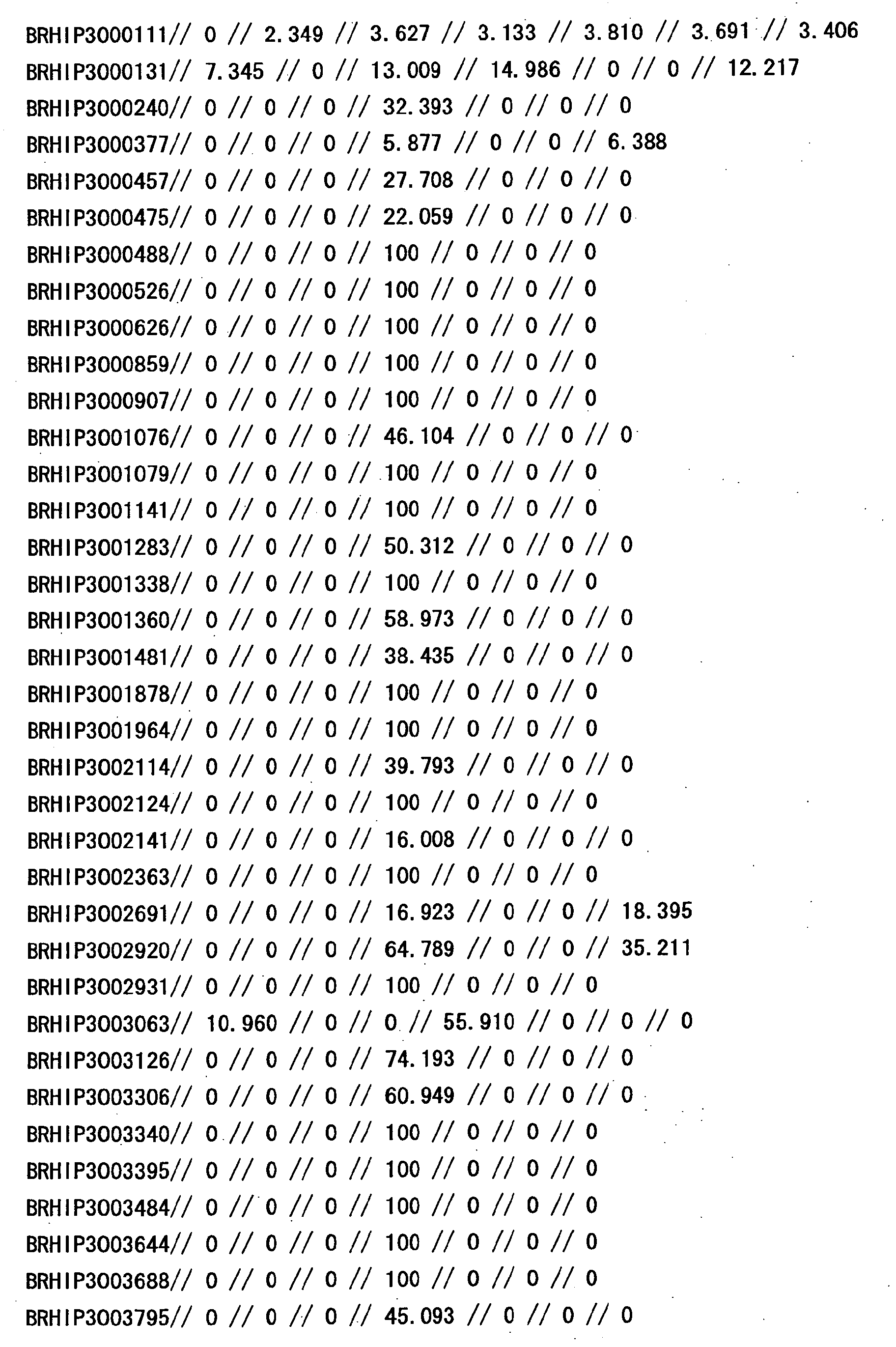

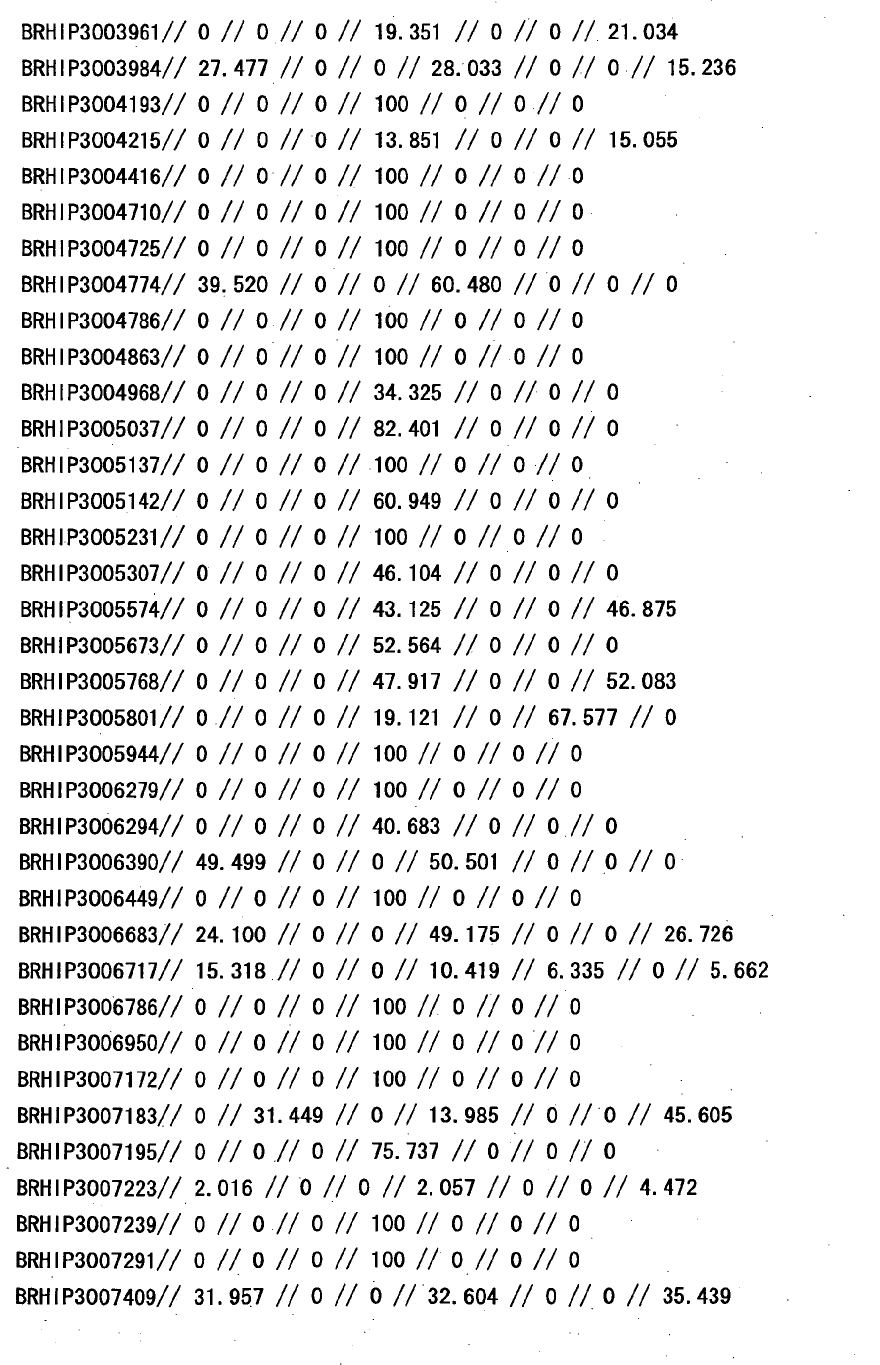

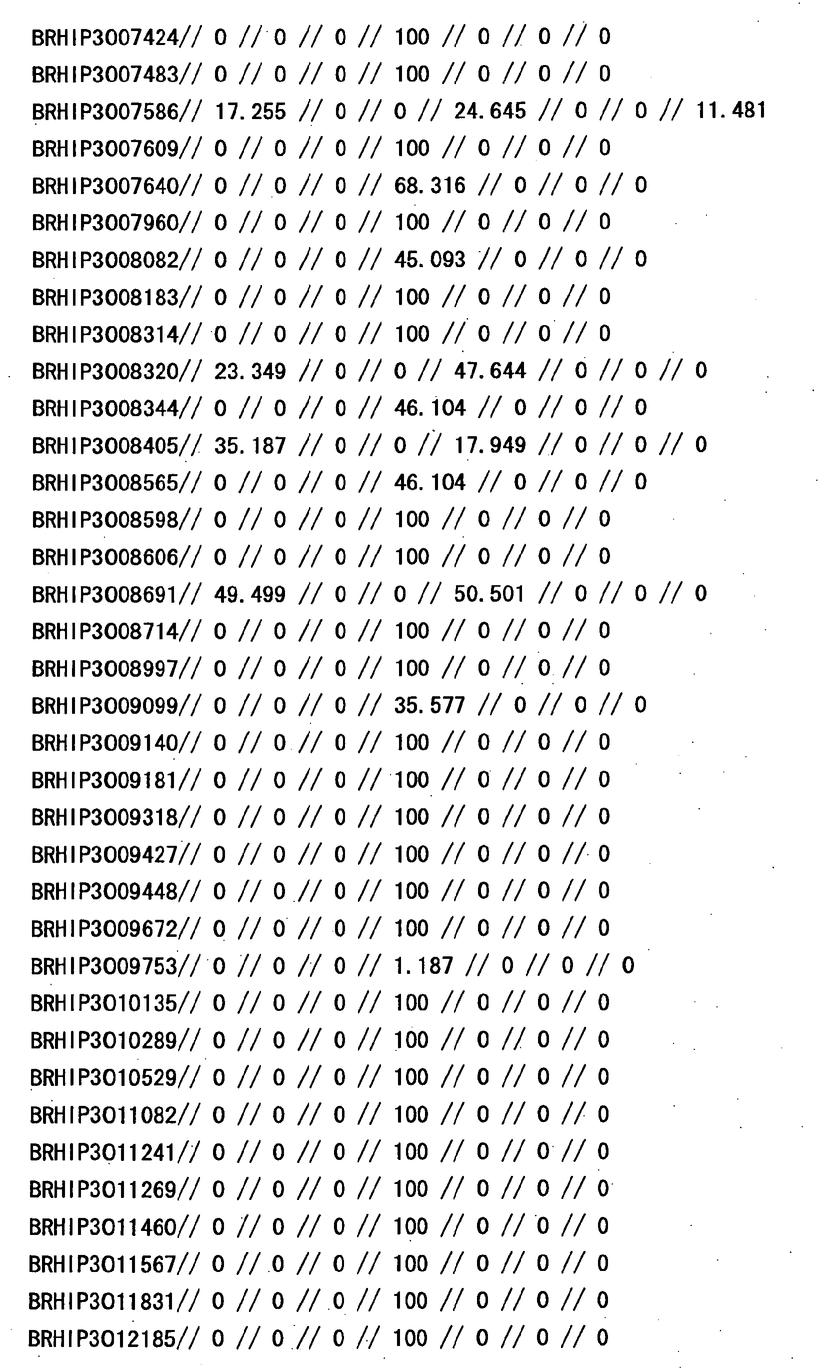

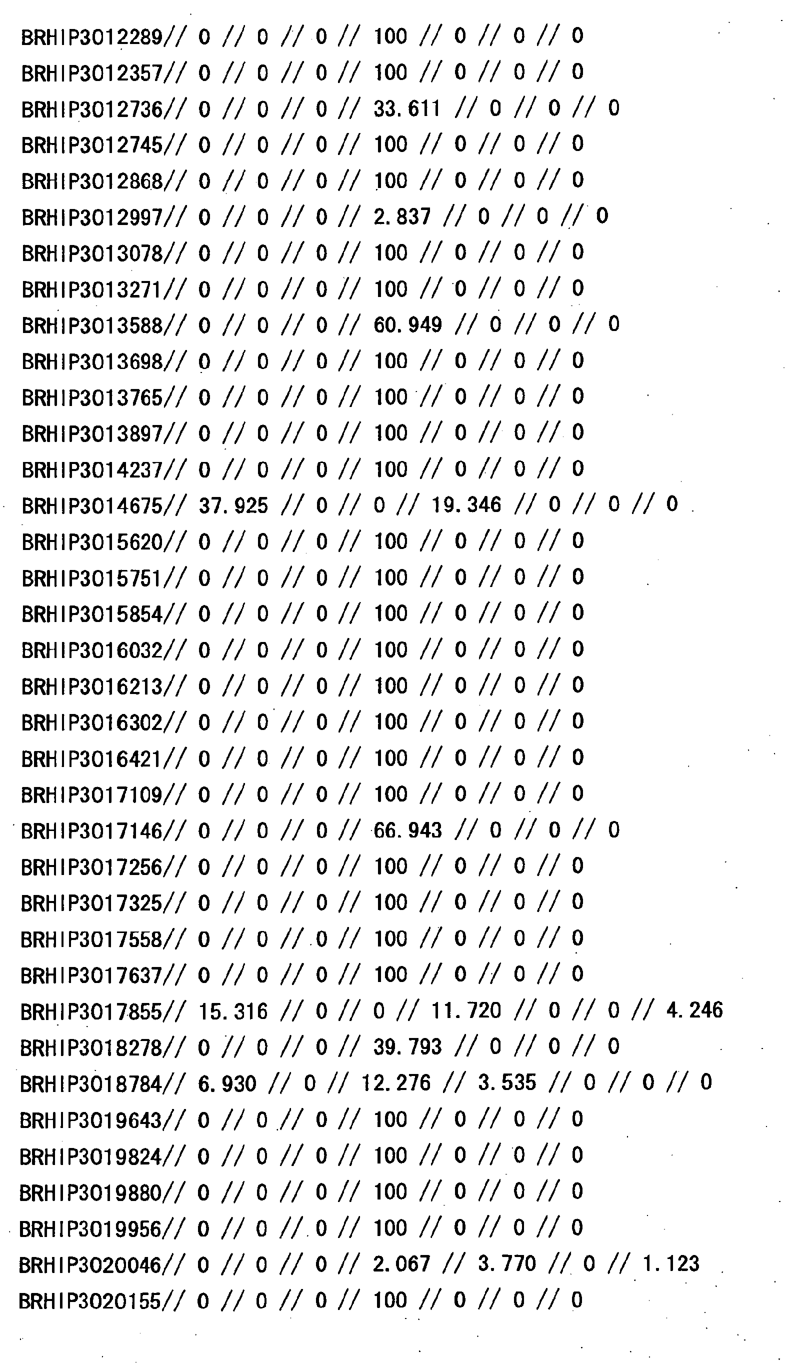

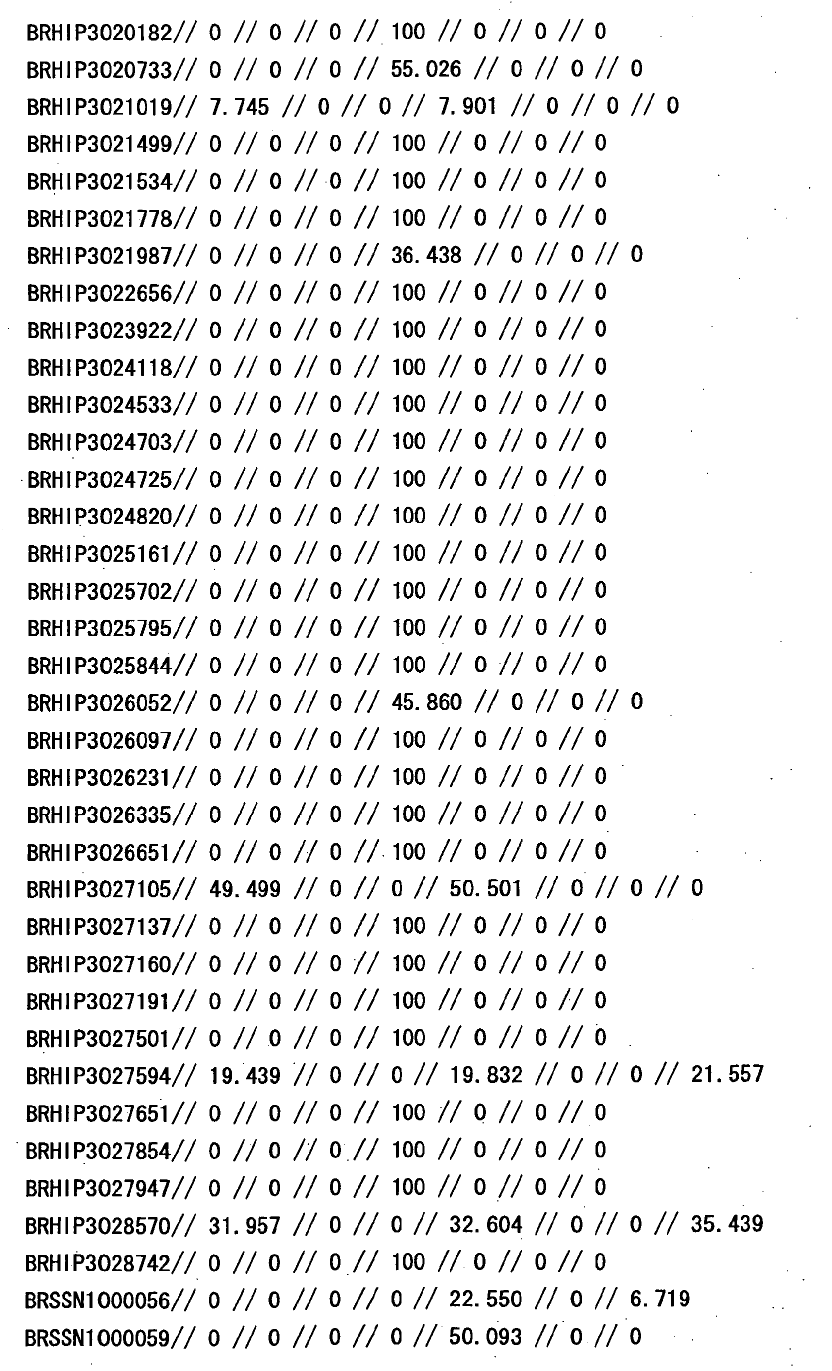

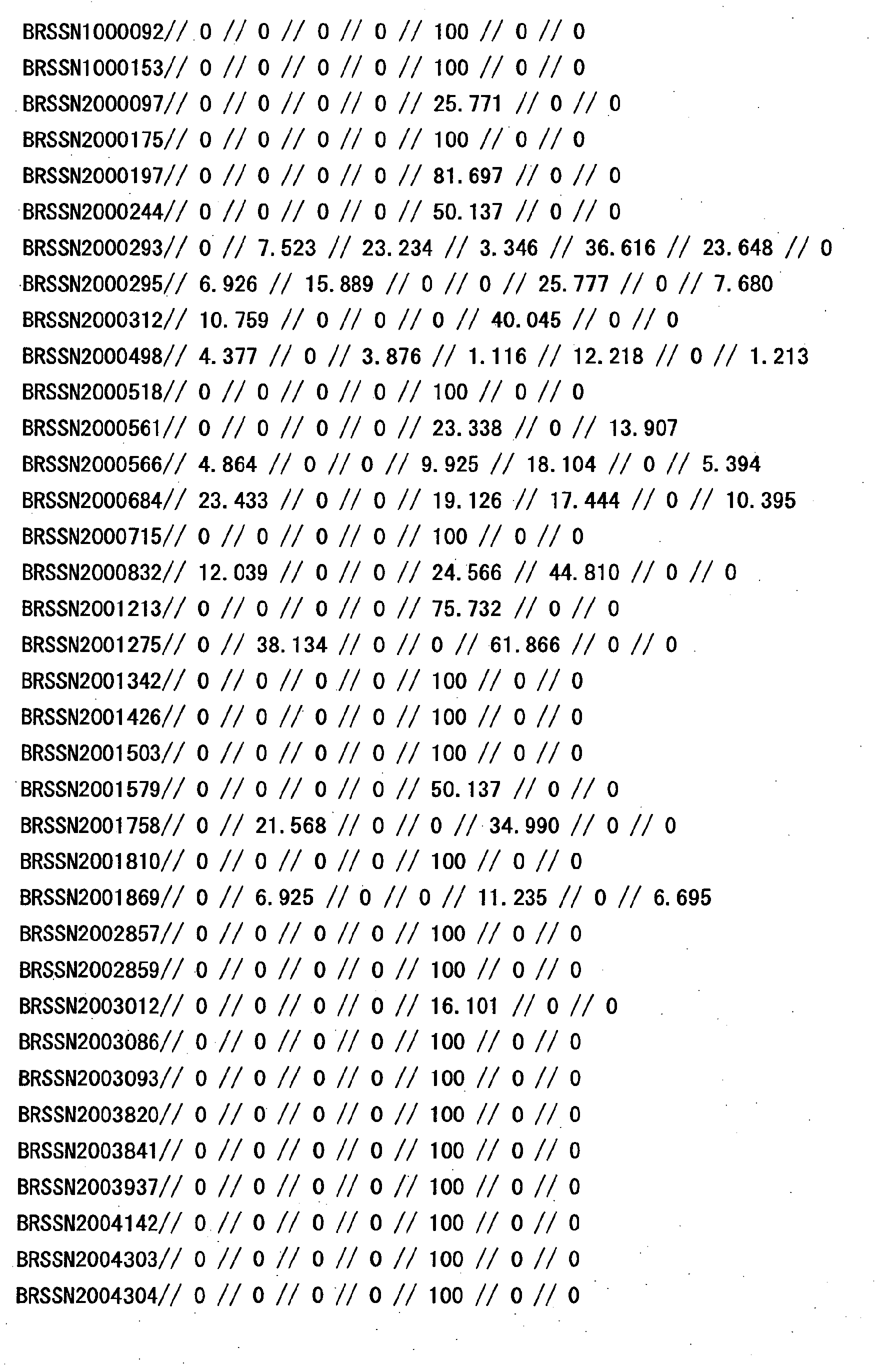

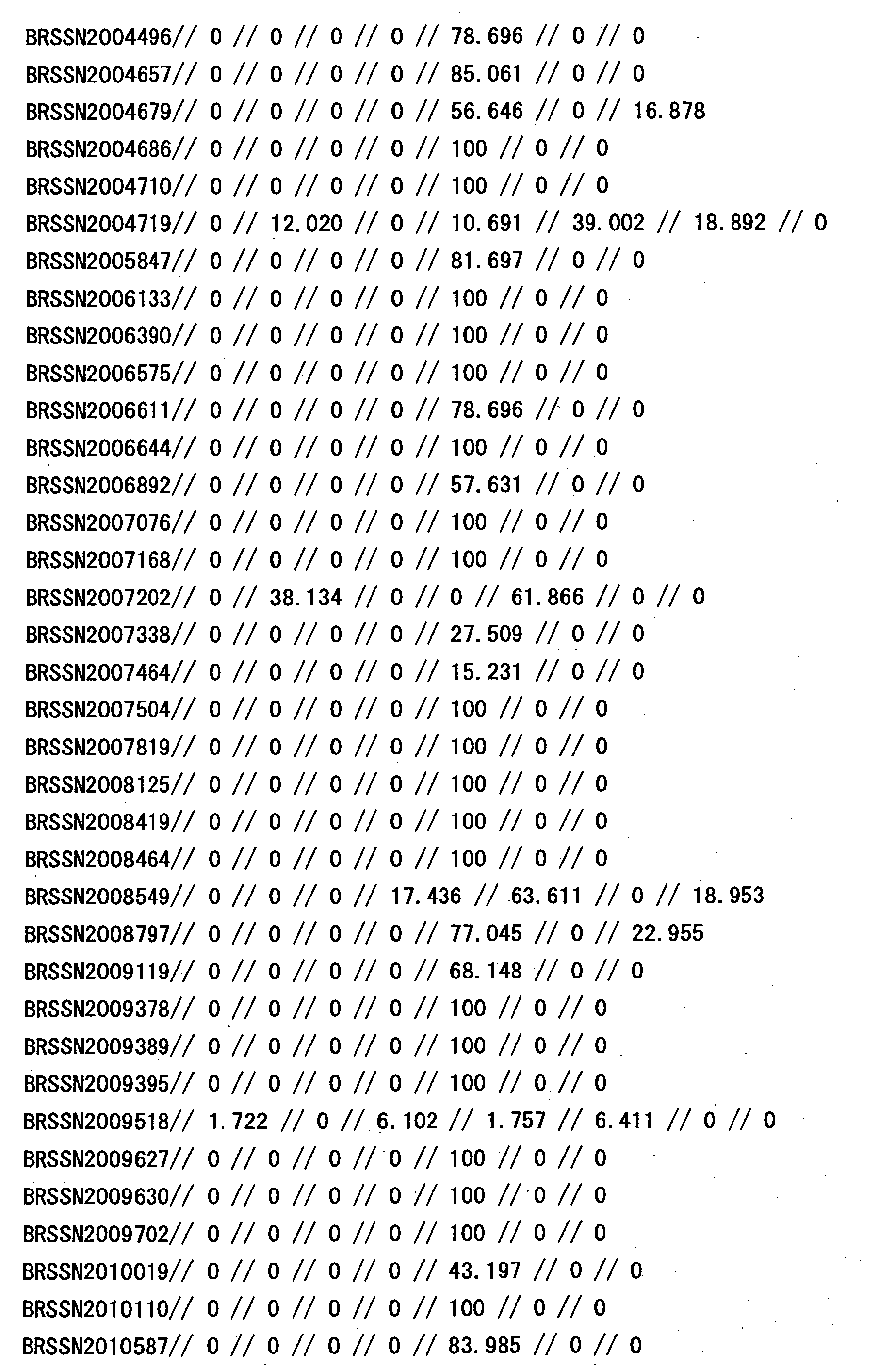

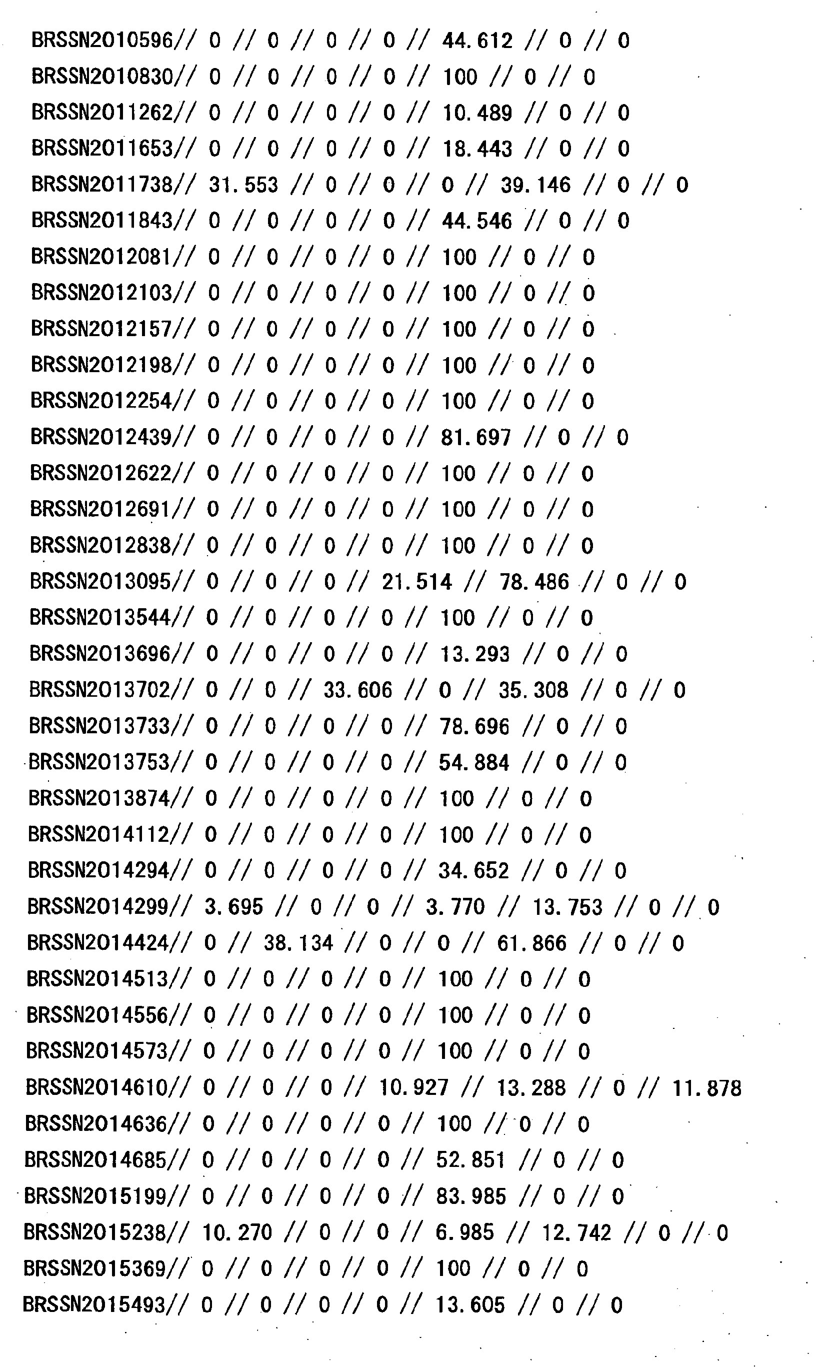

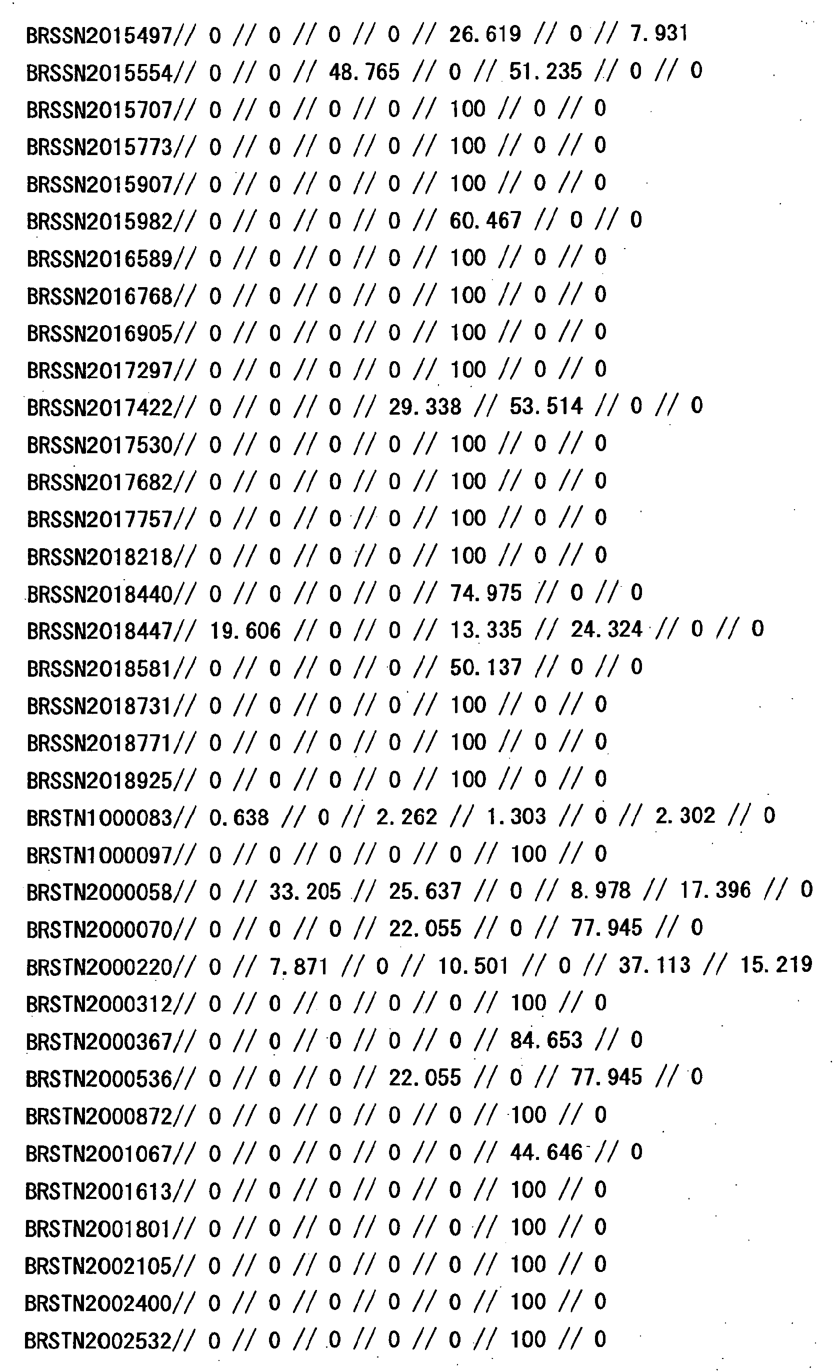

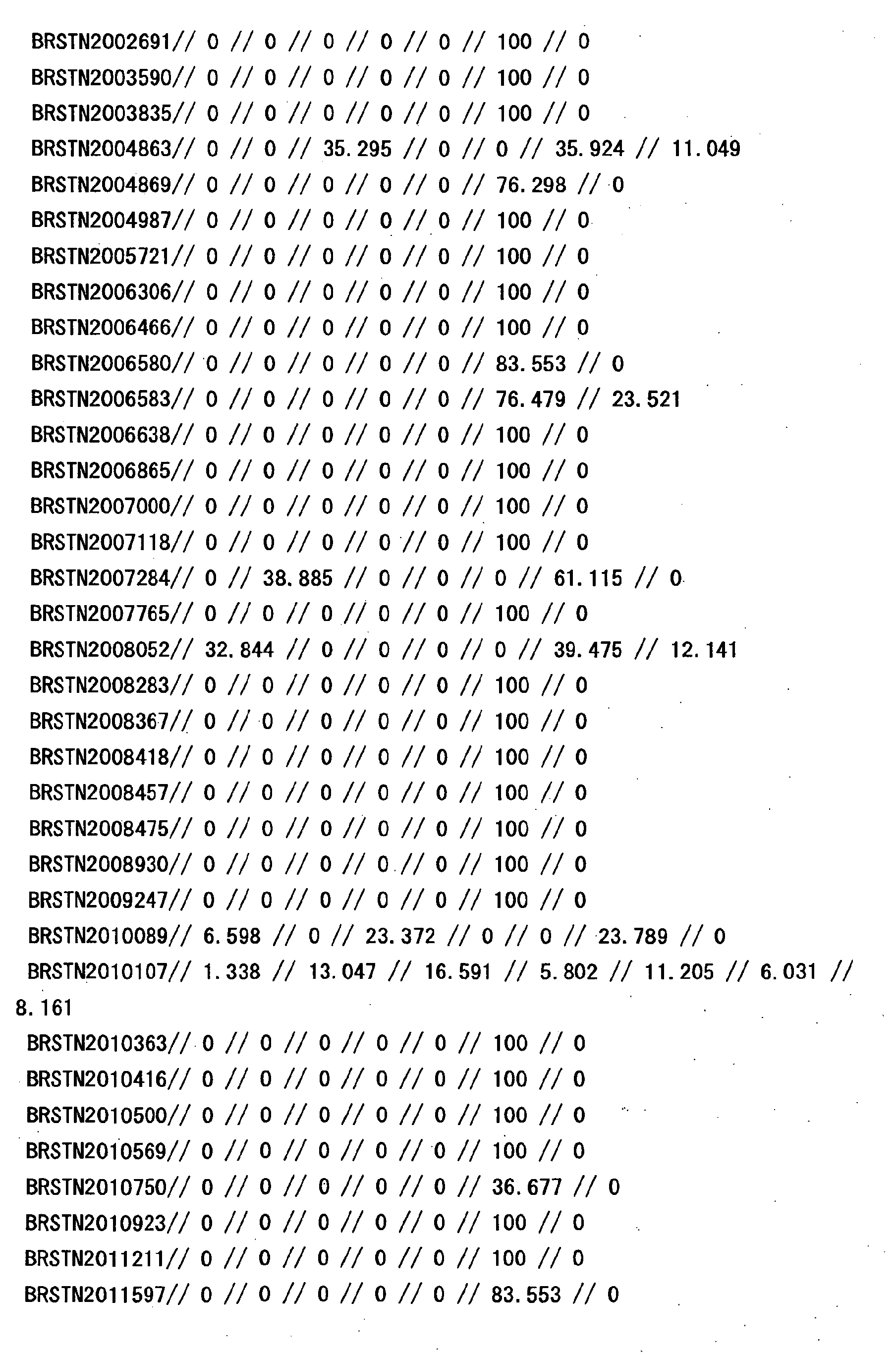

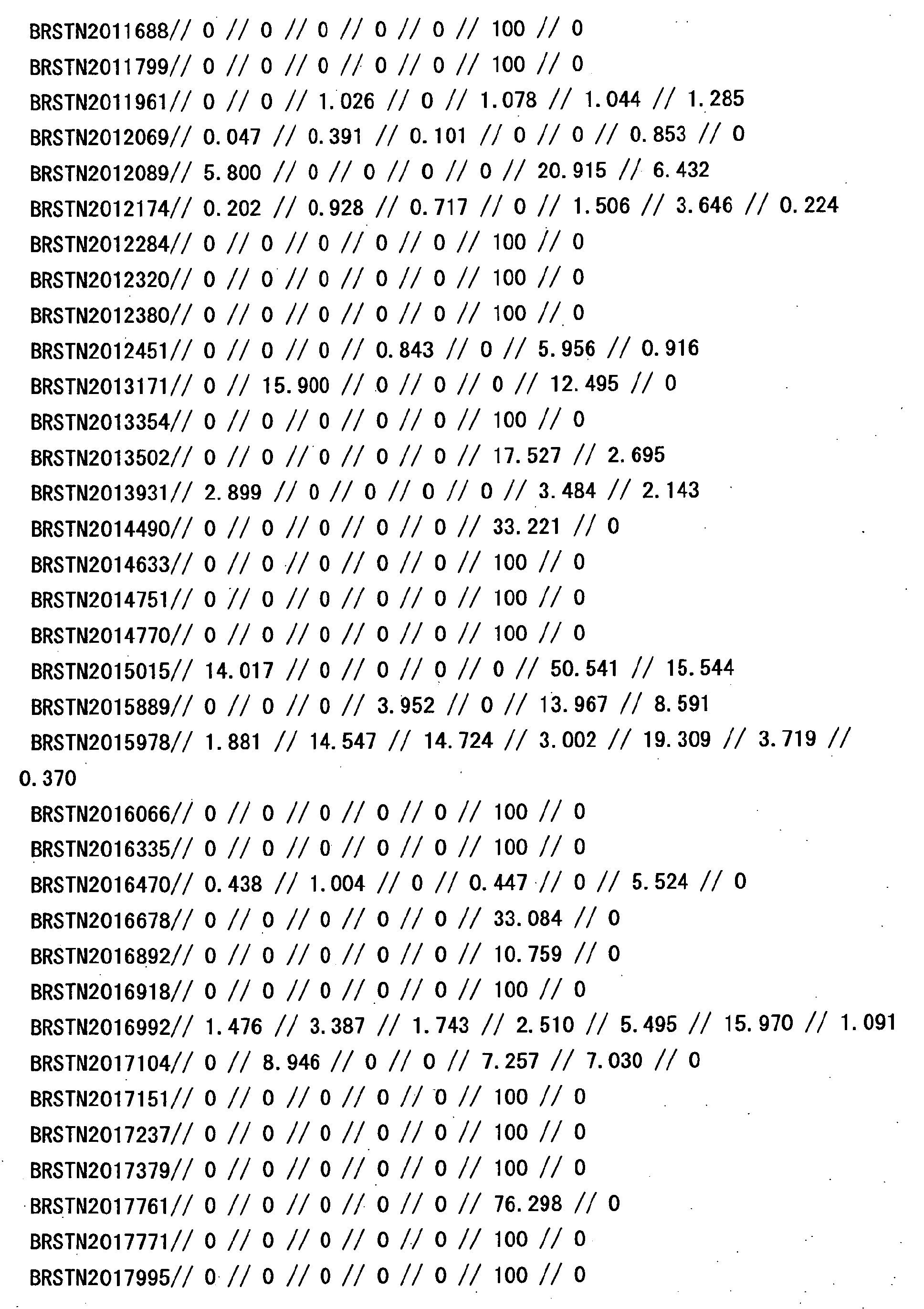

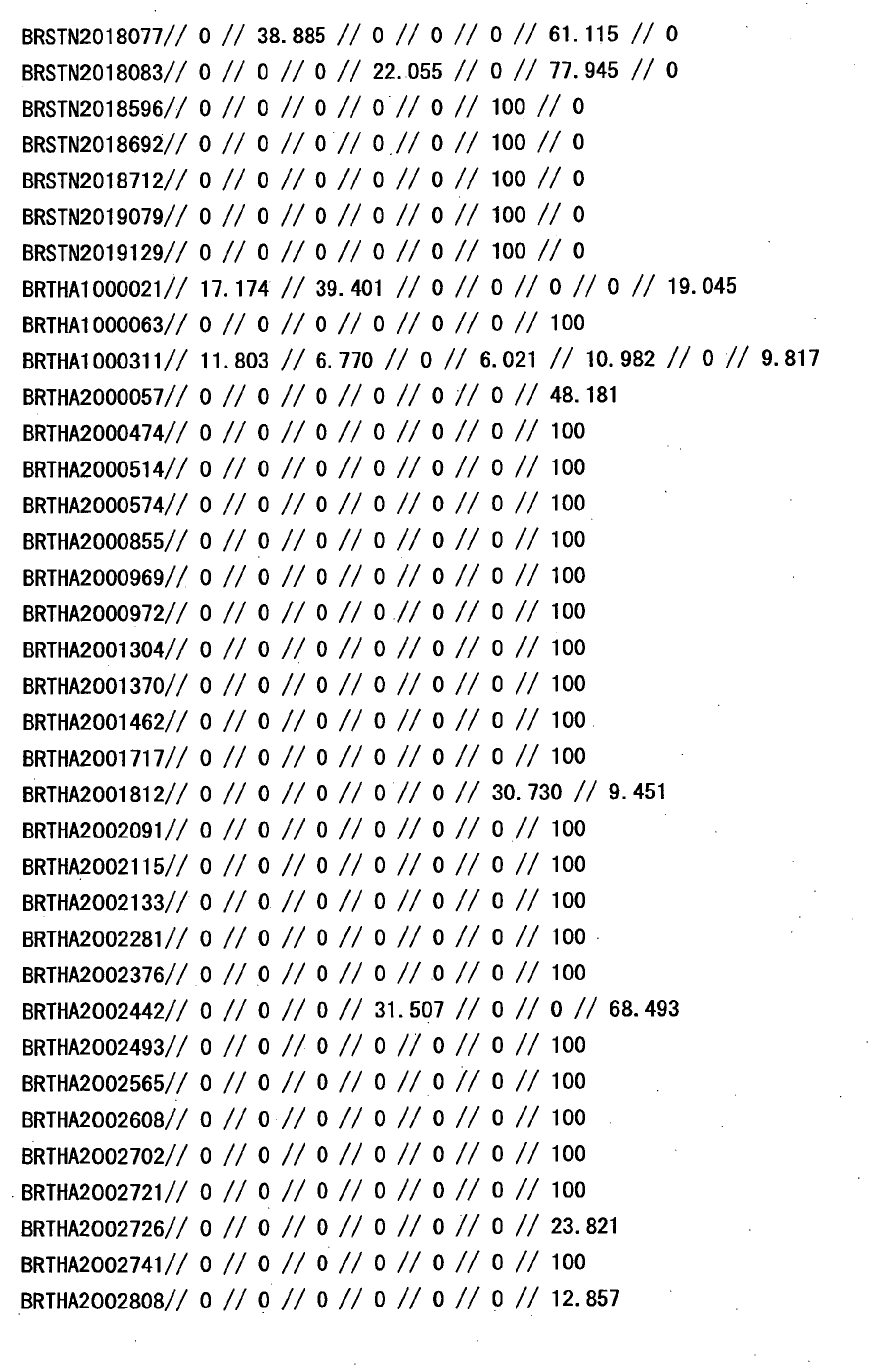

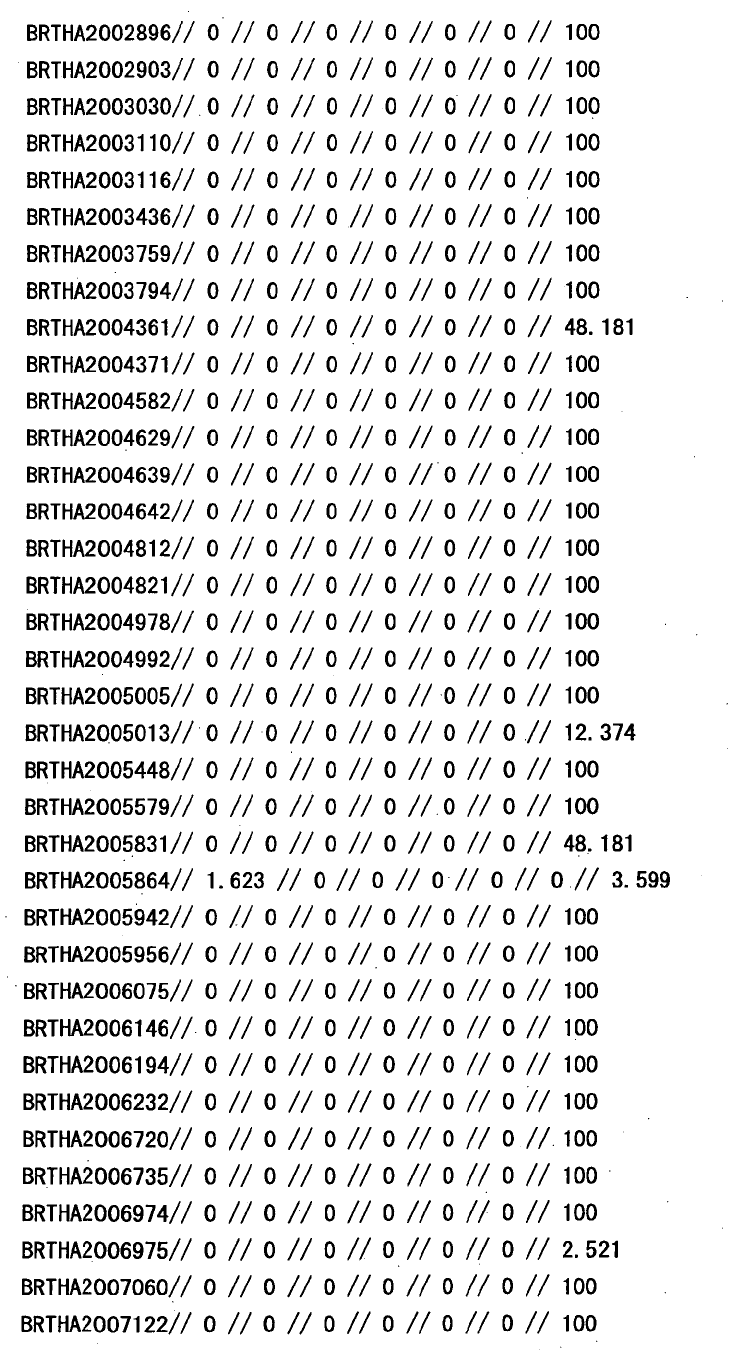

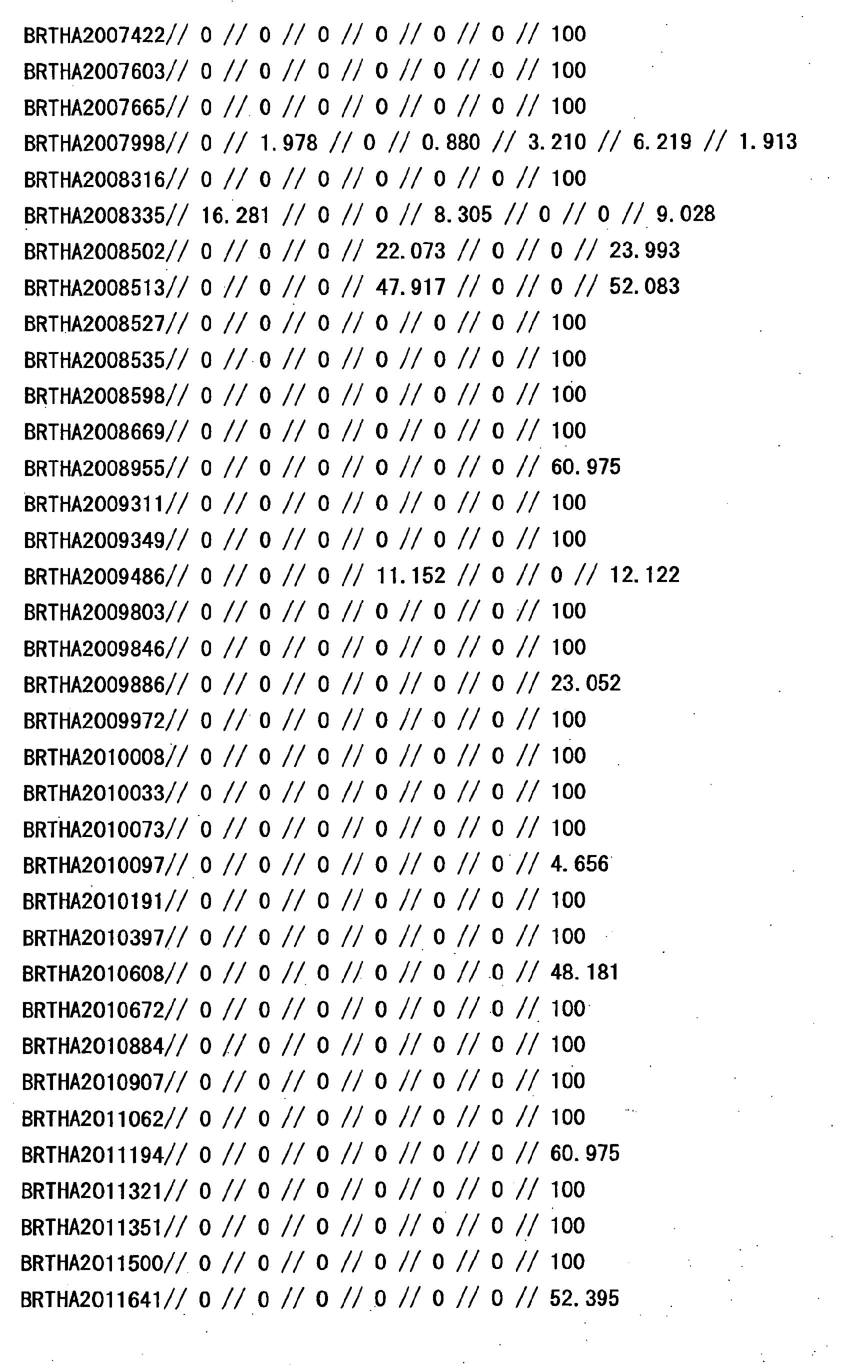

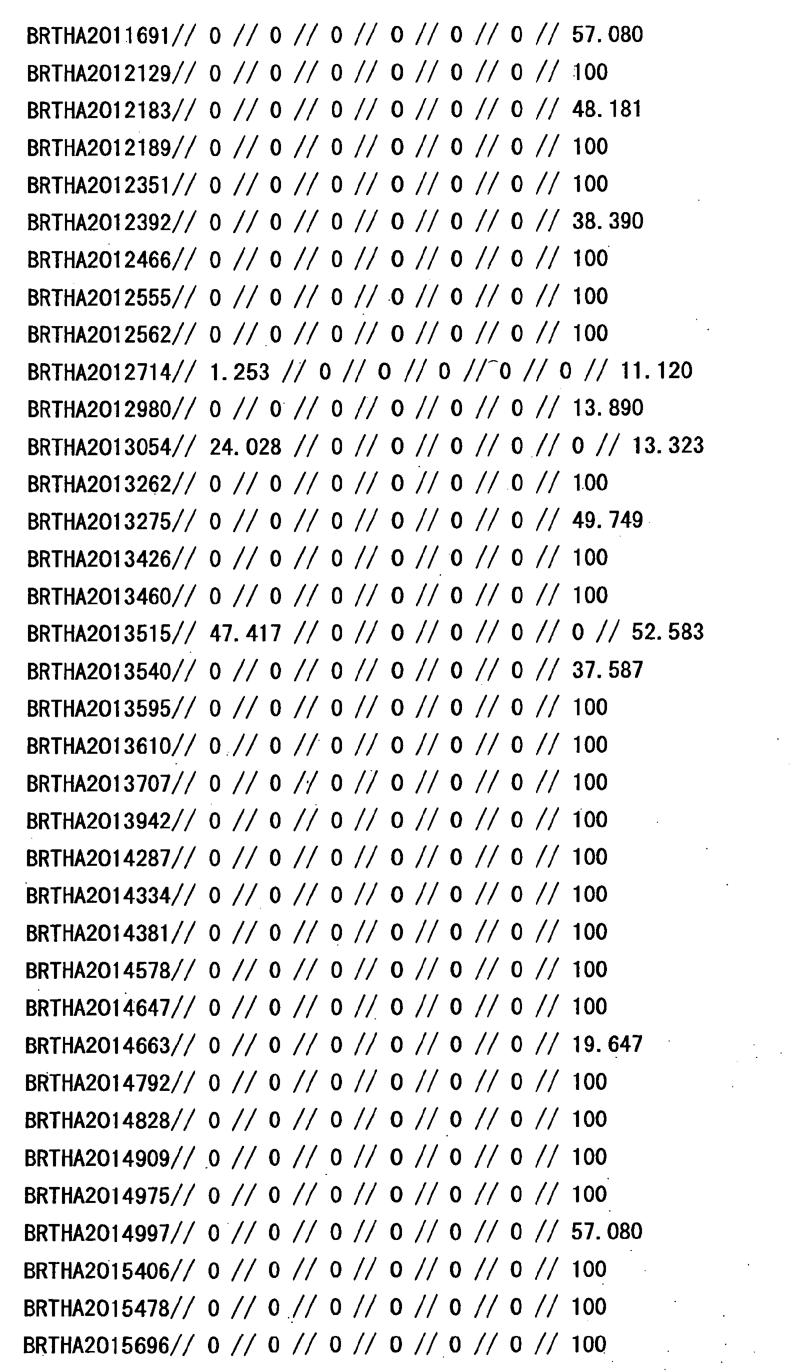

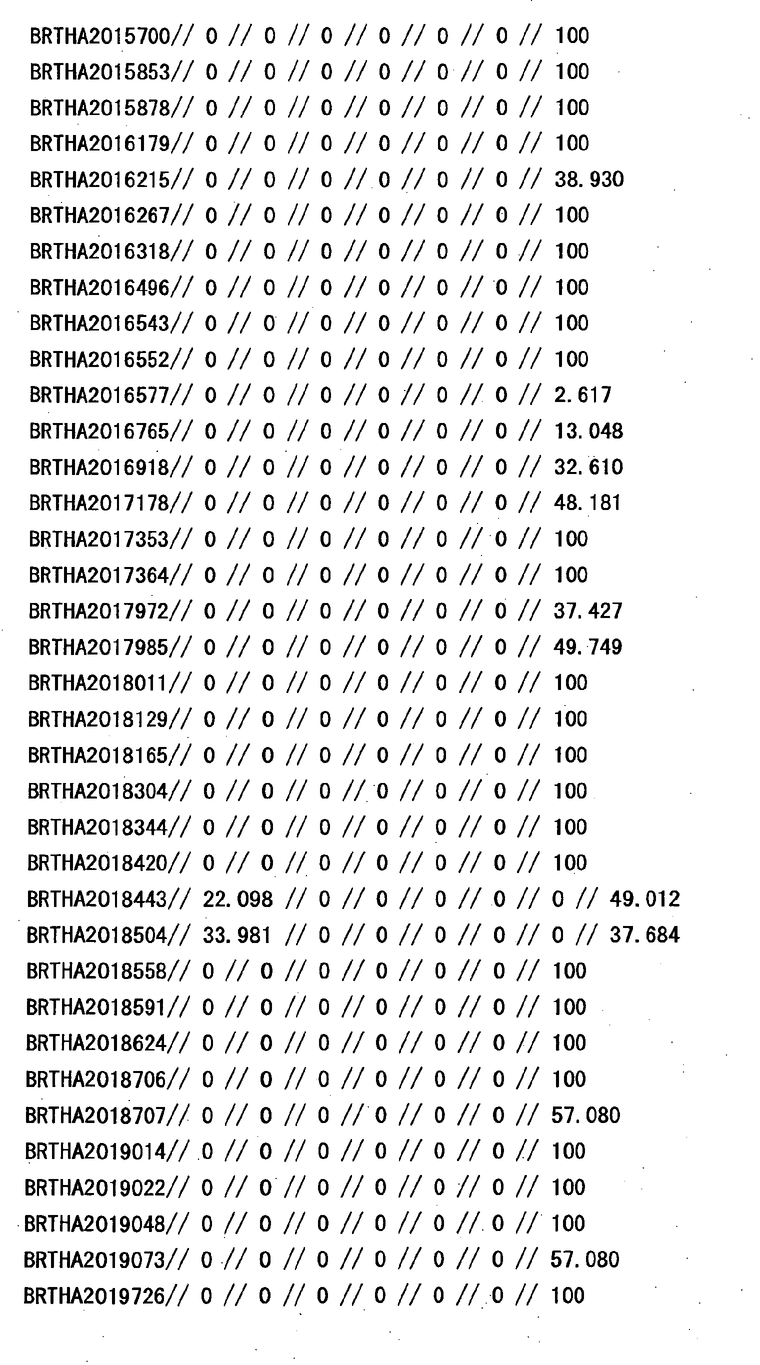

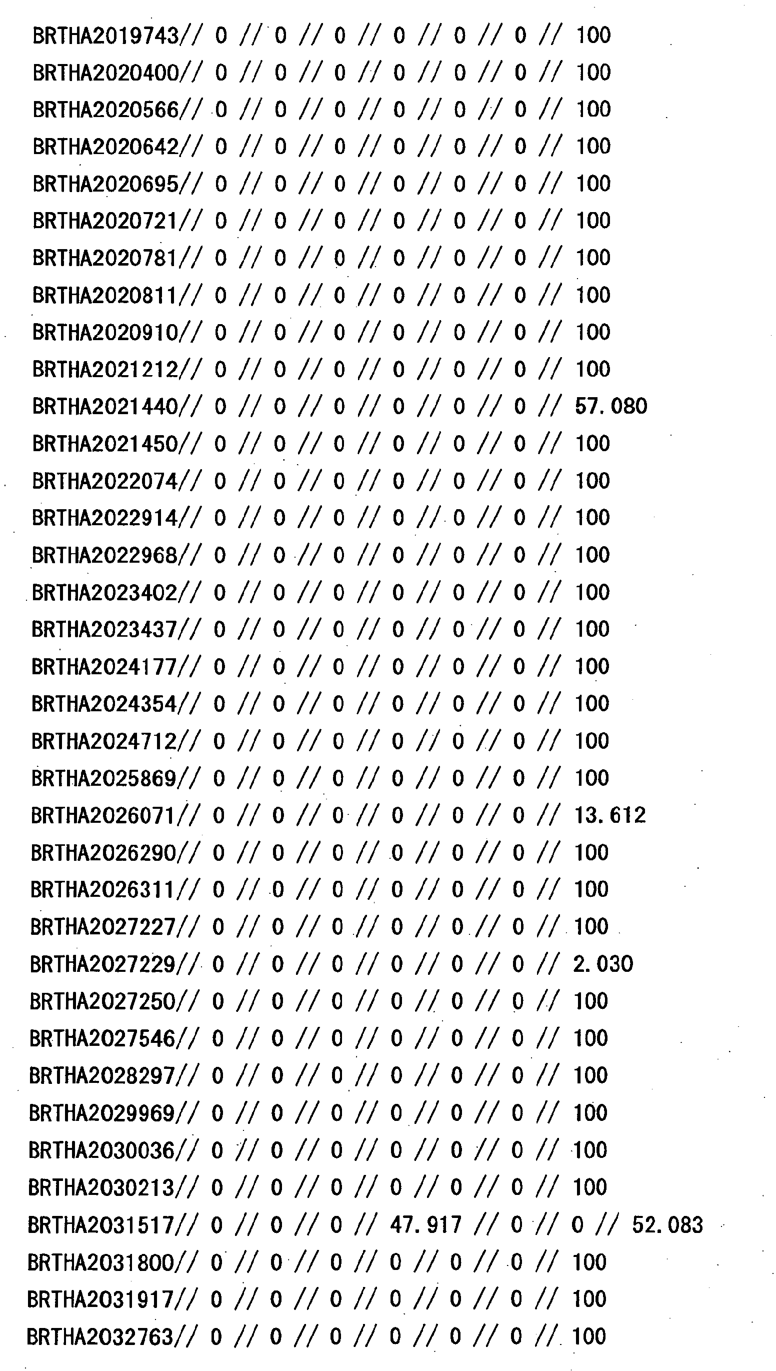

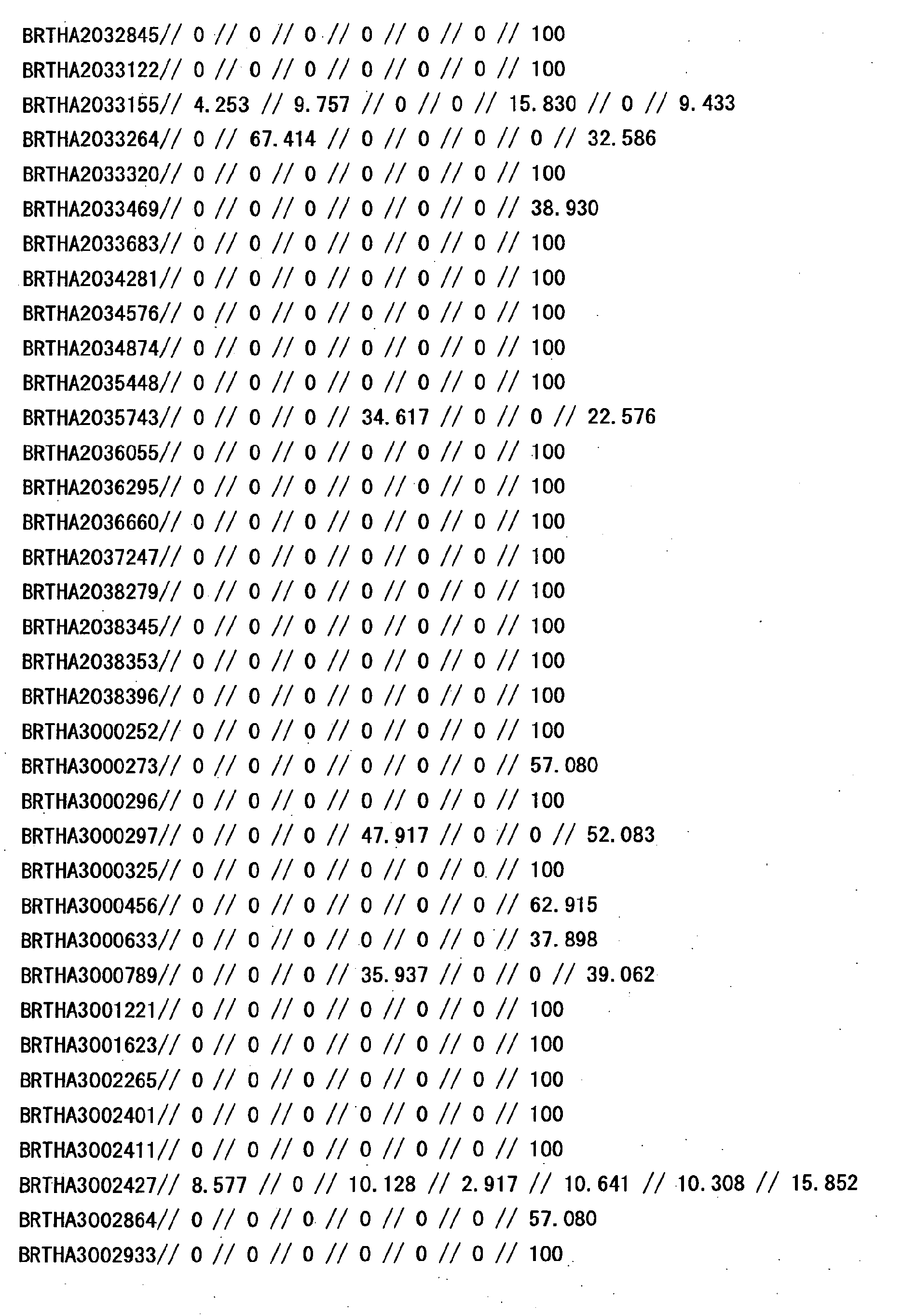

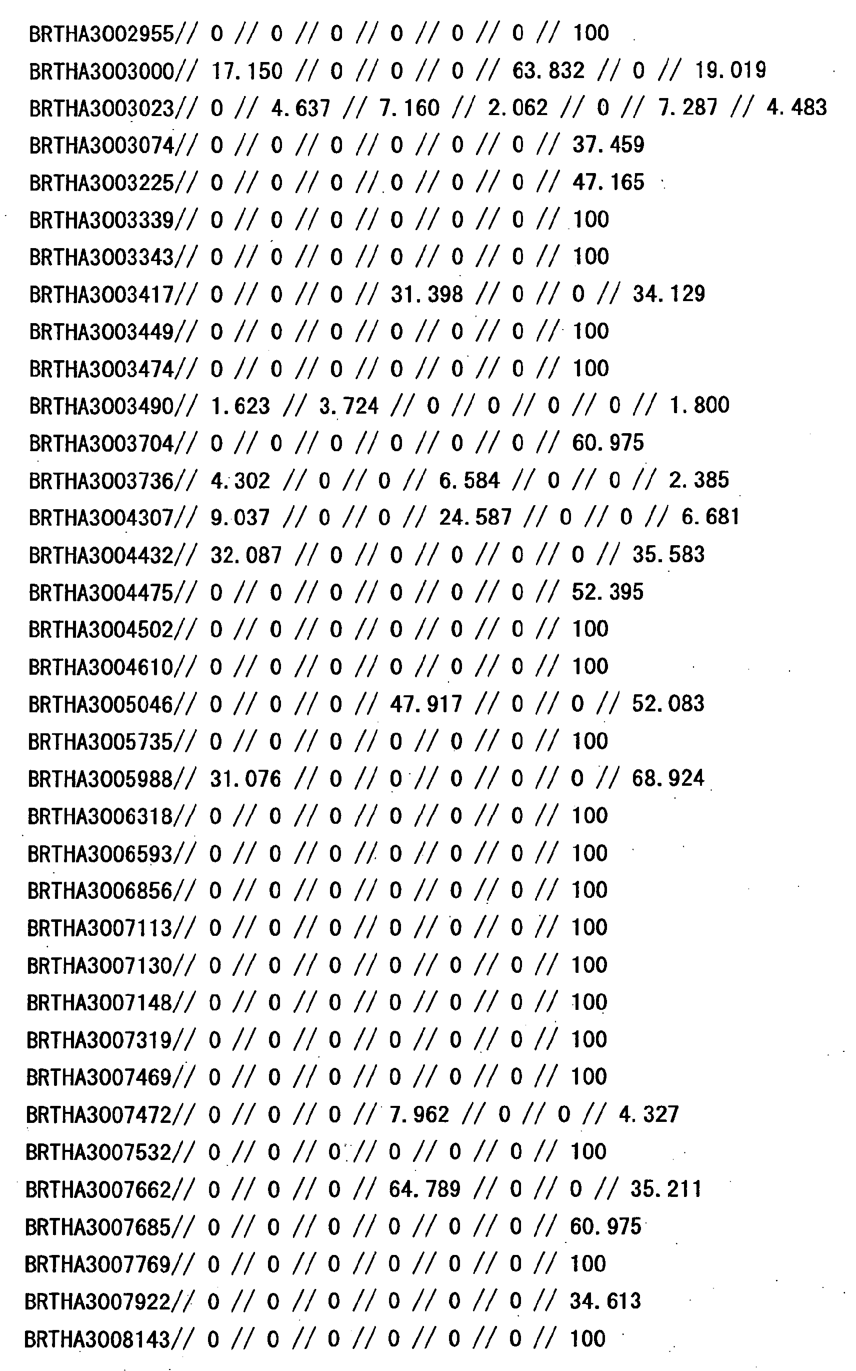

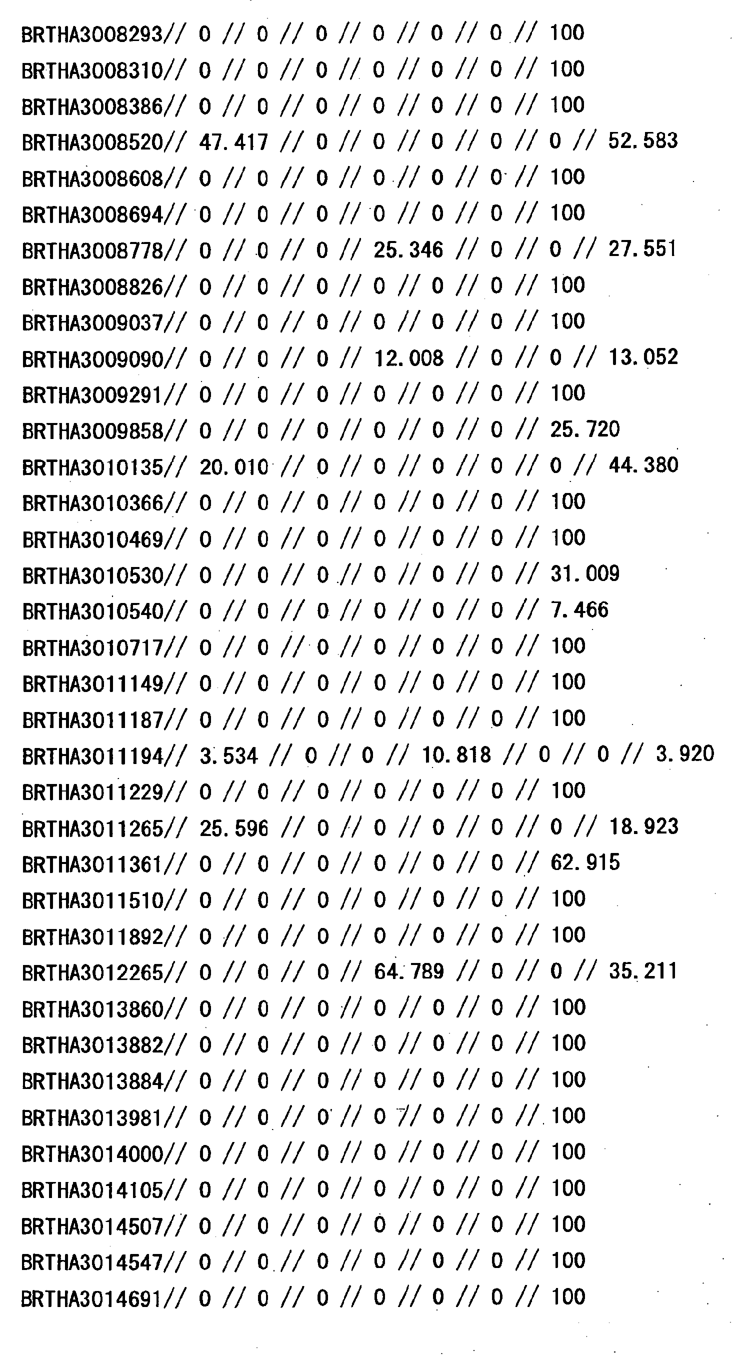

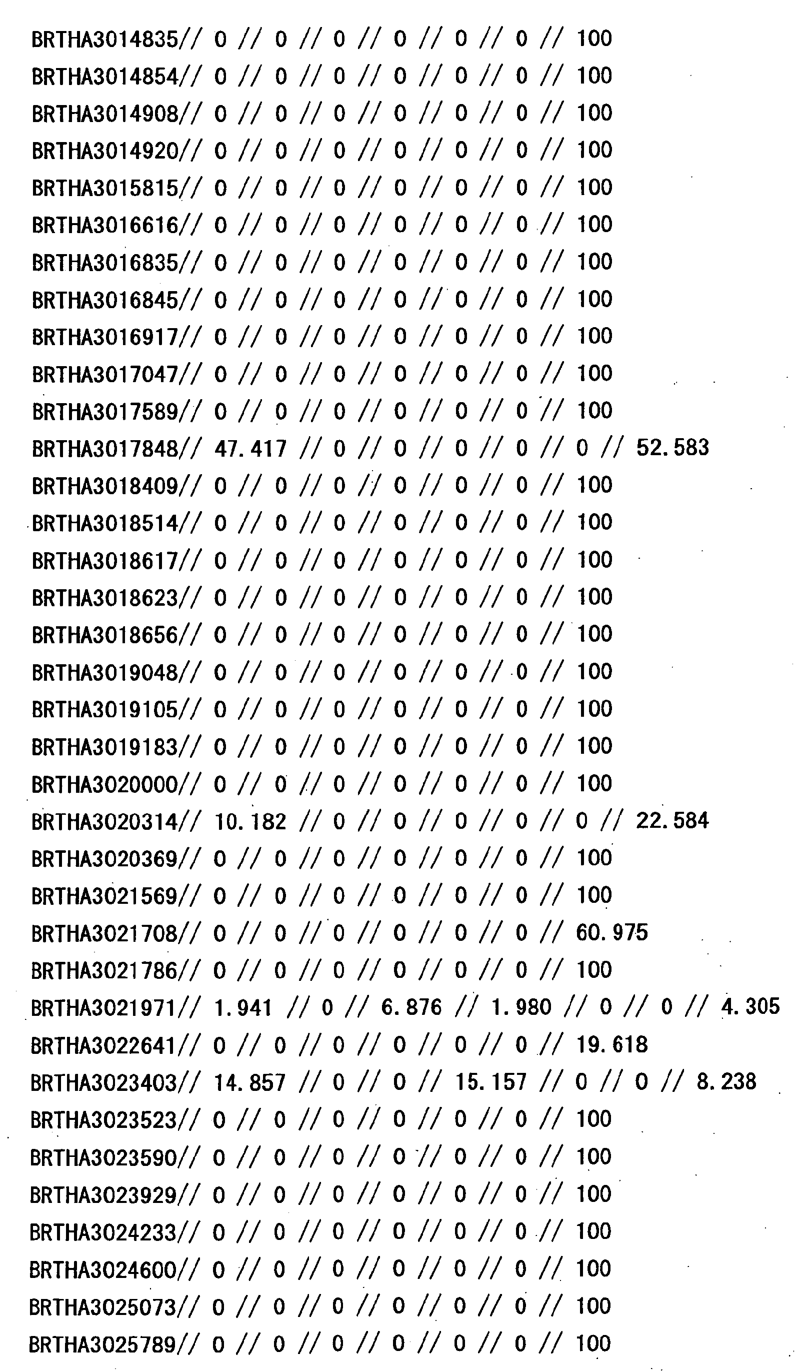

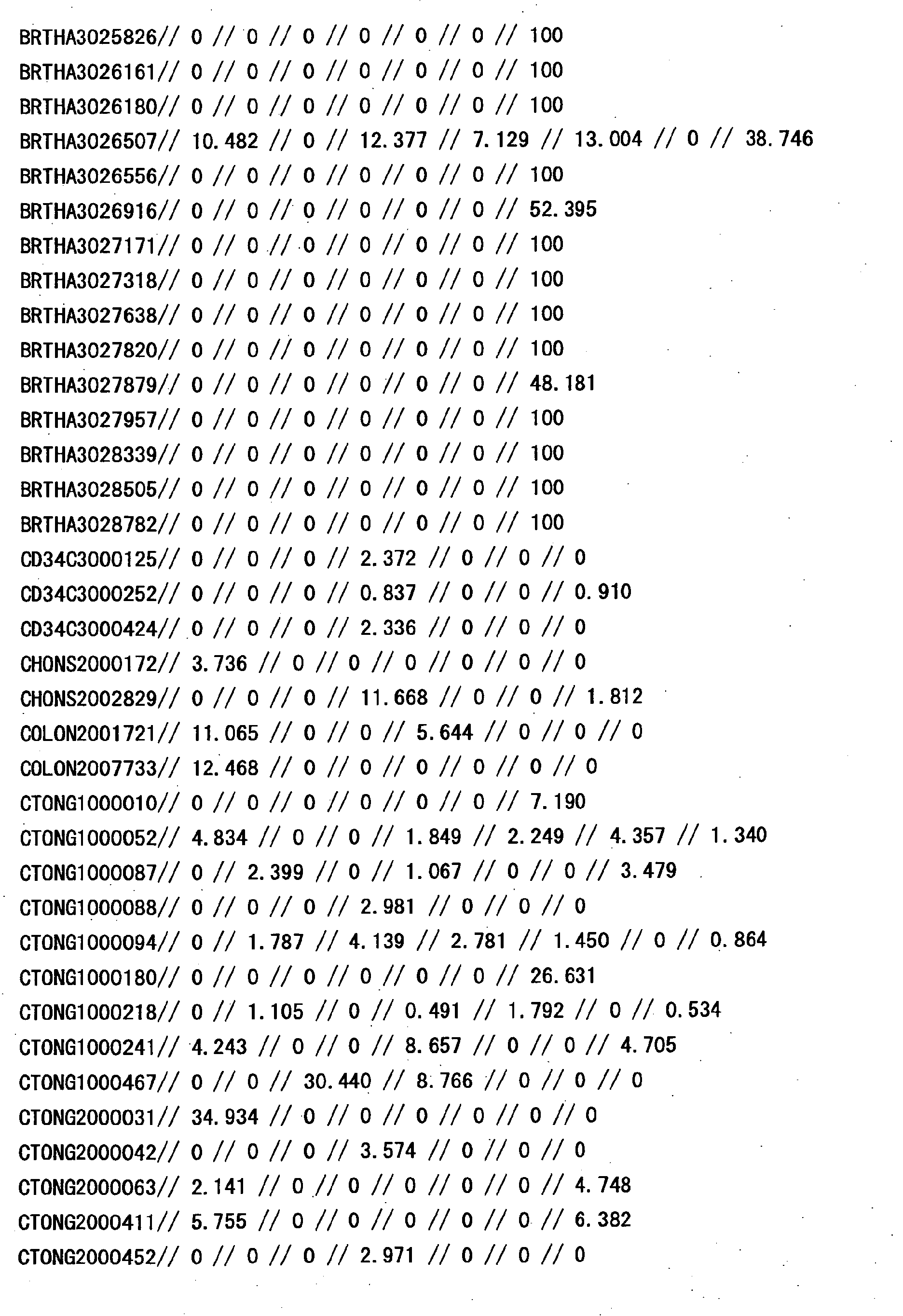

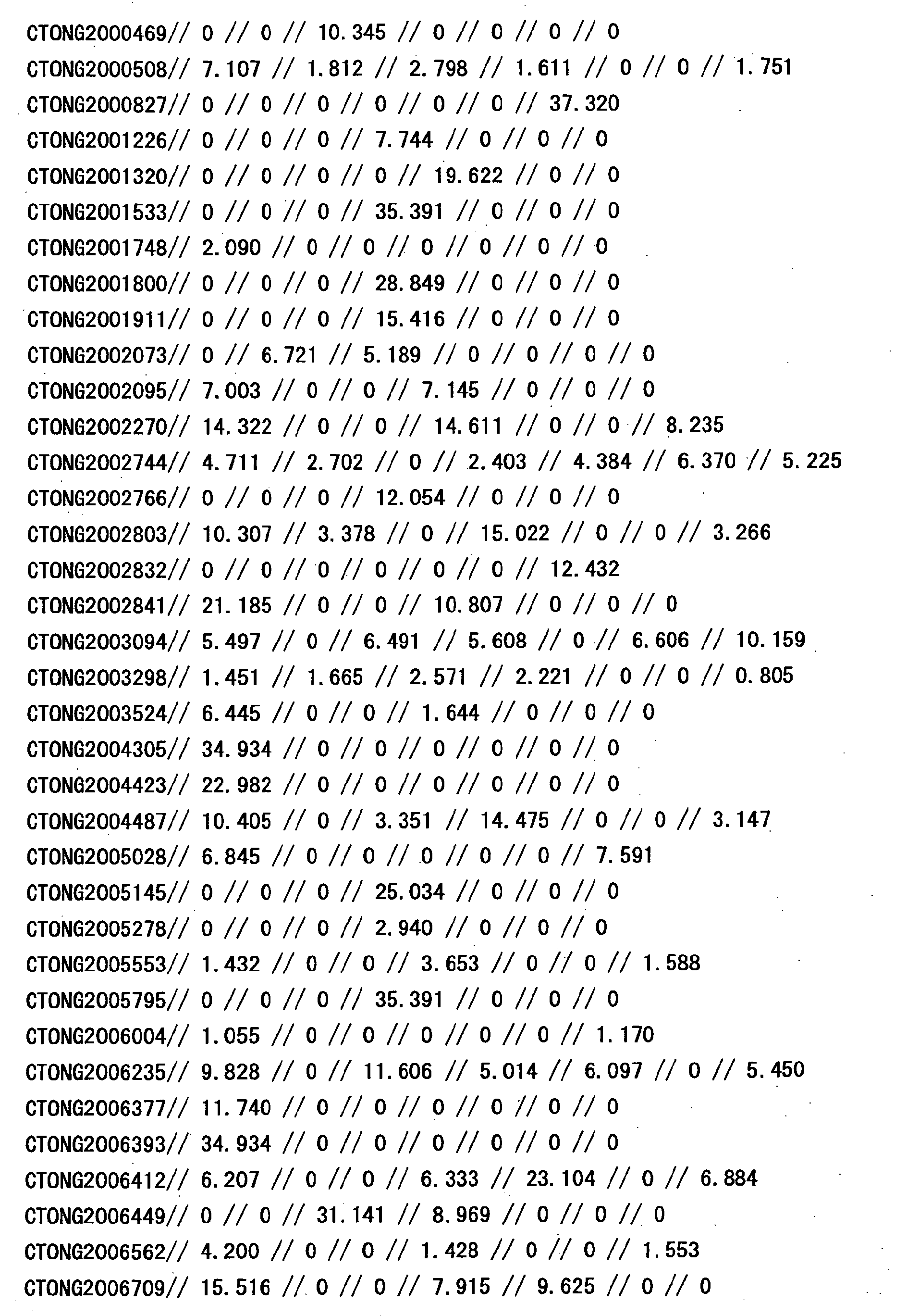

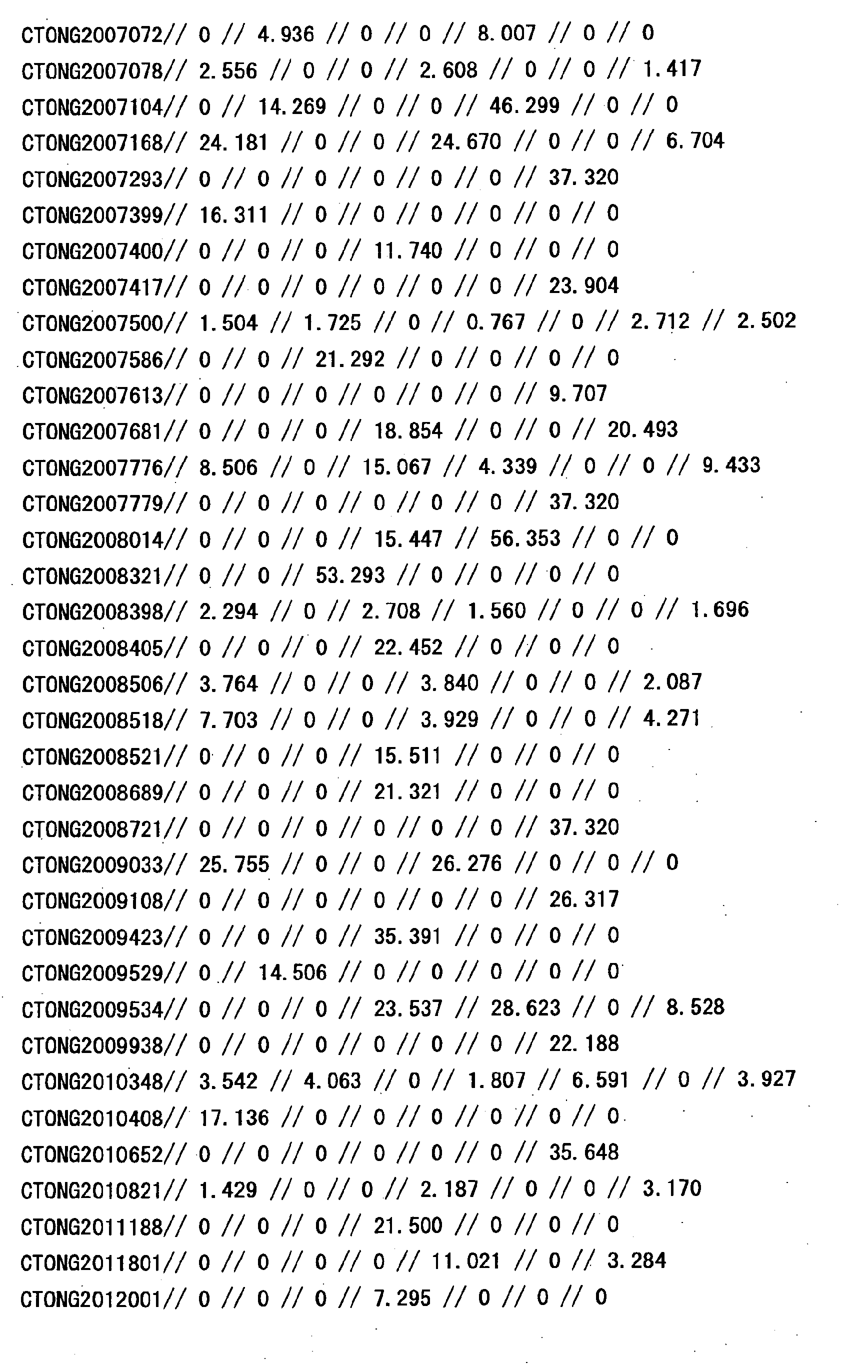

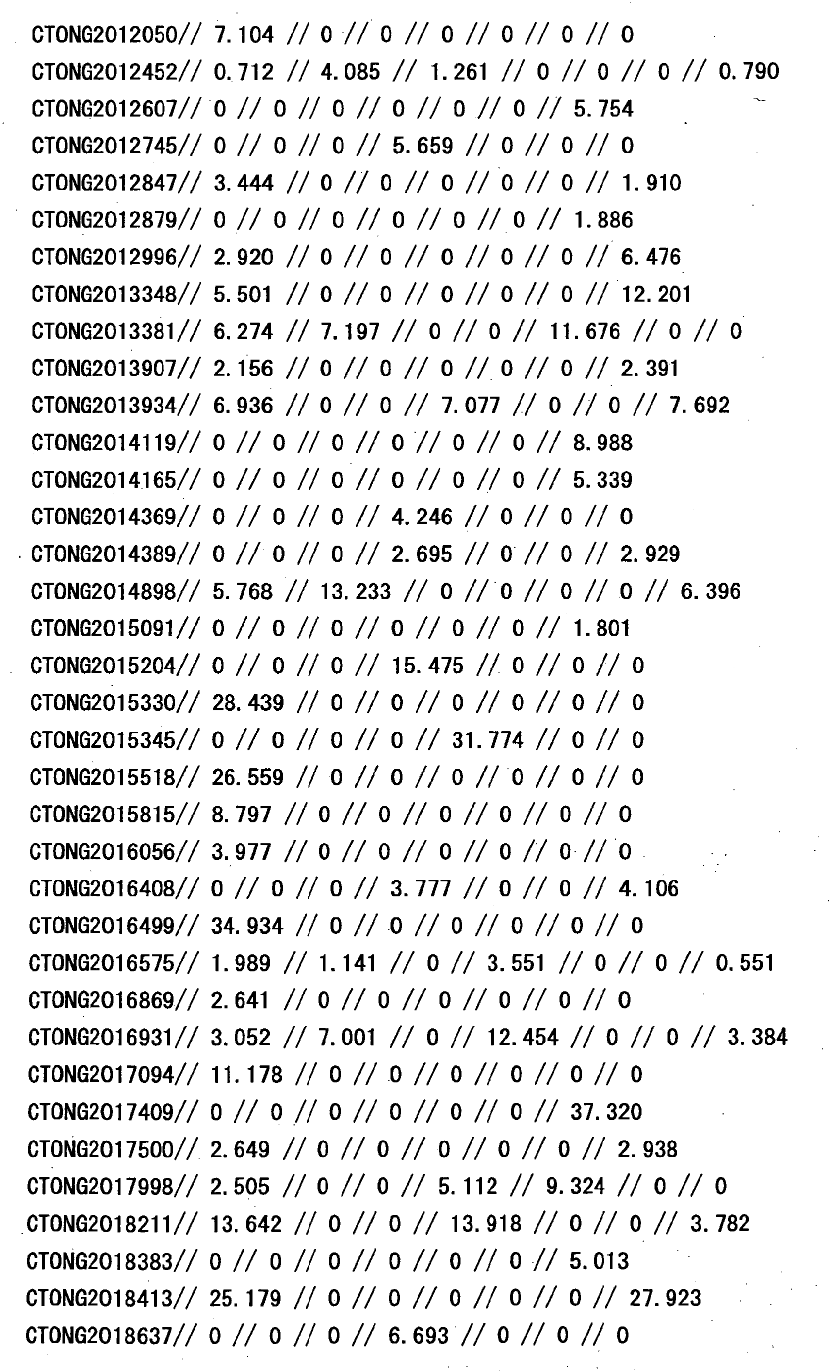

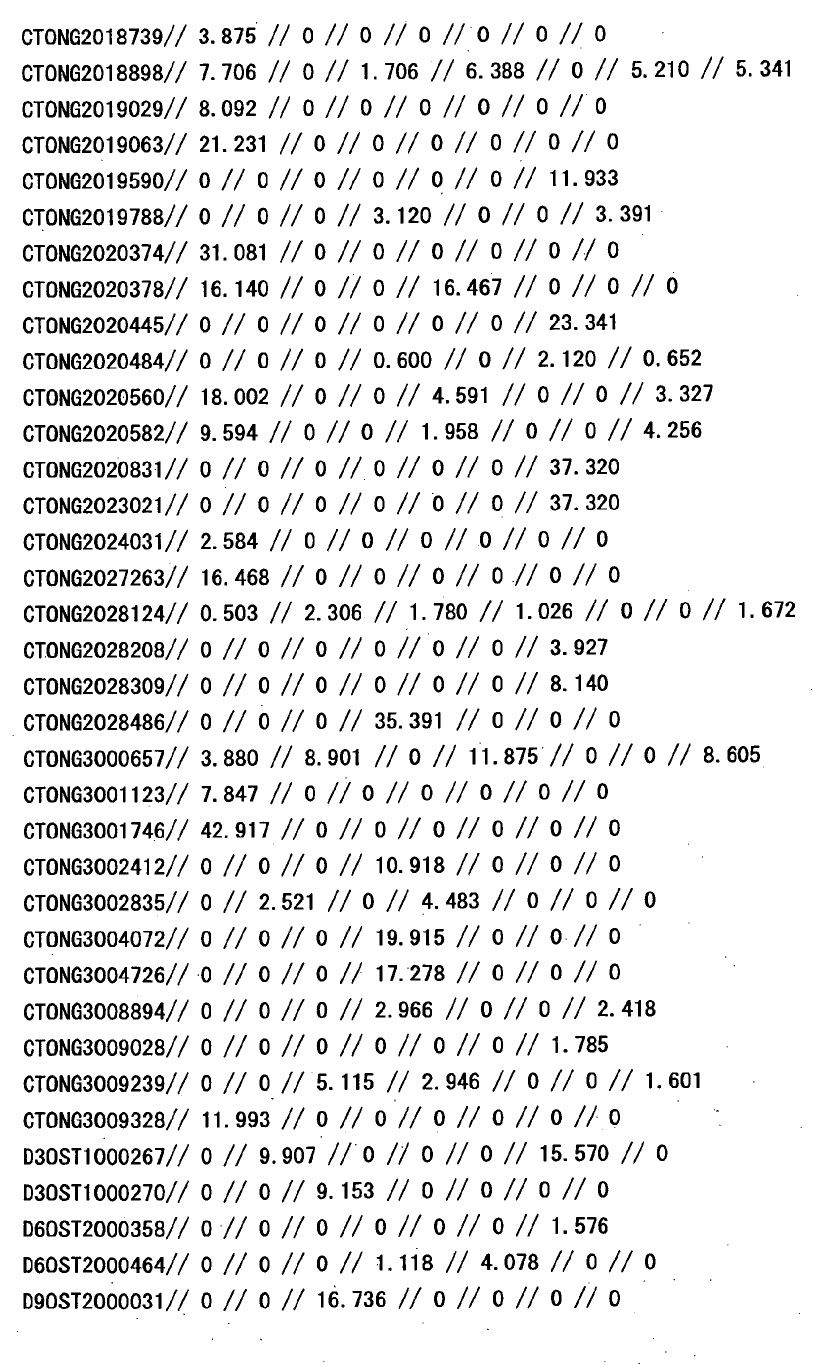

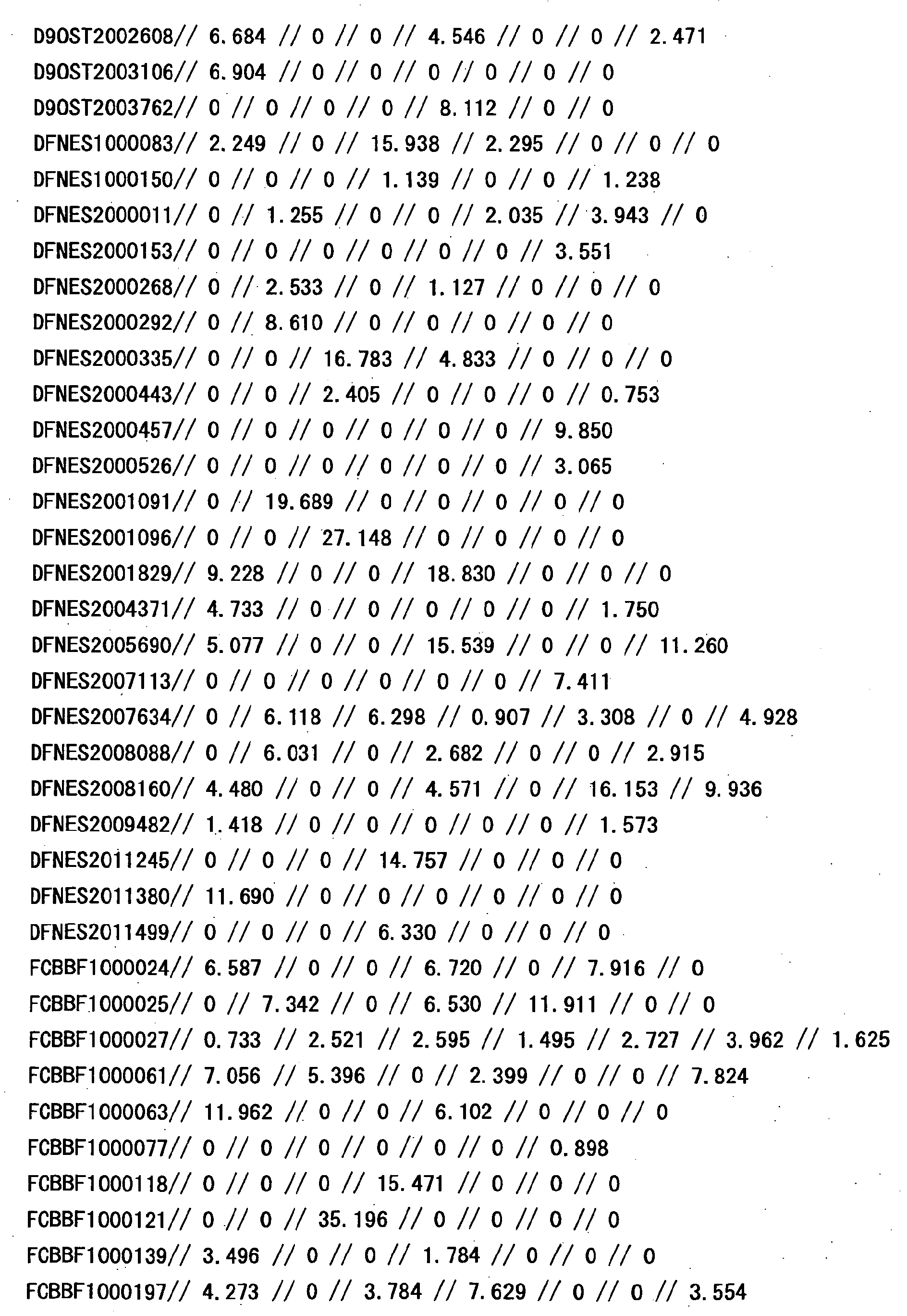

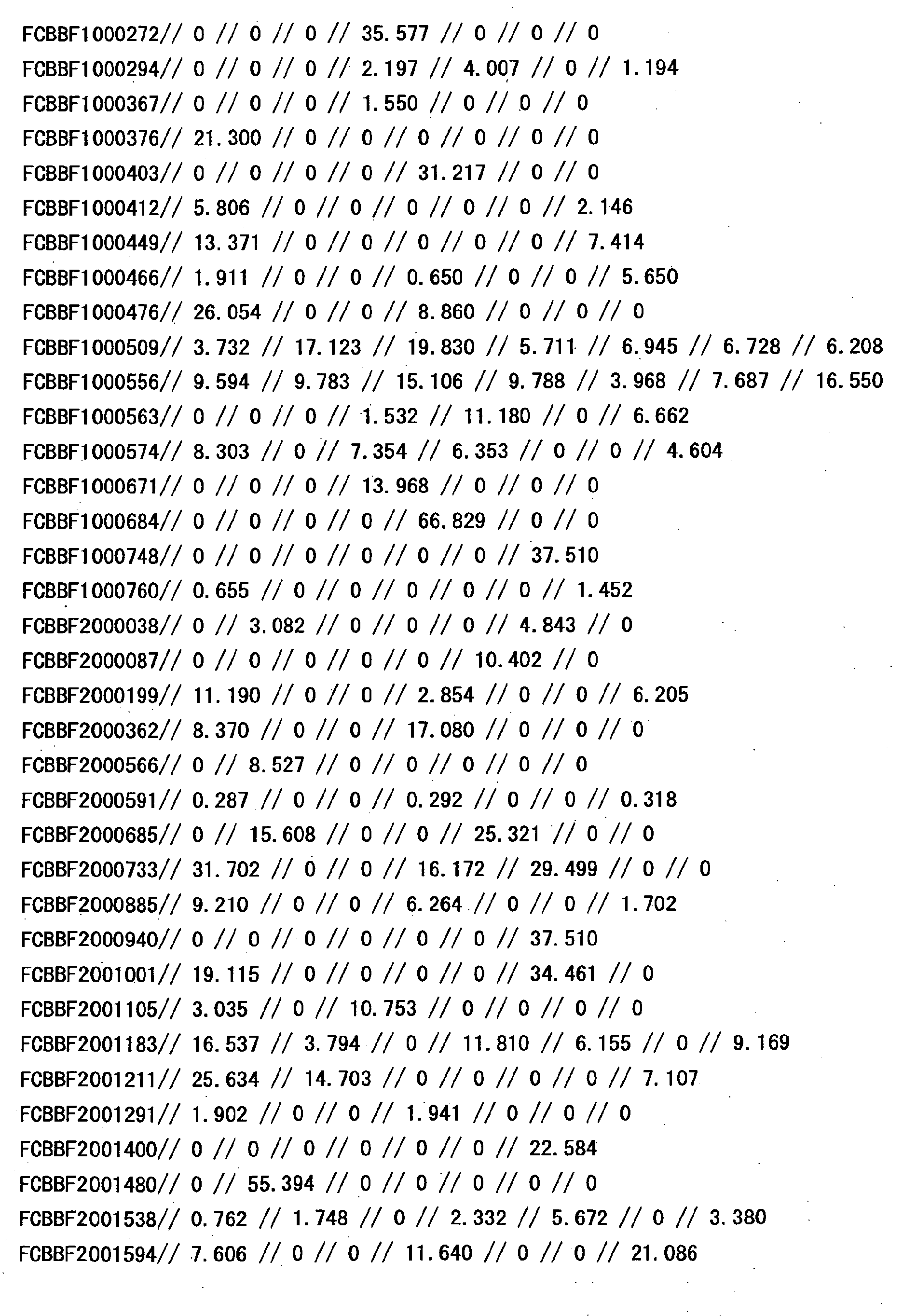

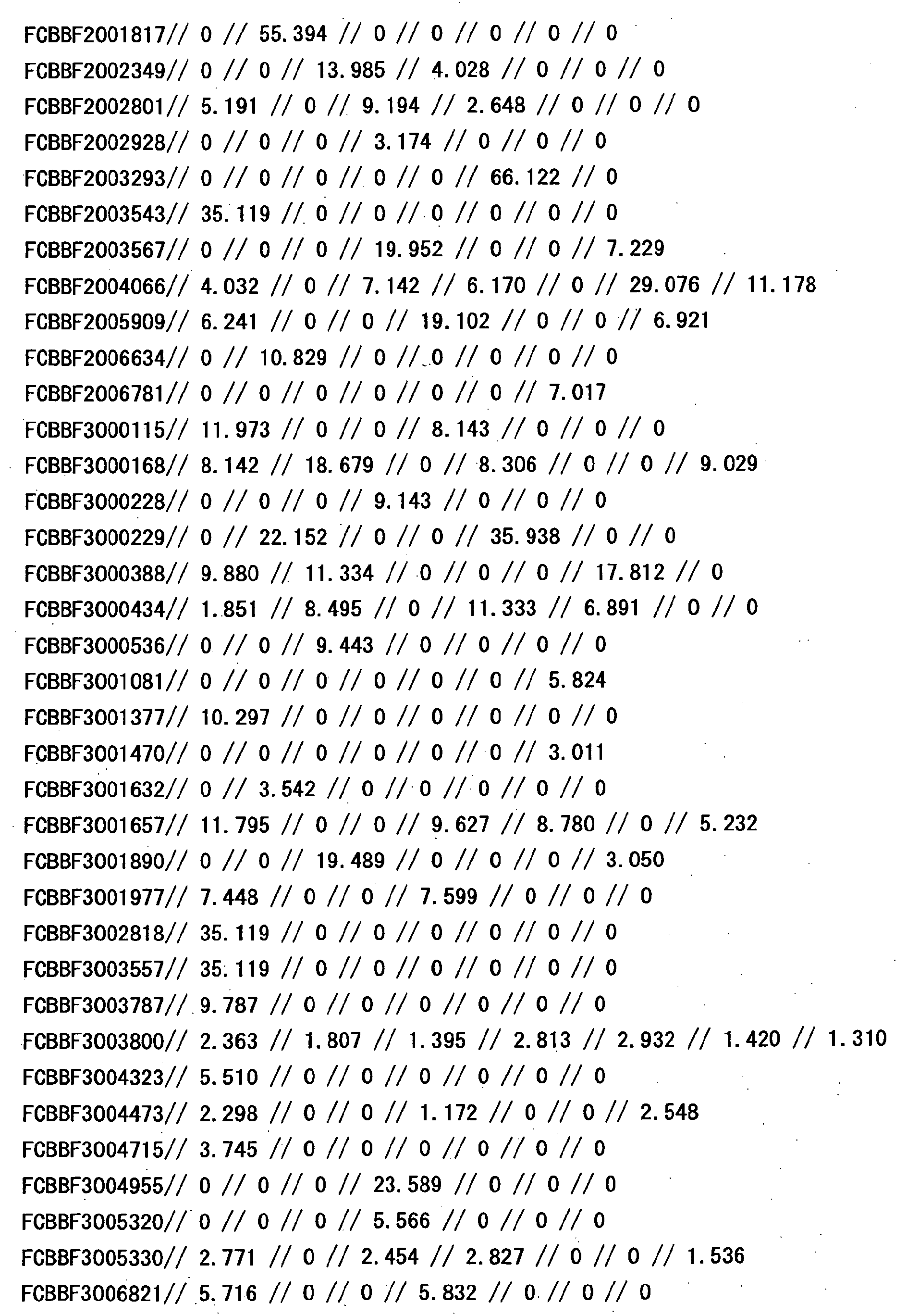

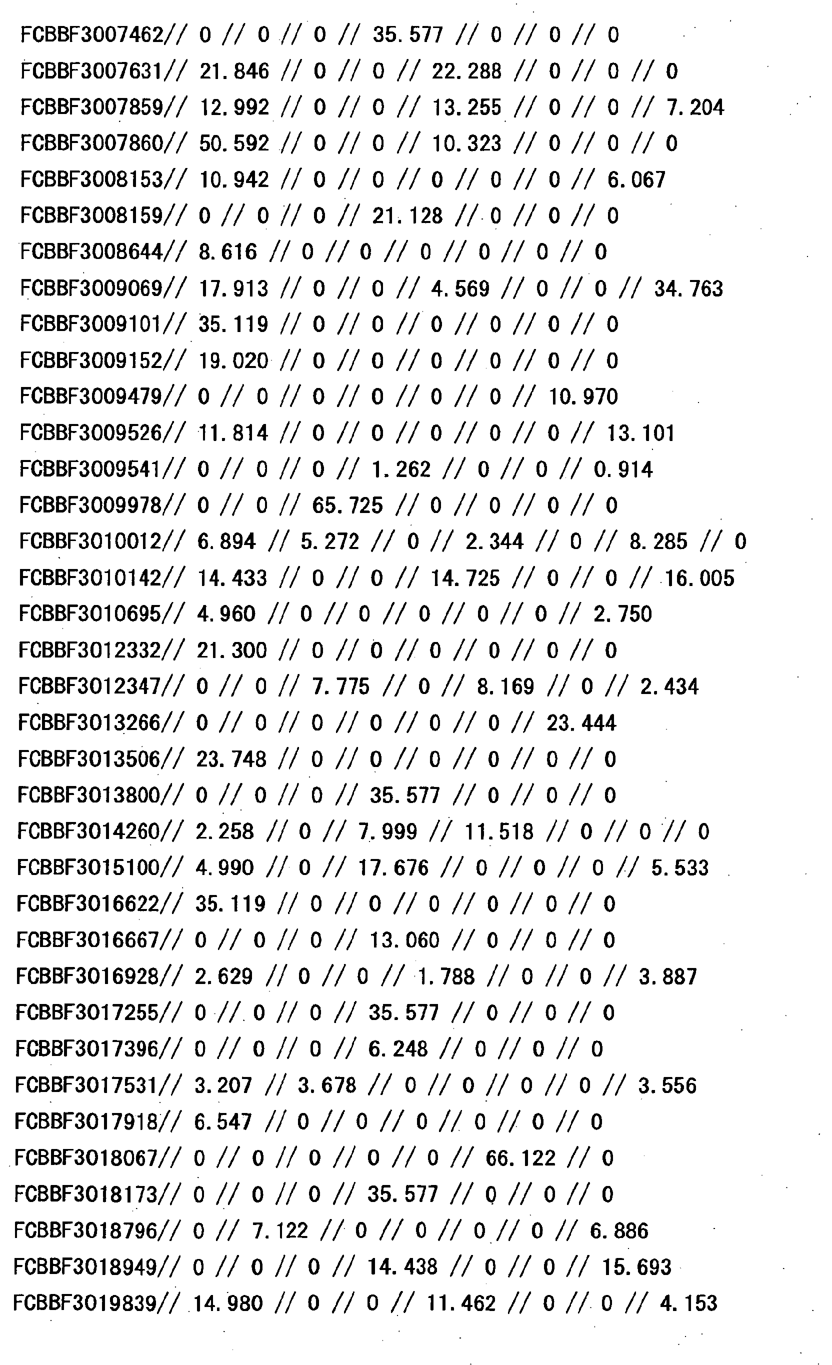

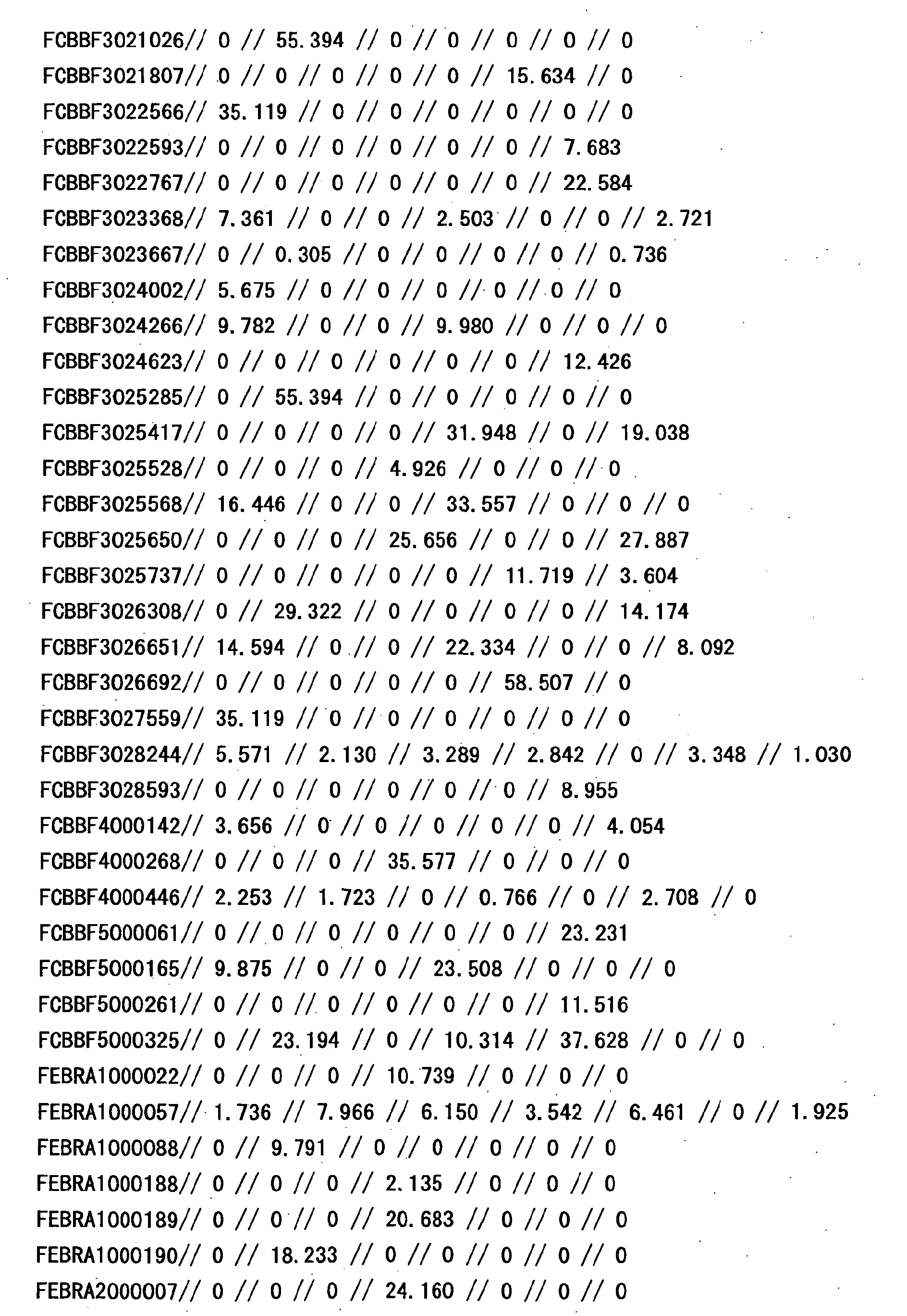

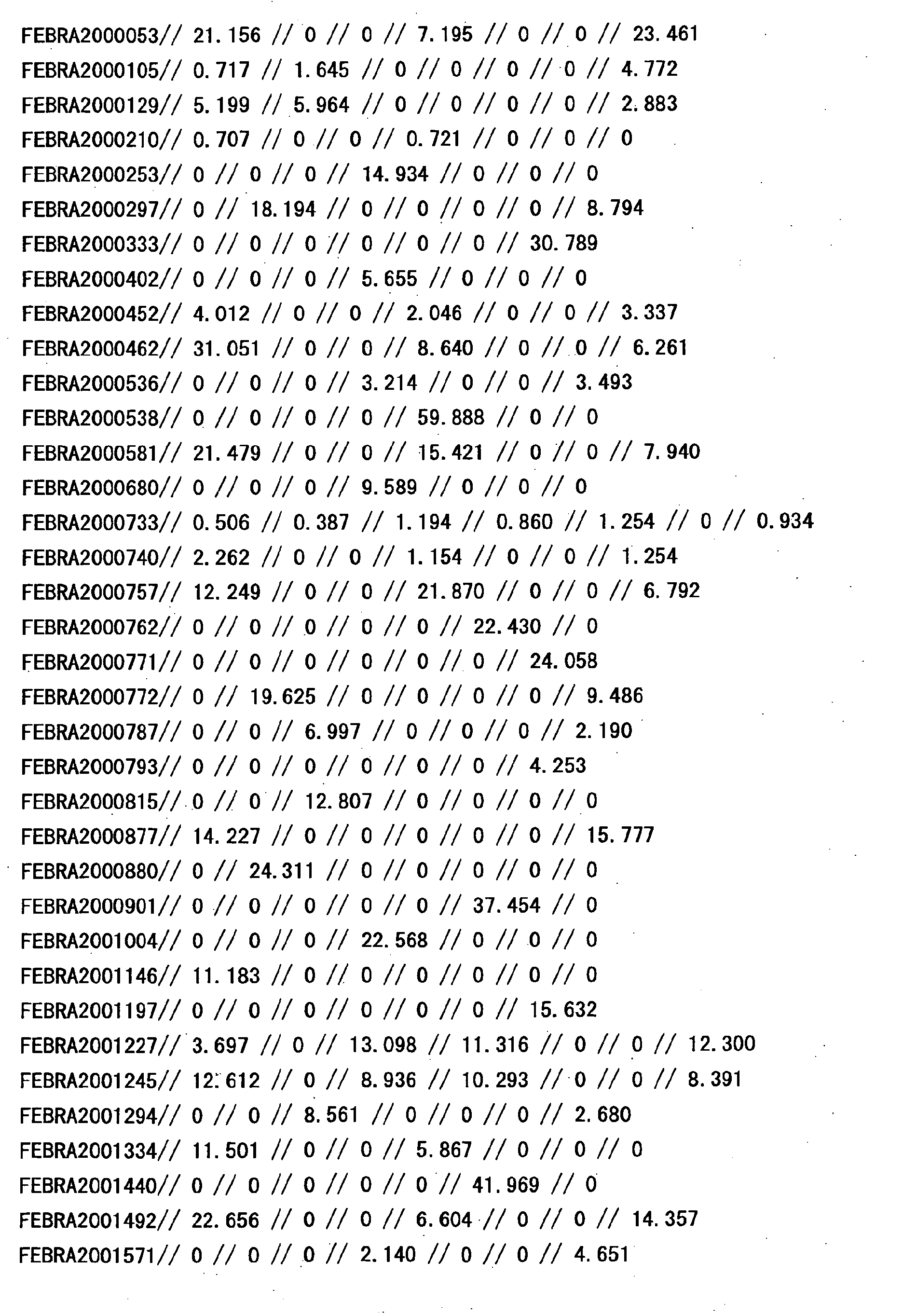

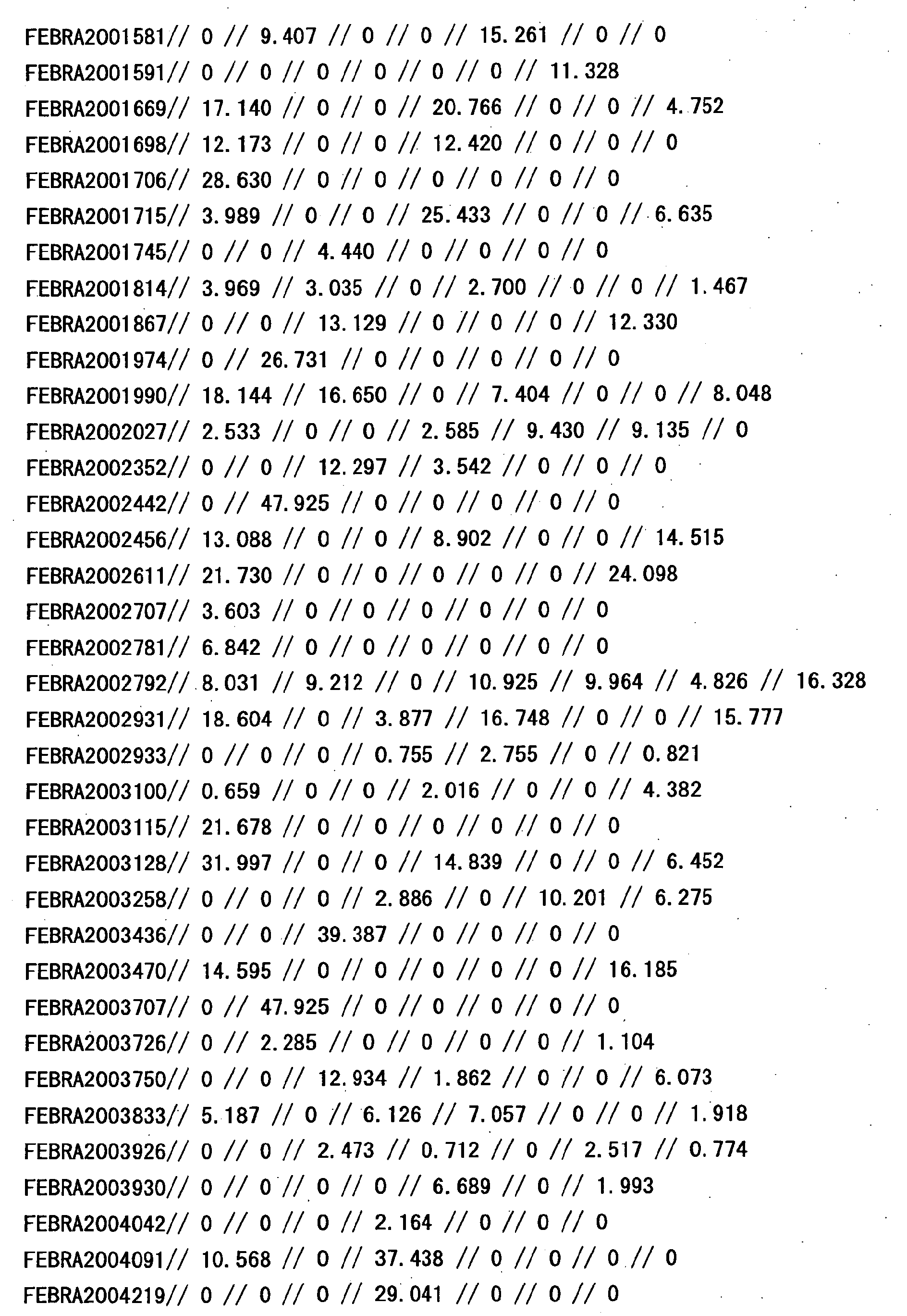

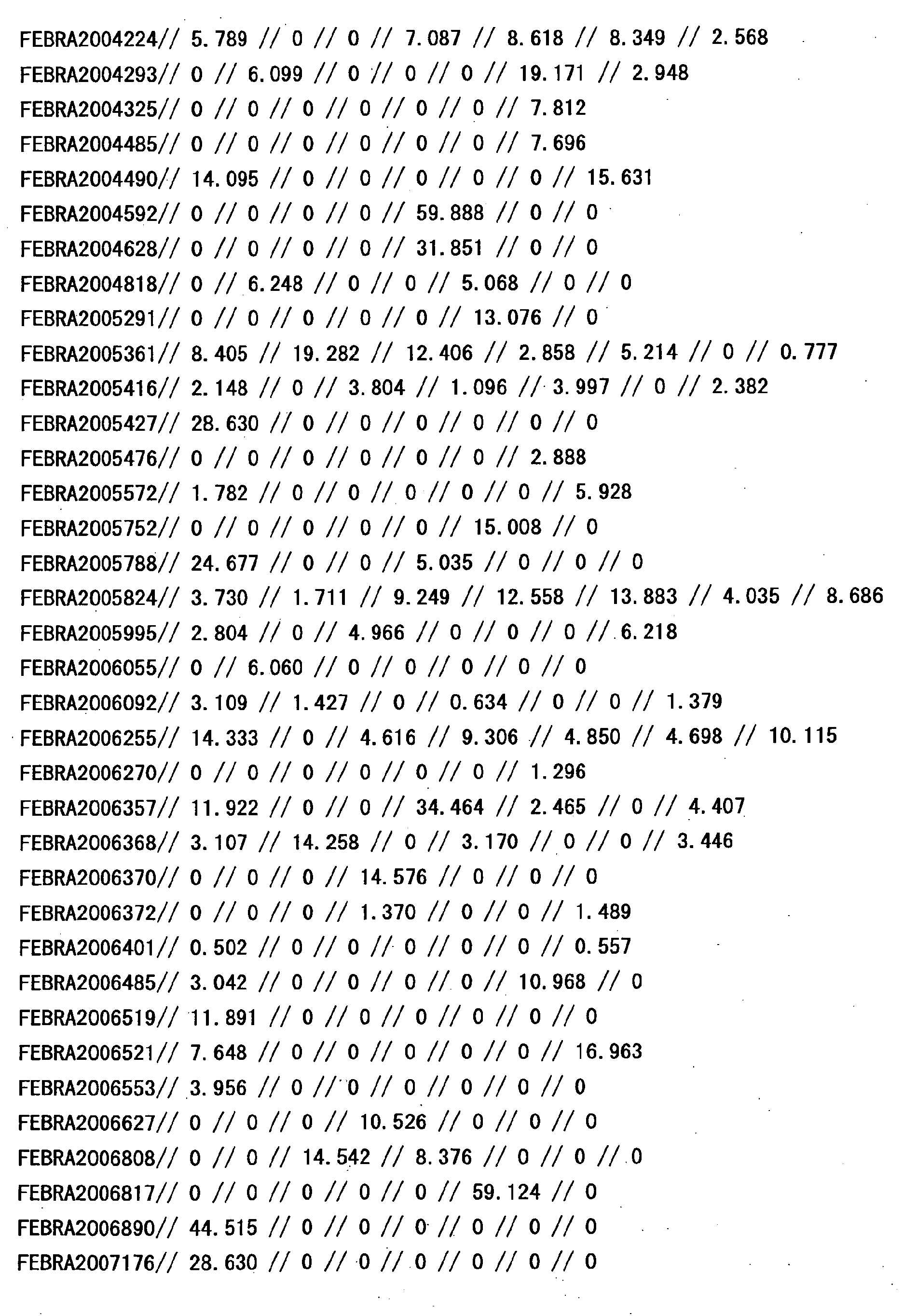

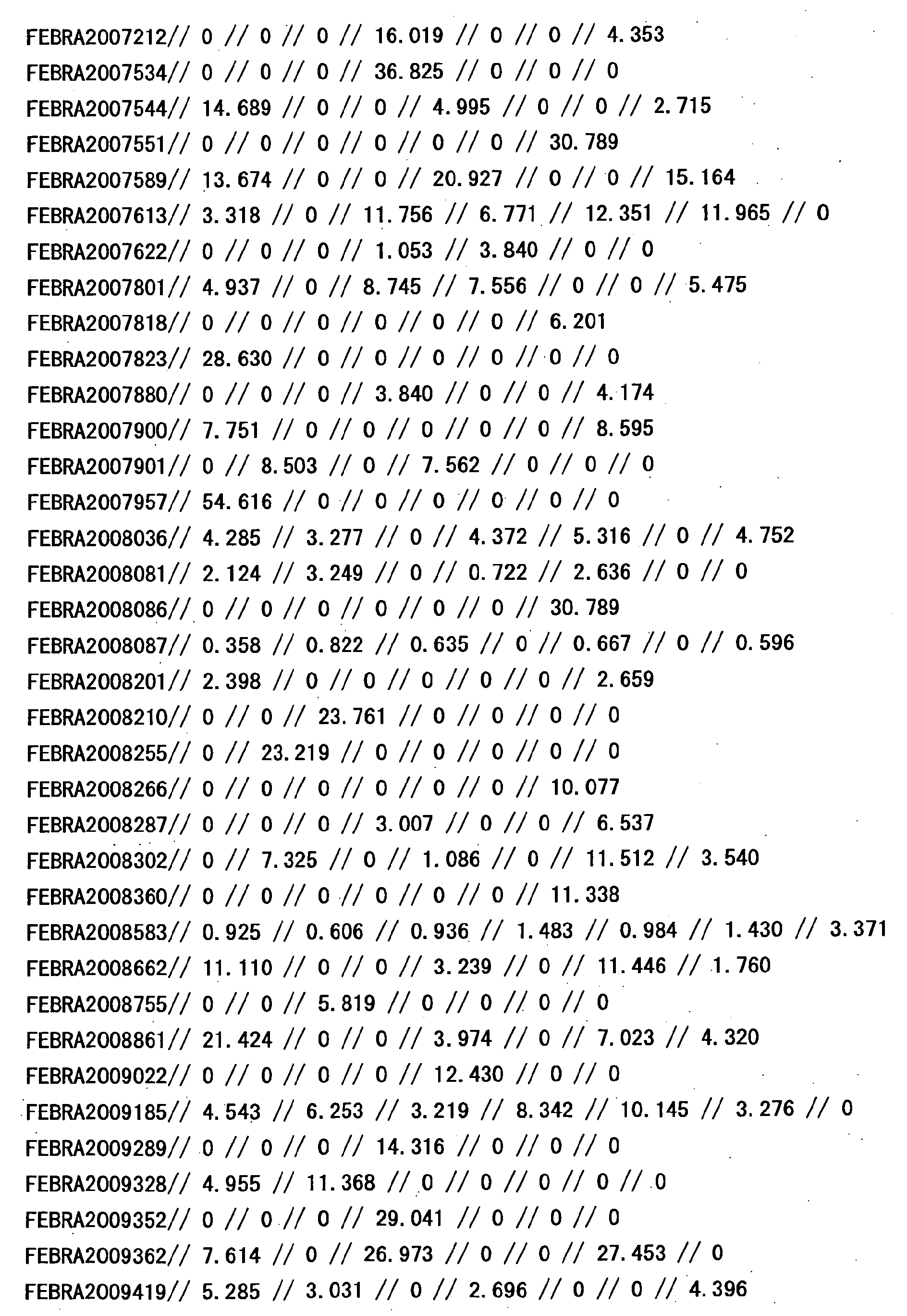

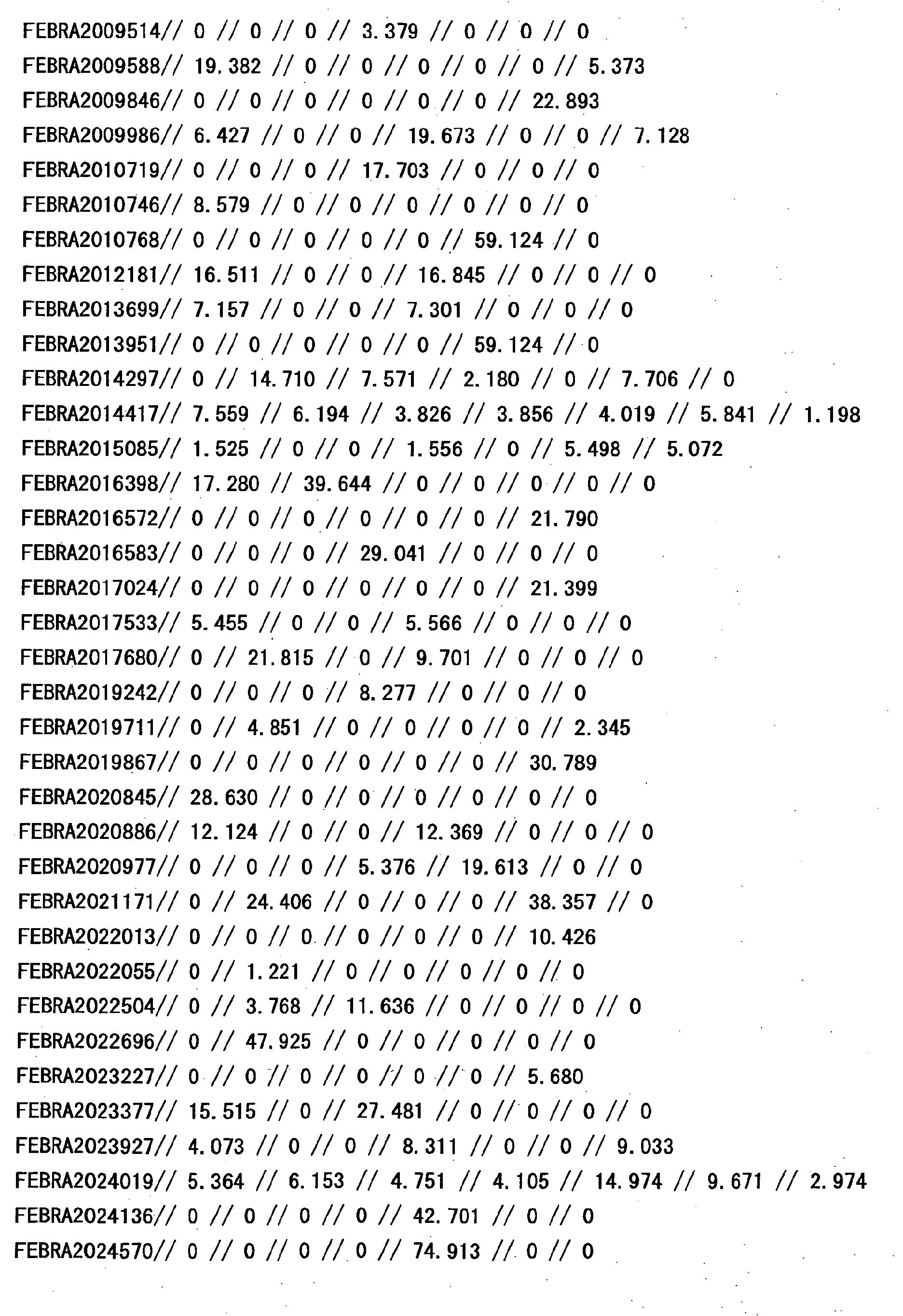

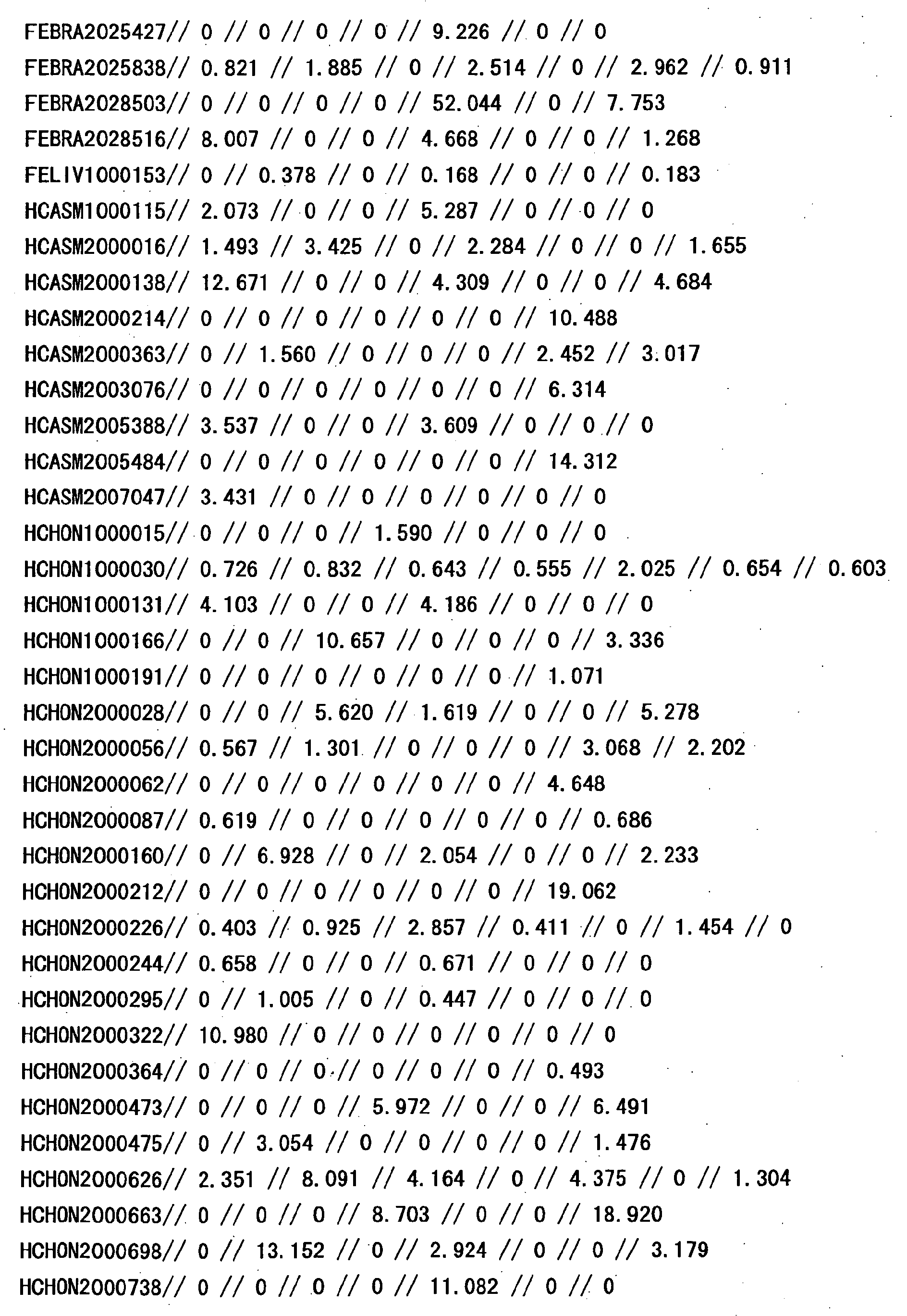

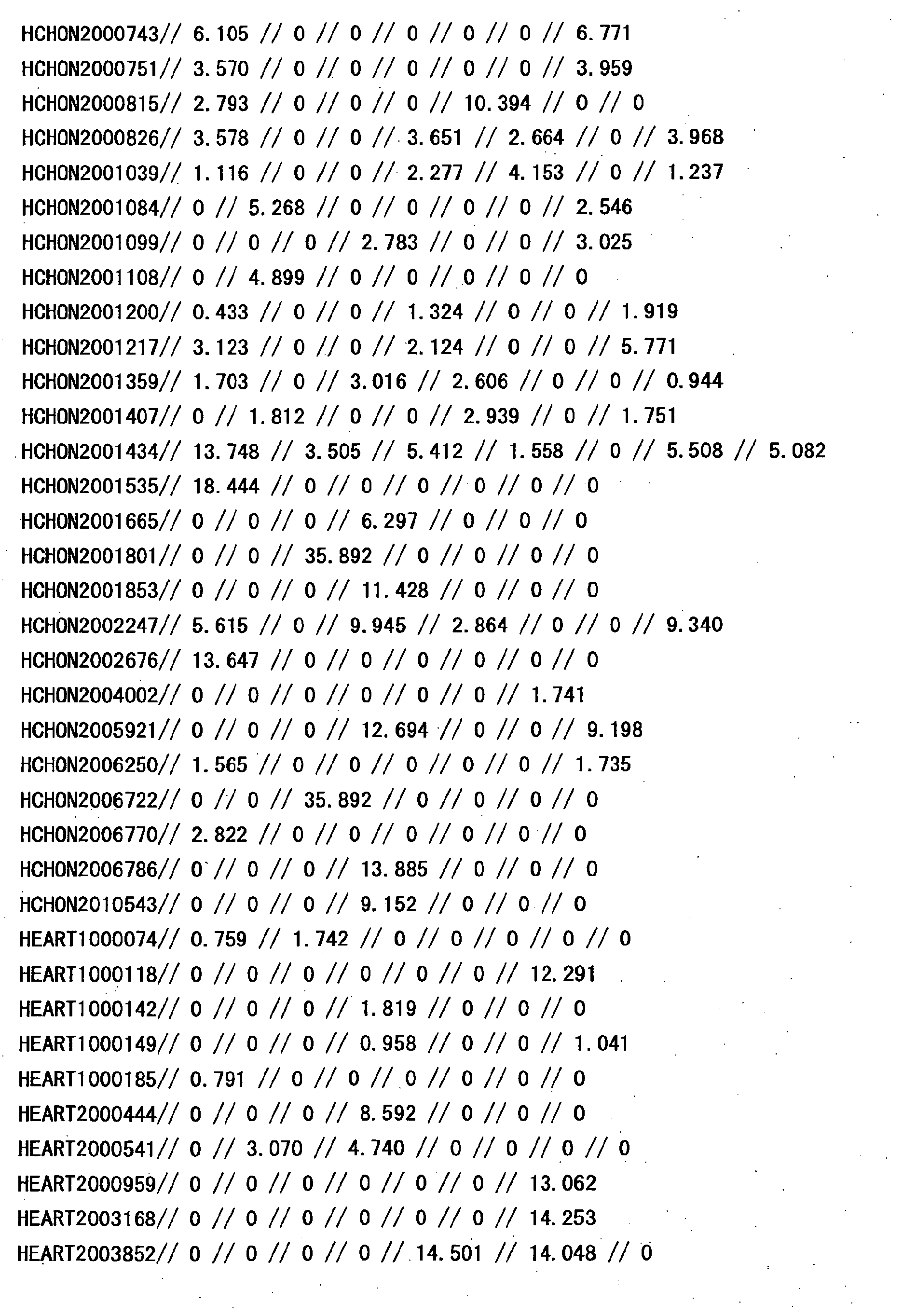

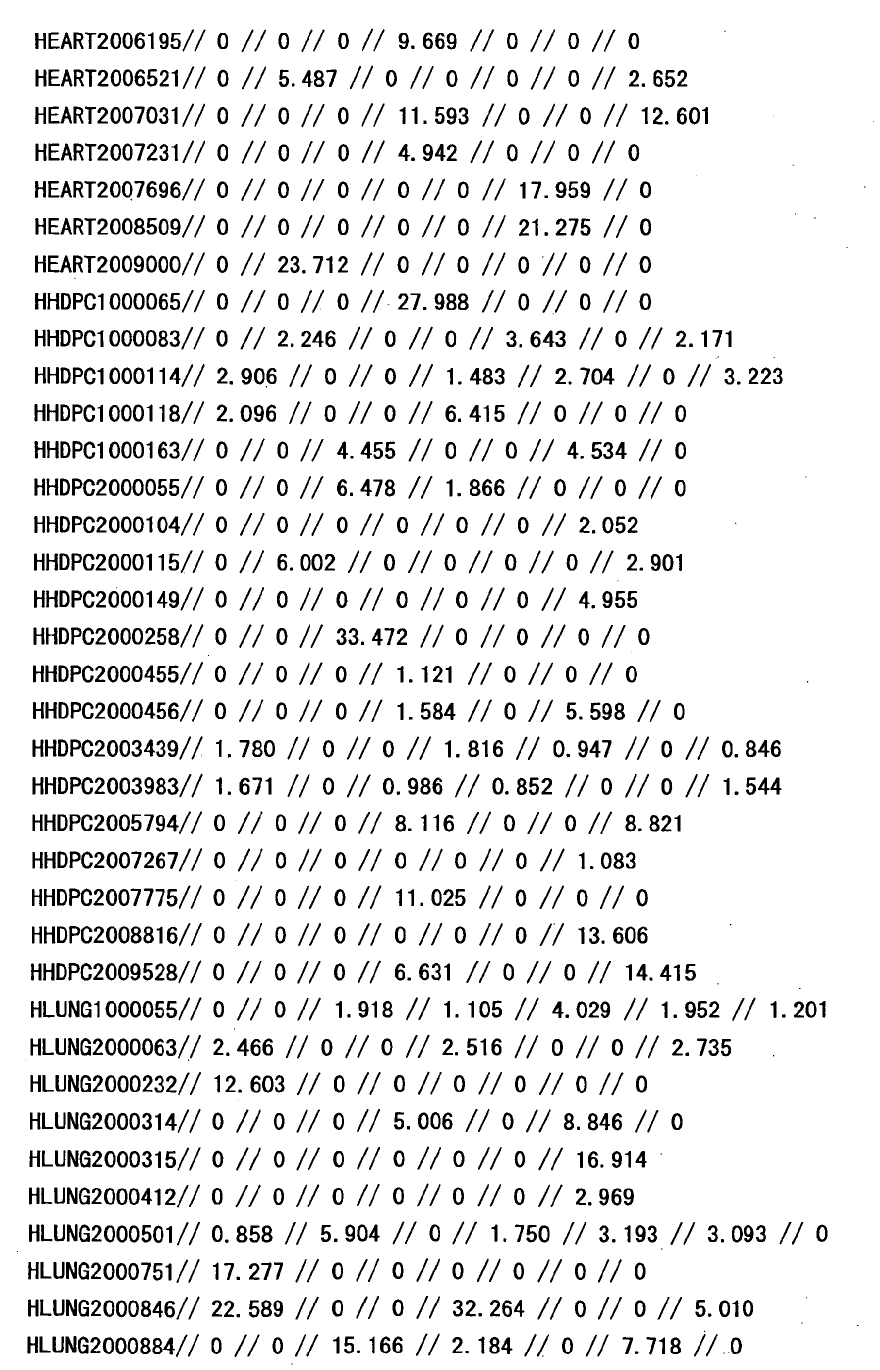

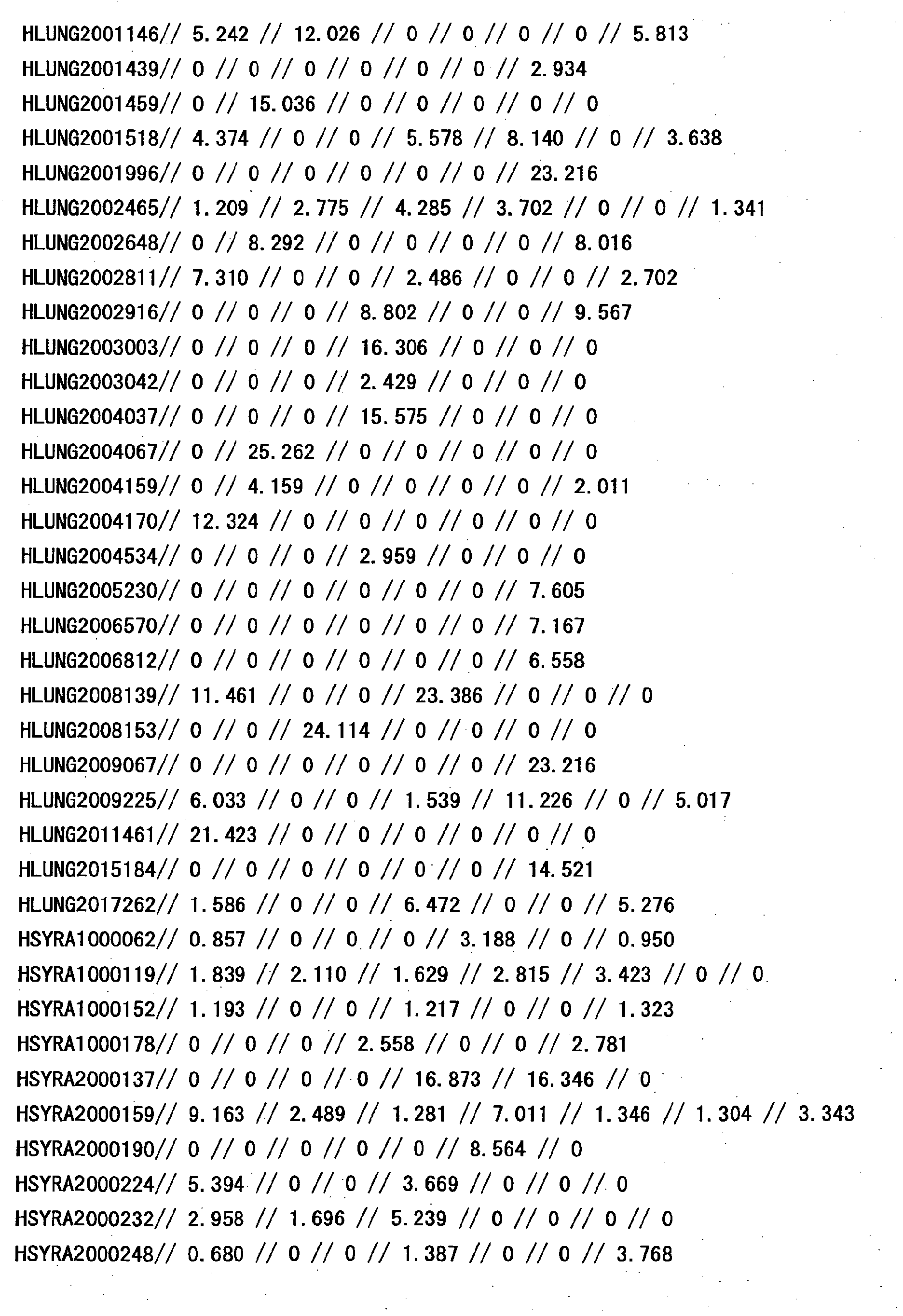

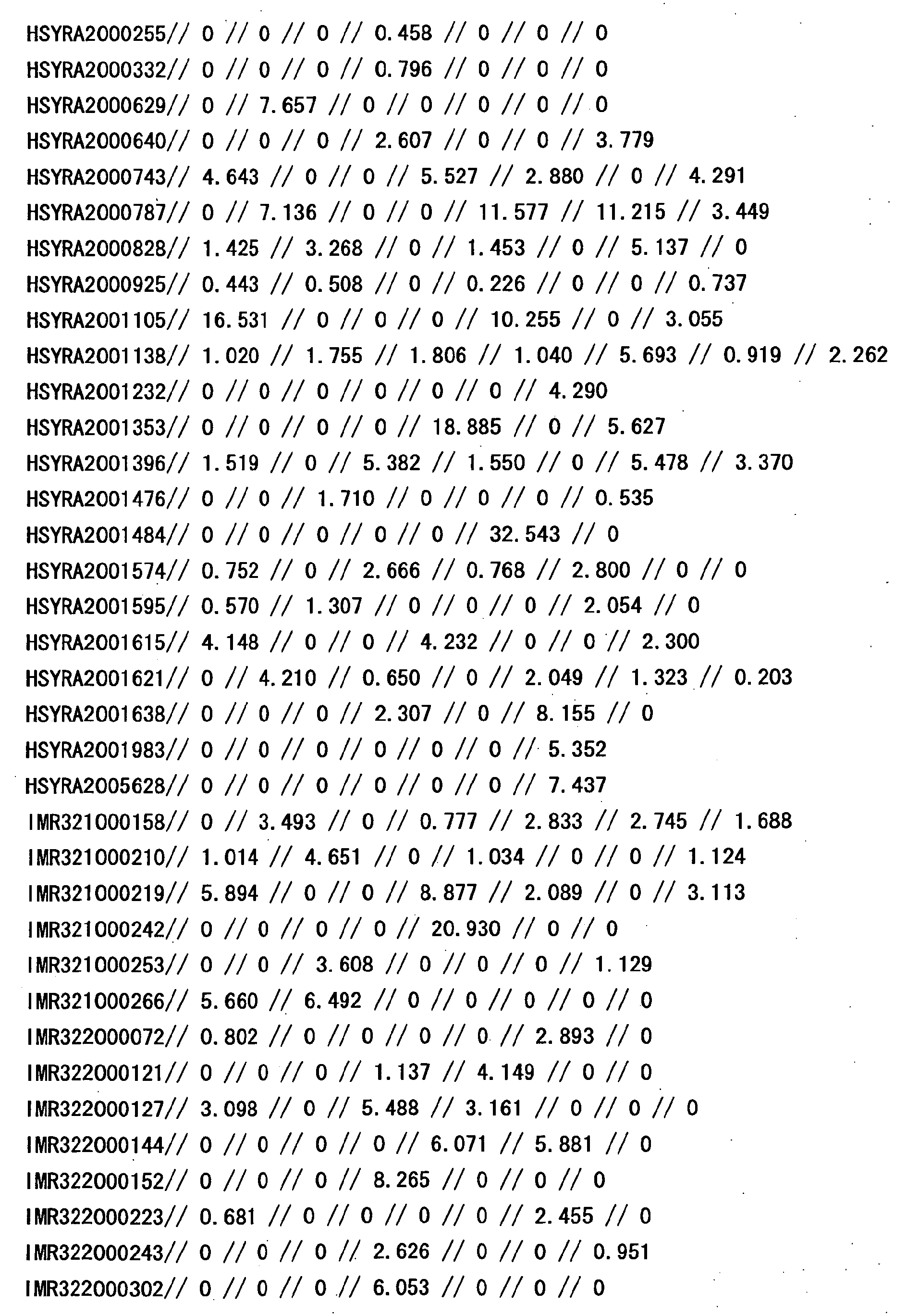

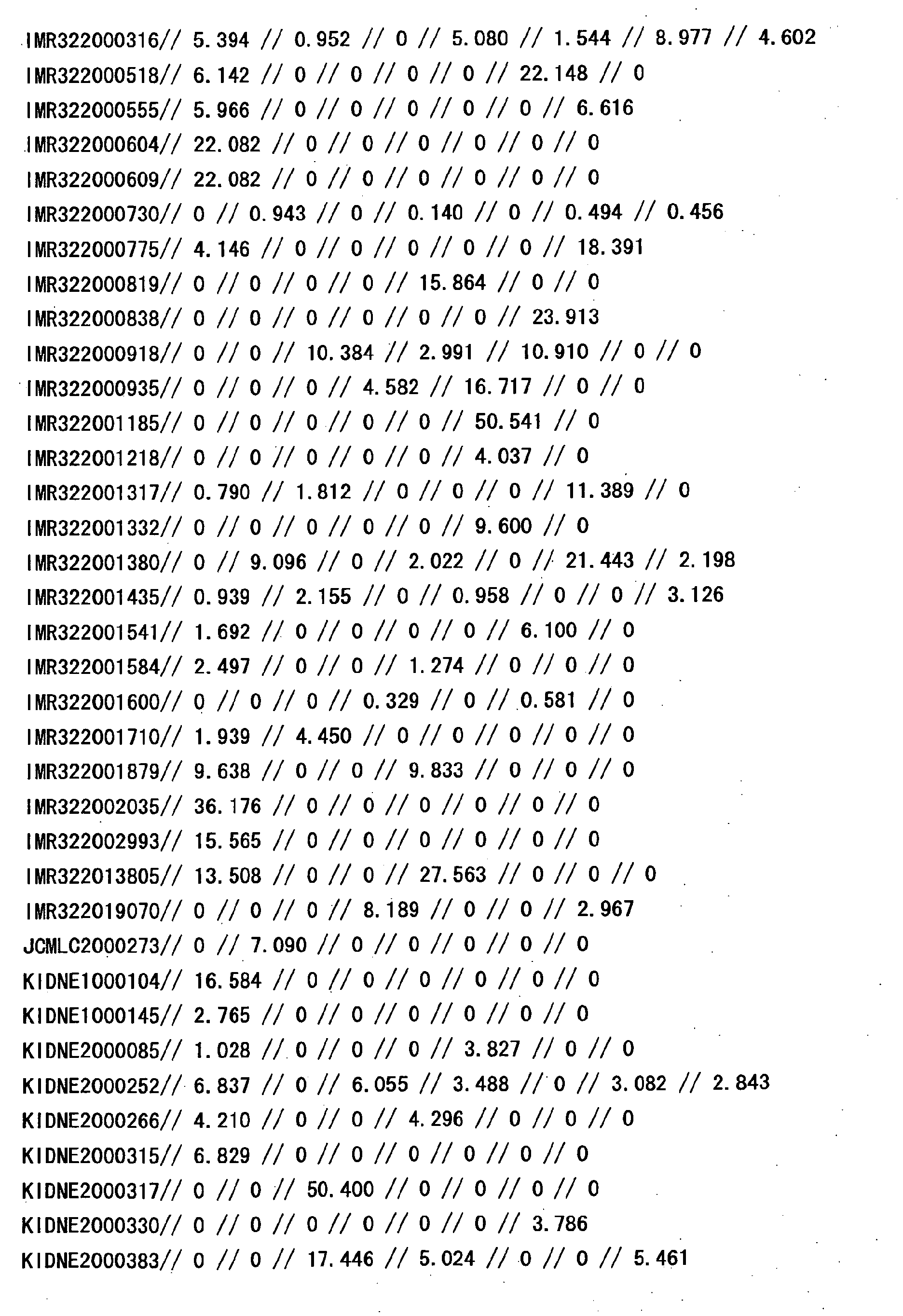

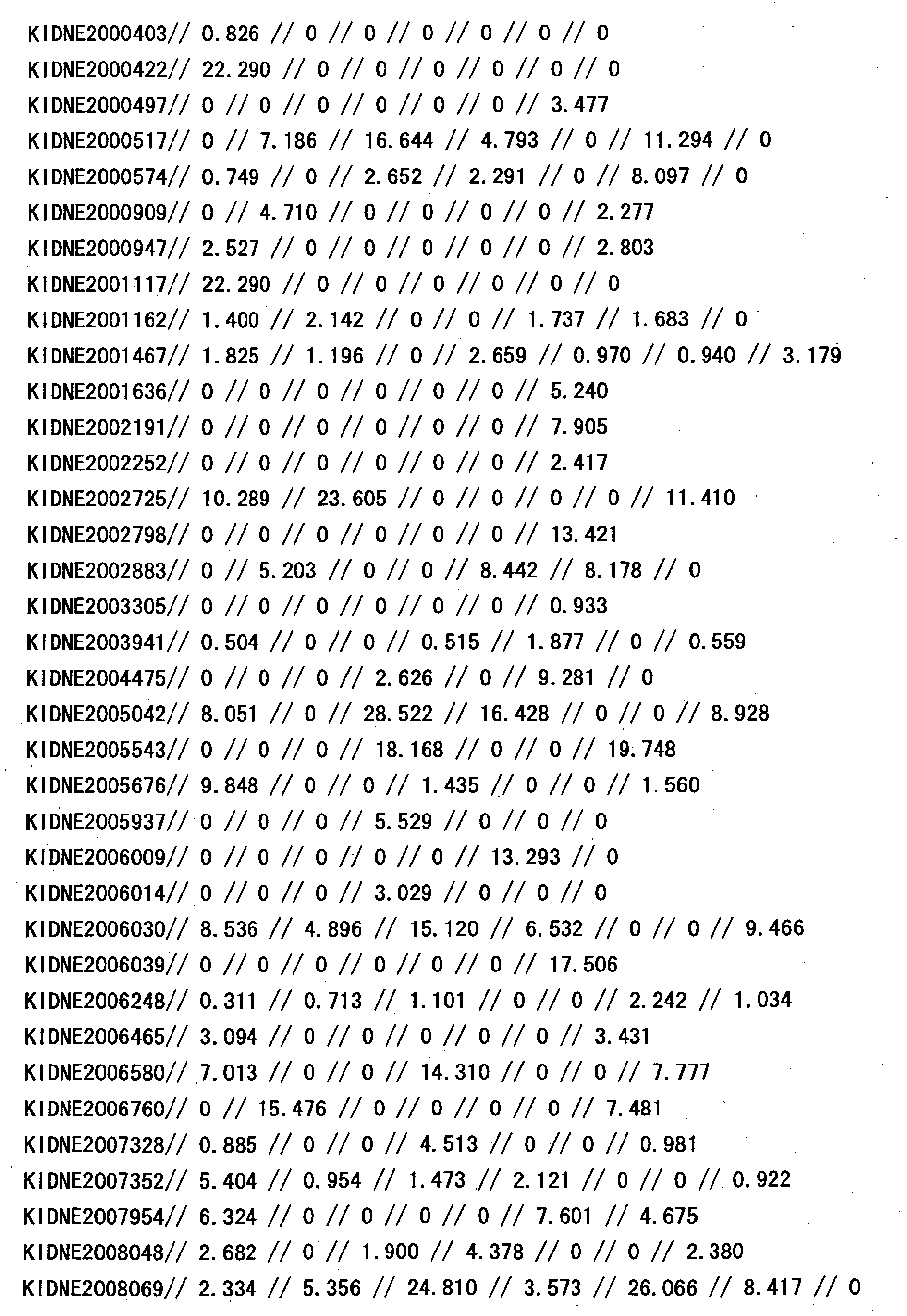

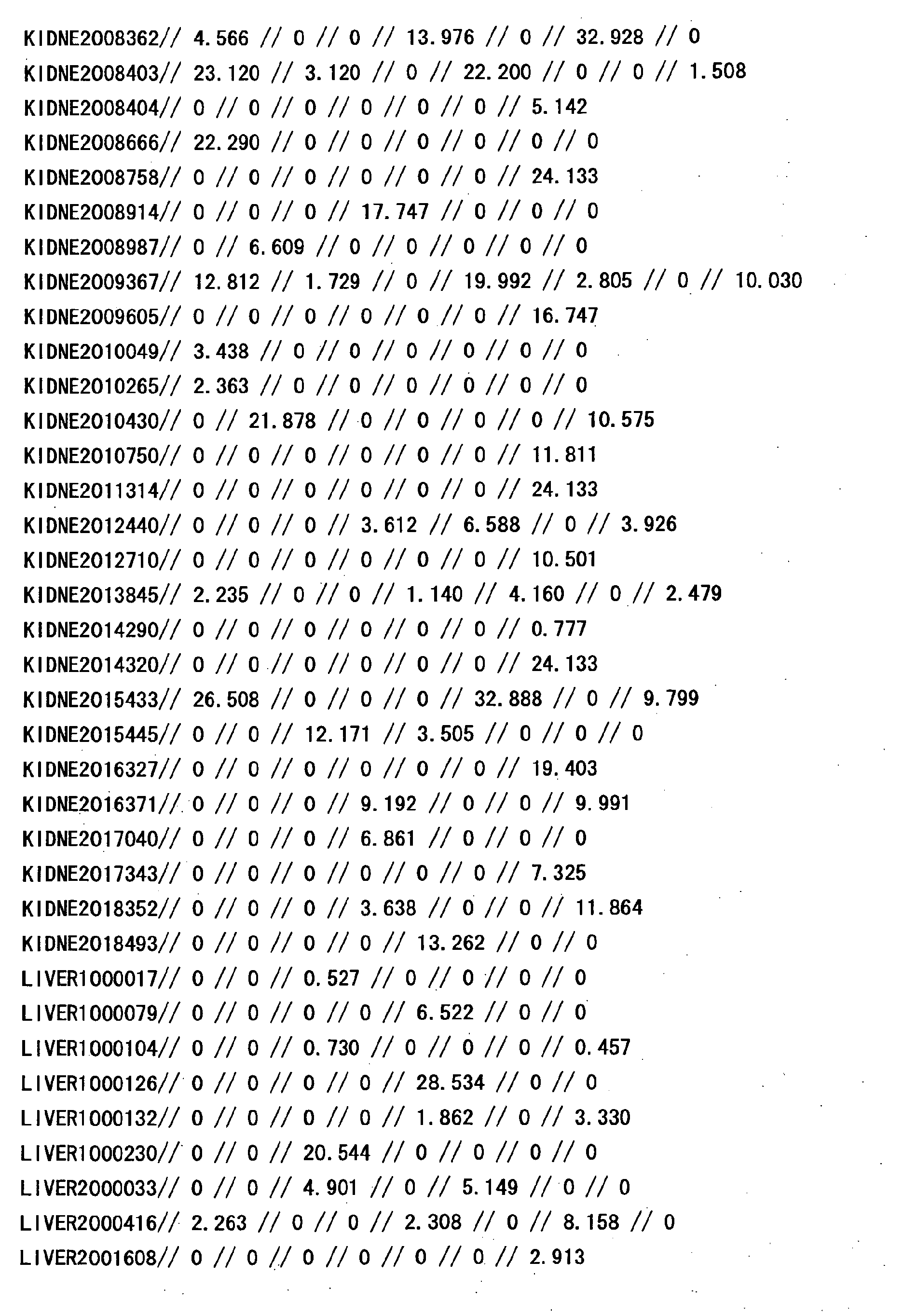

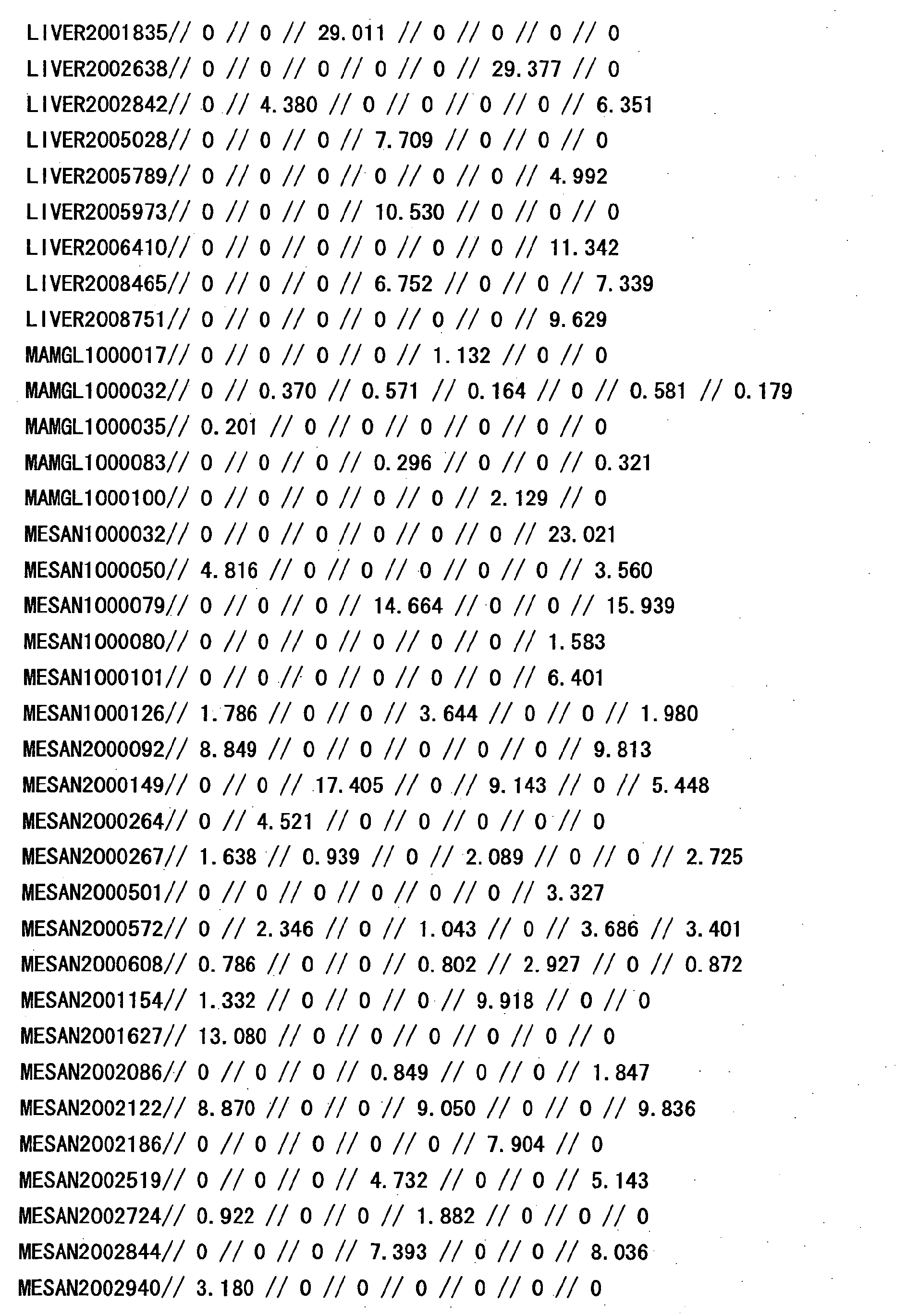

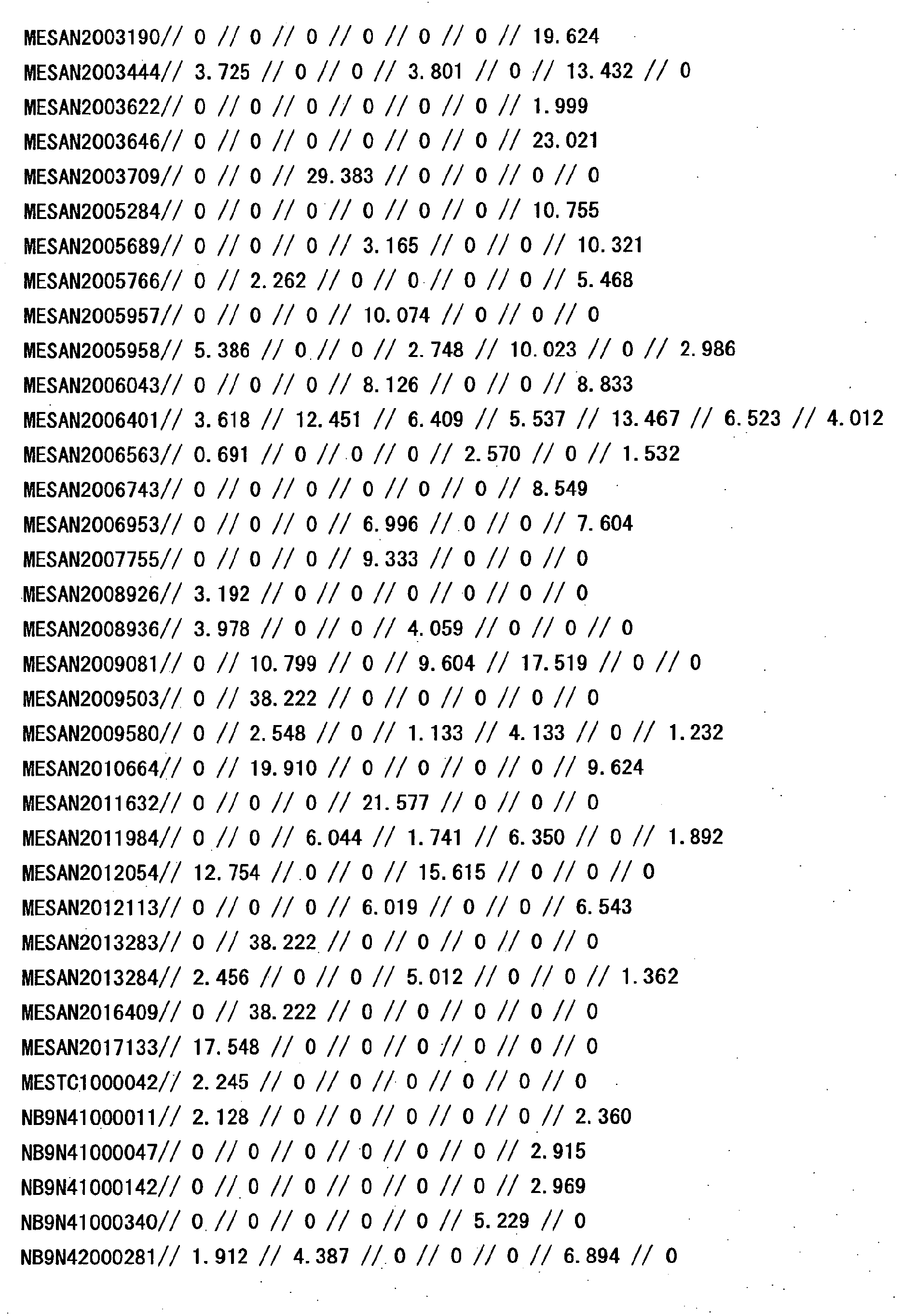

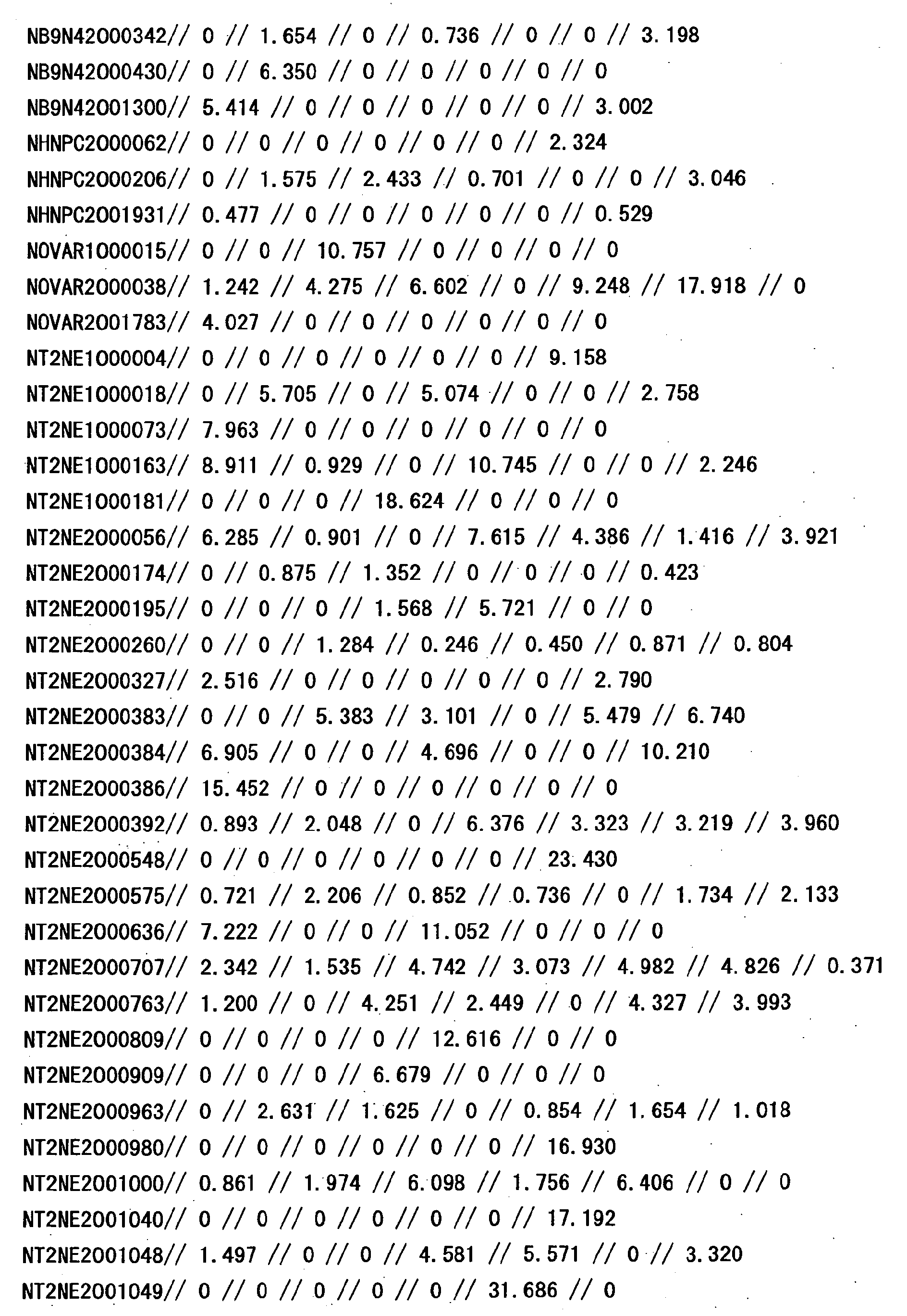

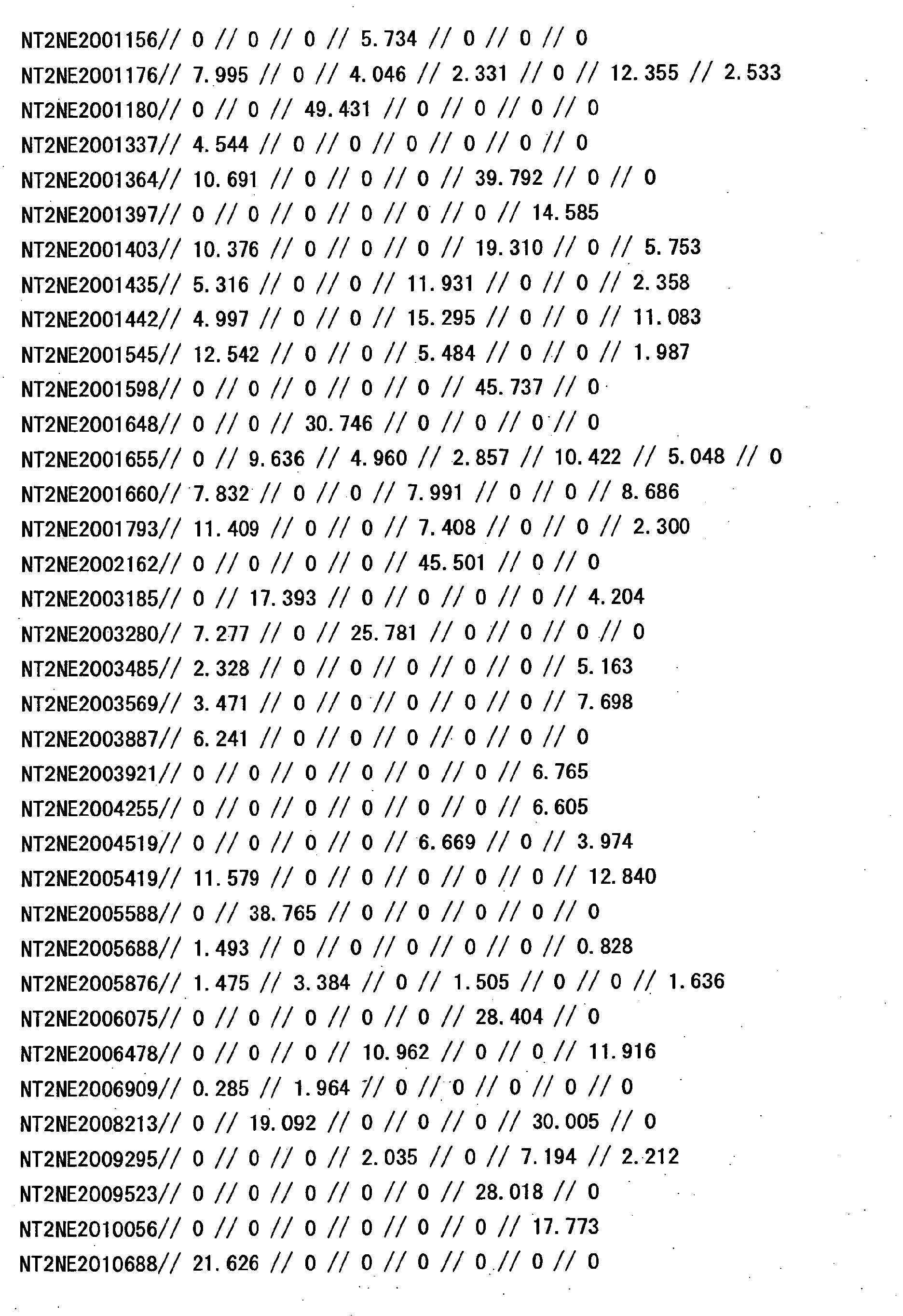

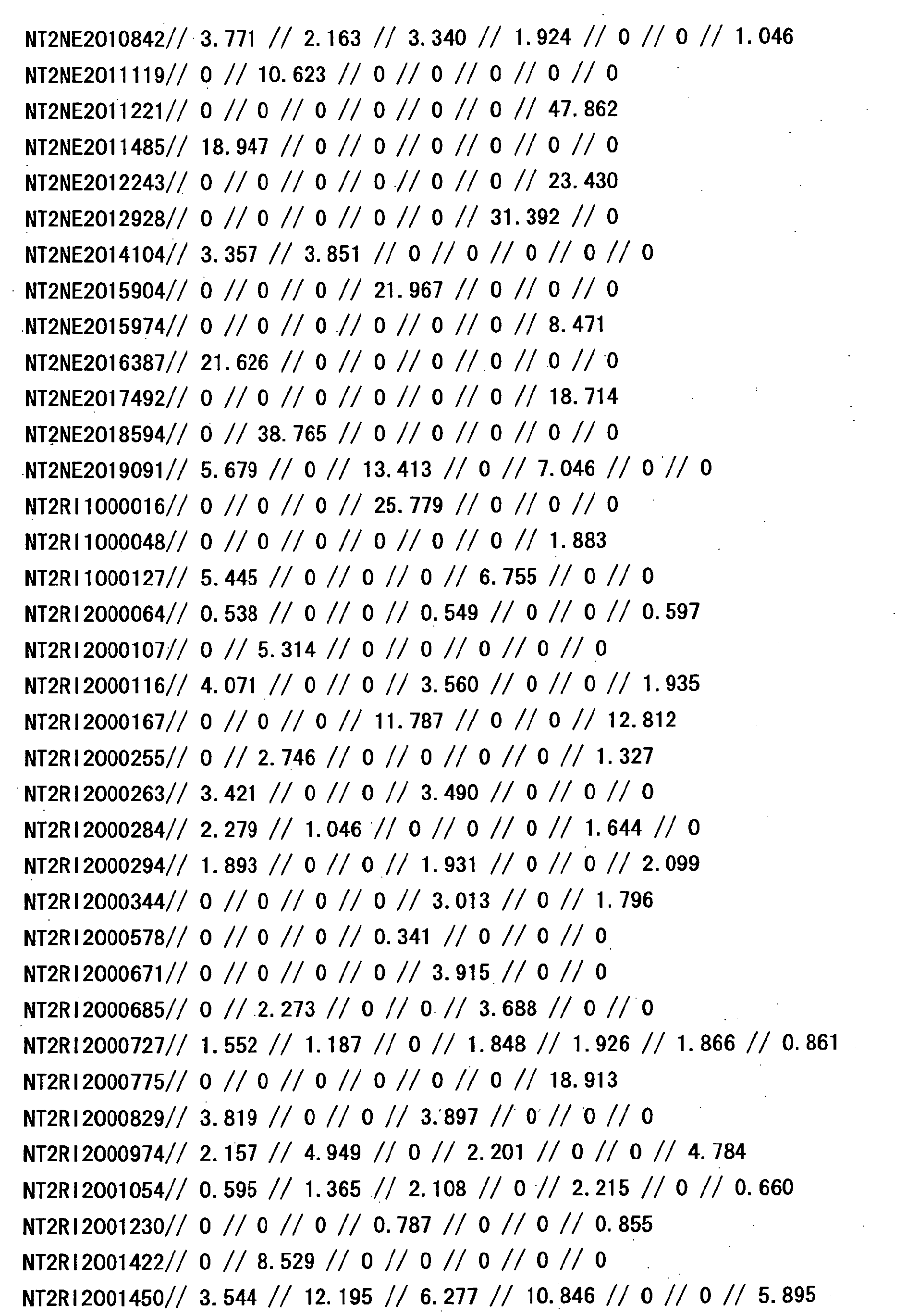

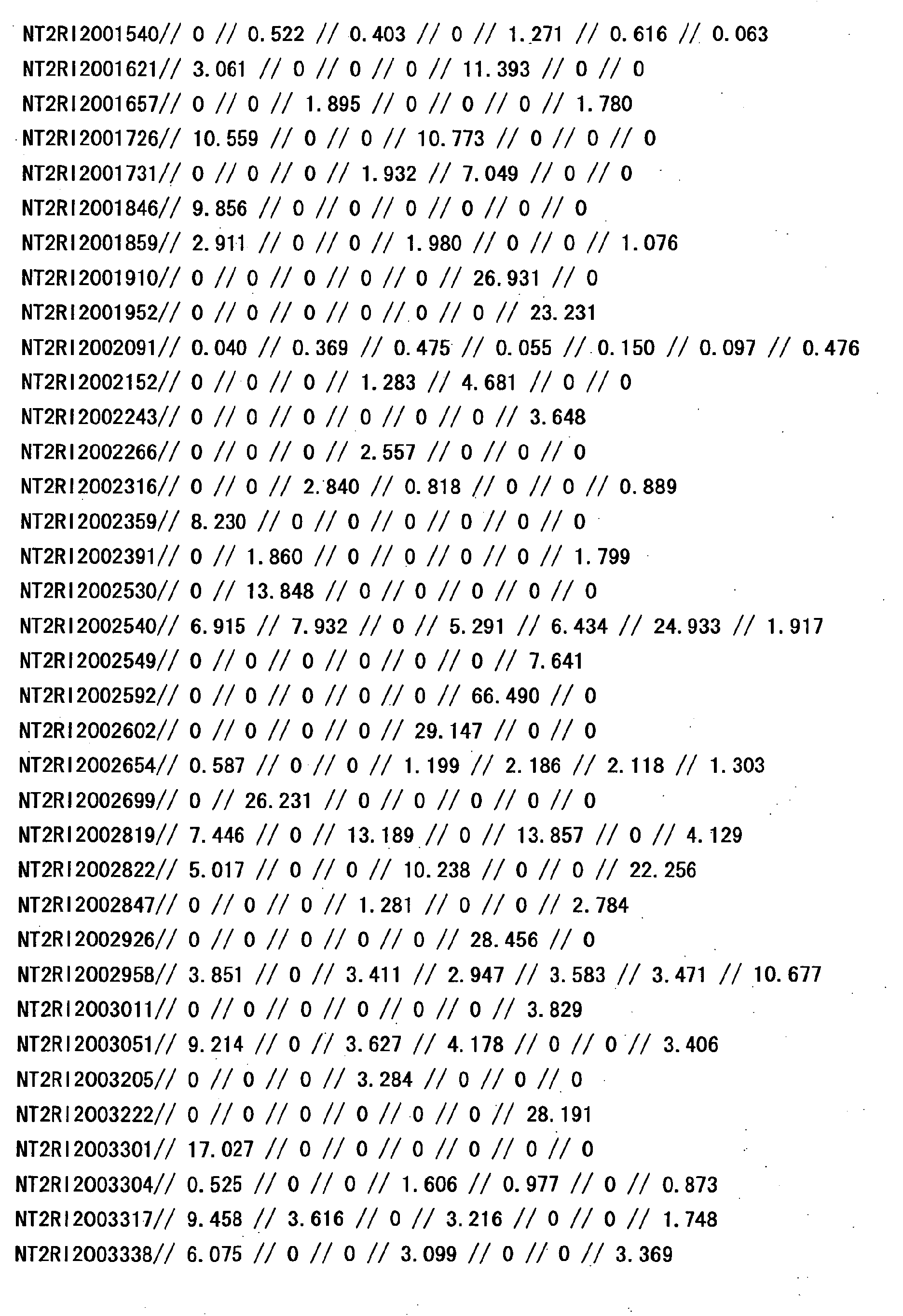

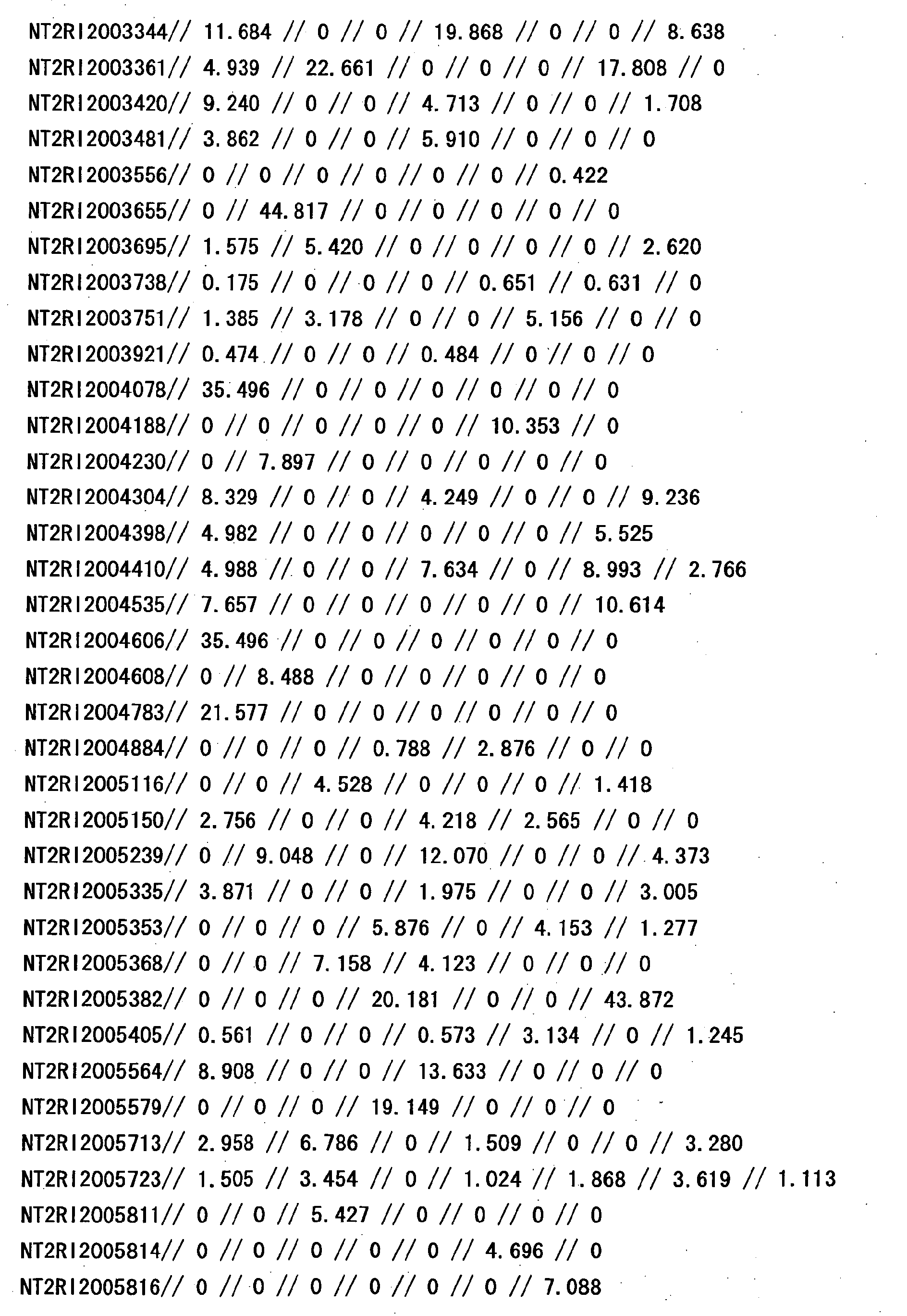

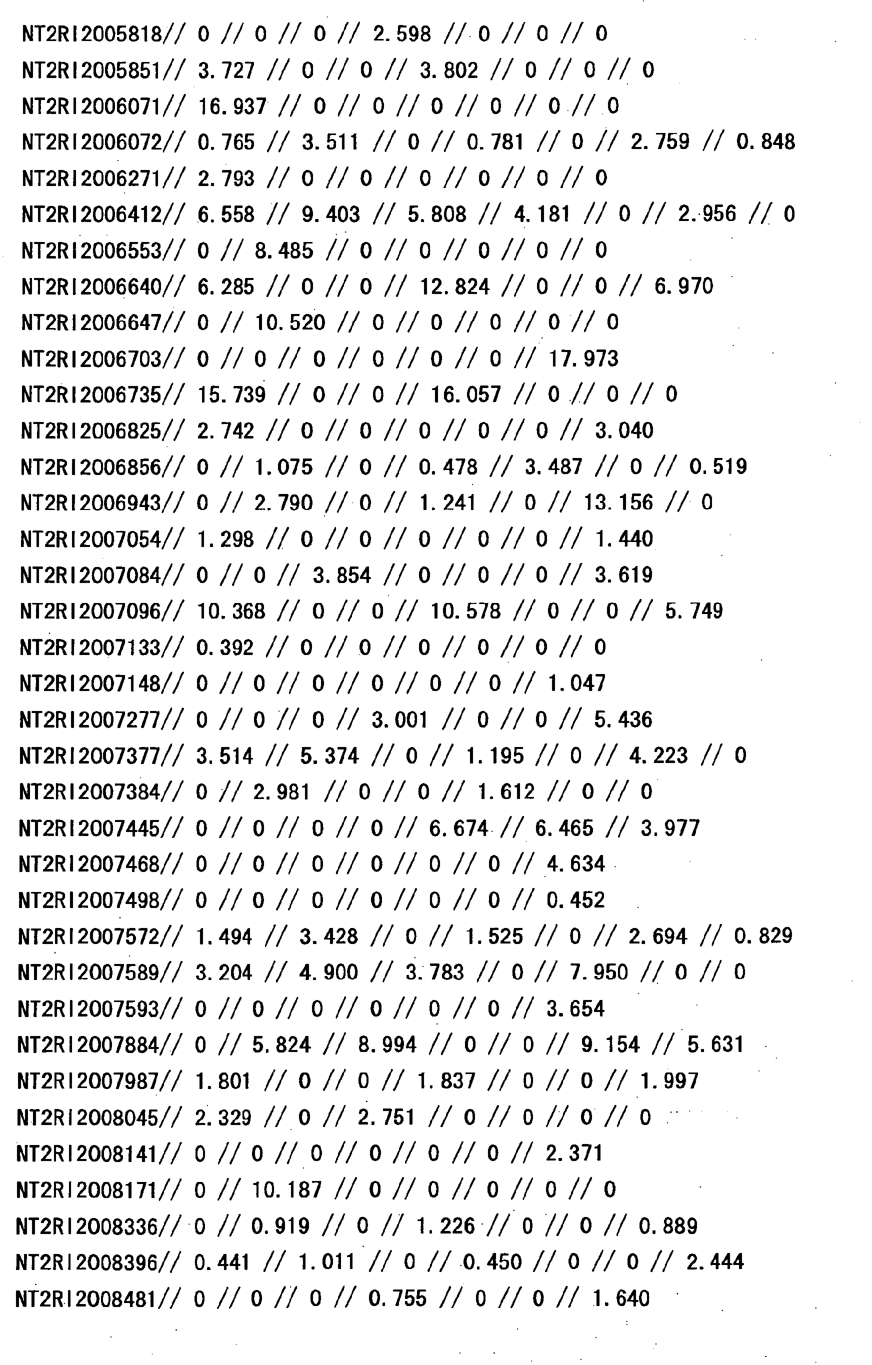

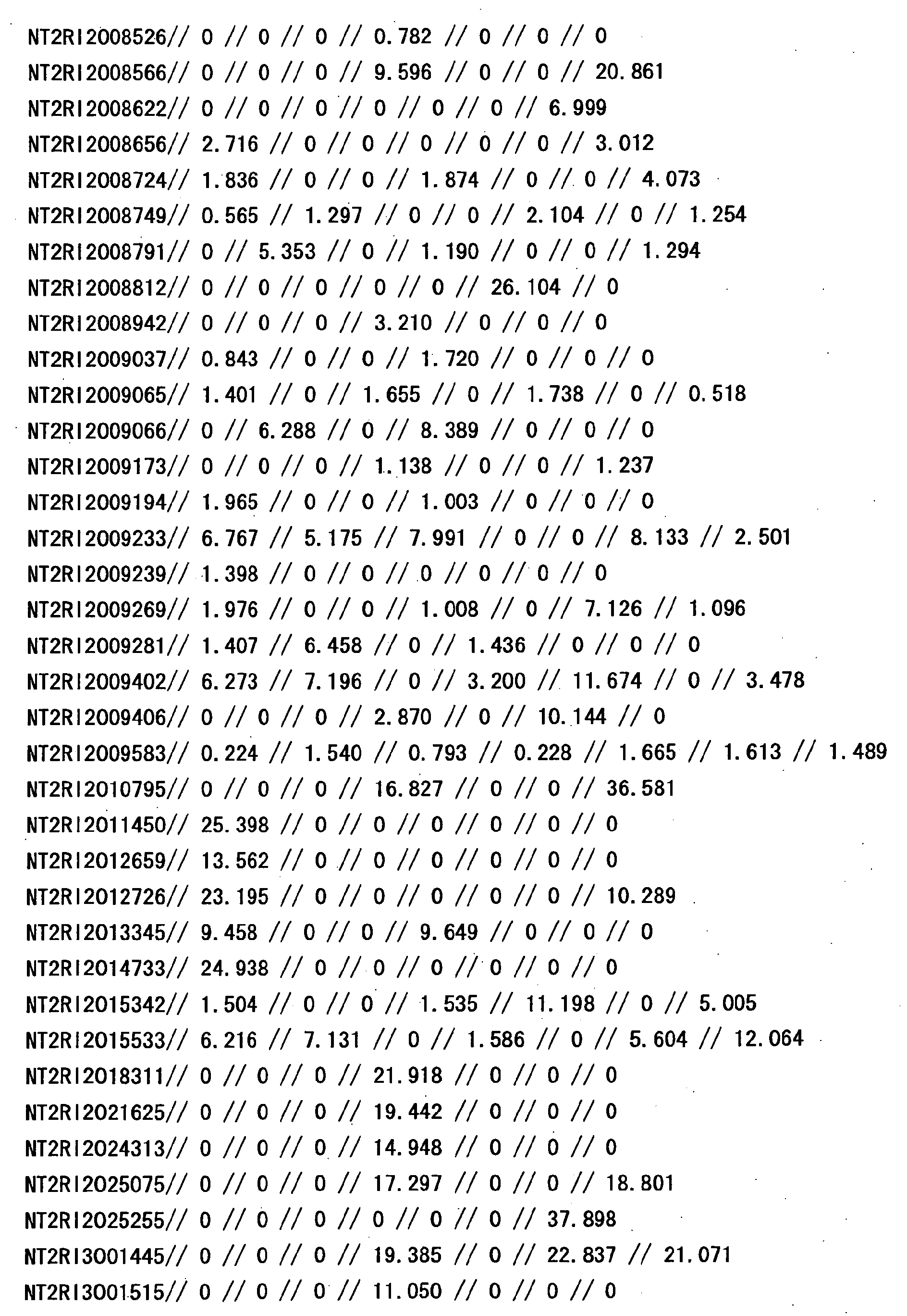

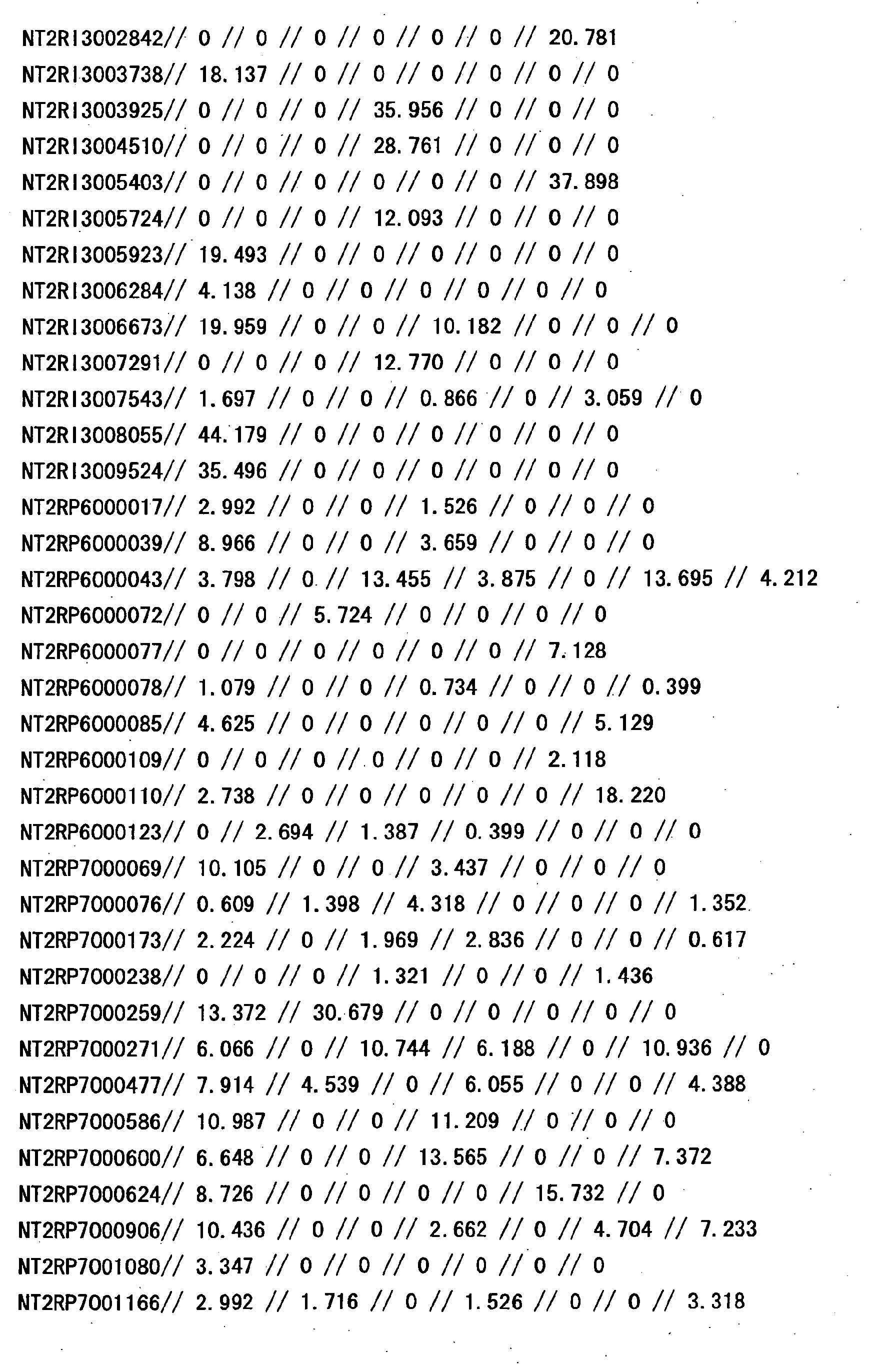

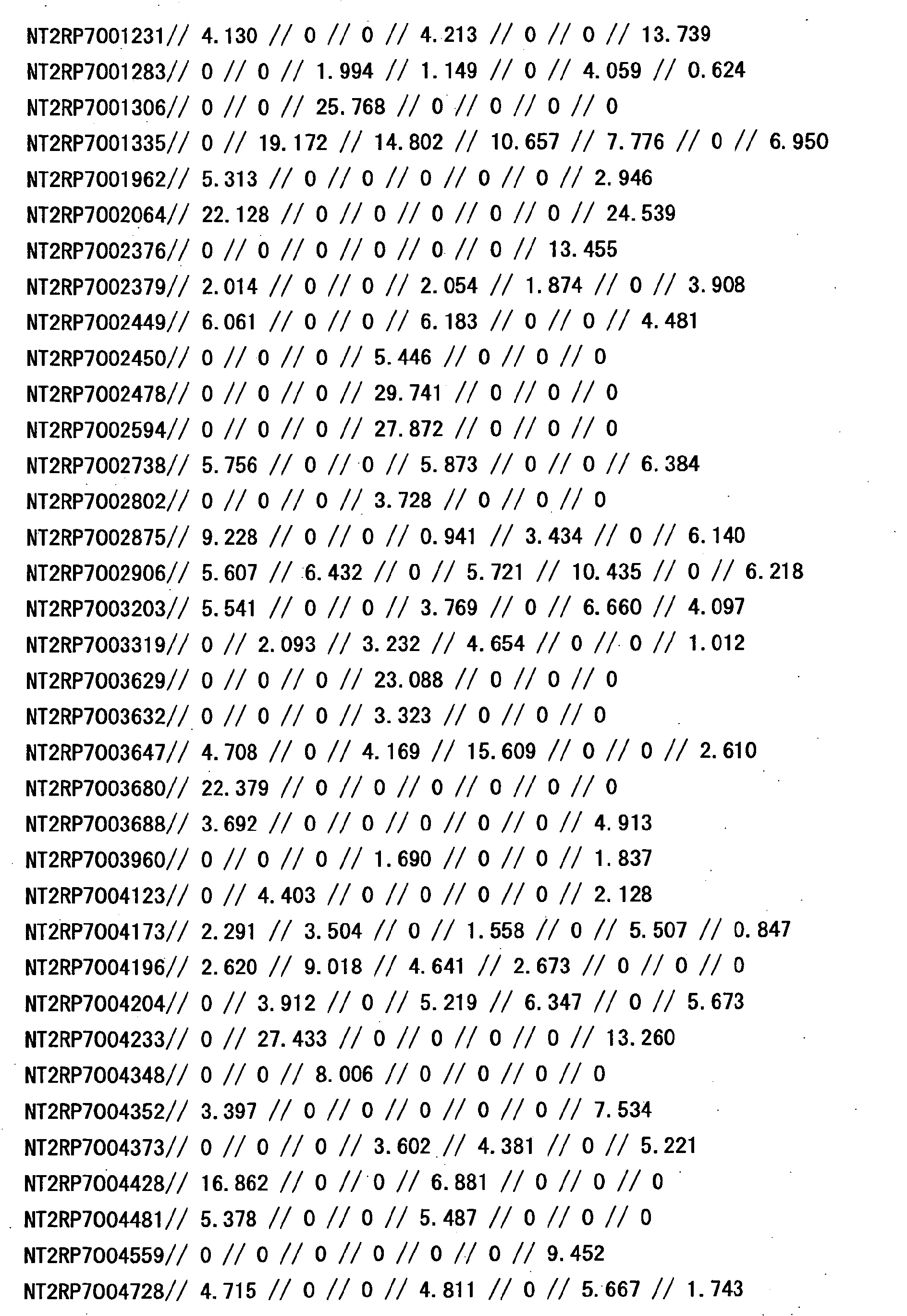

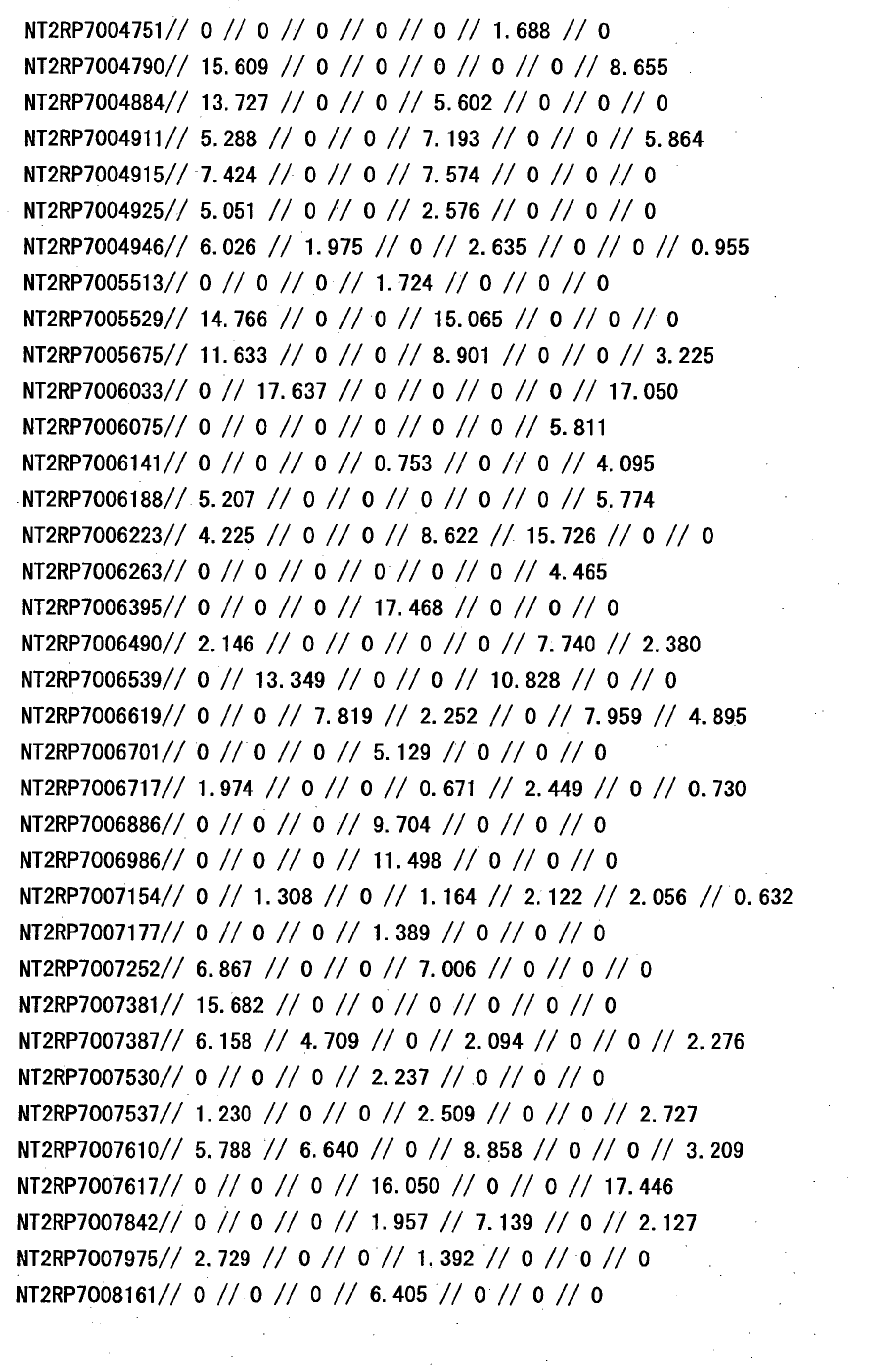

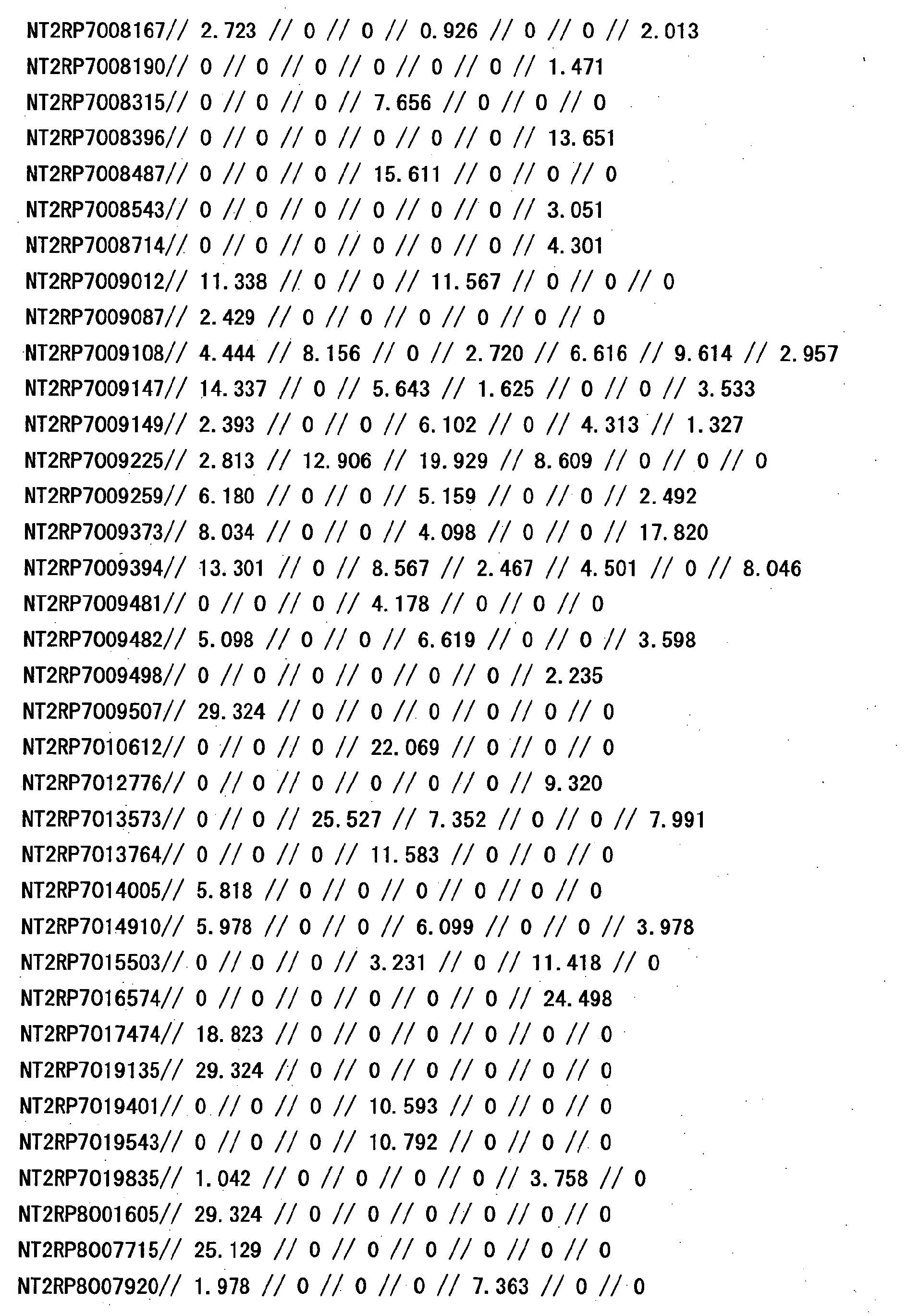

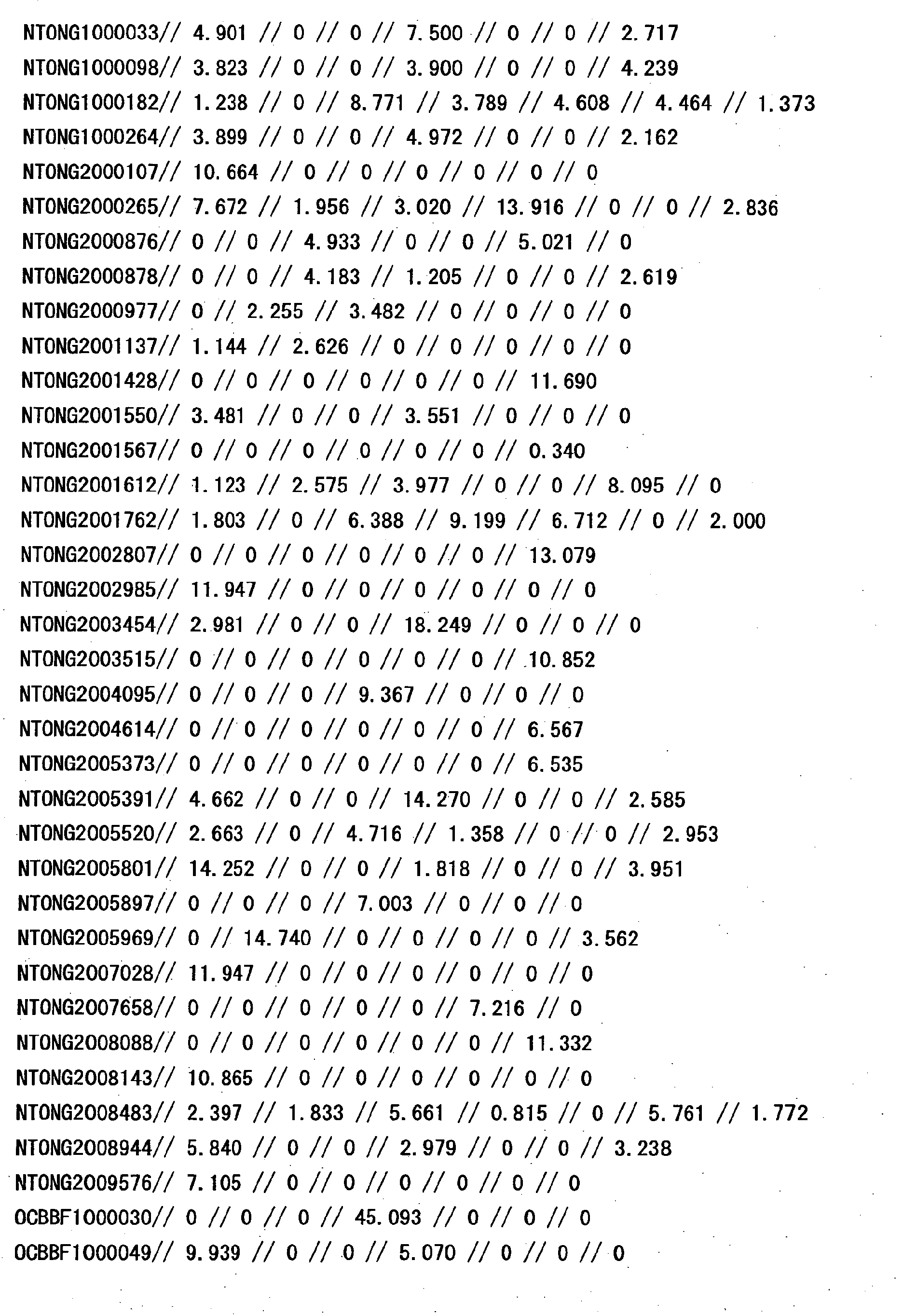

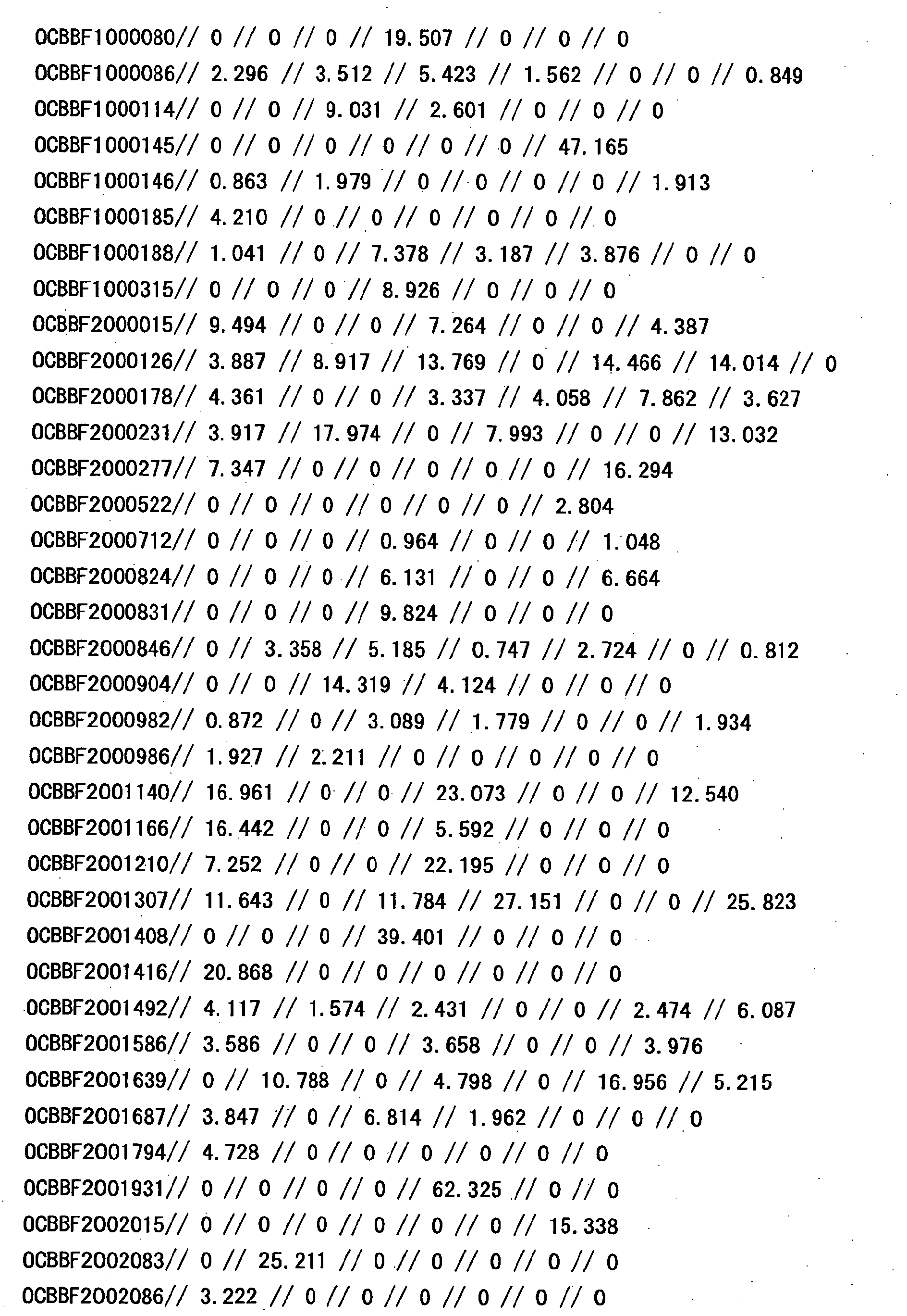

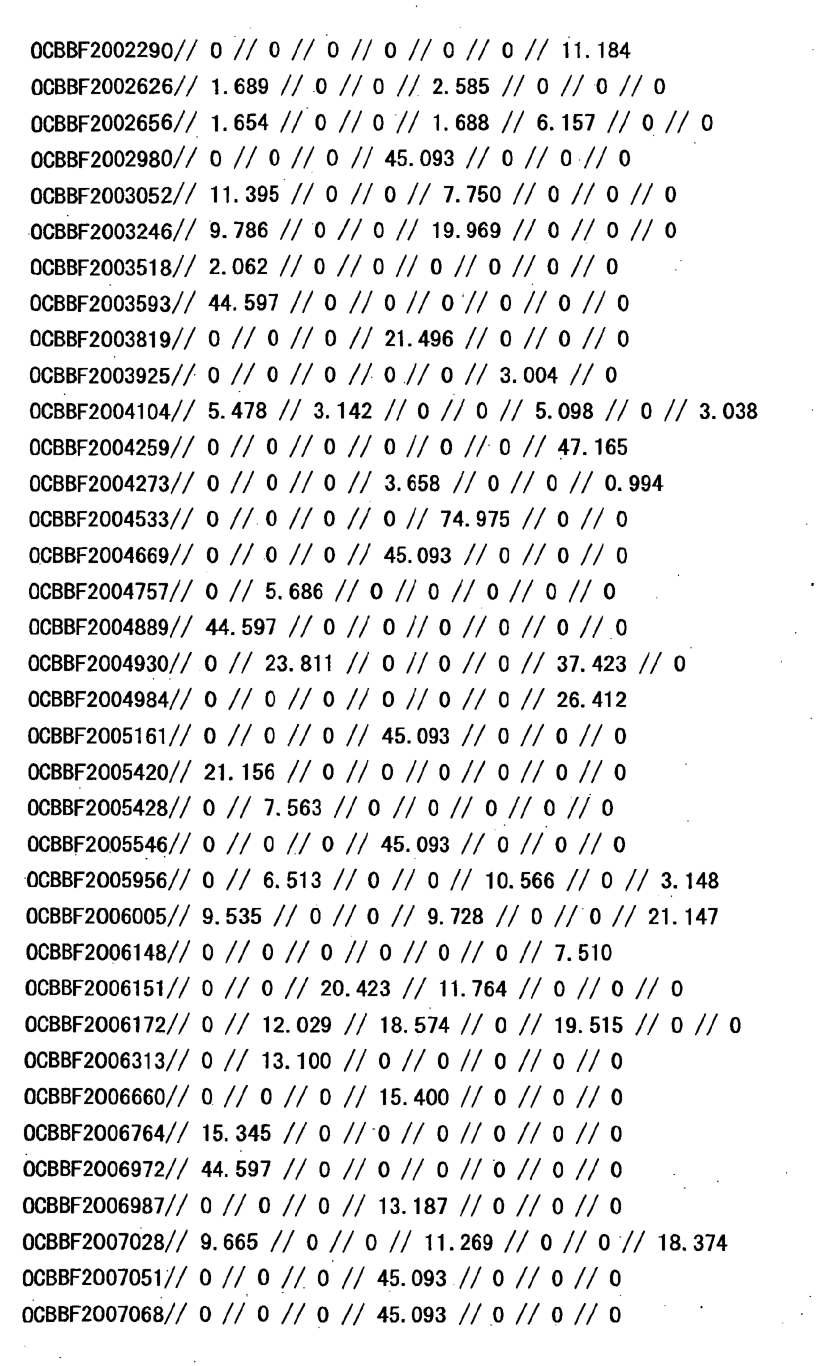

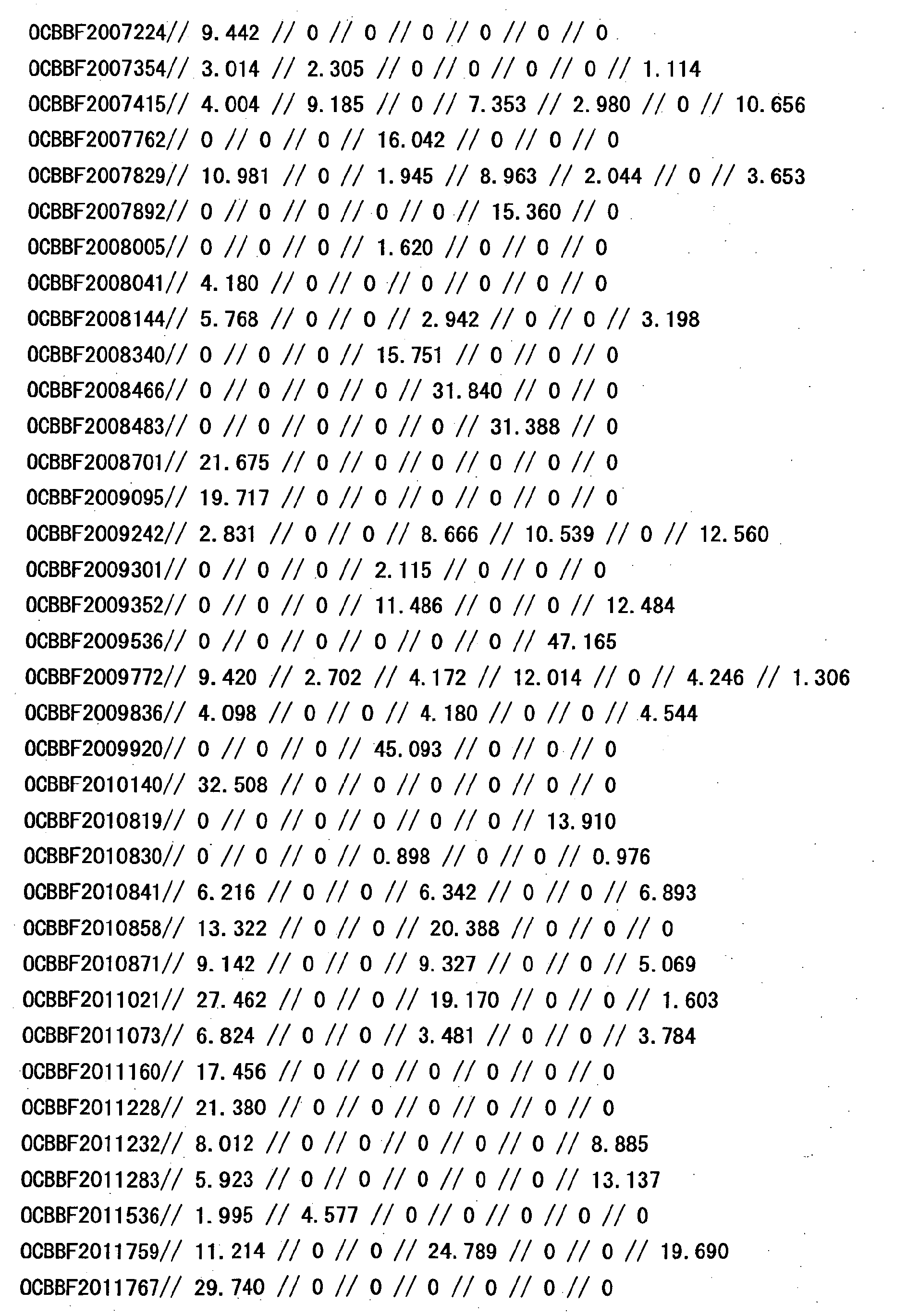

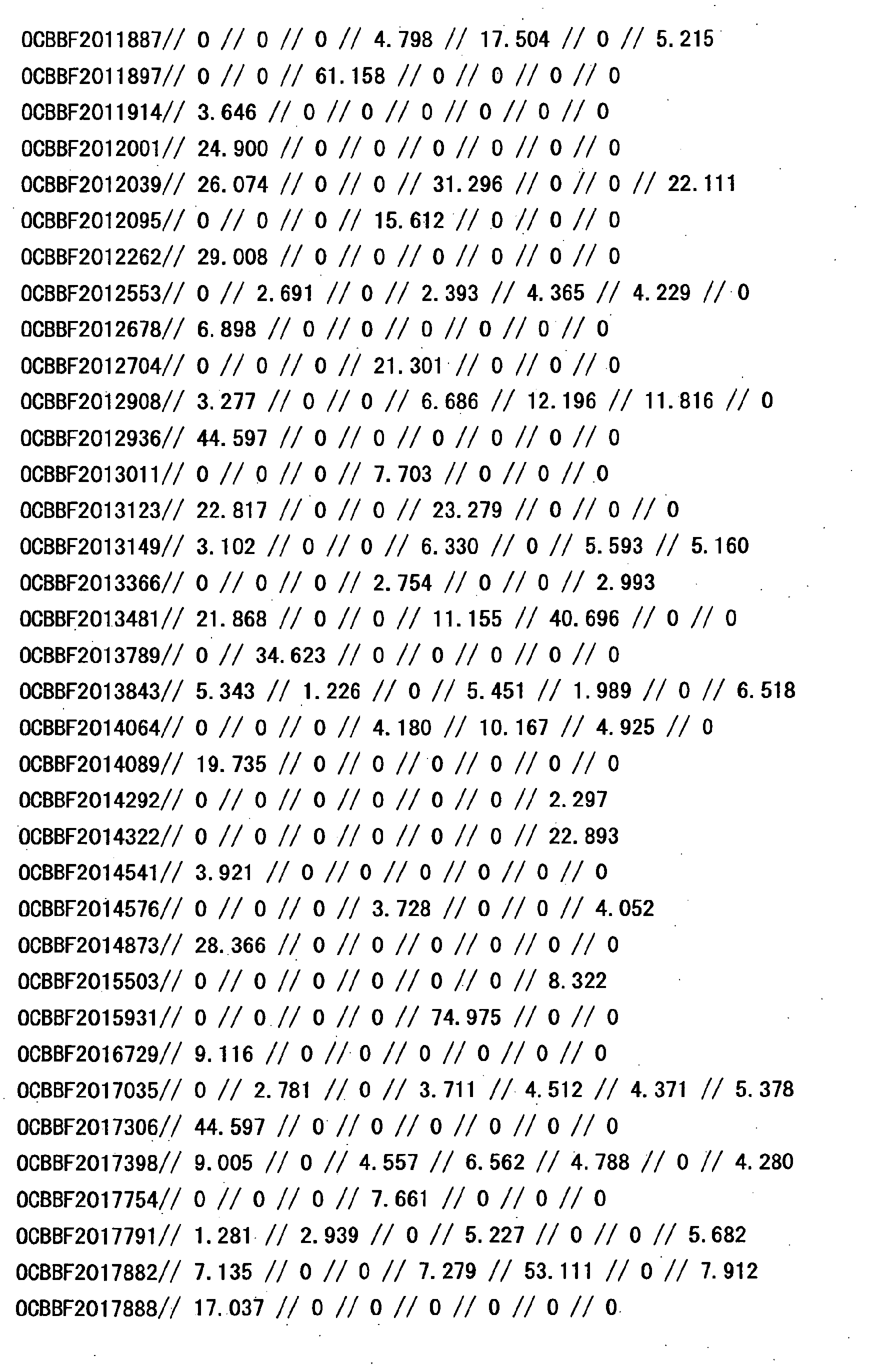

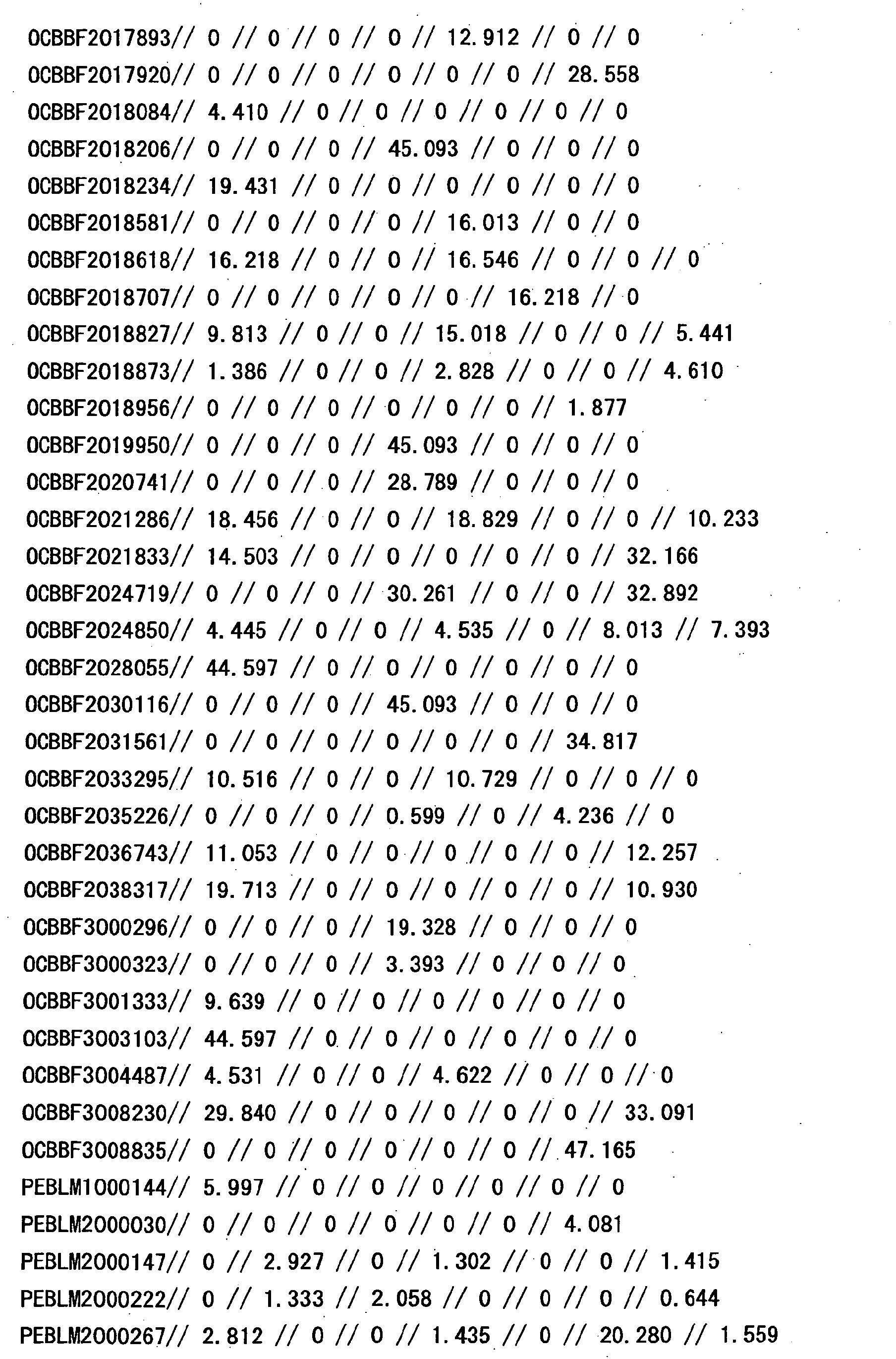

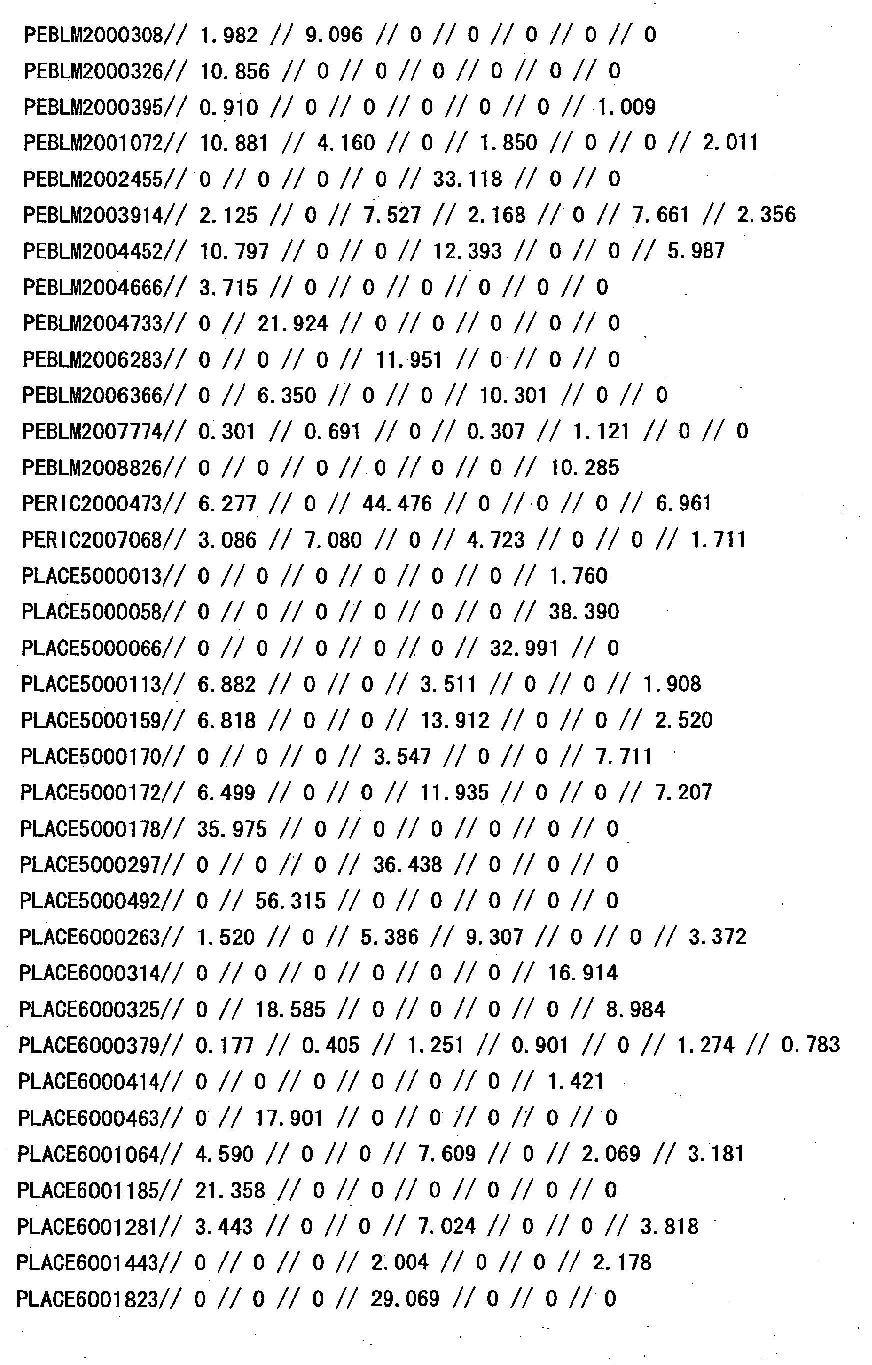

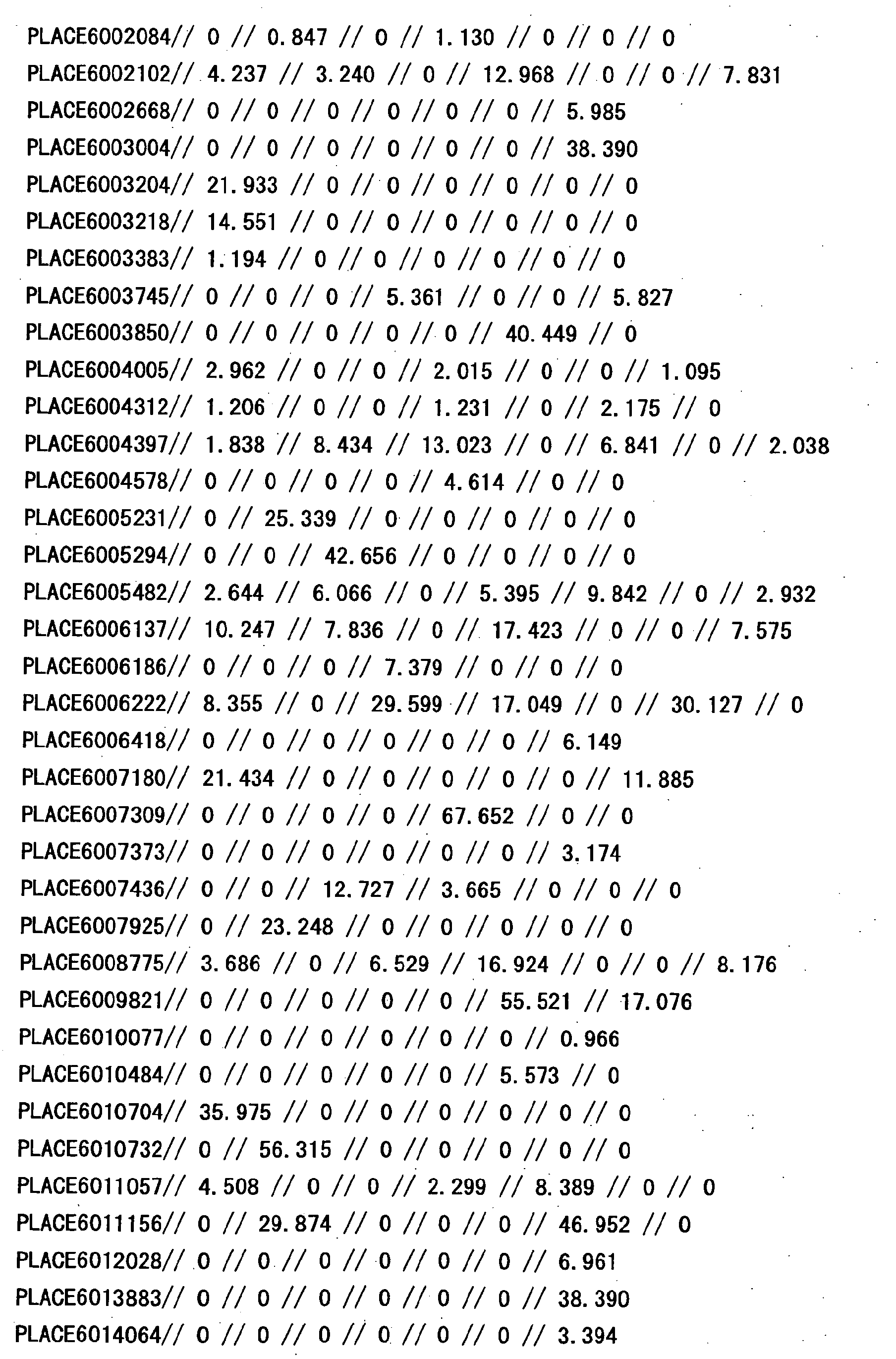

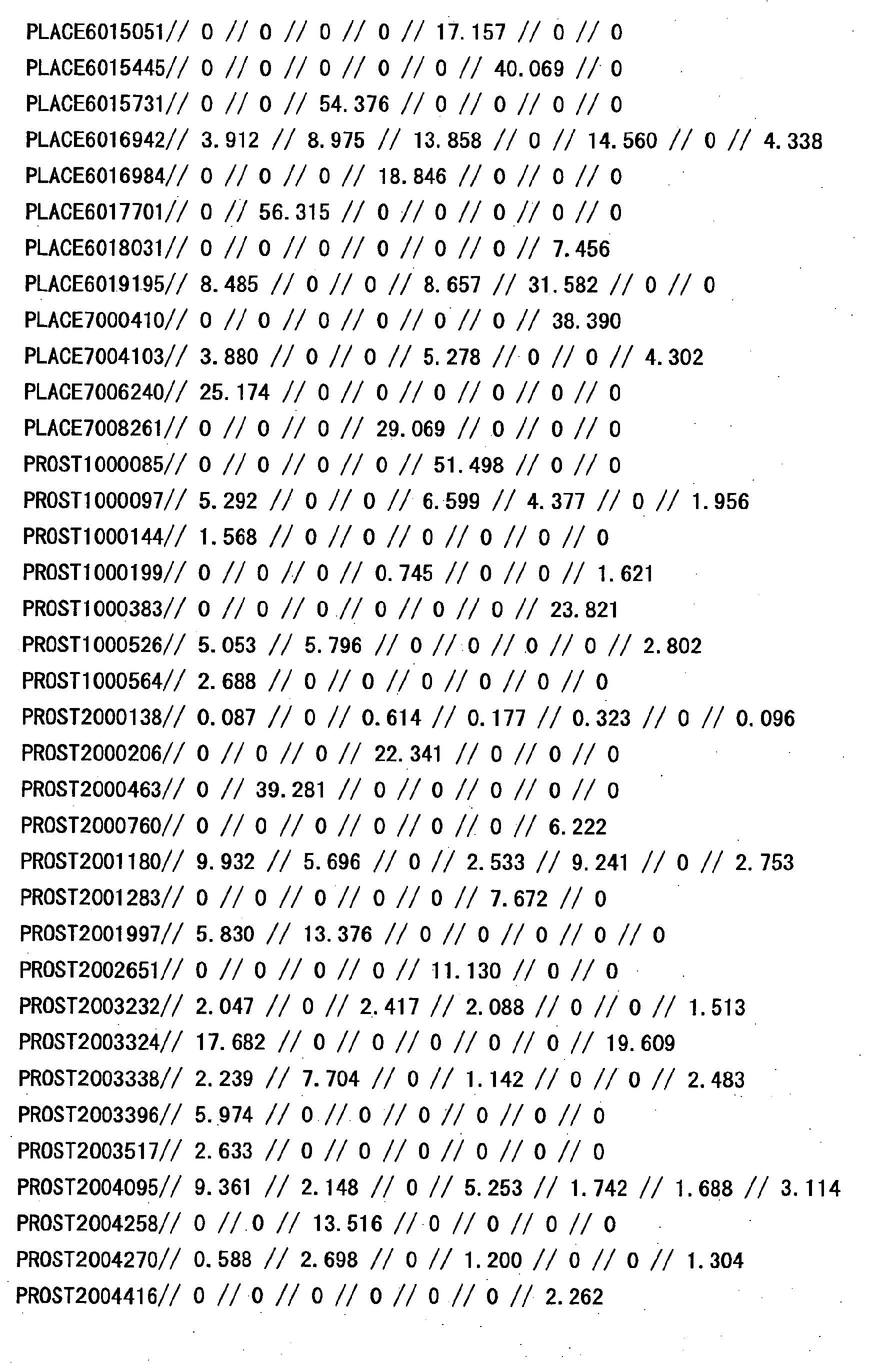

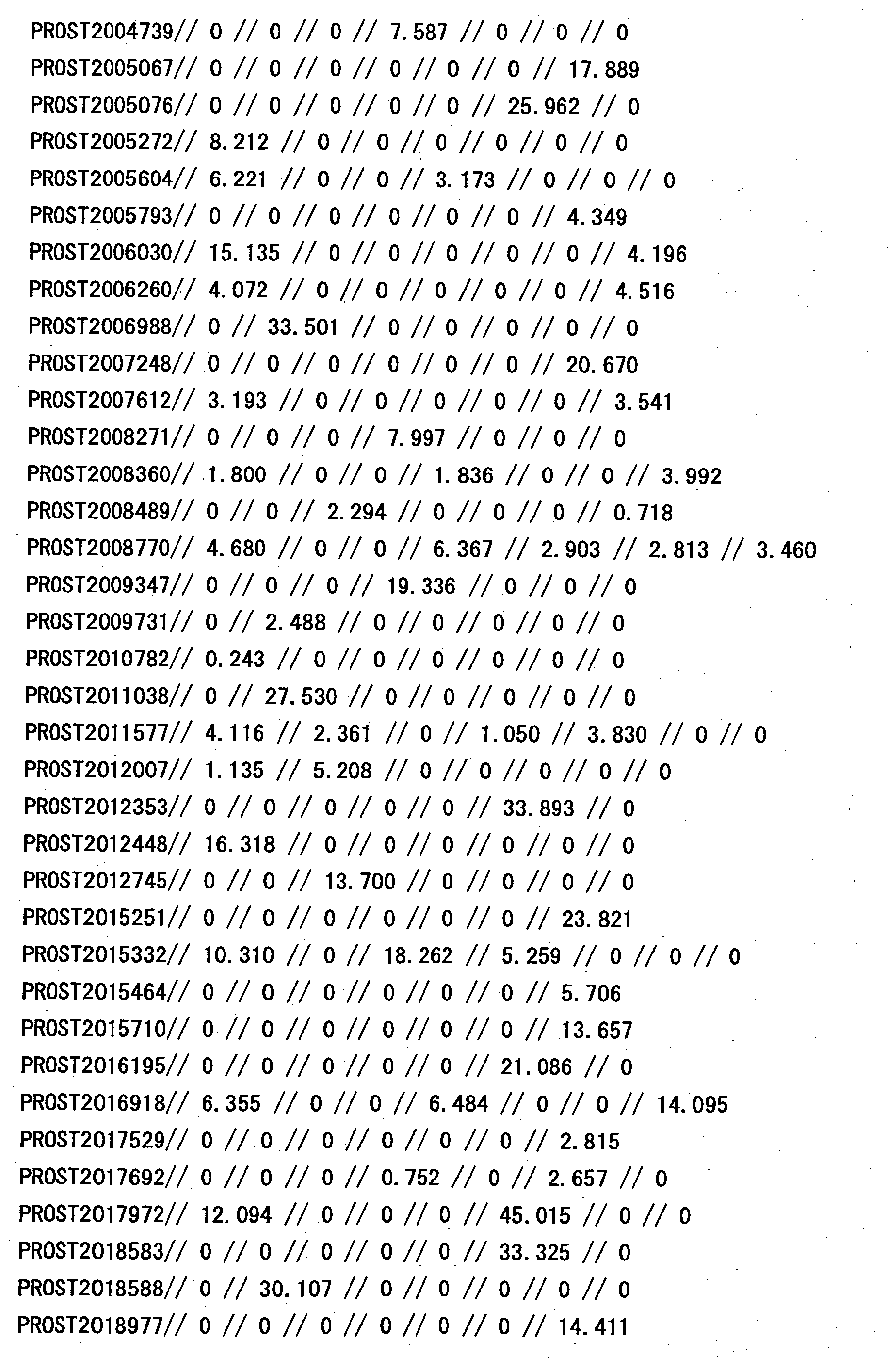

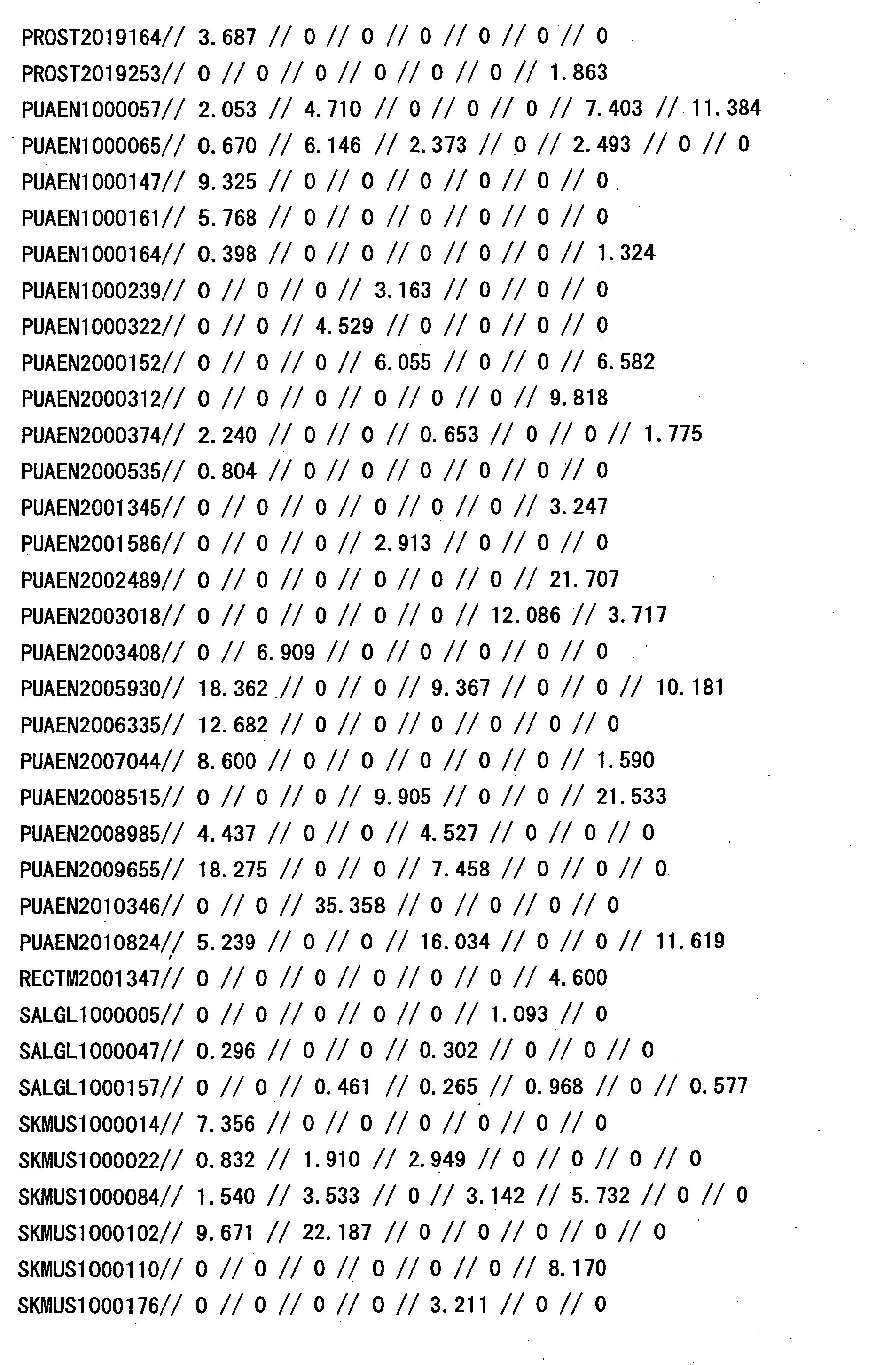

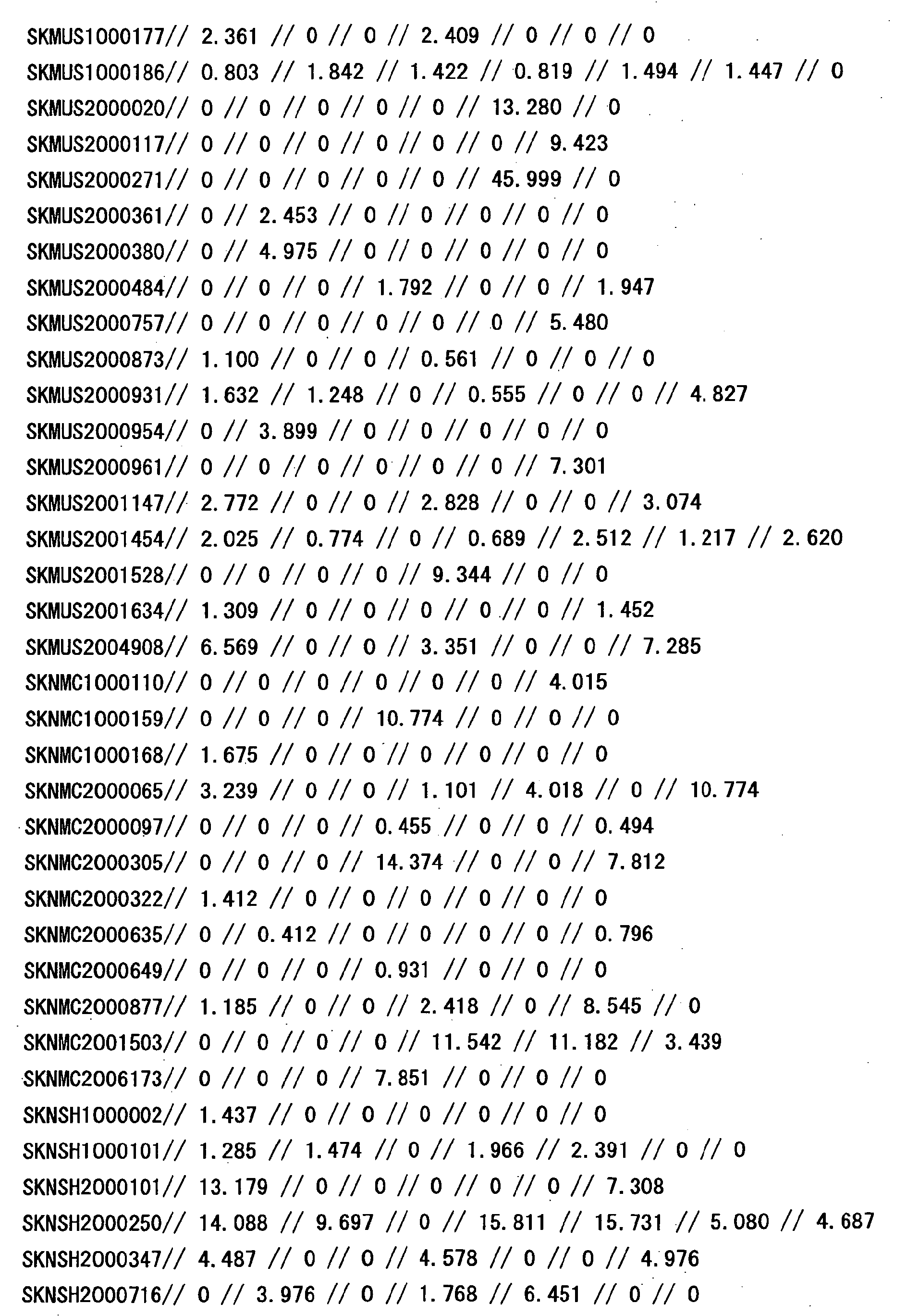

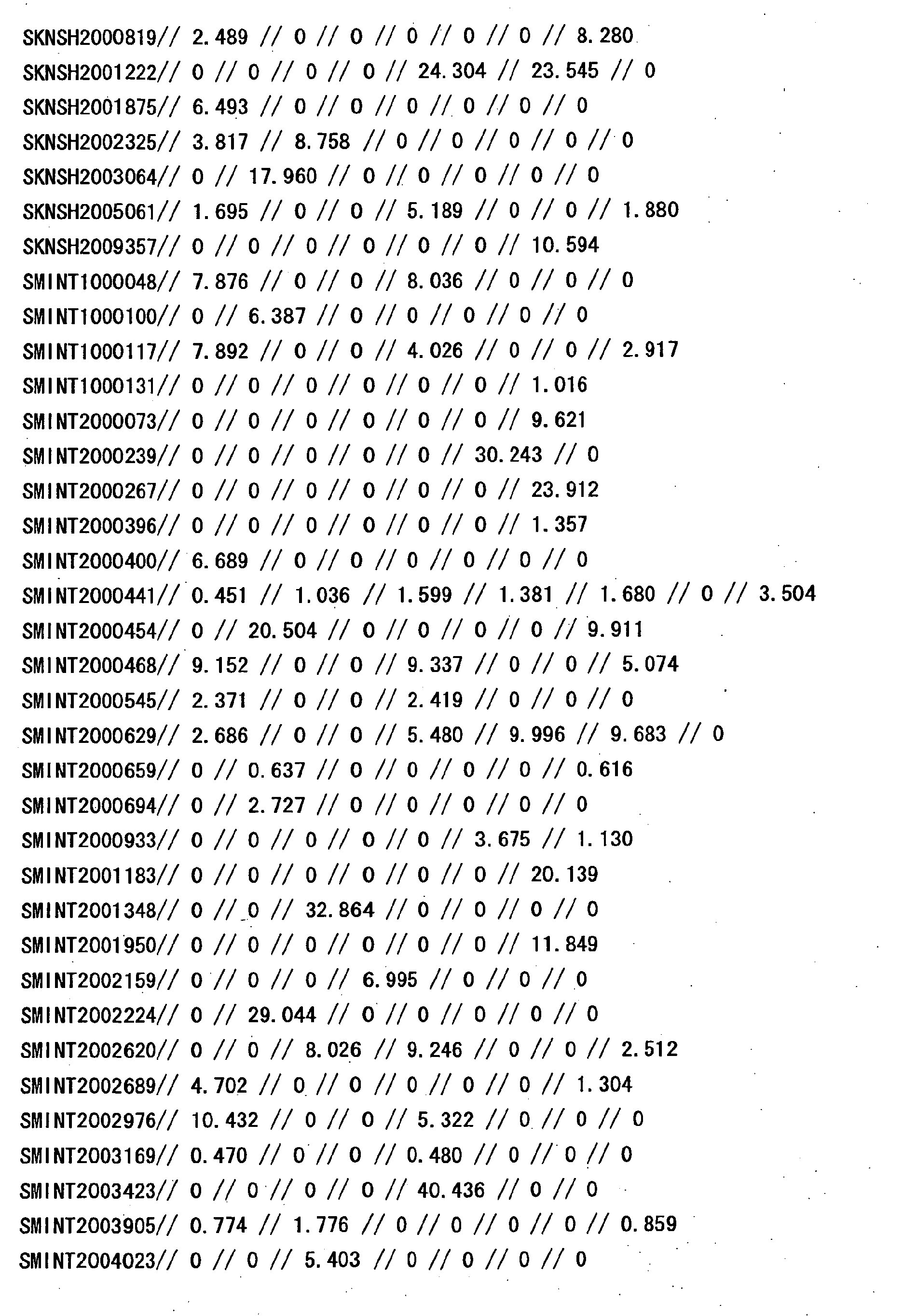

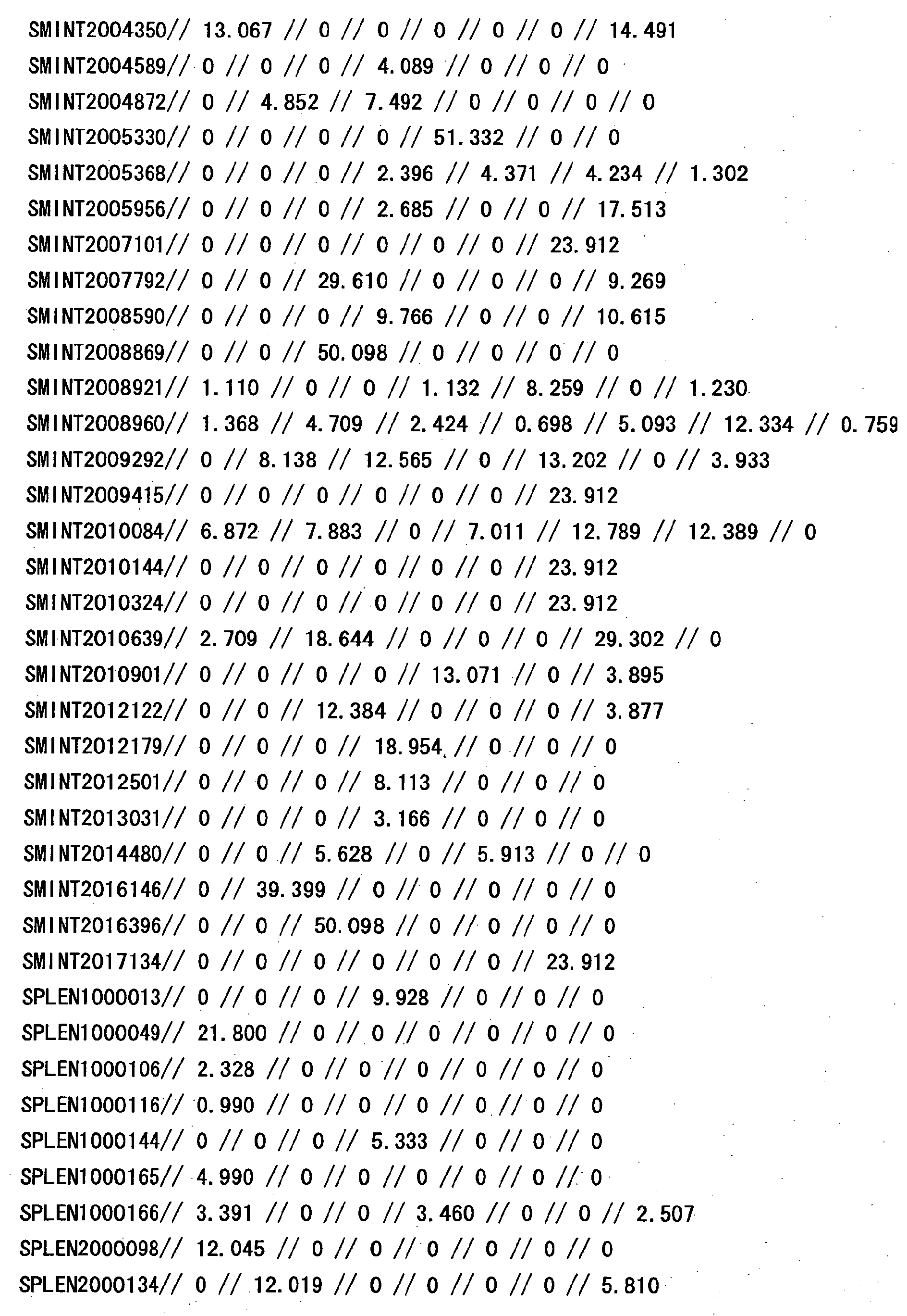

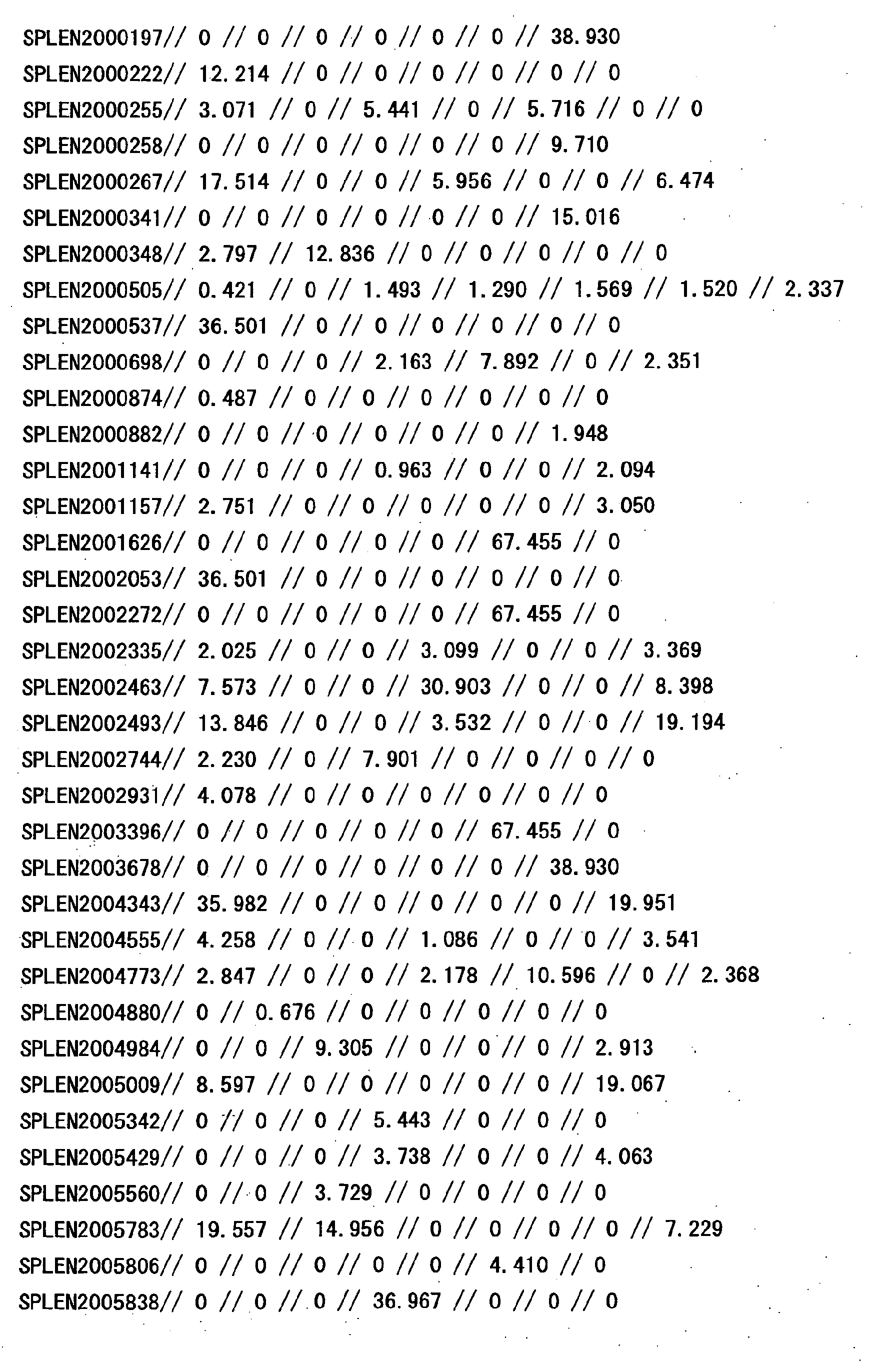

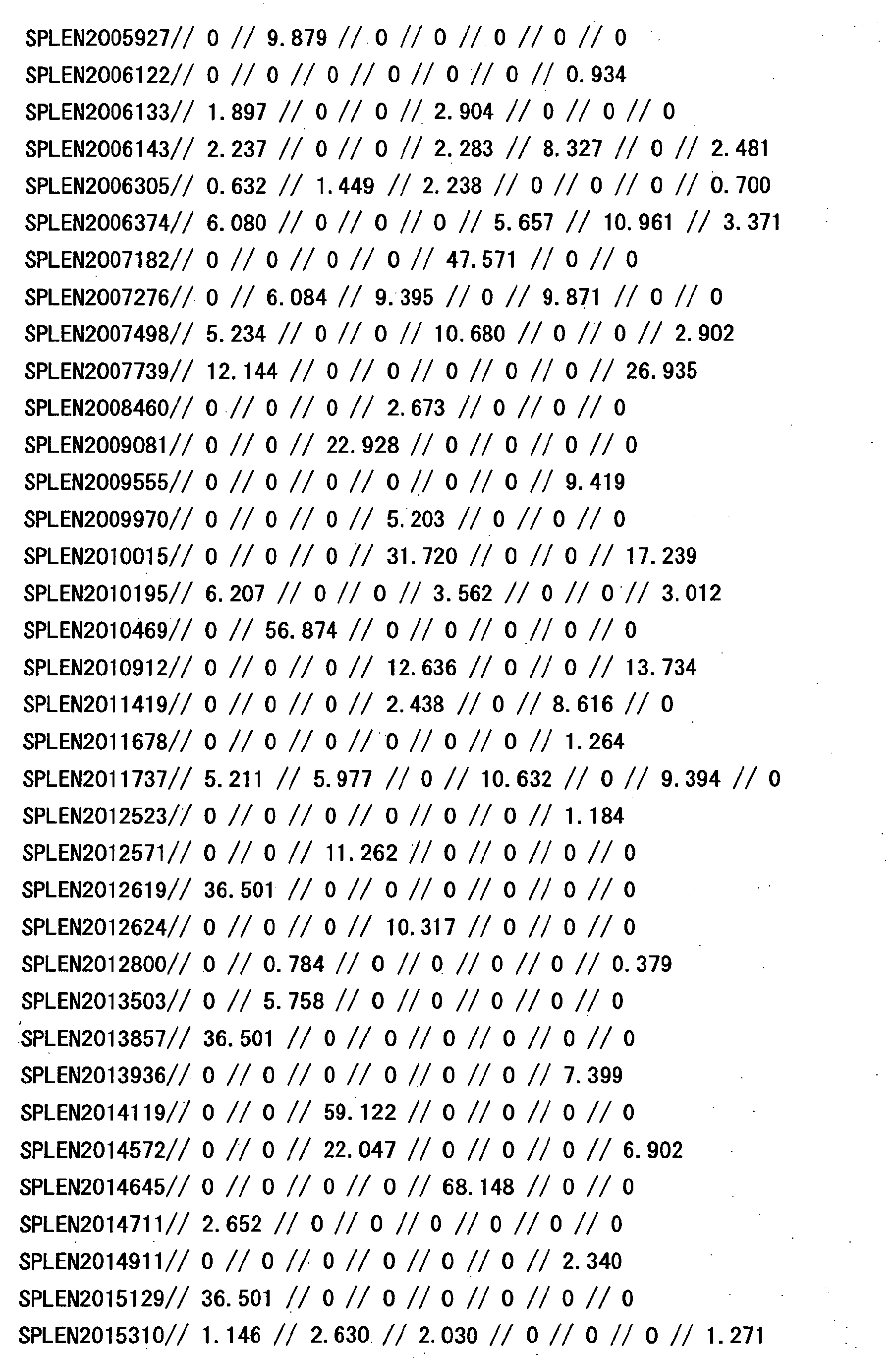

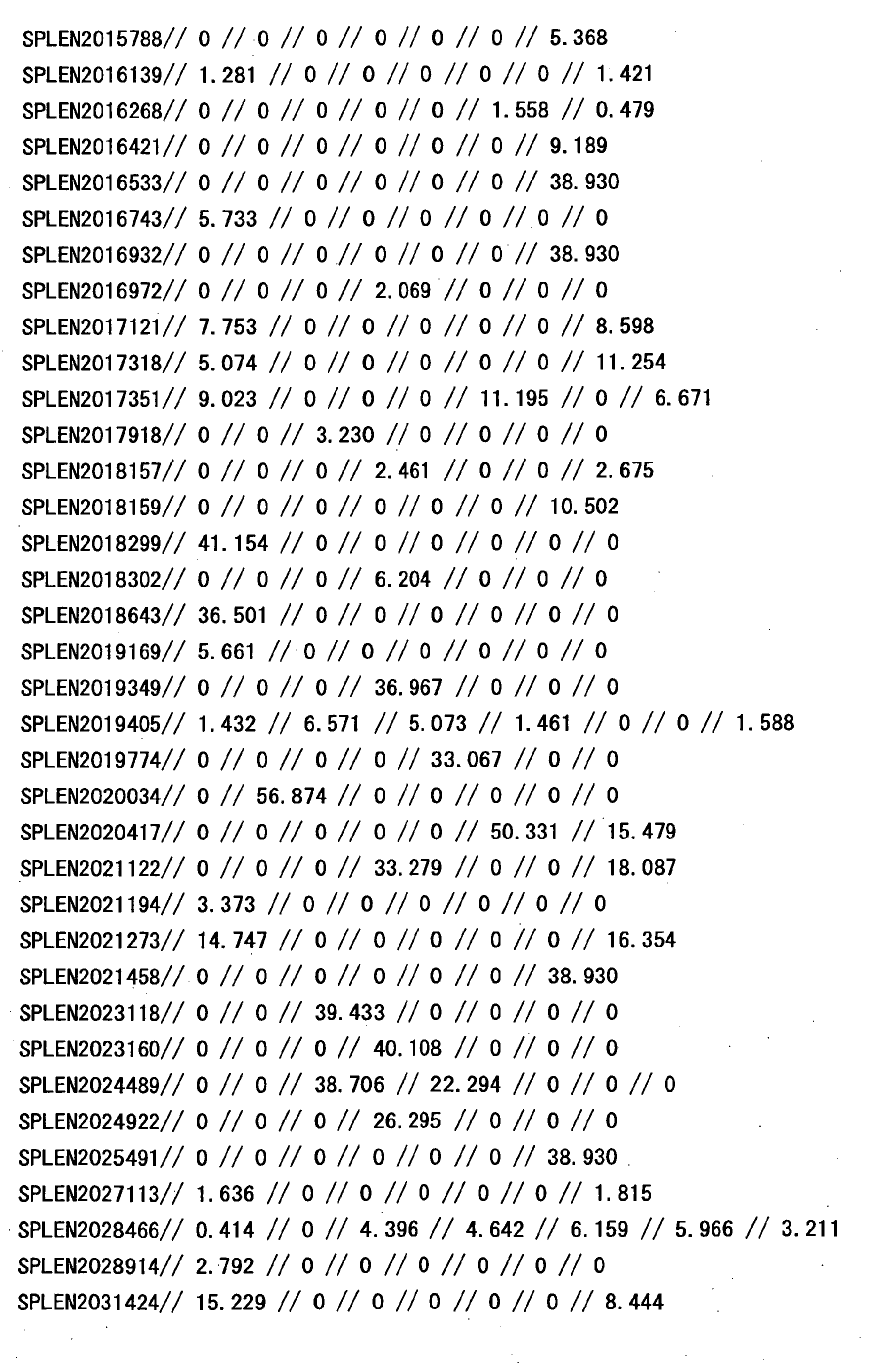

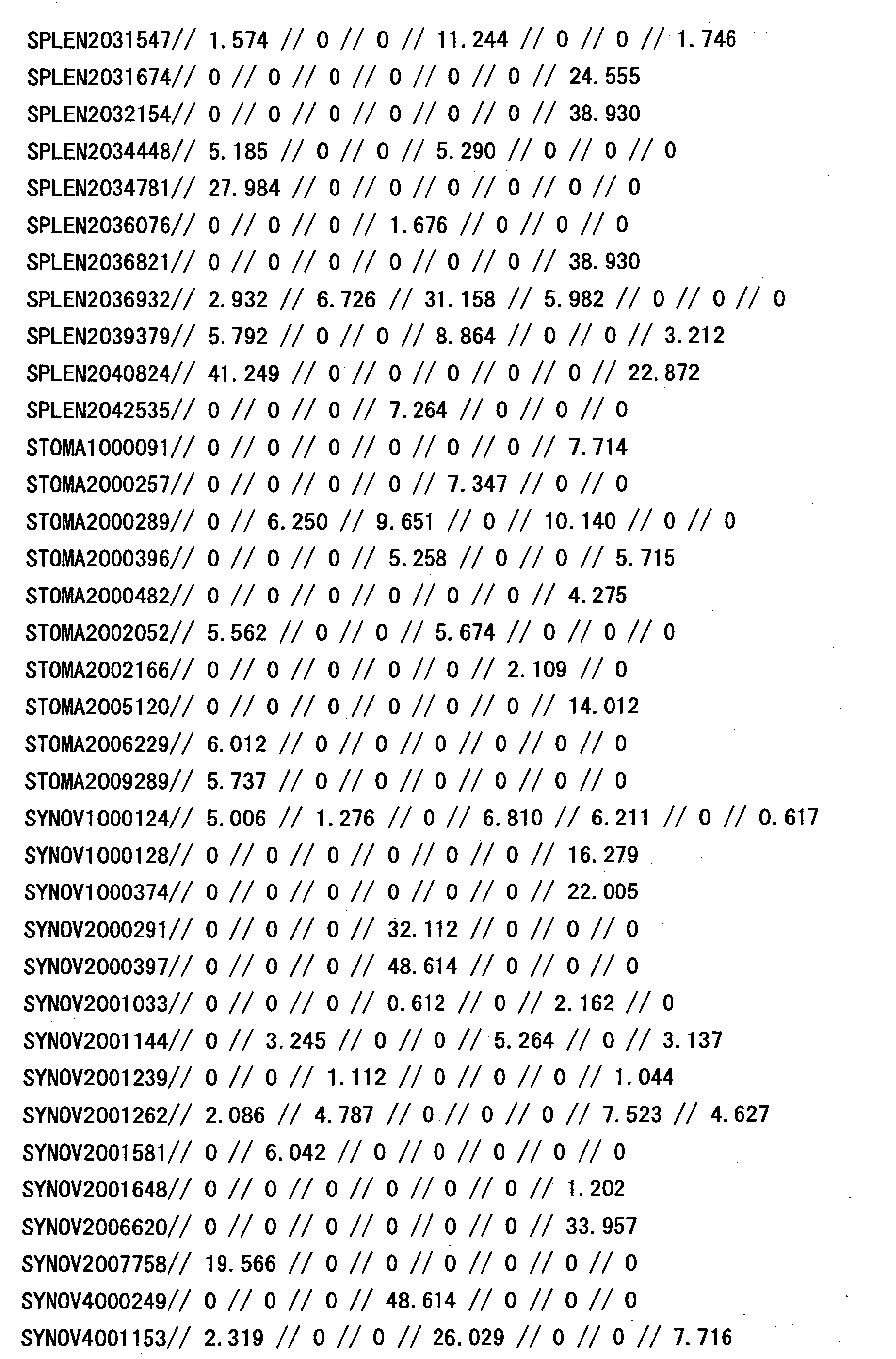

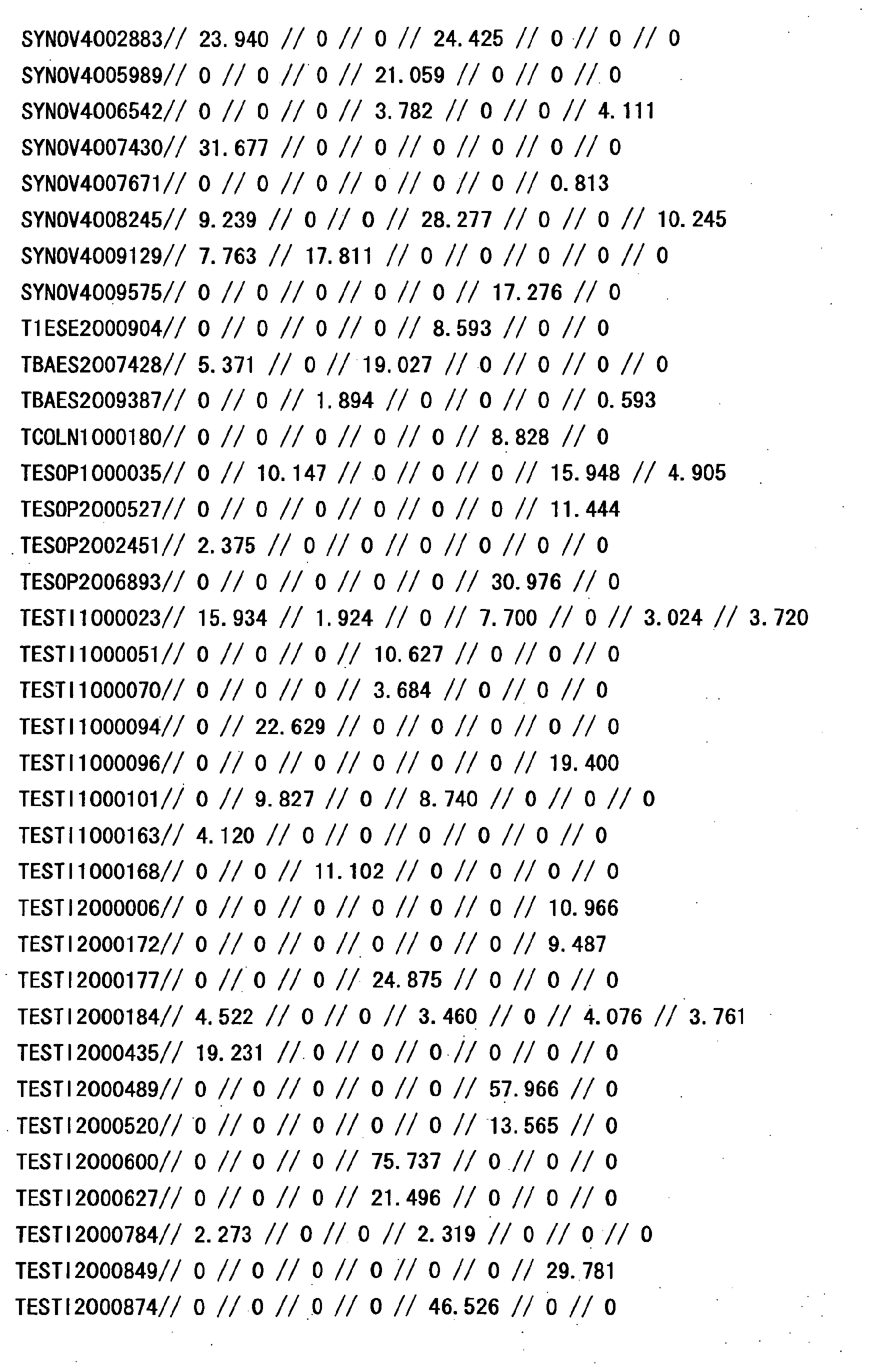

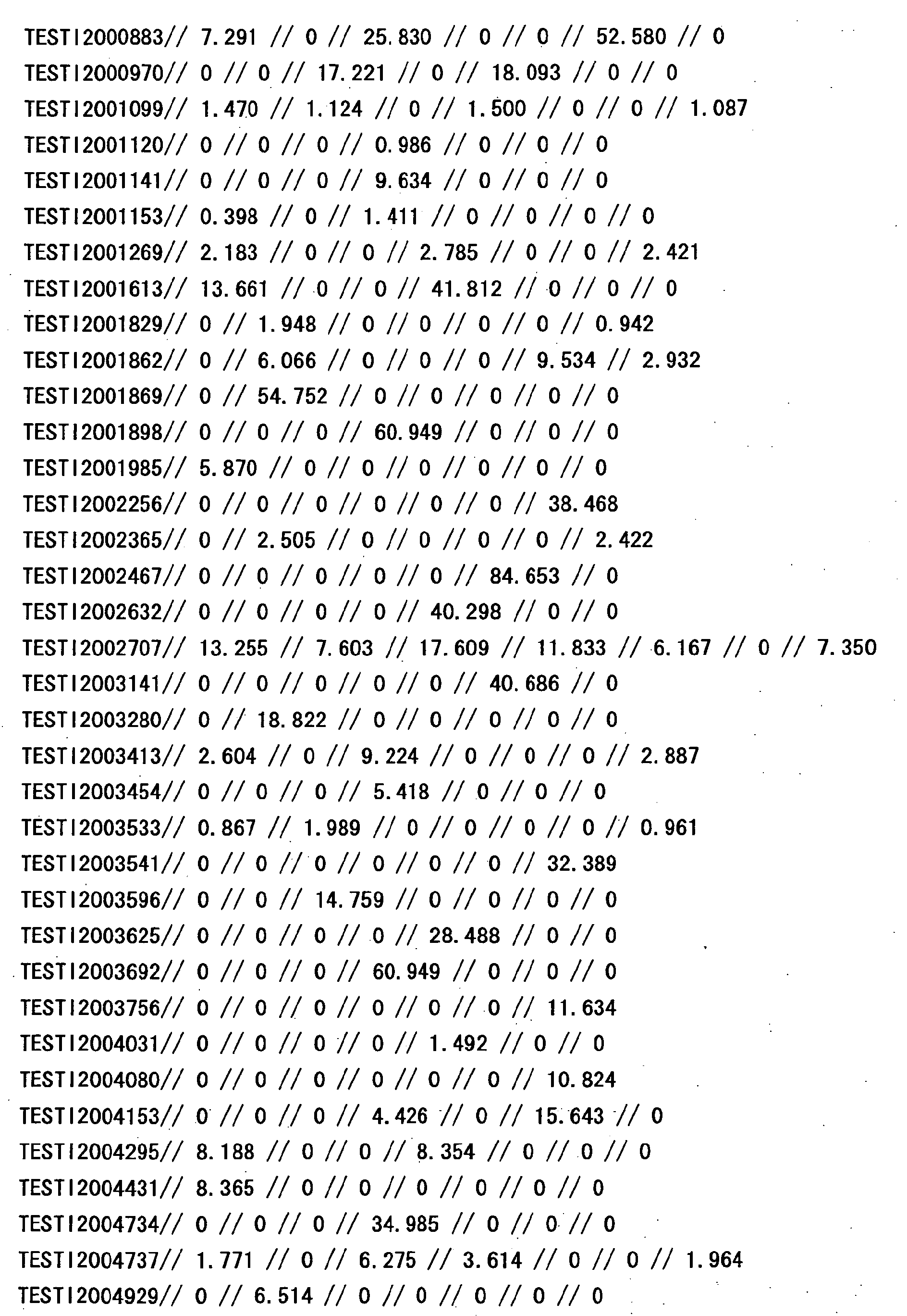

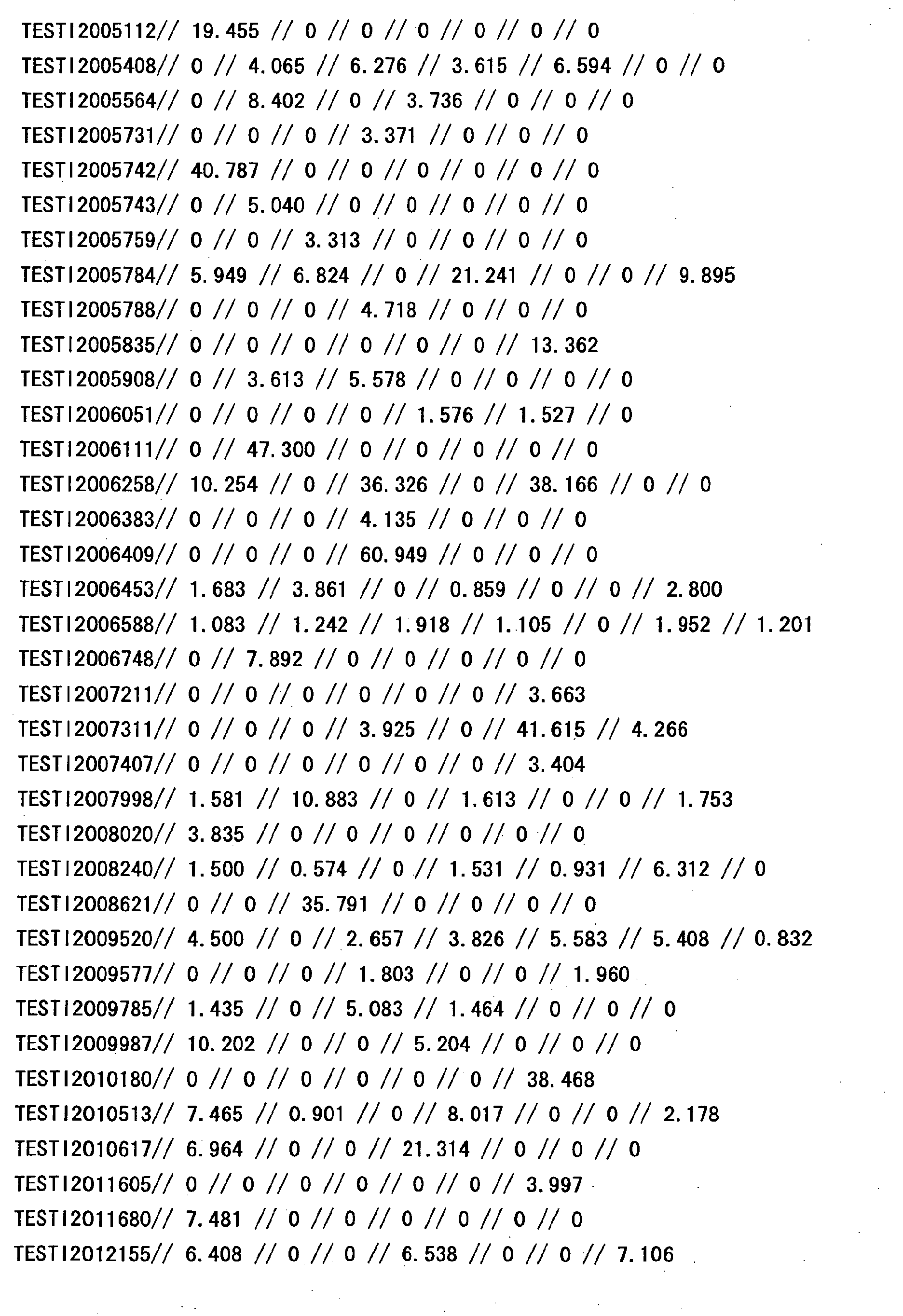

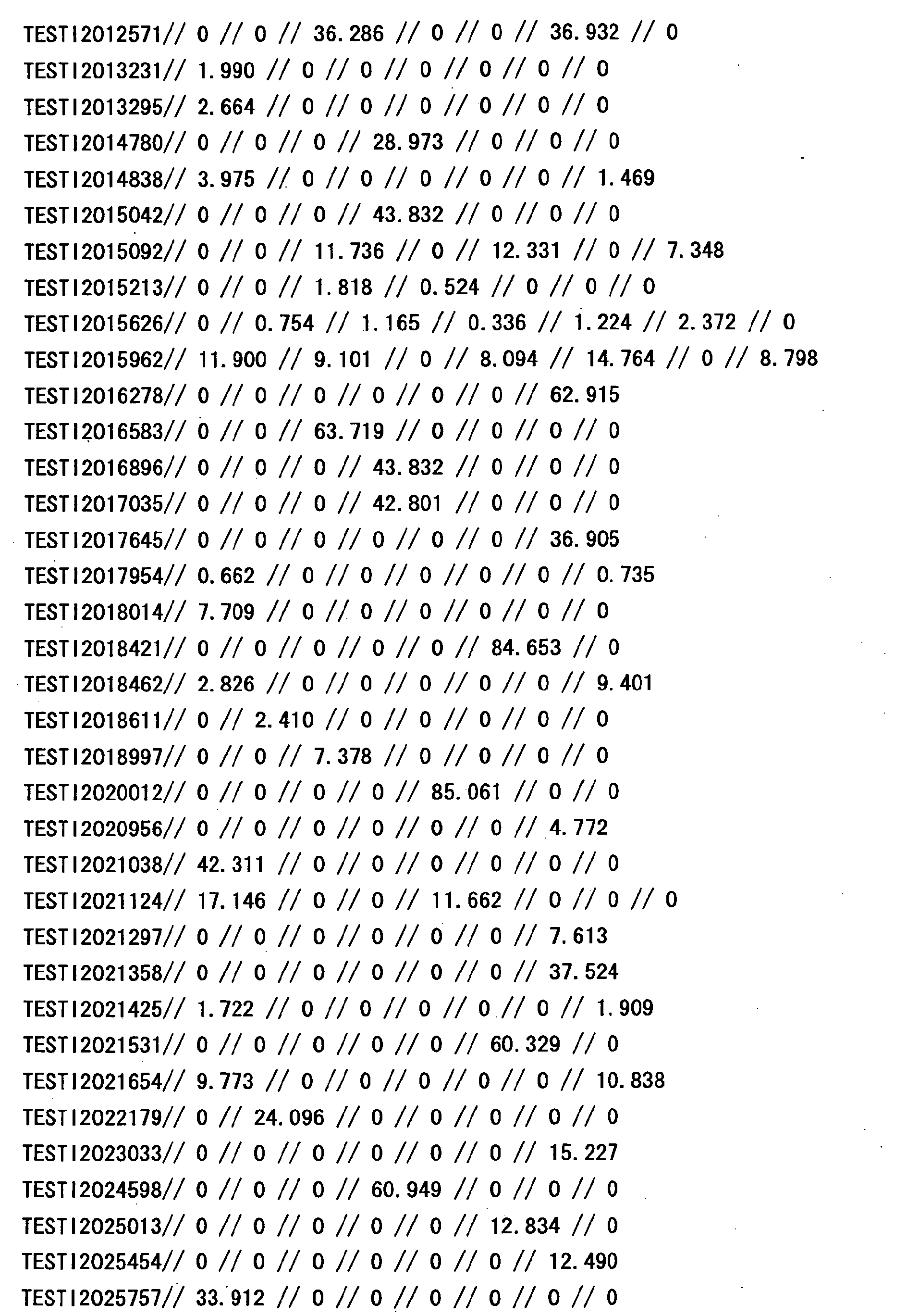

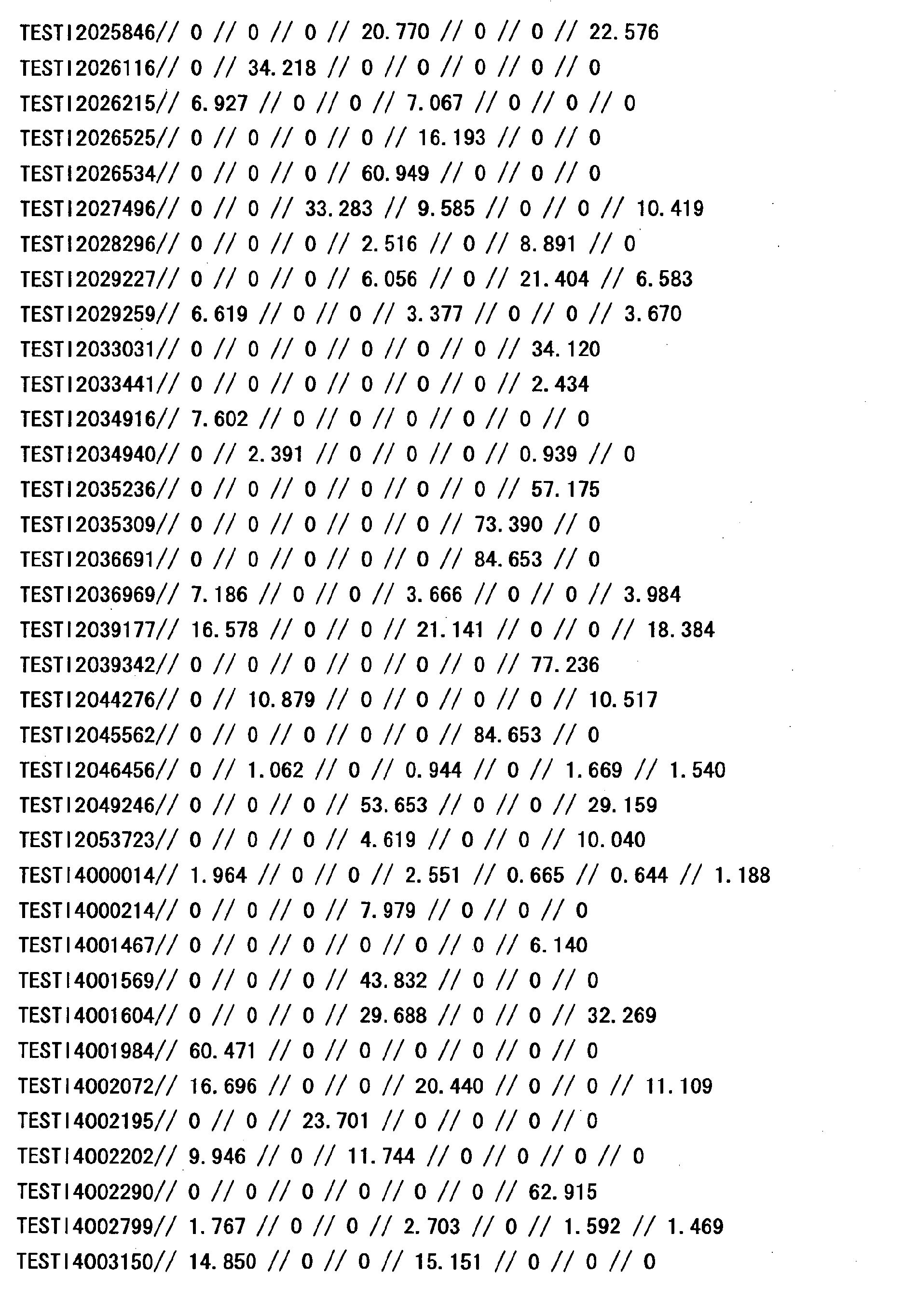

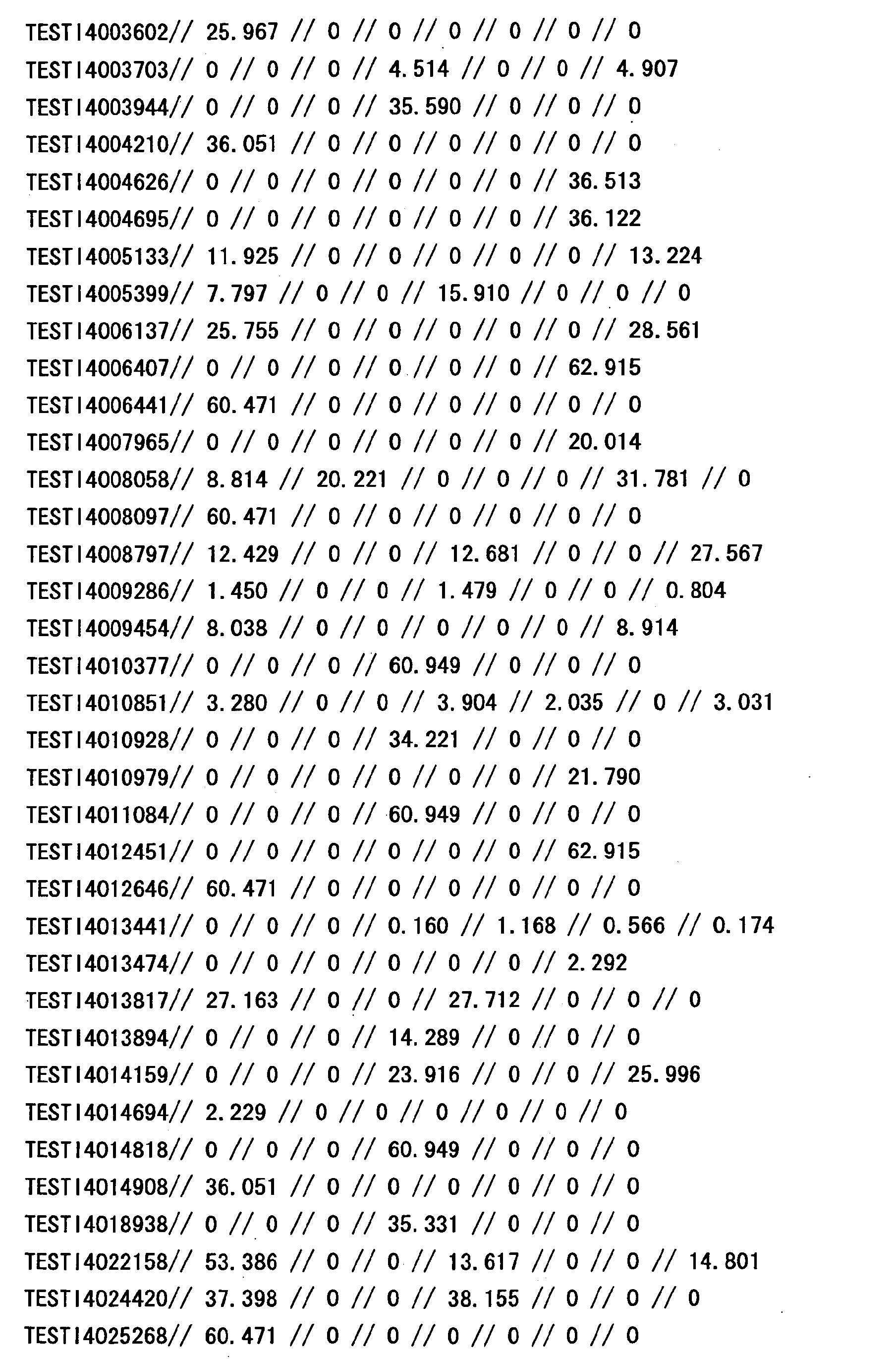

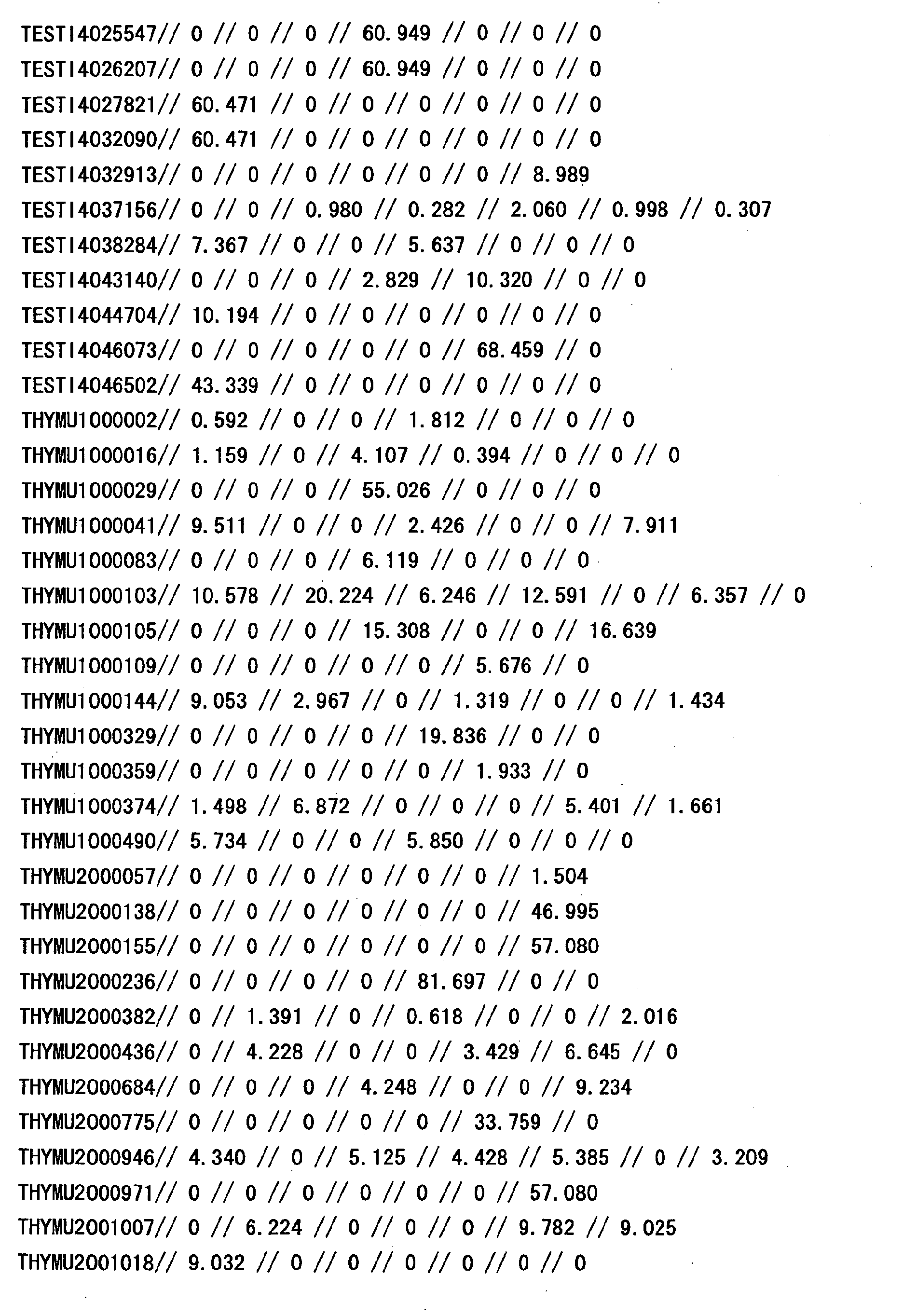

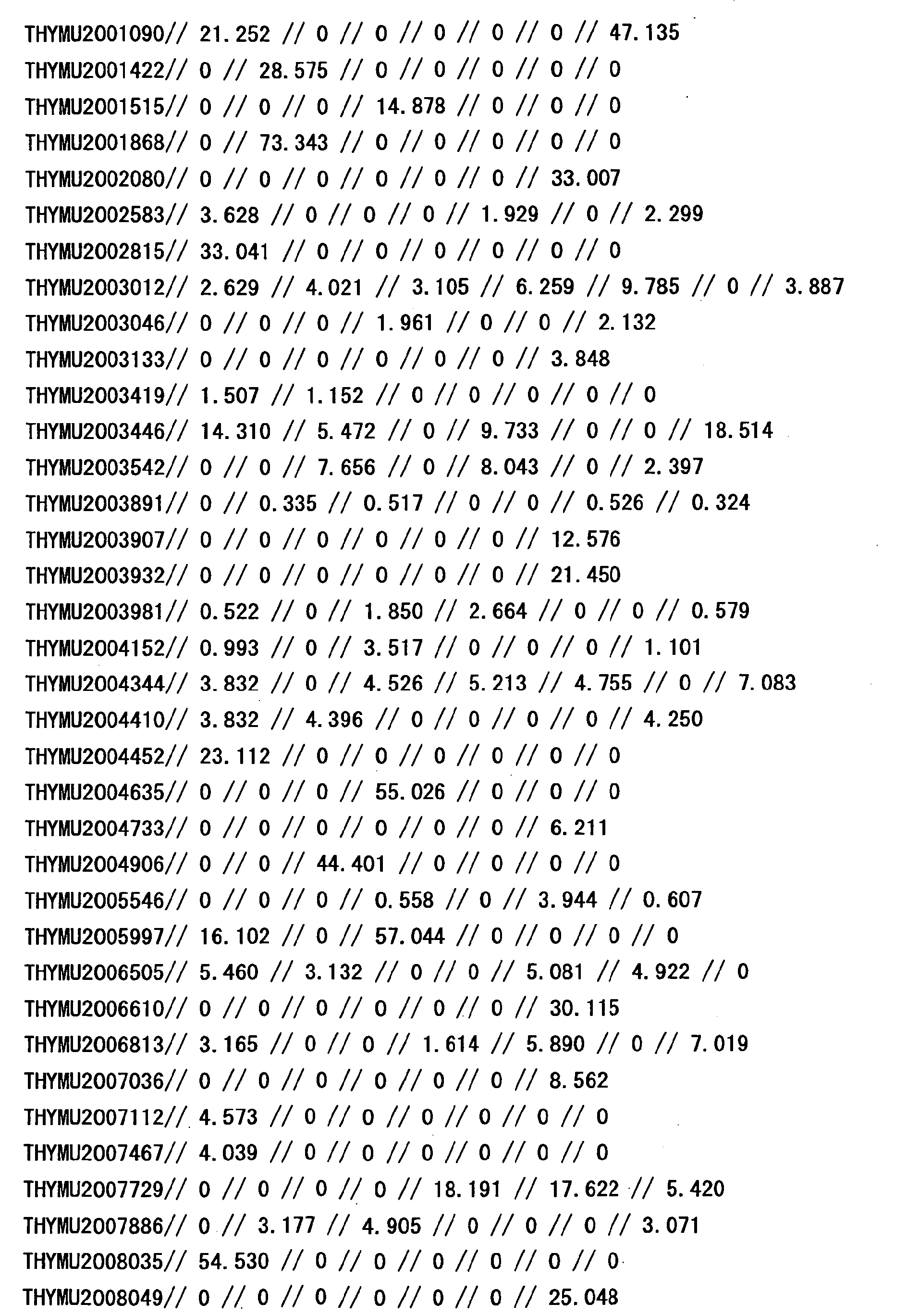

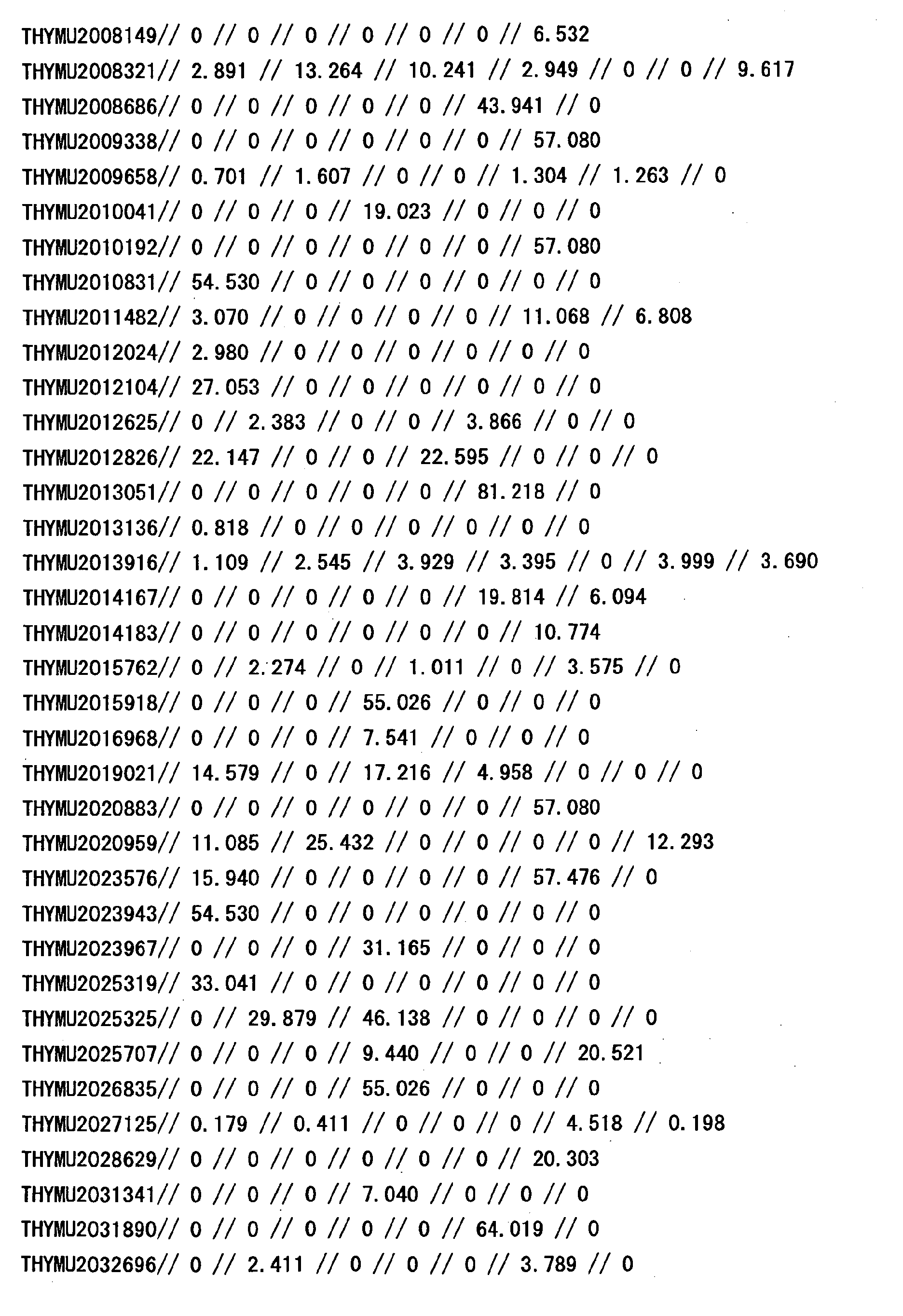

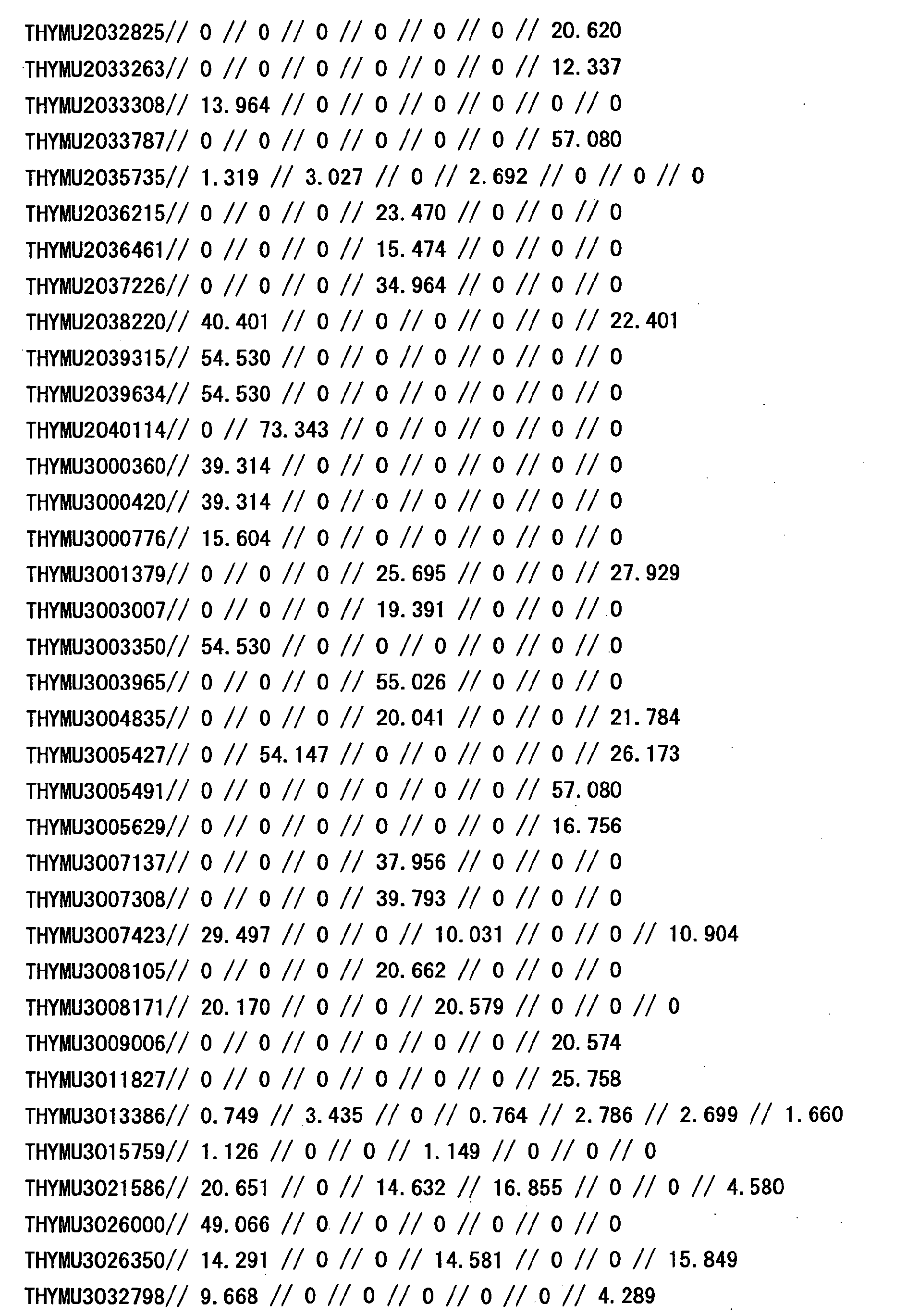

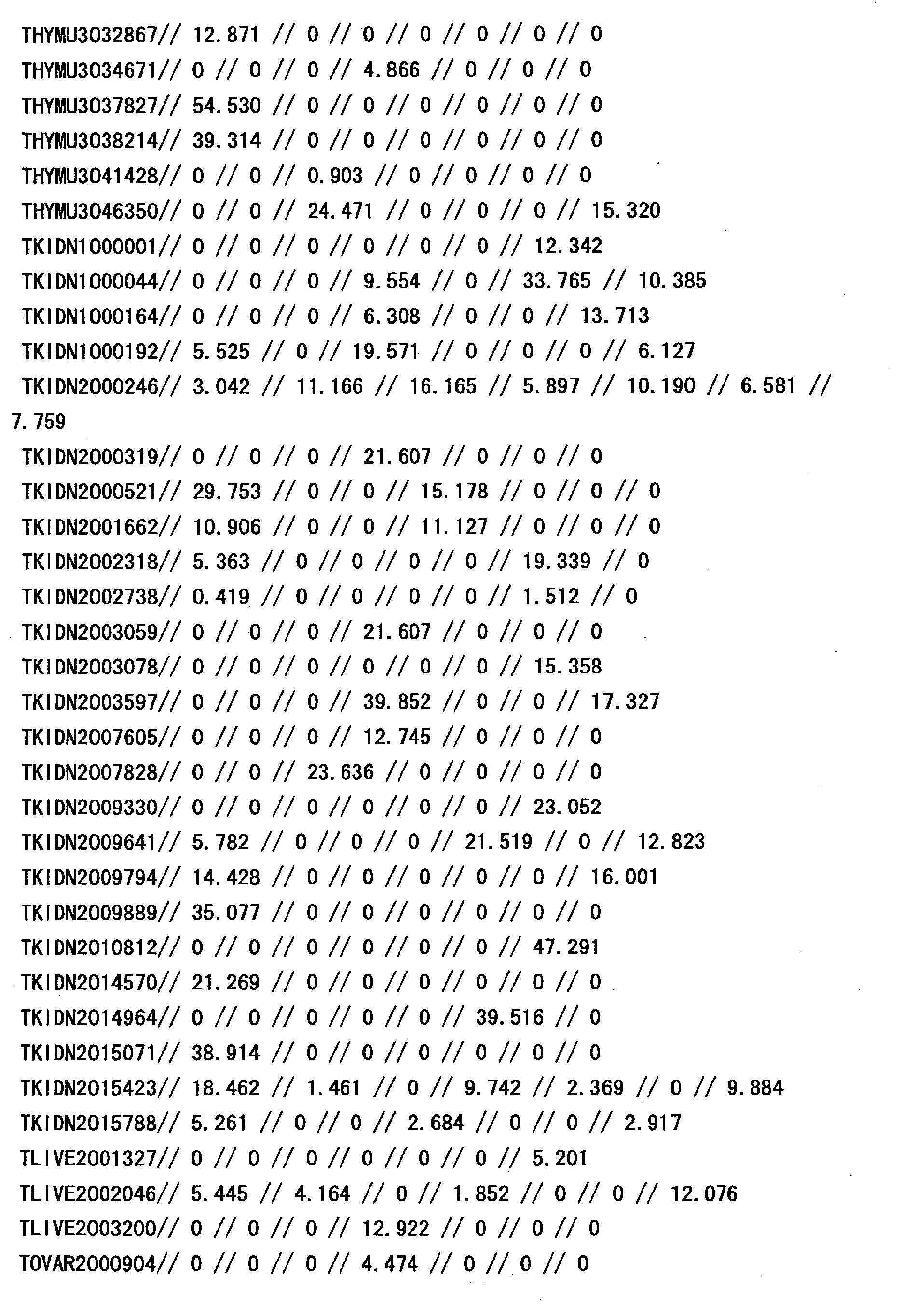

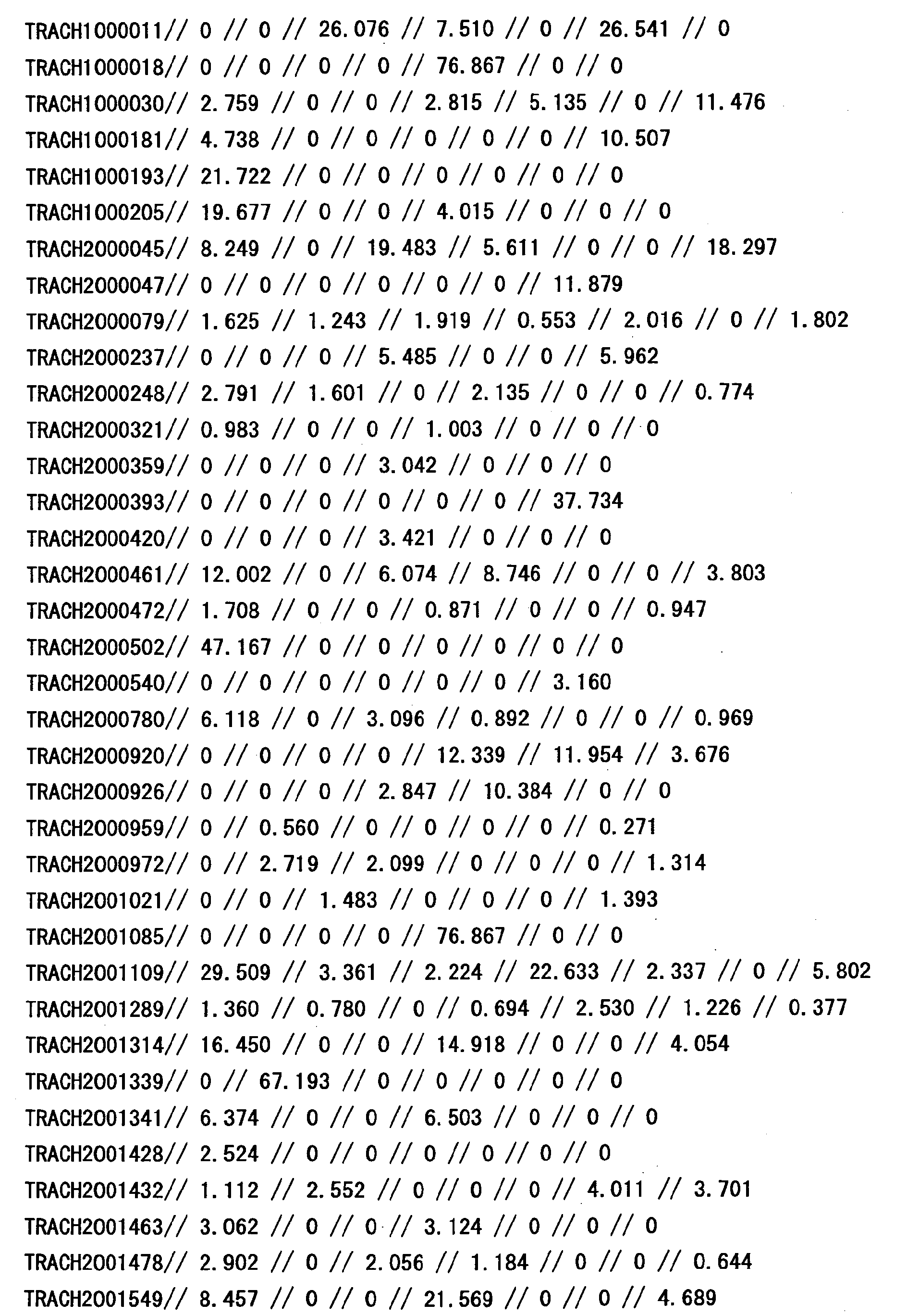

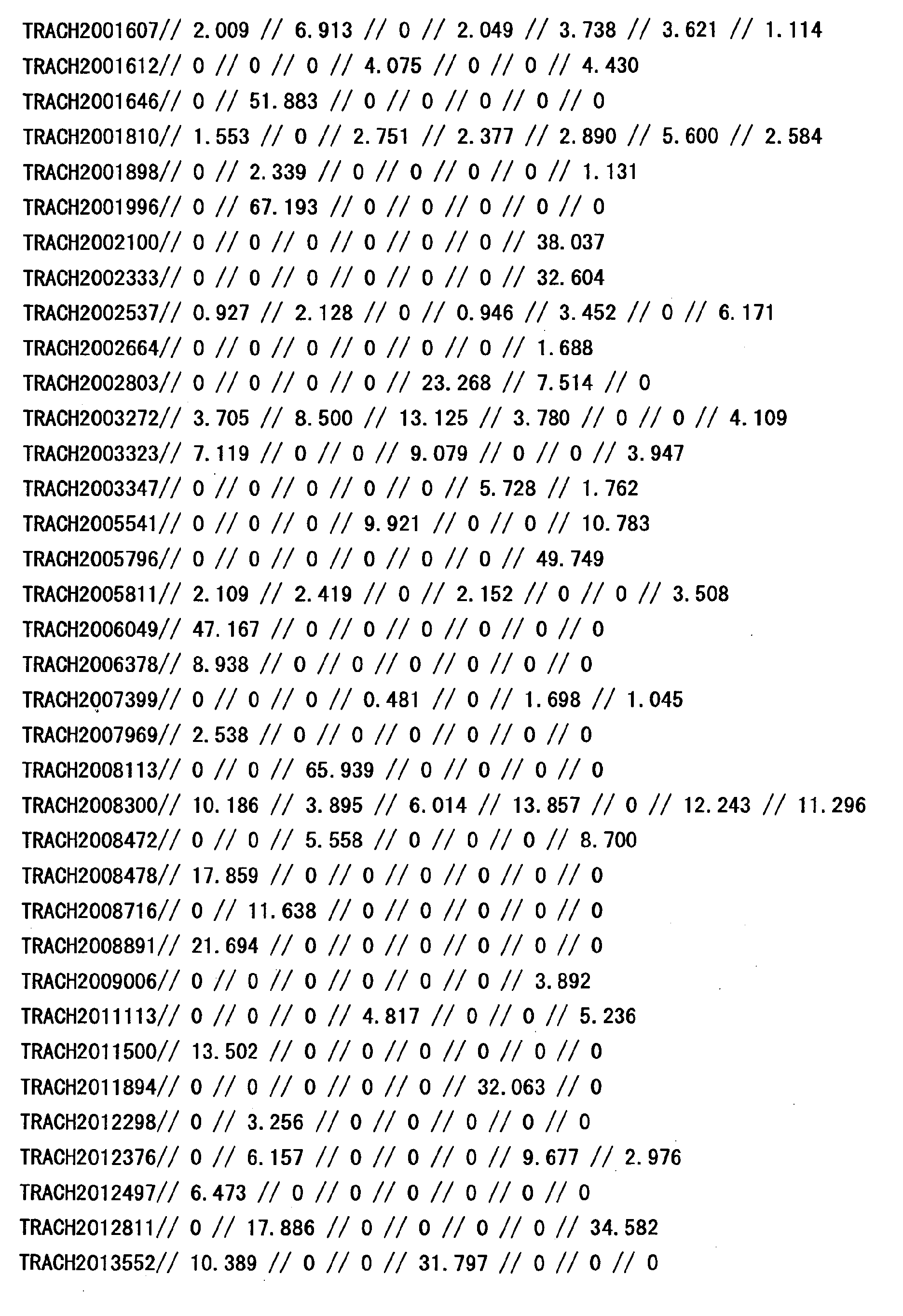

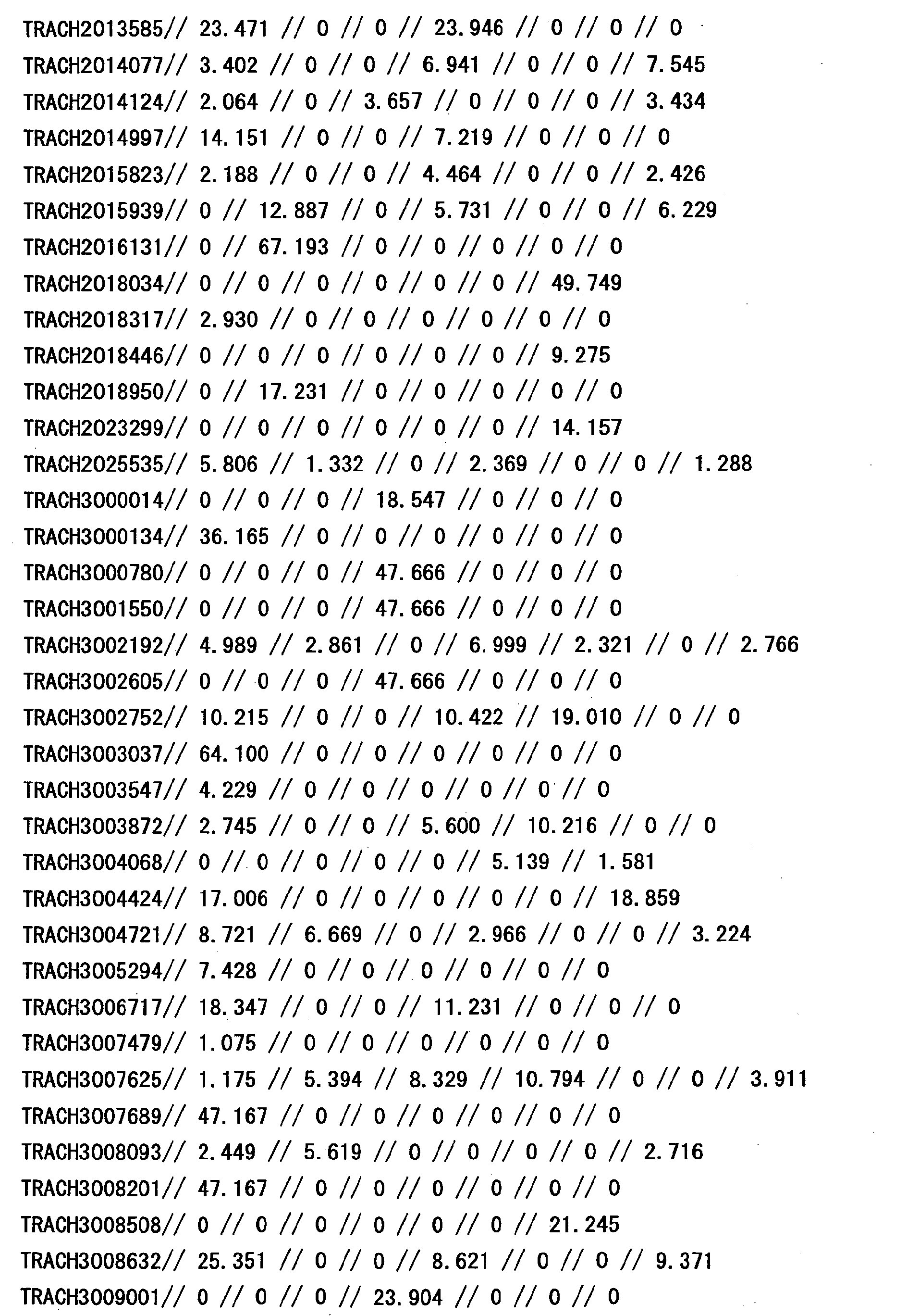

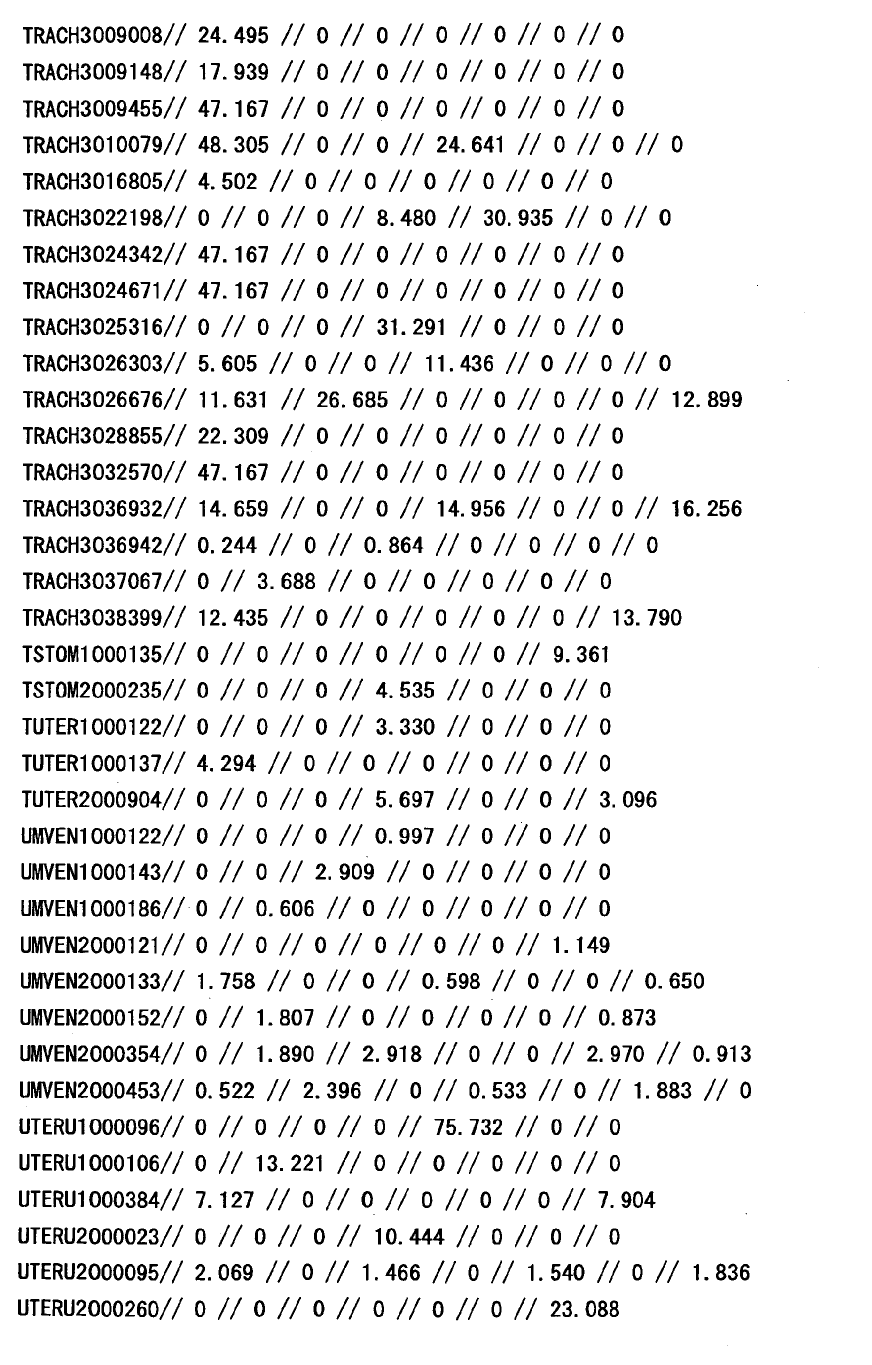

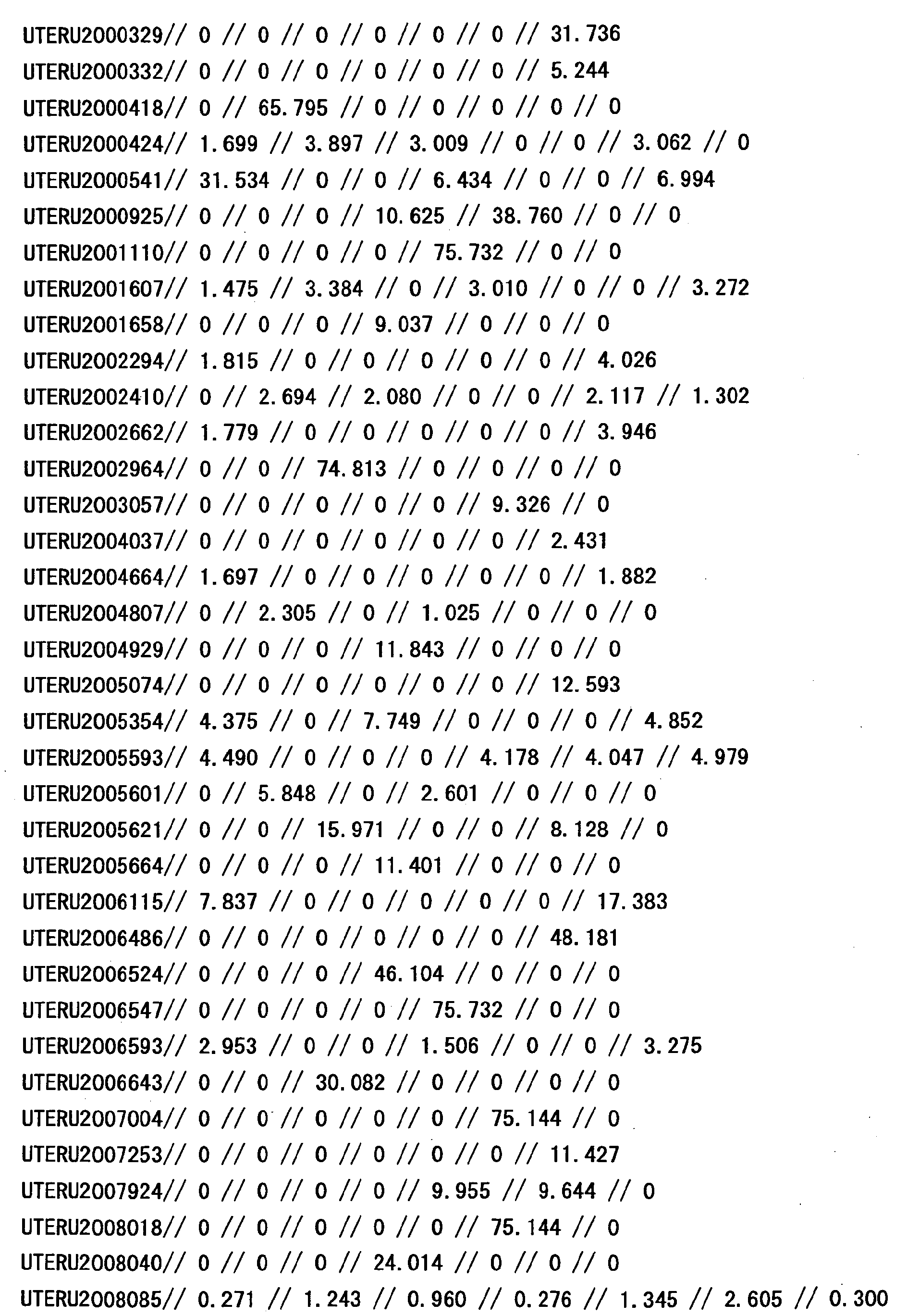

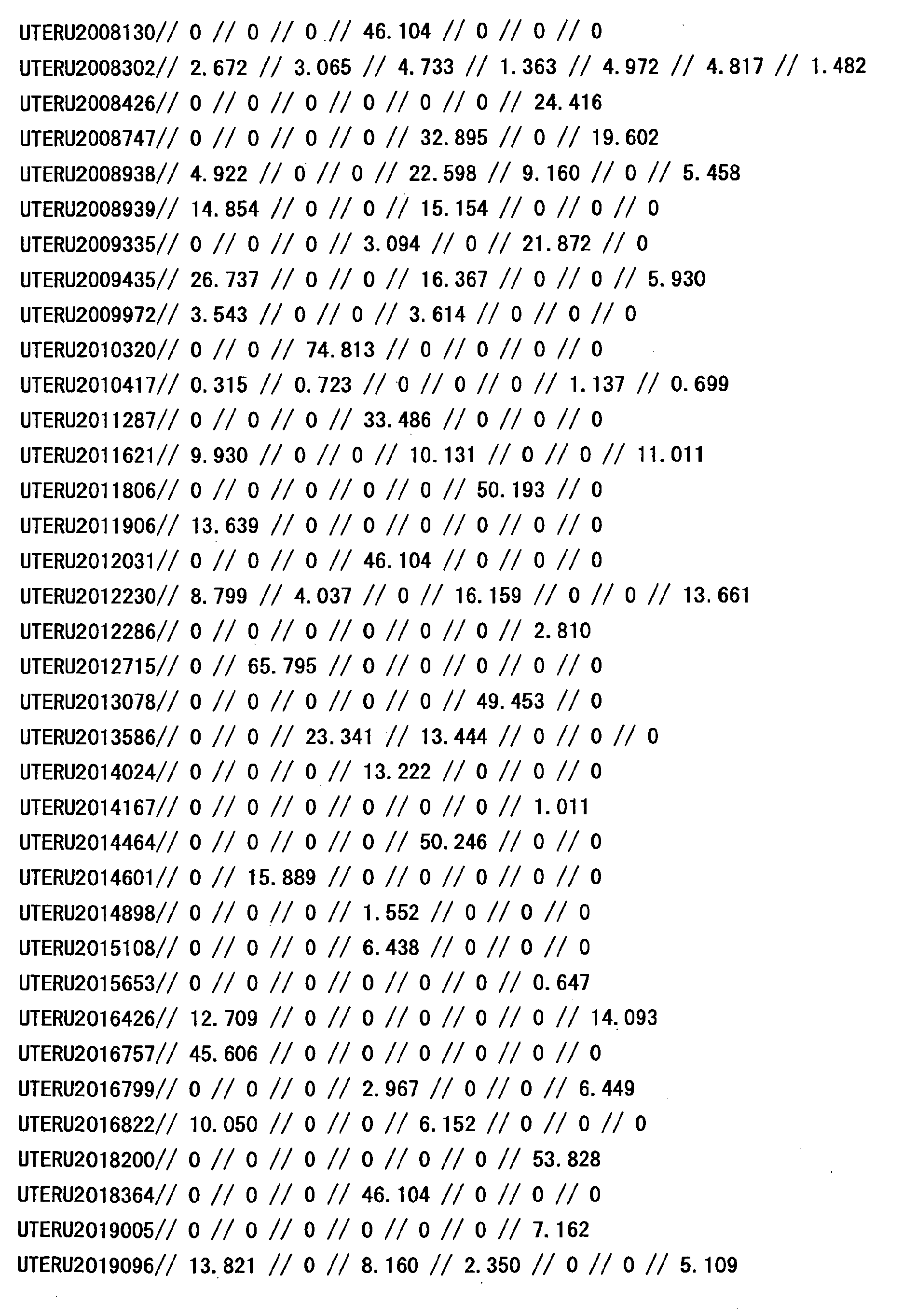

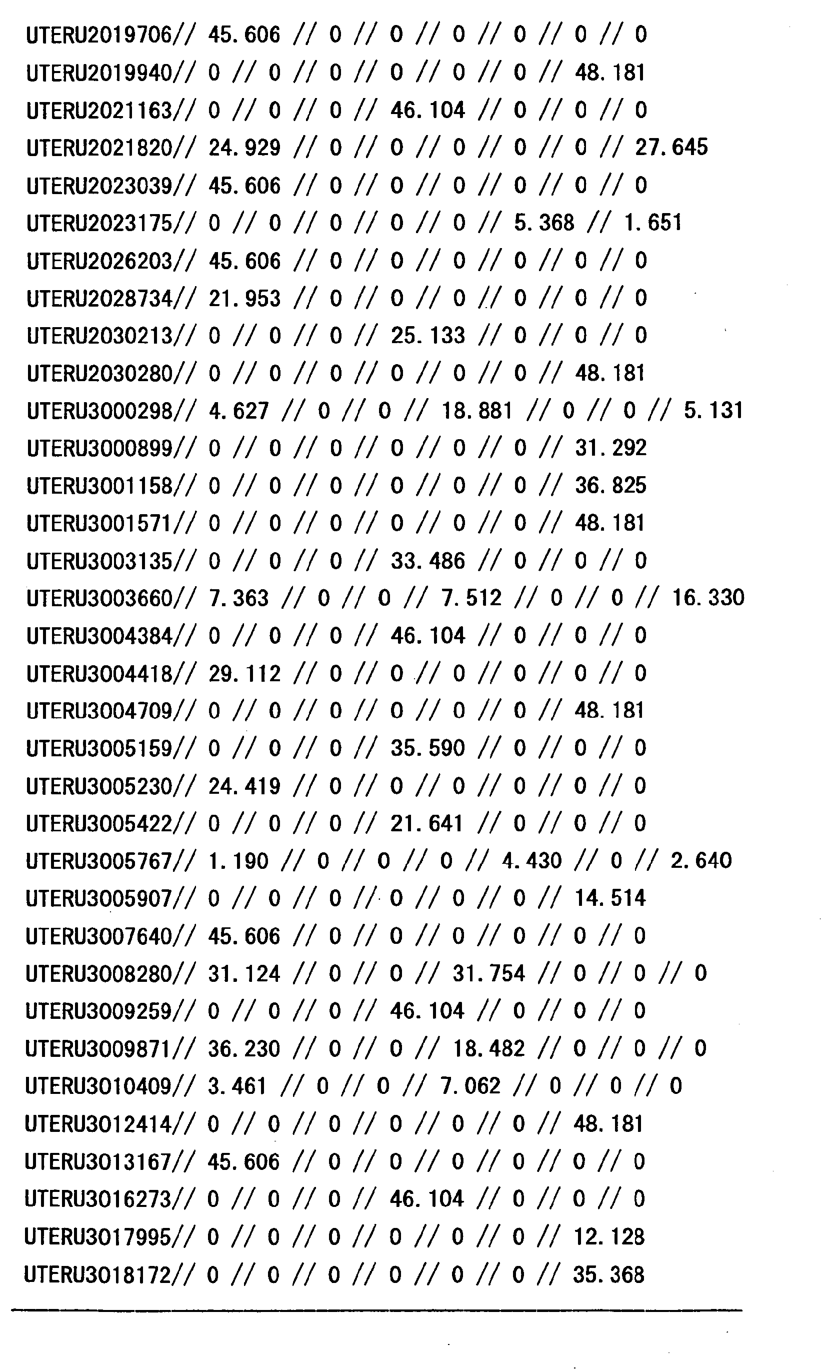

Genes whose expression levels were negligibly low in each of the above-described libraries are shown with their clone names below. 3NB691000018, 3NB691000085, 3NB691000113, 3NB691000129, 3NB692000012, 3NB692000029, 3NB692000102, 3NB692000154, 3NB692000276, 3NB692000305, 3NB692000429, 3NB692000484, 3NB692000545, 3NB692000973, 3NB692001002, 3NB692001034, 3NB692001040, 3NB692001123, 3NB692001267, 3NB692001339, 3NB692001349, 3NB692001366, 3NB692001408, 3NB692001459, 3NB692001471, 3NB692001496, 3NB692001501, 3NB692001507, 3NB692001511, 3NB692001519, 3NB692001520, 3NB692001538, 3NB692001548, 3NB692001637, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADRGL1000038, ADRGL1000067, ADRGL1000111, ADRGL1000147, ADRGL1000160, ADRGL1000165, ADRGL1000182, ADRGL2000056, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002029, ADRGL2002260, ADRGL2002392, ADRGL2002753, ADRGL2002857, ADRGL2003017, ADRGL2003329, ADRGL2003552, ADRGL2003585, ADRGL2003638, ADRGL2003684, ADRGL2003773, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010315, ADRGL2010594, ADRGL2010860, ADRGL2010974, ADRGL2011190, ADRGL2011330, ADRGL2012038, ADRGL2012179, AHMSC1000138, ASTRO1000018, ASTRO1000029, ASTRO1000096, ASTRO1000165, ASTRO2000011, ASTRO2000095, ASTRO2000141, ASTRO2000191, ASTRO2000278, ASTRO2000403, ASTRO2000417, ASTRO2000533, ASTRO2000653, ASTRO2000725, ASTRO2000801, ASTRO2001001, ASTRO2001008, ASTRO2001088, ASTRO2001122, ASTRO2001227, ASTRO2001249, ASTRO2001480, ASTRO2001806, ASTRO2002024, ASTRO2002064, ASTRO2002459, ASTRO2002571, ASTRO2002632, ASTRO2002659, ASTRO2002693, ASTRO2002720, ASTRO2002743, ASTRO2002842, ASTRO2003212, ASTRO2003581, ASTRO2003632, ASTRO2003925, ASTRO2003960, ASTRO2004032, ASTRO2004051, ASTRO2004177, ASTRO2004217, ASTRO2004297, ASTRO2004584, ASTRO2004751, ASTRO2004877, ASTRO2005008, ASTRO2005081, ASTRO2005096, ASTRO2005242, ASTRO2005273, ASTRO2005343, ASTRO2005413, ASTRO2005553, ASTRO2005557, ASTRO2005575, ASTRO2005593, ASTRO2005687, ASTRO2005726, ASTRO2005896, ASTRO2005902, ASTRO2006356, ASTRO2006732, ASTRO2006747, ASTRO2006920, ASTRO2006952, ASTRO2007047, ASTRO2007221, ASTRO2007377, ASTRO2007515, ASTRO2007578, ASTRO2007643, ASTRO2007666, ASTRO2007956, ASTRO2007962, ASTRO2008239, ASTRO2008409, ASTRO2008425, ASTRO2008508, ASTRO2008777, ASTRO2008895, ASTRO2008960, ASTRO2009118, ASTRO2009177, ASTRO2009333, ASTRO2009458, ASTRO2009526, ASTRO2009879, ASTRO2010582, ASTRO2010615, ASTRO2010962, ASTRO2010996, ASTRO2011149, ASTRO2011426, ASTRO2011437, ASTRO2011461, ASTRO2011834, ASTRO2011893, ASTRO2013124, ASTRO2013671, ASTRO2013802, ASTRO2014211, ASTRO2014363, ASTRO2014498, ASTRO2014561, ASTRO2014923, ASTRO2014961, ASTRO2015162, ASTRO2015185, ASTRO2015529, ASTRO2015987, ASTRO2016044, ASTRO2016148, ASTRO2016681, ASTRO2016819, ASTRO2016847, ASTRO2016892, ASTRO2017348, ASTRO2017627, ASTRO2018373, ASTRO2019039, ASTRO2019171, ASTRO3000154, ASTRO3000172, ASTRO3000177, ASTRO3000301, ASTRO3000474, BEAST2000454, BGGI11000193, BGGI11000285, BGGI12000022, BGGI12000067, BGGI12000161, BGGI12000533, BGGI12000544, BGGI12000684, BGGI12000693, BGGI12001097, BGGI12001241, BGGI12001247, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001458, BLADE2001575, BLADE2001753, BLADE2001935, BLADE2001940, BLADE2001987, BLADE2002073, BLADE2002310, BLADE2002730, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2005036, BLADE2005038, BLADE2005229, BLADE2005459, BLADE2005792, BLADE2006043, BLADE2006415, BLADE2006607, BLADE2006826, BLADE2006849, BLADE2006947, BLADE2007033, BLADE2007666, BLADE2007722, BLADE2007735, BLADE2007744, BLADE2007799, BLADE2007923, BLADE2007958, BLADE2008281, BLADE2008454, BLADE2008461, BLADE2008479, BLADE2008539, BLADE2008809, BLADE2008995, BLADE2009452, BLADE2009479, BLADE2009593, BNGH41000104, BNGH41000118, BNGH41000153, BNGH41000169, BNGH41000177, BNGH41000216, BNGH41000235, BNGH42000130, BNGH42000132, BNGH42000405, BNGH42000675, BNGH42000791, BNGH42000815, BNGH42000994, BNGH42001406, BNGH42001576, BNGH42001724, BNGH42002244, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42006226, BNGH42006234, BNGH42007037, BNGH42007366, BNGH42007594, BNGH42007798, BNGH42008199, BNGH42008510, BNGH42008649, BNGH42008832, BNGH42008850, BNGH42008943, BNGH42009021, BRACE1000047, BRACE1000084, BRACE1000087, BRACE1000092, BRACE1000093, BRACE1000114, BRACE1000124, BRACE1000132, BRACE1000166, BRACE1000239, BRACE1000451, BRACE1000475, BRACE1000572, BRACE2000061, BRACE2000077, BRACE2000078, BRACE2000100, BRACE2000148, BRACE2000183, BRACE2000229, BRACE2000232, BRACE2000256, BRACE2000266, BRACE2000267, BRACE2000351, BRACE2000368, BRACE2000392, BRACE2000441, BRACE2000487, BRACE2000489, BRACE2000505, BRACE2000526, BRACE2000531, BRACE2000545, BRACE2000553, BRACE2000577, BRACE2000640, BRACE2000650, BRACE2000677, BRACE2000718, BRACE2000743, BRACE2000753, BRACE2000815, BRACE2000885, BRACE2000905, BRACE2000936, BRACE2000947, BRACE2000965, BRACE2000969, BRACE2000988, BRACE2001017, BRACE2001094, BRACE2001107, BRACE2001117, BRACE2001151, BRACE2001191, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001492, BRACE2001508, BRACE2001543, BRACE2001564, BRACE2001588, BRACE2001607, BRACE2001626, BRACE2001677, BRACE2001705, BRACE2001733, BRACE2001737, BRACE2001742, BRACE2001777, BRACE2001779, BRACE2001855, BRACE2001859, BRACE2001865, BRACE2001872, BRACE2001881, BRACE2001898, BRACE2001923, BRACE2001942, BRACE2001954, BRACE2001972, BRACE2001973, BRACE2002026, BRACE2002034, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002394, BRACE2002409, BRACE2002431, BRACE2002441, BRACE2002444, BRACE2002458, BRACE2002467, BRACE2002468, BRACE2002589, BRACE2002590, BRACE2002613, BRACE2002614, BRACE2002617, BRACE2002623, BRACE2002635, BRACE2002649, BRACE2002661, BRACE2002676, BRACE2002685, BRACE2002695, BRACE2002707, BRACE2002736, BRACE2002752, BRACE2002755, BRACE2002762, BRACE2002772, BRACE2002792, BRACE2002796, BRACE2002807, BRACE2002834, BRACE2002849, BRACE2002857, BRACE2002861, BRACE2002884, BRACE2002948, BRACE2003110, BRACE2003168, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003540, BRACE2003594, BRACE2003614, BRACE2003628, BRACE2003639, BRACE2003713, BRACE2003766, BRACE2003802, BRACE2003825, BRACE2003847, BRACE2003848, BRACE2003873, BRACE2003885, BRACE2003887, BRACE2003928, BRACE2003954, BRACE2003987, BRACE2005034, BRACE2005047, BRACE2005063, BRACE2005090, BRACE2005118, BRACE2005124, BRACE2005138, BRACE2005160, BRACE2005169, BRACE2005189, BRACE2005196, BRACE2005198, BRACE2005216, BRACE2005243, BRACE2005298, BRACE2005328, BRACE2005329, BRACE2005337, BRACE2005348, BRACE2005363, BRACE2005433, BRACE2005450, BRACE2005453, BRACE2005460, BRACE2005489, BRACE2005518, BRACE2005556, BRACE2005562, BRACE2005624, BRACE2005661, BRACE2005667, BRACE2005731, BRACE2005742, BRACE2005762, BRACE2005773, BRACE2005787, BRACE2005795, BRACE2005800, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006072, BRACE2006102, BRACE2006105, BRACE2006122, BRACE2006137, BRACE2006143, BRACE2006162, BRACE2006174, BRACE2006240, BRACE2006245, BRACE2006249, BRACE2006274, BRACE2006298, BRACE2006319, BRACE2006378, BRACE2006393, BRACE2006413, BRACE2006417, BRACE2006453, BRACE2006459, BRACE2006488, BRACE2006534, BRACE2006540, BRACE2006547, BRACE2006564, BRACE2006587, BRACE2006598, BRACE2006685, BRACE2006703, BRACE2006783, BRACE2006833, BRACE2006900, BRACE2006909, BRACE2006911, BRACE2006924, BRACE2006935, BRACE2006944, BRACE2006951, BRACE2006986, BRACE2007068, BRACE2007087, BRACE2007138, BRACE2007174, BRACE2007197, BRACE2007203, BRACE2007232, BRACE2007262, BRACE2007274, BRACE2007281, BRACE2007295, BRACE2007358, BRACE2007396, BRACE2007398, BRACE2007411, BRACE2007447, BRACE2007477, BRACE2007502, BRACE2007503, BRACE2007527, BRACE2007538, BRACE2007563, BRACE2007621, BRACE2007640, BRACE2007646, BRACE2007682, BRACE2007685, BRACE2007727, BRACE2007749, BRACE2007760, BRACE2007761, BRACE2007764, BRACE2007769, BRACE2007784, BRACE2007786, BRACE2007799, BRACE2007801, BRACE2007836, BRACE2007868, BRACE2007888, BRACE2007902, BRACE2007920, BRACE2007944, BRACE2008008, BRACE2008015, BRACE2008021, BRACE2008102, BRACE2008114, BRACE2008144, BRACE2008172, BRACE2008218, BRACE2008276, BRACE2008295, BRACE2008308, BRACE2008336, BRACE2008361, BRACE2008401, BRACE2008410, BRACE2008420, BRACE2008443, BRACE2008473, BRACE2008480, BRACE2008481, BRACE2008488, BRACE2008495, BRACE2008500, BRACE2008594, BRACE2008604, BRACE2008615, BRACE2008622, BRACE2008629, BRACE2008655, BRACE2008656, BRACE2008710, BRACE2008754, BRACE2008797, BRACE2008847, BRACE2008861, BRACE2008867, BRACE2008881, BRACE2008883, BRACE2008917, BRACE2008919, BRACE2008948, BRACE2008960, BRACE2008999, BRACE2009014, BRACE2009016, BRACE2009031, BRACE2009044, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009311, BRACE2009318, BRACE2009323, BRACE2009421, BRACE2009428, BRACE2009434, BRACE2009437, BRACE2009533, BRACE2009558, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010535, BRACE2010550, BRACE2010598, BRACE2010632, BRACE2010642, BRACE2010684, BRACE2010813, BRACE2010828, BRACE2010837, BRACE2010888, BRACE2010937, BRACE2010983, BRACE2011007, BRACE2011025, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2014108, BRACE2014161, BRACE2014232, BRACE2014268, BRACE2014475, BRACE2014780, BRACE2014818, BRACE2014821, BRACE2014824, BRACE2014854, BRACE2015031, BRACE2015262, BRACE2015270, BRACE2015314, BRACE2015352, BRACE2015436, BRACE2015741, BRACE2015756, BRACE2015969, BRACE2015995, BRACE2016058, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017410, BRACE2017438, BRACE2017580, BRACE2017587, BRACE2017720, BRACE2017872, BRACE2017929, BRACE2017985, BRACE2017992, BRACE2018443, BRACE2018448, BRACE2018568, BRACE2018700, BRACE2018727, BRACE2018759, BRACE2018782, BRACE2019044, BRACE2019244, BRACE2019258, BRACE2019371, BRACE2019467, BRACE2019510, BRACE2019528, BRACE2019618, BRACE2019696, BRACE2019703, BRACE2019843, BRACE2019865, BRACE2019872, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021721, BRACE2021936, BRACE2022030, BRACE2022158, BRACE2022196, BRACE2022270, BRACE2022328, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023268, BRACE2023284, BRACE2023404, BRACE2023451, BRACE2023540, BRACE2023633, BRACE2023727, BRACE2023744, BRACE2023800, BRACE2023801, BRACE2024074, BRACE2024222, BRACE2024610, BRACE2024627, BRACE2024826, BRACE2025018, BRACE2025316, BRACE2025333, BRACE2025452, BRACE2025492, BRACE2025710, BRACE2025796, BRACE2025992, BRACE2026054, BRACE2026131, BRACE2026141, BRACE2026293, BRACE2026294, BRACE2026404, BRACE2026675, BRACE2026725, BRACE2026836, BRACE2026920, BRACE2026971, BRACE2027221, BRACE2027258, BRACE2027312, BRACE2027382, BRACE2027389, BRACE2027408, BRACE2027770, BRACE2027879, BRACE2027970, BRACE2028171, BRACE2028410, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030345, BRACE2030736, BRACE2030883, BRACE2030884, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035441, BRACE2035485, BRACE2036005, BRACE2036096, BRACE2036743, BRACE2036830, BRACE2036834, BRACE2036900, BRACE2037294, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044263, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003413, BRACE3003595, BRACE3003698, BRACE3003866, BRACE3003920, BRACE3004058, BRACE3004113, BRACE3004150, BRACE3004205, BRACE3004358, BRACE3004435, BRACE3004477, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005870, BRACE3005903, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007322, BRACE3007472, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008720, BRACE3008772, BRACE3009044, BRACE3009075, BRACE3009090, BRACE3009127, BRACE3009197, BRACE3009265, BRACE3009297, BRACE3009377, BRACE3009392, BRACE3009539, BRACE3009574, BRACE3009701, BRACE3009708, BRACE3009993, BRACE3010076, BRACE3010079, BRACE3010086, BRACE3010221, BRACE3010283, BRACE3010315, BRACE3010416, BRACE3010435, BRACE3010702, BRACE3010862, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3019055, BRACE3019084, BRACE3019570, BRACE3019611, BRACE3019817, BRACE3019828, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3026008, BRACE3026075, BRACE3026161, BRACE3026290, BRACE3026345, BRACE3026456, BRACE3026844, BRACE3026947, BRACE3026993, BRACE3027242, BRACE3027256, BRACE3027326, BRACE3027480, BRACE3027976, BRACE3028214, BRACE3028241, BRACE3028360, BRACE3028895, BRACE3028998, BRACE3029005, BRACE3029205, BRACE3029447, BRACE3030057, BRACE3030103, BRACE3030538, BRACE3030748, BRACE3031161, BRACE3031184, BRACE3031315, BRACE3031372, BRACE3031579, BRACE3031728, BRACE3031843, BRACE3032455, BRACE3032537, BRACE3032538, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039378, BRACE3039454, BRACE3040012, BRACE3040239, BRACE3040504, BRACE3040644, BRACE3040856, BRACE3041059, BRACE3041162, BRACE3041827, BRACE3042046, BRACE3042210, BRACE3042409, BRACE3042594, BRACE3043597, BRACE3044090, BRACE3044172, BRACE3044247, BRACE3044377, BRACE3044389, BRACE3044495, BRACE3045011, BRACE3045033, BRACE3045145, BRACE3045424, BRACE3045708, BRACE3046049, BRACE3046152, BRACE3046294, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2001038, BRALZ2001279, BRALZ2001350, BRALZ2001445, BRALZ2001559, BRALZ2001743, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008763, BRALZ2008869, BRALZ2008930, BRALZ2010186, BRALZ2010842, BRALZ2011337, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRAMY1000095, BRAMY1000098, BRAMY1000130, BRAMY1000157, BRAMY2000021, BRAMY2000076, BRAMY2000086, BRAMY2000151, BRAMY2000181, BRAMY2000196, BRAMY2000231, BRAMY2000277, BRAMY2000653, BRAMY2000814, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002493, BRAMY2002525, BRAMY2002584, BRAMY2002604, BRAMY2002638, BRAMY2002739, BRAMY2002853, BRAMY2002862, BRAMY2002956, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004058, BRAMY2004183, BRAMY2004335, BRAMY2004351, BRAMY2004352, BRAMY2004492, BRAMY2004537, BRAMY2004542, BRAMY2004756, BRAMY2004771, BRAMY2005064, BRAMY2005094, BRAMY2005151, BRAMY2005182, BRAMY2005244, BRAMY2005391, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007249, BRAMY2007308, BRAMY2007411, BRAMY2007458, BRAMY2007486, BRAMY2007546, BRAMY2007610, BRAMY2007653, BRAMY2007753, BRAMY2007859, BRAMY2008333, BRAMY2008382, BRAMY2008583, BRAMY2008979, BRAMY2009034, BRAMY2009105, BRAMY2009123, BRAMY2009192, BRAMY2009344, BRAMY2009349, BRAMY2009394, BRAMY2009528, BRAMY2009693, BRAMY2010068, BRAMY2010135, BRAMY2010145, BRAMY2010171, BRAMY2010208, BRAMY2010239, BRAMY2010290, BRAMY2010366, BRAMY2010395, BRAMY2010441, BRAMY2010581, BRAMY2010798, BRAMY2010808, BRAMY2010919, BRAMY2011064, BRAMY2011178, BRAMY2011196, BRAMY2011216, BRAMY2011253, BRAMY2011480, BRAMY2011639, BRAMY2011679, BRAMY2011811, BRAMY2011846, BRAMY2011872, BRAMY2011961, BRAMY2012017, BRAMY2012091, BRAMY2012119, BRAMY2012162, BRAMY2012277, BRAMY2012426, BRAMY2012497, BRAMY2012517, BRAMY2012555, BRAMY2012568, BRAMY2012581, BRAMY2012691, BRAMY2012721, BRAMY2012731, BRAMY2012776, BRAMY2012880, BRAMY2013200, BRAMY2013216, BRAMY2013405, BRAMY2013414, BRAMY2013572, BRAMY2013590, BRAMY2013621, BRAMY2013659, BRAMY2013722, BRAMY2013756, BRAMY2013944, BRAMY2013975, BRAMY2014019, BRAMY2014387, BRAMY2014469, BRAMY2014541, BRAMY2014813, BRAMY2015211, BRAMY2015311, BRAMY2015503, BRAMY2015516, BRAMY2015551, BRAMY2015559, BRAMY2015688, BRAMY2015705, BRAMY2015782, BRAMY2015855, BRAMY2015890, BRAMY2015925, BRAMY2016002, BRAMY2016020, BRAMY2016070, BRAMY2016327, BRAMY2016386, BRAMY2016706, BRAMY2016771, BRAMY2016938, BRAMY2016953, BRAMY2017014, BRAMY2017249, BRAMY2017348, BRAMY2017450, BRAMY2017455, BRAMY2017528, BRAMY2018027, BRAMY2018106, BRAMY2018122, BRAMY2018126, BRAMY2018272, BRAMY2018273, BRAMY2018279, BRAMY2018308, BRAMY2018396, BRAMY2018467, BRAMY2019055, BRAMY2019277, BRAMY2019509, BRAMY2019643, BRAMY2019985, BRAMY2020050, BRAMY2020058, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021310, BRAMY2021388, BRAMY2021498, BRAMY2021523, BRAMY2021710, BRAMY2021732, BRAMY2021746, BRAMY2021962, BRAMY2021982, BRAMY2022052, BRAMY2022263, BRAMY2022301, BRAMY2022320, BRAMY2022383, BRAMY2022445, BRAMY2022454, BRAMY2022532, BRAMY2022796, BRAMY2022929, BRAMY2022984, BRAMY2023060, BRAMY2023110, BRAMY2023161, BRAMY2023172, BRAMY2023238, BRAMY2023327, BRAMY2023358, BRAMY2023482, BRAMY2023514, BRAMY2023719, BRAMY2023729, BRAMY2023786, BRAMY2023852, BRAMY2023863, BRAMY2023939, BRAMY2024073, BRAMY2024374, BRAMY2024449, BRAMY2024489, BRAMY2024497, BRAMY2024514, BRAMY2024530, BRAMY2024535, BRAMY2024545, BRAMY2024576, BRAMY2024715, BRAMY2024728, BRAMY2024849, BRAMY2025024, BRAMY2025032, BRAMY2025175, BRAMY2025218, BRAMY2025230, BRAMY2025277, BRAMY2025495, BRAMY2025812, BRAMY2025996, BRAMY2026009, BRAMY2026091, BRAMY2026300, BRAMY2026347, BRAMY2026395, BRAMY2026405, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027140, BRAMY2027242, BRAMY2027396, BRAMY2027451, BRAMY2027461, BRAMY2027467, BRAMY2027714, BRAMY2027752, BRAMY2027782, BRAMY2028072, BRAMY2028188, BRAMY2028282, BRAMY2028472, BRAMY2028491, BRAMY2028565, BRAMY2028593, BRAMY2028682, BRAMY2028783, BRAMY2028856, BRAMY2029204, BRAMY2029602, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031377, BRAMY2031442, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035801, BRAMY2035869, BRAMY2035923, BRAMY2036079, BRAMY2036254, BRAMY2036266, BRAMY2036387, BRAMY2036396, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037971, BRAMY2038100, BRAMY2038128, BRAMY2038317, BRAMY2038475, BRAMY2038484, BRAMY2038832, BRAMY2038846, BRAMY2038887, BRAMY2038904, BRAMY2039287, BRAMY2039300, BRAMY2039314, BRAMY2039341, BRAMY2039695, BRAMY2039872, BRAMY2040051, BRAMY2040159, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002508, BRAMY3002620, BRAMY3002805, BRAMY3002886, BRAMY3003026, BRAMY3003109, BRAMY3003205, BRAMY3003310, BRAMY3003566, BRAMY3003723, BRAMY3004364, BRAMY3004672, BRAMY3004900, BRAMY3004919, BRAMY3005656, BRAMY3005932, BRAMY3006032, BRAMY3006297, BRAMY3007206, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009904, BRAMY3010321, BRAMY3010411, BRAMY3010603, BRAMY4000277, BRAMY4000962, BRAMY4001173, BRAMY4001200, BRAMY4001234, BRAMY4001449, BRAMY4001652, BRAMY4001913, BRAMY4002575, BRAMY4002628, BRASW1000053, CD34C2000051, CD34C2000152, CD34C2000175, CD34C2000443, CD34C3000314, CD34C3000494, CERVX1000042, CERVX2000812, CERVX2000968, CERVX2002006, CERVX2002013, CERVX2002430, CERVX2002770, CHONS1000058, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2008105, COLON2009337, COLON2009499, CORDB1000140, CORDB2000061, CORDB2000127, CORDB2000541, CTONG1000009, CTONG1000020, CTONG1000022, CTONG1000040, CTONG1000056, CTONG1000062, CTONG1000086, CTONG1000093, CTONG1000097, CTONG1000113, CTONG1000137, CTONG1000165, CTONG1000175, CTONG1000181, CTONG1000220, CTONG1000277, CTONG1000283, CTONG1000288, CTONG1000302, CTONG1000341, CTONG1000476, CTONG1000488, CTONG1000508, CTONG1000540, CTONG2000034, CTONG2000201, CTONG2000218, CTONG2000279, CTONG2000303, CTONG2000330, CTONG2000395, CTONG2000465, CTONG2000480, CTONG2000561, CTONG2000589, CTONG2000766, CTONG2000771, CTONG2000819, CTONG2000846, CTONG2000855, CTONG2000977, CTONG2001139, CTONG2001366, CTONG2001413, CTONG2001428, CTONG2001513, CTONG2001524, CTONG2001598, CTONG2001681, CTONG2001724, CTONG2001749, CTONG2001820, CTONG2001858, CTONG2001877, CTONG2001932, CTONG2001955, CTONG2002066, CTONG2002143, CTONG2002395, CTONG2002417, CTONG2002418, CTONG2002453, CTONG2002520, CTONG2002558, CTONG2002590, CTONG2002709, CTONG2002721, CTONG2002816, CTONG2002820, CTONG2002903, CTONG2002965, CTONG2002999, CTONG2003028, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004397, CTONG2004454, CTONG2004550, CTONG2004669, CTONG2004716, CTONG2004912, CTONG2004941, CTONG2004948, CTONG2005049, CTONG2005110, CTONG2005265, CTONG2005290, CTONG2005374, CTONG2005399, CTONG2005468, CTONG2005567, CTONG2005585, CTONG2005615, CTONG2005635, CTONG2005775, CTONG2005890, CTONG2005904, CTONG2005913, CTONG2006129, CTONG2006223, CTONG2006273, CTONG2006310, CTONG2006515, CTONG2006524, CTONG2006568, CTONG2006611, CTONG2006620, CTONG2006666, CTONG2006691, CTONG2006798, CTONG2006836, CTONG2006932, CTONG2006942, CTONG2006964, CTONG2007007, CTONG2007009, CTONG2007091, CTONG2007175, CTONG2007259, CTONG2007272, CTONG2007297, CTONG2007441, CTONG2007474, CTONG2007623, CTONG2007898, CTONG2007959, CTONG2007970, CTONG2008184, CTONG2008262, CTONG2008269, CTONG2008343, CTONG2008402, CTONG2008466, CTONG2008562, CTONG2008577, CTONG2008595, CTONG2008621, CTONG2008720, CTONG2008773, CTONG2008792, CTONG2008989, CTONG2009132, CTONG2009156, CTONG2009181, CTONG2009257, CTONG2009258, CTONG2009268, CTONG2009270, CTONG2009395, CTONG2009440, CTONG2009527, CTONG2009531, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009955, CTONG2009958, CTONG2009963, CTONG2010018, CTONG2010024, CTONG2010116, CTONG2010133, CTONG2010148, CTONG2010276, CTONG2010330, CTONG2010508, CTONG2010566, CTONG2010623, CTONG2010649, CTONG2010803, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012029, CTONG2012077, CTONG2012101, CTONG2012123, CTONG2012158, CTONG2012230, CTONG2012401, CTONG2012422, CTONG2012425, CTONG2012447, CTONG2012453, CTONG2012473, CTONG2012476, CTONG2012533, CTONG2012554, CTONG2012564, CTONG2012661, CTONG2012724, CTONG2012738, CTONG2012758, CTONG2012843, CTONG2012901, CTONG2013128, CTONG2013149, CTONG2013156, CTONG2013178, CTONG2013222, CTONG2013284, CTONG2013339, CTONG2013352, CTONG2013393, CTONG2013511, CTONG2013630, CTONG2013803, CTONG2013985, CTONG2013986, CTONG2014032, CTONG2014058, CTONG2014191, CTONG2014206, CTONG2014234, CTONG2014264, CTONG2014280, CTONG2014491, CTONG2014630, CTONG2014635, CTONG2014646, CTONG2014705, CTONG2014946, CTONG2014954, CTONG2014959, CTONG2014966, CTONG2014995, CTONG2015316, CTONG2015358, CTONG2015434, CTONG2015540, CTONG2015596, CTONG2015633, CTONG2015678, CTONG2015717, CTONG2015804, CTONG2015866, CTONG2015914, CTONG2015953, CTONG2016185, CTONG2016217, CTONG2016355, CTONG2016505, CTONG2016559, CTONG2016658, CTONG2016775, CTONG2016797, CTONG2016824, CTONG2016846, CTONG2016904, CTONG2016942, CTONG2016953, CTONG2017021, CTONG2017292, CTONG2017429, CTONG2017444, CTONG2017458, CTONG2017604, CTONG2017650, CTONG2017798, CTONG2017804, CTONG2017814, CTONG2017910, CTONG2017939, CTONG2017989, CTONG2018062, CTONG2018069, CTONG2018147, CTONG2018217, CTONG2018279, CTONG2018334, CTONG2018343, CTONG2018379, CTONG2018483, CTONG2018629, CTONG2018632, CTONG2018652, CTONG2018653, CTONG2018655, CTONG2018735, CTONG2018808, CTONG2018843, CTONG2018900, CTONG2018976, CTONG2019144, CTONG2019232, CTONG2019248, CTONG2019400, CTONG2019652, CTONG2019704, CTONG2019822, CTONG2019833, CTONG2020031, CTONG2020108, CTONG2020127, CTONG2020171, CTONG2020411, CTONG2020522, CTONG2020638, CTONG2020806, CTONG2020974, CTONG2021024, CTONG2021132, CTONG2021168, CTONG2021289, CTONG2022153, CTONG2022601, CTONG2022835, CTONG2023449, CTONG2023512, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027327, CTONG2027361, CTONG2027591, CTONG2027736, CTONG2027783, CTONG2027828, CTONG2027959, CTONG2028097, CTONG2028518, CTONG2028687, CTONG2028758, CTONG3000686, CTONG3000707, CTONG3000896, CTONG3001420, CTONG3001501, CTONG3001560, CTONG3001605, CTONG3002020, CTONG3002127, CTONG3002370, CTONG3002433, CTONG3002518, CTONG3002552, CTONG3002588, CTONG3002674, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004317, CTONG3004397, CTONG3004550, CTONG3004576, CTONG3004607, CTONG3004712, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009287, CTONG3009385, D3OST1000066, D3OST1000109, D3OST1000238, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D60ST2000127, D6OST2000169, D6OST2000245, D6OST2000507, D9OST2000278, D9OST2000440, D9OST2000869, D9OST2001319, D9OST2001433, D9OST2002397, D9OST2002673, D9OST2003108, D9OST2003137, D9OST2003397, D9OST2003580, D9OST2003594, D9OST2003791, D9OST2003989, D9OST2004018, D9OST2004073, D9OST2004417, DFNES1000003, DFNES1000107, DFNES1000185, DFNES2000143, DFNES2000146, DFNES2000212, DFNES2000426, DFNES2000432, DFNES2000471, DFNES2000913, DFNES2001108, DFNES2001177, DFNES2001404, DFNES2001564, DFNES2001565, DFNES2001647, DFNES2001800, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004383, DFNES2004608, DFNES2004684, DFNES2005266, DFNES2005527, DFNES2005540, DFNES2005766, DFNES2005779, DFNES2005875, DFNES2006346, DFNES2006944, DFNES2007299, DFNES2007332, DFNES2007641, DFNES2007757, DFNES2008280, DFNES2008881, DFNES2010502, DFNES2011192, DFNES2011221, DFNES2011239, ERLTF1000021, ERLTF2000324, ERLTF2000465, FCBBF1000023, FCBBF1000038, FCBBF1000053, FCBBF1000069, FCBBF1000115, FCBBF1000117, FCBBF1000125, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000280, FCBBF1000297, FCBBF1000305, FCBBF1000314, FCBBF1000322, FCBBF1000349, FCBBF1000374, FCBBF1000377, FCBBF1000395, FCBBF1000397, FCBBF1000425, FCBBF1000437, FCBBF1000506, FCBBF1000546, FCBBF1000550, FCBBF1000581, FCBBF1000582, FCBBF1000598, FCBBF1000604, FCBBF1000618, FCBBF1000627, FCBBF1000675, FCBBF1000686, FCBBF1000687, FCBBF1000691, FCBBF1000732, FCBBF2000094, FCBBF2000105, FCBBF2000123, FCBBF2000195, FCBBF2000214, FCBBF2000232, FCBBF2000259, FCBBF2000276, FCBBF2000291, FCBBF2000379, FCBBF2000473, FCBBF2000502, FCBBF2000576, FCBBF2000677, FCBBF2000678, FCBBF2000782, FCBBF2000808, FCBBF2000815, FCBBF2000825, FCBBF2000951, FCBBF2001088, FCBBF2001116, FCBBF2001188, FCBBF2001238, FCBBF2001243, FCBBF2001299, FCBBF2001309, FCBBF2001427, FCBBF2001529, FCBBF2001672, FCBBF2001675, FCBBF2001718, FCBBF2001720, FCBBF2001868, FCBBF2002044, FCBBF2002111, FCBBF2002281, FCBBF2002370, FCBBF2002541, FCBBF2002626, FCBBF2002897, FCBBF2002898, FCBBF2002904, FCBBF2002953, FCBBF2003083, FCBBF2003102, FCBBF2003269, FCBBF2003297, FCBBF2003395, FCBBF2003528, FCBBF2003549, FCBBF2003636, FCBBF2003823, FCBBF2003895, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005966, FCBBF2006131, FCBBF2006380, FCBBF2006418, FCBBF2006452, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000120, FCBBF3000175, FCBBF3000227, FCBBF3000233, FCBBF3000269, FCBBF3000361, FCBBF3000367, FCBBF3000462, FCBBF3000473, FCBBF3000501, FCBBF3000518, FCBBF3000550, FCBBF3000847, FCBBF3001018, FCBBF3001020, FCBBF3001235, FCBBF3001281, FCBBF3001302, FCBBF3001594, FCBBF3001818, FCBBF3001855, FCBBF3001912, FCBBF3001914, FCBBF3001918, FCBBF3001924, FCBBF3002163, FCBBF3002188, FCBBF3002190, FCBBF3002268, FCBBF3002475, FCBBF3002556, FCBBF3002592, FCBBF3002658, FCBBF3002782, FCBBF3002925, FCBBF3003216, FCBBF3003305, FCBBF3003373, FCBBF3003435, FCBBF3003475, FCBBF3003481, FCBBF3003902, FCBBF3004021, FCBBF3004041, FCBBF3004261, FCBBF3004390, FCBBF3004409, FCBBF3004502, FCBBF3004842, FCBBF3004847, FCBBF3005160, FCBBF3005218, FCBBF3005444, FCBBF3005497, FCBBF3005698, FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007244, FCBBF3007248, FCBBF3007453, FCBBF3007540, FCBBF3007597, FCBBF3007604, FCBBF3007829, FCBBF3007855, FCBBF3008073, FCBBF3008100, FCBBF3008251, FCBBF3008311, FCBBF3008362, FCBBF3008382, FCBBF3008475, FCBBF3008556, FCBBF3008689, FCBBF3008748, FCBBF3008870, FCBBF3008938, FCBBF3008944, FCBBF3009256, FCBBF3009317, FCBBF3009428, FCBBF3009464, FCBBF3009703, FCBBF3009717, FCBBF3009888, FCBBF3009949, FCBBF3010008, FCBBF3010124, FCBBF3010130, FCBBF3010149, FCBBF3010211, FCBBF3010361, FCBBF3010508, FCBBF3010544, FCBBF3010586, FCBBF3010601, FCBBF3010665, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012443, FCBBF3012588, FCBBF3012667, FCBBF3012842, FCBBF3012901, FCBBF3013041, FCBBF3013058, FCBBF3013205, FCBBF3013307, FCBBF3013341, FCBBF3013589, FCBBF3013623, FCBBF3013846, FCBBF3014229, FCBBF3014355, FCBBF3014567, FCBBF3014770, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016644, FCBBF3016987, FCBBF3017024, FCBBF3017059, FCBBF3017071, FCBBF3017123, FCBBF3017233, FCBBF3017288, FCBBF3017535, FCBBF3017681, FCBBF3017729, FCBBF3017873, FCBBF3017974, FCBBF3018453, FCBBF3018591, FCBBF3018753, FCBBF3018826, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021191, FCBBF3021221, FCBBF3021501, FCBBF3021506, FCBBF3021524, FCBBF3021940, FCBBF3021985, FCBBF3022005, FCBBF3022291, FCBBF3022311, FCBBF3022504, FCBBF3022765, FCBBF3022894, FCBBF3023061, FCBBF3023078, FCBBF3023372, FCBBF3023443, FCBBF3023599, FCBBF3023704, FCBBF3023887, FCBBF3023895, FCBBF3024096, FCBBF3024225, FCBBF3024364, FCBBF3024793, FCBBF3024911, FCBBF3025098, FCBBF3025252, FCBBF3025280, FCBBF3025674, FCBBF3025730, FCBBF3025905, FCBBF3026021, FCBBF3026048, FCBBF3026251, FCBBF3026678, FCBBF3027104, FCBBF3027199, FCBBF3027328, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028472, FCBBF3028528, FCBBF3028582, FCBBF3028671, FCBBF3028794, FCBBF4000017, FCBBF4000061, FCBBF4000076, FCBBF4000173, FCBBF4000192, FCBBF4000193, FCBBF4000282, FCBBF4000399, FCBBF4000415, FCBBF4000500, FCBBF4000529, FCBBF4000548, FCBBF5000041, FCBBF5000353, FCBBF5000384, FCBBF5000483, FCBBF5000495, FEBRA1000005, FEBRA1000008, FEBRA1000030, FEBRA1000138, FEBRA1000148, FEBRA1000149, FEBRA1000183, FEBRA2000021, FEBRA2000035, FEBRA2000126, FEBRA2000220, FEBRA2000228, FEBRA2000282, FEBRA2000286, FEBRA2000311, FEBRA2000321, FEBRA2000377, FEBRA2000378, FEBRA2000391, FEBRA2000394, FEBRA2000397, FEBRA2000399, FEBRA2000404, FEBRA2000409, FEBRA2000415, FEBRA2000436, FEBRA2000438, FEBRA2000454, FEBRA2000468, FEBRA2000491, FEBRA2000504, FEBRA2000510, FEBRA2000532, FEBRA2000575, FEBRA2000656, FEBRA2000659, FEBRA2000690, FEBRA2000727, FEBRA2000734, FEBRA2000782, FEBRA2000790, FEBRA2000803, FEBRA2000805, FEBRA2000809, FEBRA2000856, FEBRA2000862, FEBRA2000881, FEBRA2000909, FEBRA2000917, FEBRA2000928, FEBRA2000936, FEBRA2000937, FEBRA2000959, FEBRA2000972, FEBRA2001012, FEBRA2001020, FEBRA2001093, FEBRA2001124, FEBRA2001133, FEBRA2001161, FEBRA2001175, FEBRA2001217, FEBRA2001267, FEBRA2001351, FEBRA2001383, FEBRA2001438, FEBRA2001487, FEBRA2001523, FEBRA2001561, FEBRA2001584, FEBRA2001590, FEBRA2001610, FEBRA2001620, FEBRA2001659, FEBRA2001705, FEBRA2001752, FEBRA2001772, FEBRA2001790, FEBRA2001828, FEBRA2001914, FEBRA2001989, FEBRA2002009, FEBRA2002086, FEBRA2002109, FEBRA2002191, FEBRA2002260, FEBRA2002399, FEBRA2002410, FEBRA2002429, FEBRA2002508, FEBRA2002525, FEBRA2002527, FEBRA2002552, FEBRA2002574, FEBRA2002601, FEBRA2002628, FEBRA2002662, FEBRA2002682, FEBRA2002727, FEBRA2002783, FEBRA2002818, FEBRA2002858, FEBRA2002882, FEBRA2002897, FEBRA2002908, FEBRA2002962, FEBRA2002986, FEBRA2003054, FEBRA2003155, FEBRA2003163, FEBRA2003181, FEBRA2003198, FEBRA2003253, FEBRA2003275, FEBRA2003308, FEBRA2003327, FEBRA2003429, FEBRA2003454, FEBRA2003468, FEBRA2003520, FEBRA2003524, FEBRA2003541, FEBRA2003675, FEBRA2003711, FEBRA2003822, FEBRA2003897, FEBRA2003907, FEBRA2004023, FEBRA2004026, FEBRA2004029, FEBRA2004053, FEBRA2004056, FEBRA2004110, FEBRA2004191, FEBRA2004202, FEBRA2004237, FEBRA2004268, FEBRA2004329, FEBRA2004331, FEBRA2004412, FEBRA2004443, FEBRA2004538, FEBRA2004620, FEBRA2004651, FEBRA2004767, FEBRA2004813, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005377, FEBRA2005380, FEBRA2005458, FEBRA2005701, FEBRA2005726, FEBRA2005734, FEBRA2005778, FEBRA2005787, FEBRA2005998, FEBRA2006061, FEBRA2006112, FEBRA2006150, FEBRA2006165, FEBRA2006315, FEBRA2006346, FEBRA2006354, FEBRA2006379, FEBRA2006396, FEBRA2006409, FEBRA2006418, FEBRA2006427, FEBRA2006476, FEBRA2006491, FEBRA2006664, FEBRA2006667, FEBRA2006726, FEBRA2006736, FEBRA2006793, FEBRA2006873, FEBRA2006942, FEBRA2006977, FEBRA2007017, FEBRA2007200, FEBRA2007247, FEBRA2007280, FEBRA2007414, FEBRA2007436, FEBRA2007449, FEBRA2007458, FEBRA2007566, FEBRA2007620, FEBRA2007708, FEBRA2007714, FEBRA2007793, FEBRA2007931, FEBRA2008009, FEBRA2008134, FEBRA2008135, FEBRA2008159, FEBRA2008165, FEBRA2008172, FEBRA2008265, FEBRA2008282, FEBRA2008311, FEBRA2008341, FEBRA2008459, FEBRA2008468, FEBRA2008475, FEBRA2008481, FEBRA2008542, FEBRA2008660, FEBRA2008681, FEBRA2008692, FEBRA2008741, FEBRA2008836, FEBRA2008859, FEBRA2008866, FEBRA2008881, FEBRA2008928, FEBRA2008983, FEBRA2009016, FEBRA2009029, FEBRA2009074, FEBRA2009117, FEBRA2009120, FEBRA2009162, FEBRA2009240, FEBRA2009265, FEBRA2009276, FEBRA2009327, FEBRA2009385, FEBRA2009416, FEBRA2009437, FEBRA2009478, FEBRA2009507, FEBRA2009541, FEBRA2009567, FEBRA2009731, FEBRA2009754, FEBRA2009780, FEBRA2010141, FEBRA2010299, FEBRA2010484, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013905, FEBRA2014010, FEBRA2014122, FEBRA2014198, FEBRA2014213, FEBRA2014443, FEBRA2014578, FEBRA2014939, FEBRA2015076, FEBRA2015175, FEBRA2015292, FEBRA2015588, FEBRA2016112, FEBRA2016621, FEBRA2016654, FEBRA2016739, FEBRA2017098, FEBRA2017138, FEBRA2017154, FEBRA2017200, FEBRA2017223, FEBRA2017333, FEBRA2017502, FEBRA2017736, FEBRA2017780, FEBRA2017811, FEBRA2017850, FEBRA2018051, FEBRA2018203, FEBRA2018407, FEBRA2018433, FEBRA2018476, FEBRA2018746, FEBRA2019172, FEBRA2019298, FEBRA2019389, FEBRA2019457, FEBRA2019519, FEBRA2019582, FEBRA2019637, FEBRA2019663, FEBRA2019690, FEBRA2020406, FEBRA2020566, FEBRA2020582, FEBRA2020668, FEBRA2020801, FEBRA2021032, FEBRA2021134, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022148, FEBRA2022204, FEBRA2022255, FEBRA2022322, FEBRA2022601, FEBRA2022729, FEBRA2022787, FEBRA2022911, FEBRA2022956, FEBRA2022963, FEBRA2022972, FEBRA2023285, FEBRA2023351, FEBRA2023399, FEBRA2023498, FEBRA2023550, FEBRA2023721, FEBRA2023989, FEBRA2023990, FEBRA2024150, FEBRA2024343, FEBRA2024693, FEBRA2024727, FEBRA2024744, FEBRA2024797, FEBRA2024987, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEHRT2000325, FEHRT2000364, FEKID1000018, FELIV1000101, FELIV1000137, FELIV1000164, FELNG2000241, HCASM1000021, HCASM1000050, HCASM1000061, HCASM1000130, HCASM2000156, HCASM2000202, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003099, HCASM2003212, HCASM2003237, HCASM2003357, HCASM2003415, HCASM2006131, HCASM2006338, HCASM2006359, HCASM2006632, HCASM2007317, HCASM2007737, HCASM2007773, HCASM2008154, HCASM2008536, HCASM2009405, HCASM2009424, HCASM2009463, HCASM2009588, HCHON1000142, HCHON1000176, HCHON2000038, HCHON2000100, HCHON2000156, HCHON2000199, HCHON2000265, HCHON2000271, HCHON2000315, HCHON2000323, HCHON2000344, HCHON2000373, HCHON2000395, HCHON2000417, HCHON2000418, HCHON2000503, HCHON2000508, HCHON2000660, HCHON2000673, HCHON2000676, HCHON2000699, HCHON2000705, HCHON2000817, HCHON2000818, HCHON2000819, HCHON2000832, HCHON2000851, HCHON2000898, HCHON2000935, HCHON2000942, HCHON2000956, HCHON2000994, HCHON2001050, HCHON2001110, HCHON2001116, HCHON2001161, HCHON2001214, HCHON2001233, HCHON2001269, HCHON2001450, HCHON2001453, HCHON2001497, HCHON2001505, HCHON2001519, HCHON2001523, HCHON2001546, HCHON2001548, HCHON2001577, HCHON2001598, HCHON2001604, HCHON2001607, HCHON2001646, HCHON2001712, HCHON2001768, HCHON2002123, HCHON2002194, HCHON2002241, HCHON2002354, HCHON2002496, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987, HCHON2006459, HCHON2006714, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HEART1000010, HEART1000088, HEART1000099, HEART1000102, HEART1000132, HEART1000139, HEART1000151, HEART1000173, HEART2000024, HEART2000035, HEART2000099, HEART2000298, HEART2000306, HEART2000309, HEART2000411, HEART2000418, HEART2000448, HEART2000492, HEART2000506, HEART2000520, HEART2000568, HEART2000611, HEART2001680, HEART2001756, HEART2002129, HEART2002184, HEART2002220, HEART2002432, HEART2002531, HEART2002598, HEART2002717, HEART2003027, HEART2003432, HEART2003440, HEART2003781, HEART2003836, HEART2004570, HEART2004764, HEART2004931, HEART2004940, HEART2004941, HEART2004980, HEART2005244, HEART2005310, HEART2005581, HEART2005610, HEART2006131, HEART2006310, HEART2006334, HEART2006487, HEART2006557, HEART2006787, HEART2006789, HEART2006909, HEART2007443, HEART2007767, HEART2008108, HEART2008111, HEART2008257, HEART2008994, HEART2009599, HEART2009680, HEART2010391, HEART2010492, HEART2010495, HELAC2000158, HELAC2000301, HHDPC1000001, HHDPC2000070, HHDPC2000102, HHDPC2000315, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2006460, HHDPC2006862, HHDPC2006927, HHDPC2007302, HHDPC2008123, HHDPC2008185, HHDPC2008279, HHDPC2008297, HHDPC2008414, HHDPC2008474, HHDPC2008843, HHDPC2009114, HHDPC2009178, HHDPC2009208, HHDPC2009272, HLUNG1000017, HLUNG1000024, HLUNG1000030, HLUNG1000034, HLUNG1000037, HLUNG1000053, HLUNG1000064, HLUNG1000076, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000068, HLUNG2000116, HLUNG2000125, HLUNG2000130, HLUNG2000142, HLUNG2000174, HLUNG2000176, HLUNG2000217, HLUNG2000255, HLUNG2000281, HLUNG2000340, HLUNG2000362, HLUNG2000363, HLUNG2000431, HLUNG2000480, HLUNG2000489, HLUNG2000549, HLUNG2000557, HLUNG2000570, HLUNG2000761, HLUNG2000777, HLUNG2000870, HLUNG2000926, HLUNG2000950, HLUNG2000955, HLUNG2000970, HLUNG2001013, HLUNG2001126, HLUNG2001144, HLUNG2001214, HLUNG2001507, HLUNG2001633, HLUNG2001677, HLUNG2001712, HLUNG2001814, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002562, HLUNG2002659, HLUNG2002707, HLUNG2002942, HLUNG2002949, HLUNG2002958, HLUNG2002982, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004154, HLUNG2004217, HLUNG2004273, HLUNG2004388, HLUNG2004416, HLUNG2004521, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006599, HLUNG2006614, HLUNG2006671, HLUNG2006781, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA2000044, HSYRA2000135, HSYRA2000221, HSYRA2000237, HSYRA2000253, HSYRA2000371, HSYRA2000424, HSYRA2000507, HSYRA2000510, HSYRA2000605, HSYRA2000706, HSYRA2000760, HSYRA2000792, HSYRA2000832, HSYRA2000927, HSYRA2001003, HSYRA2001103, HSYRA2001142, HSYRA2001153, HSYRA2001225, HSYRA2001255, HSYRA2001332, HSYRA2001420, HSYRA2001552, HSYRA2001567, HSYRA2001580, HSYRA2001631, HSYRA2002103, HSYRA2002590, HSYRA2002741, HSYRA2003168, HSYRA2004550, HSYRA2004858, HSYRA2005456, HSYRA2005496, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000161, IMR321000165, IMR321000193, IMR321000207, IMR321000230, IMR322000010, IMR322000107, IMR322000164, IMR322000178, IMR322000374, IMR322000379, IMR322000383, IMR322000418, IMR322000656, IMR322000672, IMR322000742, IMR322000791, IMR322000811, IMR322000859, IMR322000863, IMR322000917, IMR322000919, IMR322000953, IMR322000973, IMR322000984, IMR322001018, IMR322001049, IMR322001167, IMR322001245, IMR322001318, IMR322001322, IMR322001438, IMR322001452, IMR322001491, IMR322001534, IMR322001665, IMR322001670, IMR322001724, IMR322002110, IMR322002470, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322015523, IMR322016146, IMR322017049, IMR322018117, IMR322018192, KIDNE1000008, KIDNE1000012, KIDNE1000015, KIDNE1000028, KIDNE1000036, KIDNE1000064, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000328, KIDNE2000335, KIDNE2000349, KIDNE2000375, KIDNE2000394, KIDNE2000493, KIDNE2000510, KIDNE2000513, KIDNE2000519, KIDNE2000555, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2001140, KIDNE2001269, KIDNE2001361, KIDNE2001373, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002198, KIDNE2002262, KIDNE2002265, KIDNE2002438, KIDNE2002483, KIDNE2002768, KIDNE2002795, KIDNE2002839, KIDNE2002845, KIDNE2002872, KIDNE2002882, KIDNE2002980, KIDNE2002991, KIDNE2003185, KIDNE2003188, KIDNE2003233, KIDNE2003316, KIDNE2003335, KIDNE2003357, KIDNE2003373, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005603, KIDNE2005629, KIDNE2005781, KIDNE2005798, KIDNE2005854, KIDNE2005908, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006652, KIDNE2006687, KIDNE2006733, KIDNE2006775, KIDNE2006811, KIDNE2006820, KIDNE2006880, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007910, KIDNE2007944, KIDNE2008022, KIDNE2008072, KIDNE2008116, KIDNE2008117, KIDNE2008218, KIDNE2008315, KIDNE2008378, KIDNE2008446, KIDNE2008473, KIDNE2008480, KIDNE2008649, KIDNE2008697, KIDNE2008788, KIDNE2008795, KIDNE2008824, KIDNE2008832, KIDNE2008869, KIDNE2008975, KIDNE2009019, KIDNE2009109, KIDNE2009180, KIDNE2009191, KIDNE2009332, KIDNE2009426, KIDNE2009467, KIDNE2009490, KIDNE2009553, KIDNE2009628, KIDNE2009647, KIDNE2009676, KIDNE2009723, KIDNE2010007, KIDNE2010052, KIDNE2010084, KIDNE2010137, KIDNE2010151, KIDNE2010264, KIDNE2010271, KIDNE2010419, KIDNE2010574, KIDNE2010674, KIDNE2010739, KIDNE2010762, KIDNE2010863, KIDNE2010973, KIDNE2010989, KIDNE2011200, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018617, KIDNE2018678, KIDNE2018727, KIDNE2018863, KIDNE2018891, KIDNE2018989, KIDNE2018996, KIDNE2019074, KIDNE2019187, LIVER1000050, LIVER1000055, LIVER1000058, LIVER1000063, LIVER1000067, LIVER1000073, LIVER1000082, LIVER1000099, LIVER1000111, LIVER1000139, LIVER1000155, LIVER1000165, LIVER1000175, LIVER1000224, LIVER1000278, LIVER1000303, LIVER1000397, LIVER1000421, LIVER1000433, LIVER1000479, LIVER1000482, LIVER1000542, LIVER2000003, LIVER2000037, LIVER2000158, LIVER2000216, LIVER2000237, LIVER2000247, LIVER2000360, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2002644, LIVER2002660, LIVER2002702, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005198, LIVER2005218, LIVER2005376, LIVER2005385, LIVER2005512, LIVER2005520, LIVER2005527, LIVER2005544, LIVER2005625, LIVER2005728, LIVER2005981, LIVER2006006, LIVER2006251, LIVER2006469, LIVER2006644, LIVER2006764, LIVER2007415, LIVER2007548, LIVER2007568, LIVER2007721, LIVER2007783, LIVER2008053, LIVER2008473, LIVER2008580, LIVER2008706, LIVER2008945, LIVER2008998, LIVER2009110, LIVER2009118, LIVER2009554, LYMPB1000141, LYMPB1000158, LYMPB2000083, MAMGL1000096, MAMGL1000132, MAMGL1000151, MAMGL1000173, MAMGL1000178, MAMGL1000182, MAMGL1000184, MESAN1000035, MESAN1000121, MESAN1000147, MESAN1000180, MESAN2000023, MESAN2000026, MESAN2000067, MESAN2000167, MESAN2000291, MESAN2000337, MESAN2000434, MESAN2000457, MESAN2000462, MESAN2000620, MESAN2000711, MESAN2000815, MESAN2000894, MESAN2000909, MESAN2001450, MESAN2001770, MESAN2001979, MESAN2001980, MESAN2002113, MESAN2002147, MESAN2002424, MESAN2002687, MESAN2002709, MESAN2002790, MESAN2002978, MESAN2003035, MESAN2003037, MESAN2003039, MESAN2003058, MESAN2003101, MESAN2003322, MESAN2003490, MESAN2003529, MESAN2003662, MESAN2003851, MESAN2004138, MESAN2004288, MESAN2004575, MESAN2005303, MESAN2005371, MESAN2005633, MESAN2005724, MESAN2005808, MESAN2005811, MESAN2006022, MESAN2006328, MESAN2006580, MESAN2006599, MESAN2006971, MESAN2007032, MESAN2007440, MESAN2007597, MESAN2008222, MESAN2008334, MESAN2008415, MESAN2008460, MESAN2008536, MESAN2008679, MESAN2008729, MESAN2008821, MESAN2009019, MESAN2009105, MESAN2009311, MESAN2009418, MESAN2009522, MESAN2010031, MESAN2010114, MESAN2010312, MESAN2010321, MESAN2010681, MESAN2011545, MESAN2011597, MESAN2011627, MESAN2011977, MESAN2012586, MESAN2012708, MESAN2012735, MESAN2013019, MESAN2013022, MESAN2013183, MESAN2013211, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016552, MESAN2016965, MESAN2017152, MESAN2017373, MESAN2017417, MESAN2018209, MESAN2018576, MESAN2018670, MESAN2018699, MESTC1000147, MESTC2000150, MESTC2000153, MESTC2000170, N1ESE2000698, NB9N41000121, NB9N41000135, NB9N41000146, NB9N42000104, NB9N42000122, NB9N42000196, NB9N42000314, NB9N42000495, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000187, NHNPC2000212, NHNPC2000606, NHNPC2000877, NHNPC2001108, NHNPC2001223, NHNPC2001312, NHNPC2001816, NHNPC2002565, NHNPC2002749, NOVAR1000091, NOVAR1000102, NOVAR2000136, NOVAR2000352, NOVAR2000412, NOVAR2000710, NOVAR2000783, NOVAR2000962, NOVAR2001108, NOVAR2001620, NOVAR2001876, NOVAR2002039, NT2NE1000014, NT2NE1000023, NT2NE1000024, NT2NE1000059, NT2NE1000063, NT2NE1000083, NT2NE1000120, NT2NE1000122, NT2NE1000175, NT2NE1000185, NT2NE2000038, NT2NE2000054, NT2NE2000057, NT2NE2000064, NT2NE2000109, NT2NE2000137, NT2NE2000156, NT2NE2000222, NT2NE2000259, NT2NE2000284, NT2NE2000299, NT2NE2000353, NT2NE2000356, NT2NE2000369, NT2NE2000374, NT2NE2000390, NT2NE2000455, NT2NE2000470, NT2NE2000488, NT2NE2000517, NT2NE2000536, NT2NE2000546, NT2NE2000550, NT2NE2000586, NT2NE2000612, NT2NE2000658, NT2NE2000706, NT2NE2000802, NT2NE2000808, NT2NE2000864, NT2NE2000913, NT2NE2000950, NT2NE2000951, NT2NE2001005, NT2NE2001021, NT2NE2001142, NT2NE2001181, NT2NE2001188, NT2NE2001191, NT2NE2001247, NT2NE2001252, NT2NE2001324, NT2NE2001326, NT2NE2001372, NT2NE2001428, NT2NE2001432, NT2NE2001524, NT2NE2001530, NT2NE2001617, NT2NE2001623, NT2NE2001626, NT2NE2001634, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003252, NT2NE2003308, NT2NE2003309, NT2NE2003315, NT2NE2003393, NT2NE2003408, NT2NE2003457, NT2NE2003540, NT2NE2003571, NT2NE2003705, NT2NE2003752, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005441, NT2NE2005517, NT2NE2005537, NT2NE2005555, NT2NE2005676, NT2NE2005720, NT2NE2005784, NT2NE2005804, NT2NE2005821, NT2NE2005890, NT2NE2005921, NT2NE2005968, NT2NE2006087, NT2NE2006103, NT2NE2006217, NT2NE2006288, NT2NE2006382, NT2NE2006387, NT2NE2006427, NT2NE2006432, NT2NE2006458, NT2NE2006531, NT2NE2006659, NT2NE2006784, NT2NE2006813, NT2NE2006894, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009426, NT2NE2009608, NT2NE2009691, NT2NE2010105, NT2NE2010150, NT2NE2010400, NT2NE2010632, NT2NE2010633, NT2NE2010654, NT2NE2010781, NT2NE2010854, NT2NE2011033, NT2NE2011036, NT2NE2011107, NT2NE2011154, NT2NE2011411, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2016041, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018739, NT2NE2018916, NT2NE2019056, NT2NE2019076, NT2NE2019099, NT2RI1000027, NT2RI1000035, NT2RI1000164, NT2RI1000172, NT2RI2000004, NT2RI2000007, NT2RI2000117, NT2RI2000128, NT2RI2000133, NT2RI2000173, NT2RI2000187, NT2RI2000270, NT2RI2000276, NT2RI2000282, NT2RI2000313, NT2RI2000341, NT2RI2000348, NT2RI2000412, NT2RI2000421, NT2RI2000480, NT2RI2000575, NT2RI2000588, NT2RI2000597, NT2RI2000669, NT2RI2000688, NT2RI2000689, NT2RI2000704, NT2RI2000738, NT2RI2000822, NT2RI2000841, NT2RI2000865, NT2RI2000932, NT2RI2000987, NT2RI2001010, NT2RI2001017, NT2RI2001030, NT2RI2001083, NT2RI2001091, NT2RI2001167, NT2RI2001178, NT2RI2001235, NT2RI2001244, NT2RI2001307, NT2RI2001342, NT2RI2001385, NT2RI2001409, NT2RI2001410, NT2RI2001425, NT2RI2001449, NT2RI2001474, NT2RI2001519, NT2RI2001595, NT2RI2001624, NT2RI2001769, NT2RI2001782, NT2RI2001836, NT2RI2001866, NT2RI2002022, NT2RI2002041, NT2RI2002117, NT2RI2002120, NT2RI2002153, NT2RI2002179, NT2RI2002252, NT2RI2002260, NT2RI2002270, NT2RI2002334, NT2RI2002447, NT2RI2002481, NT2RI2002507, NT2RI2002517, NT2RI2002554, NT2RI2002564, NT2RI2002585, NT2RI2002729, NT2RI2002802, NT2RI2002852, NT2RI2002865, NT2RI2002923, NT2RI2002970, NT2RI2003019, NT2RI2003067, NT2RI2003184, NT2RI2003360, NT2RI2003383, NT2RI2003392, NT2RI2003399, NT2RI2003407, NT2RI2003419, NT2RI2003487, NT2RI2003667, NT2RI2003678, NT2RI2003884, NT2RI2003973, NT2RI2003993, NT2RI2004059, NT2RI2004079, NT2RI2004093, NT2RI2004136, NT2RI2004157, NT2RI2004190, NT2RI2004209, NT2RI2004284, NT2RI2004290, NT2RI2004312, NT2RI2004381, NT2RI2004442, NT2RI2004468, NT2RI2004618, NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005130, NT2RI2005166, NT2RI2005246, NT2RI2005315, NT2RI2005358, NT2RI2005473, NT2RI2005520, NT2RI2005621, NT2RI2005628, NT2RI2005647, NT2RI2005670, NT2RI2005710, NT2RI2005772, NT2RI2005966, NT2RI2006070, NT2RI2006081, NT2RI2006126, NT2RI2006127, NT2RI2006183, NT2RI2006257, NT2RI2006294, NT2RI2006345, NT2RI2006403, NT2RI2006445, NT2RI2006487, NT2RI2006506, NT2RI2006565, NT2RI2006659, NT2RI2006662, NT2RI2006667, NT2RI2006679, NT2RI2006682, NT2RI2006686, NT2RI2006716, NT2RI2006762, NT2RI2006788, NT2RI2006838, NT2RI2006853, NT2RI2006855, NT2RI2006973, NT2RI2007046, NT2RI2007048, NT2RI2007116, NT2RI2007214, NT2RI2007303, NT2RI2007334, NT2RI2007386, NT2RI2007406, NT2RI2007439, NT2RI2007469, NT2RI2007507, NT2RI2007629, NT2RI2007723, NT2RI2007729, NT2RI2007751, NT2RI2007754, NT2RI2007827, NT2RI2007853, NT2RI2007877, NT2RI2007879, NT2RI2007891, NT2RI2007956, NT2RI2007965, NT2RI2008007, NT2RI2008050, NT2RI2008188, NT2RI2008204, NT2RI2008221, NT2RI2008233, NT2RI2008455, NT2RI2008502, NT2RI2008598, NT2RI2008714, NT2RI2008801, NT2RI2008808, NT2RI2008841, NT2RI2008952, NT2RI2009005, NT2RI2009083, NT2RI2009101, NT2RI2009103, NT2RI2009144, NT2RI2009151, NT2RI2009215, NT2RI2009259, NT2RI2009289, NT2RI2009301, NT2RI2009517, NT2RI2009585, NT2RI2009595, NT2RI2009855, NT2RI2010283, NT2RI2010508, NT2RI2011422, NT2RI2011683, NT2RI2011803, NT2RI2012350, NT2RI2012990, NT2RI2013357, NT2RI2013373, NT2RI2014247, NT2RI2014551, NT2RI2014683, NT2RI2015239, NT2RI2016128, NT2RI2016134, NT2RI2017529, NT2RI2018363, NT2RI2018448, NT2RI2018883, NT2RI2018950, NT2RI2019751, NT2RI2019826, NT2RI2020390, NT2RI2020703, NT2RI2021463, NT2RI2022468, NT2RI2022584, NT2RI2023303, NT2RI2023671, NT2RI2024008, NT2RI2024460, NT2RI2024496, NT2RI2024935, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001458, NT2RI3001573, NT2RI3001967, NT2RI3002303, NT2RI3002557, NT2RI3003027, NT2RI3003031, NT2RI3003095, NT2RI3003104, NT2RI3003162, NT2RI3003362, NT2RI3003382, NT2RI3003409, NT2RI3004133, NT2RI3004381, NT2RI3005202, NT2RI3005416, NT2RI3005861, NT2RI3005928, NT2RI3006132, NT2RI3006171, NT2RI3006340, NT2RI3006376, NT2RI3006666, NT2RI3006796, NT2RI3007065, NT2RI3007095, NT2RI3007158, NT2RI3007167, NT2RI3007213, NT2RI3007443, NT2RI3007684, NT2RI3007757, NT2RI3007878, NT2RI3007978, NT2RI3008162, NT2RI3008179, NT2RI3008228, NT2RI3008442, NT2RI3008652, NT2RI3008697, NT2RI3008974, NT2RI3009158, NT2RI3009480, NT2RP6000005, NT2RP6000008, NT2RP6000032, NT2RP6000033, NT2RP6000059, NT2RP6000060, NT2RP6000086, NT2RP6000100, NT2RP6000119, NT2RP6000127, NT2RP6000130, NT2RP6000140, NT2RP6000150, NT2RP7000041, NT2RP7000086, NT2RP7000109, NT2RP7000112, NT2RP7000311, NT2RP7000359, NT2RP7000364, NT2RP7000380, NT2RP7000391, NT2RP7000425, NT2RP7000579, NT2RP7000812, NT2RP7000926, NT2RP7001074, NT2RP7001156, NT2RP7001319, NT2RP7001364, NT2RP7001475, NT2RP7001532, NT2RP7001591, NT2RP7001602, NT2RP7001678, NT2RP7001856, NT2RP7002028, NT2RP7002151, NT2RP7002163, NT2RP7002243, NT2RP7002282, NT2RP7002554, NT2RP7002603, NT2RP7002619, NT2RP7002650, NT2RP7002779, NT2RP7002829, NT2RP7002841, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003261, NT2RP7003439, NT2RP7003511, NT2RP7003724, NT2RP7004027, NT2RP7004080, NT2RP7004114, NT2RP7004260, NT2RP7004358, NT2RP7004396, NT2RP7004541, NT2RP7004641, NT2RP7004656, NT2RP7004687, NT2RP7004722, NT2RP7004766, NT2RP7004961, NT2RP7004964, NT2RP7004975, NT2RP7005118, NT2RP7005205, NT2RP7005219, NT2RP7005323, NT2RP7005468, NT2RP7005493, NT2RP7005629, NT2RP7005631, NT2RP7005669, NT2RP7005750, NT2RP7005846, NT2RP7006160, NT2RP7006162, NT2RP7006296, NT2RP7006304, NT2RP7006374, NT2RP7006408, NT2RP7006457, NT2RP7006547, NT2RP7006601, NT2RP7006621, NT2RP7006636, NT2RP7006853, NT2RP7006887, NT2RP7006980, NT2RP7006995, NT2RP7007114, NT2RP7007221, NT2RP7007226, NT2RP7007269, NT2RP7007310, NT2RP7007359, NT2RP7007406, NT2RP7007480, NT2RP7007504, NT2RP7007580, NT2RP7007594, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008360, NT2RP7008406, NT2RP7008435, NT2RP7008441, NT2RP7008454, NT2RP7008544, NT2RP7008550, NT2RP7008557, NT2RP7008623, NT2RP7008657, NT2RP7008720, NT2RP7008855, NT2RP7008863, NT2RP7009019, NT2RP7009030, NT2RP7009097, NT2RP7009168, NT2RP7009215, NT2RP7009236, NT2RP7009253, NT2RP7009267, NT2RP7009322, NT2RP7009363, NT2RP7009370, NT2RP7009397, NT2RP7009429, NT2RP7009439, NT2RP7009466, NT2RP7009502, NT2RP7009577, NT2RP7009743, NT2RP7009867, NT2RP7010109, NT2RP7010128, NT2RP7010235, NT2RP7010275, NT2RP7010521, NT2RP7010599, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7013002, NT2RP7013374, NT2RP7013499, NT2RP7013729, NT2RP7013795, NT2RP7013999, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015512, NT2RP7015676, NT2RP7015789, NT2RP7015996, NT2RP7016508, NT2RP7016622, NT2RP7016911, NT2RP7017139, NT2RP7017284, NT2RP7017365, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019273, NT2RP7019367, NT2RP7019445, NT2RP7019682, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8003490, NT2RP8003657, NT2RP8003787, NT2RP8004306, NT2RP8005546, NT2RP8006452, NT2RP8006521, NT2RP8007416, NT2RP8007503, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000213, NTONG1000214, NTONG1000246, NTONG1000247, NTONG1000257, NTONG2000040, NTONG2000231, NTONG2000334, NTONG2000363, NTONG2000413, NTONG2000492, NTONG2000499, NTONG2000531, NTONG2000583, NTONG2000800, NTONG2000858, NTONG2000966, NTONG2000985, NTONG2001014, NTONG2001079, NTONG2001157, NTONG2001173, NTONG2001220, NTONG2001222, NTONG2001362, NTONG2001532, NTONG2001587, NTONG2001619, NTONG2001642, NTONG2001657, NTONG2001676, NTONG2002278, NTONG2002582, NTONG2002948, NTONG2002970, NTONG2003158, NTONG2003210, NTONG2003316, NTONG2003409, NTONG2003805, NTONG2003839, NTONG2003852, NTONG2003897, NTONG2003928, NTONG2004308, NTONG2004521, NTONG2004690, NTONG2004806, NTONG2004829, NTONG2004844, NTONG2004918, NTONG2004991, NTONG2005062, NTONG2005086, NTONG2005137, NTONG2005153, NTONG2005265, NTONG2005277, NTONG2005363, NTONG2005480, NTONG2005497, NTONG2005577, NTONG2005657, NTONG2005822, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007034, NTONG2007249, NTONG2007517, NTONG2007522, NTONG2007693, NTONG2007756, NTONG2008093, NTONG2008365, NTONG2008522, NTONG2008672, NTONG2008862, NTONG2008939, NTONG2009060, NTONG2009068, NTONG2009229, NTONG2009233, NTONG2009468, OCBBF1000016, OCBBF1000042, OCBBF1000054, OCBBF1000067, OCBBF1000085, OCBBF1000091, OCBBF1000104, OCBBF1000118, OCBBF1000119, OCBBF1000122, OCBBF1000130, OCBBF1000138, OCBBF1000153, OCBBF1000175, OCBBF1000192, OCBBF1000254, OCBBF2000013, OCBBF2000287, OCBBF2000323, OCBBF2000452, OCBBF2000467, OCBBF2000677, OCBBF2000703, OCBBF2000719, OCBBF2000998, OCBBF2001075, OCBBF2001101, OCBBF2001124, OCBBF2001176, OCBBF2001186, OCBBF2001196, OCBBF2001252, OCBBF2001323, OCBBF2001389, OCBBF2001402, OCBBF2001473, OCBBF2001494, OCBBF2001527, OCBBF2001528, OCBBF2001681, OCBBF2001706, OCBBF2001749, OCBBF2001910, OCBBF2001961, OCBBF2001983, OCBBF2002124, OCBBF2002161, OCBBF2002357, OCBBF2002376, OCBBF2002615, OCBBF2002663, OCBBF2002805, OCBBF2002865, OCBBF2002920, OCBBF2003028, OCBBF2003091, OCBBF2003327, OCBBF2003543, OCBBF2003744, OCBBF2004038, OCBBF2004147, OCBBF2004168, OCBBF2004235, OCBBF2004385, OCBBF2004418, OCBBF2004478, OCBBF2004482, OCBBF2004567, OCBBF2004612, OCBBF2004647, OCBBF2004826, OCBBF2004866, OCBBF2004883, OCBBF2005066, OCBBF2005077, OCBBF2005343, OCBBF2005349, OCBBF2005373, OCBBF2005433, OCBBF2005476, OCBBF2005843, OCBBF2005901, OCBBF2006030, OCBBF2006058, OCBBF2006113, OCBBF2006154, OCBBF2006214, OCBBF2006241, OCBBF2006388, OCBBF2006438, OCBBF2006567, OCBBF2006624, OCBBF2006639, OCBBF2006849, OCBBF2006859, OCBBF2007039, OCBBF2007114, OCBBF2007121, OCBBF2007184, OCBBF2007194, OCBBF2007196, OCBBF2007238, OCBBF2007414, OCBBF2007428, OCBBF2007478, OCBBF2007485, OCBBF2007520, OCBBF2007555, OCBBF2007610, OCBBF2007622, OCBBF2007756, OCBBF2007931, OCBBF2007946, OCBBF2008116, OCBBF2008138, OCBBF2008283, OCBBF2008330, OCBBF2008366, OCBBF2008511, OCBBF2008520, OCBBF2008569, OCBBF2008586, OCBBF2008640, OCBBF2008691, OCBBF2008724, OCBBF2008760, OCBBF2008768, OCBBF2008770, OCBBF2008775, OCBBF2008790, OCBBF2008814, OCBBF2008822, OCBBF2009115, OCBBF2009391, OCBBF2009424, OCBBF2009571, OCBBF2009583, OCBBF2009603, OCBBF2009788, OCBBF2009926, OCBBF2010040, OCBBF2010281, OCBBF2010313, OCBBF2010404, OCBBF2010416, OCBBF2010420, OCBBF2010557, OCBBF2010604, OCBBF2010709, OCBBF2010792, OCBBF2010843, OCBBF2010863, OCBBF2010931, OCBBF2010945, OCBBF2010978, OCBBF2010989, OCBBF2011137, OCBBF2011177, OCBBF2011311, OCBBF2011625, OCBBF2011669, OCBBF2011685, OCBBF2011706, OCBBF2011722, OCBBF2011855, OCBBF2011872, OCBBF2011960, OCBBF2011981, OCBBF2012028, OCBBF2012139, OCBBF2012191, OCBBF2012320, OCBBF2012436, OCBBF2012525, OCBBF2012714, OCBBF2012755, OCBBF2012812, OCBBF2012821, OCBBF2012881, OCBBF2012990, OCBBF2013049, OCBBF2013091, OCBBF2013127, OCBBF2013208, OCBBF2013285, OCBBF2013319, OCBBF2013324, OCBBF2013507, OCBBF2013721, OCBBF2013883, OCBBF2013926, OCBBF2014052, OCBBF2014386, OCBBF2014707, OCBBF2014745, OCBBF2014828, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015506, OCBBF2015599, OCBBF2015645, OCBBF2015659, OCBBF2015751, OCBBF2015797, OCBBF2015980, OCBBF2016038, OCBBF2016405, OCBBF2016467, OCBBF2016590, OCBBF2016591, OCBBF2016612, OCBBF2016689, OCBBF2016690, OCBBF2016841, OCBBF2016928, OCBBF2017055, OCBBF2017069, OCBBF2017325, OCBBF2017458, OCBBF2017489, OCBBF2017516, OCBBF2017665, OCBBF2017815, OCBBF2018012, OCBBF2018014, OCBBF2018167, OCBBF2018229, OCBBF2018362, OCBBF2018380, OCBBF2018563, OCBBF2018663, OCBBF2018687, OCBBF2018828, OCBBF2018934, OCBBF2019108, OCBBF2019195, OCBBF2019327, OCBBF2019684, OCBBF2019761, OCBBF2019823, OCBBF2020048, OCBBF2020318, OCBBF2020343, OCBBF2020453, OCBBF2020801, OCBBF2020825, OCBBF2020838, OCBBF2021020, OCBBF2021214, OCBBF2021323, OCBBF2021518, OCBBF2021788, OCBBF2022351, OCBBF2022573, OCBBF2022574, OCBBF2023162, OCBBF2023598, OCBBF2023643, OCBBF2024463, OCBBF2024589, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028173, OCBBF2028421, OCBBF2029471, OCBBF2029901, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2032152, OCBBF2032274, OCBBF2032539, OCBBF2032590, OCBBF2032599, OCBBF2032611, OCBBF2032671, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF3000097, OCBBF3000213, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008392, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000147, PEBLM1000174, PEBLM1000180, PEBLM2000112, PEBLM2000126, PEBLM2000170, PEBLM2000180, PEBLM2000213, PEBLM2000248, PEBLM2000270, PEBLM2000321, PEBLM2000333, PEBLM2000338, PEBLM2000479, PEBLM2000502, PEBLM2001312, PEBLM2001465, PEBLM2001488, PEBLM2001803, PEBLM2002432, PEBLM2002594, PEBLM2002749, PEBLM2002887, PEBLM2003426, PEBLM2003765, PEBLM2003935, PEBLM2004015, PEBLM2004216, PEBLM2004290, PEBLM2004497, PEBLM2005282, PEBLM2005615, PEBLM2005632, PEBLM2005697, PEBLM2006031, PEBLM2006036, PEBLM2006049,.PEBLM2006113, PEBLM2006135, PEBLM2006215, PEBLM2006298, PEBLM2006709, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008861, PERIC1000025, PERIC1000147, PERIC2000180, PERIC2000214, PERIC2000386, PERIC2000387, PERIC2000422, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000067, PLACE5000068, PLACE5000070, PLACE5000080, PLACE5000105, PLACE5000115, PLACE5000116, PLACE5000129, PLACE5000139, PLACE5000153, PLACE5000171, PLACE5000245, PLACE5000260, PLACE5000282, PLACE5000372, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000296, PLACE6000348, PLACE6000371, PLACE6000424, PLACE6000426, PLACE6000429, PLACE6000450, PLACE6000523, PLACE6000550, PLACE6000552, PLACE6000555, PLACE6000619, PLACE6000630, PLACE6000953, PLACE6001118, PLACE6001153, PLACE6001262, PLACE6001294, PLACE6001536, PLACE6001557, PLACE6001712, PLACE6001740, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002692, PLACE6002699, PLACE6002710, PLACE6002871, PLACE6002949, PLACE6002960, PLACE6003038, PLACE6003077, PLACE6003094, PLACE6003109, PLACE6003372, PLACE6003393, PLACE6003399, PLACE6003539, PLACE6003621, PLACE6003705, PLACE6003740, PLACE6003771, PLACE6003796, PLACE6003839, PLACE6004101, PLACE6004219, PLACE6004336, PLACE6004368, PLACE6004380, PLACE6004396, PLACE6004454, PLACE6004464, PLACE6004491, PLACE6004663, PLACE6004687, PLACE6004738, PLACE6004931, PLACE6004966, PLACE6004993, PLACE6005007, PLACE6005029, PLACE6005108, PLACE6005234, PLACE6005283, PLACE6005328, PLACE6005351, PLACE6005423, PLACE6005487, PLACE6005535, PLACE6005546, PLACE6005559, PLACE6005579, PLACE6005691, PLACE6005786, PLACE6006042, PLACE6006077, PLACE6006158, PLACE6006217, PLACE6006266, PLACE6006287, PLACE6006394, PLACE6006474, PLACE6006549, PLACE6006566, PLACE6006697, PLACE6006822, PLACE6006871, PLACE6006905, PLACE6006988, PLACE6007050, PLACE6007239, PLACE6007242, PLACE6007380, PLACE6007433, PLACE6007482, PLACE6007687, PLACE6007787, PLACE6007798, PLACE6007807, PLACE6008036, PLACE6008094, PLACE6008126, PLACE6008285, PLACE6008444, PLACE6008453, PLACE6008640, PLACE6008750, PLACE6008768, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009835, PLACE6009853, PLACE6010463, PLACE6010485, PLACE6010568, PLACE6010701, PLACE6010765, PLACE6010848, PLACE6010925, PLACE6010936, PLACE6010991, PLACE6011041, PLACE6011102, PLACE6011211, PLACE6011260, PLACE6011334, PLACE6011627, PLACE6011774, PLACE6011881, PLACE6011907, PLACE6011970, PLACE6011975, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013884, PLACE6013885, PLACE6014043, PLACE6014516, PLACE6014882, PLACE6015211, PLACE6015322, PLACE6015460, PLACE6015513, PLACE6015591, PLACE6015729, PLACE6015939, PLACE6016030, PLACE6016093, PLACE6016105, PLACE6016160, PLACE6016210, PLACE6016254, PLACE6016383, PLACE6017002, PLACE6017431, PLACE6017564, PLACE6017578, PLACE6017626, PLACE6017714, PLACE6017788, PLACE6017791, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006268, PLACE7006275, PLACE7006498, PLACE7006540, PLACE7007379, PLACE7007644, PLACE7007973, PLACE7008136, PLACE7008431, PLACE7008623, PLACE7008766, PLACE7009563, PROST1000033, PROST1000039, PROST1000065, PROST1000083, PROST1000110, PROST1000120, PROST1000123, PROST1000136, PROST1000145, PROST1000152, PROST1000167, PROST1000184, PROST1000215, PROST1000217, PROST1000220, PROST1000226, PROST1000246, PROST1000262, PROST1000272, PROST1000298, PROST1000322, PROST1000334, PROST1000343, PROST1000362, PROST1000377, PROST1000412, PROST1000419, PROST1000451, PROST1000480, PROST1000485, PROST1000493, PROST1000512, PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000176, PROST2000203, PROST2000212, PROST2000241, PROST2000267, PROST2000273, PROST2000274, PROST2000277, PROST2000284, PROST2000325, PROST2000337, PROST2000432, PROST2000452, PROST2000470, PROST2000505, PROST2000567, PROST2000572, PROST2000717, PROST2000761, PROST2000835, PROST2000893, PROST2000961, PROST2001002, PROST2001116, PROST2001138, PROST2001213, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003340, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004332, PROST2004481, PROST2004526, PROST2004570, PROST2004727, PROST2004742, PROST2004744, PROST2004817, PROST2005039, PROST2005093, PROST2005121, PROST2005131, PROST2005143, PROST2005228, PROST2005285, PROST2005426, PROST2005466, PROST2005630, PROST2005683, PROST2005788, PROST2005880, PROST2005886, PROST2005904, PROST2005919, PROST2005943, PROST2006008, PROST2006020, PROST2006060, PROST2006196, PROST2006201, PROST2006282, PROST2006343, PROST2006450, PROST2006491, PROST2006510, PROST2006536, PROST2006579, PROST2006688, PROST2006737, PROST2006766, PROST2006782, PROST2006933, PROST2007122, PROST2007200, PROST2007237, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008268, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009365, PROST2009388, PROST2009400, PROST2009470, PROST2009483, PROST2009571, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010885, PROST2011012, PROST2011105, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012400, PROST2012504, PROST2012542, PROST2012550, PROST2012740, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015287, PROST2015457, PROST2015537, PROST2015545, PROST2015551, PROST2015636, PROST2015756, PROST2015877, PROST2015900, PROST2015924, PROST2015932, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016980, PROST2017098, PROST2017128, PROST2017203, PROST2017367, PROST2017413, PROST2017441, PROST2017578, PROST2017612, PROST2017617, PROST2017700, PROST2017701, PROST2017729, PROST2017836, PROST2017910, PROST2018030, PROST2018090, PROST2018235, PROST2018437, PROST2018487, PROST2018511, PROST2018607, PROST2018788, PROST2018902, PROST2018922, PROST2019296, PROST2019398, PROST2019487, PROST2019781, PUAEN1000039, PUAEN1000081, PUAEN1000085, PUAEN1000087, PUAEN1000121, PUAEN1000175, PUAEN1000275, PUAEN1000276, PUAEN1000288, PUAEN2000080, PUAEN2000175, PUAEN2000247, PUAEN2000394, PUAEN2000497, PUAEN2000594, PUAEN2000684, PUAEN2000942, PUAEN2001260, PUAEN2001526, PUAEN2001882, PUAEN2002473, PUAEN2002552, PUAEN2002568, PUAEN2002616, PUAEN2002758, PUAEN2003079, PUAEN2003116, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2006029, PUAEN2006328, PUAEN2006578, PUAEN2006639, PUAEN2006761, PUAEN2007350, PUAEN2007785, PUAEN2007898, PUAEN2008123, PUAEN2008228, PUAEN2008314, PUAEN2008815, PUAEN2008939, PUAEN2008980, PUAEN2009174, PUAEN2009348, PUAEN2009534, PUAEN2009795, PUAEN2009852, RECTM1000051, RECTM1000141, RECTM2000220, RECTM2000349, RECTM2000433, RECTM2000510, RECTM2001307, RECTM2001519, RECTM2001666, RECTM2001691, RECTM2001749, RECTM2002172, RECTM2002602, RECTM2002632, SALGL1000024, SALGL1000028, SALGL1000065, SALGL1000107, SALGL1000171, SKMUS1000030, SKMUS1000064, SKMUS1000067, SKMUS1000104, SKMUS1000118, SKMUS1000124, SKMUS1000129, SKMUS1000138, SKMUS2000061, SKMUS2000074, SKMUS2000078, SKMUS2000137, SKMUS2000198, SKMUS2000243, SKMUS2000287, SKMUS2000317, SKMUS2000330, SKMUS2000339, SKMUS2000343, SKMUS2000365, SKMUS2000390, SKMUS2000416, SKMUS2000458, SKMUS2000467, SKMUS2000468, SKMUS2000515, SKMUS2000679, SKMUS2000690, SKMUS2000701, SKMUS2000705, SKMUS2000710, SKMUS2000724, SKMUS2000726, SKMUS2000774, SKMUS2000780, SKMUS2000821, SKMUS2000847, SKMUS2000863, SKMUS2000894, SKMUS2000898, SKMUS2000902, SKMUS2000933, SKMUS2000945, SKMUS2000973, SKMUS2000980, SKMUS2001008, SKMUS2001014, SKMUS2001129, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000137, SKNMC1000229, SKNMC1000251, SKNMC1000264, SKNMC2000224, SKNMC2000256, SKNMC2000356, SKNMC2000374, SKNMC2000583, SKNMC2000593, SKNMC2000612, SKNMC2000622, SKNMC2000698, SKNMC2000966, SKNMC2001113, SKNMC2001324, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006327, SKNMC2006998, SKNMC2007134, SKNMC2007502, SKNMC2007504, SKNMC2007961, SKNMC2009450, SKNSH1000086, SKNSH1000174, SKNSH1000301, SKNSH1000308, SKNSH1000416, SKNSH2000051, SKNSH2000151, SKNSH2000163, SKNSH2000319, SKNSH2000482, SKNSH2000516, SKNSH2000550, SKNSH2000971, SKNSH2001931, SKNSH2002054, SKNSH2002768, SKNSH2002866, SKNSH2003174, SKNSH2003422, SKNSH2003466, SKNSH2003483, SKNSH2003528, SKNSH2003633, SKNSH2004039, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009435, SKNSH2009991, SKNSH2010015, SMINT1000012, SMINT1000016, SMINT1000039, SMINT1000042, SMINT1000051, SMINT1000054, SMINT1000057, SMINT1000071, SMINT1000075, SMINT1000078, SMINT1000091, SMINT1000103, SMINT1000118, SMINT1000137, SMINT1000192, SMINT2000007, SMINT2000018, SMINT2000032, SMINT2000040, SMINT2000060, SMINT2000084, SMINT2000145, SMINT2000159, SMINT2000176, SMINT2000185, SMINT2000227, SMINT2000229, SMINT2000232, SMINT2000277, SMINT2000518, SMINT2000529, SMINT2000530, SMINT2000537, SMINT2000538, SMINT2000541, SMINT2000558, SMINT2000562, SMINT2000568, SMINT2000602, SMINT2000609, SMINT2000747, SMINT2000811, SMINT2000824, SMINT2000984, SMINT2001114, SMINT2001158, SMINT2001199, SMINT2001222, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2002126, SMINT2002202, SMINT2002210, SMINT2002281, SMINT2002311, SMINT2002314, SMINT2002328, SMINT2002414, SMINT2002457, SMINT2002743, SMINT2002778, SMINT2002880, SMINT2002882, SMINT2002884, SMINT2002899, SMINT2002974, SMINT2003003, SMINT2003074, SMINT2003128, SMINT2003150, SMINT2003317, SMINT2003340, SMINT2003386, SMINT2003505, SMINT2003551, SMINT2003569, SMINT2003641, SMINT2003644, SMINT2003866, SMINT2004037, SMINT2004047, SMINT2004086, SMINT2004144, SMINT2004280, SMINT2004299, SMINT2004339, SMINT2004414, SMINT2004473, SMINT2004547, SMINT2004583, SMINT2004729, SMINT2004781, SMINT2004891, SMINT2004909, SMINT2004972, SMINT2004992, SMINT2005075, SMINT2005161, SMINT2005174, SMINT2005213, SMINT2005387, SMINT2005402, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716, SMINT2006801, SMINT2007062, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008625, SMINT2008672, SMINT2008690, SMINT2008715, SMINT2008720, SMINT2008817, SMINT2008844, SMINT2008917, SMINT2009119, SMINT2009121, SMINT2009212, SMINT2009216, SMINT2009233, SMINT2009259, SMINT2009272, SMINT2009363, SMINT2009468, SMINT2009505, SMINT2009529, SMINT2009832, SMINT2009895, SMINT2009902, SMINT2009973, SMINT2010015, SMINT2010068, SMINT2010076, SMINT2010200, SMINT2010278, SMINT2010326, SMINT2010369, SMINT2010426, SMINT2010500, SMINT2010533, SMINT2010629, SMINT2010672, SMINT2010753, SMINT2010853, SMINT2010897, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012195, SMINT2012285, SMINT2012291, SMINT2012734, SMINT2012793, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016286, SMINT2016313, SMINT2016412, SMINT2016440, SMINT2016477, SMINT2016544, SMINT2016857, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000041, SPLEN1000056, SPLEN1000087, SPLEN1000089, SPLEN1000091, SPLEN1000134, SPLEN1000139, SPLEN1000143, SPLEN1000145, SPLEN1000156, SPLEN1000163, SPLEN1000178, SPLEN2000020, SPLEN2000047, SPLEN2000064, SPLEN2000072, SPLEN2000126, SPLEN2000143, SPLEN2000189, SPLEN2000205, SPLEN2000210, SPLEN2000242, SPLEN2000243, SPLEN2000247, SPLEN2000270, SPLEN2000307, SPLEN2000310, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000541, SPLEN2000607, SPLEN2000630, SPLEN2000695, SPLEN2000839, SPLEN2001135, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001650, SPLEN2001689, SPLEN2001710, SPLEN2001761, SPLEN2001781, SPLEN2001881, SPLEN2001912, SPLEN2001945, SPLEN2002007, SPLEN2002133, SPLEN2002147, SPLEN2002166, SPLEN2002175, SPLEN2002223, SPLEN2002314, SPLEN2002343, SPLEN2002354, SPLEN2002385, SPLEN2002419, SPLEN2002451, SPLEN2002462, SPLEN2002467, SPLEN2002477, SPLEN2002662, SPLEN2002695, SPLEN2002707, SPLEN2002917, SPLEN2002957, SPLEN2003160, SPLEN2003204, SPLEN2003219, SPLEN2003297, SPLEN2003878, SPLEN2003918, SPLEN2003924, SPLEN2004060, SPLEN2004078, SPLEN2004124, SPLEN2004148, SPLEN2004181, SPLEN2004220, SPLEN2004346, SPLEN2004368, SPLEN2004611, SPLEN2004662, SPLEN2004844, SPLEN2005227, SPLEN2005272, SPLEN2005416, SPLEN2005450, SPLEN2005474, SPLEN2005727, SPLEN2005790, SPLEN2005818, SPLEN2005869, SPLEN2005939, SPLEN2006232, SPLEN2006268, SPLEN2006283, SPLEN2006325, SPLEN2006389, SPLEN2006425, SPLEN2006671, SPLEN2006701, SPLEN2006785, SPLEN2006875, SPLEN2007314, SPLEN2007326, SPLEN2007350, SPLEN2007388, SPLEN2007619, SPLEN2007653, SPLEN2007689, SPLEN2007695, SPLEN2007750, SPLEN2007794, SPLEN2007879, SPLEN2007926, SPLEN2007951, SPLEN2008007, SPLEN2008135, SPLEN2008164, SPLEN2008317, SPLEN2008496, SPLEN2008591, SPLEN2008786, SPLEN2009088, SPLEN2009163, SPLEN2009315, SPLEN2009340, SPLEN2009512, SPLEN2009513, SPLEN2009541, SPLEN2009548, SPLEN2009581, SPLEN2009733, SPLEN2009803, SPLEN2009884, SPLEN2009918, SPLEN2010004, SPLEN2010112, SPLEN2010119, SPLEN2010146, SPLEN2010187, SPLEN2010350, SPLEN2010395, SPLEN2010415, SPLEN2010447, SPLEN2010510, SPLEN2010530, SPLEN2010534, SPLEN2010588, SPLEN2010625, SPLEN2010800, SPLEN2010846, SPLEN2010847, SPLEN2010862, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012524, SPLEN2012525, SPLEN2012606, SPLEN2012611, SPLEN2012725, SPLEN2012743, SPLEN2012756, SPLEN2012846, SPLEN2012889, SPLEN2012961, SPLEN2013108, SPLEN2013195, SPLEN2013644, SPLEN2013648, SPLEN2013670, SPLEN2013673, SPLEN2013690, SPLEN2013714, SPLEN2013753, SPLEN2013821, SPLEN2013860, SPLEN2013910, SPLEN2013923, SPLEN2014041, SPLEN2014063, SPLEN2014073, SPLEN2014080, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014669, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015815, SPLEN2015890, SPLEN2015891, SPLEN2015899, SPLEN2016045, SPLEN2016053, SPLEN2016069, SPLEN2016098, SPLEN2016135, SPLEN2016241, SPLEN2016250, SPLEN2016304, SPLEN2016356, SPLEN2016480, SPLEN2016531, SPLEN2016541, SPLEN2016552, SPLEN2016554, SPLEN2016602, SPLEN2016627, SPLEN2016678, SPLEN2016773, SPLEN2016781, SPLEN2016840, SPLEN2016863, SPLEN2016922, SPLEN2016973, SPLEN2017043, SPLEN2017104, SPLEN2017147, SPLEN2017187, SPLEN2017189, SPLEN2017212, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018098, SPLEN2018112, SPLEN2018180, SPLEN2018181, SPLEN2018280, SPLEN2018285, SPLEN2018364, SPLEN2018395, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019257, SPLEN2019311, SPLEN2019323, SPLEN2019375, SPLEN2019379, SPLEN2019446, SPLEN2019471, SPLEN2019480, SPLEN2019505, SPLEN2019571, SPLEN2019575, SPLEN2019709, SPLEN2019793, SPLEN2019811, SPLEN2019839, SPLEN2019956, SPLEN2019985, SPLEN2020007, SPLEN2020153, SPLEN2020154, SPLEN2020166, SPLEN2020183, SPLEN2020223, SPLEN2020359, SPLEN2020427, SPLEN2020467, SPLEN2020512, SPLEN2020608, SPLEN2020777, SPLEN2020805, SPLEN2021057, SPLEN2021157, SPLEN2021231, SPLEN2021295, SPLEN2021298, SPLEN2021383, SPLEN2021417, SPLEN2021440, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024530, SPLEN2024571, SPLEN2024577, SPLEN2024617, SPLEN2024737, SPLEN2024956, SPLEN2024990, SPLEN2025012, SPLEN2025017, SPLEN2025039, SPLEN2025219, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031724, SPLEN2031780, SPLEN2032036, SPLEN2032112, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034864, SPLEN2034934, SPLEN2035318, SPLEN2035615, SPLEN2036103, SPLEN2036326, SPLEN2036475, SPLEN2036501, SPLEN2036579, SPLEN2036608, SPLEN2036702, SPLEN2036712, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042598, SPLEN2042714, SPLEN2042920, STOMA1000013, STOMA1000047, STOMA1000052, STOMA1000074, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000279, STOMA2000383, STOMA2000386, STOMA2000395, STOMA2000478, STOMA2000529, STOMA2000539, STOMA2000567, STOMA2000640, STOMA2000678, STOMA2000686, STOMA2000888, STOMA2001025, STOMA2001389, STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005664, STOMA2005667, STOMA2005746, STOMA2005782, STOMA2006181, STOMA2006213, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, SYNOV1000045, SYNOV1000049, SYNOV1000118, SYNOV1000164, SYNOV1000190, SYNOV1000256, SYNOV2000152, SYNOV2000171, SYNOV2000173, SYNOV2000177, SYNOV2000178, SYNOV2000193, SYNOV2000251, SYNOV2000279, SYNOV2000297, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001212, SYNOV2001251, SYNOV2001300, SYNOV2001356, SYNOV2001374, SYNOV2001390, SYNOV2001451, SYNOV2001457, SYNOV2001468, SYNOV2001660, SYNOV2003326, SYNOV2005216, SYNOV2005448, SYNOV2005541, SYNOV2005673, SYNOV2005817, SYNOV2005962, SYNOV2006430, SYNOV2007965, SYNOV2008765, SYNOV2009172, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4006256, SYNOV4006327, SYNOV4007012, SYNOV4007215, SYNOV4007360, SYNOV4007521, SYNOV4007553, SYNOV4007711, SYNOV4008336, SYNOV4009139, SYNOV4009298, T1ESE2000116, T1ESE2000609, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN2000139, TCOLN2000236, TCOLN2000641, TCOLN2001268, TCOLN2002047, TCOLN2002278, TESOP1000127, TESOP1000160, TESOP2000083, TESOP2000090, TESOP2000205, TESOP2000312, TESOP2000390, TESOP2000400, TESOP2000510, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000055, TESTI1000064, TESTI1000085, TESTI1000088, TESTI1000093, TESTI1000109, TESTI1000125, TESTI1000127, TESTI1000128, TESTI1000131, TESTI1000138, TESTI1000142, TESTI1000157, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000018, TESTI2000044, TESTI2000100, TESTI2000117, TESTI2000120, TESTI2000147, TESTI2000154, TESTI2000179, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000443, TESTI2000452, TESTI2000462, TESTI2000571, TESTI2000591, TESTI2000598, TESTI2000616, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000850, TESTI2000909, TESTI2000951, TESTI2000981, TESTI2001008, TESTI2001049, TESTI2001082, TESTI2001134, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001879, TESTI2001894, TESTI2001915, TESTI2001950, TESTI2001959, TESTI2001968, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002498, TESTI2002516, TESTI2002544, TESTI2002578, TESTI2002580, TESTI2002618, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003152, TESTI2003181, TESTI2003193, TESTI2003196, TESTI2003228, TESTI2003236, TESTI2003255, TESTI2003258, TESTI2003277, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003475, TESTI2003476, TESTI2003498, TESTI2003512, TESTI2003539, TESTI2003545, TESTI2003551, TESTI2003574, TESTI2003579, TESTI2003580, TESTI2003589, TESTI2003598, TESTI2003615, TESTI2003638, TESTI2003649, TESTI2003718, TESTI2003727, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004085, TESTI2004089, TESTI2004111, TESTI2004119, TESTI2004122, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004783, TESTI2004791, TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005739, TESTI2005767, TESTI2005775, TESTI2005827, TESTI2005860, TESTI2005892, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006120, TESTI2006125, TESTI2006179, TESTI2006209, TESTI2006210, TESTI2006212, TESTI2006218, TESTI2006238, TESTI2006255, TESTI2006278, TESTI2006333, TESTI2006341, TESTI2006360, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007320, TESTI2007346, TESTI2007358, TESTI2007402, TESTI2007405, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835, TESTI2009853, TESTI2009935, TESTI2009977, TESTI2010009, TESTI2010154, TESTI2010239, TESTI2010280, TESTI2010354, TESTI2010396, TESTI2010400, TESTI2010409, TESTI2010572, TESTI2010587, TESTI2010591, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011612, TESTI2011665, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012592, TESTI2012617, TESTI2012628, TESTI2012778, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013264, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014800, TESTI2014843, TESTI2014979, TESTI2014988, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015987, TESTI2016033, TESTI2016046, TESTI2016101, TESTI2016162, TESTI2016197, TESTI2016298, TESTI2016331, TESTI2016337, TESTI2016410, TESTI2016421, TESTI2016509, TESTI2016568, TESTI2016599, TESTI2016629, TESTI2016663, TESTI2016667, TESTI2016688, TESTI2016701, TESTI2016758, TESTI2016848, TESTI2016863, TESTI2016888, TESTI2016922, TESTI2016942, TESTI2016950, TESTI2016996, TESTI2017017, TESTI2017028, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018428, TESTI2018475, TESTI2018482, TESTI2018521, TESTI2018565, TESTI2018581, TESTI2018687, TESTI2018697, TESTI2018838, TESTI2018867, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020981, TESTI2020999, TESTI2021003, TESTI2021057, TESTI2021077, TESTI2021112, TESTI2021114, TESTI2021116, TESTI2021118, TESTI2021122, TESTI2021138, TESTI2021315, TESTI2021447, TESTI2021463, TESTI2021599, TESTI2021637, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025486, TESTI2025546, TESTI2025582, TESTI2025609, TESTI2025656, TESTI2025791, TESTI2025872, TESTI2025879, TESTI2025911, TESTI2025920, TESTI2025924, TESTI2026014, TESTI2026024, TESTI2026064, TESTI2026077, TESTI2026104, TESTI2026134, TESTI2026168, TESTI2026196, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029244, TESTI2029249, TESTI2029252, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034931, TESTI2034953, TESTI2034997, TESTI2035003, TESTI2035078, TESTI2035084, TESTI2035107, TESTI2035121, TESTI2035124, TESTI2035183, TESTI2035262, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036736, TESTI2036822, TESTI2036833, TESTI2036913, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527, TESTI4001561, TESTI4001665, TESTI4001679, TESTI4001795, TESTI4001923, TESTI4001925, TESTI4002003, TESTI4002141, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003179, TESTI4003279, TESTI4003319, TESTI4003404, TESTI4003565, TESTI4003574, TESTI4003579, TESTI4003733, TESTI4003796, TESTI4004003, TESTI4004031, TESTI4004200, TESTI4004539, TESTI4004653, TESTI4004722, TESTI4004887, TESTI4004917, TESTI4005015, TESTI4005039, TESTI4005158, TESTI4005317, TESTI4005322, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006148, TESTI4006219, TESTI4006234, TESTI4006308, TESTI4006326, TESTI4006393, TESTI4006412, TESTI4006420, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4008007, TESTI4008018, TESTI4008050, TESTI4008086, TESTI4008176, TESTI4008209, TESTI4008219, TESTI4008250, TESTI4008302, TESTI4008305, TESTI4008401, TESTI4008417, TESTI4008429, TESTI4008573, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010475, TESTI4010544, TESTI4010713, TESTI4010721, TESTI4010789, TESTI4010817, TESTI4010831, TESTI4010902, TESTI4011070, TESTI4011072, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012505, TESTI4012556, TESTI4012623, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013735, TESTI4013742, TESTI4013774, TESTI4013830, TESTI4013852, TESTI4013924, TESTI4013960, TESTI4013962, TESTI4014175, TESTI4014265, TESTI4014276, TESTI4014306, TESTI4014392, TESTI4014415, TESTI4014445, TESTI4014553, TESTI4014661, TESTI4014801, TESTI4014891, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024494, TESTI4024740, TESTI4024874, TESTI4024890, TESTI4024907, TESTI4024930, TESTI4025062, TESTI4025401, TESTI4025494, TESTI4025731, TESTI4025797, TESTI4025865, TESTI4025908, TESTI4025920, TESTI4026079, TESTI4026080, TESTI4026192, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032112, TESTI4032128, TESTI4032270, TESTI4032375, TESTI4032834, TESTI4032856, TESTI4032895, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046090, TESTI4046240, TESTI4046245, TESTI4046253, TESTI4046282, TESTI4046328, TESTI4046450, TESTI4046457, TESTI4046487, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000032, THYMU1000033, THYMU1000060, THYMU1000074, THYMU1000098, THYMU1000136, THYMU1000142, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000331, THYMU1000336, THYMU1000366, THYMU1000382, THYMU1000393, THYMU1000394, THYMU1000399, THYMU1000426, THYMU1000428, THYMU1000440, THYMU1000459, THYMU1000473, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000120, THYMU2000140, THYMU2000162, THYMU2000171, THYMU2000176, THYMU2000179, THYMU2000231, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000702, THYMU2000767, THYMU2000800, THYMU2000879, THYMU2000932, THYMU2000950, THYMU2000982, THYMU2000990, THYMU2001053, THYMU2001071, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001443, THYMU2001521, THYMU2001556, THYMU2001622, THYMU2001727, THYMU2001819, THYMU2001825, THYMU2001839, THYMU2001898, THYMU2001900, THYMU2001916, THYMU2001926, THYMU2001989, THYMU2002023, THYMU2002037, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002841, THYMU2002852, THYMU2002910, THYMU2002950, THYMU2002983, THYMU2003069, THYMU2003107, THYMU2003124, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003632, THYMU2003650, THYMU2003700, THYMU2003760, THYMU2003803, THYMU2003855, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004508, THYMU2004512, THYMU2004639, THYMU2004668, THYMU2004677, THYMU2004688, THYMU2004693, THYMU2004835, THYMU2004843, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006666, THYMU2006712, THYMU2006913, THYMU2006946, THYMU2006965, THYMU2007025, THYMU2007060, THYMU2007065, THYMU2007095, THYMU2007146, THYMU2007179, THYMU2007258, THYMU2007307, THYMU2007308, THYMU2007339, THYMU2007350, THYMU2007384, THYMU2007415, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007802, THYMU2007824, THYMU2007854, THYMU2007952, THYMU2007969, THYMU2008036, THYMU2008072, THYMU2008111, THYMU2008145, THYMU2008185, THYMU2008191, THYMU2008207, THYMU2008226, THYMU2008282, THYMU2008339, THYMU2008350, THYMU2008351, THYMU2008383, THYMU2008452, THYMU2008643, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010046, THYMU2010059, THYMU2010082, THYMU2010094, THYMU2010161, THYMU2010448, THYMU2010519, THYMU2010637, THYMU2010671, THYMU2010699, THYMU2010705, THYMU2010779, THYMU2010816, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012073, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807, THYMU2012882, THYMU2012891, THYMU2012902, THYMU2012919, THYMU2013023, THYMU2013047, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015943, THYMU2015984, THYMU2016113, THYMU2016164, THYMU2016204, THYMU2016219, THYMU2016360, THYMU2016496, THYMU2016523, THYMU2016840, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023711, THYMU2023900, THYMU2024071, THYMU2024121, THYMU2024185, THYMU2024207, THYMU2024616, THYMU2024684, THYMU2024748, THYMU2025042, THYMU2025189, THYMU2025406, THYMU2025557, THYMU2025572, THYMU2025849, THYMU2025909, THYMU2025939, THYMU2026182, THYMU2026350, THYMU2026530, THYMU2026531, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031368, THYMU2031579, THYMU2031847, THYMU2031994, THYMU2032014, THYMU2032035, THYMU2032080, THYMU2032150, THYMU2032155, THYMU2032358, THYMU2032437, THYMU2032601, THYMU2032655, THYMU2032715, THYMU2032732, THYMU2032976, THYMU2033053, THYMU2033059, THYMU2033070, THYMU2033079, THYMU2033085, THYMU2033104, THYMU2033227, THYMU2033401, THYMU2033583, THYMU2033755, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036252, THYMU2036265, THYMU2036366, THYMU2036459, THYMU2036604, THYMU2036653, THYMU2037081, THYMU2037208, THYMU2037233, THYMU2037234, THYMU2037348, THYMU2037406, THYMU2037965, THYMU2038058, THYMU2038189, THYMU2038199, THYMU2038301, THYMU2038369, THYMU2038389, THYMU2038615, THYMU2038636, THYMU2038739, THYMU2038772, THYMU2038797, THYMU2038906, THYMU2039305, THYMU2039334, THYMU2039350, THYMU2039411, THYMU2039719, THYMU2039780, THYMU2039788, THYMU2039989, THYMU2040029, THYMU2040110, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000390, THYMU3000490, THYMU3000504, THYMU3000542, THYMU3000655, THYMU3000826, THYMU3000841, THYMU3001077, THYMU3001082, THYMU3001083, THYMU3001133, THYMU3001151, THYMU3001472, THYMU3001593, THYMU3001883, THYMU3001939, THYMU3001991, THYMU3002452, THYMU3002578, THYMU3002661, THYMU3003092, THYMU3003212, THYMU3003309, THYMU3003403, THYMU3003565, THYMU3003763, THYMU3003865, THYMU3003958, THYMU3004157, THYMU3004628, THYMU3004632, THYMU3004866, THYMU3005050, THYMU3005378, THYMU3005407, THYMU3005415, THYMU3005482, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007368, THYMU3007559, THYMU3007845, THYMU3008060, THYMU3008136, THYMU3008216, THYMU3008436, THYMU3008672, THYMU3008883, THYMU3008935, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3033402, THYMU3033626, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037836, THYMU3037856, THYMU3037867, THYMU3037909, THYMU3037980, THYMU3038158, THYMU3038167, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046360, THYMU3046856, THYMU3047115, THYMU3047144, THYMU3047156, THYMU3047366, THYMU3047513, THYMU3047542, THYMU3047760, THYMU3047891, TKIDN1000062, TKIDN1000066, TKIDN1000171, TKIDN2000127, TKIDN2000240, TKIDN2000464, TKIDN2000701, TKIDN2001529, TKIDN2002329, TKIDN2002424, TKIDN2002632, TKIDN2003044, TKIDN2003062, TKIDN2003366, TKIDN2003396, TKIDN2003413, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007667, TKIDN2008176, TKIDN2008778, TKIDN2009092, TKIDN2009481, TKIDN2010200, TKIDN2010232, TKIDN2010278, TKIDN2010602, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014757, TKIDN2014771, TKIDN2015025, TKIDN2015161, TKIDN2015263, TKIDN2015285, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2001281, TOVAR2001556, TOVAR2001730, TOVAR2002247, TOVAR2002514, TOVAR2002549, TOVAR2002800, TRACH1000038, TRACH1000057, TRACH1000063, TRACH1000074, TRACH1000100, TRACH1000106, TRACH1000125, TRACH1000140, TRACH1000144, TRACH1000212, TRACH1000240, TRACH2000002, TRACH2000015, TRACH2000024, TRACH2000032, TRACH2000081, TRACH2000083, TRACH2000148, TRACH2000185, TRACH2000196, TRACH2000235, TRACH2000243, TRACH2000250, TRACH2000259, TRACH2000272, TRACH2000282, TRACH2000287, TRACH2000289, TRACH2000306, TRACH2000312, TRACH2000336, TRACH2000411, TRACH2000496, TRACH2000497, TRACH2000522, TRACH2000559, TRACH2000660, TRACH2000665, TRACH2000675, TRACH2000677, TRACH2000702, TRACH2000703, TRACH2000767, TRACH2000775, TRACH2000807, TRACH2000862, TRACH2000873, TRACH2000894, TRACH2000898, TRACH2000923, TRACH2000944, TRACH2000981, TRACH2000986, TRACH2001037, TRACH2001060, TRACH2001101, TRACH2001121, TRACH2001147, TRACH2001154, TRACH2001188, TRACH2001192, TRACH2001249, TRACH2001275, TRACH2001335, TRACH2001347, TRACH2001395, TRACH2001400, TRACH2001403, TRACH2001416, TRACH2001443, TRACH2001474, TRACH2001523, TRACH2001525, TRACH2001532, TRACH2001592, TRACH2001596, TRACH2001621, TRACH2001651, TRACH2001684, TRACH2001933, TRACH2001968, TRACH2002043, TRACH2002054, TRACH2002092, TRACH2002138, TRACH2002784, TRACH2002840, TRACH2002847, TRACH2002853, TRACH2002862, TRACH2002954, TRACH2002960, TRACH2002988, TRACH2003070, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005666, TRACH2005698, TRACH2005710, TRACH2005720, TRACH2005769, TRACH2005800, TRACH2006015, TRACH2006097, TRACH2006194, TRACH2006327, TRACH2006331, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894, TRACH2007988, TRACH2008081, TRACH2008112, TRACH2008127, TRACH2008130, TRACH2008278, TRACH2008540, TRACH2008583, TRACH2008597, TRACH2008820, TRACH2008921, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011290, TRACH2011293, TRACH2011302, TRACH2011574, TRACH2011956, TRACH2012000, TRACH2012138, TRACH2012242, TRACH2012370, TRACH2012387, TRACH2012536, TRACH2012562, TRACH2012742, TRACH2012823, TRACH2012918, TRACH2013081, TRACH2013123, TRACH2013265, TRACH2013369, TRACH2013450, TRACH2013495, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2015180, TRACH2015243, TRACH2015316, TRACH2015381, TRACH2015486, TRACH2015654, TRACH2015824, TRACH2015987, TRACH2016080, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018084, TRACH2018262, TRACH2018273, TRACH2018278, TRACH2018345, TRACH2018380, TRACH2018449, TRACH2018512, TRACH2018646, TRACH2018663, TRACH2018718, TRACH2018835, TRACH2018882, TRACH2018914, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000017, TRACH3000043, TRACH3000180, TRACH3000342, TRACH3000420, TRACH3000515, TRACH3000530, TRACH3000548, TRACH3000558, TRACH3000586, TRACH3000692, TRACH3000892, TRACH3000926, TRACH3001119, TRACH3001443, TRACH3001759, TRACH3002064, TRACH3002107, TRACH3002168, TRACH3002188, TRACH3002245, TRACH3002293, TRACH3002533, TRACH3002561, TRACH3002567, TRACH3002650, TRACH3002866, TRACH3002871, TRACH3002876, TRACH3002890, TRACH3003009, TRACH3003357, TRACH3003379, TRACH3003586, TRACH3003683, TRACH3003780, TRACH3003805, TRACH3003832, TRACH3004113, TRACH3004288, TRACH3004303, TRACH3004412, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007541, TRACH3007595, TRACH3007627, TRACH3007766, TRACH3007866, TRACH3007995, TRACH3008042, TRACH3008061, TRACH3008535, TRACH3008629, TRACH3008688, TRACH3008697, TRACH3008713, TRACH3008887, TRACH3008902, TRACH3008990, TRACH3009059, TRACH3009061, TRACH3009701, TRACH3009715, TRACH3009894, TRACH3009956, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024423, TRACH3024428, TRACH3024512, TRACH3024823, TRACH3025302, TRACH3025346, TRACH3025520, TRACH3026148, TRACH3026283, TRACH3026299, TRACH3026542, TRACH3026650, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036997, TRACH3037063, TRACH3037267, TRACH3037288, TRACH3037505, TRACH3037573, TRACH3037696, TRACH3037897, TRACH3038086, TSTOM1000186, TSTOM2000139, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000156, UMVEN2000046, UMVEN2000069, UTERU1000008, UTERU1000015, UTERU1000024, UTERU1000031, UTERU1000032, UTERU1000057, UTERU1000065, UTERU1000077, UTERU1000093, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU2000047, UTERU2000074, UTERU2000099, UTERU2000154, UTERU2000197, UTERU2000218, UTERU2000238, UTERU2000243, UTERU2000263, UTERU2000300, UTERU2000338, UTERU2000349, UTERU2000377, UTERU2000393, UTERU2000465, UTERU2000485, UTERU2000517, UTERU2000524, UTERU2000537, UTERU2000539, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2001024, UTERU2001176, UTERU2001281, UTERU2001389, UTERU2001409, UTERU2001412, UTERU2001504, UTERU2001747, UTERU2001876, UTERU2002001, UTERU2002011, UTERU2002176, UTERU2002198, UTERU2002332, UTERU2002473, UTERU2002547, UTERU2002693, UTERU2002733, UTERU2002736, UTERU2002737, UTERU2002826, UTERU2002841, UTERU2002993, UTERU2003035, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004039, UTERU2004061, UTERU2004073, UTERU2004163, UTERU2004197, UTERU2004299, UTERU2004461, UTERU2004520, UTERU2004564, UTERU2004688, UTERU2004698, UTERU2004861, UTERU2005004, UTERU2005050, UTERU2005069, UTERU2005179, UTERU2005292, UTERU2005346, UTERU2005446, UTERU2005449, UTERU2005450, UTERU2005533, UTERU2005822, UTERU2005903, UTERU2005905, UTERU2006103, UTERU2006137, UTERU2006182, UTERU2006400, UTERU2006412, UTERU2006429, UTERU2006568, UTERU2006651, UTERU2006705, UTERU2006899, UTERU2007075, UTERU2007081, UTERU2007128, UTERU2007267, UTERU2007444, UTERU2007499, UTERU2007639, UTERU2007724, UTERU2007942, UTERU2008019, UTERU2008027, UTERU2008077, UTERU2008347, UTERU2008516, UTERU2008561, UTERU2008653, UTERU2008705, UTERU2008707, UTERU2008785, UTERU2008845, UTERU2008901, UTERU2008930, UTERU2008962, UTERU2009094, UTERU2009120, UTERU2009131, UTERU2009147, UTERU2009206, UTERU2009283, UTERU2009414, UTERU2009483, UTERU2009510, UTERU2009538, UTERU2009540, UTERU2009776, UTERU2009904, UTERU2009951, UTERU2010115, UTERU2010124, UTERU2010164, UTERU2010226, UTERU2010231, UTERU2010304, UTERU2010431, UTERU2010525, UTERU2010651, UTERU2010724, UTERU2010747, UTERU2011195, UTERU2011199, UTERU2011220, UTERU2011261, UTERU2011410, UTERU2011574, UTERU2011657, UTERU2011741, UTERU2011811, UTERU2011897, UTERU2011962, UTERU2011968, UTERU2012101, UTERU2012114, UTERU2012252, UTERU2012333, UTERU2012407, UTERU2012526, UTERU2012581, UTERU2012610, UTERU2012615, UTERU2012688, UTERU2012703, UTERU2012741, UTERU2012767, UTERU2012786, UTERU2012856, UTERU2012890, UTERU2012938, UTERU2012976, UTERU2013048, UTERU2013231, UTERU2013262, UTERU2013280, UTERU2013322, UTERU2013483, UTERU2013491, UTERU2013502, UTERU2013926, UTERU2013976, UTERU2014001, UTERU2014223, UTERU2014398, UTERU2014548, UTERU2014631, UTERU2014668, UTERU2014678, UTERU2014728, UTERU2014998, UTERU2015062, UTERU2015087, UTERU2015190, UTERU2015198, UTERU2015202, UTERU2015405, UTERU2015640, UTERU2015830, UTERU2015880, UTERU2016147, UTERU2016157, UTERU2016464, UTERU2016669, UTERU2016761, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018333, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019038, UTERU2019163, UTERU2019257, UTERU2019453, UTERU2019491, UTERU2019534, UTERU2019681, UTERU2019710, UTERU2019959, UTERU2019964, UTERU2020226, UTERU2020242, UTERU2020292, UTERU2020351, UTERU2020491, UTERU2020718, UTERU2021380, UTERU2021649, UTERU2022020, UTERU2022773, UTERU2022955, UTERU2022981, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026775, UTERU2027023, UTERU2027369, UTERU2027591, UTERU2027616, UTERU2027941, UTERU2028377, UTERU2029503, UTERU2029660, UTERU2029742, UTERU2029953, UTERU2030270, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000402, UTERU3000645, UTERU3000665, UTERU3000670, UTERU3000727, UTERU3000738, UTERU3000828, UTERU3000844, UTERU3000959, UTERU3001029, UTERU3001053, UTERU3001059, UTERU3001240, UTERU3001394, UTERU3001542, UTERU3001558, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003178, UTERU3003465, UTERU3003495, UTERU3003523, UTERU3003774, UTERU3003776, UTERU3004477, UTERU3004523, UTERU3004616, UTERU3004635, UTERU3004938, UTERU3004992, UTERU3005049, UTERU3005205, UTERU3005264, UTERU3005460, UTERU3005536, UTERU3005585, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007731, UTERU3007913, UTERU3008463, UTERU3008660, UTERU3008671, UTERU3008722, UTERU3008917, UTERU3009490, UTERU3009517, UTERU3009690, UTERU3009775, UTERU3009979, UTERU3009987, UTERU3010029, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012476, UTERU3012599, UTERU3012999, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016274, UTERU3016308, UTERU3016789, UTERU3017176, UTERU3017441, UTERU3017626, UTERU3018154, UTERU3018255, UTERU3018616, UTERU3018711, UTERU3019078

Of the 17,176 genes that include the cDNAs of the present invention, genes whose expression levels were high enough to detect in each of the CD34C, CERVX, CHONS, COLON, CORDB, and CTONG libraries were analyzed for expression frequency. The analysis results are described below. Each is a relative value, where the expression level over all tissues is taken as 100. The larger this value, the greater the level of expression.

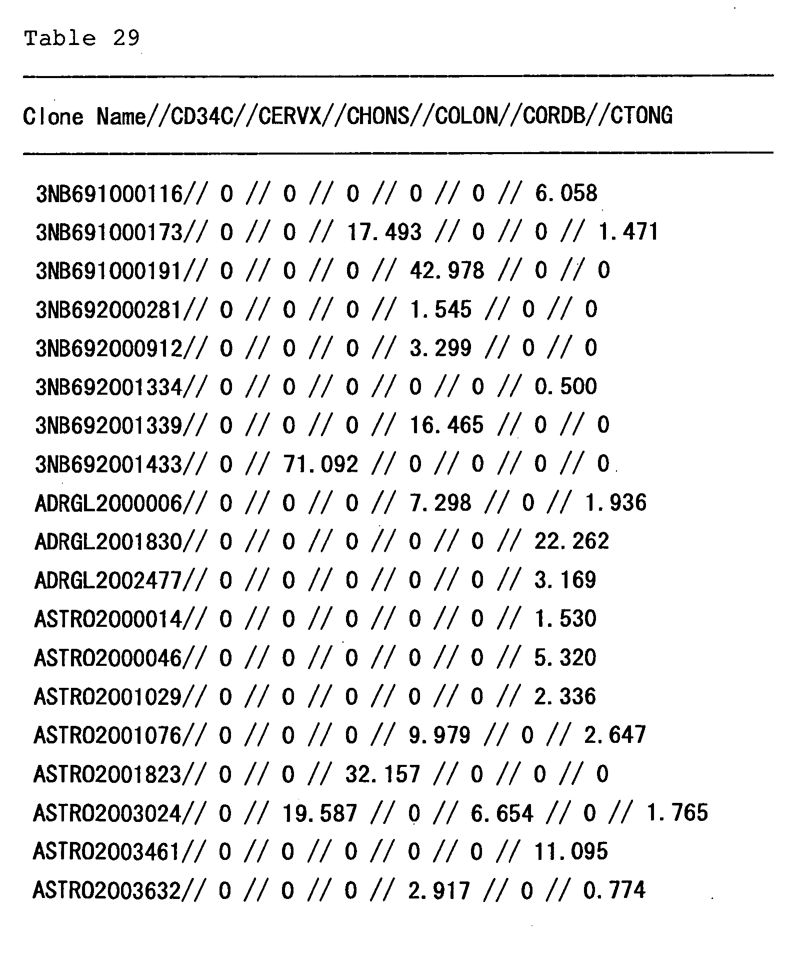

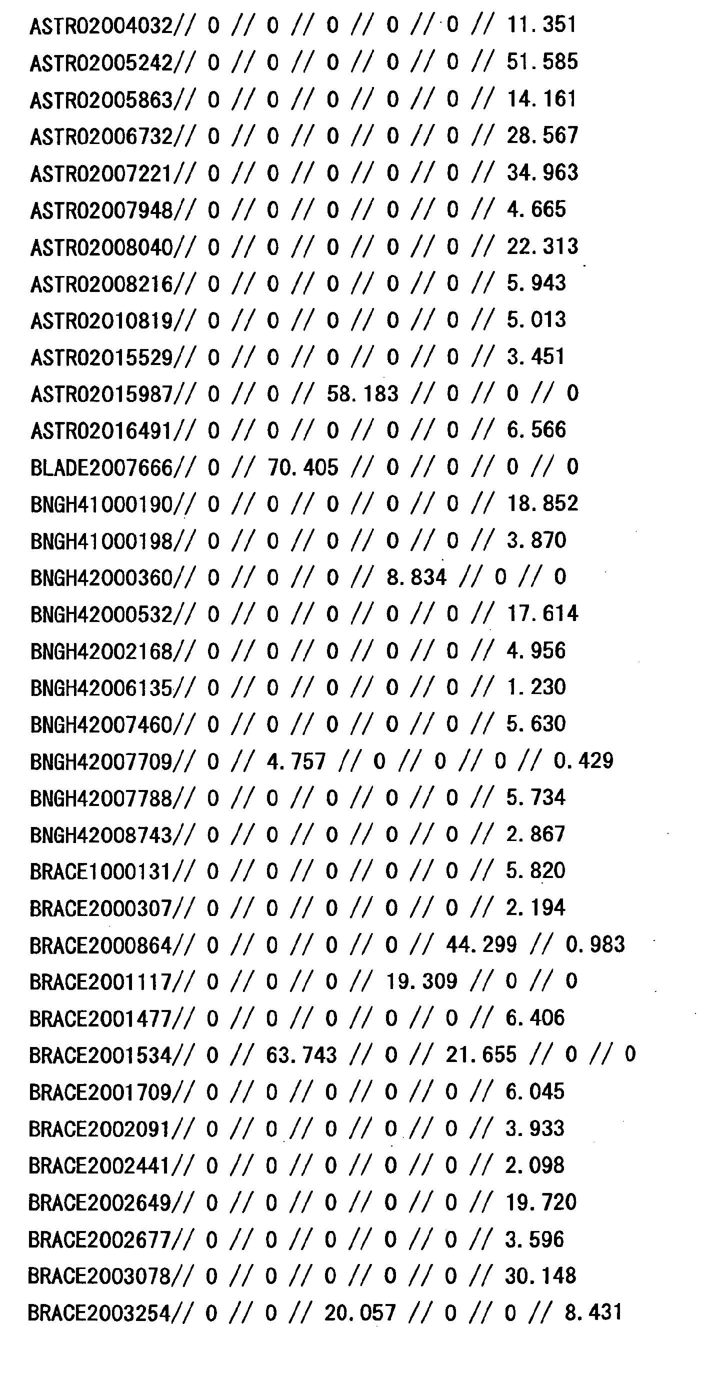

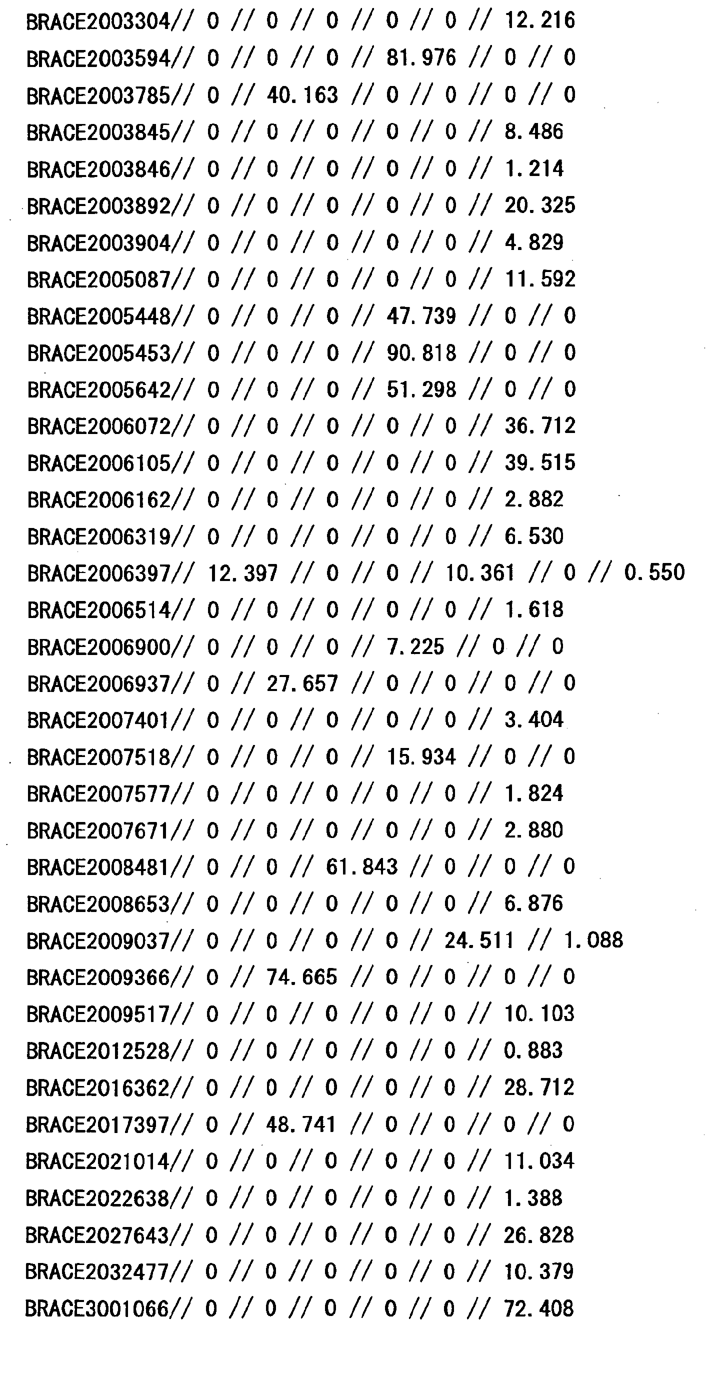

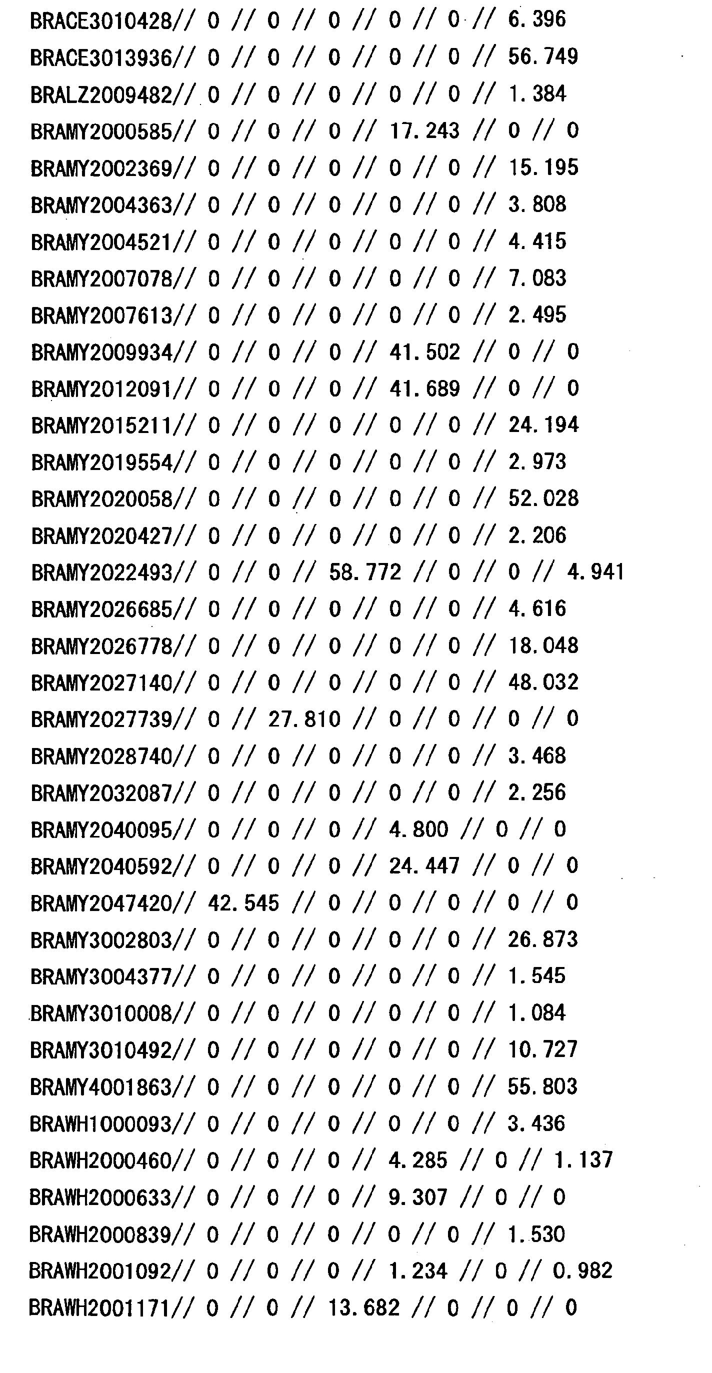

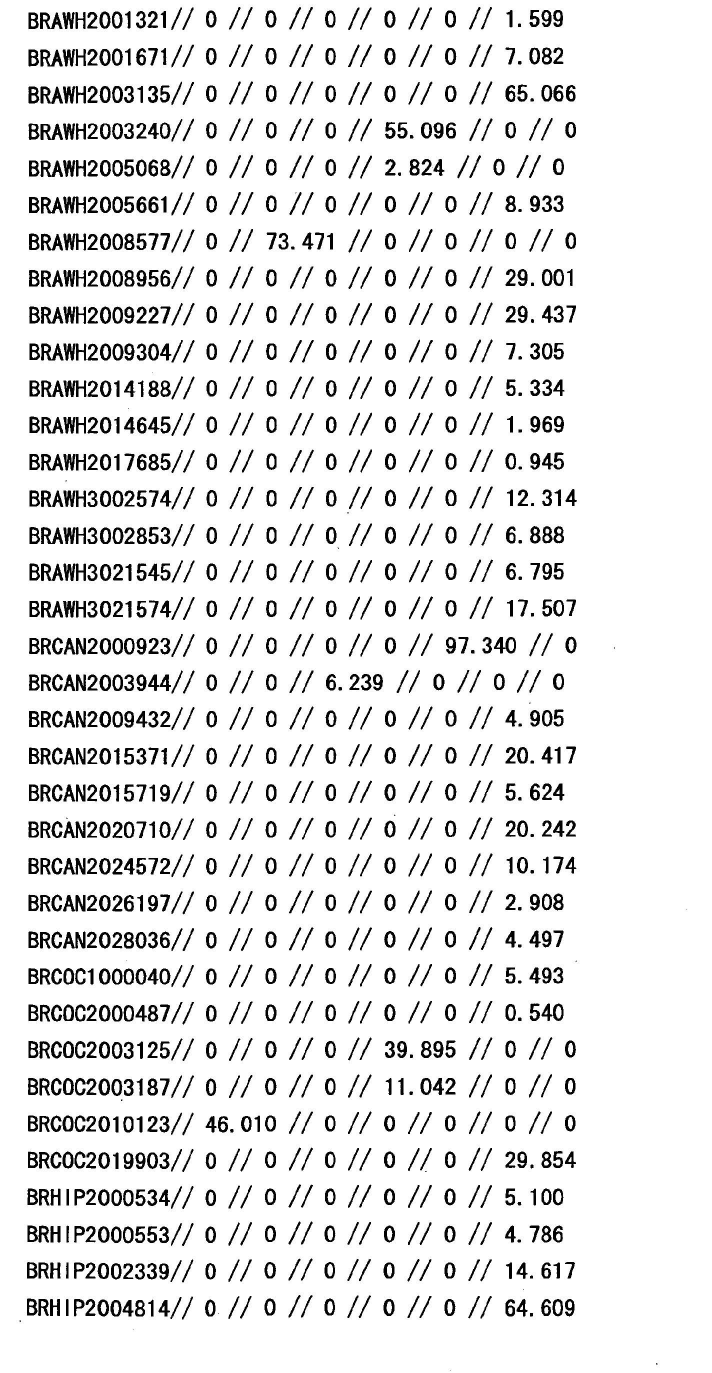

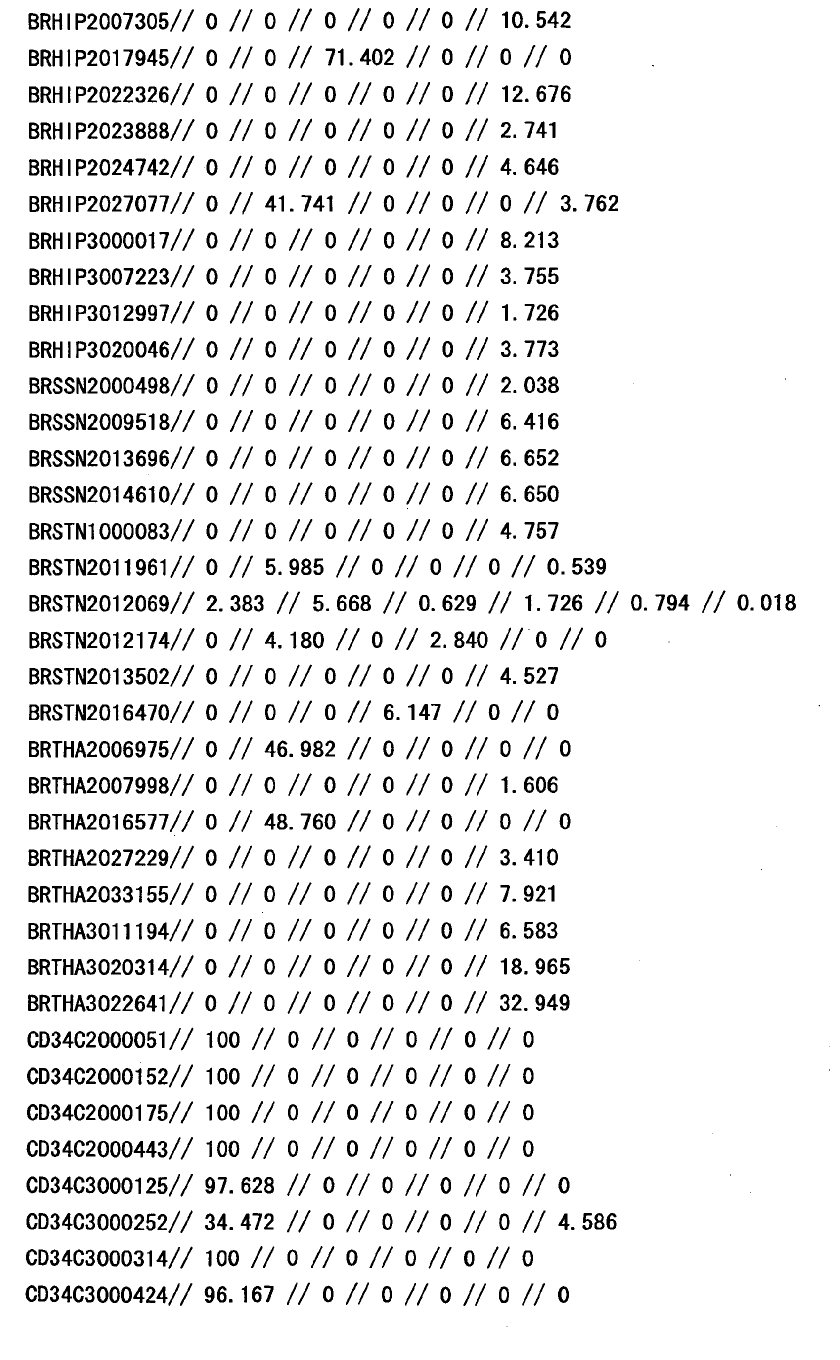

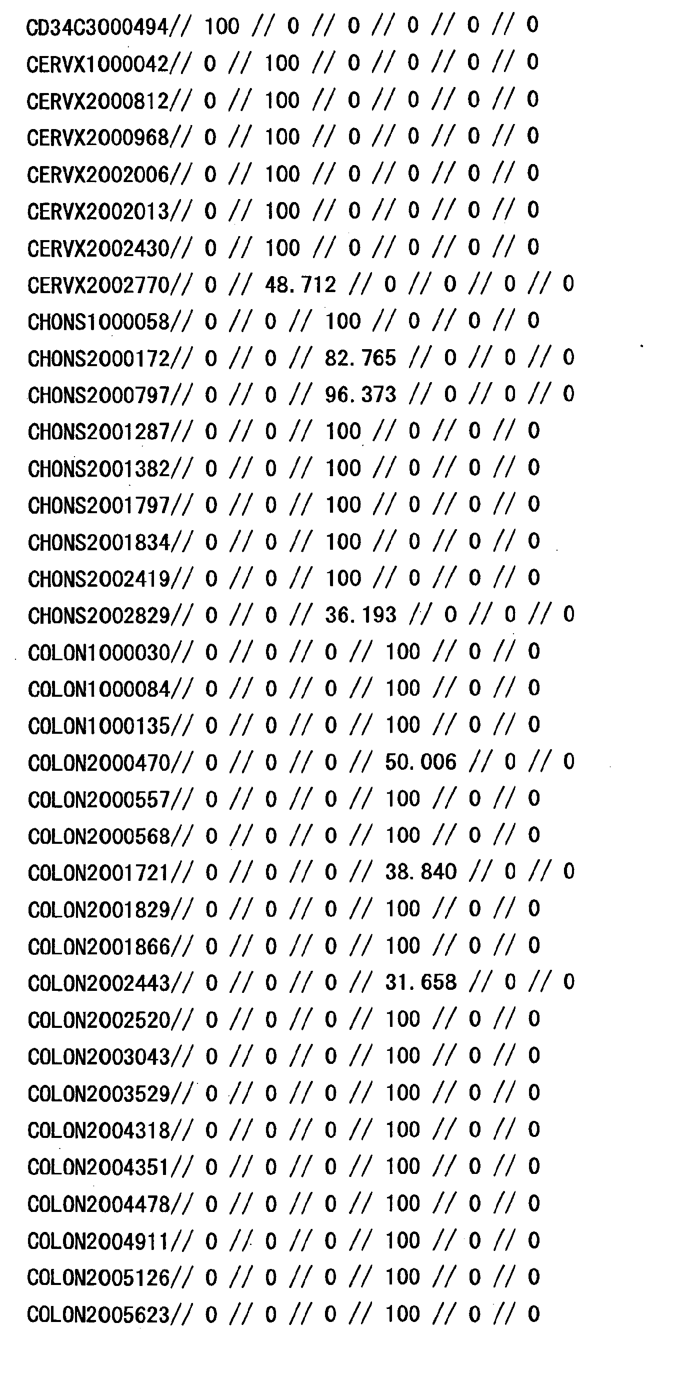

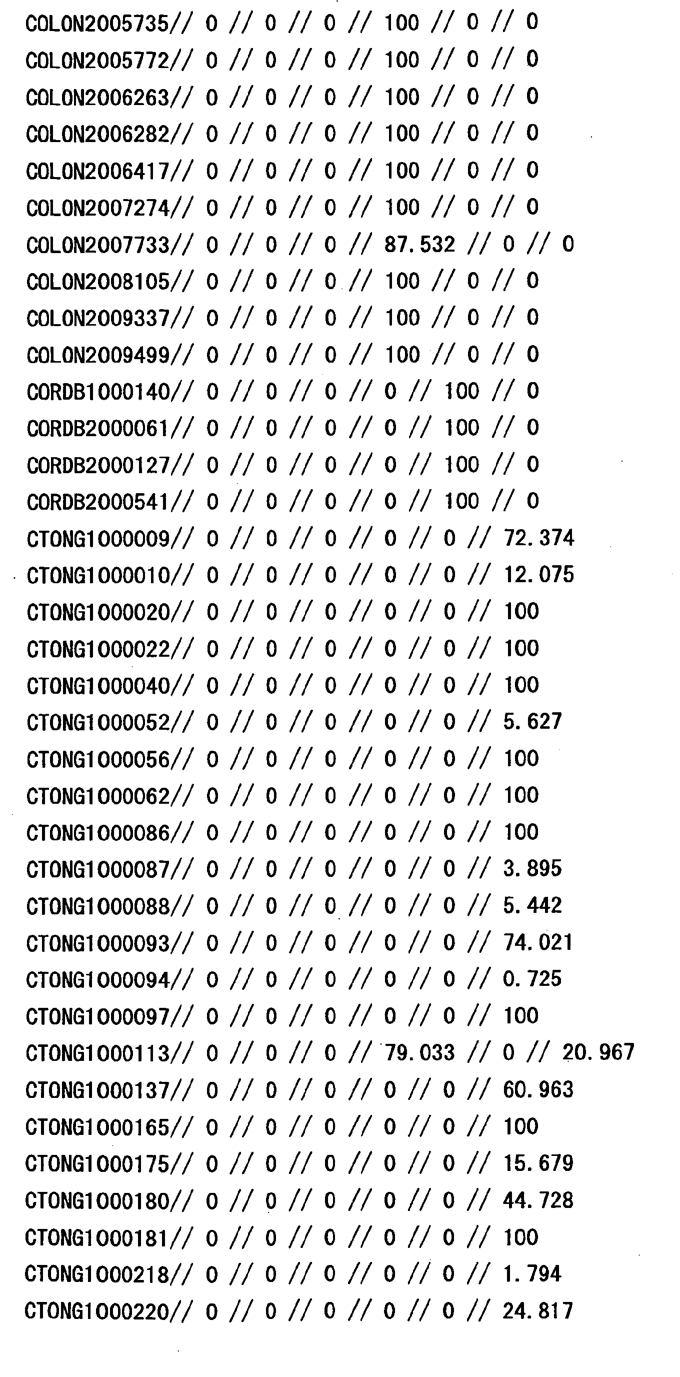

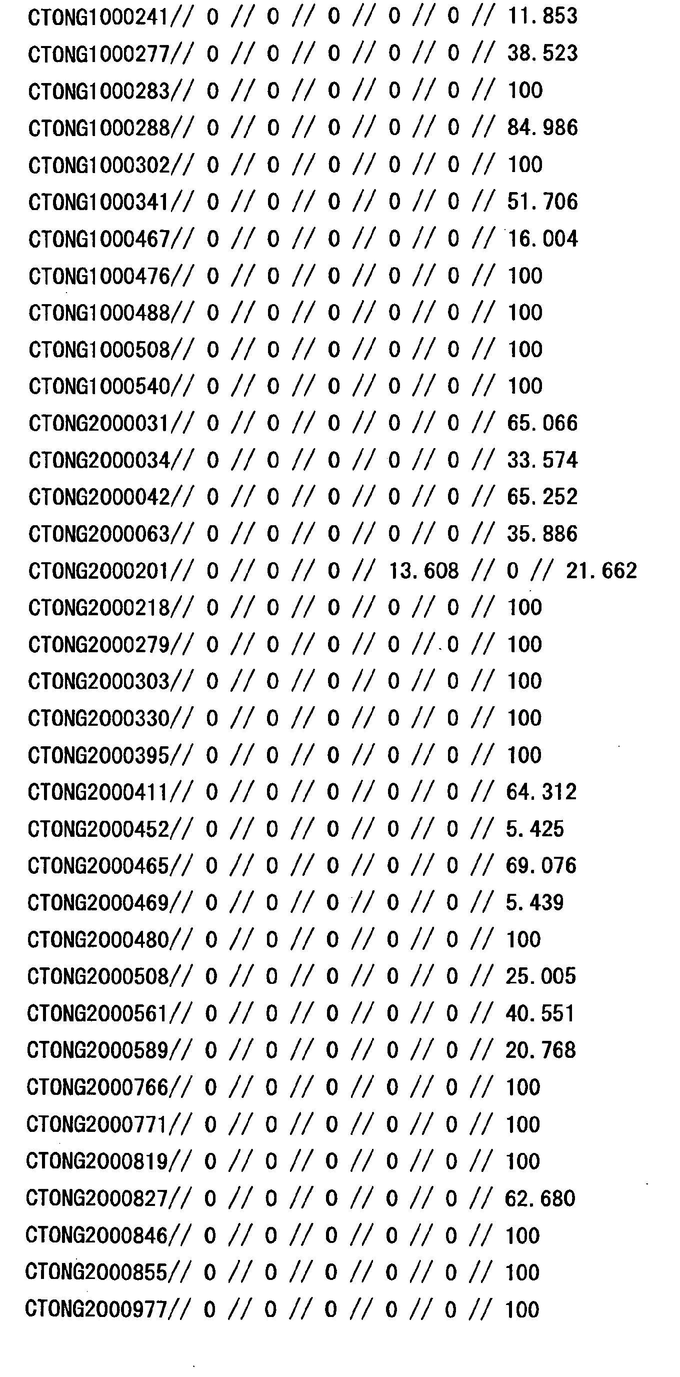

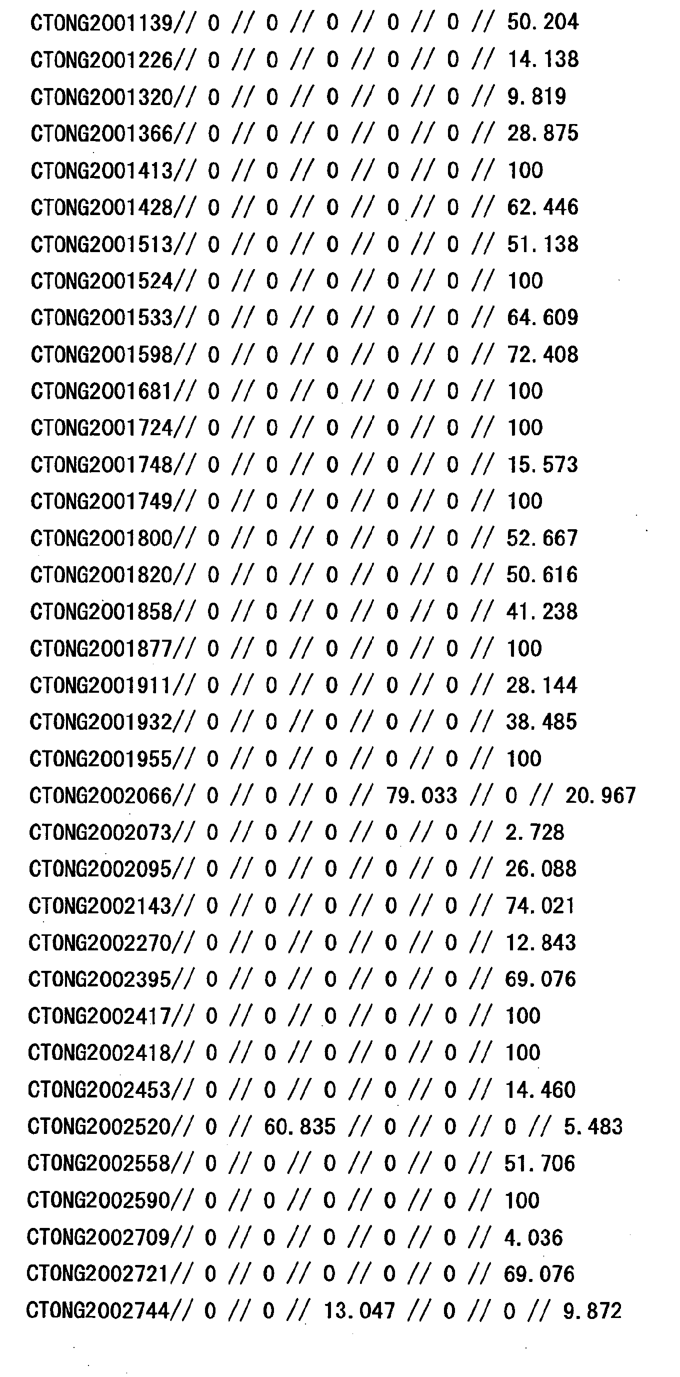

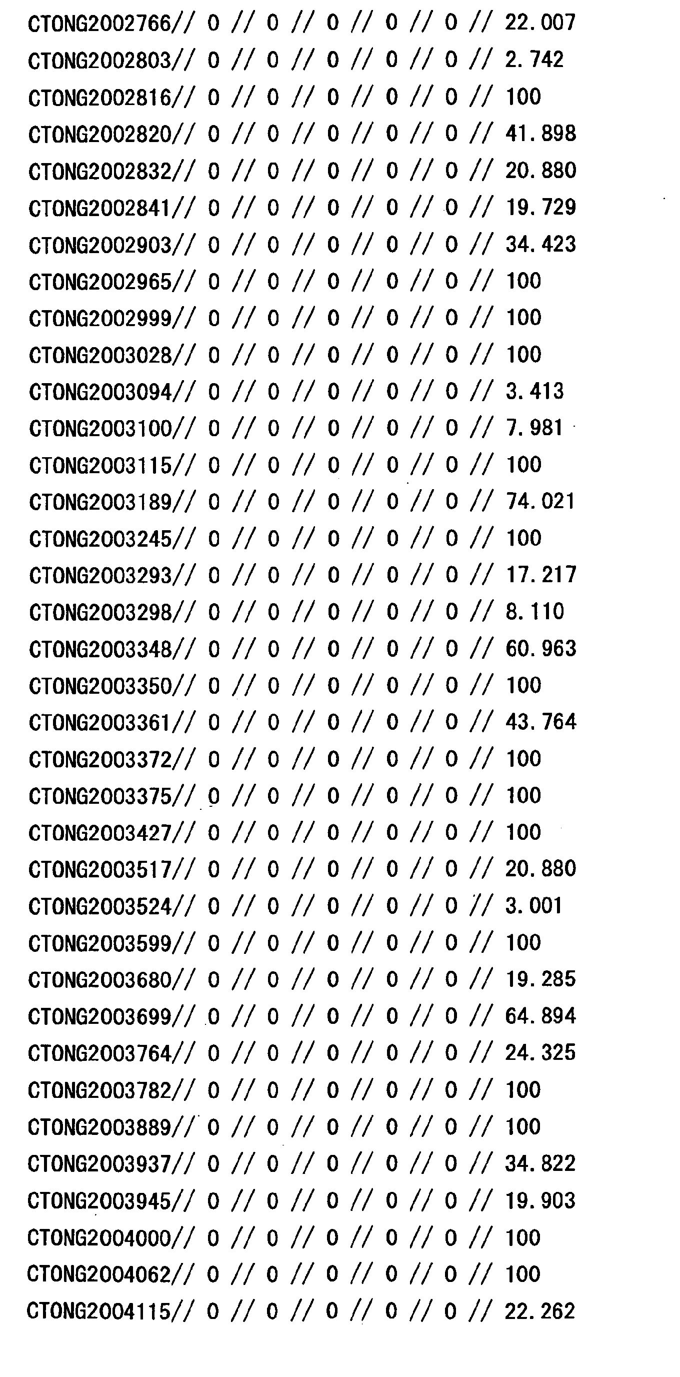

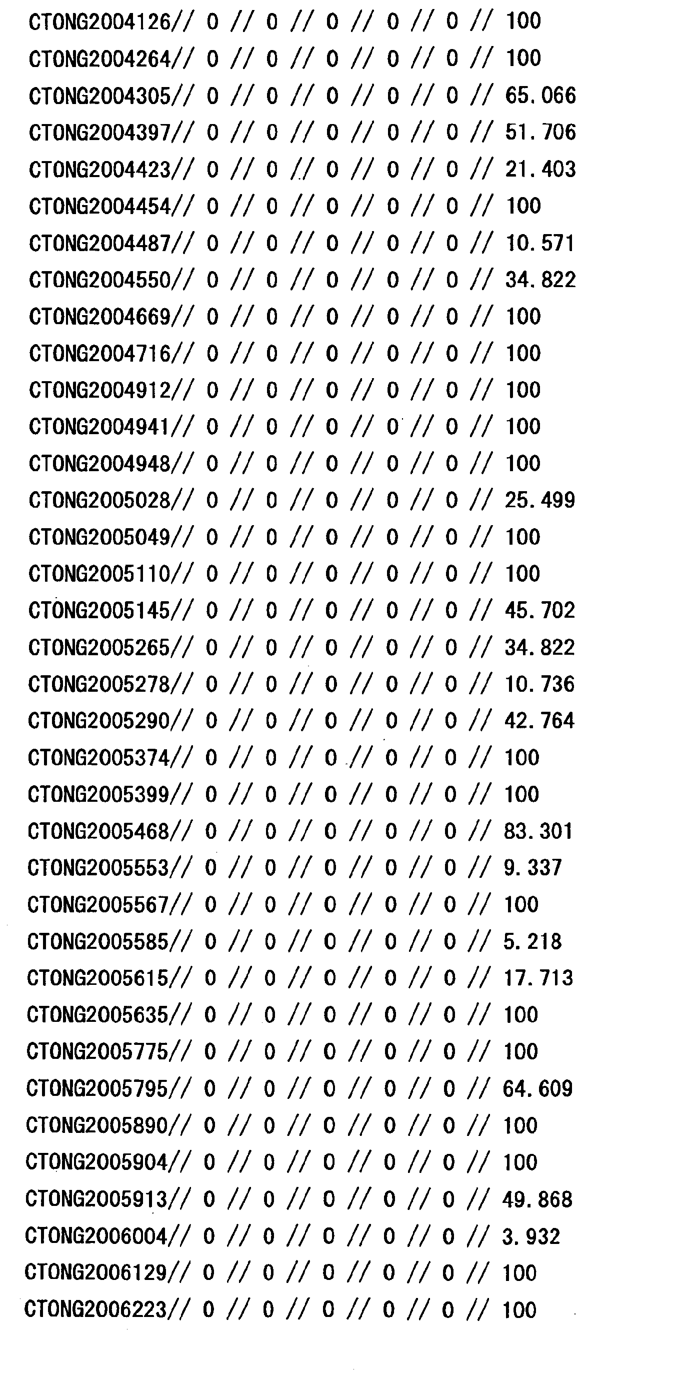

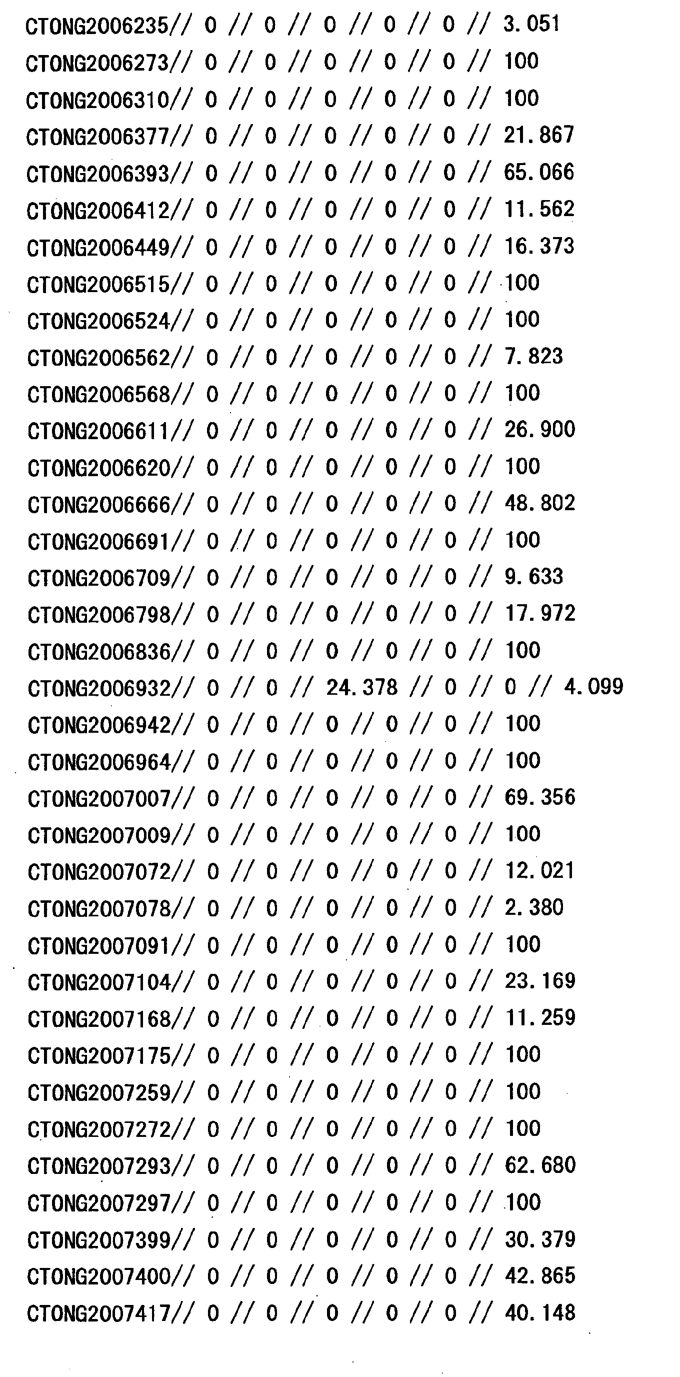

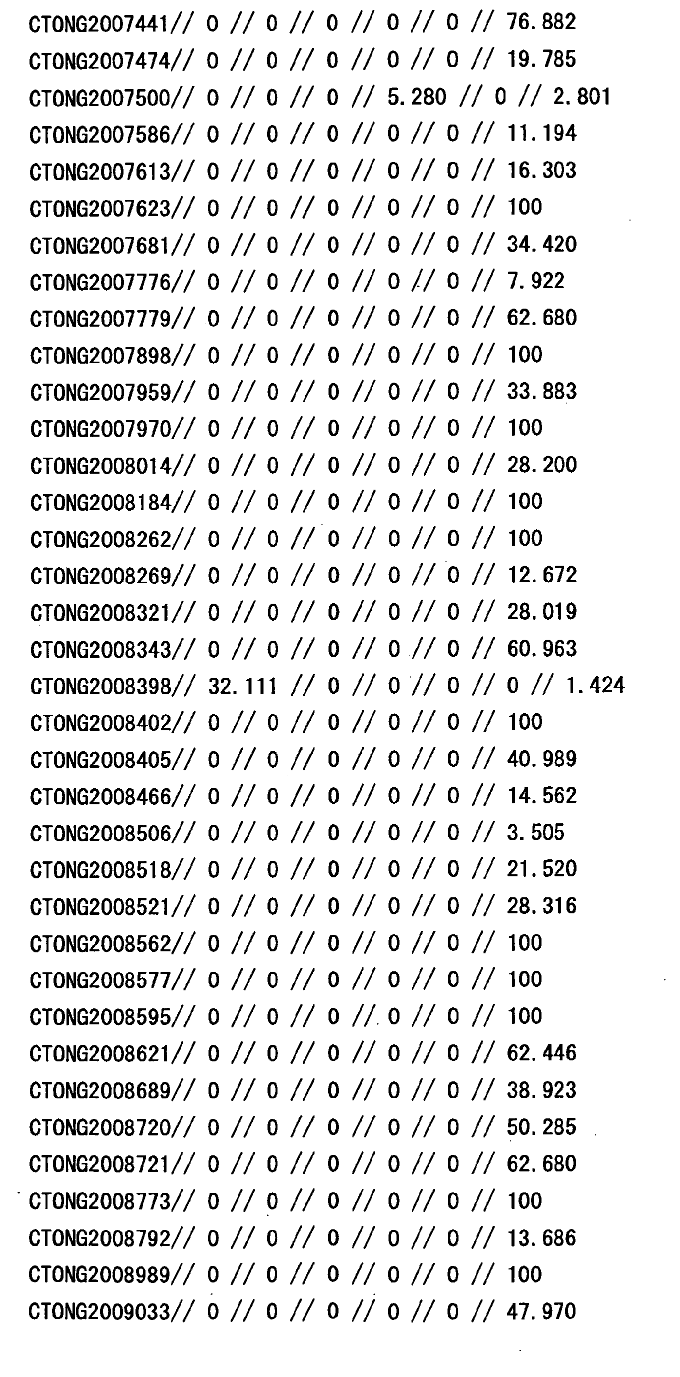

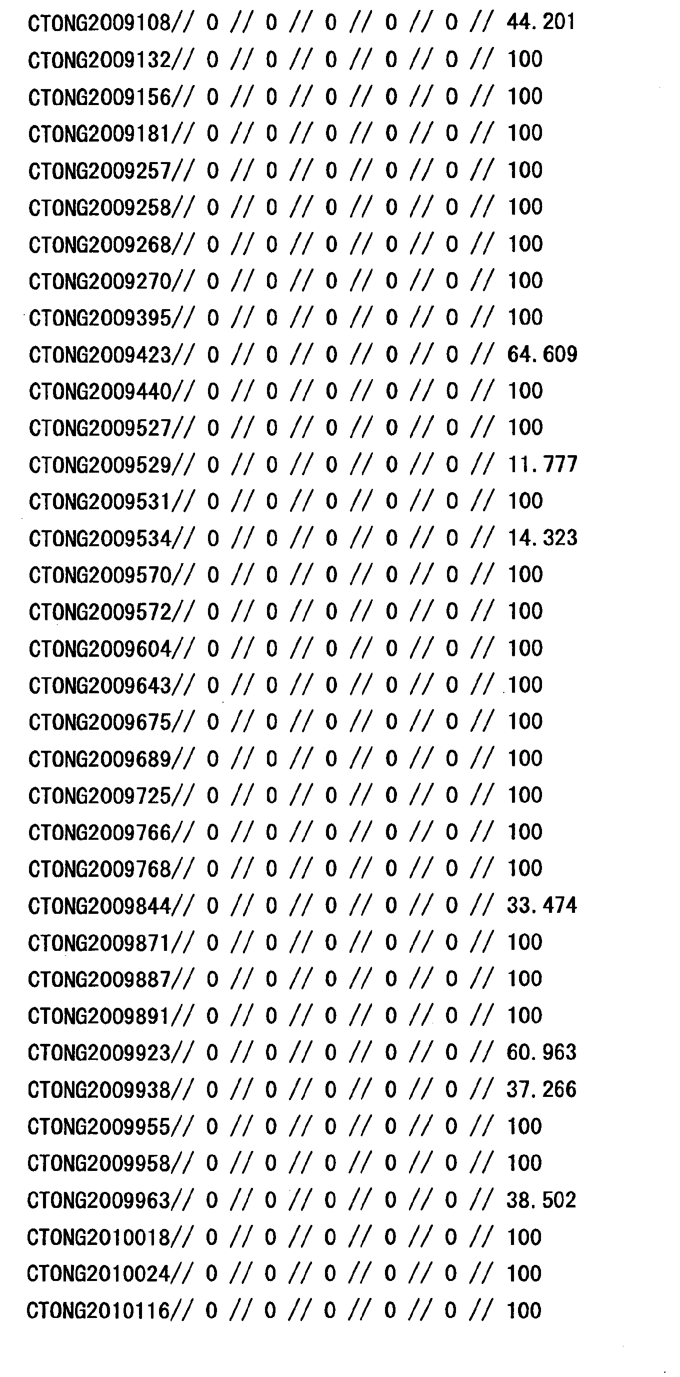

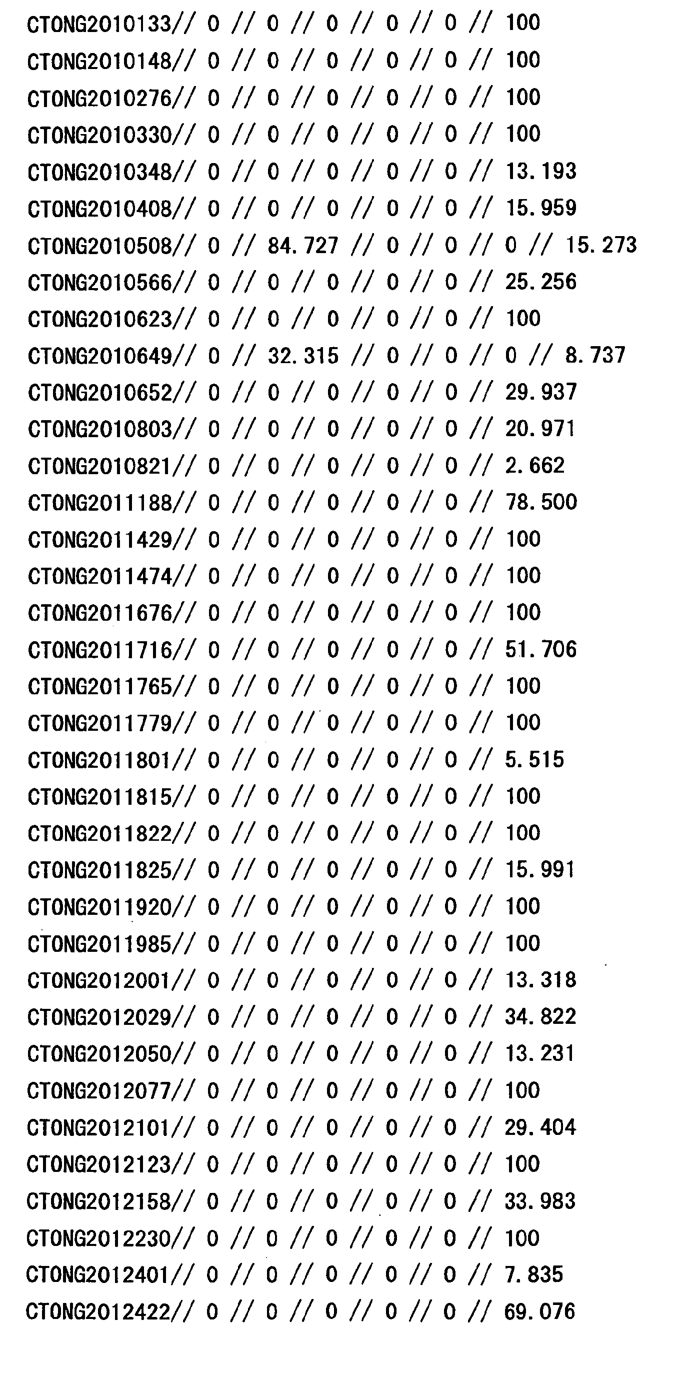

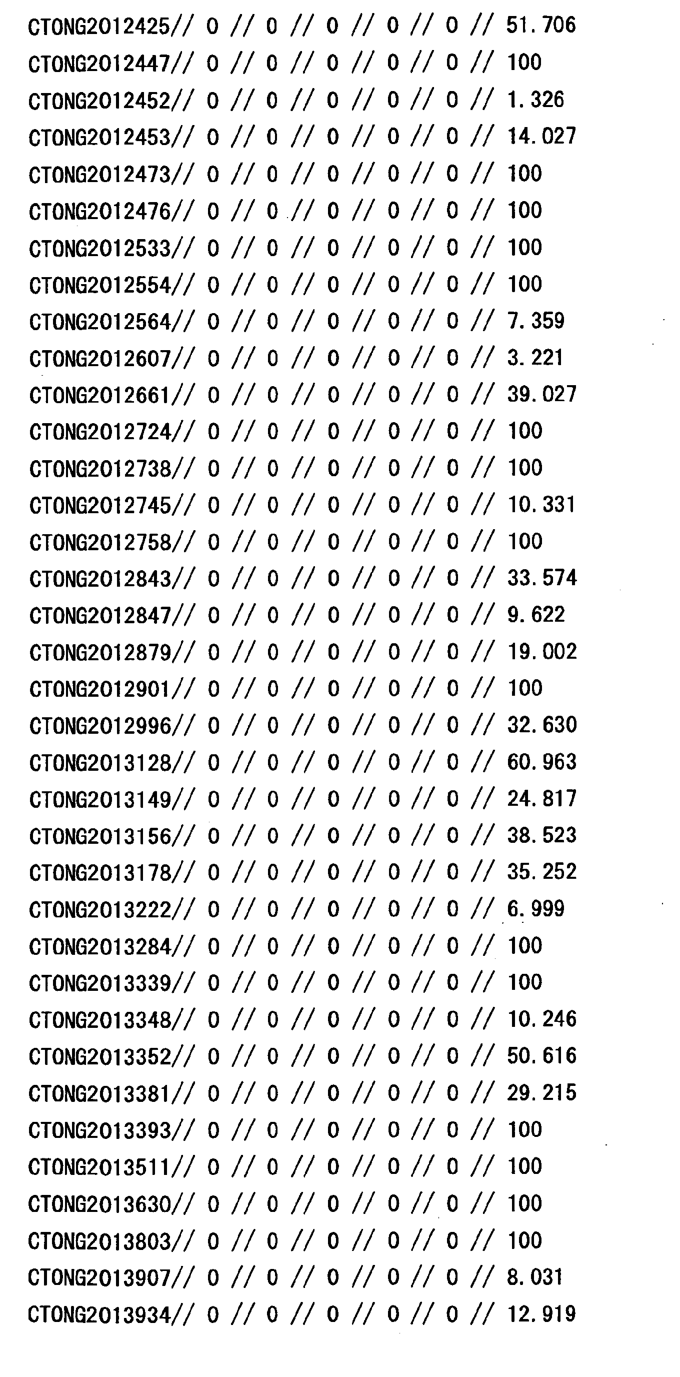

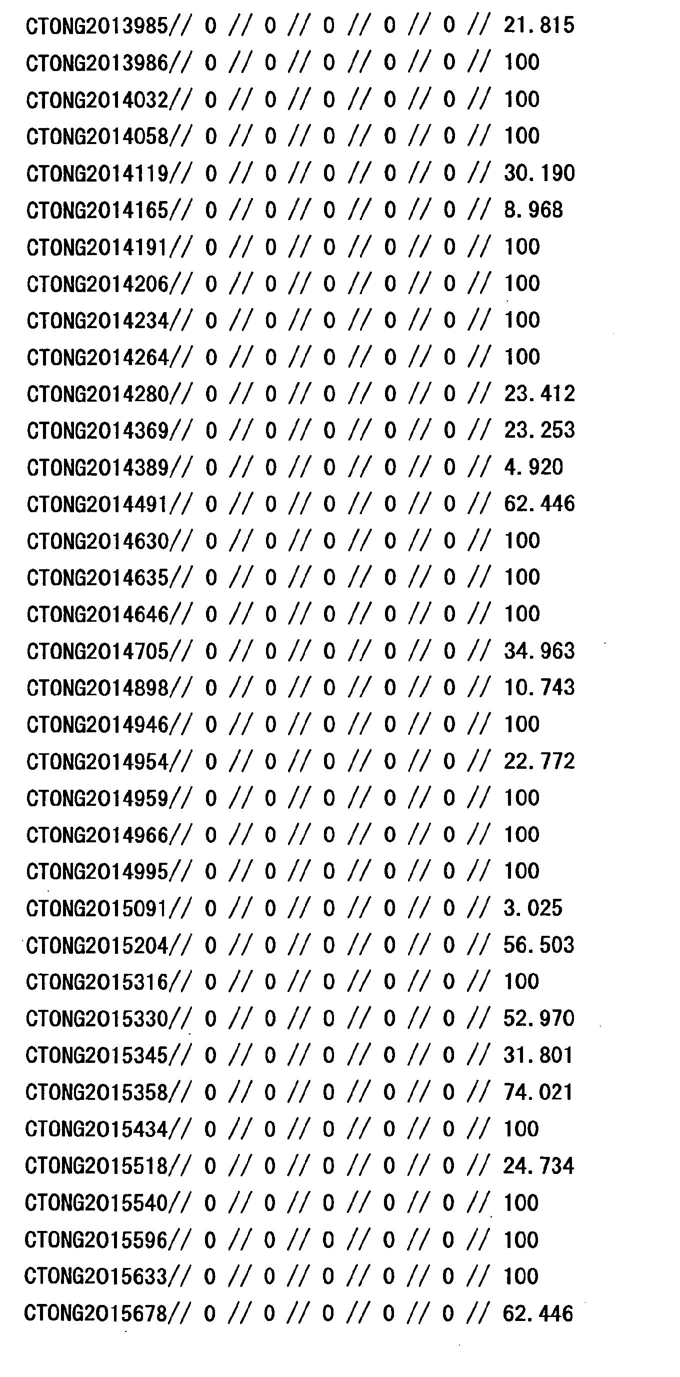

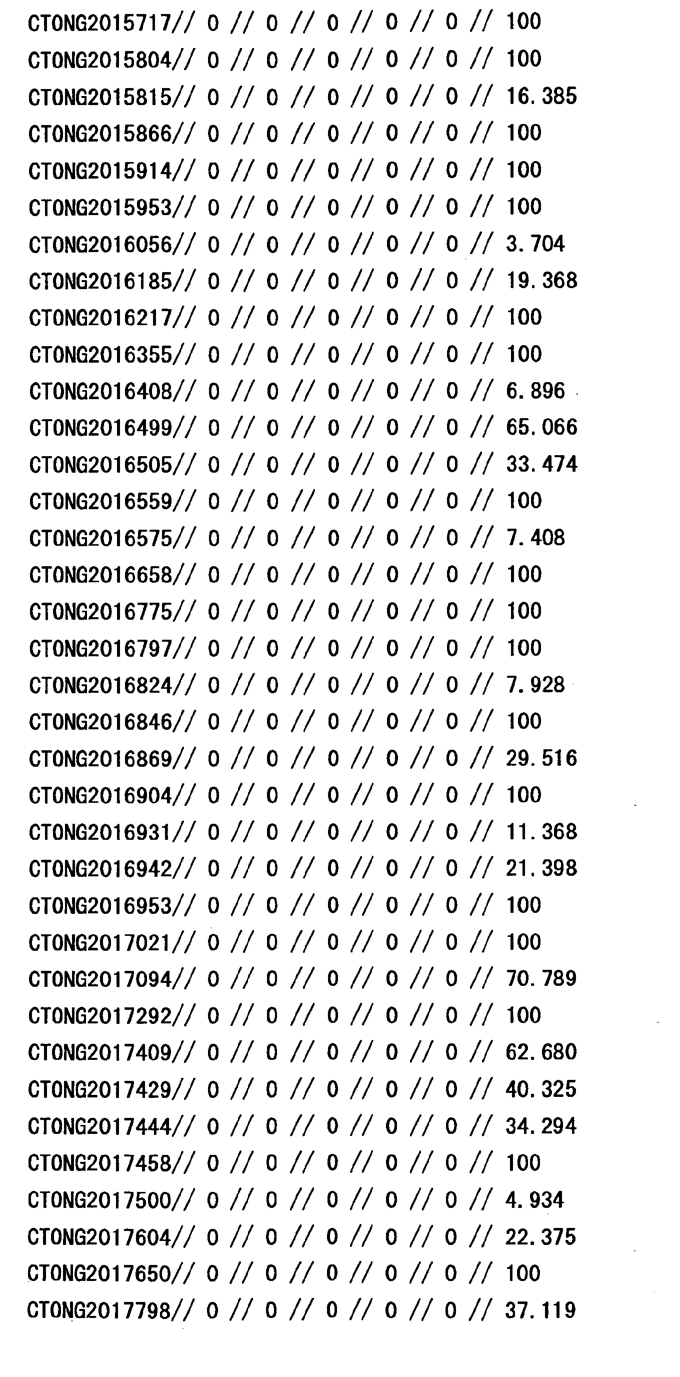

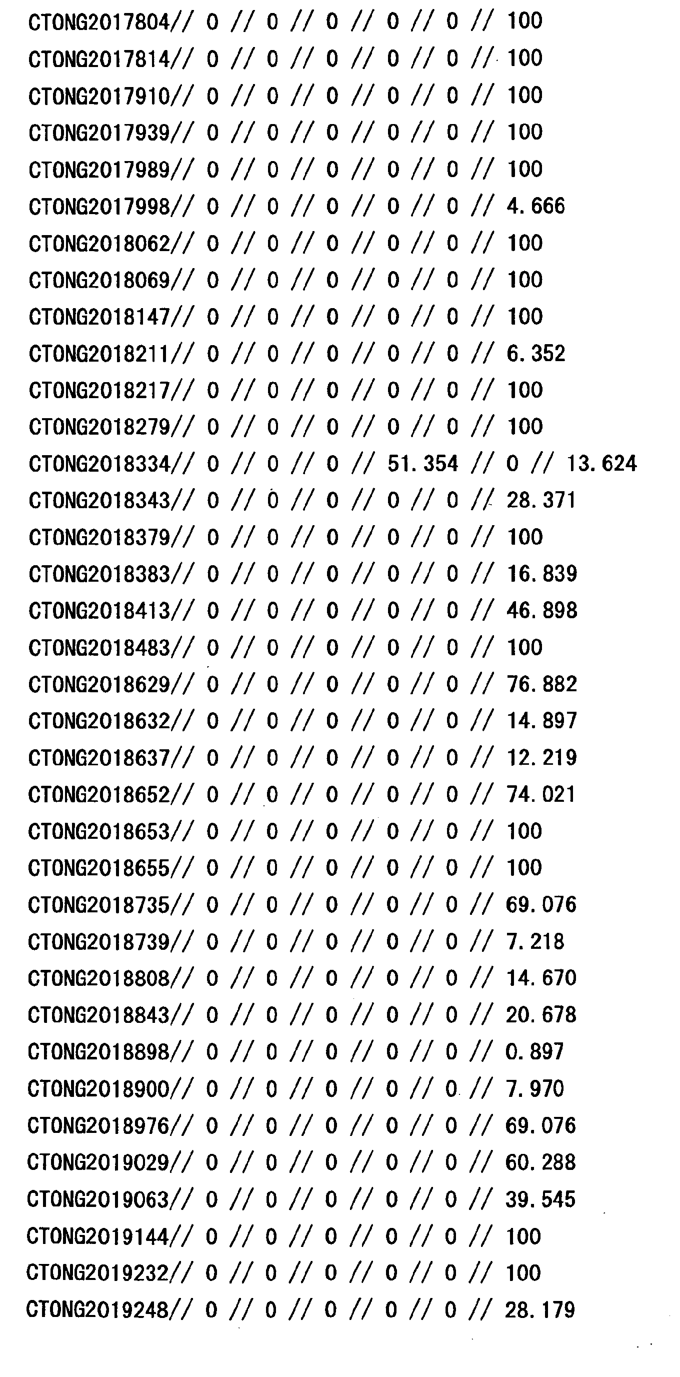

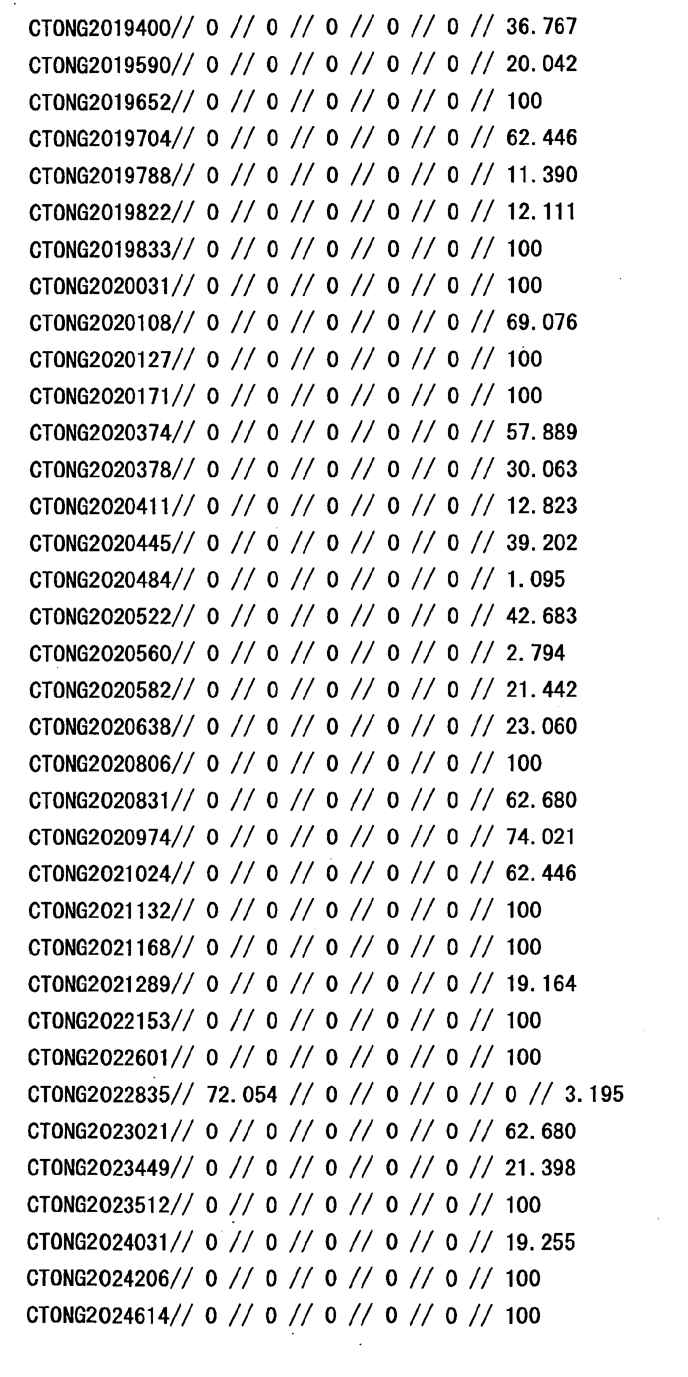

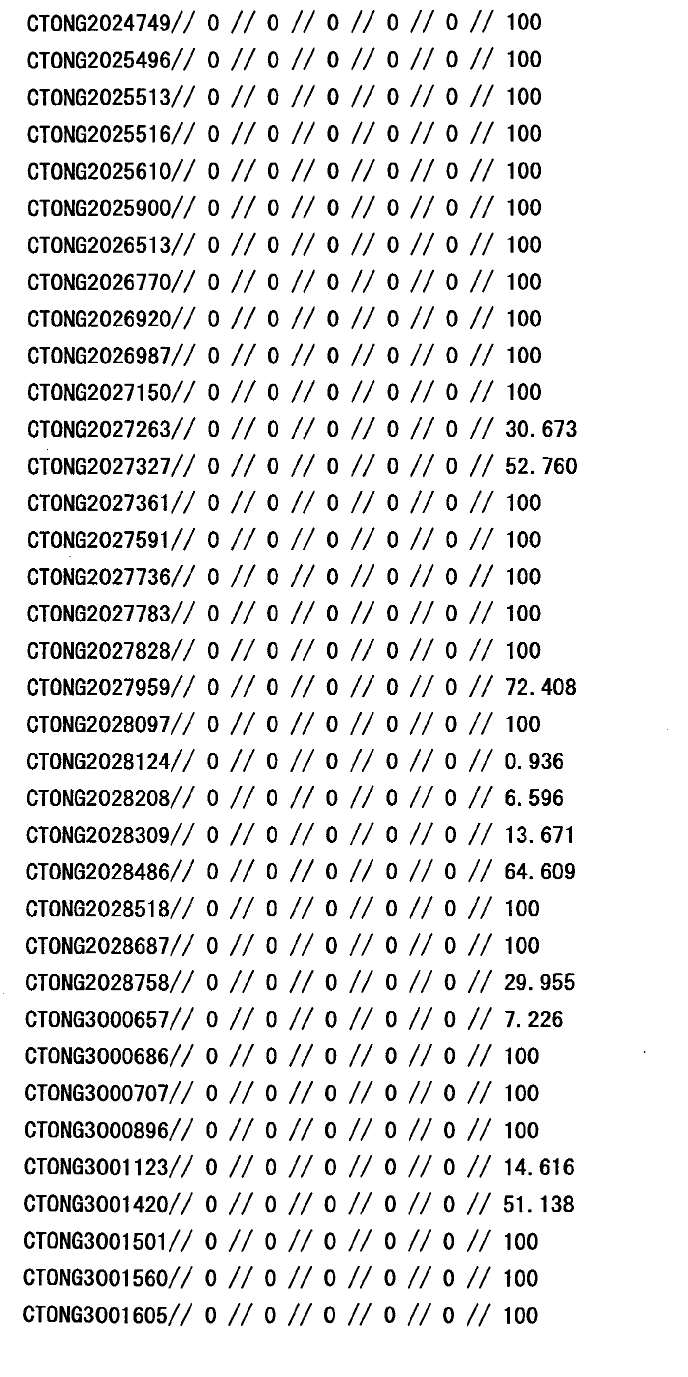

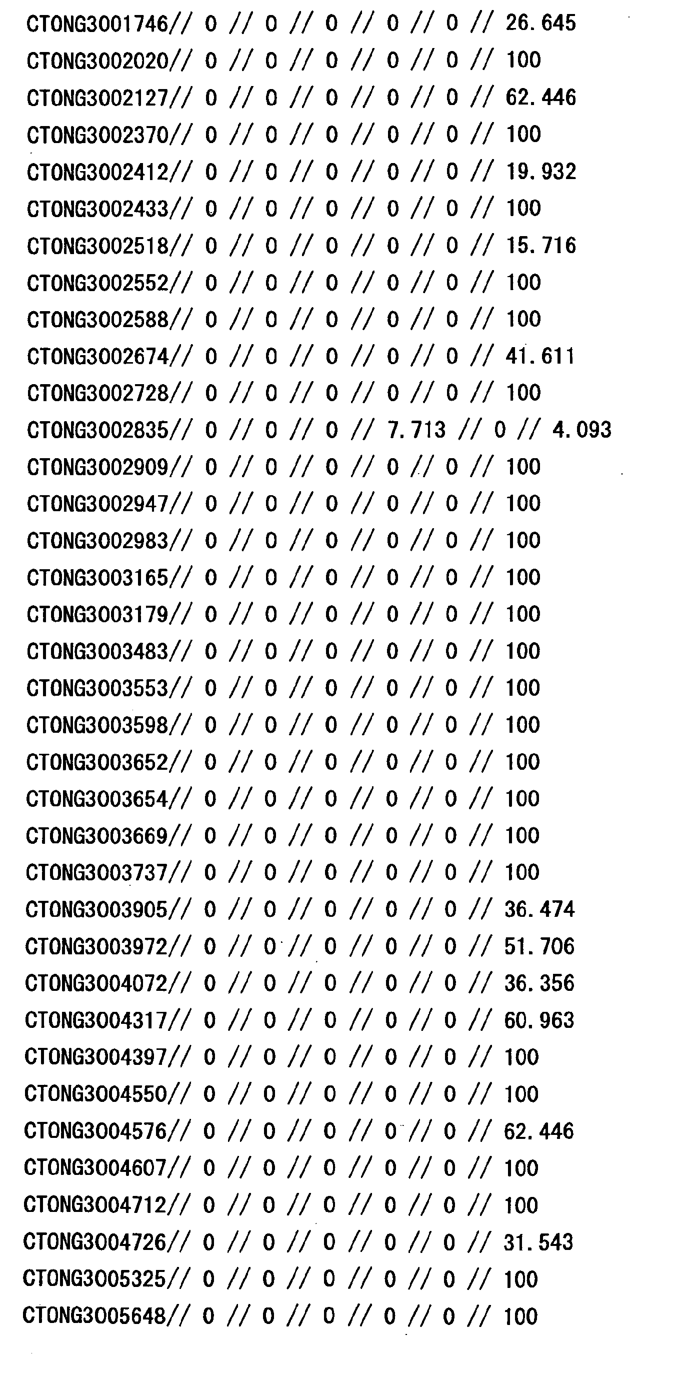

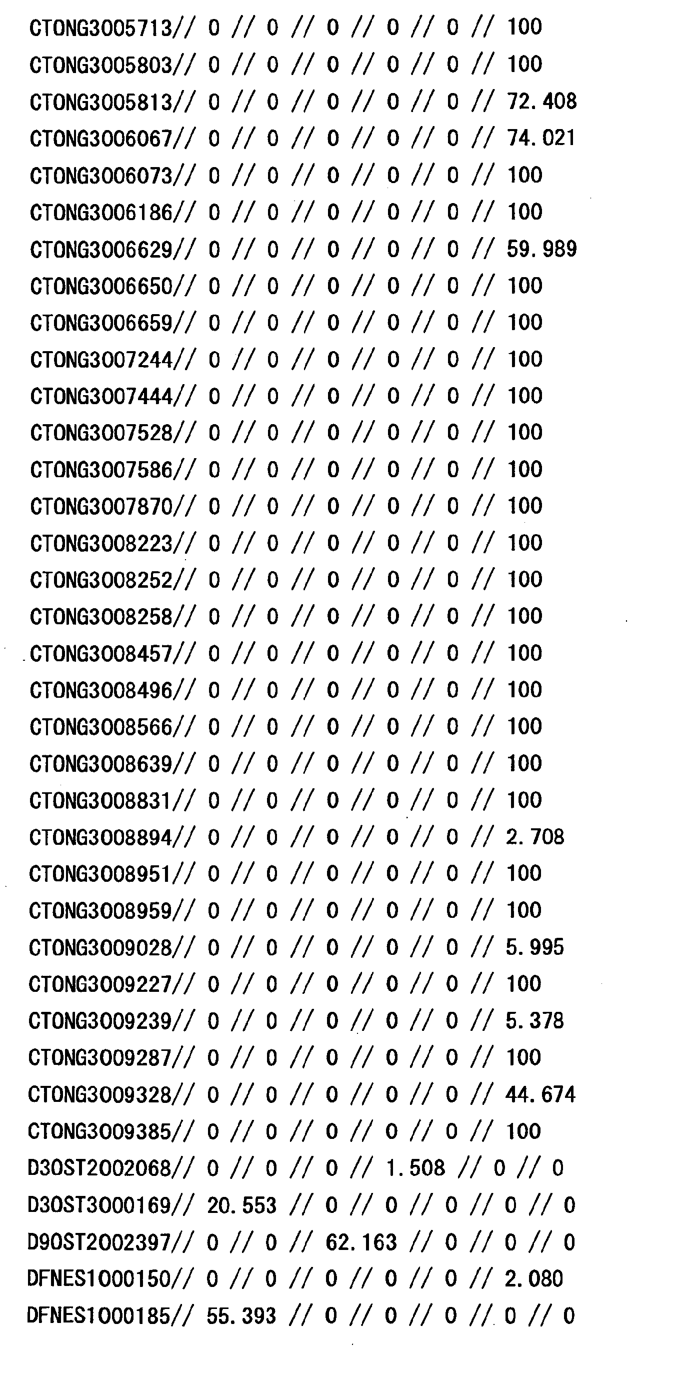

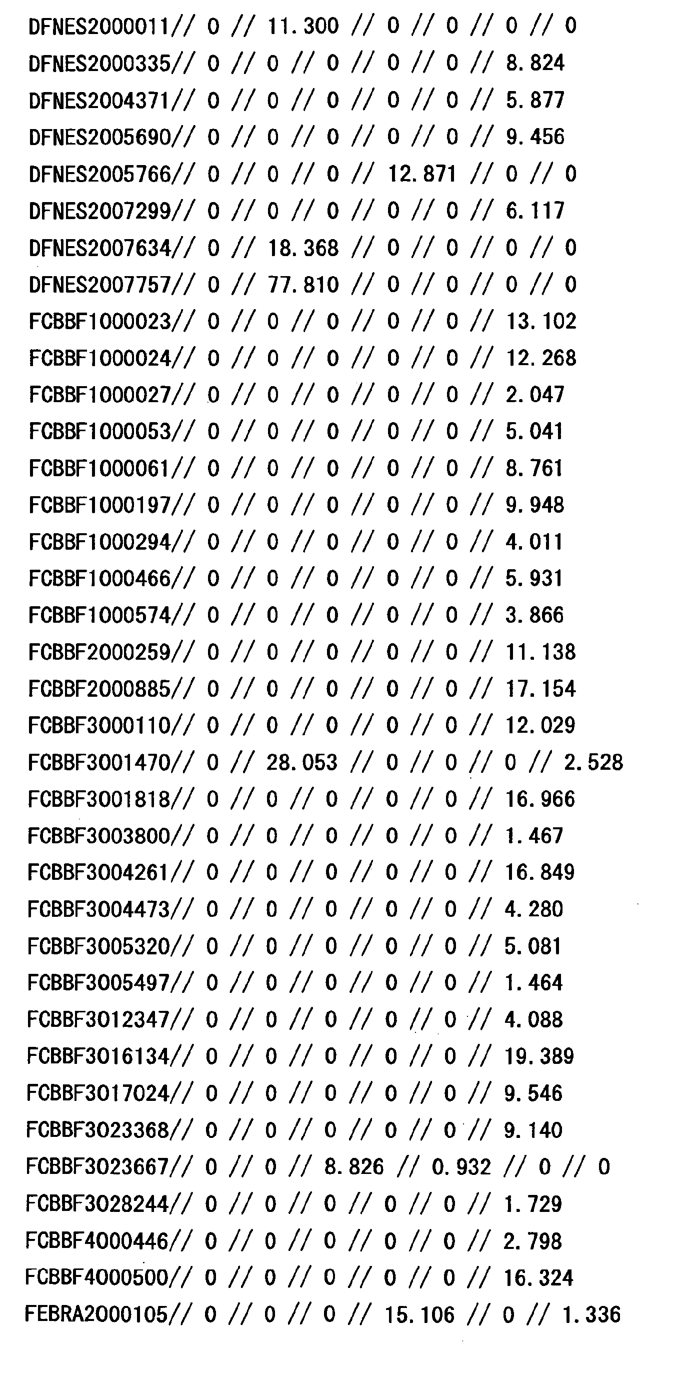

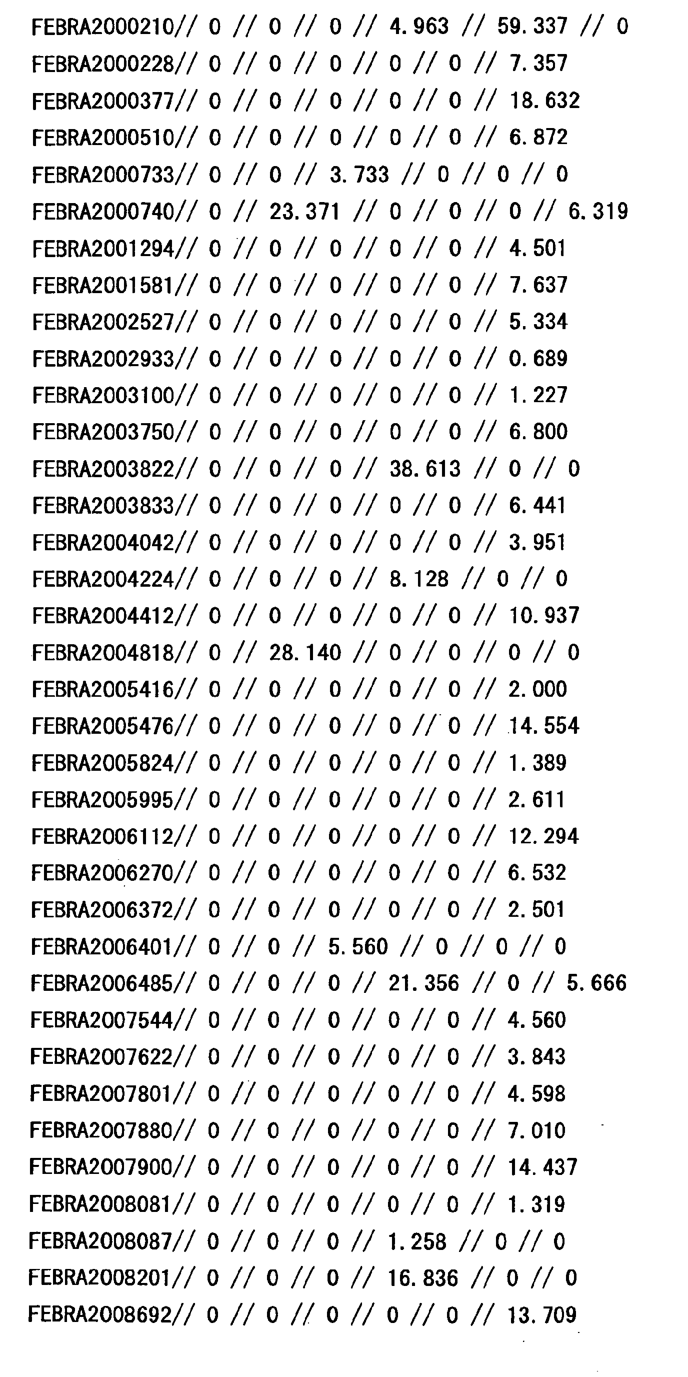

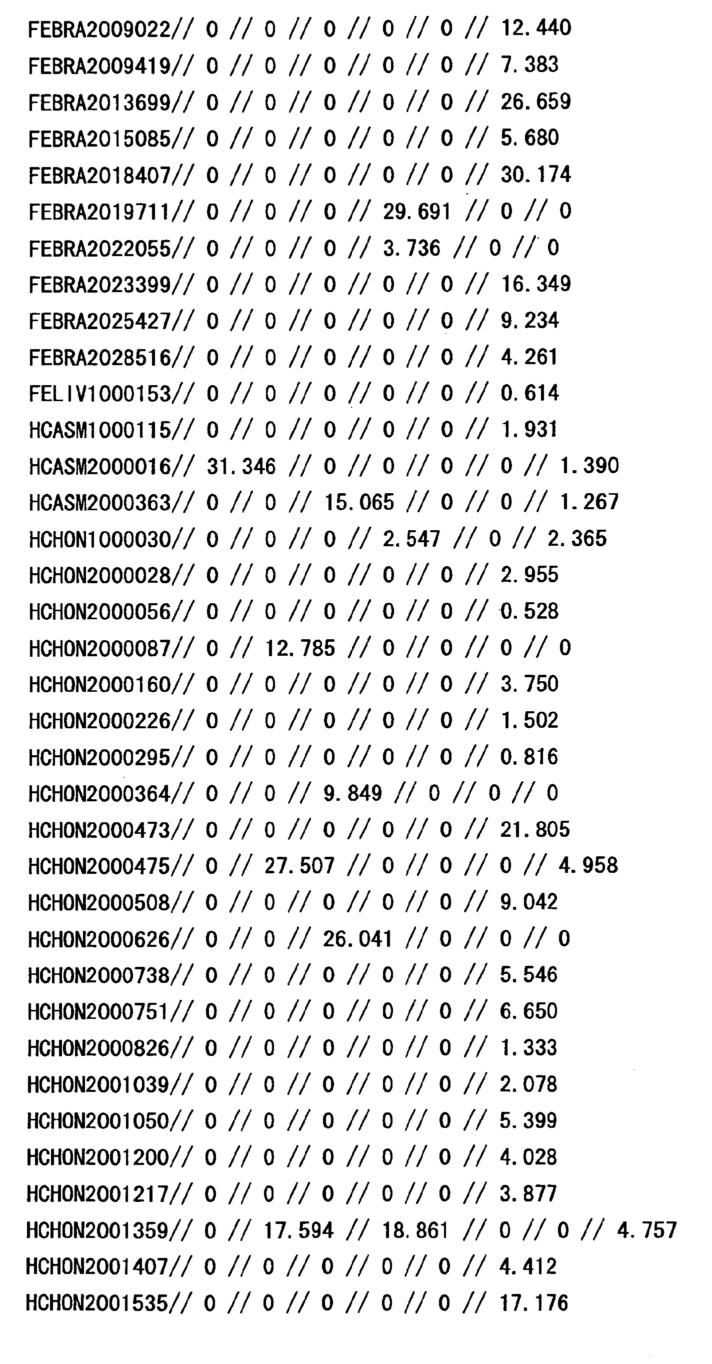

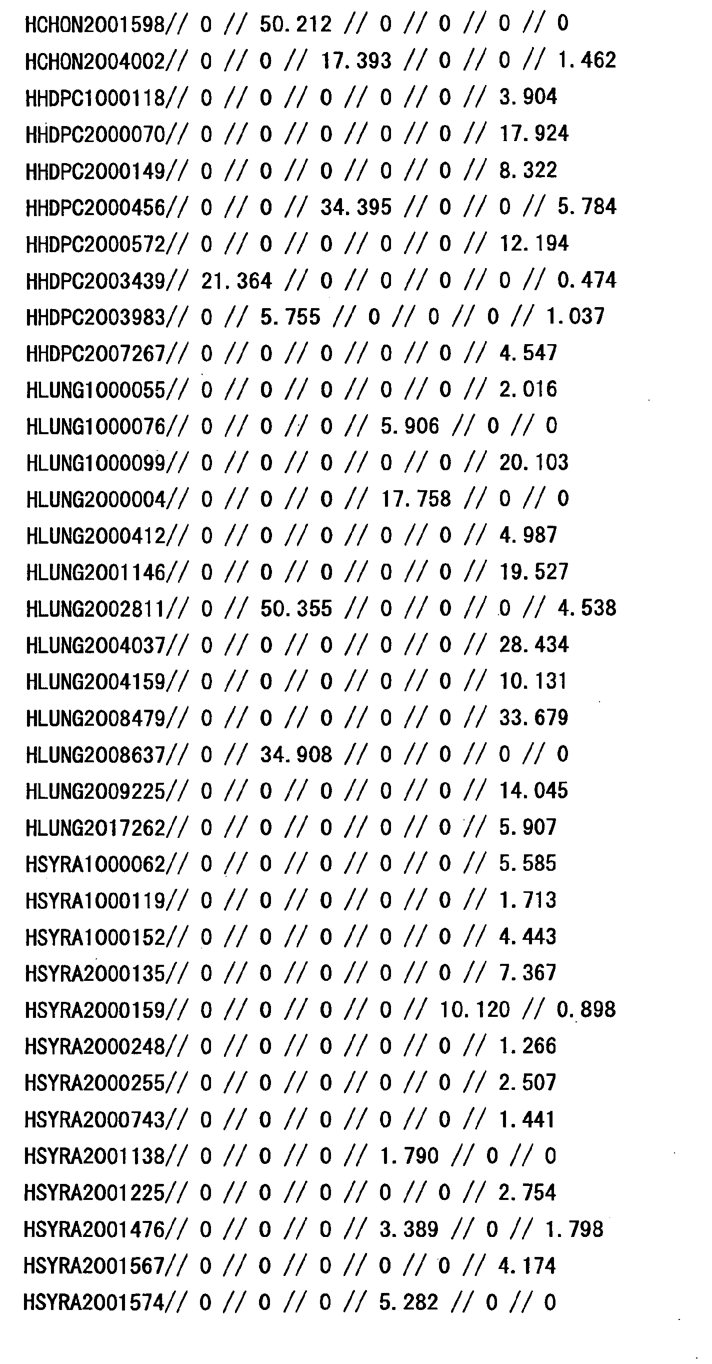

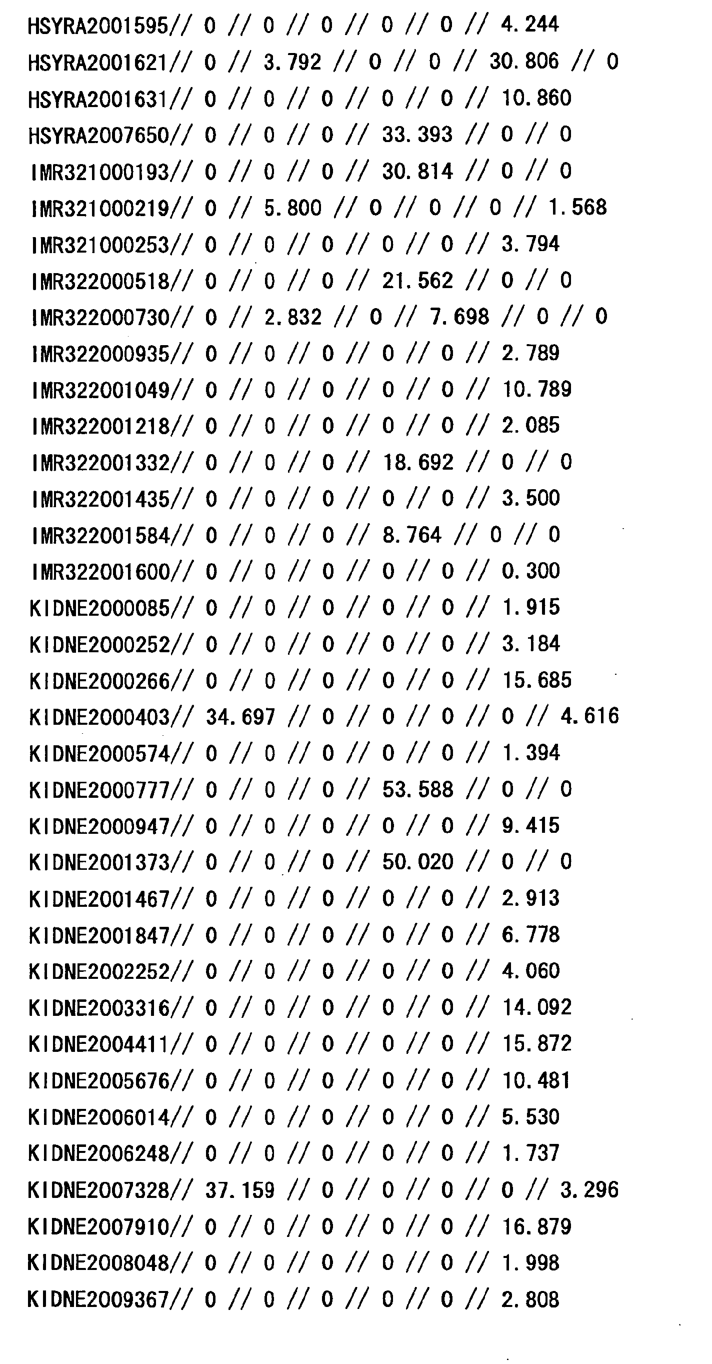

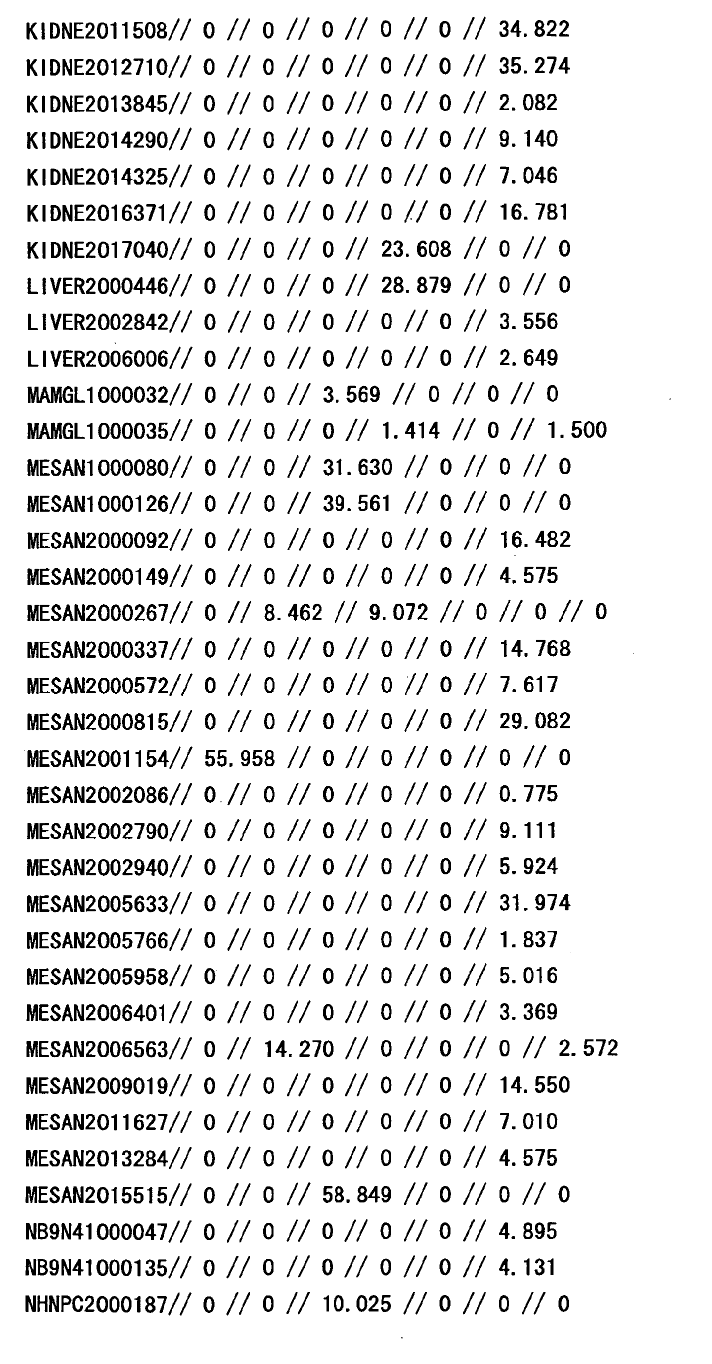

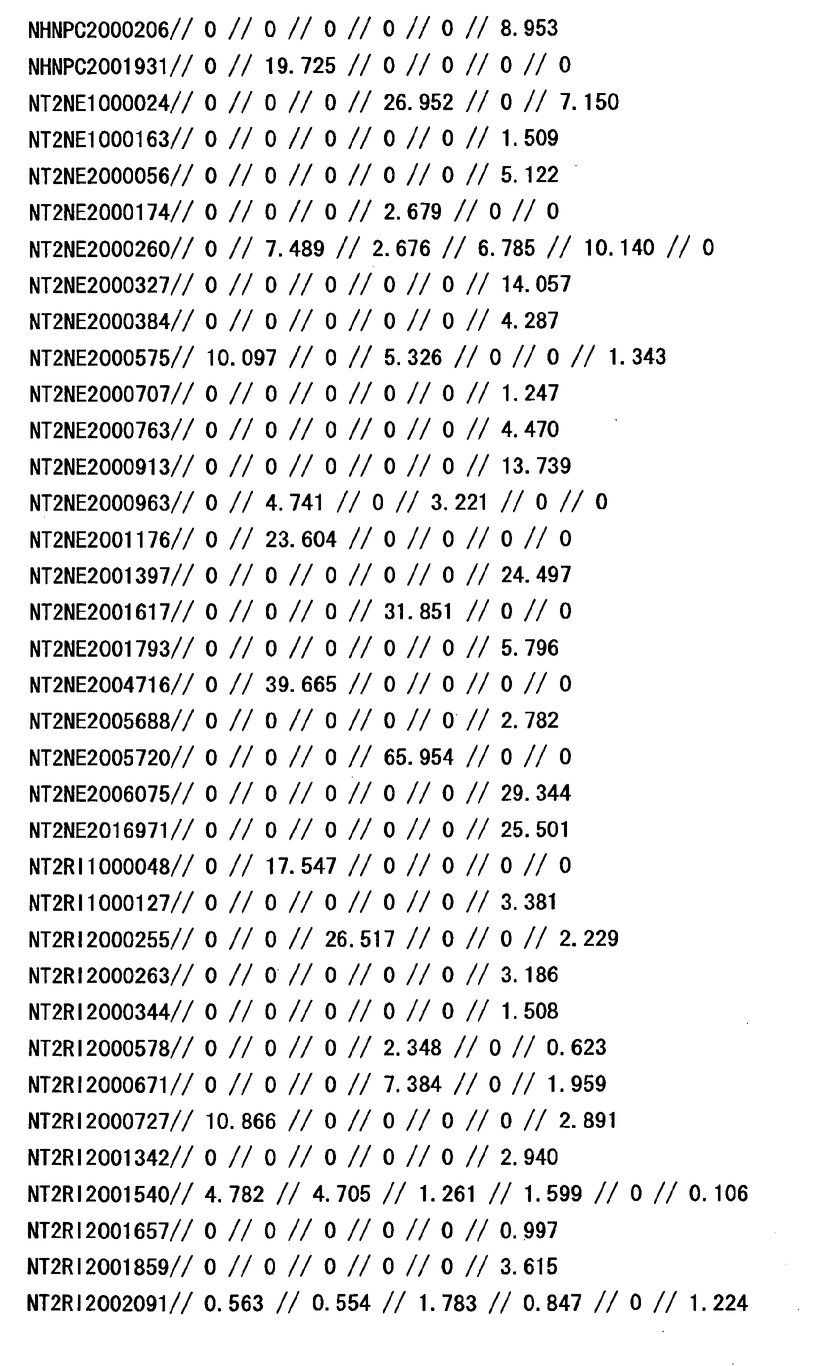

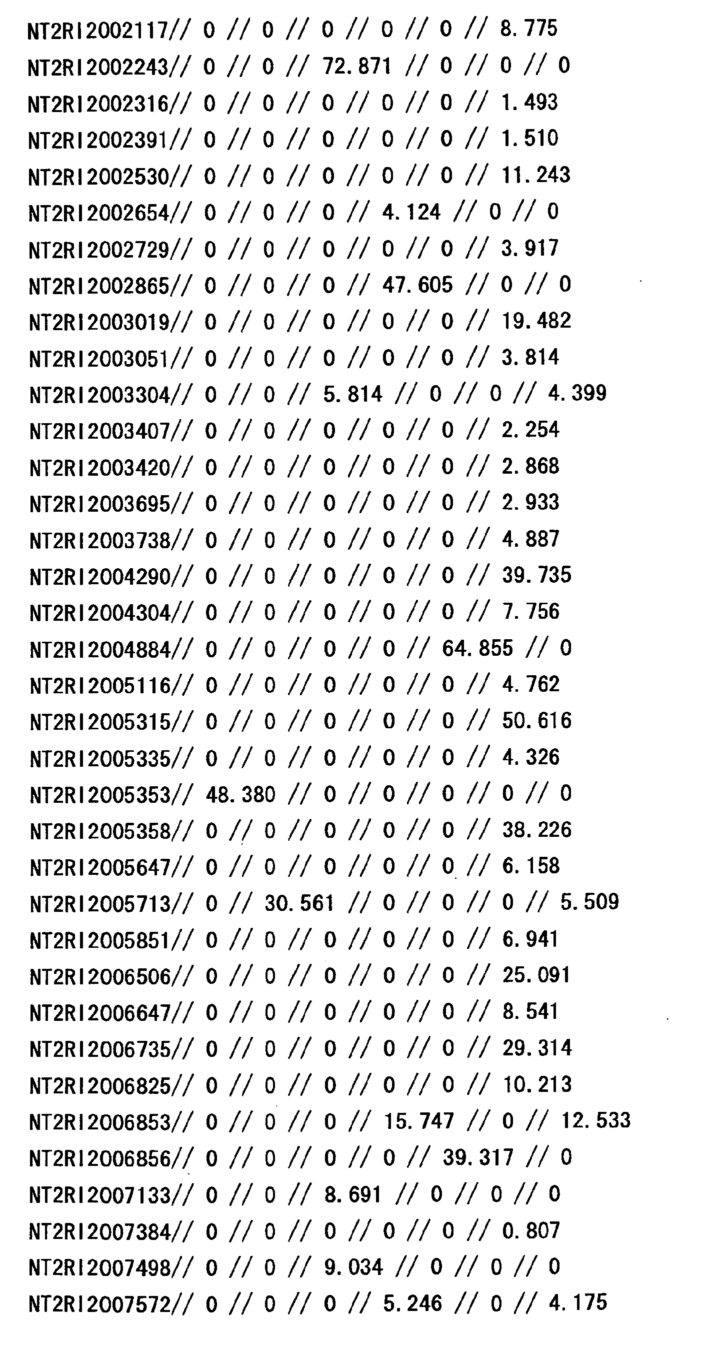

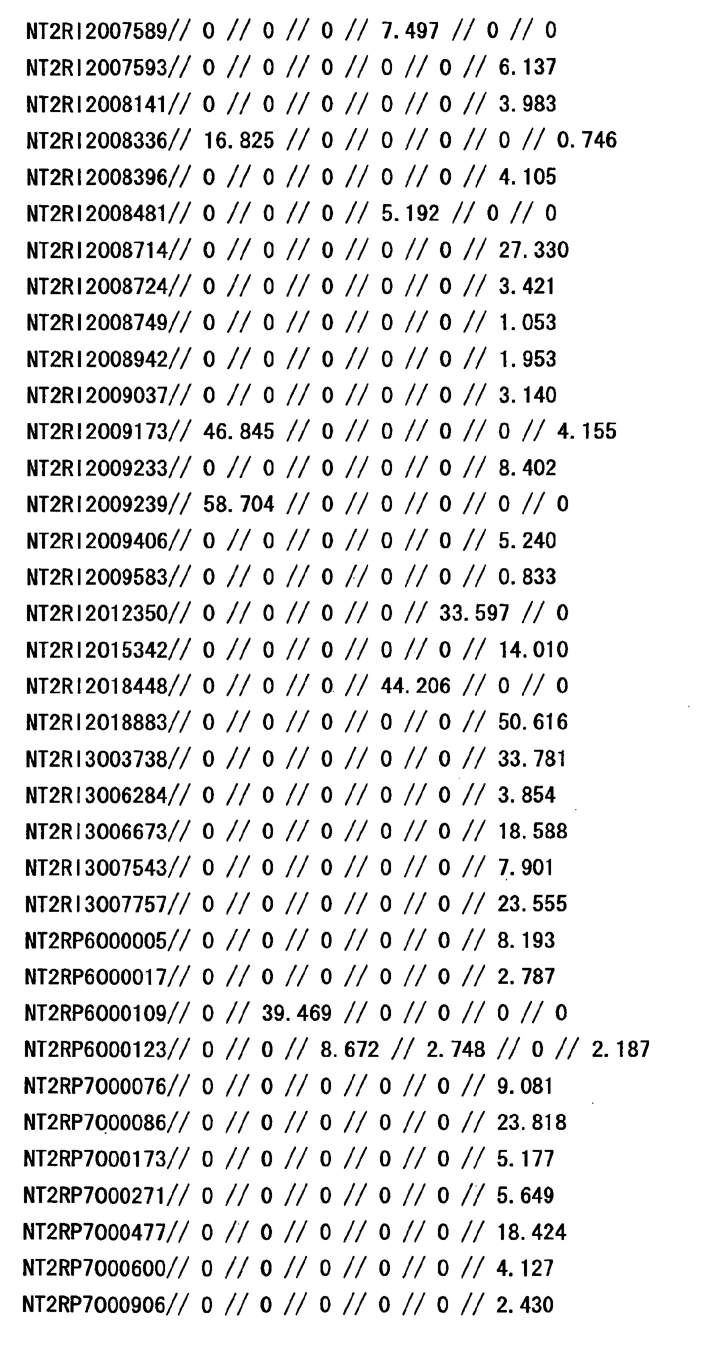

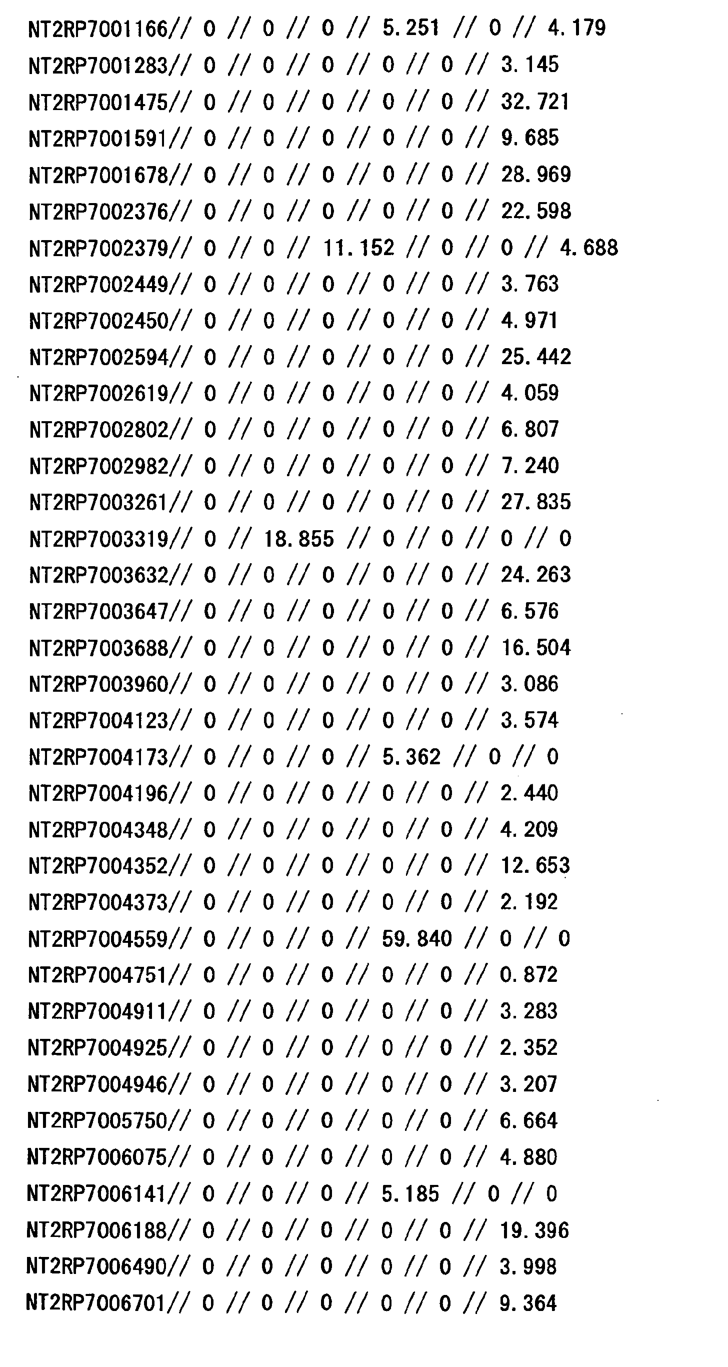

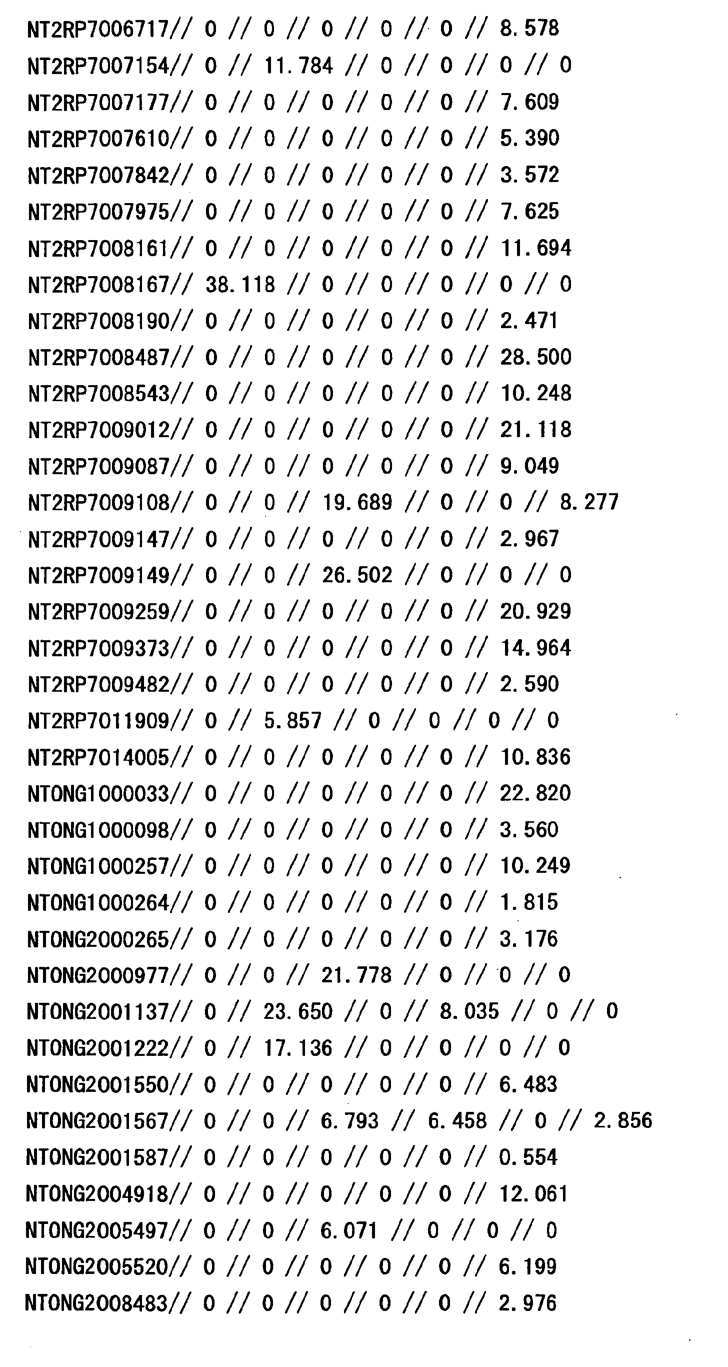

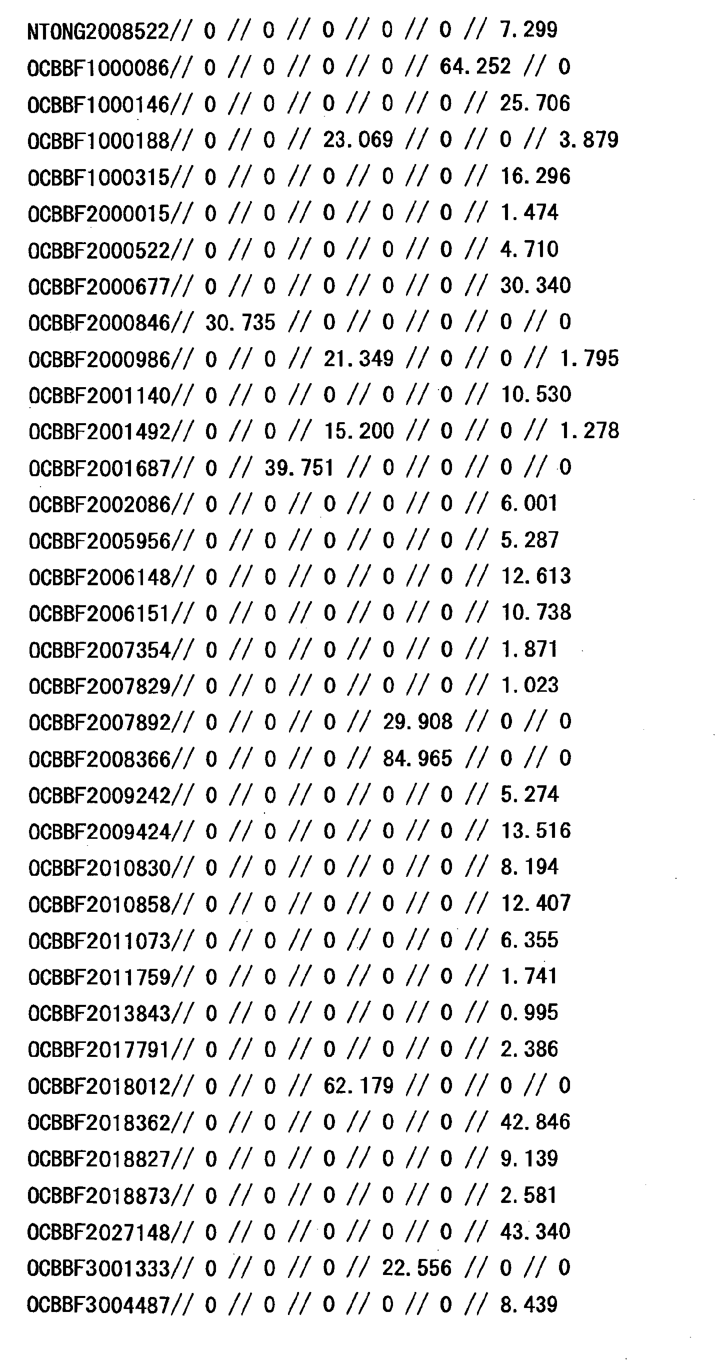

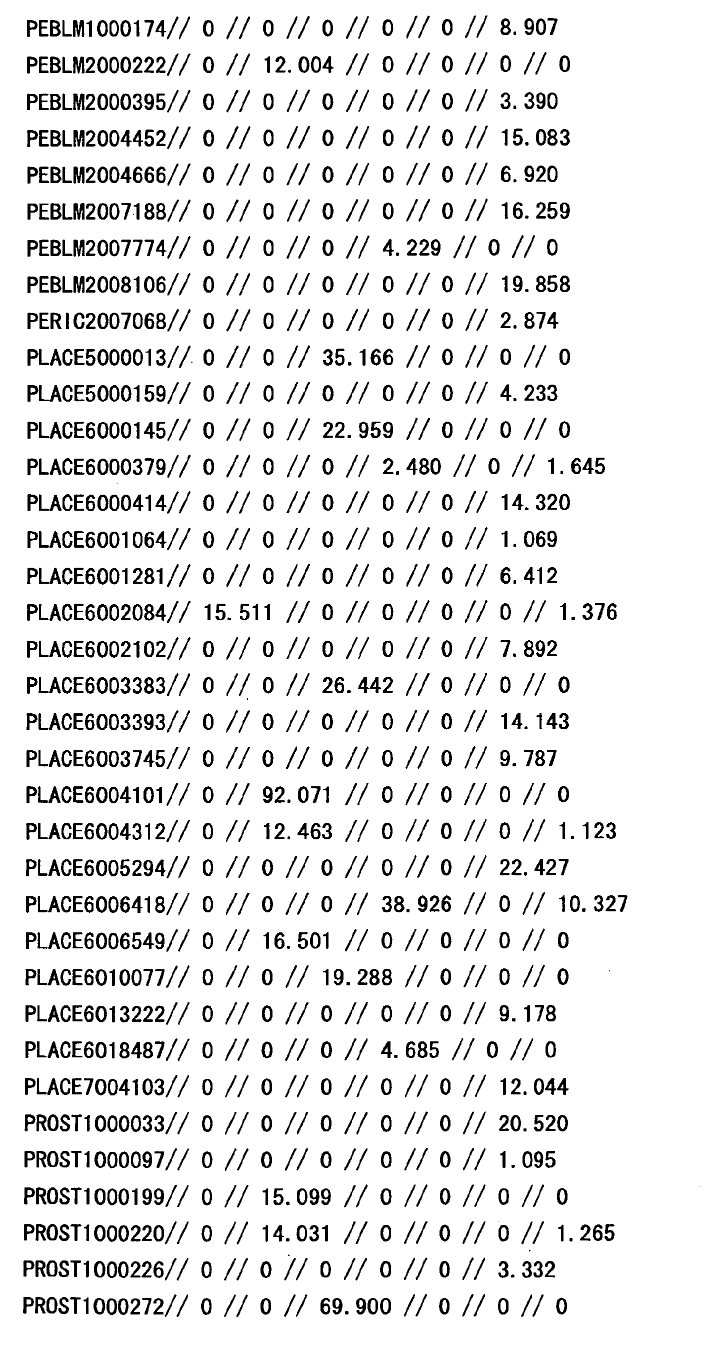

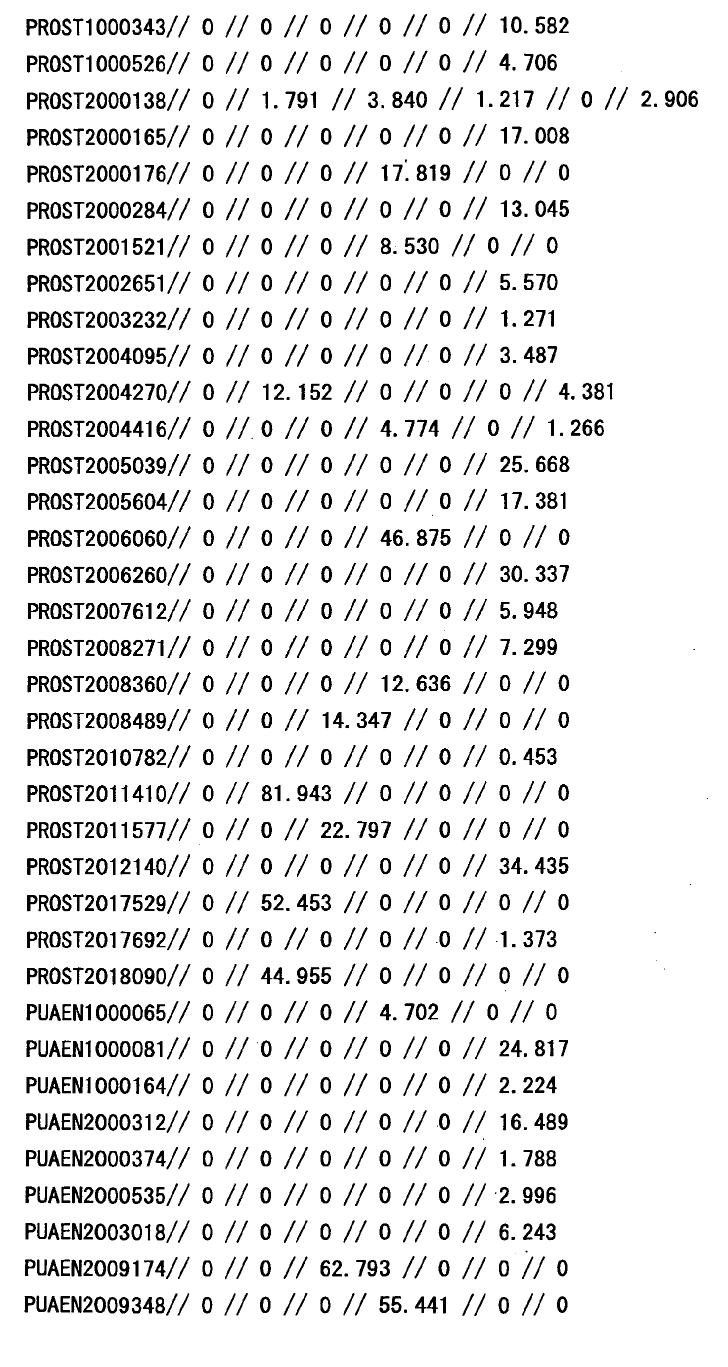

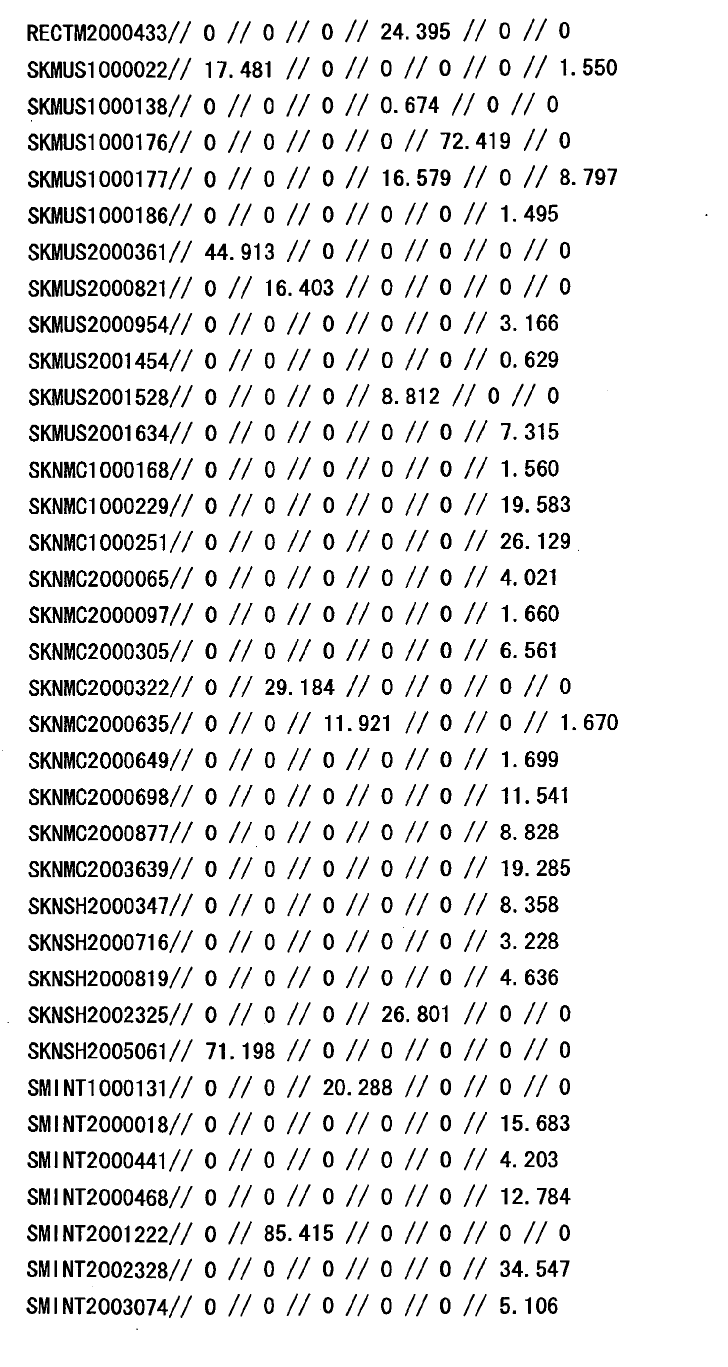

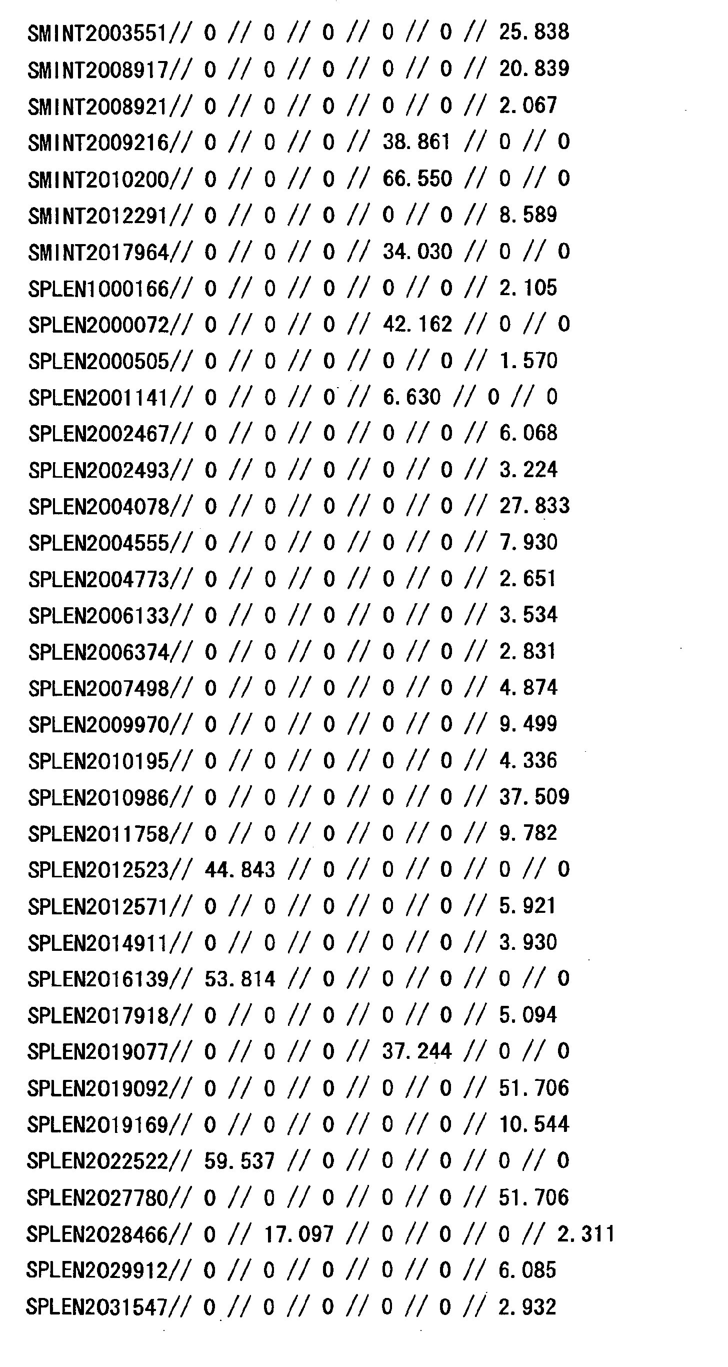

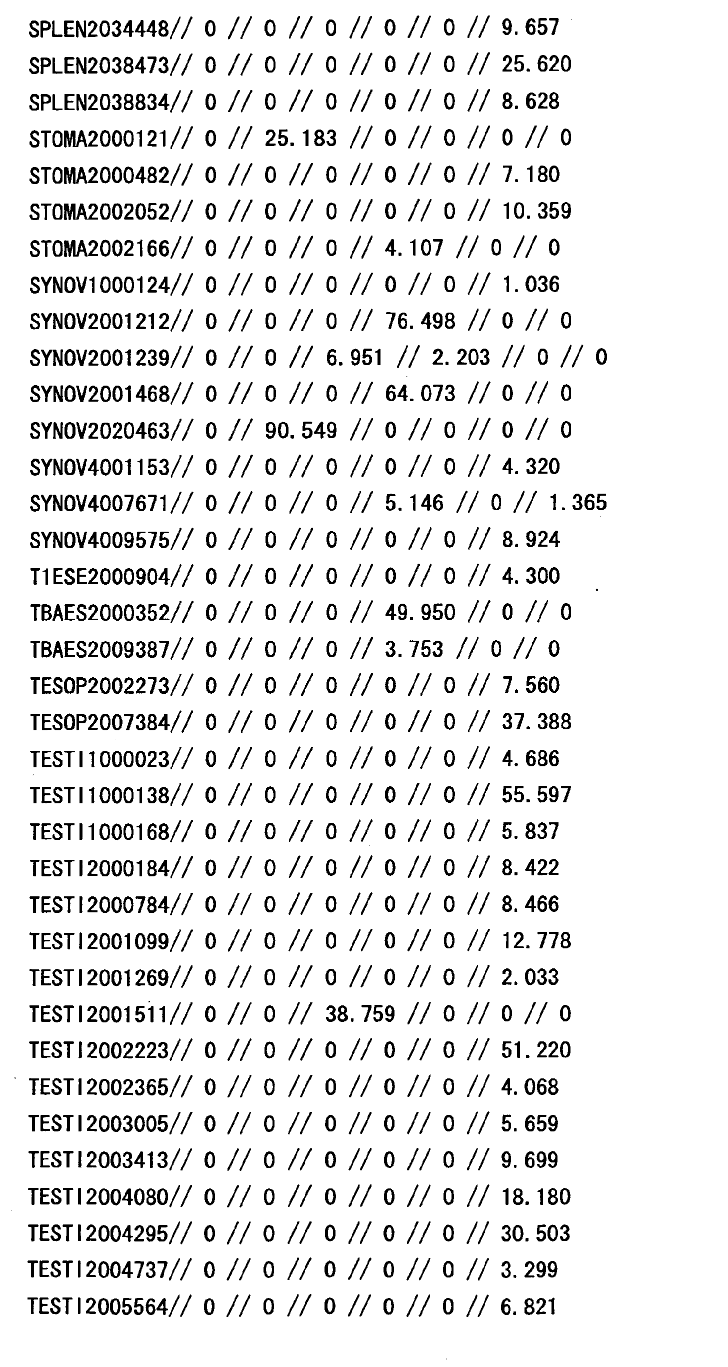

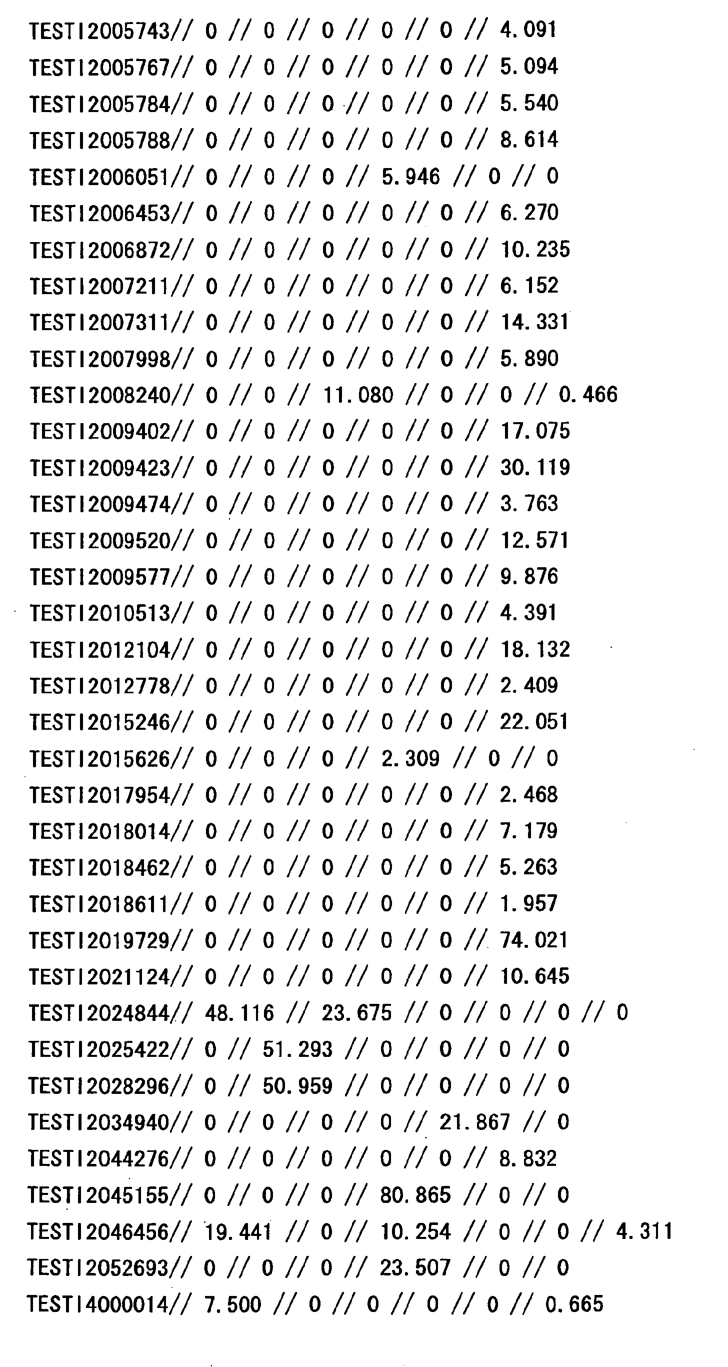

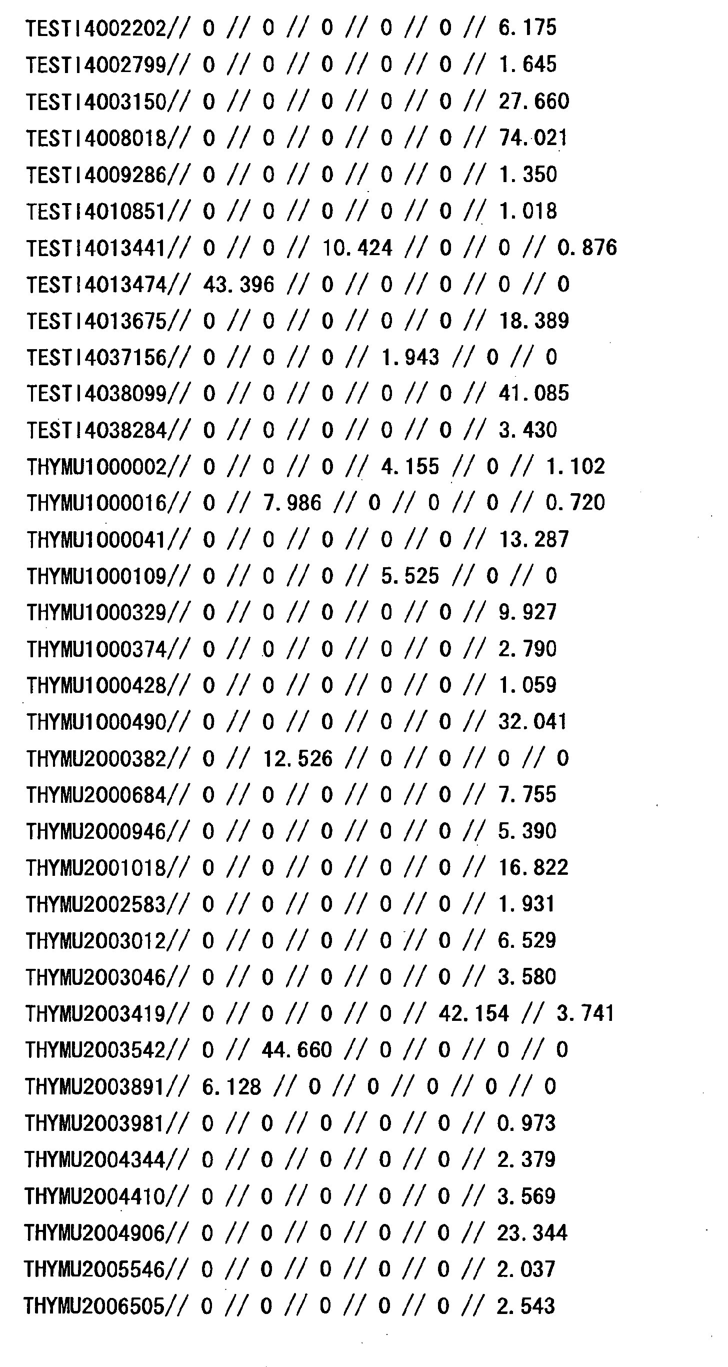

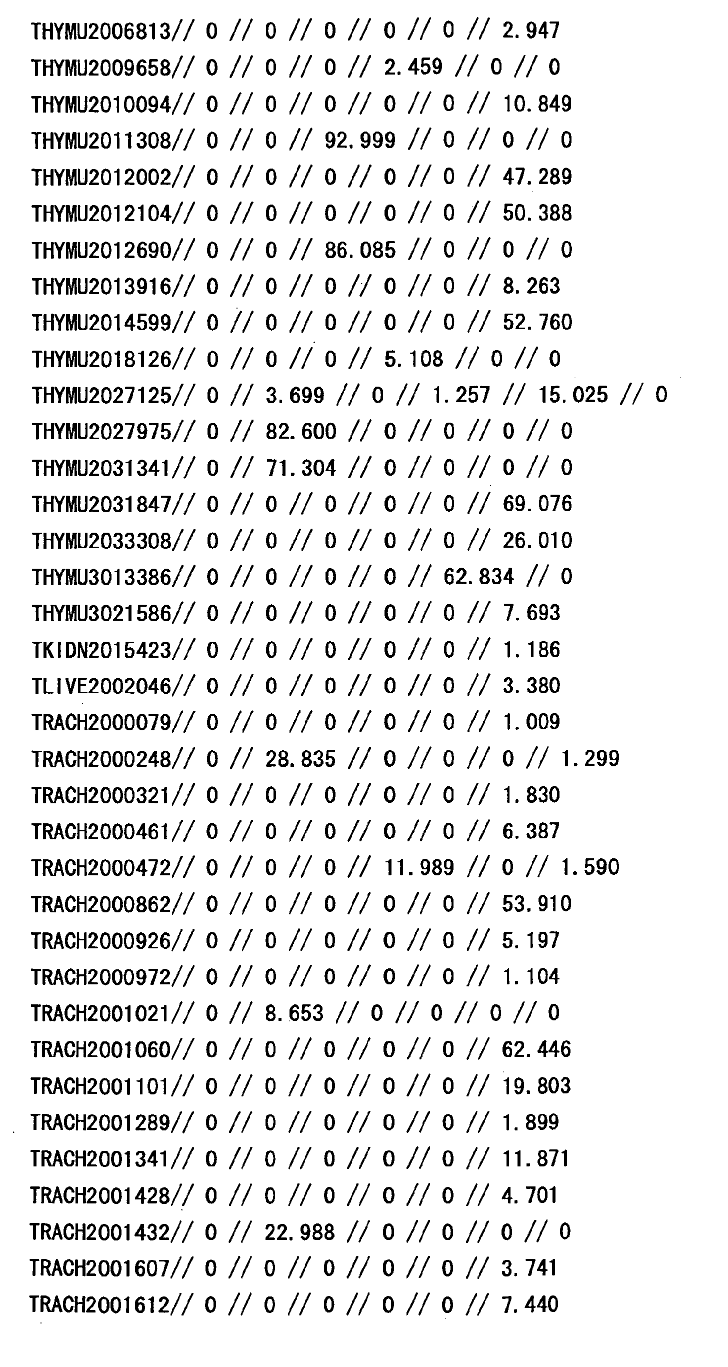

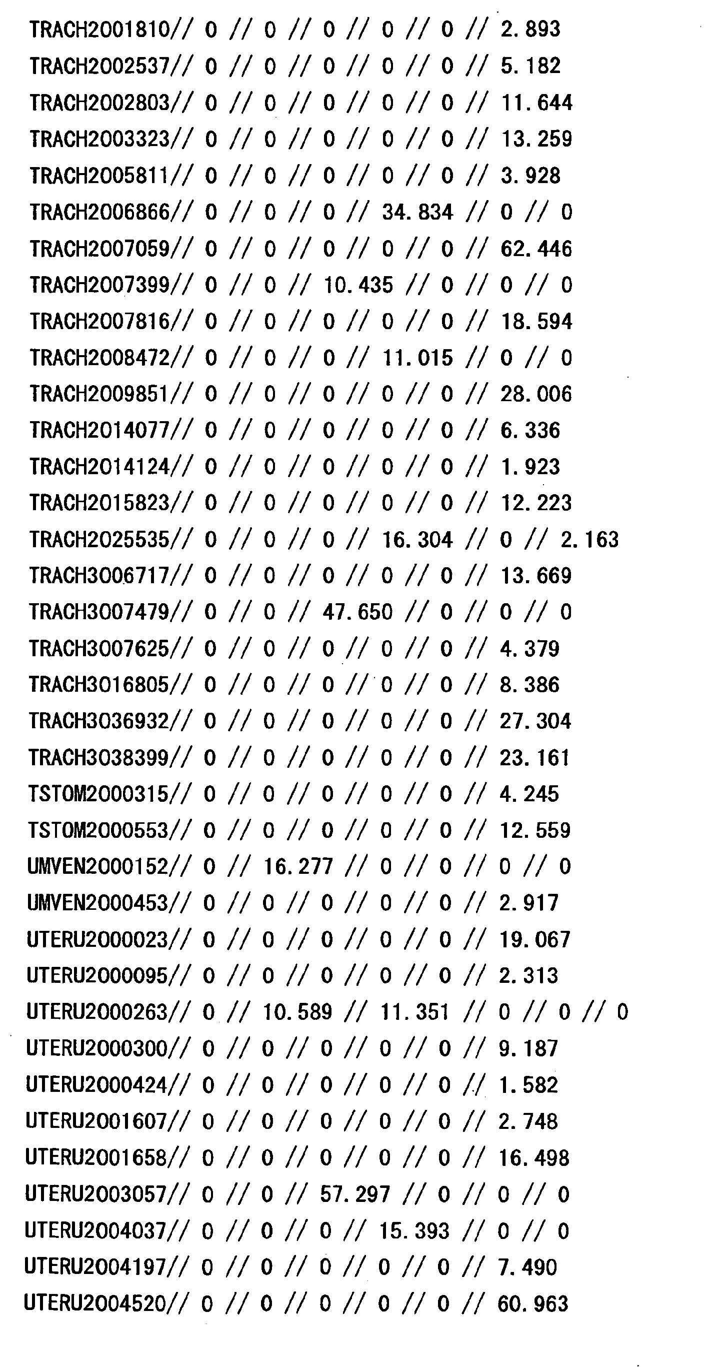

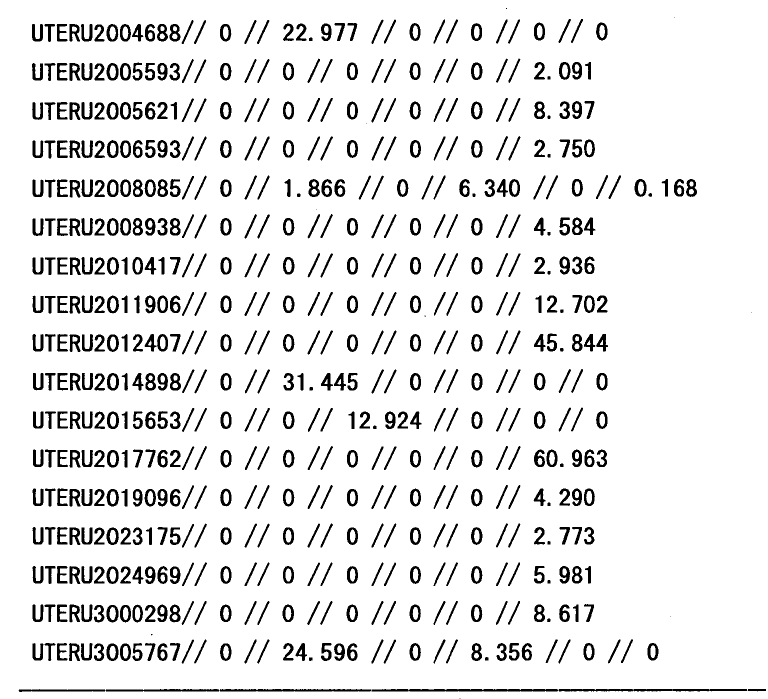

Genes whose expression levels were negligibly low in each of the above-described libraries are shown with their clone names below.
3NB691000018, 3NB691000085, 3NB691000113, 3NB691000129, 3NB692000012, 3NB692000029, 3NB692000102, 3NB692000154, 3NB692000276, 3NB692000305, 3NB692000330, 3NB692000374, 3NB692000429, 3NB692000484, 3NB692000529, 3NB692000545, 3NB692000973, 3NB692001002, 3NB692001022, 3NB692001034, 3NB692001040, 3NB692001123, 3NB692001267, 3NB692001288, 3NB692001349, 3NB692001366, 3NB692001408, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001836, ADRGL2001854, ADRGL2002013, ADRGL2002029, ADRGL2002191, ADRGL2002260, ADRGL2002392, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO1000165, ASTRO2000011, ASTRO2000095, ASTRO2000141, ASTRO2000191, ASTRO2000278, ASTRO2000372, ASTRO2000381, ASTRO2000403, ASTRO2000417, ASTRO2000480, ASTRO2000482, ASTRO2000533, ASTRO2000653, ASTRO2000662, ASTRO2000725, ASTRO2000801, ASTRO2000914, ASTRO2001001, ASTRO2001008, ASTRO2001088, ASTRO2001107, ASTRO2001122, ASTRO2001227, ASTRO2001249, ASTRO2001480, ASTRO2001806, ASTRO2002024, ASTRO2002035, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003316, ASTRO2003574, ASTRO2003581, ASTRO2003740, ASTRO2003840, ASTRO2003925, ASTRO2003960, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005273, ASTRO2005343, ASTRO2005360, ASTRO2005413, ASTRO2005423, ASTRO2005553, ASTRO2005557, ASTRO2005575, ASTRO2005593, ASTRO2005687, ASTRO2005726, ASTRO2005896, ASTRO2005902, ASTRO2006356, ASTRO2006475, ASTRO2006747, ASTRO2006920, ASTRO2006952, ASTRO2007047, ASTRO2007377, ASTRO2007515, ASTRO2007578, ASTRO2007643, ASTRO2007666, ASTRO2007956, ASTRO2007962, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2012552, ASTRO2013124, ASTRO2013585, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015328, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000054, BGGI12000067, BGGI12000161, BGGI12000533, BGGI12000544, BGGI12000616, BGGI12000684, BGGI12000693, BGGI12000839, BGGI12001002, BGGI12001075, BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE-2007043, BLADE2007589, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000153, BNGH41000169, BNGH41000177, BNGH41000216, BNGH41000235, BNGH42000130, BNGH42000132, BNGH42000405, BNGH42000675, BNGH42000739, BNGH42000791, BNGH42000815, BNGH42000994, BNGH42001247, BNGH42001406, BNGH42001576, BNGH42001724, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006226, BNGH42006234, BNGH42007037, BNGH42007366, BNGH42007594, BNGH42007798, BNGH42008199, BNGH42008510, BNGH42008603, BNGH42008649, BNGH42008832, BNGH42008850, BNGH42008943, BNGH42009021, BNGH42009025, BRACE1000020, BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000115, BRACE1000124, BRACE1000132, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000331, BRACE2000332, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001143, BRACE2001151, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001492, BRACE2001496, BRACE2001497, BRACE2001508, BRACE2001543, BRACE2001564, BRACE2001588, BRACE2001607, BRACE2001626, BRACE2001673, BRACE2001677, BRACE2001692, BRACE2001705, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972, BRACE2001973, BRACE2002026, BRACE2002034, BRACE2002050, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002661, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002860, BRACE2002861, BRACE2002884, BRACE2002896, BRACE2002948, BRACE2003037, BRACE2003097, BRACE2003110, BRACE2003168, BRACE2003172, BRACE2003199, BRACE2003210, BRACE2003217, BRACE2003269, BRACE2003285, BRACE2003319, BRACE2003398, BRACE2003420, BRACE2003428, BRACE2003431, BRACE2003512, BRACE2003516, BRACE2003527, BRACE2003539, BRACE2003540, BRACE2003598, BRACE2003609, BRACE2003614, BRACE2003628, BRACE2003639, BRACE2003700, BRACE2003713, BRACE2003766, BRACE2003788, BRACE2003800, BRACE2003802, BRACE2003825, BRACE2003827, BRACE2003847, BRACE2003848, BRACE2003873, BRACE2003885, BRACE2003887, BRACE2003905, BRACE2003928, BRACE2003944, BRACE2003954, BRACE2003982, BRACE2003987, BRACE2005034, BRACE2005047, BRACE2005063, BRACE2005083, BRACE2005090, BRACE2005099, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE20053.37, BRACE2005348, BRACE2005363, BRACE2005395, BRACE2005408, BRACE2005433, BRACE2005434, BRACE2005450, BRACE2005457, BRACE2005460, BRACE2005489, BRACE2005518, BRACE2005556, BRACE2005562, BRACE2005624, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006075, BRACE2006084, BRACE2006089, BRACE2006102, BRACE2006122, BRACE2006137, BRACE2006143, BRACE2006167, BRACE2006174, BRACE2006240, BRACE2006245, BRACE2006249, BRACE2006258, BRACE2006264, BRACE2006274, BRACE2006281, BRACE2006298, BRACE2006344, BRACE2006354, BRACE2006363, BRACE2006378, BRACE2006380, BRACE2006393, BRACE2006413, BRACE2006417, BRACE2006453, BRACE2006459, BRACE2006488, BRACE2006534, BRACE2006537, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006871, BRACE2006909, BRACE2006911, BRACE2006924, BRACE2006935, BRACE2006944, BRACE2006951, BRACE2006982, BRACE2006986, BRACE2007013, BRACE2007064, BRACE2007068, BRACE2007087, BRACE2007089, BRACE2007109, BRACE2007138, BRACE2007174, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007411, BRACE2007447, BRACE2007477, BRACE2007502, BRACE2007503, BRACE2007522, BRACE2007527, BRACE2007538, BRACE2007563, BRACE2007567, BRACE2007621, BRACE2007640, BRACE2007641, BRACE2007646, BRACE2007663, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008968, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039624, BRACE2039734, BRACE2039823, BRACE 2039 832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005938, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE304.4172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2019989, BRAMY2020050, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026713, BRAMY2026824, BRAMY2026899, BRAMY2026902, BRAMY2026904, BRAMY2026976, BRAMY2027073, BRAMY2027114, BRAMY2027242, BRAMY2027396, BRAMY2027451, BRAMY2027461, BRAMY2027467, BRAMY2027587, BRAMY2027714, BRAMY2027717, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001913, BRAMY4002575, BRAMY4002628, BRASW1000053, BRASW1000125, BRAWH1000001, BRAWH1000002, BRAWH1000007, BRAWH1000037, BRAWH1000040, BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000471, BRAWH2000476, BRAWH2000482, BRAWH2000488, BRAWH2000494, BRAWH2000500, BRAWH2000503, BRAWH2000522, BRAWH2000554, BRAWH2000588, BRAWH2000595, BRAWH2000636, BRAWH2000643, BRAWH2000651, BRAWH2000682, BRAWH2000686, BRAWH2000697, BRAWH2000752, BRAWH2000859, BRAWH2000866, BRAWH2000892, BRAWH2000901, BRAWH2000924, BRAWH2000926, BRAWH2000944, BRAWH2000984, BRAWH2001019, BRAWH2001037, BRAWH2001052, BRAWH2001103, BRAWH2001129, BRAWH2001137, BRAWH2001141, BRAWH2001159, BRAWH2001166, BRAWH2001186, BRAWH2001203, BRAWH2001238, BRAWH2001239, BRAWH2001255, BRAWH2001355, BRAWH2001378, BRAWH2001395, BRAWH2001406, BRAWH2001412, BRAWH2001418, BRAWH2001424, BRAWH2001436, BRAWH2001438, BRAWH2001439, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025, BRAWH2003307, BRAWH2003355, BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380, BRAWH2008540, BRAWH2008706, BRAWH2008790, BRAWH2008903, BRAWH2008993, BRAWH2009112, BRAWH2009238, BRAWH2009261, BRAWH2009263, BRAWH2009298, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014511, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018526, BRAWH2018527, BRAWH2018601, BRAWH2018729, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015402, BRCAN2015464, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022228, BRHIP2022298, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000060, BRHIP3000070, BRHIP3000081, BRHIP3000111, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014636, BRSSN2014685, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013171, BRSTN2013354, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016892, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194, BRTHA2006232, BRTHA2006720, BRTHA2006735, BRTHA2006974, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3003736, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020369, BRTHA3021569, BRTHA3021708, BRTHA3021786, BRTHA3021971, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000195, D3OST3000258, D3OST3000291, D3OST3000388, D3OST3000493 D6OST2000127, D6OST2000169 D6OST2000245 D6OST2000358 D6OST2000464, D6OST2000507 D9OST2000031 D9OST2000278 D9OST2000440 D9OST2000869 D9OST2001319, D9OST2001433 D9OST2002608 D9OST2002673 D9OST2003106, D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000083, DFNES1000107, DFNES2000143, DFNES2000146, DFNES2000153, DFNES2000212, DFNES2000268, DFNES2000292, DFNES2000426, DFNES2000432, DFNES2000443, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004383, DFNES2004608, DFNES2004684, DFNES2005266, DFNES2005527, DFNES2005540, DFNES2005779, DFNES2005875, DFNES2006346, DFNES2006944, DFNES2007113, DFNES2007332, DFNES2007641, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000025, FCBBF1000038, FCBBF1000063, FCBBF1000069, FCBBF1000077, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000367, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000476, FCBBF1000506, FCBBF1000509, FCBBF1000546, FCBBF1000550, FCBBF1000556, FCBBF1000563, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF1000760, FCBBF2000038, FCBBF2000087, FCBBF2000094, FCBBF2000105, FCBBF2000123, FCBBF2000195, FCBBF2000199, FCBBF2000214, FCBBF2000232, FCBBF2000276, FCBBF2000291, FCBBF2000362, FCBBF2000379, FCBBF2000473, FCBBF2000502, FCBBF2000566, FCBBF2000576, FCBBF2000591, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001001, FCBBF2001088, FCBBF2001105, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001632, FCBBF3001657, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005330, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009541, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016178, FCBBF3016451, FCBBF3016618, FCBBF3016622, FCBBF3016644, FCBBF3016667, FCBBF3016928, FCBBF3016987, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000129, FEBRA2000220, FEBRA2000253, FEBRA2000282, FEBRA2000286, FEBRA2000297, FEBRA2000311, FEBRA2000321, FEBRA2000333, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452, FEBRA2000454, FEBRA2000462, FEBRA2000468, FEBRA2000491, FEBRA2000504, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000757, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001828, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003897, FEBRA2003907, FEBRA2003926, FEBRA2003930, FEBRA2004023, FEBRA2004026, FEBRA2004029, FEBRA2004053, FEBRA2004056, FEBRA2004091, FEBRA2004110, FEBRA2004191, FEBRA2004202, FEBRA2004219, FEBRA2004237, FEBRA2004268, FEBRA2004293, FEBRA2004325, FEBRA2004329, FEBRA2004331, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005427, FEBRA2005458, FEBRA2005572, FEBRA2005701, FEBRA2005726, FEBRA2005734, FEBRA2005752, FEBRA2005778, FEBRA2005787, FEBRA2005788, FEBRA2005998, FEBRA2006055, FEBRA2006061, FEBRA2006092, FEBRA2006150, FEBRA2006165, FEBRA2006255, FEBRA2006315, FEBRA2006346, FEBRA2006354, FEBRA2006357, FEBRA2006368, FEBRA2006370, FEBRA2006379, FEBRA2006396, FEBRA2006409, FEBRA2006418, FEBRA2006427, FEBRA2006476, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007551, FEBRA2007566, FEBRA2007589, FEBRA2007613, FEBRA2007620, FEBRA2007708, FEBRA2007714, FEBRA2007793, FEBRA2007818, FEBRA2007823, FEBRA2007901, FEBRA2007931, FEBRA2007957, FEBRA2008009, FEBRA2008036, FEBRA2008086, FEBRA2008134, FEBRA2008135, FEBRA2008159, FEBRA2008165, FEBRA2008172, FEBRA2008210, FEBRA2008255, FEBRA2008265, FEBRA2008266, FEBRA2008282, FEBRA2008287, FEBRA2008302, FEBRA2008311, FEBRA2008341, FEBRA2008360, FEBRA2008459, FEBRA2008468, FEBRA2008475, FEBRA2008481, FEBRA2008542, FEBRA2008583, FEBRA2008660, FEBRA2008662, FEBRA2008681, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013905, FEBRA2013951, FEBRA2014010, FEBRA2014122, FEBRA2014198, FEBRA2014213, FEBRA2014297, FEBRA2014417, FEBRA2014443, FEBRA2014578, FEBRA2014939, FEBRA2015076, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018433, FEBRA2018476, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255, FEBRA2022322, FEBRA2022504, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025463, FEBRA2025477, FEBRA2025838, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021, HCASM1000050, HCASM1000061, HCASM1000130, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000131, HCHON1000142, HCHON1000166, HCHON1000176, HCHON1000191, HCHON2000038, HCHON2000062, HCHON2000100, HCHON2000156, HCHON2000199, HCHON2000212, HCHON2000244, HCHON2000265, HCHON2000271, HCHON2000315, HCHON2000322, HCHON2000323, HCHON2000344, HCHON2000373, HCHON2000395, HCHON2000417, HCHON2000418, HCHON2000503, HCHON2000660, HCHON2000663, HCHON2000673, HCHON2000676, HCHON2000698, HCHON2000699, HCHON2000705, HCHON2000743, HCHON2000815, HCHON2000817, HCHON2000818, HCHON2000819, HCHON2000832, HCHON2000851, HCHON2000898, HCHON2000935, HCHON2000942, HCHON2000956, HCHON2000994, HCHON2001084, HCHON2001099, HCHON2001108, HCHON2001110, HCHON2001116, HCHON2001161, HCHON2001214, HCHON2001233, HCHON2001269, HCHON2001434, HCHON2001450, HCHON2001453, HCHON2001497, HCHON2001505, HCHON2001519, HCHON2001523, HCHON2001546, HCHON2001548, HCHON2001577, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000074, HEART1000088, HEART1000099, HEART1000102, HEART1000118, HEART1000132, HEART1000139, HEART1000142, HEART1000149, HEART1000151, HEART1000173, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000083, HHDPC1000114, HHDPC1000163, HHDPC2000055, HHDPC2000102, HHDPC2000104, HHDPC2000115, HHDPC2000258, HHDPC2000315, HHDPC2000455, HHDPC2000462, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000064, HLUNG1000084, HLUNG1000091, HLUNG1000104, HLUNG1000105, HLUNG1000110, HLUNG1000151, HLUNG1000169, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000431, HLUNG2000480, HLUNG2000489, HLUNG2000501, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013, HLUNG2001126, HLUNG2001144, HLUNG2001214, HLUNG2001439, HLUNG2001459, HLUNG2001507, HLUNG2001518, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002648, HLUNG2002659, HLUNG2002707, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004067, HLUNG2004154, HLUNG2004170, HLUNG2004217, HLUNG2004273, HLUNG2004388, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008480, HLUNG2008521, HLUNG2008833, HLUNG2008875, HLUNG2008885, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA1000178, HSYRA2000044, HSYRA2000137, HSYRA2000190, HSYRA2000221, HSYRA2000224, HSYRA2000232, HSYRA2000237, HSYRA2000253, HSYRA2000332, HSYRA2000371, HSYRA2000424, HSYRA2000507, HSYRA2000510, HSYRA2000605, HSYRA2000629, HSYRA2000640, HSYRA2000706, HSYRA2000760, HSYRA2000787, HSYRA2000792, HSYRA2000828, HSYRA2000832, HSYRA2000925, HSYRA2000927, HSYRA2001003, HSYRA2001103, HSYRA2001105, HSYRA2001142, HSYRA2001153, HSYRA2001232, HSYRA2001255, HSYRA2001332, HSYRA2001353, HSYRA2001396, HSYRA2001420, HSYRA2001484, HSYRA2001552, HSYRA2001580, HSYRA2001615, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007667, HSYRA2008154, HSYRA2008714, HSYRA2009102, IMR321000155, IMR321000158, IMR321000161, IMR321000165, IMR321000207, IMR321000210, IMR321000230, IMR321000242, IMR321000266, IMR322000010, IMR322000072, IMR322000107, IMR322000121, IMR322000127, IMR322000144, IMR322000152, IMR322000164, IMR322000178, IMR322000223, IMR322000243, IMR322000302, IMR322000316, IMR322000374, IMR322000379, IMR322000383, IMR322000418, IMR322000555, IMR322000604, IMR322000609, IMR322000656, IMR322000672, IMR322000742, IMR322000775, IMR322000791, IMR322000811, IMR322000819, IMR322000838, IMR322000859, IMR322000863, IMR322000917, IMR322000918, IMR322000919, IMR322000953, IMR322000973, IMR322000984, IMR322001018, IMR322001167, IMR322001185, IMR322001245, IMR322001317, IMR322001318, IMR322001322, IMR322001380, IMR322001438, IMR322001452, IMR322001491, IMR322001534, IMR322001541, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, IMR322019070, JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000145, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000422, KIDNE2000493, KIDNE2000497, KIDNE2000510, KIDNE2000513, KIDNE2000517, KIDNE2000519, KIDNE2000555, KIDNE2000624, KIDNE2000665, KIDNE2000678, KIDNE2000717, KIDNE2000721, KIDNE2000722, KIDNE2000801, KIDNE2000832, KIDNE2000833, KIDNE2000846, KIDNE2000867, KIDNE2000909, KIDNE2001117, KIDNE2001140, KIDNE2001162, KIDNE2001269, KIDNE2001361, KIDNE2001636, KIDNE2001713, KIDNE2001802, KIDNE2001840, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882, KIDNE2002883, KIDNE2002980, KIDNE2002991, KIDNE2003185, KIDNE2003188, KIDNE2003233, KIDNE2003305, KIDNE2003335, KIDNE2003357, KIDNE2003373, KIDNE2003377, KIDNE2003507, KIDNE2003752, KIDNE2003764, KIDNE2003837, KIDNE2003941, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077, KIDNE2007186, KIDNE2007222, KIDNE2007352, KIDNE2007356, KIDNE2007422, KIDNE2007569, KIDNE2007810, KIDNE2007811, KIDNE2007944, KIDNE2007954, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126, LIVER1000132, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000416, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415, LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000173, MAMGL1000178, MAMGL1000182, MAMGL1000184, MESAN1000032, MESAN1000035, MESAN1000050, MESAN1000079, MESAN1000101, MESAN1000121, MESAN1000147, MESAN1000180, MESAN2000023, MESAN2000026, MESAN2000067, MESAN2000167, MESAN2000264, MESAN2000291, MESAN2000434, MESAN2000457, MESAN2000462, MESAN2000501, MESAN2000608, MESAN2000620, MESAN2000711, MESAN2000894, MESAN2000909, MESAN2001450, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002724, MESAN2002844, MESAN2002978, MESAN2003035, MESAN2003037, MESAN2003039, MESAN2003058, MESAN2003101, MESAN2003190, MESAN2003322, MESAN2003444, MESAN2003490, MESAN2003529, MESAN2003622, MESAN2003646, MESAN2003662, MESAN2003709, MESAN2003851, MESAN2004138, MESAN2004288, MESAN2004575, MESAN2005284, MESAN2005303, MESAN2005371, MESAN2005689, MESAN2005724, MESAN2005808, MESAN2005811, MESAN2005957, MESAN2006022, MESAN2006043, MESAN2006328, MESAN2006580, MESAN2006599, MESAN2006743, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926, MESAN2008936, MESAN2009081, MESAN2009105, MESAN2009311, MESAN2009418, MESAN2009503, MESAN2009522, MESAN2009580, MESAN2010031, MESAN2010114, MESAN2010312, MESAN2010321, MESAN2010664, MESAN2010681, MESAN2011545, MESAN2011597, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000011, NB9N41000121, NB9N41000142, NB9N41000146, NB9N41000340, NB9N42000104, NB9N42000122, NB9N42000196, NB9N42000281, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000091, NOVAR1000102, NOVAR2000038, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000195, NT2NE2000222, NT2NE2000259, NT2NE2000284, NT2NE2000299, NT2NE2000353, NT2NE2000356, NT2NE2000369, NT2NE2000374, NT2NE2000383, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000809, NT2NE2000864, NT2NE2000909, NT2NE2000950, NT2NE2000951, NT2NE2000980, NT2NE2001000, NT2NE2001005, NT2NE2001021, NT2NE2001040, NT2NE2001048, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001337, NT2NE2001364, NT2NE2001372, NT2NE2001403, NT2NE2001428, NT2NE2001432, NT2NE2001435, NT2NE2001442, NT2NE2001524, NT2NE2001530, NT2NE2001545, NT2NE2001598, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003185, NT2NE2003252, NT2NE2003280, NT2NE2003308, NT2NE2003309, NT2NE2003315, NT2NE2003393, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004519, NT2NE2004606, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005784, NT2NE2005804, NT2NE2005821, NT2NE2005876, NT2NE2005890, NT2NE2005921, NT2NE2005968, NT2NE2006087, NT2NE2006103, NT2NE2006217, NT2NE2006288, NT2NE2006382, NT2NE2006387, NT2NE2006427, NT2NE2006432, NT2NE2006458, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010842, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000027, NT2RI1000035, NT2RI1000164, NT2RI1000172, NT2RI2000004, NT2RI2000007, NT2RI2000064, NT2RI2000107, NT2RI2000116, NT2RI2000117, NT2RI2000128, NT2RI2000133, NT2RI2000167, NT2RI2000173, NT2RI2000187, NT2RI2000270, NT2RI2000276, NT2RI2000282, NT2RI2000284, NT2RI2000294, NT2RI2000313, NT2RI2000341, NT2RI2000348, NT2RI2000412, NT2RI2000421, NT2RI2000480, NT2RI2000575, NT2RI2000588, NT2RI2000597, NT2RI2000669, NT2RI2000685, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000974, NT2RI2000987, NT2RI2001010, NT2RI2001017, NT2RI2001030, NT2RI2001054, NT2RI2001083, NT2RI2001091, NT2RI2001167, NT2RI2001178, NT2RI2001230, NT2RI2001235, NT2RI2001244, NT2RI2001307, NT2RI2001385, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001726, NT2RI2001731, NT2RI2001769, NT2RI2001782, NT2RI2001836, NT2RI2001846, NT2RI2001866, NT2RI2001910, NT2RI2001952, NT2RI2002022, NT2RI2002041, NT2RI2002120, NT2RI2002152, NT2RI2002153, NT2RI2002179, NT2RI2002252, NT2RI2002260, NT2RI2002266, NT2RI2002270, NT2RI2002334, NT2RI2002359, NT2RI2002447, NT2RI2002481, NT2RI2002507, NT2RI2002517, NT2RI2002540, NT2RI2002549, NT2RI2002554, NT2RI2002564, NT2RI2002585, NT2RI2002592, NT2RI2002602, NT2RI2002699, NT2RI2002802, NT2RI2002819, NT2RI2002822, NT2RI2002847, NT2RI2002852, NT2RI2002923, NT2RI2002926, NT2RI2002958, NT2RI2002970, NT2RI2003011, NT2RI2003067, NT2RI2003184, NT2RI2003205, NT2RI2003222, NT2RI2003301, NT2RI2003317, NT2RI2003338, NT2RI2003344, NT2RI2003360, NT2RI2003361, NT2RI2003383, NT2RI2003392, NT2RI2003399, NT2RI2003419, NT2RI2003481, NT2RI2003487, NT2RI2003556, NT2RI2003655, NT2RI2003667, NT2RI2003678, NT2RI2003751, NT2RI2003884, NT2RI2003921, NT2RI2003973, NT2RI2003993, NT2RI2004059, NT2RI2004078, NT2RI2004079, NT2RI2004093, NT2RI2004136, NT2RI2004157, NT2RI2004188, NT2RI2004190, NT2RI2004209, NT2RI2004230, NT2RI2004284, NT2RI2004312, NT2RI2004381, NT2RI2004398, NT2RI2004410, NT2RI2004442, NT2RI2004468, NT2RI2004535, NT2RI2004606, NT2RI2004608, NT2RI2004618, NT2RI2004783, NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005130, NT2RI2005150, NT2RI2005166, NT2RI2005239, NT2RI2005246, NT2RI2005368, NT2RI2005382, NT2RI2005405, NT2RI2005473, NT2RI2005520, NT2RI2005564, NT2RI2005579, NT2RI2005621, NT2RI2005628, NT2RI2005670, NT2RI2005710, NT2RI2005723, NT2RI2005772, NT2RI2005811, NT2RI2005814, NT2RI2005816, NT2RI2005818, NT2RI2005966, NT2RI2006070, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006412, NT2RI2006445, NT2RI2006487, NT2RI2006553, NT2RI2006565, NT2RI2006640, NT2RI2006659, NT2RI2006662, NT2RI2006667, NT2RI2006679, NT2RI2006682, NT2RI2006686, NT2RI2006703, NT2RI2006716, NT2RI2006762, NT2RI2006788, NT2RI2006838, NT2RI2006855, NT2RI2006943, NT2RI2006973, NT2RI2007046, NT2RI2007048, NT2RI2007054, NT2RI2007084, NT2RI2007096, NT2RI2007116, NT2RI2007148, NT2RI2007214, NT2RI2007277, NT2RI2007303, NT2RI2007334, NT2RI2007377, NT2RI2007386, NT2RI2007406, NT2RI2007439, NT2RI2007445, NT2RI2007468, NT2RI2007469, NT2RI2007507, NT2RI2007629, NT2RI2007723, NT2RI2007729, NT2RI2007751, NT2RI2007754, NT2RI2007827, NT2RI2007853, NT2RI2007877, NT2RI2007879, NT2RI2007884, NT2RI2007891, NT2RI2007956, NT2RI2007965, NT2RI2007987, NT2RI2008007, NT2RI2008045, NT2RI2008050, NT2RI2008171, NT2RI2008188, NT2RI2008204, NT2RI2008221, NT2RI2008233, NT2RI2008455, NT2RI2008502, NT2RI2008526, NT2RI2008566, NT2RI2008598, NT2RI2008622, NT2RI2008656, NT2RI2008791, NT2RI2008801, NT2RI2008808, NT2RI2008812, NT2RI2008841, NT2RI2008952, NT2RI2009005, NT2RI2009065, NT2RI2009066, NT2RI2009083, NT2RI2009101, NT2RI2009103, NT2RI2009144, NT2RI2009151, NT2RI2009194, NT2RI2009215, NT2RI2009259, NT2RI2009269, NT2RI2009281, NT2RI2009289, NT2RI2009301, NT2RI2009402, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2015533, NT2RI2016128, NT2RI2016134, NT2RI2017529, NT2RI2018311, NT2RI2018363, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006340, NT2RI3006376, NT2RI3006666, NT2RI3006796, NT2RI3007065, NT2RI3007095, NT2RI3007158, NT2RI3007167, NT2RI3007213, NT2RI3007291, NT2RI3007443, NT2RI3007684, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000008, NT2RP6000032, NT2RP6000033, NT2RP6000039, NT2RP6000043, NT2RP6000059, NT2RP6000060, NT2RP6000072, NT2RP6000077, NT2RP6000078, NT2RP6000085, NT2RP6000086, NT2RP6000100, NT2RP6000110, NT2RP6000119, NT2RP6000127, NT2RP6000130, NT2RP6000140, NT2RP6000150, NT2RP7000041, NT2RP7000069, NT2RP7000109, NT2RP7000112, NT2RP7000238, NT2RP7000259, NT2RP7000311, NT2RP7000359, NT2RP7000364, NT2RP7000380, NT2RP7000391, NT2RP7000425, NT2RP7000579, NT2RP7000586, NT2RP7000624, NT2RP7000812, NT2RP7000926, NT2RP7001074, NT2RP7001080, NT2RP7001156, NT2RP7001231, NT2RP7001306, NT2RP7001319, NT2RP7001335, NT2RP7001364, NT2RP7001532, NT2RP7001602, NT2RP7001856, NT2RP7001962, NT2RP7002028, NT2RP7002064, NT2RP7002151, NT2RP7002163, NT2RP7002243, NT2RP7002282, NT2RP7002478, NT2RP7002554, NT2RP7002603, NT2RP7002650, NT2RP7002738, NT2RP7002779, NT2RP7002829, NT2RP7002841, NT2RP7002875, NT2RP7002906, NT2RP7002978, NT2RP7003025, NT2RP7003050, NT2RP7003055, NT2RP7003084, NT2RP7003091, NT2RP7003107, NT2RP7003134, NT2RP7003148, NT2RP7003203, NT2RP7003439, NT2RP7003511, NT2RP7003629, NT2RP7003680, NT2RP7003724, NT2RP7004027, NT2RP7004080, NT2RP7004114, NT2RP7004204, NT2RP7004233, NT2RP7004260, NT2RP7004358, NT2RP7004396, NT2RP7004428, NT2RP7004481, NT2RP7004541, NT2RP7004641, NT2RP7004656, NT2RP7004687, NT2RP7004722, NT2RP7004728, NT2RP7004766, NT2RP7004790, NT2RP7004884, NT2RP7004915, NT2RP7004961, NT2RP7004964, NT2RP7004975, NT2RP7005118, NT2RP7005205, NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005846, NT2RP7006033, NT2RP7006160, NT2RP7006162, NT2RP7006223, NT2RP7006263, NT2RP7006296, NT2RP7006304, NT2RP7006374, NT2RP7006395, NT2RP7006408, NT2RP7006457, NT2RP7006539, NT2RP7006547, NT2RP7006601, NT2RP7006619, NT2RP7006621, NT2RP7006636, NT2RP7006853, NT2RP7006886, NT2RP7006887, NT2RP7006980, NT2RP7006986, NT2RP7006995, NT2RP7007114, NT2RP7007221, NT2RP7007226, NT2RP7007252, NT2RP7007269, NT2RP7007310, NT2RP7007359, NT2RP7007381, NT2RP7007387, NT2RP7007406, NT2RP7007480, NT2RP7007504, NT2RP7007530, NT2RP7007537, NT2RP7007580, NT2RP7007594, NT2RP7007617, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008315, NT2RP7008360, NT2RP7008396, NT2RP7008406, NT2RP7008435, NT2RP7008441, NT2RP7008454, NT2RP7008544, NT2RP7008550, NT2RP7008557, NT2RP7008623, NT2RP7008657, NT2RP7008714, NT2RP7008720, NT2RP7008855, NT2RP7008863, NT2RP7009019, NT2RP7009030, NT2RP7009097, NT2RP7009168, NT2RP7009215, NT2RP7009225, NT2RP7009236, NT2RP7009253, NT2RP7009267, NT2RP7009322, NT2RP7009363, NT2RP7009370, NT2RP7009394, NT2RP7009397, NT2RP7009429, NT2RP7009439, NT2RP7009466, NT2RP7009481, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014178, NT2RP7014348, NT2RP7014420, NT2RP7014583, NT2RP7014721, NT2RP7014743, NT2RP7014778, NT2RP7014906, NT2RP7014910, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000246, NTONG1000247, NTONG2000040, NTONG2000107, NTONG2000231, NTONG2000334, NTONG2000363, NTONG2000413, NTONG2000492, NTONG2000499, NTONG2000531, NTONG2000583, NTONG2000800, NTONG2000858, NTONG2000876, NTONG2000878, NTONG2000966, NTONG2000985, NTONG2001014, NTONG2001079, NTONG2001157, NTONG2001173, NTONG2001220, NTONG2001362, NTONG2001428, NTONG2001532, NTONG2001612, NTONG2001619, NTONG2001642, NTONG2001657, NTONG2001676, NTONG2001762, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003839, NTONG2003852, NTONG2003897, NTONG2003928, NTONG2004095, NTONG2004308, NTONG2004521, NTONG2004614, NTONG2004690, NTONG2004806, NTONG2004829, NTONG2004844, NTONG2004991, NTONG2005062, NTONG2005086, NTONG2005137, NTONG2005153, NTONG2005265, NTONG2005277, NTONG2005363, NTONG2005373, NTONG2005391, NTONG2005480, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000114, OCBBF1000118, OCBBF1000119, OCBBF1000122, OCBBF1000130, OCBBF1000138, OCBBF1000145, OCBBF1000153, OCBBF1000175, OCBBF1000185, OCBBF1000192, OCBBF1000254, OCBBF2000013, OCBBF2000126, OCBBF2000178, OCBBF2000231, OCBBF2000277, OCBBF2000287, OCBBF2000323, OCBBF2000452, OCBBF2000467, OCBBF2000703, OCBBF2000712, OCBBF2000719, OCBBF2000824, OCBBF2000831, OCBBF2000904, OCBBF2000982, OCBBF2000998, OCBBF2001075, OCBBF2001101, OCBBF2001124, OCBBF2001166, OCBBF2001176, OCBBF2001186, OCBBF2001196, OCBBF2001210, OCBBF2001252, OCBBF2001307, OCBBF2001323, OCBBF2001389, OCBBF2001402, OCBBF2001408, OCBBF2001416, OCBBF2001473, OCBBF2001494, OCBBF2001527, OCBBF2001528, OCBBF2001586, OCBBF2001639, OCBBF2001681, OCBBF2001706, OCBBF2001749, OCBBF2001794, OCBBF2001910, OCBBF2001931, OCBBF2001961, OCBBF2001983, OCBBF2002015, OCBBF2002083, OCBBF2002124, OCBBF2002161, OCBBF2002290, OCBBF2002357, OCBBF2002376, OCBBF2002615, OCBBF2002626, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038, OCBBF2004104, OCBBF2004147, OCBBF2004168, OCBBF2004235, OCBBF2004259, OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2006005, OCBBF2006030, OCBBF2006058, OCBBF2006113, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF200-7762, OCBBF2007931, OCBBF2007946, OCBBF2008005, OCBBF2008041, OCBBF2008116, OCBBF2008138, OCBBF2008144, OCBBF2008283, OCBBF2008330, OCBBF2008340, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775, OCBBF2008790, OCBBF2008814, OCBBF2008822, OCBBF2009095, OCBBF2009115, OCBBF2009301, OCBBF2009352, OCBBF2009391, OCBBF2009536, OCBBF2009571, OCBBF2009583, OCBBF2009603, OCBBF2009772, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010841, OCBBF2010843, OCBBF2010863, OCBBF2010871, OCBBF2010931, OCBBF2010945, OCBBF2010978, OCBBF2010989, OCBBF2011021, OCBBF2011137, OCBBF2011160, OCBBF2011177, OCBBF2011228, OCBBF2011232, OCBBF2011283, OCBBF2011311, OCBBF2011536, OCBBF2011625, OCBBF2011669, OCBBF2011685, OCBBF2011706, OCBBF2011722, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012553, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013883, OCBBF2013926, OCBBF2014052, OCBBF2014064, OCBBF2014089, OCBBF2014292, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2016928, OCBBF2017035, OCBBF2017055, OCBBF2017069, OCBBF2017306, OCBBF2017325, OCBBF2017398, OCBBF2017458, OCBBF2017489, OCBBF2017516, OCBBF2017665, OCBBF2017754, OCBBF2017815, OCBBF2017882, OCBBF2017888, OCBBF2017893, OCBBF2017920, OCBBF2018014, OCBBF2018084, OCBBF2018167, OCBBF2018206, OCBBF2018229, OCBBF2018234, OCBBF2018380, OCBBF2018563, OCBBF2018581, OCBBF2018618, OCBBF2018663, OCBBF2018687, OCBBF2018707, OCBBF2018828, OCBBF2018934, OCBBF2018956, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000323, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000180, PEBLM2000030, PEBLM2000112, PEBLM2000126, PEBLM2000147, PEBLM2000170, PEBLM2000180, PEBLM2000213, PEBLM2000248, PEBLM2000267, PEBLM2000270, PEBLM2000308, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004497, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007296, PEBLM2007437, PEBLM2007447, PEBLM2007598, PEBLM2007832, PEBLM2007834, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000058, PLACE5000066, PLACE5000067, PLACE5000068, PLACE5000070, PLACE5000080, PLACE5000105, PLACE5000113, PLACE5000115, PLACE5000116, PLACE5000129, PLACE5000139, PLACE5000153, PLACE5000170, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000263, PLACE6000296, PLACE6000314, PLACE6000325, PLACE6000348, PLACE6000371, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001118, PLACE6001153, PLACE6001185, PLACE6001262, PLACE6001294, PLACE6001443, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003399, PLACE6003539, PLACE6003621, PLACE6003705, PLACE6003740, PLACE6003771, PLACE6003796, PLACE6003839, PLACE6003850, PLACE6004005, PLACE6004219, PLACE6004336, PLACE6004368, PLACE6004380, PLACE6004396, PLACE6004397, PLACE6004454, PLACE6004464, PLACE6004491, PLACE6004578, PLACE6004663, PLACE6004687, PLACE6004738, PLACE6004931, PLACE6004966, PLACE6004993, PLACE6005007, PLACE6005029, PLACE6005108, PLACE6005231, PLACE6005234, PLACE6005283, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008775, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011057, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016383, PLACE6016942, PLACE6016984, PLACE6017002, PLACE6017431, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000039, PROST1000065, PROST1000083, PROST1000085, PROST1000110, PROST1000120, PROST1000123, PROST1000136, PROST1000144, PROST1000145, PROST1000152, PROST1000167, PROST1000184, PROST1000215, PROST1000217, PROST1000246, PROST1000262, PROST1000298, PROST1000322, PROST1000334, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000564, PROST1000575, PROST2000036, PROST2000053, PROST2000067, PROST2000079, PROST2000105, PROST2000143, PROST2000162, PROST2000203, PROST2000206, PROST2000212, PROST2000241, PROST2000267, PROST2000273, PROST2000274, PROST2000277, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001180, PROST2001213, PROST2001283, PROST2001414, PROST2001415, PROST2001465, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003324, PROST2003338, PROST2003340, PROST2003396, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004258, PROST2004332, PROST2004481, PROST2004526, PROST2004570, PROST2004727, PROST2004739, PROST2004742, PROST2004744, PROST2004817, PROST2005067, PROST2005076, PROST2005093, PROST2005121, PROST2005131, PROST2005143, PROST2005228, PROST2005272, PROST2005285, PROST2005426, PROST2005466, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006196, PROST2006201, PROST2006282, PROST2006343, PROST2006450, PROST2006491, PROST2006510, PROST2006536, PROST2006579, PROST2006688, PROST2006737, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008268, PROST2008447, PROST2008468, PROST2008472, PROST2008516, PROST2008724, PROST2008770, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010885, PROST2011012, PROST2011038, PROST2011105, PROST2011297, PROST2011439, PROST2011482, PROST2011631, PROST2011660, PROST2012005, PROST2012007, PROST2012016, PROST2012088, PROST2012157, PROST2012190, PROST2012249, PROST2012353, PROST2012400, PROST2012448, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017203, PROST2017367, PROST2017413, PROST2017441, PROST2017578, PROST2017612, PROST2017617, PROST2017700, PROST2017701, PROST2017729, PROST2017836, PROST2017910, PROST2017972, PROST2018030, PROST2018235, PROST2018437, PROST2018487, PROST2018511, PROST2018583, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000085, PUAEN1000087, PUAEN1000121, PUAEN1000147, PUAEN1000161, PUAEN1000175, PUAEN1000239, PUAEN1000275, PUAEN1000276, PUAEN1000288, PUAEN1000322, PUAEN2000080, PUAEN2000152, PUAEN2000175, PUAEN2000247, PUAEN2000394, PUAEN2000497, PUAEN2000594, PUAEN2000684, PUAEN2000942, PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000157, SALGL1000171, SKMUS1000014, SKMUS1000030, SKMUS1000064, SKMUS1000067, SKMUS1000084, SKMUS1000102, SKMUS1000104, SKMUS1000110, SKMUS1000118, SKMUS1000124, SKMUS1000129, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000365, SKMUS2000380, SKMUS2000390, SKMUS2000416, SKMUS2000458, SKMUS2000467, SKMUS2000468, SKMUS2000484, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000780, SKMUS2000847, SKMUS2000863, SKMUS2000873, SKMUS2000894, SKMUS2000898, SKMUS2000902, SKMUS2000931, SKMUS2000933, SKMUS2000945, SKMUS2000961, SKMUS2000973, SKMUS2000980, SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000137, SKNMC1000159, SKNMC1000264, SKNMC2000224, SKNMC2000256, SKNMC2000356, SKNMC2000374, SKNMC2000583, SKNMC2000593, SKNMC2000612, SKNMC2000622, SKNMC2000966, SKNMC2001113, SKNMC2001324, SKNMC2001503, SKNMC2001555, SKNMC2001596, SKNMC2001623, SKNMC2002402, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000101, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000482, SKNSH2000516, SKNSH2000550, SKNSH2000971, SKNSH2001222, SKNSH2001875, SKNSH2001931, SKNSH2002054, SKNSH2002768, SKNSH2002866, SKNSH2003064, SKNSH2003174, SKNSH2003422, SKNSH2003466, SKNSH2003483, SKNSH2003528, SKNSH2003633, SKNSH2004039, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000137, SMINT1000192, SMINT2000007, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000396, SMINT2000400, SMINT2000454, SMINT2000518, SMINT2000529, SMINT2000530, SMINT2000537, SMINT2000538, SMINT2000541, SMINT2000545, SMINT2000558, SMINT2000562, SMINT2000568, SMINT2000602, SMINT2000609, SMINT2000629, SMINT2000659, SMINT2000694, SMINT2000747, SMINT2000811, SMINT2000824, SMINT2000933, SMINT2000984, SMINT2001114, SMINT2001158, SMINT2001183, SMINT2001199, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2001950, SMINT2002126, SMINT2002159, SMINT2002202, SMINT2002210, SMINT2002224, SMINT2002281, SMINT2002311, SMINT2002314, SMINT2002414, SMINT2002457, SMINT2002620, SMINT2002689, SMINT2002743, SMINT2002778, SMINT2002880, SMINT2002882, SMINT2002884, SMINT2002899, SMINT2002974, SMINT2002976, SMINT2003003, SMINT2003128, SMINT2003150, SMINT2003169, SMINT2003317, SMINT2003340, SMINT2003386, SMINT2003423, SMINT2003505, SMINT2003569, SMINT2003641, SMINT2003644, SMINT2003866, SMINT2003905, SMINT2004023, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008960, SMINT2009119, SMINT2009121, SMINT2009212, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012122, SMINT2012179, SMINT2012195, SMINT2012285, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000106, SPLEN1000116, SPLEN1000134, SPLEN1000139, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000255, SPLEN2000258, SPLEN2000267, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000698, SPLEN2000839, SPLEN2000874, SPLEN2000882, SPLEN2001135, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002477, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004124, SPLEN2004148, SPLEN2004181, SPLEN2004220, SPLEN2004343, SPLEN2004346, SPLEN2004368, SPLEN2004611, SPLEN2004662, SPLEN2004844, SPLEN2004880, SPLEN2004984, SPLEN2005009, SPLEN2005227, SPLEN2005272, SPLEN2005342, SPLEN2005416, SPLEN2005429, SPLEN2005450, SPLEN2005474, SPLEN2005560, SPLEN2005727, SPLEN2005783, SPLEN2005790, SPLEN2005806, SPLEN2005818, SPLEN2005838, SPLEN2005869, SPLEN2005927, SPLEN2005939, SPLEN2006122, SPLEN2006143, SPLEN2006232, SPLEN2006268, SPLEN2006283, SPLEN2006305, SPLEN2006325, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496, SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011737, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012524, SPLEN2012525, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015310, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016241, SPLEN2016250, SPLEN2016268, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019102, SPLEN2019131, SPLEN2019257, SPLEN2019311, SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027113, SPLEN2027157, SPLEN2027395, SPLEN2027488, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2039311, SPLEN2039379, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000148, STOMA2000168, STOMA2000183, STOMA2000188, STOMA2000257, STOMA2000279, STOMA2000289, STOMA2000383, STOMA2000386, STOMA2000395, STOMA2000396, STOMA2000478, STOMA2000529, STOMA2000539, STOMA2000567, STOMA2000640, STOMA2000678, STOMA2000686, STOMA2000888, STOMA2001025, STOMA2001389, STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001033, SYNOV2001035, SYNOV2001088, SYNOV2001111, SYNOV2001144, SYNOV2001251, SYNOV2001262, SYNOV2001300, SYNOV2001356, SYNOV2001374, SYNOV2001390, SYNOV2001451, SYNOV2001457, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2009172, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, T1ESE2000116, T1ESE2000609, T1ESE2002665, TBAES2000004, TBAES2000059, TBAES2000116, TBAES2000255, TBAES2000315, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000142, TESTI1000157, TESTI1000163, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001120, TESTI2001134, TESTI2001141, TESTI2001153, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003454, TESTI2003475, TESTI2003476, TESTI2003498, TESTI2003512, TESTI2003533, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004031, TESTI2004073, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005759, TESTI2005775, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006588, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007320, TESTI2007346, TESTI2007358, TESTI2007402, TESTI2007405, TESTI2007407, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009412, TESTI2009447, TESTI2009462, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015213, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000029, THYMU1000032, THYMU1000033, THYMU1000060, THYMU1000074, THYMU1000083, THYMU1000098, THYMU1000103, THYMU1000105, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000331, THYMU1000336, THYMU1000359, THYMU1000366, THYMU1000382, THYMU1000393, THYMU1000394, THYMU1000399, THYMU1000426, THYMU1000440, THYMU1000459, THYMU1000473, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000436, THYMU2000440, THYMU2000489, THYMU2000492, THYMU2000519, THYMU2000570, THYMU2000602, THYMU2000660, THYMU2000671, THYMU2000672, THYMU2000702, THYMU2000767, THYMU2000775, THYMU2000800, THYMU2000879, THYMU2000932, THYMU2000950, THYMU2000971, THYMU2000982, THYMU2000990, THYMU2001007, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002841, THYMU2002852, THYMU2002910, THYMU2002950, THYMU2002983, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003446, THYMU2003470, THYMU2003479, THYMU2003499, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004108, THYMU2004111, THYMU2004123, THYMU2004139, THYMU2004152, THYMU2004169, THYMU2004284, THYMU2004320, THYMU2004336, THYMU2004356, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012024, THYMU2012073, THYMU2012113, THYMU2012273, THYMU2012401, THYMU2012492, THYMU2012507, THYMU2012565, THYMU2012574, THYMU2012625, THYMU2012631, THYMU2012665, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031368, THYMU2031579, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032798, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000030, TRACH1000038, TRACH1000057, TRACH1000063, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000496, TRACH2000497, TRACH2000502, TRACH2000522, TRACH2000540, TRACH2000559, TRACH2000660, TRACH2000665, TRACH2000675, TRACH2000677, TRACH2000702, TRACH2000703, TRACH2000767, TRACH2000775, TRACH2000780, TRACH2000807, TRACH2000873, TRACH2000894, TRACH2000898, TRACH2000920, TRACH2000923, TRACH2000944, TRACH2000959, TRACH2000981, TRACH2000986, TRACH2001037, TRACH2001085, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001347, TRACH2001395, TRACH2001400, TRACH2001403, TRACH2001416, TRACH2001443, TRACH2001463, TRACH2001474, TRACH2001478, TRACH2001523, TRACH2001525, TRACH2001532, TRACH2001549, TRACH2001592, TRACH2001596, TRACH2001621, TRACH2001646, TRACH2001651, TRACH2001684, TRACH2001898, TRACH2001933, TRACH2001968, TRACH2001996, TRACH2002043, TRACH2002054, TRACH2002092, TRACH2002100, TRACH2002138, TRACH2002333, TRACH2002664, TRACH2002784, TRACH2002840, TRACH2002847, TRACH2002853, TRACH2002862, TRACH2002954, TRACH2002960, TRACH2002988, TRACH2003070, TRACH2003272, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006870, TRACH2006918, TRACH2007324, TRACH2007358, TRACH2007483, TRACH2007542, TRACH2007577, TRACH2007674, TRACH2007676, TRACH2007688, TRACH2007733, TRACH2007754, TRACH2007894, TRACH2007969, TRACH2007988, TRACH2008081, TRACH2008112, TRACH2008113, TRACH2008127, TRACH2008130, TRACH2008278, TRACH2008300, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002192, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007541, TRACH3007595, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036942, TRACH3036997, TRACH3037063, TRACH3037067, TRACH3037267, TRACH3037288, TRACH3037505, TRACH3037573, TRACH3037696, TRACH3037897, TRACH3038086, TSTOM1000135, TSTOM1000186, TSTOM2000139, TSTOM2000235, TSTOM2000442, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000133, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU1000384, UTERU2000047, UTERU2000074, UTERU2000099, UTERU2000154, UTERU2000197, UTERU2000218, UTERU2000238, UTERU2000243, UTERU2000260, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001747, UTERU2001876, UTERU2002001, UTERU2002011, UTERU2002176, UTERU2002198, UTERU2002294, UTERU2002332, UTERU2002410, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004039, UTERU2004061, UTERU2004073, UTERU2004163, UTERU2004299, UTERU2004461, UTERU2004564, UTERU2004664, UTERU2004698, UTERU2004807, UTERU2004861, UTERU2004929, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007924, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011962, UTERU2011968, UTERU2012031, UTERU2012101, UTERU2012114, UTERU2012230, UTERU2012252, UTERU2012286, UTERU2012333, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016799, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the D3OST, D6OST, D9OST, DFNES, ERLTF, FCBBF, and FEBRA libraries were analyzed for expression frequency. The analysis results are described below. Each is a relative value, where the expression level over all tissues is taken as 100. The larger this value, the greater the level of expression.

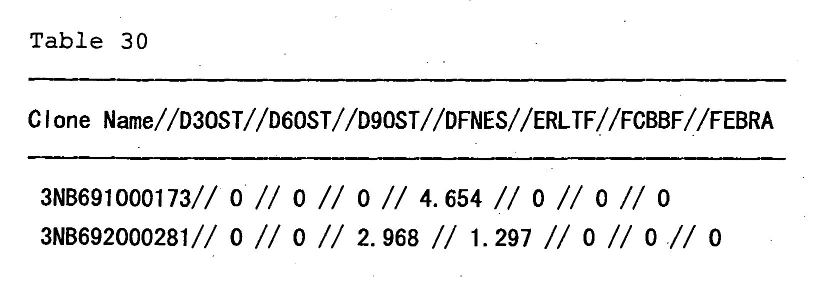

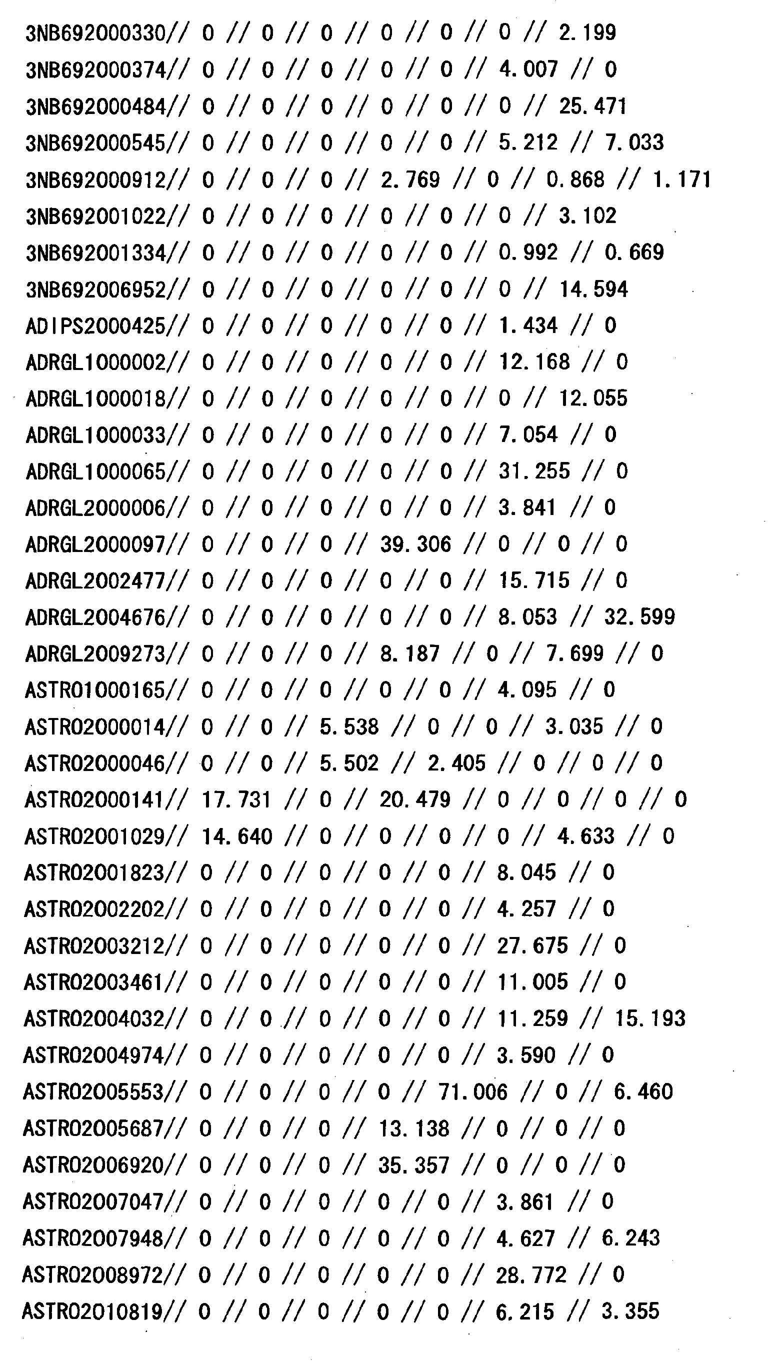

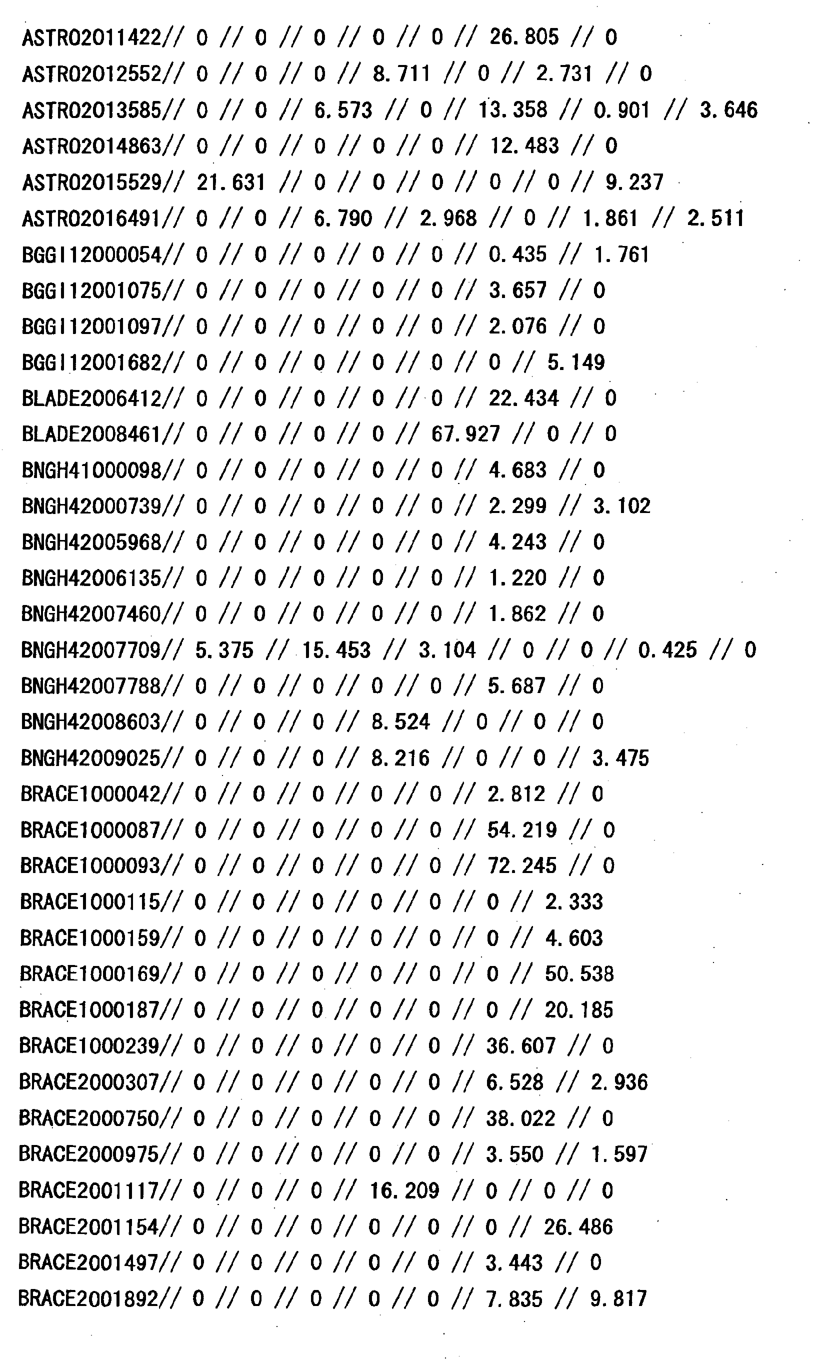

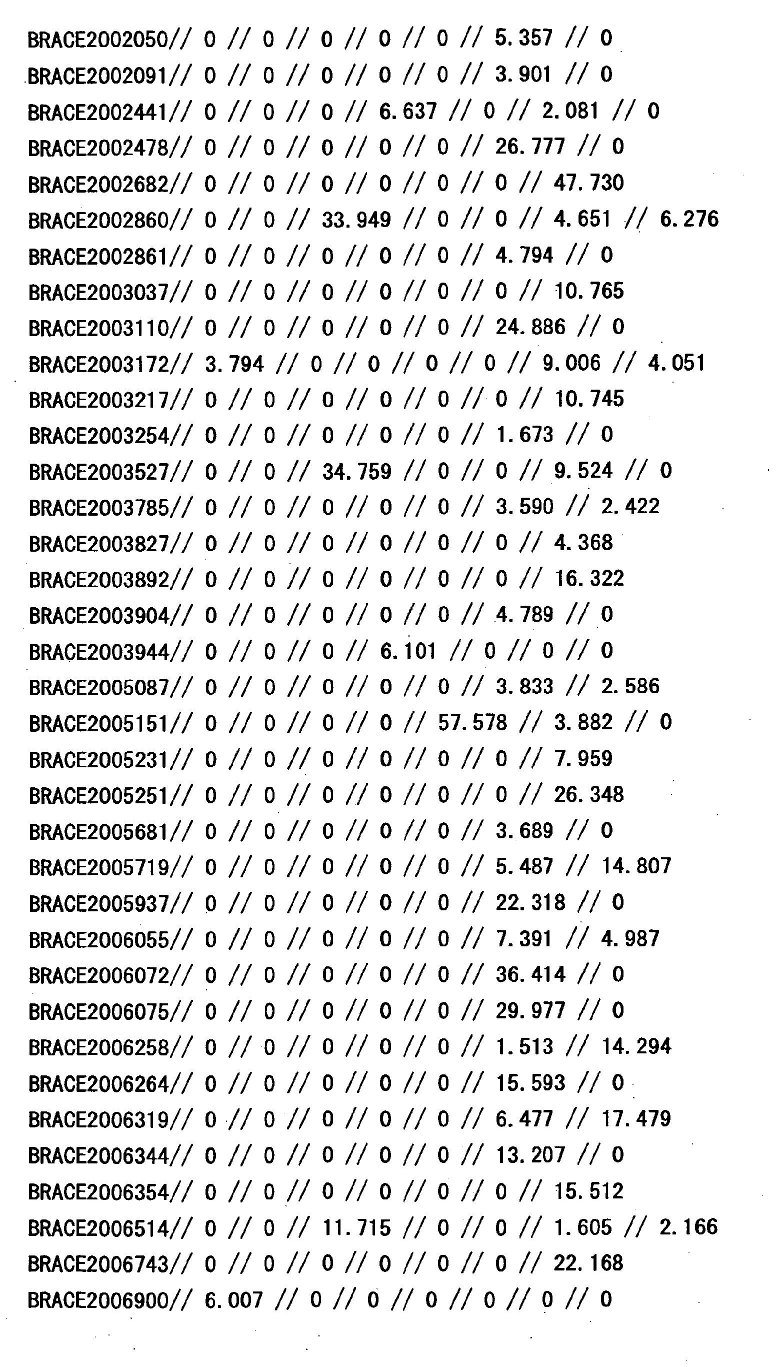

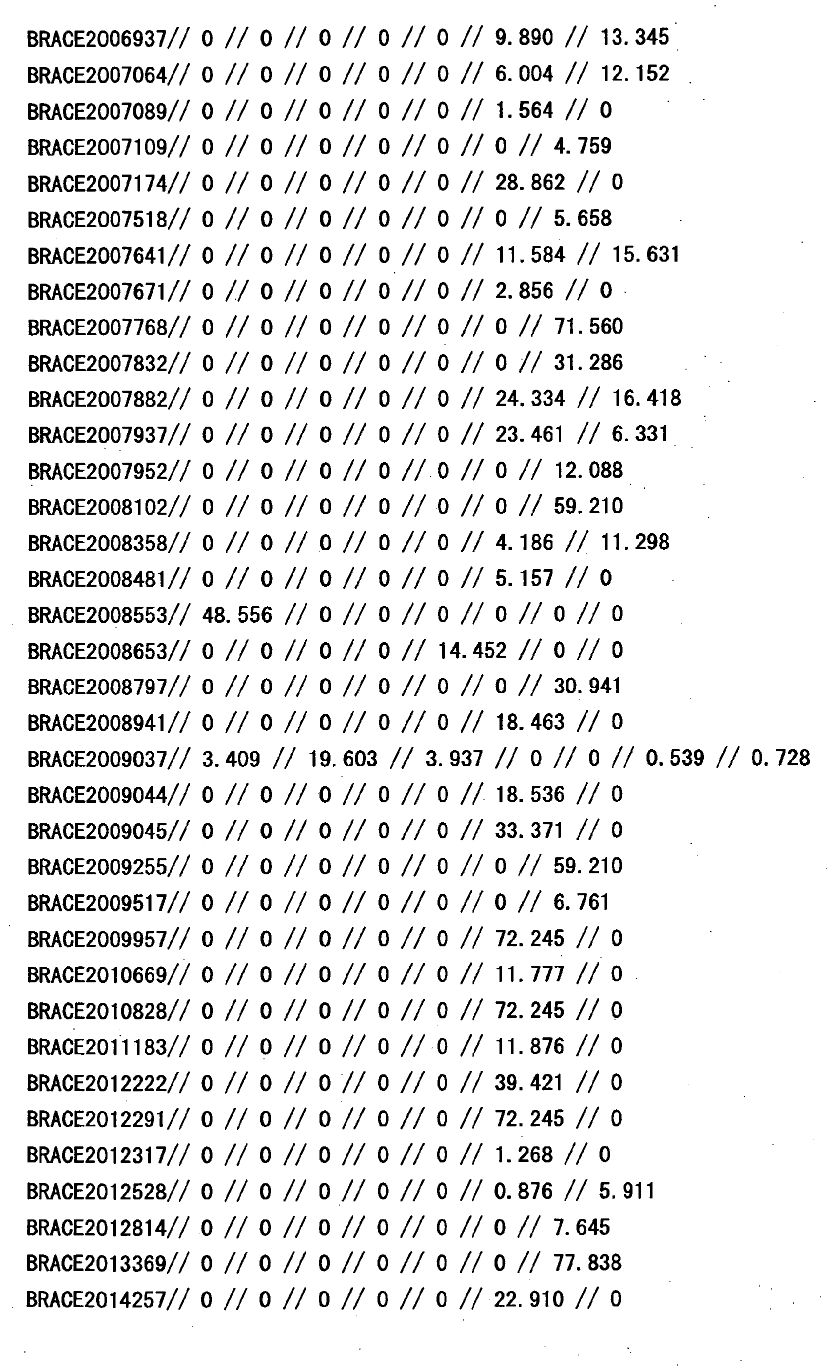

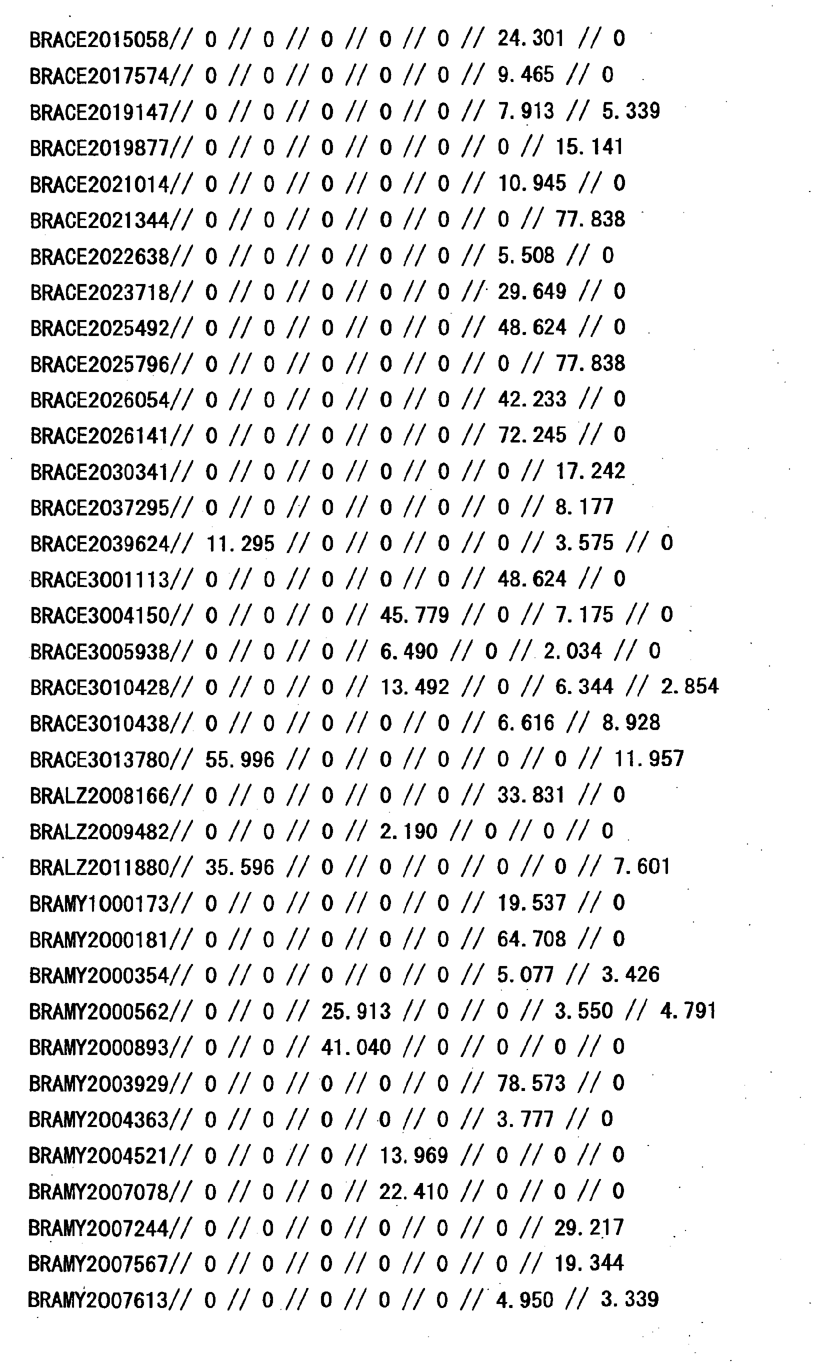

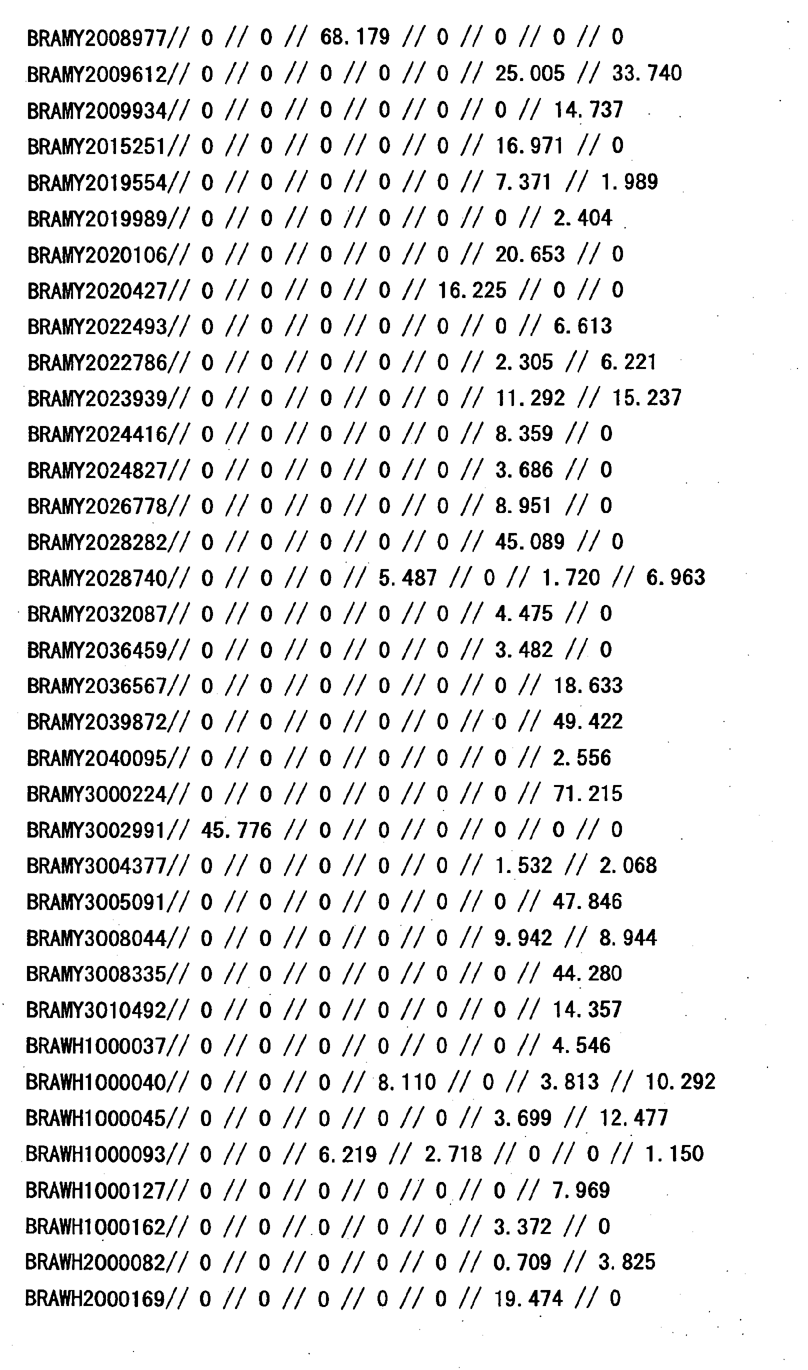

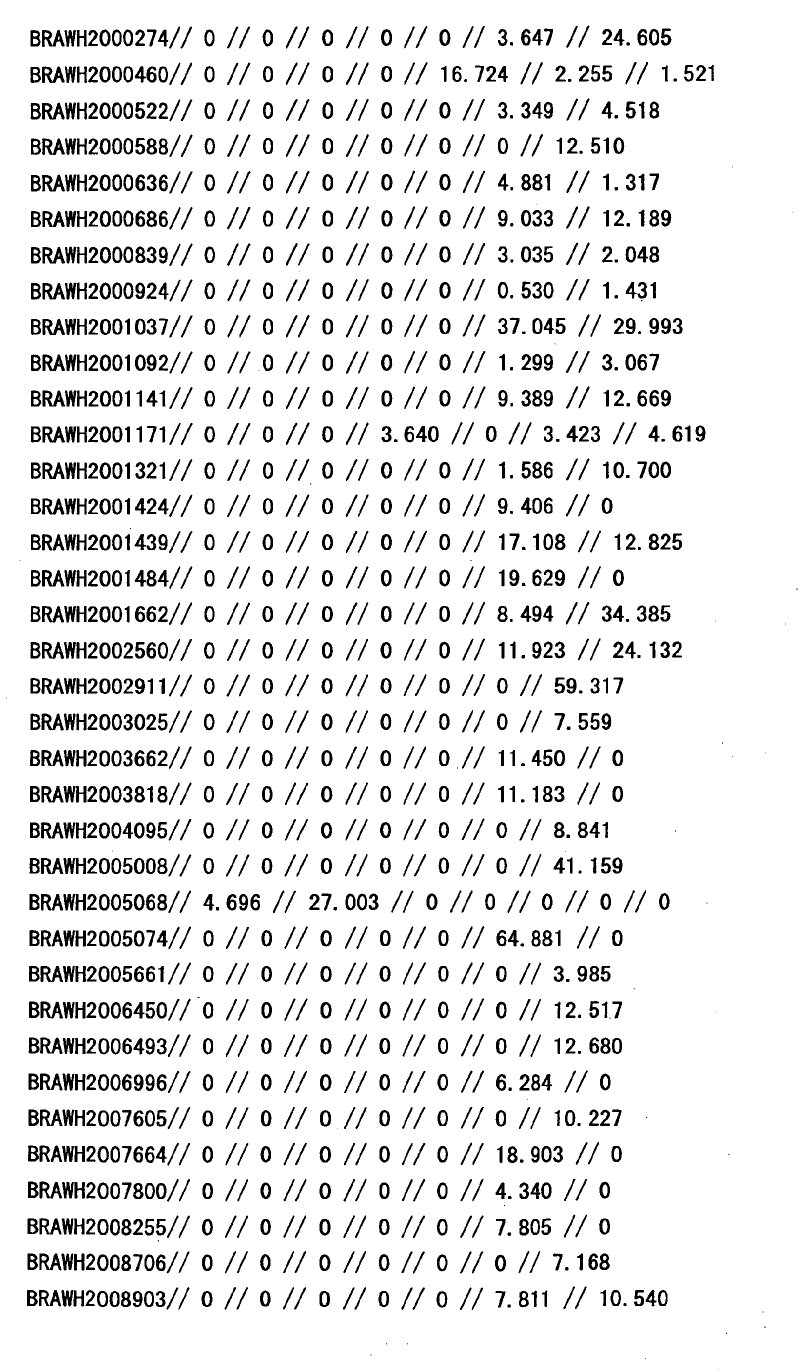

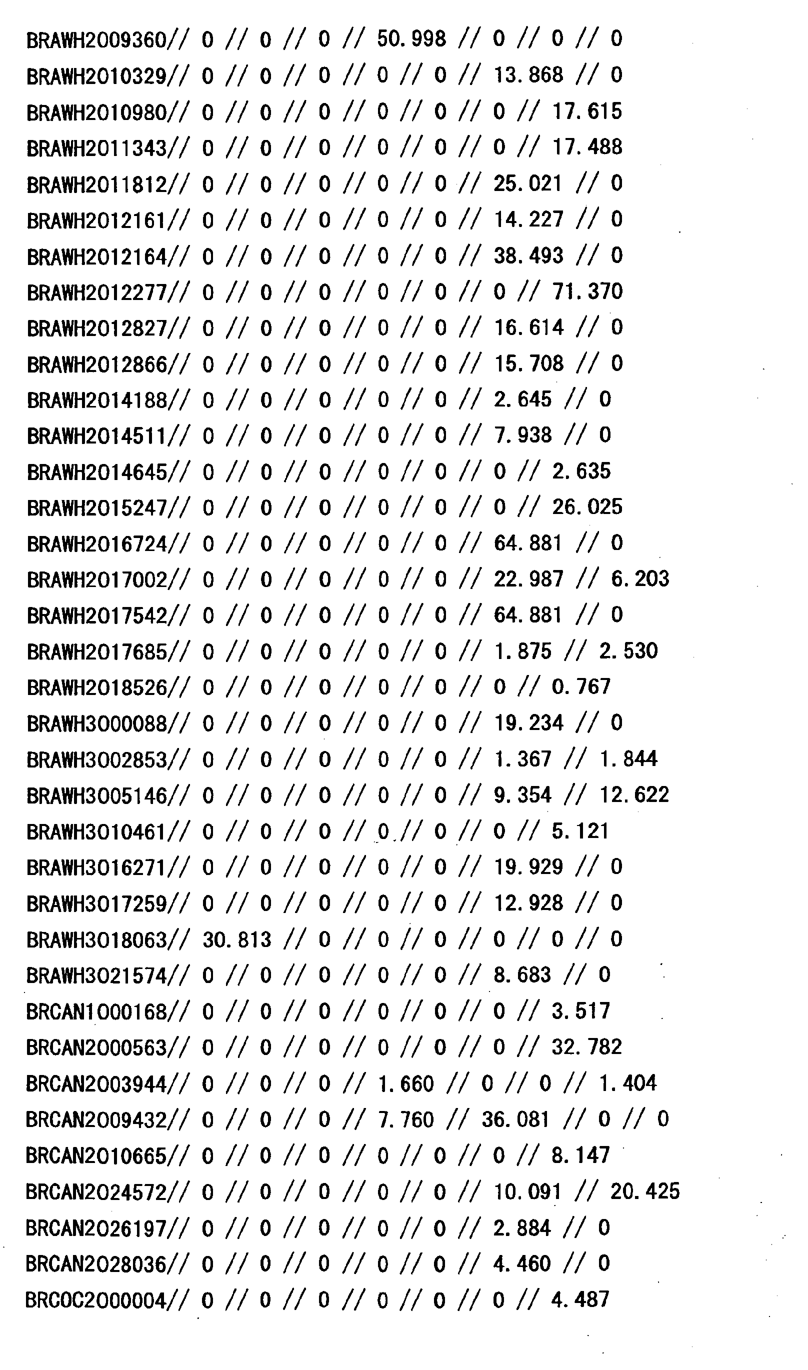

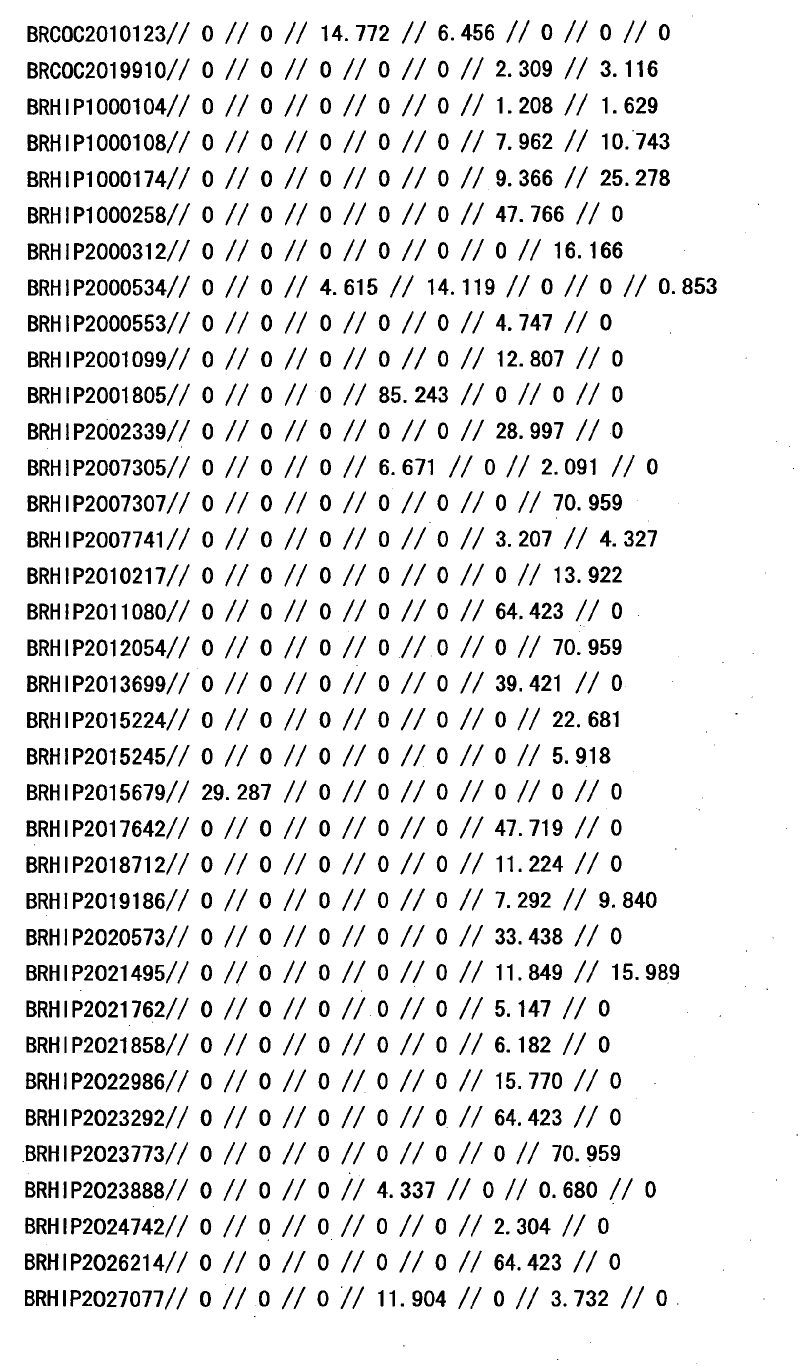

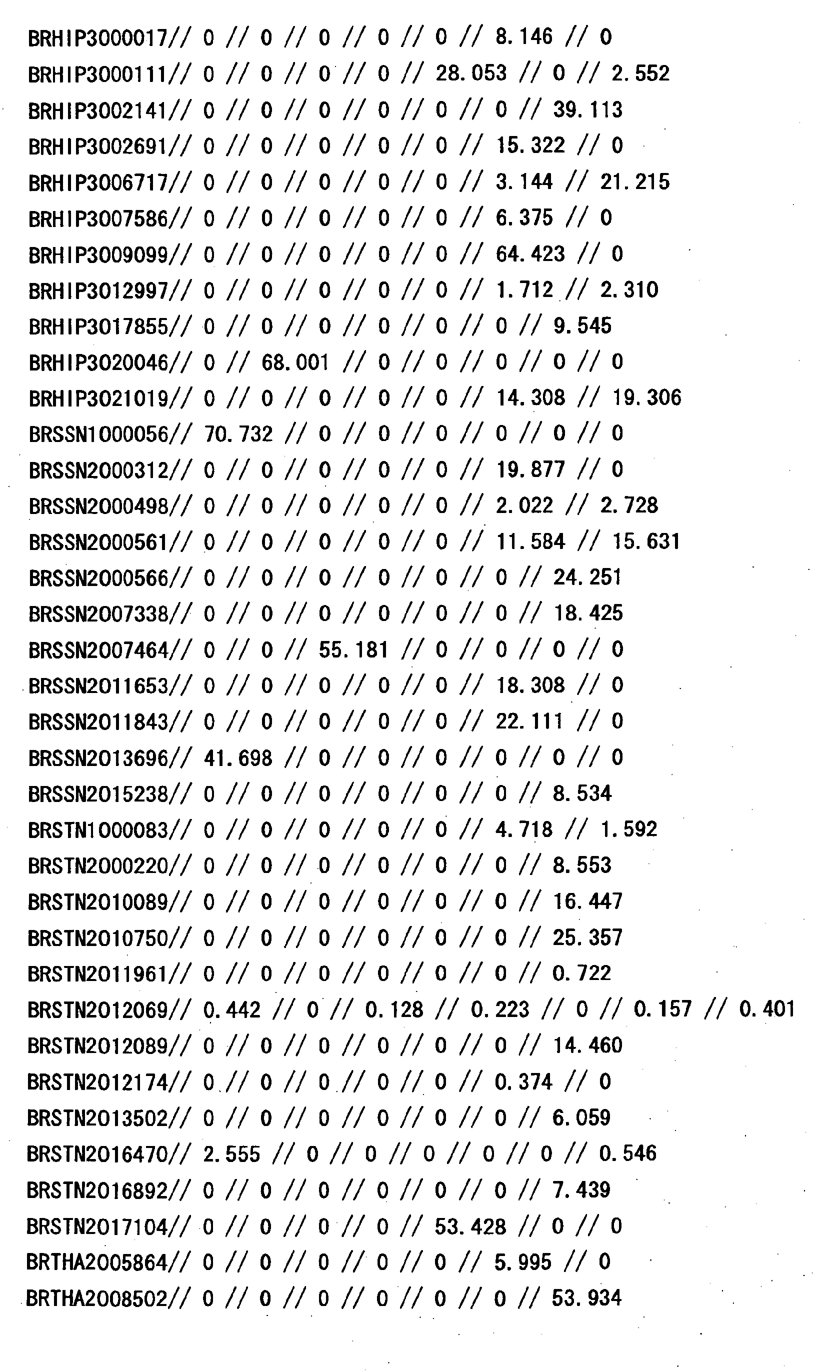

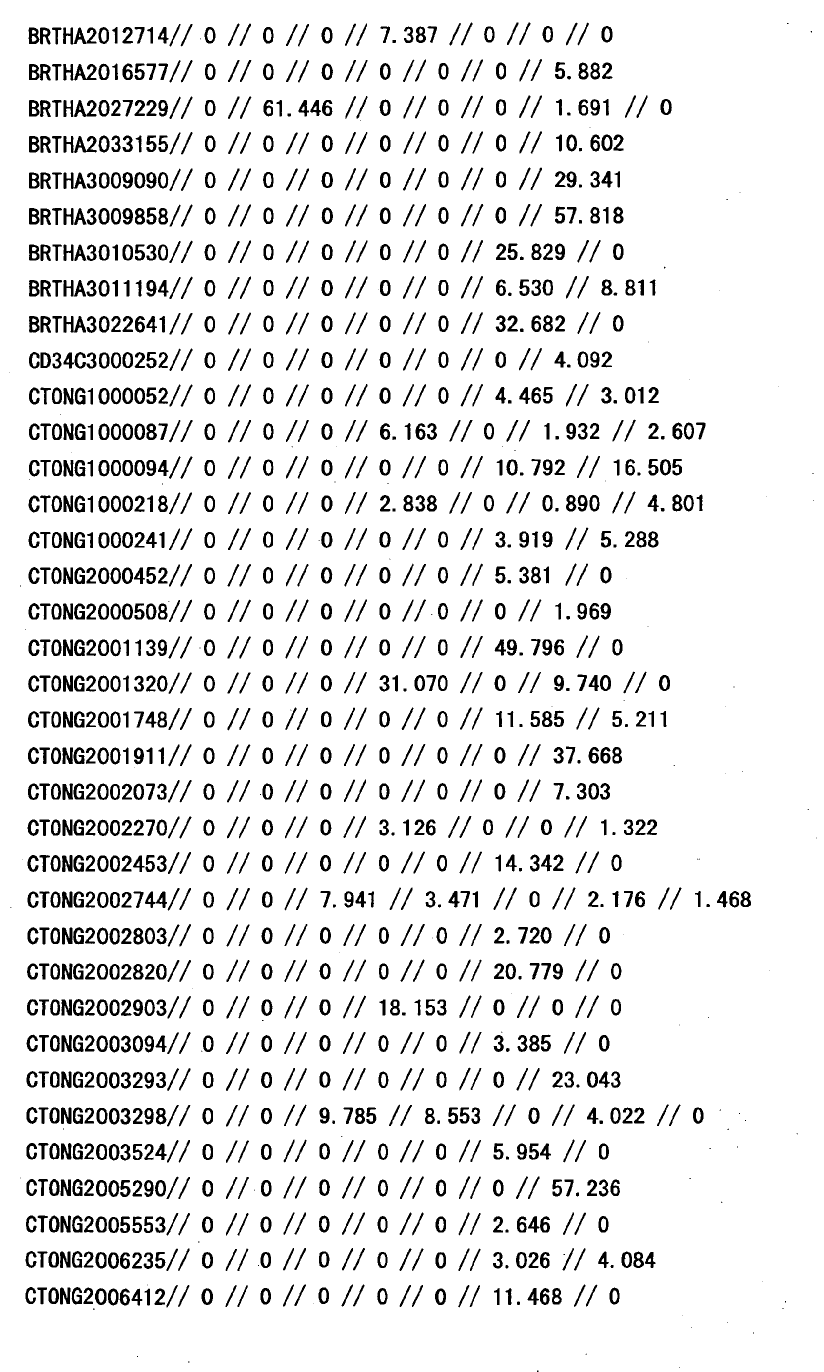

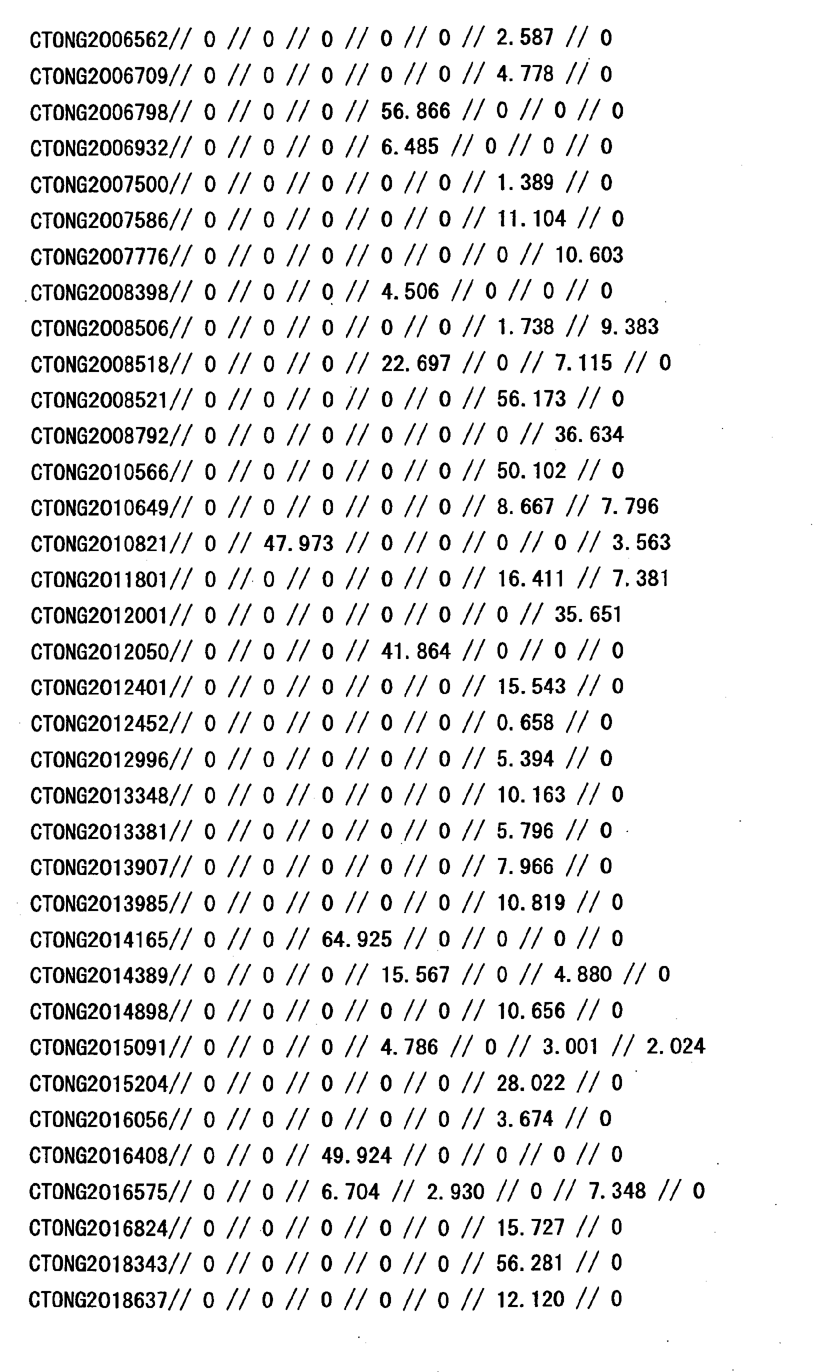

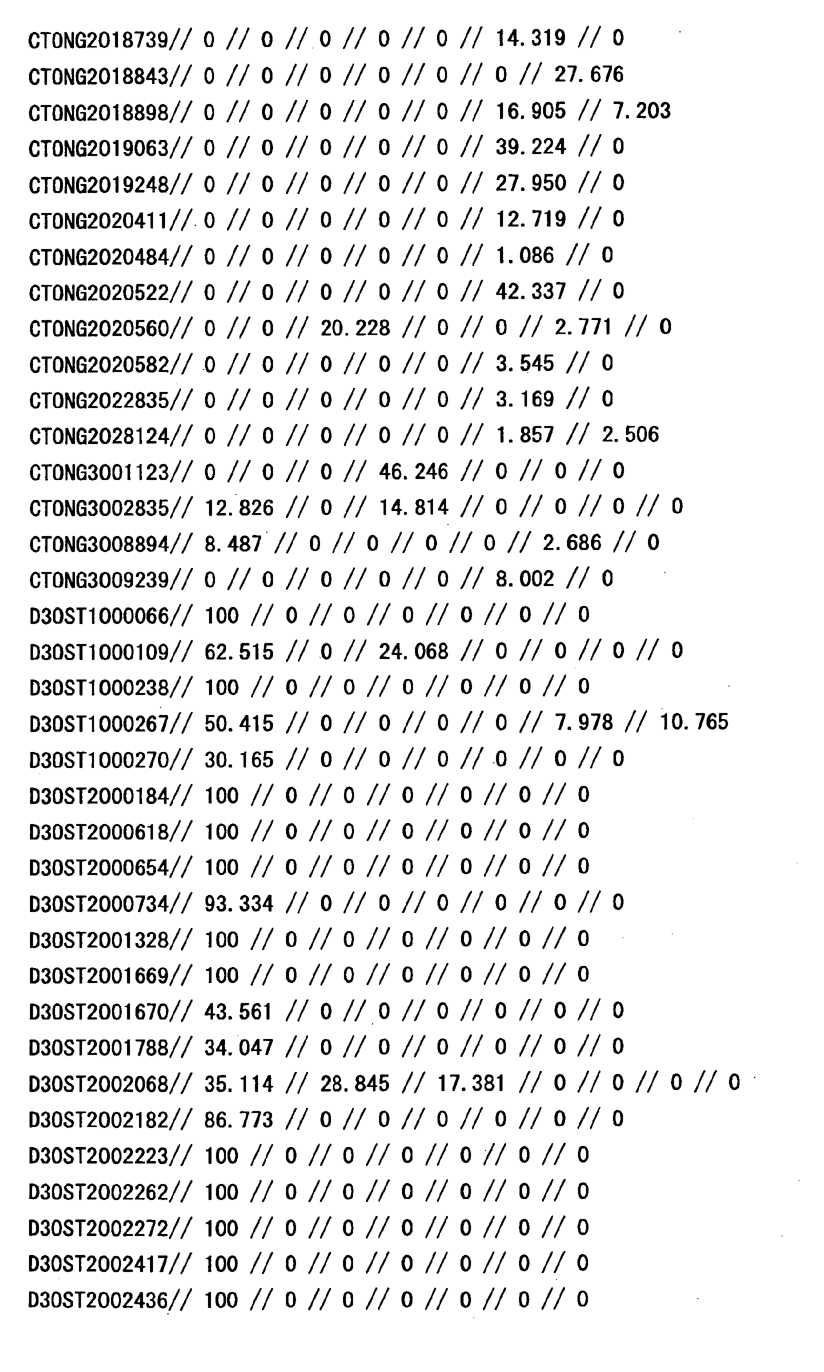

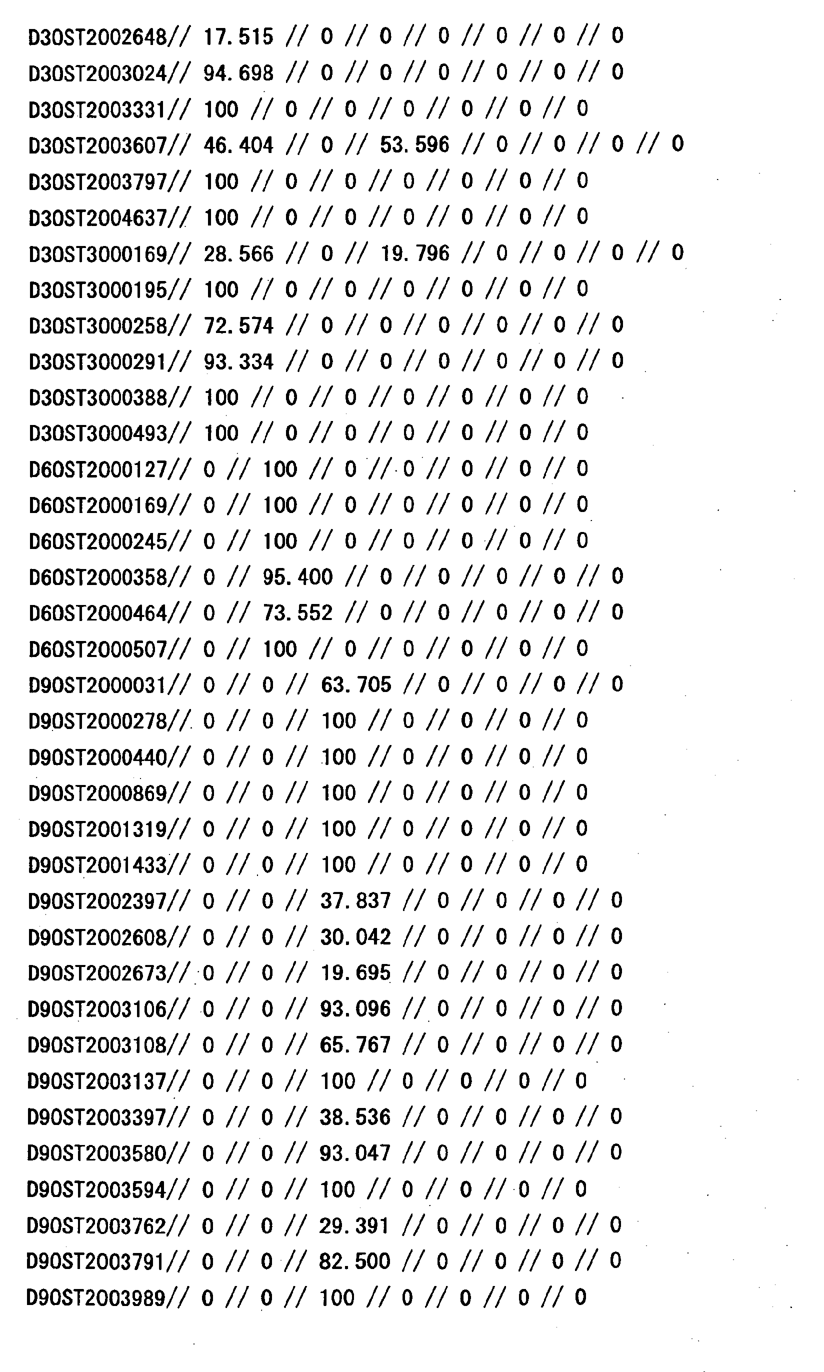

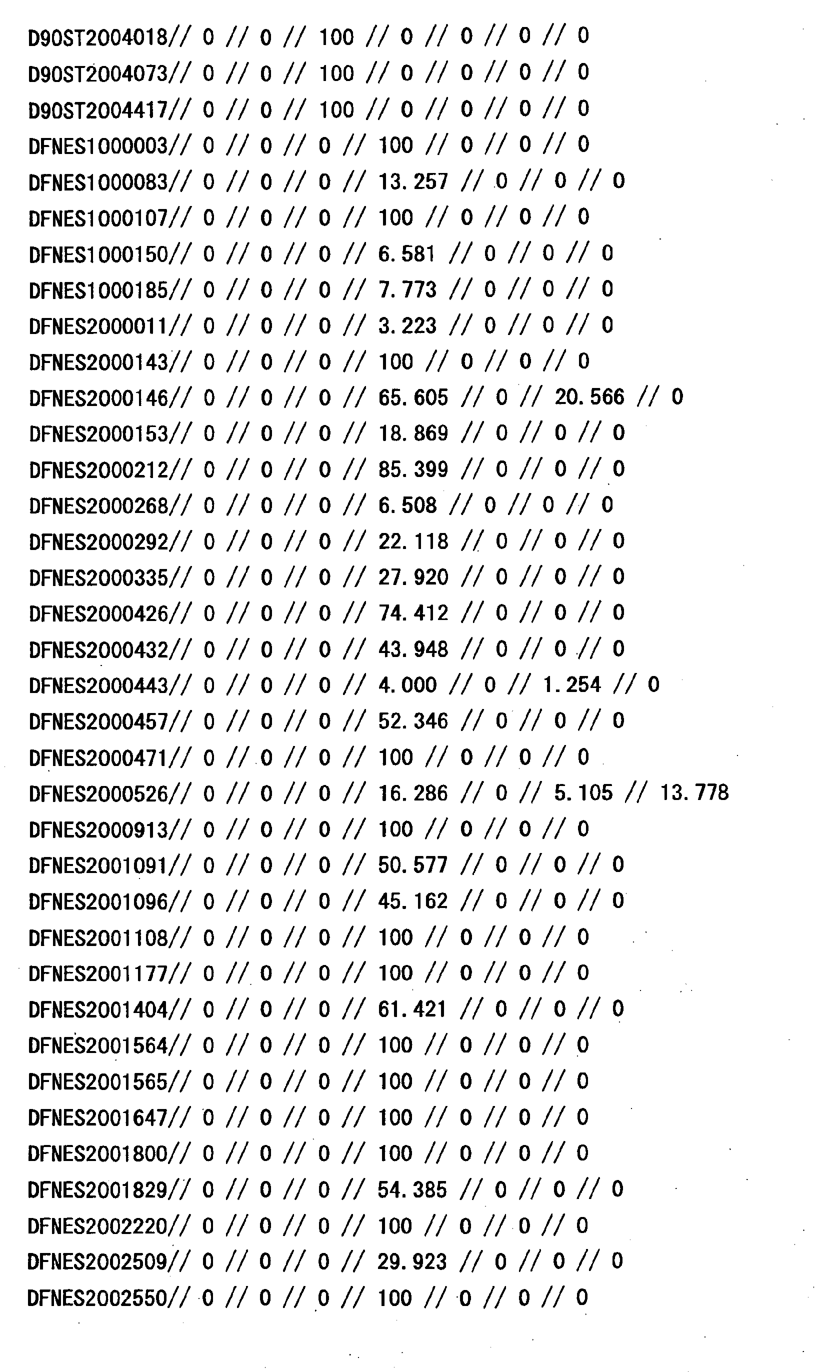

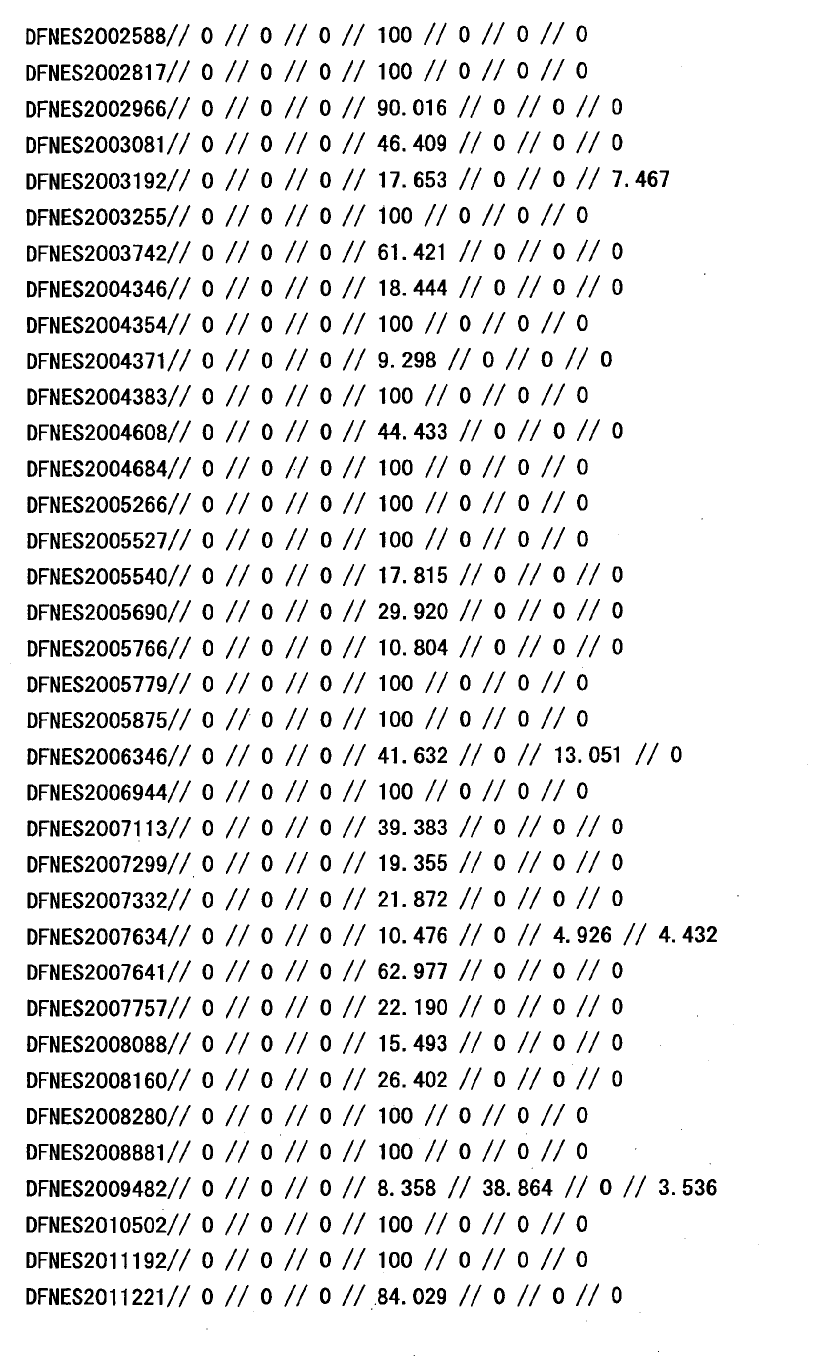

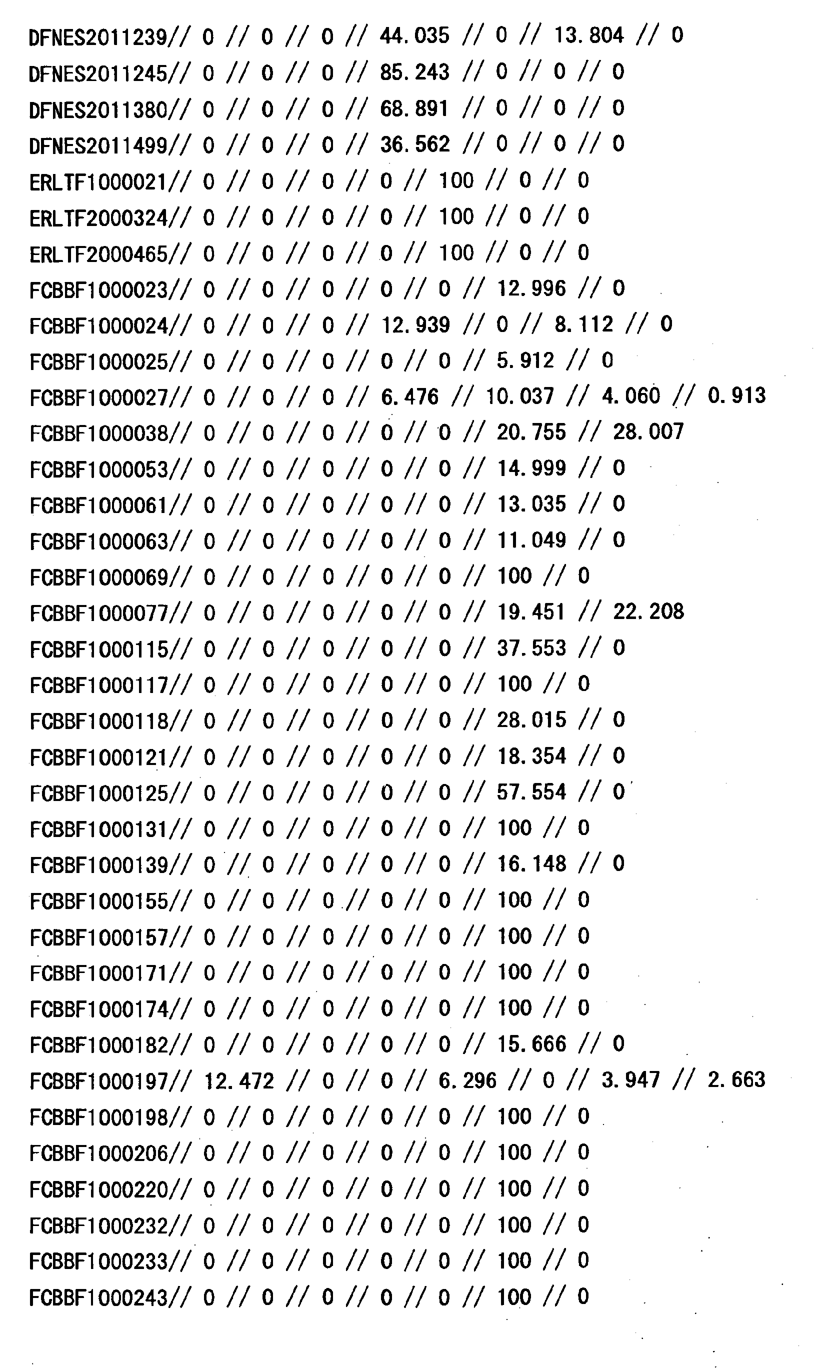

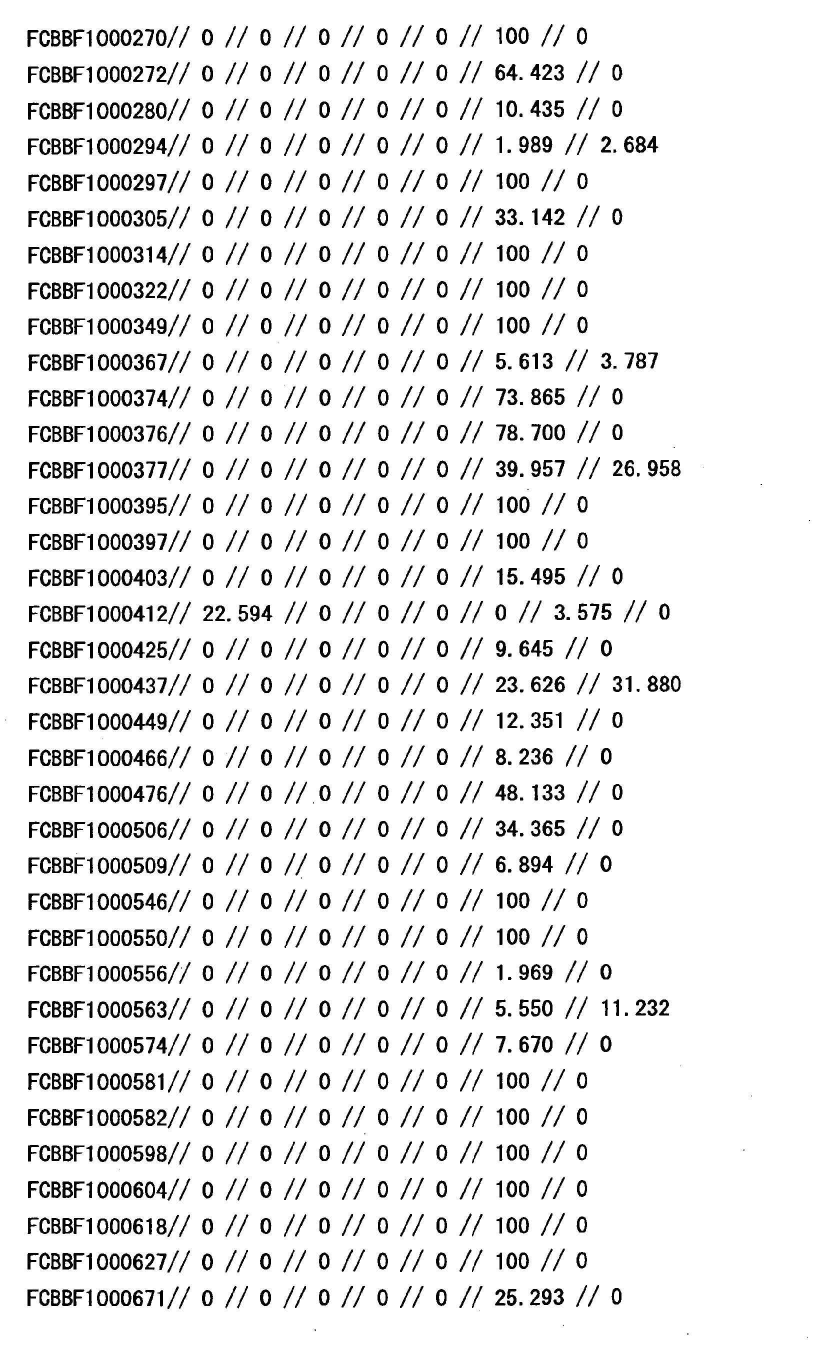

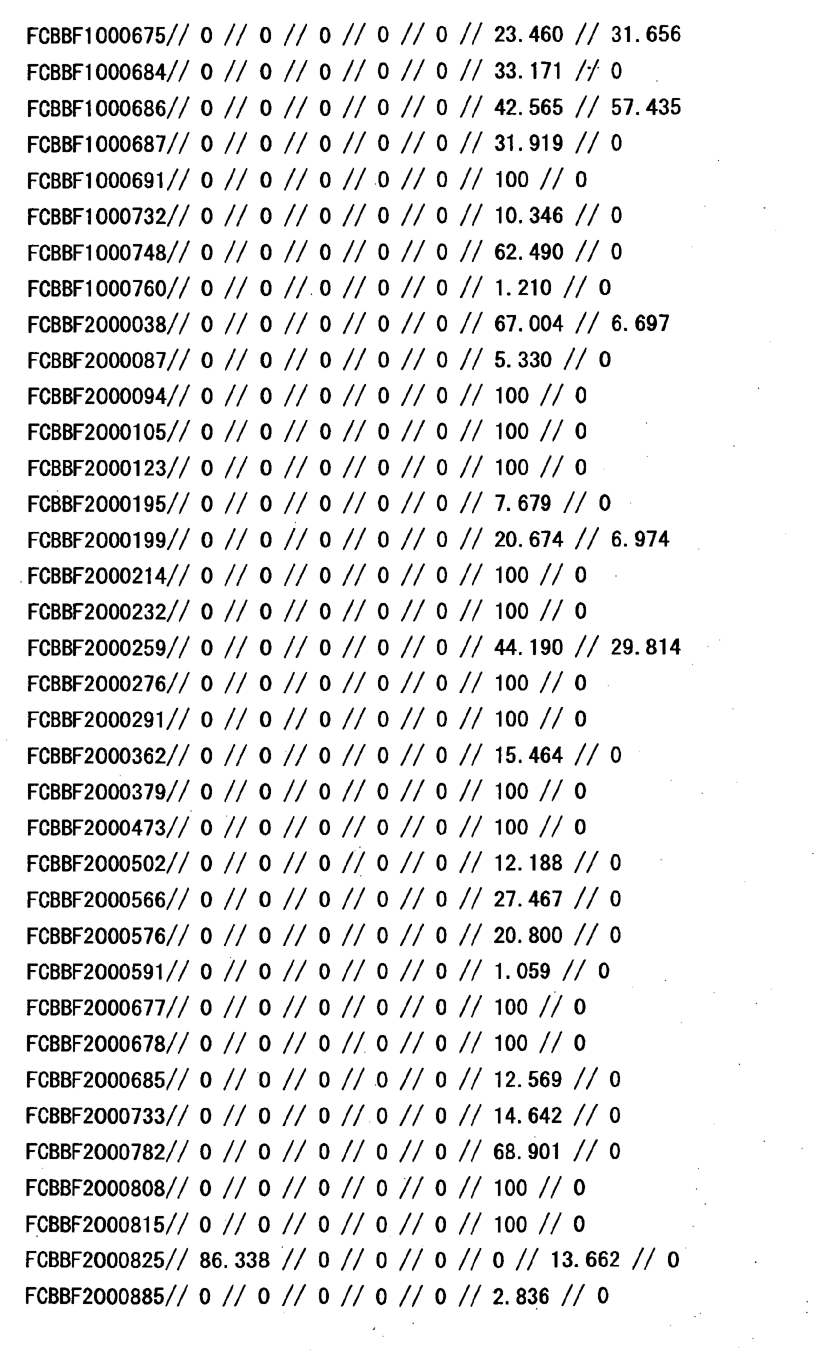

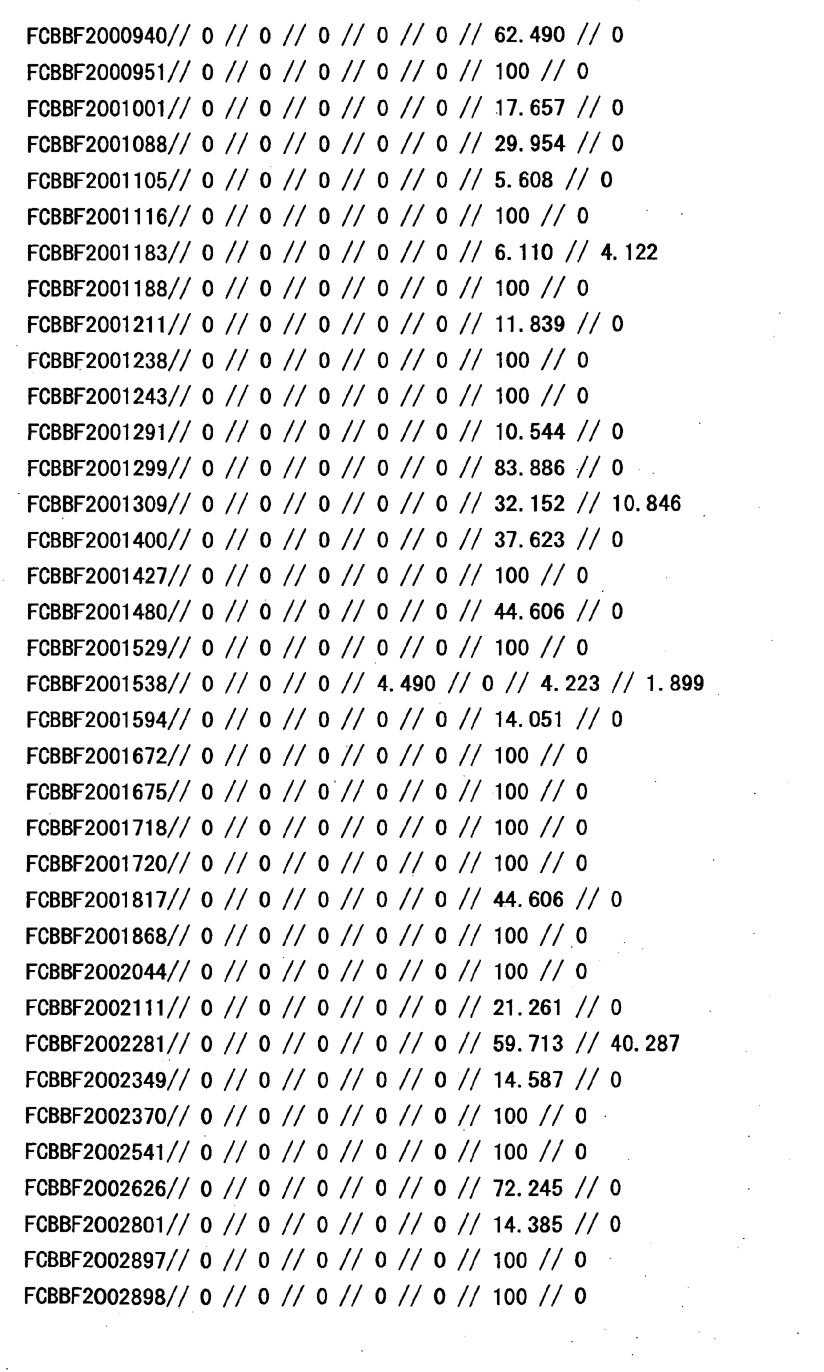

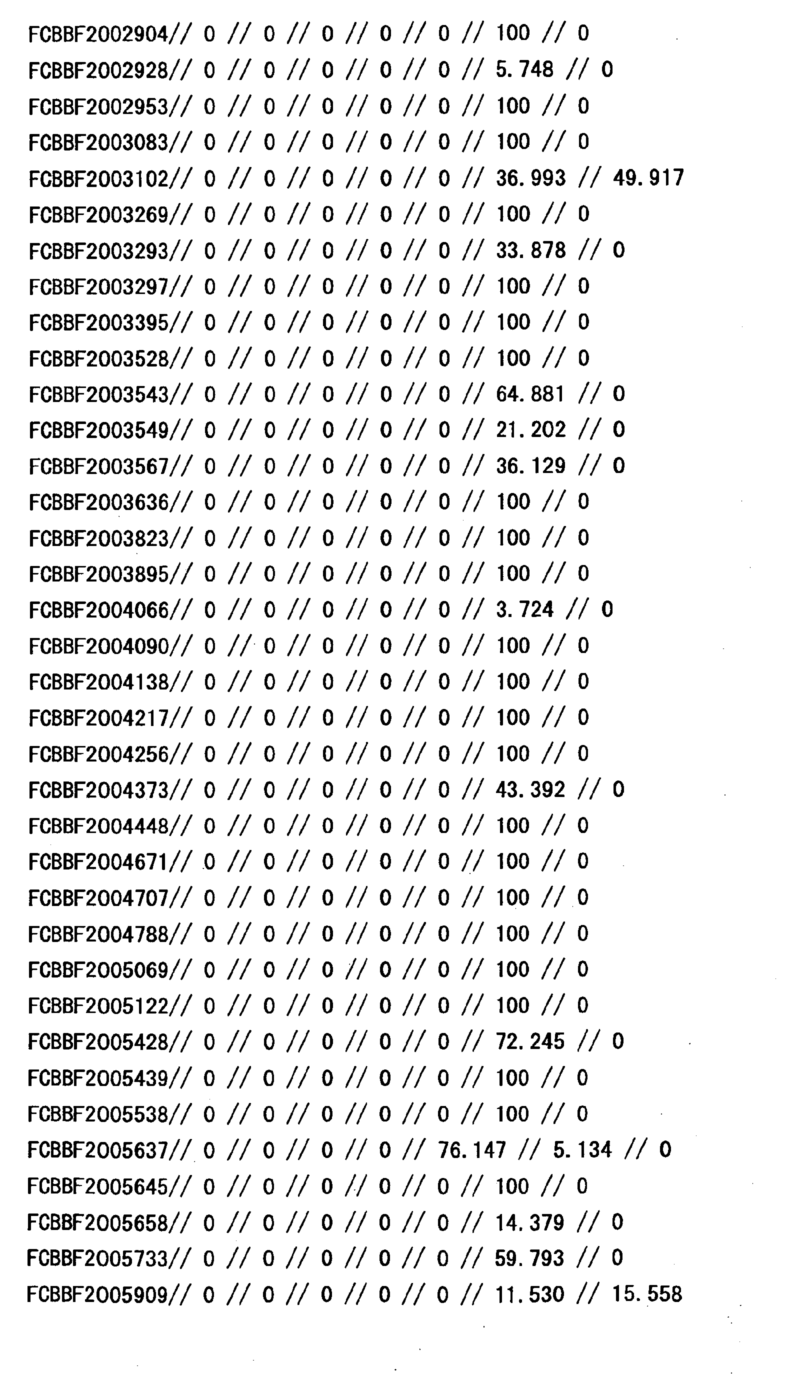

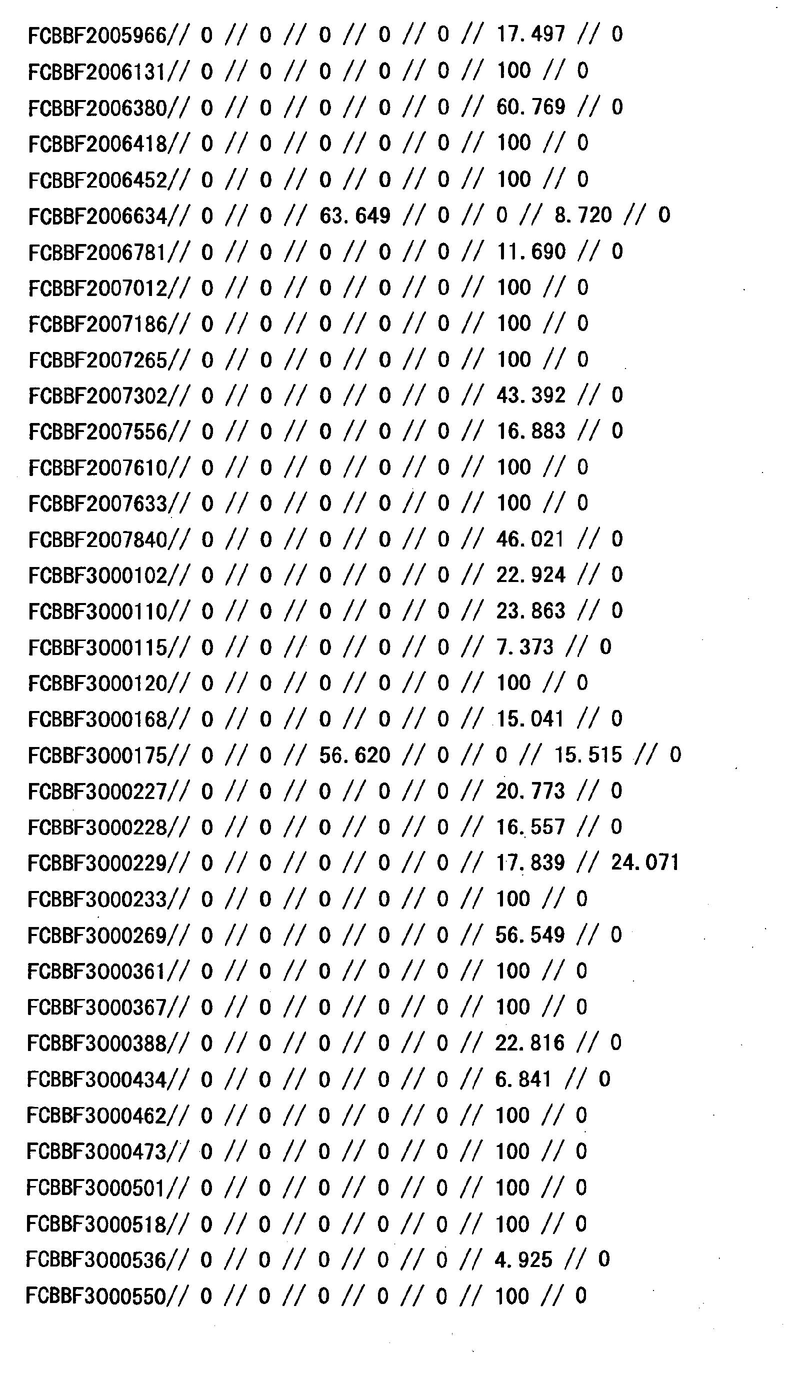

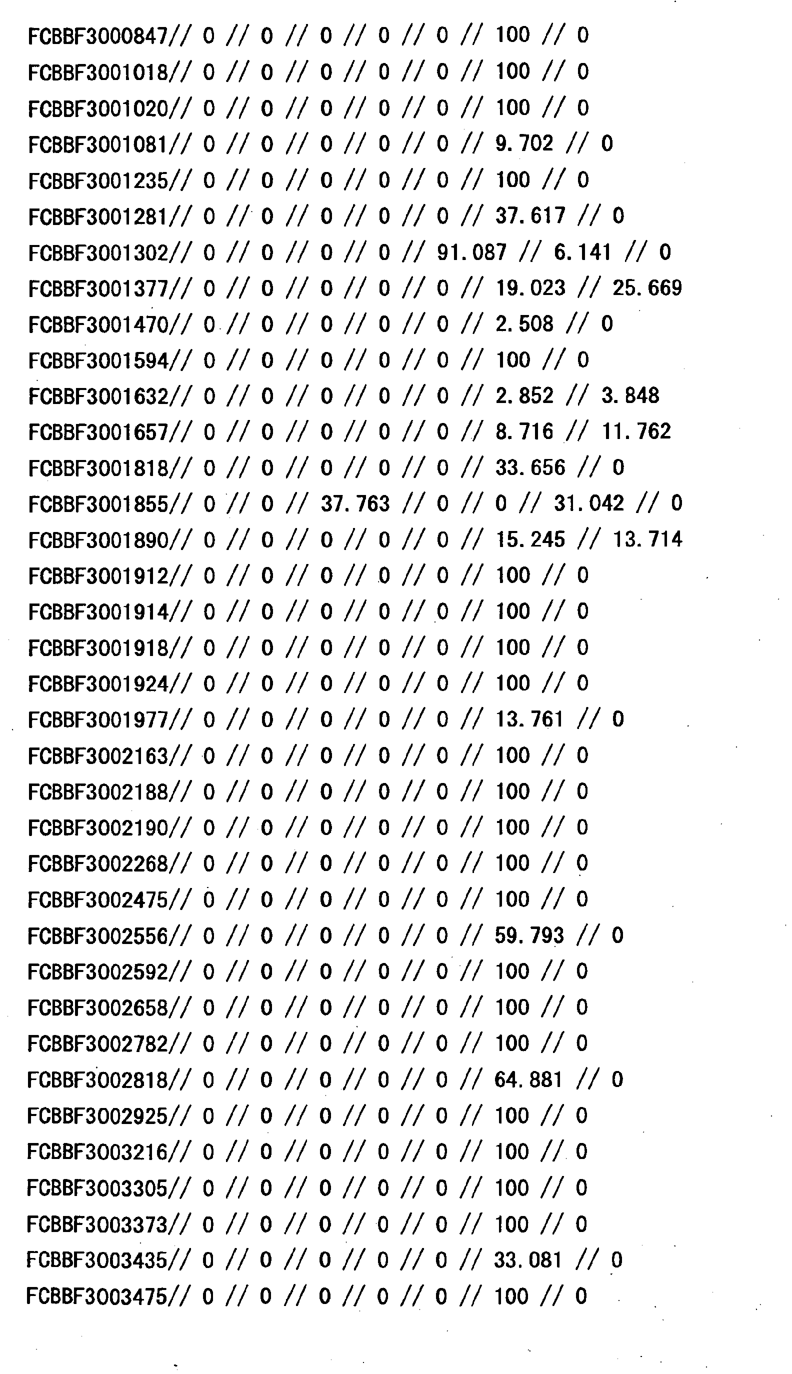

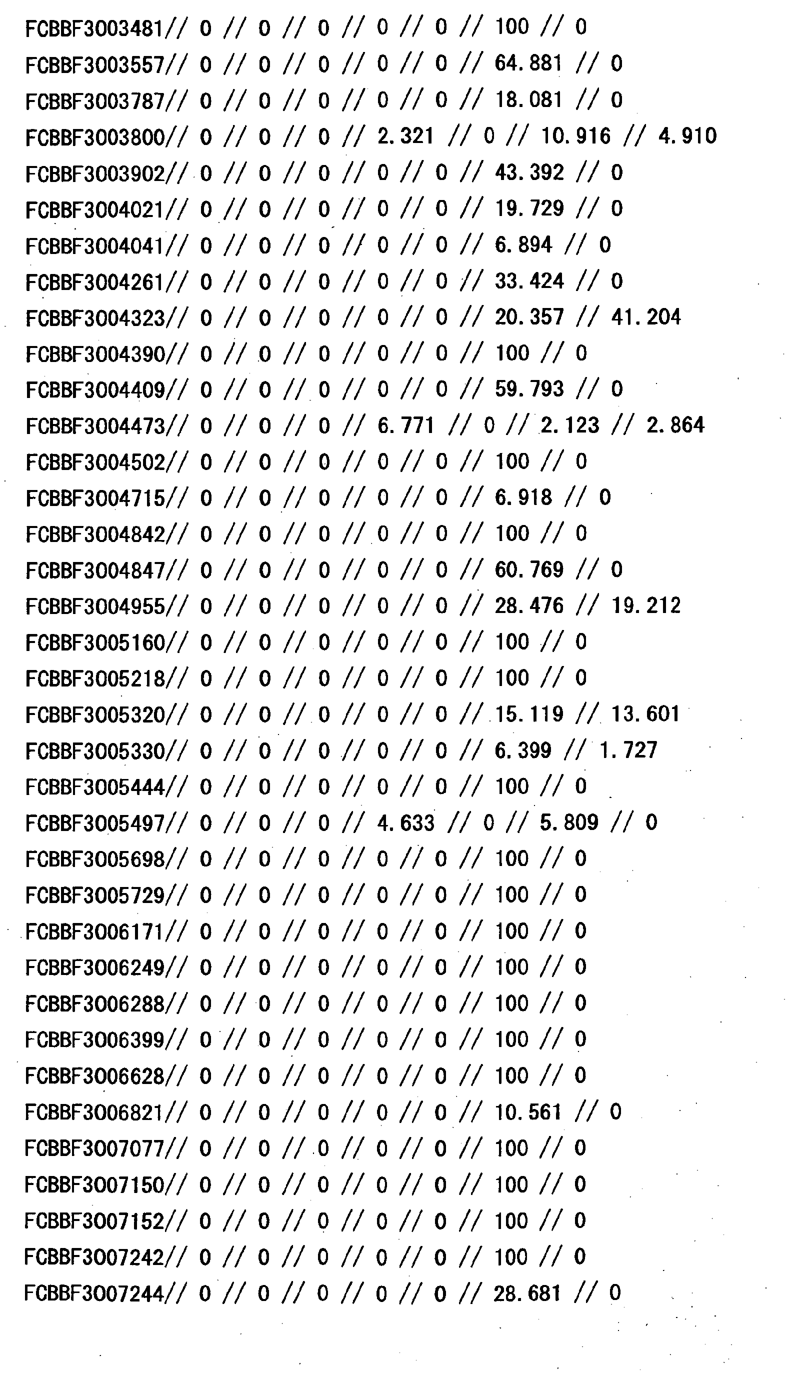

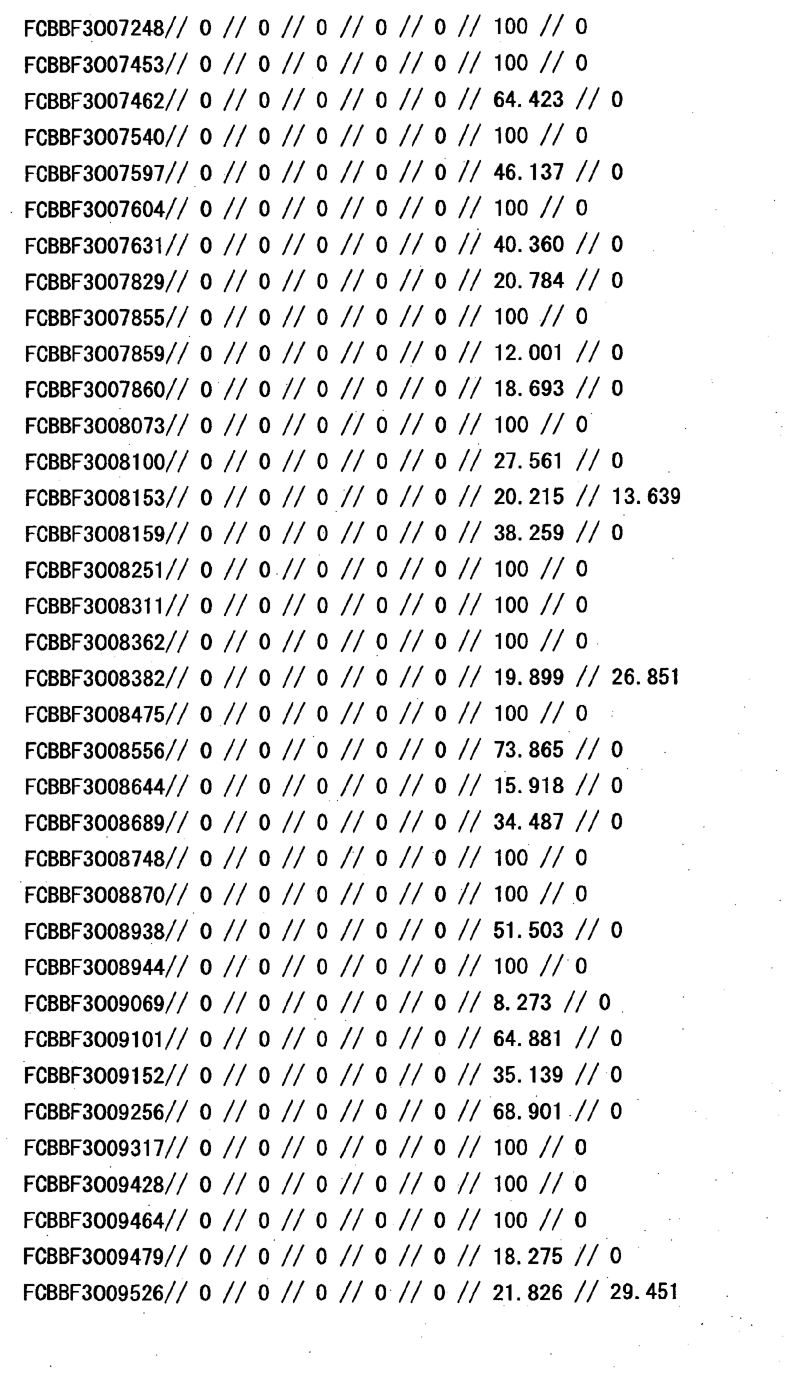

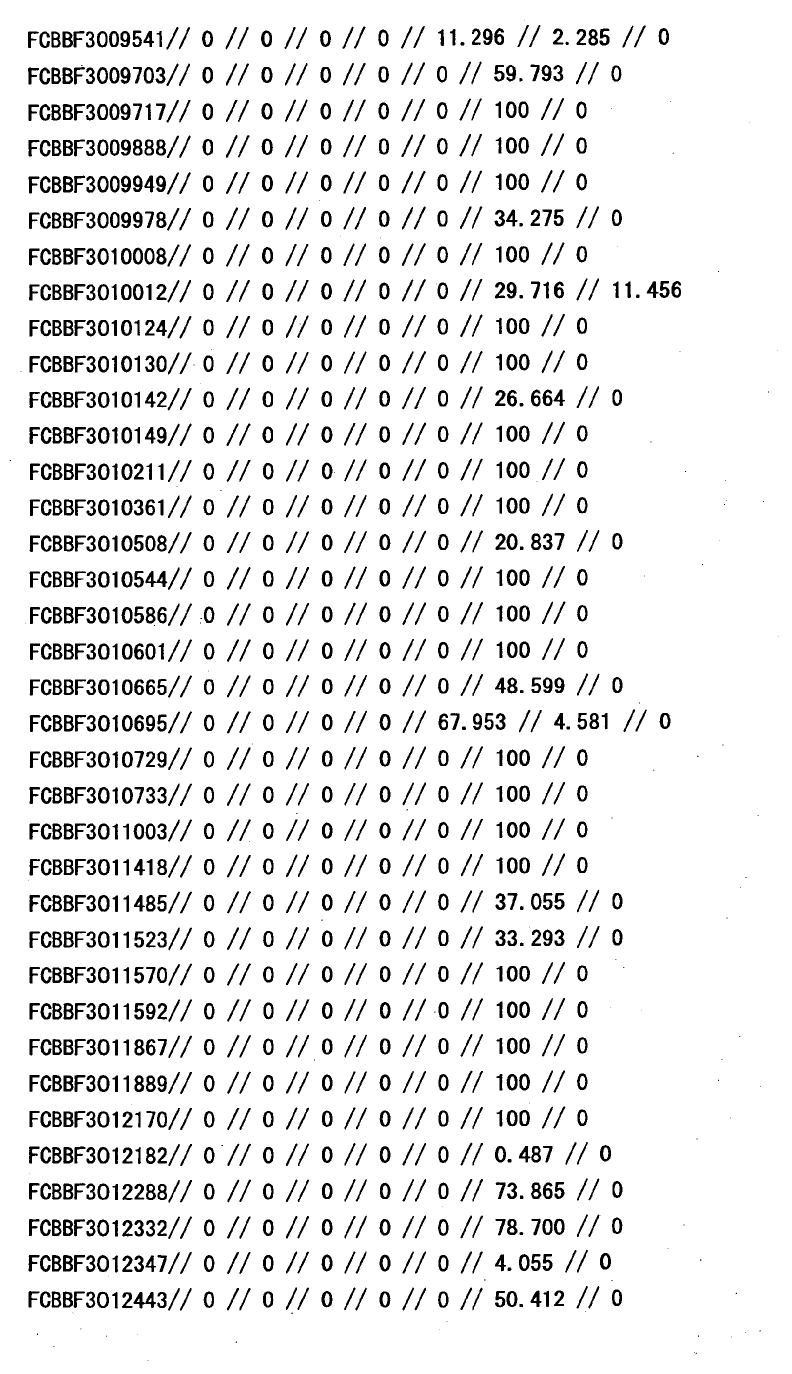

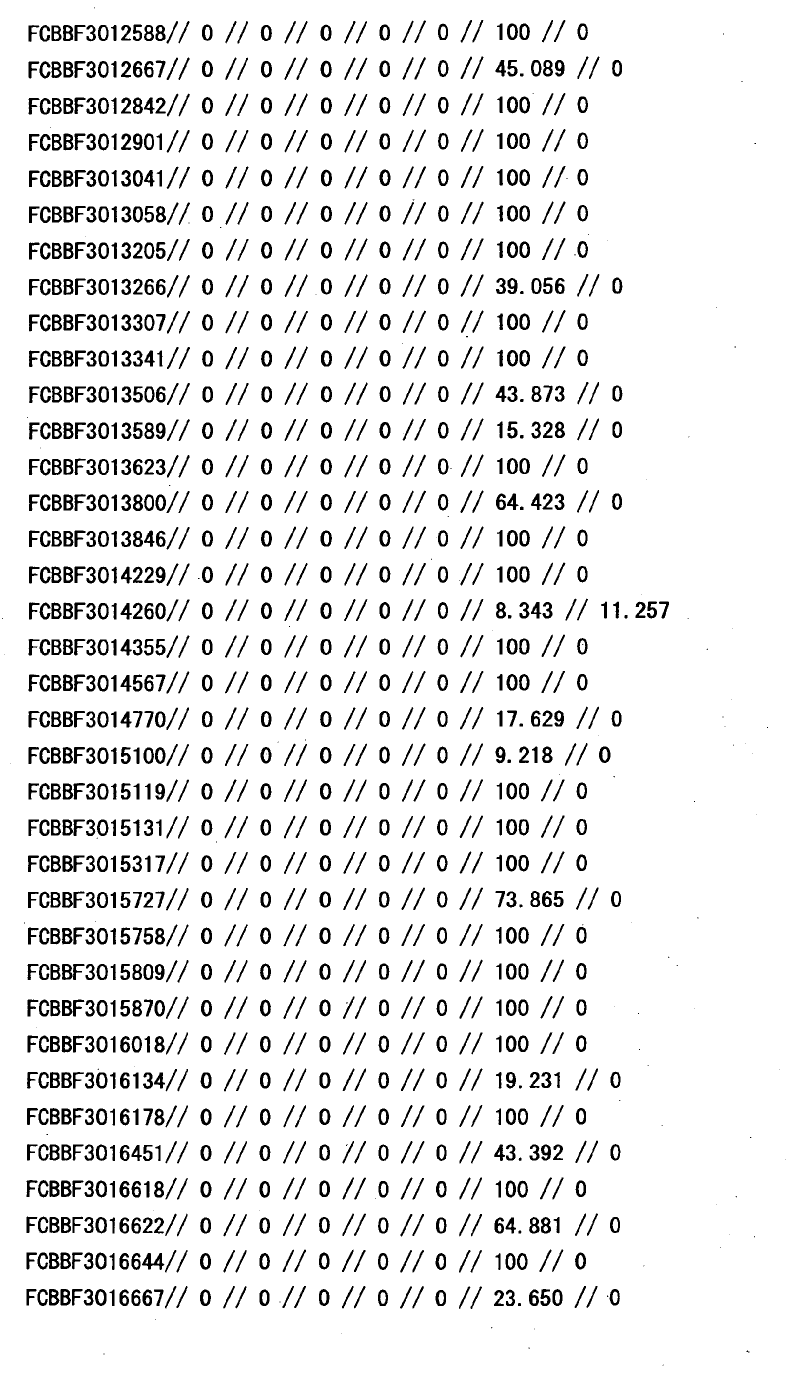

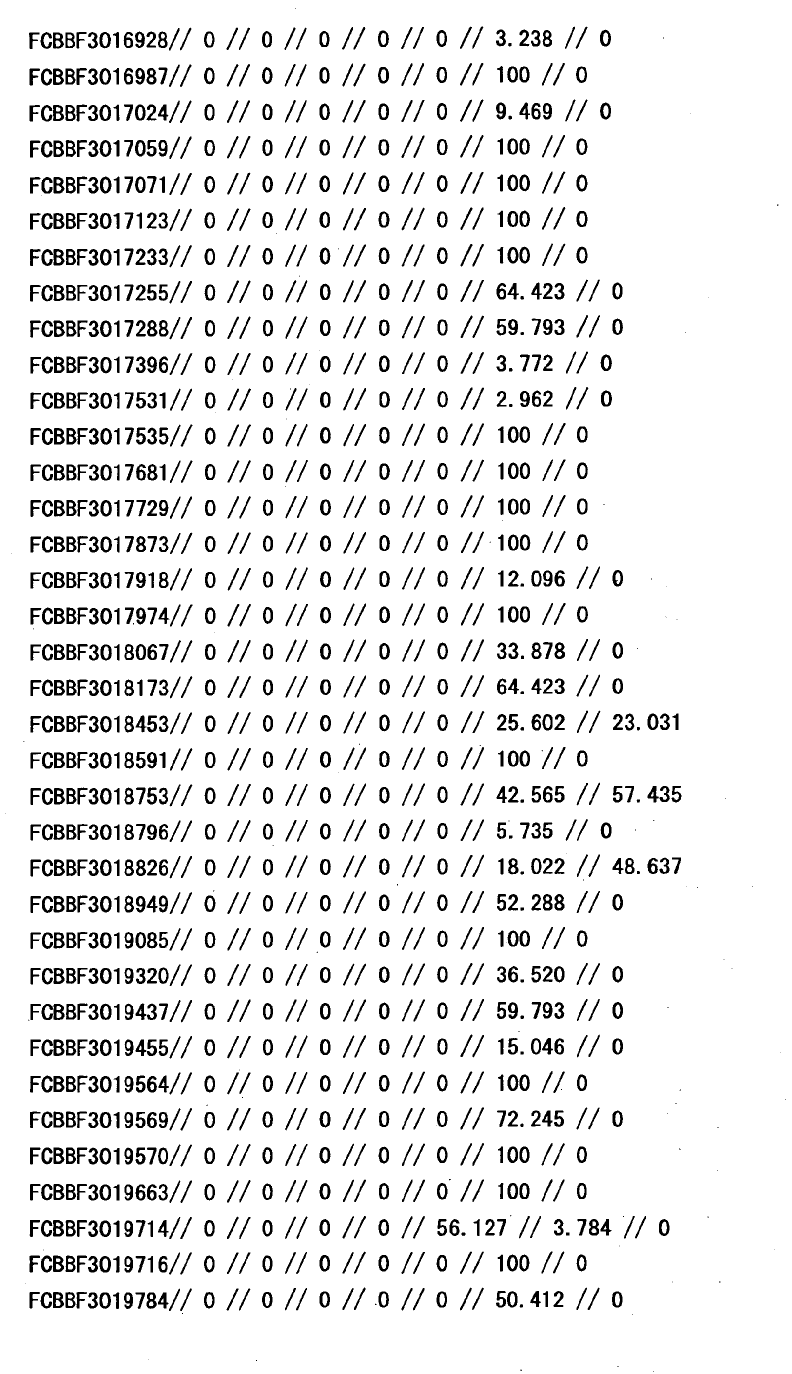

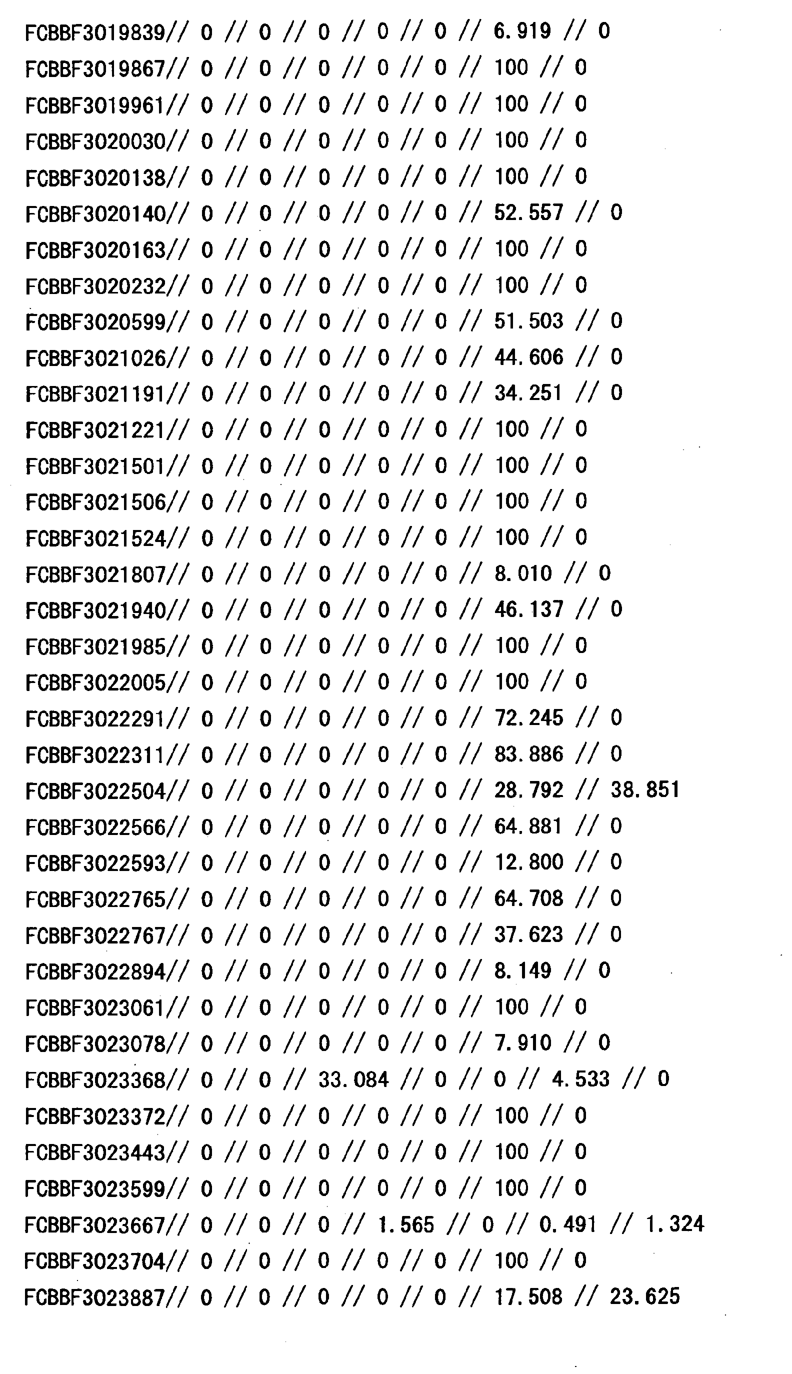

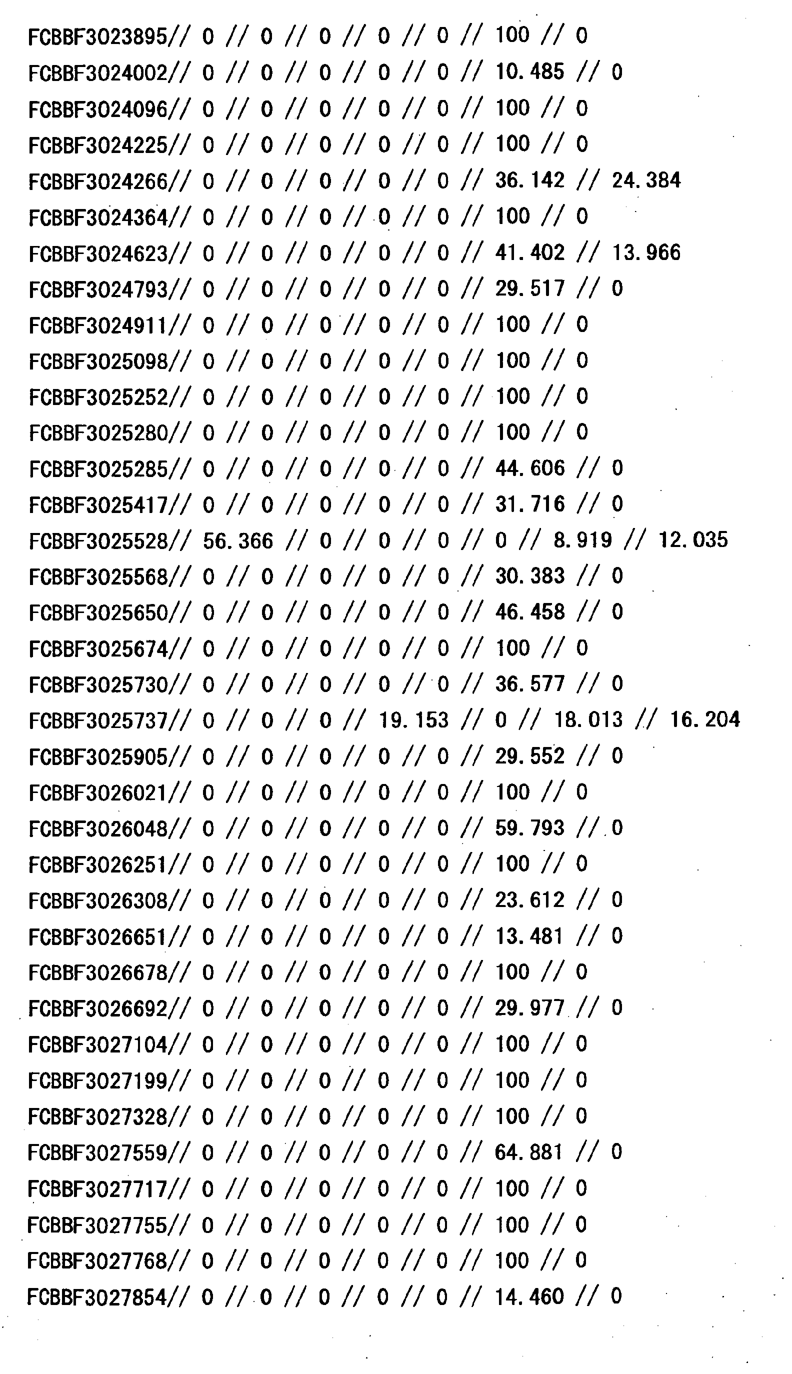

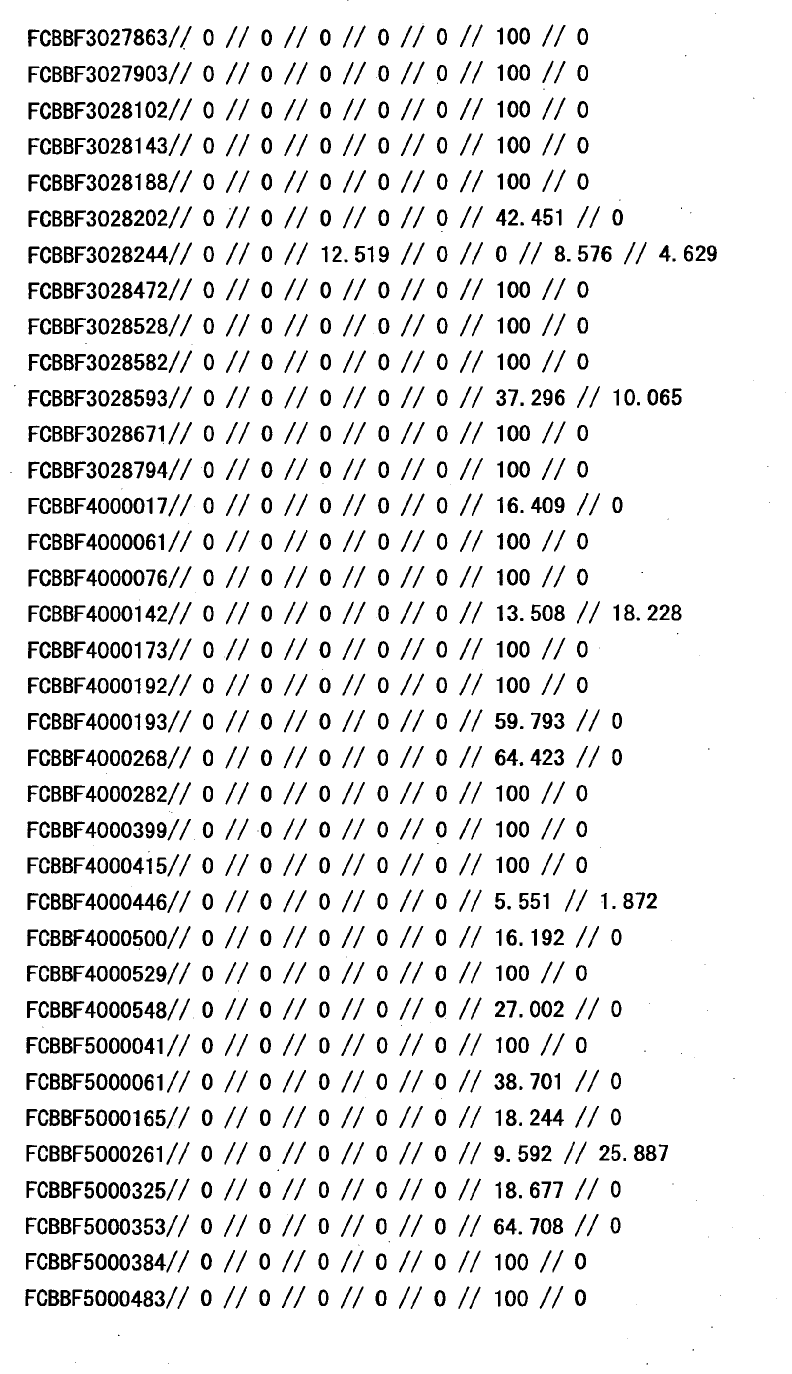

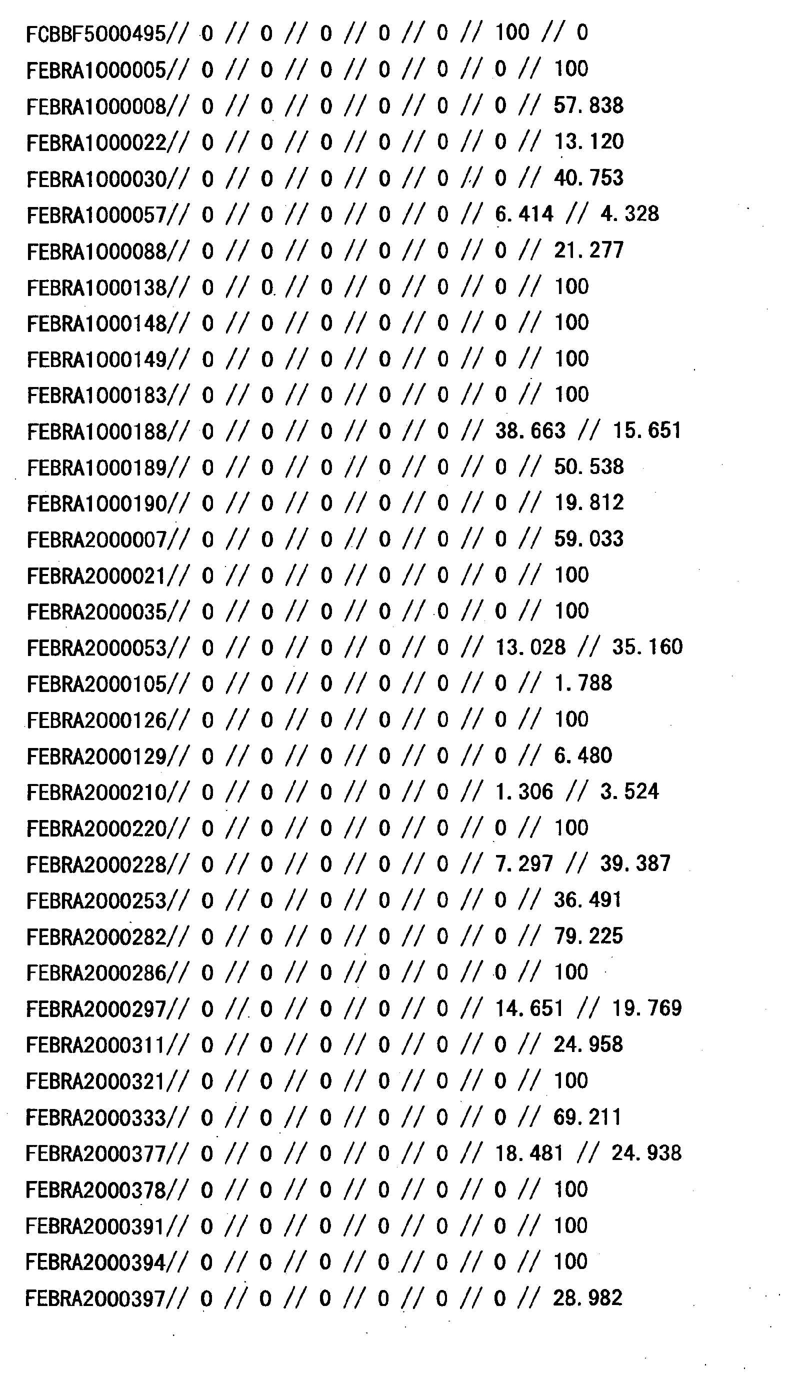

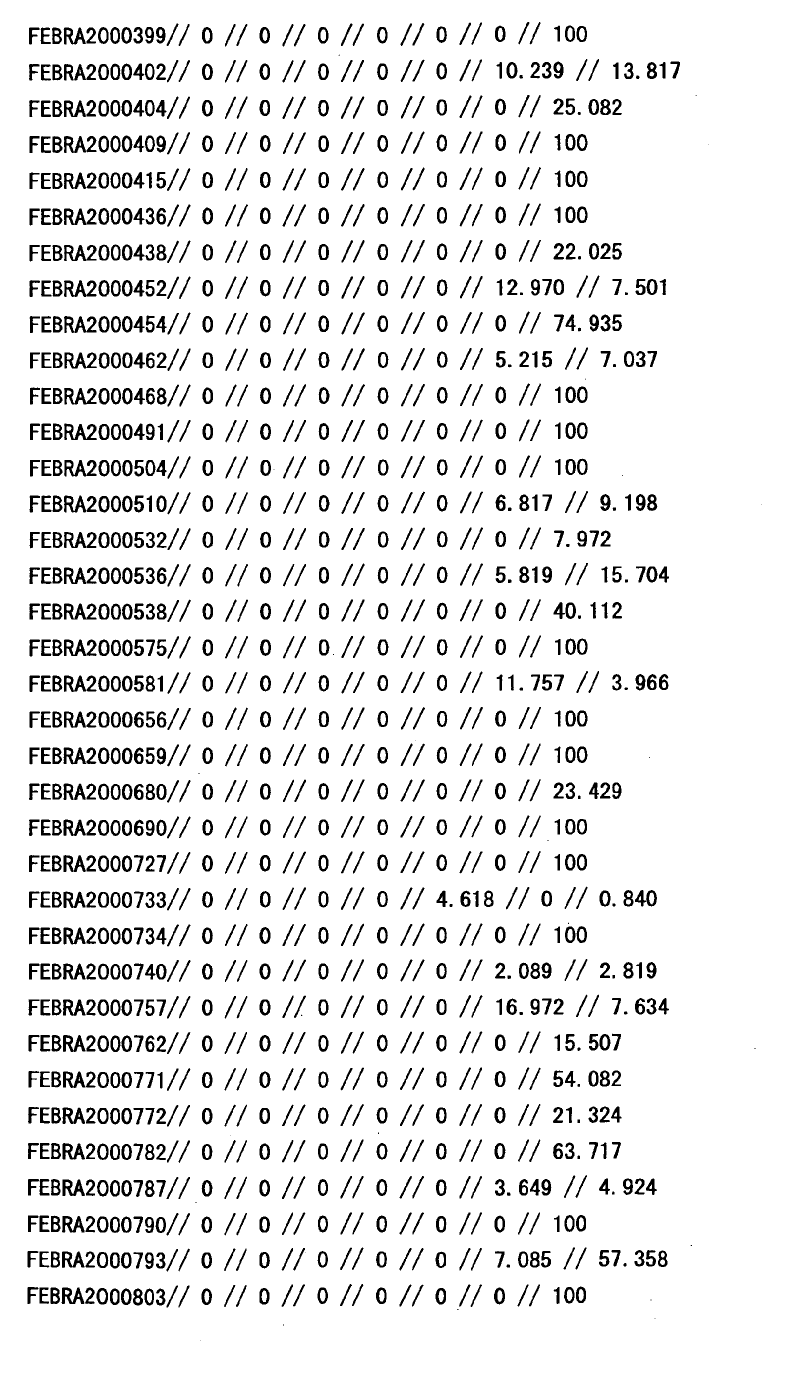

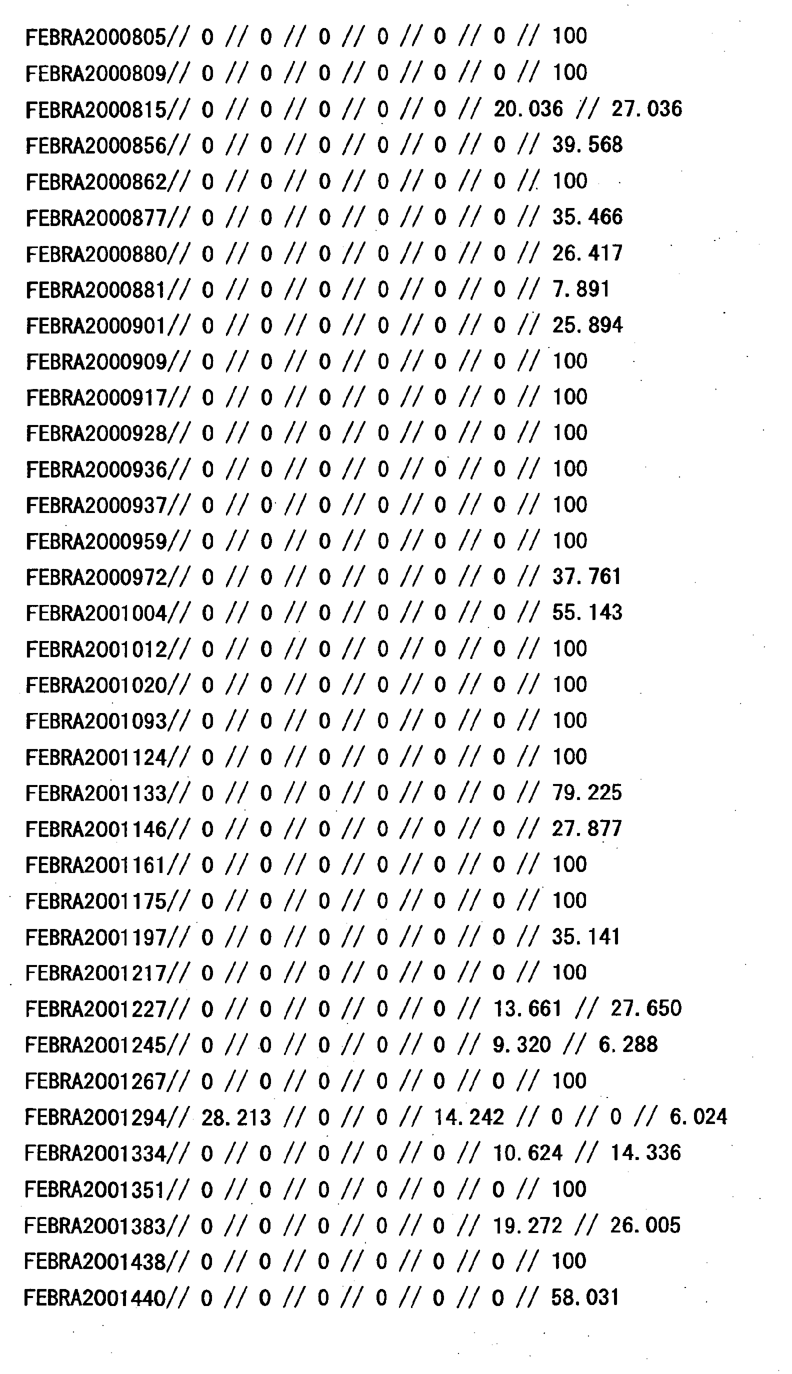

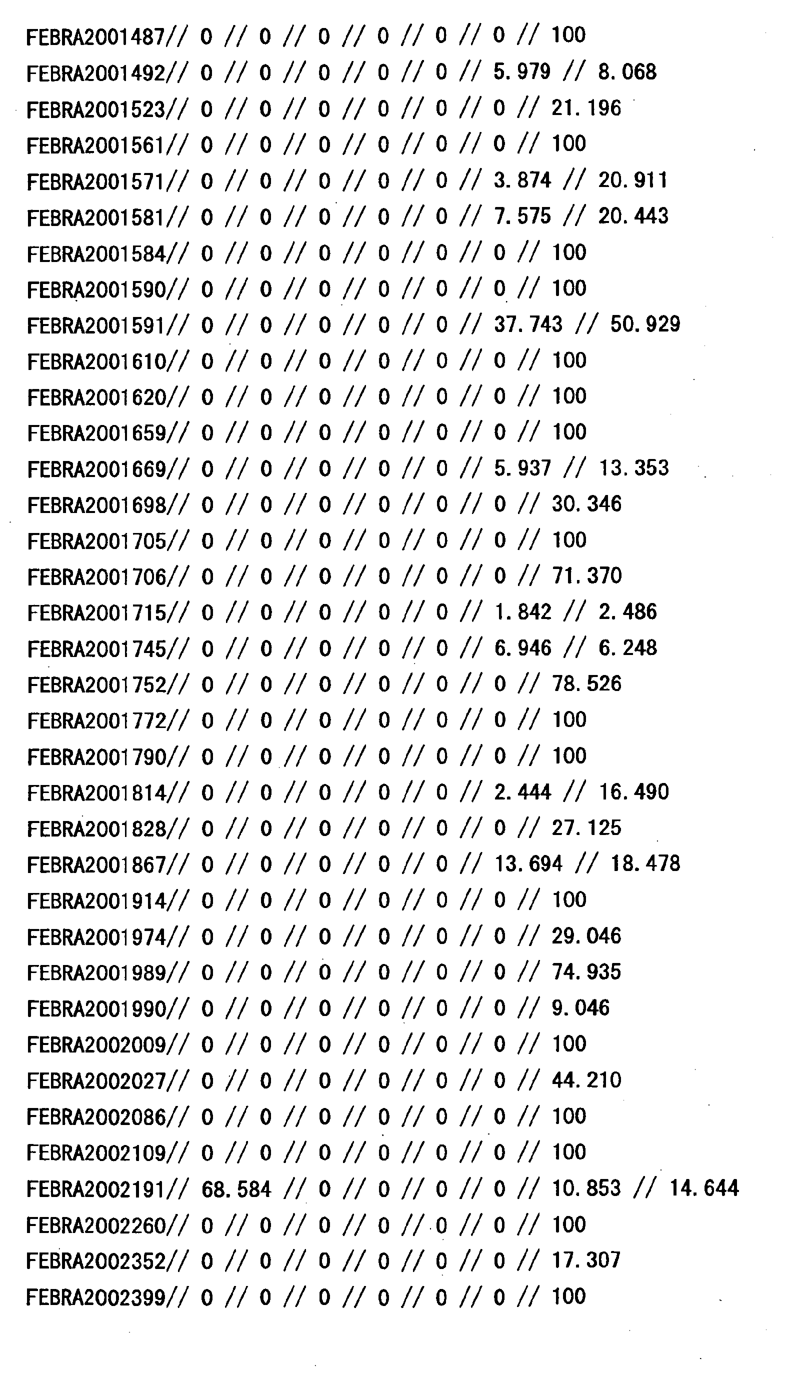

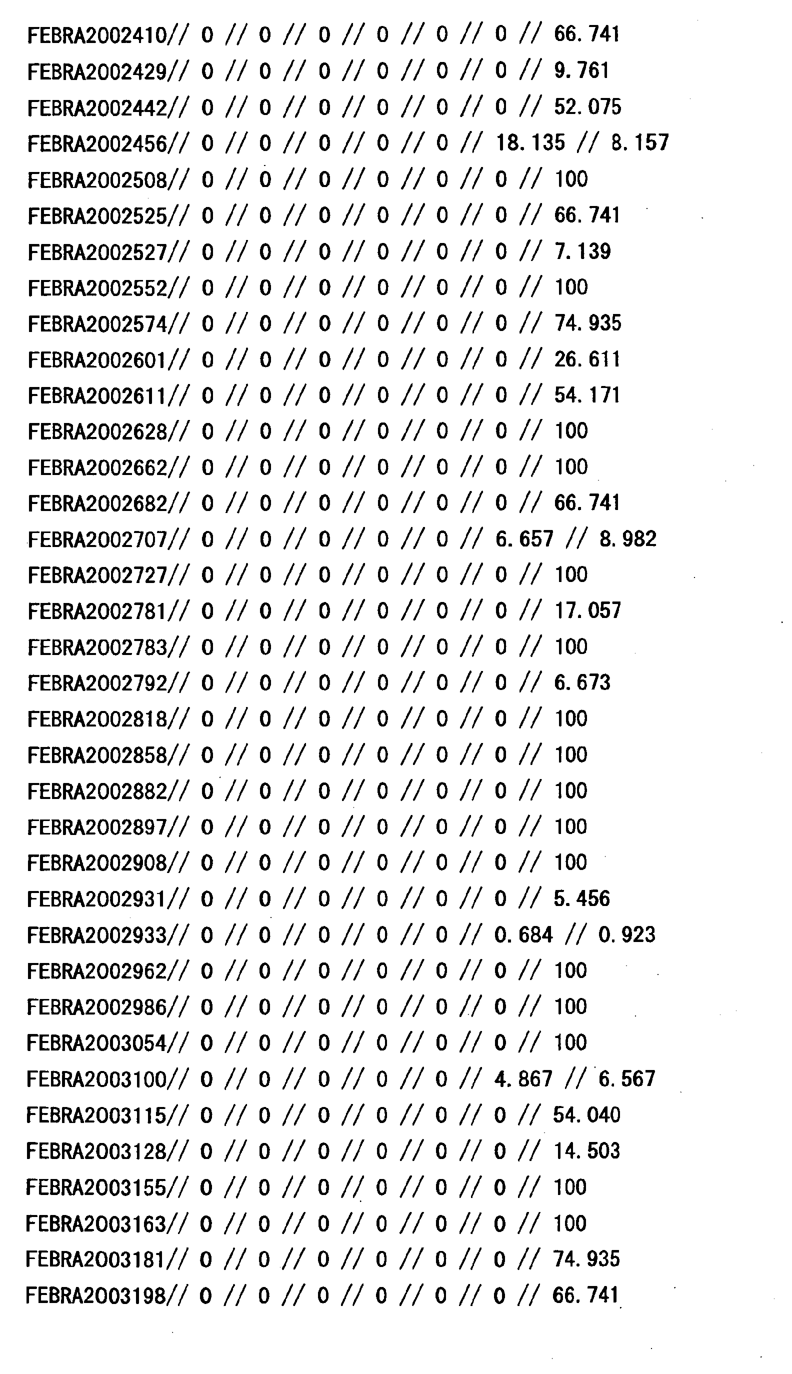

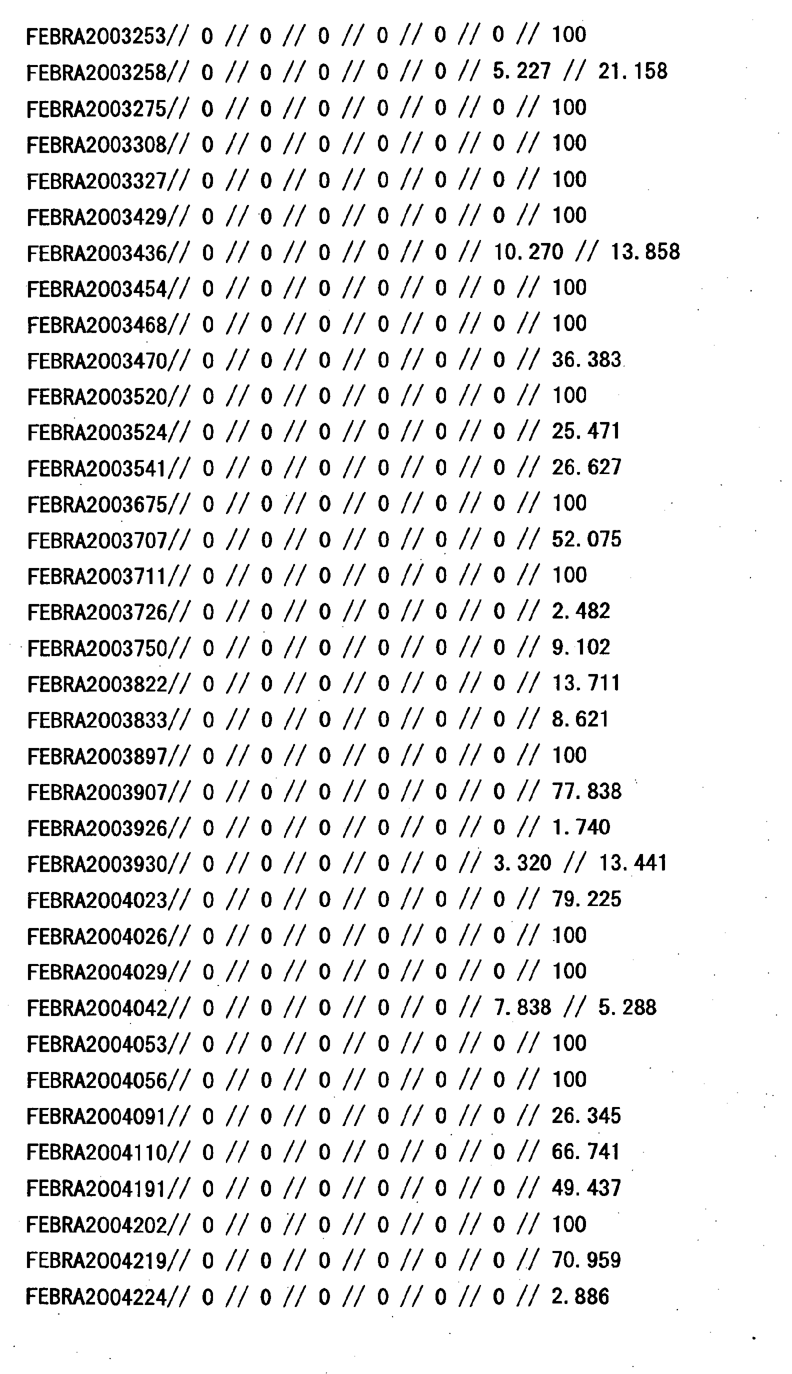

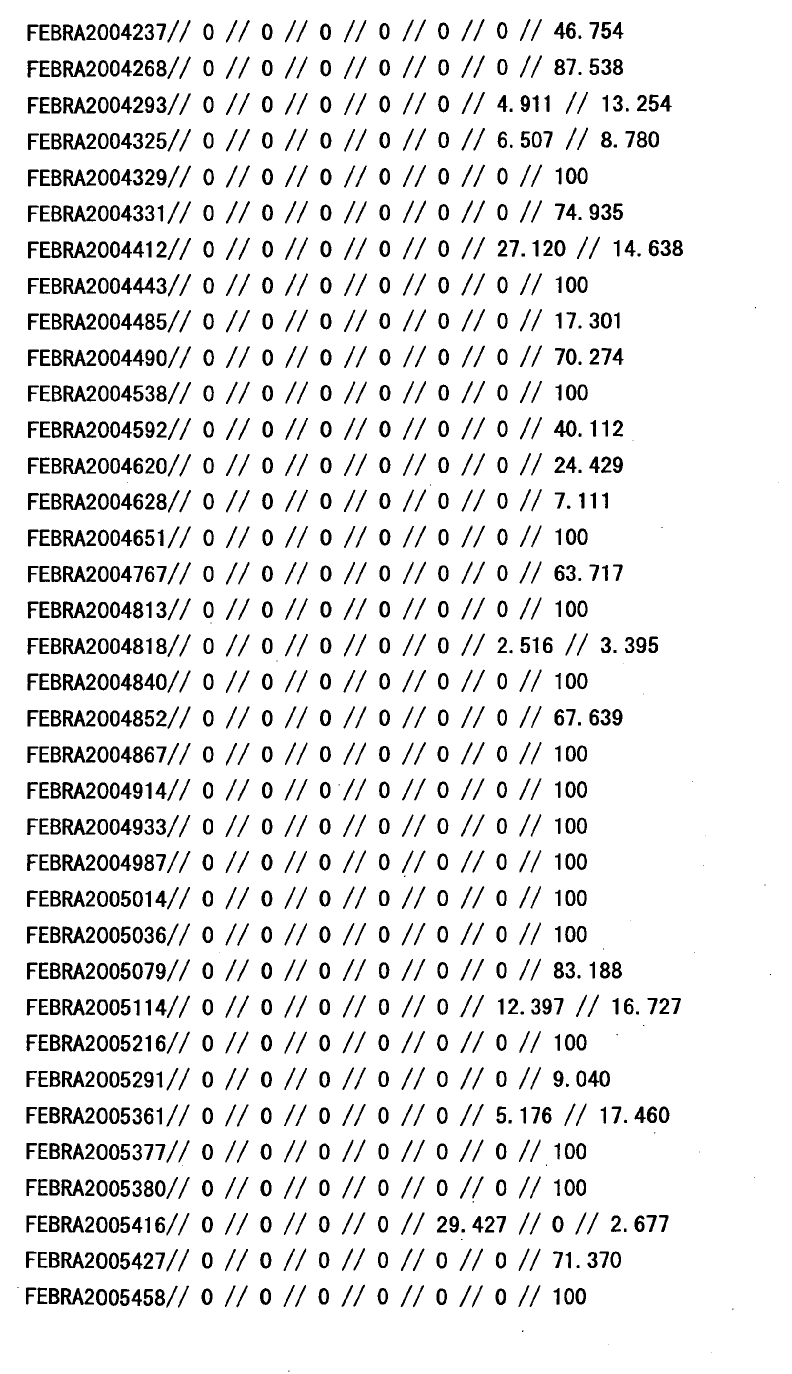

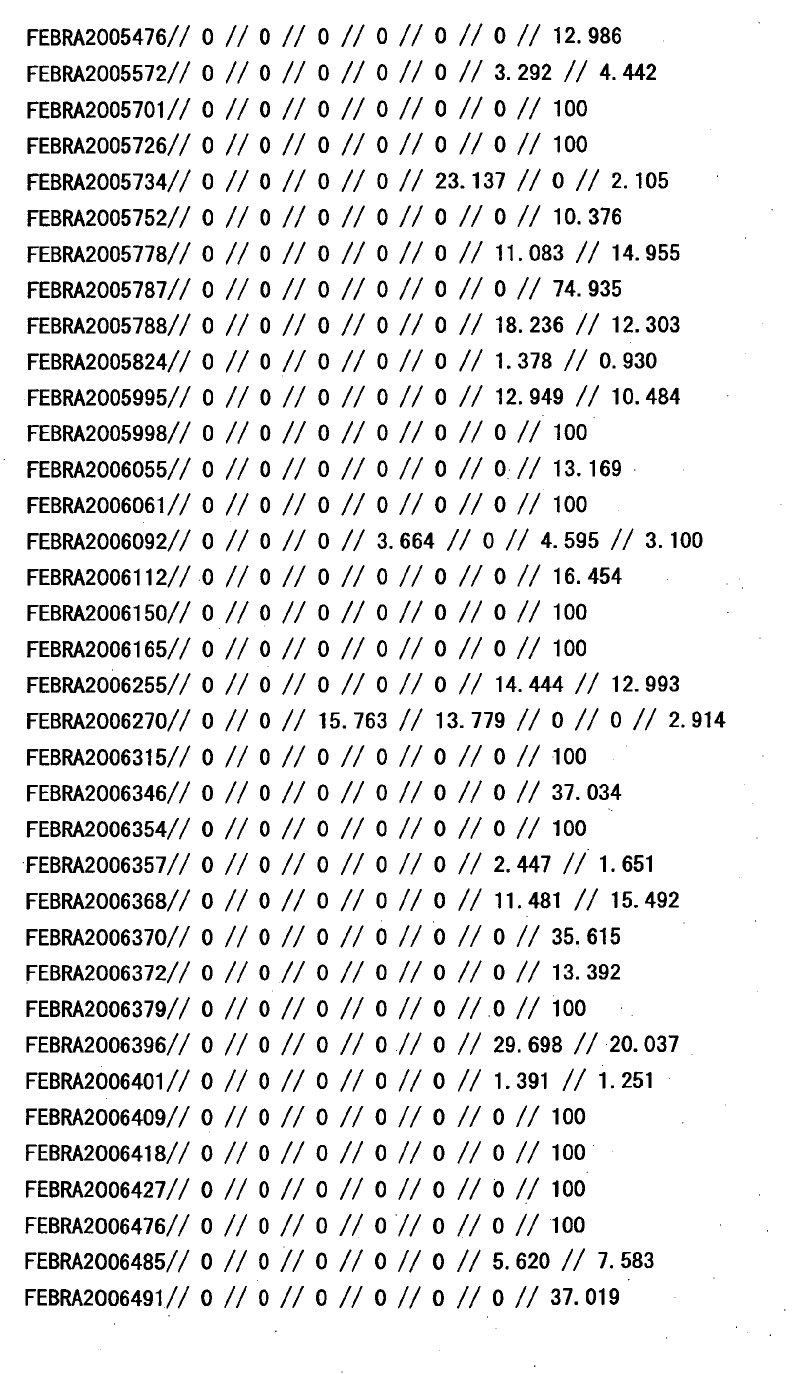

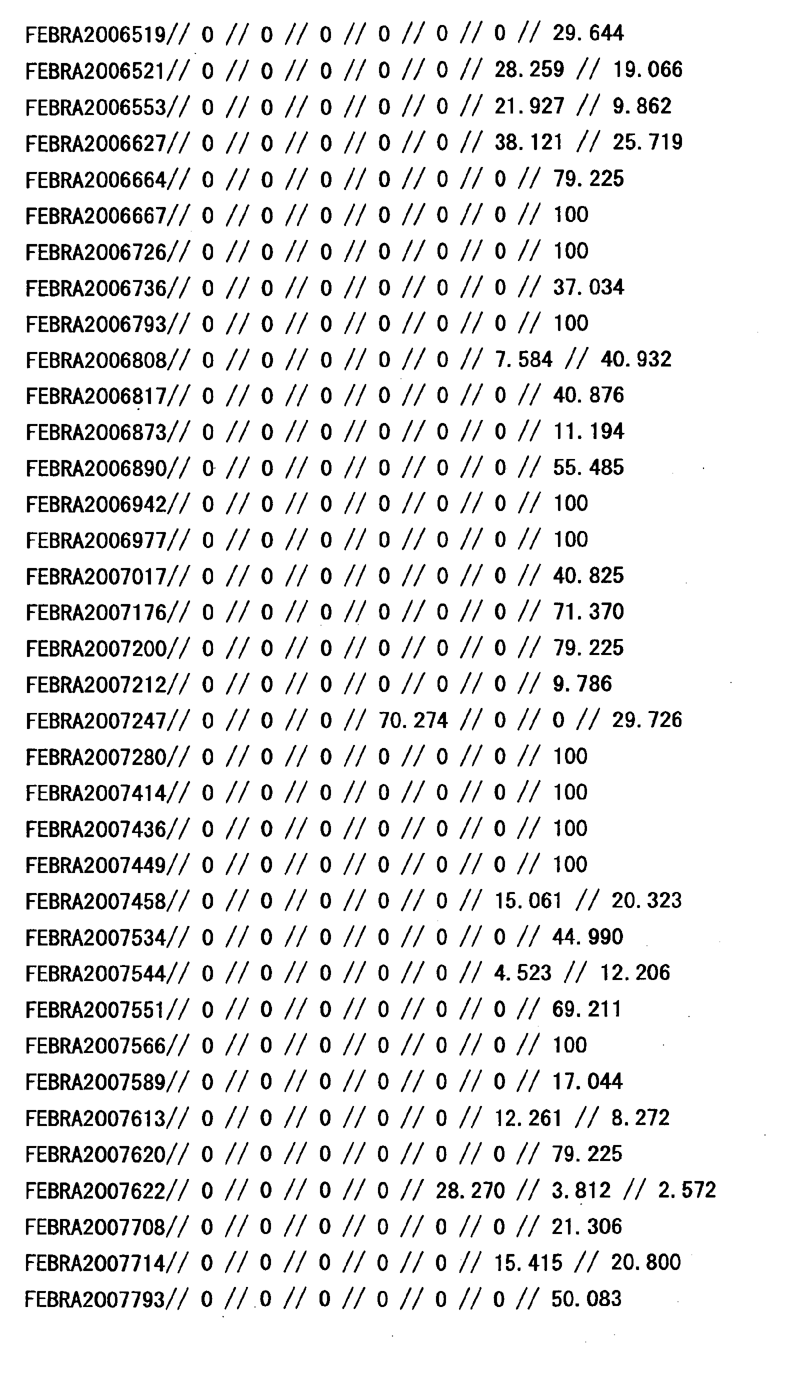

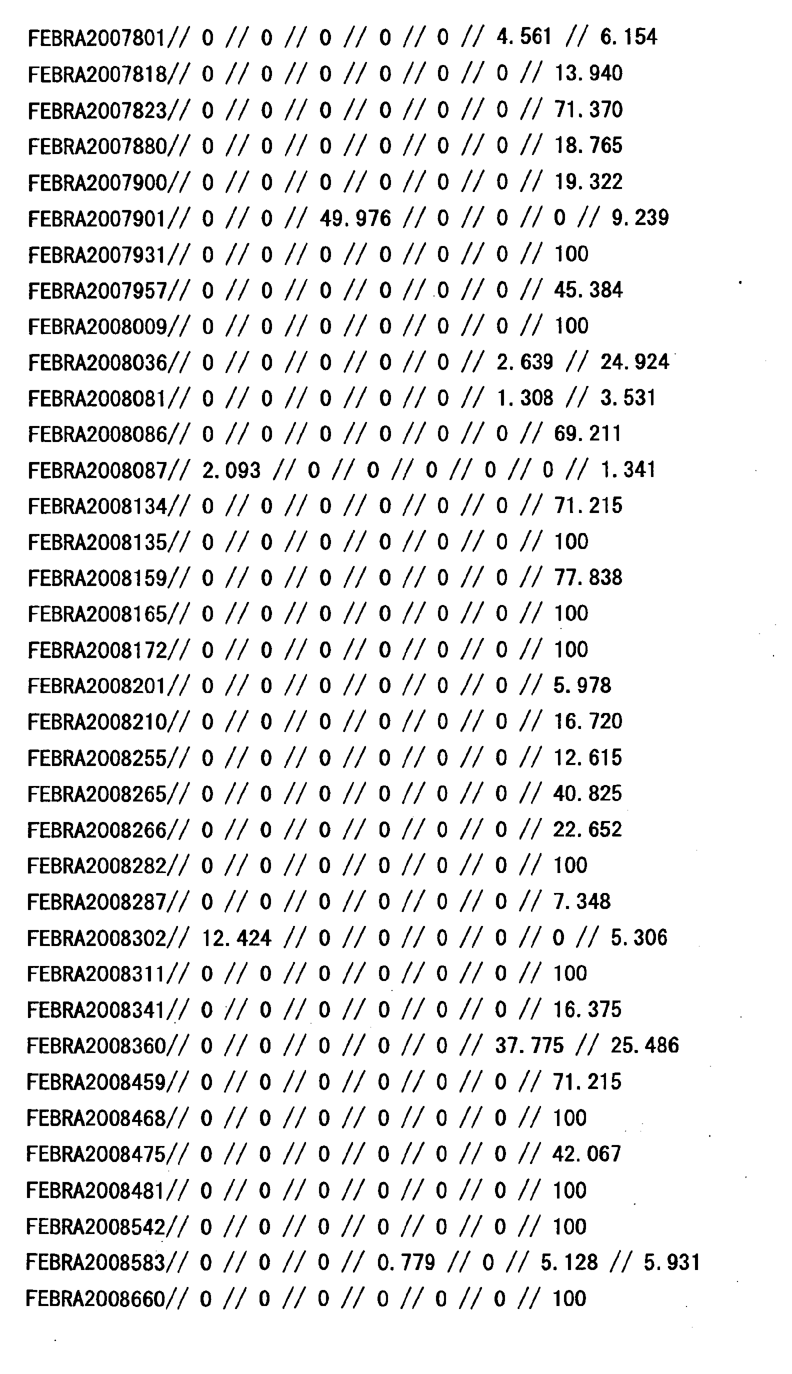

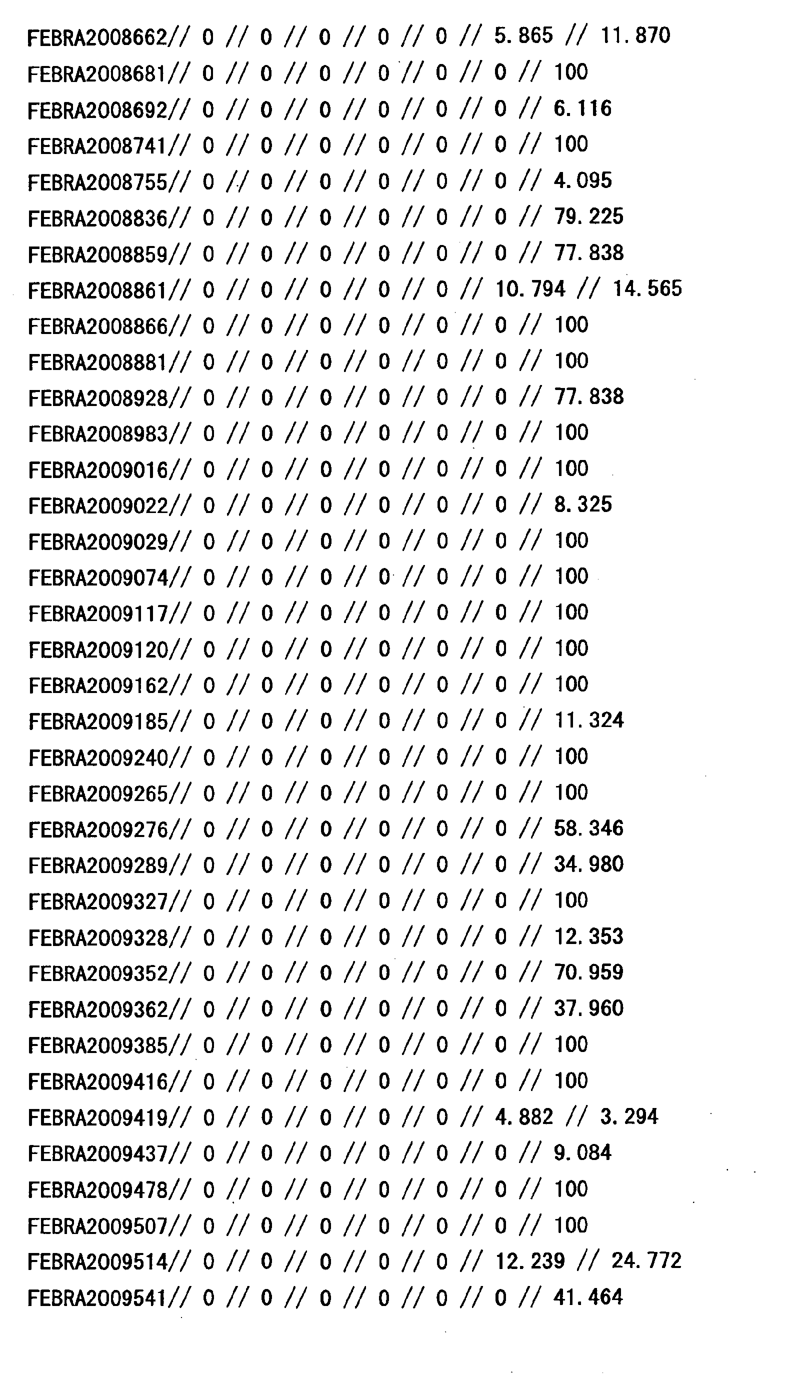

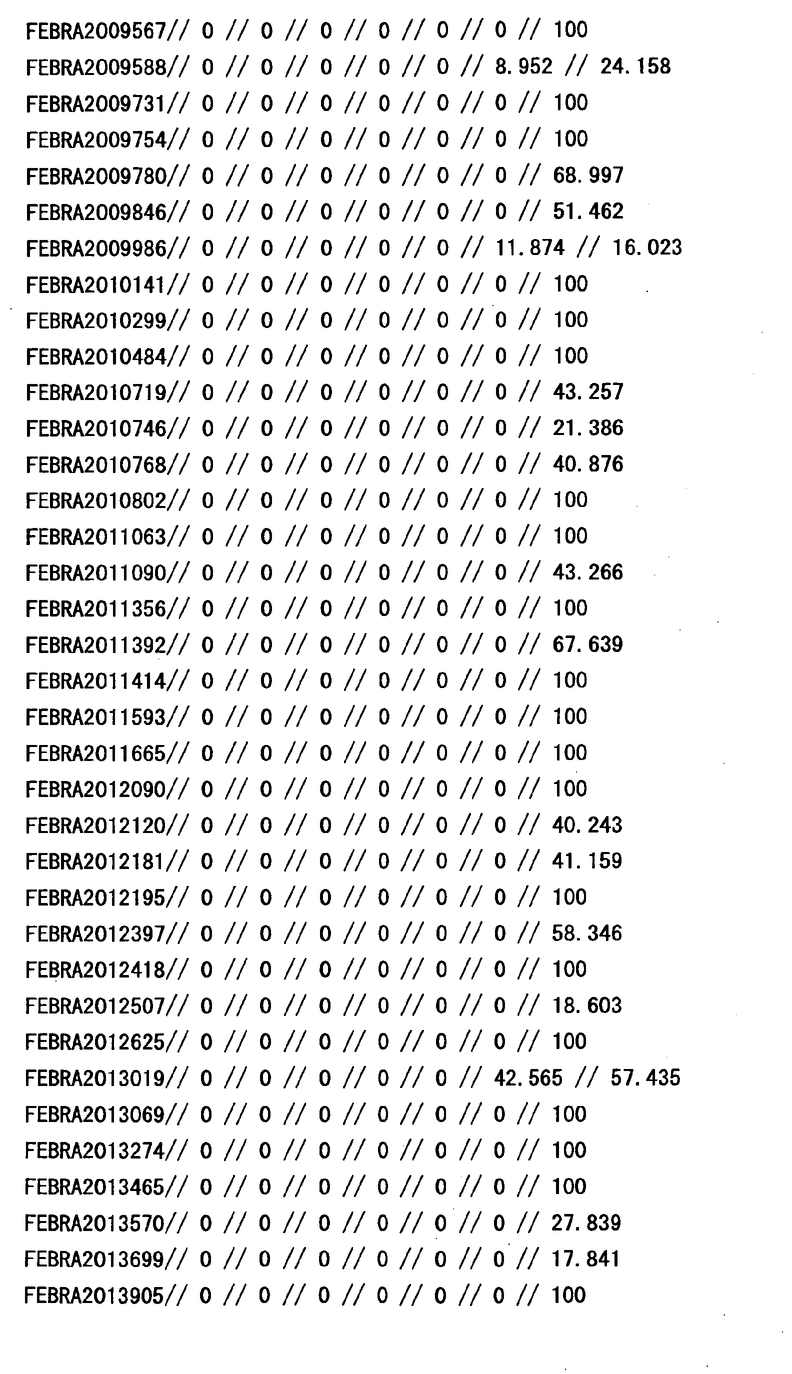

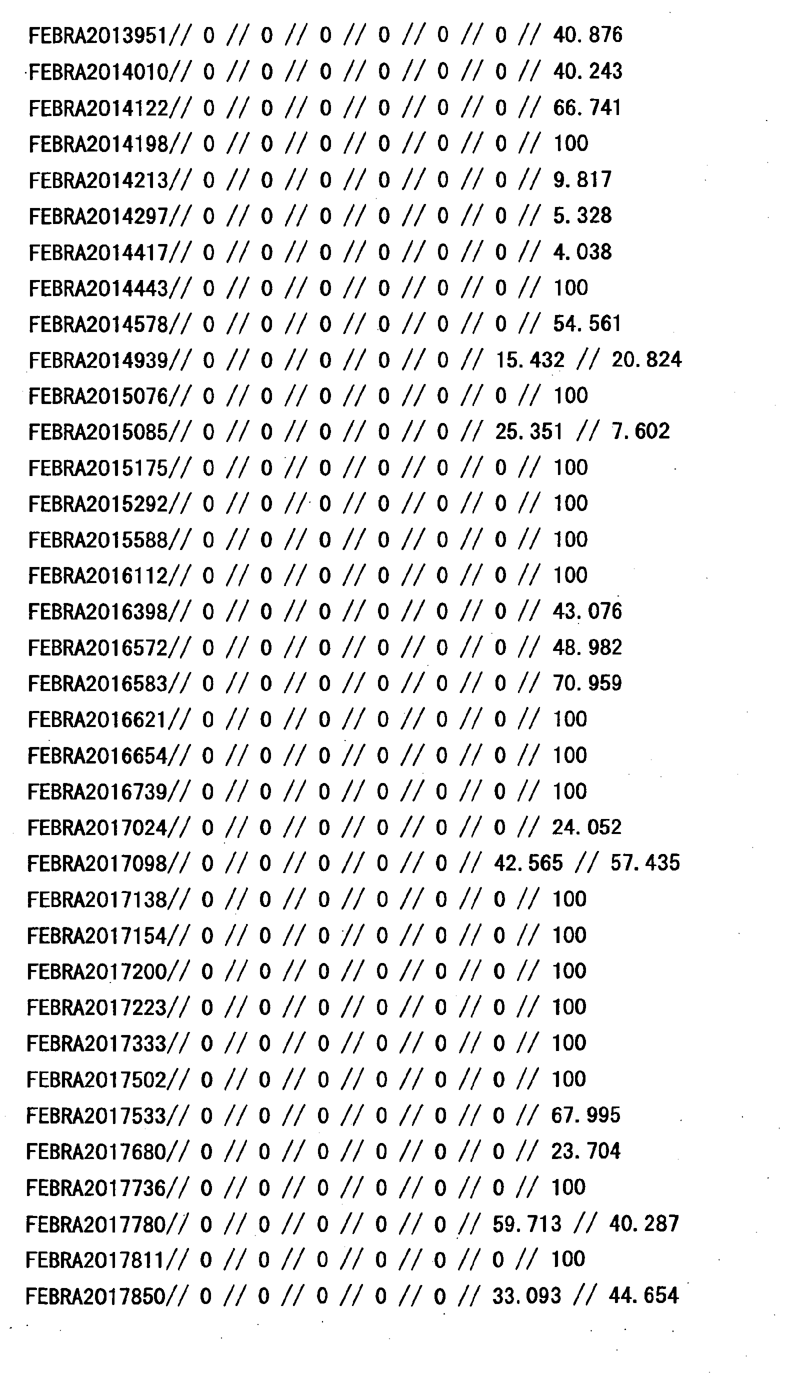

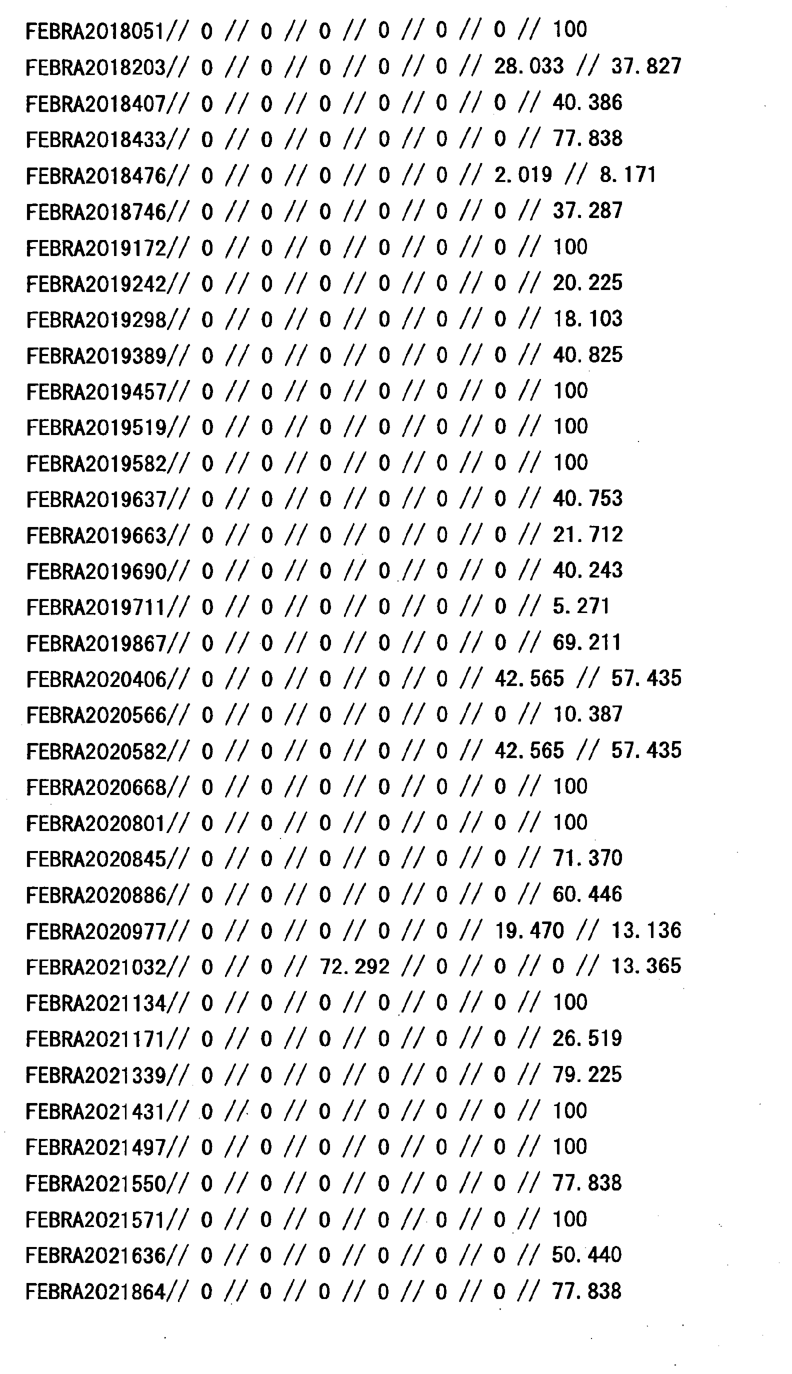

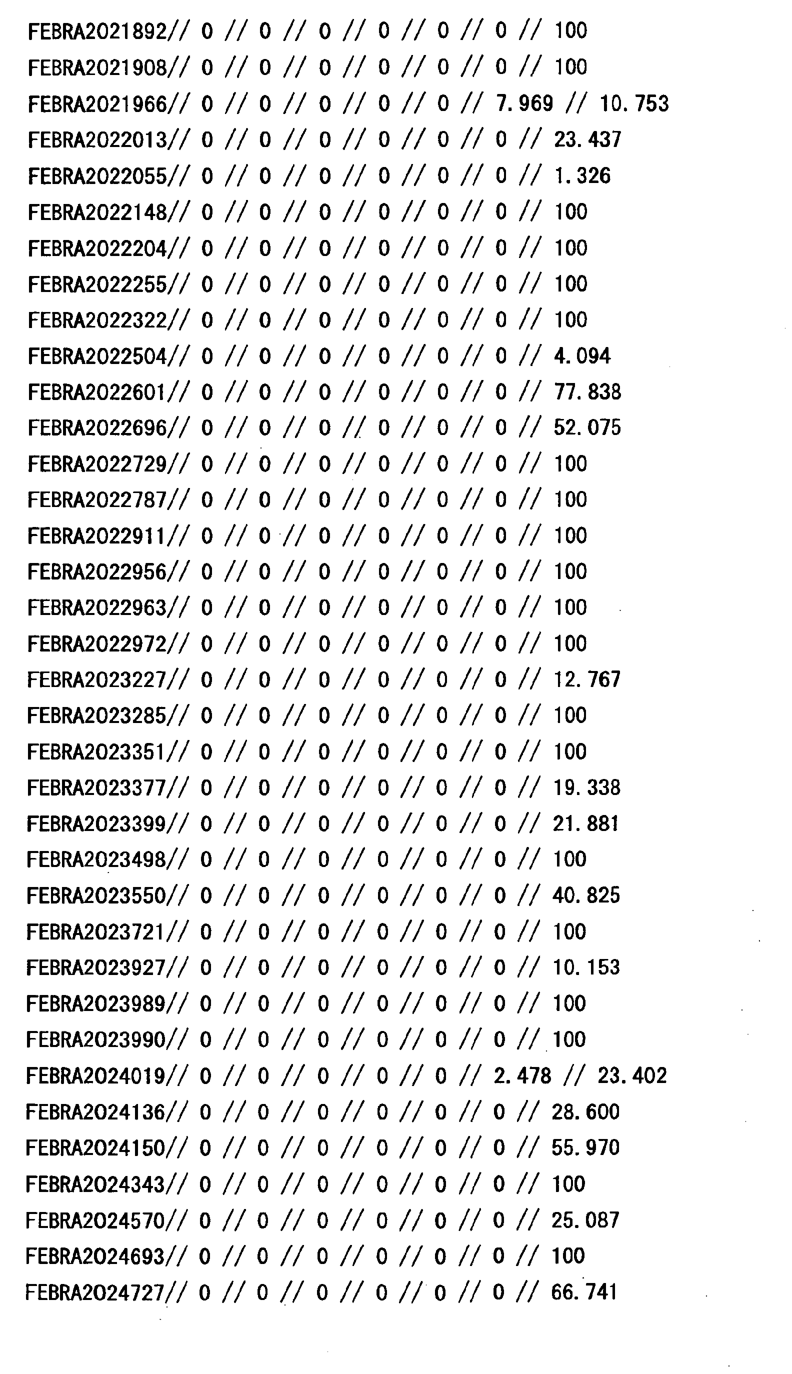

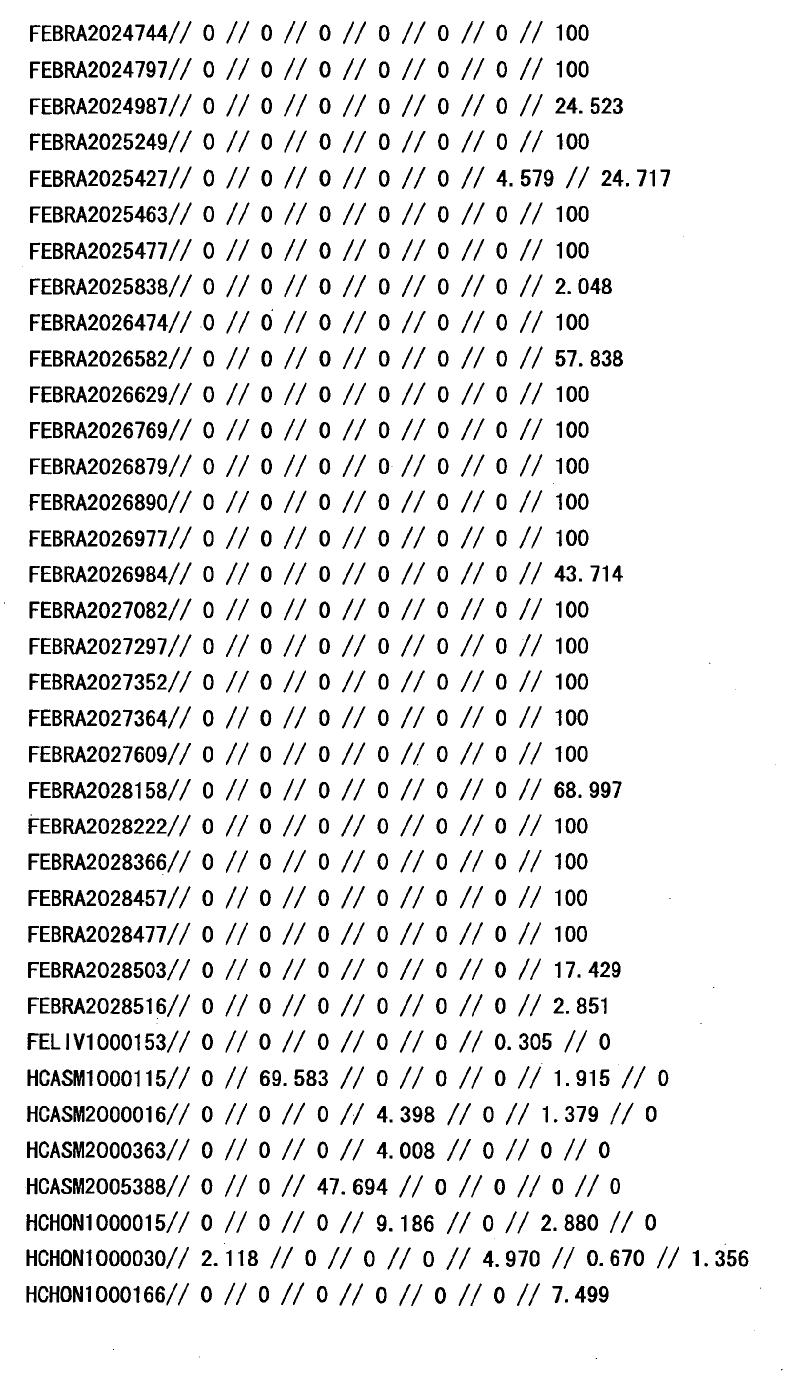

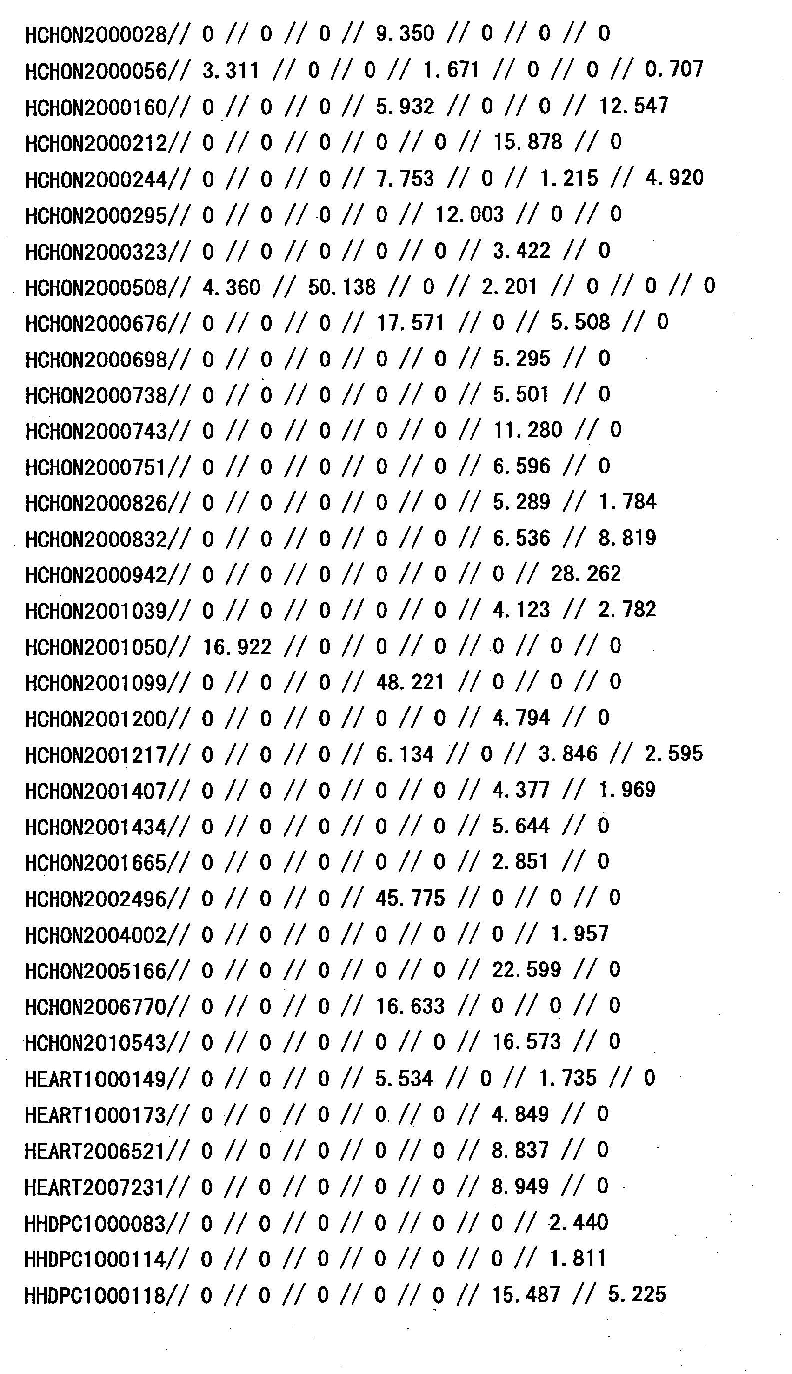

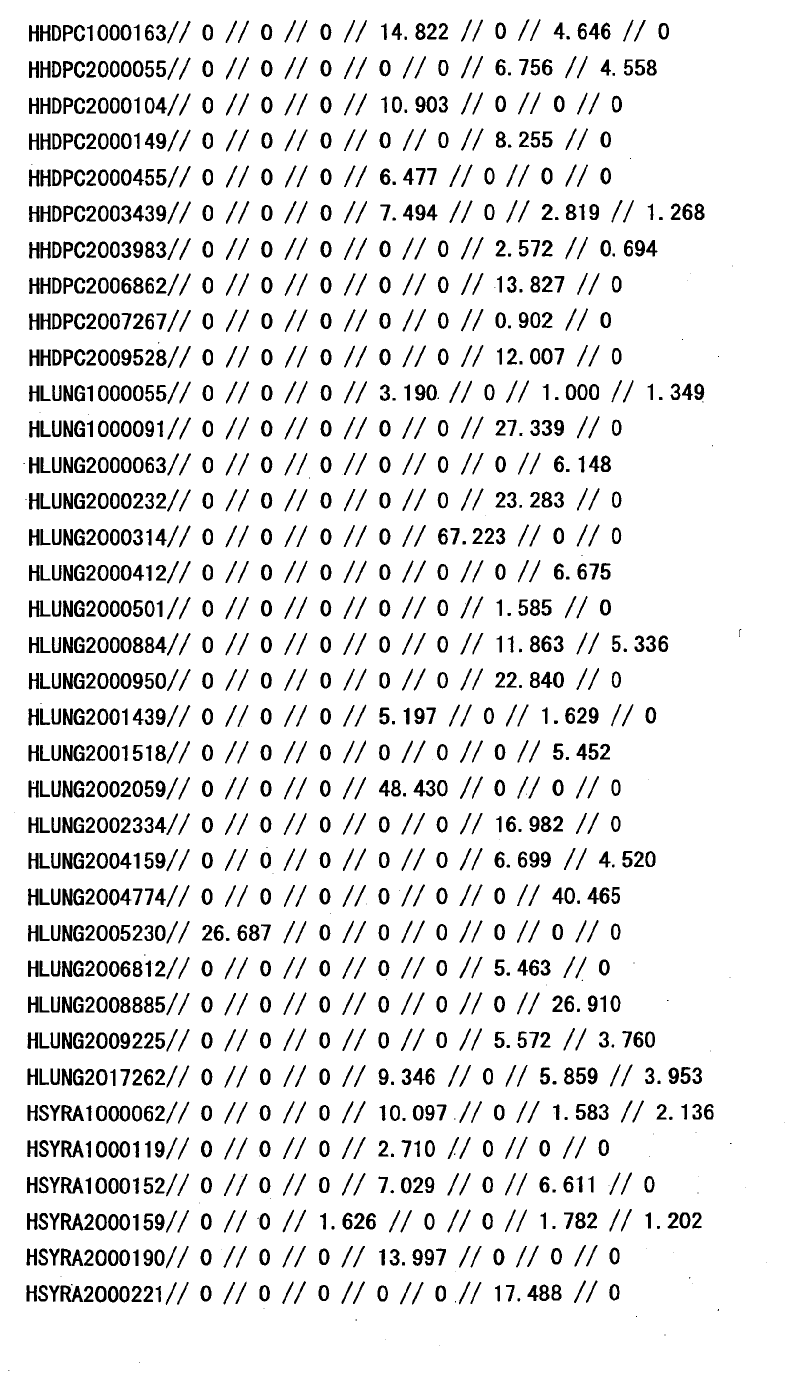

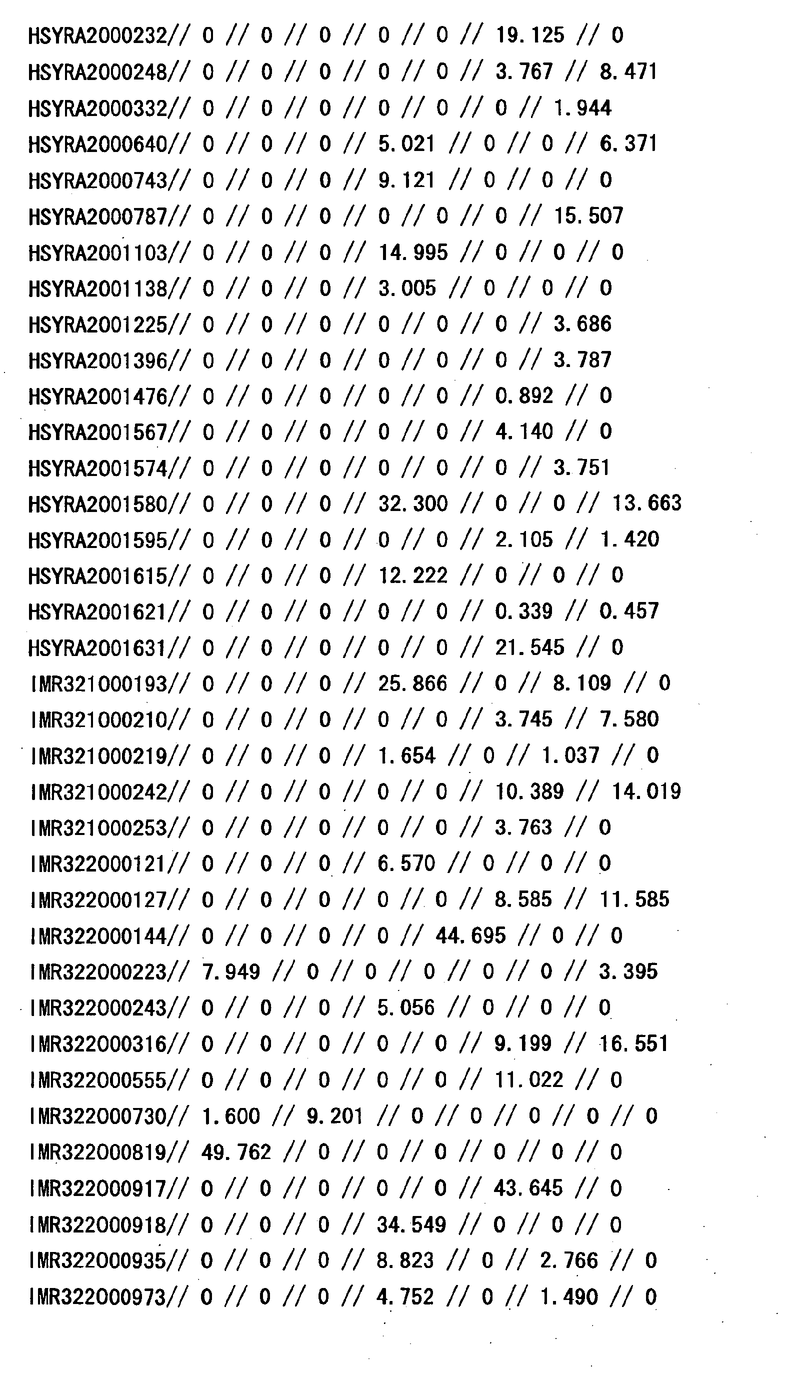

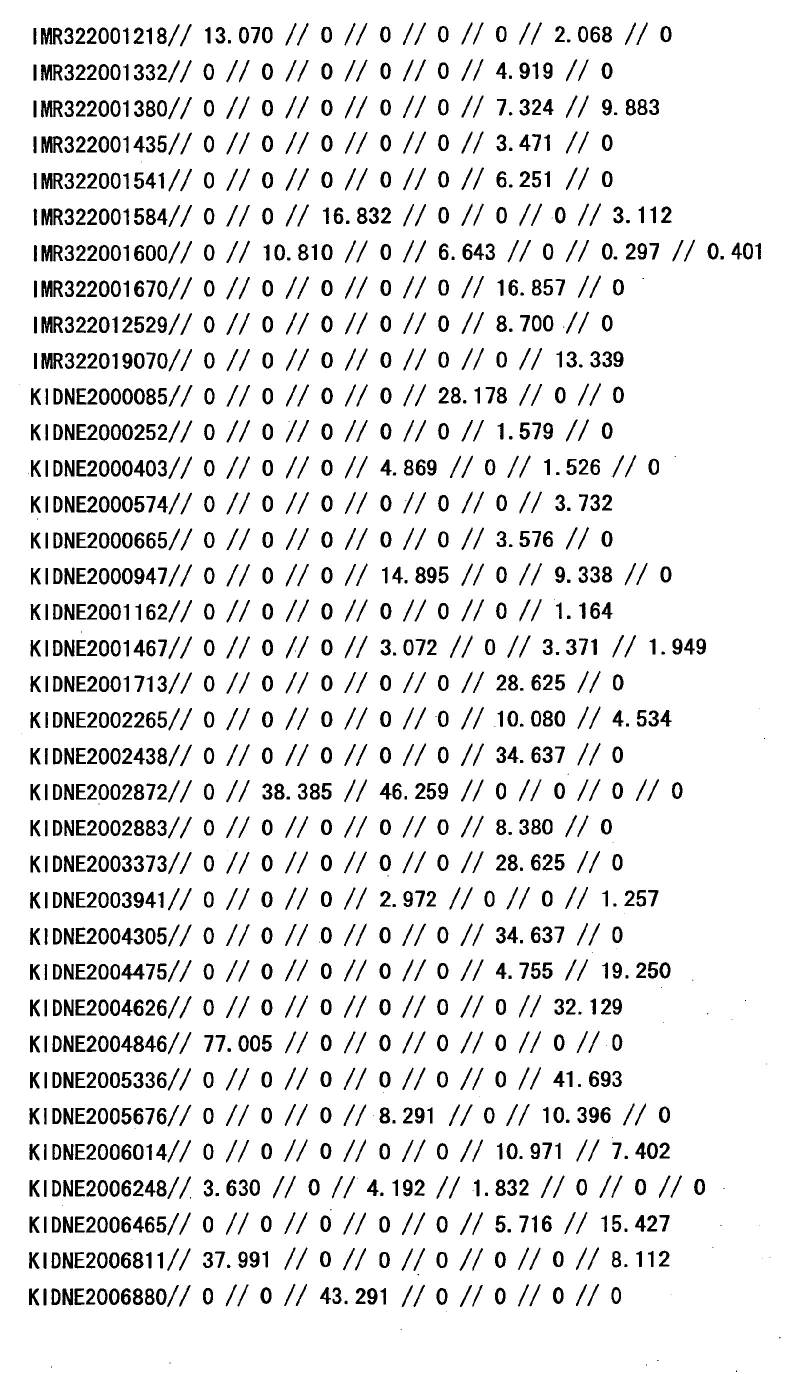

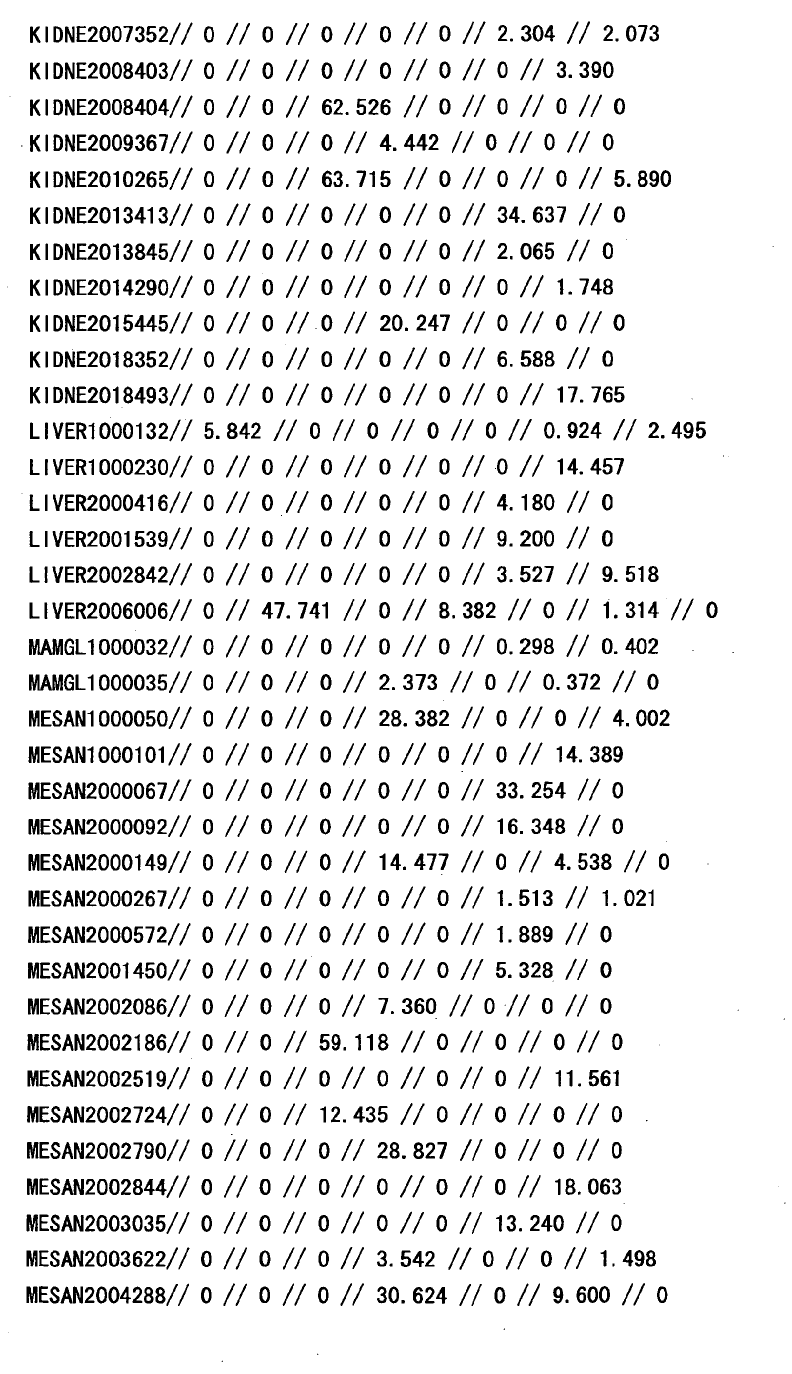

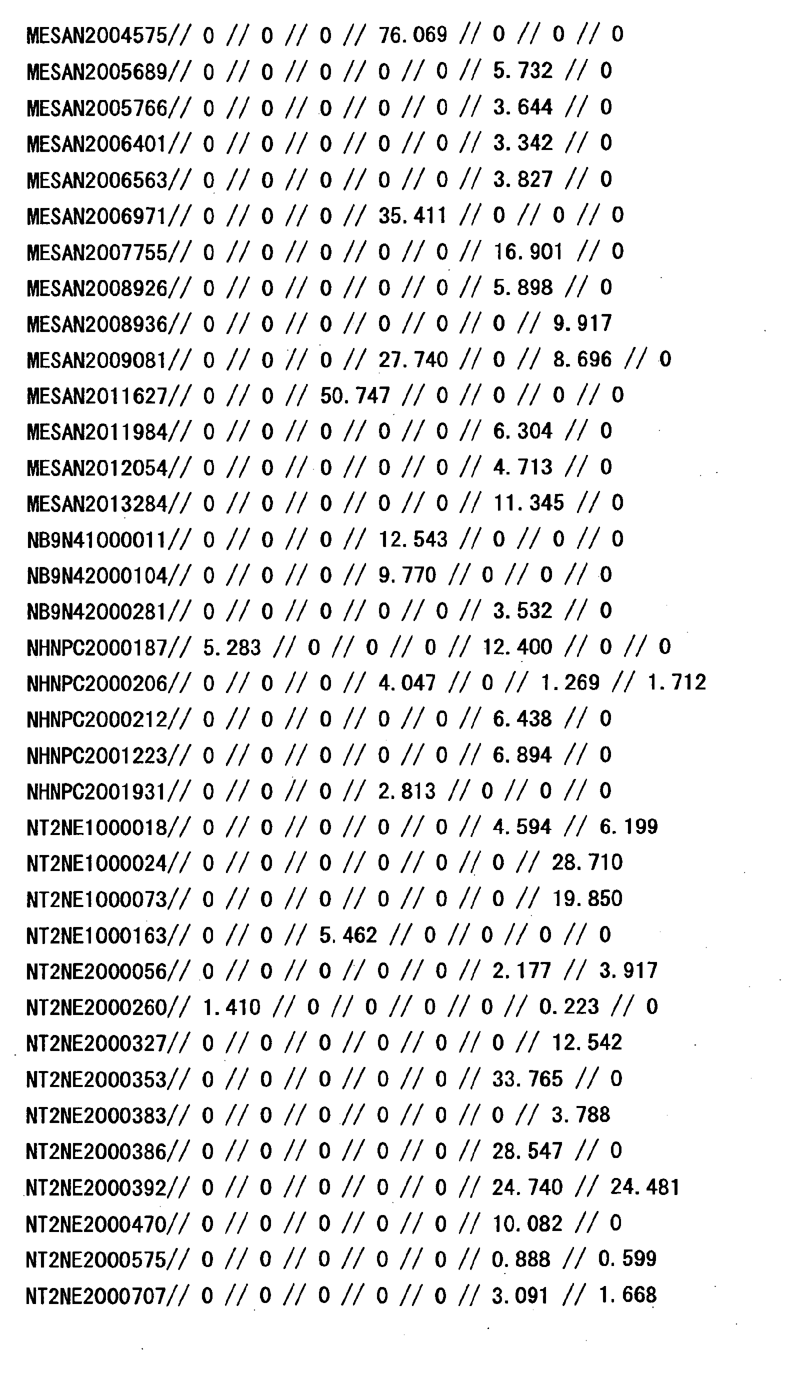

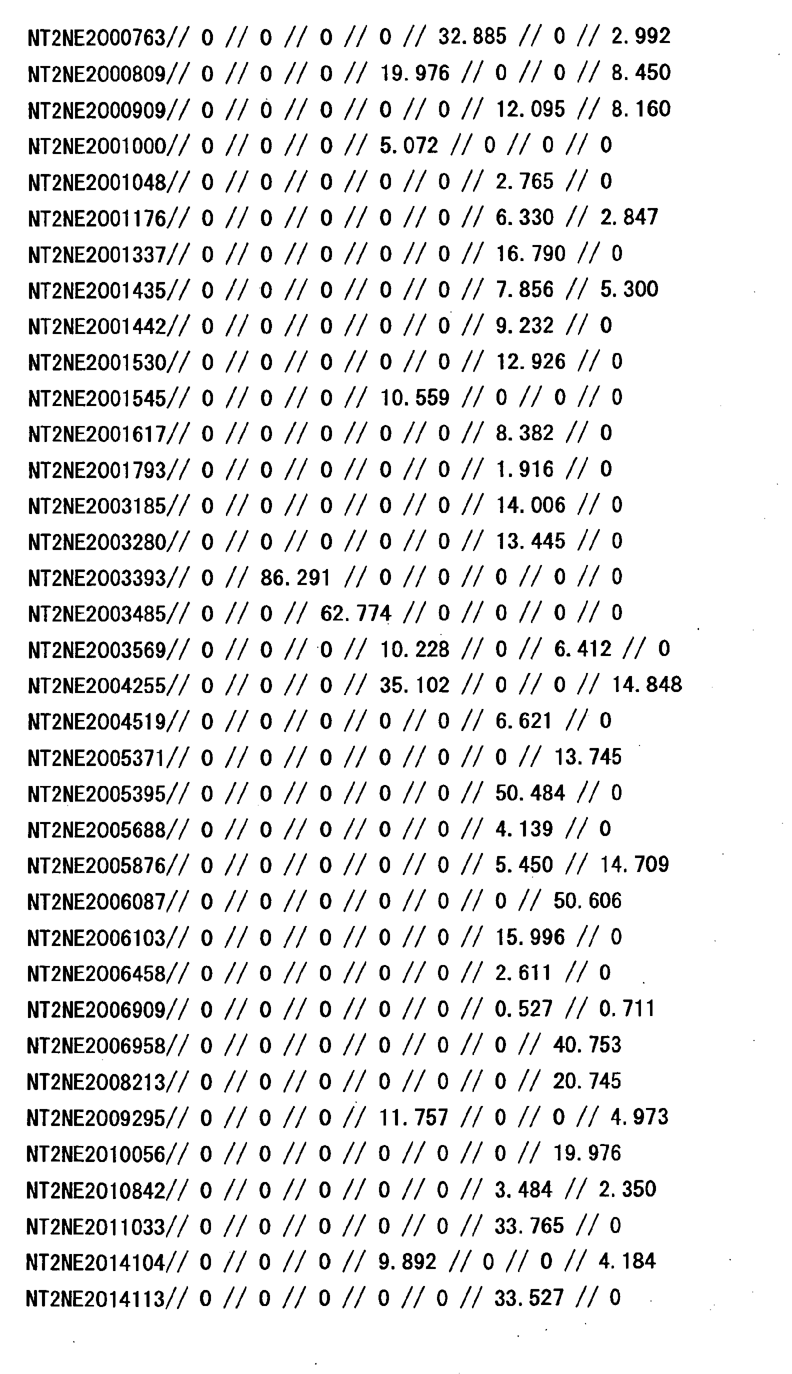

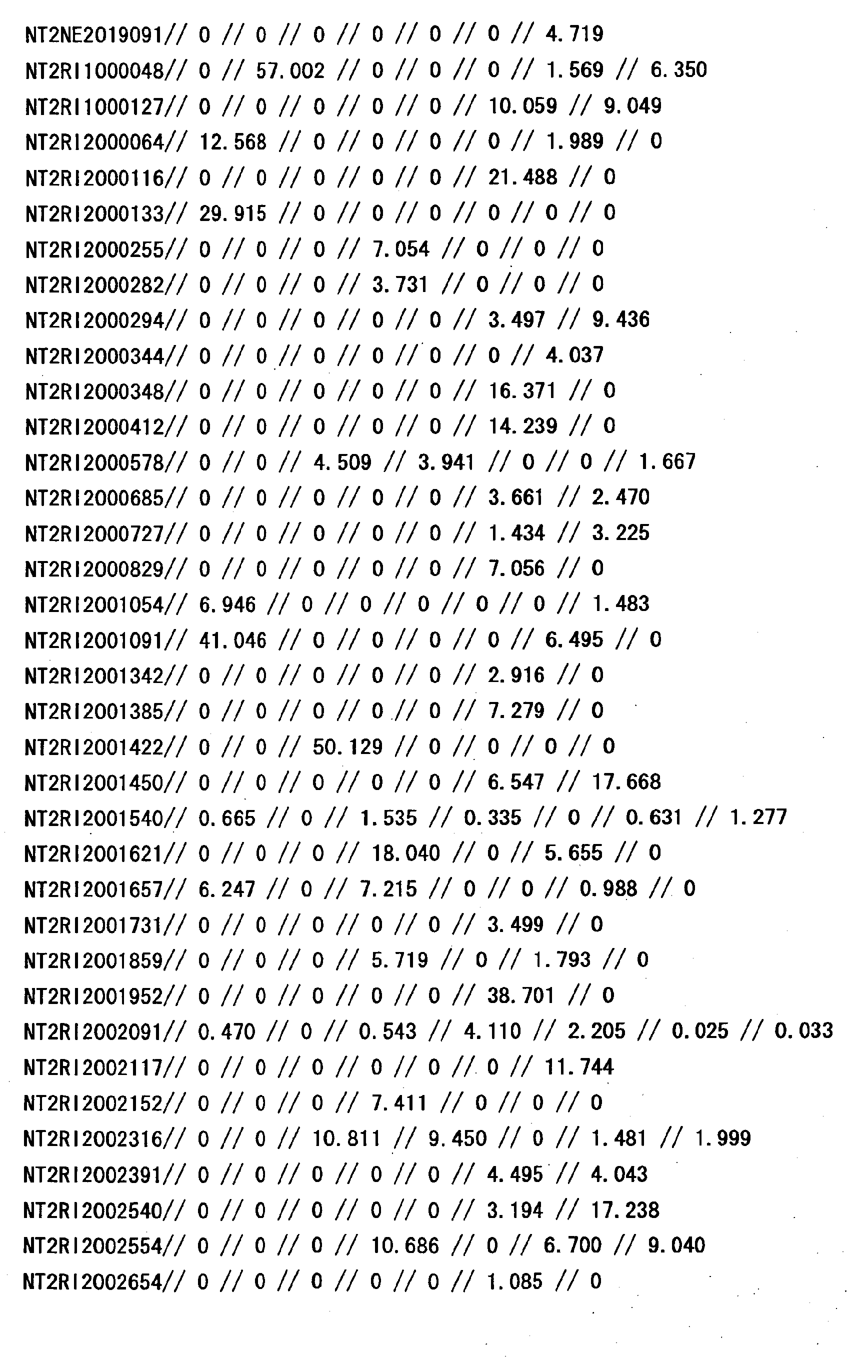

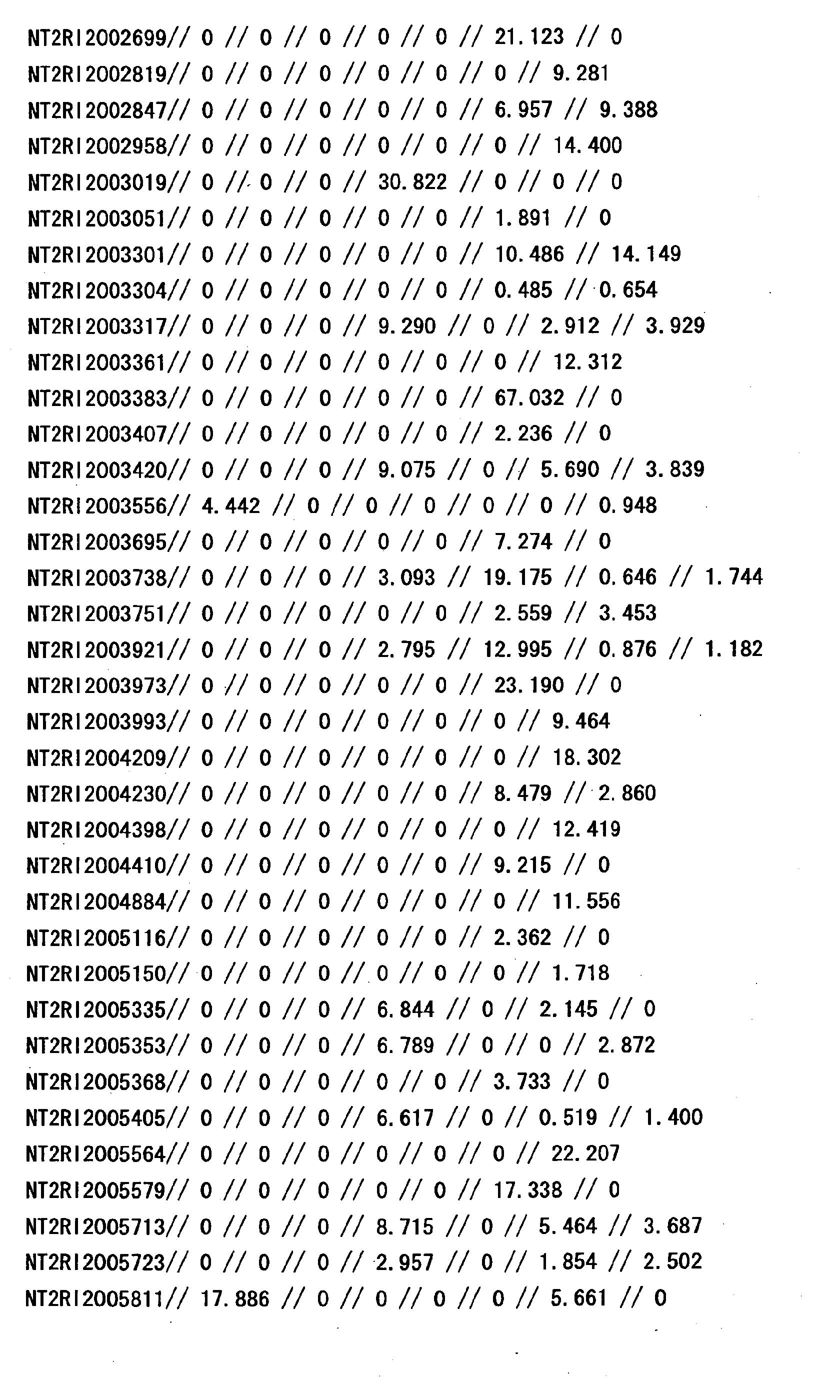

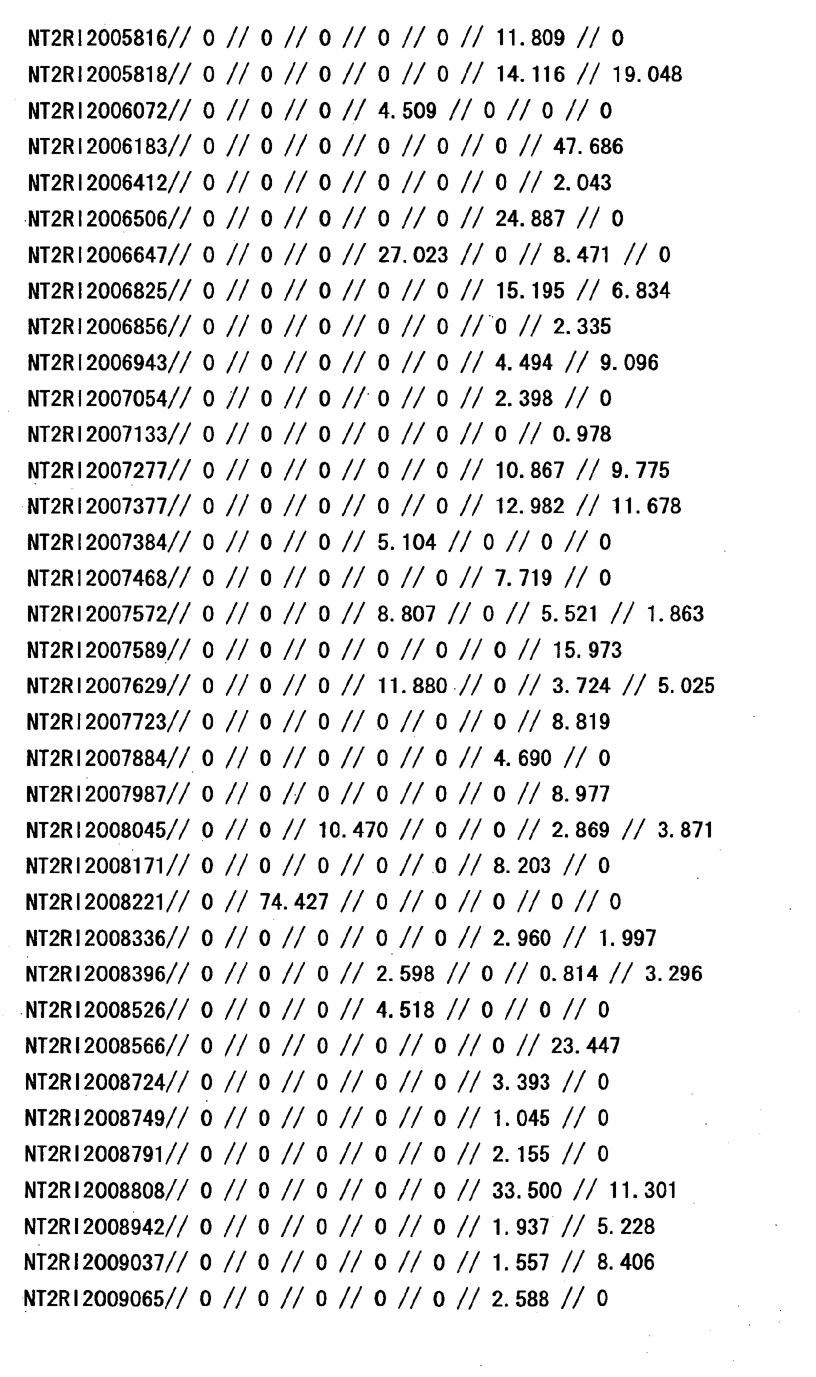

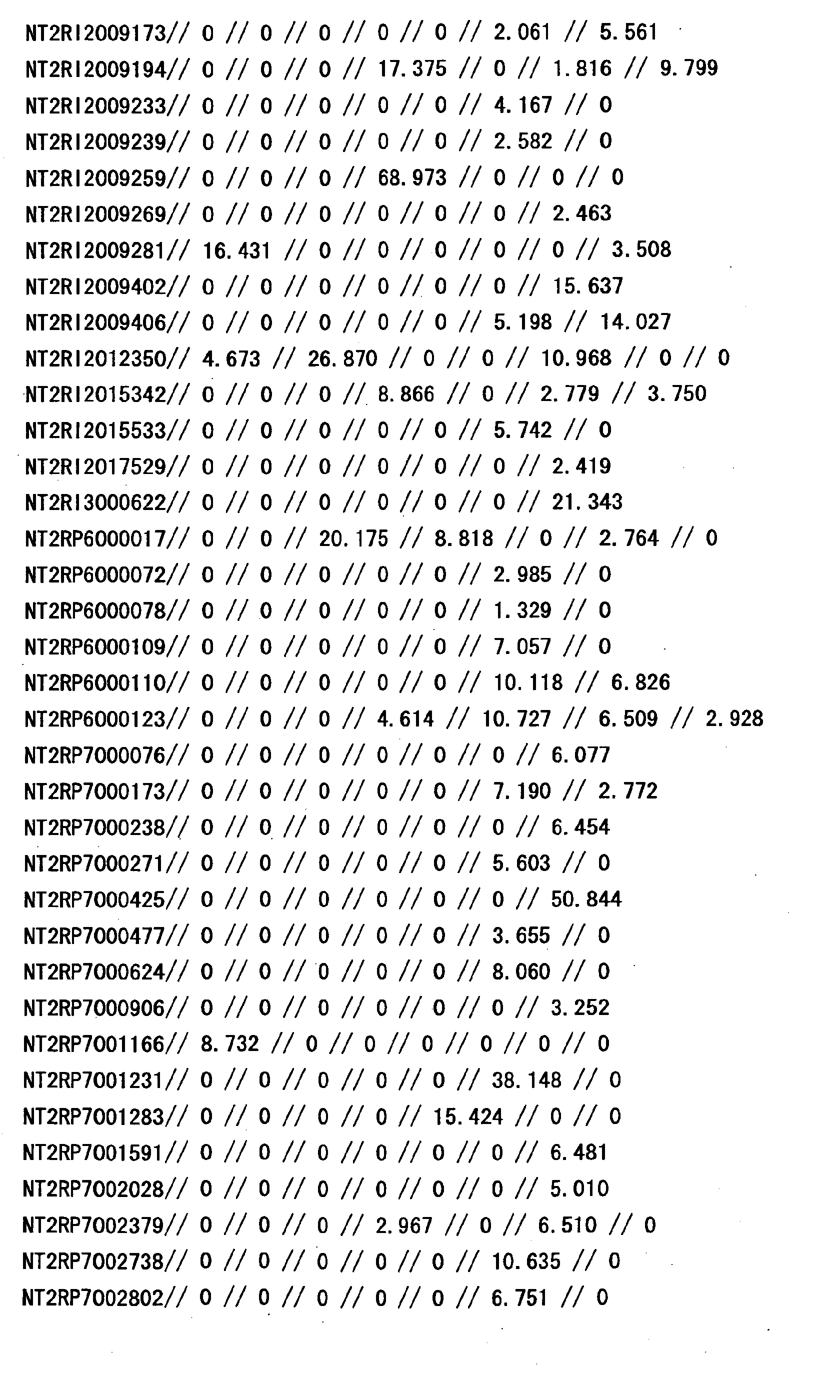

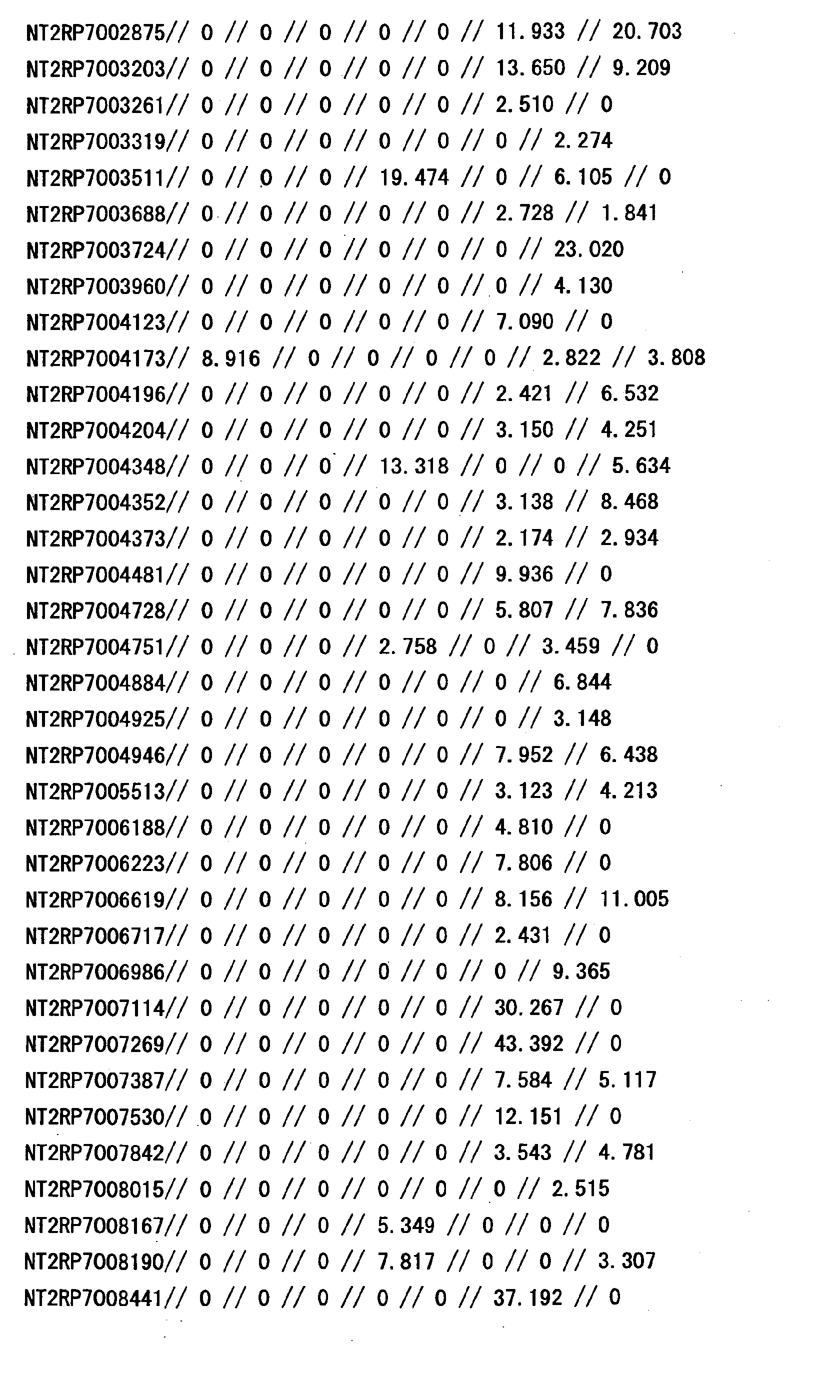

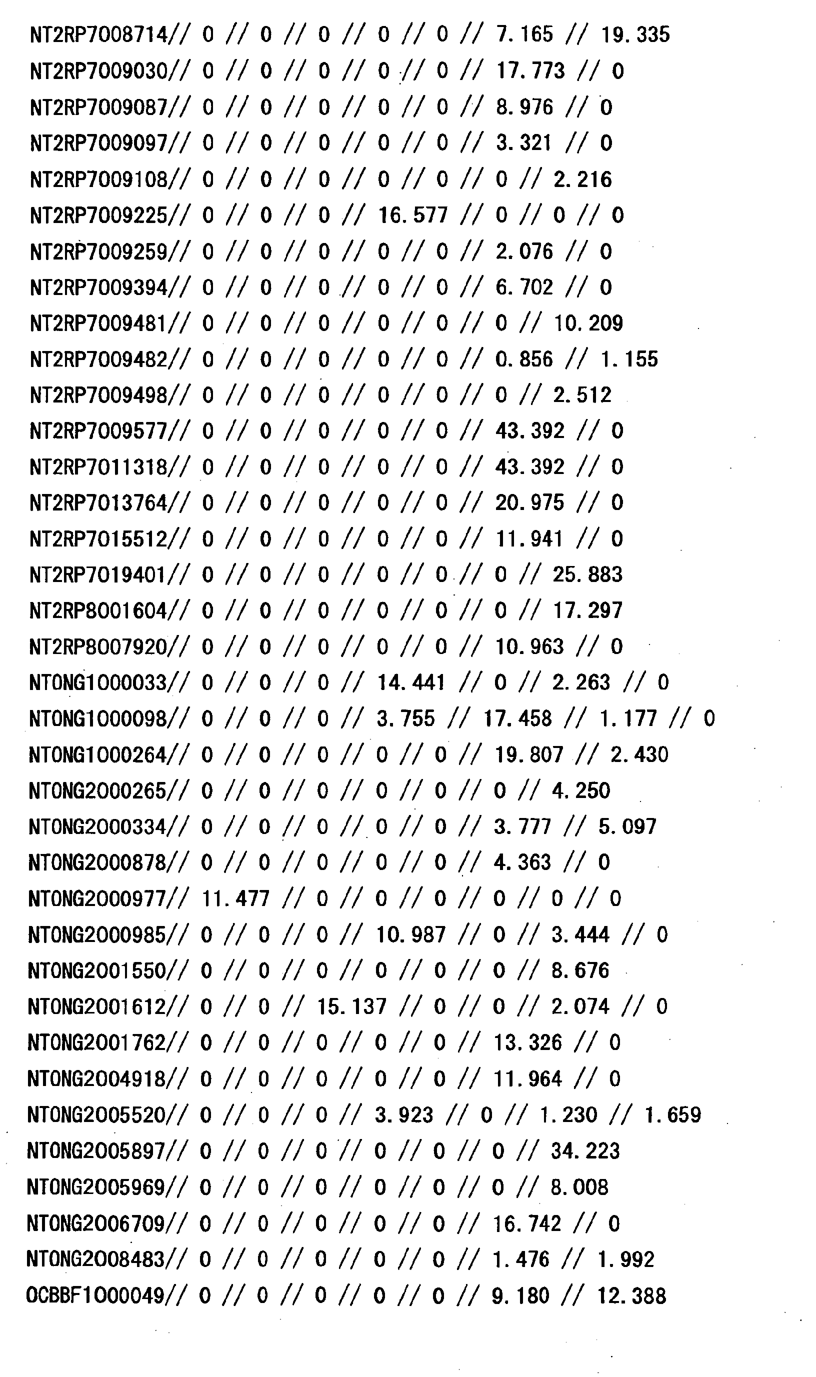

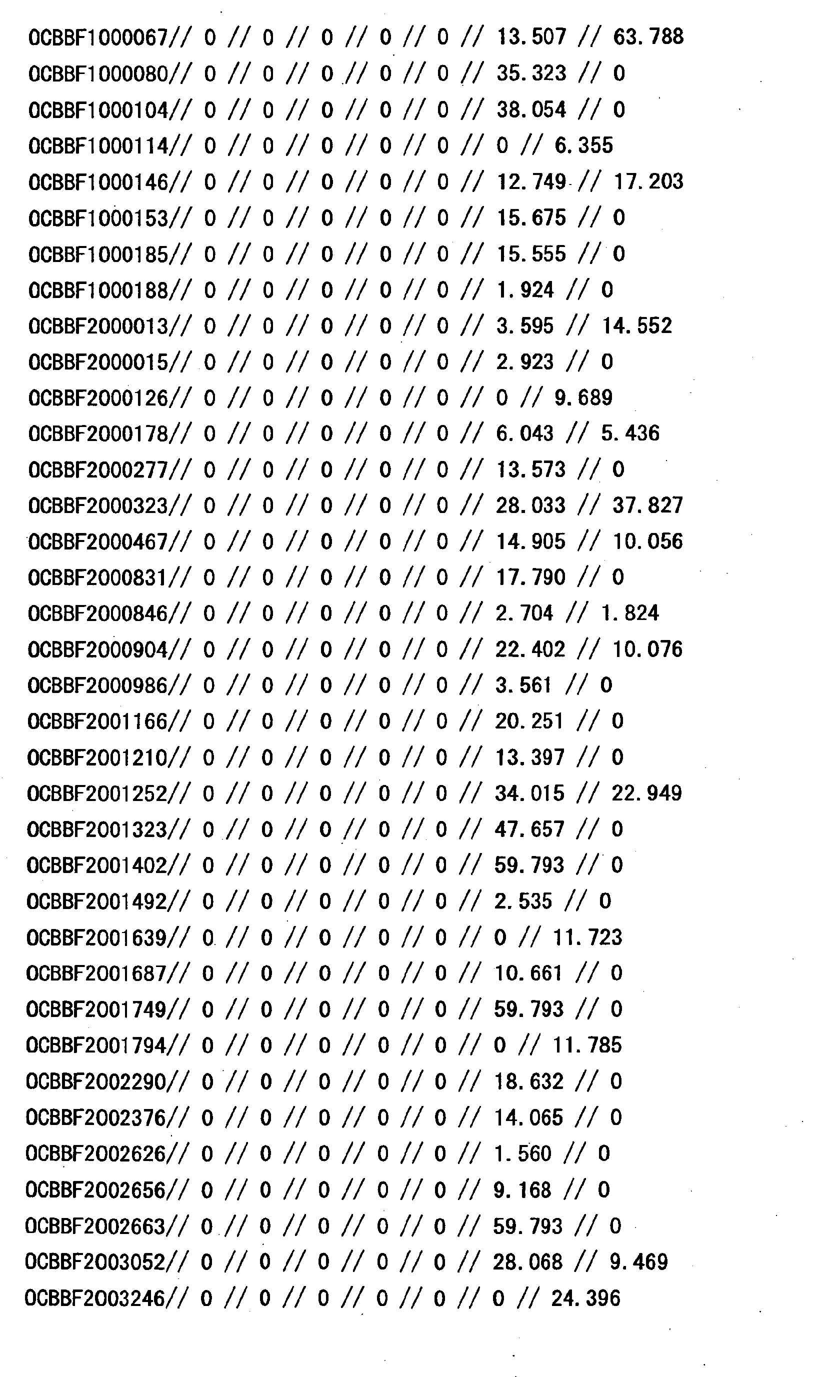

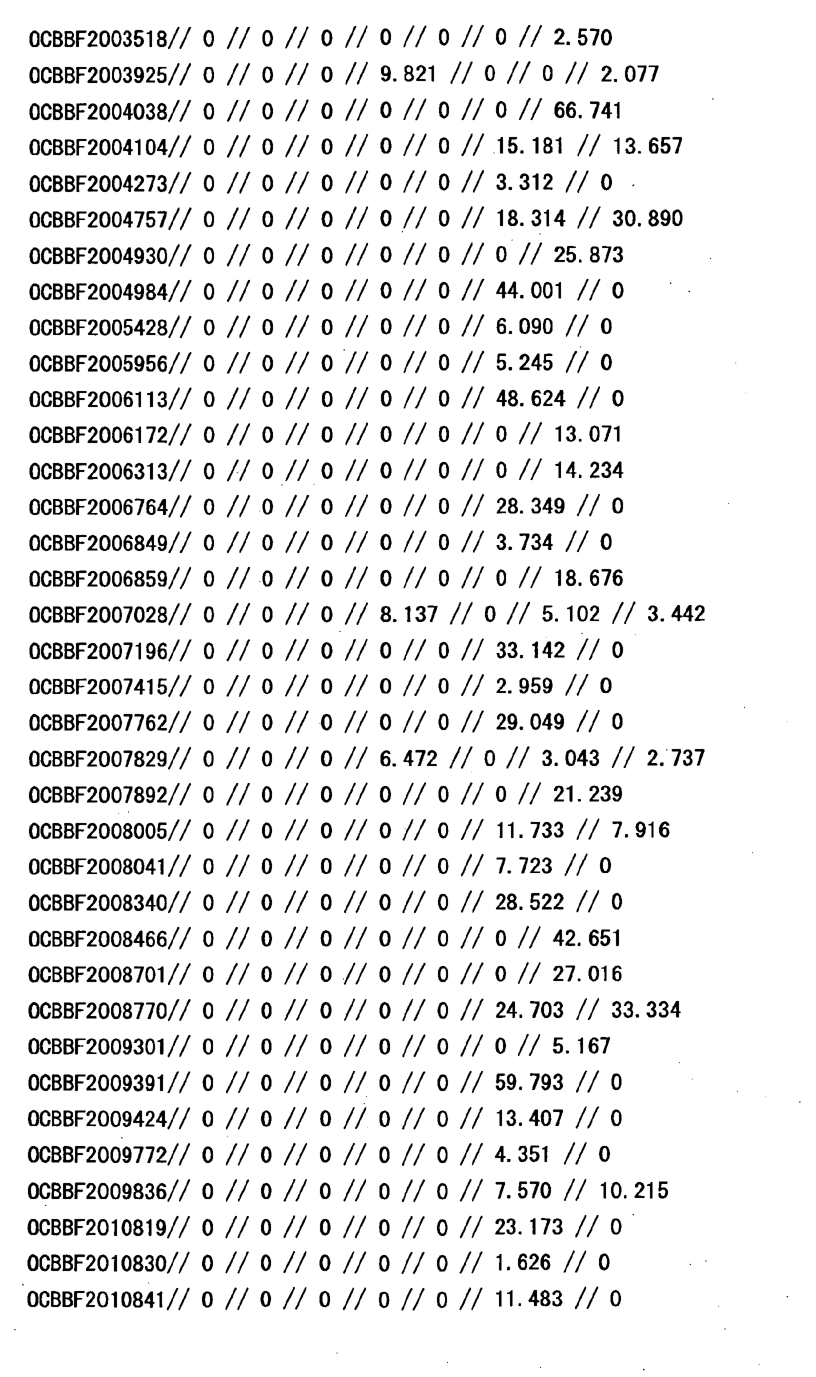

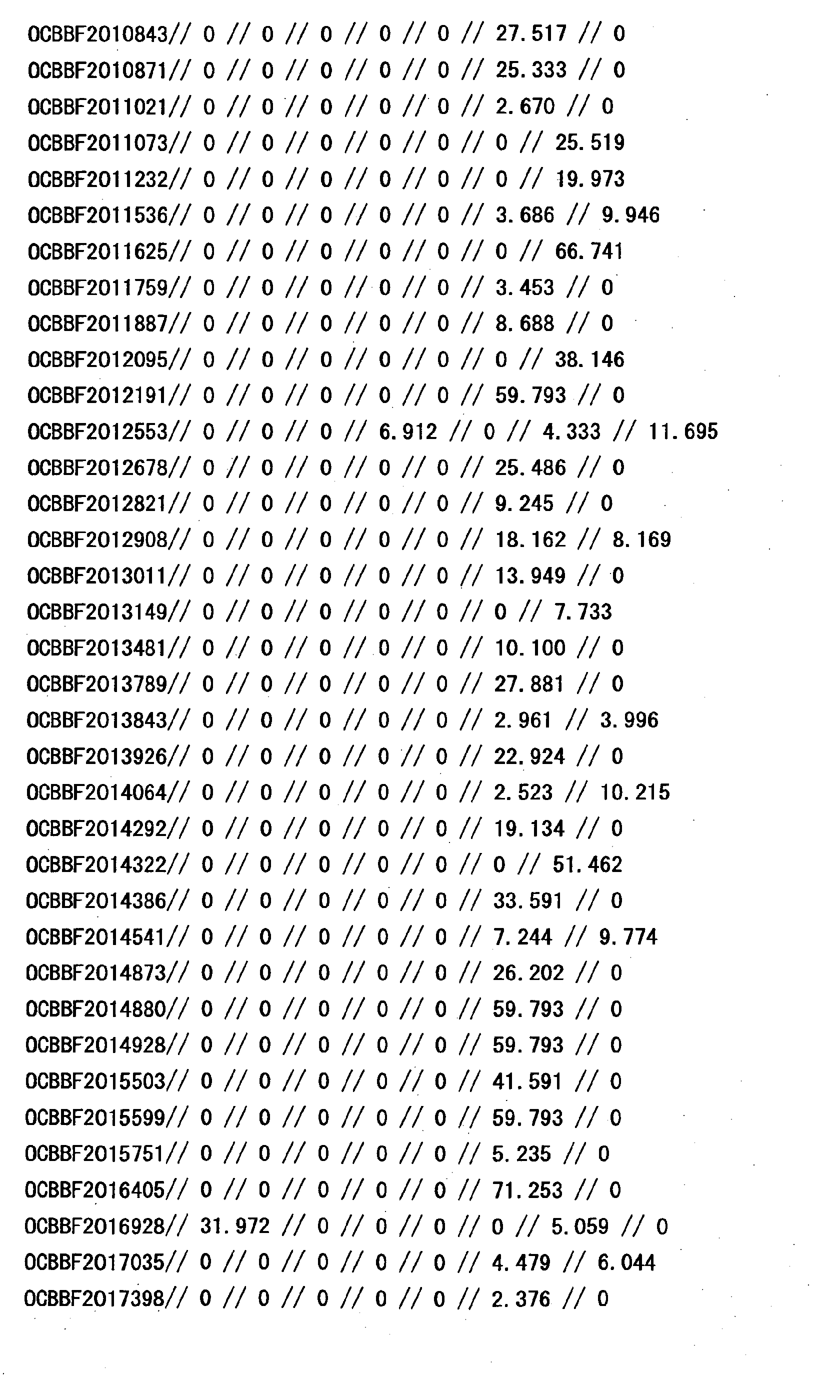

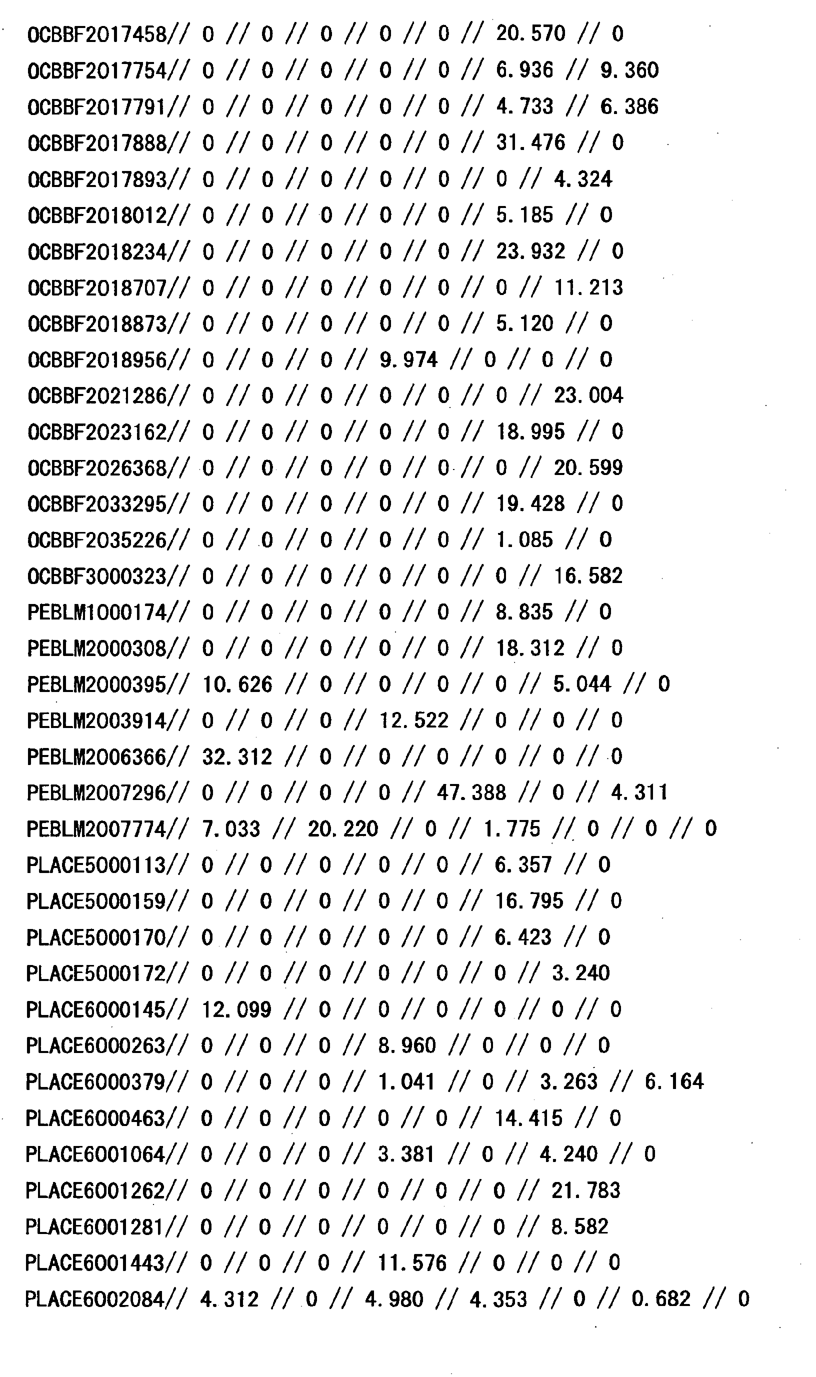

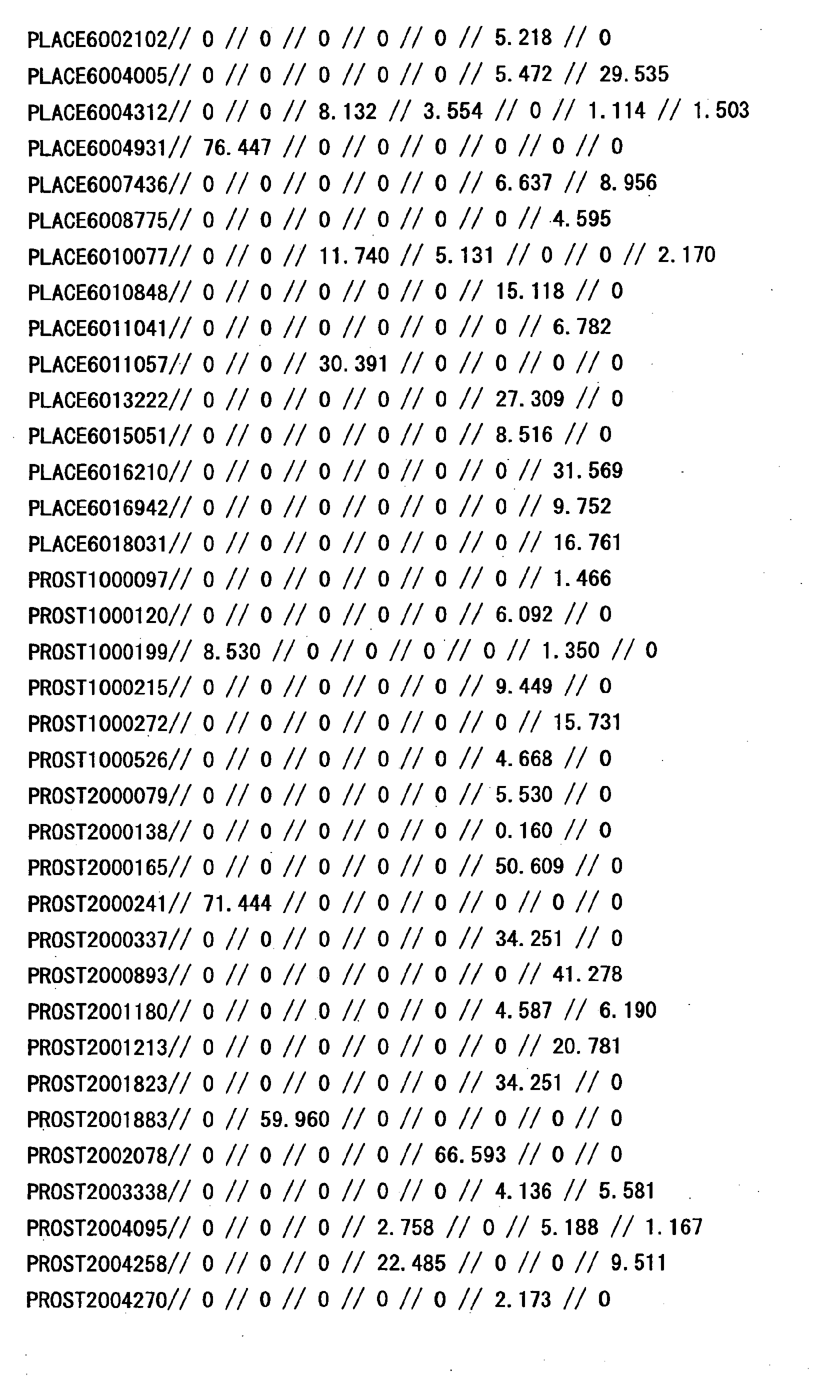

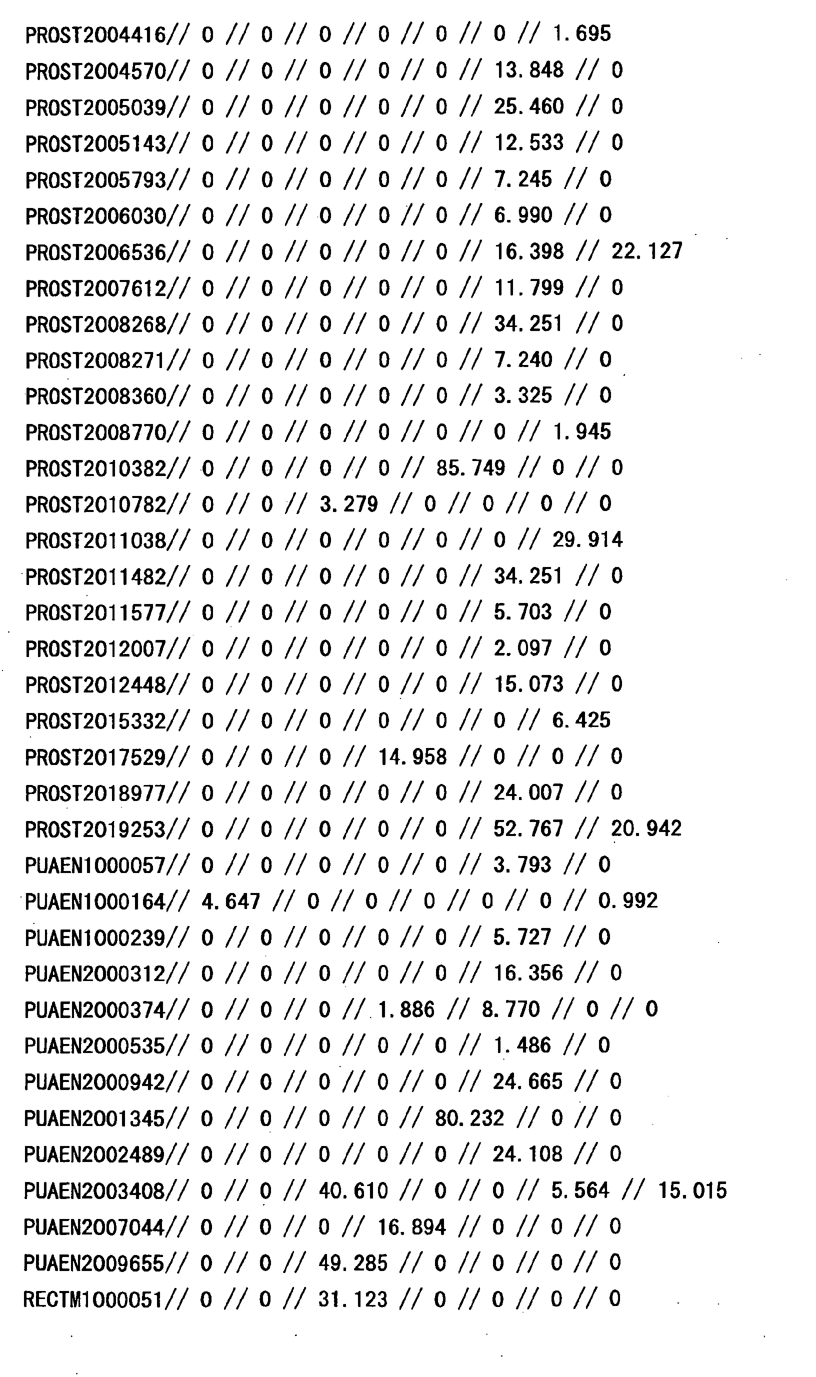

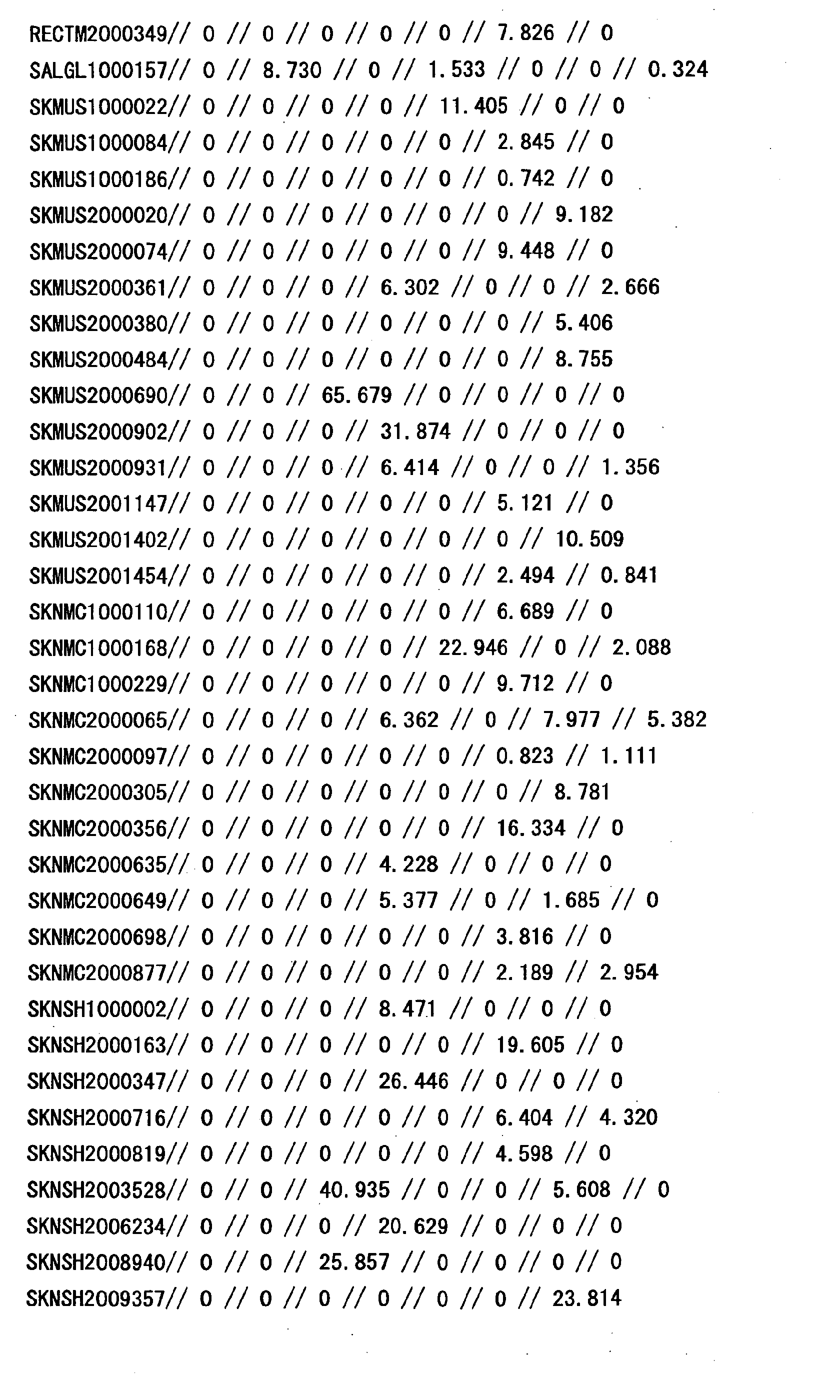

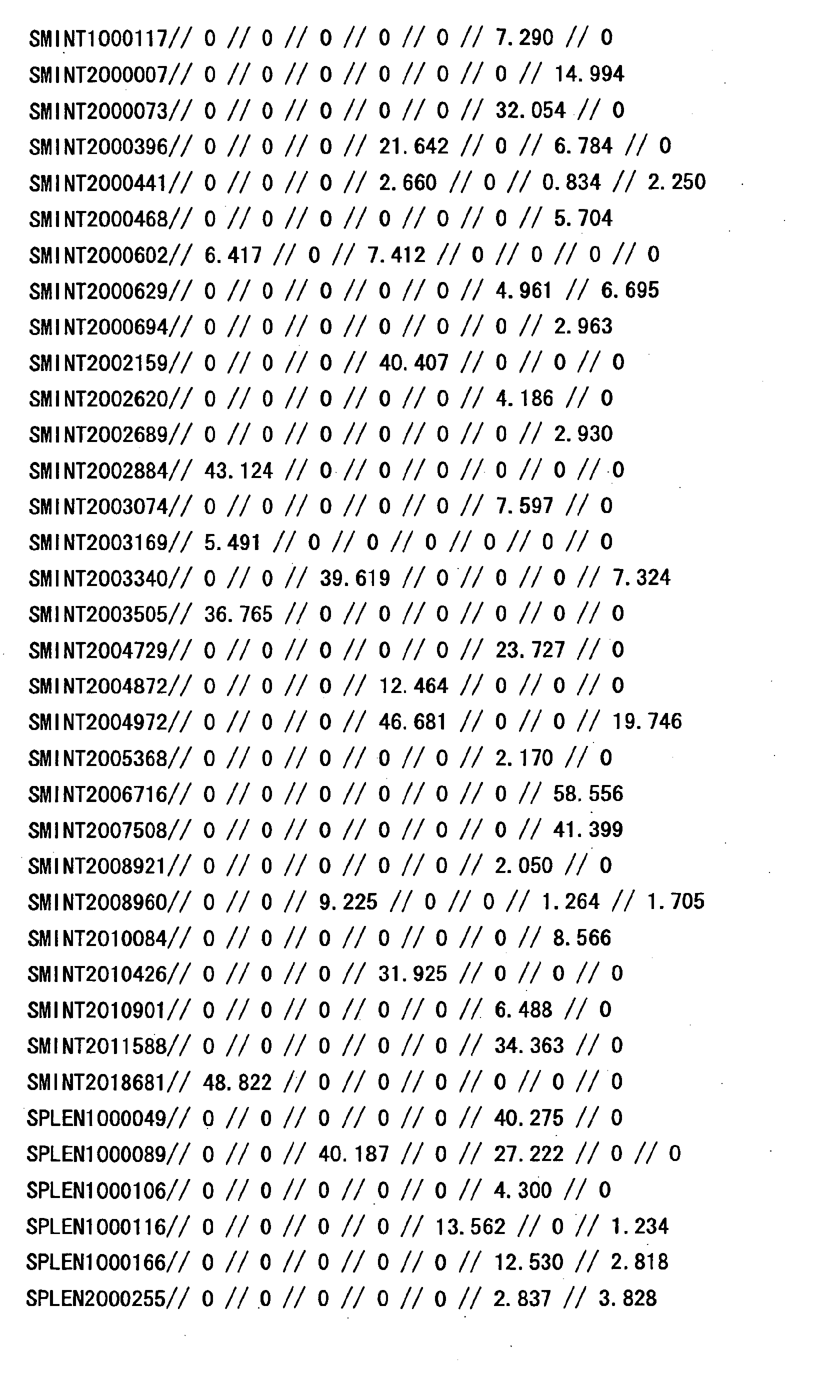

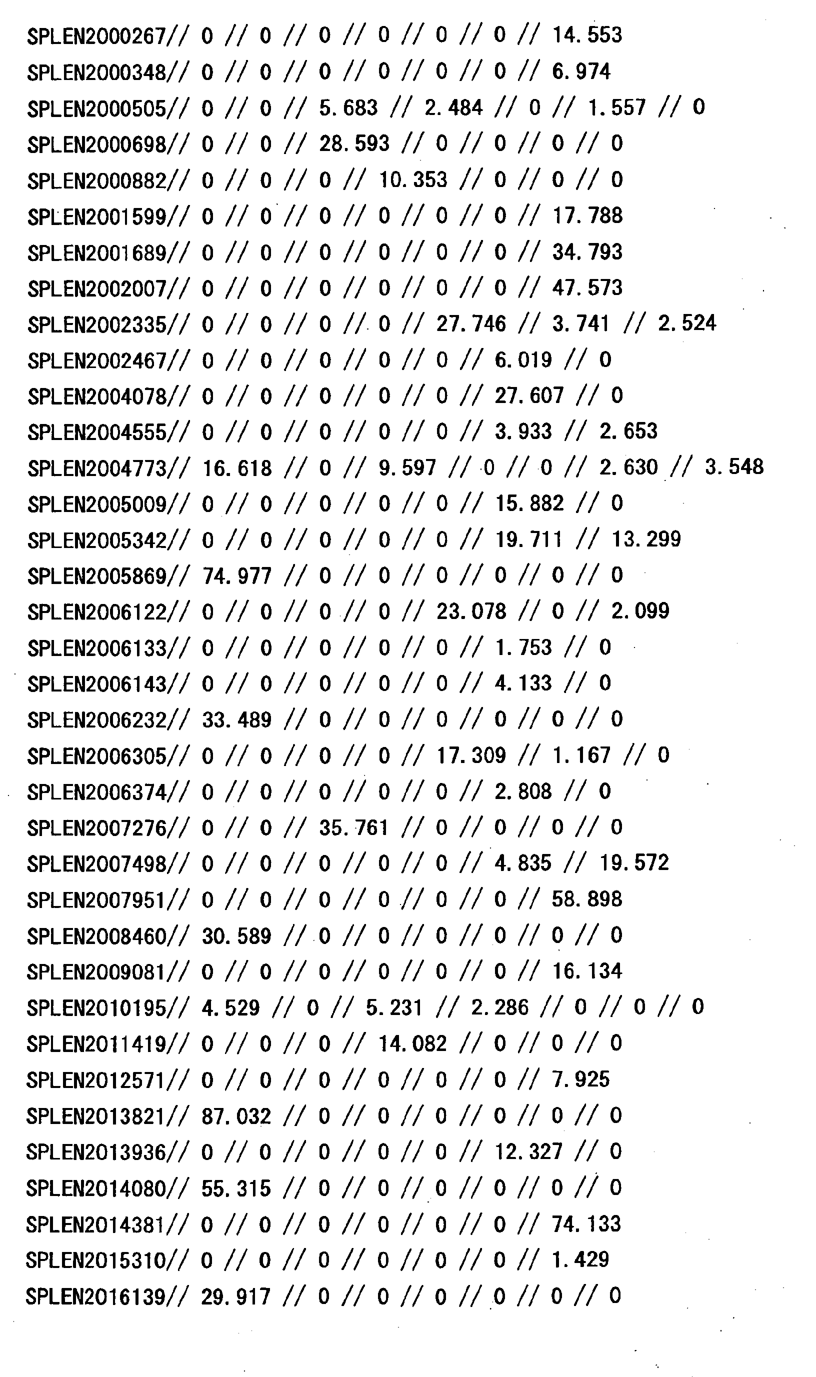

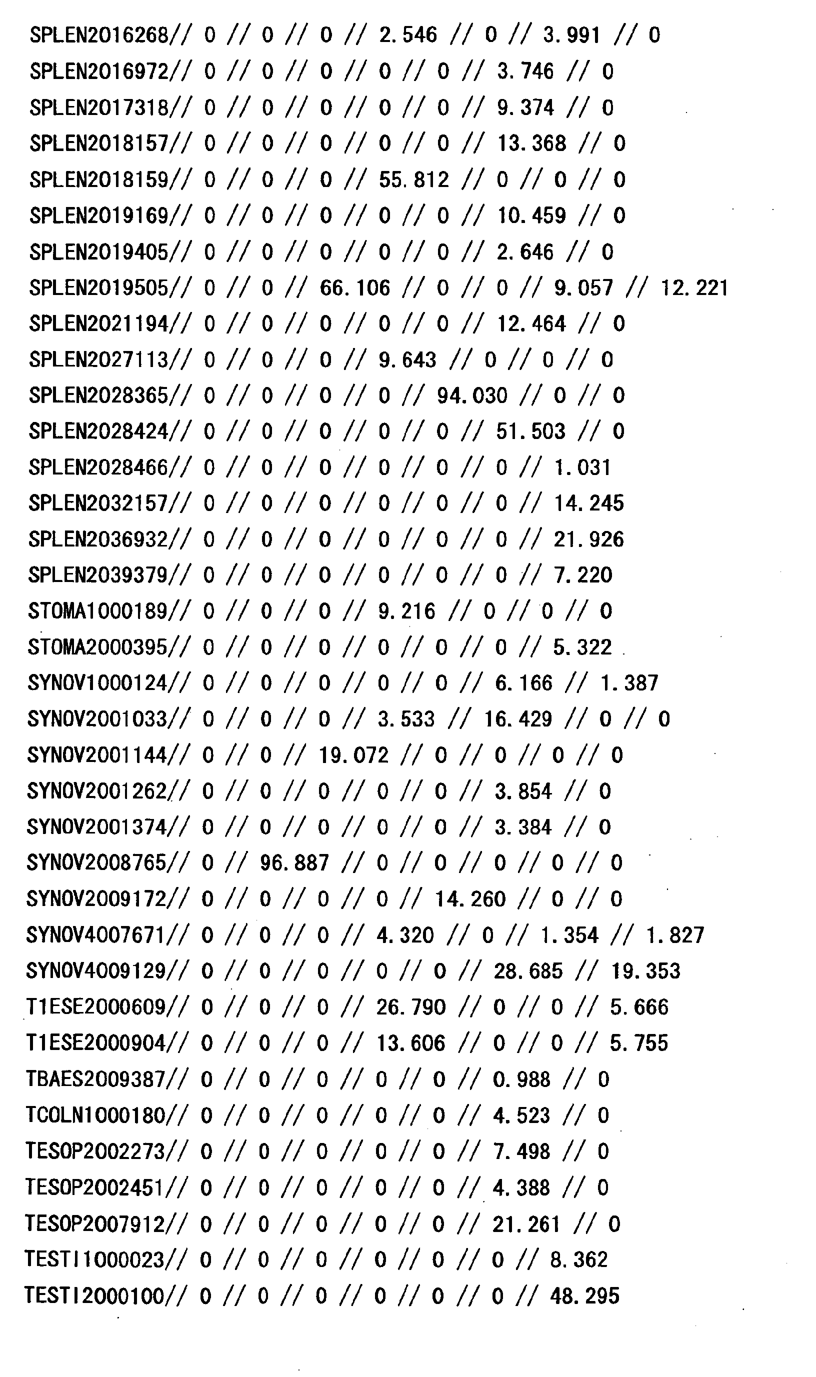

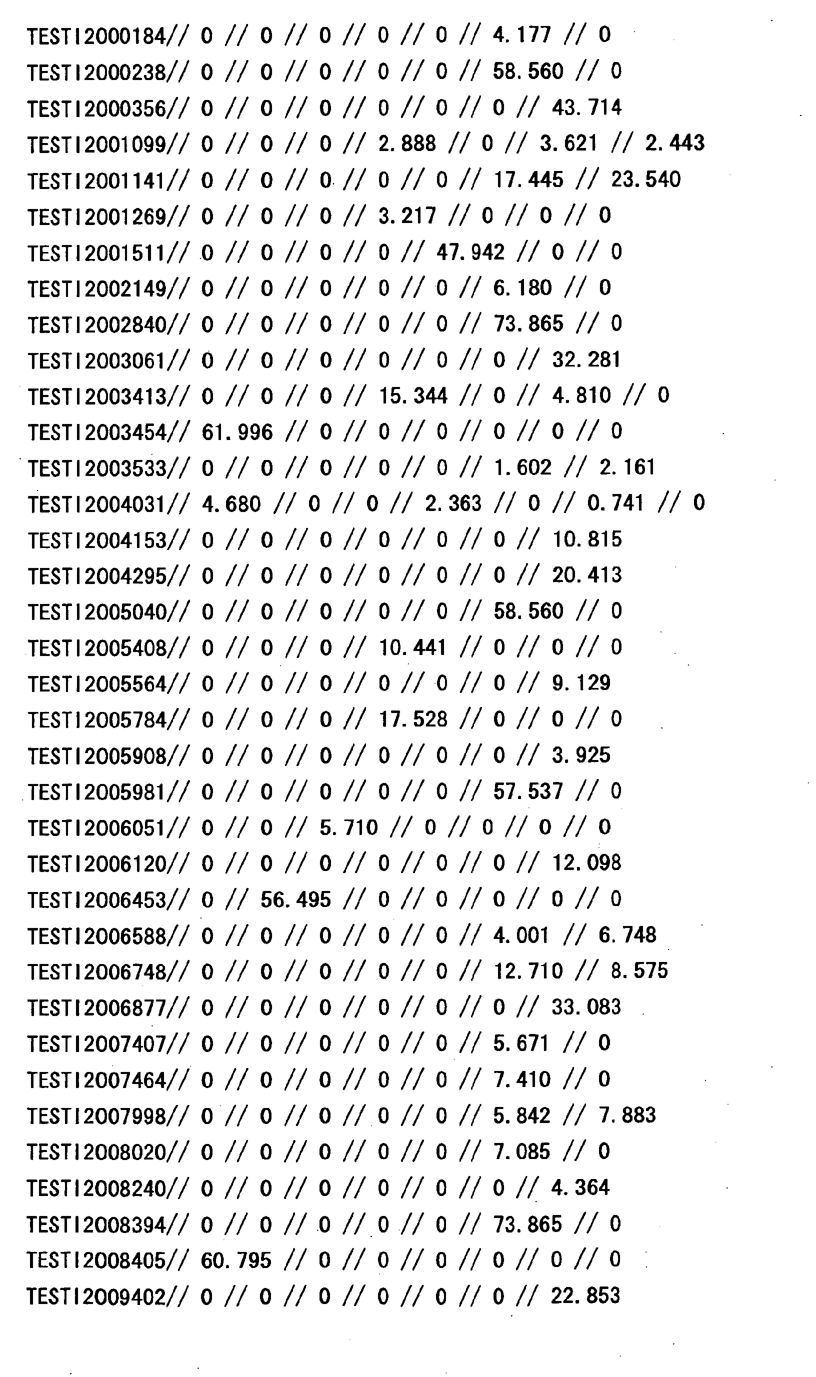

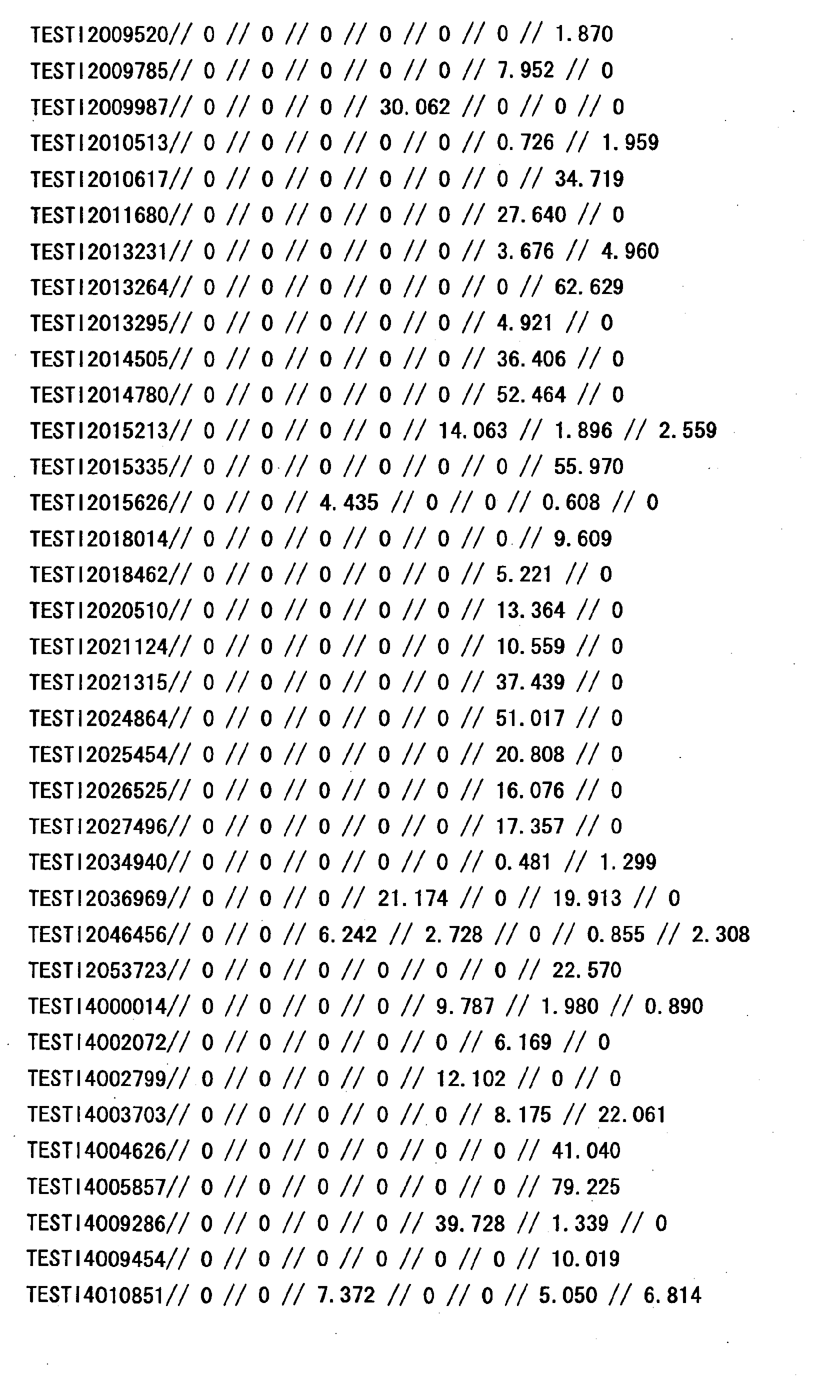

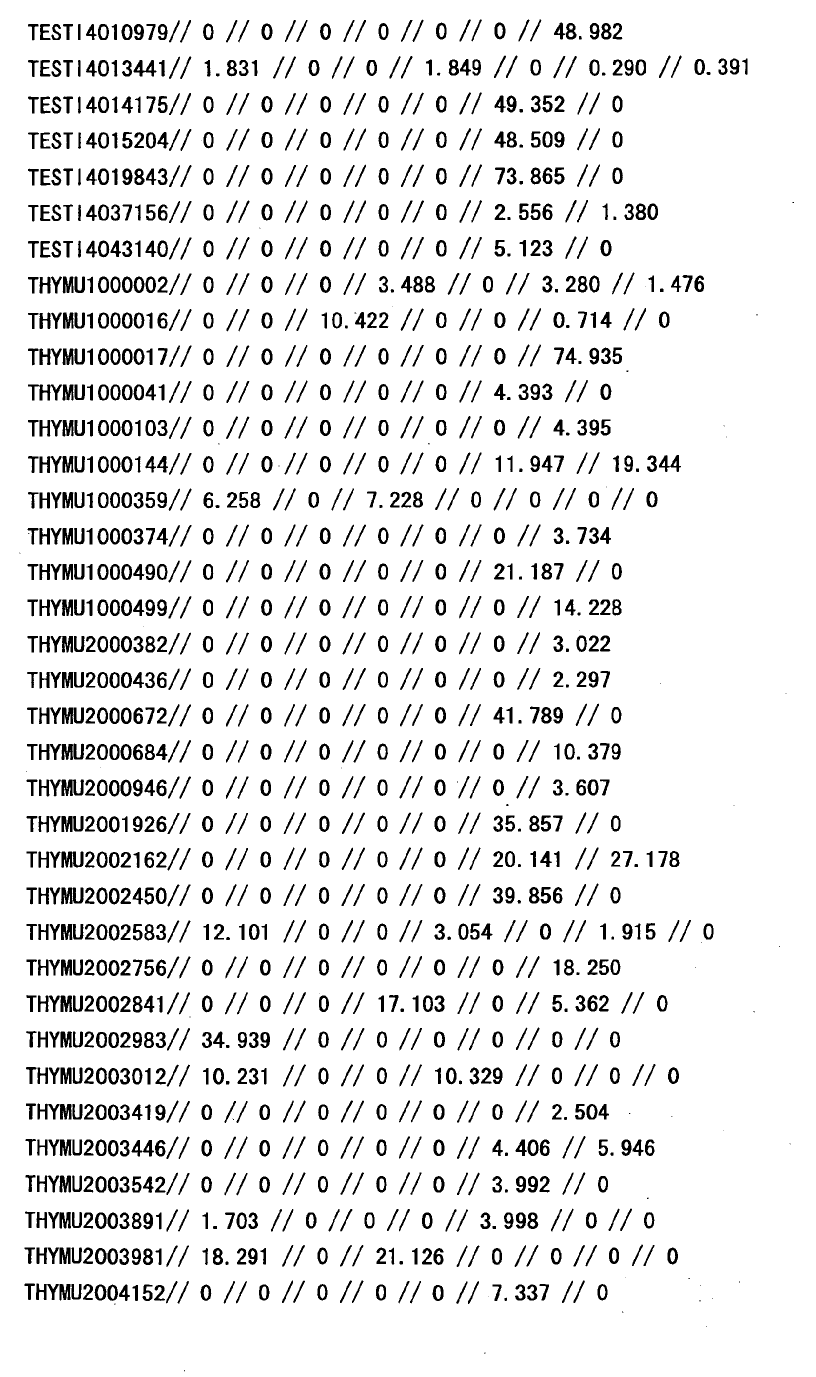

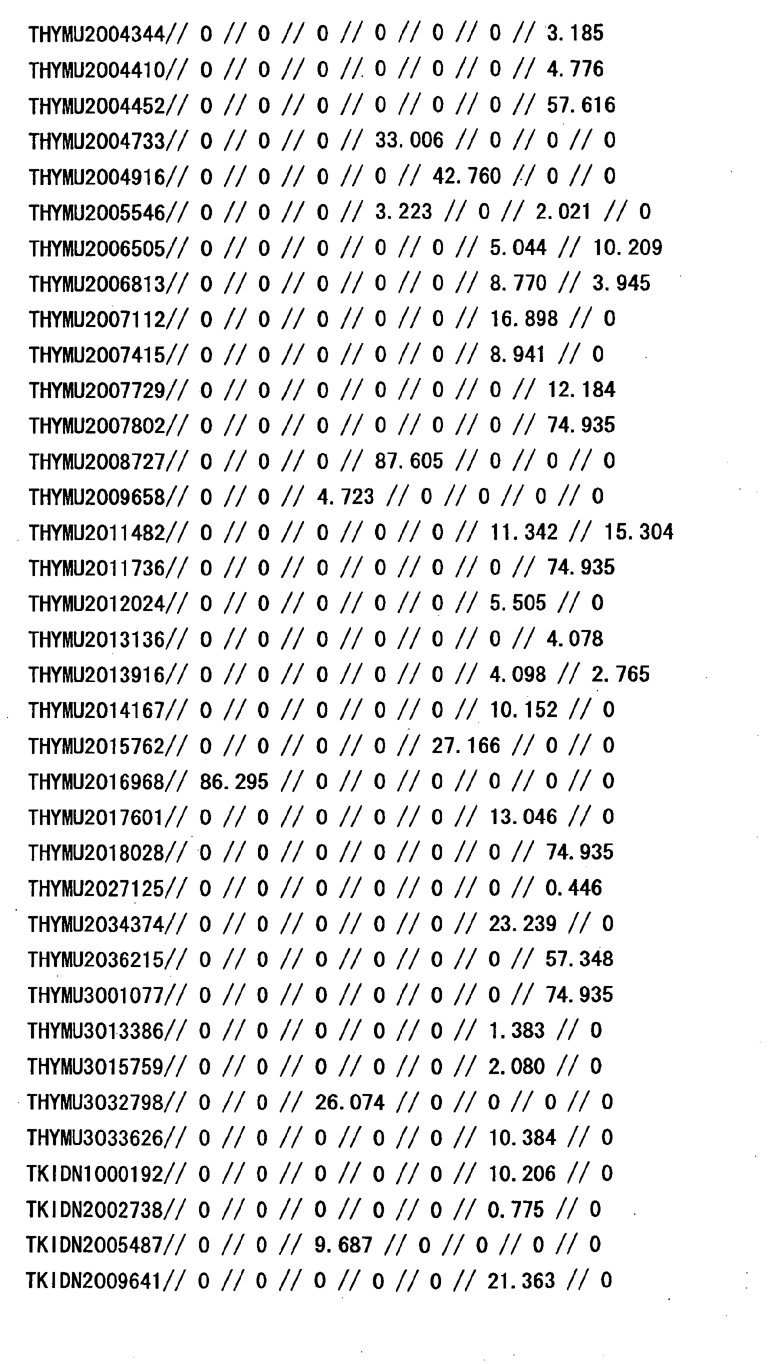

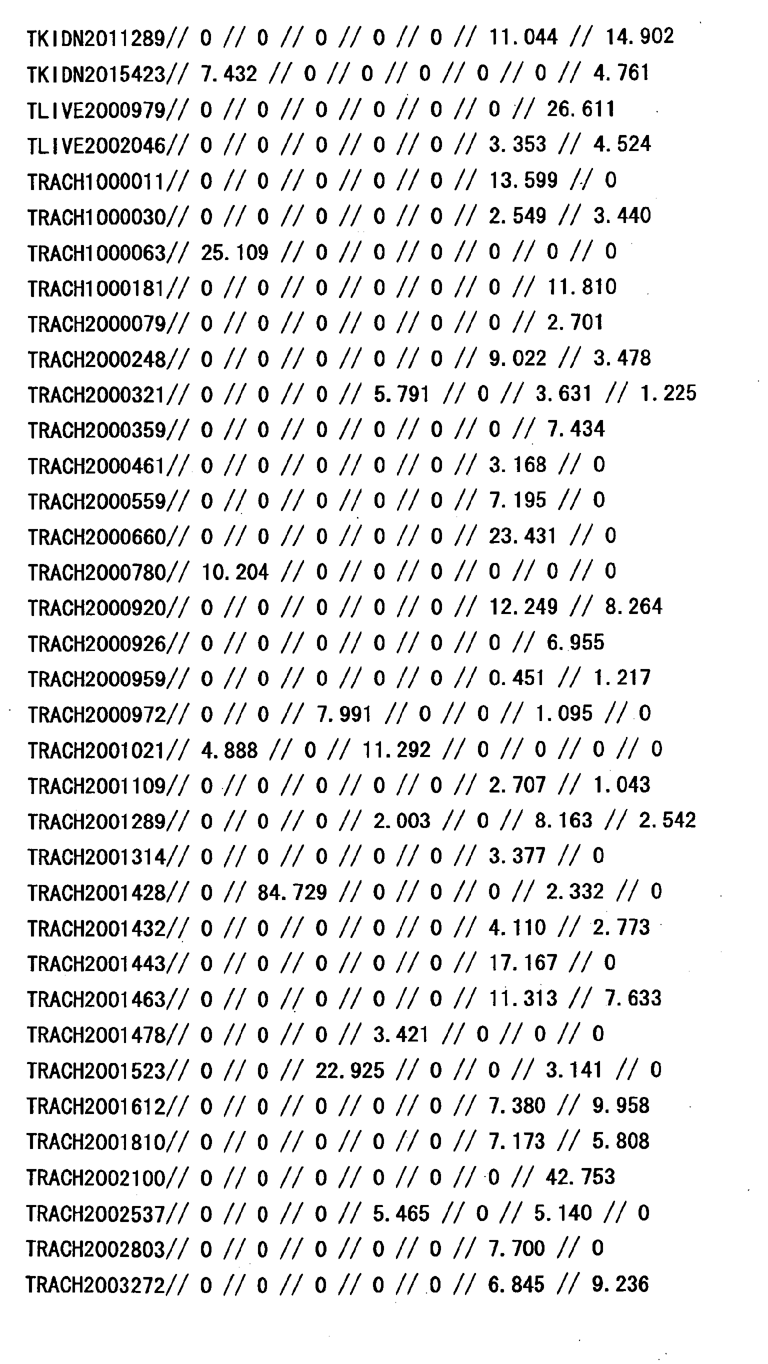

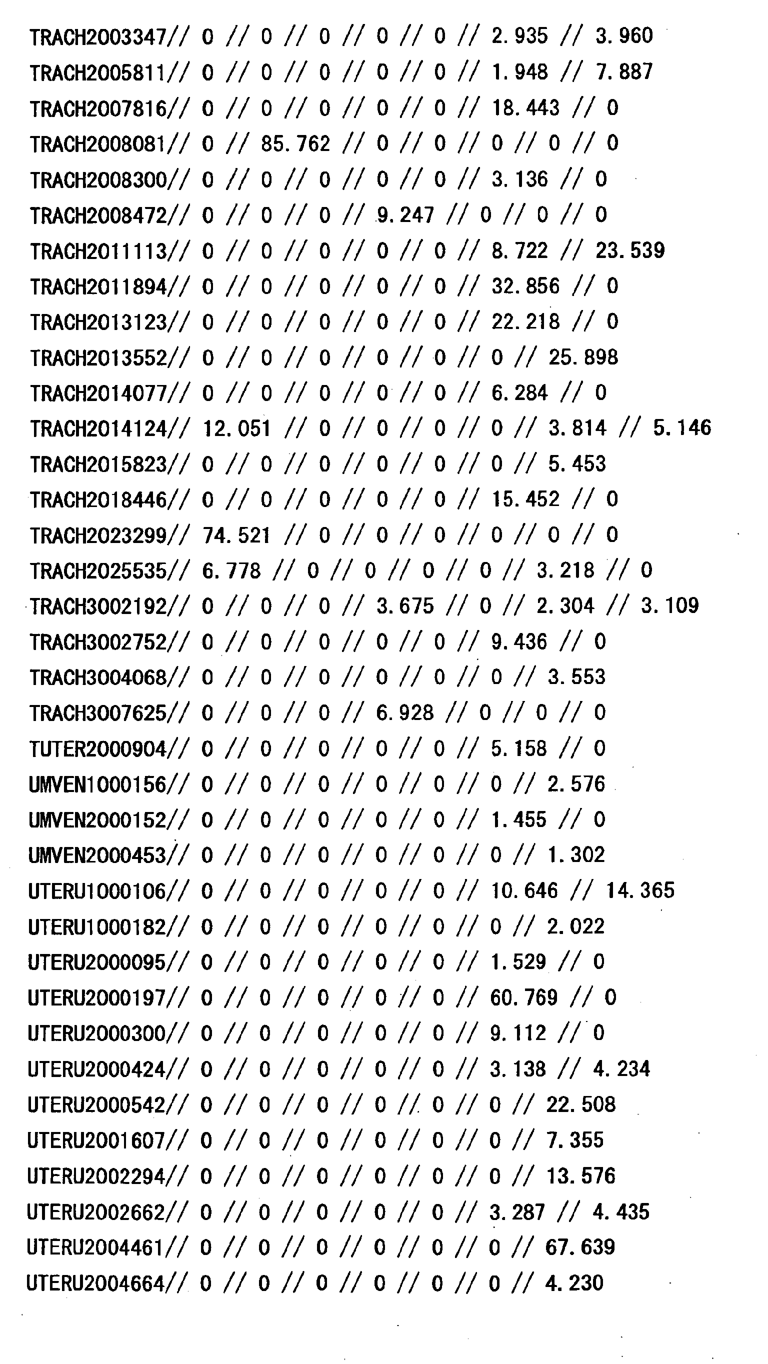

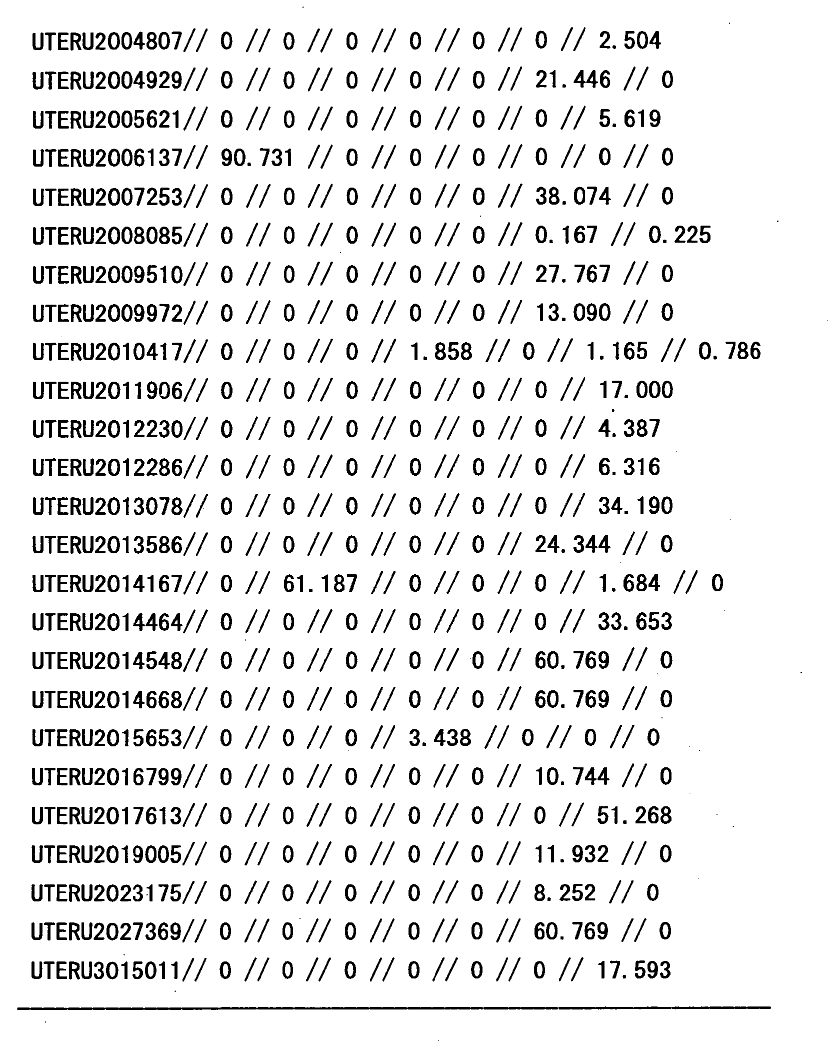

Genes whose expression levels were negligibly low in each of the above-described libraries are shown with their clone names below.
3NB691000018, 3NB691000085, 3NB691000113, 3NB691000116, 3NB691000129, 3NB691000191, 3NB692000012, 3NB692000029, 3NB692000102, 3NB692000154, 3NB692000276, 3NB692000305, 3NB692000429, 3NB692000529, 3NB692000973, 3NB692001002, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692008178, 3NB692008729, ACTVT2000380, ADIPS1000064, ADIPS2000069, ADIPS2000088, ADIPS2000245, ADRGL1000038, ADRGL1000067, ADRGL1000111, ADRGL1000144, ADRGL1000147, ADRGL1000160, ADRGL1000165, ADRGL1000182, ADRGL2000042, ADRGL2000056, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO2000011, ASTRO2000095, ASTRO2000191, ASTRO2000278, ASTRO2000372, ASTRO2000381, ASTRO2000403, ASTRO2000417, ASTRO2000480, ASTRO2000482, ASTRO2000533, ASTRO2000653, ASTRO2000662, ASTRO2000725, ASTRO2000801, ASTRO2000914, ASTRO2001001, ASTRO2001008, ASTRO2001076, ASTRO2001088, ASTRO2001107, ASTRO2001122, ASTRO2001227, ASTRO2001249, ASTRO2001480, ASTRO2001806, ASTRO2002024, ASTRO2002035, ASTRO2002064, ASTRO2002459, ASTRO2002571, ASTRO2002632, ASTRO2002659, ASTRO2002693, ASTRO2002720, ASTRO2002733, ASTRO2002743, ASTRO2002842, ASTRO2003024, ASTRO2003316, ASTRO2003574, ASTRO2003581, ASTRO2003632, ASTRO2003740, ASTRO2003840, ASTRO2003925, ASTRO2003960, ASTRO2004051, ASTRO2004177, ASTRO2004217, ASTRO2004297, ASTRO2004584, ASTRO2004628, ASTRO2004751, ASTRO2004877, ASTRO2005008, ASTRO2005081, ASTRO2005096, ASTRO2005242, ASTRO2005273, ASTRO2005343, ASTRO2005360, ASTRO2005413, ASTRO2005423, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006952, ASTRO2007221, ASTRO2007377, ASTRO2007515, ASTRO2007578, ASTRO2007643, ASTRO2007666, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014923, ASTRO2014961, ASTRO2015162, ASTRO2015185, ASTRO2015328, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000533, BGGI12000544, BGGI12000616, BGGI12000684, BGGI12000693, BGGI12000839, BGGI12001002, BGGI12001241, BGGI12001247, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006415, BLADE2006607, BLADE2006826, BLADE2006830, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008479, BLADE2008539, BLADE2008809, BLADE2008995, BLADE2009452, BLADE2009479, BLADE2009593, BNGH41000104, BNGH41000118, BNGH41000137, BNGH41000153, BNGH41000169, BNGH41000177, BNGH41000190, BNGH41000198, BNGH41000216, BNGH41000235, BNGH42000130, BNGH42000132, BNGH42000360, BNGH42000405, BNGH42000532, BNGH42000675, BNGH42000791, BNGH42000815, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42006226, BNGH42006234, BNGH42007037, BNGH42007366, BNGH42007594, BNGH42007798, BNGH42008199, BNGH42008510, BNGH42008649, BNGH42008743, BNGH42008832, BNGH42008850, BNGH42008943, BNGH42009021, BRACE1000020, BRACE1000047, BRACE1000051, BRACE1000065, BRACE1000070, BRACE1000073, BRACE1000084, BRACE1000092, BRACE1000101, BRACE1000114, BRACE1000124, BRACE1000131, BRACE1000132, BRACE1000166, BRACE1000186, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000331, BRACE2000332, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000753, BRACE2000815, BRACE2000864, BRACE2000885, BRACE2000893, BRACE2000905, BRACE2000936, BRACE2000947, BRACE2000965, BRACE2000969, BRACE2000988, BRACE2001017, BRACE2001065, BRACE2001070, BRACE2001094, BRACE2001107, BRACE2001143, BRACE2001151, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001508, BRACE2001534, BRACE2001543, BRACE2001564, BRACE2001588, BRACE2001607, BRACE2001626, BRACE2001673, BRACE2001677, BRACE2001692, BRACE2001705, BRACE2001709, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001898, BRACE2001923, BRACE2001942, BRACE2001944, BRACE2001954, BRACE2001971, BRACE2001972, BRACE2001973, BRACE2002026, BRACE2002034, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002884, BRACE2002896, BRACE2002948, BRACE2003078, BRACE2003097, BRACE2003168, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003788, BRACE2003800, BRACE2003802, BRACE2003825, BRACE2003845, BRACE2003846, BRACE2003847, BRACE2003848, BRACE2003873, BRACE2003885, BRACE2003887, BRACE2003905, BRACE2003928, BRACE2003954, BRACE2003982, BRACE2003987, BRACE2005034, BRACE2005047, BRACE2005063, BRACE2005083, BRACE2005090, BRACE2005099, BRACE2005118, BRACE2005124, BRACE2005138, BRACE2005160, BRACE2005169, BRACE2005189, BRACE2005193, BRACE2005196, BRACE2005198, BRACE2005216, BRACE2005243, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005434, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005949, BRACE2005981, BRACE2005991, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006274, BRACE2006281, BRACE2006298, BRACE2006363, BRACE2006378, BRACE2006380, BRACE2006393, BRACE2006397, BRACE2006413, BRACE2006417, BRACE2006453, BRACE2006459, BRACE2006488, BRACE2006534, BRACE2006537, BRACE2006540, BRACE2006547, BRACE2006564, BRACE2006587, BRACE2006598, BRACE2006636, BRACE2006685, BRACE2006703, BRACE2006753, BRACE2006783, BRACE2006821, BRACE2006833, BRACE2006859, BRACE2006871, BRACE2006909, BRACE2006911, BRACE2006924, BRACE2006935, BRACE2006944, BRACE2006951, BRACE2006982, BRACE2006986, BRACE2007013, BRACE2007068, BRACE2007087, BRACE2007138, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563, BRACE2007567, BRACE2007577, BRACE2007621, BRACE2007640, BRACE2007646, BRACE2007663, BRACE2007682, BRACE2007685, BRACE2007708, BRACE2007727, BRACE2007749, BRACE2007760, BRACE2007761, BRACE2007764, BRACE2007767, BRACE2007769, BRACE2007784, BRACE2007786, BRACE2007798, BRACE2007799, BRACE2007801, BRACE2007836, BRACE2007868, BRACE2007888, BRACE2007902, BRACE2007920, BRACE2007944, BRACE2008008, BRACE2008015, BRACE2008021, BRACE2008097, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008488, BRACE2008495, BRACE2008500, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008847, BRACE2008857, BRACE2008861, BRACE2008867, BRACE2008881, BRACE2008883, BRACE2008917, BRACE2008919, BRACE2008948, BRACE2008960, BRACE2008968, BRACE2008999, BRACE2009014, BRACE2009016, BRACE2009031, BRACE2009053, BRACE2009122, BRACE2009131, BRACE2009185, BRACE2009188, BRACE2009212, BRACE2009221, BRACE2009235, BRACE2009238, BRACE2009270, BRACE2009272, BRACE2009274, BRACE2009275, BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010684, BRACE2010753, BRACE2010813, BRACE2010837, BRACE2010888, BRACE2010937, BRACE2010983, BRACE2011007, BRACE2011025, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012232, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013806, BRACE2013855, BRACE2013911, BRACE2014108, BRACE2014161, BRACE2014232, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025710, BRACE2025992, BRACE2026131, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY2000021, BRAMY2000025, BRAMY2000052, BRAMY2000076, BRAMY2000086, BRAMY2000151, BRAMY2000196, BRAMY2000231, BRAMY2000277, BRAMY2000388, BRAMY2000411, BRAMY2000508, BRAMY2000585, BRAMY2000653, BRAMY2000814, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007249, BRAMY2007255, BRAMY2007287, BRAMY2007308, BRAMY2007411, BRAMY2007458, BRAMY2007486, BRAMY2007546, BRAMY2007594, BRAMY2007610, BRAMY2007653, BRAMY2007753, BRAMY2007859, BRAMY2008333, BRAMY2008382, BRAMY2008563, BRAMY2008583, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009693, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2020050, BRAMY2020058, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022525, BRAMY2022532, BRAMY2022723, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2024004, BRAMY2024073, BRAMY2024312, BRAMY2024374, BRAMY2024390, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269., BRAMY2028472, BRAMY2028491, BRAMY2028516, BRAMY2028565, BRAMY2028593, BRAMY2028682, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008088, BRAMY3008096, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010008, BRAMY3010264, BRAMY3010321, BRAMY3010411, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000083, BRAWH1000094, BRAWH1000116, BRAWH1000130, BRAWH1000157, BRAWH1000164, BRAWH1000168, BRAWH1000174, BRAWH1000180, BRAWH1000369, BRAWH2000034, BRAWH2000048, BRAWH2000071, BRAWH2000093, BRAWH2000109, BRAWH2000131, BRAWH2000142, BRAWH2000177, BRAWH2000218, BRAWH2000232, BRAWH2000248, BRAWH2000256, BRAWH2000323, BRAWH2000353, BRAWH2000370, BRAWH2000409, BRAWH2000417, BRAWH2000443, BRAWH2000471, BRAWH2000476, BRAWH2000482, BRAWH2000488, BRAWH2000494, BRAWH2000500, BRAWH2000503, BRAWH2000554, BRAWH2000595, BRAWH2000633, BRAWH2000643, BRAWH2000651, BRAWH2000682, BRAWH2000697, BRAWH2000752, BRAWH2000859, BRAWH2000866, BRAWH2000892, BRAWH2000901, BRAWH2000926, BRAWH2000944, BRAWH2000984, BRAWH2001019, BRAWH2001052, BRAWH2001103, BRAWH2001129, BRAWH2001137, BRAWH2001159, BRAWH2001166, BRAWH2001186, BRAWH2001203, BRAWH2001238, BRAWH2001239, BRAWH2001255, BRAWH2001355, BRAWH2001378, BRAWH2001395, BRAWH2001406, BRAWH2001412, BRAWH2001418, BRAWH2001436, BRAWH2001438, BRAWH2001459, BRAWH2001461, BRAWH2001492, BRAWH2001503, BRAWH2001519, BRAWH2001530, BRAWH2001535, BRAWH2001589, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002963, BRAWH2002967, BRAWH2002976, BRAWH2003000, BRAWH2003135, BRAWH2003240, BRAWH2003307, BRAWH2003355, BRAWH2003366, BRAWH2003689, BRAWH2003693, BRAWH2003832, BRAWH2003948, BRAWH2003964, BRAWH2004044, BRAWH2004068, BRAWH2004078, BRAWH2004136, BRAWH2004217, BRAWH2004580, BRAWH2004731, BRAWH2004779, BRAWH2004842, BRAWH2004884, BRAWH2004923, BRAWH2005225, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006405, BRAWH2006526, BRAWH2006543, BRAWH2006622, BRAWH2006760, BRAWH2006960, BRAWH2006989, BRAWH2007069, BRAWH2007308, BRAWH2007406, BRAWH2007550, BRAWH2007570, BRAWH2007591, BRAWH2007658, BRAWH2007808, BRAWH2007825, BRAWH2007862, BRAWH2007890, BRAWH2008058, BRAWH2008128, BRAWH2008219, BRAWH2008292, BRAWH2008297, BRAWH2008366, BRAWH2008380, BRAWH2008540, BRAWH2008577, BRAWH2008790, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010354, BRAWH2010364, BRAWH2010536, BRAWH2010552, BRAWH2010584, BRAWH2010618, BRAWH2010619, BRAWH2010754, BRAWH2011066, BRAWH2011079, BRAWH2011096, BRAWH2011155, BRAWH2011166, BRAWH2011204, BRAWH2011286, BRAWH2011294, BRAWH2011400, BRAWH2011762, BRAWH2011795, BRAWH2011796, BRAWH2011823, BRAWH2011860, BRAWH2011920, BRAWH2011958, BRAWH2012054, BRAWH2012077, BRAWH2012162, BRAWH2012258, BRAWH2012326, BRAWH2012538, BRAWH2012540, BRAWH2012619, BRAWH2012698, BRAWH2012749, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014662, BRAWH2014717, BRAWH2014729, BRAWH2014876, BRAWH2014934, BRAWH2014954, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016785, BRAWH2016792, BRAWH2016800, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244, BRAWH3003343, BRAWH3003411, BRAWH3003522, BRAwH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000577, BRCAN2000620, BRCAN2000639, BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087, BRCOC1000094, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019934, BRCOC2020122, BRCOC2020142, BRHIP1000058, BRHIP1000072, BRHIP1000129, BRHIP2000021, BRHIP2000079, BRHIP2000087, BRHIP2000163, BRHIP2000210, BRHIP2000239, BRHIP2000245, BRHIP2000359, BRHIP2000445, BRHIP2000506, BRHIP2000532, BRHIP2000621, BRHIP2000691, BRHIP2000819, BRHIP2000826, BRHIP2000920, BRHIP2001074, BRHIP2001686, BRHIP2001782, BRHIP2001802, BRHIP2001886, BRHIP2001927, BRHIP2002122, BRHIP2002143, BRHIP2002172, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007616, BRHIP2007690, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010309, BRHIP2010444, BRHIP2010487, BRHIP2010571, BRHIP2010593, BRHIP2010610, BRHIP2010723, BRHIP2010744, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015356, BRHIP2015360, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017714, BRHIP2017780, BRHIP2017945, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019149, BRHIP2019177, BRHIP2019291, BRHIP2019494, BRHIP2019589, BRHIP2019641, BRHIP2019819, BRHIP2019884, BRHIP2019972, BRHIP2020182, BRHIP2020509, BRHIP2020622, BRHIP2020695, BRHIP2020743, BRHIP2020799, BRHIP2020842, BRHIP2020859, BRHIP2020930, BRHIP2021102, BRHIP2021289, BRHIP2021615, BRHIP2021744, BRHIP2021870, BRHIP2021929, BRHIP2022006, BRHIP2022054, BRHIP2022221, BRHIP2022228, BRHIP2022298, BRHIP2022326, BRHIP2022467, BRHIP2022708, BRHIP2023071, BRHIP2023229, BRHIP2023272, BRHIP2023309, BRHIP2023369, BRHIP2023438, BRHIP2023695, BRHIP2023735, BRHIP2023762, BRHIP2023860, BRHIP2023869, BRHIP2024046, BRHIP2024165, BRHIP2024201, BRHIP2024347, BRHIP2024398, BRHIP2024549, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026288, BRHIP2026346, BRHIP2026557, BRHIP2026723, BRHIP2026877, BRHIP2027017, BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000060, BRHIP3000070, BRHIP3000081, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002363, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3018278, BRHIP3018784, BRHIP3019643, BRHIP3019824, BRHIP3019880, BRHIP3019956, BRHIP3020155, BRHIP3020182, BRHIP3020733, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000059, BRSSN1000092, BRSSN1000153, BRSSN2000097, BRSSN2000175, BRSSN2000197, BRSSN2000244, BRSSN2000293, BRSSN2000295, BRSSN2000518, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011262, BRSSN2011738, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010107, BRSTN2010363, BRSTN2010416, BRSTN2010500, BRSTN2010569, BRSTN2010923, BRSTN2011211, BRSTN2011597, BRSTN2011688, BRSTN2011799, BRSTN2012284, BRSTN2012320, BRSTN2012380, BRSTN2012451, BRSTN2013171, BRSTN2013354, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016918, BRSTN2016992, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3003736, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009291, BRTHA3010135, BRTHA3010366, BRTHA3010469, BRTHA3010540, BRTHA3010717, BRTHA3011149, BRTHA3011187, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000056, CTONG1000062, CTONG1000086, CTONG1000088, CTONG1000093, CTONG1000097, CTONG1000113, CTONG1000137, CTONG1000165, CTONG1000175, CTONG1000180, CTONG1000181, CTONG1000220, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000411, CTONG2000465, CTONG2000469, CTONG2000480, CTONG2000561, CTONG2000589, CTONG2000766, CTONG2000771, CTONG2000819, CTONG2000827, CTONG2000846, CTONG2000855, CTONG2000977, CTONG2001226, CTONG2001366, CTONG2001413, CTONG2001428, CTONG2001513, CTONG2001524, CTONG2001533, CTONG2001598, CTONG2001681, CTONG2001724, CTONG2001749, CTONG2001800, CTONG2001820, CTONG2001858, CTONG2001877, CTONG2001932, CTONG2001955, CTONG2002066, CTONG2002095, CTONG2002143, CTONG2002395, CTONG2002417, CTONG2002418, CTONG2002520, CTONG2002558, CTONG2002590, CTONG2002709, CTONG2002721, CTONG2002766, CTONG2002816, CTONG2002832, CTONG2002841, CTONG2002965, CTONG2002999, CTONG2003028, CTONG2003100, CTONG2003115, CTONG2003189, CTONG2003245, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2005913, CTONG2006004, CTONG2006129, CTONG2006223, CTONG2006273, CTONG2006310, CTONG2006377, CTONG2006393, CTONG2006449, CTONG2006515, CTONG2006524, CTONG2006568, CTONG2006611, CTONG2006620, CTONG2006666, CTONG2006691, CTONG2006836, CTONG2006942, CTONG2006964, CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007613, CTONG2007623, CTONG2007681, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008405, CTONG2008466, CTONG2008562, CTONG2008577, CTONG2008595, CTONG2008621, CTONG2008689, CTONG2008720, CTONG2008721, CTONG2008773, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010348, CTONG2010408, CTONG2010508, CTONG2010623, CTONG2010652, CTONG2010803, CTONG2011188, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012029, CTONG2012077, CTONG2012101, CTONG2012123, CTONG2012158, CTONG2012230, CTONG2012422, CTONG2012425, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012847, CTONG2012879, CTONG2012901, CTONG2013128, CTONG2013149, CTONG2013156, CTONG2013178, CTONG2013222, CTONG2013284, CTONG2013339, CTONG2013352, CTONG2013393, CTONG2013511, CTONG2013630, CTONG2013803, CTONG2013934, CTONG2013986, CTONG2014032, CTONG2014058, CTONG2014119, CTONG2014191, CTONG2014206, CTONG2014234, CTONG2014264, CTONG2014280, CTONG2014369, CTONG2014491, CTONG2014630, CTONG2014635, CTONG2014646, CTONG2014705, CTONG2014946, CTONG2014954, CTONG2014959, CTONG2014966, CTONG2014995, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016499, CTONG2016505, CTONG2016559, CTONG2016658, CTONG2016775, CTONG2016797, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017500, CTONG2017604, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018379, CTONG2018383, CTONG2018413, CTONG2018483, CTONG2018629, CTONG2018632, CTONG2018652, CTONG2018653, CTONG2018655, CTONG2018735, CTONG2018808, CTONG2018900, CTONG2018976, CTONG2019029, CTONG2019144, CTONG2019232, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020445, CTONG2020638, CTONG2020806, CTONG2020831, CTONG2020974, CTONG2021024, CTONG2021132, CTONG2021168, CTONG2021289, CTONG2022153, CTONG2022601, CTONG2023021, CTONG2023449, CTONG2023512, CTONG2024031, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028208, CTONG2028309, CTONG2028486, CTONG2028518, CTONG2028687, CTONG2028758, CTONG3000657, CTONG3000686, CTONG3000707, CTONG3000896, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009287, CTONG3009328, CTONG3009385, FEHRT2000325, FEHRT2000364, FEKID1000018, FELIV1000101, FELIV1000137, FELIV1000164, FELNG2000241, HCASM1000021, HCASM1000050, HCASM1000061, HCASM1000130, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000131, HCHON1000142, HCHON1000176, HCHON1000191, HCHON2000038, HCHON2000062, HCHON2000087, HCHON2000100, HCHON2000156, HCHON2000199, HCHON2000226, HCHON2000265, HCHON2000271, HCHON2000315, HCHON2000322, HCHON2000344, HCHON2000364, HCHON2000373, HCHON2000395, HCHON2000417, HCHON2000418, HCHON2000473, HCHON2000475, HCHON2000503, HCHON2000626, HCHON2000660, HCHON2000663, HCHON2000673, HCHON2000699, HCHON2000705, HCHON2000815, HCHON2000817, HCHON2000818, HCHON2000819, HCHON2000851, HCHON2000898, HCHON2000935, HCHON2000956, HCHON2000994, HCHON2001084, HCHON2001108, HCHON2001110, HCHON2001116, HCHON2001161, HCHON2001214, HCHON2001233, HCHON2001269, HCHON2001359, HCHON2001450, HCHON2001453, HCHON2001497, HCHON2001505, HCHON2001519, HCHON2001523, HCHON2001535, HCHON2001546, HCHON2001548, HCHON2001577, HCHON2001598, HCHON2001604, HCHON2001607, HCHON2001646, HCHON2001712, HCHON2001768, HCHON2001801, HCHON2001853, HCHON2002123, HCHON2002194, HCHON2002241, HCHON2002247, HCHON2002354, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005802, HCHON2005921, HCHON2005987, HCHON2006250, HCHON2006459, HCHON2006714, HCHON2006722, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HEART1000010, HEART1000074, HEART1000088, HEART1000099, HEART1000102, HEART1000118, HEART1000132, HEART1000139, HEART1000142, HEART1000151, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006557, HEART2006787, HEART2006789, HEART2006909, HEART2007031, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC2000070, HHDPC2000102, HHDPC2000115, HHDPC2000258, HHDPC2000315, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HLUNG1000017, HLUNG1000024, HLUNG1000030, HLUNG1000034, HLUNG1000037, HLUNG1000053, HLUNG1000064, HLUNG1000076, HLUNG1000084, HLUNG1000099, HLUNG1000104, HLUNG1000105, HLUNG1000110, HLUNG1000151, HLUNG1000169, HLUNG2000004, HLUNG2000014, HLUNG2000027, HLUNG2000068, HLUNG2000116, HLUNG2000125, HLUNG2000130, HLUNG2000142, HLUNG2000174, HLUNG2000176, HLUNG2000217, HLUNG2000255, HLUNG2000281, HLUNG2000315, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000926, HLUNG2000955, HLUNG2000970, HLUNG2001013, HLUNG2001126, HLUNG2001144, HLUNG2001146, HLUNG2001214, HLUNG2001459, HLUNG2001507, HLUNG2001633, HLUNG2001677, HLUNG2001712, HLUNG2001814, HLUNG2001996, HLUNG2002006, HLUNG2002050, HLUNG2002085, HLUNG2002145, HLUNG2002295, HLUNG2002303, HLUNG2002405, HLUNG2002429, HLUNG2002465, HLUNG2002562, HLUNG2002648, HLUNG2002659, HLUNG2002707, HLUNG2002811, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004154, HLUNG2004170, HLUNG2004217, HLUNG2004273, HLUNG2004388, HLUNG2004416, HLUNG2004521, HLUNG2004534, HLUNG2004684, HLUNG2004707, HLUNG2004775, HLUNG2005012, HLUNG2005030, HLUNG2005045, HLUNG2005076, HLUNG2005133, HLUNG2005203, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA1000178, HSYRA2000044, HSYRA2000135, HSYRA2000137, HSYRA2000224, HSYRA2000237, HSYRA2000253, HSYRA2000255, HSYRA2000371, HSYRA2000424, HSYRA2000507, HSYRA2000510, HSYRA2000605, HSYRA2000629, HSYRA2000706, HSYRA2000760, HSYRA2000792, HSYRA2000828, HSYRA2000832, HSYRA2000925, HSYRA2000927, HSYRA2001003, HSYRA2001105, HSYRA2001142, HSYRA2001153, HSYRA2001232, HSYRA2001255, HSYRA2001332, HSYRA2001353, HSYRA2001420, HSYRA2001484, HSYRA2001552, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000158, IMR321000161, IMR321000165, IMR321000207, IMR321000230, IMR321000266, IMR322000010, IMR322000072, IMR322000107, IMR322000152, IMR322000164, IMR322000178, IMR322000302, IMR322000374, IMR322000379, IMR322000383, IMR322000418, IMR322000518, IMR322000604, IMR322000609, IMR322000656, IMR322000672, IMR322000742, IMR322000775, IMR322000791, IMR322000811, IMR322000838, IMR322000859, IMR322000863, IMR322000919, IMR322000953, IMR322000984, IMR322001018, IMR322001049, IMR322001167, IMR322001185, IMR322001245, IMR322001317, IMR322001318, IMR322001322, IMR322001438, IMR322001452, IMR322001491, IMR322001534, IMR322001665, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000145, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000266, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000422, KIDNE2000493, KIDNE2000497, KIDNE2000510, KIDNE2000513, KIDNE2000517, KIDNE2000519, KIDNE2000555, KIDNE2000624, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2000909, KIDNE2001117, KIDNE2001140, KIDNE2001269, KIDNE2001361, KIDNE2001373, KIDNE2001636, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002483, KIDNE2002725, KIDNE2002768, KIDNE2002795, KIDNE2002798, KIDNE2002839, KIDNE2002845, KIDNE2002882, KIDNE2002980, KIDNE2002991, KIDNE2003185, KIDNE2003188, KIDNE2003233, KIDNE2003305, KIDNE2003316, KIDNE2003335, KIDNE2003357, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004344, KIDNE2004394, KIDNE2004411, KIDNE2004519, KIDNE2004520, KIDNE2004531, KIDNE2004534, KIDNE2004562, KIDNE2004579, KIDNE2004681, KIDNE2004828, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006580, KIDNE2006652, KIDNE2006687, KIDNE2006733, KIDNE2006760, KIDNE2006775, KIDNE2006820, KIDNE2007005, KIDNE2007040, KIDNE2007077, KIDNE2007186, KIDNE2007222, KIDNE2007328, KIDNE2007356, KIDNE2007422, KIDNE2007569, KIDNE2007810, KIDNE2007811, KIDNE2007910, KIDNE2007944, KIDNE2007954, KIDNE2008022, KIDNE2008048, KIDNE2008069, KIDNE2008072, KIDNE2008116, KIDNE2008117, KIDNE2008218, KIDNE2008315, KIDNE2008362, KIDNE2008378, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126, LIVER1000139, LIVER1000155, LIVER1000165, LIVER1000175, LIVER1000224, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001608, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415, LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000173, MAMGL1000178, MAMGL1000182, MAMGL1000184, MESAN1000032, MESAN1000035, MESAN1000079, MESAN1000080, MESAN1000121, MESAN1000126, MESAN1000147, MESAN1000180, MESAN2000023, MESAN2000026, MESAN2000167, MESAN2000264, MESAN2000291, MESAN2000337, MESAN2000434, MESAN2000457, MESAN2000462, MESAN2000501, MESAN2000608, MESAN2000620, MESAN2000711, MESAN2000815, MESAN2000894, MESAN2000909, MESAN2001154, MESAN2001627, MESAN2001770, MESAN2001979, MESAN2001980, MESAN2002113, MESAN2002122, MESAN2002147, MESAN2002424, MESAN2002687, MESAN2002709, MESAN2002940, MESAN2002978, MESAN2003037, MESAN2003039, MESAN2003058, MESAN2003101, MESAN2003190, MESAN2003322, MESAN2003444, MESAN2003490, MESAN2003529, MESAN2003646, MESAN2003662, MESAN2003709, MESAN2003851, MESAN2004138, MESAN2005284, MESAN2005303, MESAN2005371, MESAN2005633, MESAN2005724, MESAN2005808, MESAN2005811, MESAN2005957, MESAN2005958, MESAN2006022, MESAN2006043, MESAN2006328, MESAN2006580, MESAN2006599, MESAN2006743, MESAN2006953, MESAN2007032, MESAN2007440, MESAN2007597, MESAN2008222, MESAN2008334, MESAN2008415, MESAN2008460, MESAN2008536, MESAN2008679, MESAN2008729, MESAN2008821, MESAN2009019, MESAN2009105, MESAN2009311, MESAN2009418, MESAN2009503, MESAN2009522, MESAN2009580, MESAN2010031, MESAN2010114, MESAN2010312, MESAN2010321, MESAN2010664, MESAN2010681, MESAN2011545, MESAN2011597, MESAN2011632, MESAN2011977, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000047, NB9N41000121, NB9N41000135, NB9N41000142, NB9N41000146, NB9N41000340, NB9N42000122, NB9N42000196, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000606, NHNPC2000877, NHNPC2001108, NHNPC2001312, NHNPC2001816, NHNPC2002565, NHNPC2002749, NOVAR1000015, NOVAR1000091, NOVAR1000102, NOVAR2000038, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000023, NT2NE1000059, NT2NE1000063, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000174, NT2NE2000195, NT2NE2000222, NT2NE2000259, NT2NE2000284, NT2NE2000299, NT2NE2000356, NT2NE2000369, NT2NE2000374, NT2NE2000384, NT2NE2000390, NT2NE2000455, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000864, NT2NE2000913, NT2NE2000950, NT2NE2000951, NT2NE2000963, NT2NE2000980, NT2NE2001005, NT2NE2001021, NT2NE2001040, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001364, NT2NE2001372, NT2NE2001397, NT2NE2001403, NT2NE2001428, NT2NE2001432, NT2NE2001524, NT2NE2001598, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003252, NT2NE2003308, NT2NE2003309, NT2NE2003315, NT2NE2003408, NT2NE2003457, NT2NE2003540, NT2NE2003571, NT2NE2003705, NT2NE2003752, NT2NE2003887, NT2NE2003921, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005890, NT2NE2005921, NT2NE2005968, NT2NE2006075, NT2NE2006217, NT2NE2006288, NT2NE2006382, NT2NE2006387, NT2NE2006427, NT2NE2006432, NT2NE2006478, NT2NE2006531, NT2NE2006659, NT2NE2006784, NT2NE2006813, NT2NE2006894, NT2NE2006942, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009426, NT2NE2009523, NT2NE2009608, NT2NE2009691, NT2NE2010105, NT2NE2010150, NT2NE2010400, NT2NE2010632, NT2NE2010633, NT2NE2010654, NT2NE2010688, NT2NE2010781, NT2NE2010854, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019099, NT2RI1000016, NT2RI1000027, NT2RI1000035, NT2RI1000164, NT2RI1000172, NT2RI2000004, NT2RI2000007, NT2RI2000107, NT2RI2000117, NT2RI2000128, NT2RI2000167, NT2RI2000173, NT2RI2000187, NT2RI2000263, NT2RI2000270, NT2RI2000276, NT2RI2000284, NT2RI2000313, NT2RI2000341, NT2RI2000421, NT2RI2000480, NT2RI2000575, NT2RI2000588, NT2RI2000597, NT2RI2000669, NT2RI2000671, NT2RI2000688, NT2RI2000689, NT2RI2000704, NT2RI2000738, NT2RI2000775, NT2RI2000822, NT2RI2000841, NT2RI2000865, NT2RI2000932, NT2RI2000974, NT2RI2000987, NT2RI2001010, NT2RI2001017, NT2RI2001030, NT2RI2001083, NT2RI2001167, NT2RI2001178, NT2RI2001230, NT2RI2001235, NT2RI2001244, NT2RI2001307, NT2RI2001409, NT2RI2001410, NT2RI2001425, NT2RI2001449, NT2RI2001474, NT2RI2001519, NT2RI2001595, NT2RI2001624, NT2RI2001726, NT2RI2001769, NT2RI2001782, NT2RI2001836, NT2RI2001846, NT2RI2001866, NT2RI2001910, NT2RI2002022, NT2RI2002041, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002447, NT2RI2002481, NT2RI2002507, NT2RI2002517, NT2RI2002530, NT2RI2002549, NT2RI2002564, NT2RI2002585, NT2RI2002592, NT2RI2002602, NT2RI2002729, NT2RI2002802, NT2RI2002822, NT2RI2002852, NT2RI2002865, NT2RI2002923, NT2RI2002926, NT2RI2002970, NT2RI2003011, NT2RI2003067, NT2RI2003184, NT2RI2003205, NT2RI2003222, NT2RI2003338, NT2RI2003344, NT2RI2003360, NT2RI2003392, NT2RI2003399, NT2RI2003419, NT2RI2003481, NT2RI2003487, NT2RI2003655, NT2RI2003667, NT2RI2003678, NT2RI2003884, NT2RI2004059, NT2RI2004078, NT2RI2004079, NT2RI2004093, NT2RI2004136, NT2RI2004157, NT2RI2004188, NT2RI2004190, NT2RI2004284, NT2RI2004290, NT2RI2004304, NT2RI2004312, NT2RI2004381, NT2RI2004442, NT2RI2004468, NT2RI2004535, NT2RI2004606, NT2RI2004608, NT2RI2004618, NT2RI2004783, NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005130, NT2RI2005166, NT2RI2005239, NT2RI2005246, NT2RI2005315, NT2RI2005358, NT2RI2005382, NT2RI2005473, NT2RI2005520, NT2RI2005621, NT2RI2005628, NT2RI2005647, NT2RI2005670, NT2RI2005710, NT2RI2005772, NT2RI2005814, NT2RI2005851, NT2RI2005966, NT2RI2006070, NT2RI2006071, NT2RI2006081, NT2RI2006126, NT2RI2006127, NT2RI2006257, NT2RI2006271, NT2RI2006294, NT2RI2006345, NT2RI2006403, NT2RI2006445, NT2RI2006487, NT2RI2006553, NT2RI2006565, NT2RI2006640, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006838, NT2RI2006853, NT2RI2006855, NT2RI2006973, NT2RI2007046, NT2RI2007048, NT2RI2007084, NT2RI2007096, NT2RI2007116, NT2RI2007148, NT2RI2007214, NT2RI2007303, NT2RI2007334, NT2RI2007386, NT2RI2007406, NT2RI2007439, NT2RI2007445, NT2RI2007469, NT2RI2007498, NT2RI2007507, NT2RI2007593, NT2RI2007729, NT2RI2007751, NT2RI2007754, NT2RI2007827, NT2RI2007853, NT2RI2007877, NT2RI2007879, NT2RI2007891, NT2RI2007956, NT2RI2007965, NT2RI2008007, NT2RI2008050, NT2RI2008141, NT2RI2008188, NT2RI2008204, NT2RI2008233, NT2RI2008455, NT2RI2008481, NT2RI2008502, NT2RI2008598, NT2RI2008622, NT2RI2008656, NT2RI2008714, NT2RI2008801, NT2RI2008812, NT2RI2008841, NT2RI2008952, NT2RI2009005, NT2RI2009066, NT2RI2009083, NT2RI2009101, NT2RI2009103, NT2RI2009144, NT2RI2009151, NT2RI2009215, NT2RI2009289, NT2RI2009301, NT2RI2009517, NT2RI2009583, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2016128, NT2RI2016134, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007543, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000032, NT2RP6000033, NT2RP6000039, NT2RP6000043, NT2RP6000059, NT2RP6000060, NT2RP6000077, NT2RP6000085, NT2RP6000086, NT2RP6000100, NT2RP6000119, NT2RP6000127, NT2RP6000130, NT2RP6000140, NT2RP6000150, NT2RP7000041, NT2RP7000069, NT2RP7000086, NT2RP7000109, NT2RP7000112, NT2RP7000259, NT2RP7000311, NT2RP7000359, NT2RP7000364, NT2RP7000380, NT2RP7000391, NT2RP7000579, NT2RP7000586, NT2RP7000600, NT2RP7000812, NT2RP7000926, NT2RP7001074, NT2RP7001080, NT2RP7001156, NT2RP7001306, NT2RP7001319, NT2RP7001335, NT2RP7001364, NT2RP7001475, NT2RP7001532, NT2RP7001602, NT2RP7001678, NT2RP7001856, NT2RP7001962, NT2RP7002064, NT2RP7002151, NT2RP7002163, NT2RP7002243, NT2RP7002282, NT2RP7002376, NT2RP7002449, NT2RP7002450, NT2RP7002478, NT2RP7002554, NT2RP7002594, NT2RP7002603, NT2RP7002619, NT2RP7002650, NT2RP7002779, NT2RP7002829, NT2RP7002841, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003439, NT2RP7003629, NT2RP7003632, NT2RP7003647, NT2RP7003680, NT2RP7004027, NT2RP7004080, NT2RP7004114, NT2RP7004233, NT2RP7004260, NT2RP7004358, NT2RP7004396, NT2RP7004428, NT2RP7004541, NT2RP7004559, NT2RP7004641, NT2RP7004656, NT2RP7004687, NT2RP7004722, NT2RP7004766, NT2RP7004790, NT2RP7004911, NT2RP7004915, NT2RP7004961, NT2RP7004964, NT2RP7004975, NT2RP7005118, NT2RP7005205, NT2RP7005219, NT2RP7005323, NT2RP7005468, NT2RP7005493, NT2RP7005529, NT2RP7005629, NT2RP7005631, NT2RP7005669, NT2RP7005675, NT2RP7005750, NT2RP7005846, NT2RP7006033, NT2RP7006075, NT2RP7006141, NT2RP7006160, NT2RP7006162, NT2RP7006263, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006621, NT2RP7006636, NT2RP7006701, NT2RP7006853, NT2RP7006886, NT2RP7006887, NT2RP7006980, NT2RP7006995, NT2RP7007154, NT2RP7007177, NT2RP7007221, NT2RP7007226, NT2RP7007252, NT2RP7007310, NT2RP7007359, NT2RP7007381, NT2RP7007406, NT2RP7007480, NT2RP7007504, NT2RP7007537, NT2RP7007580, NT2RP7007594, NT2RP7007610, NT2RP7007617, NT2RP7007643, NT2RP7007766, NT2RP7007925, NT2RP7007930, NT2RP7007975, NT2RP7008013, NT2RP7008133, NT2RP7008137, NT2RP7008142, NT2RP7008144, NT2RP7008161, NT2RP7008315, NT2RP7008360, NT2RP7008396, NT2RP7008406, NT2RP7008435, NT2RP7008454, NT2RP7008487, NT2RP7008543, NT2RP7008544, NT2RP7008550, NT2RP7008557, NT2RP7008623, NT2RP7008657, NT2RP7008720, NT2RP7008855, NT2RP7008863, NT2RP7009012, NT2RP7009019, NT2RP7009147, NT2RP7009149, NT2RP7009168, NT2RP7009215, NT2RP7009236, NT2RP7009253, NT2RP7009267, NT2RP7009322, NT2RP7009363, NT2RP7009370, NT2RP7009373, NT2RP7009397, NT2RP7009429, NT2RP7009439, NT2RP7009466, NT2RP7009502, NT2RP7009507, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7014910, NT2RP7015080, NT2RP7015431, NT2RP7015503, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000246, NTONG1000247, NTONG1000257, NTONG2000040, NTONG2000107, NTONG2000231, NTONG2000363, NTONG2000413, NTONG2000492, NTONG2000499, NTONG2000531, NTONG2000583, NTONG2000800, NTONG2000858, NTONG2000876, NTONG2000966, NTONG2001014, NTONG2001079, NTONG2001137, NTONG2001157, NTONG2001173, NTONG2001220, NTONG2001222, NTONG2001362, NTONG2001428, NTONG2001532, NTONG2001567, NTONG2001587, NTONG2001619, NTONG2001642, NTONG2001657, NTONG2001676, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003839, NTONG2003852, NTONG2003897, NTONG2003928, NTONG2004095, NTONG2004308, NTONG2004521, NTONG2004614, NTONG2004690, NTONG2004806, NTONG2004829, NTONG2004844, NTONG2004991, NTONG2005062, NTONG2005086, NTONG2005137, NTONG2005153, NTONG2005265, NTONG2005277, NTONG2005363, NTONG2005373, NTONG2005391, NTONG2005480, NTONG2005497, NTONG2005577, NTONG2005657, NTONG2005801, NTONG2005822, NTONG2006099, NTONG2006187, NTONG2006301, NTONG2006324, NTONG2006354, NTONG2006440, NTONG2006484, NTONG2006501, NTONG2006646, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000054, OCBBF1000085, OCBBF1000086, OCBBF1000091, OCBBF1000118, OCBBF1000119, OCBBF1000122, OCBBF1000130, OCBBF1000138, OCBBF1000145, OCBBF1000175, OCBBF1000192, OCBBF1000254, OCBBF1000315, OCBBF2000231, OCBBF2000287, OCBBF2000452, OCBBF2000522, OCBBF2000677, OCBBF2000703, OCBBF2000712, OCBBF2000719, OCBBF2000824, OCBBF2000982, OCBBF2000998, OCBBF2001075, OCBBF2001101, OCBBF2001124, OCBBF2001140, OCBBF2001176, OCBBF2001186, OCBBF2001196, OCBBF2001307, OCBBF2001389, OCBBF2001408, OCBBF2001416, OCBBF2001473, OCBBF2001494, OCBBF2001527, OCBBF2001528, OCBBF2001586, OCBBF2001681, OCBBF2001706, OCBBF2001910, OCBBF2001931, OCBBF2001961, OCBBF2001983, OCBBF2002015, OCBBF2002083, OCBBF2002086, OCBBF2002124, OCBBF2002161, OCBBF2002357, OCBBF2002615, OCBBF2002805, OCBBF2002865, OCBBF2002920, OCBBF2002980, OCBBF2003028, OCBBF2003091, OCBBF2003327, OCBBF2003543, OCBBF2003593, OCBBF2003744, OCBBF2003819, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2005066, OCBBF2005077, OCBBF2005161, OCBBF2005343, OCBBF2005349, OCBBF2005373, OCBBF2005420, OCBBF2005433, OCBBF2005476, OCBBF2005546, OCBBF2005843, OCBBF2005901, OCBBF2006005, OCBBF2006030, OCBBF2006058, OCBBF2006148, OCBBF2006151, OCBBF2006154, OCBBF2006214, OCBBF2006241, OCBBF2006388, OCBBF2006438, OCBBF2006567, OCBBF2006624, OCBBF2006639, OCBBF2006660, OCBBF2006972, OCBBF2006987, OCBBF2007039, OCBBF2007051, OCBBF2007068, OCBBF2007114, OCBBF2007121, OCBBF2007184, OCBBF2007194, OCBBF2007224, OCBBF2007238, OCBBF2007354, OCBBF2007414, OCBBF2007428, OCBBF2007478, OCBBF2007485, OCBBF2007520, OCBBF2007555, OCBBF2007610, OCBBF2007622, OCBBF2007756, OCBBF2007931, OCBBF2007946, OCBBF2008116, OCBBF2008138, OCBBF2008144, OCBBF2008283, OCBBF2008330, OCBBF2008366, OCBBF2008483, OCBBF2008511, OCBBF2008520, OCBBF2008569, OCBBF2008586, OCBBF2008640, OCBBF2008691, OCBBF2008724, OCBBF2008760, OCBBF2008768, OCBBF2008775, OCBBF2008790, OCBBF2008814, OCBBF2008822, OCBBF2009095, OCBBF2009115, OCBBF2009242, OCBBF2009352, OCBBF2009536, OCBBF2009571, OCBBF2009583, OCBBF2009603, OCBBF2009788, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010858, OCBBF2010863, OCBBF2010931, OCBBF2010945, OCBBF2010978, OCBBF2010989, OCBBF2011137, OCBBF2011160, OCBBF2011177, OCBBF2011228, OCBBF2011283, OCBBF2011311, OCBBF2011669, OCBBF2011685, OCBBF2011706, OCBBF2011722, OCBBF2011767, OCBBF2011855, OCBBF2011872, OCBBF2011897, OCBBF2011914, OCBBF2011960, OCBBF2011981, OCBBF2012001, OCBBF2012028, OCBBF2012039, OCBBF2012139, OCBBF2012262, OCBBF2012320, OCBBF2012436, OCBBF2012525, OCBBF2012704, OCBBF2012714, OCBBF2012755, OCBBF2012812, OCBBF2012881, OCBBF2012936, OCBBF2012990, OCBBF2013049, OCBBF2013091, OCBBF2013123, OCBBF2013127, OCBBF2013208, OCBBF2013285, OCBBF2013319, OCBBF2013324, OCBBF2013366, OCBBF2013507, OCBBF2013721, OCBBF2013883, OCBBF2014052, OCBBF2014089, OCBBF2014576, OCBBF2014707, OCBBF2014745, OCBBF2014828, OCBBF2015031, OCBBF2015081, OCBBF2015115, OCBBF2015232, OCBBF2015233, OCBBF2015285, OCBBF2015334, OCBBF2015335, OCBBF2015506, OCBBF2015645, OCBBF2015659, OCBBF2015797, OCBBF2015931, OCBBF2015980, OCBBF2016038, OCBBF2016467, OCBBF2016590, OCBBF2016591, OCBBF2016612, OCBBF2016689, OCBBF2016690, OCBBF2016729, OCBBF2016841, OCBBF2017055, OCBBF2017069, OCBBF2017306, OCBBF2017325, OCBBF2017489, OCBBF2017516, OCBBF2017665, OCBBF2017815, OCBBF2017882, OCBBF2017920, OCBBF2018014, OCBBF2018084, OCBBF2018167, OCBBF2018206, OCBBF2018229, OCBBF2018362, OCBBF2018380, OCBBF2018563, OCBBF2018581, OCBBF2018618, OCBBF2018663, OCBBF2018687, OCBBF2018827, OCBBF2018828, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021323, OCBBF2021518, OCBBF2021788, OCBBF2021833, OCBBF2022351, OCBBF2022573, OCBBF2022574, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000180, PEBLM2000030, PEBLM2000112, PEBLM2000126, PEBLM2000147, PEBLM2000170, PEBLM2000180, PEBLM2000213, PEBLM2000222, PEBLM2000248, PEBLM2000267, PEBLM2000270, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003935, PEBLM2004015, PEBLM2004216, PEBLM2004290, PEBLM2004452, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006709, PEBLM2006912, PEBLM2007112, PEBLM2007140, PEBLM2007188, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000080, PLACE5000105, PLACE5000115, PLACE5000116, PLACE5000129, PLACE5000139, PLACE5000153, PLACE5000171, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000348, PLACE6000371, PLACE6000414, PLACE6000424, PLACE6000426, PLACE6000429, PLACE6000450, PLACE6000523, PLACE6000550, PLACE6000552, PLACE6000555, PLACE6000619, PLACE6000630, PLACE6000953, PLACE6001118, PLACE6001153, PLACE6001185, PLACE6001294, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003383, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004101, PLACE6004219, PLACE6004336, PLACE6004368, PLACE6004380, PLACE6004396, PLACE6004397, PLACE6004454, PLACE6004464, PLACE6004491, PLACE6004578, PLACE6004663, PLACE6004687, PLACE6004738, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006549, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010925, PLACE6010936, PLACE6010991, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016254, PLACE6016383, PLACE6016984, PLACE6017002, PLACE6017431, PLACE6017564, PLACE6017578, PLACE6017626, PLACE6017701, PLACE6017714, PLACE6017788, PLACE6017791, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000123, PROST1000136, PROST1000144, PROST1000145, PROST1000152, PROST1000167, PROST1000184, PROST1000217, PROST1000220, PROST1000226, PROST1000246, PROST1000262, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000564, PROST1000575, PROST2000036, PROST2000053, PROST2000067, PROST2000105, PROST2000143, PROST2000162, PROST2000176, PROST2000203, PROST2000206, PROST2000212, PROST2000267, PROST2000273, PROST2000274, PROST2000277, PROST2000284, PROST2000325, PROST2000432, PROST2000452, PROST2000463, PROST2000470, PROST2000505, PROST2000567, PROST2000572, PROST2000717, PROST2000760, PROST2000761, PROST2000835, PROST2000961, PROST2001002, PROST2001116, PROST2001138, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001899, PROST2001997, PROST2001998, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303, PROST2003324, PROST2003340, PROST2003396, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004332, PROST2004481, PROST2004526, PROST2004727, PROST2004739, PROST2004742, PROST2004744, PROST2004817, PROST2005067, PROST2005076, PROST2005093, PROST2005121, PROST2005131, PROST2005228, PROST2005272, PROST2005285, PROST2005426, PROST2005466, PROST2005604, PROST2005630, PROST2005683, PROST2005788, PROST2005880, PROST2005886, PROST2005904, PROST2005919, PROST2005943, PROST2006008, PROST2006020, PROST2006060, PROST2006196, PROST2006201, PROST2006260, PROST2006282, PROST2006343, PROST2006450, PROST2006491, PROST2006510, PROST2006579, PROST2006688, PROST2006737, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008447, PROST2008468, PROST2008472, PROST2008489, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010400, PROST2010545, PROST2010579, PROST2010606, PROST2010885, PROST2011012, PROST2011105, PROST2011297, PROST2011410, PROST2011439, PROST2011631, PROST2011660, PROST2012005, PROST2012016, PROST2012088, PROST2012140, PROST2012157, PROST2012190, PROST2012249, PROST2012353, PROST2012400, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015287, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017203, PROST2017367, PROST2017413, PROST2017441, PROST2017578, PROST2017612, PROST2017617, PROST2017692, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2019164, PROST2019296, PROST2019398, PROST2019487, PROST2019781, PUAEN1000039, PUAEN1000065, PUAEN1000081, PUAEN1000085, PUAEN1000087, PUAEN1000121, PUAEN1000147, PUAEN1000161, PUAEN1000175, PUAEN1000275, PUAEN1000276, PUAEN1000288, PUAEN1000322, PUAEN2000080, PUAEN2000152, PUAEN2000175, PUAEN2000247, PUAEN2000394, PUAEN2000497, PUAEN2000594, PUAEN2000684, PUAEN2001260, PUAEN2001526, PUAEN2001586, PUAEN2001882, PUAEN2002473, PUAEN2002552, PUAEN2002568, PUAEN2002616, PUAEN2002758, PUAEN2003018, PUAEN2003079, PUAEN2003116, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009795, PUAEN2009852, PUAEN2010346, PUAEN2010824, RECTM1000141, RECTM2000220, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000030, SKMUS1000064, SKMUS1000067, SKMUS1000102, SKMUS1000104, SKMUS1000110, SKMUS1000118, SKMUS1000124, SKMUS1000129, SKMUS1000138, SKMUS1000176, SKMUS1000177, SKMUS2000061, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000365, SKMUS2000390, SKMUS2000416, SKMUS2000458, SKMUS2000467, SKMUS2000468, SKMUS2000515, SKMUS2000679, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000933, SKMUS2000945, SKMUS2000954, SKMUS2000961, SKMUS2000973, SKMUS2000980, SKMUS2001008, SKMUS2001014, SKMUS2001129, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001412, SKMUS2001492, SKMUS2001501, SKMUS2001528, SKMUS2001543, SKMUS2001580, SKMUS2001585, SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000137, SKNMC1000159, SKNMC1000251, SKNMC1000264, SKNMC2000224, SKNMC2000256, SKNMC2000322, SKNMC2000374, SKNMC2000583, SKNMC2000593, SKNMC2000612, SKNMC2000622, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000086, SKNSH1000101, SKNSH1000174, SKNSH1000301, SKNSH1000308, SKNSH1000416, SKNSH2000051, SKNSH2000101, SKNSH2000151, SKNSH2000250, SKNSH2000319, SKNSH2000482, SKNSH2000516, SKNSH2000550, SKNSH2000971, SKNSH2001222, SKNSH2001875, SKNSH2001931, SKNSH2002054, SKNSH2002325, SKNSH2002768, SKNSH2002866, SKNSH2003064, SKNSH2003174, SKNSH2003422, SKNSH2003466, SKNSH2003483, SKNSH2003633, SKNSH2004039, SKNSH2005061, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008969, SKNSH2009197, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000118, SMINT1000131, SMINT1000137, SMINT1000192, SMINT2000018, SMINT2000032, SMINT2000040, SMINT2000060, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000400, SMINT2000454, SMINT2000518, SMINT2000529, SMINT2000530, SMINT2000537, SMINT2000538, SMINT2000541, SMINT2000545, SMINT2000558, SMINT2000562, SMINT2000568, SMINT2000609, SMINT2000659, SMINT2000747, SMINT2000811, SMINT2000824, SMINT2000933, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2001950, SMINT2002126, SMINT2002202, SMINT2002210, SMINT2002224, SMINT2002281, SMINT2002311, SMINT2002314, SMINT2002328, SMINT2002414, SMINT2002457, SMINT2002743, SMINT2002778, SMINT2002880, SMINT2002882, SMINT2002899, SMINT2002974, SMINT2002976, SMINT2003003, SMINT2003128, SMINT2003150, SMINT2003317, SMINT2003386, SMINT2003423, SMINT2003551, SMINT2003569, SMINT2003641, SMINT2003644, SMINT2003866, SMINT2003905, SMINT2004023, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004583, SMINT2004589, SMINT2004781, SMINT2004891, SMINT2004909, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006801, SMINT2007062, SMINT2007101, SMINT2007140, SMINT2007187, SMINT2007219, SMINT2007294, SMINT2007365, SMINT2007391, SMINT2007433, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010144, SMINT2010200, SMINT2010278, SMINT2010324, SMINT2010326, SMINT2010369, SMINT2010500, SMINT2010533, SMINT2010629, SMINT2010639, SMINT2010672, SMINT2010753, SMINT2010853, SMINT2010897, SMINT2010959, SMINT2010997, SMINT2011033, SMINT2011066, SMINT2011273, SMINT2011311, SMINT2011406, SMINT2011509, SMINT2011567, SMINT2011656, SMINT2011888, SMINT2011958, SMINT2012040, SMINT2012122, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2019017, SMINT2019105, SMINT2019142, SMINT2019153, SMINT2019200, SPLEN1000013, SPLEN1000041, SPLEN1000056, SPLEN1000087, SPLEN1000091, SPLEN1000134, SPLEN1000139, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000258, SPLEN2000270, SPLEN2000307, SPLEN2000310, SPLEN2000341, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000839, SPLEN2000874, SPLEN2001135, SPLEN2001141, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001626, SPLEN2001650, SPLEN2001710, SPLEN2001761, SPLEN2001781, SPLEN2001881, SPLEN2001912, SPLEN2001945, SPLEN2002053, SPLEN2002133, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002463, SPLEN2002477, SPLEN2002493, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004124, SPLEN2004148, SPLEN2004181, SPLEN2004220, SPLEN2004343, SPLEN2004346, SPLEN2004368, SPLEN2004611, SPLEN2004662, SPLEN2004844, SPLEN2004880, SPLEN2004984, SPLEN2005227, SPLEN2005272, SPLEN2005416, SPLEN2005429, SPLEN2005450, SPLEN2005474, SPLEN2005560, SPLEN2005727, SPLEN2005783, SPLEN2005790, SPLEN2005806, SPLEN2005818, SPLEN2005838, SPLEN2005927, SPLEN2005939, SPLEN2006268, SPLEN2006283, SPLEN2006325, SPLEN2006389, SPLEN2006425, SPLEN2006671, SPLEN2006701, SPLEN2006785, SPLEN2006875, SPLEN2007182, SPLEN2007314, SPLEN2007326, SPLEN2007350, SPLEN2007388, SPLEN2007619, SPLEN2007653, SPLEN2007689, SPLEN2007695, SPLEN2007739, SPLEN2007750, SPLEN2007794, SPLEN2007879, SPLEN2007926, SPLEN2008007, SPLEN2008135, SPLEN2008164, SPLEN2008317, SPLEN2008496, SPLEN2008591, SPLEN2008786, SPLEN2009088, SPLEN2009163, SPLEN2009315, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013857, SPLEN2013860, SPLEN2013910, SPLEN2013923, SPLEN2014041, SPLEN2014063, SPLEN2014073, SPLEN2014119, SPLEN2014136, SPLEN2014199, SPLEN2014210, SPLEN2014293, SPLEN2014318, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016241, SPLEN2016250, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019257, SPLEN2019311, SPLEN2019323, SPLEN2019349, SPLEN2019375, SPLEN2019379, SPLEN2019446, SPLEN2019471, SPLEN2019480, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028417, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031547, SPLEN2031674, SPLEN2031724, SPLEN2031780, SPLEN2032036, SPLEN2032112, SPLEN2032154, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000257, STOMA2000279, STOMA2000289, STOMA2000383, STOMA2000386, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002052, STOMA2002141, STOMA2002166, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001212, SYNOV2001239, SYNOV2001251, SYNOV2001300, SYNOV2001356, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009139, SYNOV4009298, SYNOV4009575, T1ESE2000116, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000117, TESTI2000120, TESTI2000147, TESTI2000154, TESTI2000172, TESTI2000177, TESTI2000179, TESTI2000207, TESTI2000208, TESTI2000221, TESTI2000253, TESTI2000272, TESTI2000278, TESTI2000349, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001120, TESTI2001134, TESTI2001153, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005017, TESTI2005025, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005759, TESTI2005767, TESTI2005775, TESTI2005788, TESTI2005827, TESTI2005835, TESTI2005860, TESTI2005892, TESTI2005911, TESTI2005933, TESTI2005937, TESTI2005948, TESTI2005979, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007422, TESTI2007452, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009412, TESTI2009423, TESTI2009447, TESTI2009462, TESTI2009474, TESTI2009477, TESTI2009497, TESTI2009502, TESTI2009511, TESTI2009544, TESTI2009545, TESTI2009551, TESTI2009577, TESTI2009585, TESTI2009588, TESTI2009683, TESTI2009727, TESTI2009739, TESTI2009812, TESTI2009835, TESTI2009853, TESTI2009935, TESTI2009977, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012778, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014577, TESTI2014578, TESTI2014716, TESTI2014800, TESTI2014838, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2017954, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021138, TESTI2021297, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003733, TESTI4003796, TESTI4003944, TESTI4004003, TESTI4004031, TESTI4004200, TESTI4004210, TESTI4004539, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000025, THYMU1000029, THYMU1000032, THYMU1000033, THYMU1000060, THYMU1000074, THYMU1000083, THYMU1000098, THYMU1000105, THYMU1000109, THYMU1000136, THYMU1000142, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000382, THYMU1000393, THYMU1000394, THYMU1000399, THYMU1000426, THYMU1000428, THYMU1000440, THYMU1000459, THYMU1000473, THYMU1000491, THYMU1000496, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000702, THYMU2000767, THYMU2000775, THYMU2000800, THYMU2000879, THYMU2000932, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001989, THYMU2002023, THYMU2002037, THYMU2002080, THYMU2002109, THYMU2002147, THYMU2002154, THYMU2002157, THYMU2002177, THYMU2002203, THYMU2002229, THYMU2002242, THYMU2002338, THYMU2002356, THYMU2002376, THYMU2002384, THYMU2002490, THYMU2002548, THYMU2002695, THYMU2002745, THYMU2002789, THYMU2002792, THYMU2002815, THYMU2002852, THYMU2002910, THYMU2002950, THYMU2003046, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004508, THYMU2004512, THYMU2004635, THYMU2004639, THYMU2004668, THYMU2004677, THYMU2004688, THYMU2004693, THYMU2004835, THYMU2004843, THYMU2004906, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007036, THYMU2007060, THYMU2007065, THYMU2007095, THYMU2007146, THYMU2007179, THYMU2007258, THYMU2007307, THYMU2007308, THYMU2007339, THYMU2007350, THYMU2007384, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666, THYMU2011785, THYMU2011806, THYMU2011852, THYMU2011875, THYMU2011881, THYMU2011937, THYMU2011939, THYMU2012002, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017675, THYMU2017707, THYMU2017831, THYMU2017844, THYMU2017878, THYMU2017948, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032867, THYMU3033402, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2001327, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000018, TRACH1000038, TRACH1000057, TRACH1000074, TRACH1000100, TRACH1000106, TRACH1000125, TRACH1000140, TRACH1000144, TRACH1000193, TRACH1000205, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000393, TRACH2000411, TRACH2000420, TRACH2000472, TRACH2000496, TRACH2000497, TRACH2000502, TRACH2000522, TRACH2000540, TRACH2000665, TRACH2000675, TRACH2000677, TRACH2000702, TRACH2000703, TRACH2000767, TRACH2000775, TRACH2000807, TRACH2000862, TRACH2000873, TRACH2000894, TRACH2000898, TRACH2000923, TRACH2000944, TRACH2000981, TRACH2000986, TRACH2001037, TRACH2001060, TRACH2001085, TRACH2001101, TRACH2001121, TRACH2001147, TRACH2001154, TRACH2001188, TRACH2001192, TRACH2001249, TRACH2001275, TRACH2001335, TRACH2001339, TRACH2001341, TRACH2001347, TRACH2001395, TRACH2001400, TRACH2001403, TRACH2001416, TRACH2001474, TRACH2001525, TRACH2001532, TRACH2001549, TRACH2001592, TRACH2001596, TRACH2001607, TRACH2001621, TRACH2001646, TRACH2001651, TRACH2001684, TRACH2001898, TRACH2001933, TRACH2001968, TRACH2001996, TRACH2002043, TRACH2002054, TRACH2002092, TRACH2002138, TRACH2002333, TRACH2002664, TRACH2002784, TRACH2002840, TRACH2002847, TRACH2002853, TRACH2002862, TRACH2002954, TRACH2002960, TRACH2002988, TRACH2003070, TRACH2003323, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007399, TRACH2007483, TRACH2007542, TRACH2007577, TRACH2007674, TRACH2007676, TRACH2007688, TRACH2007733, TRACH2007754, TRACH2007894, TRACH2007969, TRACH2007988, TRACH2008112, TRACH2008113, TRACH2008127, TRACH2008130, TRACH2008278, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011290, TRACH2011293, TRACH2011302, TRACH2011500, TRACH2011574, TRACH2011956, TRACH2012000, TRACH2012138, TRACH2012242, TRACH2012298, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013265, TRACH2013369, TRACH2013450, TRACH2013495, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000186, UMVEN2000046, UMVEN2000069, UMVEN2000121, UMVEN2000133, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000218, UTERU2000238, UTERU2000243, UTERU2000260, UTERU2000263, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002332, UTERU2002410, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004037, UTERU2004039, UTERU2004061, UTERU2004073, UTERU2004163, UTERU2004197, UTERU2004299, UTERU2004520, UTERU2004564, UTERU2004688, UTERU2004698, UTERU2004861, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005593, UTERU2005601, UTERU2005664, UTERU2005822, UTERU2005903, UTERU2005905, UTERU2006103, UTERU2006115, UTERU2006182, UTERU2006400, UTERU2006412, UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007267, UTERU2007444, UTERU2007499, UTERU2007639, UTERU2007724, UTERU2007924, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009538, UTERU2009540, UTERU2009776, UTERU2009904, UTERU2009951, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011962, UTERU2011968, UTERU2012031, UTERU2012101, UTERU2012114, UTERU2012252, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013231, UTERU2013262, UTERU2013280, UTERU2013322, UTERU2013483, UTERU2013491, UTERU2013502, UTERU2013926, UTERU2013976, UTERU2014001, UTERU2014024, UTERU2014223, UTERU2014398, UTERU2014601, UTERU2014631, UTERU2014678, UTERU2014728, UTERU2014898, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016822, UTERU2016896, UTERU2016902, UTERU2016979, UTERU2016981, UTERU2017123, UTERU2017303, UTERU2017421, UTERU2017492, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the FEHRT, FEKID, FELIV, FELNG, HCASM, HCHON, and HEART libraries were analyzed for expression frequency. The analysis results are described below. Each is a relative value, where the expression level over all tissues is taken as 100. The larger this value, the greater the level of expression.

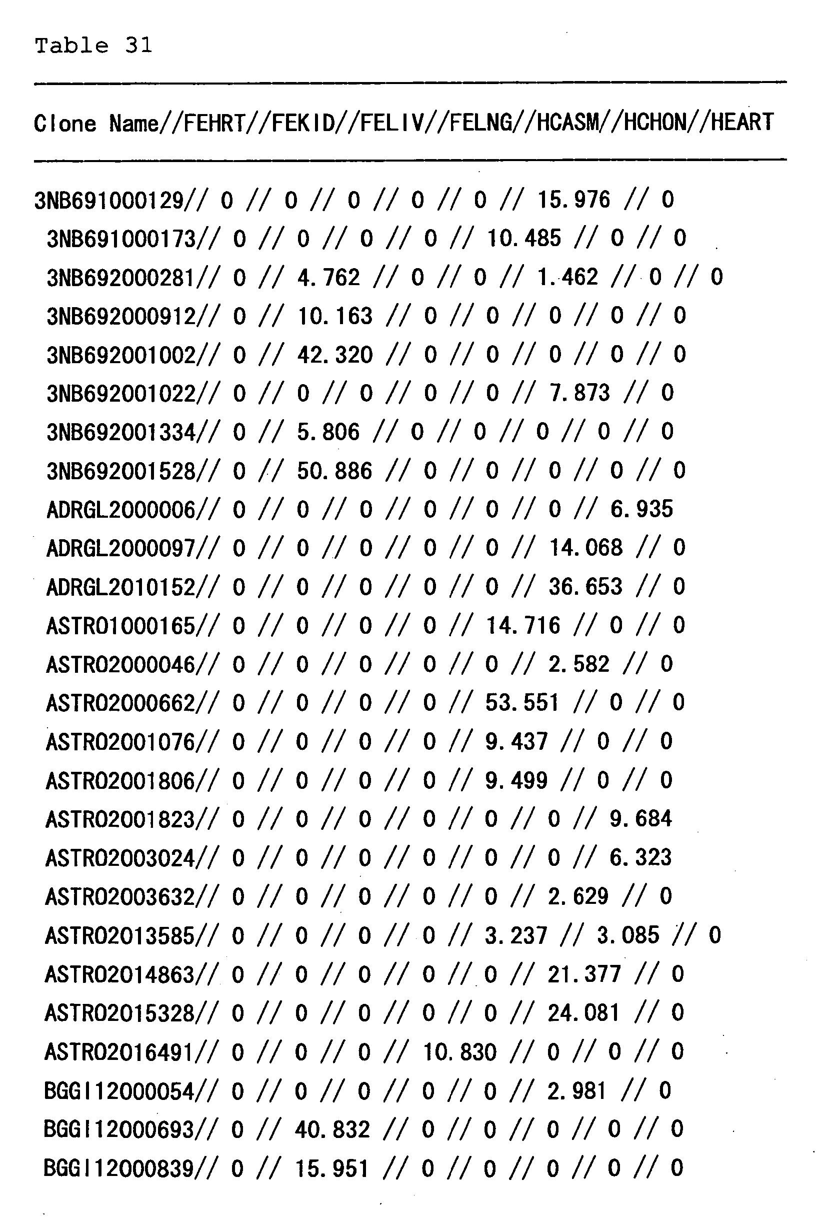

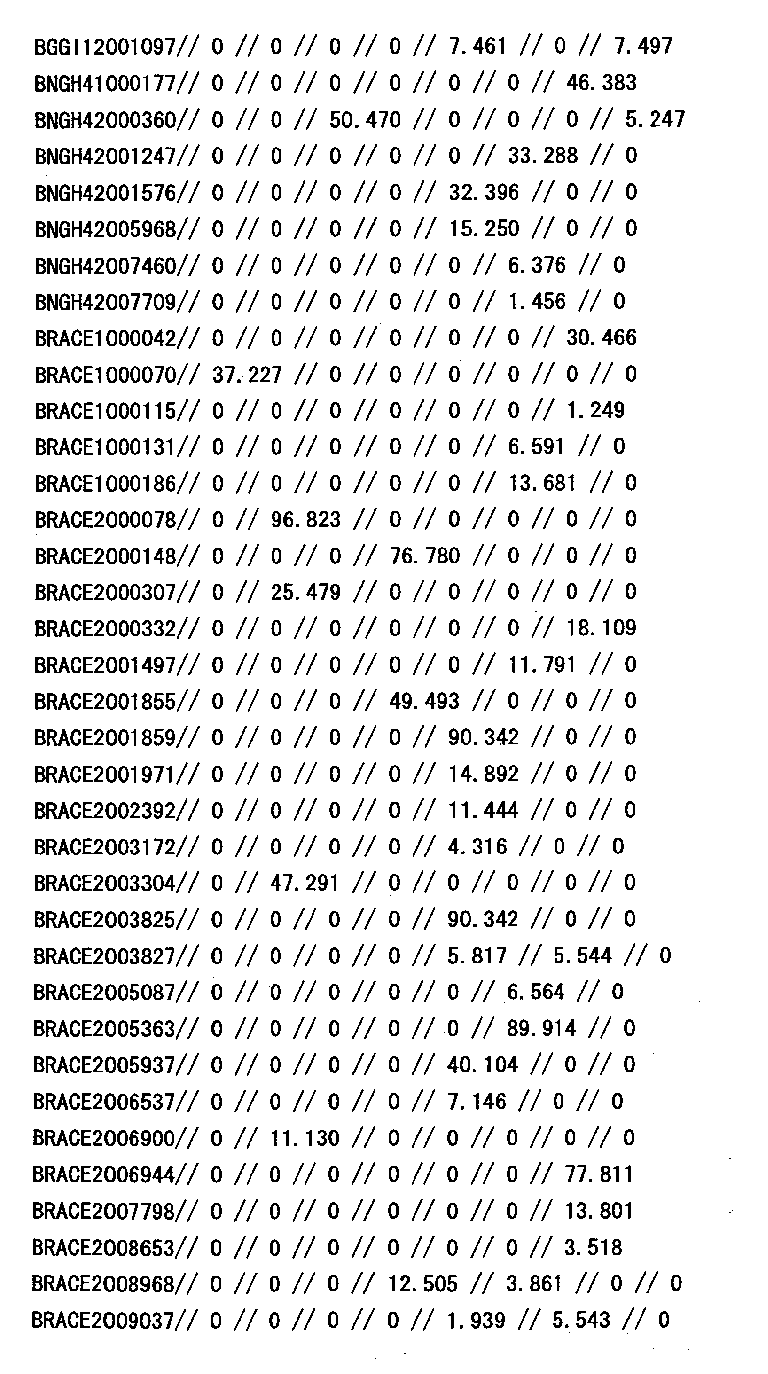

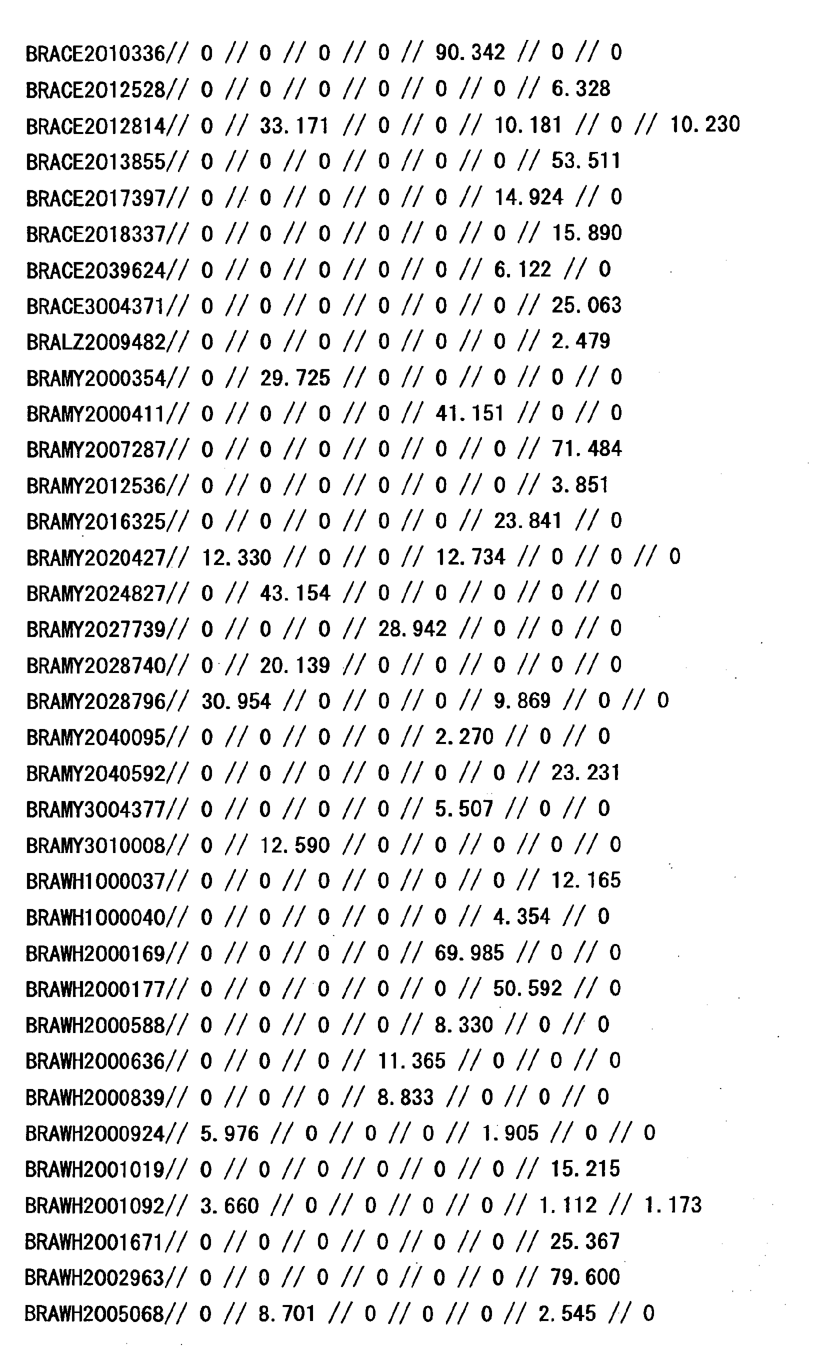

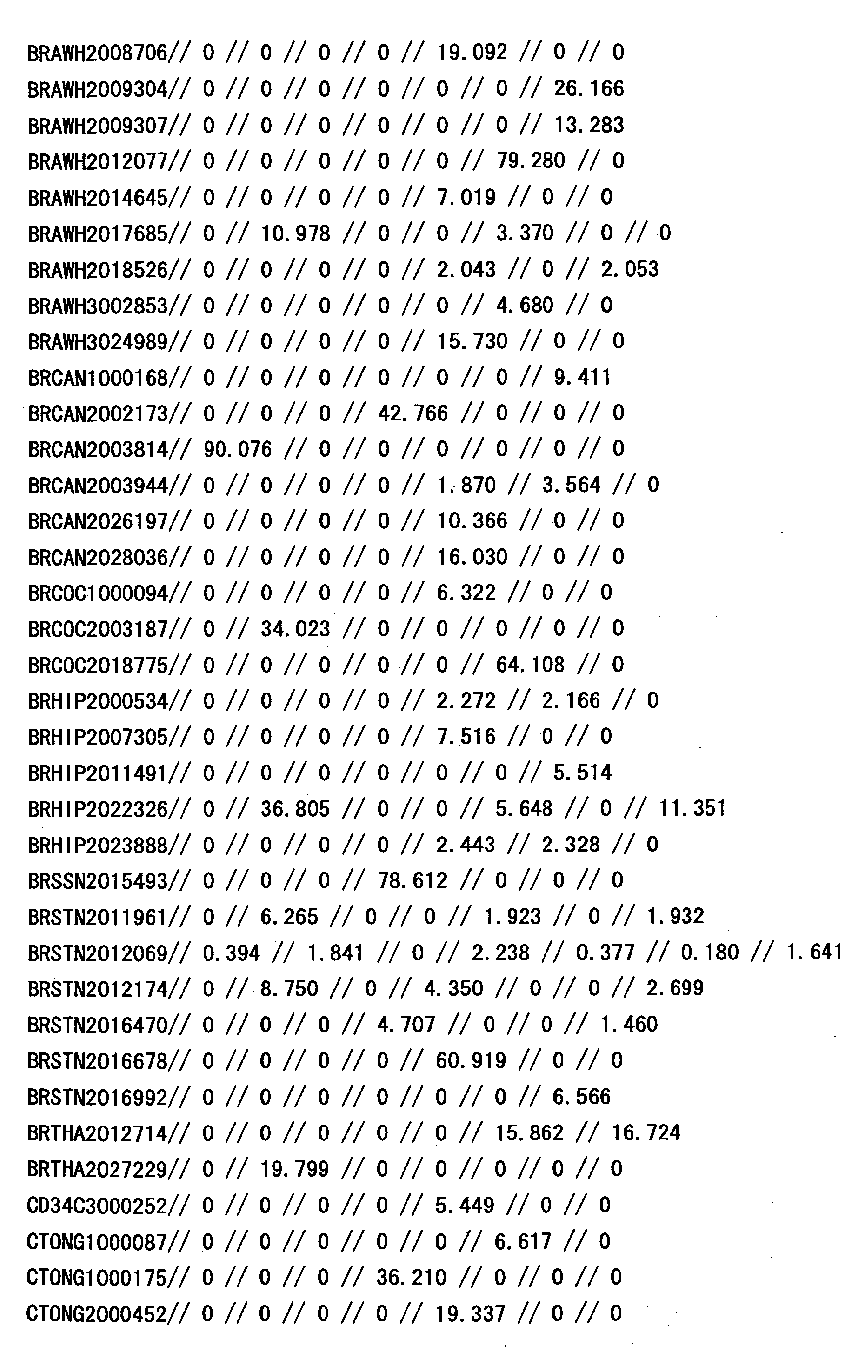

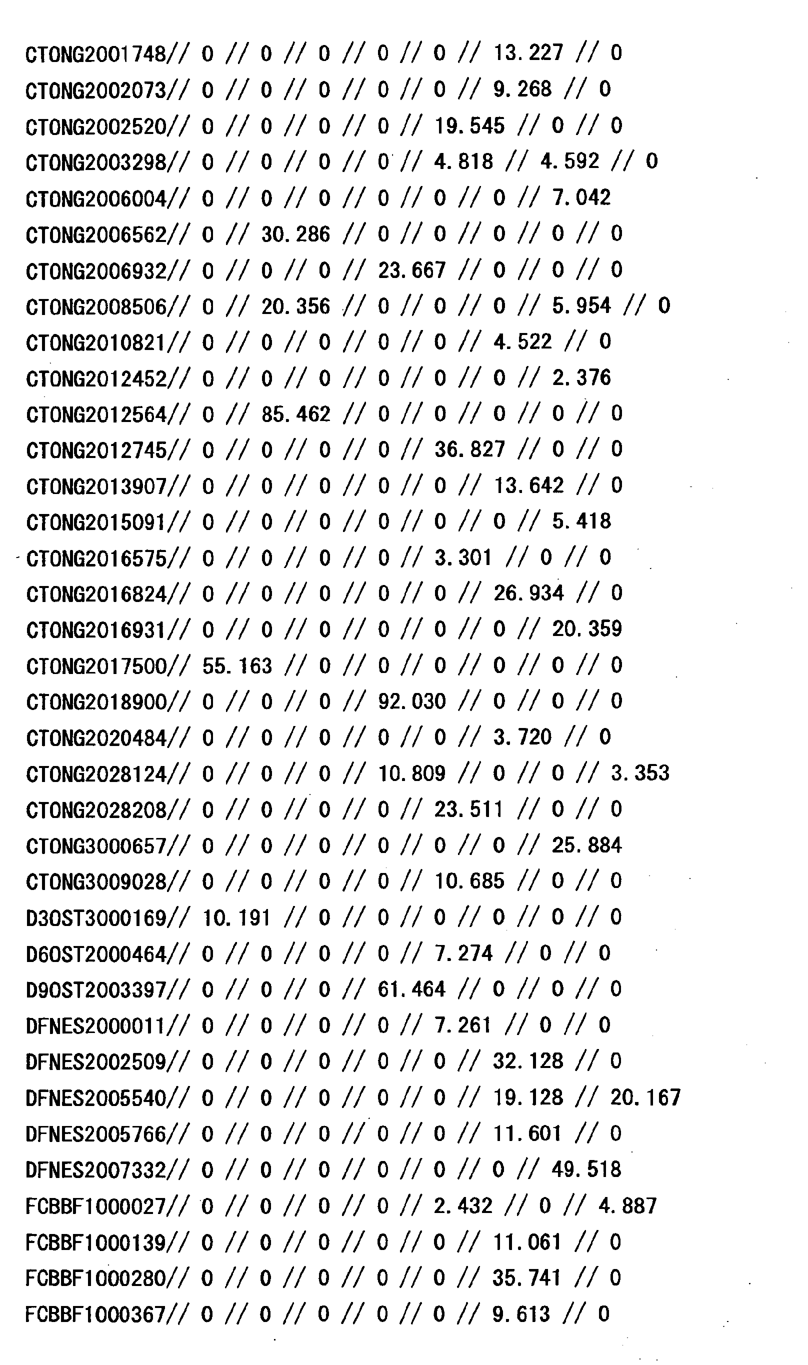

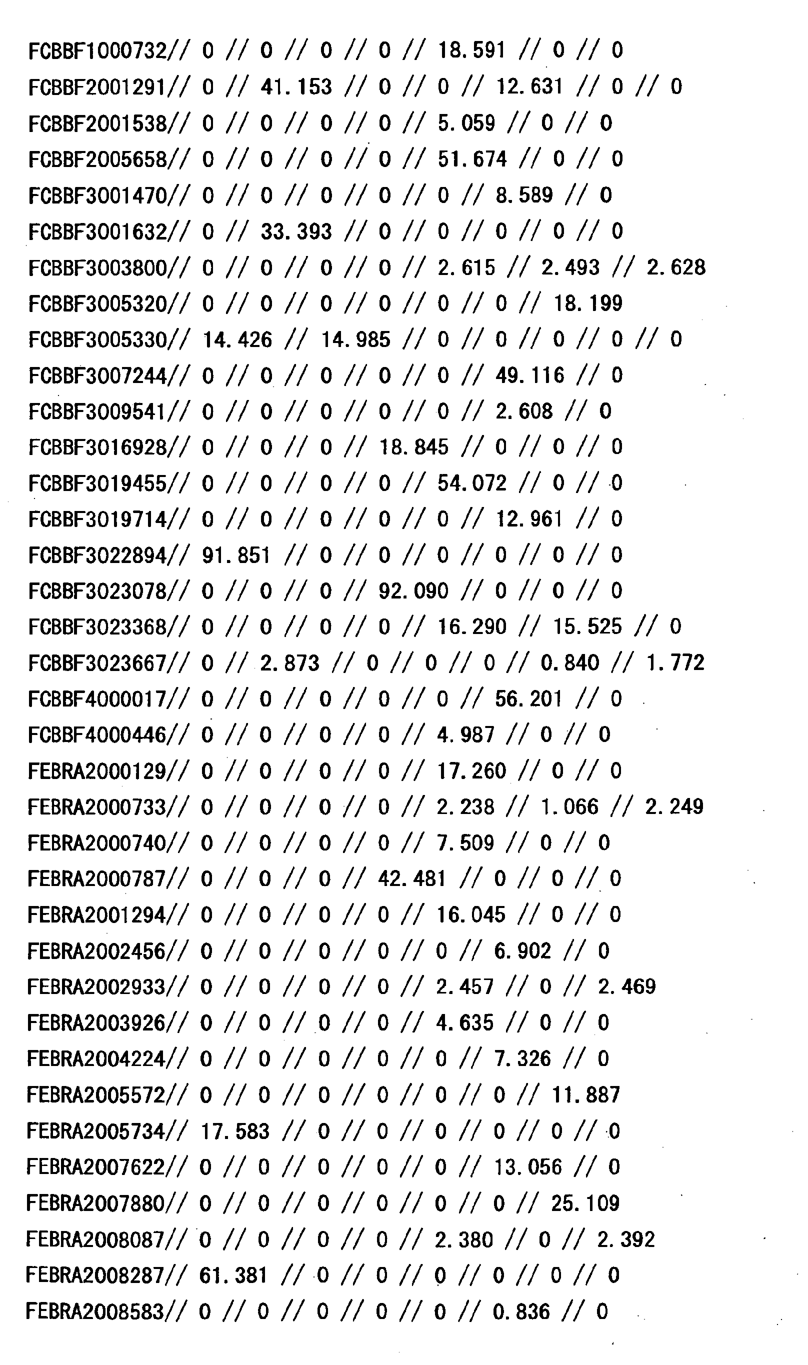

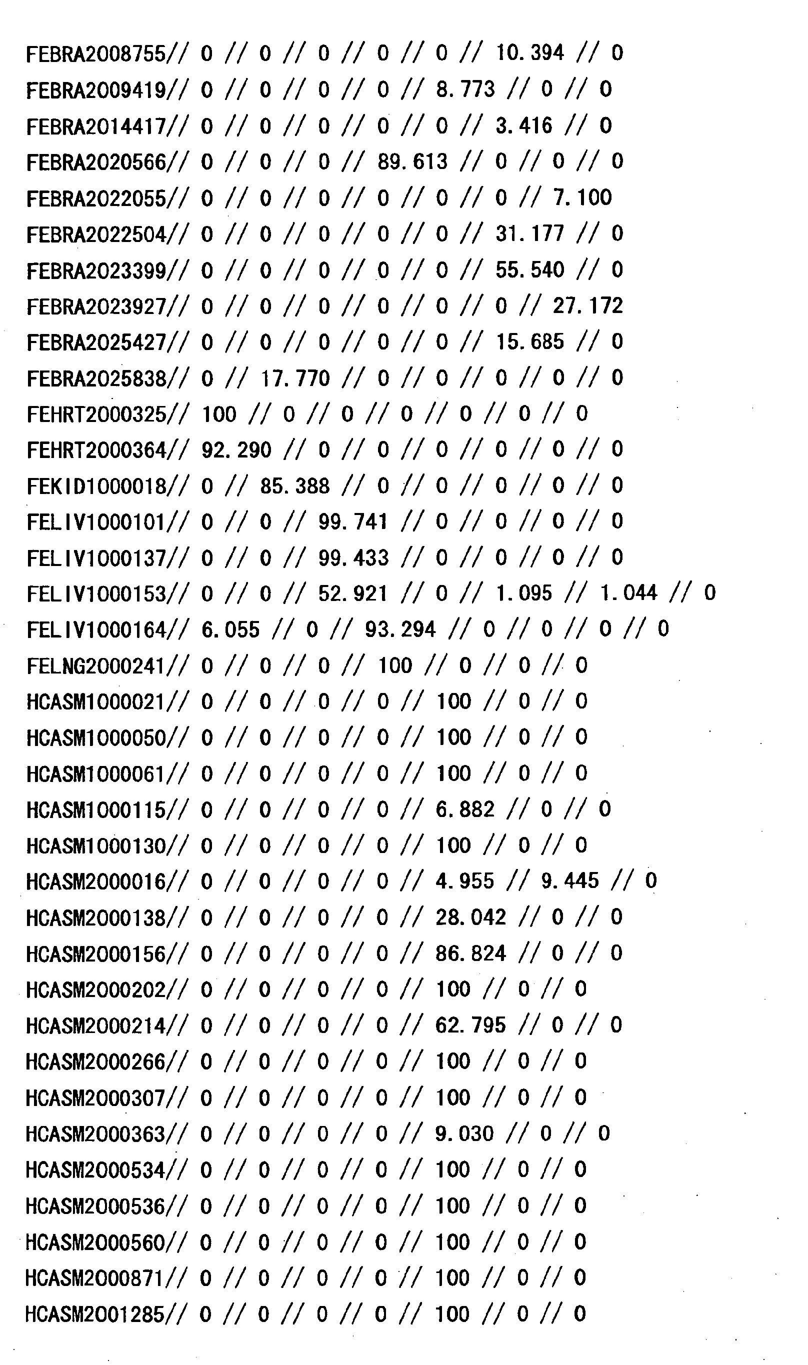

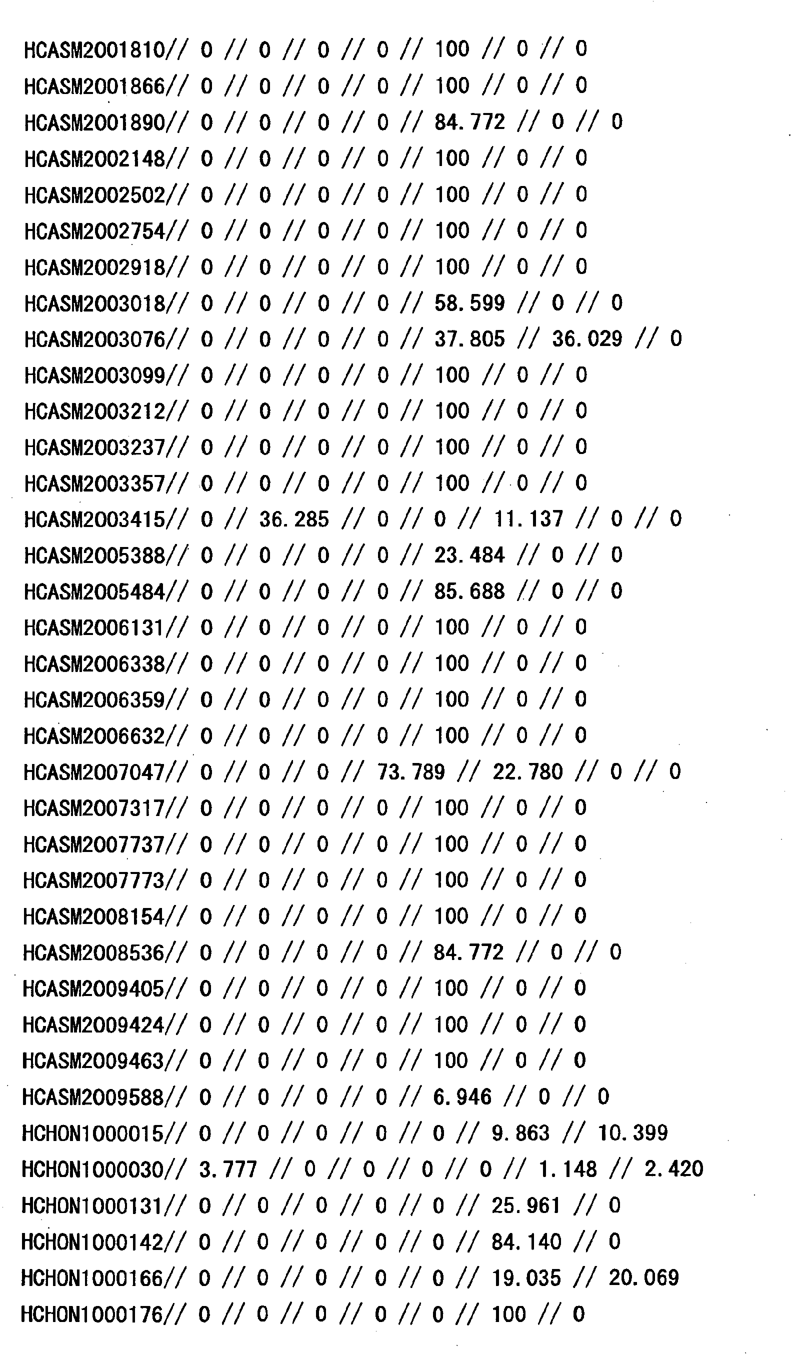

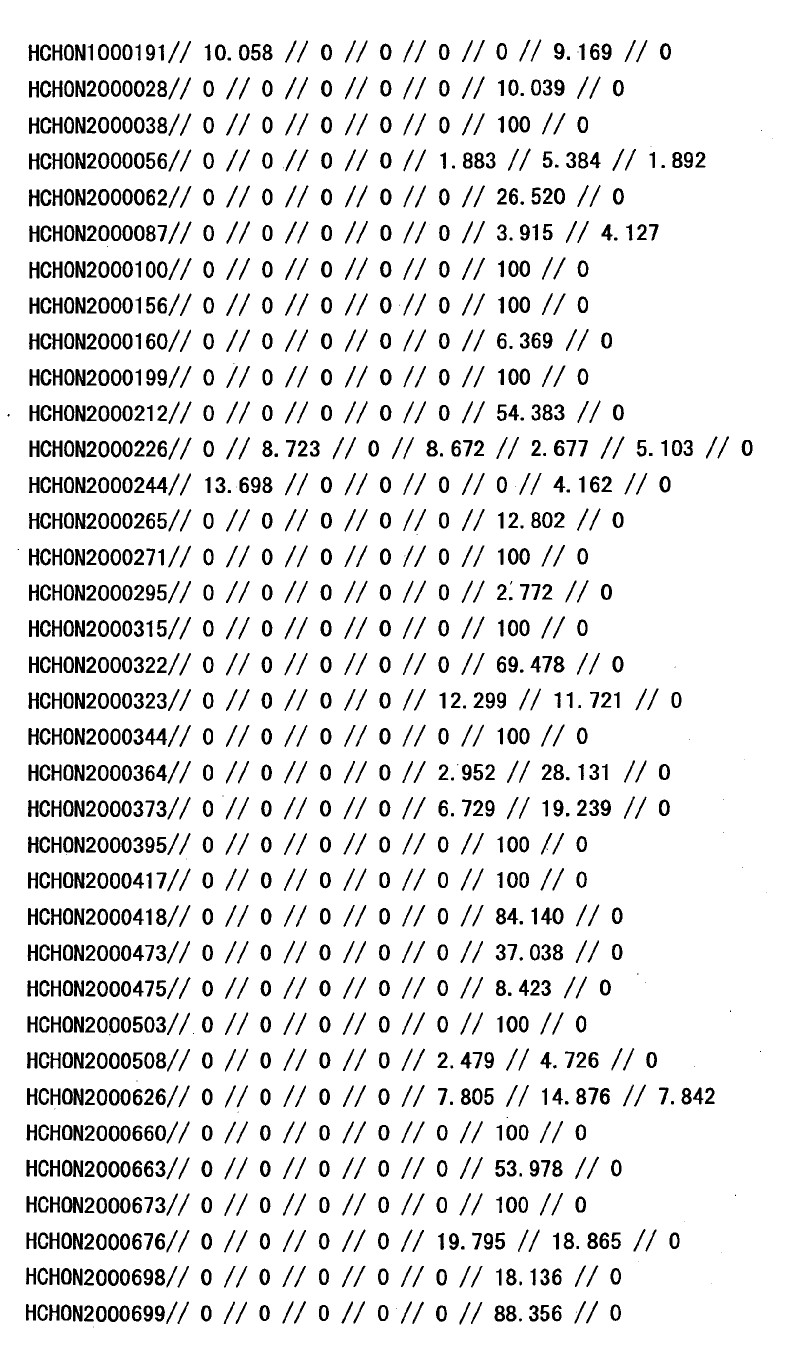

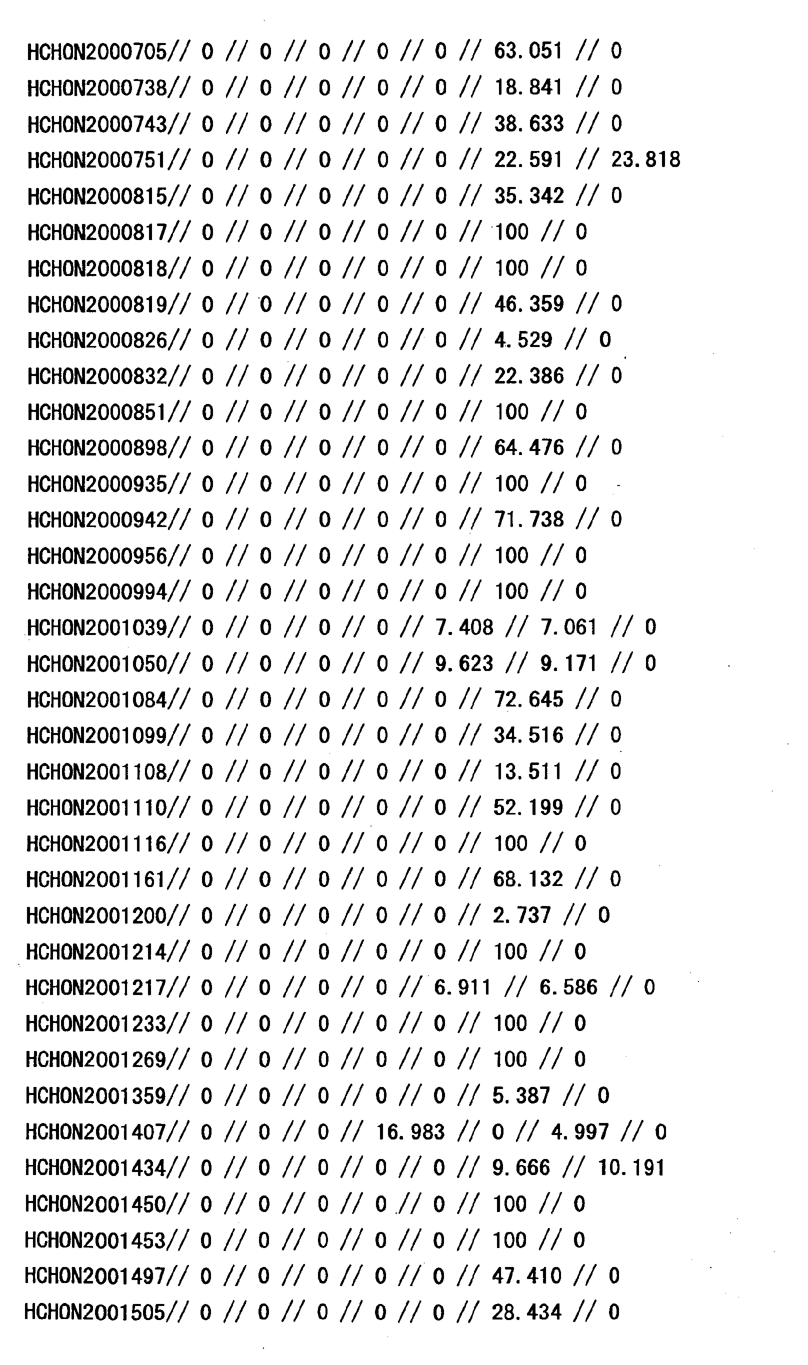

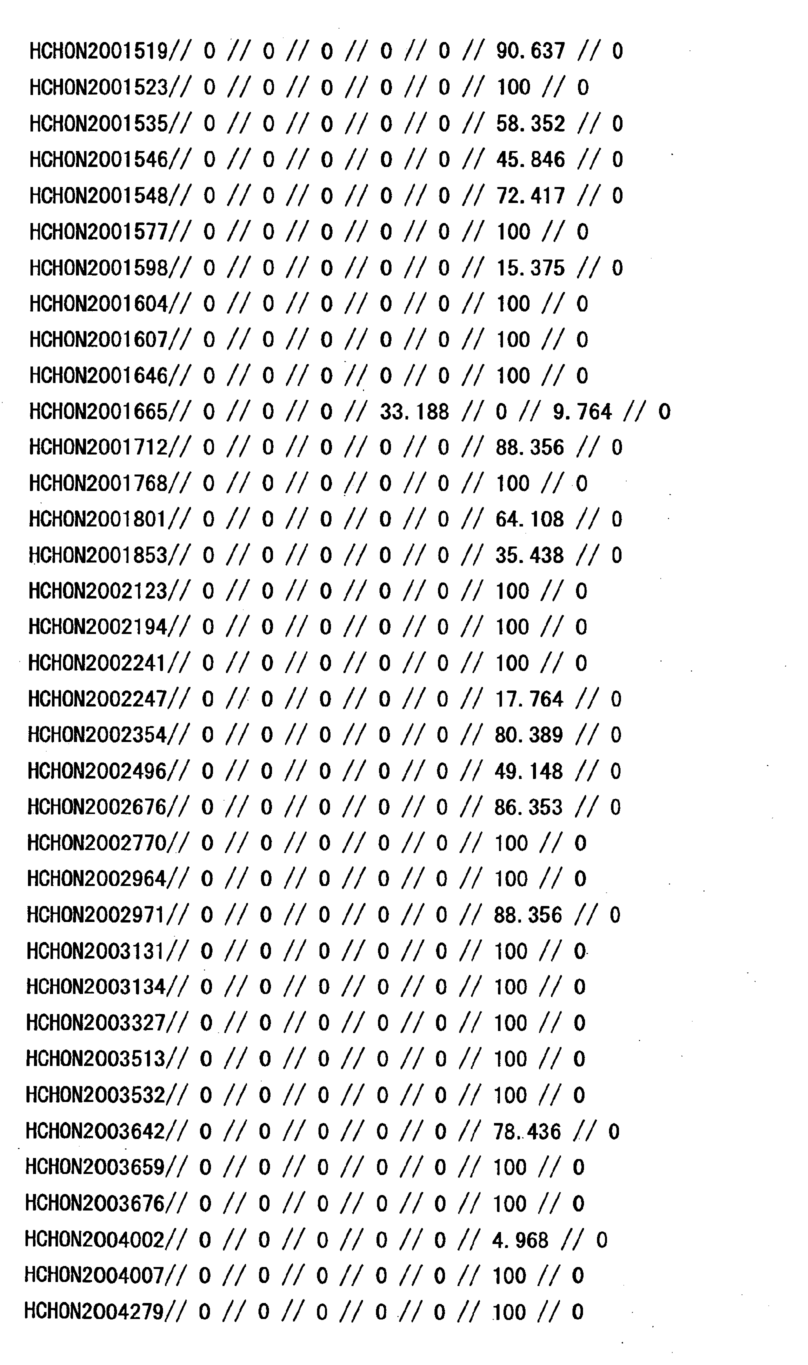

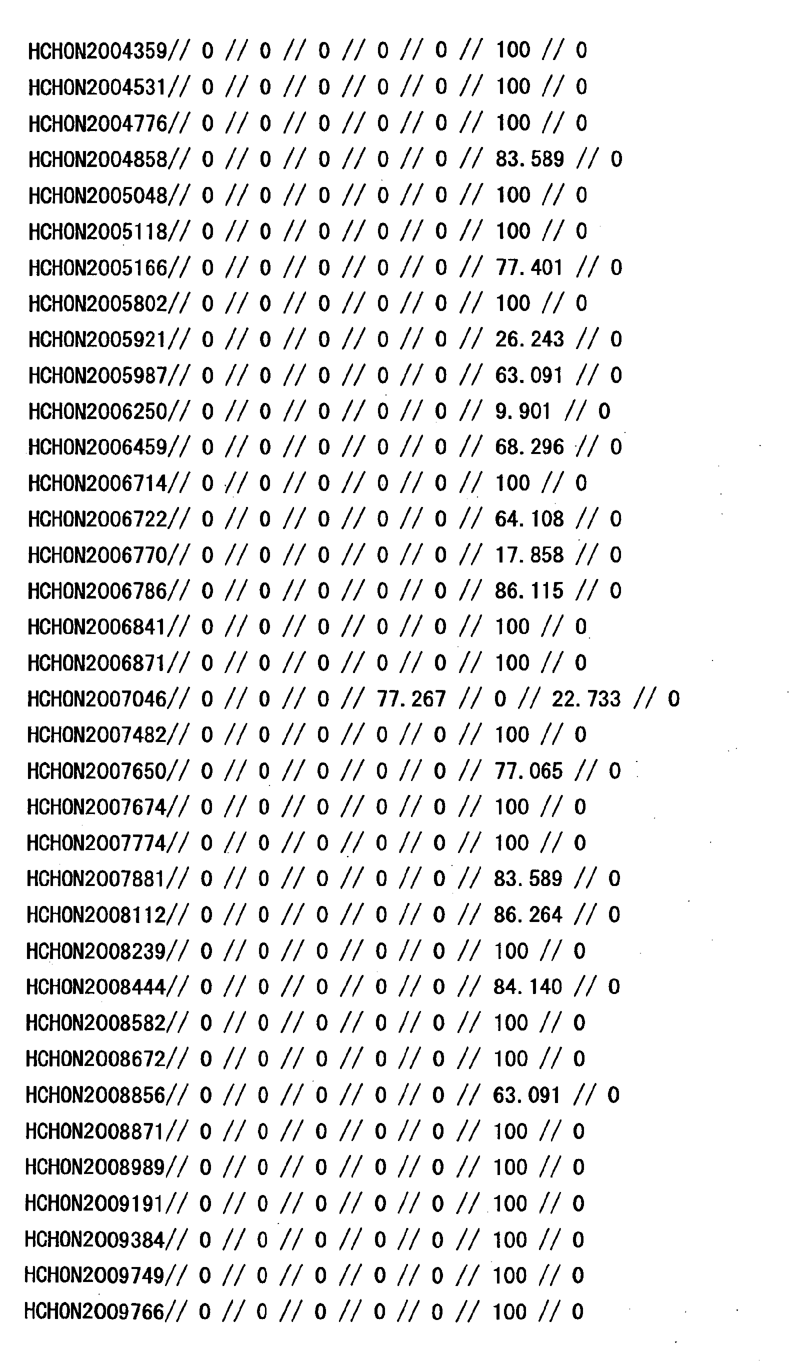

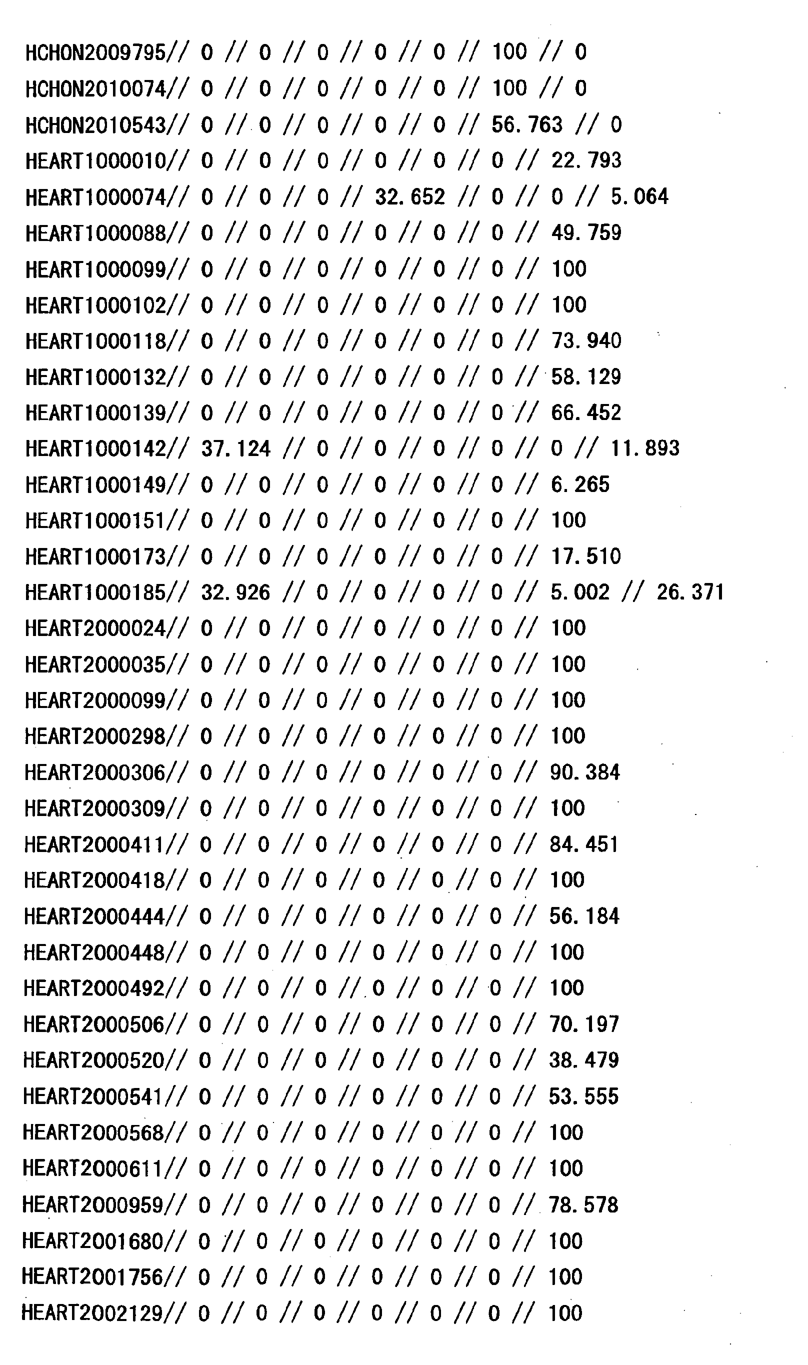

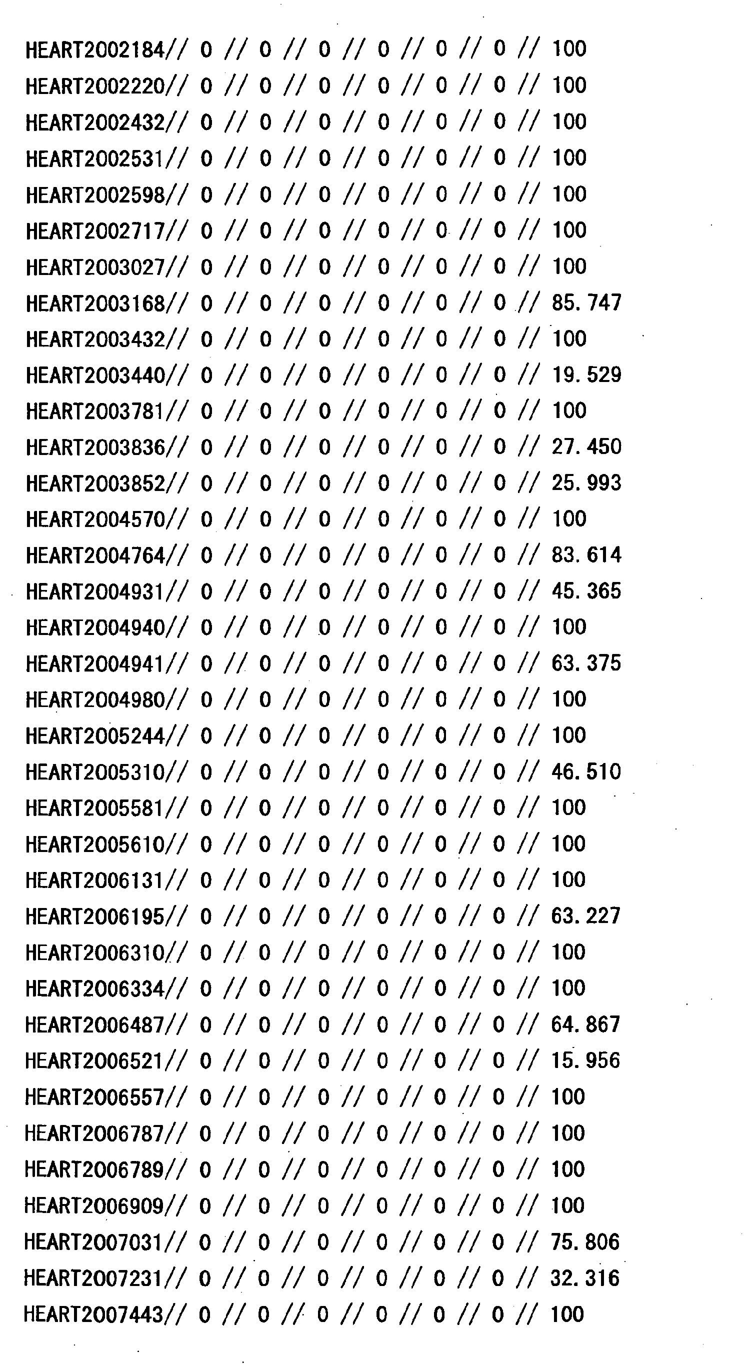

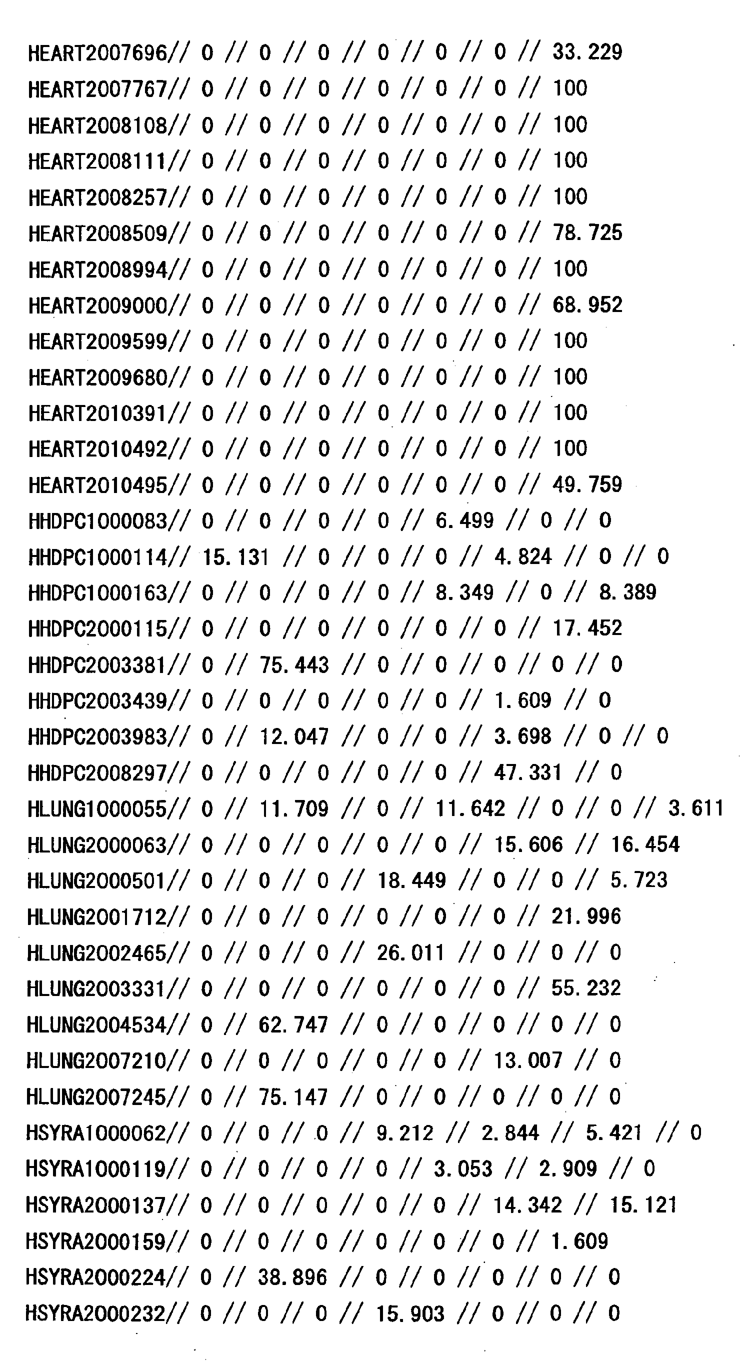

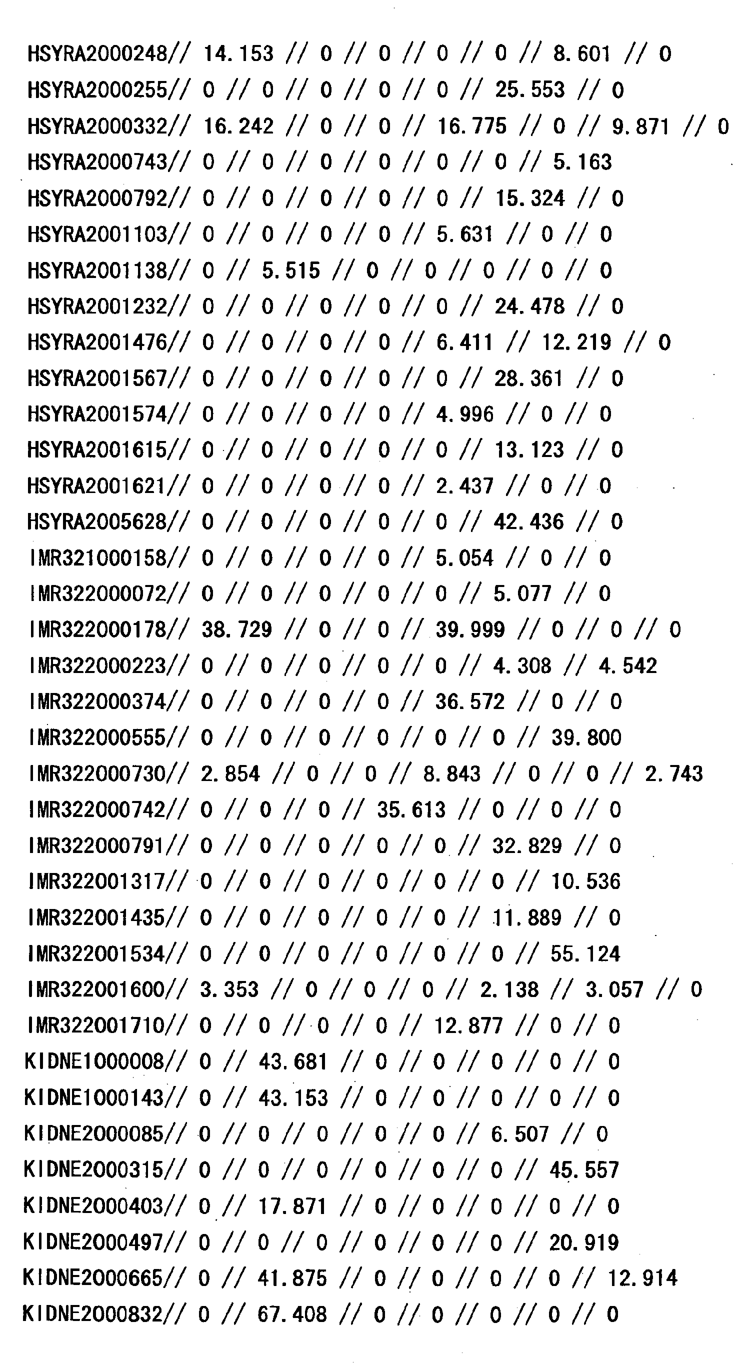

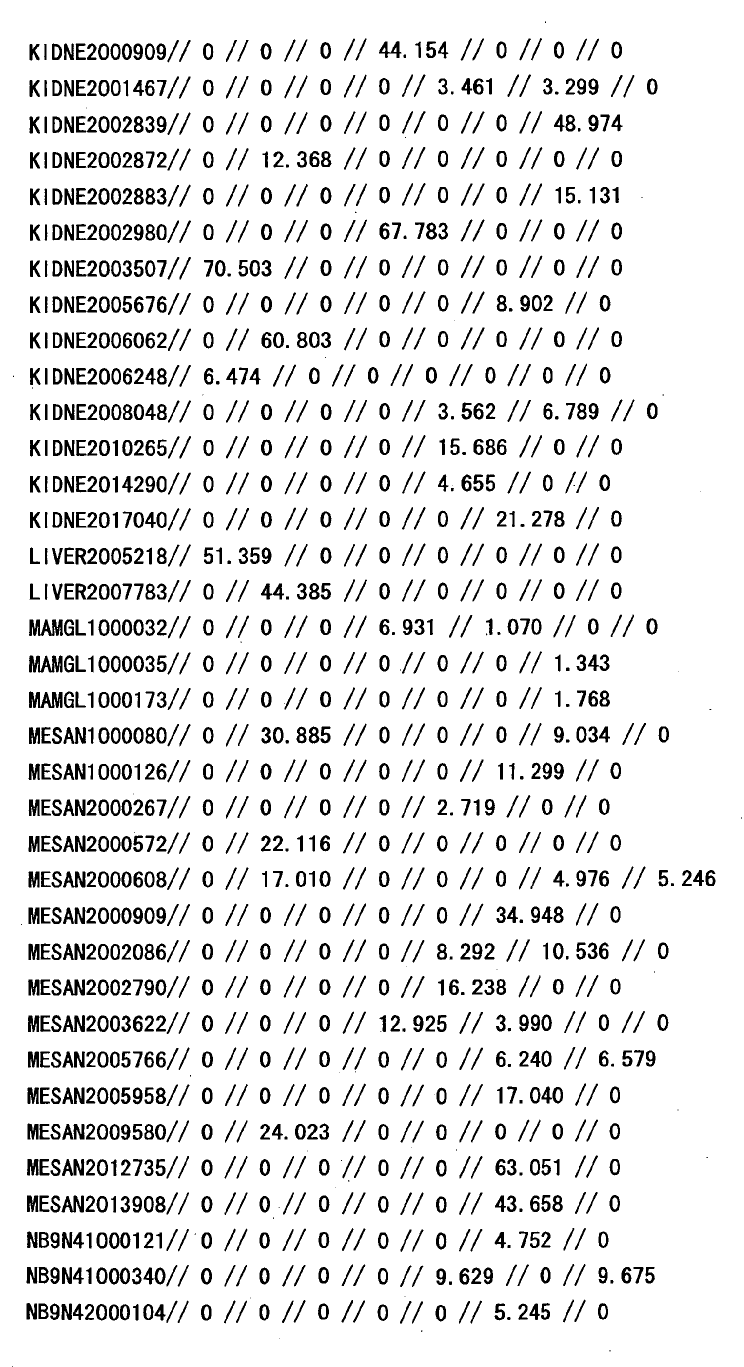

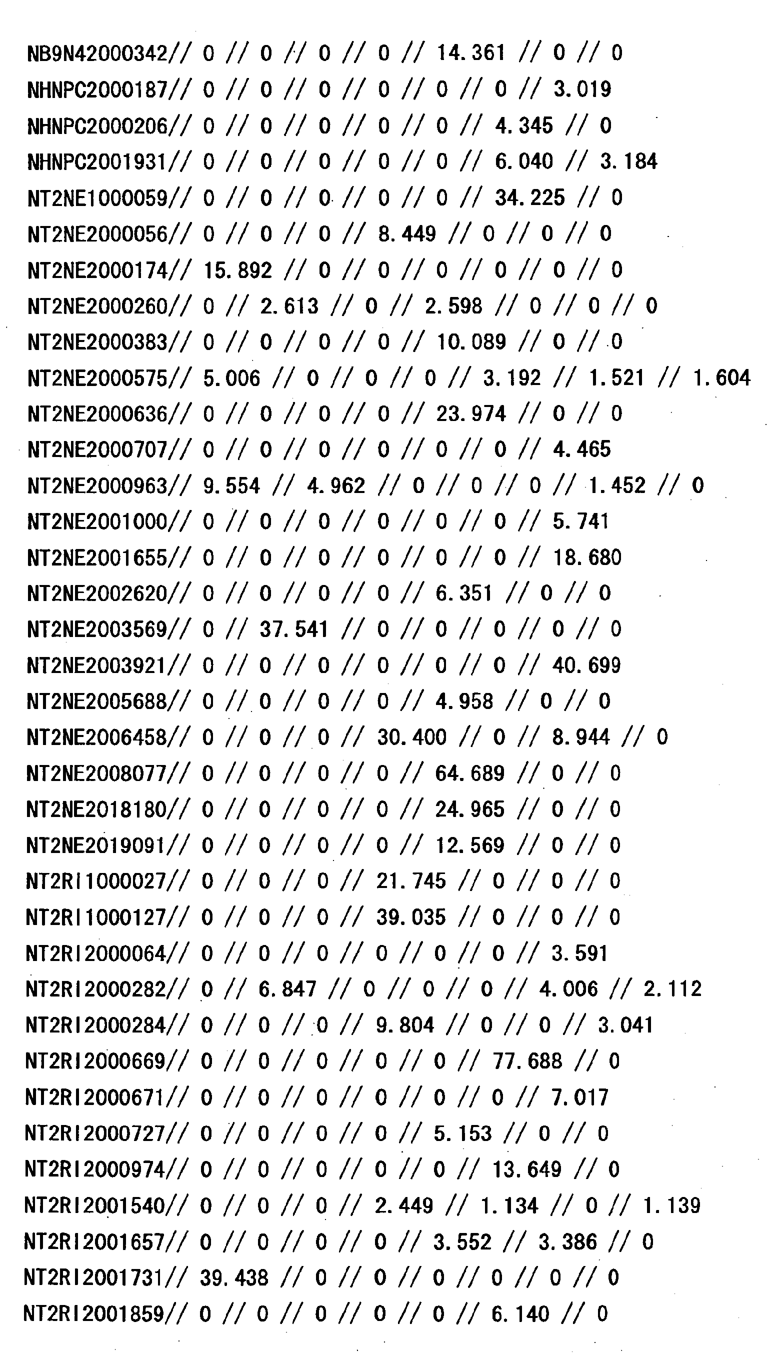

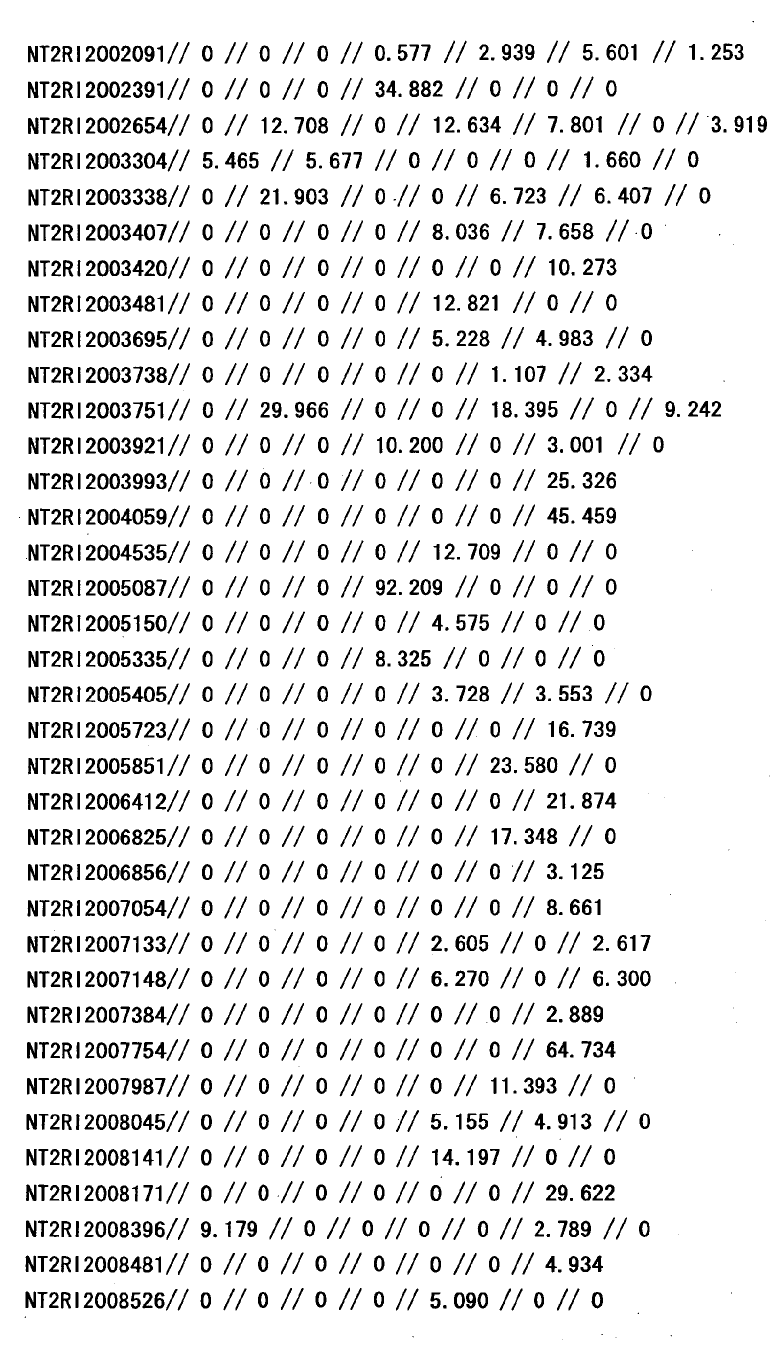

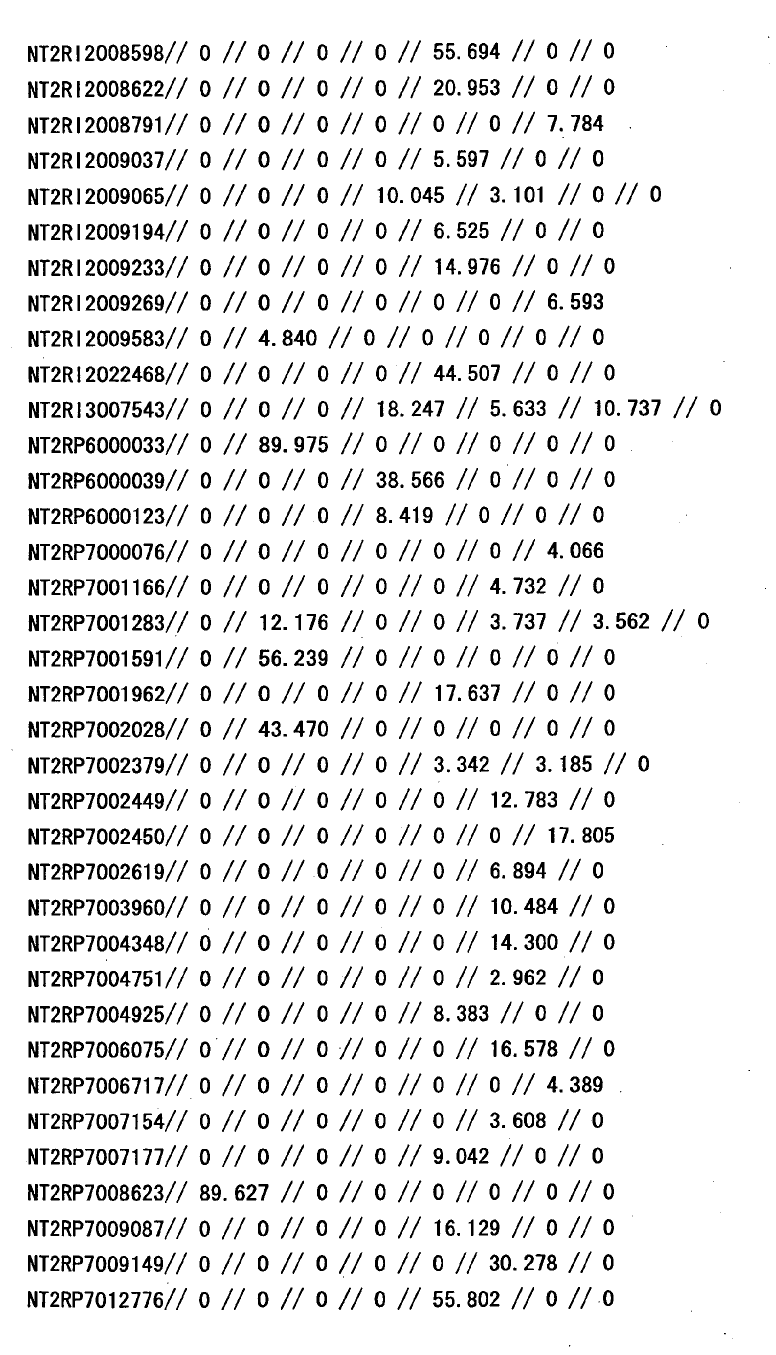

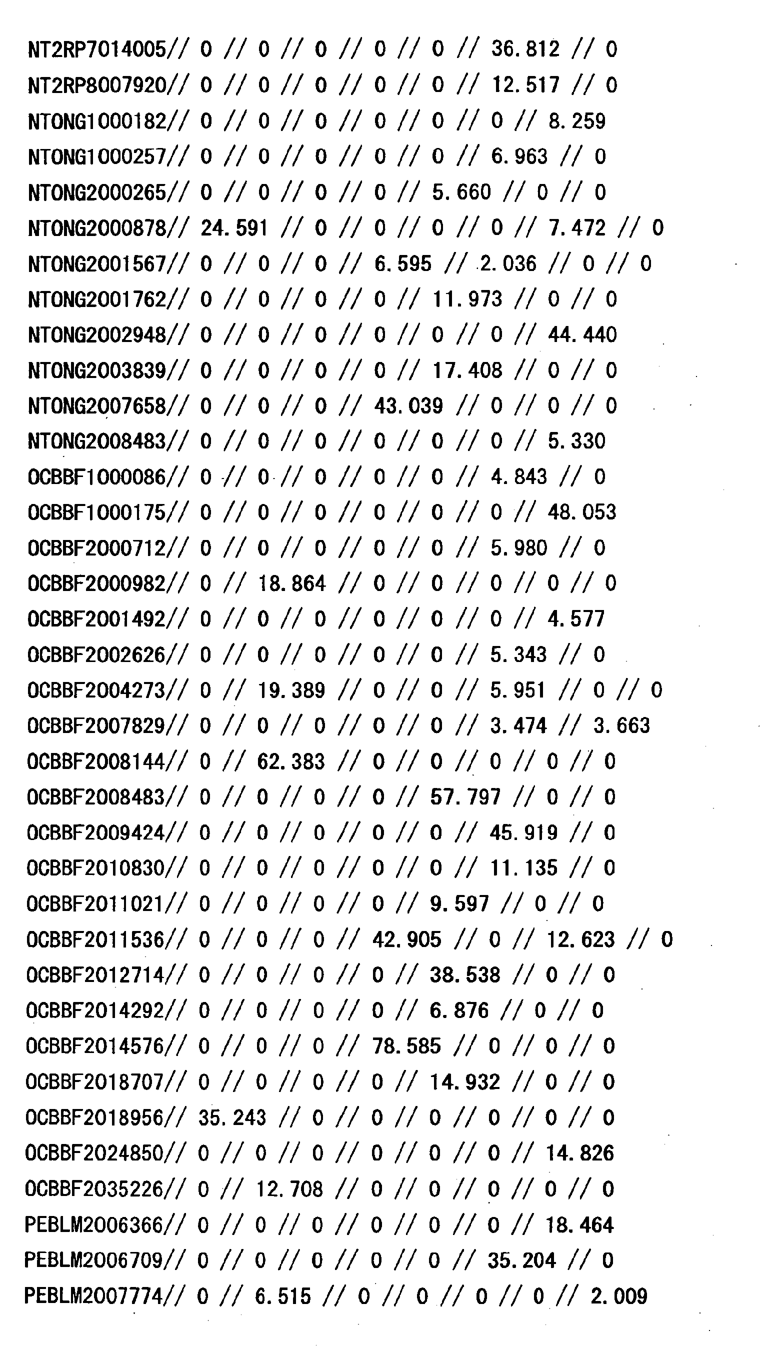

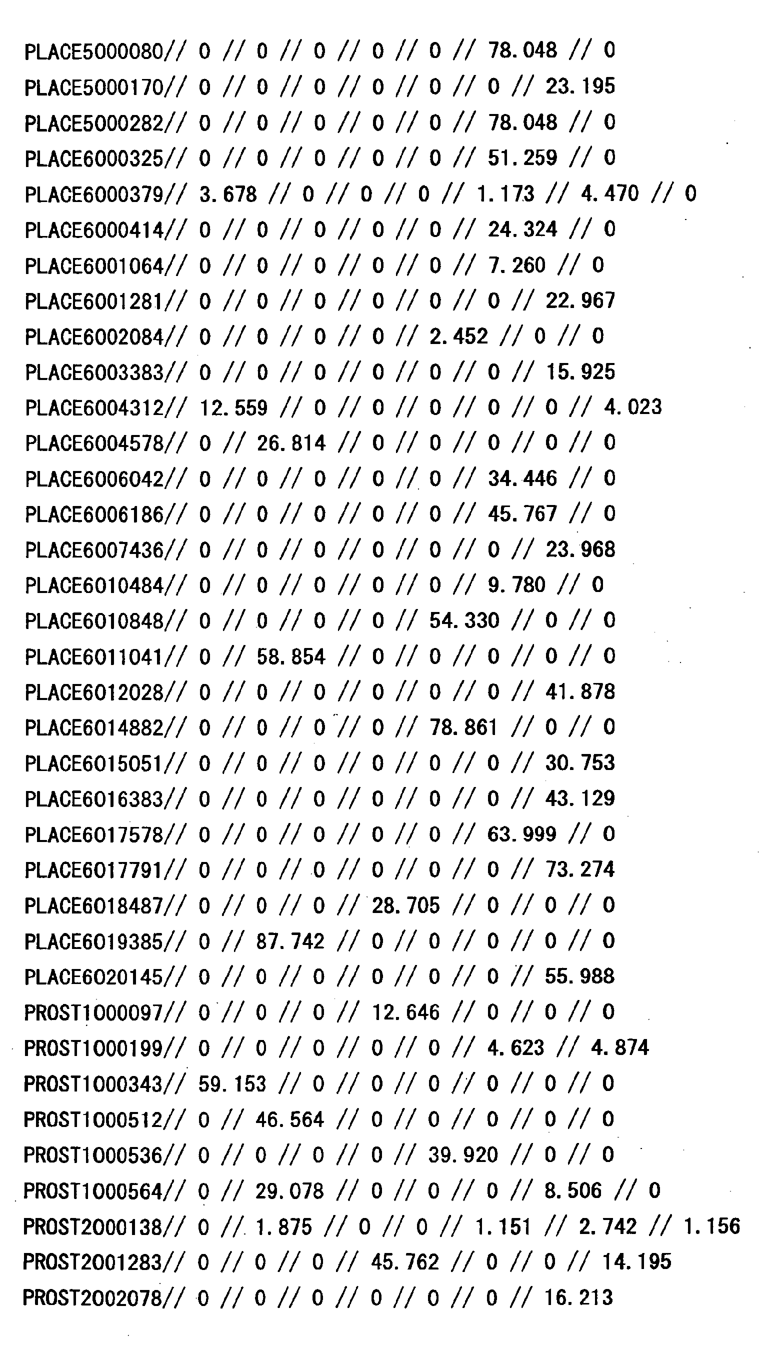

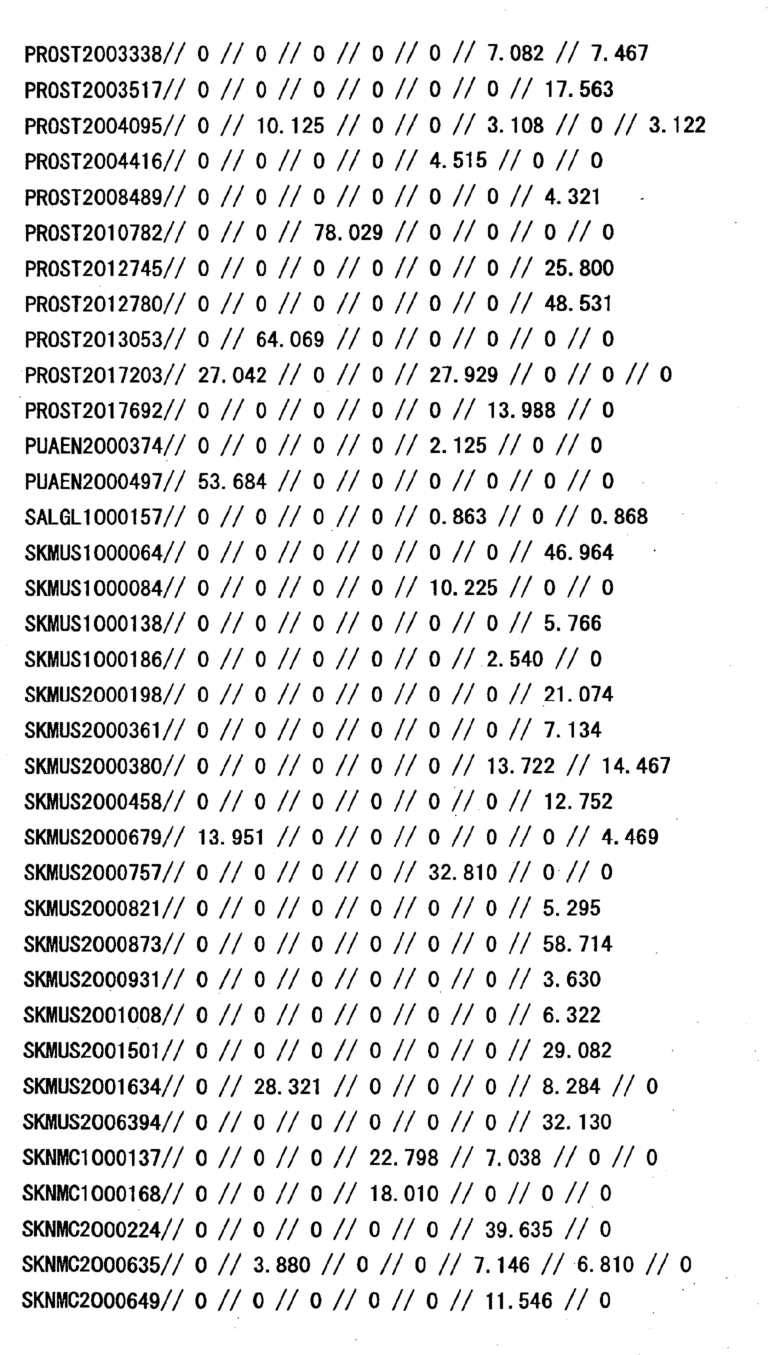

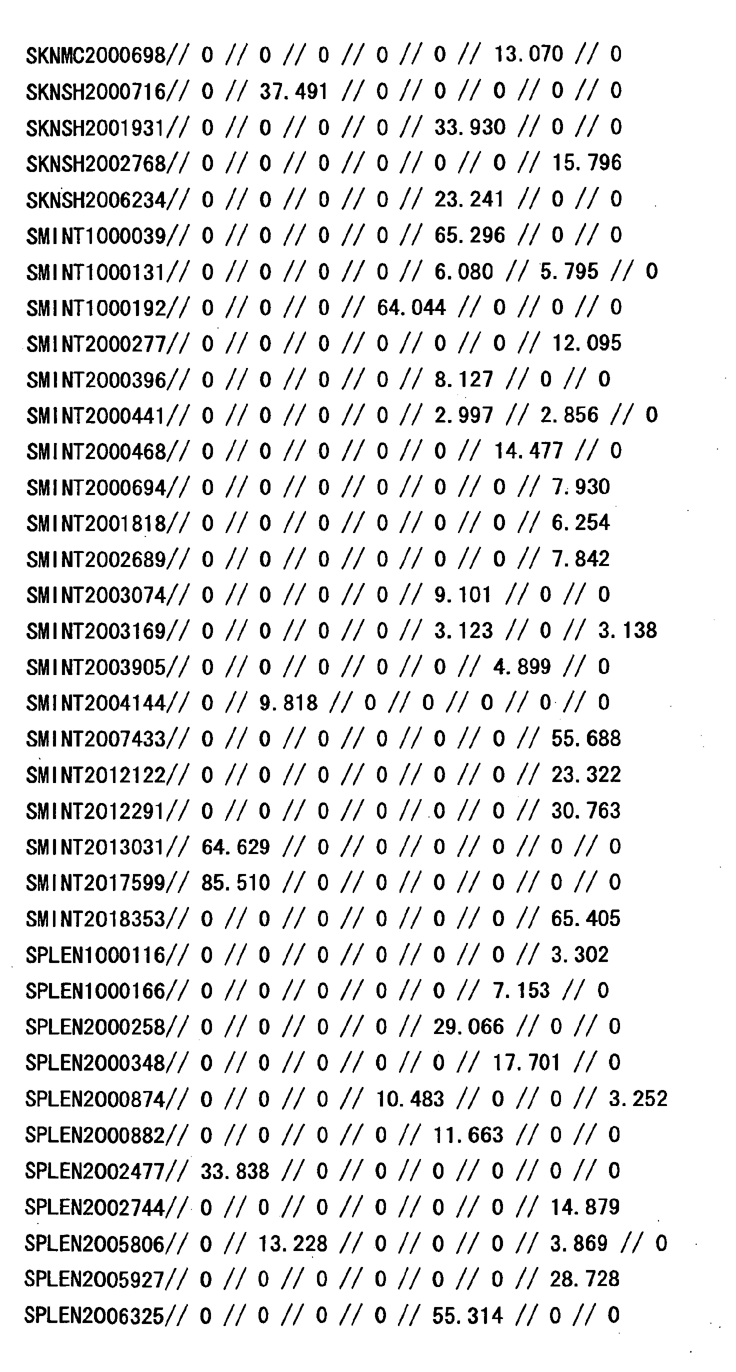

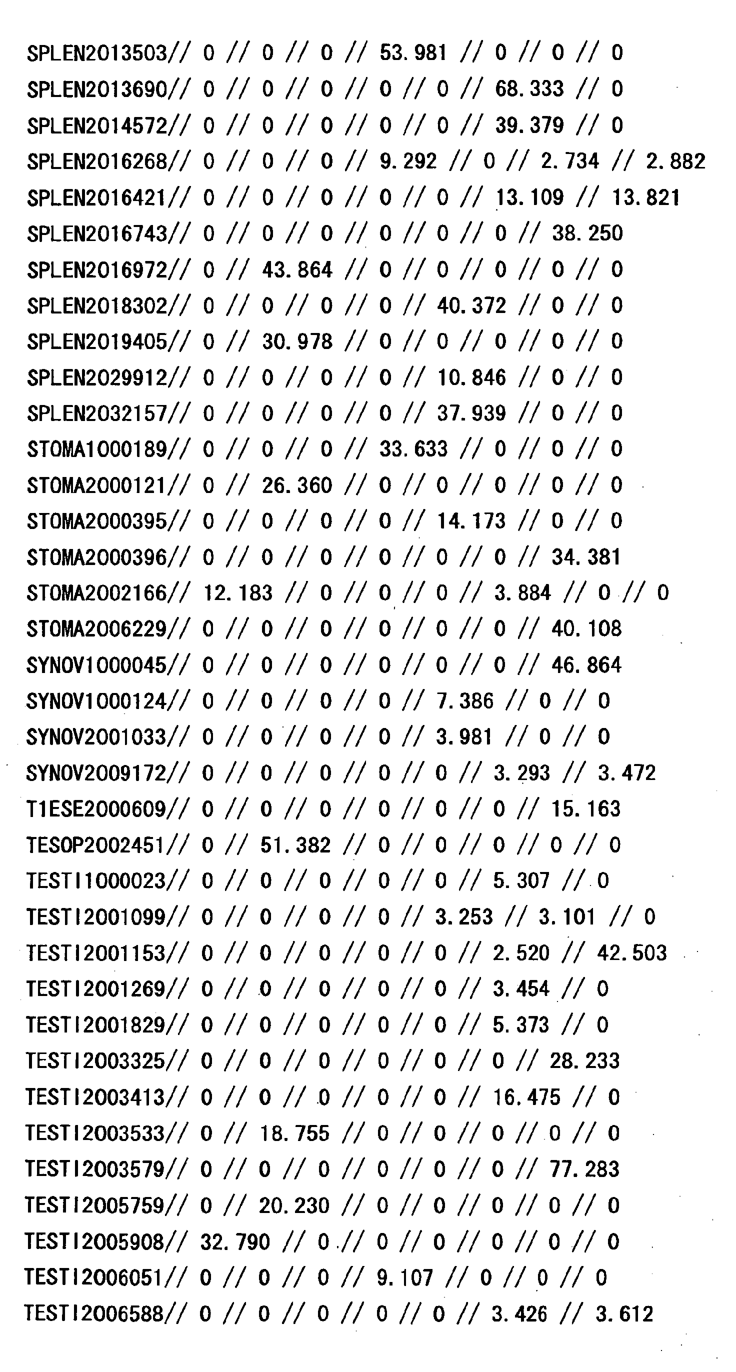

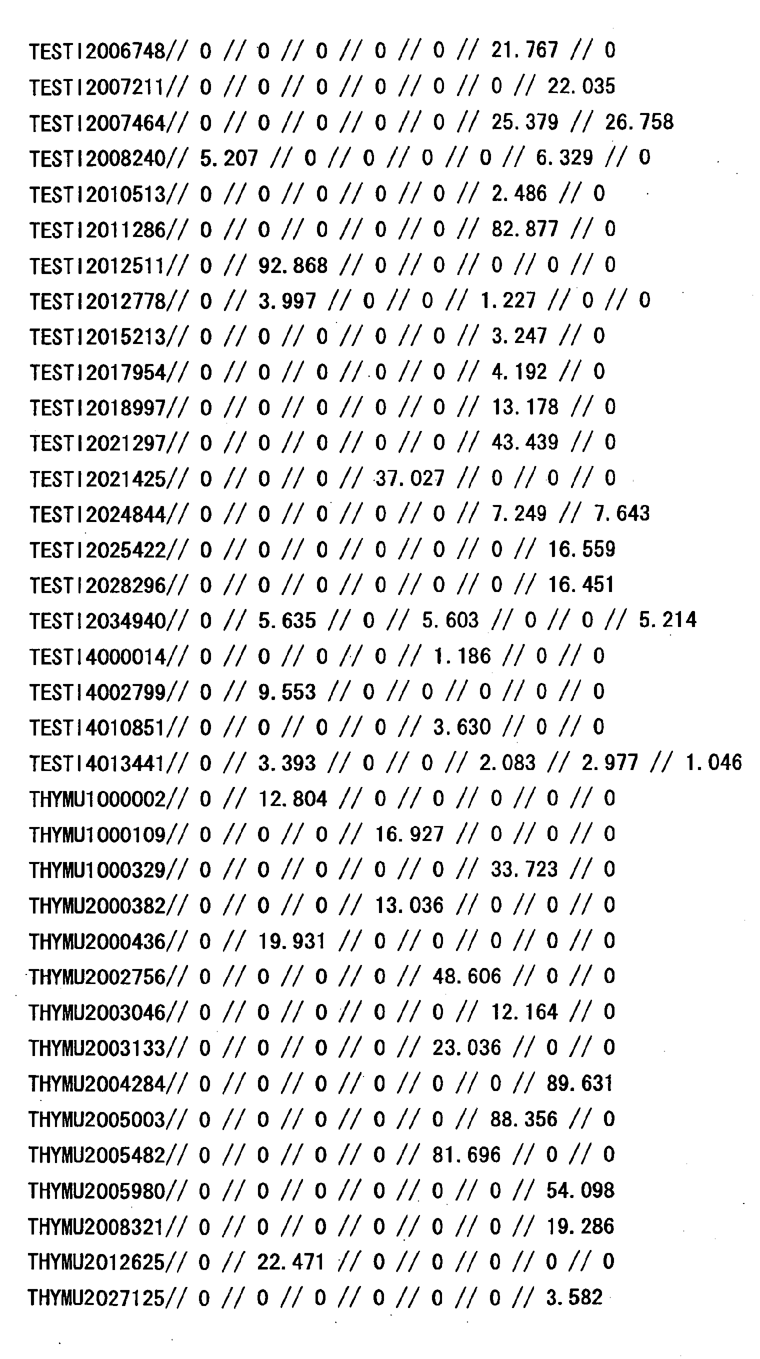

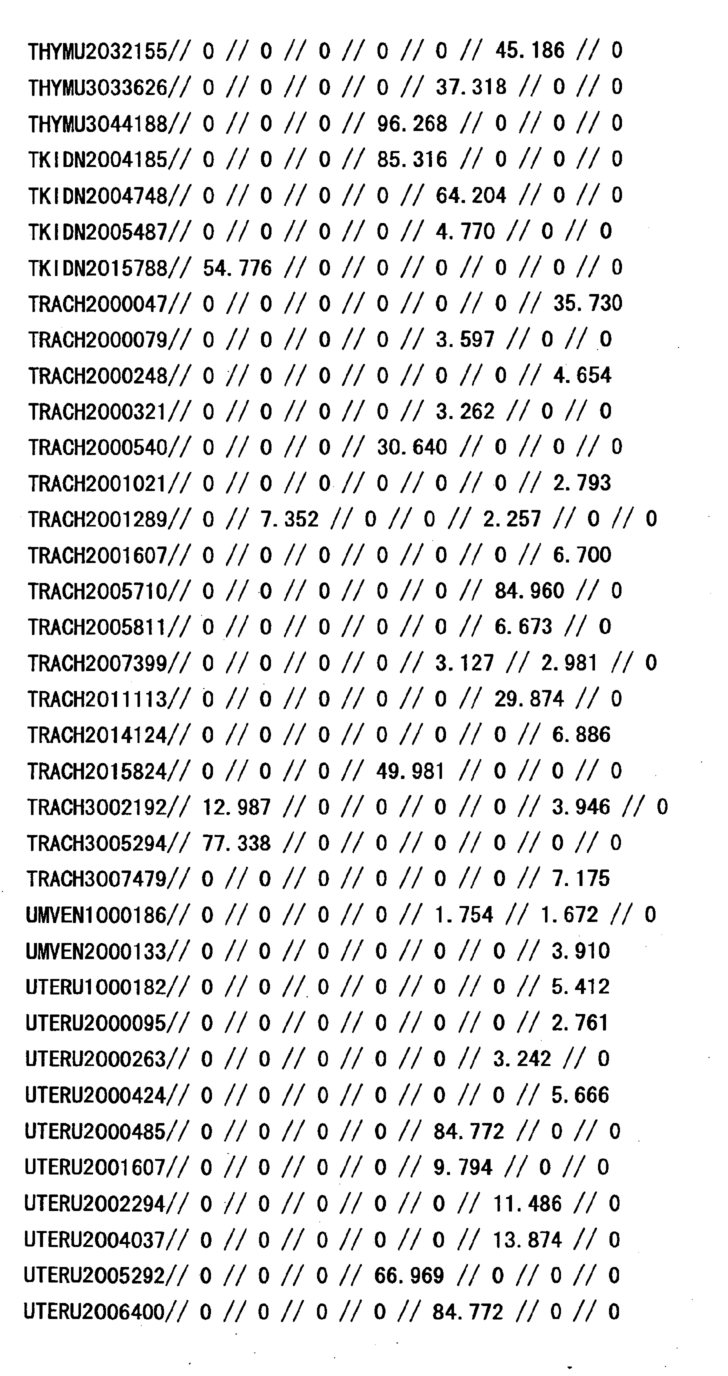

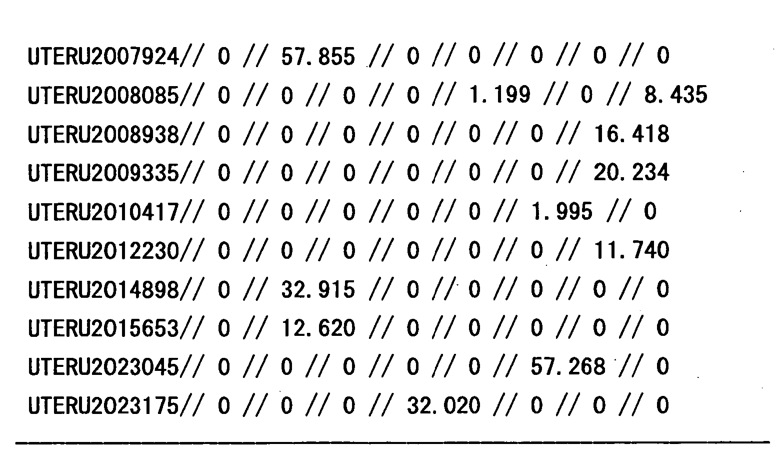

Genes whose expression levels were negligibly low in each of the above-described libraries are shown with their clone names below.
3NB691000018, 3NB691000085, 3NB691000113, 3NB691000116, 3NB691000191, 3NB692000012, 3NB692000029, 3NB692000102, 3NB692000154, 3NB692000276, 3NB692000305, 3NB692000330, 3NB692000374, 3NB692000429, 3NB692000484, 3NB692000529, 3NB692000545, 3NB692000973, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001538, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045,
3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000042, ADRGL2000056, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830,
ADRGL2001836, ADRGL2001854, ADRGL2002013, ADRGL2002029, ADRGL2002191, ADRGL2002260, ADRGL2002392, ADRGL2002477, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283,
ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO2000011, ASTRO2000014, ASTRO2000095, ASTRO2000141, ASTRO2000191, ASTRO2000278, ASTRO2000372, ASTRO2000381, ASTRO2000403, ASTRO2000417, ASTRO2000480, ASTRO2000482, ASTRO2000533, ASTRO2000653, ASTRO2000725, ASTRO2000801, ASTRO2000914,
ASTRO2001001, ASTRO2001008, ASTRO2001029, ASTRO2001088, ASTRO2001107, ASTRO2001122, ASTRO2001227, ASTRO2001249, ASTRO2001480, ASTRO2002024, ASTRO2002035, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003316, ASTRO2003461, ASTRO2003574, ASTRO2003581, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005242, ASTRO2005273, ASTRO2005343, ASTRO2005360, ASTRO2005413, ASTRO2005423,
ASTRO2005553, ASTRO2005557, ASTRO2005575, ASTRO2005593, ASTRO2005687, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007047, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010819, ASTRO2010962, ASTRO2010996, ASTRO2011149,
ASTRO2011422, ASTRO2011426, ASTRO2011437, ASTRO2011461, ASTRO2011834, ASTRO2011893, ASTRO2012552, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014923, ASTRO2014961, ASTRO2015162, ASTRO2015185, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067,
BGGI12000161, BGGI12000533, BGGI12000544, BGGI12000616, BGGI12000684, BGGI12001002, BGGI12001075, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722,
BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000153, BNGH41000169, BNGH41000190, BNGH41000198, BNGH41000216, BNGH41000235, BNGH42000130, BNGH42000132, BNGH42000405, BNGH42000532,
BNGH42000675, BNGH42000739, BNGH42000791, BNGH42000815, BNGH42000994, BNGH42001406, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42006135, BNGH42006226, BNGH42006234, BNGH42007037, BNGH42007366, BNGH42007594, BNGH42007788, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020, BRACE1000047, BRACE1000051, BRACE1000065, BRACE1000073, BRACE1000084, BRACE1000087, BRACE1000092, BRACE1000093, BRACE1000101,
BRACE1000114, BRACE1000124, BRACE1000132, BRACE1000159, BRACE1000166, BRACE1000169, BRACE1000187, BRACE1000239, BRACE1000258, BRACE1000451, BRACE1000475, BRACE1000572, BRACE2000061, BRACE2000077, BRACE2000100, BRACE2000109, BRACE2000141, BRACE2000183, BRACE2000229, BRACE2000232, BRACE2000245, BRACE2000256, BRACE2000266, BRACE2000267, BRACE2000280, BRACE2000300, BRACE2000331, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750,
BRACE2000753, BRACE2000815, BRACE2000864, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001143, BRACE2001151, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001508, BRACE2001534, BRACE2001543, BRACE2001564, BRACE2001588, BRACE2001607, BRACE2001626,
BRACE2001673, BRACE2001677, BRACE2001692, BRACE2001705, BRACE2001709, BRACE2001711, BRACE2001726, BRACE2001733, BRACE2001737, BRACE2001742, BRACE2001777, BRACE2001779, BRACE2001834, BRACE2001865, BRACE2001872, BRACE2001881, BRACE2001892, BRACE2001898, BRACE2001923, BRACE2001942, BRACE2001944, BRACE2001954, BRACE2001972, BRACE2001973, BRACE2002026, BRACE2002034, BRACE2002050, BRACE2002081, BRACE2002091, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002394, BRACE2002409, BRACE2002431, BRACE2002441, BRACE2002444, BRACE2002458, BRACE2002467, BRACE2002468, BRACE2002478,
BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002860, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003254, BRACE2003269, BRACE2003285, BRACE2003319, BRACE2003398,
BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003845, BRACE2003846, BRACE2003847, BRACE2003848, BRACE2003873, BRACE2003885, BRACE2003887, BRACE2003892, BRACE2003904, BRACE2003905, BRACE2003928, BRACE2003944, BRACE2003954, BRACE2003982, BRACE2003987, BRACE2005034, BRACE2005047, BRACE2005063, BRACE2005083, BRACE2005090, BRACE2005099, BRACE2005118, BRACE2005124, BRACE2005138, BRACE2005151, BRACE2005160, BRACE2005169, BRACE2005189, BRACE2005193,
BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005395, BRACE2005408, BRACE2005433, BRACE2005434, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005949, BRACE2005981,
BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281, BRACE2006298, BRACE2006319, BRACE2006344, BRACE2006354, BRACE2006363, BRACE2006378, BRACE2006380, BRACE2006393, BRACE2006397, BRACE2006413, BRACE2006417, BRACE2006453, BRACE2006459, BRACE2006488, BRACE2006514, BRACE2006534, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833,
BRACE2006859, BRACE2006871, BRACE2006909, BRACE2006911, BRACE2006924, BRACE2006935, BRACE2006937, BRACE2006951, BRACE2006982, BRACE2006986, BRACE2007013, BRACE2007064, BRACE2007068, BRACE2007087, BRACE2007089, BRACE2007109, BRACE2007138, BRACE2007174, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007518, BRACE2007522, BRACE2007527, BRACE2007538, BRACE2007563, BRACE2007567, BRACE2007577, BRACE2007621, BRACE2007640, BRACE2007641, BRACE2007646, BRACE2007663, BRACE2007671,
BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008385, BRACE2008399, BRACE2008401, BRACE2008402, BRACE2008410, BRACE2008420, BRACE2008443,
BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275,
BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009517, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983,
BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013911, BRACE2014108, BRACE2014161, BRACE2014232, BRACE2014257, BRACE2014268, BRACE2014306, BRACE2014475, BRACE2014657, BRACE2014746, BRACE2014780,
BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992,
BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348,.BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344,
BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022638, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333,
BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345,
BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299,
BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381,
BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE20.47385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595,
BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005938, BRACE3005989,
BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079,
BRACE3010086, BRACE3010221, BRACE3010283, BRACE3010315, BRACE3010397, BRACE3010416, BRACE3010428, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170,
BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531,
BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771,
BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078,
BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376,
BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025,
BRAMY2000052, BRAMY2000076, BRAMY2000086, BRAMY2000151, BRAMY2000181, BRAMY2000196, BRAMY2000231, BRAMY2000277, BRAMY2000388, BRAMY2000508, BRAMY2000562, BRAMY2000585, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287,
BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004363, BRAMY2004387, BRAMY2004492, BRAMY2004521, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466,
BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208,
BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200,
BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016327, BRAMY2016386, BRAMY2016706, BRAMY2016771, BRAMY2016892, BRAMY2016938, BRAMY2016953, BRAMY2017014,
BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019554, BRAMY2019600, BRAMY2019643, BRAMY2019710, BRAMY2019963, BRAMY2019985, BRAMY2019989, BRAMY2020050, BRAMY2020058, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142,
BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024004, BRAMY2024073, BRAMY2024312,
BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451,
BRAMY2027461, BRAMY2027467, BRAMY2027587, BRAMY2027714, BRAMY2027717, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032087, BRAMY2032231, BRAMY2032242, BRAMY2032290, BRAMY2032311, BRAMY2032317, BRAMY2032344, BRAMY2032589, BRAMY2032678,
BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100,
BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317,
BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205,
BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000089, BRAMY4000095,
BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000045, BRAWH1000083, BRAWH1000093, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417,
BRAWH2000443, BRAWH2000460, BRAWH2000471, BRAWH2000476, BRAWH2000482, BRAWH2000488, BRAWH2000494, BRAWH2000500, BRAWH2000503, BRAWH2000522, BRAWH2000554, BRAWH2000595, BRAWH2000633, BRAWH2000643, BRAWH2000651, BRAWH2000682, BRAWH2000686, BRAWH2000697, BRAWH2000752, BRAWH2000859, BRAWH2000866, BRAWH2000892, BRAWH2000901, BRAWH2000926, BRAWH2000944, BRAWH2000984, BRAWH2001037, BRAWH2001052, BRAWH2001103, BRAWH2001129, BRAWH2001137, BRAWH2001141, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406, BRAWH2001412, BRAWH2001418, BRAWH2001424, BRAWH2001436, BRAWH2001438,
BRAWH2001439, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002967, BRAWH2002976, BRAWH2003000, BRAWH2003025, BRAWH2003135, BRAWH2003240, BRAWH2003307, BRAWH2003355,
BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005661, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406,
BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380, BRAWH2008540, BRAWH2008577, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536,
BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014188,
BRAWH2014234, BRAWH2014379, BRAWH2014414, BRAWH2014473, BRAWH2014511, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534,
BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244,
BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461,
BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884,
BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806,
BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149,
BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401,
BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886,
BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026340,
BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003213, BRCOC2003513, BRCOC2003732,
BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013,
BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018877, BRCOC2019255, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000553, BRHIP2000621, BRHIP2000691, BRHIP2000819, BRHIP2000826, BRHIP2000920, BRHIP2001074, BRHIP2001099,
BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269,
BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958,
BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884,
BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022228, BRHIP2022298, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024742, BRHIP2024789, BRHIP2024911,
BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000111, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076,
BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683,
BRHIP3006717, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745,
BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020046, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844,
BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003012, BRSSN2003086, BRSSN2003093, BRSSN2003820, BRSSN2003841,
BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198,
BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015238, BRSSN2015369, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070,
BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284,
BRSTN2012320, BRSTN2012380, BRSTN2012451, BRSTN2013171, BRSTN2013354, BRSTN2013502, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016892, BRSTN2016918, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133,
BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665,
BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012980, BRTHA2013054, BRTHA2013262, BRTHA2013275, BRTHA2013426, BRTHA2013460, BRTHA2013515, BRTHA2013540,
BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014,
BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448,
BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3003736, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113,
BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908,
BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152,
CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337,
COLON2009499, CORDB1000140, CORDB2000061, CORDB2000127, CORDB2000541, CTONG1000009, CTONG1000010, CTONG1000020, CTONG1000022, CTONG1000040, CTONG1000052, CTONG1000056, CTONG1000062, CTONG1000086, CTONG1000088, CTONG1000093, CTONG1000094, CTONG1000097, CTONG1000113, CTONG1000137, CTONG1000165, CTONG1000180, CTONG1000181, CTONG1000218, CTONG1000220, CTONG1000241, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000411, CTONG2000465, CTONG2000469, CTONG2000480,
CTONG2000508, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001749, CTONG2001800, CTONG2001820, CTONG2001858, CTONG2001877, CTONG2001911, CTONG2001932, CTONG2001955, CTONG2002066, CTONG2002095, CTONG2002143, CTONG2002270, CTONG2002395, CTONG2002417, CTONG2002418, CTONG2002453, CTONG2002558, CTONG2002590, CTONG2002709, CTONG2002721, CTONG2002744, CTONG2002766, CTONG2002803, CTONG2002816, CTONG2002820, CTONG2002832, CTONG2002841, CTONG2002903,
CTONG2002965, CTONG2002999, CTONG2003028, CTONG2003094, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003524, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005290, CTONG2005374, CTONG2005399,
CTONG2005468, CTONG2005553, CTONG2005567, CTONG2005585, CTONG2005615, CTONG2005635, CTONG2005775, CTONG2005795, CTONG2005890, CTONG2005904, CTONG2005913, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006568, CTONG2006611, CTONG2006620, CTONG2006666, CTONG2006691, CTONG2006709, CTONG2006798, CTONG2006836, CTONG2006942, CTONG2006964, CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007500,
CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321, CTONG2008343, CTONG2008398, CTONG2008402, CTONG2008405, CTONG2008466, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604,
CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010348, CTONG2010408, CTONG2010508, CTONG2010566, CTONG2010623, CTONG2010649, CTONG2010652, CTONG2010803, CTONG2011188, CTONG2011429, CTONG2011474, CTONG2011676, CTONG2011716, CTONG2011765, CTONG2011779, CTONG2011801, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012158,
CTONG2012230, CTONG2012401, CTONG2012422, CTONG2012425, CTONG2012447, CTONG2012453, CTONG2012473, CTONG2012476, CTONG2012533, CTONG2012554, CTONG2012607, CTONG2012661, CTONG2012724, CTONG2012738, CTONG2012758, CTONG2012843, CTONG2012847, CTONG2012879, CTONG2012901, CTONG2012996, CTONG2013128, CTONG2013149, CTONG2013156, CTONG2013178, CTONG2013222, CTONG2013284, CTONG2013339, CTONG2013348, CTONG2013352, CTONG2013381, CTONG2013393, CTONG2013511, CTONG2013630, CTONG2013803, CTONG2013934, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630,
CTONG2014635, CTONG2014646, CTONG2014705, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016056, CTONG2016185, CTONG2016217, CTONG2016355, CTONG2016408, CTONG2016499, CTONG2016505, CTONG2016559, CTONG2016658, CTONG2016775, CTONG2016797, CTONG2016846, CTONG2016869, CTONG2016904, CTONG2016942, CTONG2016953, CTONG2017021, CTONG2017094, CTONG2017292, CTONG2017409, CTONG2017429, CTONG2017444, CTONG2017458, CTONG2017604,
CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018343, CTONG2018379, CTONG2018383, CTONG2018413, CTONG2018483, CTONG2018629, CTONG2018632, CTONG2018637, CTONG2018652, CTONG2018653, CTONG2018655, CTONG2018735, CTONG2018739, CTONG2018808, CTONG2018843, CTONG2018898, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378,
CTONG2020411, CTONG2020445, CTONG2020522, CTONG2020560, CTONG2020582, CTONG2020638, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024031, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028309, CTONG2028486, CTONG2028518, CTONG2028687, CTONG2028758, CTONG3000686, CTONG3000707, CTONG3000896,
CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659,
CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008894, CTONG3008951, CTONG3008959, CTONG3009227, CTONG3009239, CTONG3009287, CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000195, D3OST3000258, D3OST3000291,
D3OST3000388, D3OST3000493, D6OST2000127, D60ST2000169, D6OST2000245, D6OST2000358, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003580, D9OST2003594, D9OST2003762, D9OST2003791, D9OST2003989, D9OST2004018, D9OST2004073, D9OST2004417, DFNES1000003, DFNES1000083, DFNES1000107, DFNES1000150, DFNES1000185, DFNES2000143, DFNES2000146, DFNES2000153, DFNES2000212, DFNES2000268, DFNES2000292, DFNES2000335, DFNES2000426, DFNES2000432, DFNES2000443, DFNES2000457, DFNES2000471, DFNES2000526, DFNES2000913, DFNES2001091, DFNES2001096, DFNES2001108, DFNES2001177,
DFNES2001404, DFNES2001564, DFNES2001565, DFNES2001647, DFNES2001800, DFNES2001829, DFNES2002220, DFNES2002550, DFNES2002588, DFNES2002817, DFNES2002966, DFNES2003081, DFNES2003192, DFNES2003255, DFNES2003742, DFNES2004346, DFNES2004354, DFNES2004371, DFNES2004383, DFNES2004608, DFNES2004684, DFNES2005266, DFNES2005527, DFNES2005690, DFNES2005779, DFNES2005875, DFNES2006346, DFNES2006944, DFNES2007113, DFNES2007299, DFNES2007634, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000024,
FCBBF1000025, FCBBF1000038, FCBBF1000053, FCBBF1000061, FCBBF1000063, FCBBF1000069, FCBBF1000077, FCBBF1000115, FCBBF1000117, FCBBF1000118, FCBBF1000121, FCBBF1000125, FCBBF1000131, FCBBF1000155, FCBBF1000157, FCBBF1000171, FCBBF1000174, FCBBF1000182, FCBBF1000197, FCBBF1000198, FCBBF1000206, FCBBF1000220, FCBBF1000232, FCBBF1000233, FCBBF1000243, FCBBF1000270, FCBBF1000272, FCBBF1000294, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000466, FCBBF1000476, FCBBF1000506, FCBBF1000509, FCBBF1000546, FCBBF1000550, FCBBF1000556,
FCBBF1000563, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000748, FCBBF1000760, FCBBF2000038, FCBBF2000087, FCBBF2000094, FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000591, FCBBF2000677, FCBBF2000678, FCBBF2000685, FCBBF2000733, FCBBF2000782, FCBBF2000808, FCBBF2000815, FCBBF2000825, FCBBF2000885, FCBBF2000940, FCBBF2000951, FCBBF2001001, FCBBF2001088, FCBBF2001105, FCBBF2001116,
FCBBF2001183, FCBBF2001188, FCBBF2001211, FCBBF2001238, FCBBF2001243, FCBBF2001299, FCBBF2001309, FCBBF2001400, FCBBF2001427, FCBBF2001480, FCBBF2001529, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373,
FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005733, FCBBF2005909, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847,
FCBBF3001018, FCBBF3001020, FCBBF3001081, FCBBF3001235, FCBBF3001281, FCBBF3001302, FCBBF3001377, FCBBF3001594, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004473, FCBBF3004502, FCBBF3004715, FCBBF3004842, FCBBF3004847, FCBBF3004955, FCBBF3005160,
FCBBF3005218, FCBBF3005444, FCBBF3005497, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479,
FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800,
FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019564,
FCBBF3019569, FCBBF3019570, FCBBF3019663, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3023061, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285,
FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041,
FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000105, FEBRA2000126, FEBRA2000210, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452,
FEBRA2000454, FEBRA2000462, FEBRA2000468, FEBRA2000491, FEBRA2000504, FEBRA2000510, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000757, FEBRA2000762, FEBRA2000771, FEBRA2000772, FEBRA2000782, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175,
FEBRA2001197, FEBRA2001217, FEBRA2001227, FEBRA2001245, FEBRA2001267, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001828, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002508,
FEBRA2002525, FEBRA2002527, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003100, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003750, FEBRA2003822, FEBRA2003833,
FEBRA2003897, FEBRA2003907, FEBRA2003930, FEBRA2004023, FEBRA2004026, FEBRA2004029, FEBRA2004042, FEBRA2004053, FEBRA2004056, FEBRA2004091, FEBRA2004110, FEBRA2004191, FEBRA2004202, FEBRA2004219, FEBRA2004237, FEBRA2004268, FEBRA2004293, FEBRA2004325, FEBRA2004329, FEBRA2004331, FEBRA2004412, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458,
FEBRA2005476, FEBRA2005701, FEBRA2005726, FEBRA2005752, FEBRA2005778, FEBRA2005787, FEBRA2005788, FEBRA2005824, FEBRA2005995, FEBRA2005998, FEBRA2006055, FEBRA2006061, FEBRA2006092, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006370, FEBRA2006372, FEBRA2006379, FEBRA2006396, FEBRA2006401, FEBRA2006409, FEBRA2006418, FEBRA2006427, FEBRA2006476, FEBRA2006485, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017,
FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007708, FEBRA2007714, FEBRA2007793, FEBRA2007801, FEBRA2007818, FEBRA2007823, FEBRA2007900, FEBRA2007901, FEBRA2007931, FEBRA2007957, FEBRA2008009, FEBRA2008036, FEBRA2008081, FEBRA2008086, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008302, FEBRA2008311, FEBRA2008341, FEBRA2008360, FEBRA2008459, FEBRA2008468, FEBRA2008475, FEBRA2008481, FEBRA2008542,
FEBRA2008660, FEBRA2008662, FEBRA2008681, FEBRA2008692, FEBRA2008741, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063,
FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502,
FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018476, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019711, FEBRA2019867, FEBRA2020406, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255, FEBRA2022322, FEBRA2022601,
FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023498, FEBRA2023550, FEBRA2023721, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEBRA2028516, HELAC2000158,
HELAC2000301, HHDPC1000001, HHDPC1000065, HHDPC1000118, HHDPC2000055, HHDPC2000070, HHDPC2000102, HHDPC2000104, HHDPC2000149, HHDPC2000258, HHDPC2000315, HHDPC2000455, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003472, HHDPC2004757, HHDPC2005185, HHDPC2005513, HHDPC2005742, HHDPC2005794, HHDPC2006460, HHDPC2006862, HHDPC2006927, HHDPC2007267, HHDPC2007302, HHDPC2007775, HHDPC2008123, HHDPC2008185, HHDPC2008279, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030,
HLUNG1000034, HLUNG1000037, HLUNG1000053, HLUNG1000064, HLUNG1000076, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000412, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013,
HLUNG2001126, HLUNG2001144, HLUNG2001146, HLUNG2001214, HLUNG2001439, HLUNG2001459, HLUNG2001507, HLUNG2001518, HLUNG2001633, HLUNG2001677, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002562, HLUNG2002648, HLUNG2002659, HLUNG2002707, HLUNG2002811, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918,
HLUNG2003991, HLUNG2004014, HLUNG2004037, HLUNG2004067, HLUNG2004154, HLUNG2004159, HLUNG2004170, HLUNG2004217, HLUNG2004273, HLUNG2004388, HLUNG2004416, HLUNG2004521, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007219, HLUNG2007433, HLUNG2007931, HLUNG2008066,
HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009067, HLUNG2009215, HLUNG2009225, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288,
HLUNG2014449, HLUNG2014821, HLUNG2015184, HLUNG2015418, HLUNG2015548, HLUNG2015578, HLUNG2015617, HLUNG2016022, HLUNG2016862, HLUNG2017262, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA1000152, HSYRA1000178, HSYRA2000044, HSYRA2000135, HSYRA2000190, HSYRA2000221, HSYRA2000237, HSYRA2000253, HSYRA2000371, HSYRA2000424, HSYRA2000507, HSYRA2000510, HSYRA2000605, HSYRA2000629, HSYRA2000640, HSYRA2000706, HSYRA2000760, HSYRA2000787, HSYRA2000828, HSYRA2000832, HSYRA2000925, HSYRA2000927, HSYRA2001003, HSYRA2001105, HSYRA2001142, HSYRA2001153, HSYRA2001225, HSYRA2001255,
HSYRA2001332, HSYRA2001353, HSYRA2001396, HSYRA2001420, HSYRA2001484, HSYRA2001552, HSYRA2001580, HSYRA2001595, HSYRA2001631, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000161, IMR321000165, IMR321000193, IMR321000207, IMR321000210, IMR321000219, IMR321000230, IMR321000242, IMR321000253, IMR321000266, IMR322000010, IMR322000107, IMR322000121, IMR322000127, IMR322000144, IMR322000152, IMR322000164, IMR322000243, IMR322000302, IMR322000316,
IMR322000379, IMR322000383, IMR322000418, IMR322000518, IMR322000604, IMR322000609, IMR322000656, IMR322000672, IMR322000775, IMR322000811, IMR322000819, IMR322000838, IMR322000859, IMR322000863, IMR322000917, IMR322000918, IMR322000919, IMR322000935, IMR322000953, IMR322000973, IMR322000984, IMR322001018, IMR322001049, IMR322001167, IMR322001185, IMR322001218, IMR322001245, IMR322001318, IMR322001322, IMR322001332, IMR322001380, IMR322001438, IMR322001452, IMR322001491, IMR322001541, IMR322001584, IMR322001665, IMR322001670, IMR322001724, IMR322001879, IMR322002035, IMR322002110, IMR322002470, IMR322002993, IMR322003675, IMR322004768, IMR322005293, IMR322005833, IMR322006012, IMR322006222,
IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, IMR322019070, JCMLC2000273, KIDNE1000012, KIDNE1000015, KIDNE1000028, KIDNE1000036, KIDNE1000064, KIDNE1000104, KIDNE1000121, KIDNE1000145, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000252, KIDNE2000266, KIDNE2000287, KIDNE2000317,
KIDNE2000328, KIDNE2000330, KIDNE2000335, KIDNE2000349, KIDNE2000375, KIDNE2000383, KIDNE2000394, KIDNE2000422, KIDNE2000493, KIDNE2000510, KIDNE2000513, KIDNE2000517, KIDNE2000519, KIDNE2000555, KIDNE2000574, KIDNE2000624, KIDNE2000678, KIDNE2000717, KIDNE2000721, KIDNE2000722, KIDNE2000777, KIDNE2000801, KIDNE2000833, KIDNE2000846, KIDNE2000867, KIDNE2000947, KIDNE2001117, KIDNE2001140, KIDNE2001162, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002265,
KIDNE2002438, KIDNE2002483, KIDNE2002725, KIDNE2002768, KIDNE2002795, KIDNE2002798, KIDNE2002845, KIDNE2002882, KIDNE2002991, KIDNE2003185, KIDNE2003188, KIDNE2003233, KIDNE2003305, KIDNE2003316, KIDNE2003335, KIDNE2003357, KIDNE2003373, KIDNE2003377, KIDNE2003752, KIDNE2003764, KIDNE2003837, KIDNE2003941, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879,
KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006014, KIDNE2006030, KIDNE2006039, KIDNE2006053, KIDNE2006092, KIDNE2006145, KIDNE2006149, KIDNE2006210, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077,
KIDNE2007186, KIDNE2007222, KIDNE2007328, KIDNE2007352, KIDNE2007356, KIDNE2007422, KIDNE2007569, KIDNE2007810, KIDNE2007811, KIDNE2007910, KIDNE2007944, KIDNE2007954, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628,
KIDNE2009647, KIDNE2009676, KIDNE2009723, KIDNE2010007, KIDNE2010049, KIDNE2010052, KIDNE2010084, KIDNE2010137, KIDNE2010151, KIDNE2010264; KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263,
KIDNE2013346, KIDNE2013388, KIDNE2013413, KIDNE2013489, KIDNE2013639, KIDNE2013731, KIDNE2013734, KIDNE2013775, KIDNE2013801, KIDNE2013845, KIDNE2013866, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327,
KIDNE2016371, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126,
LIVER1000132, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2002842, LIVER2003008,
LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017,
MAMGL1000083, MAMGL1000096, MAMGL1000100, MAMGL1000132, MAMGL1000151, MAMGL1000178, MAMGL1000182, MAMGL1000184, MESAN1000032, MESAN1000035, MESAN1000050, MESAN1000079, MESAN1000101, MESAN1000121, MESAN1000147, MESAN1000180, MESAN2000023, MESAN2000026, MESAN2000067, MESAN2000092, MESAN2000149, MESAN2000167, MESAN2000264, MESAN2000291, MESAN2000337, MESAN2000434, MESAN2000457, MESAN2000462, MESAN2000501, MESAN2000620, MESAN2000711, MESAN2000815, MESAN2000894, MESAN2001154, MESAN2001450, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002724, MESAN2002844, MESAN2002940,
MESAN2002978, MESAN2003035, MESAN2003037, MESAN2003039, MESAN2003058, MESAN2003101, MESAN2003190, MESAN2003322, MESAN2003444, MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005633, MESAN2005689, MESAN2005724, MESAN2005808, MESAN2005811, MESAN2005957, MESAN2006022, MESAN2006043, MESAN2006328, MESAN2006401, MESAN2006563, MESAN2006580, MESAN2006599, MESAN2006743, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926,
MESAN2008936, MESAN2009019, MESAN2009081, MESAN2009105, MESAN2009311, MESAN2009418, MESAN2009503, MESAN2009522, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2013019, MESAN2013022, MESAN2013183, MESAN2013211, MESAN2013283, MESAN2013284, MESAN2013611, MESAN2013674, MESAN2013845, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552,
MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000011, NB9N41000047, NB9N41000135, NB9N41000142, NB9N41000146, NB9N42000122, NB9N42000196, NB9N42000281, NB9N42000314, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182,
NETRP2000439, NHNPC1000034, NHNPC1000037, NHNPC1000084, NHNPC1000101, NHNPC1000124, NHNPC2000062, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000091, NOVAR1000102, NOVAR2000038, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000063, NT2NE1000073, NT2NE1000083, NT2NE1000120, NT2NE1000122, NT2NE1000163, NT2NE1000175, NT2NE1000181, NT2NE1000185, NT2NE2000038, NT2NE2000054, NT2NE2000057, NT2NE2000064, NT2NE2000109,
NT2NE2000137, NT2NE2000156, NT2NE2000195, NT2NE2000222, NT2NE2000259, NT2NE2000284, NT2NE2000299, NT2NE2000327, NT2NE2000353, NT2NE2000356, NT2NE2000369, NT2NE2000374, NT2NE2000384, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000658, NT2NE2000706, NT2NE2000763, NT2NE2000802, NT2NE2000808, NT2NE2000809, NT2NE2000864, NT2NE2000909, NT2NE2000913, NT2NE2000950, NT2NE2000951, NT2NE2000980, NT2NE2001005, NT2NE2001021, NT2NE2001040, NT2NE2001048, NT2NE2001049, NT2NE2001142, NT2NE2001156, NT2NE2001176, NT2NE2001180, NT2NE2001181, NT2NE2001188, NT2NE2001191,
NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001617, NT2NE2001623, NT2NE2001626, NT2NE2001634, NT2NE2001648, NT2NE2001660, NT2NE2001666, NT2NE2001677, NT2NE2001697, NT2NE2001698, NT2NE2001723, NT2NE2001725, NT2NE2001793, NT2NE2001874, NT2NE2001889, NT2NE2001923, NT2NE2002100, NT2NE2002162, NT2NE2002186, NT2NE2002285, NT2NE2002311, NT2NE2002651, NT2NE2002732, NT2NE2002768, NT2NE2002791, NT2NE2002856, NT2NE2002870, NT2NE2003150, NT2NE2003185, NT2NE2003252, NT2NE2003280, NT2NE2003308, NT2NE2003309,
NT2NE2003315, NT2NE2003393, NT2NE2003408, NT2NE2003457, NT2NE2003485, NT2NE2003540, NT2NE2003571, NT2NE2003705, NT2NE2003752, NT2NE2003887, NT2NE2004255, NT2NE2004378, NT2NE2004490, NT2NE2004519, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427,
NT2NE2006432, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010842, NT2NE2010854, NT2NE2011033,
NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275,
NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916, NT2NE2019056, NT2NE2019076, NT2NE2019099, NT2RI1000016, NT2RI1000035, NT2RI1000048, NT2RI1000164, NT2RI1000172, NT2RI2000004, NT2RI2000007,
NT2RI2000107, NT2RI2000116, NT2RI2000117, NT2RI2000128, NT2RI2000133, NT2RI2000167, NT2RI2000173, NT2RI2000187, NT2RI2000255, NT2RI2000263, NT2RI2000270, NT2RI2000276, NT2RI2000294, NT2RI2000313, NT2RI2000341, NT2RI2000344, NT2RI2000348, NT2RI2000412, NT2RI2000421, NT2RI2000480, NT2RI2000575, NT2RI2000578, NT2RI2000588, NT2RI2000597, NT2RI2000685, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000987, NT2RI2001010, NT2RI2001017, NT2RI2001030, NT2RI2001054, NT2RI2001083, NT2RI2001091, NT2RI2001167, NT2RI2001178, NT2RI2001230, NT2RI2001235, NT2RI2001244, NT2RI2001307, NT2RI2001342, NT2RI2001385,
NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001726, NT2RI2001769, NT2RI2001782, NT2RI2001836, NT2RI2001846, NT2RI2001866, NT2RI2001910, NT2RI2001952, NT2RI2002022, NT2RI2002041, NT2RI2002117, NT2RI2002120, NT2RI2002152, NT2RI2002153, NT2RI2002179, NT2RI2002243, NT2RI2002252, NT2RI2002260, NT2RI2002266, NT2RI2002270, NT2RI2002316, NT2RI2002334, NT2RI2002359, NT2RI2002447, NT2RI2002481, NT2RI2002507, NT2RI2002517, NT2RI2002530, NT2RI2002540, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819,
NT2RI2002822, NT2RI2002847, NT2RI2002852, NT2RI2002865, NT2RI2002923, NT2RI2002926, NT2RI2002958, NT2RI2002970, NT2RI2003011, NT2RI2003019, NT2RI2003051, NT2RI2003067, NT2RI2003184, NT2RI2003205, NT2RI2003222, NT2RI2003301, NT2RI2003317, NT2RI2003344, NT2RI2003360, NT2RI2003361, NT2RI2003383, NT2RI2003392, NT2RI2003399, NT2RI2003419, NT2RI2003487, NT2RI2003556, NT2RI2003655, NT2RI2003667, NT2RI2003678, NT2RI2003884, NT2RI2003973, NT2RI2004078, NT2RI2004079, NT2RI2004093, NT2RI2004136, NT2RI2004157, NT2RI2004188, NT2RI2004190, NT2RI2004209, NT2RI2004230, NT2RI2004284, NT2RI2004290, NT2RI2004304, NT2RI2004312, NT2RI2004381, NT2RI2004398, NT2RI2004410, NT2RI2004442, NT2RI2004468, NT2RI2004606,
NT2RI2004608, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005096, NT2RI2005115, NT2RI2005116, NT2RI2005130, NT2RI2005166, NT2RI2005239, NT2RI2005246, NT2RI2005315, NT2RI2005353, NT2RI2005358, NT2RI2005368, NT2RI2005382, NT2RI2005473, NT2RI2005520, NT2RI2005564, NT2RI2005579, NT2RI2005621, NT2RI2005628, NT2RI2005647, NT2RI2005670, NT2RI2005710, NT2RI2005713, NT2RI2005772, NT2RI2005811, NT2RI2005814, NT2RI2005816, NT2RI2005818, NT2RI2005966, NT2RI2006070, NT2RI2006071, NT2RI2006072, NT2RI2006081, NT2RI2006126, NT2RI2006127, NT2RI2006183, NT2RI2006257, NT2RI2006271,
NT2RI2006294, NT2RI2006345, NT2RI2006403, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006838, NT2RI2006853, NT2RI2006855, NT2RI2006943, NT2RI2006973, NT2RI2007046, NT2RI2007048, NT2RI2007084, NT2RI2007096, NT2RI2007116, NT2RI2007214, NT2RI2007277, NT2RI2007303, NT2RI2007334, NT2RI2007377, NT2RI2007386, NT2RI2007406, NT2RI2007439, NT2RI2007445, NT2RI2007468, NT2RI2007469, NT2RI2007498, NT2RI2007507, NT2RI2007572, NT2RI2007589, NT2RI2007593, NT2RI2007629, NT2RI2007723, NT2RI2007729,
NT2RI2007751, NT2RI2007827, NT2RI2007853, NT2RI2007877, NT2RI2007879, NT2RI2007884, NT2RI2007891, NT2RI2007956, NT2RI2007965, NT2RI2008007, NT2RI2008050, NT2RI2008188, NT2RI2008204, NT2RI2008221, NT2RI2008233, NT2RI2008336, NT2RI2008455, NT2RI2008502, NT2RI2008566, NT2RI2008656, NT2RI2008714, NT2RI2008724, NT2RI2008749, NT2RI2008801, NT2RI2008808, NT2RI2008812, NT2RI2008841, NT2RI2008942, NT2RI2008952, NT2RI2009005, NT2RI2009066, NT2RI2009083, NT2RI2009101, NT2RI2009103, NT2RI2009144, NT2RI2009151, NT2RI2009173, NT2RI2009215, NT2RI2009239, NT2RI2009259, NT2RI2009281, NT2RI2009289, NT2RI2009301, NT2RI2009402, NT2RI2009406, NT2RI2009517, NT2RI2009585, NT2RI2009595, NT2RI2009855, NT2RI2010283,
NT2RI2010508, NT2RI2010795, NT2RI2011422, NT2RI2011450, NT2RI2011683, NT2RI2011803, NT2RI2012350, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2015342, NT2RI2015533, NT2RI2016128, NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758,
NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284,
NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000017, NT2RP6000032, NT2RP6000043, NT2RP6000059, NT2RP6000060, NT2RP6000072, NT2RP6000077, NT2RP6000078, NT2RP6000085, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000069, NT2RP7000086,
NT2RP7000109, NT2RP7000112, NT2RP7000173, NT2RP7000238, NT2RP7000259, NT2RP7000271, NT2RP7000311, NT2RP7000359, NT2RP7000364, NT2RP7000380, NT2RP7000391, NT2RP7000425, NT2RP7000477, NT2RP7000579, NT2RP7000586, NT2RP7000600, NT2RP7000624, NT2RP7000812, NT2RP7000906, NT2RP7000926, NT2RP7001074, NT2RP7001080, NT2RP7001156, NT2RP7001231, NT2RP7001306, NT2RP7001319, NT2RP7001335, NT2RP7001364, NT2RP7001475, NT2RP7001532, NT2RP7001602, NT2RP7001678, NT2RP7001856, NT2RP7002064, NT2RP7002151, NT2RP7002163, NT2RP7002243, NT2RP7002282, NT2RP7002376, NT2RP7002478, NT2RP7002554, NT2RP7002594, NT2RP7002603, NT2RP7002650, NT2RP7002738, NT2RP7002779, NT2RP7002802, NT2RP7002829, NT2RP7002841, NT2RP7002875,
NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203, NT2RP7003261, NT2RP7003319, NT2RP7003439, NT2RP7003511, NT2RP7003629, NT2RP7003632, NT2RP7003647, NT2RP7003680, NT2RP7003688, NT2RP7003724, NT2RP7004027, NT2RP7004080, NT2RP7004114, NT2RP7004123, NT2RP7004173, NT2RP7004196, NT2RP7004204, NT2RP7004233, NT2RP7004260, NT2RP7004352, NT2RP7004358, NT2RP7004373, NT2RP7004396, NT2RP7004428, NT2RP7004481, NT2RP7004541, NT2RP7004559, NT2RP7004641, NT2RP7004656, NT2RP7004687, NT2RP7004722, NT2RP7004728, NT2RP7004766, NT2RP7004790, NT2RP7004884, NT2RP7004911, NT2RP7004915, NT2RP7004946,
NT2RP7004961, NT2RP7004964, NT2RP7004975, NT2RP7005118, NT2RP7005205, NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005750, NT2RP7005846, NT2RP7006033, NT2RP7006141, NT2RP7006160, NT2RP7006162, NT2RP7006188, NT2RP7006223, NT2RP7006263, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006619, NT2RP7006621, NT2RP7006636, NT2RP7006701, NT2RP7006853, NT2RP7006886, NT2RP7006887, NT2RP7006980, NT2RP7006986, NT2RP7006995, NT2RP7007114, NT2RP7007221, NT2RP7007226, NT2RP7007252, NT2RP7007269, NT2RP7007310,
NT2RP7007359, NT2RP7007381, NT2RP7007387, NT2RP7007406, NT2RP7007480, NT2RP7007504, NT2RP7007530, NT2RP7007537, NT2RP7007580, NT2RP7007594, NT2RP7007610, NT2RP7007617, NT2RP7007643, NT2RP7007766, NT2RP7007842, NT2RP7007925, NT2RP7007930, NT2RP7007975, NT2RP7008013, NT2RP7008015, NT2RP7008133, NT2RP7008137, NT2RP7008142, NT2RP7008144, NT2RP7008161, NT2RP7008167, NT2RP7008190, NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008657, NT2RP7008714, NT2RP7008720, NT2RP7008855, NT2RP7008863, NT2RP7009012, NT2RP7009019, NT2RP7009030, NT2RP7009097, NT2RP7009108, NT2RP7009147,
NT2RP7009168, NT2RP7009215, NT2RP7009225, NT2RP7009236, NT2RP7009253, NT2RP7009259, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009482, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7013002, NT2RP7013374, NT2RP7013499, NT2RP7013573, NT2RP7013729, NT2RP7013764, NT2RP7013795, NT2RP7013999,
NT2RP7014178, NT2RP7014348, NT2RP7014420, NT2RP7014583, NT2RP7014721, NT2RP7014743, NT2RP7014778, NT2RP7014906, NT2RP7014910, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137,
NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8008057, NT2RP8009248, NTONG1000006, NTONG1000033, NTONG1000047, NTONG1000052, NTONG1000075, NTONG1000087, NTONG1000098, NTONG1000123, NTONG1000130, NTONG1000161, NTONG1000172, NTONG1000213, NTONG1000214, NTONG1000246, NTONG1000247, NTONG1000264, NTONG2000040, NTONG2000107, NTONG2000231, NTONG2000334, NTONG2000363, NTONG2000413, NTONG2000492, NTONG2000499, NTONG2000531, NTONG2000583, NTONG2000800, NTONG2000858,
NTONG2000876, NTONG2000966, NTONG2000977, NTONG2000985, NTONG2001014, NTONG2001079, NTONG2001137, NTONG2001157, NTONG2001173, NTONG2001220, NTONG2001222, NTONG2001362, NTONG2001428, NTONG2001532, NTONG2001550, NTONG2001587, NTONG2001612, NTONG2001619, NTONG2001642, NTONG2001657, NTONG2001676, NTONG2002278, NTONG2002582, NTONG2002807, NTONG2002970, NTONG2002985, NTONG2003158, NTONG2003210, NTONG2003316, NTONG2003409, NTONG2003454, NTONG2003515, NTONG2003805, NTONG2003852, NTONG2003897, NTONG2003928, NTONG2004095, NTONG2004308, NTONG2004521, NTONG2004614, NTONG2004690, NTONG2004806, NTONG2004829, NTONG2004844, NTONG2004918, NTONG2004991, NTONG2005062, NTONG2005086, NTONG2005137, NTONG2005153,
NTONG2005265, NTONG2005277, NTONG2005363, NTONG2005373, NTONG2005391, NTONG2005480, NTONG2005497, NTONG2005520, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030,
OCBBF1000042, OCBBF1000049, OCBBF1000054, OCBBF1000067, OCBBF1000080, OCBBF1000085, OCBBF1000091, OCBBF1000104, OCBBF1000114, OCBBF1000118, OCBBF1000119, OCBBF1000122, OCBBF1000130, OCBBF1000138, OCBBF1000145, OCBBF1000146, OCBBF1000153, OCBBF1000185, OCBBF1000188, OCBBF1000192, OCBBF1000254, OCBBF1000315, OCBBF2000013, OCBBF2000015, OCBBF2000126, OCBBF2000178, OCBBF2000231, OCBBF2000277, OCBBF2000287, OCBBF2000323, OCBBF2000452, OCBBF2000467, OCBBF2000522, OCBBF2000677, OCBBF2000703, OCBBF2000719, OCBBF2000824, OCBBF2000831, OCBBF2000846, OCBBF2000904, OCBBF2000986, OCBBF2000998, OCBBF2001075, OCBBF2001101, OCBBF2001124, OCBBF2001140, OCBBF2001166, OCBBF2001176, OCBBF2001186, OCBBF2001196,
OCBBF2001210, OCBBF2001252, OCBBF2001307, OCBBF2001323, OCBBF2001389, OCBBF2001402, OCBBF2001408, OCBBF2001416, OCBBF2001473, OCBBF2001494, OCBBF2001527, OCBBF2001528, OCBBF2001586, OCBBF2001639, OCBBF2001681, OCBBF2001687, OCBBF2001706, OCBBF2001749, OCBBF2001794, OCBBF2001910, OCBBF2001931, OCBBF2001961, OCBBF2001983, OCBBF2002015, OCBBF2002083, OCBBF2002086, OCBBF2002124, OCBBF2002161, OCBBF2002290, OCBBF2002357, OCBBF2002376, OCBBF2002615, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038,
OCBBF2004104, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624,
OCBBF2006639, OCBBF2006660, OCBBF2006764, OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007354, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007892, OCBBF2007931, OCBBF2007946, OCBBF2008005, OCBBF2008041, OCBBF2008116, OCBBF2008138, OCBBF2008283, OCBBF2008330, OCBBF2008340, OCBBF2008366, OCBBF2008466, OCBBF2008511, OCBBF2008520, OCBBF2008569, OCBBF2008586, OCBBF2008640, OCBBF2008691, OCBBF2008701, OCBBF2008724,
OCBBF2008760, OCBBF2008768, OCBBF2008770, OCBBF2008775, OCBBF2008790, OCBBF2008814, OCBBF2008822, OCBBF2009095, OCBBF2009115, OCBBF2009242, OCBBF2009301, OCBBF2009352, OCBBF2009391, OCBBF2009536, OCBBF2009571, OCBBF2009583, OCBBF2009603, OCBBF2009772, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010841, OCBBF2010843, OCBBF2010858, OCBBF2010863, OCBBF2010871, OCBBF2010931, OCBBF2010945, OCBBF2010978, OCBBF2010989, OCBBF2011073, OCBBF2011137, OCBBF2011160, OCBBF2011177, OCBBF2011228, OCBBF2011232, OCBBF2011283,
OCBBF2011311, OCBBF2011625, OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012553, OCBBF2012678, OCBBF2012704, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789,
OCBBF2013843, OCBBF2013883, OCBBF2013926, OCBBF2014052, OCBBF2014064, OCBBF2014089, OCBBF2014322, OCBBF2014386, OCBBF2014541, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2016928, OCBBF2017035, OCBBF2017055, OCBBF2017069, OCBBF2017306, OCBBF2017325, OCBBF2017398, OCBBF2017458,
OCBBF2017489, OCBBF2017516, OCBBF2017665, OCBBF2017754, OCBBF2017791, OCBBF2017815, OCBBF2017882, OCBBF2017888, OCBBF2017893, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018827, OCBBF2018828, OCBBF2018873, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833,
OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927,
OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000323, OCBBF3000372, OCBBF3000380,
OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000174, PEBLM1000180, PEBLM2000030,
PEBLM2000112, PEBLM2000126, PEBLM2000147, PEBLM2000170, PEBLM2000180, PEBLM2000213, PEBLM2000222, PEBLM2000248, PEBLM2000267, PEBLM2000270, PEBLM2000308, PEBLM2000321, PEBLM2000326, PEBLM2000333, PEBLM2000338, PEBLM2000395, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283,
PEBLM2006298, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131,
PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000105, PLACE5000113, PLACE5000115, PLACE5000116, PLACE5000129, PLACE5000139, PLACE5000153, PLACE5000159, PLACE5000171, PLACE5000172, PLACE5000178, PLACE5000245, PLACE5000260, PLACE5000297, PLACE5000372, PLACE5000492,
PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000314, PLACE6000348, PLACE6000371, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001118, PLACE6001153, PLACE6001185, PLACE6001262, PLACE6001294, PLACE6001443, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002102, PLACE6002157, PLACE6002312, PLACE6002323,
PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004336, PLACE6004368, PLACE6004380, PLACE6004396, PLACE6004397, PLACE6004454, PLACE6004464, PLACE6004491, PLACE6004663, PLACE6004687, PLACE6004738, PLACE6004931, PLACE6004966, PLACE6004993, PLACE6005007, PLACE6005029, PLACE6005108,
PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006077, PLACE6006137, PLACE6006158, PLACE6006217, PLACE6006222, PLACE6006266, PLACE6006287, PLACE6006394, PLACE6006418, PLACE6006474, PLACE6006549, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007482, PLACE6007687, PLACE6007787, PLACE6007798, PLACE6007807, PLACE6007925, PLACE6008036, PLACE6008094, PLACE6008126, PLACE6008285,
PLACE6008444, PLACE6008453, PLACE6008640, PLACE6008750, PLACE6008768, PLACE6008775, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010077, PLACE6010463, PLACE6010485, PLACE6010568, PLACE6010701, PLACE6010704, PLACE6010732, PLACE6010765, PLACE6010925, PLACE6010936, PLACE6010991, PLACE6011057, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012108, PLACE6012168, PLACE6012297,
PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE-6016254, PLACE6016942, PLACE6016984, PLACE6017002, PLACE6017431, PLACE6017564, PLACE6017626, PLACE6017701, PLACE6017714, PLACE6017788, PLACE6018031, PLACE6018107, PLACE6018187, PLACE6018235, PLACE6018287, PLACE6018441, PLACE6018497, PLACE6018769,
PLACE6018834, PLACE6018843, PLACE6018863, PLACE6018938, PLACE6019195, PLACE6019542, PLACE6019600, PLACE6019674, PLACE6019676, PLACE6019701, PLACE6019932, PLACE6020031, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563,
PROST1000033, PROST1000039, PROST1000065, PROST1000083, PROST1000085, PROST1000110, PROST1000120, PROST1000123, PROST1000136, PROST1000144, PROST1000145, PROST1000152, PROST1000167, PROST1000184, PROST1000215, PROST1000217, PROST1000220, PROST1000226, PROST1000246, PROST1000262, PROST1000272, PROST1000298, PROST1000322, PROST1000334, PROST1000362, PROST1000377, PROST1000383, PROST1000412, PROST1000419, PROST1000451, PROST1000480, PROST1000485, PROST1000493, PROST1000526, PROST1000527, PROST1000528, PROST1000559, PROST1000575, PROST2000036, PROST2000053, PROST2000067, PROST2000079, PROST2000105, PROST2000143, PROST2000162, PROST2000165, PROST2000176, PROST2000203, PROST2000206, PROST2000212,
PROST2000241, PROST2000267, PROST2000273, PROST2000274, PROST2000277, PROST2000284, PROST2000325, PROST2000337, PROST2000432; PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001180, PROST2001213, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002101, PROST2002162, PROST2002212, PROST2002227, PROST2002338, PROST2002425, PROST2002488,
PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303, PROST2003324, PROST2003340, PROST2003396, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004258, PROST2004270, PROST2004332, PROST2004481, PROST2004526,
PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426, PROST2005466, PROST2005604, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006060, PROST2006196, PROST2006201, PROST2006260, PROST2006282, PROST2006343, PROST2006450, PROST2006491, PROST2006510, PROST2006536, PROST2006579, PROST2006688, PROST2006737, PROST2006766, PROST2006782, PROST2006933, PROST2006988, PROST2007122, PROST2007200,
PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008271, PROST2008360, PROST2008447, PROST2008468, PROST2008472, PROST2008516, PROST2008724, PROST2008770, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046,
PROST2010219, PROST2010250, PROST2010318, PROST2010326, PROST2010382, PROST2010400, PROST2010545, PROST2010579, PROST2010606, PROST2010885, PROST2011012, PROST2011038, PROST2011105, PROST2011297, PROST2011410, PROST2011439, PROST2011482, PROST2011577, PROST2011631, PROST2011660, PROST2012005, PROST2012007, PROST2012016, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012888, PROST2012890, PROST2013032, PROST2013121, PROST2013260, PROST2013327, PROST2013531, PROST2013605, PROST2013627, PROST2013873, PROST2013880, PROST2014422, PROST2014585, PROST2014601, PROST2014659,
PROST2014862, PROST2014916, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017367, PROST2017413, PROST2017441, PROST2017529, PROST2017578, PROST2017612, PROST2017617, PROST2017700,
PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000065, PUAEN1000081, PUAEN1000085, PUAEN1000087, PUAEN1000121, PUAEN1000147, PUAEN1000161, PUAEN1000164, PUAEN1000175, PUAEN1000239, PUAEN1000275, PUAEN1000276, PUAEN1000288, PUAEN1000322, PUAEN2000080, PUAEN2000152, PUAEN2000175, PUAEN2000247, PUAEN2000312, PUAEN2000394, PUAEN2000535, PUAEN2000594, PUAEN2000684, PUAEN2000942,
PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009655,
PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000022, SKMUS1000030, SKMUS1000067, SKMUS1000102, SKMUS1000104, SKMUS1000110, SKMUS1000118, SKMUS1000124, SKMUS1000129, SKMUS1000176, SKMUS1000177, SKMUS2000020, SKMUS2000061, SKMUS2000074, SKMUS2000078, SKMUS2000117, SKMUS2000137, SKMUS2000243, SKMUS2000271, SKMUS2000287, SKMUS2000317, SKMUS2000330, SKMUS2000339,
SKMUS2000343, SKMUS2000365, SKMUS2000390, SKMUS2000416, SKMUS2000467, SKMUS2000468, SKMUS2000484, SKMUS2000515, SKMUS2000690, SKMUS2000701, SKMUS2000705, SKMUS2000710, SKMUS2000724, SKMUS2000726, SKMUS2000774, SKMUS2000780, SKMUS2000847, SKMUS2000863, SKMUS2000894, SKMUS2000898, SKMUS2000902, SKMUS2000933, SKMUS2000945, SKMUS2000954, SKMUS2000961, SKMUS2000973, SKMUS2000980, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001454, SKMUS2001492, SKMUS2001528, SKMUS2001543, SKMUS2001580, SKMUS2001585,
SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607,
SKMUS2009190, SKMUS2009232, SKMUS2009479, SKMUS2009557, SKNMC1000110, SKNMC1000123, SKNMC1000124, SKNMC1000159, SKNMC1000229, SKNMC1000251, SKNMC1000264, SKNMC2000065, SKNMC2000097, SKNMC2000256, SKNMC2000305, SKNMC2000322, SKNMC2000356, SKNMC2000374, SKNMC2000583, SKNMC2000593, SKNMC2000612, SKNMC2000622, SKNMC2000877, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000101, SKNSH1000174,
SKNSH1000301, SKNSH1000308, SKNSH1000416, SKNSH2000051, SKNSH2000101, SKNSH2000151, SKNSH2000163, SKNSH2000250, SKNSH2000319, SKNSH2000347, SKNSH2000482, SKNSH2000516, SKNSH2000550, SKNSH2000819, SKNSH2000971, SKNSH2001222, SKNSH2001875, SKNSH2002054, SKNSH2002325, SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015,
SMINT1000012, SMINT1000016, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000137, SMINT2000007, SMINT2000018, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000400, SMINT2000454, SMINT2000518, SMINT2000529, SMINT2000530, SMINT2000537, SMINT2000538, SMINT2000541, SMINT2000545, SMINT2000558, SMINT2000562, SMINT2000568, SMINT2000602, SMINT2000609, SMINT2000629, SMINT2000659, SMINT2000747, SMINT2000811,
SMINT2000824, SMINT2000933, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002159, SMINT2002202, SMINT2002210, SMINT2002224, SMINT2002281, SMINT2002311, SMINT2002314, SMINT2002328, SMINT2002414, SMINT2002457, SMINT2002620, SMINT2002743, SMINT2002778, SMINT2002880, SMINT2002882, SMINT2002884, SMINT2002899, SMINT2002974, SMINT2002976, SMINT2003003, SMINT2003128, SMINT2003150, SMINT2003317, SMINT2003340, SMINT2003386, SMINT2003423, SMINT2003505, SMINT2003551, SMINT2003569,
SMINT2003641, SMINT2003644, SMINT2003866, SMINT2004023, SMINT2004037, SMINT2004047, SMINT2004086, SMINT2004280, SMINT2004299, SMINT2004339, SMINT2004350, SMINT2004414, SMINT2004473, SMINT2004547, SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140,
SMINT2007187, SMINT2007219, SMINT2007294, SMINT2007365, SMINT2007391, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921, SMINT2008960, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529,
SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012179, SMINT2012195, SMINT2012285, SMINT2012501, SMINT2012734, SMINT2012793, SMINT2013032, SMINT2013129, SMINT2013170, SMINT2013181, SMINT2013228, SMINT2013613,
SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017659, SMINT2017736, SMINT2017781, SMINT2017855, SMINT2017884, SMINT2017964, SMINT2017974, SMINT2018343, SMINT2018592, SMINT2018681, SMINT2019017, SMINT2019105, SMINT2019142,
SMINT2019153, SMINT2019200, SPLEN1000013, SPLEN1000041, SPLEN1000049, SPLEN1000056, SPLEN1000087, SPLEN1000089, SPLEN1000091, SPLEN1000106, SPLEN1000134, SPLEN1000139, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000255, SPLEN2000267, SPLEN2000270, SPLEN2000307, SPLEN2000310, SPLEN2000341, SPLEN2000350, SPLEN2000357, SPLEN2000364, SPLEN2000402, SPLEN2000414, SPLEN2000420, SPLEN2000443, SPLEN2000448, SPLEN2000450,
SPLEN2000464, SPLEN2000496, SPLEN2000505, SPLEN2000515, SPLEN2000516, SPLEN2000537, SPLEN2000541, SPLEN2000607, SPLEN2000630, SPLEN2000695, SPLEN2000698, SPLEN2000839, SPLEN2001135, SPLEN2001141, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451,
SPLEN2002462, SPLEN2002463, SPLEN2002467, SPLEN2002493, SPLEN2002662, SPLEN2002695, SPLEN2002707, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004343, SPLEN2004346, SPLEN2004368, SPLEN2004555, SPLEN2004611, SPLEN2004662, SPLEN2004773, SPLEN2004844, SPLEN2004880, SPLEN2004984, SPLEN2005009, SPLEN2005227, SPLEN2005272, SPLEN2005342, SPLEN2005416, SPLEN2005429, SPLEN2005450, SPLEN2005474, SPLEN2005560, SPLEN2005727, SPLEN2005783, SPLEN2005790, SPLEN2005818, SPLEN2005838, SPLEN2005869,
SPLEN2005939, SPLEN2006122, SPLEN2006133, SPLEN2006143, SPLEN2006232, SPLEN2006268, SPLEN2006283, SPLEN2006305, SPLEN2006374, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496, SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009555,
SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010195, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981,
SPLEN2012115, SPLEN2012175, SPLEN2012179, SPLEN2012185, SPLEN2012234, SPLEN2012453, SPLEN2012523, SPLEN2012524, SPLEN2012525, SPLEN2012571, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013644, SPLEN2013648, SPLEN2013670, SPLEN2013673, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543,
SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015310, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016480, SPLEN2016531, SPLEN2016533,
SPLEN2016541, SPLEN2016552, SPLEN2016554, SPLEN2016602, SPLEN2016627, SPLEN2016678, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018364, SPLEN2018395, SPLEN2018643, SPLEN2018683, SPLEN2018732, SPLEN2018749, SPLEN2018933,
SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383,
SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027113, SPLEN2027157,
SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028466, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031547, SPLEN2031674, SPLEN2031724, SPLEN2031780, SPLEN2032036, SPLEN2032112, SPLEN2032154, SPLEN2032321, SPLEN2032356, SPLEN2032677, SPLEN2032813, SPLEN2032924, SPLEN2033098,
SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039379, SPLEN2039672, SPLEN2039697, SPLEN2039845,
SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000190, STOMA1000191, STOMA2000032, STOMA2000072, STOMA2000088, STOMA2000104, STOMA2000148, STOMA2000168, STOMA2000183, STOMA2000188, STOMA2000257, STOMA2000279, STOMA2000289, STOMA2000383, STOMA2000386, STOMA2000478, STOMA2000482, STOMA2000529, STOMA2000539, STOMA2000567, STOMA2000640, STOMA2000678,
STOMA2000686, STOMA2000888, STOMA2001025, STOMA2001389, STOMA2002052, STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253,
STOMA2009256, STOMA2009289, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001239, SYNOV2001251, SYNOV2001262, SYNOV2001300, SYNOV2001356, SYNOV2001374, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673,
SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570,
SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007671, SYNOV4007711, SYNOV4008245, SYNOV4008336, SYNOV4009129, SYNOV4009139, SYNOV4009298, SYNOV4009575, T1ESE2000116, T1ESE2000904, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543,
TBAES2006568, TBAES2006881, TBAES2007379, TBAES2007428, TBAES2007481, TBAES2007504, TBAES2007548, TBAES2007697, TBAES2007862, TBAES2007964, TBAES2008133, TBAES2009387, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005,
TESOP2002110, TESOP2002150, TESOP2002273, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247,
TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154,
TESTI2000172, TESTI2000177, TESTI2000179, TESTI2000184, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951,
TESTI2000970, TESTI2000981, TESTI2001008, TESTI2001049, TESTI2001082, TESTI2001120, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001862, TESTI2001869, TESTI2001879, TESTI2001894,
TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707,
TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003327, TESTI2003330, TESTI2003347,
TESTI2003354, TESTI2003356, TESTI2003372, TESTI2003376, TESTI2003418, TESTI2003419, TESTI2003432, TESTI2003454, TESTI2003475, TESTI2003476, TESTI2003498, TESTI2003512, TESTI2003539, TESTI2003541, TESTI2003545, TESTI2003551, TESTI2003574, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004031, TESTI2004073, TESTI2004080, TESTI2004085, TESTI2004089, TESTI2004111, TESTI2004119, TESTI2004122, TESTI2004153,
TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994,
TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005767, TESTI2005775, TESTI2005784,
TESTI2005788, TESTI2005827, TESTI2005835, TESTI2005860, TESTI2005892, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465,
TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007311, TESTI2007320, TESTI2007346, TESTI2007358, TESTI2007402, TESTI2007405, TESTI2007407, TESTI2007422,
TESTI2007452, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2007998, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405,
TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009520, TESTI2009544, TESTI2009545, TESTI2009551, TESTI2009577,
TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011294, TESTI2011314, TESTI2011315, TESTI2011394, TESTI2011407, TESTI2011448, TESTI2011480, TESTI2011549, TESTI2011575, TESTI2011605, TESTI2011612,
TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257,
TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988,
TESTI2015042, TESTI2015092, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015626, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896,
TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018462, TESTI2018475, TESTI2018482, TESTI2018521, TESTI2018565, TESTI2018581,
TESTI2018611, TESTI2018687, TESTI2018697, TESTI2018838, TESTI2018867, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426,
TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021124, TESTI2021138, TESTI2021315, TESTI2021358, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323,
TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498,
TESTI2024560, TESTI2024567, TESTI2024586, TESTI2024598, TESTI2024641, TESTI2024648, TESTI2024718, TESTI2024744, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196,
TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736,
TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754,
TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718,
TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081,
TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707,
TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958,
TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046456, TESTI2046535, TESTI2046536, TESTI2046552, TESTI2046569,
TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767,
TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600,
TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319,
TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441,
TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034,
TESTI4009123, TESTI4009160, TESTI4009215, TESTI4009283, TESTI4009286, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258,
TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977,
TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555,
TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344,
TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904,
TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498,
TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037156, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800,
TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168,
TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000016, THYMU1000017, THYMU1000025, THYMU1000029, THYMU1000032, THYMU1000033, THYMU1000041, THYMU1000060, THYMU1000074, THYMU1000083, THYMU1000098, THYMU1000103, THYMU1000105, THYMU1000136, THYMU1000142, THYMU1000144,
THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000331, THYMU1000336, THYMU1000359, THYMU1000366, THYMU1000374, THYMU1000382, THYMU1000393, THYMU1000394, THYMU1000399, THYMU1000426, THYMU1000428, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231,
THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879, THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556,
THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002583, THYMU2002695, THYMU2002745, THYMU2002789, THYMU2002792, THYMU2002815, THYMU2002841, THYMU2002852, THYMU2002910, THYMU2002950, THYMU2002983, THYMU2003012, THYMU2003069, THYMU2003107, THYMU2003124, THYMU2003166, THYMU2003232, THYMU2003242, THYMU2003249, THYMU2003270,
THYMU2003282, THYMU2003287, THYMU2003336, THYMU2003366, THYMU2003397, THYMU2003419, THYMU2003440, THYMU2003446, THYMU2003470, THYMU2003479, THYMU2003499, THYMU2003542, THYMU2003632, THYMU2003650, THYMU2003700, THYMU2003760, THYMU2003803, THYMU2003855, THYMU2003891, THYMU2003907, THYMU2003932, THYMU2003981, THYMU2004003, THYMU2004008, THYMU2004042, THYMU2004108, THYMU2004111, THYMU2004123, THYMU2004139, THYMU2004152, THYMU2004169, THYMU2004320, THYMU2004336, THYMU2004344, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916,
THYMU2004986, THYMU2005001, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005507, THYMU2005545, THYMU2005546, THYMU2005574, THYMU2005576, THYMU2005748, THYMU2005759, THYMU2005807, THYMU2005815, THYMU2005855, THYMU2005881, THYMU2005948, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006505, THYMU2006610, THYMU2006666, THYMU2006712, THYMU2006813,
THYMU2006913, THYMU2006946, THYMU2006965, THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282,
THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009658, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637,
THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012492, THYMU2012507, THYMU2012565, THYMU2012574, THYMU2012631,
THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757, THYMU2013779, THYMU2013863, THYMU2013916, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441,
THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721,
THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042,
THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796,
THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101,
THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029,
THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763,
THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081,
THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856,
THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706,
THYMU3030710, THYMU3030752, THYMU3031146, THYMU3031402, THYMU3031610, THYMU3031612, THYMU3031868, THYMU3031878, THYMU3032032, THYMU3032798, THYMU3032867, THYMU3033402, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970,
THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542,
THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078, TKIDN2003366, TKIDN2003396, TKIDN2003413, TKIDN2003597, TKIDN2003601, TKIDN2004386, TKIDN2004458, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641,
TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015423, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200,
TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800,
TRACH1000011, TRACH1000018, TRACH1000030, TRACH1000038, TRACH1000057, TRACH1000063, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000045, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000461, TRACH2000472, TRACH2000496, TRACH2000497, TRACH2000502, TRACH2000522, TRACH2000559,
TRACH2000660, TRACH2000665, TRACH2000675, TRACH2000677, TRACH2000702, TRACH2000703, TRACH2000767, TRACH2000775, TRACH2000780, TRACH2000807, TRACH2000862, TRACH2000873, TRACH2000894, TRACH2000898, TRACH2000920, TRACH2000923, TRACH2000926, TRACH2000944, TRACH2000959, TRACH2000972, TRACH2000981, TRACH2000986, TRACH2001037, TRACH2001060, TRACH2001085, TRACH2001101, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001463, TRACH2001474, TRACH2001478, TRACH2001523,
TRACH2001525, TRACH2001532, TRACH2001549, TRACH2001592, TRACH2001596, TRACH2001612, TRACH2001621, TRACH2001646, TRACH2001651, TRACH2001684, TRACH2001810, TRACH2001898, TRACH2001933, TRACH2001968, TRACH2001996, TRACH2002043, TRACH2002054, TRACH2002092, TRACH2002100, TRACH2002138, TRACH2002333, TRACH2002537, TRACH2002664, TRACH2002784, TRACH2002803, TRACH2002840, TRACH2002847, TRACH2002853, TRACH2002862, TRACH2002954, TRACH2002960, TRACH2002988, TRACH2003070, TRACH2003272, TRACH2003323, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292,
TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894,
TRACH2007969, TRACH2007988, TRACH2008081, TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771,
TRACH2010824, TRACH2010883, TRACH2010965, TRACH2010974, TRACH2011057, TRACH2011093, TRACH2011290, TRACH2011293, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014077, TRACH2014082, TRACH2014268, TRACH2014284, TRACH2014337, TRACH2014356,
TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015823, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980,
TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299,
TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892,
TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840,
TRACH3004931, TRACH3004934, TRACH3005072, TRACH3005085, TRACH3005102, TRACH3005173, TRACH3005191, TRACH3005211, TRACH3005274, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007541, TRACH3007595, TRACH3007625, TRACH3007627, TRACH3007689, TRACH3007766, TRACH3007866, TRACH3007995, TRACH3008042, TRACH3008061, TRACH3008093,
TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043,
TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058,
TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428,
TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400,
TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063,
TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143,
UMVEN1000156, UMVEN2000046, UMVEN2000069, UMVEN2000121, UMVEN2000152, UMVEN2000354, UMVEN2000453, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000465, UTERU2000517,
UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002332, UTERU2002410, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135,
UTERU2003321, UTERU2003399, UTERU2003411, UTERU2003456, UTERU2003577, UTERU2003704, UTERU2003926, UTERU2003973, UTERU2004015, UTERU2004039, UTERU2004061, UTERU2004073, UTERU2004163, UTERU2004197, UTERU2004299, UTERU2004461, UTERU2004520, UTERU2004564, UTERU2004664, UTERU2004688, UTERU2004698, UTERU2004807, UTERU2004861, UTERU2004929, UTERU2005004, UTERU2005050, UTERU2005069, UTERU2005074, UTERU2005179, UTERU2005346, UTERU2005354, UTERU2005446, UTERU2005449, UTERU2005450, UTERU2005533, UTERU2005593, UTERU2005601, UTERU2005621, UTERU2005664, UTERU2005822, UTERU2005903, UTERU2005905, UTERU2006103, UTERU2006115, UTERU2006137, UTERU2006182, UTERU2006412, UTERU2006429, UTERU2006486, UTERU2006524,
UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008939, UTERU2008962, UTERU2009094, UTERU2009120, UTERU2009131, UTERU2009147, UTERU2009206, UTERU2009283, UTERU2009414, UTERU2009435, UTERU2009483, UTERU2009510, UTERU2009538,
UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012252, UTERU2012286, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786,
UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016799, UTERU2016822, UTERU2016896, UTERU2016902,
UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292,
UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742,
UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423,
UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660,
UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690,
UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172,
UTERU3018255, UTERU3018616, UTERU3018711, UTERU3019078

Of the 17,176 genes including the cDNAs of the present invention, genes, whose expression levels were high enough to detect in each library of HELAC, HHDPC, HLUNG, HSYRA, IMR32, JCMLC, and KIDNE, were analyzed for the expression frequencies. The analysis results are described below. Each is a relative value, where the expression level over all tissues is taken as 100. The larger this value, the greater the level of expression.

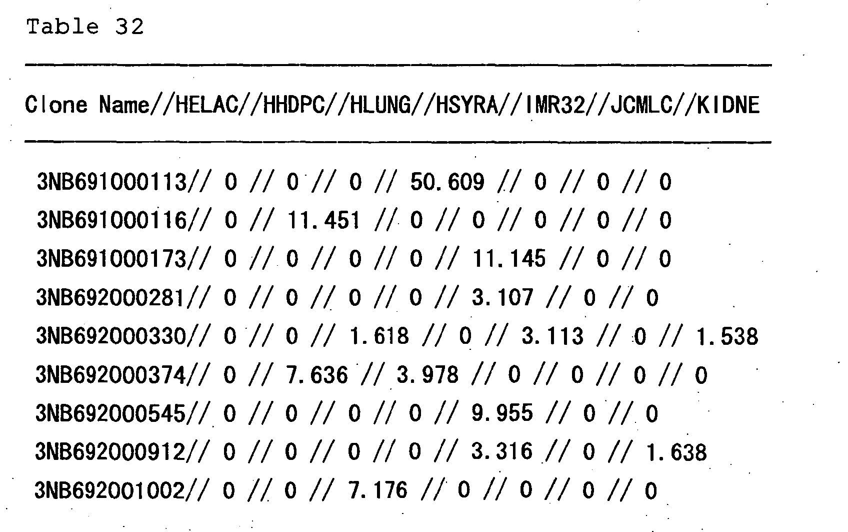

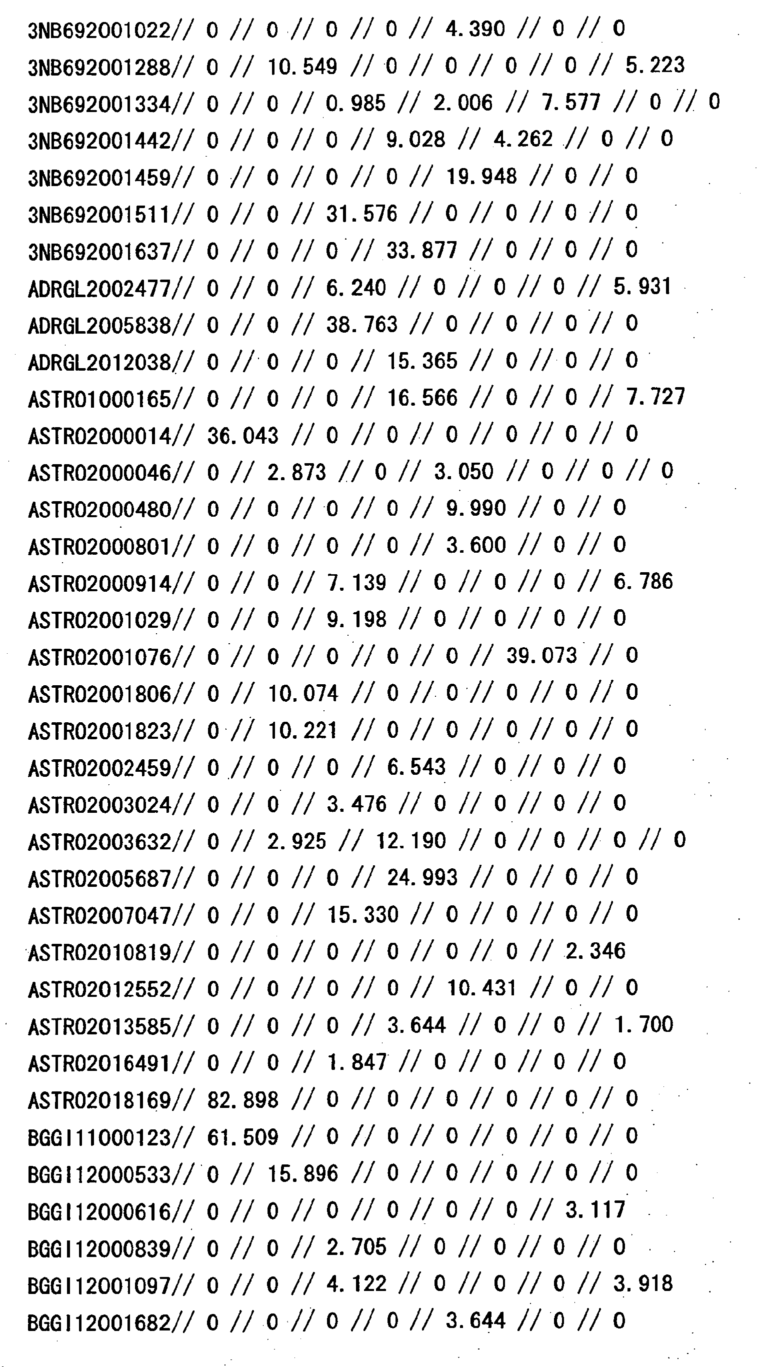

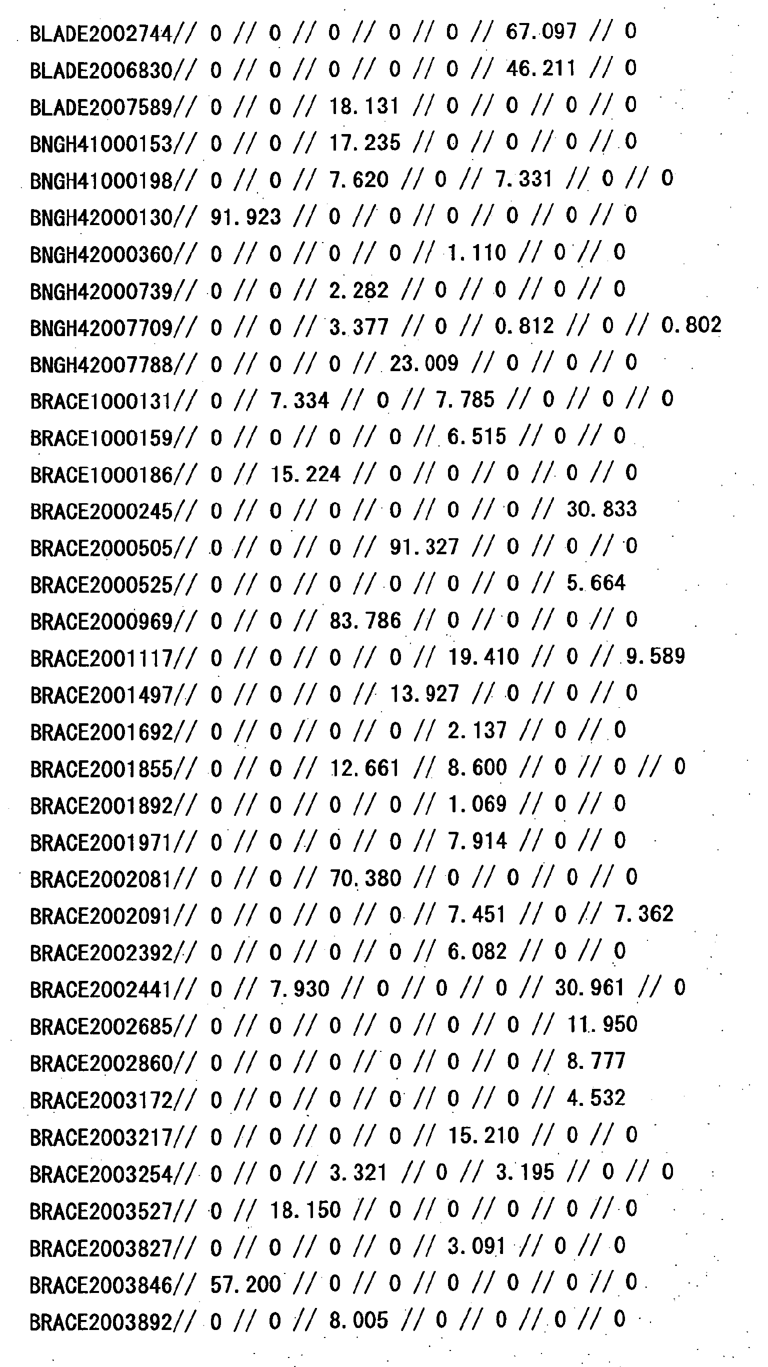

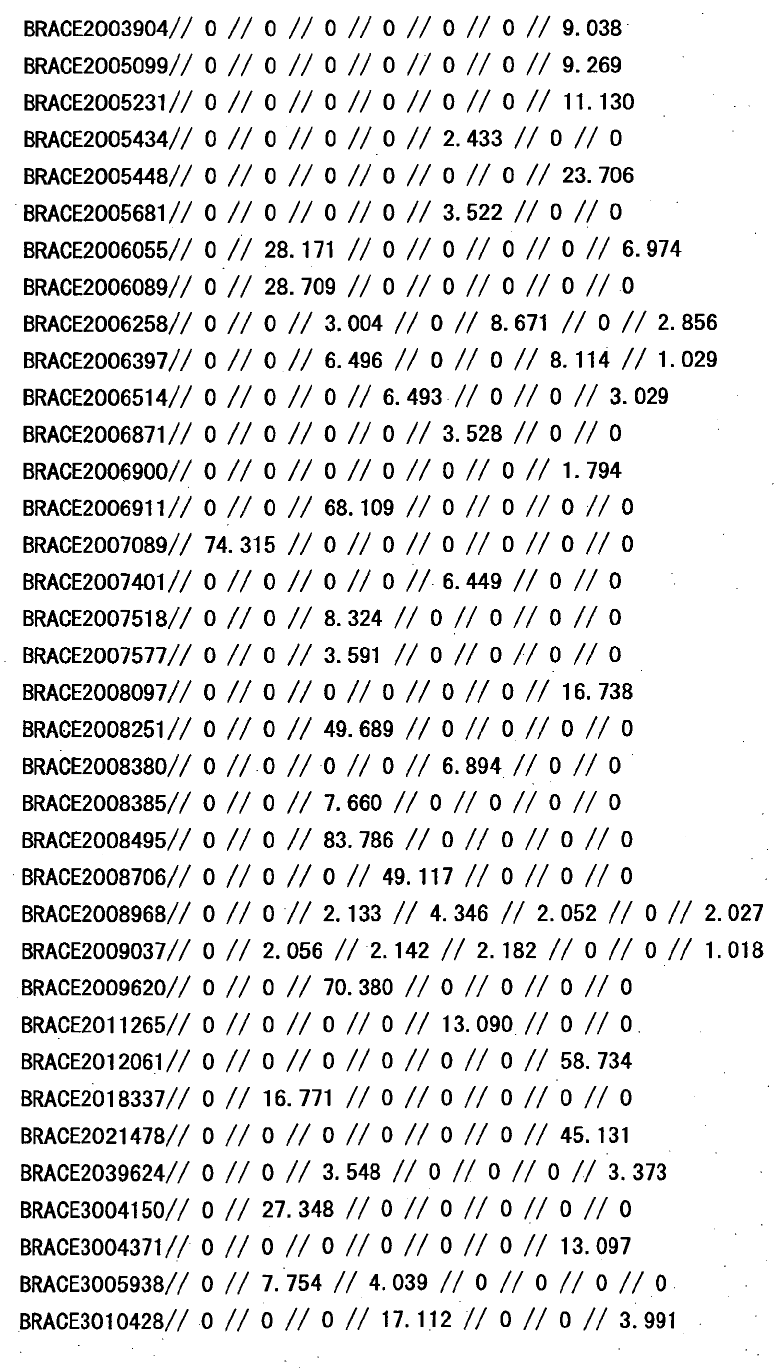

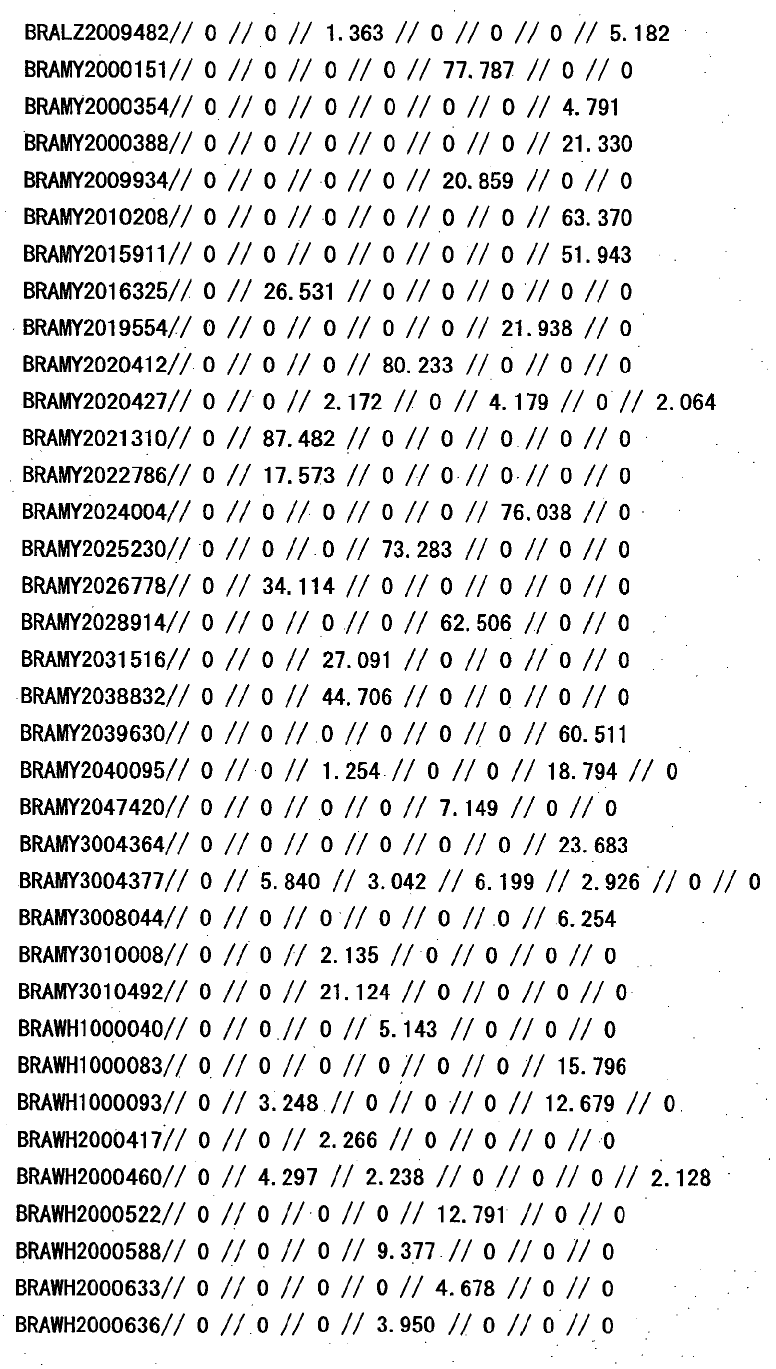

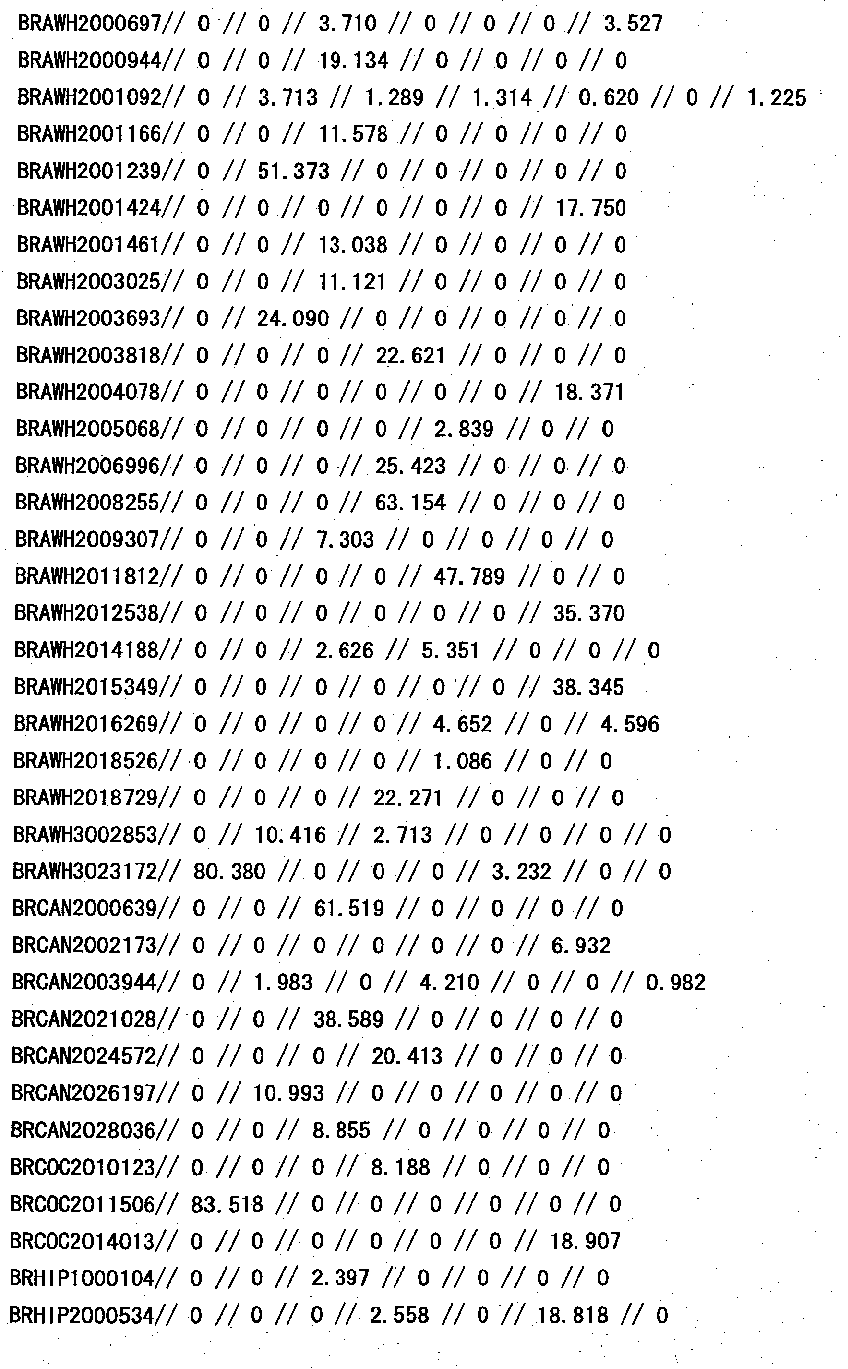

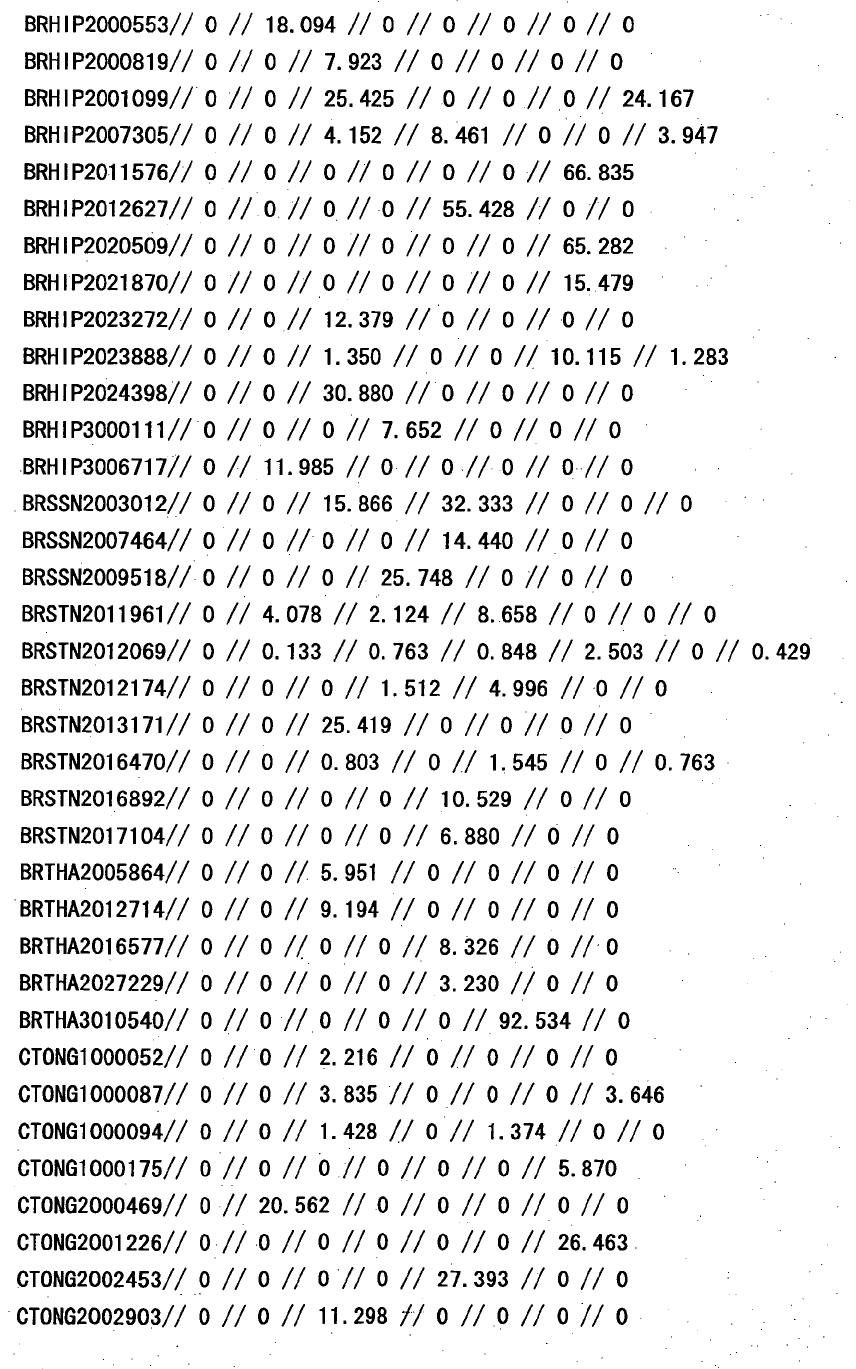

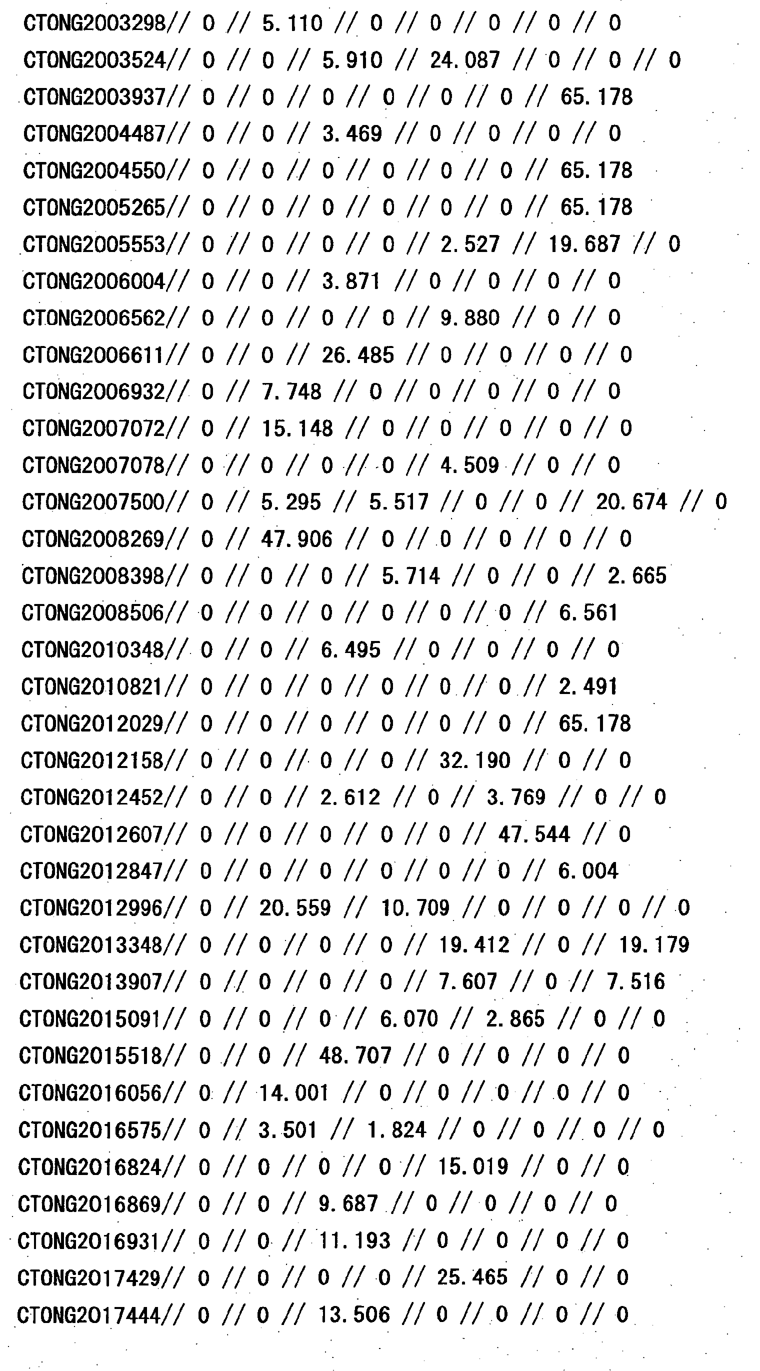

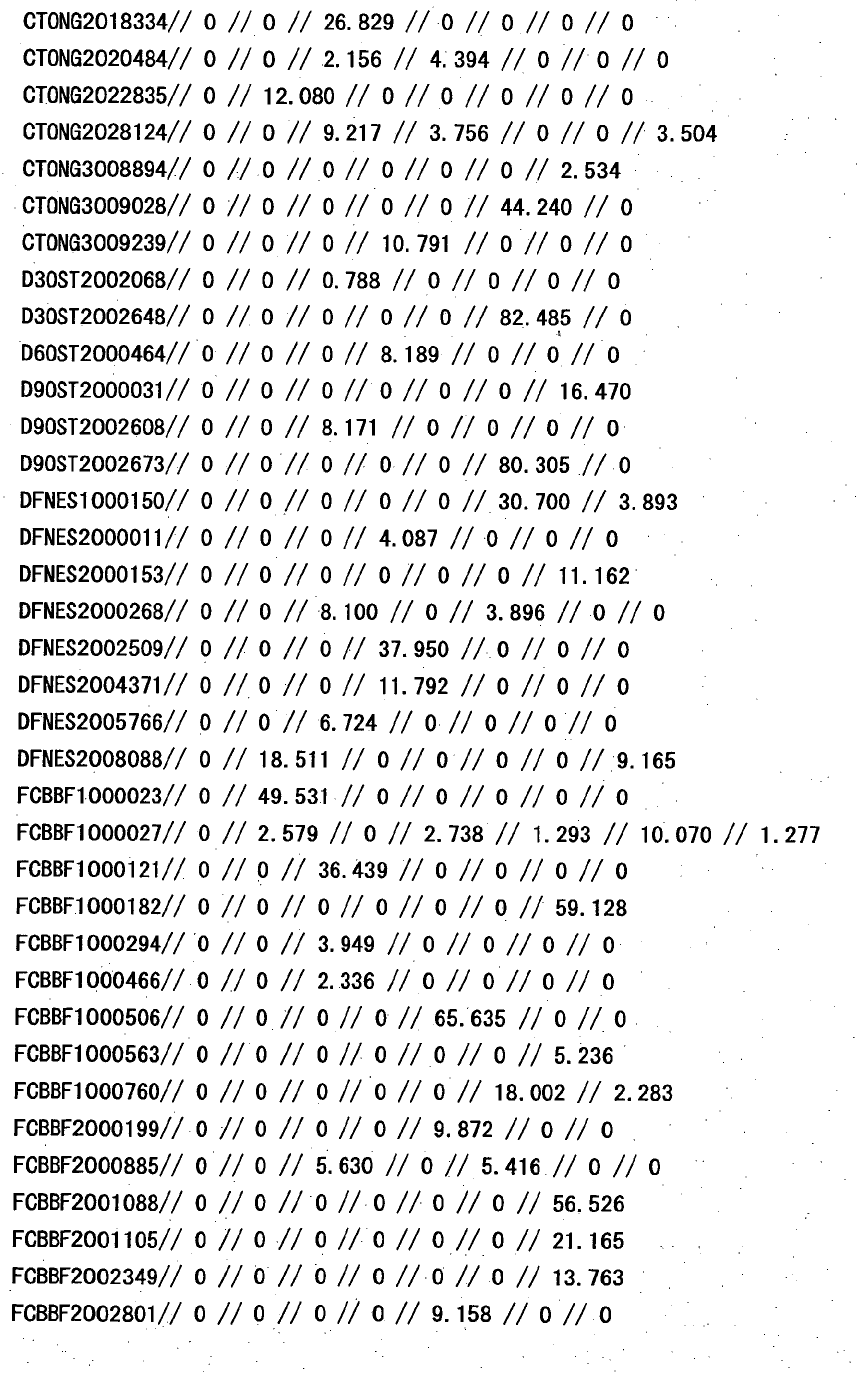

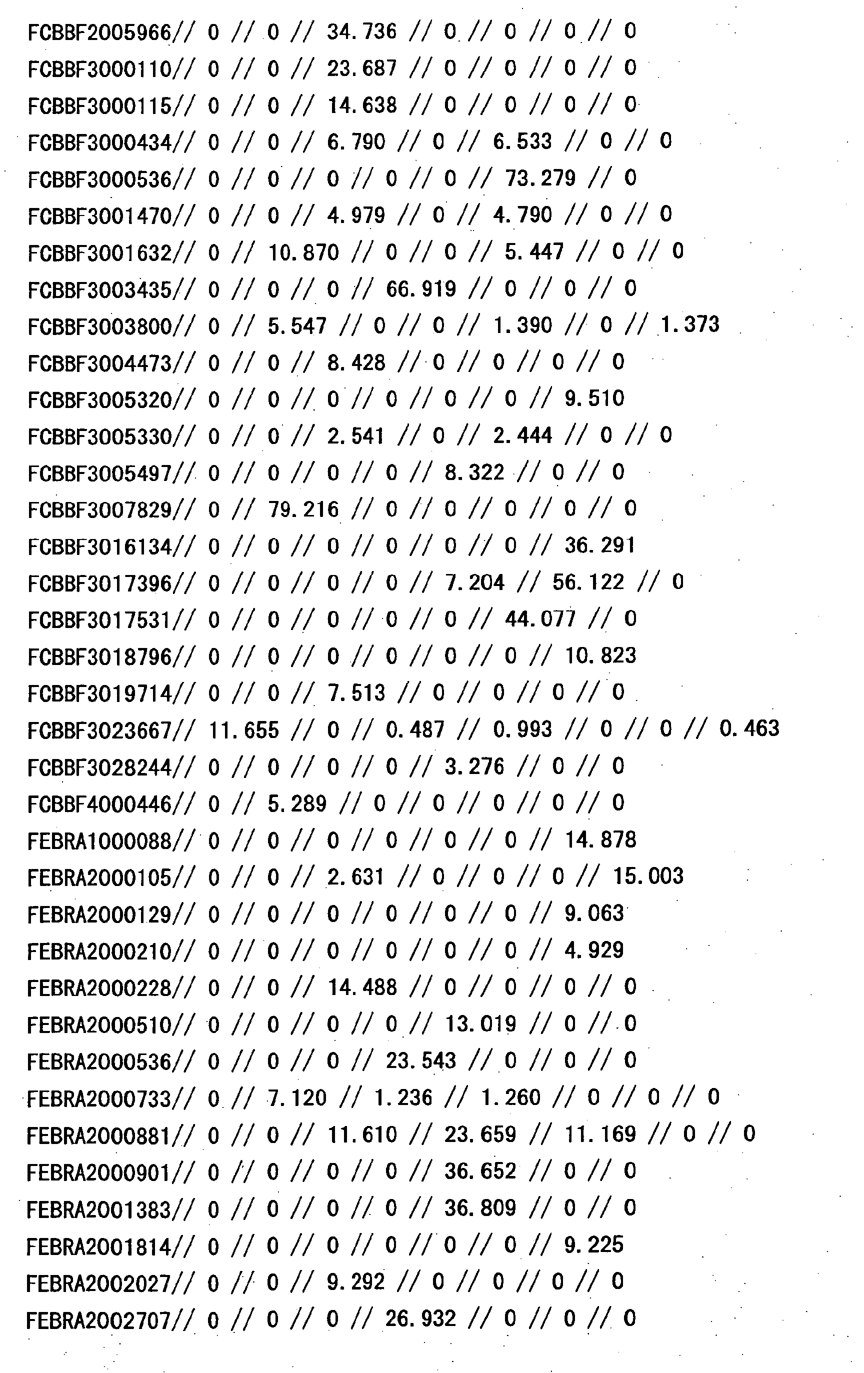

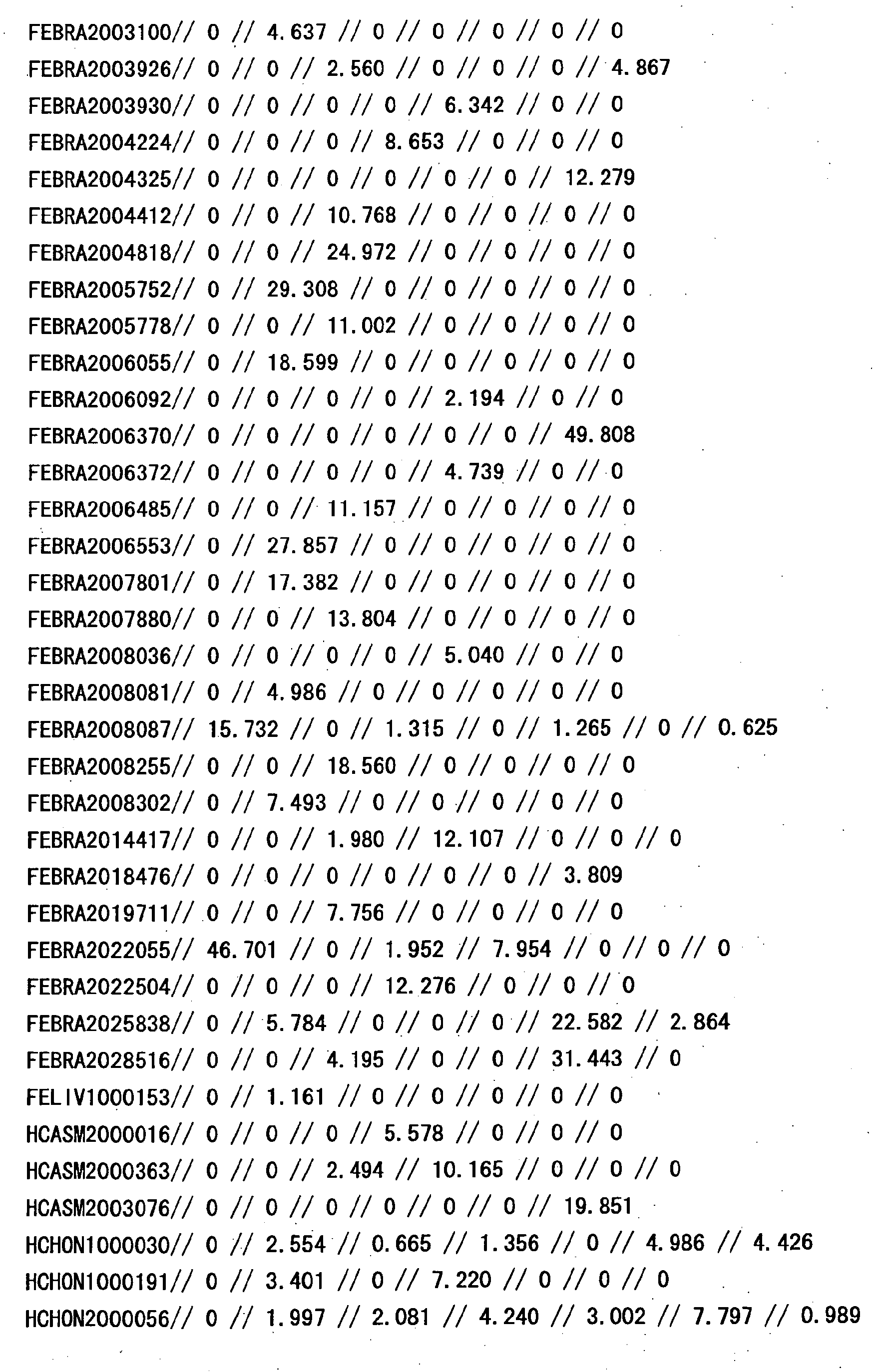

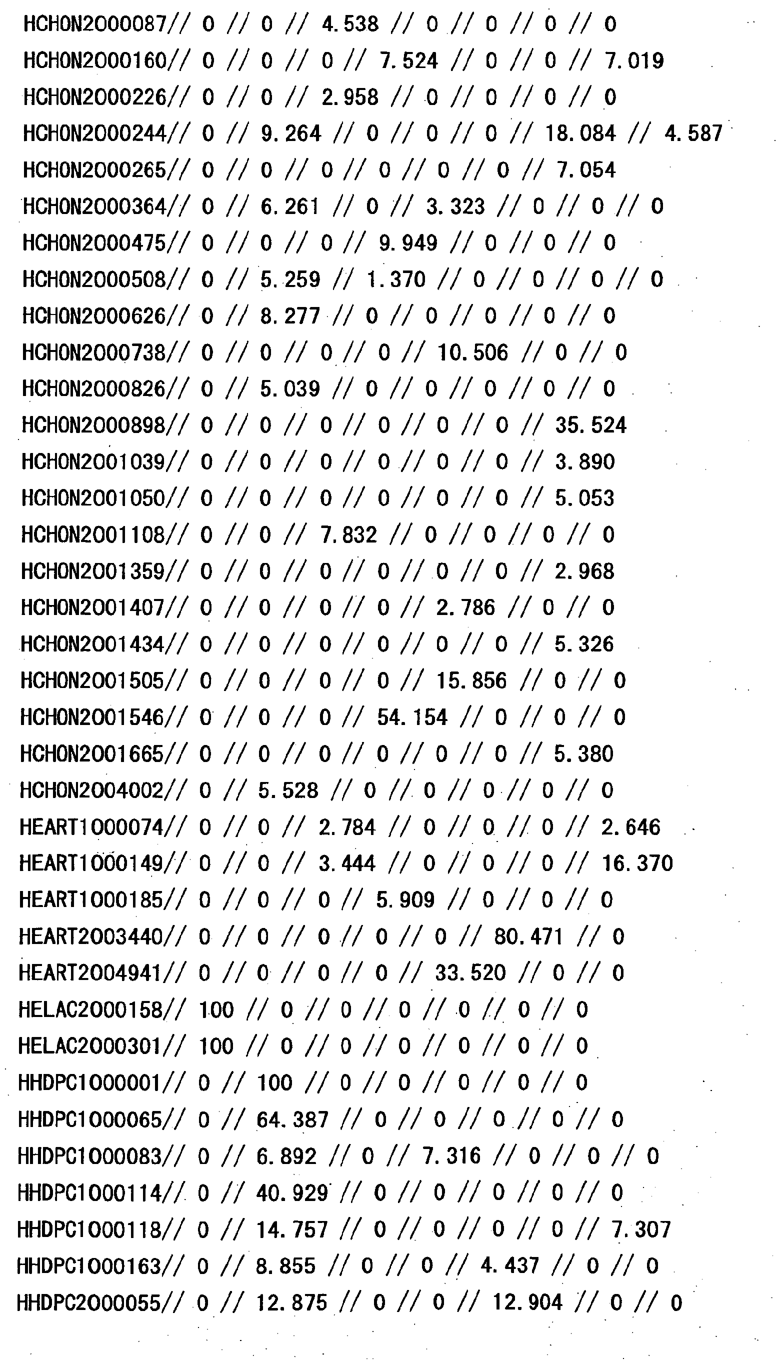

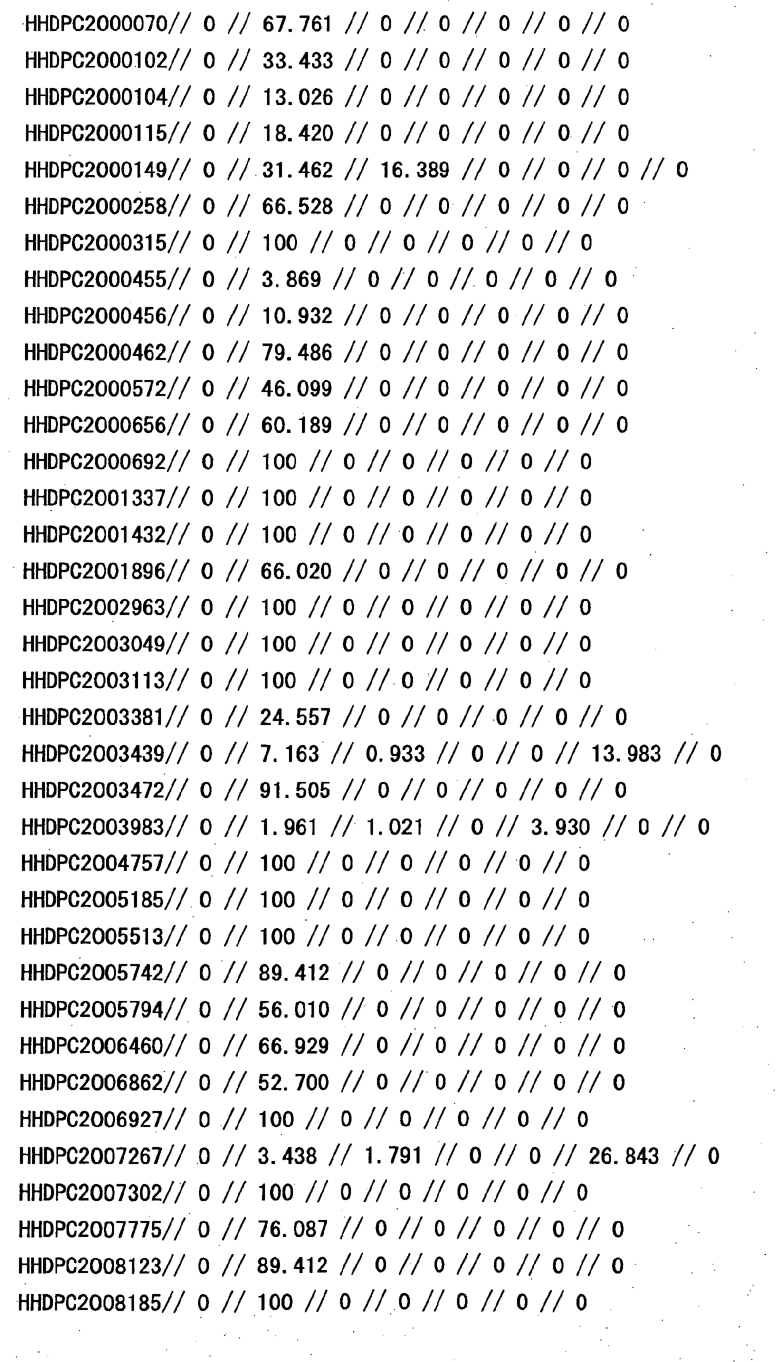

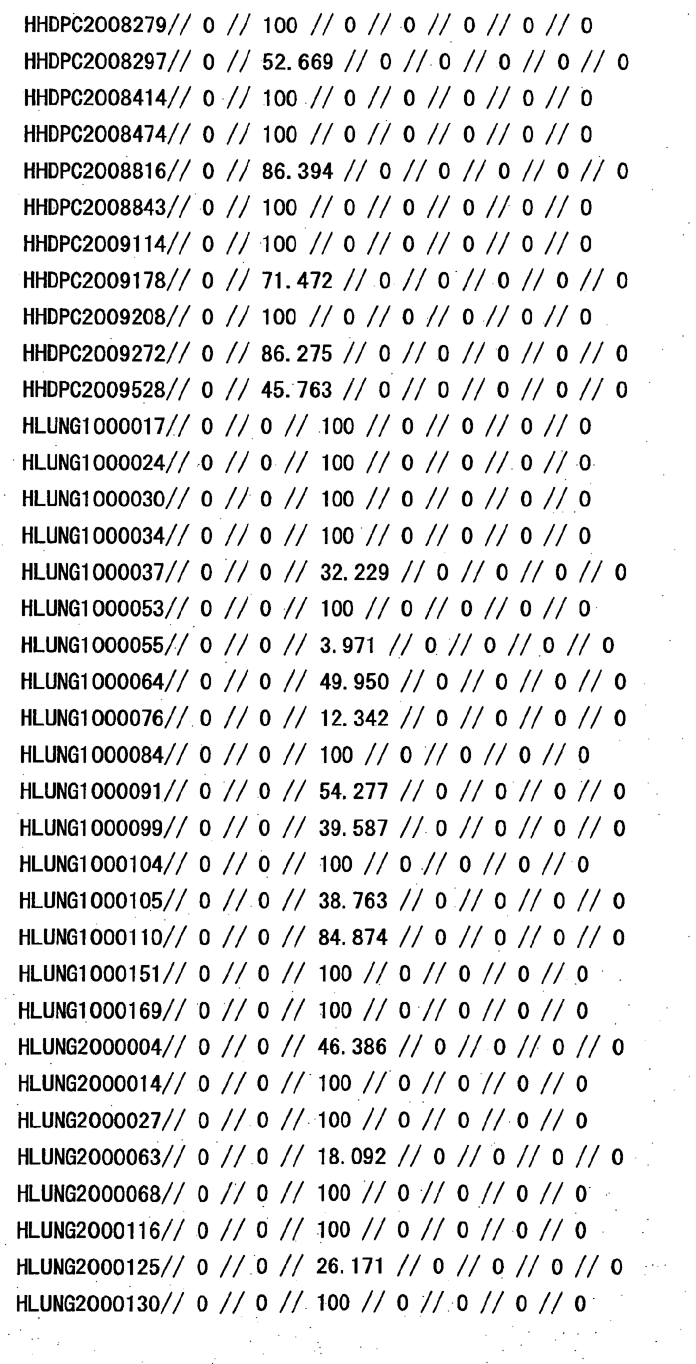

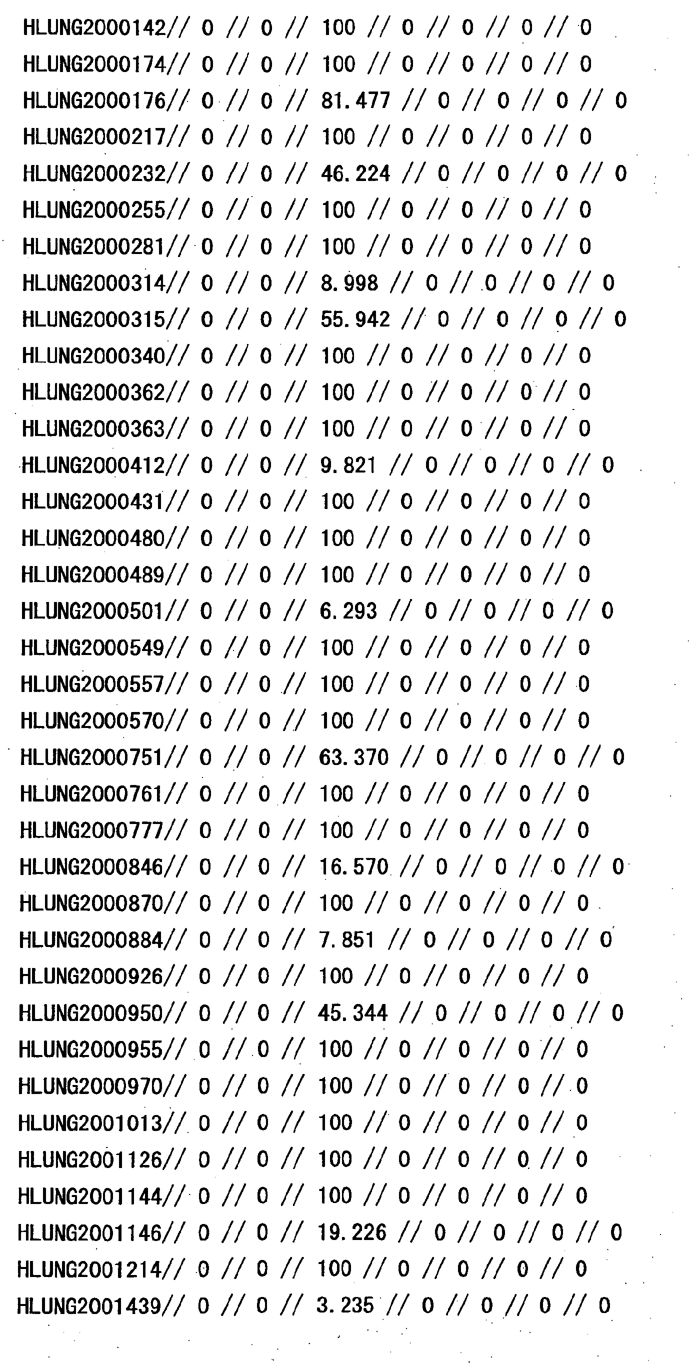

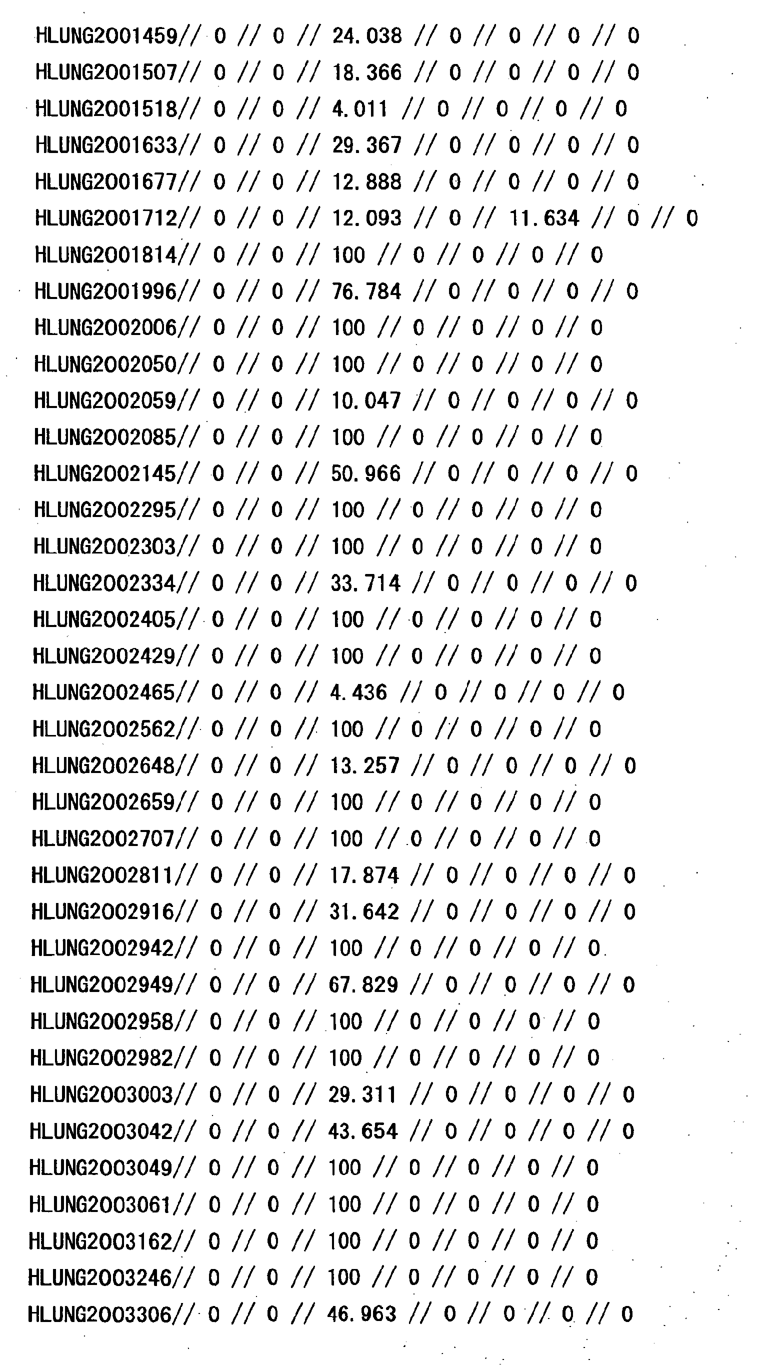

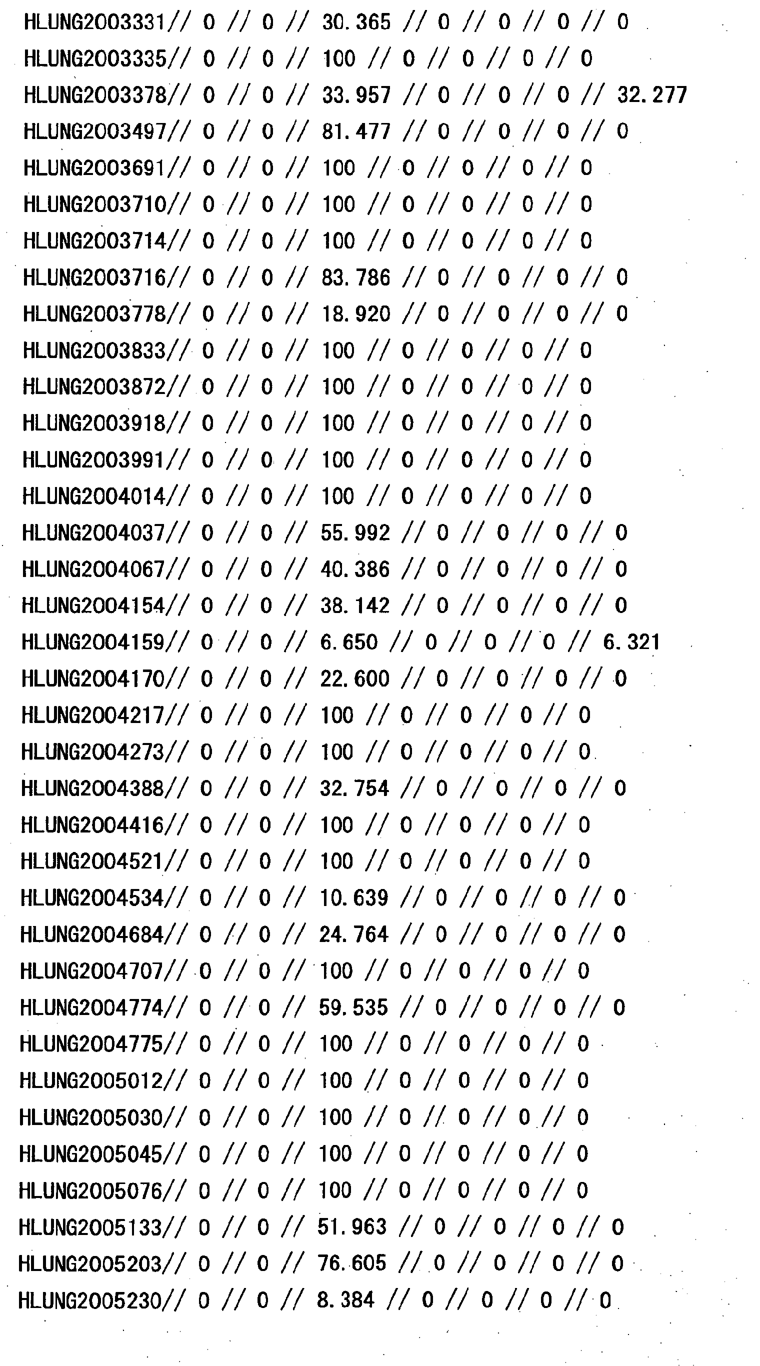

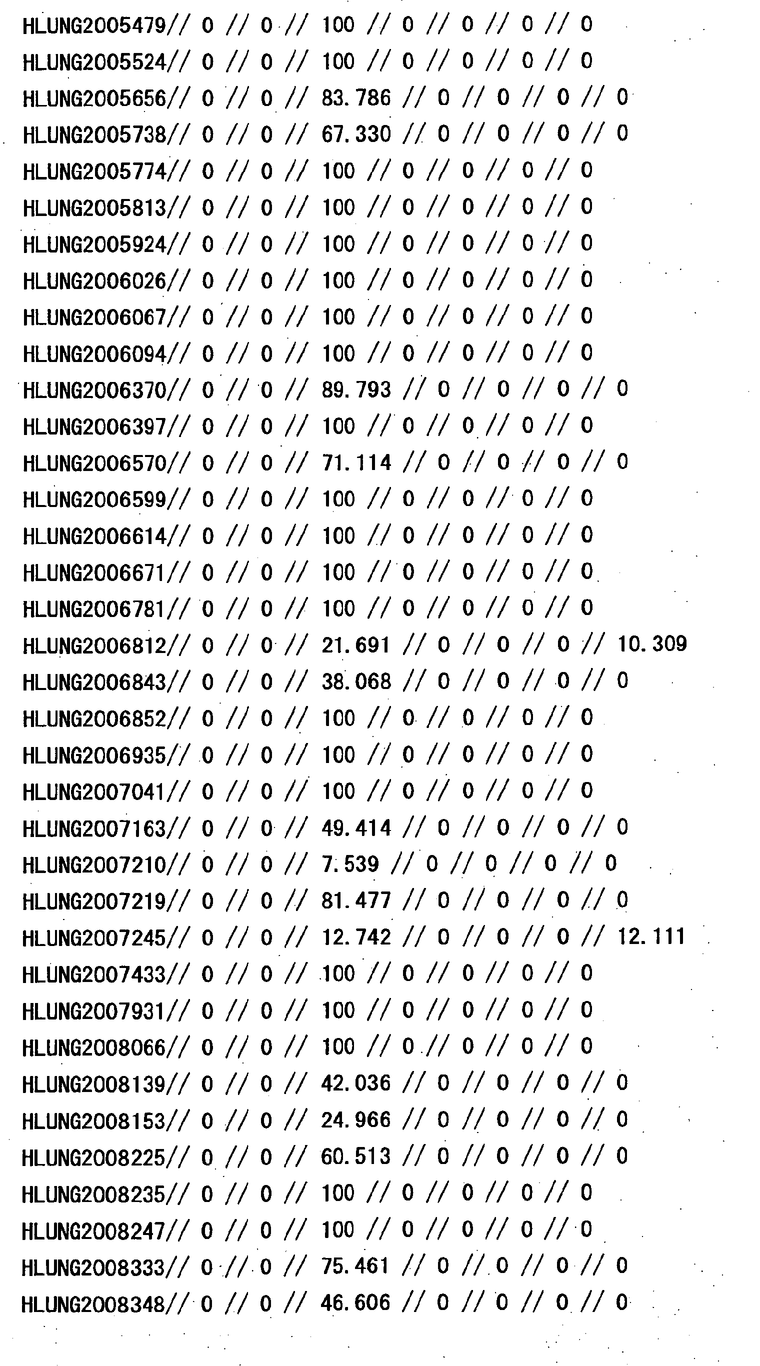

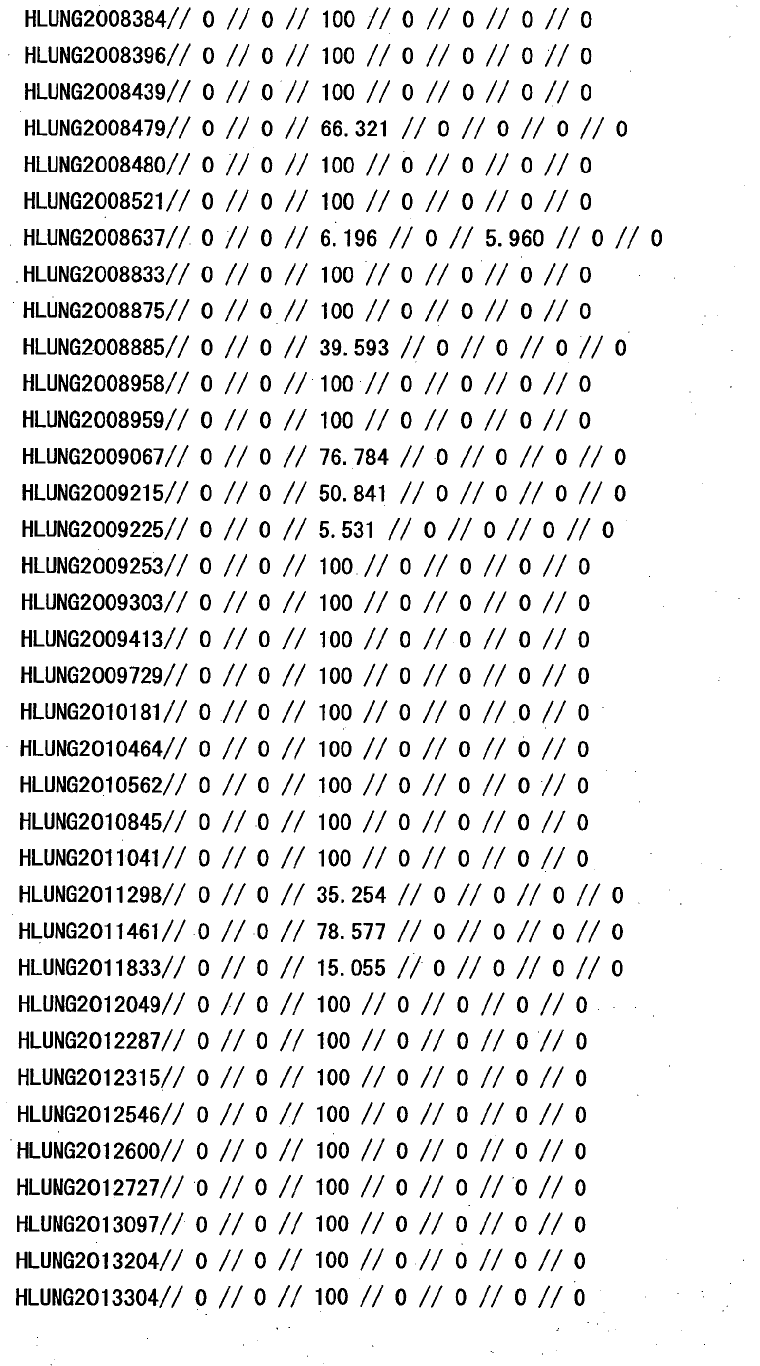

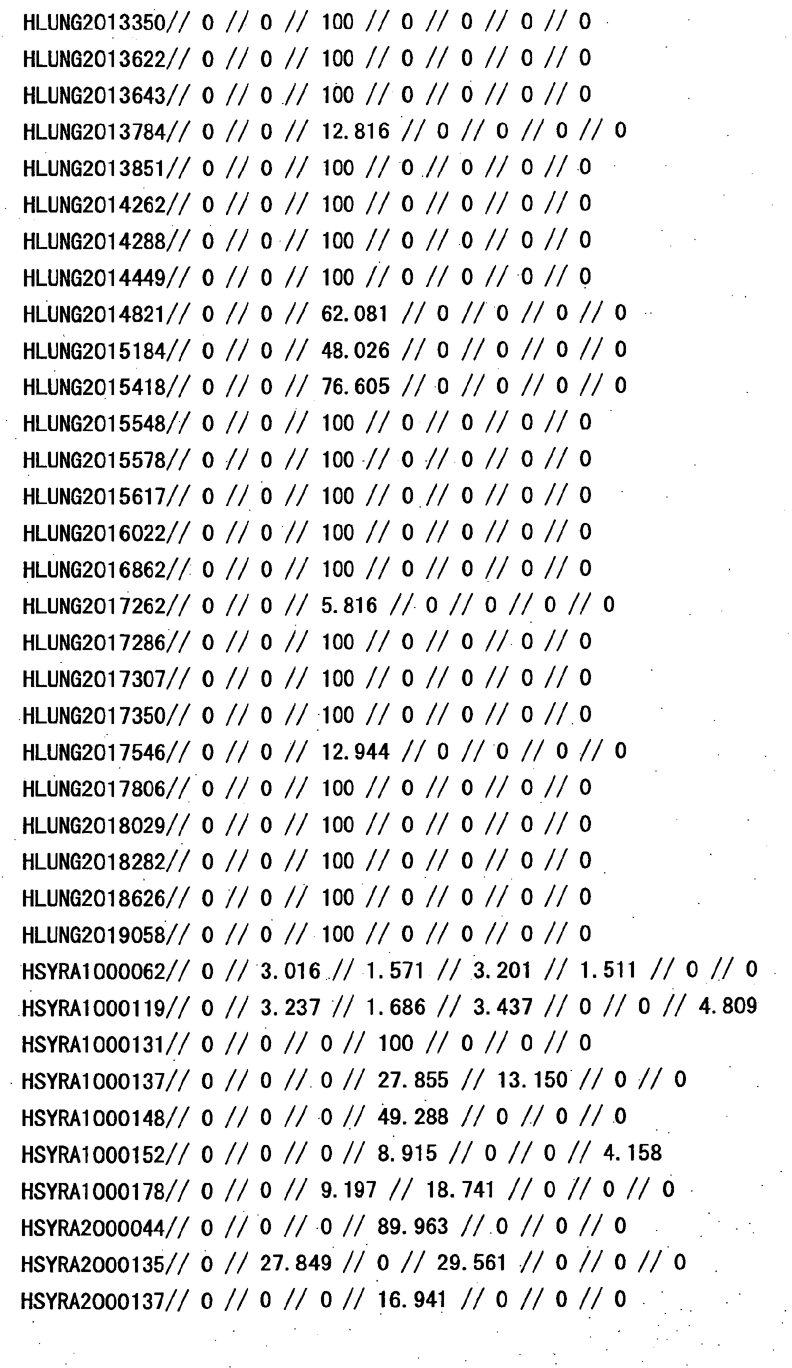

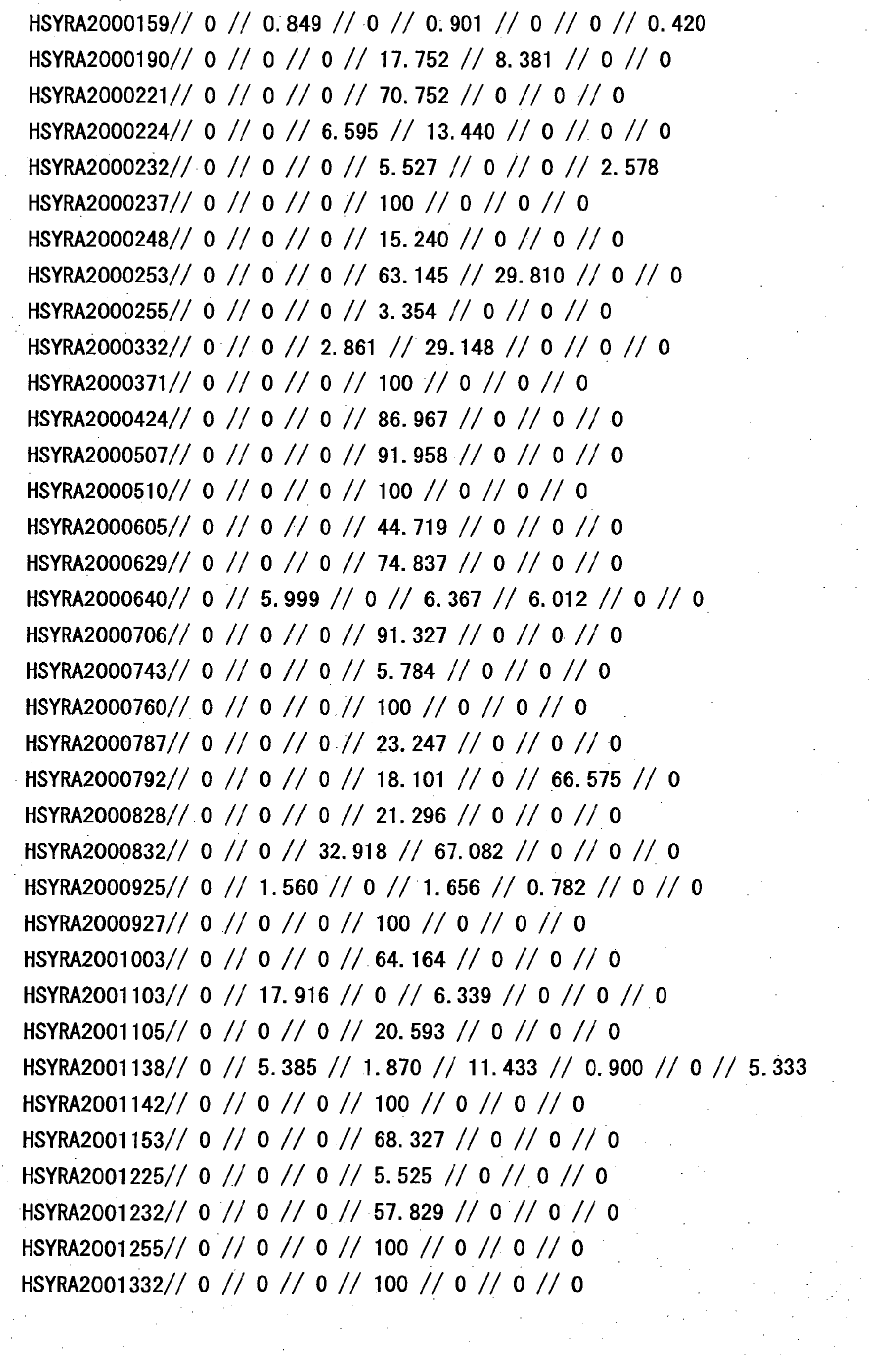

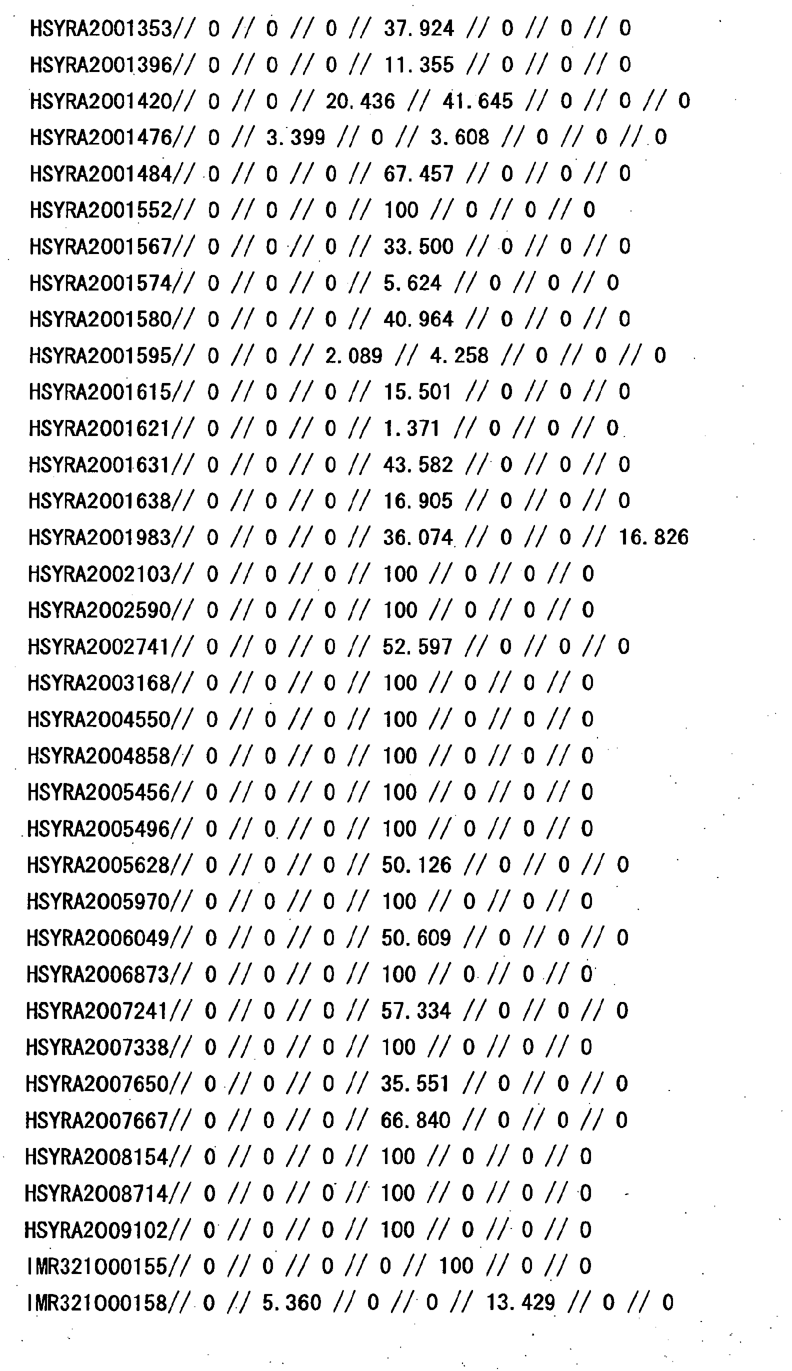

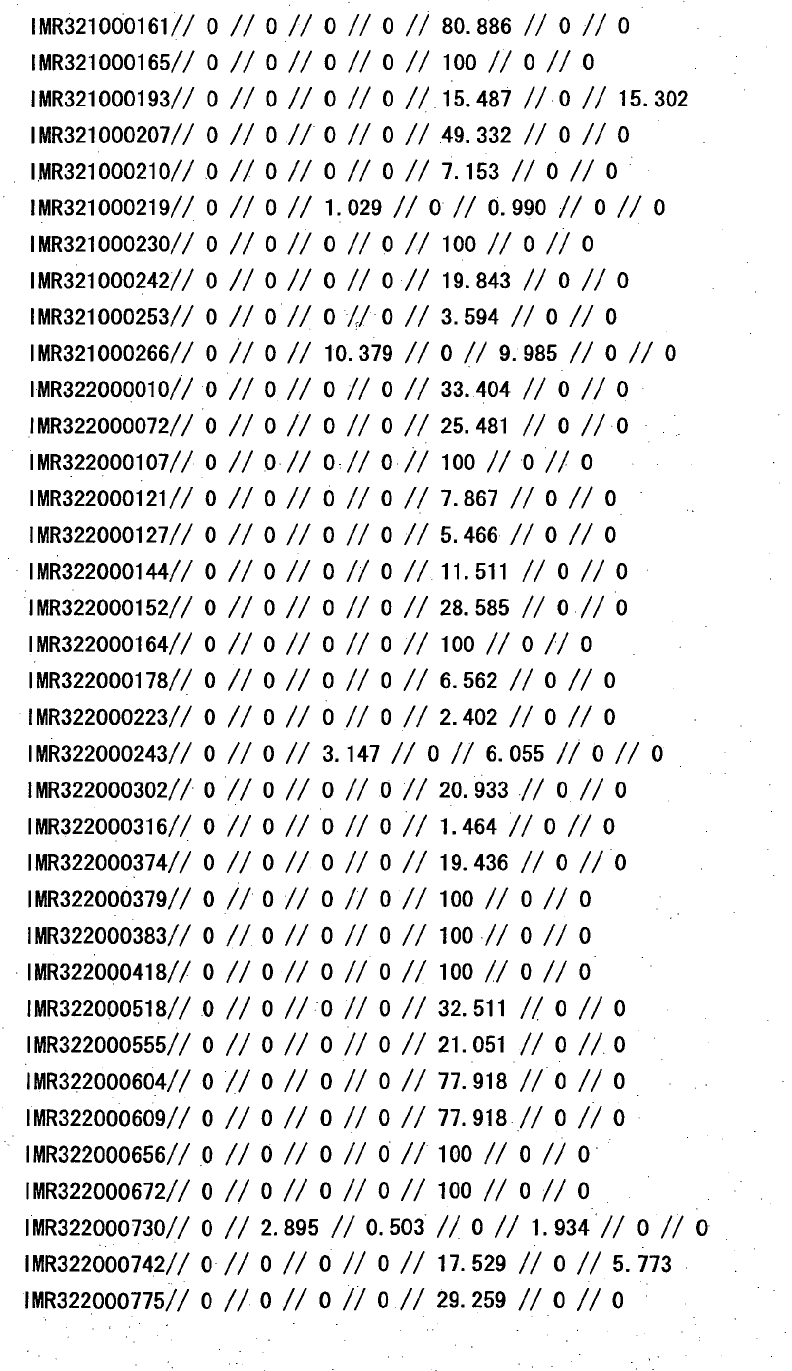

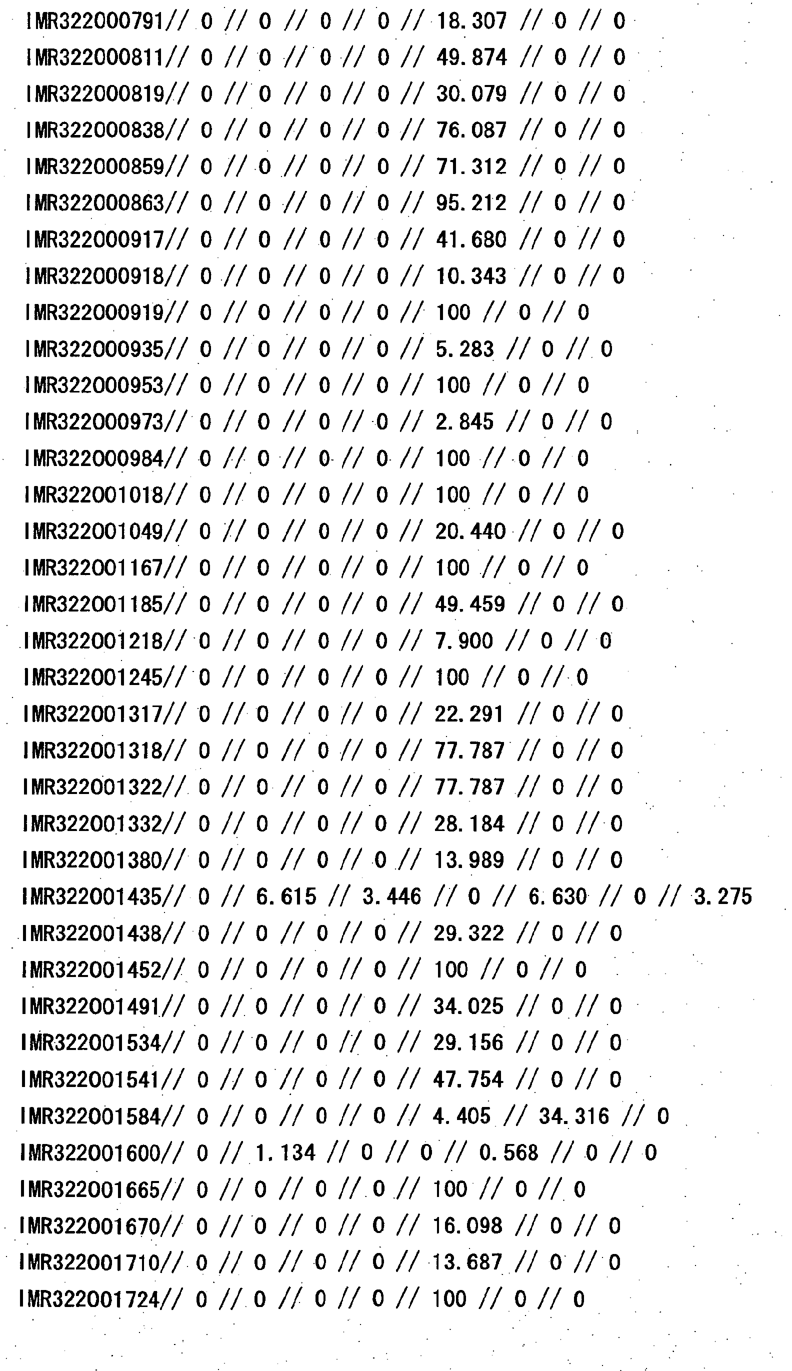

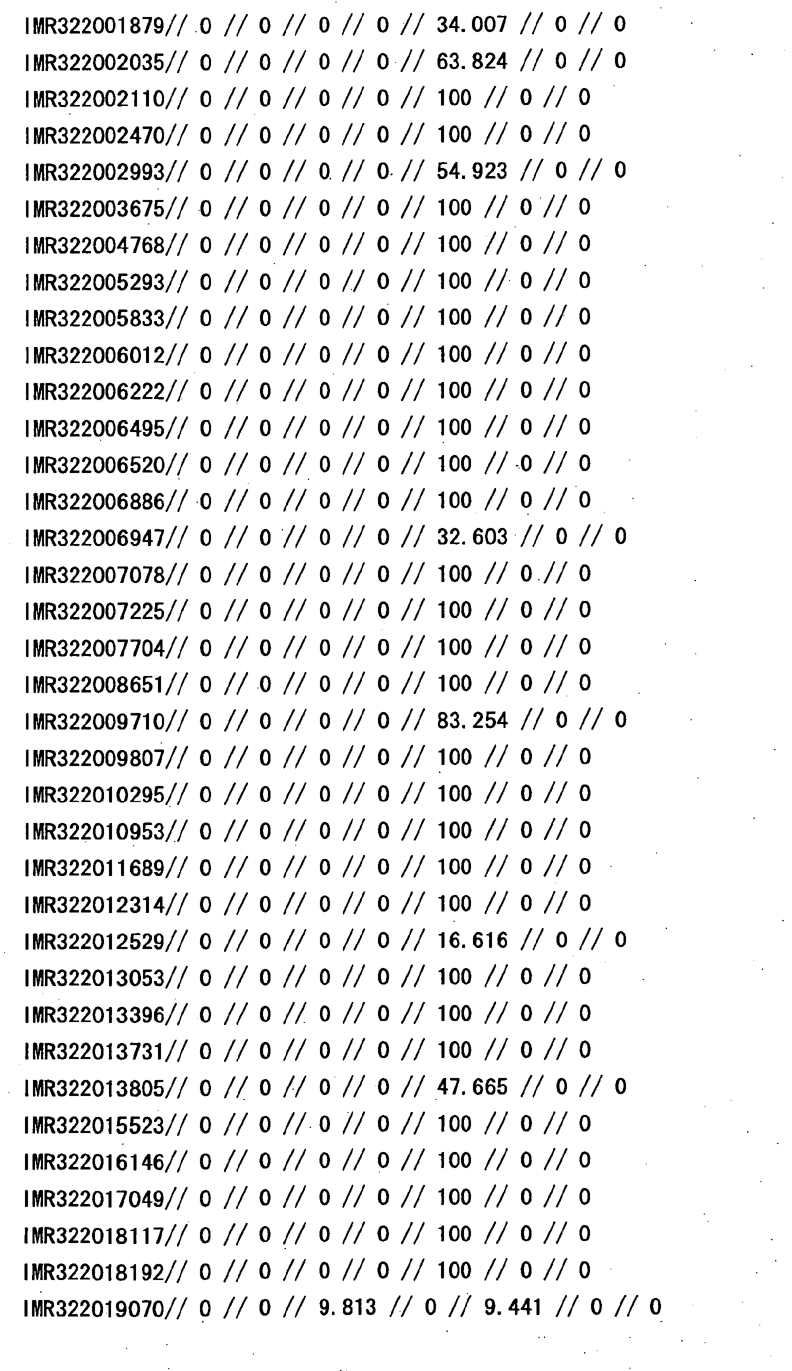

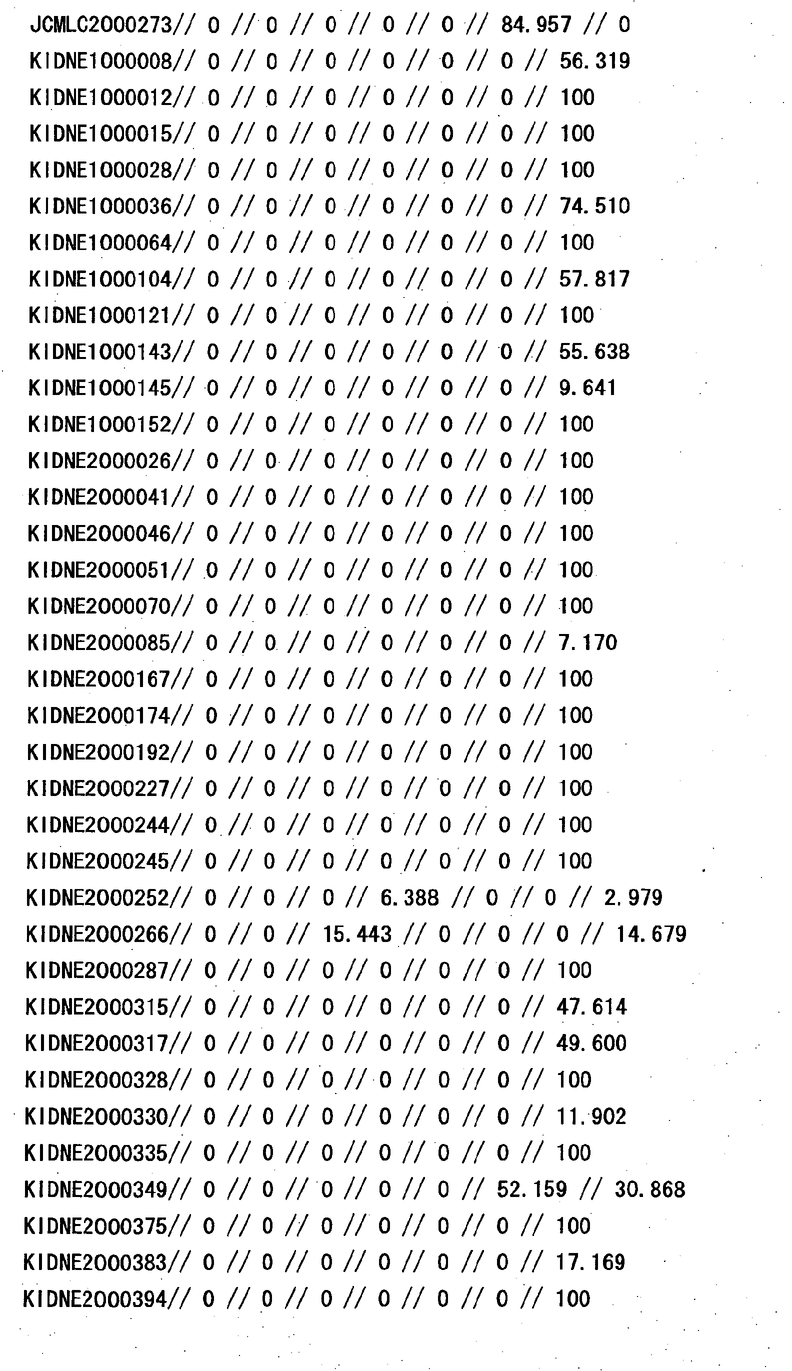

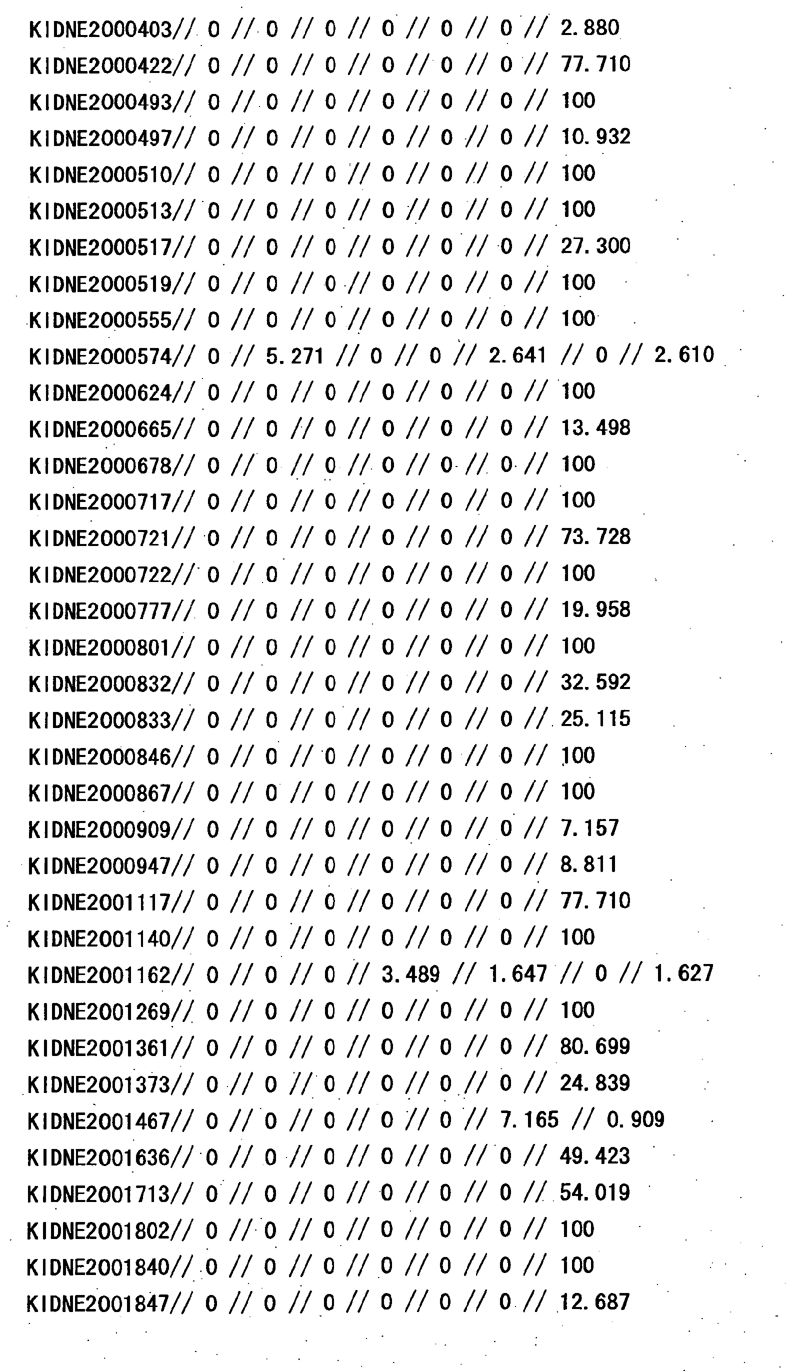

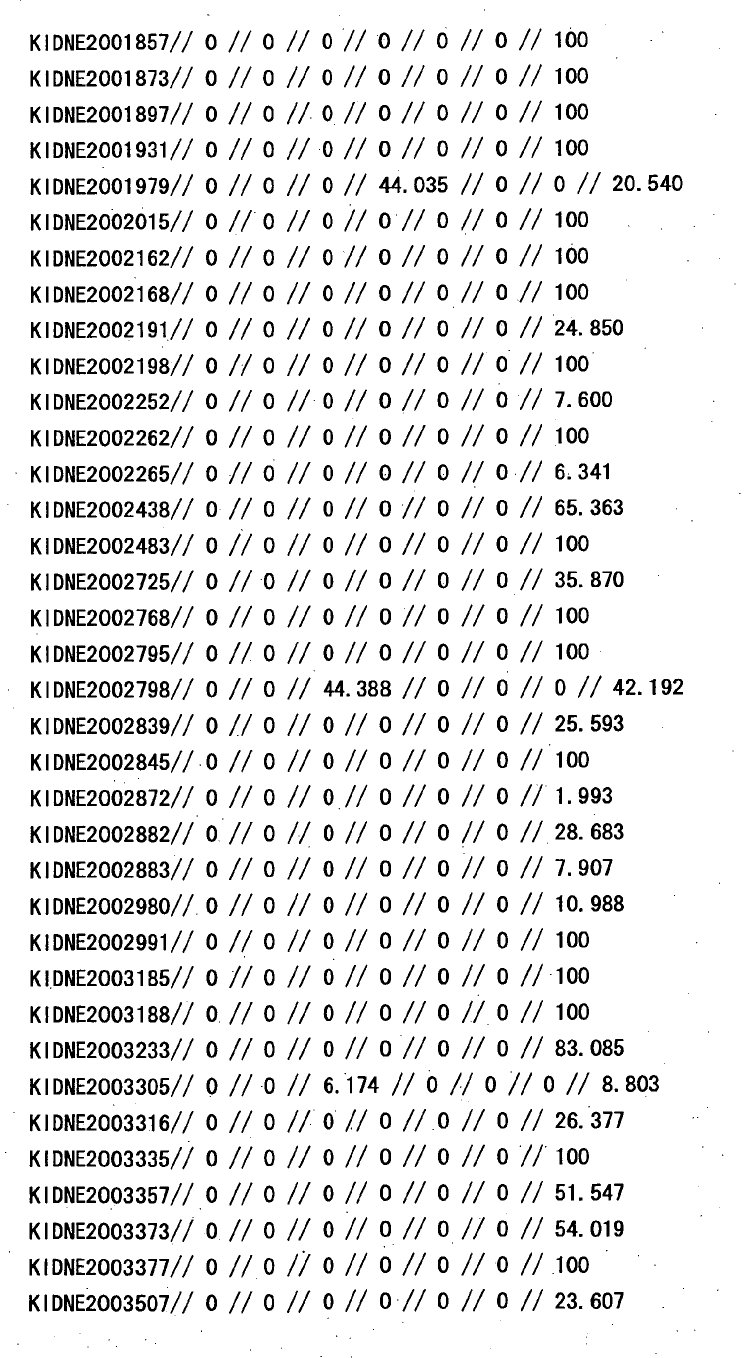

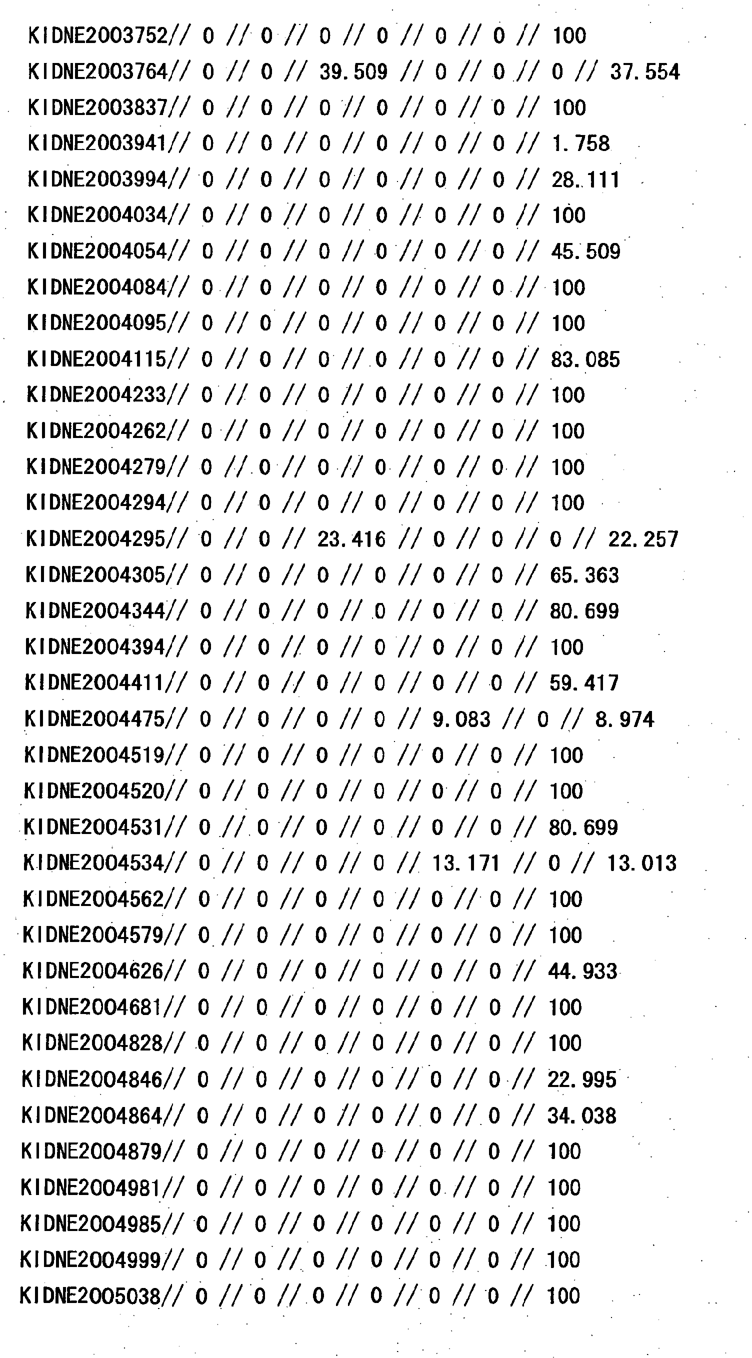

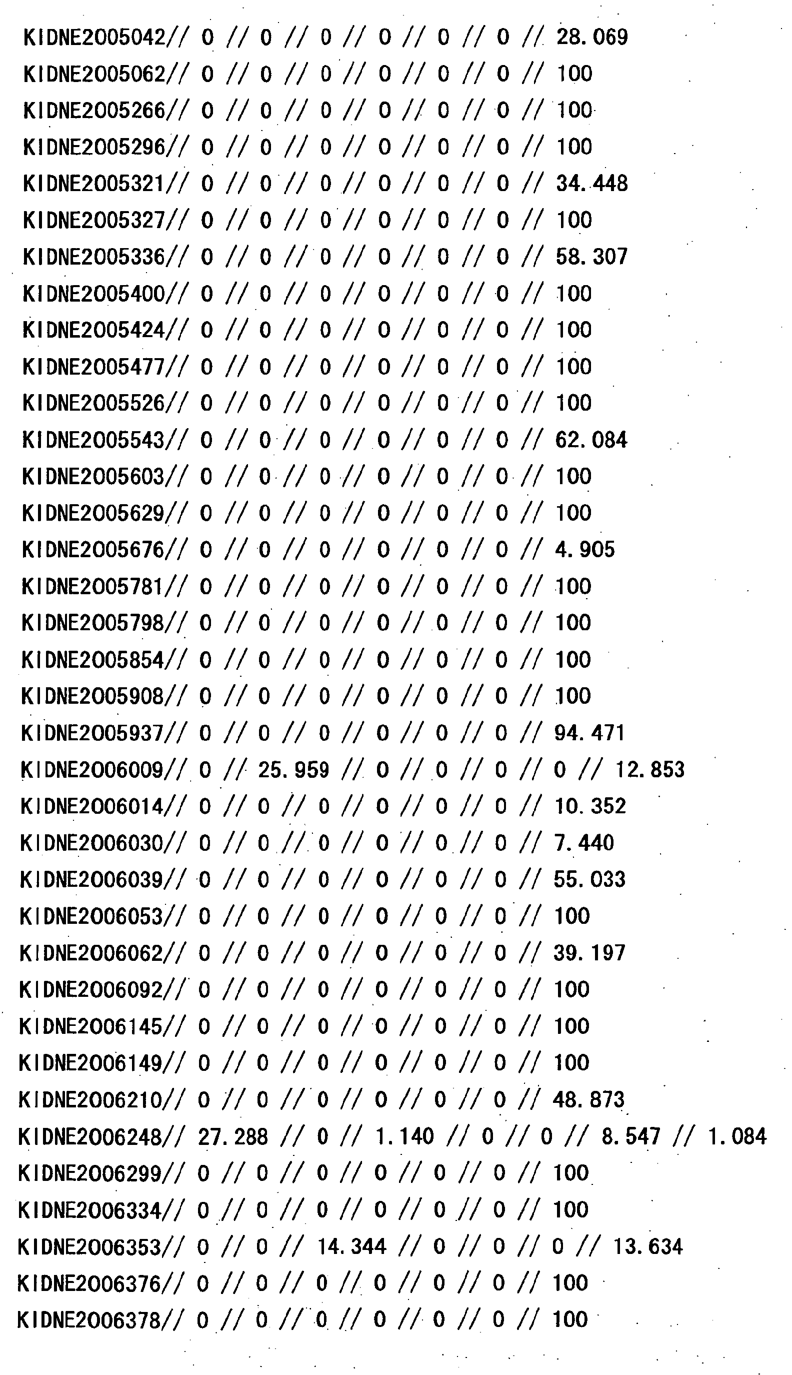

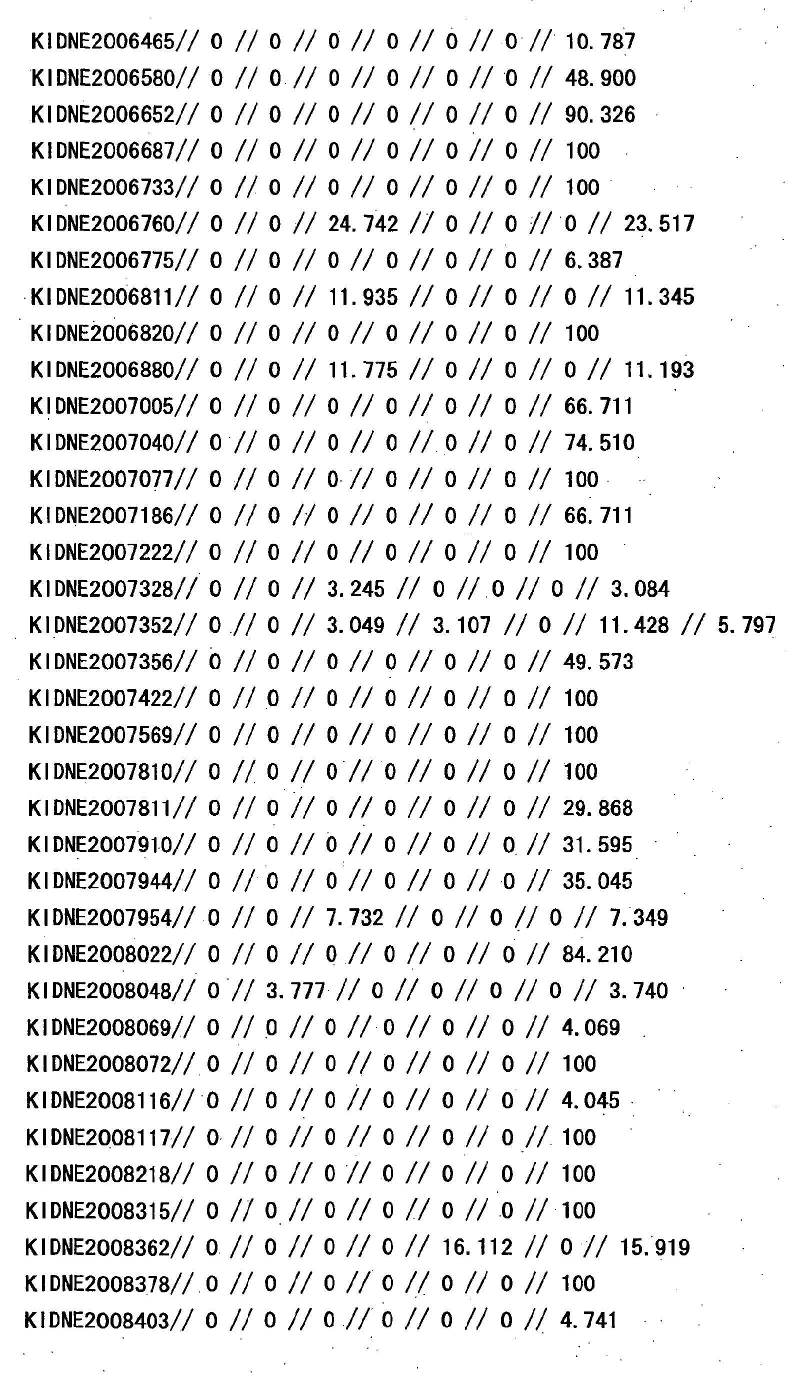

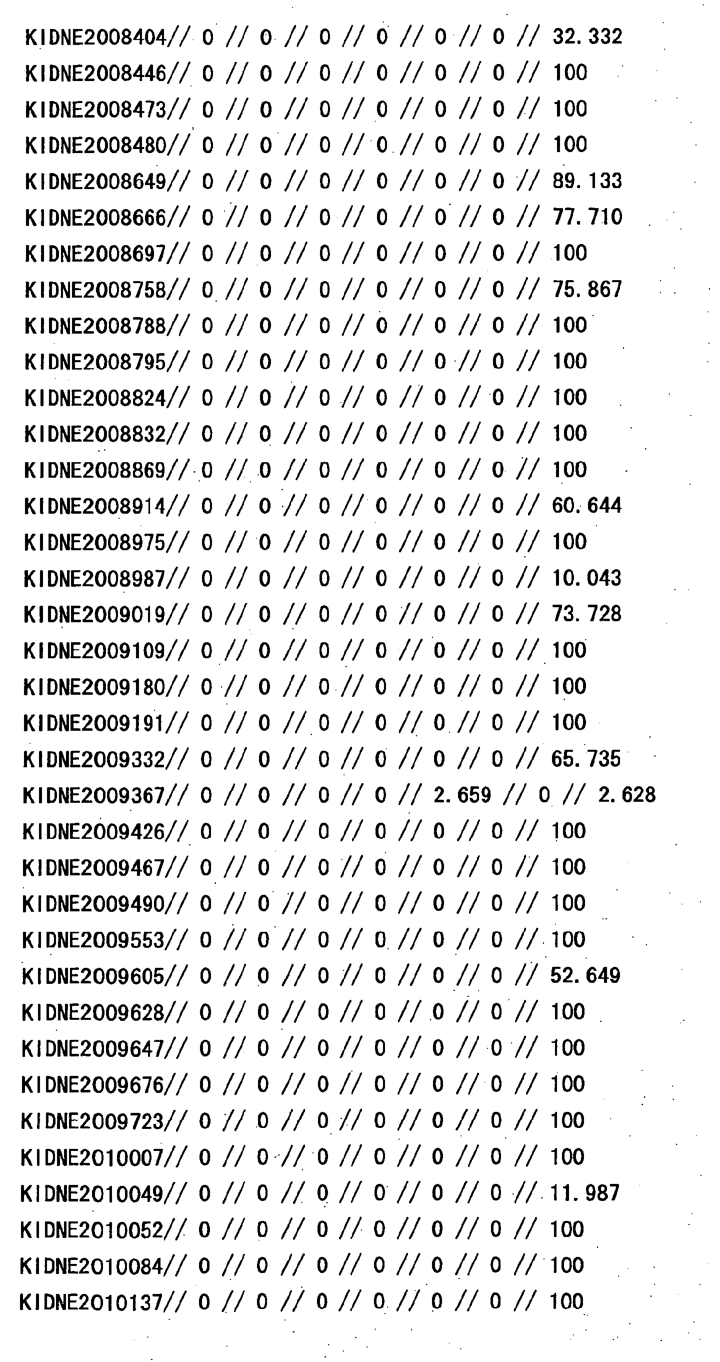

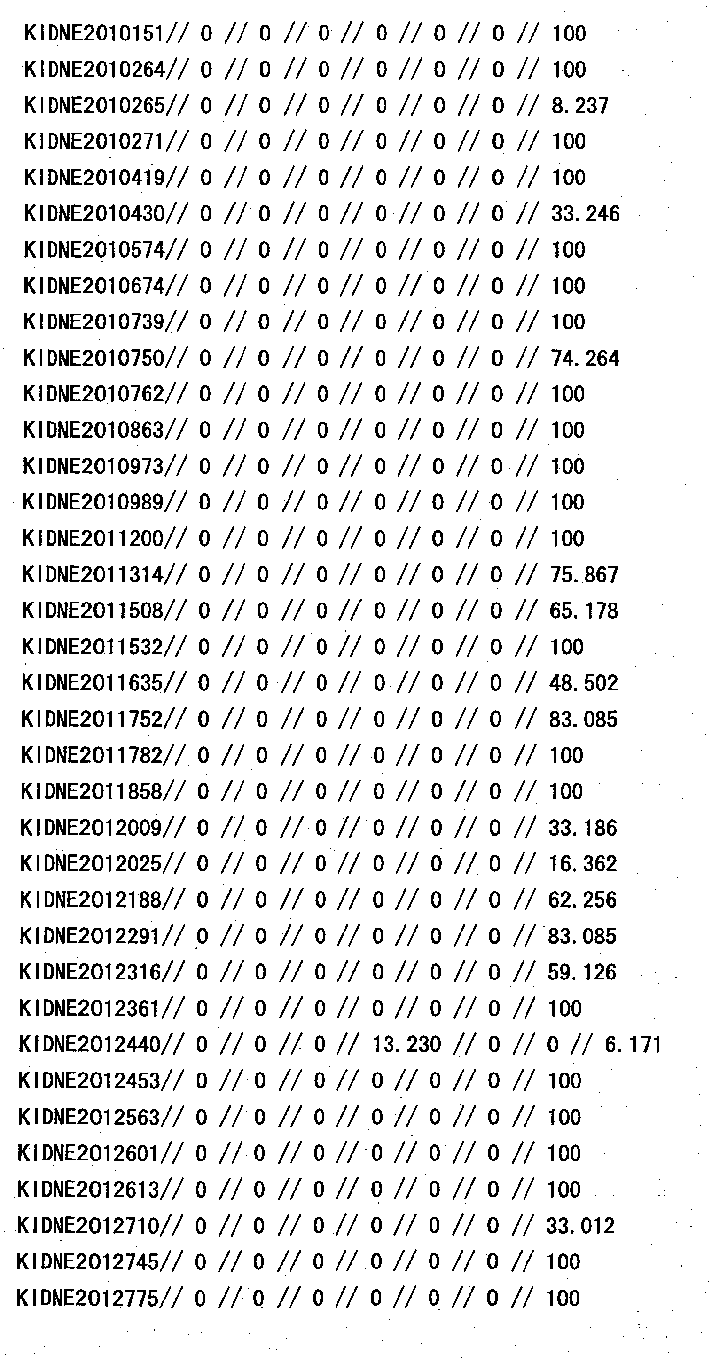

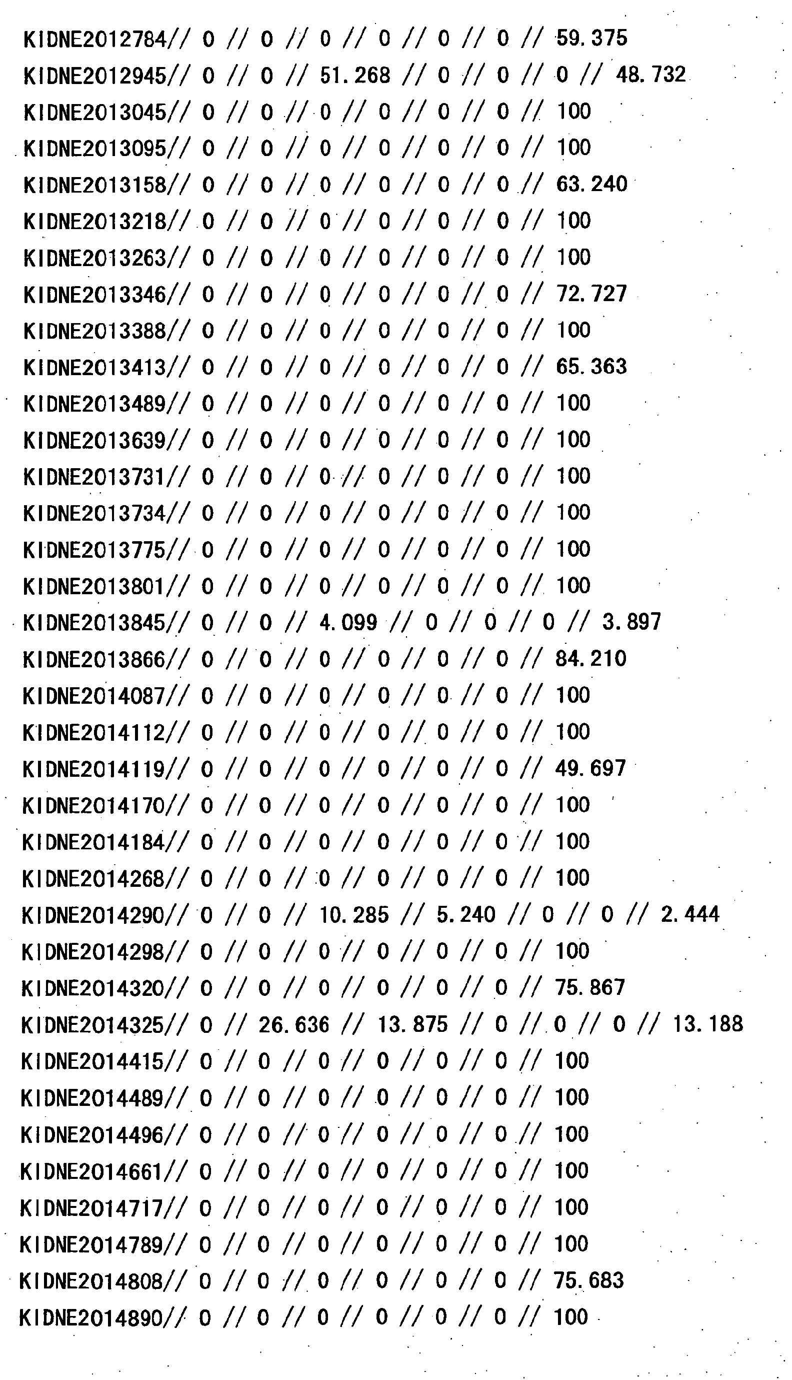

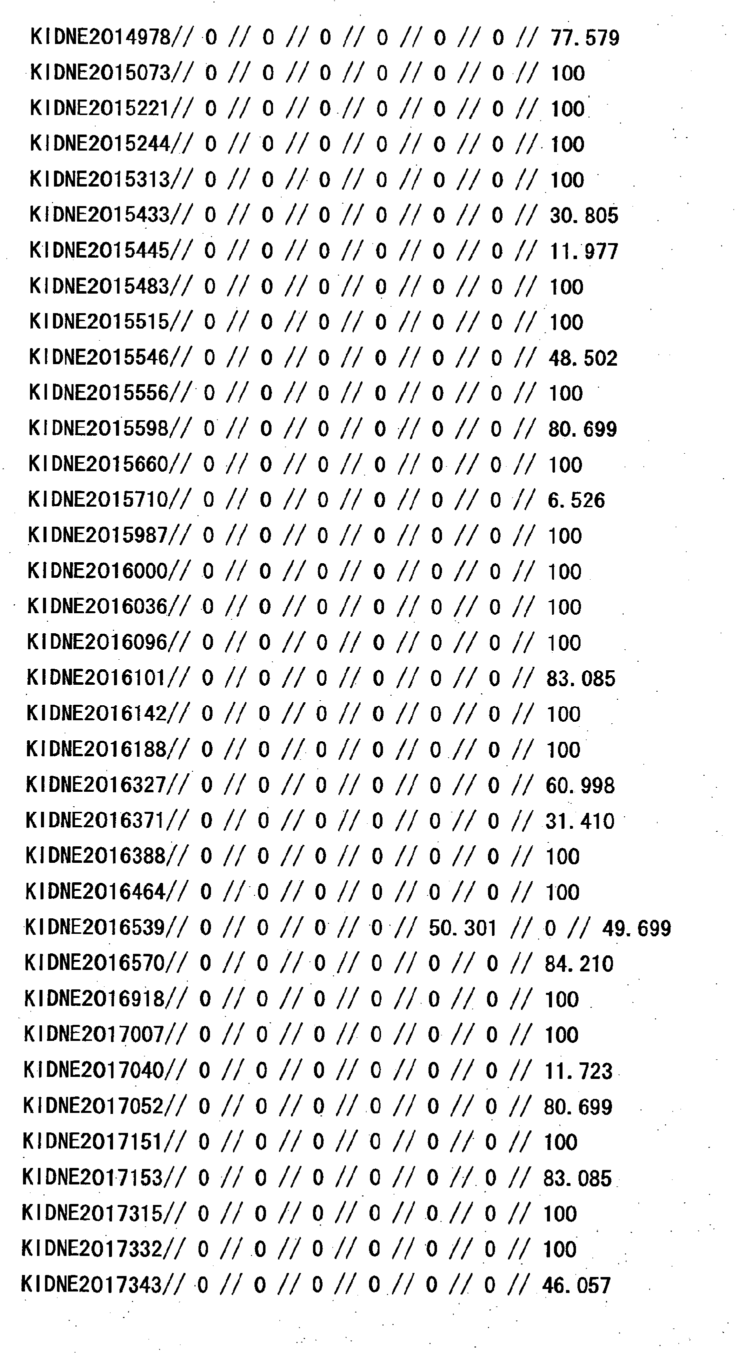

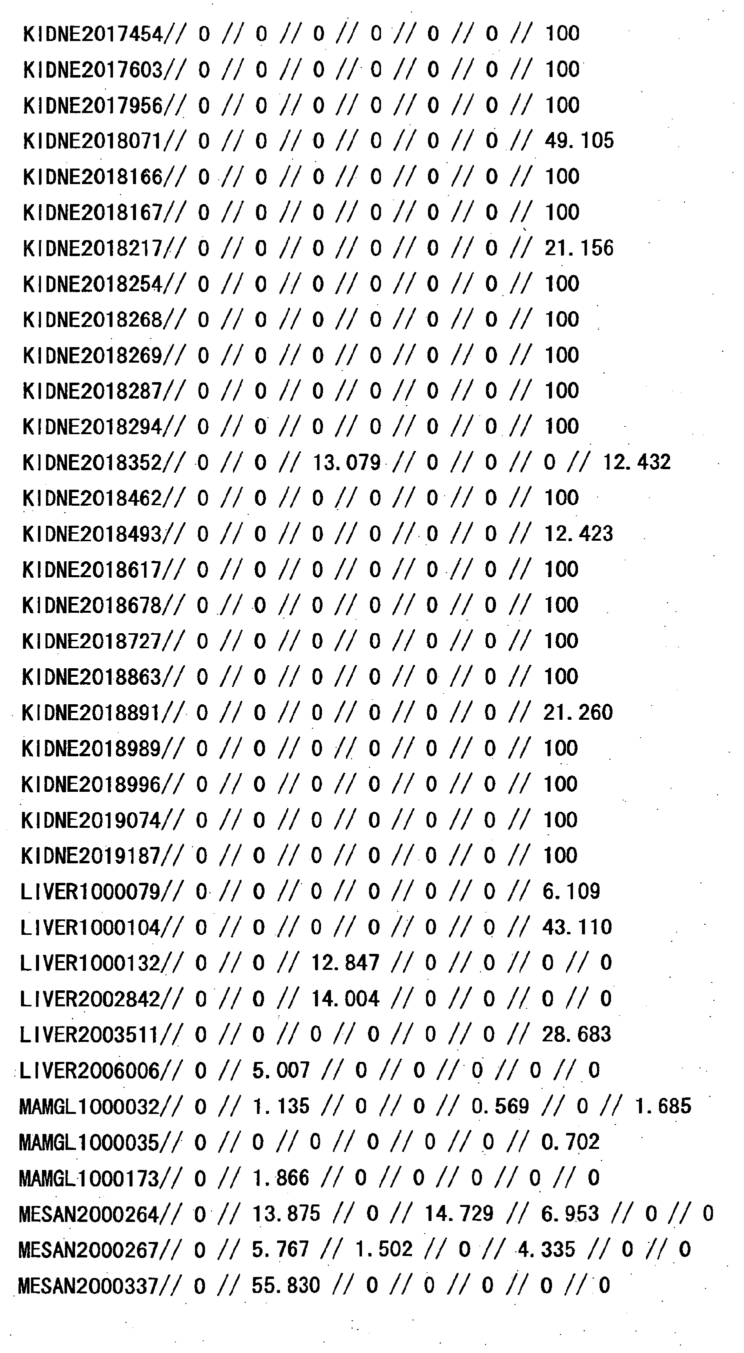

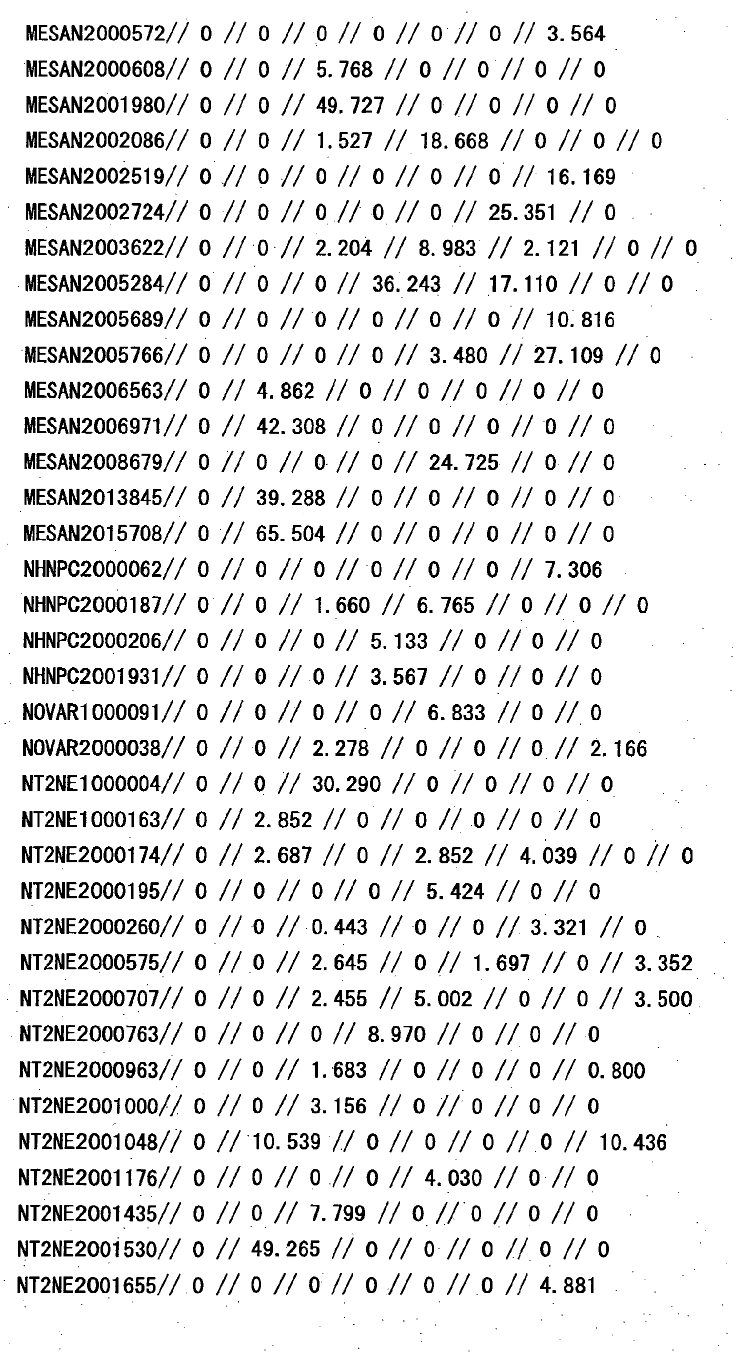

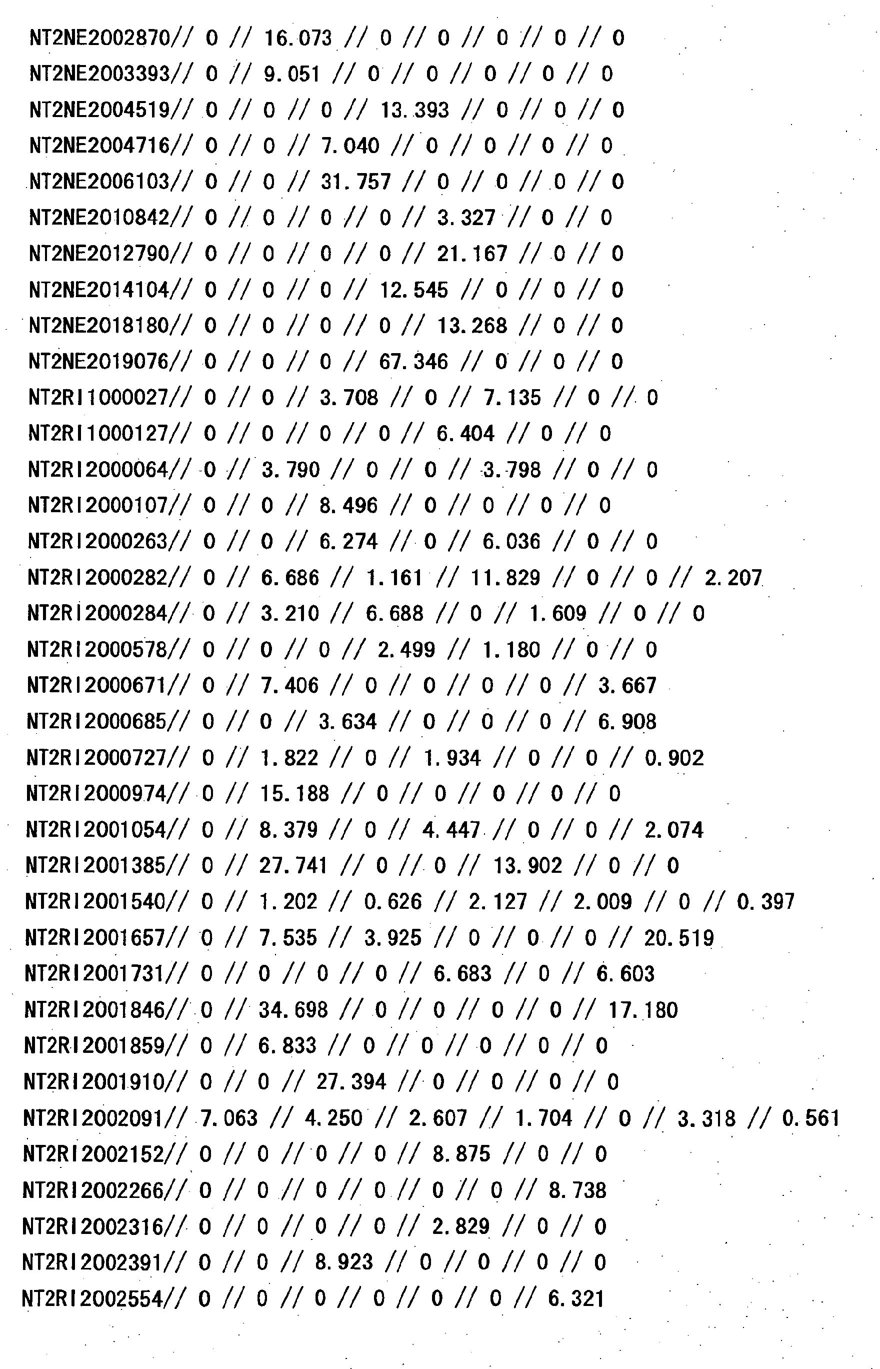

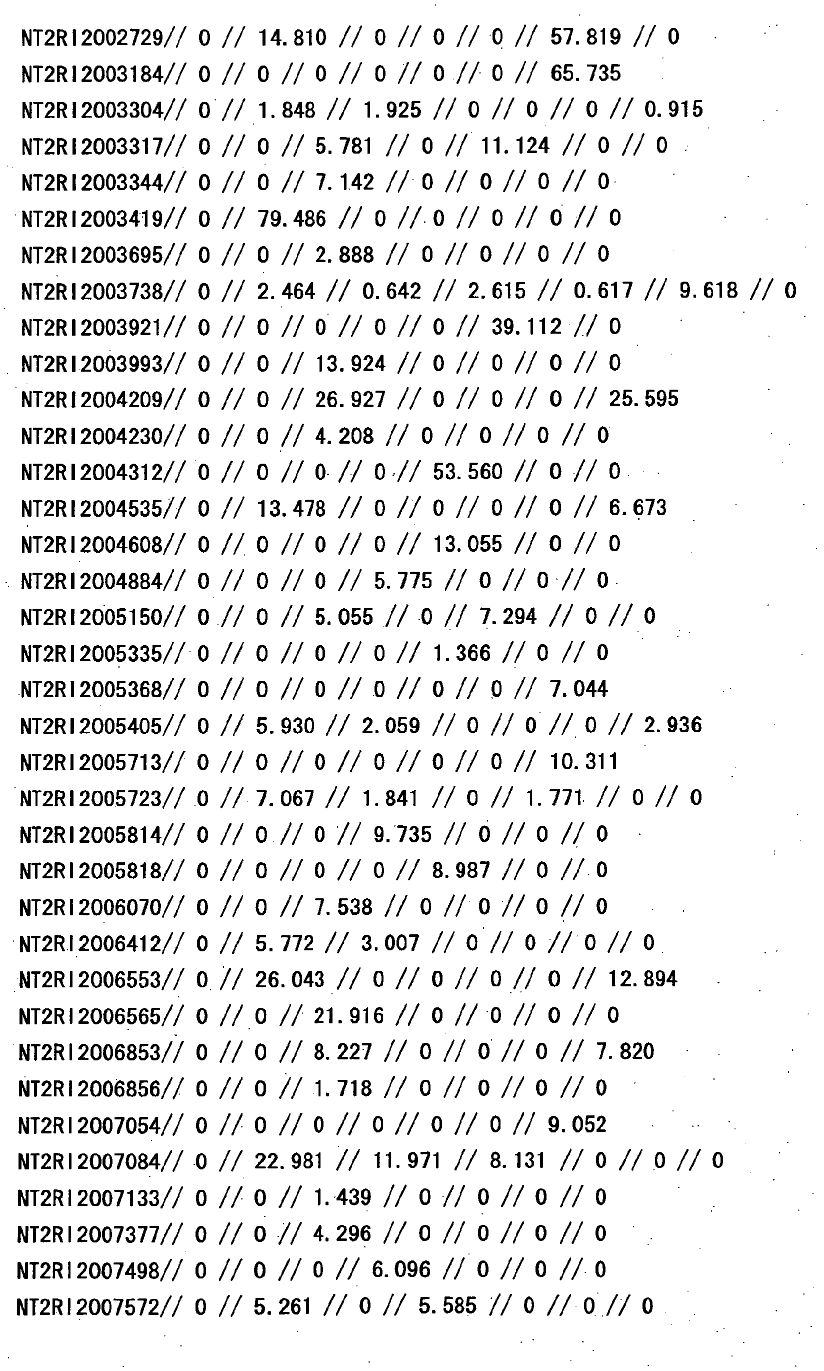

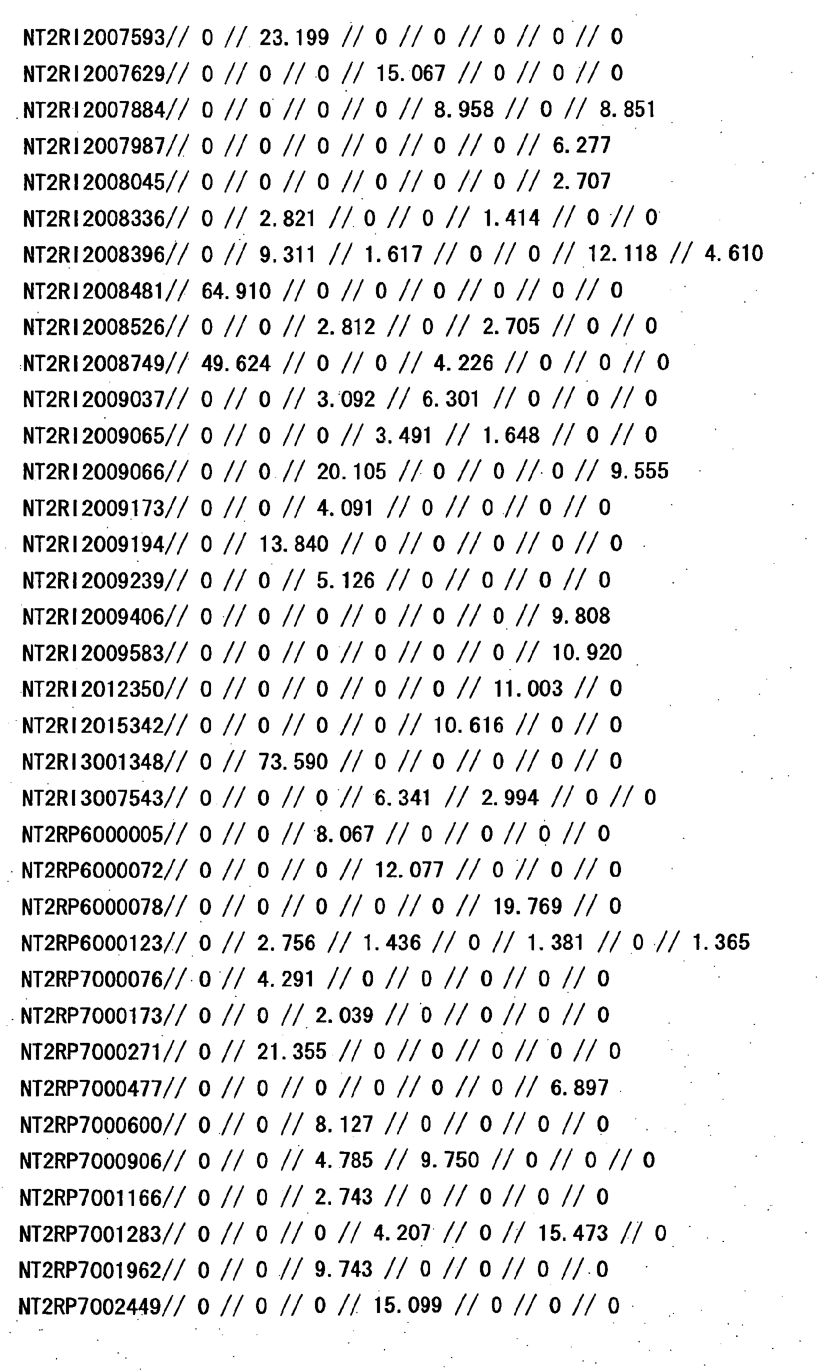

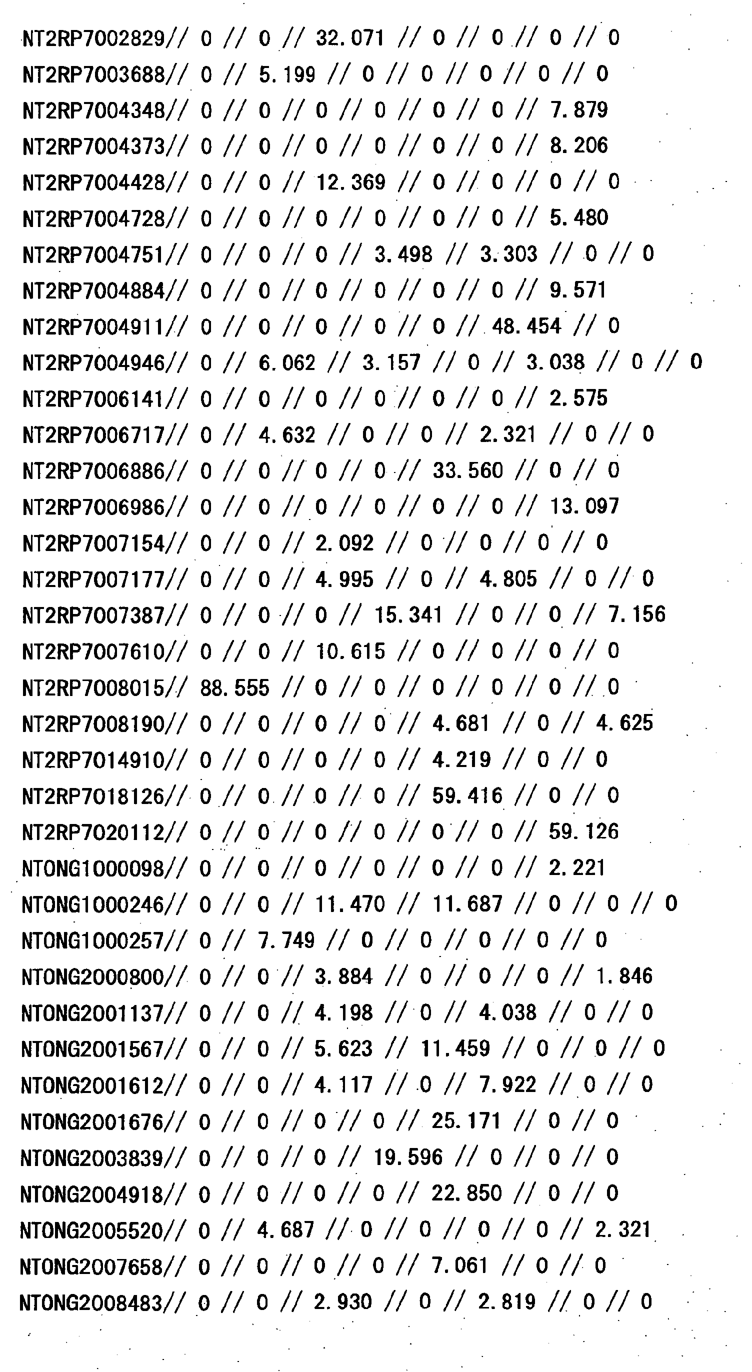

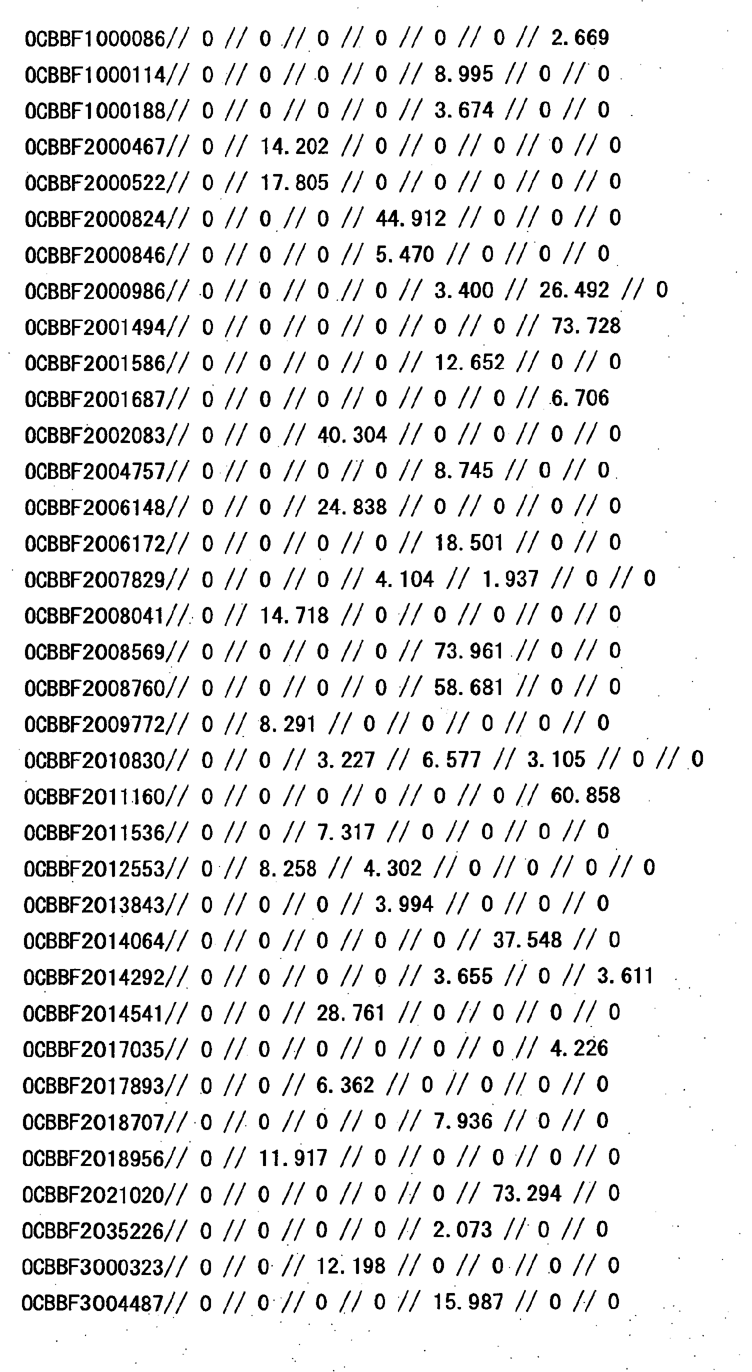

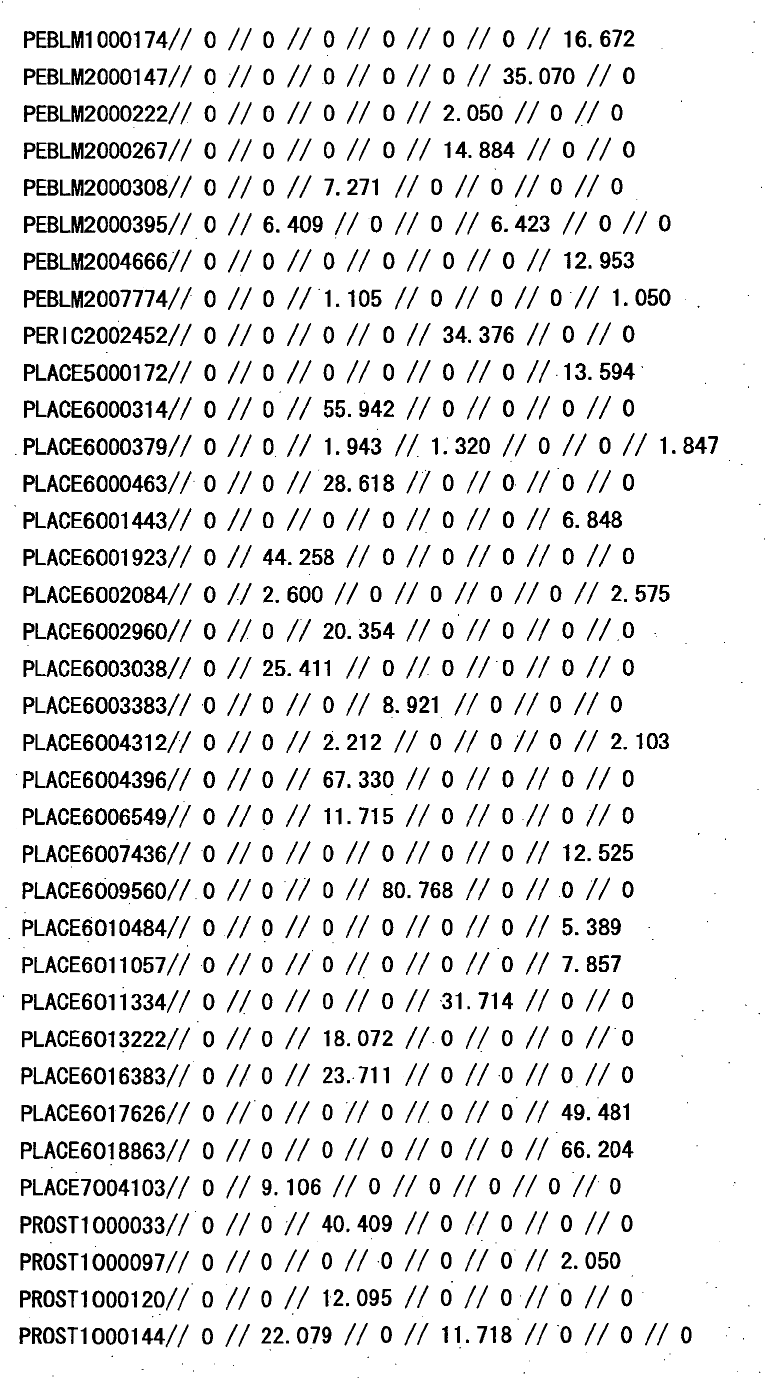

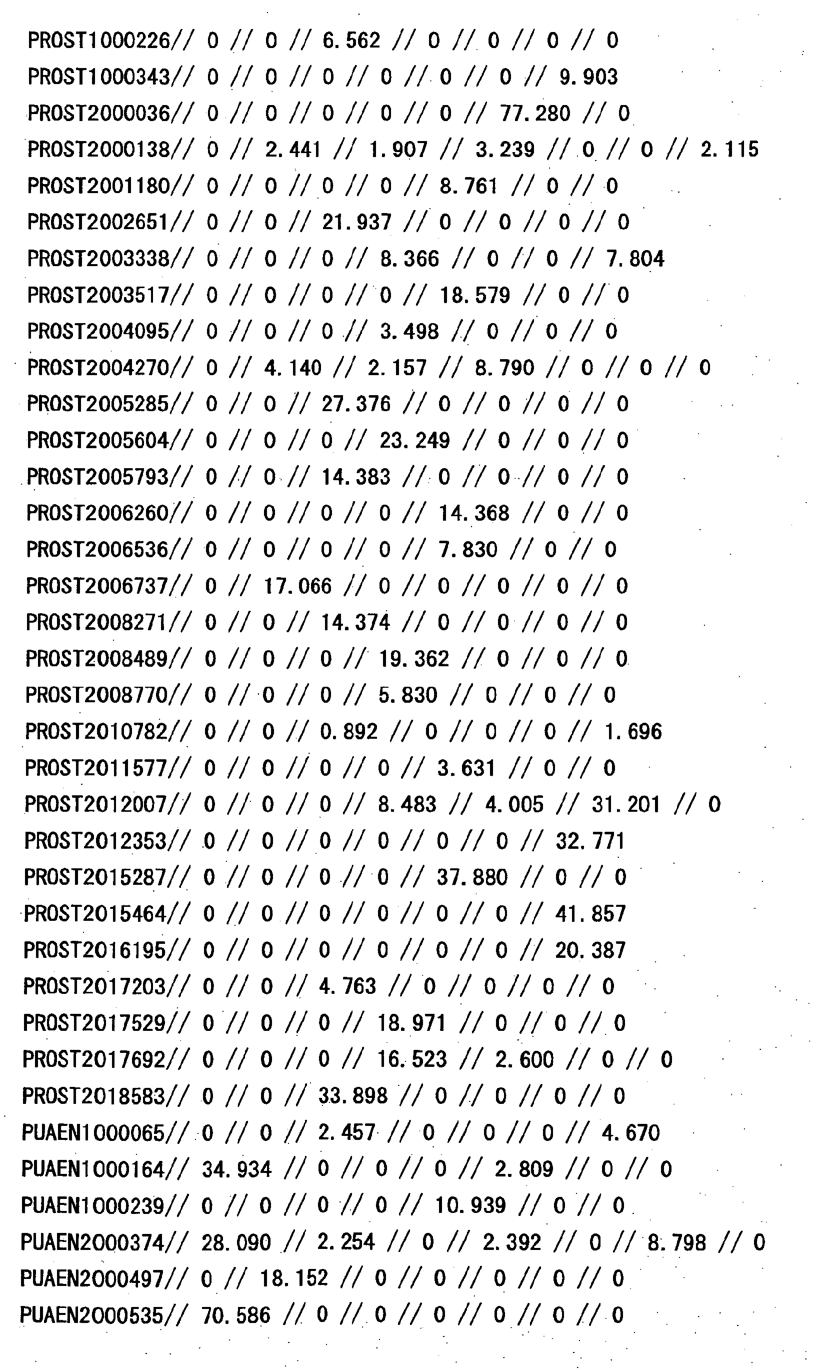

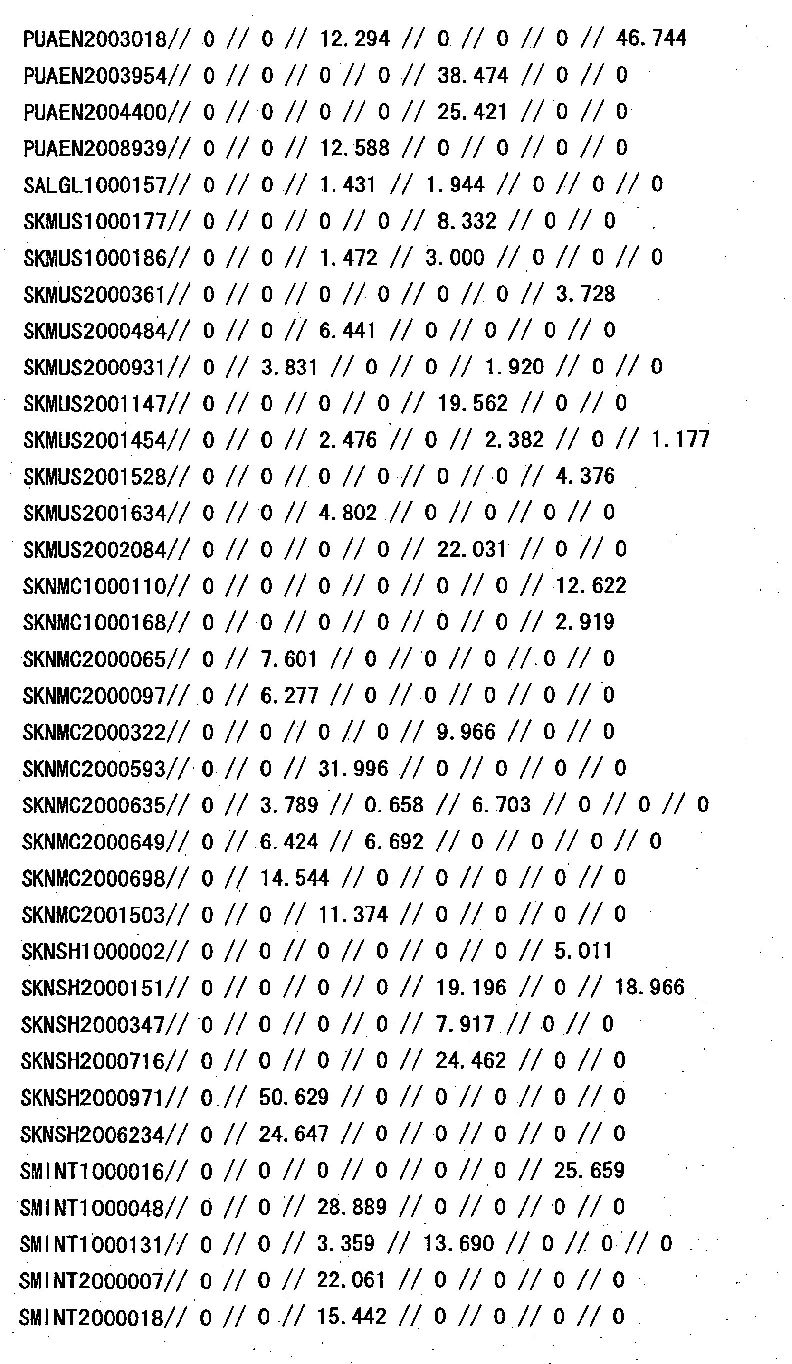

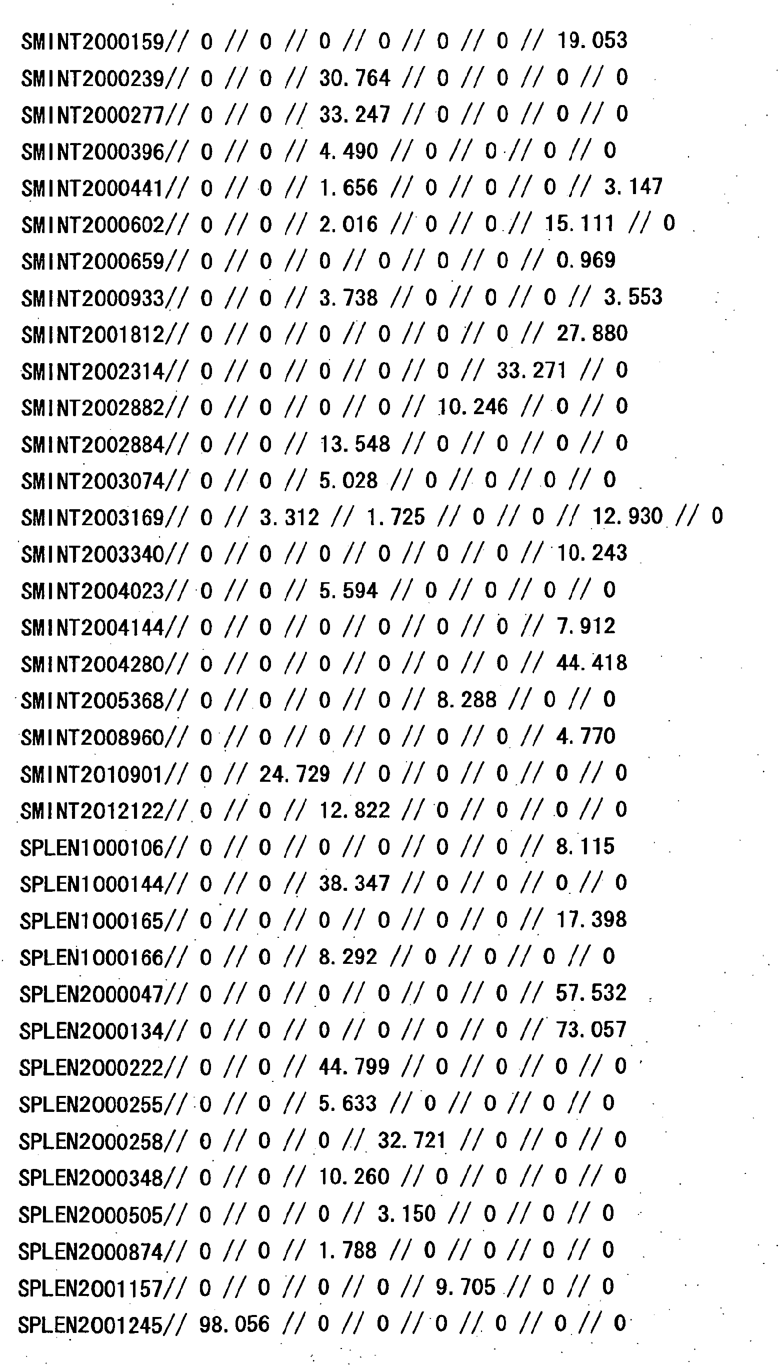

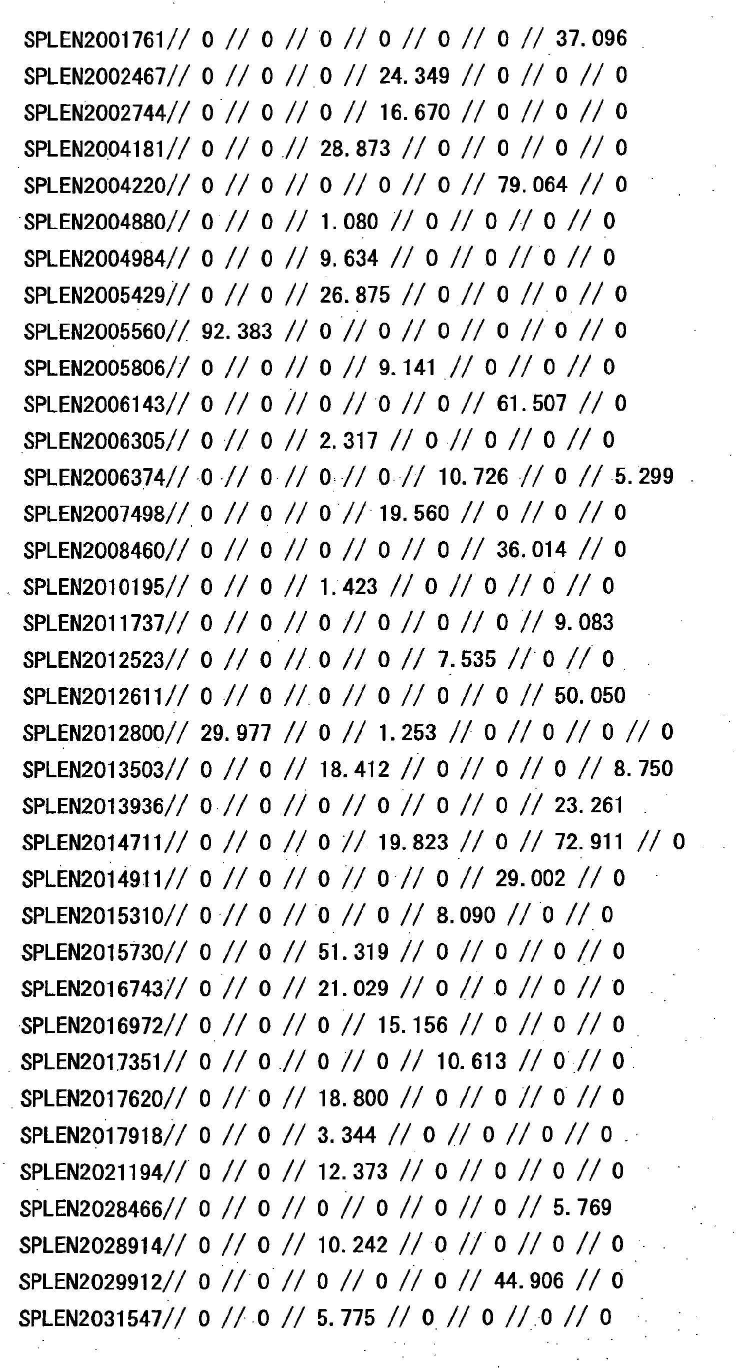

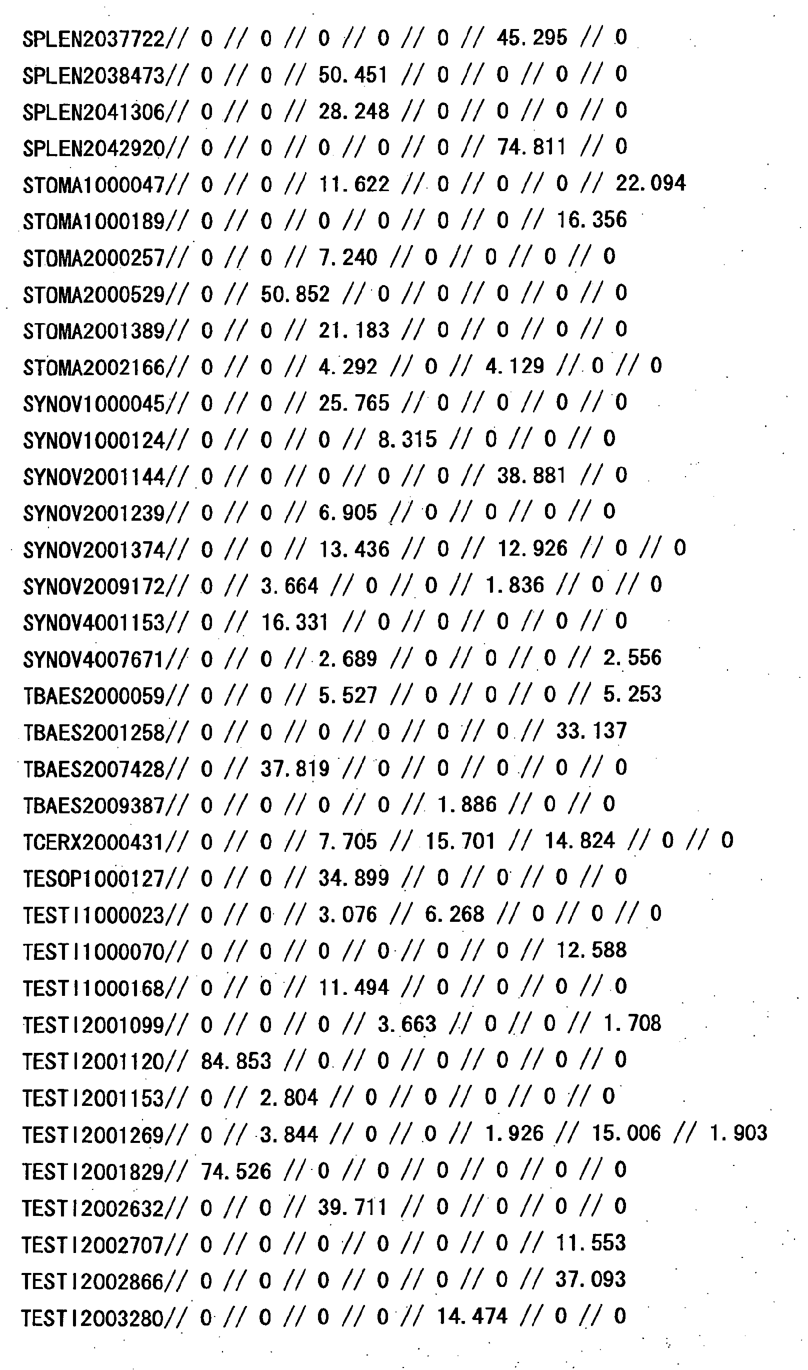

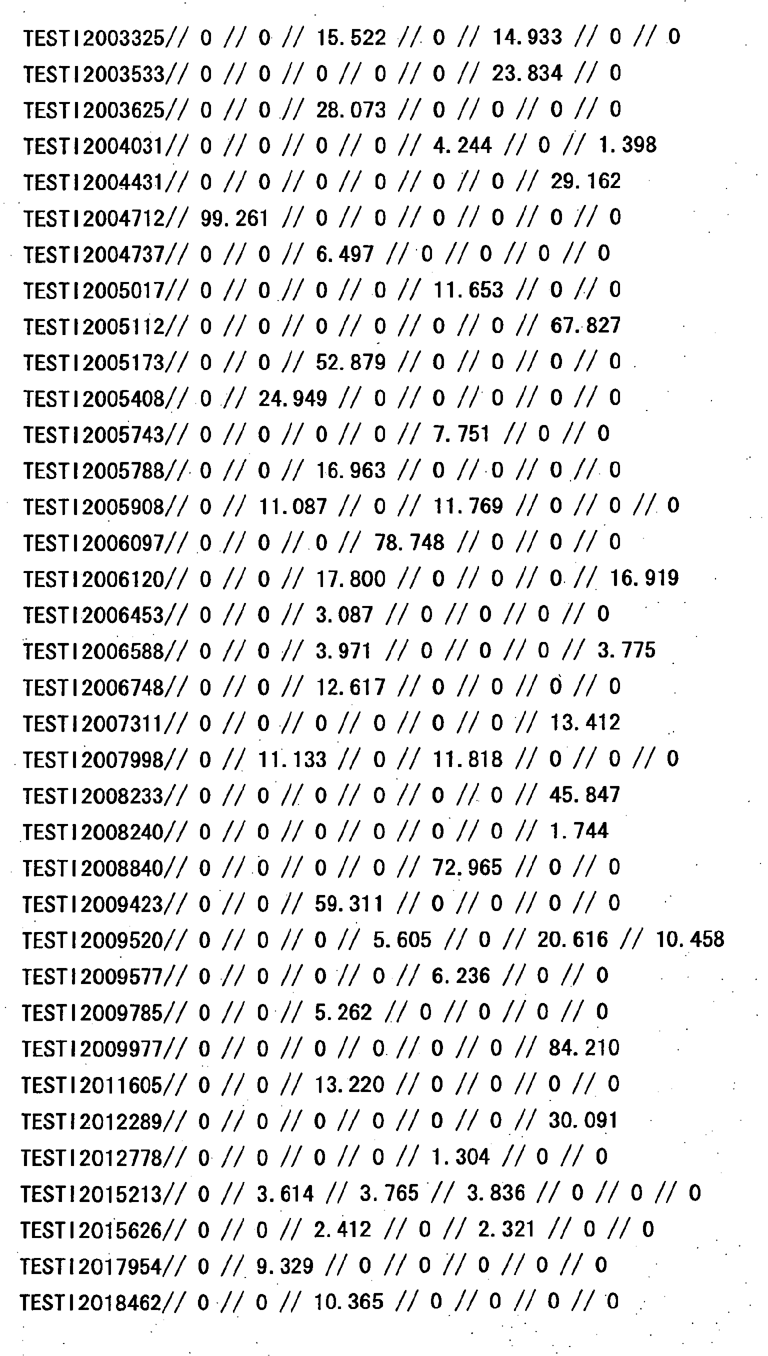

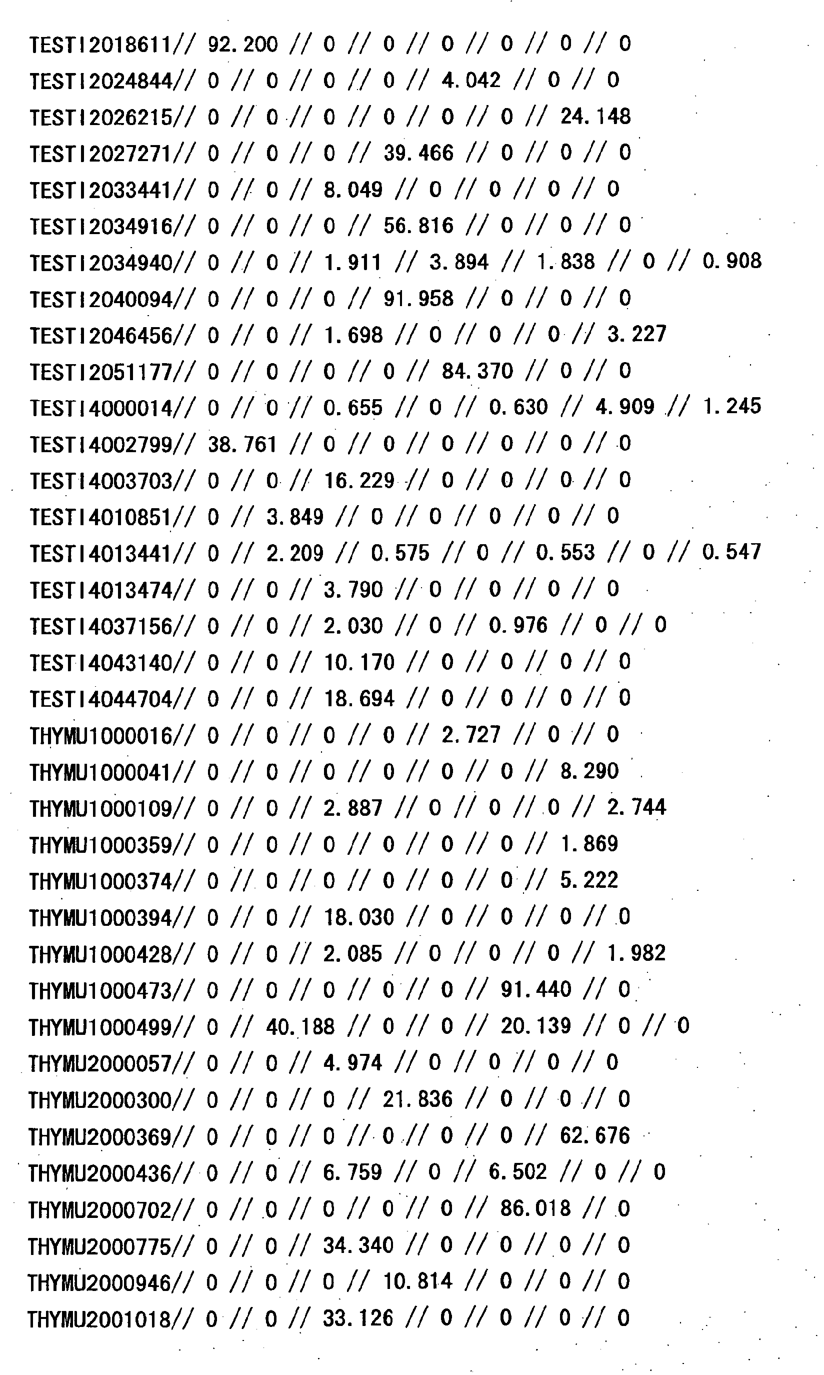

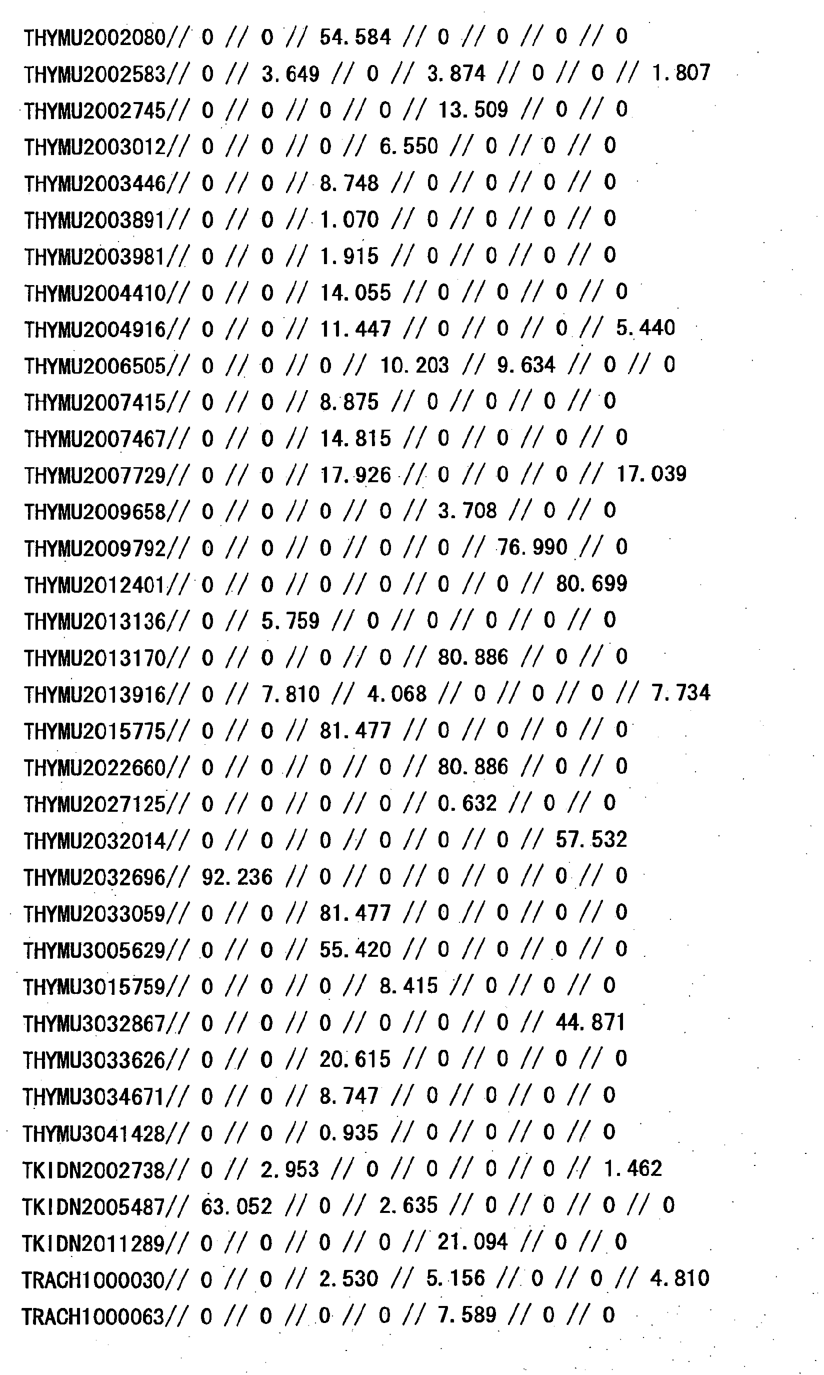

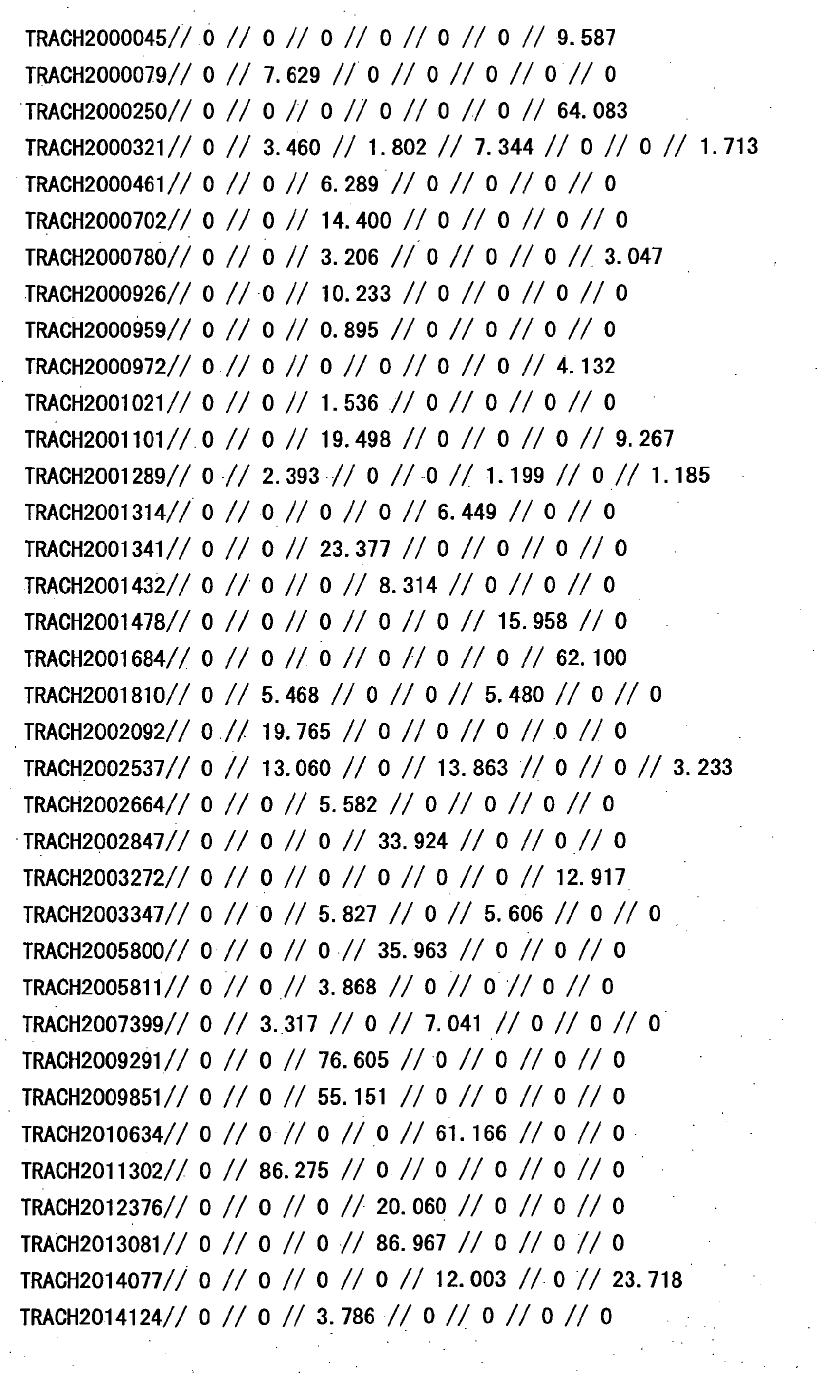

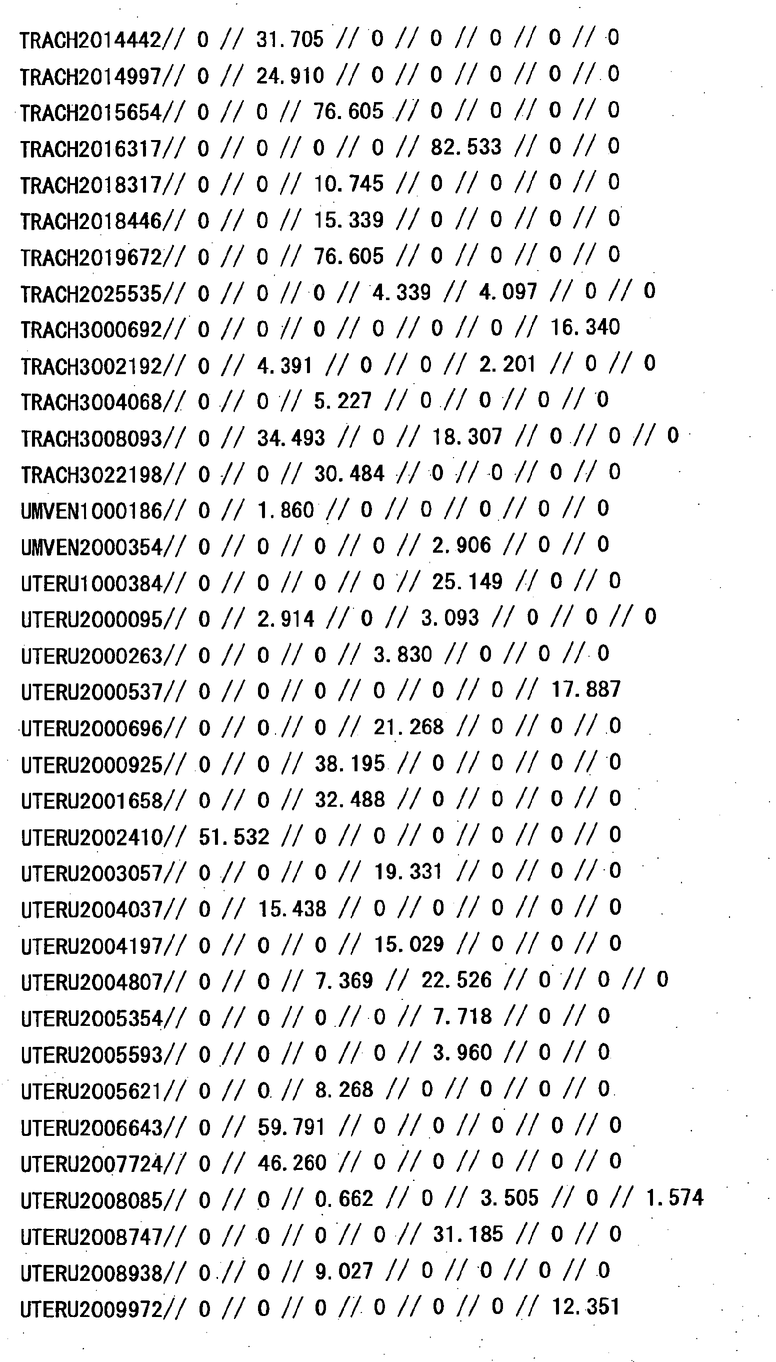

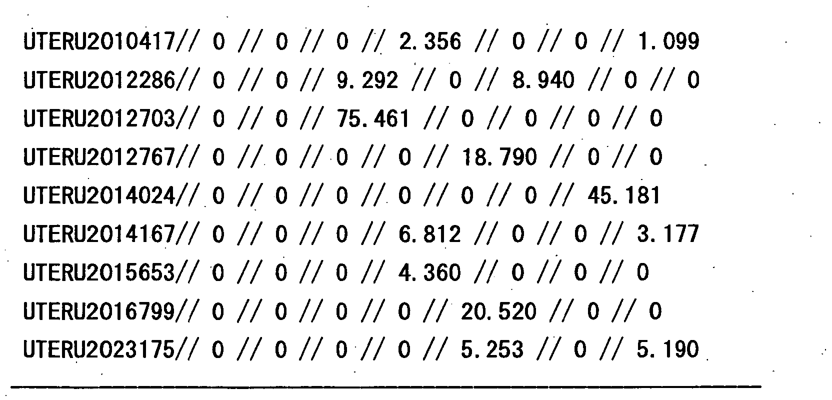

Genes whose expression levels were negligibly low in each of the above-described libraries are shown with their clone names below.
3NB691000018, 3NB691000085, 3NB691000129, 3NB691000191, 3NB692000012, 3NB692000029, 3NB692000102, 3NB692000154, 3NB692000276, 3NB692000305, 3NB692000429, 3NB692000484, 3NB692000529, 3NB692000973, 3NB692001034, 3NB692001040, 3NB692001123, 3NB692001267, 3NB692001339, 3NB692001349, 3NB692001366, 3NB692001408, 3NB692001433, 3NB692001471, 3NB692001496, 3NB692001501, 3NB692001507, 3NB692001519, 3NB692001520, 3NB692001528, 3NB692001538, 3NB692001548, 3NB692001557, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069,
ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000006, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260,
ADRGL2002392, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974,
ADRGL2008023, ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012179, AHMSC1000138, ASTRO1000009, ASTRO1000018, ASTRO1000029, ASTRO1000096, ASTRO2000011, ASTRO2000095, ASTRO2000141, ASTRO2000191, ASTRO2000278, ASTRO2000372, ASTRO2000381, ASTRO2000403, ASTRO2000417, ASTRO2000482, ASTRO2000533, ASTRO2000653, ASTRO2000662, ASTRO2000725, ASTRO2001001, ASTRO2001008, ASTRO2001088, ASTRO2001107, ASTRO2001122, ASTRO2001227, ASTRO2001249, ASTRO2001480, ASTRO2002024, ASTRO2002035, ASTRO2002064,
ASTRO2002202, ASTRO2002571, ASTRO2002632, ASTRO2002659, ASTRO2002693, ASTRO2002720, ASTRO2002733, ASTRO2002743, ASTRO2002842, ASTRO2003212, ASTRO2003316, ASTRO2003461, ASTRO2003574, ASTRO2003581, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005242, ASTRO2005273, ASTRO2005343, ASTRO2005360, ASTRO2005413, ASTRO2005423, ASTRO2005553, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006920,
ASTRO2006952, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561,
ASTRO2014576, ASTRO2014863, ASTRO2014923, ASTRO2014961, ASTRO2015162, ASTRO2015185, ASTRO2015328, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000193, BGGI11000285, BGGI12000022, BGGI12000054, BGGI12000067, BGGI12000161, BGGI12000544, BGGI12000684, BGGI12000693, BGGI12001002, BGGI12001075, BGGI12001241, BGGI12001247, BGGI12001714, BLADE1000176, BLADE2000181, BLADE2000256, BLADE2000340, BLADE2000389, BLADE2000463, BLADE2000492,
BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006849, BLADE2006947, BLADE2007033, BLADE2007043, BLADE2007666, BLADE2007722, BLADE2007735, BLADE2007744,
BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000169, BNGH41000177, BNGH41000190, BNGH41000216, BNGH41000235, BNGH42000132, BNGH42000405, BNGH42000532, BNGH42000675, BNGH42000791, BNGH42000815, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504,
BNGH42005968, BNGH42006135, BNGH42006226, BNGH42006234, BNGH42007037, BNGH42007366, BNGH42007460, BNGH42007594, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020, BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000115, BRACE1000124, BRACE1000132, BRACE1000166, BRACE1000169, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148,
BRACE2000183, BRACE2000229, BRACE2000232, BRACE2000256, BRACE2000266, BRACE2000267, BRACE2000280, BRACE2000300, BRACE2000307, BRACE2000331, BRACE2000332, BRACE2000347, BRACE2000351, BRACE2000368, BRACE2000392, BRACE2000441, BRACE2000487, BRACE2000489, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000893, BRACE2000905, BRACE2000936, BRACE2000947, BRACE2000965, BRACE2000975, BRACE2000988, BRACE2001017, BRACE2001065, BRACE2001070, BRACE2001094, BRACE2001107, BRACE2001143, BRACE2001151,
BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001508, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737, BRACE2001742, BRACE2001777, BRACE2001779, BRACE2001834, BRACE2001859, BRACE2001865, BRACE2001872, BRACE2001881, BRACE2001898, BRACE2001923, BRACE2001942, BRACE2001944,
BRACE2001954, BRACE2001972, BRACE2001973, BRACE2002026, BRACE2002034, BRACE2002050, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002682, BRACE2002695, BRACE2002707, BRACE2002736, BRACE2002752, BRACE2002755, BRACE2002762, BRACE2002772, BRACE2002792,
BRACE2002796, BRACE2002803, BRACE2002807, BRACE2002812, BRACE2002834, BRACE2002849, BRACE2002857, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003825, BRACE2003845, BRACE2003847, BRACE2003848, BRACE2003873, BRACE2003885, BRACE2003887,
BRACE2003905, BRACE2003928, BRACE2003944, BRACE2003954, BRACE2003982, BRACE2003987, BRACE2005034, BRACE2005047, BRACE2005063, BRACE2005083, BRACE2005087, BRACE2005090, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005719, BRACE2005731,
BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006072, BRACE2006075, BRACE2006084, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006264, BRACE2006274, BRACE2006281, BRACE2006298, BRACE2006319, BRACE2006344, BRACE2006354, BRACE2006363, BRACE2006378, BRACE2006380, BRACE2006393, BRACE2006413, BRACE2006417, BRACE2006453, BRACE2006459, BRACE2006488, BRACE2006534,
BRACE2006537, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006909, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007109, BRACE2007138, BRACE2007174, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007411, BRACE2007447, BRACE2007477, BRACE2007502, BRACE2007503, BRACE2007522, BRACE2007527,
BRACE2007538, BRACE2007563, BRACE2007567, BRACE2007621, BRACE2007640, BRACE2007641, BRACE2007646, BRACE2007663, BRACE2007671, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008102, BRACE2008114, BRACE2008144, BRACE2008172, BRACE2008216, BRACE2008218, BRACE2008276, BRACE2008295, BRACE2008308, BRACE2008336, BRACE2008358, BRACE2008361,
BRACE2008399, BRACE2008401, BRACE2008402, BRACE2008410, BRACE2008420, BRACE2008443, BRACE2008473, BRACE2008480, BRACE2008481, BRACE2008488, BRACE2008500, BRACE2008553, BRACE2008594, BRACE2008604, BRACE2008615, BRACE2008622, BRACE2008629, BRACE2008653, BRACE2008655, BRACE2008656, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238,
BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009517, BRACE2009533, BRACE2009558, BRACE2009566, BRACE2009591, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813,
BRACE2010828, BRACE2010837, BRACE2010888, BRACE2010937, BRACE2010983, BRACE2011007, BRACE2011025, BRACE2011183, BRACE2011199, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012062, BRACE2012185, BRACE2012222, BRACE2012232, BRACE2012291, BRACE2012317, BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257,
BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587,
BRACE2017720, BRACE2017844, BRACE2017872, BRACE2017929, BRACE2017934, BRACE2017985, BRACE2017992, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982,
BRACE2021014, BRACE2021143, BRACE2021148, BRACE2021245, BRACE2021254, BRACE2021264, BRACE2021344, BRACE2021433, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022638, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610,
BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849,
BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830,
BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873,
BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113,
BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004205, BRACE3004358, BRACE3004435, BRACE3004477; BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499,
BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708,
BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020,
BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153,
BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537,
BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495,
BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734,
BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157,
BRAMY1000173, BRAMY2000021, BRAMY2000025, BRAMY2000052, BRAMY2000076, BRAMY2000086, BRAMY2000181, BRAMY2000196, BRAMY2000231, BRAMY2000277, BRAMY2000411, BRAMY2000508, BRAMY2000562, BRAMY2000585, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037,
BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004363, BRAMY2004387, BRAMY2004492, BRAMY2004521, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411,
BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2010068, BRAMY2010135, BRAMY2010145,
BRAMY2010171, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881,
BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015925, BRAMY2016002, BRAMY2016009, BRAMY2016020, BRAMY2016070, BRAMY2016251, BRAMY2016327, BRAMY2016386, BRAMY2016706, BRAMY2016771, BRAMY2016892, BRAMY2016938, BRAMY2016953,
BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2019989, BRAMY2020050, BRAMY2020058, BRAMY2020106, BRAMY2020270, BRAMY2020354, BRAMY2020355, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276,
BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024073, BRAMY2024312, BRAMY2024374, BRAMY2024390, BRAMY2024416, BRAMY2024449,
BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717,
BRAMY2027739, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028740, BRAMY2028783, BRAMY2028796, BRAMY2028856, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2032014, BRAMY2032087, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003,
BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484,
BRAMY2038516, BRAMY2038846, BRAMY2038887, BRAMY2038904, BRAMY2039287, BRAMY2039300, BRAMY2039314, BRAMY2039341, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299,
BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004672,
BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652,
BRAMY4001863, BRAMY4001913, BRAMY4002575, BRAMY4002628, BRASW1000053, BRASW1000125, BRAWH1000001, BRAWH1000002, BRAWH1000007, BRAWH1000037, BRAWH1000045, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000443, BRAWH2000471, BRAWH2000476, BRAWH2000482, BRAWH2000488, BRAWH2000494, BRAWH2000500, BRAWH2000503, BRAWH2000554,
BRAWH2000595, BRAWH2000643, BRAWH2000651, BRAWH2000682, BRAWH2000686, BRAWH2000752, BRAWH2000839, BRAWH2000859, BRAWH2000866, BRAWH2000892, BRAWH2000901, BRAWH2000924, BRAWH2000926, BRAWH2000984, BRAWH2001019, BRAWH2001037, BRAWH2001052, BRAWH2001103, BRAWH2001129, BRAWH2001137, BRAWH2001141, BRAWH2001159, BRAWH2001171, BRAWH2001186, BRAWH2001203, BRAWH2001238, BRAWH2001255, BRAWH2001321, BRAWH2001355, BRAWH2001378, BRAWH2001395, BRAWH2001406, BRAWH2001412, BRAWH2001418, BRAWH2001436, BRAWH2001438, BRAWH2001439, BRAWH2001459, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686,
BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003135, BRAWH2003240, BRAWH2003307, BRAWH2003355, BRAWH2003366, BRAWH2003662, BRAWH2003689, BRAWH2003832, BRAWH2003948, BRAWH2003964, BRAWH2004044, BRAWH2004068, BRAWH2004095, BRAWH2004136, BRAWH2004217, BRAWH2004580, BRAWH2004731, BRAWH2004779,
BRAWH2004842, BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005661, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2007069, BRAWH2007308, BRAWH2007406, BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008219, BRAWH2008292, BRAWH2008297, BRAWH2008366, BRAWH2008380,
BRAWH2008540, BRAWH2008577, BRAWH2008706, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762,
BRAWH2011795, BRAWH2011796, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014511, BRAWH2014645, BRAWH2014662, BRAWH2014717, BRAWH2014729, BRAWH2014876, BRAWH2014934, BRAWH2014954, BRAWH2015247, BRAWH2015375, BRAWH2015385, BRAWH2015595, BRAWH2015728, BRAWH2015853, BRAWH2015866,
BRAWH2016028, BRAWH2016106, BRAWH2016160, BRAWH2016166, BRAWH2016209, BRAWH2016221, BRAWH2016223, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017685, BRAWH2017913, BRAWH2018206, BRAWH2018267, BRAWH2018282, BRAWH2018317, BRAWH2018506, BRAWH2018514, BRAWH2018527, BRAWH2018601, BRAWH2018745, BRAWH2018875, BRAWH2019053, BRAWH2019055, BRAWH2019198, BRAWH3000078, BRAWH3000088, BRAWH3000100,
BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760,. BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300,
BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197,
BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023274, BRAWH3023421, BRAWH3024231,
BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114,
BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000763, BRCAN2000832, BRCAN2000923, BRCAN2001223, BRCAN2001866, BRCAN2002416, BRCAN2002473, BRCAN2002562,
BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2009432, BRCAN2010374, BRCAN2010376,
BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653,
BRCAN2019773, BRCAN2019907, BRCAN2020234, BRCAN2020331, BRCAN2020412, BRCAN2020467, BRCAN2020710, BRCAN2020880, BRCAN2021024, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087,
BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769,
BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010510, BRCOC2010730, BRCOC2010980, BRCOC2010994, BRCOC2011010, BRCOC2011398, BRCOC2011418, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934,
BRCOC2020122, BRCOC2020142, BRHIP1000058, BRHIP1000072, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000826, BRHIP2000920, BRHIP2001074, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355,
BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491,
BRHIP2011508, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553,
BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021929, BRHIP2022006, BRHIP2022054, BRHIP2022221, BRHIP2022228, BRHIP2022298, BRHIP2022326, BRHIP2022467, BRHIP2022708,
BRHIP2022986, BRHIP2023071, BRHIP2023229, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176,
BRHIP2029283, BRHIP2029393, BRHIP2029529, BRHIP2029643, BRHIP2029663, BRHIP3000017, BRHIP3000060, BRHIP3000070, BRHIP3000081, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710,
BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181,
BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020046,
BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566,
BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395,
BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768,
BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457,
BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013354, BRSTN2013502, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016918, BRSTN2016992, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063,
BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831,
BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129,
BRTHA2012183, BRTHA2012189, BRTHA2012351, BRTHA2012392, BRTHA2012466, BRTHA2012555, BRTHA2012562, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985,
BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517,
BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490,
BRTHA3003704, BRTHA3003736, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010717, BRTHA3011149, BRTHA3011187, BRTHA3011194, BRTHA3011229, BRTHA3011265, BRTHA3011361, BRTHA3011510, BRTHA3011892,
BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507,
BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318,
COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000056, CTONG1000062, CTONG1000086, CTONG1000088, CTONG1000093, CTONG1000097, CTONG1000113, CTONG1000137, CTONG1000165, CTONG1000180, CTONG1000181, CTONG1000218, CTONG1000220, CTONG1000241, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034,
CTONG2000042, CTONG2000063, CTONG2000201, CTONG2000218, CTONG2000279, CTONG2000303, CTONG2000330, CTONG2000395, CTONG2000411, CTONG2000452, CTONG2000465, CTONG2000480, CTONG2000508, CTONG2000561, CTONG2000589, CTONG2000766, CTONG2000771, CTONG2000819, CTONG2000827, CTONG2000846, CTONG2000855, CTONG2000977, CTONG2001139, CTONG2001320, CTONG2001366, CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001932, CTONG2001955, CTONG2002066, CTONG2002073, CTONG2002095, CTONG2002143, CTONG2002270, CTONG2002395, CTONG2002417, CTONG2002418,
CTONG2002520, CTONG2002558, CTONG2002590, CTONG2002709, CTONG2002721, CTONG2002744, CTONG2002766, CTONG2002803, CTONG2002816, CTONG2002820, CTONG2002832, CTONG2002841, CTONG2002965, CTONG2002999, CTONG2003028, CTONG2003094, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003945, CTONG2004000, CTONG2004062, CTONG2004115, CTONG2004126, CTONG2004264, CTONG2004305, CTONG2004397, CTONG2004423, CTONG2004454, CTONG2004669, CTONG2004716, CTONG2004912, CTONG2004941, CTONG2004948, CTONG2005028,
CTONG2005049, CTONG2005110, CTONG2005145, CTONG2005278, CTONG2005290, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2005913, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006568, CTONG2006620, CTONG2006666, CTONG2006691, CTONG2006709, CTONG2006798, CTONG2006836, CTONG2006942, CTONG2006964, CTONG2007007, CTONG2007009, CTONG2007091, CTONG2007104, CTONG2007168, CTONG2007175, CTONG2007259, CTONG2007272, CTONG2007293, CTONG2007297, CTONG2007399, CTONG2007400, CTONG2007417,
CTONG2007441, CTONG2007474, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008321, CTONG2008343, CTONG2008402, CTONG2008405, CTONG2008466, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604,
CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010408, CTONG2010508, CTONG2010566, CTONG2010623, CTONG2010649, CTONG2010652, CTONG2010803, CTONG2011188, CTONG2011429, CTONG2011474, CTONG2011676, CTONG2011716, CTONG2011765, CTONG2011779, CTONG2011801, CTONG2011815, CTONG2011822, CTONG2011825, CTONG2011920, CTONG2011985, CTONG2012001, CTONG2012050, CTONG2012077, CTONG2012101, CTONG2012123, CTONG2012230, CTONG2012401, CTONG2012422,
CTONG2012425, CTONG2012447, CTONG2012453, CTONG2012473, CTONG2012476, CTONG2012533, CTONG2012554, CTONG2012564, CTONG2012661, CTONG2012724, CTONG2012738, CTONG2012745, CTONG2012758, CTONG2012843, CTONG2012879, CTONG2012901, CTONG2013128, CTONG2013149, CTONG2013156, CTONG2013178, CTONG2013222, CTONG2013284, CTONG2013339, CTONG2013352, CTONG2013381, CTONG2013393, CTONG2013511, CTONG2013630, CTONG2013803, CTONG2013934, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014705, CTONG2014898, CTONG2014946,
CTONG2014954, CTONG2014959, CTONG2014966, CTONG2014995, CTONG2015204, CTONG2015316, CTONG2015330, CTONG2015345, CTONG2015358, CTONG2015434, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016408, CTONG2016499, CTONG2016505, CTONG2016559, CTONG2016658, CTONG2016775, CTONG2016797, CTONG2016846, CTONG2016904, CTONG2016942, CTONG2016953, CTONG2017021, CTONG2017094, CTONG2017292, CTONG2017409, CTONG2017458, CTONG2017500, CTONG2017604, CTONG2017650, CTONG2017798, CTONG2017804, CTONG2017814, CTONG2017910, CTONG2017939, CTONG2017989, CTONG2017998, CTONG2018062,
CTONG2018069, CTONG2018147, CTONG2018211, CTONG2018217, CTONG2018279, CTONG2018343, CTONG2018379, CTONG2018383, CTONG2018413, CTONG2018483, CTONG2018629, CTONG2018632, CTONG2018637, CTONG2018652, CTONG2018653, CTONG2018655, CTONG2018735, CTONG2018739; CTONG2018808, CTONG2018843, CTONG2018898, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020411, CTONG2020445, CTONG2020522, CTONG2020560, CTONG2020582, CTONG2020638, CTONG2020806, CTONG2020831, CTONG2020974,
CTONG2021024, CTONG2021132, CTONG2021168, CTONG2021289, CTONG2022153, CTONG2022601, CTONG2023021, CTONG2023449, CTONG2023512, CTONG2024031, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028208, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127,
CTONG3002370, CTONG3002412, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258,
CTONG3008457, CTONG3008496, CTONG3008566, CTONG3008639, CTONG3008831, CTONG3008951, CTONG3008959, CTONG3009227, CTONG3009287, CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002182, D3OST2002223, D3OST2002262, D3OST2002272, D3OST2002417, D3OST2002436, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000358, D6OST2000507, D9OST2000278, D9OST2000440, D9OST2000869, D9OST2001319,
D9OST2001433, D9OST2002397, D9OST2003106, D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000083, DFNES1000107, DFNES1000185, DFNES2000143, DFNES2000146, DFNES2000212, DFNES2000292, DFNES2000335, DFNES2000426, DFNES2000432, DFNES2000443, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002550, DFNES2002588, DFNES2002817, DFNES2002966, DFNES2003081, DFNES2003192, DFNES2003255, DFNES2003742, DFNES2004346,
DFNES2004354, DFNES2004383, DFNES2004608, DFNES2004684, DFNES2005266, DFNES2005527, DFNES2005540, DFNES2005690, DFNES2005779, DFNES2005875, DFNES2006346, DFNES2006944, DFNES2007113, DFNES2007299, DFNES2007332, DFNES2007634, DFNES2007641, DFNES2007757, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000024, FCBBF1000025, FCBBF1000038, FCBBF1000053, FCBBF1000061, FCBBF1000063, FCBBF1000069, FCBBF1000077, FCBBF1000115, FCBBF1000117, FCBBF1000118, FCBBF1000125, FCBBF1000131, FCBBF1000139, FCBBF1000155, FCBBF1000157, FCBBF1000171, FCBBF1000174,
FCBBF1000197, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000367, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000476, FCBBF1000509, FCBBF1000546, FCBBF1000550, FCBBF1000556, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF2000038, FCBBF2000087, FCBBF2000094, FCBBF2000105,
FCBBF2000123, FCBBF2000195, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000591, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001001, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370,
FCBBF2002541, FCBBF2002626, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302,
FCBBF2007556, FCBBF2007610, FCBBF2007633, FCBBF2007840, FCBBF3000102, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000462, FCBBF3000473, FCBBF3000501, FCBBF3000518, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925,
FCBBF3003216, FCBBF3003305, FCBBF3003373, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007855, FCBBF3007859, FCBBF3007860, FCBBF3008073, FCBBF3008100, FCBBF3008153, FCBBF3008159, FCBBF3008251,
FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009541, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889,
FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016178, FCBBF3016451, FCBBF3016618, FCBBF3016622, FCBBF3016644, FCBBF3016667, FCBBF3016928, FCBBF3016987, FCBBF3017024, FCBBF3017059, FCBBF3017071, FCBBF3017123, FCBBF3017233, FCBBF3017255, FCBBF3017288,
FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894,
FCBBF3023061, FCBBF3023078, FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202,
FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000057, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000220, FEBRA2000253, FEBRA2000282, FEBRA2000286,
FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452, FEBRA2000454, FEBRA2000462, FEBRA2000468, FEBRA2000491, FEBRA2000504, FEBRA2000532, FEBRA2000538, FEBRA2000575, FEBRA2000581, FEBRA2000656, FEBRA2000659, FEBRA2000680, FEBRA2000690, FEBRA2000727, FEBRA2000734, FEBRA2000740, FEBRA2000757, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000909,
FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001294, FEBRA2001334, FEBRA2001351, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001828, FEBRA2001867, FEBRA2001914, FEBRA2001974,
FEBRA2001989, FEBRA2001990, FEBRA2002009, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002527, FEBRA2002552, FEBRA2002574, FEBRA2002601, FEBRA2002611, FEBRA2002628, FEBRA2002662, FEBRA2002682, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002933, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436,
FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003750, FEBRA2003822, FEBRA2003833, FEBRA2003897, FEBRA2003907, FEBRA2004023, FEBRA2004026, FEBRA2004029, FEBRA2004042, FEBRA2004053, FEBRA2004056, FEBRA2004091, FEBRA2004110, FEBRA2004191, FEBRA2004202, FEBRA2004219, FEBRA2004237, FEBRA2004268, FEBRA2004293, FEBRA2004329, FEBRA2004331, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004840, FEBRA2004852, FEBRA2004867, FEBRA2004914, FEBRA2004933, FEBRA2004987, FEBRA2005014, FEBRA2005036, FEBRA2005079,
FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005572, FEBRA2005701, FEBRA2005726, FEBRA2005734, FEBRA2005787, FEBRA2005788, FEBRA2005824, FEBRA2005995, FEBRA2005998, FEBRA2006061, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006379, FEBRA2006396, FEBRA2006401, FEBRA2006409, FEBRA2006418, FEBRA2006427, FEBRA2006476, FEBRA2006491, FEBRA2006519, FEBRA2006521, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890,
FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007622, FEBRA2007708, FEBRA2007714, FEBRA2007793, FEBRA2007818, FEBRA2007823, FEBRA2007900, FEBRA2007901, FEBRA2007931, FEBRA2007957, FEBRA2008009, FEBRA2008086, FEBRA2008134, FEBRA2008135, FEBRA2008159, FEBRA2008165, FEBRA2008172, FEBRA2008201, FEBRA2008210, FEBRA2008265, FEBRA2008266, FEBRA2008282, FEBRA2008287, FEBRA2008311, FEBRA2008341, FEBRA2008360, FEBRA2008459, FEBRA2008468, FEBRA2008475, FEBRA2008481, FEBRA2008542,
FEBRA2008583, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746,
FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200,
FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255,
FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366,
FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154,
HCASM2008536, HCASM2009405, HCASM2009424, HCASM2009463, HCASM2009588, HCHON1000015, HCHON1000131, HCHON1000142, HCHON1000166, HCHON1000176, HCHON2000028, HCHON2000038, HCHON2000062, HCHON2000100, HCHON2000156, HCHON2000199, HCHON2000212, HCHON2000271, HCHON2000295, HCHON2000315, HCHON2000322, HCHON2000323, HCHON2000344, HCHON2000373, HCHON2000395, HCHON2000417, HCHON2000418, HCHON2000473, HCHON2000503, HCHON2000660, HCHON2000663, HCHON2000673, HCHON2000676, HCHON2000698, HCHON2000699, HCHON2000705, HCHON2000743, HCHON2000751, HCHON2000815, HCHON2000817, HCHON2000818, HCHON2000819, HCHON2000832, HCHON2000851, HCHON2000935, HCHON2000942, HCHON2000956, HCHON2000994, HCHON2001084, HCHON2001099,
HCHON2001110, HCHON2001116, HCHON2001161, HCHON2001200, HCHON2001214, HCHON2001217, HCHON2001233, HCHON2001269, HCHON2001450, HCHON2001453, HCHON2001497, HCHON2001519, HCHON2001523, HCHON2001535, HCHON2001548, HCHON2001577, HCHON2001598, HCHON2001604, HCHON2001607, HCHON2001646, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118,
HCHON2005166, HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000088, HEART1000099, HEART1000102, HEART1000118, HEART1000132, HEART1000139, HEART1000142, HEART1000151, HEART1000173, HEART2000024, HEART2000035, HEART2000099, HEART2000298, HEART2000306, HEART2000309, HEART2000411,
HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003781, HEART2003836, HEART2003852, HEART2004570, HEART2004764, HEART2004931, HEART2004940, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443, HEART2007696, HEART2007767, HEART2008108,
HEART2008111, HEART2008257, HEART2008509, HEART2008994, HEART2009000, HEART2009599, HEART2009680, HEART2010391, HEART2010492, HEART2010495, LIVER1000017, LIVER1000050, LIVER1000055, LIVER1000058, LIVER1000063, LIVER1000067, LIVER1000073, LIVER1000082, LIVER1000099, LIVER1000111, LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769,
LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2003008, LIVER2003065, LIVER2003234, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415,
LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000178; MAMGL1000182, MAMGL1000184, MESAN1000032, MESAN1000035, MESAN1000050, MESAN1000079, MESAN1000080, MESAN1000101, MESAN1000121, MESAN1000126, MESAN1000147, MESAN1000180, MESAN2000023, MESAN2000026, MESAN2000067, MESAN2000092, MESAN2000149, MESAN2000167, MESAN2000291, MESAN2000434, MESAN2000457, MESAN2000462, MESAN2000501, MESAN2000620, MESAN2000711,
MESAN2000815, MESAN2000894, MESAN2000909, MESAN2001154, MESAN2001450, MESAN2001627, MESAN2001770, MESAN2001979, MESAN2002113, MESAN2002122, MESAN2002147, MESAN2002186, MESAN2002424, MESAN2002687, MESAN2002709, MESAN2002790, MESAN2002844, MESAN2002940, MESAN2002978, ME,SAN2003035, MESAN2003037, MESAN2003039, MESAN2003058, MESAN2003101, MESAN2003190, MESAN2003322, MESAN2003444, MESAN2003490, MESAN2003529, MESAN2003646, MESAN2003662, MESAN2003709, MESAN2003851, MESAN2004138, MESAN2004288, MESAN2004575, MESAN2005303, MESAN2005371, MESAN2005633, MESAN2005724, MESAN2005808, MESAN2005811, MESAN2005957, MESAN2005958, MESAN2006022, MESAN2006043, MESAN2006328, MESAN2006401, MESAN2006580, MESAN2006599,
MESAN2006743, MESAN2006953, MESAN2007032, MESAN2007440, MESAN2007597, MESAN2007755, MESAN2008222, MESAN2008334, MESAN2008415, MESAN2008460, MESAN2008536, MESAN2008729, MESAN2008821, MESAN2008926, MESAN2008936, MESAN2009019, MESAN2009081, MESAN2009105, MESAN2009311, MESAN2009418, MESAN2009503, MESAN2009522, MESAN2009580, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013284, MESAN2013611, MESAN2013674, MESAN2013908, MESAN2013936,
MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000011, NB9N41000047, NB9N41000121, NB9N41000135, NB9N41000142, NB9N41000146, NB9N41000340, NB9N42000104, NB9N42000122, NB9N42000196, NB9N42000281, NB9N42000314, NB9N42000342, NB9N42000430, NB9N42000495, NB9N42001300, NCRRP1000129, NESOP1000010, NESOP1000087, NESOP1000108,
NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000102, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000014, NT2NE1000018, NT2NE1000023,
NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000056, NT2NE2000057, NT2NE2000064, NT2NE2000109, NT2NE2000137, NT2NE2000156, NT2NE2000222, NT2NE2000259, NT2NE2000284, NT2NE2000299, NT2NE2000327, NT2NE2000353, NT2NE2000356, NT2NE2000369, NT2NE2000374, NT2NE2000383, NT2NE2000384, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000809, NT2NE2000864, NT2NE2000909,
NT2NE2000913, NT2NE2000950, NT2NE2000951, NT2NE2000980, NT2NE2001005, NT2NE2001021, NT2NE2001040, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001442, NT2NE2001524, NT2NE2001545, NT2NE2001598, NT2NE2001617, NT2NE2001623, NT2NE2001626, NT2NE2001634, NT2NE2001648, NT2NE2001660, NT2NE2001666, NT2NE2001677, NT2NE2001697, NT2NE2001698, NT2NE2001723, NT2NE2001725, NT2NE2001793, NT2NE2001874, NT2NE2001889, NT2NE2001923, NT2NE2002100, NT2NE2002162, NT2NE2002186, NT2NE2002285, NT2NE2002311,
NT2NE2002620, NT2NE2002651, NT2NE2002732, NT2NE2002768, NT2NE2002791, NT2NE2002856, NT2NE2003150, NT2NE2003185, NT2NE2003252, NT2NE2003280, NT2NE2003308, NT2NE2003309, NT2NE2003315, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004606, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005688, NT2NE2005720, NT2NE2005784, NT2NE2005804, NT2NE2005821,
NT2NE2005876, NT2NE2005890, NT2NE2005921, NT2NE2005968, NT2NE2006075, NT2NE2006087, NT2NE2006217, NT2NE2006288, NT2NE2006382, NT2NE2006387, NT2NE2006427, NT2NE2006432, NT2NE2006458, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608,
NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012847, NT2NE2012928, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221,
NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916,
NT2NE2019056, NT2NE2019091, NT2NE2019099, NT2RI1000016, NT2RI1000035, NT2RI1000048, NT2RI1000164, NT2RI1000172, NT2RI2000004, NT2RI2000007, NT2RI2000116, NT2RI2000117, NT2RI2000128, NT2RI2000133, NT2RI2000167, NT2RI2000173, NT2RI2000187, NT2RI2000255, NT2RI2000270, NT2RI2000276, NT2RI2000294, NT2RI2000313, NT2RI2000341, NT2RI2000344, NT2RI2000348, NT2RI2000412, NT2RI2000421, NT2RI2000480, NT2RI2000575, NT2RI2000588, NT2RI2000597, NT2RI2000669, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000987, NT2RI2001010, NT2RI2001017, NT2RI2001030, NT2RI2001083, NT2RI2001091, NT2RI2001167, NT2RI2001178, NT2RI2001230, NT2RI2001235, NT2RI2001244, NT2RI2001307, NT2RI2001342, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001726, NT2RI2001769, NT2RI2001782, NT2RI2001836, NT2RI2001866, NT2RI2001952, NT2RI2002022, NT2RI2002041, NT2RI2002117, NT2RI2002120, NT2RI2002153, NT2RI2002179, NT2RI2002243, NT2RI2002252, NT2RI2002260, NT2RI2002270, NT2RI2002334, NT2RI2002359, NT2RI2002447, NT2RI2002481, NT2RI2002507, NT2RI2002517, NT2RI2002530, NT2RI2002540, NT2RI2002549, NT2RI2002564, NT2RI2002585, NT2RI2002592, NT2RI2002602, NT2RI2002654, NT2RI2002699, NT2RI2002802, NT2RI2002819, NT2RI2002822,
NT2RI2002847, NT2RI2002852, NT2RI2002865, NT2RI2002923, NT2RI2002926, NT2RI2002958, NT2RI2002970, NT2RI2003011, NT2RI2003019, NT2RI2003051, NT2RI2003067, NT2RI2003205, NT2RI2003222, NT2RI2003301, NT2RI2003338, NT2RI2003360, NT2RI2003361, NT2RI2003383, NT2RI2003392, NT2RI2003399, NT2RI2003407, NT2RI2003420, NT2RI2003481, NT2RI2003487, NT2RI2003556, NT2RI2003655, NT2RI2003667, NT2RI2003678, NT2RI2003751, NT2RI2003884, NT2RI2003973, NT2RI2004059, NT2RI2004078, NT2RI2004079, NT2RI2004093, NT2RI2004136, NT2RI2004157, NT2RI2004188, NT2RI2004190, NT2RI2004284, NT2RI2004290, NT2RI2004304, NT2RI2004381, NT2RI2004398, NT2RI2004410, NT2RI2004442, NT2RI2004468, NT2RI2004606, NT2RI2004618, NT2RI2004783,
NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005116, NT2RI2005130, NT2RI2005166, NT2RI2005239, NT2RI2005246, NT2RI2005315, NT2RI2005353, NT2RI2005358, NT2RI2005382, NT2RI2005473, NT2RI2005520, NT2RI2005564, NT2RI2005579, NT2RI2005621, NT2RI2005628, NT2RI2005647, NT2RI2005670, NT2RI2005710, NT2RI2005772, NT2RI2005811, NT2RI2005816, NT2RI2005851, NT2RI2005966, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487, NT2RI2006506, NT2RI2006640,
NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006825, NT2RI2006838, NT2RI2006855, NT2RI2006943, NT2RI2006973, NT2RI2007046, NT2RI2007048, NT2RI2007096, NT2RI2007116, NT2RI2007148, NT2RI2007214, NT2RI2007277, NT2RI2007303, NT2RI2007334, NT2RI2007384, NT2RI2007386, NT2RI2007406, NT2RI2007439, NT2RI2007445, NT2RI2007468, NT2RI2007469, NT2RI2007507, NT2RI2007589, NT2RI2007723, NT2RI2007729, NT2RI2007751, NT2RI2007754, NT2RI2007827, NT2RI2007853, NT2RI2007877, NT2RI2007879, NT2RI2007891, NT2RI2007956, NT2RI2007965, NT2RI2008007, NT2RI2008050, NT2RI2008141, NT2RI2008171,
NT2RI2008188, NT2RI2008204, NT2RI2008221, NT2RI2008233, NT2RI2008455, NT2RI2008502, NT2RI2008566, NT2RI2008598, NT2RI2008622, NT2RI2008656, NT2RI2008714, NT2RI2008724, NT2RI2008791, NT2RI2008801, NT2RI2008808, NT2RI2008812, NT2RI2008841, NT2RI2008942, NT2RI2008952, NT2RI2009005, NT2RI2009083, NT2RI2009101, NT2RI2009103, NT2RI2009144, NT2RI2009151, NT2RI2009215, NT2RI2009233, NT2RI2009259, NT2RI2009269, NT2RI2009281, NT2RI2009289, NT2RI2009301, NT2RI2009402, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373,
NT2RI2014247, NT2RI2014551, NT2RI2014683, NT2RI2014733, NT2RI2015239, NT2RI2015533, NT2RI2016128, NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564,. NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213,
NT2RI3000423, NT2RI3000622, NT2RI3001132, NT2RI3001263, NT2RI3001279, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757,
NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000008, NT2RP6000017, NT2RP6000032, NT2RP6000033, NT2RP6000039, NT2RP6000043, NT2RP6000059, NT2RP6000060, NT2RP6000077, NT2RP6000085, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000069, NT2RP7000086, NT2RP7000109, NT2RP7000112, NT2RP7000238, NT2RP7000259, NT2RP7000311, NT2RP7000359, NT2RP7000364, NT2RP7000380, NT2RP7000391, NT2RP7000425, NT2RP7000579, NT2RP7000586, NT2RP7000624, NT2RP7000812, NT2RP7000926,
NT2RP7001074, NT2RP7001080, NT2RP7001156, NT2RP7001231, NT2RP7001306, NT2RP7001319, NT2RP7001335, NT2RP7001364, NT2RP7001475, NT2RP7001532, NT2RP7001591, NT2RP7001602, NT2RP7001678, NT2RP7001856, NT2RP7002028, NT2RP7002064, NT2RP7002151, NT2RP7002163, NT2RP7002243, NT2RP7002282, NT2RP7002376, NT2RP7002379, NT2RP7002450, NT2RP7002478, NT2RP7002554, NT2RP7002594, NT2RP7002603, NT2RP7002619, NT2RP7002650, NT2RP7002738, NT2RP7002779, NT2RP7002802, NT2RP7002841, NT2RP7002875, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203, NT2RP7003261, NT2RP7003319, NT2RP7003439, NT2RP7003511,
NT2RP7003629, NT2RP7003632, NT2RP7003647, NT2RP7003680, NT2RP7003724, NT2RP7003960, NT2RP7004027, NT2RP7004080, NT2RP7004114, NT2RP7004123, NT2RP7004173, NT2RP7004196, NT2RP7004204, NT2RP7004233, NT2RP7004260, NT2RP7004352, NT2RP7004358, NT2RP7004396, NT2RP7004481, NT2RP7004541, NT2RP7004559, NT2RP7004641, NT2RP7004656, NT2RP7004687, NT2RP7004722, NT2RP7004766, NT2RP7004790, NT2RP7004915, NT2RP7004925, NT2RP7004961, NT2RP7004964, NT2RP7004975, NT2RP7005118, NT2RP7005205, NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005750, NT2RP7005846, NT2RP7006033, NT2RP7006075, NT2RP7006160, NT2RP7006162,
NT2RP7006188, NT2RP7006223, NT2RP7006263, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006619, NT2RP7006621, NT2RP7006636, NT2RP7006701, NT2RP7006853, NT2RP7006887, NT2RP7006980, NT2RP7006995, NT2RP7007114, NT2RP7007221, NT2RP7007226, NT2RP7007252, NT2RP7007269, NT2RP7007310, NT2RP7007359, NT2RP7007381, NT2RP7007406, NT2RP7007480, NT2RP7007504, NT2RP7007530, NT2RP7007537, NT2RP7007580, NT2RP7007594, NT2RP7007617, NT2RP7007643, NT2RP7007766, NT2RP7007842, NT2RP7007925, NT2RP7007930, NT2RP7007975, NT2RP7008013, NT2RP7008133, NT2RP7008137, NT2RP7008142, NT2RP7008144, NT2RP7008161, NT2RP7008167,
NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009097, NT2RP7009108, NT2RP7009147, NT2RP7009149, NT2RP7009168, NT2RP7009215, NT2RP7009225, NT2RP7009236, NT2RP7009253, NT2RP7009259, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009482, NT2RP7009498, NT2RP7009502, NT2RP7009507, NT2RP7009577, NT2RP7009743, NT2RP7009867,
NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365,
NT2RP7017474, NT2RP7017546, NT2RP7017567, NT2RP7017795, NT2RP7017971, NT2RP7018032, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000033,
NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000247, NTONG1000264, NTONG2000040, NTONG2000107, NTONG2000231, NTONG2000265, NTONG2000334, NTONG2000363, NTONG2000413, NTONG2000492, NTONG2000499, NTONG2000531, NTONG2000583, NTONG2000858, NTONG2000876, NTONG2000878, NTONG2000966, NTONG2000977, NTONG2000985, NTONG2001014, NTONG2001079, NTONG2001157, NTONG2001173, NTONG2001220, NTONG2001222, NTONG2001362, NTONG2001428, NTONG2001532, NTONG2001550, NTONG2001587, NTONG2001619, NTONG2001642, NTONG2001657, NTONG2001762, NTONG2002278, NTONG2002582, NTONG2002807, NTONG2002948, NTONG2002970,
NTONG2002985, NTONG2003158, NTONG2003210, NTONG2003316, NTONG2003409, NTONG2003454, NTONG2003515, NTONG2003805, NTONG2003852, NTONG2003897, NTONG2003928, NTONG2004095, NTONG2004308, NTONG2004521, NTONG2004614, NTONG2004690, NTONG2004806, NTONG2004829, NTONG2004844, NTONG2004991, NTONG2005062, NTONG2005086, NTONG2005137, NTONG2005153, NTONG2005265, NTONG2005277, NTONG2005363, NTONG2005373, NTONG2005391, NTONG2005480, NTONG2005497, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028,
NTONG2007034, NTONG2007249, NTONG2007517, NTONG2007522, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000118, OCBBF1000119, OCBBF1000122, OCBBF1000130, OCBBF1000138, OCBBF1000145, OCBBF1000146, OCBBF1000153, OCBBF1000175, OCBBF1000185, OCBBF1000192, OCBBF1000254, OCBBF1000315, OCBBF2000013, OCBBF2000015, OCBBF2000126, OCBBF2000178, OCBBF2000231, OCBBF2000277,
OCBBF2000287, OCBBF2000323, OCBBF2000452, OCBBF2000677, OCBBF2000703, OCBBF2000712, OCBBF2000719, OCBBF2000831, OCBBF2000904, OCBBF2000982, OCBBF2000998, OCBBF2001075, OCBBF2001101, OCBBF2001124, OCBBF2001140, OCBBF2001166, OCBBF2001176, OCBBF2001186, OCBBF2001196, OCBBF2001210, OCBBF2001252, OCBBF2001307, OCBBF2001323, OCBBF2001389, OCBBF2001402, OCBBF2001408, OCBBF2001416, OCBBF2001473, OCBBF2001492, OCBBF2001527, OCBBF2001528, OCBBF2001639, OCBBF2001681, OCBBF2001706, OCBBF2001749, OCBBF2001794, OCBBF2001910, OCBBF2001931, OCBBF2001961, OCBBF2001983, OCBBF2002015, OCBBF2002086, OCBBF2002124, OCBBF2002161, OCBBF2002290, OCBBF2002357, OCBBF2002376, OCBBF2002615, OCBBF2002626, OCBBF2002656,
OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038, OCBBF2004104, OCBBF2004147, OCBBF2004168, OCBBF2004235, OCBBF2004259, OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843,
OCBBF2005901, OCBBF2005956, OCBBF2006005, OCBBF2006030, OCBBF2006058, OCBBF2006113, OCBBF2006151, OCBBF2006154, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007354, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007892, OCBBF2007931, OCBBF2007946, OCBBF2008005, OCBBF2008116,
OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008586, OCBBF2008640, OCBBF2008691, OCBBF2008701, OCBBF2008724, OCBBF2008768, OCBBF2008770, OCBBF2008775, OCBBF2008790, OCBBF2008814, OCBBF2008822, OCBBF2009095, OCBBF2009115, OCBBF2009242, OCBBF2009301, OCBBF2009352, OCBBF2009391, OCBBF2009424, OCBBF2009536, OCBBF2009571, OCBBF2009583, OCBBF2009603, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010841, OCBBF2010843,
OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011177, OCBBF2011228, OCBBF2011232, OCBBF2011283, OCBBF2011311, OCBBF2011625, OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011,
OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013883, OCBBF2013926, OCBBF2014052, OCBBF2014089, OCBBF2014322, OCBBF2014386, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612,
OCBBF2016689, OCBBF2016690, OCBBF2016729, OCBBF2016841, OCBBF2016928, OCBBF2017055, OCBBF2017069, OCBBF2017306, OCBBF2017325, OCBBF2017398, OCBBF2017458, OCBBF2017489, OCBBF2017516, OCBBF2017665, OCBBF2017754, OCBBF2017791, OCBBF2017815, OCBBF2017882, OCBBF2017888, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018827, OCBBF2018828, OCBBF2018873, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741,
OCBBF2020801, OCBBF2020825, OCBBF2020838, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173,
OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598,
OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066,
PEBLM1000068, PEBLM1000071, PEBLM1000083, PEBLM1000144, PEBLM1000147, PEBLM1000180, PEBLM2000030, PEBLM2000112, PEBLM2000126, PEBLM2000170, PEBLM2000180, PEBLM2000213, PEBLM2000248, PEBLM2000270, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215,
PEBLM2006283, PEBLM2006298, PEBLM2006366, PEBLM2006709, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957,
PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000080, PLACE5000105, PLACE5000113, PLACE5000115, PLACE5000116, PLACE5000129, PLACE5000139, PLACE5000153, PLACE5000159, PLACE5000170, PLACE5000171, PLACE5000178, PLACE5000245, PLACE5000260,
PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000325, PLACE6000348, PLACE6000371, PLACE6000414, PLACE6000424, PLACE6000426, PLACE6000429, PLACE6000450, PLACE6000523, PLACE6000550, PLACE6000552, PLACE6000555, PLACE6000619, PLACE6000630, PLACE6000953, PLACE6001064, PLACE6001118, PLACE6001153, PLACE6001185, PLACE6001262, PLACE6001281, PLACE6001294, PLACE6001536, PLACE6001557, PLACE6001712, PLACE6001740, PLACE6001823, PLACE6001886, PLACE6001925, PLACE6001933, PLACE6002011, PLACE6002016, PLACE6002056,
PLACE6002102, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6003004, PLACE6003077, PLACE6003094, PLACE6003109, PLACE6003204, PLACE6003218, PLACE6003372, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004336, PLACE6004368, PLACE6004380, PLACE6004397, PLACE6004454, PLACE6004464, PLACE6004491, PLACE6004578, PLACE6004663, PLACE6004687, PLACE6004738, PLACE6004931, PLACE6004966, PLACE6004993, PLACE6005007,
PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007482, PLACE6007687, PLACE6007787, PLACE6007798, PLACE6007807, PLACE6007925, PLACE6008036,
PLACE6008094, PLACE6008126, PLACE6008285, PLACE6008444, PLACE6008453, PLACE6008640, PLACE6008750, PLACE6008768, PLACE6008775, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009590, PLACE6009821, PLACE6009835, PLACE6009853, PLACE6010077, PLACE6010463, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011102, PLACE6011156, PLACE6011211, PLACE6011260, PLACE6011627, PLACE6011774, PLACE6011881, PLACE6011907, PLACE6011970, PLACE6011975, PLACE6012028,
PLACE6012108, PLACE6012168, PLACE6012297, PLACE6012308, PLACE6012574, PLACE6012908, PLACE6012936, PLACE6012942, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016942, PLACE6016984, PLACE6017002, PLACE6017431, PLACE6017564, PLACE6017578, PLACE6017701, PLACE6017714, PLACE6017788, PLACE6017791, PLACE6018031, PLACE6018107, PLACE6018187,
PLACE6018235, PLACE6018287, PLACE6018441, PLACE6018487, PLACE6018497, PLACE6018769, PLACE6018834, PLACE6018843, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973,
PLACE7008136, PLACE7008261, PLACE7008431, PLACE7008623, PLACE7008766, PLACE7009563, PROST1000039, PROST1000065, PROST1000083, PROST1000085, PROST1000110, PROST1000123, PROST1000136, PROST1000145, PROST1000152, PROST1000167, PROST1000184, PROST1000199, PROST1000215, PROST1000217, PROST1000220, PROST1000246, PROST1000262, PROST1000272, PROST1000298, PROST1000322, PROST1000334, PROST1000362, PROST1000377, PROST1000383, PROST1000412, PROST1000419, PROST1000451, PROST1000480, PROST1000485, PROST1000493, PROST1000512, PROST1000526, PROST1000527, PROST1000528, PROST1000536, PROST1000559, PROST1000564, PROST1000575, PROST2000053, PROST2000067, PROST2000079, PROST2000105, PROST2000143, PROST2000162,
PROST2000165, PROST2000176, PROST2000203, PROST2000206, PROST2000212, PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001213, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101,
PROST2002162, PROST2002212, PROST2002227, PROST2002338, PROST2002425, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303, PROST2003324, PROST2003340, PROST2003396, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251,
PROST2004258, PROST2004332, PROST2004416, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005426, PROST2005466, PROST2005630, PROST2005683, PROST2005788, PROST2005880, PROST2005886, PROST2005904, PROST2005919, PROST2005943, PROST2006008, PROST2006020, PROST2006030, PROST2006060, PROST2006196, PROST2006201, PROST2006282, PROST2006343, PROST2006450, PROST2006491, PROST2006510, PROST2006579, PROST2006688, PROST2006766, PROST2006782, PROST2006933, PROST2006988, PROST2007122, PROST2007200, PROST2007237,
PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008360, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318,
PROST2010326, PROST2010382, PROST2010400, PROST2010545, PROST2010579, PROST2010606, PROST2010885, PROST2011012, PROST2011038, PROST2011105, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012400, PROST2012448, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925,
PROST2015124, PROST2015137, PROST2015198, PROST2015243, PROST2015246, PROST2015251, PROST2015332, PROST2015457, PROST2015537, PROST2015545, PROST2015551, PROST2015636, PROST2015710, PROST2015756, PROST2015877, PROST2015900, PROST2015924, PROST2015932, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017367, PROST2017413, PROST2017441, PROST2017578, PROST2017612, PROST2017617, PROST2017700, PROST2017701, PROST2017729, PROST2017836, PROST2017910, PROST2017972, PROST2018030, PROST2018090,
PROST2018235, PROST2018437, PROST2018487, PROST2018511, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000081, PUAEN1000085, PUAEN1000087, PUAEN1000121, PUAEN1000147, PUAEN1000161, PUAEN1000175, PUAEN1000275, PUAEN1000276, PUAEN1000288, PUAEN1000322, PUAEN2000080, PUAEN2000152, PUAEN2000175, PUAEN2000247, PUAEN2000312, PUAEN2000394, PUAEN2000594, PUAEN2000684, PUAEN2000942, PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003079,
PUAEN2003116, PUAEN2003408, PUAEN2003517, PUAEN2004067, PUAEN2004083, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749,
RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000022, SKMUS1000030, SKMUS1000064, SKMUS1000067, SKMUS1000084, SKMUS1000102, SKMUS1000104, SKMUS1000110, SKMUS1000118, SKMUS1000124, SKMUS1000129, SKMUS1000138, SKMUS1000176, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000365, SKMUS2000380, SKMUS2000390, SKMUS2000416, SKMUS2000458, SKMUS2000467, SKMUS2000468, SKMUS2000515, SKMUS2000679, SKMUS2000690, SKMUS2000701, SKMUS2000705,
SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000902, SKMUS2000933, SKMUS2000945, SKMUS2000954, SKMUS2000961, SKMUS2000973, SKMUS2000980, SKMUS2001008, SKMUS2001014, SKMUS2001129, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695,
SKMUS2001703, SKMUS2001740, SKMUS2001742, SKMUS2001823, SKMUS2001850, SKMUS2002077, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000137, SKNMC1000159, SKNMC1000229,
SKNMC1000251, SKNMC1000264, SKNMC2000224, SKNMC2000256, SKNMC2000305, SKNMC2000356, SKNMC2000374, SKNMC2000583, SKNMC2000612, SKNMC2000622, SKNMC2000877, SKNMC2000966, SKNMC2001113, SKNMC2001324, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000086, SKNSH1000101, SKNSH1000174, SKNSH1000301, SKNSH1000308, SKNSH1000416, SKNSH2000051, SKNSH2000101, SKNSH2000163, SKNSH2000250, SKNSH2000319, SKNSH2000482, SKNSH2000516, SKNSH2000550, SKNSH2000819, SKNSH2001222, SKNSH2001875,
SKNSH2001931, SKNSH2002054, SKNSH2002325, SKNSH2002768, SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000039, SMINT1000042, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000137,
SMINT1000192, SMINT2000032, SMINT2000040, SMINT2000060, SMINT2000073, SMINT2000084, SMINT2000145, SMINT2000176, SMINT2000185, SMINT2000227, SMINT2000229, SMINT2000232, SMINT2000267, SMINT2000400, SMINT2000454, SMINT2000468, SMINT2000518, SMINT2000529, SMINT2000530, SMINT2000537, SMINT2000538, SMINT2000541, SMINT2000545, SMINT2000558, SMINT2000562, SMINT2000568, SMINT2000609, SMINT2000629, SMINT2000694, SMINT2000747, SMINT2000811, SMINT2000824, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001818, SMINT2001950,
SMINT2002126, SMINT2002159, SMINT2002202, SMINT2002210, SMINT2002224, SMINT2002281, SMINT2002311, SMINT2002328, SMINT2002414, SMINT2002457, SMINT2002620, SMINT2002689, SMINT2002743, SMINT2002778, SMINT2002880, SMINT2002899, SMINT2002974, SMINT2002976, SMINT2003003, SMINT2003128, SMINT2003150, SMINT2003317, SMINT2003386, SMINT2003423, SMINT2003505, SMINT2003551, SMINT2003569, SMINT2003641, SMINT2003644, SMINT2003866, SMINT2003905, SMINT2004037, SMINT2004047, SMINT2004086, SMINT2004299, SMINT2004339, SMINT2004350, SMINT2004414, SMINT2004473, SMINT2004547, SMINT2004583, SMINT2004589, SMINT2004729, SMINT2004781, SMINT2004872, SMINT2004891, SMINT2004909, SMINT2004972, SMINT2004992, SMINT2005075,
SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545,
SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010959, SMINT2010997,
SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313,
SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000116, SPLEN1000134, SPLEN1000139, SPLEN1000143, SPLEN1000145, SPLEN1000156, SPLEN1000163, SPLEN1000178, SPLEN2000020, SPLEN2000064, SPLEN2000072, SPLEN2000098, SPLEN2000126,
SPLEN2000143, SPLEN2000189, SPLEN2000197, SPLEN2000205, SPLEN2000210, SPLEN2000242, SPLEN2000243, SPLEN2000247, SPLEN2000267, SPLEN2000270, SPLEN2000307, SPLEN2000310, SPLEN2000341, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000698, SPLEN2000839, SPLEN2000882, SPLEN2001135, SPLEN2001141, SPLEN2001176, SPLEN2001227, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710,
SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002477, SPLEN2002493, SPLEN2002662, SPLEN2002695, SPLEN2002707, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004343, SPLEN2004346, SPLEN2004368, SPLEN2004555, SPLEN2004611, SPLEN2004662, SPLEN2004773, SPLEN2004844,
SPLEN2005009, SPLEN2005227, SPLEN2005272, SPLEN2005342, SPLEN2005416, SPLEN2005450, SPLEN2005474, SPLEN2005727, SPLEN2005783, SPLEN2005790, SPLEN2005818, SPLEN2005838, SPLEN2005869, SPLEN2005927, SPLEN2005939, SPLEN2006122, SPLEN2006133, SPLEN2006232, SPLEN2006268, SPLEN2006283, SPLEN2006325, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008496, SPLEN2008591, SPLEN2008786,
SPLEN2009081, SPLEN2009088, SPLEN2009163, SPLEN2009315, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672,
SPLEN2011678, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012524, SPLEN2012525, SPLEN2012571, SPLEN2012606, SPLEN2012619, SPLEN2012624, SPLEN2012725, SPLEN2012743, SPLEN2012756, SPLEN2012846, SPLEN2012889, SPLEN2012961, SPLEN2013108, SPLEN2013195, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318,
SPLEN2014381, SPLEN2014452, SPLEN2014454, SPLEN2014486, SPLEN2014543, SPLEN2014572, SPLEN2014645, SPLEN2014669, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016268, SPLEN2016304, SPLEN2016356,
SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212, SPLEN2017318, SPLEN2017426, SPLEN2017452, SPLEN2017538, SPLEN2017592, SPLEN2017613, SPLEN2017643, SPLEN2017687, SPLEN2017740, SPLEN2017781, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732,
SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295,
SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765,
SPLEN2027113, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098,
SPLEN20331'53, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037891, SPLEN2038055, SPLEN2038180, SPLEN2038345, SPLEN2038407, SPLEN2038834, SPLEN2039311, SPLEN2039379, SPLEN2039672, SPLEN2039697, SPLEN2039845, SPLEN2039936, SPLEN2040222,
SPLEN2040237, SPLEN2040824, SPLEN2041304, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, STOMA1000013, STOMA1000052, STOMA1000074, STOMA1000091, STOMA1000117, STOMA1000133, STOMA1000175, STOMA1000186, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000279, STOMA2000289, STOMA2000383, STOMA2000386, STOMA2000395, STOMA2000396, STOMA2000478, STOMA2000482, STOMA2000539, STOMA2000567, STOMA2000640, STOMA2000678, STOMA2000686, STOMA2000888, STOMA2001025, STOMA2002052,
STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000049, SYNOV1000118,
SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001033, SYNOV2001035, SYNOV2001088, SYNOV2001111, SYNOV2001212, SYNOV2001251, SYNOV2001262, SYNOV2001300, SYNOV2001356, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965,
SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542,
SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, SYNOV4009575, T1ESE2000116, T1ESE2000609, T1ESE2000904, T1ESE2002665, TBAES2000004, TBAES2000116, TBAES2000255, TBAES2000315, TBAES2000352, TBAES2000932, TBAES2001171, TBAES2001220, TBAES2001229, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007481, TBAES2007504, TBAES2007548, TBAES2007697, TBAES2007862, TBAES2007964,
TBAES2008133, TCERX2000171, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002451, TESOP2002489, TESOP2002539, TESOP2002545, TESOP2002804, TESOP2002950, TESOP2003273, TESOP2003308, TESOP2003553, TESOP2003753,
TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000085,
TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179, TESTI2000184, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001134, TESTI2001141, TESTI2001180, TESTI2001208, TESTI2001229, TESTI2001236, TESTI2001237, TESTI2001306, TESTI2001313,
TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105,
TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002877, TESTI2002910, TESTI2002912, TESTI2002928, TESTI2002937, TESTI2002965, TESTI2002993, TESTI2003005, TESTI2003020,
TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003299, TESTI2003311, TESTI2003321, TESTI2003327, TESTI2003330, TESTI2003347, TESTI2003354, TESTI2003356, TESTI2003372, TESTI2003376, TESTI2003413, TESTI2003418, TESTI2003419, TESTI2003432, TESTI2003454, TESTI2003475, TESTI2003476, TESTI2003498, TESTI2003512, TESTI2003539, TESTI2003541, TESTI2003545, TESTI2003551, TESTI2003574, TESTI2003579,
TESTI2003580, TESTI2003589, TESTI2003596, TESTI2003598, TESTI2003615, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004432, TESTI2004452, TESTI2004459,
TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004734, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005025, TESTI2005040, TESTI2005060, TESTI2005072, TESTI2005085, TESTI2005120, TESTI2005153, TESTI2005155, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415,
TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005759, TESTI2005767, TESTI2005775, TESTI2005784, TESTI2005827, TESTI2005835, TESTI2005860, TESTI2005892, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006109,
TESTI2006111, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006877, TESTI2006879, TESTI2006894, TESTI2006940,
TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007320, TESTI2007346, TESTI2007358, TESTI2007402, TESTI2007405, TESTI2007407, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032,
TESTI2008033, TESTI2008046, TESTI2008081, TESTI2008099, TESTI2008139, TESTI2008144, TESTI2008161, TESTI2008189, TESTI2008204, TESTI2008219, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239,
TESTI2009325, TESTI2009388, TESTI2009390, TESTI2009400, TESTI2009402, TESTI2009412, TESTI2009447, TESTI2009462, TESTI2009474, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835, TESTI2009853, TESTI2009935, TESTI2009987, TESTI2010009, TESTI2010154, TESTI2010180, TESTI2010239, TESTI2010280, TESTI2010354, TESTI2010396, TESTI2010400, TESTI2010409, TESTI2010513, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009,
TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812,
TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339,
TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421,
TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178,
TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917,
TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021124, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736,
TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968,
TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025757,
TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206,
TESTI2027238, TESTI2027239, TESTI2027243, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252,
TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505,
TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650,
TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041,
TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302,
TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964,
TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137,
TESTI2050681, TESTI2050780, TESTI2050987, TESTI2051148, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288,
TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647,
TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148,
TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429,
TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009286, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532,
TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661,
TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961,
TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654,
TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062,
TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432,
TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744,
TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291,
TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000002, THYMU1000010, THYMU1000017, THYMU1000025, THYMU1000029, THYMU1000032, THYMU1000033, THYMU1000060, THYMU1000074, THYMU1000083, THYMU1000098, THYMU1000103,
THYMU1000105, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000382, THYMU1000393, THYMU1000399, THYMU1000426, THYMU1000440, THYMU1000459, THYMU1000490, THYMU1000491, THYMU1000496, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000120, THYMU2000138, THYMU2000140, THYMU2000155, THYMU2000162, THYMU2000171, THYMU2000176, THYMU2000179, THYMU2000231, THYMU2000236, THYMU2000276,
THYMU2000280, THYMU2000317, THYMU2000368, THYMU2000382, THYMU2000418, THYMU2000440, THYMU2000489, THYMU2000492, THYMU2000519, THYMU2000570, THYMU2000602, THYMU2000660, THYMU2000671, THYMU2000672, THYMU2000684, THYMU2000767, THYMU2000800, THYMU2000879, THYMU2000932, THYMU2000950, THYMU2000971, THYMU2000982, THYMU2000990, THYMU2001007, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898,
THYMU2001900, THYMU2001916, THYMU2001926, THYMU2001989, THYMU2002023, THYMU2002037, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002756, THYMU2002789, THYMU2002792, THYMU2002815, THYMU2002841, THYMU2002852, THYMU2002910, THYMU2002950, THYMU2002983, THYMU2003046, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003419, THYMU2003440, THYMU2003470,
THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004108, THYMU2004111, THYMU2004123, THYMU2004139, THYMU2004152, THYMU2004169, THYMU2004284, THYMU2004320, THYMU2004336, THYMU2004344, THYMU2004356, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283,
THYMU2005303, THYMU2005321, THYMU2005356, THYMU2005369, THYMU2005439, THYMU2005480, THYMU2005482, THYMU2005507, THYMU2005545, THYMU2005546, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006813, THYMU2006913, THYMU2006946, THYMU2006965, THYMU2007025, THYMU2007036, THYMU2007060, THYMU2007065, THYMU2007095, THYMU2007112, THYMU2007146,
THYMU2007179, THYMU2007258, THYMU2007307, THYMU2007308, THYMU2007339, THYMU2007350, THYMU2007384, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007802, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781,
THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183,
THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013364,
THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010,
THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416,
THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249,
THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032035, THYMU2032080, THYMU2032150, THYMU2032155, THYMU2032358, THYMU2032437,
THYMU2032601, THYMU2032655, THYMU2032715, THYMU2032732, THYMU2032825, THYMU2032976, THYMU2033053, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226,
THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420,
THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005696, THYMU3006118, THYMU3006132, THYMU3006168, THYMU3006172, THYMU3006371,
THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620,
THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313,
THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032798, THYMU3033402, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200,
THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200,
THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059, TKIDN2003062,
TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263,
TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528,
TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000038, TRACH1000057, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000047, TRACH2000081, TRACH2000083, TRACH2000148, TRACH2000185,
TRACH2000196, TRACH2000235, TRACH2000237, TRACH2000243, TRACH2000248, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000472, TRACH2000496, TRACH2000497, TRACH2000502, TRACH2000522, TRACH2000540, TRACH2000559, TRACH2000660, TRACH2000665, TRACH2000675, TRACH2000677, TRACH2000703, TRACH2000767, TRACH2000775, TRACH2000807, TRACH2000862, TRACH2000873, TRACH2000894, TRACH2000898, TRACH2000920, TRACH2000923, TRACH2000944, TRACH2000981, TRACH2000986, TRACH2001037, TRACH2001060, TRACH2001085, TRACH2001109, TRACH2001121, TRACH2001147, TRACH2001154, TRACH2001188, TRACH2001192,
TRACH2001249, TRACH2001275, TRACH2001335, TRACH2001339, TRACH2001347, TRACH2001395, TRACH2001400, TRACH2001403, TRACH2001416, TRACH2001428, TRACH2001443, TRACH2001463, TRACH2001474, TRACH2001523, TRACH2001525, TRACH2001532, TRACH2001549, TRACH2001592, TRACH2001596, TRACH2001607, TRACH2001612, TRACH2001621, TRACH2001646, TRACH2001651, TRACH2001898, TRACH2001933, TRACH2001968, TRACH2001996, TRACH2002043, TRACH2002054, TRACH2002100, TRACH2002138, TRACH2002333, TRACH2002784, TRACH2002803, TRACH2002840, TRACH2002853, TRACH2002862, TRACH2002954, TRACH2002960, TRACH2002988, TRACH2003070, TRACH2003323, TRACH2003484, TRACH2003516, TRACH2003605, TRACH2003736, TRACH2003759, TRACH2003941, TRACH2004039,
TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577,
TRACH2007674, TRACH2007676, TRACH2007688, TRACH2007733, TRACH2007754, TRACH2007816, TRACH2007894, TRACH2007969, TRACH2007988, TRACH2008081, TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009310, TRACH2009340, TRACH2009348, TRACH2009358, TRACH2009661, TRACH2009730, TRACH2009743, TRACH2009754, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600,
TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011500, TRACH2011574, TRACH2011894, TRACH2011956, TRACH2012000, TRACH2012138, TRACH2012242, TRACH2012298, TRACH2012370, TRACH2012387, TRACH2012497, TRACH2012536, TRACH2012562, TRACH2012742, TRACH2012811, TRACH2012823, TRACH2012918, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337,
TRACH2014356, TRACH2014371, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2015180, TRACH2015243, TRACH2015316, TRACH2015381, TRACH2015486, TRACH2015823, TRACH2015824, TRACH2015939, TRACH2015987, TRACH2016080, TRACH2016131, TRACH2016286, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084,
TRACH2018262, TRACH2018273, TRACH2018278, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860,
TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000780, TRACH3000892, TRACH3000926, TRACH3001119, TRACH3001443, TRACH3001550, TRACH3001759, TRACH3002064, TRACH3002107,
TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211,
TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007625, TRACH3007627, TRACH3007689, TRACH3007766, TRACH3007866, TRACH3007995, TRACH3008042, TRACH3008061, TRACH3008201, TRACH3008508, TRACH3008535, TRACH3008629, TRACH3008632, TRACH3008688, TRACH3008697,
TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700,
TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621,
TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148,
TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065,
TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037067, TRACH3037267, TRACH3037288, TRACH3037505, TRACH3037573, TRACH3037696, TRACH3037897, TRACH3038086,
TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN2000046, UMVEN2000069, UMVEN2000121, UMVEN2000133, UMVEN2000152, UMVEN2000453, UTERU1000008,
UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU2000023, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000424, UTERU2000465, UTERU2000485, UTERU2000517, UTERU2000524, UTERU2000539, UTERU2000541, UTERU2000542, UTERU2000546, UTERU2000550,
UTERU2000569, UTERU2000607, UTERU2000629, UTERU2000649, UTERU2000663, UTERU2000794, UTERU2000830, UTERU2000844, UTERU2000922, UTERU2001024, UTERU2001110, UTERU2001176, UTERU2001281, UTERU2001389, UTERU2001409, UTERU2001412, UTERU2001504, UTERU2001607, UTERU2001747, UTERU2001876, UTERU2002001, UTERU2002011, UTERU2002176, UTERU2002198, UTERU2002294, UTERU2002332, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004039,
UTERU2004061, UTERU2004073, UTERU2004163, UTERU2004299, UTERU2004461, UTERU2004520, UTERU2004564, UTERU2004664, UTERU2004688, UTERU2004698, UTERU2004861, UTERU2004929, UTERU2005004, UTERU2005050, UTERU2005069, UTERU2005074, UTERU2005179, UTERU2005292, UTERU2005346, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006593, UTERU2006651, UTERU2006705, UTERU2006899, UTERU2007004, UTERU2007075, UTERU2007081, UTERU2007128, UTERU2007253, UTERU2007267, UTERU2007444,
UTERU2007499, UTERU2007639, UTERU2007924, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008785, UTERU2008845, UTERU2008901, UTERU2008930, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951, UTERU2010115, UTERU2010124, UTERU2010164, UTERU2010226, UTERU2010231, UTERU2010304, UTERU2010320, UTERU2010431, UTERU2010525, UTERU2010651,
UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012333, UTERU2012407, UTERU2012526, UTERU2012581, UTERU2012610, UTERU2012615, UTERU2012688, UTERU2012715, UTERU2012741, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001,
UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514,
UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941,
UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172,
UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959,
UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460,
UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837,
UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the LIVER, LYMPB, MAMGL, MESAN, MESTC, N1ESE, and NB9N4 libraries were analyzed for expression frequency. The analysis results are described below. Each is a relative value, where the expression level over all tissues is taken as 100. The larger this value, the greater the level of expression.

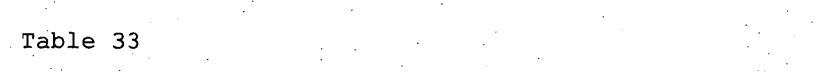

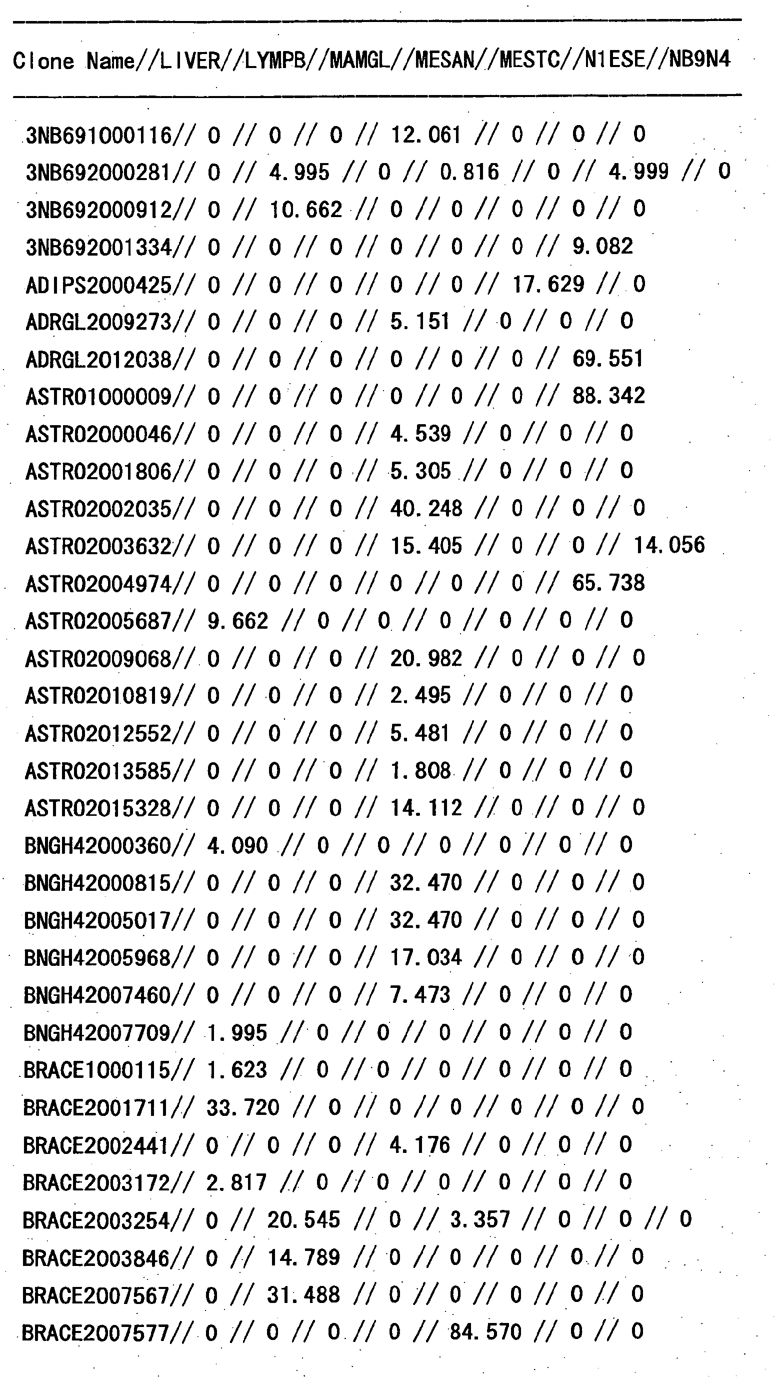

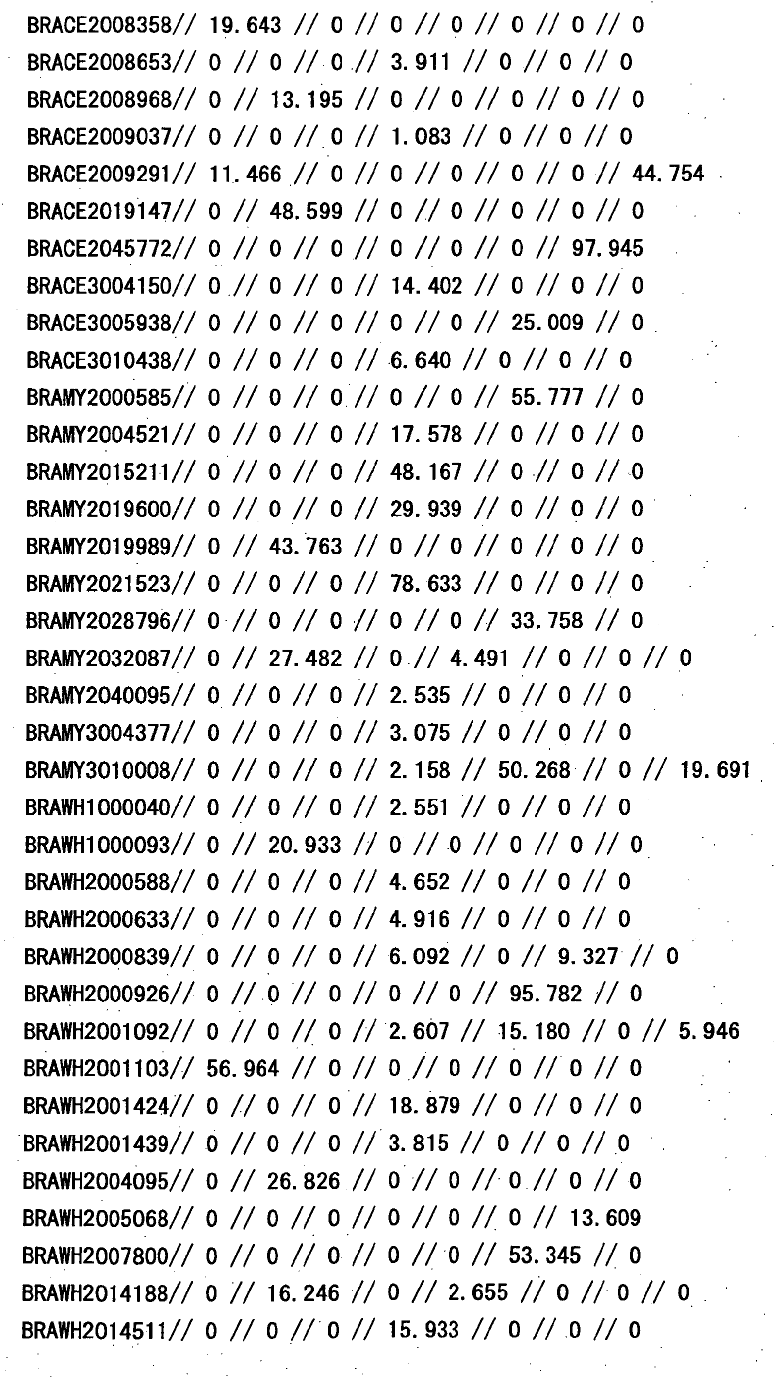

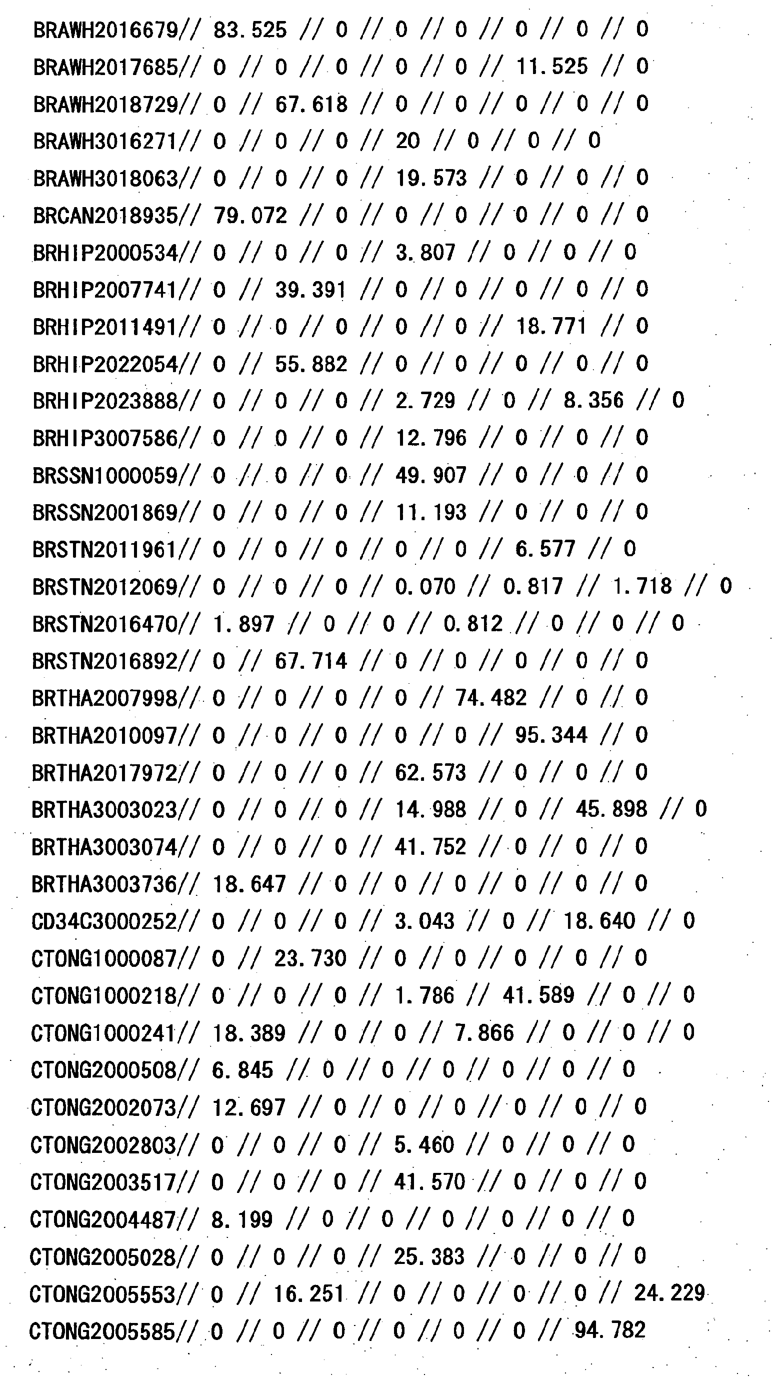

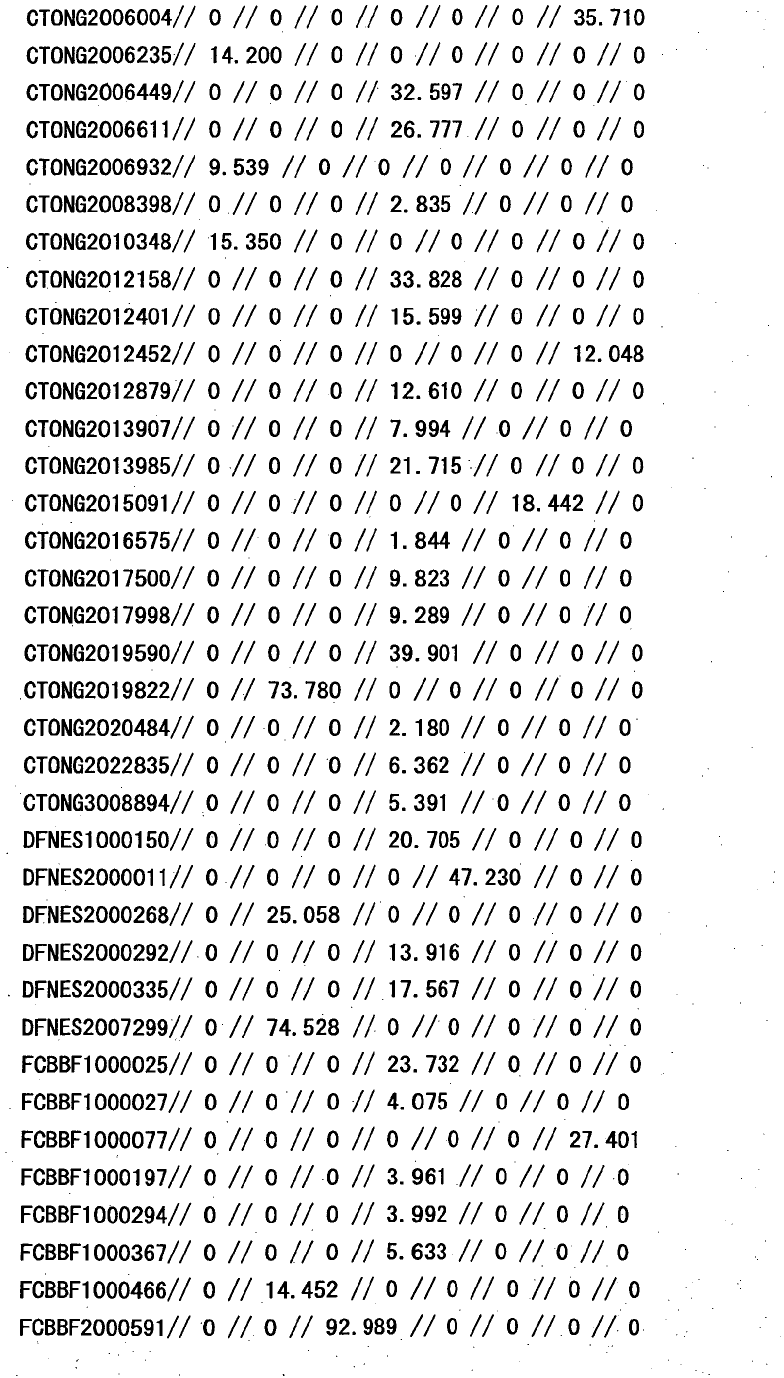

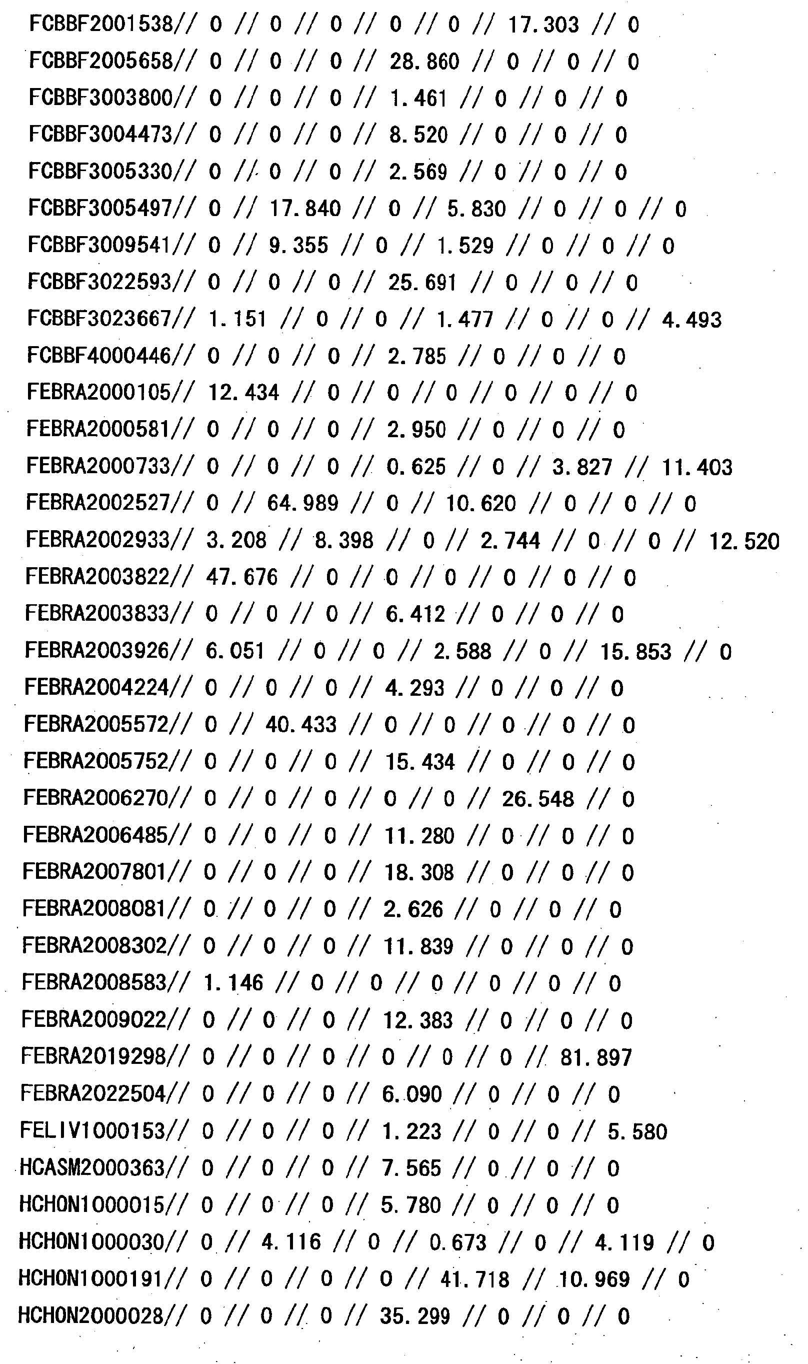

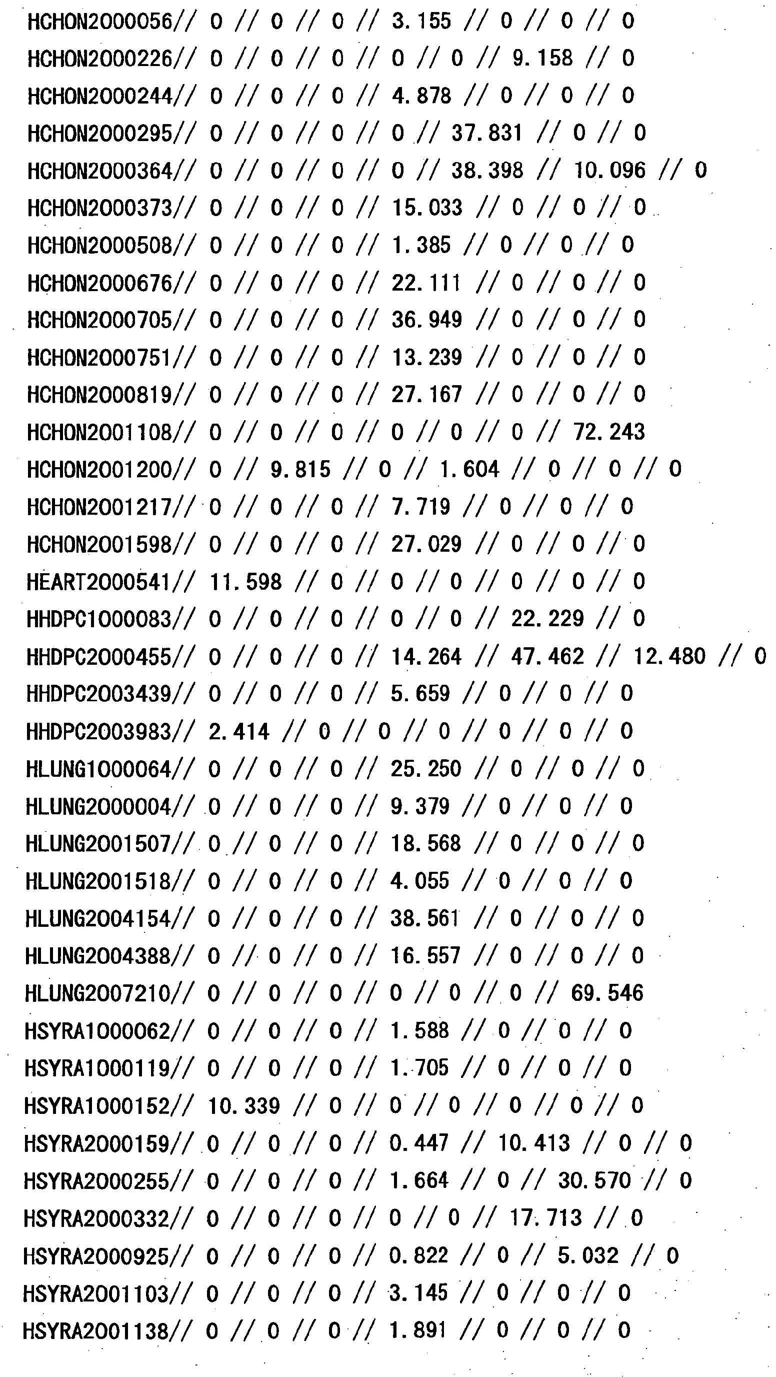

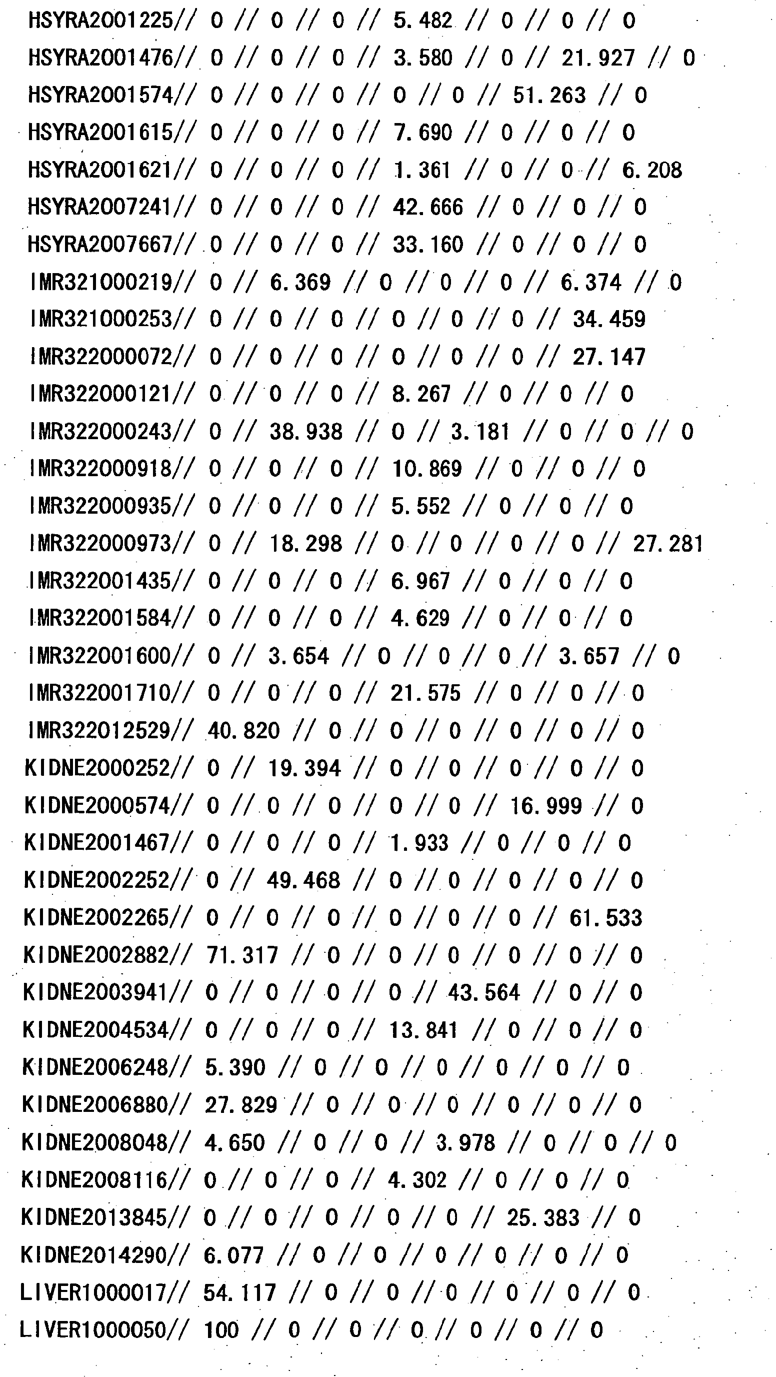

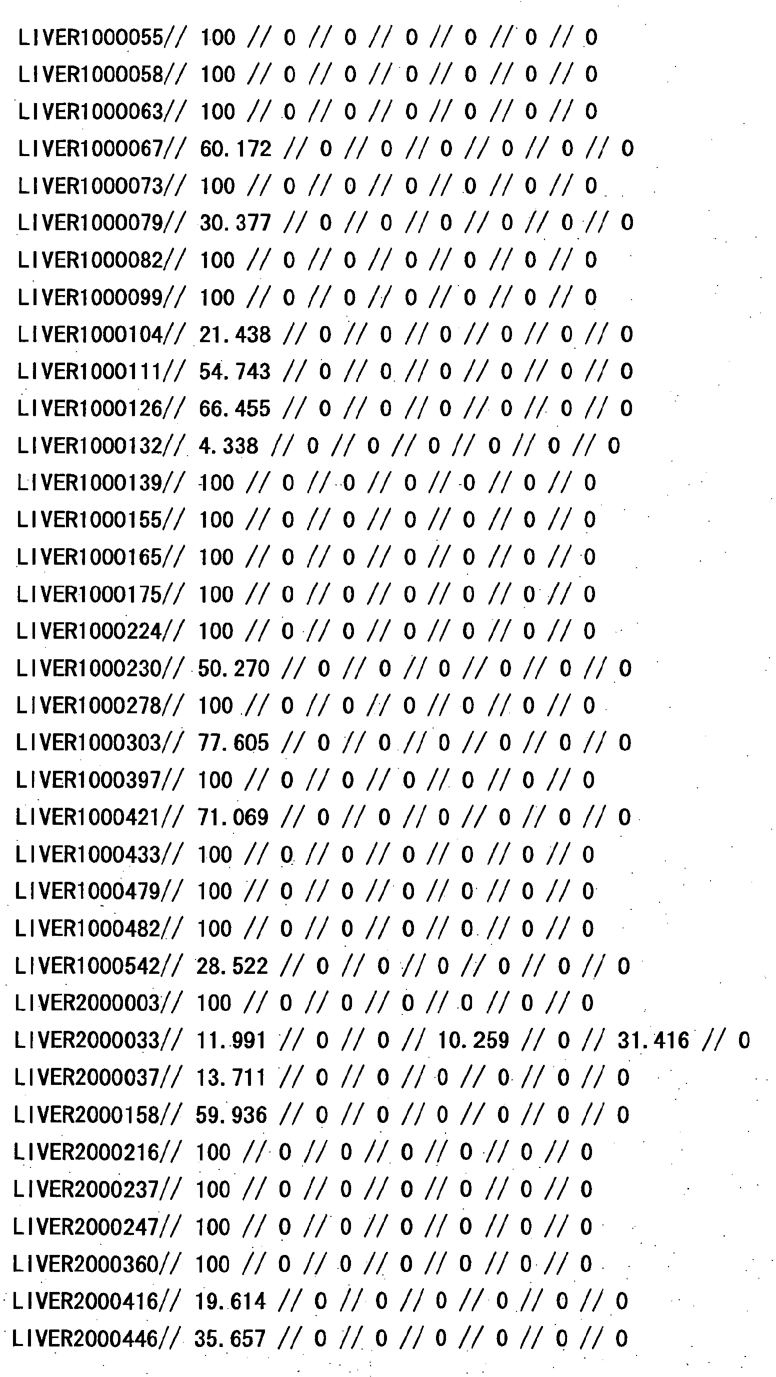

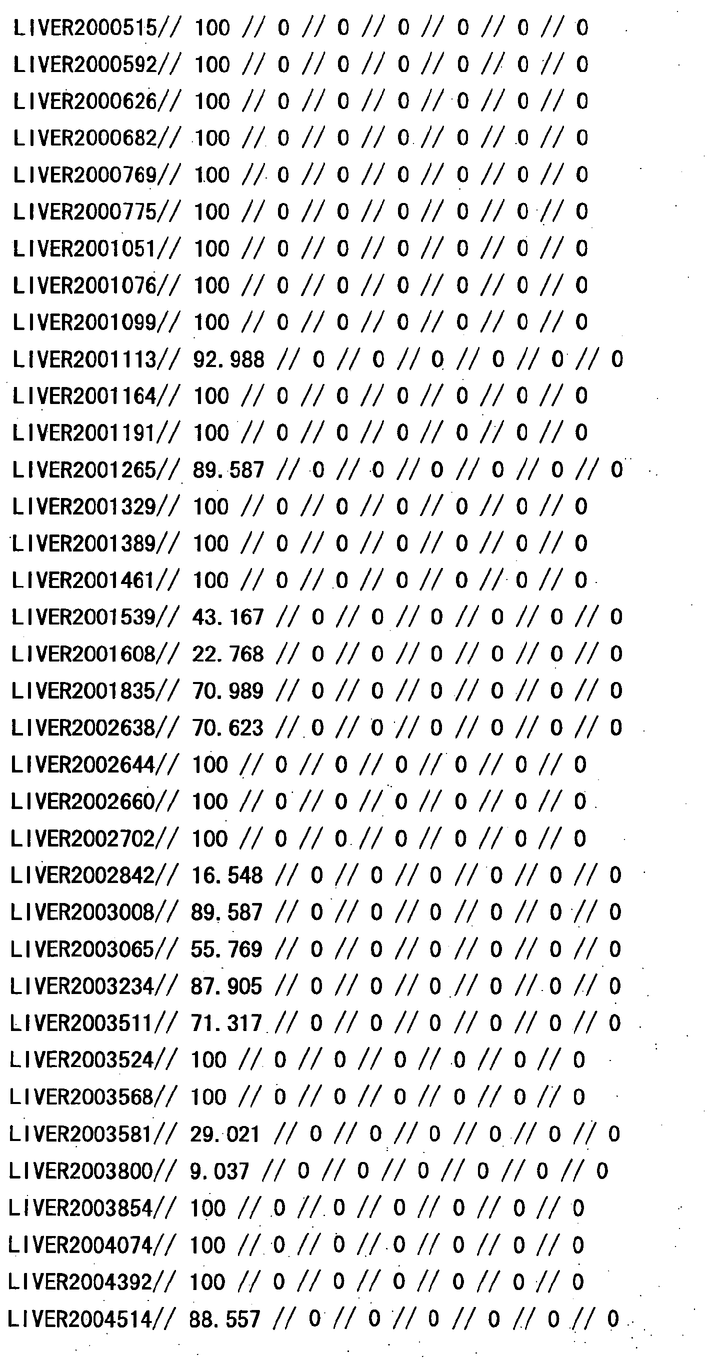

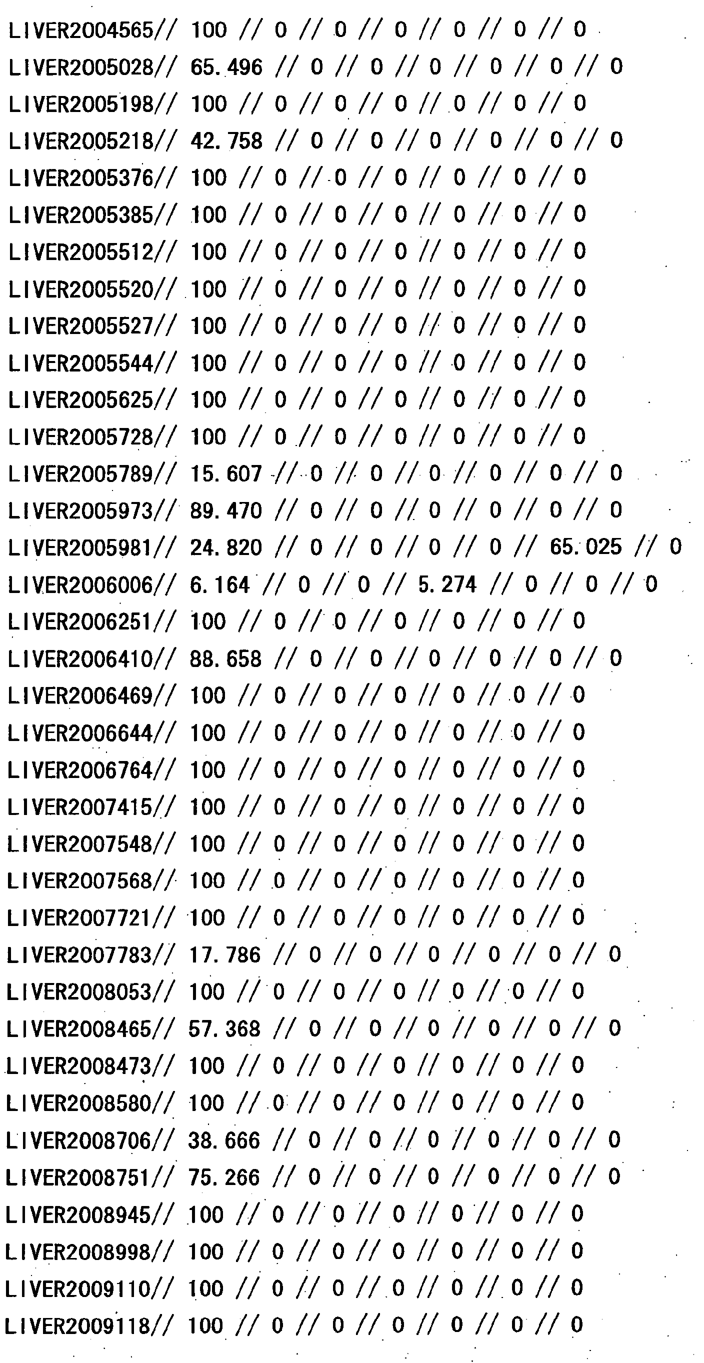

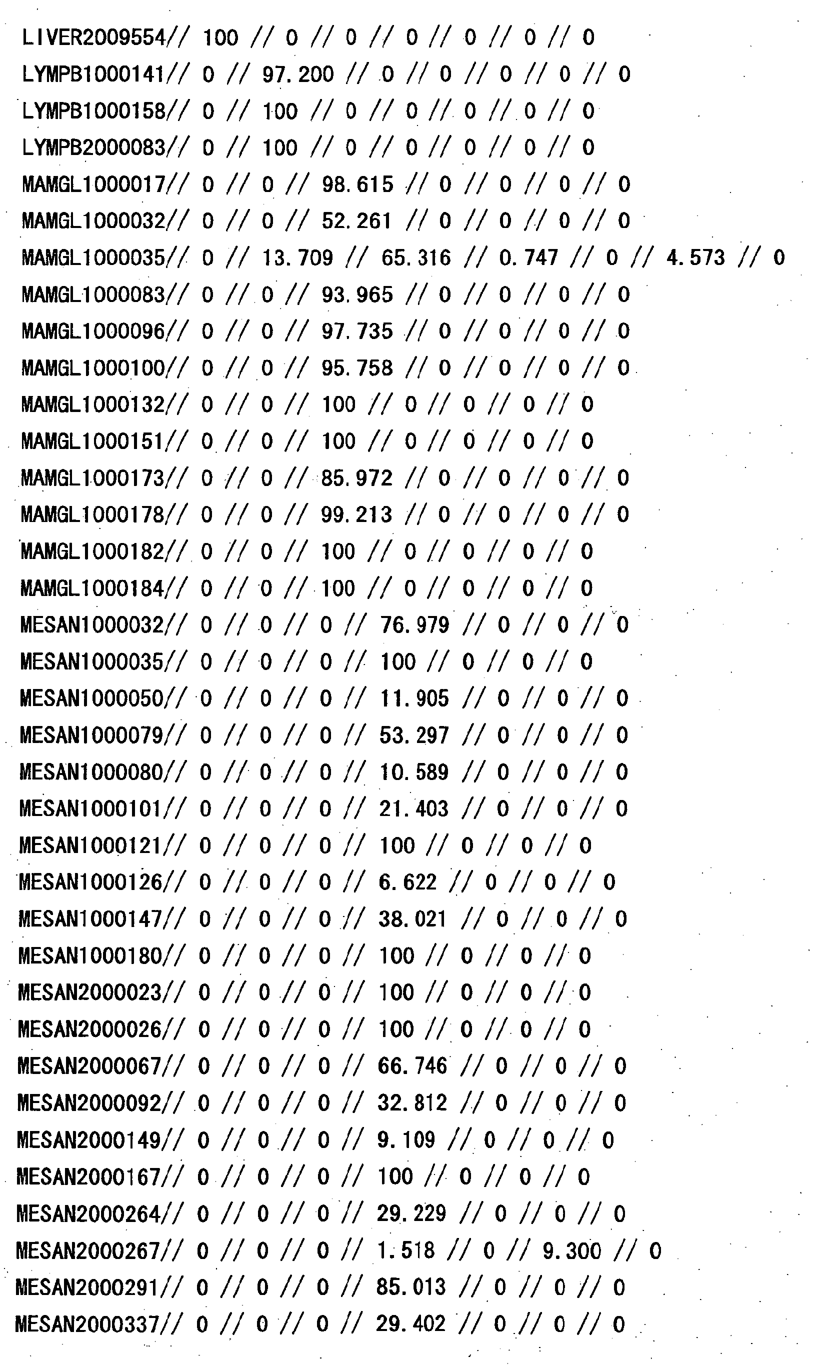

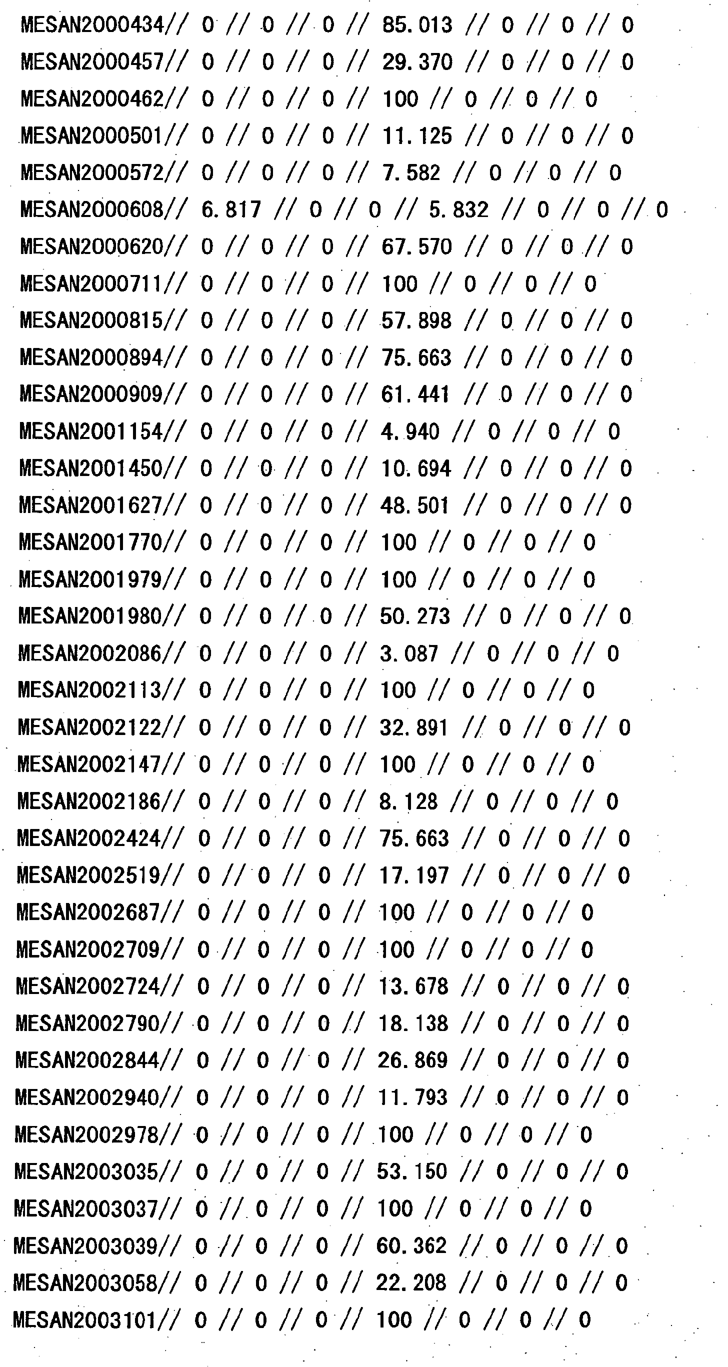

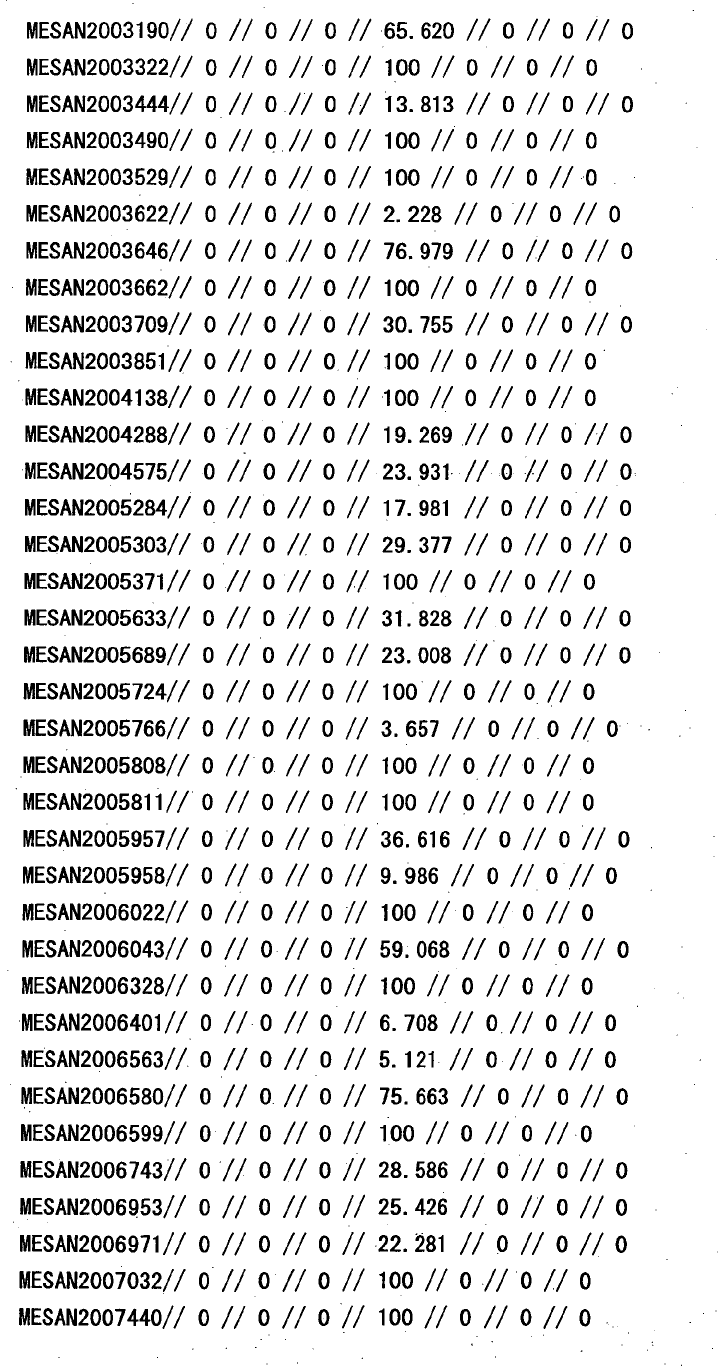

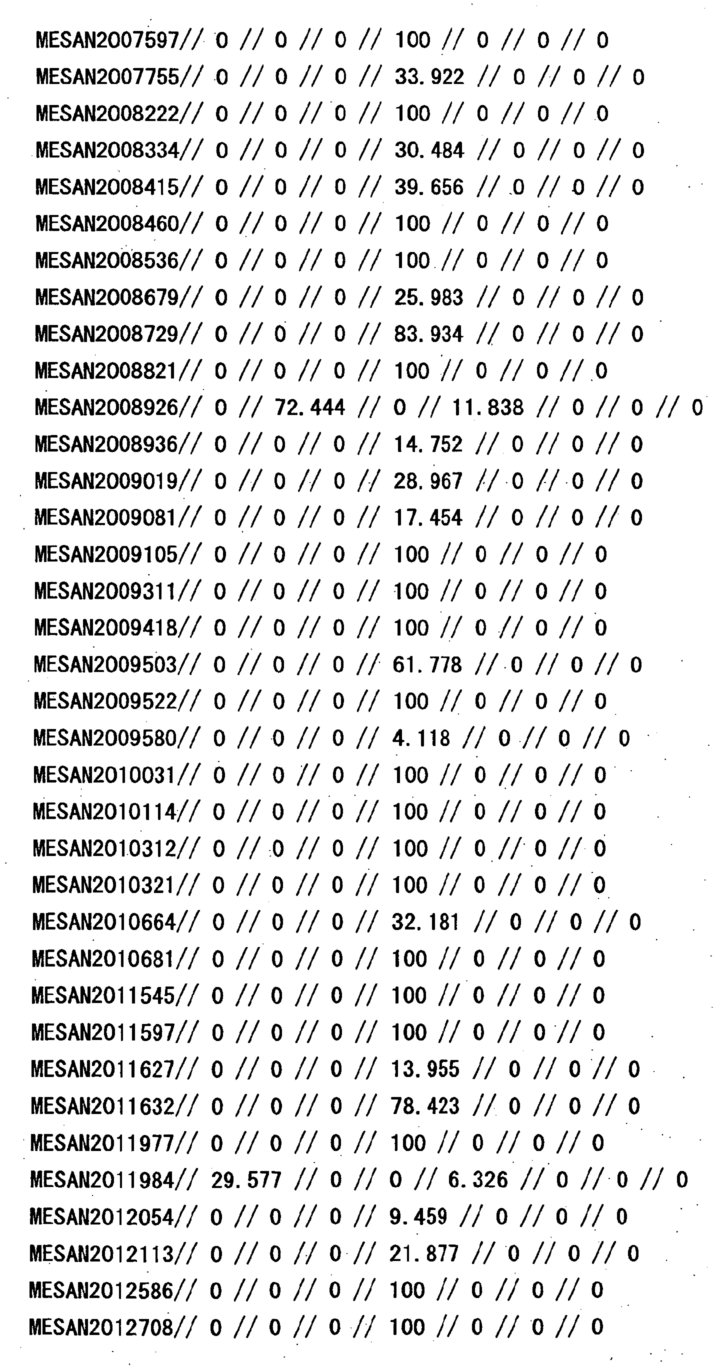

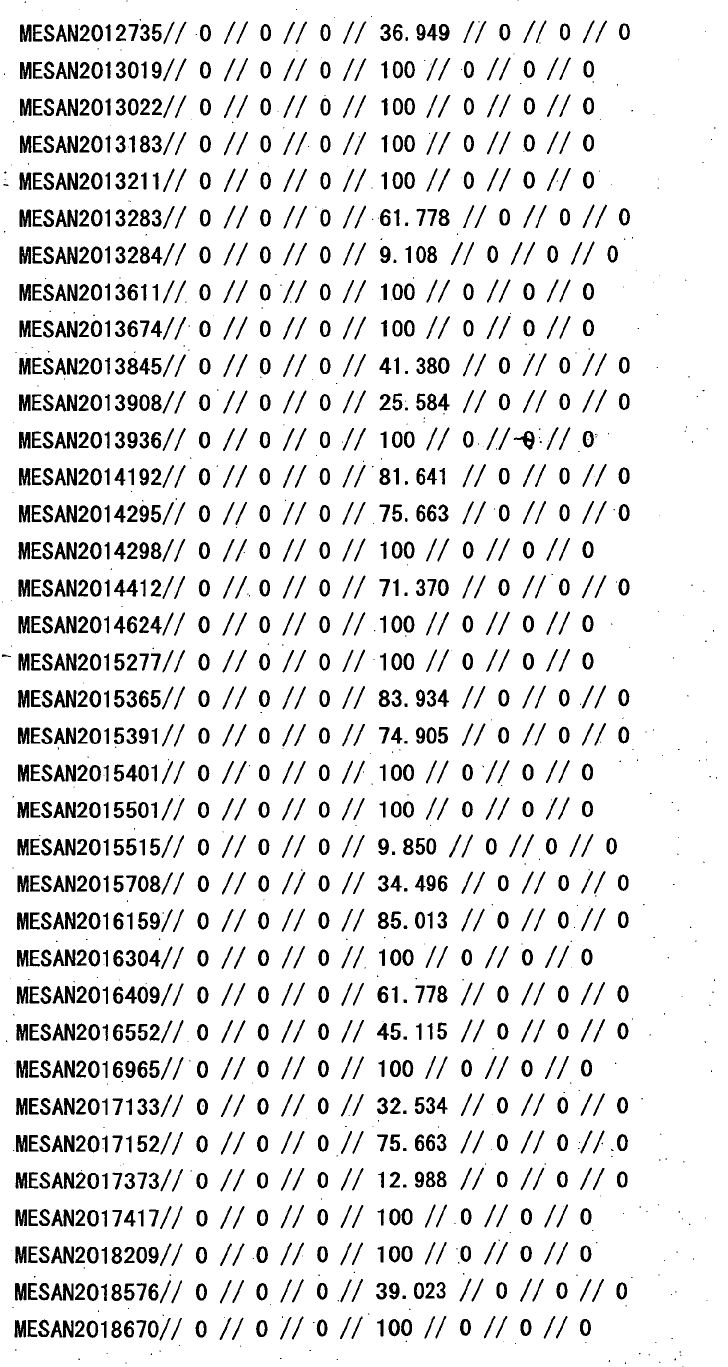

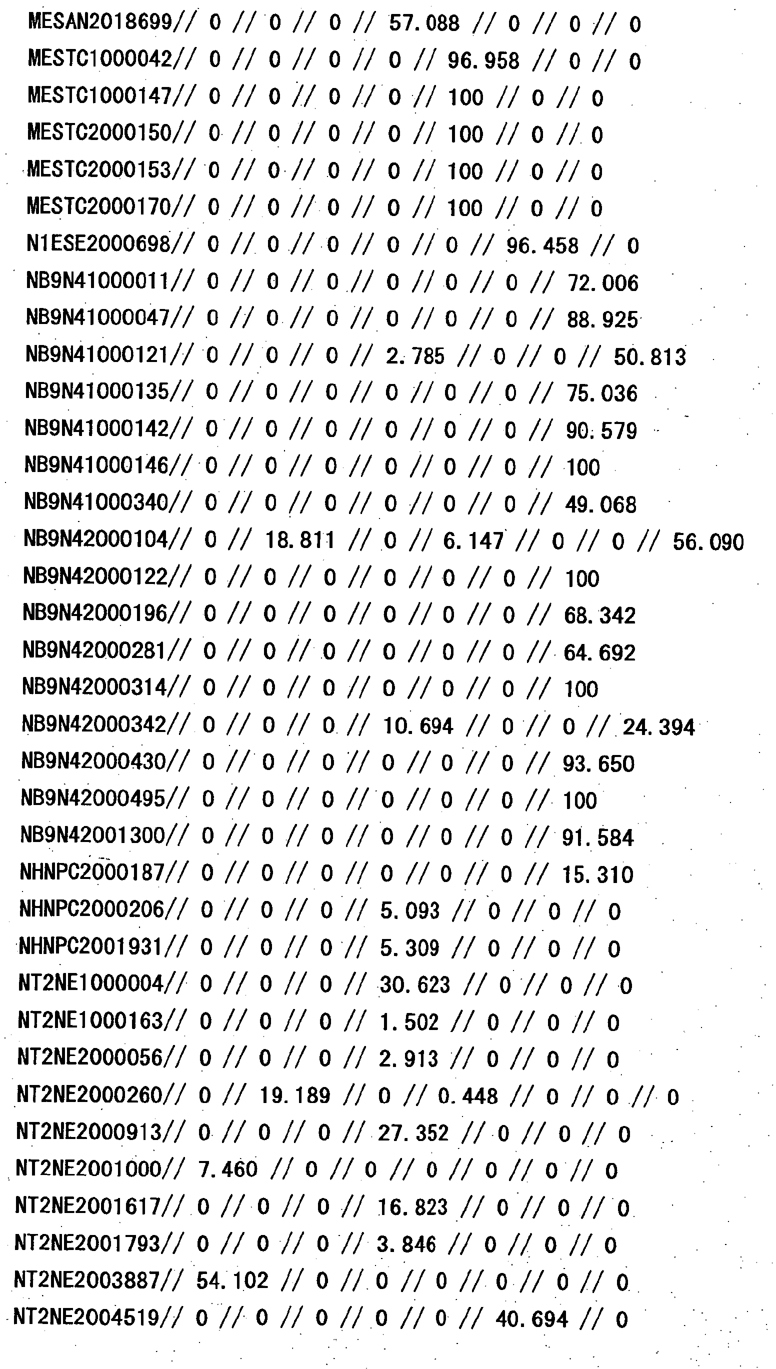

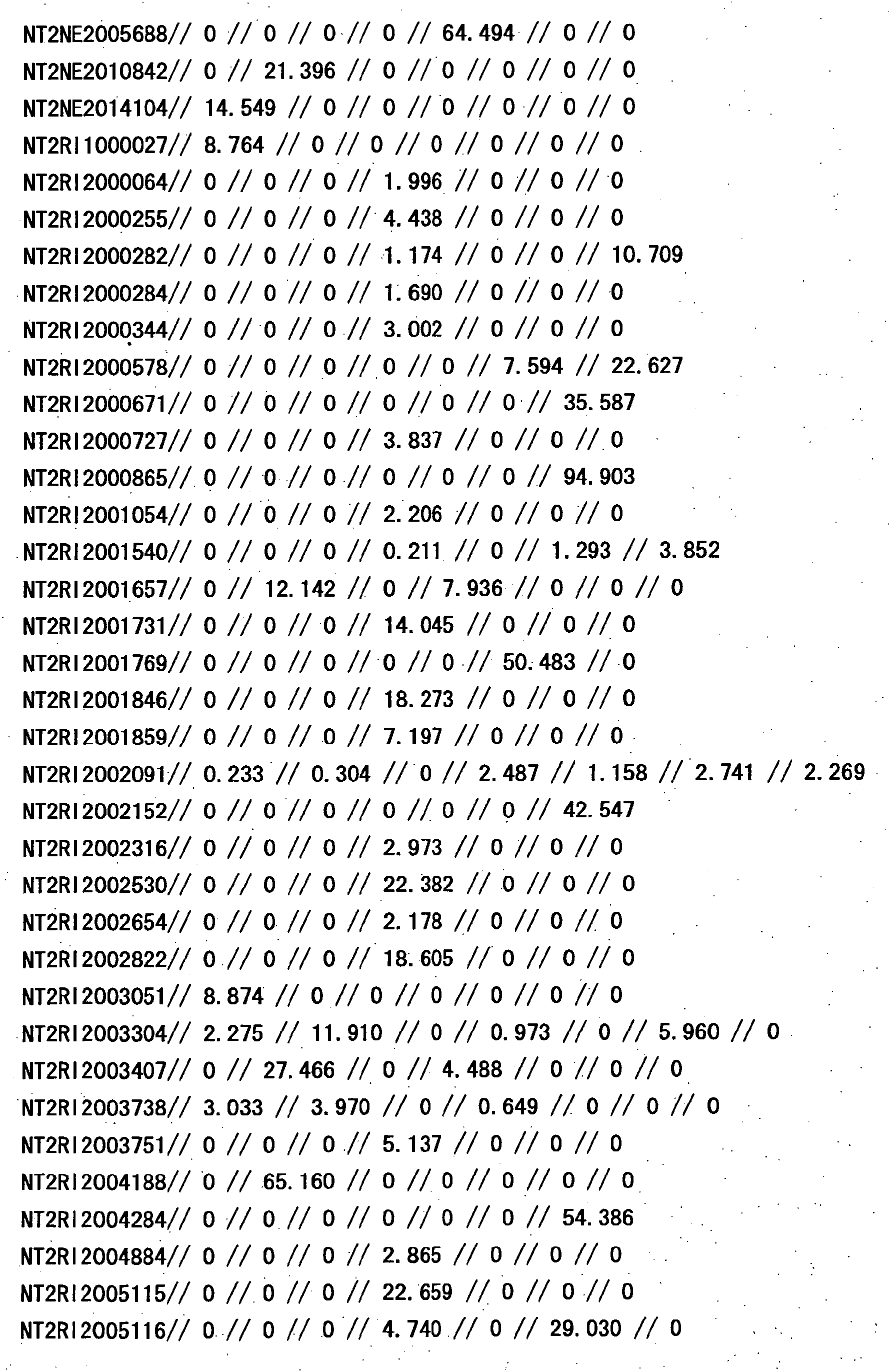

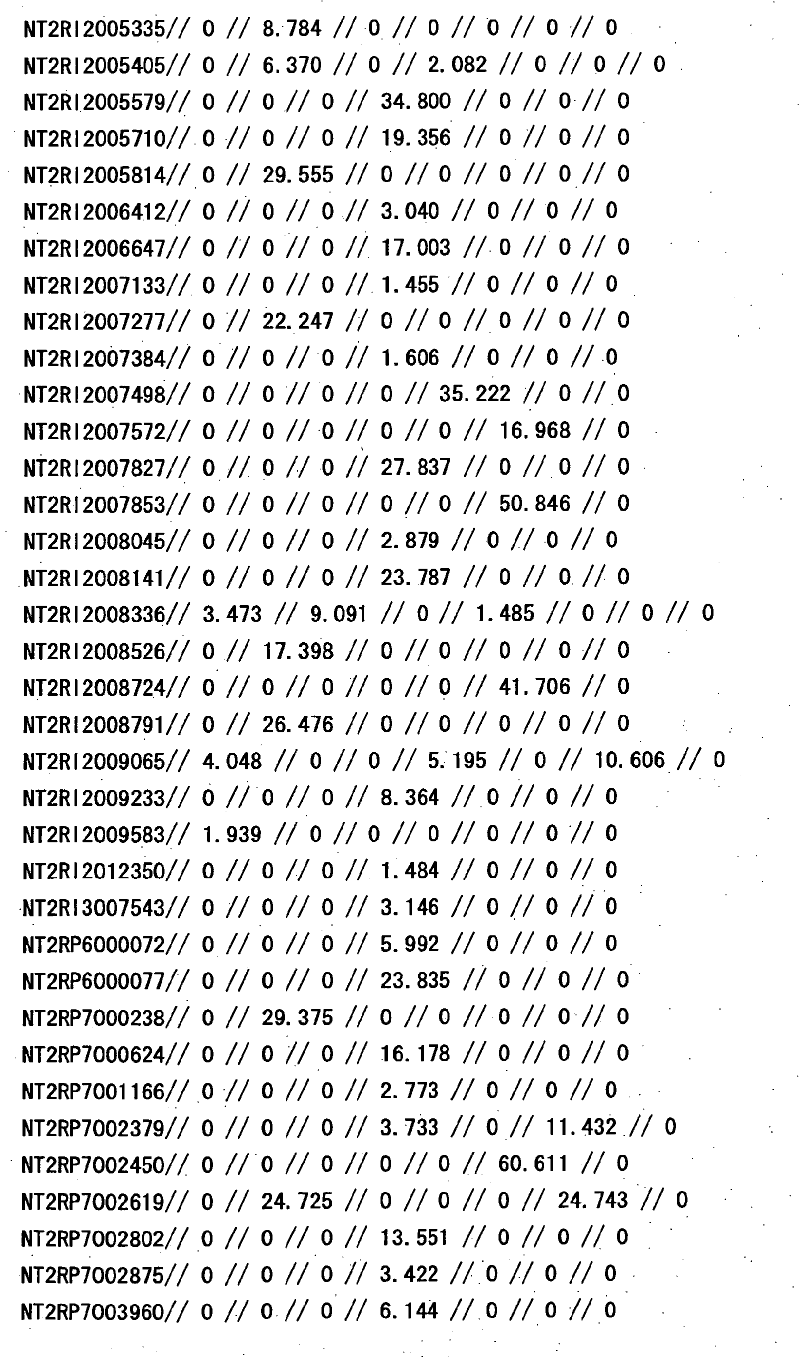

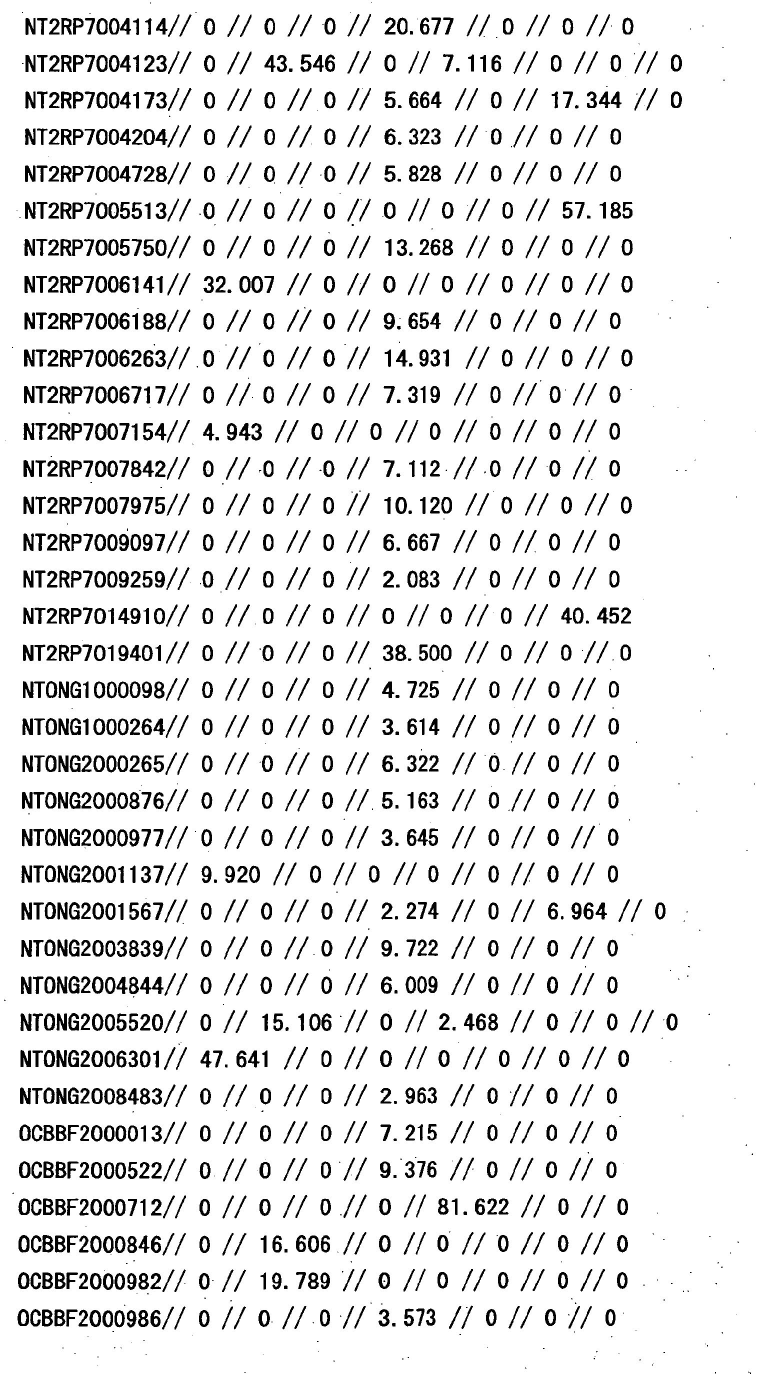

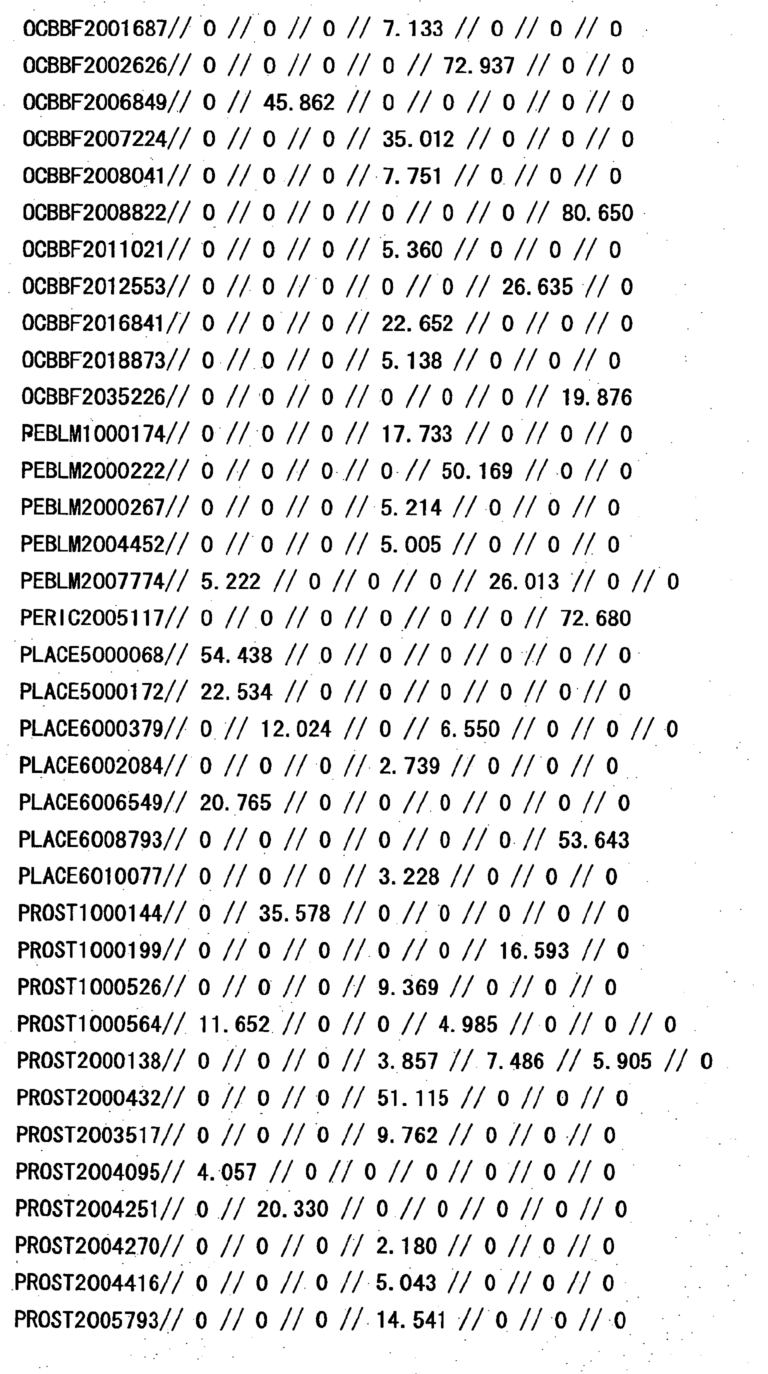

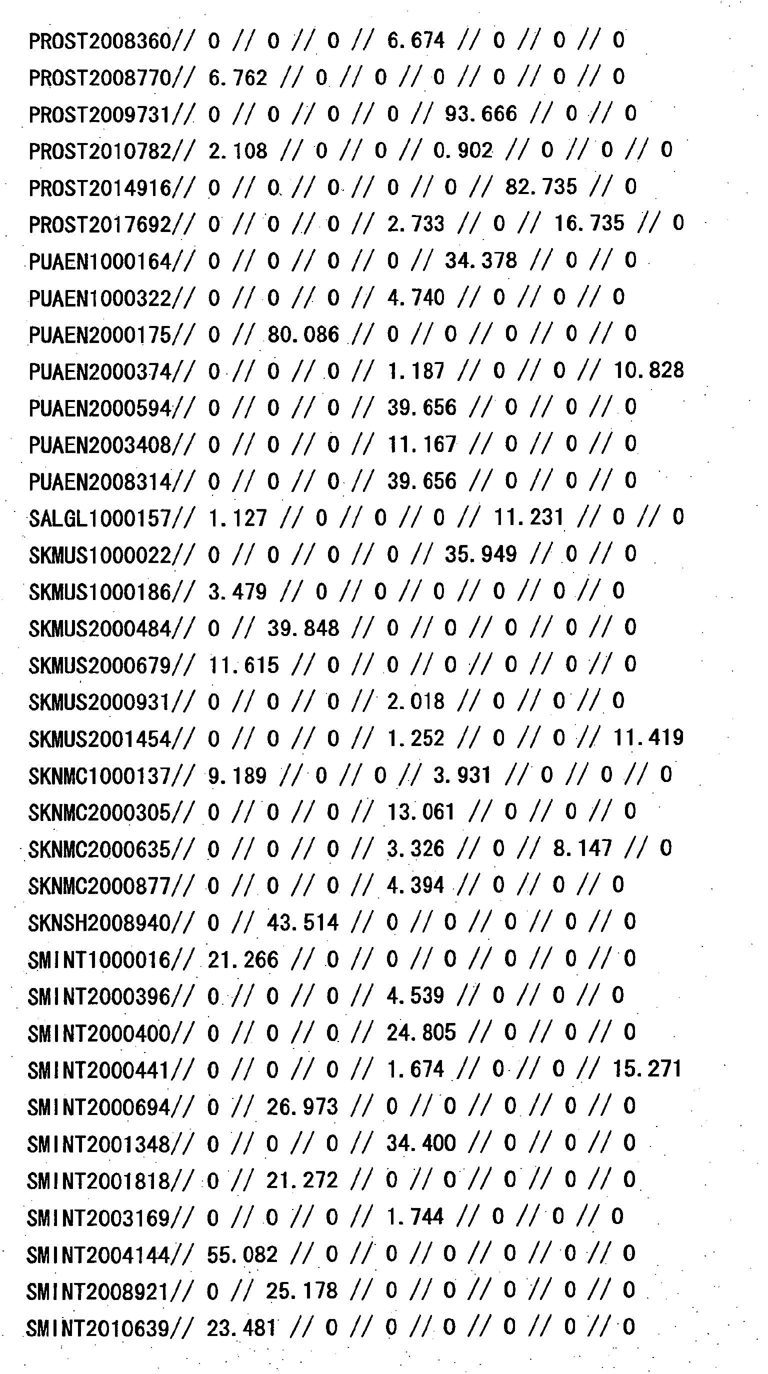

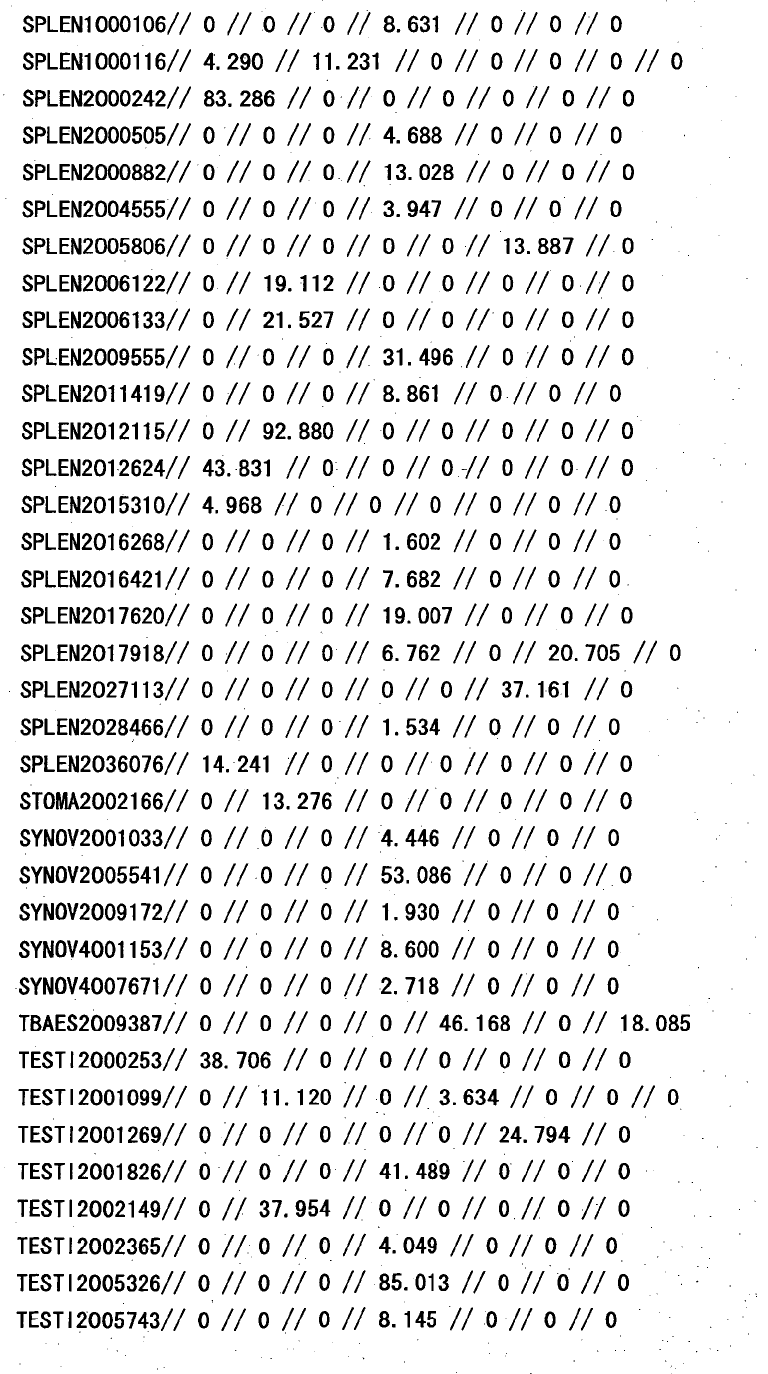

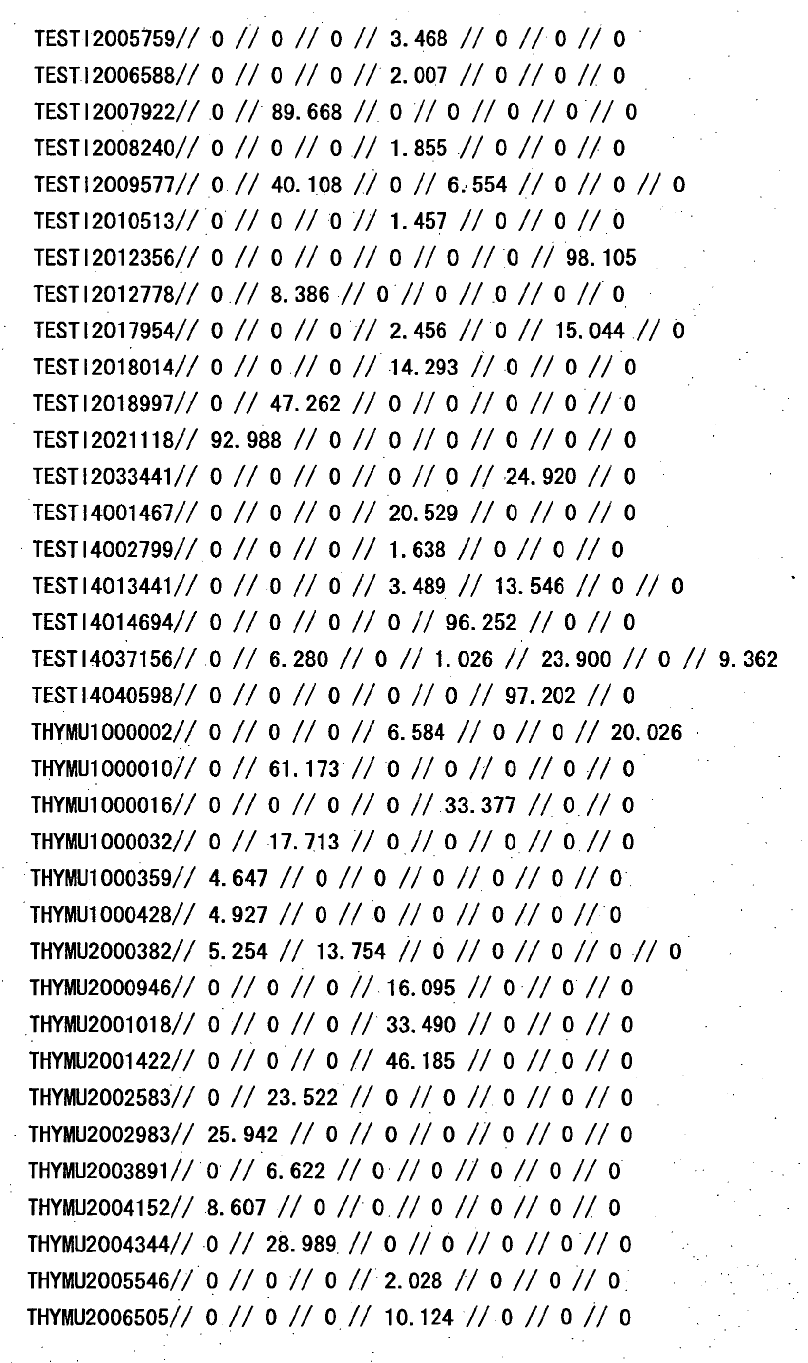

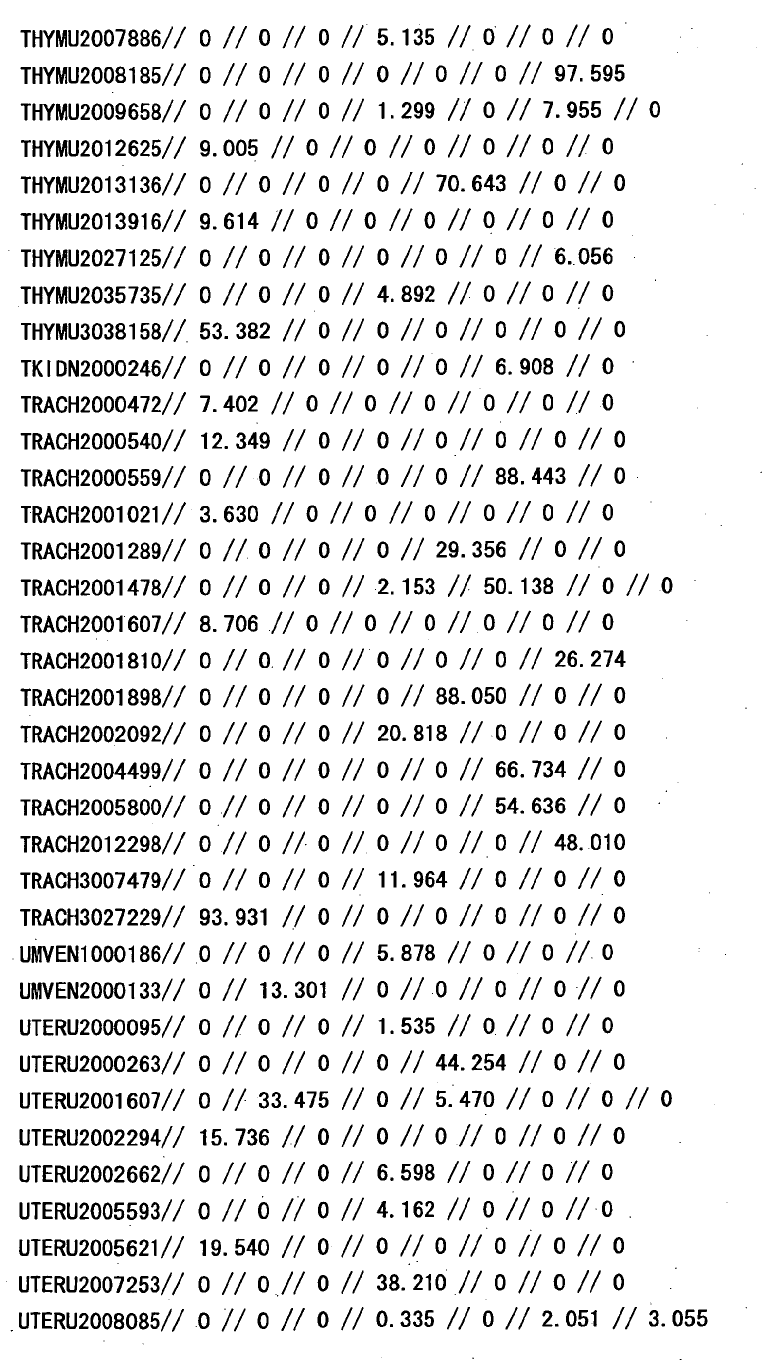

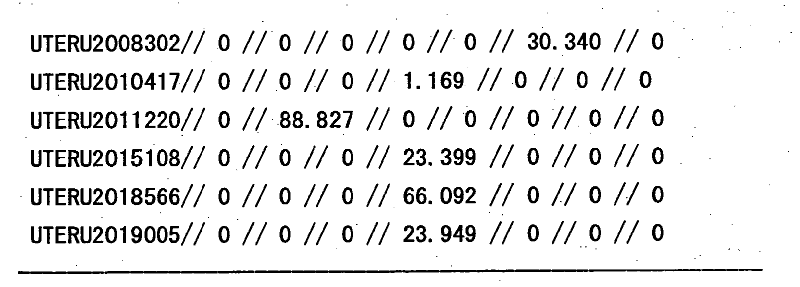

Genes whose expression levels were negligibly low in each of the above-described libraries are shown with their clone names below.
3NB691000018, 3NB691000085, 3NB691000113, 3NB691000129, 3NB691000173, 3NB691000191, 3NB692000012, 3NB692000029, 3NB692000102, 3NB692000154, 3NB692000276, 3NB692000305, 3NB692000330, 3NB692000374, 3NB692000429, 3NB692000484, 3NB692000529, 3NB692000545, 3NB692000973, 3NB692001002, 3NB692001022, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001853, 3NB692001861, 3NB692002051, 3NB692002365,
3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000006, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301,
ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002477, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817,
ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012179, AHMSC1000138, ASTRO1000018, ASTRO1000029, ASTRO1000096, ASTRO1000165, ASTRO2000011, ASTRO2000014, ASTRO2000095, ASTRO2000141, ASTRO2000191, ASTRO2000278, ASTRO2000372, ASTRO2000381, ASTRO2000403, ASTRO2000417, ASTRO2000480, ASTRO2000482, ASTRO2000533,
ASTRO2000653, ASTRO2000662, ASTRO2000725, ASTRO2000801, ASTRO2000914, ASTRO2001001, ASTRO2001008, ASTRO2001029, ASTRO2001076, ASTRO2001088, ASTRO2001107, ASTRO2001122, ASTRO2001227, ASTRO2001249, ASTRO2001480, ASTRO2001823, ASTRO2002024, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003024, ASTRO2003212, ASTRO2003316, ASTRO2003461, ASTRO2003574, ASTRO2003581, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2005008, ASTRO2005081, ASTRO2005096,
ASTRO2005242, ASTRO2005273, ASTRO2005343, ASTRO2005360, ASTRO2005413, ASTRO2005423, ASTRO2005553, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007047, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799,
ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015529, ASTRO2015987, ASTRO2016044, ASTRO2016114, ASTRO2016148, ASTRO2016491, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123,
BGGI11000193, BGGI11000285, BGGI12000022, BGGI12000054, BGGI12000067, BGGI12000161, BGGI12000533, BGGI12000544, BGGI12000616, BGGI12000684, BGGI12000693, BGGI12000839, BGGI12001002, BGGI12001075, BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474,
BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000153, BNGH41000169,
BNGH41000177, BNGH41000190, BNGH41000198, BNGH41000216, BNGH41000235, BNGH42000130, BNGH42000132, BNGH42000405, BNGH42000532, BNGH42000675, BNGH42000739, BNGH42000791, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005235, BNGH42005504, BNGH42006135, BNGH42006226, BNGH42006234, BNGH42007037, BNGH42007366, BNGH42007594, BNGH42007788, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020,
BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000124, BRACE1000131, BRACE1000132, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148,. BRACE2000183, BRACE2000229, BRACE2000232, BRACE2000245, BRACE2000256, BRACE2000266, BRACE2000267, BRACE2000280, BRACE2000300, BRACE2000307, BRACE2000331, BRACE2000332, BRACE2000347, BRACE2000351, BRACE2000368, BRACE2000392, BRACE2000441, BRACE2000487,
BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001143, BRACE2001151, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353,
BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001673, BRACE2001677, BRACE2001692, BRACE2001705, BRACE2001709, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972, BRACE2001973, BRACE2002026, BRACE2002034, BRACE2002050, BRACE2002081, BRACE2002091, BRACE2002139,
BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834,
BRACE2002849, BRACE2002857, BRACE2002860, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003269, BRACE2003285, BRACE2003304, BRACE2003319, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003825, BRACE2003827, BRACE2003845, BRACE2003847, BRACE2003848, BRACE2003873, BRACE2003885, BRACE2003887, BRACE2003892,
BRACE2003904, BRACE2003905, BRACE2003928, BRACE2003944, BRACE2003954, BRACE2003982, BRACE2003987, BRACE2005034, BRACE2005047, BRACE2005063, BRACE2005083, BRACE2005087, BRACE2005090, BRACE2005099, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005434, BRACE2005448, BRACE2005450, BRACE2005453, BRACE2005457, BRACE2005460, BRACE2005489, BRACE2005518, BRACE2005556, BRACE2005562, BRACE2005624,
BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281, BRACE2006298, BRACE2006319, BRACE2006344, BRACE2006354, BRACE2006363,
BRACE2006378, BRACE2006380, BRACE2006393, BRACE2006397, BRACE2006413, BRACE2006417, BRACE2006453, BRACE2006459, BRACE2006488, BRACE2006514, BRACE2006534, BRACE2006537, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006871, BRACE2006900, BRACE2006909, BRACE2006911, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007089, BRACE2007109, BRACE2007138, BRACE2007174, BRACE2007191, BRACE2007197, BRACE2007201, BRACE2007203, BRACE2007232,
BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007518, BRACE2007522, BRACE2007527, BRACE2007538, BRACE2007563, BRACE2007621, BRACE2007640, BRACE2007641, BRACE2007646, BRACE2007663, BRACE2007671, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944,
BRACE2007952, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883,
BRACE2008917, BRACE2008919, BRACE2008941, BRACE2008948, BRACE2008960, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009517, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957,
BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317,
BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194,
BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE-2018700, BRACE2018727, BRACE2018736, BRACE2018759, BRACE2018762, BRACE2018782, BRACE2018847, BRACE2019004, BRACE2019044, BRACE2019244, BRACE2019258, BRACE2019327, BRACE2019348, BRACE2019371, BRACE2019467, BRACE2019510, BRACE2019519,
BRACE2019528, BRACE2019618, BRACE2019696, BRACE2019703, BRACE2019843, BRACE2019865, BRACE2019872, BRACE-2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549,
BRACE2022633, BRACE2022638, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258,
BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584,
BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE-2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600,
BRACE2039607, BRACE2039624, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045947, BRACE2045954, BRACE2046251,
BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046,
BRACE3004058, BRACE3004113, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036,
BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010428, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418,
BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594,
BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241,
BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358,
BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988,
BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009482, BRALZ2009486, BRALZ2010186, BRALZ2010842, BRALZ2011337, BRALZ2011796, BRALZ2011880, BRALZ2012050,
BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000653, BRAMY2000814, BRAMY2000893, BRAMY2000946, BRAMY2000981, BRAMY2000987, BRAMY2001068, BRAMY2001114, BRAMY2001203,
BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004363, BRAMY2004387,
BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653,
BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480,
BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813,
BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055,
BRAMY2019111, BRAMY2019277, BRAMY2019300, BRAMY2019468, BRAMY2019509, BRAMY2019554, BRAMY2019643, BRAMY2019710, BRAMY2019963, BRAMY2019985, BRAMY2020050, BRAMY2020058, BRAMY2020106, BRAMY2020270, BRAMY2020354, BRAMY2020355, BRAMY2020412, BRAMY2020427, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493,
BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218,
BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028740, BRAMY2028783,
BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993,
BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214,
BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420,
BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078,
BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000037,
BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000460, BRAWH2000471, BRAWH2000476, BRAWH2000482, BRAWH2000488, BRAWH2000494, BRAWH2000500, BRAWH2000503, BRAWH2000522, BRAWH2000554, BRAWH2000595, BRAWH2000636, BRAWH2000643, BRAWH2000651, BRAWH2000682, BRAWH2000686,
BRAWH2000697, BRAWH2000752, BRAWH2000859, BRAWH2000866, BRAWH2000892, BRAWH2000901, BRAWH2000924, BRAWH2000944, BRAWH2000984, BRAWH2001019, BRAWH2001037, BRAWH2001052, BRAWH2001129, BRAWH2001137, BRAWH2001141, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406, BRAWH2001412, BRAWH2001418, BRAWH2001436, BRAWH2001438, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873,
BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025, BRAWH2003135, BRAWH2003240, BRAWH2003307, BRAWH2003355, BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004136, BRAWH2004217, BRAWH2004580, BRAWH2004731, BRAWH2004779, BRAWH2004842,
BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005661, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007605, BRAWH2007658, BRAWH2007664, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380,
BRAWH2008540, BRAWH2008577, BRAWH2008706, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400,
BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014645, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595,
BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018526, BRAWH2018527, BRAWH2018601, BRAWH2018745, BRAWH2018875, BRAWH2019053, BRAWH2019055, BRAWH2019198,
BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3002853, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047,
BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662,
BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017259, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172,
BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448,
BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223,
BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003944, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894,
BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566,
BRCAN2018667, BRCAN2018748, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026197, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028036,. BRCAN2028040, BRCAN2028319,
BRCAN2028338, BRCAN2028355, BRCAN2028378, BRCAN2028460, BRCAN2028545, BRCAN2028634, BRCAN2028702, BRCOC1000040, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164,
BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661,
BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000553, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172,
BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007305, BRHIP2007307, BRHIP2007616, BRHIP2007690, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414,
BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954,
BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842,
BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022221, BRHIP2022228, BRHIP2022298, BRHIP2022326, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061,
BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000111, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114,
BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007223, BRHIP3007239, BRHIP3007291,
BRHIP3007409, BRHIP3007424, BRHIP3007483, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675,
BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020046, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501,
BRHIP3027594, BRHIP3027651, BRHIP3027854, BRHIP3027947, BRHIP3028570, BRHIP3028742, BRSSN1000056, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133,
BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874,
BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532,
BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012174, BRSTN2012284, BRSTN2012320, BRSTN2012380, BRSTN2012451, BRSTN2013171, BRSTN2013354, BRSTN2013502, BRSTN2013931, BRSTN2014490, BRSTN2014633, BRSTN2014751,
BRSTN2014770, BRSTN2015015, BRSTN2015889, BRSTN2015978, BRSTN2016066, BRSTN2016335, BRSTN2016678, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726,
BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2008955,
BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663,
BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781,
BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252,
BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003225, BRTHA3003339, BRTHA3003343, BRTHA3003417, BRTHA3003449, BRTHA3003474, BRTHA3003490, BRTHA3003704, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310,
BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623,
BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770,
CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000052, CTONG1000056, CTONG1000062,
CTONG1000086, CTONG1000088, CTONG1000093, CTONG1000094, CTONG1000097, CTONG1000113, CTONG1000137, CTONG1000165,. CTONG1000175, CTONG1000180, CTONG1000181, CTONG1000220, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000411, CTONG2000452, CTONG2000465, CTONG2000469, CTONG2000480, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366,
CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001932, CTONG2001955, CTONG2002066, CTONG2002095, CTONG2002143, CTONG2002270, CTONG2002395, CTONG2002417, CTONG2002418, CTONG2002453, CTONG2002520, CTONG2002558, CTONG2002590, CTONG2002709, CTONG2002721, CTONG2002744, CTONG2002766, CTONG2002816, CTONG2002820, CTONG2002832, CTONG2002841, CTONG2002903, CTONG2002965, CTONG2002999, CTONG2003028, CTONG2003094, CTONG2003100, CTONG2003115, CTONG2003189, CTONG2003245, CTONG2003293, CTONG2003298, CTONG2003348, CTONG2003350, CTONG2003361,
CTONG2003372, CTONG2003375, CTONG2003427, CTONG2003524, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004550, CTONG2004669, CTONG2004716, CTONG2004912, CTONG2004941, CTONG2004948, CTONG2005049, CTONG2005110, CTONG2005145, CTONG2005265, CTONG2005278, CTONG2005290, CTONG2005374, CTONG2005399, CTONG2005468, CTONG2005567, CTONG2005615, CTONG2005635, CTONG2005775, CTONG2005795, CTONG2005890, CTONG2005904, CTONG2005913, CTONG2006129, CTONG2006223, CTONG2006273, CTONG2006310, CTONG2006377, CTONG2006393,
CTONG2006412, CTONG2006515, CTONG2006524, CTONG2006562, CTONG2006568, CTONG2006620, CTONG2006666, CTONG2006691, CTONG2006709, CTONG2006798, CTONG2006836, CTONG2006942, CTONG2006964, CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007500, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008405, CTONG2008466, CTONG2008506,
CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133,
CTONG2010148, CTONG2010276, CTONG2010330, CTONG2010408, CTONG2010508, CTONG2010566, CTONG2010623, CTONG2010649, CTONG2010652, CTONG2010803, CTONG2010821, CTONG2011188, CTONG2011429, CTONG2011474, CTONG2011676, CTONG2011716, CTONG2011765, CTONG2011779, CTONG2011801, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012230, CTONG2012422, CTONG2012425, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012847, CTONG2012901, CTONG2012996,
CTONG2013128, CTONG2013149, CTONG2013156, CTONG2013178, CTONG2013222, CTONG2013284, CTONG2013339, CTONG2013348, CTONG2013352, CTONG2013381, CTONG2013393, CTONG2013511, CTONG2013630, CTONG2013803, CTONG2013934, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014705, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717,
CTONG2015804, CTONG2015815, CTONG2015866, CTONG2015914, CTONG2015953, CTONG2016056, CTONG2016185, CTONG2016217, CTONG2016355, CTONG2016408, CTONG2016499, CTONG2016505, CTONG2016559, CTONG2016658, CTONG2016775, CTONG2016797, CTONG2016824, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017604, CTONG2017650, CTONG2017798, CTONG2017804, CTONG2017814, CTONG2017910, CTONG2017939, CTONG2017989, CTONG2018062, CTONG2018069, CTONG2018147, CTONG2018211, CTONG2018217, CTONG2018279, CTONG2018334, CTONG2018343, CTONG2018379, CTONG2018383, CTONG2018413, CTONG2018483,
CTONG2018629, CTONG2018632, CTONG2018637, CTONG2018652, CTONG2018653, CTONG2018655, CTONG2018735, CTONG2018739, CTONG2018808, CTONG2018843, CTONG2018898, CTONG2018900, CTONG2018976, CTONG2019029, CTONG2019063, CTONG2019144, CTONG2019232, CTONG2019248, CTONG2019400, CTONG2019652, CTONG2019704, CTONG2019788, CTONG2019833, CTONG2020031, CTONG2020108, CTONG2020127, CTONG2020171, CTONG2020374, CTONG2020378, CTONG2020411, CTONG2020445, CTONG2020522, CTONG2020560, CTONG2020582, CTONG2020638, CTONG2020806, CTONG2020831, CTONG2020974, CTONG2021024, CTONG2021132, CTONG2021168, CTONG2021289, CTONG2022153, CTONG2022601, CTONG2023021, CTONG2023449, CTONG2023512, CTONG2024031, CTONG2024206, CTONG2024614,
CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028124, CTONG2028208, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002835, CTONG3002909, CTONG3002947,
CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009239, CTONG3009287,
CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D60ST2000358, D6OST2000464, D6OST2000507, D9OST2000031, D9OST2000278, D9OST2000440, D9OST2000869, D9OST2001319, D9OST2001433, D9OST2002397, D9OST2002608, D9OST2002673, D9OST2003106,
D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000083, DFNES1000107, DFNES1000185, DFNES2000143, DFNES2000146, DFNES2000153, DFNES2000212, DFNES2000426, DFNES2000432, DFNES2000443, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004371, DFNES2004383,
DFNES2004608, DFNES2004684, DFNES2005266, DFNES2005527, DFNES2005540, DFNES2005690, DFNES2005766, DFNES2005779, DFNES2005875, DFNES2006346, DFNES2006944, DFNES2007113, DFNES2007332, DFNES2007634, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000024, FCBBF1000038, FCBBF1000053, FCBBF1000061, FCBBF1000063, FCBBF1000069, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182,
FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000476, FCBBF1000506, FCBBF1000509, FCBBF1000546, FCBBF1000550, FCBBF1000556, FCBBF1000563, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF1000760, FCBBF2000038, FCBBF2000087, FCBBF2000094,
FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000677, FCBBF2000678, FCBBF2000685, FCBBF2000733, FCBBF2000782, FCBBF2000808, FCBBF2000815, FCBBF2000825, FCBBF2000885, FCBBF2000940, FCBBF2000951, FCBBF2001001, FCBBF2001088, FCBBF2001105, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001594, FCBBF2001672, FCBBF2001675, FCBBF2001718, FCBBF2001720, FCBBF2001817, FCBBF2001868, FCBBF2002044,
FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005733, FCBBF2005909, FCBBF2005966, FCBBF2006131, FCBBF2006380, FCBBF2006418, FCBBF2006452, FCBBF2006634,
FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001470, FCBBF3001594, FCBBF3001632, FCBBF3001657, FCBBF3001818, FCBBF3001855, FCBBF3001890, FCBBF3001912, FCBBF3001914, FCBBF3001918, FCBBF3001924, FCBBF3001977,
FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005320, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453,
FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586,
FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178,
FCBBF3016451, FCBBF3016618, FCBBF3016622, FCBBF3016644, FCBBF3016667, FCBBF3016928, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599,
FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308,
FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022,
FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000129, FEBRA2000210, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452, FEBRA2000454, FEBRA2000462, FEBRA2000468, FEBRA2000491, FEBRA2000504, FEBRA2000510, FEBRA2000532, FEBRA2000536, FEBRA2000538, FEBRA2000575, FEBRA2000656,
FEBRA2000659, FEBRA2000680, FEBRA2000690, FEBRA2000727, FEBRA2000734, FEBRA2000740, FEBRA2000757, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001294, FEBRA2001334, FEBRA2001351, FEBRA2001383, FEBRA2001438,
FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001828, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002552, FEBRA2002574, FEBRA2002601, FEBRA2002611, FEBRA2002628, FEBRA2002662, FEBRA2002682,
FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003100, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003750, FEBRA2003897, FEBRA2003907, FEBRA2003930, FEBRA2004023, FEBRA2004026, FEBRA2004029, FEBRA2004042, FEBRA2004053, FEBRA2004056, FEBRA2004091, FEBRA2004110,
FEBRA2004191, FEBRA2004202, FEBRA2004219, FEBRA2004237, FEBRA2004268, FEBRA2004293, FEBRA2004325, FEBRA2004329, FEBRA2004331, FEBRA2004412, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005701, FEBRA2005726, FEBRA2005734, FEBRA2005778, FEBRA2005787, FEBRA2005788, FEBRA2005824, FEBRA2005995, FEBRA2005998, FEBRA2006055,
FEBRA2006061, FEBRA2006092, FEBRA2006112, FEBRA2006150, FEBRA2006165, FEBRA2006255, FEBRA2006315, FEBRA2006346, FEBRA2006354, FEBRA2006357, FEBRA2006368, FEBRA2006370, FEBRA2006372, FEBRA2006379, FEBRA2006396, FEBRA2006401, FEBRA2006409, FEBRA2006418, FEBRA2006427, FEBRA2006476, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566,
FEBRA2007589, FEBRA2007613, FEBRA2007620, FEBRA2007622, FEBRA2007708, FEBRA2007714, FEBRA2007793, FEBRA2007818, FEBRA2007823, FEBRA2007880, FEBRA2007900, FEBRA2007901, FEBRA2007931, FEBRA2007957, FEBRA2008009, FEBRA2008036, FEBRA2008086, FEBRA2008087, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008287, FEBRA2008311, FEBRA2008341, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928,
FEBRA2008983, FEBRA2009016, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397,
FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014417, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433,
FEBRA2018476, FEBRA2018746, FEBRA2019172, FEBRA2019242, FEBRA2019389, FEBRA2019457, FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022055, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285,
FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2025838, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEBRA2028516, FEHRT2000325, FEHRT2000364, FEKID1000018, FELIV1000101, FELIV1000137, FELIV1000164,
FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000131, HCHON1000142,
HCHON1000166, HCHON1000176, HCHON2000038, HCHON2000062, HCHON2000087, HCHON2000100, HCHON2000156, HCHON2000160, HCHON2000199, HCHON2000212, HCHON2000265, HCHON2000271, HCHON2000315, HCHON2000322, HCHON2000323, HCHON2000344, HCHON2000395, HCHON2000417, HCHON2000418, HCHON2000473, HCHON2000475, HCHON2000503, HCHON2000626, HCHON2000660, HCHON2000663, HCHON2000673, HCHON2000698, HCHON2000699, HCHON2000738, HCHON2000743, HCHON2000815, HCHON2000817, HCHON2000818, HCHON2000826, HCHON2000832, HCHON2000851, HCHON2000898, HCHON2000935, HCHON2000942, HCHON2000956, HCHON2000994, HCHON2001039, HCHON2001050, HCHON2001084, HCHON2001099, HCHON2001110, HCHON2001116, HCHON2001161, HCHON2001214, HCHON2001233,
HCHON2001269, HCHON2001359, HCHON2001407, HCHON2001434, HCHON2001450, HCHON2001453, HCHON2001497, HCHON2001505, HCHON2001519, HCHON2001523, HCHON2001535, HCHON2001546, HCHON2001548, HCHON2001577, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004002, HCHON2004007, HCHON2004279, HCHON2004359, HCHON2004531, HCHON2004776, HCHON2004858, HCHON2005048, HCHON2005118, HCHON2005166,
HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000074, HEART1000088, HEART1000099, HEART1000102, HEART1000118, HEART1000132, HEART1000139, HEART1000142, HEART1000149, HEART1000151, HEART1000173, HEART1000185, HEART2000024, HEART2000035, HEART2000099, HEART2000298, HEART2000306,
HEART2000309, HEART2000411, HEART2000418, HEART2000444, HEART2000448, HEART2000492, HEART2000506, HEART2000520, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443,
HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000114, HHDPC1000118, HHDPC1000163, HHDPC2000055, HHDPC2000070, HHDPC2000102, HHDPC2000104, HHDPC2000115, HHDPC2000149, HHDPC2000258, HHDPC2000315, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007267,
HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000055, HLUNG1000076, HLUNG1000084, HLUNG1000091, HLUNG1000099, HLUNG1000104, HLUNG1000105, HLUNG1000110, HLUNG1000151, HLUNG1000169, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363,
HLUNG2000412, HLUNG2000431, HLUNG2000480, HLUNG2000489, HLUNG2000501, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013, HLUNG2001126, HLUNG2001144, HLUNG2001146, HLUNG2001214, HLUNG2001439, HLUNG2001459, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002648, HLUNG2002659, HLUNG2002707, HLUNG2002811, HLUNG2002916, HLUNG2002942, HLUNG2002949, HLUNG2002958,
HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004159, HLUNG2004170, HLUNG2004217, HLUNG2004273, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026,
HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009067, HLUNG2009215, HLUNG2009225, HLUNG2009253, HLUNG2009303, HLUNG2009413, HLUNG2009729, HLUNG2010181, HLUNG2010464, HLUNG2010562, HLUNG2010845,
HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017262, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA1000178, HSYRA2000044, HSYRA2000135, HSYRA2000137, HSYRA2000190, HSYRA2000221, HSYRA2000224, HSYRA2000232,
HSYRA2000237, HSYRA2000248, HSYRA2000253, HSYRA2000371, HSYRA2000424, HSYRA2000507, HSYRA2000510, HSYRA2000605, HSYRA2000629, HSYRA2000640, HSYRA2000706, HSYRA2000743, HSYRA2000760, HSYRA2000787, HSYRA2000792, HSYRA2000828, HSYRA2000832, HSYRA2000927, HSYRA2001003, HSYRA2001105, HSYRA2001142, HSYRA2001153, HSYRA2001232, HSYRA2001255, HSYRA2001332, HSYRA2001353, HSYRA2001396, HSYRA2001420, HSYRA2001484, HSYRA2001552, HSYRA2001567, HSYRA2001580, HSYRA2001595, HSYRA2001631, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007338, HSYRA2007650,
HSYRA2008154, HSYRA2008714, HSYRA2009102, IMR321000155, IMR321000158, IMR321000161, IMR321000165, IMR321000193, IMR321000207, IMR321000210, IMR321000230, IMR321000242, IMR321000266, IMR322000010, IMR322000107, IMR322000127, IMR322000144, IMR322000152, IMR322000164, IMR322000178, IMR322000223, IMR322000302, IMR322000316, IMR322000374, IMR322000379, IMR322000383, IMR322000418, IMR322000518, IMR322000555, IMR322000604, IMR322000609, IMR322000656, IMR322000672, IMR322000730, IMR322000742, IMR322000775, IMR322000791, IMR322000811, IMR322000819, IMR322000838, IMR322000859, IMR322000863, IMR322000917, IMR322000919, IMR322000953, IMR322000984, IMR322001018, IMR322001049, IMR322001167, IMR322001185,
IMR322001218, IMR322001245, IMR322001317, IMR322001318, IMR322001322, IMR322001332, IMR322001380, IMR322001438, IMR322001452, IMR322001491, IMR322001534, IMR322001541, IMR322001665, IMR322001670, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322013053, IMR322013396, IMR322013731, IMR322013805, IMR322015523, IMR322016146, IMR322017049, IMR322018117, IMR322018192, IMR322019070,
JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000145, KIDNE1000152, KIDNE2000026, KIDNE2000041, KIDNE2000046, KIDNE2000051, KIDNE2000070, KIDNE2000085, KIDNE2000167, KIDNE2000174, KIDNE2000192, KIDNE2000227, KIDNE2000244, KIDNE2000245, KIDNE2000266, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000403, KIDNE2000422, KIDNE2000493, KIDNE2000497, KIDNE2000510, KIDNE2000513, KIDNE2000517, KIDNE2000519, KIDNE2000555, KIDNE2000624, KIDNE2000665, KIDNE2000678, KIDNE2000717, KIDNE2000721, KIDNE2000722,
KIDNE2000777, KIDNE2000801, KIDNE2000832, KIDNE2000833, KIDNE2000846, KIDNE2000867, KIDNE2000909, KIDNE2000947, KIDNE2001117, KIDNE2001140, KIDNE2001162, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002438, KIDNE2002483, KIDNE2002725, KIDNE2002768, KIDNE2002795, KIDNE2002798, KIDNE2002839, KIDNE2002845, KIDNE2002872, KIDNE2002883, KIDNE2002980, KIDNE2002991, KIDNE2003185, KIDNE2003188, KIDNE2003233, KIDNE2003305, KIDNE2003316, KIDNE2003335, KIDNE2003357, KIDNE2003373,
KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004475, KIDNE2004519, KIDNE2004520, KIDNE2004531, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629,
KIDNE2005676, KIDNE2005781, KIDNE2005798, KIDNE2005854, KIDNE2005908, KIDNE2005937, KIDNE2006009, KIDNE2006014, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2007005, KIDNE2007040, KIDNE2007077, KIDNE2007186, KIDNE2007222, KIDNE2007328, KIDNE2007352, KIDNE2007356, KIDNE2007422, KIDNE2007569, KIDNE2007810, KIDNE2007811, KIDNE2007910, KIDNE2007944, KIDNE2007954, KIDNE2008022, KIDNE2008069, KIDNE2008072, KIDNE2008117, KIDNE2008218,
KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750,
KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298,
KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071,
KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062,
NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000091, NOVAR1000102, NOVAR2000038, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000174, NT2NE2000195, NT2NE2000222, NT2NE2000259, NT2NE2000284, NT2NE2000299,
NT2NE2000327, NT2NE2000353, NT2NE2000356, NT2NE2000369, NT2NE2000374, NT2NE2000383, NT2NE2000384, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000575, NT2NE2000586, NT2NE2000612, NT2NE2000636, NT2NE2000658, NT2NE2000706, NT2NE2000707, NT2NE2000763, NT2NE2000802, NT2NE2000808, NT2NE2000809, NT2NE2000864, NT2NE2000909, NT2NE2000950, NT2NE2000951, NT2NE2000963, NT2NE2000980, NT2NE2001005, NT2NE2001021, NT2NE2001040, NT2NE2001048, NT2NE2001049, NT2NE2001142, NT2NE2001156, NT2NE2001176, NT2NE2001180, NT2NE2001181, NT2NE2001188, NT2NE2001191, NT2NE2001247, NT2NE2001252, NT2NE2001324,
NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003185, NT2NE2003252, NT2NE2003280, NT2NE2003308, NT2NE2003309, NT2NE2003315, NT2NE2003393, NT2NE2003408,
NT2NE2003457, NT2NE2003485, NT2NE2003540, NT2NE2003569, NT2NE2003571, NT2NE2003705, NT2NE2003752, NT2NE2003921, NT2NE2004255, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006458, NT2NE2006478,
NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107,
NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511,
NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000035, NT2RI1000048, NT2RI1000127, NT2RI1000164, NT2RI1000172, NT2RI2000004, NT2RI2000007,
NT2RI2000107, NT2RI2000116, NT2RI2000117, NT2RI2000128, NT2RI2000133, NT2RI2000167, NT2RI2000173, NT2RI2000187, NT2RI2000263, NT2RI2000270, NT2RI2000276, NT2RI2000294, NT2RI2000313, NT2RI2000341, NT2RI2000348, NT2RI2000412, NT2RI2000421, NT2RI2000480, NT2RI2000575, NT2RI2000588, NT2RI2000597, NT2RI2000669, NT2RI2000685, NT2RI2000688, NT2RI2000689, NT2RI2000704, NT2RI2000738, NT2RI2000775, NT2RI2000822, NT2RI2000829, NT2RI2000841, NT2RI2000932, NT2RI2000974, NT2RI2000987, NT2RI2001010, NT2RI2001017, NT2RI2001030, NT2RI2001083, NT2RI2001091, NT2RI2001167, NT2RI2001178, NT2RI2001230, NT2RI2001235, NT2RI2001244, NT2RI2001307, NT2RI2001342, NT2RI2001385, NT2RI2001409, NT2RI2001410, NT2RI2001422,
NT2RI2001425, NT2RI2001449, NT2RI2001450, NT2RI2001474, NT2RI2001519, NT2RI2001595, NT2RI2001621, NT2RI2001624, NT2RI2001726, NT2RI2001782, NT2RI2001836, NT2RI2001866, NT2RI2001910, NT2RI2001952, NT2RI2002022, NT2RI2002041, NT2RI2002117, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002391, NT2RI2002447, NT2RI2002481, NT2RI2002507, NT2RI2002517, NT2RI2002540, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819, NT2RI2002847, NT2RI2002852, NT2RI2002865, NT2RI2002923, NT2RI2002926, NT2RI2002958, NT2RI2002970,
NT2RI2003011, NT2RI2003019, NT2RI2003067, NT2RI2003184, NT2RI2003205, NT2RI2003222, NT2RI2003301, NT2RI2003317, NT2RI2003338, NT2RI2003344, NT2RI2003360, NT2RI2003361, NT2RI2003383, NT2RI2003392, NT2RI2003399, NT2RI2003419, NT2RI2003420, NT2RI2003481, NT2RI2003487, NT2RI2003556, NT2RI2003655, NT2RI2003667, NT2RI2003678, NT2RI2003695, NT2RI2003884, NT2RI2003921, NT2RI2003973, NT2RI2003993, NT2RI2004059, NT2RI2004078, NT2RI2004079, NT2RI2004093, NT2RI2004136, NT2RI2004157, NT2RI2004190, NT2RI2004209, NT2RI2004230, NT2RI2004290, NT2RI2004304, NT2RI2004312, NT2RI2004381, NT2RI2004398, NT2RI2004410, NT2RI2004442, NT2RI2004468, NT2RI2004535, NT2RI2004606, NT2RI2004608, NT2RI2004618, NT2RI2004783,
NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005130, NT2RI2005150, NT2RI2005166, NT2RI2005239, NT2RI2005246, NT2RI2005315, NT2RI2005353, NT2RI2005358, NT2RI2005368, NT2RI2005382, NT2RI2005473, NT2RI2005520, NT2RI2005564, NT2RI2005621, NT2RI2005628, NT2RI2005647, NT2RI2005670, NT2RI2005713, NT2RI2005723, NT2RI2005772, NT2RI2005811, NT2RI2005816, NT2RI2005818, NT2RI2005851, NT2RI2005966, NT2RI2006070, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487,
NT2RI2006506, NT2RI2006553, NT2RI2006565, NT2RI2006640, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006825, NT2RI2006838, NT2RI2006853, NT2RI2006855, NT2RI2006856, NT2RI2006943, NT2RI2006973, NT2RI2007046, NT2RI2007048, NT2RI2007054, NT2RI2007084, NT2RI2007096, NT2RI2007116, NT2RI2007148, NT2RI2007214, NT2RI2007303, NT2RI2007334, NT2RI2007377, .NT2RI2007386, NT2RI2007406, NT2RI2007439, NT2RI2007445, NT2RI2007468, NT2RI2007469, NT2RI2007507, NT2RI2007589, NT2RI2007593, NT2RI2007629, NT2RI2007723, NT2RI2007729, NT2RI2007751, NT2RI2007754, NT2RI2007877, NT2RI2007879, NT2RI2007884,
NT2RI2007891, NT2RI2007956, NT2RI2007965, NT2RI2007987, NT2RI2008007, NT2RI2008050, NT2RI2008171, NT2RI2008188, NT2RI2008204, NT2RI2008221, NT2RI2008233, NT2RI2008396, NT2RI2008455, NT2RI2008481, NT2RI2008502, NT2RI2008566, NT2RI2008598, NT2RI2008622, NT2RI2008656, NT2RI2008714, NT2RI2008749, NT2RI2008801, NT2RI2008808, NT2RI2008812, NT2RI2008841, NT2RI2008942, NT2RI2008952, NT2RI2009005, NT2RI2009037, NT2RI2009066, NT2RI2009083, NT2RI2009101, NT2RI2009103, NT2RI2009144, NT2RI2009151, NT2RI2009173, NT2RI2009194, NT2RI2009215, NT2RI2009239, NT2RI2009259, NT2RI2009269, NT2RI2009281, NT2RI2009289, NT2RI2009301, NT2RI2009402, NT2RI2009406, NT2RI2009517, NT2RI2009585, NT2RI2009595, NT2RI2009855,
NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2015342, NT2RI2015533, NT2RI2016128, NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976,
NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171,
NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000017, NT2RP6000032, NT2RP6000033, NT2RP6000039, NT2RP6000043, NT2RP6000059, NT2RP6000060, NT2RP6000078, NT2RP6000085, NT2RP6000086, NT2RP6000100, NT2RP6000109, NT2RP6000110, NT2RP6000119, NT2RP6000123, NT2RP6000127, NT2RP6000130, NT2RP6000140, NT2RP6000150, NT2RP7000041,
NT2RP7000069, NT2RP7000076, NT2RP7000086, NT2RP7000109, NT2RP7000112, NT2RP7000173, NT2RP7000259, NT2RP7000271, NT2RP7000311, NT2RP7000359, NT2RP7000364, NT2RP7000380, NT2RP7000391, NT2RP7000425, NT2RP7000477, NT2RP7000579, NT2RP7000586, NT2RP7000600, NT2RP7000812, NT2RP7000906, NT2RP7000926, NT2RP7001074, NT2RP7001080, NT2RP7001156, NT2RP7001231, NT2RP7001283, NT2RP7001306, NT2RP7001319, NT2RP7001335, NT2RP7001364, NT2RP7001475, NT2RP7001532, NT2RP7001591, NT2RP7001602, NT2RP7001678, NT2RP7001856, NT2RP7001962, NT2RP7002028, NT2RP7002064, NT2RP7002151, NT2RP7002163, NT2RP7002243, NT2RP7002282, NT2RP7002376, NT2RP7002449, NT2RP7002478, NT2RP7002554, NT2RP7002594, NT2RP7002603, NT2RP7002650,
NT2RP7002738, NT2RP7002779, NT2RP7002829, NT2RP7002841, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203, NT2RP7003261, NT2RP7003319, NT2RP7003439, NT2RP7003511, NT2RP7003629, NT2RP7003632, NT2RP7003647, NT2RP7003680, NT2RP7003688, NT2RP7003724, NT2RP7004027, NT2RP7004080, NT2RP7004196, NT2RP7004233, NT2RP7004260, NT2RP7004348, NT2RP7004352, NT2RP7004358, NT2RP7004373, NT2RP7004396, NT2RP7004428, NT2RP7004481, NT2RP7004541, NT2RP7004559, NT2RP7004641, NT2RP7004656, NT2RP7004687, NT2RP7004722, NT2RP7004751, NT2RP7004766, NT2RP7004790, NT2RP7004884, NT2RP7004911, NT2RP7004915,
NT2RP7004925, NT2RP7004946, NT2RP7004961, NT2RP7004964, NT2RP7004975, NT2RP7005118, NT2RP7005205, NT2RP7005219, NT2RP7005323, NT2RP7005468, NT2RP7005493, NT2RP7005529, NT2RP7005629, NT2RP7005631, NT2RP7005669, NT2RP7005675, NT2RP7005846, NT2RP7006033, NT2RP7006075, NT2RP7006160, NT2RP7006162, NT2RP7006223, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006619, NT2RP7006621, NT2RP7006636, NT2RP7006701, NT2RP7006853, NT2RP7006886, NT2RP7006887, NT2RP7006980, NT2RP7006986, NT2RP7006995, NT2RP7007114, NT2RP7007177, NT2RP7007221, NT2RP7007226, NT2RP7007252, NT2RP7007269, NT2RP7007310, NT2RP7007359,
NT2RP7007381, NT2RP7007387, NT2RP7007406, NT2RP7007480, NT2RP7007504, NT2RP7007530, NT2RP7007537, NT2RP7007580, NT2RP7007594, NT2RP7007610, NT2RP7007617, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008161, NT2RP7008167, NT2RP7008190, NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009108, NT2RP7009147, NT2RP7009149, NT2RP7009168,
NT2RP7009215, NT2RP7009225, NT2RP7009236, NT2RP7009253, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009482, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005,
NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435,
NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000033, NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000246, NTONG1000247, NTONG1000257, NTONG2000040, NTONG2000107, NTONG2000231, NTONG2000334, NTONG2000363, NTONG2000413, NTONG2000492, NTONG2000499, NTONG2000531, NTONG2000583, NTONG2000800, NTONG2000858, NTONG2000878,
NTONG2000966, NTONG2000985, NTONG2001014, NTONG2001079, NTONG2001157, NTONG2001173, NTONG2001220, NTONG2001222, NTONG2001362, NTONG2001428, NTONG2001532, NTONG2001550, NTONG2001587, NTONG2001612, NTONG2001619, NTONG2001642, NTONG2001657, NTONG2001676, NTONG2001762, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003852, NTONG2003897, NTONG2003928, NTONG2004095, NTONG2004308, NTONG2004521, NTONG2004614, NTONG2004690, NTONG2004806, NTONG2004829, NTONG2004918, NTONG2004991, NTONG2005062, NTONG2005086, NTONG2005137, NTONG2005153, NTONG2005265, NTONG2005277,
NTONG2005363, NTONG2005373, NTONG2005391, NTONG2005480, NTONG2005497, NTONG2005577, NTONG2005657, NTONG2005801, NTONG2005822, NTONG2005897, NTONG2005969, NTONG2006099, NTONG2006187, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054,
OCBBF1000067, OCBBF1000080, OCBBF1000085, OCBBF1000086, OCBBF1000091, OCBBF1000104, OCBBF1000114, OCBBF1000118, OCBBF1000119, OCBBF1000122, OCBBF1000130, OCBBF1000138, OCBBF1000145, OCBBF1000146, OCBBF1000153, OCBBF1000175, OCBBF1000185, OCBBF1000188, OCBBF1000192, OCBBF1000254, OCBBF1000315, OCBBF2000015, OCBBF2000126, OCBBF2000178, OCBBF2000231, OCBBF2000277, OCBBF2000287, OCBBF2000323, OCBBF2000452, OCBBF2000467, OCBBF2000677, OCBBF2000703, OCBBF2000719, OCBBF2000824, OCBBF2000831, OCBBF2000904, OCBBF2000998, OCBBF2001075, OCBBF2001101, OCBBF2001124, OCBBF2001140, OCBBF2001166, OCBBF2001176, OCBBF2001186, OCBBF2001196, OCBBF2001210, OCBBF2001252, OCBBF2001307, OCBBF2001323, OCBBF2001389,
OCBBF2001402, OCBBF2001408, OCBBF2001416, OCBBF2001473, OCBBF2001492, OCBBF2001494, OCBBF2001527, OCBBF2001528, OCBBF2001586, OCBBF2001639, OCBBF2001681, OCBBF2001706, OCBBF2001749, OCBBF2001794, OCBBF2001910, OCBBF2001931, OCBBF2001961, OCBBF2001983, OCBBF2002015, OCBBF2002083, OCBBF2002086, OCBBF2002124, OCBBF2002161, OCBBF2002290, OCBBF2002357, OCBBF2002376, OCBBF2002615, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038, OCBBF2004104, OCBBF2004147, OCBBF2004168, OCBBF2004235, OCBBF2004259,
OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006859,
OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007238, OCBBF2007354, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007829, OCBBF2007892, OCBBF2007931, OCBBF2007946, OCBBF2008005, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775,
OCBBF2008790, OCBBF2008814, OCBBF2009095, OCBBF2009115, OCBBF2009242, OCBBF2009301, OCBBF2009352, OCBBF2009391, OCBBF2009424, OCBBF2009536, OCBBF2009571, OCBBF2009583, OCBBF2009603, OCBBF2009772, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010830, OCBBF2010841, OCBBF2010843, OCBBF2010858, OCBBF2010863, OCBBF2010871, OCBBF2010931, OCBBF2010945, OCBBF2010978, OCBBF2010989, OCBBF2011073, OCBBF2011137, OCBBF2011160, OCBBF2011177, OCBBF2011228, OCBBF2011232, OCBBF2011283, OCBBF2011311, OCBBF2011536, OCBBF2011625,
OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013843, OCBBF2013883,
OCBBF2013926, OCBBF2014052, OCBBF2014064, OCBBF2014089, OCBBF2014292, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016928, OCBBF2017035, OCBBF2017055, OCBBF2017069, OCBBF2017306, OCBBF2017325, OCBBF2017398, OCBBF2017458, OCBBF2017489,
OCBBF2017516, OCBBF2017665, OCBBF2017754, OCBBF2017791, OCBBF2017815, OCBBF2017882, OCBBF2017888, OCBBF2017893, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018707, OCBBF2018827, OCBBF2018828, OCBBF2018934, OCBBF2018956, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833,
OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708,
OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000323, OCBBF3000372,
OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000180, PEBLM2000030,
PEBLM2000112, PEBLM2000126, PEBLM2000147, PEBLM2000170, PEBLM2000180, PEBLM2000213, PEBLM2000248, PEBLM2000270, PEBLM2000308, PEBLM2000321, PEBLM2000326, PEBLM2000333, PEBLM2000338, PEBLM2000395, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709,
PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259,
PERIC2004379, PERIC2004429, PERIC2004510, PERIC2004613, PERIC2004909, PERIC2005111, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000070, PLACE5000080, PLACE5000105, PLACE5000113, PLACE5000115, PLACE5000116, PLACE5000129, PLACE5000139, PLACE5000153, PLACE5000159, PLACE5000170, PLACE5000171, PLACE5000178, PLACE5000245, PLACE5000260, PLACE5000282, PLACE5000297, PLACE5000372, PLACE5000492, PLACE5000522,
PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000314, PLACE6000325, PLACE6000348, PLACE6000371, PLACE6000414, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001064, PLACE6001118, PLACE6001153, PLACE6001185, PLACE6001262, PLACE6001281, PLACE6001294, PLACE6001443, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002102,
PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003383, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004312, PLACE6004336, PLACE6004368, PLACE6004380, PLACE6004396, PLACE6004397, PLACE6004454, PLACE6004464, PLACE6004491, PLACE6004578, PLACE6004663, PLACE6004687, PLACE6004738,
PLACE6004931, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687,
PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008775, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011057, PLACE6011102, PLACE6011156, PLACE6011211, PLACE6011260, PLACE6011334,
PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016383, PLACE6016942, PLACE6016984, PLACE6017002, PLACE6017431,
PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169,
PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000097, PROST1000110, PROST1000120, PROST1000123, PROST1000136, PROST1000145, PROST1000152, PROST1000167, PROST1000184, PROST1000215, PROST1000217, PROST1000220, PROST1000226, PROST1000246, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000480,
PROST1000485, PROST1000493, PROST1000512, PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000176, PROST2000203, PROST2000206, PROST2000212, PROST2000241, PROST2000267, PROST2000273, PROST2000274, PROST2000277, PROST2000284, PROST2000325, PROST2000337, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001180, PROST2001213, PROST2001283, PROST2001414, PROST2001415, PROST2001465, PROST2001521,
PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303, PROST2003324, PROST2003338, PROST2003340, PROST2003396, PROST2003428, PROST2003472, PROST2003537, PROST2003563,
PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004258, PROST2004332, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426, PROST2005466, PROST2005604, PROST2005630, PROST2005683, PROST2005788, PROST2005880, PROST2005886, PROST2005904, PROST2005919, PROST2005943, PROST2006008, PROST2006020, PROST2006030,
PROST2006060, PROST2006196, PROST2006201, PROST2006260, PROST2006282, PROST2006343, PROST2006450, PROST2006491, PROST2006510, PROST2006536, PROST2006579, PROST2006688, PROST2006737, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008271, PROST2008447, PROST2008468, PROST2008472, PROST2008489, PROST2008516, PROST2008724, PROST2008835, PROST2008993, PROST2009022,
PROST2009208, PROST2009222, PROST2009254, PROST2009273, PROST2009320, PROST2009347, PROST2009365, PROST2009388, PROST2009400, PROST2009470, PROST2009483, PROST2009571, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010885, PROST2011012, PROST2011038, PROST2011105, PROST2011297, PROST2011410, PROST2011439, PROST2011482, PROST2011577, PROST2011631, PROST2011660, PROST2012005, PROST2012007, PROST2012016, PROST2012088, PROST2012140, PROST2012157, PROST2012190, PROST2012249, PROST2012353, PROST2012400, PROST2012448, PROST2012504, PROST2012542,
PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512,
PROST2016566, PROST2016582, PROST2016668, PROST2016684, PROST2016777, PROST2016829, PROST2016860, PROST2016918, PROST2016980, PROST2017098, PROST2017128, PROST2017203, PROST2017367, PROST2017413, PROST2017441, PROST2017529, PROST2017578, PROST2017612, PROST2017617, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000065, PUAEN1000081, PUAEN1000085, PUAEN1000087,
PUAEN1000121, PUAEN1000147, PUAEN1000161, PUAEN1000175, PUAEN1000239, PUAEN1000275, PUAEN1000276, PUAEN1000288, PUAEN2000080, PUAEN2000152, PUAEN2000247, PUAEN2000312, PUAEN2000394, PUAEN2000497, PUAEN2000535, PUAEN2000684, PUAEN2000942, PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761,
PUAEN2007044, PUAEN2007350, PUAEN2007785, PUAEN2007898, PUAEN2008123, PUAEN2008228, PUAEN2008515, PUAEN2008815, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000030, SKMUS1000064, SKMUS1000067, SKMUS1000084, SKMUS1000102, SKMUS1000104, SKMUS1000110, SKMUS1000118,
SKMUS1000124, SKMUS1000129, SKMUS1000138, SKMUS1000176, SKMUS1000177, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000361, SKMUS2000365, SKMUS2000380, SKMUS2000390, SKMUS2000416, SKMUS2000458, SKMUS2000467, SKMUS2000468, SKMUS2000515, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000902, SKMUS2000933, SKMUS2000945, SKMUS2000954, SKMUS2000961, SKMUS2000973, SKMUS2000980,
SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001528, SKMUS2001543, SKMUS2001580, SKMUS2001585, SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034,
SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000159, SKNMC1000168, SKNMC1000229, SKNMC1000251, SKNMC1000264, SKNMC2000065, SKNMC2000097, SKNMC2000224, SKNMC2000256, SKNMC2000322, SKNMC2000356, SKNMC2000374, SKNMC2000583, SKNMC2000593, SKNMC2000612, SKNMC2000622, SKNMC2000649,
SKNMC2000698, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000101, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000347, SKNSH2000482, SKNSH2000516, SKNSH2000550, SKNSH2000716, SKNSH2000819, SKNSH2000971, SKNSH2001222, SKNSH2001875, SKNSH2001931, SKNSH2002054, SKNSH2002325, SKNSH2002768,
SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008969, SKNSH2009197, SKNSH2009357, SKNSH2009435, SKNSH2009991, SKNSH2010015, SMINT1000012, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000131, SMINT1000137, SMINT1000192, SMINT2000007,
SMINT2000018, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000454, SMINT2000468, SMINT2000518, SMINT2000529, SMINT2000530, SMINT2000537, SMINT2000538, SMINT2000541, SMINT2000545, SMINT2000558, SMINT2000562, SMINT2000568, SMINT2000602, SMINT2000609, SMINT2000629, SMINT2000659, SMINT2000747, SMINT2000811, SMINT2000824, SMINT2000933, SMINT2000984, SMINT2001114, SMINT2001158, SMINT2001183, SMINT2001199, SMINT2001222, SMINT2001397, SMINT2001458, SMINT2001461, SMINT2001535, SMINT2001559, SMINT2001609, SMINT2001615, SMINT2001683,
SMINT2001731, SMINT2001812, SMINT2001950, SMINT2002126, SMINT2002159, SMINT2002202, SMINT2002210, SMINT2002224, SMINT2002281, SMINT2002311, SMINT2002314, SMINT2002328, SMINT2002414, SMINT2002457, SMINT2002620, SMINT2002689, SMINT2002743, SMINT2002778, SMINT2002880, SMINT2002882, SMINT2002884, SMINT2002899, SMINT2002974, SMINT2002976, SMINT2003003, SMINT2003074, SMINT2003128, SMINT2003150, SMINT2003317, SMINT2003340, SMINT2003386, SMINT2003423, SMINT2003505, SMINT2003551, SMINT2003569, SMINT2003641, SMINT2003644, SMINT2003866, SMINT2003905, SMINT2004023, SMINT2004037, SMINT2004047, SMINT2004086, SMINT2004280, SMINT2004299, SMINT2004339, SMINT2004350, SMINT2004414, SMINT2004473, SMINT2004547,
SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796,
SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008960, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369,
SMINT2010426, SMINT2010500, SMINT2010533, SMINT2010629, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012122, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294,
SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134,
SPLEN1000139, SPLEN1000143, SPLEN1000144, SPLEN1000145, SPLEN1000156, SPLEN1000163, SPLEN1000165, SPLEN1000166, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000243, SPLEN2000247, SPLEN2000255, SPLEN2000258, SPLEN2000267, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695,
SPLEN2000698, SPLEN2000839, SPLEN2000874, SPLEN2001135, SPLEN2001141, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002467, SPLEN2002477, SPLEN2002493, SPLEN2002662, SPLEN2002695, SPLEN2002707, SPLEN2002744,
SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004343, SPLEN2004346, SPLEN2004368, SPLEN2004611, SPLEN2004662, SPLEN2004773, SPLEN2004844, SPLEN2004880, SPLEN2004984, SPLEN2005009, SPLEN2005227, SPLEN2005272, SPLEN2005342, SPLEN2005416, SPLEN2005429, SPLEN2005450, SPLEN2005474, SPLEN2005560, SPLEN2005727, SPLEN2005783, SPLEN2005790, SPLEN2005818, SPLEN2005838, SPLEN2005869, SPLEN2005927, SPLEN2005939, SPLEN2006143, SPLEN2006232, SPLEN2006268, SPLEN2006283, SPLEN2006305, SPLEN2006325,
SPLEN2006374, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496, SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009581, SPLEN2009733, SPLEN2009803, SPLEN2009884, SPLEN2009918, SPLEN2009970, SPLEN2010004, SPLEN2010015, SPLEN2010112,
SPLEN2010119, SPLEN2010146, SPLEN2010187, SPLEN2010195, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981, SPLEN2012175, SPLEN2012179, SPLEN2012185, SPLEN2012234, SPLEN2012453, SPLEN2012523, SPLEN2012524, SPLEN2012525, SPLEN2012571, SPLEN2012606,
SPLEN2012611, SPLEN2012619, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014911, SPLEN2014919, SPLEN2014920,
SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840,
SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017643, SPLEN2017687, SPLEN2017740, SPLEN2017781, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311,
SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983,
SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138,
SPLEN2028365, SPLEN2028417, SPLEN2028424, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031547, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081,
SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039379, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645,
SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000257, STOMA2000279, STOMA2000289, STOMA2000383, STOMA2000386, STOMA2000395, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002052,
STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049,
SYNOV1000118, SYNOV1000124, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001239, SYNOV2001251, SYNOV2001262, SYNOV2001300, SYNOV2001356, SYNOV2001374, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005673, SYNOV2005817, SYNOV2005962, SYNOV2006430,
SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989,
SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, SYNOV4009575, T1ESE2000116, T1ESE2000609, T1ESE2000904, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428,
TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002451, TESOP2002489,
TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000023,
TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179,
TESTI2000184, TESTI2000207, TESTI2000208, TESTI2000221, TESTI2000238, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049,
TESTI2001082, TESTI2001120, TESTI2001134, TESTI2001141, TESTI2001153, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001827, TESTI2001829, TESTI2001852, TESTI2001862, TESTI2001869, TESTI2001879, TESTI2001894, TESTI2001898, TESTI2001915, TESTI2001950,
TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789,
TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376,
TESTI2003413, TESTI2003418, TESTI2003419, TESTI2003432, TESTI2003454, TESTI2003475, TESTI2003476, TESTI2003498, TESTI2003512, TESTI2003533, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004031, TESTI2004073, TESTI2004080, TESTI2004085, TESTI2004089, TESTI2004111, TESTI2004119, TESTI2004122, TESTI2004153, TESTI2004163,
TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999,
TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005767, TESTI2005775, TESTI2005784, TESTI2005788, TESTI2005827, TESTI2005835,
TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465, TESTI2006483,
TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007407,
TESTI2007422, TESTI2007452, TESTI2007464, TESTI2007466,. TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007951, TESTI2007972, TESTI2007973, TESTI2007998, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394,
TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009520, TESTI2009544, TESTI2009545, TESTI2009551,
TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605,
TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231,
TESTI2013257, TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979,
TESTI2014988, TESTI2015042, TESTI2015092, TESTI2015105, TESTI2015180, TESTI2015213, TESTI2015246, TESTI2015249, TESTI2015327, TESTI2015331, TESTI2015335, TESTI2015350, TESTI2015375, TESTI2015437, TESTI2015590, TESTI2015610, TESTI2015626, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863,
TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018462, TESTI2018475, TESTI2018482, TESTI2018521, TESTI2018565,
TESTI2018581, TESTI2018611, TESTI2018687, TESTI2018697, TESTI2018838, TESTI2018867, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379,
TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021122, TESTI2021124, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203,
TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473,
TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104,
TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562,
TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342,
TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401,
TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034940, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951,
TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276,
TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400,TESTI2042450, TESTI2042473, TESTI2042507, TESTI2042508, TESTI2042750,
TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439,
TESTI2046456, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279,
TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000014, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394,
TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889,
TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326,
TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840,
TESTI4008935, TESTI4008993, TESTI4009022, TESTI4009028, TESTI4009034, TESTI4009123, TESTI4009160, TESTI4009215, TESTI4009283, TESTI4009286, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010851, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745,
TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014801, TESTI4014818,
TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152,
TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942,
TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612,
TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633,
TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363,
TESTI4040535, TESTI4040559, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682,
TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000017, THYMU1000025, THYMU1000029, THYMU1000033, THYMU1000041, THYMU1000060, THYMU1000074, THYMU1000083, THYMU1000098, THYMU1000103, THYMU1000105, THYMU1000109, THYMU1000136, THYMU1000142,
THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000374, THYMU1000382, THYMU1000393, THYMU1000394, THYMU1000399, THYMU1000426, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231,
THYMU2000236, THYMU2000276, THYMU2000280, THYMU2000300, THYMU2000317, THYMU2000368, THYMU2000369, THYMU2000418, THYMU2000436, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879, THYMU2000932, THYMU2000950, THYMU2000971, THYMU2000982, THYMU2000990, THYMU2001007, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001443, THYMU2001515, THYMU2001521, THYMU2001556, THYMU2001622, THYMU2001727,
THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002841, THYMU2002852, THYMU2002910, THYMU2002950, THYMU2003012, THYMU2003046, THYMU2003069, THYMU2003107, THYMU2003124, THYMU2003133, THYMU2003166, THYMU2003232, THYMU2003242, THYMU2003249, THYMU2003270, THYMU2003282,
THYMU2003287, THYMU2003336, THYMU2003366, THYMU2003397, THYMU2003419, THYMU2003440, THYMU2003446, THYMU2003470, THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2003981, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003,
THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006813, THYMU2006913, THYMU2006946,
THYMU2006965, THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008191, THYMU2008207, THYMU2008226, THYMU2008282, THYMU2008321, THYMU2008339, THYMU2008350, THYMU2008351,
THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637, THYMU2010671, THYMU2010699, THYMU2010705, THYMU2010779,
THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807,
THYMU2012826, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015775, THYMU2015825, THYMU2015918, THYMU2015943,
THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054,
THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572,
THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249,
THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554,
THYMU2035710, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824,
THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632,
THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717,
THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900,
THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612,
THYMU3031868, THYMU3031878, THYMU3032032, THYMU3032798, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168,
THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062,
TKIDN1000066, TKIDN1000164, TKIDN1000171, TKIDN1000192, TKIDN2000127, TKIDN2000240, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200,
TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381,
TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018,
TRACH1000030, TRACH1000038, TRACH1000057, TRACH1000063, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000045, TRACH2000047, TRACH2000079, TRACH2000081, TRACH2000083, TRACH2000148, TRACH2000185, TRACH2000196, TRACH2000235, TRACH2000237, TRACH2000243, TRACH2000248, TRACH2000250, TRACH2000259, TRACH2000272, TRACH2000282, TRACH2000287, TRACH2000289, TRACH2000306, TRACH2000312, TRACH2000321, TRACH2000336, TRACH2000359, TRACH2000393, TRACH2000411, TRACH2000420, TRACH2000461, TRACH2000496, TRACH2000497, TRACH2000502, TRACH2000522,
TRACH2000660, TRACH2000665, TRACH2000675, TRACH2000677, TRACH2000702, TRACH2000703, TRACH2000767, TRACH2000775, TRACH2000780, TRACH2000807, TRACH2000862, TRACH2000873, TRACH2000894, TRACH2000898, TRACH2000920, TRACH2000923, TRACH2000926, TRACH2000944, TRACH2000959, TRACH2000972, TRACH2000981, TRACH2000986, TRACH2001037, TRACH2001060, TRACH2001085, TRACH2001101, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001463, TRACH2001474, TRACH2001523, TRACH2001525,
TRACH2001532, TRACH2001549, TRACH2001592, TRACH2001596, TRACH2001612, TRACH2001621, TRACH2001646, TRACH2001651, TRACH2001684, TRACH2001933, TRACH2001968, TRACH2001996, TRACH2002043, TRACH2002054, TRACH2002100, TRACH2002138, TRACH2002333, TRACH2002537, TRACH2002664, TRACH2002784, TRACH2002803, TRACH2002840, TRACH2002847, TRACH2002853, TRACH2002862, TRACH2002954, TRACH2002960, TRACH2002988, TRACH2003070, TRACH2003272, TRACH2003323, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458,
TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005811, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007399, TRACH2007483, TRACH2007542, TRACH2007577, TRACH2007674, TRACH2007676, TRACH2007688, TRACH2007733, TRACH2007754, TRACH2007816, TRACH2007894, TRACH2007969, TRACH2007988, TRACH2008081,
TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965,
TRACH2010974, TRACH2011057, TRACH2011093, TRACH2011113, TRACH2011290, TRACH2011293, TRACH2011302, TRACH2011500, TRACH2011574, TRACH2011894, TRACH2011956, TRACH2012000, TRACH2012138, TRACH2012242, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014077, TRACH2014082, TRACH2014124, TRACH2014268, TRACH2014284, TRACH2014337, TRACH2014356, TRACH2014371, TRACH2014442,
TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015823, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034,
TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306,
TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926,
TRACH3001119, TRACH3001443, TRACH3001550, TRACH3001759, TRACH3002064, TRACH3002107, TRACH3002168, TRACH3002188, TRACH3002192, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840,
TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007541, TRACH3007595, TRACH3007625, TRACH3007627, TRACH3007689, TRACH3007766, TRACH3007866, TRACH3007995, TRACH3008042, TRACH3008061,
TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942,
TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018,
TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423,
TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400,
TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063,
TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143,
UMVEN1000156, UMVEN2000046, UMVEN2000069, UMVEN2000121, UMVEN2000152, UMVEN2000354, UMVEN2000453, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000424,
UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002332, UTERU2002410, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057,
UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004037, UTERU2004039, UTERU2004061, UTERU2004073, UTERU2004163, UTERU2004197, UTERU2004299, UTERU2004461, UTERU2004520, UTERU2004564, UTERU2004664, UTERU2004688, UTERU2004698, UTERU2004807, UTERU2004861, UTERU2004929, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412,
UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007267, UTERU2007444, UTERU2007499, UTERU2007639, UTERU2007724, UTERU2007924, UTERU2007942, UTERU2008018, UTERU2008019, UTERU2008027, UTERU2008040, UTERU2008077, UTERU2008130, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009335, UTERU2009414,
UTERU2009435, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012286, UTERU2012333, UTERU2012407, UTERU2012526, UTERU2012581, UTERU2012610, UTERU2012615, UTERU2012688, UTERU2012703,
UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015062, UTERU2015087, UTERU2015190, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757,
UTERU2016761, UTERU2016799, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018609, UTERU2018674, UTERU2018712, UTERU2018784, UTERU2018789, UTERU2018811, UTERU2018867, UTERU2018881, UTERU2018884, UTERU2018955, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964,
UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023175, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941,
UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530,
UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135,
UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722,
UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176,
UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the NCRRM, NCRRP, NESOP, NETRP, NHNPC, and NOVAR libraries were analyzed for expression frequency. The analysis results are described below. Each is a relative value, where the expression level over all tissues is taken as 100. The larger this value, the greater the level of expression.

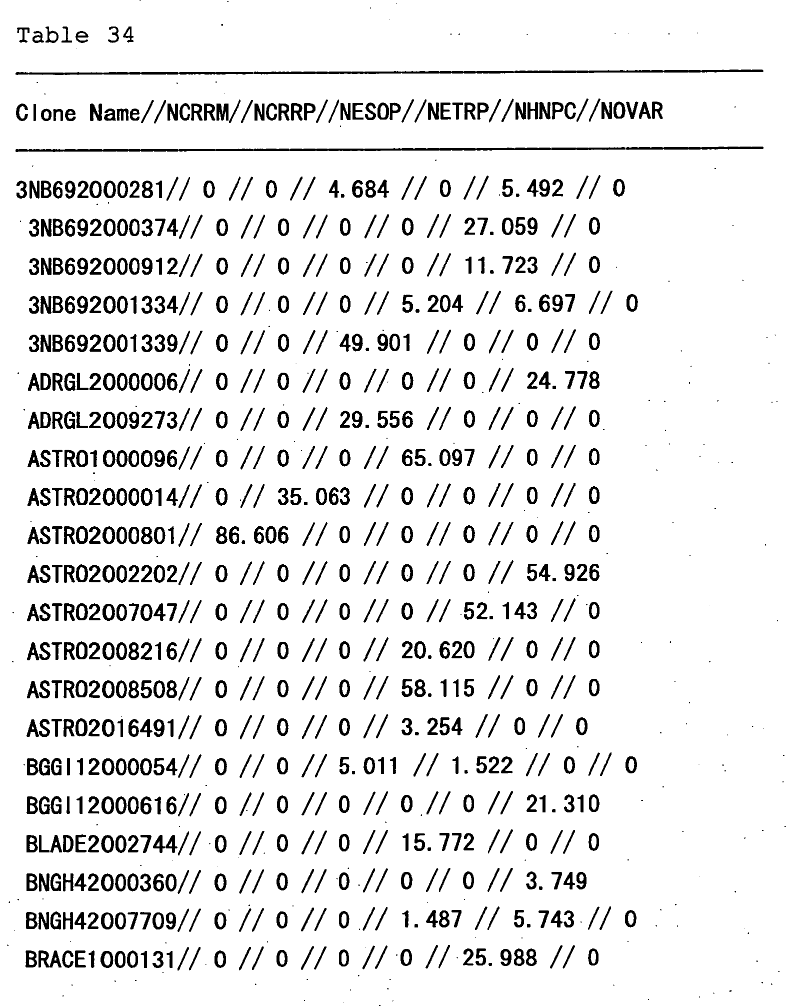

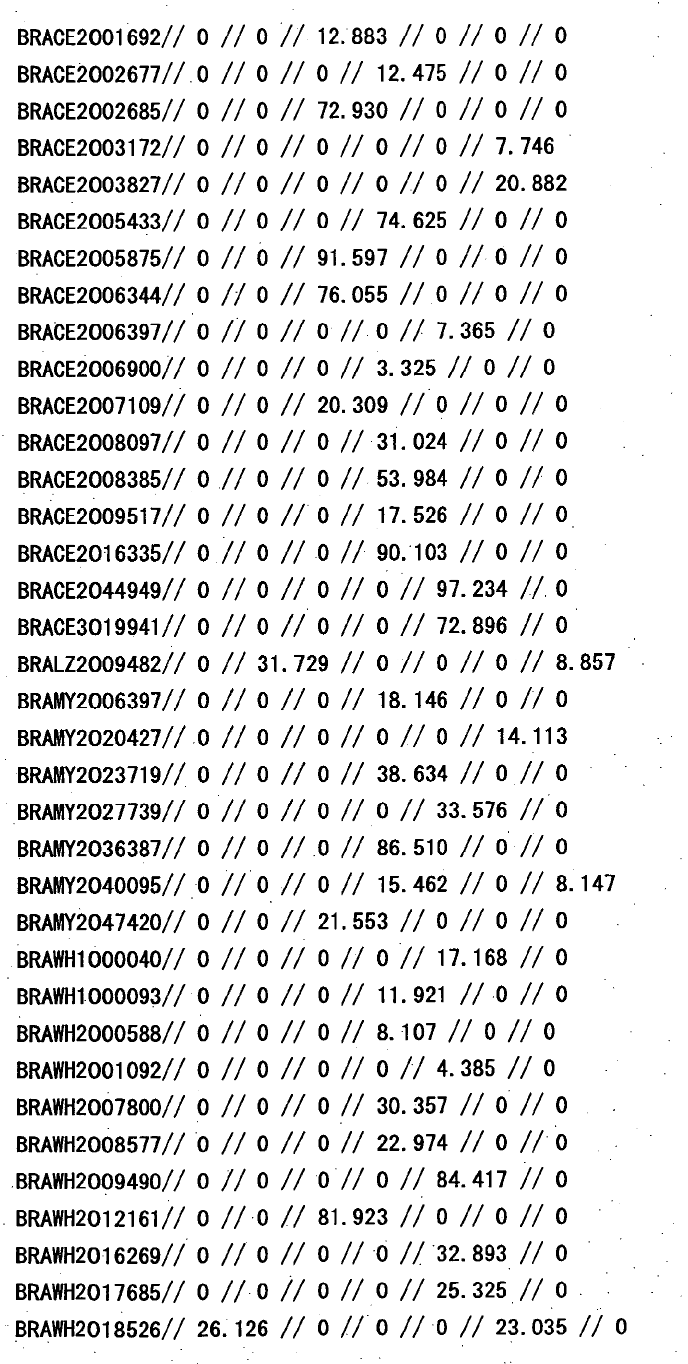

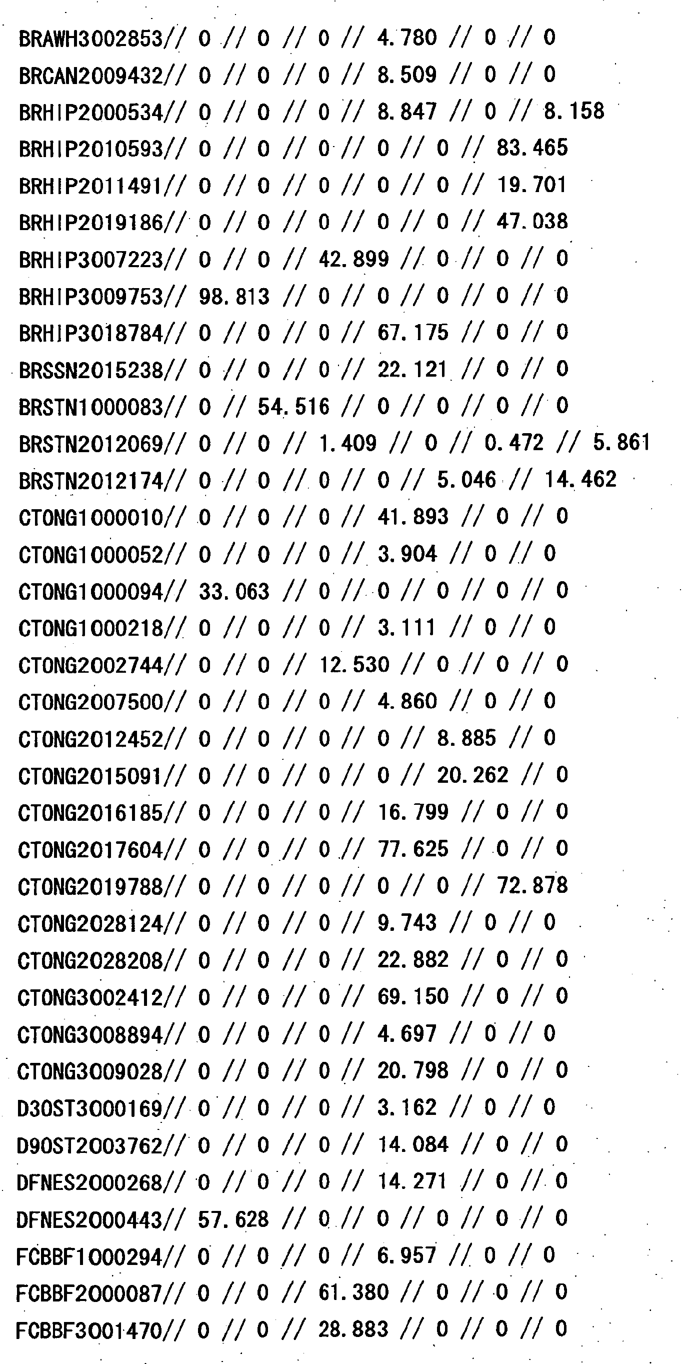

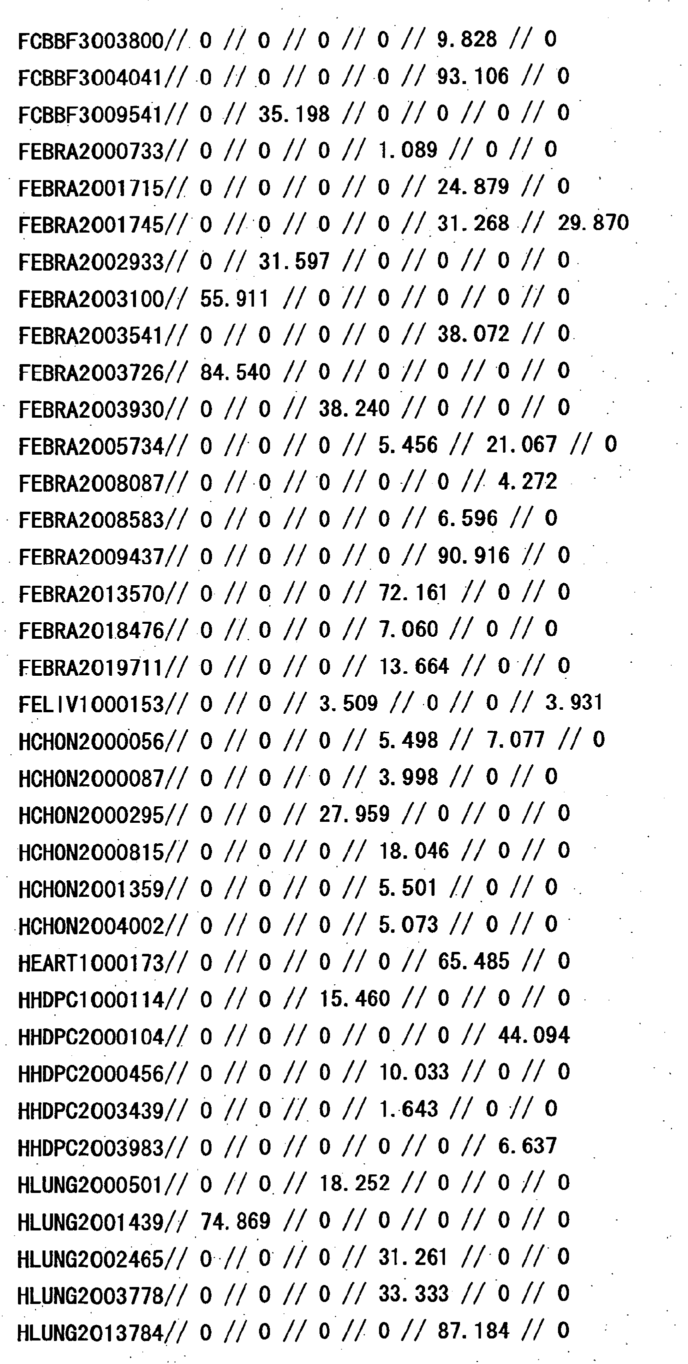

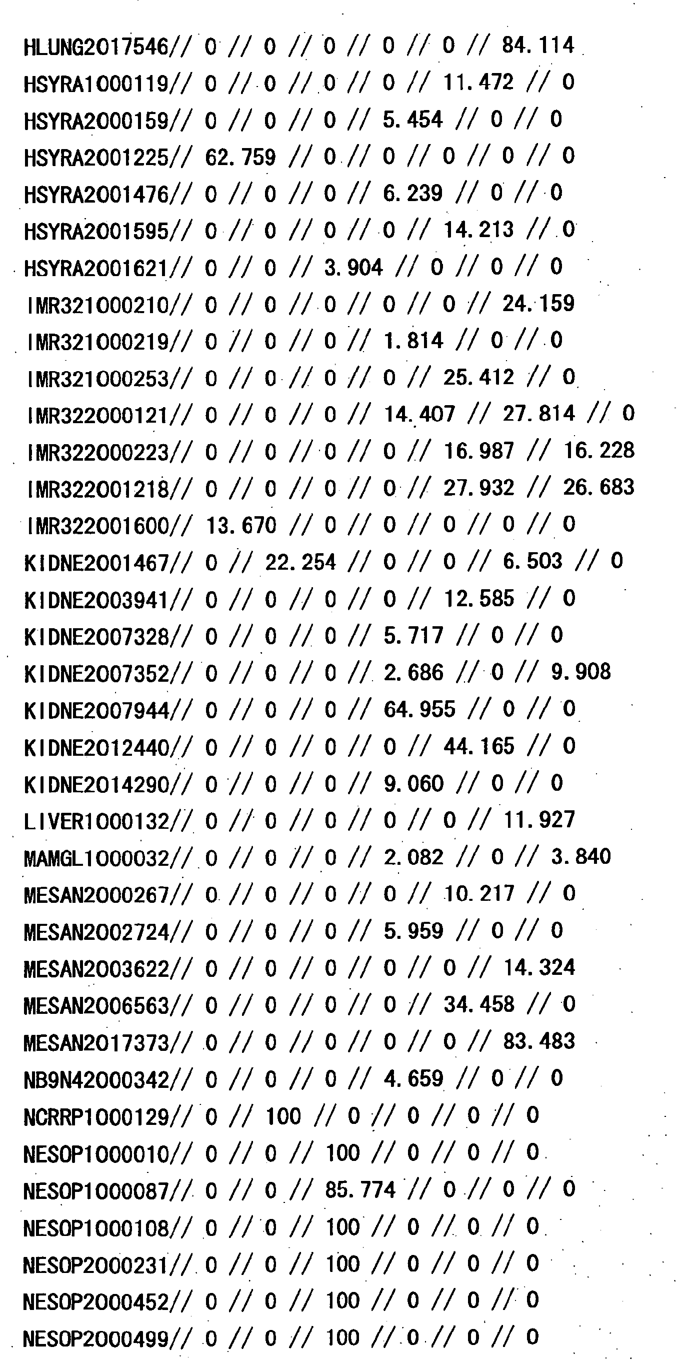

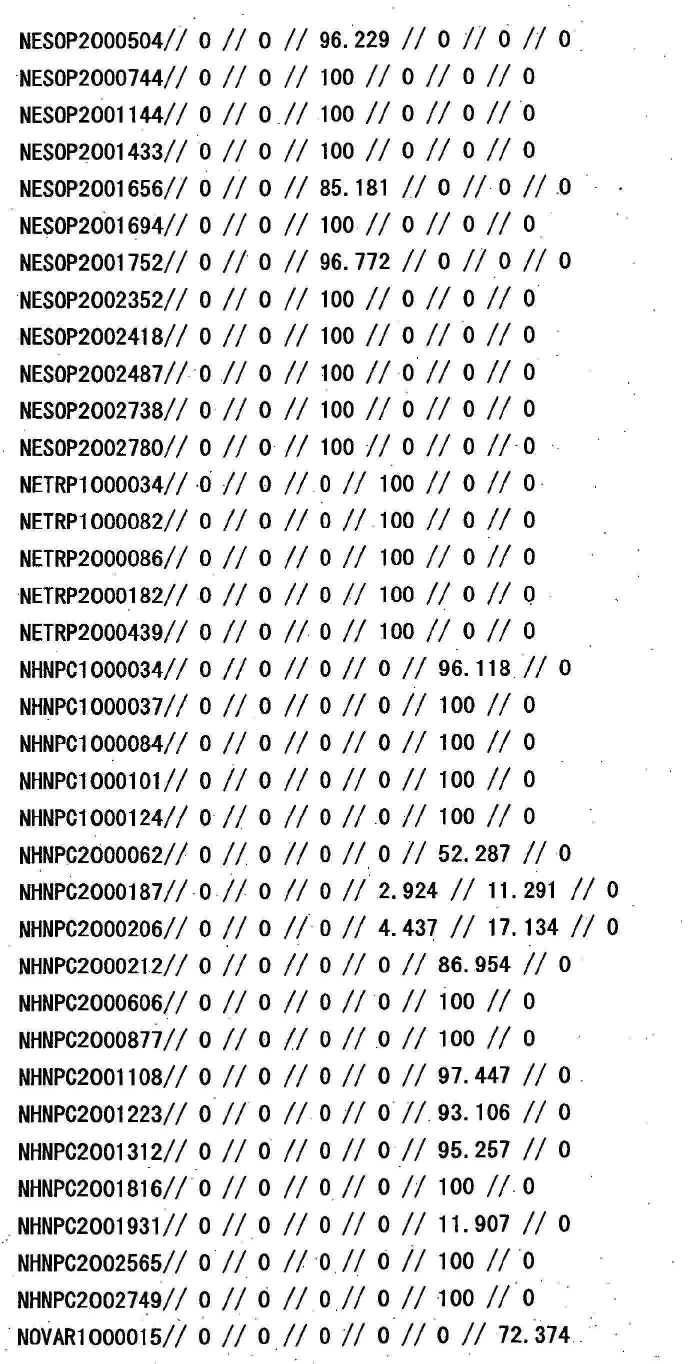

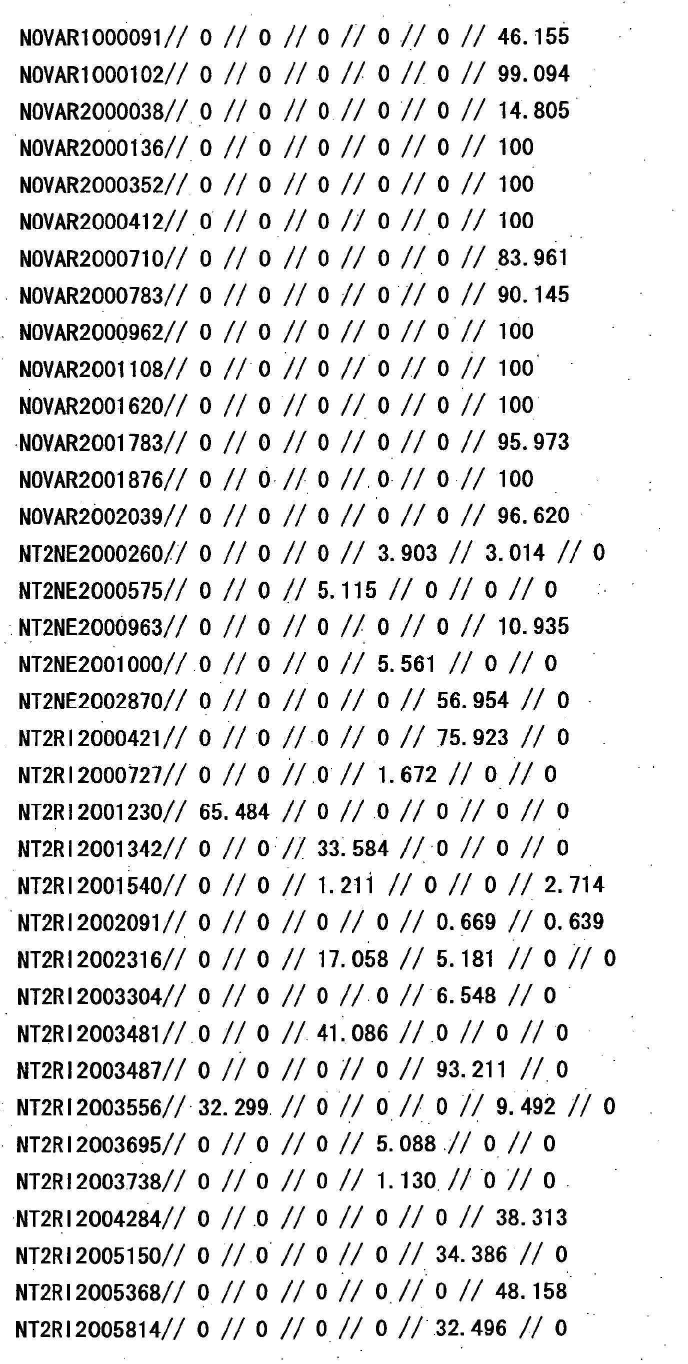

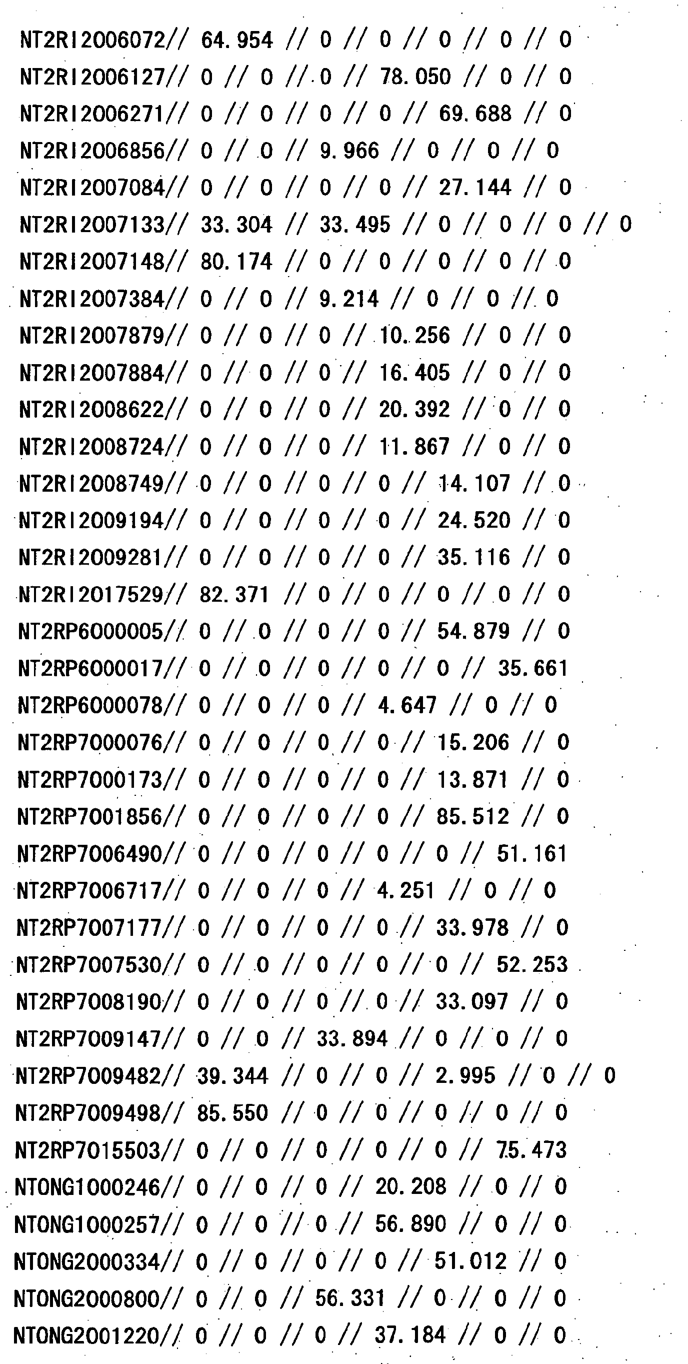

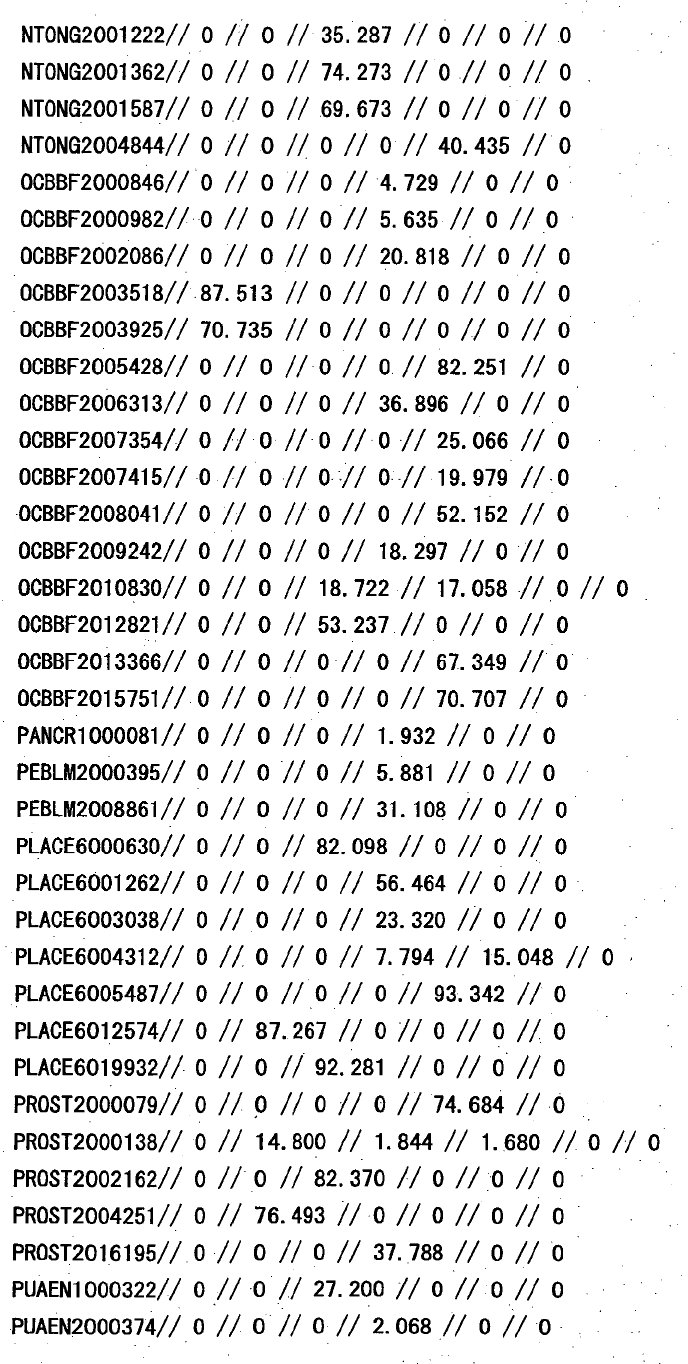

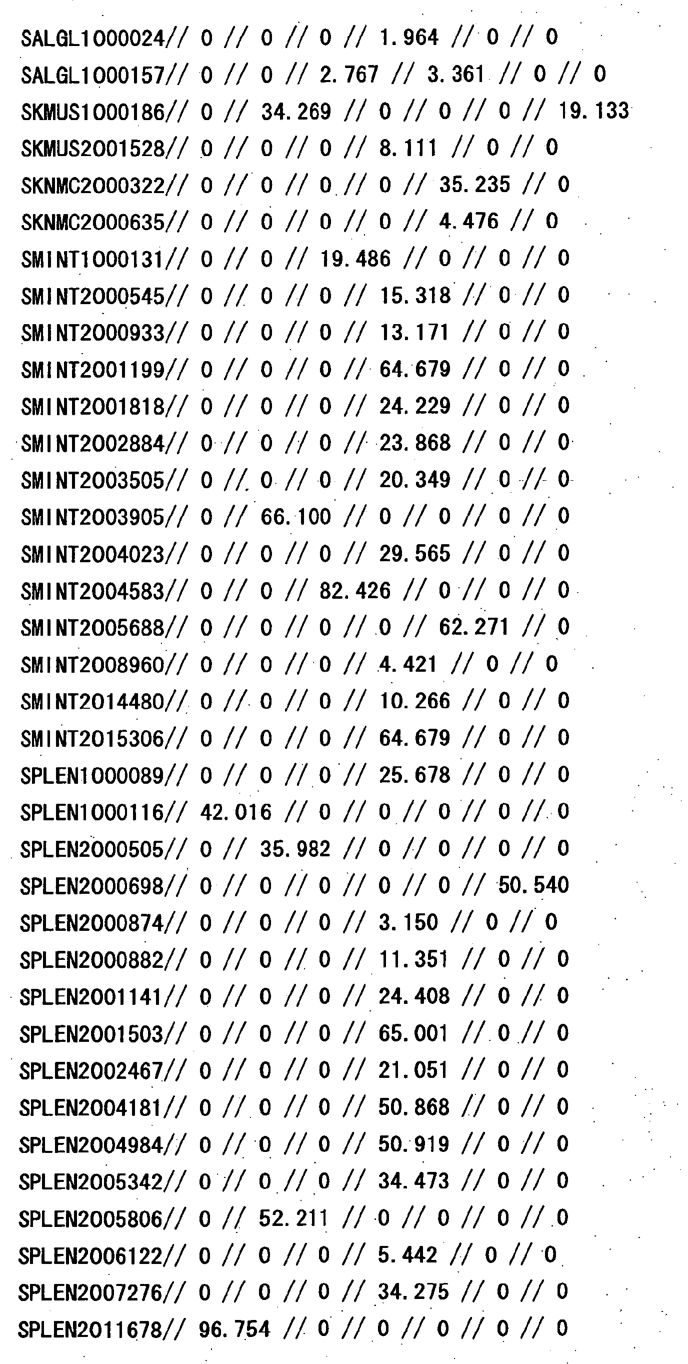

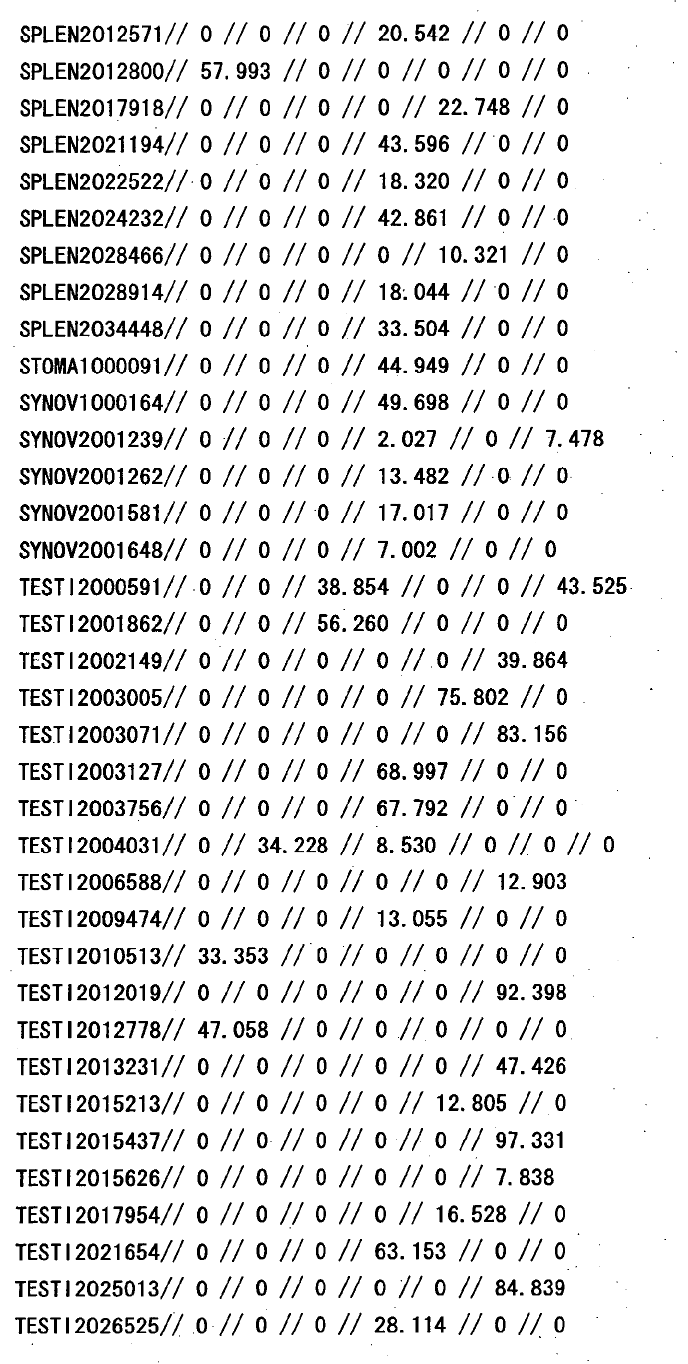

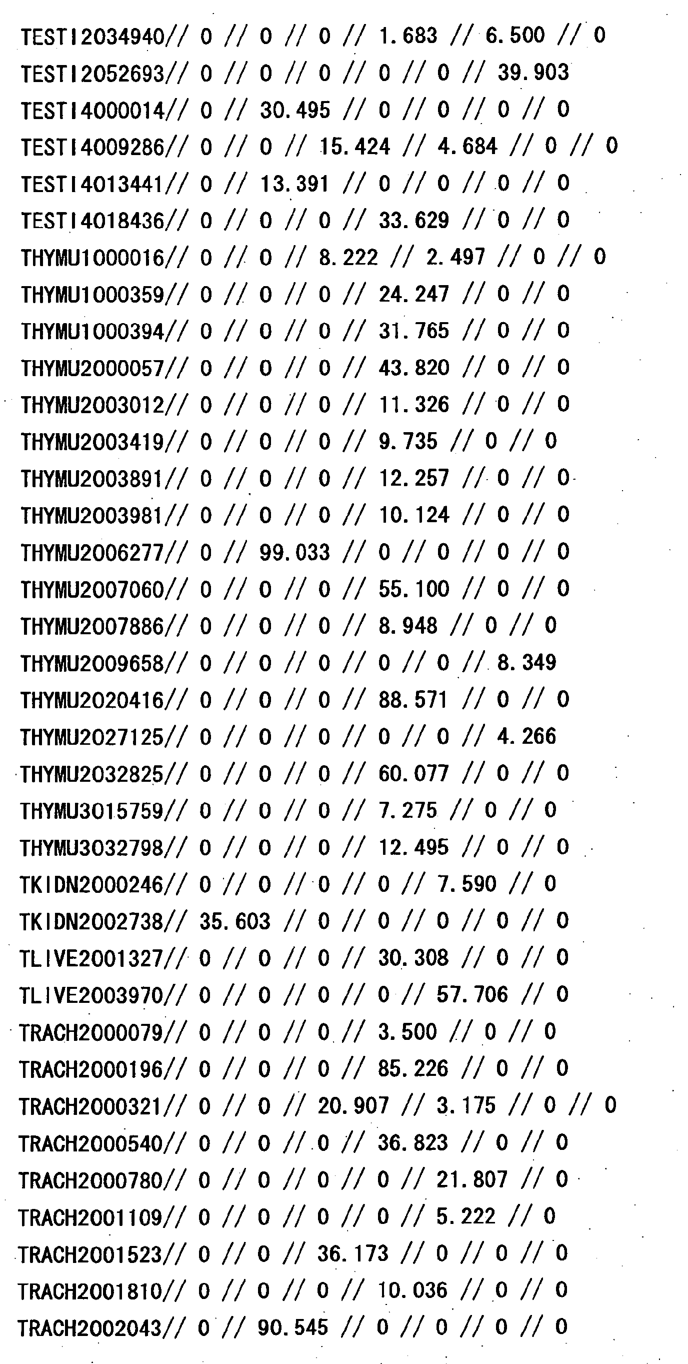

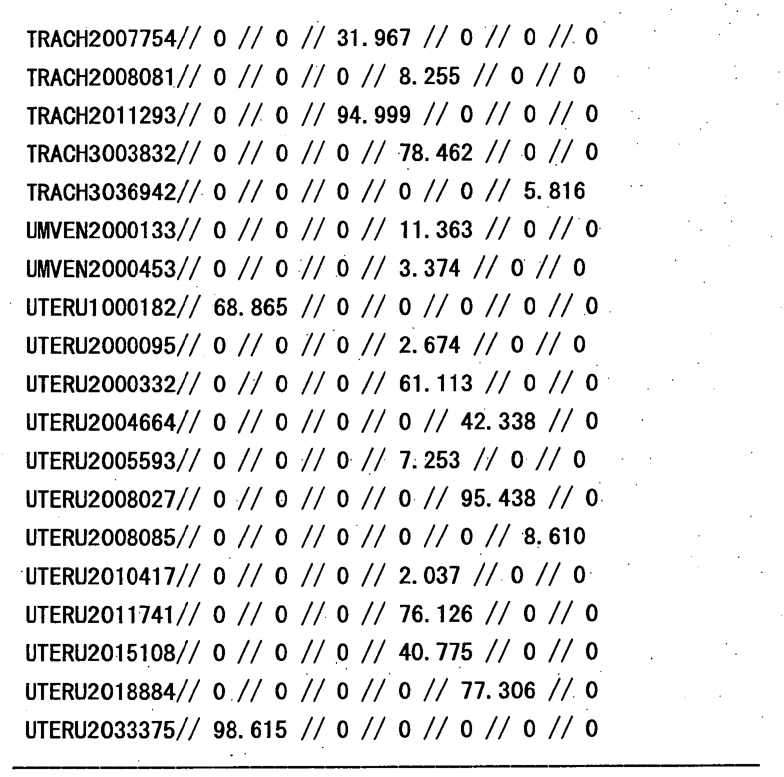

Genes whose expression levels were negligibly low in each of the above-described libraries are shown with their clone names below.
3NB691000018, 3NB691000085, 3NB691000113, 3NB691000116, 3NB691000129, 3NB691000173, 3NB691000191, 3NB692000012, 3NB692000029, 3NB692000102, 3NB692000154, 3NB692000276, 3NB692000305, 3NB692000330, 3NB692000429, 3NB692000484, 3NB692000529, 3NB692000545, 3NB692000973, 3NB692001002, 3NB692001022, 3NB692001034, 3NB692001040, 3NB692001123, 3NB692001267, 3NB692001288, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001853, 3NB692001861, 3NB692002051, 3NB692002365, 3NB692002685,
3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002477, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767,
ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000165, ASTRO2000011, ASTRO2000046, ASTRO2000095, ASTRO2000141, ASTRO2000191, ASTRO2000278, ASTRO2000372, ASTRO2000381, ASTRO2000403, ASTRO2000417, ASTRO2000480,
ASTRO2000482, ASTRO2000533, ASTRO2000653, ASTRO2000662, ASTRO2000725, ASTRO2000914, ASTRO2001001, ASTRO2001008, ASTRO2001029, ASTRO2001076, ASTRO2001088, ASTRO2001107, ASTRO2001122, ASTRO2001227, ASTRO2001249, ASTRO2001480, ASTRO2001806, ASTRO2001823, ASTRO2002024, ASTRO2002035, ASTRO2002064, ASTRO2002459, ASTRO2002571, ASTRO2002632, ASTRO2002659, ASTRO2002693, ASTRO2002720, ASTRO2002733, ASTRO2002743, ASTRO2002842, ASTRO2003024, ASTRO2003212, ASTRO2003316, ASTRO2003461, ASTRO2003574, ASTRO2003581, ASTRO2003632, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877,
ASTRO2004974, ASTRO2005008, ASTRO2005081, ASTRO2005096, ASTRO2005242, ASTRO2005273, ASTRO2005343, ASTRO2005360, ASTRO2005413, ASTRO2005423, ASTRO2005553, ASTRO2005557, ASTRO2005575, ASTRO2005593, ASTRO2005687, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008239, ASTRO2008409, ASTRO2008425, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072,
ASTRO2010582, ASTRO2010615, ASTRO2010799, ASTRO2010819, ASTRO2010962, ASTRO2010996, ASTRO2011149, ASTRO2011422, ASTRO2011426, ASTRO2011437, ASTRO2011461, ASTRO2011834, ASTRO2011893, ASTRO2012552, ASTRO2013124, ASTRO2013585, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015328, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172,
ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000533, BGGI12000544, BGGI12000684, BGGI12000693, BGGI12000839, BGGI12001002, BGGI12001075, BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002782,
BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118,
BNGH41000137, BNGH41000153, BNGH41000169, BNGH41000177, BNGH41000190, BNGH41000198, BNGH41000216, BNGH41000235, BNGH42000130, BNGH42000132, BNGH42000405, BNGH42000532, BNGH42000675, BNGH42000739, BNGH42000791, BNGH42000815, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006135, BNGH42006226, BNGH42006234, BNGH42007037, BNGH42007366, BNGH42007460, BNGH42007594, BNGH42007788, BNGH42007798, BNGH42008199, BNGH42008510, BNGH42008603, BNGH42008649,
BNGH42008743, BNGH42008832, BNGH42008850, BNGH42008943, BNGH42009021, BNGH42009025, BRACE1000020, BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000115, BRACE1000124, BRACE1000132, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000307, BRACE2000331,
BRACE2000332, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001143, BRACE2001151, BRACE2001154, BRACE2001188, BRACE2001191, BRACE2001197, BRACE2001236, BRACE2001239,
BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972,
BRACE2001973, BRACE2002026, BRACE2002034, BRACE2002050, BRACE2002081, BRACE2002091, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002441, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002682, BRACE2002695, BRACE2002707, BRACE2002736, BRACE2002752, BRACE2002755, BRACE2002762, BRACE2002772,
BRACE2002792, BRACE2002796, BRACE2002803, BRACE2002807, BRACE2002812, BRACE2002834, BRACE2002849, BRACE2002857, BRACE2002860, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003254, BRACE2003269, BRACE2003285, BRACE2003304, BRACE2003319, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003825, BRACE2003845,
BRACE2003846, BRACE2003847, BRACE2003848, BRACE2003873, BRACE2003885, BRACE2003887, BRACE2003892, BRACE2003904, BRACE2003905, BRACE2003928, BRACE2003944, BRACE2003954, BRACE2003982, BRACE2003987, BRACE2005034, BRACE2005047, BRACE2005063, BRACE2005083, BRACE2005087, BRACE2005090, BRACE2005099, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005434, BRACE2005448, BRACE2005450, BRACE2005453, BRACE2005457,
BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE 2005858, BRACE2005869, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281,
BRACE2006298, BRACE2006319, BRACE2006354, BRACE2006363, BRACE2006378, BRACE2006380, BRACE2006393, BRACE2006413, BRACE2006417, BRACE2006453, BRACE2006459, BRACE2006488, BRACE2006514, BRACE2006534, BRACE2006537, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006871, BRACE2006909, BRACE2006911, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007089, BRACE2007138, BRACE2007174, BRACE2007191, BRACE2007197, BRACE2007201, BRACE2007203,
BRACE2007232, BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007518, BRACE2007522, BRACE2007527, BRACE2007538, BRACE2007563, BRACE2007567, BRACE2007577, BRACE2007621, BRACE2007640, BRACE2007641, BRACE2007646, BRACE2007663, BRACE2007671, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902,
BRACE2007920, BRACE2007937, BRACE2007944, BRACE2007952, BRACE2008008, BRACE2008015, BRACE2008021, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008653, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008968, BRACE2008999, BRACE2009014, BRACE2009016, BRACE2009031, BRACE2009037, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009533, BRACE2009558; BRACE2009566, BRACE2009591, BRACE2009620, BRACE2009654, BRACE2009721, BRACE2009732,
BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062,
BRACE2012185, BRACE2012222, BRACE2012232, BRACE2012291, BRACE2012317, BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741,
BRACE2015756, BRACE2015969, BRACE2015995, BRACE2016058, BRACE2016194, BRACE2016315, BRACE2016325, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736; BRACE2018759, BRACE2018762, BRACE2018782, BRACE2018847, BRACE2019004, BRACE2019044, BRACE2019147, BRACE2019244, BRACE2019258, BRACE2019327,
BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389,
BRACE2022394, BRACE2022435, BRACE2022448, BRACE2022450, BRACE2022549, BRACE2022633, BRACE2022638, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836,
BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385,
BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286,
BRACE2039297, BRACE2039327, BRACE2039362, BRACE2039475, BRACE2039600, BRACE2039607, BRACE2039624, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2045300, BRACE2045428, BRACE2045445,
BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413,
BRACE3003595, BRACE3003698, BRACE3003866, BRACE3003920, BRACE3004046, BRACE3004058, BRACE3004113, BRACE3004150, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005938, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480,
BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010428, BRACE3010435, BRACE3010438, BRACE3010702, BRACE3010862, BRACE3011271, BRACE3011421, BRACE3011447, BRACE3011505,
BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611,
BRACE3019817, BRACE3019828, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256,
BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012,
BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018,
BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486,
BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000585, BRAMY2000653, BRAMY2000814,
BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031,
BRAMY2004058, BRAMY2004183, BRAMY2004335, BRAMY2004351, BRAMY2004352, BRAMY2004363, BRAMY2004387, BRAMY2004492, BRAMY2004521, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458,
BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064,
BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, _{.} BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019,
BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273,
BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019554, BRAMY2019600, BRAMY2019643, BRAMY2019710, BRAMY2019963, BRAMY2019985, BRAMY2019989, BRAMY2020050, BRAMY2020058, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982,
BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711,
BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467,. BRAMY2027587, BRAMY2027714, BRAMY2027717, BRAMY2027752, BRAMY2027782, BRAMY2028072, BRAMY2028183, BRAMY2028188, BRAMY2028269, BRAMY2028282,
BRAMY2028472, BRAMY2028491, BRAMY2028516, BRAMY2028565, BRAMY2028593, BRAMY2028682, BRAMY2028740, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032087, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506,
BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300,
BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489,
BRAMY2046537, BRAMY2046667, BRAMY2046742, BRAMY2046871, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004377, BRAMY3004672, BRAMY3004800, BRAMY3004900, BRAMY3004919,
BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010008, BRAMY3010264, BRAMY3010321, BRAMY3010411, BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652,
BRAMY4001863, BRAMY4001913, BRAMY4002575, BRAMY4002628, BRASW1000053, BRASW1000125, BRAWH1000001, BRAWH1000002, BRAWH1000007, BRAWH1000037, BRAWH1000045, BRAWH1000083, BRAWH1000094, BRAWH1000116, BRANH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000460, BRAWH2000471, BRAWH2000476, BRAWH2000482, BRAWH2000488, BRAWH2000494,
BRAWH2000500, BRAWH2000503, BRAWH2000522, BRAWH2000554, BRAWH2000595, BRAWH2000633, BRAWH2000636, BRAWH2000643, BRAWH2000651, BRAWH2000682, BRAWH2000686, BRAWH2000697, BRAWH2000752, BRAWH2000839, BRAWH2000859, BRAWH2000866, BRAWH2000892, BRAWH2000901, BRAWH2000924, BRAWH2000926, BRAWH2000944, BRAWH2000984, BRAWH2001019, BRAWH2001037, BRAWH2001052, BRAWH2001103, BRAWH2001129, BRAWH2001137, BRAWH2001141, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406, BRAWH2001412, BRAWH2001418, BRAWH2001424, BRAWH2001436, BRAWH2001438, BRAWH2001439, BRAWH2001459, BRAWH2001461,
BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025, BRAWH2003135, BRAWH2003240, BRAWH2003307, BRAWH2003355, BRAWH2003366,
BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005225, BRAWH2005315, BRAWH2005517, BRAWH2005524, BRAWH2005533, BRAWH2005578, BRAWH2005661, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406,
BRAWH2007550, BRAWH2007570, BRAWH2007591, BRAWH2007605, BRAWH2007658, BRAWH2007664, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380, BRAWH2008540, BRAWH2008706, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009360, BRAWH2009393, BRAWH2009485, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536,
BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014188,
BRAWH2014234, BRAWH2014379, BRAWH2014414, BRAWH2014473, BRAWH2014511, BRAWH2014645, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534,
BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244,
BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461,
BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884,
BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556,
BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168,
BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003944, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117,
BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402,
BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368,
BRCAN2025712, BRCAN2025982, BRCAN2026117, BRCAN2026197, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028036, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756,
BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229,
BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506,
BRHIP2000532, BRHIP2000553, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800,
BRHIP2006819, BRHIP2006921, BRHIP2007305, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010610, BRHIP2010723, BRHIP2010744, BRHIP2011080, BRHIP2011120, BRHIP2011199, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750,
BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047,
BRHIP2019149, BRHIP2019177, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022228, BRHIP2022298, BRHIP2022326, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869,
BRHIP2023888, BRHIP2024046, BRHIP2024165, BRHIP2024201, BRHIP2024347, BRHIP2024398, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000111, BRHIP3000131,
BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307,
BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3010135, BRHIP3010289, BRHIP3010529, BRHIP3011082, BRHIP3011241, BRHIP3011269,
BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3019643, BRHIP3019824, BRHIP3019880, BRHIP3019956, BRHIP3020046, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533,
BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869,
BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011262,
BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581,
BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923,
BRSTN2011211, BRSTN2011597, BRSTN2011688, BRSTN2011799, BRSTN2011961, BRSTN2012089, BRSTN2012284, BRSTN2012320, BRSTN2012380, BRSTN2012451, BRSTN2013171, BRSTN2013354, BRSTN2013502, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016470, BRSTN2016678, BRSTN2016892, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855,
BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194,
BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466,
BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165,
BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917,
BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3003736,
BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265,
BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556,
BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351,
COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000020, CTONG1000022, CTONG1000040, CTONG1000056, CTONG1000062, CTONG1000086, CTONG1000087, CTONG1000088, CTONG1000093, CTONG1000097, CTONG1000113, CTONG1000137, CTONG1000165, CTONG1000175, CTONG1000180, CTONG1000181, CTONG1000220, CTONG1000241, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042,
CTONG2000063, CTONG2000201, CTONG2000218, CTONG2000279, CTONG2000303, CTONG2000330, CTONG2000395, CTONG2000411, CTONG2000452, CTONG2000465, CTONG2000469, CTONG2000480, CTONG2000508, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001932, CTONG2001955, CTONG2002066, CTONG2002073, CTONG2002095, CTONG2002143, CTONG2002270, CTONG2002395, CTONG2002417,
CTONG2002418, CTONG2002453, CTONG2002520, CTONG2002558, CTONG2002590, CTONG2002709, CTONG2002721, CTONG2002766, CTONG2002803, CTONG2002816, CTONG2002820, CTONG2002832, CTONG2002841, CTONG2002903, CTONG2002965, CTONG2002999, CTONG2003028, CTONG2003094, CTONG2003100, CTONG2003115, CTONG2003189, CTONG2003245, CTONG2003293, CTONG2003298, CTONG2003348, CTONG2003350, CTONG2003361, CTONG2003372, CTONG2003375, CTONG2003427, CTONG2003517, CTONG2003524, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004487,
CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005290, CTONG2005374, CTONG2005399, CTONG2005468, CTONG2005553, CTONG2005567, CTONG2005585, CTONG2005615, CTONG2005635, CTONG2005775, CTONG2005795, CTONG2005890, CTONG2005904, CTONG2005913, CTONG2006004, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006562, CTONG2006568, CTONG2006611, CTONG2006620, CTONG2006666, CTONG2006691, CTONG2006709, CTONG2006798, CTONG2006836, CTONG2006932, CTONG2006942, CTONG2006964,
CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321, CTONG2008343, CTONG2008398, CTONG2008402, CTONG2008405, CTONG2008466, CTONG2008506, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033,
CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010348, CTONG2010408, CTONG2010508, CTONG2010566, CTONG2010623, CTONG2010649, CTONG2010652, CTONG2010803, CTONG2010821, CTONG2011188,
CTONG2011429, CTONG2011474, CTONG2011676, CTONG2011716, CTONG2011765, CTONG2011779, CTONG2011801, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012158, CTONG2012230, CTONG2012401, CTONG2012422, CTONG2012425, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012847, CTONG2012879, CTONG2012901, CTONG2012996, CTONG2013128, CTONG2013149, CTONG2013156, CTONG2013178, CTONG2013222, CTONG2013284, CTONG2013339, CTONG2013348, CTONG2013352,
CTONG2013381, CTONG2013393, CTONG2013511, CTONG2013630, CTONG2013803, CTONG2013907, CTONG2013934, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014705, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016056, CTONG2016217,
CTONG2016355, CTONG2016408, CTONG2016499, CTONG2016505, CTONG2016559, CTONG2016575, CTONG2016658, CTONG2016775, CTONG2016797, CTONG2016824, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017500, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018343, CTONG2018379, CTONG2018383, CTONG2018413, CTONG2018483, CTONG2018629, CTONG2018632, CTONG2018637, CTONG2018652, CTONG2018653, CTONG2018655,
CTONG2018735, CTONG2018739, CTONG2018808, CTONG2018843, CTONG2018898, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019822, CTONG2019833, CTONG2020031, CTONG2020108, CTONG2020127, CTONG2020171, CTONG2020374, CTONG2020378, CTONG2020411, CTONG2020445, CTONG2020484, CTONG2020522, CTONG2020560, CTONG2020582, CTONG2020638, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024031, CTONG2024206, CTONG2024614, CTONG2024749, CTONG2025496, CTONG2025513,
CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553, CTONG3003598,
CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009239, CTONG3009287, CTONG3009328, CTONG3009385, D3OST1000066, D3OST1000109, D3OST1000238, D3OST1000267, D3OST1000270,
D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000195, D3OST3000258, D3OST3000291, D3OST3000388, D3OST3000493, D6OST2000127, D6OST2000169, D6OST2000245, D60ST2000358, D6OST2000464, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003791, D9OST2003989, D9OST2004018,
D9OST2004073, D9OST2004417, DFNES1000003, DFNES1000083, DFNES1000107, DFNES1000150, DFNES1000185, DFNES2000011, DFNES2000143, DFNES2000146, DFNES2000153, DFNES2000212, DFNES2000292, DFNES2000335, DFNES2000426, DFNES2000432, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004371, DFNES2004383, DFNES2004608, DFNES2004684, DFNES2005266, DFNES2005527, DFNES2005540, DFNES2005690,
DFNES2005766, DFNES2005779, DFNES2005875, DFNES2006346, DFNES2006944, DFNES2007113, DFNES2007299, DFNES2007332, DFNES2007634, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000024, FCBBF1000025, FCBBF1000027, FCBBF1000038, FCBBF1000053, FCBBF1000061, FCBBF1000063, FCBBF1000069, FCBBF1000077, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182, FCBBF1000197, FCBBF1000198,
FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000367, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000466, FCBBF1000476, FCBBF1000506, FCBBF1000509, FCBBF1000546, FCBBF1000550, FCBBF1000556, FCBBF1000563, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF1000760, FCBBF2000038, FCBBF2000094,
FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000591, FCBBF2000677, FCBBF2000678, FCBBF2000685, FCBBF2000733, FCBBF2000782, FCBBF2000808, FCBBF2000815, FCBBF2000825, FCBBF2000885, FCBBF2000940, FCBBF2000951, FCBBF2001001, FCBBF2001088, FCBBF2001105, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817,
FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006380,
FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001632, FCBBF3001657, FCBBF3001818, FCBBF3001855, FCBBF3001890, FCBBF3001912, FCBBF3001914, FCBBF3001918,
FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004261, FCBBF3004323, FCBBF3004390, FCBBF3004409, FCBBF3004473, FCBBF3004502, FCBBF3004715, FCBBF3004842, FCBBF3004847, FCBBF3004955, FCBBF3005160, FCBBF3005218, FCBBF3005320, FCBBF3005330, FCBBF3005444, FCBBF3005497, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152,
FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211,
FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809,
FCBBF3015870, FCBBF3016018, FCBBF3016134, FCBBF3016178, FCBBF3016451, FCBBF3016618, FCBBF3016622, FCBBF3016644, FCBBF3016667, FCBBF3016928, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019839, FCBBF3019867, FCBBF3019961, FCBBF3020030, FCBBF3020138,
FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023667, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730,
FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000446, FCBBF4000500, FCBBF4000529, FCBBF4000548, FCBBF5000041, FCBBF5000061, FCBBF5000165, FCBBF5000261, FCBBF5000325,
FCBBF5000353, FCBBF5000384, FCBBF5000483, FCBBF5000495, FEBRA1000005, FEBRA1000008, FEBRA1000022, FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000105, FEBRA2000126, FEBRA2000129, FEBRA2000210, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452, FEBRA2000454, FEBRA2000462, FEBRA2000468,
FEBRA2000491, FEBRA2000504, FEBRA2000510, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000740, FEBRA2000757, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197,
FEBRA2001217, FEBRA2001227, FEBRA2001245, FEBRA2001267, FEBRA2001294, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001752, FEBRA2001772, FEBRA2001790, FEBRA2001814, FEBRA2001828, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525,
FEBRA2002527, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003675, FEBRA2003707, FEBRA2003711, FEBRA2003750, FEBRA2003822, FEBRA2003833, FEBRA2003897, FEBRA2003907, FEBRA2003926, FEBRA2004023,
FEBRA2004026, FEBRA2004029, FEBRA2004042, FEBRA2004053, FEBRA2004056, FEBRA2004091, FEBRA2004110, FEBRA2004191, FEBRA2004202, FEBRA2004219, FEBRA2004224, FEBRA2004237, FEBRA2004268, FEBRA2004293, FEBRA2004325, FEBRA2004329, FEBRA2004331, FEBRA2004412, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005572, FEBRA2005701,
FEBRA2005726, FEBRA2005752, FEBRA2005778, FEBRA2005787, FEBRA2005788, FEBRA2005824, FEBRA2005995, FEBRA2005998, FEBRA2006055, FEBRA2006061, FEBRA2006092, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006370, FEBRA2006372, FEBRA2006379, FEBRA2006396, FEBRA2006401, FEBRA2006409, FEBRA2006418, FEBRA2006427, FEBRA2006476, FEBRA2006485, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200,
FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007622, FEBRA2007708, FEBRA2007714, FEBRA2007793, FEBRA2007801, FEBRA2007818, FEBRA2007823, FEBRA2007880, FEBRA2007900, FEBRA2007901, FEBRA2007931, FEBRA2007957, FEBRA2008009, FEBRA2008036, FEBRA2008081, FEBRA2008086, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008287, FEBRA2008302, FEBRA2008311, FEBRA2008341, FEBRA2008360, FEBRA2008459, FEBRA2008468, FEBRA2008475, FEBRA2008481,
FEBRA2008542, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768,
FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013699, FEBRA2013905, FEBRA2013951, FEBRA2014010, FEBRA2014122, FEBRA2014198, FEBRA2014213, FEBRA2014297, FEBRA2014417, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223,
FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022055, FEBRA2022148, FEBRA2022204, FEBRA2022255,
FEBRA2022322, FEBRA2022504, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2025838, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158,
FEBRA2028222, FEBRA2028366, FEBRA2028457, FEBRA2028477, FEBRA2028503, FEBRA2028516, FEHRT2000325, FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000363, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359,
HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000030, HCHON1000131, HCHON1000142, HCHON1000166, HCHON1000176, HCHON1000191, HCHON2000028, HCHON2000038, HCHON2000062, HCHON2000100, HCHON2000156, HCHON2000160, HCHON2000199, HCHON2000212, HCHON2000226, HCHON2000244, HCHON2000265, HCHON2000271, HCHON2000315, HCHON2000322, HCHON2000323, HCHON2000344, HCHON2000364, HCHON2000373, HCHON2000395, HCHON2000417, HCHON2000418, HCHON2000473, HCHON2000475, HCHON2000503, HCHON2000508, HCHON2000626, HCHON2000660, HCHON2000663, HCHON2000673, HCHON2000676, HCHON2000698, HCHON2000699,
HCHON2000705, HCHON2000738, HCHON2000743, HCHON2000751, HCHON2000817, HCHON2000818, HCHON2000819, HCHON2000826, HCHON2000832, HCHON2000851, HCHON2000898, HCHON2000935, HCHON2000942, HCHON2000956, HCHON2000994, HCHON2001039, HCHON2001050, HCHON2001084, HCHON2001099, HCHON2001108, HCHON2001110, HCHON2001116, HCHON2001161, HCHON2001200, HCHON2001214, HCHON2001217, HCHON2001233, HCHON2001269, HCHON2001407, HCHON2001434, HCHON2001450, HCHON2001453, HCHON2001497, HCHON2001505, HCHON2001519, HCHON2001523, HCHON2001535, HCHON2001546, HCHON2001548, HCHON2001577, HCHON2001598, HCHON2001604, HCHON2001607, HCHON2001646, HCHON2001665, HCHON2001712, HCHON2001768, HCHON2001801, HCHON2001853, HCHON2002123,
HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871,
HCHON2008989, HCHON2009191, HCHON2009384, HCHON2009749, HCHON2009766, HCHON2009795, HCHON2010074, HCHON2010543, HEART1000010, HEART1000074, HEART1000088, HEART1000099, HEART1000102, HEART1000118, HEART1000132, HEART1000139, HEART1000142, HEART1000149, HEART1000151, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440,
HEART2003781, HEART2003836, HEART2003852, HEART2004570, HEART2004764, HEART2004931, HEART2004940, HEART2004941 HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000083, HHDPC1000118, HHDPC1000163, HHDPC2000055, HHDPC2000070, HHDPC2000102, HHDPC2000115,
HHDPC2000149, HHDPC2000258, HHDPC2000315, HHDPC2000455, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007267, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000055, HLUNG1000064, HLUNG1000076, HLUNG1000084,
HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000412, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013, HLUNG2001126, HLUNG2001144, HLUNG2001146, HLUNG2001214, HLUNG2001459,
HLUNG2001507, HLUNG2001518, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002562, HLUNG2002648, HLUNG2002659, HLUNG2002707, HLUNG2002811, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003833, HLUNG2003872, HLUNG2003918, HLUNG2003991, HLUNG2004014, HLUNG2004037, HLUNG2004067, HLUNG2004154,
HLUNG2004159, HLUNG2004170, HLUNG2004217, HLUNG2004273, HLUNG2004388, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153,
HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009067, HLUNG2009215, HLUNG2009225, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013851, HLUNG2014262, HLUNG2014288, HLUNG2014449, HLUNG2014821, HLUNG2015184,
HLUNG2015418, HLUNG2015548, HLUNG2015578, HLUNG2015617, HLUNG2016022, HLUNG2016862, HLUNG2017262, HLUNG2017286, HLUNG2017307, HLUNG2017350, HLUNG2017806, HLUNG2018029, HLUNG2018282, HLUNG2018626, HLUNG2019058, HSYRA1000062, HSYRA1000131, HSYRA1000137, HSYRA1000148, HSYRA1000152, HSYRA1000178, HSYRA2000044, HSYRA2000135, HSYRA2000137, HSYRA2000190, HSYRA2000221, HSYRA2000224, HSYRA2000232, HSYRA2000237, HSYRA2000248, HSYRA2000253, HSYRA2000255, HSYRA2000332, HSYRA2000371, HSYRA2000424, HSYRA2000507, HSYRA2000510, HSYRA2000605, HSYRA2000629, HSYRA2000640, HSYRA2000706, HSYRA2000743, HSYRA2000760, HSYRA2000787, HSYRA2000792, HSYRA2000828, HSYRA2000832, HSYRA2000925, HSYRA2000927, HSYRA2001003,
HSYRA2001103, HSYRA2001105, HSYRA2001138, HSYRA2001142, HSYRA2001153, HSYRA2001232, HSYRA2001255, HSYRA2001332, HSYRA2001353, HSYRA2001396, HSYRA2001420, HSYRA2001484, HSYRA2001552, HSYRA2001567, HSYRA2001574, HSYRA2001580, HSYRA2001615, HSYRA2001631, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000158, IMR321000161, IMR321000165, IMR321000193, IMR321000207, IMR321000230, IMR321000242, IMR321000266, IMR322000010, IMR322000072,
IMR322000107, IMR322000127, IMR322000144, IMR322000152, IMR322000164, IMR322000178, IMR322000243, IMR322000302, IMR322000316, IMR322000374, IMR322000379, IMR322000383, IMR322000418, IMR322000518, IMR322000555, IMR322000604, IMR322000609, IMR322000656, IMR322000672, IMR322000730, IMR322000742, IMR322000775, IMR322000791, IMR322000811, IMR322000819, IMR322000838, IMR322000859, IMR322000863, IMR322000917, IMR322000918, IMR322000919, IMR322000935, IMR322000953, IMR322000973, IMR322000984, IMR322001018, IMR322001049, IMR322001167, IMR322001185, IMR322001245, IMR322001317, IMR322001318, IMR322001322, IMR322001332, IMR322001380, IMR322001435, IMR322001438, IMR322001452, IMR322001491, IMR322001534,
IMR322001541, IMR322001584, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, IMR322019070, JCMLC2000273, KIDNE1000008, KIDNE1000012, KIDNE1000015, KIDNE1000028, KIDNE1000036, KIDNE1000064, KIDNE1000104,
KIDNE1000121, KIDNE1000143, KIDNE1000145, KIDNE1000152, KIDNE2000026, KIDNE2000041, KIDNE2000046, KIDNE2000051, KIDNE2000070, KIDNE2000085, KIDNE2000167, KIDNE2000174, KIDNE2000192, KIDNE2000227, KIDNE2000244, KIDNE2000245, KIDNE2000252, KIDNE2000266, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000403, KIDNE2000422, KIDNE2000493, KIDNE2000497, KIDNE2000510, KIDNE2000513, KIDNE2000517, KIDNE2000519, KIDNE2000555, KIDNE2000574, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867,
KIDNE2000909, KIDNE2000947, KIDNE2001117, KIDNE2001140, KIDNE2001162, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882, KIDNE2002883, KIDNE2002980, KIDNE2002991, KIDNE2003185, KIDNE2003188, KIDNE2003233, KIDNE2003305, KIDNE2003316, KIDNE2003335, KIDNE2003357, KIDNE2003373, KIDNE2003377, KIDNE2003507, KIDNE2003752,
KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477; KIDNE2005526, KIDNE2005543, KIDNE2005603, KIDNE2005629, KIDNE2005676, KIDNE2005781,
KIDNE2005798, KIDNE2005854, KIDNE2005908, KIDNE2005937, KIDNE2006009, KIDNE2006014, KIDNE2006030, KIDNE2006039, KIDNE2006053, KIDNE2006062, KIDNE2006092, KIDNE2006145, KIDNE2006149, KIDNE2006210, KIDNE2006248, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007910, KIDNE2007954, KIDNE2008022, KIDNE2008048, KIDNE2008069, KIDNE2008072, KIDNE2008116, KIDNE2008117, KIDNE2008218, KIDNE2008315,
KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762,
KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013845, KIDNE2013866, KIDNE2014087, KIDNE2014112, KIDNE2014119, KIDNE2014170, KIDNE2014184, KIDNE2014268, KIDNE2014298, KIDNE2014320,
KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166,
KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158,
LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2002842, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520,
LIVER2005527, LIVER2005544, LIVER2005625, LIVER2005728, LIVER2005789, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000035, MAMGL1000083, MAMGL1000096, MAMGL1000100, MAMGL1000132, MAMGL1000151, MAMGL1000173, MAMGL1000178, MAMGL1000182, MAMGL1000184, MESAN1000032, MESAN1000035, MESAN1000050, MESAN1000079, MESAN1000080, MESAN1000101, MESAN1000121,
MESAN1000126, MESAN1000147, MESAN1000180, MESAN2000023, MESAN2000026, MESAN2000067, MESAN2000092, MESAN2000149, MESAN2000167, MESAN2000264, MESAN2000291, MESAN2000337, MESAN2000434, MESAN2000457, MESAN2000462, MESAN2000501, MESAN2000572, MESAN2000608, MESAN2000620, MESAN2000711, MESAN2000815, MESAN2000894, MESAN2000909, MESAN2001154, MESAN2001450, MESAN2001627, MESAN2001770, MESAN2001979, MESAN2001980, MESAN2002086, MESAN2002113, MESAN2002122, MESAN2002147, MESAN2002186, MESAN2002424, MESAN2002519, MESAN2002687, MESAN2002709, MESAN2002790, MESAN2002844, MESAN2002940, MESAN2002978, MESAN2003035, MESAN2003037, MESAN2003039, MESAN2003058, MESAN2003101, MESAN2003190, MESAN2003322, MESAN2003444,
MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005633, MESAN2005689, MESAN2005724, MESAN2005766, MESAN2005808, MESAN2005811, MESAN2005957, MESAN2005958, MESAN2006022, MESAN2006043, MESAN2006328, MESAN2006401, MESAN2006580, MESAN2006599, MESAN2006743, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926, MESAN2008936, MESAN2009019, MESAN2009081, MESAN2009105, MESAN2009311, MESAN2009418, MESAN2009503, MESAN2009522,
MESAN2009580, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013284, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017417, MESAN2018209,
MESAN2018576, MESAN2018670, MESAN2018699, MESTC1000042, MESTC1000147, MESTC2000150, MESTC2000153, MESTC2000170, N1ESE2000698, NB9N41000011, NB9N41000047, NB9N41000121, NB9N41000135, NB9N41000142, NB9N41000146, NB9N41000340, NB9N42000104, NB9N42000122, NB9N42000196, NB9N42000281, NB9N42000314, NB9N42000430, NB9N42000495, NB9N42001300, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000163, NT2NE1000175, NT2NE1000181, NT2NE1000185, NT2NE2000038, NT2NE2000054, NT2NE2000056, NT2NE2000057, NT2NE2000064, NT2NE2000109, NT2NE2000137, NT2NE2000156, NT2NE2000174, NT2NE2000195, NT2NE2000222,
NT2NE2000259, NT2NE2000284, NT2NE2000299, NT2NE2000327, NT2NE2000353, NT2NE2000356, NT2NE2000369, NT2NE2000374, NT2NE2000383, NT2NE2000384, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000707, NT2NE2000763, NT2NE2000802, NT2NE2000808, NT2NE2000809, NT2NE2000864, NT2NE2000909, NT2NE2000913, NT2NE2000950, NT2NE2000951, NT2NE2000980, NT2NE2001005, NT2NE2001021, NT2NE2001040, NT2NE2001048, NT2NE2001049, NT2NE2001142, NT2NE2001156, NT2NE2001176, NT2NE2001180, NT2NE2001181, NT2NE2001188, NT2NE2001191, NT2NE2001247,
NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001617, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001793, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2003150, NT2NE2003185, NT2NE2003252, NT2NE2003280, NT2NE2003308, NT2NE2003309,
NT2NE2003315, NT2NE2003393, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004519, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005688, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288,
NT2NE2006382, NT2NE2006387, NT2NE2006427, NT2NE2006432, NT2NE2006458, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654,
NT2NE2010688, NT2NE2010781, NT2NE2010842, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758,
NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099,
NT2RI1000016, NT2RI1000027, NT2RI1000035, NT2RI1000048, NT2RI1000127, NT2RI1000164, NT2RI1000172, NT2RI2000004, NT2RI2000007, NT2RI2000064, NT2RI2000107, NT2RI2000116, NT2RI2000117, NT2RI2000128, NT2RI2000133, NT2RI2000167, NT2RI2000173, NT2RI2000187, NT2RI2000255, NT2RI2000263, NT2RI2000270, NT2RI2000276, NT2RI2000282, NT2RI2000284, NT2RI2000294, NT2RI2000313, NT2RI2000341, NT2RI2000344, NT2RI2000348, NT2RI2000412, NT2RI2000480, NT2RI2000575, NT2RI2000578, NT2RI2000588, NT2RI2000597, NT2RI2000669, NT2RI2000671, NT2RI2000685, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000974, NT2RI2000987,
NT2RI2001010, NT2RI2001017, NT2RI2001030, NT2RI2001054, NT2RI2001083, NT2RI2001091, NT2RI2001167, NT2RI2001178, NT2RI2001235, NT2RI2001244, NT2RI2001307, NT2RI2001385, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001657, NT2RI2001726, NT2RI2001731, NT2RI2001769, NT2RI2001782, NT2RI2001836, NT2RI2001846, NT2RI2001859, NT2RI2001866, NT2RI2001910, NT2RI2001952, NT2RI2002022, NT2RI2002041, NT2RI2002117, NT2RI2002120, NT2RI2002152, NT2RI2002153, NT2RI2002179, NT2RI2002243, NT2RI2002252, NT2RI2002260, NT2RI2002266, NT2RI2002270, NT2RI2002334, NT2RI2002359, NT2RI2002391, NT2RI2002447,
NT2RI2002481, NT2RI2002507, NT2RI2002517, NT2RI2002530, NT2RI2002540, NT2RI2002549, NT2RI2002554, NT2RI2002564, NT2RI2002585, NT2RI2002592, NT2RI2002602, NT2RI2002654, NT2RI2002699, NT2RI2002729, NT2RI2002802, NT2RI2002819, NT2RI2002822, NT2RI2002847, NT2RI2002852, NT2RI2002865, NT2RI2002923, NT2RI2002926, NT2RI2002958, NT2RI2002970, NT2RI2003011, NT2RI2003019, NT2RI2003051, NT2RI2003067, NT2RI2003184, NT2RI2003205, NT2RI2003222, NT2RI2003301, NT2RI2003317, NT2RI2003338, NT2RI2003344, NT2RI2003360, NT2RI2003361, NT2RI2003383, NT2RI2003392, NT2RI2003399, NT2RI2003407, NT2RI2003419, NT2RI2003420, NT2RI2003655, NT2RI2003667, NT2RI2003678, NT2RI2003751, NT2RI2003884, NT2RI2003921, NT2RI2003973,
NT2RI2003993, NT2RI2004059, NT2RI2004078, NT2RI2004079, NT2RI2004093, NT2RI2004136, NT2RI2004157, NT2RI2004188, NT2RI2004190, NT2RI2004209, NT2RI2004230, NT2RI2004290, NT2RI2004304, NT2RI2004312, NT2RI2004381, NT2RI2004398, NT2RI2004410, NT2RI2004442, NT2RI2004468, NT2RI2004535, NT2RI2004606, NT2RI2004608, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005116, NT2RI2005130, NT2RI2005166, NT2RI2005239, NT2RI2005246, NT2RI2005315, NT2RI2005335, NT2RI2005353, NT2RI2005358, NT2RI2005382, NT2RI2005405, NT2RI2005473, NT2RI2005520,
NT2RI2005564, NT2RI2005579, NT2RI2005621, NT2RI2005628, NT2RI2005647, NT2RI2005670, NT2RI2005710, NT2RI2005713, NT2RI2005723, NT2RI2005772, NT2RI2005811, NT2RI2005816, NT2RI2005818, NT2RI2005851, NT2RI2005966, NT2RI2006070, NT2RI2006071, NT2RI2006081, NT2RI2006126, NT2RI2006183, NT2RI2006257, NT2RI2006294, NT2RI2006345, NT2RI2006403, NT2RI2006412, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006825, NT2RI2006838, NT2RI2006853, NT2RI2006855, NT2RI2006943, NT2RI2006973, NT2RI2007046,
NT2RI2007048, NT2RI2007054, NT2RI2007096, NT2RI2007116, NT2RI2007214, NT2RI2007277, NT2RI2007303, NT2RI2007334, NT2RI2007377, NT2RI2007386, NT2RI2007406, NT2RI2007439, NT2RI2007445, NT2RI2007468, NT2RI2007469, NT2RI2007498, NT2RI2007507, NT2RI2007572, NT2RI2007589, NT2RI2007593, NT2RI2007629, NT2RI2007723, NT2RI2007729, NT2RI2007751, NT2RI2007754, NT2RI2007827, NT2RI2007853, NT2RI2007877, NT2RI2007891, NT2RI2007956, NT2RI2007965, NT2RI2007987, NT2RI2008007, NT2RI2008045, NT2RI2008050, NT2RI2008141, NT2RI2008171, NT2RI2008188, NT2RI2008204, NT2RI2008221, NT2RI2008233, NT2RI2008336, NT2RI2008396, NT2RI2008455, NT2RI2008481, NT2RI2008502, NT2RI2008526, NT2RI2008566, NT2RI2008598, NT2RI2008656,
NT2RI2008714, NT2RI2008791, NT2RI2008801, NT2RI2008808, NT2RI2008812, NT2RI2008841, NT2RI2008942, NT2RI2008952, NT2RI2009005, NT2RI2009037, NT2RI2009065, NT2RI2009066, NT2RI2009083, NT2RI2009101, NT2RI2009103, NT2RI2009144, NT2RI2009151, NT2RI2009173, NT2RI2009215, NT2RI2009233, NT2RI2009239, NT2RI2009259, NT2RI2009269, NT2RI2009289, NT2RI2009301, NT2RI2009402, NT2RI2009406, NT2RI2009517, NT2RI2009583, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012350, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733,
NT2RI2015239, NT2RI2015342, NT2RI2015533, NT2RI2016128, NT2RI2016134, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263,
NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007543, NT2RI3007684, NT2RI3007757, NT2RI3007878, NT2RI3007978,
NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000008, NT2RP6000032, NT2RP6000033, NT2RP6000039, NT2RP6000043, NT2RP6000059, NT2RP6000060, NT2RP6000072, NT2RP6000077, NT2RP6000085, NT2RP6000086, NT2RP6000100, NT2RP6000109, NT2RP6000110, NT2RP6000119, NT2RP6000123, NT2RP6000127, NT2RP6000130, NT2RP6000140, NT2RP6000150, NT2RP7000041, NT2RP7000069, NT2RP7000086, NT2RP7000109, NT2RP7000112, NT2RP7000238, NT2RP7000259, NT2RP7000271, NT2RP7000311, NT2RP7000359, NT2RP7000364, NT2RP7000380, NT2RP7000391, NT2RP7000425, NT2RP7000477, NT2RP7000579, NT2RP7000586, NT2RP7000600, NT2RP7000624,
NT2RP7000812, NT2RP7000906, NT2RP7000926, NT2RP7001074, NT2RP7001080, NT2RP7001156, NT2RP7001166, NT2RP7001231, NT2RP7001283, NT2RP7001306, NT2RP7001319, NT2RP7001335, NT2RP7001364, NT2RP7001475, NT2RP7001532, NT2RP7001591, NT2RP7001602, NT2RP7001678, NT2RP7001962, NT2RP7002028, NT2RP7002064, NT2RP7002151, NT2RP7002163, NT2RP7002243, NT2RP7002282, NT2RP7002376, NT2RP7002379, NT2RP7002449, NT2RP7002450, NT2RP7002478, NT2RP7002554, NT2RP7002594, NT2RP7002603, NT2RP7002619, NT2RP7002650, NT2RP7002738, NT2RP7002779, NT2RP7002802, NT2RP7002829, NT2RP7002841, NT2RP7002875, NT2RP7002906, NT2RP7002978, NT2RP7002982, NT2RP7003025, NT2RP7003050, NT2RP7003055, NT2RP7003084, NT2RP7003091, NT2RP7003107,
NT2RP7003134, NT2RP7003148, NT2RP7003203, NT2RP7003261, NT2RP7003319, NT2RP7003439, NT2RP7003511, NT2RP7003629, NT2RP7003632, NT2RP7003647, NT2RP7003680, NT2RP7003688, NT2RP7003724, NT2RP7003960, NT2RP7004027, NT2RP7004080, NT2RP7004114, NT2RP7004123, NT2RP7004173, NT2RP7004196, NT2RP7004204, NT2RP7004233, NT2RP7004260, NT2RP7004348, NT2RP7004352, NT2RP7004358, NT2RP7004373, NT2RP7004396, NT2RP7004428, NT2RP7004481, NT2RP7004541, NT2RP7004559, NT2RP7004641, NT2RP7004656, NT2RP7004687, NT2RP7004722, NT2RP7004728, NT2RP7004751, NT2RP7004766, NT2RP7004790, NT2RP7004884, NT2RP7004911, NT2RP7004915, NT2RP7004925, NT2RP7004946, NT2RP7004961, NT2RP7004964, NT2RP7004975, NT2RP7005118, NT2RP7005205,
NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005750, NT2RP7005846, NT2RP7006033, NT2RP7006075, NT2RP7006141, NT2RP7006160, NT2RP7006162, NT2RP7006188, NT2RP7006223, NT2RP7006263, NT2RP7006296, NT2RP7006304, NT2RP7006374, NT2RP7006395, NT2RP7006408, NT2RP7006457, NT2RP7006539, NT2RP7006547, NT2RP7006601, NT2RP7006619, NT2RP7006621, NT2RP7006636, NT2RP7006701, NT2RP7006853, NT2RP7006886, NT2RP7006887, NT2RP7006980, NT2RP7006986, NT2RP7006995, NT2RP7007114, NT2RP7007154, NT2RP7007221, NT2RP7007226, NT2RP7007252, NT2RP7007269, NT2RP7007310, NT2RP7007359, NT2RP7007381, NT2RP7007387, NT2RP7007406,
NT2RP7007480, NT2RP7007504, NT2RP7007537, NT2RP7007580, NT2RP7007594, NT2RP7007610, NT2RP7007617, NT2RP7007643, NT2RP7007766, NT2RP7007842, NT2RP7007925, NT2RP7007930, NT2RP7007975, NT2RP7008013, NT2RP7008015, NT2RP7008133, NT2RP7008137, NT2RP7008142, NT2RP7008144, NT2RP7008161, NT2RP7008167, NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009097, NT2RP7009108, NT2RP7009149, NT2RP7009168, NT2RP7009215, NT2RP7009225, NT2RP7009236,
NT2RP7009253, NT2RP7009259, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599; NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583,
NT2RP7014721, NT2RP7014743, NT2RP7014778, NT2RP7014906, NT2RP7014910, NT2RP7015080, NT2RP7015431, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633,
NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000033, NTONG1000047, NTONG1000052, NTONG1000075, NTONG1000087, NTONG1000098, NTONG1000123, NTONG1000130, NTONG1000161, NTONG1000172, NTONG1000182, NTONG1000213, NTONG1000214, NTONG1000247, NTONG1000264, NTONG2000040, NTONG2000107, NTONG2000231, NTONG2000265, NTONG2000363, NTONG2000413, NTONG2000492, NTONG2000499, NTONG2000531, NTONG2000583, NTONG2000858, NTONG2000876, NTONG2000878, NTONG2000966, NTONG2000977, NTONG2000985,
NTONG2001014, NTONG2001079, NTONG2001137, NTONG2001157, NTONG2001173, NTONG2001428, NTONG2001532, NTONG2001550, NTONG2001567, NTONG2001612, NTONG2001619, NTONG2001642, NTONG2001657, NTONG2001676, NTONG2001762, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003839, NTONG2003852, NTONG2003897, NTONG2003928, NTONG2004095, NTONG2004308, NTONG2004521, NTONG2004614, NTONG2004690, NTONG2004806, NTONG2004829, NTONG2004918, NTONG2004991, NTONG2005062, NTONG2005086, NTONG2005137, NTONG2005153, NTONG2005265, NTONG2005277, NTONG2005363, NTONG2005373, NTONG2005391,
NTONG2005480, NTONG2005497, NTONG2005520, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008483, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054,
OCBBF1000067, OCBBF1000080, OCBBF1000085, OCBBF1000086, OCBBF1000091, OCBBF1000104, OCBBF1000114, OCBBF1000118, OCBBF1000119, OCBBF1000122, OCBBF1000130, OCBBF1000138, OCBBF1000145, OCBBF1000146, OCBBF1000153, OCBBF1000175, OCBBF1000185, OCBBF1000188, OCBBF1000192, OCBBF1000254, OCBBF1000315, OCBBF2000013, OCBBF2000015, OCBBF2000126, OCBBF2000178, OCBBF2000231, OCBBF2000277, OCBBF2000287, OCBBF2000323, OCBBF2000452, OCBBF2000467, OCBBF2000522, OCBBF2000677, OCBBF2000703, OCBBF2000712, OCBBF2000719, OCBBF2000824, OCBBF2000831, OCBBF2000904, OCBBF2000986, OCBBF2000998, OCBBF2001075, OCBBF2001101, OCBBF2001124, OCBBF2001140, OCBBF2001166, OCBBF2001176, OCBBF2001186, OCBBF2001196, OCBBF2001210,
OCBBF2001252, OCBBF2001307, OCBBF2001323, OCBBF2001389, OCBBF2001402, OCBBF2001408, OCBBF2001416, OCBBF2001473, OCBBF2001492, OCBBF2001494, OCBBF2001527, OCBBF2001528, OCBBF2001586, OCBBF2001639, OCBBF2001681, OCBBF2001687, OCBBF2001706, OCBBF2001749, OCBBF2001794, OCBBF2001910, OCBBF2001931, OCBBF2001961, OCBBF2001983, OCBBF2002015, OCBBF2002083, OCBBF2002124, OCBBF2002161, OCBBF2002290, OCBBF2002357, OCBBF2002376, OCBBF2002615, OCBBF2002626, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003543, OCBBF2003593, OCBBF2003744, OCBBF2003819, OCBBF2004038, OCBBF2004104, OCBBF2004147,
OCBBF2004168, OCBBF2004235, OCBBF2004259, OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005433, OCBBF2005476, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006388, OCBBF2006438, OCBBF2006567, OCBBF2006624, OCBBF2006639, OCBBF2006660, OCBBF2006764,
OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007428, OCBBF2007478, OCBBF2007485, OCBBF2007520, OCBBF2007555, OCBBF2007610, OCBBF2007622, OCBBF2007756, OCBBF2007762, OCBBF2007829, OCBBF2007892, OCBBF2007931, OCBBF2007946, OCBBF2008005, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770,
OCBBF2008775, OCBBF2008790, OCBBF2008814, OCBBF2008822, OCBBF2009095, OCBBF2009115, OCBBF2009301, OCBBF2009352, OCBBF2009391, OCBBF2009424, OCBBF2009536, OCBBF2009571, OCBBF2009583, OCBBF2009603, OCBBF2009772, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010841, OCBBF2010843, OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011160, OCBBF2011177, OCBBF2011228, OCBBF2011232, OCBBF2011283, OCBBF2011311, OCBBF2011536,
OCBBF2011625, OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012553, OCBBF2012678, OCBBF2012704, OCBBF2012714, OCBBF2012755, OCBBF2012812, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013481, OCBBF2013507, OCBBF2013721, OCBBF2013789, OCBBF2013843, OCBBF2013883,
OCBBF2013926, OCBBF2014052, OCBBF2014064, OCBBF2014089, OCBBF2014292, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2016928, OCBBF2017035, OCBBF2017055, OCBBF2017069, OCBBF2017306, OCBBF2017325, OCBBF2017398, OCBBF2017458, OCBBF2017489,
OCBBF2017516, OCBBF2017665, OCBBF2017754, OCBBF2017791, OCBBF2017815, OCBBF2017882, OCBBF2017888, OCBBF2017893, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018707, OCBBF2018827, OCBBF2018828, OCBBF2018873, OCBBF2018934, OCBBF2018956, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788,
OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574,
OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296,
OCBBF3000323, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000174,
PEBLM1000180, PEBLM2000030, PEBLM2000112, PEBLM2000126, PEBLM2000147, PEBLM2000170, PEBLM2000180, PEBLM2000213, PEBLM2000222, PEBLM2000248, PEBLM2000267, PEBLM2000270, PEBLM2000308, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215,
PEBLM2006283, PEBLM2006298, PEBLM2006366, PEBLM2006709, PEBLM2006912, PEBLM2007112, PEBLM2007140, PEBLM2007188, PEBLM2007296, PEBLM2007437, PEBLM2007447, PEBLM2007598, PEBLM2007774, PEBLM2007832, PEBLM2007834, PEBLM2008106, PEBLM2008576, PEBLM2008605, PEBLM2008826, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919,
PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000080, PLACE5000105, PLACE5000113, PLACE5000115, PLACE5000116, PLACE5000129, PLACE5000139, PLACE5000153, PLACE5000159, PLACE5000170, PLACE5000171, PLACE5000172, PLACE5000178,
PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000314, PLACE6000325, PLACE6000348, PLACE6000371, PLACE6000379, PLACE6000414, PLACE6000424, PLACE6000426, PLACE6000429, PLACE6000450, PLACE6000463, PLACE6000523, PLACE6000550, PLACE6000552, PLACE6000555, PLACE6000619, PLACE6000953, PLACE6001064, PLACE6001118, PLACE6001153, PLACE6001185, PLACE6001281, PLACE6001294, PLACE6001443, PLACE6001536, PLACE6001557, PLACE6001712, PLACE6001740, PLACE6001823, PLACE6001886, PLACE6001923,
PLACE6001925, PLACE6001933, PLACE6002011, PLACE6002016, PLACE6002056, PLACE6002084, PLACE6002102, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003077, PLACE6003094, PLACE6003109, PLACE6003204, PLACE6003218, PLACE6003372, PLACE6003383, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004336, PLACE6004368, PLACE6004380, PLACE6004396, PLACE6004397, PLACE6004454, PLACE6004464,
PLACE6004491, PLACE6004578, PLACE6004663, PLACE6004687, PLACE6004738, PLACE6004931, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006549, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373,
PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008775, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010077, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991,
PLACE6011041, PLACE6011057, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210,
PLACE6016254, PLACE6016383, PLACE6016942, PLACE6016984, PLACE6017002, PLACE6017431, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938; PLACE6019195, PLACE6019385, PLACE6019542, PLACE6019600, PLACE6019674, PLACE6019676, PLACE6019701, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657,
PLACE7003684, PLACE7003985, PLACE7004103, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000097, PROST1000110, PROST1000120, PROST1000123, PROST1000136, PROST1000144, PROST1000145, PROST1000152, PROST1000167, PROST1000184, PROST1000199, PROST1000215, PROST1000217, PROST1000220, PROST1000226, PROST1000246, PROST1000262, PROST1000272, PROST1000298, PROST1000322, PROST1000334, PROST1000343,
PROST1000362, PROST1000377, PROST1000383, PROST1000412, PROST1000419, PROST1000451, PROST1000480, PROST1000485, PROST1000493, PROST1000512, PROST1000526, PROST1000527, PROST1000528, PROST1000536, PROST1000559, PROST1000564, PROST1000575, PROST2000036, PROST2000053, PROST2000067, PROST2000105, PROST2000143, PROST2000162, PROST2000165, PROST2000176, PROST2000203, PROST2000206, PROST2000212, PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002,
PROST2001116, PROST2001138, PROST2001180, PROST2001213, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303,
PROST2003324, PROST2003338, PROST2003340, PROST2003396, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004095, PROST2004115, PROST2004142, PROST2004146, PROST2004161, PROST2004258, PROST2004270, PROST2004332, PROST2004416, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426, PROST2005466,
PROST2005604, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006060, PROST2006196, PROST2006201, PROST2006260, PROST2006282, PROST2006343, PROST2006450, PROST2006491, PROST2006510, PROST2006536, PROST2006579, PROST2006688, PROST2006737, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088,
PROST2008243, PROST2008245, PROST2008268, PROST2008271, PROST2008360, PROST2008447, PROST2008468, PROST2008472, PROST2008489, PROST2008516, PROST2008724, PROST2008770, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010782, PROST2010885, PROST2011012, PROST2011038, PROST2011105, PROST2011297, PROST2011410, PROST2011439,
PROST2011482, PROST2011577, PROST2011631, PROST2011660, PROST2012005, PROST2012007, PROST2012016, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015287, PROST2015332, PROST2015457, PROST2015464,
PROST2015537, PROST2015545, PROST2015551, PROST2015636, PROST2015710, PROST2015756, PROST2015877, PROST2015900, PROST2015924, PROST2015932, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017203, PROST2017367, PROST2017413, PROST2017441, PROST2017529, PROST2017578, PROST2017612, PROST2017617, PROST2017692, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583,
PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000065, PUAEN1000081, PUAEN1000085, PUAEN1000087, PUAEN1000121, PUAEN1000147, PUAEN1000161, PUAEN1000164, PUAEN1000175, PUAEN1000239, PUAEN1000275, PUAEN1000276, PUAEN1000288, PUAEN2000080, PUAEN2000152, PUAEN2000175, PUAEN2000247, PUAEN2000312, PUAEN2000394, PUAEN2000497, PUAEN2000535, PUAEN2000594, PUAEN2000684, PUAEN2000942, PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003018,
PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347,
RECTM2001519, RECTM2001666, RECTM2001691, RECTM2001749, RECTM2002172, RECTM2002602, RECTM2002632, SALGL1000005, SALGL1000028, SALGL1000047, SALGL1000065, SALGL1000107, SALGL1000171, SKMUS1000014, SKMUS1000022, SKMUS1000030, SKMUS1000064, SKMUS1000067, SKMUS1000084, SKMUS1000102, SKMUS1000104, SKMUS1000110, SKMUS1000118, SKMUS1000124, SKMUS1000129, SKMUS1000138, SKMUS1000176, SKMUS1000177, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000361, SKMUS2000365, SKMUS2000380, SKMUS2000390, SKMUS2000416, SKMUS2000458, SKMUS2000467, SKMUS2000468,
SKMUS2000484, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000902, SKMUS2000931, SKMUS2000933, SKMUS2000945, SKMUS2000954, SKMUS2000961, SKMUS2000973, SKMUS2000980, SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001454, SKMUS2001492, SKMUS2001501, SKMUS2001543, SKMUS2001580, SKMUS2001585,
SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474,
SKMUS2008585, SKMUS2008607, SKMUS2009190, SKMUS2009232, SKMUS2009479, SKMUS2009557, SKNMC1000110, SKNMC1000123, SKNMC1000124, SKNMC1000137, SKNMC1000159, SKNMC1000168, SKNMC1000229, SKNMC1000251, SKNMC1000264, SKNMC2000065, SKNMC2000097, SKNMC2000224, SKNMC2000256, SKNMC2000305, SKNMC2000356, SKNMC2000374, SKNMC2000583, SKNMC2000593, SKNMC2000612, SKNMC2000622, SKNMC2000649, SKNMC2000698, SKNMC2000877, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504,
SKNMC2007961, SKNMC2009450, SKNSH1000002, SKNSH1000086, SKNSH1000101, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000347, SKNSH2000482, SKNSH2000516, SKNSH2000550, SKNSH2000716, SKNSH2000819, SKNSH2000971, SKNSH2001222, SKNSH2001875, SKNSH2001931, SKNSH2002054, SKNSH2002325, SKNSH2002768, SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149,
SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000137, SMINT1000192, SMINT2000007, SMINT2000018, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000396, SMINT2000400, SMINT2000441, SMINT2000454, SMINT2000468,
SMINT2000518, SMINT2000529, SMINT2000530, SMINT2000537, SMINT2000538, SMINT2000541, SMINT2000558, SMINT2000562, SMINT2000568, SMINT2000602, SMINT2000609, SMINT2000629, SMINT2000659, SMINT2000694, SMINT2000747, SMINT2000811, SMINT2000824, SMINT2000984, SMINT2001114, SMINT2001158, SMINT2001183, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002159, SMINT2002202, SMINT2002210, SMINT2002224, SMINT2002281, SMINT2002311, SMINT2002314, SMINT2002328, SMINT2002414, SMINT2002457, SMINT2002620, SMINT2002689, SMINT2002743, SMINT2002778, SMINT2002880,
SMINT2002882, SMINT2002899, SMINT2002974, SMINT2002976, SMINT2003003, SMINT2003074, SMINT2003128, SMINT2003150, SMINT2003169, SMINT2003317, SMINT2003340, SMINT2003386, SMINT2003423, SMINT2003551, SMINT2003569, SMINT2003641, SMINT2003644, SMINT2003866, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387, SMINT2005402, SMINT2005405, SMINT2005621, SMINT2005623, SMINT2005624, SMINT2005800,
SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921,
SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958,
SMINT2012040, SMINT2012122, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014489, SMINT2014721, SMINT2015006, SMINT2015294, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432,
SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000091, SPLEN1000106, SPLEN1000134, SPLEN1000139, SPLEN1000143, SPLEN1000144, SPLEN1000145, SPLEN1000156, SPLEN1000163, SPLEN1000165, SPLEN1000166, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243,
SPLEN2000247, SPLEN2000255, SPLEN2000258, SPLEN2000267, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000839, SPLEN2001135, SPLEN2001157, SPLEN2001176, SPLEN2001227, SPLEN2001245, SPLEN2001278, SPLEN2001354, SPLEN2001367, SPLEN2001383, SPLEN2001476, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007,
SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002477, SPLEN2002493, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004220, SPLEN2004343, SPLEN2004346, SPLEN2004368, SPLEN2004555, SPLEN2004611, SPLEN2004662, SPLEN2004773, SPLEN2004844, SPLEN2004880, SPLEN2005009, SPLEN2005227,
SPLEN2005272, SPLEN2005416, SPLEN2005429, SPLEN2005450, SPLEN2005474, SPLEN2005560, SPLEN2005727, SPLEN2005783, SPLEN2005790, SPLEN2005818, SPLEN2005838, SPLEN2005869, SPLEN2005927, SPLEN2005939, SPLEN2006133, SPLEN2006143, SPLEN2006232, SPLEN2006268, SPLEN2006283, SPLEN2006305, SPLEN2006325, SPLEN2006374, SPLEN2006389, SPLEN2006425, SPLEN2006671, SPLEN2006701, SPLEN2006785, SPLEN2006875, SPLEN2007182, SPLEN2007314, SPLEN2007326, SPLEN2007350, SPLEN2007388, SPLEN2007498, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496,
SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010195, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252,
SPLEN2011419, SPLEN2011422, SPLEN2011672, SPLEN2011737, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080,
SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015310, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045,
SPLEN2016053, SPLEN2016069, SPLEN2016098, SPLEN2016135, SPLEN2016139, SPLEN2016241, SPLEN2016250, SPLEN2016268, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018098,
SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223,
SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024273, SPLEN2024383, SPLEN2024489, SPLEN2024530, SPLEN2024571, SPLEN2024577, SPLEN2024617, SPLEN2024737, SPLEN2024922,
SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027113, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424,
SPLEN2031547, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678,
SPLEN2037722, SPLEN2037891, SPLEN2038055, SPLEN2038180, SPLEN2038345, SPLEN2038407, SPLEN2038473, SPLEN2038834, SPLEN2039311, SPLEN2039379, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168,
STOMA2000183, STOMA2000188, STOMA2000257, STOMA2000279, STOMA2000289, STOMA2000383, STOMA2000386, STOMA2000395, STOMA2000396, STOMA2000478, STOMA2000482, STOMA2000529, STOMA2000539, STOMA2000567, STOMA2000640, STOMA2000678, ST.OMA2000686, STOMA2000888, STOMA2001025, STOMA2001389, STOMA2002052, STOMA2002141, STOMA2002166, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213,
STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000124, SYNOV1000128, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001033, SYNOV2001035, SYNOV2001088, SYNOV2001111,
SYNOV2001144, SYNOV2001212, SYNOV2001251, SYNOV2001300, SYNOV2001356, SYNOV2001374, SYNOV2001390, SYNOV2001451, SYNOV2001457, SYNOV2001468, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2009172, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224,
SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007671, SYNOV4007711, SYNOV4008245, SYNOV4008336, SYNOV4009129, SYNOV4009139, SYNOV4009298, SYNOV4009575, T1ESE2000116, T1ESE2000609, T1ESE2000904, T1ESE2002665, TBAES2000004, TBAES2000059, TBAES2000116, TBAES2000255, TBAES2000315, TBAES2000352, TBAES2000932, TBAES2001171,
TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TBAES2009387, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312,
TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865,
TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000023, TESTI1000025, TESTI1000029, TESTI1000030, TESTI1000042, TESTI1000045, TESTI1000051, TESTI1000055, TESTI1000064, TESTI1000.070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179,
TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179, TESTI2000184, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000598, TESTI2000600, TESTI2000616, TESTI2000627, TESTI2000644, TESTI2000671,
TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001099, TESTI2001120, TESTI2001134, TESTI2001141, TESTI2001153, TESTI2001180, TESTI2001208, TESTI2001229, TESTI2001236, TESTI2001237, TESTI2001269, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593,
TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365,
TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003074, TESTI2003089, TESTI2003104, TESTI2003109, TESTI2003117, TESTI2003130, TESTI2003131,
TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003413, TESTI2003418, TESTI2003419, TESTI2003432, TESTI2003454, TESTI2003475, TESTI2003476, TESTI2003498, TESTI2003512, TESTI2003533, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727,
TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675,
TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568,
TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005759, TESTI2005767, TESTI2005775, TESTI2005784, TESTI2005788, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006097, TESTI2006109,
TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006872, TESTI2006877,
TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007407, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972,
TESTI2007973, TESTI2007998, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008240, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960,
TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009477, TESTI2009497, TESTI2009502, TESTI2009511, TESTI2009520, TESTI2009544, TESTI2009545, TESTI2009551, TESTI2009577, TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591,
TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356,
TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889,
TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015335, TESTI2015350, TESTI2015375, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033,
TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816,
TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018462, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648,
TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021124, TESTI2021138,
TESTI2021297, TESTI2021315, TESTI2021358, TESTI2021425, TESTI2021447, TESTI2021463, TESTI2021531, TESTI2021599, TESTI2021637, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414,
TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025022, TESTI2025063, TESTI2025117, TESTI2025144, TESTI2025161, TESTI2025174, TESTI2025229, TESTI2025269, TESTI2025365,
TESTI2025403, TESTI2025409, TESTI2025422, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026647, TESTI2026674, TESTI2026794, TESTI2026824, TESTI2026854, TESTI2026925,
TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776,
TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643,
TESTI2032681, TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309,
TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065,
TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362,
TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833,
TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046456, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109,
TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723,
TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665,
TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002799, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133,
TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799,
TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475,
TESTI4010544, TESTI4010713, TESTI4010721, TESTI4010789, TESTI4010817, TESTI4010831, TESTI4010851, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735,
TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865,
TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920,
TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390,
TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335,
TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037156, TESTI4037188, TESTI4037228, TESTI4037244, TESTI4037727,
TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904,. TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711,
TESTI4042846, TESTI4043067, TESTI4043129, TESTI4043140, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903,
TESTI4046962, TESTI4047069, TESTI4047119, TESTI4047305, TESTI4047328, TESTI4047437, TESTI4047569, TESTI4047746, TESTI4047808, THYMU1000002, THYMU1000010, THYMU1000017, THYMU1000025, THYMU1000029, THYMU1000032, THYMU1000033, THYMU1000041, THYMU1000060, THYMU1000074, THYMU1000083, THYMU1000098, THYMU1000103, THYMU1000105, THYMU1000109, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000374, THYMU1000382, THYMU1000393, THYMU1000399, THYMU1000426, THYMU1000428, THYMU1000440, THYMU1000459,
THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000382, THYMU2000418, THYMU2000436, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879,
THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242,
THYMU2002338, THYMU2002356, THYMU2002376, THYMU2002384, THYMU2002450, THYMU2002490, THYMU2002548, THYMU2002583, THYMU2002695, THYMU2002745, THYMU2002756, THYMU2002789, THYMU2002792, THYMU2002815, THYMU2002841, THYMU2002852, THYMU2002910, THYMU2002950, THYMU2002983, THYMU2003046, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003446, THYMU2003470, THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2004003, THYMU2004008,
THYMU2004042, THYMU2004108, THYMU2004111, THYMU2004123, THYMU2004139, THYMU2004152, THYMU2004169, THYMU2004284, THYMU2004320, THYMU2004336, THYMU2004344, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005546, THYMU2005574,
THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006303, THYMU2006324, THYMU2006350, THYMU2006357, THYMU2006365, THYMU2006420, THYMU2006468, THYMU2006505, THYMU2006610, THYMU2006666, THYMU2006712, THYMU2006813, THYMU2006913, THYMU2006946, THYMU2006965, THYMU2007025, THYMU2007036, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528,
THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101,
THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538,
THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757,
THYMU2013779, THYMU2013863, THYMU2013916, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158,
THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020491, THYMU2020499, THYMU2020560, THYMU2020667, THYMU2020830,
THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734,
THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696,
THYMU2032715, THYMU2032732, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234,
THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504,
THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485,
THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701,
THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642,
THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983,
THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758,
THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059,
TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025,
TKIDN2015071, TKIDN2015161, TKIDN2015263, TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192,
TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000030, TRACH1000038, TRACH1000057, TRACH1000063, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000045, TRACH2000047,
TRACH2000081, TRACH2000083, TRACH2000148, TRACH2000185, TRACH2000235, TRACH2000237, TRACH2000243, TRACH2000248, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000461, TRACH2000472, TRACH2000496, TRACH2000497, TRACH2000502, TRACH2000522, TRACH2000559, TRACH2000660, TRACH2000665, TRACH2000675, TRACH2000677, TRACH2000702, TRACH2000703, TRACH2000767, TRACH2000775, TRACH2000807, TRACH2000862, TRACH2000873, TRACH2000894, TRACH2000898, TRACH2000920, TRACH2000923, TRACH2000926, TRACH2000944, TRACH2000959, TRACH2000972, TRACH2000981, TRACH2000986, TRACH2001021,
TRACH2001037, TRACH2001060, TRACH2001085, TRACH2001101, TRACH2001121, TRACH2001147, TRACH2001154, TRACH2001188, TRACH2001192, TRACH2001249, TRACH2001275, TRACH2001289, TRACH2001314, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001463, TRACH2001474, TRACH2001478, TRACH2001525, TRACH2001532, TRACH2001549, TRACH2001592, TRACH2001596, TRACH2001607, TRACH2001612, TRACH2001621, TRACH2001646, TRACH2001651, TRACH2001684, TRACH2001898, TRACH2001933, TRACH2001968, TRACH2001996, TRACH2002054, TRACH2002092, TRACH2002100, TRACH2002138, TRACH2002333, TRACH2002537, TRACH2002664, TRACH2002784,
TRACH2002803, TRACH2002840, TRACH2002847, TRACH2002853, TRACH2002862, TRACH2002954, TRACH2002960, TRACH2002988, TRACH2003070, TRACH2003272, TRACH2003323, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800,
TRACH2005811, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007399, TRACH2007483, TRACH2007542, TRACH2007577, TRACH2007674, TRACH2007676, TRACH2007688, TRACH2007733, TRACH2007816, TRACH2007894, TRACH2007969, TRACH2007988, TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268,
TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012497, TRACH2012536, TRACH2012562,
TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014077, TRACH2014082, TRACH2014124, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015823,
TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080,
TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084,
TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002192, TRACH3002245, TRACH3002293, TRACH3002533, TRACH3002561, TRACH3002567, TRACH3002605, TRACH3002650, TRACH3002752, TRACH3002866, TRACH3002871, TRACH3002876,
TRACH3002890, TRACH3003009, TRACH3003037, TRACH3003357, TRACH3003379, TRACH3003547, TRACH3003586, TRACH3003683, TRACH3003780, TRACH3003805, TRACH3003872, TRACH3004068, TRACH3004113, TRACH3004288; TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264,
TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007625, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715,
TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136,
TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930,
TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681,
TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145,
TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036997, TRACH3037063, TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274,
TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000152, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131,
UTERU1000138, UTERU1000148, UTERU1000160, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000263, UTERU2000300, UTERU2000329, UTERU2000338, UTERU2000349, UTERU2000377, UTERU2000393, UTERU2000418, UTERU2000424, UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925,
UTERU2001024, UTERU2001110, UTERU2001176, UTERU2001281, UTERU2001389, UTERU2001409, UTERU2001412, UTERU2001504, UTERU2001607, UTERU2001658, UTERU2001747, UTERU2001876, UTERU2002001, UTERU2002011, UTERU2002176, UTERU2002198, UTERU2002294, UTERU2002332, UTERU2002410, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004037, UTERU2004039, UTERU2004061, UTERU2004073, UTERU2004163, UTERU2004197, UTERU2004299,
UTERU2004461, UTERU2004520, UTERU2004564, UTERU2004688, UTERU2004698, UTERU2004807, UTERU2004861, UTERU2004929, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005601, UTERU2005621, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499,
UTERU2007639, UTERU2007724, UTERU2007924, UTERU2007942, UTERU2008018, UTERU2008019, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431,
UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012286, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502,
UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015062, UTERU2015087, UTERU2015190, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757, UTERU2016761, UTERU2016799, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810,
UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045,
UTERU2023175, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703,
UTERU2031834, UTERU2031851, UTERU2032075, UTERU2032220, UTERU2032279, UTERU2032726, UTERU2033172, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670,
UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049,
UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092,
UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the NT2NE, NT2RI, NT2RM, NT2RP, NTISM, NTONG, and OCBBF libraries were analyzed for expression frequency. The analysis results are described below. Each is a relative value, where the expression level over all tissues is taken as 100. The larger this value, the greater the level of expression.

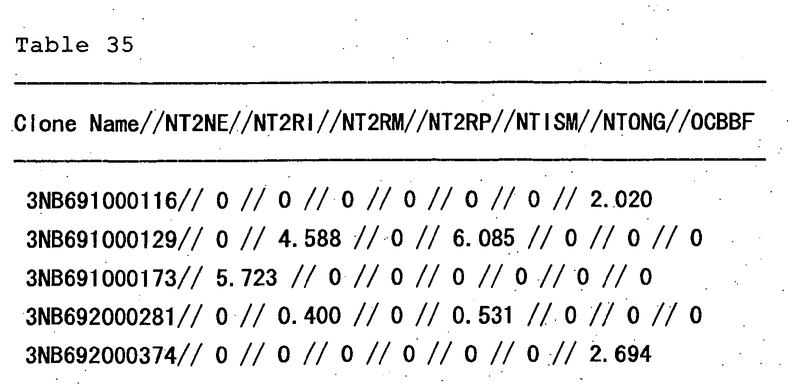

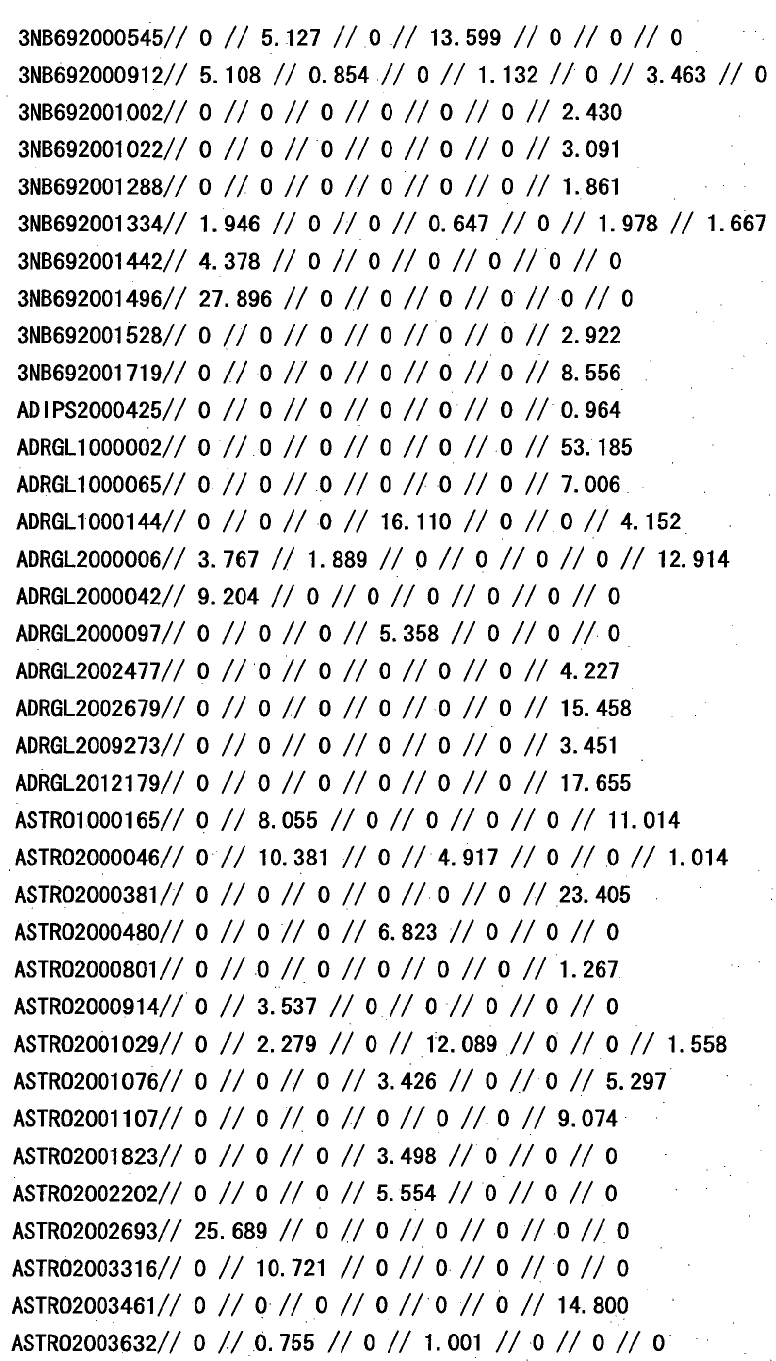

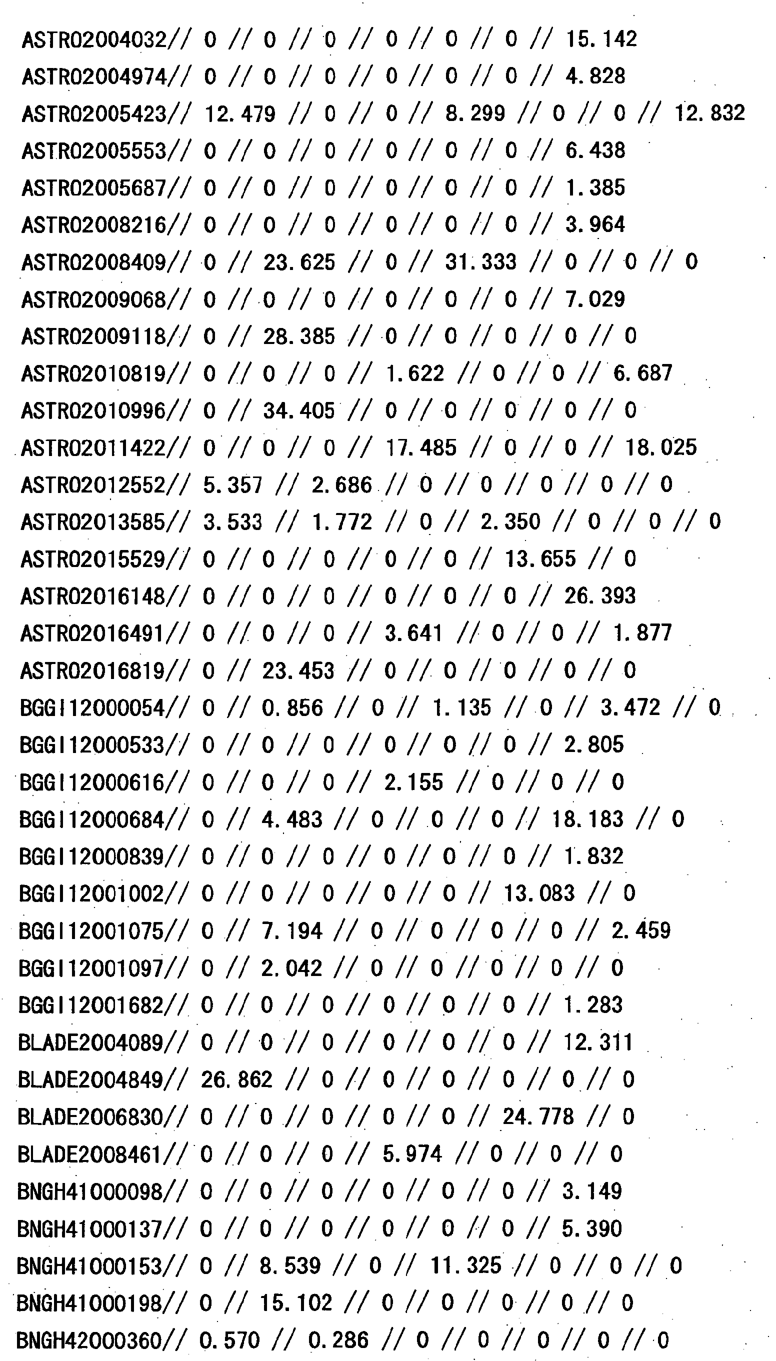

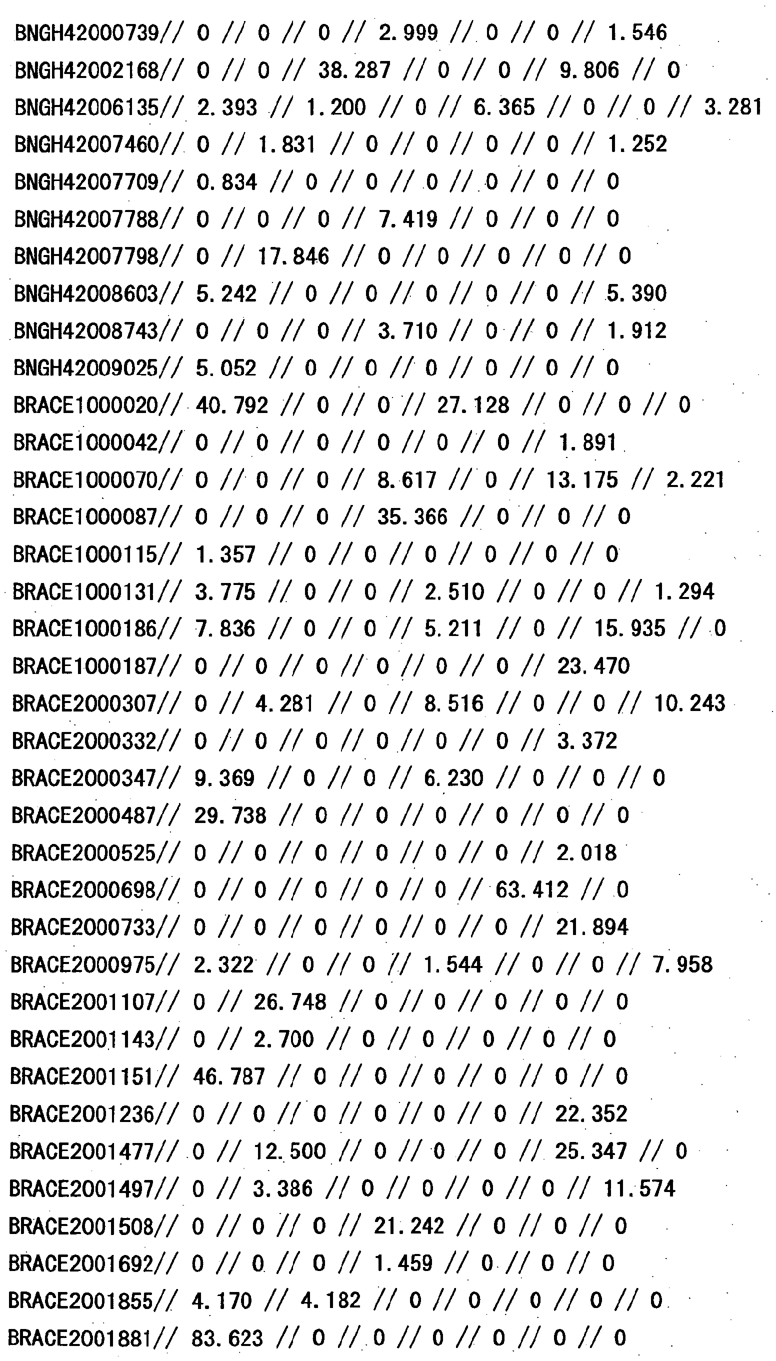

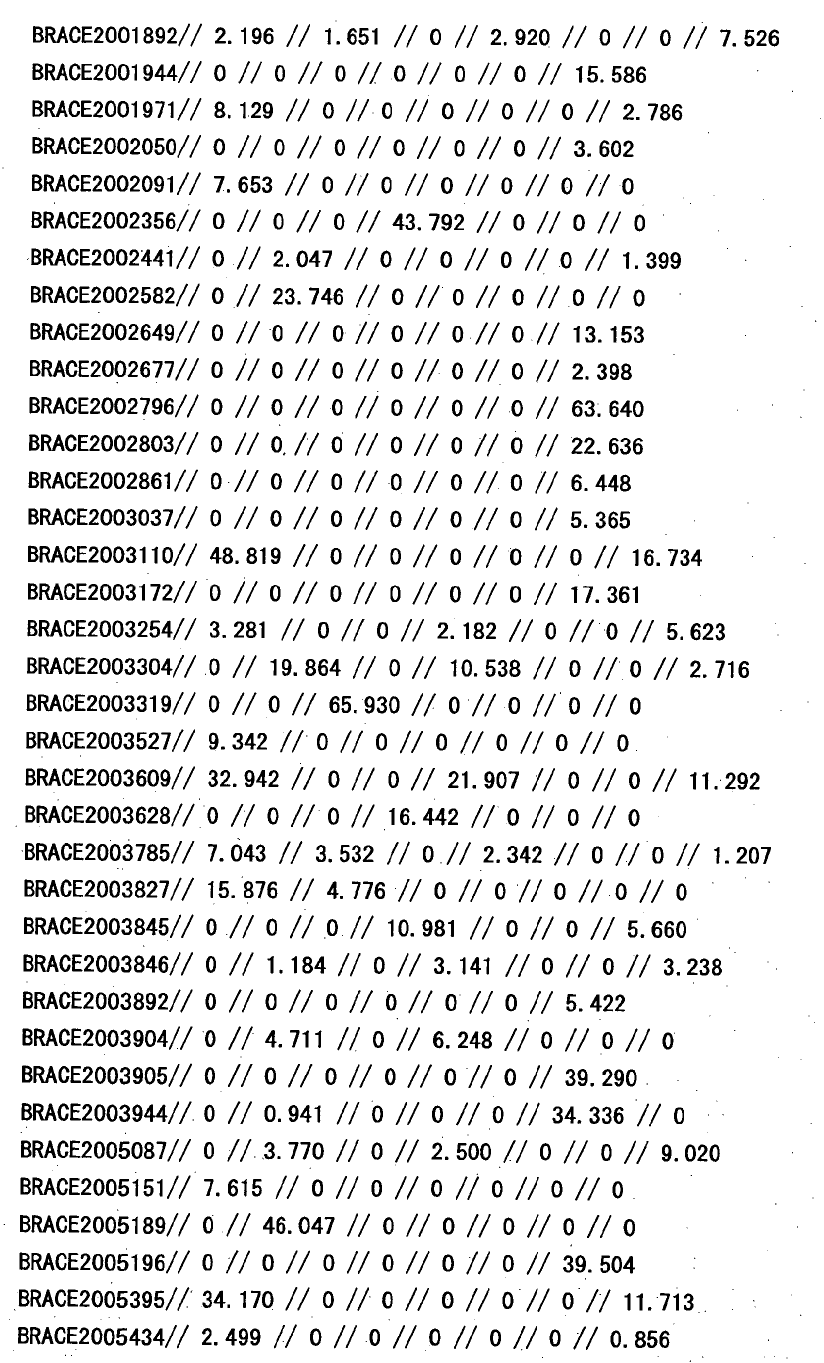

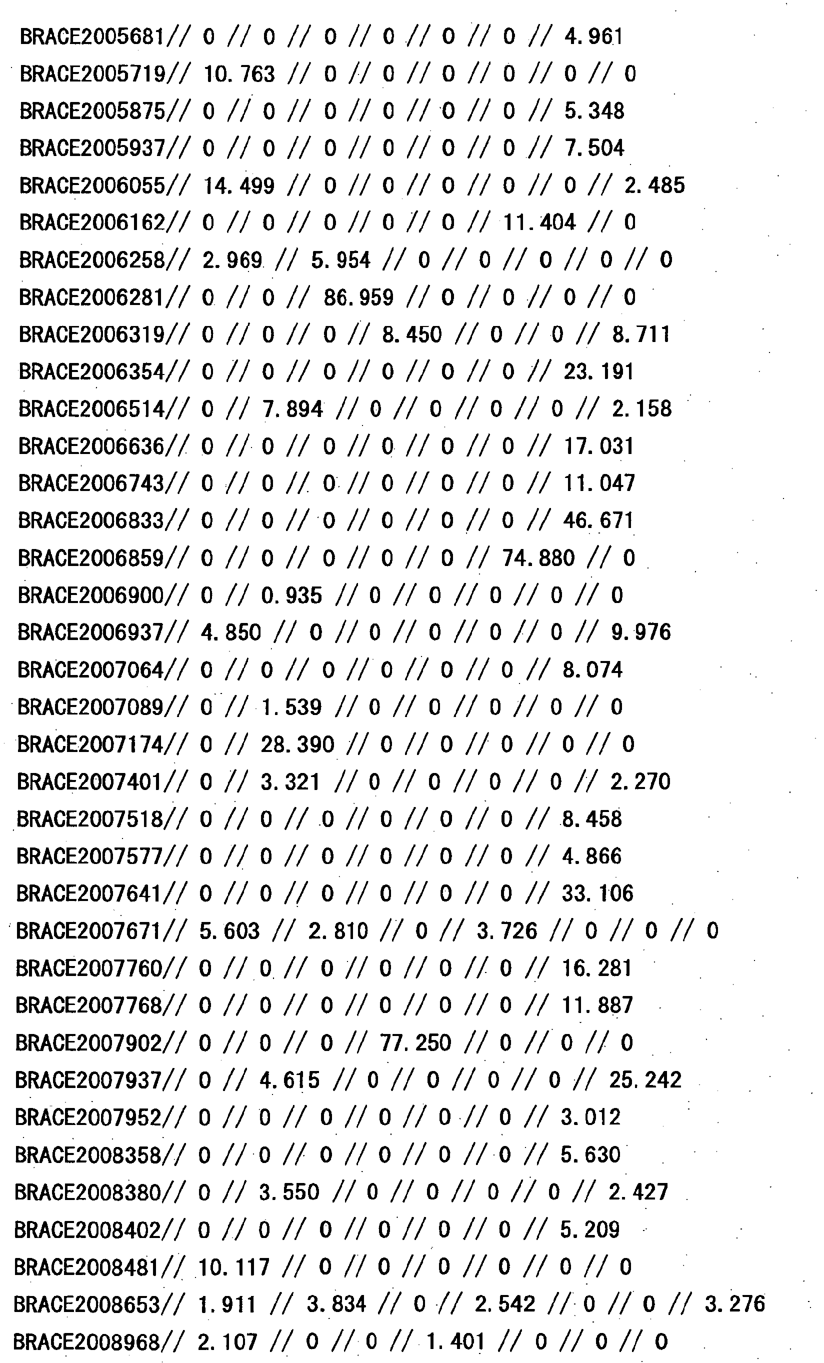

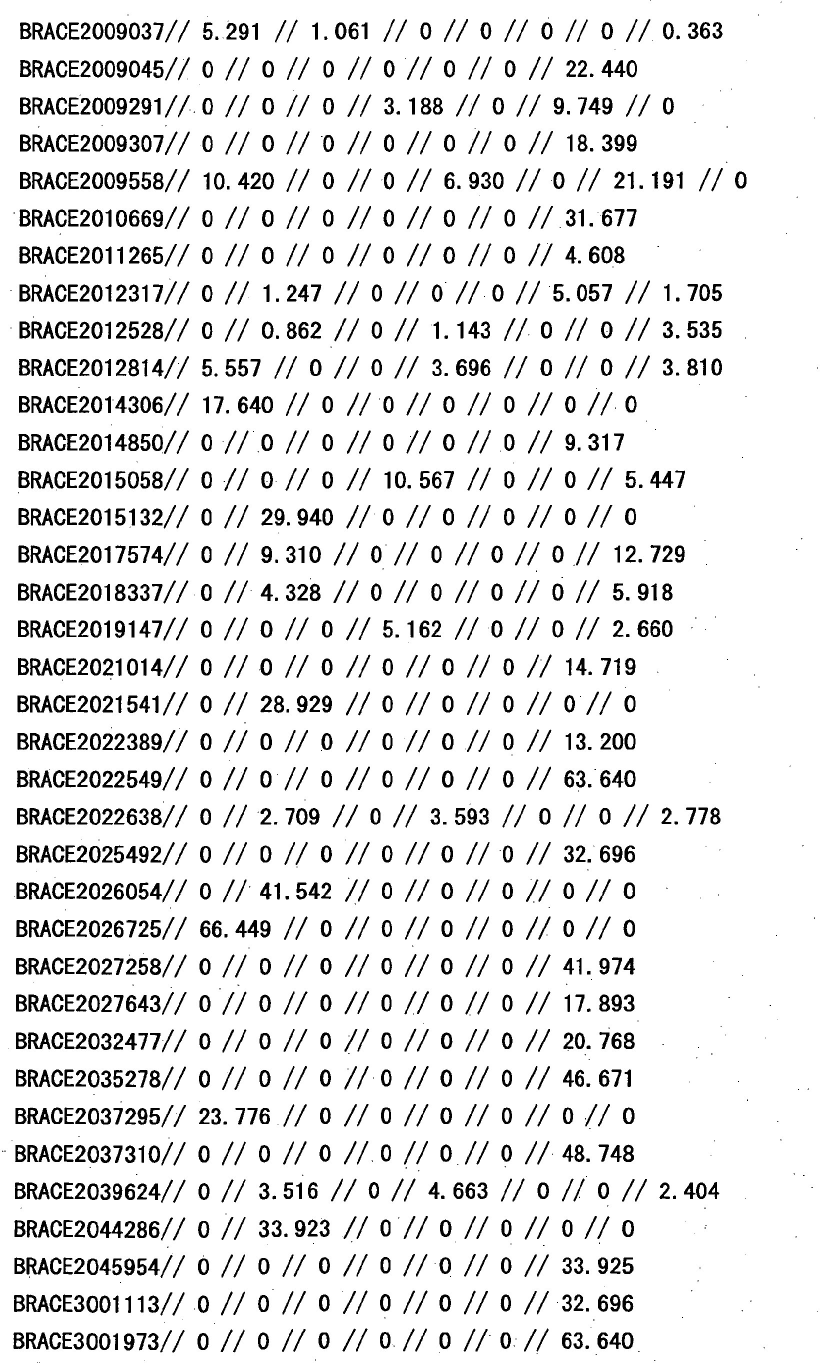

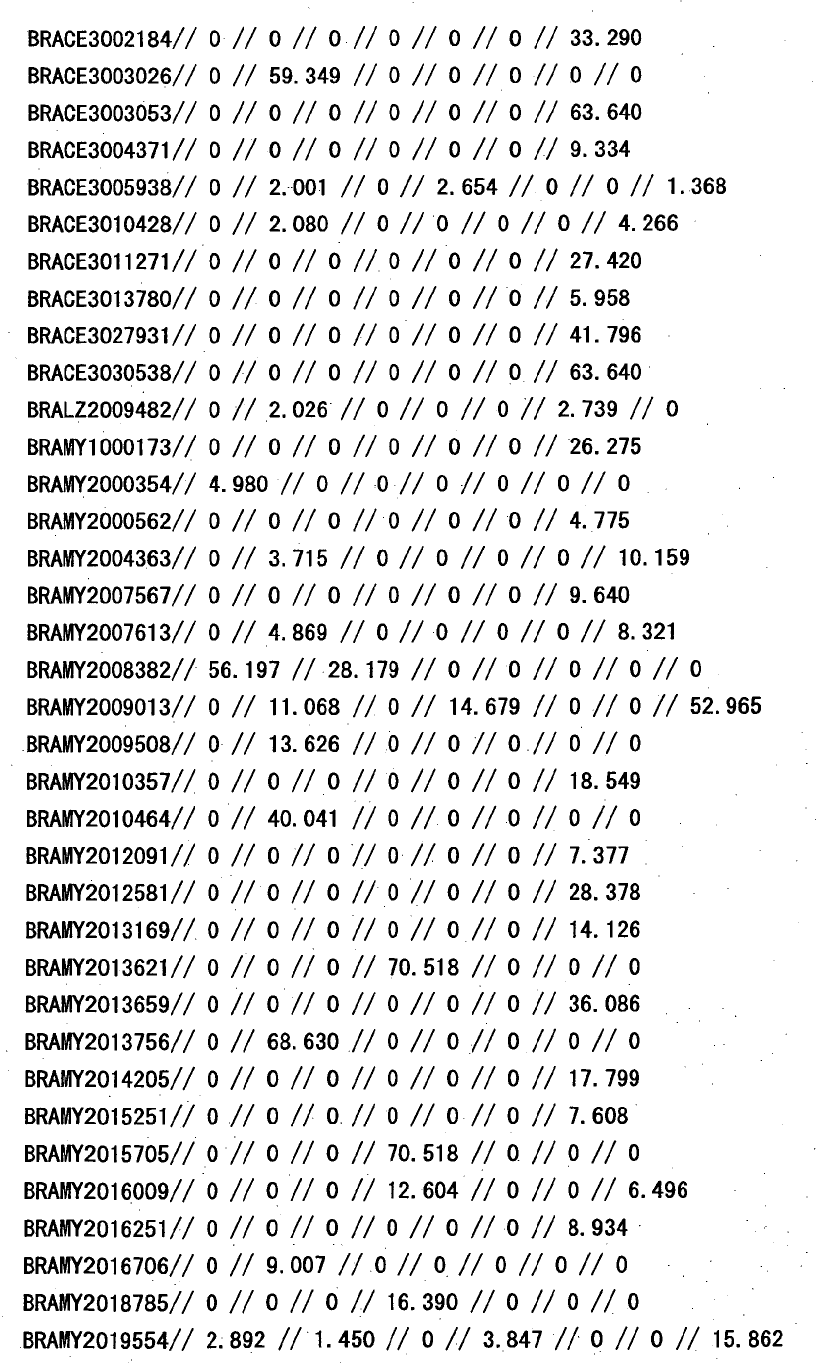

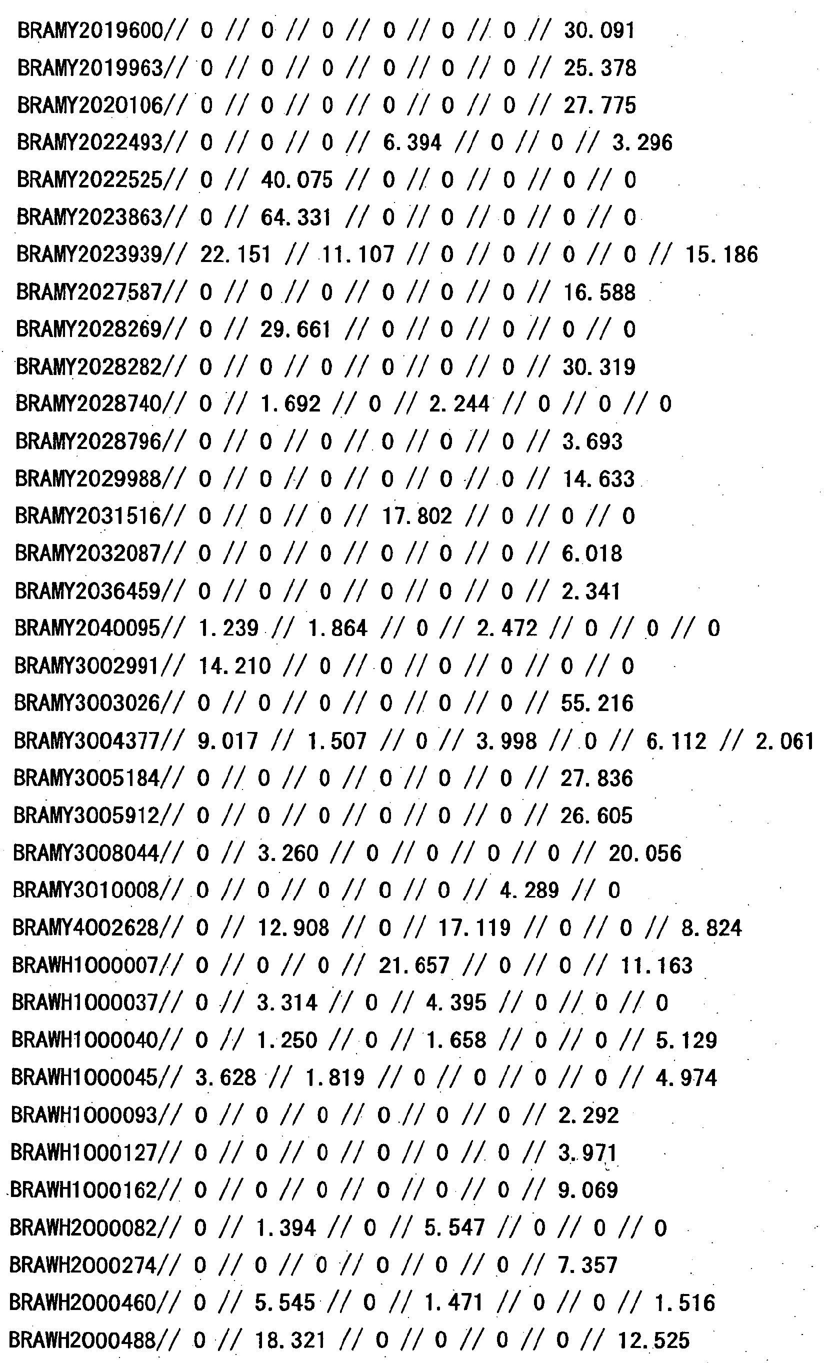

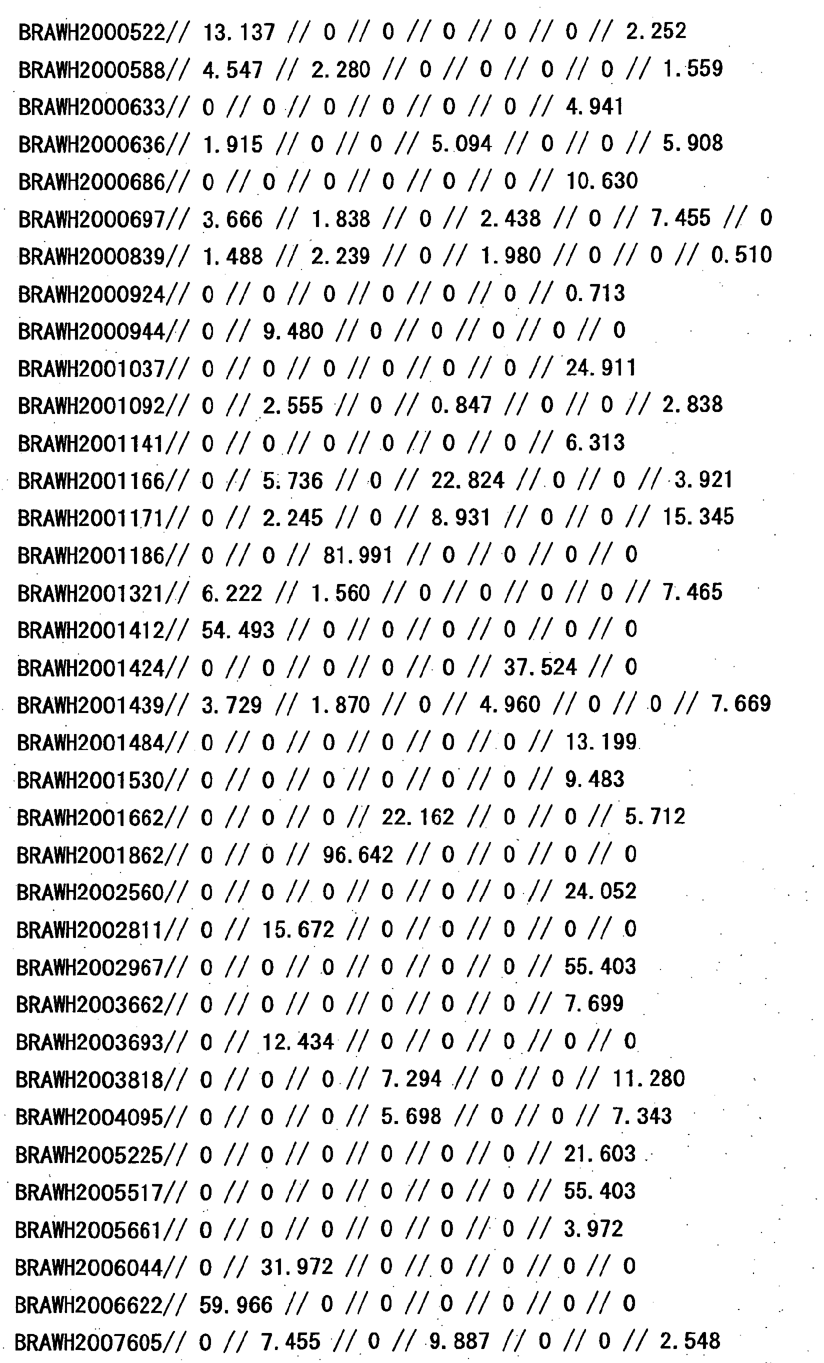

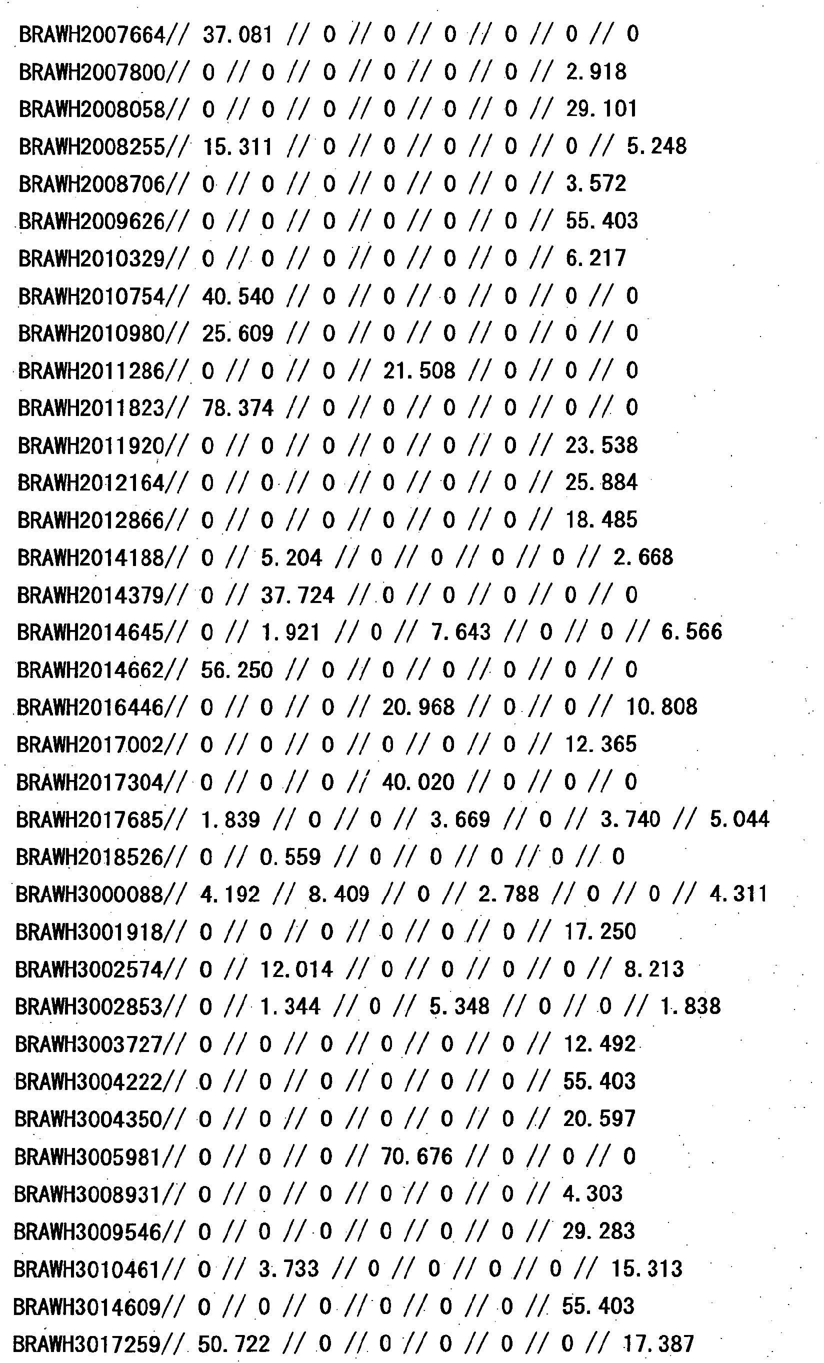

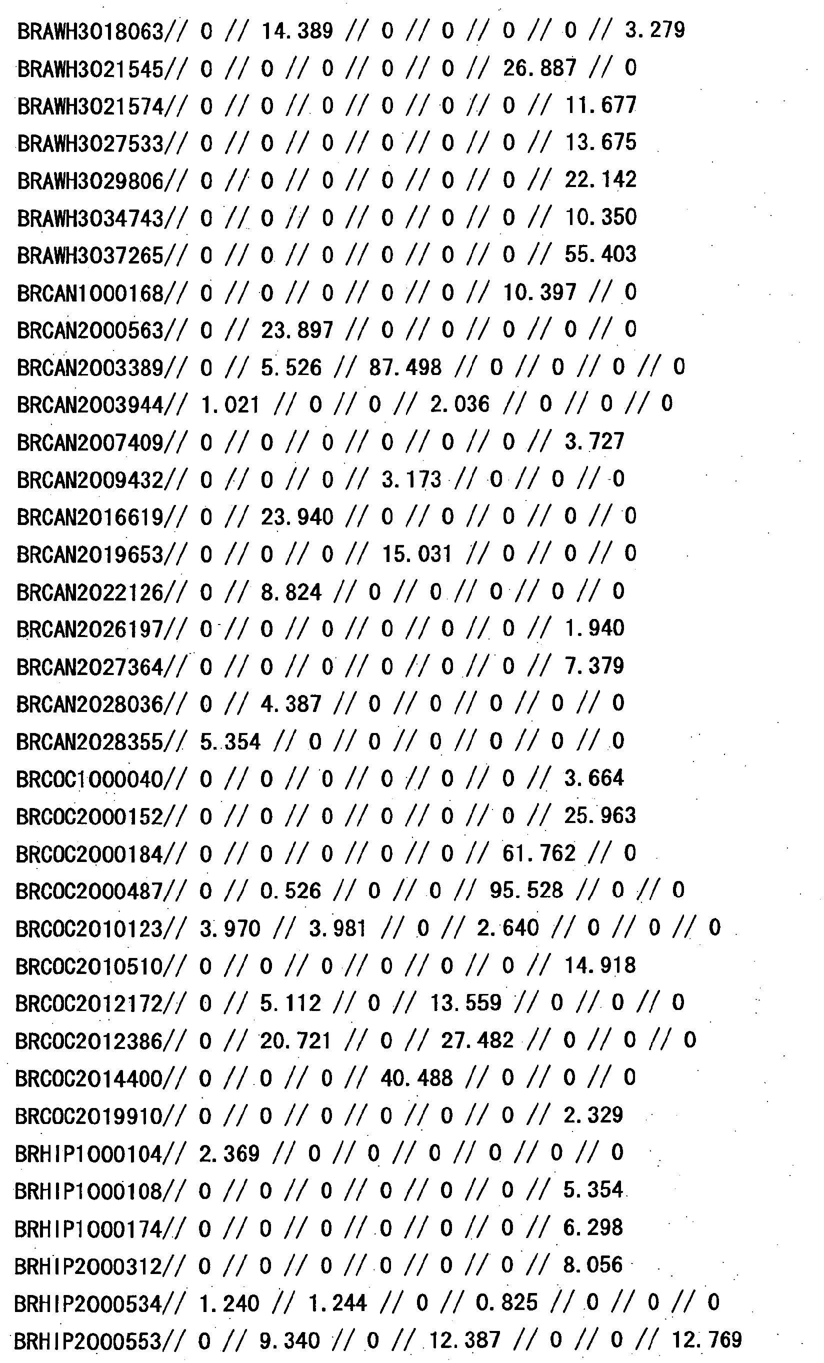

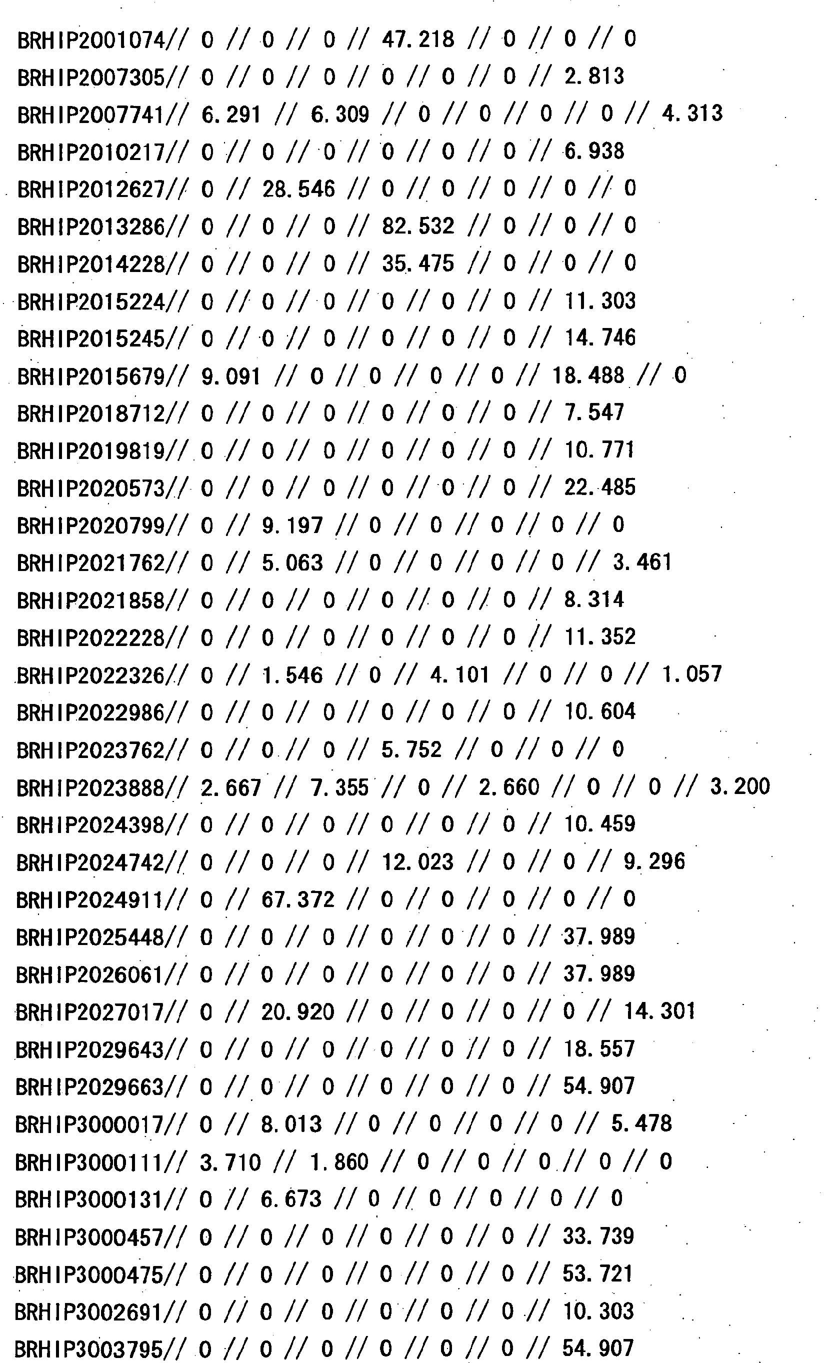

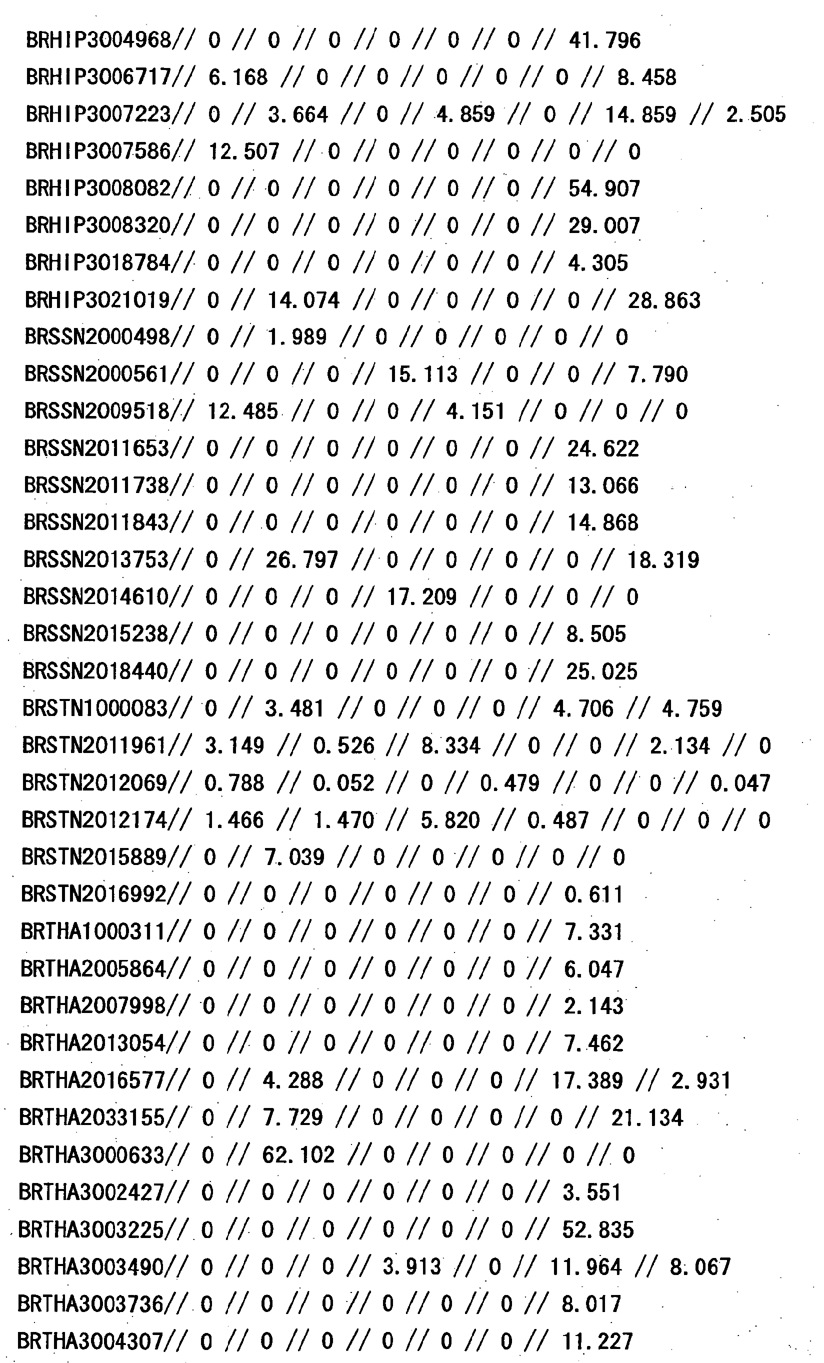

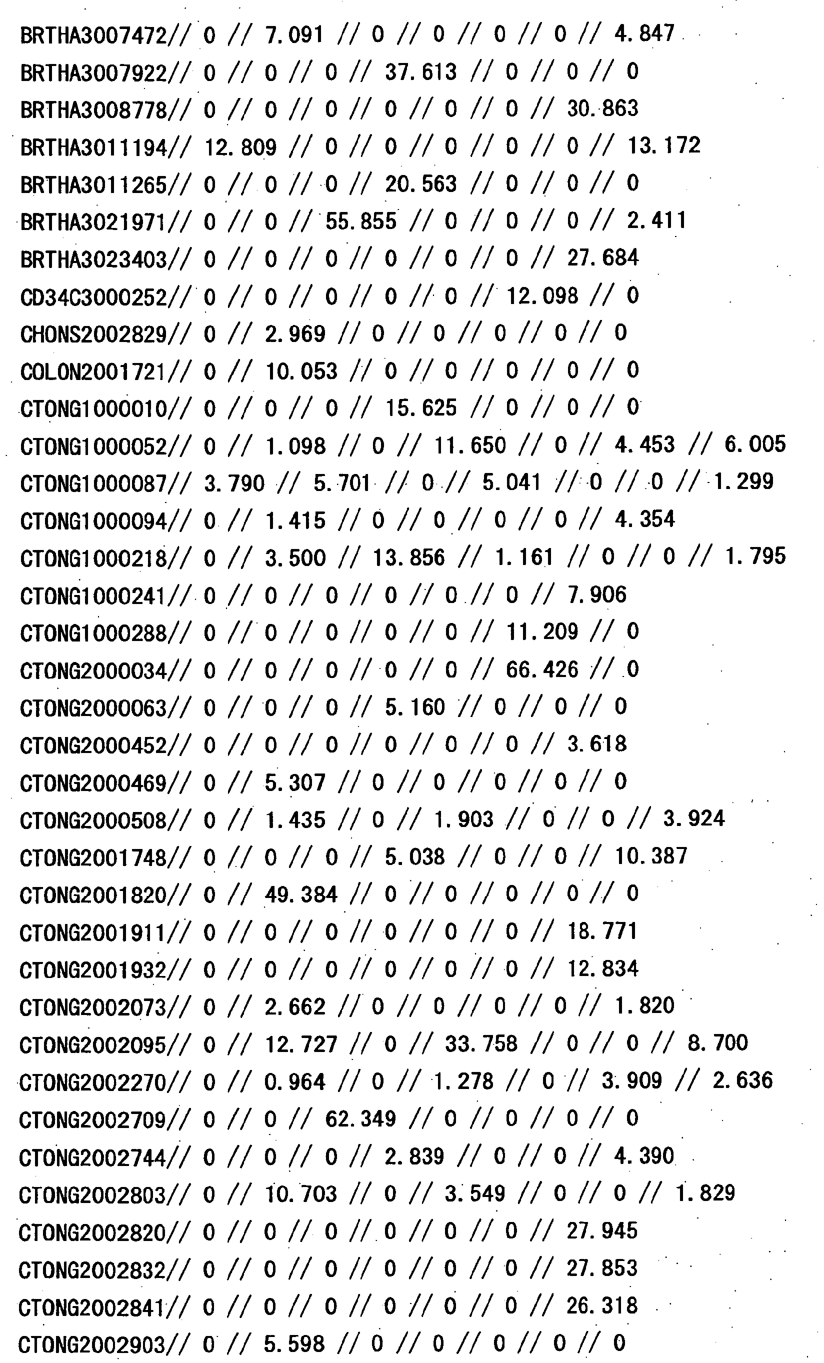

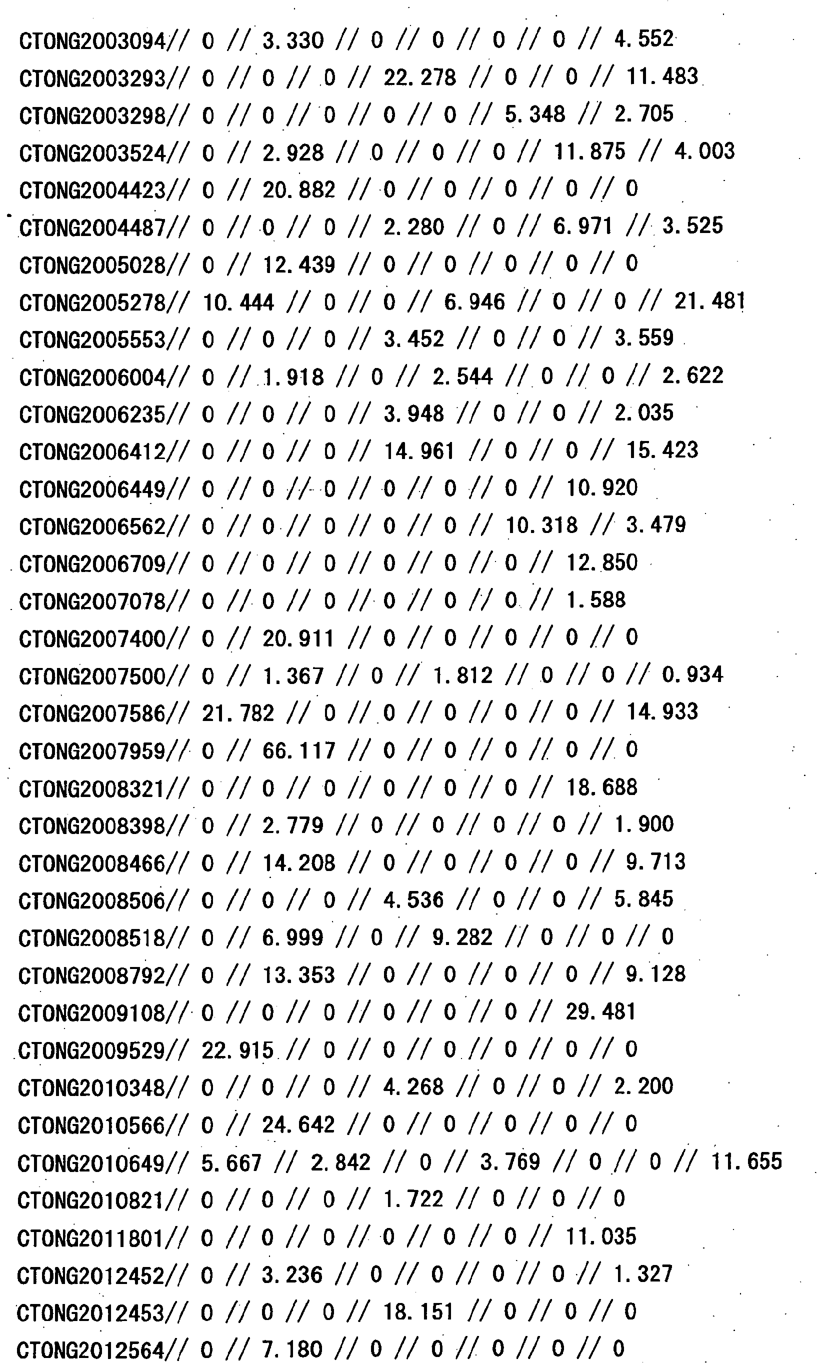

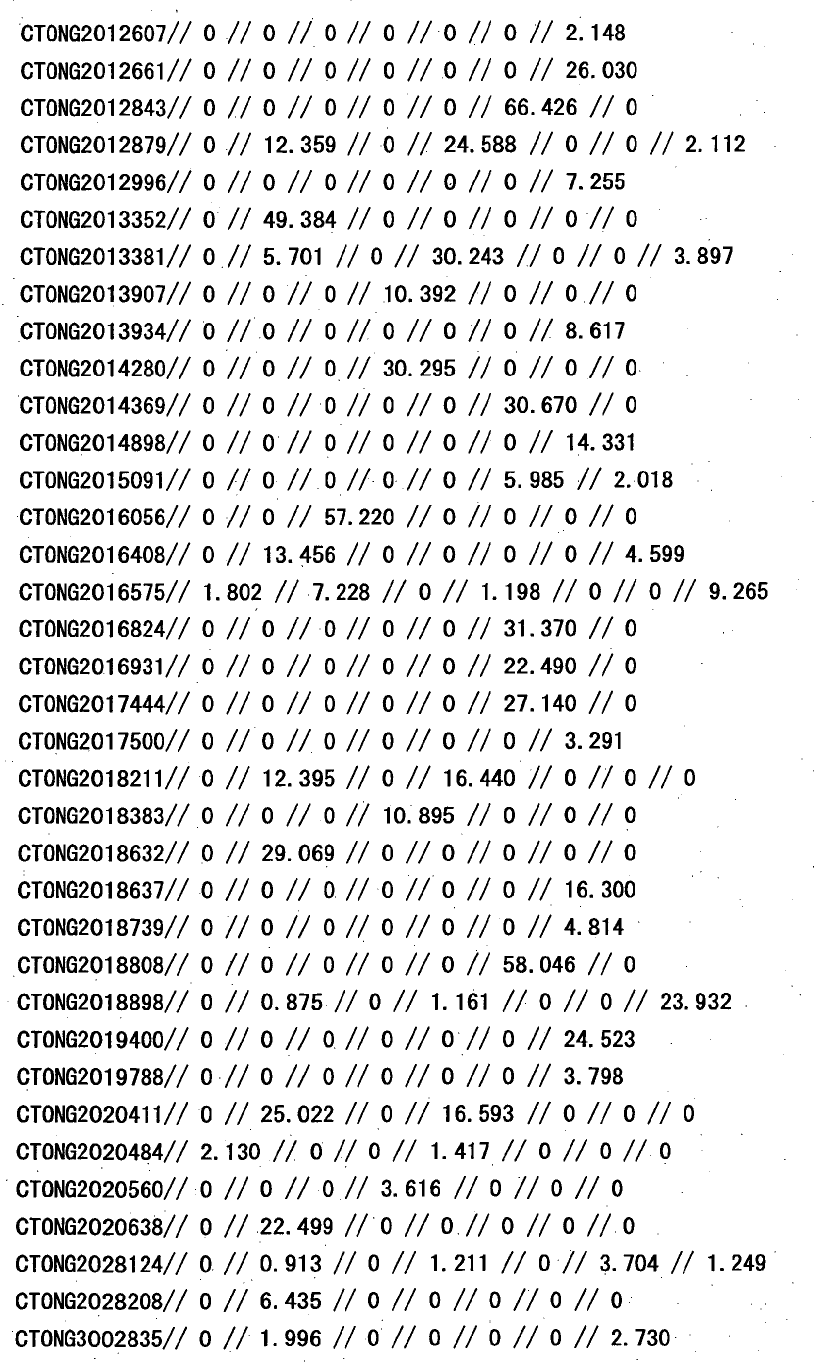

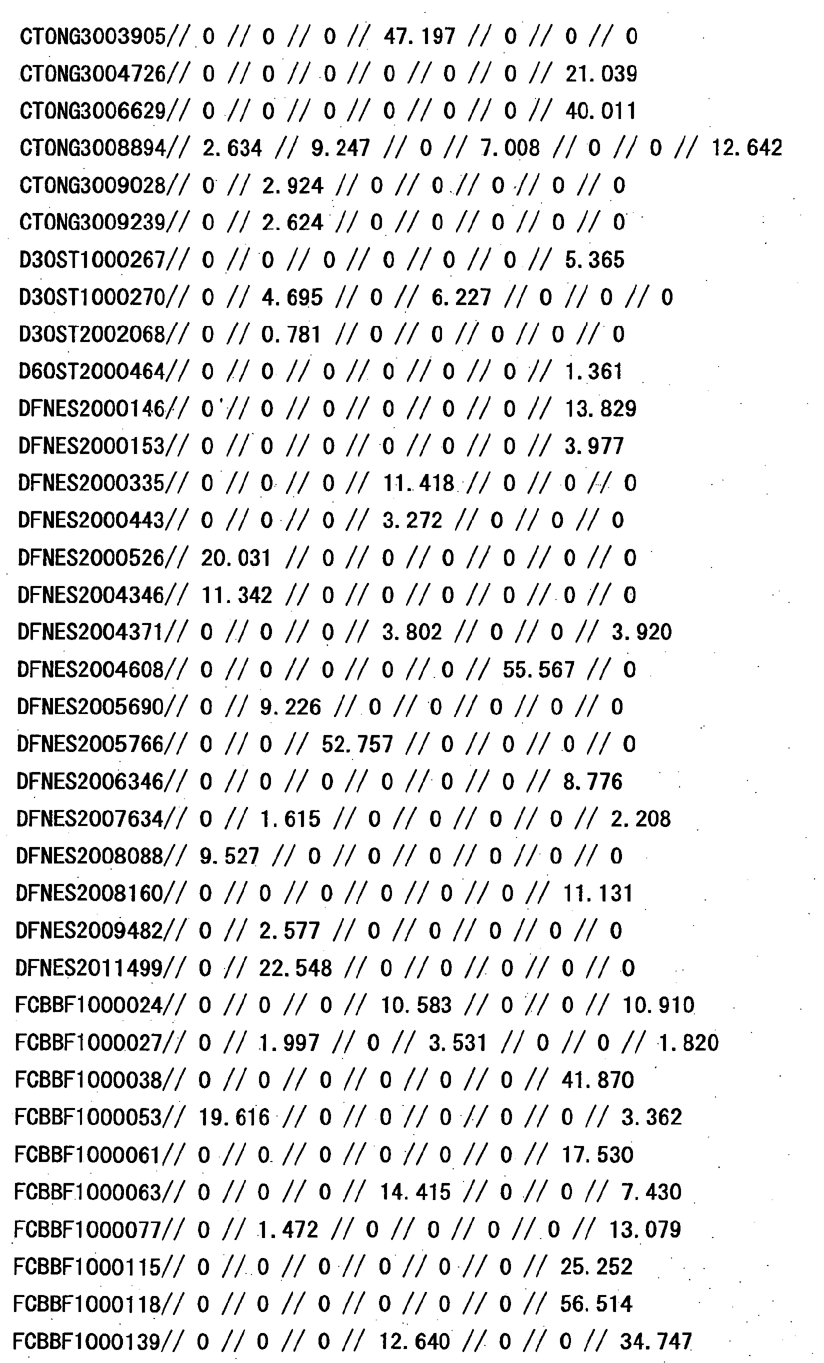

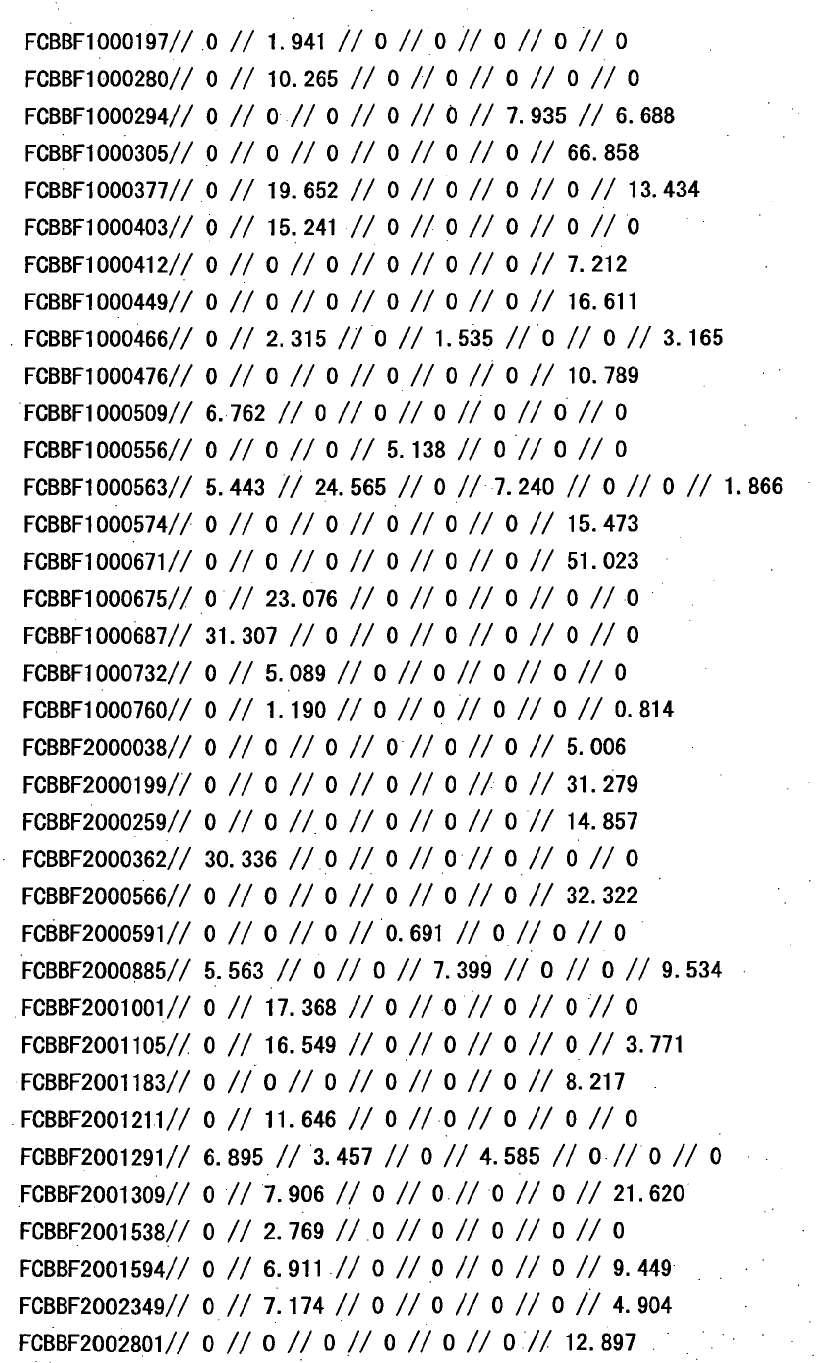

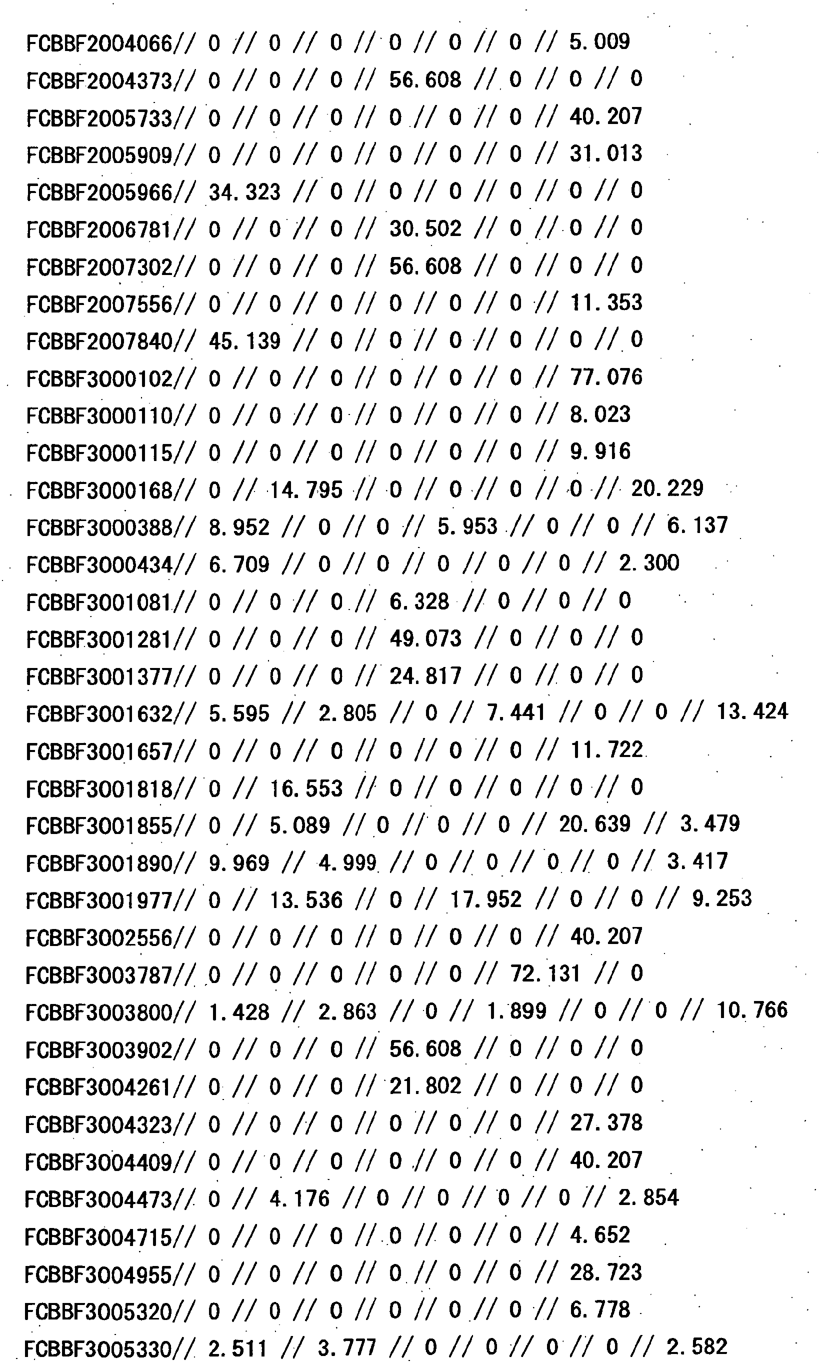

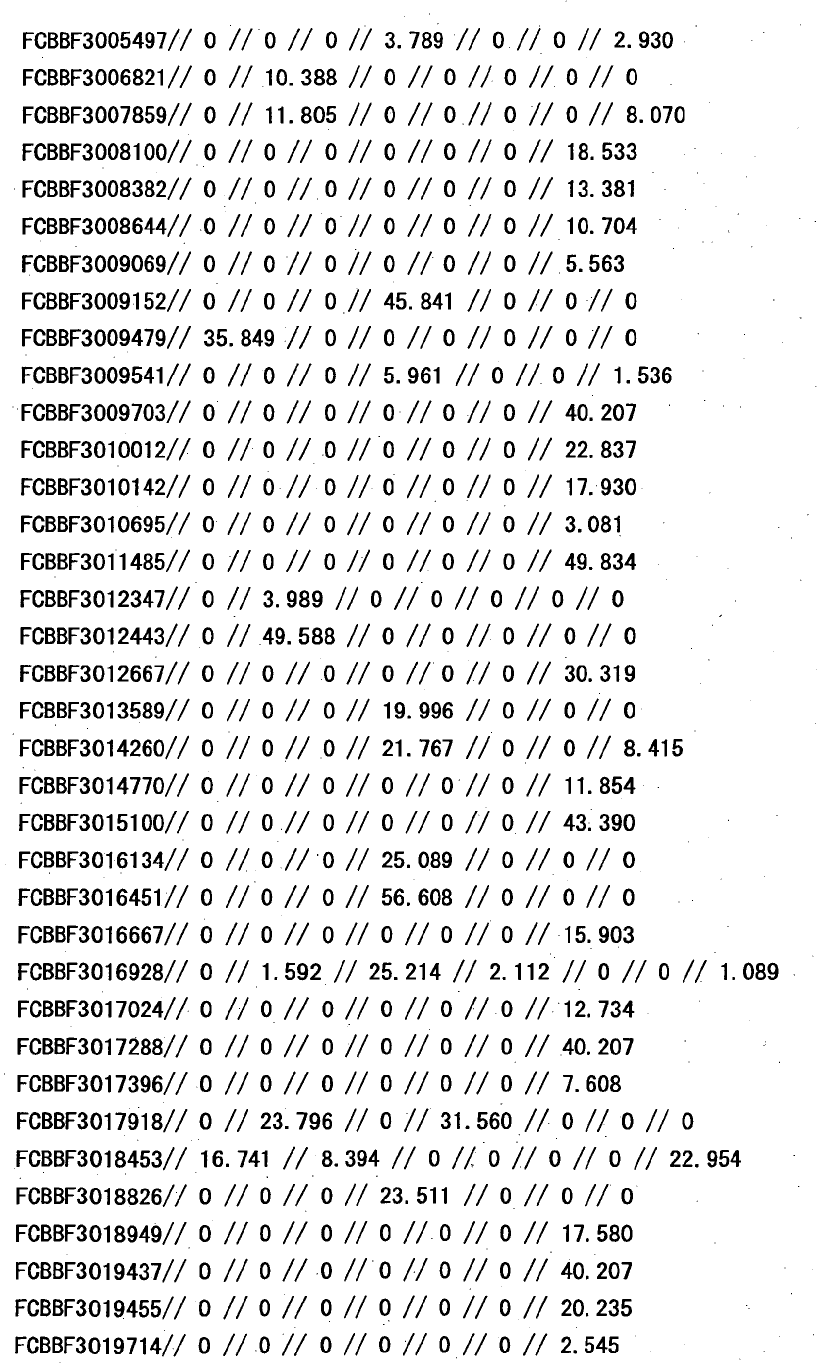

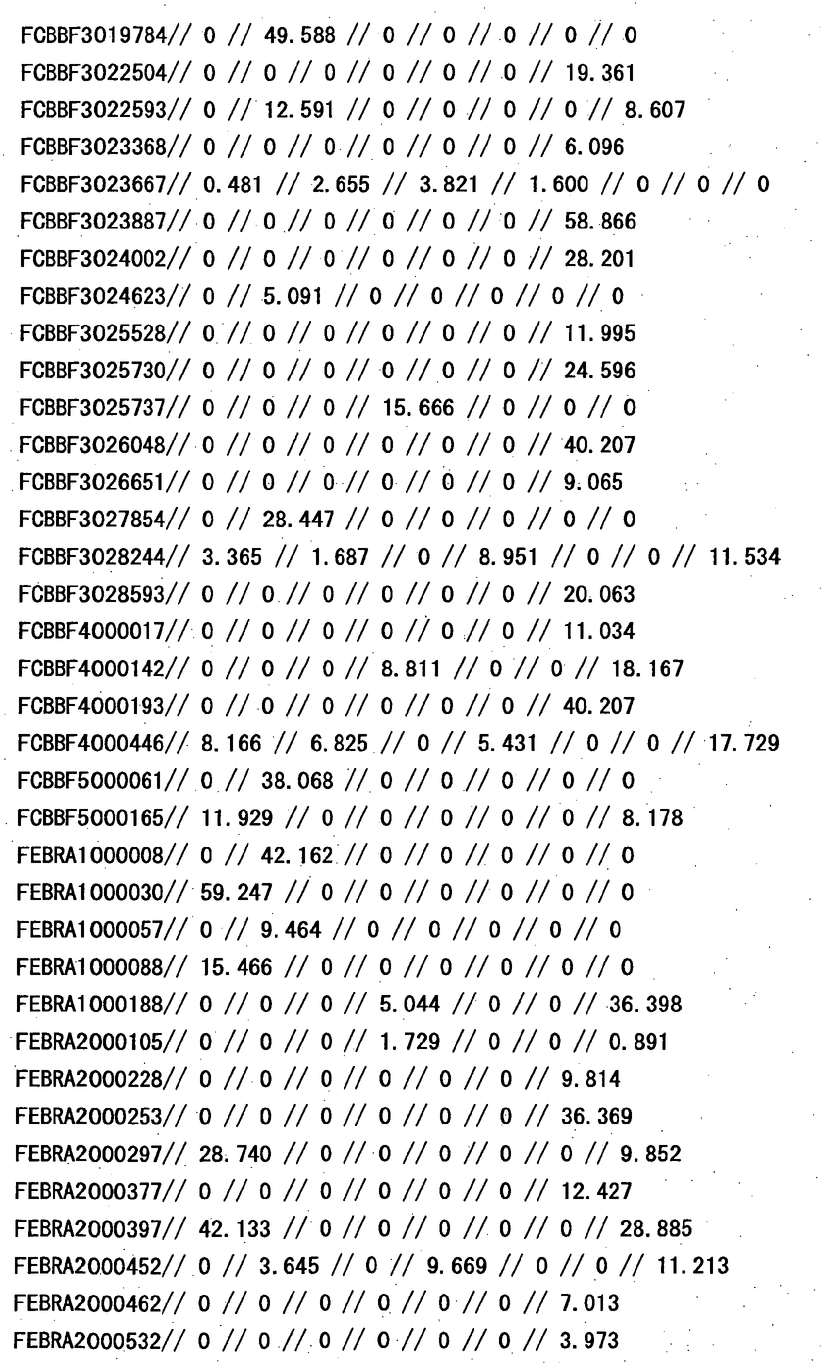

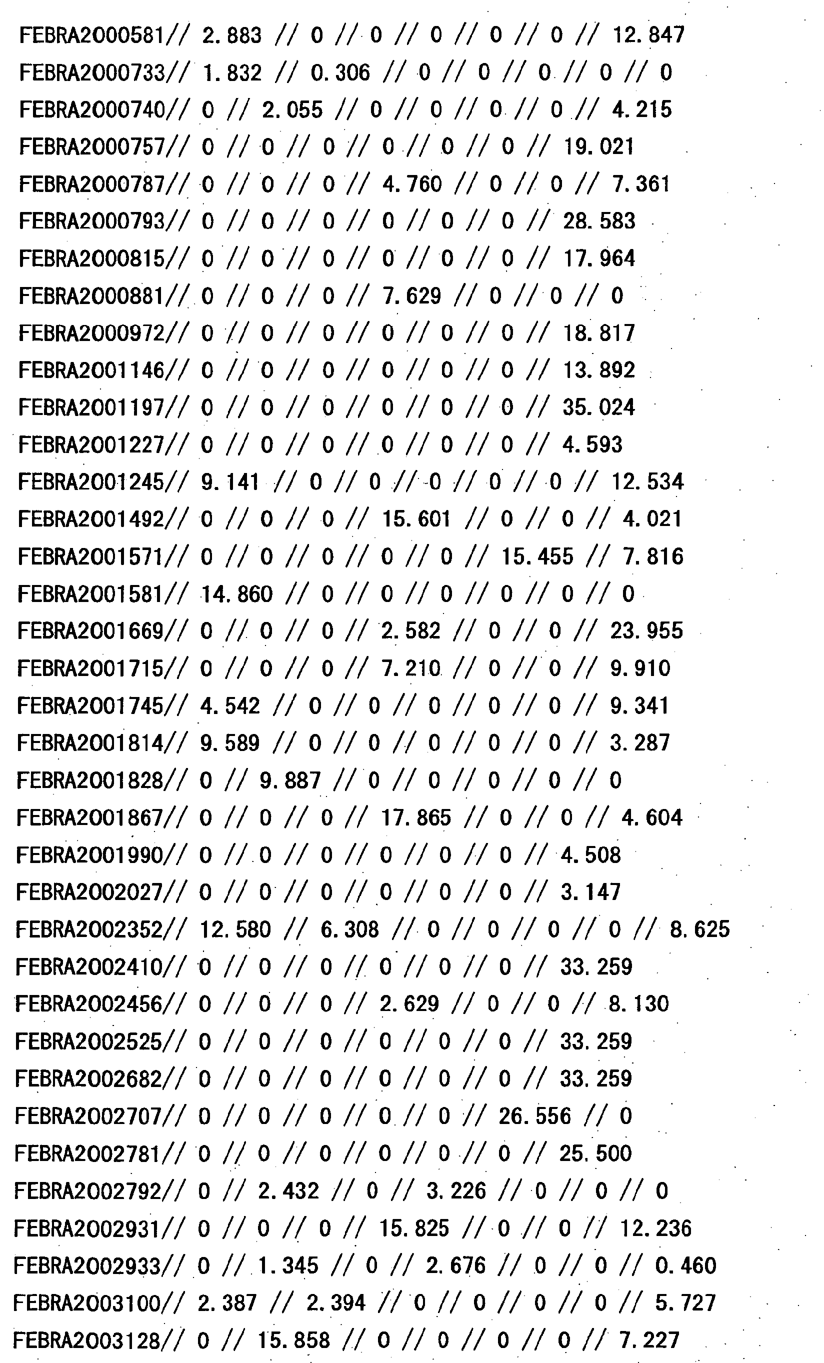

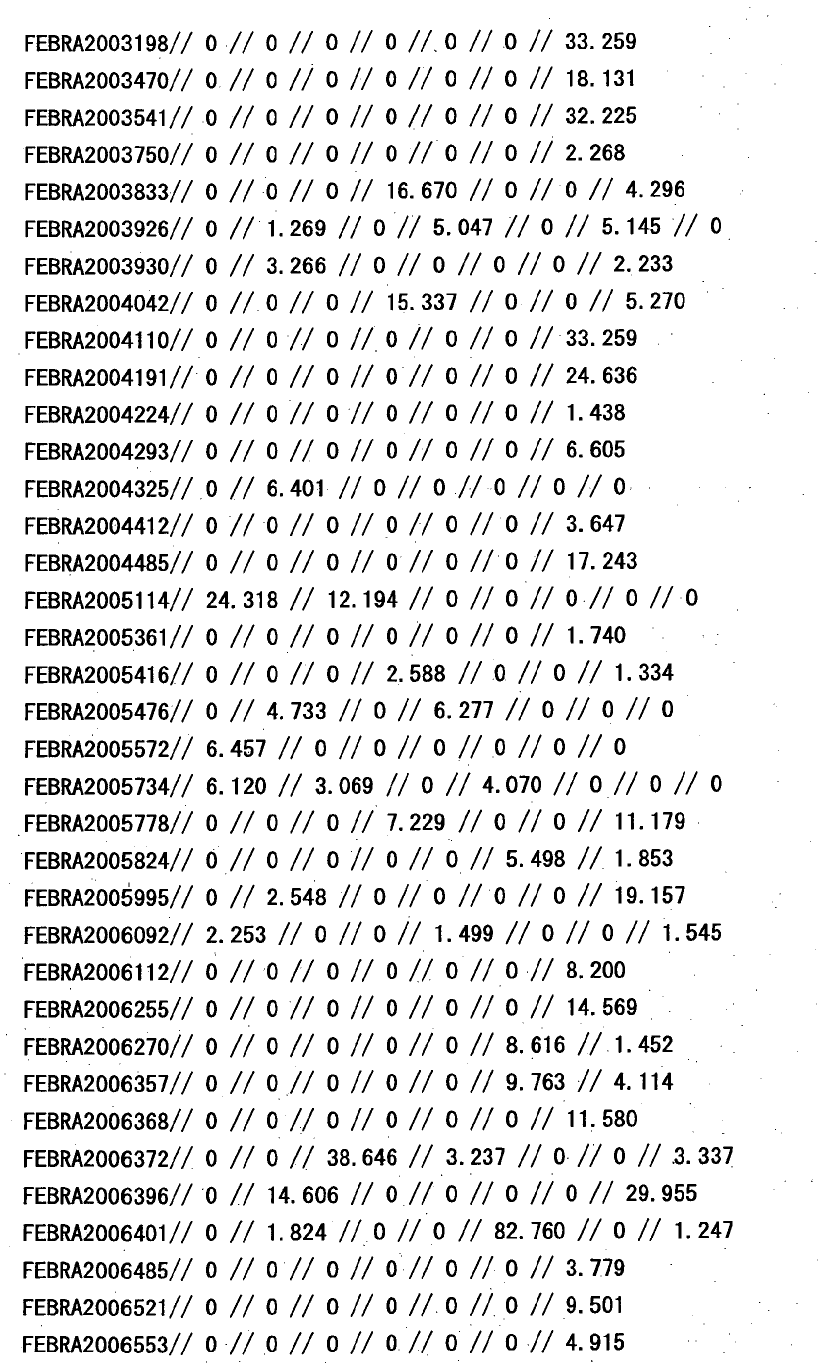

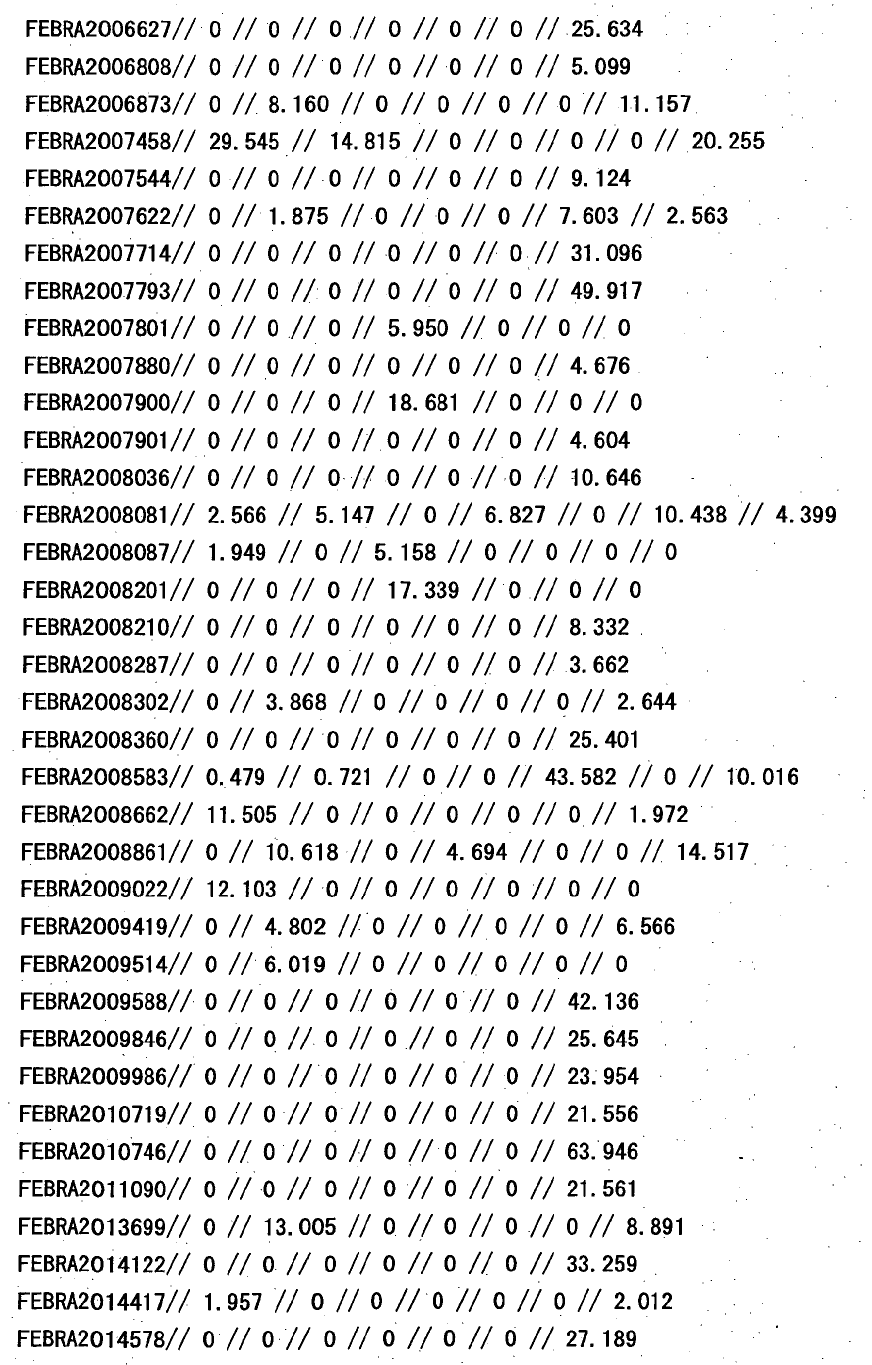

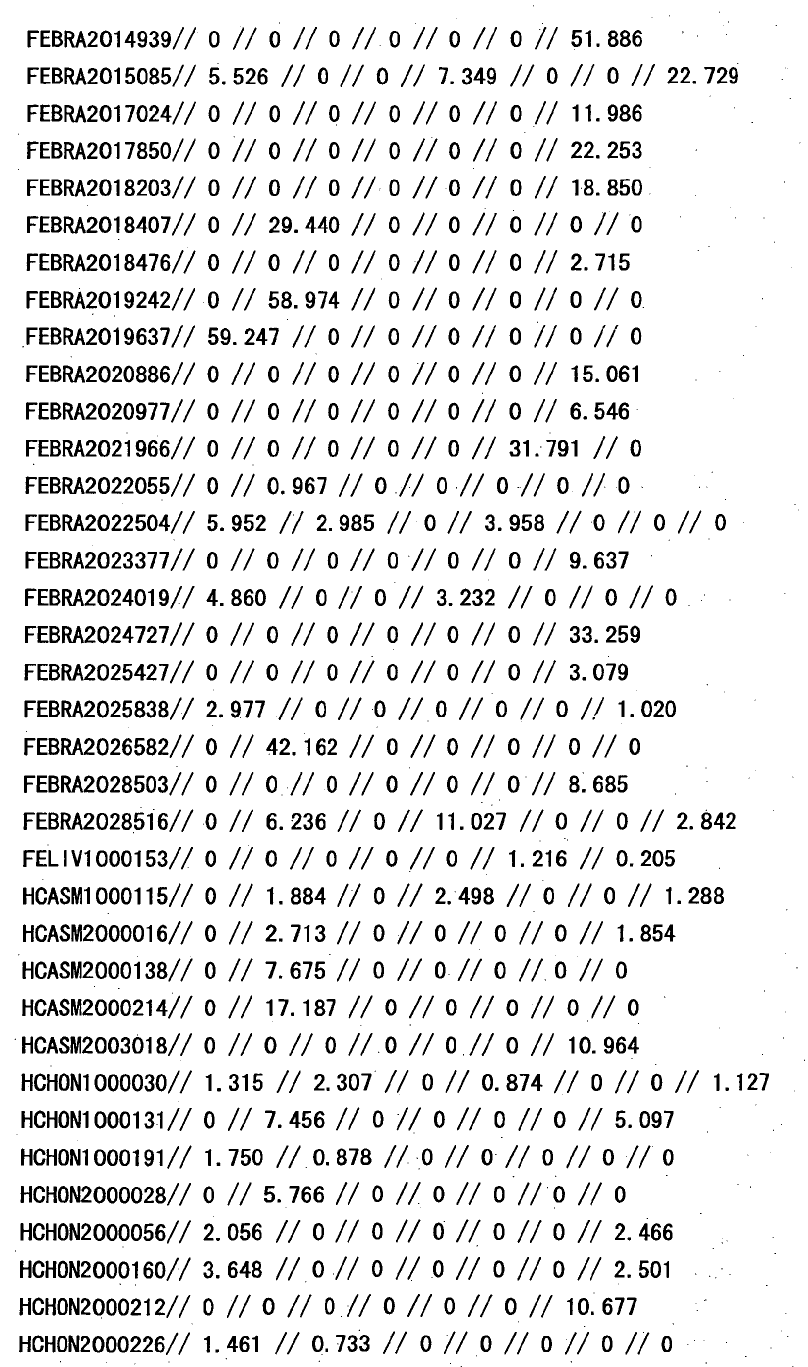

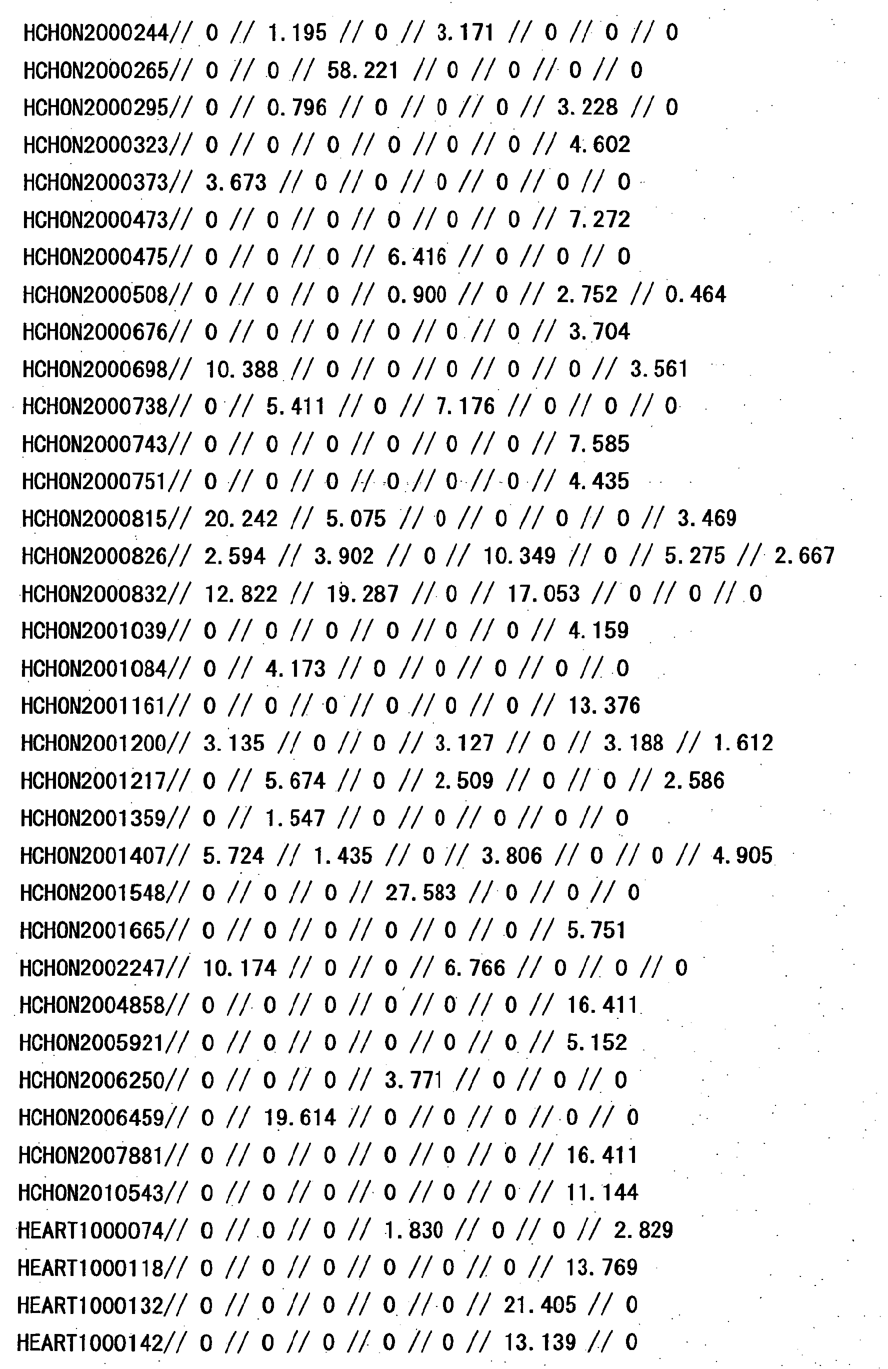

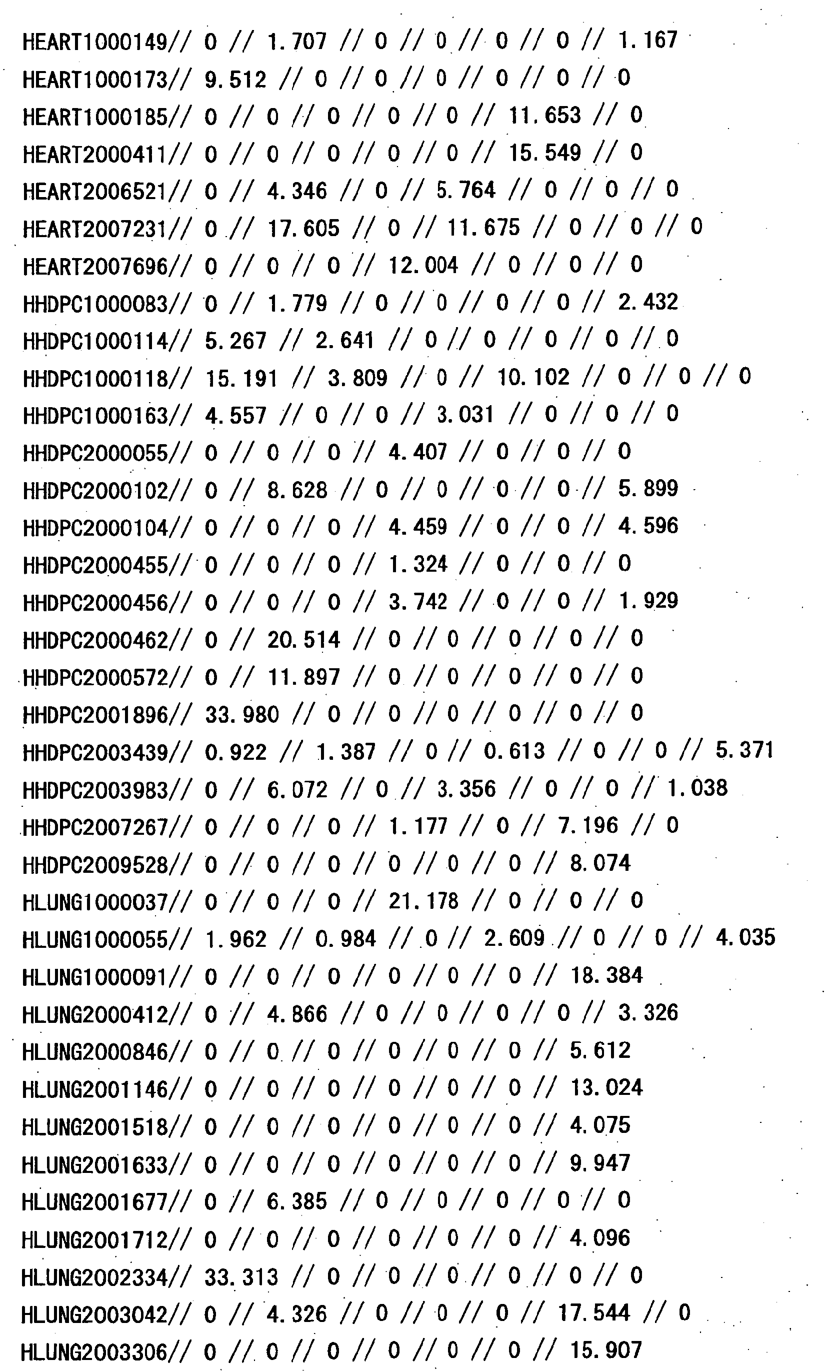

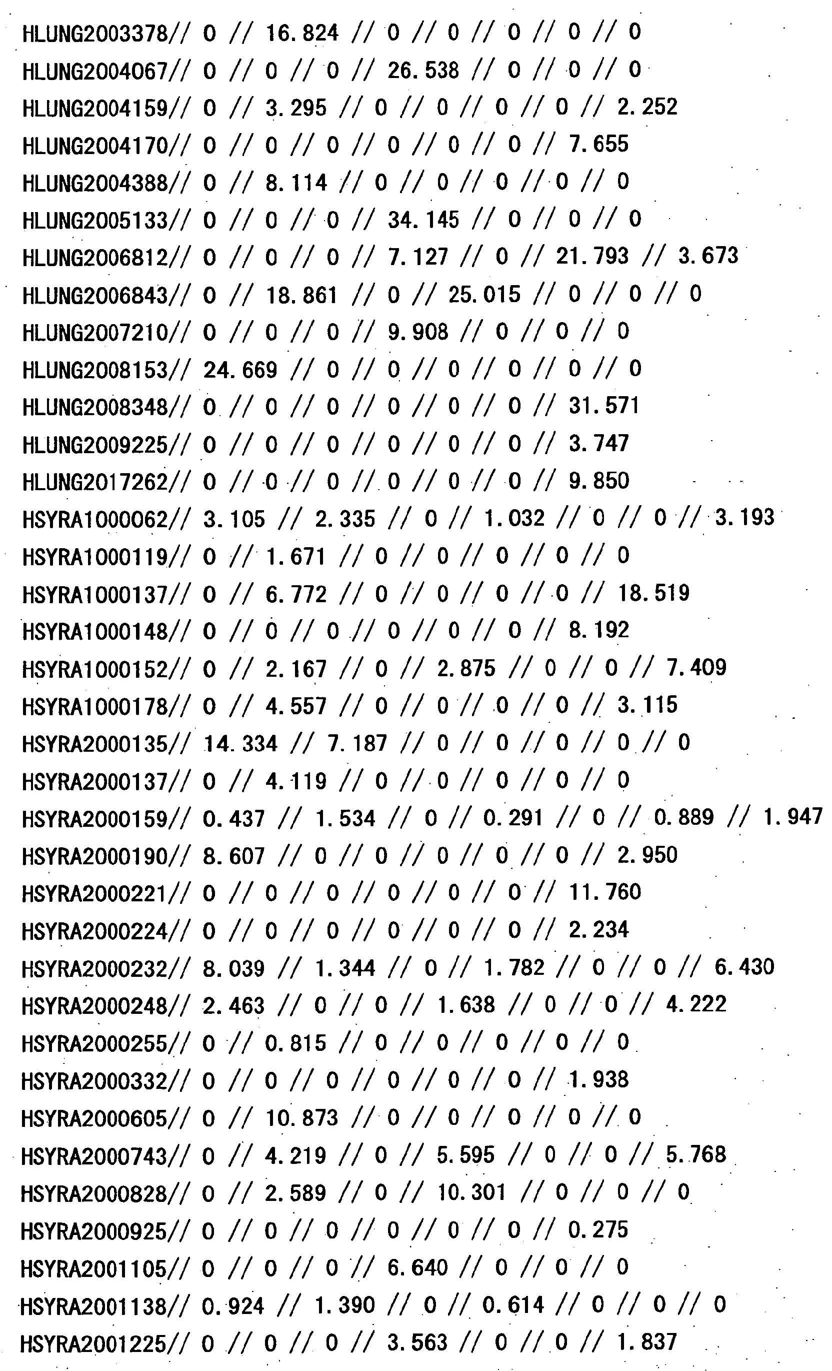

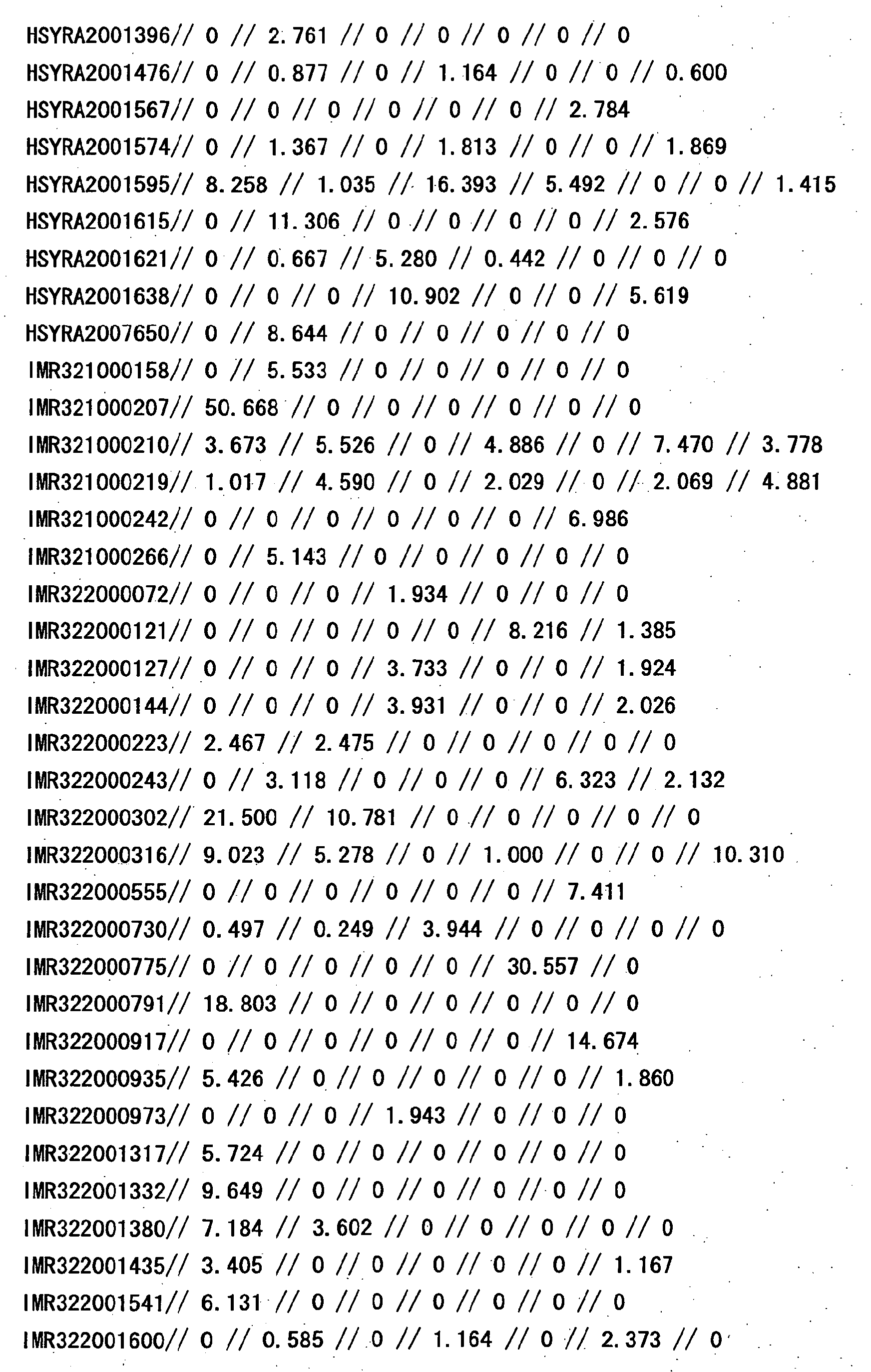

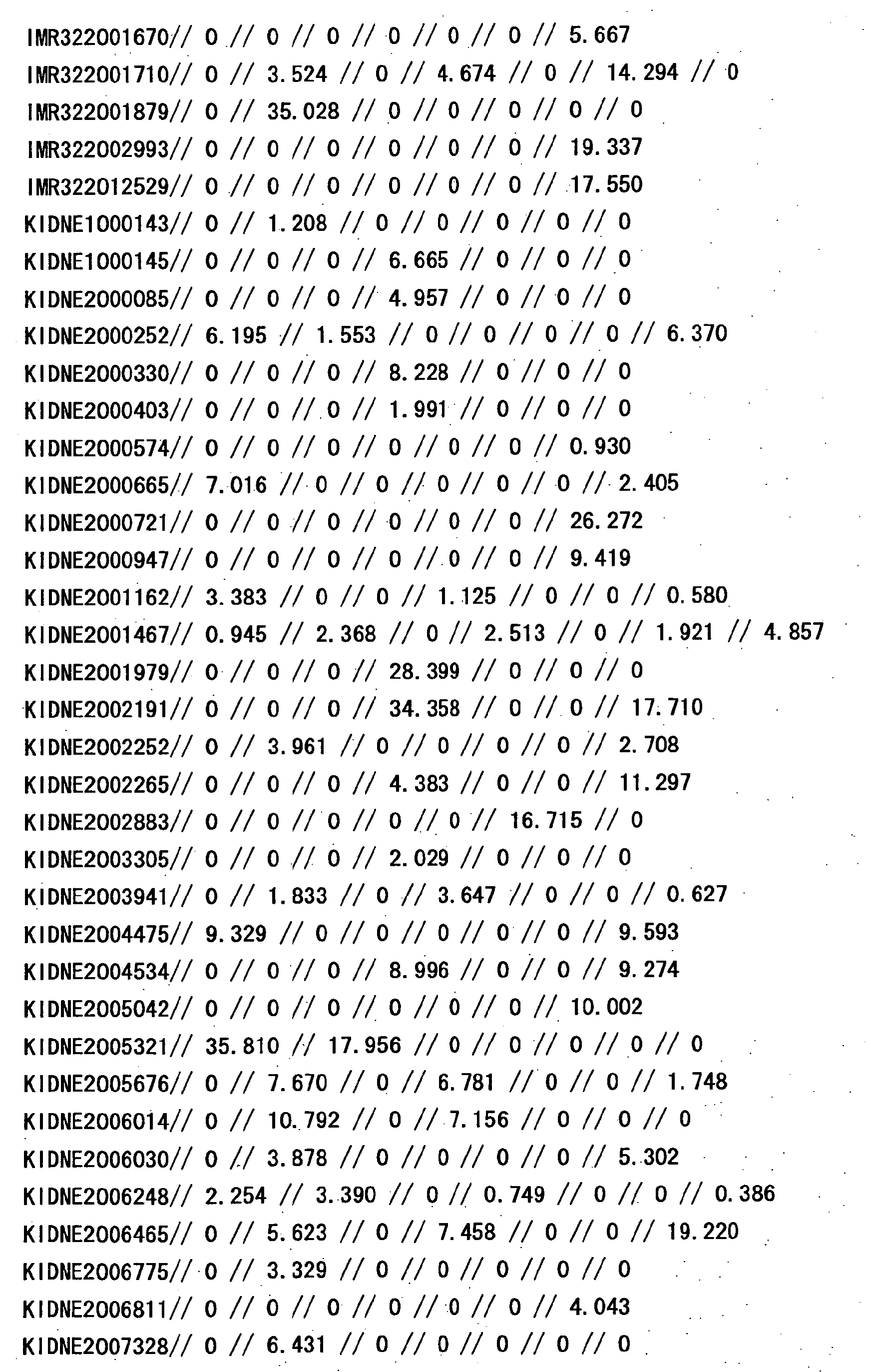

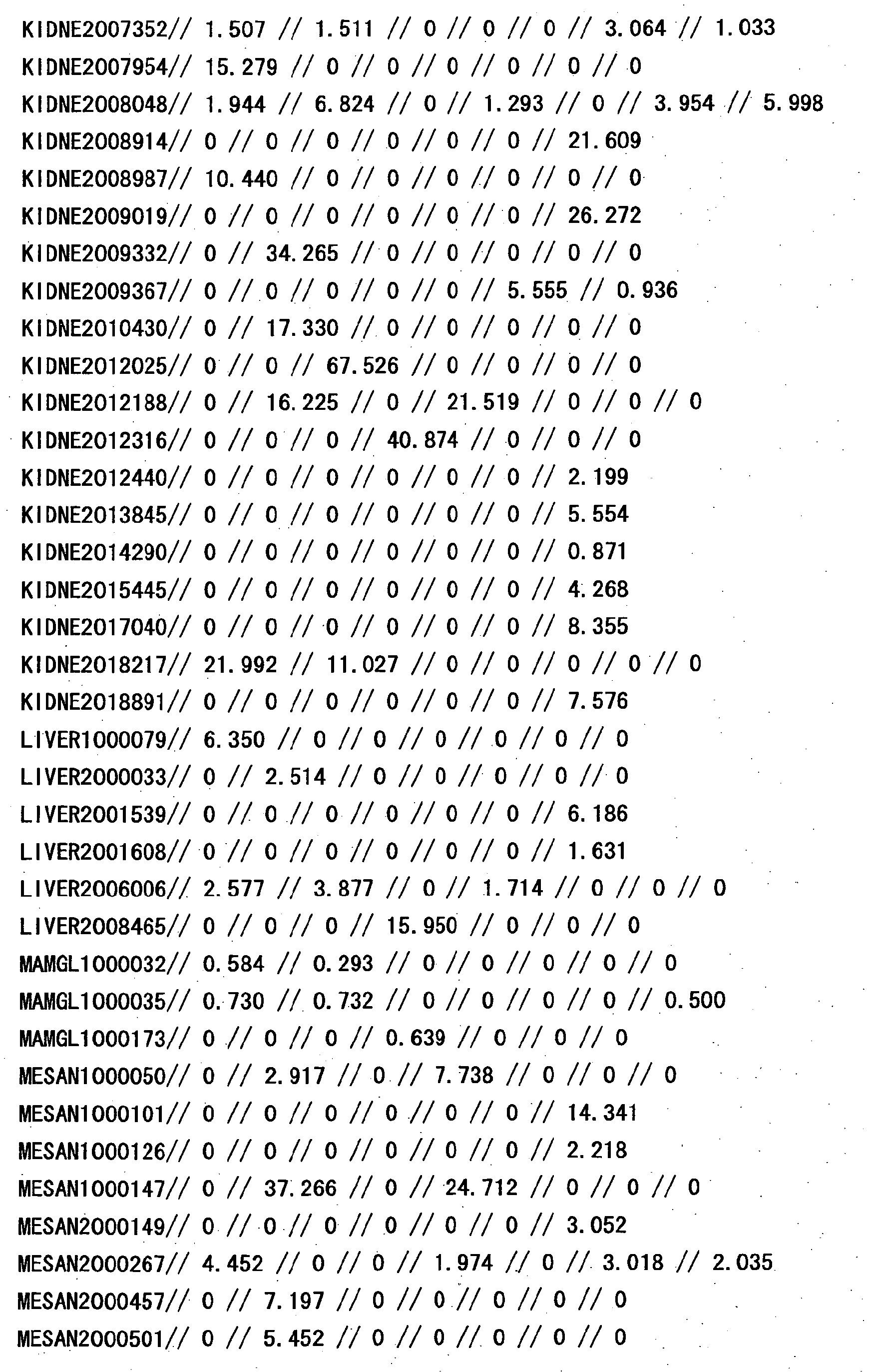

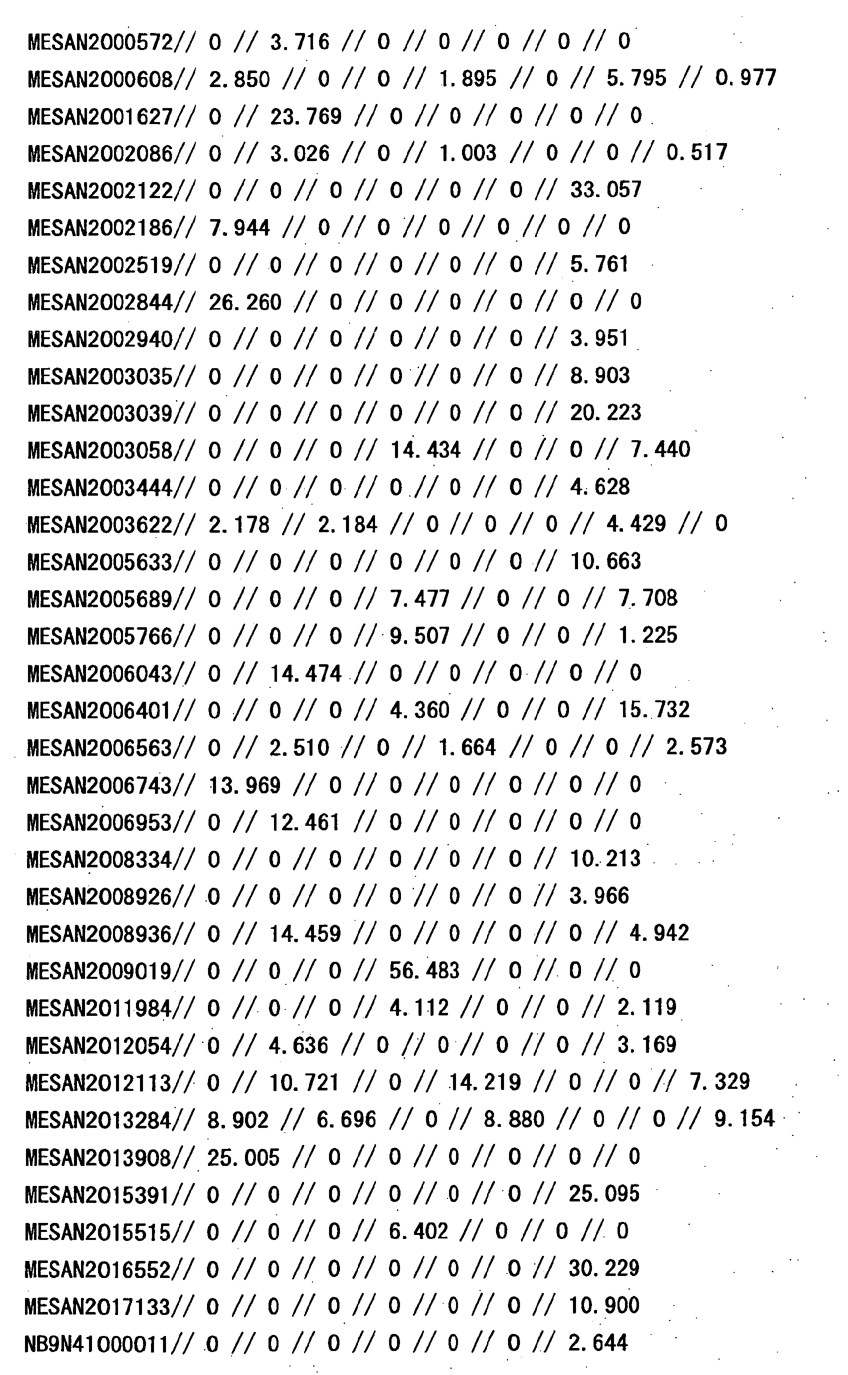

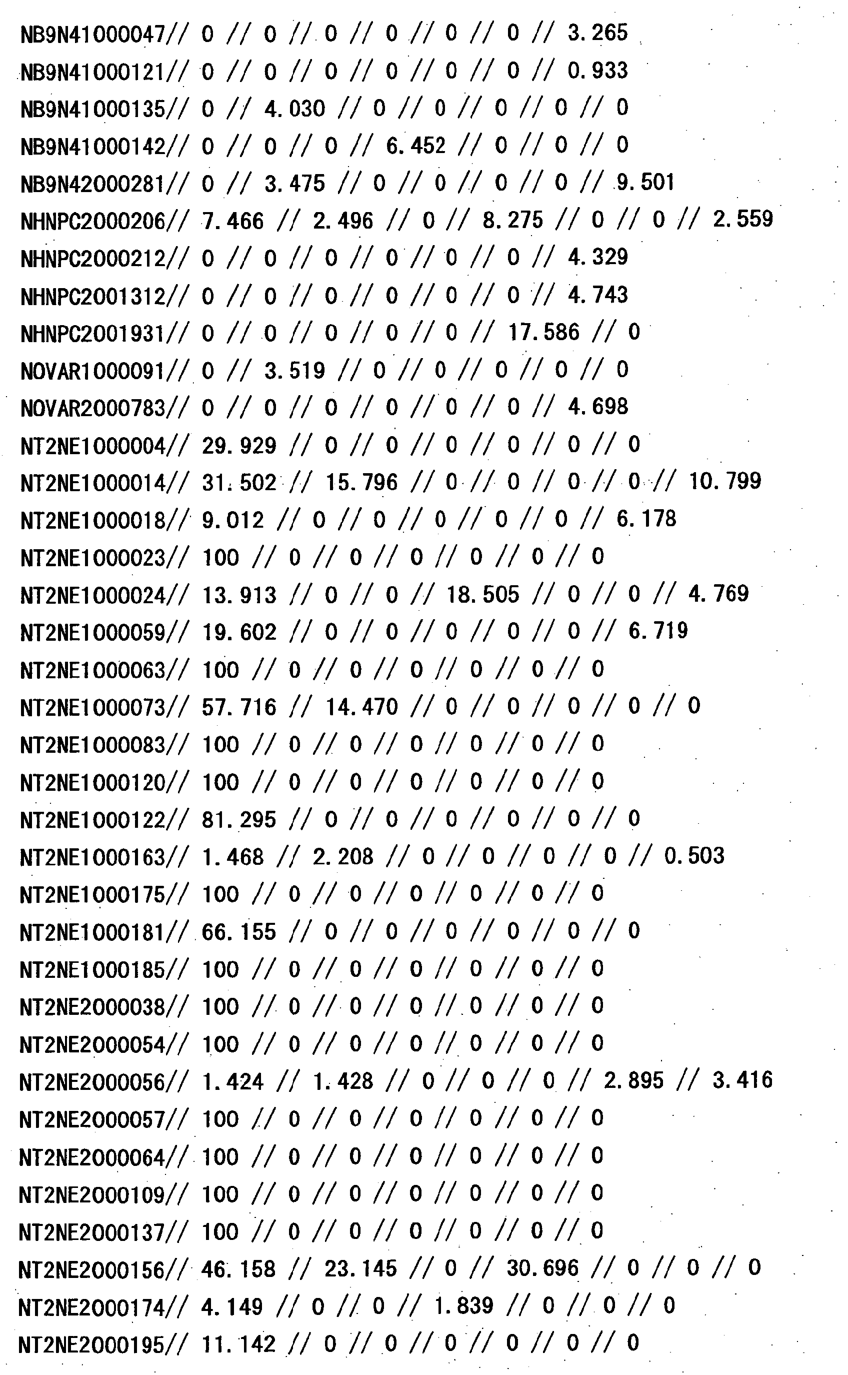

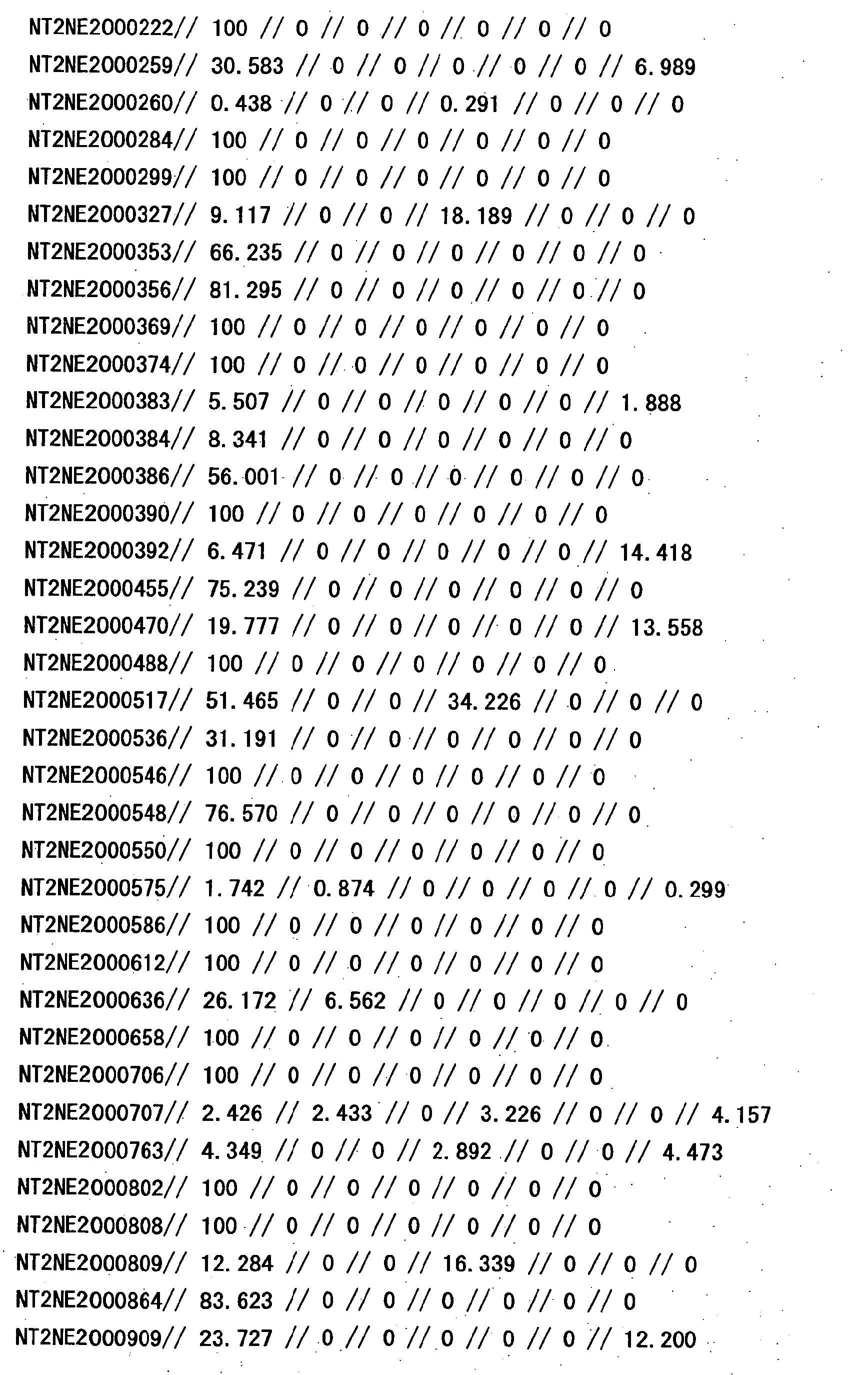

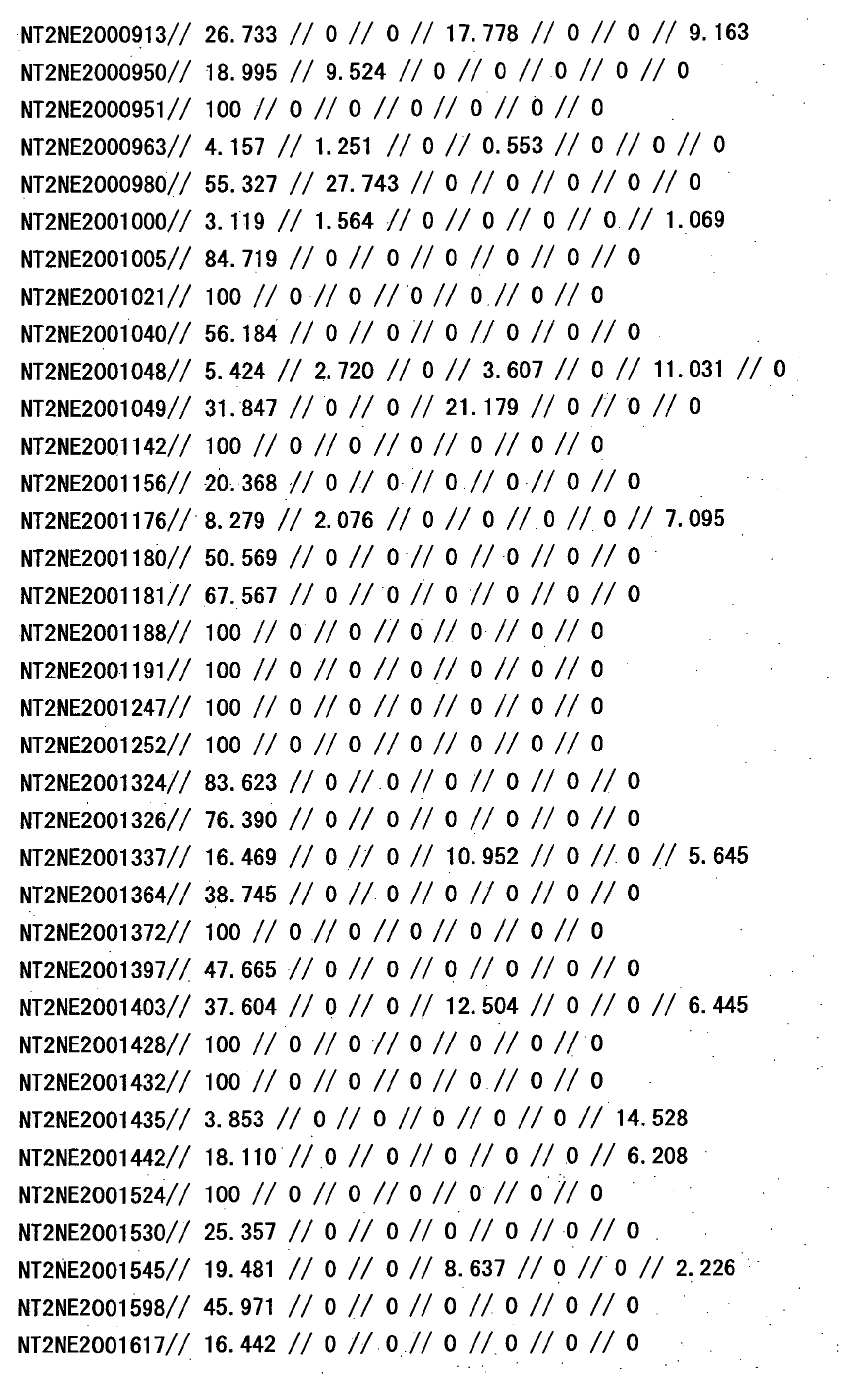

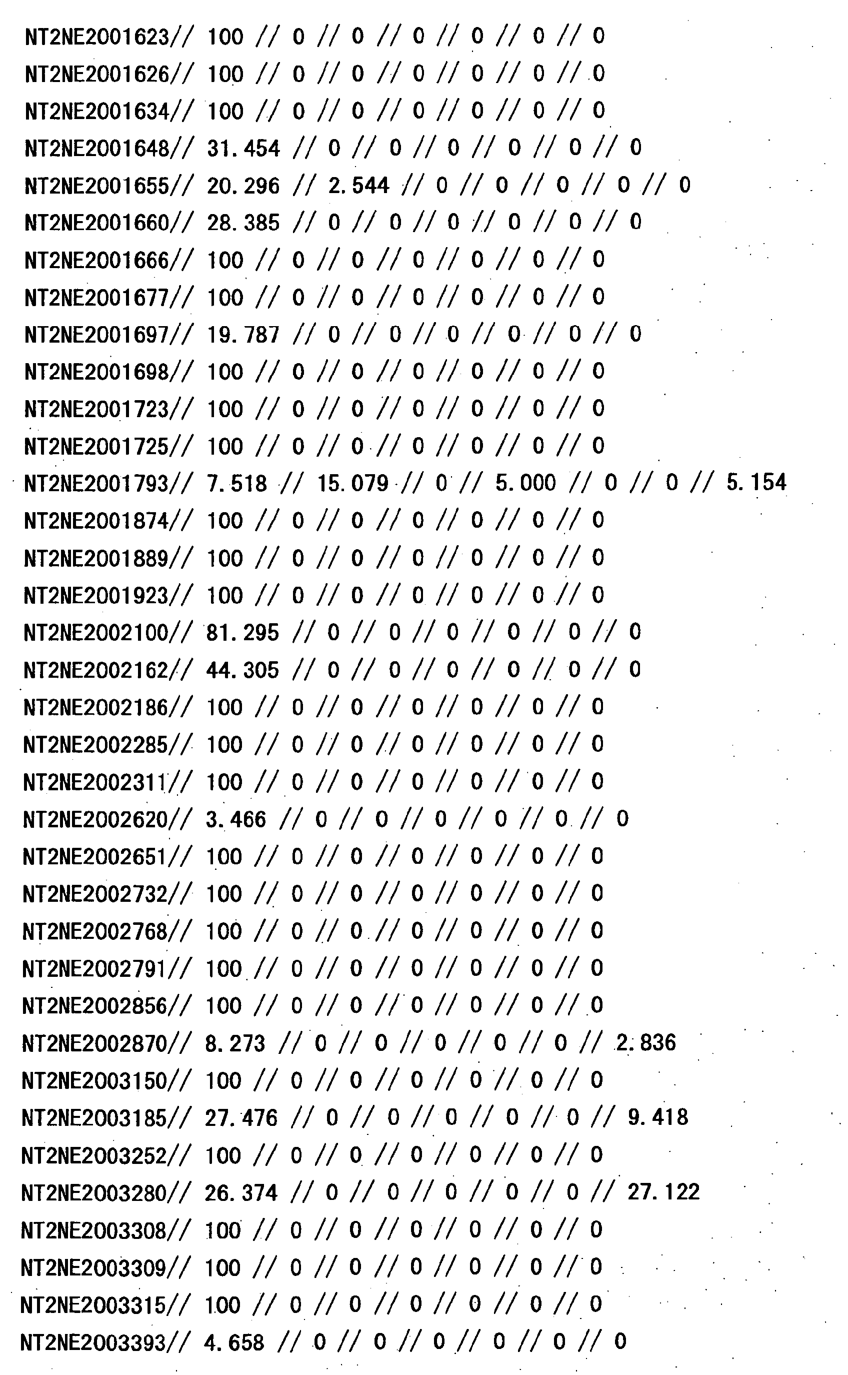

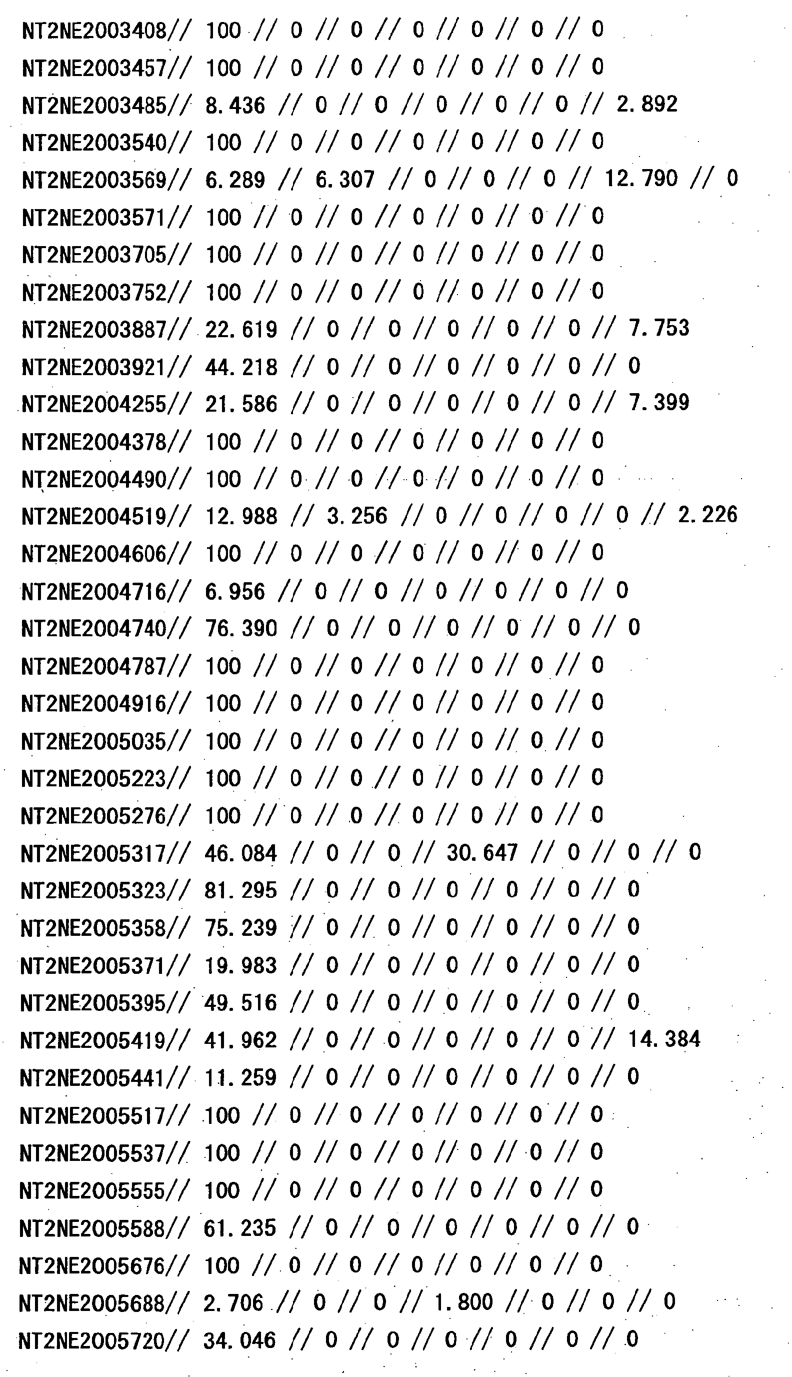

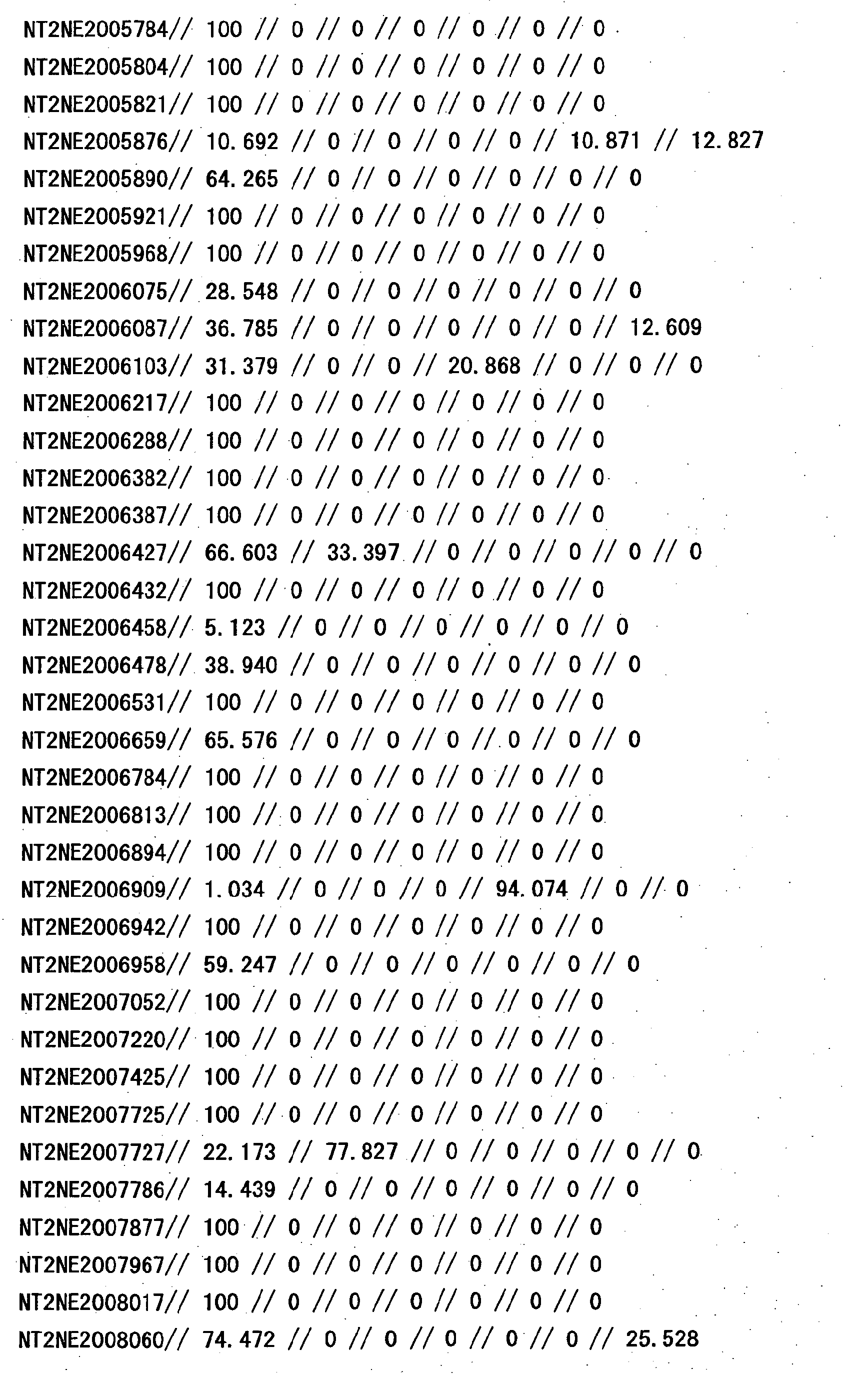

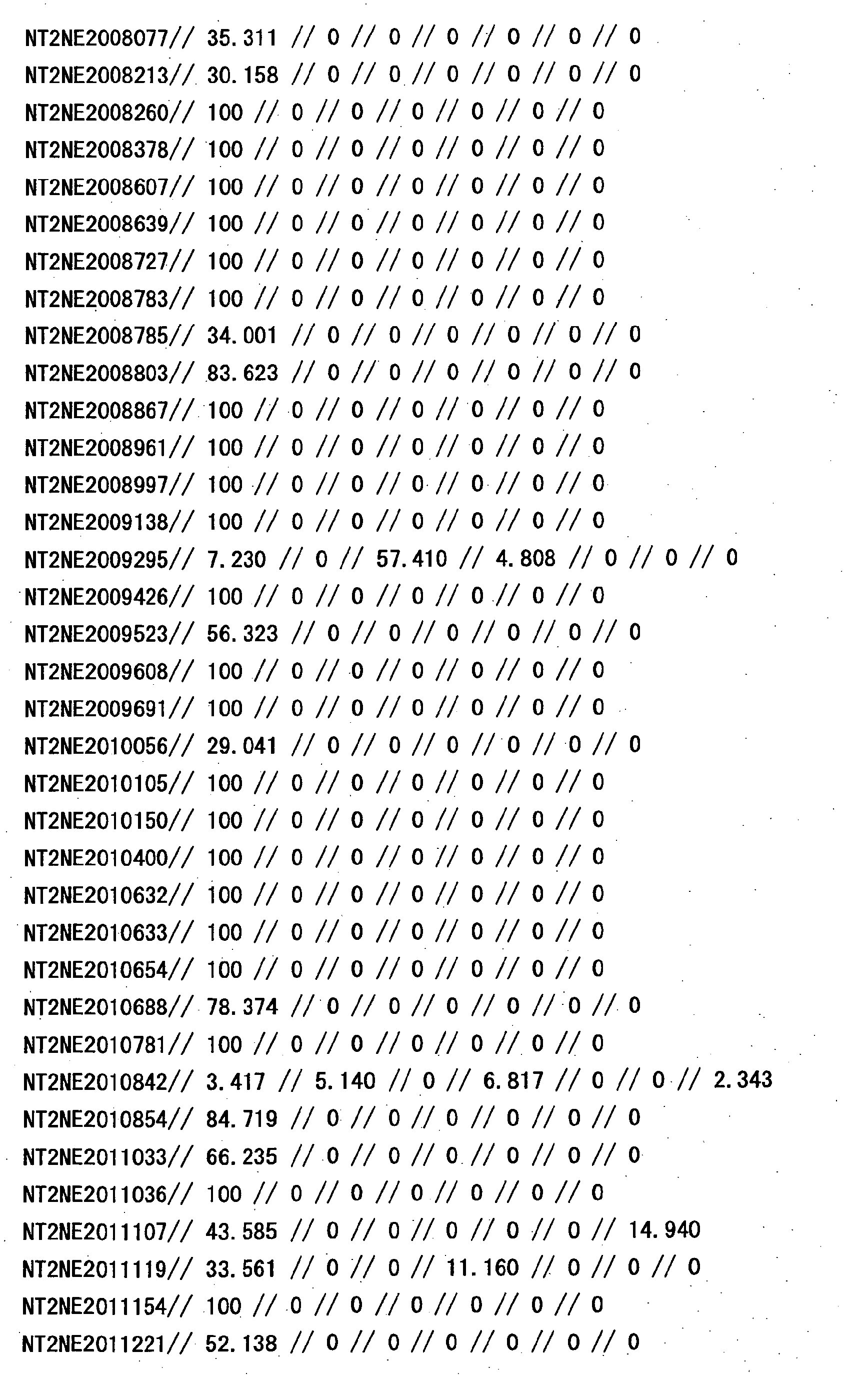

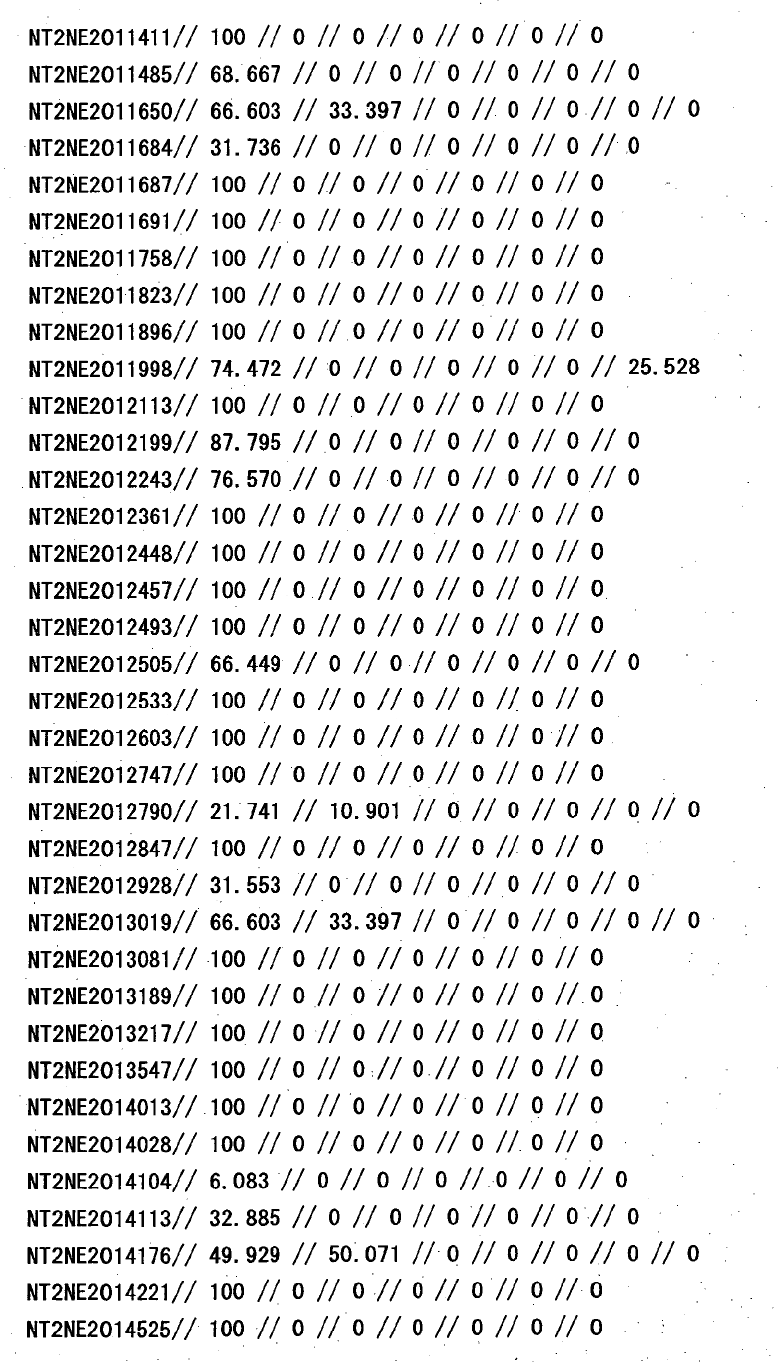

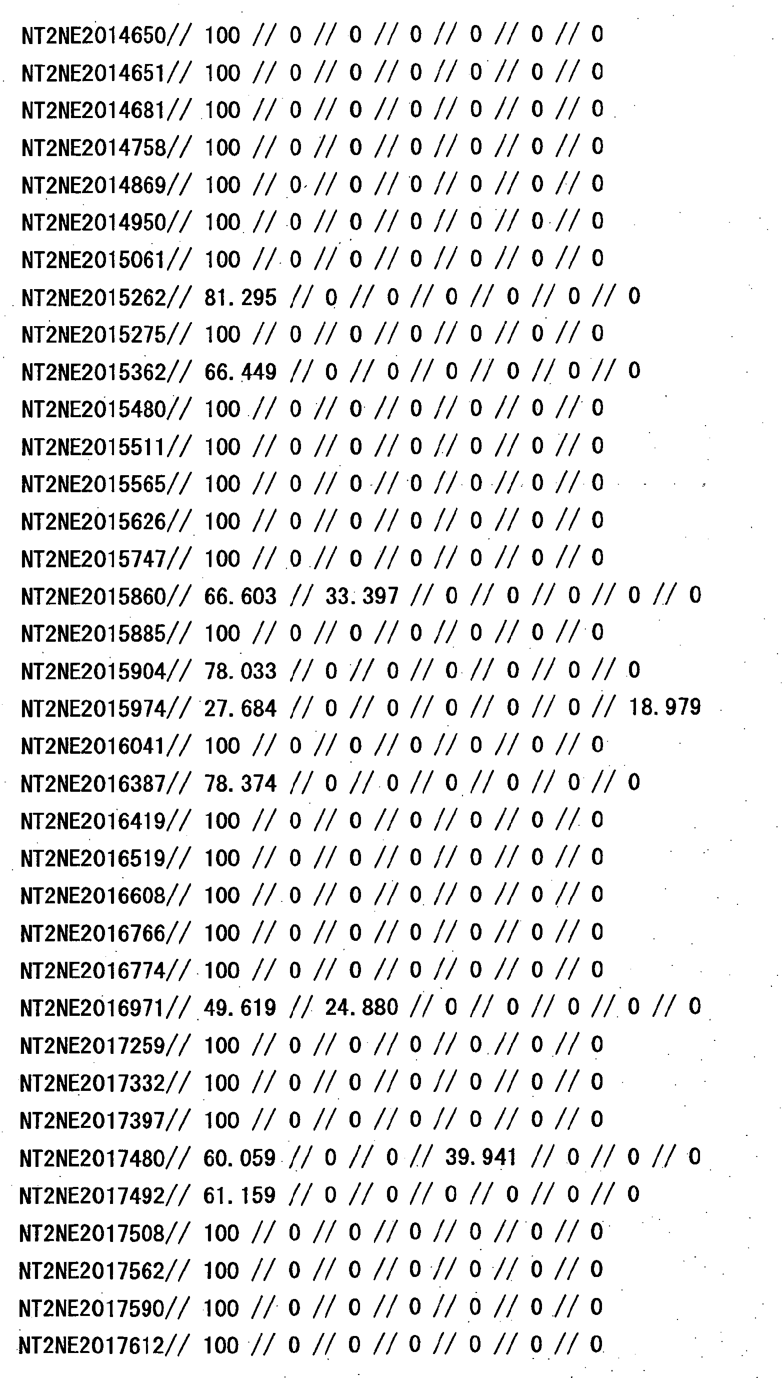

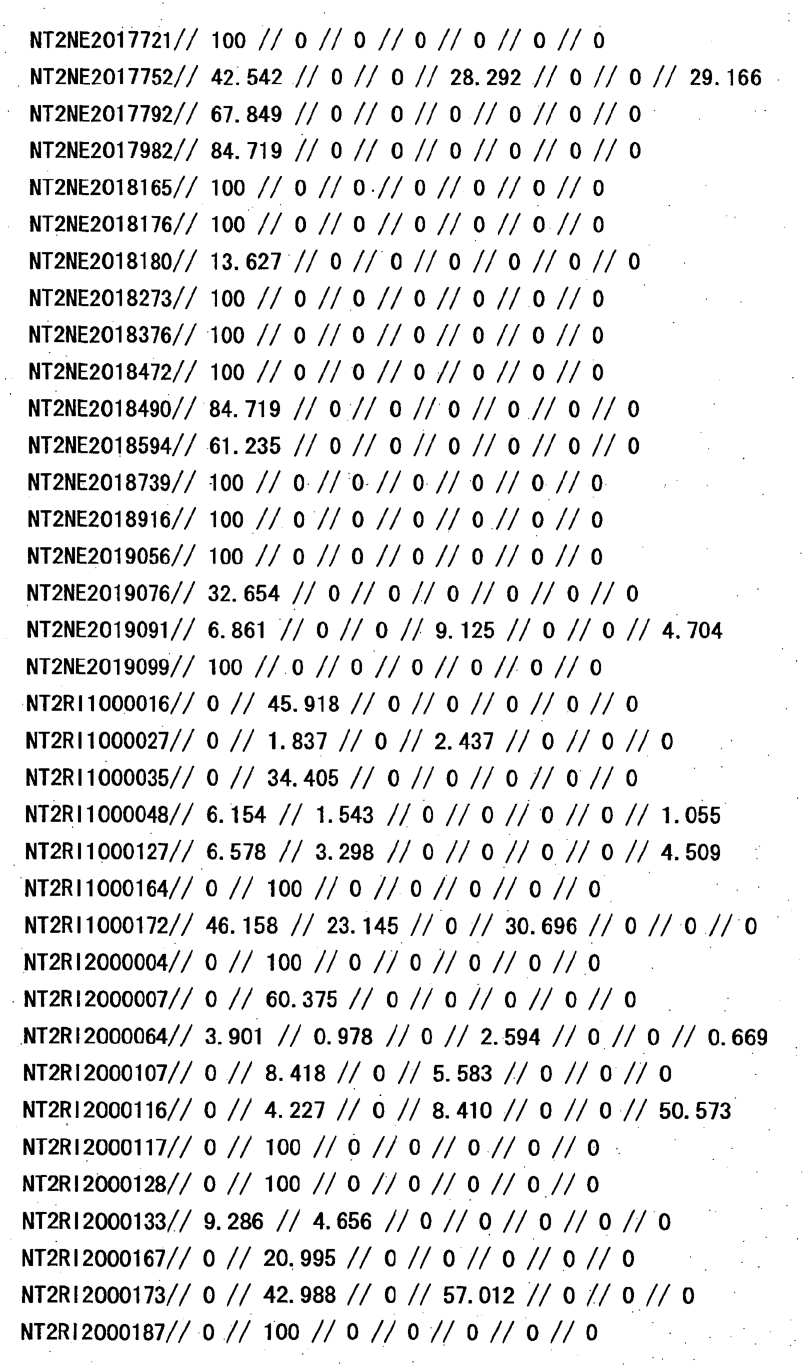

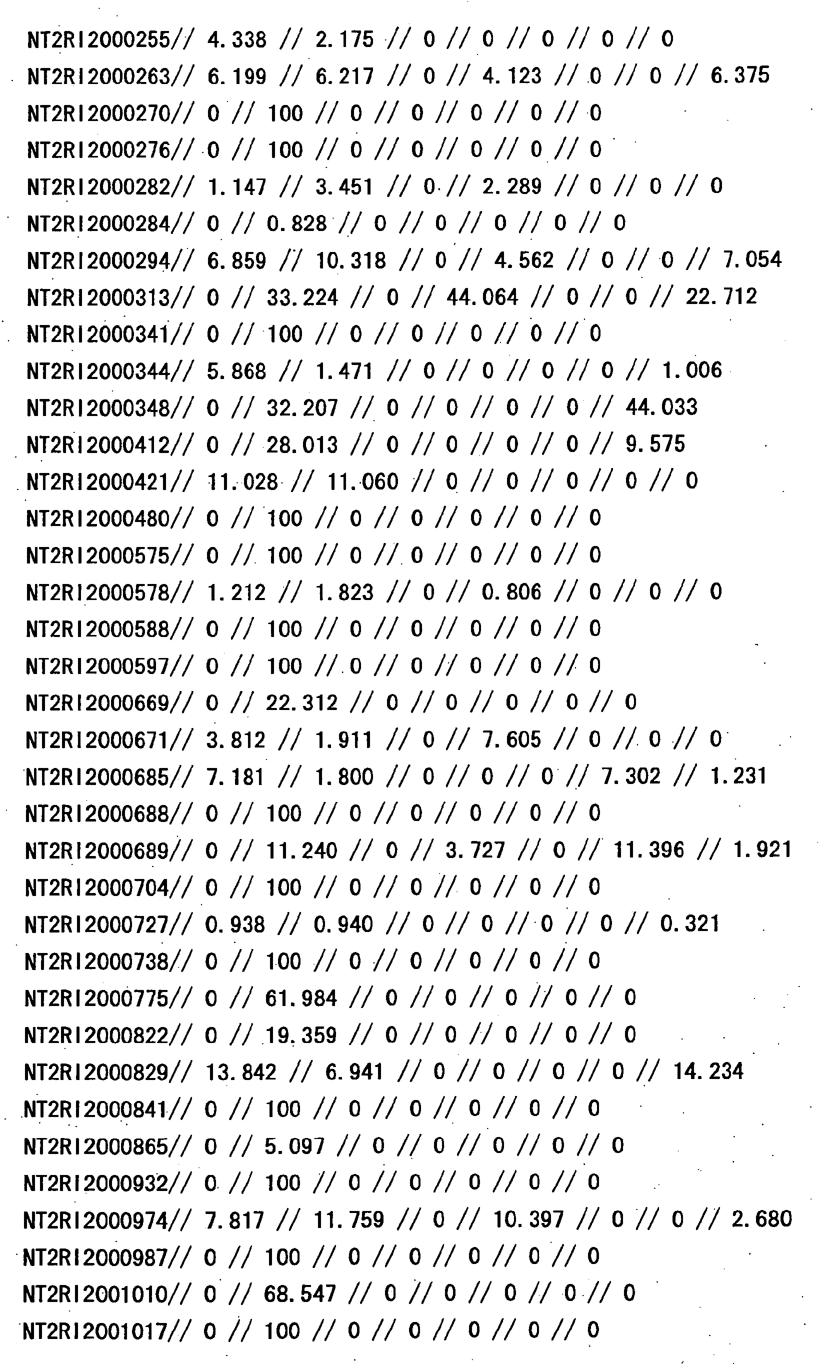

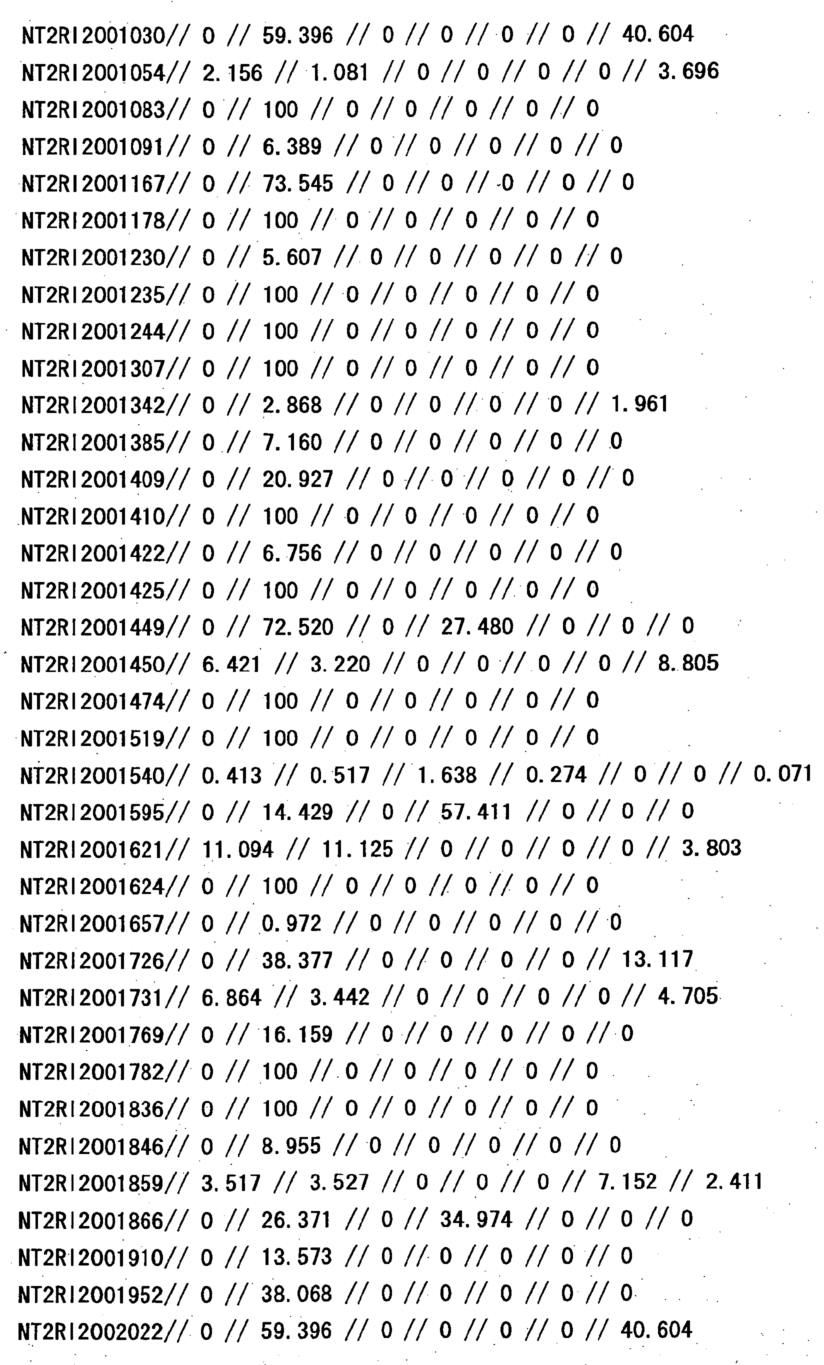

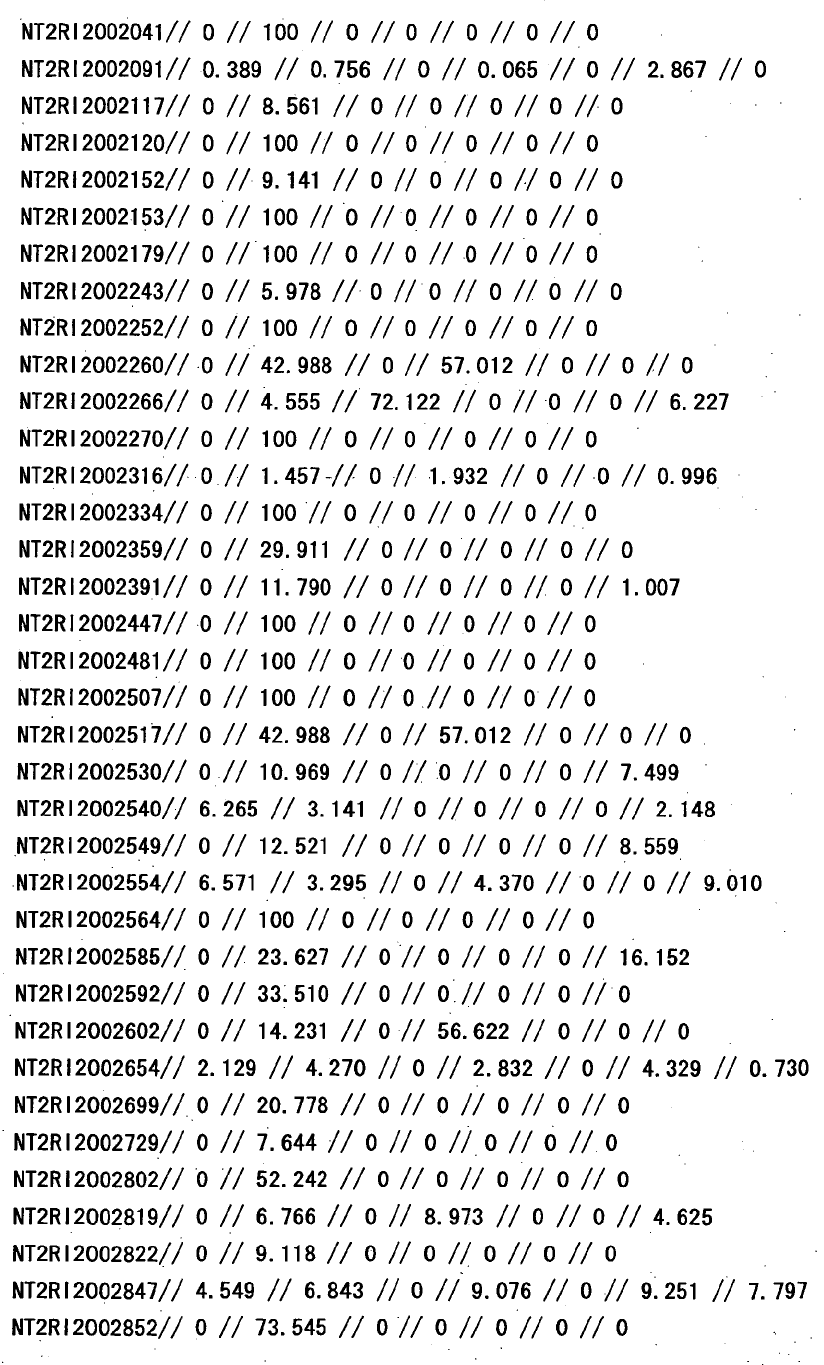

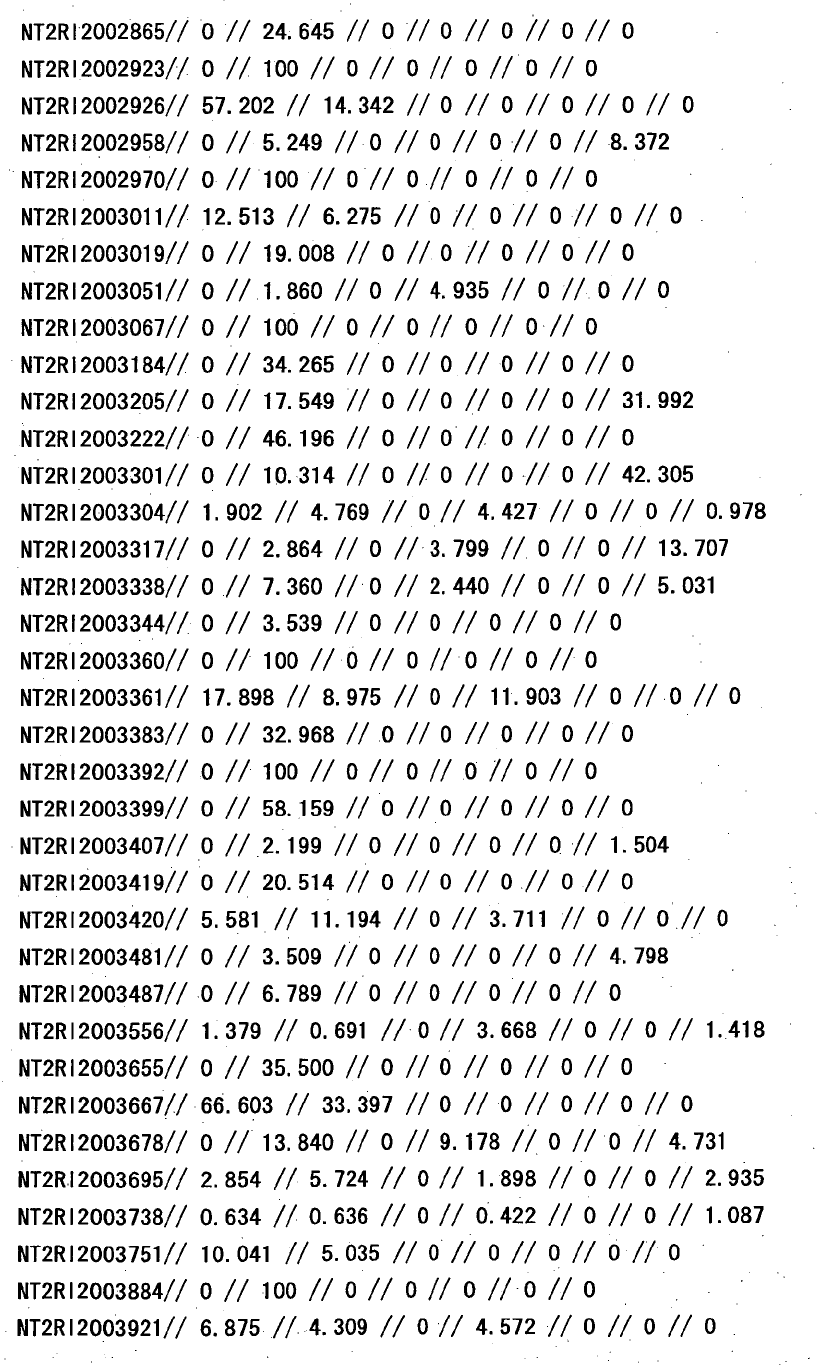

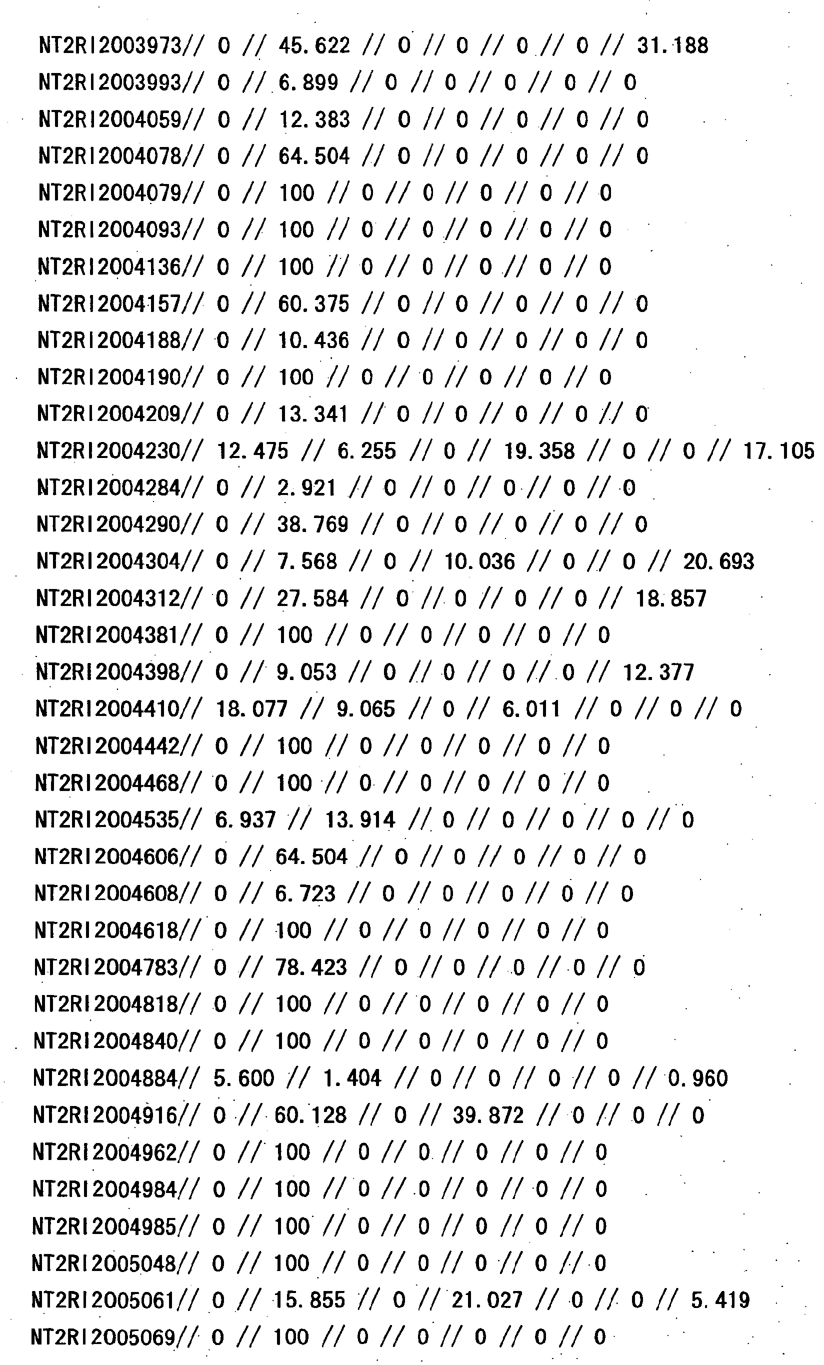

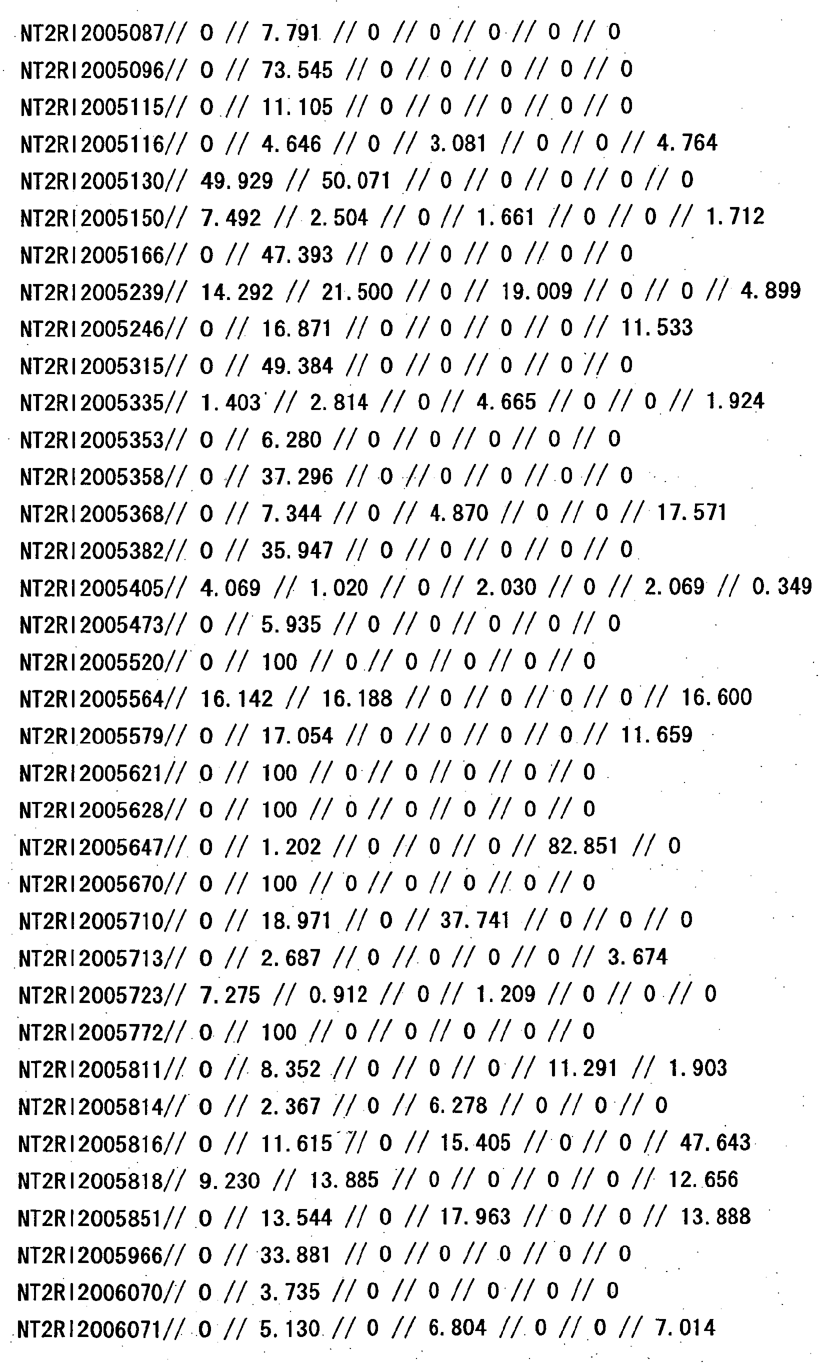

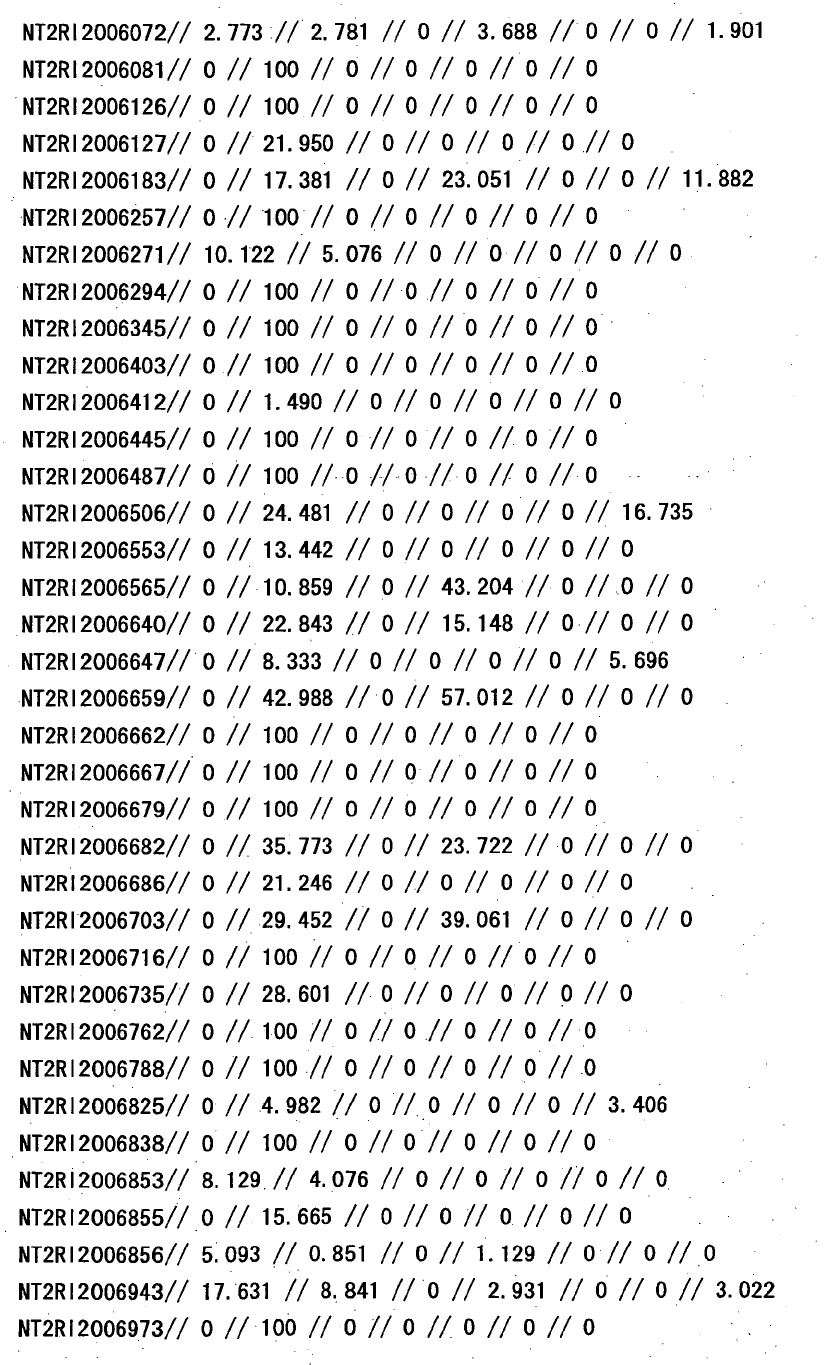

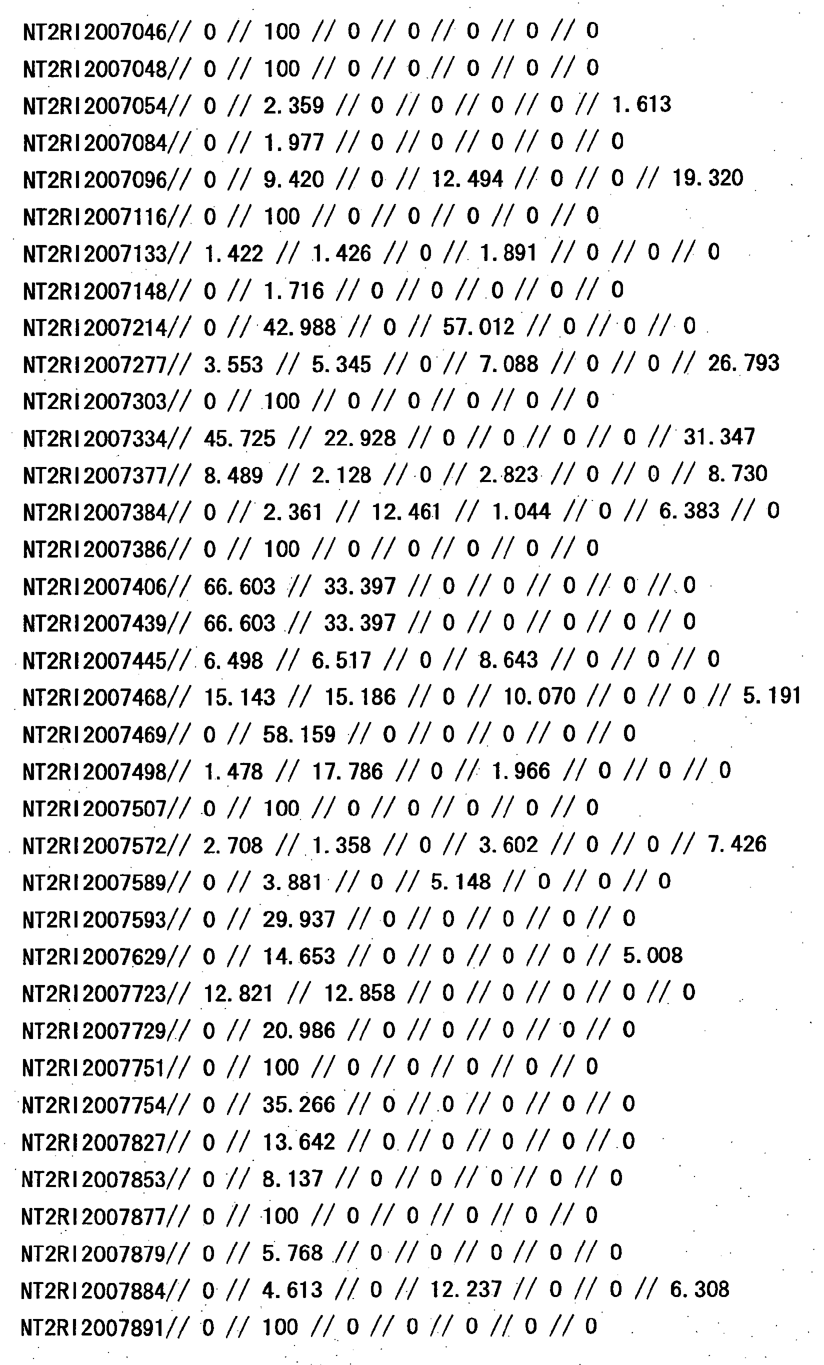

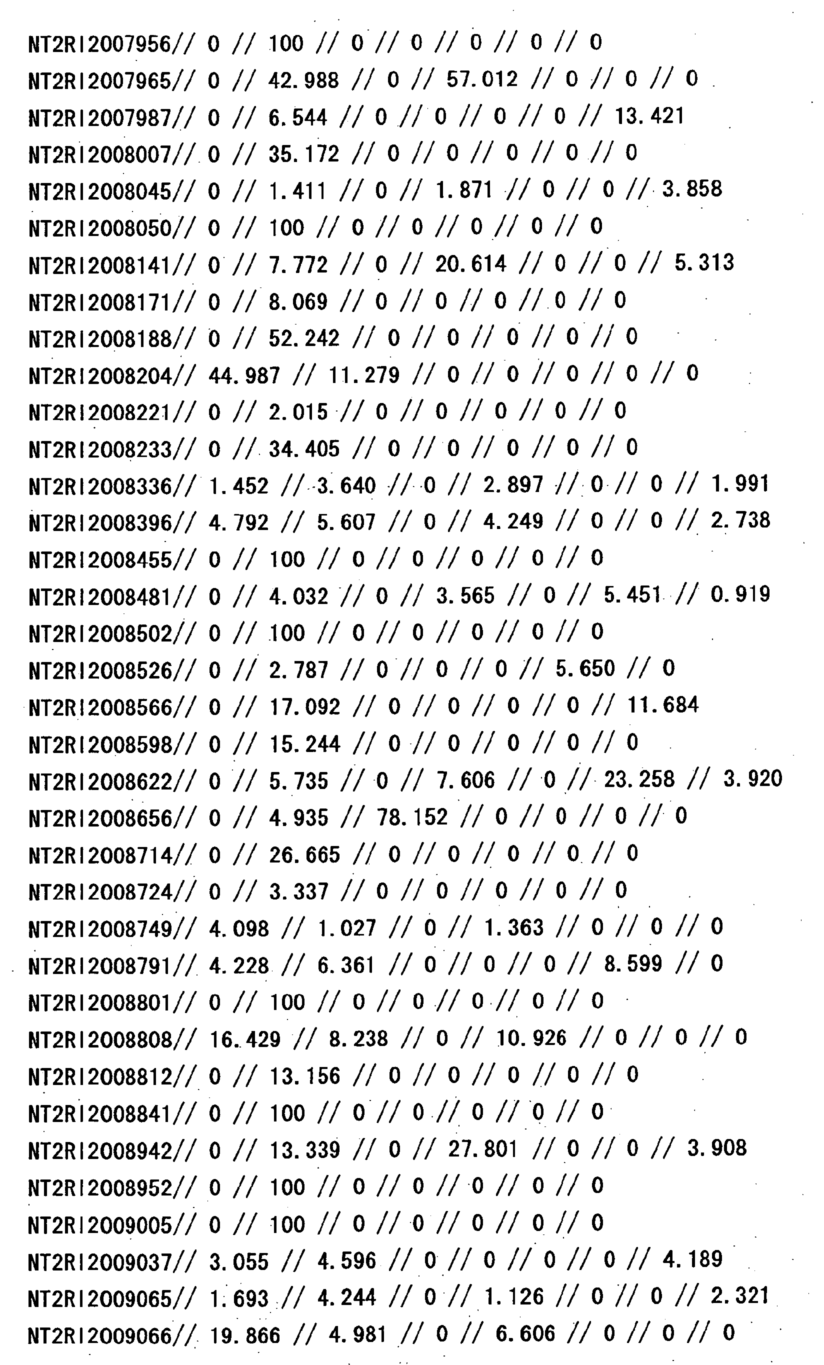

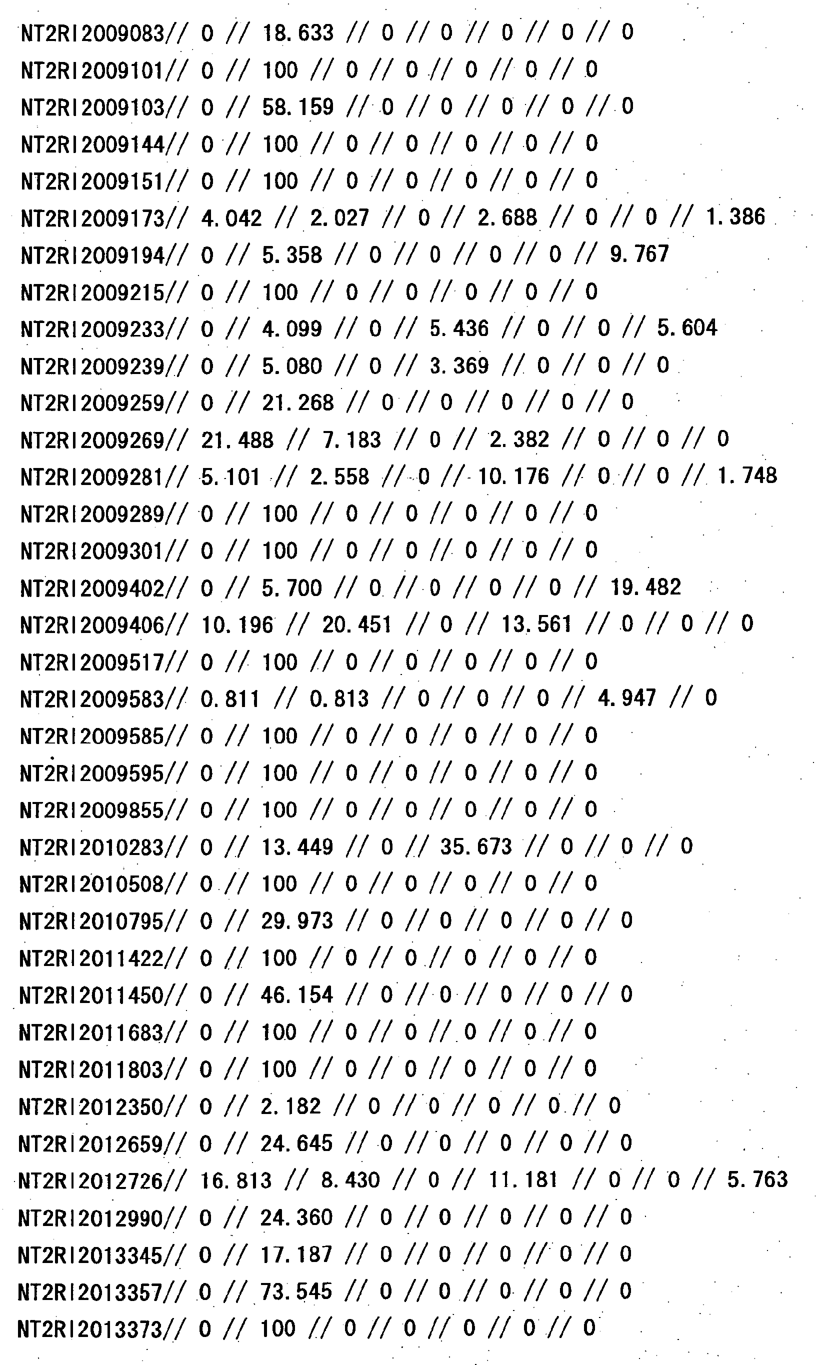

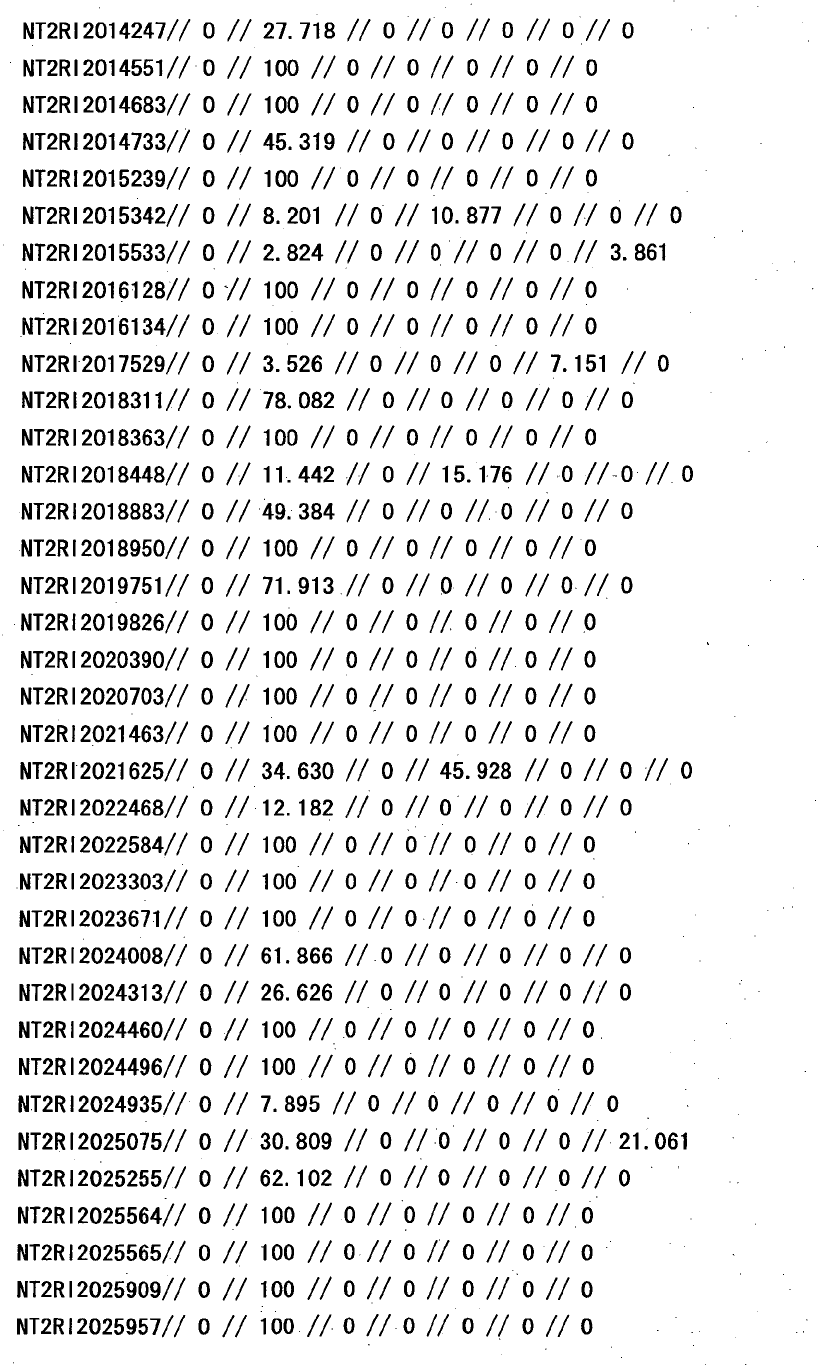

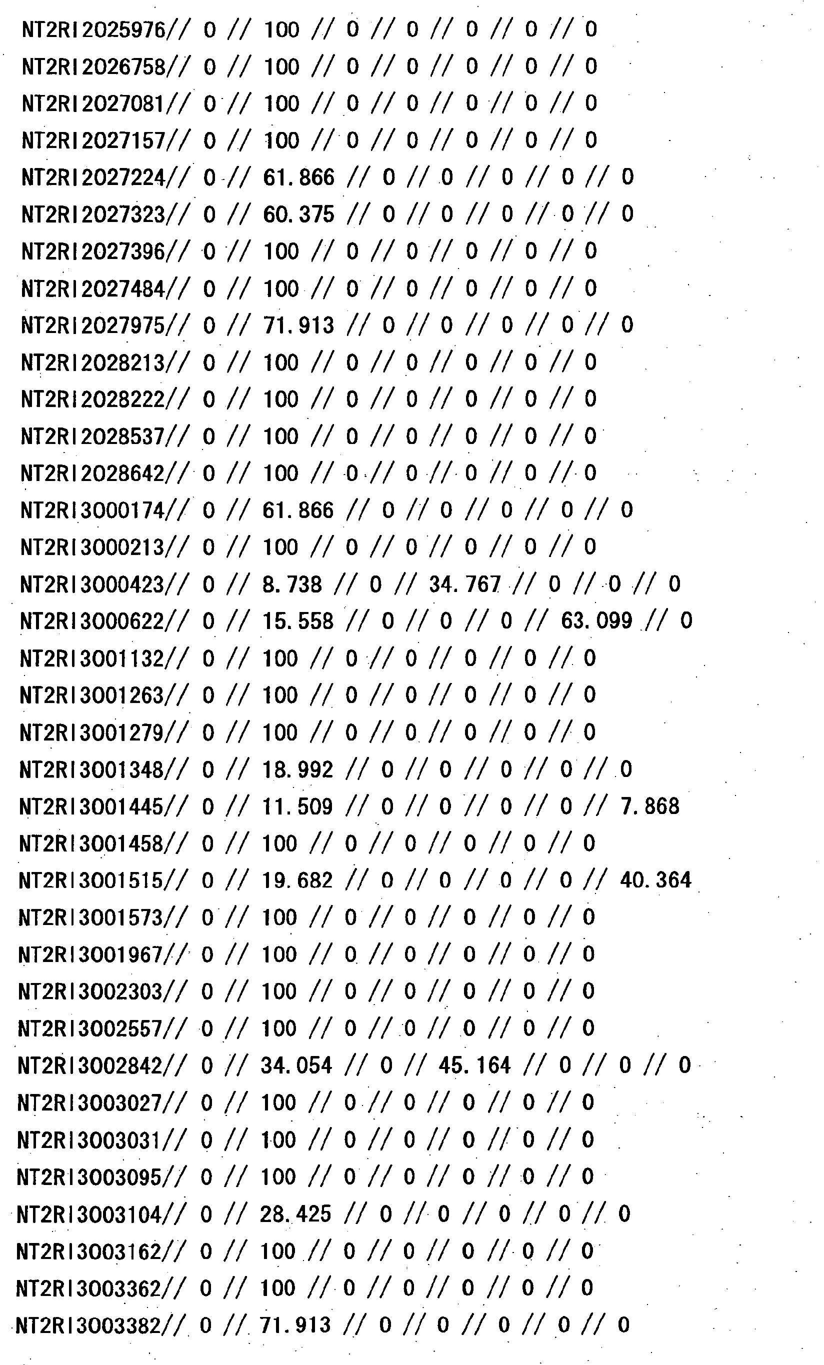

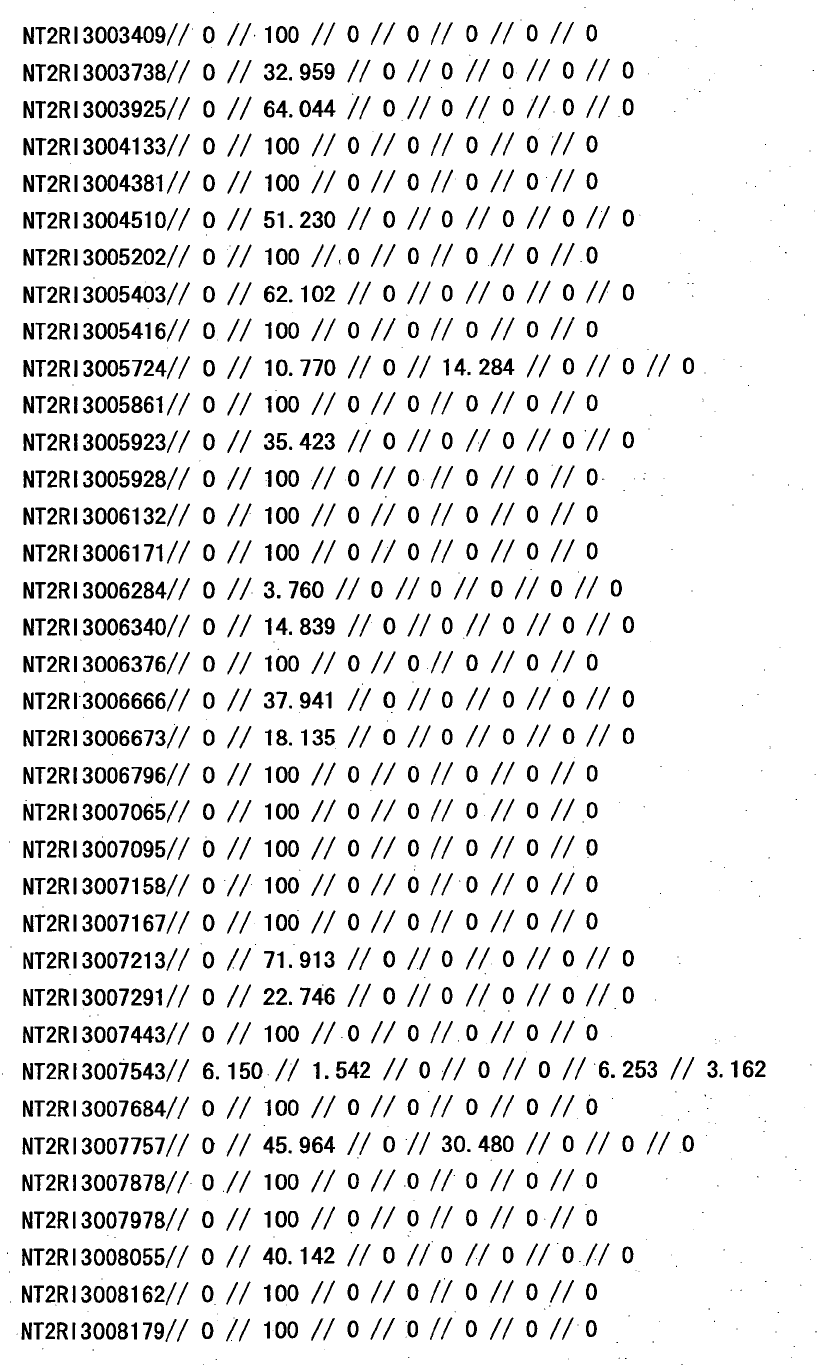

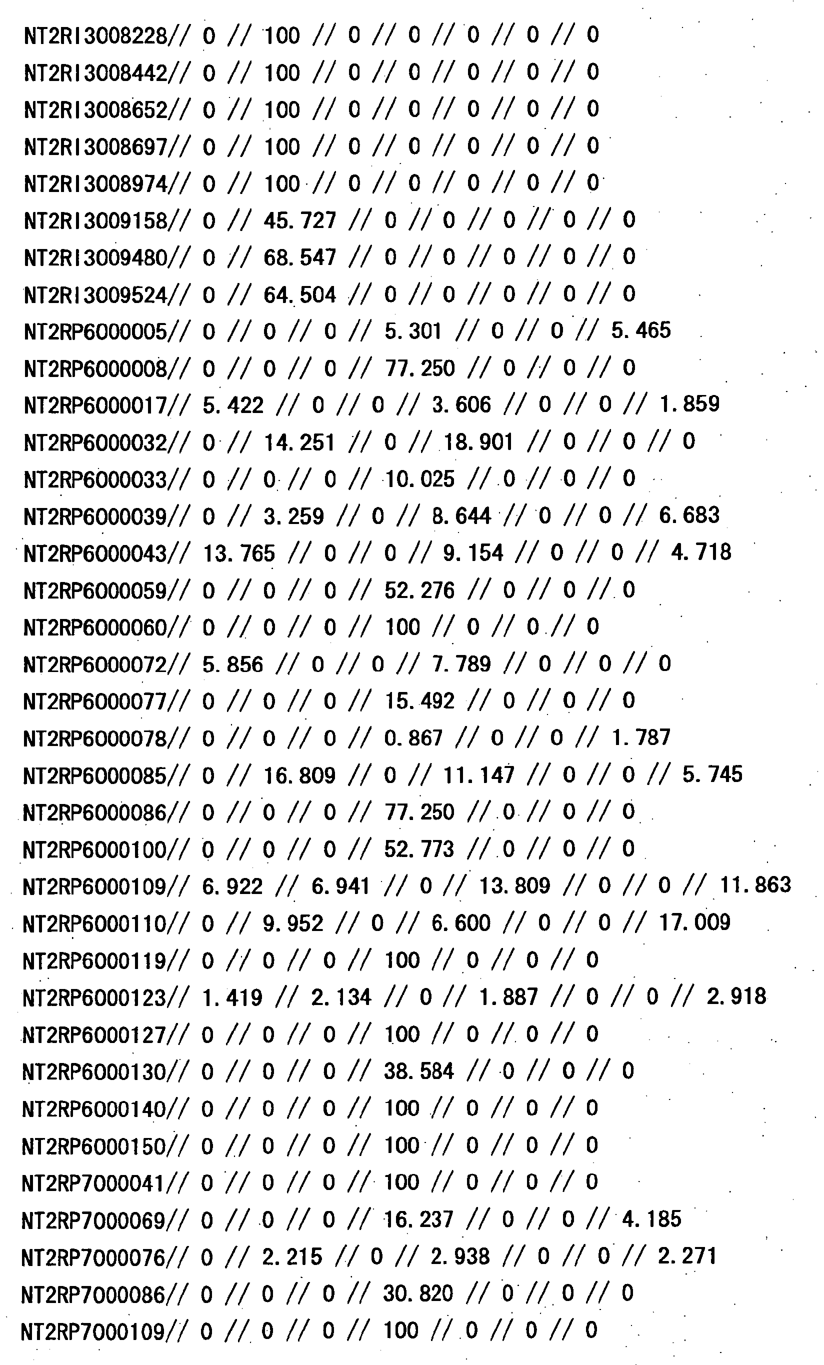

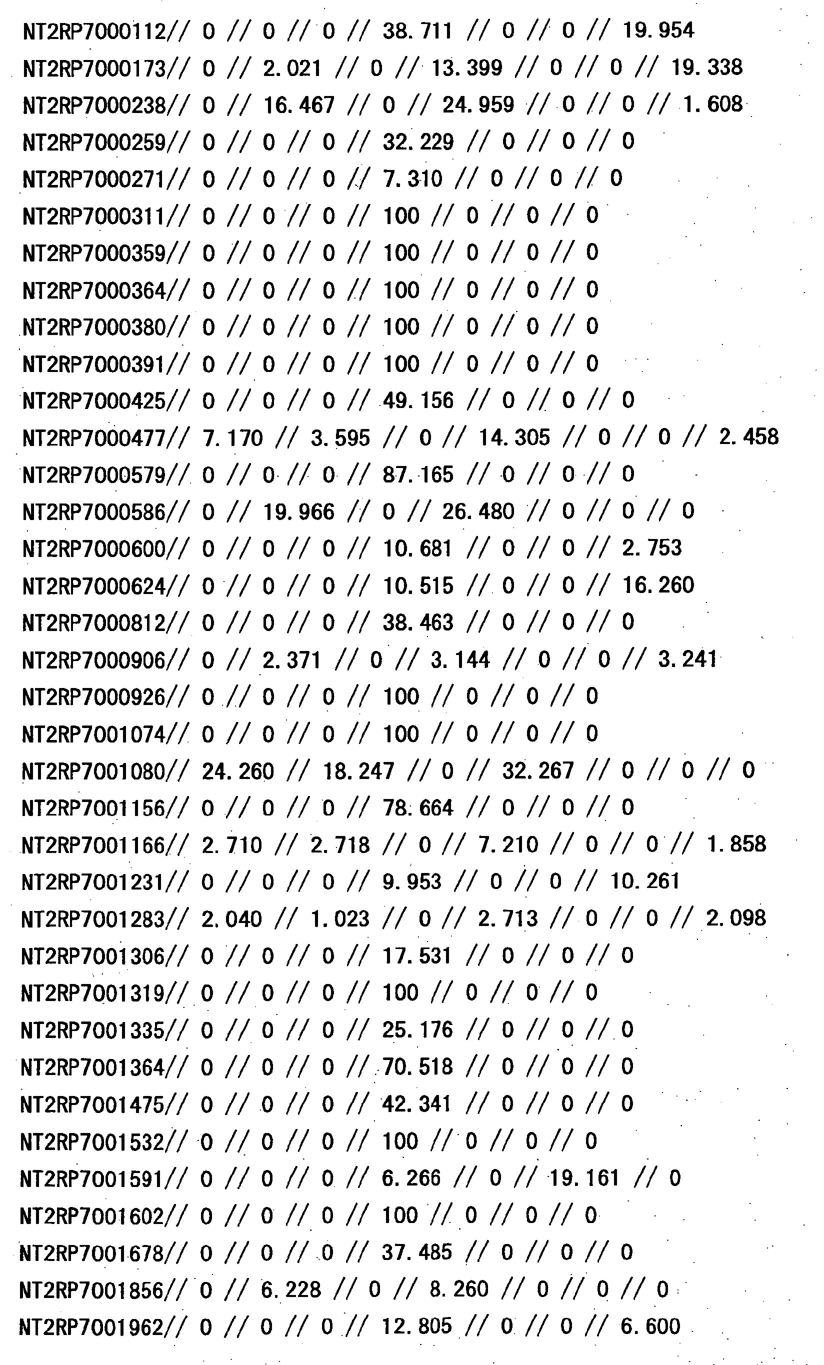

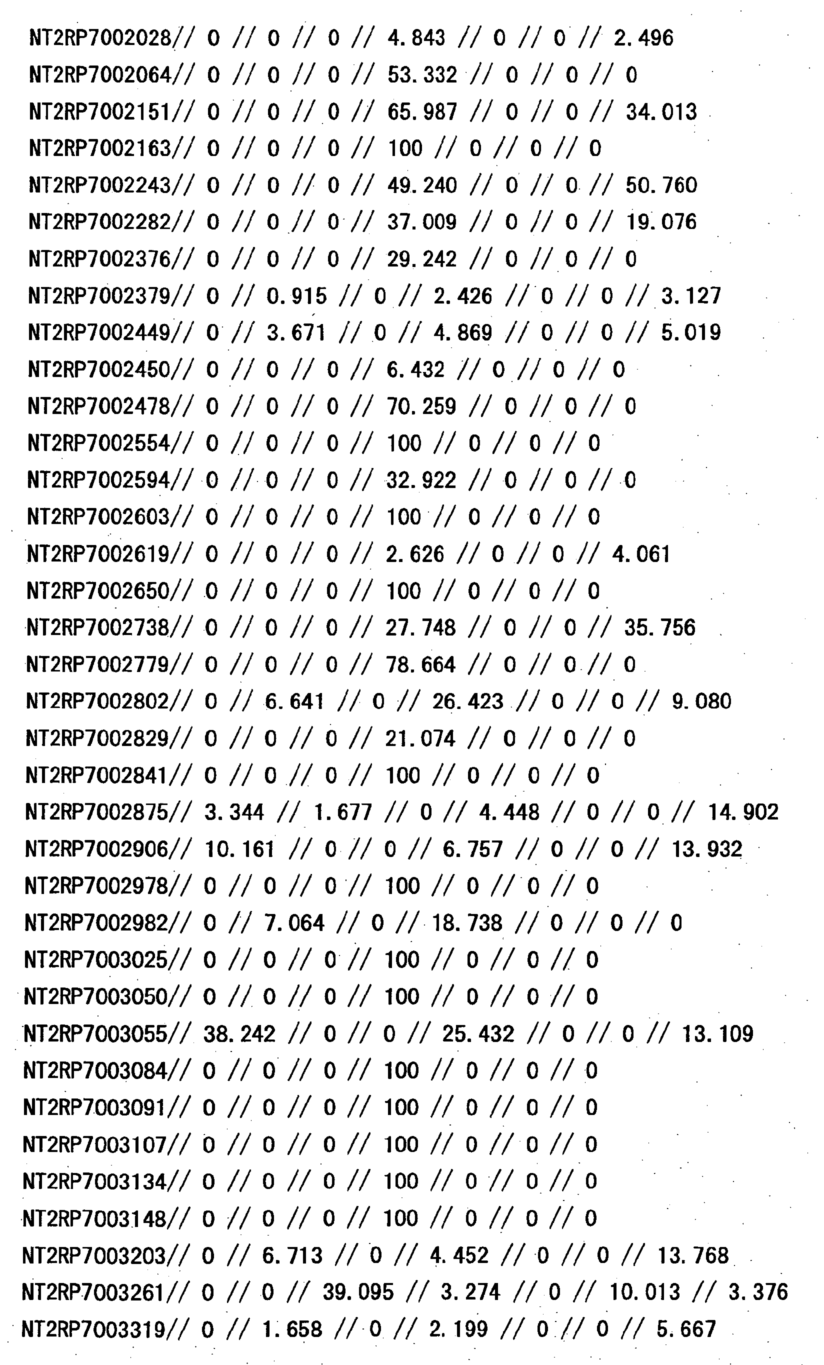

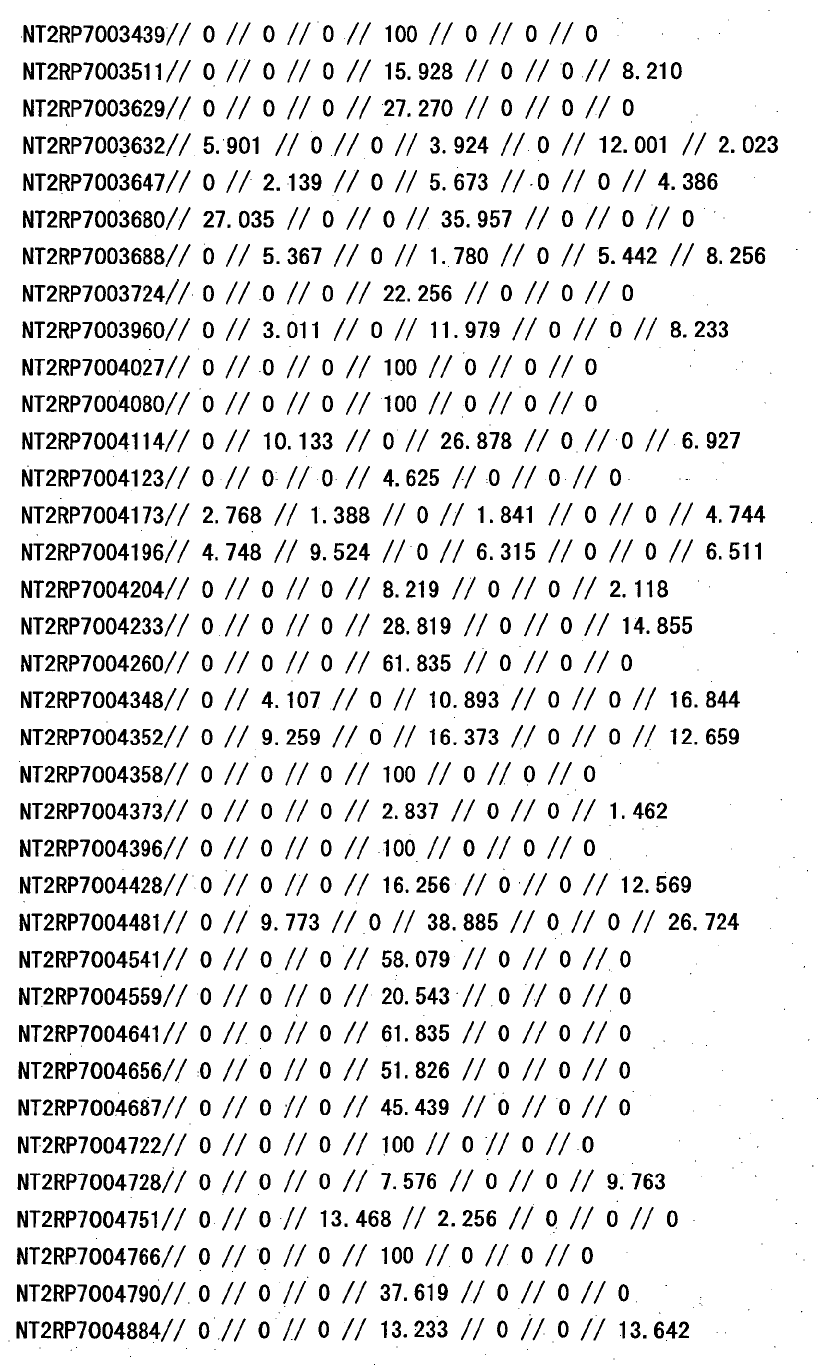

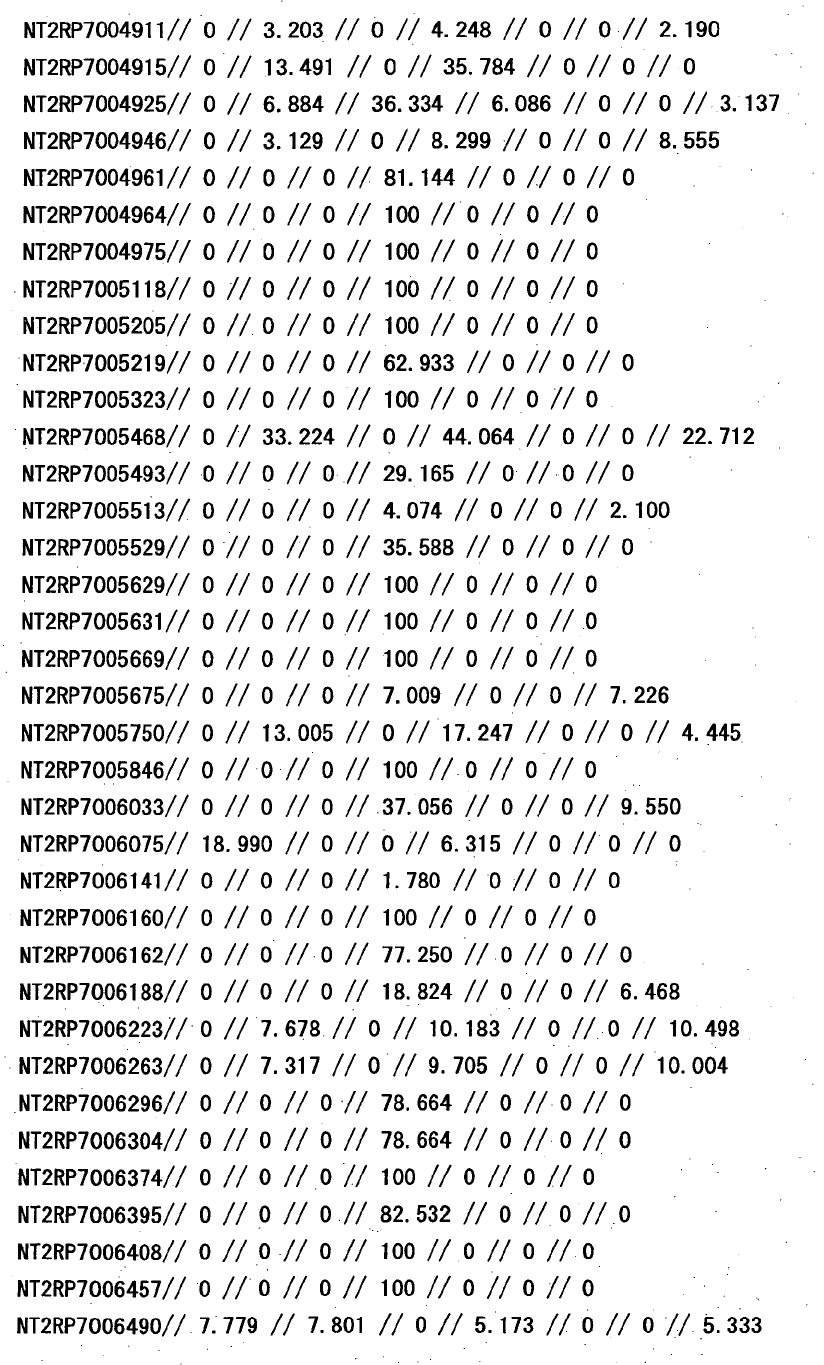

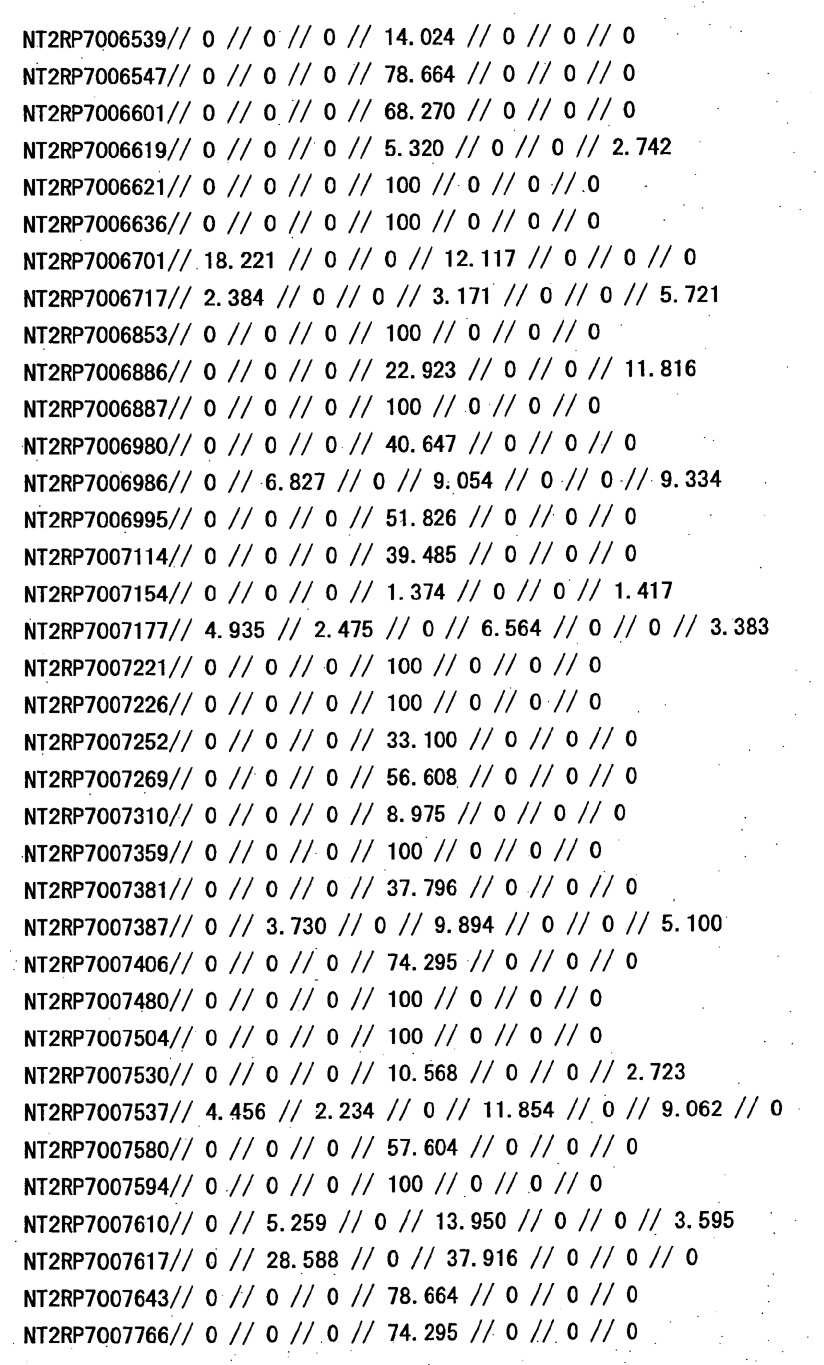

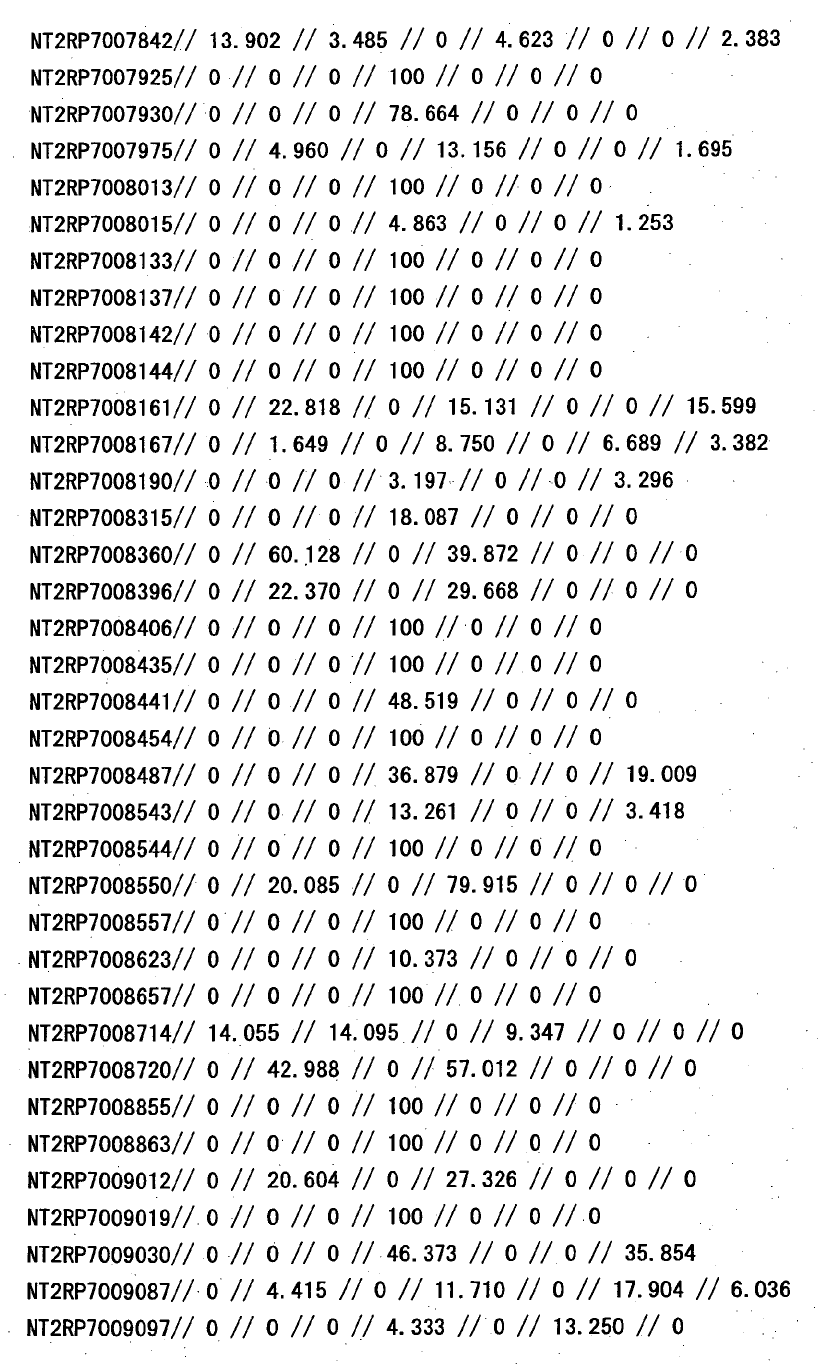

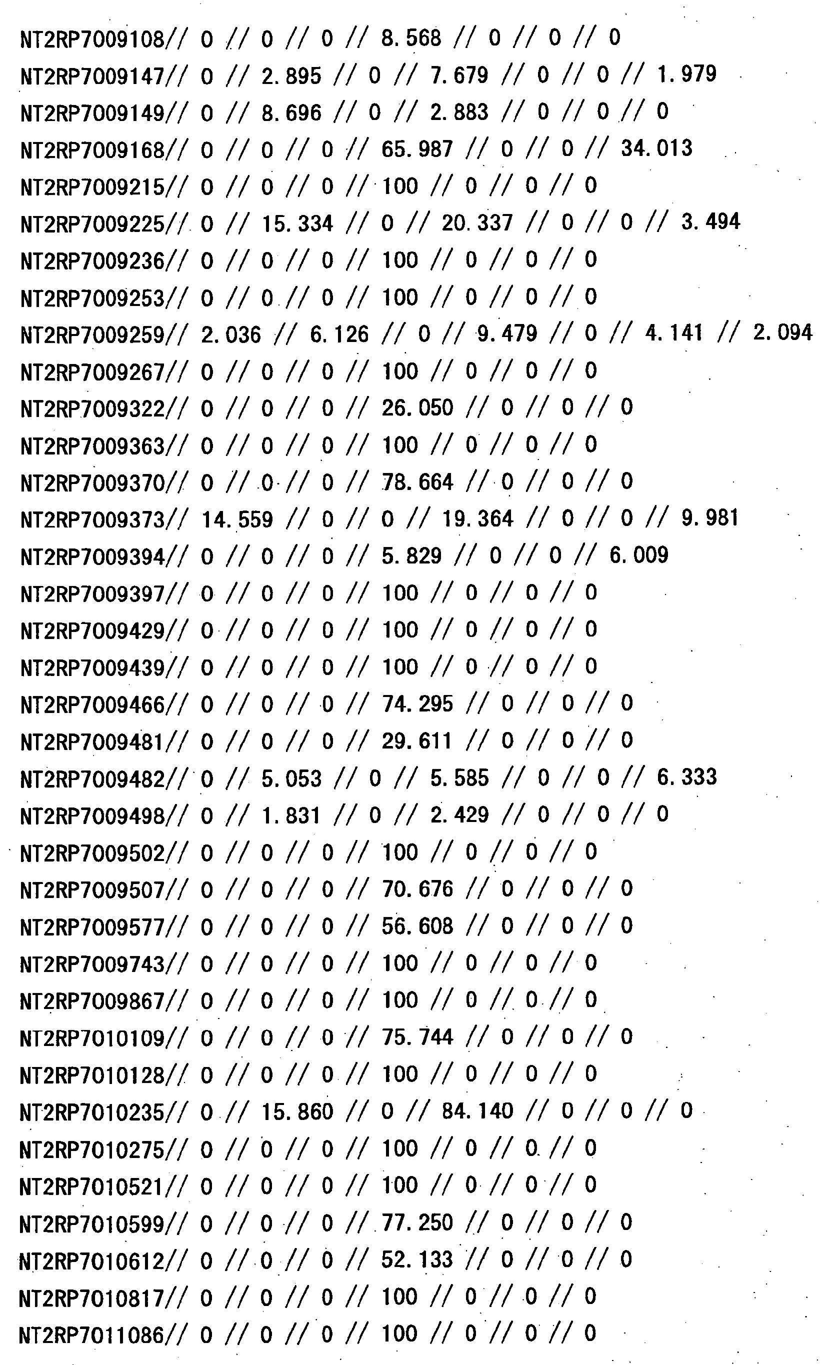

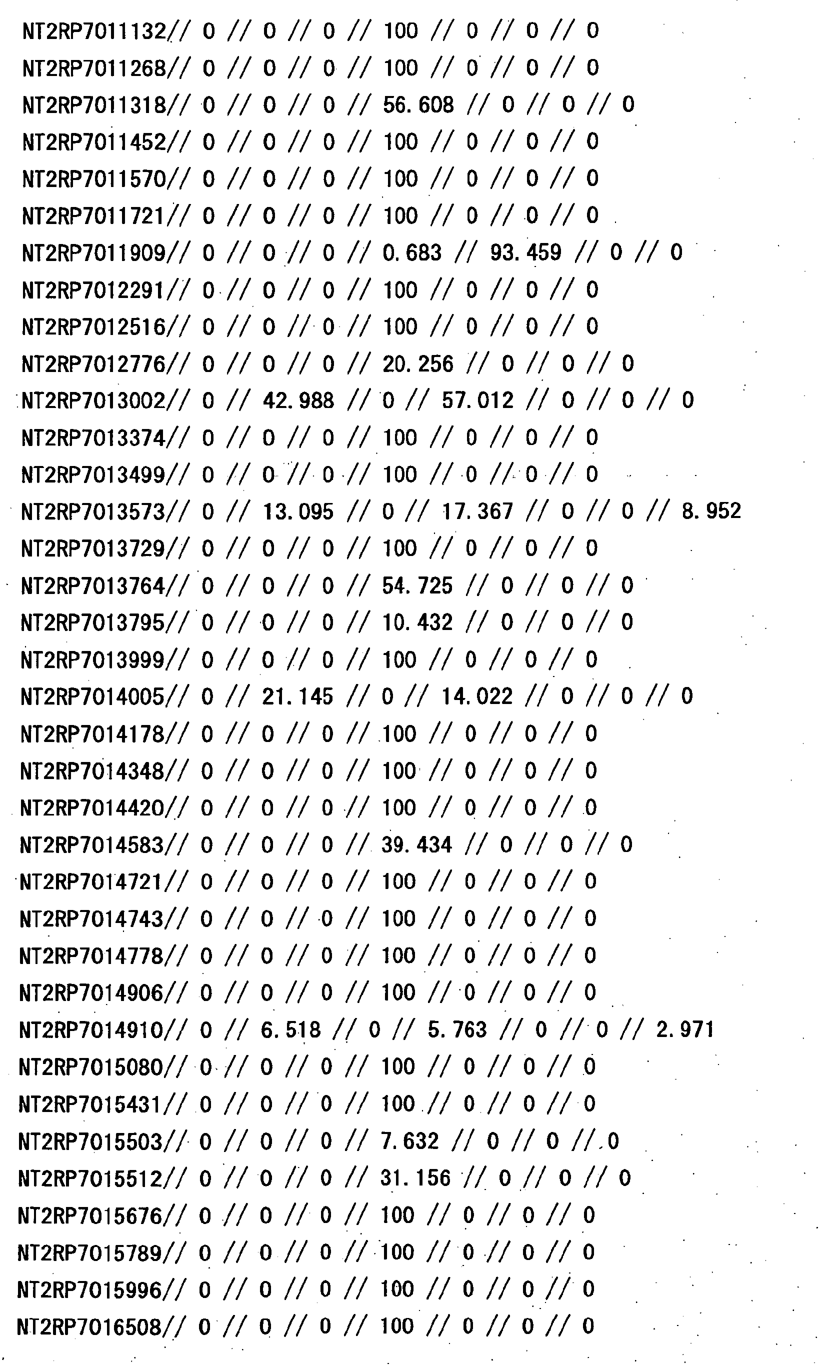

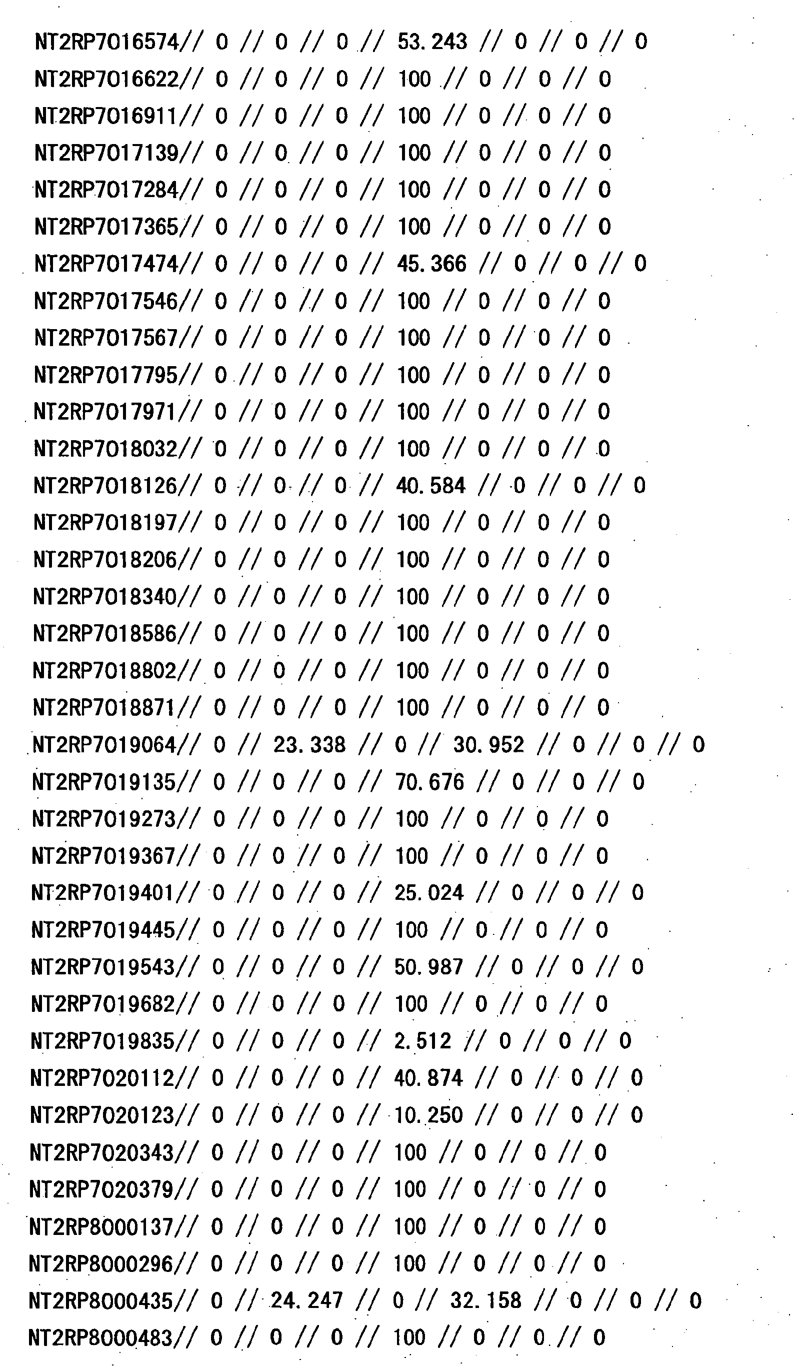

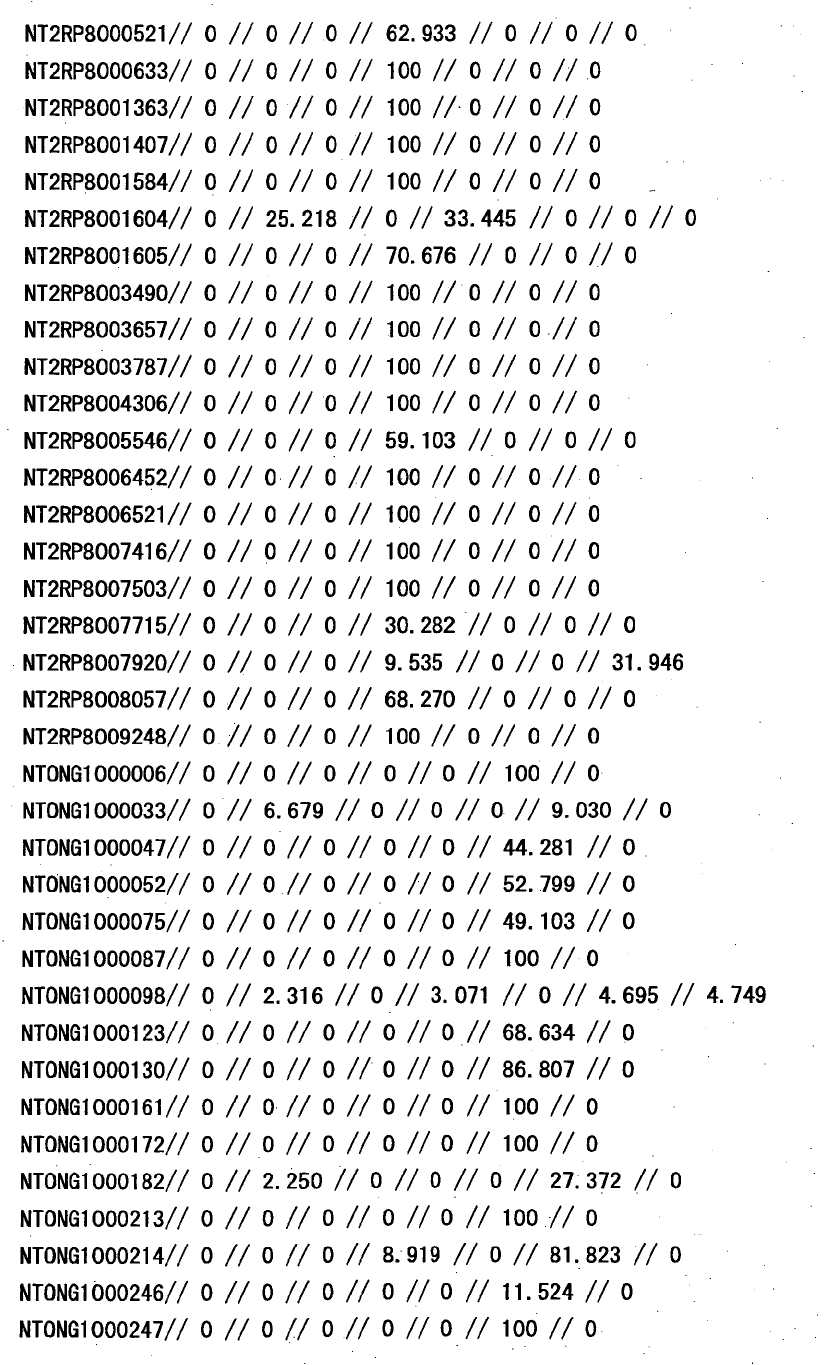

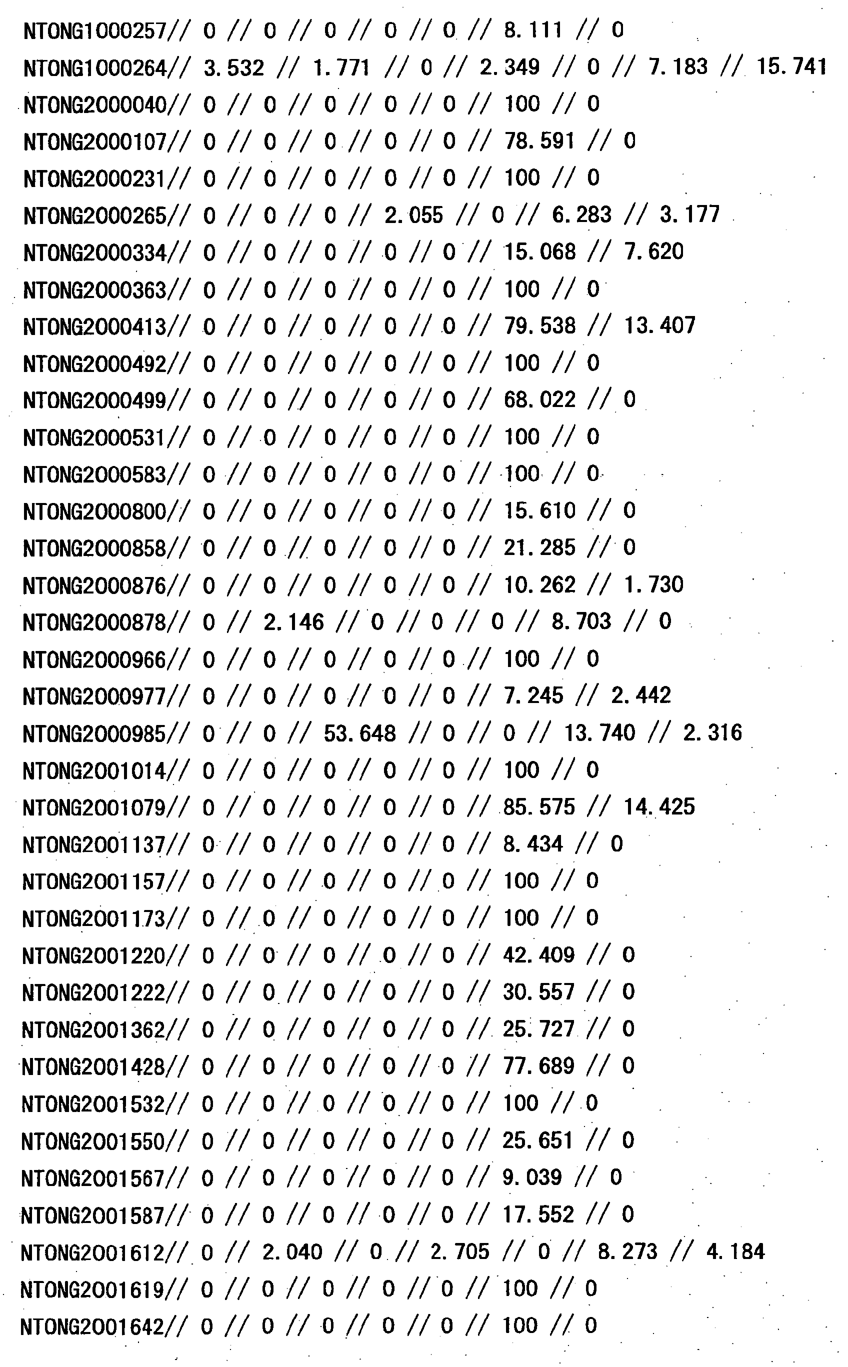

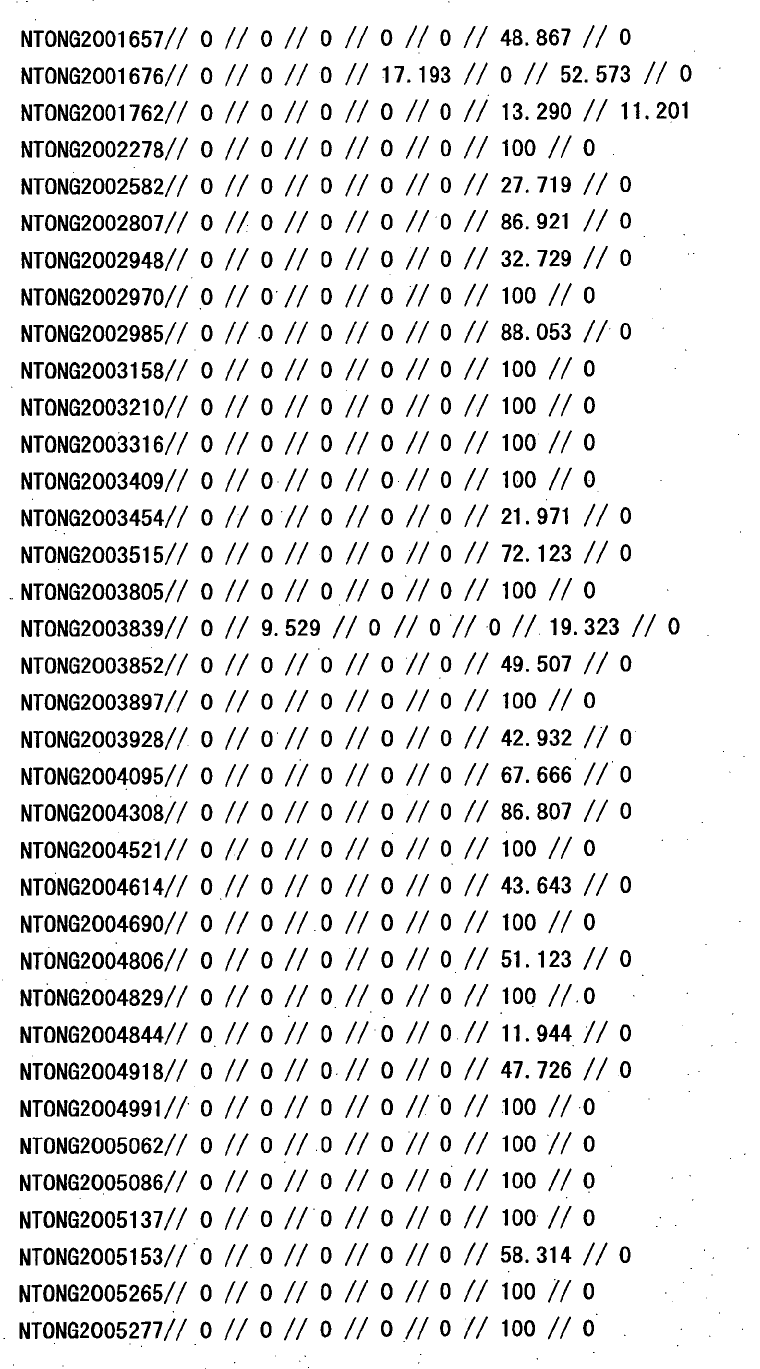

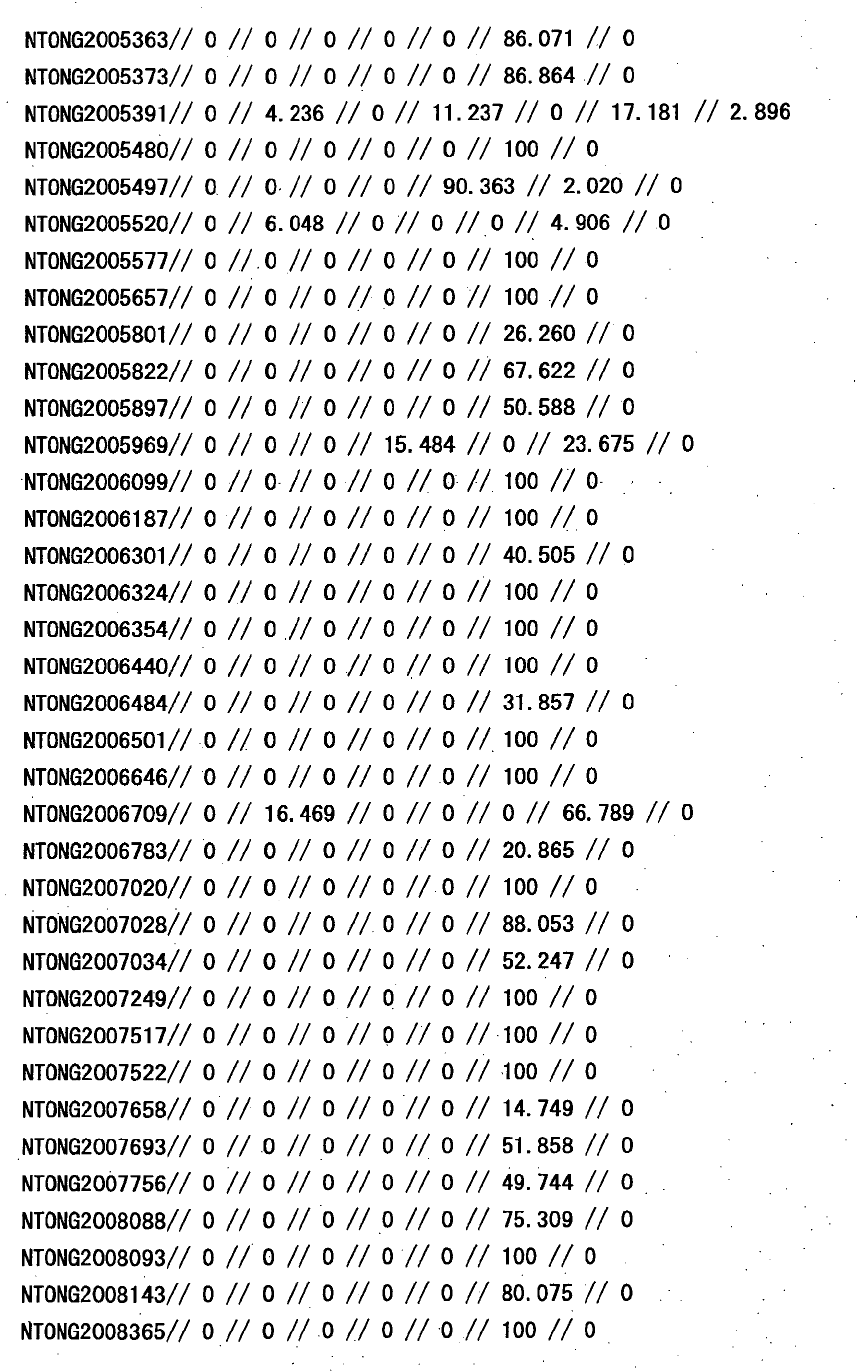

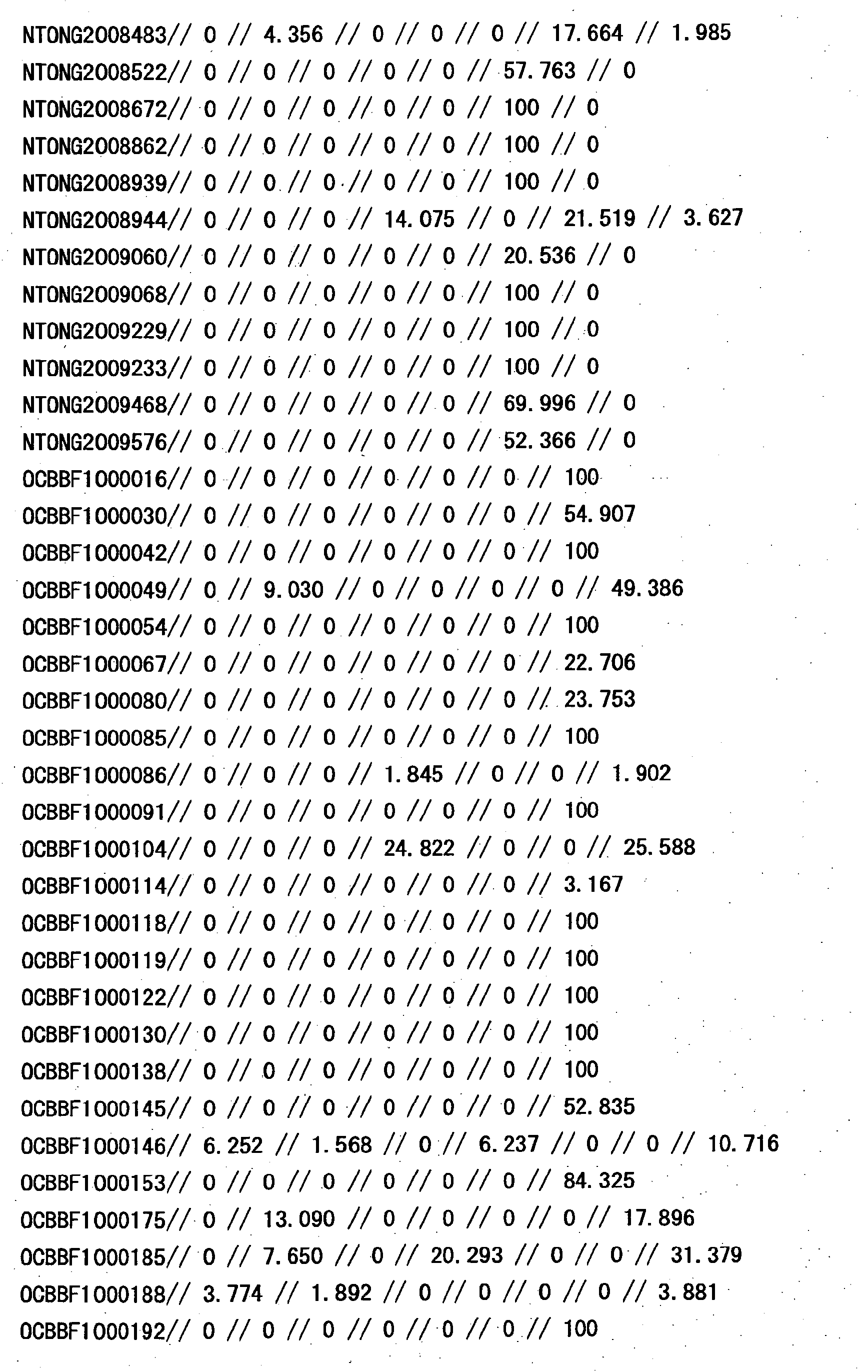

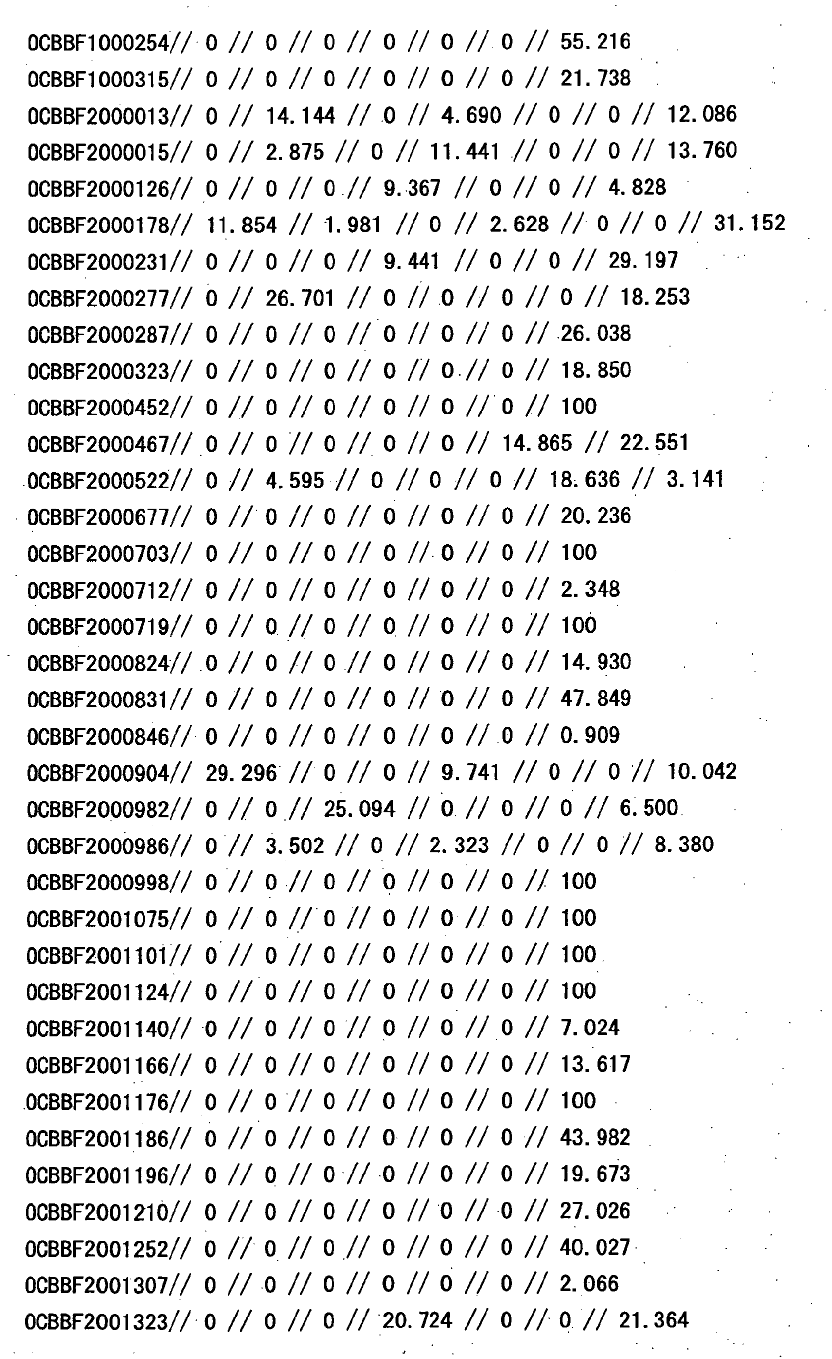

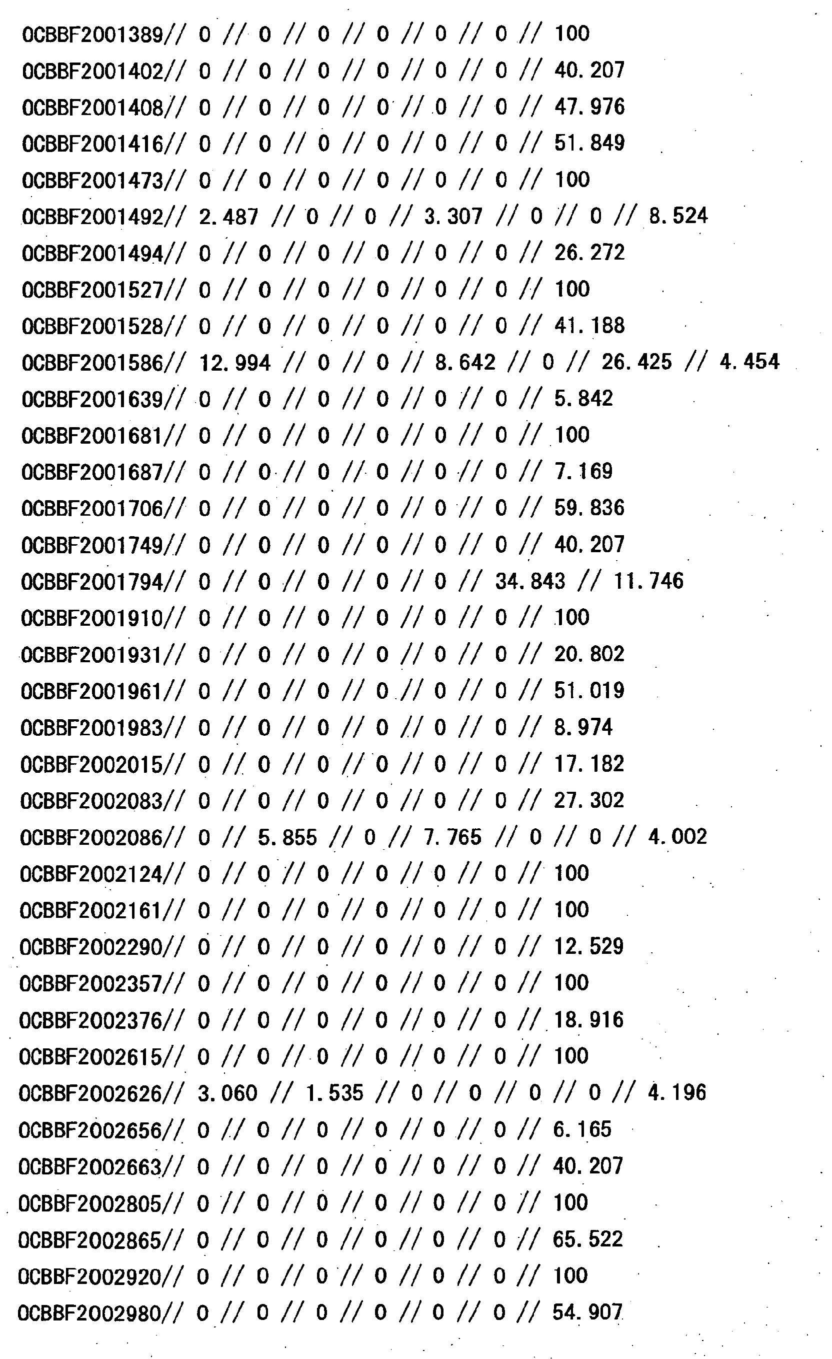

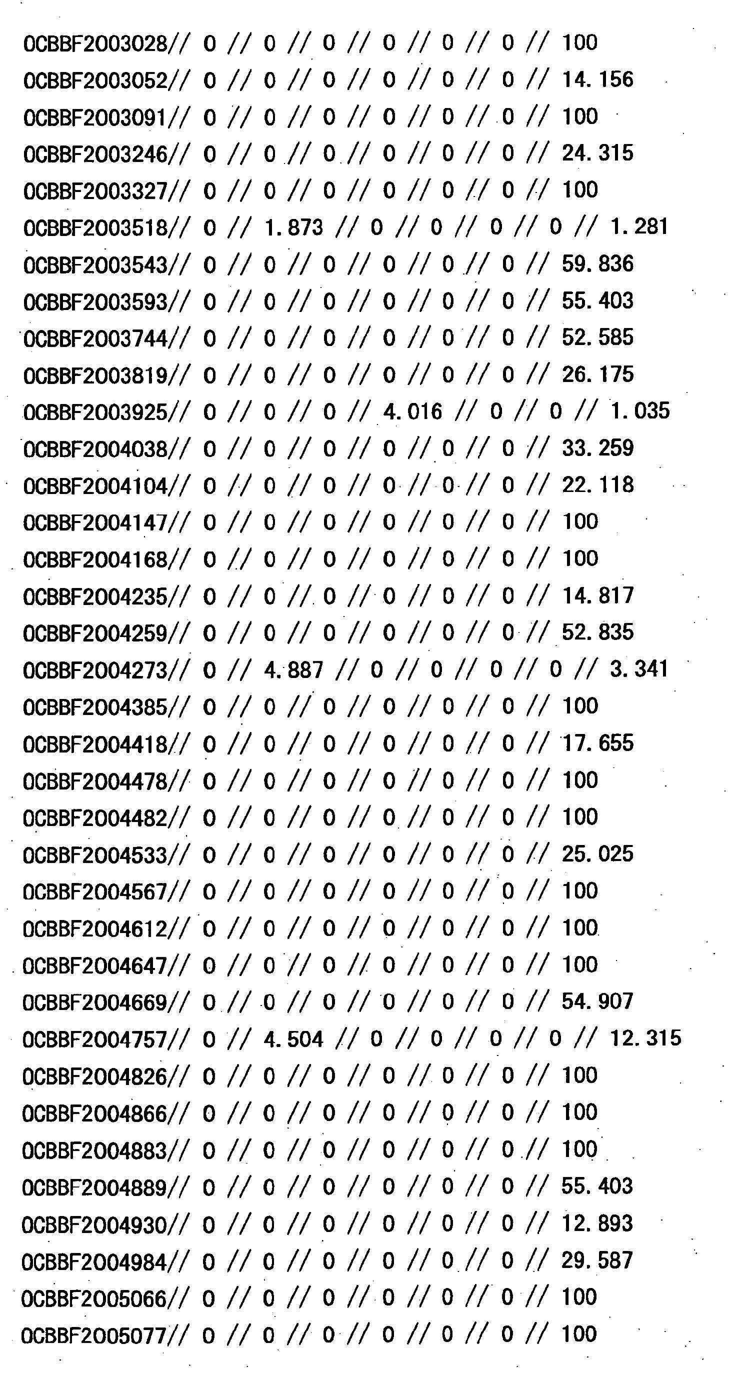

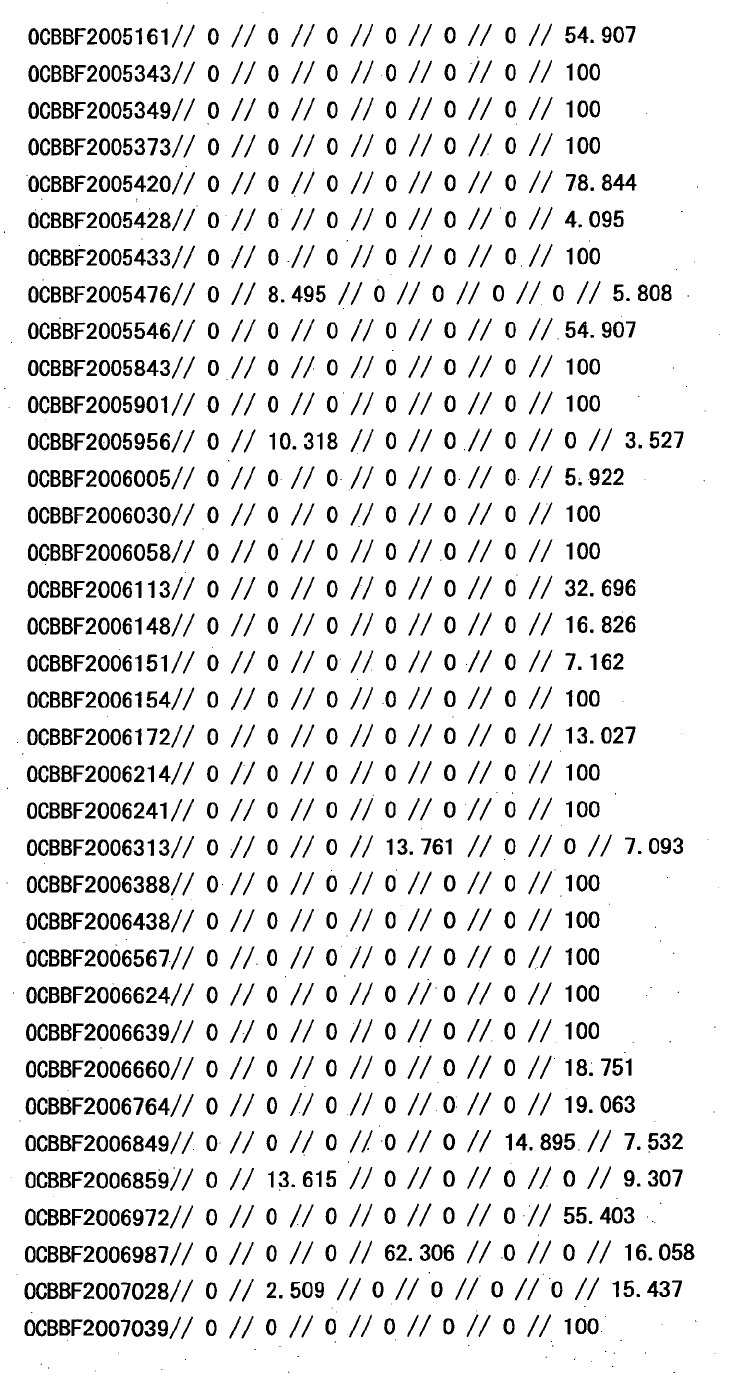

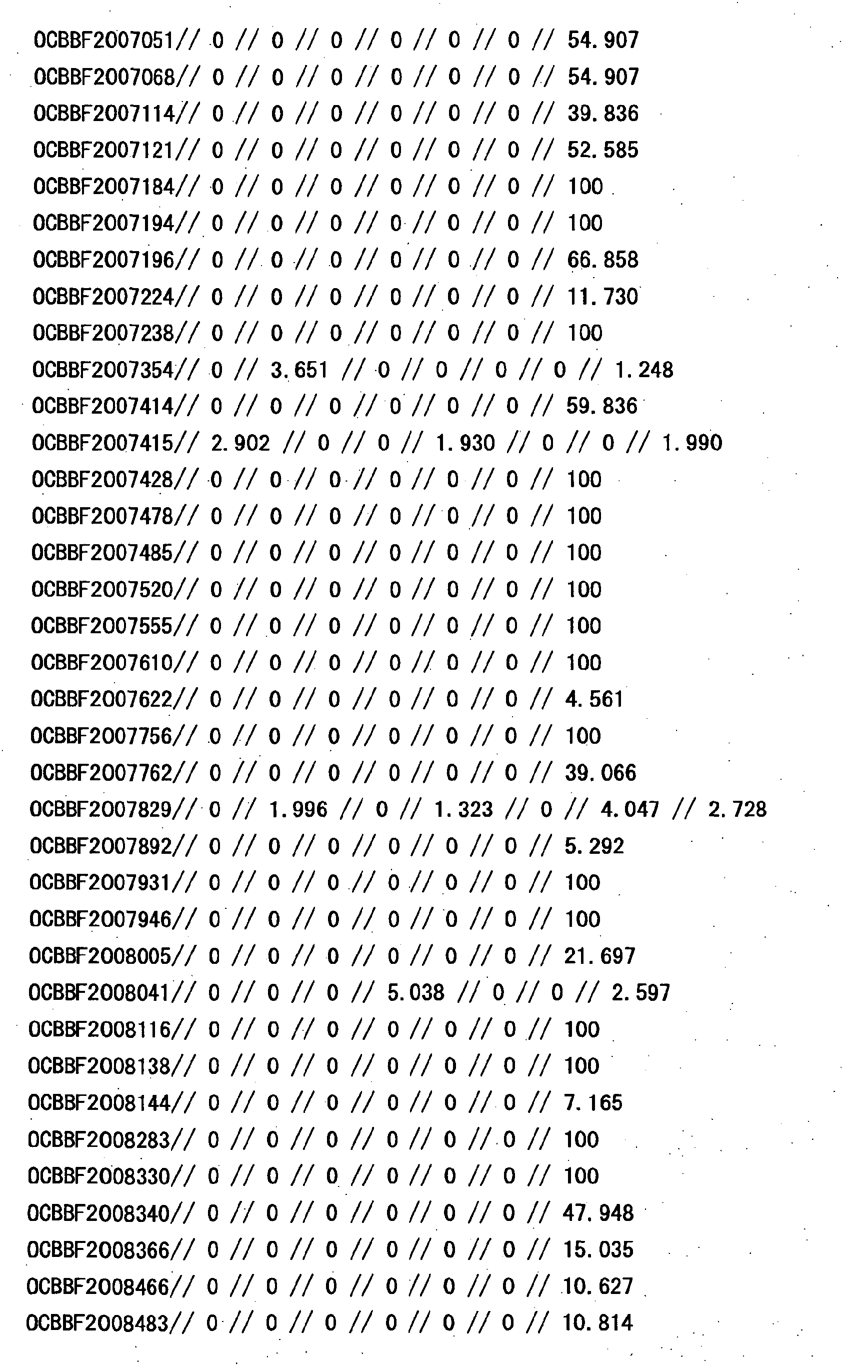

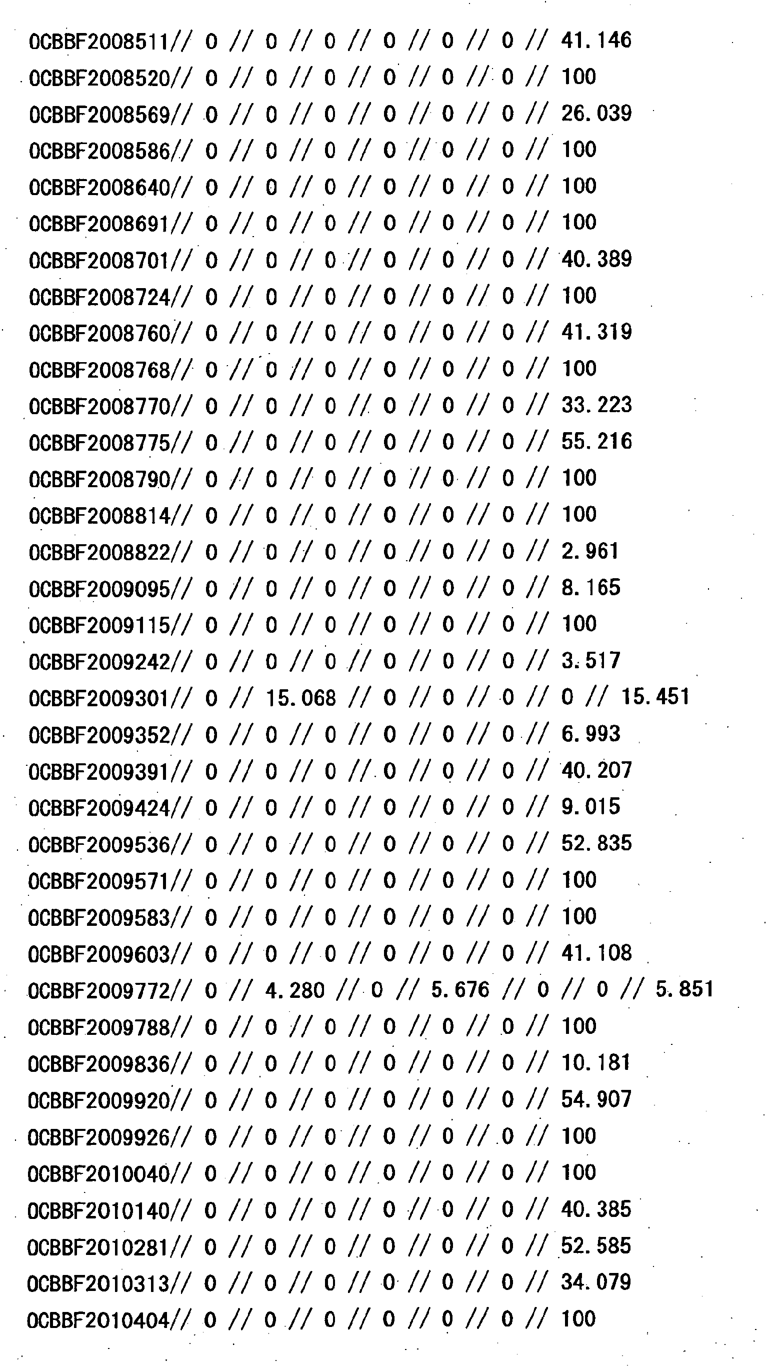

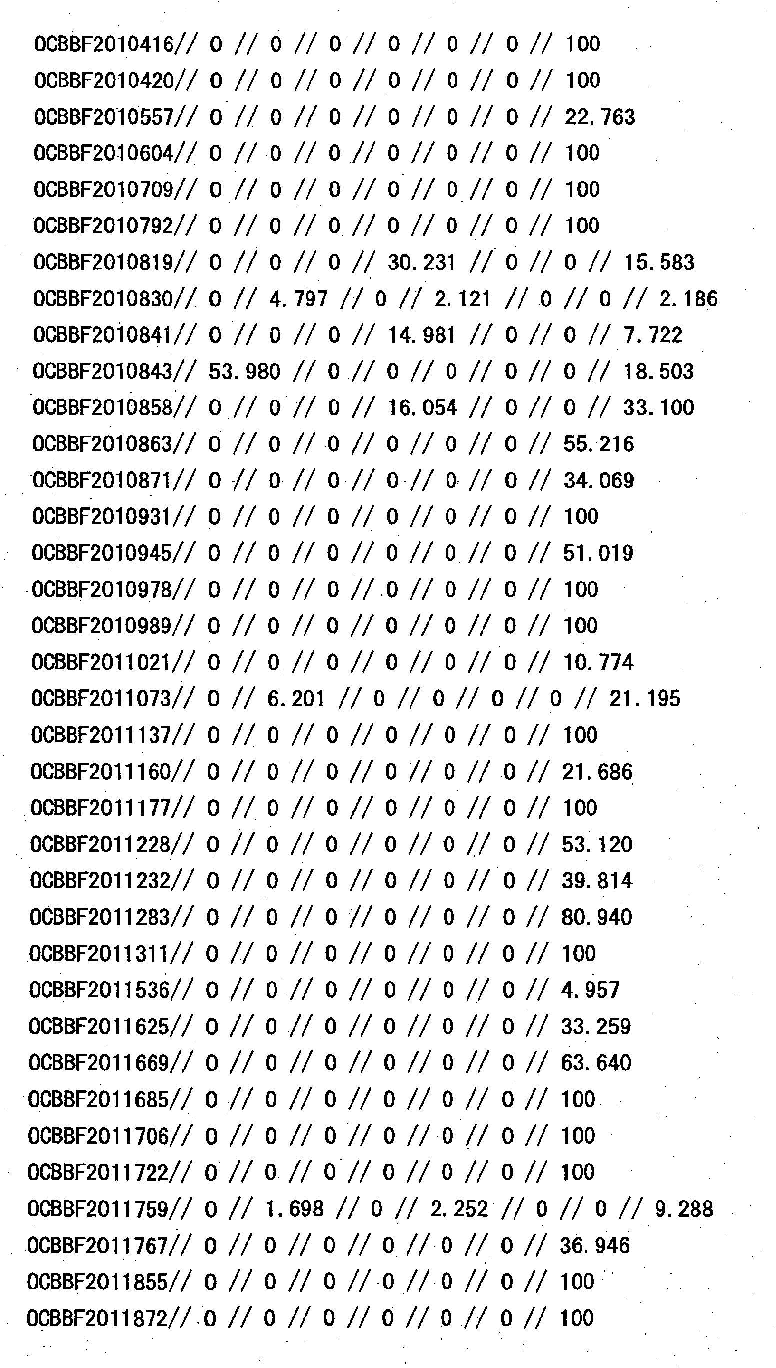

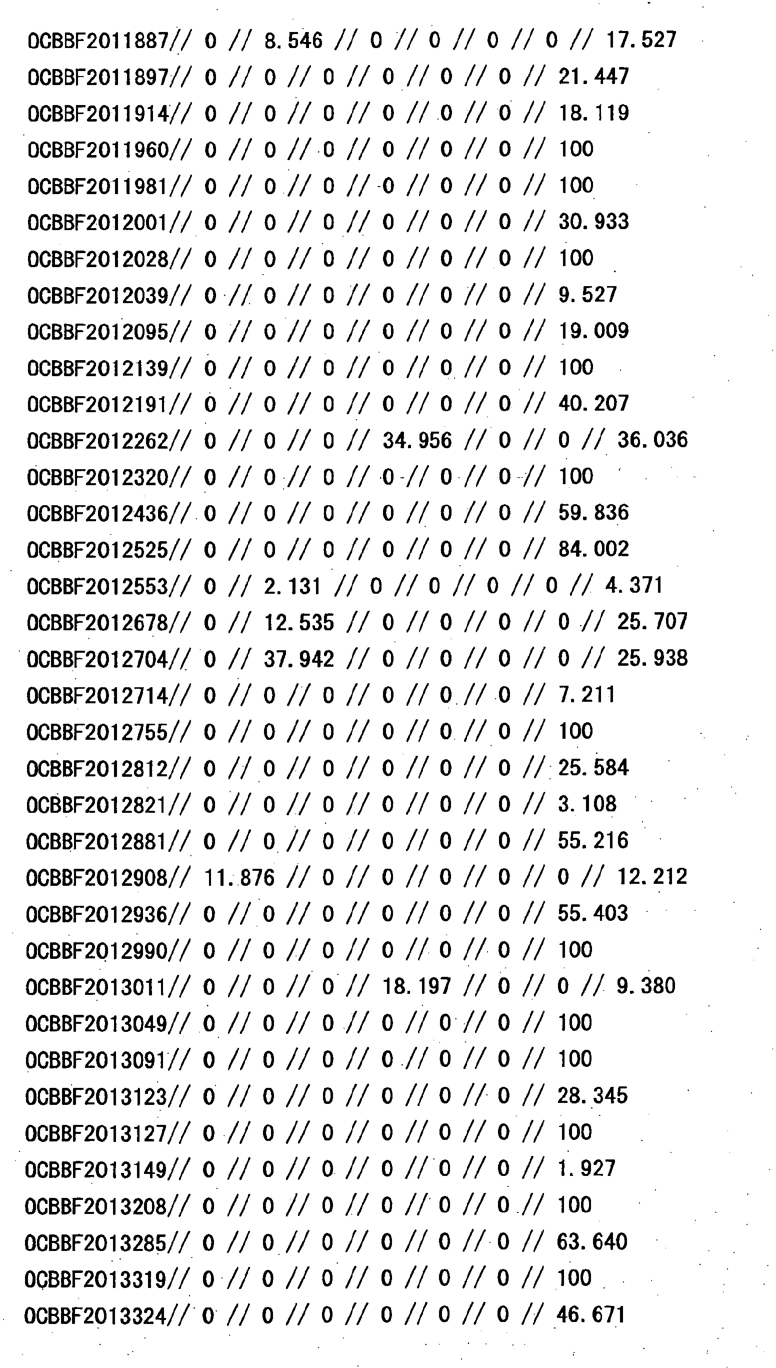

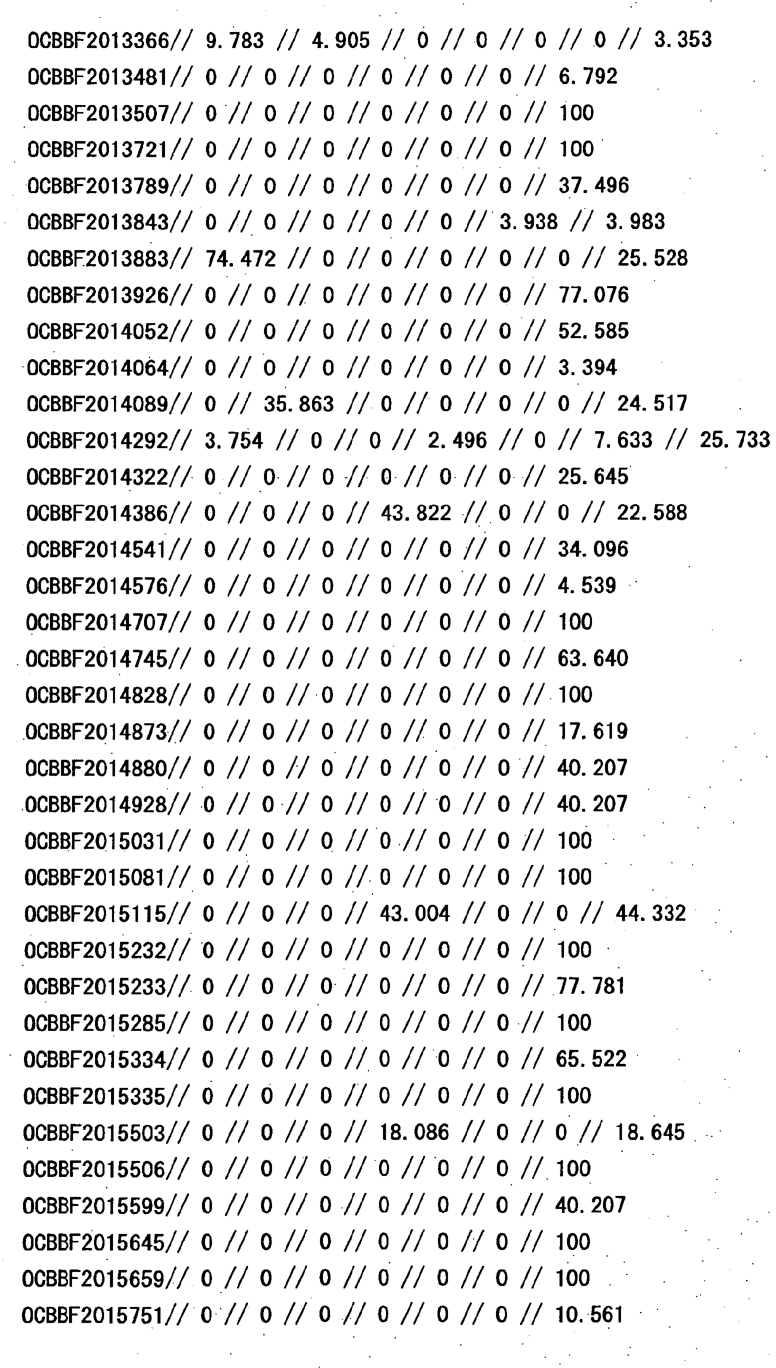

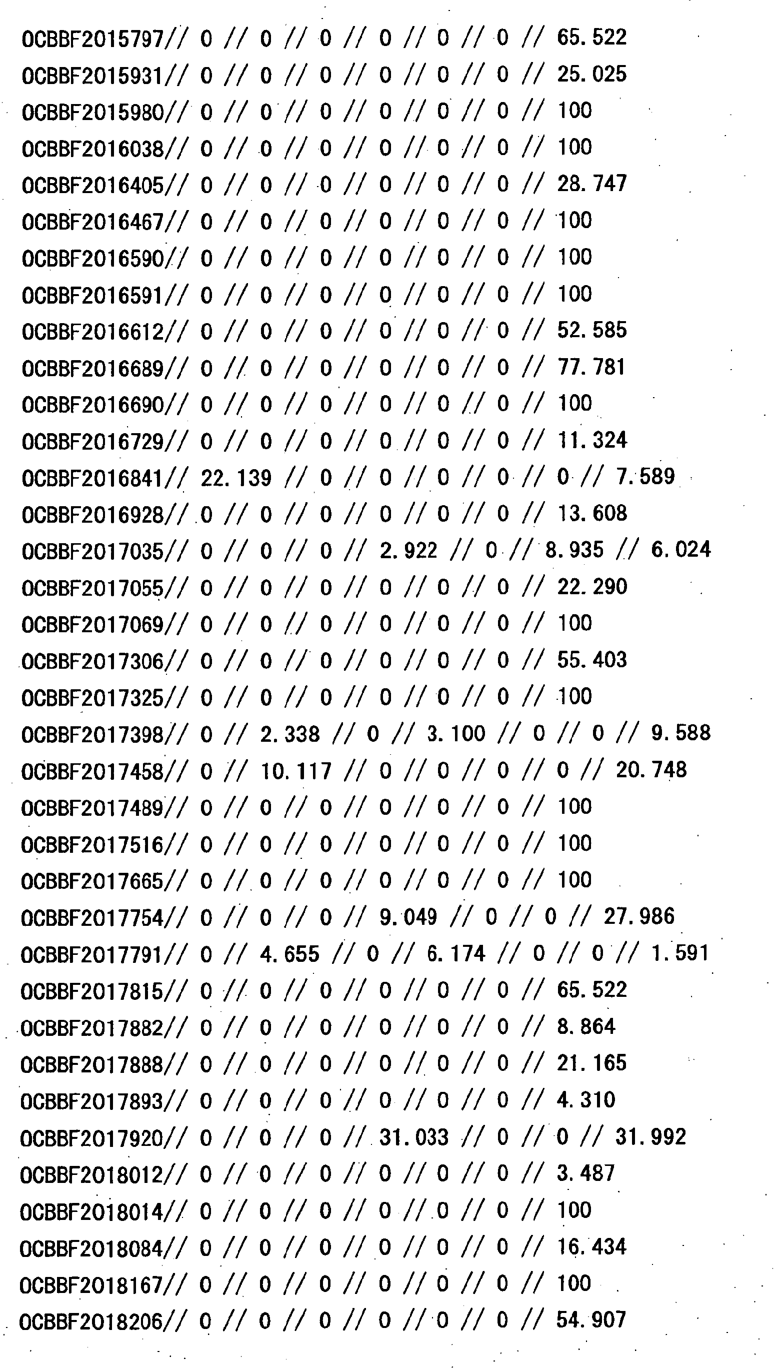

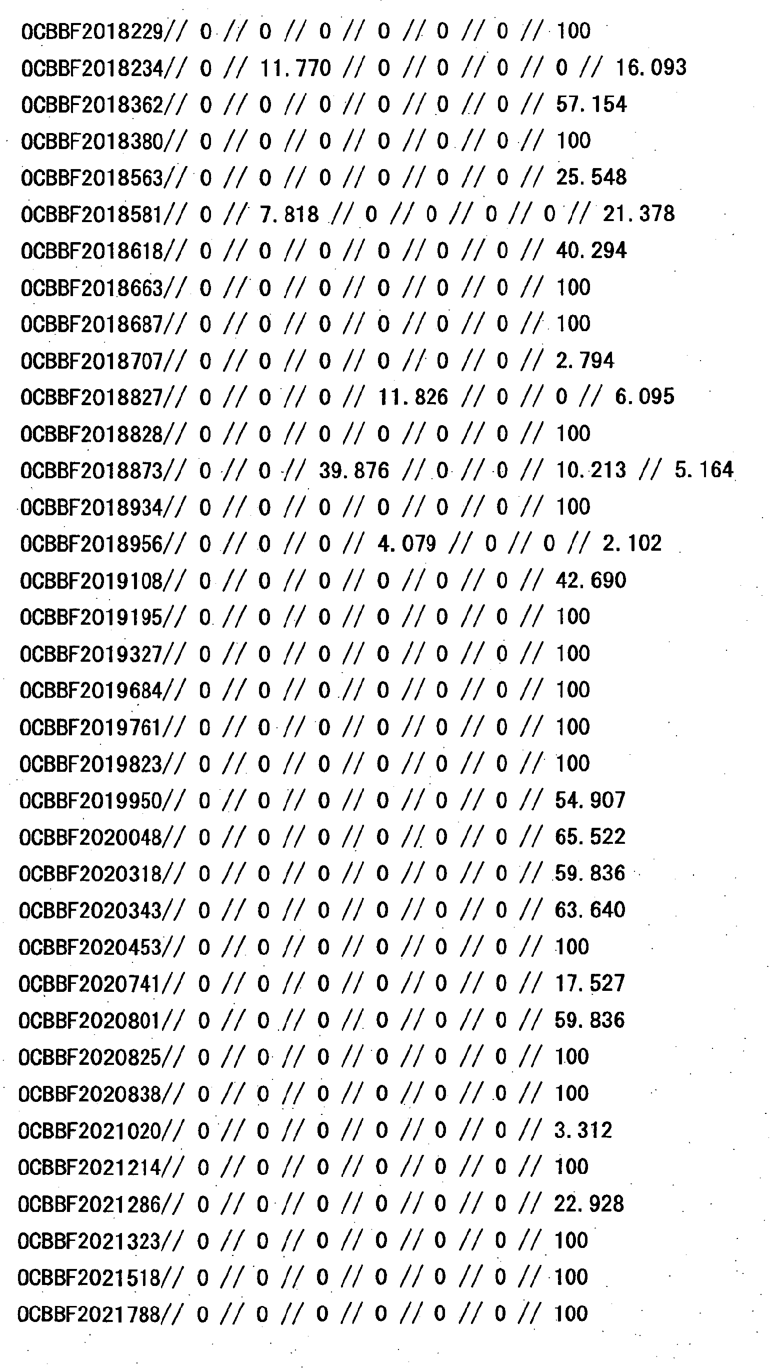

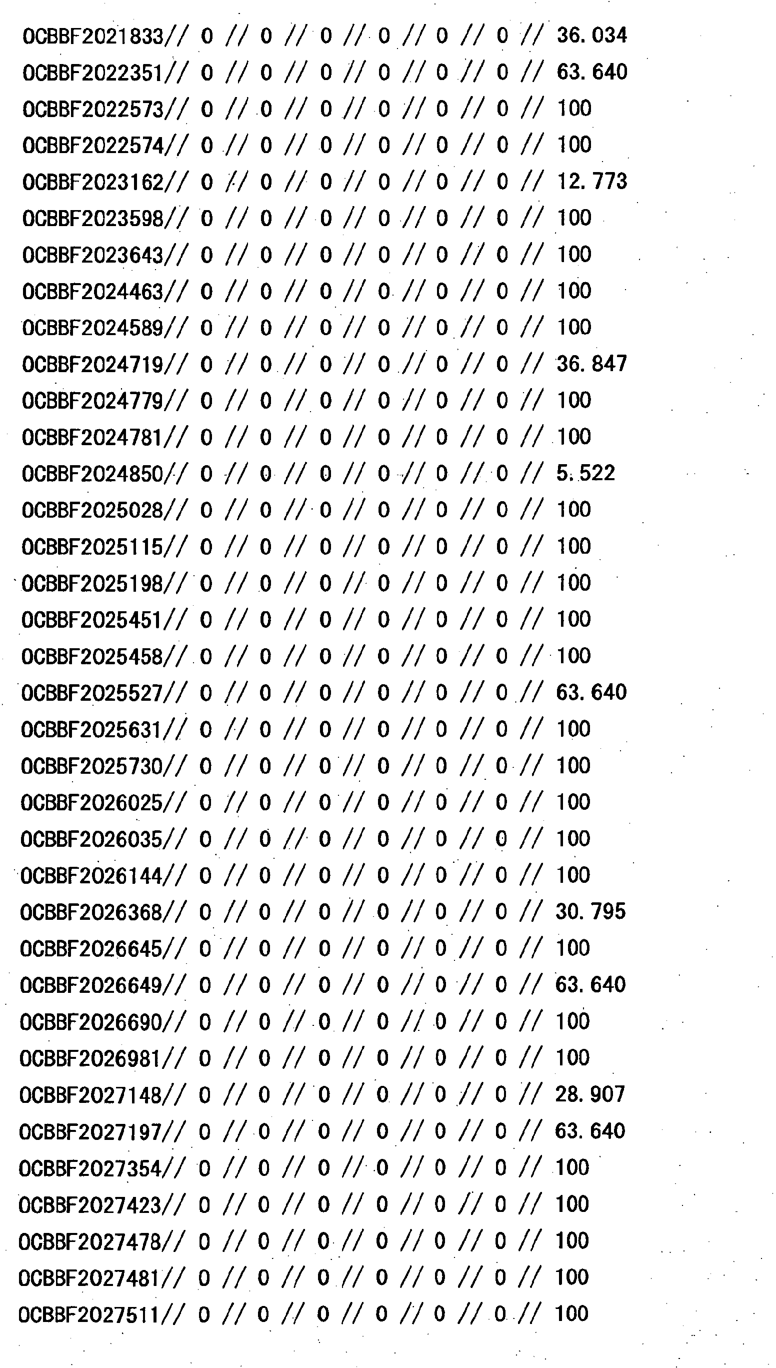

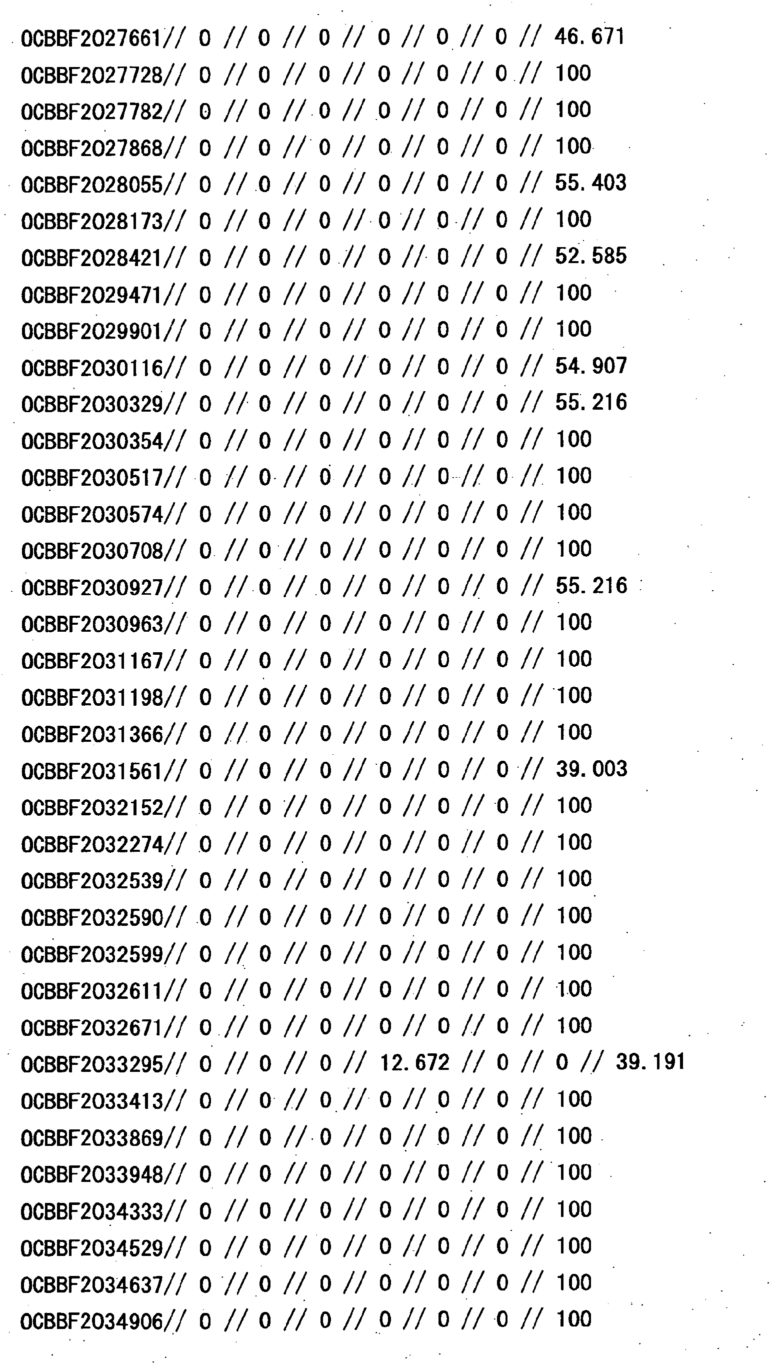

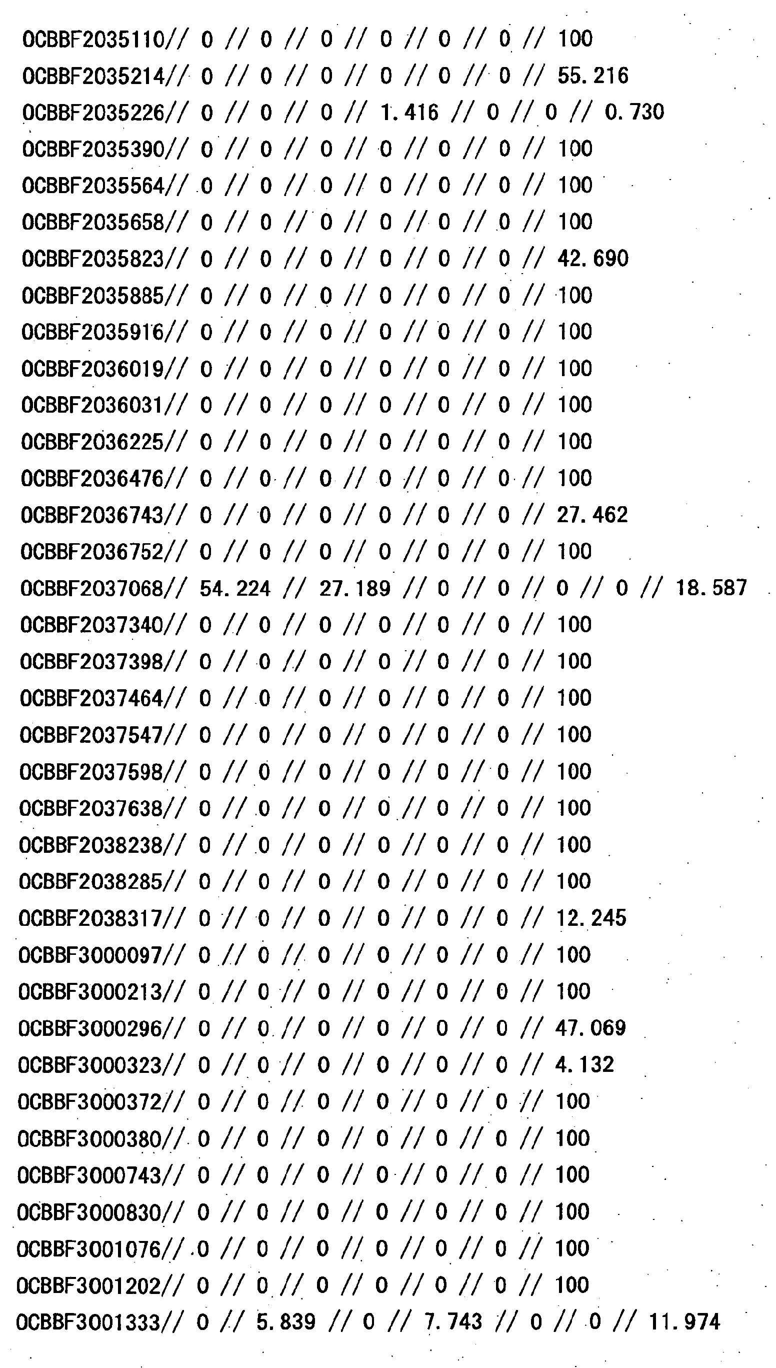

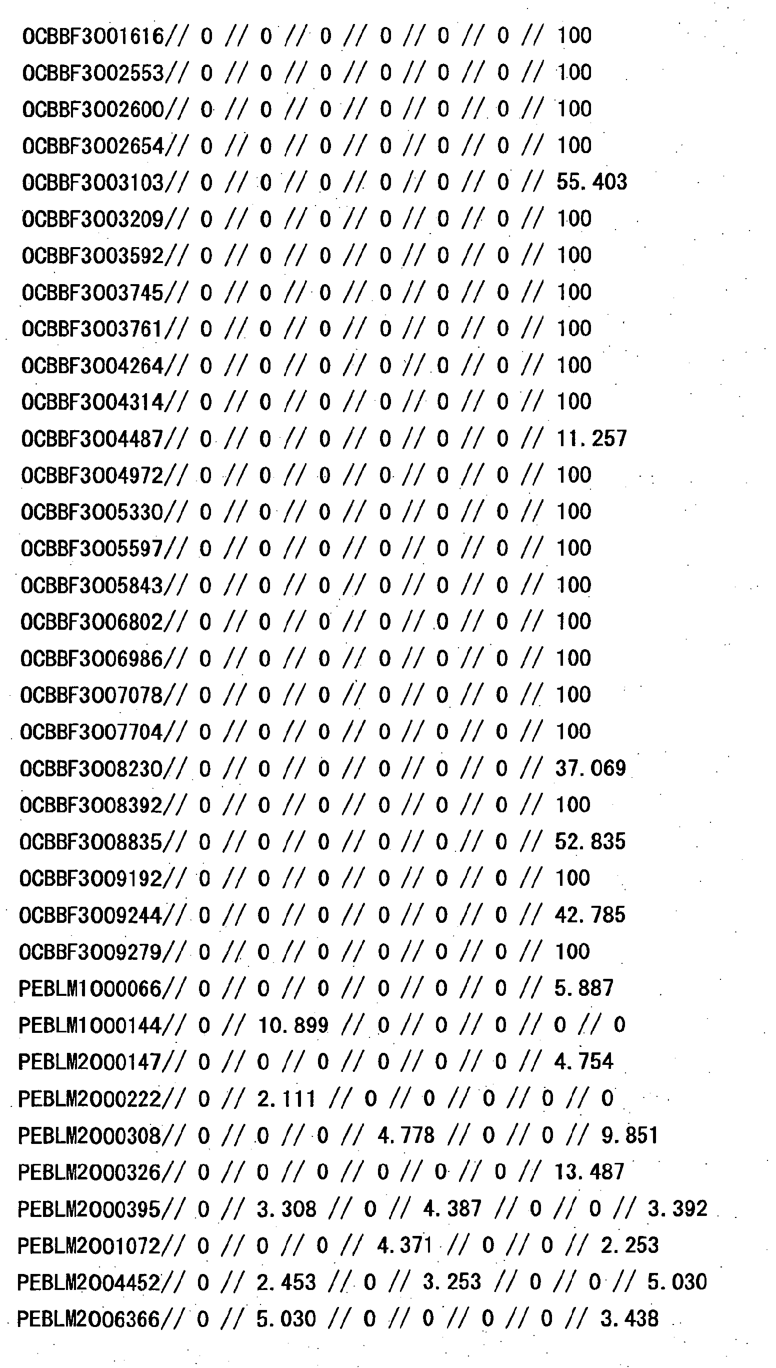

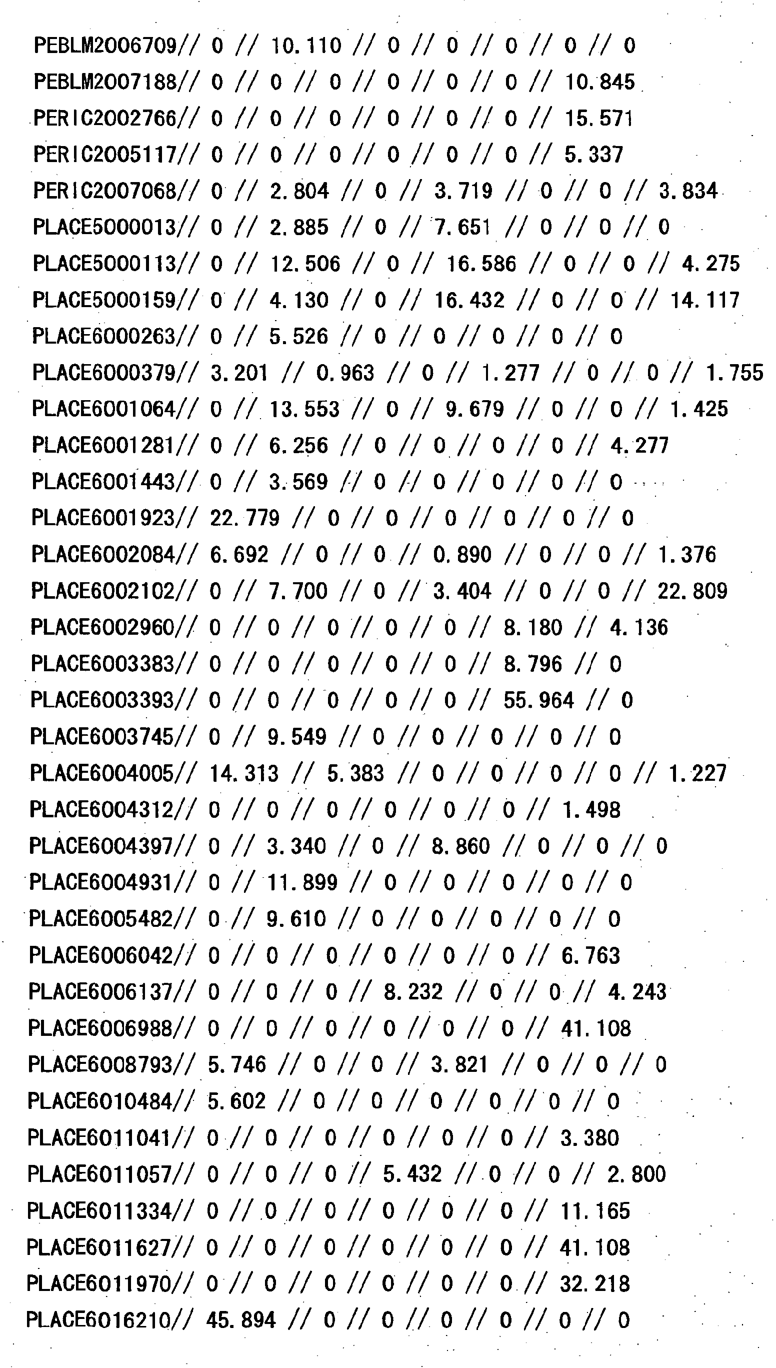

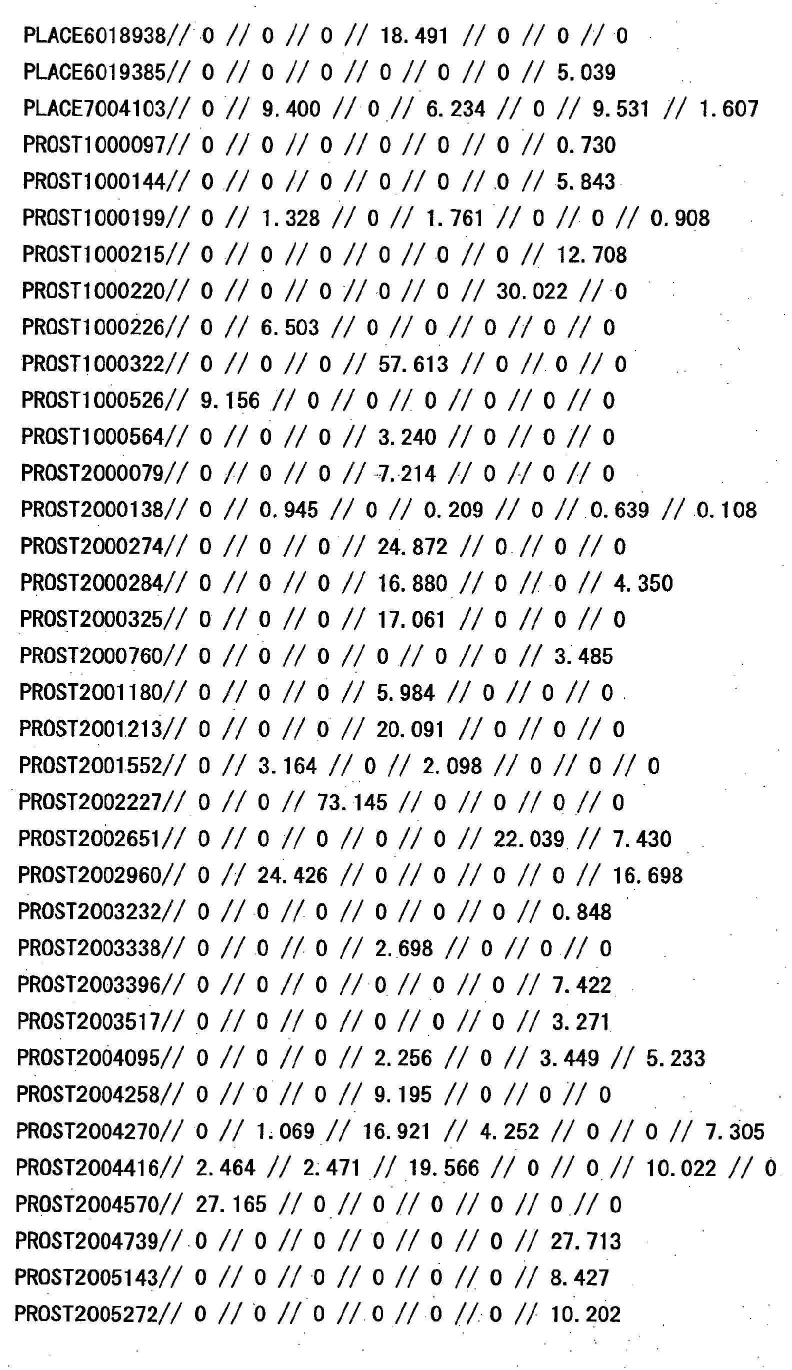

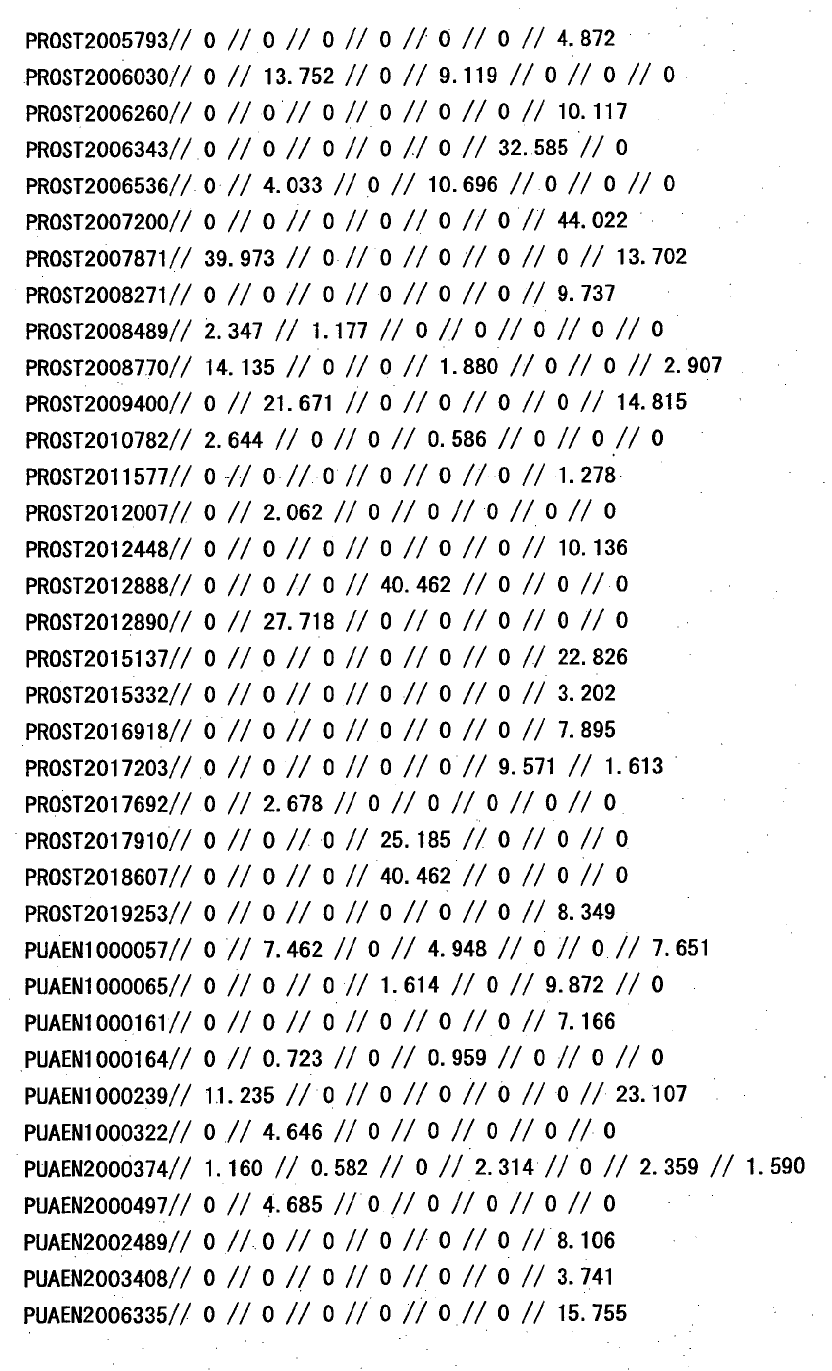

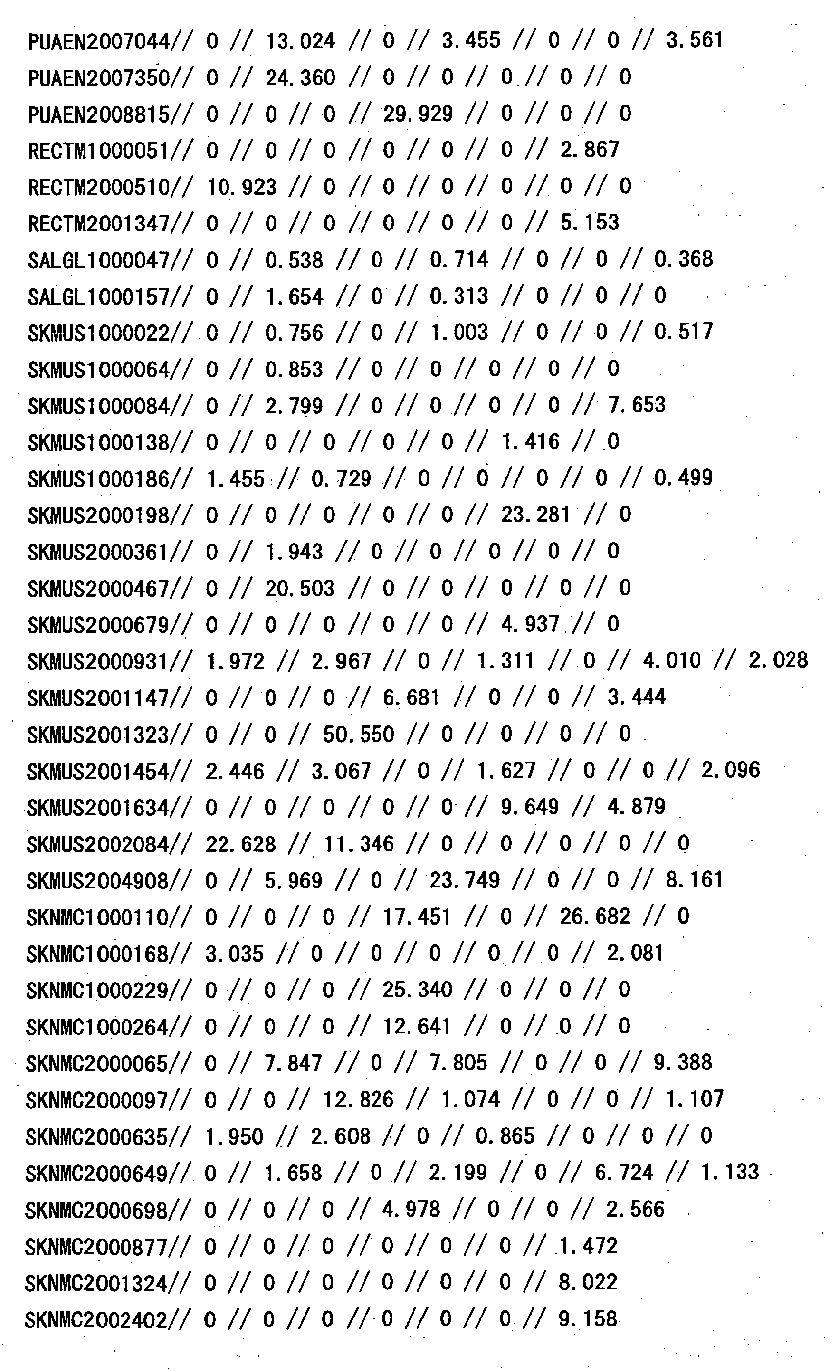

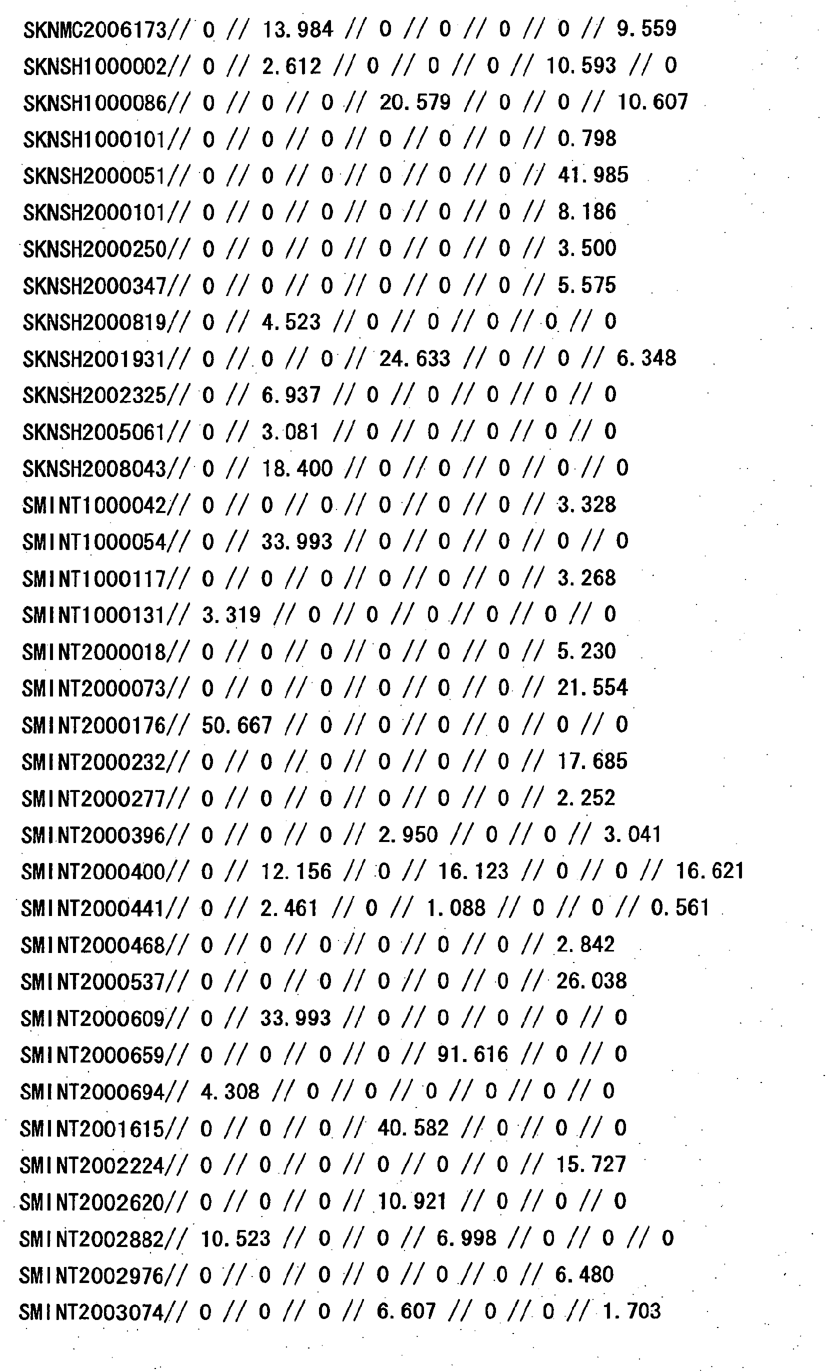

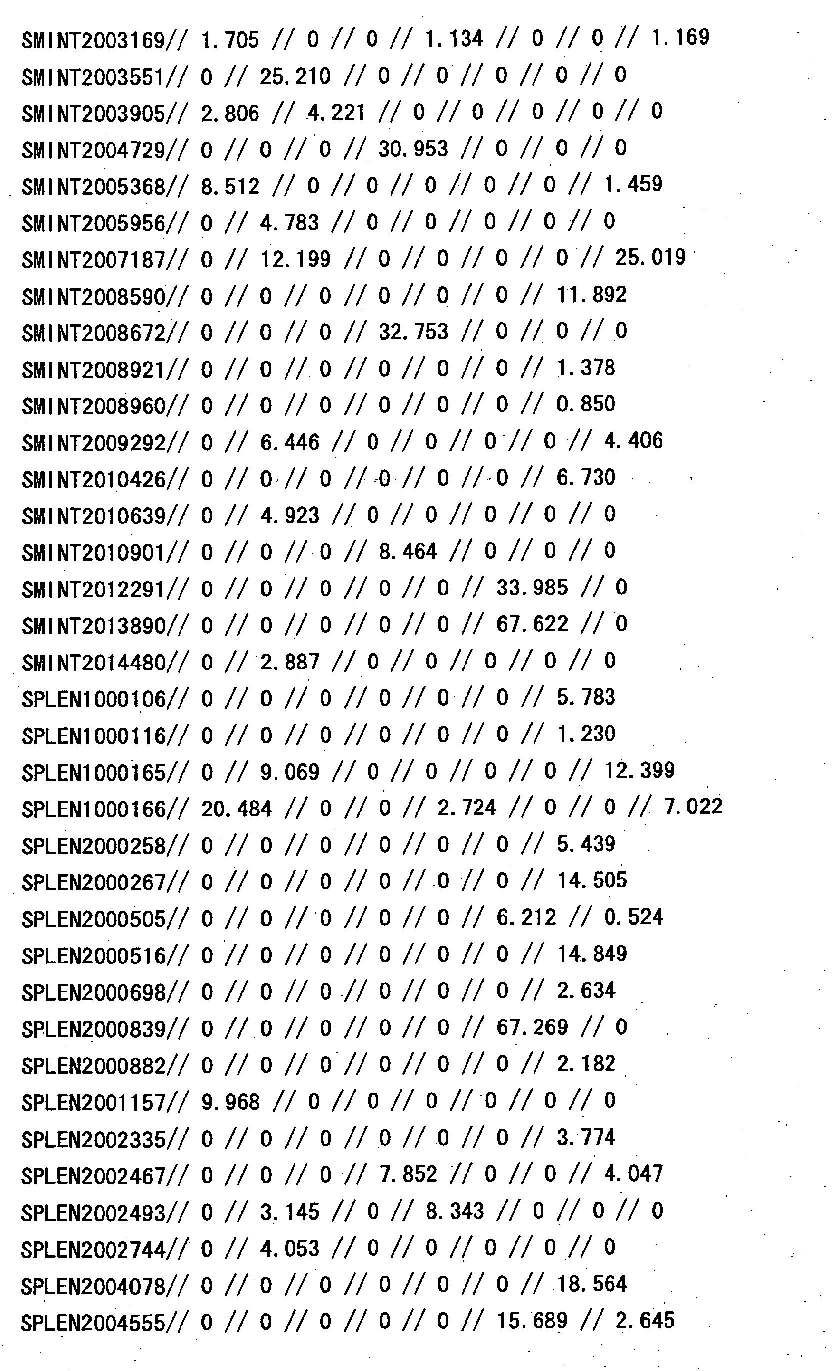

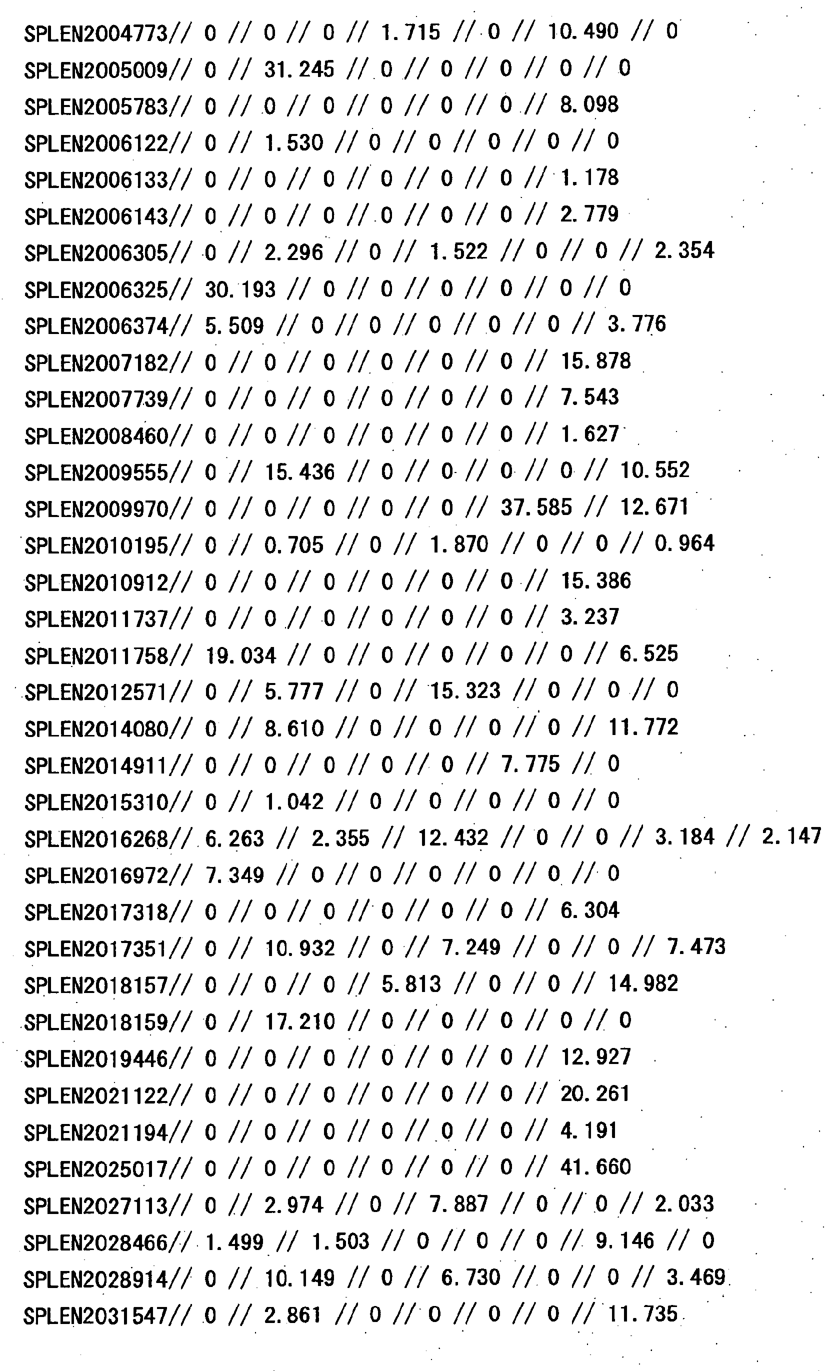

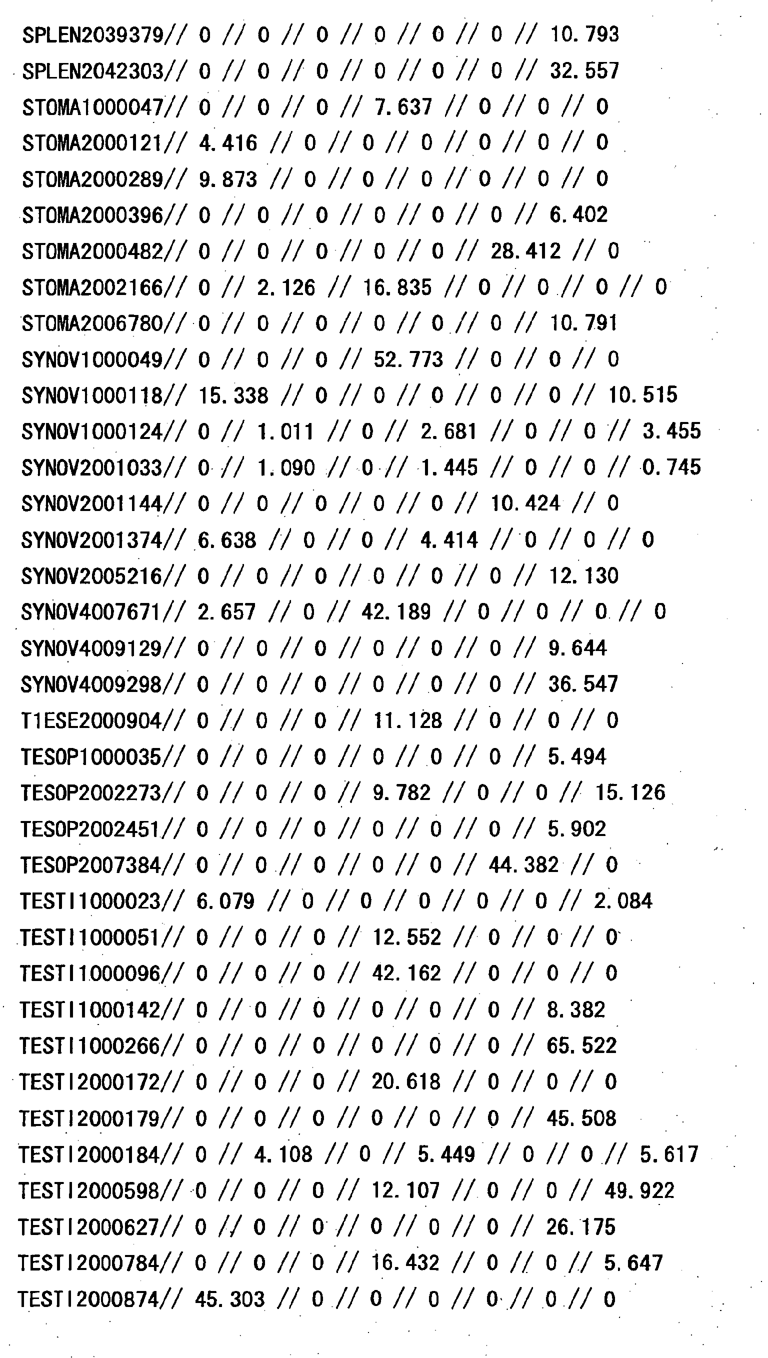

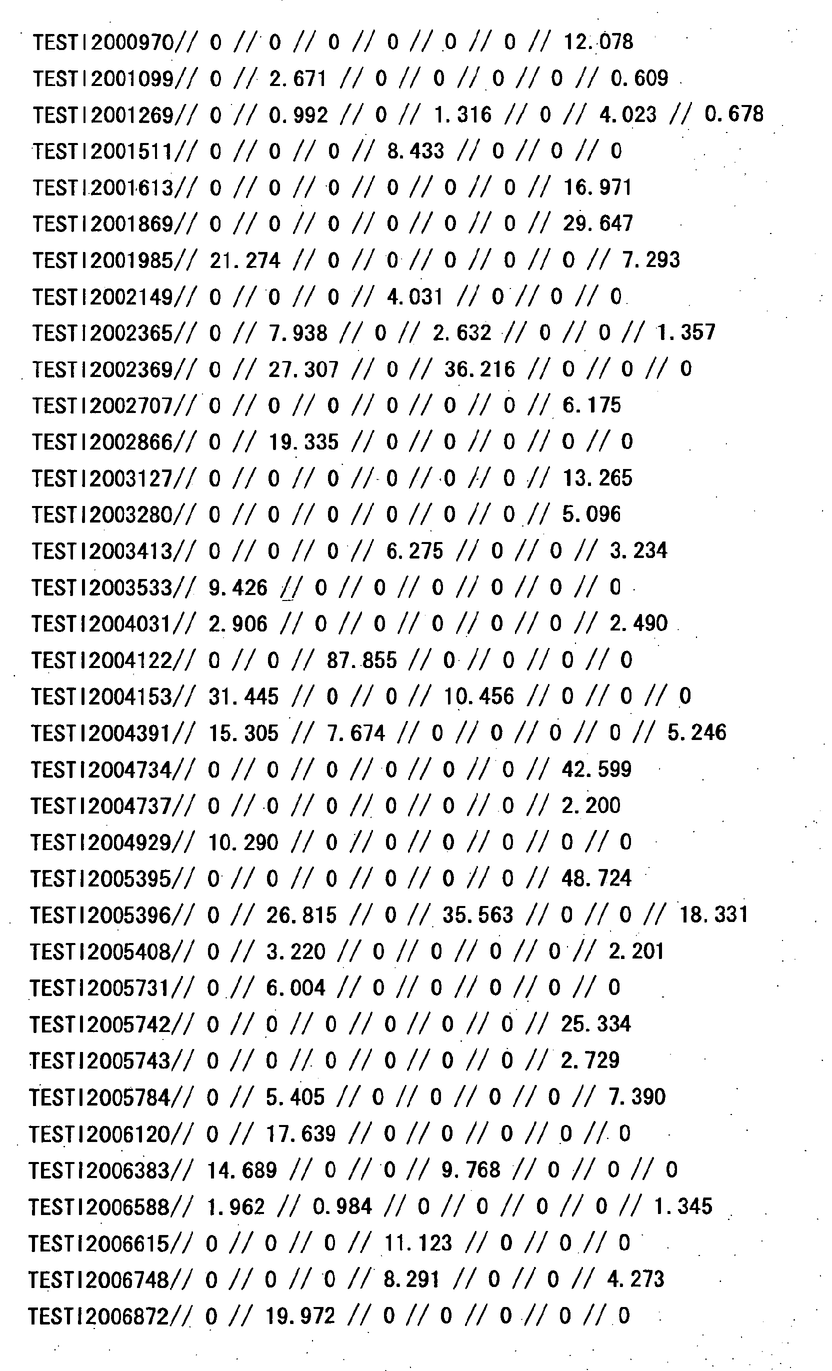

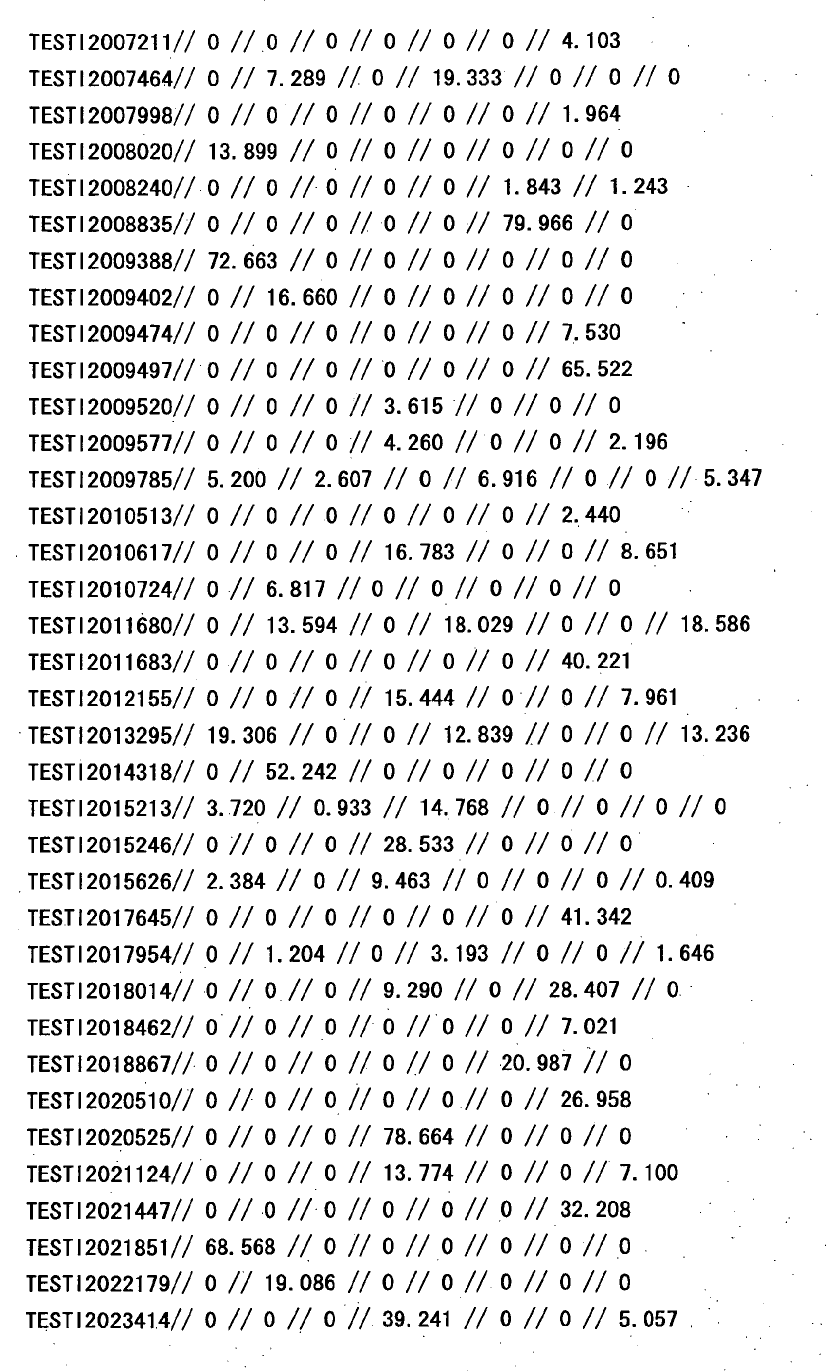

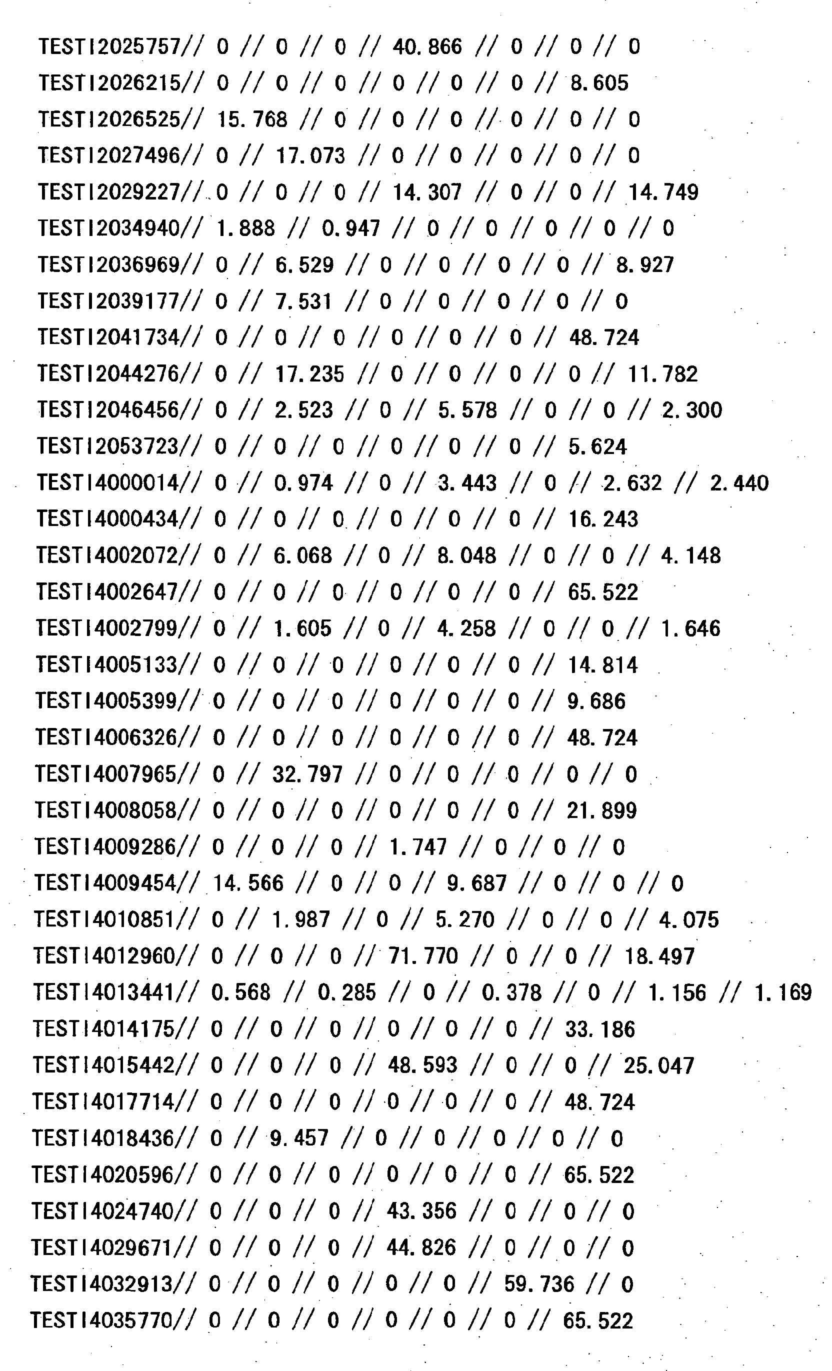

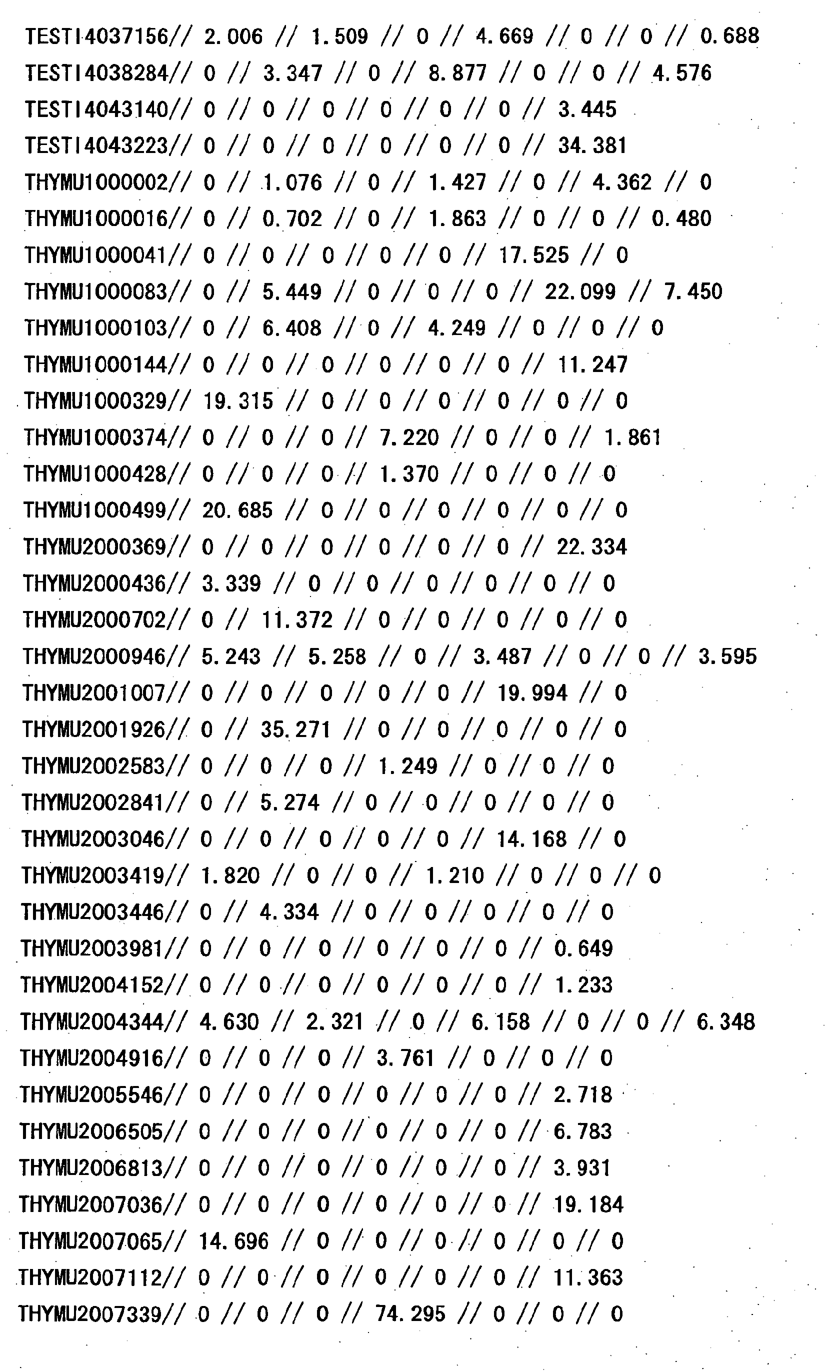

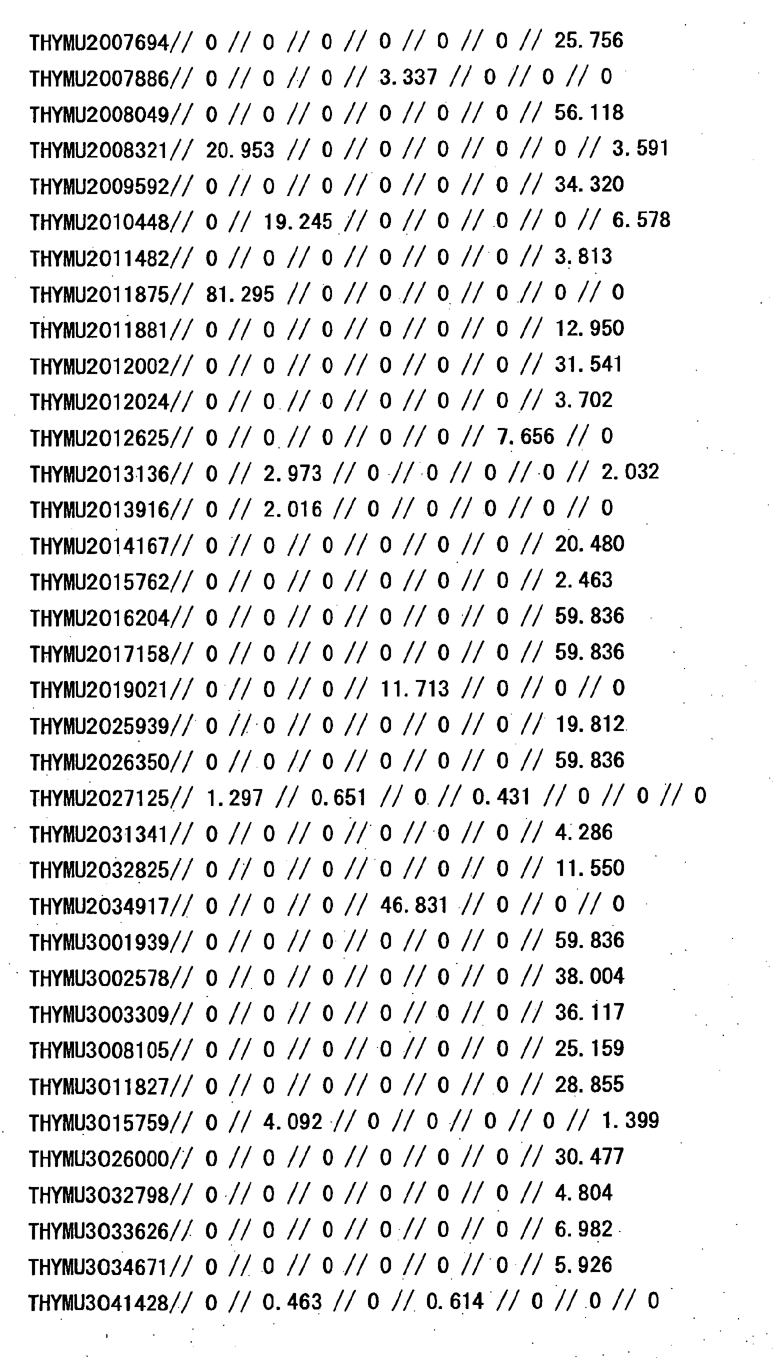

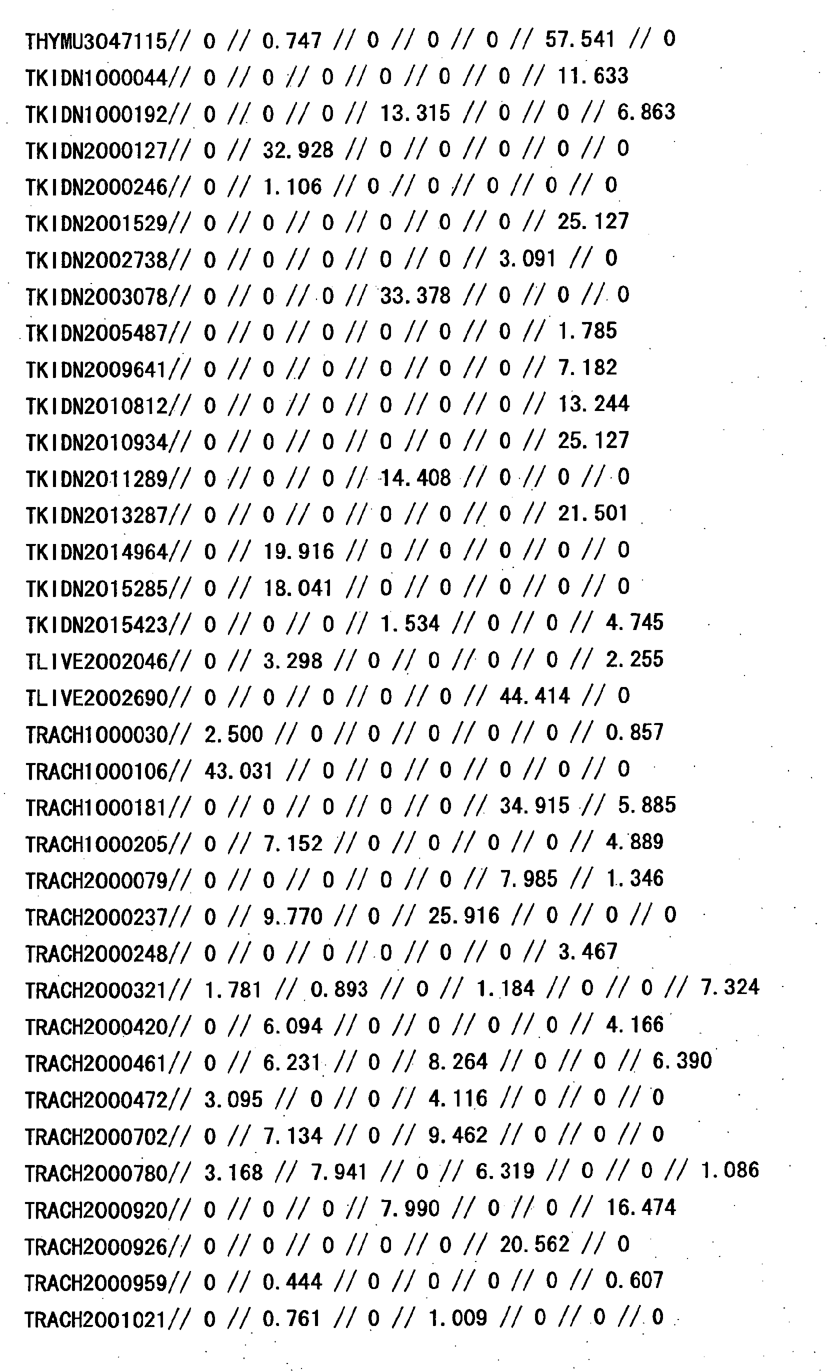

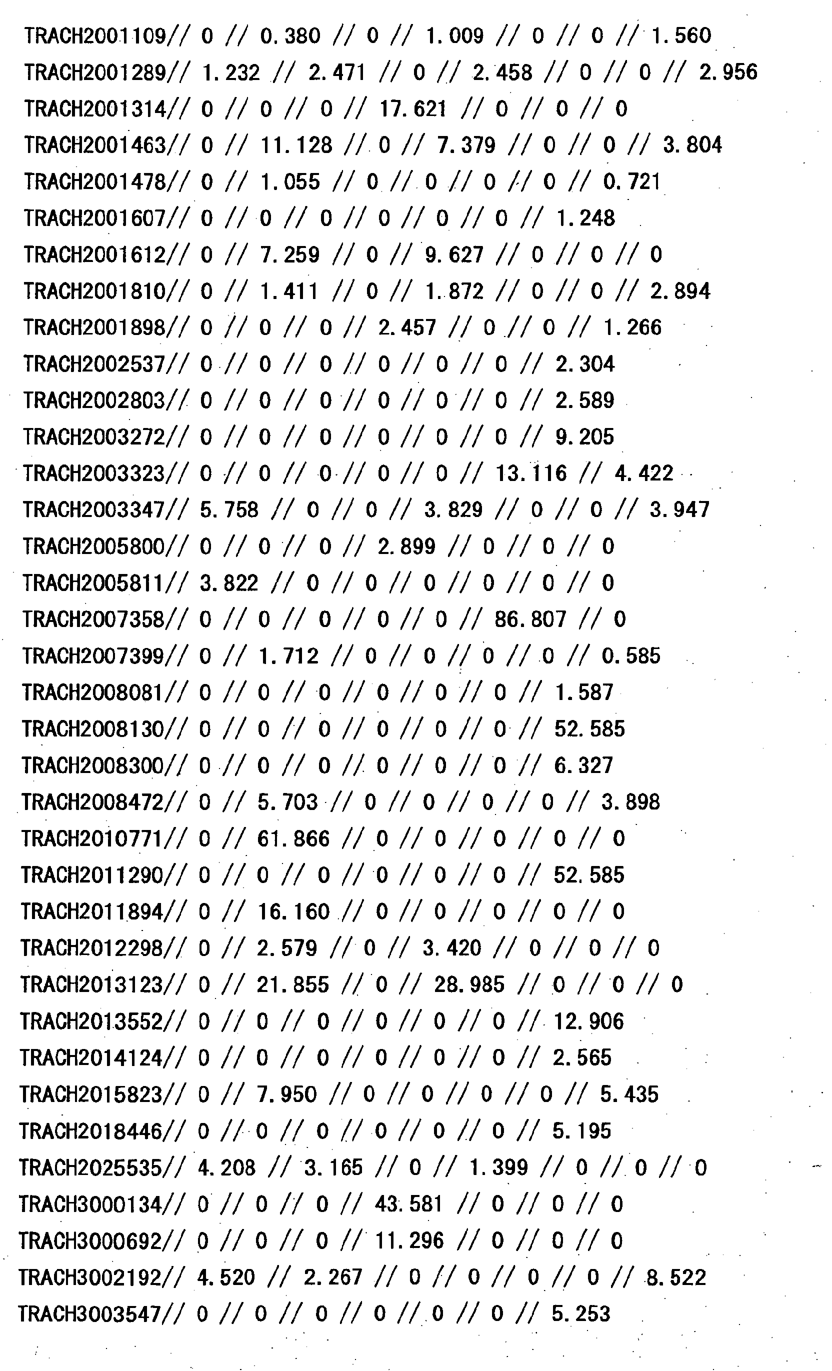

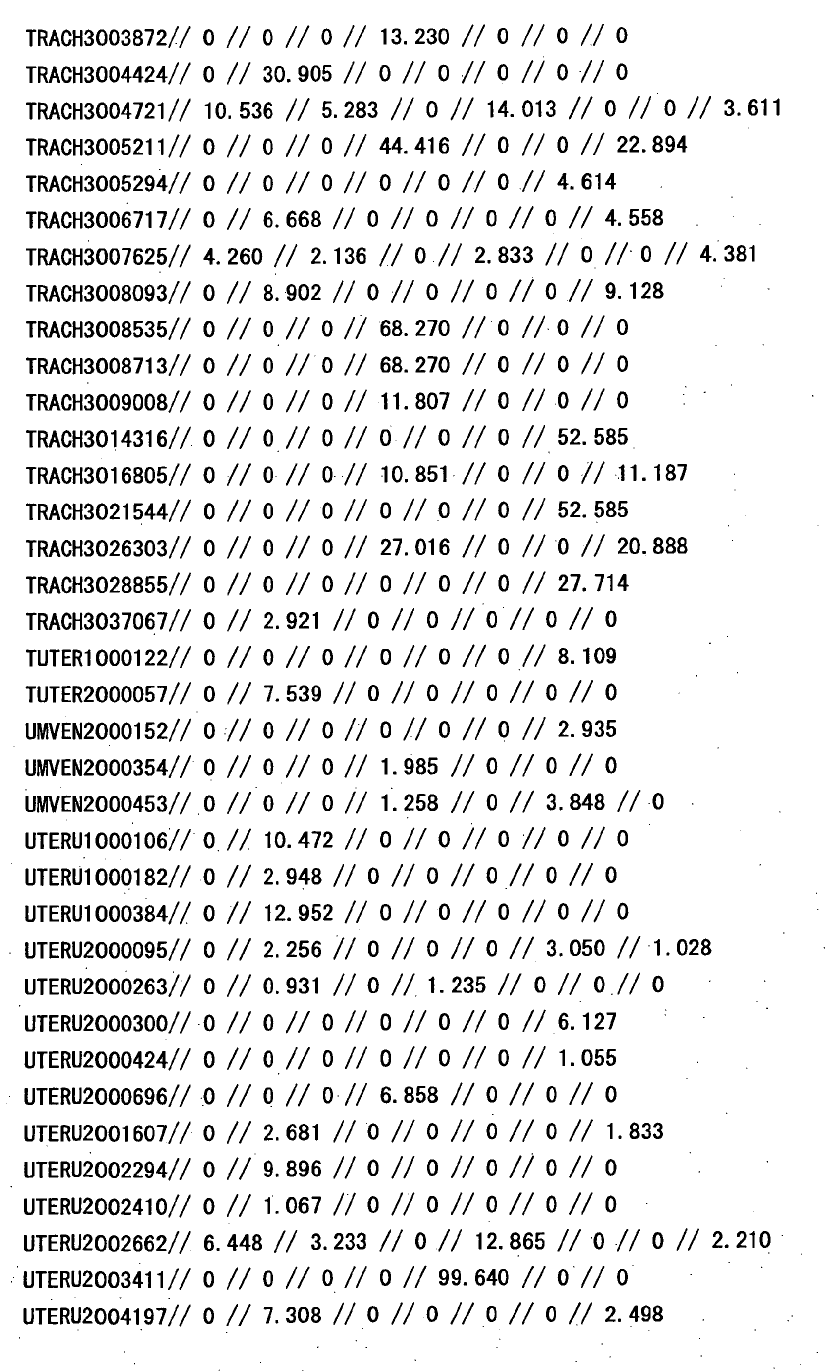

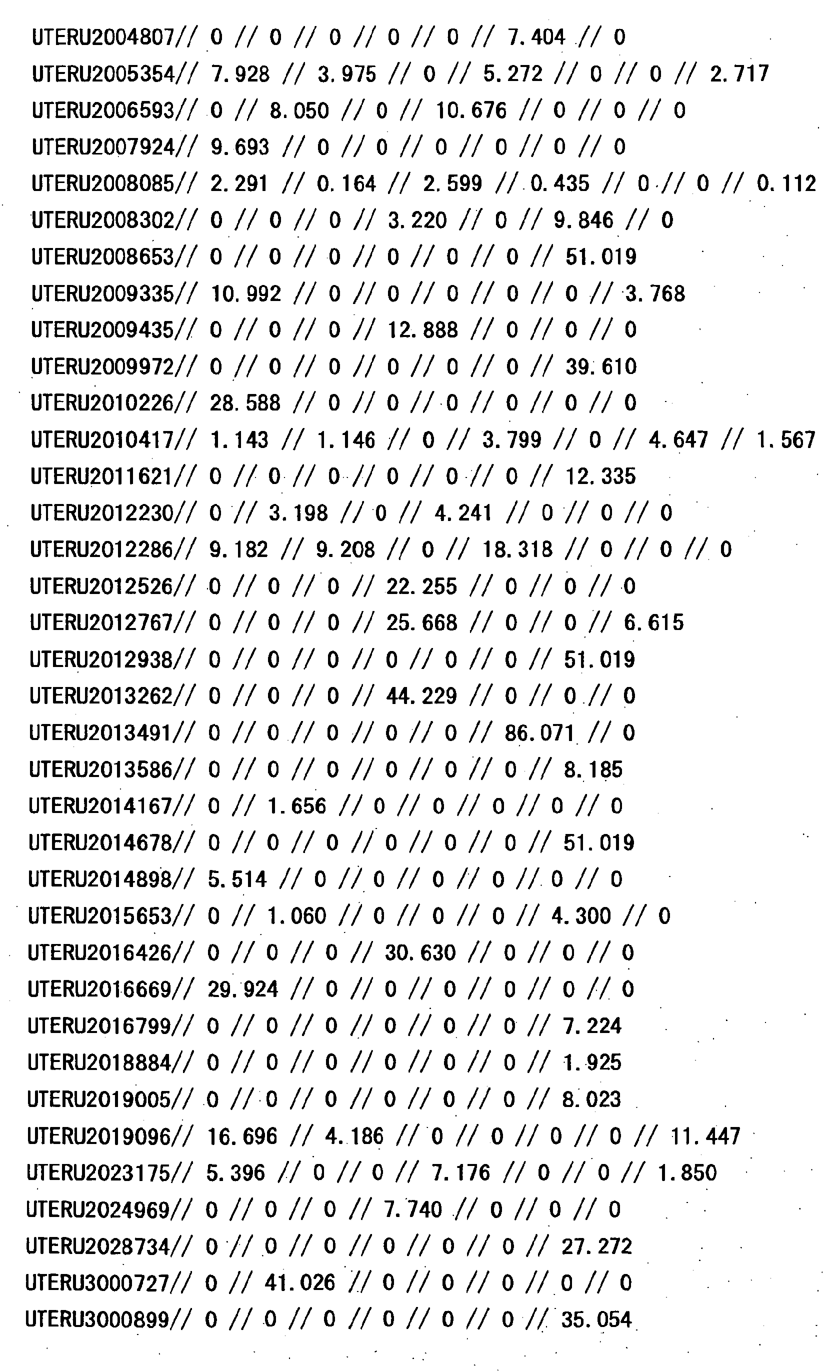

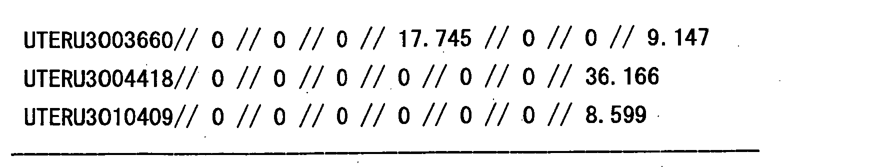

Genes whose expression levels were negligibly low in each of the above-described libraries are shown with their clone names below.
3NB691000018, 3NB691000085, 3NB691000113, 3NB691000191, 3NB692000012, 3NB692000029, 3NB692000102, 3NB692000154, 3NB692000276, 3NB692000305, 3NB692000330, 3NB692000429, 3NB692000484, 3NB692000529, 3NB692000973, 3NB692001034, 3NB692001040, 3NB692001123, 3NB692001267, 3NB692001339, 3NB692001349, 3NB692001366, 3NB692001408, 3NB692001433, 3NB692001459, 3NB692001471, 3NB692001501, 3NB692001507, 3NB692001511, 3NB692001519, 3NB692001520, 3NB692001538, 3NB692001548, 3NB692001557, 3NB692001637, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064,
ADIPS2000069, ADIPS2000088, ADIPS2000245, ADRGL1000018, ADRGL1000033, ADRGL1000038, ADRGL1000067, ADRGL1000111, ADRGL1000147, ADRGL1000160, ADRGL1000165, ADRGL1000182, ADRGL2000056, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002753, ADRGL2002857, ADRGL2003017, ADRGL2003329, ADRGL2003552,
ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146,
ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, AHMSC1000138, ASTRO1000009, ASTRO1000018, ASTRO1000029, ASTRO1000096, ASTRO2000011, ASTRO2000014, ASTRO2000095, ASTRO2000141, ASTRO2000191, ASTRO2000278, ASTRO2000372, ASTRO2000403, ASTRO2000417, ASTRO2000482, ASTRO2000533, ASTRO2000653, ASTRO2000662, ASTRO2000725, ASTRO2001001, ASTRO2001008, ASTRO2001088, ASTRO2001122, ASTRO2001227, ASTRO2001249, ASTRO2001480, ASTRO2001806, ASTRO2002024, ASTRO2002035, ASTRO2002064, ASTRO2002459, ASTRO2002571, ASTRO2002632, ASTRO2002659, ASTRO2002720, ASTRO2002733, ASTRO2002743,
ASTRO2002842, ASTRO2003024, ASTRO2003212, ASTRO2003574, ASTRO2003581, ASTRO2003740, ASTRO2003840, ASTRO2003925, ASTRO2003960, ASTRO2004051, ASTRO2004177, ASTRO2004217, ASTRO2004297, ASTRO2004584, ASTRO2004628, ASTRO2004751, ASTRO2004877, ASTRO2005008, ASTRO2005081, ASTRO2005096, ASTRO2005242, ASTRO2005273, ASTRO2005343, ASTRO2005360, ASTRO2005413, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007047, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008239,
ASTRO2008425, ASTRO2008508, ASTRO2008777, ASTRO2008895, ASTRO2008960, ASTRO2008972, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2011149, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015328, ASTRO2015987, ASTRO2016044, ASTRO2016114, ASTRO2016681, ASTRO2016847, ASTRO2016892, ASTRO2017348, ASTRO2017627, ASTRO2018169, ASTRO2018373, ASTRO2018540,
ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000544, BGGI12000693, BGGI12001241, BGGI12001247, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972,
BLADE2003474, BLADE2003916, BLADE2004345, BLADE2004389, BLADE2004462, BLADE2004628, BLADE2004670, BLADE2004722, BLADE2005036, BLADE2005038, BLADE2005229, BLADE2005459, BLADE2005792, BLADE2006043, BLADE2006412, BLADE2006415, BLADE2006607, BLADE2006826, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008479, BLADE2008539, BLADE2008809, BLADE2008995, BLADE2009452, BLADE2009479, BLADE2009593, BNGH41000104, BNGH41000118, BNGH41000169, BNGH41000177, BNGH41000190, BNGH41000216, BNGH41000235, BNGH42000130, BNGH42000132,
BNGH42000405, BNGH42000532, BNGH42000675, BNGH42000791, BNGH42000815, BNGH42000994, BNGH42001247, BNGH42001406, BNGH42001576, BNGH42001724, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006226, BNGH42006234, BNGH42007037, BNGH42007366, BNGH42007594, BNGH42008199, BNGH42008510, BNGH42008649, BNGH42008832, BNGH42008850, BNGH42008943, BNGH42009021, BRACE1000047, BRACE1000051, BRACE1000065, BRACE1000073, BRACE1000084, BRACE1000092, BRACE1000093, BRACE1000101, BRACE1000114, BRACE1000124, BRACE1000132, BRACE1000159, BRACE1000166,
BRACE1000169, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000331, BRACE2000351, BRACE2000368, BRACE2000392, BRACE2000441, BRACE2000489, BRACE2000505, BRACE2000526, BRACE2000531, BRACE2000545, BRACE2000553, BRACE2000565, BRACE2000577, BRACE2000640, BRACE2000650, BRACE2000677, BRACE2000718, BRACE2000743, BRACE2000750, BRACE2000753, BRACE2000815, BRACE2000864, BRACE2000885, BRACE2000893, BRACE2000905, BRACE2000936, BRACE2000947, BRACE2000965,
BRACE2000969, BRACE2000988, BRACE2001017, BRACE2001065, BRACE2001070, BRACE2001094, BRACE2001117, BRACE2001154, BRACE2001188, BRACE2001191, BRACE2001197, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001492, BRACE2001496, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737, BRACE2001742, BRACE2001777, BRACE2001779, BRACE2001834, BRACE2001859, BRACE2001865, BRACE2001872, BRACE2001898,
BRACE2001923, BRACE2001942, BRACE2001954, BRACE2001972, BRACE2001973, BRACE2002026, BRACE2002034, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002392, BRACE2002394, BRACE2002409, BRACE2002431, BRACE2002444, BRACE2002458, BRACE2002467, BRACE2002468, BRACE2002478, BRACE2002495, BRACE2002585, BRACE2002589, BRACE2002590, BRACE2002606, BRACE2002613, BRACE2002614, BRACE2002617, BRACE2002623, BRACE2002635, BRACE2002661, BRACE2002676, BRACE2002682, BRACE2002685, BRACE2002695, BRACE2002707, BRACE2002736, BRACE2002752, BRACE2002755, BRACE2002762, BRACE2002772, BRACE2002792,
BRACE2002807, BRACE2002812, BRACE2002834, BRACE2002849, BRACE2002857, BRACE2002860, BRACE2002884, BRACE2002896, BRACE2002948, BRACE2003078, BRACE2003097, BRACE2003168, BRACE2003199, BRACE2003210, BRACE2003217, BRACE2003269, BRACE2003285, BRACE2003398, BRACE2003420, BRACE2003428, BRACE2003431, BRACE2003512, BRACE2003516, BRACE2003539, BRACE2003540, BRACE2003594, BRACE2003598, BRACE2003614, BRACE2003639, BRACE2003700, BRACE2003713, BRACE2003766, BRACE2003788, BRACE2003800, BRACE2003802, BRACE2003825, BRACE2003847, BRACE2003848, BRACE2003873, BRACE2003885, BRACE2003887, BRACE2003928, BRACE2003954, BRACE2003982, BRACE2003987, BRACE2005034, BRACE2005047, BRACE2005063, BRACE2005083, BRACE2005090,
BRACE2005099, BRACE2005118, BRACE2005124, BRACE2005138, BRACE2005160, BRACE2005169, BRACE2005193, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005408, BRACE2005433, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005731, BRACE2005742, BRACE2005762, BRACE2005773, BRACE2005787, BRACE2005795, BRACE2005800, BRACE2005858, BRACE2005869, BRACE2005881, BRACE2005898, BRACE2005904, BRACE2005906, BRACE2005907, BRACE2005911,
BRACE2005949, BRACE2005981, BRACE2005991, BRACE2006072, BRACE2006075, BRACE2006084, BRACE2006089, BRACE2006102, BRACE2006105, BRACE2006122, BRACE2006137, BRACE2006143, BRACE2006167, BRACE2006174, BRACE2006240, BRACE2006245, BRACE2006249, BRACE2006264, BRACE2006274, BRACE2006298, BRACE2006344, BRACE2006363, BRACE2006378, BRACE2006380, BRACE2006393, BRACE2006397, BRACE2006413, BRACE2006417, BRACE2006453, BRACE2006459, BRACE2006488, BRACE2006534, BRACE2006537, BRACE2006540, BRACE2006547, BRACE2006564, BRACE2006587, BRACE2006598, BRACE2006685, BRACE2006703, BRACE2006753, BRACE2006783, BRACE2006821, BRACE2006871, BRACE2006909, BRACE2006911, BRACE2006924, BRACE2006935, BRACE2006944, BRACE2006951,
BRACE2006982, BRACE2006986, BRACE2007013, BRACE2007068, BRACE2007087, BRACE2007109, BRACE2007138, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007411, BRACE2007447, BRACE2007477, BRACE2007502, BRACE2007503, BRACE2007522, BRACE2007527, BRACE2007538, BRACE2007563, BRACE2007567, BRACE2007621, BRACE2007640, BRACE2007646, BRACE2007663, BRACE2007682, BRACE2007685, BRACE2007708, BRACE2007727, BRACE2007749, BRACE2007761, BRACE2007764, BRACE2007767, BRACE2007769, BRACE2007784, BRACE2007786, BRACE2007798, BRACE2007799, BRACE2007801, BRACE2007832, BRACE2007836,
BRACE2007868, BRACE2007882, BRACE2007888, BRACE2007920, BRACE2007944, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008385, BRACE2008399, BRACE2008401, BRACE2008410, BRACE2008420, BRACE2008443, BRACE2008473, BRACE2008480, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881,
BRACE2008883, BRACE2008917, BRACE2008919, BRACE2008941, BRACE2008948, BRACE2008960, BRACE2008999, BRACE2009014, BRACE2009016, BRACE2009031, BRACE2009044, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009311, BRACE2009318, BRACE2009323, BRACE2009366, BRACE2009421, BRACE2009428, BRACE2009434, BRACE2009437, BRACE2009483, BRACE2009517, BRACE2009533, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171,
BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012510, BRACE2012625, BRACE2012653, BRACE2012833, BRACE2012838,
BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014475, BRACE2014657, BRACE2014746, BRACE2014780, BRACE2014818, BRACE2014821, BRACE2014824, BRACE2014854, BRACE2015031, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896,
BRACE2016981, BRACE2017108, BRACE2017124, BRACE2017298, BRACE2017359, BRACE2017397, BRACE2017410, BRACE2017438, BRACE2017580, BRACE2017587, BRACE2017720, BRACE2017844, BRACE2017872, BRACE2017929, BRACE2017934, BRACE2017985, BRACE2017992, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278,
BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021344, BRACE2021433, BRACE2021478, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022394, BRACE2022435, BRACE2022448, BRACE2022450, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744,
BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025710, BRACE2025796, BRACE2025992, BRACE2026131, BRACE2026141, BRACE2026142, BRACE2026293, BRACE2026294, BRACE2026404, BRACE2026675, BRACE2026836, BRACE2026920, BRACE2026971, BRACE2027018, BRACE2027221, BRACE2027312, BRACE2027382, BRACE2027389, BRACE2027408, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849,
BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035381, BRACE2035441, BRACE2035485, BRACE2036005, BRACE2036096, BRACE2036743, BRACE2036830, BRACE2036834, BRACE2036900,
BRACE2037132, BRACE2037294, BRACE2037299, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264,. BRACE2041890, BRACE2041898, BRACE2042394, BRACE2042398, BRACE2042550, BRACE2042620, BRACE2042628., BRACE2042873, BRACE2043036, BRACE2043105, BRACE2043142, BRACE2043248,
BRACE2043349, BRACE2043381, BRACE2043774, BRACE2043942, BRACE2044057, BRACE2044105, BRACE2044263, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001217, BRACE3001299, BRACE3001384, BRACE3001391, BRACE3001403, BRACE3001595, BRACE3001754,
BRACE3001948, BRACE3002298, BRACE3002344, BRACE3002390, BRACE3002420, BRACE3002508, BRACE3002541, BRACE3002756, BRACE3003004, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553,
BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416,
BRACE3010435, BRACE3010438, BRACE3010702, BRACE3010862, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308,
BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456,
BRACE3026735, BRACE3026802, BRACE3026844, BRACE3026947, BRACE3026993, BRACE3027242, BRACE3027256, BRACE3027326, BRACE3027478, BRACE3027480, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156,
BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609,
BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696,
BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000388, BRAMY2000411, BRAMY2000508, BRAMY2000585,
BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926,
BRAMY2003929, BRAMY2004031, BRAMY2004058, BRAMY2004183, BRAMY2004335, BRAMY2004351, BRAMY2004352, BRAMY2004387, BRAMY2004492, BRAMY2004521, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308,
BRAMY2007411, BRAMY2007458, BRAMY2007486, BRAMY2007546, BRAMY2007594, BRAMY2007610, BRAMY2007653, BRAMY2007753, BRAMY2007859, BRAMY2008333, BRAMY2008563, BRAMY2008583, BRAMY2008977, BRAMY2008979, BRAMY2009023, BRAMY2009034, BRAMY2009105, BRAMY2009123, BRAMY2009192, BRAMY2009344, BRAMY2009349, BRAMY2009394, BRAMY2009489, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010366, BRAMY2010395, BRAMY2010441, BRAMY2010442, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253,
BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012691, BRAMY2012721, BRAMY2012731, BRAMY2012776, BRAMY2012880, BRAMY2012881, BRAMY2013200, BRAMY2013216, BRAMY2013405, BRAMY2013414, BRAMY2013572, BRAMY2013590, BRAMY2013722, BRAMY2013736, BRAMY2013944, BRAMY2013975, BRAMY2014019, BRAMY2014387, BRAMY2014462, BRAMY2014469, BRAMY2014541, BRAMY2014754, BRAMY2014813, BRAMY2015211, BRAMY2015311, BRAMY2015420, BRAMY2015503, BRAMY2015516,
BRAMY2015550, BRAMY2015551, BRAMY2015559, BRAMY2015688, BRAMY2015782, BRAMY2015833, BRAMY2015855, BRAMY2015890, BRAMY2015911, BRAMY2015925, BRAMY2016002, BRAMY2016020, BRAMY2016070, BRAMY2016325, BRAMY2016327, BRAMY2016386, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2019055, BRAMY2019111, BRAMY2019277, BRAMY2019300, BRAMY2019468, BRAMY2019509, BRAMY2019643, BRAMY2019710, BRAMY2019985, BRAMY2019989, BRAMY2020050,
BRAMY2020058, BRAMY2020270, BRAMY2020354, BRAMY2020355, BRAMY2020412, BRAMY2020427, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172,
BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538,
BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027714, BRAMY2027717, BRAMY2027739, BRAMY2027752, BRAMY2027782, BRAMY2028072, BRAMY2028183, BRAMY2028188, BRAMY2028472, BRAMY2028491, BRAMY2028516, BRAMY2028565, BRAMY2028593, BRAMY2028682, BRAMY2028783, BRAMY2028856, BRAMY2028914, BRAMY2029204, BRAMY2029602, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442,
BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036567, BRAMY2036615, BRAMY2036699, BRAMY2036913, BRAMY2036918, BRAMY2036968,
BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760,
BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458,
BRAMY3002508, BRAMY3002620, BRAMY3002803, BRAMY3002805, BRAMY3002886, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005656, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492,
BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRASW1000053, BRASW1000125, BRAWH1000001, BRAWH1000002, BRAWH1000083, BRAWH1000094, BRAWH1000116, BRAWH1000130, BRAWH1000157, BRAWH1000164, BRAWH1000168, BRAWH1000174, BRAWH1000180, BRAWH1000369, BRAWH2000034, BRAWH2000048, BRAWH2000071, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000323, BRAWH2000353, BRAWH2000370, BRAWH2000409, BRAWH2000417, BRAWH2000443, BRAWH2000471,
BRAWH2000476, BRAWH2000482, BRAWH2000494, BRAWH2000500, BRAWH2000503, BRAWH2000554, BRAWH2000595, BRAWH2000643, BRAWH2000651, BRAWH2000682, BRAWH2000752, BRAWH2000859, BRAWH2000866, BRAWH2000892, BRAWH2000901, BRAWH2000926, BRAWH2000984, BRAWH2001019, BRAWH2001052, BRAWH2001103, BRAWH2001129, BRAWH2001137, BRAWH2001159, BRAWH2001203, BRAWH2001238, BRAWH2001239, BRAWH2001255, BRAWH2001355, BRAWH2001378, BRAWH2001395, BRAWH2001406, BRAWH2001418, BRAWH2001436, BRAWH2001438, BRAWH2001459, BRAWH2001461, BRAWH2001492, BRAWH2001503, BRAWH2001519, BRAWH2001535, BRAWH2001589, BRAWH2001666, BRAWH2001671, BRAWH2001679, BRAWH2001686, BRAWH2001701, BRAWH2001842, BRAWH2001873, BRAWH2001940, BRAWH2001973,
BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002601, BRAWH2002623, BRAWH2002630, BRAWH2002635, BRAWH2002662, BRAWH2002679, BRAWH2002725, BRAWH2002761, BRAWH2002861, BRAWH2002911, BRAWH2002963, BRAWH2002976, BRAWH2003000, BRAWH2003025, BRAWH2003135, BRAWH2003240, BRAWH2003307, BRAWH2003355, BRAWH2003366, BRAWH2003689, BRAWH2003832, BRAWH2003948, BRAWH2003964, BRAWH2004044, BRAWH2004068, BRAWH2004078, BRAWH2004136, BRAWH2004217, BRAWH2004580, BRAWH2004731, BRAWH2004779, BRAWH2004842, BRAWH2004884, BRAWH2004923; BRAWH2005008, BRAWH2005068, BRAWH2005074, BRAWH2005315, BRAWH2005524, BRAWH2005533,
BRAWH2005578, BRAWH2005812, BRAWH2005974, BRAWH2005998, BRAWH2006083, BRAWH2006207, BRAWH2006301, BRAWH2006395, BRAWH2006405, BRAWH2006450, BRAWH2006493, BRAWH2006526, BRAWH2006543, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007658, BRAWH2007808, BRAWH2007825, BRAWH2007862, BRAWH2007890, BRAWH2008128, BRAWH2008219, BRAWH2008292, BRAWH2008297, BRAWH2008366, BRAWH2008380, BRAWH2008540, BRAWH2008577, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009360, BRAWH2009393,
BRAWH2009485, BRAWH2009490, BRAWH2009558, BRAWH2009590, BRAWH2009678, BRAWH2009695, BRAWH2009907, BRAWH2010000, BRAWH2010069, BRAWH2010084, BRAWH2010136, BRAWH2010318, BRAWH2010354, BRAWH2010364, BRAWH2010536, BRAWH2010552, BRAWH2010584, BRAWH2010618, BRAWH2010619, BRAWH2011066, BRAWH2011079, BRAWH2011096, BRAWH2011155, BRAWH2011166, BRAWH2011204, BRAWH2011294, BRAWH2011343, BRAWH2011400, BRAWH2011762, BRAWH2011795, BRAWH2011796, BRAWH2011812, BRAWH2011860, BRAWH2011958, BRAWH2012054, BRAWH2012077, BRAWH2012161, BRAWH2012162, BRAWH2012258, BRAWH2012277, BRAWH2012326, BRAWH2012538, BRAWH2012540, BRAWH2012619, BRAWH2012698, BRAWH2012749, BRAWH2012827, BRAWH2012963, BRAWH2013047, BRAWH2013219,
BRAWH2013294, BRAWH2013748, BRAWH2013866, BRAWH2013871, BRAWH2013914, BRAWH2013941, BRAWH2013955, BRAWH2014053, BRAWH2014234, BRAWH2014414, BRAWH2014473, BRAWH2014511, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016514, BRAWH2016562, BRAWH2016655, BRAWH2016679, BRAWH2016702, BRAWH2016724, BRAWH2016785, BRAWH2016792, BRAWH2016800, BRAWH2017103, BRAWH2017250, BRAWH2017294, BRAWH2017305, BRAWH2017379, BRAWH2017407,
BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3002433, BRAWH3002467, BRAWH3002513, BRAWH3002600, BRAWH3002819, BRAWH3003019, BRAWH3003136, BRAWH3003244,
BRAWH3003343, BRAWH3003411, BRAWH3003522, BRAWH3003573, BRAWH3003598, BRAWH3003801, BRAWH3003975, BRAWH3004335, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3009017, BRAWH3009285, BRAWH3009297, BRAWH3009961, BRAWH3010560, BRAWH3010602, BRAWH3010657, BRAWH3010726, BRAWH3010833, BRAWH3011101, BRAWH3011331,
BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014639, BRAWH3015017, BRAWH3015175, BRAWH3015260, BRAWH3015610, BRAWH3015825, BRAWH3016123, BRAWH3016271, BRAWH3016715, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431, BRAWH3022459, BRAWH3022542,
BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620,
BRAWH3032930, BRAWH3033117, BRAWH3033293, BRAWH3033448, BRAWH3033513, BRAWH3034097, BRAWH3034114, BRAWH3034134, BRAWH3034668, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000577, BRCAN2000620, BRCAN2000639, BRCAN2000763, BRCAN2000832, BRCAN2000923, BRCAN2001223,
BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203,
BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387,
BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027513, BRCAN2027593, BRCAN2027970, BRCAN2028021, BRCAN2028040, BRCAN2028319, BRCAN2028338, BRCAN2028378, BRCAN2028460, BRCAN2028545, BRCAN2028634, BRCAN2028702, BRCOC1000087, BRCOC1000094, BRCOC2000004,
BRCOC2000047, BRCOC2000186, BRCOC2000333, BRCOC2000360, BRCOC2000404, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052,
BRCOC2009196, BRCOC2009380, BRCOC2009638, BRCOC2009937, BRCOC2010115, BRCOC2010730, BRCOC2010980, BRCOC2010994, BRCOC2011010, BRCOC2011398, BRCOC2011418, BRCOC2011506, BRCOC2011769, BRCOC2011996, BRCOC2012229, BRCOC2012551, BRCOC2012813, BRCOC2013448, BRCOC2013573, BRCOC2013675, BRCOC2013780, BRCOC2014013, BRCOC2014033, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019934, BRCOC2020122, BRCOC2020142, BRHIP1000058, BRHIP1000072, BRHIP1000129, BRHIP1000258, BRHIP2000021, BRHIP2000079,
BRHIP2000087, BRHIP2000163, BRHIP2000210, BRHIP2000239, BRHIP2000245, BRHIP2000359, BRHIP2000445, BRHIP2000506, BRHIP2000532, BRHIP2000621, BRHIP2000691, BRHIP2000819, BRHIP2000826, BRHIP2000920, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719,
BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360,
BRHIP2012545, BRHIP2012580, BRHIP2012642, BRHIP2012750, BRHIP2012922, BRHIP2012923, BRHIP2012972, BRHIP2013447, BRHIP2013510, BRHIP2013699, BRHIP2013723, BRHIP2013958, BRHIP2013972, BRHIP2014063, BRHIP2014285, BRHIP2014291, BRHIP2014373, BRHIP2014386, BRHIP2014387, BRHIP2014954, BRHIP2015153, BRHIP2015356, BRHIP2015360, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019149, BRHIP2019177,
BRHIP2019186, BRHIP2019291, BRHIP2019494, BRHIP2019589, BRHIP2019641, BRHIP2019884, BRHIP2019972, BRHIP2020182, BRHIP2020509, BRHIP2020622, BRHIP2020695, BRHIP2020743, BRHIP2020842, BRHIP2020859, BRHIP2020930, BRHIP2021102, BRHIP2021289, BRHIP2021495, BRHIP2021615, BRHIP2021744, BRHIP2021870, BRHIP2021929, BRHIP2022006, BRHIP2022054, BRHIP2022221, BRHIP2022298, BRHIP2022467, BRHIP2022708, BRHIP2023071, BRHIP2023229, BRHIP2023272, BRHIP2023292, BRHIP2023309, BRHIP2023369, BRHIP2023438, BRHIP2023695, BRHIP2023735, BRHIP2023773, BRHIP2023860, BRHIP2023869, BRHIP2024046, BRHIP2024165, BRHIP2024201, BRHIP2024347, BRHIP2024549, BRHIP2024789., BRHIP2024941, BRHIP2025245, BRHIP2025366, BRHIP2025679,
BRHIP2025739, BRHIP2025797, BRHIP2026021, BRHIP2026155, BRHIP2026214, BRHIP2026288, BRHIP2026346, BRHIP2026557, BRHIP2026723, BRHIP2026877, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP3000060, BRHIP3000070, BRHIP3000081, BRHIP3000240, BRHIP3000377, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002920, BRHIP3002931,
BRHIP3003063, BRHIP3003126, BRHIP3003306, BRHIP3003340, BRHIP3003395, BRHIP3003484, BRHIP3003644, BRHIP3003688, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007609, BRHIP3007640, BRHIP3007960, BRHIP3008183, BRHIP3008314, BRHIP3008344, BRHIP3008405,
BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558,
BRHIP3017637, BRHIP3017855, BRHIP3018278, BRHIP3019643, BRHIP3019824, BRHIP3019880, BRHIP3019956, BRHIP3020046, BRHIP3020155, BRHIP3020182, BRHIP3020733, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244,
BRSSN2000293, BRSSN2000295, BRSSN2000312, BRSSN2000518, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419,
BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010019, BRSSN2010110, BRSSN2010587, BRSSN2010596, BRSSN2010830, BRSSN2011262, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013874, BRSSN2014112, BRSSN2014294, BRSSN2014299, BRSSN2014424, BRSSN2014513, BRSSN2014556, BRSSN2014573, BRSSN2014636, BRSSN2014685, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589,
BRSSN2016768, BRSSN2016905, BRSSN2017297, BRSSN2017422, BRSSN2017530, BRSSN2017682, BRSSN2017757, BRSSN2018218, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475,
BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013171, BRSTN2013354, BRSTN2013502, BRSTN2013931, BRSTN2014490, BRSTN2014633, BRSTN2014751, BRSTN2014770, BRSTN2015015, BRSTN2015978, BRSTN2016066, BRSTN2016335, BRSTN2016470, BRSTN2016678, BRSTN2016892, BRSTN2016918, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021,
BRTHA1000063, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831,
BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183,
BRTHA2012189, BRTHA2012351, BRTHA2012392, BRTHA2012466, BRTHA2012555, BRTHA2012562, BRTHA2012714, BRTHA2012980, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985, BRTHA2018011,
BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517,
BRTHA2031800, BRTHA2031917, BRTHA2032763, BRTHA2032845, BRTHA2033122, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000789, BRTHA3001221, BRTHA3001623, BRTHA3002265, BRTHA3002401, BRTHA3002411, BRTHA3002864, BRTHA3002933, BRTHA3002955, BRTHA3003000, BRTHA3003023, BRTHA3003074, BRTHA3003339, BRTHA3003343, BRTHA3003417, BRTHA3003449, BRTHA3003474, BRTHA3003704, BRTHA3004432, BRTHA3004475, BRTHA3004502, BRTHA3004610,
BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685, BRTHA3007769, BRTHA3008143, BRTHA3008293, BRTHA3008310, BRTHA3008386, BRTHA3008520, BRTHA3008608, BRTHA3008694, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011229, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835,
BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3022641, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152,
CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140,
CORDB2000061, CORDB2000127, CORDB2000541, CTONG1000009, CTONG1000020, CTONG1000022, CTONG1000040, CTONG1000056, CTONG1000062, CTONG1000086, CTONG1000088, CTONG1000093, CTONG1000097, CTONG1000113, CTONG1000137, CTONG1000165, CTONG1000175, CTONG1000180, CTONG1000181, CTONG1000220, CTONG1000277, CTONG1000283, CTONG1000302, CTONG1000341, CTONG1000467, CTONG1000476, CTONG1000488, CTONG1000508, CTONG1000540, CTONG2000031, CTONG2000042, CTONG2000201, CTONG2000218, CTONG2000279, CTONG2000303, CTONG2000330, CTONG2000395, CTONG2000411, CTONG2000465, CTONG2000480, CTONG2000561, CTONG2000589, CTONG2000766, CTONG2000771, CTONG2000819, CTONG2000827, CTONG2000846, CTONG2000855, CTONG2000977, CTONG2001139,
CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001749, CTONG2001800, CTONG2001858, CTONG2001877, CTONG2001955, CTONG2002066, CTONG2002143, CTONG2002395, CTONG2002417, CTONG2002418, CTONG2002453, CTONG2002520, CTONG2002558, CTONG2002590, CTONG2002721, CTONG2002766, CTONG2002816, CTONG2002965, CTONG2002999, CTONG2003028, CTONG2003100, CTONG2003115, CTONG2003189, CTONG2003245, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003937, CTONG2003945,
CTONG2004000, CTONG2004062, CTONG2004115, CTONG2004126, CTONG2004264, CTONG2004305, CTONG2004397, CTONG2004454, CTONG2004550, CTONG2004669, CTONG2004716, CTONG2004912, CTONG2004941, CTONG2004948, CTONG2005049, CTONG2005110, CTONG2005145, CTONG2005265, CTONG2005290, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2005913, CTONG2006129, CTONG2006223, CTONG2006273, CTONG2006310, CTONG2006377, CTONG2006393, CTONG2006515, CTONG2006524, CTONG2006568, CTONG2006611, CTONG2006620, CTONG2006666, CTONG2006691, CTONG2006798, CTONG2006836, CTONG2006932, CTONG2006942, CTONG2006964, CTONG2007007,
CTONG2007009, CTONG2007072, CTONG2007091, CTONG2007104, CTONG2007168, CTONG2007175, CTONG2007259, CTONG2007272, CTONG2007293, CTONG2007297, CTONG2007399, CTONG2007417, CTONG2007441, CTONG2007474, CTONG2007613, CTONG2007623, CTONG2007681, CTONG2007776, CTONG2007779, CTONG2007898, CTONG2007970, CTONG2008014, CTONG2008184, CTONG2008262, CTONG2008269, CTONG2008343, CTONG2008402, CTONG2008405, CTONG2008521, CTONG2008562, CTONG2008577, CTONG2008595, CTONG2008621, CTONG2008689, CTONG2008720, CTONG2008721, CTONG2008773, CTONG2008989, CTONG2009033, CTONG2009132, CTONG2009156, CTONG2009181, CTONG2009257, CTONG2009258, CTONG2009268, CTONG2009270, CTONG2009395, CTONG2009423, CTONG2009440, CTONG2009527,
CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010408, CTONG2010508, CTONG2010623, CTONG2010652, CTONG2010803, CTONG2011188, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012001, CTONG2012029, CTONG2012050, CTONG2012077, CTONG2012101, CTONG2012123,
CTONG2012158, CTONG2012230, CTONG2012401, CTONG2012422, CTONG2012425, CTONG2012447, CTONG2012473, CTONG2012476, CTONG2012533, CTONG2012554, CTONG2012724, CTONG2012738, CTONG2012745, CTONG2012758, CTONG2012847, CTONG2012901, CTONG2013128, CTONG2013149, CTONG2013156, CTONG2013178, CTONG2013222, CTONG2013284, CTONG2013339, CTONG2013348, CTONG2013393, CTONG2013511, CTONG2013630, CTONG2013803, CTONG2013985, CTONG2013986, CTONG2014032, CTONG2014058, CTONG2014119, CTONG2014165, CTONG2014191, CTONG2014206, CTONG2014234, CTONG2014264, CTONG2014389, CTONG2014491, CTONG2014630, CTONG2014635, CTONG2014646, CTONG2014705, CTONG2014946, CTONG2014954, CTONG2014959, CTONG2014966, CTONG2014995, CTONG2015204,
CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016499, CTONG2016505, CTONG2016559, CTONG2016658, CTONG2016775, CTONG2016797, CTONG2016846, CTONG2016869, CTONG2016904, CTONG2016942, CTONG2016953, CTONG2017021, CTONG2017094, CTONG2017292, CTONG2017409, CTONG2017429, CTONG2017458, CTONG2017604, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018217, CTONG2018279,
CTONG2018334, CTONG2018343, CTONG2018379, CTONG2018413, CTONG2018483, CTONG2018629, CTONG2018652, CTONG2018653, CTONG2018655, CTONG2018735, CTONG2018843, CTONG2018900, CTONG2018976, CTONG2019029, CTONG2019063, CTONG2019144, CTONG2019232, CTONG2019248, CTONG2019590, CTONG2019652, CTONG2019704, CTONG2019822, CTONG2019833, CTONG2020031, CTONG2020108, CTONG2020127, CTONG2020171, CTONG2020374, CTONG2020378, CTONG2020445, CTONG2020522, CTONG2020582, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024031, CTONG2024206, CTONG2024614, CTONG2024749, CTONG2025496,
CTONG2025513, CTONG2025516, CTONG2025610, CTONG2025900,. CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002909, CTONG3002947, CTONG3002983, CTONG3003165, CTONG3003179, CTONG3003483, CTONG3003553,
CTONG3003598, CTONG3003652, CTONG3003654, CTONG3003669, CTONG3003737, CTONG3003972, CTONG3004072, CTONG3004317, CTONG3004397, CTONG3004550, CTONG3004576, CTONG3004607, CTONG3004712, CTONG3005325, CTONG3005648, CTONG3005713, CTONG3005803, CTONG3005813, CTONG3006067, CTONG3006073, CTONG3006186, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009287, CTONG3009328, CTONG3009385, D3OST100006, D3OST1000109, D3OST10002, D3OST2000184, D3OST2000618, D3OST2000654, D3OST2000734, D3OST2001328,
D3OST2001669, D3OST2001670, D3OST2001788, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000358, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000083, DFNES1000107,
DFNES1000150, DFNES1000185, DFNES2000011, DFNES2000143, DFNES2000212, DFNES2000268, DFNES2000292, DFNES2000426, DFNES2000432, DFNES2000457, DFNES2000471, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004354, DFNES2004383, DFNES2004684, DFNES2005266, DFNES2005527, DFNES2005540, DFNES2005779, DFNES2005875, DFNES2006944, DFNES2007113, DFNES2007299, DFNES2007332, DFNES2007641, DFNES2007757, DFNES2008280, DFNES2008881, DFNES2010502, DFNES2011192,
DFNES2011221, DFNES2011239, DFNES2011245, DFNES2011380, ERLTF1000021, ERLTF2000324, ERLTF2000465, FCBBF1000023, FCBBF1000025, FCBBF1000069, FCBBF1000117, FCBBF1000121, FCBBF1000125, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000297, FCBBF1000314, FCBBF1000322, FCBBF1000349, FCBBF1000367, FCBBF1000374, FCBBF1000376, FCBBF1000395, FCBBF1000397, FCBBF1000425, FCBBF1000437, FCBBF1000506, FCBBF1000546, FCBBF1000550, FCBBF1000581, FCBBF1000582, FCBBF1000598, FCBBF1000604, FCBBF1000618, FCBBF1000627, FCBBF1000684, FCBBF1000686, FCBBF1000691,
FCBBF1000748, FCBBF2000087, FCBBF2000094, FCBBF2000105, FCBBF2000123, FCBBF2000195, FCBBF2000214, FCBBF2000232, FCBBF2000276, FCBBF2000291, FCBBF2000379, FCBBF2000473, FCBBF2000502, FCBBF2000576, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001088, FCBBF2001116, FCBBF2001188, FCBBF2001238, FCBBF2001243, FCBBF2001299, FCBBF2001400, FCBBF2001427, FCBBF2001480, FCBBF2001529, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370, FCBBF2002541, FCBBF2002626, FCBBF2002897, FCBBF2002898, FCBBF2002904, FCBBF2002928,
FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004090, FCBBF2004138, FCBBF2004217, FCBBF2004256, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2006131, FCBBF2006380, FCBBF2006418, FCBBF2006452, FCBBF2006634, FCBBF2007012, FCBBF2007186, FCBBF2007265, FCBBF2007610, FCBBF2007633, FCBBF3000120, FCBBF3000175, FCBBF3000227, FCBBF3000228, FCBBF3000229, FCBBF3000233, FCBBF3000269, FCBBF3000361, FCBBF3000367, FCBBF3000462,
FCBBF3000473, FCBBF3000501, FCBBF3000518, FCBBF3000536, FCBBF3000550, FCBBF3000847, FCBBF3001018, FCBBF3001020, FCBBF3001235, FCBBF3001302, FCBBF3001470, FCBBF3001594, FCBBF3001912, FCBBF3001914, FCBBF3001918, FCBBF3001924, FCBBF3002163, FCBBF3002188, FCBBF3002190, FCBBF3002268, FCBBF3002475, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3004021, FCBBF3004041, FCBBF3004390, FCBBF3004502, FCBBF3004842, FCBBF3004847, FCBBF3005160, FCBBF3005218, FCBBF3005444, FCBBF3005698, FCBBF3005729, FCBBF3006171, FCBBF3006249, FCBBF3006288, FCBBF3006399, FCBBF3006628, FCBBF3007077,
FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007860, FCBBF3008073, FCBBF3008153, FCBBF3008159, FCBBF3008251, FCBBF3008311, FCBBF3008362, FCBBF3008475, FCBBF3008556, FCBBF3008689, FCBBF3008748, FCBBF3008870, FCBBF3008938, FCBBF3008944, FCBBF3009101, FCBBF3009256, FCBBF3009317, FCBBF3009428, FCBBF3009464, FCBBF3009526, FCBBF3009717, FCBBF3009888, FCBBF3009949, FCBBF3009978, FCBBF3010008, FCBBF3010124, FCBBF3010130, FCBBF3010149, FCBBF3010211, FCBBF3010361, FCBBF3010508, FCBBF3010544, FCBBF3010586, FCBBF3010601, FCBBF3010665, FCBBF3010729, FCBBF3010733,
FCBBF3011003, FCBBF3011418, FCBBF3011523, FCBBF3011570, FCBBF3011592, FCBBF3011867, FCBBF3011889, FCBBF3012170, FCBBF3012182, FCBBF3012288, FCBBF3012332, FCBBF3012588, FCBBF3012842, FCBBF3012901, FCBBF3013041, FCBBF3013058, FCBBF3013205, FCBBF3013266, FCBBF3013307, FCBBF3013341, FCBBF3013506, FCBBF3013623, FCBBF3013800, FCBBF3013846, FCBBF3014229, FCBBF3014355, FCBBF3014567, FCBBF3015119, FCBBF3015131, FCBBF3015317, FCBBF3015727, FCBBF3015758, FCBBF3015809, FCBBF3015870, FCBBF3016018, FCBBF3016178, FCBBF3016618, FCBBF3016622, FCBBF3016644, FCBBF3016987, FCBBF3017059, FCBBF3017071, FCBBF3017123, FCBBF3017233, FCBBF3017255, FCBBF3017531, FCBBF3017535, FCBBF3017681, FCBBF3017729, FCBBF3017873,
FCBBF3017974, FCBBF3018067, FCBBF3018173, FCBBF3018591, FCBBF3018753, FCBBF3018796, FCBBF3019085, FCBBF3019320, FCBBF3019564, FCBBF3019569, FCBBF3019570, FCBBF3019663, FCBBF3019716, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022566, FCBBF3022765, FCBBF3022767, FCBBF3022894, FCBBF3023061, FCBBF3023078, FCBBF3023372, FCBBF3023443, FCBBF3023599, FCBBF3023704, FCBBF3023895, FCBBF3024096, FCBBF3024225, FCBBF3024266, FCBBF3024364, FCBBF3024793,
FCBBF3024911, FCBBF3025098, FCBBF3025252, FCBBF3025280, FCBBF3025285, FCBBF3025417, FCBBF3025568, FCBBF3025650, FCBBF3025674, FCBBF3025905, FCBBF3026021, FCBBF3026251, FCBBF3026308, FCBBF3026678, FCBBF3026692, FCBBF3027104, FCBBF3027199, FCBBF3027328, FCBBF3027559, FCBBF3027717, FCBBF3027755, FCBBF3027768, FCBBF3027863, FCBBF3027903, FCBBF3028102, FCBBF3028143, FCBBF3028188, FCBBF3028202, FCBBF3028472, FCBBF3028528, FCBBF3028582, FCBBF3028671, FCBBF3028794, FCBBF4000061, FCBBF4000076, FCBBF4000173, FCBBF4000192, FCBBF4000268, FCBBF4000282, FCBBF4000399, FCBBF4000415, FCBBF4000500, FCBBF4000529, FCBBF4000548, FCBBF5000041, FCBBF5000261, FCBBF5000325, FCBBF5000353, FCBBF5000384, FCBBF5000483,
FCBBF5000495, FEBRA1000005, FEBRA1000022, FEBRA1000138, FEBRA1000148, FEBRA1000149, FEBRA1000183, FEBRA1000189, FEBRA1000190, FEBRA2000007, FEBRA2000021, FEBRA2000035, FEBRA2000053, FEBRA2000126, FEBRA2000129, FEBRA2000210, FEBRA2000220, FEBRA2000282, FEBRA2000286, FEBRA2000311, FEBRA2000321, FEBRA2000333, FEBRA2000378, FEBRA2000391, FEBRA2000394, FEBRA2000399, FEBRA2000402, FEBRA2000404, FEBRA2000409, FEBRA2000415, FEBRA2000436, FEBRA2000438, FEBRA2000454, FEBRA2000468, FEBRA2000491, FEBRA2000504, FEBRA2000510, FEBRA2000536, FEBRA2000538, FEBRA2000575, FEBRA2000656, FEBRA2000659, FEBRA2000680, FEBRA2000690, FEBRA2000727, FEBRA2000734, FEBRA2000762, FEBRA2000771, FEBRA2000772, FEBRA2000782,
FEBRA2000790, FEBRA2000803, FEBRA2000805, FEBRA2000809, FEBRA2000856, FEBRA2000862, FEBRA2000877, FEBRA2000880, FEBRA2000901, FEBRA2000909, FEBRA2000917, FEBRA2000928, FEBRA2000936, FEBRA2000937, FEBRA2000959, FEBRA2001004, FEBRA2001012, FEBRA2001020, FEBRA2001093, FEBRA2001124, FEBRA2001133, FEBRA2001161, FEBRA2001175, FEBRA2001217, FEBRA2001267, FEBRA2001294, FEBRA2001334, FEBRA2001351, FEBRA2001383, FEBRA2001438, FEBRA2001440, FEBRA2001487, FEBRA2001523, FEBRA2001561, FEBRA2001584, FEBRA2001590, FEBRA2001591, FEBRA2001610, FEBRA2001620, FEBRA2001659, FEBRA2001698, FEBRA2001705, FEBRA2001706, FEBRA2001752, FEBRA2001772, FEBRA2001790, FEBRA2001914, FEBRA2001974, FEBRA2001989, FEBRA2002009,
FEBRA2002086, FEBRA2002109, FEBRA2002191, FEBRA2002260, FEBRA2002399, FEBRA2002429, FEBRA2002442, FEBRA2002508, FEBRA2002527, FEBRA2002552, FEBRA2002574, FEBRA2002601, FEBRA2002611, FEBRA2002628, FEBRA2002662, FEBRA2002727, FEBRA2002783, FEBRA2002818, FEBRA2002858, FEBRA2002882, FEBRA2002897, FEBRA2002908, FEBRA2002962, FEBRA2002986, FEBRA2003054, FEBRA2003115, FEBRA2003155, FEBRA2003163, FEBRA2003181, FEBRA2003253, FEBRA2003258, FEBRA2003275, FEBRA2003308, FEBRA2003327, FEBRA2003429, FEBRA2003436, FEBRA2003454, FEBRA2003468, FEBRA2003520, FEBRA2003524, FEBRA2003675, FEBRA2003707, FEBRA2003711, FEBRA2003726, FEBRA2003822, FEBRA2003897, FEBRA2003907, FEBRA2004023, FEBRA2004026, FEBRA2004029,
FEBRA2004053, FEBRA2004056, FEBRA2004091, FEBRA2004202, FEBRA2004219, FEBRA2004237, FEBRA2004268, FEBRA2004329, FEBRA2004331, FEBRA2004443, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005216, FEBRA2005291, FEBRA2005377, FEBRA2005380, FEBRA2005427, FEBRA2005458, FEBRA2005701, FEBRA2005726, FEBRA2005752, FEBRA2005787, FEBRA2005788, FEBRA2005998, FEBRA2006055, FEBRA2006061, FEBRA2006150, FEBRA2006165, FEBRA2006315, FEBRA2006346, FEBRA2006354, FEBRA2006370, FEBRA2006379, FEBRA2006409,
FEBRA2006418, FEBRA2006427, FEBRA2006476, FEBRA2006491, FEBRA2006519, FEBRA2006664, FEBRA2006667, FEBRA2006726, FEBRA2006736, FEBRA2006793, FEBRA2006817, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007534, FEBRA2007551, FEBRA2007566, FEBRA2007589, FEBRA2007613, FEBRA2007620, FEBRA2007708, FEBRA2007818, FEBRA2007823, FEBRA2007931, FEBRA2007957, FEBRA2008009, FEBRA2008086, FEBRA2008134, FEBRA2008135, FEBRA2008159, FEBRA2008165, FEBRA2008172, FEBRA2008255, FEBRA2008265, FEBRA2008266, FEBRA2008282, FEBRA2008311, FEBRA2008341, FEBRA2008459, FEBRA2008468, FEBRA2008475,
FEBRA2008481, FEBRA2008542, FEBRA2008660, FEBRA2008681, FEBRA2008692, FEBRA2008741, FEBRA2008755, FEBRA2008836,. FEBRA2008859, FEBRA2008866, FEBRA2008881, FEBRA2008928, FEBRA2008983, FEBRA2009016, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009541, FEBRA2009567, FEBRA2009731, FEBRA2009754, FEBRA2009780, FEBRA2010141, FEBRA2010299, FEBRA2010484, FEBRA2010768, FEBRA2010802, FEBRA2011063, FEBRA2011356, FEBRA2011392, FEBRA2011414, FEBRA2011593, FEBRA2011665, FEBRA2012090,
FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013905, FEBRA2013951, FEBRA2014010, FEBRA2014198, FEBRA2014213, FEBRA2014297, FEBRA2014443, FEBRA2015076, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2018051, FEBRA2018433, FEBRA2018746, FEBRA2019172, FEBRA2019298, FEBRA2019389, FEBRA2019457, FEBRA2019519,
FEBRA2019582, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2022013, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023399, FEBRA2023498, FEBRA2023550, FEBRA2023721, FEBRA2023927, FEBRA2023989, FEBRA2023990, FEBRA2024136, FEBRA2024150, FEBRA2024343, FEBRA2024570,
FEBRA2024693, FEBRA2024744, FEBRA2024797, FEBRA2024987, FEBRA2025249, FEBRA2025463, FEBRA2025477, FEBRA2026474, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEHRT2000325, FEHRT2000364, FEKID1000018, FELIV1000101, FELIV1000137, FELIV1000164, FELNG2000241, HCASM1000021, HCASM1000050, HCASM1000061, HCASM1000130, HCASM2000156, HCASM2000202, HCASM2000266, HCASM2000307, HCASM2000363, HCASM2000534, HCASM2000536, HCASM2000560, HCASM2000871, HCASM2001285, HCASM2001810, HCASM2001866, HCASM2001890, HCASM2002148, HCASM2002502,
HCASM2002754, HCASM2002918, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000142, HCHON1000166, HCHON1000176, HCHON2000038, HCHON2000062, HCHON2000087, HCHON2000100, HCHON2000156, HCHON2000199, HCHON2000271, HCHON2000315, HCHON2000322, HCHON2000344, HCHON2000364, HCHON2000395, HCHON2000417, HCHON2000418, HCHON2000503, HCHON2000626, HCHON2000660, HCHON2000663, HCHON2000673, HCHON2000699, HCHON2000705, HCHON2000817,
HCHON2000818, HCHON2000819, HCHON2000851, HCHON2000898, HCHON2000935, HCHON2000942, HCHON2000956, HCHON2000994, HCHON2001050, HCHON2001099, HCHON2001108, HCHON2001110, HCHON2001116, HCHON2001214, HCHON2001233, HCHON2001269, HCHON2001434, HCHON2001450, HCHON2001453, HCHON2001497, HCHON2001505, HCHON2001519, HCHON2001523, HCHON2001535, HCHON2001546, HCHON2001577, HCHON2001598, HCHON2001604, HCHON2001607, HCHON2001646, HCHON2001712, HCHON2001768, HCHON2001801, HCHON2001853, HCHON2002123, HCHON2002194, HCHON2002241, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659,
HCHON2003676, HCHON2004002, HCHON2004007, HCHON2004279, HCHON2004359, HCHON2004531, HCHON2004776, HCHON2005048, HCHON2005118, HCHON2005166, HCHON2005802, HCHON2005987, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HEART1000010, HEART1000088, HEART1000099, HEART1000102, HEART1000139, HEART1000151, HEART2000024, HEART2000035, HEART2000099, HEART2000298, HEART2000306, HEART2000309, HEART2000418,
HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006557, HEART2006787, HEART2006789, HEART2006909, HEART2007031, HEART2007443, HEART2007767, HEART2008108, HEART2008111, HEART2008257,
HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC2000070, HHDPC2000115, HHDPC2000149, HHDPC2000258, HHDPC2000315, HHDPC2000656, HHDPC2000692, HHDPC2001337, HHDPC2001432, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HLUNG1000017, HLUNG1000024,
HLUNG1000030, HLUNG1000034, HLUNG1000053, HLUNG1000064, HLUNG1000076, HLUNG1000084, HLUNG1000099, HLUNG1000104, HLUNG1000105, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000431, HLUNG2000480, HLUNG2000489, HLUNG2000501, HLUNG2000549, HLUNG2000557, HLUNG2000570, HLUNG2000751, HLUNG2000761, HLUNG2000777, HLUNG2000870, HLUNG2000884, HLUNG2000926, HLUNG2000950, HLUNG2000955, HLUNG2000970, HLUNG2001013, HLUNG2001126,
HLUNG2001144, HLUNG2001214, HLUNG2001439, HLUNG2001459, HLUNG2001507, HLUNG2001814, HLUNG2001996, HLUNG2002006, HLUNG2002050, HLUNG2002059, HLUNG2002085, HLUNG2002145, HLUNG2002295, HLUNG2002303, HLUNG2002405, HLUNG2002429, HLUNG2002465, HLUNG2002562, HLUNG2002648, HLUNG2002659, HLUNG2002707, HLUNG2002811, HLUNG2002916, HLUNG2002942, HLUNG2002949, HLUNG2002958, HLUNG2002982, HLUNG2003003, HLUNG2003049, HLUNG2003061, HLUNG2003162, HLUNG2003246, HLUNG2003331, HLUNG2003335, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004154, HLUNG2004217, HLUNG2004273, HLUNG2004416,
HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006852, HLUNG2006935, HLUNG2007041, HLUNG2007163, HLUNG2007219, HLUNG2007245, HLUNG2007433, HLUNG2007931, HLUNG2008066, HLUNG2008139, HLUNG2008225, HLUNG2008235, HLUNG2008247, HLUNG2008333, HLUNG2008384, HLUNG2008396, HLUNG2008439, HLUNG2008479, HLUNG2008480, HLUNG2008521, HLUNG2008637,
HLUNG2008833, HLUNG2008875, HLUNG2008885, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029,
HLUNG2018282, HLUNG2018626, HLUNG2019058, HSYRA1000131, HSYRA2000044, HSYRA2000237, HSYRA2000253, HSYRA2000371, HSYRA2000424, HSYRA2000507, HSYRA2000510, HSYRA2000629, HSYRA2000640, HSYRA2000706, HSYRA2000760, HSYRA2000787, HSYRA2000792, HSYRA2000832, HSYRA2000927, HSYRA2001003, HSYRA2001103, HSYRA2001142, HSYRA2001153, HSYRA2001232, HSYRA2001255, HSYRA2001332, HSYRA2001353, HSYRA2001420, HSYRA2001484, HSYRA2001552, HSYRA2001580, HSYRA2001631, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007667, HSYRA2008154, HSYRA2008714,
HSYRA2009102, IMR321000155, IMR321000161, IMR321000165, IMR321000193, IMR321000230, IMR321000253, IMR322000010, IMR322000107, IMR322000152, IMR322000164, IMR322000178, IMR322000374, IMR322000379, IMR322000383, IMR322000418, IMR322000518, IMR322000604, IMR322000609, IMR322000656, IMR322000672, IMR322000742, IMR322000811, IMR322000819, IMR322000838, IMR322000859, IMR322000863, IMR322000918, IMR322000919, IMR322000953, IMR322000984, IMR322001018, IMR322001049, IMR322001167, IMR322001185, IMR322001218, IMR322001245, IMR322001318, IMR322001322, IMR322001438, IMR322001452, IMR322001491, IMR322001534, IMR322001584, IMR322001665, IMR322001724, IMR322002035, IMR322002110, IMR322002470, IMR322003675,
IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322013053, IMR322013396, IMR322013731, IMR322013805, IMR322015523, IMR322016146, IMR322017049, IMR322018117, IMR322018192, IMR322019070, JCMLC2000273, KIDNE1000008, KIDNE1000012, KIDNE1000015, KIDNE1000028, KIDNE1000036, KIDNE1000064, KIDNE1000104, KIDNE1000121, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245,
KIDNE2000266, KIDNE2000287, KIDNE2000315, KIDNE2000317, KIDNE2000328, KIDNE2000335, KIDNE2000349, KIDNE2000375, KIDNE2000383, KIDNE2000394, KIDNE2000422, KIDNE2000493, KIDNE2000497, KIDNE2000510, KIDNE2000513, KIDNE2000517, KIDNE2000519, KIDNE2000555, KIDNE2000624, KIDNE2000678, KIDNE2000717, KIDNE2000722, KIDNE2000777, KIDNE2000801, KIDNE2000832, KIDNE2000833, KIDNE2000846, KIDNE2000867, KIDNE2000909, KIDNE2001117, KIDNE2001140, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2002015, KIDNE2002162, KIDNE2002168, KIDNE2002198, KIDNE2002262, KIDNE2002438, KIDNE2002483,
KIDNE2002725, KIDNE2002768, KIDNE2002795, KIDNE2002798, KIDNE2002839, KIDNE2002845, KIDNE2002872, KIDNE2002882, KIDNE2002980, KIDNE2002991, KIDNE2003185, KIDNE2003188, KIDNE2003233, KIDNE2003316, KIDNE2003335, KIDNE2003357, KIDNE2003373, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004519, KIDNE2004520, KIDNE2004531, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985,
KIDNE2004999, KIDNE2005038, KIDNE2005062, KIDNE2005266, KIDNE2005296, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006580, KIDNE2006652, KIDNE2006687, KIDNE2006733, KIDNE2006760, KIDNE2006820, KIDNE2006880, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007910,
KIDNE2007944, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008975, KIDNE2009109, KIDNE2009180, KIDNE2009191, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010574, KIDNE2010674, KIDNE2010739,
KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012291, KIDNE2012361, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415,
KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018254, KIDNE2018268, KIDNE2018269,
KIDNE2018287, KIDNE2018294, KIDNE2018352, KIDNE2018462, KIDNE2018493, KIDNE2018617, KIDNE2018678, KIDNE2018727, KIDNE2018863, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099, LIVER1000104, LIVER1000111, LIVER1000126, LIVER1000132, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000037, LIVER2000158, LIVER2000216, LIVER2000237, LIVER2000247, LIVER2000360, LIVER2000416, LIVER2000446, LIVER2000515,
LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001835, LIVER2002638, LIVER2002644, LIVER2002660, LIVER2002702, LIVER2002842, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410,
LIVER2006469, LIVER2006644, LIVER2006764, LIVER2007415, LIVER2007548, LIVER2007568, LIVER2007721, LIVER2007783, LIVER2008053, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000178, MAMGL1000182, MAMGL1000184, MESAN1000032, MESAN1000035, MESAN1000079, MESAN1000080, MESAN1000121, MESAN1000180, MESAN2000023, MESAN2000026, MESAN2000067, MESAN2000092, MESAN2000167, MESAN2000264, MESAN2000291, MESAN2000337, MESAN2000434, MESAN2000462, MESAN2000620, MESAN2000711, MESAN2000815, MESAN2000894,
MESAN2000909, MESAN2001154, MESAN2001450, MESAN2001770, MESAN2001979, MESAN2001980, MESAN2002113, MESAN2002147, MESAN2002424, MESAN2002687, MESAN2002709, MESAN2002724, MESAN2002790, MESAN2002978, MESAN2003037, MESAN2003101, MESAN2003190, MESAN2003322, MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005724, MESAN2005808, MESAN2005811, MESAN2005957, MESAN2005958, MESAN2006022, MESAN2006328, MESAN2006580, MESAN2006599, MESAN2006971, MESAN2007032, MESAN2007440, MESAN2007597, MESAN2007755, MESAN2008222, MESAN2008415, MESAN2008460, MESAN2008536, MESAN2008679, MESAN2008729,
MESAN2008821, MESAN2009081, MESAN2009105, MESAN2009311, MESAN2009418, MESAN2009503, MESAN2009522, MESAN2009580, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013936, MESAN2014192, MESAN2014295, MESAN2014298, MESAN2014412, MESAN2014624, MESAN2015277, MESAN2015365, MESAN2015401, MESAN2015501, MESAN2015708, MESAN2016159, MESAN2016304, MESAN2016409, MESAN2016965, MESAN2017152, MESAN2017373, MESAN2017417, MESAN2018209, MESAN2018576,
MESAN2018670, MESAN2018699, MESTC1000042, MESTC1000147, MESTC2000150, MESTC2000153, MESTC2000170, N1ESE2000698, NB9N41000146, NB9N41000340, NB9N42000104, NB9N42000122, NB9N42000196, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000187, NHNPC2000606,
NHNPC2000877, NHNPC2001108, NHNPC2001223, NHNPC2001816, NHNPC2002565, NHNPC2002749, NOVAR1000015, NOVAR1000102, NOVAR2000038, NOVAR2000136, NOVAR2000352, NOVAR2000412, NOVAR2000710, NOVAR2000962, NOVAR2001108, NOVAR2001620, NOVAR2001783, NOVAR2001876, NOVAR2002039, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000068, PEBLM1000071, PEBLM1000083, PEBLM1000147, PEBLM1000174, PEBLM1000180, PEBLM2000030, PEBLM2000112, PEBLM2000126, PEBLM2000170, PEBLM2000180, PEBLM2000213, PEBLM2000248, PEBLM2000267, PEBLM2000270, PEBLM2000321, PEBLM2000333, PEBLM2000338, PEBLM2000479, PEBLM2000502, PEBLM2001312,
PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006912, PEBLM2007112, PEBLM2007140, PEBLM2007296, PEBLM2007437, PEBLM2007447, PEBLM2007598, PEBLM2007774, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386,
PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086,
PERIC2009329, PERIC2009566, PLACE5000001, PLACE5000037, PLACE5000058, PLACE5000066, PLACE5000067, PLACE5000068, PLACE5000070, PLACE5000080, PLACE5000105, PLACE5000115, PLACE5000116, PLACE5000129, PLACE5000139, PLACE5000153, PLACE5000170, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000296, PLACE6000314, PLACE6000325, PLACE6000348, PLACE6000371, PLACE6000414, PLACE6000424, PLACE6000426, PLACE6000429, PLACE6000450, PLACE6000463, PLACE6000523, PLACE6000550,
PLACE6000552, PLACE6000555, PLACE6000619, PLACE6000630, PLACE6000953, PLACE6001118, PLACE6001153, PLACE6001185, PLACE6001262, PLACE6001294, PLACE6001536, PLACE6001557, PLACE6001712, PLACE6001740, PLACE6001823, PLACE6001886, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6003004, PLACE6003038, PLACE6003077, PLACE6003094, PLACE6003109, PLACE6003204, PLACE6003218, PLACE6003372, PLACE6003399, PLACE6003539, PLACE6003621, PLACE6003705, PLACE6003740, PLACE6003771, PLACE6003796, PLACE6003839,
PLACE6003850, PLACE6004101, PLACE6004219, PLACE6004336, PLACE6004368, PLACE6004380, PLACE6004396, PLACE6004454, PLACE6004464, PLACE6004491, PLACE6004578, PLACE6004663, PLACE6004687, PLACE6004738, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005487, PLACE6005535, PLACE6005546, PLACE6005559, PLACE6005579, PLACE6005691, PLACE6005786, PLACE6006077, PLACE6006158, PLACE6006186, PLACE6006217, PLACE6006222, PLACE6006266, PLACE6006287, PLACE6006394, PLACE6006418, PLACE6006474, PLACE6006549, PLACE6006566, PLACE6006697, PLACE6006822, PLACE6006871, PLACE6006905, PLACE6007050,
PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008775, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010077, PLACE6010463, PLACE6010485, PLACE6010568, PLACE6010701, PLACE6010704, PLACE6010732, PLACE6010765, PLACE6010848,
PLACE6010925, PLACE6010936, PLACE6010991, PLACE6011102, PLACE6011156, PLACE6011211, PLACE6011260, PLACE6011774, PLACE6011881, PLACE6011907, PLACE6011975, PLACE6012028, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016254, PLACE6016383,
PLACE6016942, PLACE6016984, PLACE6017002, PLACE6017431, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6019195, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7.001936, PLACE7002303, PLACE7002641, PLACE7003639, PLACE7003657, PLACE7003684, PLACE7003985, PLACE7004961,
PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000120, PROST1000123, PROST1000136, PROST1000145, PROST1000152, PROST1000167, PROST1000184, PROST1000217, PROST1000246, PROST1000262, PROST1000272, PROST1000298, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527,
PROST1000528, PROST1000536, PROST1000559, PROST1000575, PROST2000036, PROST2000053, PROST2000067, PROST2000105, PROST2000143, PROST2000162, PROST2000165, PROST2000176, PROST2000203, PROST2000206, PROST2000212, PROST2000241, PROST2000267, PROST2000273, PROST2000277, PROST2000337, PROST2000432, PROST2000452, PROST2000463, PROST2000470, PROST2000505, PROST2000567, PROST2000572, PROST2000717, PROST2000761, PROST2000835, PROST2000893, PROST2000961, PROST2001002, PROST2001116, PROST2001138, PROST2001283, PROST2001414, PROST2001415, PROST2001465, PROST2001521, PROST2001540, PROST2001599, PROST2001611, PROST2001636, PROST2001739, PROST2001796, PROST2001805, PROST2001823, PROST2001883, PROST2001899,
PROST2001997, PROST2001998, PROST2002078, PROST2002101, PROST2002162, PROST2002212, PROST2002338, PROST2002425, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2003102, PROST2003117, PROST2003210, PROST2003302, PROST2003303, PROST2003324, PROST2003340, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251,
PROST2004332, PROST2004481, PROST2004526, PROST2004727, PROST2004742, PROST2004744, PROST2004817, PROST2005039, PROST2005067, PROST2005076, PROST2005093, PROST2005121, PROST2005131, PROST2005228, PROST2005285, PROST2005426, PROST2005466, PROST2005604, PROST2005630, PROST2005683, PROST2005788, PROST2005880, PROST2005886, PROST2005904, PROST2005919, PROST2005943, PROST2006008, PROST2006020, PROST2006060, PROST2006196, PROST2006201, PROST2006282, PROST2006450, PROST2006491, PROST2006510, PROST2006579, PROST2006688, PROST2006737, PROST2006766, PROST2006782, PROST2006933, PROST2006988, PROST2007122, PROST2007237, PROST2007248, PROST2007289, PROST2007317, PROST2007328, PROST2007382, PROST2007389,
PROST2007416, PROST2007528, PROST2007612, PROST2007818, PROST2007950, PROST2007956, PROST2007974, PROST2007988, PROST2008079, PROST2008088, PROST2008243, PROST2008245, PROST2008268, PROST2008360, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010885, PROST2011012, PROST2011038, PROST2011105, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012353, PROST2012400, PROST2012504, PROST2012542, PROST2012550, PROST2012740, PROST2012745, PROST2012780, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015198, PROST2015243, PROST2015246, PROST2015251, PROST2015287, PROST2015457, PROST2015464, PROST2015537, PROST2015545,
PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016980, PROST2017098, PROST2017128, PROST2017367, PROST2017413, PROST2017441, PROST2017529, PROST2017578, PROST2017612, PROST2017617, PROST2017700, PROST2017701, PROST2017729, PROST2017836, PROST2017972, PROST2018030, PROST2018090, PROST2018235, PROST2018437, PROST2018487, PROST2018511, PROST2018583, PROST2018588, PROST2018788, PROST2018902, PROST2018922, PROST2018977,
PROST2019164, PROST2019296, PROST2019398, PROST2019487, PROST2019781, PUAEN1000039, PUAEN1000081, PUAEN1000085, PUAEN1000087, PUAEN1000121, PUAEN1000147, PUAEN1000175, PUAEN1000275, PUAEN1000276, PUAEN1000288, PUAEN2000080, PUAEN2000152, PUAEN2000175, PUAEN2000247, PUAEN2000312, PUAEN2000394, PUAEN2000535, PUAEN2000594, PUAEN2000684, PUAEN2000942, PUAEN2001260, PUAEN2001345, PUAEN2001526, PUAEN2001586, PUAEN2001882, PUAEN2002473, PUAEN2002552, PUAEN2002568, PUAEN2002616, PUAEN2002758, PUAEN2003018, PUAEN2003079, PUAEN2003116, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588,
PUAEN2005930, PUAEN2006029, PUAEN2006328, PUAEN2006578, PUAEN2006639, PUAEN2006761, PUAEN2007785, PUAEN2007898, PUAEN2008123, PUAEN2008228, PUAEN2008314, PUAEN2008515, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000141, RECTM2000220, RECTM2000349, RECTM2000433, RECTM2001307, RECTM2001519, RECTM2001666, RECTM2001691, RECTM2001749, RECTM2002172, RECTM2002602, RECTM2002632, SALGL1000005, SALGL1000024, SALGL1000028, SALGL1000065, SALGL1000107, SALGL1000171, SKMUS1000014, SKMUS1000030, SKMUS1000067, SKMUS1000102, SKMUS1000104, SKMUS1000110, SKMUS1000118, SKMUS1000124, SKMUS1000129,
SKMUS1000176, SKMUS1000177, SKMUS2000020, SKMUS2000061, SKMUS2000074, SKMUS2000078, SKMUS2000117, SKMUS2000137, SKMUS2000243, SKMUS2000271, SKMUS2000287, SKMUS2000317, SKMUS2000330, SKMUS2000339, SKMUS2000343, SKMUS2000365, SKMUS2000380, SKMUS2000390, SKMUS2000416, SKMUS2000458, SKMUS2000468, SKMUS2000484, SKMUS2000515, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000902, SKMUS2000933, SKMUS2000945, SKMUS2000954, SKMUS2000961, SKMUS2000973, SKMUS2000980, SKMUS2001008, SKMUS2001014, SKMUS2001129, SKMUS2001157, SKMUS2001164,
SKMUS2001199, SKMUS2001201, SKMUS2001215, SKMUS2001247, SKMUS2001254, SKMUS2001271, SKMUS2001287, SKMUS2001364, SKMUS2001398, SKMUS2001402, SKMUS2001412, SKMUS2001492, SKMUS2001501, SKMUS2001528, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897,
SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000137, SKNMC1000159, SKNMC1000251, SKNMC2000224, SKNMC2000256, SKNMC2000305, SKNMC2000322, SKNMC2000356, SKNMC2000374, SKNMC2000583, SKNMC2000593, SKNMC2000612, SKNMC2000622, SKNMC2000966, SKNMC2001113, SKNMC2001503, SKNMC2001555, SKNMC2001596, SKNMC2001623, SKNMC2003639, SKNMC2003924, SKNMC2003987, SKNMC2004457, SKNMC2004643, SKNMC2004651, SKNMC2005772, SKNMC2006327, SKNMC2006998,
SKNMC2007134, SKNMC2007502, SKNMC2007504, SKNMC2007961, SKNMC2009450, SKNSH1000174, SKNSH1000301, SKNSH1000308, SKNSH1000416, SKNSH2000151, SKNSH2000163, SKNSH2000319, SKNSH2000482, SKNSH2000516, SKNSH2000550, SKNSH2000716, SKNSH2000971, SKNSH2001222, SKNSH2001875, SKNSH2002054, SKNSH2002768, SKNSH2002866, SKNSH2003064, SKNSH2003174, SKNSH2003422, SKNSH2003466, SKNSH2003483, SKNSH2003528, SKNSH2003633, SKNSH2004039, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008940, SKNSH2008969, SKNSH2009197, SKNSH2009357, SKNSH2009435, SKNSH2009991,
SKNSH2010015, SMINT1000012, SMINT1000016, SMINT1000039, SMINT1000048, SMINT1000051, SMINT1000057, SMINT1000071, SMINT1000075, SMINT1000078, SMINT1000091, SMINT1000100, SMINT1000103, SMINT1000118, SMINT1000137, SMINT1000192, SMINT2000007, SMINT2000032, SMINT2000040, SMINT2000060, SMINT2000084, SMINT2000145, SMINT2000159, SMINT2000185, SMINT2000227, SMINT2000229, SMINT2000239, SMINT2000267, SMINT2000454, SMINT2000518, SMINT2000529, SMINT2000530, SMINT2000538, SMINT2000541, SMINT2000545, SMINT2000558, SMINT2000562, SMINT2000568, SMINT2000602, SMINT2000629, SMINT2000747, SMINT2000811, SMINT2000824, SMINT2000933, SMINT2000984, SMINT2001114, SMINT2001158, SMINT2001183, SMINT2001199, SMINT2001222,
SMINT2001348, SMINT2001397, SMINT2001458, SMINT2001461, SMINT2001535, SMINT2001559, SMINT2001609, SMINT2001683, SMINT2001731, SMINT2001812, SMINT2001818, SMINT2001950, SMINT2002126, SMINT2002159, SMINT2002202, SMINT2002210, SMINT2002281, SMINT2002311, SMINT2002314, SMINT2002328, SMINT2002414, SMINT2002457, SMINT2002689, SMINT2002743, SMINT2002778, SMINT2002880, SMINT2002884, SMINT2002899, SMINT2002974, SMINT2003003, SMINT2003128, SMINT2003150, SMINT2003317, SMINT2003340, SMINT2003386, SMINT2003423, SMINT2003505, SMINT2003569, SMINT2003641, SMINT2003644, SMINT2003866, SMINT2004023, SMINT2004037, SMINT2004047, SMINT2004086, SMINT2004144, SMINT2004280, SMINT2004299, SMINT2004339, SMINT2004350,
SMINT2004414, SMINT2004473, SMINT2004547, SMINT2004583, SMINT2004589, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867,
SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008625, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009233, SMINT2009259, SMINT2009272, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010500, SMINT2010533, SMINT2010629, SMINT2010672, SMINT2010753,
SMINT2010853, SMINT2010897, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012122, SMINT2012179, SMINT2012195, SMINT2012285, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2014166, SMINT2014282, SMINT2014443, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146,
SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000178, SPLEN2000020, SPLEN2000047, SPLEN2000064,
SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000255, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000537, SPLEN2000541, SPLEN2000607, SPLEN2000630, SPLEN2000695, SPLEN2000874, SPLEN2001135, SPLEN2001141, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599,
SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002463, SPLEN2002477, SPLEN2002662, SPLEN2002695, SPLEN2002707, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004124, SPLEN2004148, SPLEN2004181, SPLEN2004220, SPLEN2004343, SPLEN2004346, SPLEN2004368, SPLEN2004611,
SPLEN2004662, SPLEN2004844, SPLEN2004880, SPLEN2004984, SPLEN2005227, SPLEN2005272, SPLEN2005342, SPLEN2005416, SPLEN2005429, SPLEN2005450, SPLEN2005474, SPLEN2005560, SPLEN2005727, SPLEN2005790, SPLEN2005806, SPLEN2005818, SPLEN2005838, SPLEN2005869, SPLEN2005927, SPLEN2005939, SPLEN2006232, SPLEN2006268, SPLEN2006283, SPLEN2006389, SPLEN2006425, SPLEN2006671, SPLEN2006701, SPLEN2006785, SPLEN2006875, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007750, SPLEN2007794, SPLEN2007879, SPLEN2007926, SPLEN2007951, SPLEN2008007, SPLEN2008135, SPLEN2008164, SPLEN2008317, SPLEN2008496, SPLEN2008591,
SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009581, SPLEN2009733, SPLEN2009803, SPLEN2009884, SPLEN2009918, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011766,
SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014119, SPLEN2014136, SPLEN2014199, SPLEN2014210, SPLEN2014293, SPLEN2014318,
SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304,
SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683,
SPLEN2018732, SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019405, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021157, SPLEN2021231, SPLEN2021273, SPLEN2021295, SPLEN2021298,
SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025039, SPLEN2025219, SPLEN2025491, SPLEN2025626, SPLEN2025820, SPLEN2026144,SPLEN2026411, SPLEN2026765, SPLEN2027157, SPLEN2027395,
SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333,
SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237,
SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042521, SPLEN2042535, SPLEN2042598, SPLEN2042714, SPLEN2042920, STOMA1000013, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000148, STOMA2000168, STOMA2000183, STOMA2000188, STOMA2000257, STOMA2000279, STOMA2000383, STOMA2000386, STOMA2000395, STOMA2000478, STOMA2000529, STOMA2000539, STOMA2000567, STOMA2000640, STOMA2000678, STOMA2000686, STOMA2000888, STOMA2001025, STOMA2001389, STOMA2002052,
STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA200419.4, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000128, SYNOV1000164,
SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001212, SYNOV2001239, SYNOV2001251, SYNOV2001262, SYNOV2001300, SYNOV2001356, SYNOV2001390, SYNOV2001451, SYNOV2001457, SYNOV2001468, SYNOV2001581, SYNOV2001648, SYNOV2001660, SYNOV2003326, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2009172, SYNOV2011233,
SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012,
SYNOV4007215, SYNOV4007360, SYNOV4007430, SYNOV4007521, SYNOV4007553, SYNOV4007711, SYNOV4008245, SYNOV4008336, SYNOV4009139, SYNOV4009575, T1ESE2000116, T1ESE2000609, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133,
TBAES2009387, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002489, TESOP2002539, TESOP2002545, TESOP2002804, TESOP2002950, TESOP2003273, TESOP2003308, TESOP2003553, TESOP2003753, TESOP2004110,
TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000055, TESTI1000064, TESTI1000070, TESTI1000085, TESTI1000088, TESTI1000093,
TESTI1000094, TESTI1000101, TESTI1000109, TESTI1000125, TESTI1000127, TESTI1000128, TESTI1000131, TESTI1000138, TESTI1000157, TESTI1000163, TESTI1000168, TESTI1000169, TESTI1000179, TESTI1000183, TESTI1000191, TESTI1000257, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000177, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462,
TESTI2000489, TESTI2000520, TESTI2000571, TESTI2000591, TESTI2000600, TESTI2000616, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000849, TESTI2000850, TESTI2000883, TESTI2000909, TESTI2000951, TESTI2000981, TESTI2001008, TESTI2001049, TESTI2001082, TESTI2001120, TESTI2001134, TESTI2001141, TESTI2001153, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518,
TESTI2001556, TESTI2001593, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001862, TESTI2001879, TESTI2001894, TESTI2001898, TESTI2001915, TESTI2001950, TESTI2001959, TESTI2001968, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002406,
TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003130, TESTI2003131, TESTI2003139, TESTI2003141,
TESTI2003152, TESTI2003181, TESTI2003193, TESTI2003196, TESTI2003228, TESTI2003236, TESTI2003255, TESTI2003258, TESTI2003277, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003454, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824,
TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004783, TESTI2004791,
TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720,
TESTI2005739, TESTI2005759, TESTI2005767, TESTI2005775, TESTI2005788, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006097, TESTI2006109, TESTI2006111, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006409, TESTI2006425, TESTI2006437,
TESTI2006453, TESTI2006457, TESTI2006460, TESTI2006465, TESTI2006483, TESTI2006499, TESTI2006523, TESTI2006543, TESTI2006565, TESTI2006572, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007311, TESTI2007320, TESTI2007346, TESTI2007358, TESTI2007402, TESTI2007405,
TESTI2007407, TESTI2007422, TESTI2007452, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405,
TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009390, TESTI2009400, TESTI2009412, TESTI2009423, TESTI2009447, TESTI2009462, TESTI2009477, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835,
TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010682, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011712, TESTI2011750, TESTI2011840, TESTI2011846, TESTI2011915, TESTI2011928, TESTI2011971, TESTI2012019,
TESTI2012050, TESTI2012090, TESTI2012095, TESTI2012104, TESTI2012152, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012778, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013322, TESTI2013381, TESTI2013382, TESTI2013401, TESTI2013427, TESTI2013468, TESTI2013497, TESTI2013545,
TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015249, TESTI2015327, TESTI2015331, TESTI2015335, TESTI2015350, TESTI2015375, TESTI2015437, TESTI2015590,
TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256,
TESTI2017305, TESTI2017311, TESTI2017396, TESTI2017507, TESTI2017537, TESTI2017582, TESTI2017584, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336,
TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020515, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112,
TESTI2021114, TESTI2021116, TESTI2021118, TESTI2021122, TESTI2021138, TESTI2021297, TESTI2021315, TESTI2021358, TESTI2021425, TESTI2021463, TESTI2021531, TESTI2021599, TESTI2021637, TESTI2021654, TESTI2021667, TESTI2021736, TESTI2021785, TESTI2021819, TESTI2021895, TESTI2021911, TESTI2021939, TESTI2021999, TESTI2022010, TESTI2022023, TESTI2022065, TESTI2022086, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214,
TESTI2023254, TESTI2023386, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174,
TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026647, TESTI2026674, TESTI2026794, TESTI2026824, TESTI2026854,
TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776,
TESTI2028811, TESTI2028891, TESTI2028985, TESTI2028997, TESTI2029131, TESTI2029162, TESTI2029196, TESTI2029201, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681,
TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688,
TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275,
TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664,
TESTI2041730, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155,
TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206,
TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI3000002, TESTI3000005, TESTI4000068, TESTI4000079, TESTI4000093, TESTI4000134,
TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002141,
TESTI4002195, TESTI4002202, TESTI4002290, TESTI4002319, TESTI4002491, TESTI4002520, TESTI4002552, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005158, TESTI4005317, TESTI4005322, TESTI4005470, TESTI4005500, TESTI4005534, TESTI4005543, TESTI4005628, TESTI4005801, TESTI4005805, TESTI4005857,
TESTI4005961, TESTI4006053, TESTI4006079, TESTI4006112, TESTI4006137, TESTI4006148, TESTI4006219, TESTI4006234, TESTI4006308, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4008007, TESTI4008018, TESTI4008050, TESTI4008086, TESTI4008097, TESTI4008176, TESTI4008209, TESTI4008219, TESTI4008250, TESTI4008302, TESTI4008305,
TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505,
TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014265, TESTI4014276, TESTI4014306, TESTI4014392, TESTI4014415, TESTI4014445, TESTI4014553, TESTI4014661,
TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017763, TESTI4017848, TESTI4017854, TESTI4017901, TESTI4017961, TESTI4017984, TESTI4018101,
TESTI4018152, TESTI4018208, TESTI4018382, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096,
TESTI4024240, TESTI4024245, TESTI4024294, TESTI4024344, TESTI4024387, TESTI4024420, TESTI4024494, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809,
TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065,
TESTI4035498, TESTI4035508, TESTI4035581, TESTI4035602, TESTI4035637, TESTI4035649, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939,
TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701,
TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000029, THYMU1000032, THYMU1000033, THYMU1000060, THYMU1000074, THYMU1000098, THYMU1000105, THYMU1000109, THYMU1000136, THYMU1000142, THYMU1000158, THYMU1000176, THYMU1000179, THYMU1000191, THYMU1000198, THYMU1000217, THYMU1000240, THYMU1000242,
THYMU1000291, THYMU1000316, THYMU1000318, THYMU1000331, THYMU1000336, THYMU1000359, THYMU1000366, THYMU1000382, THYMU1000393, THYMU1000394, THYMU1000399, THYMU1000426, THYMU1000440, THYMU1000459, THYMU1000473, THYMU1000490, THYMU1000491, THYMU1000496, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000382, THYMU2000418, THYMU2000440, THYMU2000489, THYMU2000492,
THYMU2000519, THYMU2000570, THYMU2000602, THYMU2000660, THYMU2000671, THYMU2000672, THYMU2000684, THYMU2000767, THYMU2000775, THYMU2000800, THYMU2000879, THYMU2000932, THYMU2000950, THYMU2000971, THYMU2000982, THYMU2000990, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001989, THYMU2002023, THYMU2002037, THYMU2002080, THYMU2002109,
THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002852, THYMU2002910, THYMU2002950, THYMU2002983, THYMU2003012, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003542, THYMU2003632, THYMU2003650, THYMU2003700, THYMU2003760, THYMU2003803,
THYMU2003855, THYMU2003891, THYMU2003907, THYMU2003932, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507,
THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007060, THYMU2007095, THYMU2007146, THYMU2007179, THYMU2007258, THYMU2007307, THYMU2007308, THYMU2007350, THYMU2007384, THYMU2007415, THYMU2007467, THYMU2007493, THYMU2007521, THYMU2007528, THYMU2007530, THYMU2007532,
THYMU2007561, THYMU2007571, THYMU2007635, THYMU2007658, THYMU2007667, THYMU2007725, THYMU2007729, THYMU2007802, THYMU2007824, THYMU2007854, THYMU2007952, THYMU2007969, THYMU2008035, THYMU2008036, THYMU2008072, THYMU2008111, THYMU2008145, THYMU2008149, THYMU2008185, THYMU2008191, THYMU2008207, THYMU2008226, THYMU2008282, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186,
THYMU2009338, THYMU2009425, THYMU2009516, THYMU2009526, THYMU2009546, THYMU2009596, THYMU2009658, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666,
THYMU2011736, THYMU2011785, THYMU2011806, THYMU2011852, THYMU2011937, THYMU2011939, THYMU2012073, THYMU2012104, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014183, THYMU2014204, THYMU2014297, THYMU2014323, THYMU2014327, THYMU2014353, THYMU2014492,
THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015918, THYMU2015943, THYMU2015984, THYMU2016113, THYMU2016164, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071,
THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943,
THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2026182, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068,
THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338,
THYMU2034374, THYMU2034647, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634,
THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001991, THYMU3002452, THYMU3002661, THYMU3003007, THYMU3003092, THYMU3003212, THYMU3003350, THYMU3003403,
THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012,
THYMU3011081, THYMU3011244, THYMU3011360, THYMU3011534, THYMU3011543, THYMU3011556, THYMU3011717, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869,
THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752,
THYMU3031146, THYMU3031402, THYMU3031610, THYMU3031612, THYMU3031868, THYMU3031878, THYMU3032032, THYMU3032867, THYMU3033402, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126,
THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047144, THYMU3047156, THYMU3047366, THYMU3047513, THYMU3047542, THYMU3047760, THYMU3047891, TKIDN1000001, TKIDN1000062, TKIDN1000066,
TKIDN1000164, TKIDN1000171, TKIDN2000240, TKIDN2000319, TKIDN2000464, TKIDN2000521, TKIDN2000701, TKIDN2001662, TKIDN2002318, TKIDN2002329, TKIDN2002424, TKIDN2002632, TKIDN2003044, TKIDN2003059, TKIDN2003062, TKIDN2003366, TKIDN2003396, TKIDN2003413, TKIDN2003597, TKIDN2003601, TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009794, TKIDN2009889, TKIDN2010200, TKIDN2010232, TKIDN2010278, TKIDN2010602, TKIDN2011051, TKIDN2011160, TKIDN2011324, TKIDN2012034, TKIDN2012771,
TKIDN2012824, TKIDN2013134, TKIDN2014570, TKIDN2014757, TKIDN2014771, TKIDN2015025, TKIDN2015071, TKIDN2015161, TKIDN2015263, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002336, TLIVE2002338, TLIVE2002562, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733,
TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000038, TRACH1000057, TRACH1000063, TRACH1000074, TRACH1000100, TRACH1000125, TRACH1000140, TRACH1000144, TRACH1000193, TRACH1000212, TRACH1000240, TRACH2000002, TRACH2000015, TRACH2000024, TRACH2000032, TRACH2000045, TRACH2000047,
TRACH2000081, TRACH2000083, TRACH2000148, TRACH2000185, TRACH2000196, TRACH2000235, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000496, TRACH2000497, TRACH2000502, TRACH2000522, TRACH2000540, TRACH2000559, TRACH2000660, TRACH2000665, TRACH2000675, TRACH2000677, TRACH2000703, TRACH2000767, TRACH2000775, TRACH2000807, TRACH2000862, TRACH2000873, TRACH2000894, TRACH2000898, TRACH2000923, TRACH2000944, TRACH2000972, TRACH2000981, TRACH2000986, TRACH2001037, TRACH2001060, TRACH2001085, TRACH2001101, TRACH2001121, TRACH2001147, TRACH2001154, TRACH2001188,
TRACH2001192, TRACH2001249, TRACH2001275, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001474, TRACH2001523, TRACH2001525, TRACH2001532, TRACH2001549, TRACH2001592, TRACH2001596, TRACH2001621, TRACH2001646, TRACH2001651, TRACH2001684, TRACH2001933, TRACH2001968, TRACH2001996, TRACH2002043, TRACH2002054, TRACH2002092, TRACH2002100, TRACH2002138, TRACH2002333, TRACH2002664, TRACH2002784, TRACH2002840, TRACH2002847, TRACH2002853, TRACH2002862, TRACH2002954, TRACH2002960, TRACH2002988, TRACH2003070, TRACH2003484, TRACH2003516, TRACH2003605, TRACH2003736, TRACH2003759, TRACH2003941,
TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007483, TRACH2007542, TRACH2007577,
TRACH2007674, TRACH2007676, TRACH2007688, TRACH2007733, TRACH2007754, TRACH2007816, TRACH2007894, TRACH2007969, TRACH2007988, TRACH2008112, TRACH2008113, TRACH2008127, TRACH2008278, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639,
TRACH2010677, TRACH2010698, TRACH2010824, TRACH2010883, TRACH2010965, TRACH2010974, TRACH2011057, TRACH2011093, TRACH2011113, TRACH2011293, TRACH2011302, TRACH2011500, TRACH2011574, TRACH2011956, TRACH2012000, TRACH2012138, TRACH2012242, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013265, TRACH2013369, TRACH2013450, TRACH2013495, TRACH2013585, TRACH2013671, TRACH2013726, TRACH2013902, TRACH2013928, TRACH2013982, TRACH2013991, TRACH2014018, TRACH2014045, TRACH2014046, TRACH2014077, TRACH2014082, TRACH2014268, TRACH2014284, TRACH2014337, TRACH2014356, TRACH2014371, TRACH2014442,
TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084,
TRACH2018262, TRACH2018273, TRACH2018278, TRACH2018317, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479,
TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000180, TRACH3000342, TRACH3000420, TRACH3000515, TRACH3000530, TRACH3000548, TRACH3000558, TRACH3000586, TRACH3000780, TRACH3000892, TRACH3000926, TRACH3001119, TRACH3001443, TRACH3001550, TRACH3001759, TRACH3002064,
TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003586, TRACH3003683, TRACH3003780, TRACH3003805, TRACH3003832, TRACH3004068, TRACH3004113, TRACH3004288, TRACH3004303, TRACH3004412, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005274, TRACH3005479, TRACH3005549,
TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007627, TRACH3007689, TRACH3007766, TRACH3007866, TRACH3007995, TRACH3008042, TRACH3008061, TRACH3008201, TRACH3008508, TRACH3008629, TRACH3008632, TRACH3008688, TRACH3008697, TRACH3008887, TRACH3008902, TRACH3008990, TRACH3009001, TRACH3009059, TRACH3009061, TRACH3009148,
TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014415, TRACH3014580, TRACH3014641,
TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928,
TRACH3020930, TRACH3021023, TRACH3021066, TRACH3021212, TRACH3021335, TRACH3021373, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026542, TRACH3026650, TRACH3026676, TRACH3026744, TRACH3026949, TRACH3027229, TRACH3027527, TRACH3027681, TRACH3027701,
TRACH3027885, TRACH3028031, TRACH3028164, TRACH3028180, TRACH3028195, TRACH3028441, TRACH3028597, TRACH3028678, TRACH3028837, TRACH3028847, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488,
TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996,
TSTOM2002265, TSTOM2002505, TSTOM2002561, TSTOM2002611, TSTOM2002672, TSTOM2002682, TUTER1000014, TUTER1000137, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000133, UTERU1000008, UTERU1000015, UTERU1000024, UTERU1000031, UTERU1000032, UTERU1000057, UTERU1000065, UTERU1000077, UTERU1000093, UTERU1000096, UTERU1000109, UTERU1000131, UTERU1000138, UTERU1000148, UTERU1000160, UTERU1000183, UTERU1000187, UTERU1000192,
UTERU1000249, UTERU1000337, UTERU1000339, UTERU2000023, UTERU2000047, UTERU2000074, UTERU2000099, UTERU2000154, UTERU2000197, UTERU2000218, UTERU2000238, UTERU2000243, UTERU2000260, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747,
UTERU2001876, UTERU2002001, UTERU2002011, UTERU2002176, UTERU2002198, UTERU2002332, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003456, UTERU2003577, UTERU2003704, UTERU2003926, UTERU2003973, UTERU2004015, UTERU2004037, UTERU2004039, UTERU2004061, UTERU2004073, UTERU2004163, UTERU2004299, UTERU2004461, UTERU2004520, UTERU2004564, UTERU2004664, UTERU2004688, UTERU2004698, UTERU2004861, UTERU2004929, UTERU2005004, UTERU2005050, UTERU2005069, UTERU2005074, UTERU2005179, UTERU2005292, UTERU2005346, UTERU2005446,
UTERU2005449, UTERU2005450, UTERU2005533, UTERU2005593, UTERU2005601, UTERU2005621, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007942, UTERU2008018, UTERU2008019, UTERU2008027, UTERU2008040, UTERU2008077, UTERU2008130, UTERU2008347, UTERU2008426, UTERU2008516, UTERU2008561, UTERU2008705, UTERU2008707, UTERU2008747, UTERU2008785,
UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009414, UTERU2009483, UTERU2009510, UTERU2009538, UTERU2009540, UTERU2009776, UTERU2009904, UTERU2009951, UTERU2010115, UTERU2010124, UTERU2010164, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012252,
UTERU2012333, UTERU2012407, UTERU2012581, UTERU2012610, UTERU2012615, UTERU2012688, UTERU2012703, UTERU2012715, UTERU2012741, UTERU2012786, UTERU2012856, UTERU2012890, UTERU2012976, UTERU2013048, UTERU2013078, UTERU2013231, UTERU2013280, UTERU2013322, UTERU2013483, UTERU2013502, UTERU2013926, UTERU2013976, UTERU2014001, UTERU2014024, UTERU2014223, UTERU2014398, UTERU2014464, UTERU2014548, UTERU2014601, UTERU2014631, UTERU2014668, UTERU2014728, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016464, UTERU2016757, UTERU2016761, UTERU2016822, UTERU2016896,
UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018955, UTERU2019038, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491,
UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2029503, UTERU2029660, UTERU2029742, UTERU2029953, UTERU2030213, UTERU2030270,
UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791,
UTERU2037843, UTERU2038171, UTERU2038251, UTERU3000226, UTERU3000298, UTERU3000402, UTERU3000645, UTERU3000665, UTERU3000670, UTERU3000738, UTERU3000828, UTERU3000844, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003774, UTERU3003776, UTERU3004384, UTERU3004477, UTERU3004523, UTERU3004616,
UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010526, UTERU3010604,
UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078 Of the 17,176 genes that include the cDNAs of the present invention, genes whose expression levels were high enough to detect in each of the PANCR, PEBLM, PERIC, PLACE, PROST, PUAEN, and RECTM libraries were analyzed for expression frequency. The analysis results are described below. Each is a relative value, where the expression level over all tissues is taken as 100. The larger this value, the greater the level of expression.

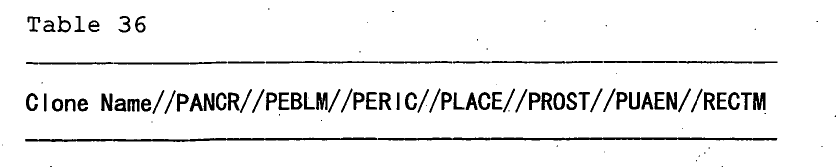

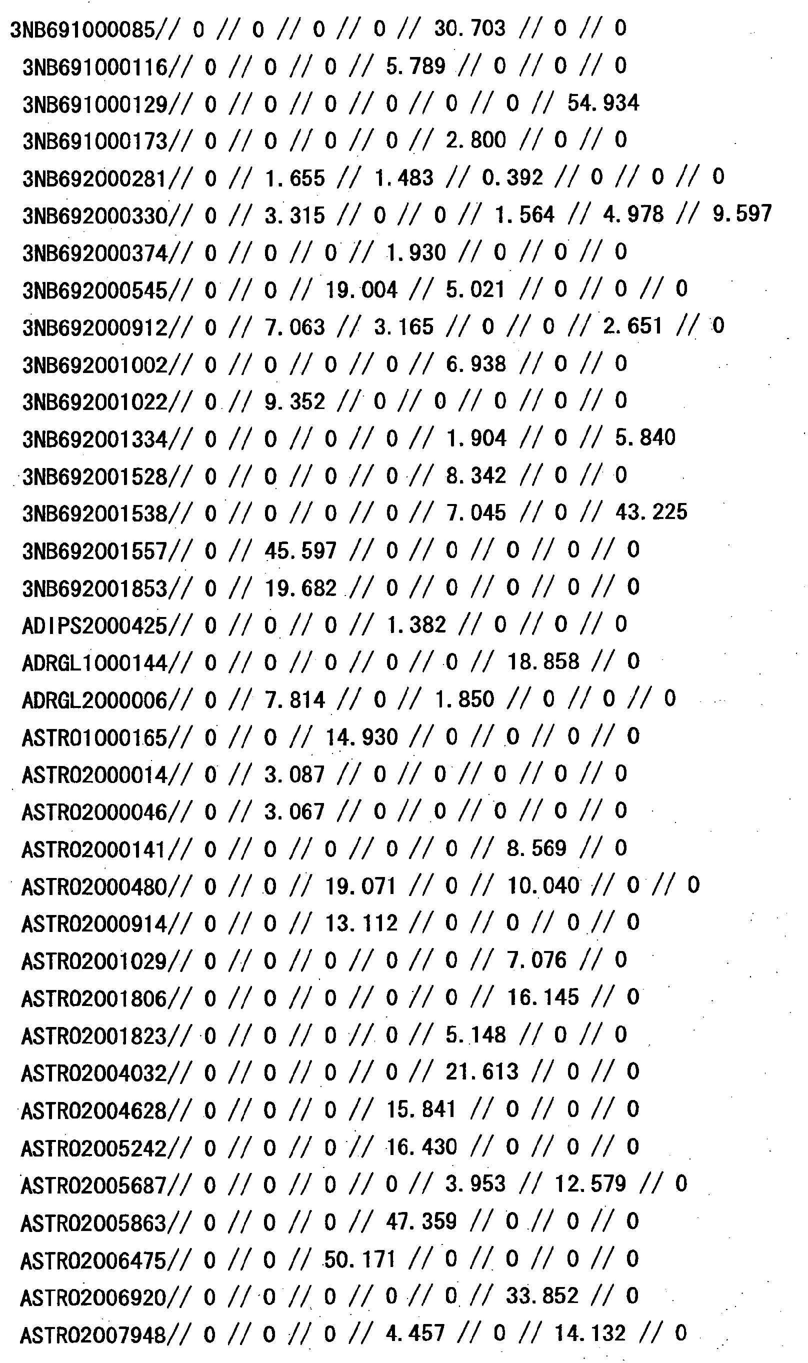

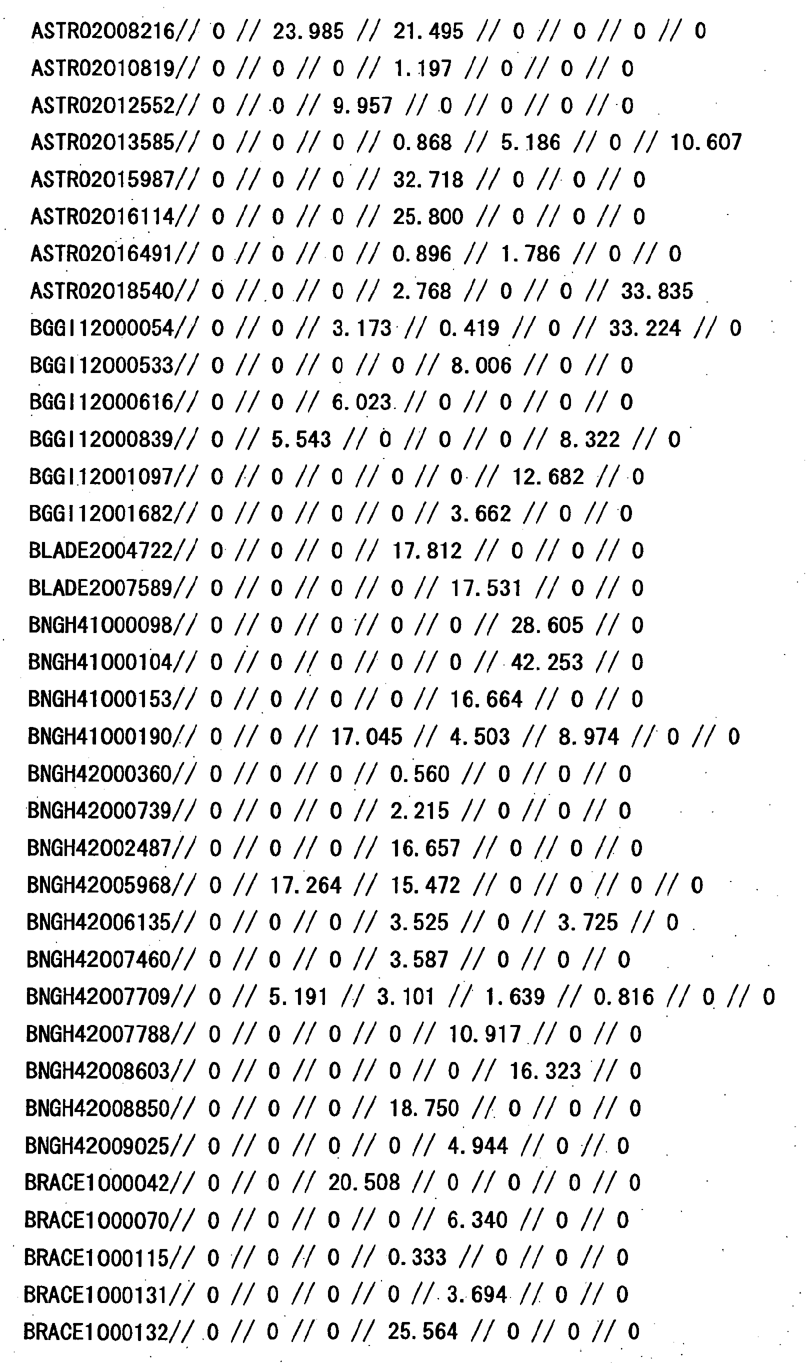

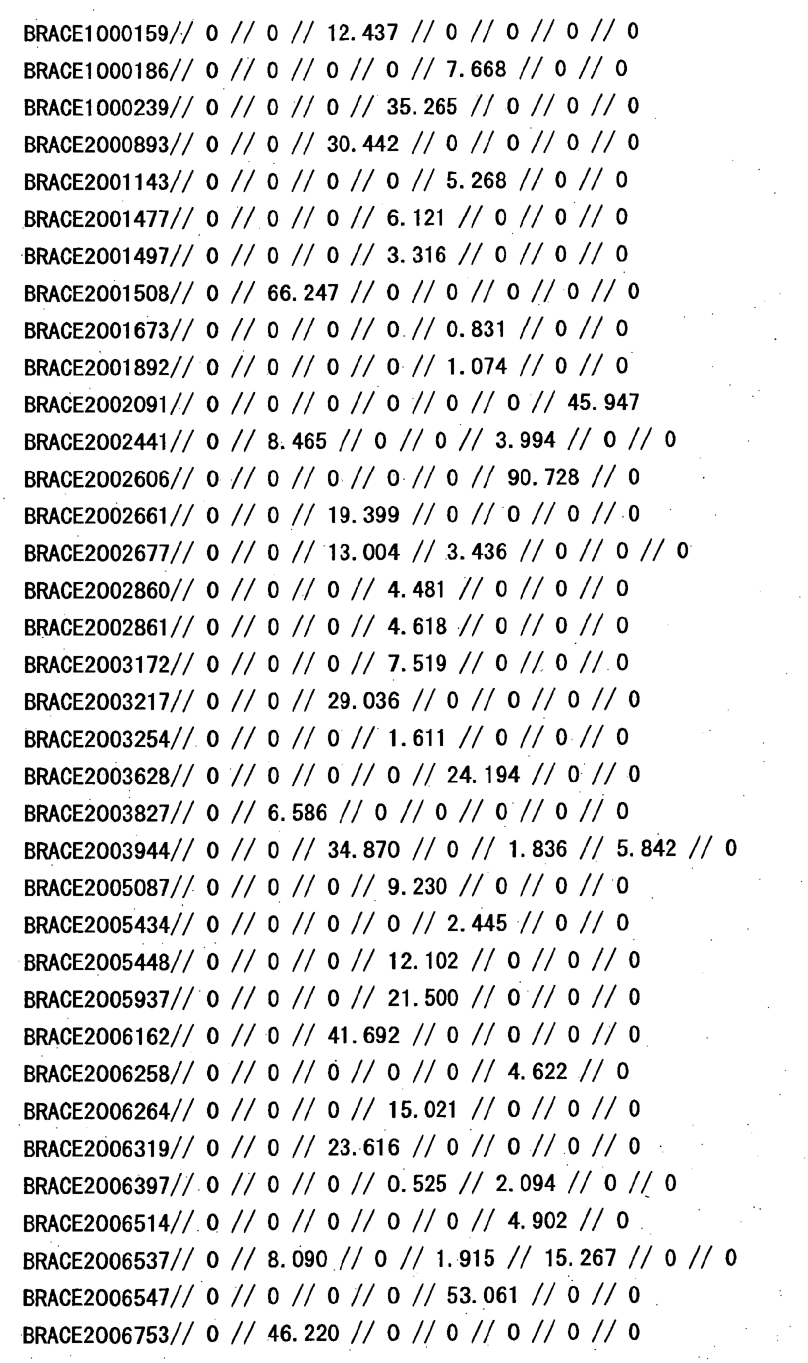

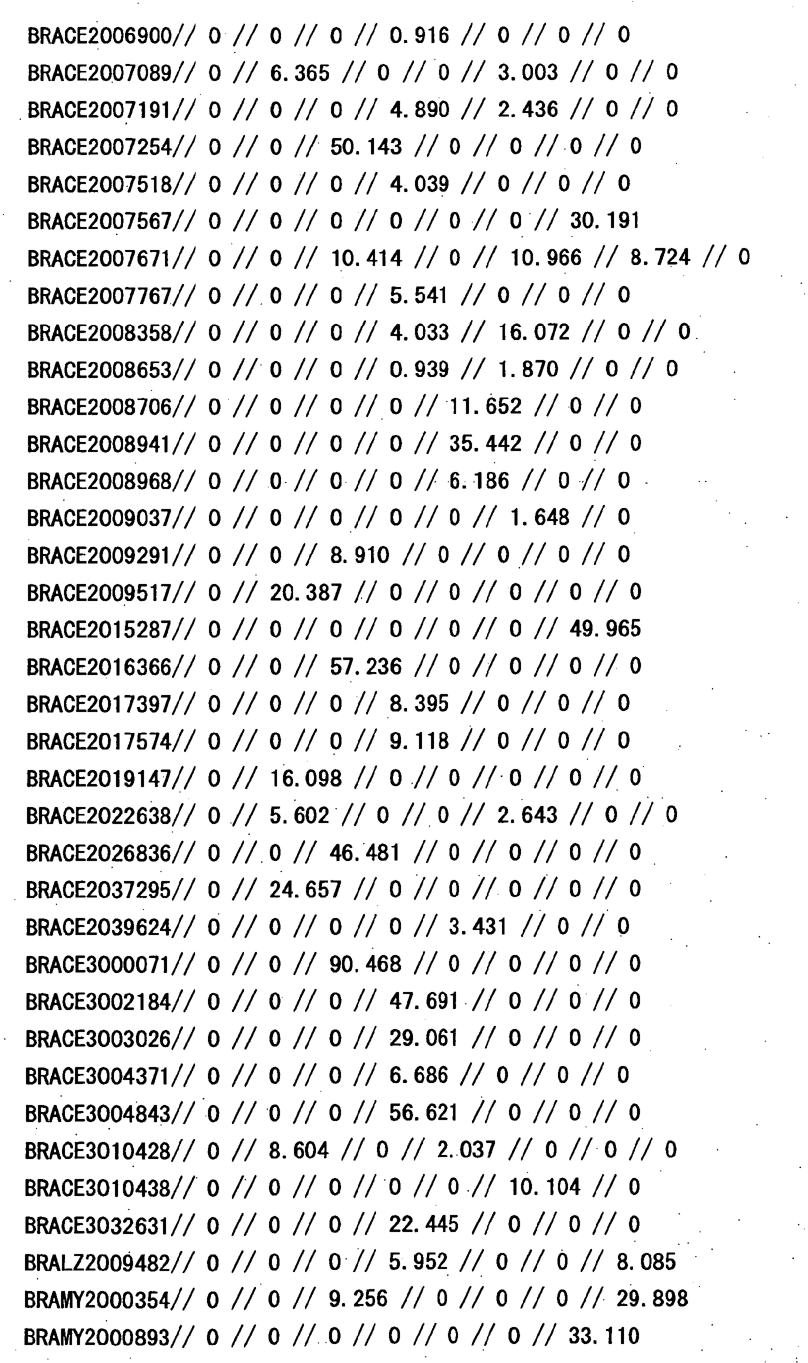

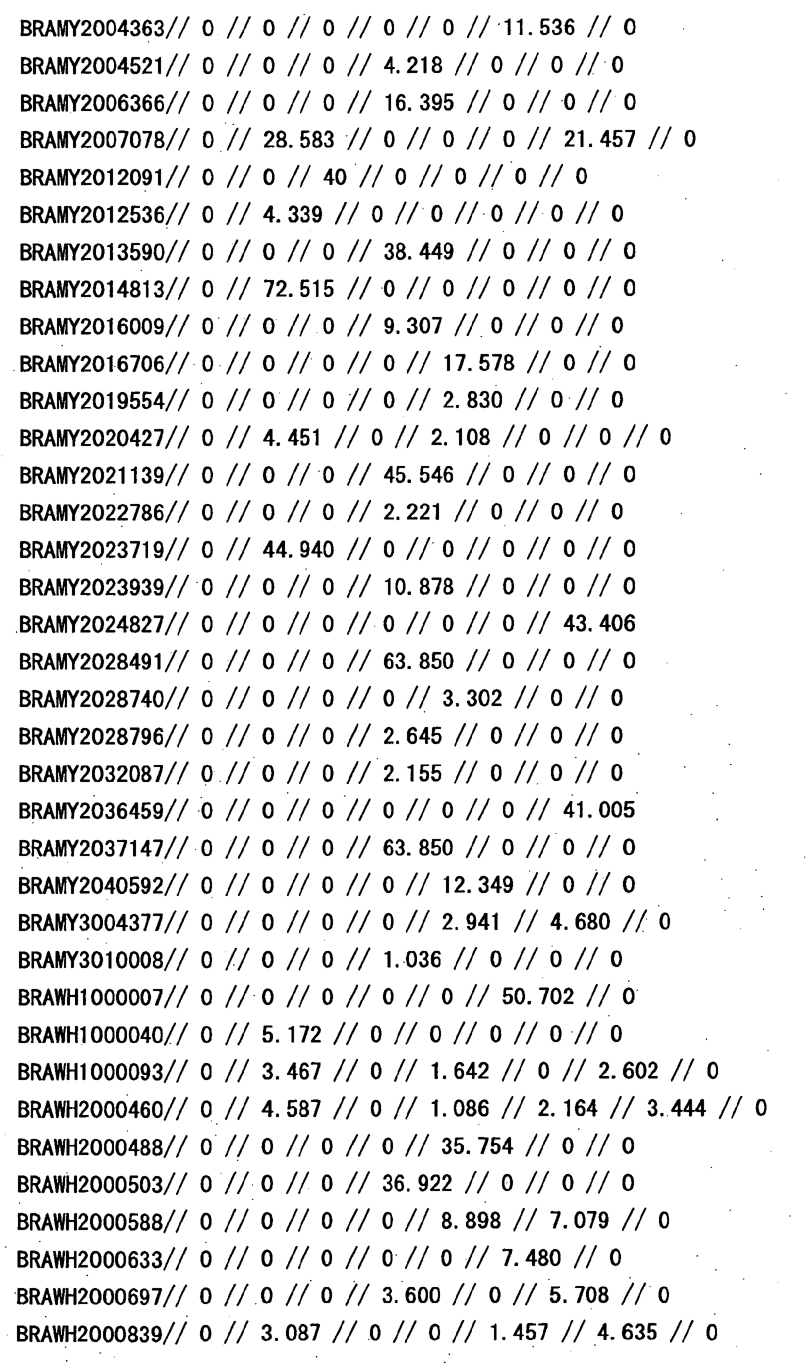

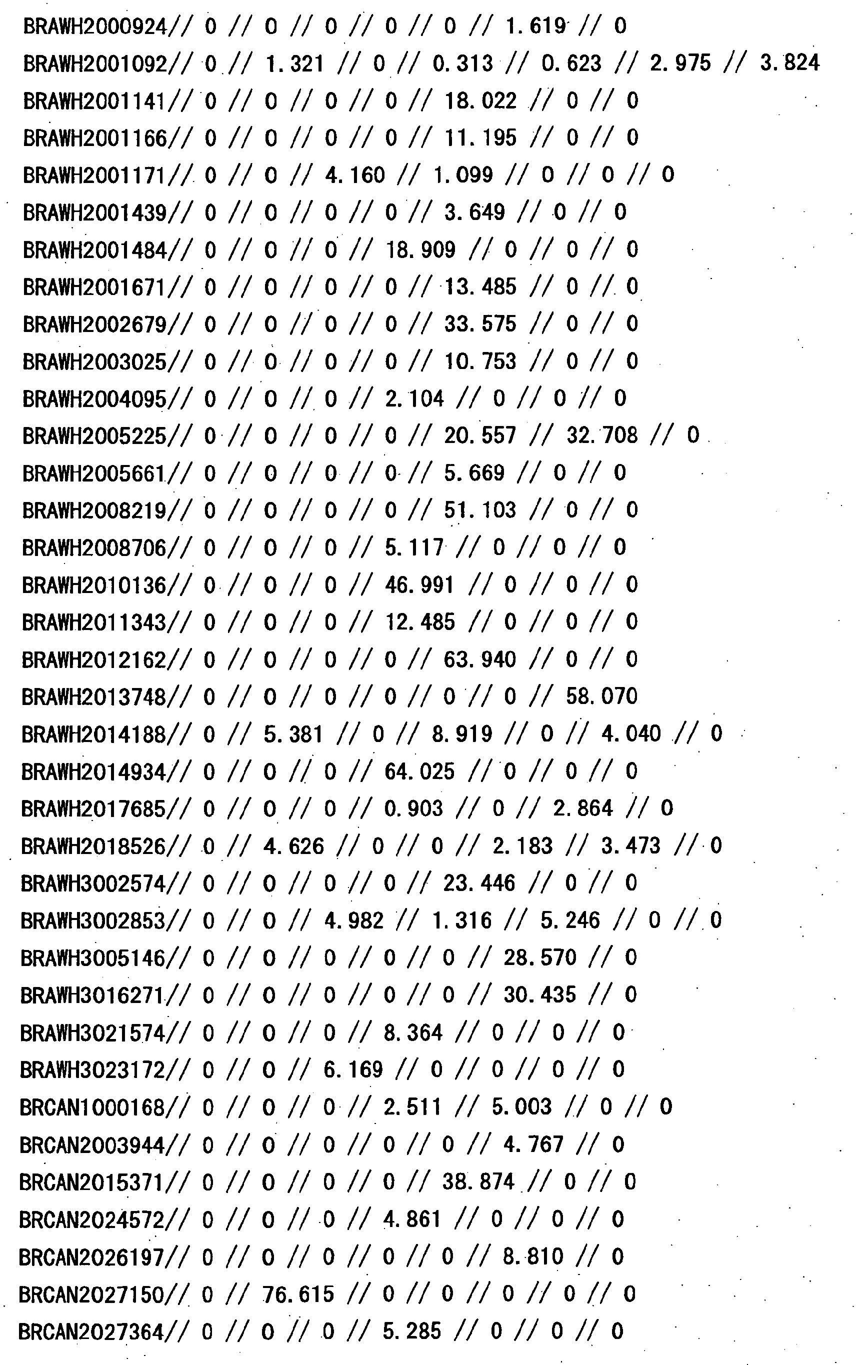

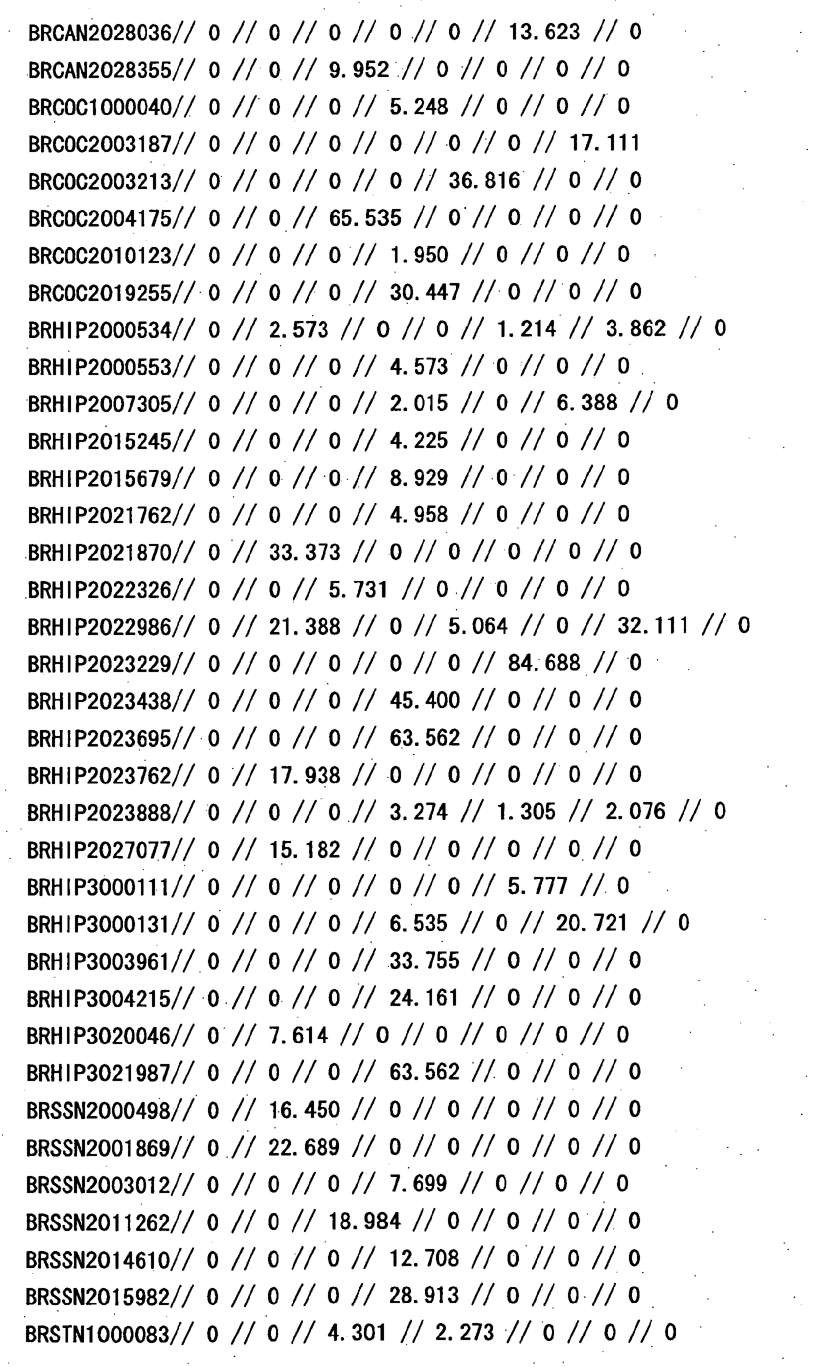

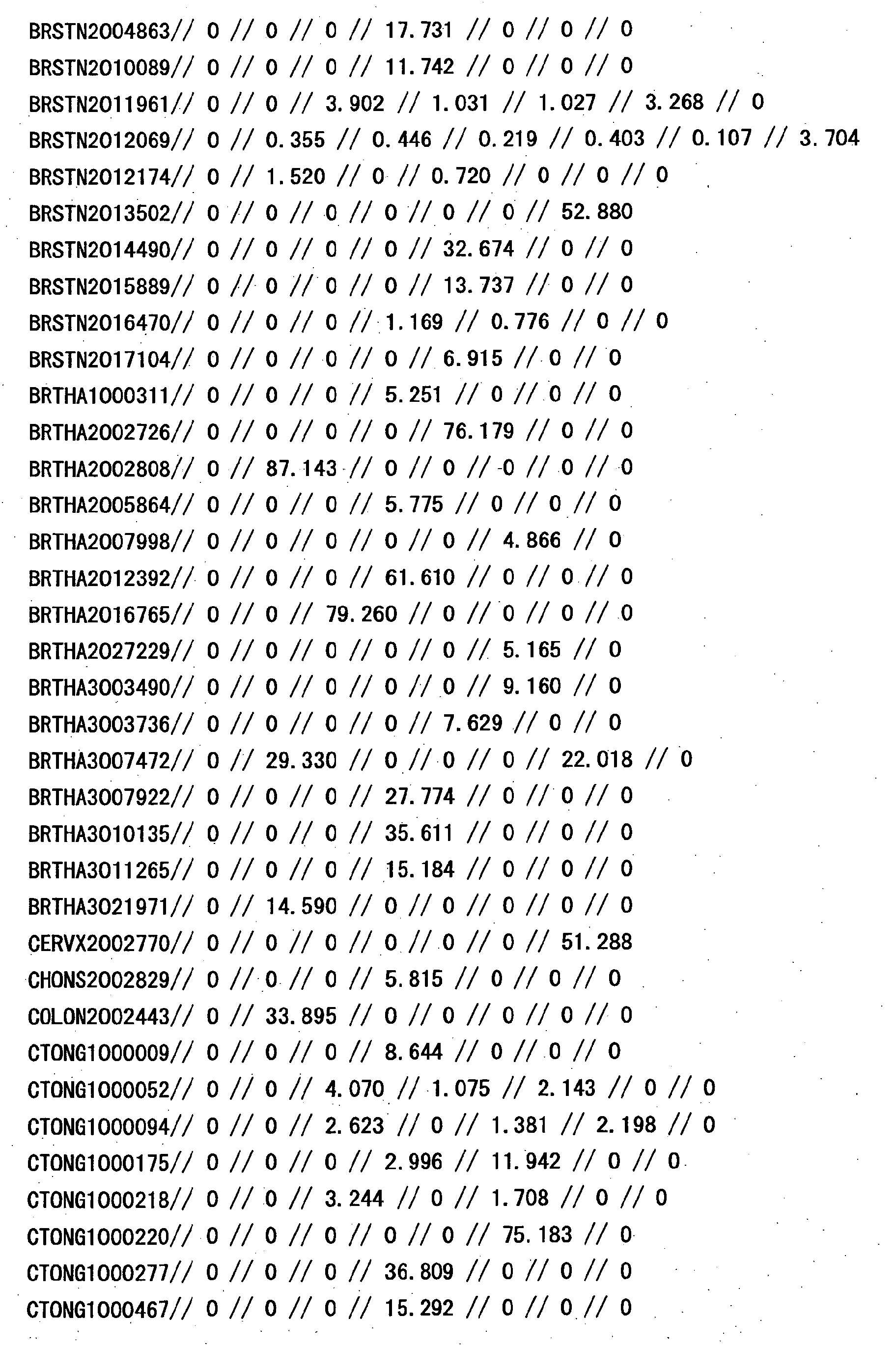

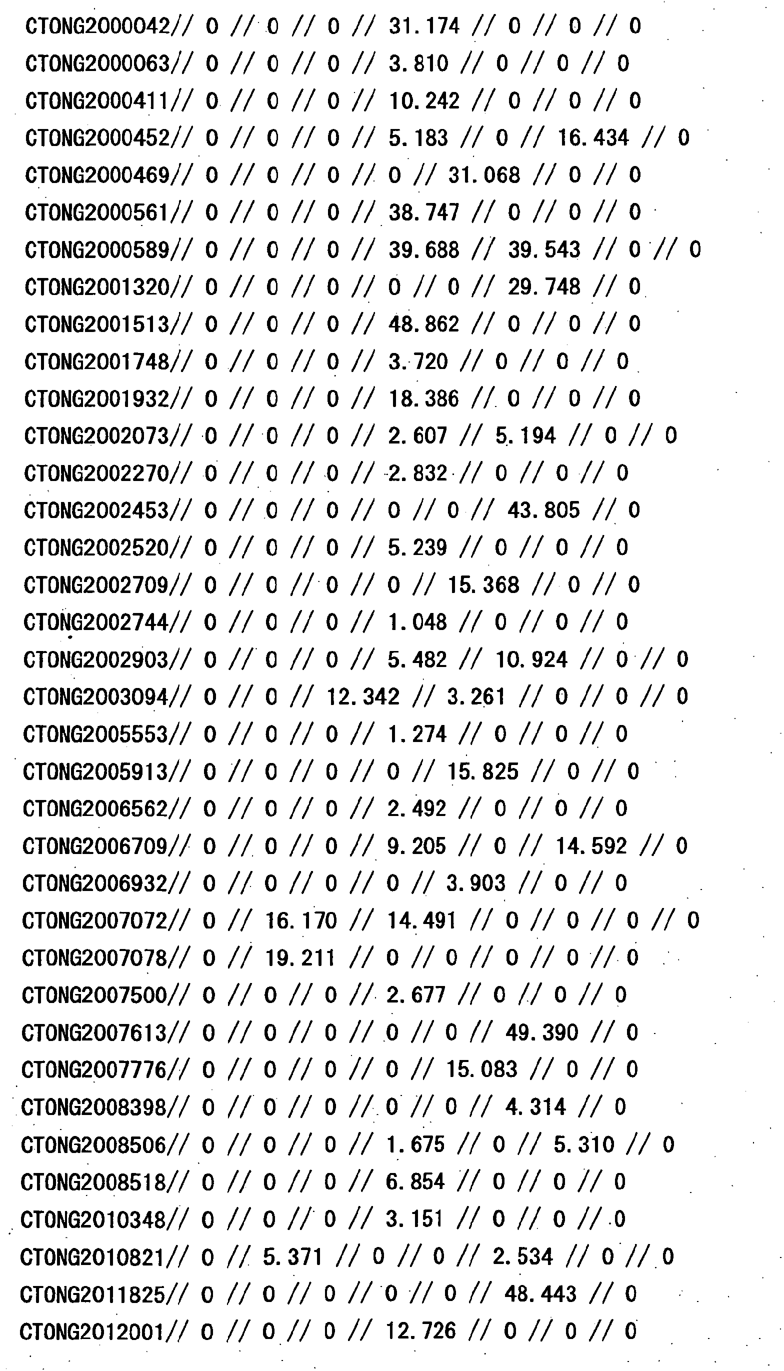

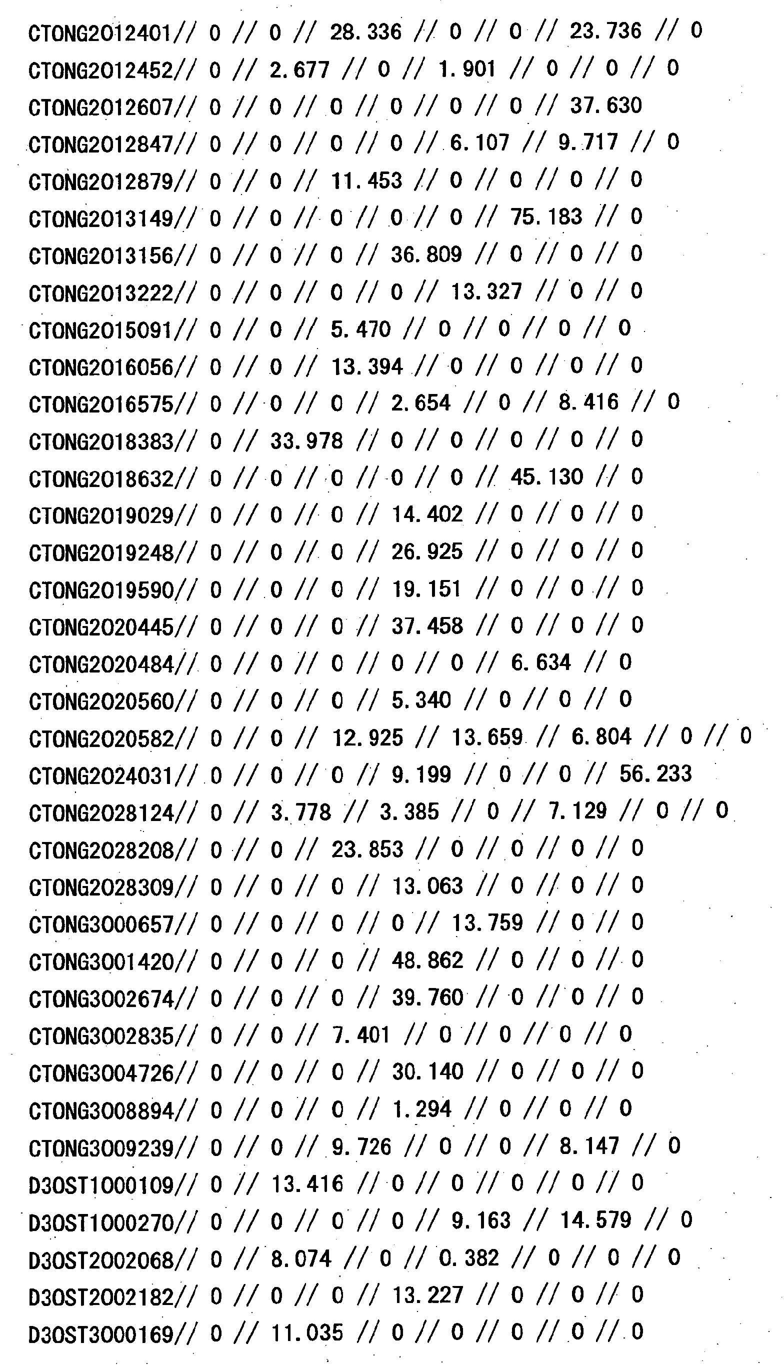

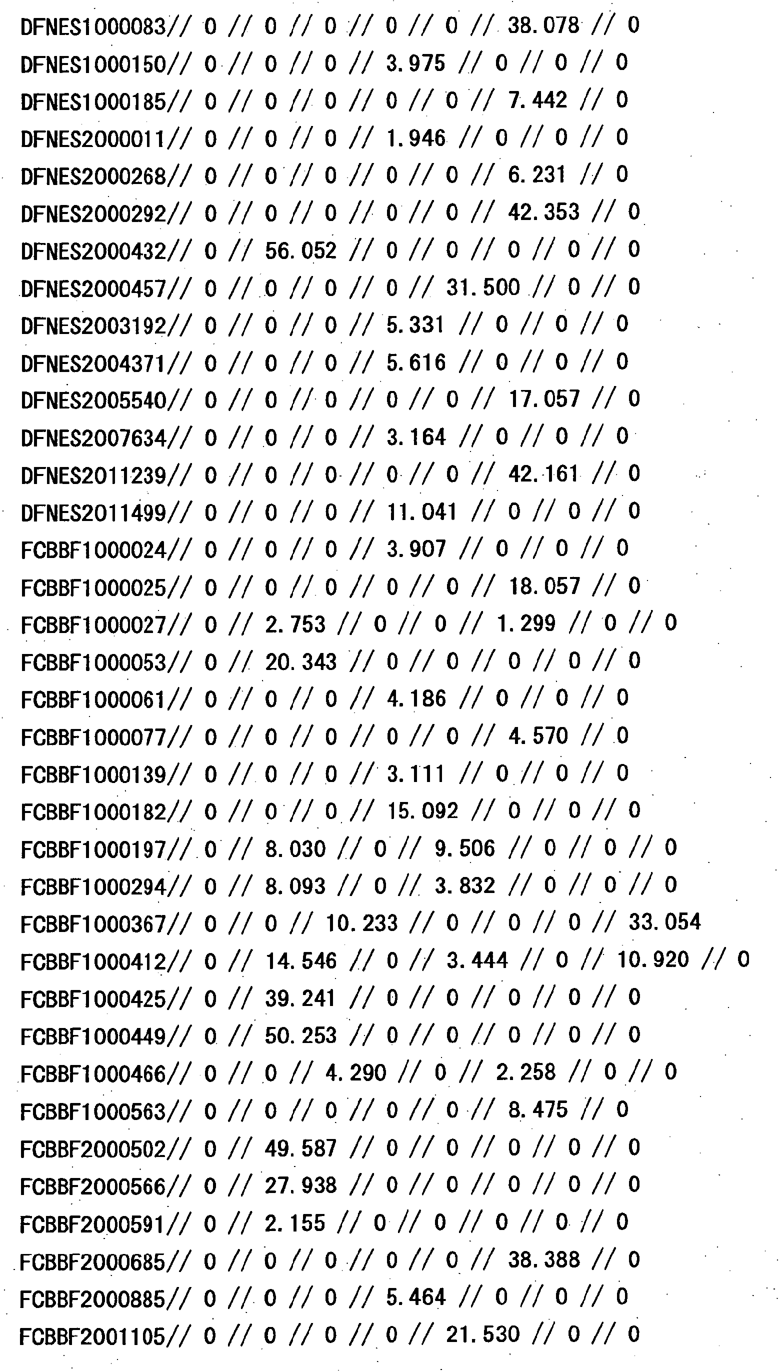

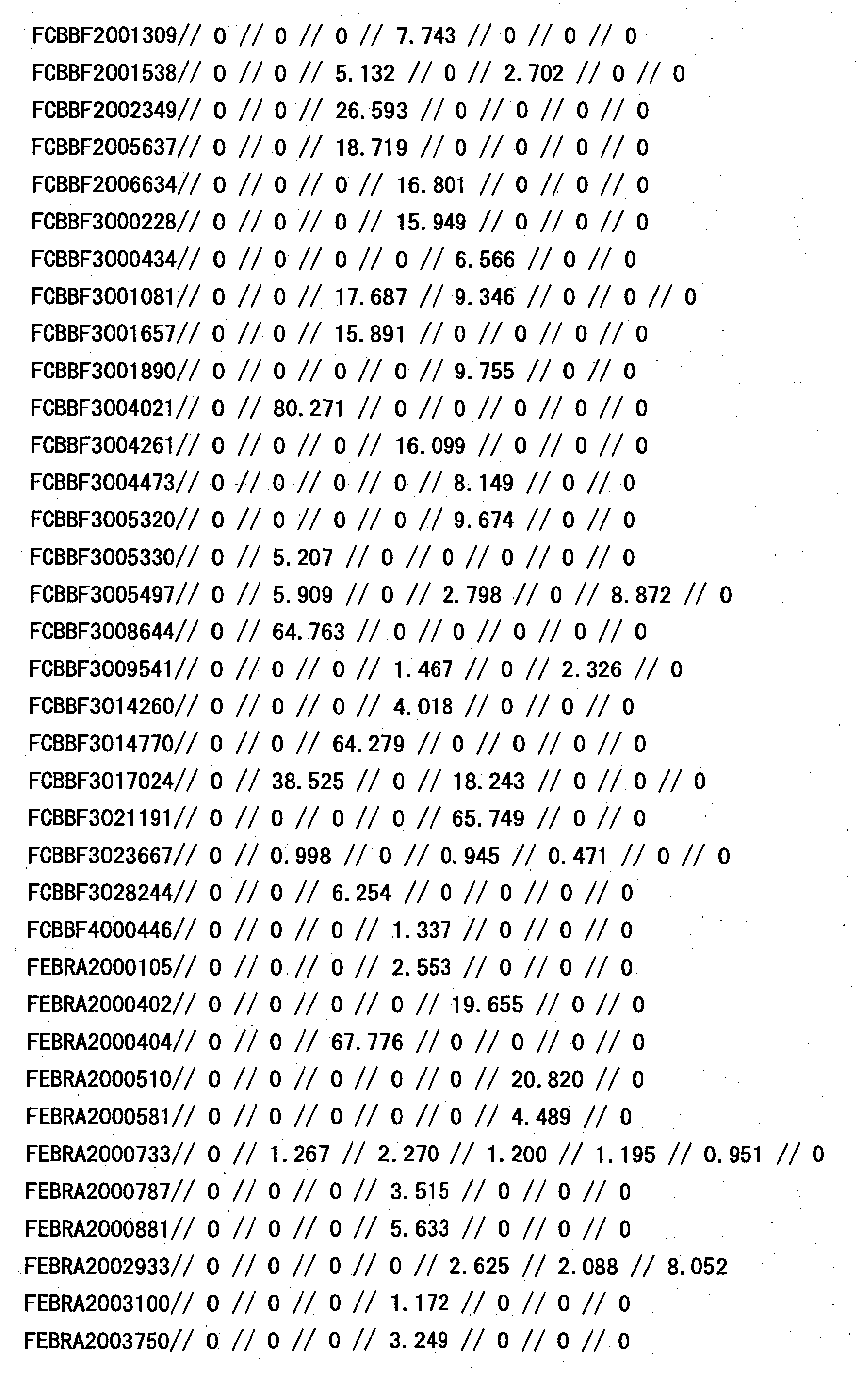

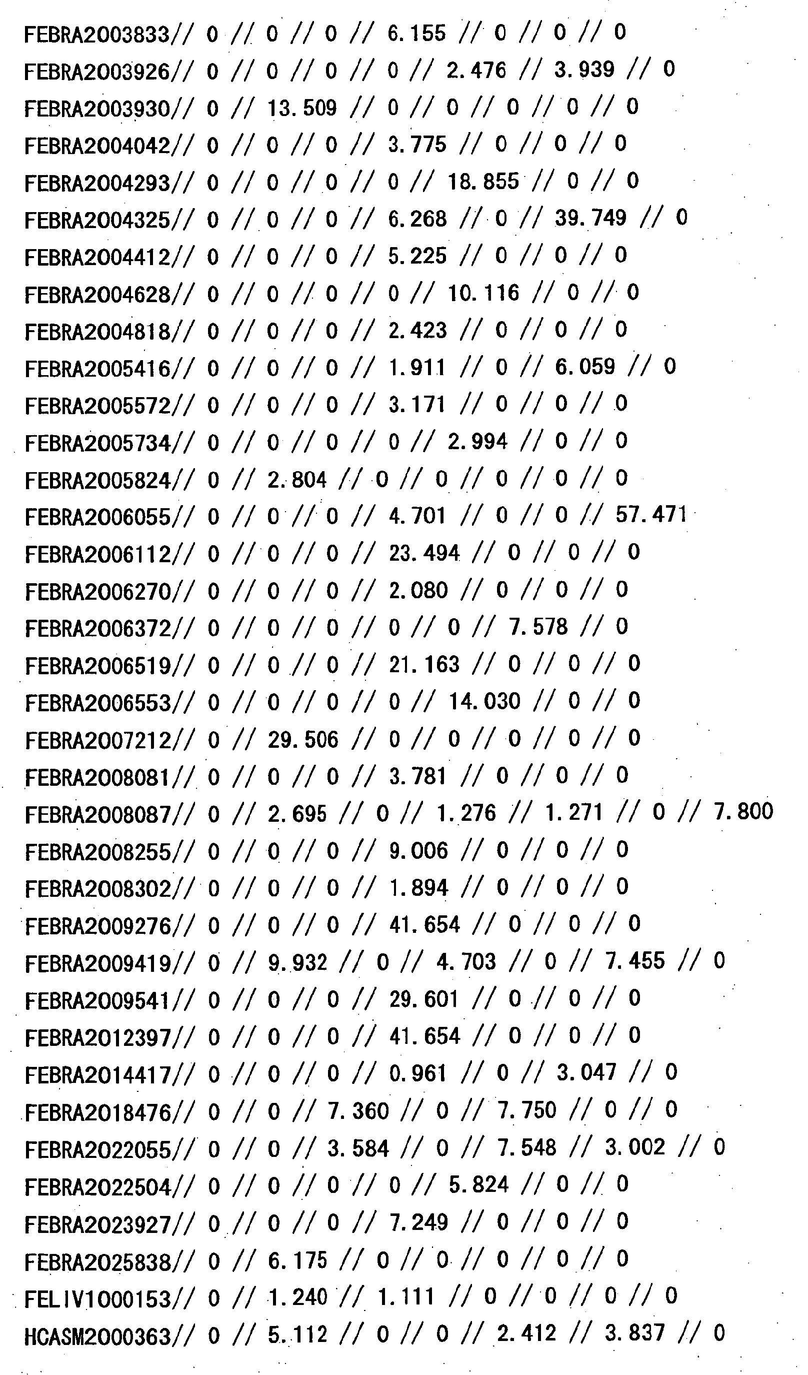

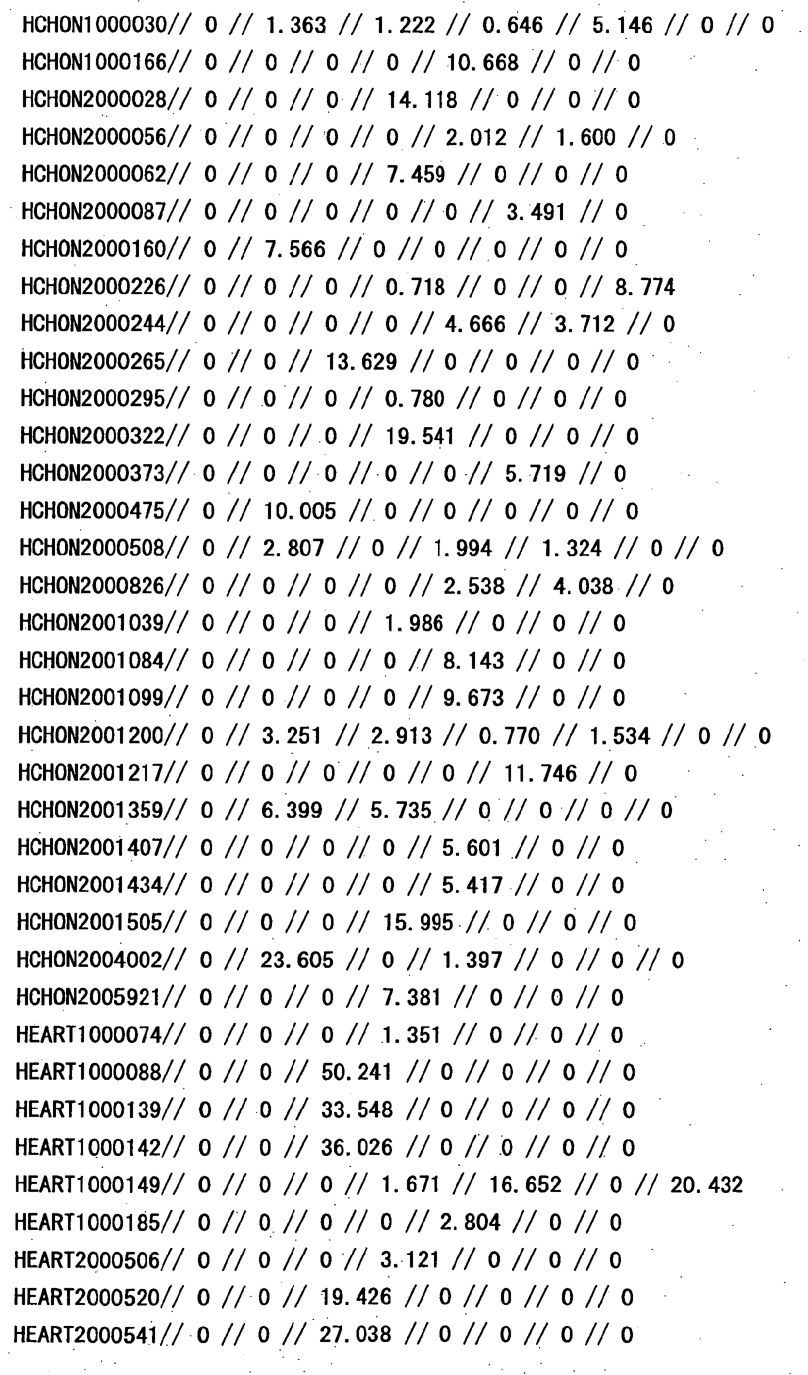

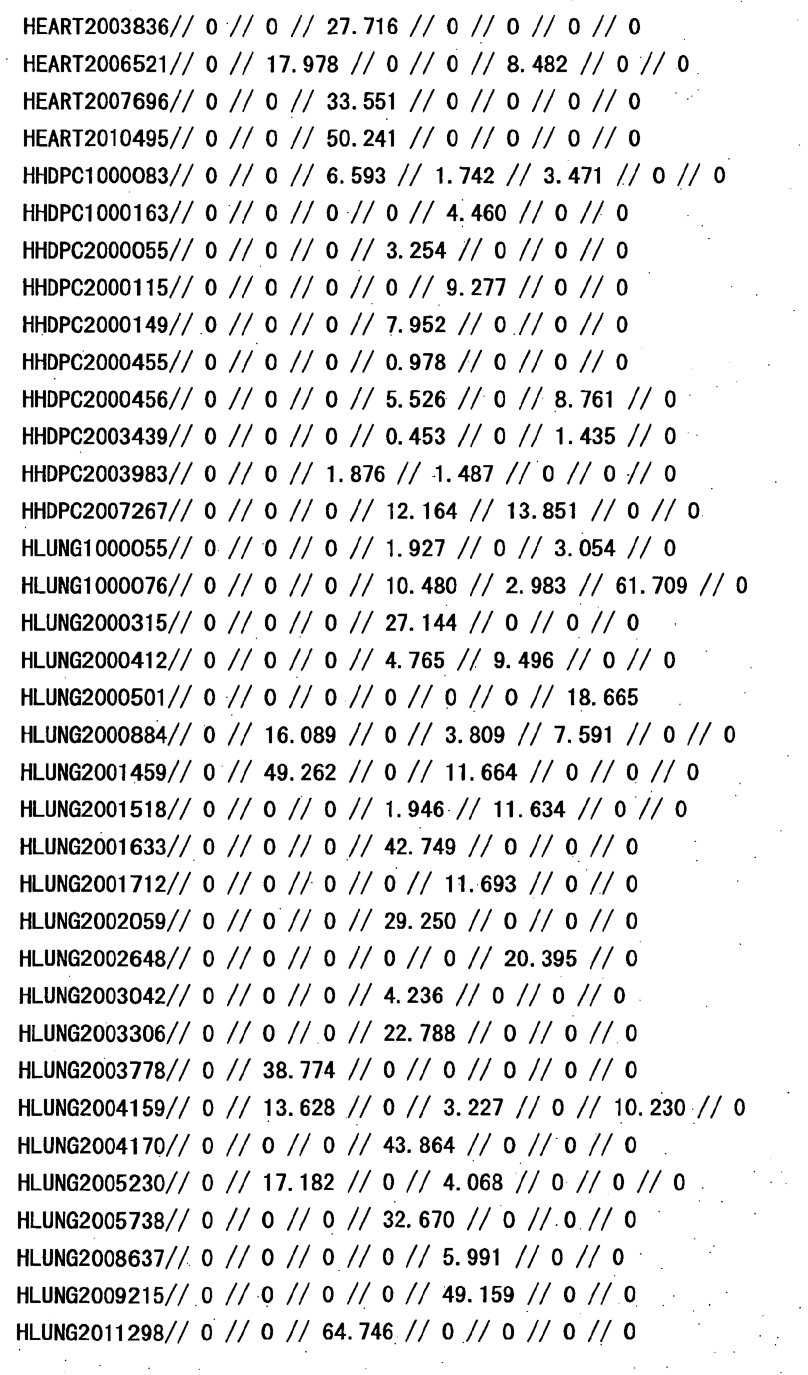

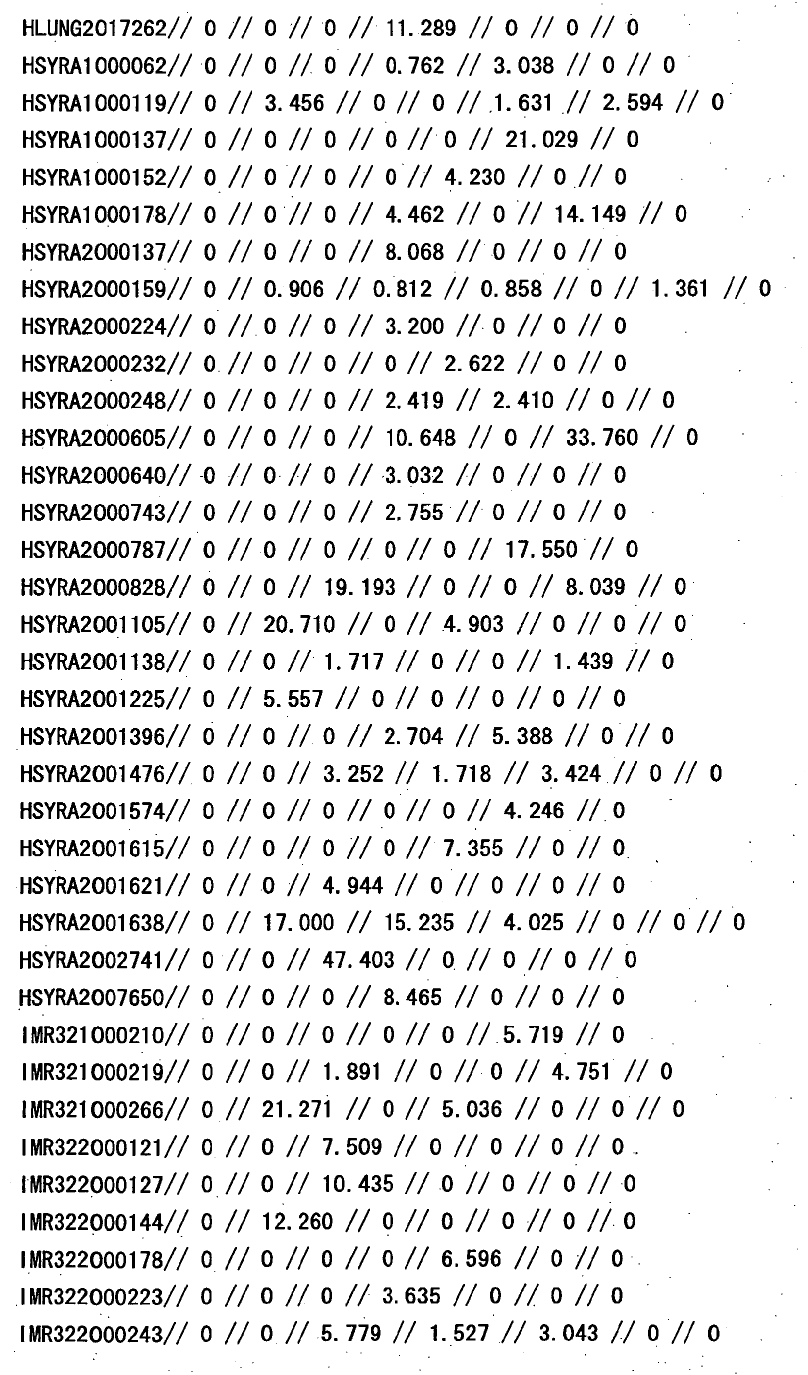

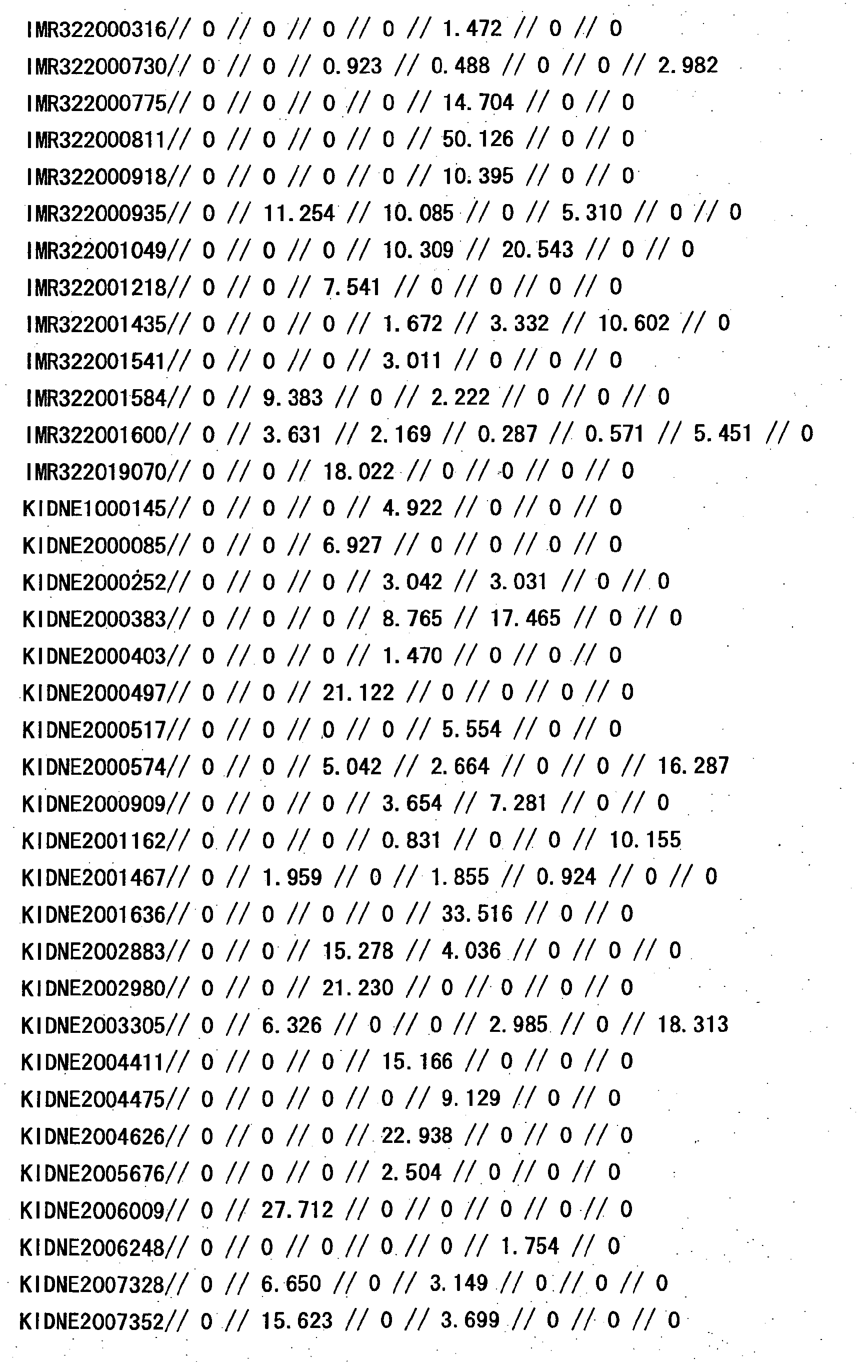

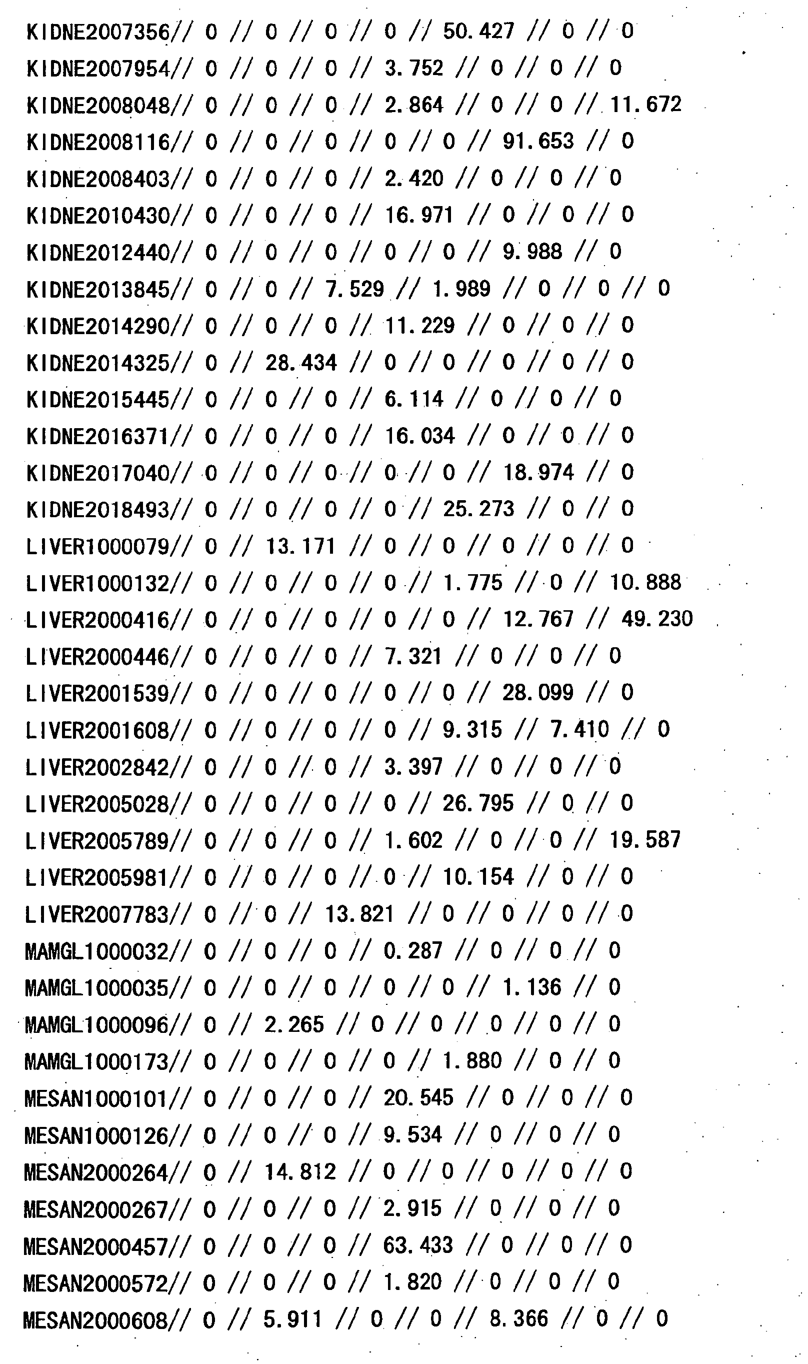

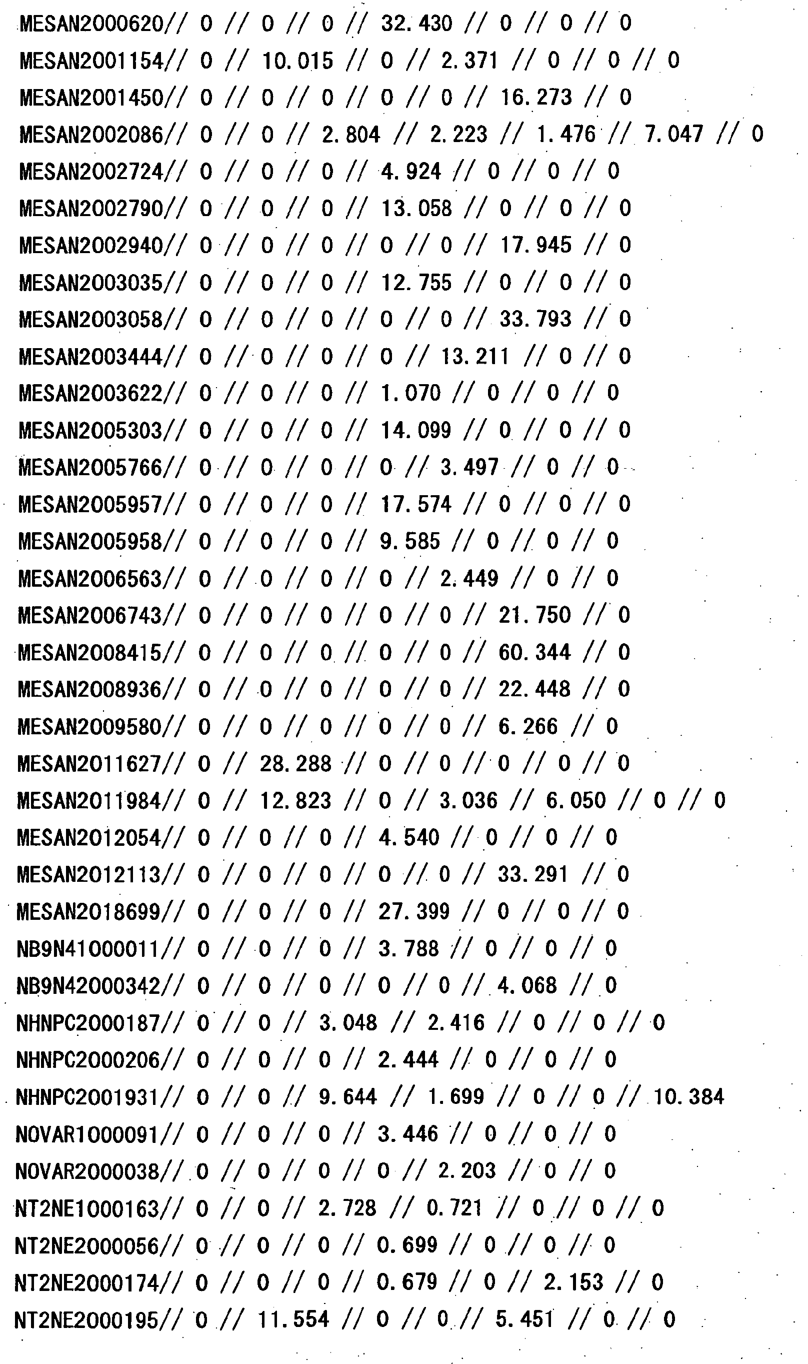

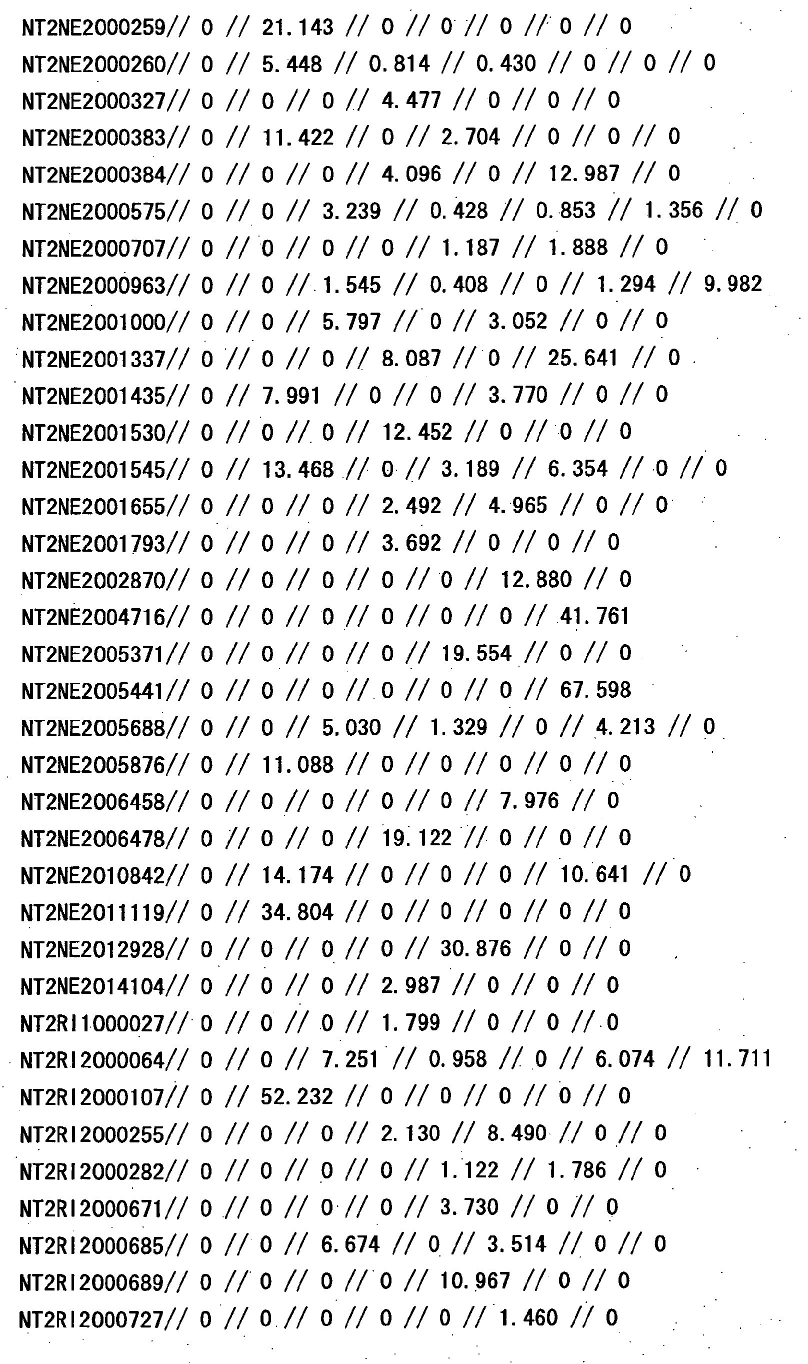

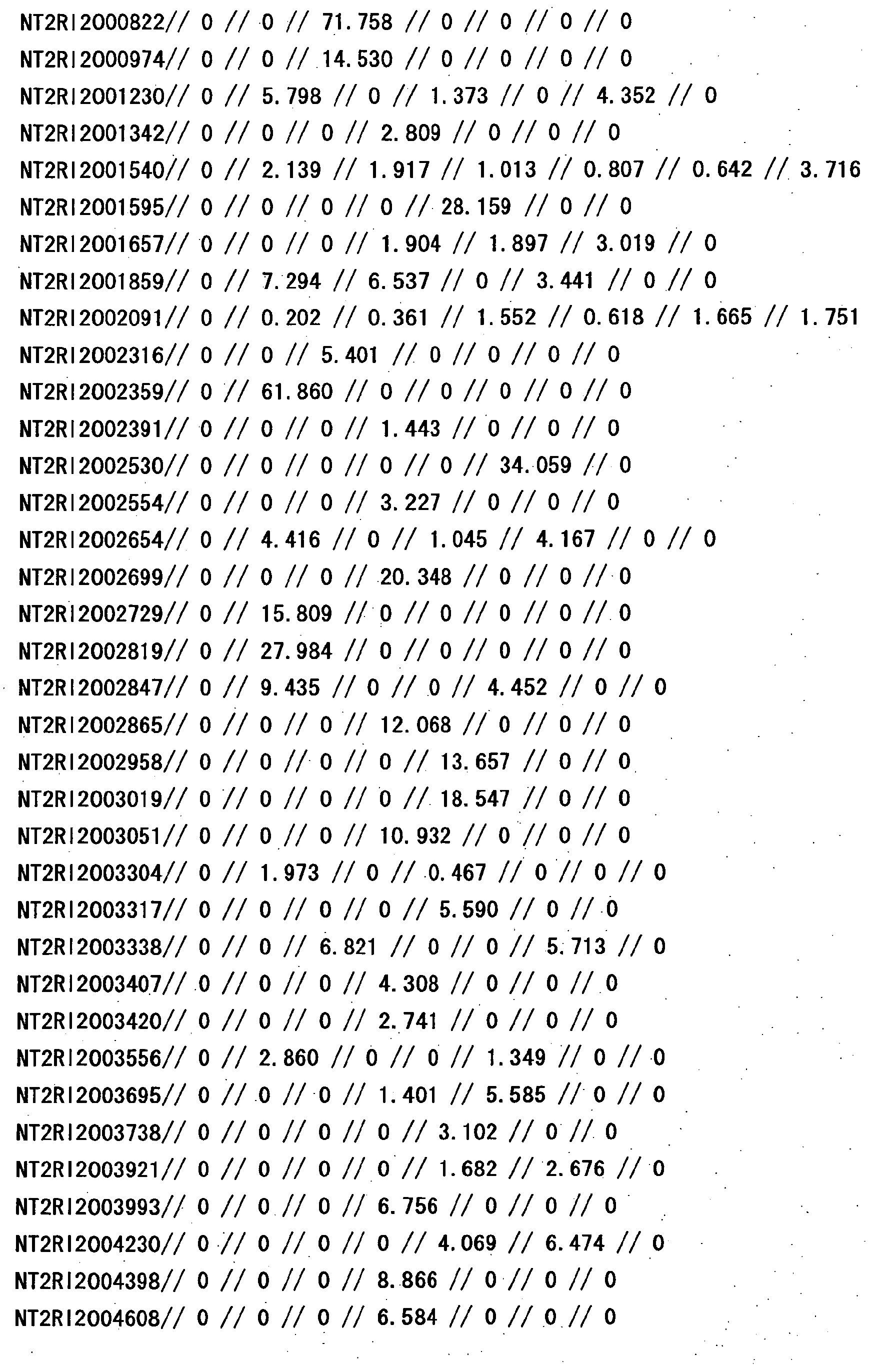

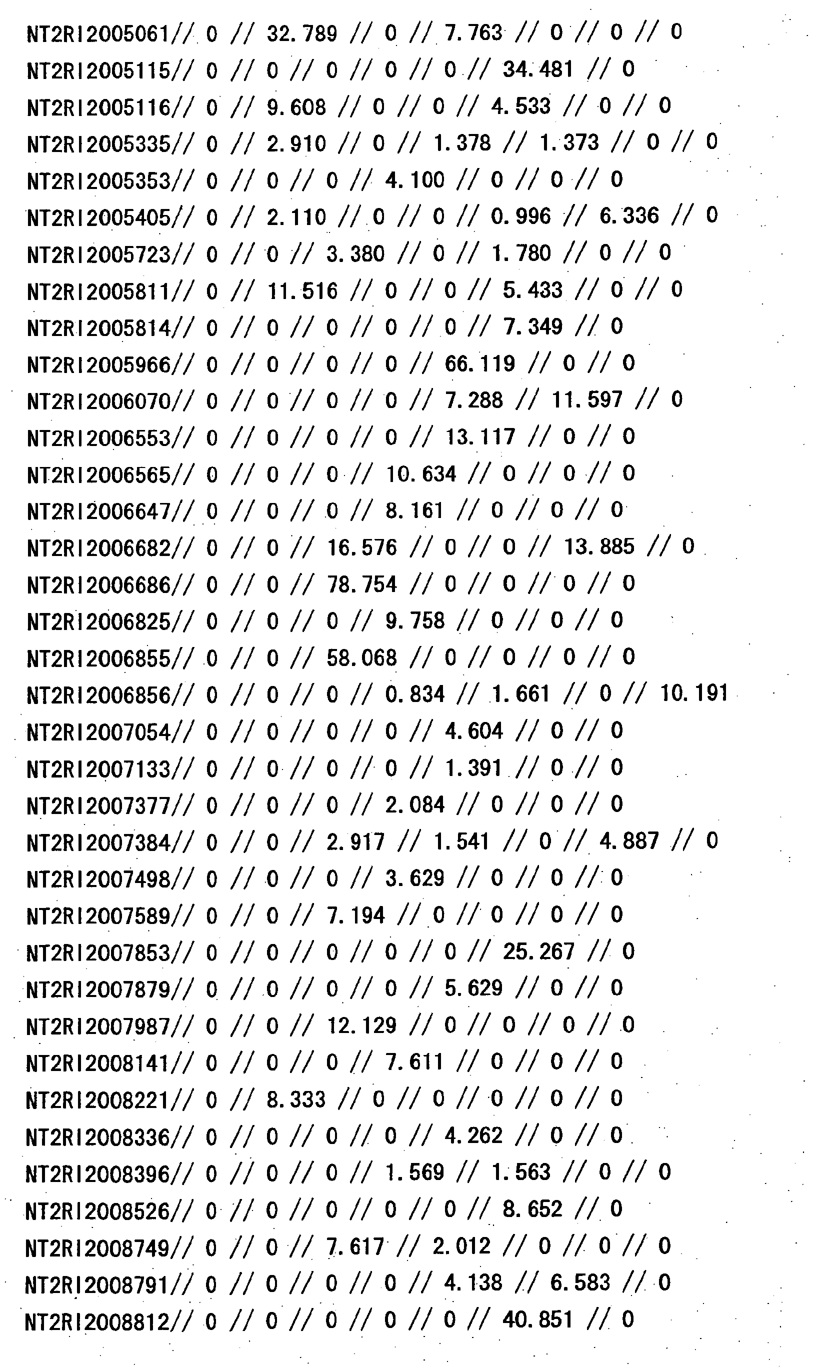

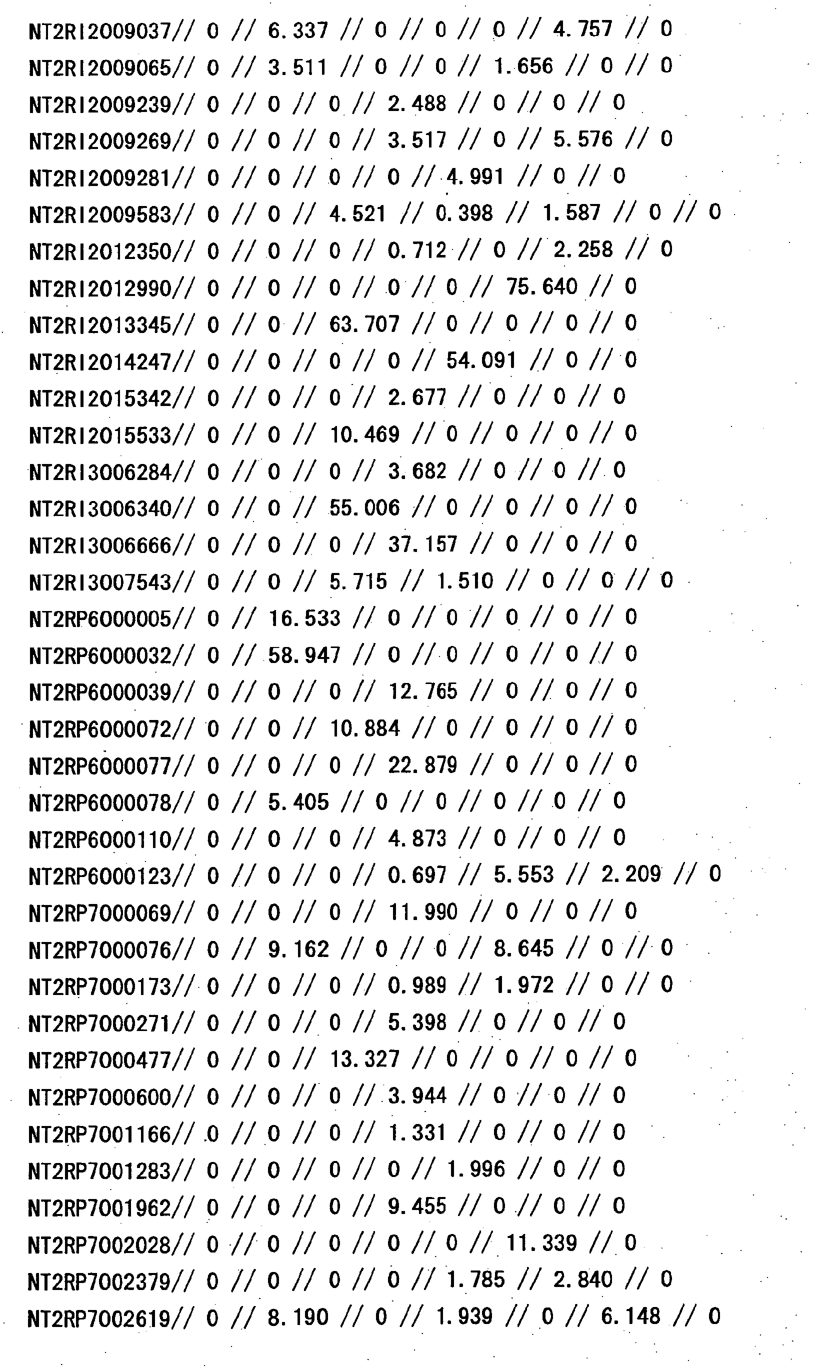

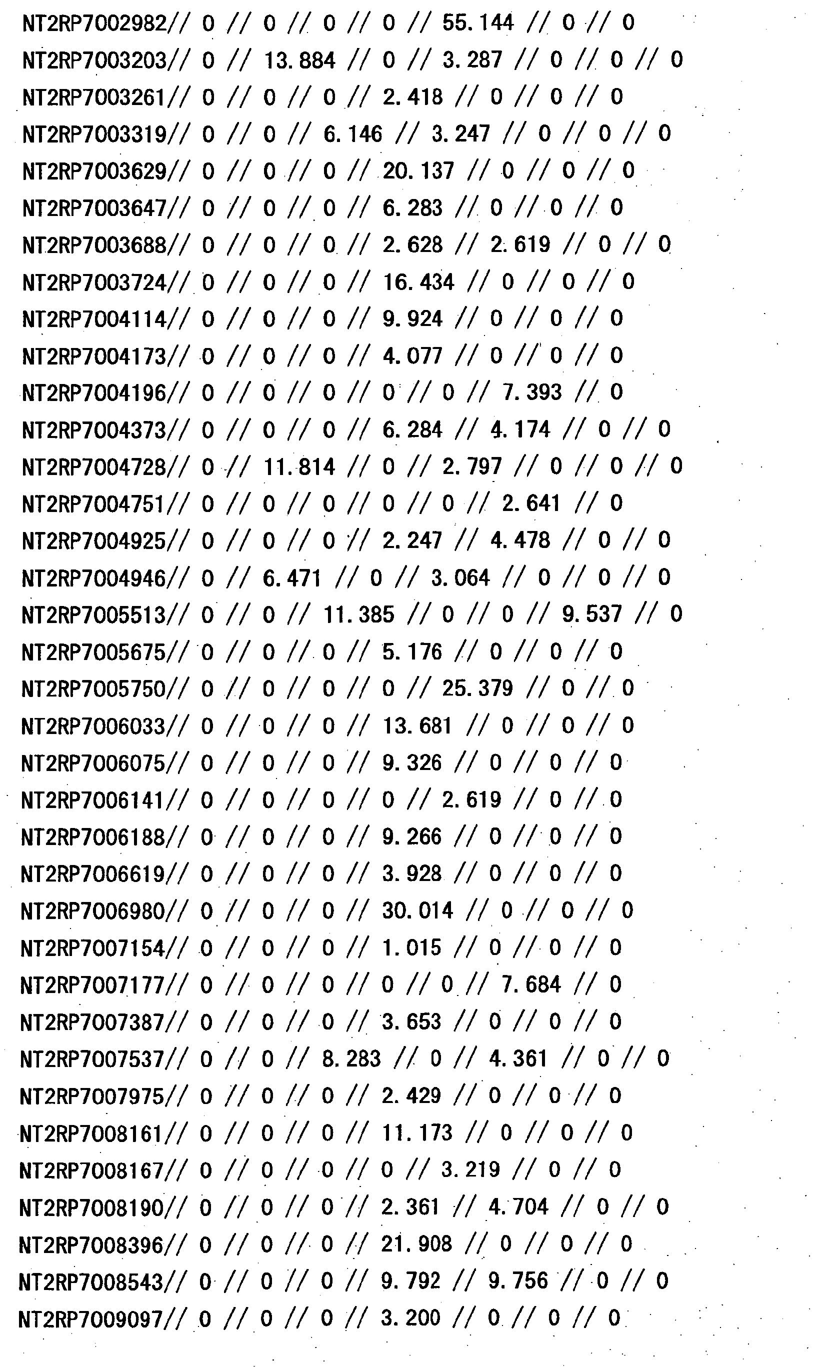

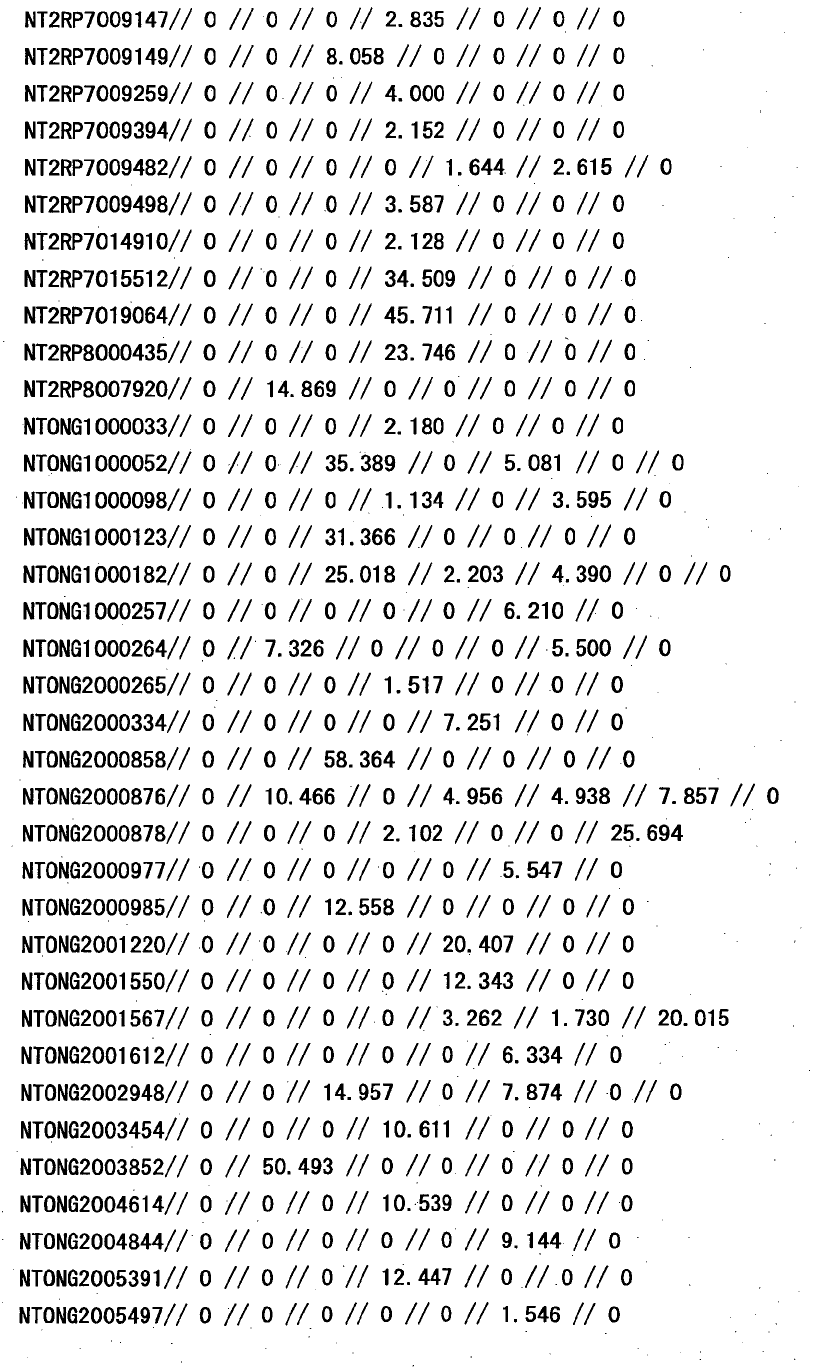

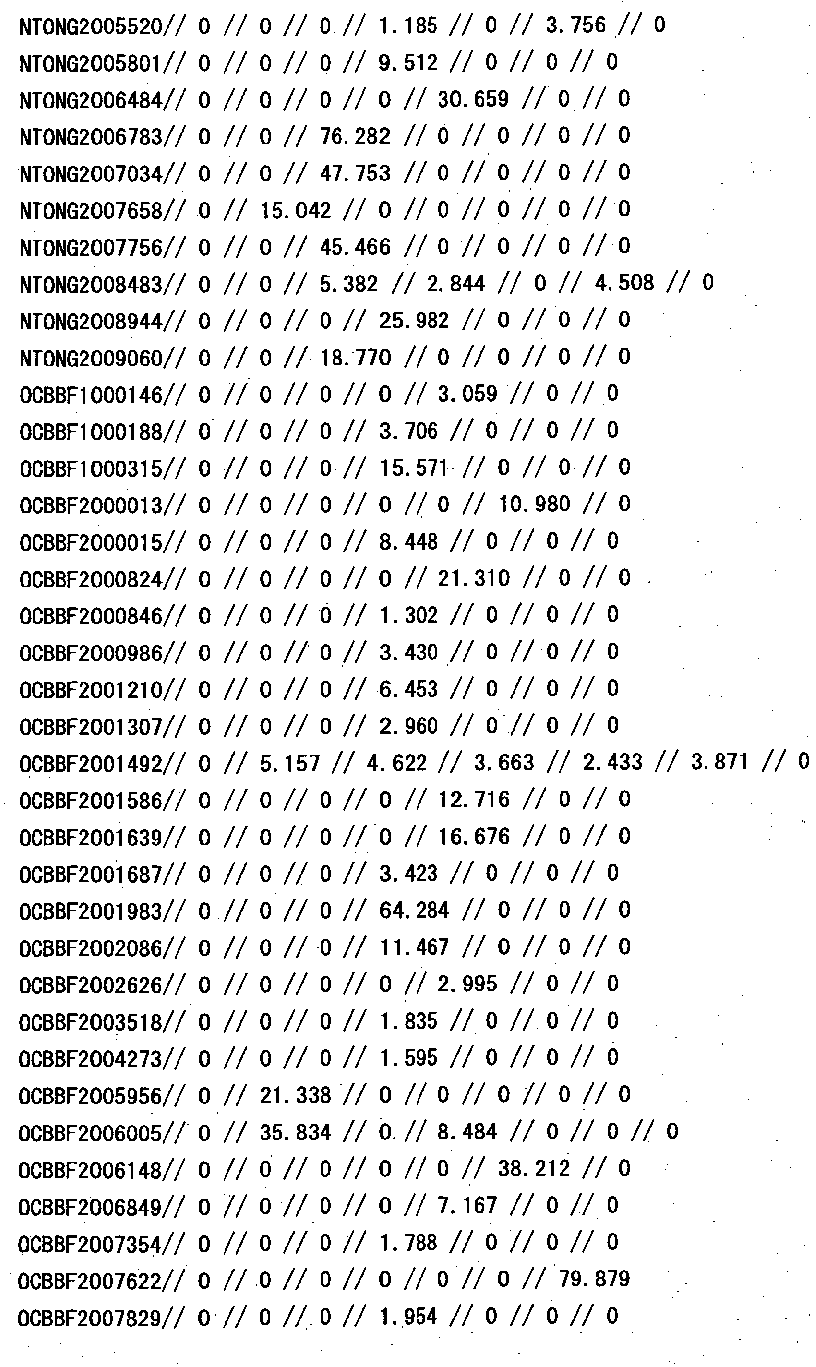

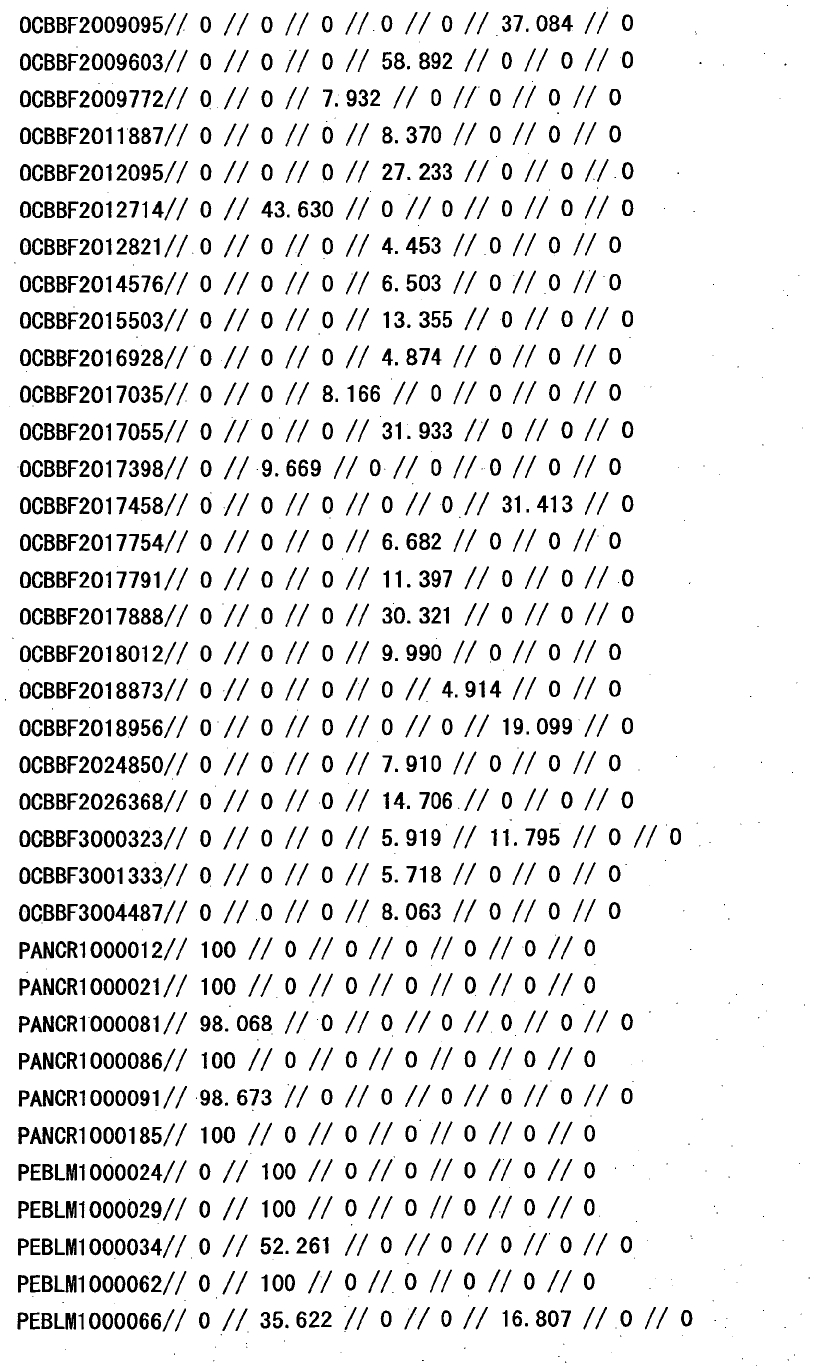

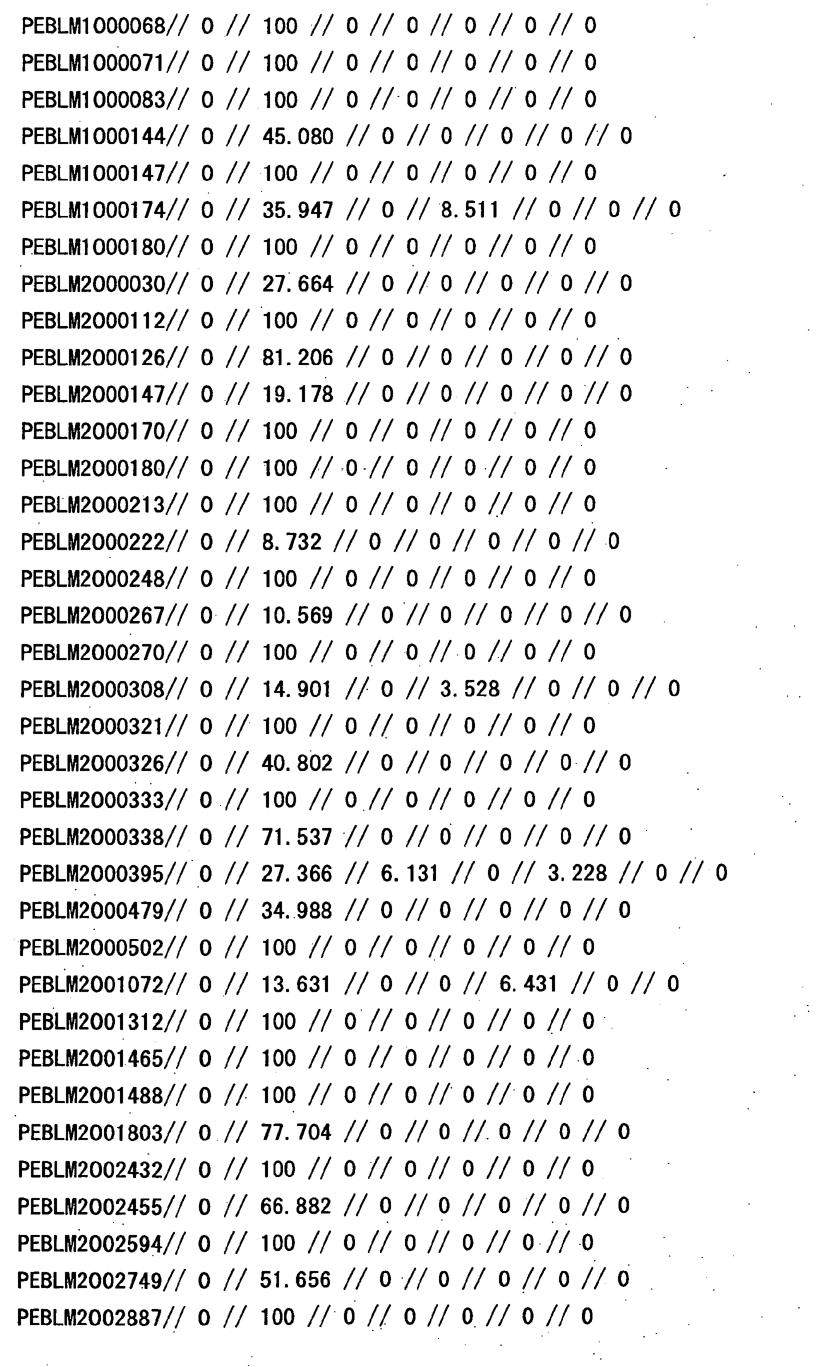

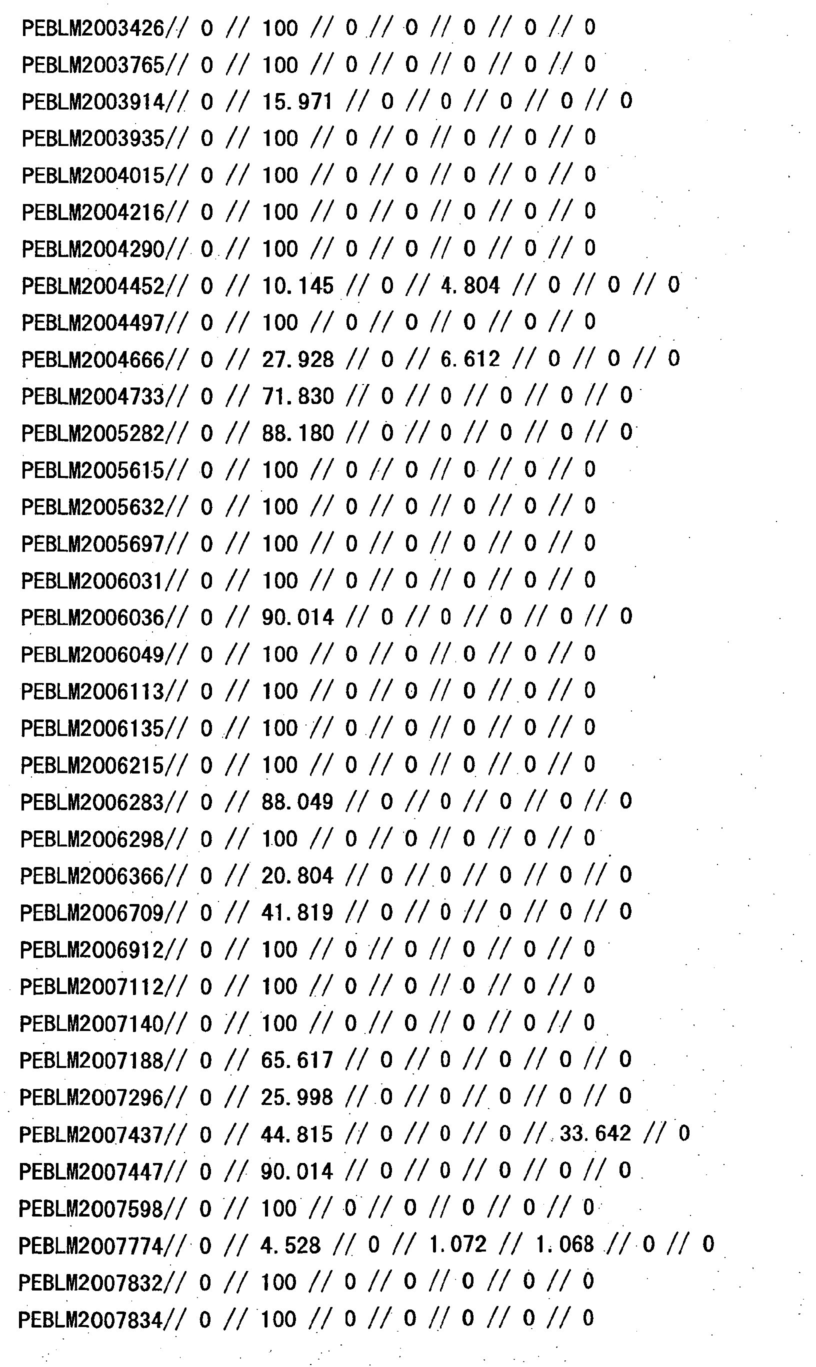

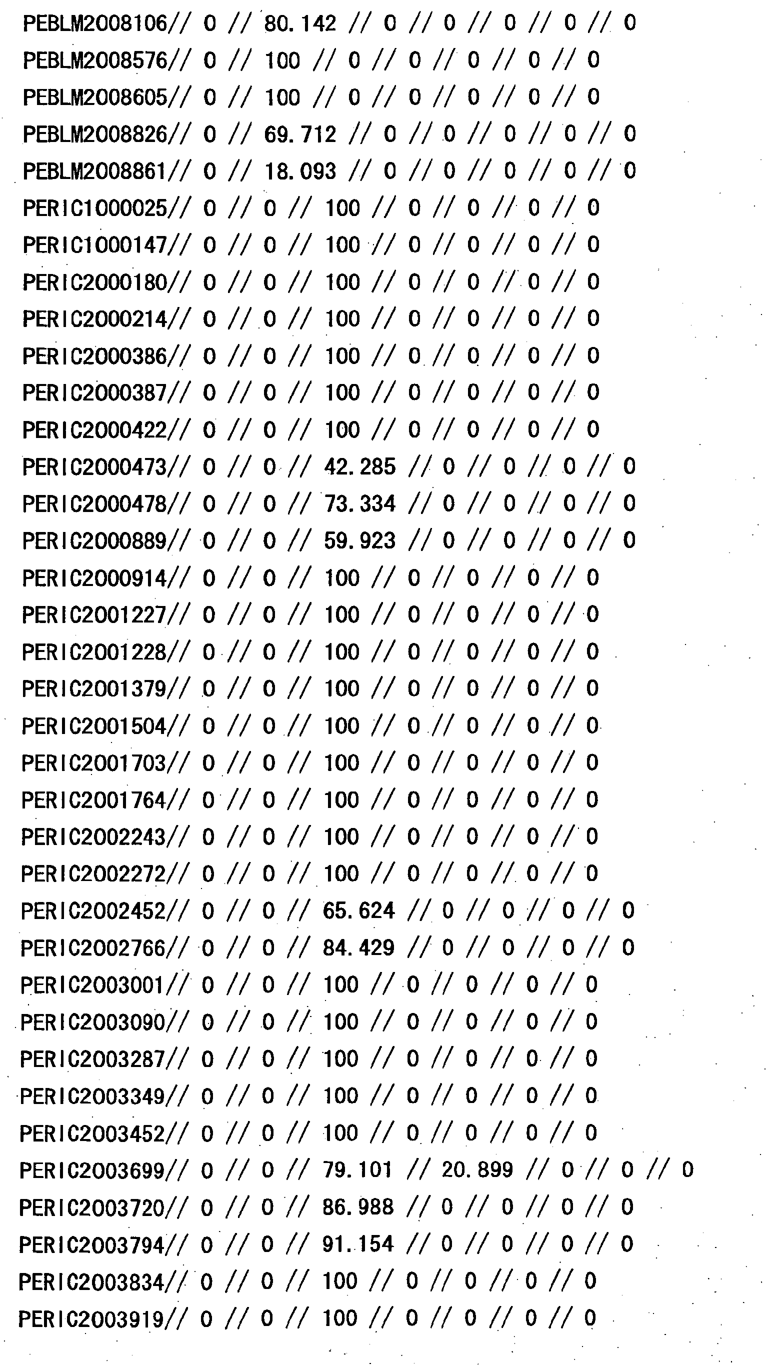

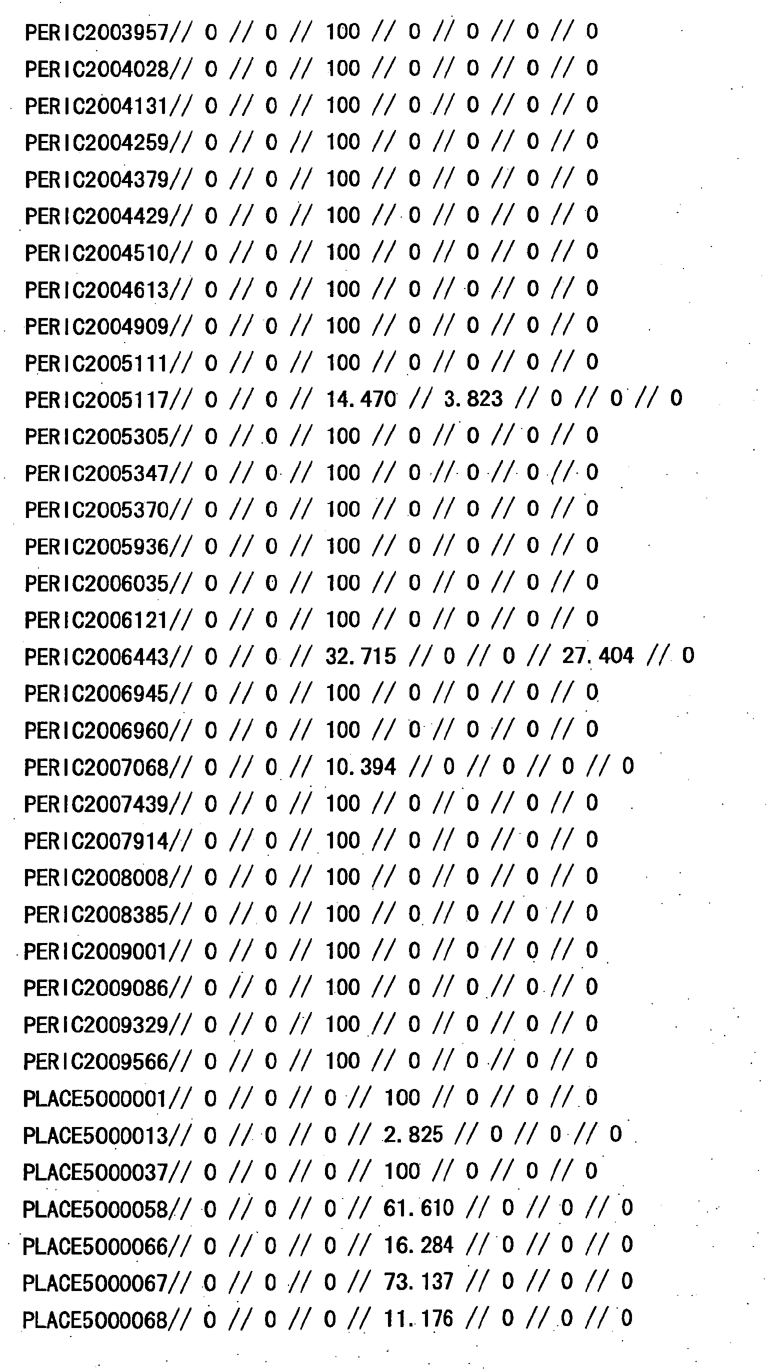

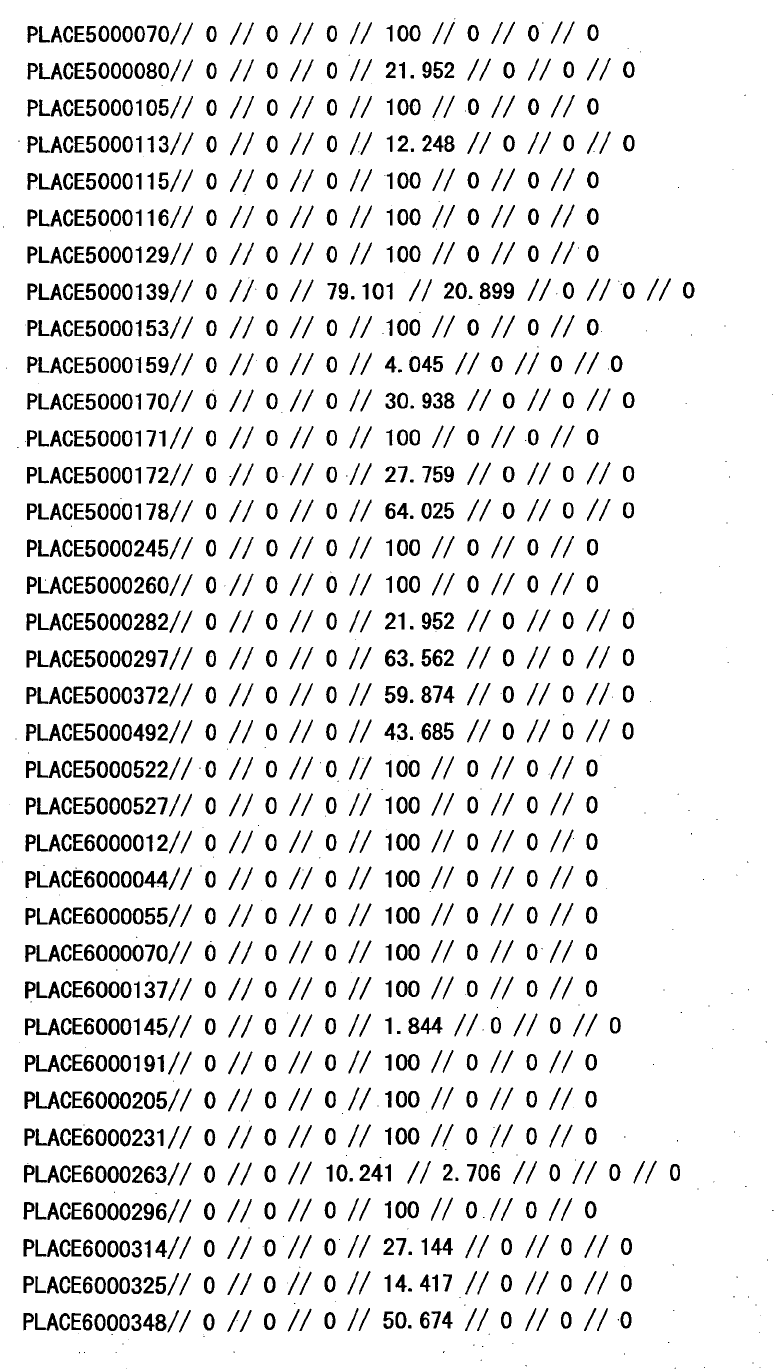

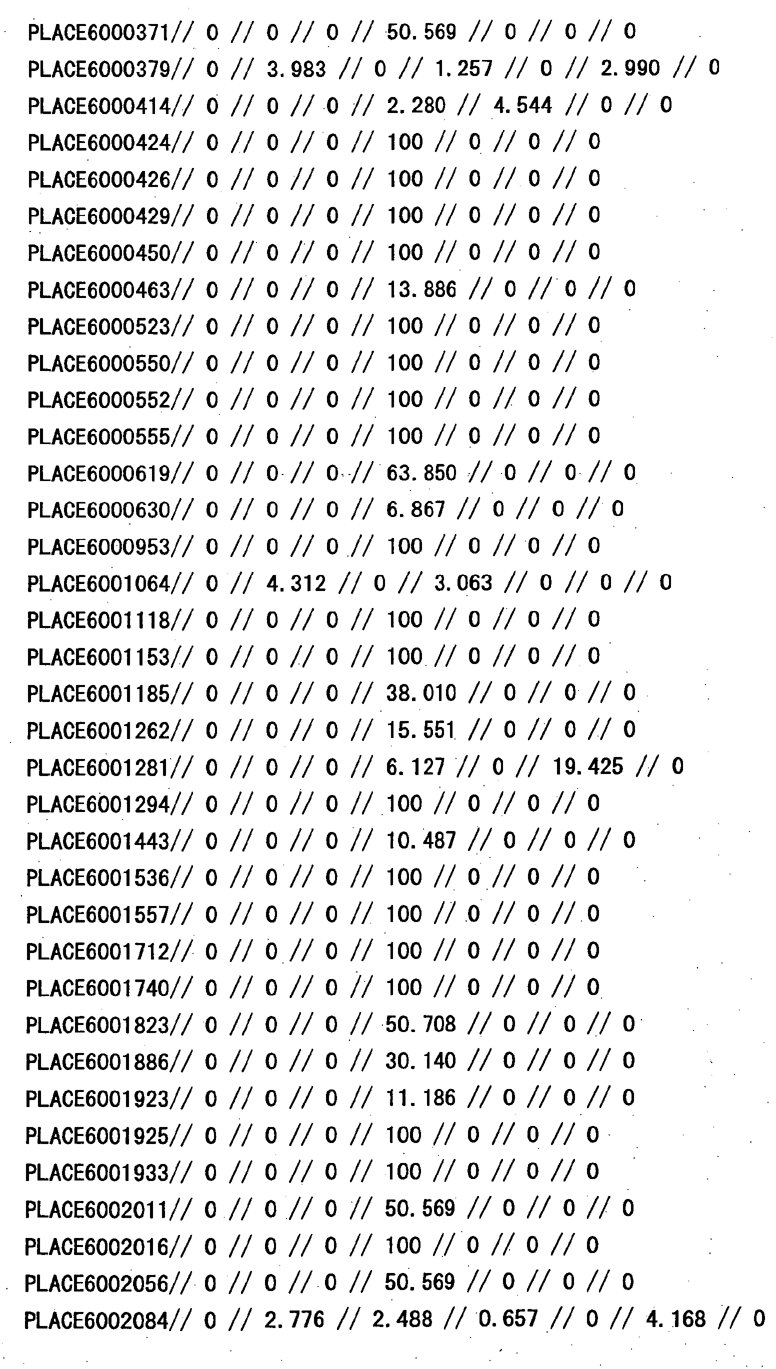

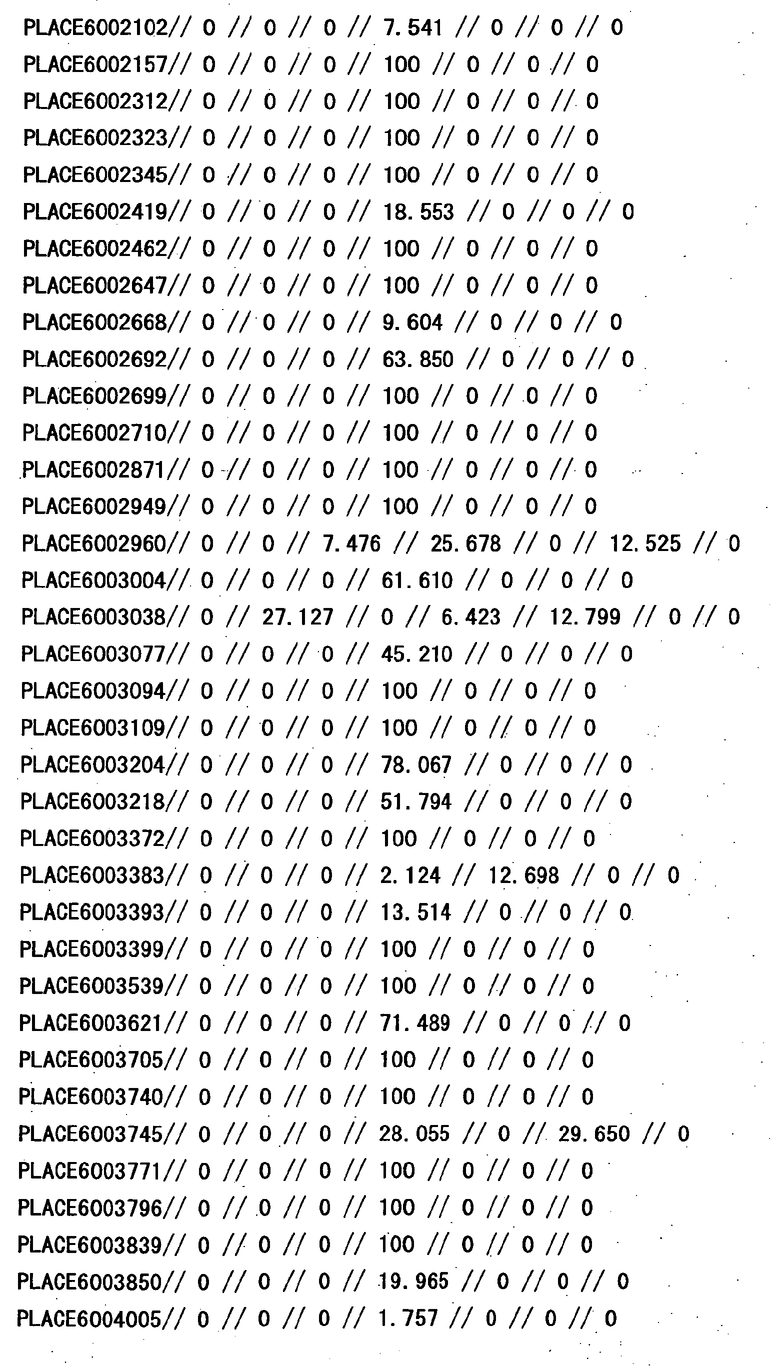

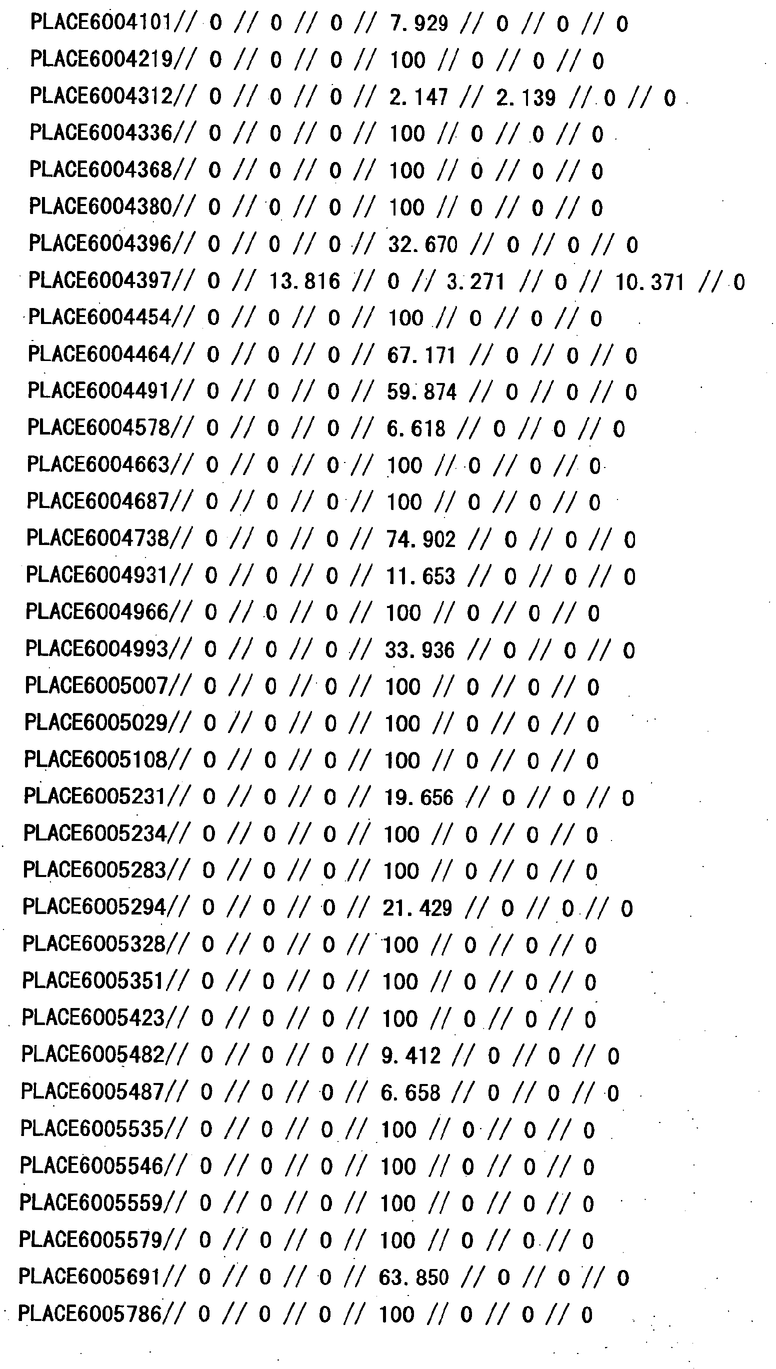

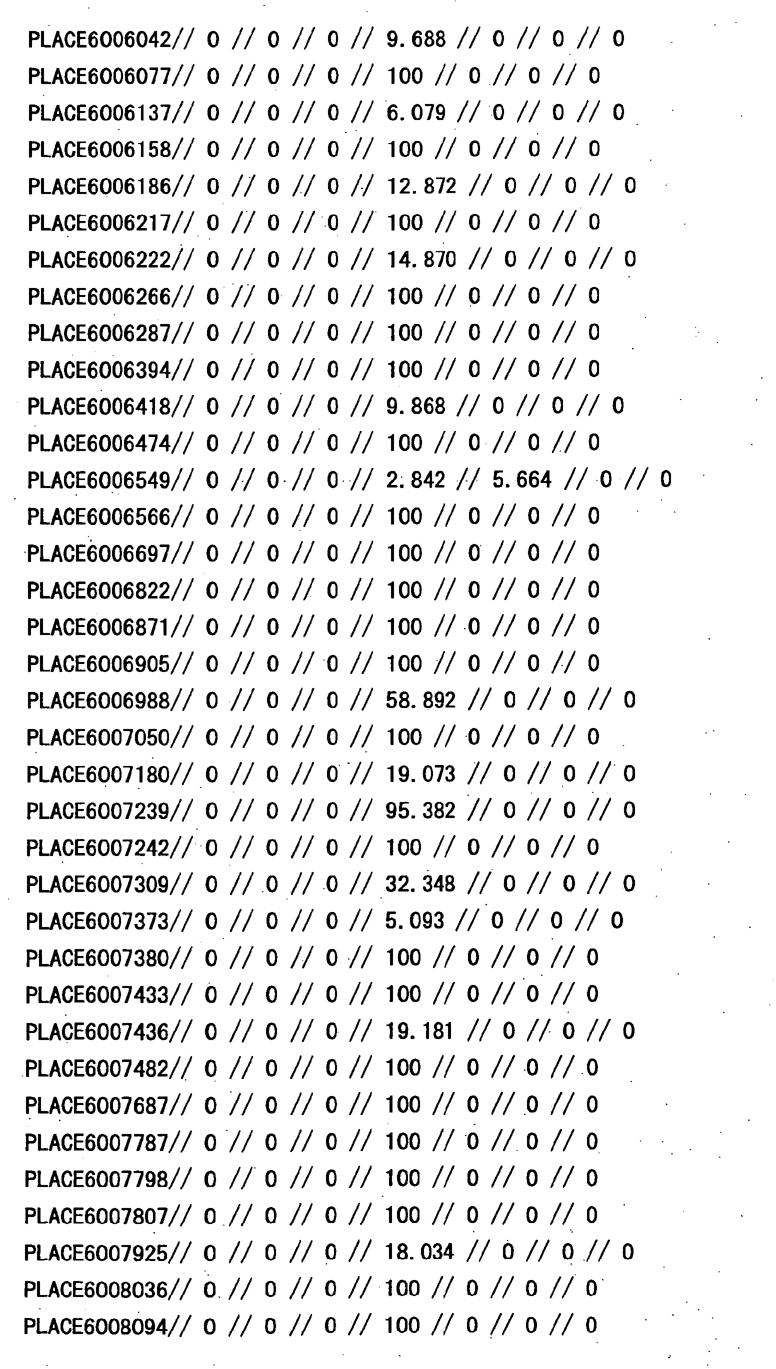

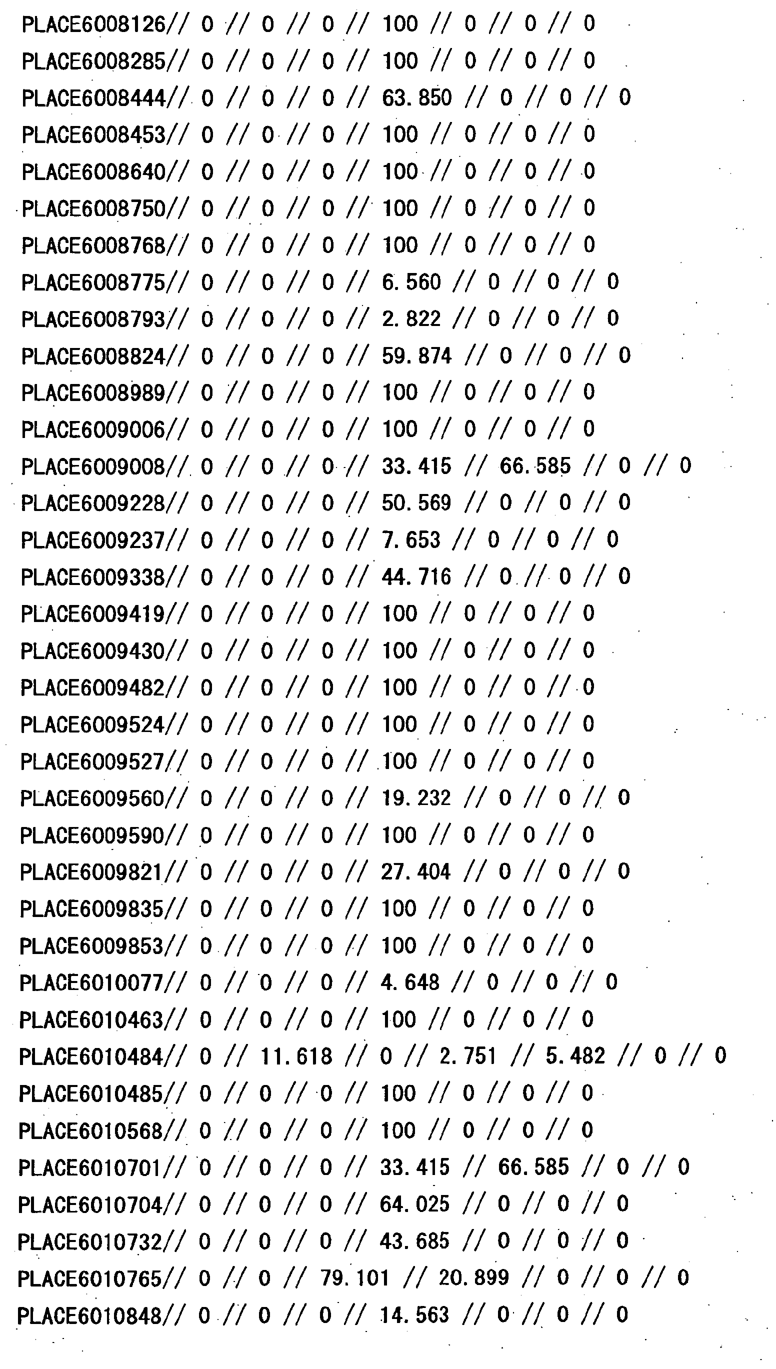

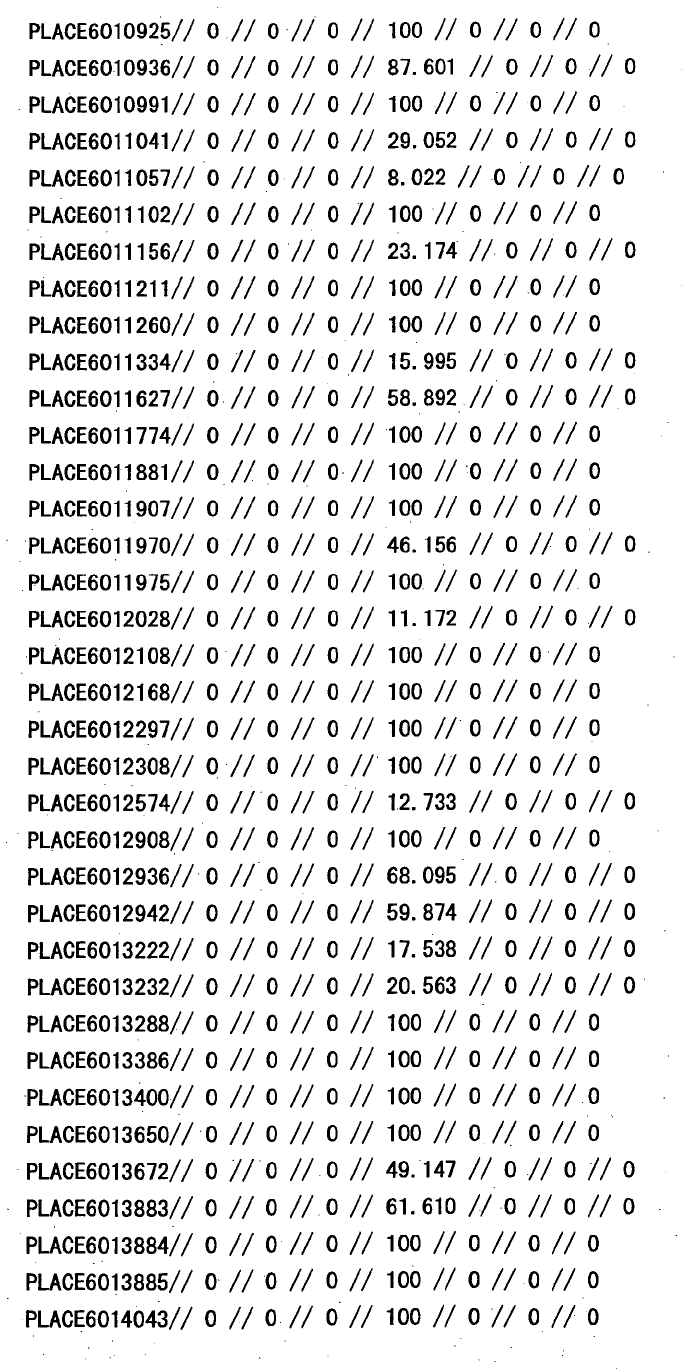

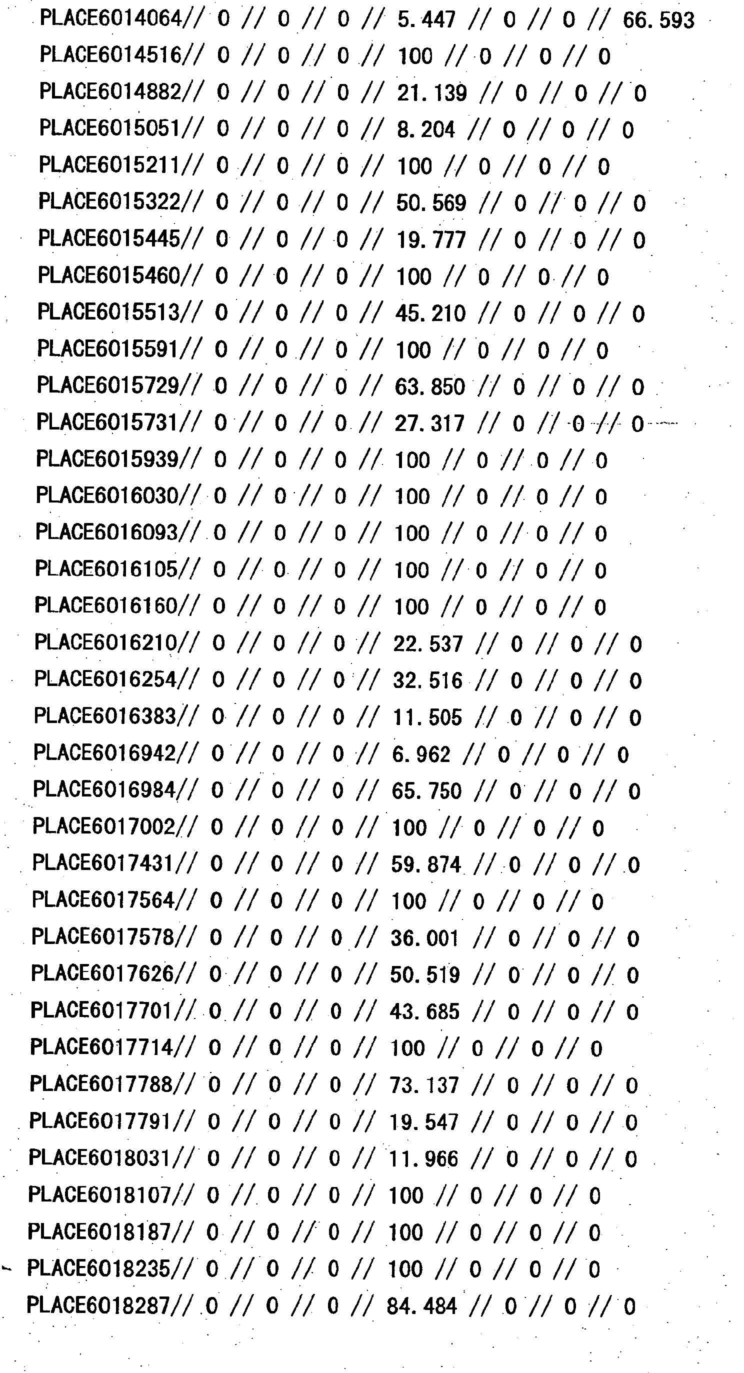

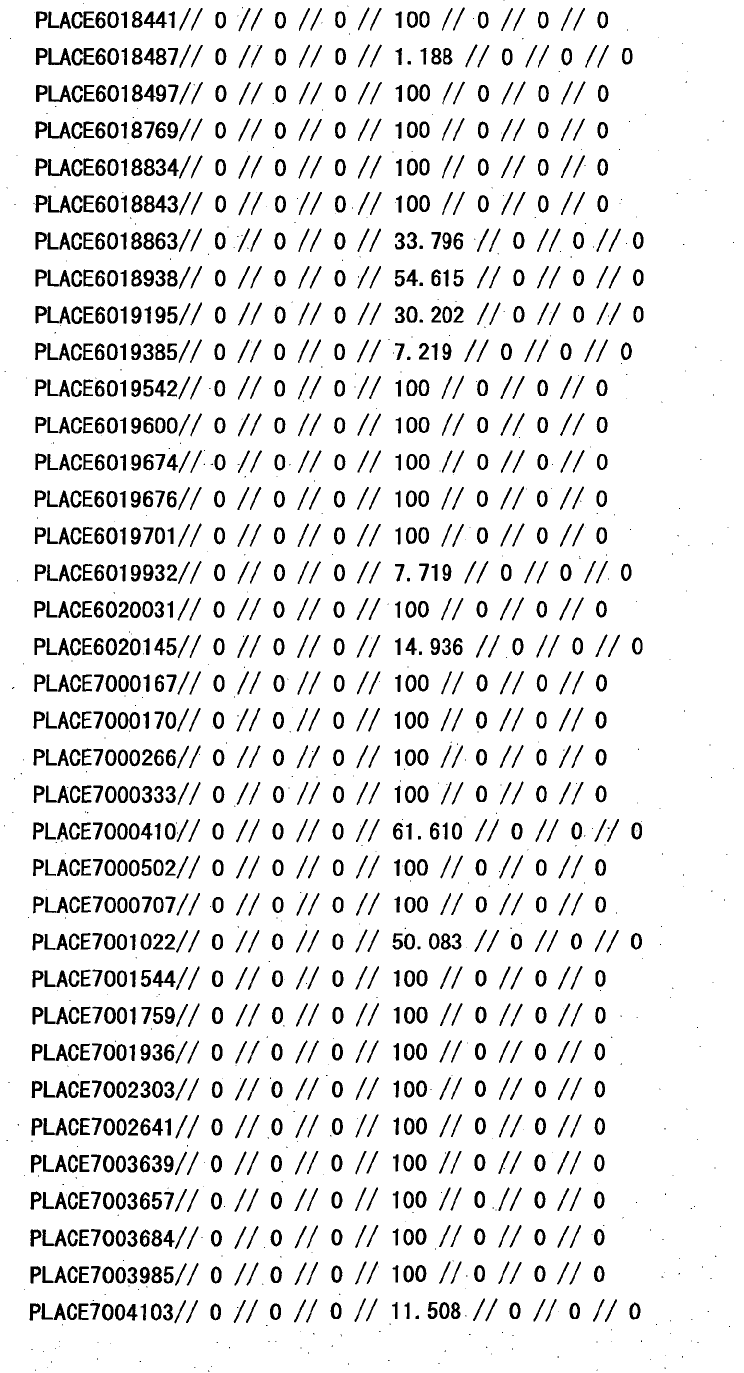

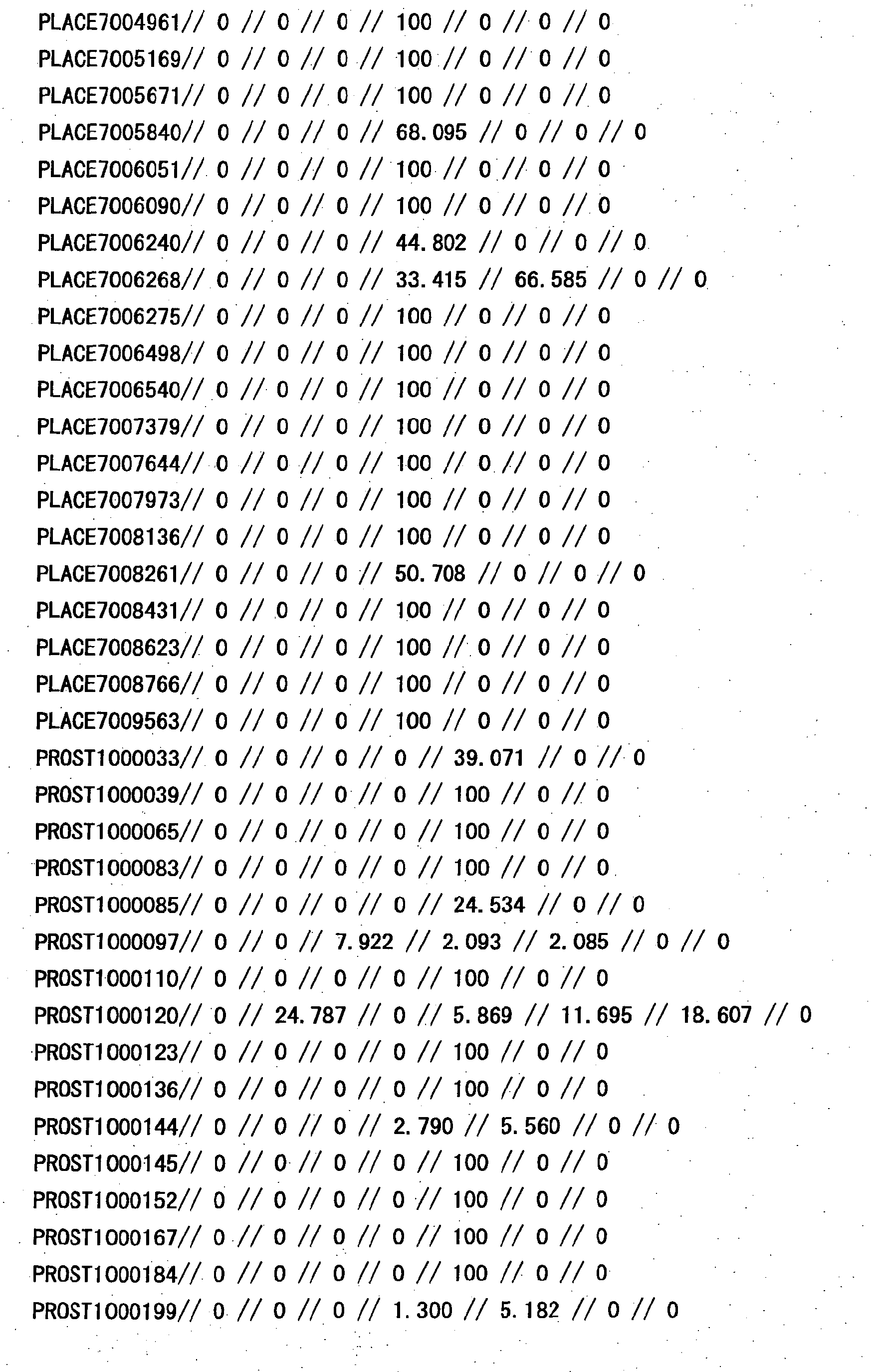

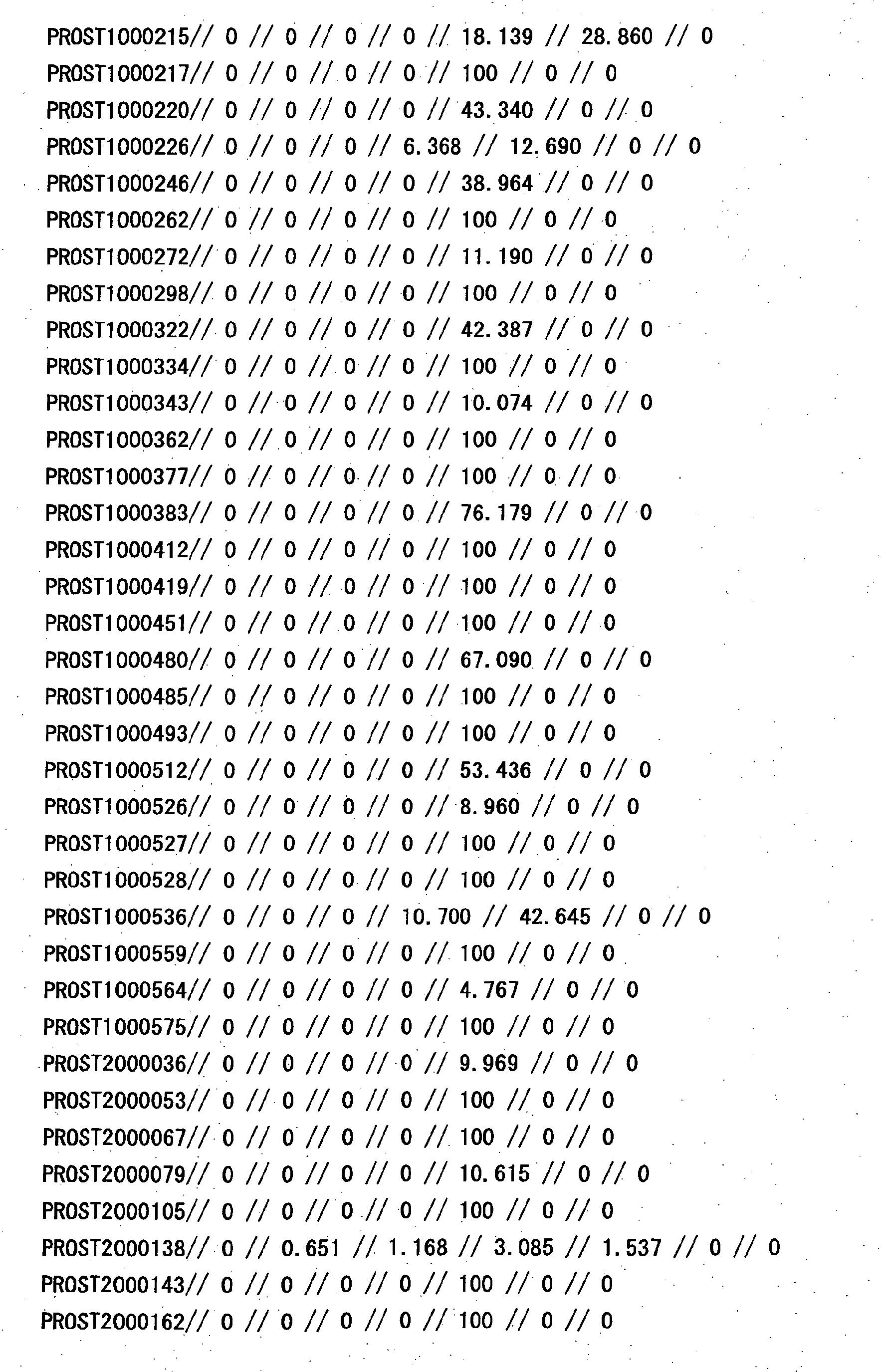

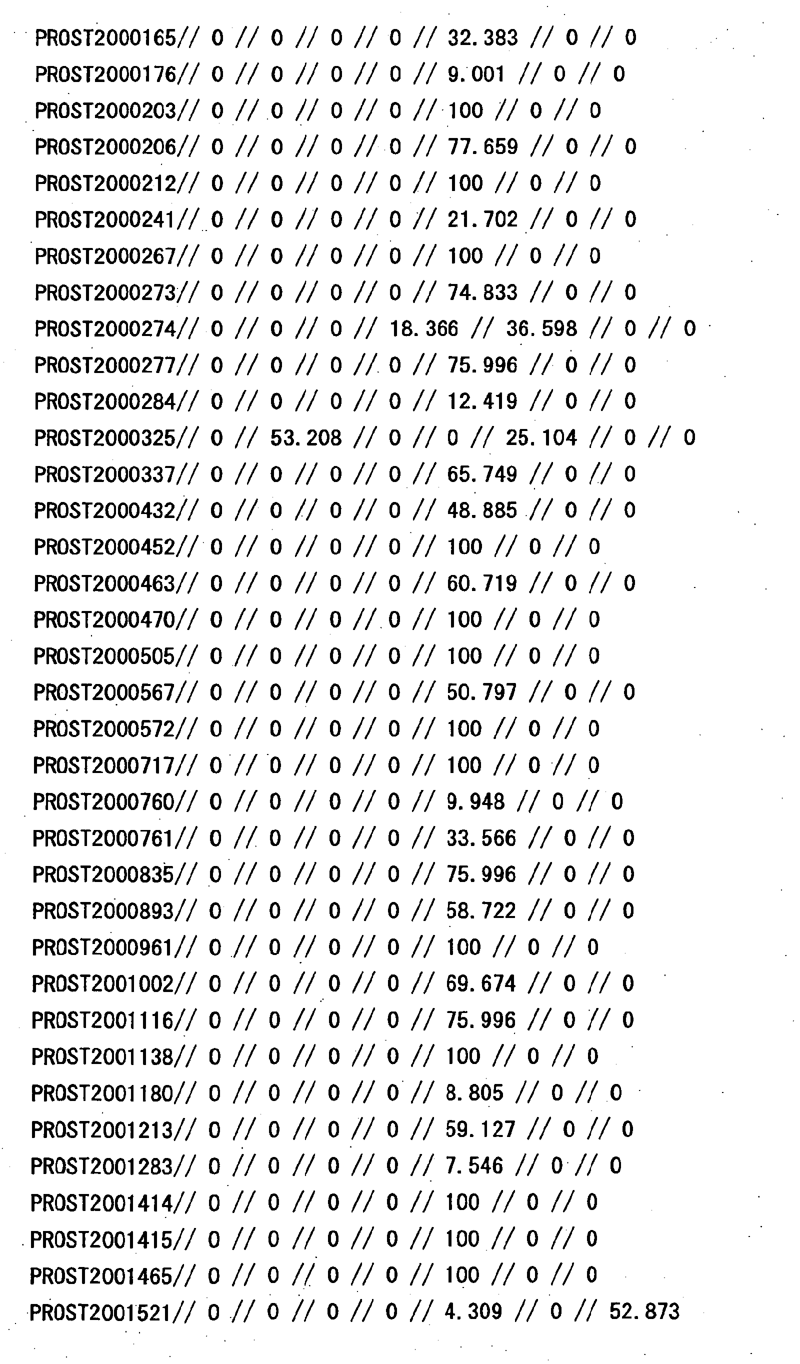

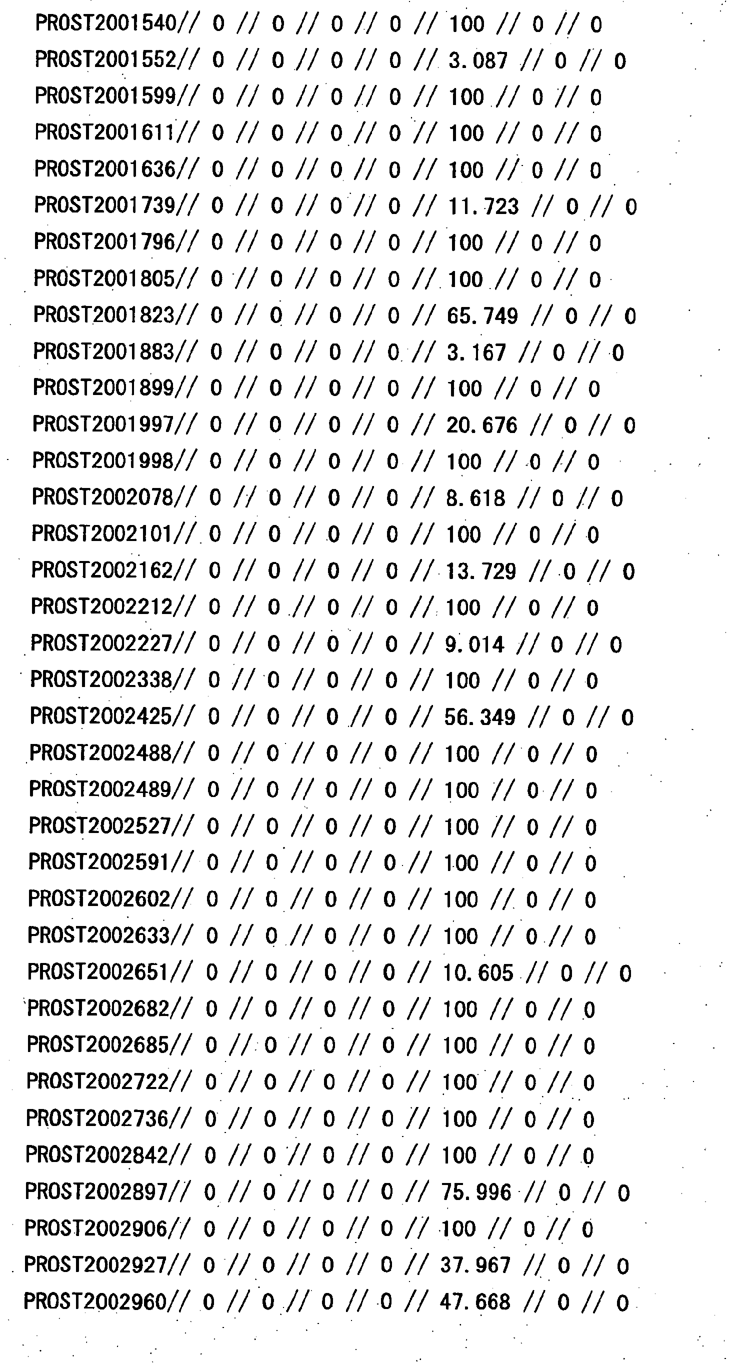

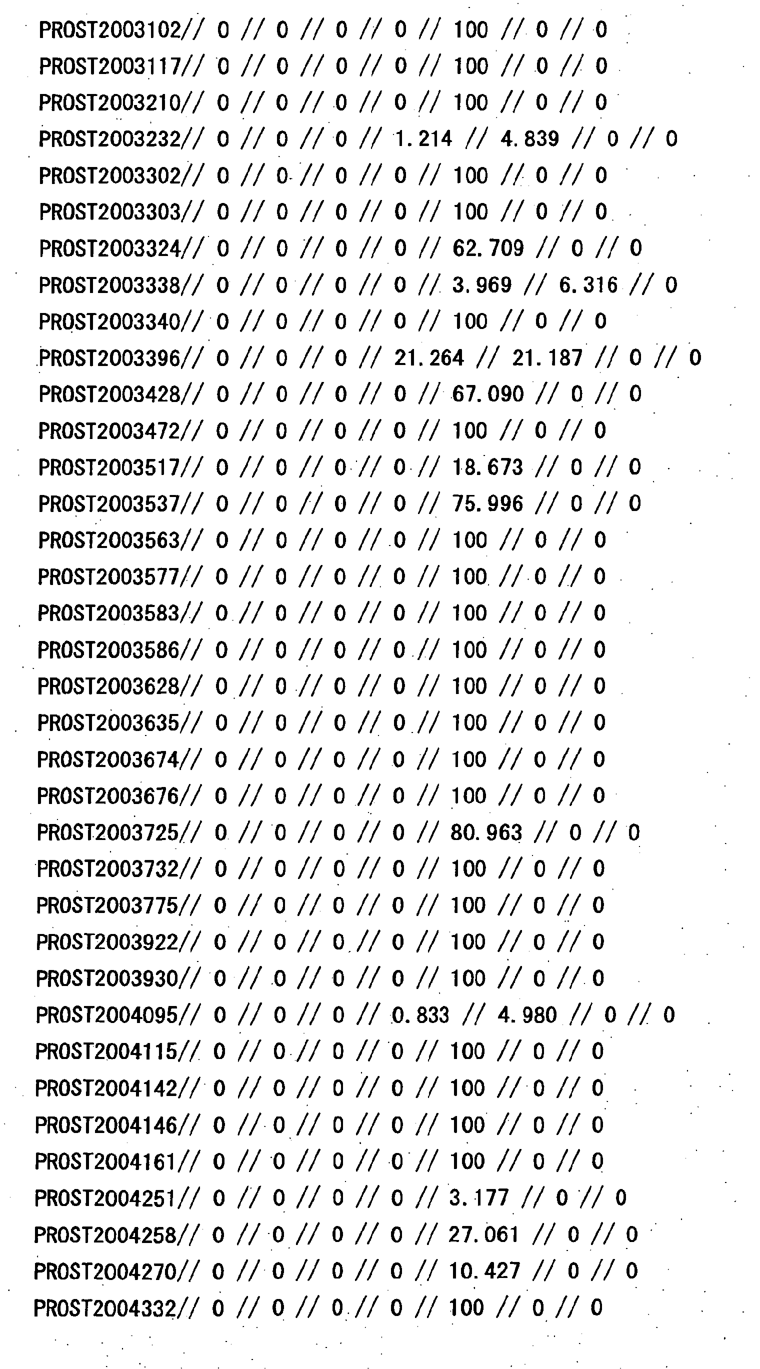

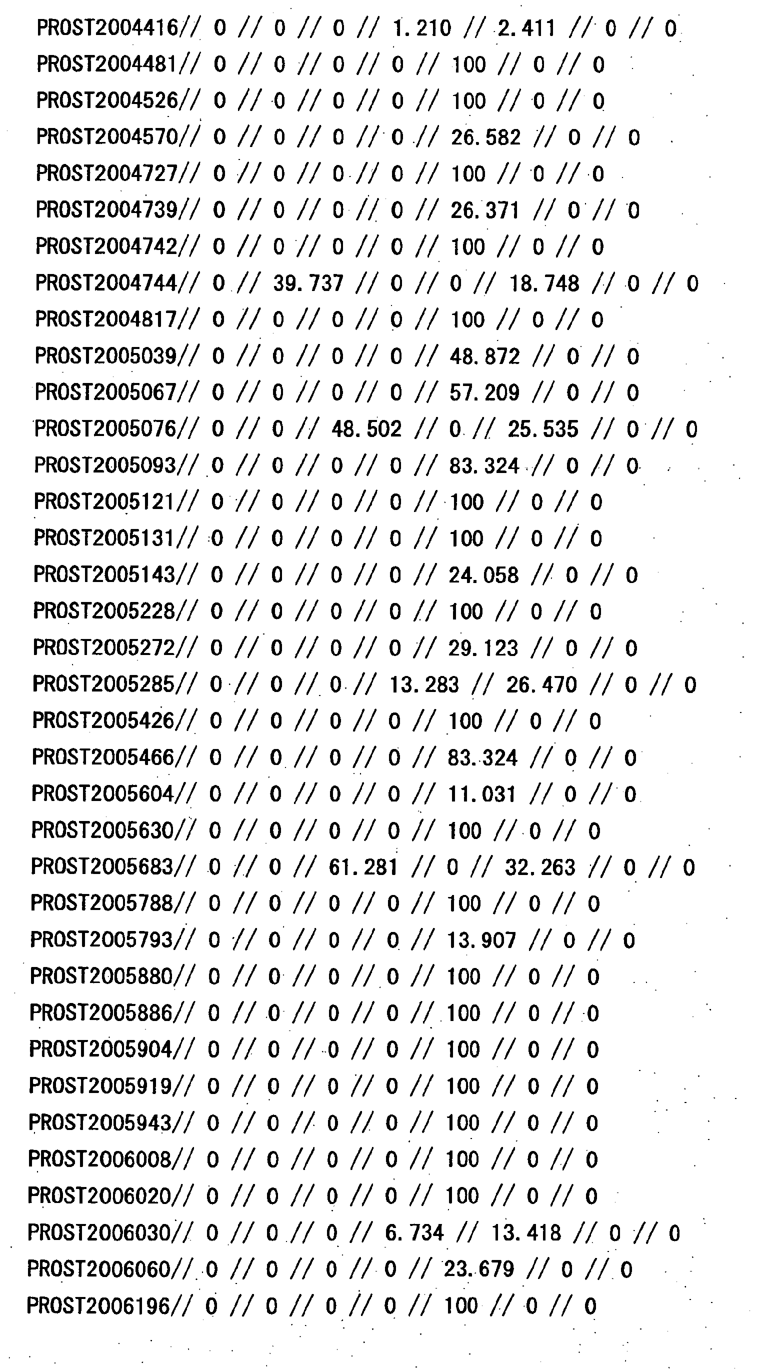

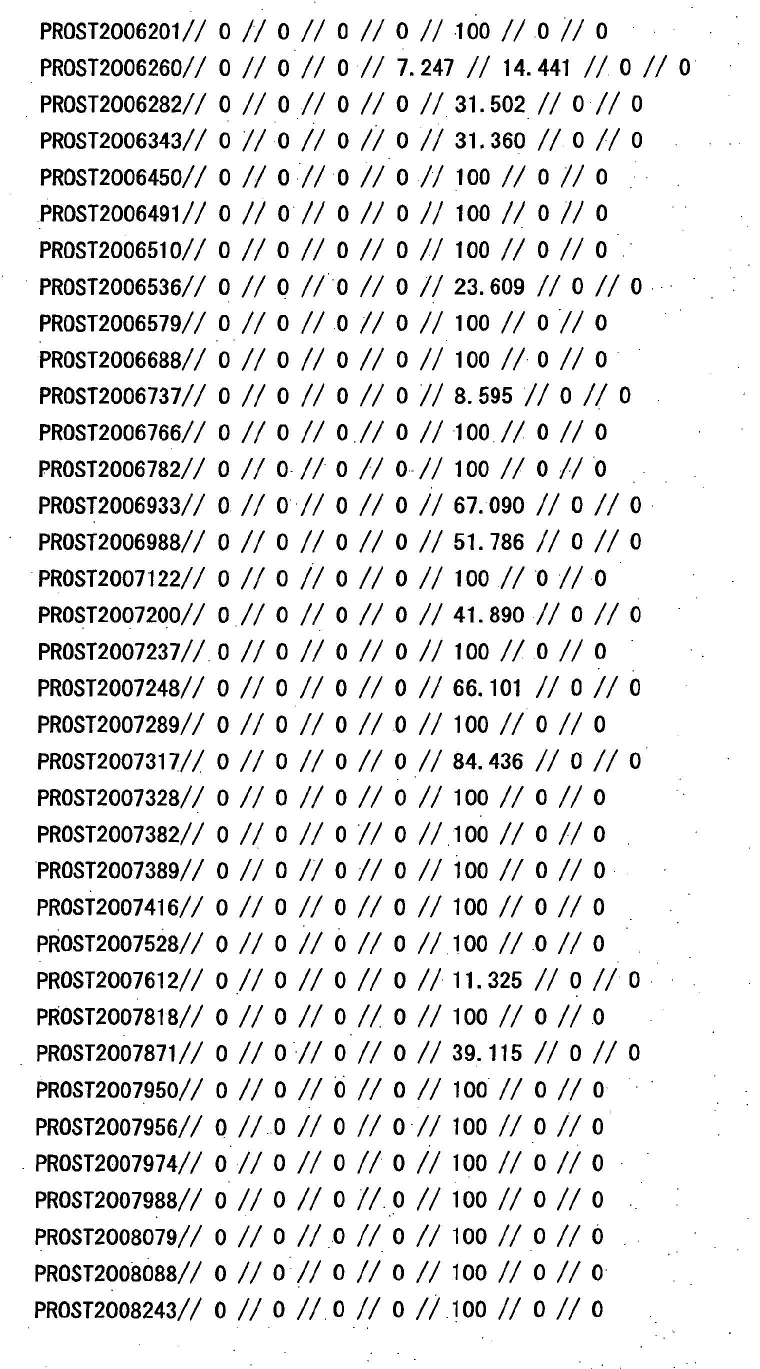

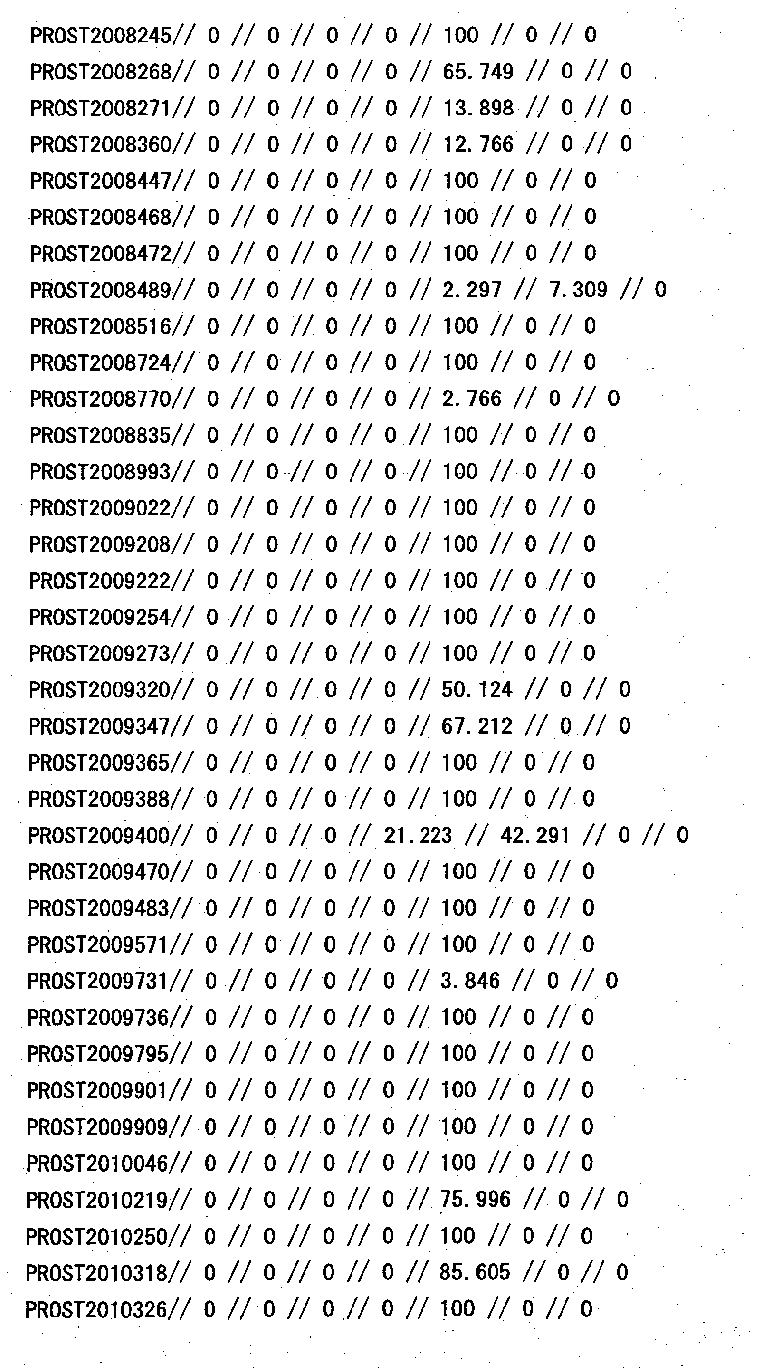

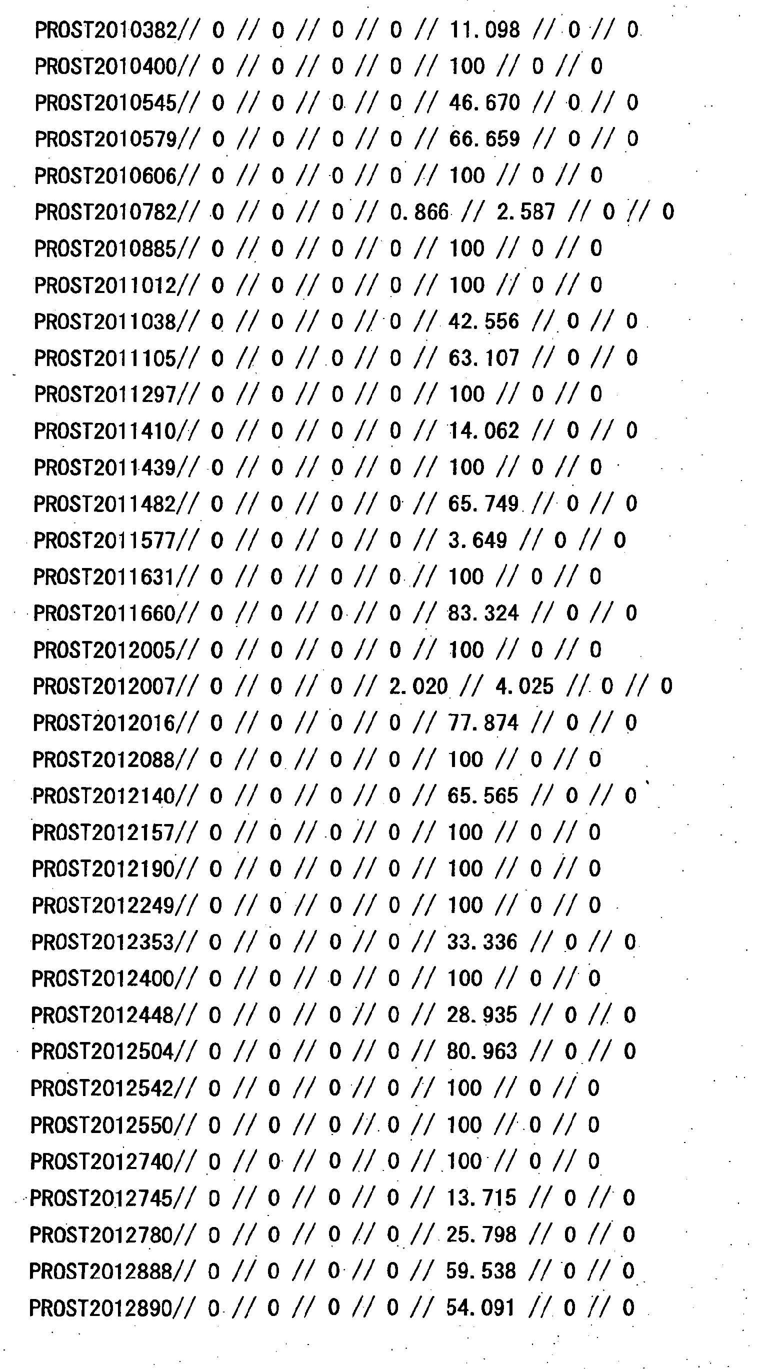

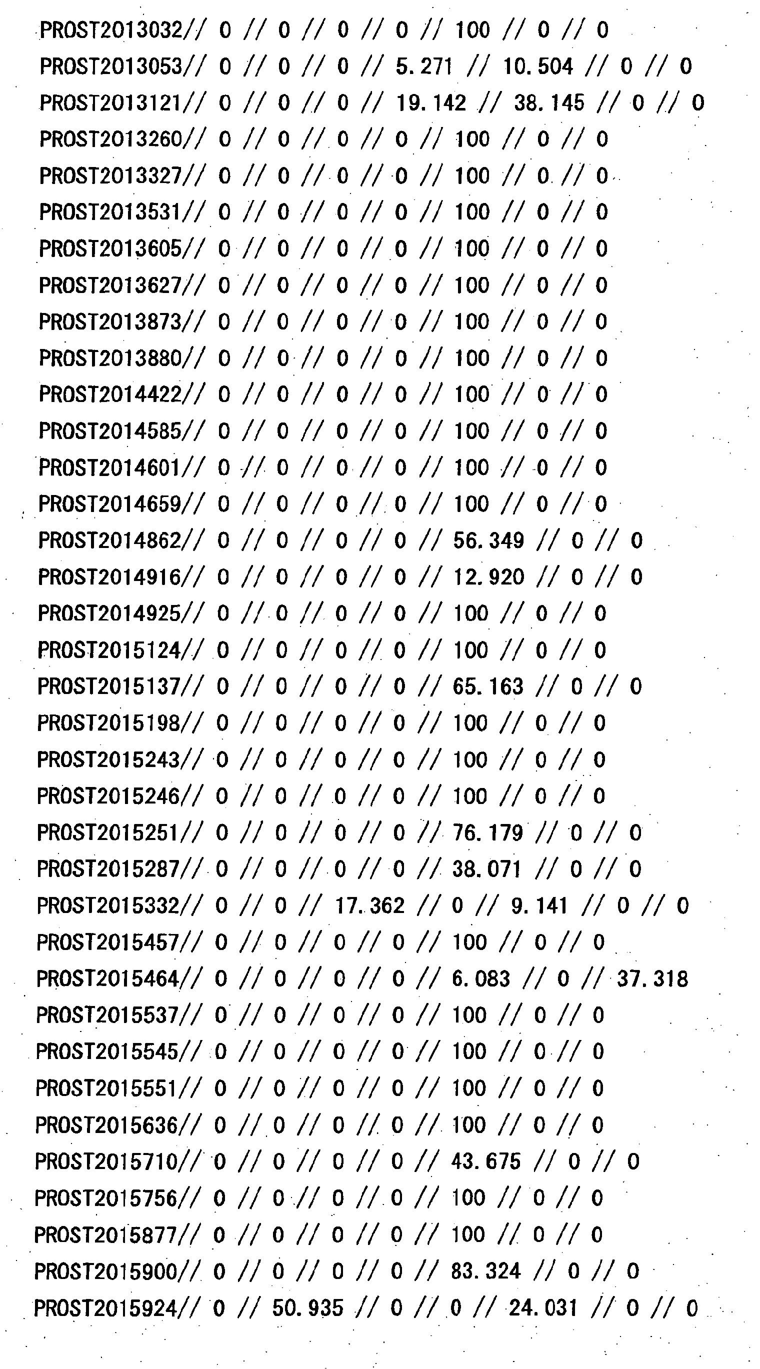

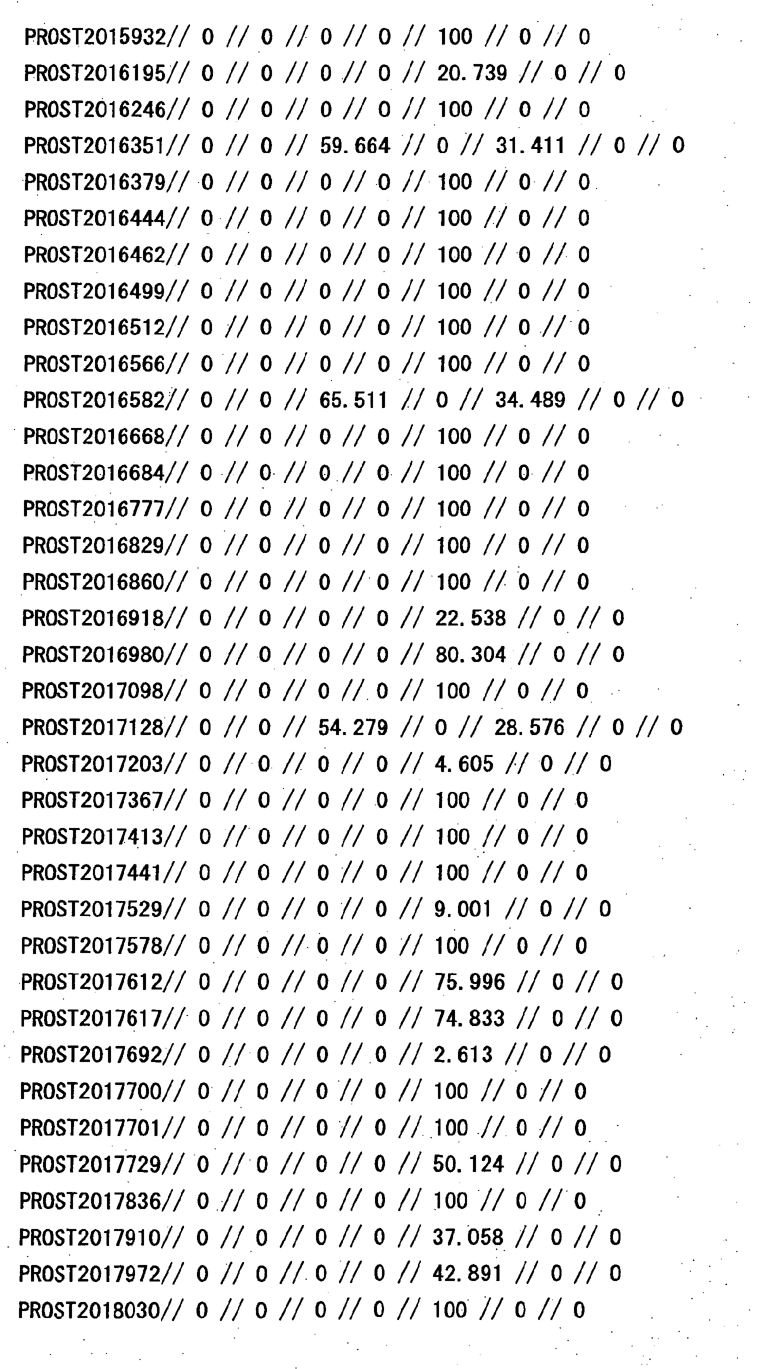

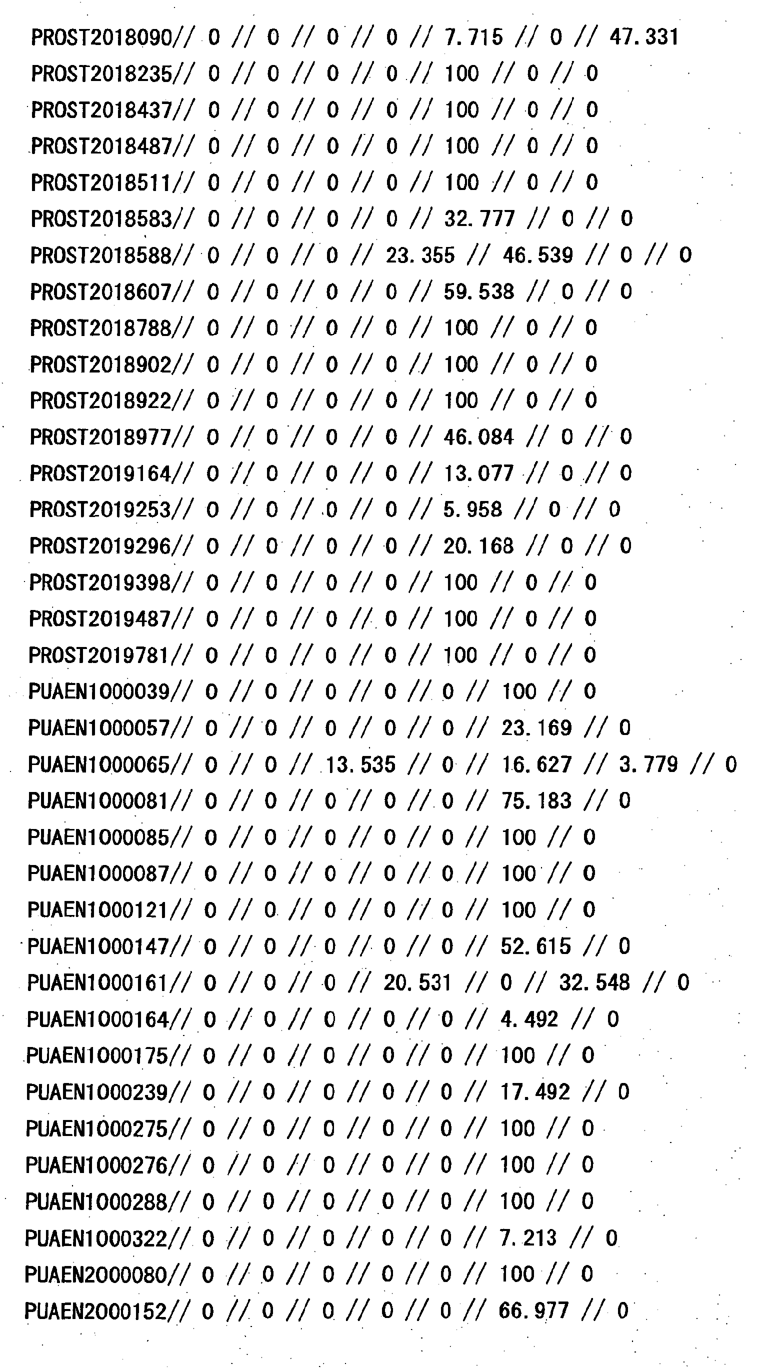

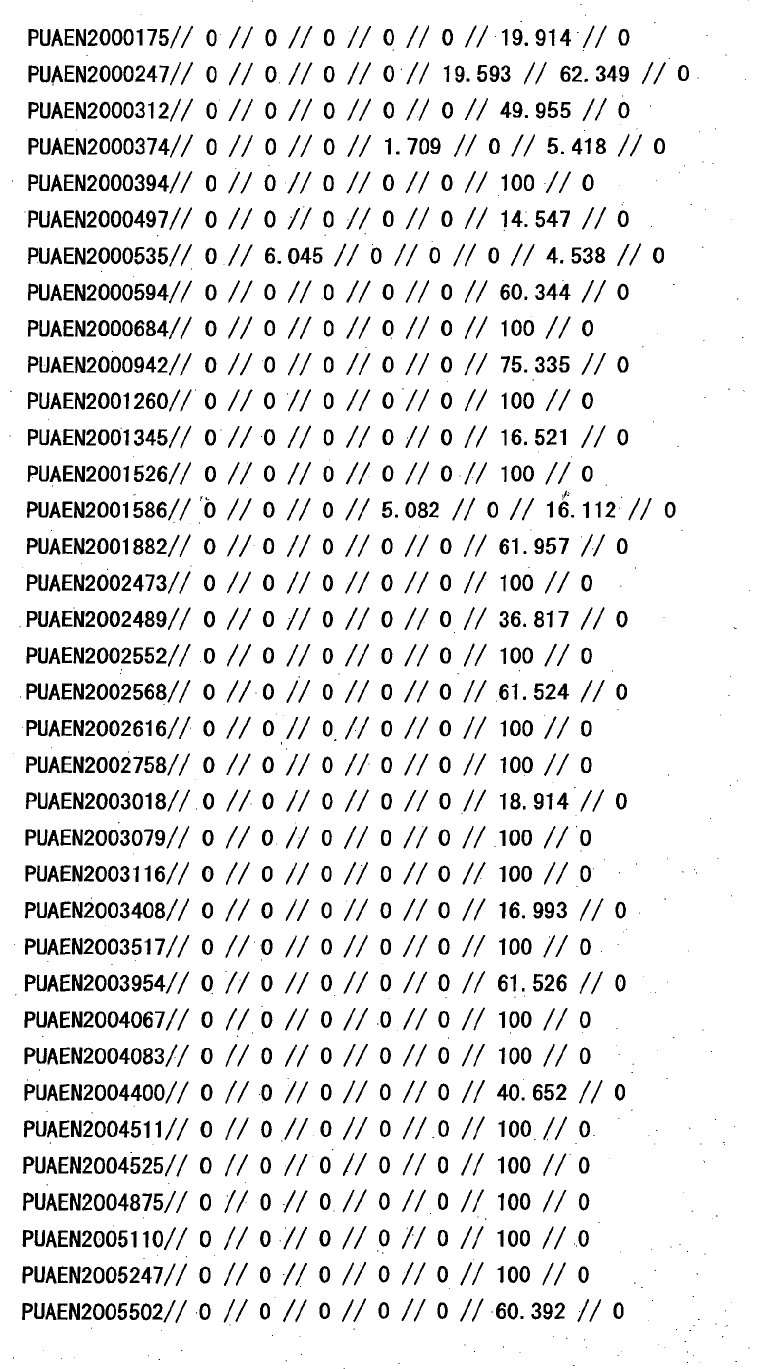

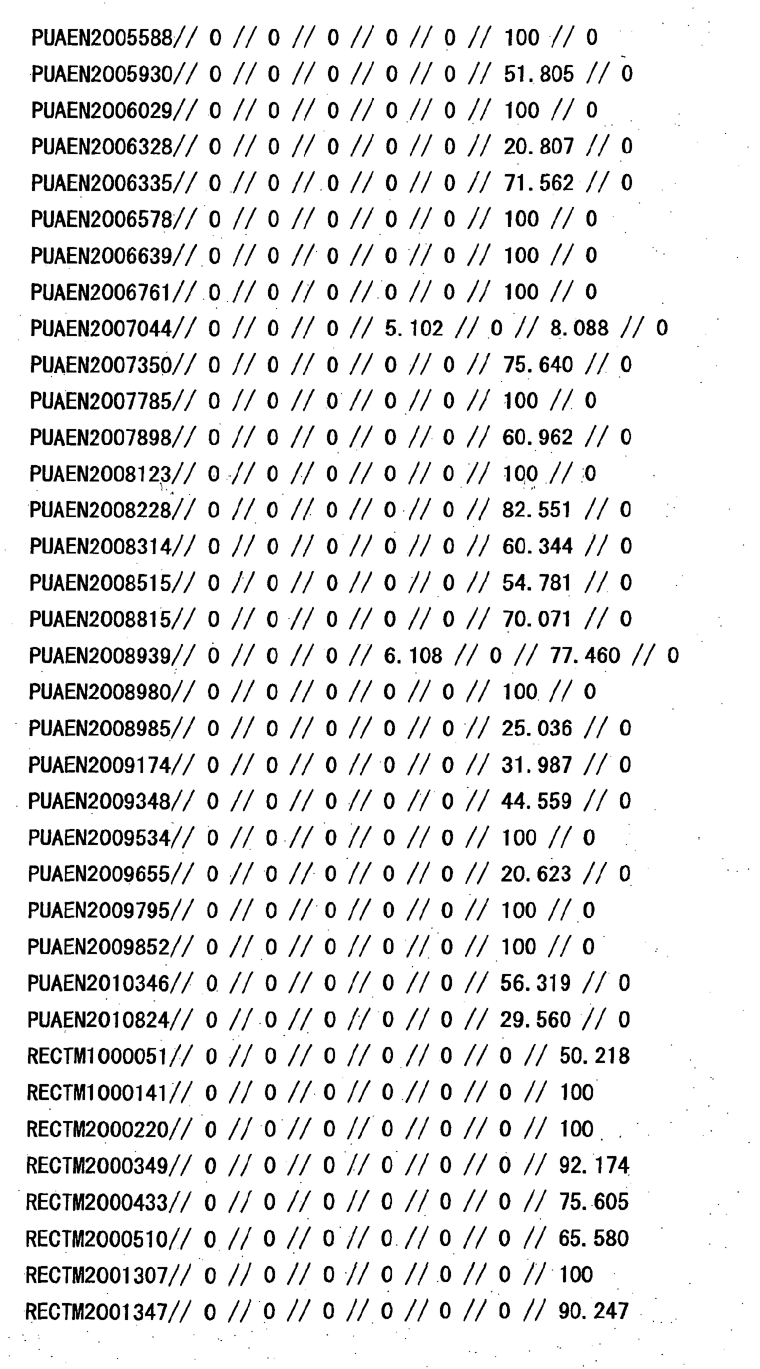

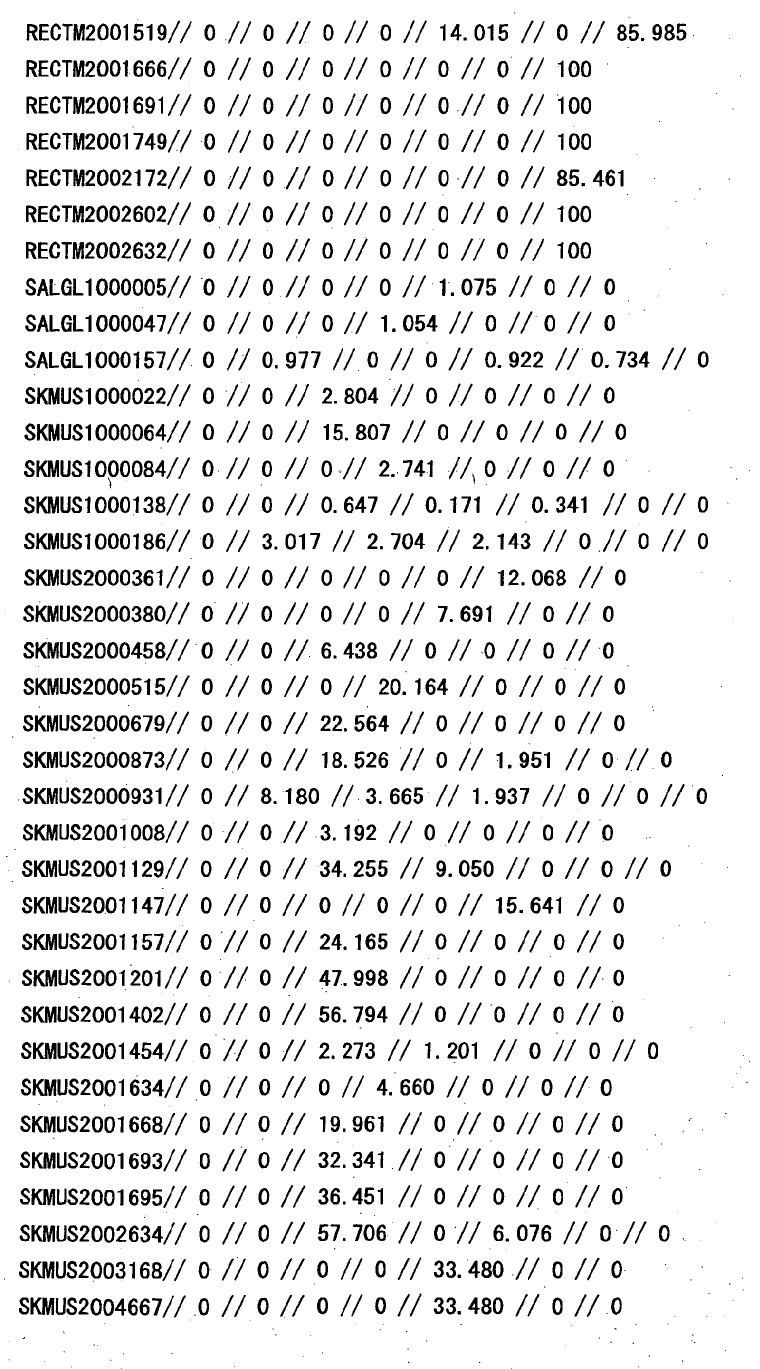

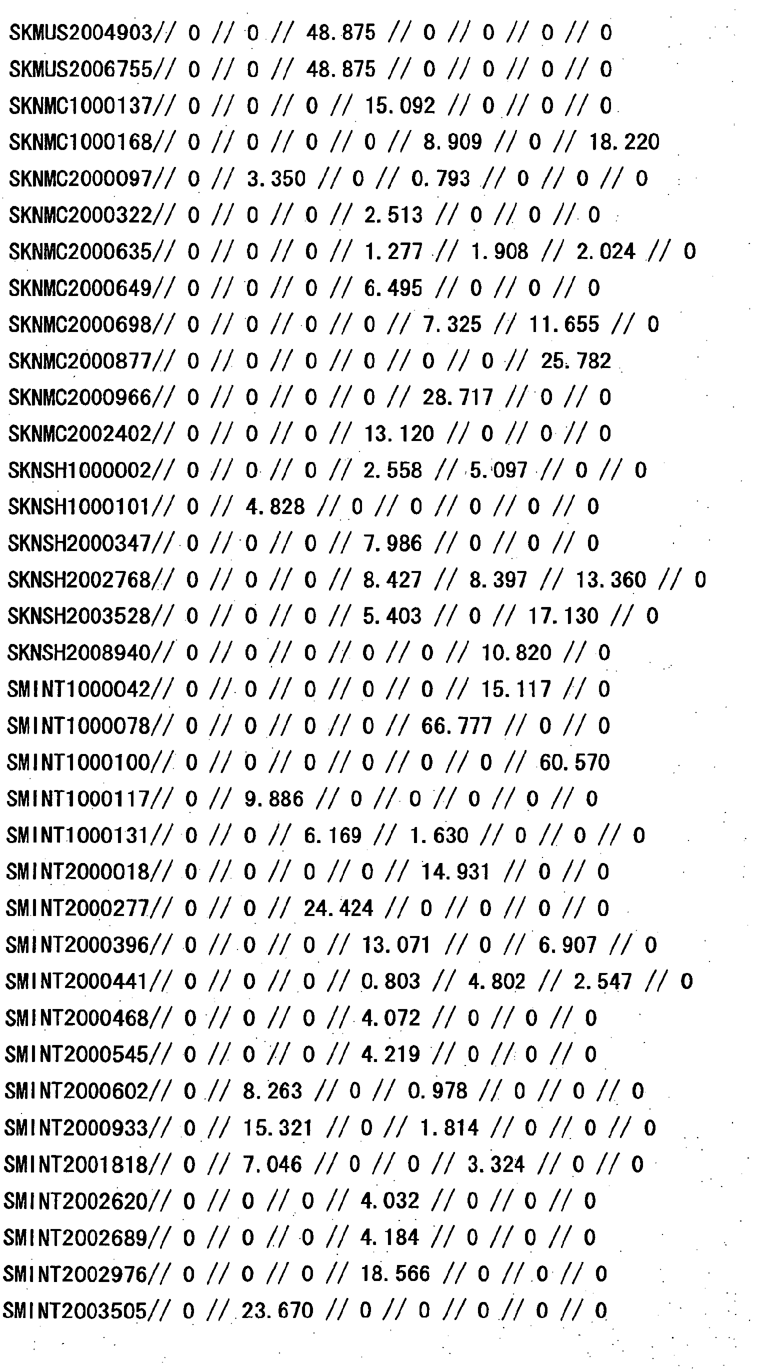

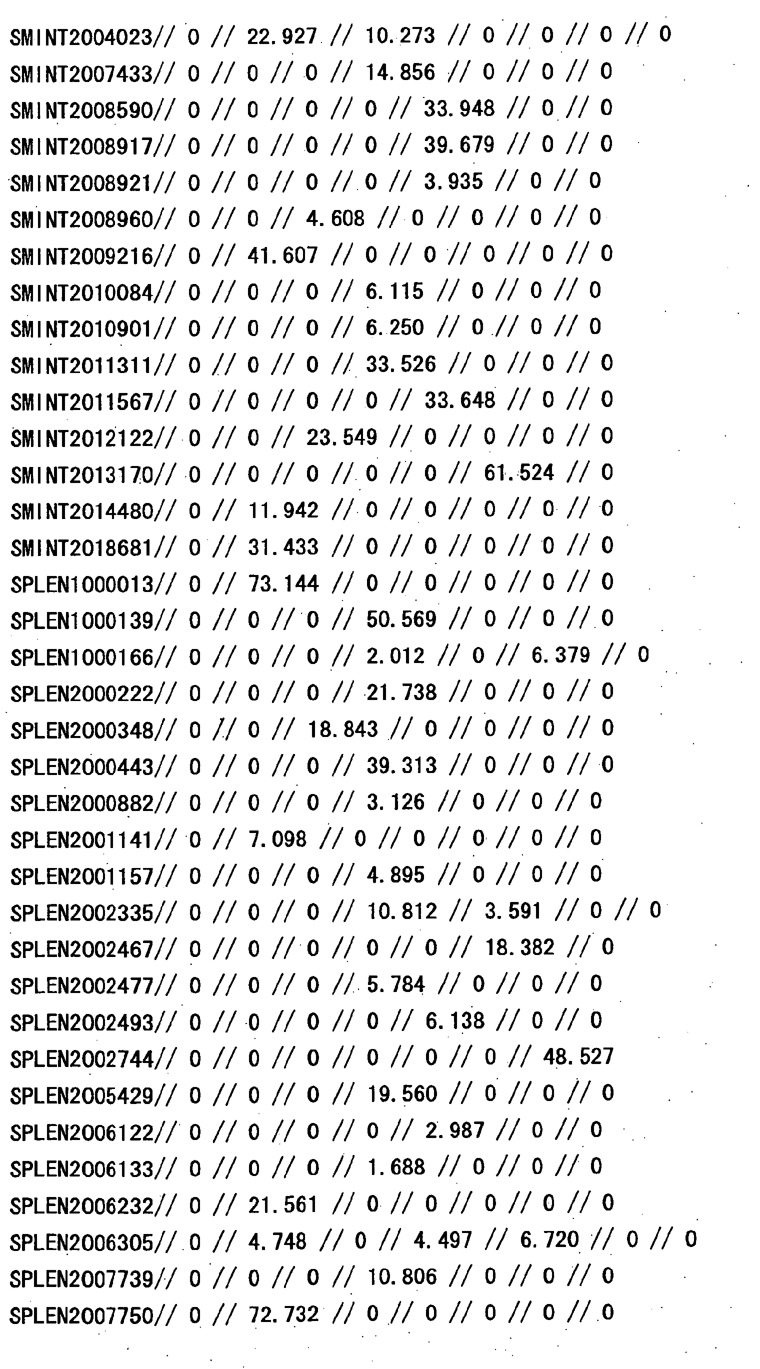

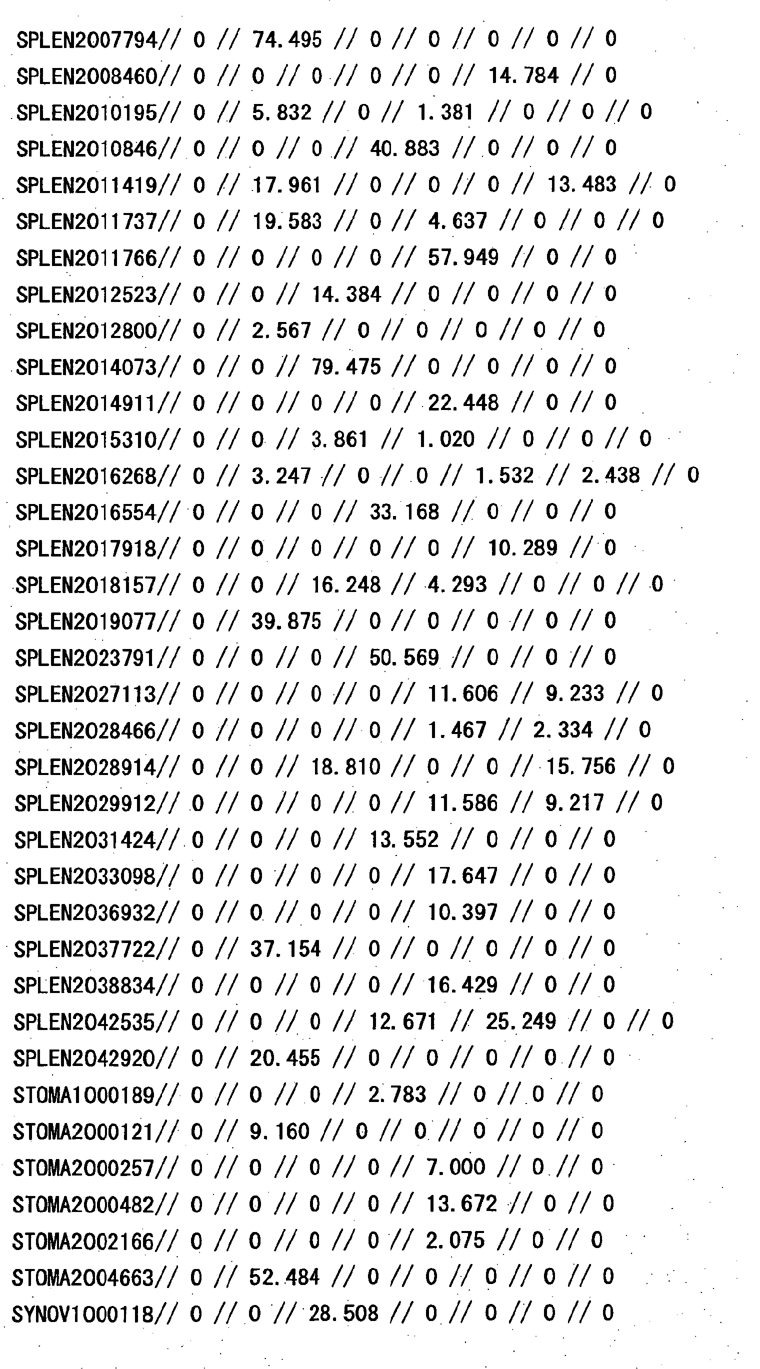

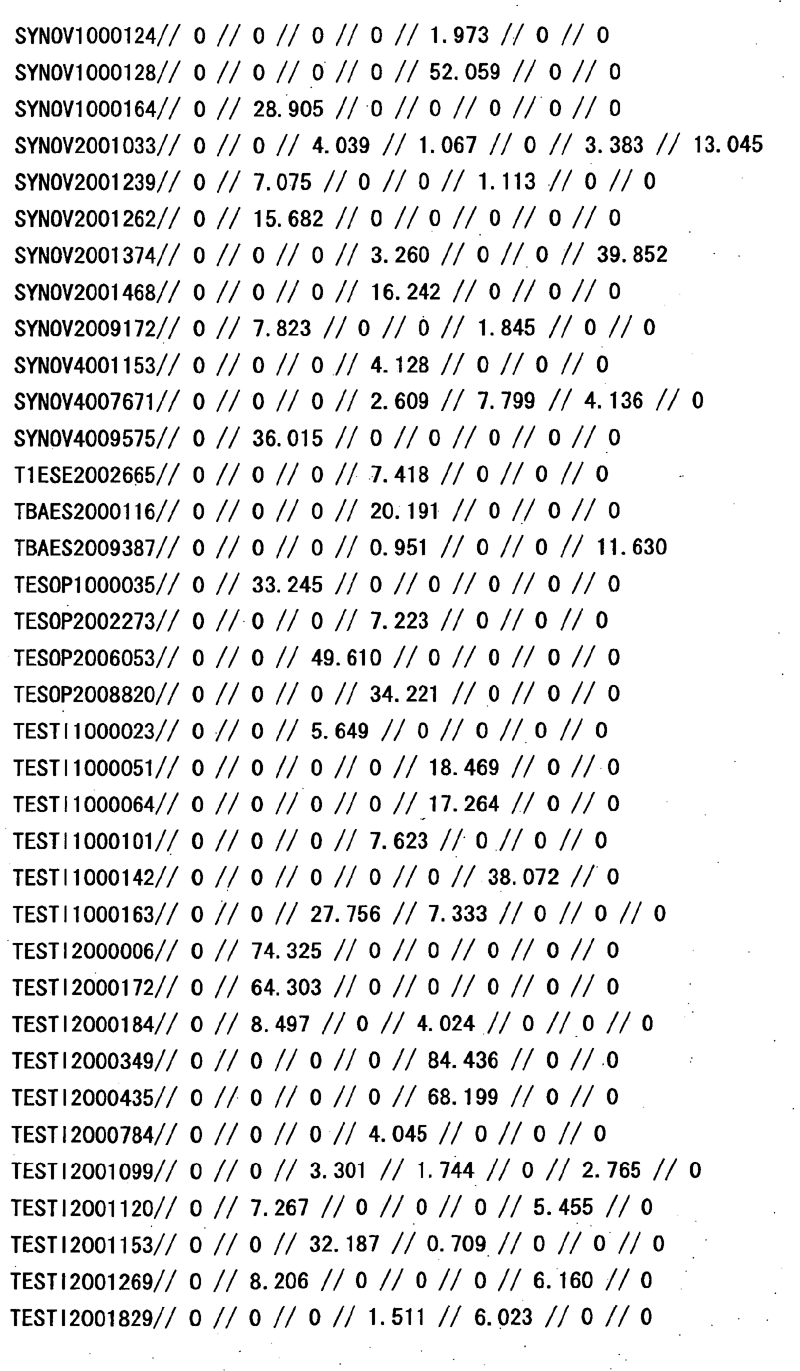

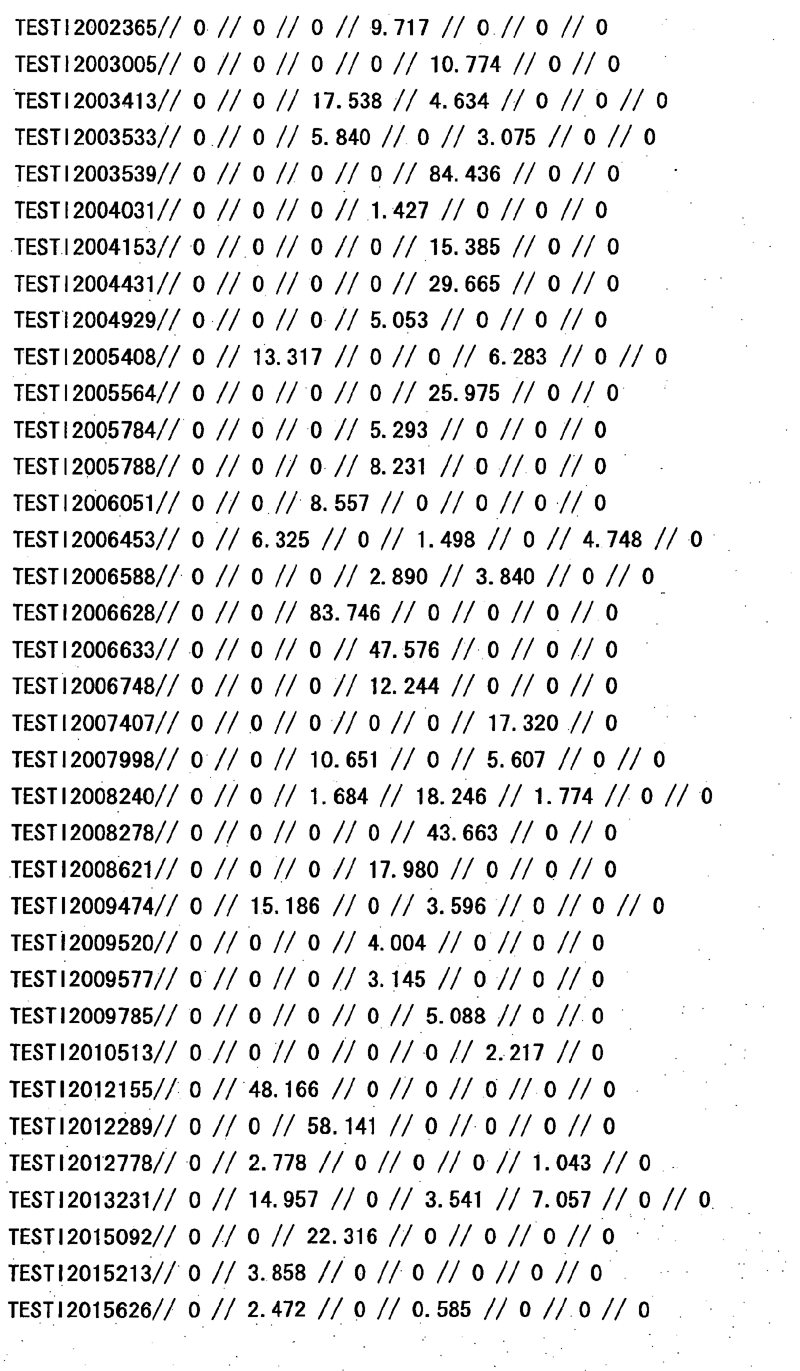

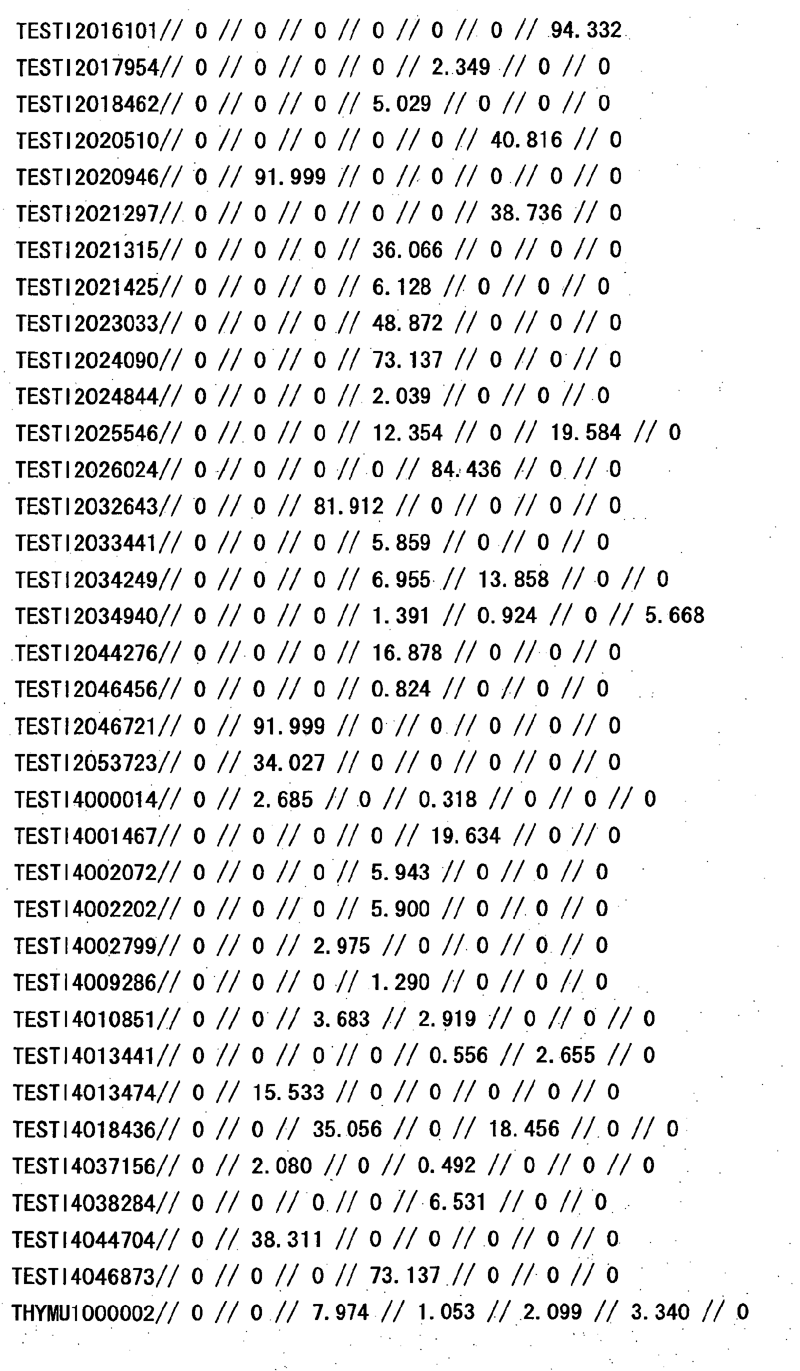

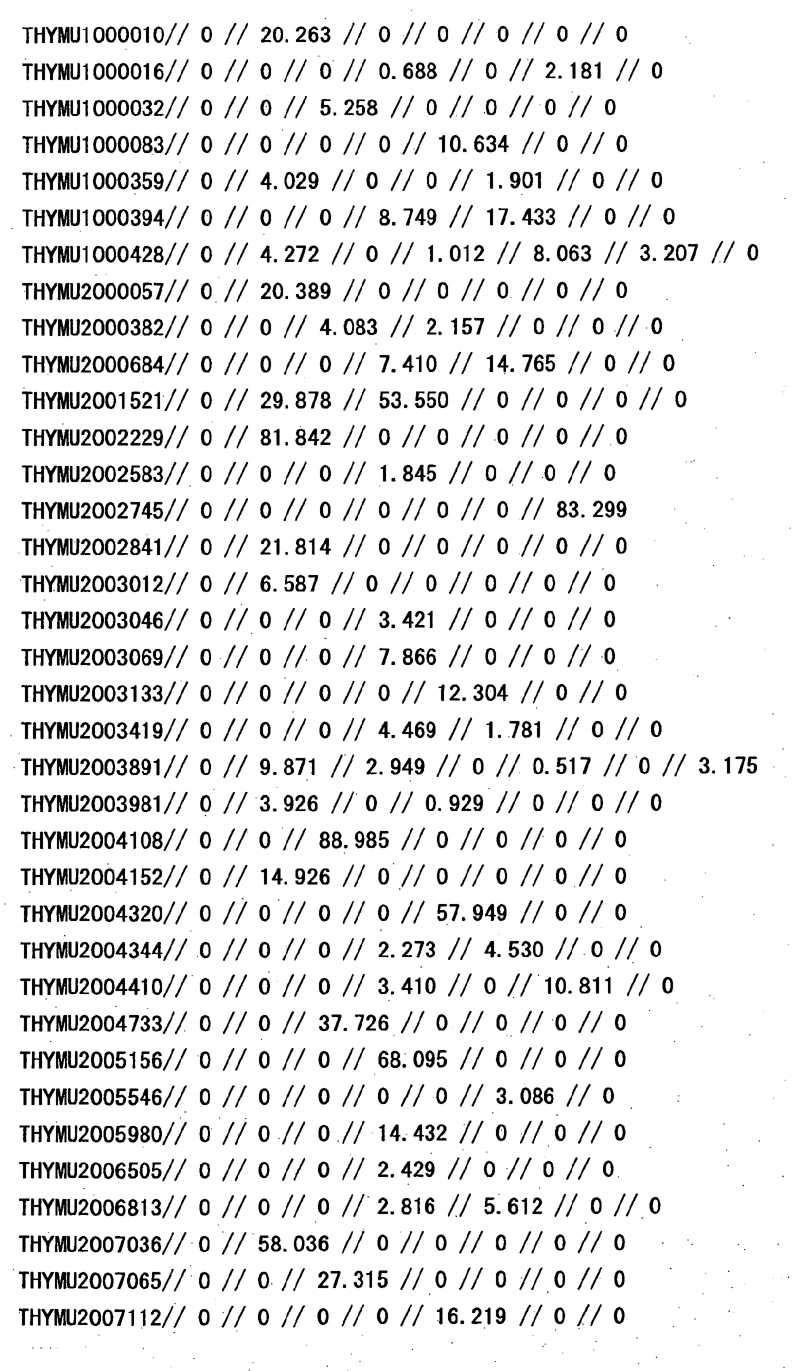

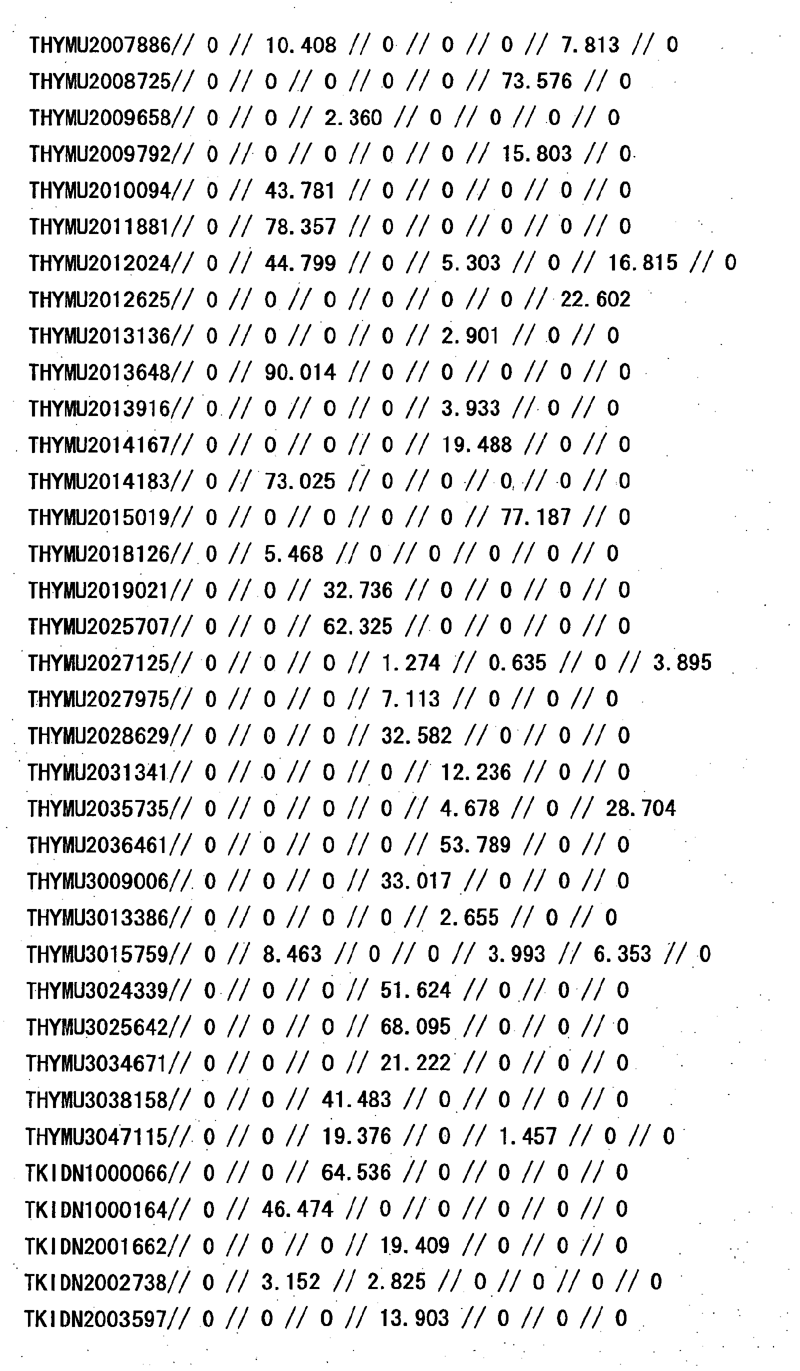

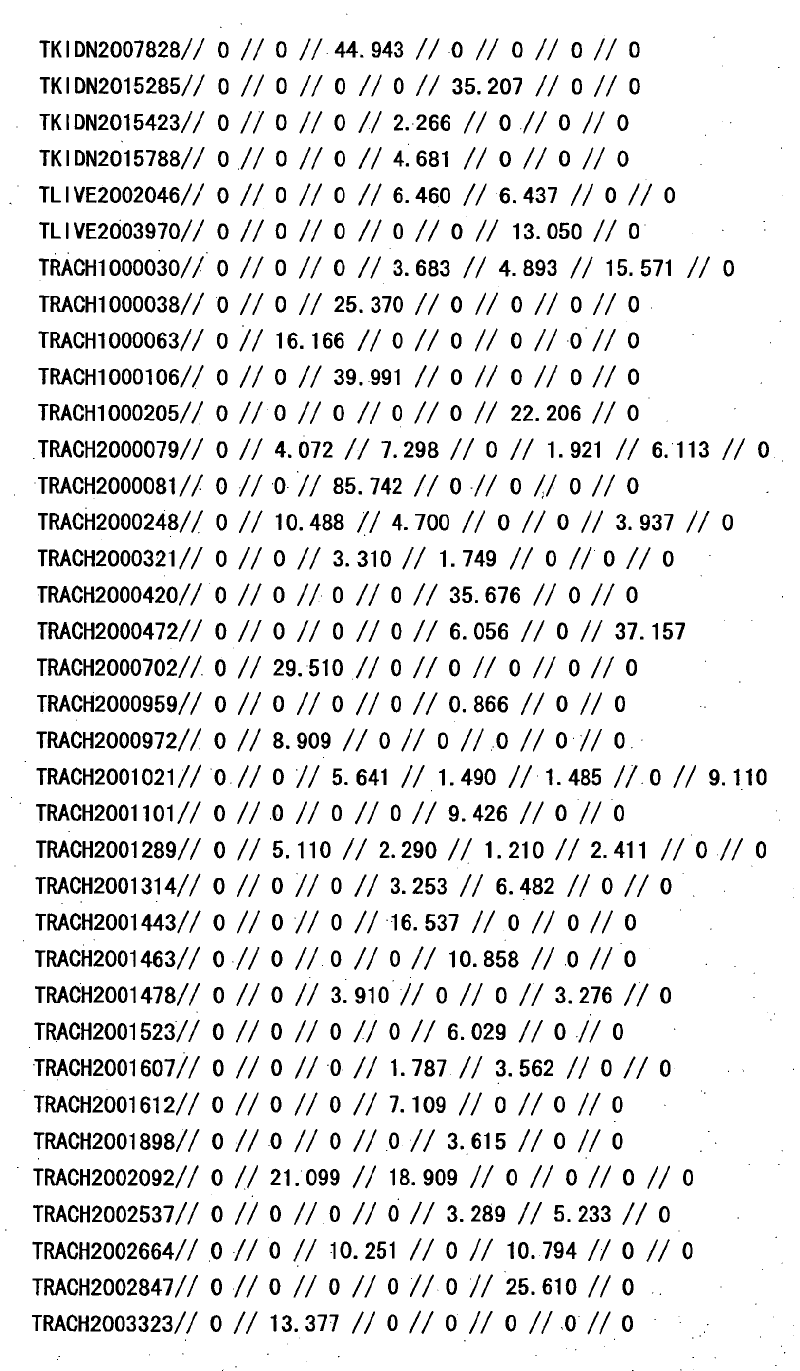

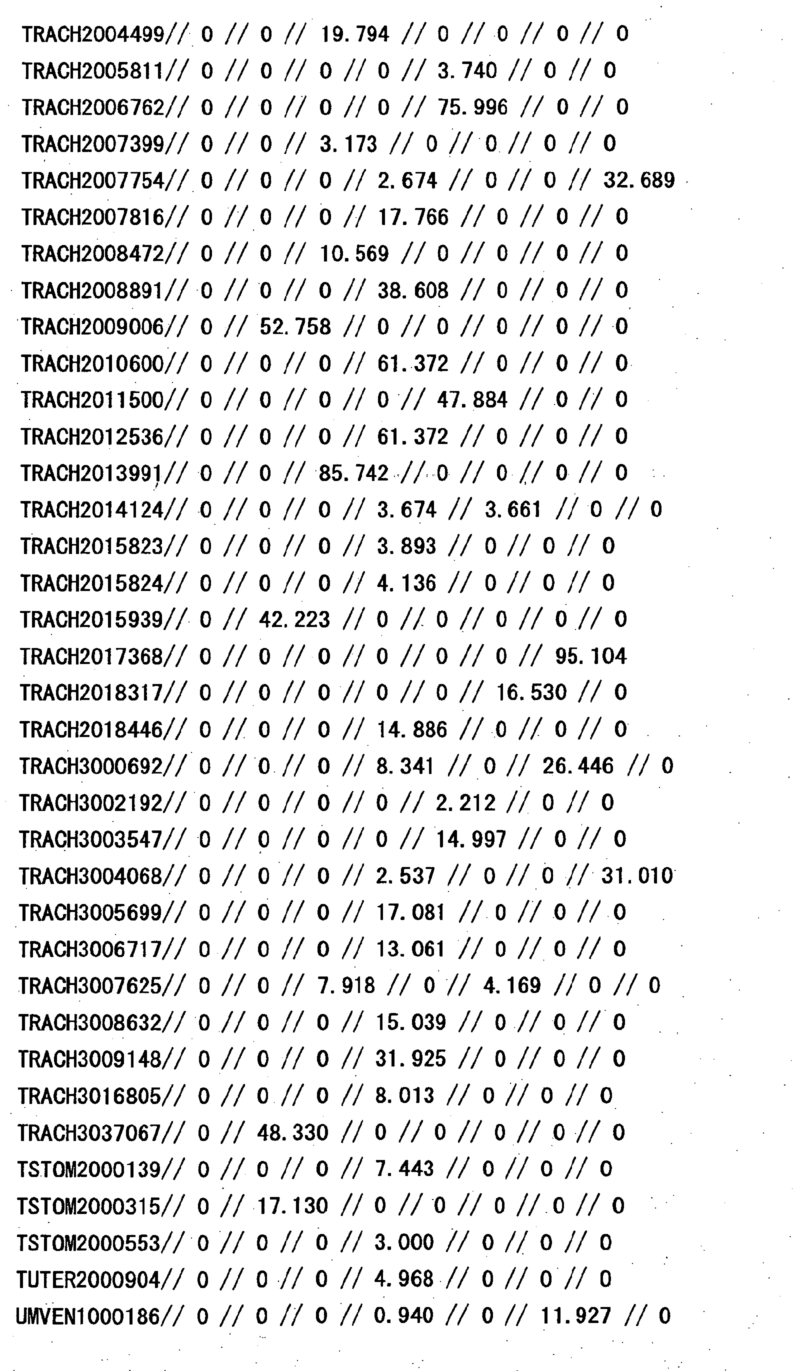

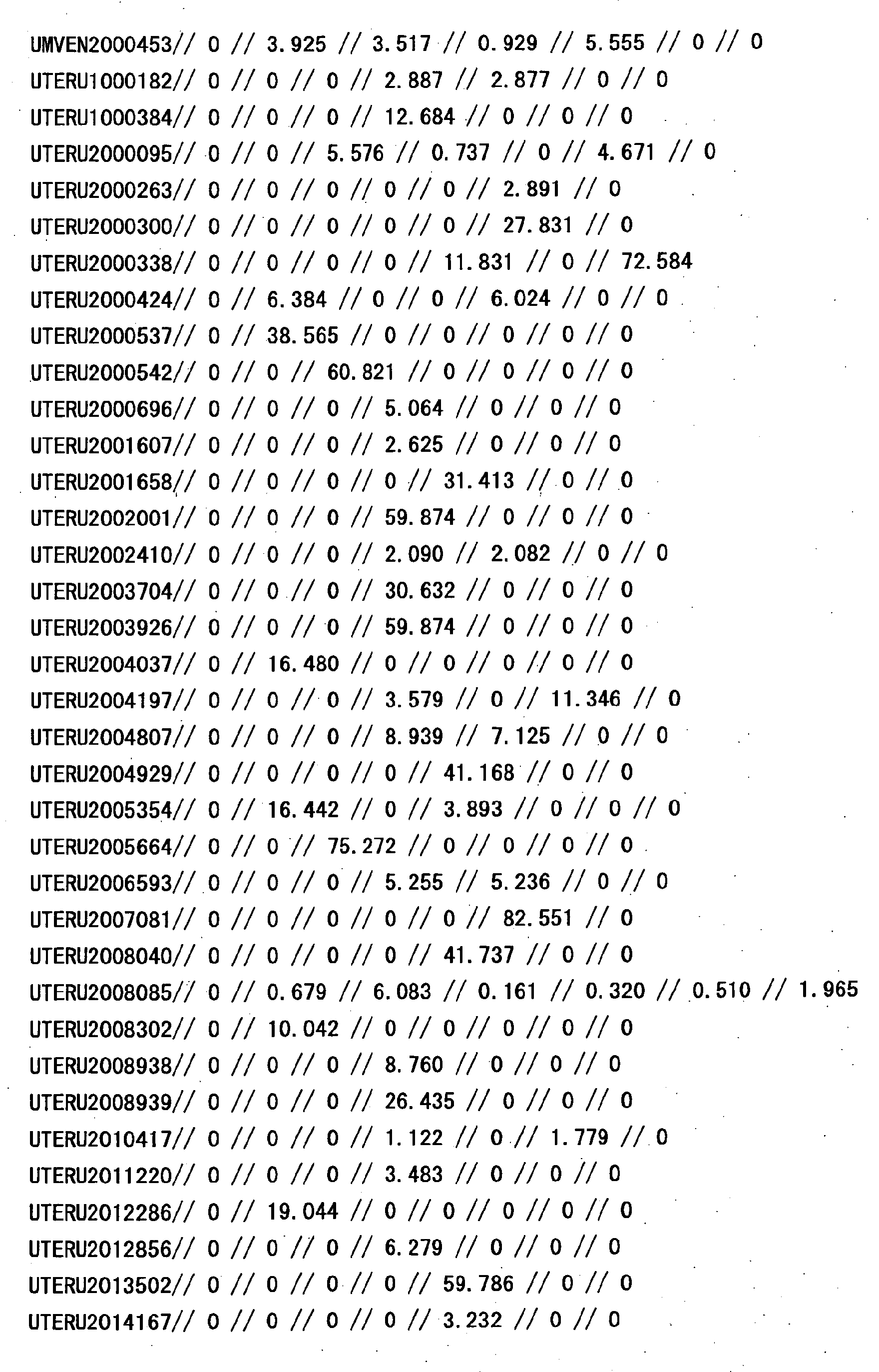

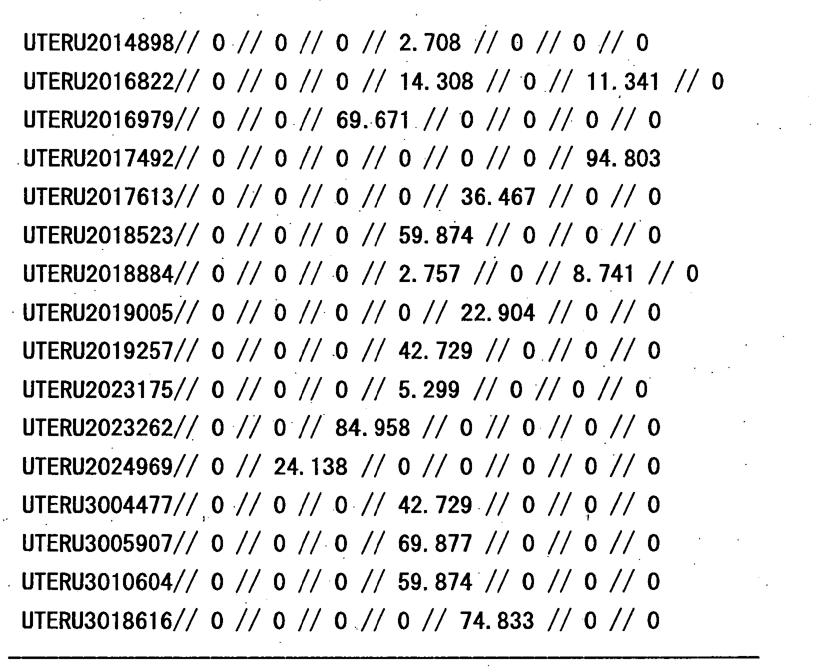

Genes whose expression levels were negligibly low in each of the above-described libraries are shown with their clone names below.
3NB691000018, 3NB691000113, 3NB691000191, 3NB692000012, 3NB692000029, 3NB692000102, 3NB692000154, 3NB692000276, 3NB692000305, 3NB692000429, 3NB692000484, 3NB692000529, 3NB692000973, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001548, 3NB692001637, 3NB692001719, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069,
ADIPS2000088, ADIPS2000245, ADRGL1000002, ADRGL1000018, ADRGL1000033, ADRGL1000038, ADRGL1000065, ADRGL1000067, ADRGL1000111, ADRGL1000147, ADRGL1000160, ADRGL1000165, ADRGL1000182, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002477, ADRGL2002679,
ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023,
ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO2000011, ASTRO2000095, ASTRO2000191, ASTRO2000278, ASTRO2000372, ASTRO2000381, ASTRO2000403, ASTRO2000417, ASTRO2000482, ASTRO2000533, ASTRO2000653, ASTRO2000662, ASTRO2000725, ASTRO2000801, ASTRO2001001, ASTRO2001008, ASTRO2001076, ASTRO2001088, ASTRO2001107, ASTRO2001122, ASTRO2001227, ASTRO2001249, ASTRO2001480, ASTRO2002024, ASTRO2002035,
ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003024, ASTRO2003212, ASTRO2003316, ASTRO2003461, ASTRO2003574, ASTRO2003581, ASTRO2003632, ASTRO2003740, ASTRO2003840, ASTRO2003925, ASTRO2003960, ASTRO2004051, ASTRO2004177, ASTRO2004217, ASTRO2004297, ASTRO2004584, ASTRO2004751, ASTRO2004877, ASTRO2004974, ASTRO2005008, ASTRO2005081, ASTRO2005096, ASTRO2005273, ASTRO2005343, ASTRO2005360, ASTRO2005413, ASTRO2005423, ASTRO2005553, ASTRO2005557, ASTRO2005575, ASTRO2005593, ASTRO2005726, ASTRO2005896, ASTRO2005902, ASTRO2006356, ASTRO2006732, ASTRO2006747, ASTRO2006952, ASTRO2007047,
ASTRO2007221, ASTRO2007377, ASTRO2007515, ASTRO2007578, ASTRO2007643, ASTRO2007666, ASTRO2007956, ASTRO2007962, ASTRO2008040, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923,
ASTRO2014961, ASTRO2015162, ASTRO2015185, ASTRO2015328, ASTRO2015529, ASTRO2016044, ASTRO2016148, ASTRO2016681, ASTRO2016819, ASTRO2016847, ASTRO2016892, ASTRO2017348, ASTRO2017627, ASTRO2018169, ASTRO2018373, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000544, BGGI12000684, BGGI12000693, BGGI12001002, BGGI12001075, BGGI12001241, BGGI12001247, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031,
BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007666, BLADE2007722, BLADE2007735, BLADE2007744, BLADE2007799, BLADE2007923, BLADE2007935, BLADE2007958,
BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000118, BNGH41000137, BNGH41000169, BNGH41000177, BNGH41000198, BNGH41000216, BNGH41000235, BNGH42000130, BNGH42000132, BNGH42000405, BNGH42000532, BNGH42000675, BNGH42000791, BNGH42000815, BNGH42000994, BNGH42001247, BNGH42001406, BNGH42001576, BNGH42001724, BNGH42002168, BNGH42002244, BNGH42002387, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42006226, BNGH42006234, BNGH42007037, BNGH42007366, BNGH42007594, BNGH42007798,
BNGH42008199, BNGH42008510, BNGH42008649, BNGH42008743, BNGH42008832, BNGH42008943, BNGH42009021, BRACE1000020, BRACE1000047, BRACE1000051, BRACE1000065, BRACE1000073, BRACE1000084, BRACE1000087, BRACE1000092, BRACE1000093, BRACE1000101, BRACE1000114, BRACE1000124, BRACE1000166, BRACE1000169, BRACE1000187, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000307, BRACE2000331, BRACE2000332, BRACE2000347, BRACE2000351, BRACE2000368, BRACE2000392, BRACE2000441,
BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001151, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374,
BRACE2001375, BRACE2001423, BRACE2001424, BRACE2001445, BRACE2001453, BRACE2001455, BRACE2001492, BRACE2001496, BRACE2001534, BRACE2001543, BRACE2001564, BRACE2001588, BRACE2001607, BRACE2001626, BRACE2001677, BRACE2001692, BRACE2001705, BRACE2001709, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001898, BRACE2001923, BRACE2001942, BRACE2001944, BRACE2001954, BRACE2001971, BRACE2001972, BRACE2001973, BRACE2002026, BRACE2002034, BRACE2002050, BRACE2002081, BRACE2002139, BRACE2002142, BRACE2002151, BRACE2002157, BRACE2002176, BRACE2002202, BRACE2002206,
BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002613, BRACE2002614, BRACE2002617, BRACE2002623, BRACE2002635, BRACE2002649, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002884, BRACE2002896, BRACE2002948, BRACE2003037, BRACE2003078, BRACE2003097, BRACE2003110,
BRACE2003168, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003639, BRACE2003700, BRACE2003713, BRACE2003766, BRACE2003785, BRACE2003788, BRACE2003800, BRACE2003802, BRACE2003825, BRACE2003845, BRACE2003846, BRACE2003847, BRACE2003848, BRACE2003873, BRACE2003885, BRACE2003887, BRACE2003892, BRACE2003904, BRACE2003905, BRACE2003928, BRACE2003954, BRACE2003982, BRACE2003987, BRACE2005034, BRACE2005047, BRACE2005063, BRACE2005083, BRACE2005090, BRACE2005099, BRACE2005118,
BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898,
BRACE2005904, BRACE2005906, BRACE2005907, BRACE2005911, BRACE2005949, BRACE2005981, BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006167, BRACE2006174, BRACE2006240, BRACE2006245, BRACE2006249, BRACE2006274, BRACE2006281, BRACE2006298, BRACE2006344, BRACE2006354, BRACE2006363, BRACE2006378, BRACE2006380, BRACE2006393, BRACE2006413, BRACE2006417, BRACE2006453, BRACE2006459, BRACE2006488, BRACE2006534, BRACE2006540, BRACE2006564, BRACE2006587, BRACE2006598, BRACE2006636, BRACE2006685, BRACE2006703, BRACE2006743, BRACE2006783, BRACE2006821, BRACE2006833, BRACE2006859, BRACE2006871,
BRACE2006909, BRACE2006911, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007109, BRACE2007138, BRACE2007174, BRACE2007197, BRACE2007201, BRACE2007203, BRACE2007232, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563, BRACE2007577, BRACE2007621, BRACE2007640, BRACE2007641, BRACE2007646, BRACE2007663, BRACE2007682, BRACE2007685, BRACE2007708, BRACE2007727, BRACE2007749, BRACE2007760, BRACE2007761,
BRACE2007764, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008553, BRACE2008594,
BRACE2008604, BRACE2008615, BRACE2008622, BRACE2008629, BRACE2008655, BRACE2008656, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008948, BRACE2008960, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009307, BRACE2009311, BRACE2009318, BRACE2009323, BRACE2009366, BRACE2009421, BRACE2009428, BRACE2009434, BRACE2009437,
BRACE2009483, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646,
BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132,
BRACE2015262, BRACE2015270, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847,
BRACE2019004, BRACE2019044, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158,
BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294,
BRACE2026404, BRACE2026675, BRACE2026725, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE 2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148,
BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170,
BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300,
BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002298, BRACE3002344, BRACE3002390, BRACE3002420, BRACE3002508, BRACE3002541, BRACE3002756, BRACE3003004, BRACE3003053, BRACE3003192, BRACE3003413, BRACE3003595,
BRACE3003698, BRACE3003866, BRACE3003920, BRACE3004046, BRACE3004058, BRACE3004113, BRACE3004150, BRACE3004205, BRACE3004358, BRACE3004435, BRACE3004477, BRACE3004767, BRACE3004772, BRACE3004783, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005938, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642,
BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806,
BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194,
BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931,
BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760,
BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189,
BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880,
BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000388, BRAMY2000411, BRAMY2000508, BRAMY2000562, BRAMY2000585, BRAMY2000653, BRAMY2000814, BRAMY2000946, BRAMY2000981, BRAMY2000987, BRAMY2001068, BRAMY2001114, BRAMY2001203,
BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492,
BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333,
BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216,
BRAMY2011253, BRAMY2011446, BRAMY2011480, BRAMY2011639, BRAMY2011679, BRAMY2011811,
BRAMY2011841, BRAMY2011846, BRAMY2011849, BRAMY2011872, BRAMY2011961, BRAMY2012017, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2015211, BRAMY2015251, BRAMY2015311, BRAMY2015420, BRAMY2015503, BRAMY2015516, BRAMY2015550,
BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016020, BRAMY2016070, BRAMY2016251, BRAMY2016325, BRAMY2016327, BRAMY2016386, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710,
BRAMY2019963, BRAMY2019985, BRAMY2019989, BRAMY2020050, BRAMY2020058, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022796, BRAMY2022929, BRAMY2022980, BRAMY2022984, BRAMY2023060,
BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023729, BRAMY2023786, BRAMY2023852, BRAMY2023863, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395,
BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028282, BRAMY2028472, BRAMY2028516, BRAMY2028565, BRAMY2028593, BRAMY2028682, BRAMY2028783, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768,
BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387,
BRAMY2036396, BRAMY2036567, BRAMY2036615, BRAMY2036699, BRAMY2036913, BRAMY2036918, BRAMY2036968, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040095, BRAMY2040159, BRAMY2040214, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244,
BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401,
BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937,
BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000037, BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082,
BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000471, BRAWH2000476, BRAWH2000482, BRAWH2000494, BRAWH2000500, BRAWH2000522, BRAWH2000554, BRAWH2000595, BRAWH2000636, BRAWH2000643, BRAWH2000651, BRAWH2000682, BRAWH2000686, BRAWH2000752, BRAWH2000859, BRAWH2000866, BRAWH2000892, BRAWH2000901, BRAWH2000926, BRAWH2000944, BRAWH2000984, BRAWH2001019, BRAWH2001037, BRAWH2001052, BRAWH2001103, BRAWH2001129, BRAWH2001137, BRAWH2001159, BRAWH2001186, BRAWH2001203, BRAWH2001238, BRAWH2001239, BRAWH2001255,
BRAWH2001321, BRAWH2001355, BRAWH2001378, BRAWH2001395, BRAWH2001406, BRAWH2001412, BRAWH2001418, BRAWH2001424, BRAWH2001436, BRAWH2001438, BRAWH2001459, BRAWH2001461, BRAWH2001492, BRAWH2001503, BRAWH2001519, BRAWH2001530, BRAWH2001535, BRAWH2001589, BRAWH2001662, BRAWH2001666, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002725, BRAWH2002761, BRAWH2002811, BRAWH2002861, BRAWH2002911, BRAWH2002963,
BRAWH2002967, BRAWH2002976, BRAWH2003000, BRAWH2003135, BRAWH2003240, BRAWH2003307, BRAWH2003355, BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960,
BRAWH2006989, BRAWH2006996, BRAWH2007069, BRAWH2007308, BRAWH2007406, BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008255, BRAWH2008292, BRAWH2008297, BRAWH2008366, BRAWH2008380, BRAWH2008540, BRAWH2008577, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084,
BRAWH2010318, BRAWH2010329, BRAWH2010354, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013866, BRAWH2013871, BRAWH2013914, BRAWH2013941,
BRAWH2013955, BRAWH2014053, BRAWH2014234, BRAWH2014379, BRAWH2014414, BRAWH2014473, BRAWH2014511, BRAWH2014645, BRAWH2014662, BRAWH2014717, BRAWH2014729, BRAWH2014876, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433,
BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002600, BRAWH3002819, BRAWH3003019, BRAWH3003136,
BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461,
BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928,
BRAWH3021012, BRAWH3021545, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH30229.00, BRAWH3023156, BRAWH3023168, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772,
BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149, BRCAN2000209, BRCAN2000239,
BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401,
BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015402, BRCAN2015464, BRCAN2015719, BRCAN2015757, BRCAN2015886, BRCAN2016025,
BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026340, BRCAN2026711, BRCAN2027310,
BRCAN2027334, BRCAN2027355, BRCAN2027513, BRCAN2027593, BRCAN2027970, BRCAN2028021, BRCAN2028040, BRCAN2028319, BRCAN2028338, BRCAN2028378, BRCAN2028460, BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003513, BRCOC2003732, BRCOC2003740, BRCOC2004036, BRCOC2004354, BRCOC2004455, BRCOC2004950, BRCOC2005542,
BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944,
BRCOC2016525, BRCOC2016661, BRCOC2017061, BRCOC2017652, BRCOC2017856, BRCOC2018775, BRCOC2018877, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172,
BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414,
BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387,
BRHIP2014954, BRHIP2015153, BRHIP2015224, BRHIP2015356, BRHIP2015360, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859,
BRHIP2020930, BRHIP2021102, BRHIP2021289, BRHIP2021495, BRHIP2021615, BRHIP2021744, BRHIP2021858, BRHIP2021929, BRHIP2022006, BRHIP2022054, BRHIP2022221, BRHIP2022228, BRHIP2022298, BRHIP2022467, BRHIP2022708, BRHIP2023071, BRHIP2023272, BRHIP2023292, BRHIP2023309, BRHIP2023369, BRHIP2023735, BRHIP2023773, BRHLP2023860, BRHIP2023869, BRHIP2024046, BRHIP2024165, BRHIP2024201, BRHIP2024347, BRHIP2024398, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017,
BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395,
BRHIP3003484, BRHIP3003644, BRHIP3003688, BRHIP3003795, BRHIP3003984, BRHIP3004193, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405,
BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558,
BRHIP3017637, BRHIP3017855, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244,
BRSSN2000293, BRSSN2000295, BRSSN2000312, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2002857, BRSSN2002859, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464,
BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014636, BRSSN2014685, BRSSN2015199, BRSSN2015238, BRSSN2015369, BRSSN2015493, BRSSN2015497, BRSSN2015554, BRSSN2015707,
BRSSN2015773, BRSSN2015907, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367,
BRSTN2008418, BRSTN2008457, BRSTN2008475, BRSTN2008930, BRSTN2009247, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013171, BRSTN2013354, BRSTN2013931, BRSTN2014633, BRSTN2014751, BRSTN2014770, BRSTN2015015, BRSTN2015978, BRSTN2016066, BRSTN2016335, BRSTN2016678, BRSTN2016892, BRSTN2016918, BRSTN2016992, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063,
BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002741, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005942, BRTHA2005956, BRTHA2006075,
BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012466,
BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304,
BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845,
BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003704, BRTHA3004307, BRTHA3004432, BRTHA3004475, BRTHA3004502,
BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685, BRTHA3007769, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010366, BRTHA3010469, BRTHA3010530, BRTHA3010540, BRTHA3010717, BRTHA3011149, BRTHA3011187, BRTHA3011194, BRTHA3011229, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547,
BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505,
BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CHONS1000058, CHONS2000172, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001721, COLON2001829, COLON2001866, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105,
COLON2009337, COLON2009499, CORDB1000140, CORDB2000061, CORDB2000127, CORDB2000541, CTONG1000010, CTONG1000020, CTONG1000022, CTONG1000040, CTONG1000056, CTONG1000062, CTONG1000086, CTONG1000087, CTONG1000088, CTONG1000093, CTONG1000097, CTONG1000113, CTONG1000137, CTONG1000165, CTONG1000180, CTONG1000181, CTONG1000241, CTONG1000283, CTONG1000288, CTONG1000302, CTONG1000341, CTONG1000476, CTONG1000488, CTONG1000508, CTONG1000540, CTONG2000031, CTONG2000034, CTONG2000201, CTONG2000218, CTONG2000279, CTONG2000303, CTONG2000330, CTONG2000395, CTONG2000465, CTONG2000480, CTONG2000508, CTONG2000766, CTONG2000771, CTONG2000819, CTONG2000827, CTONG2000846, CTONG2000855, CTONG2000977, CTONG2001139,
CTONG2001226, CTONG2001366, CTONG2001413, CTONG2001428, CTONG2001524, CTONG2001533, CTONG2001598, CTONG2001681, CTONG2001724, CTONG2001749, CTONG2001800, CTONG2001820, CTONG2001858, CTONG2001877, CTONG2001911, CTONG2001955, CTONG2002066, CTONG2002095, CTONG2002143, CTONG2002395, CTONG2002417, CTONG2002418, CTONG2002558, CTONG2002590, CTONG2002721, CTONG2002766, CTONG2002803, CTONG2002816, CTONG2002820, CTONG2002832, CTONG2002841, CTONG2002965, CTONG2002999, CTONG2003028, CTONG2003100, CTONG2003115, CTONG2003189, CTONG2003245, CTONG2003293, CTONG2003298, CTONG2003348, CTONG2003350, CTONG2003361, CTONG2003372, CTONG2003375, CTONG2003427, CTONG2003517, CTONG2003524, CTONG2003599, CTONG2003680,
CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005290, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2006004, CTONG2006129, CTONG2006223, CTONG2006235, CTONG2006273, CTONG2006310, CTONG2006377, CTONG2006393, CTONG2006412, CTONG2006449,
CTONG2006515, CTONG2006524, CTONG2006568, CTONG2006611, CTONG2006620, CTONG2006666, CTONG2006691, CTONG2006798, CTONG2006836, CTONG2006942, CTONG2006964, CTONG2007007, CTONG2007009, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007586, CTONG2007623, CTONG2007681, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008405, CTONG2008466, CTONG2008521, CTONG2008562, CTONG2008577, CTONG2008595, CTONG2008621, CTONG2008689, CTONG2008720, CTONG2008721,
CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010408, CTONG2010508, CTONG2010566, CTONG2010623, CTONG2010649, CTONG2010652,
CTONG2010803, CTONG2011188, CTONG2011429, CTONG2011474, CTONG2011676, CTONG2011716, CTONG2011765, CTONG2011779, CTONG2011801, CTONG2011815, CTONG2011822, CTONG2011920, CTONG2011985, CTONG2012029, CTONG2012050, CTONG2012077, CTONG2012101, CTONG2012123, CTONG2012158, CTONG2012230, CTONG2012422, CTONG2012425, CTONG2012447, CTONG2012453, CTONG2012473, CTONG2012476, CTONG2012533, CTONG2012554, CTONG2012564, CTONG2012661, CTONG2012724, CTONG2012738, CTONG2012745, CTONG2012758, CTONG2012843, CTONG2012901, CTONG2012996, CTONG2013128, CTONG2013178, CTONG2013284, CTONG2013339, CTONG2013348, CTONG2013352, CTONG2013381, CTONG2013393, CTONG2013511, CTONG2013630, CTONG2013803, CTONG2013907, CTONG2013934,
CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014705, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016408, CTONG2016499, CTONG2016505, CTONG2016559, CTONG2016658, CTONG2016775,
CTONG2016797, CTONG2016824, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017500, CTONG2017604, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018343, CTONG2018379, CTONG2018413, CTONG2018483, CTONG2018629, CTONG2018637, CTONG2018652, CTONG2018653, CTONG2018655, CTONG2018735, CTONG2018739, CTONG2018808, CTONG2018843, CTONG2018898, CTONG2018900, CTONG2018976, CTONG2019063, CTONG2019144,
CTONG2019232, CTONG2019400, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020411, CTONG2020522, CTONG2020638, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959,
CTONG2028097, CTONG2028486, CTONG2028518, CTONG2028687, CTONG2028758, CTONG3000686, CTONG3000707, CTONG3000896, CTONG3001123, CTONG3001501, CTONG3001560, CTONG3001605, CTONG3001746, CTONG3002020, CTONG3002127, CTONG3002370, CTONG3002412, CTONG3002433, CTONG3002518; CTONG3002552, CTONG3002588, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3005325, CTONG3005648, CTONG3005713, CTONG3005803, CTONG3005813, CTONG3006067, CTONG3006073,
CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009287, CTONG3009328, CTONG3009385, D3OST1000066, D3OST1000238, D3OST1000267, D3OST2000184, D3OST2000618, D3OST2000654, D3OST2000734, D3OST2001328, D3OST2001669, D3OST2001670, D3OST2001788, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000195, D3OST3000258, D3OST3000291, D3OST3000388,
D3OST3000493, D6OST2000127, D6OST2000169, D6OST2000245, D6OST2000358, D6OST2000464, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000107, DFNES2000143, DFNES2000146, DFNES2000153, DFNES2000212, DFNES2000335, DFNES2000426, DFNES2000443, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829,
DFNES2002220, DFNES2002509, DFNES2002550, DFNES2002588, DFNES2002817, DFNES2002966, DFNES2003081, DFNES2003255, DFNES2003742, DFNES2004346, DFNES2004354, DFNES2004383, DFNES2004608, DFNES2004684, DFNES2005266, DFNES2005527, DFNES2005690, DFNES2005766, DFNES2005779, DFNES2005875, DFNES2006346, DFNES2006944, DFNES2007113, DFNES2007299, DFNES2007332, DFNES2007641, DFNES2007757, DFNES2008088, DFNES2008160, DFNES2008280, DFNES2008881, DFNES2009482, DFNES2010502, DFNES2011192, DFNES2011221, DFNES2011245, DFNES2011380, ERLTF1000021, ERLTF2000324, ERLTF2000465, FCBBF1000023, FCBBF1000038, FCBBF1000063, FCBBF1000069, FCBBF1000115, FCBBF1000117, FCBBF1000118, FCBBF1000121, FCBBF1000125, FCBBF1000131,
FCBBF1000155, FCBBF1000157, FCBBF1000171, FCBBF1000174, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000437, FCBBF1000476, FCBBF1000506, FCBBF1000509, FCBBF1000546, FCBBF1000550, FCBBF1000556, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF1000760, FCBBF2000038, FCBBF2000087, FCBBF2000094,
FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000576, FCBBF2000677, FCBBF2000678, FCBBF2000733, FCBBF2000782, FCBBF2000808, FCBBF2000815, FCBBF2000825, FCBBF2000940, FCBBF2000951, FCBBF2001001, FCBBF2001088, FCBBF2001116, FCBBF2001183, FCBBF2001188, FCBBF2001211, FCBBF2001238, FCBBF2001243, FCBBF2001291, FCBBF2001299, FCBBF2001400, FCBBF2001427, FCBBF2001480, FCBBF2001529, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370, FCBBF2002541, FCBBF2002626, FCBBF2002801,
FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005645, FCBBF2005658, FCBBF2005733, FCBBF2005909, FCBBF2005966, FCBBF2006131, FCBBF2006380, FCBBF2006418, FCBBF2006452, FCBBF2006781, FCBBF2007012, FCBBF2007186, FCBBF2007265, FCBBF2007302, FCBBF2007556, FCBBF2007610, FCBBF2007633,
FCBBF2007840, FCBBF3000102, FCBBF3000110, FCBBF3000115, FCBBF3000120, FCBBF3000168, FCBBF3000175, FCBBF3000227, FCBBF3000229, FCBBF3000233, FCBBF3000269, FCBBF3000361, FCBBF3000367, FCBBF3000388, FCBBF3000462, FCBBF3000473, FCBBF3000501, FCBBF3000518, FCBBF3000536, FCBBF3000550, FCBBF3000847, FCBBF3001018, FCBBF3001020, FCBBF3001235, FCBBF3001281, FCBBF3001302, FCBBF3001377, FCBBF3001470, FCBBF3001594, FCBBF3001632, FCBBF3001818, FCBBF3001855, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305,
FCBBF3003373, FCBBF3003435, FCBBF3003475, FCBBF3003481, FCBBF3003557, FCBBF3003787, FCBBF3003800, FCBBF3003902, FCBBF3004041, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311,
FCBBF3008362, FCBBF3008382, FCBBF3008475, FCBBF3008556, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288,
FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014355, FCBBF3014567, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016928, FCBBF3016987, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729,
FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078,
FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028472, FCBBF3028528,
FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000129, FEBRA2000210, FEBRA2000220, FEBRA2000228, FEBRA2000253,
FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000409, FEBRA2000415, FEBRA2000436, FEBRA2000438, FEBRA2000452, FEBRA2000454, FEBRA2000462, FEBRA2000468, FEBRA2000491, FEBRA2000504, FEBRA2000532, FEBRA2000536, FEBRA2000538, FEBRA2000575, FEBRA2000656, FEBRA2000659, FEBRA2000680, FEBRA2000690, FEBRA2000727, FEBRA2000734, FEBRA2000740, FEBRA2000757, FEBRA2000762, FEBRA2000771, FEBRA2000772, FEBRA2000782, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000901, FEBRA2000909,
FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001294, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001828, FEBRA2001867,
FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002527, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308,
FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003822, FEBRA2003897, FEBRA2003907, FEBRA2004023, FEBRA2004026, FEBRA2004029, FEBRA2004053, FEBRA2004056, FEBRA2004091, FEBRA2004110, FEBRA2004191, FEBRA2004202, FEBRA2004219, FEBRA2004224, FEBRA2004237, FEBRA2004268, FEBRA2004329, FEBRA2004331, FEBRA2004443, FEBRA2004485, FEBRA2004490, FEBRA2004538, FEBRA2004592, FEBRA2004620, FEBRA2004651, FEBRA2004767, FEBRA2004813, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114,
FEBRA2005216, FEBRA2005291, FEBRA2005361, FEBRA2005377, FEBRA2005380, FEBRA2005427, FEBRA2005458, FEBRA2005476, FEBRA2005701, FEBRA2005726, FEBRA2005752, FEBRA2005778, FEBRA2005787, FEBRA2005788, FEBRA2005995, FEBRA2005998, FEBRA2006061, FEBRA2006092, FEBRA2006150, FEBRA2006165, FEBRA2006255, FEBRA2006315, FEBRA2006346, FEBRA2006354, FEBRA2006357, FEBRA2006368, FEBRA2006370, FEBRA2006379, FEBRA2006396, FEBRA2006401, FEBRA2006409, FEBRA2006418, FEBRA2006427, FEBRA2006476, FEBRA2006485, FEBRA2006491, FEBRA2006521, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017,
FEBRA2007176, FEBRA2007200, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007622, FEBRA2007708, FEBRA2007714, FEBRA2007793, FEBRA2007801, FEBRA2007818, FEBRA2007823, FEBRA2007880, FEBRA2007900, FEBRA2007901, FEBRA2007931, FEBRA2007957, FEBRA2008009, FEBRA2008036, FEBRA2008086, FEBRA2008134, FEBRA2008135, FEBRA2008159, FEBRA2008165, FEBRA2008172, FEBRA2008201, FEBRA2008210, FEBRA2008265, FEBRA2008266, FEBRA2008282, FEBRA2008287, FEBRA2008311, FEBRA2008341, FEBRA2008360, FEBRA2008459, FEBRA2008468, FEBRA2008475, FEBRA2008481, FEBRA2008542, FEBRA2008583,
FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009514, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090,
FEBRA2011356, FEBRA2011392, FEBRA2011414, FEBRA2011593, FEBRA2011665, FEBRA2012090, FEBRA2012120, FEBRA2012181, FEBRA2012195, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680,
FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255, FEBRA2022322, FEBRA2022601, FEBRA2022696, FEBRA2022729,
FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEBRA2028516, FEHRT2000325,
FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405,
HCASM2009424, HCASM2009463, HCASM2009588, HCHON1000015, HCHON1000131, HCHON1000142, HCHON1000176, HCHON1000191, HCHON2000038, HCHON2000100, HCHON2000156, HCHON2000199, HCHON2000212, HCHON2000271, HCHON2000315, HCHON2000323, HCHON2000344, HCHON2000364, HCHON2000395, HCHON2000417, HCHON2000418, HCHON2000473, HCHON2000503, HCHON2000626, HCHON2000660, HCHON2000663, HCHON2000673, HCHON2000676, HCHON2000698, HCHON2000699, HCHON2000705, HCHON2000738, HCHON2000743, HCHON2000751, HCHON2000815, HCHON2000817, HCHON2000818, HCHON2000819, HCHON2000832, HCHON2000851, HCHON2000898, HCHON2000935, HCHON2000942, HCHON2000956, HCHON2000994, HCHON2001050, HCHON2001108, HCHON2001110, HCHON2001116, HCHON2001161,
HCHON2001214, HCHON2001233, HCHON2001269, HCHON2001450, HCHON2001453, HCHON2001497, HCHON2001519, HCHON2001523, HCHON2001535, HCHON2001546, HCHON2001548, HCHON2001577, HCHON2001598, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987,
HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000099, HEART1000102, HEART1000118, HEART1000132, HEART1000151, HEART1000173, HEART2000024, HEART2000035, HEART2000099, HEART2000298, HEART2000306, HEART2000309, HEART2000411, HEART2000418, HEART2000444, HEART2000448, HEART2000492, HEART2000568, HEART2000611, HEART2000959,
HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006557, HEART2006787, HEART2006789, HEART2006909, HEART2007031, HEART2007231, HEART2007443, HEART2007767, HEART2008108, HEART2008111, HEART2008257, HEART2008509, HEART2008994, HEART2009000, HEART2009599, HEART2009680, HEART2010391, HEART2010492, HELAC2000158, HELAC2000301,
HHDPC1000001, HHDPC1000065, HHDPC1000114, HHDPC1000118, HHDPC2000070, HHDPC2000102, HHDPC2000104, HHDPC2000258, HHDPC2000315, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053,
HLUNG1000064, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000926, HLUNG2000950, HLUNG2000955, HLUNG2000970, HLUNG2001013, HLUNG2001126, HLUNG2001144, HLUNG2001146, HLUNG2001214, HLUNG2001439, HLUNG2001507,
HLUNG2001677, HLUNG2001814, HLUNG2001996, HLUNG2002006, HLUNG2002050, HLUNG2002085, HLUNG2002145, HLUNG2002295, HLUNG2002303, HLUNG2002334, HLUNG2002405, HLUNG2002429, HLUNG2002465, HLUNG2002562, HLUNG2002659, HLUNG2002707, HLUNG2002811, HLUNG2002916, HLUNG2002942, HLUNG2002949, HLUNG2002958, HLUNG2002982, HLUNG2003003, HLUNG2003049, HLUNG2003061, HLUNG2003162, HLUNG2003246, HLUNG2003331, HLUNG2003335, HLUNG2003378, HLUNG2003497, HLUNG2003691, HLUNG2003710, HLUNG2003714, HLUNG2003716, HLUNG2003833, HLUNG2003872, HLUNG2003918, HLUNG2003991, HLUNG2004014, HLUNG2004037, HLUNG2004067, HLUNG2004154, HLUNG2004217, HLUNG2004273, HLUNG2004388, HLUNG2004416, HLUNG2004521, HLUNG2004534, HLUNG2004684,
HLUNG2004707, HLUNG2004774, HLUNG2004775, HLUNG2005012, HLUNG2005030, HLUNG2005045, HLUNG2005076, HLUNG2005133, HLUNG2005203, HLUNG2005479, HLUNG2005524, HLUNG2005656, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521,
HLUNG2008833, HLUNG2008875, HLUNG2008885, HLUNG2008958, HLUNG2008959, HLUNG2009067, HLUNG2009225, HLUNG2009253, HLUNG2009303, HLUNG2009413, HLUNG2009729, HLUNG2010181, HLUNG2010464, HLUNG2010562, HLUNG2010845, HLUNG2011041, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282,
HLUNG2018626, HLUNG2019058, HSYRA1000131, HSYRA1000148, HSYRA2000044, HSYRA2000135, HSYRA2000190, HSYRA2000221, HSYRA2000237, HSYRA2000253, HSYRA2000255, HSYRA2000332, HSYRA2000371, HSYRA2000424, HSYRA2000507, HSYRA2000510, HSYRA2000629, HSYRA2000706, HSYRA2000760, HSYRA2000792, HSYRA2000832, HSYRA2000925, HSYRA2000927, HSYRA2001003, HSYRA2001103, HSYRA2001142, HSYRA2001153, HSYRA2001232, HSYRA2001255, HSYRA2001332, HSYRA2001353, HSYRA2001420, HSYRA2001484, HSYRA2001552, HSYRA2001567, HSYRA2001580, HSYRA2001595, HSYRA2001631, HSYRA2001983, HSYRA2002103, HSYRA2002590, HSYRA2003168, HSYRA2004550, HSYRA2004858, HSYRA2005456, HSYRA2005496, HSYRA2005628, HSYRA2005970, HSYRA2006049, HSYRA2006873,
HSYRA2007241, HSYRA2007338, HSYRA2007667, HSYRA2008154, HSYRA2008714, HSYRA2009102, IMR321000155, IMR321000158, IMR321000161, IMR321000165, IMR321000193, IMR321000207, IMR321000230, IMR321000242, IMR321000253, IMR322000010, IMR322000072, IMR322000107, IMR322000152, IMR322000164, IMR322000302, IMR322000374, IMR322000379, IMR322000383, IMR322000418, IMR322000518, IMR322000555, IMR322000604, IMR322000609, IMR322000656, IMR322000672, IMR322000742, IMR322000791, IMR322000819, IMR322000838, IMR322000859, IMR322000863, IMR322000917, IMR322000919, IMR322000953, IMR322000973, IMR322000984, IMR322001018, IMR322001167, IMR322001185, IMR322001245, IMR322001317, IMR322001318, IMR322001322, IMR322001332,
IMR322001380, IMR322001438, IMR322001452, IMR322001491, IMR322001534, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000036,
KIDNE1000064, KIDNE1000104, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000266, KIDNE2000287, KIDNE2000315, KIDNE2000317, KIDNE2000328, KIDNE2000330, KIDNE2000335, KIDNE2000349, KIDNE2000375, KIDNE2000394, KIDNE2000422, KIDNE2000493, KIDNE2000510, KIDNE2000513, KIDNE2000519, KIDNE2000555, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2000947, KIDNE2001117, KIDNE2001140, KIDNE2001269, KIDNE2001361, KIDNE2001373,
KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882, KIDNE2002991, KIDNE2003185, KIDNE2003188, KIDNE2003233, KIDNE2003316, KIDNE2003335, KIDNE2003357, KIDNE2003373, KIDNE2003377, KIDNE2003507, KIDNE2003752, KIDNE2003764, KIDNE2003837, KIDNE2003941, KIDNE2003994, KIDNE2004034, KIDNE2004054, KIDNE2004084, KIDNE2004095, KIDNE2004115, KIDNE2004233, KIDNE2004262, KIDNE2004279,
KIDNE2004294, KIDNE2004295, KIDNE2004305, KIDNE2004344, KIDNE2004394, KIDNE2004519, KIDNE2004520, KIDNE2004531, KIDNE2004534, KIDNE2004562, KIDNE2004579, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006014, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334,
KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077, KIDNE2007186, KIDNE2007222, KIDNE2007422, KIDNE2007569, KIDNE2007810, KIDNE2007811, KIDNE2007910, KIDNE2007944, KIDNE2008022, KIDNE2008069, KIDNE2008072, KIDNE2008117, KIDNE2008218, KIDNE2008315, KIDNE2008362, KIDNE2008378, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109,
KIDNE2009180, KIDNE2009191, KIDNE2009332, KIDNE2009367, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012710,
KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015483, KIDNE2015515, KIDNE2015546, KIDNE2015556, KIDNE2015598, KIDNE2015660, KIDNE2015710, KIDNE2015987, KIDNE2016000,
KIDNE2016036, KIDNE2016096, KIDNE2016101, KIDNE2016142, KIDNE2016188, KIDNE2016327, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099,
LIVER1000104, LIVER1000111, LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001835, LIVER2002638, LIVER2002644, LIVER2002660, LIVER2002702, LIVER2003008, LIVER2003065, LIVER2003234, LIVER2003511,
LIVER2003524, LIVER2003568, LIVER2003581, LIVER2003800, LIVER2003854, LIVER2004074, LIVER2004392, LIVER2004514, LIVER2004565, LIVER2005198, LIVER2005218, LIVER2005376, LIVER2005385, LIVER2005512, LIVER2005520, LIVER2005527, LIVER2005544, LIVER2005625, LIVER2005728, LIVER2005973, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000100, MAMGL1000132, MAMGL1000151, MAMGL1000178,
MAMGL1000182, MAMGL1000184, MESAN1000032, MESAN1000035, MESAN1000050, MESAN1000079, MESAN1000080, MESAN1000121, MESAN1000147, MESAN1000180, MESAN2000023, MESAN2000026, MESAN2000067, MESAN2000092, MESAN2000149, MESAN2000167, MESAN2000291, MESAN2000337, MESAN2000434, MESAN2000462, MESAN2000501, MESAN2000711, MESAN2000815, MESAN2000894, MESAN2000909, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002844, MESAN2002978, MESAN2003037, MESAN2003039, MESAN2003101, MESAN2003190, MESAN2003322, MESAN2003490, MESAN2003529, MESAN2003646, MESAN2003662, MESAN2003709, MESAN2003851,
MESAN2004138, MESAN2004288, MESAN2004575, MESAN2005284, MESAN2005371, MESAN2005633, MESAN2005689, MESAN2005724, MESAN2005808, MESAN2005811, MESAN2006022, MESAN2006043, MESAN2006328, MESAN2006401, MESAN2006580, MESAN2006599, MESAN2006953, MESAN2006971, MESAN2007032, MESAN2007440, MESAN2007597, MESAN2007755, MESAN2008222, MESAN2008334, MESAN2008460, MESAN2008536, MESAN2008679, MESAN2008729, MESAN2008821, MESAN2008926, MESAN2009019, MESAN2009081, MESAN2009105, MESAN2009311, MESAN2009418, MESAN2009503, MESAN2009522, MESAN2010031, MESAN2010114, MESAN2010312, MESAN2010321, MESAN2010664, MESAN2010681, MESAN2011545, MESAN2011597, MESAN2011632, MESAN2011977, MESAN2012586, MESAN2012708, MESAN2012735,
MESAN2013019, MESAN2013022, MESAN2013183, MESAN2013211, MESAN2013283, MESAN2013284, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESTC1000042, MESTC1000147, MESTC2000150, MESTC2000153, MESTC2000170, N1ESE2000698, NB9N41000047, NB9N41000121, NB9N41000135, NB9N41000142, NB9N41000146, NB9N41000340, NB9N42000104, NB9N42000122, NB9N42000196,
NB9N42000281, NB9N42000314, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000102, NOVAR2000136, NOVAR2000352, NOVAR2000412, NOVAR2000710,
NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000222, NT2NE2000284, NT2NE2000299, NT2NE2000353, NT2NE2000356, NT2NE2000369, NT2NE2000374, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658,
NT2NE2000706, NT2NE2000763, NT2NE2000802, NT2NE2000808, NT2NE2000809, NT2NE2000864, NT2NE2000909, NT2NE2000913, NT2NE2000950, NT2NE2000951, NT2NE2000980, NT2NE2001005, NT2NE2001021, NT2NE2001040, NT2NE2001048, NT2NE2001049, NT2NE2001142, NT2NE2001156, NT2NE2001176, NT2NE2001180, NT2NE2001181, NT2NE2001188, NT2NE2001191, NT2NE2001247, NT2NE2001252, NT2NE2001324, NT2NE2001326, NT2NE2001364, NT2NE2001372, NT2NE2001397, NT2NE2001403, NT2NE2001428, NT2NE2001432, NT2NE2001442, NT2NE2001524, NT2NE2001598, NT2NE2001617, NT2NE2001623, NT2NE2001626, NT2NE2001634, NT2NE2001648, NT2NE2001660, NT2NE2001666, NT2NE2001677, NT2NE2001697, NT2NE2001698, NT2NE2001723, NT2NE2001725, NT2NE2001874, NT2NE2001889,
NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2003150, NT2NE2003185, NT2NE2003252, NT2NE2003280, NT2NE2003308, NT2NE2003309, NT2NE2003315, NT2NE2003393, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004519, NT2NE2004606, NT2NE2004740, NT2NE2004787, NT2NE2004916, NT2NE2005035, NT2NE2005223, NT2NE2005276, NT2NE2005317, NT2NE2005323, NT2NE2005358, NT2NE2005395, NT2NE2005419, NT2NE2005517, NT2NE2005537, NT2NE2005555, NT2NE2005588,
NT2NE2005676, NT2NE2005720, NT2NE2005784, NT2NE2005804, NT2NE2005821, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295,
NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2013019, NT2NE2013081, NT2NE2013189, NT2NE2013217, NT2NE2013547, NT2NE2014013, NT2NE2014028, NT2NE2014113,
NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490,
NT2NE2018594, NT2NE2018739, NT2NE2018916, NT2NE2019056, NT2NE2019076, NT2NE2019091, NT2NE2019099, NT2RI1000016, NT2RI1000035, NT2RI1000048, NT2RI1000127, NT2RI1000164, NT2RI1000172, NT2RI2000004, NT2RI2000007, NT2RI2000116, NT2RI2000117, NT2RI2000128, NT2RI2000133, NT2RI2000167, NT2RI2000173, NT2RI2000187, NT2RI2000263, NT2RI2000270, NT2RI2000276, NT2RI2000284, NT2RI2000294, NT2RI2000313, NT2RI2000341, NT2RI2000344, NT2RI2000348, NT2RI2000412, NT2RI2000421, NT2RI2000480, NT2RI2000575, NT2RI2000578, NT2RI2000588, NT2RI2000597, NT2RI2000669, NT2RI2000688, NT2RI2000704, NT2RI2000738, NT2RI2000775, NT2RI2000829, NT2RI2000841, NT2RI2000865, NT2RI2000932, NT2RI2000987, NT2RI2001010, NT2RI2001017,
NT2RI2001030, NT2RI2001054, NT2RI2001083, NT2RI2001091, NT2RI2001167, NT2RI2001178, NT2RI2001235, NT2RI2001244, NT2RI2001307, NT2RI2001385, NT2RI2001409, NT2RI2001410, NT2RI2001422, NT2RI2001425, NT2RI2001449, NT2RI2001450, NT2RI2001474, NT2RI2001519, NT2RI2001621, NT2RI2001624, NT2RI2001726, NT2RI2001731, NT2RI2001769, NT2RI2001782, NT2RI2001836, NT2RI2001846, NT2RI2001866, NT2RI2001910, NT2RI2001952, NT2RI2002022, NT2RI2002041, NT2RI2002117, NT2RI2002120, NT2RI2002152, NT2RI2002153, NT2RI2002179, NT2RI2002243, NT2RI2002252, NT2RI2002260, NT2RI2002266, NT2RI2002270, NT2RI2002334, NT2RI2002447, NT2RI2002481, NT2RI2002507, NT2RI2002517, NT2RI2002540, NT2RI2002549, NT2RI2002564, NT2RI2002585,
NT2RI2002592, NT2RI2002602, NT2RI2002802, NT2RI2002822, NT2RI2002852, NT2RI2002923, NT2RI2002926, NT2RI2002970, NT2RI2003011, NT2RI2003067, NT2RI2003184, NT2RI2003205, NT2RI2003222, NT2RI2003301, NT2RI2003344, NT2RI2003360, NT2RI2003361, NT2RI2003383, NT2RI2003392, NT2RI2003399, NT2RI2003419, NT2RI2003481, NT2RI2003487, NT2RI2003655, NT2RI2003667, NT2RI2003678, NT2RI2003751, NT2RI2003884, NT2RI2003973, NT2RI2004059, NT2RI2004078, NT2RI2004079, NT2RI2004093, NT2RI2004136, NT2RI2004157, NT2RI2004188, NT2RI2004190, NT2RI2004209, NT2RI2004284, NT2RI2004290, NT2RI2004304, NT2RI2004312, NT2RI2004381, NT2RI2004410, NT2RI2004442, NT2RI2004468, NT2RI2004535, NT2RI2004606, NT2RI2004618, NT2RI2004783,
NT2RI2004818, NT2RI2004840, NT2RI2004884, NT2RI2004916, NT2RI2004962, NT2RI2004984, NT2RI2004985, NT2RI2005048, NT2RI2005069, NT2RI2005087, NT2RI2005096, NT2RI2005130, NT2RI2005150, NT2RI2005166, NT2RI2005239, NT2RI2005246, NT2RI2005315, NT2RI2005358, NT2RI2005368, NT2RI2005382, NT2RI2005473, NT2RI2005520, NT2RI2005564, NT2RI2005579, NT2RI2005621, NT2RI2005628, NT2RI2005647, NT2RI2005670, NT2RI2005710, NT2RI2005713, NT2RI2005772, NT2RI2005816, NT2RI2005818, NT2RI2005851, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006412, NT2RI2006445, NT2RI2006487, NT2RI2006506, NT2RI2006640,
NT2RI2006659, NT2RI2006662, NT2RI2006667, NT2RI2006679, NT2RI2006703, NT2RI2006716, NT2RI2006735, NT2RI2006762, NT2RI2006788, NT2RI2006838, NT2RI2006853, NT2RI2006943, NT2RI2006973, NT2RI2007046, NT2RI2007048, NT2RI2007084, NT2RI2007096, NT2RI2007116, NT2RI2007148, NT2RI2007214, NT2RI2007277, NT2RI2007303, NT2RI2007334, NT2RI2007386, NT2RI2007406, NT2RI2007439, NT2RI2007445, NT2RI2007468, NT2RI2007469, NT2RI2007507, NT2RI2007572, NT2RI2007593, NT2RI2007629, NT2RI2007723, NT2RI2007729, NT2RI2007751, NT2RI2007754, NT2RI2007827, NT2RI2007877, NT2RI2007884, NT2RI2007891, NT2RI2007956, NT2RI2007965, NT2RI2008007, NT2RI2008045, NT2RI2008050, NT2RI2008171, NT2RI2008188, NT2RI2008204, NT2RI2008233,
NT2RI2008455, NT2RI2008481, NT2RI2008502, NT2RI2008566, NT2RI2008598, NT2RI2008622, NT2RI2008656, NT2RI2008714, NT2RI2008724, NT2RI2008801, NT2RI2008808, NT2RI2008841, NT2RI2008942, NT2RI2008952, NT2RI2009005, NT2RI2009066, NT2RI2009083, NT2RI2009101, NT2RI2009103, NT2RI2009144, NT2RI2009151, NT2RI2009173, NT2RI2009194, NT2RI2009215, NT2RI2009233, NT2RI2009259, NT2RI2009289, NT2RI2009301, NT2RI2009402, NT2RI2009406, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2013357, NT2RI2013373, NT2RI2014551, NT2RI2014683, NT2RI2014733, NT2RI2015239, NT2RI2016128,
NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445,
NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006376, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697,
NT2RI3008974, NT2RI3009158, NT2RI3009480, NT2RI3009524, NT2RP6000008, NT2RP6000017, NT2RP6000033, NT2RP6000043, NT2RP6000059, NT2RP6000060, NT2RP6000085, NT2RP6000086, NT2RP6000100, NT2RP6000109, NT2RP6000119, NT2RP6000127, NT2RP6000130, NT2RP6000140, NT2RP6000150, NT2RP7000041, NT2RP7000086, NT2RP7000109, NT2RP7000112, NT2RP7000238, NT2RP7000259, NT2RP7000311, NT2RP7000359, NT2RP7000364, NT2RP7000380, NT2RP7000391, NT2RP7000425, NT2RP7000579, NT2RP7000586, NT2RP7000624, NT2RP7000812, NT2RP7000906, NT2RP7000926, NT2RP7001074, NT2RP7001080, NT2RP7001156, NT2RP7001231, NT2RP7001306, NT2RP7001319, NT2RP7001335, NT2RP7001364, NT2RP7001475, NT2RP7001532, NT2RP7001591, NT2RP7001602, NT2RP7001678,
NT2RP7001856, NT2RP7002064, NT2RP7002151, NT2RP7002163, NT2RP7002243, NT2RP7002282, NT2RP7002376, NT2RP7002449, NT2RP7002450, NT2RP7002478, NT2RP7002554, NT2RP7002594, NT2RP7002603, NT2RP7002650, NT2RP7002738, NT2RP7002779, NT2RP7002802, NT2RP7002829, NT2RP7002841, NT2RP7002875, NT2RP7002906, NT2RP7002978, NT2RP7003025, NT2RP7003050, NT2RP7003055, NT2RP7003084, NT2RP7003091, NT2RP7003107, NT2RP7003134, NT2RP7003148, NT2RP7003439, NT2RP7003511, NT2RP7003632, NT2RP7003680, NT2RP7003960, NT2RP7004027, NT2RP7004080, NT2RP7004123, NT2RP7004204, NT2RP7004233, NT2RP7004260, NT2RP7004348, NT2RP7004352, NT2RP7004358, NT2RP7004396, NT2RP7004428, NT2RP7004481, NT2RP7004541, NT2RP7004559, NT2RP7004641,
NT2RP7004656, NT2RP7004687, NT2RP7004722, NT2RP7004766, NT2RP7004790, NT2RP7004884, NT2RP7004911, NT2RP7004915, NT2RP7004961, NT2RP7004964, NT2RP7004975, NT2RP7005118, NT2RP7005205, NT2RP7005219, NT2RP7005323, NT2RP7005468, NT2RP7005493, NT2RP7005529, NT2RP7005629, NT2RP7005631, NT2RP7005669, NT2RP7005846, NT2RP7006160, NT2RP7006162, NT2RP7006223, NT2RP7006263, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006621, NT2RP7006636, NT2RP7006701, NT2RP7006717, NT2RP7006853, NT2RP7006886, NT2RP7006887, NT2RP7006986, NT2RP7006995, NT2RP7007114, NT2RP7007221, NT2RP7007226, NT2RP7007252, NT2RP7007269,
NT2RP7007310, NT2RP7007359, NT2RP7007381, NT2RP7007406, NT2RP7007480, NT2RP7007504, NT2RP7007530, NT2RP7007580, NT2RP7007594, NT2RP7007610, NT2RP7007617, NT2RP7007643, NT2RP7007766, NT2RP7007842, NT2RP7007925, NT2RP7007930, NT2RP7008013, NT2RP7008015, NT2RP7008133, NT2RP7008137, NT2RP7008142, NT2RP7008144, NT2RP7008315, NT2RP7008360, NT2RP7008406, NT2RP7008435, NT2RP7008441, NT2RP7008454, NT2RP7008487, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009108, NT2RP7009168, NT2RP7009215, NT2RP7009225, NT2RP7009236, NT2RP7009253, NT2RP7009267, NT2RP7009322,
NT2RP7009363, NT2RP7009370, NT2RP7009373, NT2RP7009397, NT2RP7009429, NT2RP7009439, NT2RP7009466, NT2RP7009481, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080,
NT2RP7015431, NT2RP7015503, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787,
NT2RP8004306, NT2RP8005546, NT2RP8006452, NT2RP8006521, NT2RP8007416, NT2RP8007503, NT2RP8007715, NT2RP8008057, NT2RP8009248, NTONG1000006, NTONG1000047, NTONG1000075, NTONG1000087, NTONG1000130, NTONG1000161, NTONG1000172, NTONG1000213, NTONG1000214, NTONG1000246, NTONG1000247, NTONG2000040, NTONG2000107, NTONG2000231, NTONG2000363, NTONG2000413, NTONG2000492, NTONG2000499, NTONG2000531, NTONG2000583, NTONG2000800, NTONG2000966, NTONG2001014, NTONG2001079, NTONG2001137, NTONG2001157, NTONG2001173, NTONG2001222, NTONG2001362, NTONG2001428, NTONG2001532, NTONG2001587, NTONG2001619, NTONG2001642, NTONG2001657, NTONG2001676, NTONG2001762, NTONG2002278, NTONG2002582, NTONG2002807, NTONG2002970,
NTONG2002985, NTONG2003158, NTONG2003210, NTONG2003316, NTONG2003409, NTONG2003515, NTONG2003805, NTONG2003839, NTONG2003897, NTONG2003928, NTONG2004095, NTONG2004308, NTONG2004521, NTONG2004690, NTONG2004806, NTONG2004829, NTONG2004918, NTONG2004991, NTONG2005062, NTONG2005086, NTONG2005137, NTONG2005153, NTONG2005265, NTONG2005277, NTONG2005363, NTONG2005373, NTONG2005480, NTONG2005577, NTONG2005657, NTONG2005822, NTONG2005897, NTONG2005969, NTONG2006099, NTONG2006187, NTONG2006301, NTONG2006324, NTONG2006354, NTONG2006440, NTONG2006501, NTONG2006646, NTONG2006709, NTONG2007020, NTONG2007028, NTONG2007249, NTONG2007517, NTONG2007522, NTONG2007693, NTONG2008088, NTONG2008093, NTONG2008143,
NTONG2008365, NTONG2008522, NTONG2008672, NTONG2008862, NTONG2008939, NTONG2009068, NTONG2009229, NTONG2009233, NTONG2009468, NTONG2009576, OCBBF1000016, OCBBF1000030, OCBBF1000042, OCBBF1000049, OCBBF1000054, OCBBF1000067, OCBBF1000080, OCBBF1000085, OCBBF1000086, OCBBF1000091, OCBBF1000104, OCBBF1000114, OCBBF1000118, OCBBF1000119, OCBBF1000122, OCBBF1000130, OCBBF1000138, OCBBF1000145, OCBBF1000153, OCBBF1000175, OCBBF1000185, OCBBF1000192, OCBBF1000254, OCBBF2000126, OCBBF2000178, OCBBF2000231, OCBBF2000277, OCBBF2000287, OCBBF2000323, OCBBF2000452, OCBBF2000467, OCBBF2000522, OCBBF2000677, OCBBF2000703, OCBBF2000712, OCBBF2000719, OCBBF2000831, OCBBF2000904, OCBBF2000982, OCBBF2000998,
OCBBF2001075, OCBBF2001101, OCBBF2001124, OCBBF2001140, OCBBF2001166, OCBBF2001176, OCBBF2001186, OCBBF2001196, OCBBF2001252, OCBBF2001323, OCBBF2001389, OCBBF2001402, OCBBF2001408, OCBBF2001416, OCBBF2001473, OCBBF2001494, OCBBF2001527, OCBBF2001528, OCBBF2001681, OCBBF2001706, OCBBF2001749, OCBBF2001794, OCBBF2001910, OCBBF2001931, OCBBF2001961, OCBBF2002015, OCBBF2002083, OCBBF2002124, OCBBF2002161, OCBBF2002290, OCBBF2002357, OCBBF2002376, OCBBF2002615, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003543, OCBBF2003593, OCBBF2003744, OCBBF2003819, OCBBF2003925, OCBBF2004038,
OCBBF2004104, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2006030, OCBBF2006058, OCBBF2006113, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764,
OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007415, OCBBF2007428, OCBBF2007478, OCBBF2007485, OCBBF2007520, OCBBF2007555, OCBBF2007610, OCBBF2007756, OCBBF2007762, OCBBF2007892, OCBBF2007931, OCBBF2007946, OCBBF2008005, OCBBF2008041, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775,
OCBBF2008790, OCBBF2008814, OCBBF2008822, OCBBF2009115, OCBBF2009242, OCBBF2009301, OCBBF2009352, OCBBF2009391, OCBBF2009424, OCBBF2009536, OCBBF2009571, OCBBF2009583, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010830, OCBBF2010841, OCBBF2010843, OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011160, OCBBF2011177, OCBBF2011228, OCBBF2011232, OCBBF2011283, OCBBF2011311, OCBBF2011536, OCBBF2011625, OCBBF2011669,
OCBBF2011685, OCBBF2011706, OCBBF2011722, OCBBF2011759, OCBBF2011767, OCBBF2011855, OCBBF2011872, OCBBF2011897, OCBBF2011914, OCBBF2011960, OCBBF2011981, OCBBF2012001, OCBBF2012028, OCBBF2012039, OCBBF2012139, OCBBF2012191, OCBBF2012262, OCBBF2012320, OCBBF2012436, OCBBF2012525, OCBBF2012553, OCBBF2012678, OCBBF2012704, OCBBF2012755, OCBBF2012812, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013843, OCBBF2013883, OCBBF2013926, OCBBF2014052, OCBBF2014064, OCBBF2014089,
OCBBF2014292, OCBBF2014322, OCBBF2014386, OCBBF2014541, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2017069, OCBBF2017306, OCBBF2017325, OCBBF2017489, OCBBF2017516, OCBBF2017665, OCBBF2017815, OCBBF2017882, OCBBF2017893, OCBBF2017920, OCBBF2018014, OCBBF2018084, OCBBF2018167, OCBBF2018206,
OCBBF2018229, OCBBF2018234, OCBBF2018362, OCBBF2018380, OCBBF2018563, OCBBF2018581, OCBBF2018618, OCBBF2018663, OCBBF2018687, OCBBF2018707, OCBBF2018827, OCBBF2018828, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451,
OCBBF2025458, OCBBF2025527, OCBBF2025631, OCBBF2025730, OCBBF2026025, OCBBF2026035, OCBBF2026144, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948,
OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972,
OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, SALGL1000024, SALGL1000028, SALGL1000065, SALGL1000107, SALGL1000171, SKMUS1000014, SKMUS1000030, SKMUS1000067, SKMUS1000102, SKMUS1000104, SKMUS1000110, SKMUS1000118, SKMUS1000124, SKMUS1000129, SKMUS1000176, SKMUS1000177, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000365, SKMUS2000390, SKMUS2000416, SKMUS2000467, SKMUS2000468, SKMUS2000484, SKMUS2000690,
SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000894, SKMUS2000898, SKMUS2000902, SKMUS2000933, SKMUS2000945, SKMUS2000954, SKMUS2000961, SKMUS2000973, SKMUS2000980, SKMUS2001014, SKMUS2001164, SKMUS2001199, SKMUS2001215, SKMUS2001247, SKMUS2001254, SKMUS2001271, SKMUS2001287, SKMUS2001323, SKMUS2001364, SKMUS2001398, SKMUS2001412, SKMUS2001492, SKMUS2001501, SKMUS2001528, SKMUS2001543, SKMUS2001580, SKMUS2001585, SKMUS2001608, SKMUS2001631, SKMUS2001662, SKMUS2001663, SKMUS2001671, SKMUS2001683, SKMUS2001703, SKMUS2001740, SKMUS2001742, SKMUS2001823, SKMUS2001850, SKMUS2002077,
SKMUS2002084, SKMUS2002153, SKMUS2002475, SKMUS2002602, SKMUS2002693, SKMUS2002821, SKMUS2002840, SKMUS2002920, SKMUS2003034, SKMUS2003074, SKMUS2003194, SKMUS2003744, SKMUS2004044, SKMUS2004047, SKMUS2004483, SKMUS2004897, SKMUS2004908, SKMUS2005428, SKMUS2006394, SKMUS2006481, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000159, SKNMC1000229, SKNMC1000251, SKNMC1000264, SKNMC2000065, SKNMC2000224, SKNMC2000256, SKNMC2000305, SKNMC2000356, SKNMC2000374, SKNMC2000583, SKNMC2000593,
SKNMC2000612, SKNMC2000622, SKNMC2001113, SKNMC2001324, SKNMC2001503, SKNMC2001555, SKNMC2001596, SKNMC2001623, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000086, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000482, SKNSH2000516, SKNSH2000550, SKNSH2000716, SKNSH2000819, SKNSH2000971, SKNSH2001222, SKNSH2001875, SKNSH2001931, SKNSH2002054, SKNSH2002325, SKNSH2002866, SKNSH2003064, SKNSH2003174, SKNSH2003422, SKNSH2003466,
SKNSH2003483, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008969, SKNSH2009197, SKNSH2009357, SKNSH2009435, SKNSH2009991, SKNSH2010015, SMINT1000012, SMINT1000016, SMINT1000039, SMINT1000048, SMINT1000051, SMINT1000054, SMINT1000057, SMINT1000071, SMINT1000075, SMINT1000091, SMINT1000103, SMINT1000118, SMINT1000137, SMINT1000192, SMINT2000007, SMINT2000032, SMINT2000040, SMINT2000060, SMINT2000073, SMINT2000084, SMINT2000145, SMINT2000159, SMINT2000176, SMINT2000185, SMINT2000227,
SMINT2000229, SMINT2000232, SMINT2000239, SMINT2000267, SMINT2000400, SMINT2000454, SMINT2000518, SMINT2000529, SMINT2000530, SMINT2000537, SMINT2000538, SMINT2000541, SMINT2000558, SMINT2000562, SMINT2000568, SMINT2000609, SMINT2000629, SMINT2000659, SMINT2000694, SMINT2000747, SMINT2000811, SMINT2000824, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002159, SMINT2002202, SMINT2002210, SMINT2002224, SMINT2002281, SMINT2002311, SMINT2002314, SMINT2002328, SMINT2002414,
SMINT2002457, SMINT2002743, SMINT2002778, SMINT2002880, SMINT2002882, SMINT2002884, SMINT2002899, SMINT2002974, SMINT2003003, SMINT2003074, SMINT2003128, SMINT2003150, SMINT2003169, SMINT2003317, SMINT2003340, SMINT2003386, SMINT2003423, SMINT2003551, SMINT2003569, SMINT2003641, SMINT2003644, SMINT2003866, SMINT2003905, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387,
SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008625, SMINT2008672, SMINT2008690, SMINT2008715, SMINT2008720,
SMINT2008817, SMINT2008844, SMINT2008869, SMINT2009119, SMINT2009121, SMINT2009212, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010959, SMINT2010997, SMINT2011033, SMINT2011066, SMINT2011273, SMINT2011406, SMINT2011509, SMINT2011588, SMINT2011656, SMINT2011888, SMINT2011958, SMINT2012040, SMINT2012179,
SMINT2012195, SMINT2012285, SMINT2012291, SMINT2012501, SMINT2012734, SMINT2012793, SMINT2013031, SMINT2013032, SMINT2013129, SMINT2013181, SMINT2013228, SMINT2013613, SMINT2013626, SMINT2013695, SMINT2013833, SMINT2013890, SMINT2014166, SMINT2014282, SMINT2014443, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659,
SMINT2017736, SMINT2017781, SMINT2017855, SMINT2017884, SMINT2017964, SMINT2017974, SMINT2018343, SMINT2018353, SMINT2018592, SMINT2019017, SMINT2019105, SMINT2019142, SMINT2019153, SMINT2019200, SPLEN1000041, SPLEN1000049, SPLEN1000056, SPLEN1000087, SPLEN1000089, SPLEN1000091, SPLEN1000106, SPLEN1000116, SPLEN1000134, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000242, SPLEN2000243, SPLEN2000247, SPLEN2000255, SPLEN2000258, SPLEN2000267, SPLEN2000270, SPLEN2000307,
SPLEN2000310, SPLEN2000341, SPLEN2000350, SPLEN2000357, SPLEN2000364, SPLEN2000402, SPLEN2000414, SPLEN2000420, SPLEN2000448, SPLEN2000450, SPLEN2000464, SPLEN2000496, SPLEN2000505, SPLEN2000515, SPLEN2000516, SPLEN2000537, SPLEN2000541, SPLEN2000607, SPLEN2000630, SPLEN2000695, SPLEN2000698, SPLEN2000839, SPLEN2000874, SPLEN2001135, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175,
SPLEN2002223, SPLEN2002272, SPLEN2002314, SPLEN2002343, SPLEN2002354, SPLEN2002385, SPLEN2002419, SPLEN2002451, SPLEN2002462, SPLEN2002463, SPLEN2002662, SPLEN2002695, SPLEN2002707, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004343, SPLEN2004346, SPLEN2004368, SPLEN2004555, SPLEN2004611, SPLEN2004662, SPLEN2004773, SPLEN2004844, SPLEN2004880, SPLEN2004984, SPLEN2005009, SPLEN2005227, SPLEN2005272, SPLEN2005342, SPLEN2005416, SPLEN2005450, SPLEN2005474, SPLEN2005560, SPLEN2005727,
SPLEN2005783, SPLEN2005790, SPLEN2005806, SPLEN2005818, SPLEN2005838, SPLEN2005869, SPLEN2005927, SPLEN2005939, SPLEN2006143, SPLEN2006268, SPLEN2006283, SPLEN2006325, SPLEN2006374, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007879, SPLEN2007926, SPLEN2007951, SPLEN2008007, SPLEN2008135, SPLEN2008164, SPLEN2008317, SPLEN2008496, SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009555,
SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011758, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234,
SPLEN2012453, SPLEN2012524, SPLEN2012525, SPLEN2012571, SPLEN2012606, SPLEN2012611, SPLEN2012619, SPLEN2012624, SPLEN2012725, SPLEN2012743, SPLEN2012756, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860,
SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016421, SPLEN2016480, SPLEN2016531, SPLEN2016533, SPLEN2016541, SPLEN2016552, SPLEN2016602, SPLEN2016627, SPLEN2016678, SPLEN2016743,
SPLEN2016773, SPLEN2016781, SPLEN2016840, SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018098, SPLEN2018112, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019092, SPLEN2019102, SPLEN2019131, SPLEN2019169,
SPLEN2019257, SPLEN2019311, SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701,
SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066,
SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031547, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551,
SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036953, SPLEN2037077, SPLEN2037194, SPLEN2037319, SPLEN2037444, SPLEN2037580, SPLEN2037630, SPLEN2037678, SPLEN2037891, SPLEN2038055, SPLEN2038180, SPLEN2038345, SPLEN2038407, SPLEN2038473, SPLEN2039311, SPLEN2039379, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303,
SPLEN2042521, SPLEN2042598, SPLEN2042714, STOMA1000013, STOMA1000047, STOMA1000052, STOMA1000074, STOMA1000091, STOMA1000117, STOMA1000133, STOMA1000175, STOMA1000186, STOMA1000190, STOMA1000191, STOMA2000032, STOMA2000072, STOMA2000088, STOMA2000104, STOMA2000148, STOMA2000168, STOMA2000183, STOMA2000188, STOMA2000279, STOMA2000289, STOMA2000383, STOMA2000386, STOMA2000395, STOMA2000396, STOMA2000478, STOMA2000529, STOMA2000539, STOMA2000567, STOMA2000640, STOMA2000678, STOMA2000686, STOMA2000888, STOMA2001025, STOMA2001389, STOMA2002052, STOMA2002141, STOMA2002367, STOMA2002688, STOMA2002828, STOMA2003158, STOMA2003289, STOMA2003444, STOMA2003477, STOMA2003646, STOMA2003716, STOMA2003781,
STOMA2003894, STOMA2004194, STOMA2004294, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291,
SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001251, SYNOV2001300, SYNOV2001356, SYNOV2001390, SYNOV2001451, SYNOV2001457, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320,
SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, T1ESE2000116, T1ESE2000609, T1ESE2000904, TBAES2000004,
TBAES2000059, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000127, TESOP1000160,
TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006060, TESOP2006068, TESOP2006110, TESOP2006398, TESOP2006670, TESOP2006704,
TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000055, TESTI1000070, TESTI1000085, TESTI1000088, TESTI1000093, TESTI1000094, TESTI1000096, TESTI1000109, TESTI1000125, TESTI1000127, TESTI1000128, TESTI1000131, TESTI1000138, TESTI1000157, TESTI1000168, TESTI1000169, TESTI1000179, TESTI1000183, TESTI1000191, TESTI1000257, TESTI1000266, TESTI1000319,
TESTI1000330, TESTI1000348, TESTI1000390, TESTI1000391, TESTI1000459, TESTI1000491, TESTI1000545, TESTI2000018, TESTI2000044, TESTI2000100, TESTI2000117, TESTI2000120, TESTI2000147, TESTI2000154, TESTI2000177, TESTI2000179, TESTI2000207, TESTI2000208, TESTI2000221, TESTI2000238, TESTI2000253, TESTI2000272, TESTI2000278, TESTI2000356, TESTI2000358, TESTI2000372, TESTI2000427, TESTI2000431, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000808,
TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815,
TESTI2001823, TESTI2001826, TESTI2001827, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TEST!2002498, TESTI2002516, TESTI2002544, TESTI2002578, TESTI2002580, TESTI2002618,
TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280,
TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003454, TESTI2003475, TESTI2003476, TESTI2003498, TESTI2003512, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080,
TESTI2004085, TESTI2004089, TESTI2004111, TESTI2004119, TESTI2004122, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004936, TESTI2004941, TESTI2004971,
TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005759, TESTI2005767,
TESTI2005775, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006457, TESTI2006460, TESTI2006465,
TESTI2006483, TESTI2006499, TESTI2006523, TESTI2006543, TESTI2006565, TESTI2006572, TESTI2006615, TESTI2006617, TESTI2006624, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007422, TESTI2007452, TESTI2007464,
TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008320, TESTI2008331, TESTI2008343, TESTI2008387, TESTI2008389, TESTI2008394, TESTI2008405, TESTI2008420, TESTI2008425, TESTI2008440,
TESTI2008567, TESTI2008595, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835,
TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846,
TESTI2011915, TESTI2011928, TESTI2011971, TESTI2012019, TESTI2012050, TESTI2012090, TESTI2012095, TESTI2012104, TESTI2012152, TESTI2012171, TESTI2012207, TESTI2012231, TESTI2012244, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013264, TESTI2013268, TESTI2013295, TESTI2013322, TESTI2013381, TESTI2013382, TESTI2013401, TESTI2013427, TESTI2013468,
TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015105, TESTI2015180, TESTI2015246, TESTI2015249, TESTI2015327, TESTI2015331, TESTI2015335, TESTI2015350,
TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113,
TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228,
TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020515, TESTI2020525, TESTI2020579, TESTI2020717, TESTI2020840, TESTI2020871, TESTI2020905, TESTI2020906, TESTI2020956, TESTI2020981, TESTI2020999,
TESTI2021003, TESTI2021038, TESTI2021057, TESTI2021077, TESTI2021112, TESTI2021114, TESTI2021116, TESTI2021118, TESTI2021122, TESTI2021124, TESTI2021138, TESTI2021358, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025,
TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024864, TESTI2024885, TESTI2024936, TESTI2024978, TESTI2024999, TESTI2025006, TESTI2025013, TESTI2025022, TESTI2025063,
TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605,
TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583,
TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919,
TESTI2031935, TESTI2031986, TESTI2032044, TESTI2032067, TESTI2032079, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183,
TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877,
TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983,
TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044306, TESTI2044309, TESTI2044413, TESTI2044418, TESTI2044754, TESTI2044788,
TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109,
TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667,
TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527, TESTI4001561, TESTI4001569, TESTI4001604, TESTI4001665, TESTI4001679,
TESTI4001795, TESTI4001923, TESTI4001925, TESTI4001984, TESTI4002003, TESTI4002141, TESTI4002195, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399,
TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007,
TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789,
TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013735, TESTI4013742, TESTI4013774, TESTI4013817, TESTI4013830, TESTI4013852, TESTI4013894,
TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229,
TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491,
TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762,
TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270,
TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339,
TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371,
TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000017,
THYMU1000025, THYMU1000029, THYMU1000033, THYMU1000041, THYMU1000060, THYMU1000074, THYMU1000098, THYMU1000103, THYMU1000105, THYMU1000109, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000374, THYMU1000382, THYMU1000393, THYMU1000399, THYMU1000426, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022,
THYMU2000032, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000436, THYMU2000440, THYMU2000489, THYMU2000492, THYMU2000519, THYMU2000570, THYMU2000602, THYMU2000660, THYMU2000671, THYMU2000672, THYMU2000702, THYMU2000767, THYMU2000775, THYMU2000800, THYMU2000879, THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270,
THYMU2001291, THYMU2001314, THYMU2001325, THYMU2001357, THYMU2001381, THYMU2001422, THYMU2001443, THYMU2001515, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002242, THYMU2002338, THYMU2002356, THYMU2002376, THYMU2002384, THYMU2002450, THYMU2002490, THYMU2002548, THYMU2002695, THYMU2002756, THYMU2002789, THYMU2002792, THYMU2002815, THYMU2002852, THYMU2002910, THYMU2002950, THYMU2002983, THYMU2003107, THYMU2003124, THYMU2003166,
THYMU2003232, THYMU2003242, THYMU2003249, THYMU2003270, THYMU2003282, THYMU2003287, THYMU2003336, THYMU2003366, THYMU2003397, THYMU2003440, THYMU2003446, THYMU2003470, THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004111, THYMU2004123, THYMU2004139, THYMU2004169, THYMU2004284, THYMU2004336, THYMU2004356, THYMU2004452, THYMU2004508, THYMU2004512, THYMU2004635, THYMU2004639, THYMU2004668, THYMU2004677, THYMU2004688, THYMU2004693, THYMU2004835, THYMU2004843, THYMU2004906, THYMU2004916, THYMU2004986, THYMU2005001, THYMU2005003, THYMU2005046,
THYMU2005134, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007060, THYMU2007095,
THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714,
THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009835, THYMU2009906, THYMU2009948, THYMU2010030, THYMU2010041, THYMU2010046, THYMU2010059, THYMU2010082, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142,
THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011937, THYMU2011939, THYMU2012002, THYMU2012073, THYMU2012104, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013170, THYMU2013364,
THYMU2013386, THYMU2013426, THYMU2013578, THYMU2013705, THYMU2013757, THYMU2013779, THYMU2013863, THYMU2013950, THYMU2013981, THYMU2014067, THYMU2014068, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015161, THYMU2015316, THYMU2015321, THYMU2015439, THYMU2015441, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099,
THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830,
THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894,
THYMU2027996, THYMU2028041, THYMU2028368, THYMU2028379, THYMU2028394, THYMU2028412, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976,
THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036215, THYMU2036252, THYMU2036265, THYMU2036366, THYMU2036459, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189,
THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841,
THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137,
THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962,
THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024602, THYMU3024879, THYMU3025118, THYMU3025313, THYMU3025683, THYMU3025772, THYMU3025865, THYMU3026000, THYMU3026350, THYMU3026479, THYMU3026532, THYMU3026783, THYMU3026869,
THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032798, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827,
THYMU3037836, THYMU3037856, THYMU3037867, THYMU3037909, THYMU3037980, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175,
THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2002318, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003601, TKIDN2004185, TKIDN2004386, TKIDN2004458, TKIDN2004748, TKIDN2005487, TKIDN2005721, TKIDN2005934,
TKIDN2005947, TKIDN2005956, TKIDN2006525, TKIDN2006761, TKIDN2006852, TKIDN2007605, TKIDN2007667, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616,
TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575,
TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000057, TRACH1000074, TRACH1000100, TRACH1000125, TRACH1000140, TRACH1000144, TRACH1000181, TRACH1000193, TRACH1000212, TRACH1000240, TRACH2000002, TRACH2000015, TRACH2000024, TRACH2000032, TRACH2000045, TRACH2000047, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000461, TRACH2000496, TRACH2000497, TRACH2000502,
TRACH2000522, TRACH2000540, TRACH2000559, TRACH2000660, TRACH2000665, TRACH2000675, TRACH2000677, TRACH2000703, TRACH2000767, TRACH2000775, TRACH2000780, TRACH2000807, TRACH2000862, TRACH2000873, TRACH2000894, TRACH2000898, TRACH2000920, TRACH2000923, TRACH2000926, TRACH2000944, TRACH2000981, TRACH2000986, TRACH2001037, TRACH2001060, TRACH2001085, TRACH2001109, TRACH2001121, TRACH2001147, TRACH2001154, TRACH2001188, TRACH2001192, TRACH2001249, TRACH2001275, TRACH2001335, TRACH2001339, TRACH2001341, TRACH2001347, TRACH2001395, TRACH2001400, TRACH2001403, TRACH2001416, TRACH2001428, TRACH2001432, TRACH2001474, TRACH2001525, TRACH2001532, TRACH2001549, TRACH2001592, TRACH2001596, TRACH2001621,
TRACH2001646, TRACH2001651, TRACH2001684, TRACH2001810, TRACH2001933, TRACH2001968, TRACH2001996, TRACH2002043, TRACH2002054, TRACH2002100, TRACH2002138, TRACH2002333, TRACH2002784, TRACH2002803, TRACH2002840, TRACH2002853, TRACH2002862, TRACH2002954, TRACH2002960, TRACH2002988, TRACH2003070, TRACH2003272, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004521, TRACH2004570, TRACH2004845, TRACH2004887, TRACH2004950, TRACH2005004, TRACH2005017, TRACH2005066, TRACH2005159,
TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007894, TRACH2007969, TRACH2007988, TRACH2008081, TRACH2008112, TRACH2008113, TRACH2008127, TRACH2008130, TRACH2008278, TRACH2008300, TRACH2008478, TRACH2008540, TRACH2008583, TRACH2008597, TRACH2008716, TRACH2008820, TRACH2008921,
TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011302, TRACH2011574, TRACH2011894, TRACH2011956, TRACH2012000, TRACH2012138, TRACH2012242, TRACH2012298, TRACH2012370, TRACH2012376, TRACH2012387,
TRACH2012497, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2014018, TRACH2014045, TRACH2014046, TRACH2014077, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015987,
TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017498, TRACH2017609, TRACH2017949, TRACH2017965, TRACH2017980, TRACH2018034, TRACH2018084, TRACH2018262, TRACH2018273, TRACH2018278, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425,
TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535,
TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003586, TRACH3003683, TRACH3003780,
TRACH3003805, TRACH3003832, TRACH3003872, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699,
TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008688, TRACH3008697, TRACH3008713, TRACH3008887, TRACH3008902, TRACH3008990, TRACH3009001, TRACH3009008, TRACH3009059, TRACH3009061, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313,
TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992,
TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758,
TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451,
TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000235, TSTOM2000442, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000916, TUTER2001286, TUTER2001341, TUTER2001387,
TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN2000046, UMVEN2000069, UMVEN2000121, UMVEN2000133, UMVEN2000152, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU2000023, UTERU2000047, UTERU2000074, UTERU2000099, UTERU2000154, UTERU2000197, UTERU2000218, UTERU2000238, UTERU2000243, UTERU2000260,
UTERU2000329, UTERU2000332, UTERU2000349, UTERU2000377, UTERU2000393, UTERU2000418, UTERU2000465, UTERU2000485, UTERU2000517, UTERU2000524, UTERU2000539, UTERU2000541, UTERU2000546, UTERU2000550, UTERU2000569, UTERU2000607, UTERU2000629, UTERU2000649, UTERU2000663, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001747, UTERU2001876, UTERU2002011, UTERU2002176, UTERU2002198, UTERU2002294, UTERU2002332, UTERU2002473, UTERU2002547, UTERU2002662, UTERU2002693, UTERU2002733, UTERU2002736, UTERU2002737, UTERU2002826, UTERU2002841, UTERU2002964, UTERU2002993,
UTERU2003035, UTERU2003057, UTERU2003126, UTERU2003135, UTERU2003321, UTERU2003399, UTERU2003411, UTERU2003456, UTERU2003577, UTERU2003973, UTERU2004015, UTERU2004039, UTERU2004061, UTERU2004073, UTERU2004163, UTERU2004299, UTERU2004461, UTERU2004520, UTERU2004564, UTERU2004664, UTERU2004688, UTERU2004698, UTERU2004861, UTERU2005004, UTERU2005050, UTERU2005069, UTERU2005074, UTERU2005179, UTERU2005292, UTERU2005346, UTERU2005446, UTERU2005449, UTERU2005450, UTERU2005533, UTERU2005593, UTERU2005601, UTERU2005621, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547,
UTERU2006568, UTERU2006643, UTERU2006651, UTERU2006705, UTERU2006899, UTERU2007004, UTERU2007075, UTERU2007128, UTERU2007253, UTERU2007267, UTERU2007444, UTERU2007499, UTERU2007639, UTERU2007724, UTERU2007924, UTERU2007942, UTERU2008018, UTERU2008019, UTERU2008027, UTERU2008077, UTERU20D8130, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951,
UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012890, UTERU2012938, UTERU2012976, UTERU2013048, UTERU2013078,
UTERU2013231, UTERU2013262, UTERU2013280, UTERU2013322, UTERU2013483, UTERU2013491, UTERU2013586, UTERU2013926, UTERU2013976, UTERU2014001, UTERU2014024, UTERU2014223, UTERU2014398, UTERU2014464, UTERU2014548, UTERU2014601, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757, UTERU2016761, UTERU2016799, UTERU2016896, UTERU2016902, UTERU2016981, UTERU2017123, UTERU2017303, UTERU2017421, UTERU2017623, UTERU2017632, UTERU2017761, UTERU2017762,
UTERU2017810, UTERU2017988, UTERU2018127, UTERU2018180, UTERU2018200, UTERU2018333, UTERU2018364, UTERU2018514, UTERU2018522, UTERU2018544, UTERU2018566, UTERU2018609, UTERU2018674, UTERU2018712, UTERU2018784, UTERU2018789, UTERU2018811, UTERU2018867, UTERU2018881, UTERU2018955, UTERU2019038, UTERU2019096, UTERU2019163, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023550, UTERU2023651,
UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279,
UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844,
UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422,
UTERU3005460, UTERU3005536, UTERU3005585, UTERU3005767, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010892, UTERU3010919, UTERU3011063, UTERU3011092, UTERU3011273, UTERU3011398, UTERU3011558, UTERU3011579, UTERU3011795, UTERU3011837, UTERU3012293,
UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018711, UTERU3019078

Of the 17,176 genes that include the cDNAs of the present invention, genes whose expression levels were high enough to detect in each of the SALGL, SKMUS, SKNMC, SKNSH, SMINT, SPLEN, and STOMA libraries were analyzed for expression frequency. The analysis results are described below. Each is a relative value, where the expression level over all tissues is taken as 100. The larger this value, the greater the level of expression.

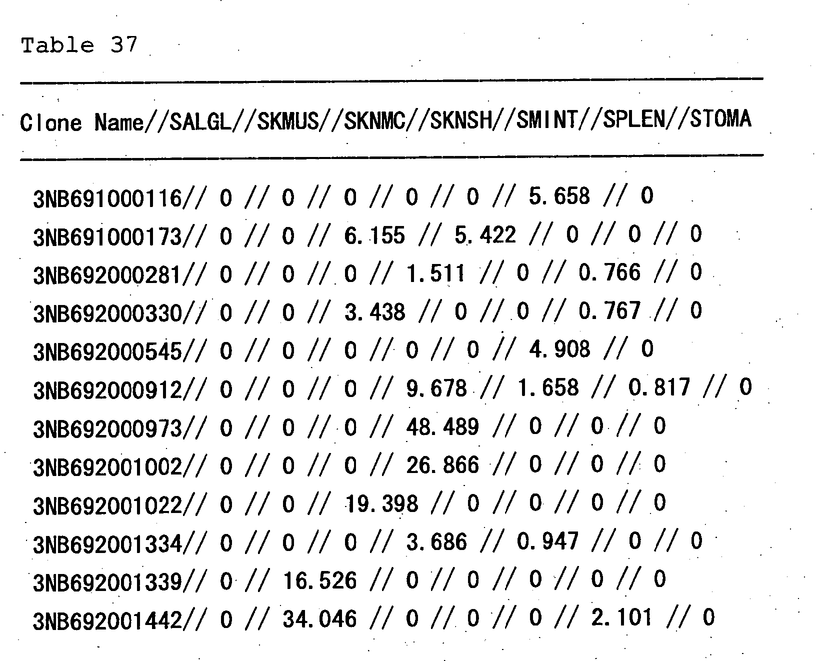

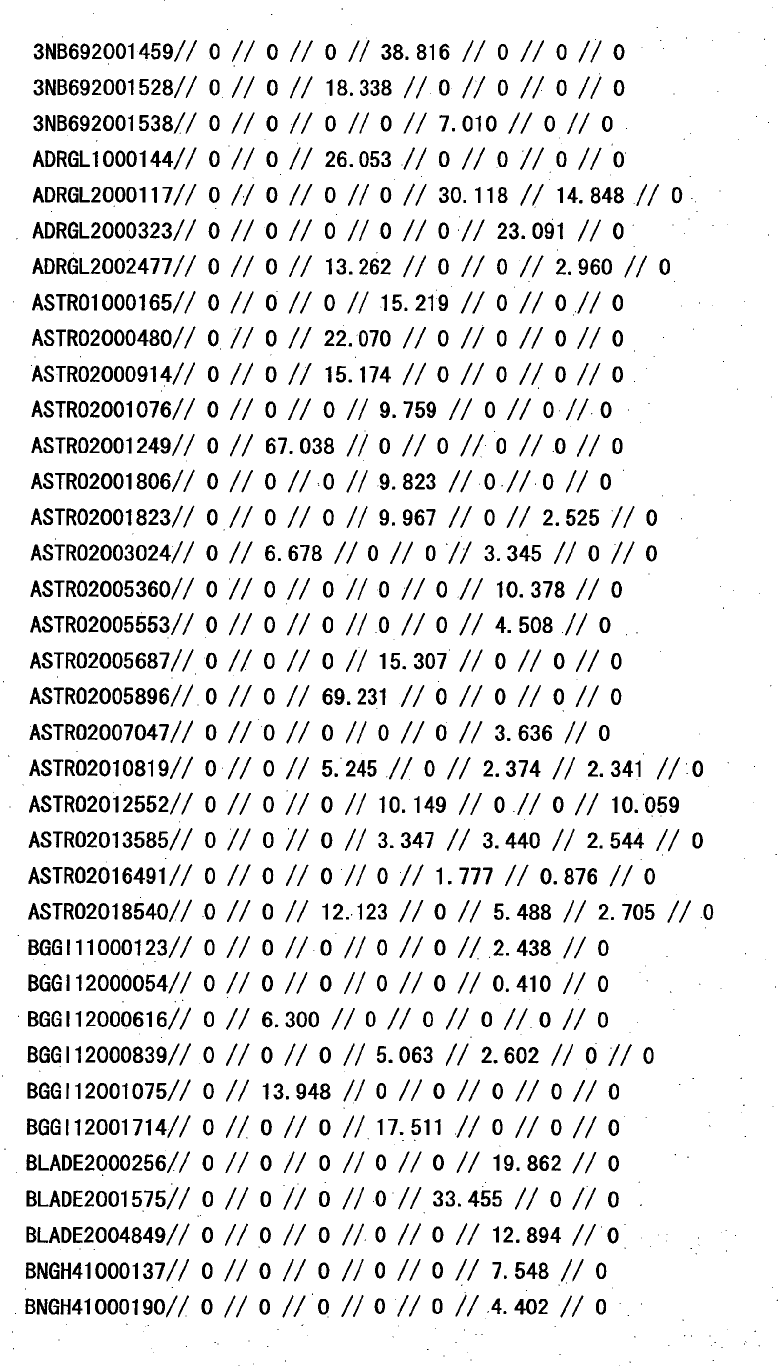

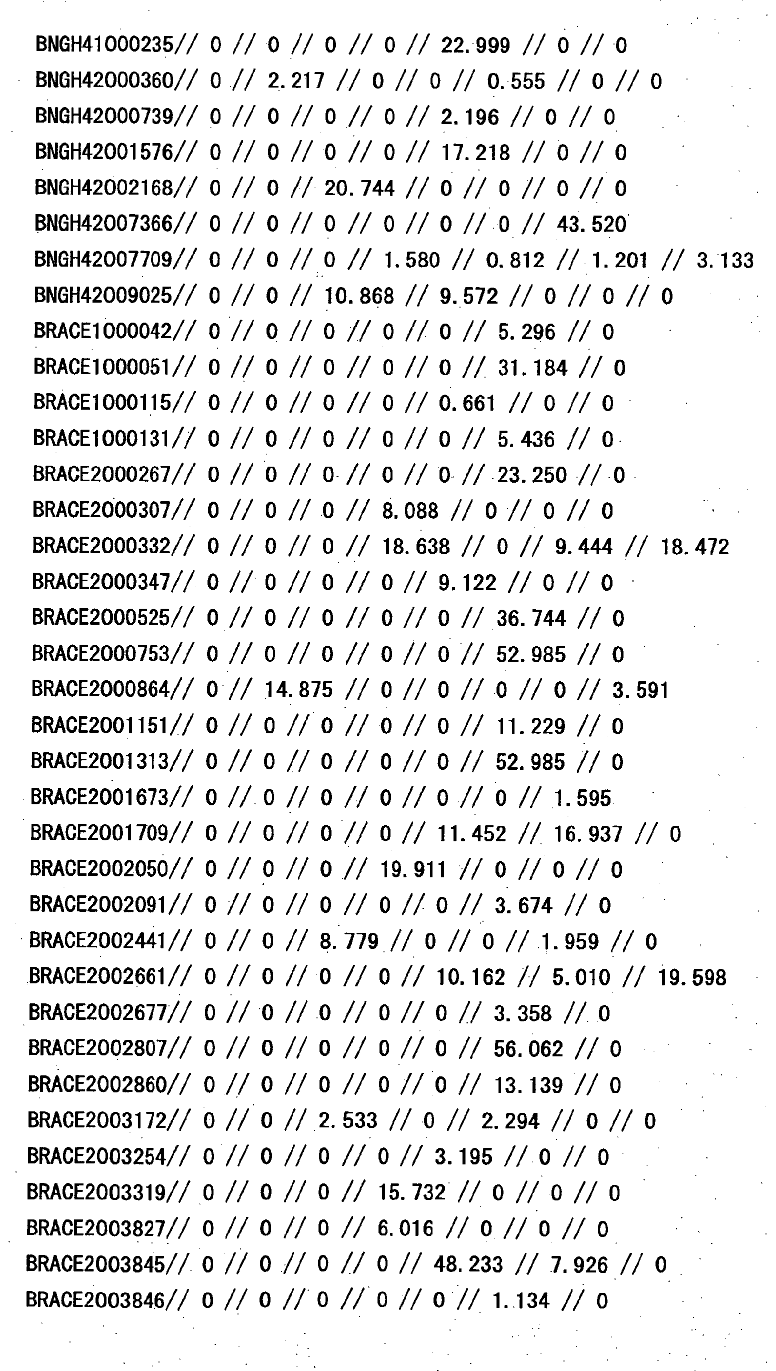

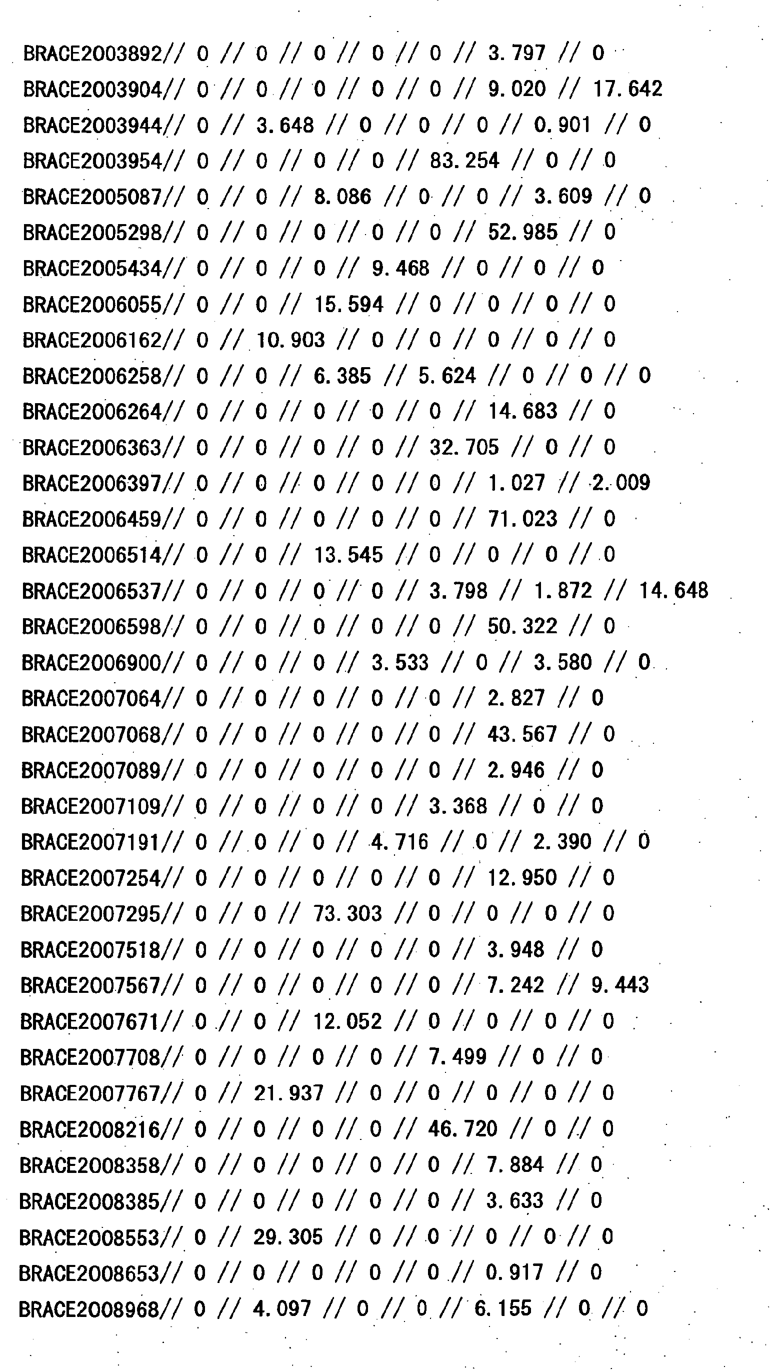

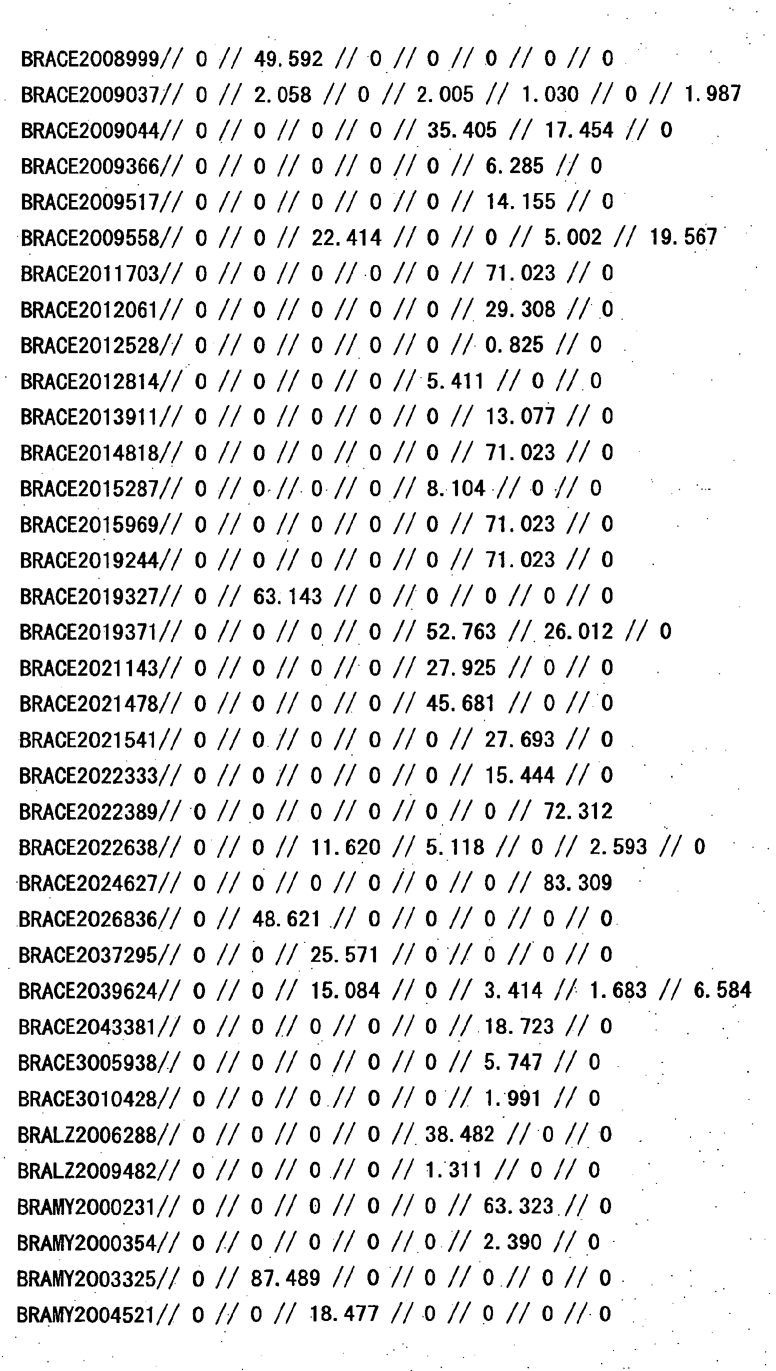

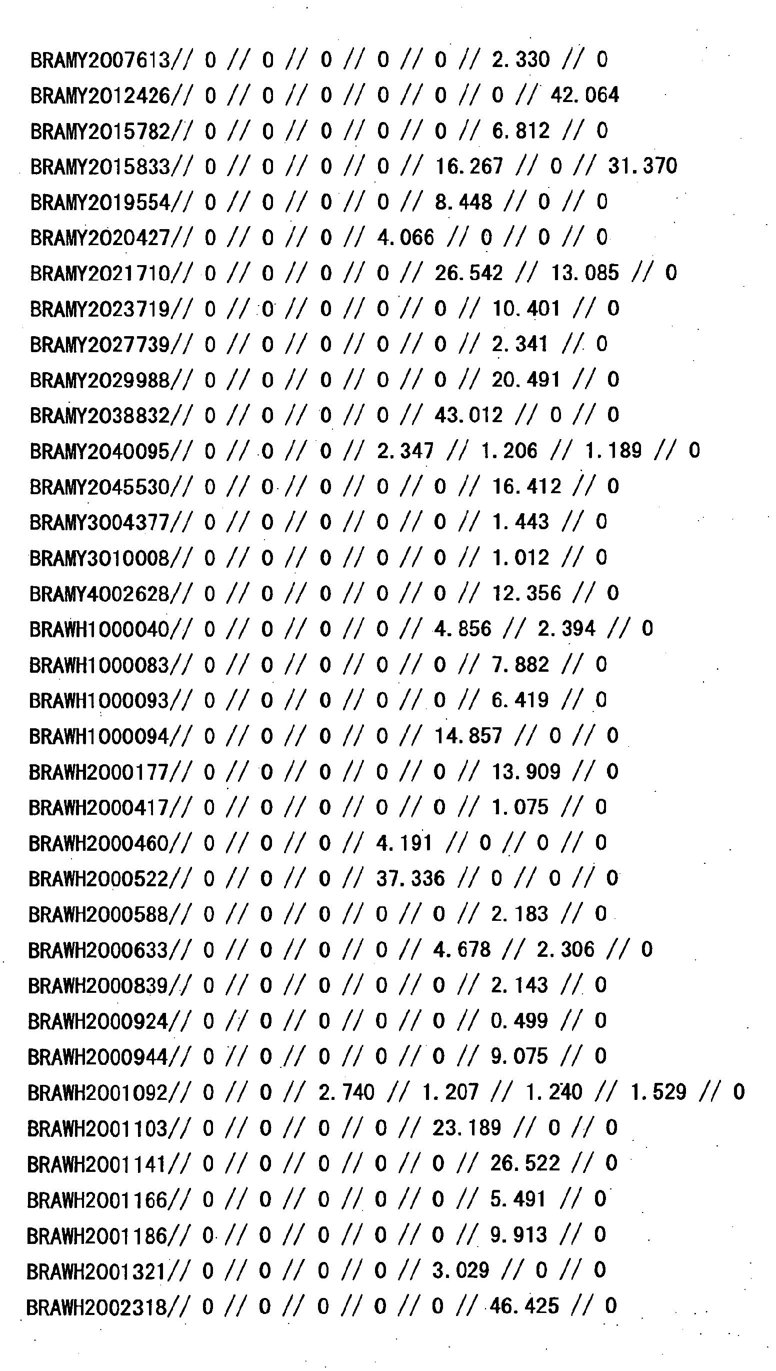

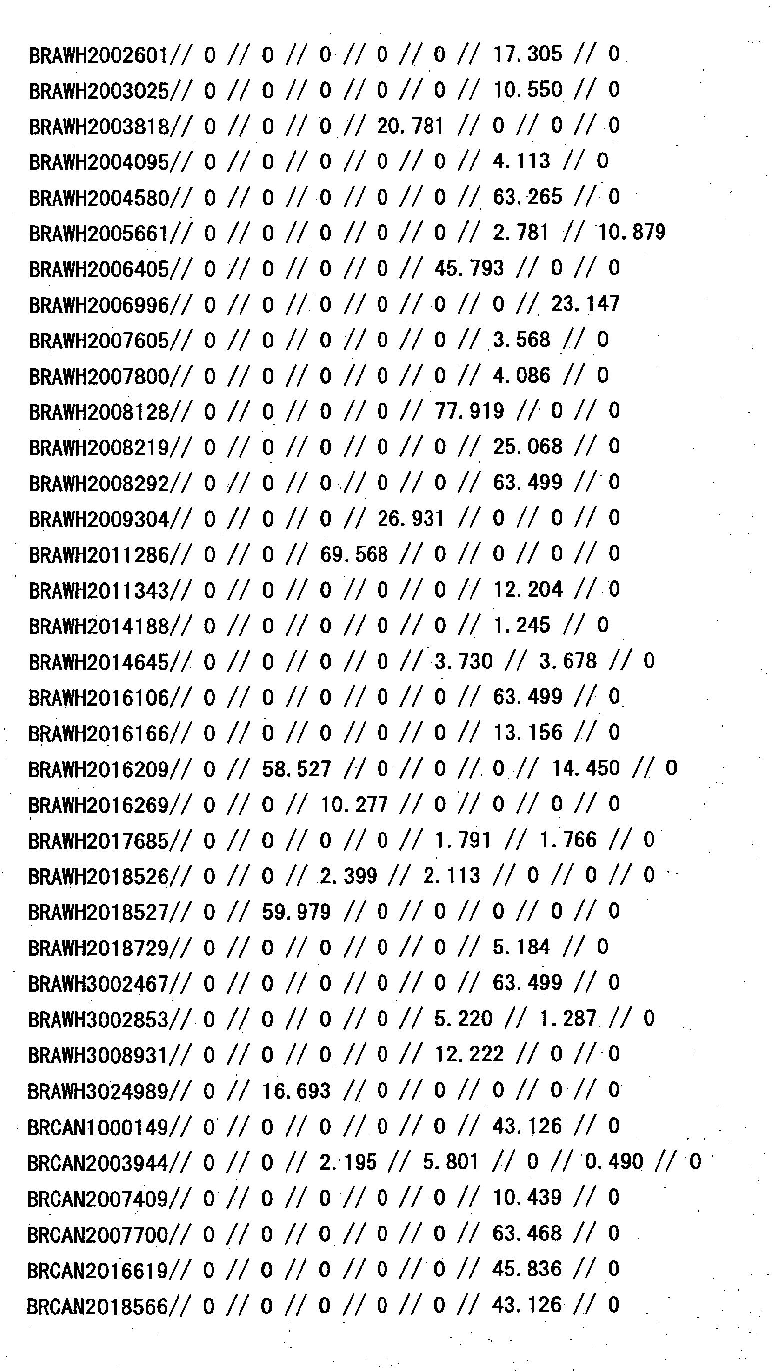

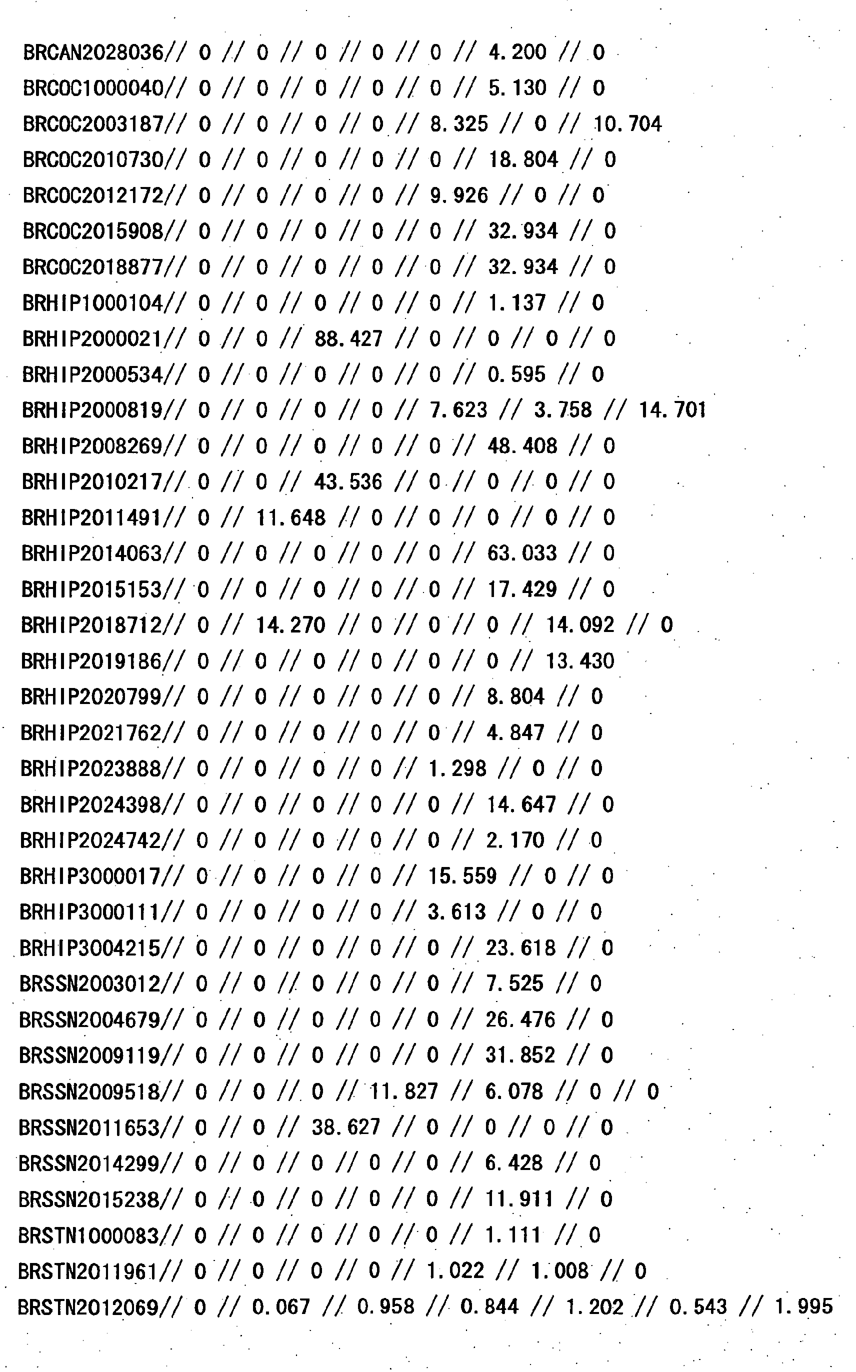

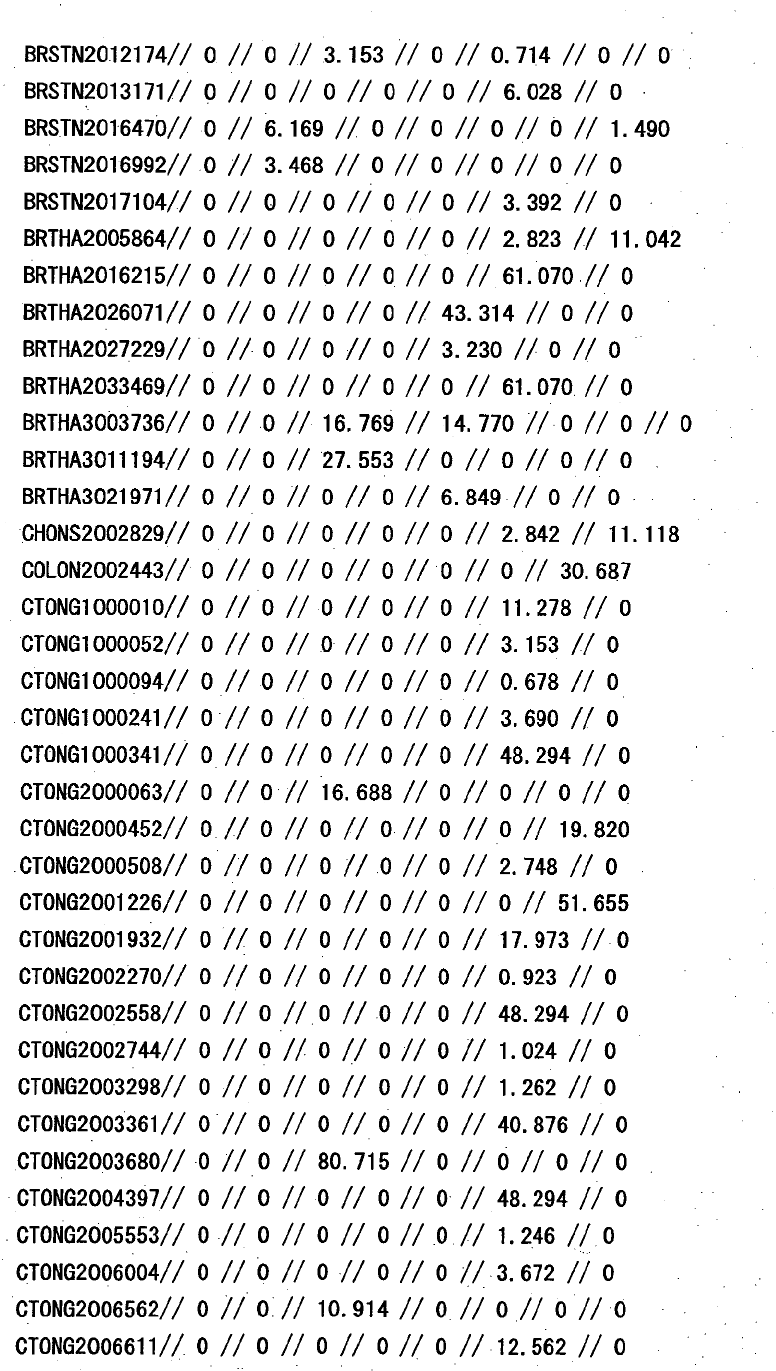

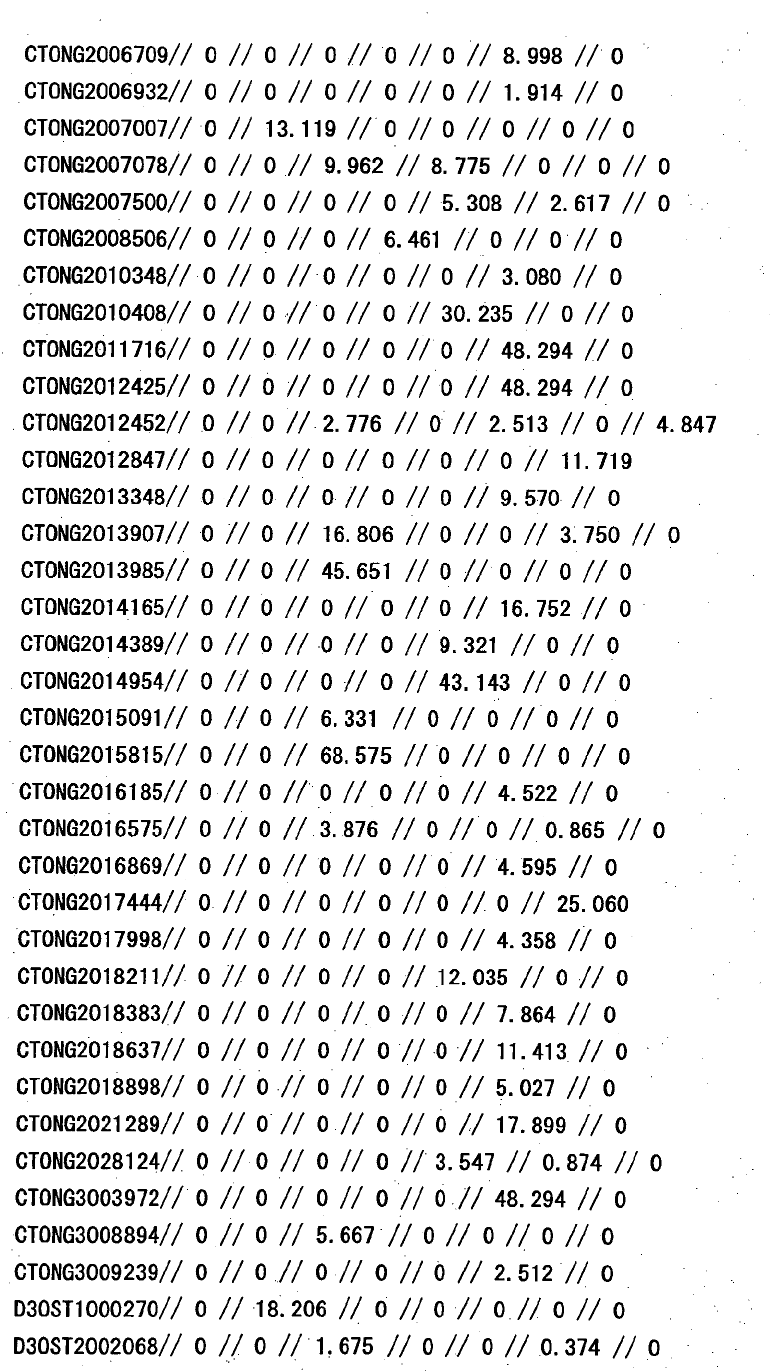

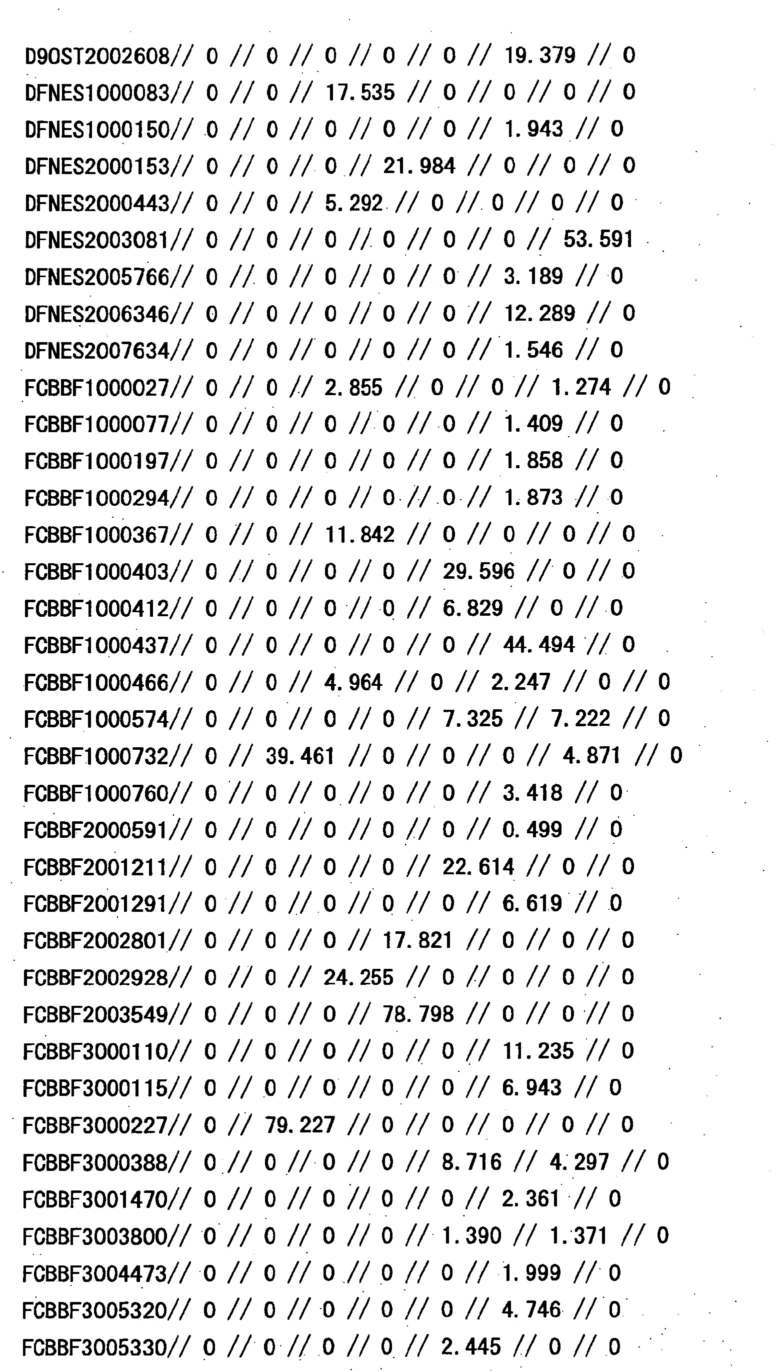

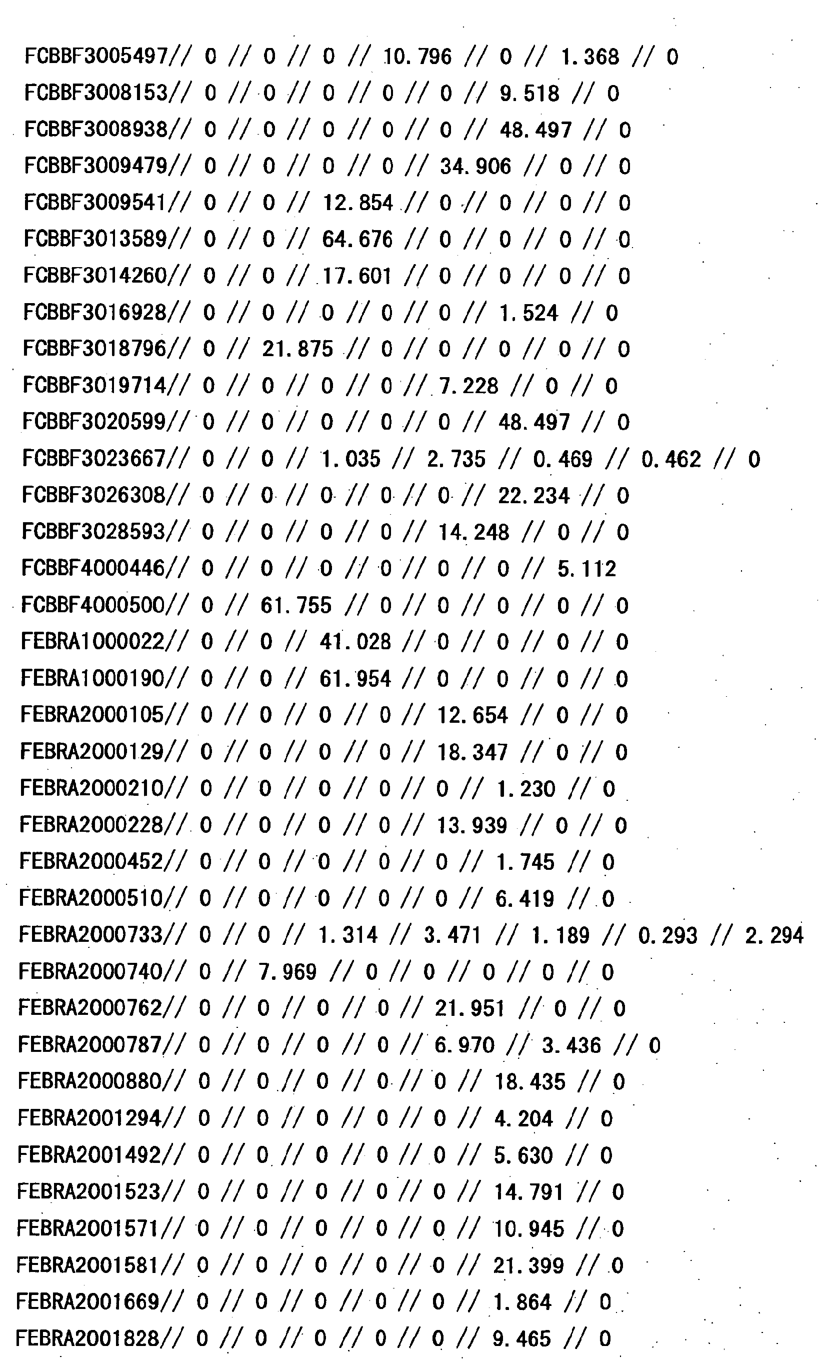

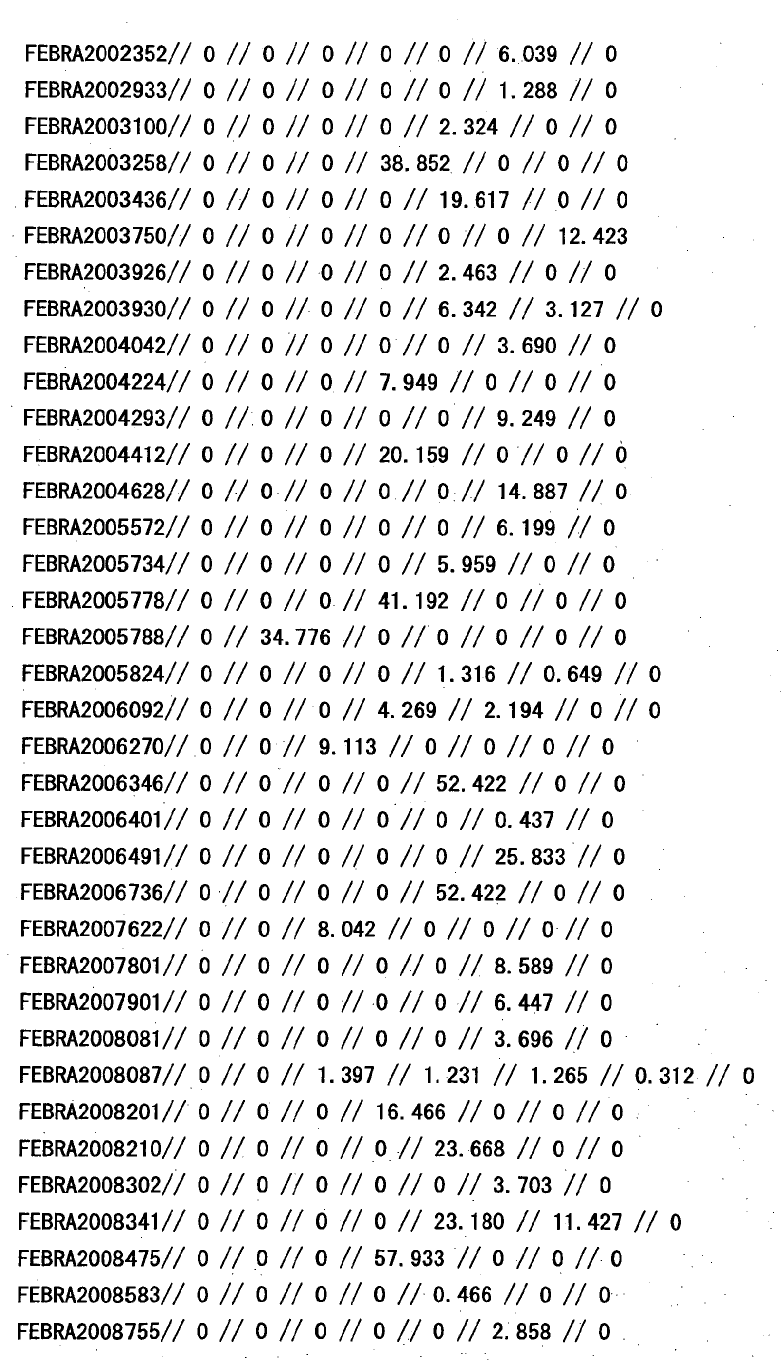

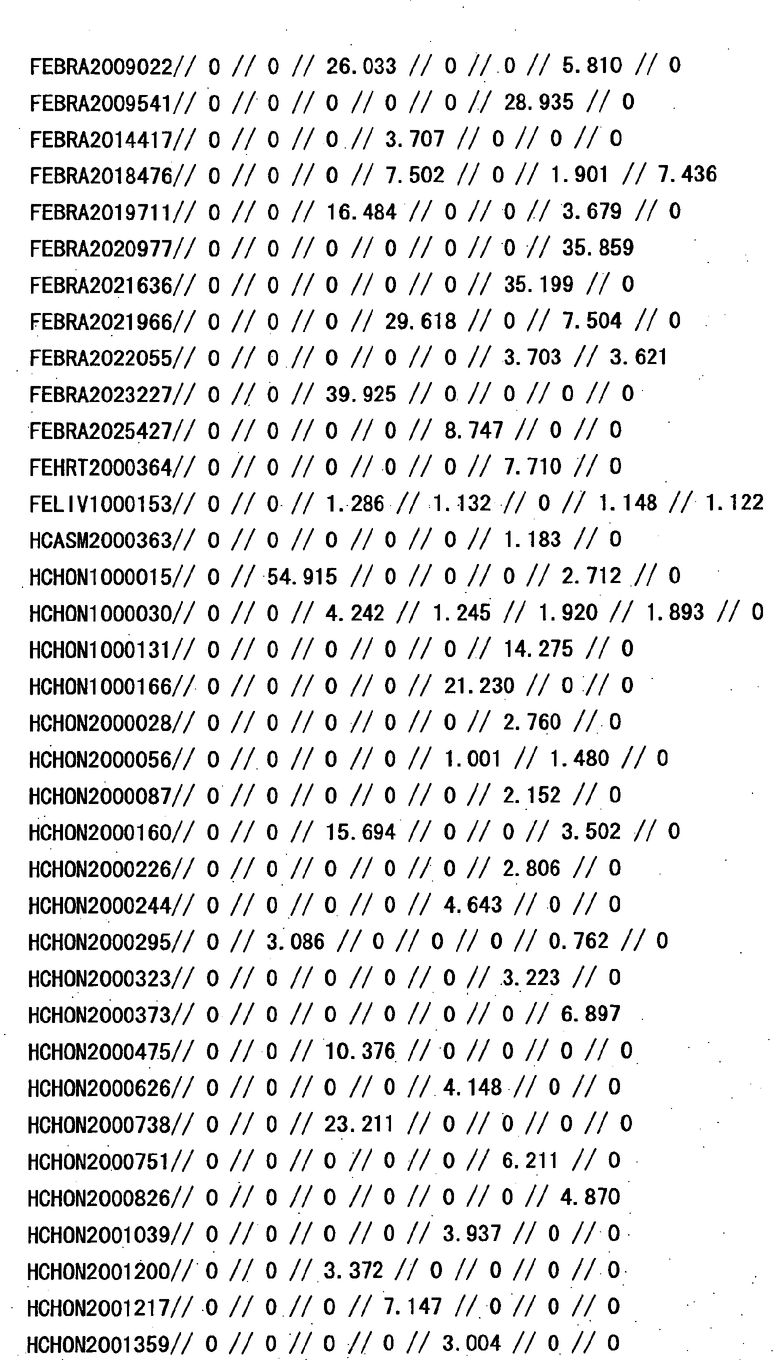

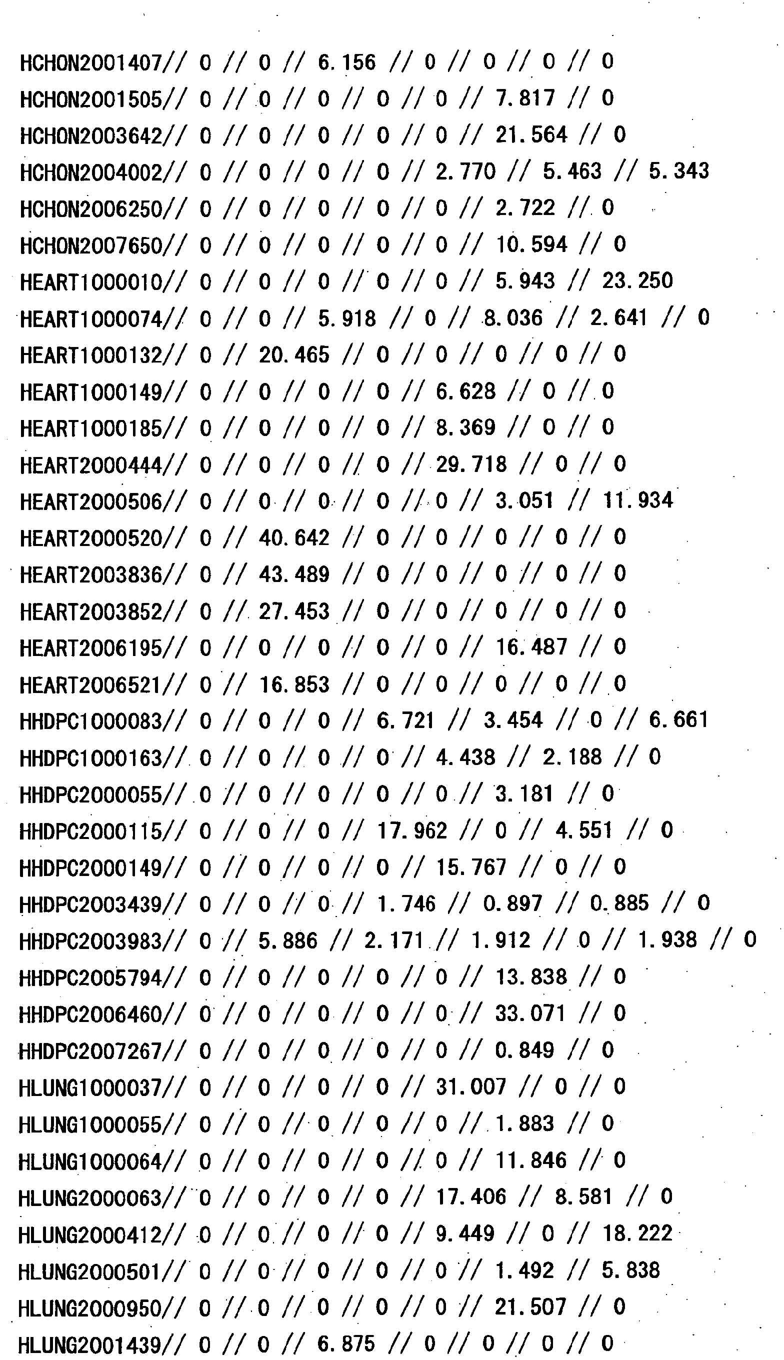

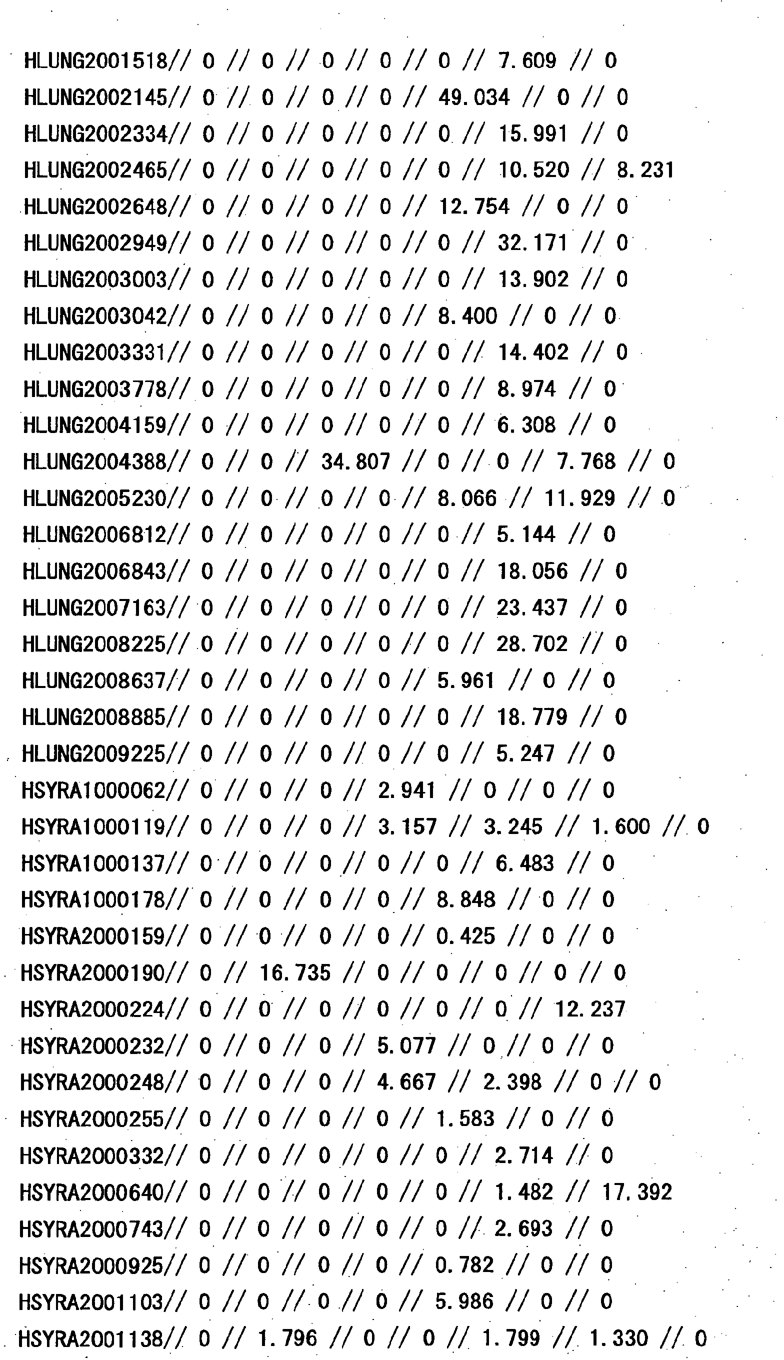

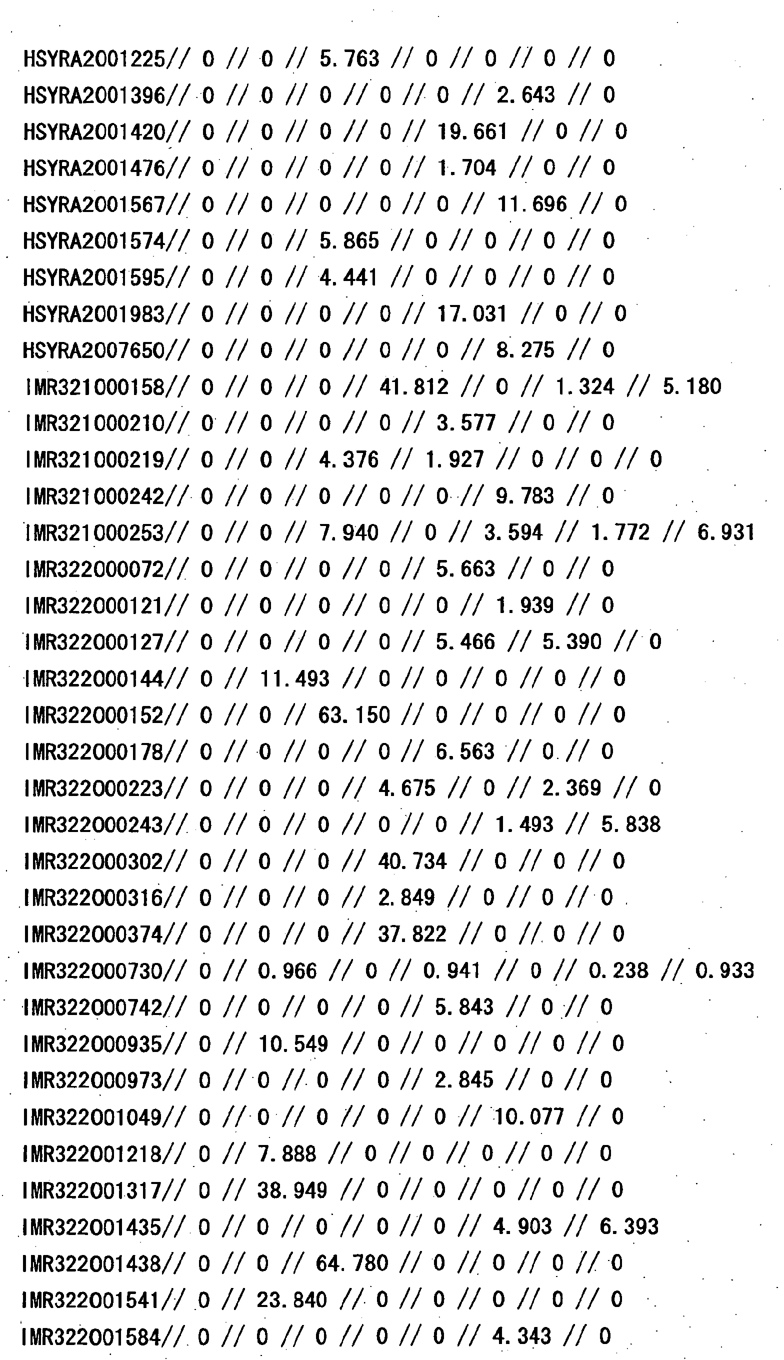

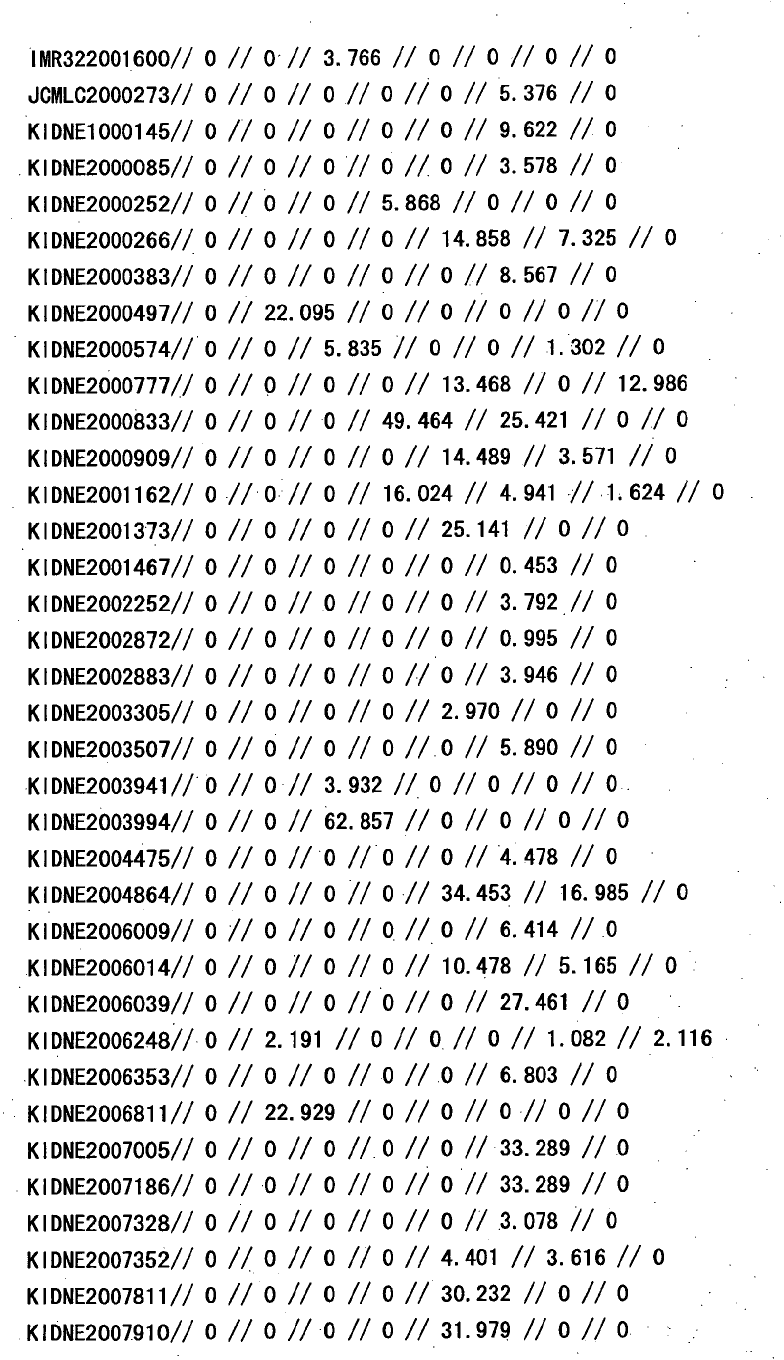

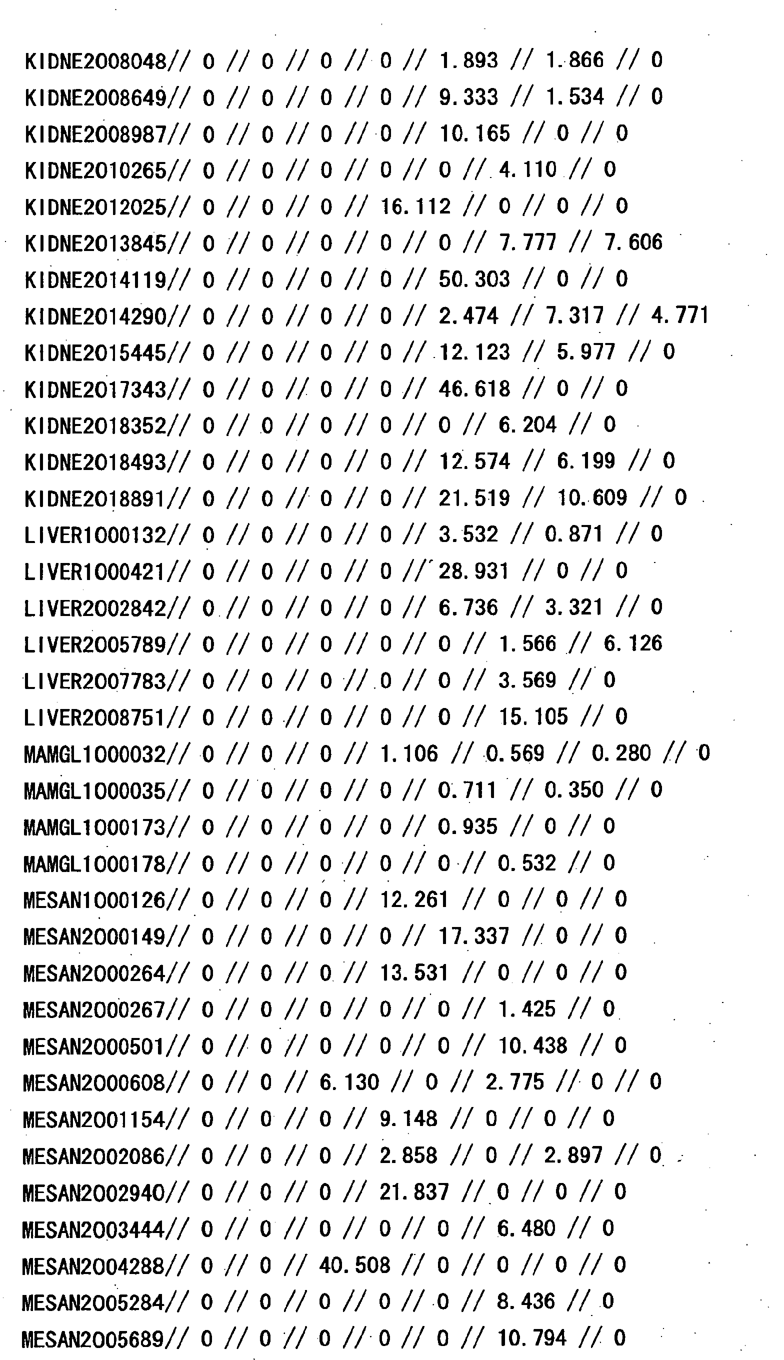

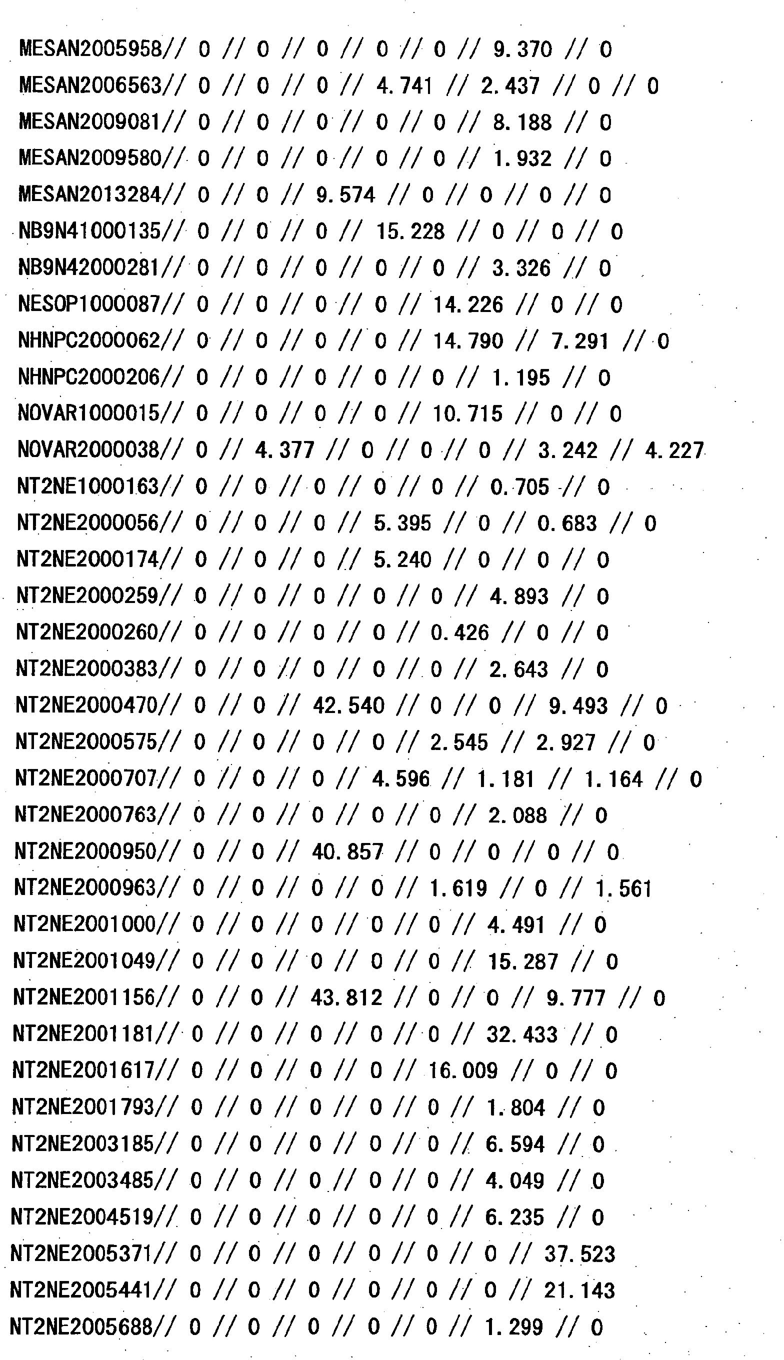

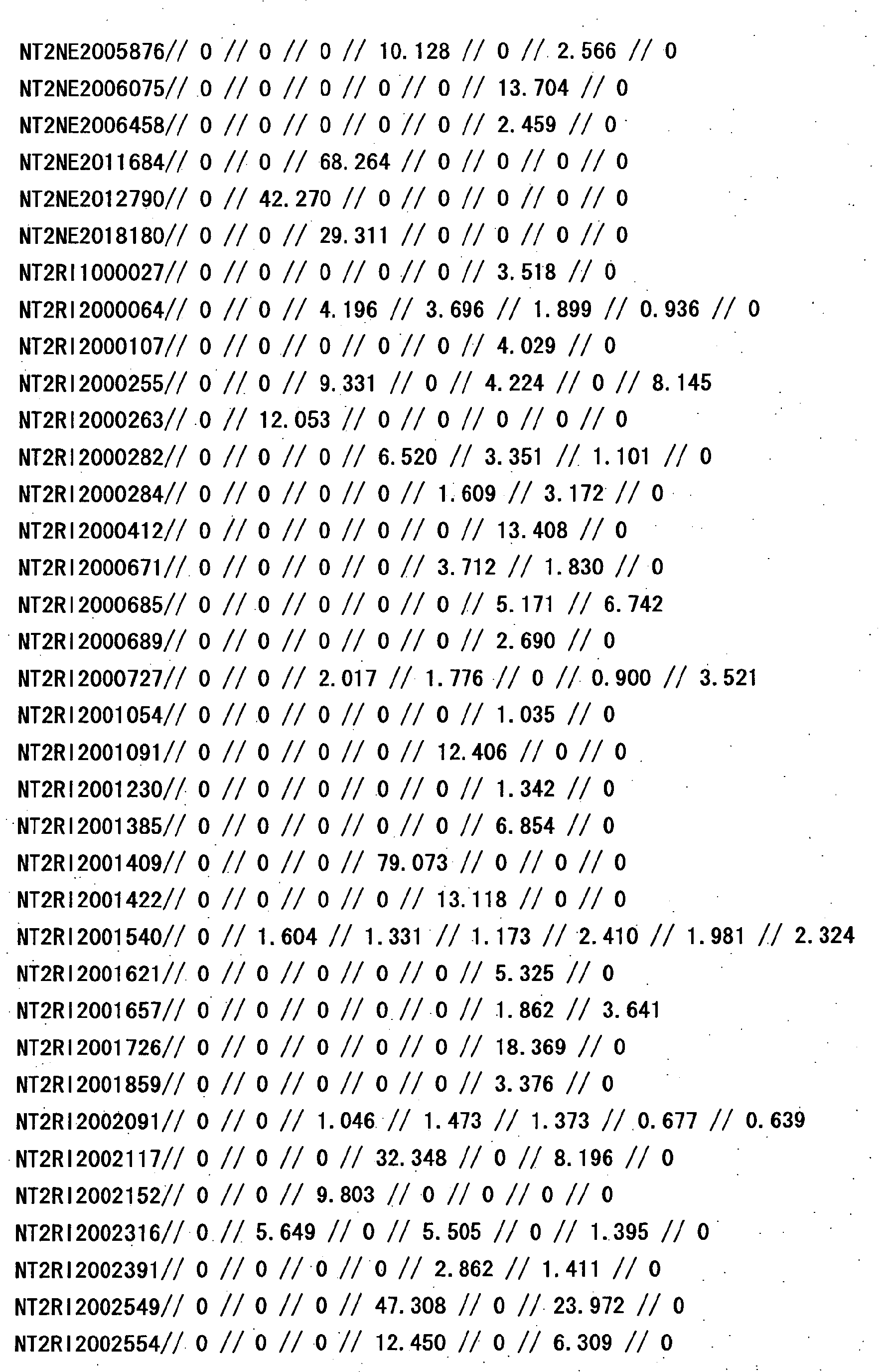

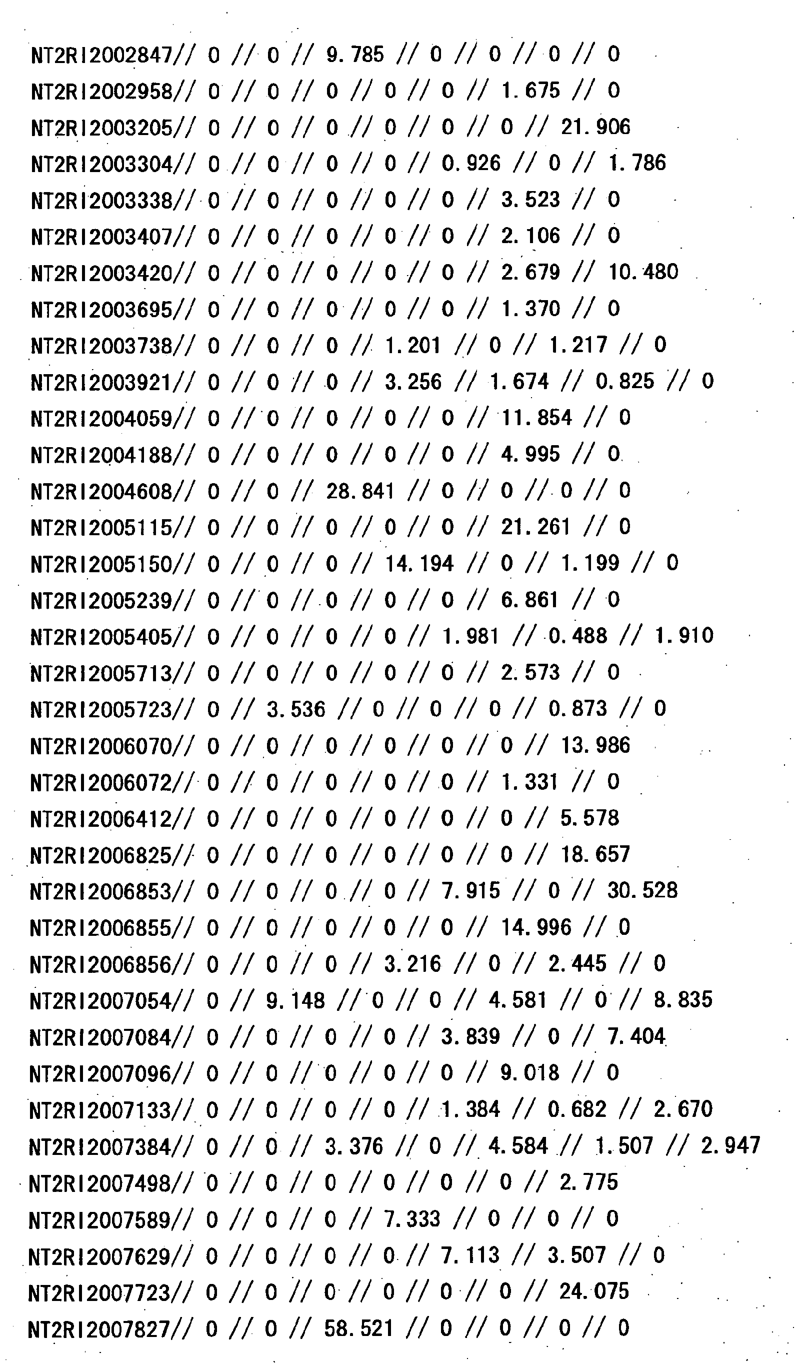

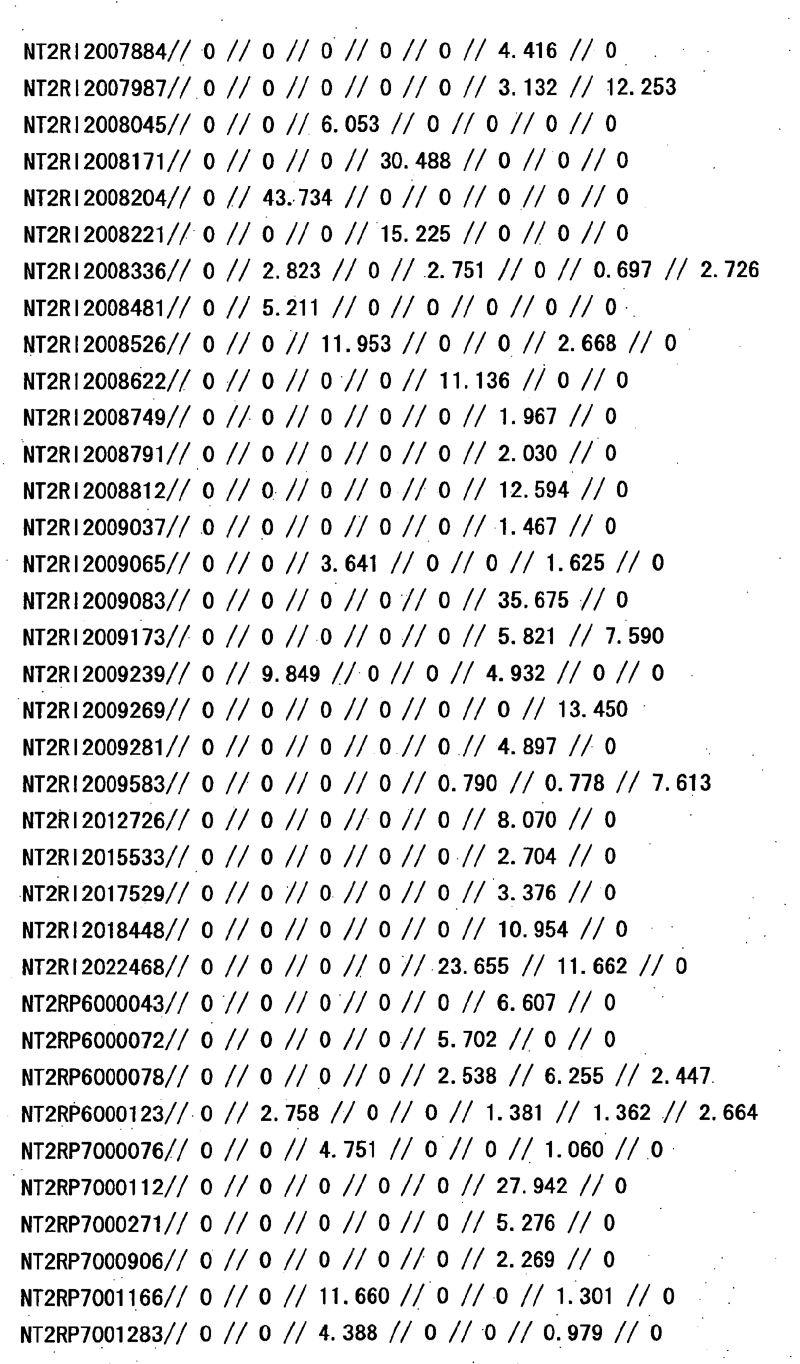

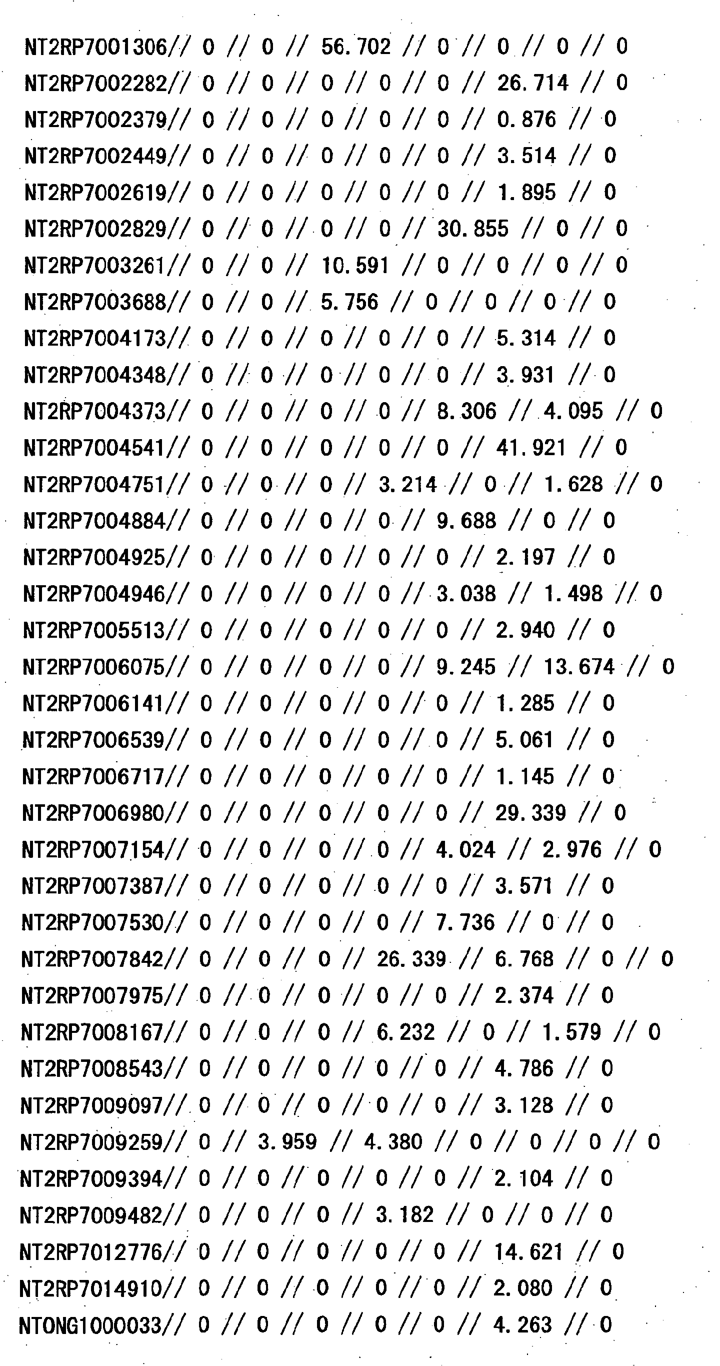

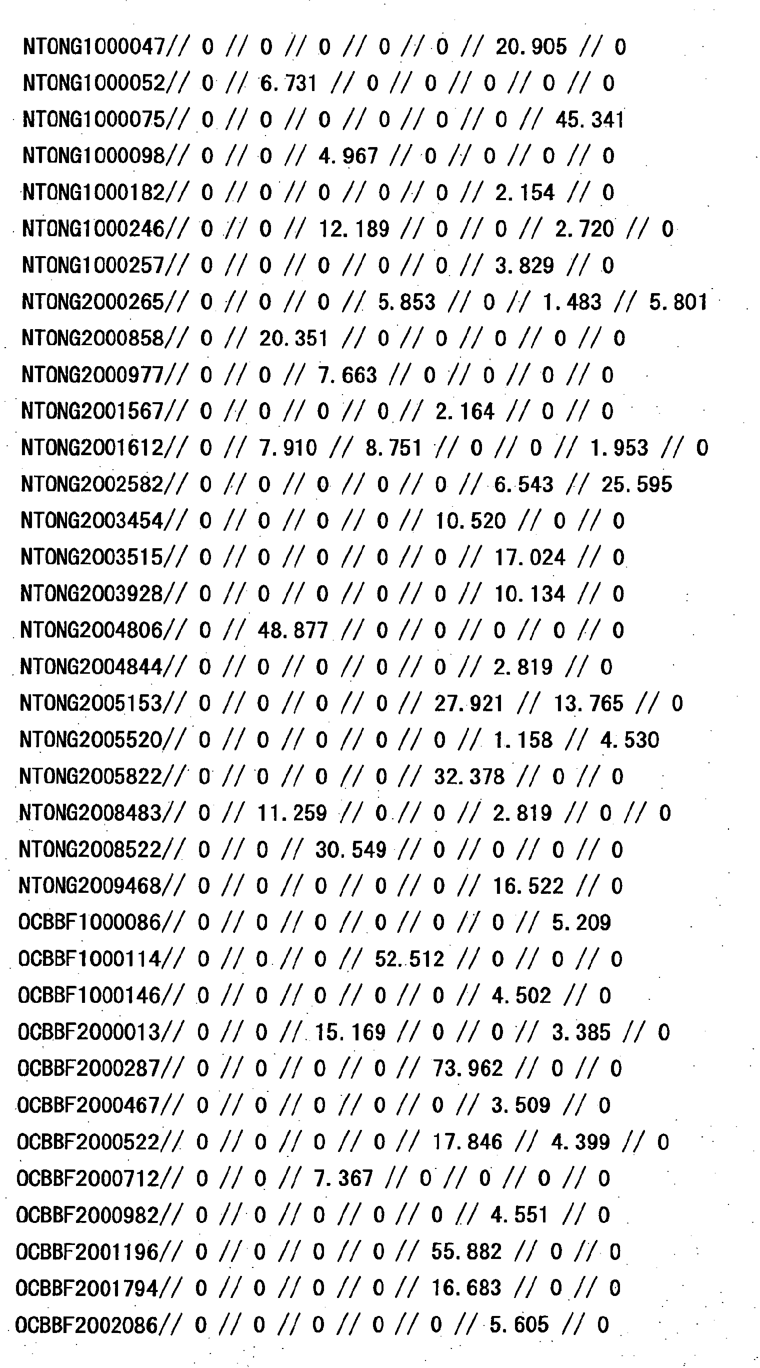

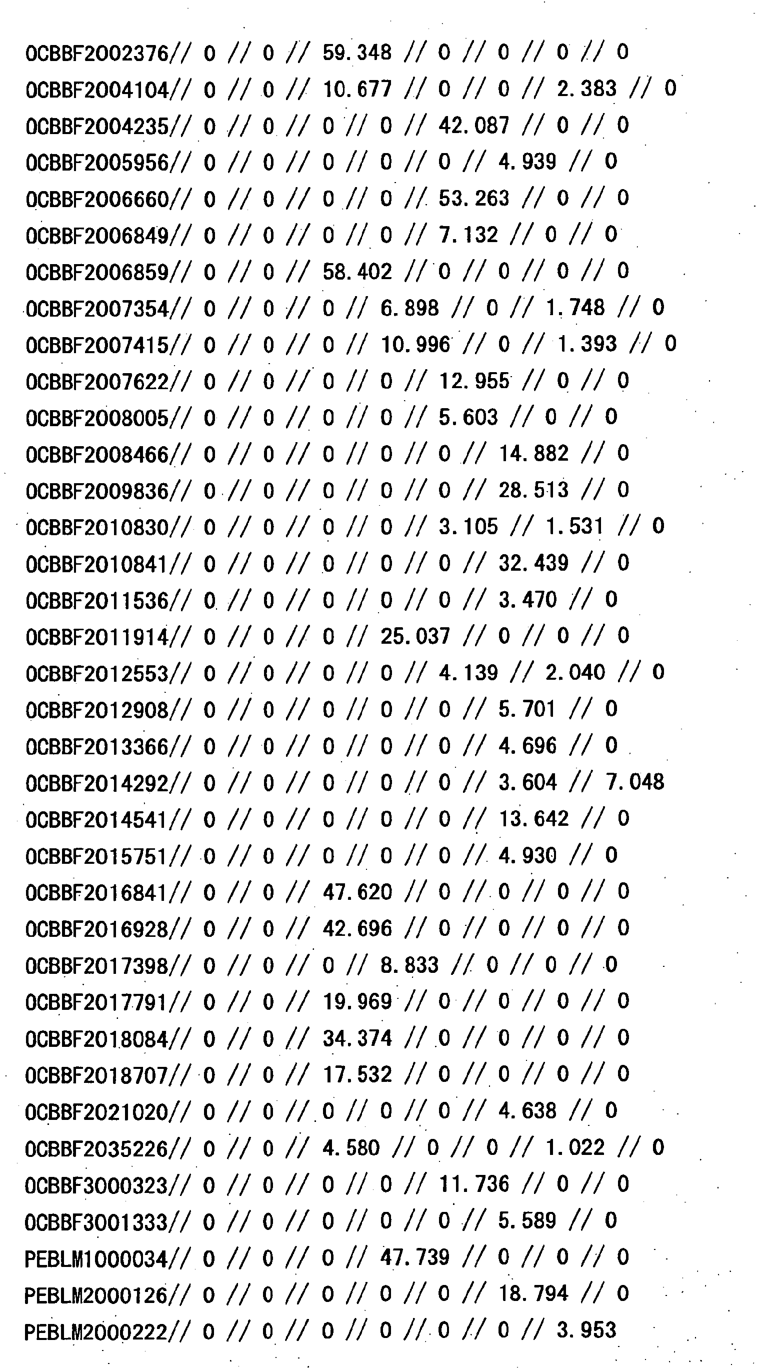

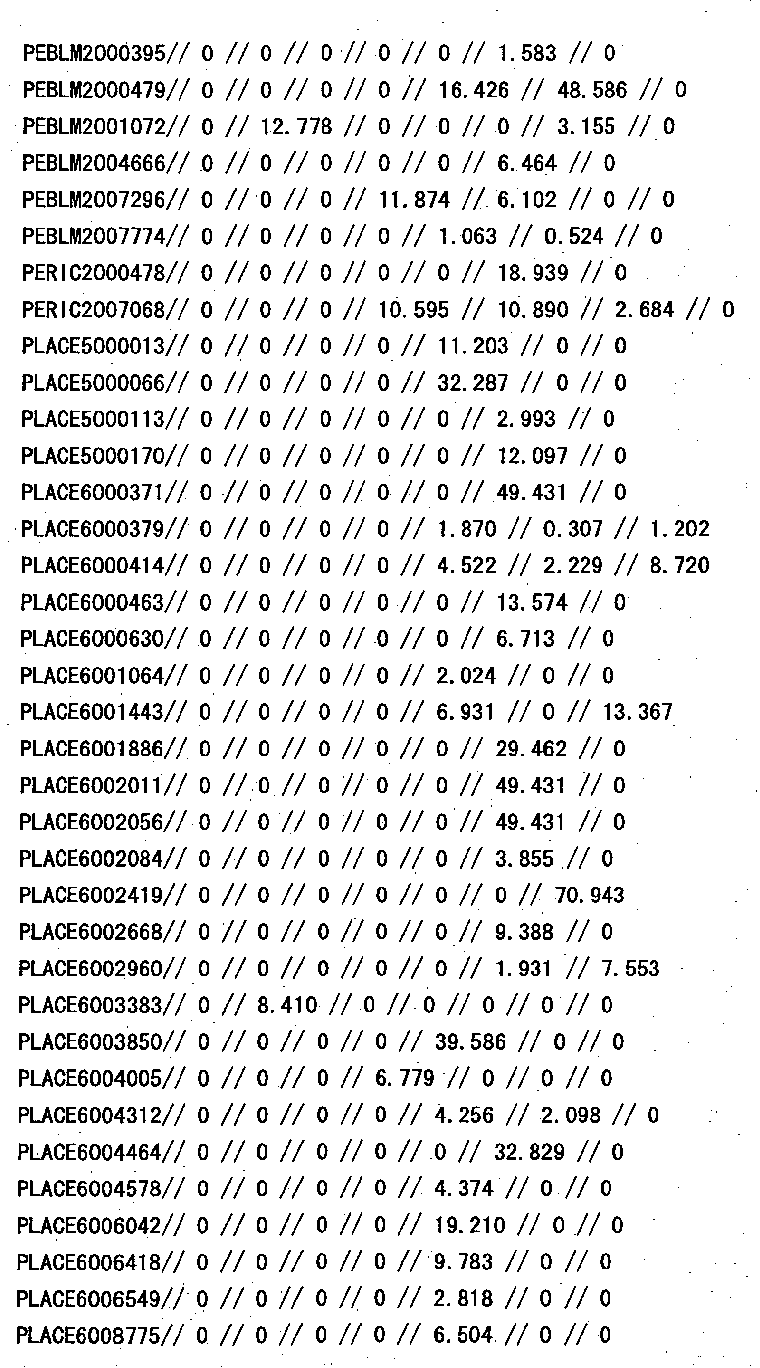

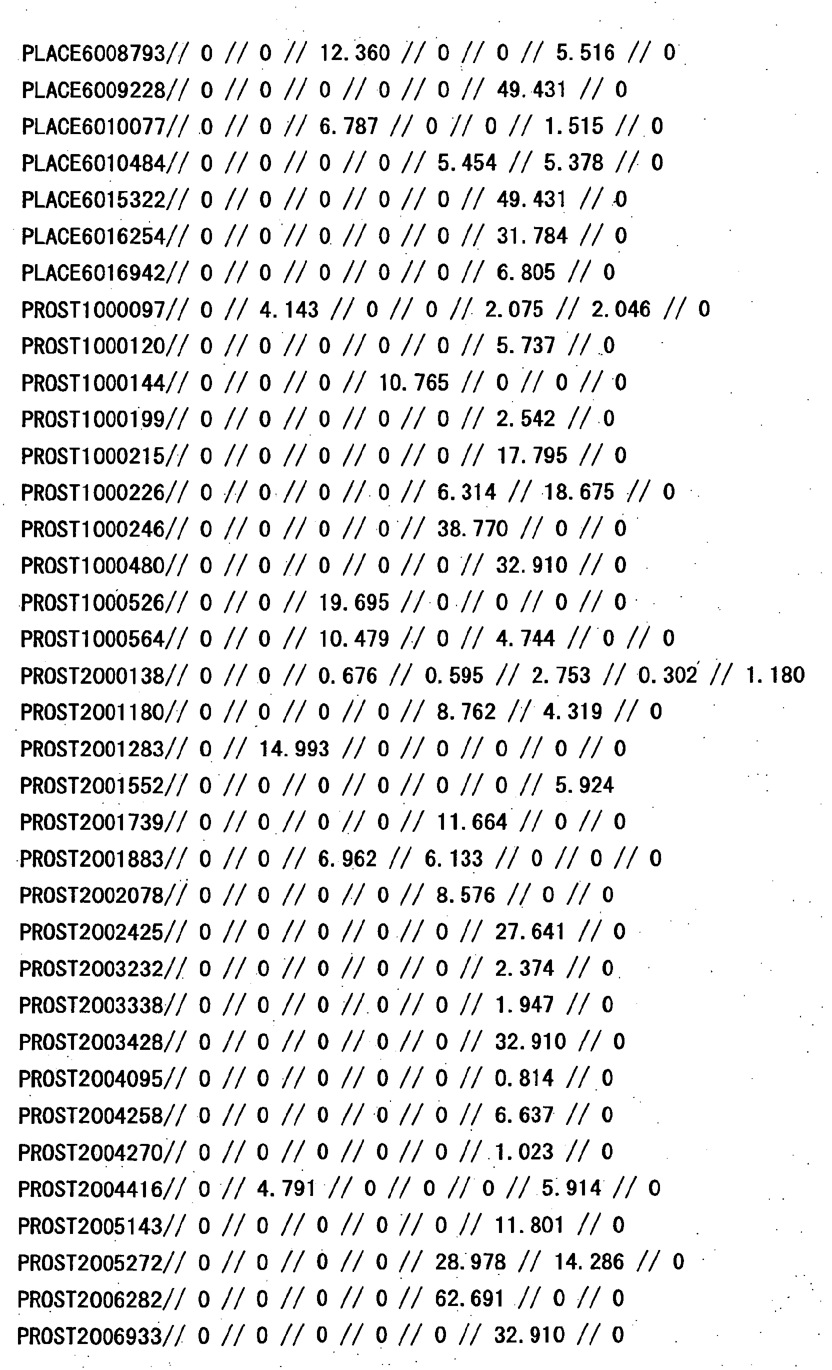

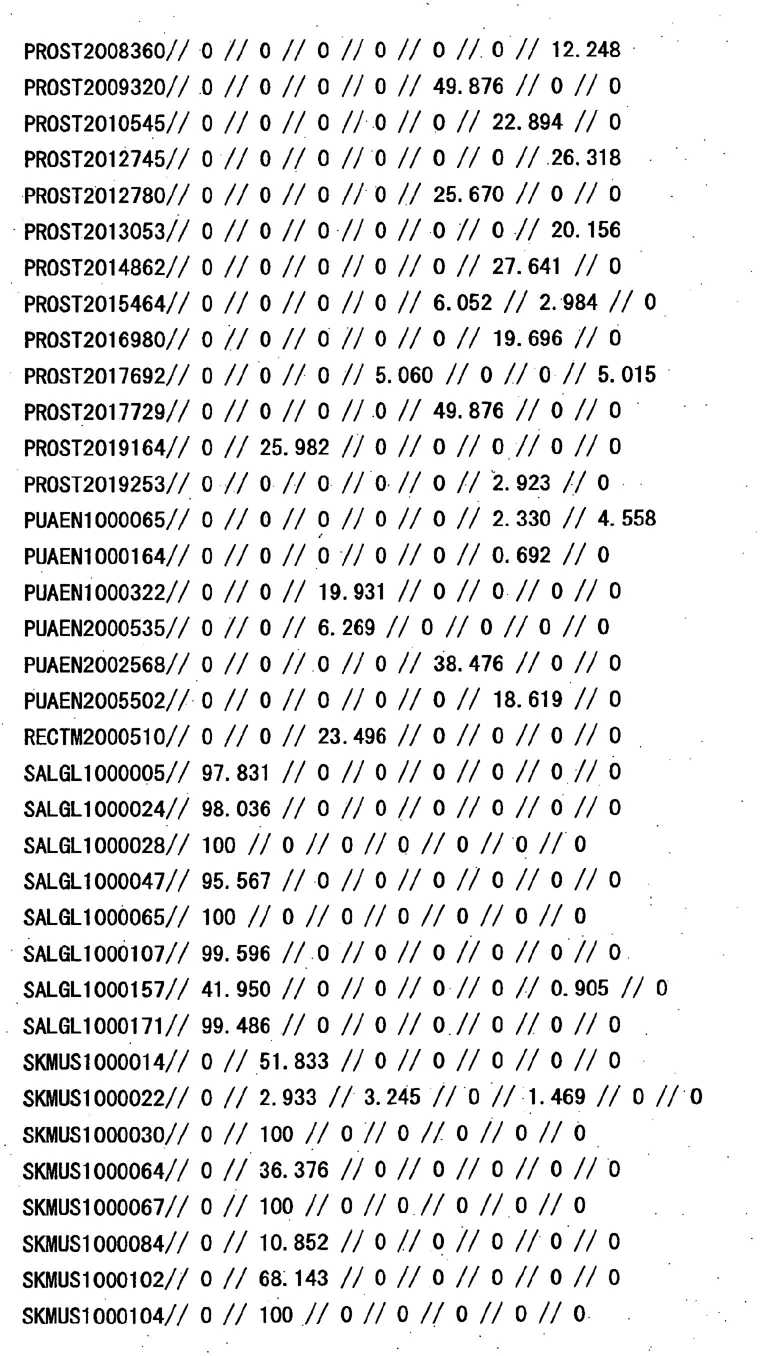

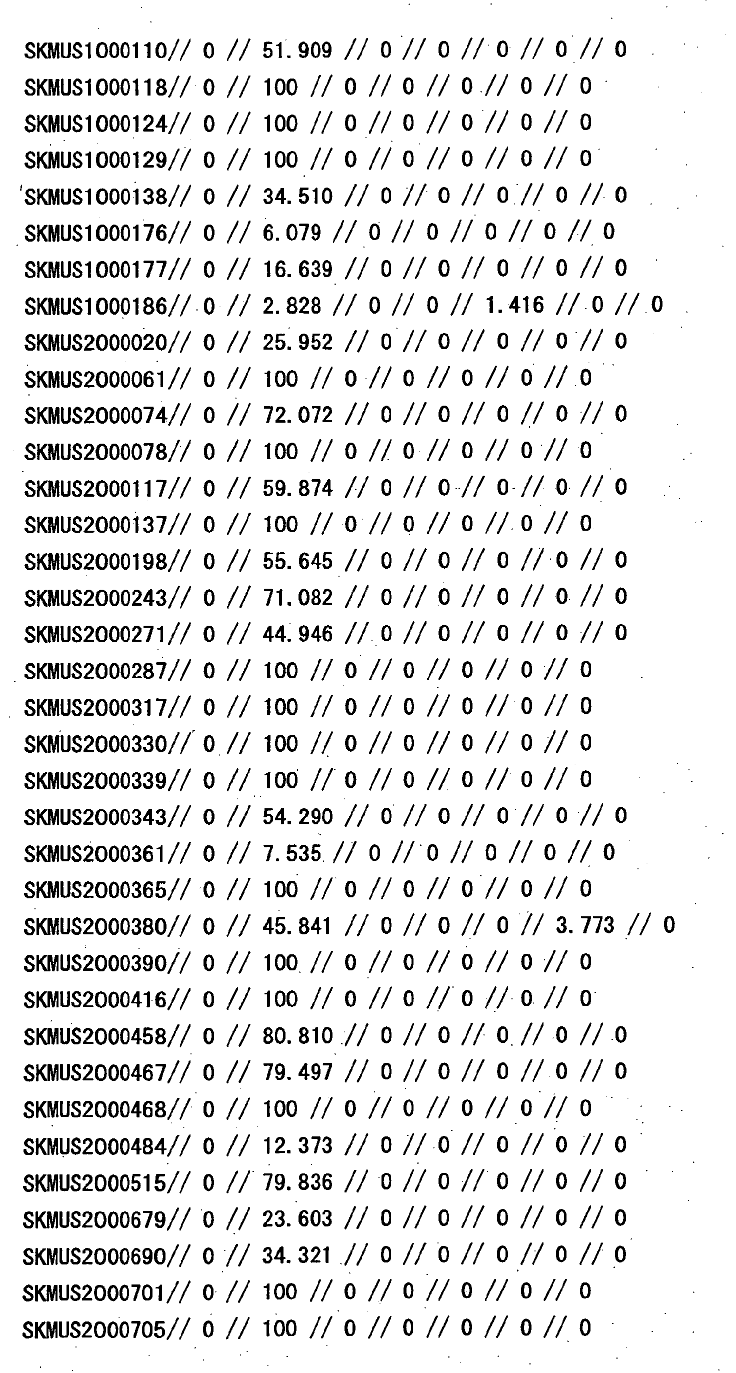

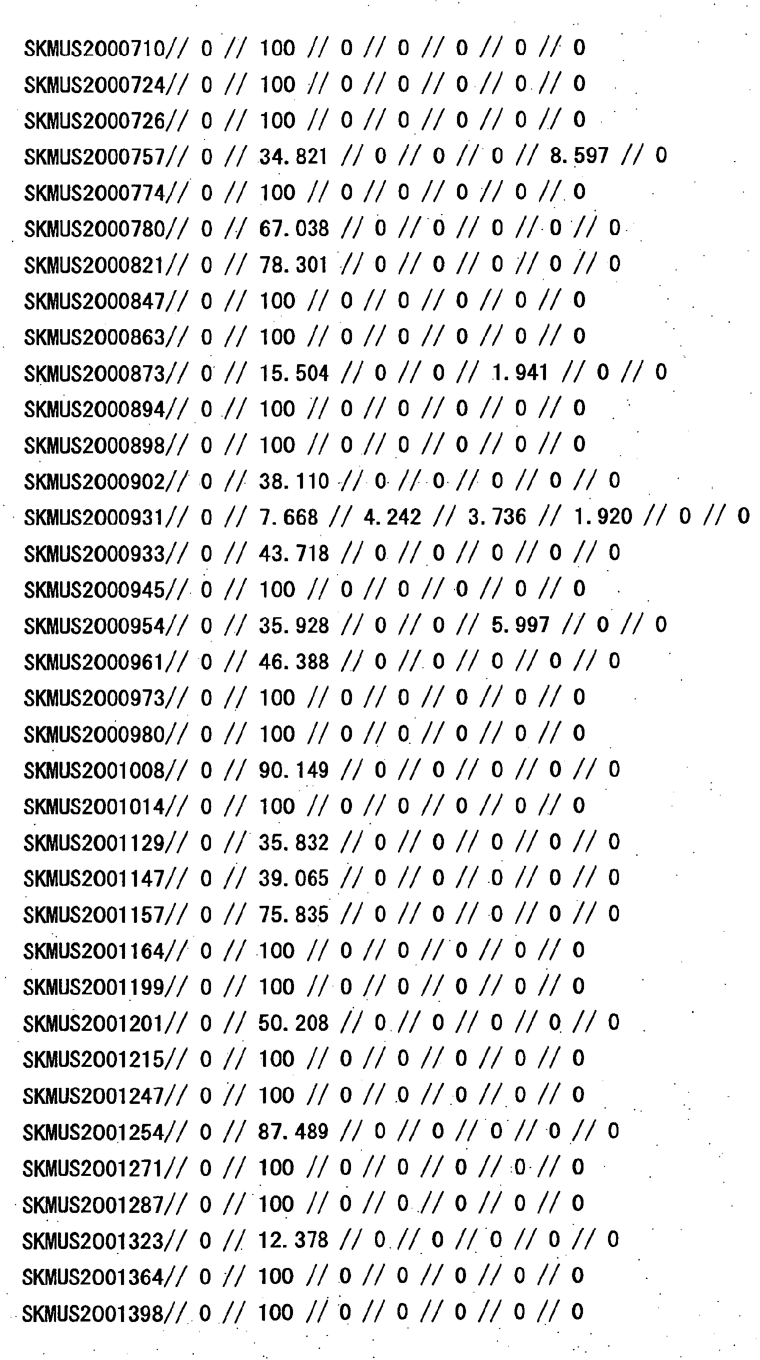

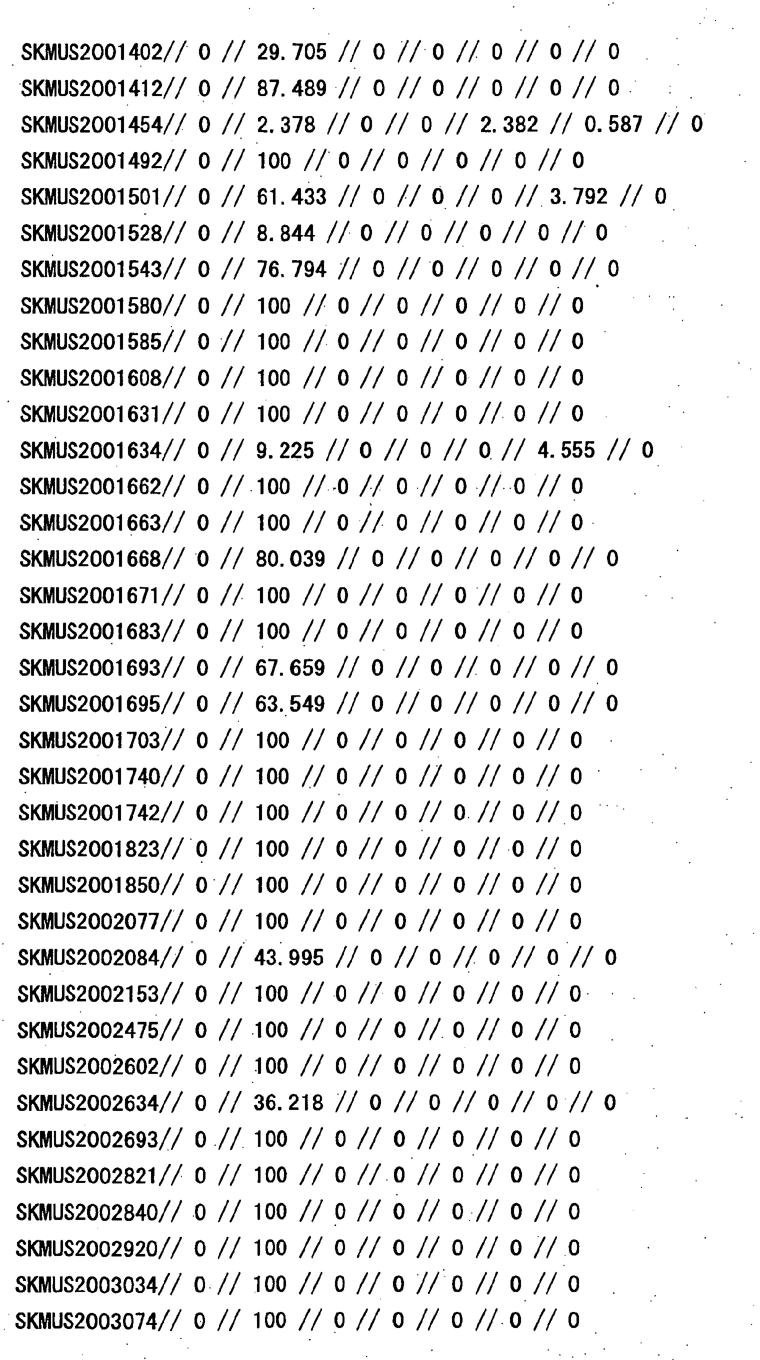

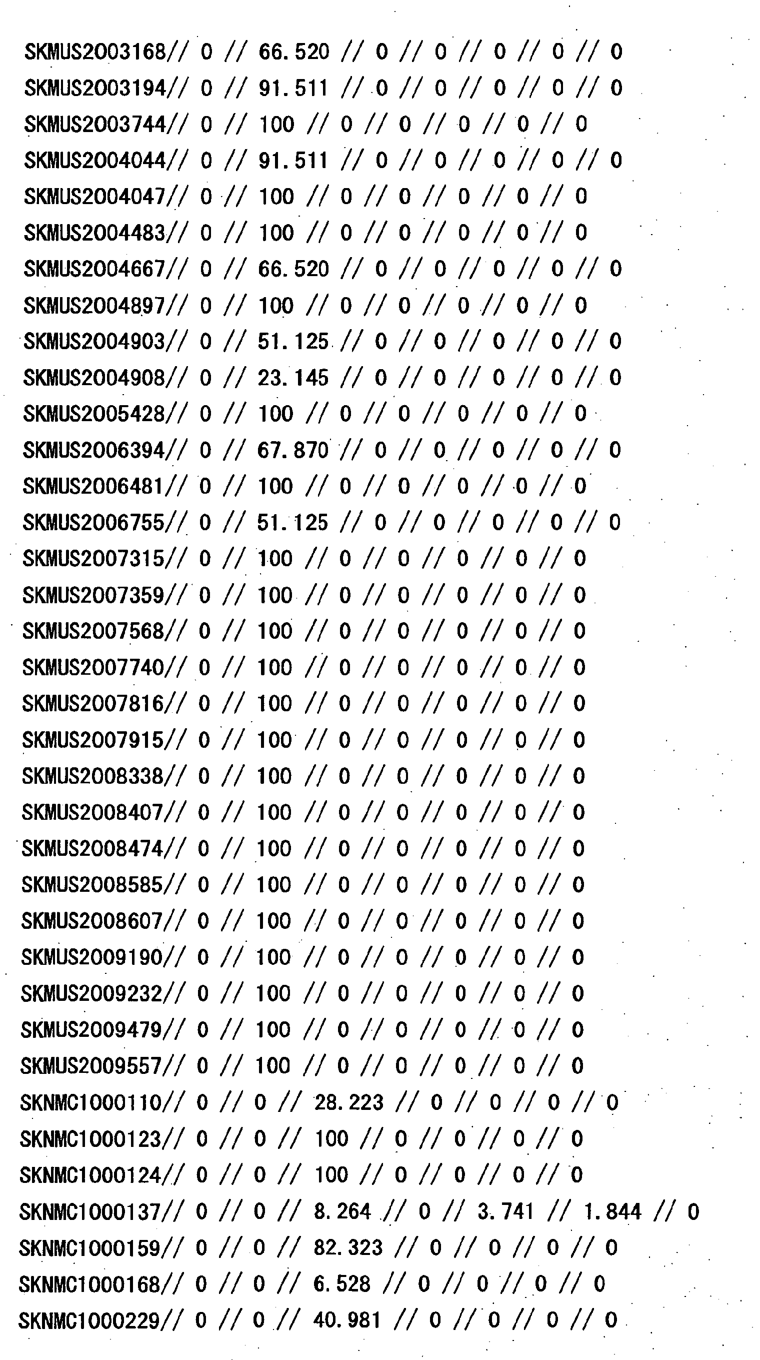

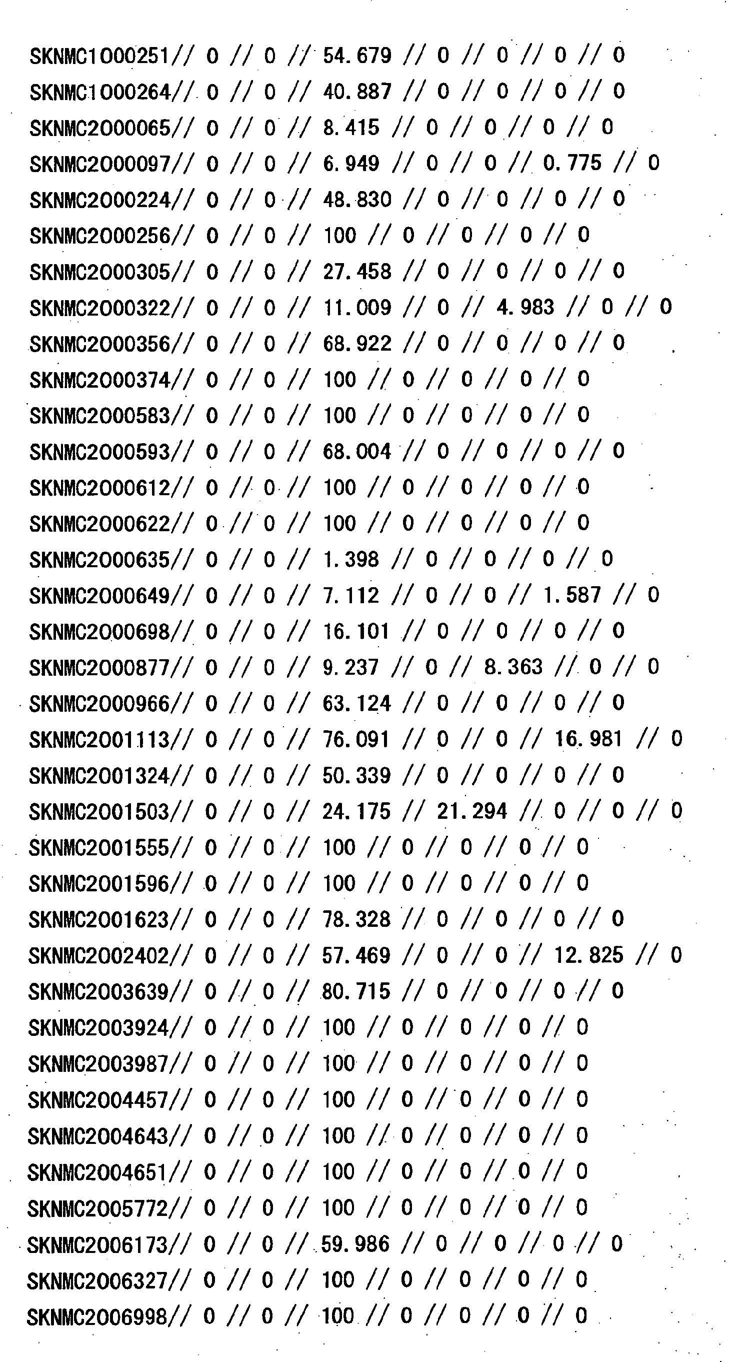

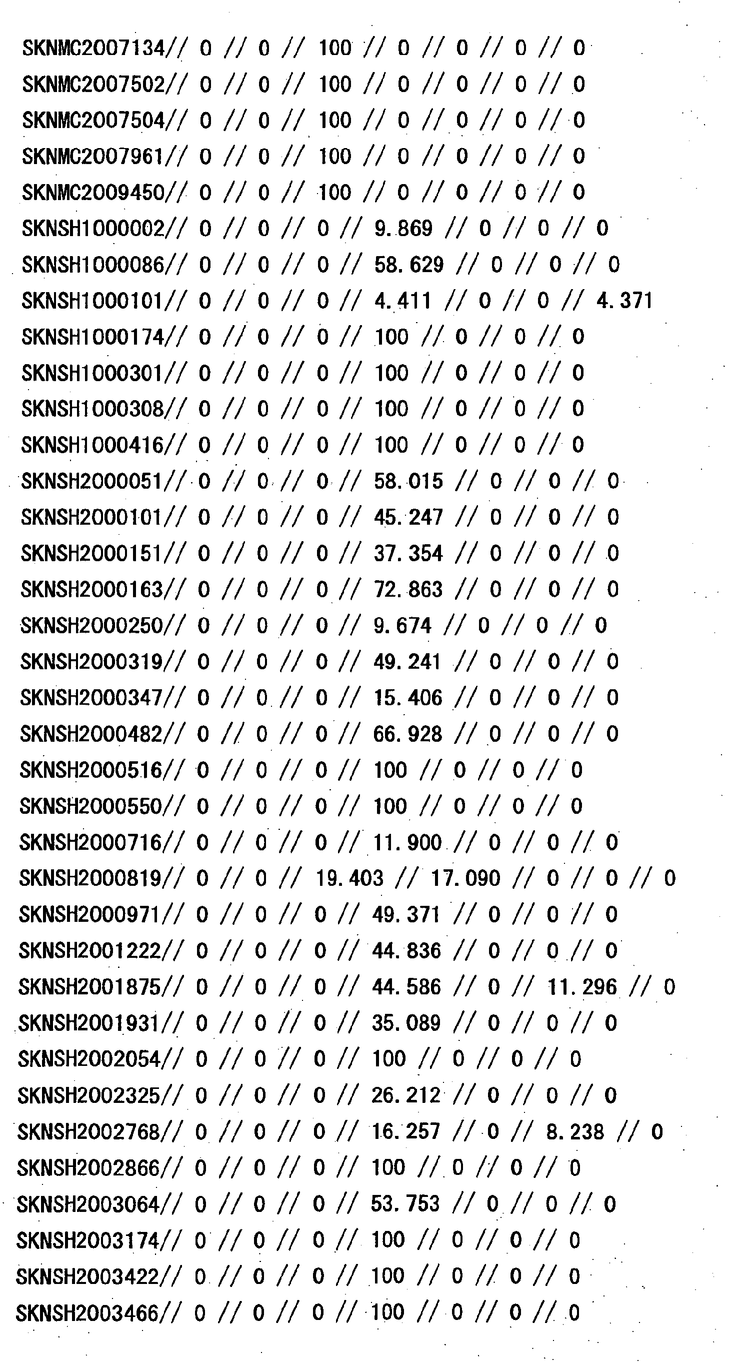

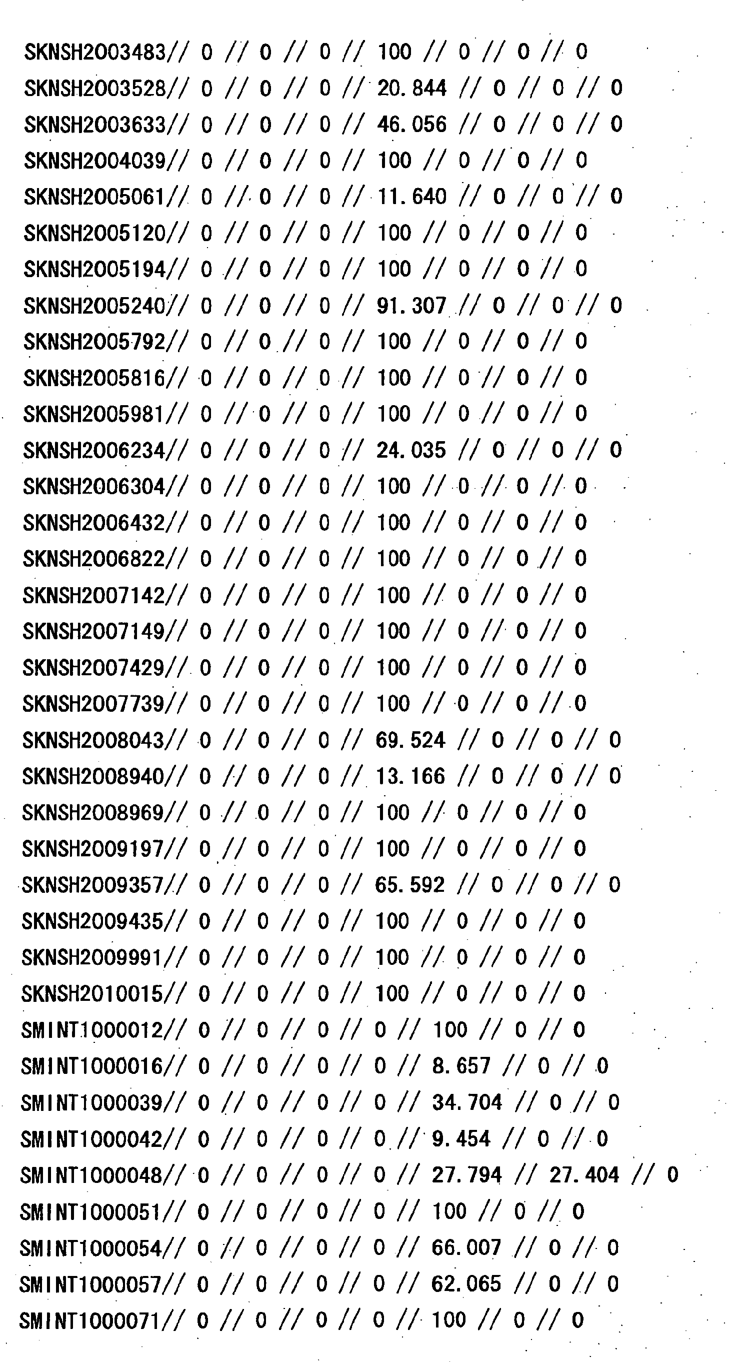

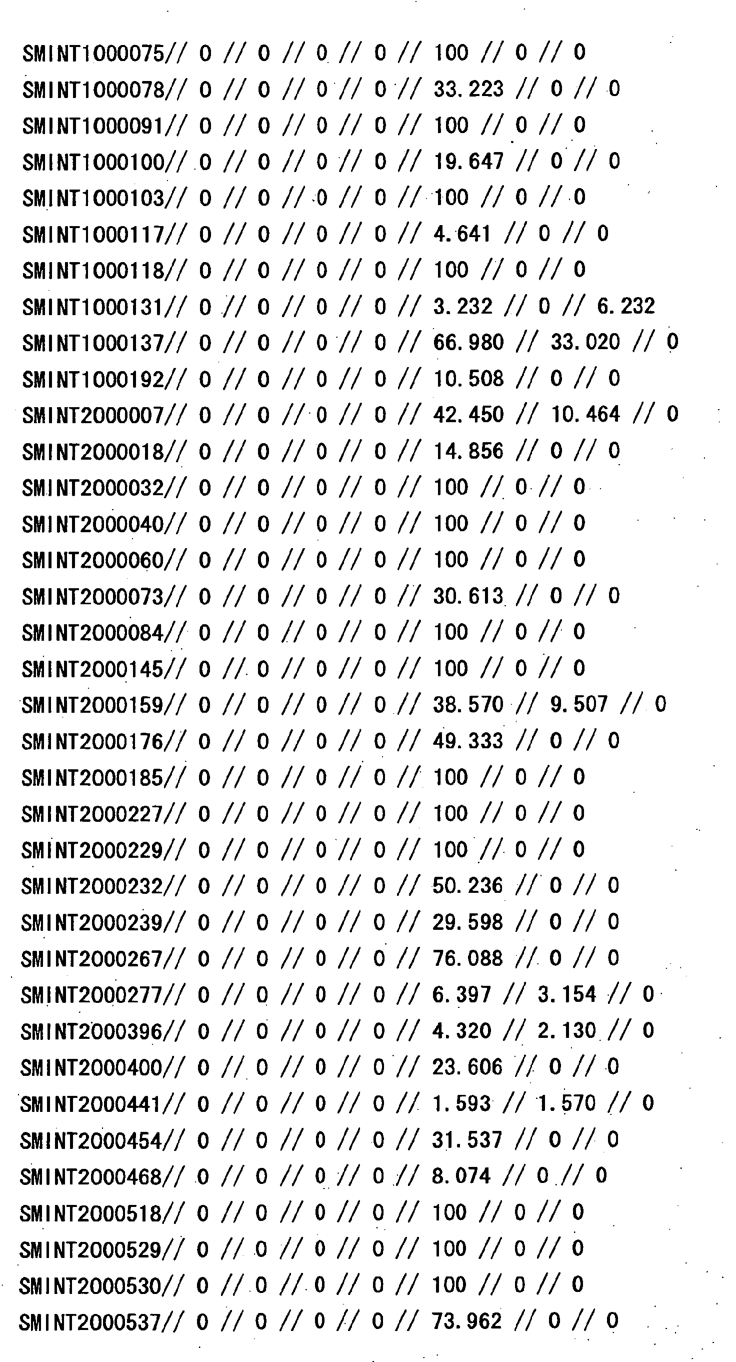

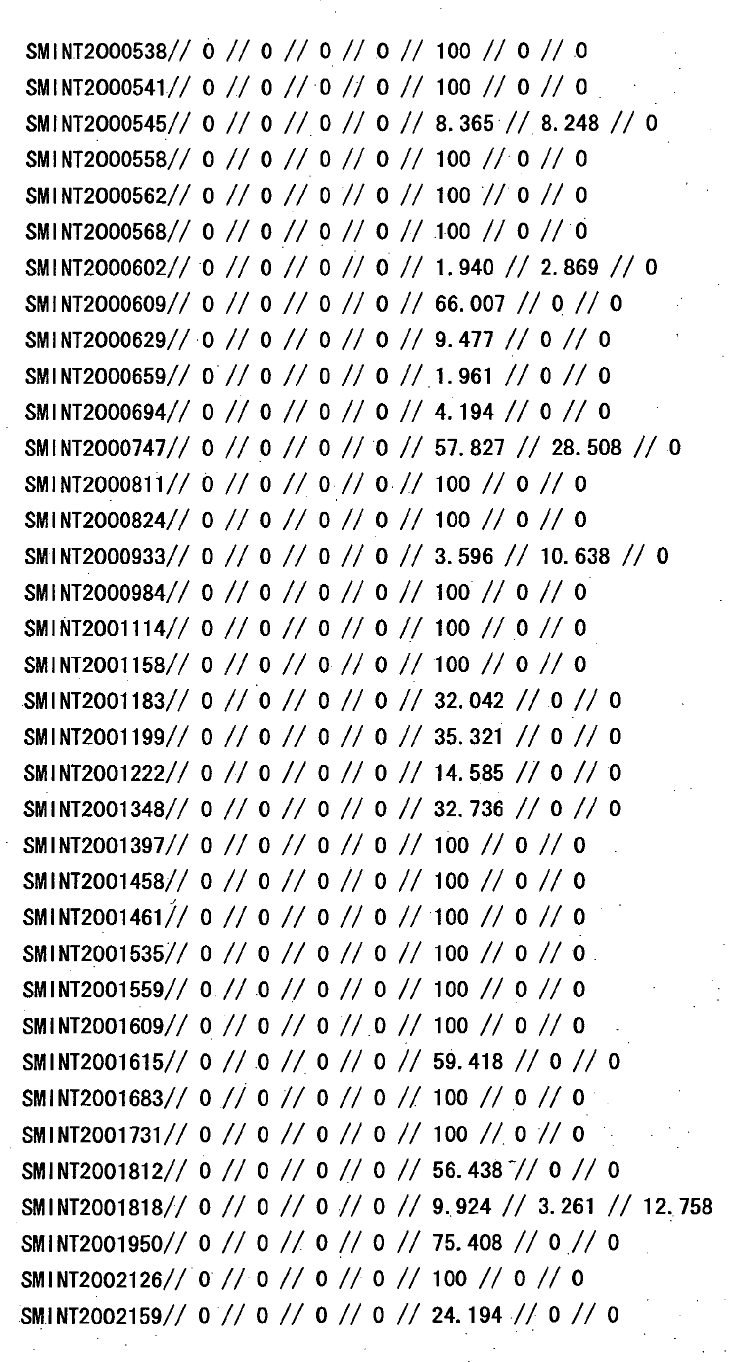

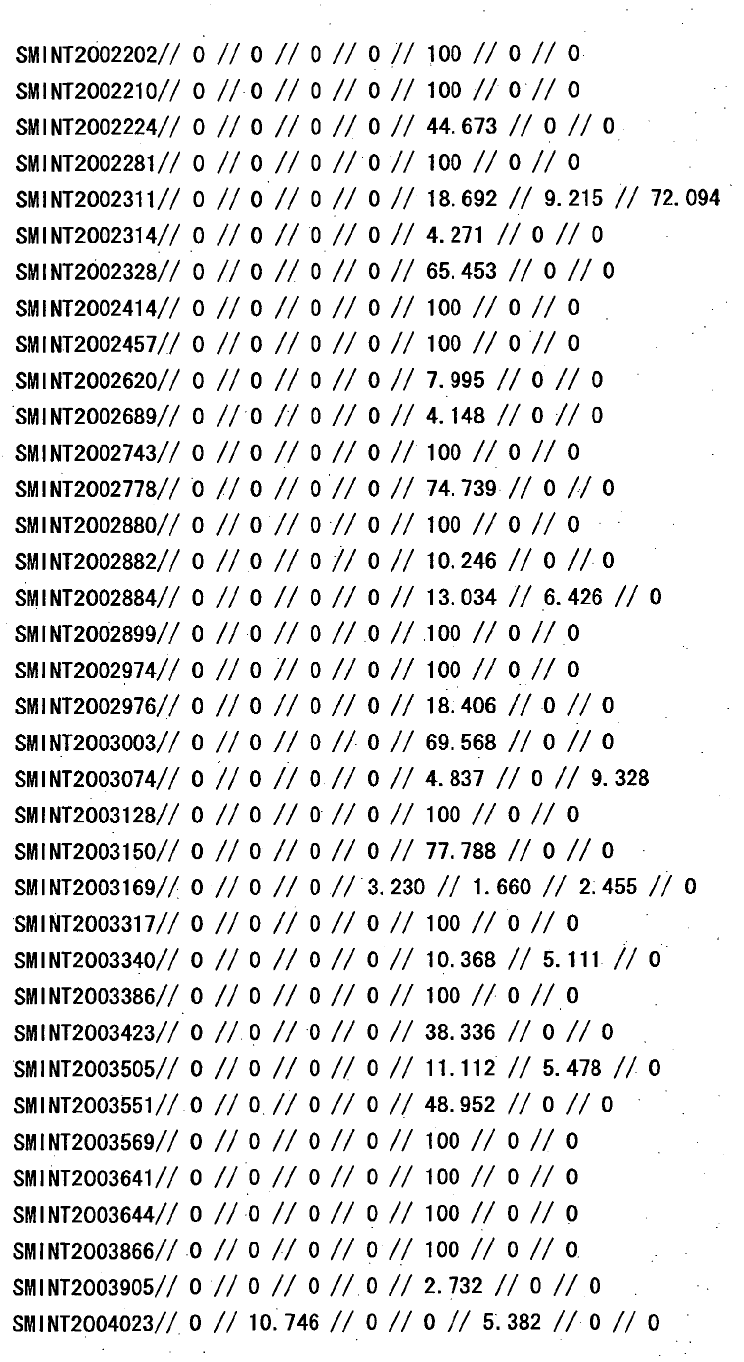

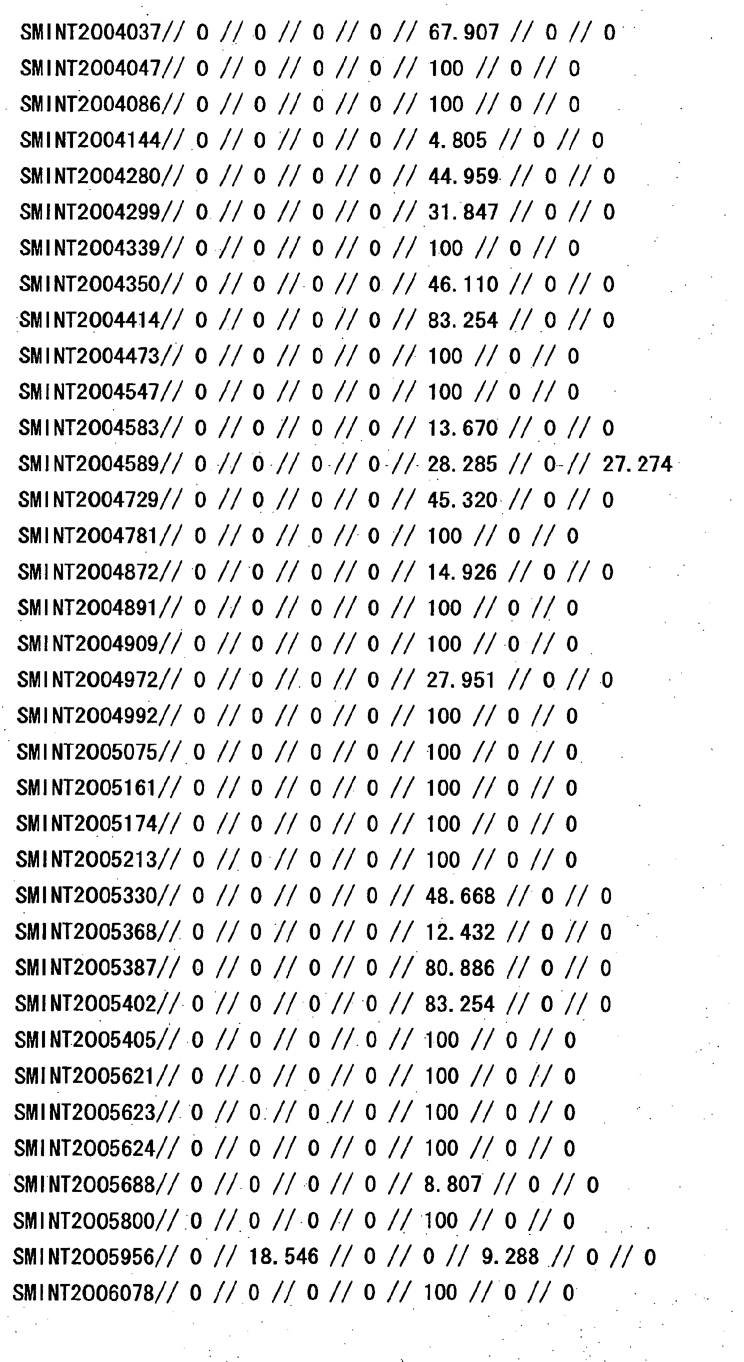

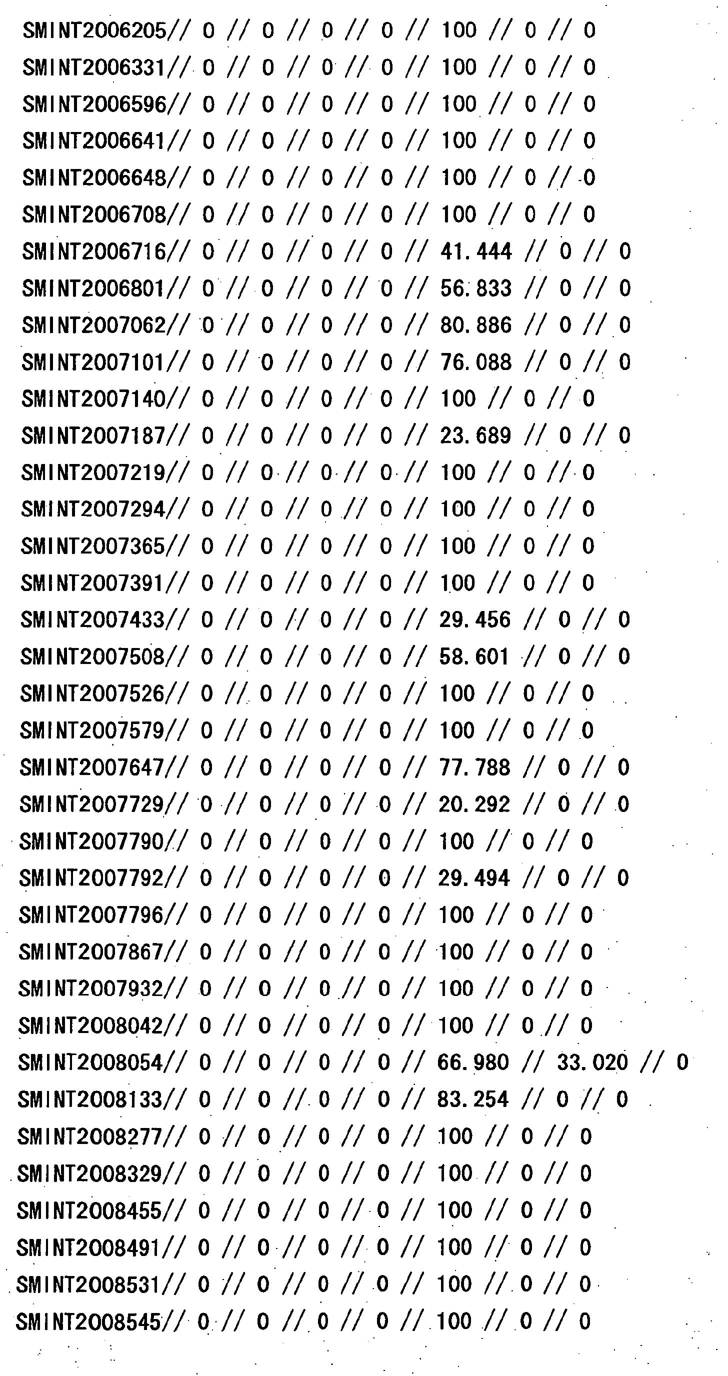

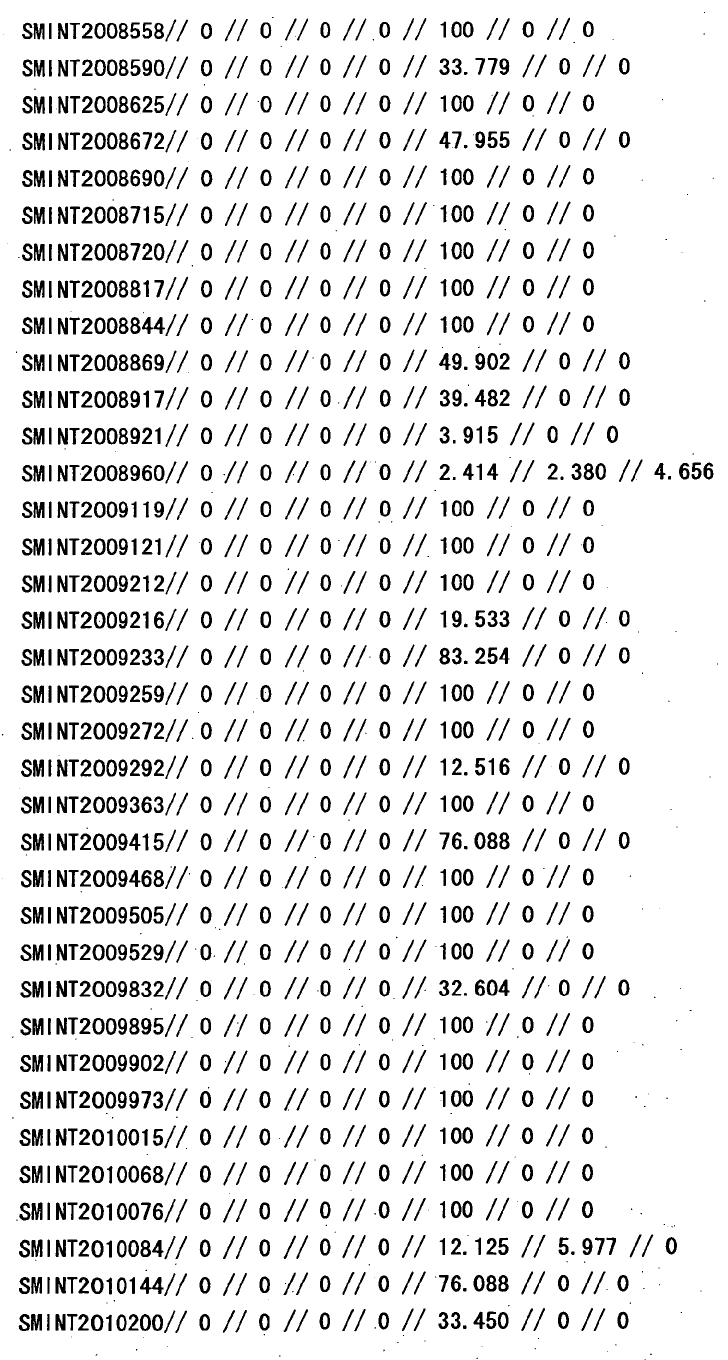

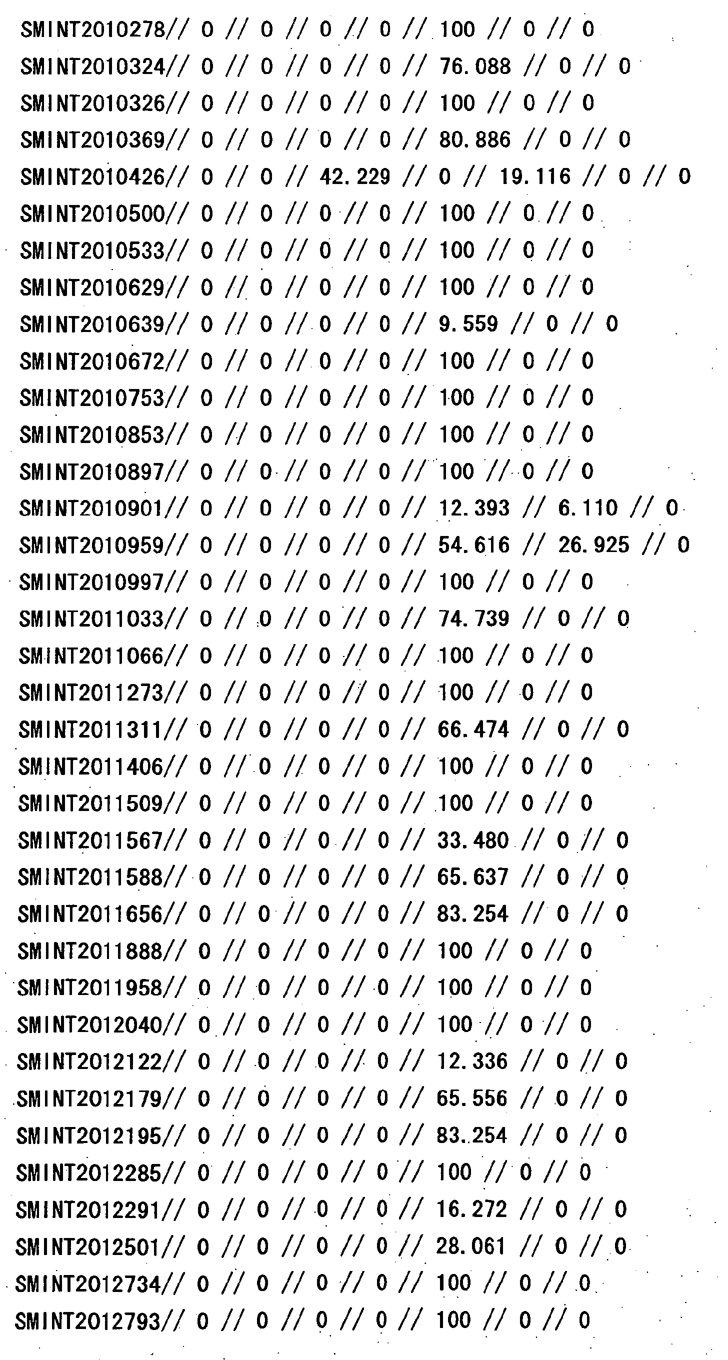

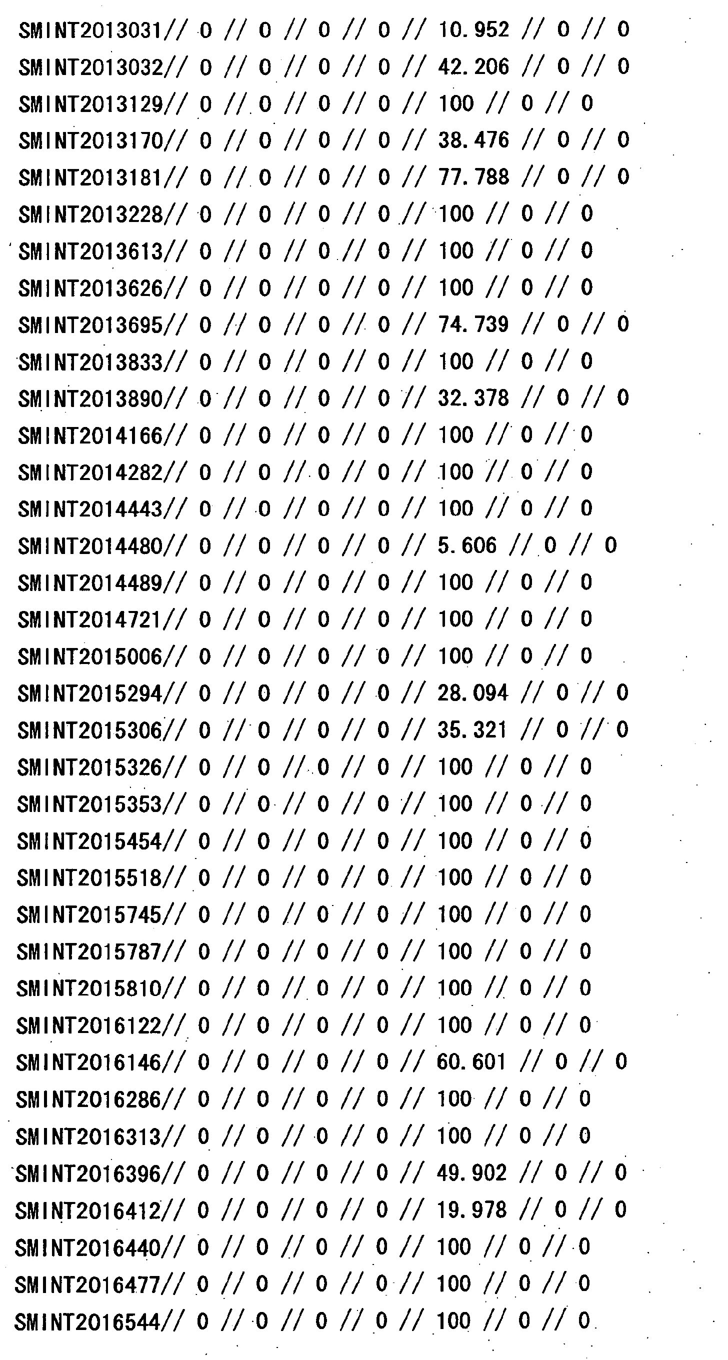

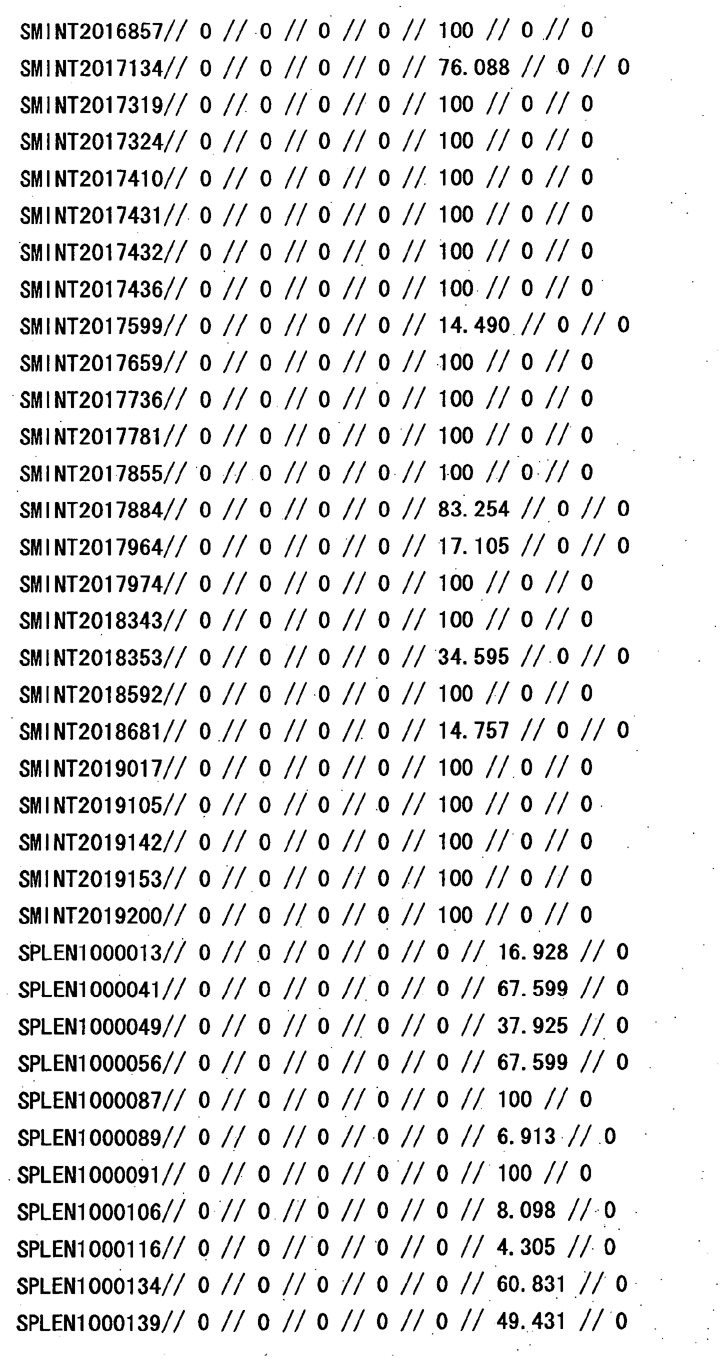

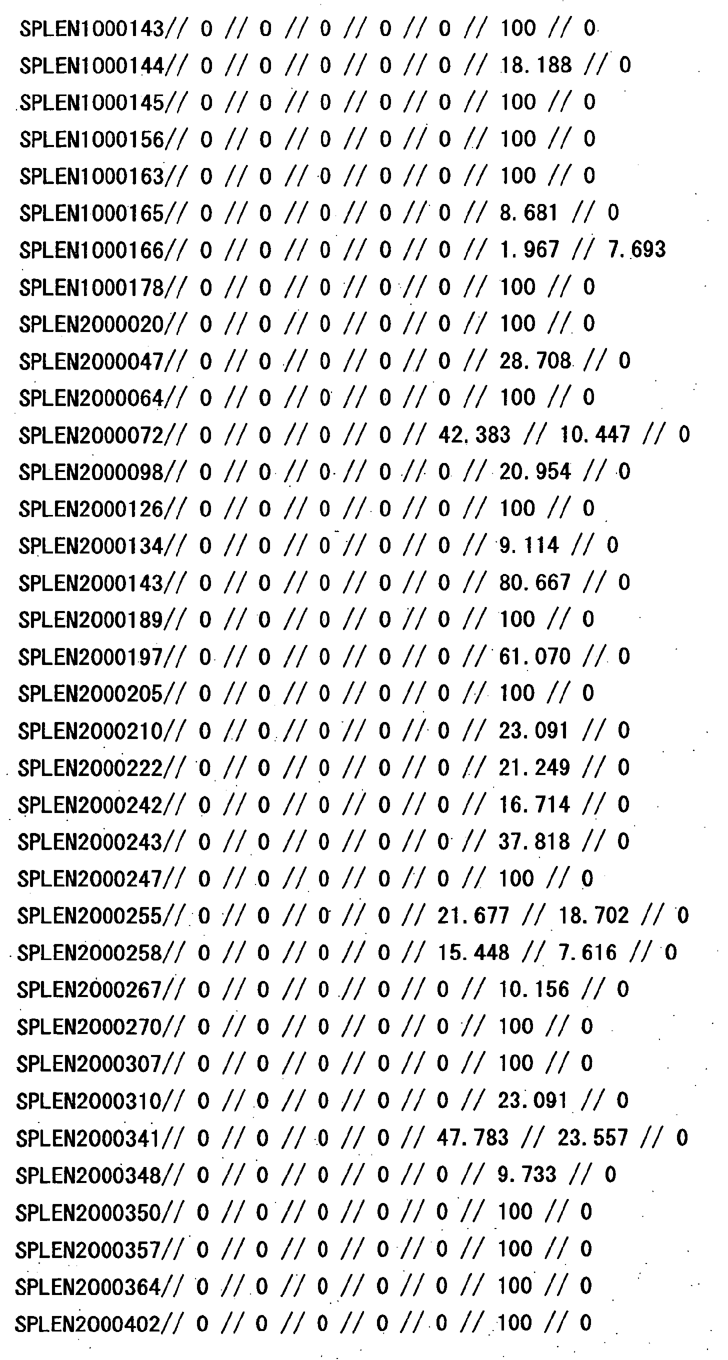

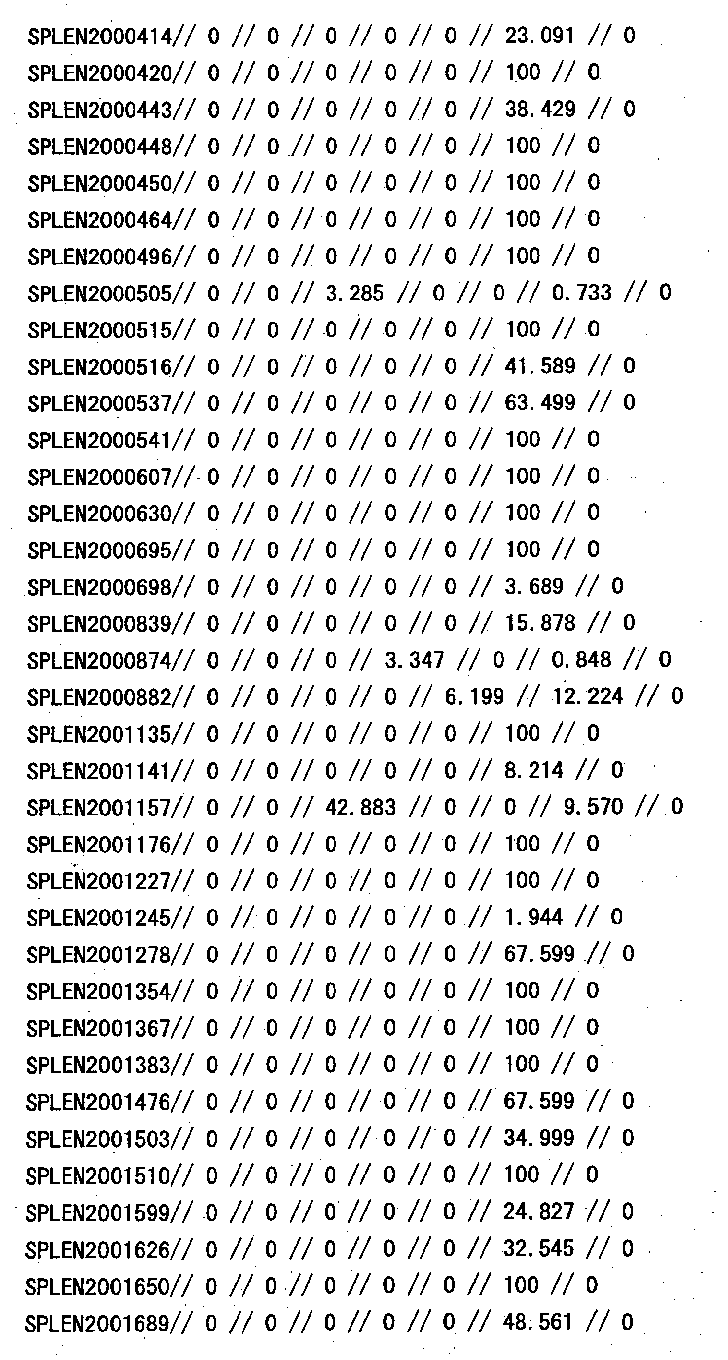

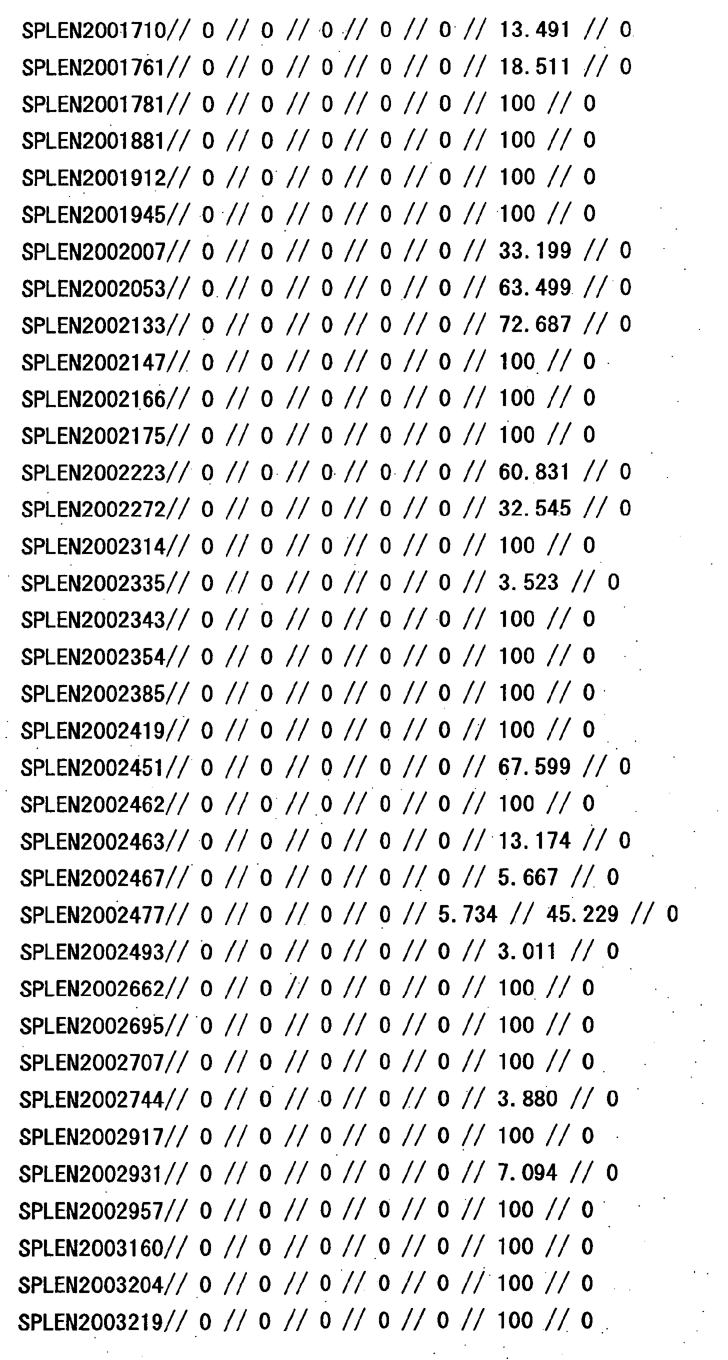

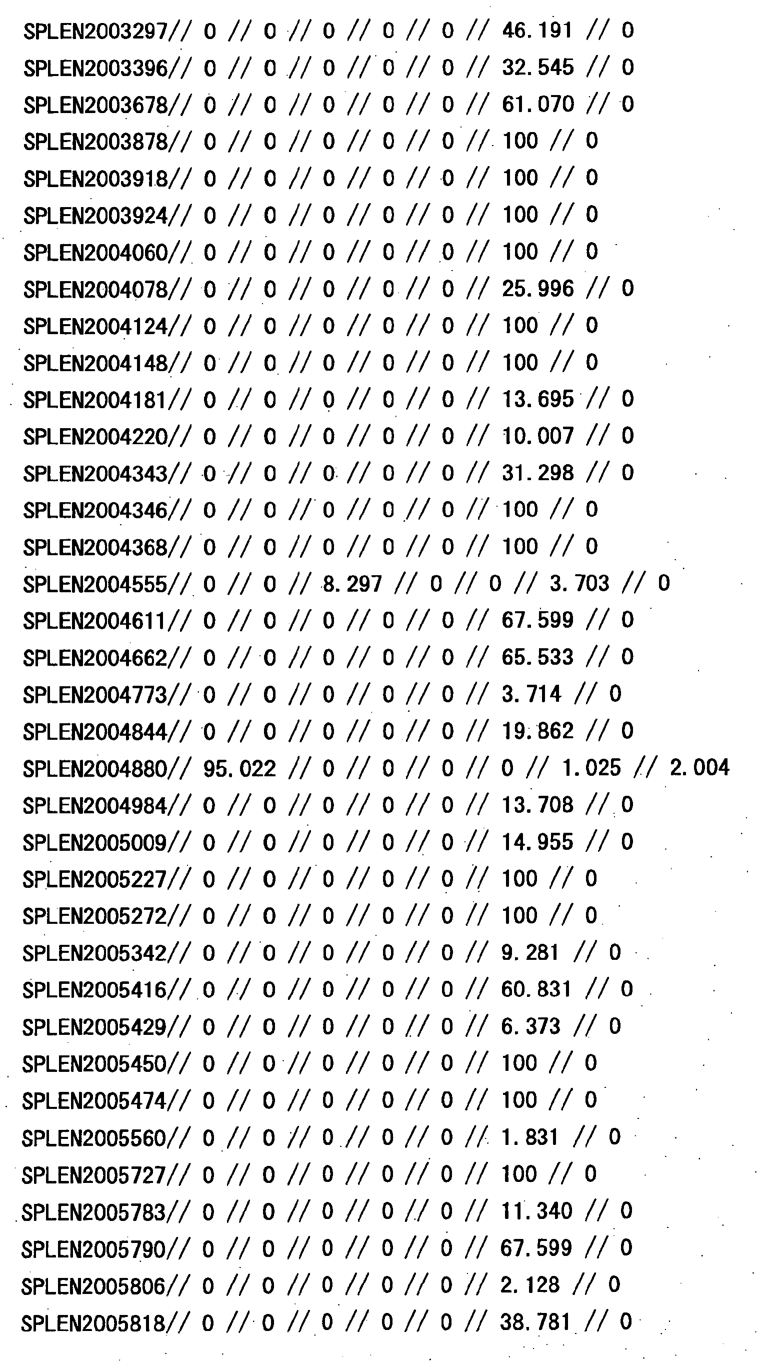

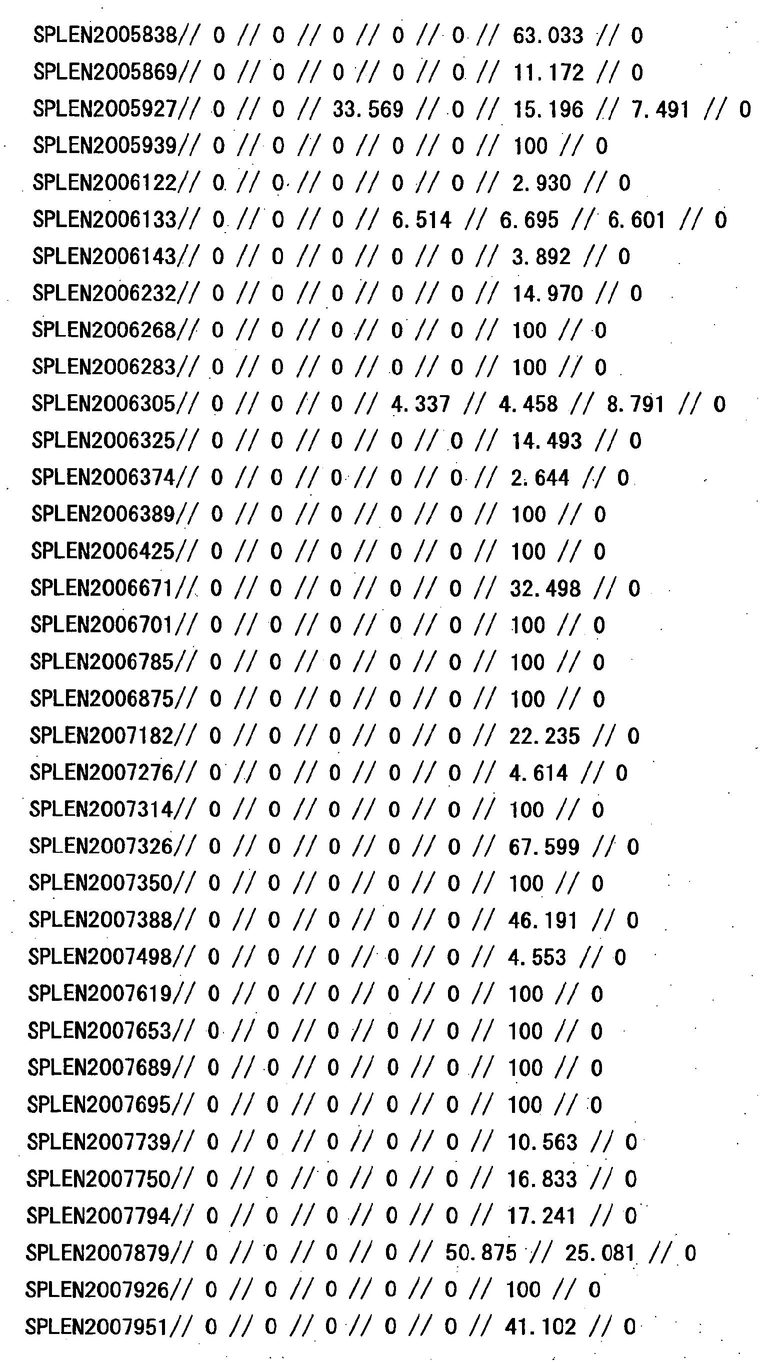

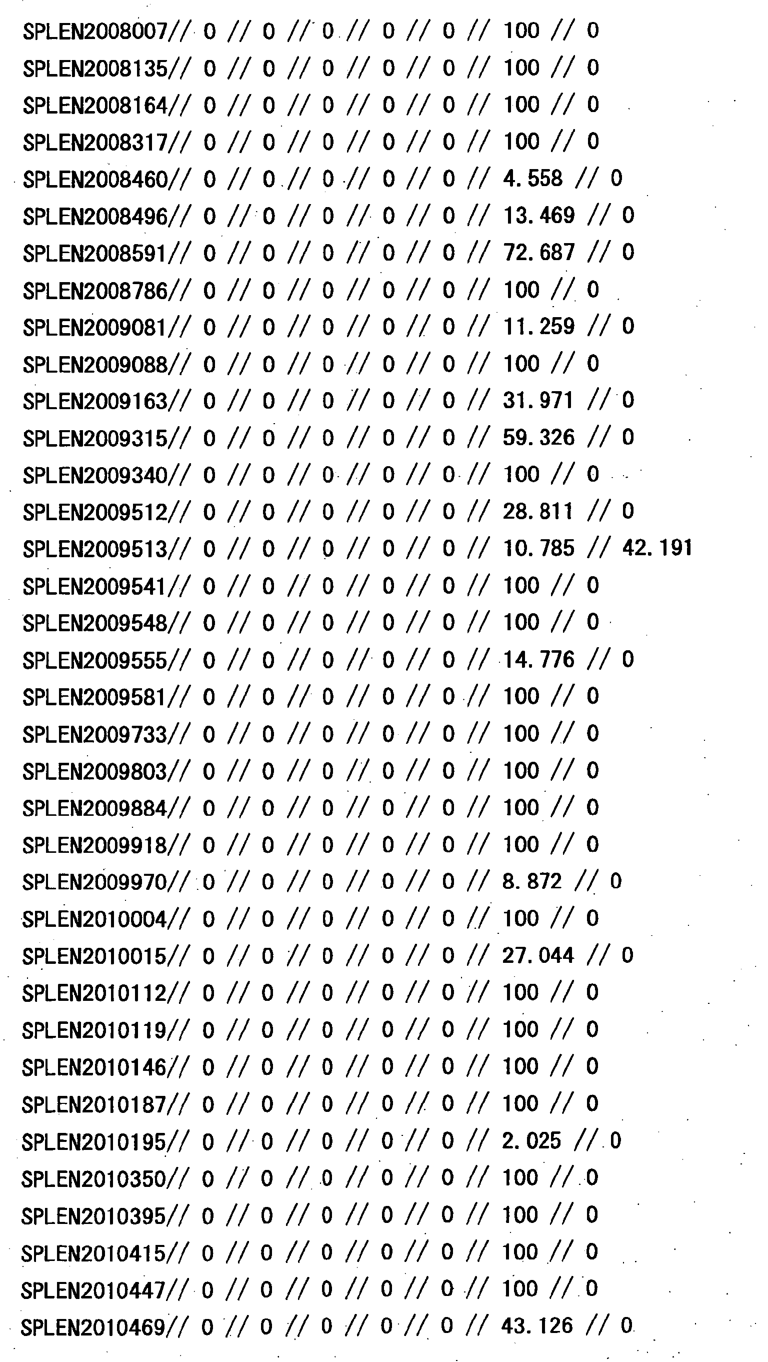

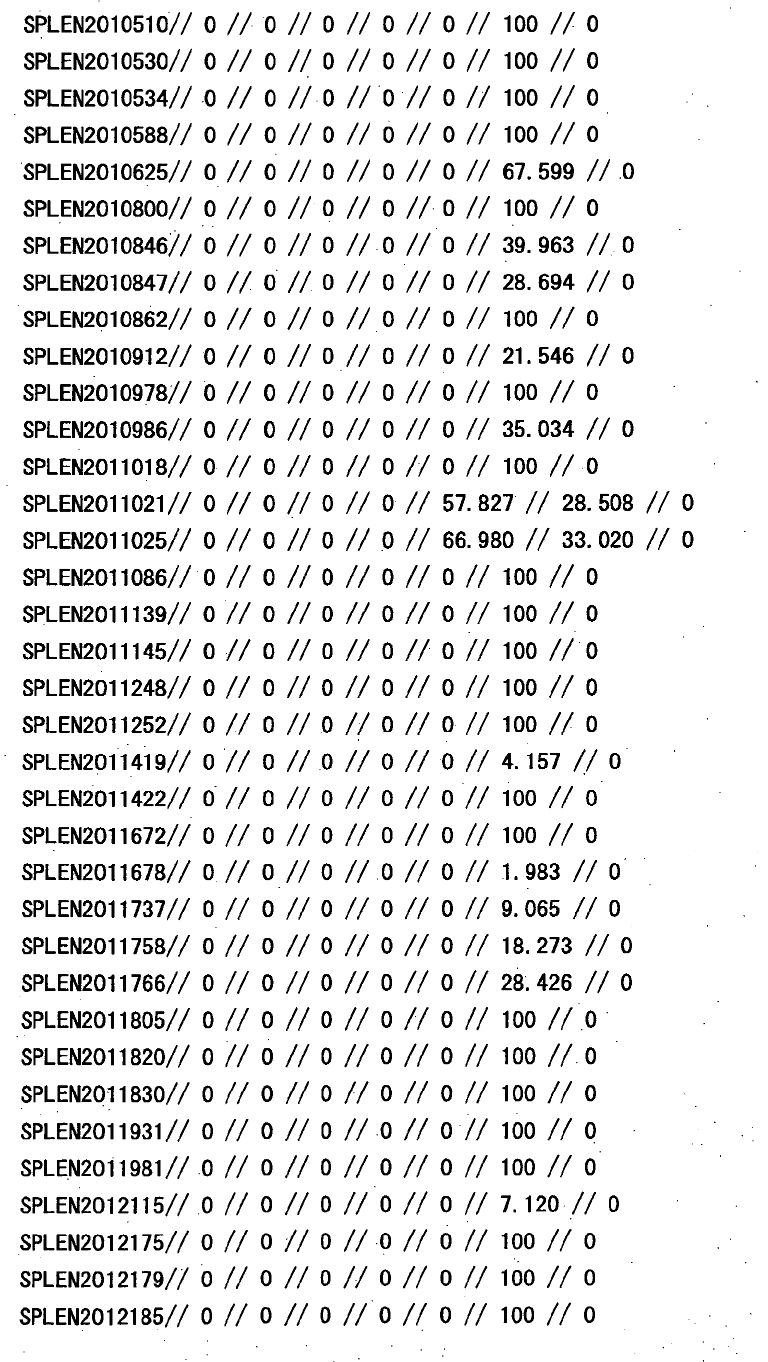

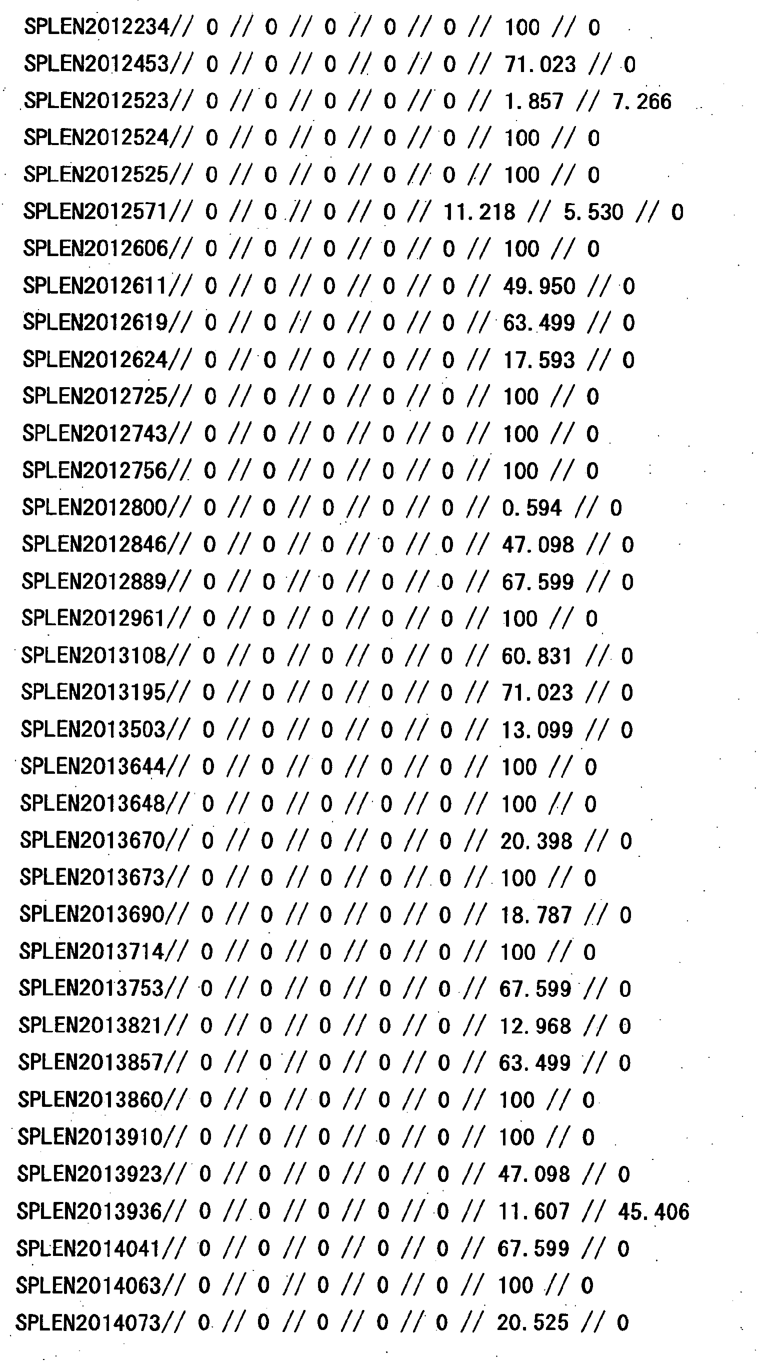

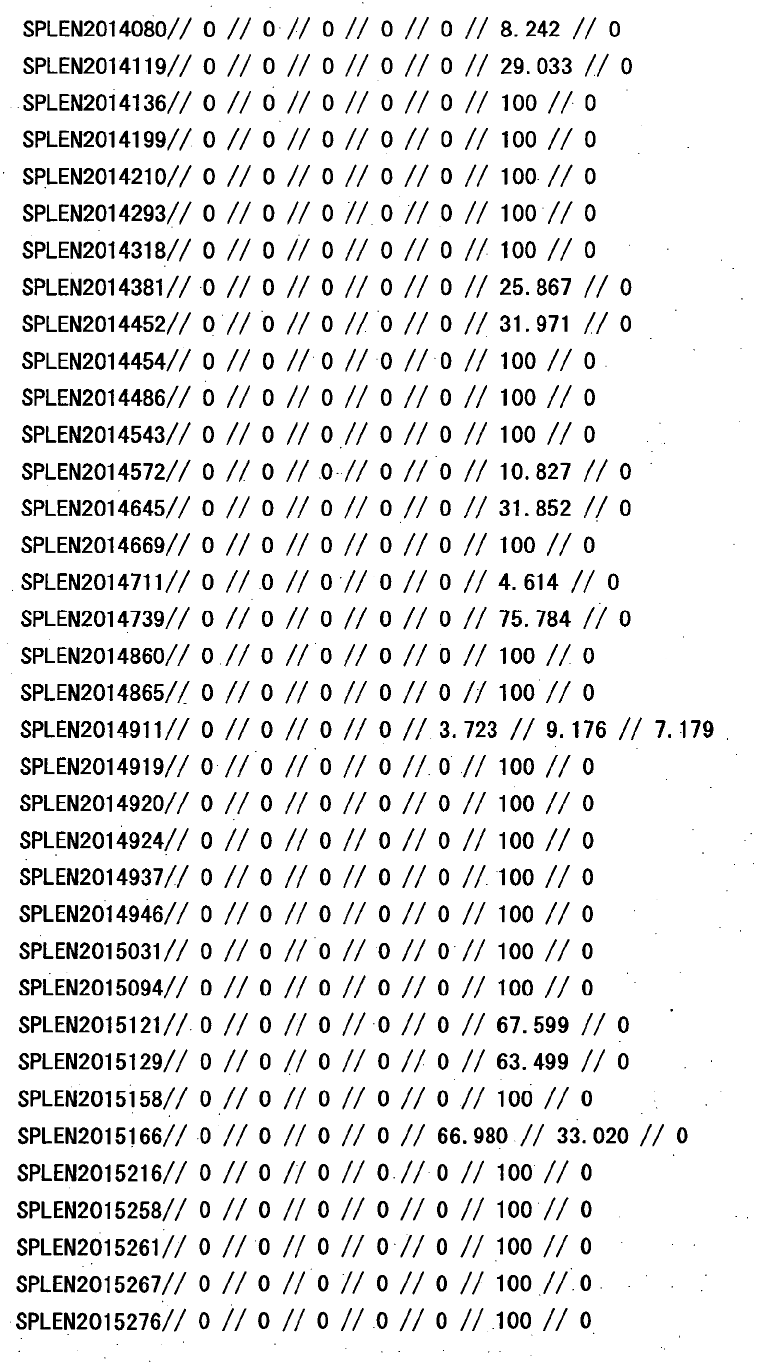

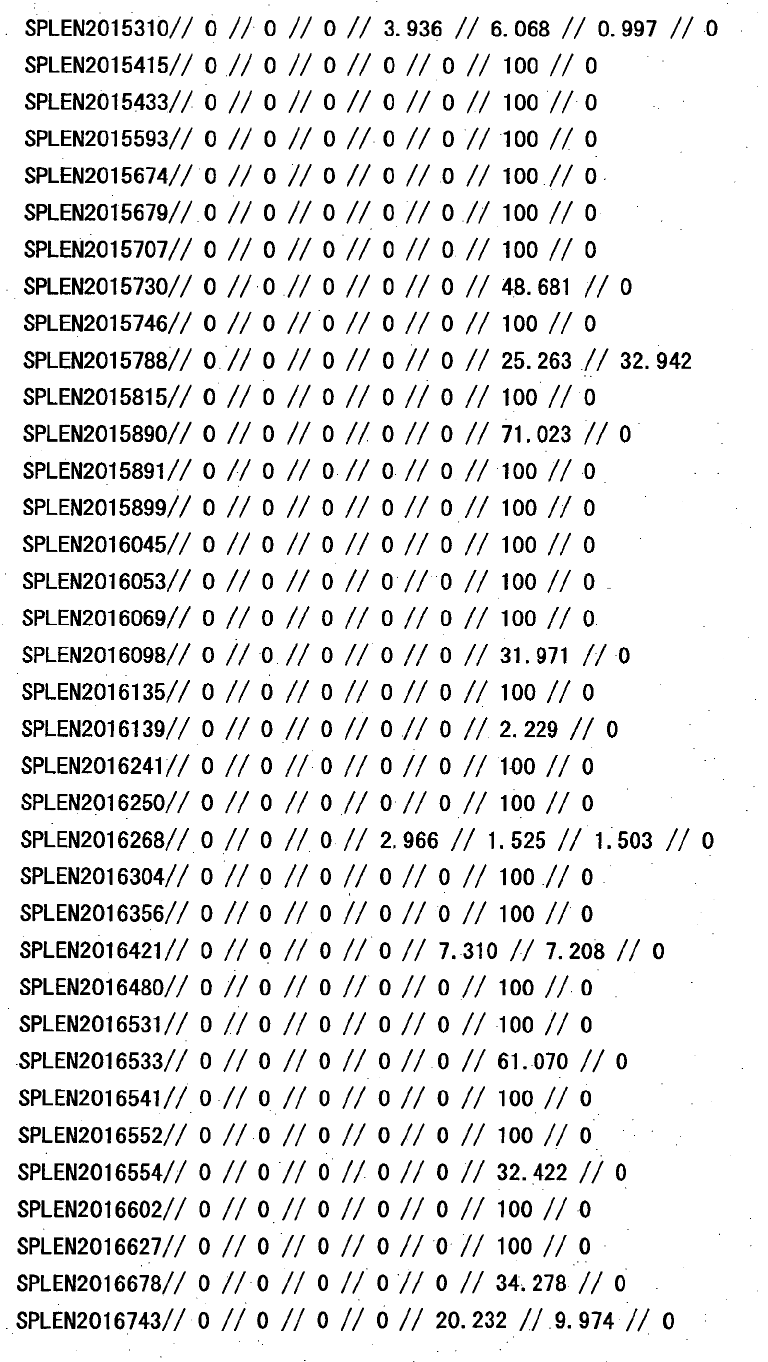

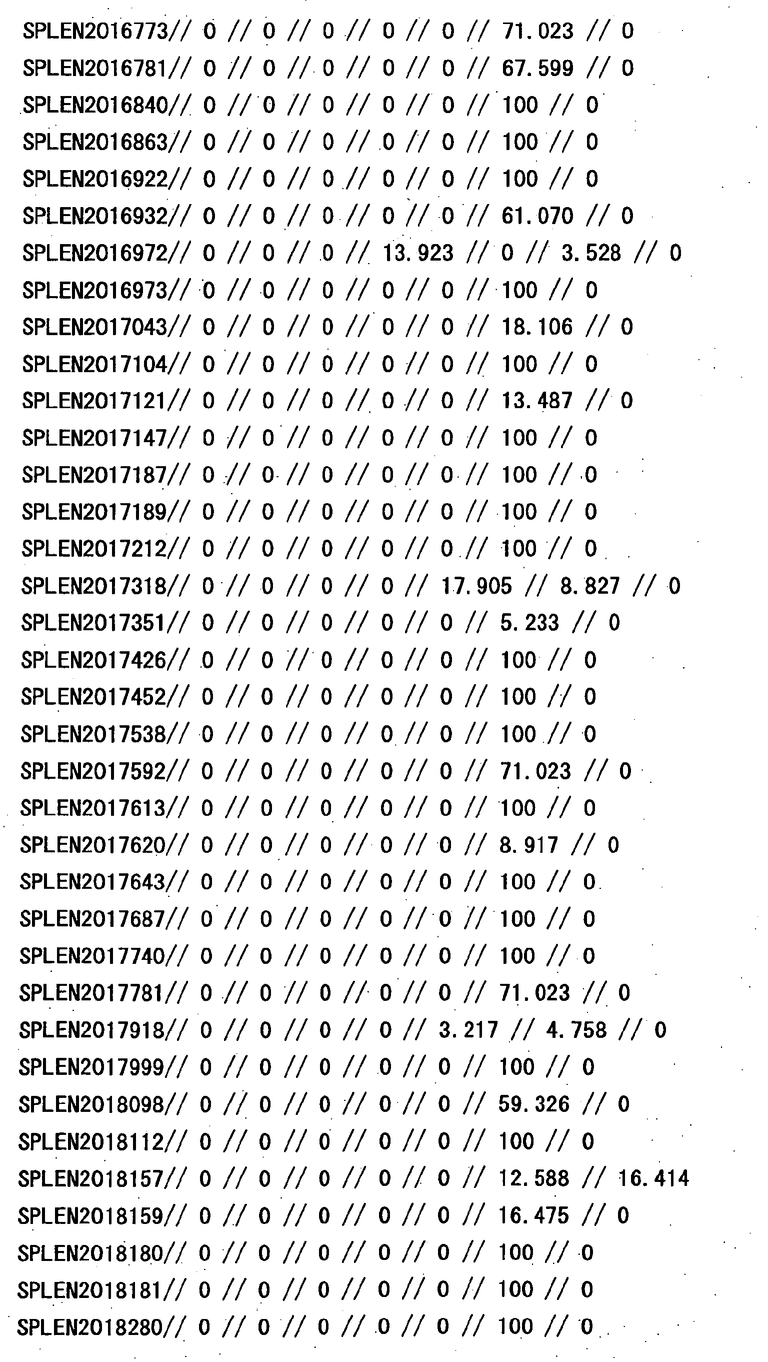

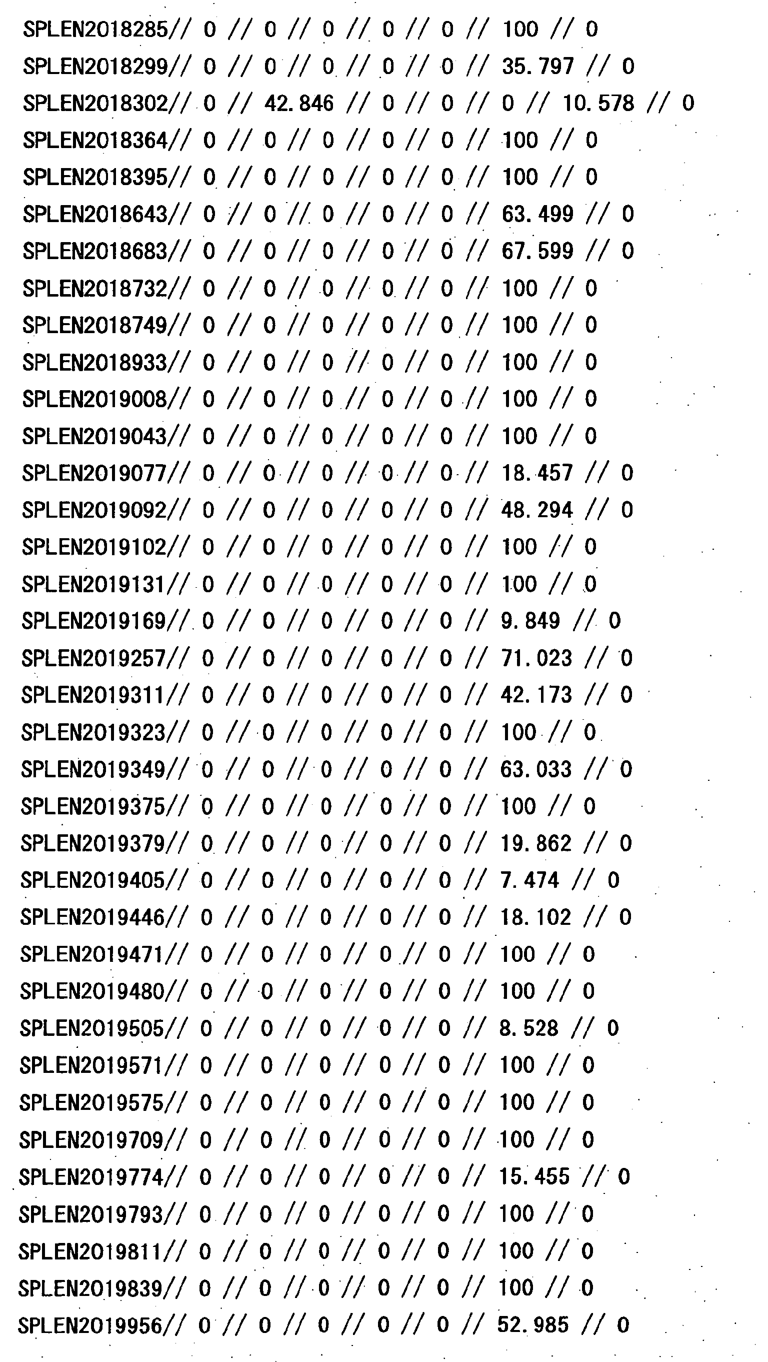

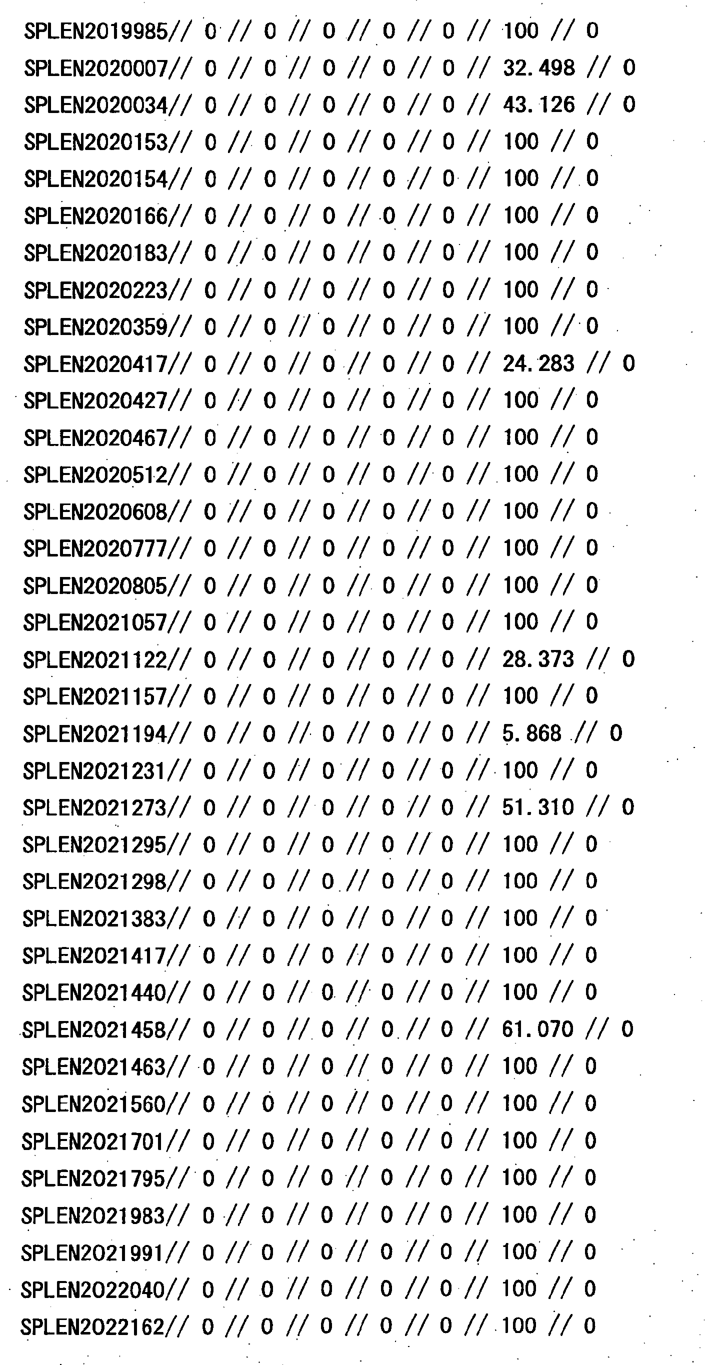

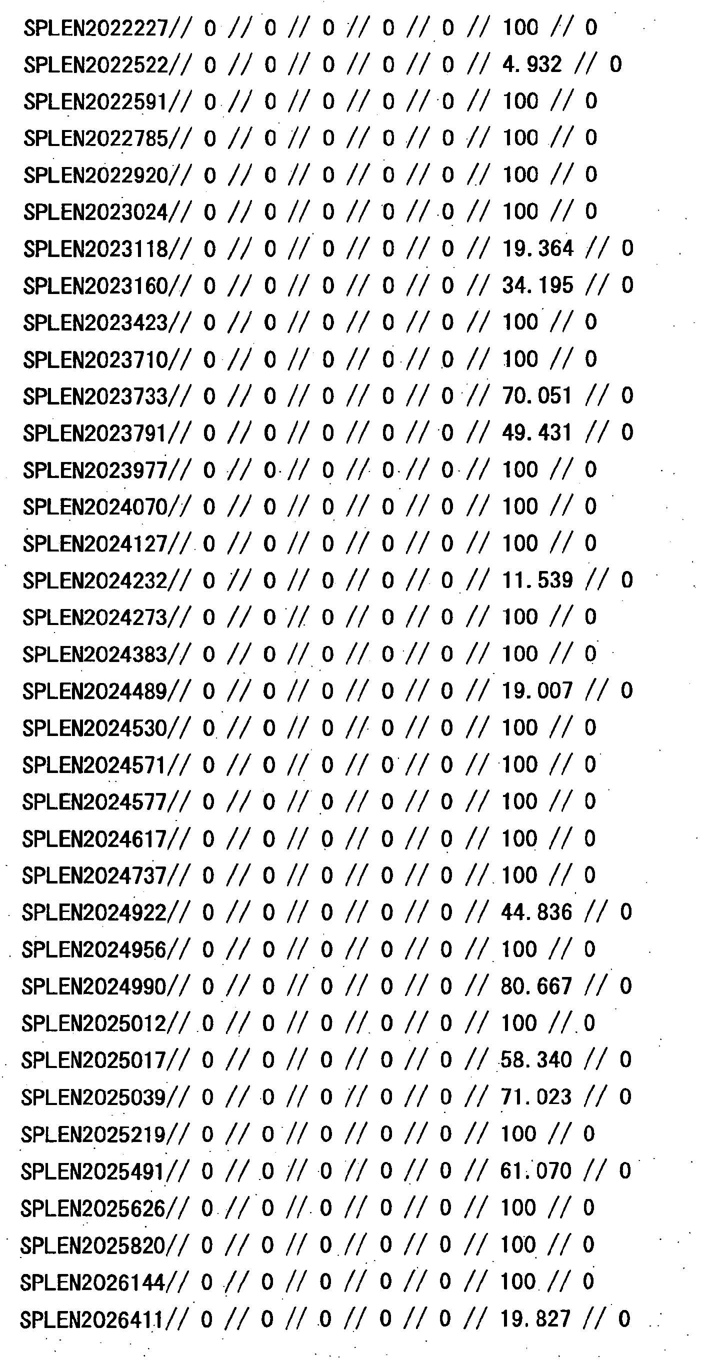

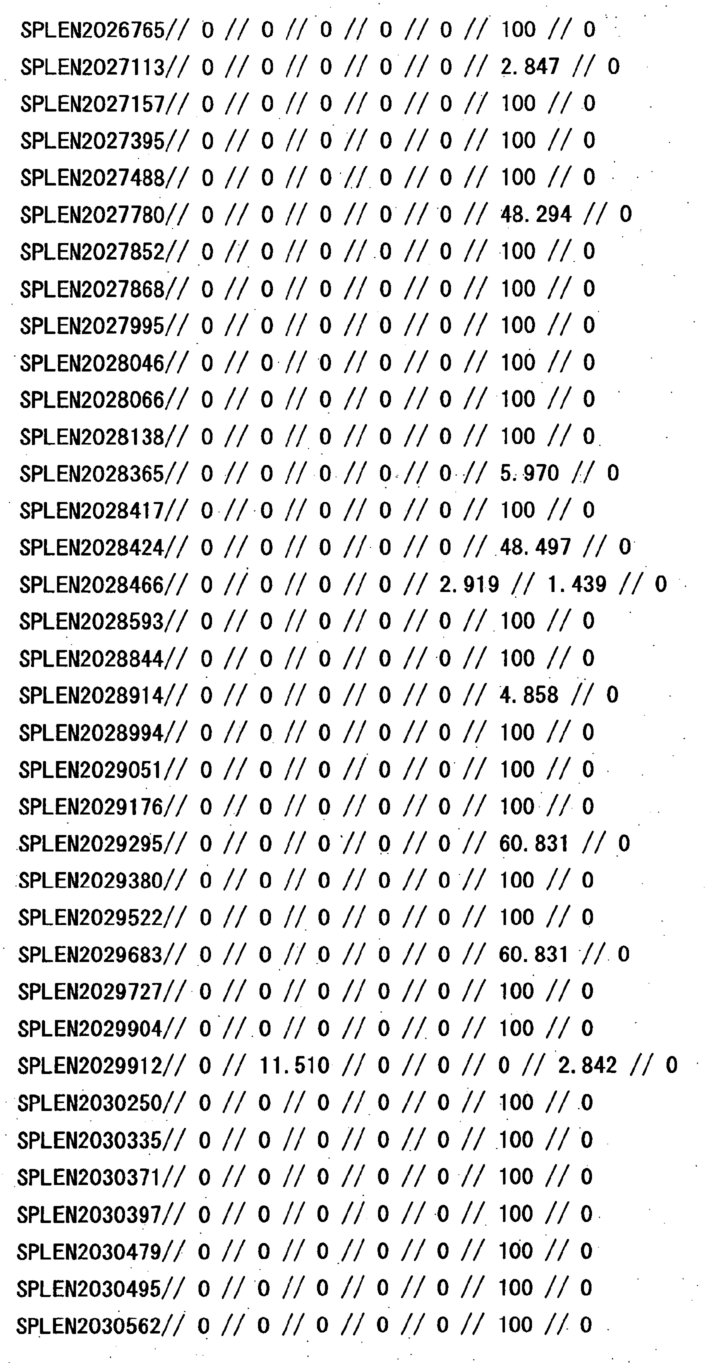

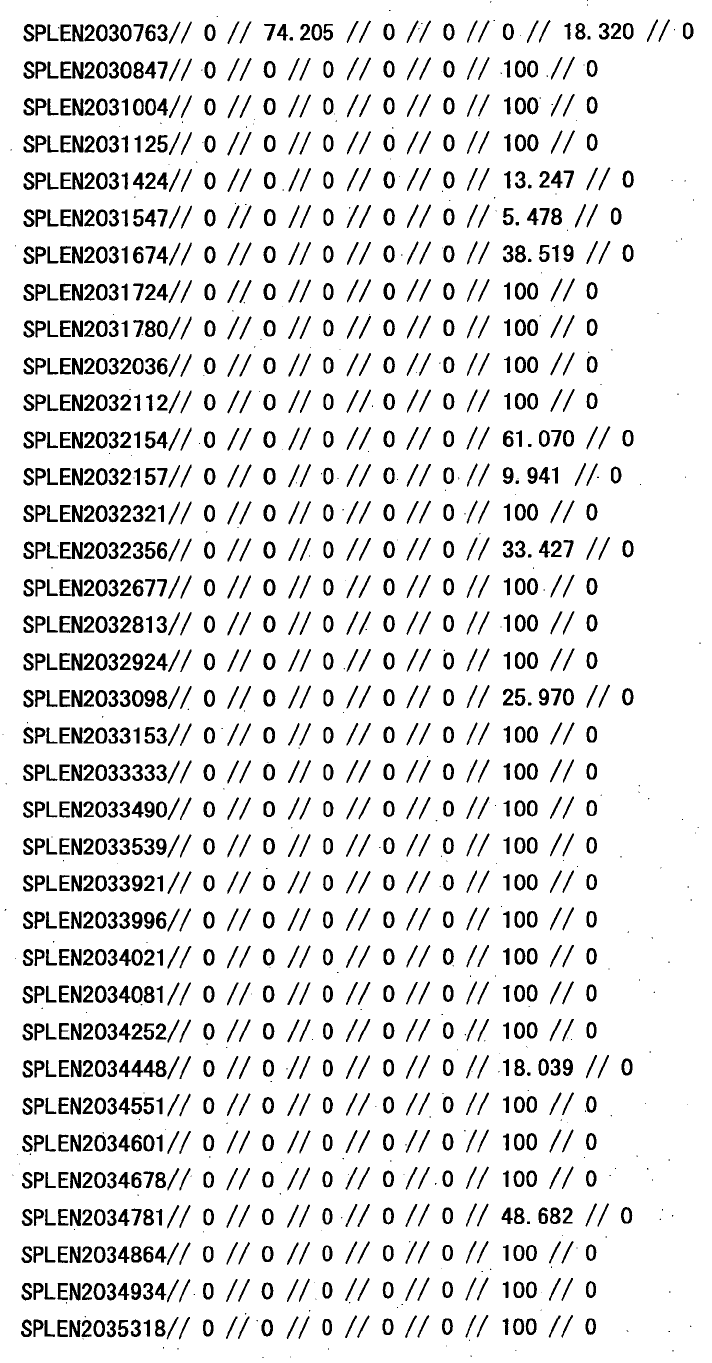

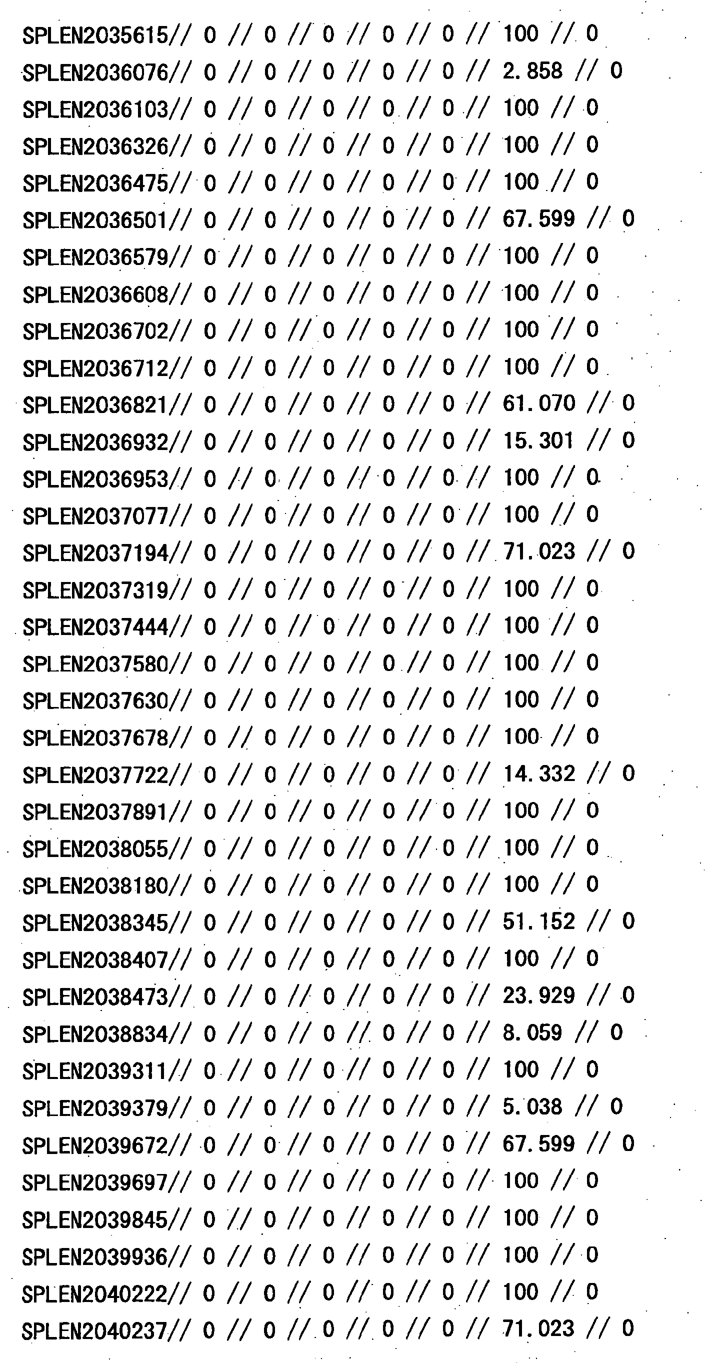

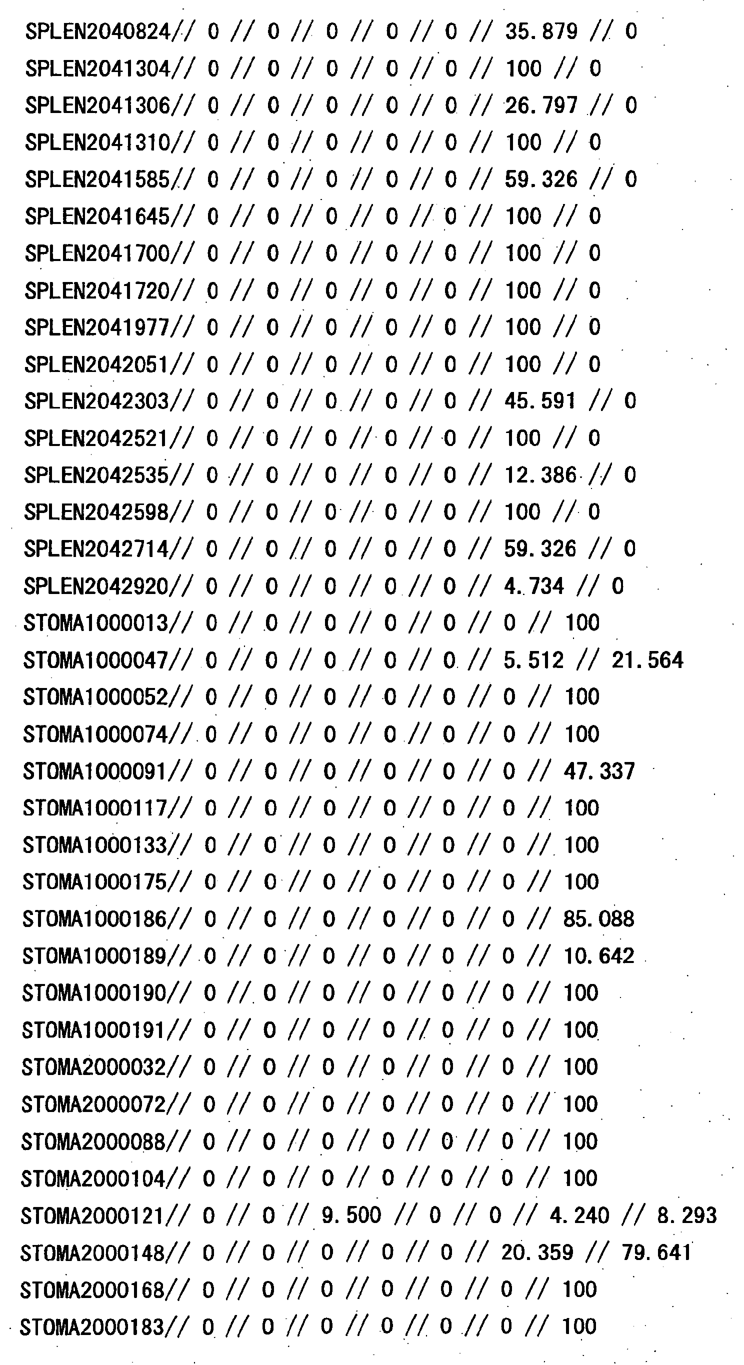

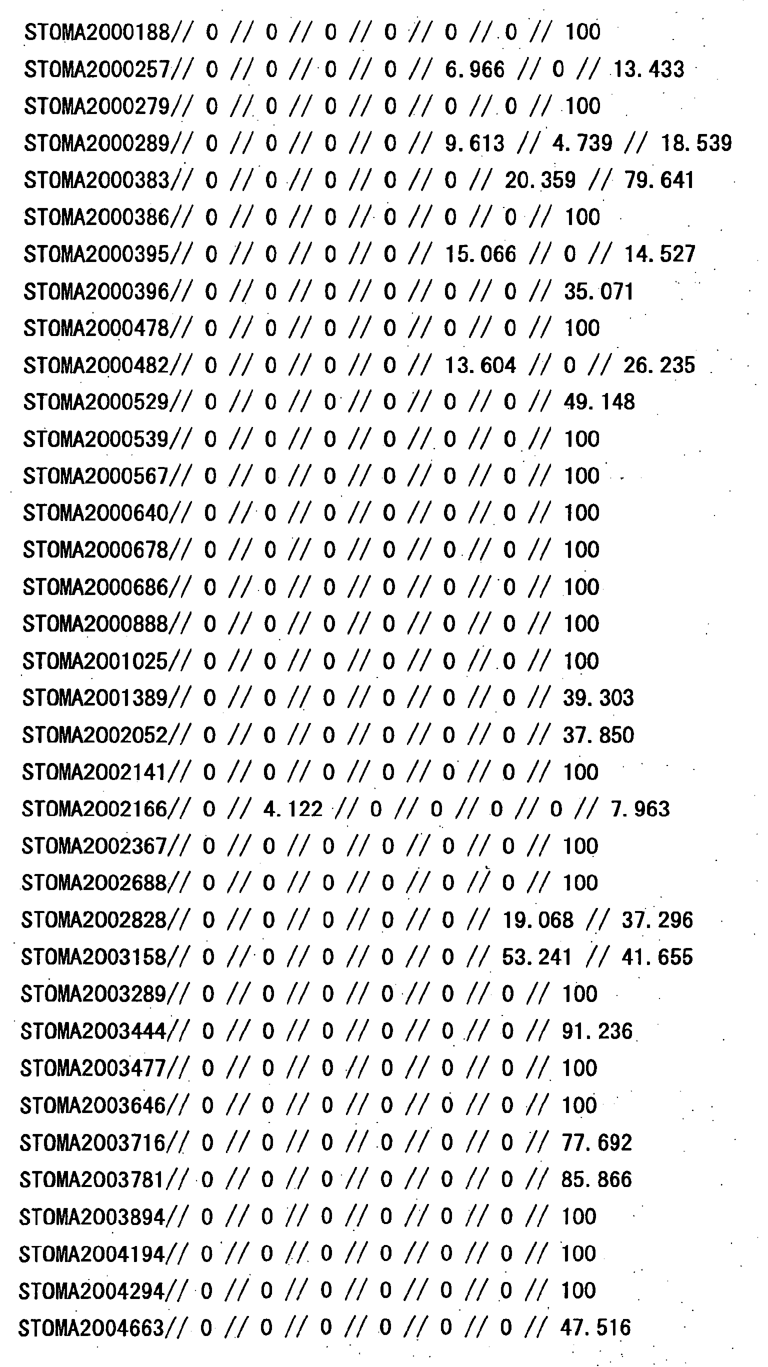

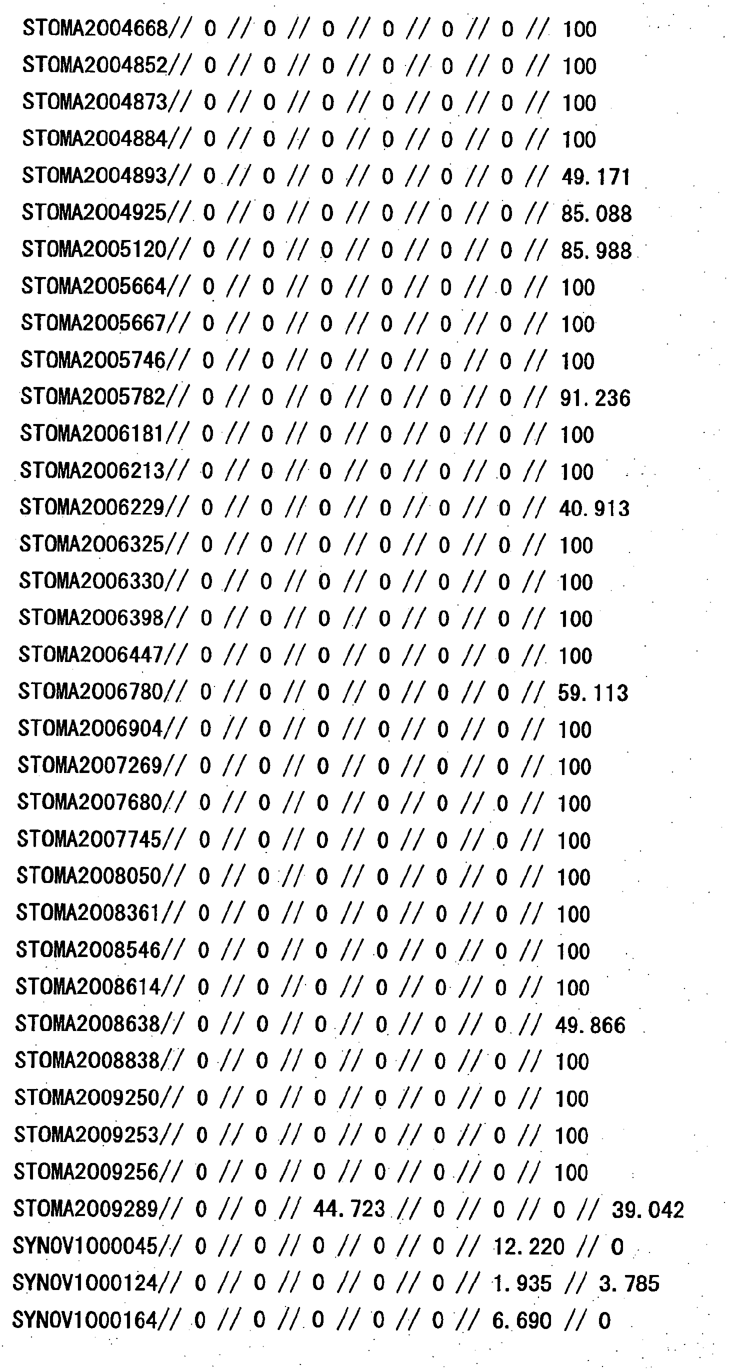

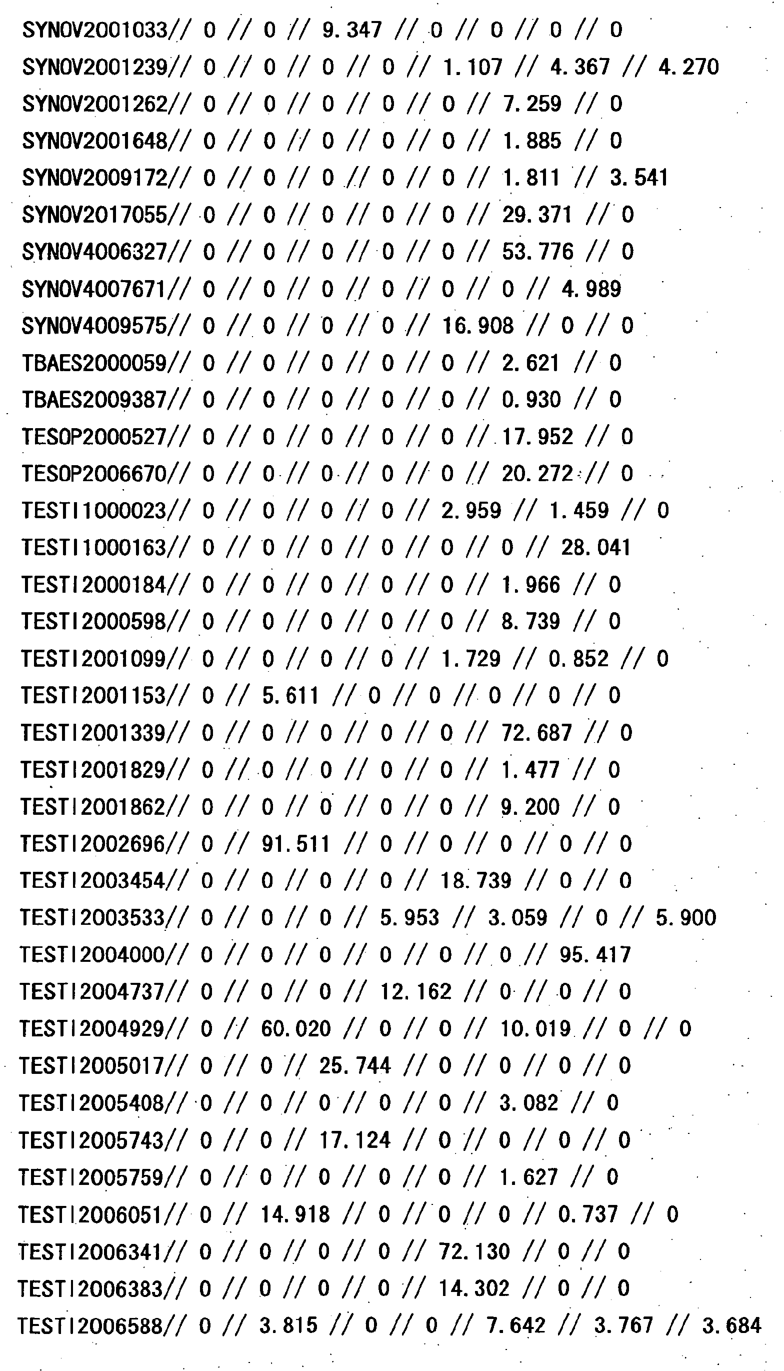

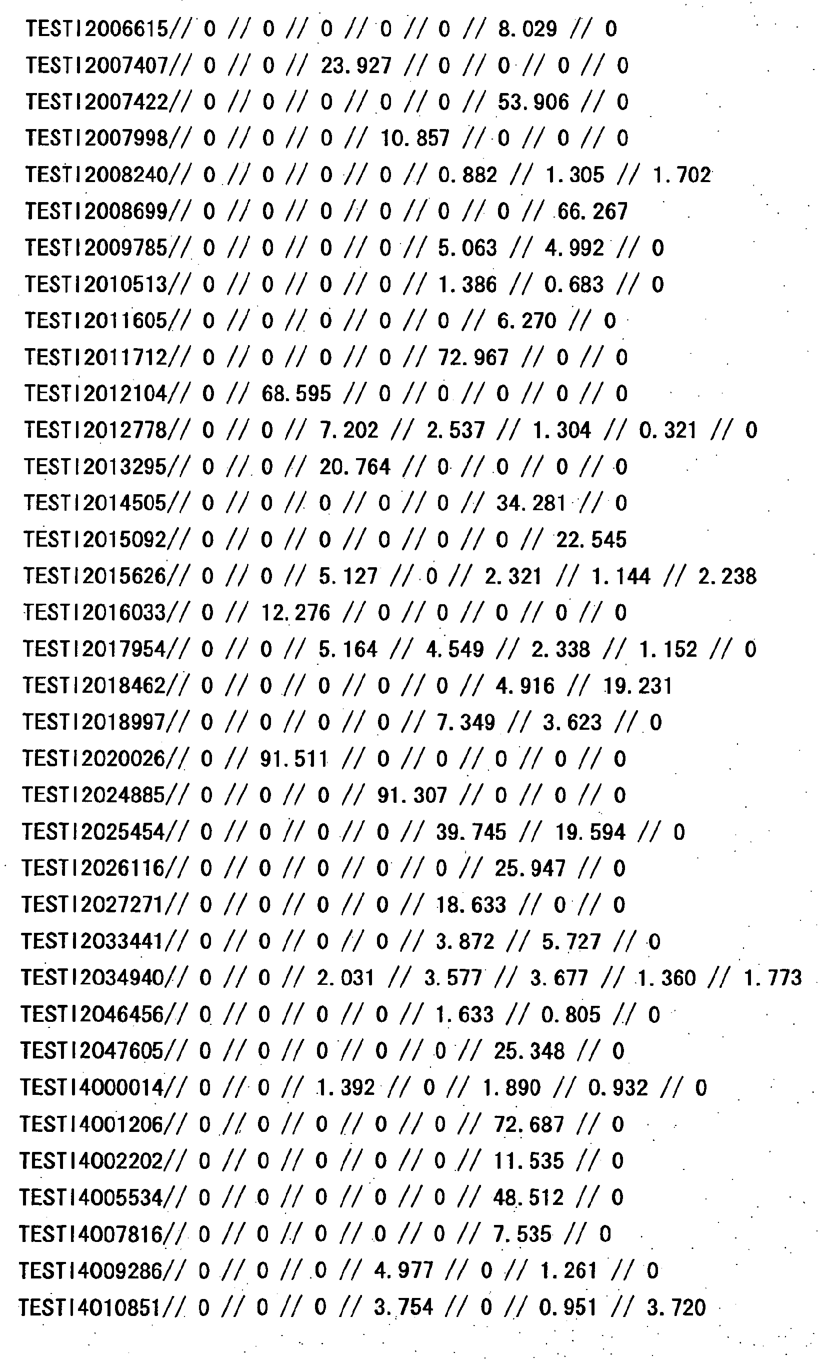

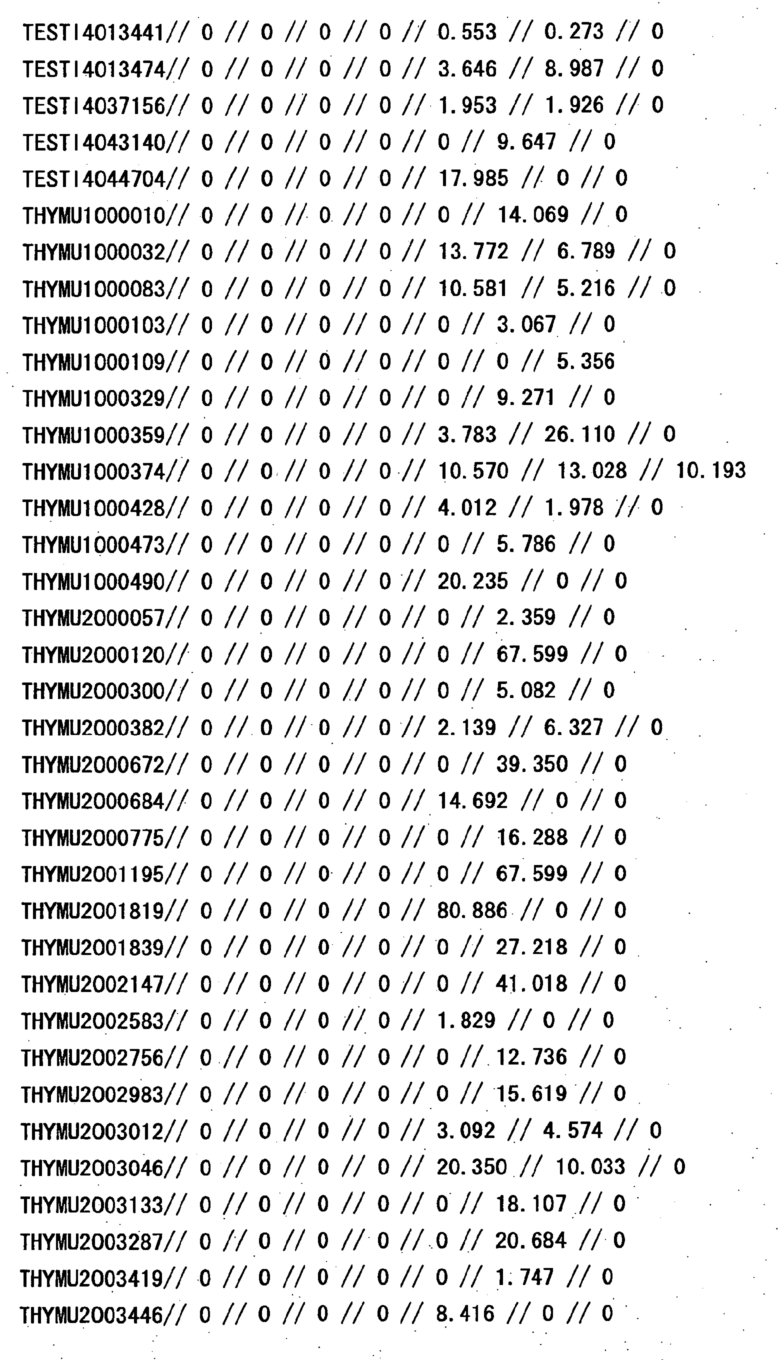

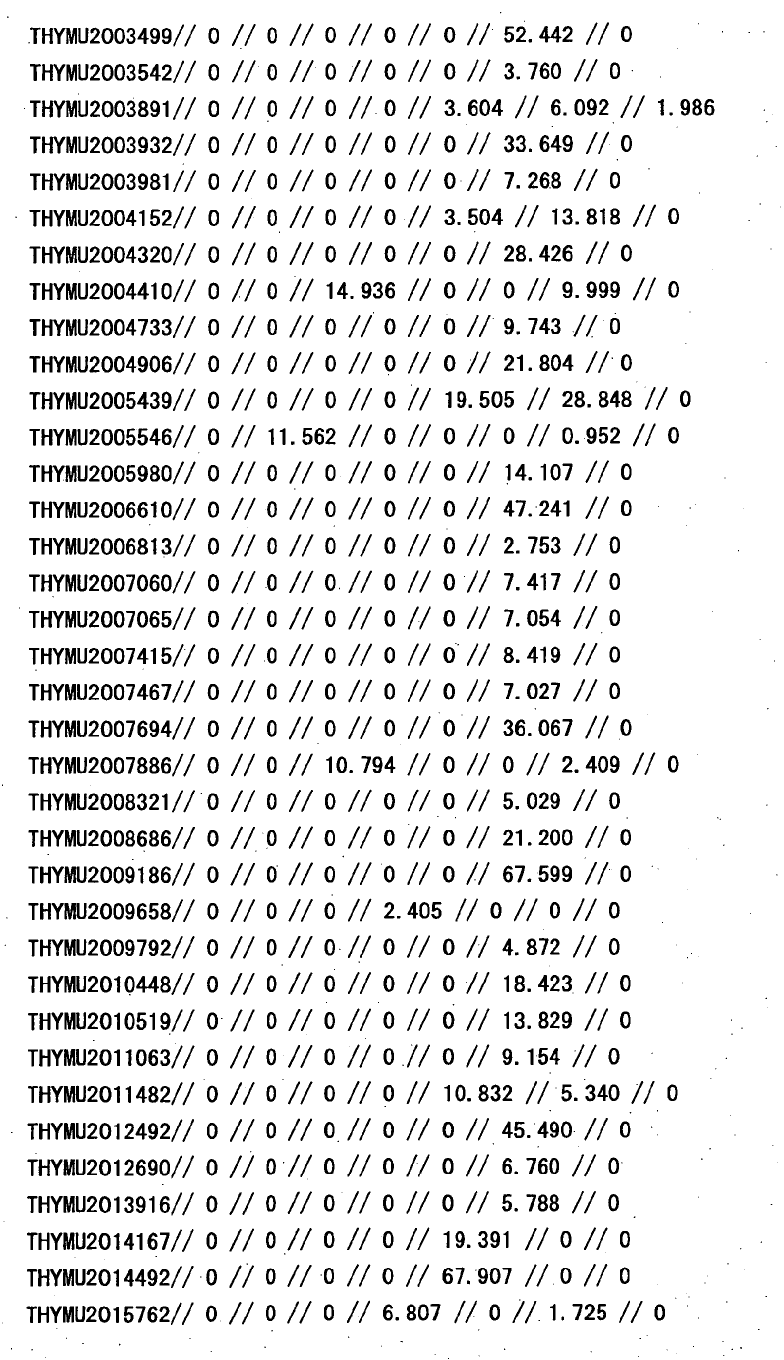

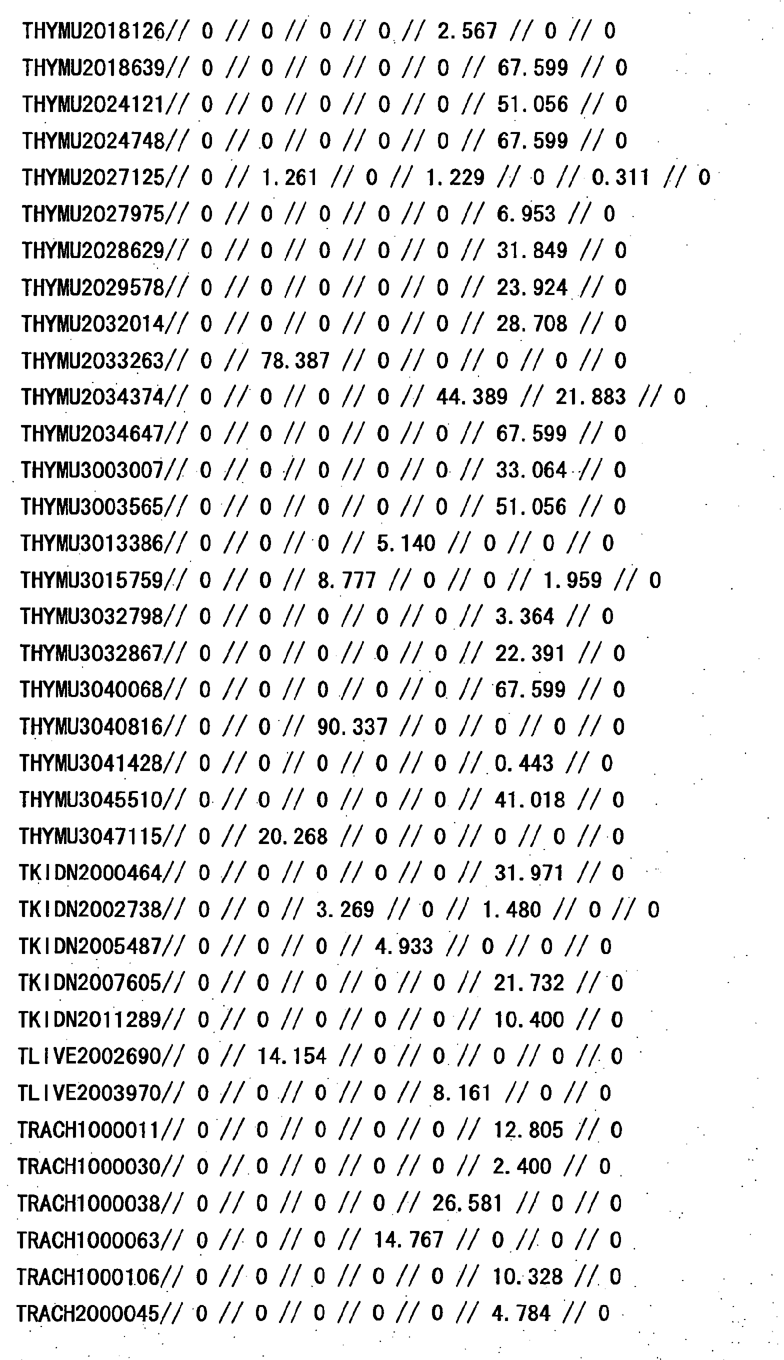

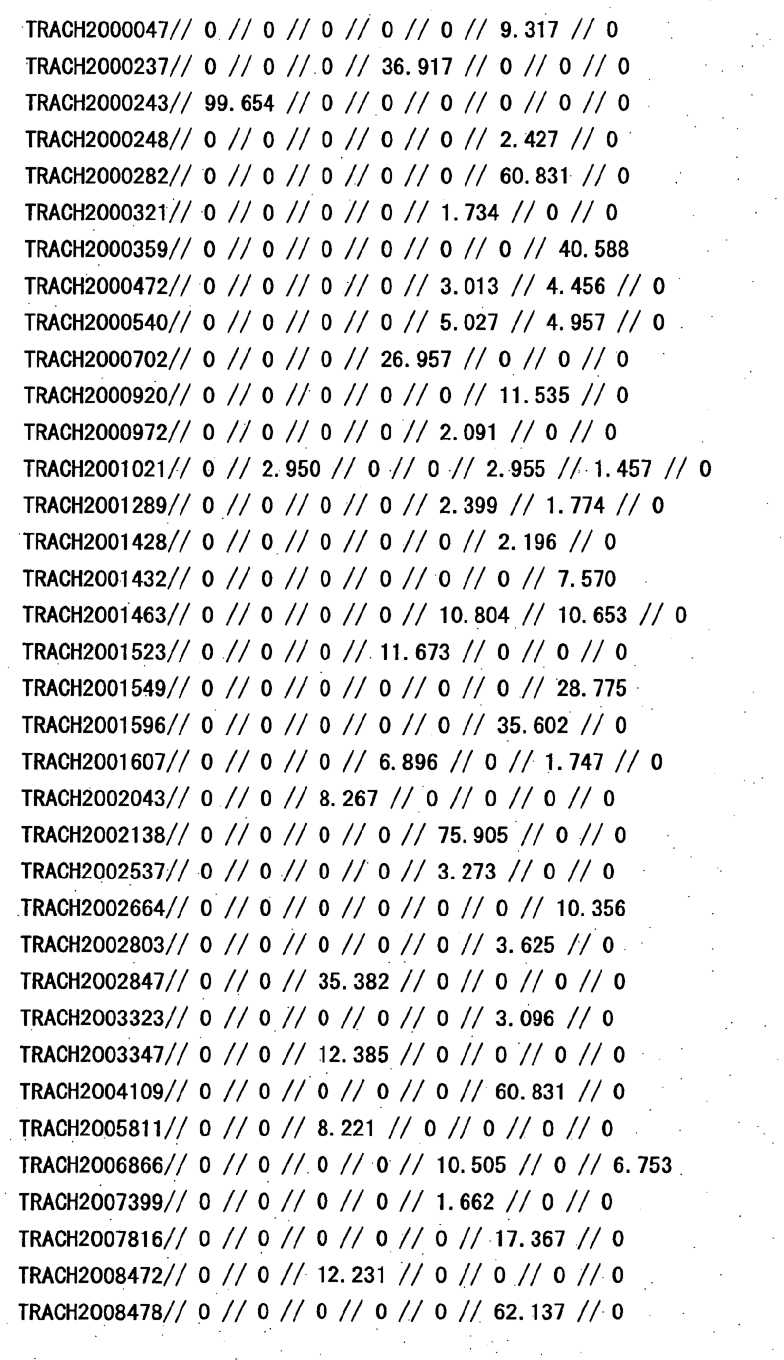

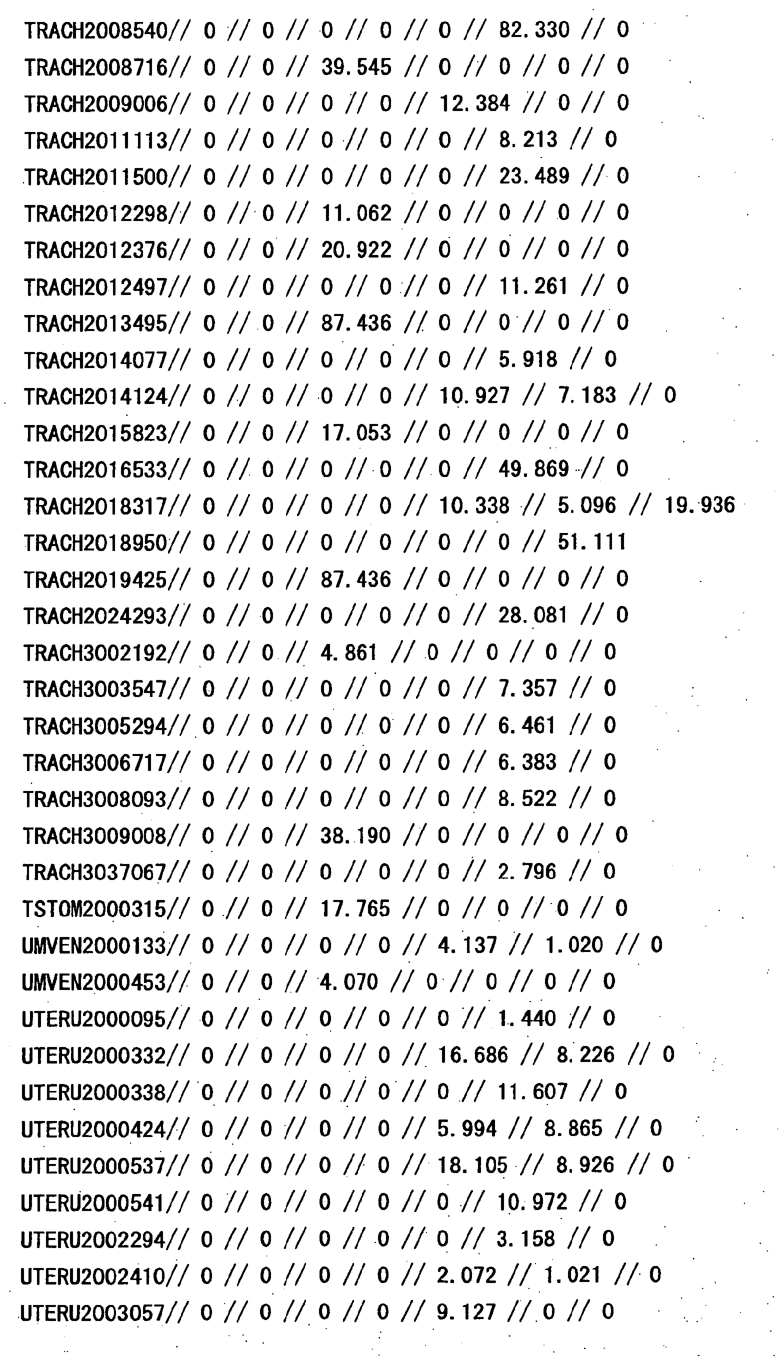

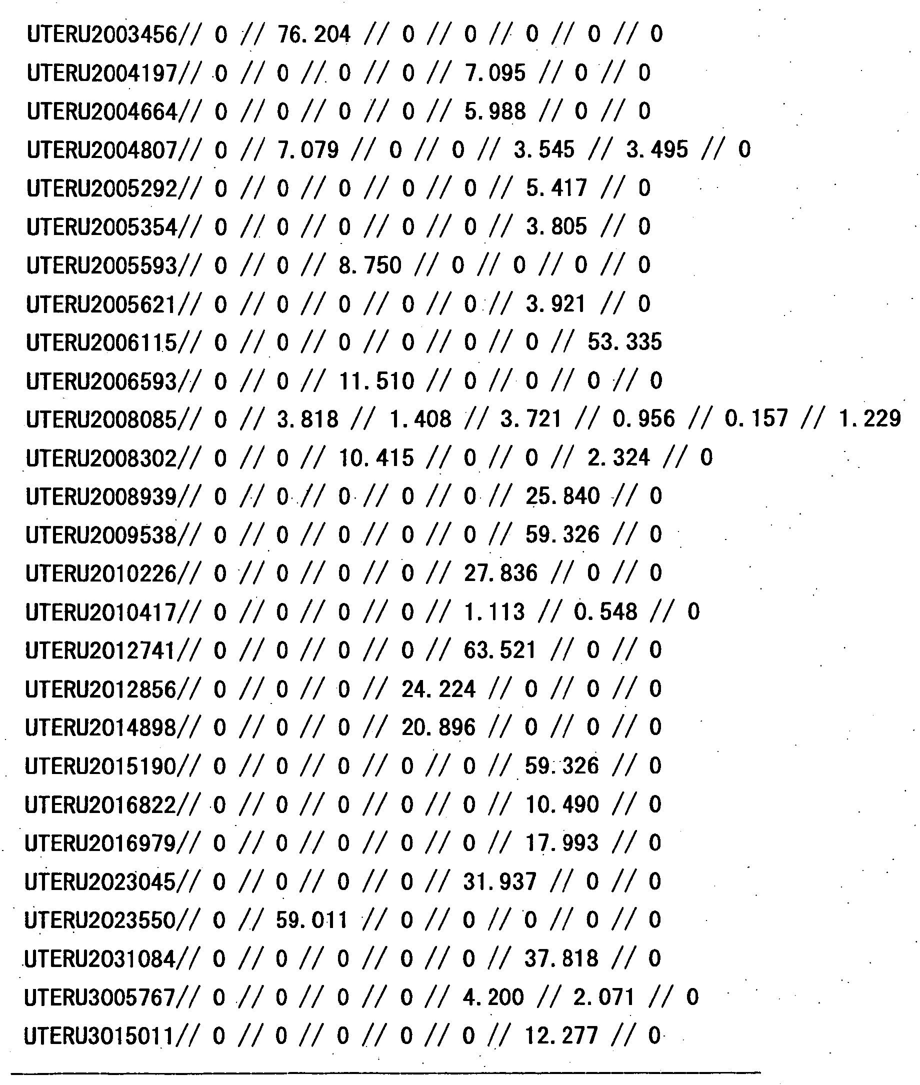

Genes whose expression levels were negligibly low in each of the above-described libraries are shown with their clone names below.
3NB691000018, 3NB691000085, 3NB691000113, 3NB691000129, 3NB691000191, 3NB692000012, 3NB692000029, 3NB692000102, 3NB692000154, 3NB692000276, 3NB692000305, 3NB692000374, 3NB692000429, 3NB692000484, 3NB692000529, 3NB692001034, 3NB692001040, 3NB692001123, 3NB692001267, 3NB692001288, 3NB692001349, 3NB692001366, 3NB692001408, 3NB692001433, 3NB692001471, 3NB692001496, 3NB692001501, 3NB692001507, 3NB692001511, 3NB692001519, 3NB692001520, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064,
ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000147, ADRGL1000160, ADRGL1000165, ADRGL1000182, ADRGL2000006, ADRGL2000042, ADRGL2000056, ADRGL2000064, ADRGL2000074, ADRGL2000085, ADRGL2000097, ADRGL2000142, ADRGL2000172, ADRGL2000248, ADRGL2000261, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002679,
ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023,
ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO2000011, ASTRO2000014, ASTRO2000046, ASTRO2000095, ASTRO2000141, ASTRO2000191, ASTRO2000278, ASTRO2000372, ASTRO2000381, ASTRO2000403, ASTRO2000417, ASTRO2000482, ASTRO2000533, ASTRO2000653, ASTRO2000662, ASTRO2000725, ASTRO2000801, ASTRO2001001, ASTRO2001008, ASTRO2001029, ASTRO2001088, ASTRO2001107, ASTRO2001122, ASTRO2001227, ASTRO2001480,
ASTRO2002024, ASTRO2002035, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003316, ASTRO2003461, ASTRO2003574, ASTRO2003581, ASTRO2003632, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005242, ASTRO2005273, ASTRO2005343, ASTRO2005413, ASTRO2005423, ASTRO2005557, ASTRO2005575, ASTRO2005593, ASTRO2005726, ASTRO2005863, ASTRO2005902, ASTRO2006356, ASTRO2006475, ASTRO2006732,
ASTRO2006747, ASTRO2006920, ASTRO2006952, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392,
ASTRO2014498, ASTRO2014561, ASTRO2014576, ASTRO2014863, ASTRO2014923, ASTRO2014961, ASTRO2015162, ASTRO2015185, ASTRO2015328, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2019039, ASTRO2019171, ASTRO3000154, ASTRO3000172, ASTRO3000177, ASTRO3000301, ASTRO3000474, ASTRO3000482, BEAST2000454, BGGI11000193, BGGI11000285, BGGI12000022, BGGI12000067, BGGI12000161, BGGI12000533, BGGI12000544, BGGI12000684, BGGI12000693, BGGI12001002, BGGI12001097, BGGI12001241, BGGI12001247, BGGI12001682, BLADE1000176, BLADE2000181, BLADE2000340, BLADE2000389, BLADE2000463,
BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007666, BLADE2007722,
BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000153, BNGH41000169, BNGH41000177, BNGH41000198, BNGH41000216, BNGH42000130, BNGH42000132, BNGH42000405, BNGH42000532, BNGH42000675, BNGH42000791, BNGH42000815, BNGH42000994, BNGH42001247, BNGH42001406, BNGH42001724, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504,
BNGH42005968, BNGH42006135, BNGH42006226, BNGH42006234, BNGH42007037, BNGH42007460, BNGH42007594, BNGH42007788, BNGH42007798, BNGH42008199, BNGH42008510, BNGH42008603, BNGH42008649, BNGH42008743, BNGH42008832, BNGH42008850, BNGH42008943, BNGH42009021, BRACE1000020, BRACE1000047, BRACE1000065, BRACE1000070, BRACE1000073, BRACE1000084, BRACE1000087, BRACE1000092, BRACE1000093, BRACE1000101, BRACE1000114, BRACE1000124, BRACE1000132, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229,
BRACE2000232, BRACE2000245, BRACE2000256, BRACE2000266, BRACE2000280, BRACE2000300, BRACE2000331, BRACE2000351, BRACE2000368, BRACE2000392, BRACE2000441, BRACE2000487, BRACE2000489, BRACE2000505, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000815, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001143, BRACE2001154, BRACE2001188, BRACE2001191, BRACE2001197, BRACE2001236,
BRACE2001239, BRACE2001269, BRACE2001312, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001677, BRACE2001692, BRACE2001705, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972, BRACE2001973,
BRACE2002026, BRACE2002034, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002812,
BRACE2002834, BRACE2002849, BRACE2002857, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003269, BRACE2003285, BRACE2003304, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003825, BRACE2003847, BRACE2003848, BRACE2003873, BRACE2003885, BRACE2003887, BRACE2003905, BRACE2003928, BRACE2003982, BRACE2003987,
BRACE2005034, BRACE2005047, BRACE2005063, BRACE2005083, BRACE2005090, BRACE2005099, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005328, BRACE2005329, BRACE2005337, BRACE2005348, BRACE2005363, BRACE2005395, BRACE2005408, BRACE2005433, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787,
BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006072, BRACE2006075, BRACE2006084, BRACE2006089, BRACE2006102, BRACE2006105, BRACE2006122, BRACE2006137, BRACE2006143, BRACE2006167, BRACE2006174, BRACE2006240, BRACE2006245, BRACE2006249, BRACE2006274, BRACE2006281, BRACE2006298, BRACE2006319, BRACE2006344, BRACE2006354, BRACE2006378, BRACE2006380, BRACE2006393, BRACE2006413, BRACE2006417, BRACE2006453, BRACE2006488, BRACE2006534, BRACE2006540, BRACE2006547, BRACE2006564, BRACE2006587, BRACE2006636, BRACE2006685, BRACE2006703,
BRACE2006743, BRACE2006753, BRACE2006783, BRACE2006821, BRACE2006833, BRACE2006859, BRACE2006871, BRACE2006909, BRACE2006911, BRACE2006924, BRACE2006935, BRACE2006937, BRACE2006944, BRACE2006951, BRACE2006982, BRACE2006986, BRACE2007013, BRACE2007087, BRACE2007138, BRACE2007174, BRACE2007197, BRACE2007201, BRACE2007203, BRACE2007232, BRACE2007262, BRACE2007274, BRACE2007281, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563, BRACE2007577, BRACE2007621, BRACE2007640, BRACE2007641, BRACE2007646, BRACE2007663, BRACE2007682, BRACE2007685, BRACE2007727, BRACE2007749,
BRACE2007760, BRACE2007761, BRACE2007764, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008218, BRACE2008251, BRACE2008276, BRACE2008295, BRACE2008308, BRACE2008336, BRACE2008361, BRACE2008380, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008594, BRACE2008604,
BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2009014, BRACE2009016, BRACE2009031, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009421, BRACE2009428, BRACE2009434, BRACE2009437, BRACE2009483,
BRACE2009533, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011747,
BRACE2011838, BRACE2011892, BRACE2011904, BRACE2011947, BRACE2012062, BRACE2012185, BRACE2012222, BRACE2012232, BRACE2012291, BRACE2012317, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2014108, BRACE2014161, BRACE2014232, BRACE2014257, BRACE2014268, BRACE2014306, BRACE2014475, BRACE2014657, BRACE2014746, BRACE2014780, BRACE2014821, BRACE2014824, BRACE2014850, BRACE2014854, BRACE2015031, BRACE2015058, BRACE2015132, BRACE2015262, BRACE2015270, BRACE2015314, BRACE2015352, BRACE2015368, BRACE2015412, BRACE2015436, BRACE2015741,
BRACE2015756, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019258, BRACE2019348, BRACE2019467,
BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021148, BRACE2021245, BRACE2021254, BRACE2021264, BRACE2021344, BRACE2021433, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022394, BRACE2022435, BRACE2022448, BRACE2022450, BRACE2022549, BRACE2022633, BRACE2022693, BRACE2022848,
BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026920, BRACE2026971, BRACE2027018, BRACE2027221, BRACE2027258, BRACE2027312, BRACE2027382, BRACE2027389, BRACE2027408, BRACE2027643, BRACE2027767,
BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786,
BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138,
BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492,
BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004371, BRACE3004435, BRACE3004477,
BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384,
BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005,
BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE-3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805,
BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447,
BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856,
BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834,
BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168,
BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000277, BRAMY2000388, BRAMY2000411, BRAMY2000508, BRAMY2000562, BRAMY2000585, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311,
BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004363, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182,
BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105,
BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162,
BRAMY2012163, BRAMY2012277, BRAMY2012340, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015855, BRAMY2015890,
BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2019989, BRAMY2020050, BRAMY2020058, BRAMY2020106,
BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238,
BRAMY2023327, BRAMY2023358, BRAMY2023406, BRAMY2023482, BRAMY2023514, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538,
BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028740, BRAMY2028783, BRAMY2028796, BRAMY2028856, BRAMY2028914, BRAMY2029204, BRAMY2029602, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859,
BRAMY2030915, BRAMY2031317, BRAMY2031377, BRAMY2031442, BRAMY2031516, BRAMY2032014, BRAMY2032087, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396,
BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244,
BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409,
BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158,
BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRASW1000053, BRASW1000125, BRAWH1000001, BRAWH1000002, BRAWH1000007, BRAWH1000037, BRAWH1000045, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131,
BRAWH2000142, BRAWH2000169, BRAWH2000218, BRAWH2000232, BRAWH2000248, BRAWH2000256, BRAWH2000274, BRAWH2000323, BRAWH2000353, BRAWH2000370, BRAWH2000409, BRAWH2000443, BRAWH2000471, BRAWH2000476, BRAWH2000482, BRAWH2000488, BRAWH2000494, BRAWH2000500, BRAWH2000503, BRAWH2000554, BRAWH2000595, BRAWH2000636, BRAWH2000643, BRAWH2000651, BRAWH2000682, BRAWH2000686, BRAWH2000697, BRAWH2000752, BRAWH2000859, BRAWH2000866, BRAWH2000892, BRAWH2000901, BRAWH2000926, BRAWH2000984, BRAWH2001019, BRAWH2001037, BRAWH2001052, BRAWH2001129, BRAWH2001137, BRAWH2001159, BRAWH2001171, BRAWH2001203, BRAWH2001238, BRAWH2001239, BRAWH2001255, BRAWH2001355, BRAWH2001378, BRAWH2001395, BRAWH2001406, BRAWH2001412,
BRAWH2001418, BRAWH2001424, BRAWH2001436, BRAWH2001438, BRAWH2001439, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002333, BRAWH2002352, BRAWH2002383, BRAWH2002549, BRAWH2002560, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003135,
BRAWH2003240, BRAWH2003307, BRAWH2003355, BRAWH2003366, BRAWH2003662, BRAWH2003689, BRAWH2003693, BRAWH2003832, BRAWH2003948, BRAWH2003964, BRAWH2004044, BRAWH2004068, BRAWH2004078, BRAWH2004136, BRAWH2004217, BRAWH2004731, BRAWH2004779, BRAWH2004842, BRAWH2004884, BRAWH2004923, BRAWH2005008, BRAWH2005068, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006450, BRAWH2006493, BRAWH2006526, BRAWH2006543, BRAWH2006622, BRAWH2006760, BRAWH2006960, BRAWH2006989, BRAWH2007069, BRAWH2007308, BRAWH2007406, BRAWH2007550, BRAWH2007570,
BRAWH2007591, BRAWH2007658, BRAWH2007664, BRAWH2007808, BRAWH2007825, BRAWH2007862, BRAWH2007890, BRAWH2008058, BRAWH2008255, BRAWH2008297, BRAWH2008366, BRAWH2008380, BRAWH2008540, BRAWH2008577, BRAWH2008706, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536, BRAWH2010552, BRAWH2010584, BRAWH2010618, BRAWH2010619, BRAWH2010754,
BRAWH2010980, BRAWH2011066, BRAWH2011079, BRAWH2011096, BRAWH2011155, BRAWH2011166, BRAWH2011204, BRAWH2011294, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014511, BRAWH2014662, BRAWH2014717,
BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016160, BRAWH2016221, BRAWH2016223, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018601, BRAWH2018745,
BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002513, BRAWH3002574, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRANH3004335, BRAWH3004350, BRAWH3004453, BRAWH3004530, BRAWH3004666,
BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005,
BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900,
BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117,
BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923,
BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007426, BRCAN2007525, BRCAN2008494, BRCAN2008528, BRCAN2008701, BRCAN2008894, BRCAN2009156, BRCAN2009168,
BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016762, BRCAN2016814, BRCAN2017442, BRCAN2017905, BRCAN2018240, BRCAN2018269, BRCAN2018490, BRCAN2018667, BRCAN2018748, BRCAN2018935, BRCAN2019002,
BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026197, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460,
BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034,
BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010980, BRCOC2010994, BRCOC2011010, BRCOC2011398, BRCOC2011418, BRCOC2011506, BRCOC2011769, BRCOC2011996, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015944, BRCOC2016525, BRCOC2016661, BRCOC2017061, BRCOC2017652, BRCOC2017856, BRCOC2018775, BRCOC2019255, BRCOC2019549, BRCOC2019841, BRCOC2019896, BRCOC2019903,
BRCOC2019910, BRCOC2019934, BRCOC2020122, BRCOC2020142, BRHIP1000058, BRHIP1000072, BRHIP1000108, BRHIP1000129, BRHIP1000174, BRHIP1000258, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000553, BRHIP2000621, BRHIP2000691, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828,
BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007305, BRHIP2007307, BRHIP2007616, BRHIP2007690, BRHIP2007741, BRHIP2007928, BRHIP2007969, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120,
BRHIP2011199, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014228, BRHIP2014285, BRHIP2014291, BRHIP2014373, BRHIP2014386, BRHIP2014387, BRHIP2014954, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542,
BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019149, BRHIP2019177, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020842, BRHIP2020859, BRHIP2020930, BRHIP2021102, BRHIP2021289, BRHIP2021495, BRHIP2021615, BRHIP2021744, BRHIP2021858, BRHIP2021870, BRHIP2021929, BRHIP2022006, BRHIP2022054, BRHIP2022221, BRHIP2022228, BRHIP2022298, BRHIP2022326, BRHIP2022467, BRHIP2022708, BRHIP2022986,
BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024549, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283,
BRHIP2029393, BRHIP2029529, BRHIP2029643, BRHIP2029663, BRHIP3000060, BRHIP3000070, BRHIP3000081, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004416, BRHIP3004710, BRHIP3004725, BRHIP3004774, BRHIP3004786,
BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427,
BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020046, BRHIP3020155, BRHIP3020182,
BRHIP3020733, BRHIP3021019, BRHIP3021499, BRHIP3021534,
BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059,. BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715,
BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009378, BRSSN2009389, BRSSN2009395, BRSSN2009627, BRSSN2009630, BRSSN2009702,
BRSSN2010019, BRSSN2010110, BRSSN2010587, BRSSN2010596, BRSSN2010830, BRSSN2011262, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014424, BRSSN2014513, BRSSN2014556, BRSSN2014573, BRSSN2014610, BRSSN2014636, BRSSN2014685, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757,
BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416,
BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013354, BRSTN2013502, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016892, BRSTN2016918, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969,
BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005942, BRTHA2005956, BRTHA2006075, BRTHA2006146, BRTHA2006194, BRTHA2006232, BRTHA2006720,
BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335., BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562,
BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420,
BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264,
BRTHA2033320, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3004307, BRTHA3004432, BRTHA3004475, BRTHA3004502, BRTHA3004610, BRTHA3005046, BRTHA3005735,
BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547,
BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505,
BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001721, COLON2001829, COLON2001866, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733,
COLON2008105, COLON2009337, COLON2009499, CORDB1000140, CORDB2000061, CORDB2000127, CORDB2000541, CTONG1000009, CTONG1000020, CTONG1000022, CTONG1000040, CTONG1000056, CTONG1000062, CTONG1000086, CTONG1000087, CTONG1000088, CTONG1000093, CTONG1000097, CTONG1000113, CTONG1000137, CTONG1000165, CTONG1000175, CTONG1000180, CTONG1000181, CTONG1000218, CTONG1000220, CTONG1000277, CTONG1000283, CTONG1000288, CTONG1000302, CTONG1000467, CTONG1000476, CTONG1000488, CTONG1000508, CTONG1000540, CTONG2000031, CTONG2000034, CTONG2000042, CTONG2000201, CTONG2000218, CTONG2000279, CTONG2000303, CTONG2000330, CTONG2000395, CTONG2000411, CTONG2000465, CTONG2000469, CTONG2000480, CTONG2000561, CTONG2000589,
CTONG2000766, CTONG2000771, CTONG2000819, CTONG2000827, CTONG2000846, CTONG2000855, CTONG2000977, CTONG2001139, CTONG2001320, CTONG2001366, CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001955, CTONG2002066, CTONG2002073, CTONG2002095, CTONG2002143, CTONG2002395, CTONG2002417, CTONG2002418, CTONG2002453, CTONG2002520, CTONG2002590, CTONG2002709, CTONG2002721, CTONG2002766, CTONG2002803, CTONG2002816, CTONG2002820, CTONG2002832, CTONG2002841, CTONG2002903, CTONG2002965, CTONG2002999, CTONG2003028, CTONG2003094, CTONG2003100,
CTONG2003115, CTONG2003189, CTONG2003245, CTONG2003293, CTONG2003348, CTONG2003350, CTONG2003372, CTONG2003375, CTONG2003427, CTONG2003517, CTONG2003524, CTONG2003599, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005290, CTONG2005374, CTONG2005399, CTONG2005468, CTONG2005567, CTONG2005585, CTONG2005615, CTONG2005635, CTONG2005775, CTONG2005795, CTONG2005890,
CTONG2005904, CTONG2005913, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006568, CTONG2006620, CTONG2006666, CTONG2006691, CTONG2006798, CTONG2006836, CTONG2006942, CTONG2006964, CTONG2007009, CTONG2007072, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321,
CTONG2008343, CTONG2008398, CTONG2008402, CTONG2008405, CTONG2008466, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958,
CTONG2009963, CTONG2010018, CTONG2010024, CTONG2010116, CTONG2010133, CTONG2010148, CTONG2010276, CTONG2010330, CTONG2010508, CTONG2010566, CTONG2010623, CTONG2010649, CTONG2010652, CTONG2010803, CTONG2010821, CTONG2011188, CTONG2011429, CTONG2011474, CTONG2011676, CTONG2011765, CTONG2011779, CTONG2011801, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012158, CTONG2012230, CTONG2012401, CTONG2012422, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758,
CTONG2012843, CTONG2012879, CTONG2012901, CTONG2012996, CTONG2013128, CTONG2013149, CTONG2013156, CTONG2013178, CTONG2013222, CTONG2013284, CTONG2013339, CTONG2013352, CTONG2013381, CTONG2013393, CTONG2013511, CTONG2013630, CTONG2013803, CTONG2013934, CTONG2013986, CTONG2014032, CTONG2014058, CTONG2014119, CTONG2014191, CTONG2014206, CTONG2014234, CTONG2014264, CTONG2014280, CTONG2014369, CTONG2014491, CTONG2014630, CTONG2014635, CTONG2014646, CTONG2014705, CTONG2014898, CTONG2014946, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717,
CTONG2015804, CTONG2015866, CTONG2015914, CTONG2015953, CTONG2016056, CTONG2016217, CTONG2016355, CTONG2016408, CTONG2016499, CTONG2016505, CTONG2016559, CTONG2016658, CTONG2016775, CTONG2016797, CTONG2016824, CTONG2016846, CTONG2016904, CTONG2016931, CTONG2016942, CTONG2016953, CTONG2017021, CTONG2017094, CTONG2017292, CTONG2017409, CTONG2017429, CTONG2017458, CTONG2017500, CTONG2017604, CTONG2017650, CTONG2017798, CTONG2017804, CTONG2017814, CTONG2017910, CTONG2017939, CTONG2017989, CTONG2018062, CTONG2018069, CTONG2018147, CTONG2018217, CTONG2018279, CTONG2018334, CTONG2018343, CTONG2018379, CTONG2018413, CTONG2018483, CTONG2018629, CTONG2018632, CTONG2018652, CTONG2018653, CTONG2018655,
CTONG2018735, CTONG2018739, CTONG2018808, CTONG2018843, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020411, CTONG2020445, CTONG2020484, CTONG2020522, CTONG2020560, CTONG2020582, CTONG2020638, CTONG2020806, CTONG2020831, CTONG2020974, CTONG2021024, CTONG2021132, CTONG2021168, CTONG2022153, CTONG2022601, CTONG2022835, CTONG2023021, CTONG2023449, CTONG2023512, CTONG2024031, CTONG2024206, CTONG2024614, CTONG2024749, CTONG2025496, CTONG2025513, CTONG2025516,
CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028208, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553,
CTONG3003598, CTONG3003652, CTONG3003654, CTONG3003669, CTONG3003737, CTONG3003905, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009287, CTONG3009328, CTONG3009385, D3OST1000066, D3OST1000109, D3OST1000238, D3OST1000267, D3OST2000184,
D3OST2000618, D3OST2000654, D3OST2000734, D3OST2001328, D3OST2001669, D3OST2001670, D3OST2001788, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D60ST2000245, D6OST2000358, D6OST2000464, D6OST2000507, D9OST2000031, D9OST2000278, D9OST2000440, D9OST2000869, D9OST2001319, D9OST2001433, D9OST2002397, D9OST2002673, D9OST2003106, D9OST2003108, D9OST2003137, D9OST2003397, D9OST2003580, D9OST2003594, D9OST2003762, D9OST2003791, D9OST2003989, D9OST2004018, D9OST2004073,
D9OST2004417, DFNES1000003, DFNES1000107, DFNES1000185,
DFNES2000011, DFNES2000143, DFNES2000146, DFNES2000212, DFNES2000268, DFNES2000292, DFNES2000335, DFNES2000426, DFNES2000432, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003192, DFNES2003255, DFNES2003742, DFNES2004346, DFNES2004354, DFNES2004371, DFNES2004383, DFNES2004608, DFNES2004684, DFNES2005266, DFNES2005527, DFNES2005540, DFNES2005690, DFNES2005779, DFNES2005875, DFNES2006944, DFNES2007113,
DFNES2007299, DFNES2007332, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000024, FCBBF1000025, FCBBF1000038, FCBBF1000053, FCBBF1000061, FCBBF1000063, FCBBF1000069, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305,
FCBBF1000314, FCBBF1000322, FCBBF1000349, FCBBF1000374, FCBBF1000376, FCBBF1000377, FCBBF1000395, FCBBF1000397, FCBBF1000425, FCBBF1000449, FCBBF1000476, FCBBF1000506, FCBBF1000509, FCBBF1000546, FCBBF1000550, FCBBF1000556, FCBBF1000563, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000748, FCBBF2000038, FCBBF2000087, FCBBF2000094, FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000677, FCBBF2000678,
FCBBF2000685, FCBBF2000733, FCBBF2000782, FCBBF2000808, FCBBF2000815, FCBBF2000825, FCBBF2000885, FCBBF2000940, FCBBF2000951, FCBBF2001001, FCBBF2001088, FCBBF2001105, FCBBF2001116, FCBBF2001183, FCBBF2001188, FCBBF2001238, FCBBF2001243, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002897, FCBBF2002898, FCBBF2002904, FCBBF2002953, FCBBF2003083, FCBBF2003102, FCBBF2003269, FCBBF2003293, FCBBF2003297, FCBBF2003395, FCBBF2003528, FCBBF2003543,
FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000120, FCBBF3000168, FCBBF3000175, FCBBF3000228, FCBBF3000229, FCBBF3000233, FCBBF3000269, FCBBF3000361, FCBBF3000367, FCBBF3000434,
FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001632, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390,
FCBBF3004409, FCBBF3004502, FCBBF3004715, FCBBF3004842, FCBBF3004847, FCBBF3004955, FCBBF3005160, FCBBF3005218, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008944, FCBBF3009069, FCBBF3009101, FCBBF3009152,
FCBBF3009256, FCBBF3009317, FCBBF3009428, FCBBF3009464, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341,
FCBBF3013506, FCBBF3013623, FCBBF3013800, FCBBF3013846, FCBBF3014229, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018826, FCBBF3018949, FCBBF3019085, FCBBF3019320, FCBBF3019437,
FCBBF3019455, FCBBF3019564, FCBBF3019569, FCBBF3019570, FCBBF3019663, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911,
FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000529,
FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000030, FEBRA1000057, FEBRA1000088, FEBRA1000138, FEBRA1000148, FEBRA1000149, FEBRA1000183, FEBRA1000188, FEBRA1000189, FEBRA2000007, FEBRA2000021, FEBRA2000035, FEBRA2000053, FEBRA2000126, FEBRA2000220, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000454, FEBRA2000462, FEBRA2000468, FEBRA2000491,
FEBRA2000504, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000757, FEBRA2000771, FEBRA2000772, FEBRA2000782, FEBRA2000790, FEBRA2000793, FEBRA2000803, FEBRA2000805, FEBRA2000809, FEBRA2000815, FEBRA2000856, FEBRA2000862, FEBRA2000877, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001334, FEBRA2001351,
FEBRA2001383, FEBRA2001438, FEBRA2001440, FEBRA2001487, FEBRA2001561, FEBRA2001584, FEBRA2001590, FEBRA2001591, FEBRA2001610, FEBRA2001620, FEBRA2001659, FEBRA2001698, FEBRA2001705, FEBRA2001706, FEBRA2001715, FEBRA2001745, FEBRA2001752, FEBRA2001772, FEBRA2001790, FEBRA2001814, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002399, FEBRA2002410, FEBRA2002429, FEBRA2002442, FEBRA2002456, FEBRA2002508, FEBRA2002525, FEBRA2002527, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783,
FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003275, FEBRA2003308, FEBRA2003327, FEBRA2003429, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003822, FEBRA2003833, FEBRA2003897, FEBRA2003907, FEBRA2004023, FEBRA2004026, FEBRA2004029, FEBRA2004053, FEBRA2004056, FEBRA2004091, FEBRA2004110, FEBRA2004191, FEBRA2004202, FEBRA2004219, FEBRA2004237, FEBRA2004268, FEBRA2004325, FEBRA2004329, FEBRA2004331,
FEBRA2004443, FEBRA2004485, FEBRA2004490, FEBRA2004538, FEBRA2004592, FEBRA2004620, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005701, FEBRA2005726, FEBRA2005752, FEBRA2005787, FEBRA2005995, FEBRA2005998, FEBRA2006055, FEBRA2006061, FEBRA2006112, FEBRA2006150, FEBRA2006165, FEBRA2006255, FEBRA2006315, FEBRA2006354, FEBRA2006357, FEBRA2006368, FEBRA2006370, FEBRA2006372, FEBRA2006379, FEBRA2006396, FEBRA2006409,
FEBRA2006418, FEBRA2006427, FEBRA2006476, FEBRA2006485, FEBRA2006519, FEBRA2006521, FEBRA2006553, FEBRA2006627, FEBRA2006664, FEBRA2006667, FEBRA2006726, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007708, FEBRA2007714, FEBRA2007793, FEBRA2007818, FEBRA2007823, FEBRA2007880, FEBRA2007900, FEBRA2007931, FEBRA2007957, FEBRA2008009, FEBRA2008036, FEBRA2008086, FEBRA2008134, FEBRA2008135, FEBRA2008159,
FEBRA2008165, FEBRA2008172, FEBRA2008255, FEBRA2008265, FEBRA2008266, FEBRA2008282, FEBRA2008287, FEBRA2008311, FEBRA2008360, FEBRA2008459, FEBRA2008468, FEBRA2008481, FEBRA2008542, FEBRA2008660, FEBRA2008662, FEBRA2008681, FEBRA2008692, FEBRA2008741, FEBRA2008836, FEBRA2008859, FEBRA2008861, FEBRA2008866, FEBRA2008881, FEBRA2008928, FEBRA2008983, FEBRA2009016, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009437, FEBRA2009478, FEBRA2009507, FEBRA2009514, FEBRA2009567, FEBRA2009588, FEBRA2009731,
FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572,
FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019867, FEBRA2020406, FEBRA2020566, FEBRA2020582, FEBRA2020668, FEBRA2020801, FEBRA2020845, FEBRA2020886, FEBRA2021032, FEBRA2021134, FEBRA2021171, FEBRA2021339, FEBRA2021431, FEBRA2021497, FEBRA2021550, FEBRA2021571, FEBRA2021864,
FEBRA2021892, FEBRA2021908, FEBRA2022013, FEBRA2022148, FEBRA2022204, FEBRA2022255, FEBRA2022322, FEBRA2022504, FEBRA2022601, FEBRA2022696, FEBRA2022729, FEBRA2022787, FEBRA2022911, FEBRA2022956, FEBRA2022963, FEBRA2022972, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025463, FEBRA2025477, FEBRA2025838, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297,
FEBRA2027352, FEBRA2027364, FEBRA2027609, FEBRA2028158, FEBRA2028222, FEBRA2028366, FEBRA2028457, FEBRA2028477, FEBRA2028503, FEBRA2028516, FEHRT2000325, FEKID1000018, FELIV1000101, FELIV1000137, FELIV1000164, FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131,
HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000142, HCHON1000176, HCHON1000191, HCHON2000038, HCHON2000062, HCHON2000100, HCHON2000156, HCHON2000199, HCHON2000212, HCHON2000265, HCHON2000271, HCHON2000315, HCHON2000322, HCHON2000344, HCHON2000364, HCHON2000395, HCHON2000417, HCHON2000418, HCHON2000473, HCHON2000503, HCHON2000508, HCHON2000660, HCHON2000663, HCHON2000673, HCHON2000676, HCHON2000698, HCHON2000699, HCHON2000705, HCHON2000743, HCHON2000815, HCHON2000817, HCHON2000818, HCHON2000819, HCHON2000832, HCHON2000851, HCHON2000898, HCHON2000935,
HCHON2000942, HCHON2000956, HCHON2000994, HCHON2001050, HCHON2001084, HCHON2001099, HCHON2001108, HCHON2001110, HCHON2001116, HCHON2001161, HCHON2001214, HCHON2001233, HCHON2001269, HCHON2001434, HCHON2001450, HCHON2001453, HCHON2001497, HCHON2001519, HCHON2001523, HCHON2001535, HCHON2001546, HCHON2001548, HCHON2001577, HCHON2001598, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003659, HCHON2003676, HCHON2004007,
HCHON2004279, HCHON2004359, HCHON2004531, HCHON2004776, HCHON2004858, HCHON2005048, HCHON2005118, HCHON2005166, HCHON2005802, HCHON2005921, HCHON2005987, HCHON2006459, HCHON2006714, HCHON2006722, HCHON2006770, HCHON2006786, HCHON2006841, HCHON2006871, HCHON2007046, HCHON2007482, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000088, HEART1000099, HEART1000102, HEART1000118, HEART1000139, HEART1000142, HEART1000151, HEART1000173, HEART2000024, HEART2000035, HEART2000099, HEART2000298,
HEART2000306, HEART2000309, HEART2000411, HEART2000418, HEART2000448, HEART2000492, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2004570, HEART2004764, HEART2004931, HEART2004940, HEART2004941, HEART2004980, HEART2005244, HEART2005310, HEART2005581, HEART2005610, HEART2006131, HEART2006310, HEART2006334, HEART2006487, HEART2006557, HEART2006787, HEART2006789, HEART2006909, HEART2007031, HEART2007231, HEART2007443, HEART2007696, HEART2007767, HEART2008108, HEART2008111, HEART2008257,
HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000114, HHDPC1000118, HHDPC2000070, HHDPC2000102, HHDPC2000104, HHDPC2000258, HHDPC2000315, HHDPC2000455, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114,
HHDPC2009178, HHDPC2009208, HHDPC2009272, HHDPC2009528, HLUNG1000017, HLUNG1000024, HLUNG1000030, HLUNG1000034, HLUNG1000053, HLUNG1000076, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926,
HLUNG2000955, HLUNG2000970, HLUNG2001013, HLUNG2001126, HLUNG2001144, HLUNG2001146, HLUNG2001214, HLUNG2001459, HLUNG2001507, HLUNG2001633, HLUNG2001677, HLUNG2001712, HLUNG2001814, HLUNG2001996, HLUNG2002006, HLUNG2002050, HLUNG2002059, HLUNG2002085, HLUNG2002295, HLUNG2002303, HLUNG2002405, HLUNG2002429, HLUNG2002562, HLUNG2002659, HLUNG2002707, HLUNG2002811, HLUNG2002916, HLUNG2002942, HLUNG2002958, HLUNG2002982, HLUNG2003049, HLUNG2003061, HLUNG2003162, HLUNG2003246, HLUNG2003306, HLUNG2003335, HLUNG2003378, HLUNG2003497, HLUNG2003691, HLUNG2003710, HLUNG2003714, HLUNG2003716, HLUNG2003833, HLUNG2003872, HLUNG2003918, HLUNG2003991, HLUNG2004014, HLUNG2004037, HLUNG2004067, HLUNG2004154,
HLUNG2004170, HLUNG2004217, HLUNG2004273, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006852, HLUNG2006935, HLUNG2007041, HLUNG2007210, HLUNG2007219, HLUNG2007245, HLUNG2007433, HLUNG2007931, HLUNG2008066, HLUNG2008139, HLUNG2008153, HLUNG2008235, HLUNG2008247, HLUNG2008333, HLUNG2008348, HLUNG2008384, HLUNG2008396,
HLUNG2008439, HLUNG2008479, HLUNG2008480, HLUNG2008521, HLUNG2008833, HLUNG2008875, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017262, HLUNG2017286, HLUNG2017307,
HLUNG2017350, HLUNG2017546, HLUNG2017806, HLUNG2018029, HLUNG2018282, HLUNG2018626, HLUNG2019058, HSYRA1000131, HSYRA1000148, HSYRA1000152, HSYRA2000044, HSYRA2000135, HSYRA2000137, HSYRA2000221, HSYRA2000237, HSYRA2000253, HSYRA2000371, HSYRA2000424, HSYRA2000507, HSYRA2000510, HSYRA2000605, HSYRA2000629, HSYRA2000706, HSYRA2000760, HSYRA2000787, HSYRA2000792, HSYRA2000828, HSYRA2000832, HSYRA2000927, HSYRA2001003, HSYRA2001105, HSYRA2001142, HSYRA2001153, HSYRA2001232, HSYRA2001255, HSYRA2001332, HSYRA2001353, HSYRA2001484, HSYRA2001552, HSYRA2001580, HSYRA2001615, HSYRA2001621, HSYRA2001631, HSYRA2001638, HSYRA2002103, HSYRA2002590, HSYRA2002741, HSYRA2003168, HSYRA2004550, HSYRA2004858,
HSYRA2005456, HSYRA2005496, HSYRA2005628, HSYRA2005970, HSYRA2006049, HSYRA2006873, HSYRA2007241, HSYRA2007338, HSYRA2007667, HSYRA2008154, HSYRA2008714, HSYRA2009102, IMR321000155, IMR321000161, IMR321000165, IMR321000193, IMR321000207, IMR321000230, IMR321000266, IMR322000010, IMR322000107, IMR322000164, IMR322000379, IMR322000383, IMR322000418, IMR322000518, IMR322000555, IMR322000604, IMR322000609, IMR322000656, IMR322000672, IMR322000775, IMR322000791, IMR322000811, IMR322000819, IMR322000838, IMR322000859, IMR322000863, IMR322000917, IMR322000918, IMR322000919, IMR322000953, IMR322000984, IMR322001018, IMR322001167, IMR322001185, IMR322001245, IMR322001318, IMR322001322, IMR322001332,
IMR322001380, IMR322001452, IMR322001491, IMR322001534, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, IMR322019070, KIDNE1000008, KIDNE1000012, KIDNE1000015, KIDNE1000028, KIDNE1000036, KIDNE1000064,
KIDNE1000104, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000349, KIDNE2000375, KIDNE2000394, KIDNE2000403, KIDNE2000422, KIDNE2000493, KIDNE2000510, KIDNE2000513, KIDNE2000517, KIDNE2000519, KIDNE2000555, KIDNE2000624, KIDNE2000665, KIDNE2000678, KIDNE2000717, KIDNE2000721, KIDNE2000722, KIDNE2000801, KIDNE2000832, KIDNE2000846, KIDNE2000867, KIDNE2000947, KIDNE2001117, KIDNE2001140, KIDNE2001269, KIDNE2001361, KIDNE2001636, KIDNE2001713, KIDNE2001802,
KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002882, KIDNE2002980, KIDNE2002991, KIDNE2003185, KIDNE2003188, KIDNE2003233, KIDNE2003316, KIDNE2003335, KIDNE2003357, KIDNE2003373, KIDNE2003377, KIDNE2003752, KIDNE2003764, KIDNE2003837, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411,
KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005676, KIDNE2005781, KIDNE2005798, KIDNE2005854, KIDNE2005908, KIDNE2005937, KIDNE2006030, KIDNE2006053, KIDNE2006062, KIDNE2006092, KIDNE2006145, KIDNE2006149, KIDNE2006210, KIDNE2006299, KIDNE2006334, KIDNE2006376, KIDNE2006378, KIDNE2006465, KIDNE2006580, KIDNE2006652, KIDNE2006687,
KIDNE2006733, KIDNE2006760, KIDNE2006775, KIDNE2006820, KIDNE2006880, KIDNE2007040, KIDNE2007077, KIDNE2007222, KIDNE2007356, KIDNE2007422, KIDNE2007569, KIDNE2007810, KIDNE2007944, KIDNE2007954, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628,
KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346,
KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539,
KIDNE2016570, KIDNE2016918, KIDNE2017007, KIDNE2017040, KIDNE2017052, KIDNE2017151, KIDNE2017153, KIDNE2017315, KIDNE2017332, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018617, KIDNE2018678, KIDNE2018727, KIDNE2018863, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126, LIVER1000139, LIVER1000155, LIVER1000165, LIVER1000175, LIVER1000224, LIVER1000230, LIVER1000278,
LIVER1000303, LIVER1000397, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003581, LIVER2003800, LIVER2003854, LIVER2004074, LIVER2004392,
LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008465, LIVER2008473, LIVER2008580, LIVER2008706, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000182, MAMGL1000184, MESAN1000032, MESAN1000035, MESAN1000050, MESAN1000079,
MESAN1000080, MESAN1000101, MESAN1000121, MESAN1000147, MESAN1000180, MESAN2000023, MESAN2000026, MESAN2000067, MESAN2000092, MESAN2000167, MESAN2000291, MESAN2000337, MESAN2000434, MESAN2000457, MESAN2000462, MESAN2000572, MESAN2000620, MESAN2000711, MESAN2000815, MESAN2000894, MESAN2000909, MESAN2001450, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002724, MESAN2002790, MESAN2002844, MESAN2002978, MESAN2003035, MESAN2003037, MESAN2003039, MESAN2003058, MESAN2003101, MESAN2003190, MESAN2003322, MESAN2003490, MESAN2003529, MESAN2003622, MESAN2003646, MESAN2003662,
MESAN2003709, MESAN2003851, MESAN2004138, MESAN2004575, MESAN2005303, MESAN2005371, MESAN2005633, MESAN2005724, MESAN2005766, MESAN2005808, MESAN2005811, MESAN2005957, MESAN2006022, MESAN2006043, MESAN2006328, MESAN2006401, MESAN2006580, MESAN2006599, MESAN2006743, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926, MESAN2008936, MESAN2009019, MESAN2009105, MESAN2009311, MESAN2009418, MESAN2009503, MESAN2009522, MESAN2010031, MESAN2010114, MESAN2010312, MESAN2010321, MESAN2010664, MESAN2010681, MESAN2011545, MESAN2011597, MESAN2011627,
MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000011,
NB9N41000047, NB9N41000121, NB9N41000142, NB9N41000146, NB9N41000340, NB9N42000104, NB9N42000122, NB9N42000196, NB9N42000314, NB9N42000342, NB9N42000430, NB9N42000495, NB9N42001300, NCRRP1000129, NESOP1000010, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000187, NHNPC2000212, NHNPC2000606, NHNPC2000877, NHNPC2001108, NHNPC2001223, NHNPC2001312, NHNPC2001816, NHNPC2001931,
NHNPC2002565, NHNPC2002749, NOVAR1000091, NOVAR1000102, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000195, NT2NE2000222, NT2NE2000284, NT2NE2000299, NT2NE2000327, NT2NE2000353, NT2NE2000356, NT2NE2000369, NT2NE2000374, NT2NE2000384, NT2NE2000386, NT2NE2000390, NT2NE2000392, NT2NE2000455,
NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000809, NT2NE2000864, NT2NE2000909, NT2NE2000913, NT2NE2000951, NT2NE2000980, NT2NE2001005, NT2NE2001021, NT2NE2001040, NT2NE2001048, NT2NE2001142, NT2NE2001176, NT2NE2001180, NT2NE2001188, NT2NE2001191, NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001623, NT2NE2001626, NT2NE2001634, NT2NE2001648, NT2NE2001655,
NT2NE2001660, NT2NE2001666, NT2NE2001677, NT2NE2001697, NT2NE2001698, NT2NE2001723, NT2NE2001725, NT2NE2001874, .NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003252, NT2NE2003280, NT2NE2003308, NT2NE2003309, NT2NE2003315, NT2NE2003393, NT2NE2003408, NT2NE2003457, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005317,
NT2NE2005323, NT2NE2005358, NT2NE2005395, NT2NE2005419, NT2NE2005517, NT2NE2005537, NT2NE2005555, NT2NE2005588, NT2NE2005676, NT2NE2005720, NT2NE2005784, NT2NE2005804, NT2NE2005821, NT2NE2005890, NT2NE2005921, NT2NE2005968, NT2NE2006087, NT2NE2006103, NT2NE2006217, NT2NE2006288, NT2NE2006382, NT2NE2006387, NT2NE2006427, NT2NE2006432, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727,
NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010842, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012847,
NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721,
NT2NE2017752, NT2NE2017792, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000035, NT2RI1000048, NT2RI1000127, NT2RI1000164, NT2RI1000172, NT2RI2000004, NT2RI2000007, NT2RI2000116, NT2RI2000117, NT2RI2000128, NT2RI2000133, NT2RI2000167, NT2RI2000173, NT2RI2000187, NT2RI2000270, NT2RI2000276, NT2RI2000294, NT2RI2000313, NT2RI2000341, NT2RI2000344, NT2RI2000348, NT2RI2000421, NT2RI2000480, NT2RI2000575, NT2RI2000578, NT2RI2000588, NT2RI2000597, NT2RI2000669, NT2RI2000688, NT2RI2000704, NT2RI2000738, NT2RI2000775, NT2RI2000822,
NT2RI2000829, NT2RI2000841, NT2RI2000865, NT2RI2000932, NT2RI2000974, NT2RI2000987, NT2RI2001010, NT2RI2001017, NT2RI2001030, NT2RI2001083, NT2RI2001167, NT2RI2001178, NT2RI2001235, NT2RI2001244, NT2RI2001307, NT2RI2001342, NT2RI2001410, NT2RI2001425, NT2RI2001449, NT2RI2001450, NT2RI2001474, NT2RI2001519, NT2RI2001595, NT2RI2001624, NT2RI2001731, NT2RI2001769, NT2RI2001782, NT2RI2001836, NT2RI2001846, NT2RI2001866, NT2RI2001910, NT2RI2001952, NT2RI2002022, NT2RI2002041, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002447, NT2RI2002481, NT2RI2002507, NT2RI2002517, NT2RI2002530, NT2RI2002540,
NT2RI2002564, NT2RI2002585, NT2RI2002592, NT2RI2002602, NT2RI2002654, NT2RI2002699, NT2RI2002729, NT2RI2002802, NT2RI2002819, NT2RI2002822, NT2RI2002852, NT2RI2002865, NT2RI2002923, NT2RI2002926, NT2RI2002970, NT2RI2003011, NT2RI2003019, NT2RI2003051, NT2RI2003067, NT2RI2003184, NT2RI2003222, NT2RI2003301, NT2RI2003317, NT2RI2003344, NT2RI2003360, NT2RI2003361, NT2RI2003383, NT2RI2003392, NT2RI2003399, NT2RI2003419, NT2RI2003481, NT2RI2003487, NT2RI2003556, NT2RI2003655, NT2RI2003667, NT2RI2003678, NT2RI2003751, NT2RI2003884, NT2RI2003973, NT2RI2003993, NT2RI2004078, NT2RI2004079, NT2RI2004093, NT2RI2004136, NT2RI2004157, NT2RI2004190, NT2RI2004209, NT2RI2004230, NT2RI2004284, NT2RI2004290,
NT2RI2004304, NT2RI2004312, NT2RI2004381, NT2RI2004398, NT2RI2004410, NT2RI2004442, NT2RI2004468, NT2RI2004535, NT2RI2004606, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005087, NT2RI2005096, NT2RI2005116, NT2RI2005130, NT2RI2005166, NT2RI2005246, NT2RI2005315, NT2RI2005335, NT2RI2005353, NT2RI2005358, NT2RI2005368, NT2RI2005382, NT2RI2005473, NT2RI2005520, NT2RI2005564, NT2RI2005579, NT2RI2005621, NT2RI2005628, NT2RI2005647, NT2RI2005670, NT2RI2005710, NT2RI2005772, NT2RI2005811, NT2RI2005814, NT2RI2005816, NT2RI2005818, NT2RI2005851, NT2RI2005966, NT2RI2006071,
NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006838, NT2RI2006943, NT2RI2006973, NT2RI2007046, NT2RI2007048, NT2RI2007116, NT2RI2007148, NT2RI2007214, NT2RI2007277, NT2RI2007303, NT2RI2007334, NT2RI2007377, NT2RI2007386, NT2RI2007406, NT2RI2007439, NT2RI2007445, NT2RI2007468, NT2RI2007469, NT2RI2007507, NT2RI2007572, NT2RI2007593, NT2RI2007729, NT2RI2007751,
NT2RI2007754, NT2RI2007853, NT2RI2007877, NT2RI2007879, NT2RI2007891, NT2RI2007956, NT2RI2007965, NT2RI2008007, NT2RI2008050, NT2RI2008141, NT2RI2008188, NT2RI2008233, NT2RI2008396, NT2RI2008455, NT2RI2008502, NT2RI2008566, NT2RI2008598, NT2RI2008656, NT2RI2008714, NT2RI2008724, NT2RI2008801, NT2RI2008808, NT2RI2008841, NT2RI2008942, NT2RI2008952, NT2RI2009005, NT2RI2009066, NT2RI2009101, NT2RI2009103, NT2RI2009144, NT2RI2009151, NT2RI2009194, NT2RI2009215, NT2RI2009233, NT2RI2009259, NT2RI2009289, NT2RI2009301, NT2RI2009402, NT2RI2009406, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803,
NT2RI2012350, NT2RI2012659, NT2RI2012990, NT2RI2013345, NT2RI2013357, NT2RI2013373, NT2RI2014247, NT2RI2014551, NT2RI2014683, NT2RI2014733, NT2RI2015239, NT2RI2015342, NT2RI2016128, NT2RI2016134, NT2RI2018311, NT2RI2018363, NT2RI2018883, NT2RI2018950, NT2RI2019751, NT2RI2019826, NT2RI2020390, NT2RI2020703, NT2RI2021463, NT2RI2021625, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537,
NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213,
NT2RI3007291, NT2RI3007443, NT2RI3007543, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000017, NT2RP6000032, NT2RP6000033, NT2RP6000039, NT2RP6000059, NT2RP6000060, NT2RP6000077, NT2RP6000085, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000069, NT2RP7000086, NT2RP7000109, NT2RP7000173, NT2RP7000238, NT2RP7000259, NT2RP7000311, NT2RP7000359, NT2RP7000364, NT2RP7000380, NT2RP7000391, NT2RP7000425,
NT2RP7000477, NT2RP7000579, NT2RP7000586, NT2RP7000600, NT2RP7000624, NT2RP7000812, NT2RP7000926, NT2RP7001074, NT2RP7001080, NT2RP7001156, NT2RP7001231, NT2RP7001319, NT2RP7001335, NT2RP7001364, NT2RP7001475, NT2RP7001532, NT2RP7001591, NT2RP7001602, NT2RP7001678, NT2RP7001856, NT2RP7001962, NT2RP7002028, NT2RP7002064, NT2RP7002151, NT2RP7002163, NT2RP7002243, NT2RP7002376, NT2RP7002450, NT2RP7002478, NT2RP7002554, NT2RP7002594, NT2RP7002603, NT2RP7002650, NT2RP7002738, NT2RP7002779, NT2RP7002802, NT2RP7002841, NT2RP7002875, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203,
NT2RP7003319, NT2RP7003439, NT2RP7003511, NT2RP7003629, NT2RP7003632, NT2RP7003647, NT2RP7003680, NT2RP7003724, NT2RP7003960, NT2RP7004027, NT2RP7004080, NT2RP7004114, NT2RP7004123, NT2RP7004196, NT2RP7004204, NT2RP7004233, NT2RP7004260, NT2RP7004352, NT2RP7004358, NT2RP7004396, NT2RP7004428, NT2RP7004481, NT2RP7004559, NT2RP7004641, NT2RP7004656, NT2RP7004687, NT2RP7004722, NT2RP7004728, NT2RP7004766, NT2RP7004790, NT2RP7004911, NT2RP7004915, NT2RP7004961, NT2RP7004964, NT2RP7004975, NT2RP7005118, NT2RP7005205, NT2RP7005219, NT2RP7005323, NT2RP7005468, NT2RP7005493, NT2RP7005529, NT2RP7005629, NT2RP7005631, NT2RP7005669, NT2RP7005675, NT2RP7005750, NT2RP7005846, NT2RP7006033, NT2RP7006160,
NT2RP7006162, NT2RP7006188, NT2RP7006223, NT2RP7006263, NT2RP7006296, NT2RP7006304, NT2RP7006374, NT2RP7006395, NT2RP7006408, NT2RP7006457, NT2RP7006490, NT2RP7006547, NT2RP7006601, NT2RP7006619, NT2RP7006621, NT2RP7006636, NT2RP7006701, NT2RP7006853, NT2RP7006886, NT2RP7006887, NT2RP7006986, NT2RP7006995, NT2RP7007114, NT2RP7007177, NT2RP7007221, NT2RP7007226, NT2RP7007252, NT2RP7007269, NT2RP7007310, NT2RP7007359, NT2RP7007381, NT2RP7007406, NT2RP7007480, NT2RP7007504, NT2RP7007537, NT2RP7007580, NT2RP7007594, NT2RP7007610, NT2RP7007617, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008161,
NT2RP7008190, NT2RP7008315, NT2RP7008360, NT2RP7008396, NT2RP7008406, NT2RP7008435, NT2RP7008441, NT2RP7008454, NT2RP7008487, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009108, NT2RP7009147, NT2RP7009149, NT2RP7009168, NT2RP7009215, NT2RP7009225, NT2RP7009236, NT2RP7009253, NT2RP7009267, NT2RP7009322, NT2RP7009363, NT2RP7009370, NT2RP7009373, NT2RP7009397, NT2RP7009429, NT2RP7009439, NT2RP7009466, NT2RP7009481, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275,
NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971,
NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000087, NTONG1000123, NTONG1000130, NTONG1000161,
NTONG1000172, NTONG1000213, NTONG1000214, NTONG1000247, NTONG1000264, NTONG2000040, NTONG2000107, NTONG2000231, NTONG2000334, NTONG2000363, NTONG2000413, NTONG2000492, NTONG2000499, NTONG2000531, NTONG2000583, NTONG2000800, NTONG2000876, NTONG2000878, NTONG2000966, NTONG2000985, NTONG2001014, NTONG2001079, NTONG2001137, NTONG2001157, NTONG2001173, NTONG2001220, NTONG2001222, NTONG2001362, NTONG2001428, NTONG2001532, NTONG2001550, NTONG2001587, NTONG2001619, NTONG2001642, NTONG2001657, NTONG2001676, NTONG2001762, NTONG2002278, NTONG2002807, NTONG2002948, NTONG2002970, NTONG2002985, NTONG2003158, NTONG2003210, NTONG2003316, NTONG2003409, NTONG2003805, NTONG2003839, NTONG2003852, NTONG2003897,
NTONG2004095, NTONG2004308, NTONG2004521, NTONG2004614, NTONG2004690, NTONG2004829, NTONG2004918, NTONG2004991, NTONG2005062, NTONG2005086, NTONG2005137, NTONG2005265, NTONG2005277, NTONG2005363, NTONG2005373, NTONG2005391, NTONG2005480, NTONG2005497, NTONG2005577, NTONG2005657, NTONG2005801, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008672, NTONG2008862, NTONG2008939,
NTONG2008944, NTONG2009060, NTONG2009068, NTONG2009229, NTONG2009233, NTONG2009576, OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000118, OCBBF1000119, OCBBF1000122, OCBBF1000130, OCBBF1000138, OCBBF1000145, OCBBF1000153, OCBBF1000175, OCBBF1000185, OCBBF1000188, OCBBF1000192, OCBBF1000254, OCBBF1000315, OCBBF2000015, OCBBF2000126, OCBBF2000178, OCBBF2000231, OCBBF2000277, OCBBF2000323, OCBBF2000452, OCBBF2000677, OCBBF2000703, OCBBF2000719, OCBBF2000824, OCBBF2000831, OCBBF2000846, OCBBF2000904, OCBBF2000986, OCBBF2000998, OCBBF2001075, OCBBF2001101, OCBBF2001124, OCBBF2001140, OCBBF2001166,
OCBBF2001176, OCBBF2001186, OCBBF2001210, OCBBF2001252, OCBBF2001307, OCBBF2001323, OCBBF2001389, OCBBF2001402, OCBBF2001408, OCBBF2001416, OCBBF2001473, OCBBF2001492, OCBBF2001494, OCBBF2001527, OCBBF2001528, OCBBF2001586, OCBBF2001639, OCBBF2001681, OCBBF2001687, OCBBF2001706, OCBBF2001749, OCBBF2001910, OCBBF2001931, OCBBF2001961, OCBBF2001983, OCBBF2002015, OCBBF2002083, OCBBF2002124, OCBBF2002161, OCBBF2002290, OCBBF2002357, OCBBF2002615, OCBBF2002626, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925,
OCBBF2004038, OCBBF2004147, OCBBF2004168, OCBBF2004259, OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639,
OCBBF2006764, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007428, OCBBF2007478, OCBBF2007485, OCBBF2007520, OCBBF2007555, OCBBF2007610, OCBBF2007756, OCBBF2007762, OCBBF2007829, OCBBF2007892, OCBBF2007931, OCBBF2007946, OCBBF2008041, OCBBF2008116, OCBBF2008138, OCBBF2008144, OCBBF2008283, OCBBF2008330, OCBBF2008340, OCBBF2008366, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775, OCBBF2008790, OCBBF2008814,
OCBBF2008822, OCBBF2009095, OCBBF2009115, OCBBF2009242, OCBBF2009301, OCBBF2009352, OCBBF2009391, OCBBF2009424, OCBBF2009536, OCBBF2009571, OCBBF2009583, OCBBF2009603, OCBBF2009772, OCBBF2009788, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010843, OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011160, OCBBF2011177, OCBBF2011228, OCBBF2011232, OCBBF2011283, OCBBF2011311, OCBBF2011625, OCBBF2011669, OCBBF2011685, OCBBF2011706, OCBBF2011722,
OCBBF2011759, OCBBF2011767, OCBBF2011855, OCBBF2011872, OCBBF2011887, OCBBF2011897, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012678, OCBBF2012704, OCBBF2012714, OCBBF2012755, OCBBF2012812, OCBBF2012821, OCBBF2012881, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013481, OCBBF2013507, OCBBF2013721, OCBBF2013789, OCBBF2013843, OCBBF2013883, OCBBF2013926, OCBBF2014052, OCBBF2014064, OCBBF2014089, OCBBF2014322, OCBBF2014386, OCBBF2014576,
OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2017035, OCBBF2017055, OCBBF2017069, OCBBF2017306, OCBBF2017325, OCBBF2017458, OCBBF2017489, OCBBF2017516, OCBBF2017665, OCBBF2017754, OCBBF2017815, OCBBF2017882, OCBBF2017888, OCBBF2017893, OCBBF2017920, OCBBF2018012, OCBBF2018014, OCBBF2018167, OCBBF2018206,
OCBBF2018229, OCBBF2018234, OCBBF2018362, OCBBF2018380, OCBBF2018563, OCBBF2018581, OCBBF2018618, OCBBF2018663, OCBBF2018687, OCBBF2018827, OCBBF2018828, OCBBF2018873, OCBBF2018934, OCBBF2018956, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198,
OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413,
OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314,
OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000062, PEBLM1000066, PEBLM1000068, PEBLM1000071, PEBLM1000083, PEBLM1000144, PEBLM1000147, PEBLM1000174, PEBLM1000180, PEBLM2000030, PEBLM2000112, PEBLM2000147, PEBLM2000170, PEBLM2000180, PEBLM2000213, PEBLM2000248, PEBLM2000267, PEBLM2000270, PEBLM2000308, PEBLM2000321, PEBLM2000326, PEBLM2000333, PEBLM2000338, PEBLM2000502, PEBLM2001312, PEBLM2001465, PEBLM2001488,
PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007188, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387,
PERIC2000422, PERIC2000473, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086,
PERIC2009329, PERIC2009566, PLACE5000001, PLACE5000037, PLACE5000058, PLACE5000067, PLACE5000068, PLACE5000070, PLACE5000080, PLACE5000105, PLACE5000115, PLACE5000116, PLACE5000129, PLACE5000139, PLACE5000153, PLACE5000159, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000314, PLACE6000325, PLACE6000348, PLACE6000424, PLACE6000426, PLACE6000429, PLACE6000450, PLACE6000523, PLACE6000550, PLACE6000552, PLACE6000555, PLACE6000619,
PLACE6000953, PLACE6001118, PLACE6001153, PLACE6001185, PLACE6001262, PLACE6001281, PLACE6001294, PLACE6001536, PLACE6001557, PLACE6001712, PLACE6001740, PLACE6001823, PLACE6001923, PLACE6001925, PLACE6001933, PLACE6002016, PLACE6002102, PLACE6002157, PLACE6002312, PLACE6002323, PLACE6002345, PLACE6002462, PLACE6002647, PLACE6002692, PLACE6002699, PLACE6002710, PLACE6002871, PLACE6002949, PLACE6003004, PLACE6003038, PLACE6003077, PLACE6003094, PLACE6003109, PLACE6003204, PLACE6003218, PLACE6003372, PLACE6003393, PLACE6003399, PLACE6003539, PLACE6003621, PLACE6003705, PLACE6003740, PLACE6003745, PLACE6003771, PLACE6003796, PLACE6003839, PLACE6004101, PLACE6004219, PLACE6004336, PLACE6004368,
PLACE6004380, PLACE6004396, PLACE6004397, PLACE6004454, PLACE6004491, PLACE6004663, PLACE6004687, PLACE6004738, PLACE6004931, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006077, PLACE6006137, PLACE6006158, PLACE6006186, PLACE6006217, PLACE6006222, PLACE6006266, PLACE6006287, PLACE6006394, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309,
PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008824, PLACE6008989, PLACE6009006, PLACE6009008, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011057, PLACE6011102, PLACE6011156,
PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016383, PLACE6016984, PLACE6017002, PLACE6017431,
PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169,
PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000123, PROST1000136, PROST1000145, PROST1000152, PROST1000167, PROST1000184, PROST1000217, PROST1000220, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000485, PROST1000493, PROST1000512, PROST1000527, PROST1000528, PROST1000536,
PROST1000559, PROST1000575, PROST2000036, PROST2000053, PROST2000067, PROST2000079, PROST2000105, PROST2000143, PROST2000162, PROST2000165, PROST2000176, PROST2000203, PROST2000206, PROST2000212, PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001213, PROST2001414, PROST2001415, PROST2001465, PROST2001521, PROST2001540, PROST2001599, PROST2001611, PROST2001636, PROST2001796, PROST2001805, PROST2001823,
PROST2001899, PROST2001997, PROST2001998, PROST2002101, PROST2002162, PROST2002212, PROST2002227, PROST2002338, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003324, PROST2003340, PROST2003396, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142,
PROST2004146, PROST2004161, PROST2004251, PROST2004332, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005228, PROST2005285, PROST2005426, PROST2005466, PROST2005604, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006060, PROST2006196, PROST2006201, PROST2006260, PROST2006343, PROST2006450, PROST2006491, PROST2006510, PROST2006536, PROST2006579, PROST2006688, PROST2006737, PROST2006766, PROST2006782, PROST2006988,
PROST2007122, PROST2007200, PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008271, PROST2008447, PROST2008468, PROST2008472, PROST2008489, PROST2008516, PROST2008724, PROST2008770, PROST2008835, PROST2008993, PROST2009022, PROST2009208, PROST2009222, PROST2009254, PROST2009273, PROST2009347, PROST2009365, PROST2009388, PROST2009400, PROST2009470, PROST2009483, PROST2009571, PROST2009731, PROST2009736, PROST2009795, PROST2009901, PROST2009909,
PROST2010046, PROST2010219, PROST2010250, PROST2010318, PROST2010326, PROST2010382, PROST2010400, PROST2010579, PROST2010606, PROST2010782, PROST2010885, PROST2011012, PROST2011038, PROST2011105, PROST2011297, PROST2011410, PROST2011439, PROST2011482, PROST2011577, PROST2011631, PROST2011660, PROST2012005, PROST2012007, PROST2012016, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012888, PROST2012890, PROST2013032, PROST2013121, PROST2013260, PROST2013327, PROST2013531, PROST2013605, PROST2013627, PROST2013873, PROST2013880, PROST2014422, PROST2014585, PROST2014601,
PROST2014659, PROST2014916, PROST2014925, PROST2015124, PROST2015137, PROST2015198, PROST2015243, PROST2015246, PROST2015251, PROST2015287, PROST2015332, PROST2015457, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2017098, PROST2017128, PROST2017203, PROST2017367, PROST2017413, PROST2017441, PROST2017529, PROST2017578, PROST2017612, PROST2017617, PROST2017700, PROST2017701,
PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019296, PROST2019398, PROST2019487, PROST2019781, PUAEN1000039, PUAEN1000057, PUAEN1000081, PUAEN1000085, PUAEN1000087, PUAEN1000121, PUAEN1000147, PUAEN1000161, PUAEN1000175, PUAEN1000239, PUAEN1000275, PUAEN1000276, PUAEN1000288, PUAEN2000080, PUAEN2000152, PUAEN2000175, PUAEN2000247, PUAEN2000312, PUAEN2000374, PUAEN2000394, PUAEN2000497, PUAEN2000594, PUAEN2000684, PUAEN2000942, PUAEN2001260, PUAEN2001345, PUAEN2001526, PUAEN2001586, PUAEN2001882, PUAEN2002473,
PUAEN2002489, PUAEN2002552, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349,
RECTM2000433, RECTM2001307, RECTM2001347, RECTM2001519, RECTM2001666, RECTM2001691, RECTM2001749, RECTM2002172, RECTM2002602, RECTM2002632, SYNOV1000049, SYNOV1000118, SYNOV1000128, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001251, SYNOV2001300, SYNOV2001356, SYNOV2001374, SYNOV2001390, SYNOV2001451, SYNOV2001457, SYNOV2001468, SYNOV2001581, SYNOV2001660, SYNOV2003326,
SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823,
SYNOV4004914, SYNOV4005256, SYNOV4005570, SYNOV4005739, SYNOV4005889, SYNOV4005981, SYNOV4005989, SYNOV4006256, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, T1ESE2000116, T1ESE2000609, T1ESE2000904, T1ESE2002665, TBAES2000004, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543,
TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150,
TESOP2002273, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018,
TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179,
TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082,
TESTI2001120, TESTI2001134, TESTI2001141, TESTI2001180, TESTI2001208, TESTI2001229, TESTI2001236, TESTI2001237, TESTI2001269, TESTI2001306, TESTI2001313, TESTI2001330, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001869, TESTI2001879, TESTI2001894, TESTI2001898, TESTI2001915, TESTI2001950, TESTI2001959, TESTI2001968, TESTI2001985,
TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002698, TESTI2002700, TESTI2002707, TESTI2002724, TESTI2002729, TESTI2002752, TESTI2002753, TESTI2002789, TESTI2002802, TESTI2002806,
TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003413, TESTI2003418,
TESTI2003419, TESTI2003432, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004031, TESTI2004073, TESTI2004080, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229,
TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005025, TESTI2005040, TESTI2005060, TESTI2005072, TESTI2005085, TESTI2005112, TESTI2005120,
TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005767, TESTI2005775, TESTI2005784, TESTI2005788, TESTI2005827, TESTI2005835, TESTI2005860, TESTI2005892, TESTI2005908, TESTI2005911, TESTI2005933, TESTI2005937, TESTI2005948, TESTI2005979,
TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006360, TESTI2006409, TESTI2006425, TESTI2006437, TESTI2006453, TESTI2006457, TESTI2006460, TESTI2006465, TESTI2006483, TESTI2006499, TESTI2006523, TESTI2006543, TESTI2006565, TESTI2006572, TESTI2006617, TESTI2006624, TESTI2006628, TESTI2006633, TESTI2006643, TESTI2006648,
TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692,
TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008715, TESTI2008762, TESTI2008774, TESTI2008786,
TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009520, TESTI2009544, TESTI2009545, TESTI2009551, TESTI2009577, TESTI2009585, TESTI2009588, TESTI2009683, TESTI2009727, TESTI2009739, TESTI2009812, TESTI2009835, TESTI2009853, TESTI2009935, TESTI2009977, TESTI2009987, TESTI2010009, TESTI2010154, TESTI2010180,
TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011612, TESTI2011665, TESTI2011680, TESTI2011683, TESTI2011750, TESTI2011840, TESTI2011846, TESTI2011915, TESTI2011928, TESTI2011971, TESTI2012019, TESTI2012050, TESTI2012090, TESTI2012095, TESTI2012152, TESTI2012155,
TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610,
TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014577, TESTI2014578, TESTI2014716, TESTI2014780, TESTI2014800, TESTI2014838, TESTI2014843, TESTI2014979, TESTI2014988, TESTI2015042, TESTI2015105, TESTI2015180, TESTI2015213, TESTI2015246, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752,
TESTI2015830, TESTI2015914, TESTI2015938, TESTI2015947, TESTI2015962, TESTI2015987, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582,
TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455,
TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118,
TESTI2021122, TESTI2021124, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194,
TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024936, TESTI2024978, TESTI2024999, TESTI2025006, TESTI2025013, TESTI2025022, TESTI2025063, TESTI2025117, TESTI2025144, TESTI2025161,
TESTI2025174, TESTI2025229, TESTI2025269, TESTI2025365, TESTI2025403, TESTI2025409, TESTI2025422, TESTI2025448, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794,
TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670,
TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067,
TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262,
TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048,
TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330,
TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796,
TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047792, TESTI2047801, TESTI2047818, TESTI2047824, TESTI2047885, TESTI2047930, TESTI2048105, TESTI2048109, TESTI2048465,
TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723,
TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001348, TESTI4001441, TESTI4001467, TESTI4001473, TESTI4001517, TESTI4001527, TESTI4001561, TESTI4001569, TESTI4001604, TESTI4001665, TESTI4001679,
TESTI4001795, TESTI4001923, TESTI4001925, TESTI4001984, TESTI4002003, TESTI4002072, TESTI4002141, TESTI4002195, TESTI4002290, TESTI4002319, TESTI4002491, TESTI4002520, TESTI4002552, TESTI4002647, TESTI4002703, TESTI4002754, TESTI4002799, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317,
TESTI4005322, TESTI4005399, TESTI4005470, TESTI4005500, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007965, TESTI4008007,
TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789,
TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013735, TESTI4013742, TESTI4013774, TESTI4013817, TESTI4013830, TESTI4013852, TESTI4013894,
TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229,
TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456,
TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700,
TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128,
TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258,
TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223,
TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746,
TESTI4047808, THYMU1000002, THYMU1000016, THYMU1000017, THYMU1000025, THYMU1000029, THYMU1000033, THYMU1000041, THYMU1000060, THYMU1000074, THYMU1000098, THYMU1000105, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000331, THYMU1000336, THYMU1000366, THYMU1000382, THYMU1000393, THYMU1000394, THYMU1000399, THYMU1000426, THYMU1000440, THYMU1000459, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032,
THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000317, THYMU2000368, THYMU2000369, THYMU2000418, THYMU2000436, THYMU2000440, THYMU2000489, THYMU2000492, THYMU2000519, THYMU2000570, THYMU2000602, THYMU2000660, THYMU2000671, THYMU2000702, THYMU2000767, THYMU2000800, THYMU2000879, THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001202, THYMU2001203, THYMU2001256, THYMU2001270, THYMU2001291, THYMU2001314, THYMU2001325, THYMU2001357, THYMU2001381, THYMU2001422,
THYMU2001443, THYMU2001515, THYMU2001521, THYMU2001556, THYMU2001622, THYMU2001727, THYMU2001825, THYMU2001868, THYMU2001898, THYMU2001900, THYMU2001916, THYMU2001926, THYMU2001989, THYMU2002023, THYMU2002037, THYMU2002080, THYMU2002109, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002789, THYMU2002792, THYMU2002815, THYMU2002841, THYMU2002852, THYMU2002910, THYMU2002950, THYMU2003069, THYMU2003107, THYMU2003124, THYMU2003166, THYMU2003232, THYMU2003242, THYMU2003249, THYMU2003270, THYMU2003282, THYMU2003336,
THYMU2003366, THYMU2003397, THYMU2003440, THYMU2003470; THYMU2003479, THYMU2003632, THYMU2003650, THYMU2003700, THYMU2003760, THYMU2003803, THYMU2003855, THYMU2003907, THYMU2004003, THYMU2004008, THYMU2004042, THYMU2004108, THYMU2004111, THYMU2004123, THYMU2004139, THYMU2004169, THYMU2004284, THYMU2004336, THYMU2004344, THYMU2004356, THYMU2004452, THYMU2004508, THYMU2004512, THYMU2004635, THYMU2004639, THYMU2004668, THYMU2004677, THYMU2004688, THYMU2004693, THYMU2004835, THYMU2004843, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321,
THYMU2005356, THYMU2005369, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006505, THYMU2006666, THYMU2006712, THYMU2006913, THYMU2006946, THYMU2006965, THYMU2007025, THYMU2007036, THYMU2007095, THYMU2007112, THYMU2007146, THYMU2007179, THYMU2007258, THYMU2007307, THYMU2007308, THYMU2007339, THYMU2007350, THYMU2007384, THYMU2007493,
THYMU2007521, THYMU2007528, THYMU2007530, THYMU2007532, THYMU2007561, THYMU2007571, THYMU2007635, THYMU2007658, THYMU2007667, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008339, THYMU2008350, THYMU2008351, THYMU2008383, THYMU2008452, THYMU2008643, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104,
THYMU2009134, THYMU2009157, THYMU2009181, THYMU2009338, THYMU2009425, THYMU2009516, THYMU2009526, THYMU2009546, THYMU2009592, THYMU2009596, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010637, THYMU2010671, THYMU2010699, THYMU2010705, THYMU2010779, THYMU2010816, THYMU2010831, THYMU2010848, THYMU2011053, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666,
THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012507, THYMU2012565, THYMU2012574, THYMU2012625, THYMU2012631, THYMU2012665, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014183, THYMU2014204, THYMU2014297,
THYMU2014323, THYMU2014327, THYMU2014353, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831,
THYMU2017844, THYMU2017878, THYMU2017948, THYMU2018028, THYMU2018071, THYMU2018189, THYMU2018197, THYMU2018384, THYMU2018455, THYMU2018547, THYMU2018565, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264,
THYMU2023576, THYMU2023711, THYMU2023900, THYMU2023943, THYMU2023967, THYMU2024071, THYMU2024185, THYMU2024207, THYMU2024616, THYMU2024684, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027996, THYMU2028041, THYMU2028368, THYMU2028379, THYMU2028394, THYMU2028412, THYMU2028632, THYMU2028724, THYMU2028739, THYMU2028942, THYMU2028978, THYMU2029134, THYMU2029676, THYMU2029688, THYMU2029788,
THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033308, THYMU2033401, THYMU2033583, THYMU2033755, THYMU2033787, THYMU2033816, THYMU2034182, THYMU2034279, THYMU2034314,
THYMU2034338, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634,
THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003092, THYMU3003212, THYMU3003309,
THYMU3003350, THYMU3003403, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755,
THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713,
THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231,
THYMU3030706, THYMU3030710, THYMU3030752, THYMU3031146, THYMU3031402, THYMU3031610, THYMU3031612, THYMU3031868, THYMU3031878, THYMU3032032, THYMU3033402, THYMU3033626, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970,
THYMU3039807, THYMU3039846, THYMU3040126, THYMU3040146, THYMU3040168, THYMU3040172, THYMU3040725, THYMU3040746, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045673, THYMU3045692, THYMU3045704, THYMU3046140, THYMU3046350, THYMU3046360, THYMU3046856, THYMU3047144, THYMU3047156, THYMU3047366, THYMU3047513, THYMU3047542, THYMU3047760, THYMU3047891, TKIDN1000001, TKIDN1000044,
TKIDN1000062, TKIDN1000066, TKIDN1000164, TKIDN1000171, TKIDN1000192, TKIDN2000127, TKIDN2000240, TKIDN2000246, TKIDN2000319, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278,
TKIDN2010602, TKIDN2010812, TKIDN2010934, TKIDN2011051, TKIDN2011160, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002882, TLIVE2003197, TLIVE2003200, TLIVE2003225, TLIVE2003381, TLIVE2003737, TLIVE2003961, TLIVE2004110, TLIVE2004320,
TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000018, TRACH1000057, TRACH1000074, TRACH1000100, TRACH1000125, TRACH1000140, TRACH1000144,
TRACH1000181, TRACH1000193, TRACH1000205, TRACH1000212, TRACH1000240, TRACH2000002, TRACH2000015, TRACH2000024, TRACH2000032, TRACH2000079, TRACH2000081, TRACH2000083, TRACH2000148, TRACH2000185, TRACH2000196, TRACH2000235, TRACH2000250, TRACH2000259, TRACH2000272, TRACH2000287, TRACH2000289, TRACH2000306, TRACH2000312, TRACH2000336, TRACH2000393, TRACH2000411, TRACH2000420, TRACH2000461, TRACH2000496, TRACH2000497, TRACH2000502, TRACH2000522, TRACH2000559, TRACH2000660, TRACH2000665, TRACH2000675, TRACH2000677, TRACH2000703, TRACH2000767, TRACH2000775, TRACH2000780, TRACH2000807, TRACH2000862, TRACH2000873, TRACH2000894, TRACH2000898, TRACH2000923, TRACH2000926, TRACH2000944, TRACH2000959,
TRACH2000981, TRACH2000986, TRACH2001037, TRACH2001060, TRACH2001085, TRACH2001101, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001341, TRACH2001347, TRACH2001395, TRACH2001400, TRACH2001403, TRACH2001416, TRACH2001443, TRACH2001474, TRACH2001478, TRACH2001525, TRACH2001532, TRACH2001592, TRACH2001612, TRACH2001621, TRACH2001646, TRACH2001651, TRACH2001684, TRACH2001810, TRACH2001898, TRACH2001933, TRACH2001968, TRACH2001996, TRACH2002054, TRACH2002092, TRACH2002100, TRACH2002333, TRACH2002784, TRACH2002840, TRACH2002853, TRACH2002862, TRACH2002954, TRACH2002960, TRACH2002988,
TRACH2003070, TRACH2003272, TRACH2003484, TRACH2003516, TRACH2003605, TRACH2003736, TRACH2003759, TRACH2003941, TRACH2004039, TRACH2004054, TRACH2004087, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762,
TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007894, TRACH2007969, TRACH2007988, TRACH2008081, TRACH2008112, TRACH2008113, TRACH2008127, TRACH2008130, TRACH2008278, TRACH2008300, TRACH2008583, TRACH2008597, TRACH2008820, TRACH2008891, TRACH2008921, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419,
TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011290, TRACH2011293, TRACH2011302, TRACH2011574, TRACH2011894, TRACH2011956, TRACH2012000, TRACH2012138, TRACH2012242, TRACH2012370, TRACH2012387, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268,
TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949,
TRACH2017965, TRACH2017980, TRACH2018034, TRACH2018084, TRACH2018262, TRACH2018273, TRACH2018278, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2019024, TRACH2019046, TRACH2019080, TRACH2019151, TRACH2019154, TRACH2019176, TRACH2019248, TRACH2019309, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306,
TRACH2023416, TRACH2023479, TRACH2023684, TRACH2023860, TRACH2023876, TRACH2024139, TRACH2024187, TRACH2024271, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119,
TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072,
TRACH3005085, TRACH3005102, TRACH3005173, TRACH3005191, TRACH3005211, TRACH3005274, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007625, TRACH3007627, TRACH3007689, TRACH3007766, TRACH3007866, TRACH3007995, TRACH3008042, TRACH3008061, TRACH3008201, TRACH3008508, TRACH3008535, TRACH3008629,
TRACH3008632, TRACH3008688, TRACH3008697, TRACH3008713, TRACH3008887, TRACH3008902, TRACH3008990, TRACH3009001, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684,
TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447,
TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316,
TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678,
TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037267, TRACH3037288, TRACH3037505, TRACH3037573, TRACH3037696,
TRACH3037897, TRACH3038086, TRACH3038399, TSTOM1000135, TSTOM1000186, TSTOM2000139, TSTOM2000235, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000152, UMVEN2000354,
UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000263, UTERU2000300, UTERU2000329, UTERU2000349, UTERU2000377, UTERU2000393, UTERU2000418, UTERU2000465, UTERU2000485, UTERU2000517, UTERU2000524, UTERU2000539, UTERU2000542, UTERU2000546, UTERU2000550, UTERU2000569,
UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001607, UTERU2001658, UTERU2001747, UTERU2001876, UTERU2002001, UTERU2002011, UTERU2002176, UTERU2002198, UTERU2002332, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003126, UTERU2003135, UTERU2003321, UTERU2003399, UTERU2003411, UTERU2003577, UTERU2003704, UTERU2003926, UTERU2003973, UTERU2004015, UTERU2004037,
UTERU2004039, UTERU2004061, UTERU2004073, UTERU2004163, UTERU2004299, UTERU2004461, UTERU2004520, UTERU2004564, UTERU2004688, UTERU2004698, UTERU2004861, UTERU2004929, UTERU2005004, UTERU2005050, UTERU2005069, UTERU2005074, UTERU2005179, UTERU2005346, UTERU2005446, UTERU2005449, UTERU2005450, UTERU2005533, UTERU2005601, UTERU2005664, UTERU2005822, UTERU2005903, UTERU2005905, UTERU2006103, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639,
UTERU2007724, UTERU2007924, UTERU2007942, UTERU2008018, UTERU2008019, UTERU2008027, UTERU2008040, UTERU2008077, UTERU2008130, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009540, UTERU2009776, UTERU2009904, UTERU2009951, UTERU2009972, UTERU2010115, UTERU2010124, UTERU2010164, UTERU2010231, UTERU2010304, UTERU2010320, UTERU2010431, UTERU2010525, UTERU2010651, UTERU2010724, UTERU2010747,
UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012286, UTERU2012333, UTERU2012407, UTERU2012526, UTERU2012581, UTERU2012610, UTERU2012615, UTERU2012688, UTERU2012703, UTERU2012715, UTERU2012767, UTERU2012786, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024,
UTERU2014167, UTERU2014223, UTERU2014398, UTERU2014464, UTERU2014548, UTERU2014601, UTERU2014631, UTERU2014668, UTERU2014678, UTERU2014728, UTERU2014998, UTERU2015062, UTERU2015087, UTERU2015108, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757, UTERU2016761, UTERU2016799, UTERU2016896, UTERU2016902, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522,
UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023175, UTERU2023262, UTERU2023651, UTERU2023687, UTERU2023712, UTERU2023941, UTERU2024002, UTERU2024042,
UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420,
UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059,
UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005907,
UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599,
UTERU3012999, UTERU3013167, UTERU3013302, UTERU3013781, UTERU3014274, UTERU3014446, UTERU3014611, UTERU3014647, UTERU3014791, UTERU3014906, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the SYNOV, T1ESE, TBAES, TCERX, TCOLN, TESOP, and TESTI libraries were analyzed for expression frequency. The analysis results are described below. Each is a relative value, where the expression level over all tissues is taken as 100. The larger this value, the greater the level of expression.

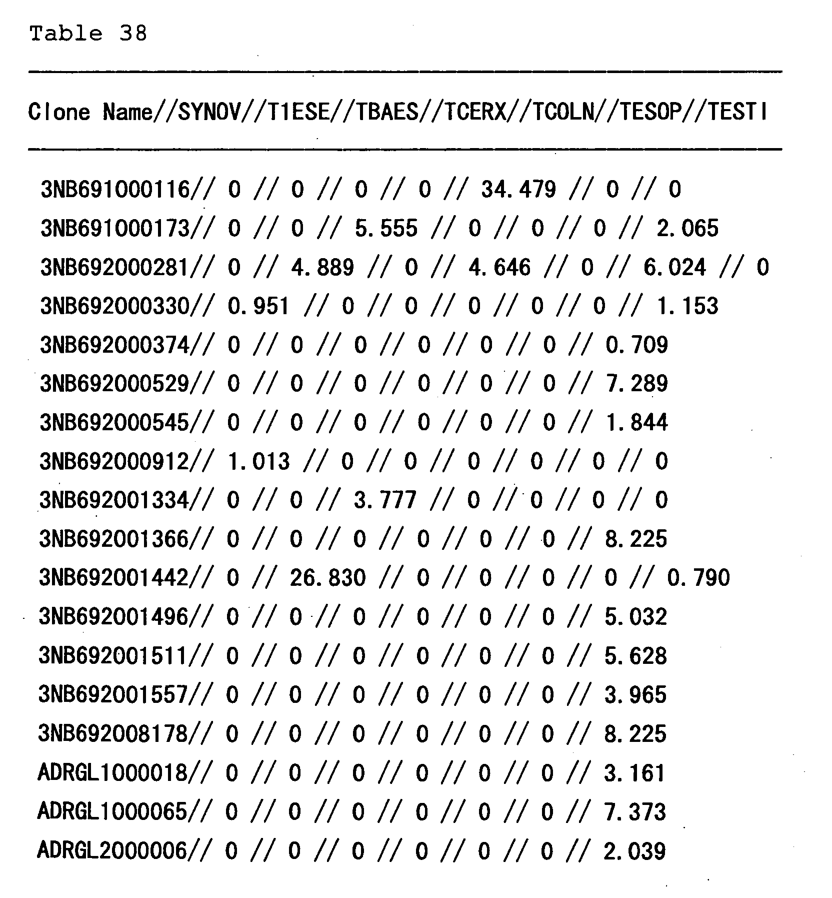

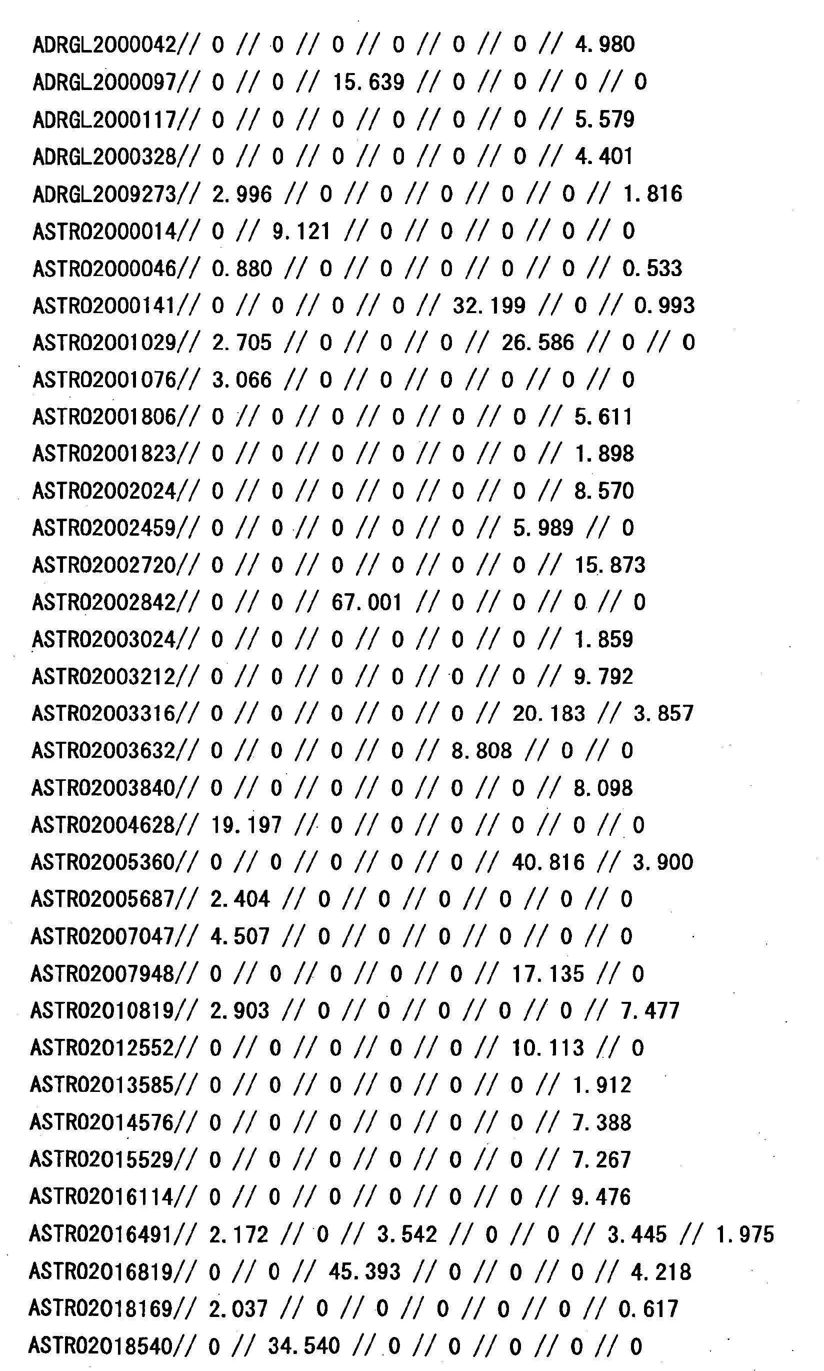

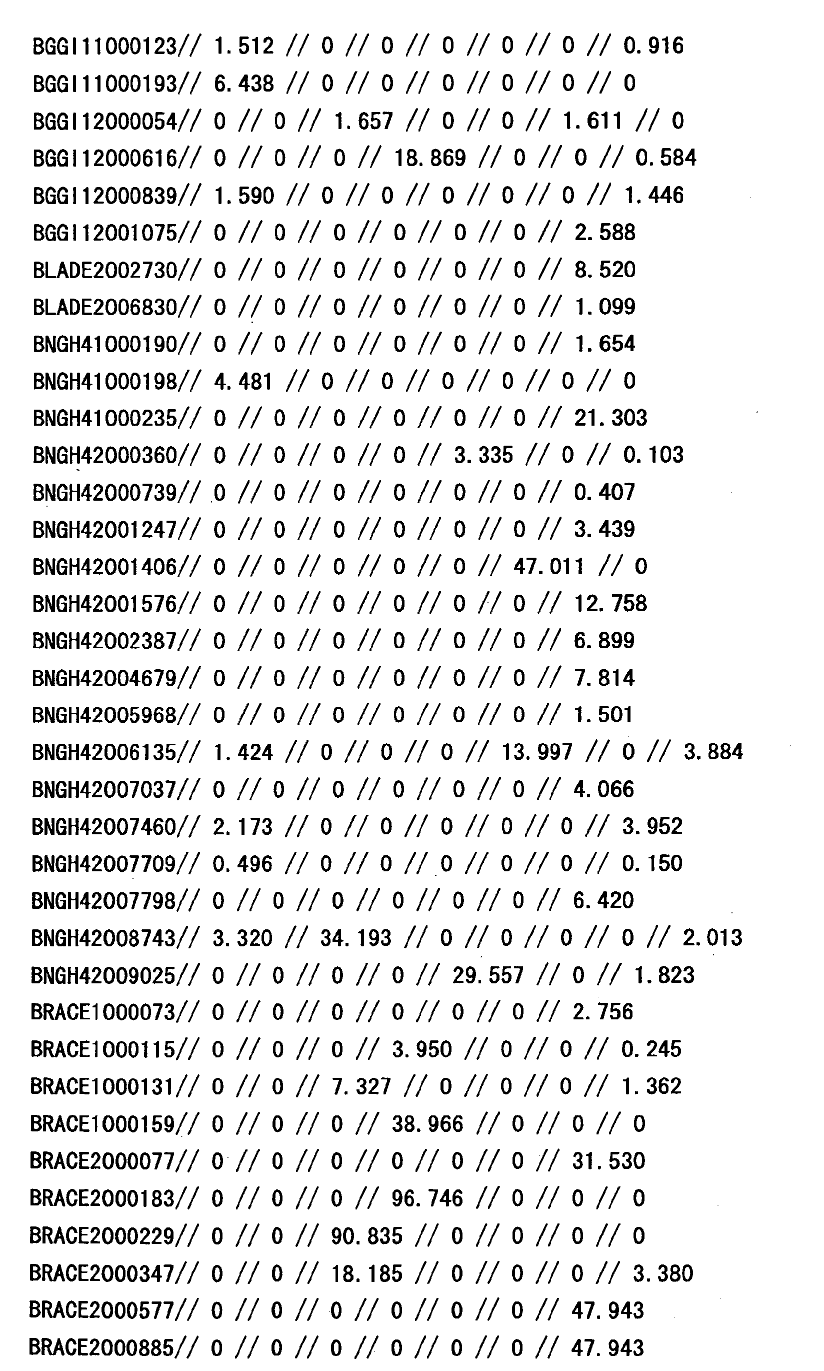

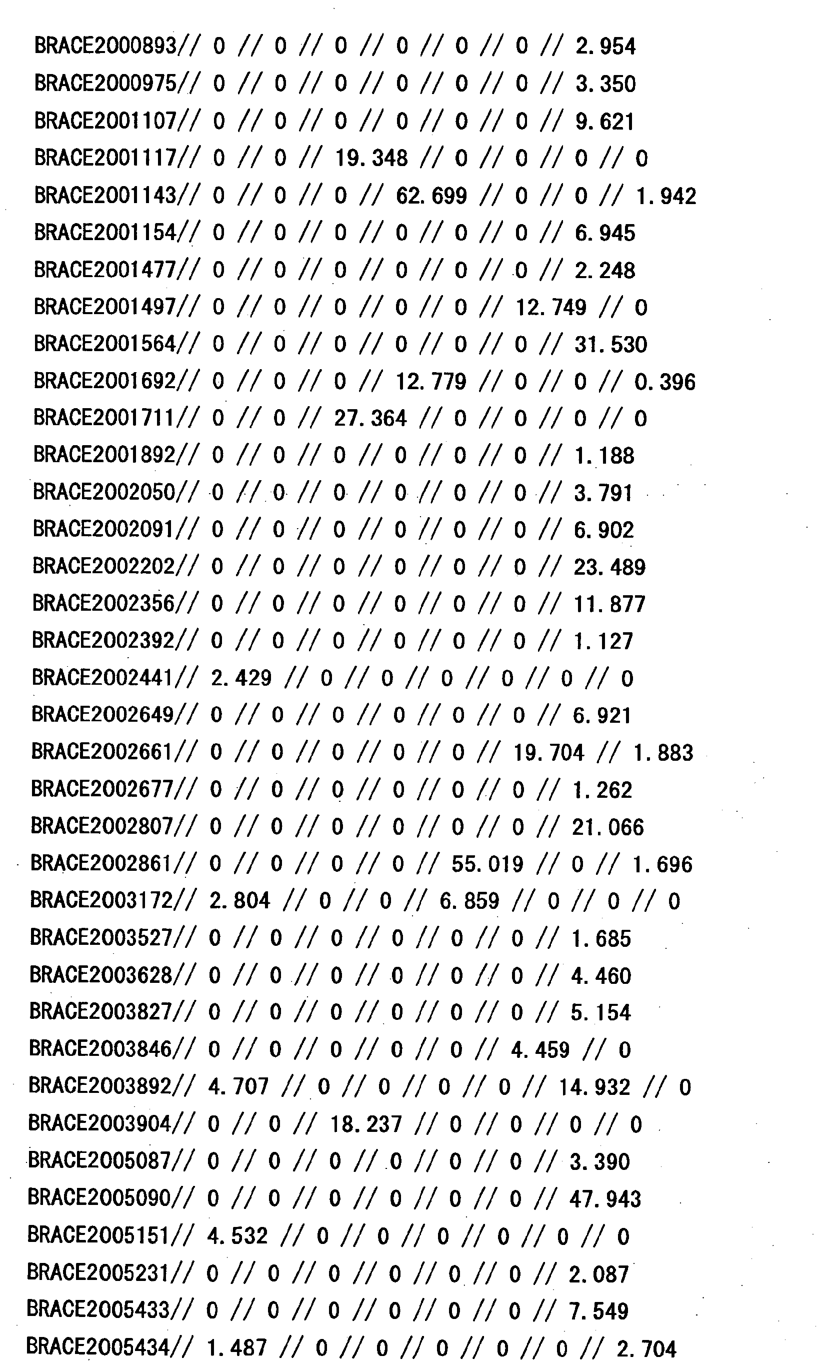

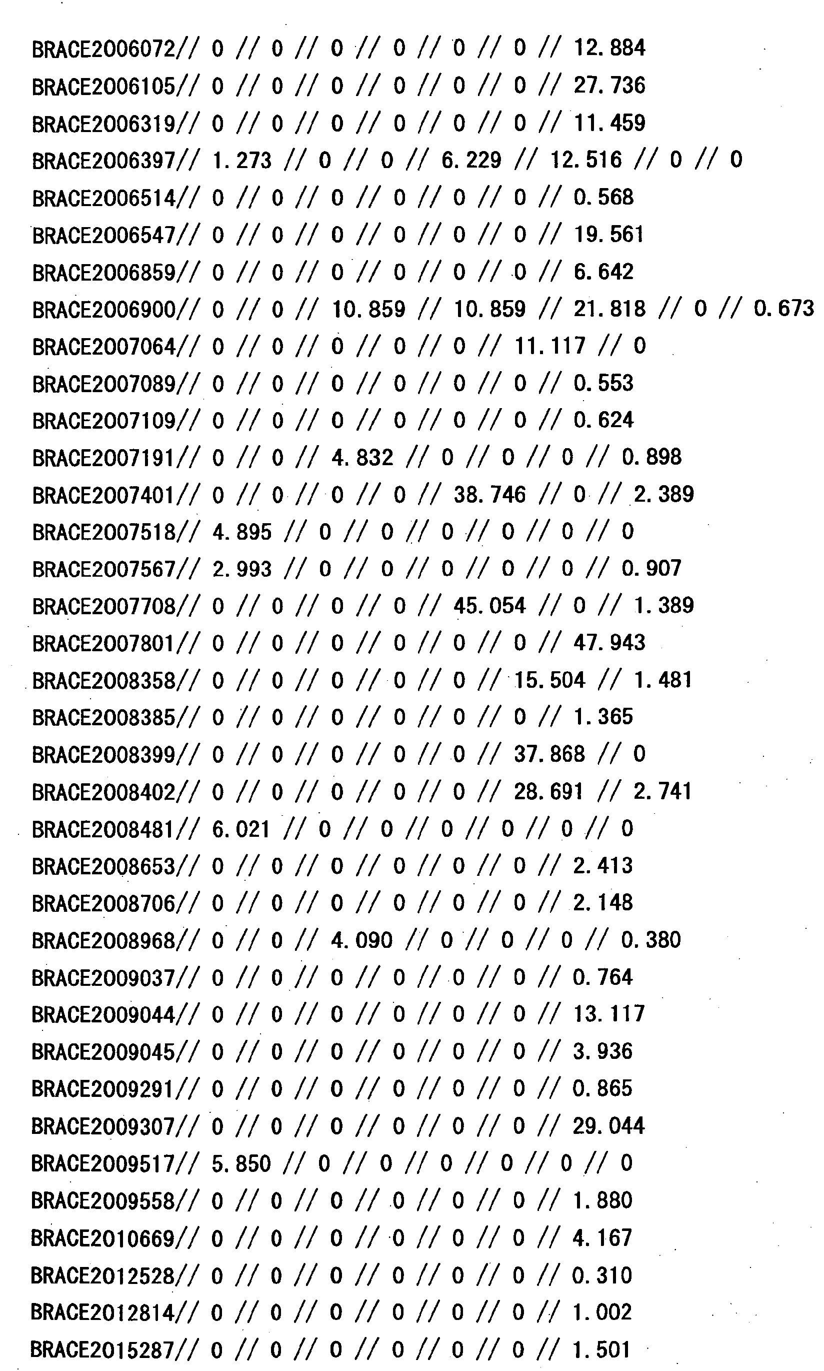

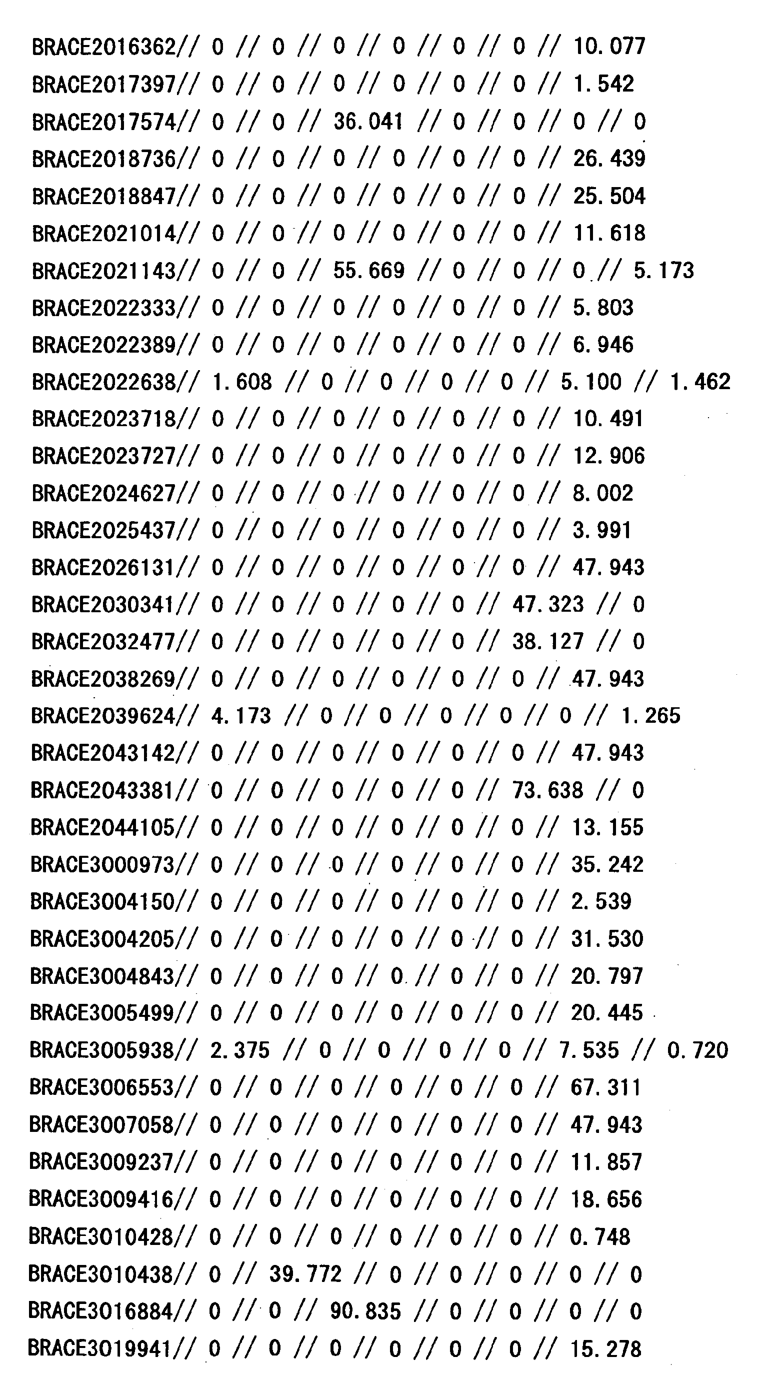

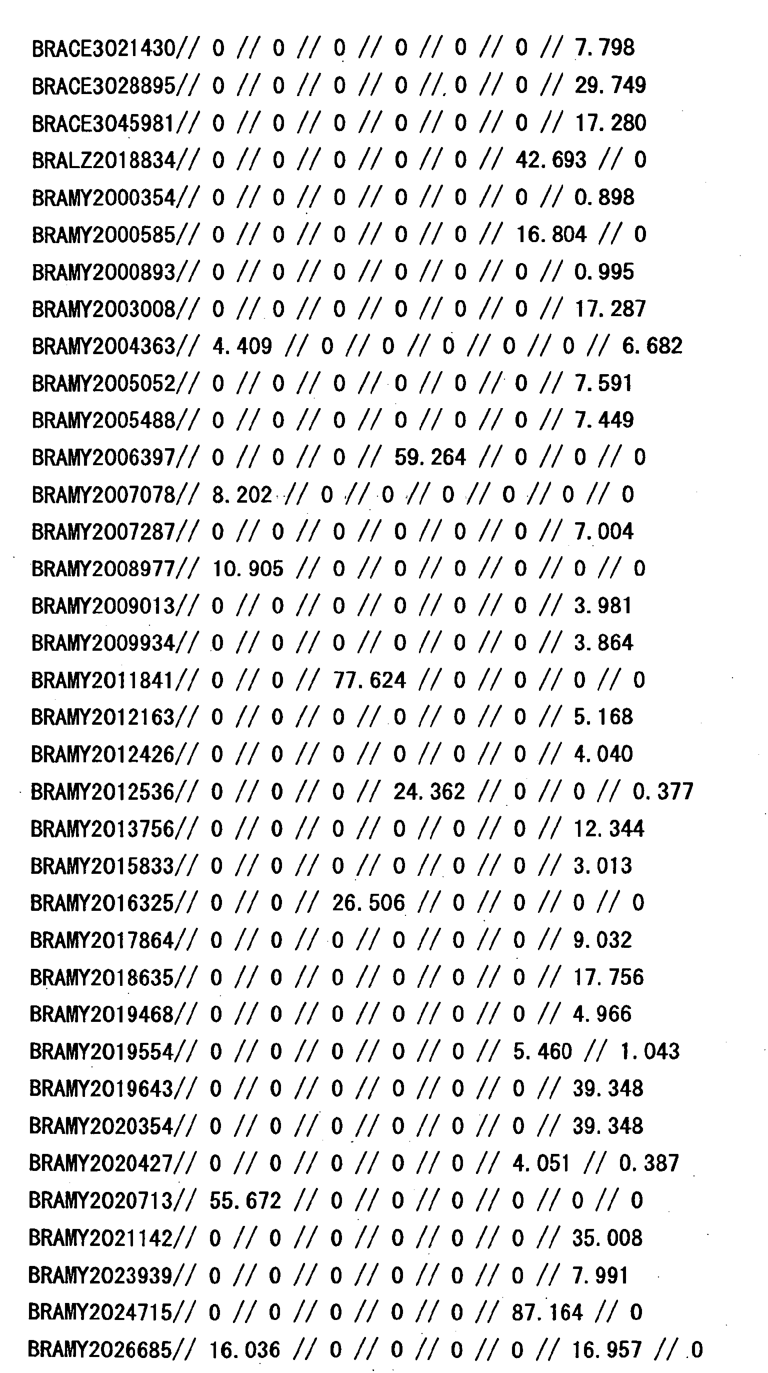

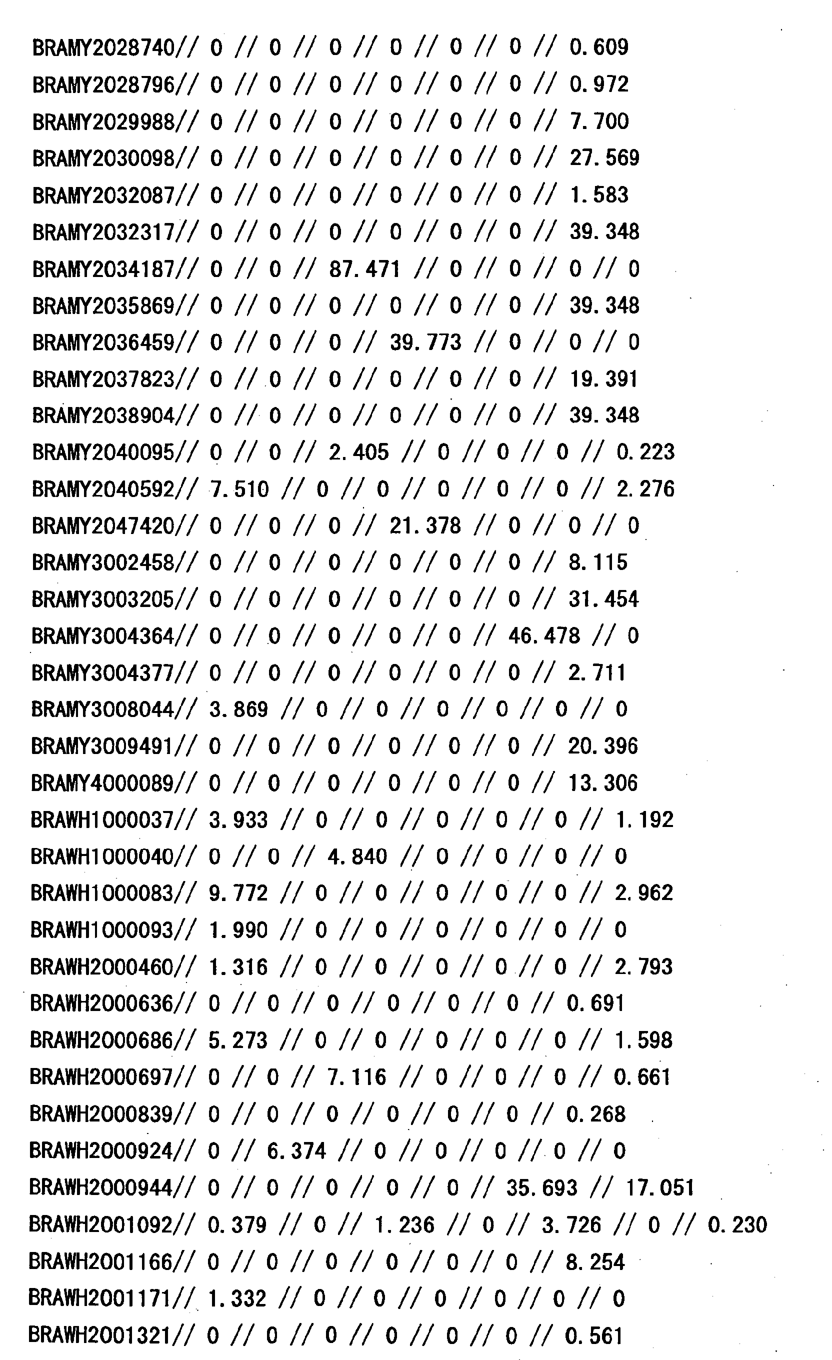

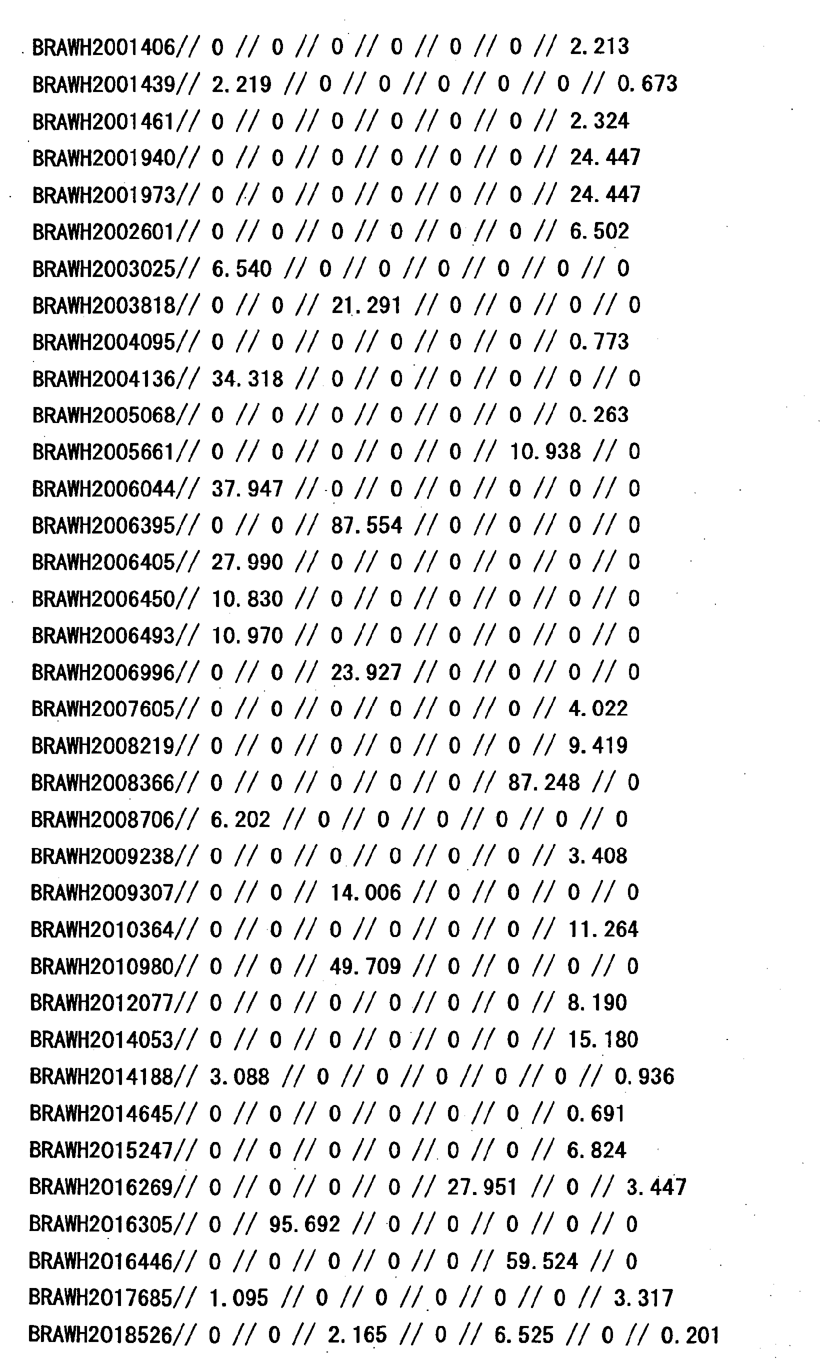

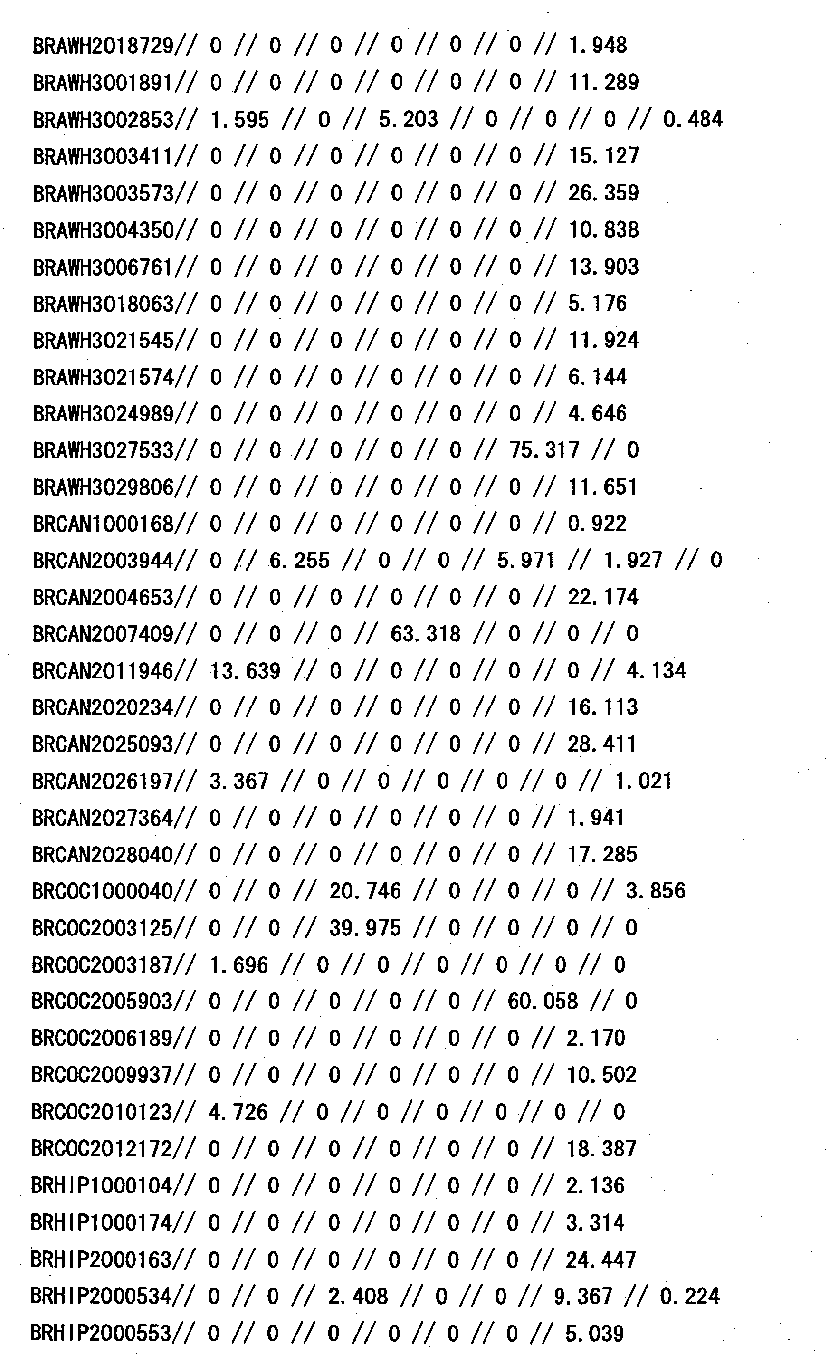

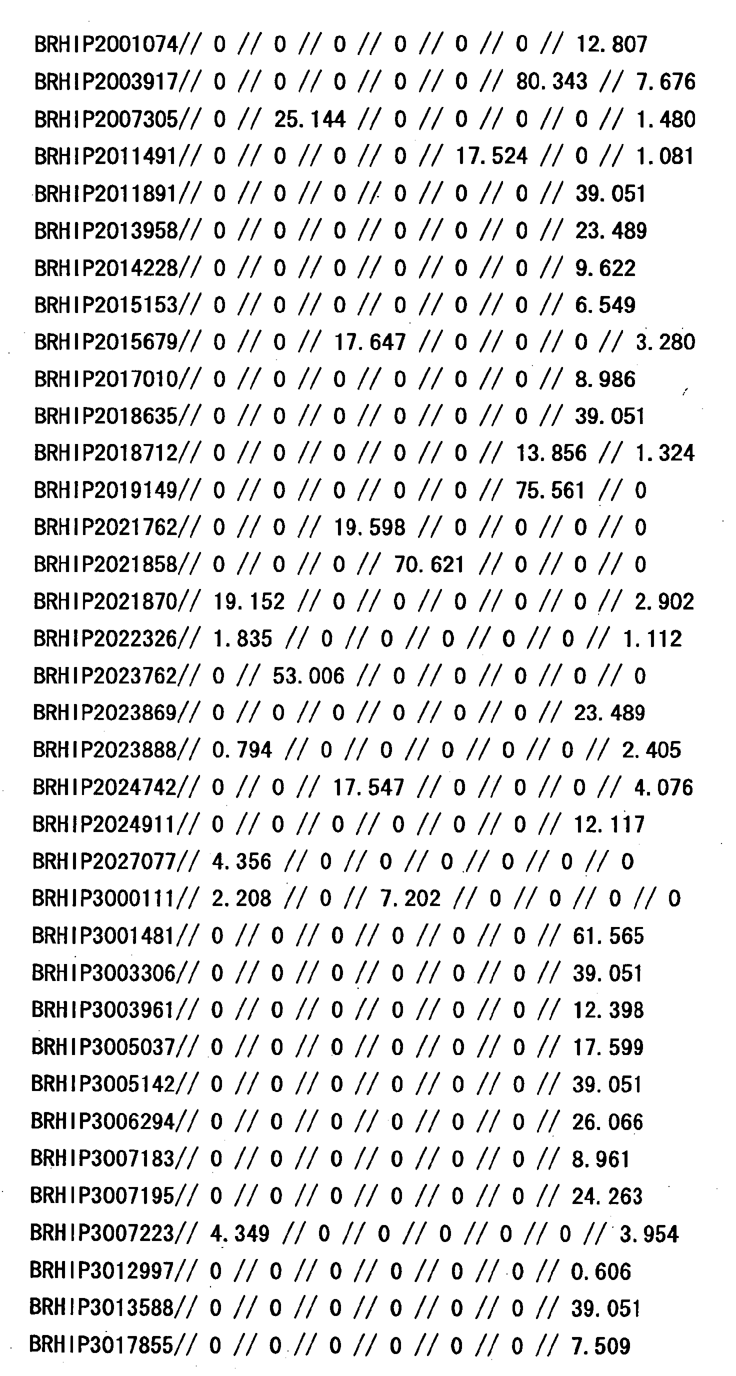

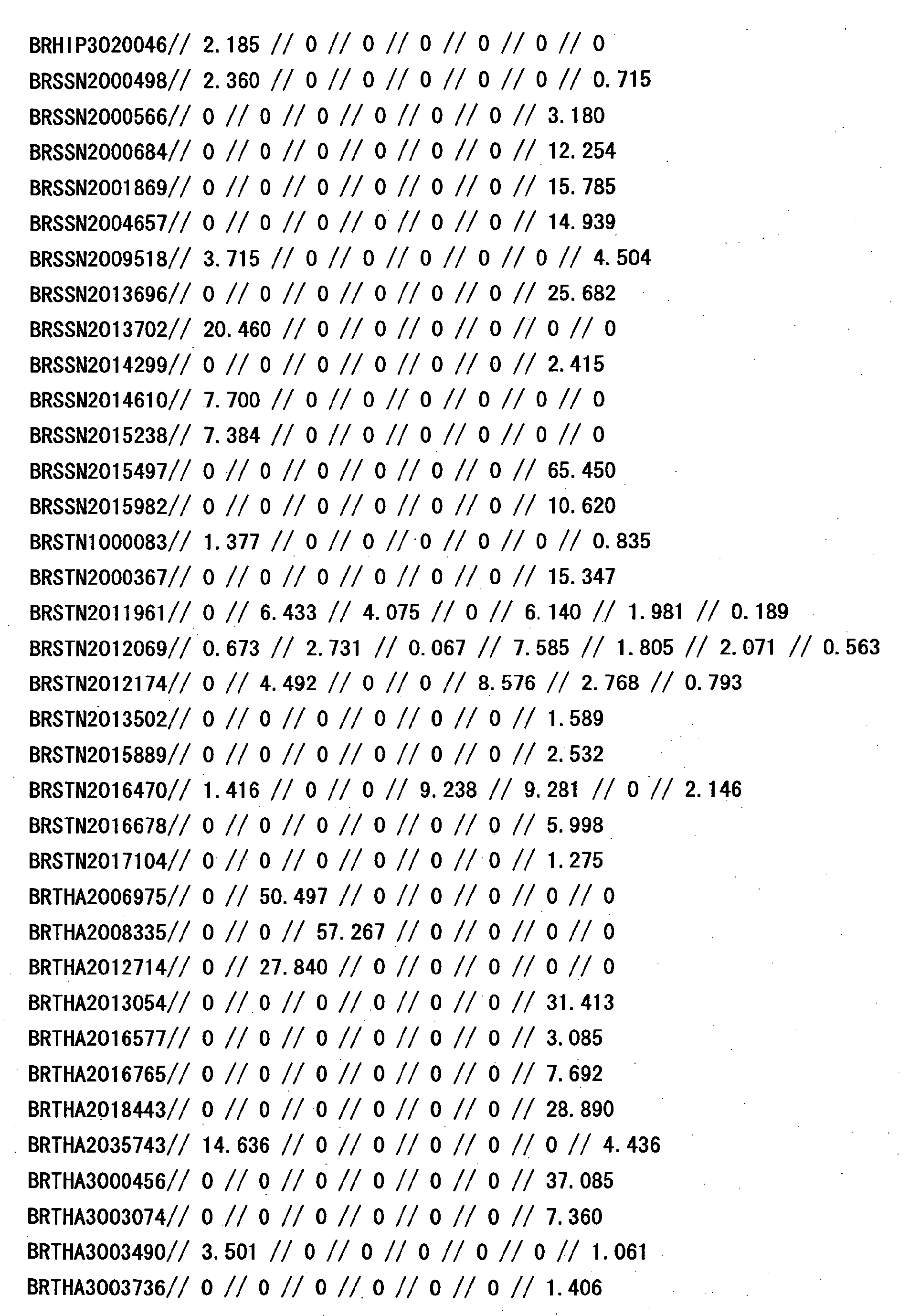

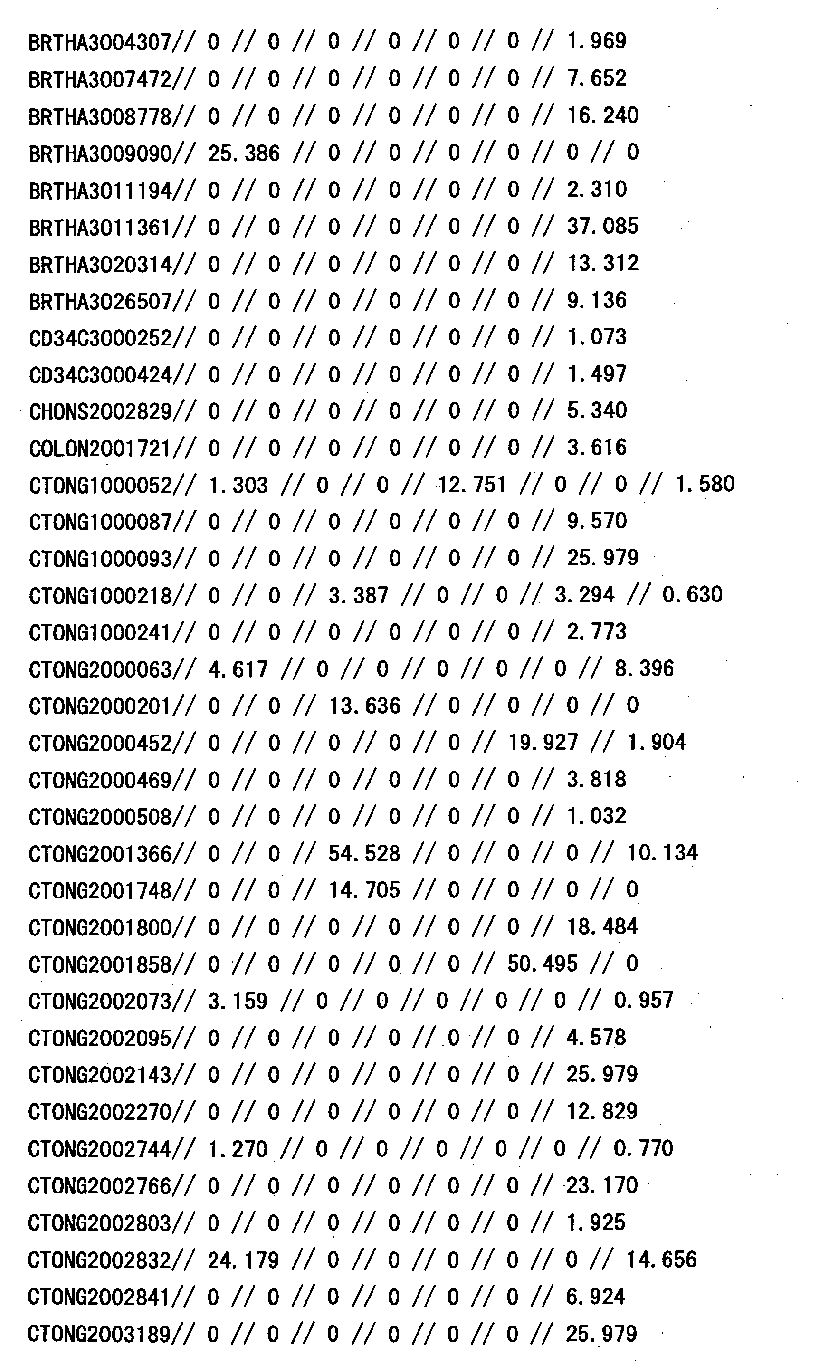

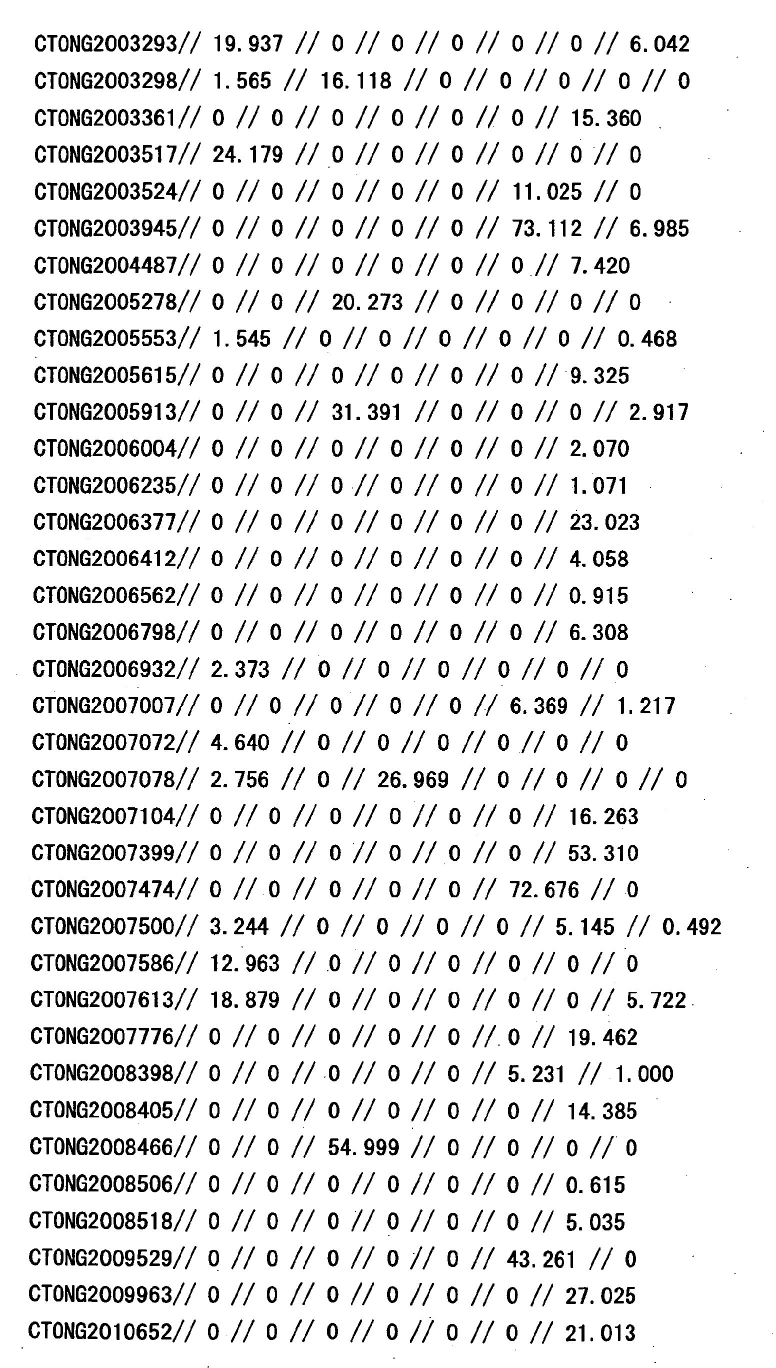

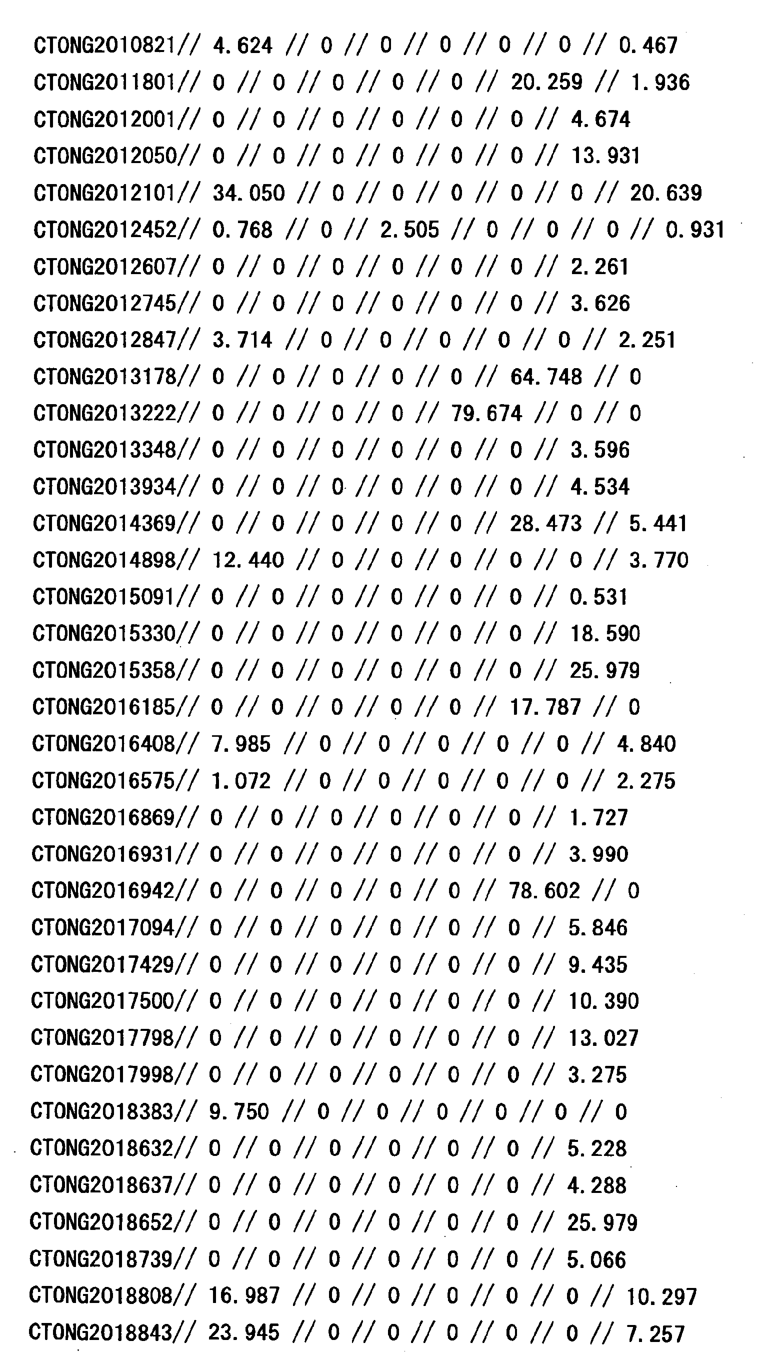

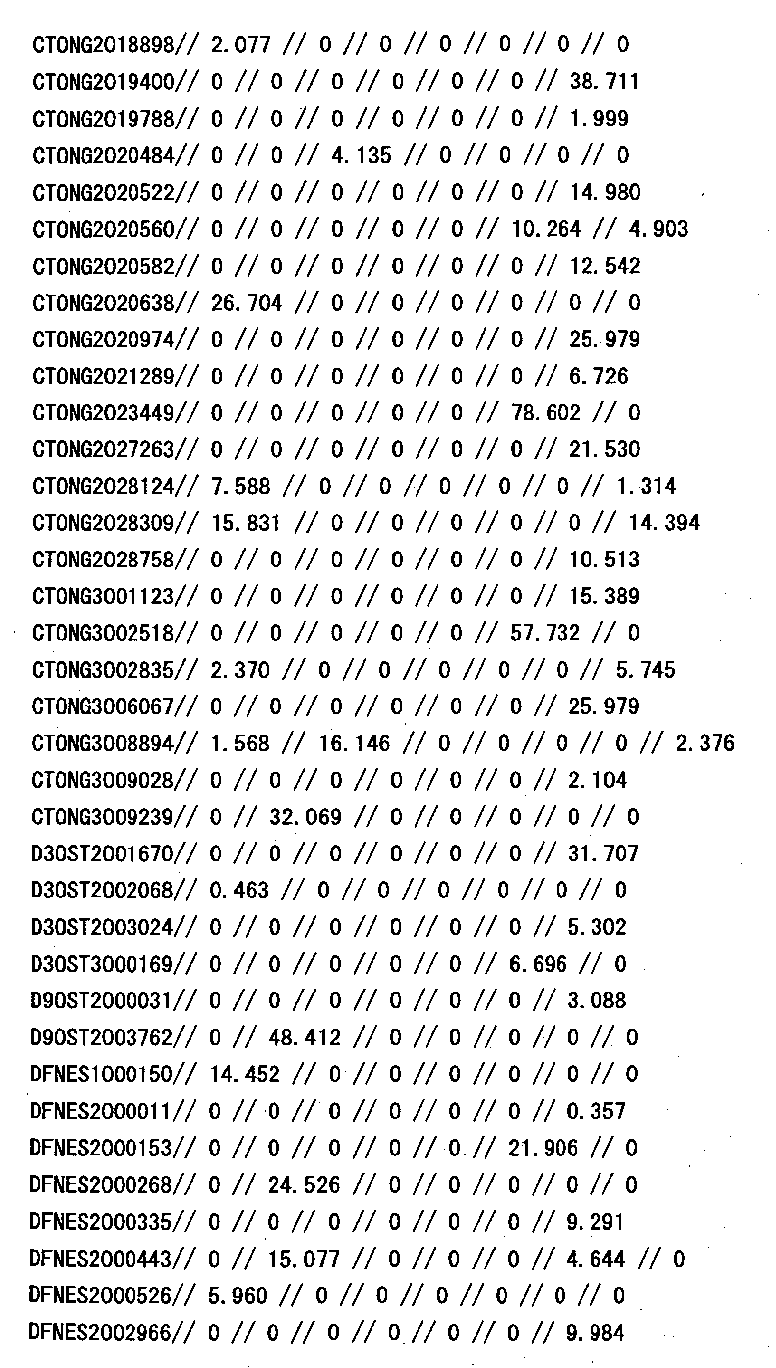

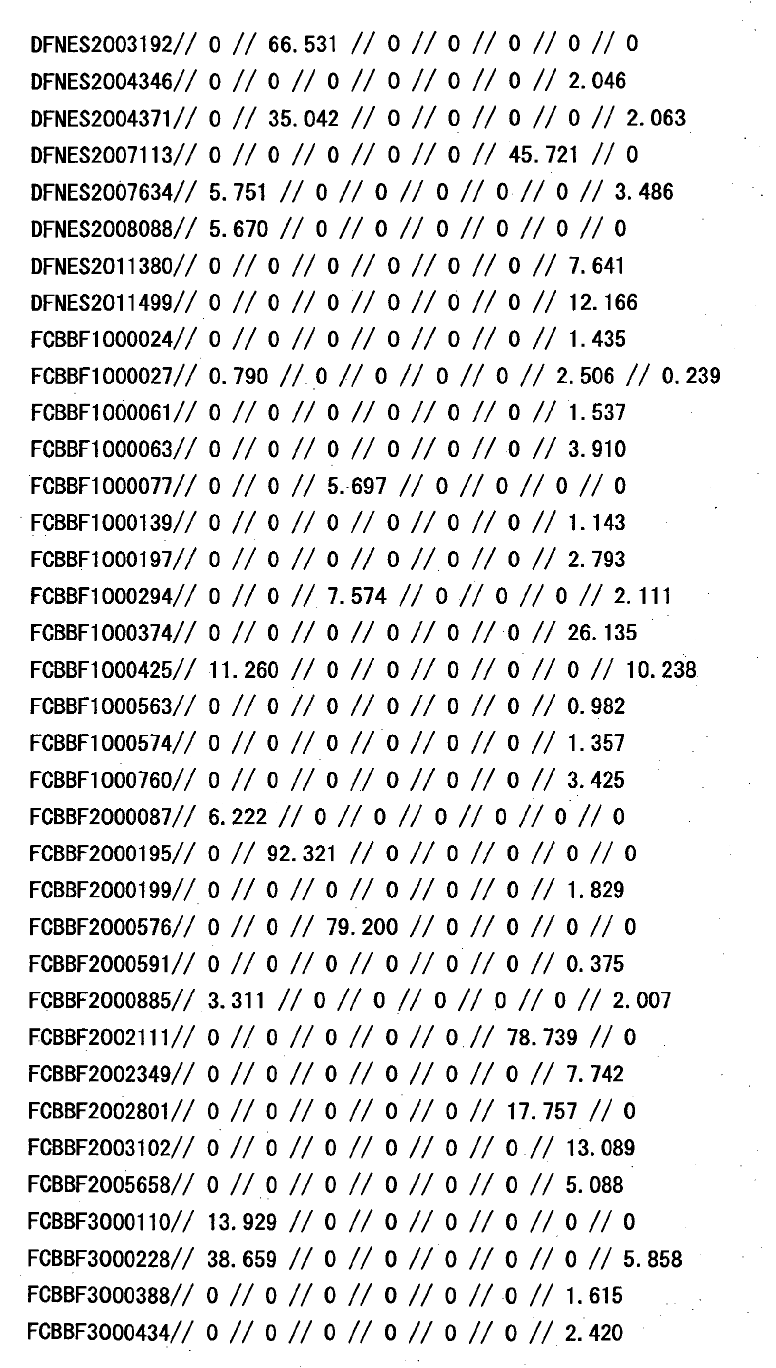

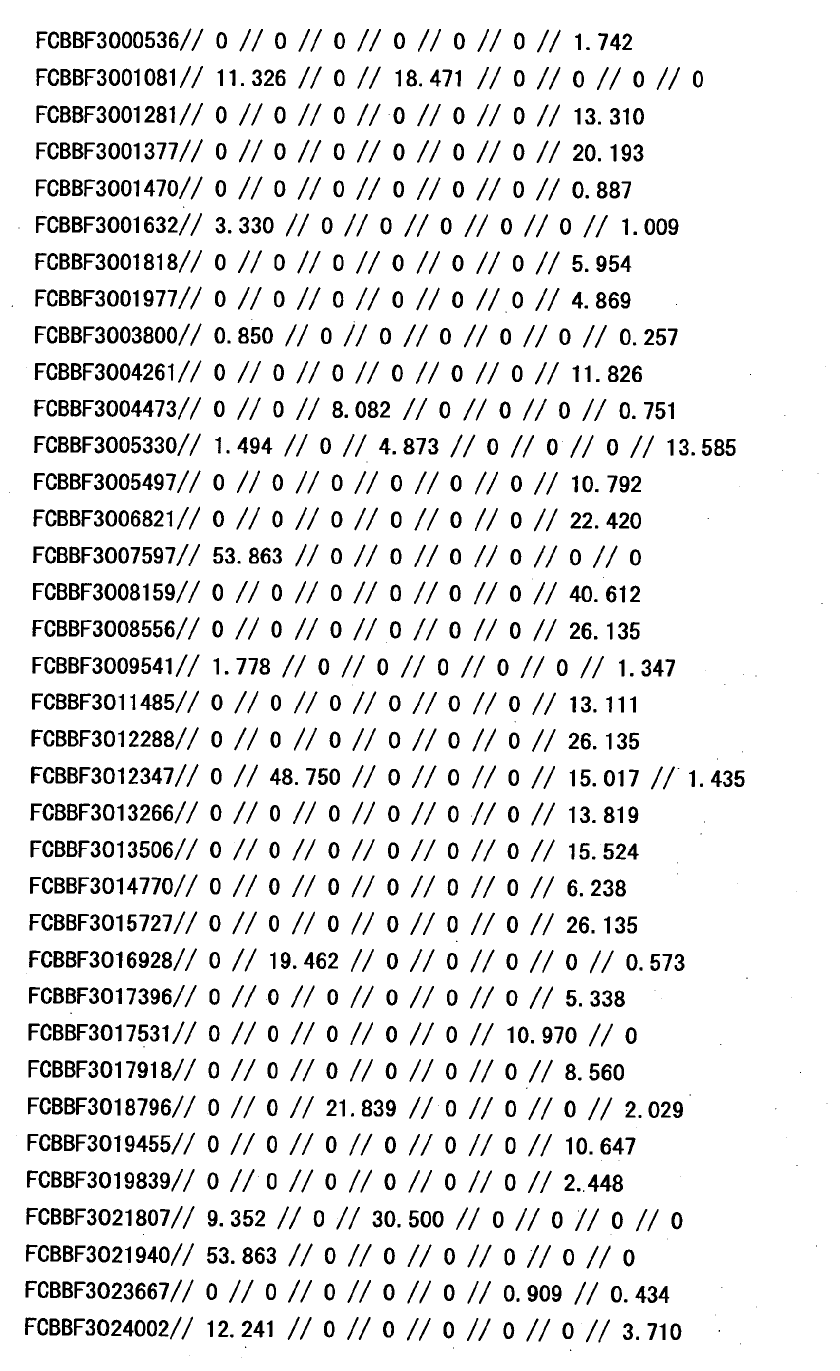

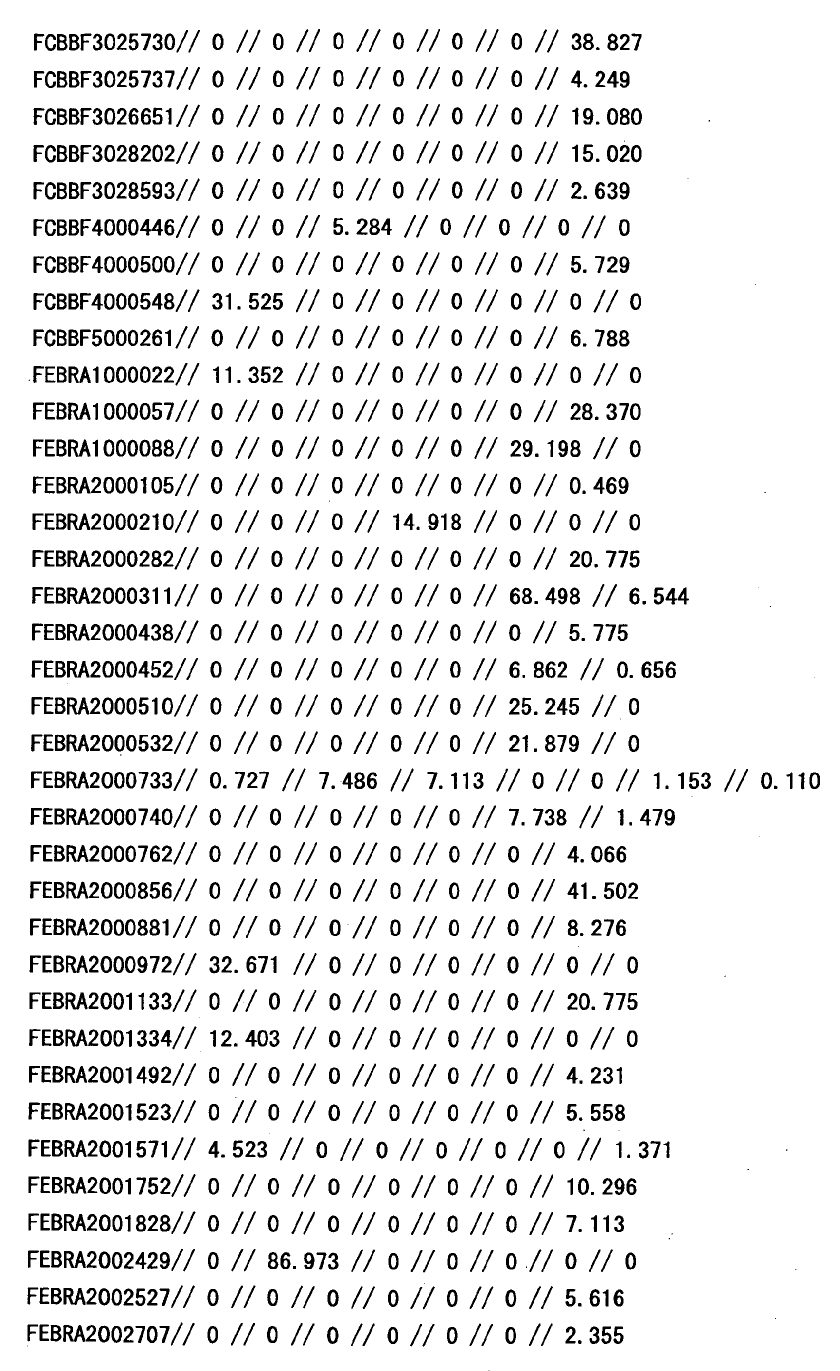

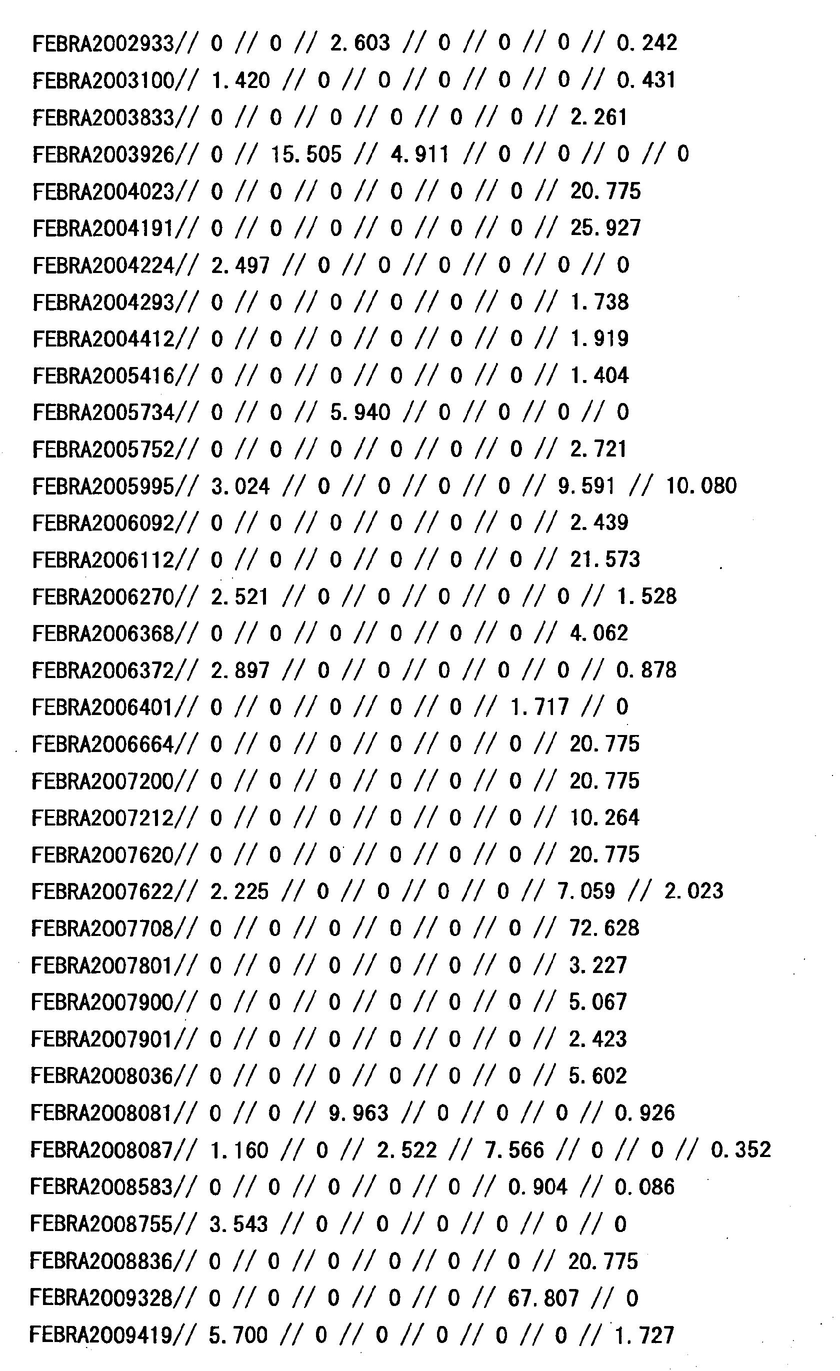

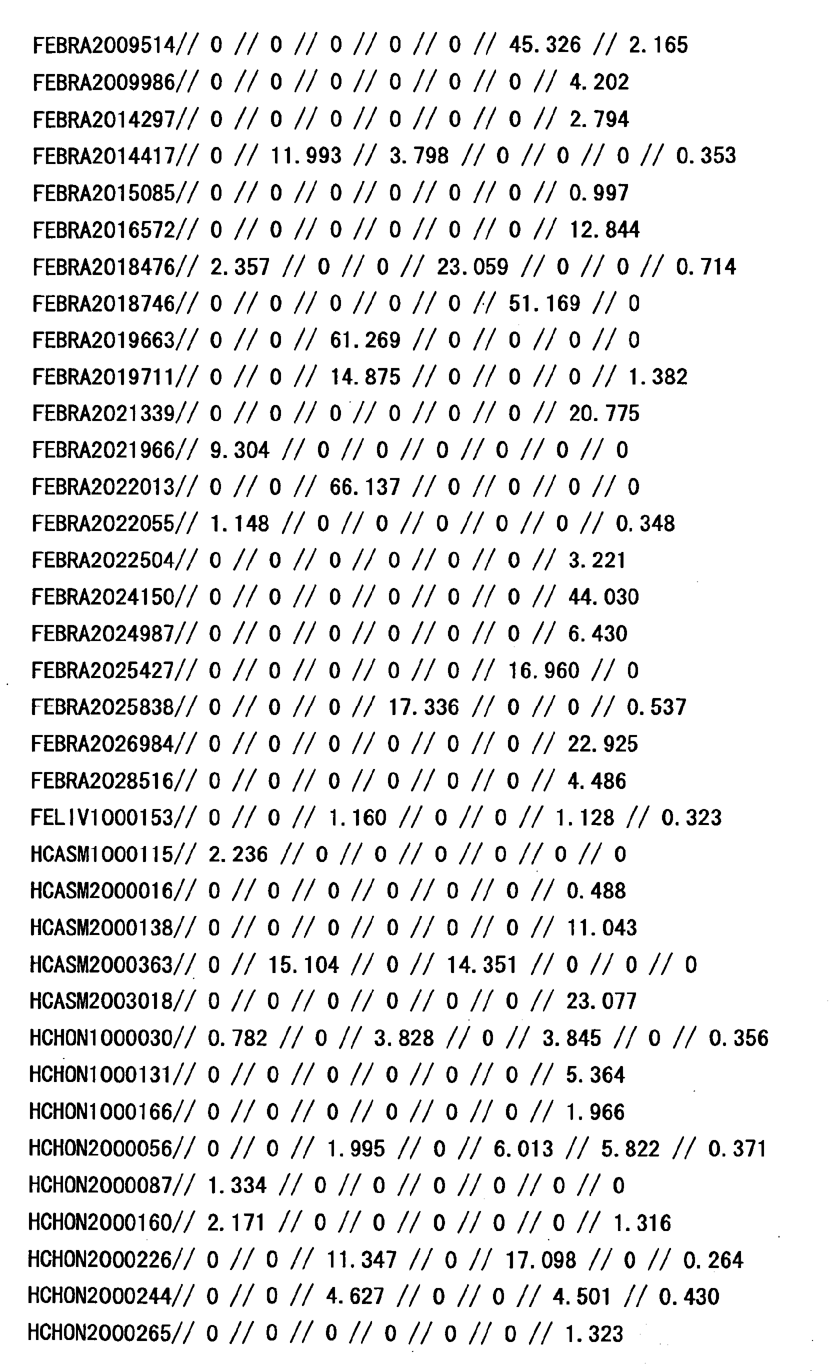

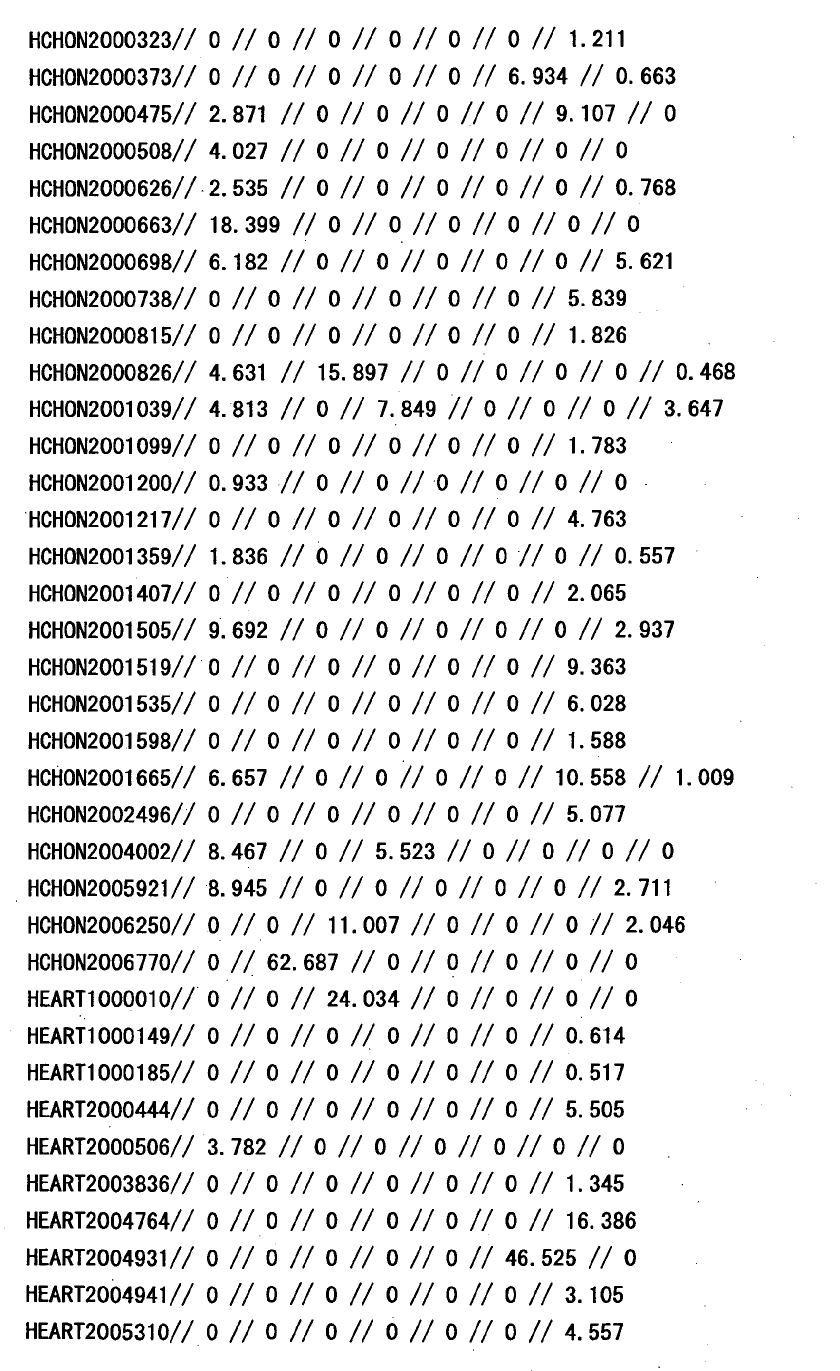

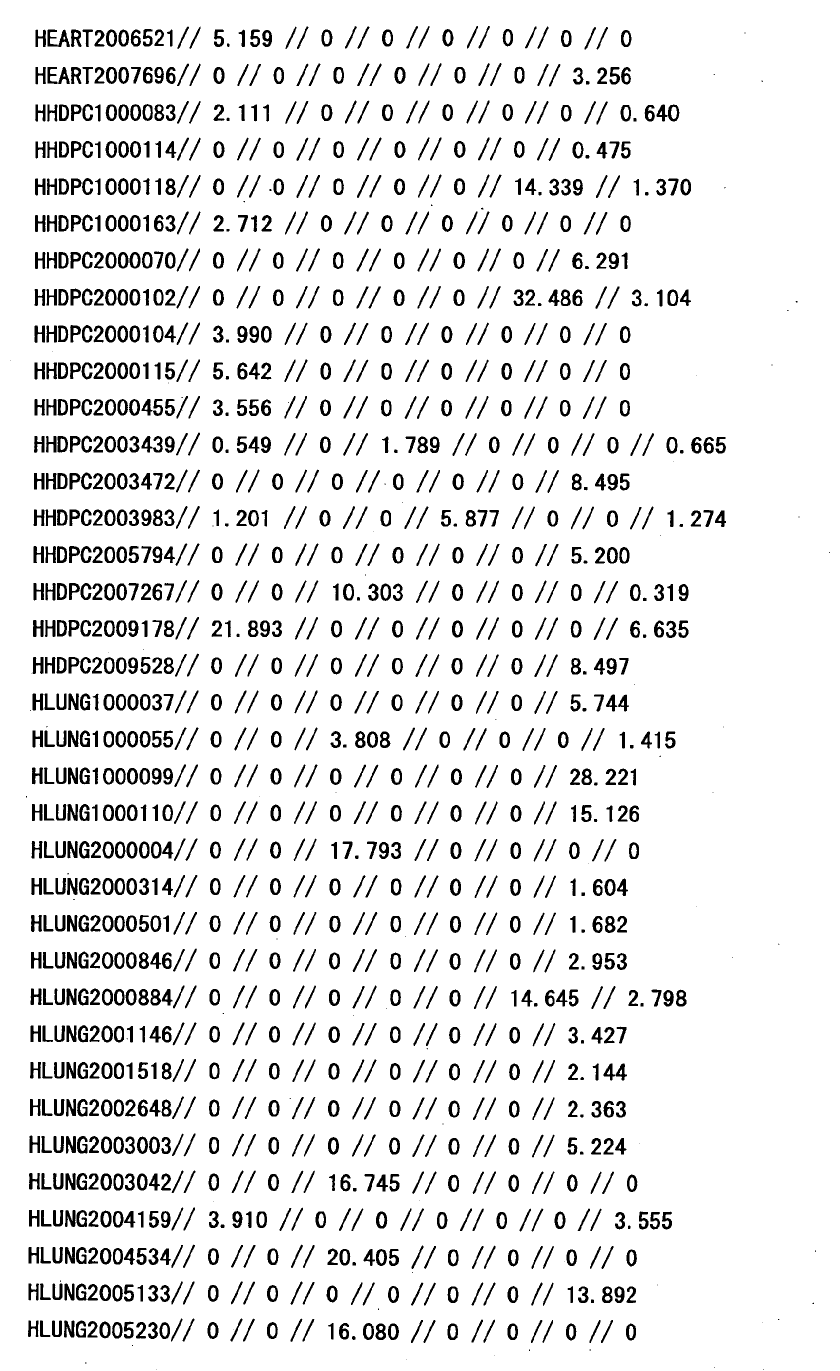

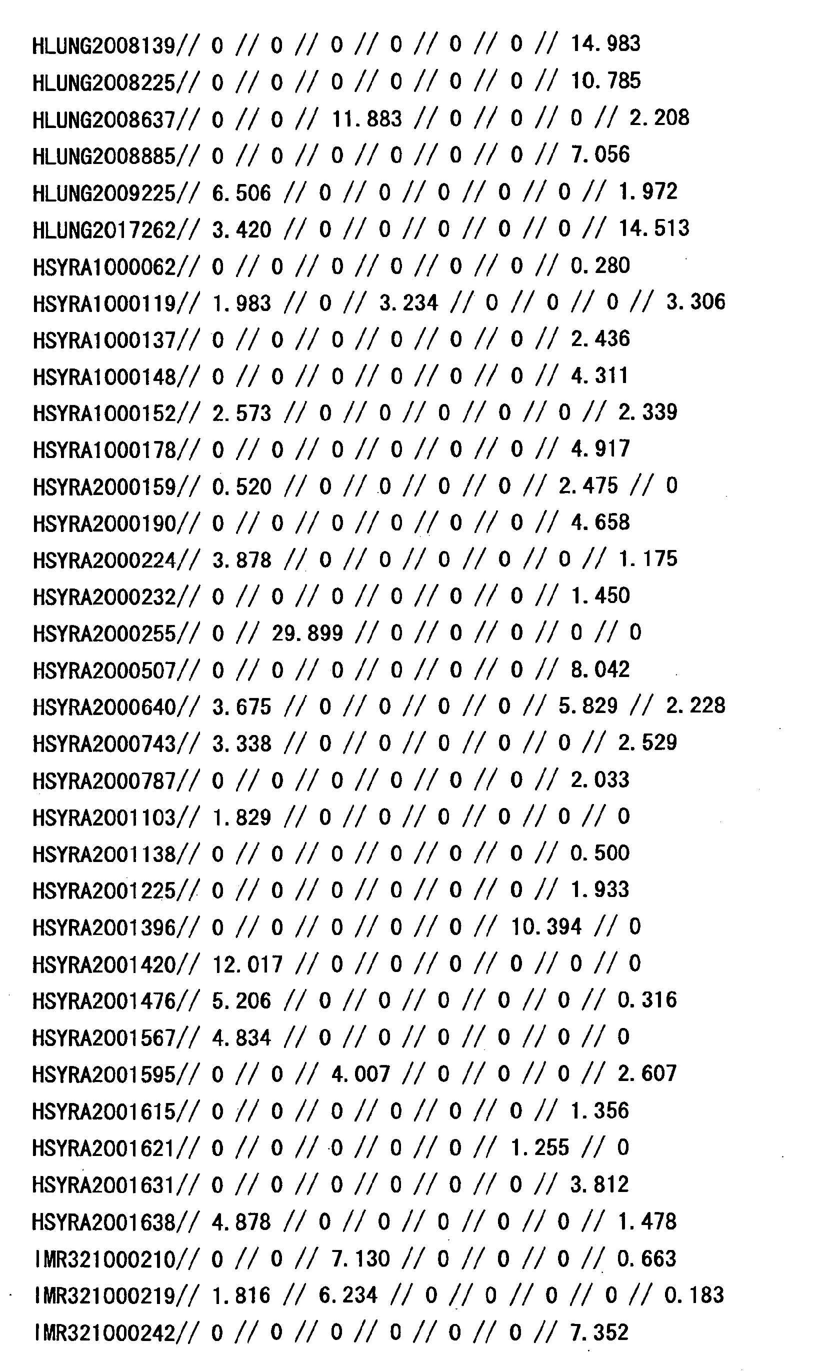

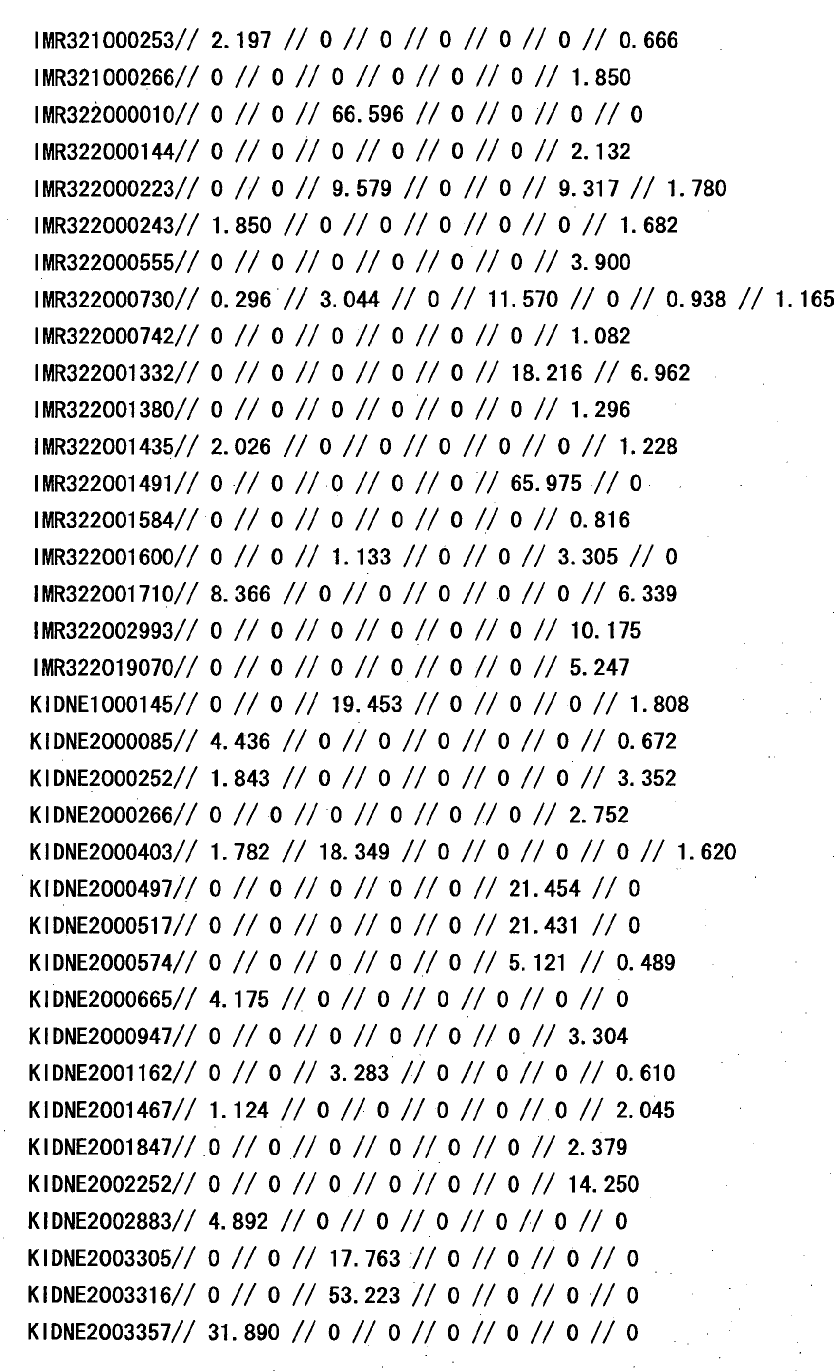

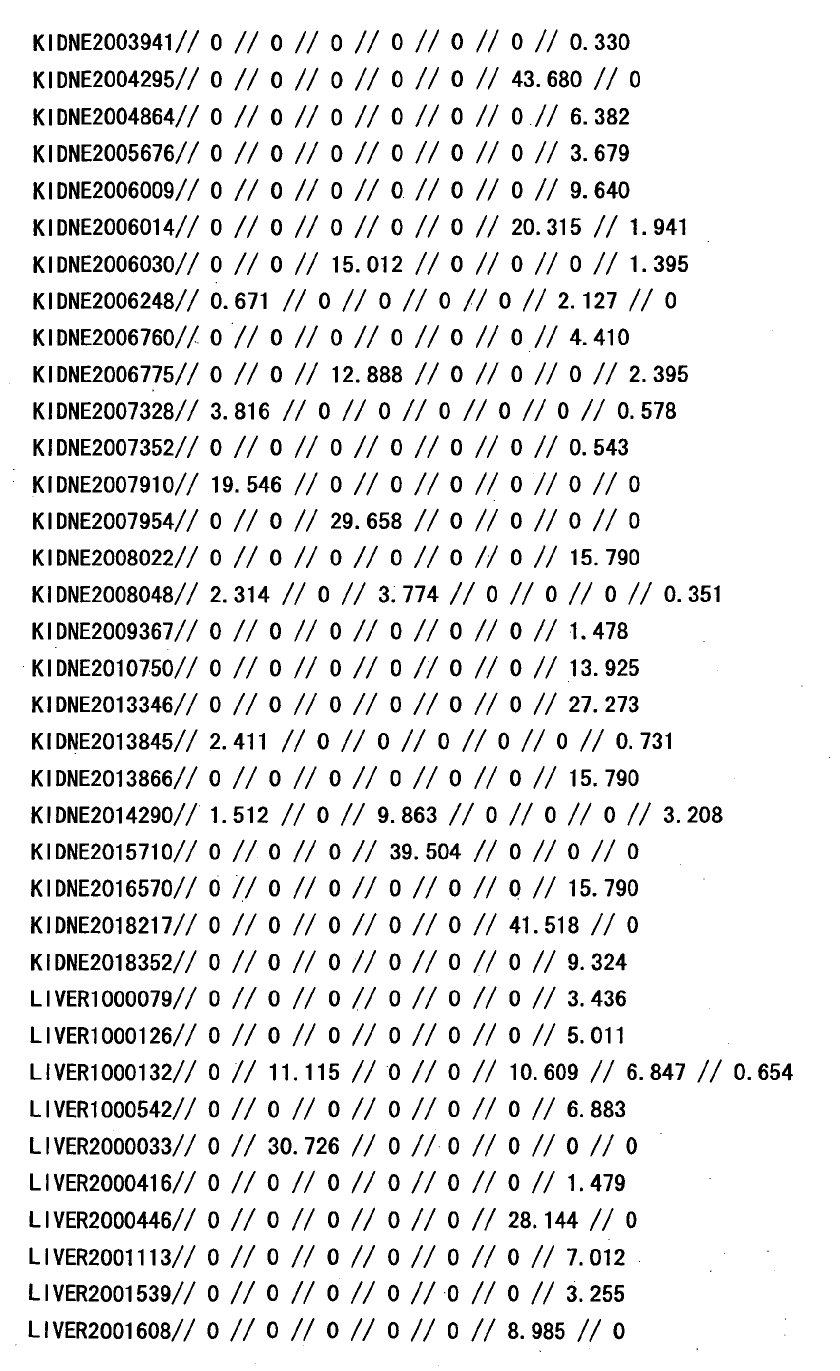

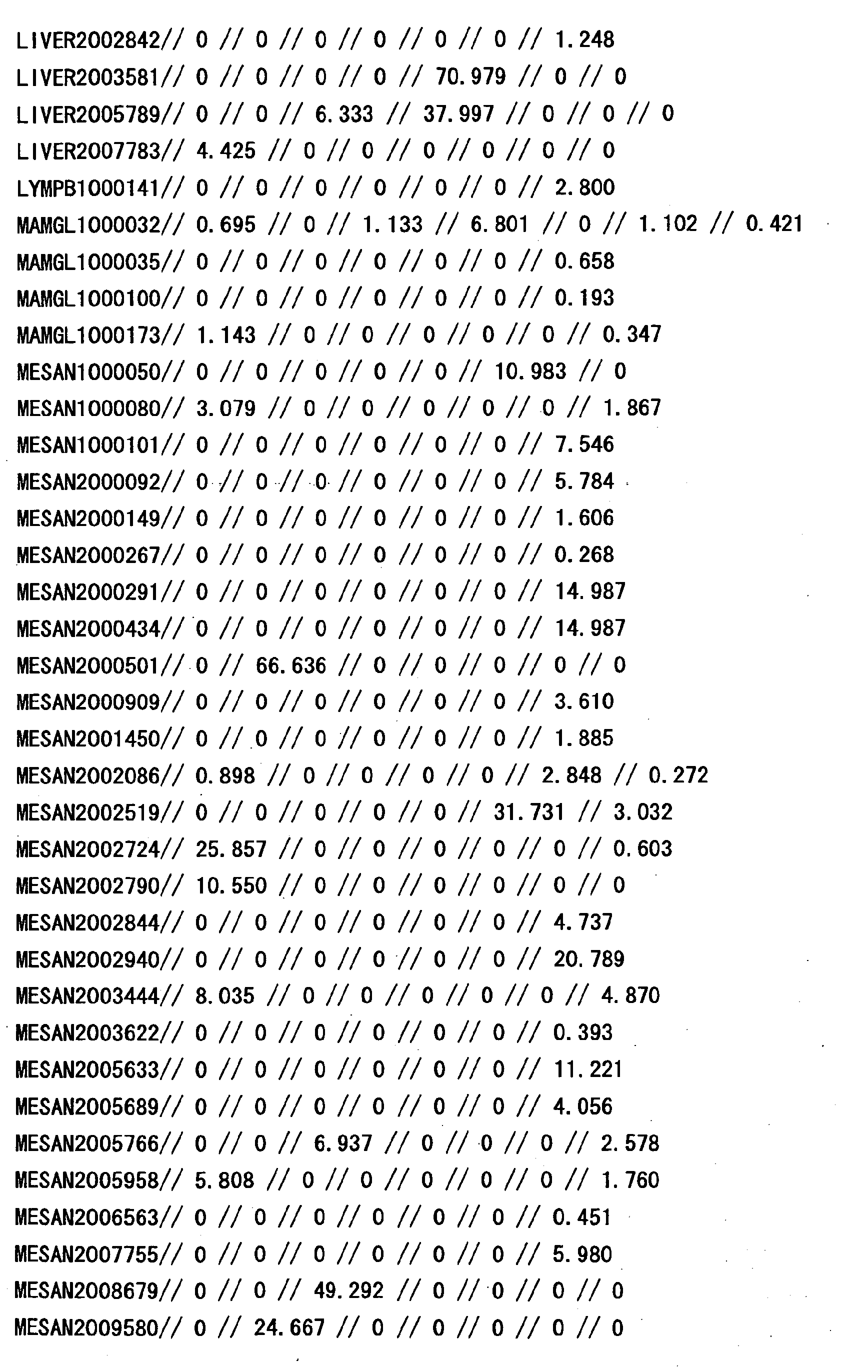

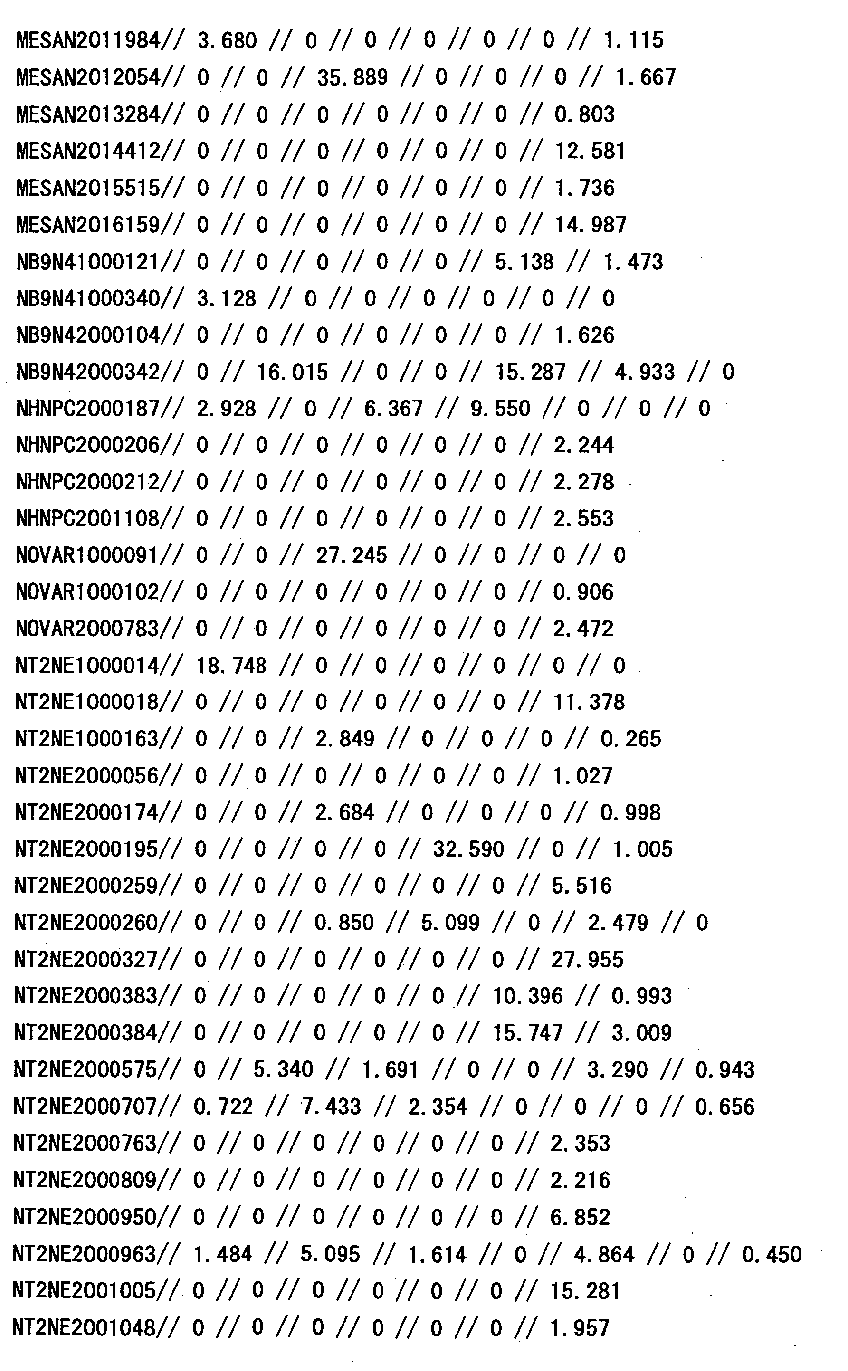

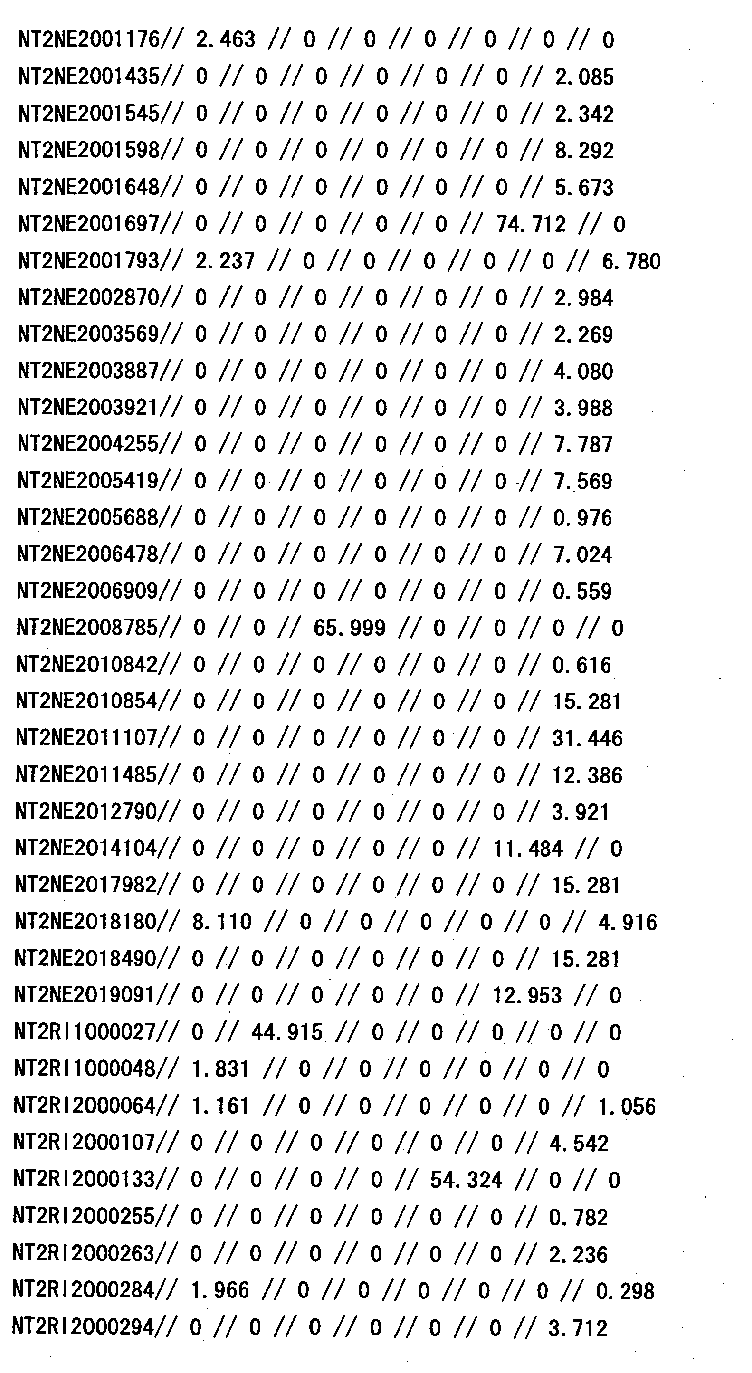

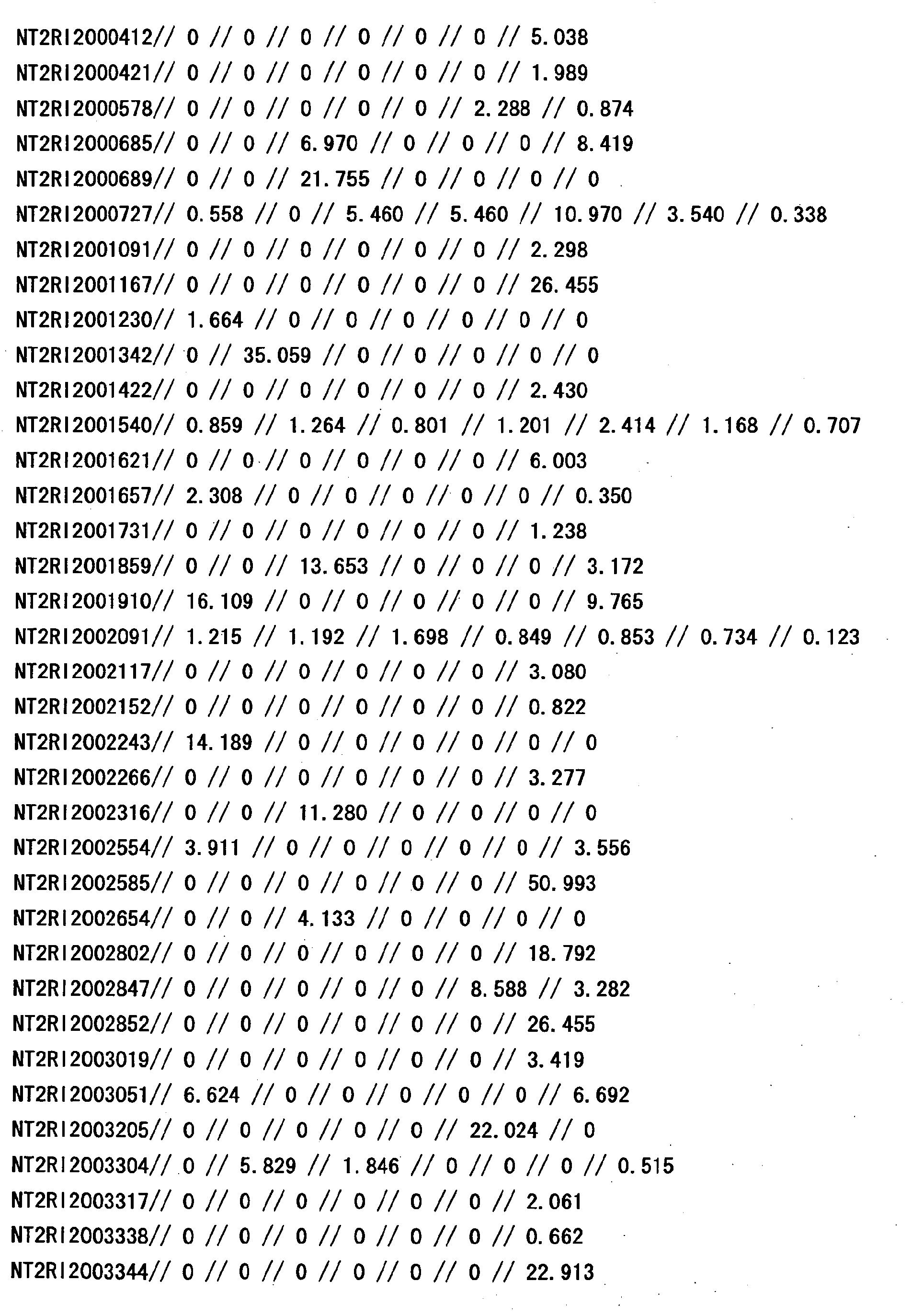

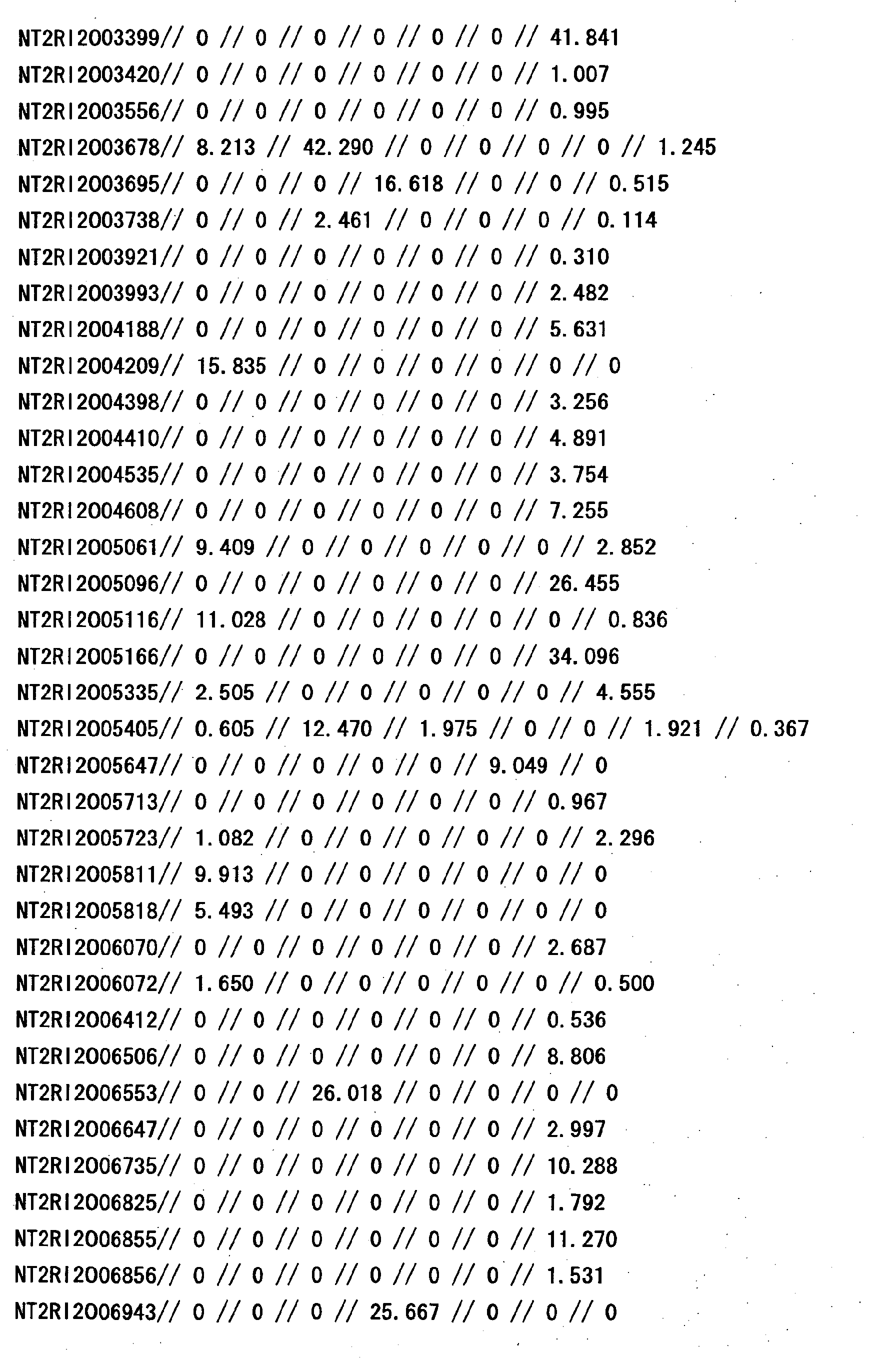

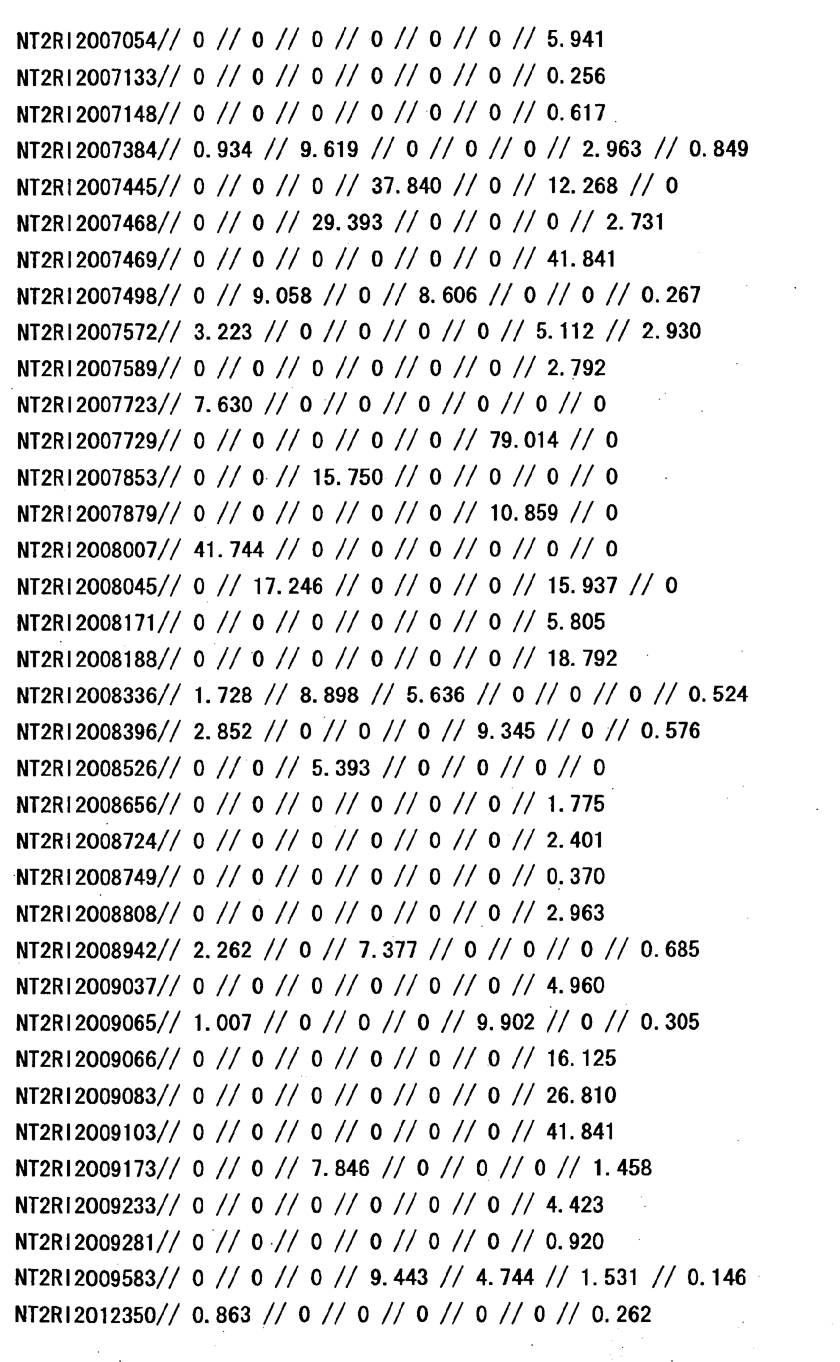

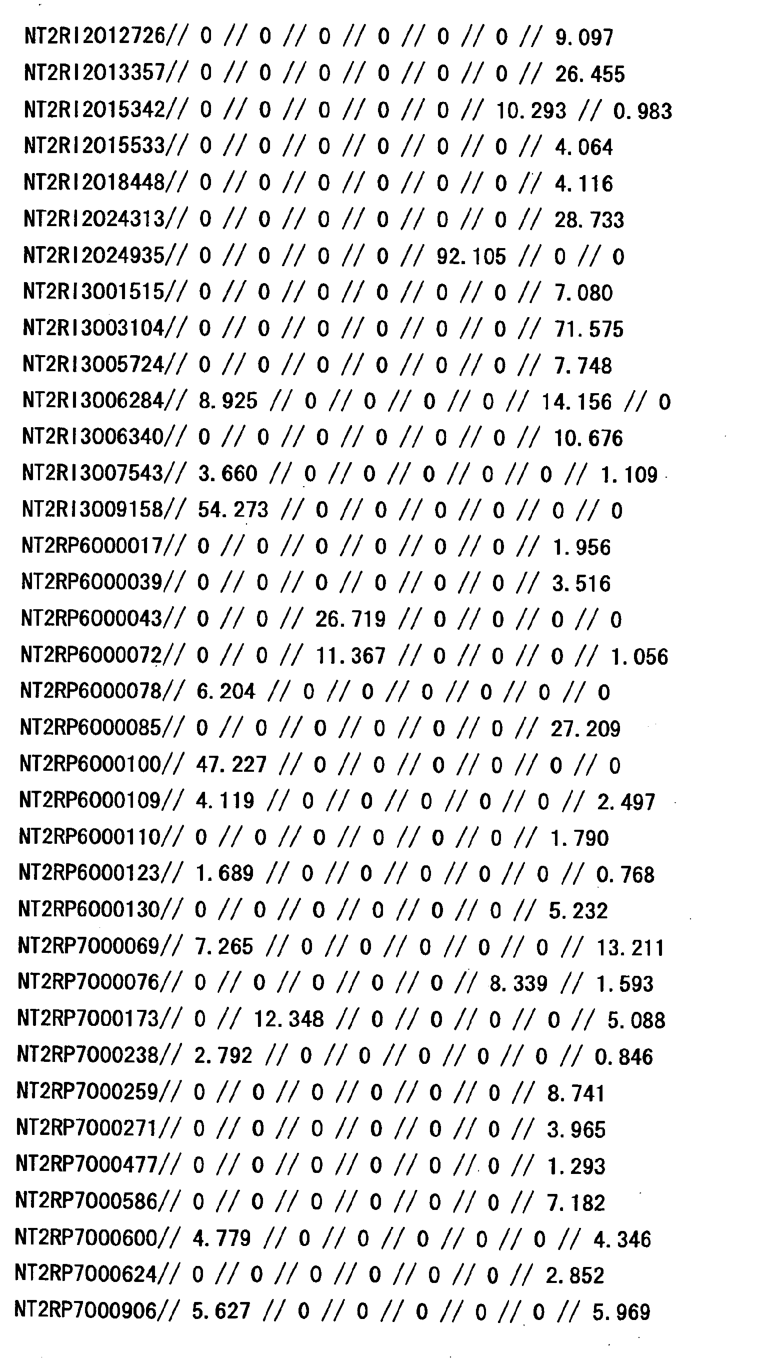

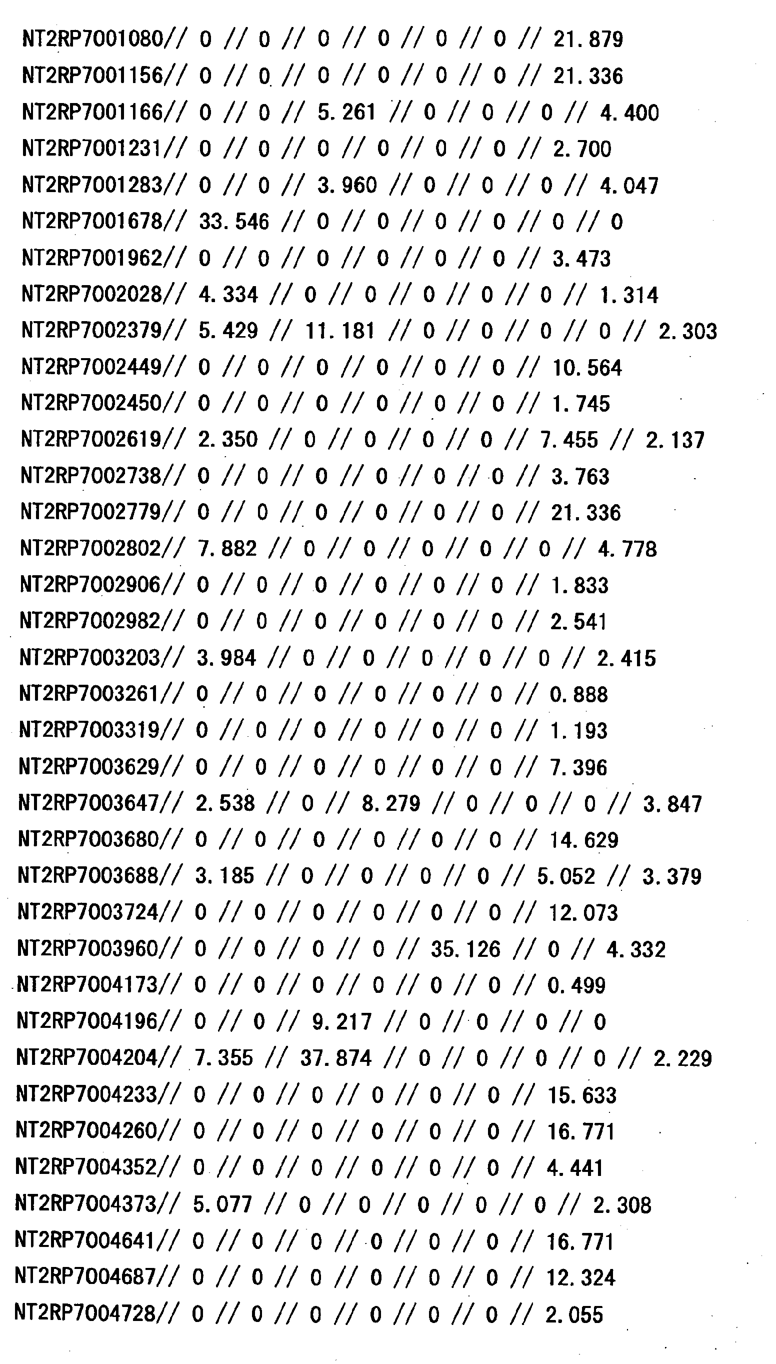

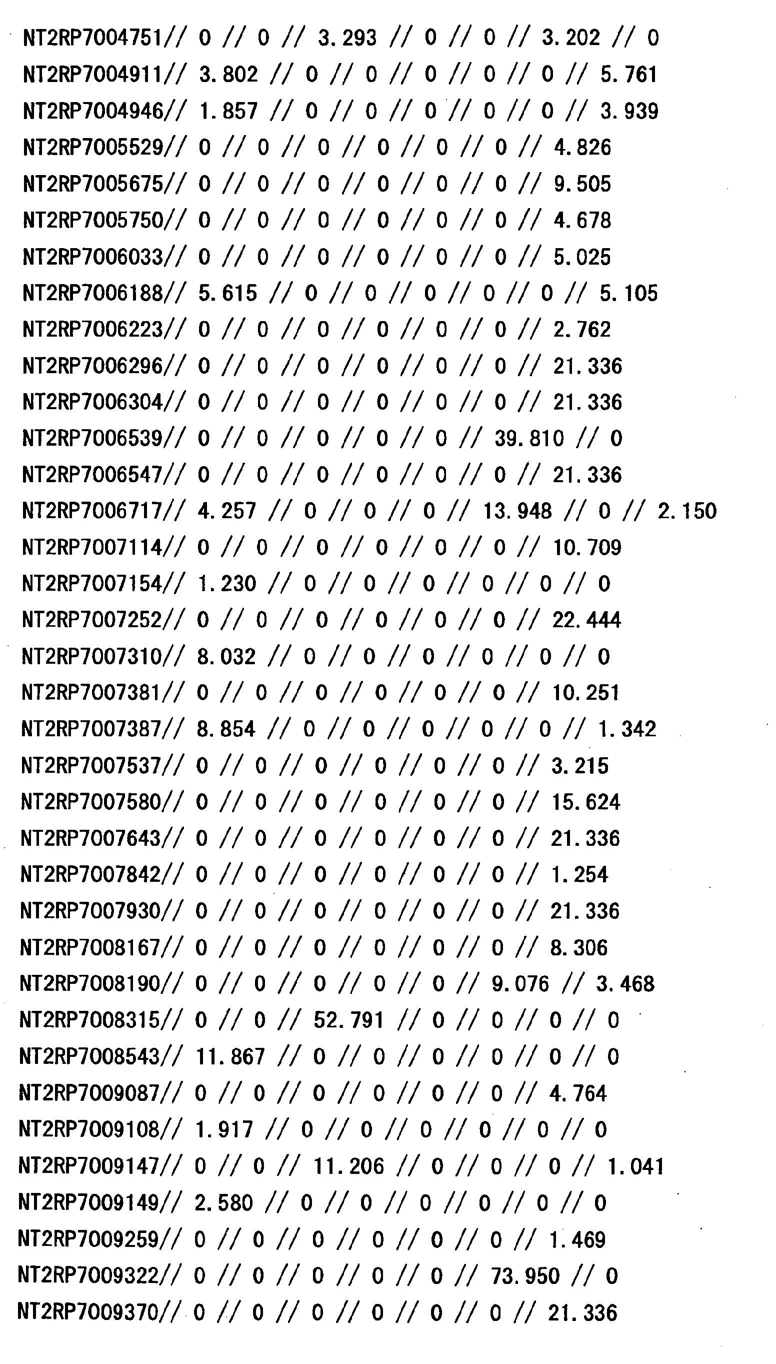

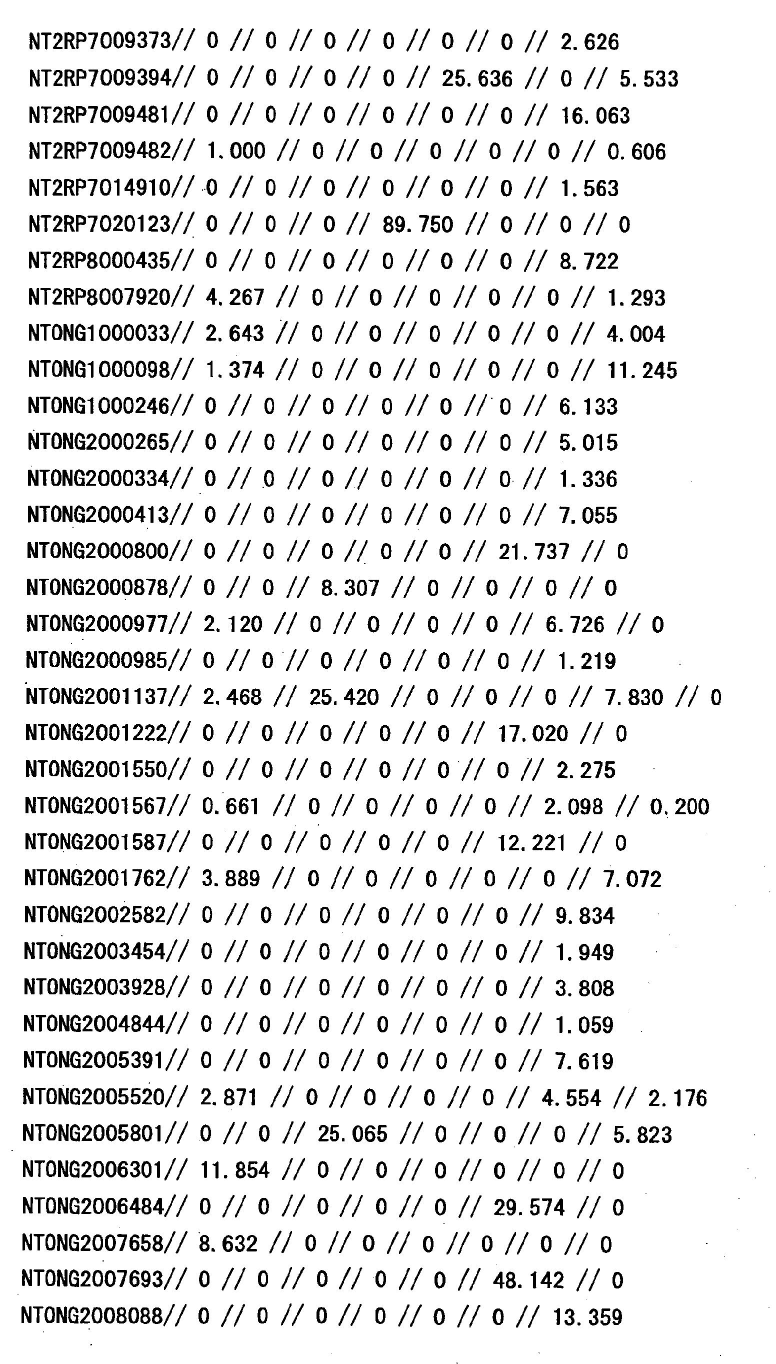

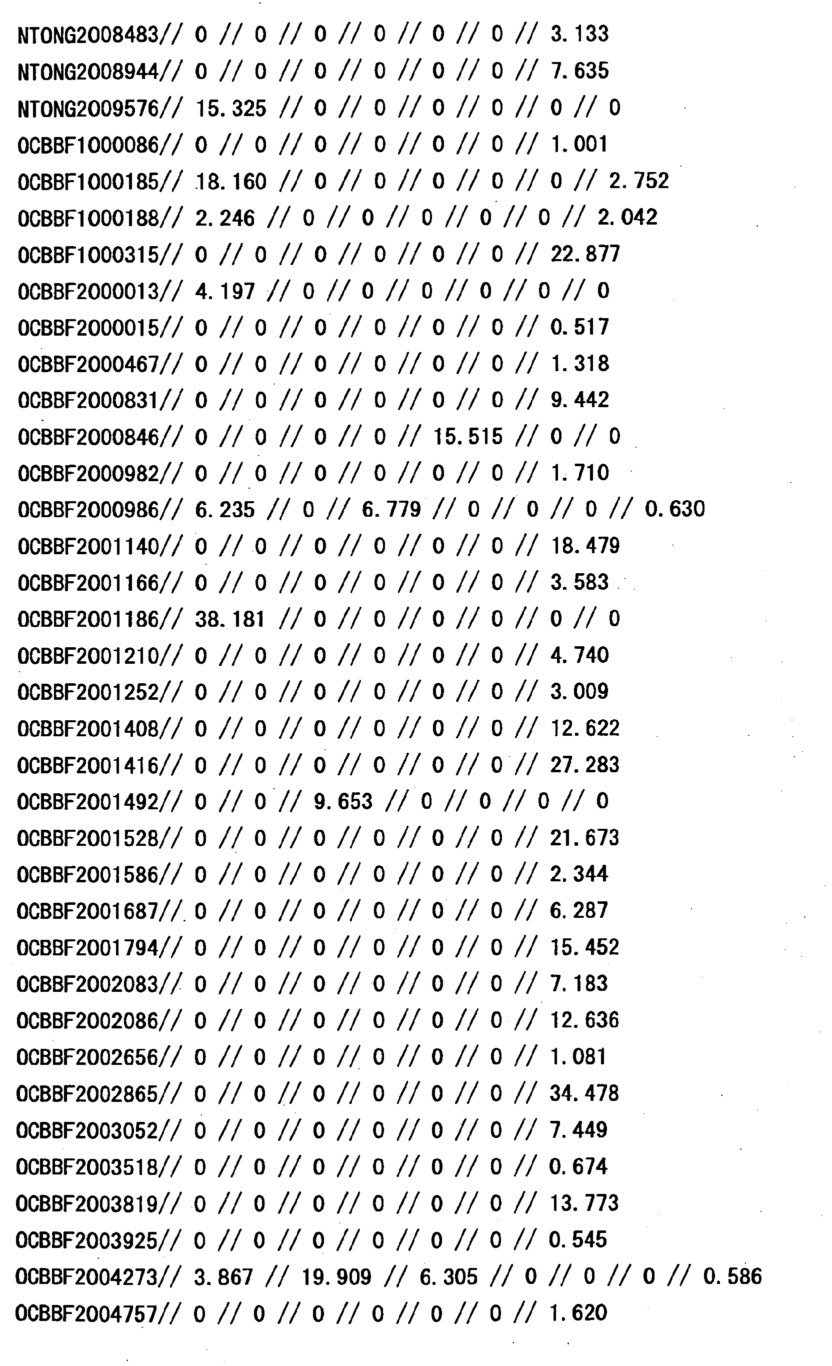

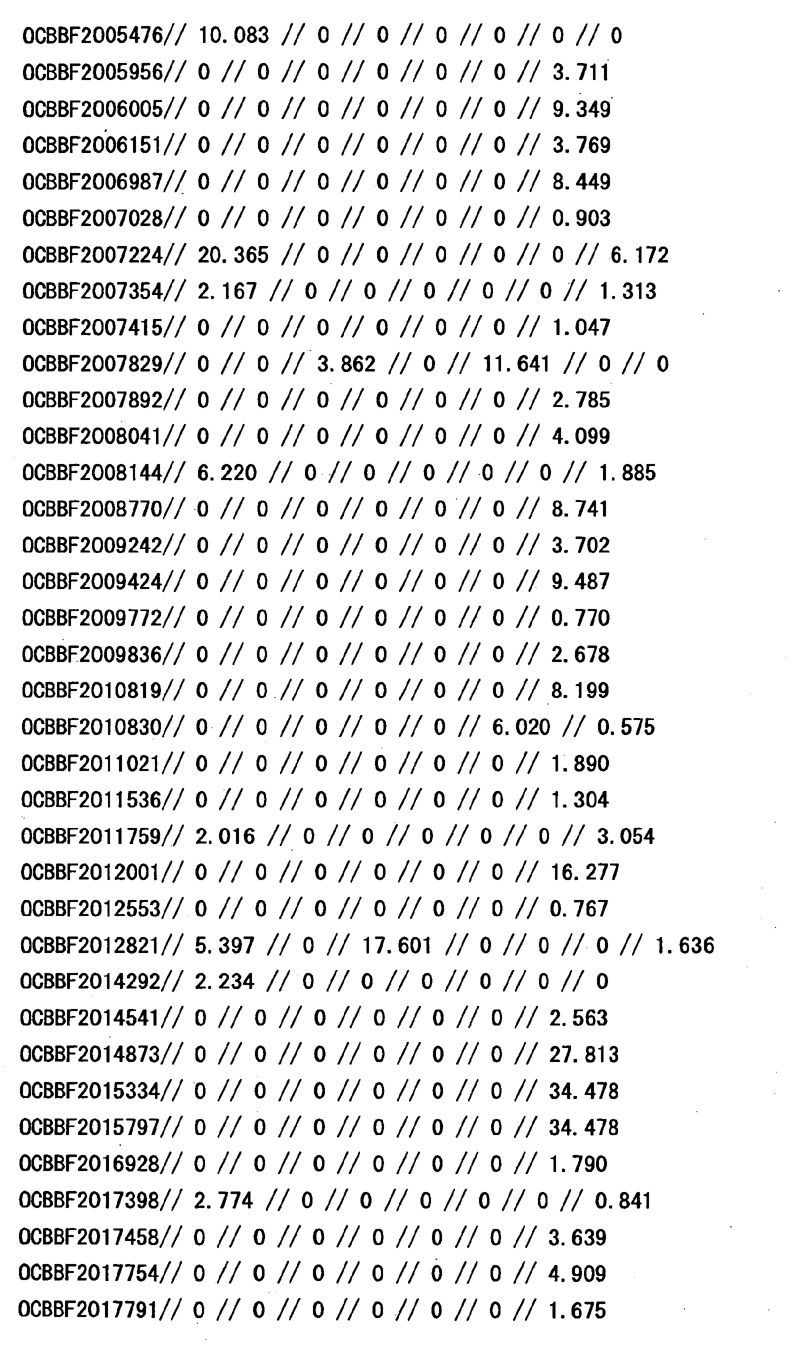

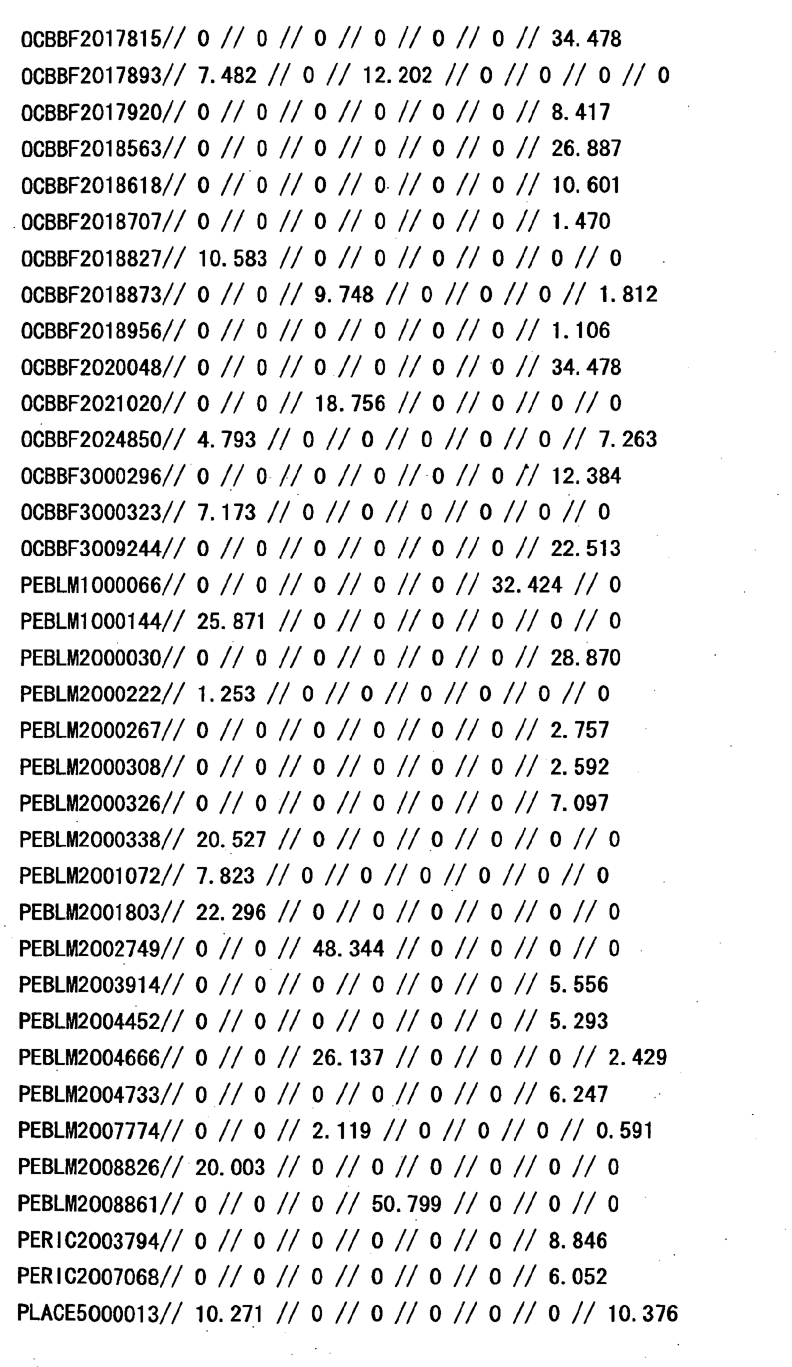

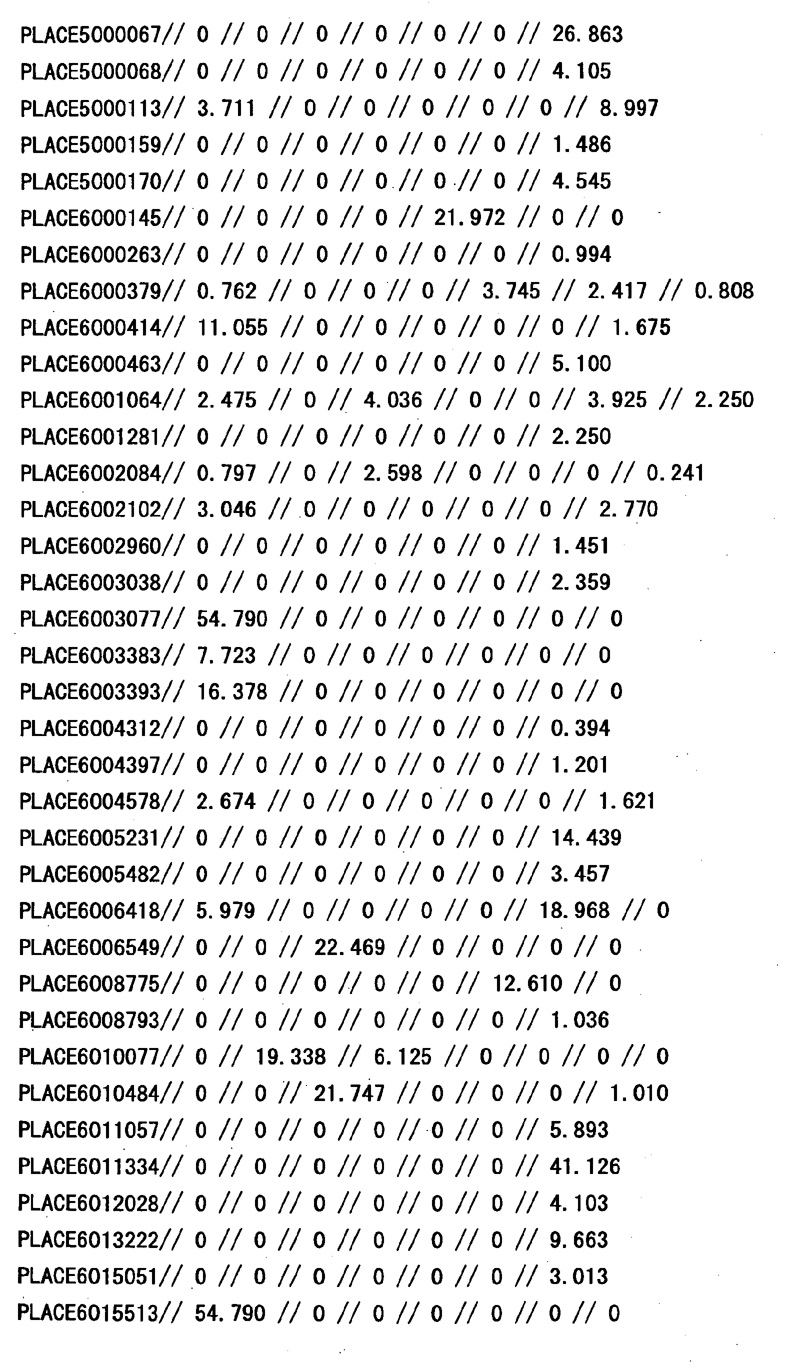

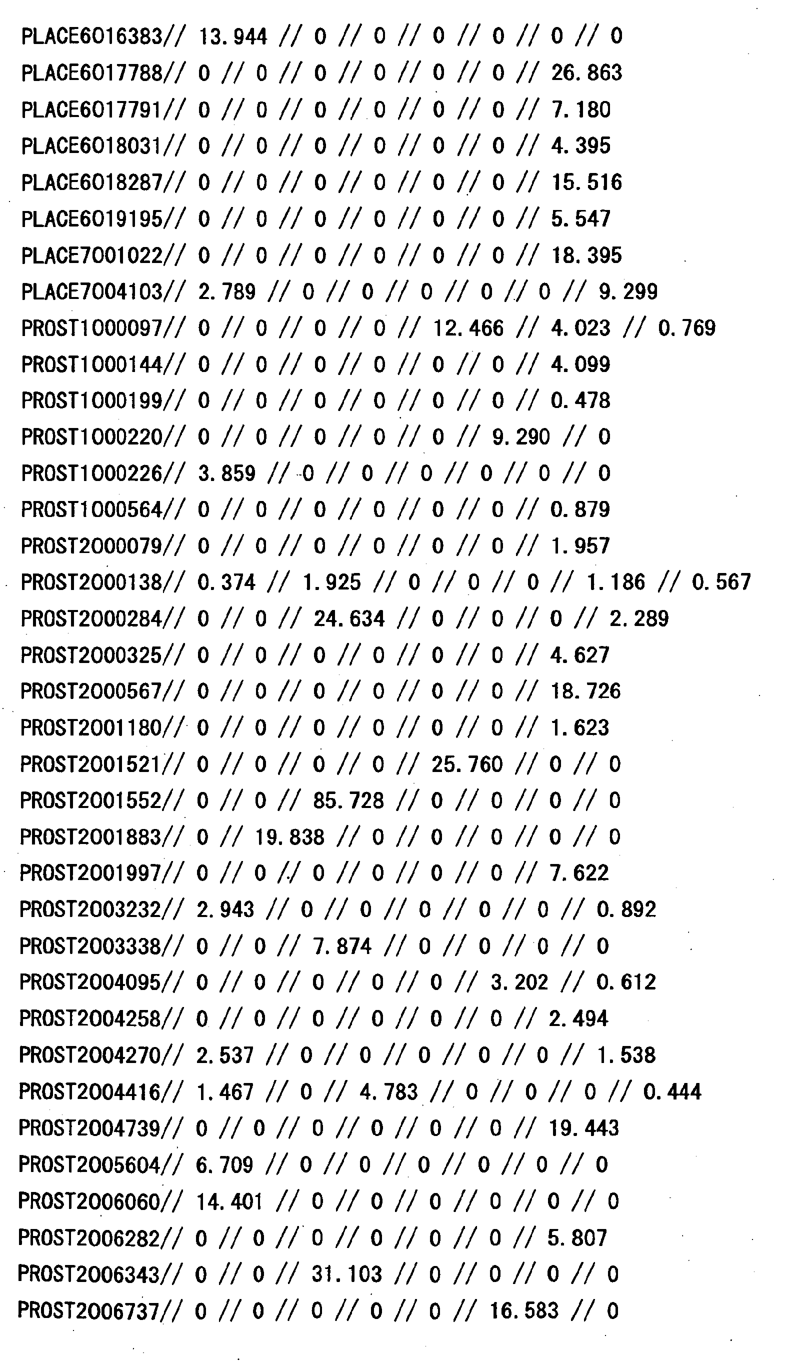

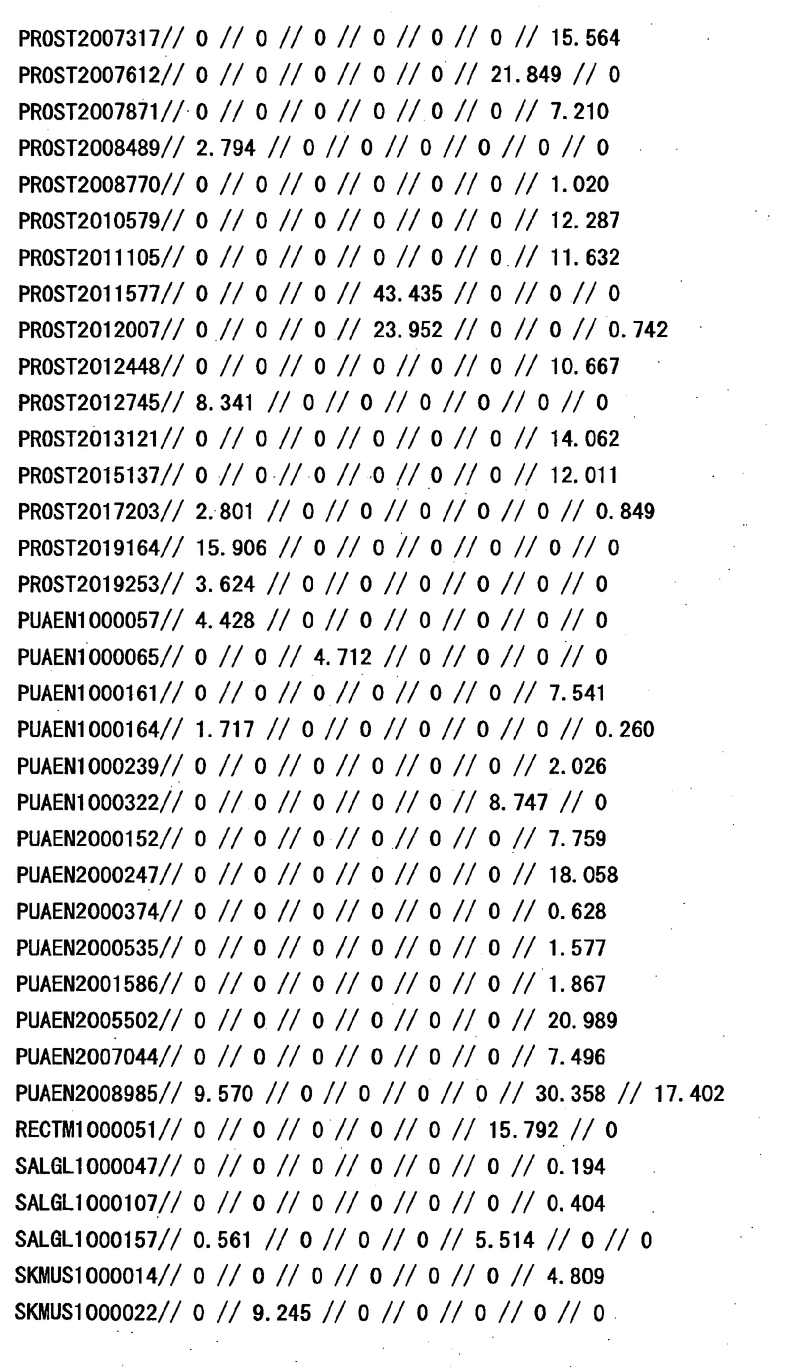

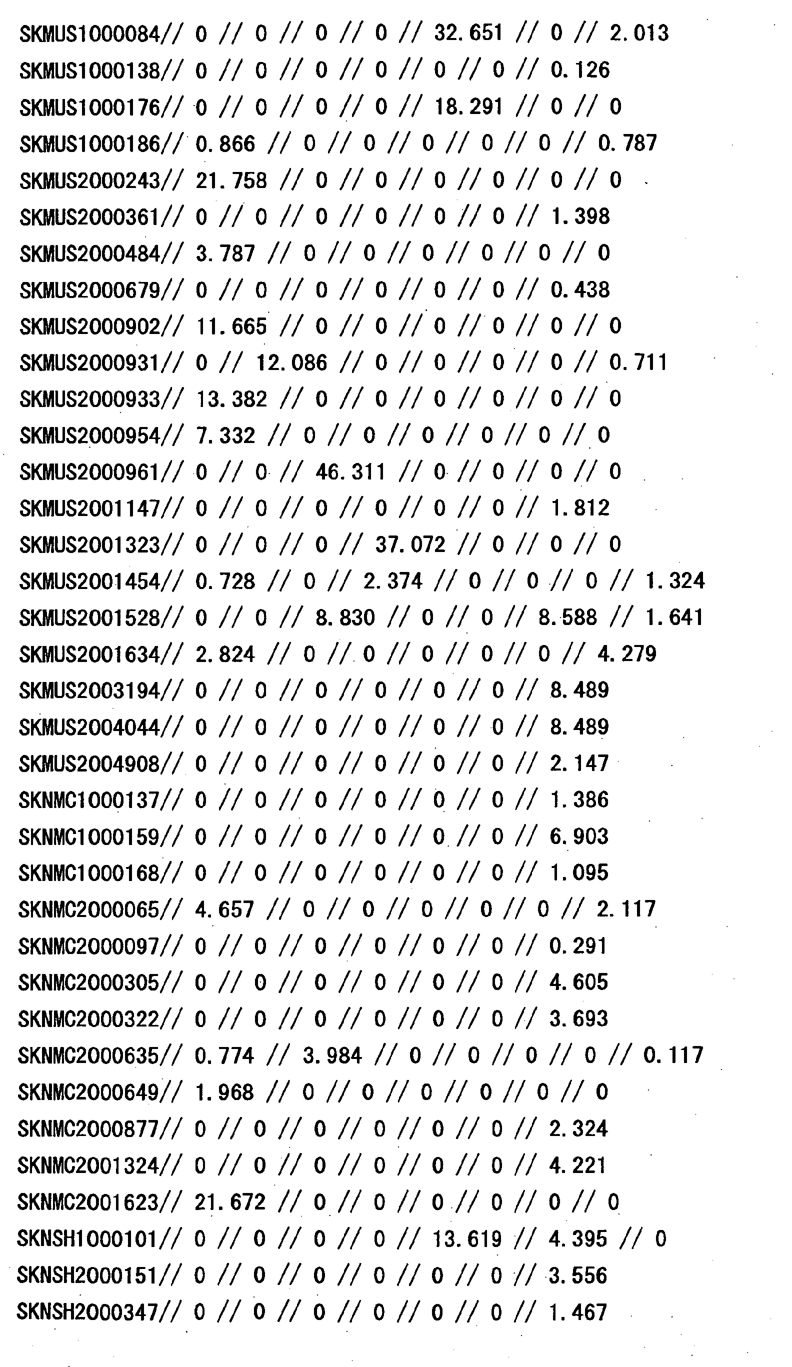

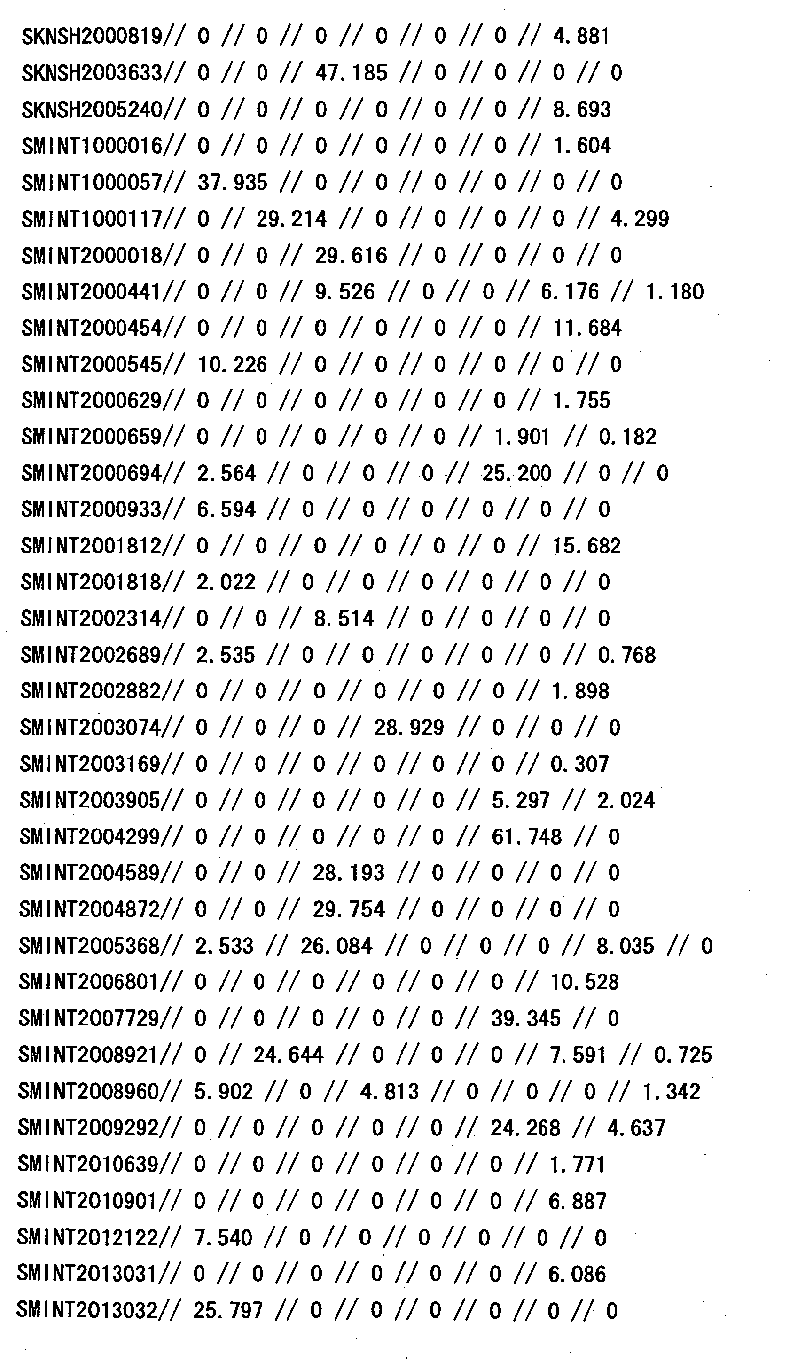

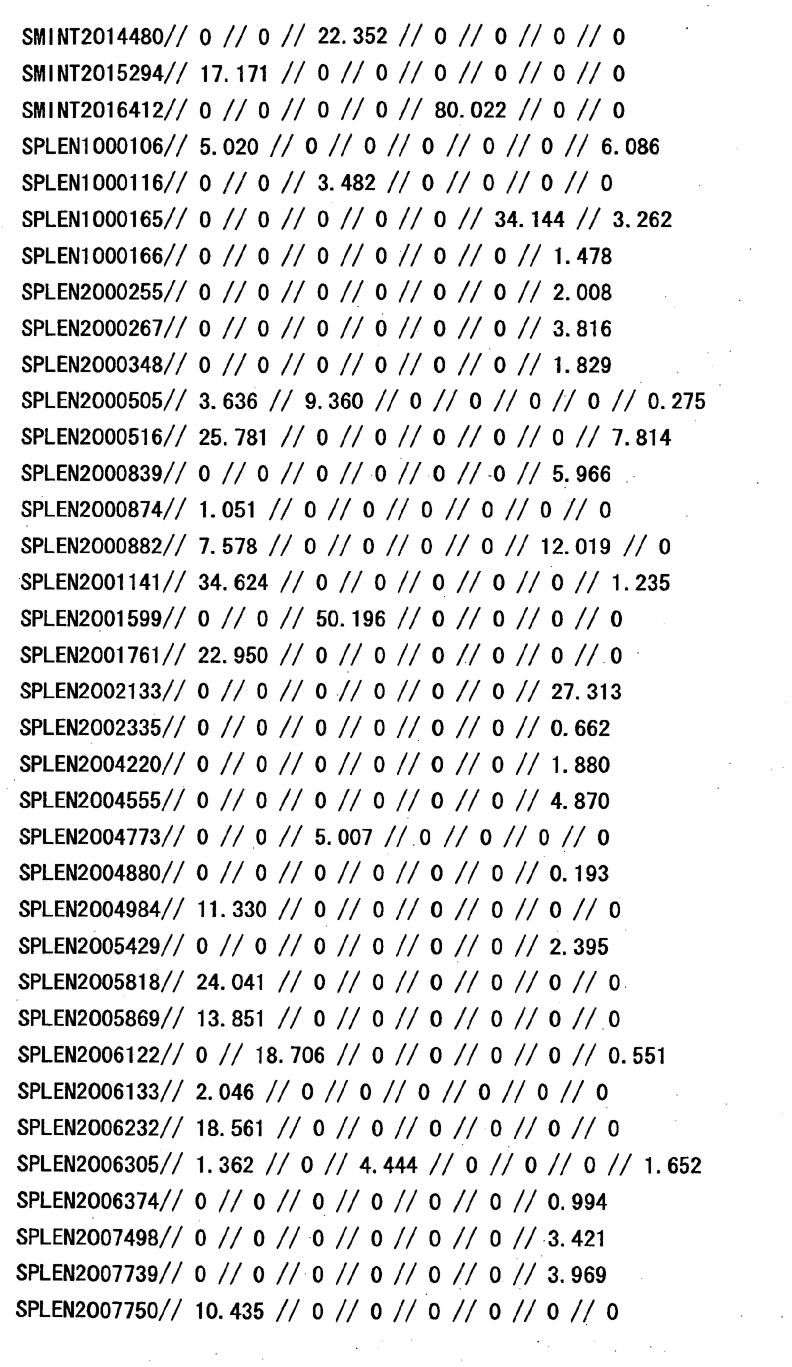

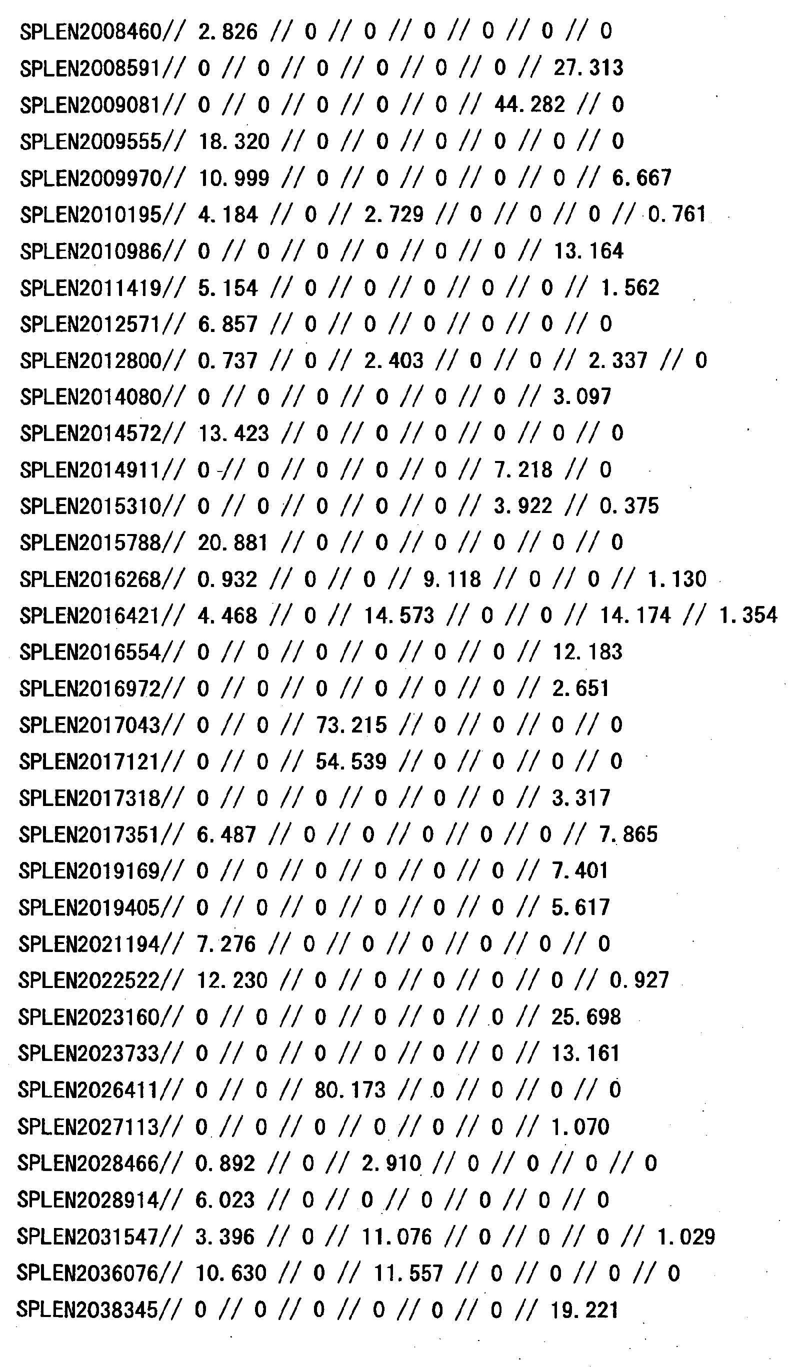

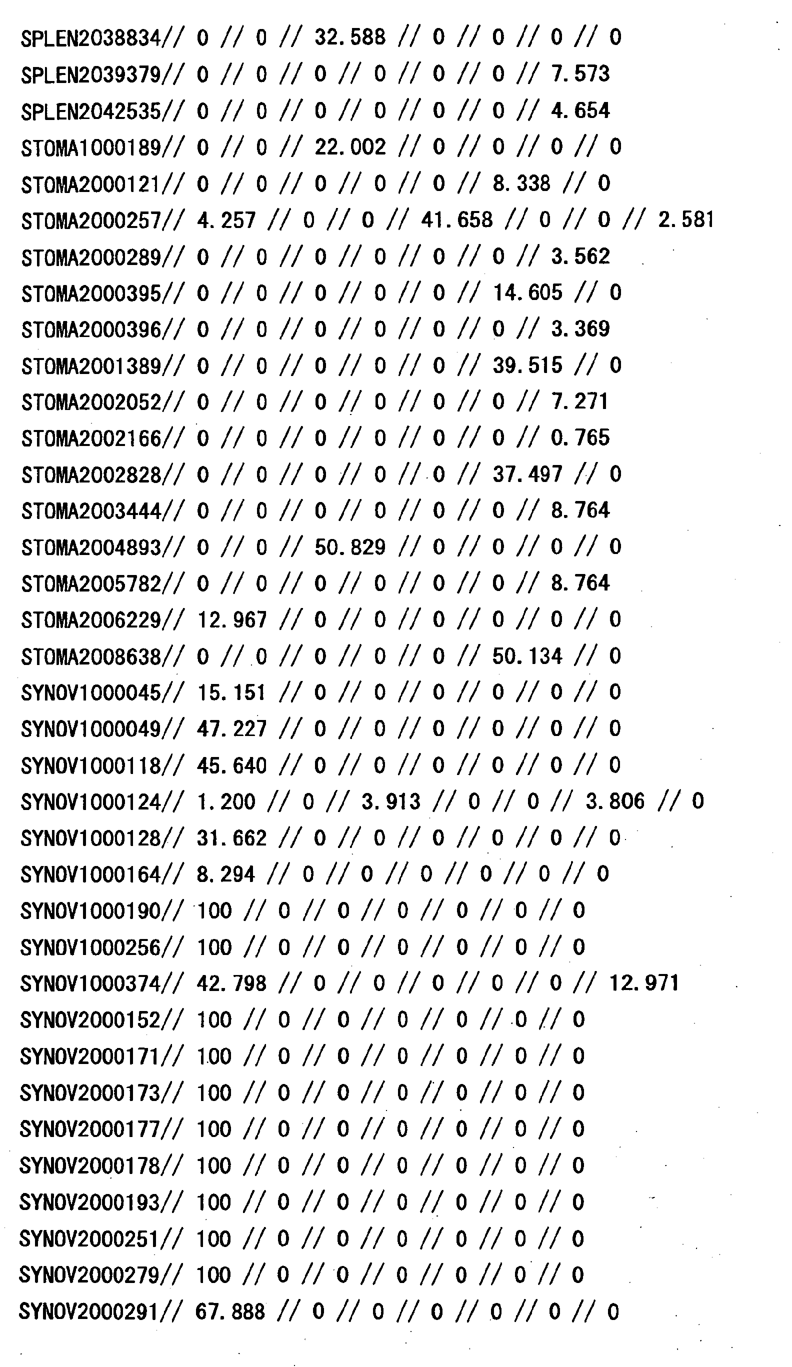

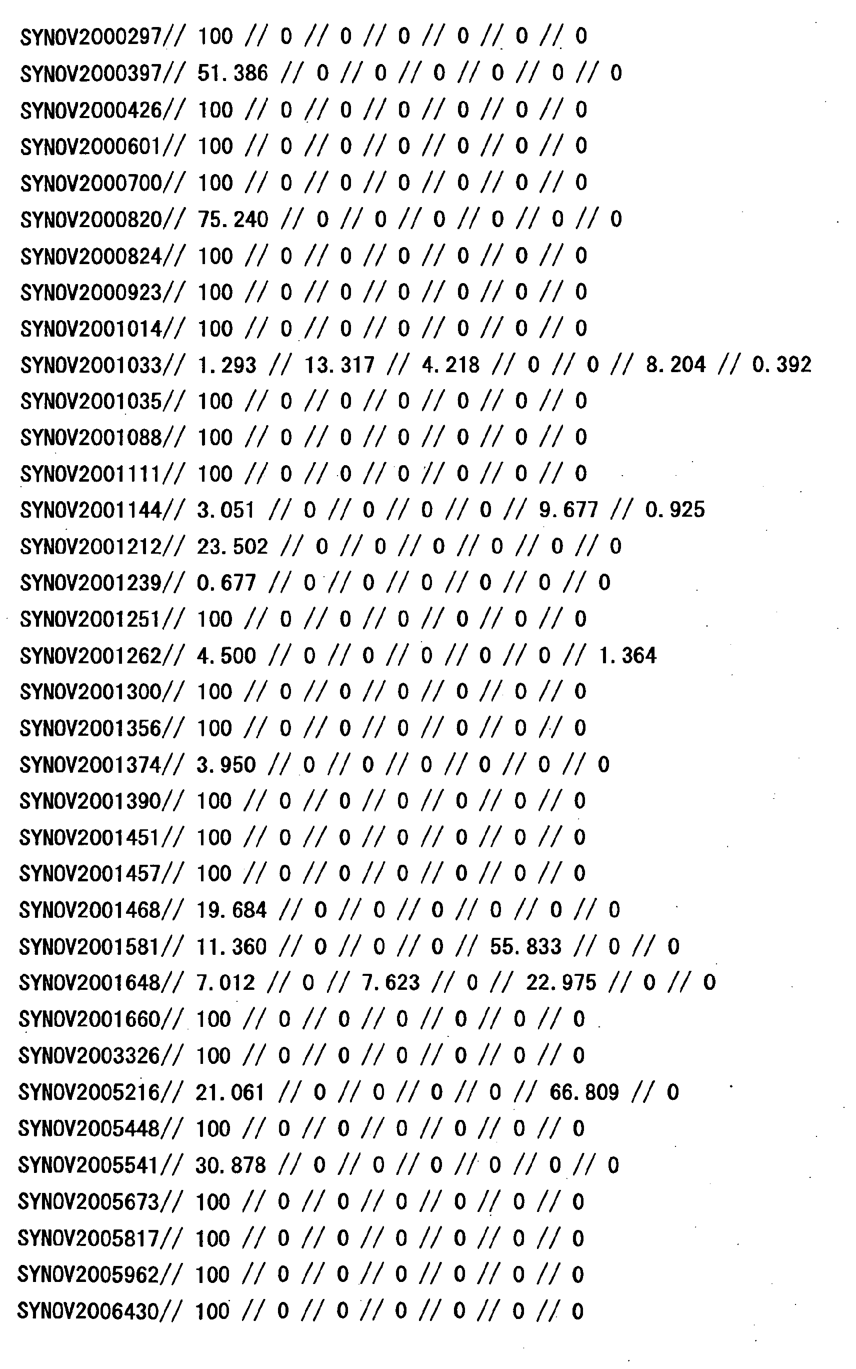

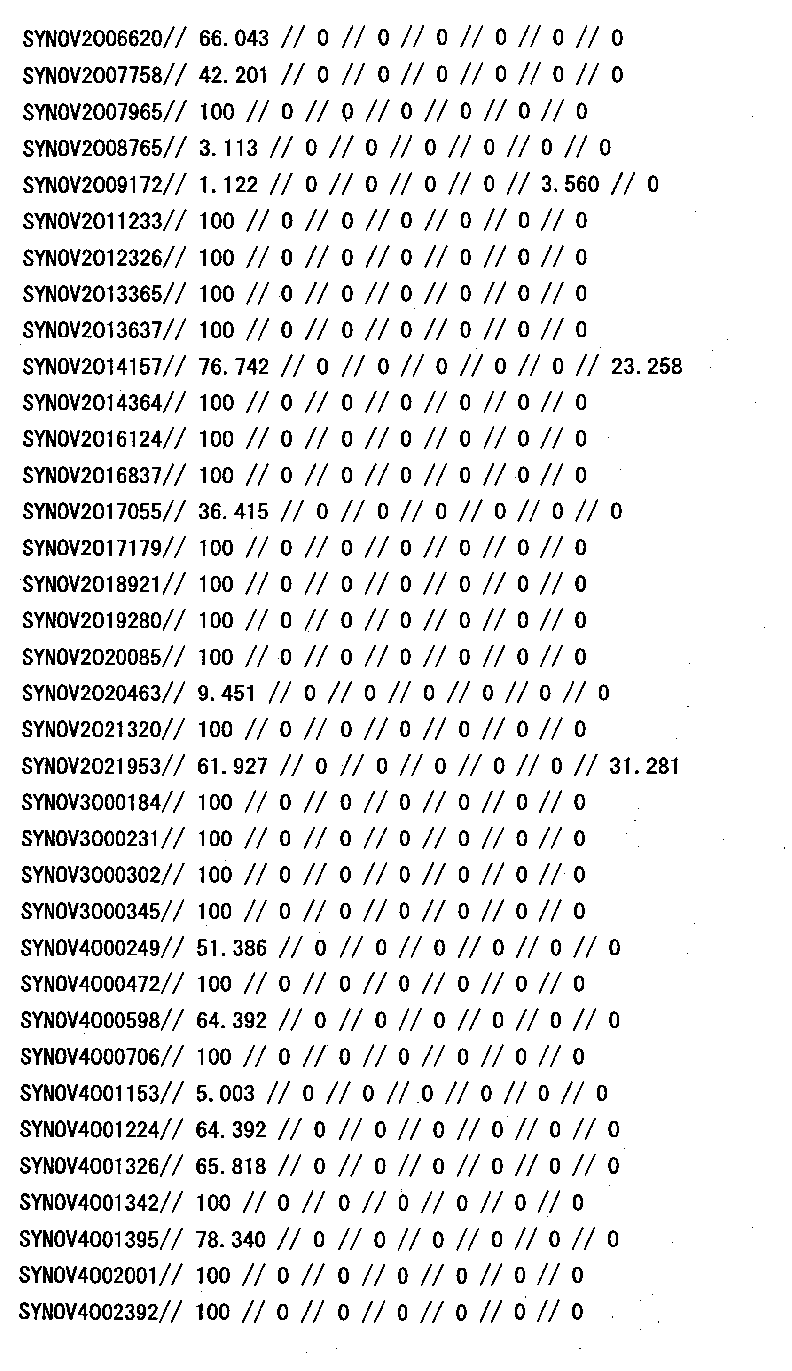

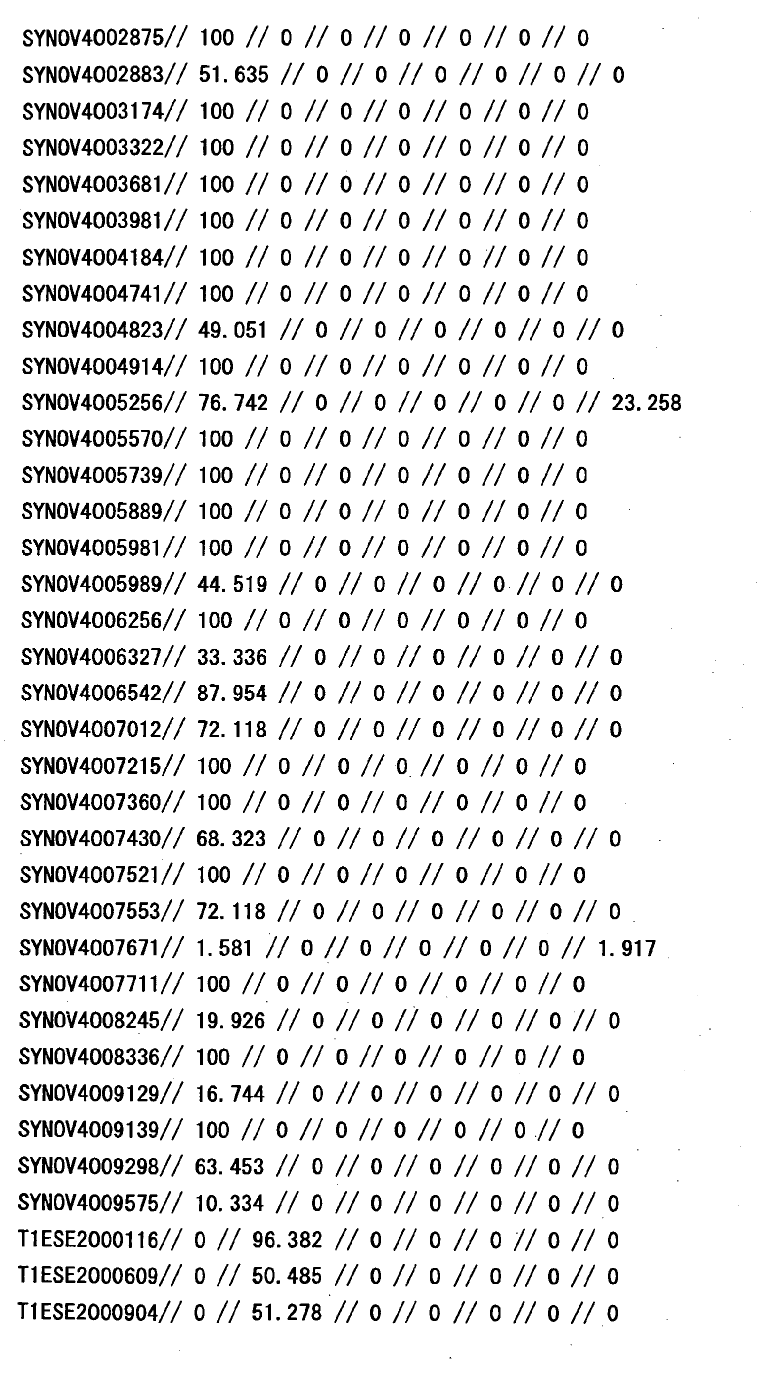

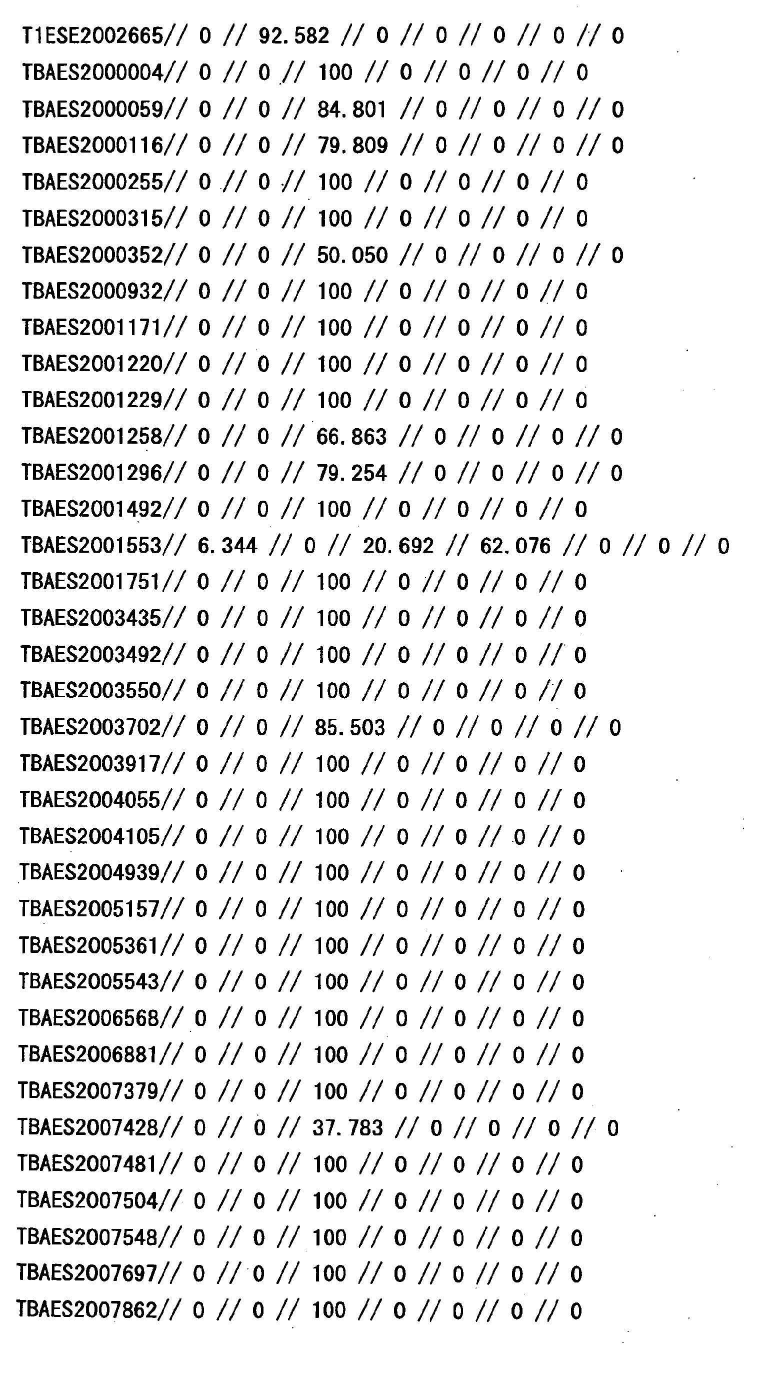

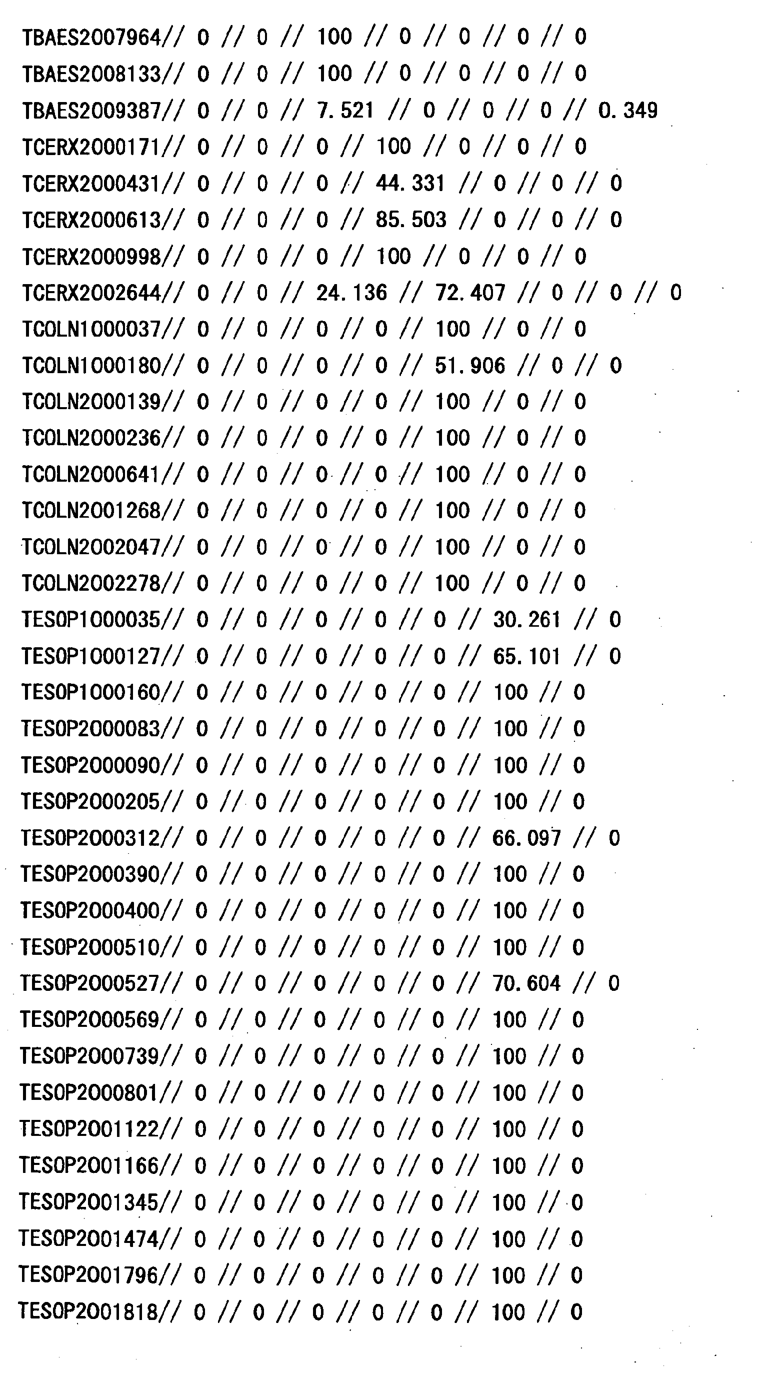

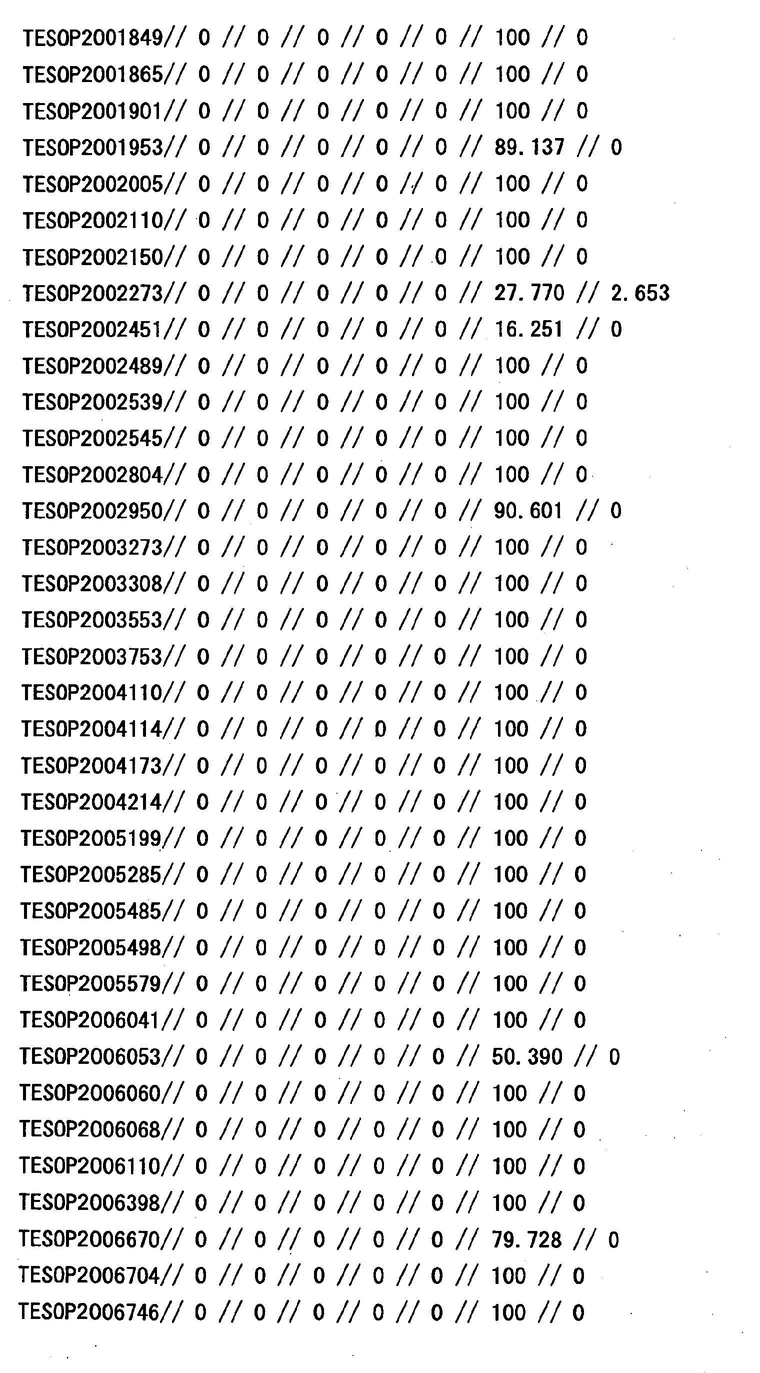

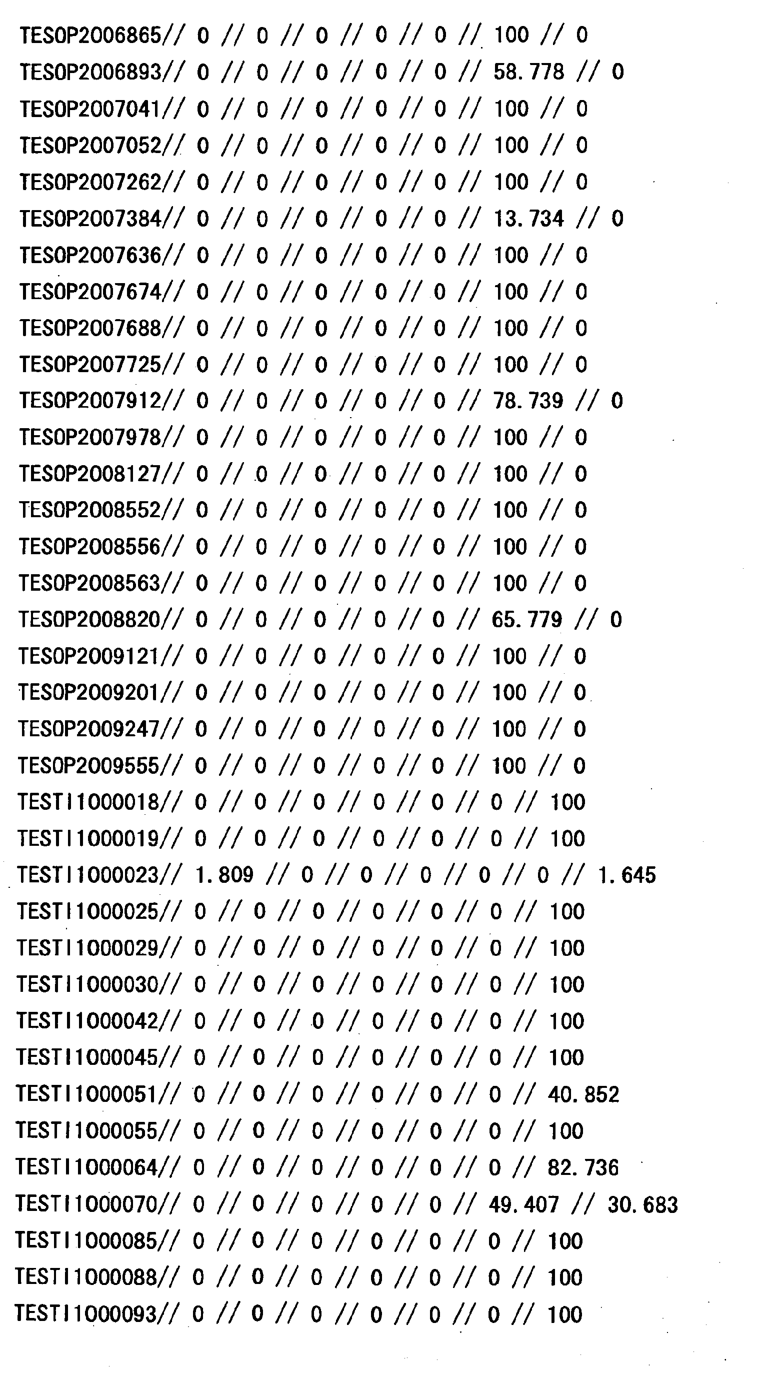

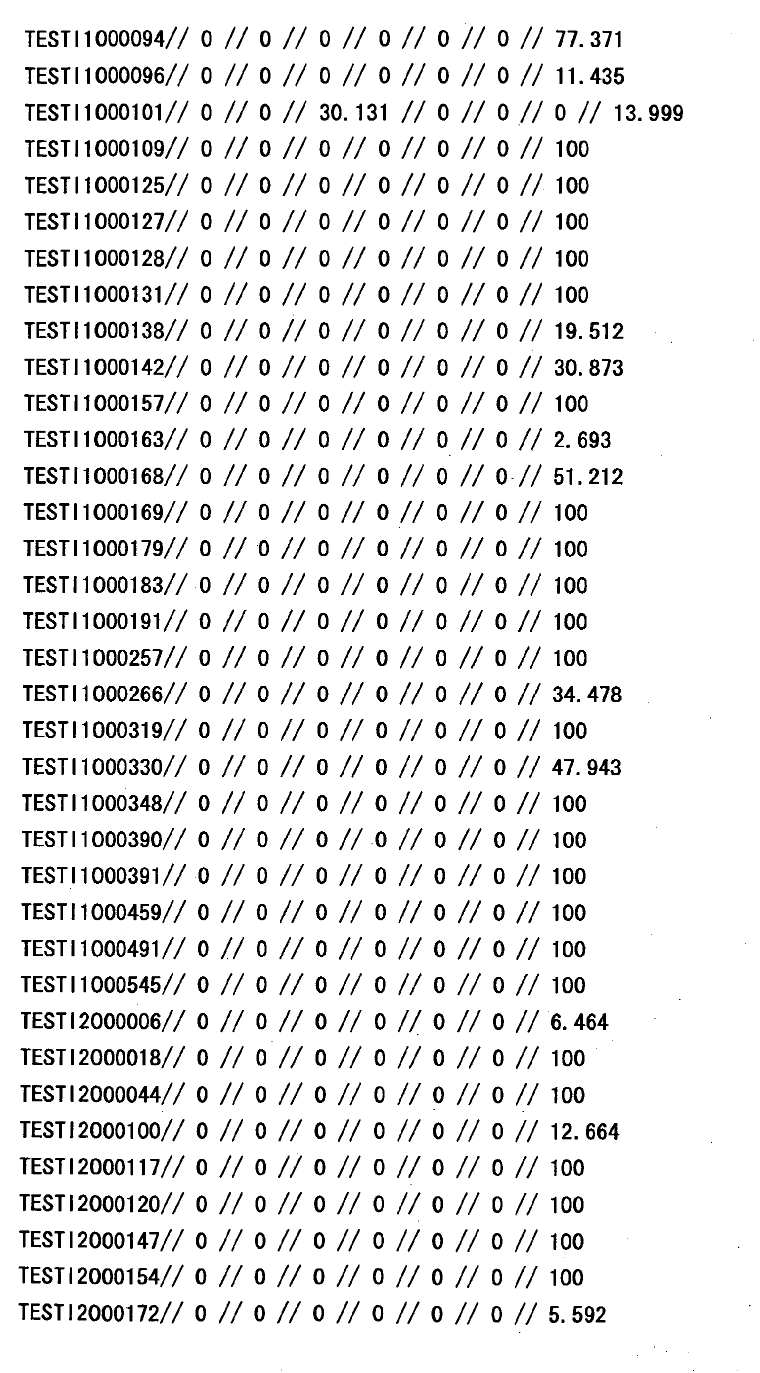

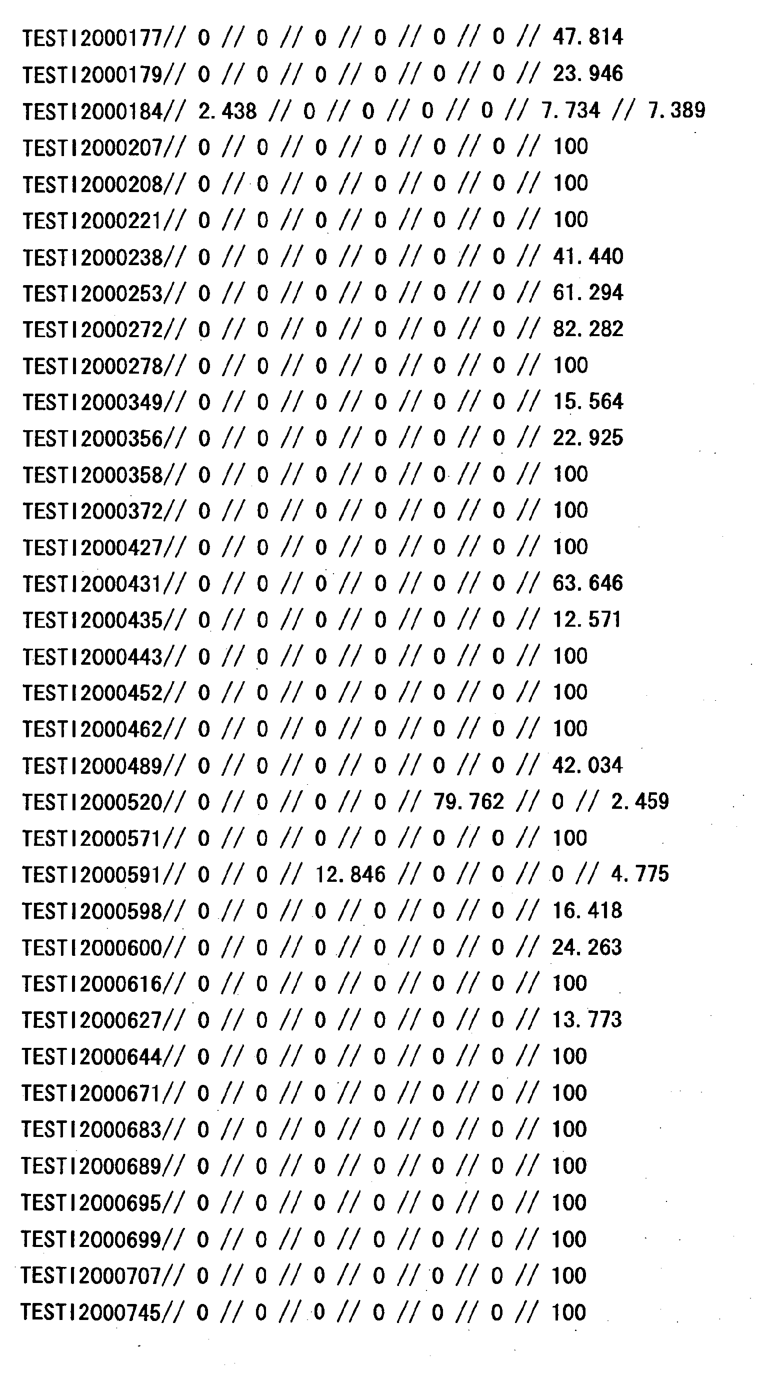

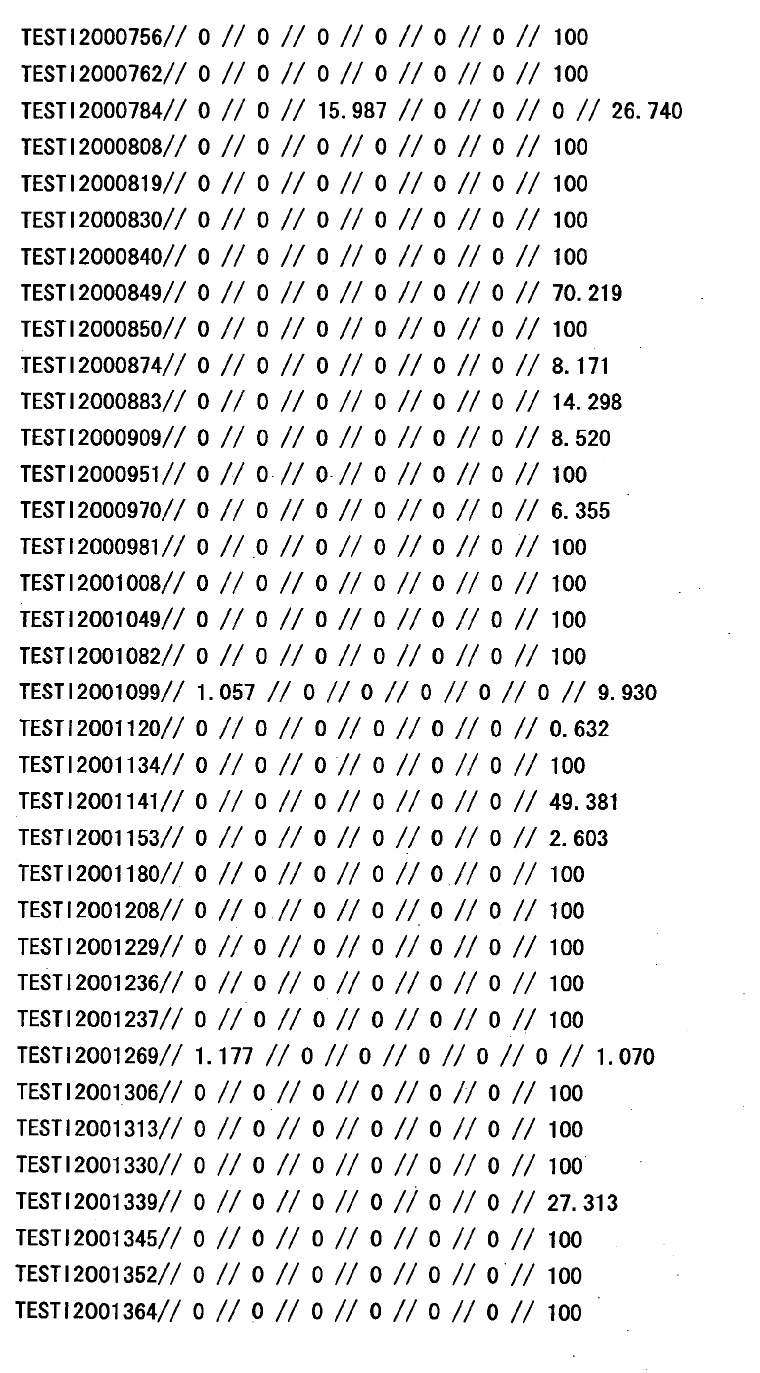

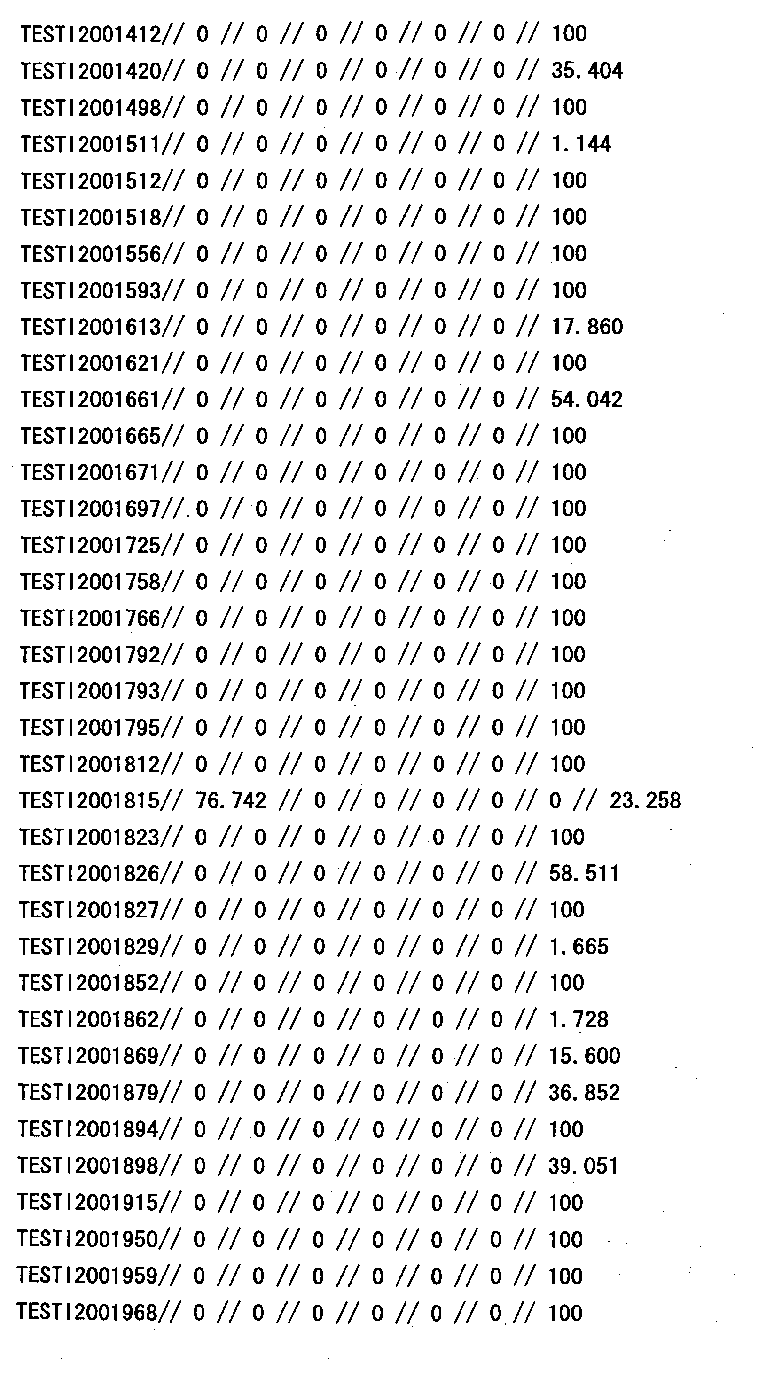

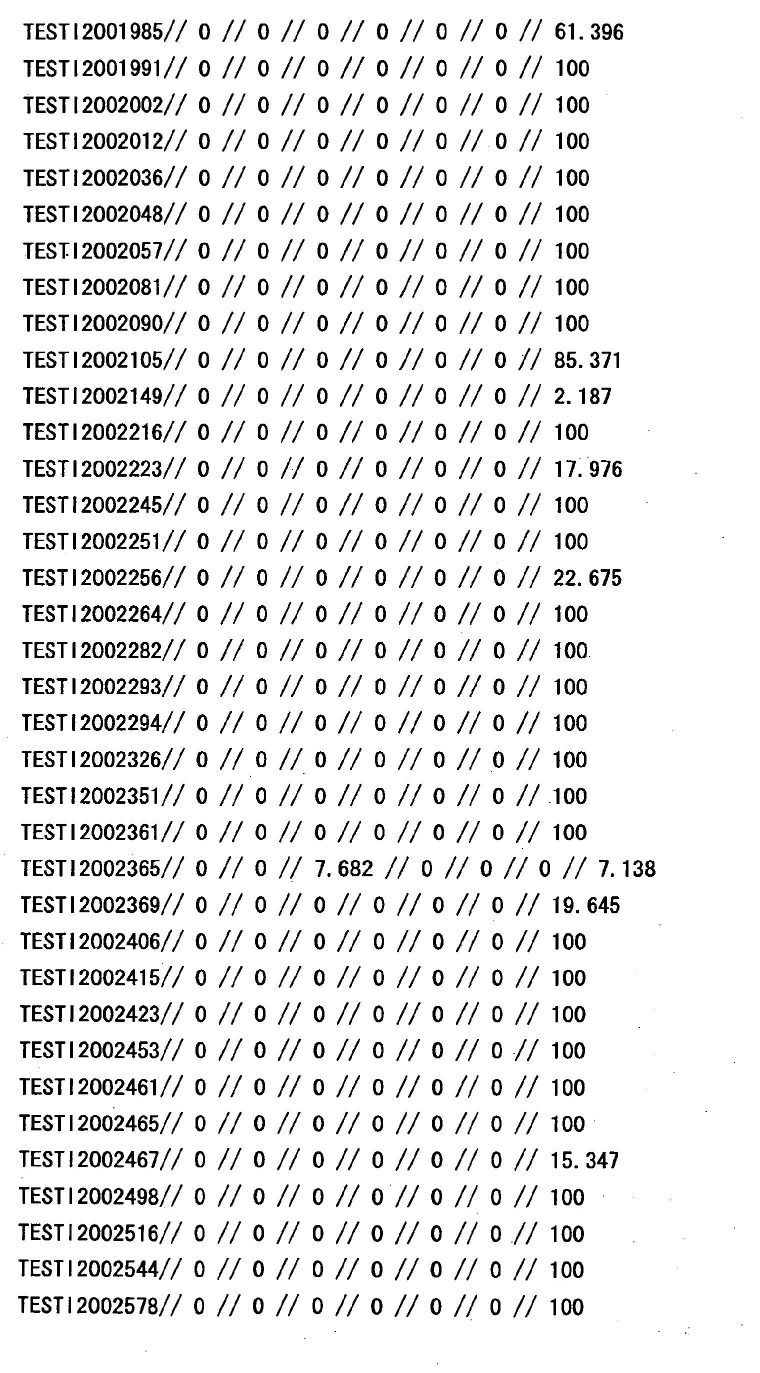

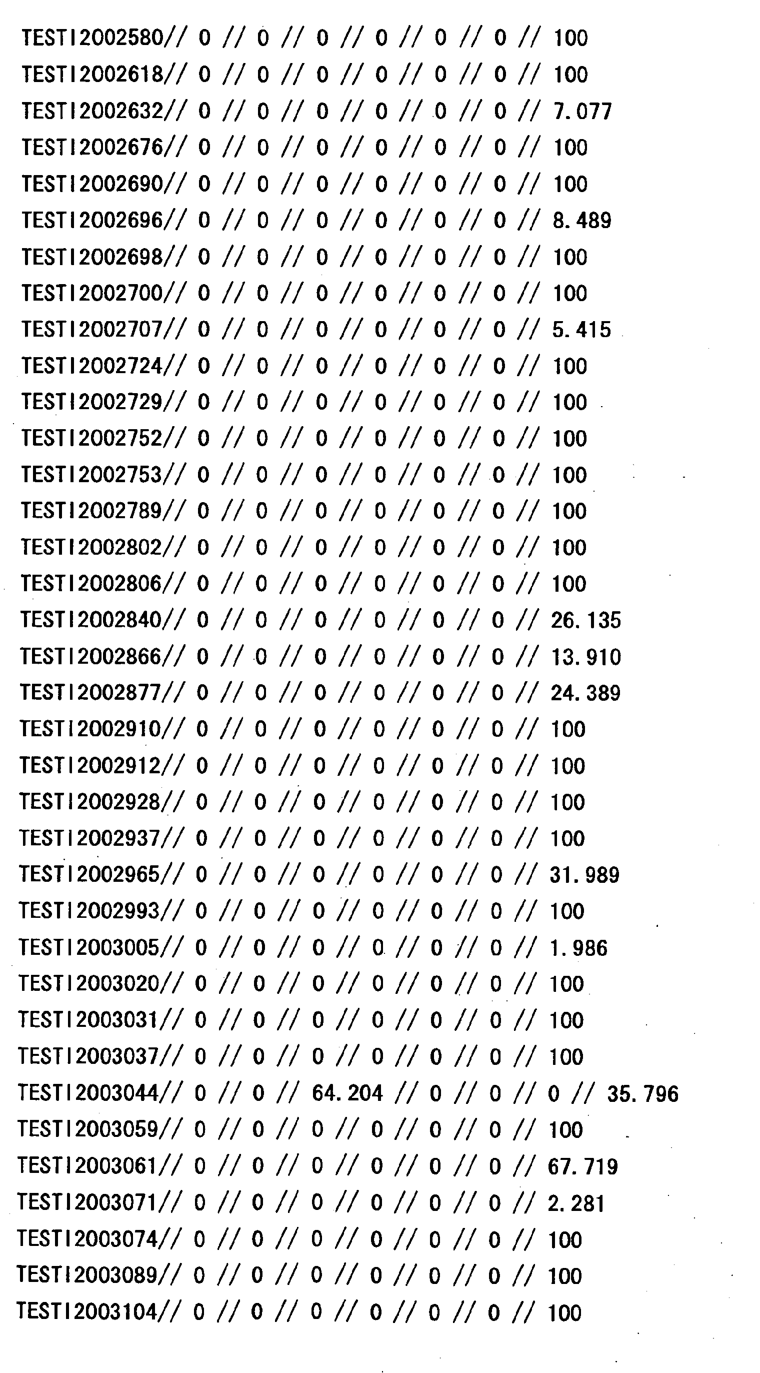

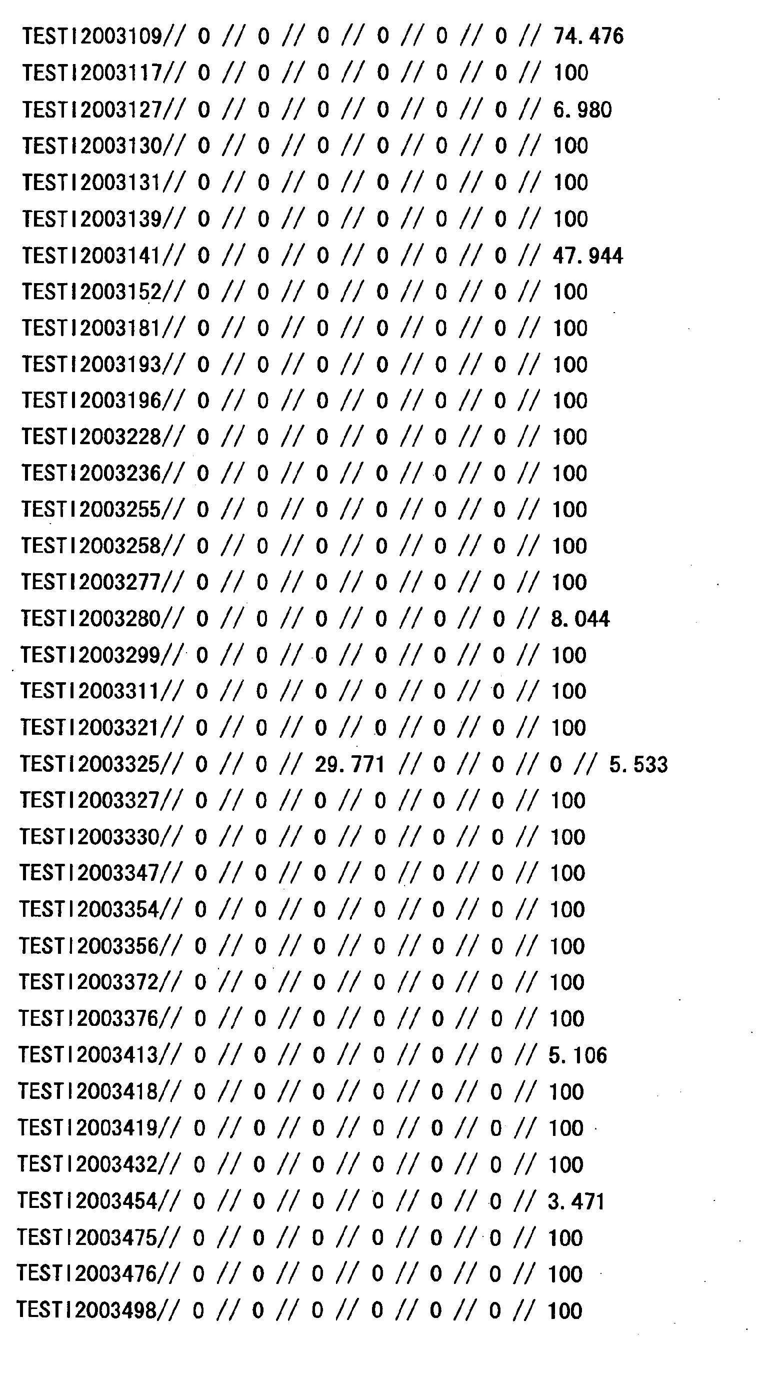

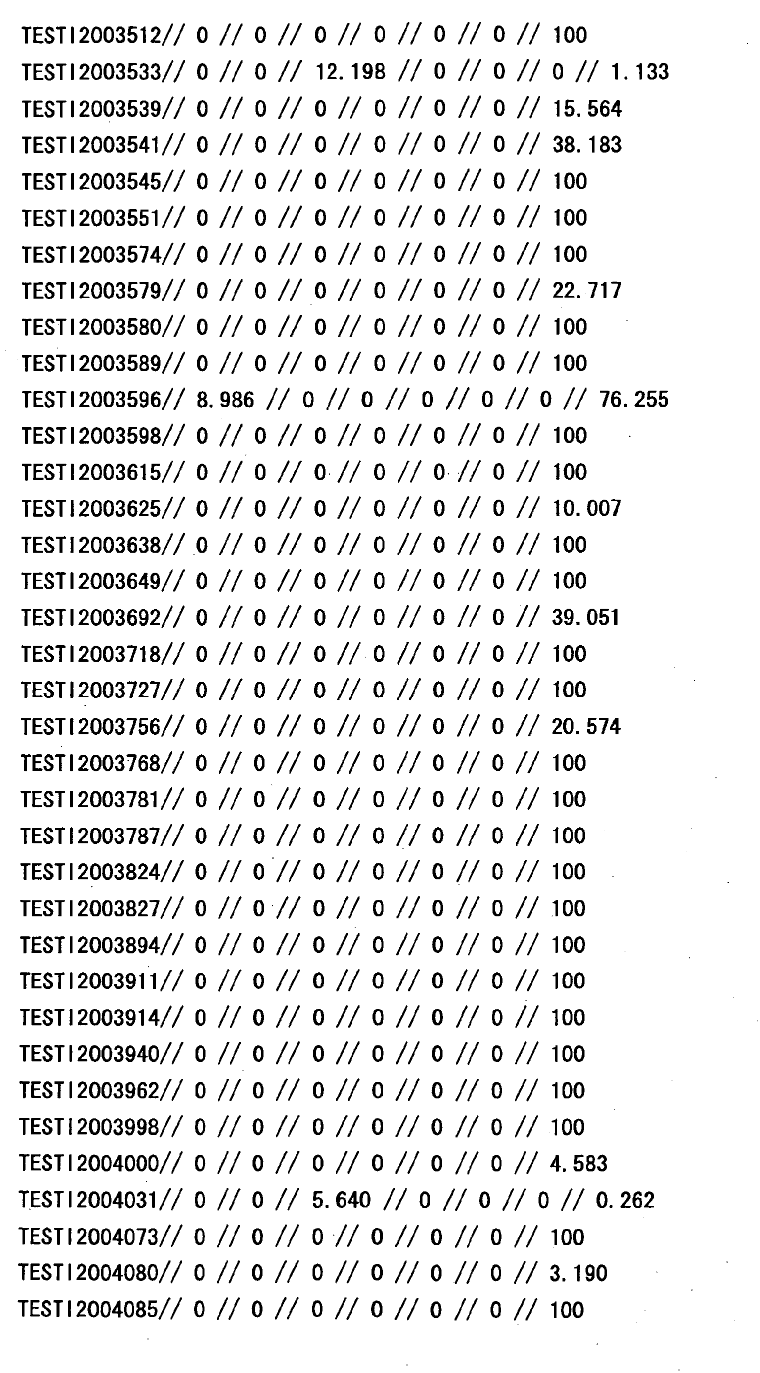

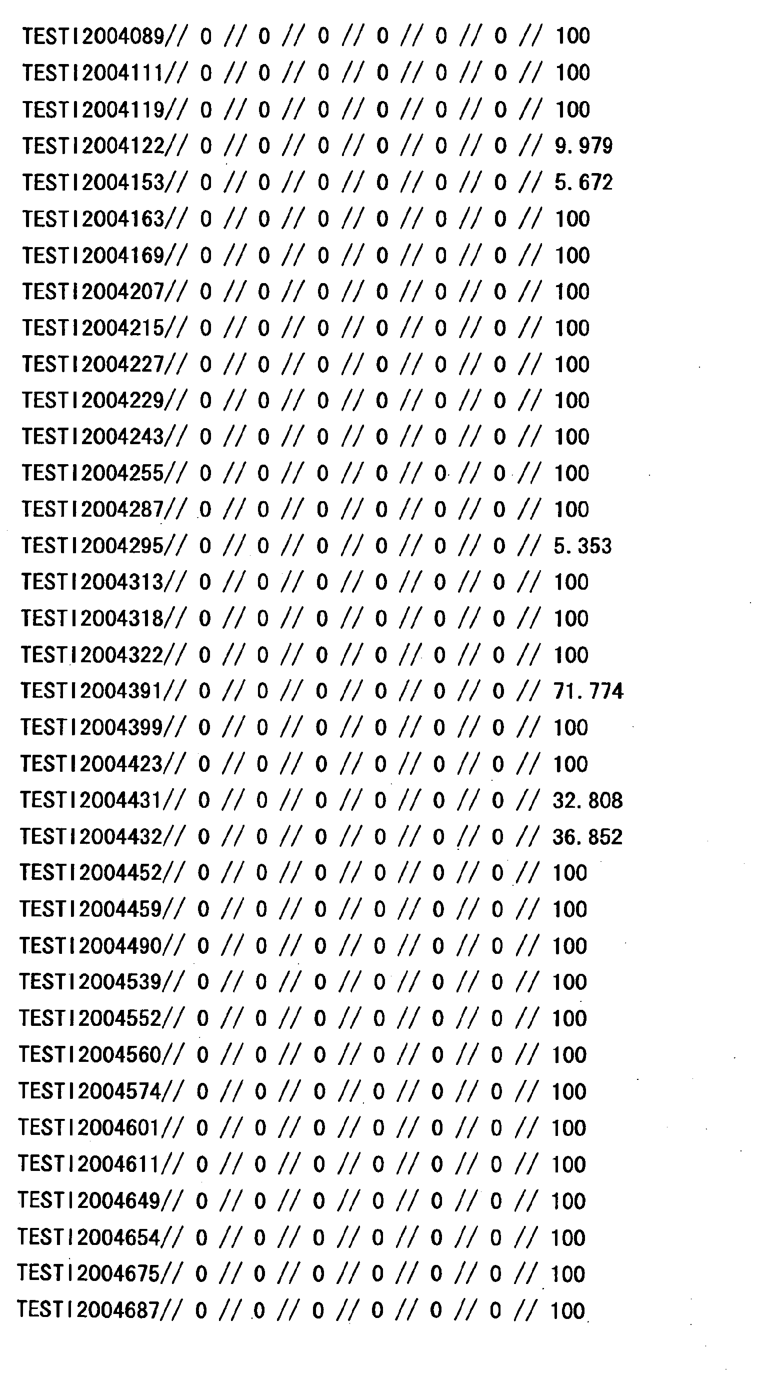

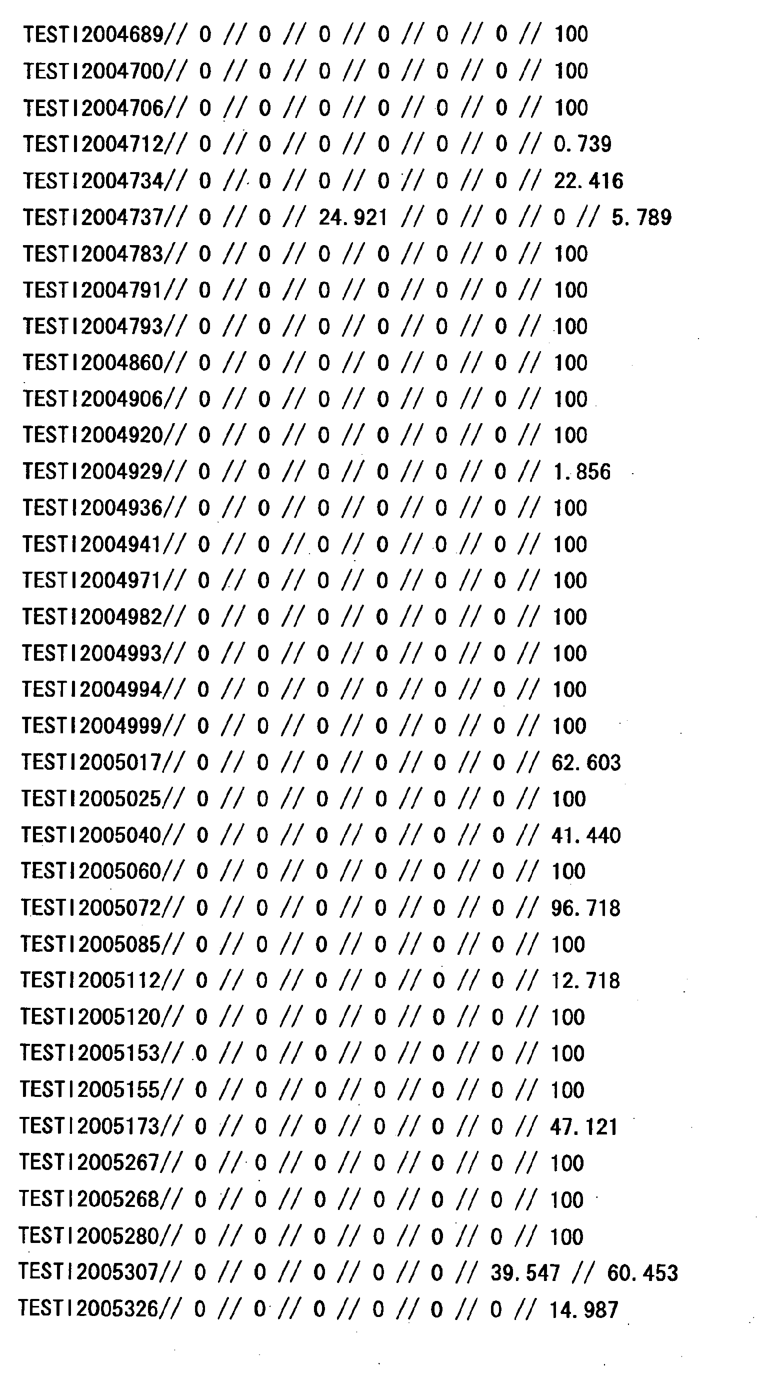

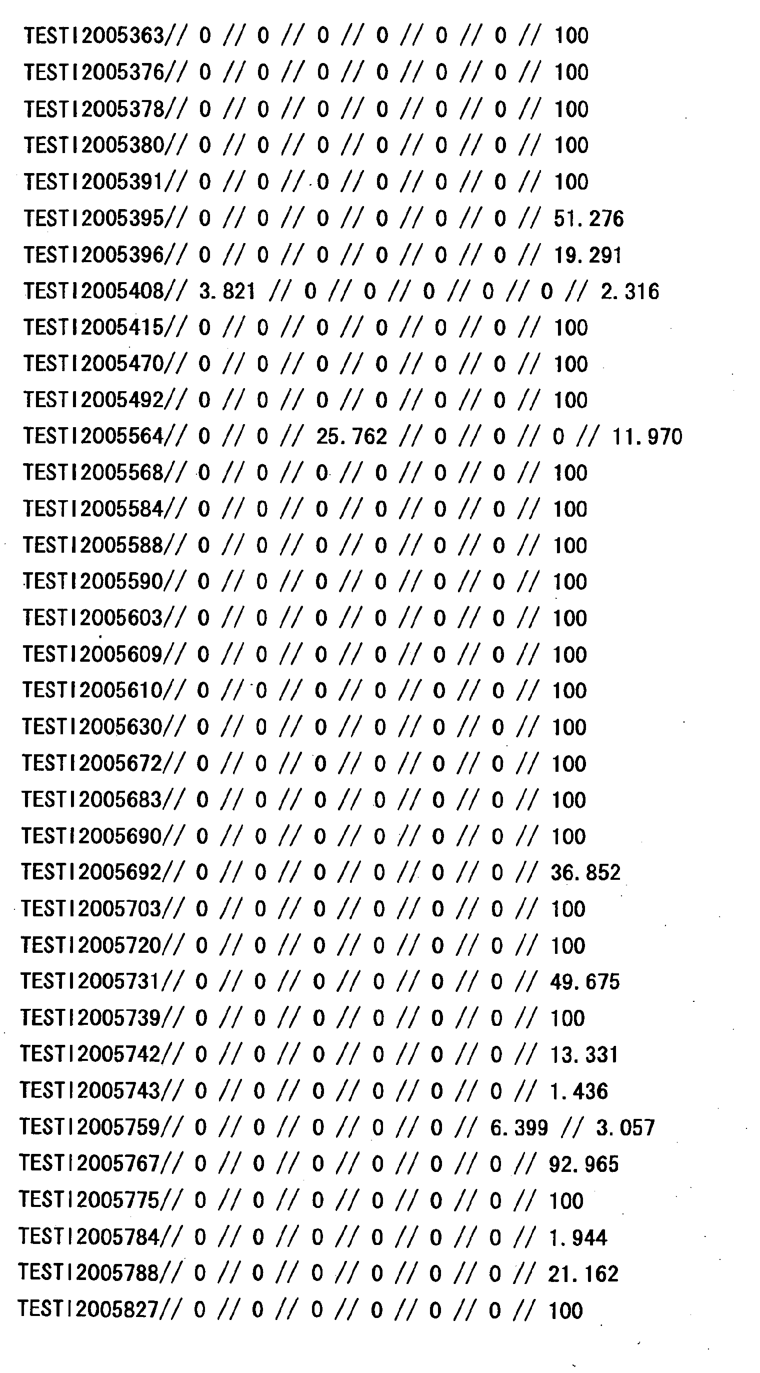

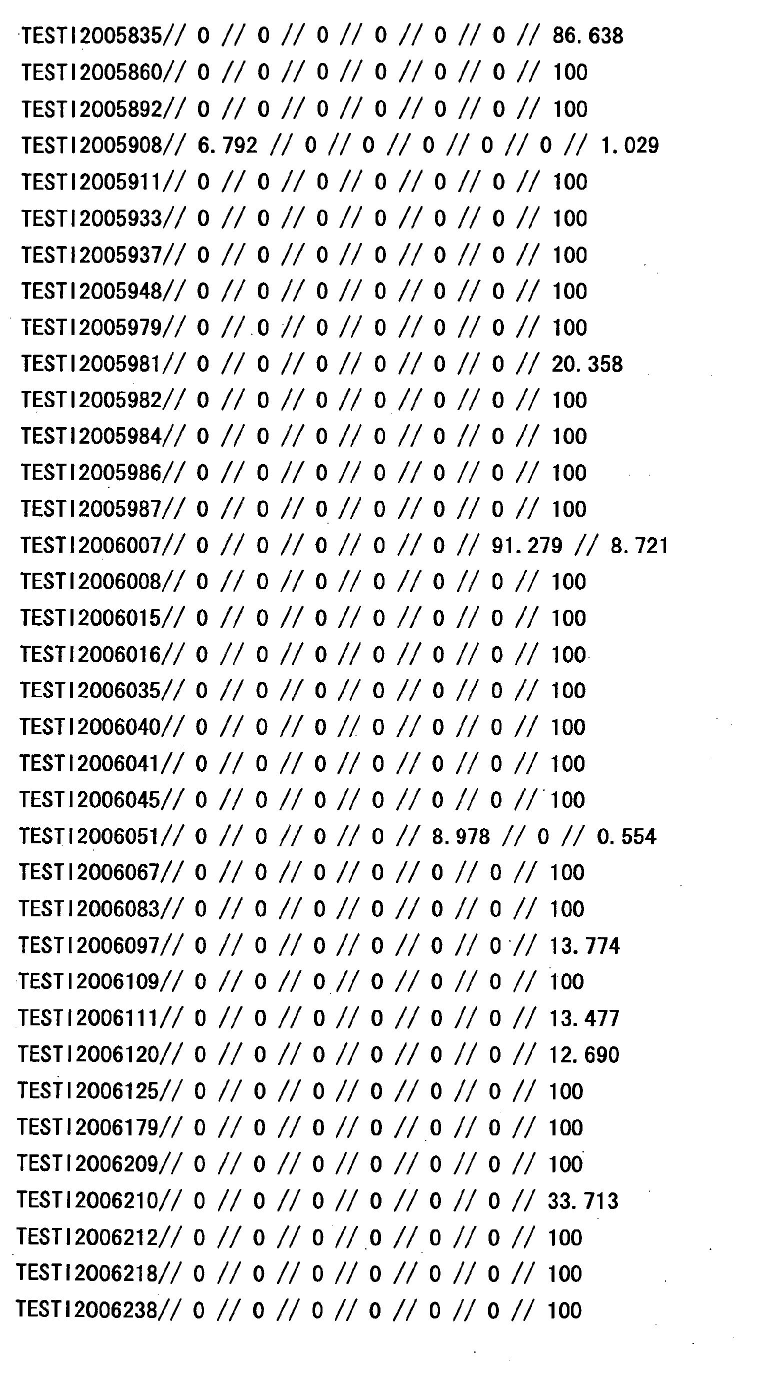

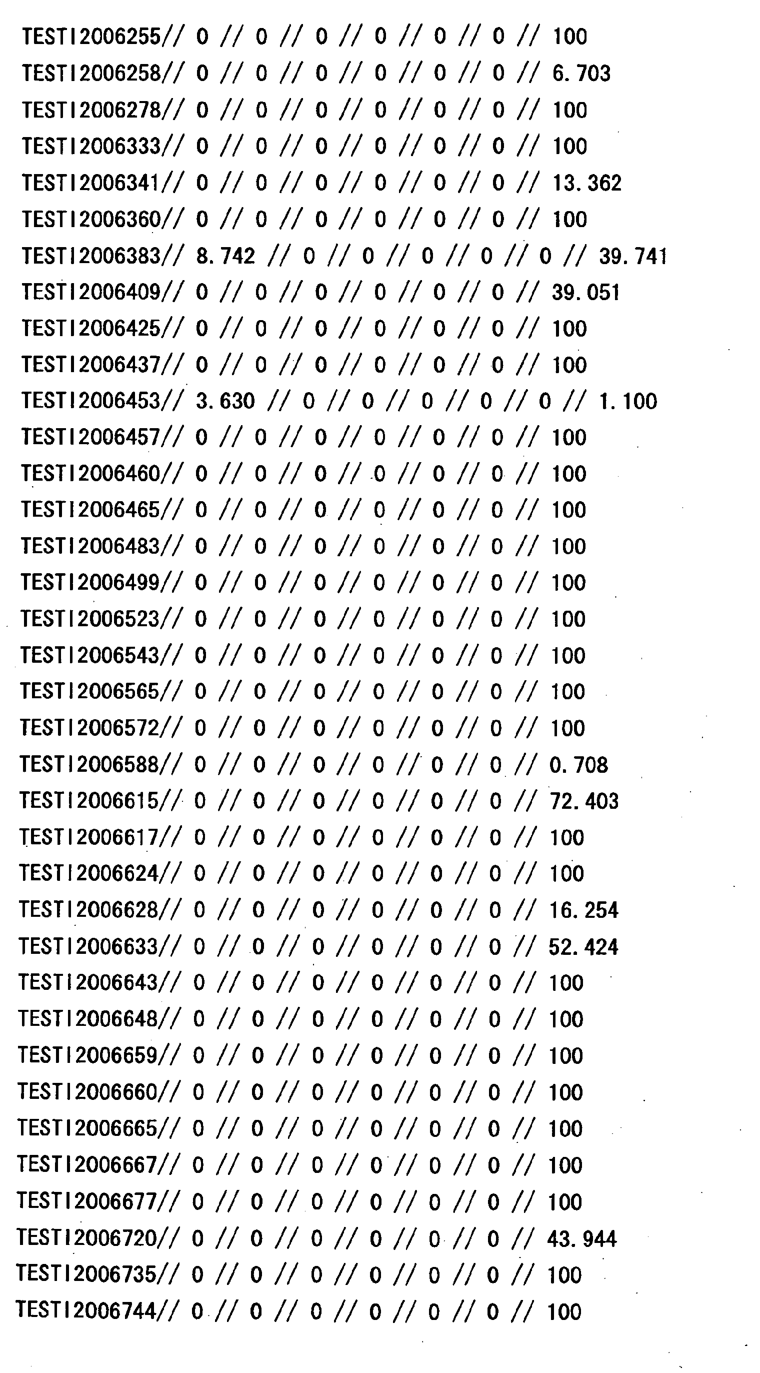

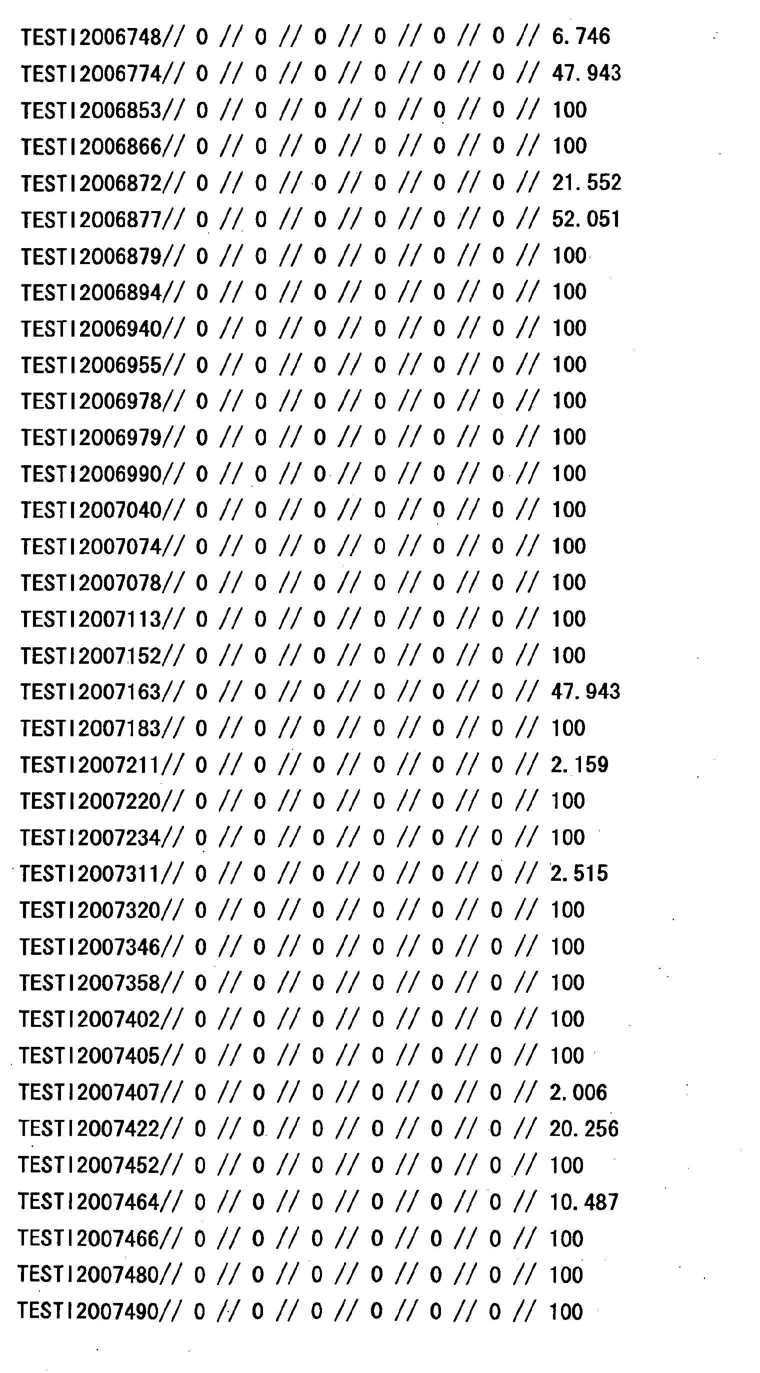

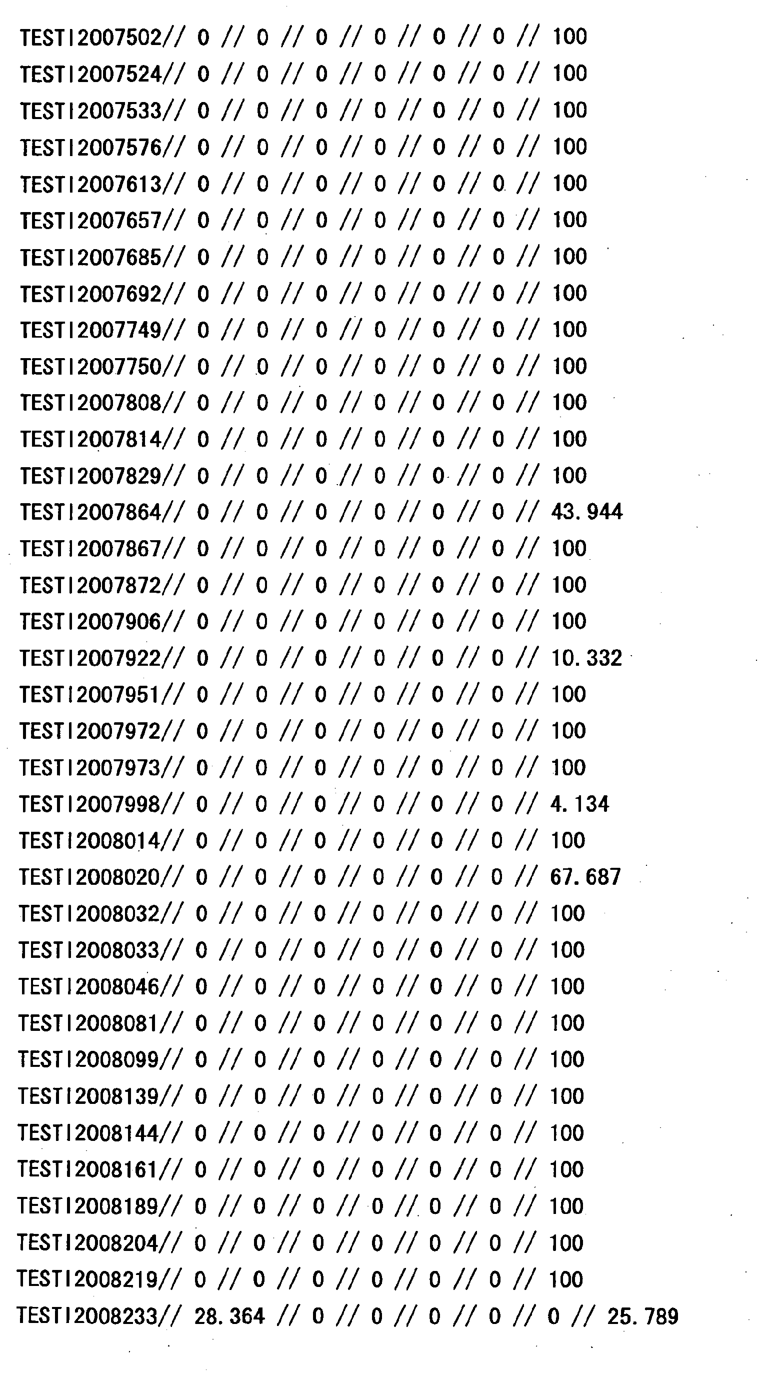

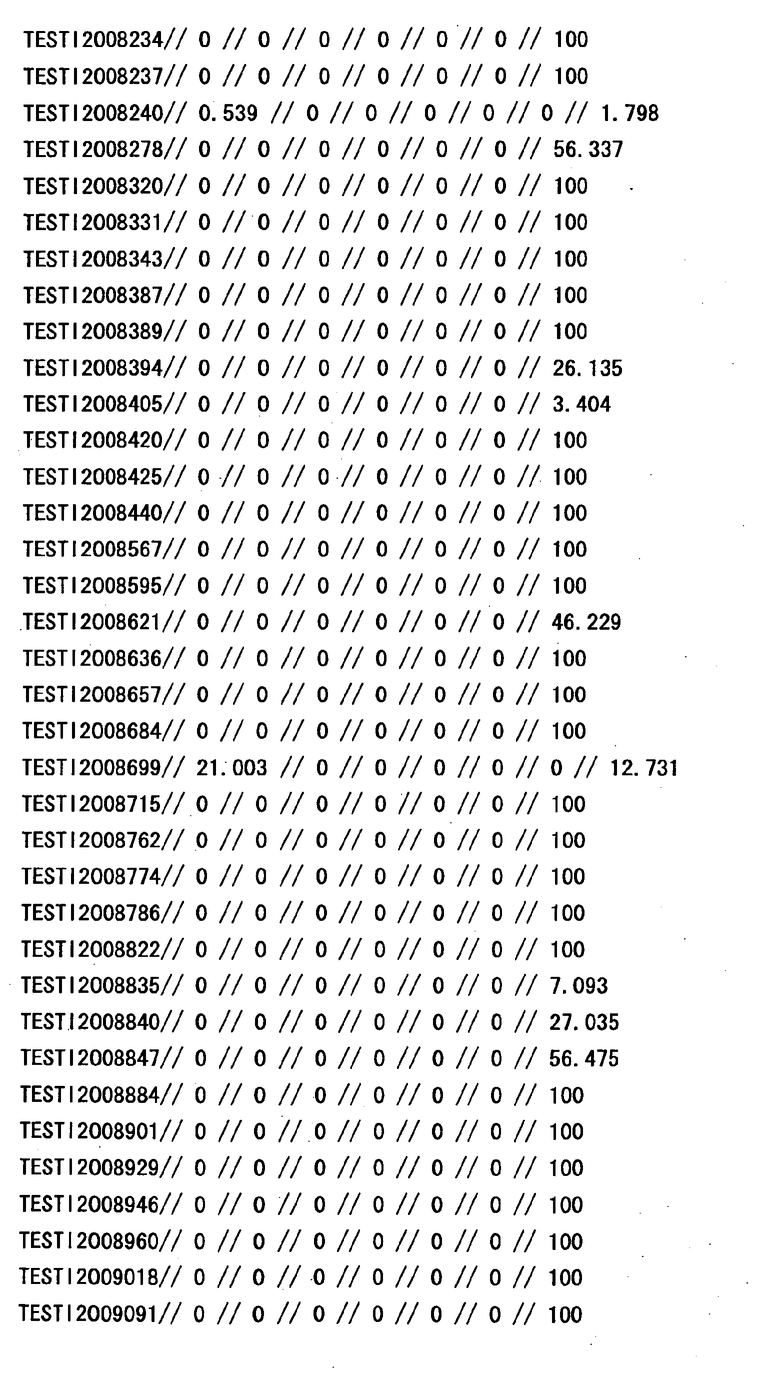

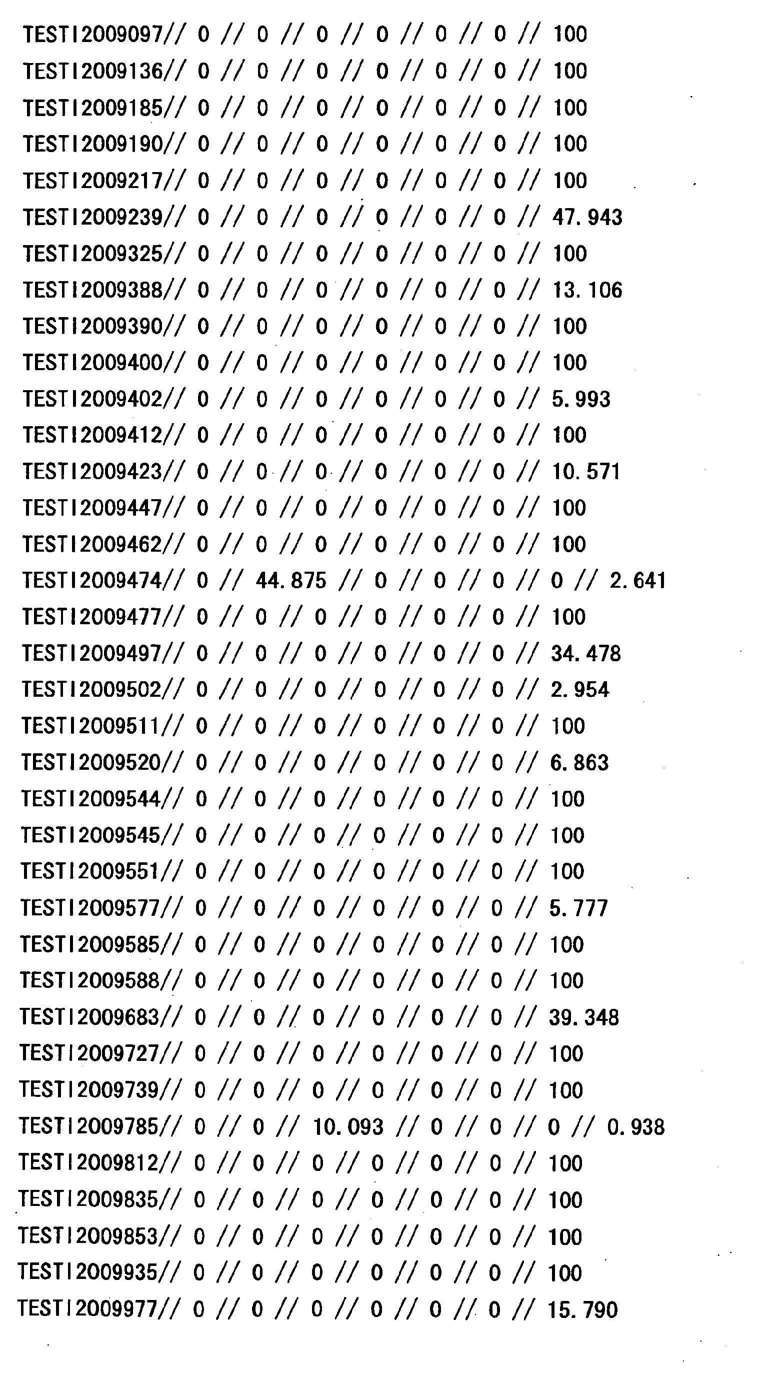

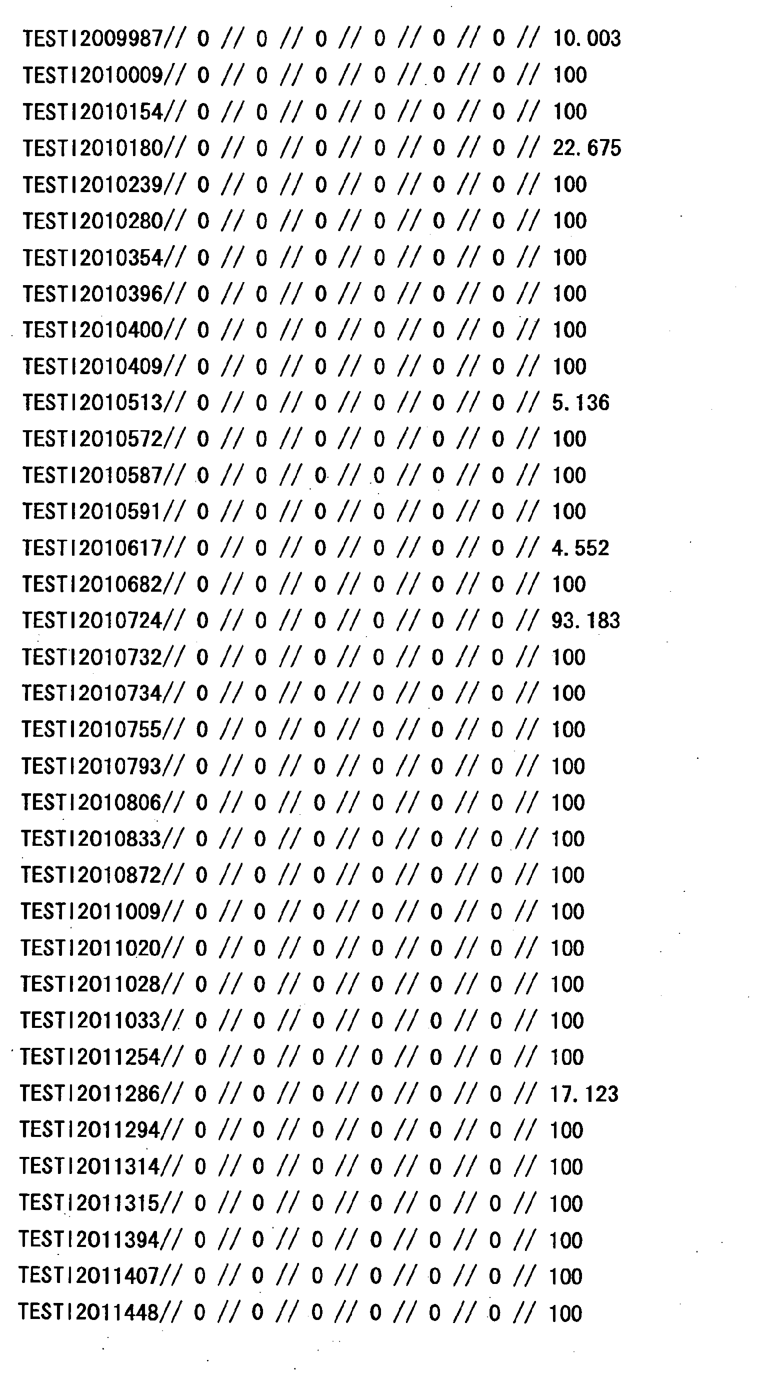

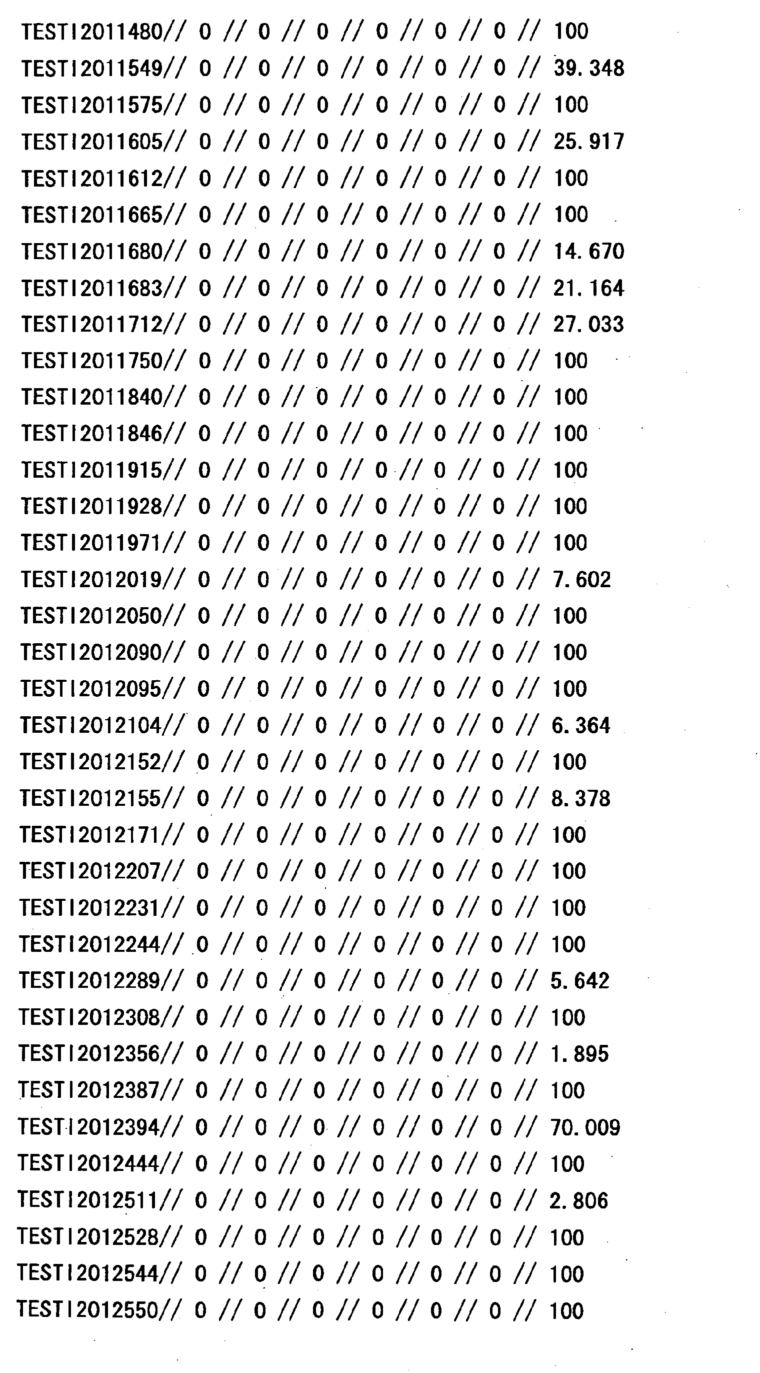

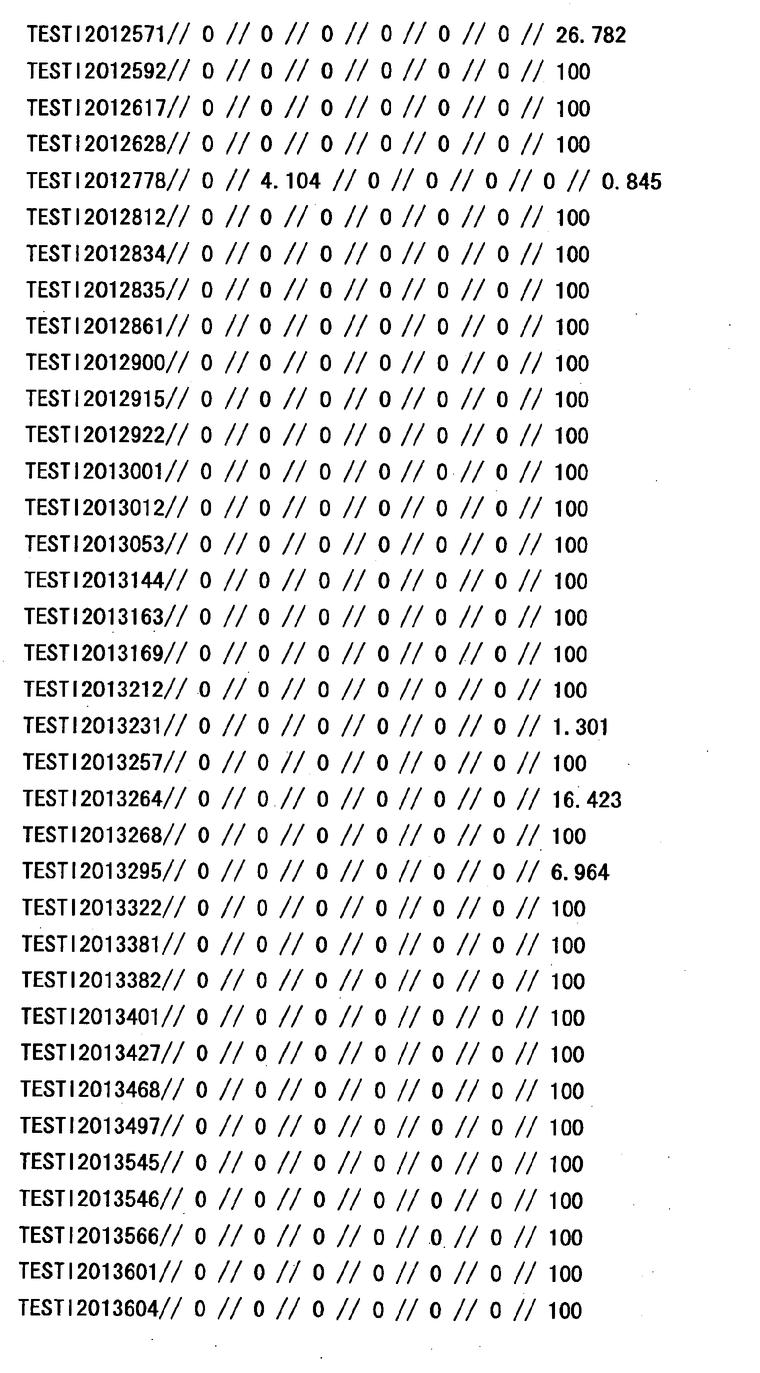

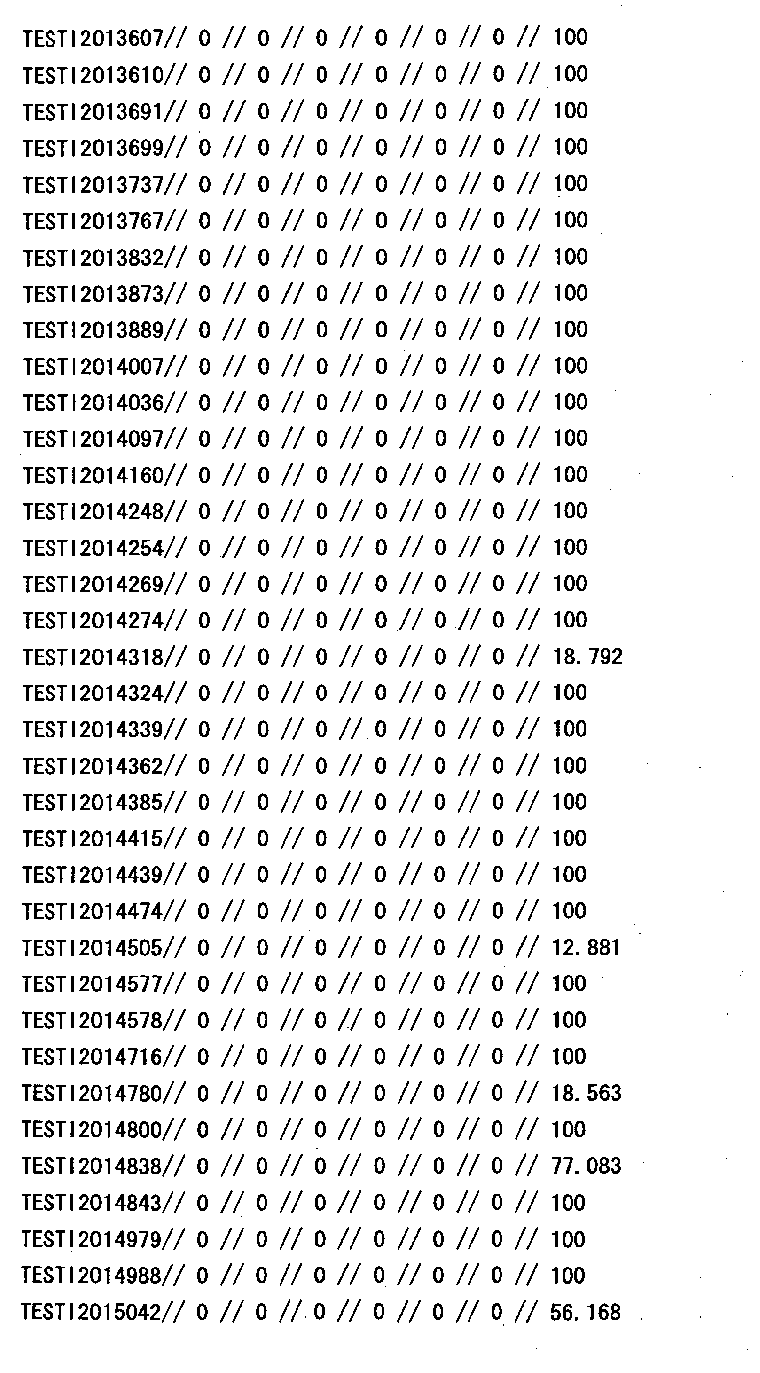

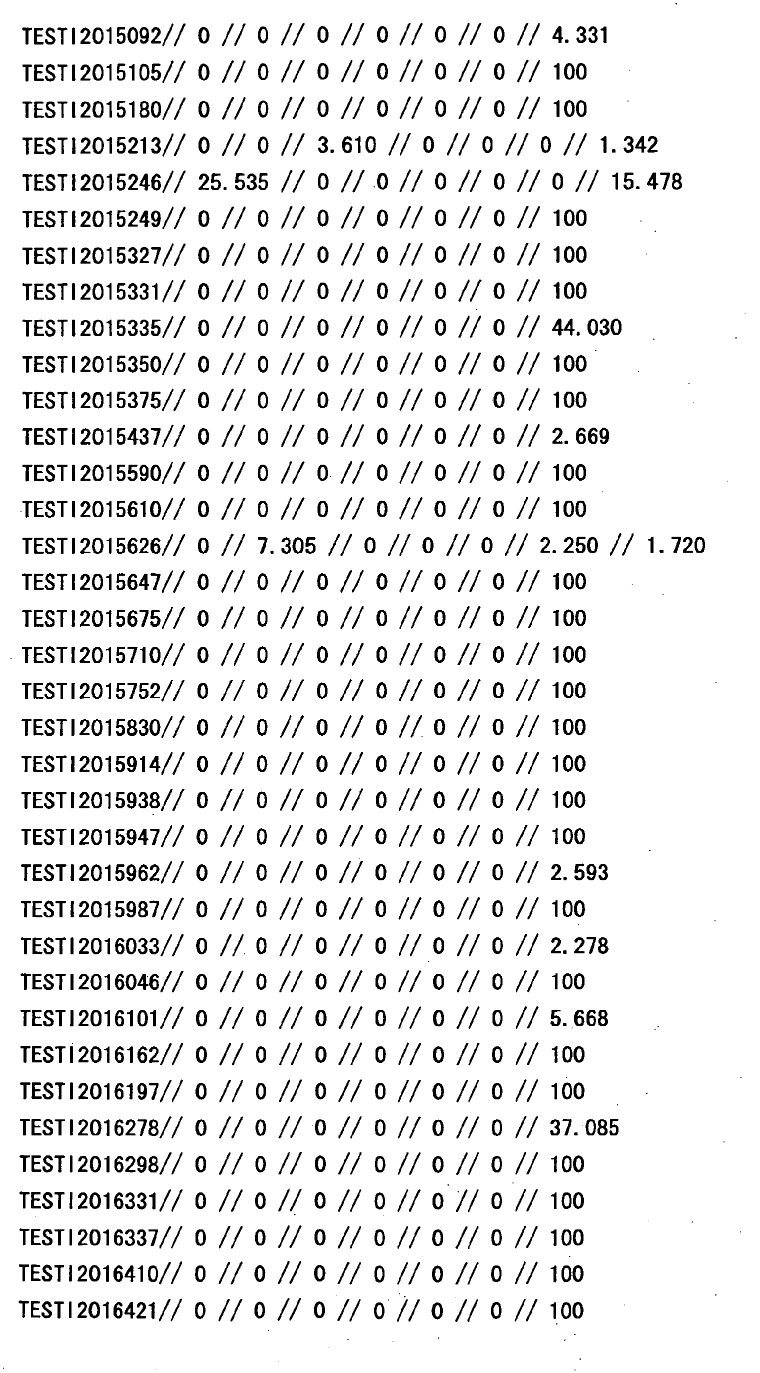

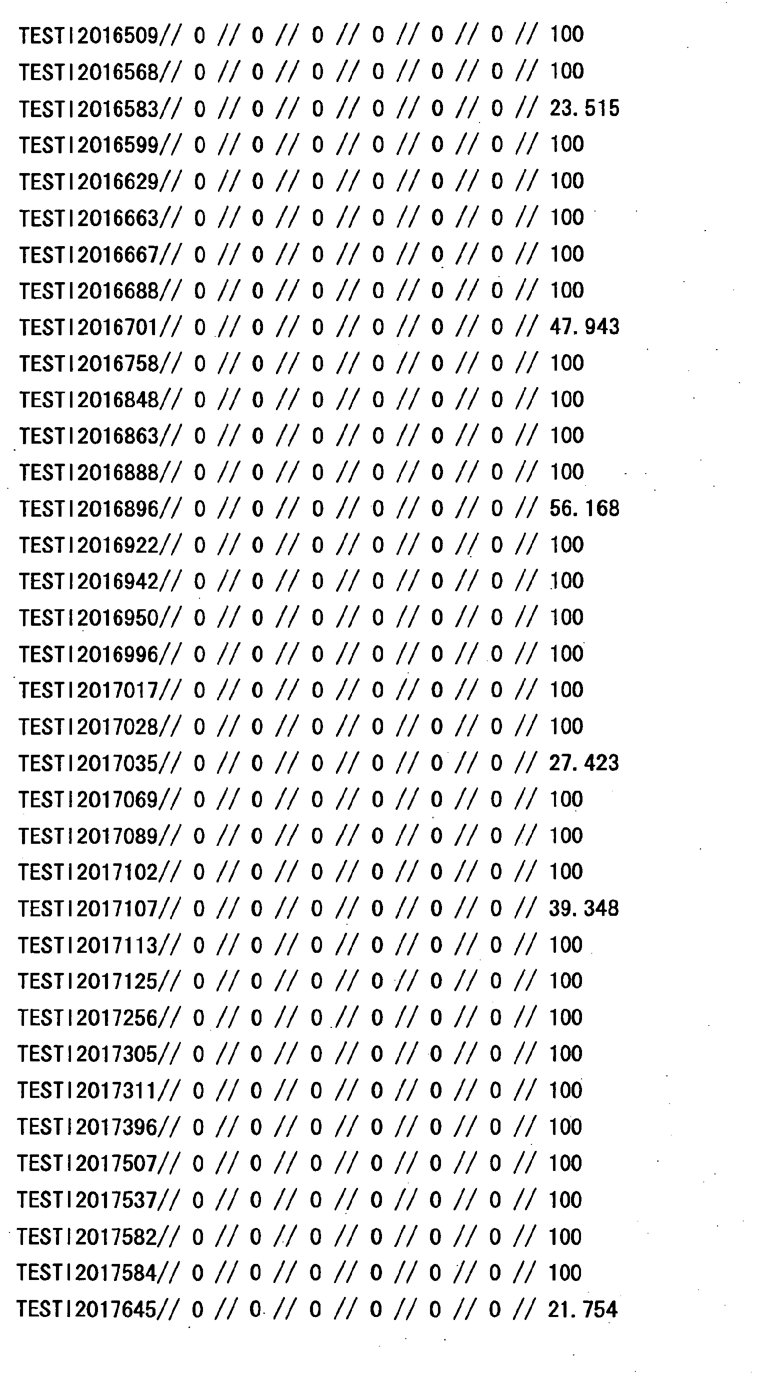

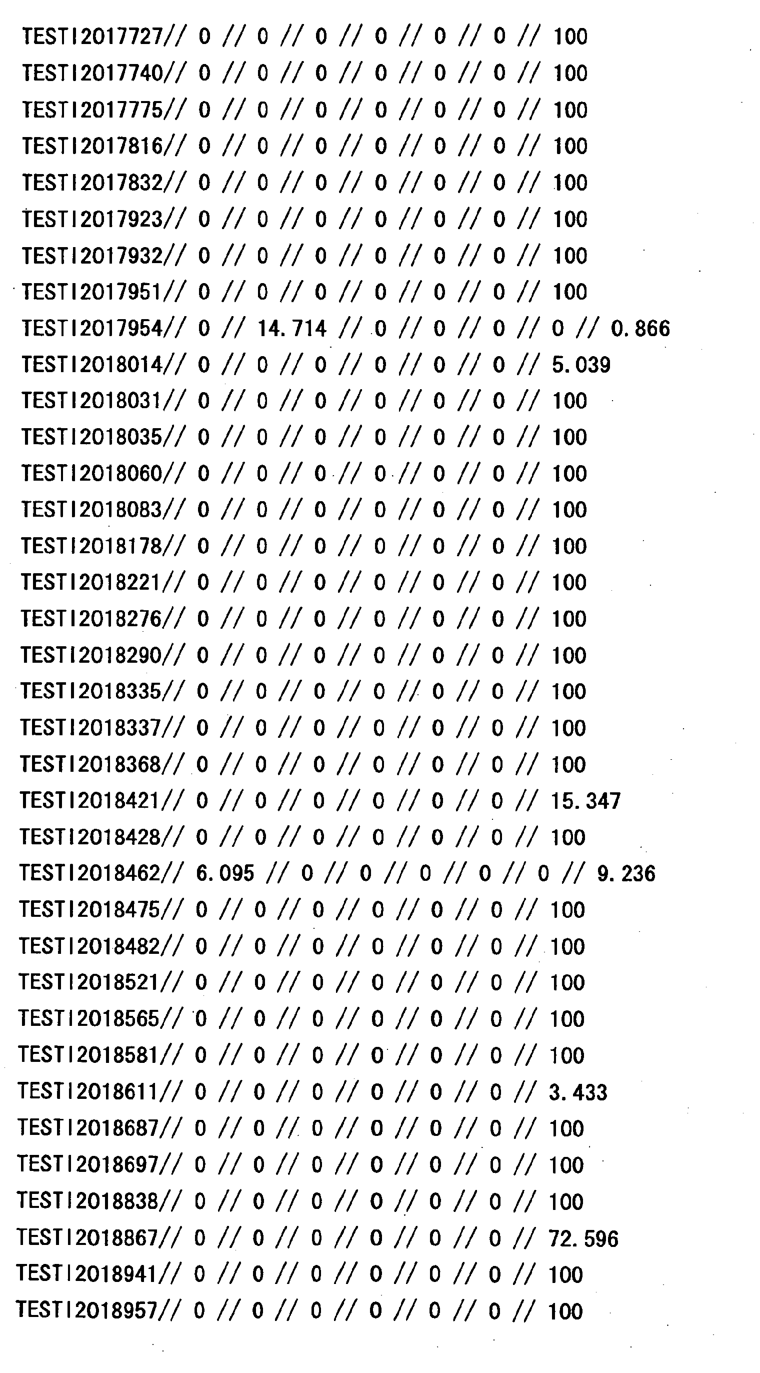

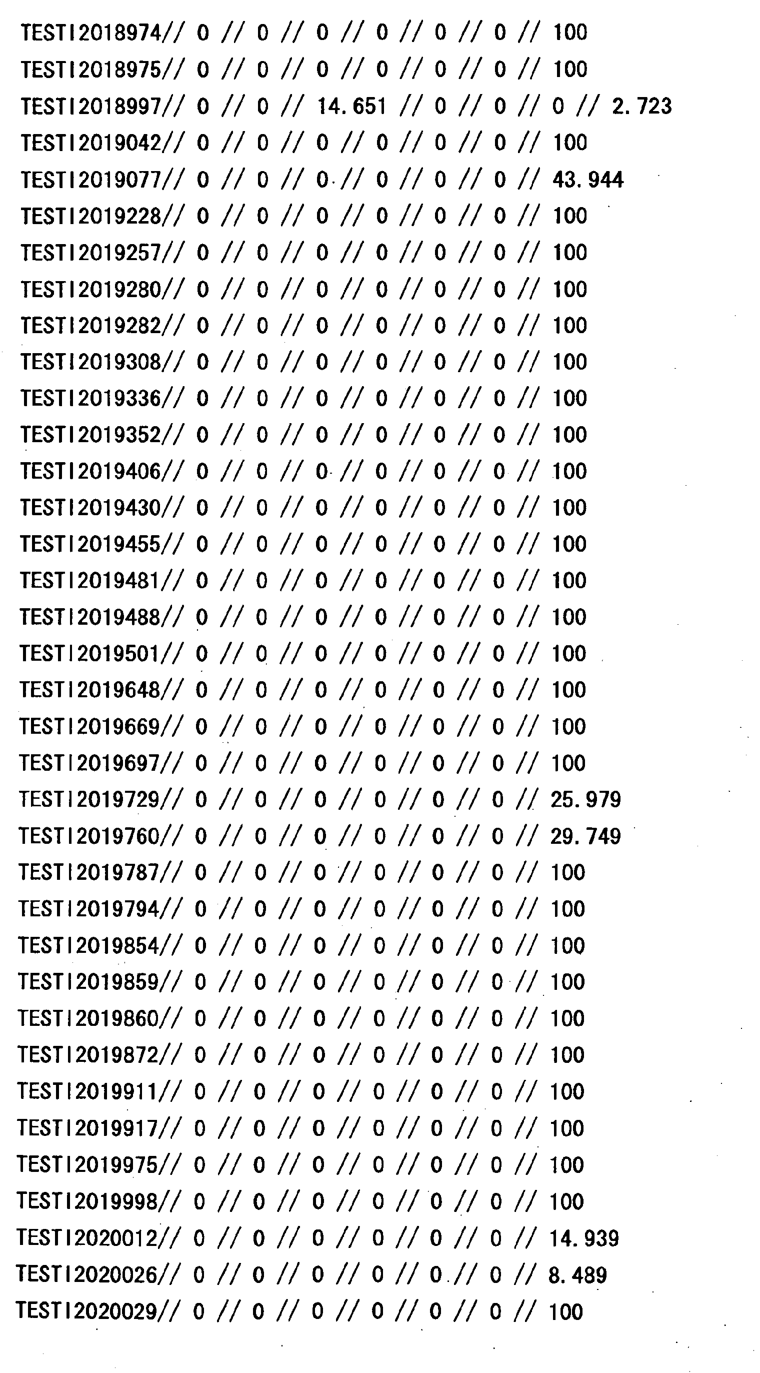

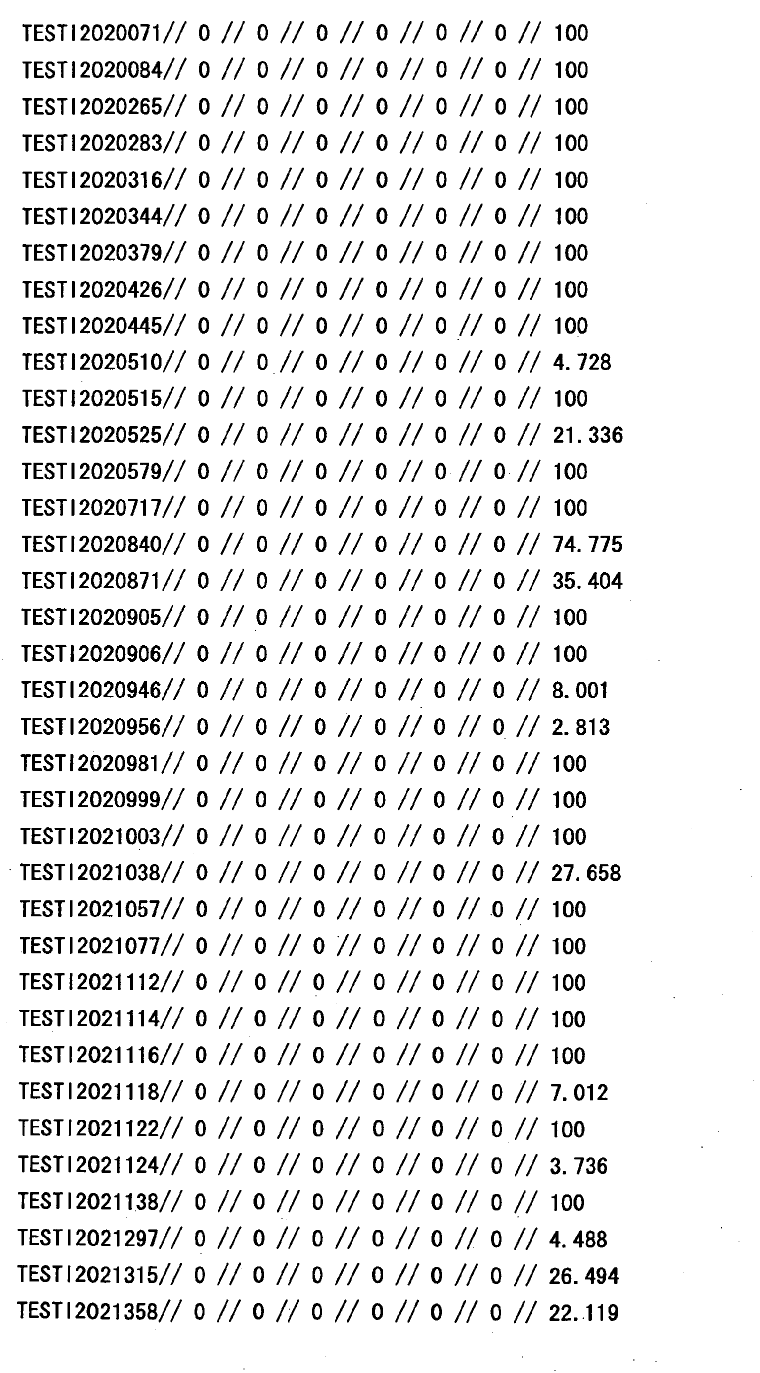

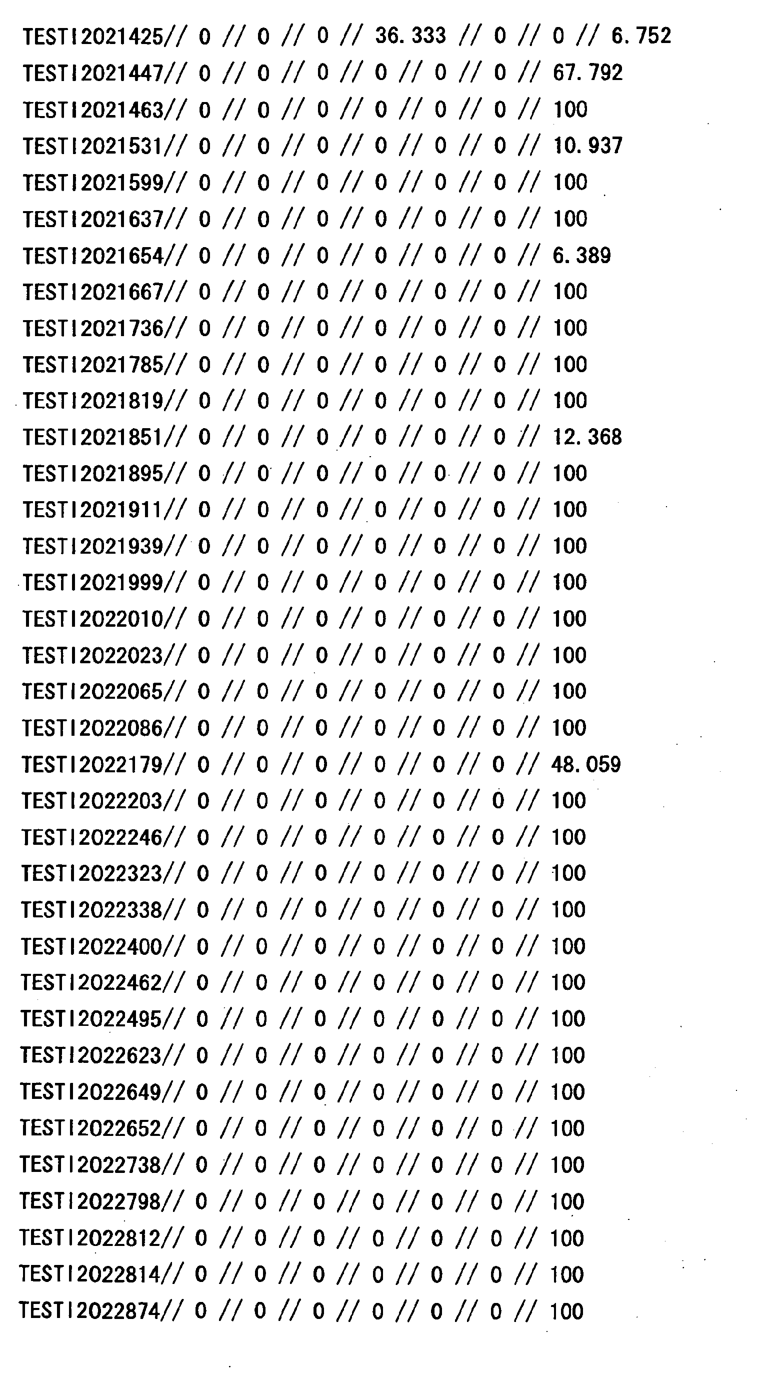

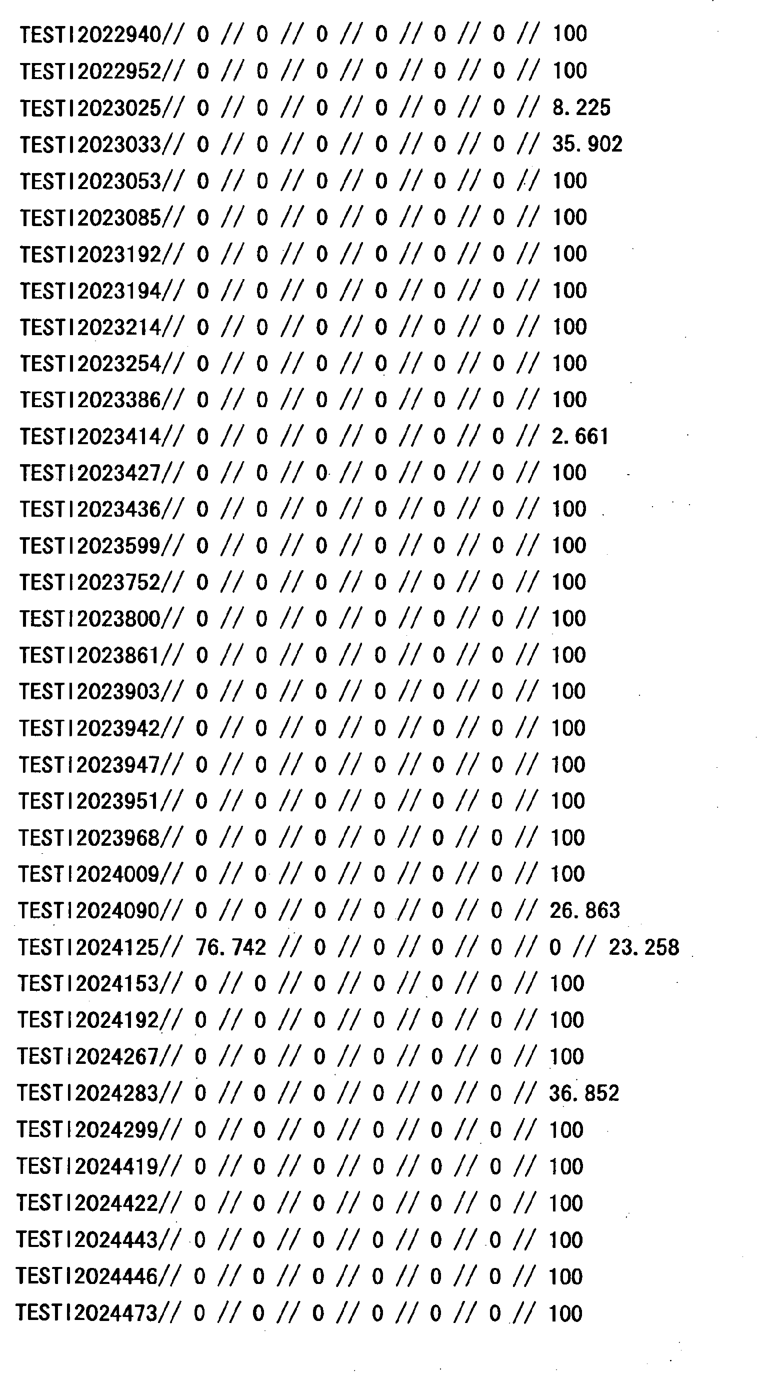

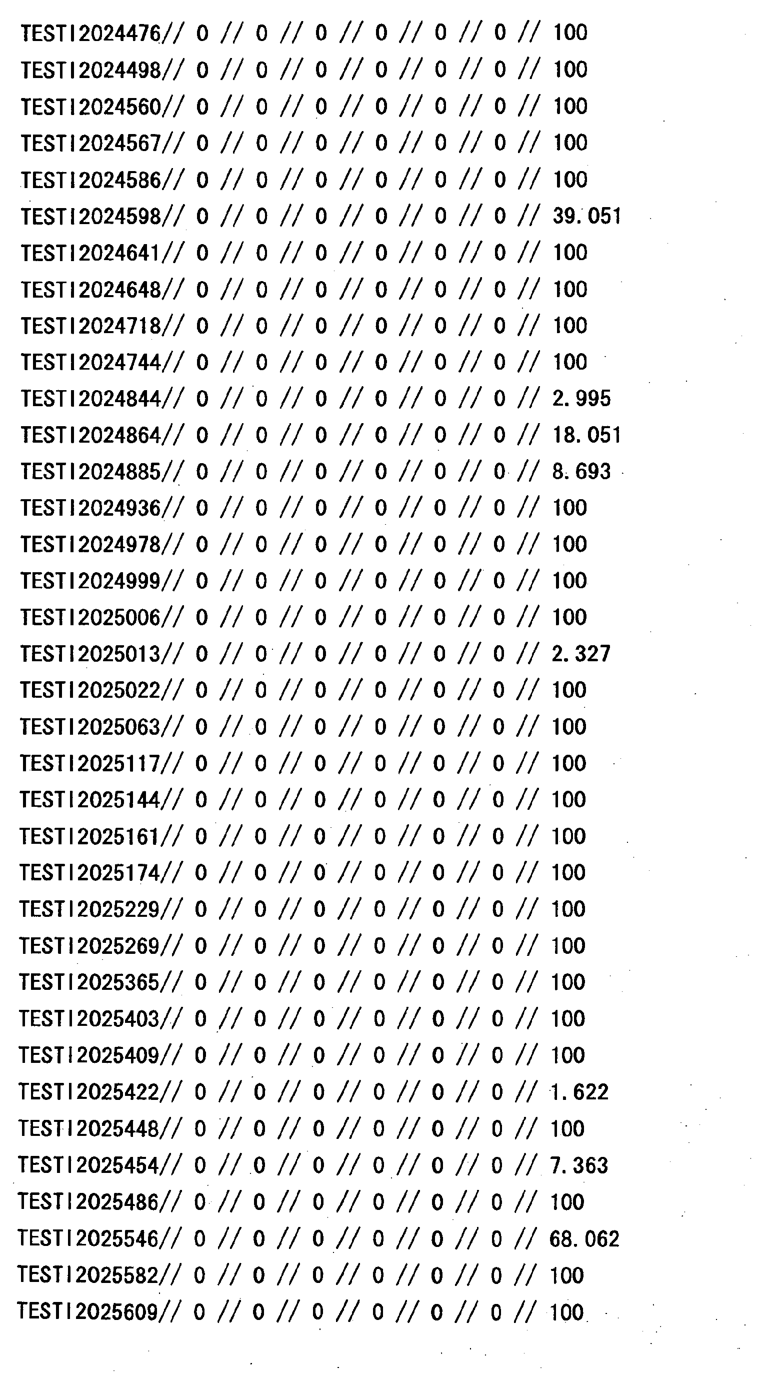

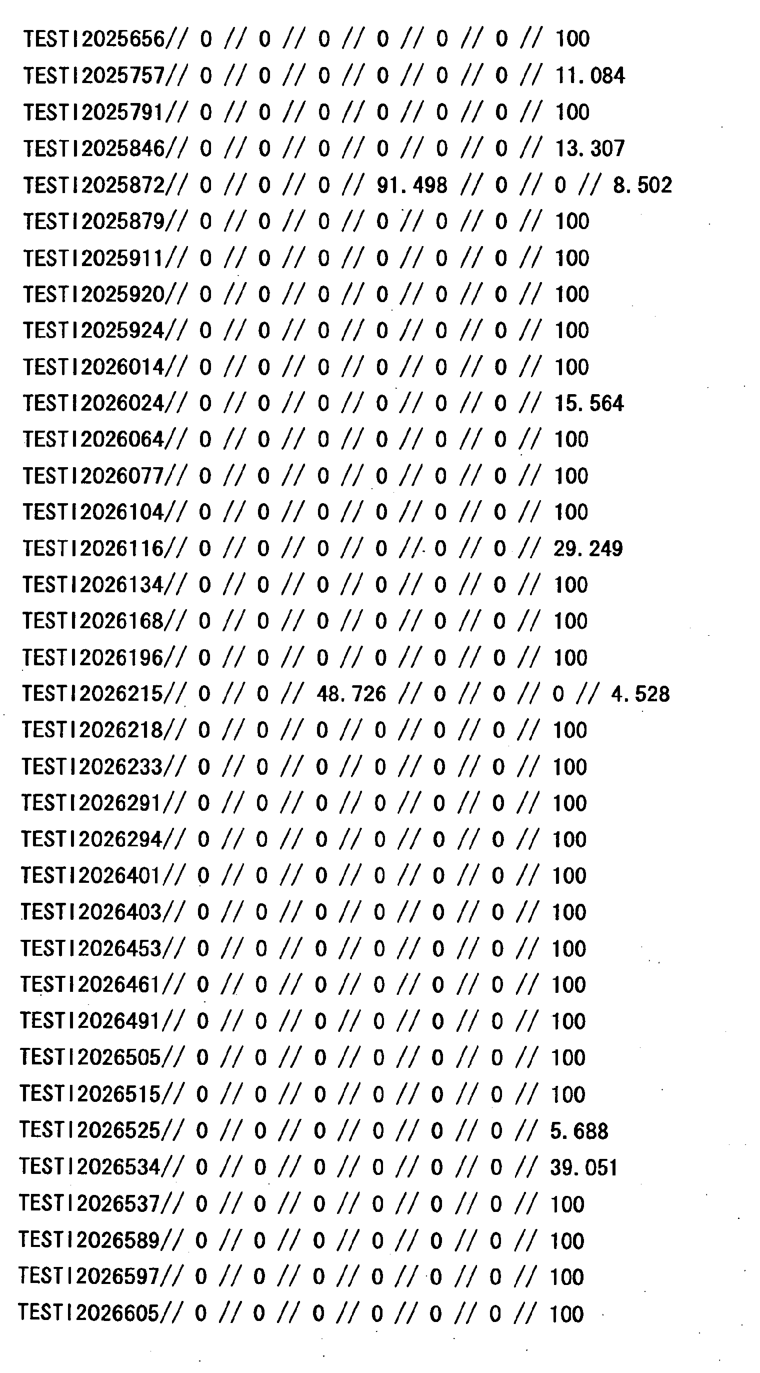

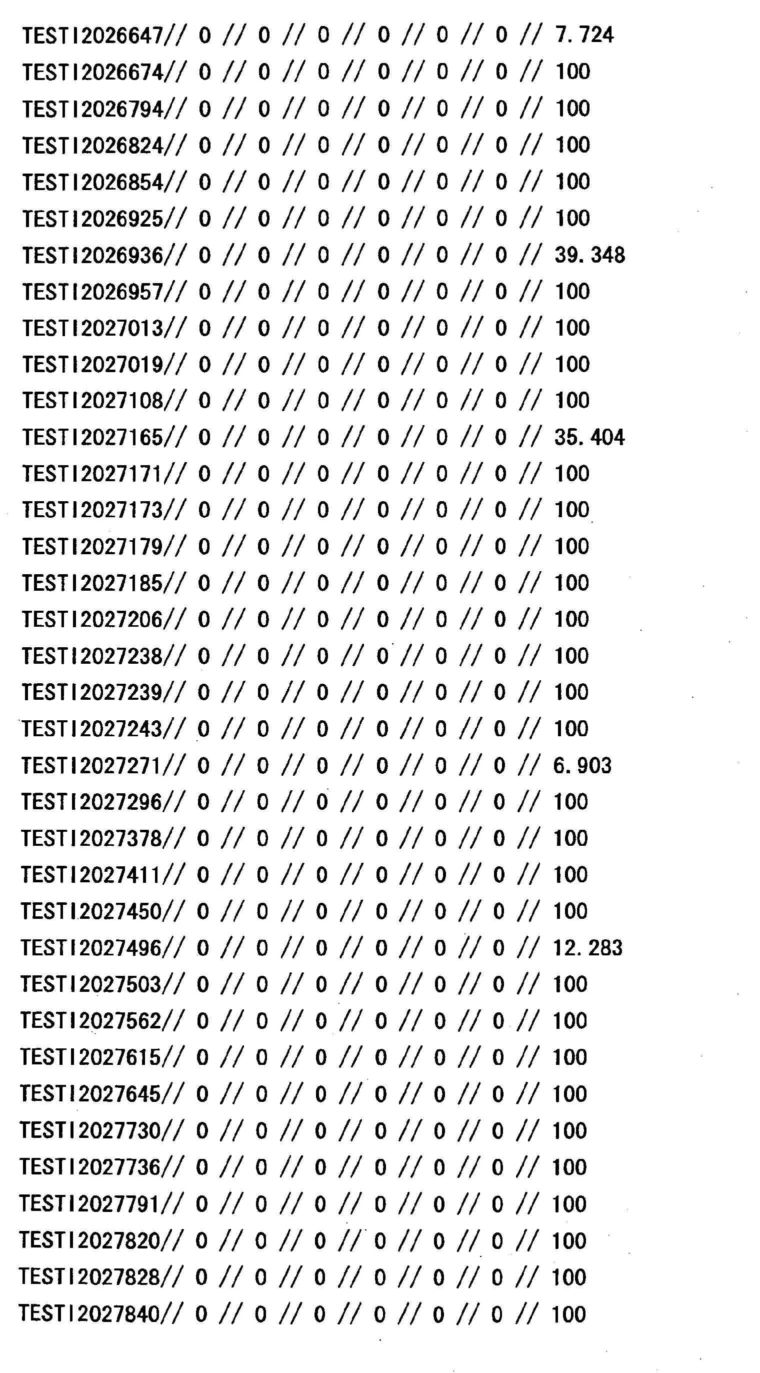

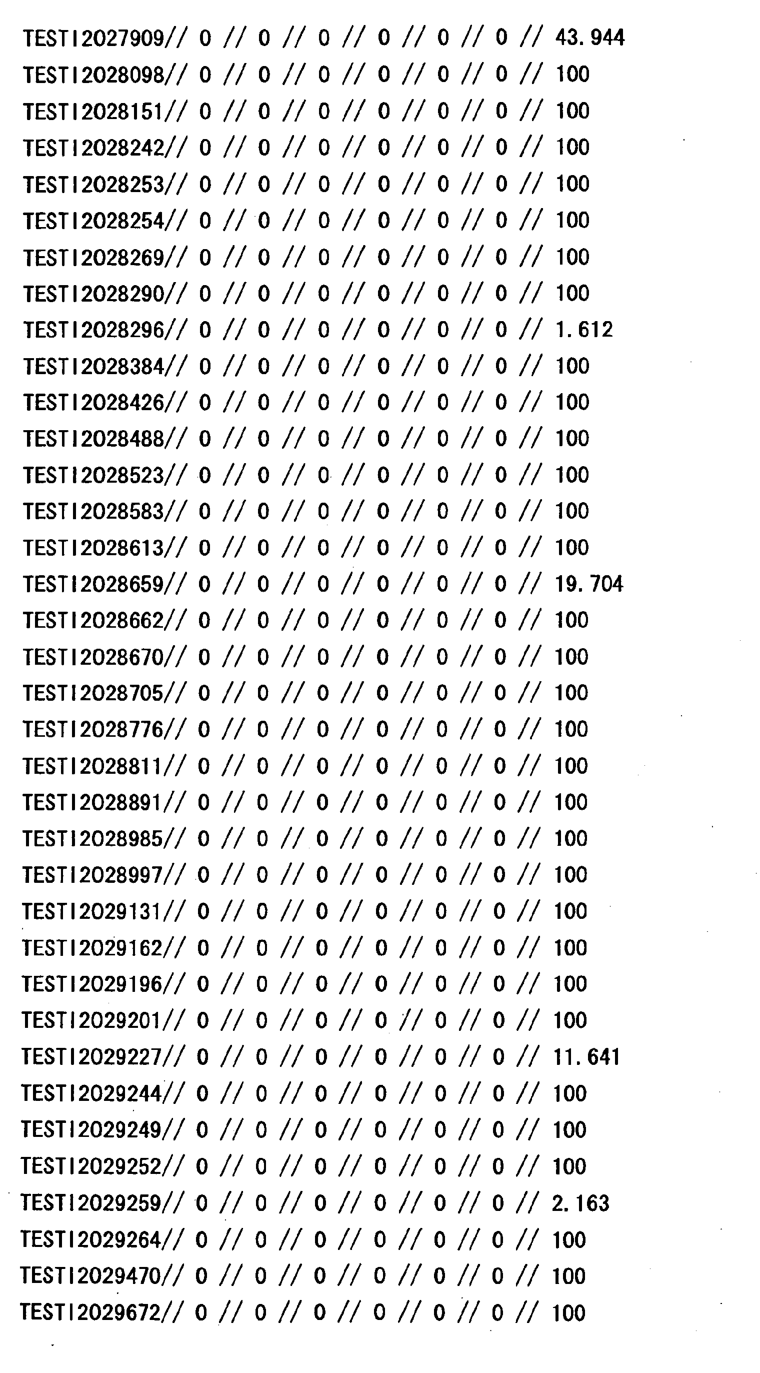

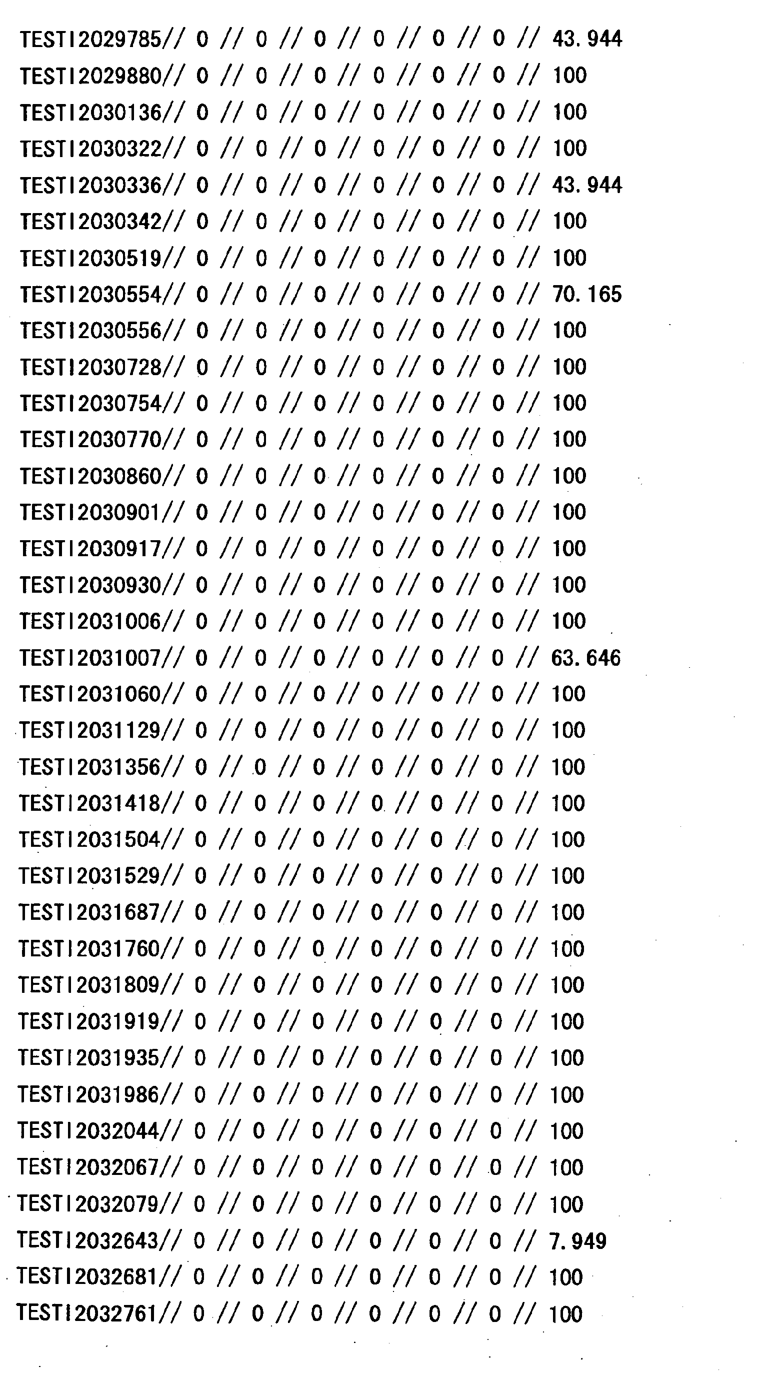

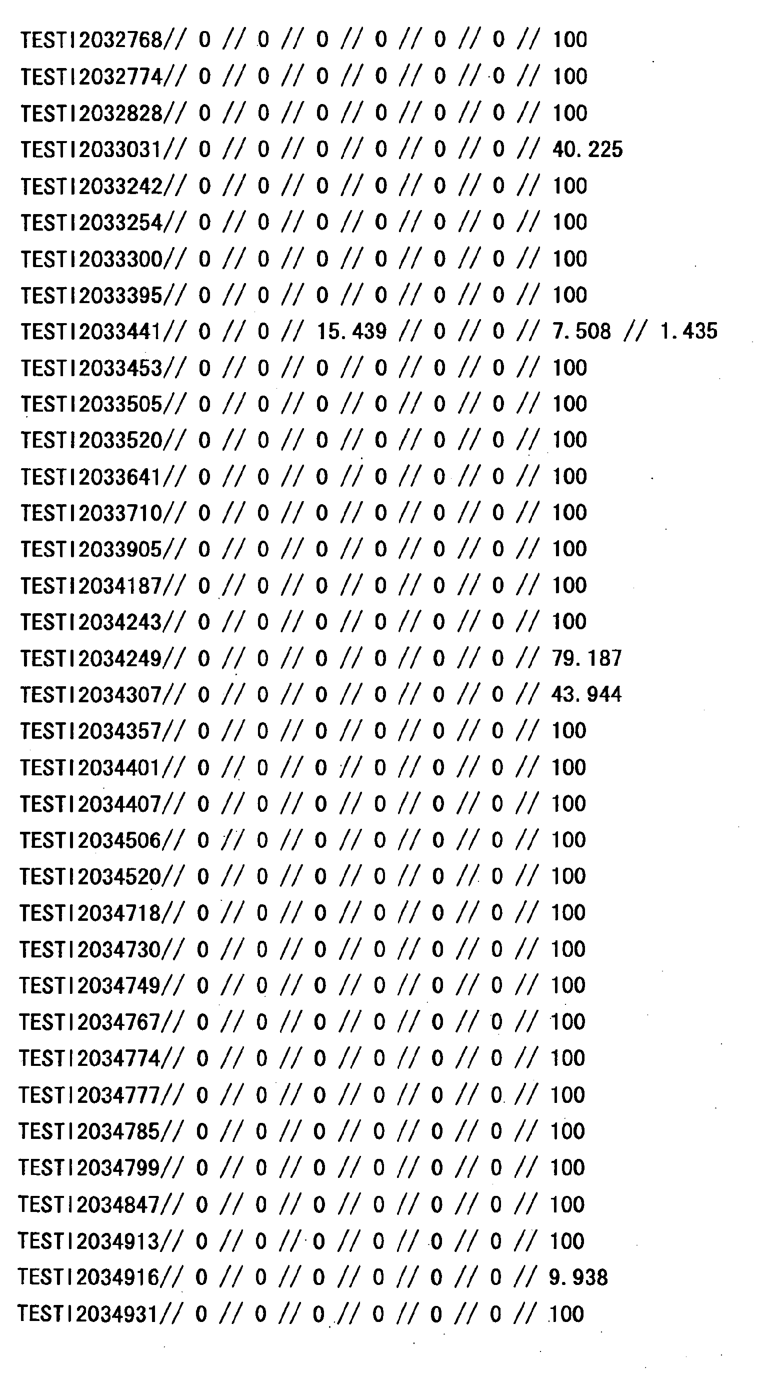

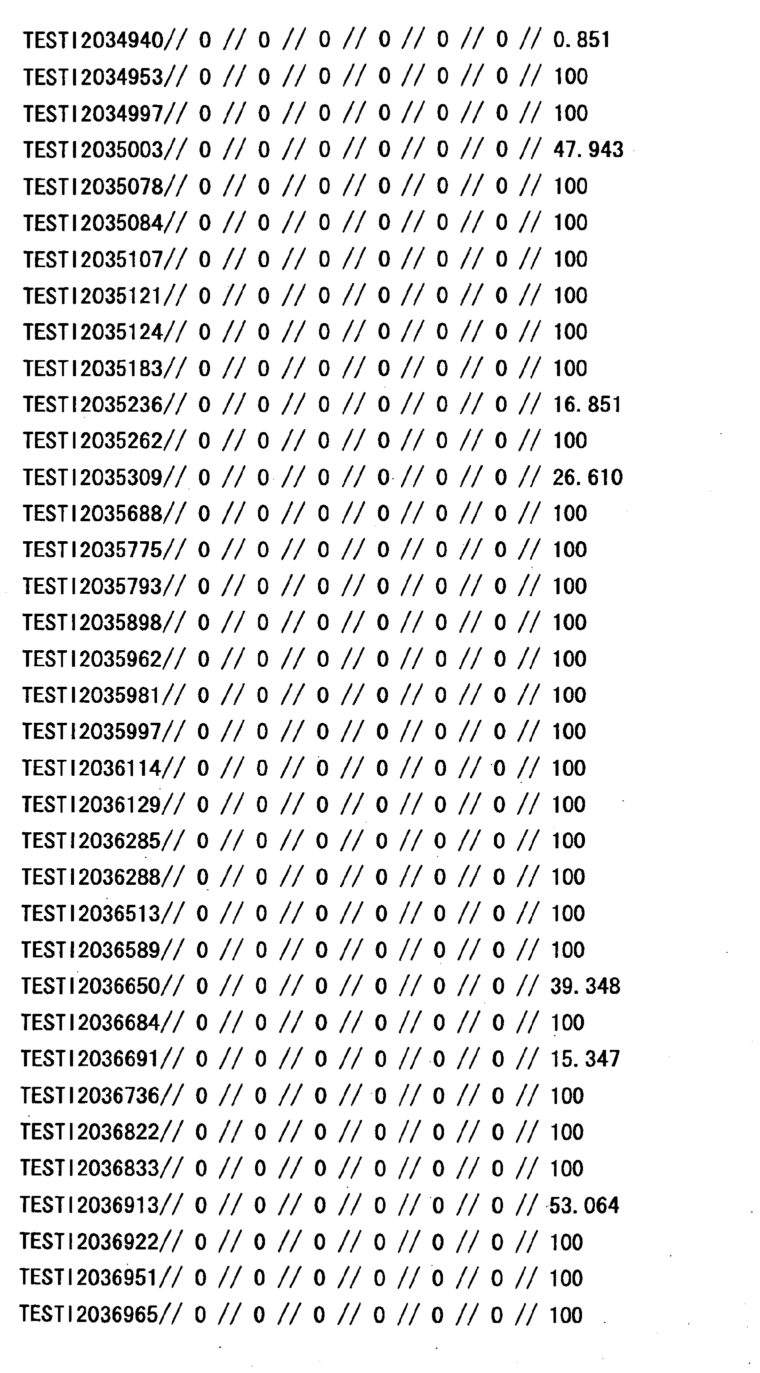

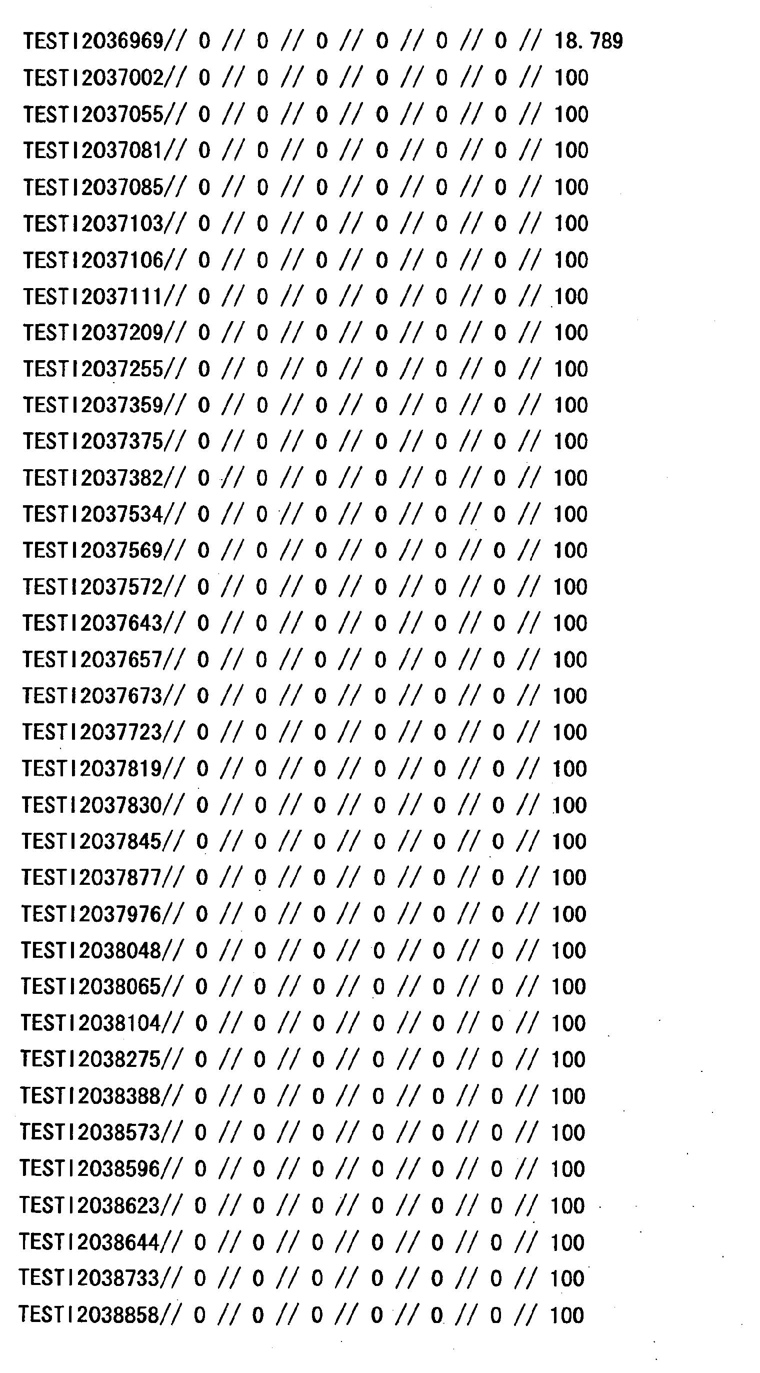

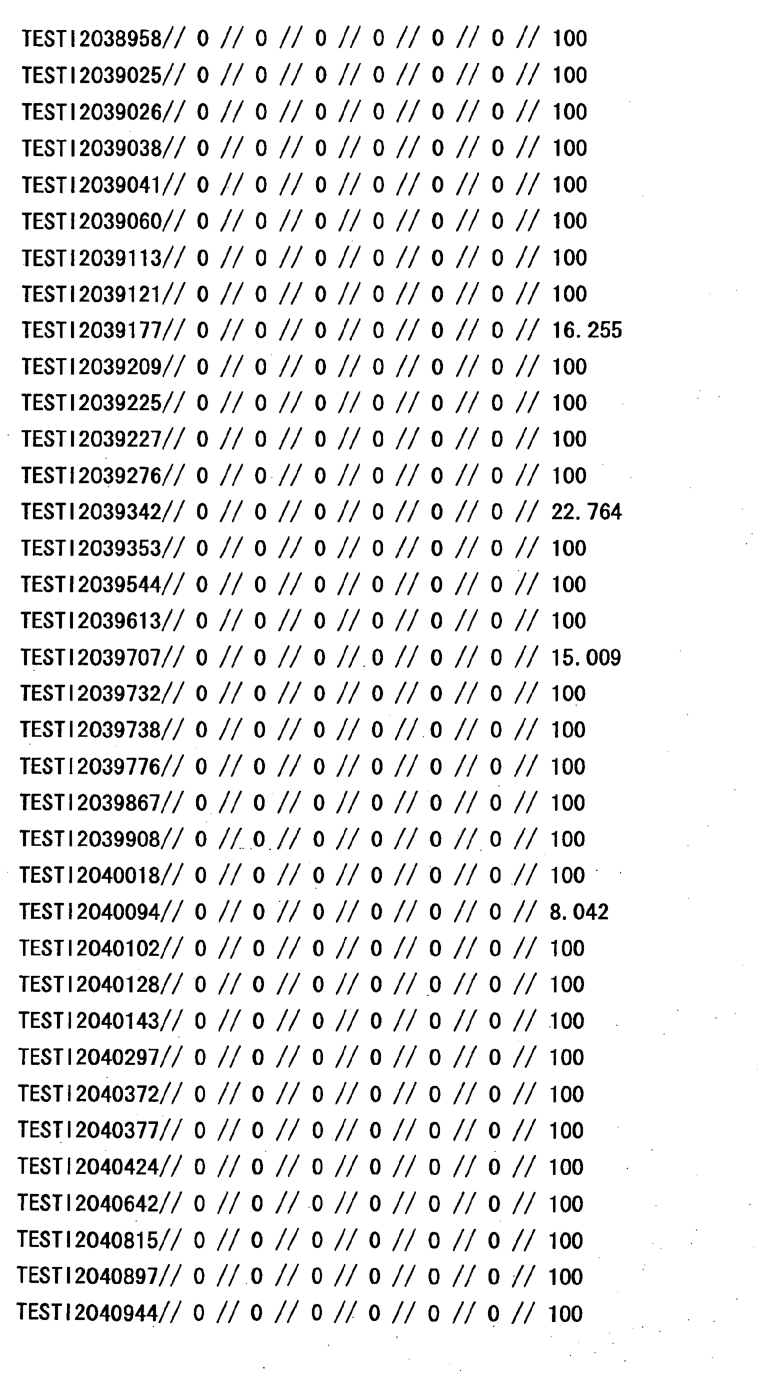

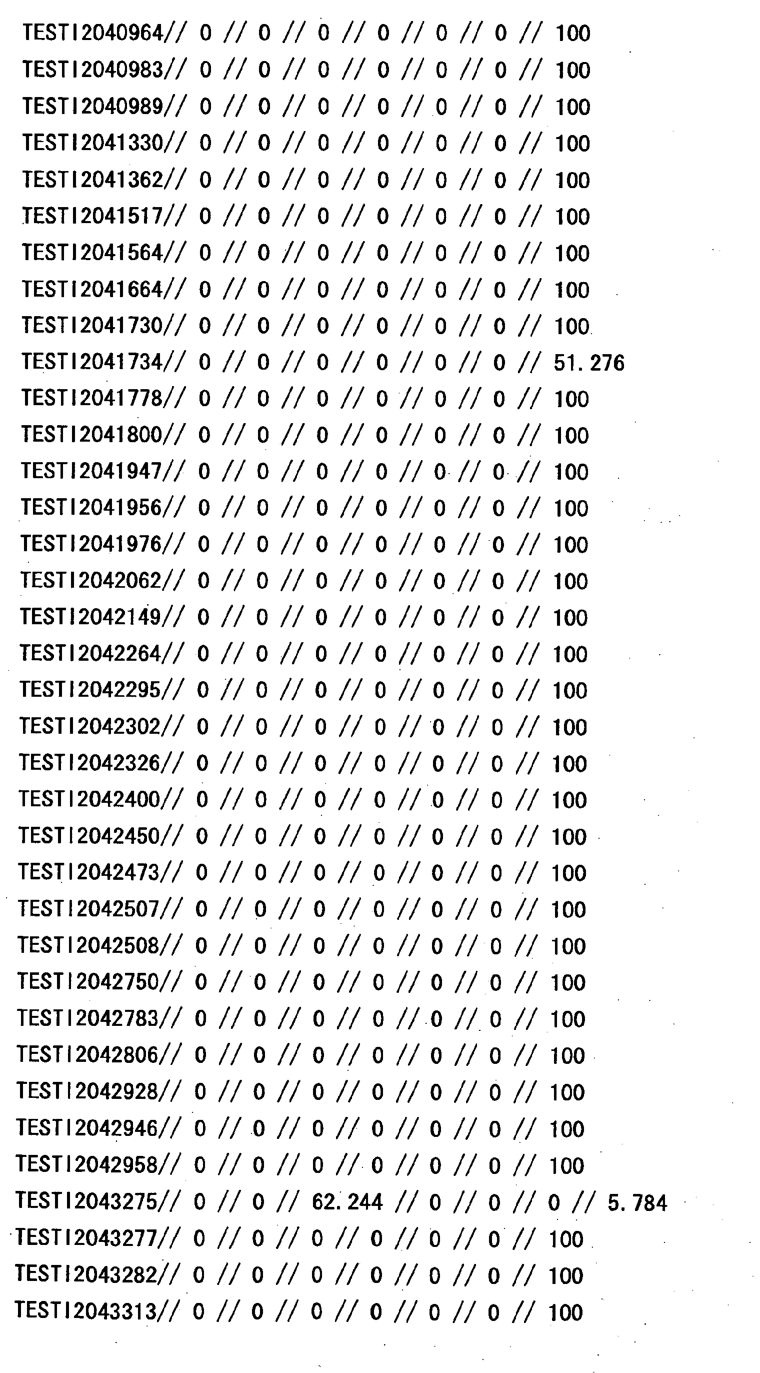

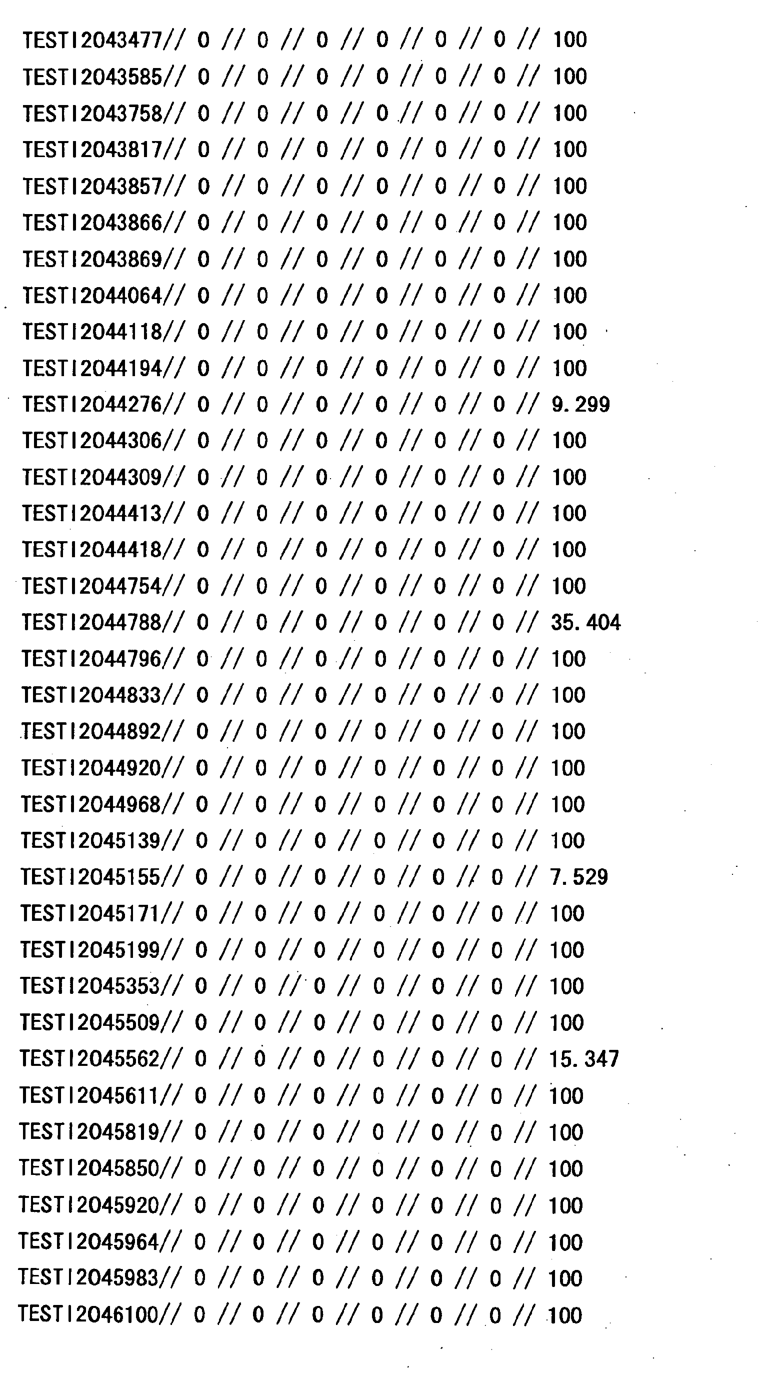

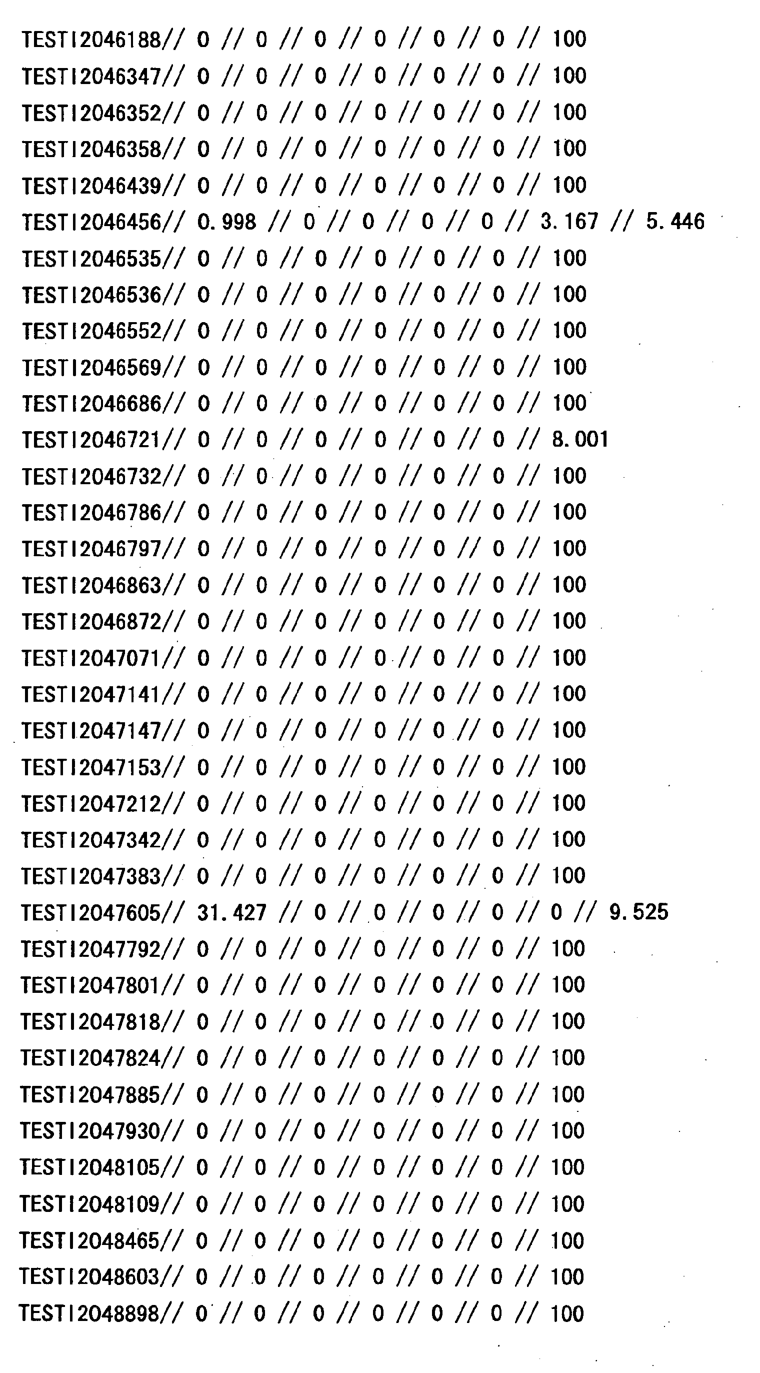

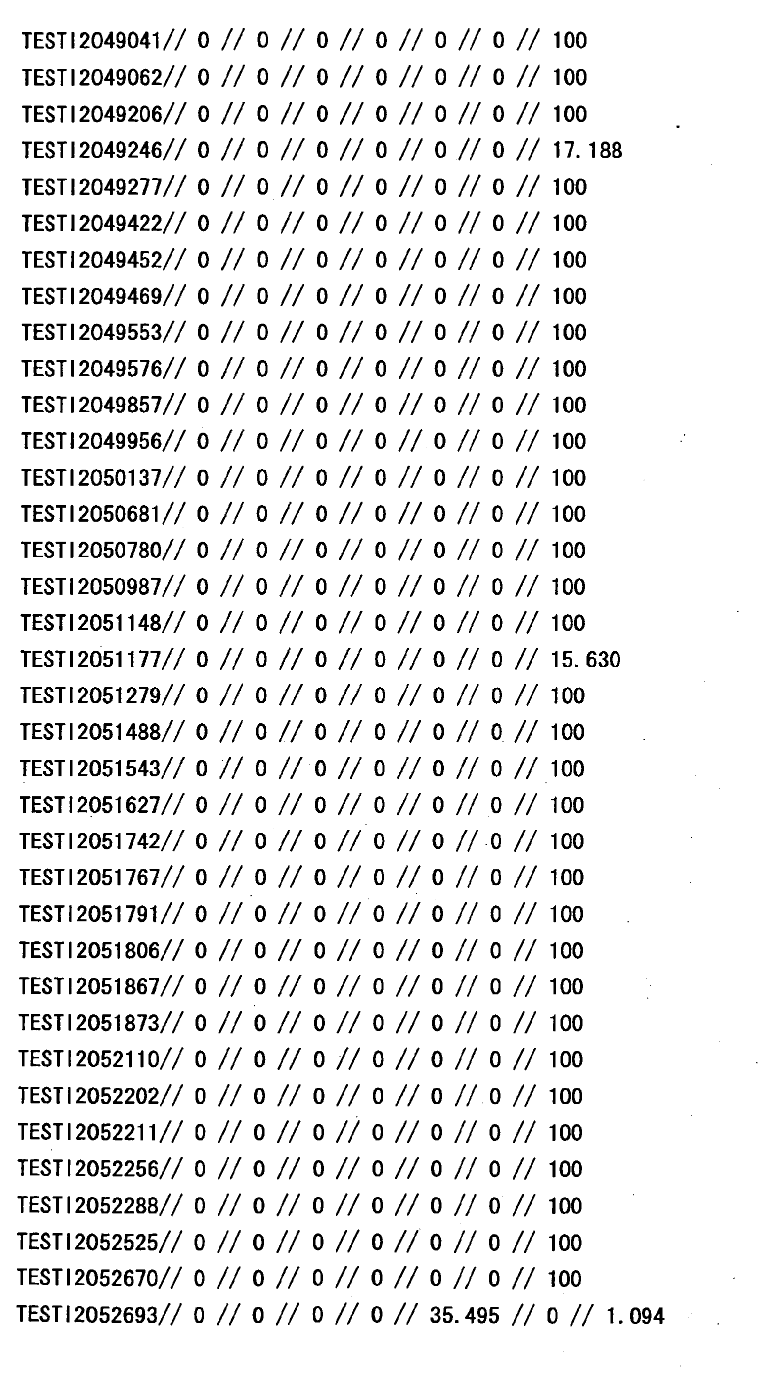

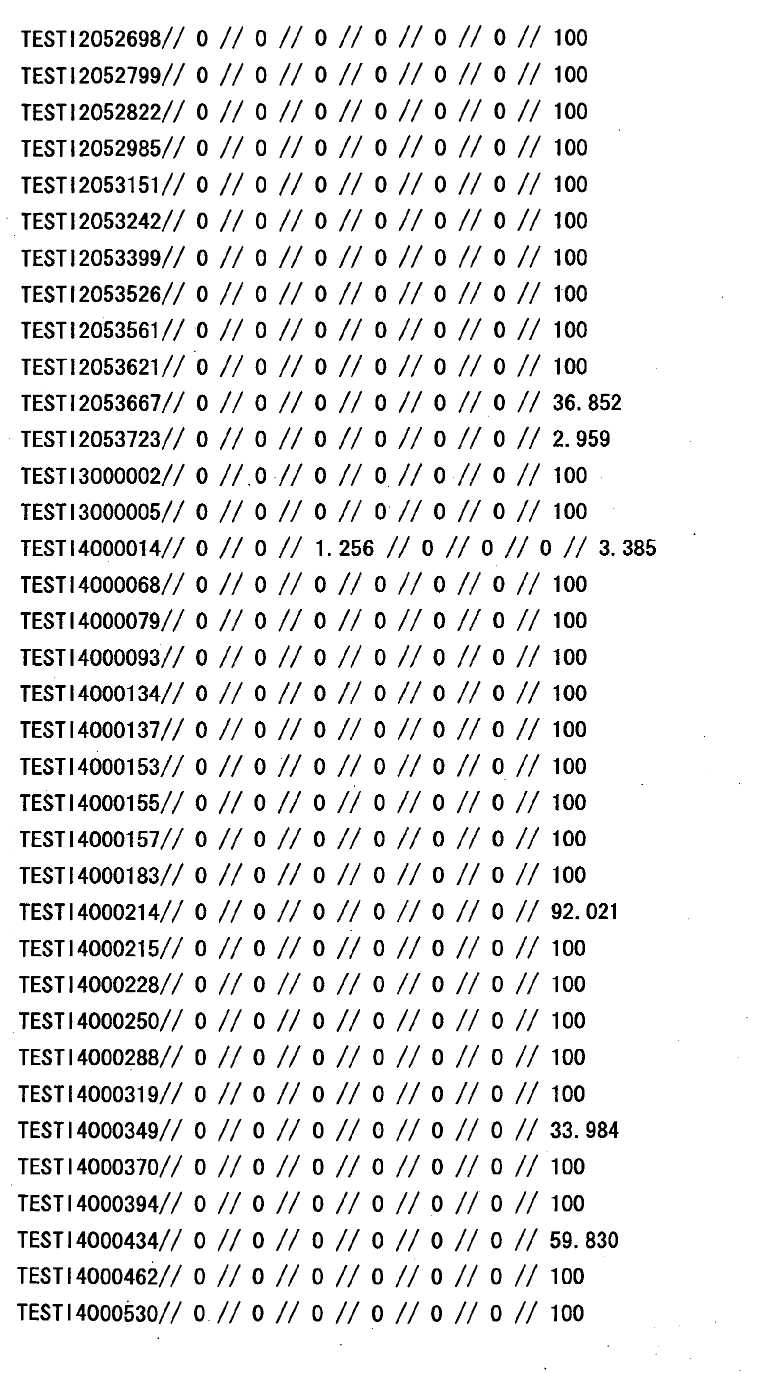

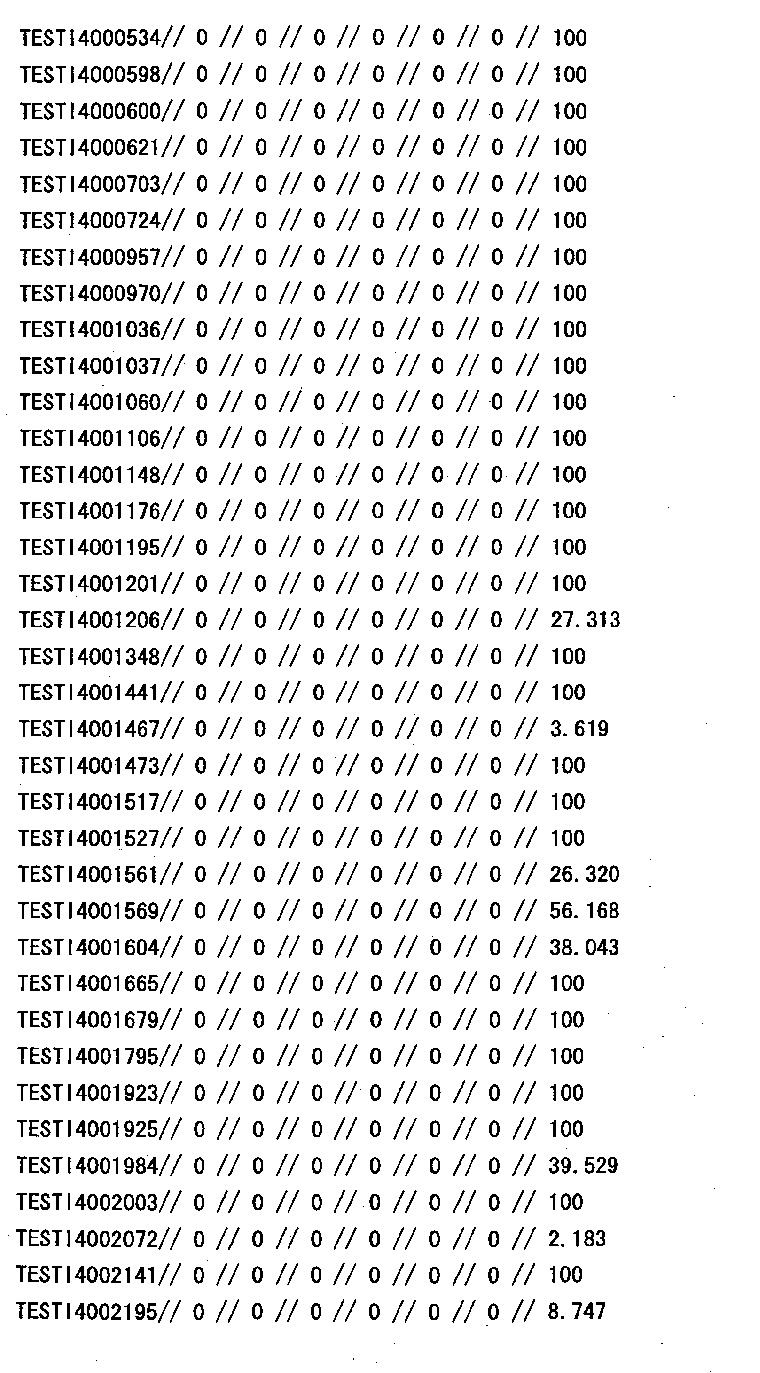

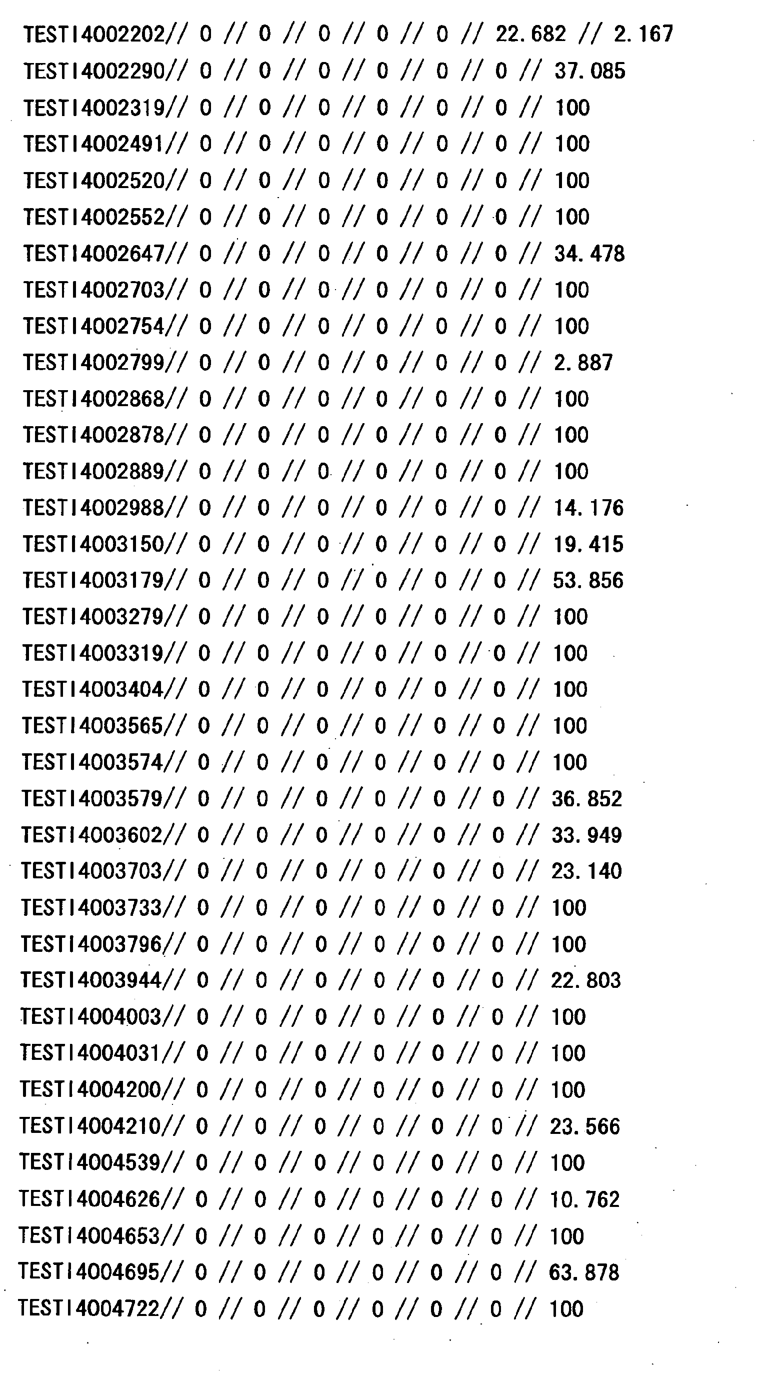

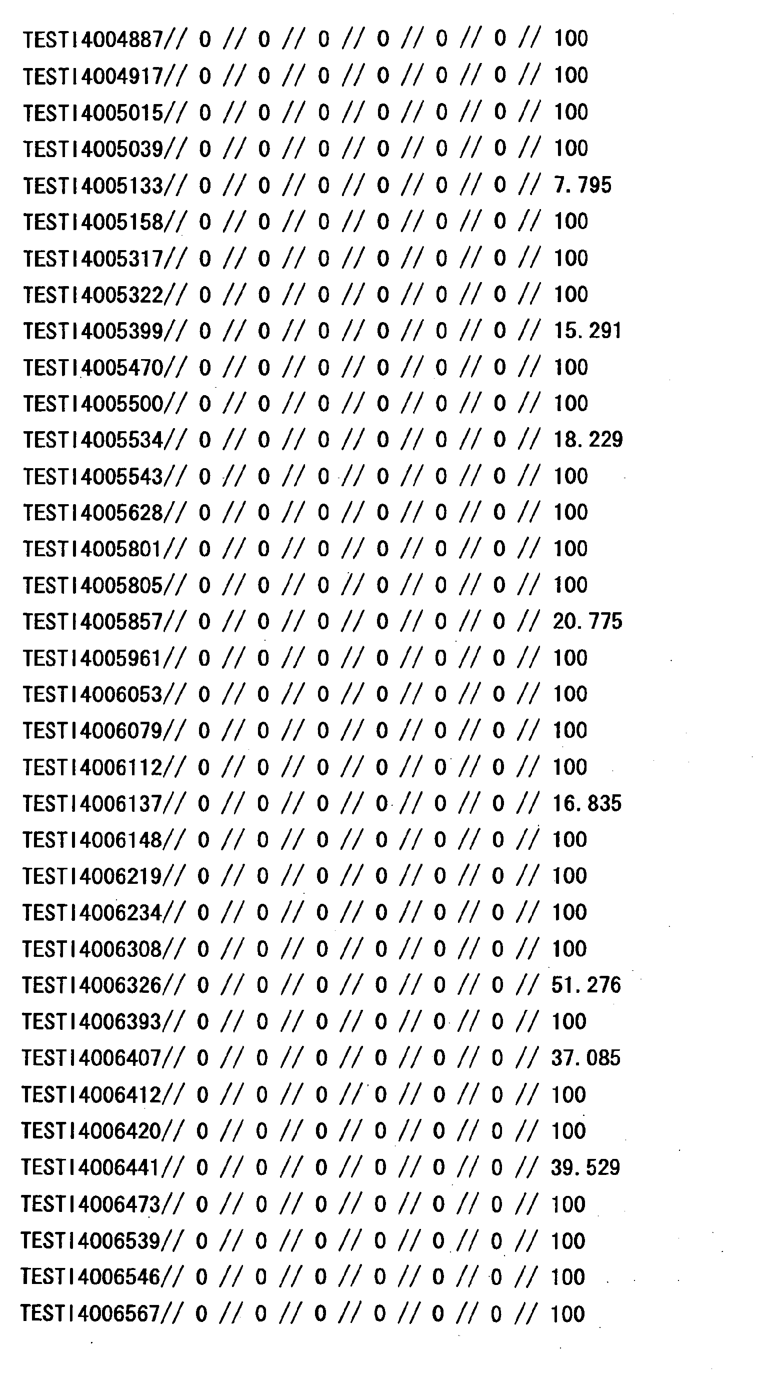

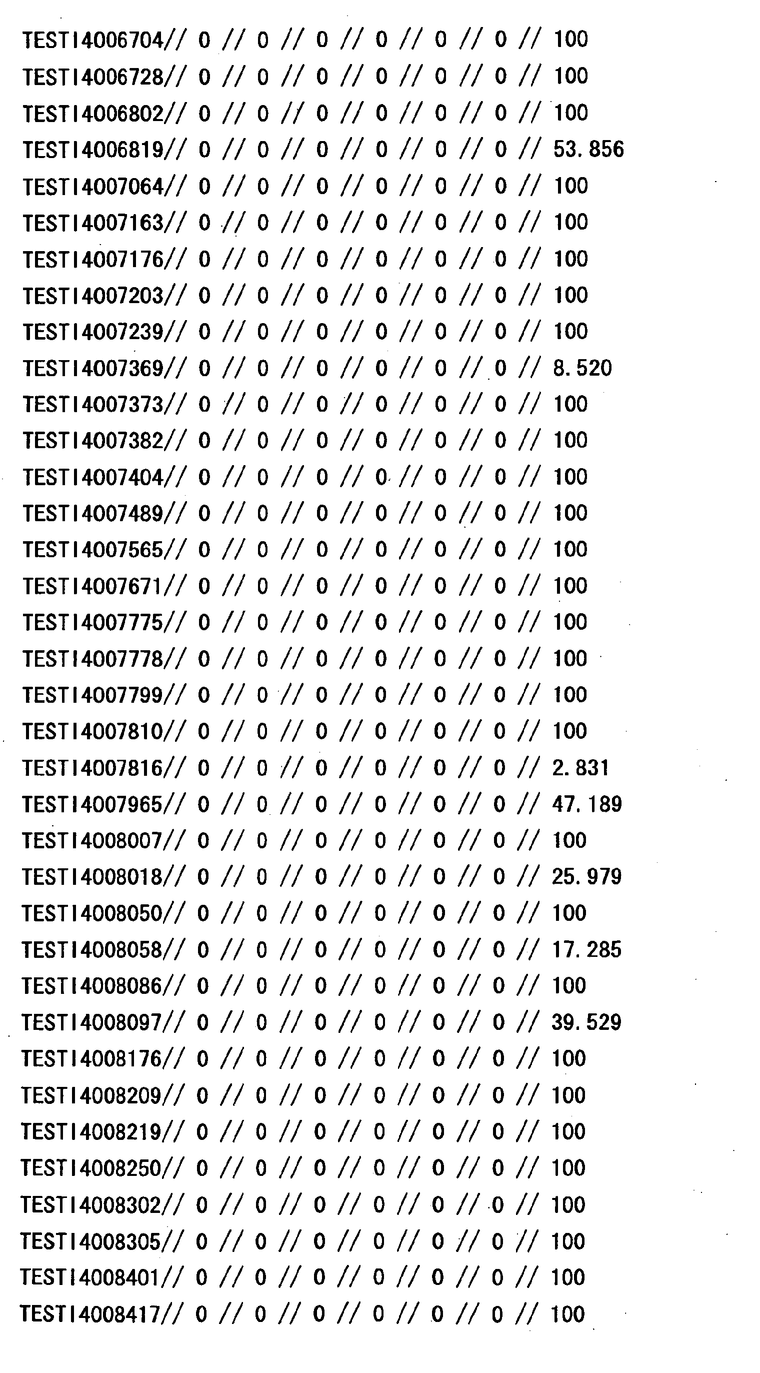

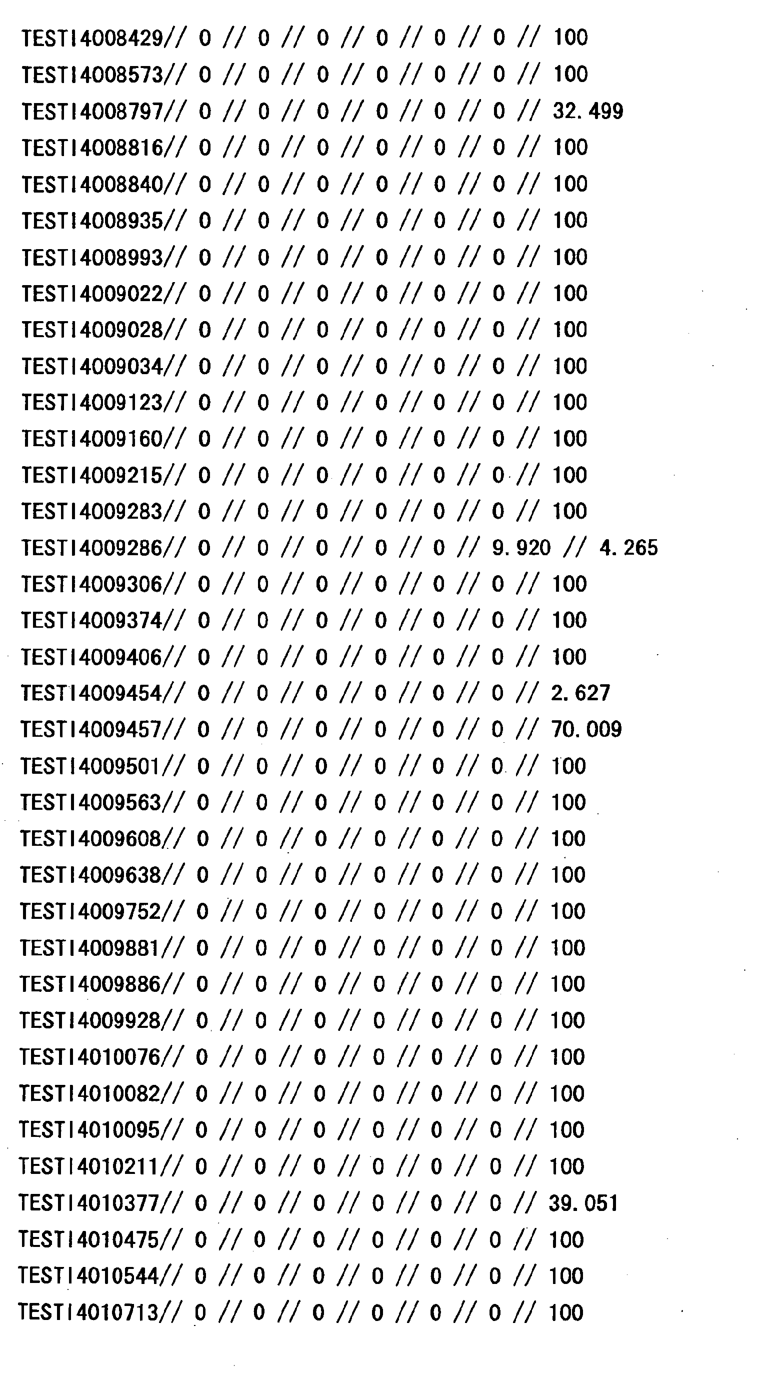

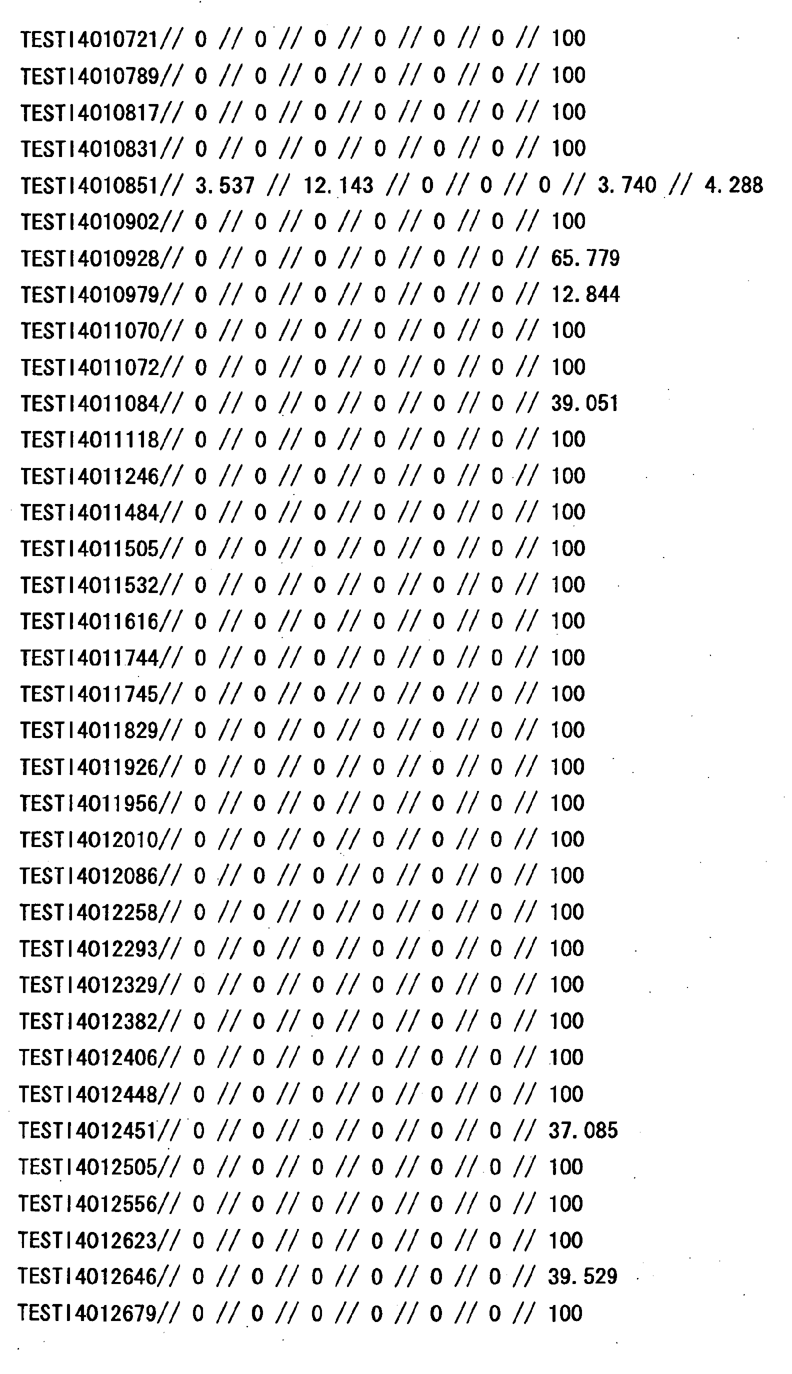

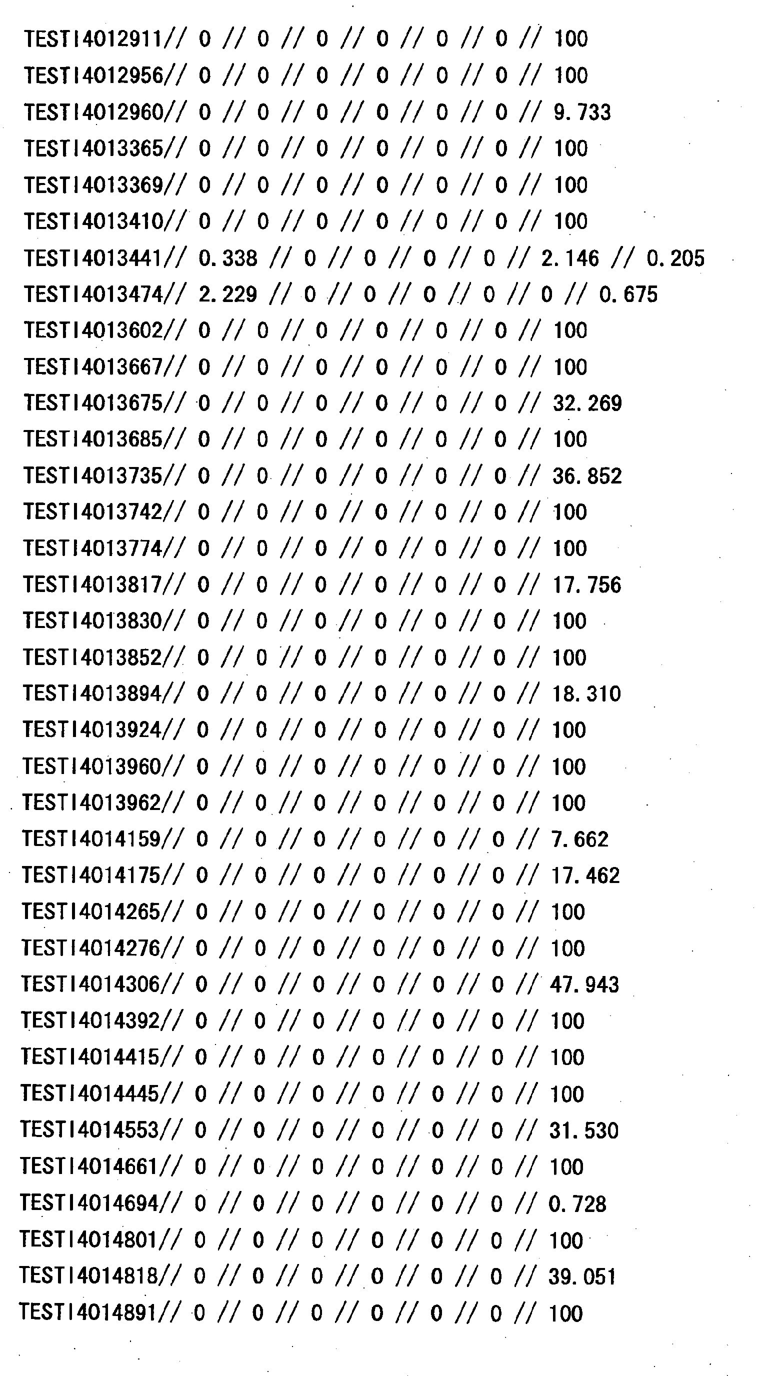

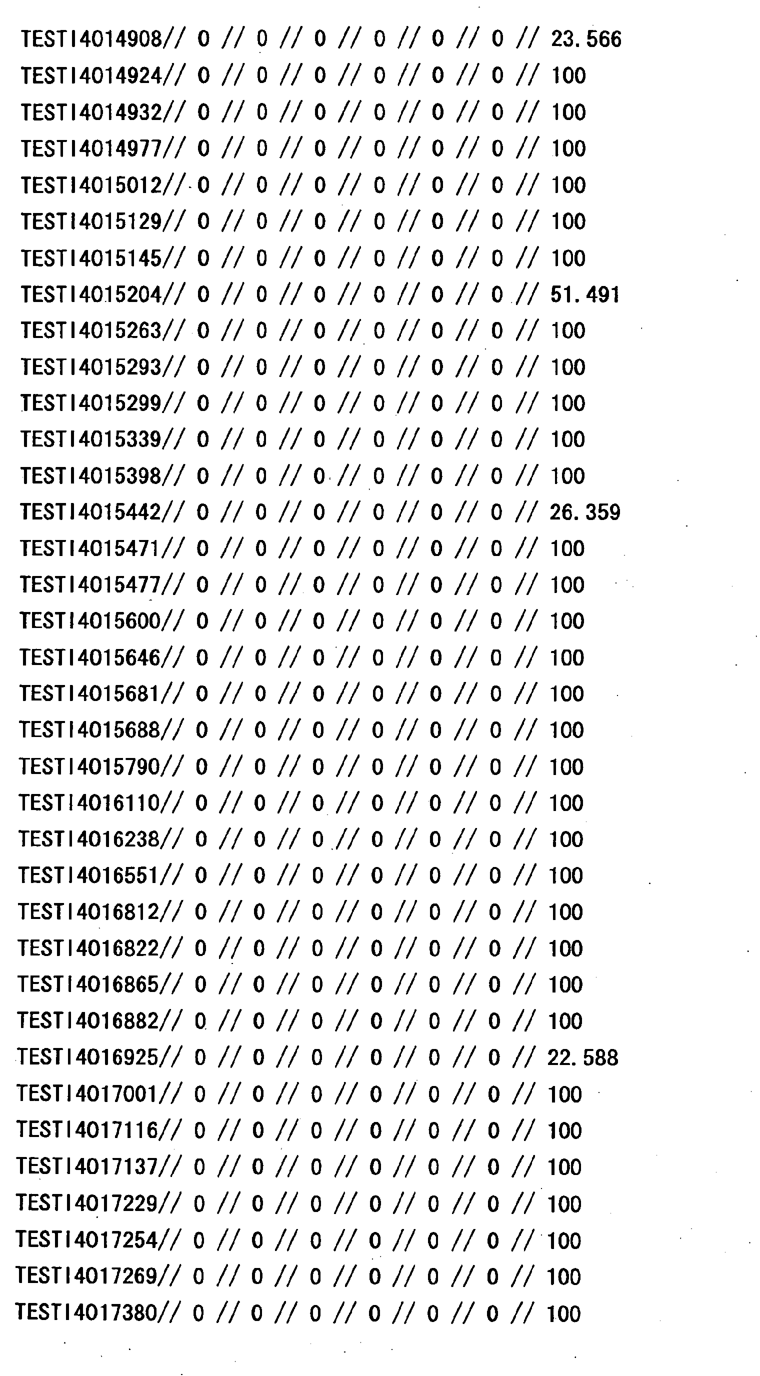

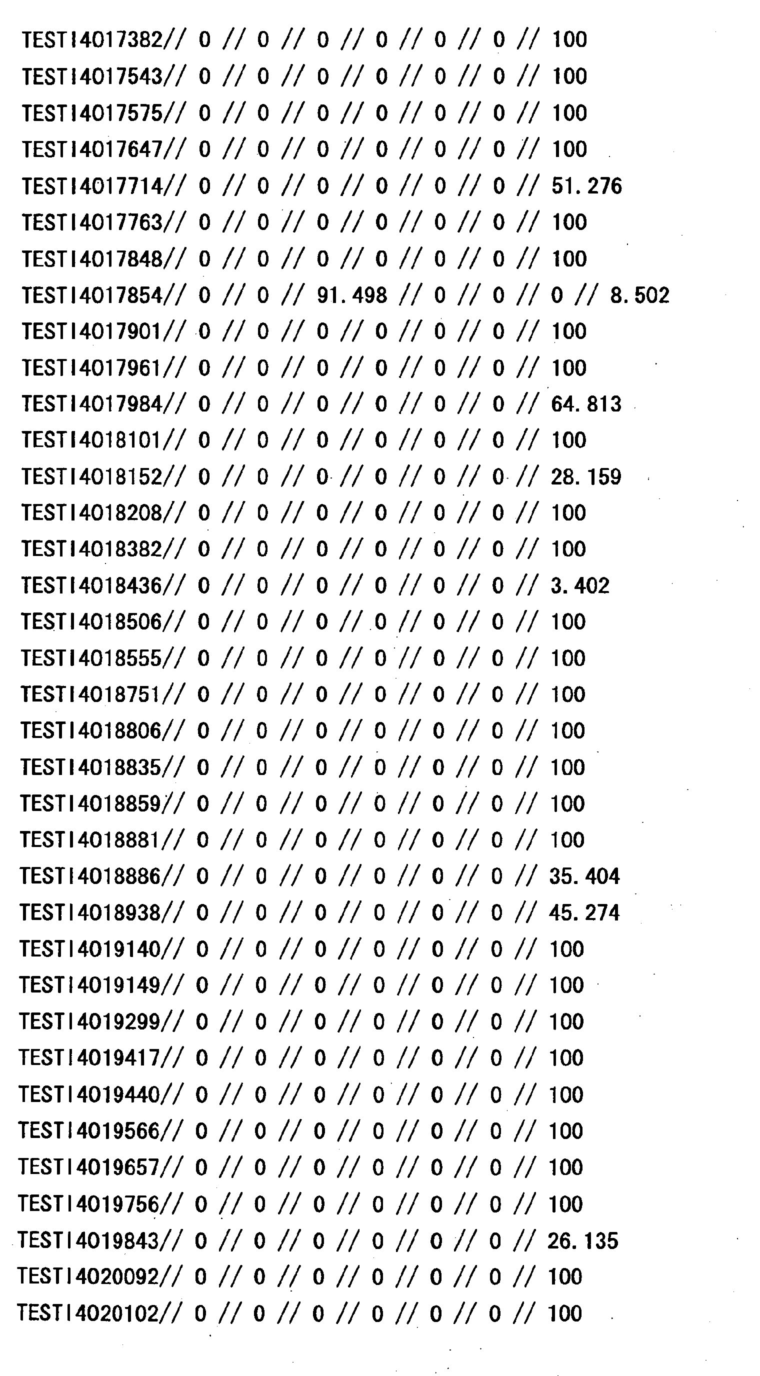

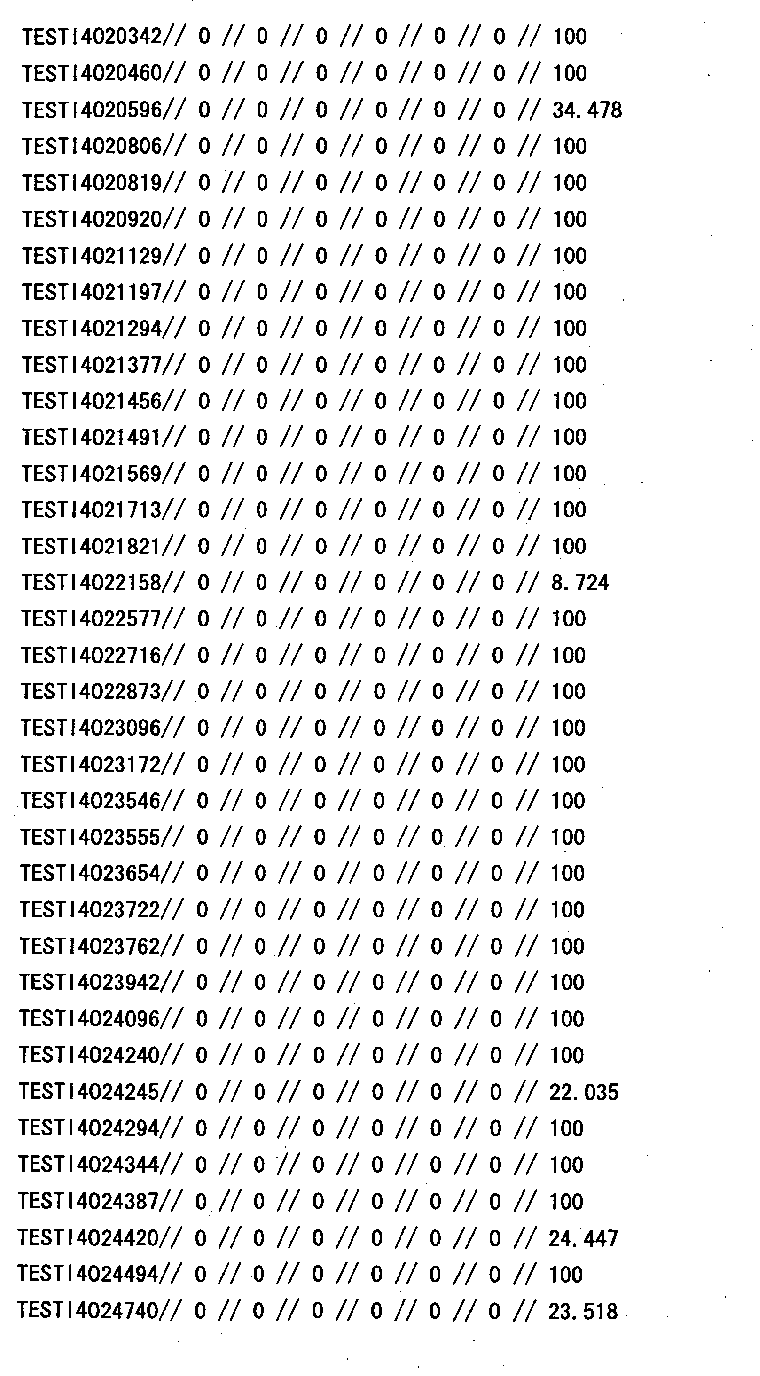

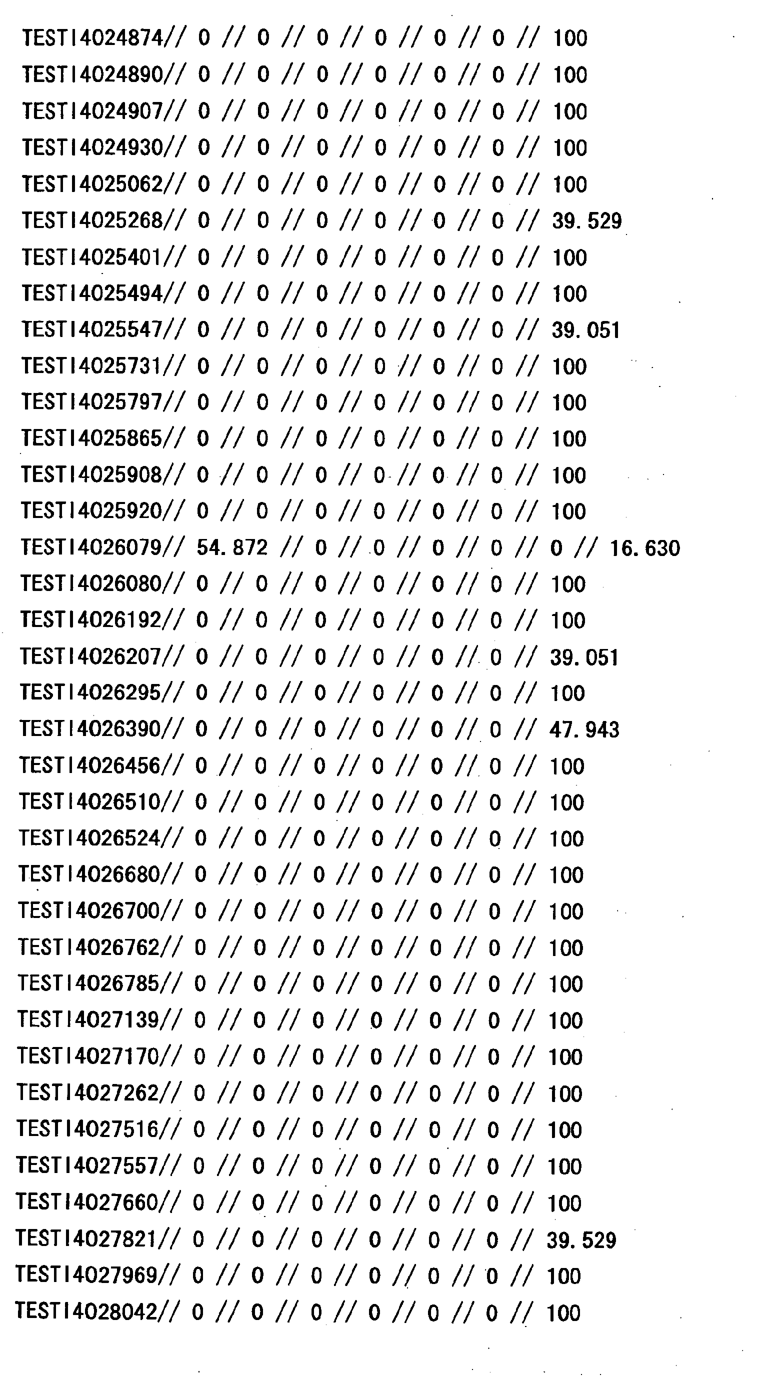

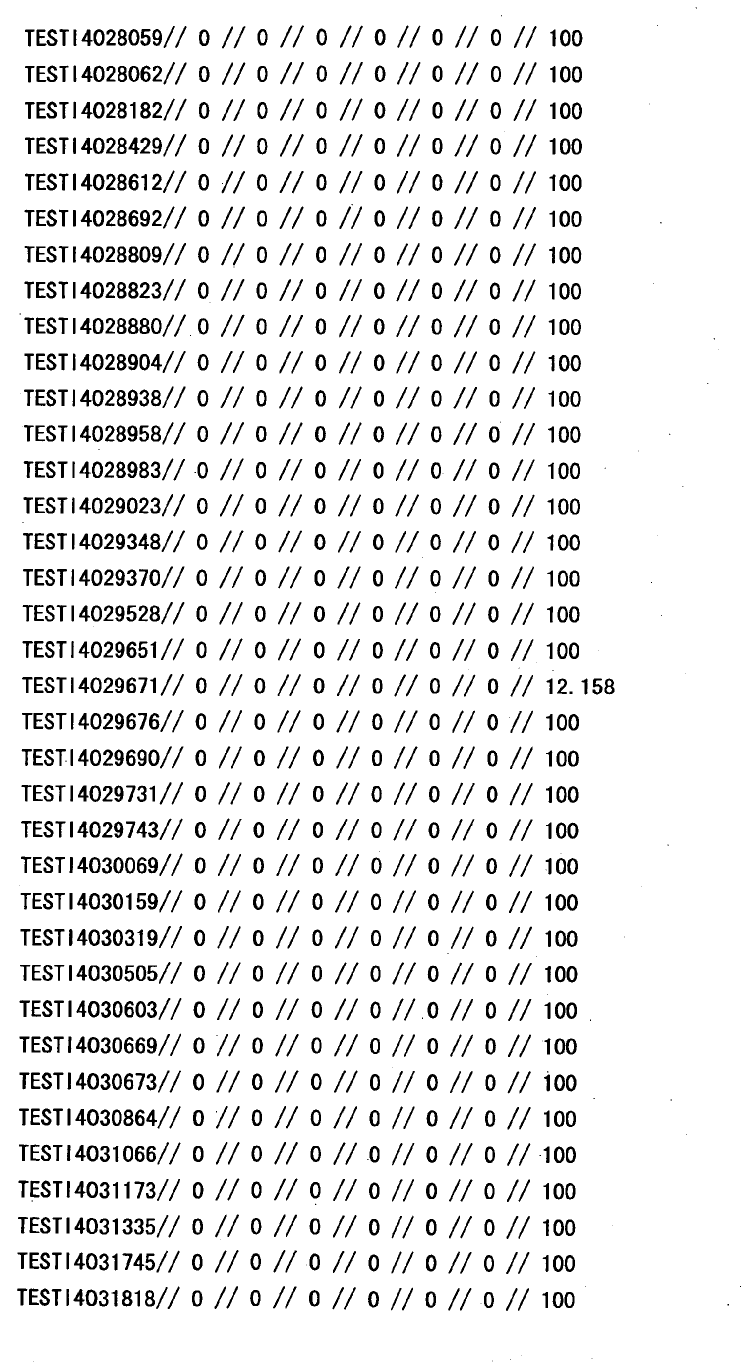

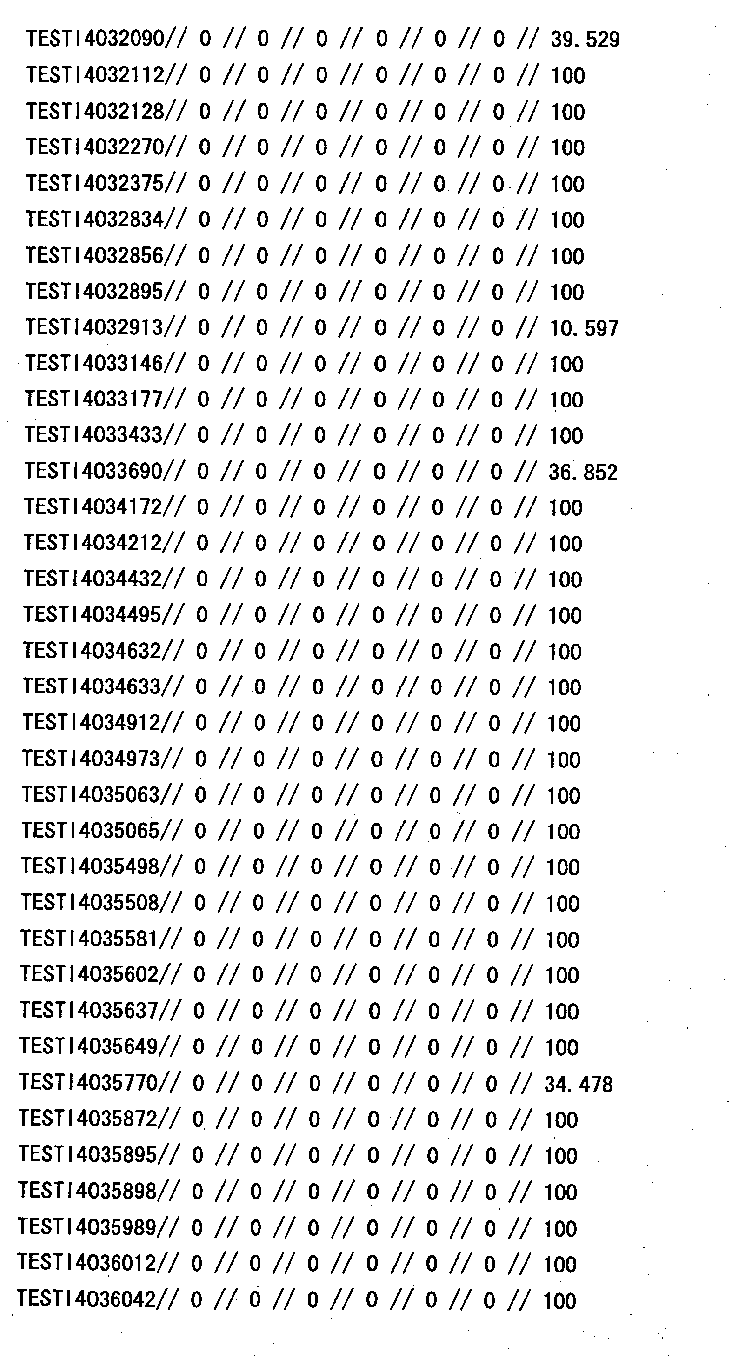

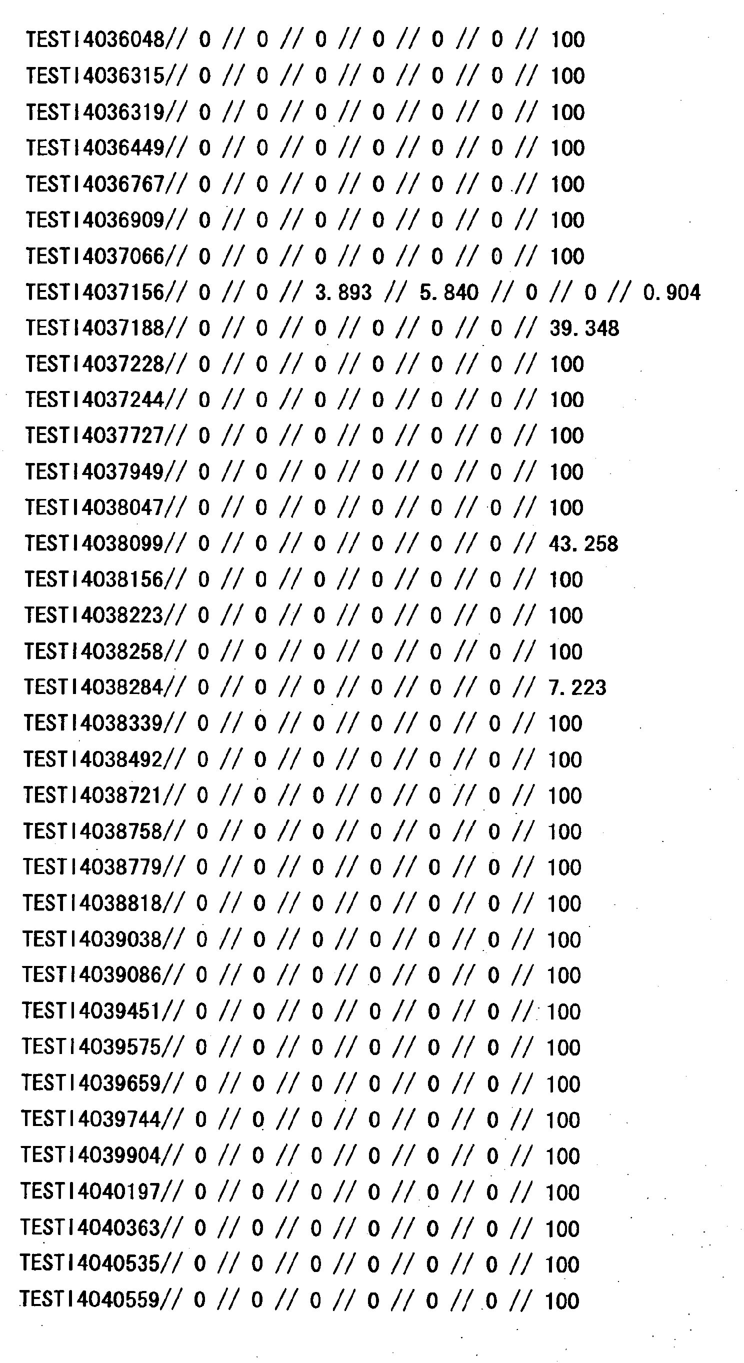

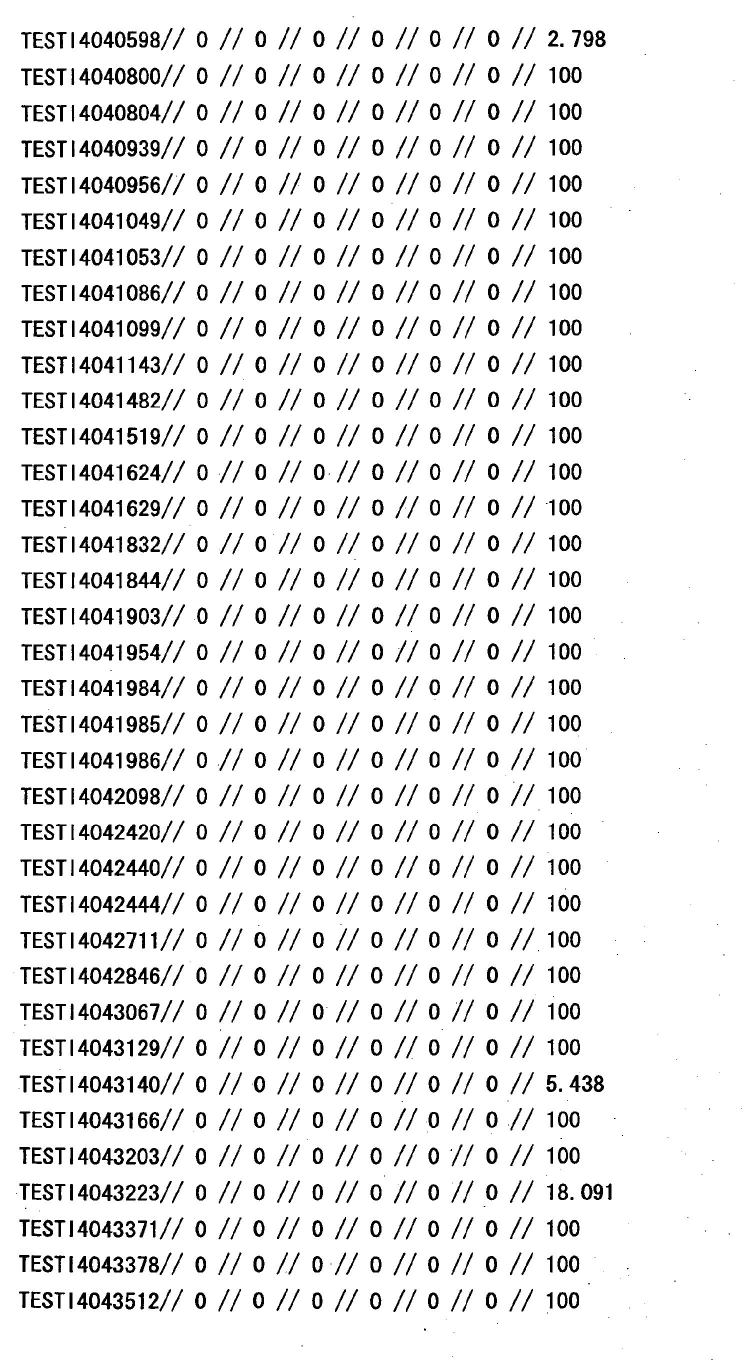

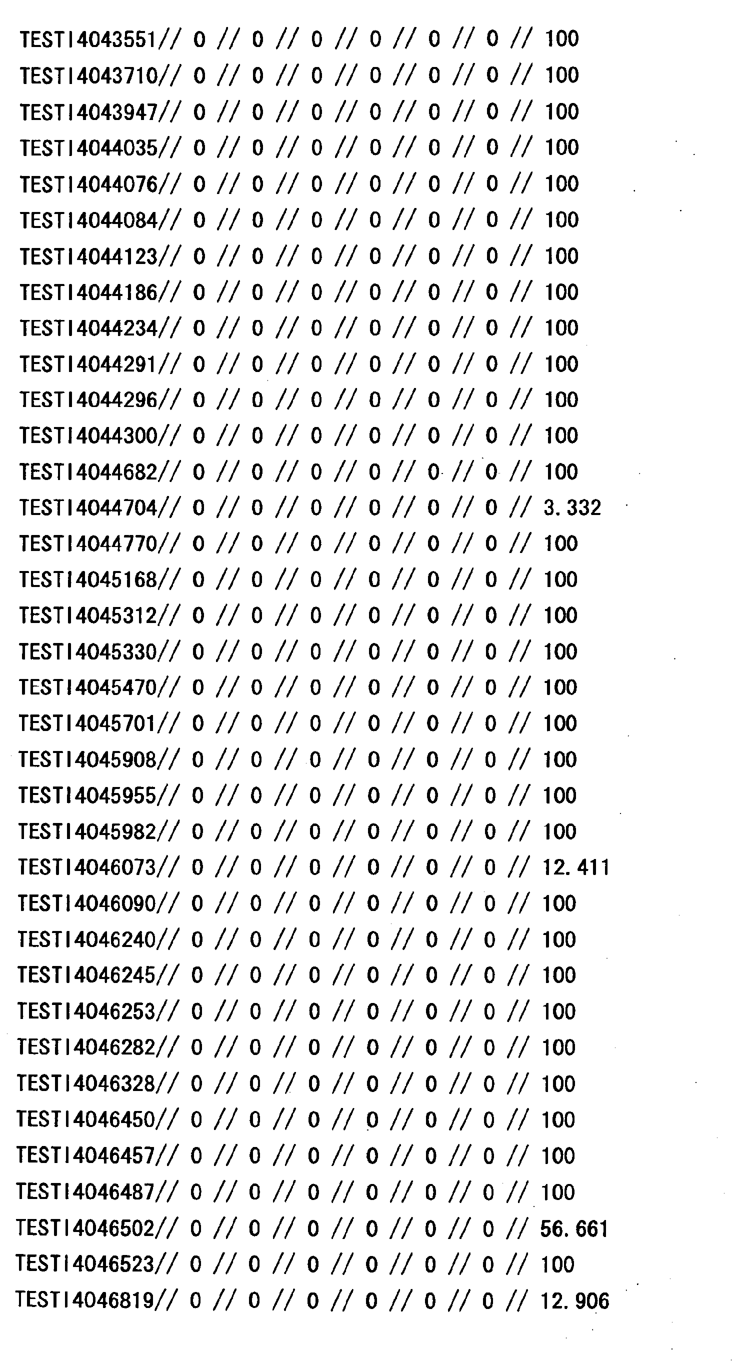

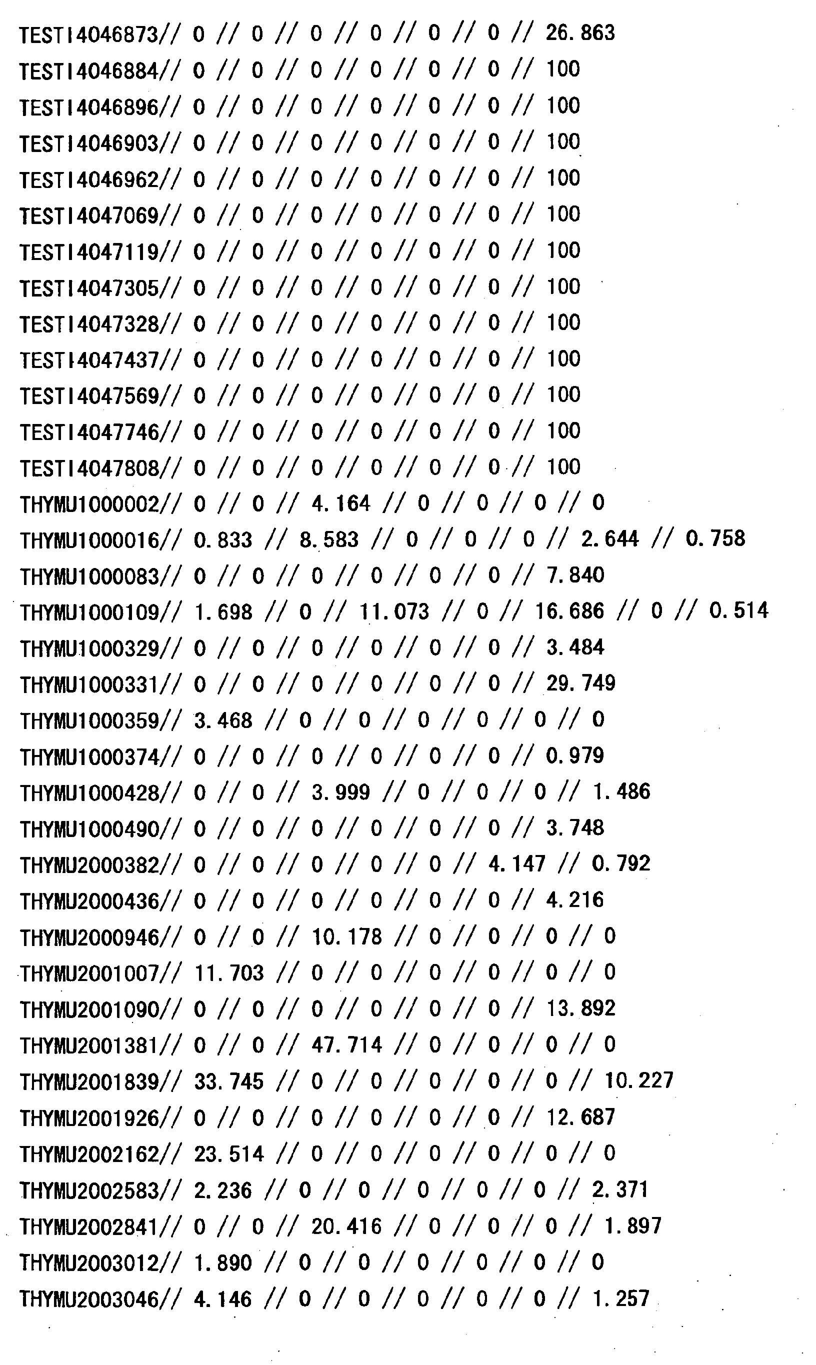

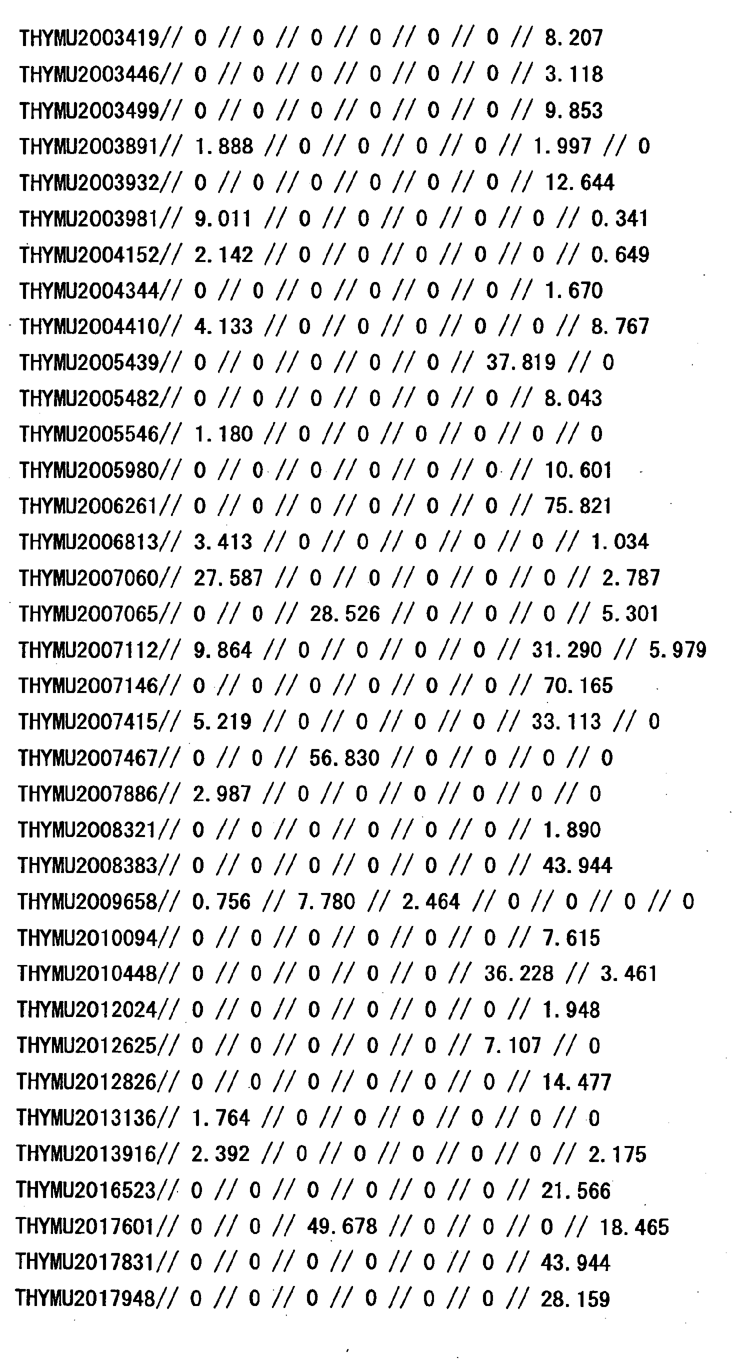

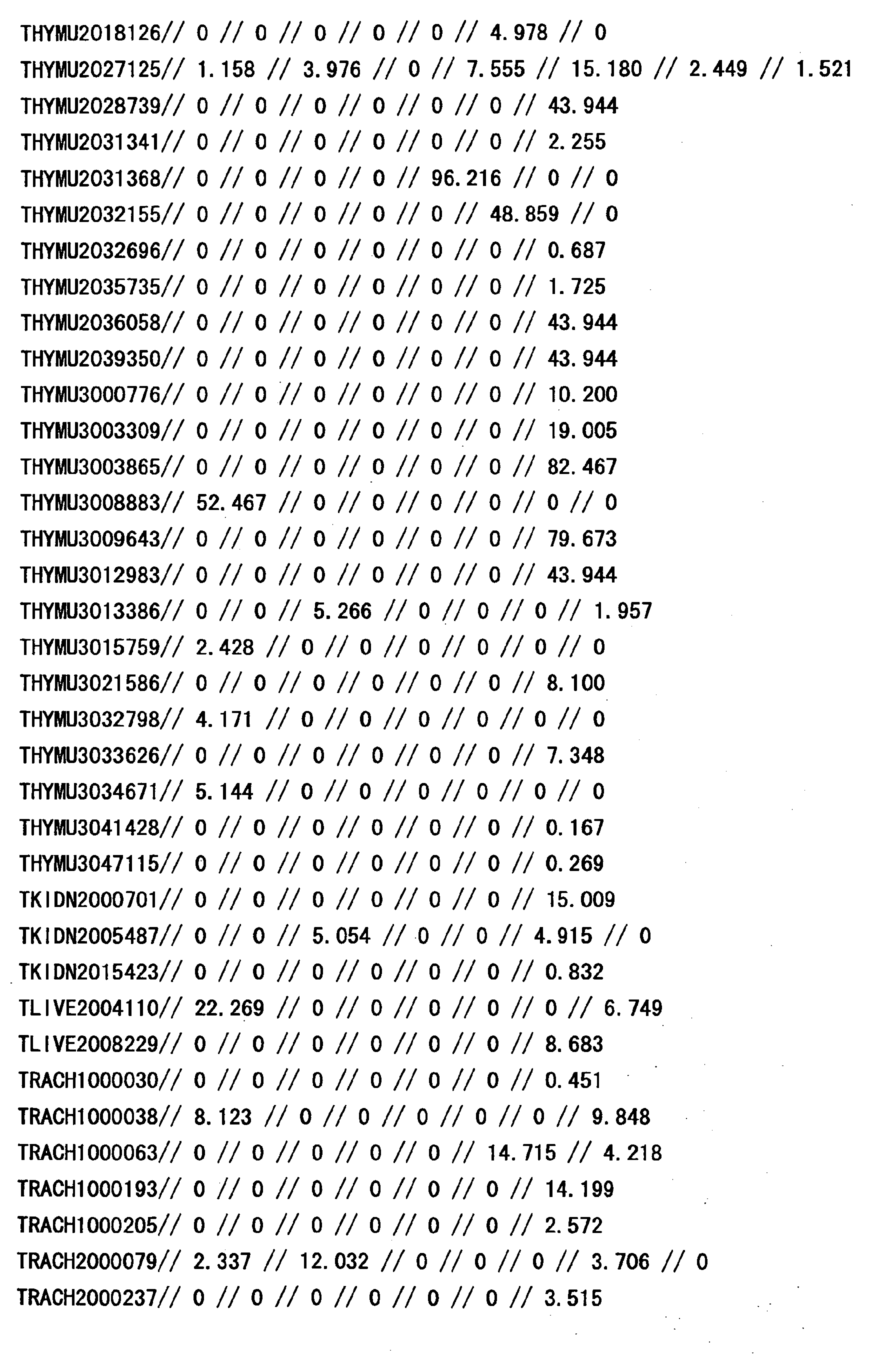

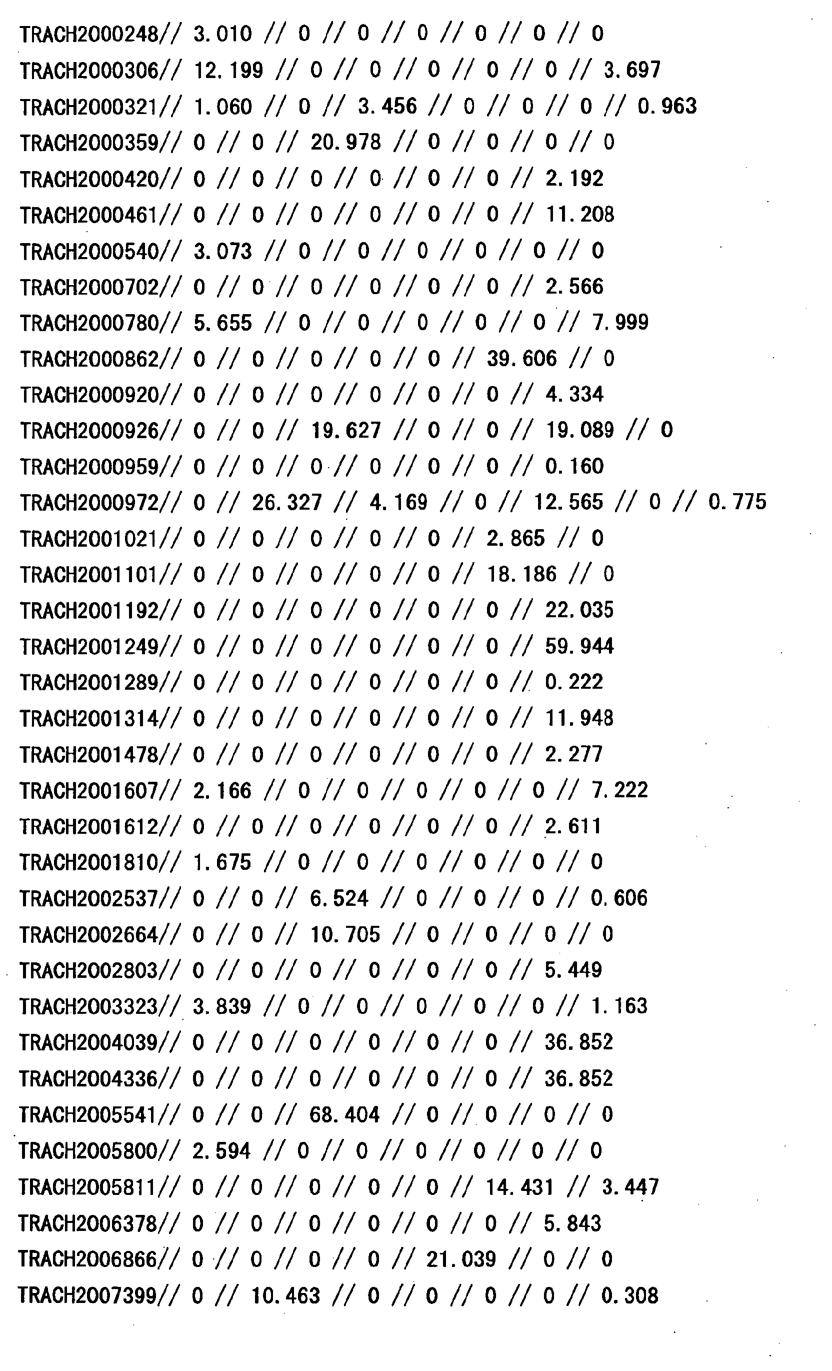

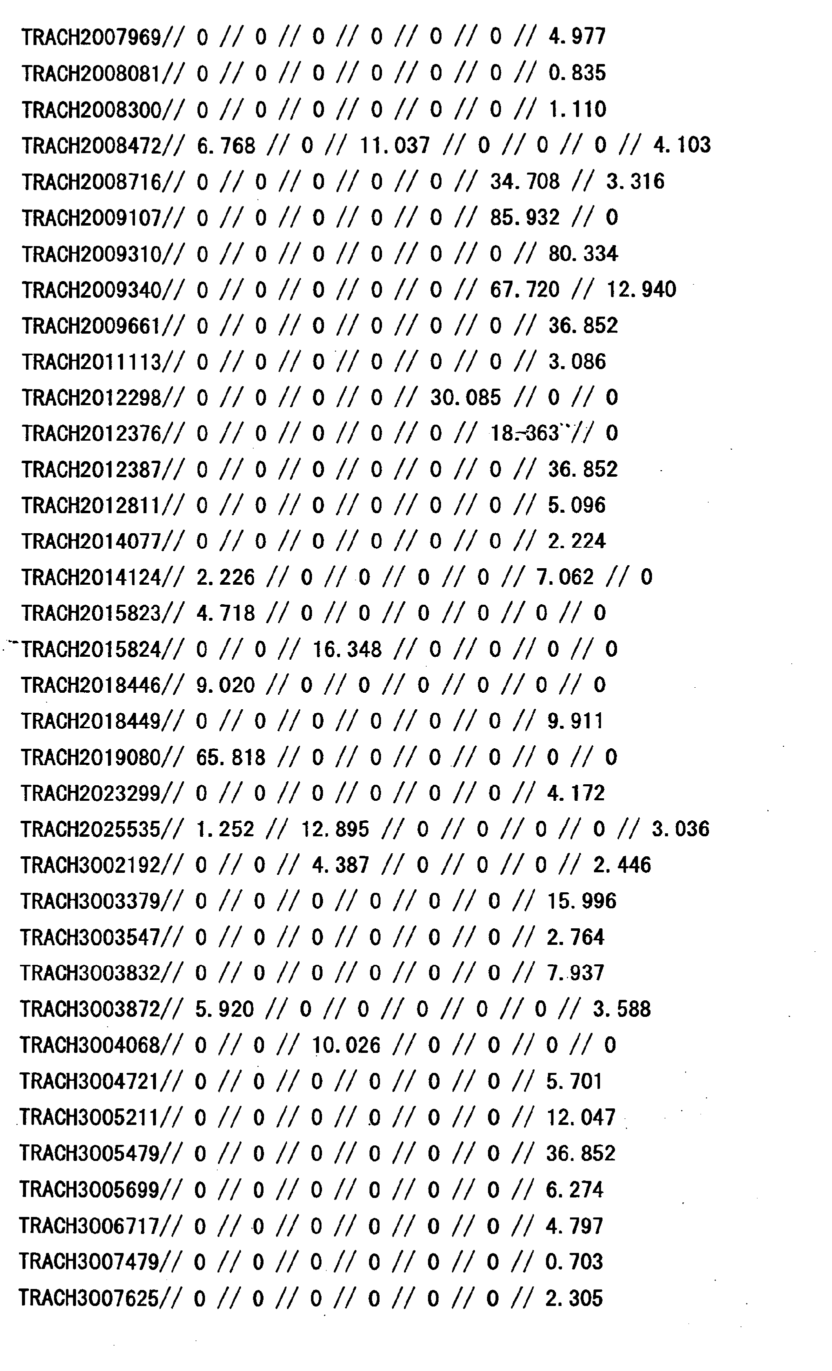

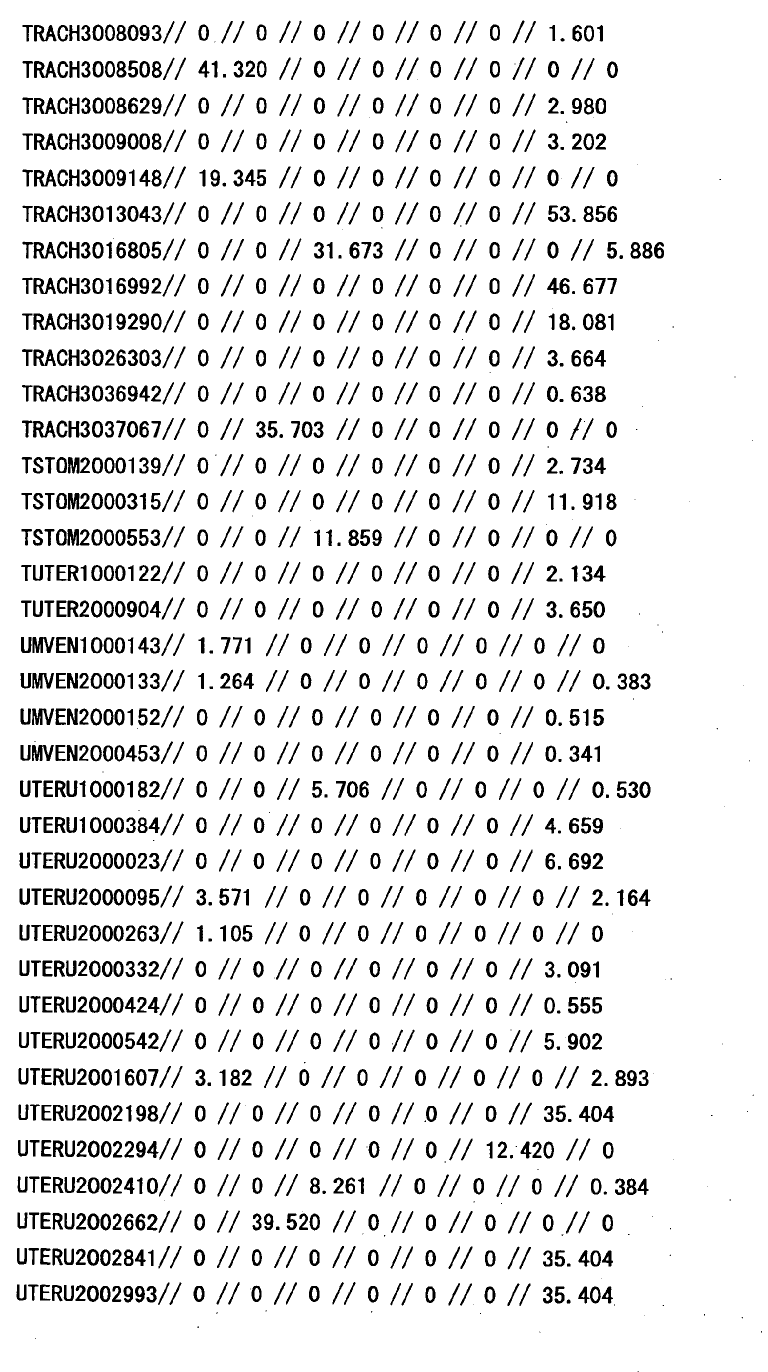

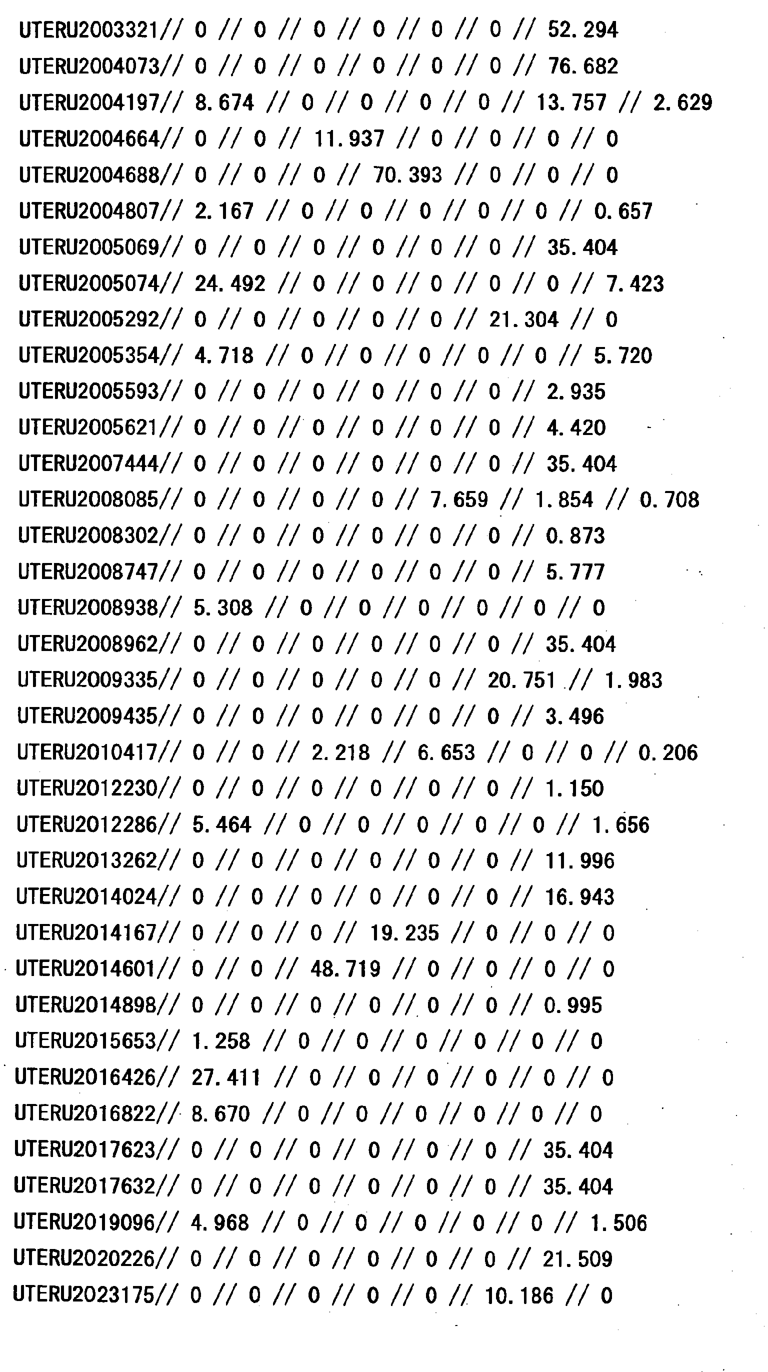

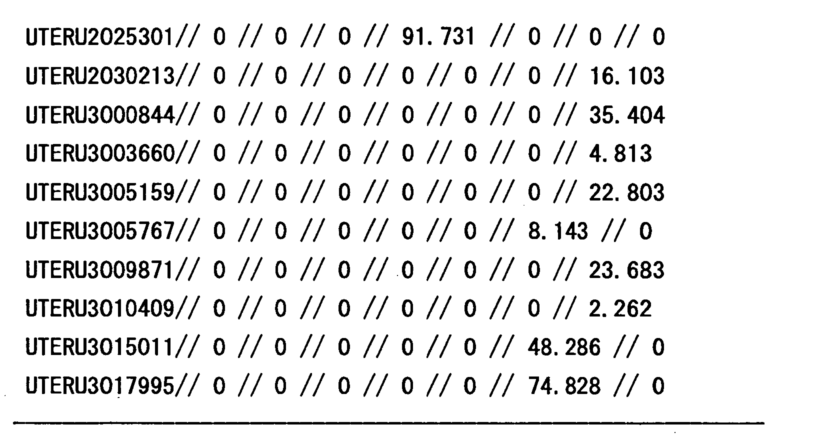

Genes whose expression levels were negligibly low in each of the above-described libraries are shown with their clone names below.
3NB691000018, 3NB691000085, 3NB691000113, 3NB691000129, 3NB691000191, 3NB692000012, 3NB692000029, 3NB692000102, 3NB692000154, 3NB692000276, 3NB692000305, 3NB692000429, 3NB692000484, 3NB692000973, 3NB692001002, 3NB692001022, 3NB692001034, 3NB692001040, 3NB692001123, 3NB692001267, 3NB692001288, 3NB692001339, 3NB692001349, 3NB692001408, 3NB692001433, 3NB692001459, 3NB692001471, 3NB692001501, 3NB692001507, 3NB692001519, 3NB692001520, 3NB692001528, 3NB692001538, 3NB692001548, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008729, ACTVT2000380, ADIPS1000064,
ADIPS2000069, ADIPS2000088, ADIPS2000245, ADIPS2000425, ADRGL1000002, ADRGL1000033, ADRGL1000038, ADRGL1000067, ADRGL1000111, ADRGL1000144, ADRGL1000147, ADRGL1000160, ADRGL1000165, ADRGL1000182, ADRGL2000056, ADRGL2000064, ADRGL2000074, ADRGL2000085, ADRGL2000142, ADRGL2000172, ADRGL2000248, ADRGL2000261, ADRGL2000323, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002477, ADRGL2002679, ADRGL2002753, ADRGL2002857, ADRGL2003017,
ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535,
ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO1000165, ASTRO2000011, ASTRO2000095, ASTRO2000191, ASTRO2000278, ASTRO2000372, ASTRO2000381, ASTRO2000403, ASTRO2000417, ASTRO2000480, ASTRO2000482, ASTRO2000533, ASTRO2000653, ASTRO2000662, ASTRO2000725, ASTRO2000801, ASTRO2000914, ASTRO2001001, ASTRO2001008, ASTRO2001088, ASTRO2001107, ASTRO2001122, ASTRO2001227, ASTRO2001249, ASTRO2001480, ASTRO2002035, ASTRO2002064, ASTRO2002202, ASTRO2002571,
ASTRO2002632, ASTRO2002659, ASTRO2002693, ASTRO2002733, ASTRO2002743, ASTRO2003461, ASTRO2003574, ASTRO2003581, ASTRO2003740, ASTRO2003925, ASTRO2003960, ASTRO2004032, ASTRO2004051, ASTRO2004177, ASTRO2004217, ASTRO2004297, ASTRO2004584, ASTRO2004751, ASTRO2004877, ASTRO2004974, ASTRO2005008, ASTRO2005081, ASTRO2005096, ASTRO2005242, ASTRO2005273, ASTRO2005343, ASTRO2005413, ASTRO2005423, ASTRO2005553, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007221, ASTRO2007377, ASTRO2007515, ASTRO2007578, ASTRO2007643, ASTRO2007666, ASTRO2007956, ASTRO2007962,
ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014863, ASTRO2014923, ASTRO2014961, ASTRO2015162, ASTRO2015185, ASTRO2015328, ASTRO2015987, ASTRO2016044, ASTRO2016148, ASTRO2016681,
ASTRO2016847, ASTRO2016892, ASTRO2017348, ASTRO2017627, ASTRO2018373, ASTRO2019039, ASTRO2019171, ASTRO3000154, ASTRO3000172, ASTRO3000177, ASTRO3000301, ASTRO3000474, ASTRO3000482, BEAST2000454, BGGI11000285, BGGI12000022, BGGI12000067, BGGI12000161, BGGI12000533, BGGI12000544, BGGI12000684, BGGI12000693, BGGI12001002, BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987,
BLADE2002073, BLADE2002310, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479,
BLADE2009593, BNGH41000098, BNGH41000104, BNGH41000118, BNGH41000137, BNGH41000153, BNGH41000169, BNGH41000177, BNGH41000216, BNGH42000130, BNGH42000132, BNGH42000405, BNGH42000532, BNGH42000675, BNGH42000791, BNGH42000815, BNGH42000994, BNGH42001724, BNGH42002168, BNGH42002244, BNGH42002487, BNGH42002489, BNGH42003529, BNGH42003570, BNGH42003641, BNGH42004076, BNGH42004291, BNGH42004538, BNGH42005017, BNGH42005235, BNGH42005504, BNGH42006226, BNGH42006234, BNGH42007366, BNGH42007594, BNGH42007788, BNGH42008199, BNGH42008510, BNGH42008603, BNGH42008649, BNGH42008832, BNGH42008850, BNGH42008943, BNGH42009021, BRACE1000020, BRACE1000042, BRACE1000047, BRACE1000051, BRACE1000065, BRACE1000070,
BRACE1000084, BRACE1000087, BRACE1000092, BRACE1000093, BRACE1000101, BRACE1000114, BRACE1000124, BRACE1000132, BRACE1000166, BRACE1000169, BRACE1000186, BRACE1000187, BRACE1000239, BRACE1000258, BRACE1000451, BRACE1000475, BRACE1000572, BRACE2000061, BRACE2000078, BRACE2000100, BRACE2000109, BRACE2000141, BRACE2000148, BRACE2000232, BRACE2000245, BRACE2000256, BRACE2000266, BRACE2000267, BRACE2000280, BRACE2000300, BRACE2000307, BRACE2000331, BRACE2000332, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000640, BRACE2000650, BRACE2000677, BRACE2000698,
BRACE2000718, BRACE2000733, BRACE2000743, BRACE2000750, BRACE2000753, BRACE2000815, BRACE2000864, BRACE2000905, BRACE2000936, BRACE2000947, BRACE2000965, BRACE2000969, BRACE2000988, BRACE2001017, BRACE2001065, BRACE2001070, BRACE2001094, BRACE2001151, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001492, BRACE2001496, BRACE2001508, BRACE2001534, BRACE2001543, BRACE2001588, BRACE2001607, BRACE2001626, BRACE2001673, BRACE2001677, BRACE2001705, BRACE2001709, BRACE2001726,
BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001898, BRACE2001923, BRACE2001942, BRACE2001944, BRACE2001954, BRACE2001971, BRACE2001972, BRACE2001973, BRACE2002026, BRACE2002034, BRACE2002081, BRACE2002139, BRACE2002142, BRACE2002151, BRACE2002157, BRACE2002176, BRACE2002206, BRACE2002227, BRACE2002260, BRACE2002297, BRACE2002304, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623,
BRACE2002635, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002812, BRACE2002834, BRACE2002849, BRACE2002857, BRACE2002860, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003254, BRACE2003269, BRACE2003285, BRACE2003304, BRACE2003319, BRACE2003398, BRACE2003420, BRACE2003428, BRACE2003431, BRACE2003512, BRACE2003516, BRACE2003539, BRACE2003540, BRACE2003594, BRACE2003598, BRACE2003609, BRACE2003614, BRACE2003639, BRACE2003700, BRACE2003713,
BRACE2003766, BRACE2003785, BRACE2003788, BRACE2003800, BRACE2003802, BRACE2003825, BRACE2003845, BRACE2003847, BRACE2003848, BRACE2003873, BRACE2003885, BRACE2003887, BRACE2003905, BRACE2003928, BRACE2003944, BRACE2003954, BRACE2003982, BRACE2003987, BRACE2005034, BRACE2005047, BRACE2005063, BRACE2005083, BRACE2005099, BRACE2005118, BRACE2005124, BRACE2005138, BRACE2005160, BRACE2005169, BRACE2005189, BRACE2005193, BRACE2005196, BRACE2005198, BRACE2005216, BRACE2005243, BRACE2005251, BRACE2005253, BRACE2005290, BRACE2005298, BRACE2005328, BRACE2005329, BRACE2005337, BRACE-2005348, BRACE2005363, BRACE2005395, BRACE2005408, BRACE2005448, BRACE2005450, BRACE2005453, BRACE2005457, BRACE2005460,
BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006075, BRACE2006084, BRACE2006089, BRACE2006102, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281, BRACE2006298, BRACE2006344,
BRACE2006354, BRACE2006363, BRACE2006378, BRACE2006380, BRACE2006393, BRACE2006413, BRACE2006417, BRACE2006453, BRACE2006459, BRACE2006488, BRACE2006534, BRACE2006537, BRACE2006540, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006871, BRACE2006909, BRACE2006911, BRACE2006924, BRACE2006935, BRACE2006937, BRACE2006944, BRACE2006951, BRACE2006982, BRACE2006986, BRACE2007013, BRACE2007068, BRACE2007087, BRACE2007138, BRACE2007174, BRACE2007197, BRACE2007201, BRACE2007203, BRACE2007232, BRACE2007254, BRACE2007262, BRACE2007274, BRACE2007281, BRACE2007295, BRACE2007358, BRACE2007396,
BRACE2007398, BRACE2007411, BRACE2007447, BRACE2007477, BRACE2007502, BRACE2007503, BRACE2007522, BRACE2007527, BRACE2007538, BRACE2007563, BRACE2007577, BRACE2007621, BRACE2007640, BRACE2007641, BRACE2007646, BRACE2007663, BRACE2007671, BRACE2007682, BRACE2007685, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008216,
BRACE2008218, BRACE2008251, BRACE2008276, BRACE2008295, BRACE2008308, BRACE2008336, BRACE2008361, BRACE2008380, BRACE2008401, BRACE2008410, BRACE2008420, BRACE2008443, BRACE2008473, BRACE2008480, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008999, BRACE2009014, BRACE2009016, BRACE2009031, BRACE2009053, BRACE2009122, BRACE2009131, BRACE2009185, BRACE2009188, BRACE2009212,
BRACE2009221, BRACE2009235, BRACE2009238, BRACE2009255, BRACE2009270, BRACE2009272, BRACE2009274, BRACE2009275, BRACE2009293, BRACE2009311, BRACE2009318, BRACE2009323, BRACE2009366, BRACE2009421, BRACE2009428, BRACE2009434, BRACE2009437, BRACE2009483, BRACE2009533, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010684, BRACE2010753, BRACE2010813, BRACE2010828,
BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268,
BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017580, BRACE2017587, BRACE2017720, BRACE2017844, BRACE2017872, BRACE2017929, BRACE2017934,
BRACE2017985, BRACE2017992, BRACE2018337, BRACE2018443, BRACE2018448, BRACE2018568, BRACE2018700, BRACE2018727, BRACE2018759, BRACE2018762, BRACE2018782, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021148, BRACE2021245, BRACE2021254, BRACE2021264, BRACE2021344, BRACE2021433,
BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023744, BRACE2023800, BRACE2023801, BRACE2024074, BRACE2024222, BRACE2024610, BRACE2024781, BRACE2024826, BRACE2025018, BRACE2025316, BRACE2025333, BRACE2025452, BRACE2025492, BRACE2025710, BRACE2025796, BRACE2025992, BRACE2026054, BRACE2026141,
BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030345, BRACE2030736, BRACE2030883, BRACE2030884, BRACE2031154, BRACE2031232, BRACE2031527, BRACE2031531, BRACE2031553, BRACE2031899, BRACE2032044,
BRACE2032090, BRACE2032134, BRACE2032148, BRACE2032182, BRACE2032329, BRACE2032385, BRACE2032436, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038271, BRACE2038329, BRACE2038492, BRACE2038551, BRACE2038618, BRACE2039009,
BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043248, BRACE2043349, BRACE2043774, BRACE2043942, BRACE2044057, BRACE2044263, BRACE2044286, BRACE2044304, BRACE2044473, BRACE2044510, BRACE2044640, BRACE2044816, BRACE2044946, BRACE2044949, BRACE2045300, BRACE2045428,
BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413,
BRACE3003595, BRACE3003698, BRACE3003866, BRACE3003920, BRACE3004046, BRACE3004058, BRACE3004113, BRACE3004358, BRACE3004371, BRACE3004435, BRACE3004477, BRACE3004767, BRACE3004772, BRACE3004783, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005760, BRACE3005870, BRACE3005903, BRACE3005989, BRACE3006113, BRACE3006185, BRACE3006226, BRACE3006462, BRACE3006463, BRACE3006872, BRACE3006917, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036,
BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009265, BRACE3009297,. BRACE3009377, BRACE3009392, BRACE3009539, BRACE3009574, BRACE3009701, BRACE3009708, BRACE3009724, BRACE3009993, BRACE3010076, BRACE3010079, BRACE30100.86, BRACE3010221, BRACE3010283, BRACE3010315, BRACE3010397, BRACE3010416, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780,
BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3020151, BRACE3020194, BRACE3020286, BRACE3020356, BRACE3020594, BRACE3020669, BRACE3020671, BRACE3020890, BRACE3021148, BRACE3021517,
BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028998, BRACE3029005, BRACE3029021, BRACE3029205, BRACE3029447,
BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856,
BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174,
BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009482, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951,
BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000388, BRAMY2000411, BRAMY2000508, BRAMY2000562, BRAMY2000653, BRAMY2000814, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339,
BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004521, BRAMY2004524, BRAMY2004537, BRAMY2004542, BRAMY2004756, BRAMY2004771, BRAMY2005064, BRAMY2005094, BRAMY2005151, BRAMY2005182, BRAMY2005244, BRAMY2005391,
BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007244, BRAMY2007249, BRAMY2007255, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008979, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508,
BRAMY2009528, BRAMY2009557, BRAMY2009612, BRAMY2009693, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011846, BRAMY2011849, BRAMY2011872, BRAMY2011961, BRAMY2012017, BRAMY2012091, BRAMY2012119, BRAMY2012162, BRAMY2012277, BRAMY2012340, BRAMY2012497, BRAMY2012517, BRAMY2012555, BRAMY2012568, BRAMY2012581, BRAMY2012691, BRAMY2012721,
BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015855, BRAMY2015890, BRAMY2015911, BRAMY2015925, BRAMY2016002, BRAMY2016009, BRAMY2016020, BRAMY2016070, BRAMY2016251, BRAMY2016327, BRAMY2016386, BRAMY2016706, BRAMY2016771,
BRAMY2016892, BRAMY2016938, BRAMY2016953, BRAMY2017014, BRAMY2017249, BRAMY2017348, BRAMY2017450, BRAMY2017455, BRAMY2017528, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018785, BRAMY2019055, BRAMY2019111, BRAMY2019277, BRAMY2019300, BRAMY2019509, BRAMY2019600, BRAMY2019710, BRAMY2019963, BRAMY2019985, BRAMY2019989, BRAMY2020050, BRAMY2020058, BRAMY2020106, BRAMY2020270, BRAMY2020355, BRAMY2020412, BRAMY2020445, BRAMY2020466, BRAMY2020520, BRAMY2020574, BRAMY2020634, BRAMY2021040, BRAMY2021063, BRAMY2021098, BRAMY2021139, BRAMY2021276, BRAMY2021310, BRAMY2021388, BRAMY2021498,
BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2024004, BRAMY2024073, BRAMY2024312, BRAMY2024374, BRAMY2024390, BRAMY2024416, BRAMY2024449, BRAMY2024489,
BRAMY2024497, BRAMY2024514, BRAMY2024530, BRAMY2024535, BRAMY2024545, BRAMY2024576, BRAMY2024593, BRAMY2024711, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739,
BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028856, BRAMY2028914, BRAMY2029204, BRAMY2029602, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332,
BRAMY2033506, BRAMY2033594, BRAMY2033895, BRAMY2033914, BRAMY2034072, BRAMY2034185, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035923, BRAMY2036076, BRAMY2036079, BRAMY2036254, BRAMY2036266, BRAMY2036387, BRAMY2036396, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2039287, BRAMY2039300, BRAMY2039314, BRAMY2039341, BRAMY2039630,
BRAMY2039695, BRAMY2039728, BRAMY2039872, BRAMY2040051, BRAMY2040159, BRAMY2040214, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871,
BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002508, BRAMY3002620, BRAMY3002803, BRAMY3002805, BRAMY3002886, BRAMY3002991, BRAMY3003026, BRAMY3003109, BRAMY3003310, BRAMY3003566, BRAMY3003723, BRAMY3004126, BRAMY3004224, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761,
BRAMY3007078, BRAMY3007206, BRAMY3007311, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009556, BRAMY3009782, BRAMY3009811, BRAMY3009904, BRAMY3010008, BRAMY3010264, BRAMY3010321, BRAMY3010411, BRAMY3010492, BRAMY3010603, BRAMY3010654, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000045, BRAWH1000094,
BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000471, BRAWH2000476, BRAWH2000482, BRAWH2000488, BRAWH2000494, BRAWH2000500, BRAWH2000503, BRAWH2000522, BRAWH2000554, BRAWH2000588, BRAWH2000595, BRAWH2000633, BRAWH2000643, BRAWH2000651, BRAWH2000682, BRAWH2000752, BRAWH2000859, BRAWH2000866, BRAWH2000892,
BRAWH2000901, BRAWH2000926, BRAWH2000984, BRAWH2001019, BRAWH2001037, BRAWH2001052, BRAWH2001103, BRAWH2001129, BRAWH2001137, BRAWH2001141, BRAWH2001159, BRAWH2001186, BRAWH2001203, BRAWH2001238, BRAWH2001239, BRAWH2001255, BRAWH2001355, BRAWH2001378, BRAWH2001395, BRAWH2001412, BRAWH2001418, BRAWH2001424, BRAWH2001436, BRAWH2001438, BRAWH2001459, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2002047, BRAWH2002060, BRAWH2002062, BRAWH2002125, BRAWH2002191, BRAWH2002318, BRAWH2002333, BRAWH2002352, BRAWH2002383,
BRAWH2002549, BRAWH2002560, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003135, BRAWH2003240, BRAWH2003307, BRAWH2003355, BRAWH2003366, BRAWH2003662, BRAWH2003689, BRAWH2003693, BRAWH2003832, BRAWH2003948, BRAWH2003964, BRAWH2004044, BRAWH2004068, BRAWH2004078, BRAWH2004217, BRAWH2004580, BRAWH2004731, BRAWH2004779, BRAWH2004842, BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006083, BRAWH2006207,
BRAWH2006301, BRAWH2006526, BRAWH2006543, BRAWH2006622, BRAWH2006760, BRAWH2006960, BRAWH2006989, BRAWH2007069, BRAWH2007308, BRAWH2007406, BRAWH2007550, BRAWH2007570, BRAWH2007591, BRAWH2007658, BRAWH2007664, BRAWH2007800, BRAWH2007808, BRAWH2007825, BRAWH2007862, BRAWH2007890, BRAWH2008058, BRAWH2008128, BRAWH2008255, BRAWH2008292, BRAWH2008297, BRAWH2008380, BRAWH2008540, BRAWH2008577, BRAWH2008790, BRAWH2008903, BRAWH2008956, BRAWH2008993, BRAWH2009112, BRAWH2009227, BRAWH2009261, BRAWH2009263, BRAWH2009298, BRAWH2009304, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069,
BRAWH2010084, BRAWH2010136, BRAWH2010318, BRAWH2010329, BRAWH2010354, BRAWH2010536, BRAWH2010552, BRAWH2010584, BRAWH2010618, BRAWH2010619, BRAWH2010754, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871,
BRAWH2013914, BRAWH2013941, BRAWH2013955, BRAWH2014234, BRAWH2014379, BRAWH2014414, BRAWH2014473, BRAWH2014511, BRAWH2014662, BRAWH2014717, BRAWH2014729, BRAWH2014876, BRAWH2014934, BRAWH2014954, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016439, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542,
BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244, BRAWH3003343, BRAWH3003522, BRAWH3003598,
BRAWH3003727, BRAWH3003801, BRAWH3003975, BRAWH3004222, BRAWH3004335, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331,
BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431,
BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3026349, BRAWH3026394, BRAWH3026529, BRAWH3027420, BRAWH3027440, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571,
BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639,
BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007426, BRCAN2007525, BRCAN2007700, BRCAN2008494, BRCAN2008528, BRCAN2008701,
BRCAN2008894, BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566,
BRCAN2018667, BRCAN2018748, BRCAN2018935, BRCAN2019002, BRCAN2019387, BRCAN2019653, BRCAN2019773, BRCAN2019907, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027513, BRCAN2027593, BRCAN2027970, BRCAN2028021, BRCAN2028036, BRCAN2028319, BRCAN2028338, BRCAN2028355, BRCAN2028378, BRCAN2028460,
BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005951, BRCOC2006164, BRCOC2006243, BRCOC2006270, BRCOC2006639, BRCOC2006942, BRCOC2007034, BRCOC2007593, BRCOC2007733, BRCOC2007769,
BRCOC2008064, BRCOC2008225, BRCOC2008642, BRCOC2008993, BRCOC2009052, BRCOC2009196, BRCOC2009380, BRCOC2009638, BRCOC2010115, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934,
BRCOC2020122, BRCOC2020142, BRHIP1000058, BRHIP1000072, BRHIP1000108, BRHIP1000129, BRHIP1000258, BRHIP2000021, BRHIP2000079, BRHIP2000087, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000819, BRHIP2000826, BRHIP2000920, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2004312, BRHIP2004355, BRHIP2004359, BRHIP2004814,
BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011508, BRHIP2011576, BRHIP2011616,
BRHIP2011783, BRHIP2011838, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013972, BRHIP2014063, BRHIP2014285, BRHIP2014291, BRHIP2014373, BRHIP2014386, BRHIP2014387, BRHIP2014954, BRHIP2015224, BRHIP2015245, BRHIP2015356, BRHIP2015360, BRHIP2016005, BRHIP2016125, BRHIP2016788, BRHIP2016968, BRHIP2016990, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612,
BRHIP2018650, BRHIP2018719, BRHIP2018805, BRHIP2018998, BRHIP2019007, BRHIP2019047, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021929, BRHIP2022006, BRHIP2022054, BRHIP2022221, BRHIP2022228, BRHIP2022298, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023773, BRHIP2023860,
BRHIP2024046, BRHIP2024165, BRHIP2024201, BRHIP2024347, BRHIP2024398, BRHIP2024549, BRHIP2024789, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000131, BRHIP3000240, BRHIP3000377, BRHIP3000457, BRHIP3000475, BRHIP3000488,
BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003340, BRHIP3003395, BRHIP3003484, BRHIP3003644, BRHIP3003688, BRHIP3003795, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005137, BRHIP3005231, BRHIP3005307, BRHIP3005574, BRHIP3005673, BRHIP3005768, BRHIP3005801, BRHIP3005944, BRHIP3006279, BRHIP3006390, BRHIP3006449, BRHIP3006683, BRHIP3006717,
BRHIP3006786, BRHIP3006950, BRHIP3007172, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013698,
BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160,
BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000518, BRSSN2000561, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004679, BRSSN2004686, BRSSN2004710, BRSSN2004719, BRSSN2005847, BRSSN2006133, BRSSN2006390,
BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013733, BRSSN2013753, BRSSN2013874, BRSSN2014112, BRSSN2014294, BRSSN2014424, BRSSN2014513,
BRSSN2014556, BRSSN2014573, BRSSN2014636, BRSSN2014685, BRSSN2015199, BRSSN2015369, BRSSN2015493, BRSSN2015554, BRSSN2015707, BRSSN2015773, BRSSN2015907, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583,
BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013171, BRSTN2013354, BRSTN2013931, BRSTN2014490, BRSTN2014633, BRSTN2014751, BRSTN2014770, BRSTN2015015, BRSTN2015978, BRSTN2016066, BRSTN2016335, BRSTN2016892, BRSTN2016918, BRSTN2016992, BRSTN2017151, BRSTN2017237, BRSTN2017379, BRSTN2017761, BRSTN2017771,
BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639,
BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194, BRTHA2006232, BRTHA2006720, BRTHA2006735, BRTHA2006974, BRTHA2007060, BRTHA2007122, BRTHA2007422, BRTHA2007603, BRTHA2007665, BRTHA2007998, BRTHA2008316, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884,
BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012980, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543,
BRTHA2016552, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229,
BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003225, BRTHA3003339, BRTHA3003343,
BRTHA3003417, BRTHA3003449, BRTHA3003474, BRTHA3003704, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008826, BRTHA3009037, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011229, BRTHA3011265, BRTHA3011510, BRTHA3011892, BRTHA3012265, BRTHA3013860, BRTHA3013882,
BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026556, BRTHA3026916, BRTHA3027171, BRTHA3027318, BRTHA3027638,
BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282,
COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000056, CTONG1000062, CTONG1000086, CTONG1000088, CTONG1000094, CTONG1000097, CTONG1000113, CTONG1000137, CTONG1000165, CTONG1000175, CTONG1000180, CTONG1000181, CTONG1000220, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000218, CTONG2000279, CTONG2000303, CTONG2000330, CTONG2000395, CTONG2000411, CTONG2000465, CTONG2000480, CTONG2000561,
CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001413, CTONG2001428, CTONG2001513, CTONG2001524, CTONG2001533, CTONG2001598, CTONG2001681, CTONG2001724, CTONG2001749, CTONG2001820, CTONG2001877, CTONG2001911, CTONG2001932, CTONG2001955, CTONG2002066, CTONG2002395, CTONG2002417, CTONG2002418, CTONG2002453, CTONG2002520, CTONG2002558, CTONG2002590, CTONG2002709, CTONG2002721, CTONG2002816, CTONG2002820, CTONG2002903, CTONG2002965, CTONG2002999, CTONG2003028, CTONG2003094, CTONG2003100, CTONG2003115, CTONG2003245, CTONG2003348, CTONG2003350, CTONG2003372, CTONG2003375, CTONG2003427,
CTONG2003599, CTONG2003680, CTONG2003699, CTONG2003764, CTONG2003782, CTONG2003889, CTONG2003937, CTONG2004000, CTONG2004062, CTONG2004115, CTONG2004126, CTONG2004264, CTONG2004305, CTONG2004397, CTONG2004423, CTONG2004454, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005290, CTONG2005374, CTONG2005399, CTONG2005468, CTONG2005567, CTONG2005585, CTONG2005635, CTONG2005775, CTONG2005795, CTONG2005890, CTONG2005904, CTONG2006129, CTONG2006223, CTONG2006273, CTONG2006310, CTONG2006393, CTONG2006449, CTONG2006515, CTONG2006524, CTONG2006568, CTONG2006611, CTONG2006620, CTONG2006666,
CTONG2006691, CTONG2006709, CTONG2006836, CTONG2006942, CTONG2006964, CTONG2007009, CTONG2007091, CTONG2007168, CTONG2007175, CTONG2007259, CTONG2007272, CTONG2007293, CTONG2007297, CTONG2007400, CTONG2007417, CTONG2007441, CTONG2007623, CTONG2007681, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395,
CTONG2009423, CTONG2009440, CTONG2009527, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2010018, CTONG2010024, CTONG2010116, CTONG2010133, CTONG2010148, CTONG2010276, CTONG2010330, CTONG2010348, CTONG2010408, CTONG2010508, CTONG2010566, CTONG2010623, CTONG2010649, CTONG2010803, CTONG2011188, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012029, CTONG2012077,
CTONG2012123, CTONG2012158, CTONG2012230, CTONG2012401, CTONG2012422, CTONG2012425, CTONG2012447, CTONG2012453, CTONG2012473, CTONG2012476, CTONG2012533, CTONG2012554, CTONG2012564, CTONG2012661, CTONG2012724, CTONG2012738, CTONG2012758, CTONG2012843, CTONG2012879, CTONG2012901, CTONG2012996, CTONG2013128, CTONG2013149, CTONG2013156, CTONG2013284, CTONG2013339, CTONG2013352, CTONG2013381, CTONG2013393, CTONG2013511, CTONG2013630, CTONG2013803, CTONG2013907, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014389, CTONG2014491, CTONG2014630, CTONG2014635, CTONG2014646, CTONG2014705,
CTONG2014946, CTONG2014954, CTONG2014959, CTONG2014966, CTONG2014995, CTONG2015204, CTONG2015316, CTONG2015345, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016056, CTONG2016217, CTONG2016355, CTONG2016499, CTONG2016505, CTONG2016559, CTONG2016658, CTONG2016775, CTONG2016797, CTONG2016824, CTONG2016846, CTONG2016904, CTONG2016953, CTONG2017021, CTONG2017292, CTONG2017409, CTONG2017444, CTONG2017458, CTONG2017604, CTONG2017650, CTONG2017804, CTONG2017814, CTONG2017910, CTONG2017939, CTONG2017989, CTONG2018062, CTONG2018069, CTONG2018147, CTONG2018211, CTONG2018217,
CTONG2018279, CTONG2018334, CTONG2018343, CTONG2018379, CTONG2018413, CTONG2018483, CTONG2018629, CTONG2018653, CTONG2018655, CTONG2018735, CTONG2018900, CTONG2018976, CTONG2019029, CTONG2019063, CTONG2019144, CTONG2019232, CTONG2019248, CTONG2019590, CTONG2019652, CTONG2019704, CTONG2019822, CTONG2019833, CTONG2020031, CTONG2020108, CTONG2020127, CTONG2020171, CTONG2020374, CTONG2020378, CTONG2020411, CTONG2020445, CTONG2020806, CTONG2020831, CTONG2021024, CTONG2021132, CTONG2021168, CTONG2022153, CTONG2022601, CTONG2022835, CTONG2023021, CTONG2023512, CTONG2024031, CTONG2024206, CTONG2024614, CTONG2024749, CTONG2025496, CTONG2025513, CTONG2025516, CTONG2025610, CTONG2025900, CTONG2026513,
CTONG2026770, CTONG2026920, CTONG2026987, CTONG2027150, CTONG2027327, CTONG2027361, CTONG2027591, CTONG2027736, CTONG2027783, CTONG2027828, CTONG2027959, CTONG2028097, CTONG2028208, CTONG2028486, CTONG2028518, CTONG2028687, CTONG3000657, CTONG3000686, CTONG3000707, CTONG3000896, CTONG3001420, CTONG3001501, CTONG3001560, CTONG3001605, CTONG3001746, CTONG3002020, CTONG3002127, CTONG3002370, CTONG3002412, CTONG3002433, CTONG3002552, CTONG3002588, CTONG3002674, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317,
CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009287, CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001788, D3OST2002182, D3OST2002223, D3OST2002262,
D3OST2002272, D3OST2002417, D3OST2002436, D3OST2002648, D3OST2003331, D3OST2003607, D3OST2003797, D3OST2004637, D3OST3000195, D3OST3000258, D3OST3000291, D3OST3000388, D3OST3000493, D6OST2000127, D6OST2000169, D6OST2000245, D6OST2000358, D6OST2000464, D6OST2000507, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003791, D9OST2003989, D9OST2004018, D9OST2004073, D9OST2004417, DFNES1000003, DFNES1000083, DFNES1000107, DFNES1000185, DFNES2000143, DFNES2000146, DFNES2000212, DFNES2000292, DFNES2000426, DFNES2000432, DFNES2000457, DFNES2000471,
DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2003081, DFNES2003255, DFNES2003742, DFNES2004354, DFNES2004383, DFNES2004608, DFNES2004684, DFNES2005266, DFNES2005527, DFNES2005540, DFNES2005690, DFNES2005766, DFNES2005779, DFNES2005875, DFNES2006346, DFNES2006944, DFNES2007299, DFNES2007332, DFNES2007641, DFNES2007757, DFNES2008160, DFNES2008280, DFNES2008881, DFNES2009482, DFNES2010502, DFNES2011192, DFNES2011221, DFNES2011239, DFNES2011245, ERLTF1000021, ERLTF2000324, ERLTF2000465, FCBBF1000023, FCBBF1000025,
FCBBF1000038, FCBBF1000053, FCBBF1000069, FCBBF1000115, FCBBF1000117, FCBBF1000118, FCBBF1000121, FCBBF1000125, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000367, FCBBF1000376, FCBBF1000377, FCBBF1000395, FCBBF1000397, FCBBF1000403, FCBBF1000412, FCBBF1000437, FCBBF1000449, FCBBF1000466, FCBBF1000476, FCBBF1000506, FCBBF1000509, FCBBF1000546, FCBBF1000550, FCBBF1000556, FCBBF1000581, FCBBF1000582, FCBBF1000598, FCBBF1000604, FCBBF1000618, FCBBF1000627,
FCBBF1000671, FCBBF1000675, FCBBF1000684, FCBBF1000686, FCBBF1000687, FCBBF1000691, FCBBF1000732, FCBBF1000748, FCBBF2000038, FCBBF2000094, FCBBF2000105, FCBBF2000123, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001001, FCBBF2001088, FCBBF2001105, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594,
FCBBF2001672, FCBBF2001675, FCBBF2001718, FCBBF2001720, FCBBF2001817, FCBBF2001868, FCBBF2002044, FCBBF2002281, FCBBF2002370, FCBBF2002541, FCBBF2002626, FCBBF2002897, FCBBF2002898, FCBBF2002904, FCBBF2002928, FCBBF2002953, FCBBF2003083, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005733, FCBBF2005909, FCBBF2005966, FCBBF2006131, FCBBF2006380,
FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000115, FCBBF3000120, FCBBF3000168, FCBBF3000175, FCBBF3000227, FCBBF3000229, FCBBF3000233, FCBBF3000269, FCBBF3000361, FCBBF3000367, FCBBF3000462, FCBBF3000473, FCBBF3000501, FCBBF3000518, FCBBF3000550, FCBBF3000847, FCBBF3001018, FCBBF3001020, FCBBF3001235, FCBBF3001302, FCBBF3001594, FCBBF3001657, FCBBF3001855, FCBBF3001890, FCBBF3001912, FCBBF3001914, FCBBF3001918, FCBBF3001924, FCBBF3002163, FCBBF3002188, FCBBF3002190, FCBBF3002268, FCBBF3002475, FCBBF3002556, FCBBF3002592, FCBBF3002658, FCBBF3002782,
FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005320, FCBBF3005444, FCBBF3005698, FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007244, FCBBF3007248, FCBBF3007453, FCBBF3007462, FCBBF3007540, FCBBF3007604, FCBBF3007631, FCBBF3007829, FCBBF3007855, FCBBF3007859, FCBBF3007860, FCBBF3008073, FCBBF3008100, FCBBF3008153,
FCBBF3008251, FCBBF3008311, FCBBF3008362, FCBBF3008382, FCBBF3008475, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011523, FCBBF3011570, FCBBF3011592, FCBBF3011867, FCBBF3011889, FCBBF3012170, FCBBF3012182,
FCBBF3012332, FCBBF3012443, FCBBF3012588, FCBBF3012667, FCBBF3012842, FCBBF3012901, FCBBF3013041, FCBBF3013058, FCBBF3013205, FCBBF3013307, FCBBF3013341, FCBBF3013589, FCBBF3013623, FCBBF3013800, FCBBF3013846, FCBBF3014229, FCBBF3014260, FCBBF3014355, FCBBF3014567, FCBBF3015100, FCBBF3015119, FCBBF3015131, FCBBF3015317, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017535, FCBBF3017681, FCBBF3017729, FCBBF3017873, FCBBF3017974, FCBBF3018067, FCBBF3018173, FCBBF3018453,
FCBBF3018591, FCBBF3018753, FCBBF3018826, FCBBF3018949, FCBBF3019085, FCBBF3019320, FCBBF3019437, FCBBF3019564, FCBBF3019569, FCBBF3019570, FCBBF3019663, FCBBF3019714, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024096, FCBBF3024225, FCBBF3024266,
FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025905, FCBBF3026021, FCBBF3026048, FCBBF3026251, FCBBF3026308, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028244, FCBBF3028472, FCBBF3028528, FCBBF3028582, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415,
FCBBF4000529, FCBBF5000041, FCBBF5000061, FCBBF5000165, FCBBF5000325, FCBBF5000353, FCBBF5000384, FCBBF5000483, FCBBF5000495, FEBRA1000005, FEBRA1000008, FEBRA1000030, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000129, FEBRA2000220, FEBRA2000228, FEBRA2000253, FEBRA2000286, FEBRA2000297, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000454, FEBRA2000462, FEBRA2000468, FEBRA2000491, FEBRA2000504, FEBRA2000536, FEBRA2000538,
FEBRA2000575, FEBRA2000581, FEBRA2000656, FEBRA2000659, FEBRA2000680, FEBRA2000690, FEBRA2000727, FEBRA2000734, FEBRA2000757, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000862, FEBRA2000877, FEBRA2000880, FEBRA2000901, FEBRA2000909, FEBRA2000917, FEBRA2000928, FEBRA2000936, FEBRA2000937, FEBRA2000959, FEBRA2001004, FEBRA2001012, FEBRA2001020, FEBRA2001093, FEBRA2001124, FEBRA2001146, FEBRA2001161, FEBRA2001175, FEBRA2001197, FEBRA2001217, FEBRA2001227, FEBRA2001245, FEBRA2001267, FEBRA2001294, FEBRA2001351, FEBRA2001383, FEBRA2001438, FEBRA2001440, FEBRA2001487, FEBRA2001561, FEBRA2001581,
FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001772, FEBRA2001790, FEBRA2001814, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002442, FEBRA2002456, FEBRA2002508, FEBRA2002525, FEBRA2002552, FEBRA2002574, FEBRA2002601, FEBRA2002611, FEBRA2002628, FEBRA2002662, FEBRA2002682, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931,
FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003750, FEBRA2003822, FEBRA2003897, FEBRA2003907, FEBRA2003930, FEBRA2004026, FEBRA2004029, FEBRA2004042, FEBRA2004053, FEBRA2004056, FEBRA2004091, FEBRA2004110, FEBRA2004202, FEBRA2004219, FEBRA2004237, FEBRA2004268, FEBRA2004325, FEBRA2004329, FEBRA2004331, FEBRA2004443, FEBRA2004485, FEBRA2004490, FEBRA2004538, FEBRA2004592,
FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005427, FEBRA2005458, FEBRA2005476, FEBRA2005572, FEBRA2005701, FEBRA2005726, FEBRA2005778, FEBRA2005787, FEBRA2005788, FEBRA2005824, FEBRA2005998, FEBRA2006055, FEBRA2006061, FEBRA2006150, FEBRA2006165, FEBRA2006255, FEBRA2006315, FEBRA2006346, FEBRA2006354, FEBRA2006357, FEBRA2006370, FEBRA2006379, FEBRA2006396, FEBRA2006409, FEBRA2006418, FEBRA2006427, FEBRA2006476, FEBRA2006485, FEBRA2006491,
FEBRA2006519, FEBRA2006521, FEBRA2006553, FEBRA2006627, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007714, FEBRA2007793, FEBRA2007818, FEBRA2007823, FEBRA2007880, FEBRA2007931, FEBRA2007957, FEBRA2008009, FEBRA2008086, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008287, FEBRA2008302,
FEBRA2008311, FEBRA2008341, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009352, FEBRA2009362, FEBRA2009385, FEBRA2009416, FEBRA2009437, FEBRA2009478, FEBRA2009507, FEBRA2009541, FEBRA2009567, FEBRA2009588, FEBRA2009731, FEBRA2009754, FEBRA2009780, FEBRA2009846, FEBRA2010141, FEBRA2010299, FEBRA2010484, FEBRA2010719,
FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014443, FEBRA2014578, FEBRA2014939, FEBRA2015076, FEBRA2015175, FEBRA2015292, FEBRA2015588, FEBRA2016112, FEBRA2016398, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333,
FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2019172, FEBRA2019242, FEBRA2019298, FEBRA2019389, FEBRA2019457, FEBRA2019519, FEBRA2019582, FEBRA2019637, FEBRA2019690, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021431, FEBRA2021497, FEBRA2021550, FEBRA2021571, FEBRA2021636, FEBRA2021864, FEBRA2021892, FEBRA2021908, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956,
FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024343, FEBRA2024570, FEBRA2024693, FEBRA2024727, FEBRA2024744, FEBRA2024797, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021,
HCASM1000050, HCASM1000061, HCASM1000130, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000142, HCHON1000176, HCHON1000191, HCHON2000028, HCHON2000038, HCHON2000062, HCHON2000100,
HCHON2000156, HCHON2000199, HCHON2000212, HCHON2000271, HCHON2000295, HCHON2000315, HCHON2000322, HCHON2000344, HCHON2000364, HCHON2000395, HCHON2000417, HCHON2000418, HCHON2000473, HCHON2000503, HCHON2000660, HCHON2000673, HCHON2000676, HCHON2000699, HCHON2000705, HCHON2000743, HCHON2000751, HCHON2000817, HCHON2000818, HCHON2000819, HCHON2000832, HCHON2000851, HCHON2000898, HCHON2000935, HCHON2000942, HCHON2000956, HCHON2000994, HCHON2001050, HCHON2001084, HCHON2001108, HCHON2001110, HCHON2001116, HCHON2001161, HCHON2001214, HCHON2001233, HCHON2001269, HCHON2001434, HCHON2001450, HCHON2001453, HCHON2001497, HCHON2001523, HCHON2001546, HCHON2001548, HCHON2001577, HCHON2001604, HCHON2001607,
HCHON2001646, HCHON2001712, HCHON2001768, HCHON2001801, HCHON2001853, HCHON2002123, HCHON2002194, HCHON2002241, HCHON2002247, HCHON2002354, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987, HCHON2006459, HCHON2006714, HCHON2006722, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672,
HCHON2008856, HCHON2008871, HCHON2008989, HCHON2009191, HCHON2009384, HCHON2009749, HCHON2009766, HCHON2009795, HCHON2010074, HCHON2010543, HEART1000074, HEART1000088, HEART1000099, HEART1000102, HEART1000118, HEART1000132, HEART1000139, HEART1000142, HEART1000151, HEART1000173, HEART2000024, HEART2000035, HEART2000099, HEART2000298, HEART2000306, HEART2000309, HEART2000411, HEART2000418, HEART2000448, HEART2000492, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003852,
HEART2004570, HEART2004940, HEART2004980, HEART2005244, HEART2005581, HEART2005610, HEART2006131, HEART2006195, HEART2006310, HEART2006334, HEART2006487, HEART2006557, HEART2006787, HEART2006789, HEART2006909, HEART2007031, HEART2007231, HEART2007443, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC2000055, HHDPC2000149, HHDPC2000258, HHDPC2000315, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381,
HHDPC2004757, HHDPC2005185, HHDPC2005513, HHDPC2005742, HHDPC2006460, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009208, HHDPC2009272, HLUNG1000017, HLUNG1000024, HLUNG1000030, HLUNG1000034, HLUNG1000053, HLUNG1000064, HLUNG1000076, HLUNG1000084, HLUNG1000091, HLUNG1000104, HLUNG1000105, HLUNG1000151, HLUNG1000169, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000315, HLUNG2000340, HLUNG2000362,
HLUNG2000363, HLUNG2000412, HLUNG2000431, HLUNG2000480, HLUNG2000489, HLUNG2000549, HLUNG2000557, HLUNG2000570, HLUNG2000751, HLUNG2000761, HLUNG2000777, HLUNG2000870, HLUNG2000926, HLUNG2000950, HLUNG2000955, HLUNG2000970, HLUNG2001013, HLUNG2001126, HLUNG2001144, HLUNG2001214, HLUNG2001439, HLUNG2001459, HLUNG2001507, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002659, HLUNG2002707, HLUNG2002811, HLUNG2002916, HLUNG2002942, HLUNG2002949, HLUNG2002958, HLUNG2002982, HLUNG2003049, HLUNG2003061,
HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004154, HLUNG2004170, HLUNG2004217, HLUNG2004273, HLUNG2004388, HLUNG2004416, HLUNG2004521, HLUNG2004684, HLUNG2004707, HLUNG2004774, HLUNG2004775, HLUNG2005012, HLUNG2005030, HLUNG2005045, HLUNG2005076, HLUNG2005203, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614,
HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008153, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008833, HLUNG2008875, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097,
HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA2000044, HSYRA2000135, HSYRA2000137, HSYRA2000221, HSYRA2000237, HSYRA2000248, HSYRA2000253, HSYRA2000332, HSYRA2000371, HSYRA2000424, HSYRA2000510, HSYRA2000605, HSYRA2000629, HSYRA2000706, HSYRA2000760, HSYRA2000792, HSYRA2000828, HSYRA2000832, HSYRA2000925, HSYRA2000927, HSYRA2001003, HSYRA2001105,
HSYRA2001142, HSYRA2001153, HSYRA2001232, HSYRA2001255, HSYRA2001332, HSYRA2001353, HSYRA2001484, HSYRA2001552, HSYRA2001574, HSYRA2001580, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000158, IMR321000161, IMR321000165, IMR321000193, IMR321000207, IMR321000230, IMR322000072, IMR322000107, IMR322000121, IMR322000127, IMR322000152, IMR322000164, IMR322000178, IMR322000302, IMR322000316, IMR322000374, IMR322000379, IMR322000383, IMR322000418,
IMR322000518, IMR322000604, IMR322000609, IMR322000656, IMR322000672, IMR322000775, IMR322000791, IMR322000811, IMR322000819, IMR322000838, IMR322000859, IMR322000863, IMR322000917, IMR322000918, IMR322000919, IMR322000935, IMR322000953, IMR322000973, IMR322000984, IMR322001018, IMR322001049, IMR322001167, IMR322001185, IMR322001218, IMR322001245, IMR322001317, IMR322001318, IMR322001322, IMR322001438, IMR322001452, IMR322001534, IMR322001541, IMR322001665, IMR322001670, IMR322001724, IMR322001879, IMR322002035, IMR322002110, IMR322002470, IMR322003675, IMR322004768, IMR322005293, IMR322005833, IMR322006012, IMR322006222, IMR322006495, IMR322006520, IMR322006886, IMR322006947, IMR322007078,
IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000349, KIDNE2000375, KIDNE2000383,
KIDNE2000394, KIDNE2000422, KIDNE2000493, KIDNE2000510, KIDNE2000513, KIDNE2000519, KIDNE2000555, KIDNE2000624, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2000909, KIDNE2001117, KIDNE2001140, KIDNE2001269, KIDNE2001361, KIDNE2001373, KIDNE2001636, KIDNE2001713, KIDNE2001802, KIDNE2001840, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882,
KIDNE2002980, KIDNE2002991, KIDNE2003185, KIDNE2003188, KIDNE2003233, KIDNE2003335, KIDNE2003373, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004305, KIDNE2004344, KIDNE2004394, KIDNE2004411, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400,
KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006811, KIDNE2006820, KIDNE2006880, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007944, KIDNE2008069, KIDNE2008072, KIDNE2008116, KIDNE2008117, KIDNE2008218, KIDNE2008315, KIDNE2008362, KIDNE2008378,
KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010762, KIDNE2010863, KIDNE2010973, KIDNE2010989, KIDNE2011200,
KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013388, KIDNE2013413, KIDNE2013489, KIDNE2013639, KIDNE2013731, KIDNE2013734, KIDNE2013775, KIDNE2013801, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717,
KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018254, KIDNE2018268, KIDNE2018269, KIDNE2018287, KIDNE2018294, KIDNE2018462, KIDNE2018493,
KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099, LIVER1000104, LIVER1000111, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER2000003, LIVER2000037, LIVER2000158, LIVER2000216, LIVER2000237, LIVER2000247, LIVER2000360, LIVER2000515, LIVER2000592, LIVER2000626, LIVER2000682, LIVER2000769, LIVER2000775, LIVER2001051, LIVER2001076, LIVER2001099, LIVER2001164,
LIVER2001191, LIVER2001265, LIVER2001329, LIVER2001389, LIVER2001461, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008465, LIVER2008473, LIVER2008580, LIVER2008706,
LIVER2008751, LIVER2008945, LIVER2008998, LIVER2009110, LIVER2009118, LIVER2009554, LYMPB1000158, LYMPB2000083, MAMGL1000017, MAMGL1000083, MAMGL1000096, MAMGL1000132, MAMGL1000151, MAMGL1000178, MAMGL1000182, MAMGL1000184, MESAN1000032, MESAN1000035, MESAN1000079, MESAN1000121, MESAN1000126, MESAN1000147, MESAN1000180, MESAN2000023, MESAN2000026, MESAN2000067, MESAN2000167, MESAN2000264, MESAN2000337, MESAN2000457, MESAN2000462, MESAN2000572, MESAN2000608, MESAN2000620, MESAN2000711, MESAN2000815, MESAN2000894, MESAN2001154, MESAN2001627, MESAN2001770, MESAN2001979, MESAN2001980, MESAN2002113, MESAN2002122, MESAN2002147, MESAN2002186, MESAN2002424, MESAN2002687, MESAN2002709, MESAN2002978,
MESAN2003035, MESAN2003037, MESAN2003039, MESAN2003058, MESAN2003101, MESAN2003190, MESAN2003322, MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005724, MESAN2005808, MESAN2005811, MESAN2005957, MESAN2006022, MESAN2006043, MESAN2006328, MESAN2006401, MESAN2006580, MESAN2006599, MESAN2006743, MESAN2006953, MESAN2006971, MESAN2007032, MESAN2007440, MESAN2007597, MESAN2008222, MESAN2008334, MESAN2008415, MESAN2008460, MESAN2008536, MESAN2008729, MESAN2008821, MESAN2008926, MESAN2008936, MESAN2009019, MESAN2009081, MESAN2009105, MESAN2009311, MESAN2009418, MESAN2009503,
MESAN2009522, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014624, MESAN2015277, MESAN2015365, MESAN2015391, MESAN2015401, MESAN2015501, MESAN2015708, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147,
MESTC2000150, MESTC2000153, MESTC2000170, N1ESE2000698, NB9N41000011, NB9N41000047, NB9N41000135, NB9N41000142, NB9N41000146, NB9N42000122, NB9N42000196, NB9N42000281, NB9N42000314, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000606, NHNPC2000877, NHNPC2001223, NHNPC2001312,
NHNPC2001816, NHNPC2001931, NHNPC2002565, NHNPC2002749, NOVAR1000015, NOVAR2000038, NOVAR2000136, NOVAR2000352, NOVAR2000412, NOVAR2000710, NOVAR2000962, NOVAR2001108, NOVAR2001620, NOVAR2001783, NOVAR2001876, NOVAR2002039, NT2NE1000004, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000222, NT2NE2000284, NT2NE2000299, NT2NE2000353, NT2NE2000356, NT2NE2000369, NT2NE2000374, NT2NE2000386, NT2NE2000390, NT2NE2000392, NT2NE2000455, NT2NE2000470, NT2NE2000488, NT2NE2000517, NT2NE2000536,
NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000864, NT2NE2000909, NT2NE2000913, NT2NE2000951, NT2NE2000980, NT2NE2001000, NT2NE2001021, NT2NE2001040, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001442, NT2NE2001524, NT2NE2001530, NT2NE2001617, NT2NE2001623, NT2NE2001626, NT2NE2001634, NT2NE2001655, NT2NE2001660, NT2NE2001666, NT2NE2001677, NT2NE2001698, NT2NE2001723, NT2NE2001725,
NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2003150, NT2NE2003185, NT2NE2003252, NT2NE2003280, NT2NE2003308, NT2NE2003309, NT2NE2003315, NT2NE2003393, NT2NE2003408, NT2NE2003457, NT2NE2003485, NT2NE2003540, NT2NE2003571, NT2NE2003705, NT2NE2003752, NT2NE2004378, NT2NE2004490, NT2NE2004519, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005441, NT2NE2005517, NT2NE2005537, NT2NE2005555, NT2NE2005588,
NT2NE2005676, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006458, NT2NE2006531, NT2NE2006659, NT2NE2006784, NT2NE2006813, NT2NE2006894, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008803, NT2NE2008867, NT2NE2008961, NT2NE2008997, NT2NE2009138, NT2NE2009295,
NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2011033, NT2NE2011036, NT2NE2011119, NT2NE2011154, NT2NE2011221, NT2NE2011411, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012847, NT2NE2012928, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221,
NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2018165, NT2NE2018176, NT2NE2018273, NT2NE2018376, NT2NE2018472, NT2NE2018594, NT2NE2018739, NT2NE2018916, NT2NE2019056, NT2NE2019076,
NT2NE2019099, NT2RI1000016, NT2RI1000035, NT2RI1000127, NT2RI1000164, NT2RI1000172, NT2RI2000004, NT2RI2000007, NT2RI2000116, NT2RI2000117, NT2RI2000128, NT2RI2000167, NT2RI2000173, NT2RI2000187, NT2RI2000270, NT2RI2000276, NT2RI2000282, NT2RI2000313, NT2RI2000341, NT2RI2000344, NT2RI2000348, NT2RI2000480, NT2RI2000575, NT2RI2000588, NT2RI2000597, NT2RI2000669, NT2RI2000671, NT2RI2000688, NT2RI2000704, NT2RI2000738, NT2RI2000775, NT2RI2000822, NT2RI2000829, NT2RI2000841, NT2RI2000865, NT2RI2000932, NT2RI2000974, NT2RI2000987, NT2RI2001010, NT2RI2001017, NT2RI2001030, NT2RI2001054, NT2RI2001083, NT2RI2001178, NT2RI2001235, NT2RI2001244, NT2RI2001307, NT2RI2001385, NT2RI2001409, NT2RI2001410,
NT2RI2001425, NT2RI2001449, NT2RI2001450, NT2RI2001474, NT2RI2001519, NT2RI2001595, NT2RI2001624, NT2RI2001726, NT2RI2001769, NT2RI2001782, NT2RI2001836, NT2RI2001846, NT2RI2001866, NT2RI2001952, NT2RI2002022, NT2RI2002041, NT2RI2002120, NT2RI2002153, NT2RI2002179, NT2RI2002252, NT2RI2002260, NT2RI2002270, NT2RI2002334, NT2RI2002359, NT2RI2002391, NT2RI2002447, NT2RI2002481, NT2RI2002507, NT2RI2002517, NT2RI2002530, NT2RI2002540, NT2RI2002549, NT2RI2002564, NT2RI2002592, NT2RI2002602, NT2RI2002699, NT2RI2002729, NT2RI2002819, NT2RI2002822, NT2RI2002865, NT2RI2002923, NT2RI2002926, NT2RI2002958, NT2RI2002970, NT2RI2003011, NT2RI2003067, NT2RI2003184, NT2RI2003222, NT2RI2003301, NT2RI2003360,
NT2RI2003361, NT2RI2003383, NT2RI2003392, NT2RI2003407, NT2RI2003419, NT2RI2003481, NT2RI2003487, NT2RI2003655, NT2RI2003667, NT2RI2003751, NT2RI2003884, NT2RI2003973, NT2RI2004059, NT2RI2004078, NT2RI2004079, NT2RI2004093, NT2RI2004136, NT2RI2004157, NT2RI2004190, NT2RI2004230, NT2RI2004284, NT2RI2004290, NT2RI2004304, NT2RI2004312, NT2RI2004381, NT2RI2004442, NT2RI2004468, NT2RI2004606, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005069, NT2RI2005087, NT2RI2005115, NT2RI2005130, NT2RI2005150, NT2RI2005239, NT2RI2005246, NT2RI2005315, NT2RI2005353, NT2RI2005358, NT2RI2005368, NT2RI2005382,
NT2RI2005473, NT2RI2005520, NT2RI2005564, NT2RI2005579, NT2RI2005621, NT2RI2005628, NT2RI2005670, NT2RI2005710, NT2RI2005772, NT2RI2005814, NT2RI2005816, NT2RI2005851, NT2RI2005966, NT2RI2006071, NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487, NT2RI2006565, NT2RI2006640, NT2RI2006659, NT2RI2006662, NT2RI2006667, NT2RI2006679, NT2RI2006682, NT2RI2006686, NT2RI2006703, NT2RI2006716, NT2RI2006762, NT2RI2006788, NT2RI2006838, NT2RI2006853, NT2RI2006973, NT2RI2007046, NT2RI2007048, NT2RI2007084, NT2RI2007096, NT2RI2007116, NT2RI2007214, NT2RI2007277, NT2RI2007303, NT2RI2007334, NT2RI2007377,
NT2RI2007386, NT2RI2007406, NT2RI2007439, NT2RI2007507, NT2RI2007593, NT2RI2007629, NT2RI2007751, NT2RI2007754, NT2RI2007827, NT2RI2007877, NT2RI2007884, NT2RI2007891, NT2RI2007956, NT2RI2007965, NT2RI2007987, NT2RI2008050, NT2RI2008141, NT2RI2008204, NT2RI2008221, NT2RI2008233, NT2RI2008455, NT2RI2008481, NT2RI2008502, NT2RI2008566, NT2RI2008598, NT2RI2008622, NT2RI2008714, NT2RI2008791, NT2RI2008801, NT2RI2008812, NT2RI2008841, NT2RI2008952, NT2RI2009005, NT2RI2009101, NT2RI2009144, NT2RI2009151, NT2RI2009194, NT2RI2009215, NT2RI2009239, NT2RI2009259, NT2RI2009269, NT2RI2009289, NT2RI2009301, NT2RI2009402, NT2RI2009406, NT2RI2009517, NT2RI2009585, NT2RI2009595, NT2RI2009855, NT2RI2010283,
NT2RI2010508, NT2RI2010795, NT2RI2011422, NT2RI2011450, NT2RI2011683, NT2RI2011803, NT2RI2012659, NT2RI2012990, NT2RI2013345, NT2RI2013373, NT2RI2014247, NT2RI2014551, NT2RI2014683, NT2RI2014733, NT2RI2015239, NT2RI2016128, NT2RI2016134, NT2RI2017529, NT2RI2018311, NT2RI2018363, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024460, NT2RI2024496, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975,
NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001573, NT2RI3001967, NT2RI3002303, NT2RI3002557, NT2RI3002842, NT2RI3003027, NT2RI3003031, NT2RI3003095, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005861, NT2RI3005923, NT2RI3005928, NT2RI3006132, NT2RI3006171, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443,
NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009480, NT2RI3009524, NT2RP6000005, NT2RP6000008, NT2RP6000032, NT2RP6000033, NT2RP6000059, NT2RP6000060, NT2RP6000077, NT2RP6000086, NT2RP6000119, NT2RP6000127, NT2RP6000140, NT2RP6000150, NT2RP7000041, NT2RP7000086, NT2RP7000109, NT2RP7000112, NT2RP7000311, NT2RP7000359, NT2RP7000364, NT2RP7000380, NT2RP7000391, NT2RP7000425, NT2RP7000579, NT2RP7000812, NT2RP7000926, NT2RP7001074, NT2RP7001306, NT2RP7001319, NT2RP7001335, NT2RP7001364, NT2RP7001475, NT2RP7001532, NT2RP7001591, NT2RP7001602, NT2RP7001856, NT2RP7002064,
NT2RP7002151, NT2RP7002163, NT2RP7002243, NT2RP7002282, NT2RP7002376, NT2RP7002478, NT2RP7002554, NT2RP7002594, NT2RP7002603, NT2RP7002650, NT2RP7002829, NT2RP7002841, NT2RP7002875, NT2RP7002978, NT2RP7003025, NT2RP7003050, NT2RP7003055, NT2RP7003084, NT2RP7003091, NT2RP7003107, NT2RP7003134, NT2RP7003148, NT2RP7003439, NT2RP7003511, NT2RP7003632, NT2RP7004027, NT2RP7004080, NT2RP7004114, NT2RP7004123, NT2RP7004348, NT2RP7004358, NT2RP7004396, NT2RP7004428, NT2RP7004481, NT2RP7004541, NT2RP7004559, NT2RP7004656, NT2RP7004722, NT2RP7004766, NT2RP7004790, NT2RP7004884, NT2RP7004915, NT2RP7004925, NT2RP7004961, NT2RP7004964, NT2RP7004975, NT2RP7005118, NT2RP7005205, NT2RP7005219, NT2RP7005323,
NT2RP7005468, NT2RP7005493, NT2RP7005513, NT2RP7005629, NT2RP7005631, NT2RP7005669, NT2RP7005846, NT2RP7006075, NT2RP7006141, NT2RP7006160, NT2RP7006162, NT2RP7006263, NT2RP7006374, NT2RP7006395, NT2RP7006408, NT2RP7006457, NT2RP7006490, NT2RP7006601, NT2RP7006619, NT2RP7006621, NT2RP7006636, NT2RP7006701, NT2RP7006853, NT2RP7006886, NT2RP7006887, NT2RP7006980, NT2RP7006986, NT2RP7006995, NT2RP7007177, NT2RP7007221, NT2RP7007226, NT2RP7007269, NT2RP7007359, NT2RP7007406, NT2RP7007480, NT2RP7007504, NT2RP7007530, NT2RP7007594, NT2RP7007610, NT2RP7007617, NT2RP7007766, NT2RP7007925, NT2RP7007975, NT2RP7008013, NT2RP7008015, NT2RP7008133, NT2RP7008137, NT2RP7008142, NT2RP7008144, NT2RP7008161,
NT2RP7008360, NT2RP7008396, NT2RP7008406, NT2RP7008435, NT2RP7008441, NT2RP7008454, NT2RP7008487, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009097, NT2RP7009168, NT2RP7009215, NT2RP7009225, NT2RP7009236, NT2RP7009253, NT2RP7009267, NT2RP7009363, NT2RP7009397, NT2RP7009429, NT2RP7009439, NT2RP7009466, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452,
NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871,
NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020343, NT2RP7020379, NT2RP8000137, NT2RP8000296, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000247, NTONG1000257, NTONG1000264, NTONG2000040,
NTONG2000107, NTONG2000231, NTONG2000363, NTONG2000492, NTONG2000499, NTONG2000531, NTONG2000583, NTONG2000858, NTONG2000876, NTONG2000966, NTONG2001014, NTONG2001079, NTONG2001157, NTONG2001173, NTONG2001220, NTONG2001362, NTONG2001428, NTONG2001532, NTONG2001612, NTONG2001619, NTONG2001642, NTONG2001657, NTONG2001676, NTONG2002278, NTONG2002807, NTONG2002948, NTONG2002970, NTONG2002985, NTONG2003158, NTONG2003210, NTONG2003316, NTONG2003409, NTONG2003515, NTONG2003805, NTONG2003839, NTONG2003852, NTONG2003897, NTONG2004095, NTONG2004308, NTONG2004521, NTONG2004614, NTONG2004690, NTONG2004806, NTONG2004829, NTONG2004918, NTONG2004991, NTONG2005062, NTONG2005086, NTONG2005137, NTONG2005153,
NTONG2005265, NTONG2005277, NTONG2005363, NTONG2005373, NTONG2005480, NTONG2005497, NTONG2005577, NTONG2005657, NTONG2005822, NTONG2005897, NTONG2005969, NTONG2006099, NTONG2006187, NTONG2006324, NTONG2006354, NTONG2006440, NTONG2006501, NTONG2006646, NTONG2006709, NTONG2006783, NTONG2007020, NTONG2007028, NTONG2007034, NTONG2007249, NTONG2007517, NTONG2007522, NTONG2007756, NTONG2008093, NTONG2008143, NTONG2008365, NTONG2008522, NTONG2008672, NTONG2008862, NTONG2008939, NTONG2009060, NTONG2009068, NTONG2009229, NTONG2009233, NTONG2009468, OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000114,
OCBBF1000118, OCBBF1000119, OCBBF1000122, OCBBF1000130, OCBBF1000138, OCBBF1000145, OCBBF1000146, OCBBF1000153, OCBBF1000175, OCBBF1000192, OCBBF1000254, OCBBF2000126, OCBBF2000178, OCBBF2000231, OCBBF2000277, OCBBF2000287, OCBBF2000323, OCBBF2000452, OCBBF2000522, OCBBF2000677, OCBBF2000703, OCBBF2000712, OCBBF2000719, OCBBF2000824, OCBBF2000904, OCBBF2000998, OCBBF2001075, OCBBF2001101, OCBBF2001124, OCBBF2001176, OCBBF2001196, OCBBF2001307, OCBBF2001323, OCBBF2001389, OCBBF2001402, OCBBF2001473, OCBBF2001494, OCBBF2001527, OCBBF2001639, OCBBF2001681, OCBBF2001706, OCBBF2001749, OCBBF2001910, OCBBF2001931, OCBBF2001961, OCBBF2001983, OCBBF2002015, OCBBF2002124, OCBBF2002161, OCBBF2002290,
OCBBF2002357, OCBBF2002376, OCBBF2002615, OCBBF2002626, OCBBF2002663, OCBBF2002805, OCBBF2002920, OCBBF2002980, OCBBF2003028, OCBBF2003091, OCBBF2003246, OCBBF2003327, OCBBF2003543, OCBBF2003593, OCBBF2003744, OCBBF2004038, OCBBF2004104, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005546, OCBBF2005843, OCBBF2005901, OCBBF2006030, OCBBF2006058,
OCBBF2006113, OCBBF2006148, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006849, OCBBF2006859, OCBBF2006972, OCBBF2007039, OCBBF2007051, OCBBF2007068, OCBBF2007114, OCBBF2007121, OCBBF2007184, OCBBF2007194, OCBBF2007196, OCBBF2007238, OCBBF2007414, OCBBF2007428, OCBBF2007478, OCBBF2007485, OCBBF2007520, OCBBF2007555, OCBBF2007610, OCBBF2007622, OCBBF2007756, OCBBF2007762, OCBBF2007931, OCBBF2007946, OCBBF2008005, OCBBF2008116, OCBBF2008138, OCBBF2008283, OCBBF2008330, OCBBF2008340, OCBBF2008366, OCBBF2008466, OCBBF2008483, OCBBF2008511, OCBBF2008520, OCBBF2008569,
OCBBF2008586, OCBBF2008640, OCBBF2008691, OCBBF2008701, OCBBF2008724, OCBBF2008760, OCBBF2008768, OCBBF2008775, OCBBF2008790, OCBBF2008814, OCBBF2008822, OCBBF2009095, OCBBF2009115, OCBBF2009301, OCBBF2009352, OCBBF2009391, OCBBF2009536, OCBBF2009571, OCBBF2009583, OCBBF2009603, OCBBF2009788, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010841, OCBBF2010843, OCBBF2010858, OCBBF2010863, OCBBF2010871, OCBBF2010931, OCBBF2010945, OCBBF2010978, OCBBF2010989, OCBBF2011073, OCBBF2011137, OCBBF2011160, OCBBF2011177, OCBBF2011228, OCBBF2011232, OCBBF2011283,
OCBBF2011311, OCBBF2011625, OCBBF2011669, OCBBF2011685, OCBBF2011706, OCBBF2011722, OCBBF2011767, OCBBF2011855, OCBBF2011872, OCBBF2011887, OCBBF2011897, OCBBF2011914, OCBBF2011960, OCBBF2011981, OCBBF2012028, OCBBF2012039, OCBBF2012095, OCBBF2012139, OCBBF2012191, OCBBF2012262, OCBBF2012320, OCBBF2012436, OCBBF2012525, OCBBF2012678, OCBBF2012704, OCBBF2012714, OCBBF2012755, OCBBF2012812, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013843, OCBBF2013883, OCBBF2013926,
OCBBF2014052, OCBBF2014064, OCBBF2014089, OCBBF2014322, OCBBF2014386, OCBBF2014576, OCBBF2014707, OCBBF2014745, OCBBF2014828, OCBBF2014880, OCBBF2014928, OCBBF2015031, OCBBF2015081, OCBBF2015115, OCBBF2015232, OCBBF2015233, OCBBF2015285, OCBBF2015335, OCBBF2015503, OCBBF2015506, OCBBF2015599, OCBBF2015645, OCBBF2015659, OCBBF2015751, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2017035, OCBBF2017055, OCBBF2017069, OCBBF2017306, OCBBF2017325, OCBBF2017489, OCBBF2017516, OCBBF2017665, OCBBF2017882, OCBBF2017888, OCBBF2018012, OCBBF2018014, OCBBF2018084, OCBBF2018167,
OCBBF2018206, OCBBF2018229, OCBBF2018234, OCBBF2018362, OCBBF2018380, OCBBF2018581, OCBBF2018663, OCBBF2018687, OCBBF2018828, OCBBF2018934, OCBBF2019108, OCBBF2019195, OCBBF2019327, OCBBF2019684, OCBBF2019761, OCBBF2019823, OCBBF2019950, OCBBF2020318, OCBBF2020343, OCBBF2020453, OCBBF2020741, OCBBF2020801, OCBBF2020825, OCBBF2020838, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025,
OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906,
OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843,
OCBBF3006802, OCBBF3006986, OCBBF3007078, OCBBF3007704, OCBBF3008230, OCBBF3008392, OCBBF3008835, OCBBF3009192, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000068, PEBLM1000071, PEBLM1000083, PEBLM1000147, PEBLM1000174, PEBLM1000180, PEBLM2000112, PEBLM2000126, PEBLM2000147, PEBLM2000170, PEBLM2000180, PEBLM2000213, PEBLM2000248, PEBLM2000270, PEBLM2000321, PEBLM2000333, PEBLM2000395, PEBLM2000479, PEBLM2000502, PEBLM2001312, PEBLM2001465, PEBLM2001488, PEBLM2002432, PEBLM2002455, PEBLM2002594, PEBLM2002887, PEBLM2003426, PEBLM2003765, PEBLM2003935, PEBLM2004015, PEBLM2004216,
PEBLM2004290, PEBLM2004497, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766,
PERIC2003001, PERIC2003090, PERIC2003287, PERIC2003349, PERIC2003452, PERIC2003699, PERIC2003720, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000058, PLACE5000066, PLACE5000070, PLACE5000080, PLACE5000105, PLACE5000115, PLACE5000116, PLACE5000129, PLACE5000139, PLACE5000153, PLACE5000171,
PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000348, PLACE6000371, PLACE6000424, PLACE6000426, PLACE6000429, PLACE6000450, PLACE6000523, PLACE6000550, PLACE6000552, PLACE6000555, PLACE6000619, PLACE6000630, PLACE6000953, PLACE6001118, PLACE6001153, PLACE6001185, PLACE6001262, PLACE6001294, PLACE6001443, PLACE6001536, PLACE6001557, PLACE6001712, PLACE6001740, PLACE6001823, PLACE6001886, PLACE6001923, PLACE6001925, PLACE6001933, PLACE6002011,
PLACE6002016, PLACE6002056, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6003004, PLACE6003094, PLACE6003109, PLACE6003204, PLACE6003218, PLACE6003372, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004336, PLACE6004368, PLACE6004380, PLACE6004396, PLACE6004454, PLACE6004464, PLACE6004491, PLACE6004663, PLACE6004687, PLACE6004738, PLACE6004931, PLACE6004966, PLACE6004993, PLACE6005007, PLACE6005029, PLACE6005108,
PLACE6005234, PLACE6005283, PLACE6005294, PLACE6005328, PLACE6005351, PLACE6005423, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444,
PLACE6008453, PLACE6008640, PLACE6008750, PLACE6008768, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011102, PLACE6011156, PLACE6011211, PLACE6011260, PLACE6011627, PLACE6011774, PLACE6011881, PLACE6011907, PLACE6011970, PLACE6011975, PLACE6012108, PLACE6012168, PLACE6012297, PLACE6012308, PLACE6012574, PLACE6012908, PLACE6012936,
PLACE6012942, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015211, PLACE6015322, PLACE6015445, PLACE6015460, PLACE6015591, PLACE6015729, PLACE6015731, PLACE6015939, PLACE6016030, PLACE6016093, PLACE6016105, PLACE6016160, PLACE6016210, PLACE6016254, PLACE6016942, PLACE6016984, PLACE6017002, PLACE6017431, PLACE6017564, PLACE6017578, PLACE6017626, PLACE6017701, PLACE6017714, PLACE6018107, PLACE6018187, PLACE6018235, PLACE6018441, PLACE6018487, PLACE6018497, PLACE6018769, PLACE6018834, PLACE6018843, PLACE6018863, PLACE6018938, PLACE6019385, PLACE6019542,
PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000120,
PROST1000123, PROST1000136, PROST1000145, PROST1000152, PROST1000167, PROST1000184, PROST1000215, PROST1000217, PROST1000246, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000526, PROST1000527, PROST1000528, PROST1000536, PROST1000559, PROST1000575, PROST2000036, PROST2000053, PROST2000067, PROST2000105, PROST2000143, PROST2000162, PROST2000165, PROST2000176, PROST2000203, PROST2000206, PROST2000212, PROST2000241, PROST2000267, PROST2000273, PROST2000274, PROST2000277, PROST2000337, PROST2000432, PROST2000452,
PROST2000463, PROST2000470, PROST2000505, PROST2000572, PROST2000717, PROST2000760, PROST2000761, PROST2000835, PROST2000893, PROST2000961, PROST2001002, PROST2001116, PROST2001138, PROST2001213, PROST2001283, PROST2001414, PROST2001415, PROST2001465, PROST2001540, PROST2001599, PROST2001611, PROST2001636, PROST2001739, PROST2001796, PROST2001805, PROST2001823, PROST2001899, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927,
PROST2002960, PROST2003102, PROST2003117, PROST2003210, PROST2003302, PROST2003303, PROST2003324, PROST2003340, PROST2003396, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004332, PROST2004481, PROST2004526, PROST2004570, PROST2004727, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426,
PROST2005466, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006196, PROST2006201, PROST2006260, PROST2006450, PROST2006491, PROST2006510, PROST2006536, PROST2006579, PROST2006688, PROST2006766, PROST2006782, PROST2006933, PROST2006988, PROST2007122, PROST2007200, PROST2007237, PROST2007248, PROST2007289, PROST2007328, PROST2007382, PROST2007389, PROST2007416, PROST2007528, PROST2007818, PROST2007950, PROST2007956, PROST2007974, PROST2007988, PROST2008079, PROST2008088, PROST2008243, PROST2008245, PROST2008268, PROST2008271, PROST2008360, PROST2008447, PROST2008468,
PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010606, PROST2010782, PROST2010885, PROST2011012, PROST2011038, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012353,
PROST2012400, PROST2012504, PROST2012542, PROST2012550, PROST2012740, PROST2012780, PROST2012888, PROST2012890, PROST2013032, PROST2013053, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499,
PROST2016512, PROST2016566, PROST2016582, PROST2016668, PROST2016684, PROST2016777, PROST2016829, PROST2016860, PROST2016918, PROST2016980, PROST2017098, PROST2017128, PROST2017367, PROST2017413, PROST2017441, PROST2017529, PROST2017578, PROST2017612, PROST2017617, PROST2017692, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019296, PROST2019398, PROST2019487, PROST2019781, PUAEN1000039, PUAEN1000081, PUAEN1000085, PUAEN1000087, PUAEN1000121, PUAEN1000147, PUAEN1000175,
PUAEN1000275, PUAEN1000276, PUAEN1000288, PUAEN2000080, PUAEN2000175, PUAEN2000312, PUAEN2000394, PUAEN2000497, PUAEN2000594, PUAEN2000684, PUAEN2000942, PUAEN2001260, PUAEN2001345, PUAEN2001526, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005588, PUAEN2005930, PUAEN2006029, PUAEN2006328, PUAEN2006335, PUAEN2006578, PUAEN2006639, PUAEN2006761, PUAEN2007350, PUAEN2007785, PUAEN2007898, PUAEN2008123, PUAEN2008228, PUAEN2008314, PUAEN2008515,
PUAEN2008815, PUAEN2008939, PUAEN2008980, PUAEN2009174, PUAEN2009348, PUAEN2009534, PUAEN2009655, PUAEN2009795, PUAEN2009852, PUAEN2010346, PUAEN2010824, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000065, SALGL1000171, SKMUS1000030, SKMUS1000064, SKMUS1000067, SKMUS1000102, SKMUS1000104, SKMUS1000110, SKMUS1000118, SKMUS1000124, SKMUS1000129, SKMUS1000177, SKMUS2000020, SKMUS2000061, SKMUS2000074, SKMUS2000078, SKMUS2000117, SKMUS2000137, SKMUS2000198, SKMUS2000271, SKMUS2000287, SKMUS2000317,
SKMUS2000330, SKMUS2000339, SKMUS2000343, SKMUS2000365, SKMUS2000380, SKMUS2000390, SKMUS2000416, SKMUS2000458, SKMUS2000467, SKMUS2000468, SKMUS2000515, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000945, SKMUS2000973, SKMUS2000980, SKMUS2001008, SKMUS2001014, SKMUS2001129, SKMUS2001157, SKMUS2001164, SKMUS2001199, SKMUS2001201, SKMUS2001215, SKMUS2001247, SKMUS2001254, SKMUS2001271, SKMUS2001287, SKMUS2001364, SKMUS2001398, SKMUS2001402, SKMUS2001412, SKMUS2001492, SKMUS2001501, SKMUS2001543, SKMUS2001580, SKMUS2001585,
SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003744, SKMUS2004047, SKMUS2004483, SKMUS2004667, SKMUS2004897, SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232,
SKMUS2009479, SKMUS2009557, SKNMC1000110, SKNMC1000123, SKNMC1000124, SKNMC1000229, SKNMC1000251, SKNMC1000264, SKNMC2000224, SKNMC2000256, SKNMC2000356, SKNMC2000374, SKNMC2000583, SKNMC2000593, SKNMC2000612, SKNMC2000622, SKNMC2000698, SKNMC2000966, SKNMC2001113, SKNMC2001503, SKNMC2001555, SKNMC2001596, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000174, SKNSH1000301, SKNSH1000308, SKNSH1000416, SKNSH2000051, SKNSH2000101, SKNSH2000163, SKNSH2000250, SKNSH2000319, SKNSH2000482,
SKNSH2000516, SKNSH2000550, SKNSH2000716, SKNSH2000971, SKNSH2001222, SKNSH2001875, SKNSH2001931, SKNSH2002054, SKNSH2002325, SKNSH2002768, SKNSH2002866, SKNSH2003064, SKNSH2003174, SKNSH2003422, SKNSH2003466, SKNSH2003483, SKNSH2003528, SKNSH2004039, SKNSH2005061, SKNSH2005120, SKNSH2005194, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000039, SMINT1000042, SMINT1000048, SMINT1000051, SMINT1000054, SMINT1000071, SMINT1000075, SMINT1000078, SMINT1000091,
SMINT1000100, SMINT1000103, SMINT1000118, SMINT1000131, SMINT1000137, SMINT1000192, SMINT2000007, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000396, SMINT2000400, SMINT2000468, SMINT2000518, SMINT2000529, SMINT2000530, SMINT2000537, SMINT2000538, SMINT2000541, SMINT2000558, SMINT2000562, SMINT2000568, SMINT2000602, SMINT2000609, SMINT2000747, SMINT2000811, SMINT2000824, SMINT2000984, SMINT2001114, SMINT2001158, SMINT2001183, SMINT2001199, SMINT2001222, SMINT2001348, SMINT2001397, SMINT2001458, SMINT2001461, SMINT2001535,
SMINT2001559, SMINT2001609, SMINT2001615, SMINT2001683, SMINT2001731, SMINT2001950, SMINT2002126, SMINT2002159, SMINT2002202, SMINT2002210, SMINT2002224, SMINT2002281, SMINT2002311, SMINT2002328, SMINT2002414, SMINT2002457, SMINT2002620, SMINT2002743, SMINT2002778, SMINT2002880, SMINT2002884, SMINT2002899, SMINT2002974, SMINT2002976, SMINT2003003, SMINT2003128, SMINT2003150, SMINT2003317, SMINT2003340, SMINT2003386, SMINT2003423, SMINT2003505, SMINT2003551, SMINT2003569, SMINT2003641, SMINT2003644, SMINT2003866, SMINT2004023, SMINT2004037, SMINT2004047, SMINT2004086, SMINT2004144, SMINT2004280, SMINT2004339, SMINT2004350, SMINT2004414, SMINT2004473, SMINT2004547, SMINT2004583, SMINT2004729,
SMINT2004781, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007790, SMINT2007792, SMINT2007796, SMINT2007867, SMINT2007932, SMINT2008042, SMINT2008054, SMINT2008133, SMINT2008277, SMINT2008329,
SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009233, SMINT2009259, SMINT2009272, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010672, SMINT2010753, SMINT2010853, SMINT2010897, SMINT2010959,
SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012179, SMINT2012195, SMINT2012285, SMINT2012291, SMINT2012501, SMINT2012734, SMINT2012793, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014489, SMINT2014721, SMINT2015006, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016440, SMINT2016477,
SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189,
SPLEN2000197, SPLEN2000205, SPLEN2000210, SPLEN2000222, SPLEN2000242, SPLEN2000243, SPLEN2000247, SPLEN2000258, SPLEN2000270, SPLEN2000307, SPLEN2000310, SPLEN2000341, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000537, SPLEN2000541, SPLEN2000607, SPLEN2000630, SPLEN2000695, SPLEN2000698, SPLEN2001135, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001626, SPLEN2001650, SPLEN2001689, SPLEN2001710, SPLEN2001781, SPLEN2001881, SPLEN2001912, SPLEN2001945,
SPLEN2002007, SPLEN2002053, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002463, SPLEN2002467, SPLEN2002477, SPLEN2002493, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004343, SPLEN2004346, SPLEN2004368, SPLEN2004611, SPLEN2004662, SPLEN2004844, SPLEN2005009, SPLEN2005227, SPLEN2005272, SPLEN2005342, SPLEN2005416,
SPLEN2005450, SPLEN2005474, SPLEN2005560, SPLEN2005727, SPLEN2005783, SPLEN2005790, SPLEN2005806, SPLEN2005838, SPLEN2005927, SPLEN2005939, SPLEN2006143, SPLEN2006268, SPLEN2006283, SPLEN2006325, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007619, SPLEN2007653, SPLEN2007689, SPLEN2007695, SPLEN2007794, SPLEN2007879, SPLEN2007926, SPLEN2007951, SPLEN2008007, SPLEN2008135, SPLEN2008164, SPLEN2008317, SPLEN2008496, SPLEN2008786, SPLEN2009088, SPLEN2009163, SPLEN2009315, SPLEN2009340, SPLEN2009512, SPLEN2009513, SPLEN2009541, SPLEN2009548, SPLEN2009581, SPLEN2009733,
SPLEN2009803, SPLEN2009884, SPLEN2009918, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453,
SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014645, SPLEN2014669, SPLEN2014711, SPLEN2014739, SPLEN2014860, SPLEN2014865, SPLEN2014919,
SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016480, SPLEN2016531, SPLEN2016533, SPLEN2016541, SPLEN2016552, SPLEN2016602, SPLEN2016627, SPLEN2016678, SPLEN2016743, SPLEN2016773, SPLEN2016781, SPLEN2016840, SPLEN2016863,
SPLEN2016922, SPLEN2016932, SPLEN2016973, SPLEN2017104, SPLEN2017147, SPLEN2017187, SPLEN2017189, SPLEN2017212, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019257, SPLEN2019311, SPLEN2019323, SPLEN2019349, SPLEN2019375, SPLEN2019379, SPLEN2019446,
SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920,
SPLEN2023024, SPLEN2023118, SPLEN2023423, SPLEN2023710, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522,
SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036103, SPLEN2036326, SPLEN2036475,
SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038407, SPLEN2038473, SPLEN2039311, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042598, SPLEN2042714, SPLEN2042920, STOMA1000013, STOMA1000047, STOMA1000052, STOMA1000074, STOMA1000091, STOMA1000117, STOMA1000133,
STOMA1000175, STOMA1000186, STOMA1000190, STOMA1000191, STOMA2000032, STOMA2000072, STOMA2000088, STOMA2000104, STOMA2000148, STOMA2000168, STOMA2000183, STOMA2000188, STOMA2000279, STOMA2000383, STOMA2000386, STOMA2000478, STOMA2000482, STOMA2000529, STOMA2000539, STOMA2000567, STOMA2000640, STOMA2000678, STOMA2000686, STOMA2000888, STOMA2001025, STOMA2002141, STOMA2002367, STOMA2002688, STOMA2003158, STOMA2003289, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004925, STOMA2005120, STOMA2005664, STOMA2005667, STOMA2005746, STOMA2006181, STOMA2006213, STOMA2006325,
STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008838, STOMA2009250, STOMA2009253, STOMA2009256, STOMA2009289, THYMU1000010, THYMU1000017, THYMU1000025, THYMU1000029, THYMU1000032, THYMU1000033, THYMU1000041, THYMU1000060, THYMU1000074, THYMU1000098, THYMU1000103, THYMU1000105, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000336, THYMU1000366, THYMU1000382, THYMU1000393, THYMU1000394, THYMU1000399, THYMU1000426,
THYMU1000440, THYMU1000459, THYMU1000473, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879,
THYMU2000932, THYMU2000950, THYMU2000971, THYMU2000982, THYMU2000990, THYMU2001018, THYMU2001053, THYMU2001071, THYMU2001139, THYMU2001195, THYMU2001202, THYMU2001203, THYMU2001256, THYMU2001270, THYMU2001291, THYMU2001314, THYMU2001325, THYMU2001357, THYMU2001422, THYMU2001443, THYMU2001515, THYMU2001521, THYMU2001556, THYMU2001622, THYMU2001727, THYMU2001819, THYMU2001825, THYMU2001868, THYMU2001898, THYMU2001900, THYMU2001916, THYMU2001989, THYMU2002023, THYMU2002037, THYMU2002080, THYMU2002109, THYMU2002147, THYMU2002154, THYMU2002157, THYMU2002177, THYMU2002203, THYMU2002229, THYMU2002242, THYMU2002338, THYMU2002356, THYMU2002376, THYMU2002384, THYMU2002450, THYMU2002490, THYMU2002548,
THYMU2002695, THYMU2002745, THYMU2002756, THYMU2002789, THYMU2002792, THYMU2002815, THYMU2002852, THYMU2002910, THYMU2002950, THYMU2002983, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003542, THYMU2003632, THYMU2003650, THYMU2003700, THYMU2003760, THYMU2003803, THYMU2003855, THYMU2003907, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004452, THYMU2004508, THYMU2004512,
THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005480, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005997, THYMU2006001, THYMU2006048, THYMU2006098, THYMU2006160, THYMU2006170, THYMU2006252, THYMU2006277, THYMU2006303, THYMU2006324, THYMU2006350,
THYMU2006357, THYMU2006365, THYMU2006420, THYMU2006468, THYMU2006505, THYMU2006610, THYMU2006666, THYMU2006712, THYMU2006913, THYMU2006946, THYMU2006965, THYMU2007025, THYMU2007036, THYMU2007095, THYMU2007179, THYMU2007258, THYMU2007307, THYMU2007308, THYMU2007339, THYMU2007350, THYMU2007384, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226,
THYMU2008282, THYMU2008339, THYMU2008350, THYMU2008351, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010161, THYMU2010192, THYMU2010519, THYMU2010637, THYMU2010671, THYMU2010699, THYMU2010705,
THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012073, THYMU2012104, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807,
THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015775, THYMU2015825, THYMU2015918, THYMU2015943, THYMU2015984,
THYMU2016113, THYMU2016164, THYMU2016204, THYMU2016219, THYMU2016360, THYMU2016496, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017675, THYMU2017707, THYMU2017844, THYMU2017878, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587,
THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350,
THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031579, THYMU2031847, THYMU2031890, THYMU2031994, THYMU2032014, THYMU2032035,
THYMU2032080, THYMU2032150, THYMU2032358, THYMU2032437, THYMU2032601, THYMU2032655, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2036105, THYMU2036207, THYMU2036215, THYMU2036252, THYMU2036265, THYMU2036366, THYMU2036459, THYMU2036461, THYMU2036604, THYMU2036653,
THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360,
THYMU3000390, THYMU3000420, THYMU3000490, THYMU3000504, THYMU3000542, THYMU3000655, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003350, THYMU3003403, THYMU3003565, THYMU3003763, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371,
THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008935, THYMU3009006, THYMU3009255, THYMU3009391, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571,
THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000,
THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032867, THYMU3033402, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192,
THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779,
THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078, TKIDN2003366, TKIDN2003396, TKIDN2003413, TKIDN2003597, TKIDN2003601,
TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015788, TKIDN2016309, TKIDN2016399, TKIDN2016846,
TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221,
TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000057, TRACH1000074, TRACH1000100, TRACH1000106, TRACH1000125, TRACH1000140, TRACH1000144, TRACH1000181, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235, TRACH2000243, TRACH2000250, TRACH2000259, TRACH2000272, TRACH2000282, TRACH2000287, TRACH2000289, TRACH2000312,
TRACH2000336, TRACH2000393, TRACH2000411, TRACH2000472, TRACH2000496, TRACH2000497, TRACH2000502, TRACH2000522, TRACH2000559, TRACH2000660, TRACH2000665, TRACH2000675, TRACH2000677, TRACH2000703, TRACH2000767, TRACH2000775, TRACH2000807, TRACH2000873, TRACH2000894, TRACH2000898, TRACH2000923, TRACH2000944, TRACH2000981, TRACH2000986, TRACH2001037, TRACH2001060, TRACH2001085, TRACH2001109, TRACH2001121, TRACH2001147, TRACH2001154, TRACH2001188, TRACH2001275, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001463, TRACH2001474, TRACH2001523, TRACH2001525, TRACH2001532, TRACH2001549,
TRACH2001592, TRACH2001596, TRACH2001621, TRACH2001646, TRACH2001651, TRACH2001684, TRACH2001898, TRACH2001933, TRACH2001968, TRACH2001996, TRACH2002043, TRACH2002054, TRACH2002092, TRACH2002100, TRACH2002138, TRACH2002333, TRACH2002784, TRACH2002840, TRACH2002847, TRACH2002853, TRACH2002862, TRACH2002954, TRACH2002960, TRACH2002988, TRACH2003070, TRACH2003272, TRACH2003347, TRACH2003484, TRACH2003516, TRACH2003605, TRACH2003736, TRACH2003759, TRACH2003941, TRACH2004054, TRACH2004087, TRACH2004109, TRACH2004170, TRACH2004183, TRACH2004251, TRACH2004292, TRACH2004399, TRACH2004428, TRACH2004458, TRACH2004499, TRACH2004521, TRACH2004570, TRACH2004845, TRACH2004887, TRACH2004950, TRACH2005004,
TRACH2005017, TRACH2005066, TRACH2005159, TRACH2005314, TRACH2005444, TRACH2005666, TRACH2005698, TRACH2005710, TRACH2005720, TRACH2005769, TRACH2005796, TRACH2006015, TRACH2006049, TRACH2006097, TRACH2006194, TRACH2006327, TRACH2006331, TRACH2006387, TRACH2006528, TRACH2006670, TRACH2006762, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894, TRACH2007988, TRACH2008112, TRACH2008113, TRACH2008127, TRACH2008130, TRACH2008278, TRACH2008478, TRACH2008540, TRACH2008583, TRACH2008597, TRACH2008820, TRACH2008891, TRACH2008921, TRACH2009006,
TRACH2009060, TRACH2009123, TRACH2009268, TRACH2009291, TRACH2009348, TRACH2009358, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011290, TRACH2011293, TRACH2011302, TRACH2011500, TRACH2011574, TRACH2011894, TRACH2011956, TRACH2012000, TRACH2012138, TRACH2012242, TRACH2012370, TRACH2012497, TRACH2012536, TRACH2012562, TRACH2012742, TRACH2012823, TRACH2012918,
TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015939, TRACH2015987, TRACH2016080, TRACH2016131, TRACH2016286, TRACH2016317, TRACH2016347,
TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018512, TRACH2018646, TRACH2018663, TRACH2018718, TRACH2018835, TRACH2018882, TRACH2018914, TRACH2018950, TRACH2019024, TRACH2019046, TRACH2019151, TRACH2019154, TRACH2019176, TRACH2019248, TRACH2019309, TRACH2019425, TRACH2019473, TRACH2019602, TRACH2019661, TRACH2019672, TRACH2019673,
TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911,
TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003586, TRACH3003683, TRACH3003780, TRACH3003805, TRACH3004113, TRACH3004288, TRACH3004303, TRACH3004412, TRACH3004424, TRACH3004456,
TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005274, TRACH3005294, TRACH3005549, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007541, TRACH3007595, TRACH3007627, TRACH3007689,
TRACH3007766, TRACH3007866, TRACH3007995, TRACH3008042, TRACH3008061, TRACH3008201, TRACH3008535, TRACH3008632, TRACH3008688, TRACH3008697, TRACH3008713, TRACH3008887, TRACH3008902, TRACH3008990, TRACH3009001, TRACH3009059, TRACH3009061, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942,
TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019347,
TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823,
TRACH3025302, TRACH3025316, TRACH3025346, TRACH3025520, TRACH3026148, TRACH3026283, TRACH3026299, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660,
TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036997, TRACH3037063, TRACH3037267, TRACH3037288, TRACH3037505, TRACH3037573, TRACH3037696,
TRACH3037897, TRACH3038086, TRACH3038399, TSTOM1000135, TSTOM1000186, TSTOM2000235, TSTOM2000442, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000137, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000156, UMVEN1000186, UMVEN2000046, UMVEN2000069, UMVEN2000121, UMVEN2000354, UTERU1000008, UTERU1000015, UTERU1000024, UTERU1000031, UTERU1000032, UTERU1000057,
UTERU1000065, UTERU1000077, UTERU1000093, UTERU1000096, UTERU1000106, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000546, UTERU2000550, UTERU2000569, UTERU2000607, UTERU2000629, UTERU2000649, UTERU2000663, UTERU2000696, UTERU2000794, UTERU2000830, UTERU2000844,
UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002332, UTERU2002473, UTERU2002547, UTERU2002693, UTERU2002733, UTERU2002736, UTERU2002737, UTERU2002826, UTERU2002964, UTERU2003035, UTERU2003057, UTERU2003126, UTERU2003135, UTERU2003399, UTERU2003411, UTERU2003456, UTERU2003577, UTERU2003704, UTERU2003926, UTERU2003973, UTERU2004015, UTERU2004037, UTERU2004039, UTERU2004061, UTERU2004163, UTERU2004299, UTERU2004461, UTERU2004520, UTERU2004564, UTERU2004698, UTERU2004861, UTERU2004929, UTERU2005004, UTERU2005050,
UTERU2005179, UTERU2005346, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007253, UTERU2007267, UTERU2007499, UTERU2007639, UTERU2007724, UTERU2007924, UTERU2007942, UTERU2008018, UTERU2008019, UTERU2008027, UTERU2008040, UTERU2008077, UTERU2008130, UTERU2008347, UTERU2008426, UTERU2008516, UTERU2008561, UTERU2008653, UTERU2008705,
UTERU2008707, UTERU2008785, UTERU2008845, UTERU2008901, UTERU2008930, UTERU2008939, UTERU2009094, UTERU2009120, UTERU2009131, UTERU2009147, UTERU2009206, UTERU2009283, UTERU2009414, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031,
UTERU2012101, UTERU2012114, UTERU2012252, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013280, UTERU2013322, UTERU2013483, UTERU2013491, UTERU2013502, UTERU2013586, UTERU2013926, UTERU2013976, UTERU2014001, UTERU2014223, UTERU2014398, UTERU2014464, UTERU2014548, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015830, UTERU2015880,
UTERU2016147, UTERU2016157, UTERU2016464, UTERU2016669, UTERU2016757, UTERU2016761, UTERU2016799, UTERU2016896, UTERU2016902, UTERU2016979, UTERU2016981, UTERU2017123, UTERU2017303, UTERU2017421, UTERU2017492, UTERU2017613, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019163, UTERU2019257, UTERU2019453, UTERU2019491, UTERU2019534, UTERU2019681, UTERU2019706, UTERU2019710,
UTERU2019940, UTERU2019959, UTERU2019964, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941,
UTERU2028377, UTERU2028734, UTERU2029503, UTERU2029660, UTERU2029742, UTERU2029953, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623,
UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465,
UTERU3003495, UTERU3003523, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005205, UTERU3005230, UTERU3005264, UTERU3005422, UTERU3005460, UTERU3005536, UTERU3005585, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690,
UTERU3009775, UTERU3009979, UTERU3009987, UTERU3010029, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the THYMU, TKIDN, TLIVE, TLUNG, TOVAR, TRACH, and TSTOM libraries were analyzed for expression frequency. The analysis results are described below. Each is a relative value, where the expression level over all tissues is taken as 100. The larger this value, the greater the level of expression.

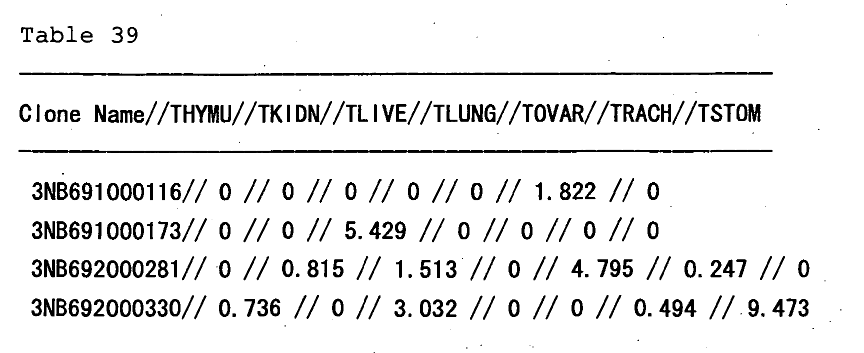

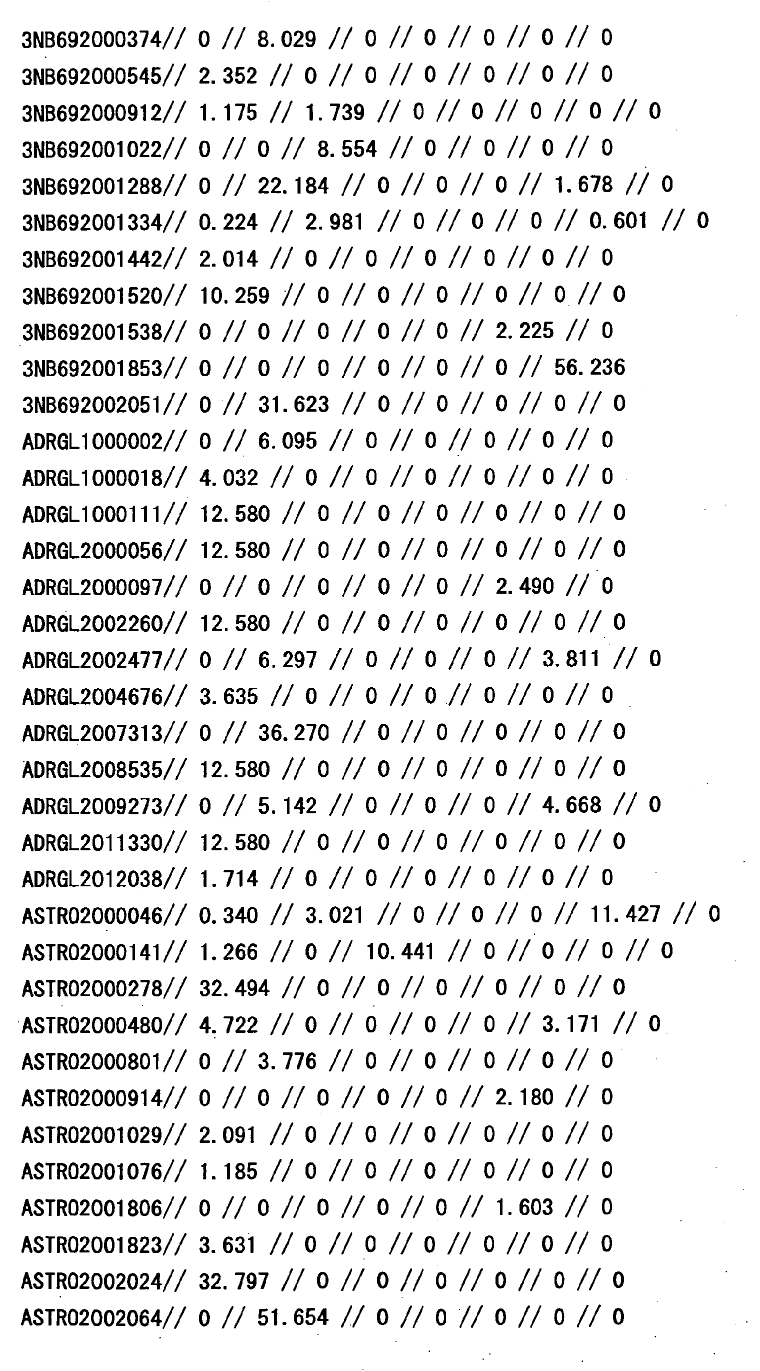

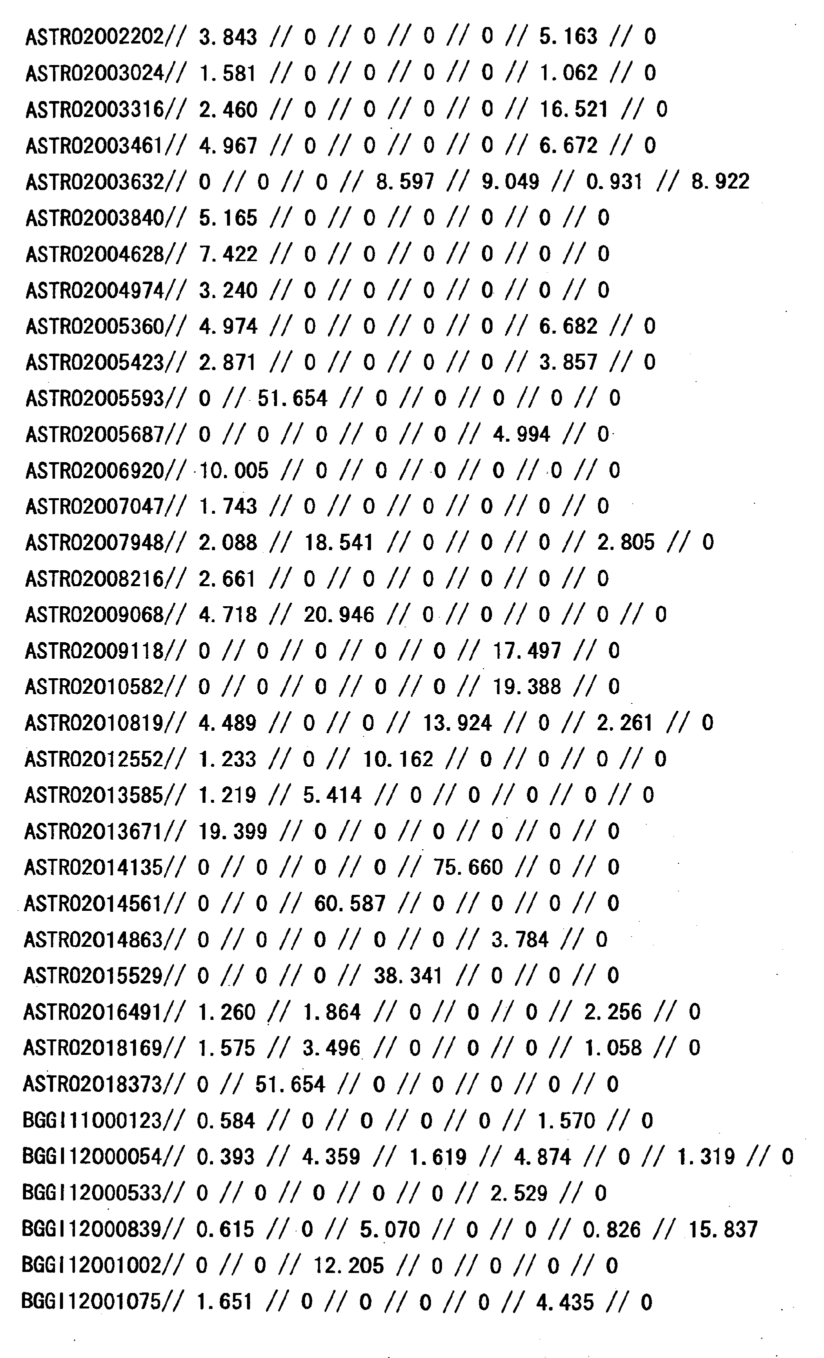

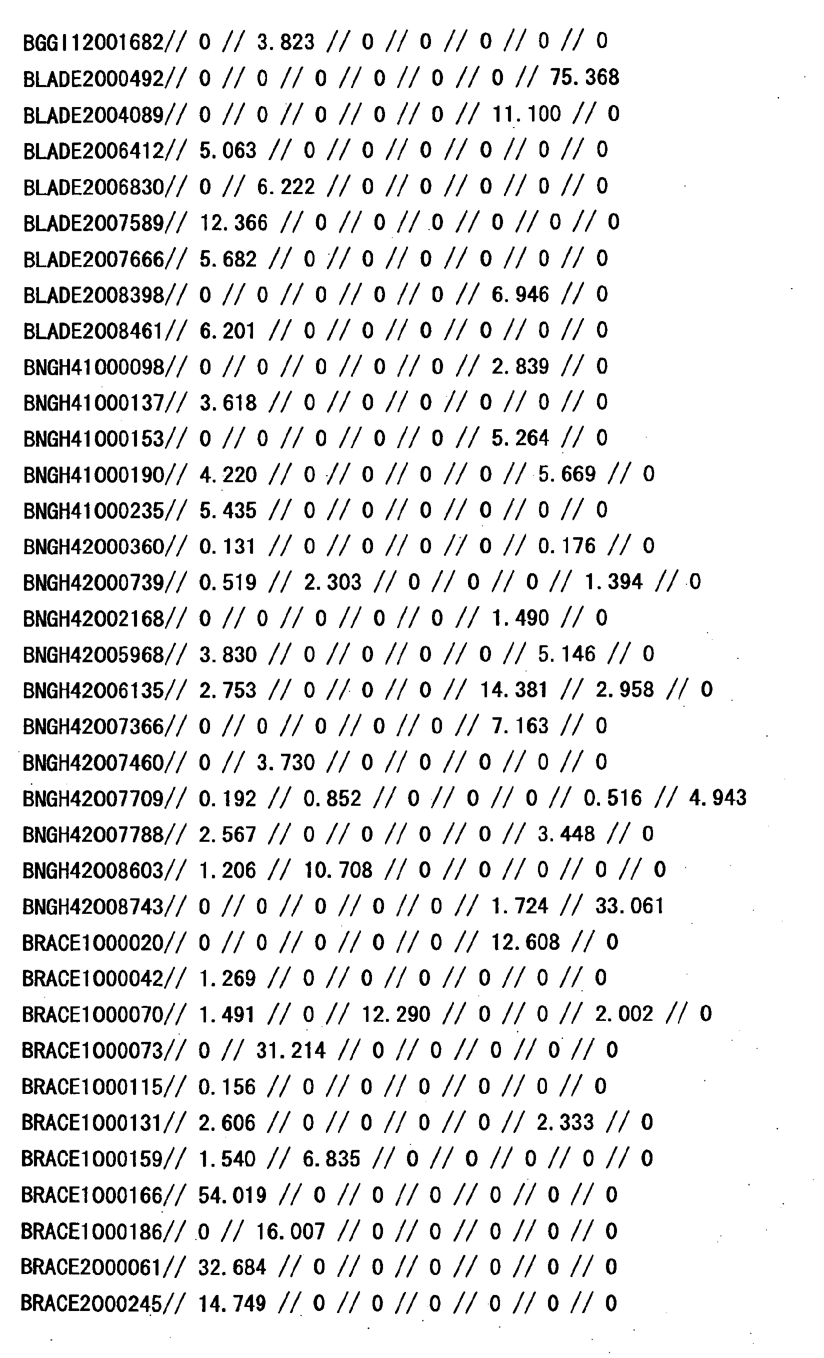

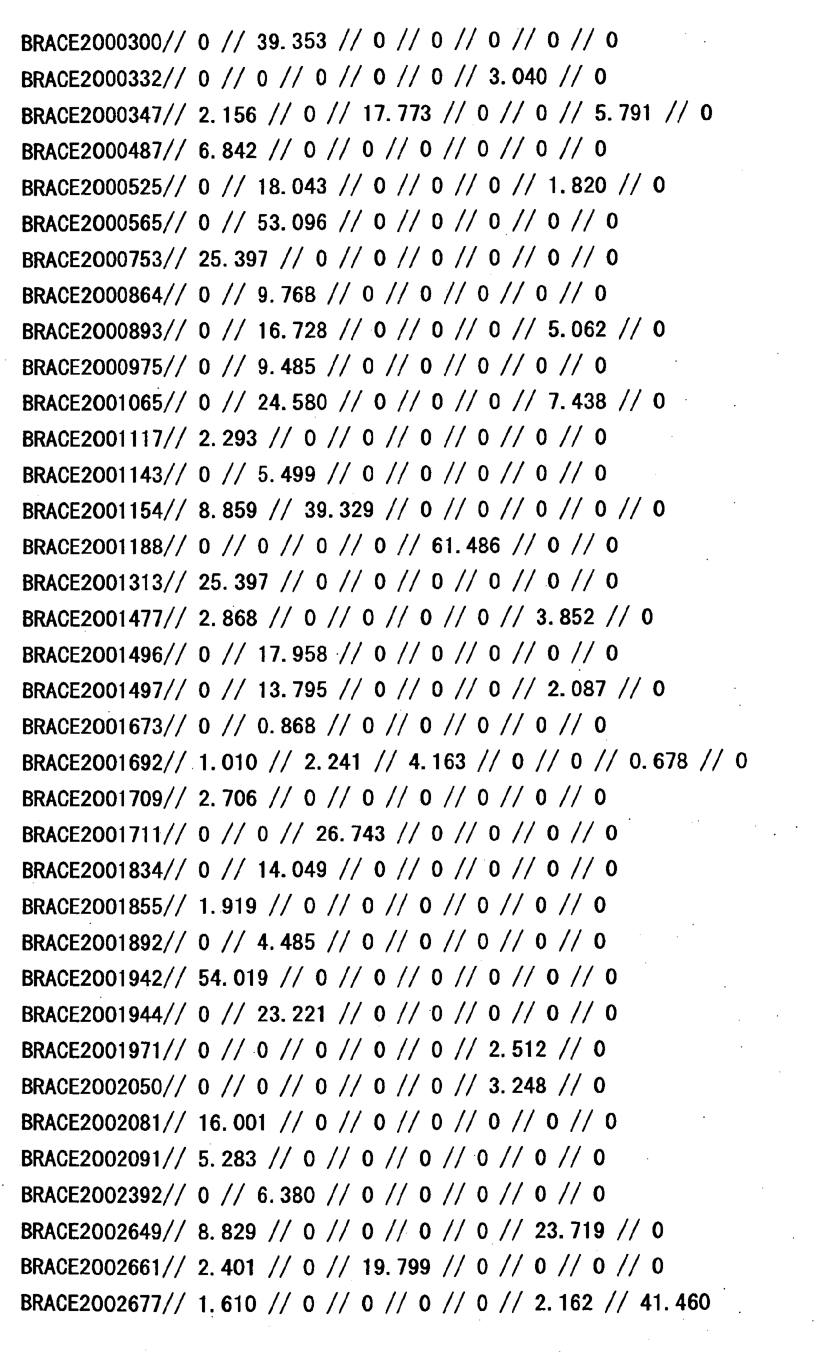

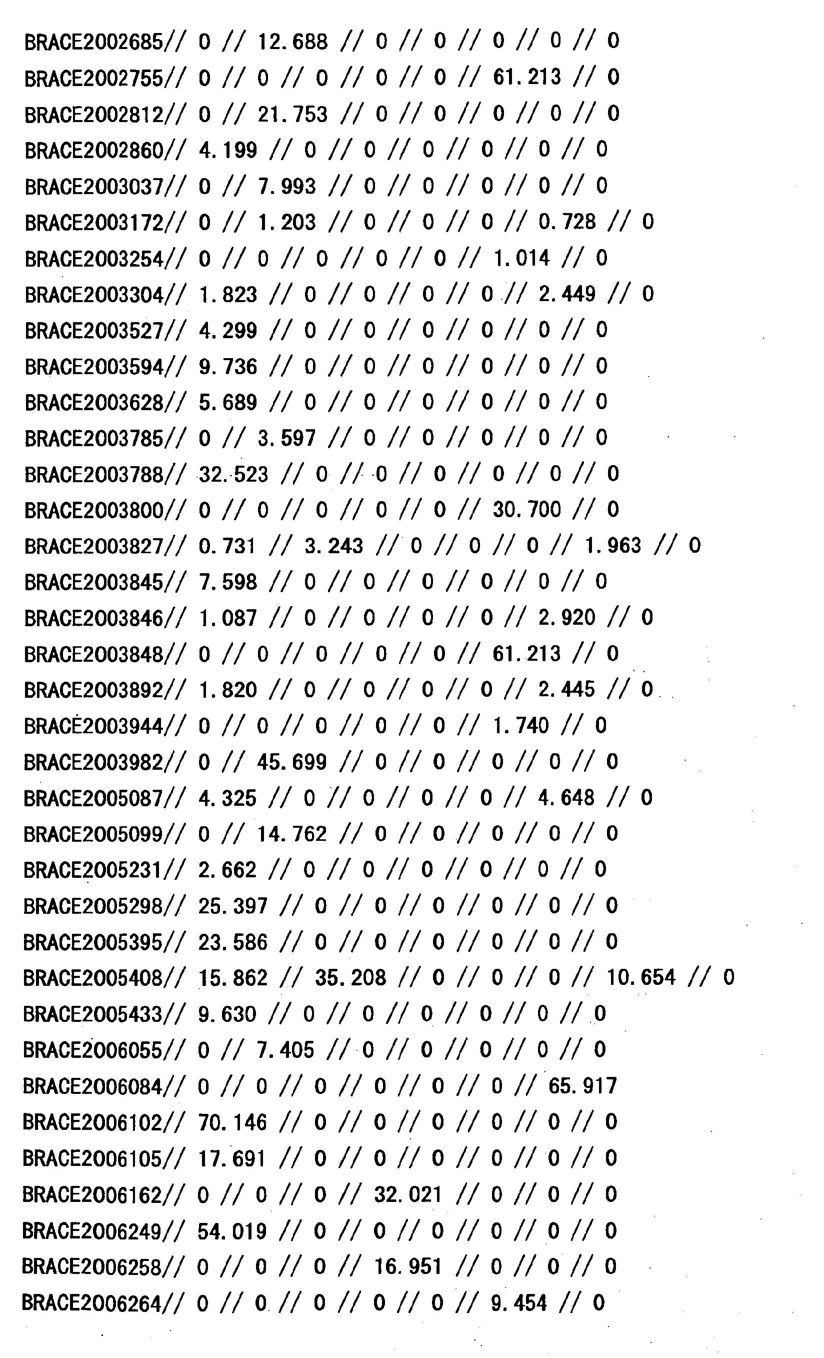

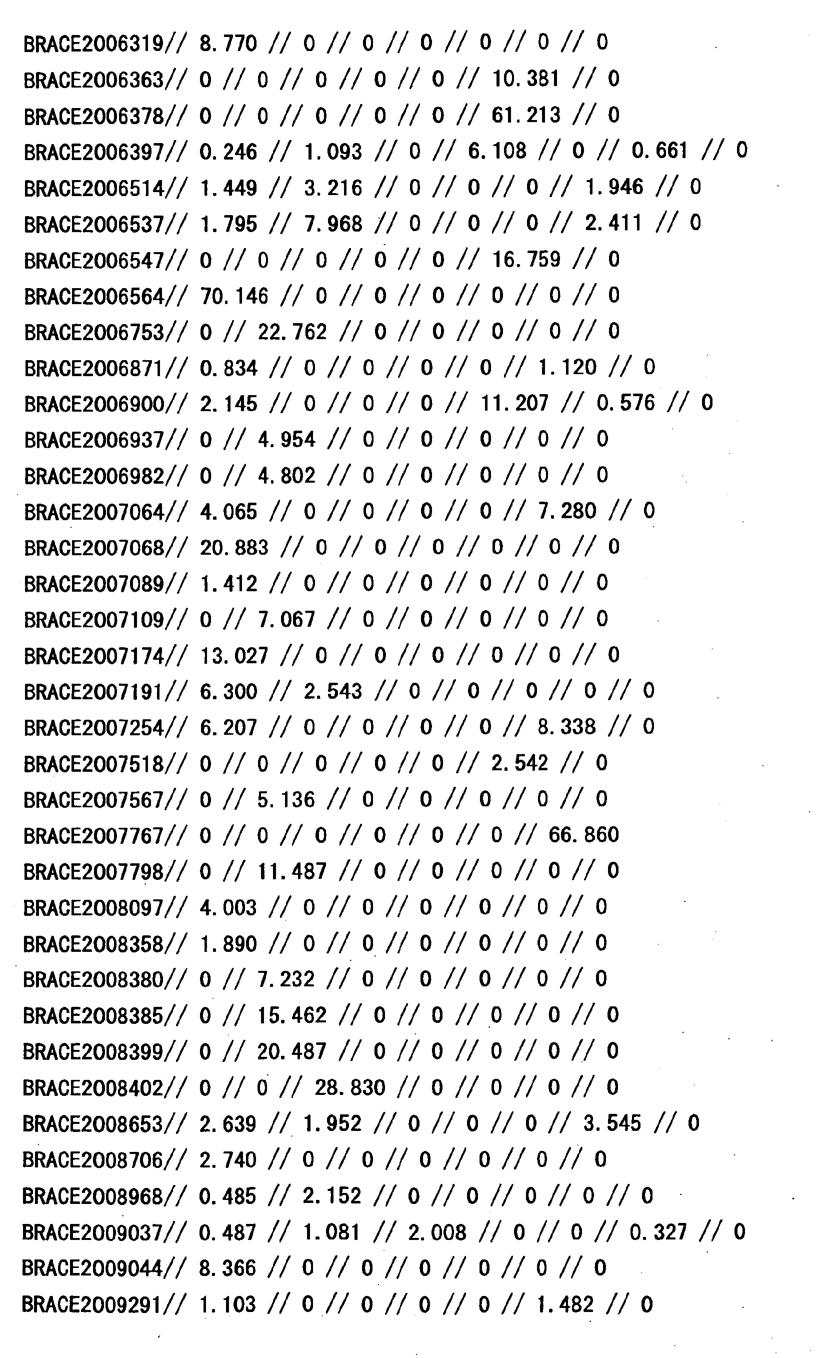

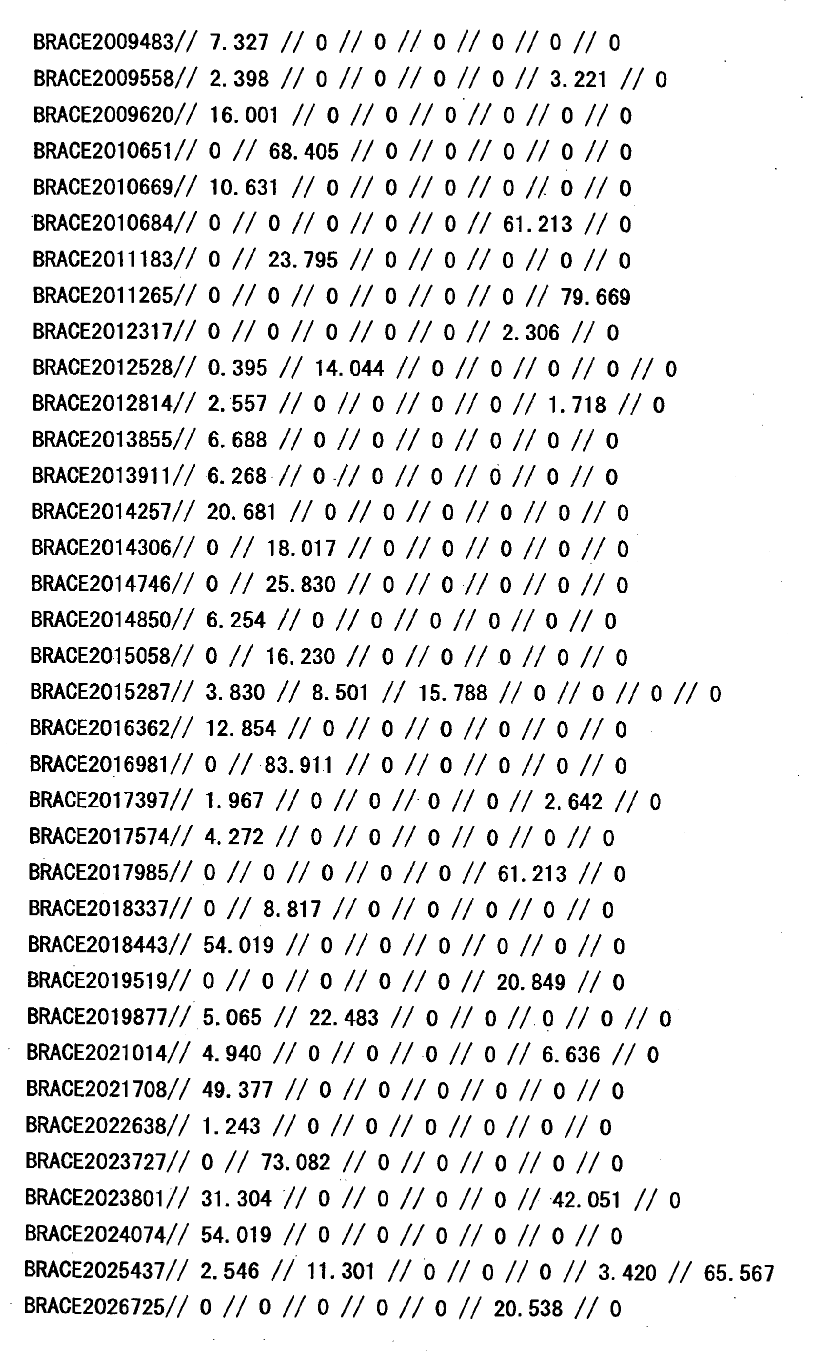

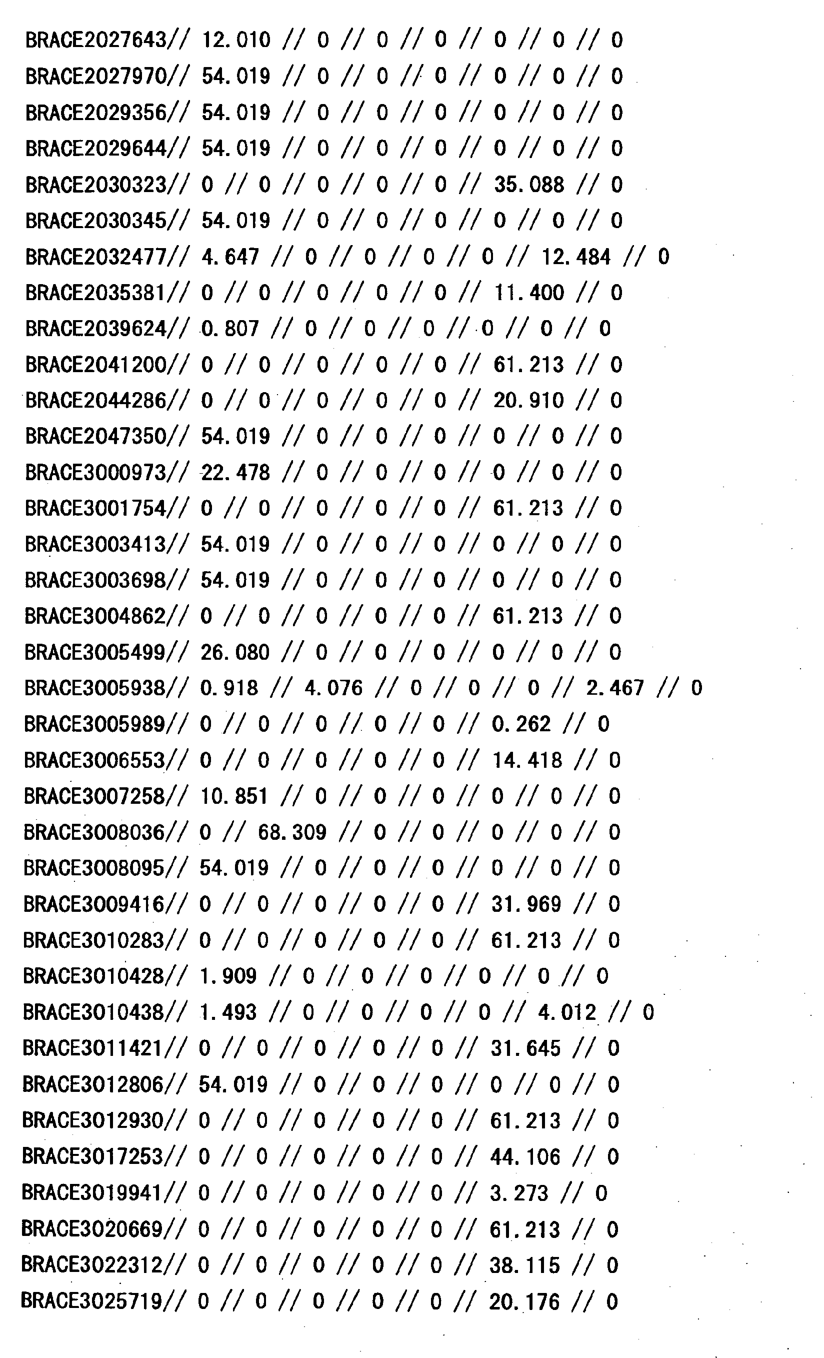

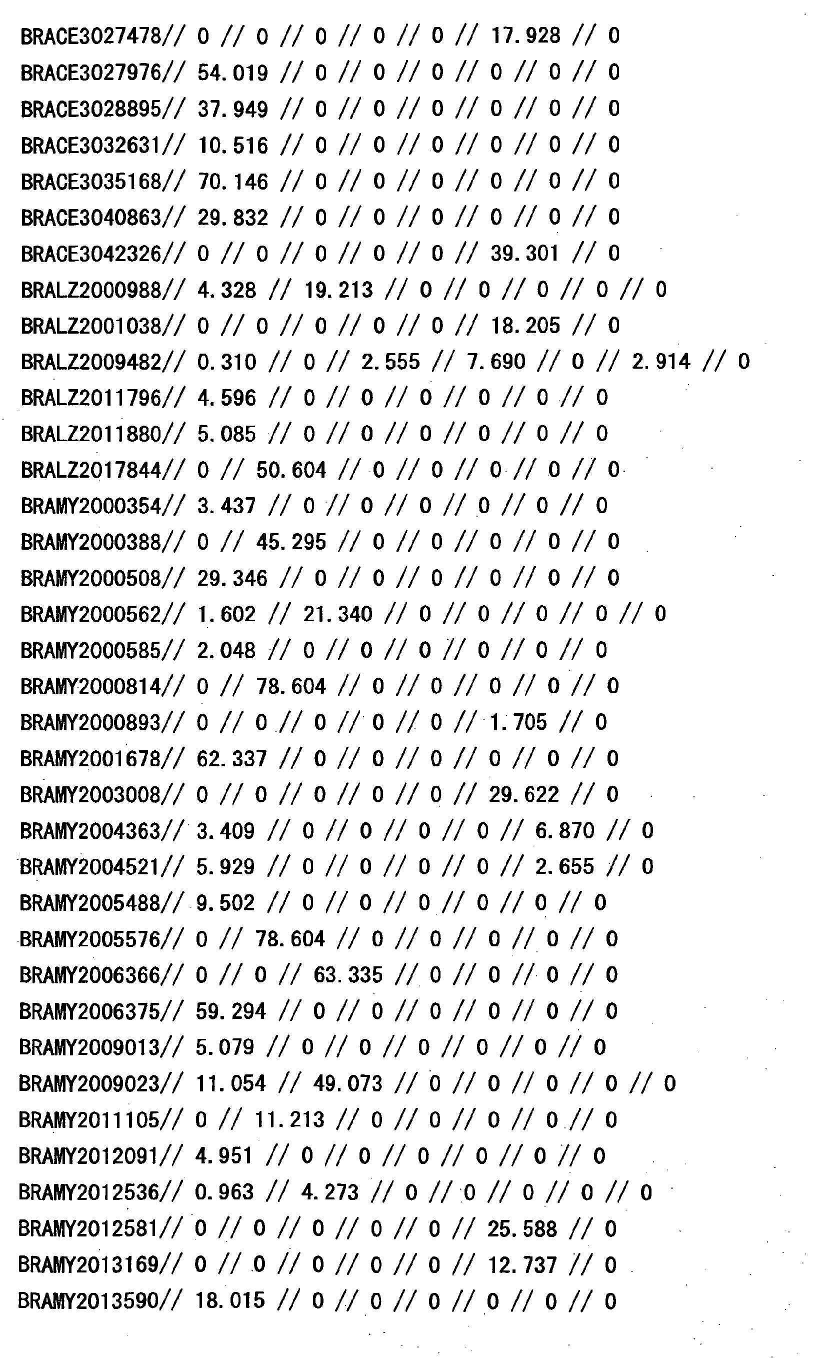

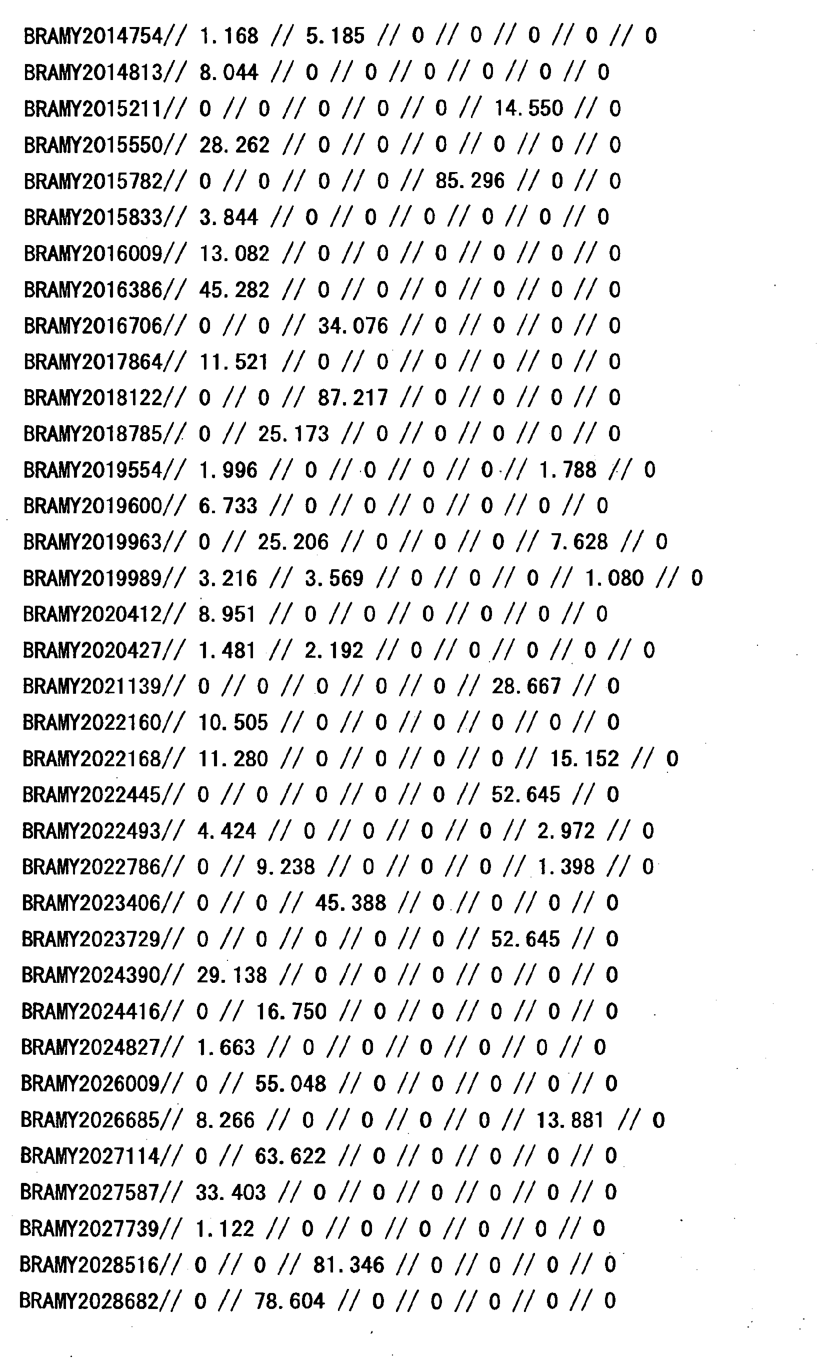

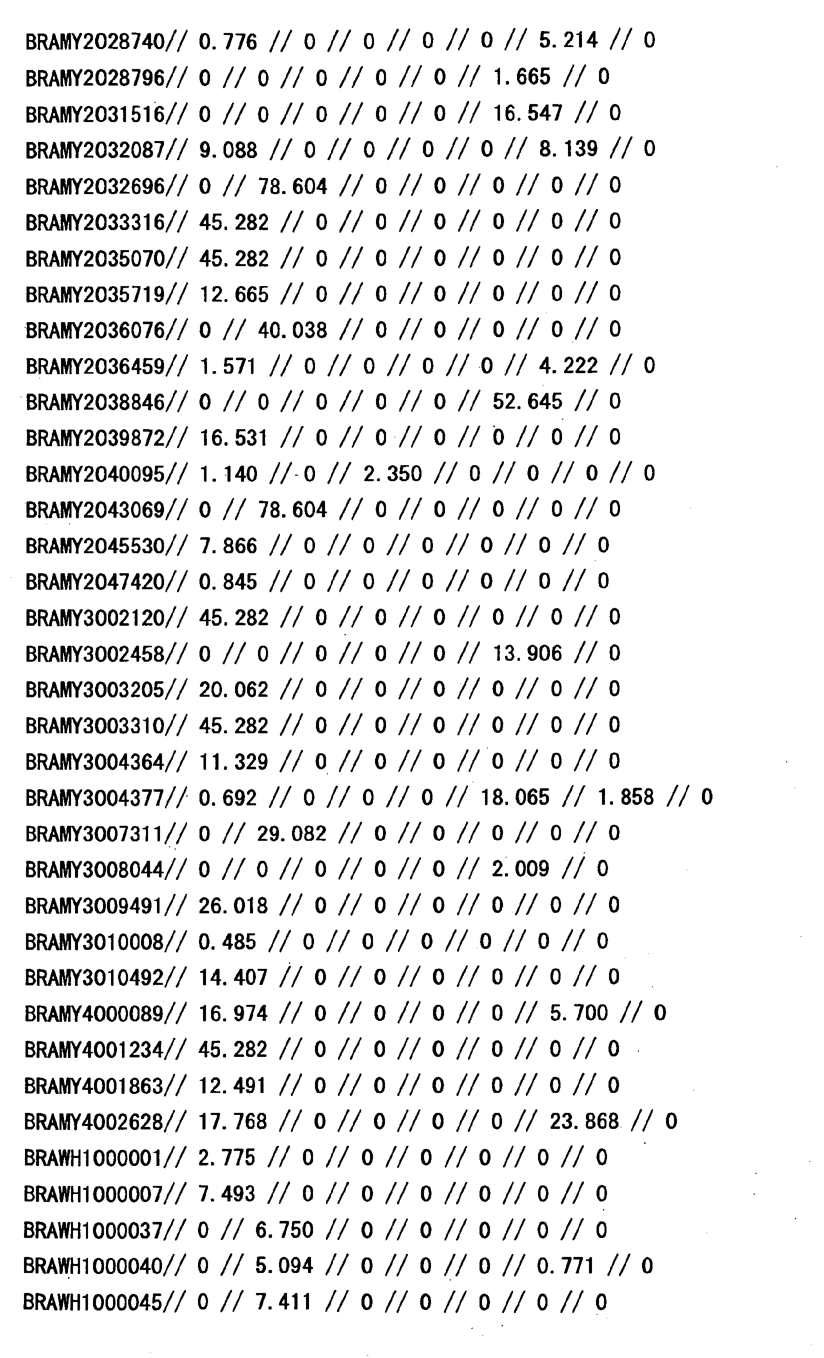

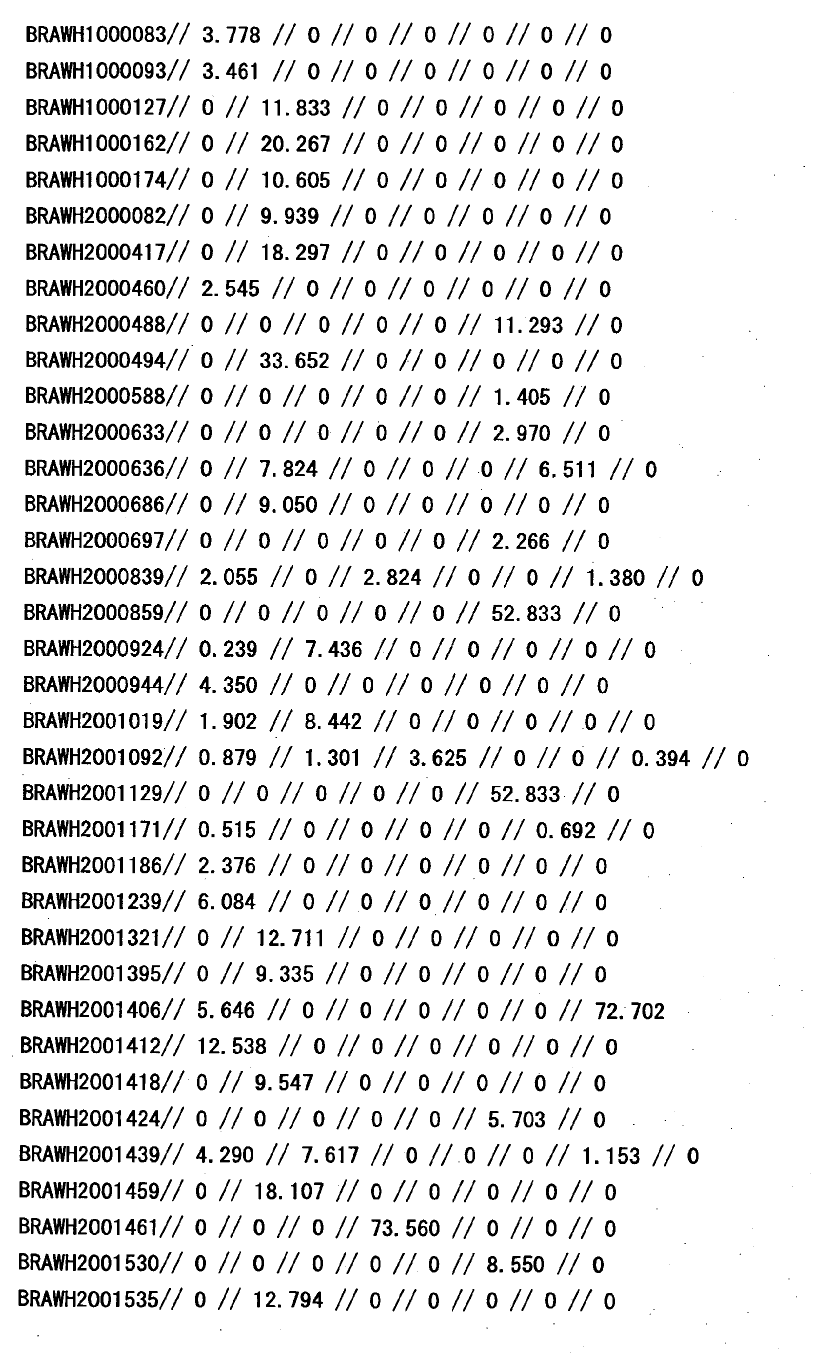

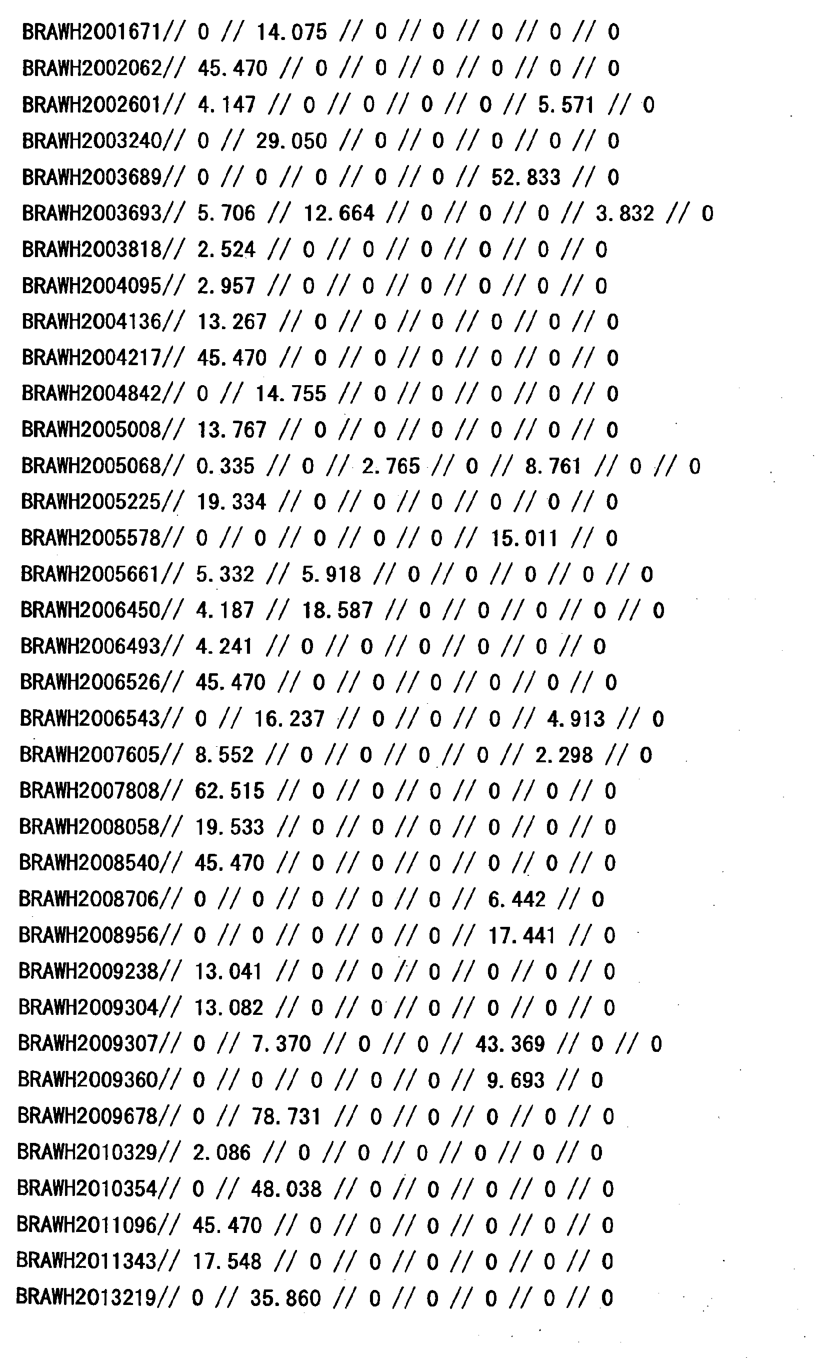

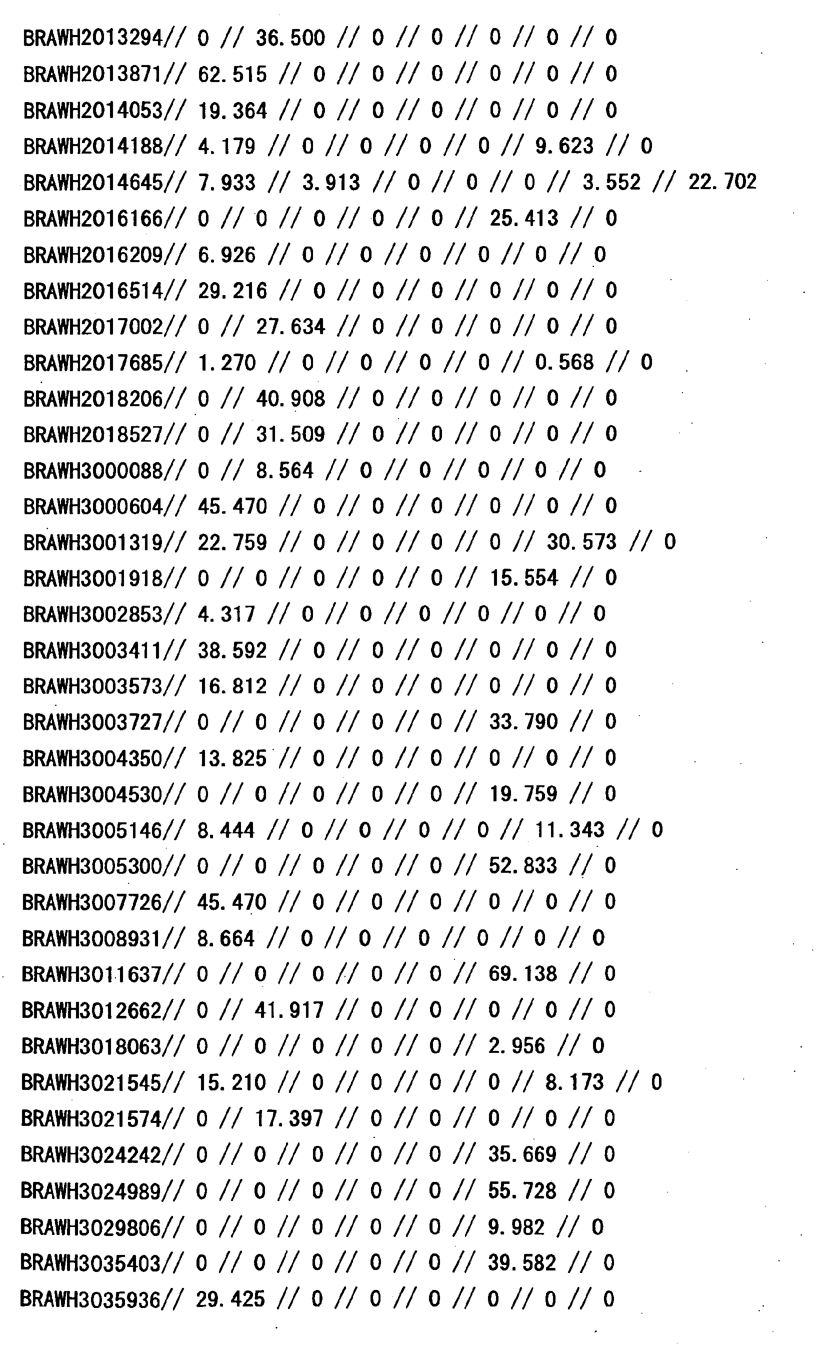

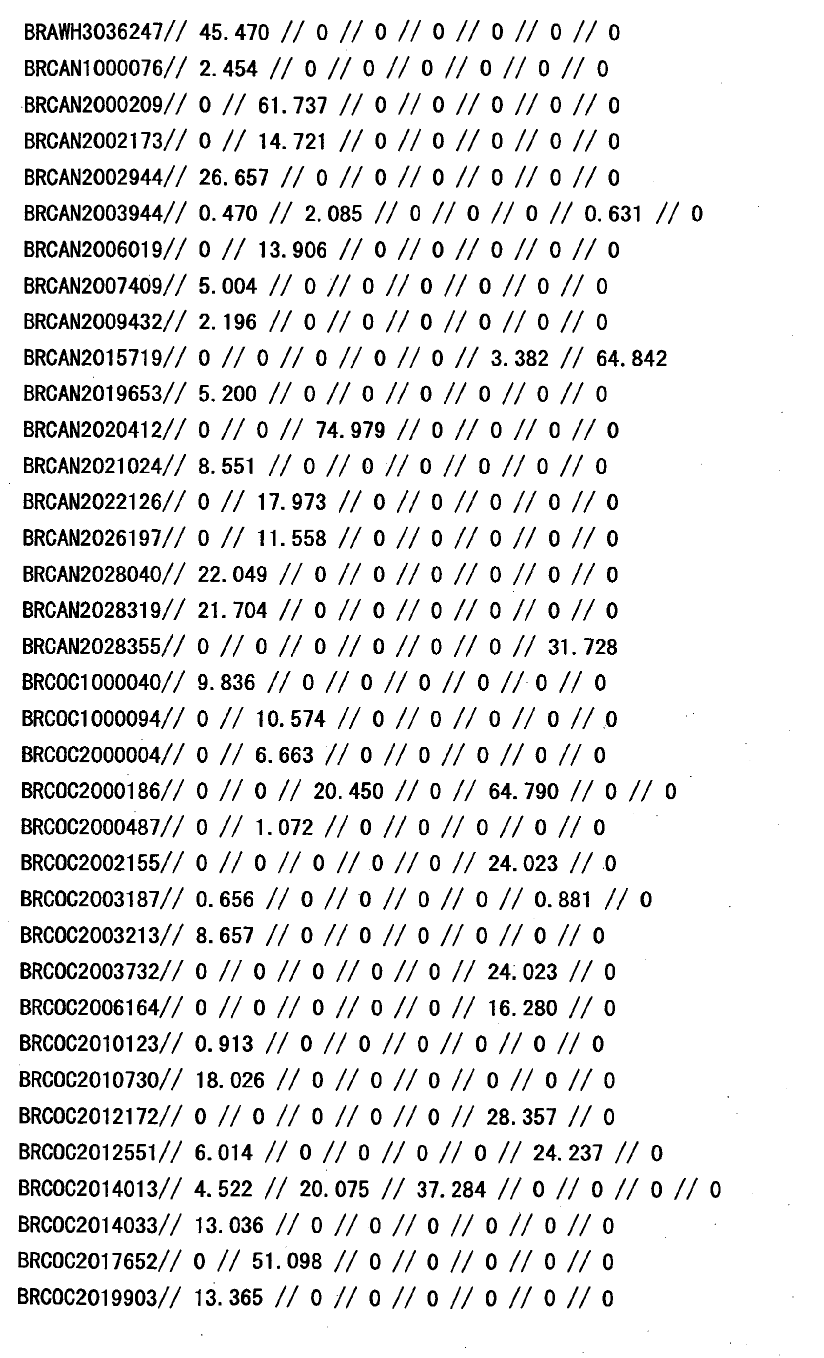

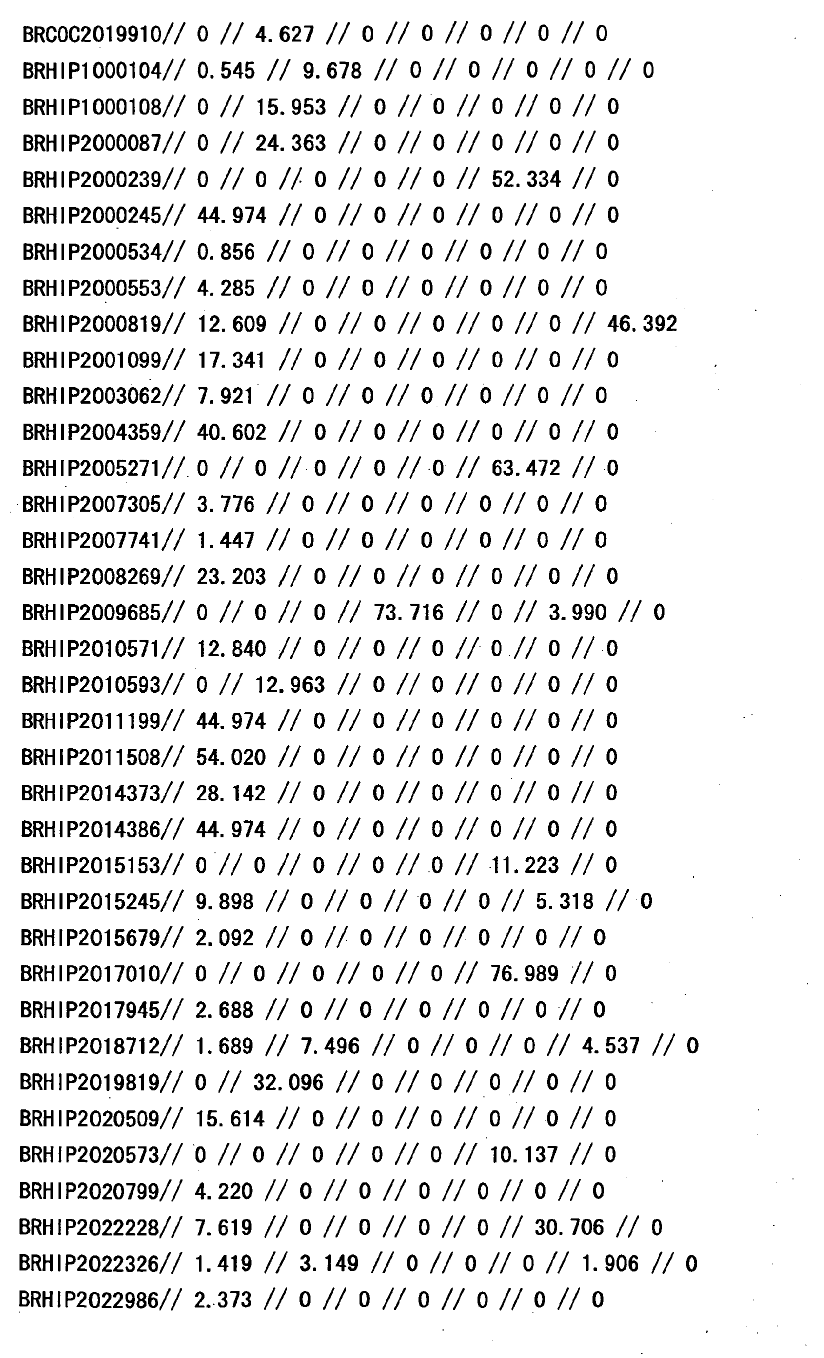

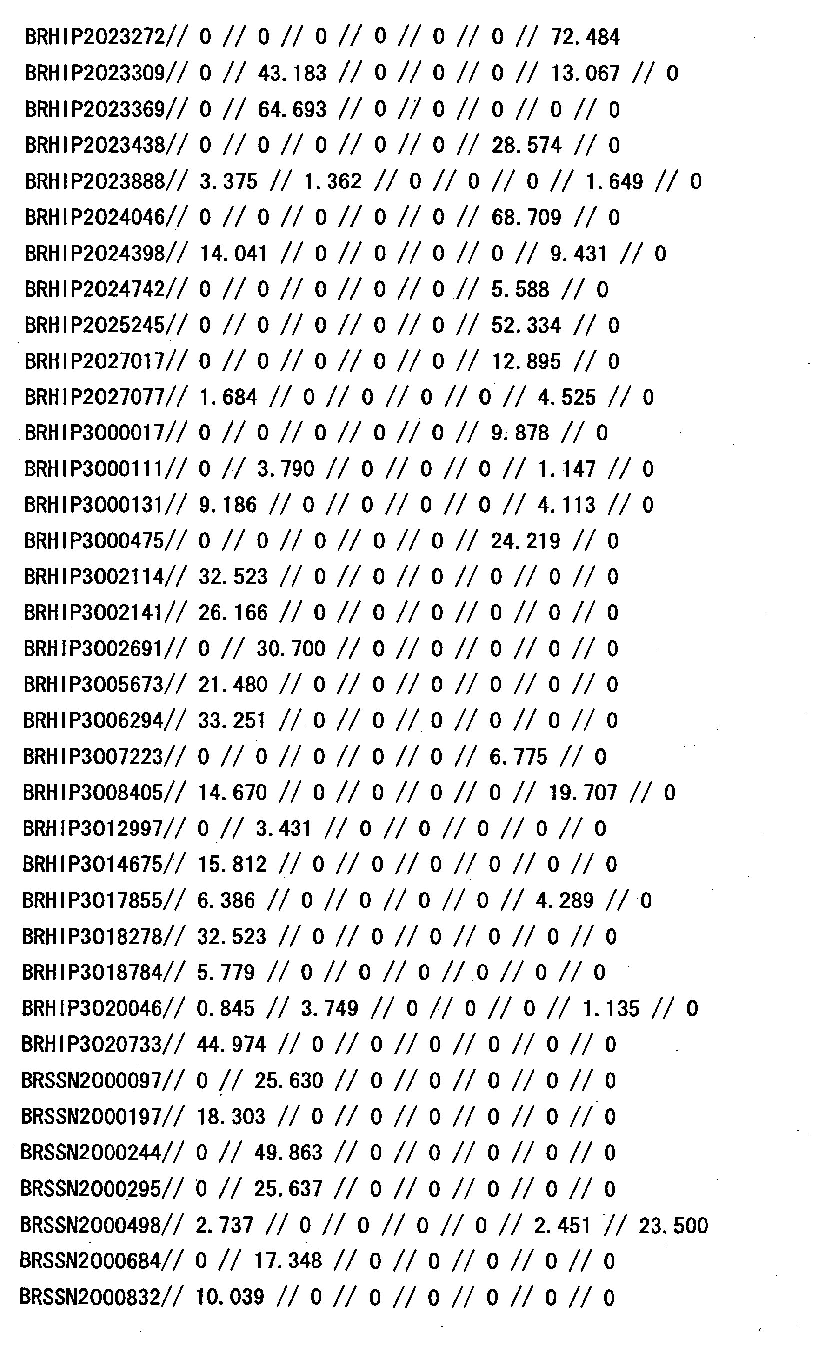

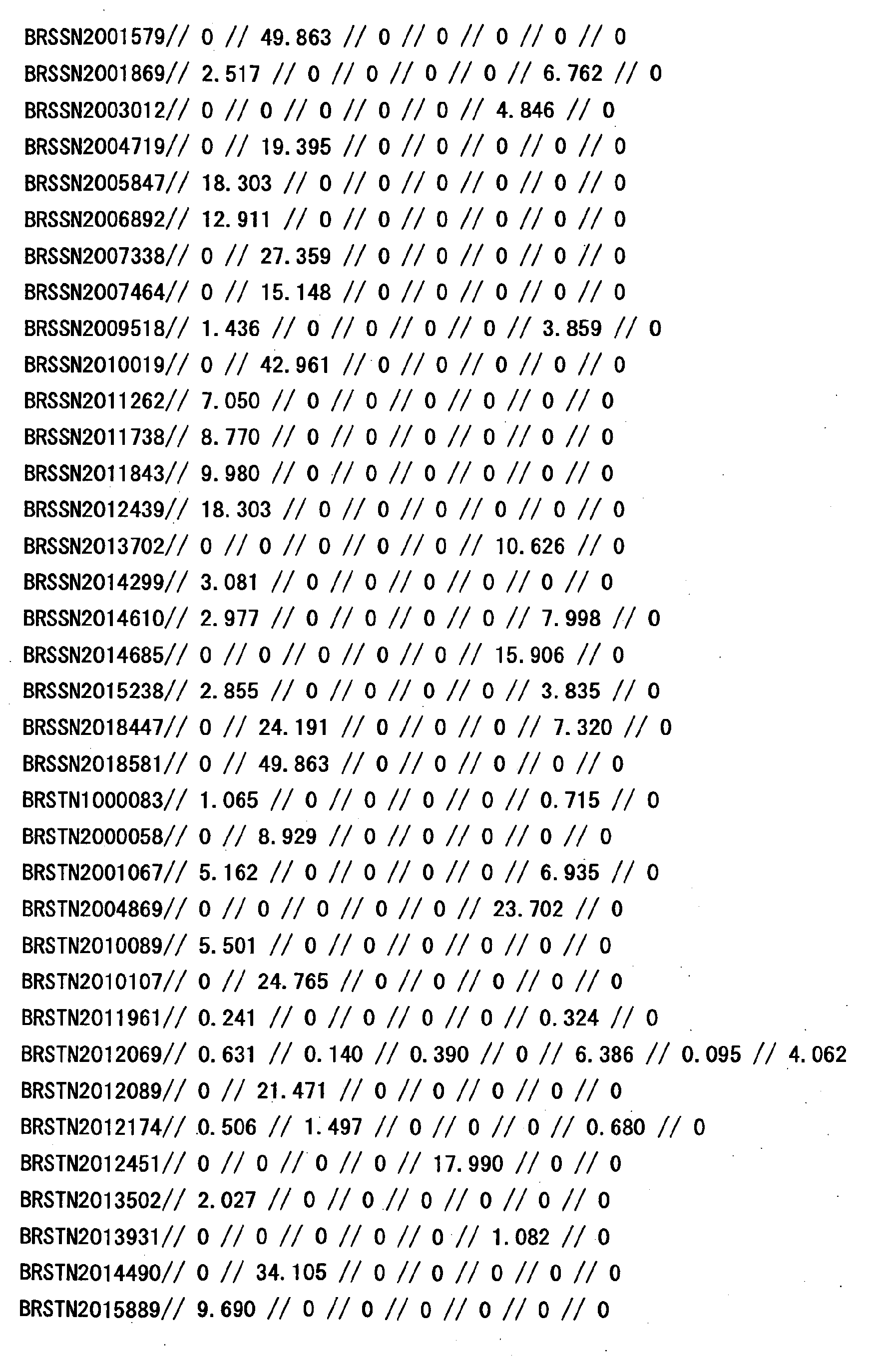

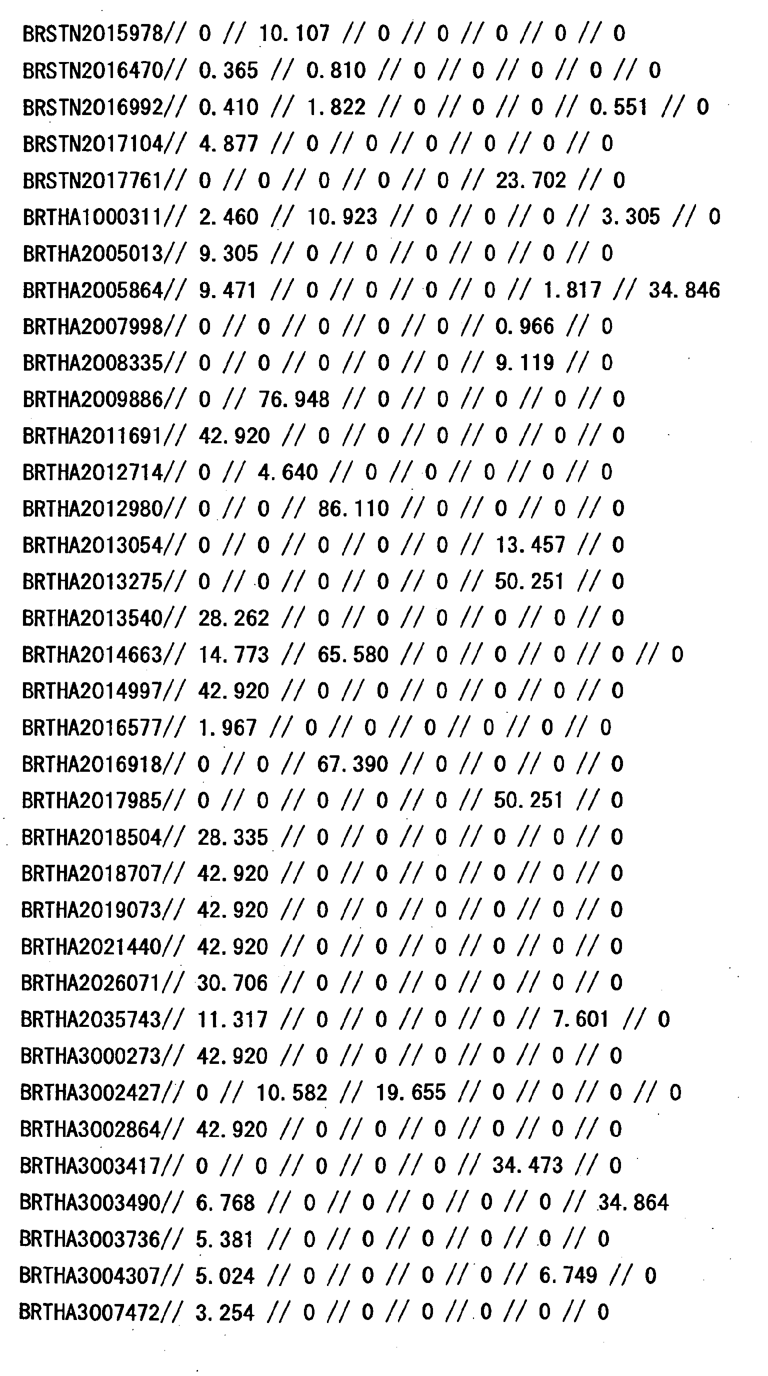

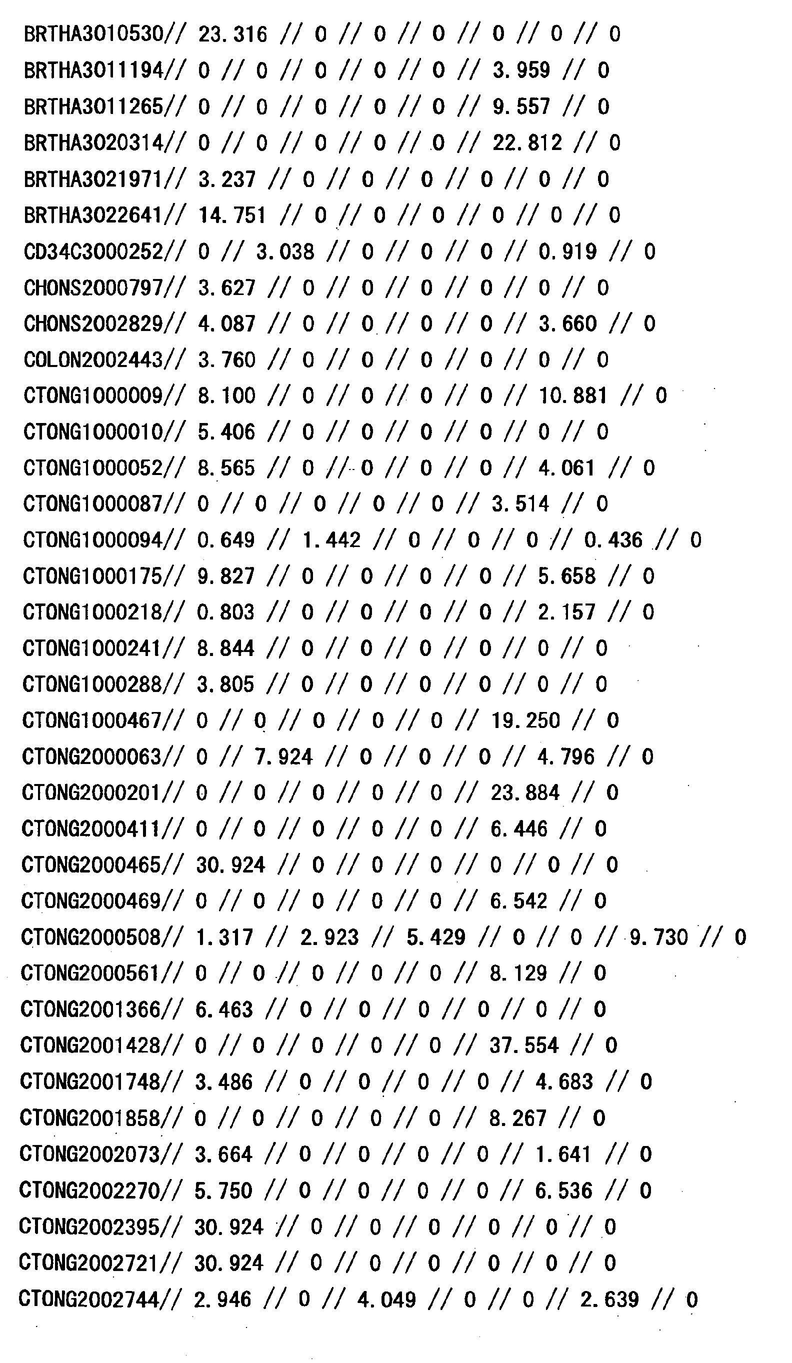

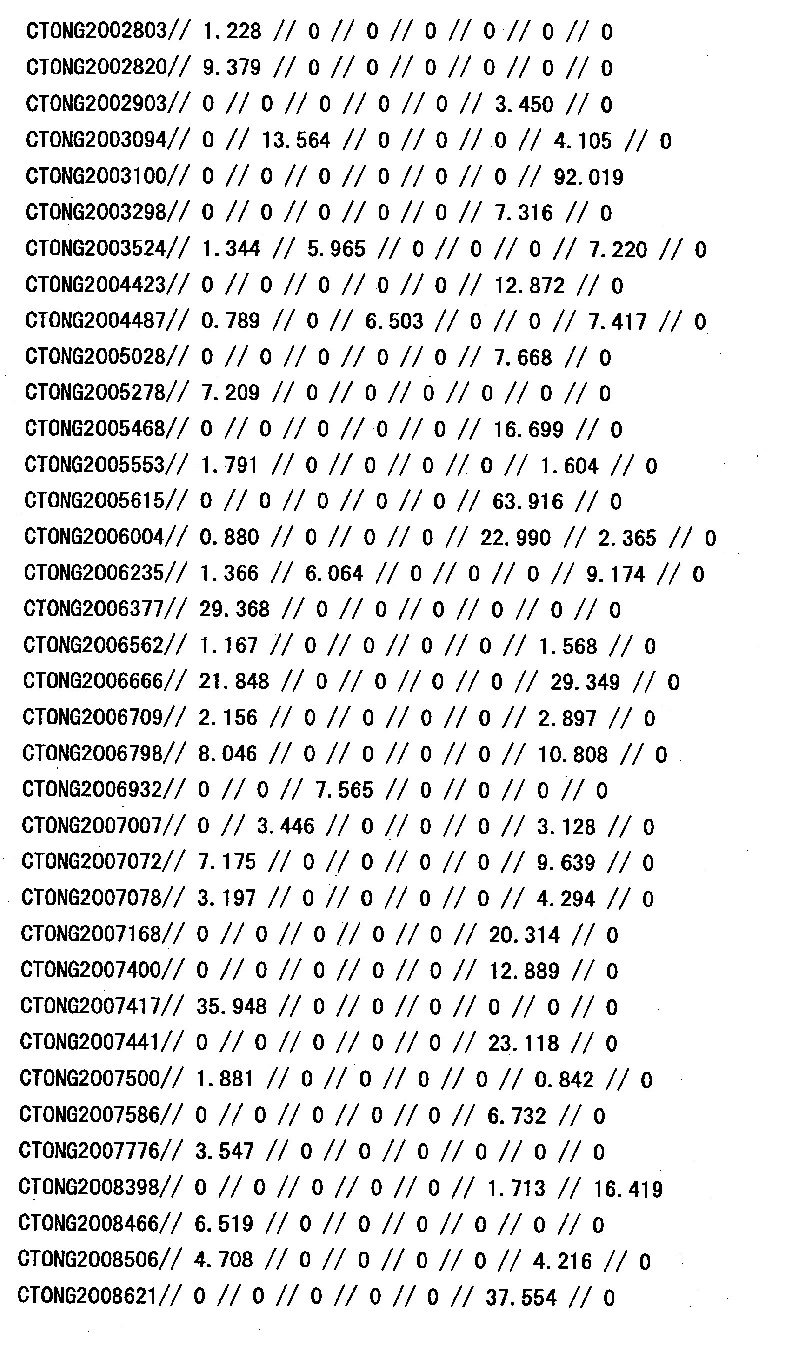

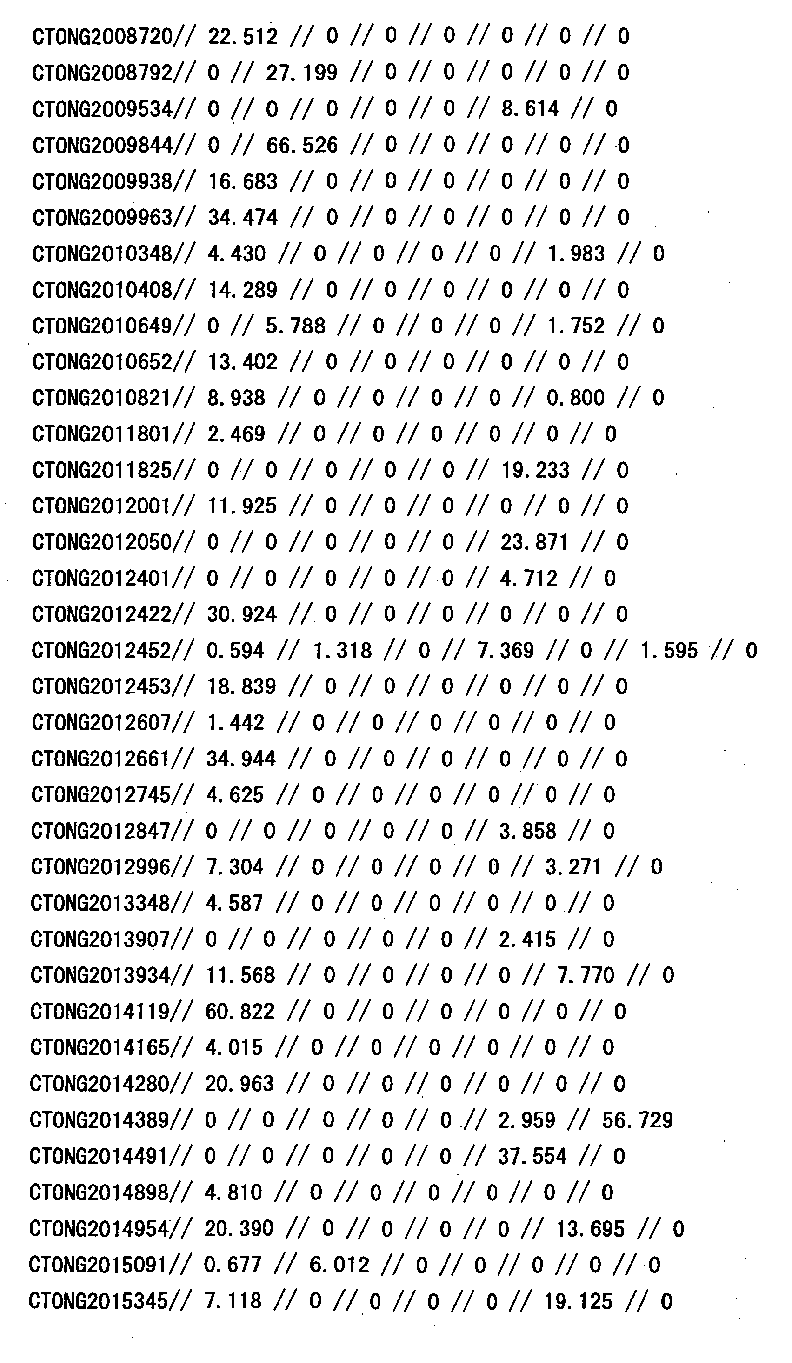

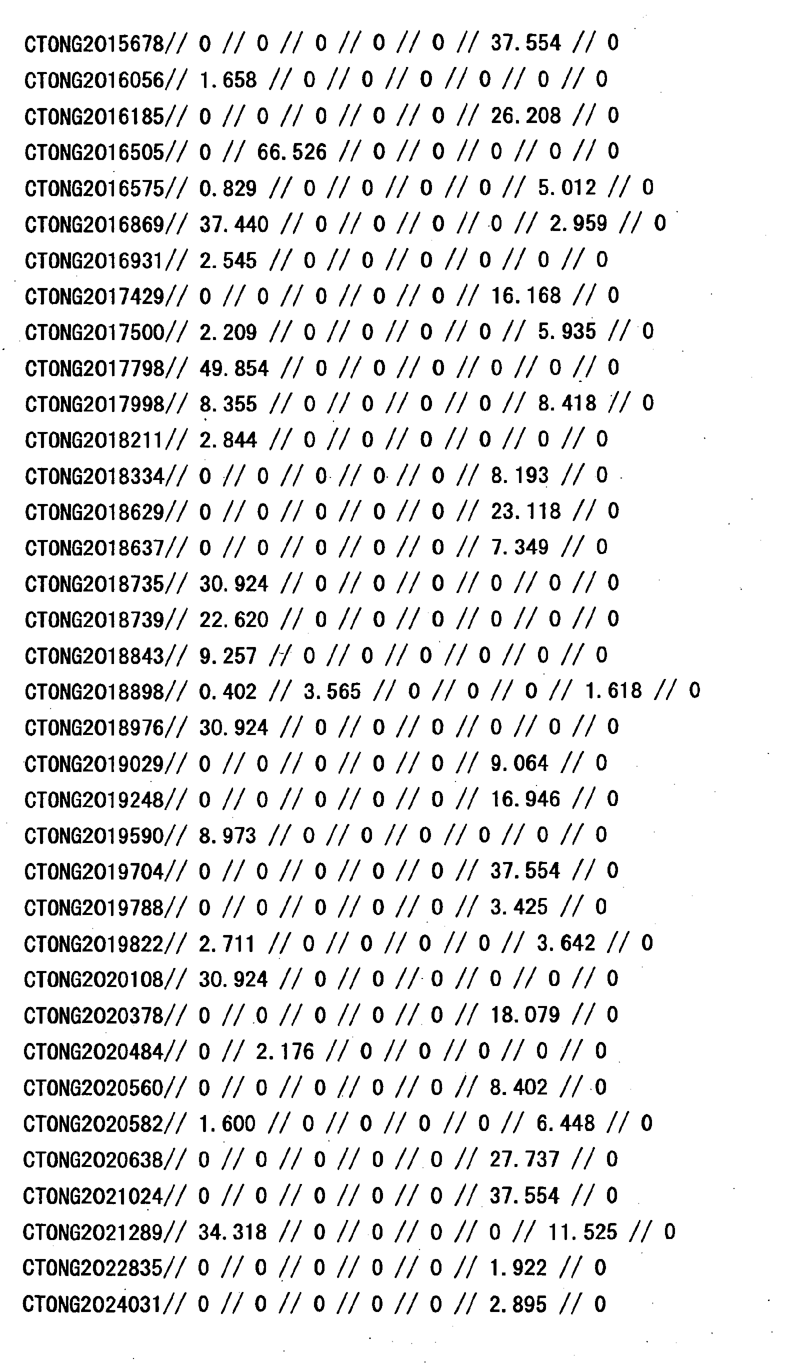

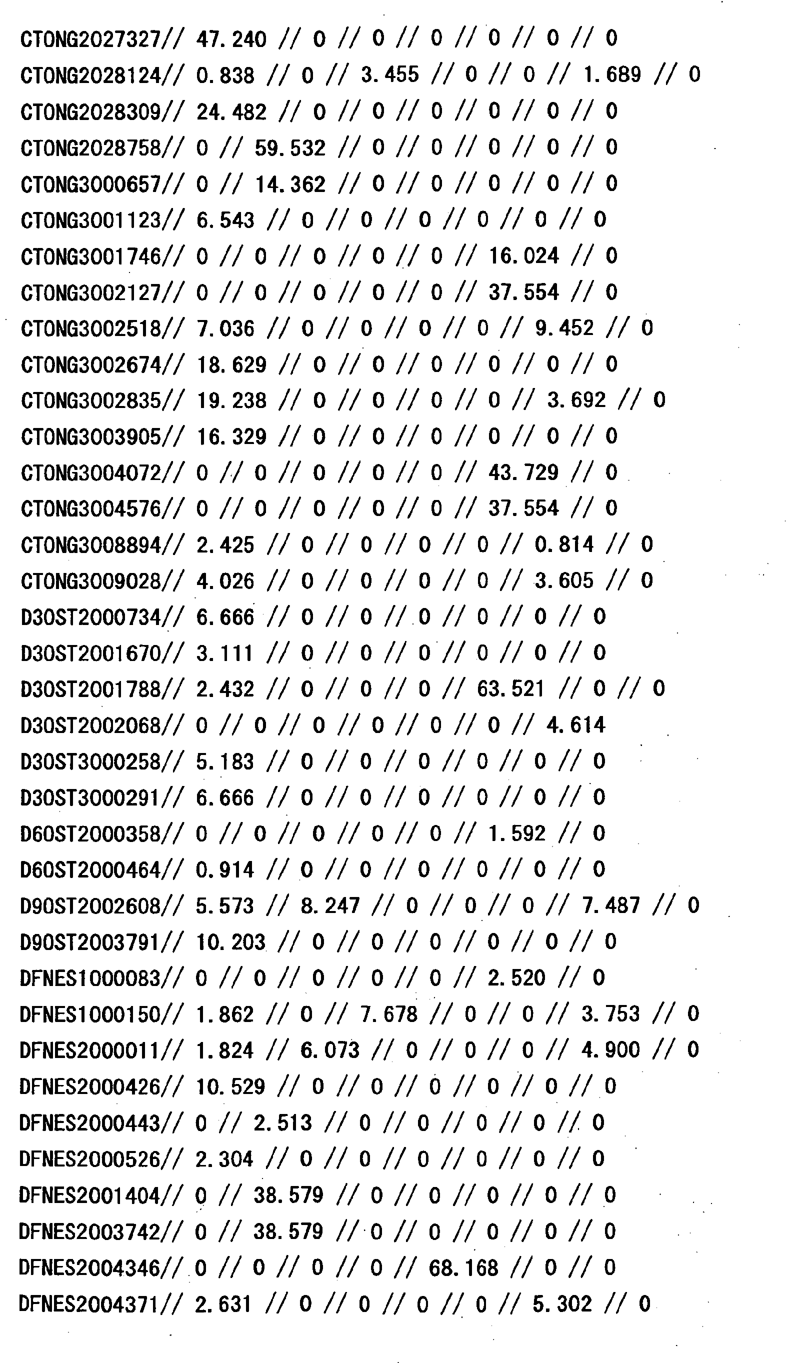

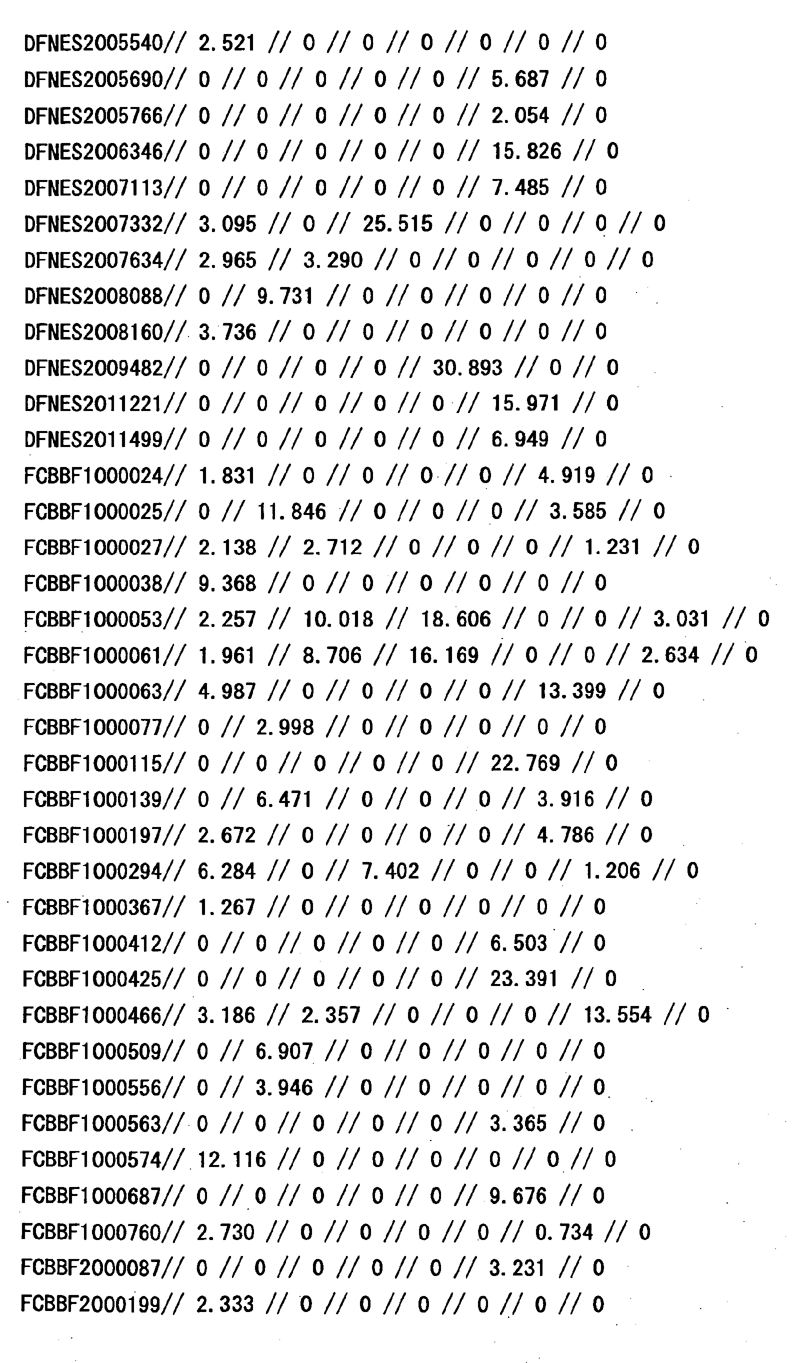

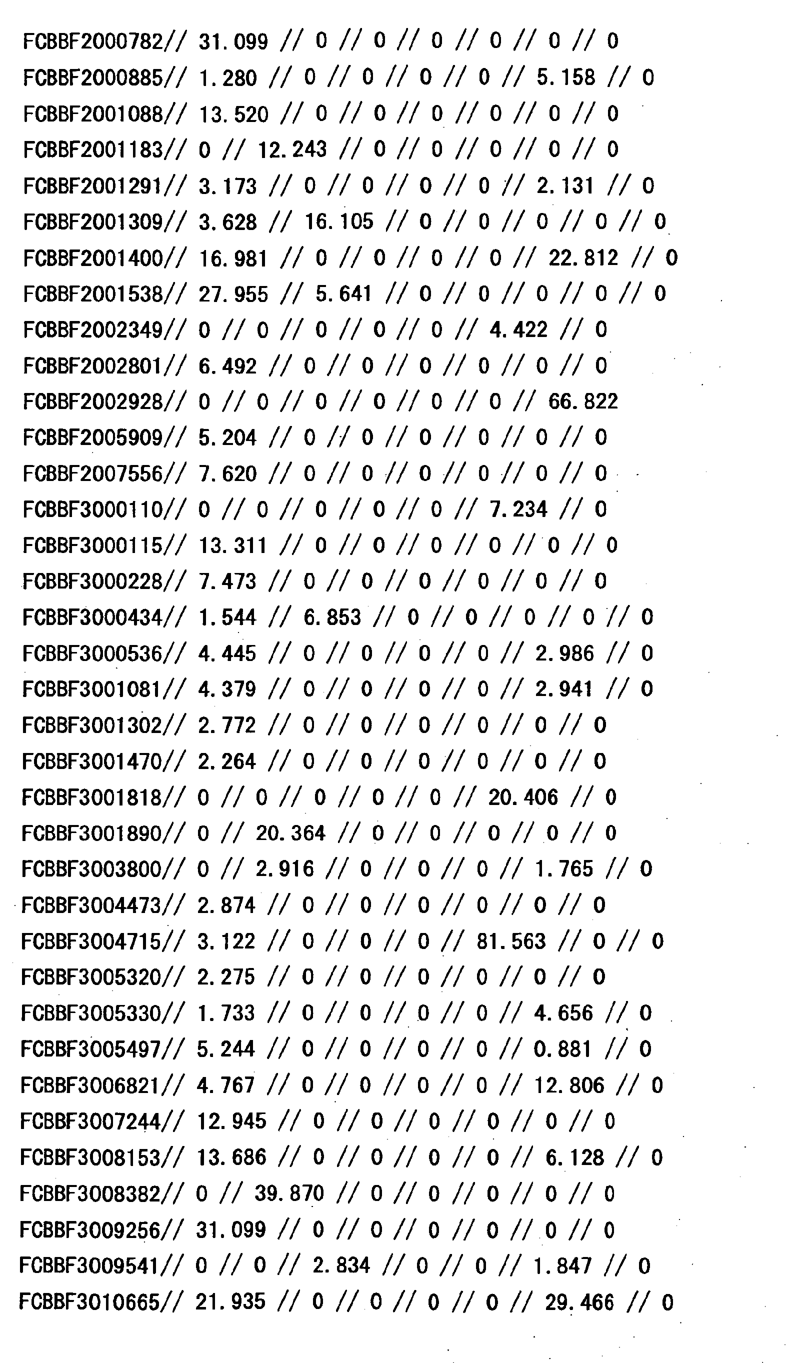

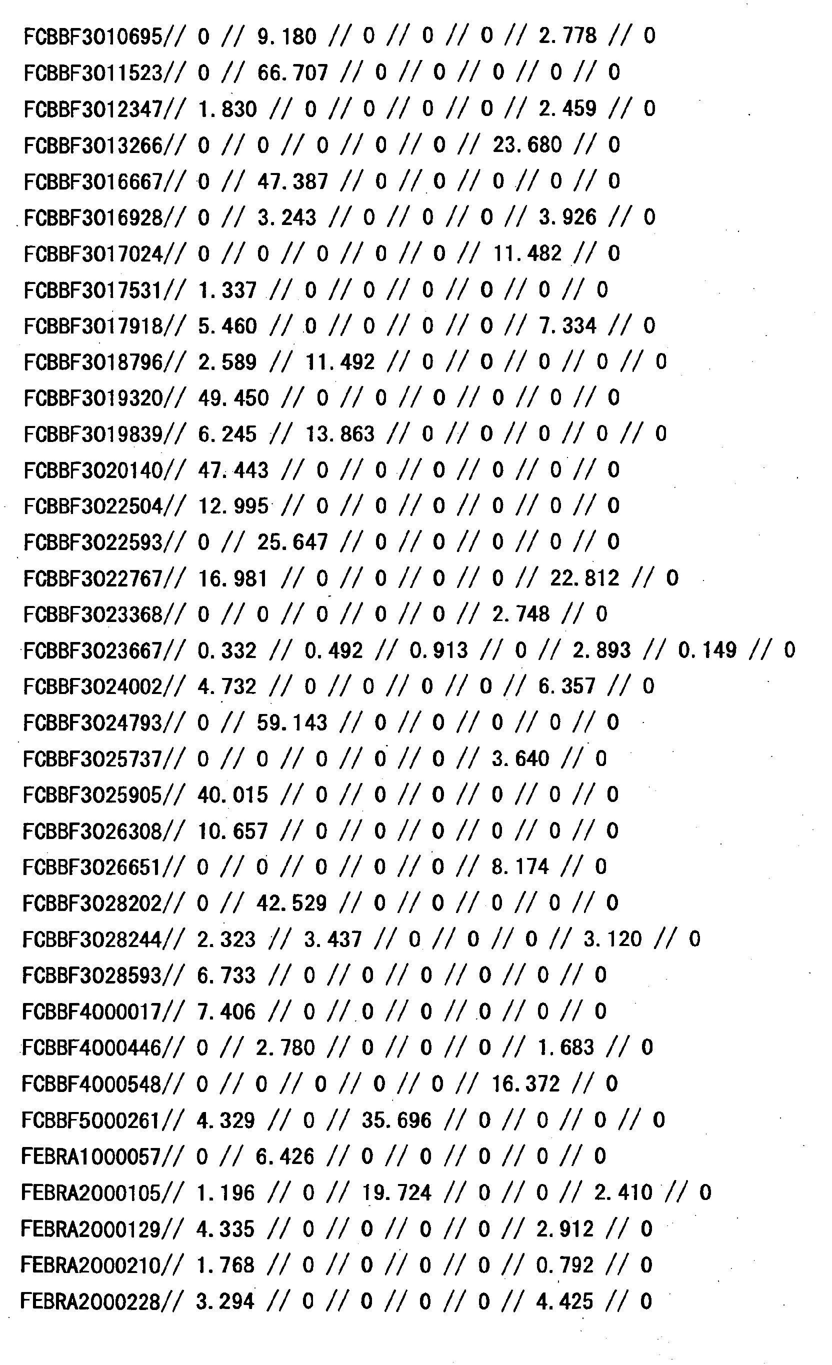

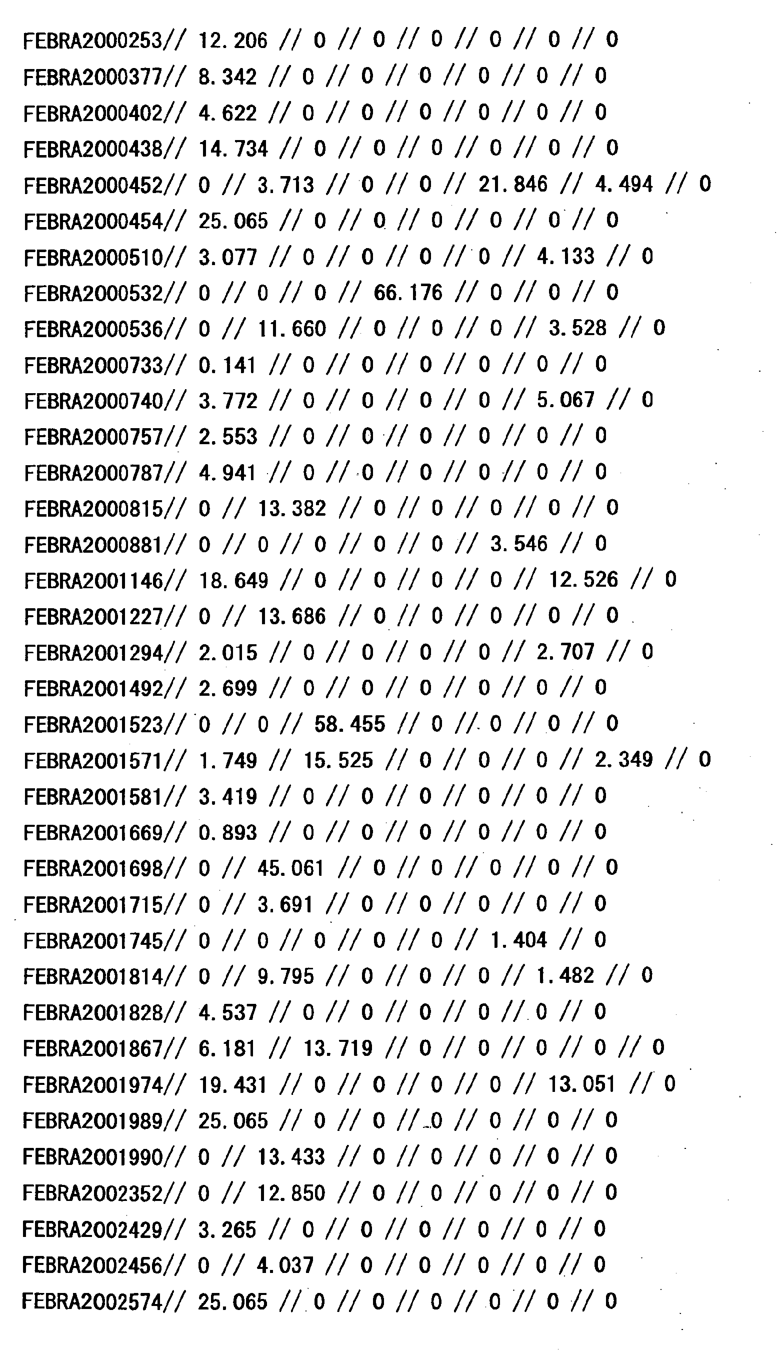

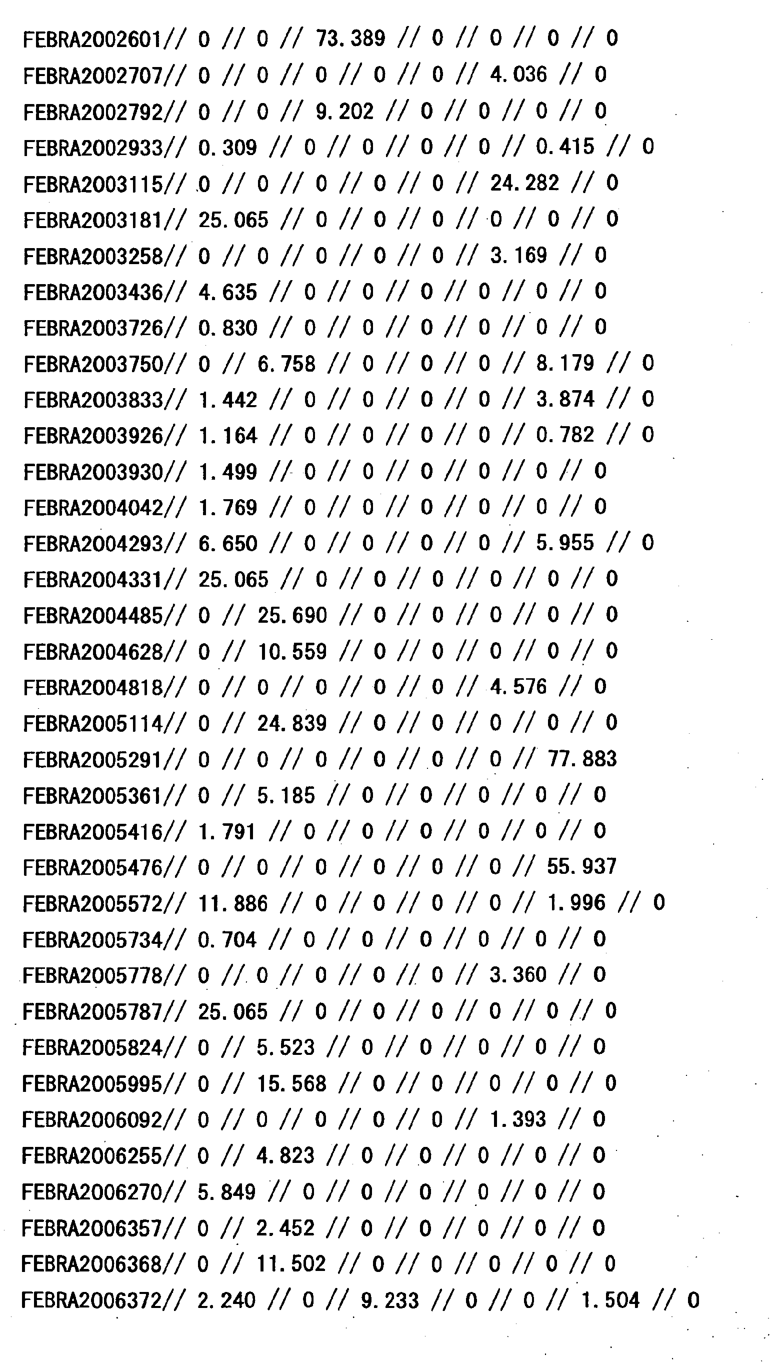

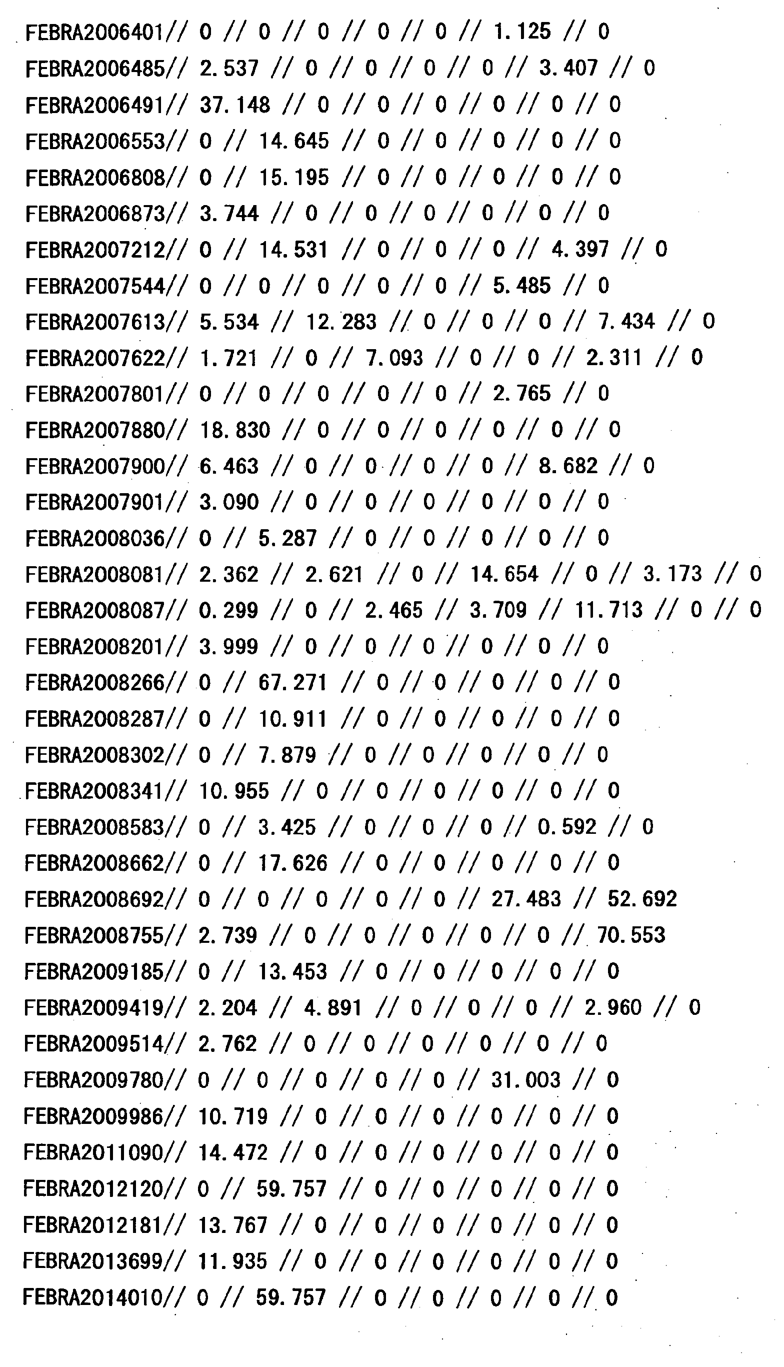

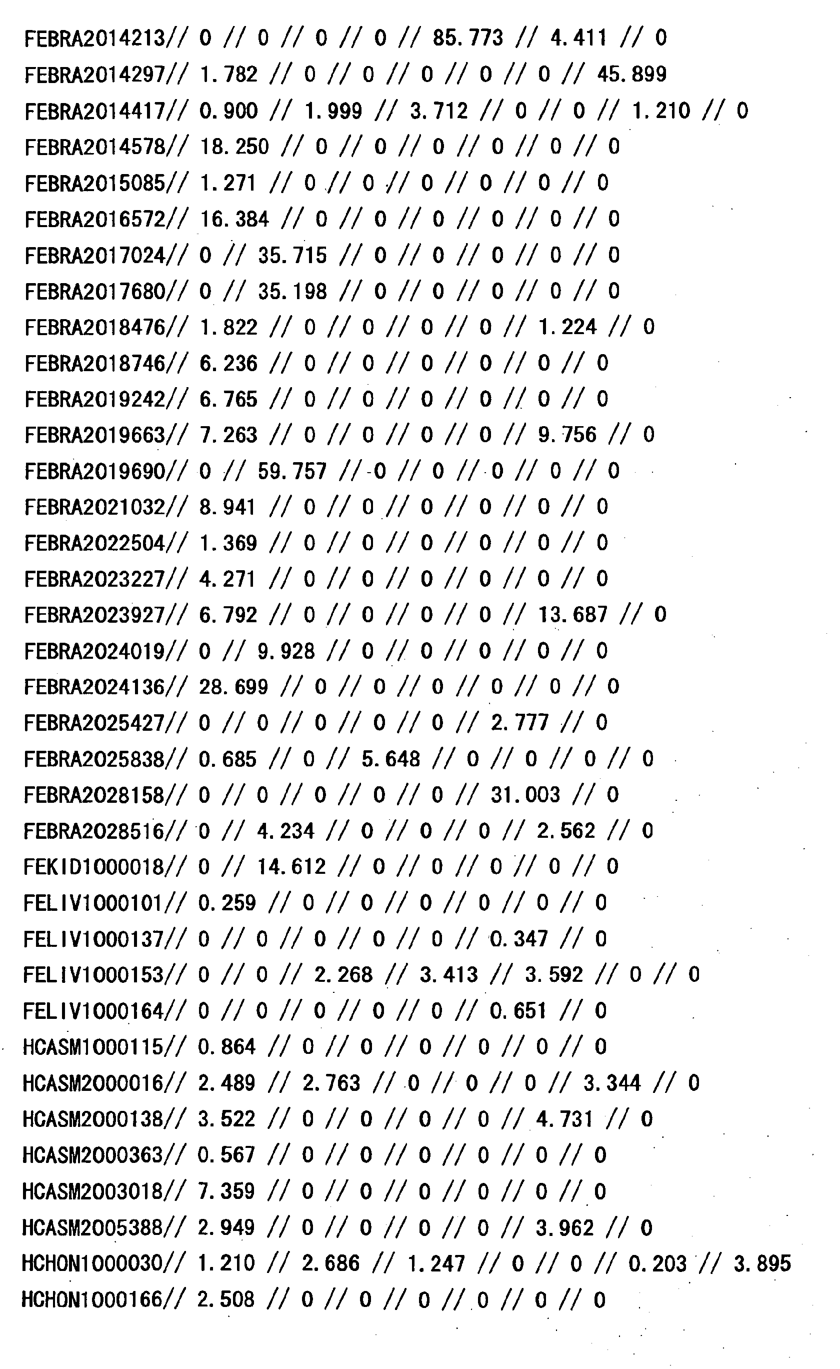

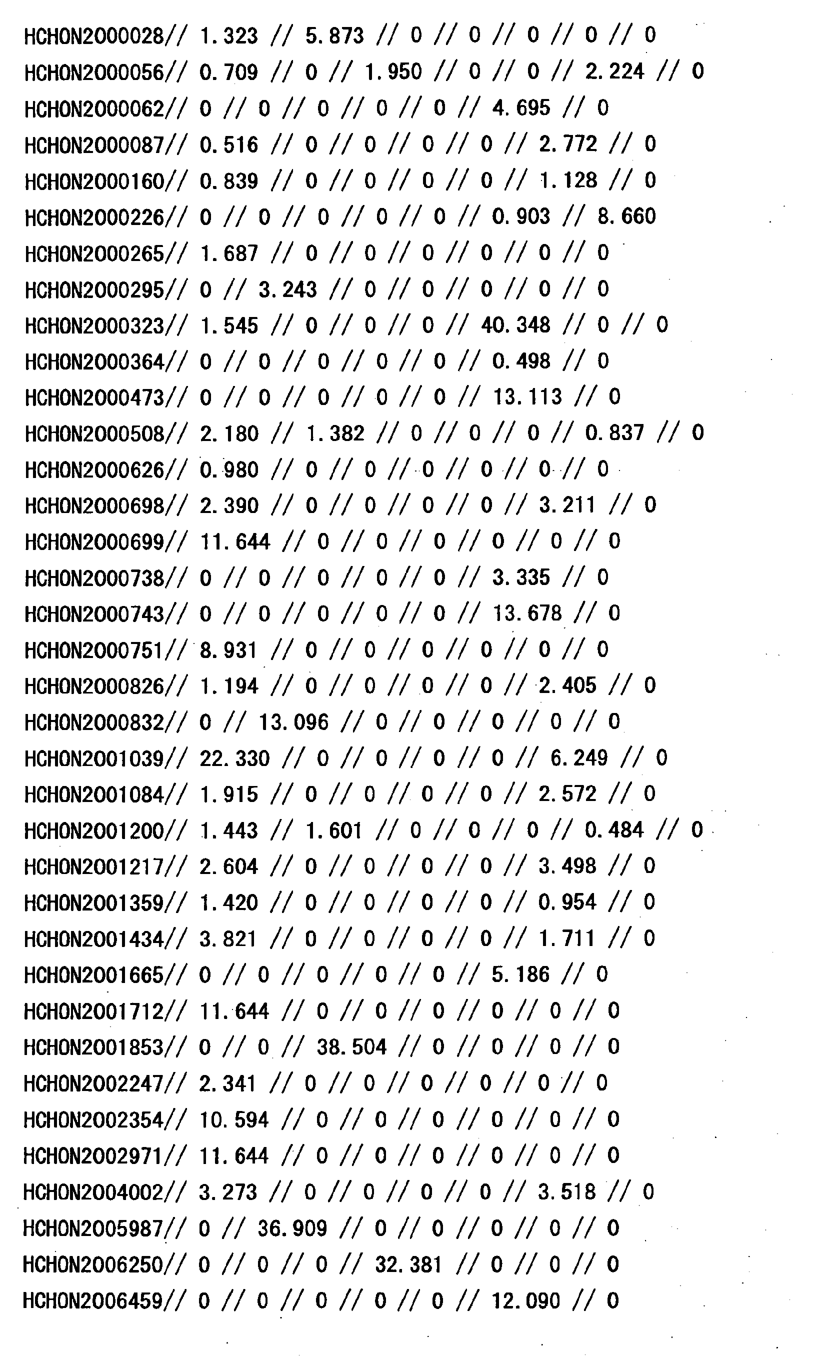

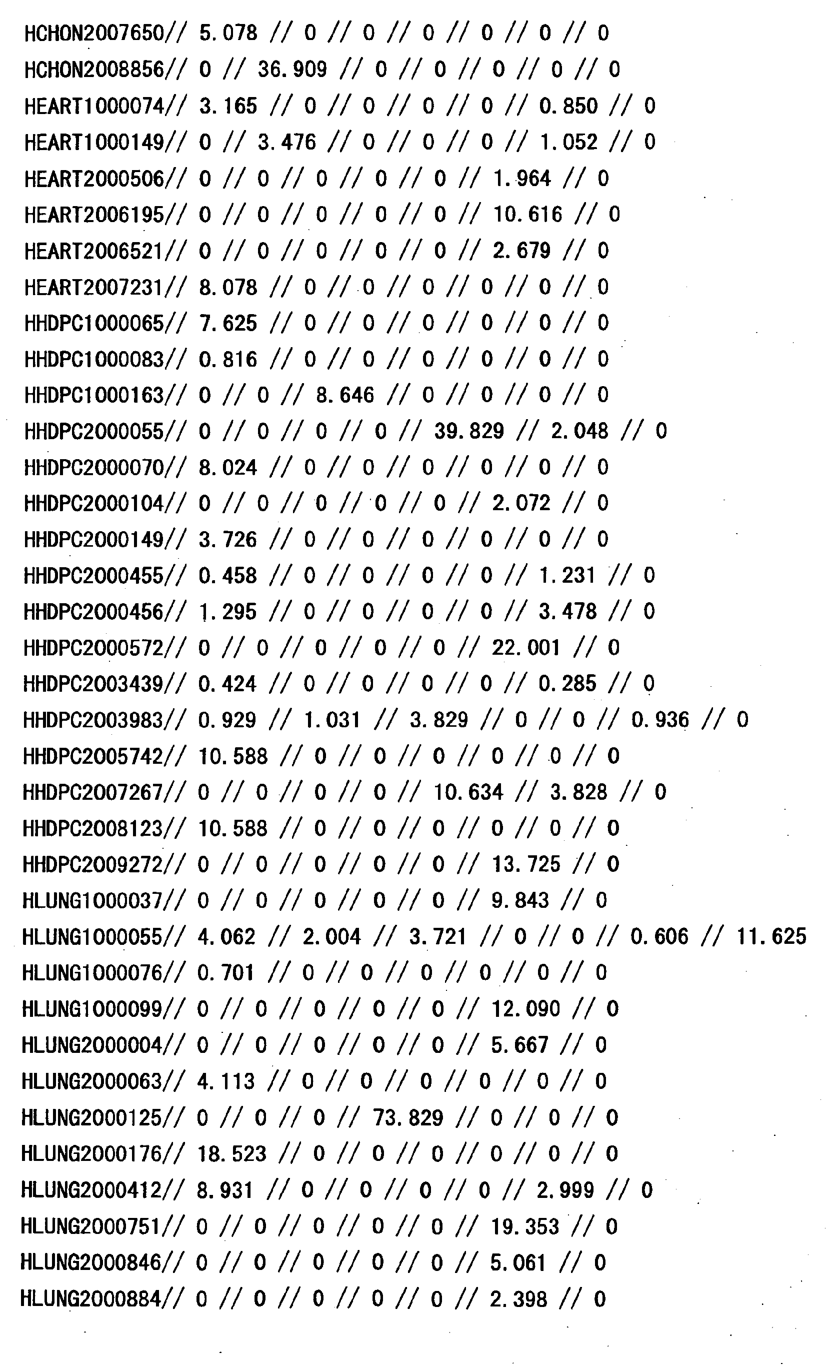

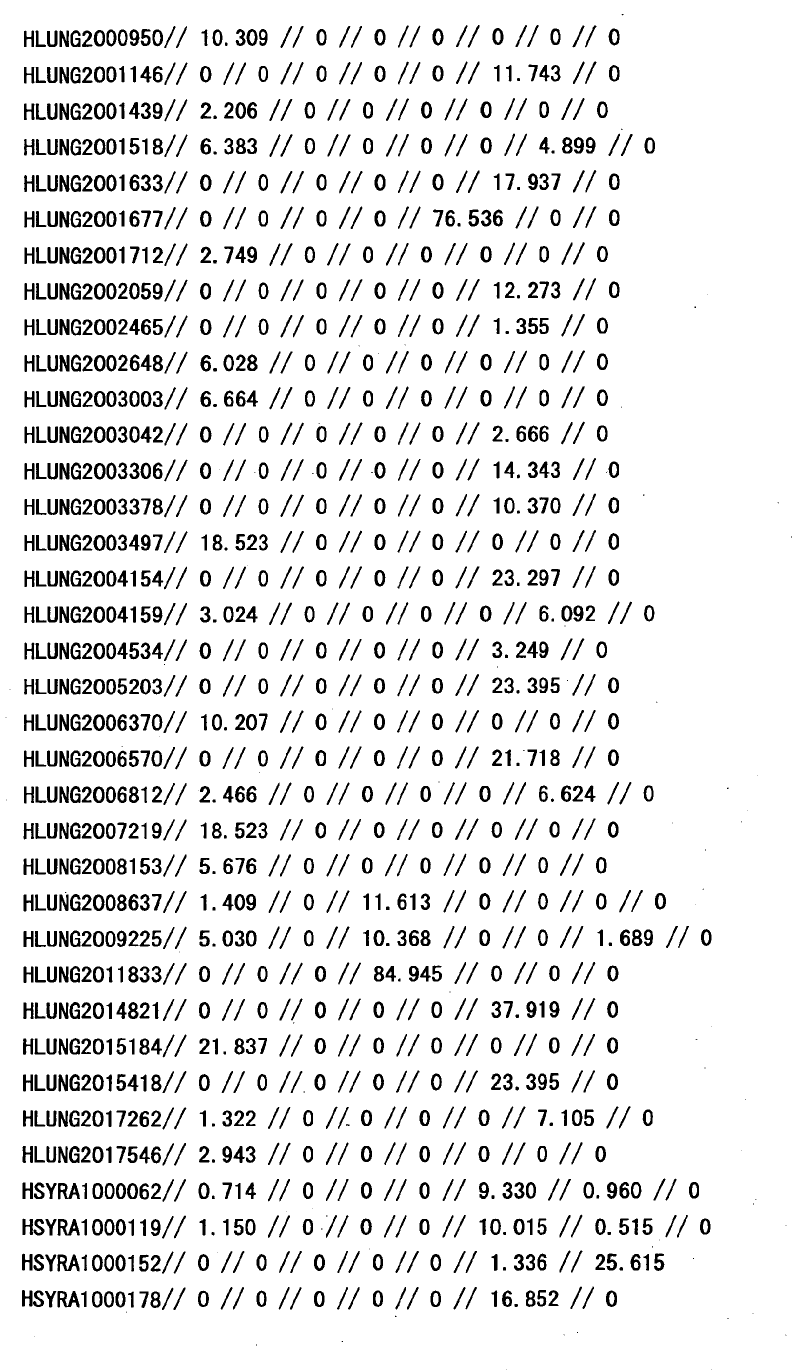

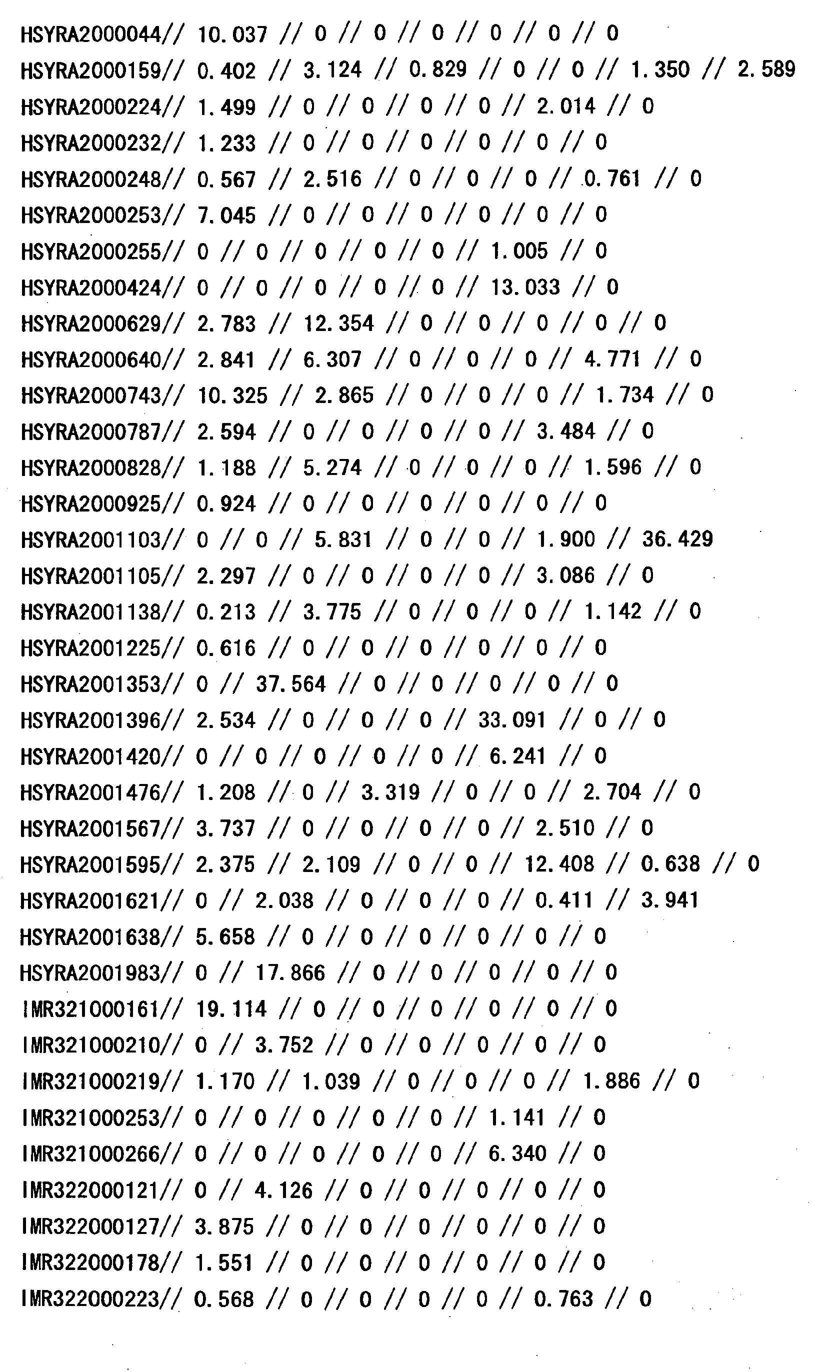

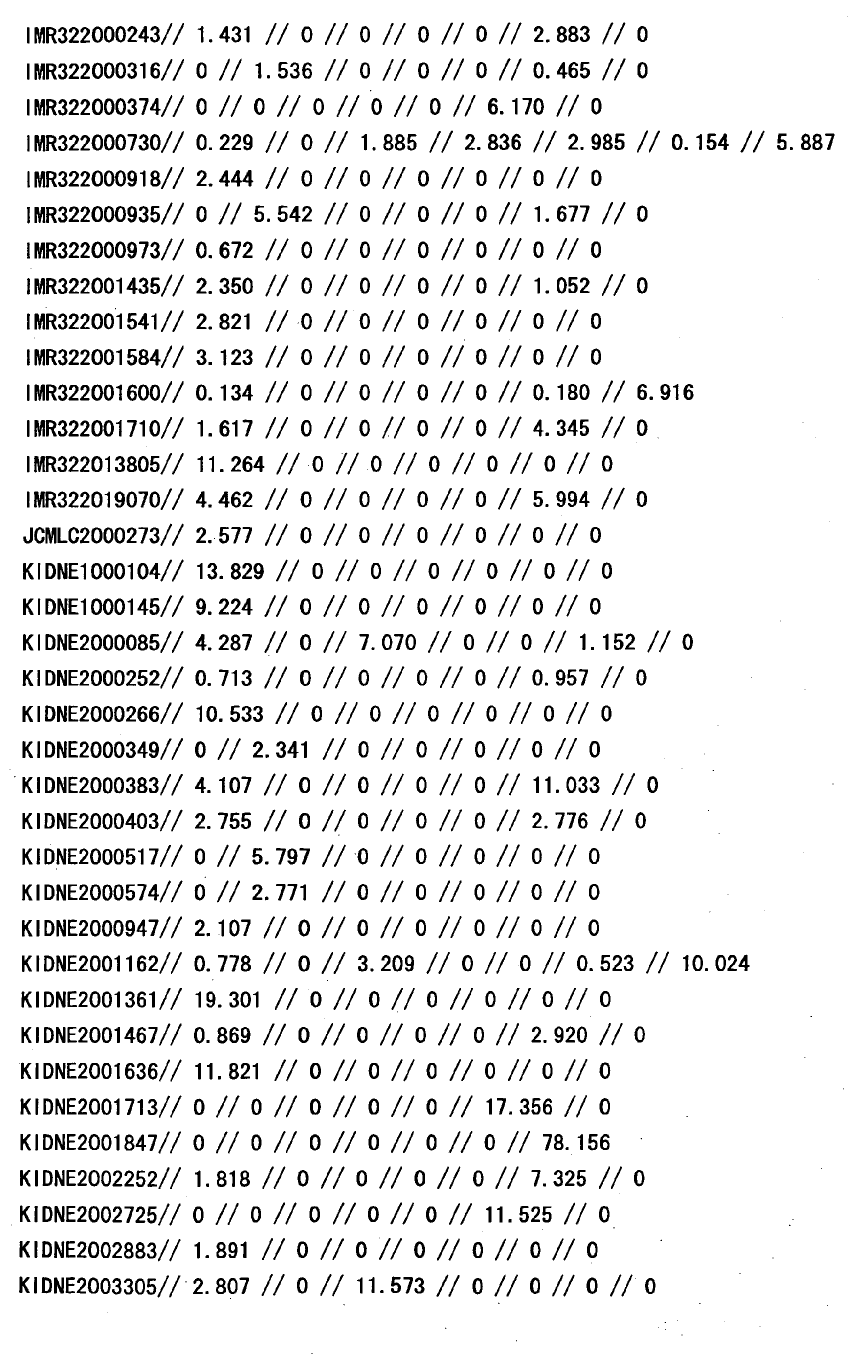

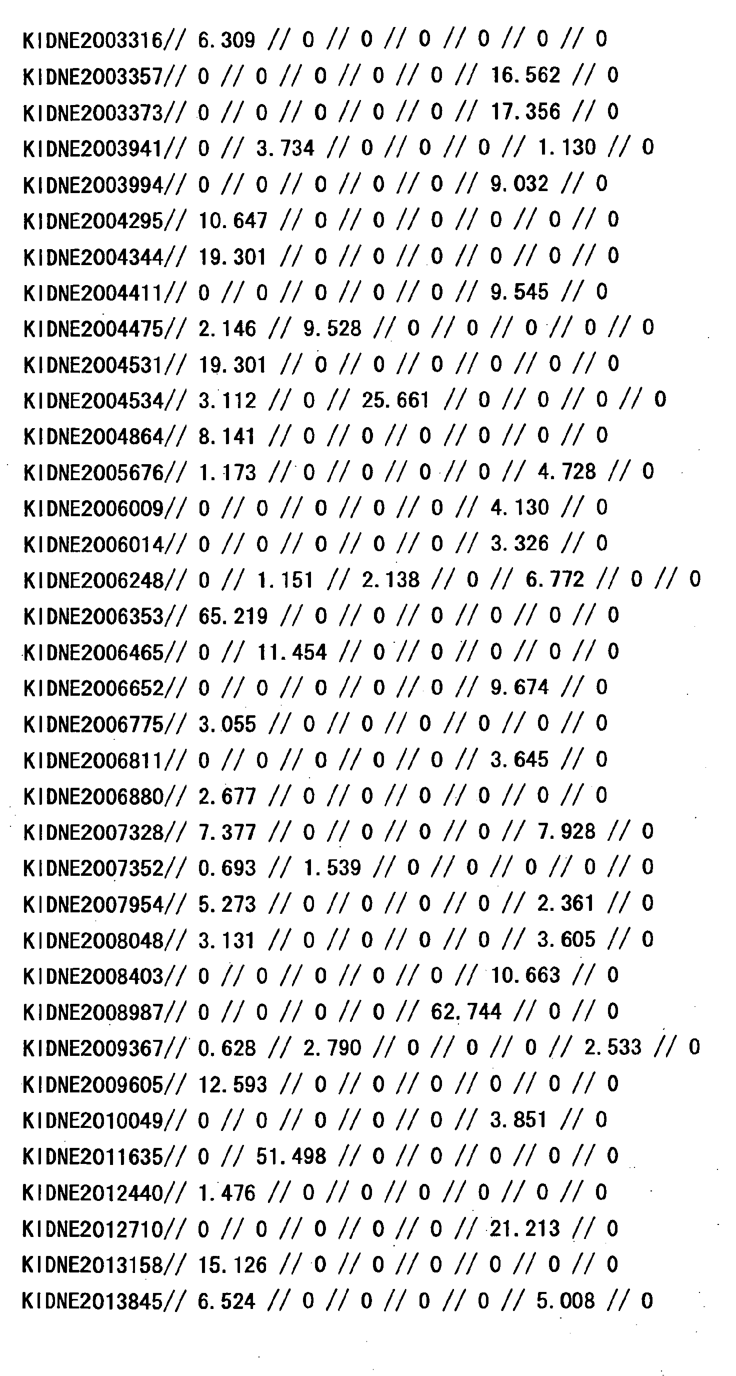

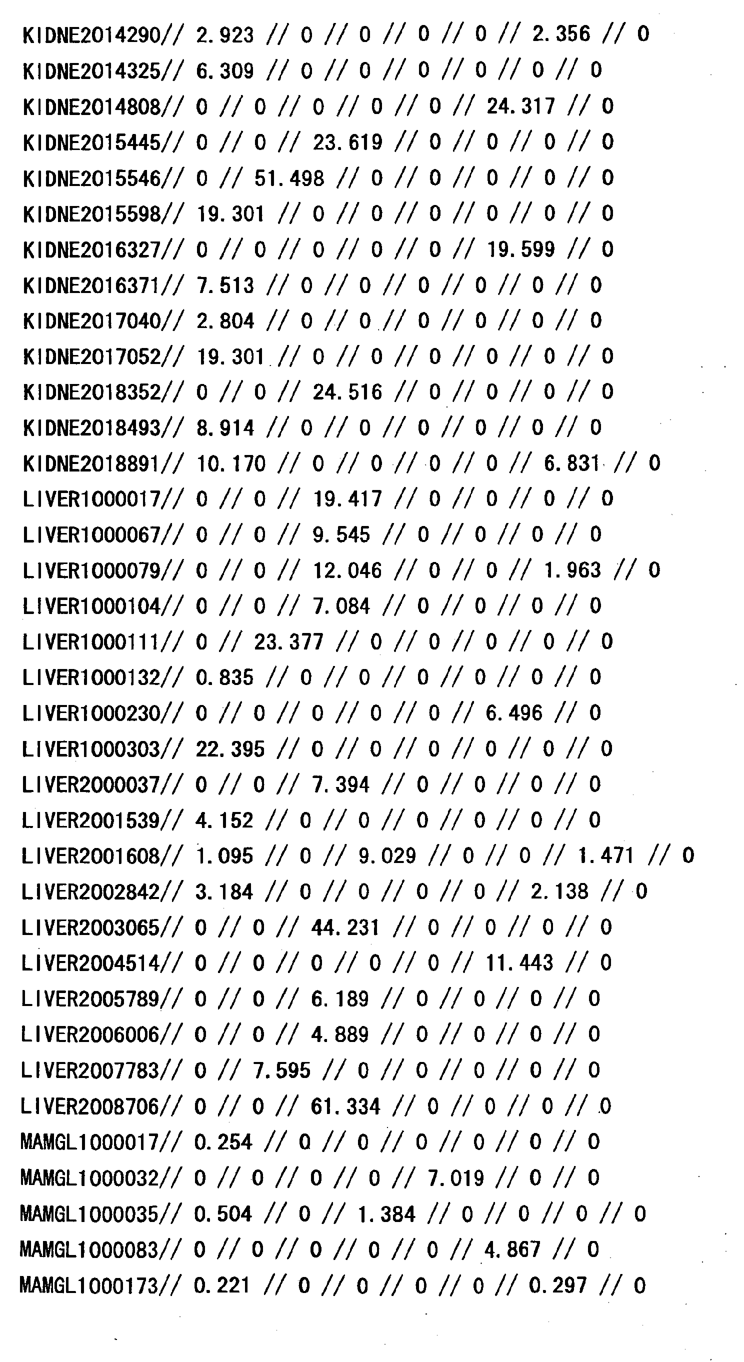

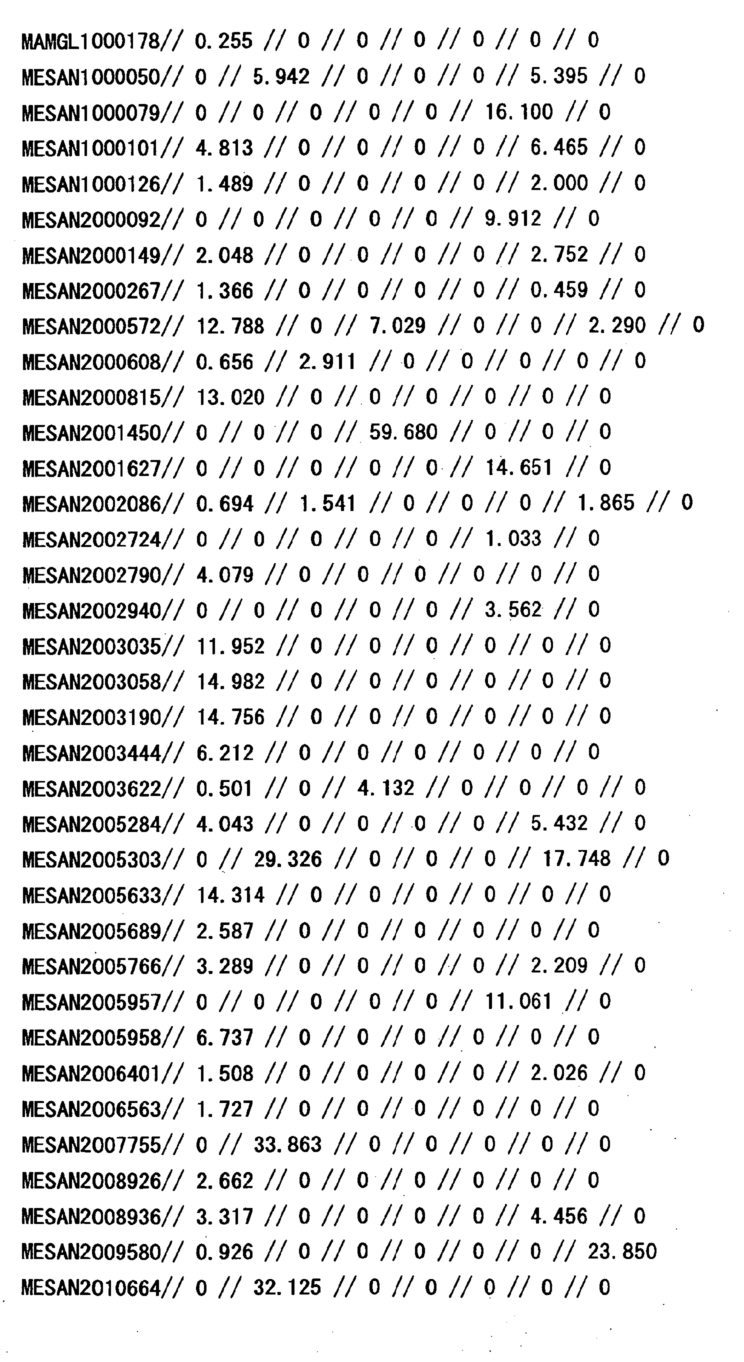

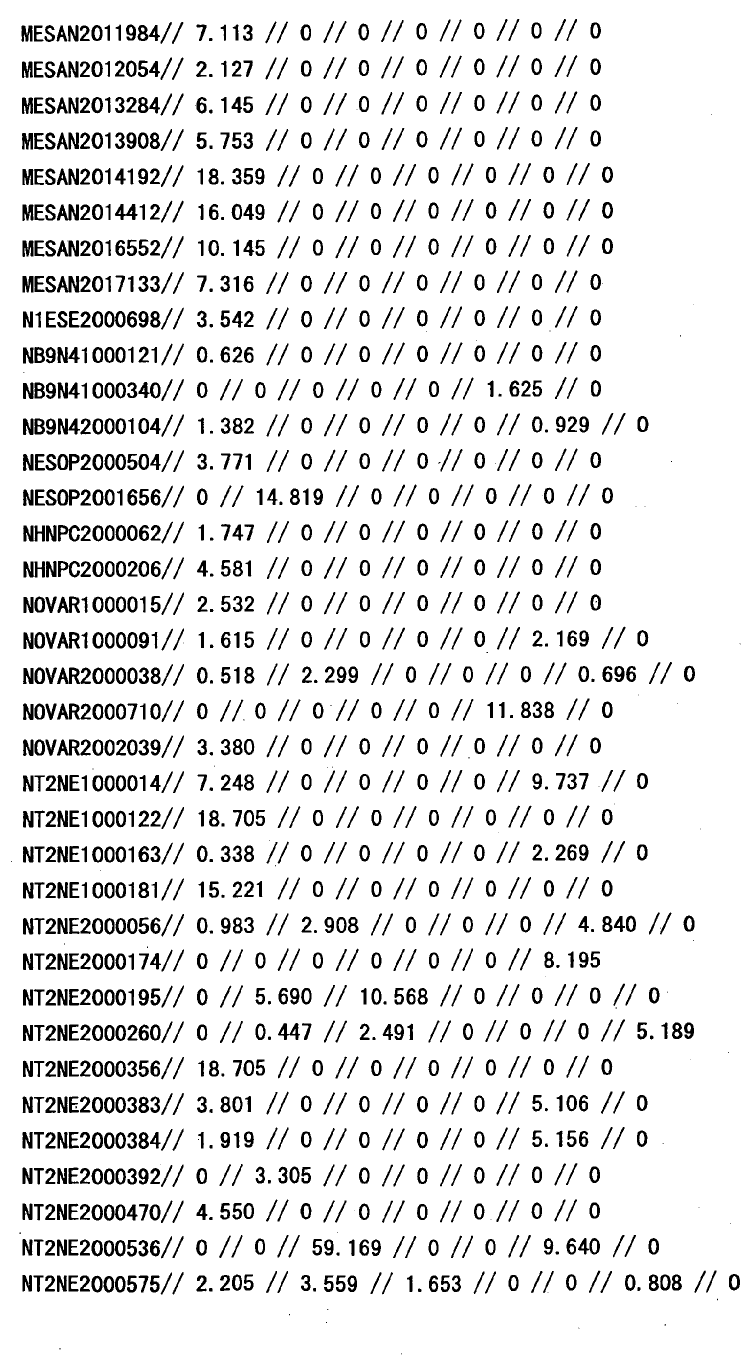

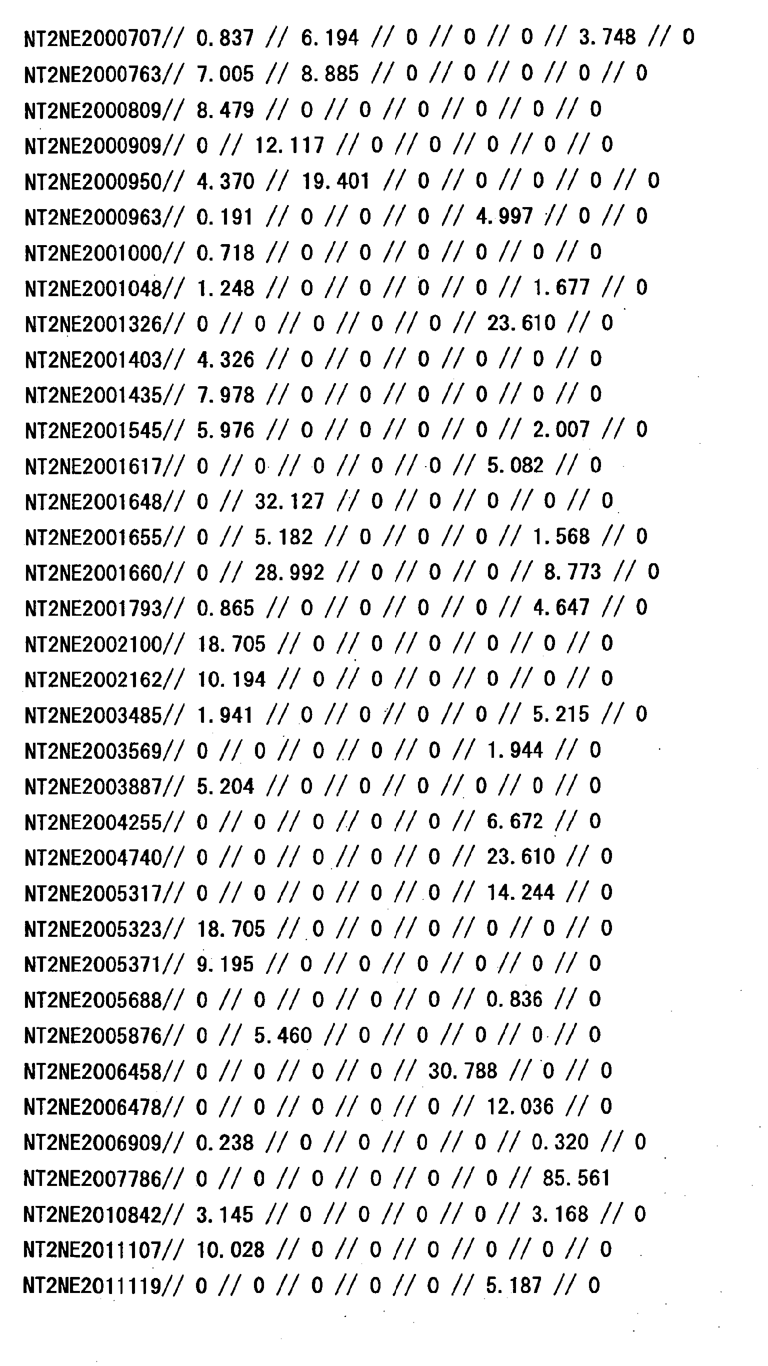

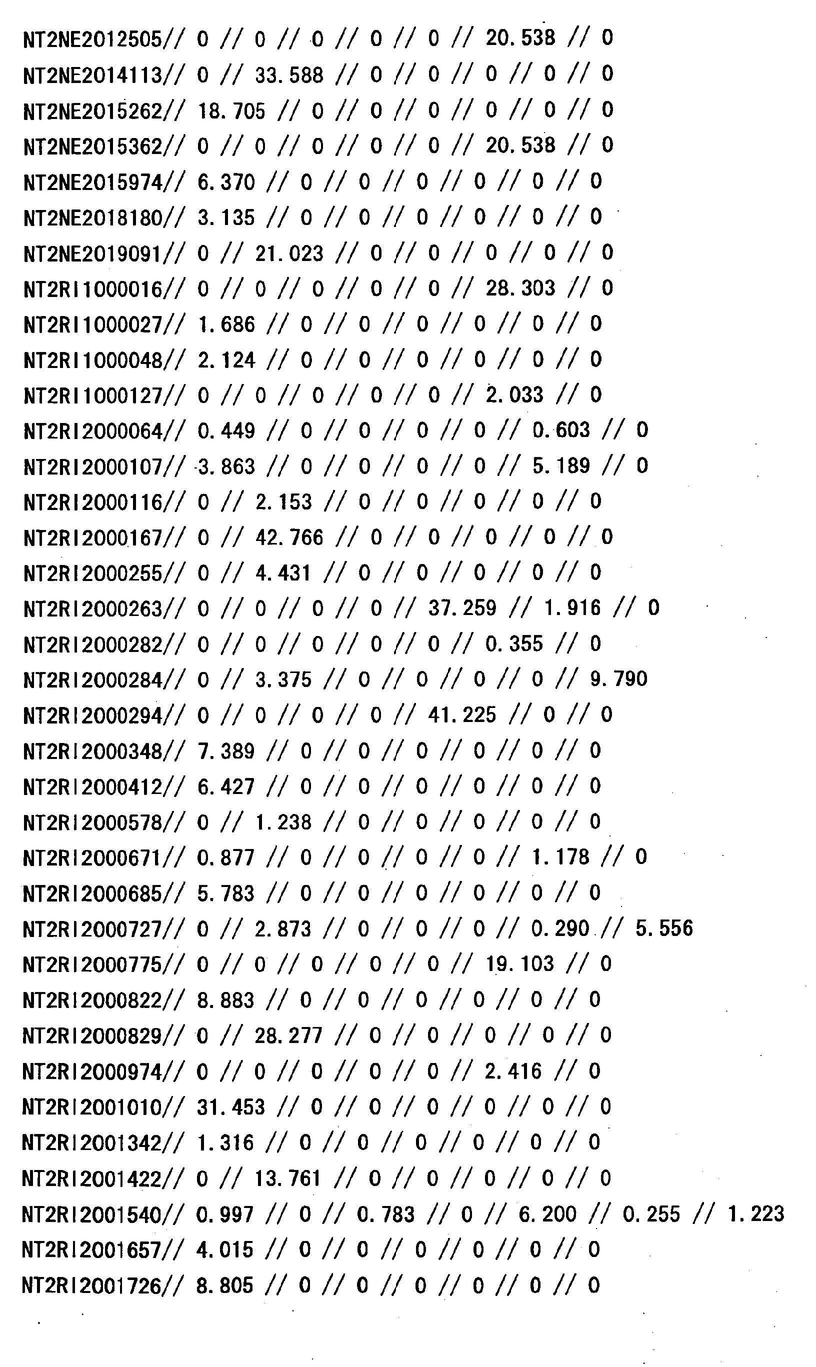

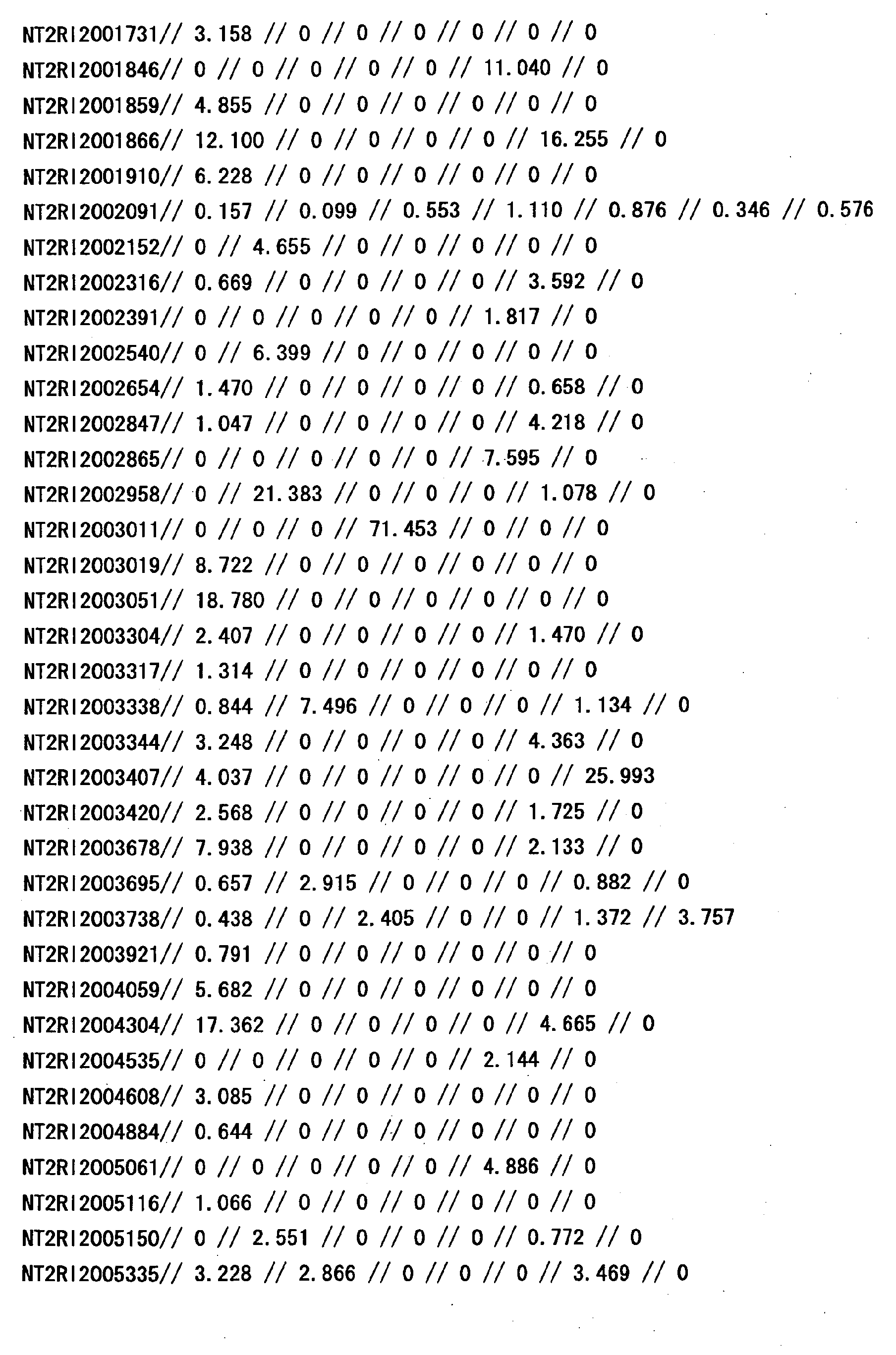

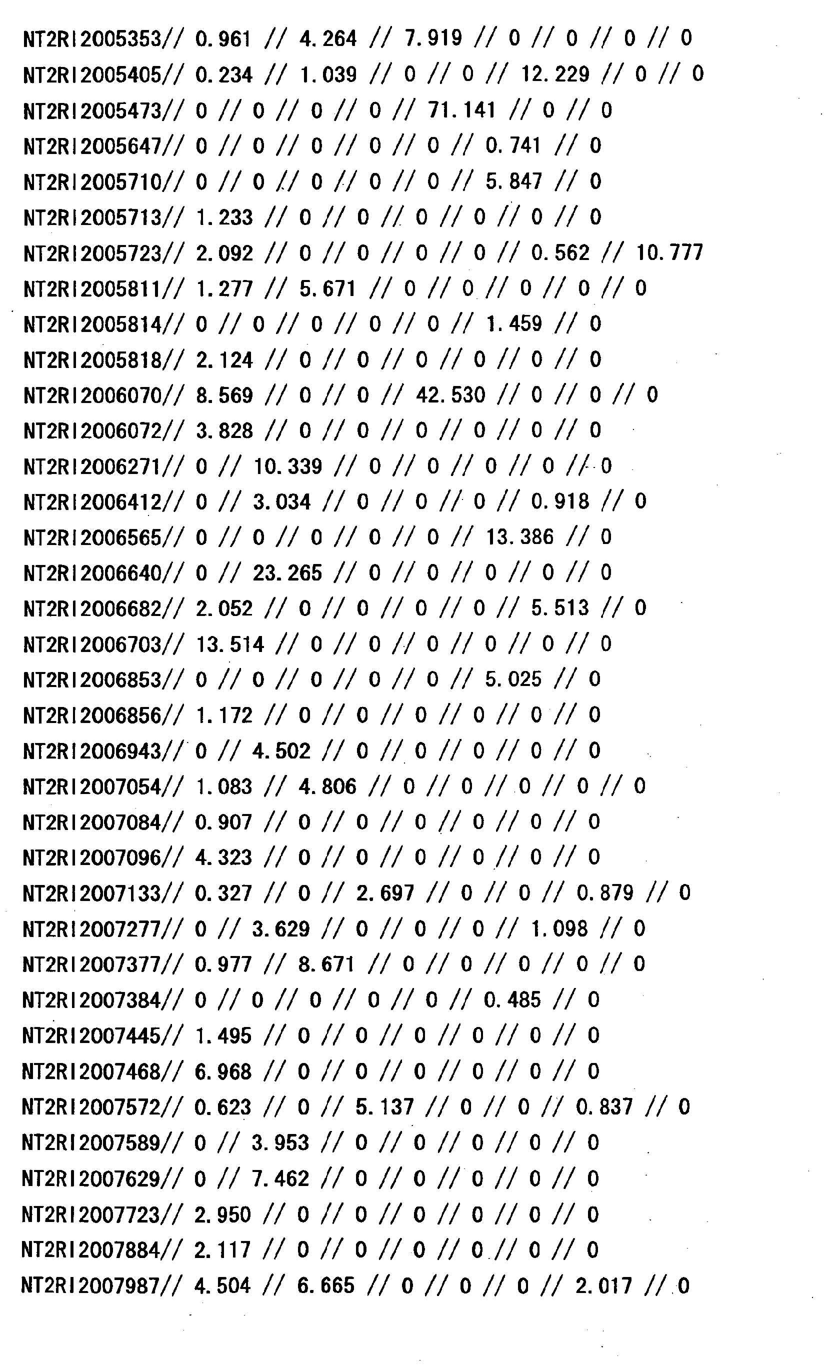

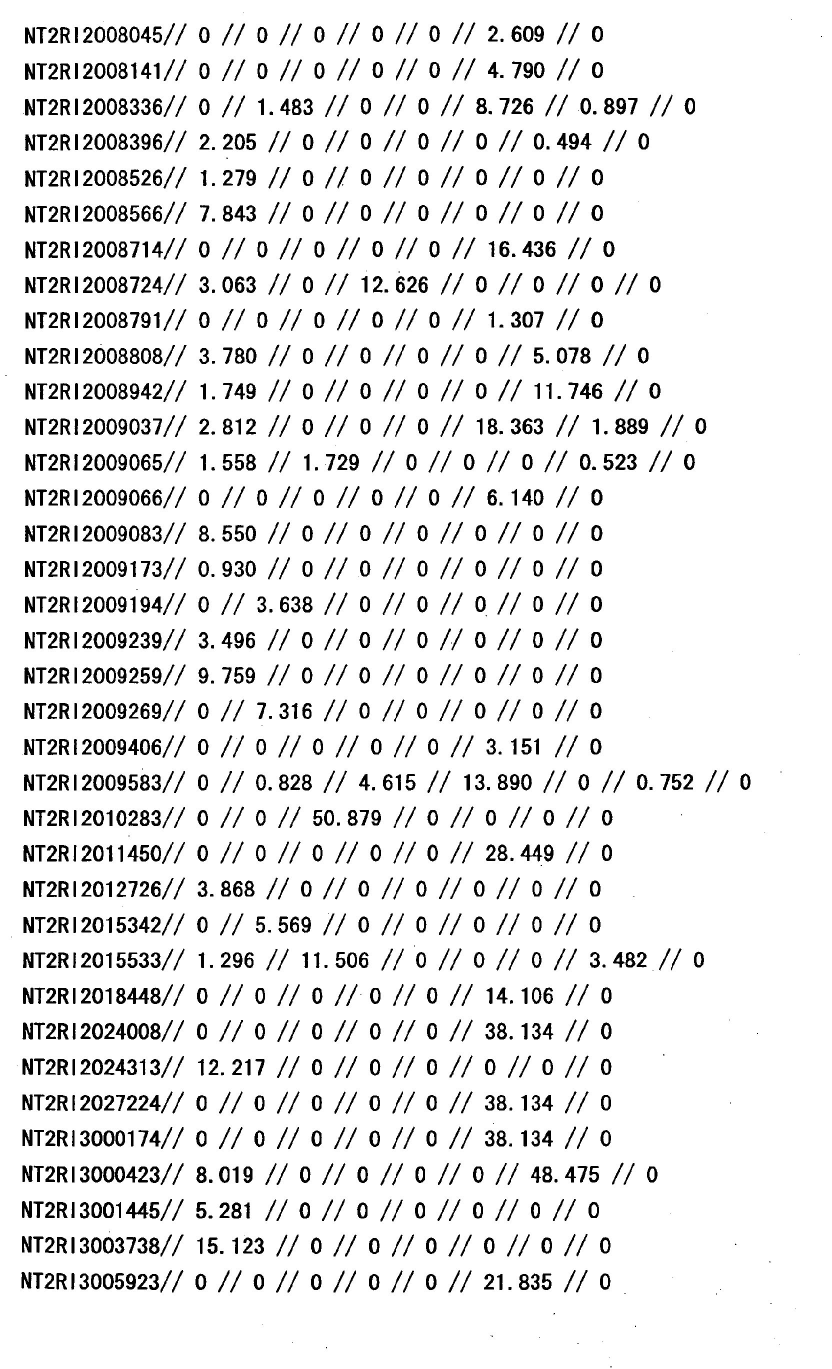

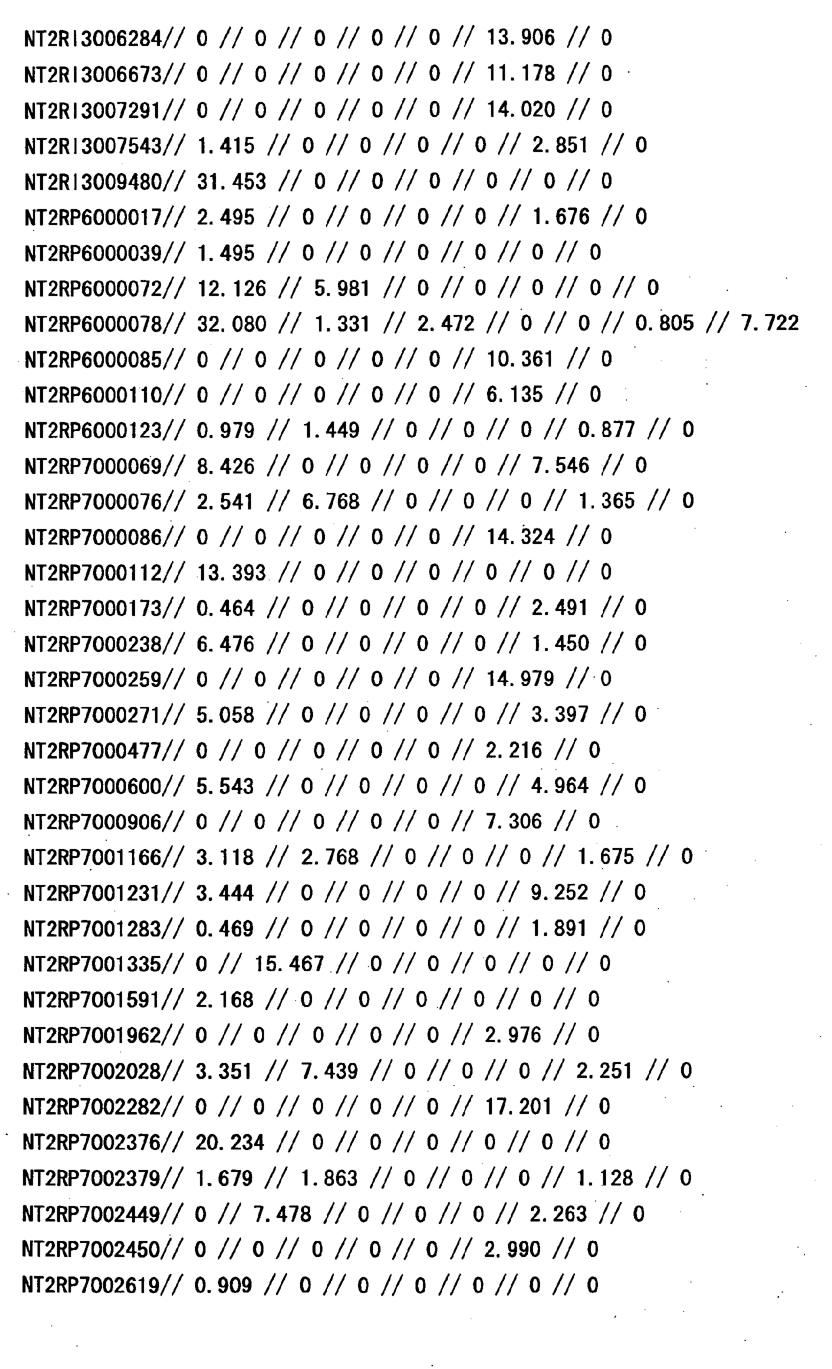

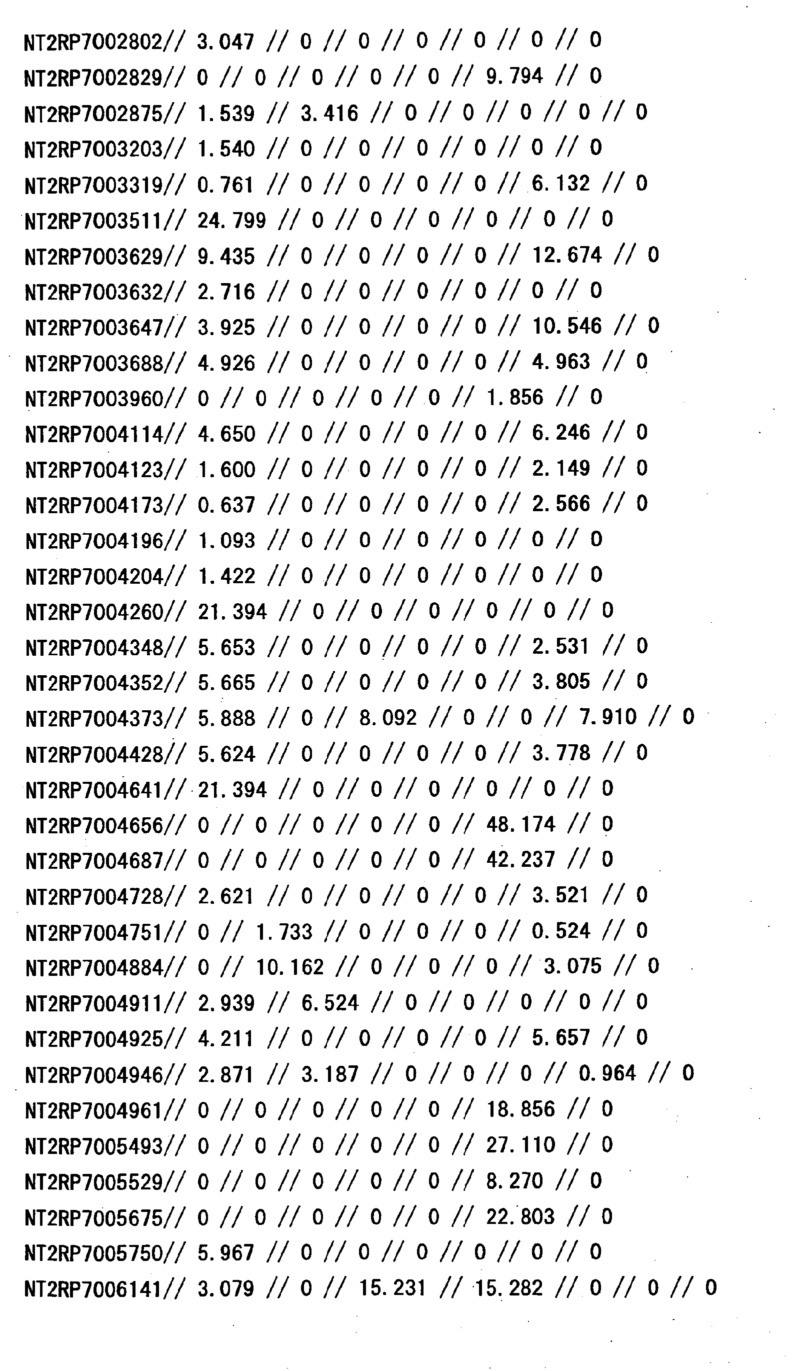

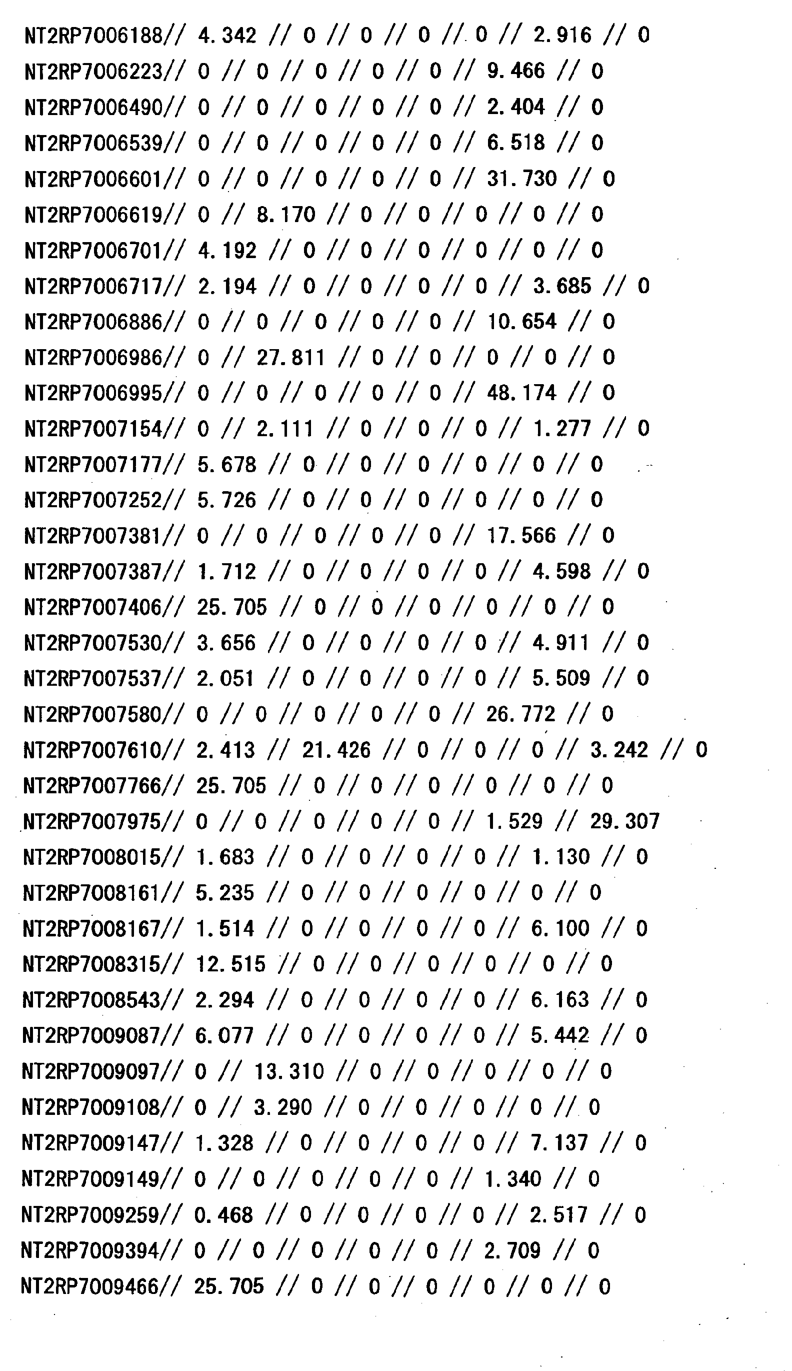

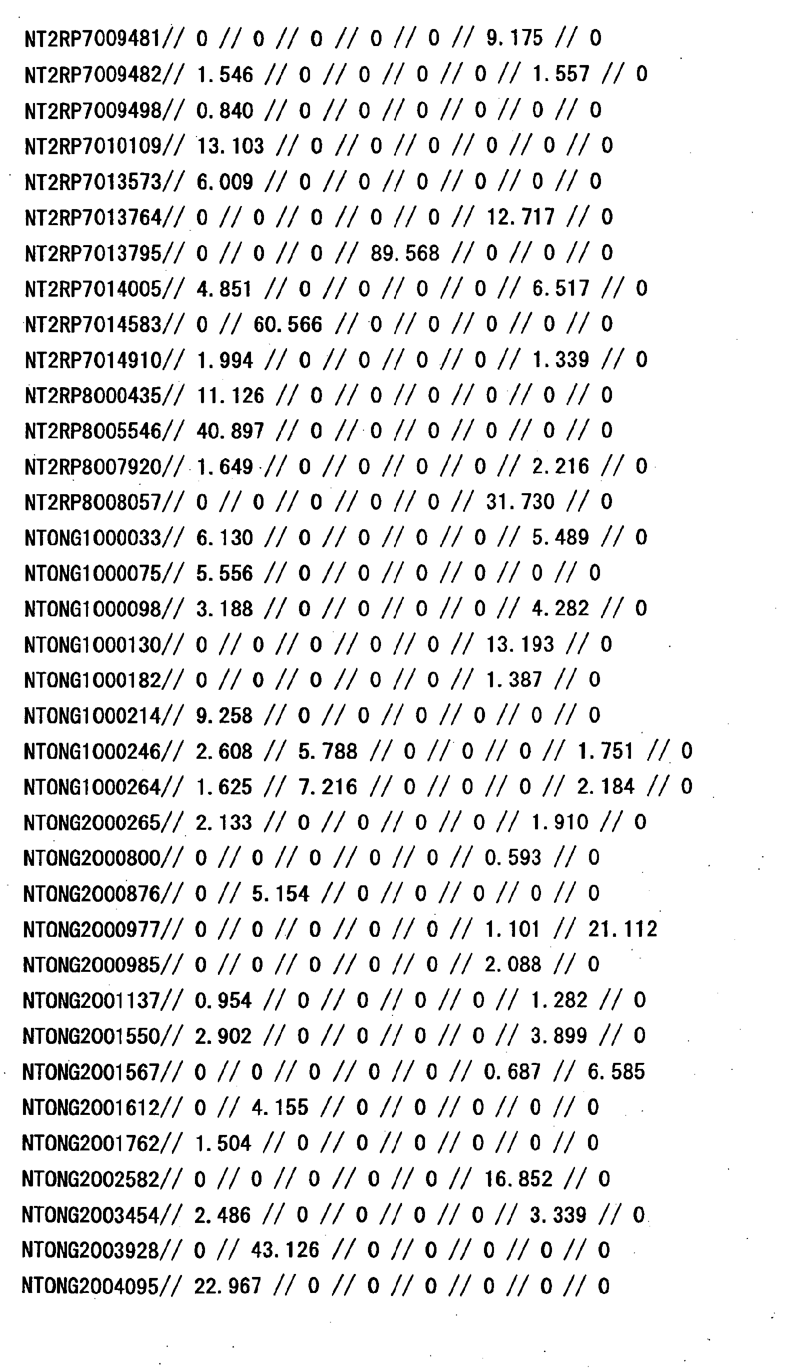

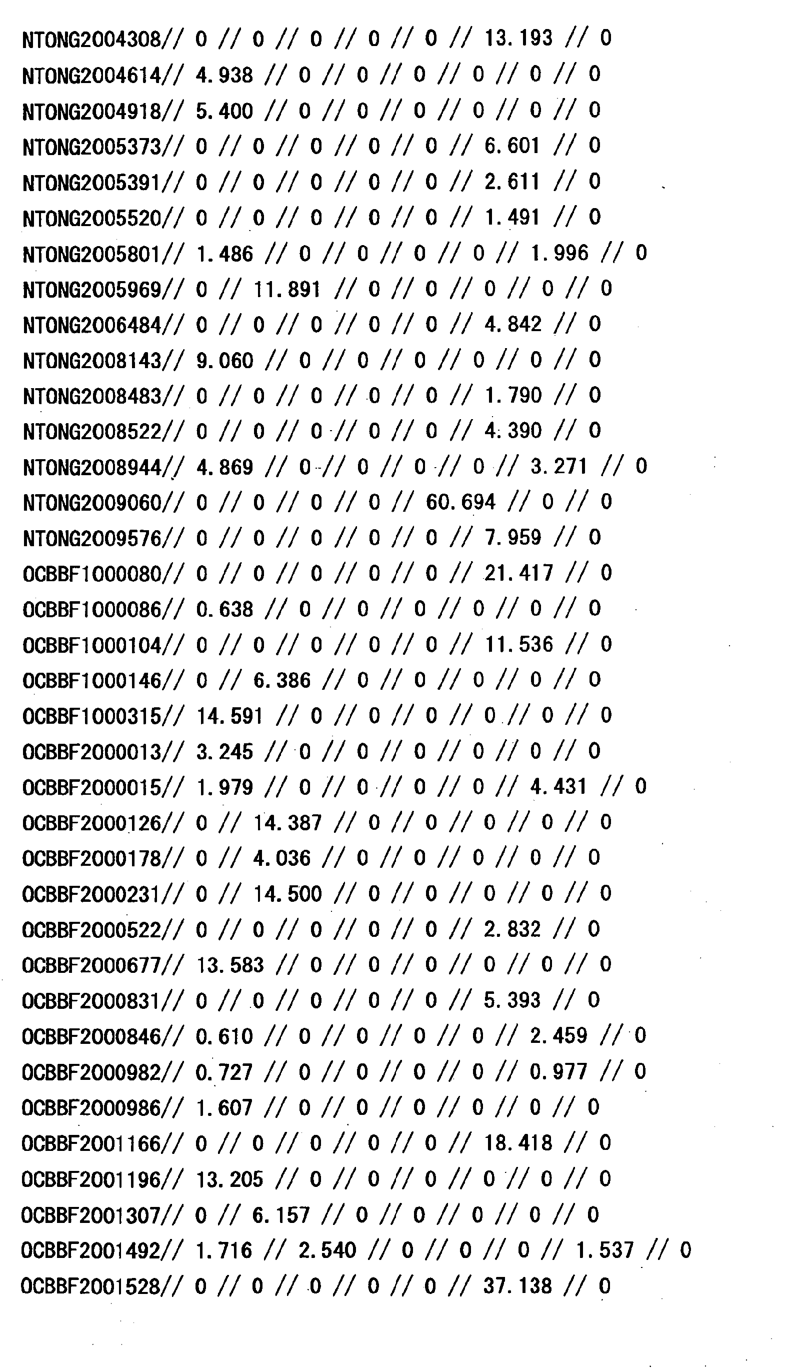

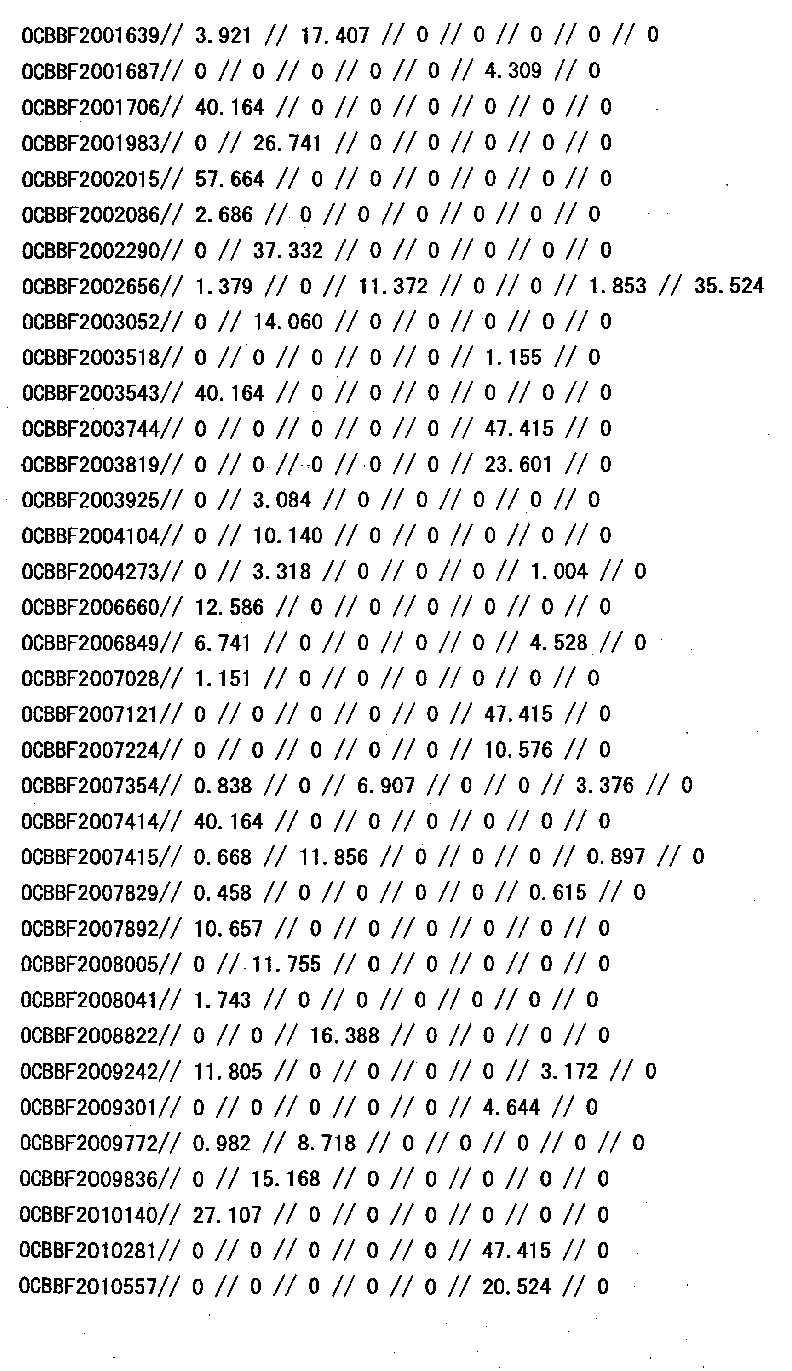

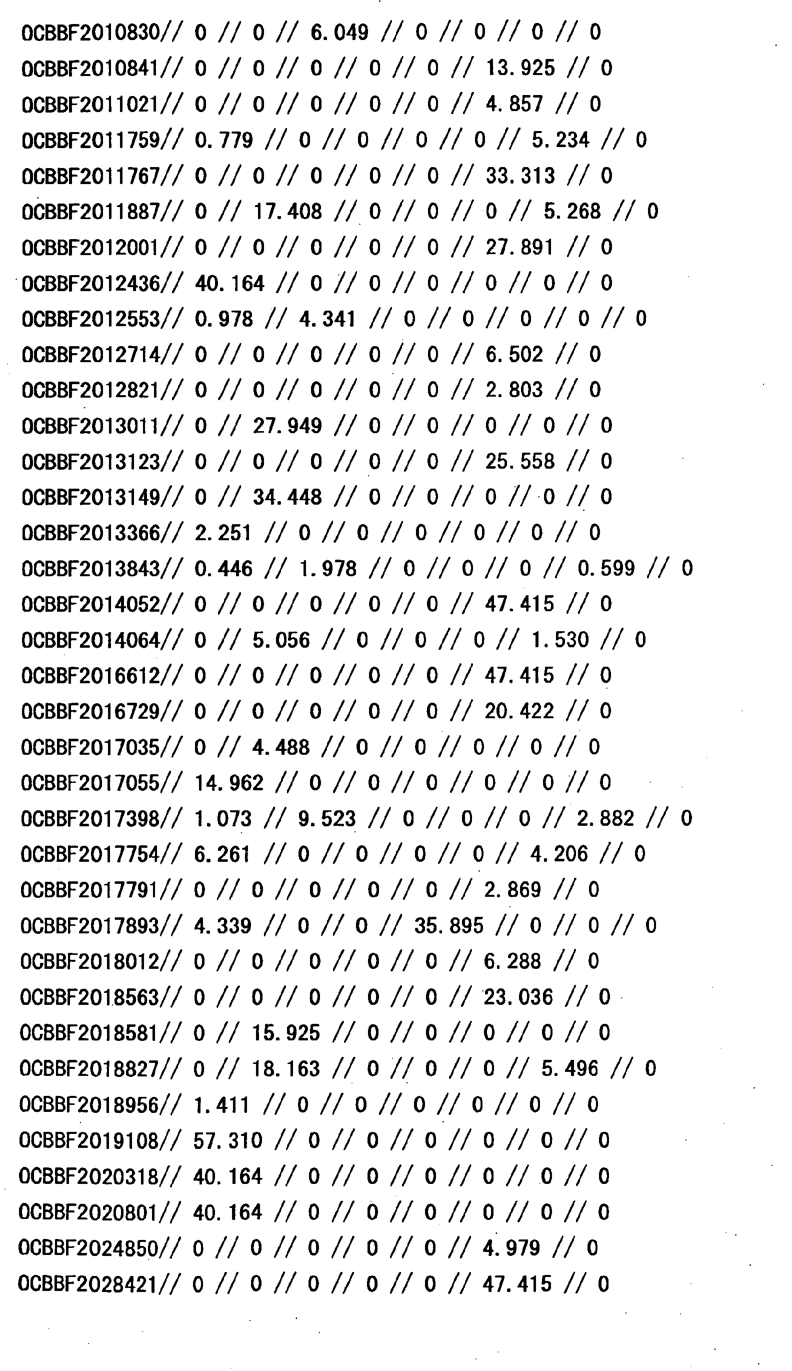

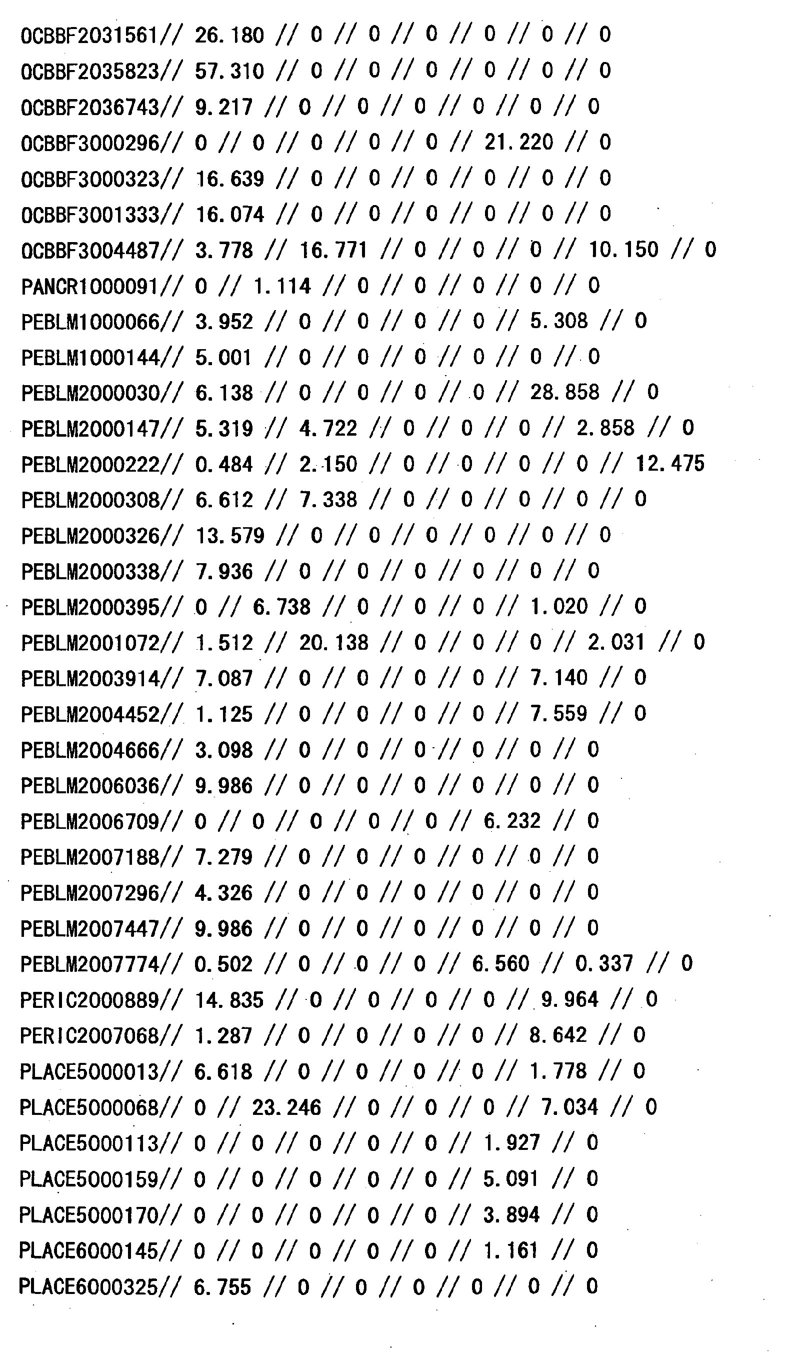

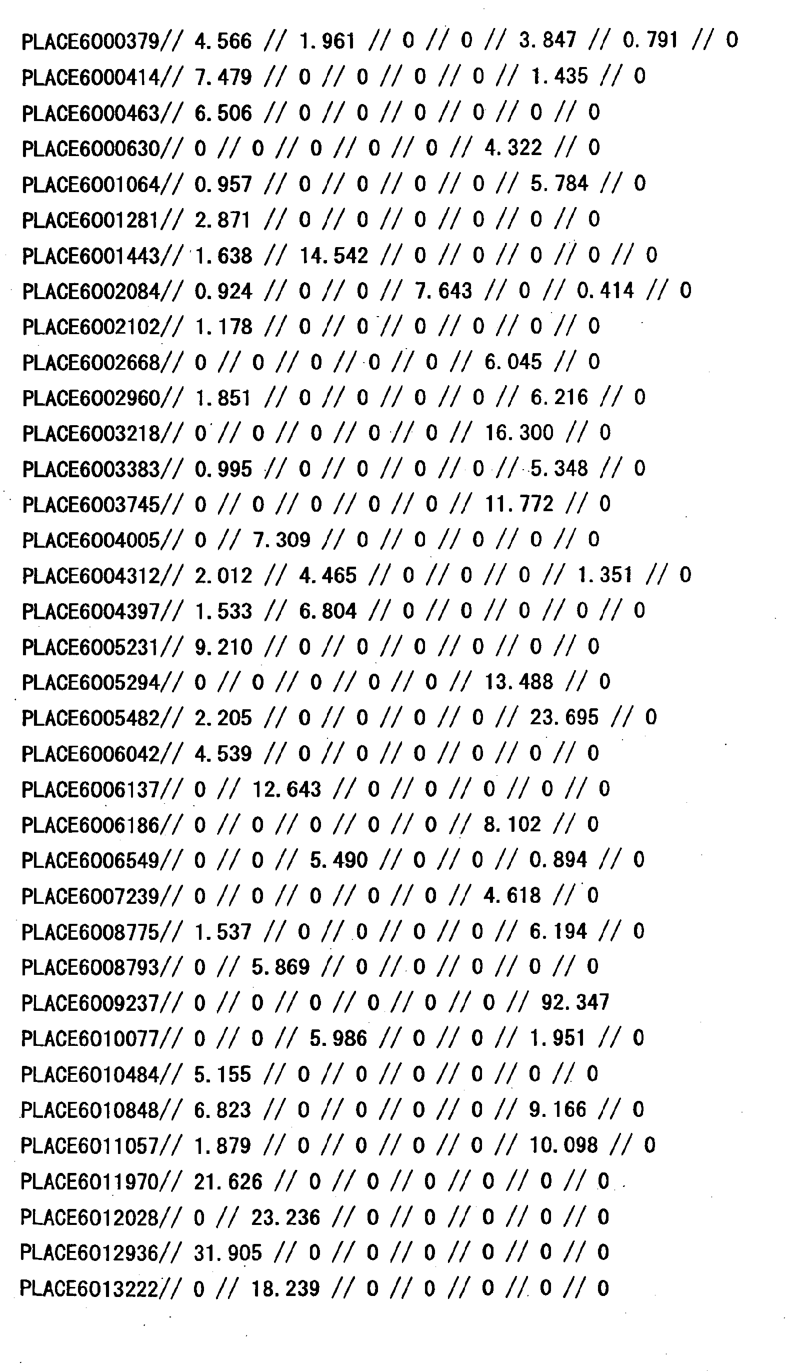

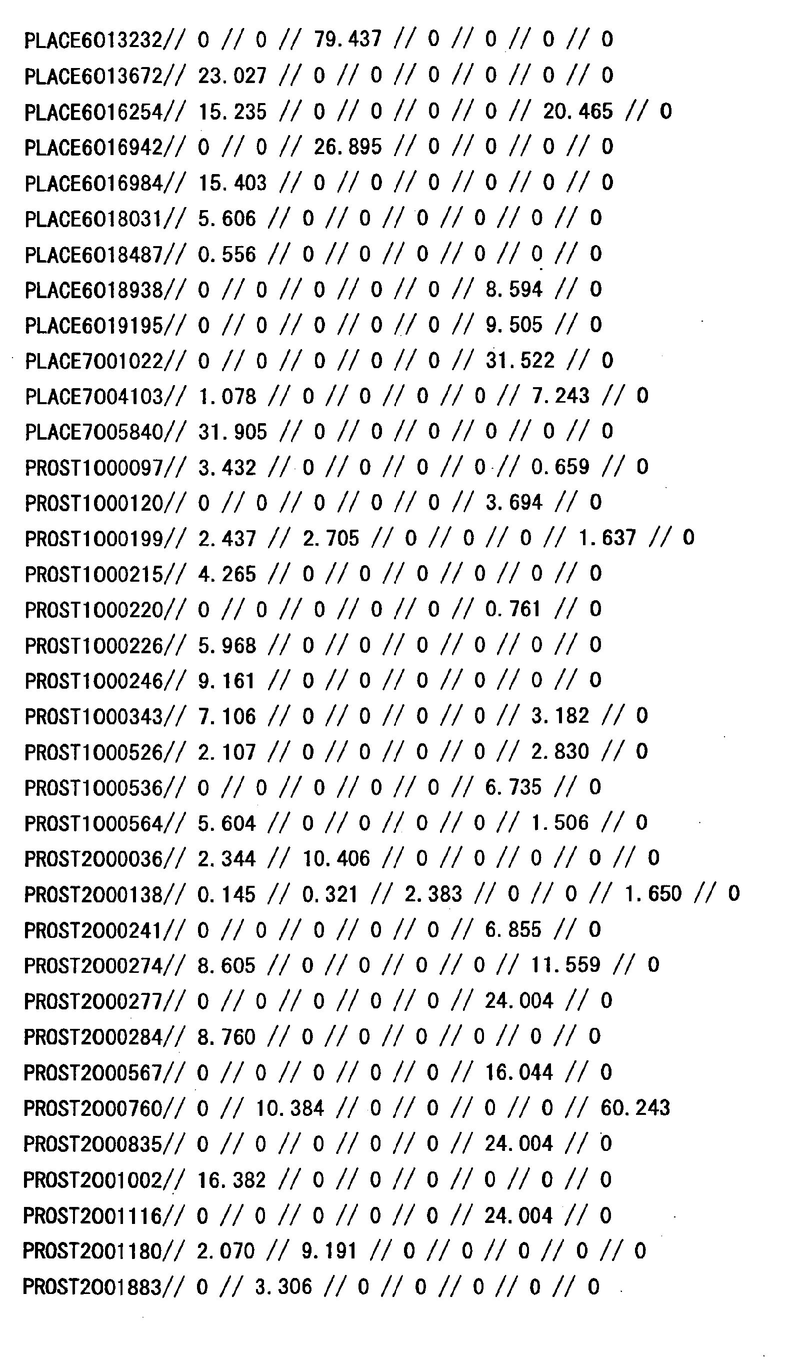

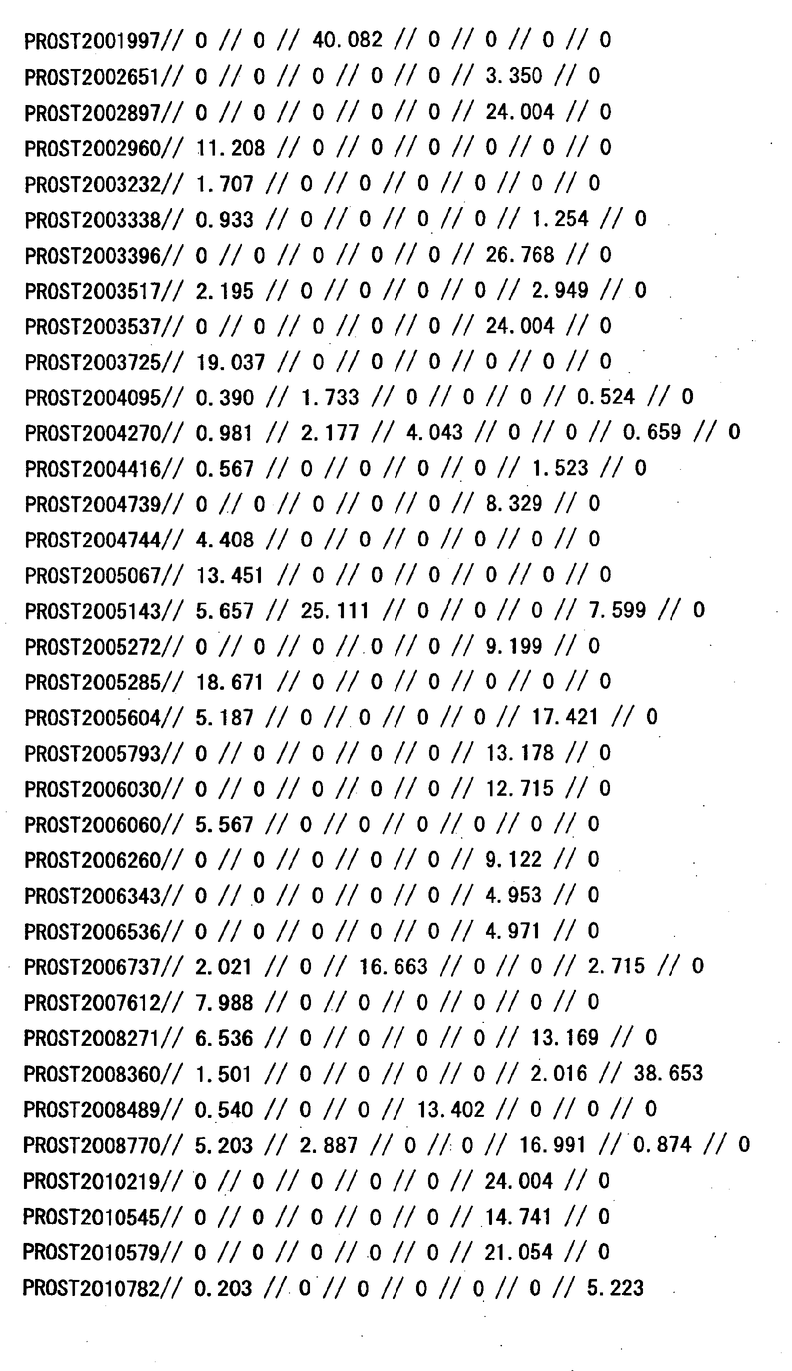

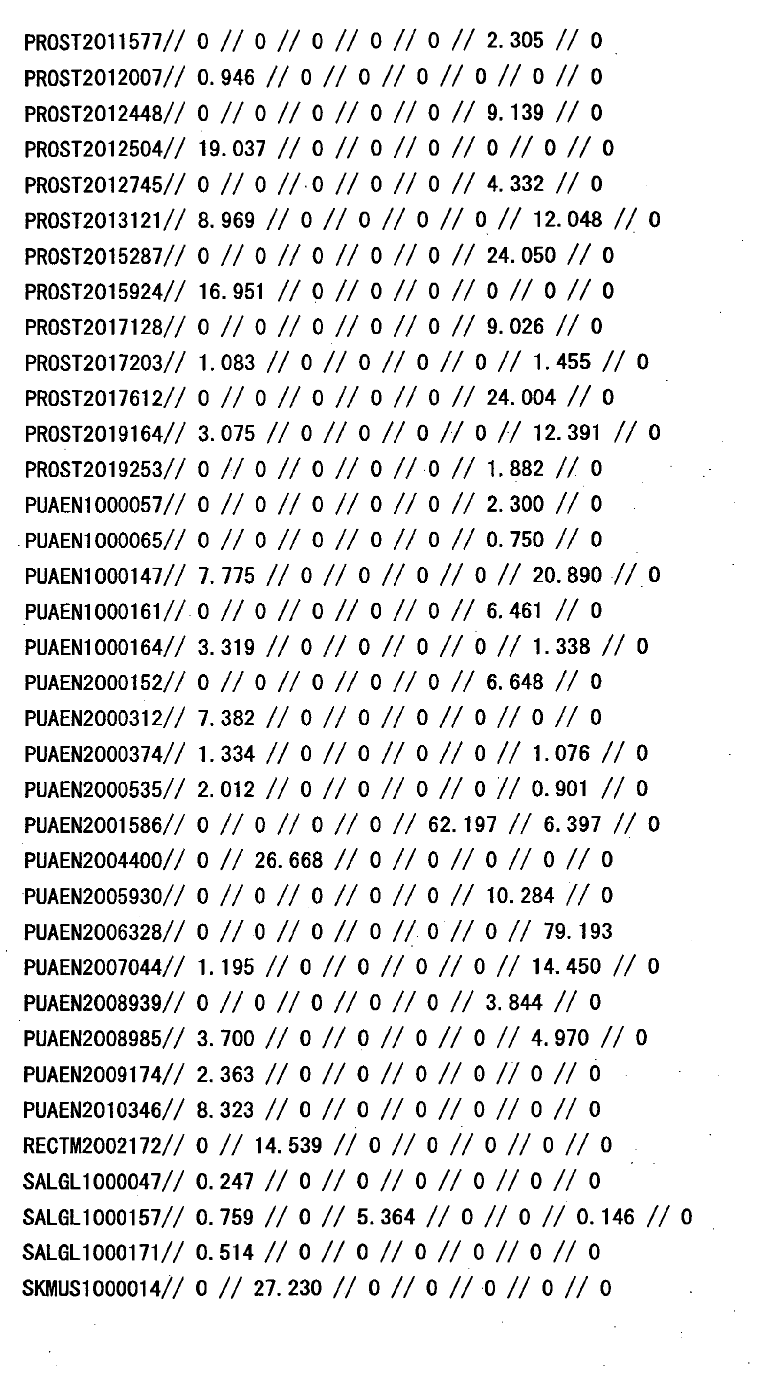

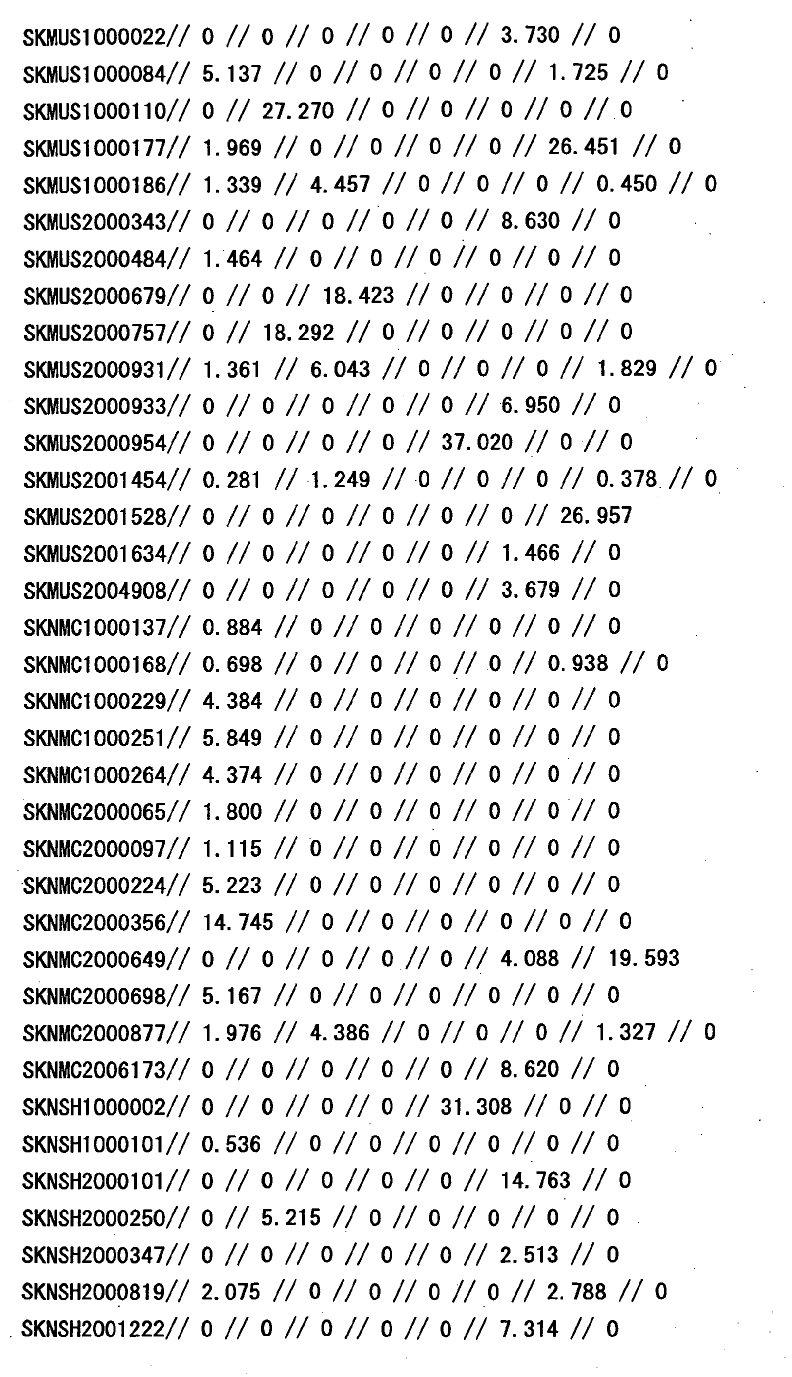

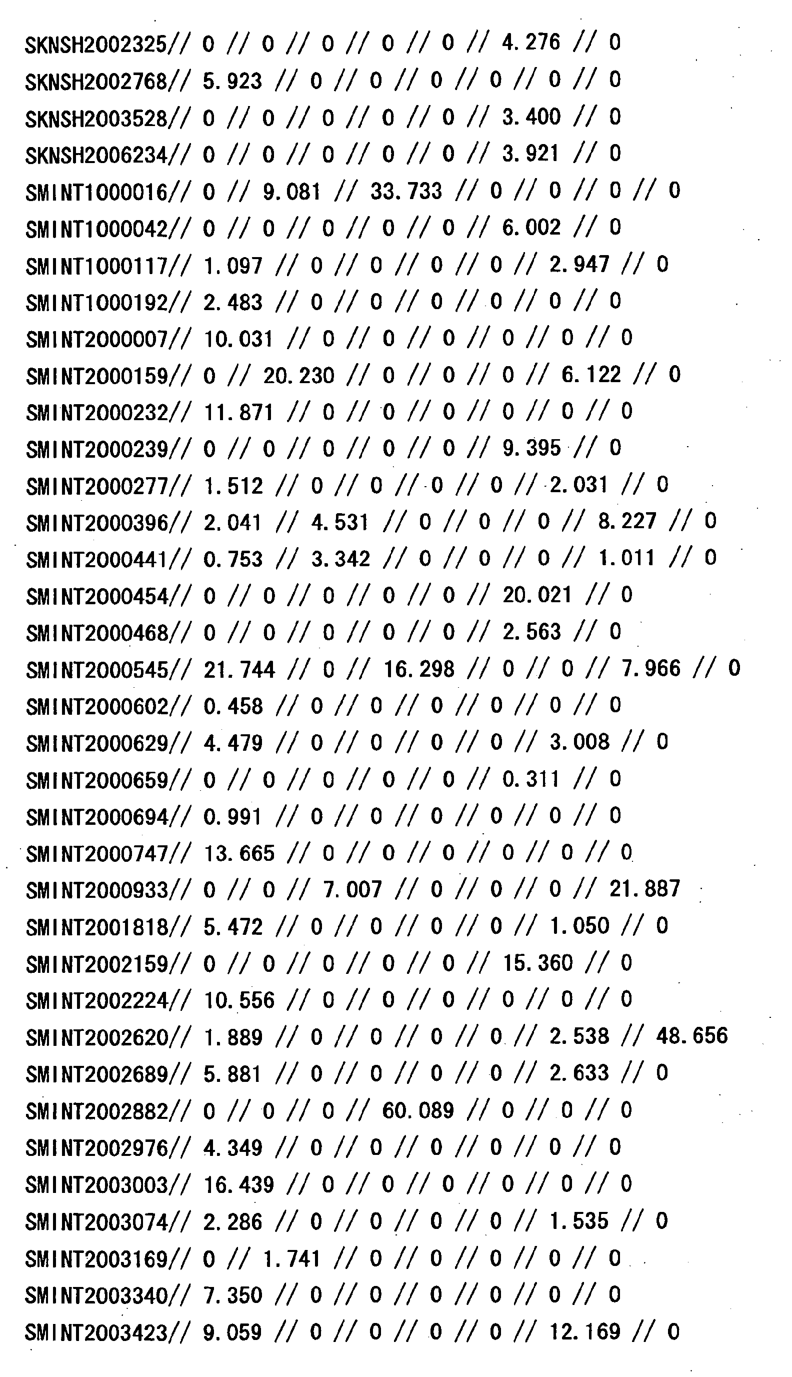

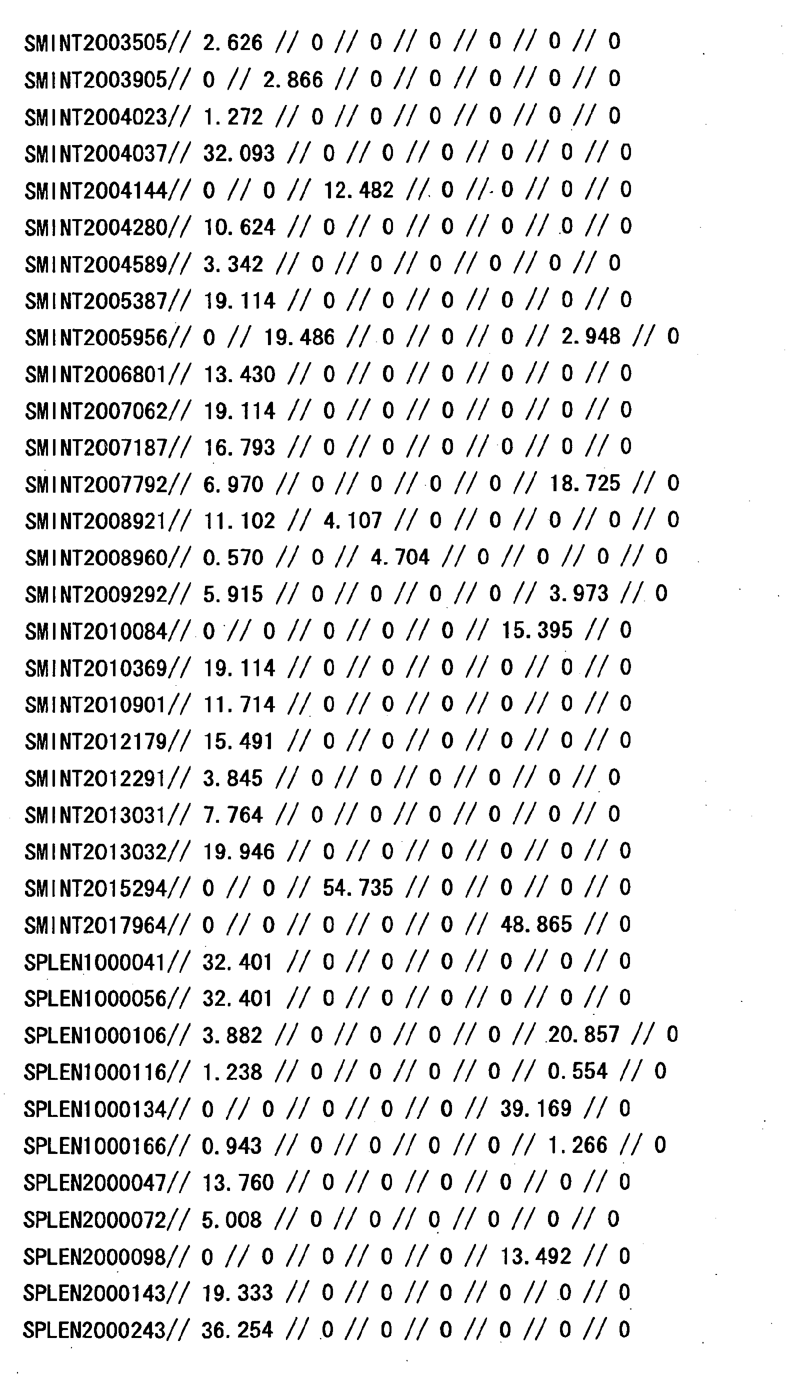

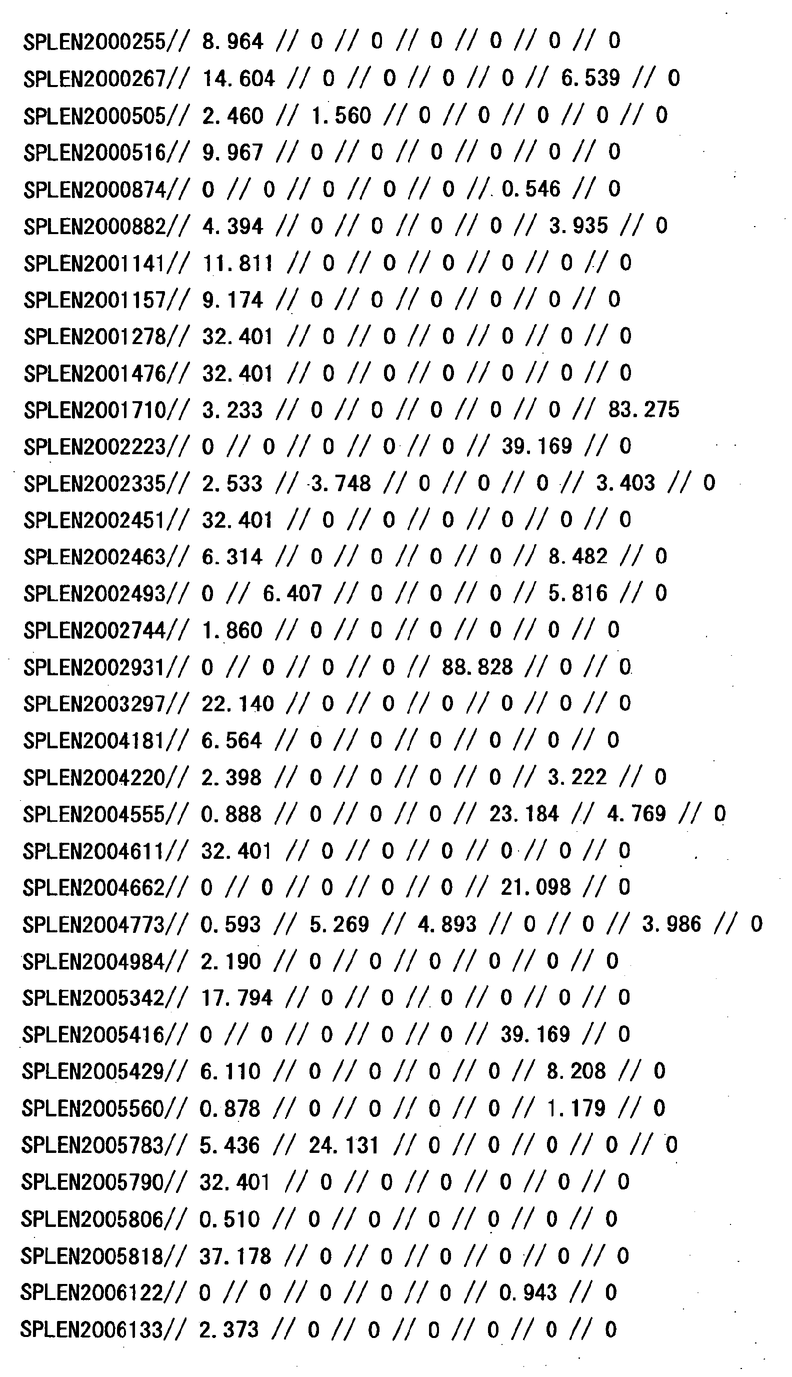

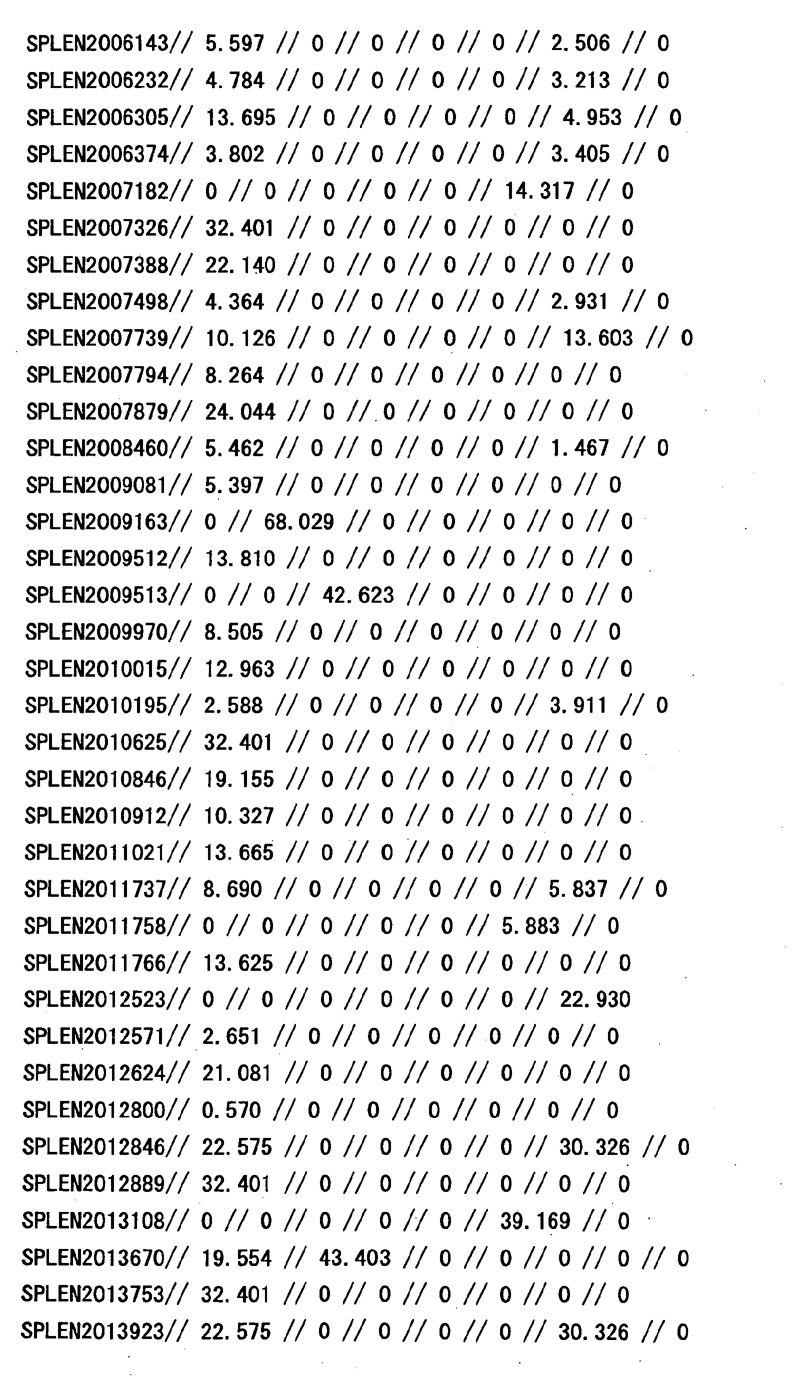

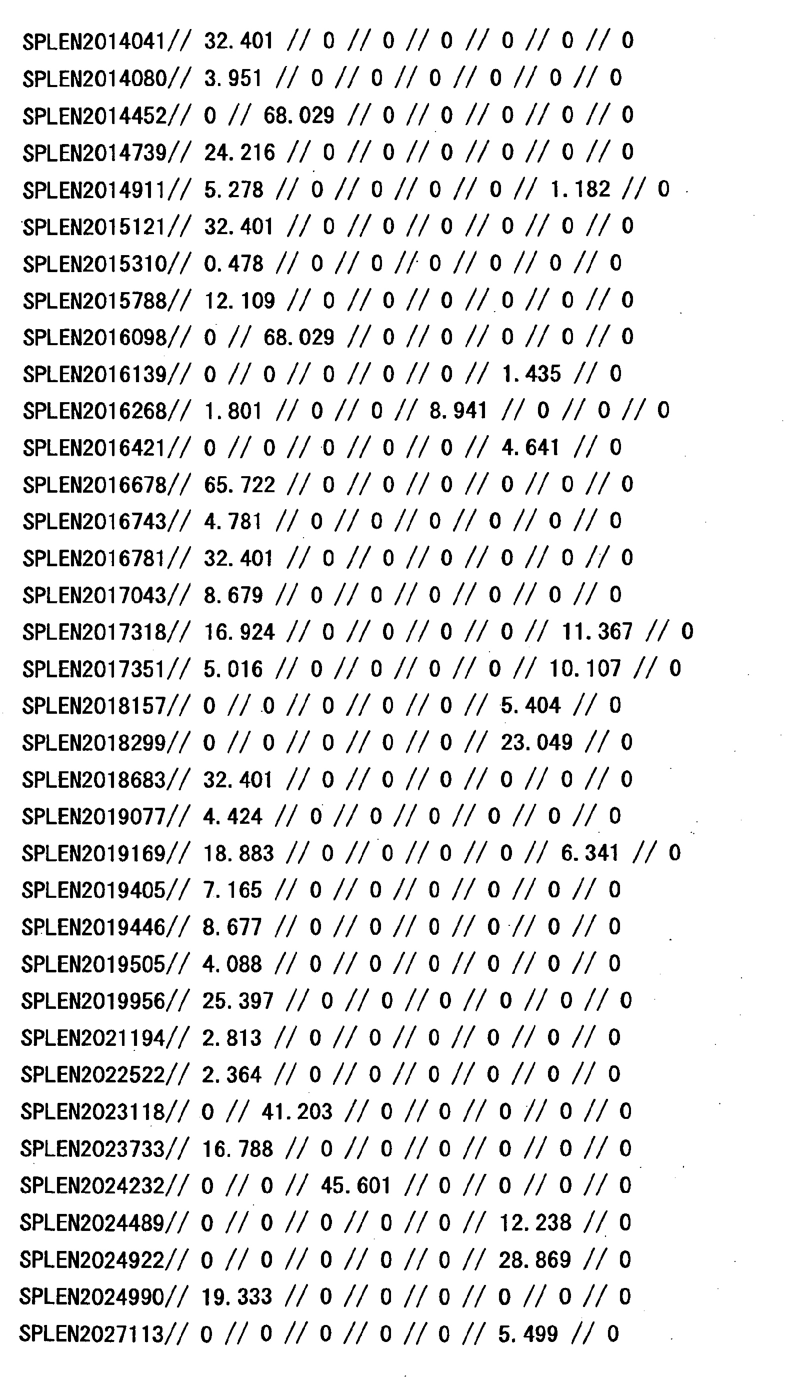

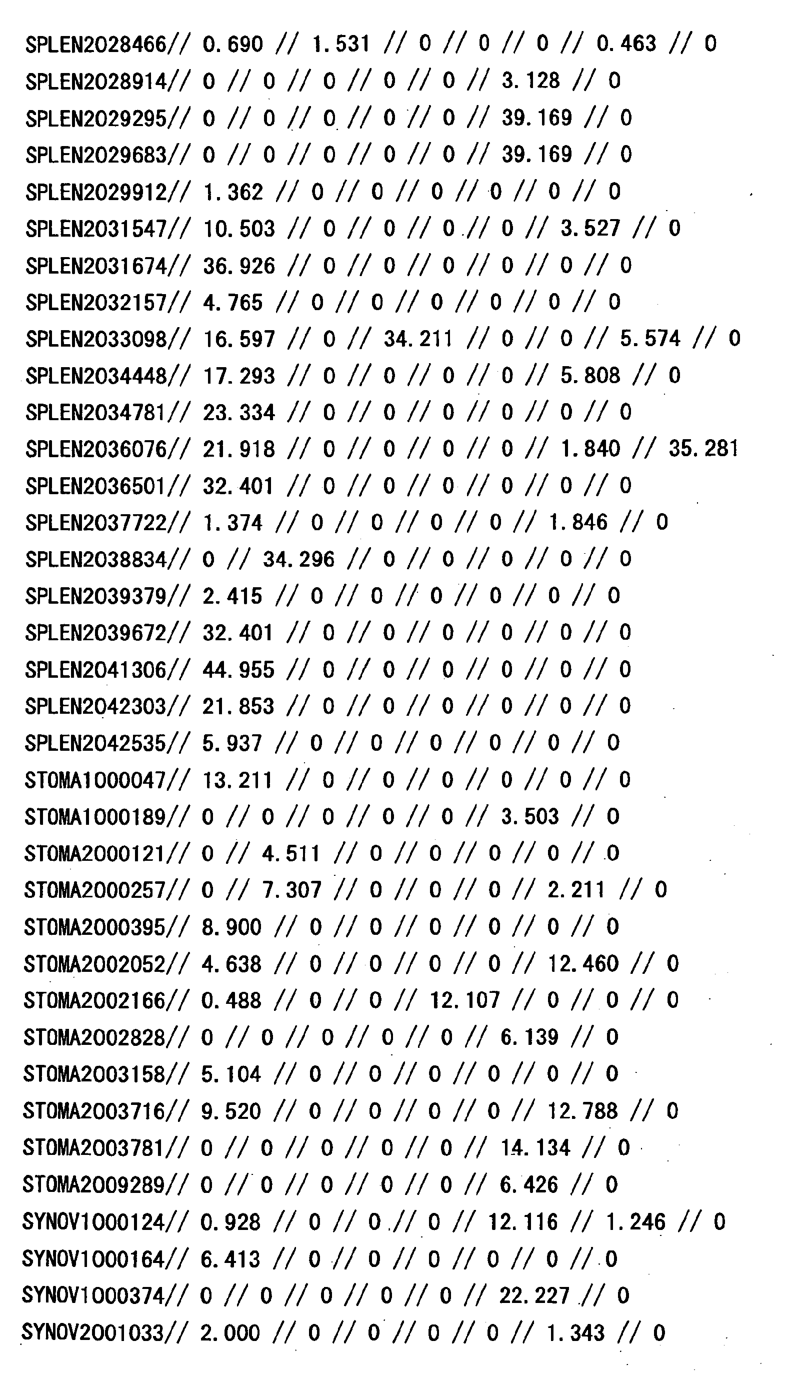

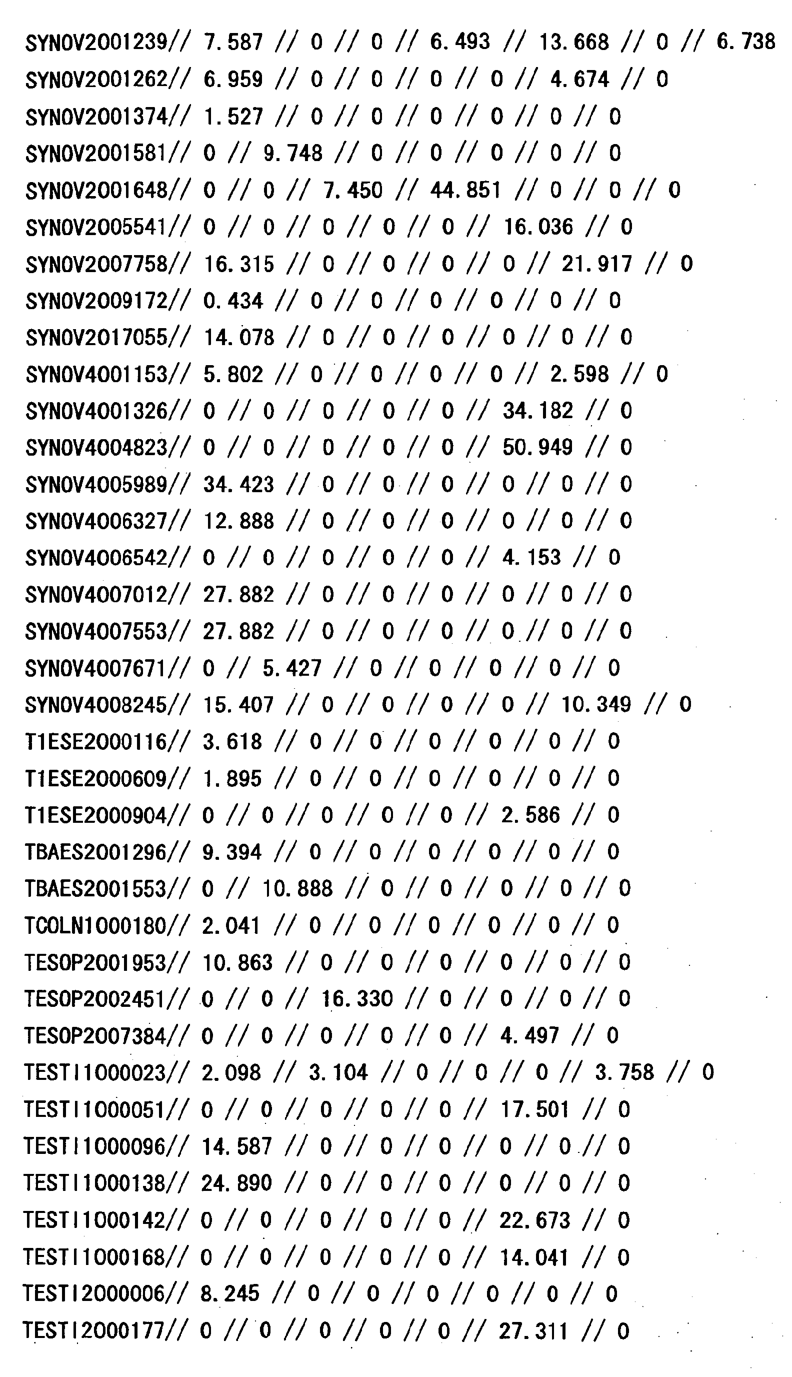

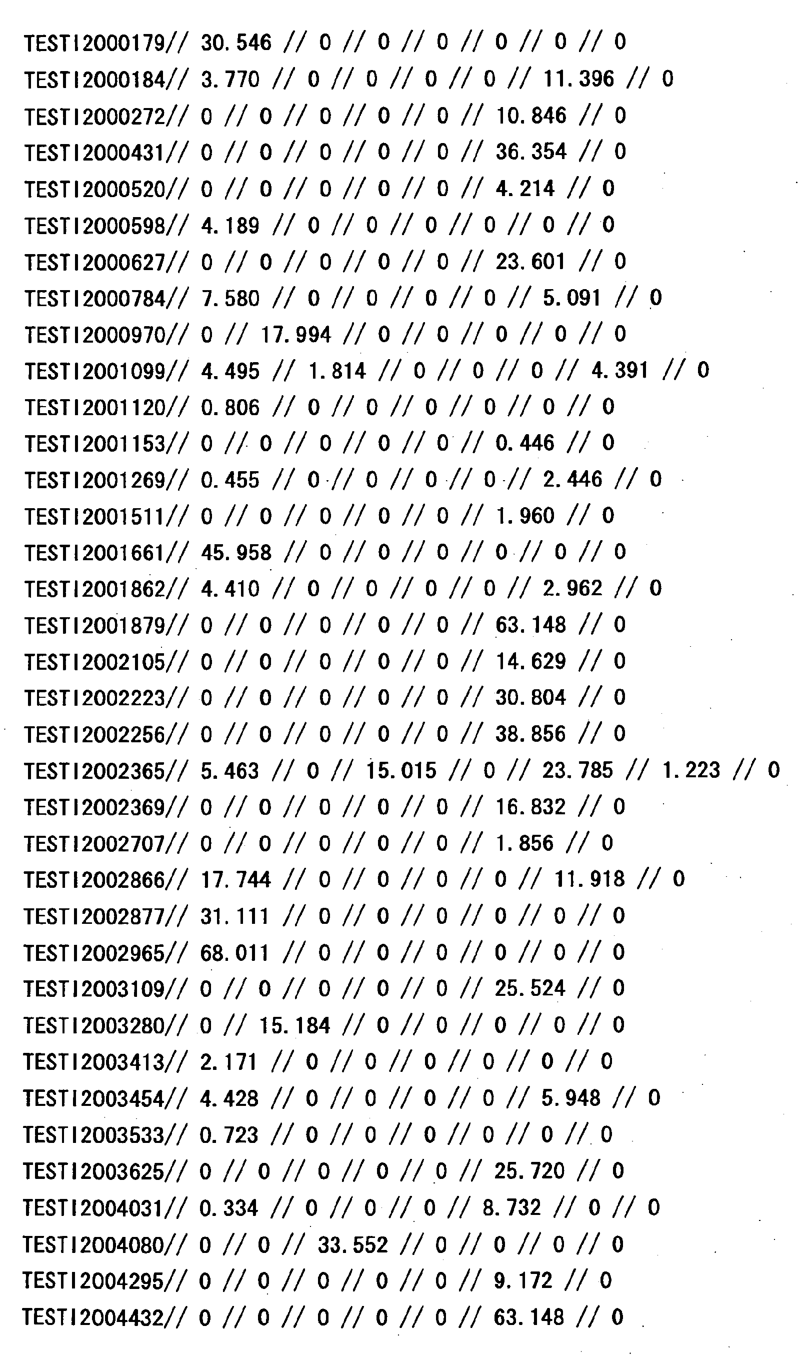

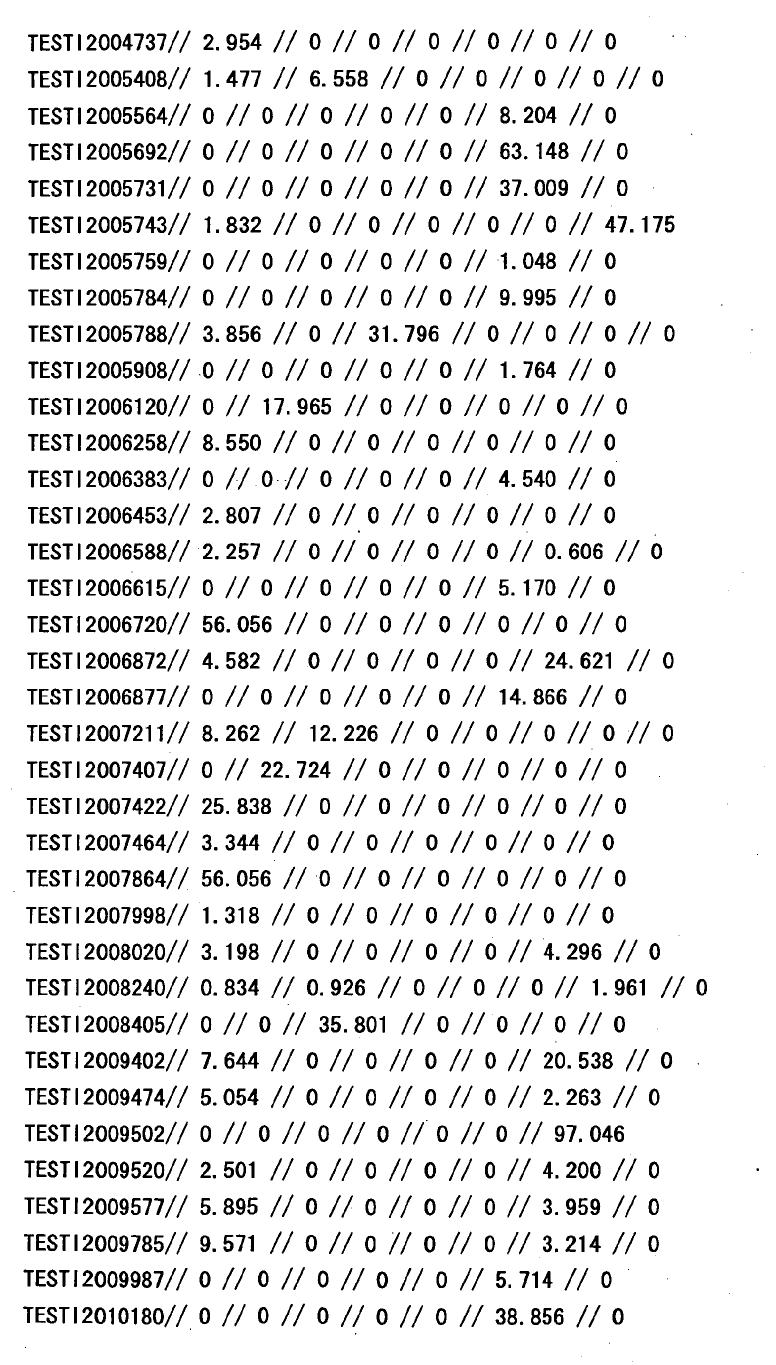

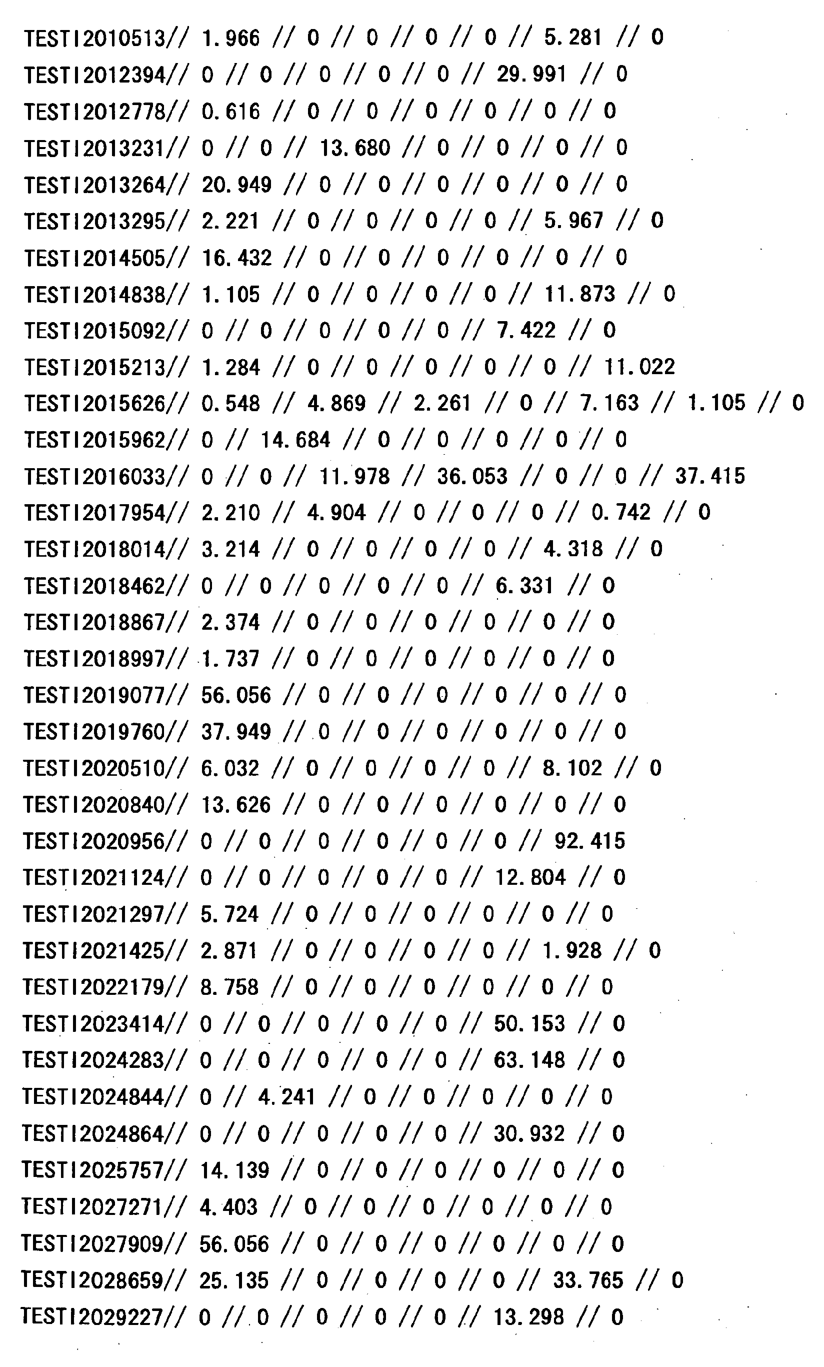

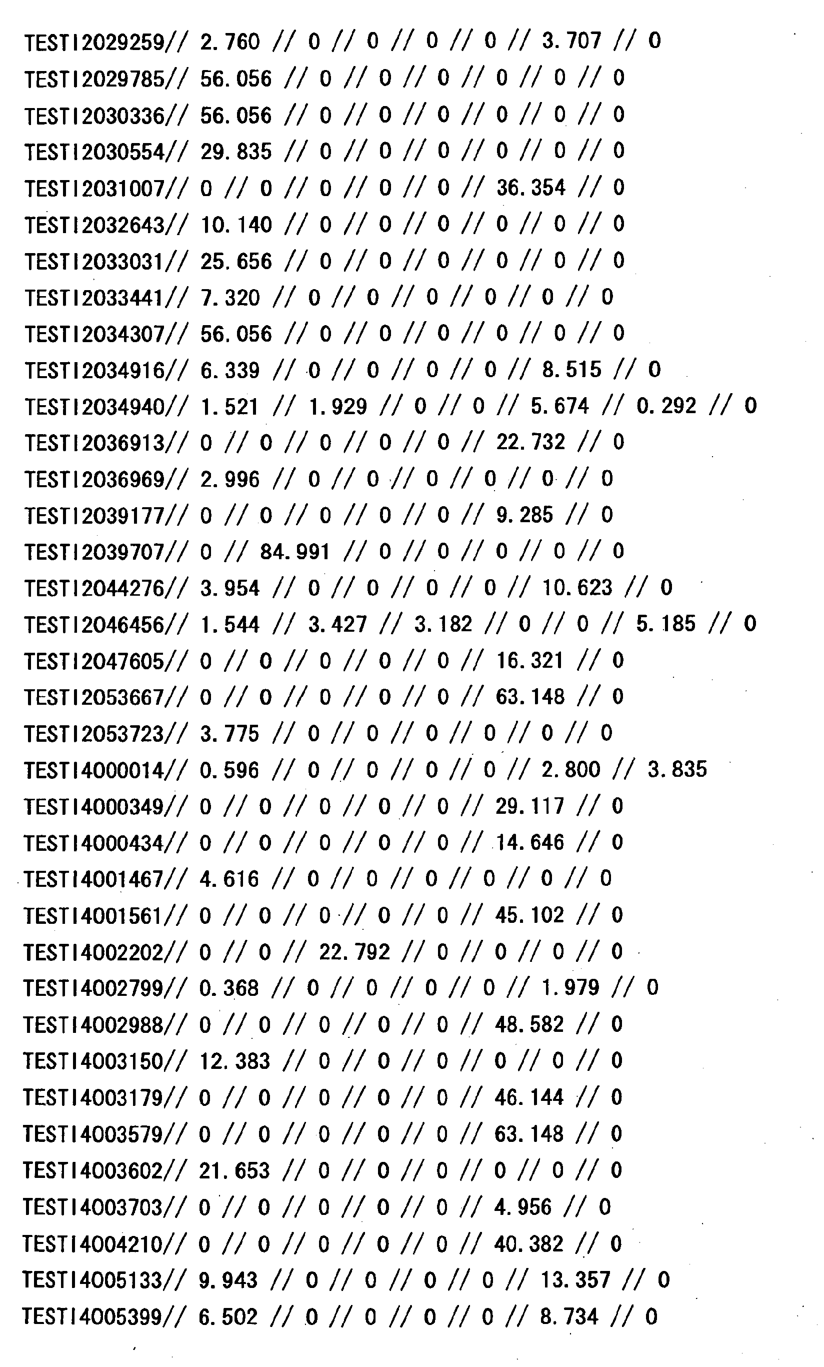

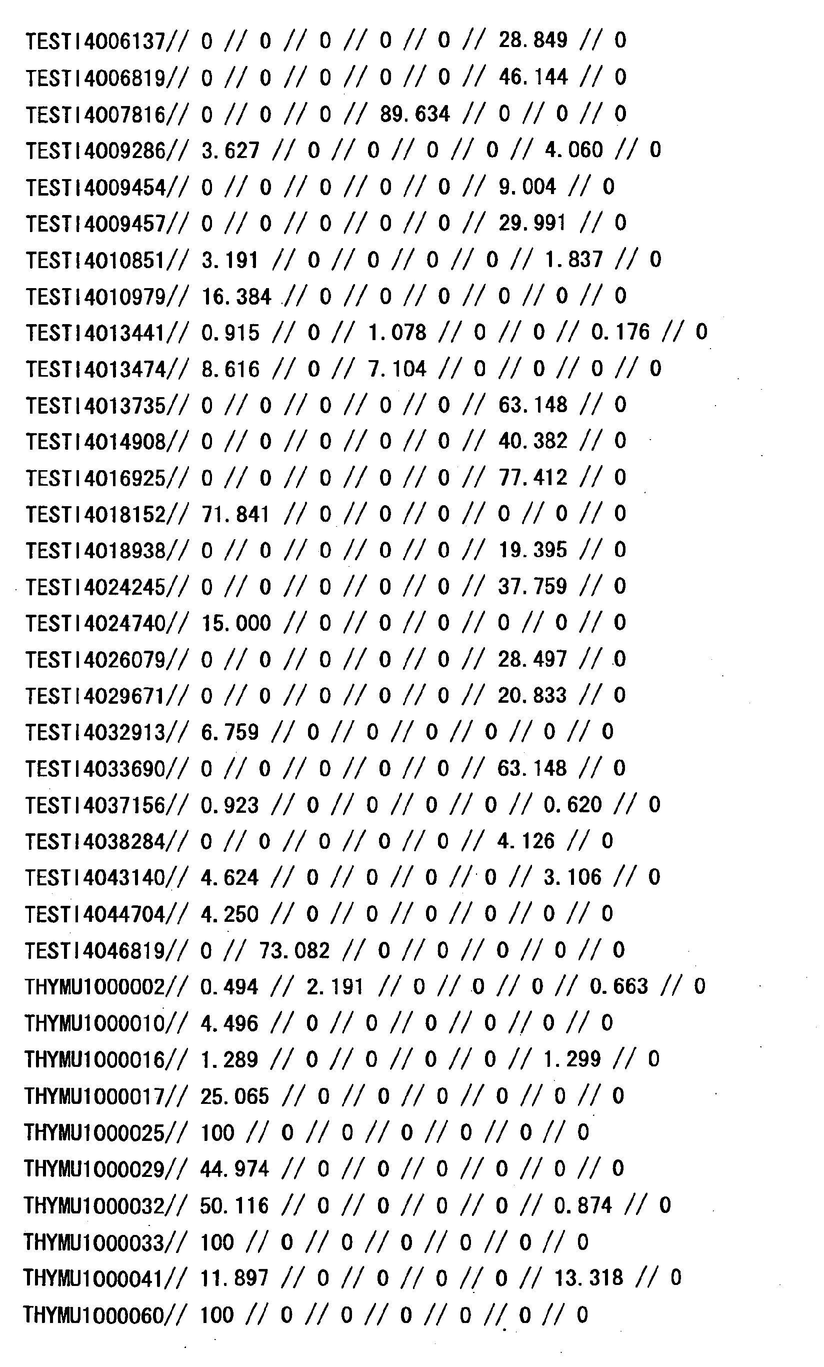

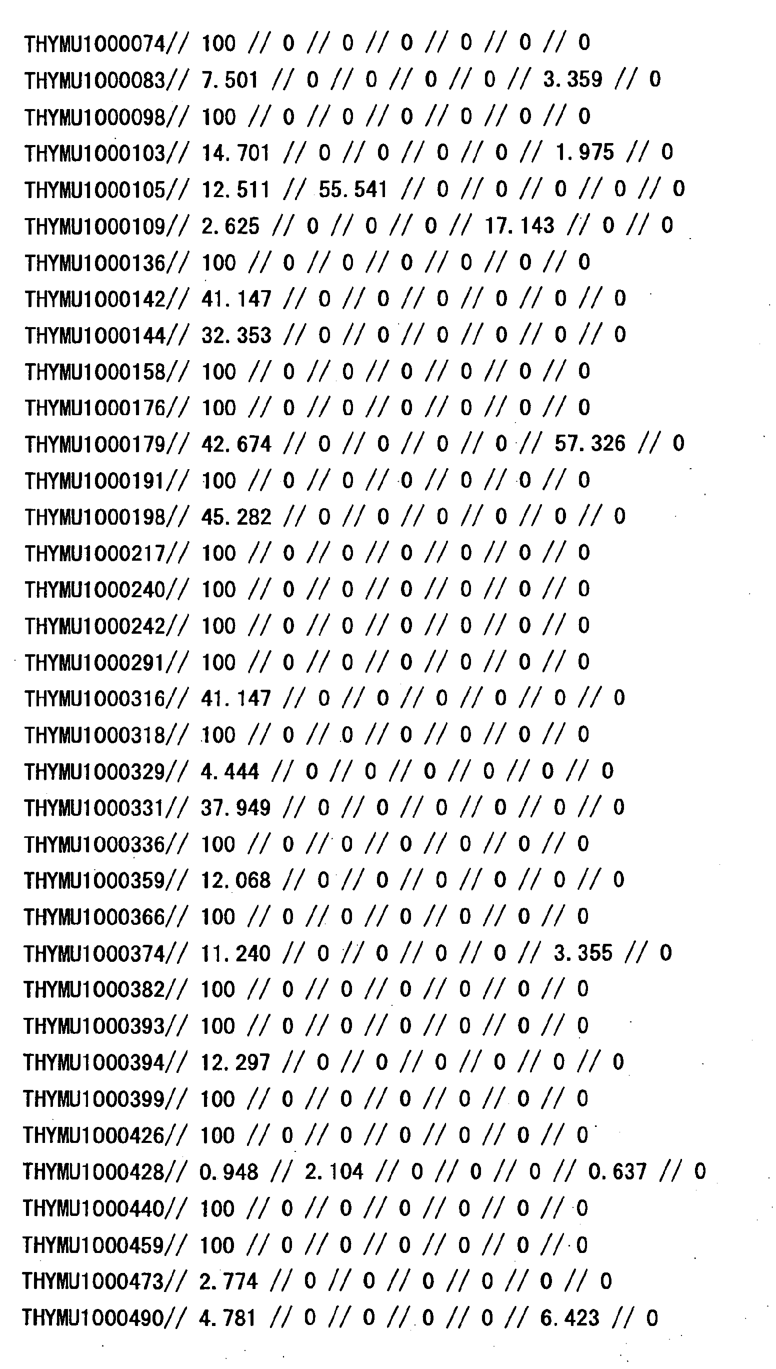

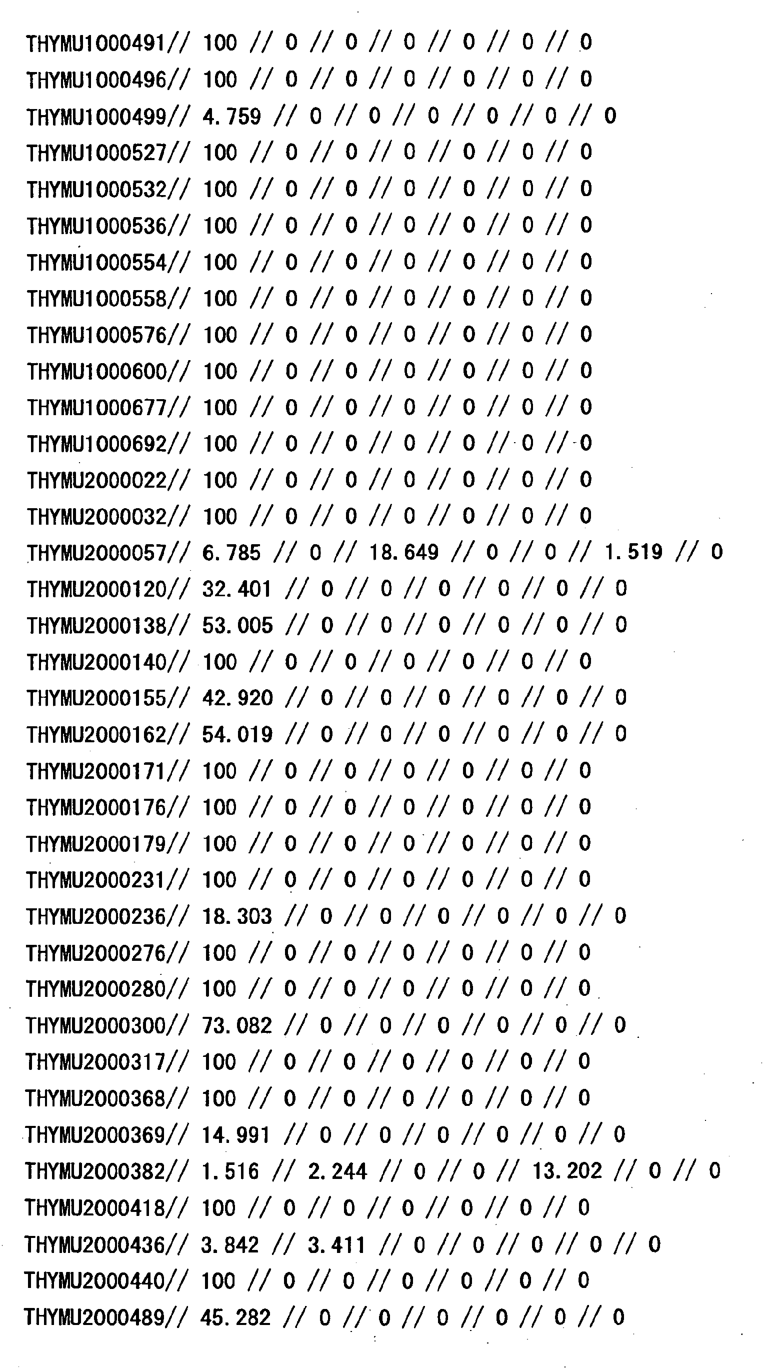

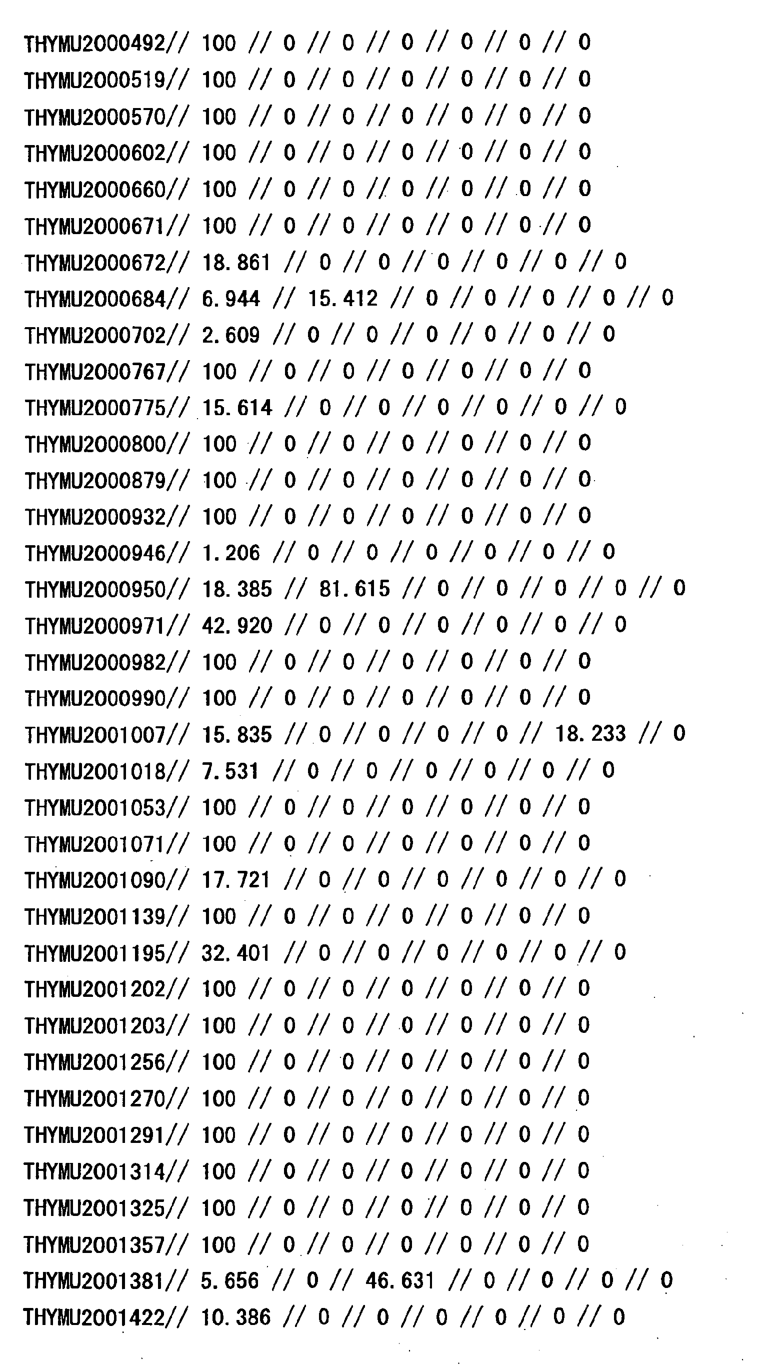

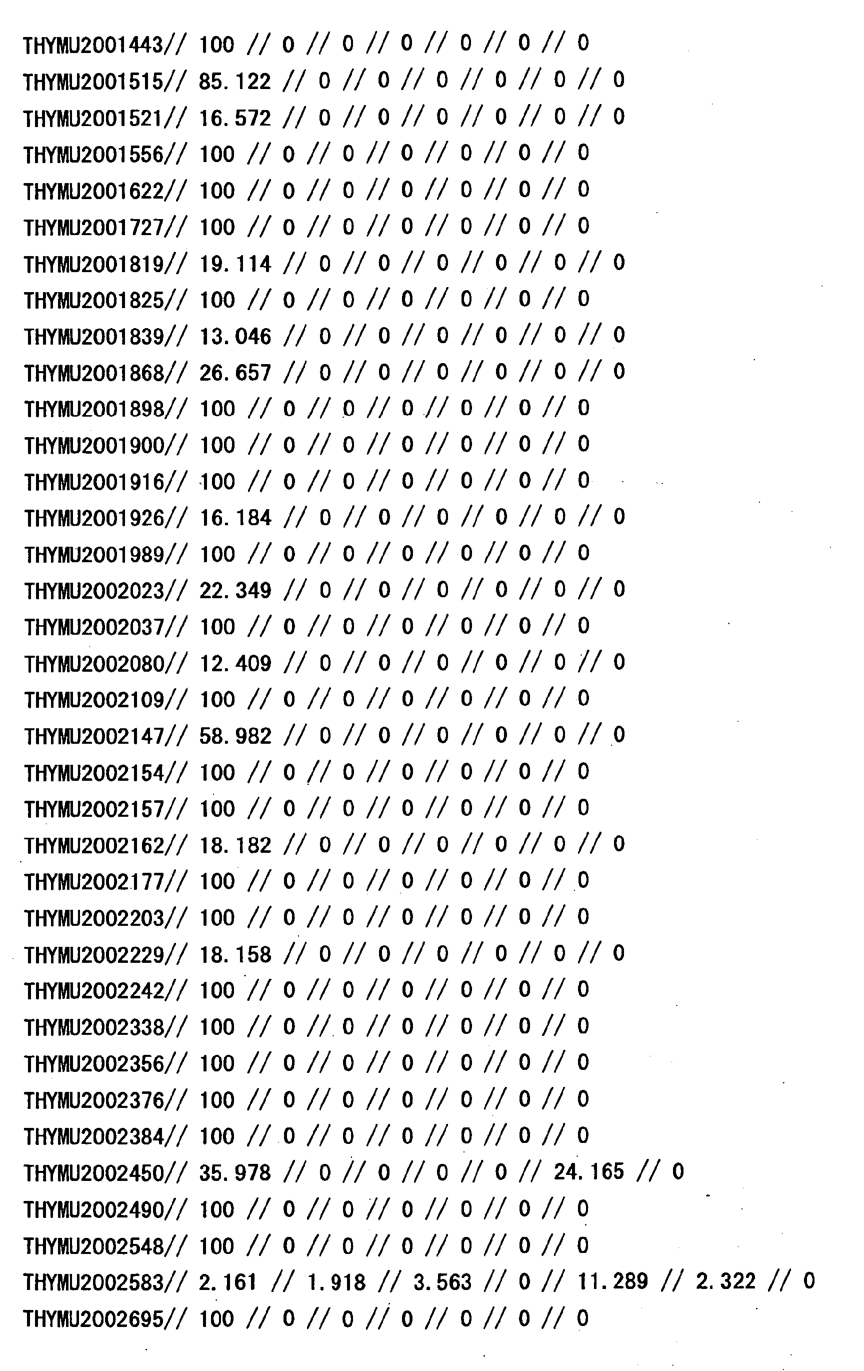

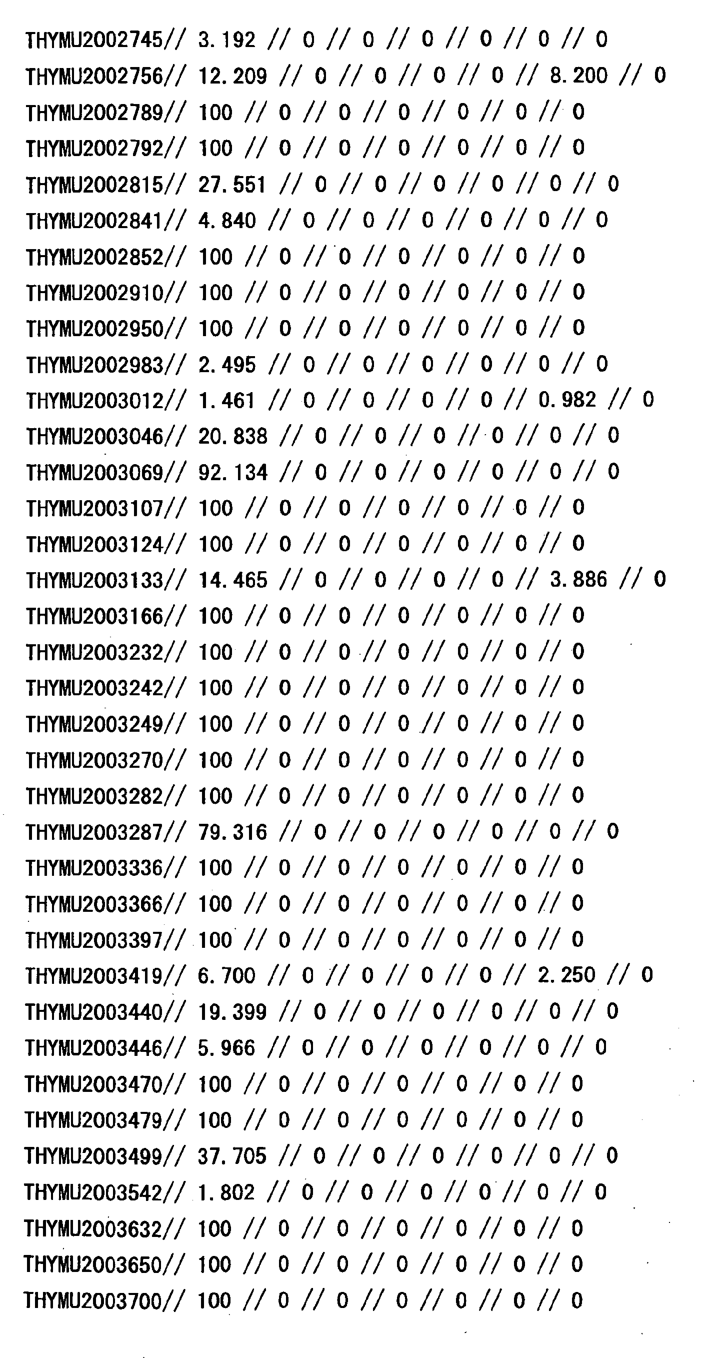

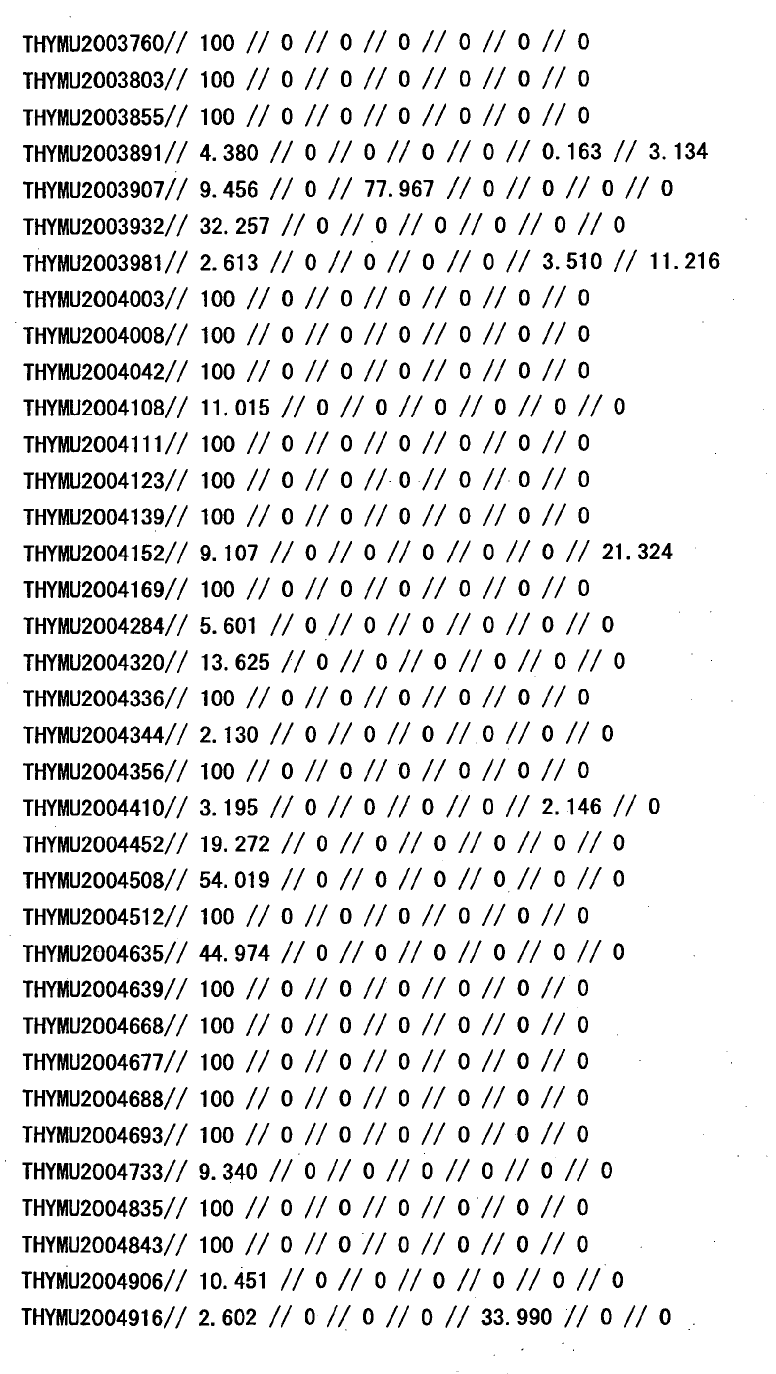

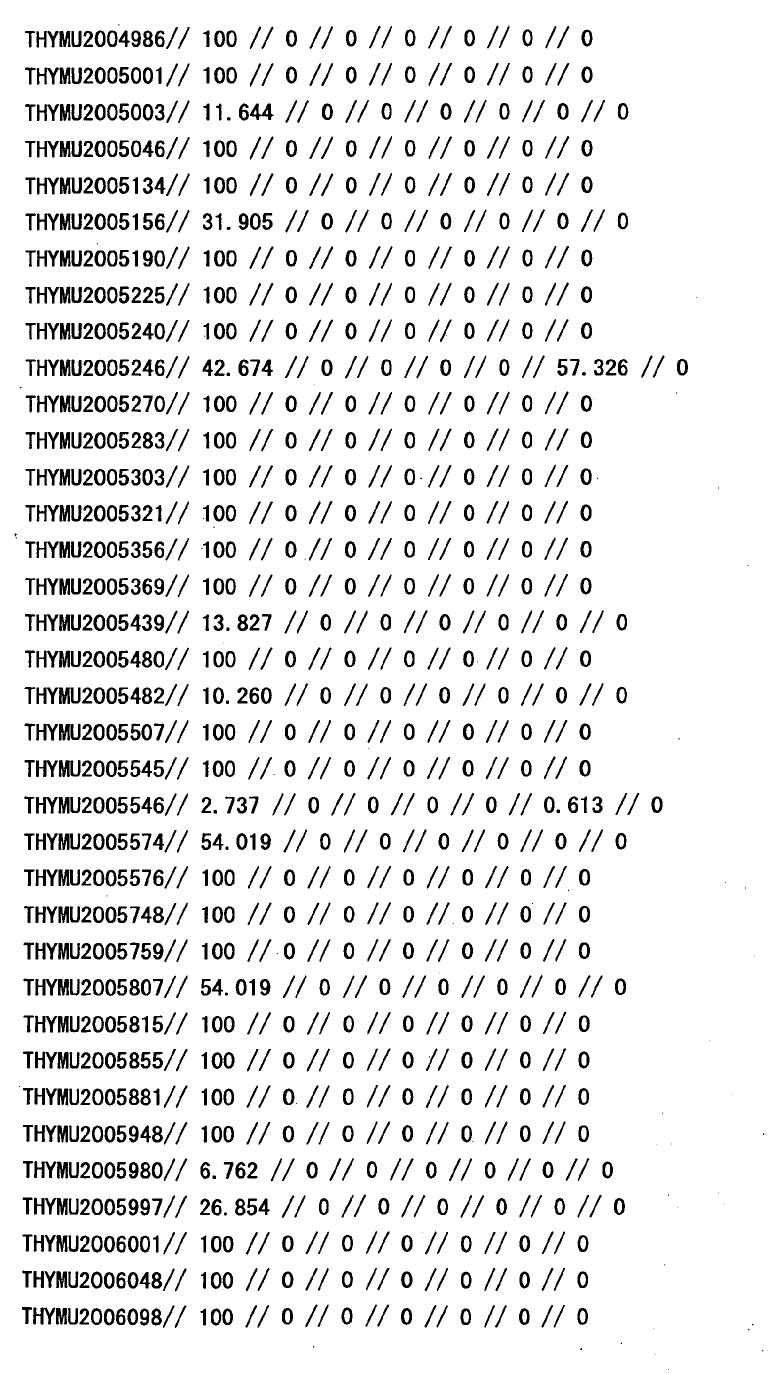

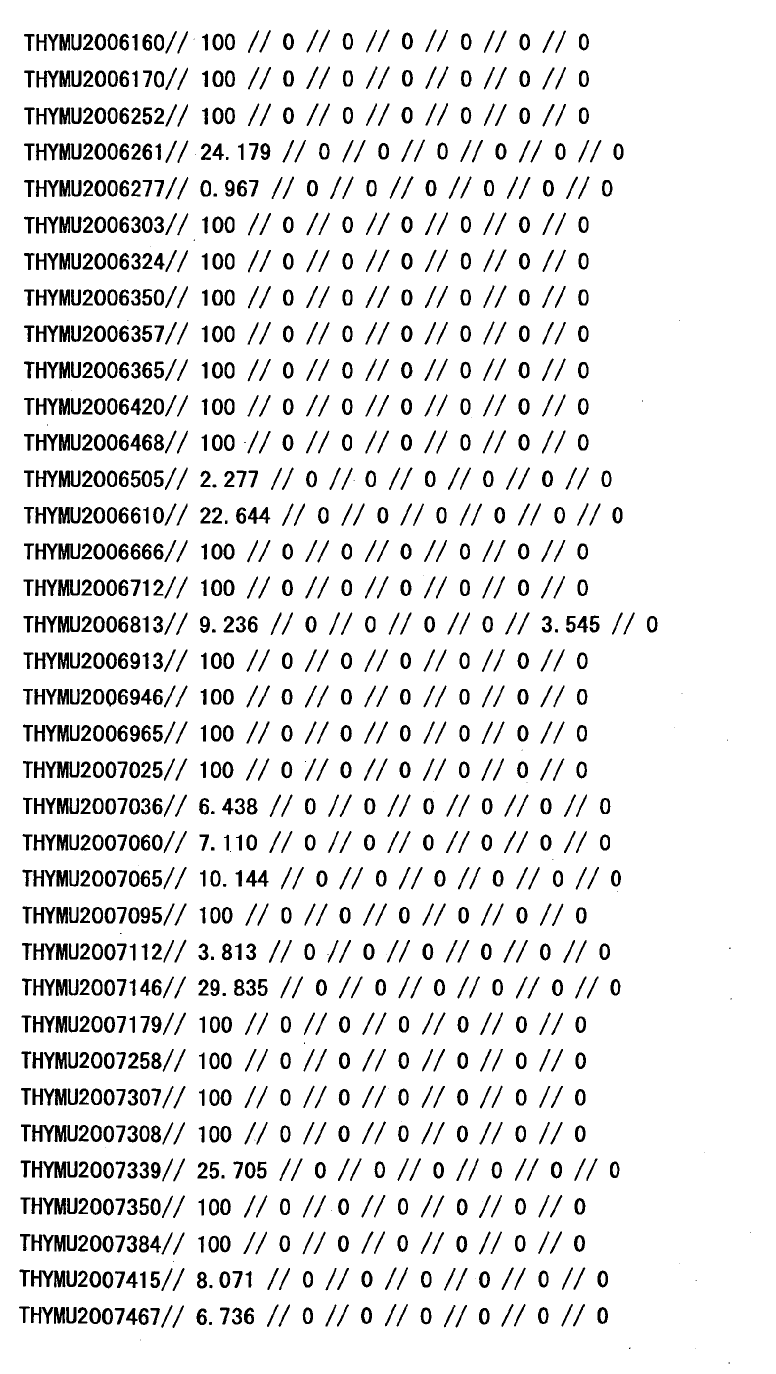

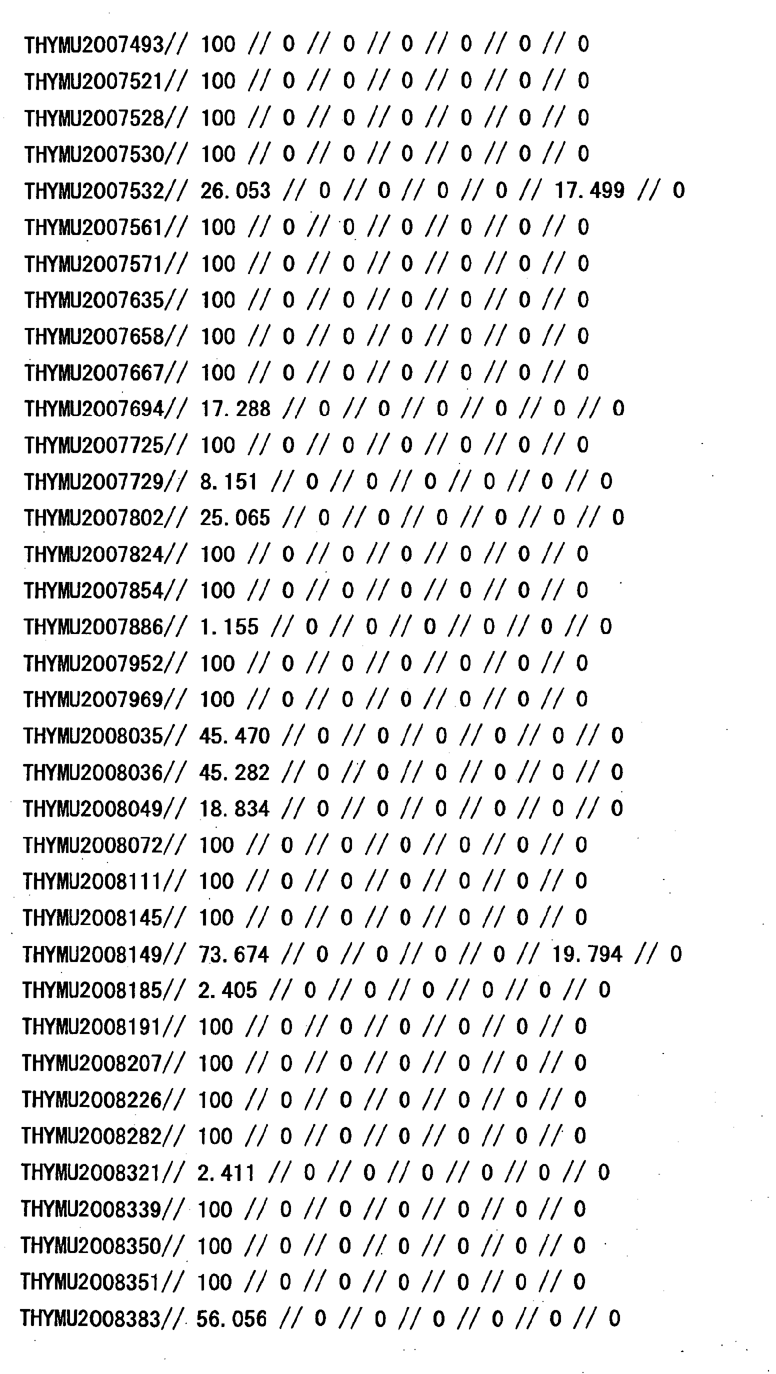

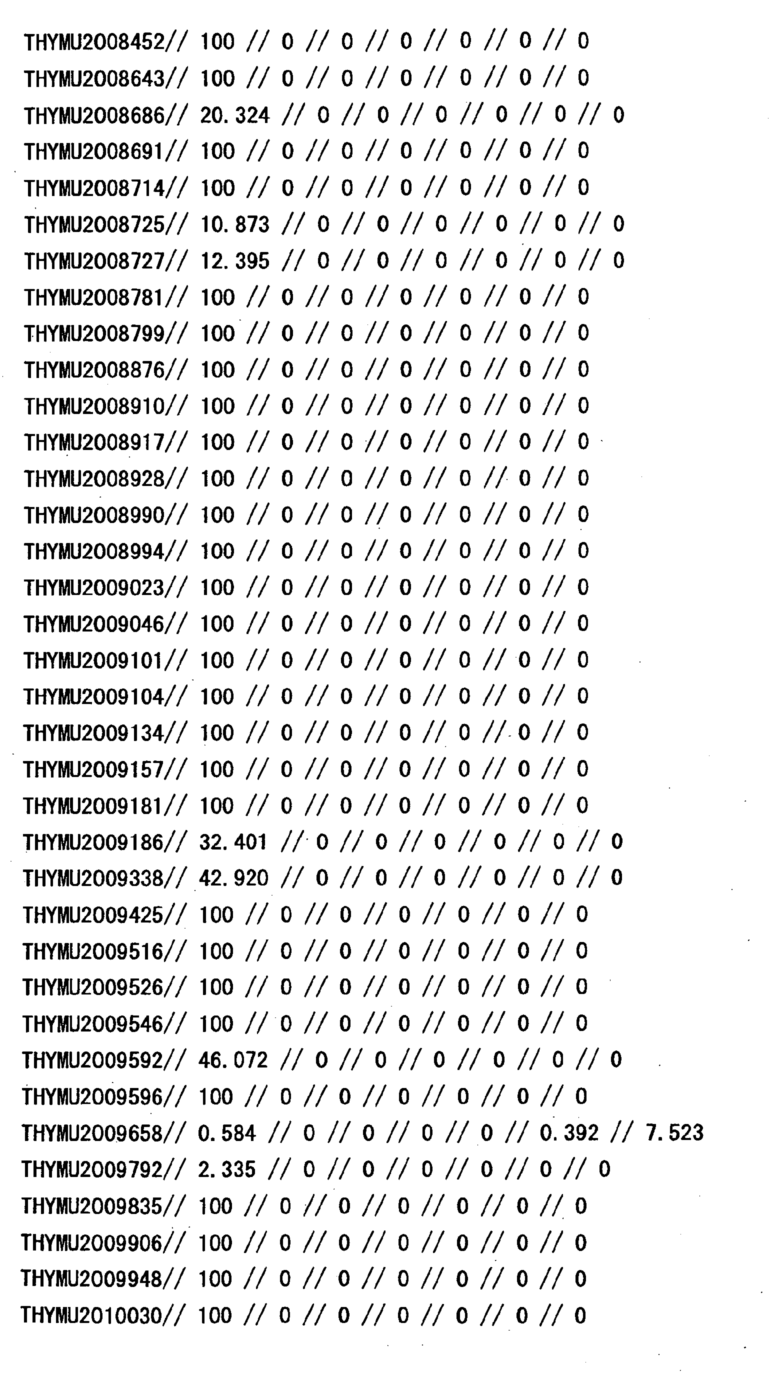

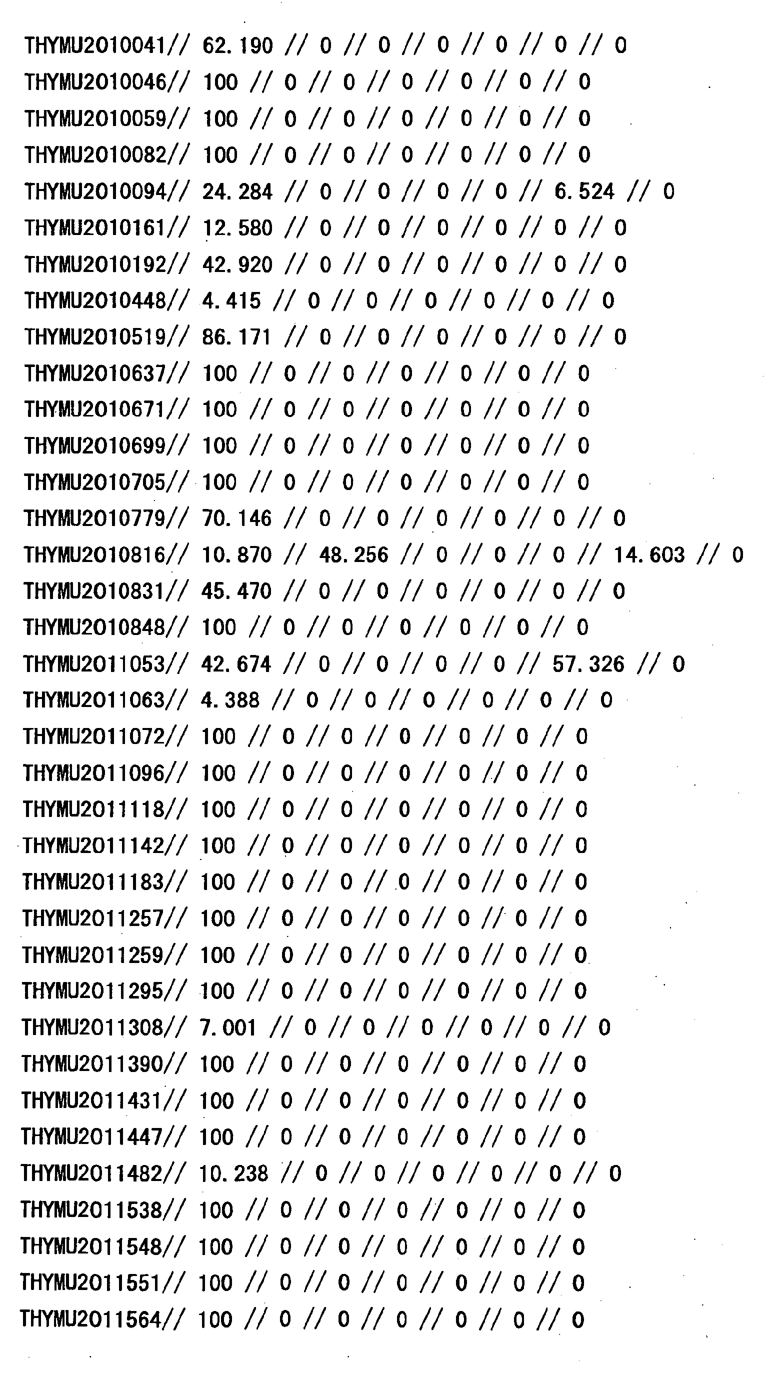

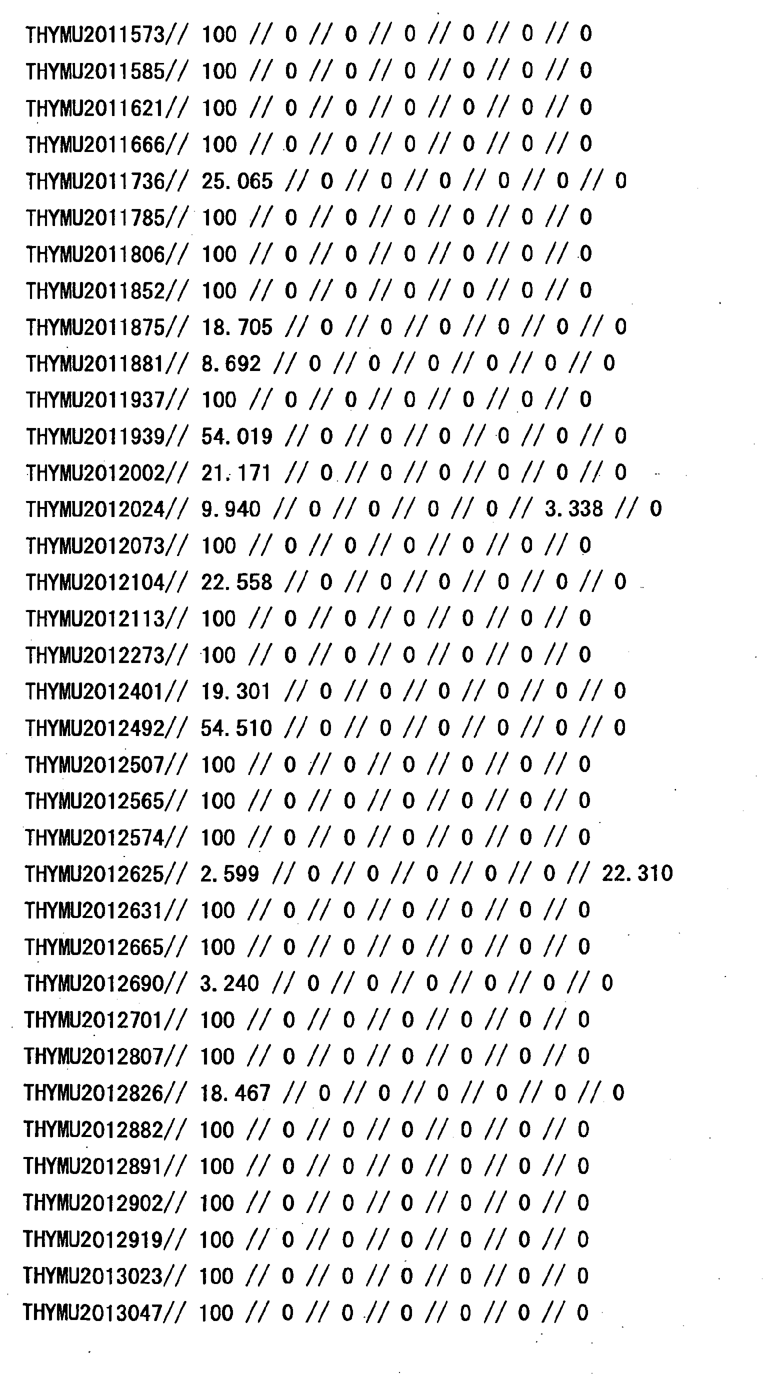

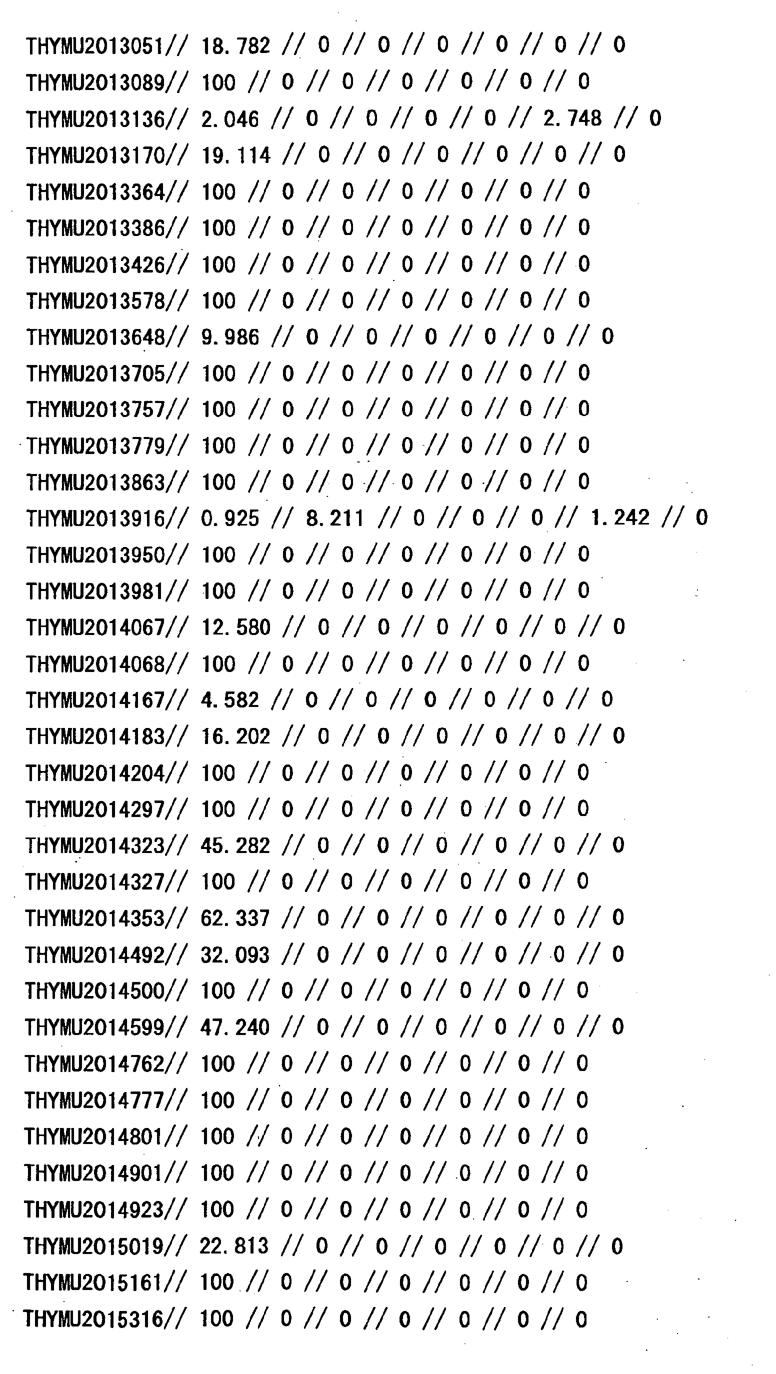

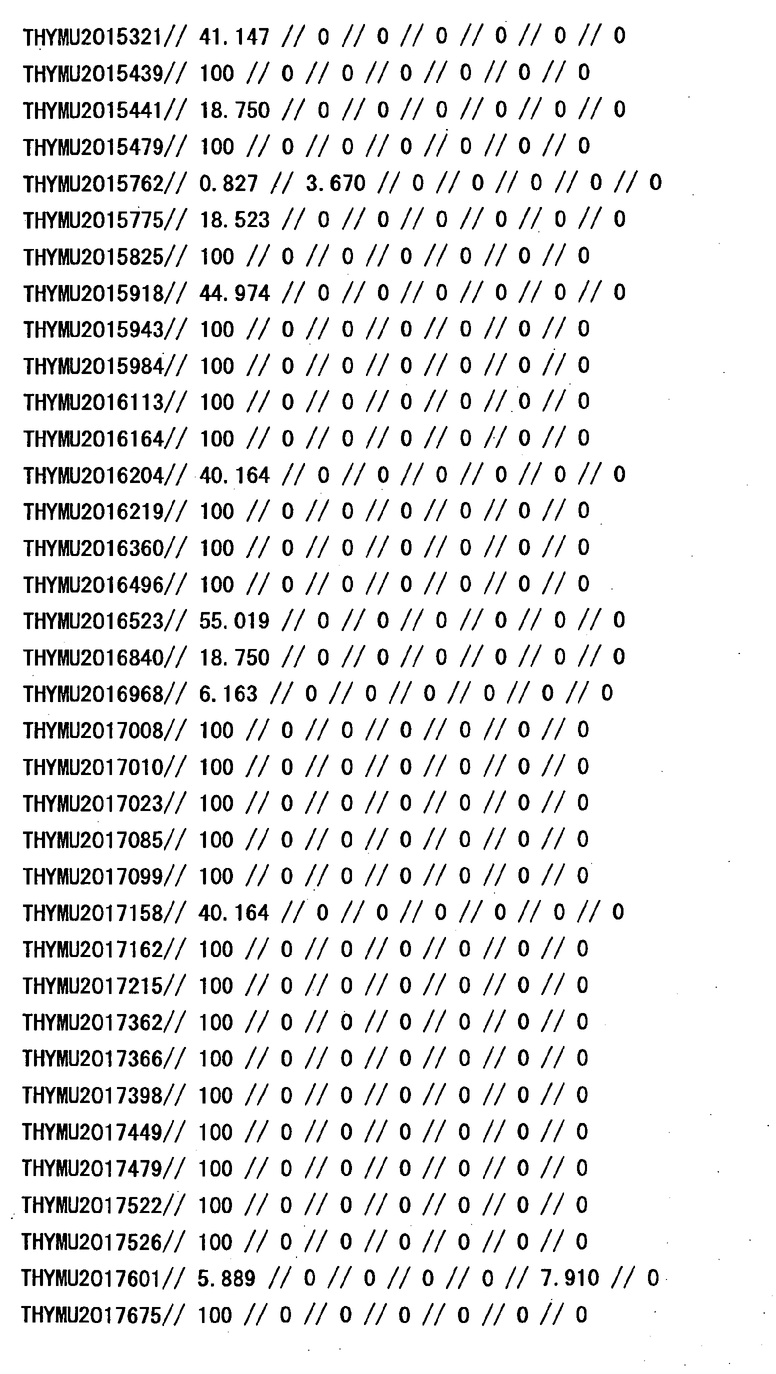

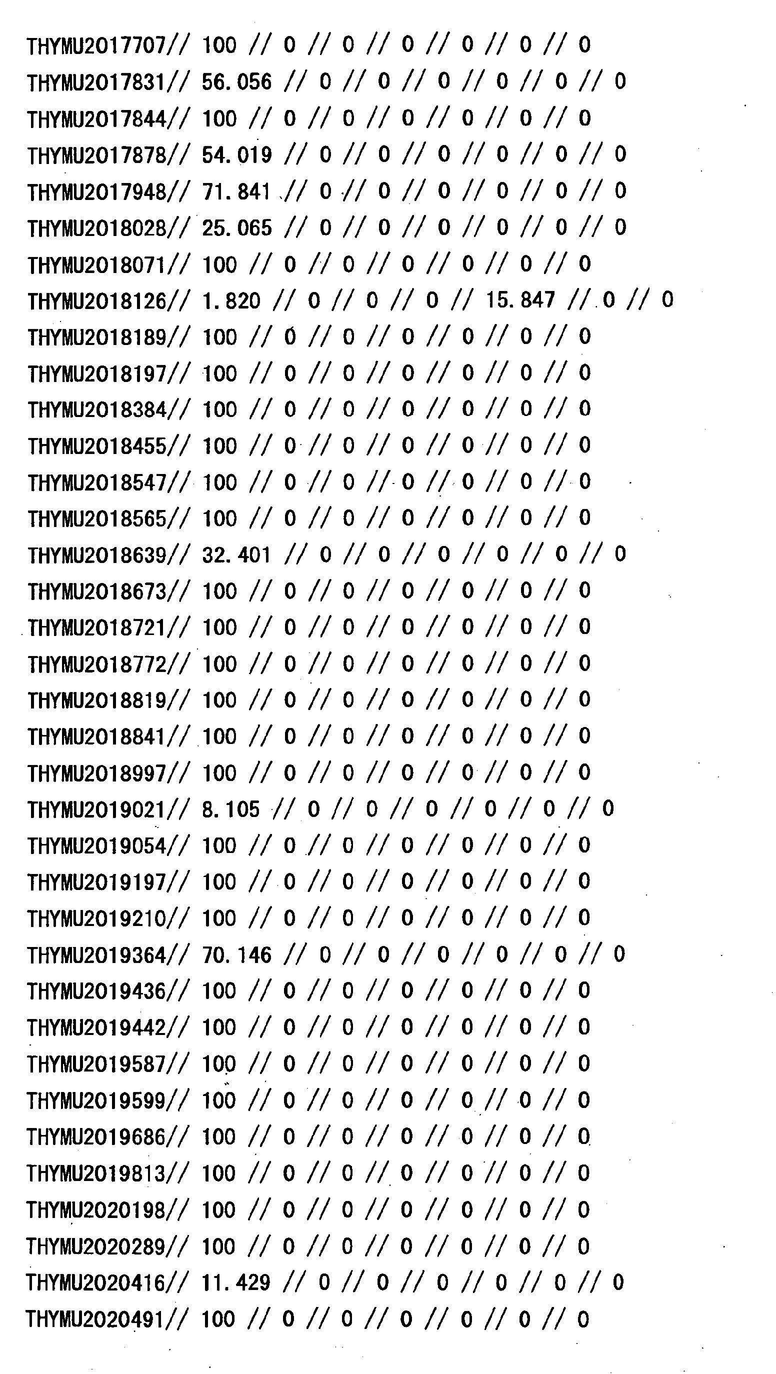

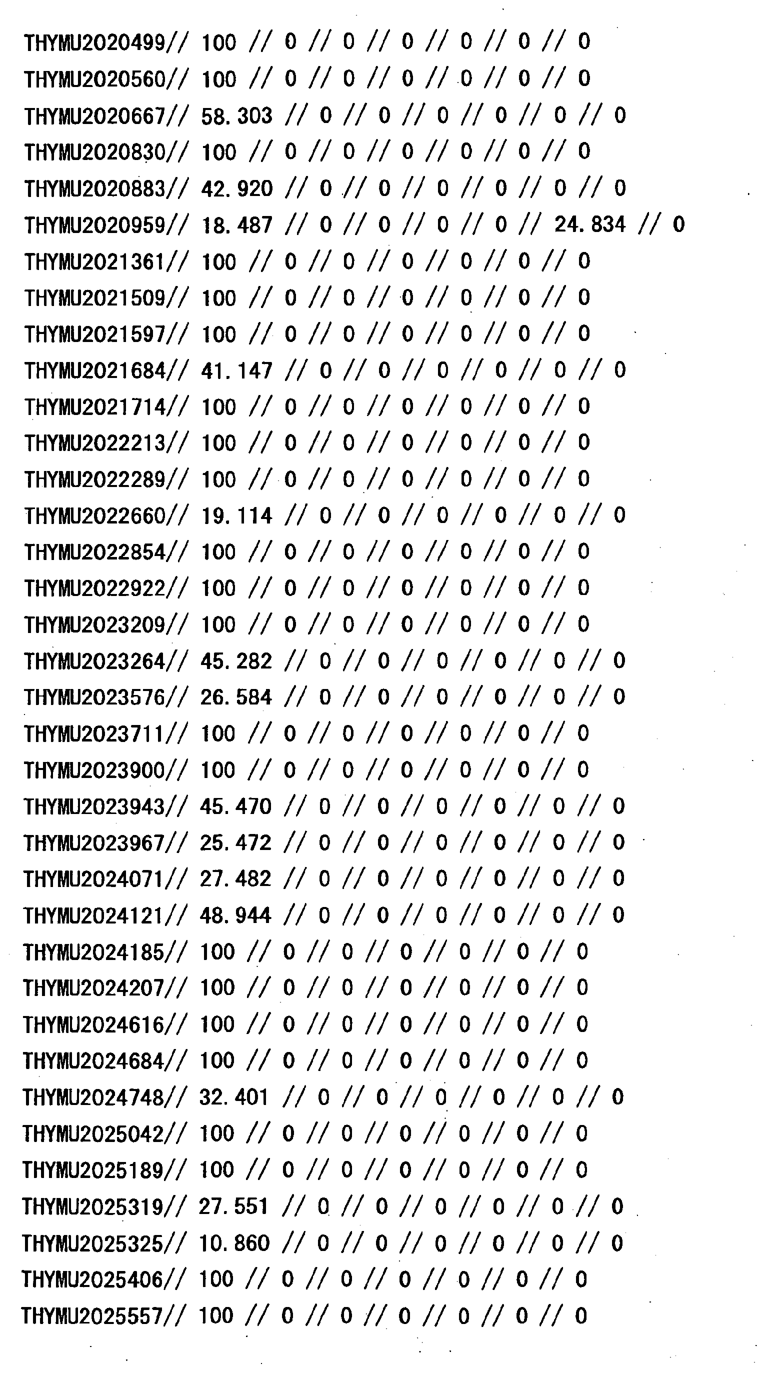

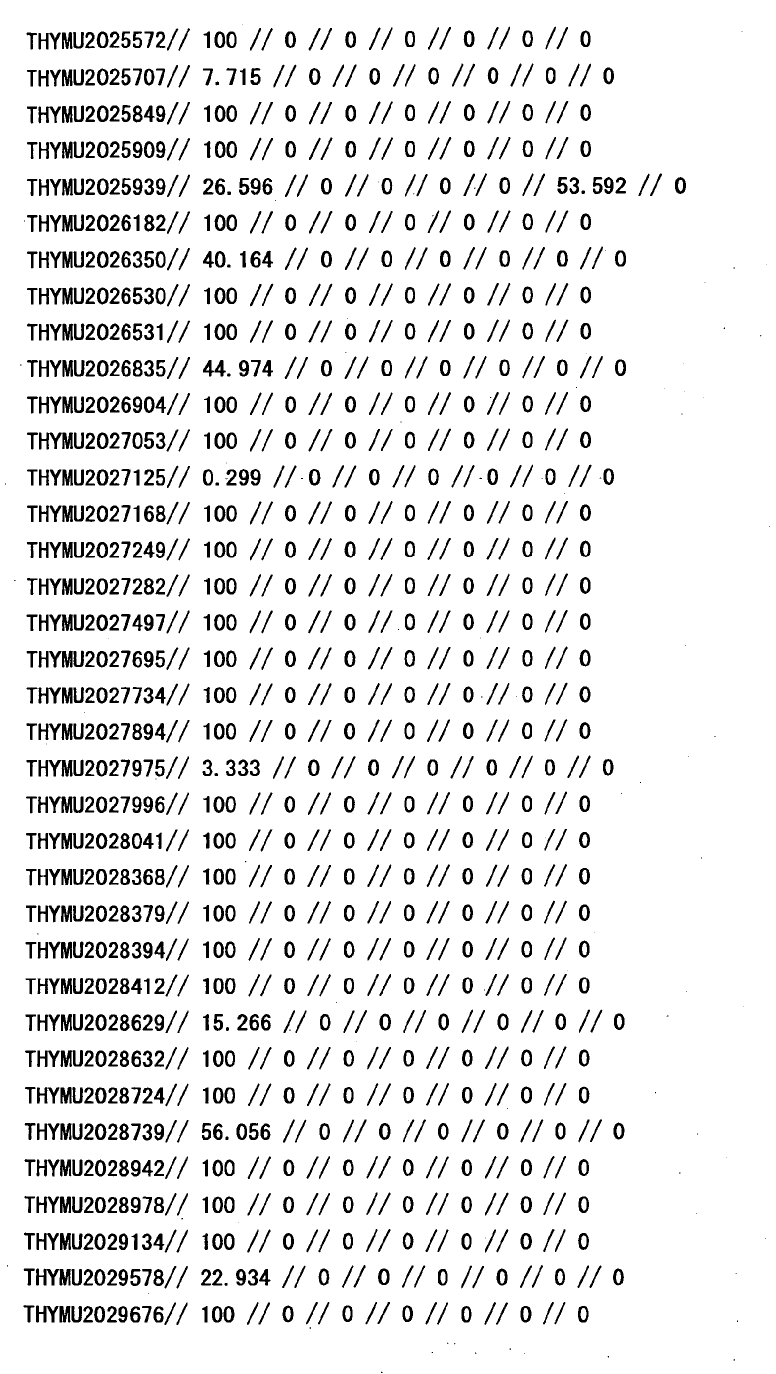

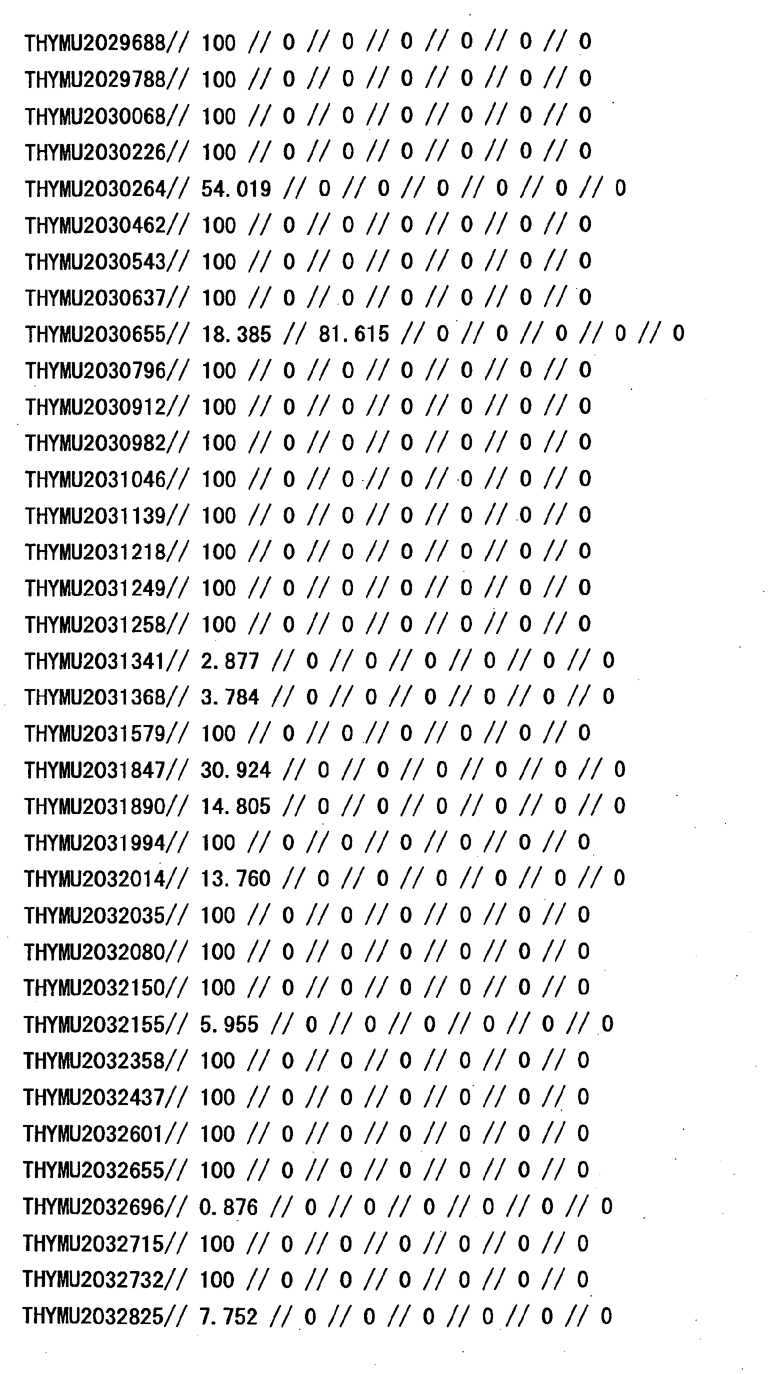

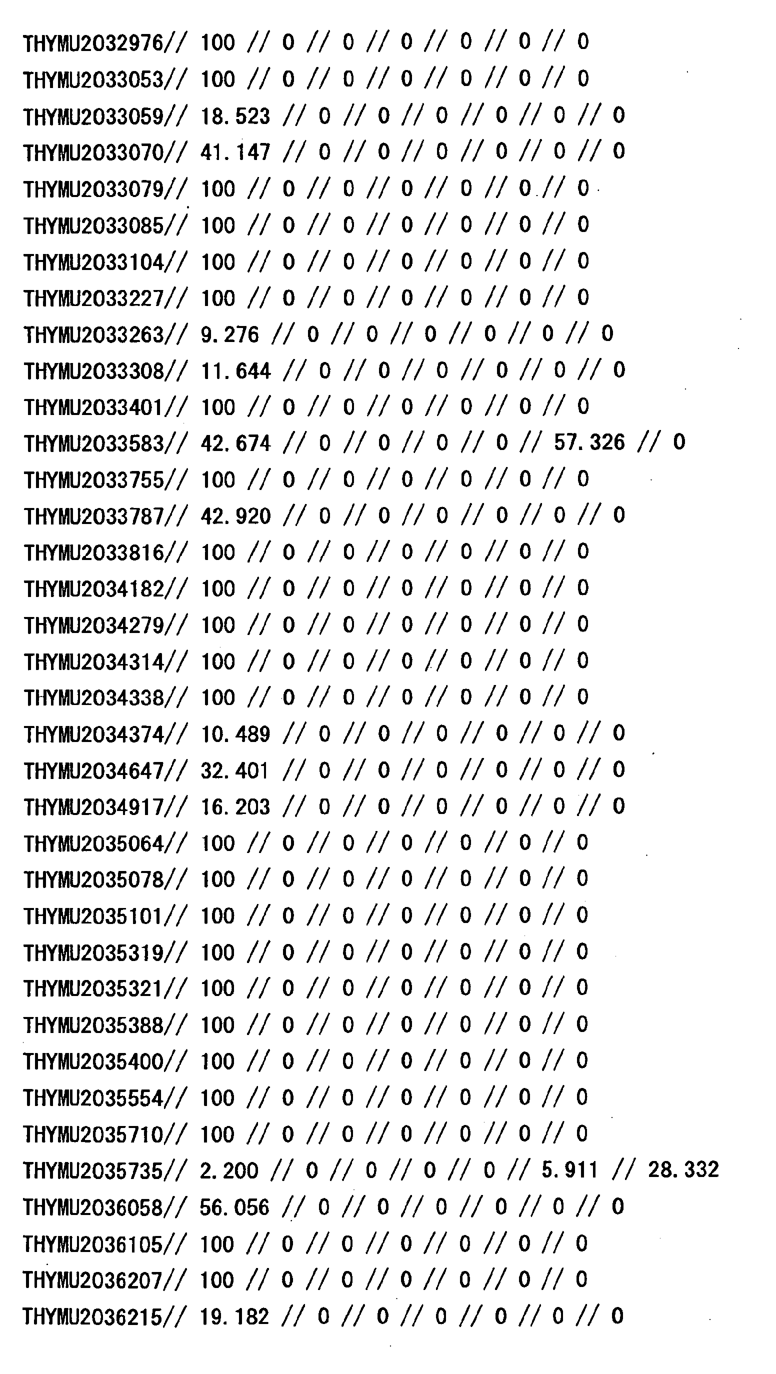

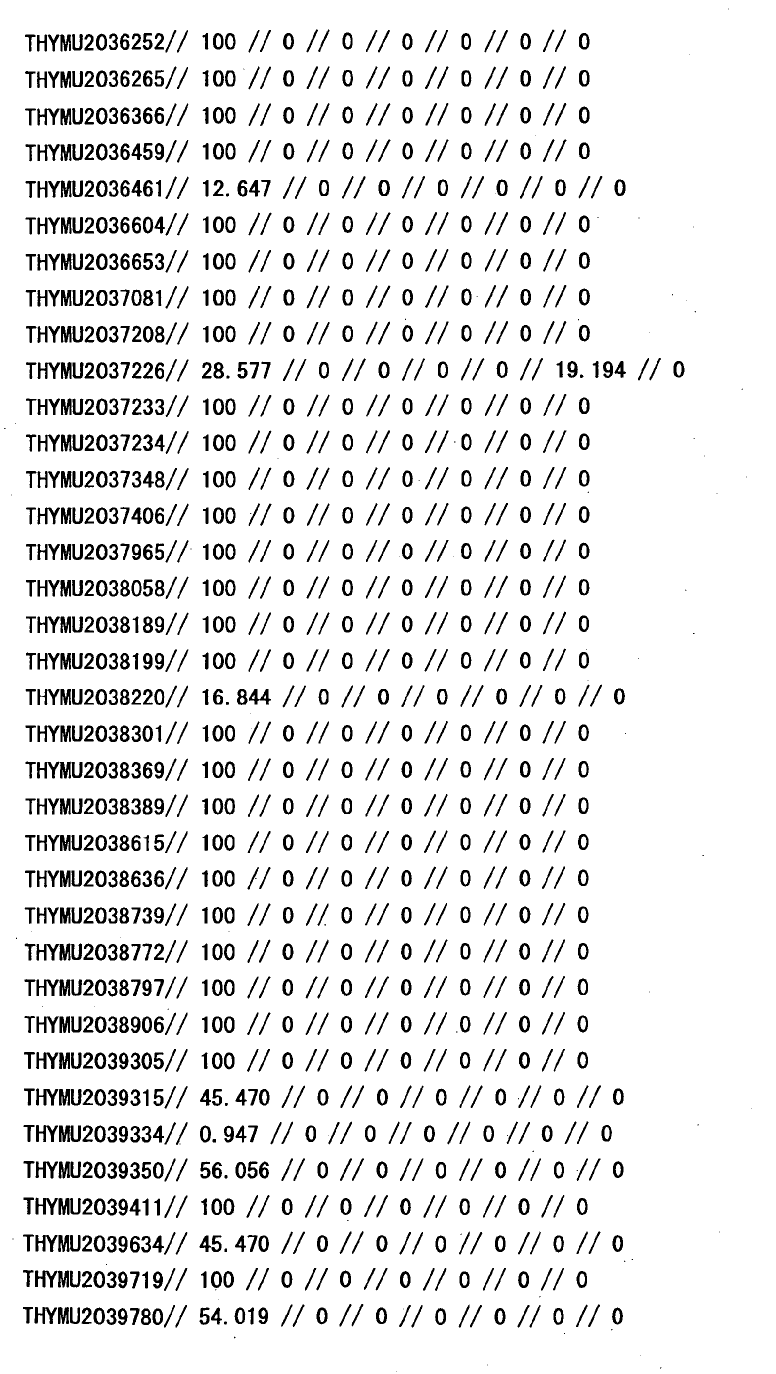

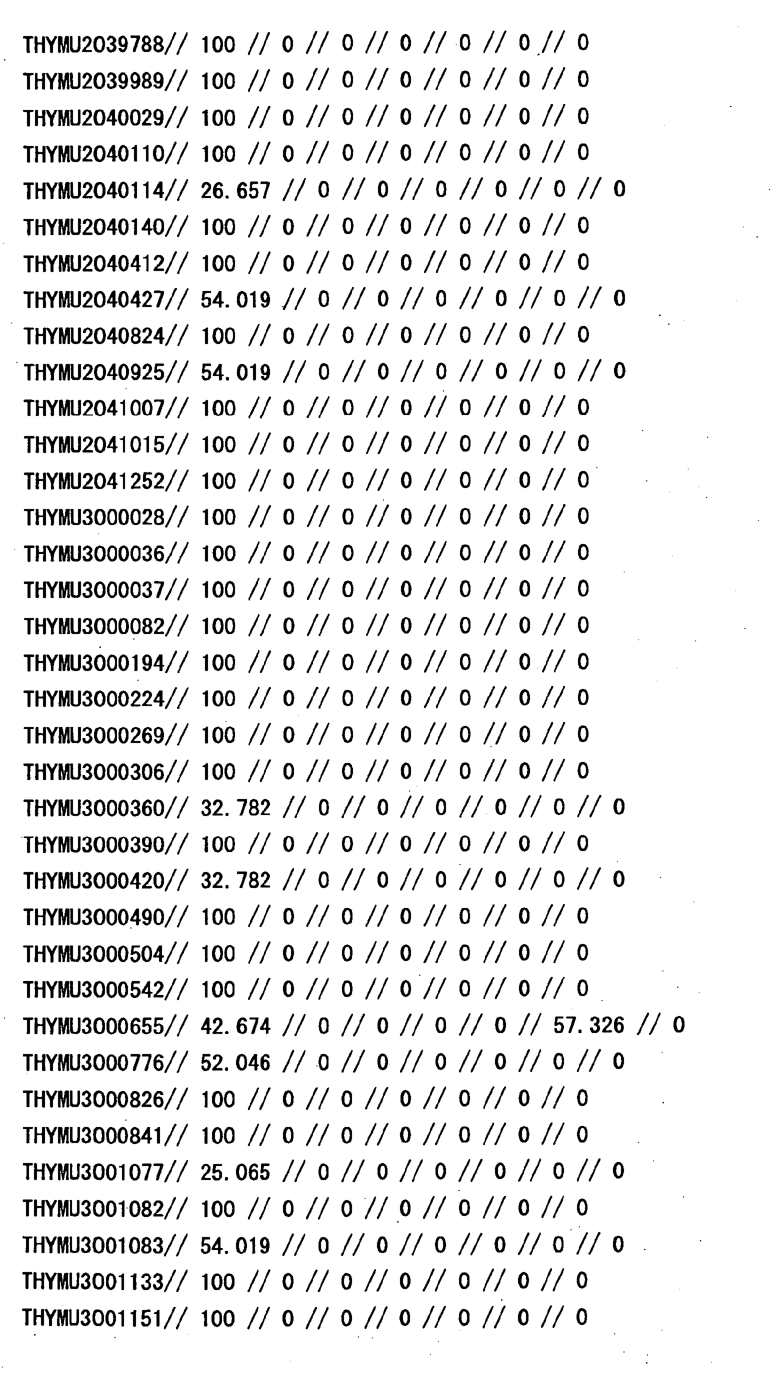

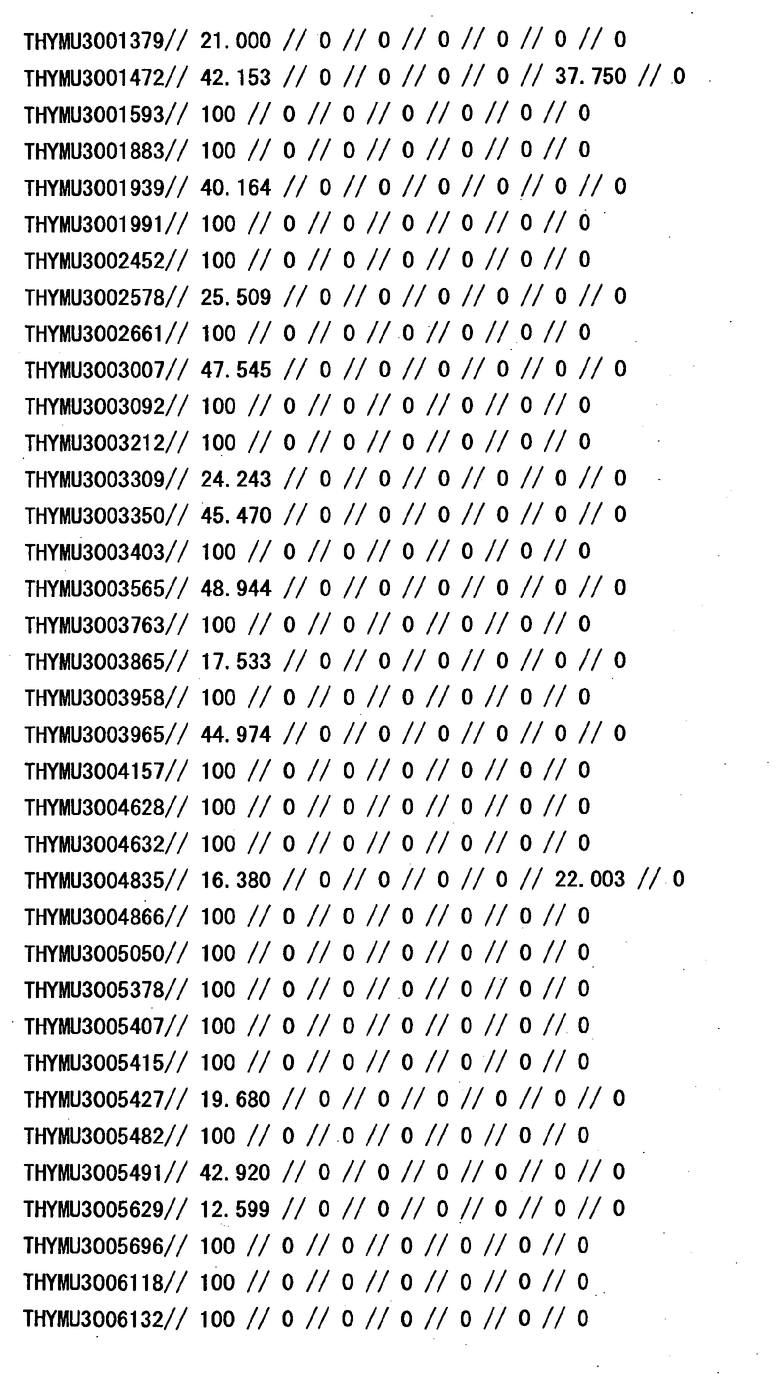

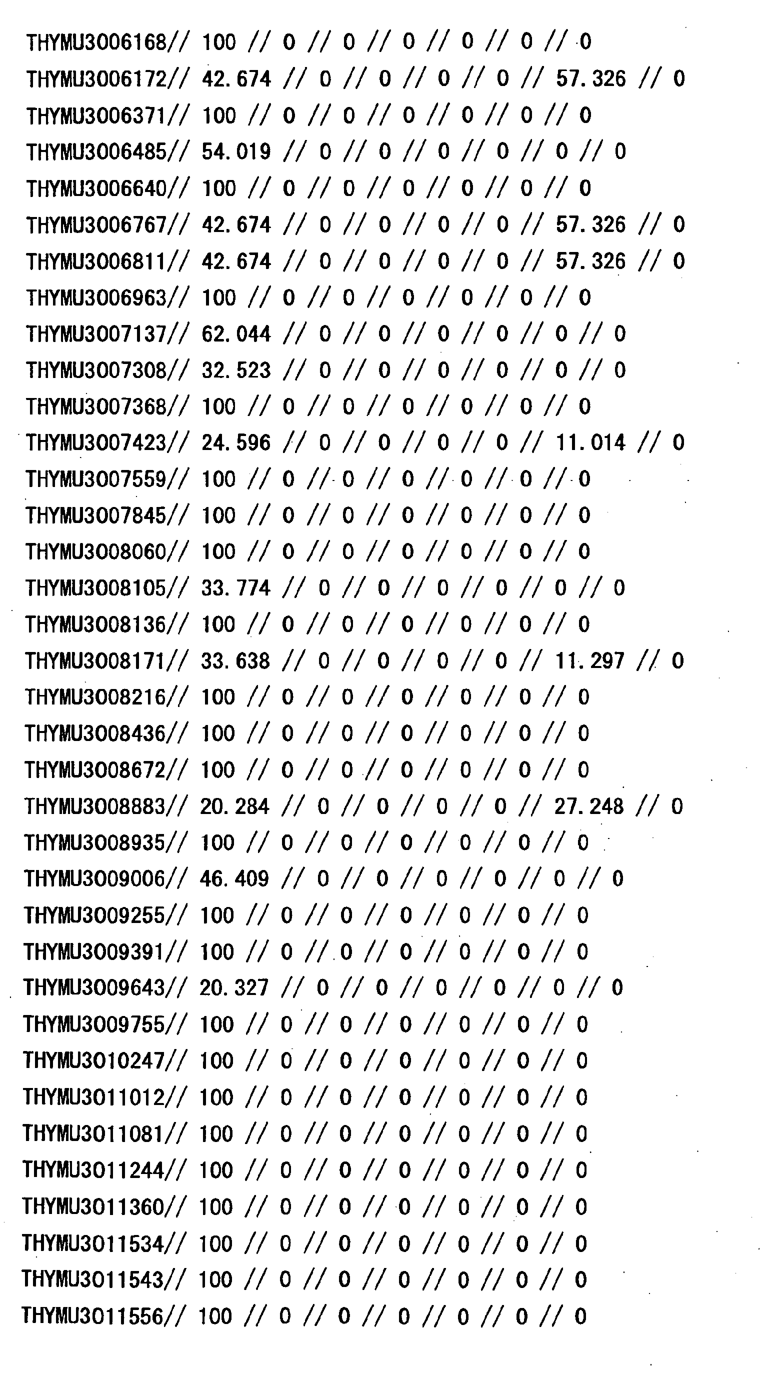

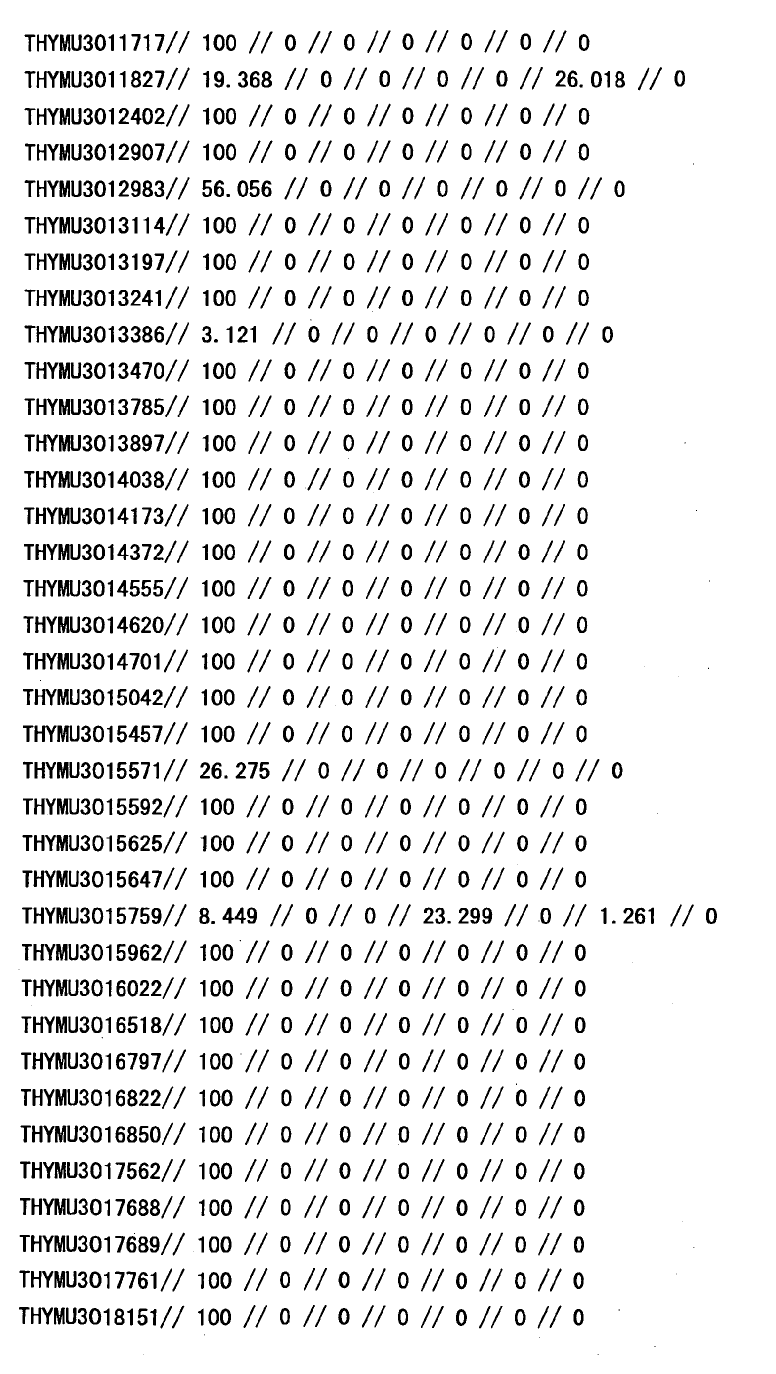

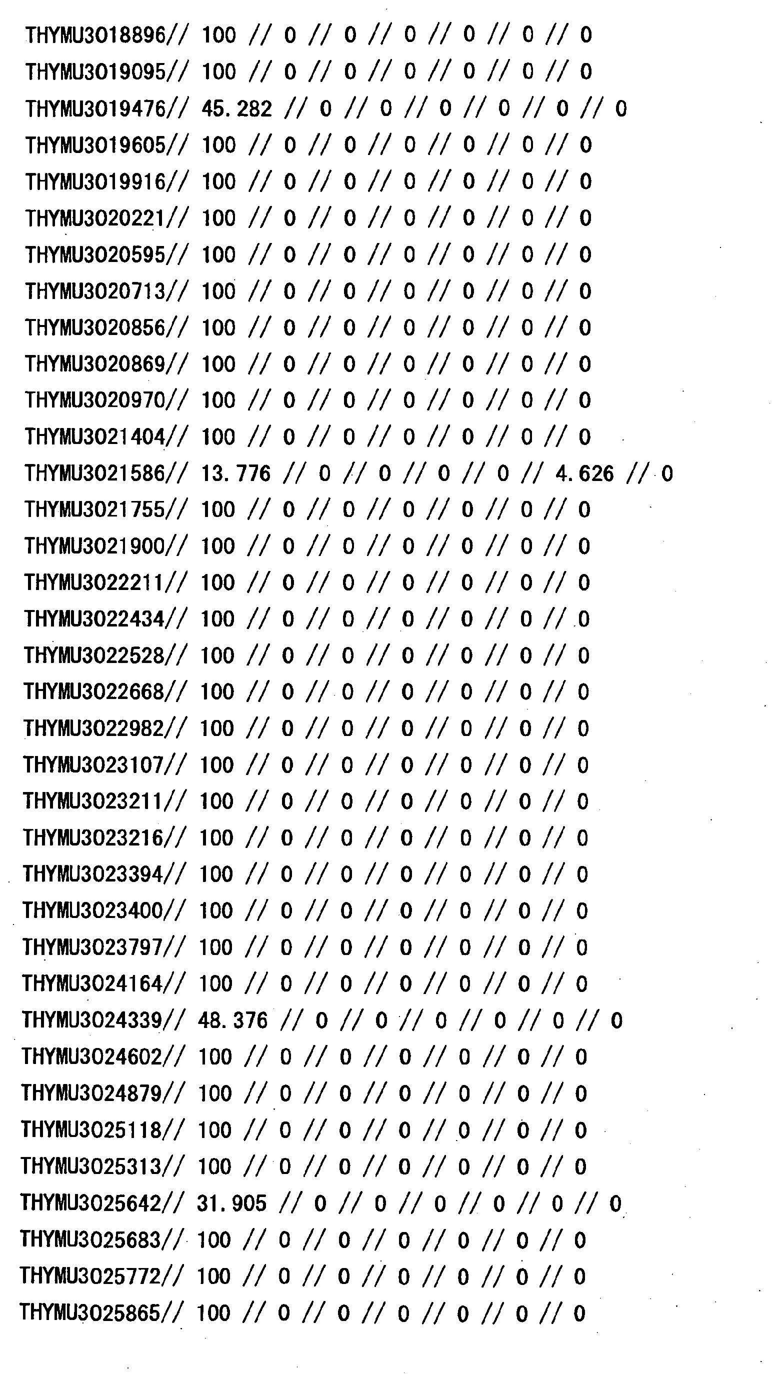

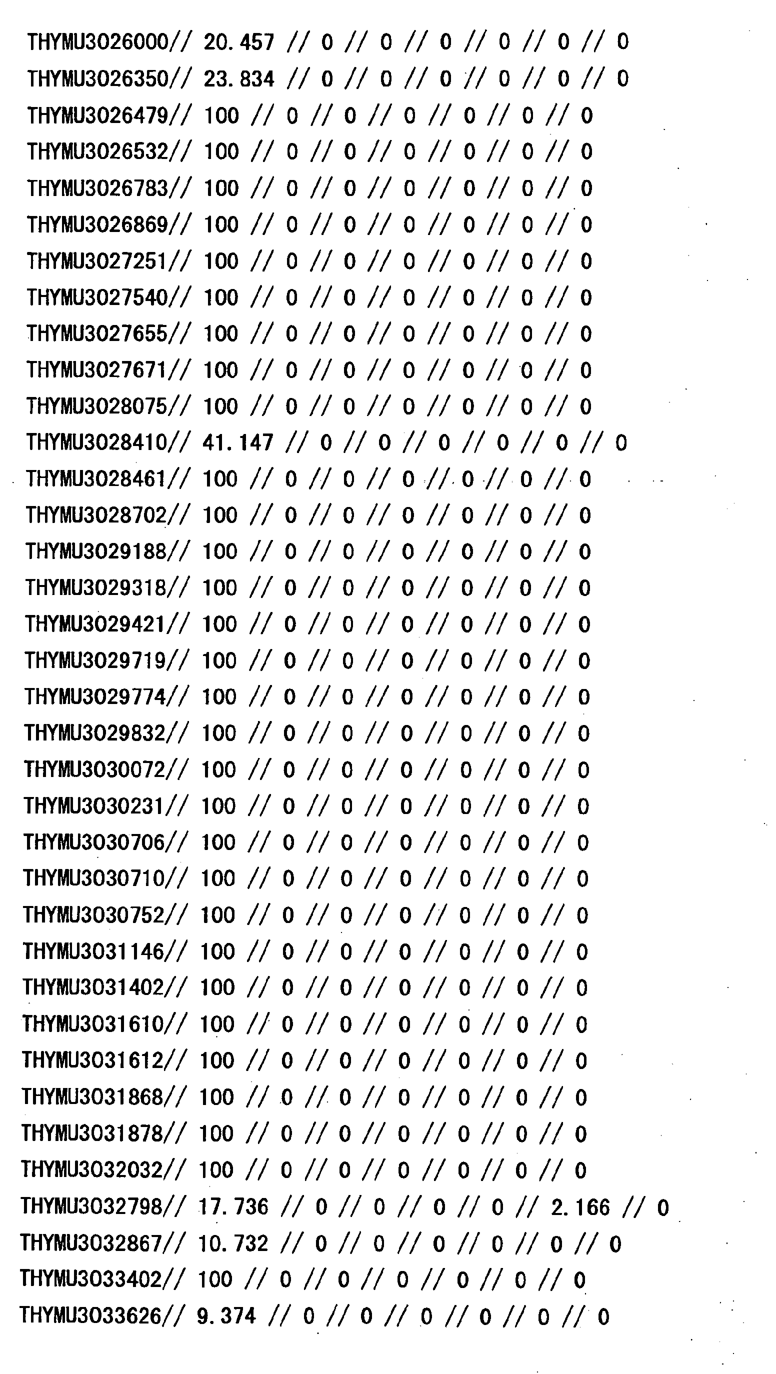

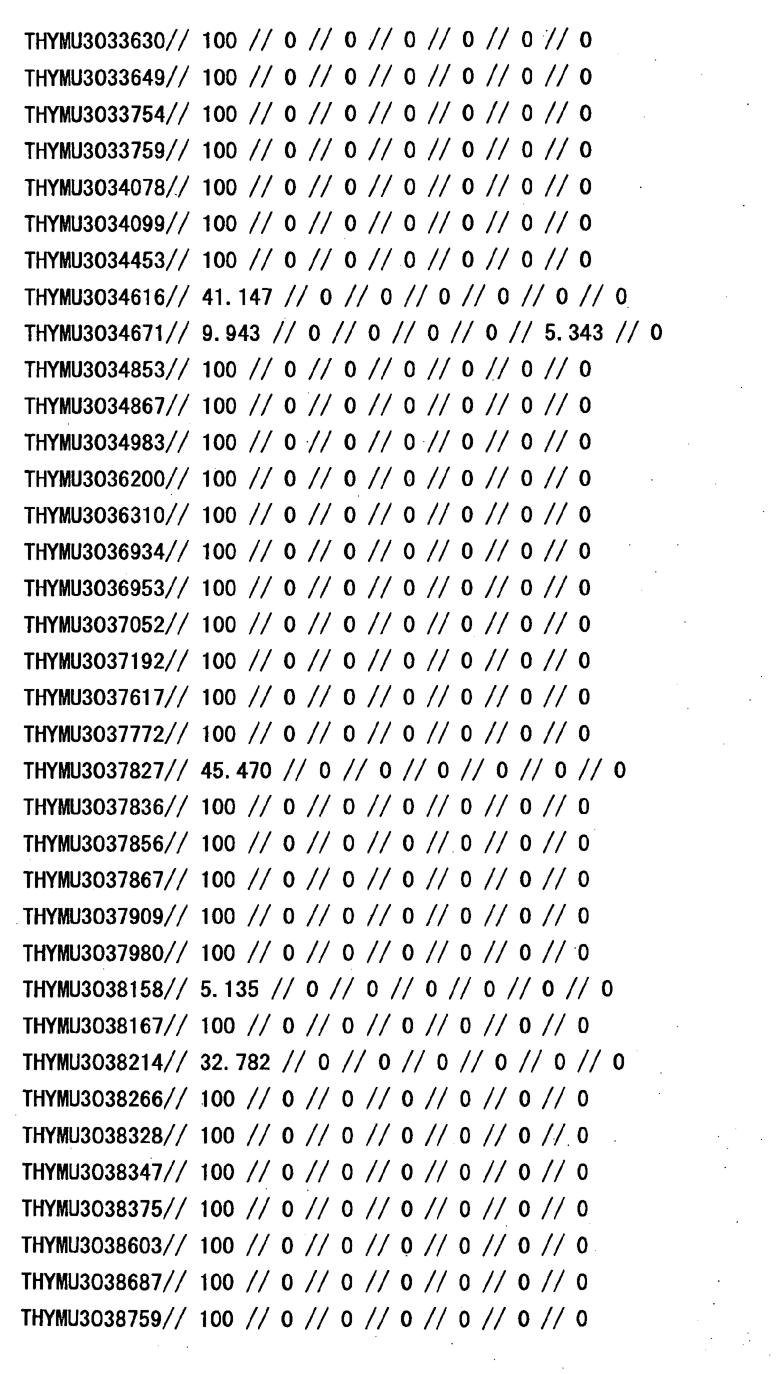

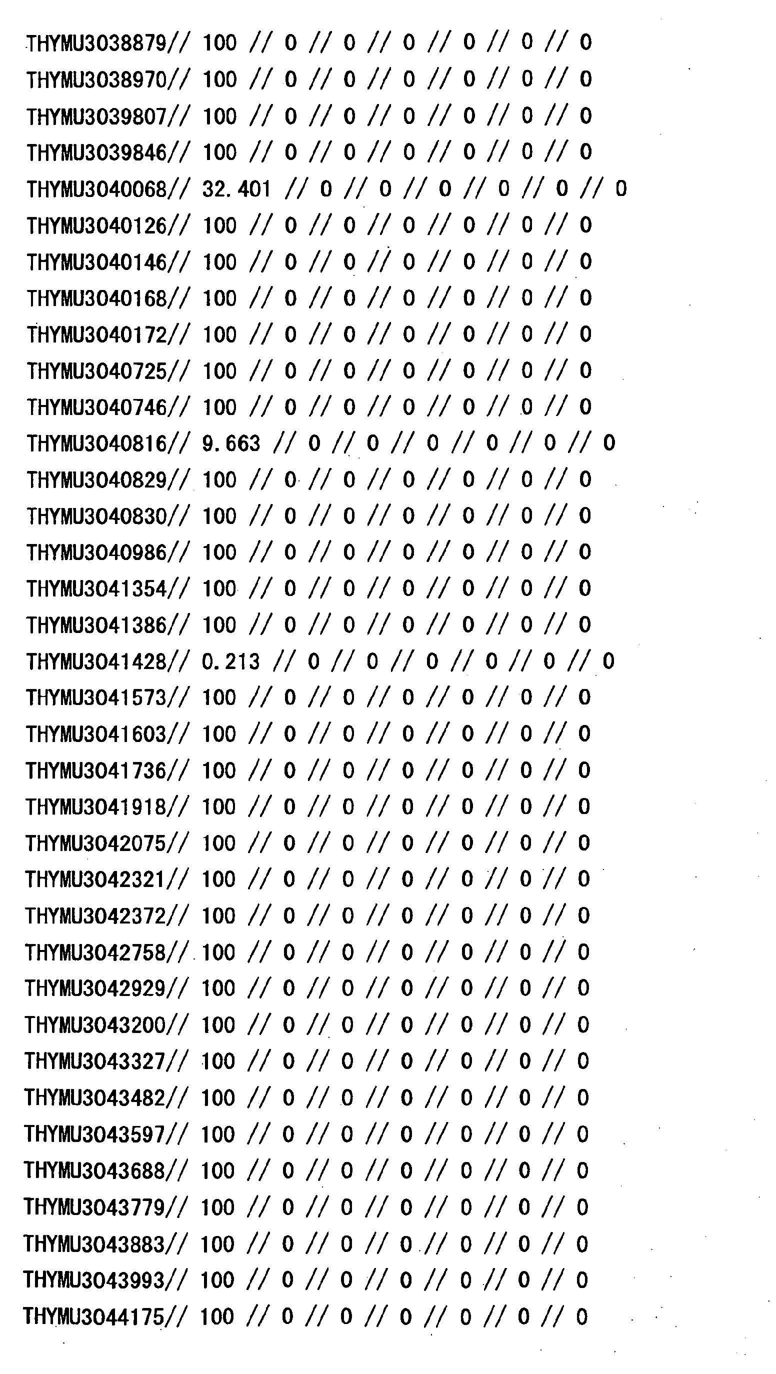

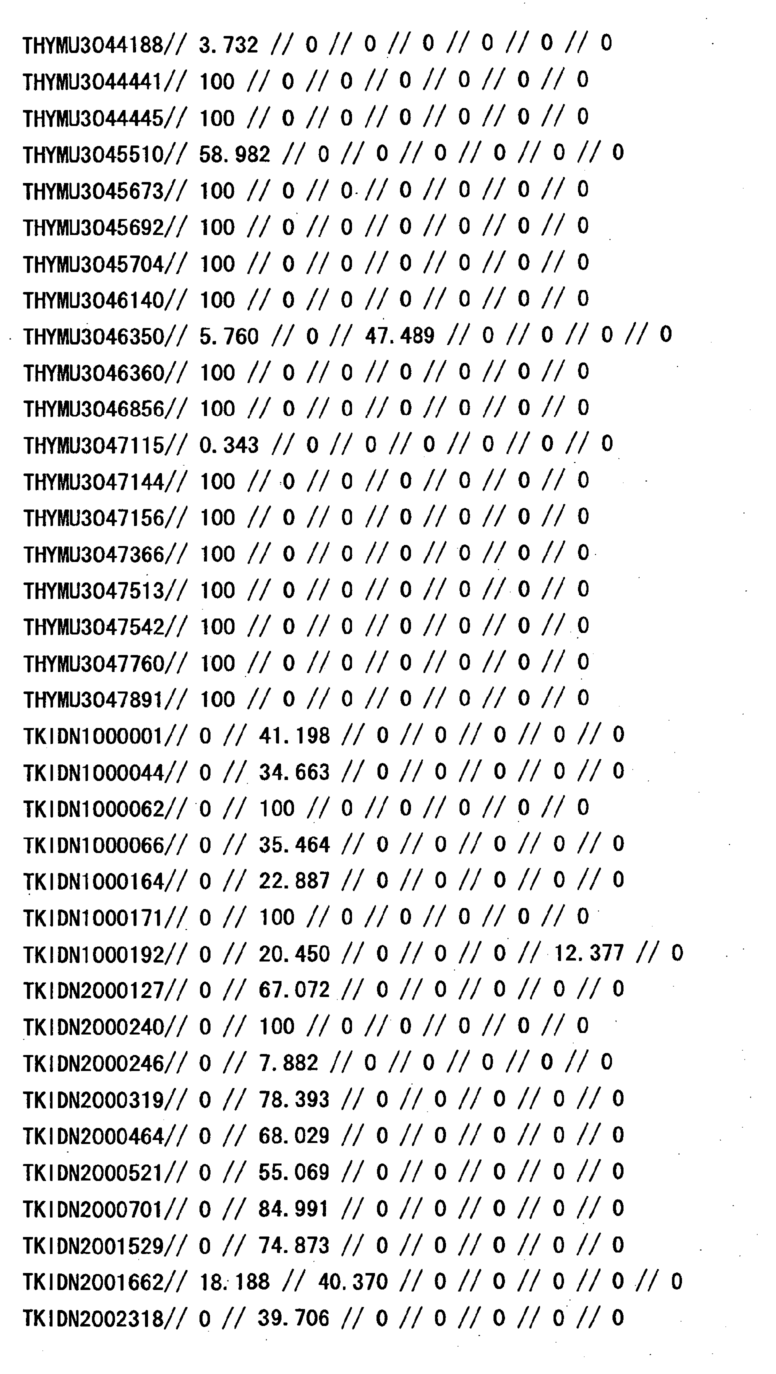

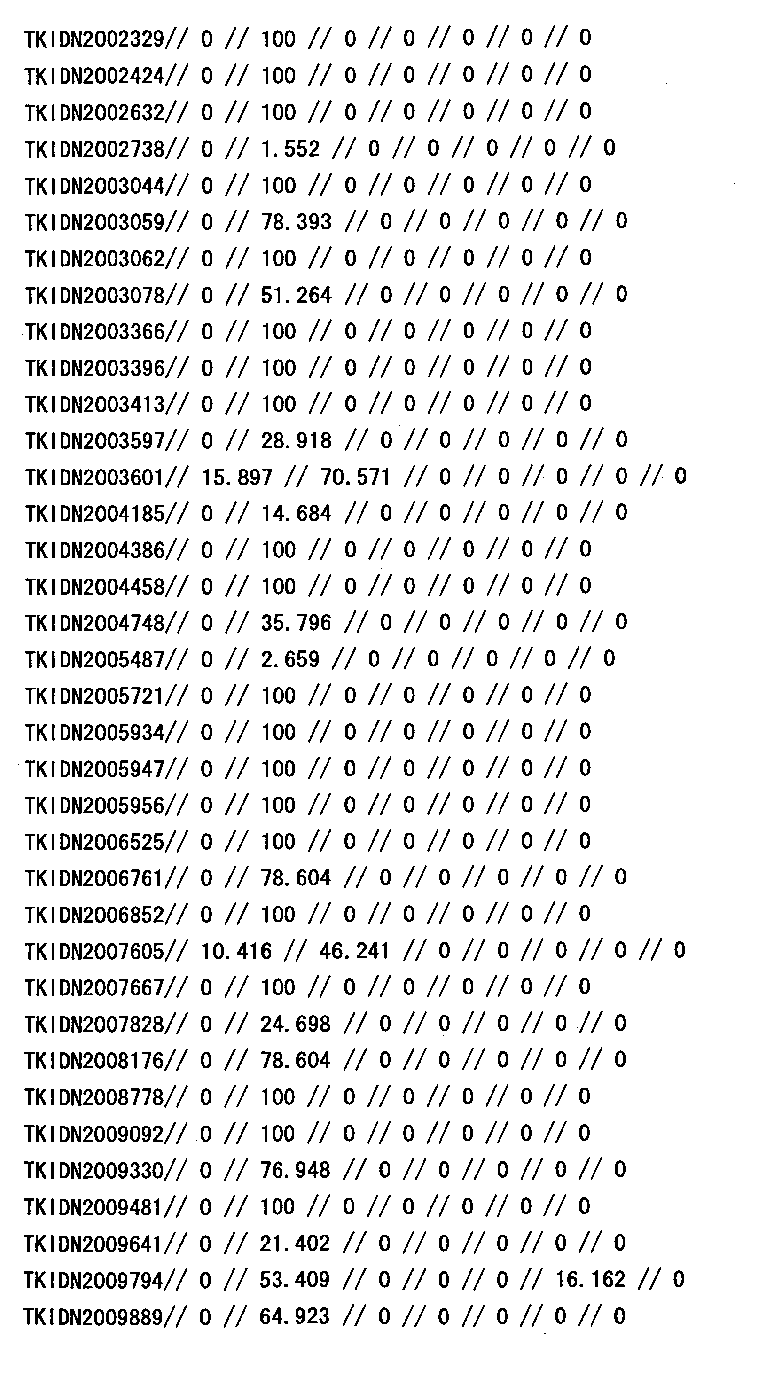

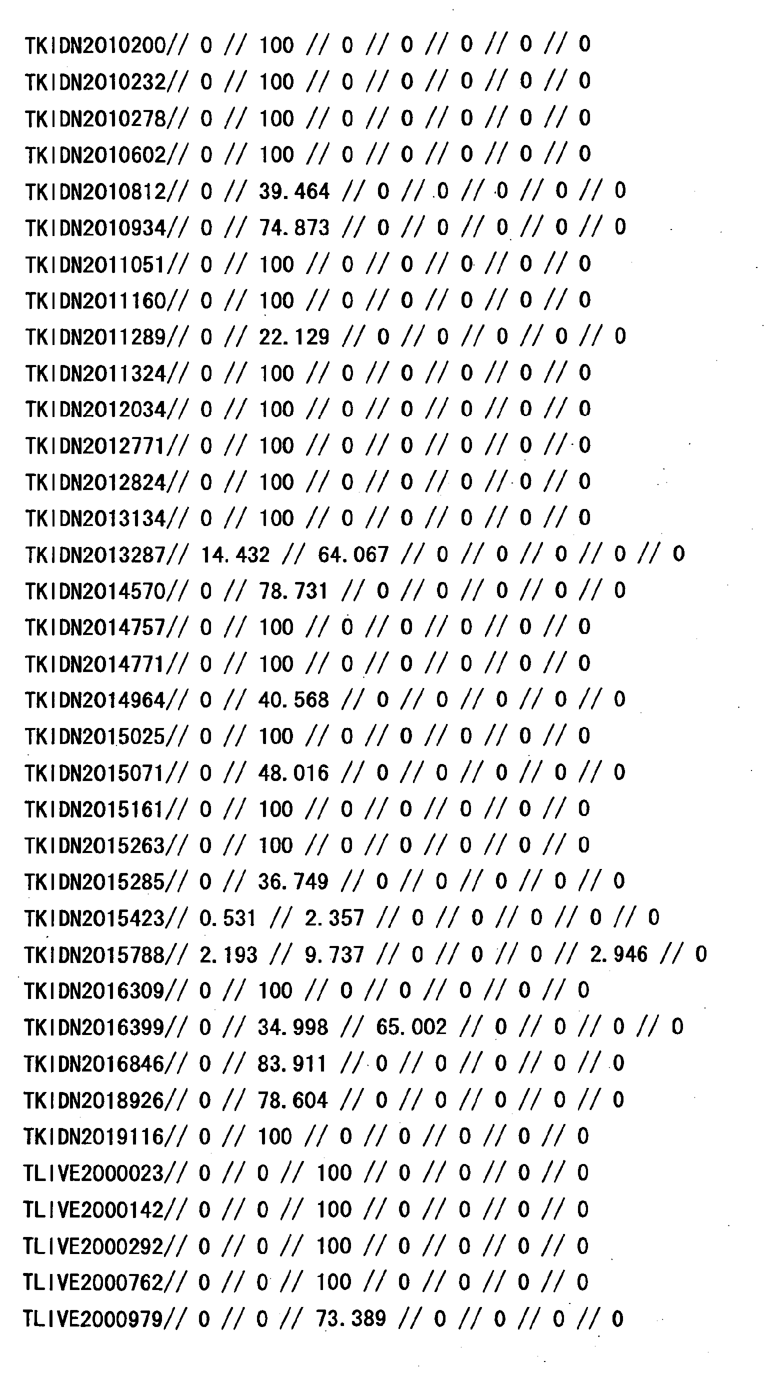

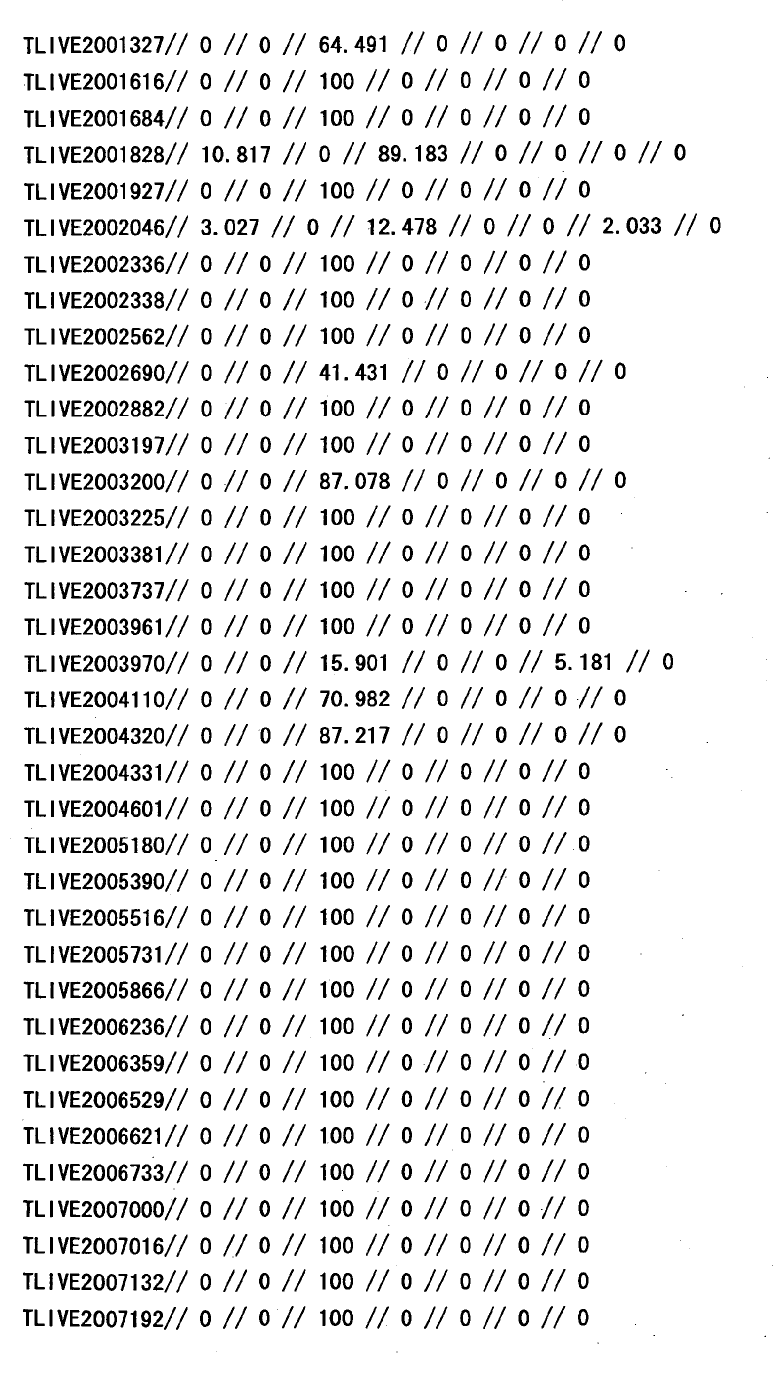

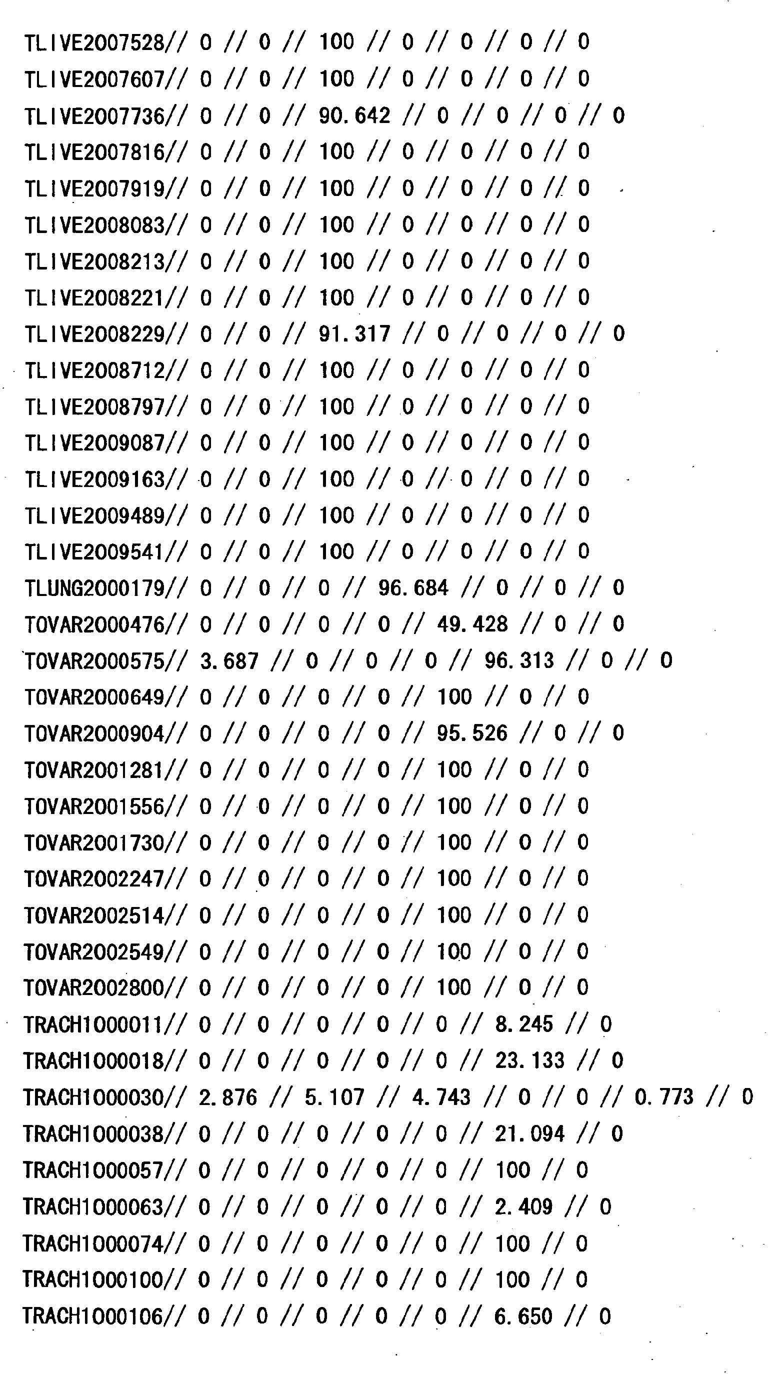

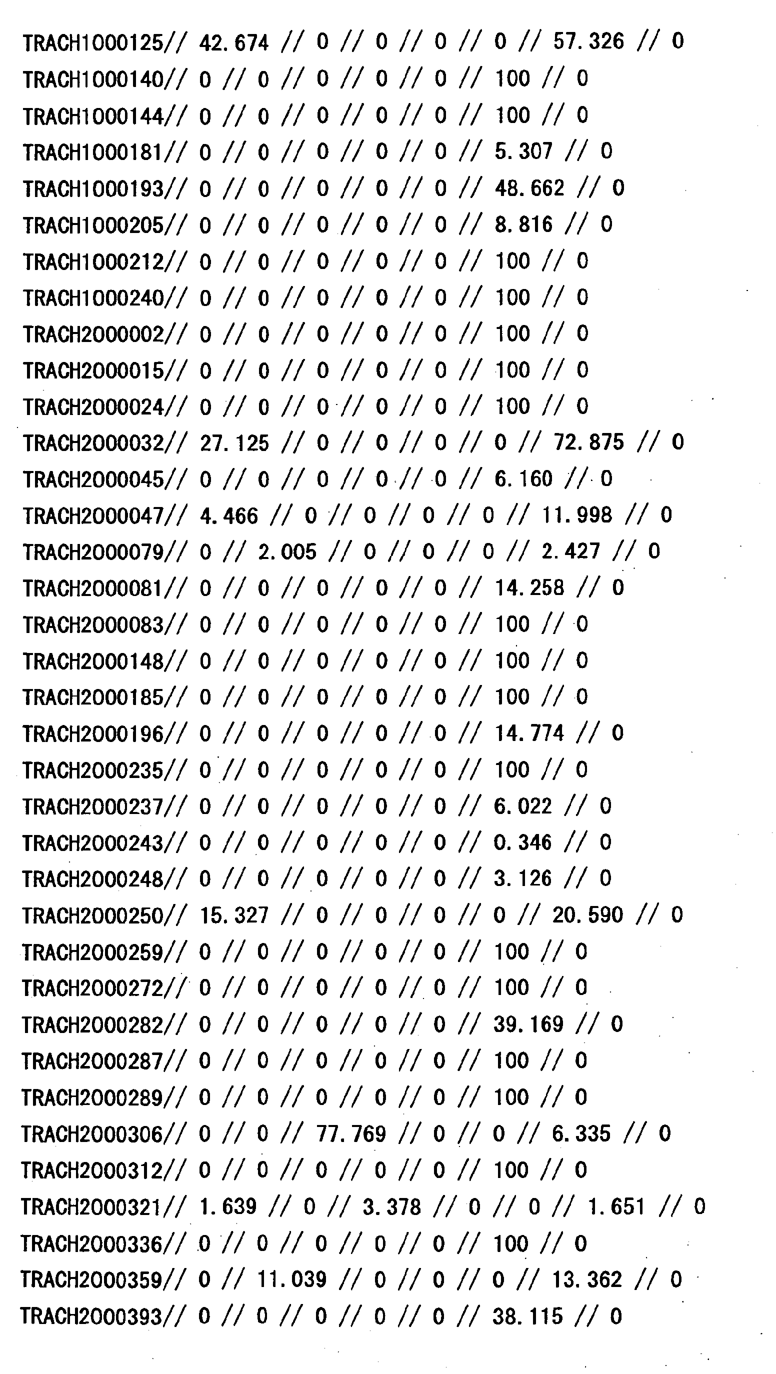

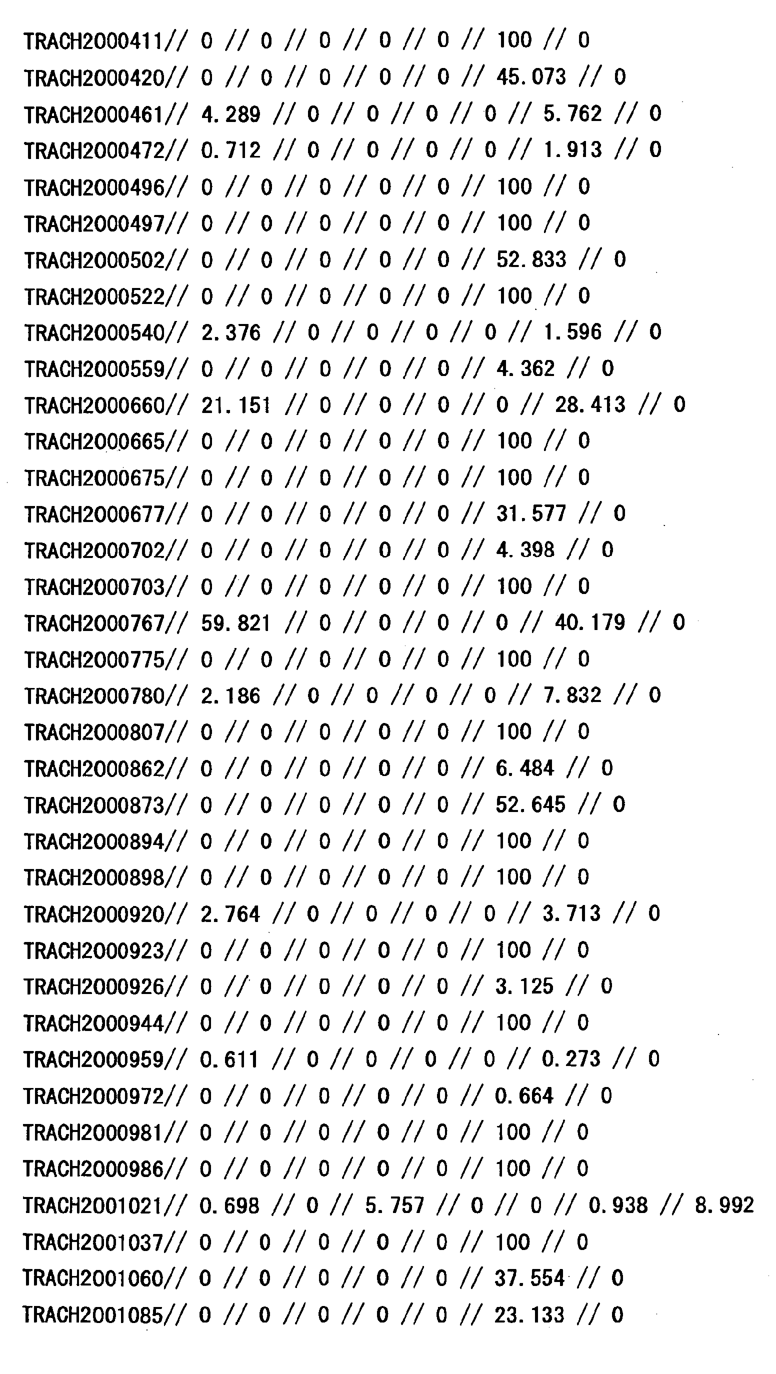

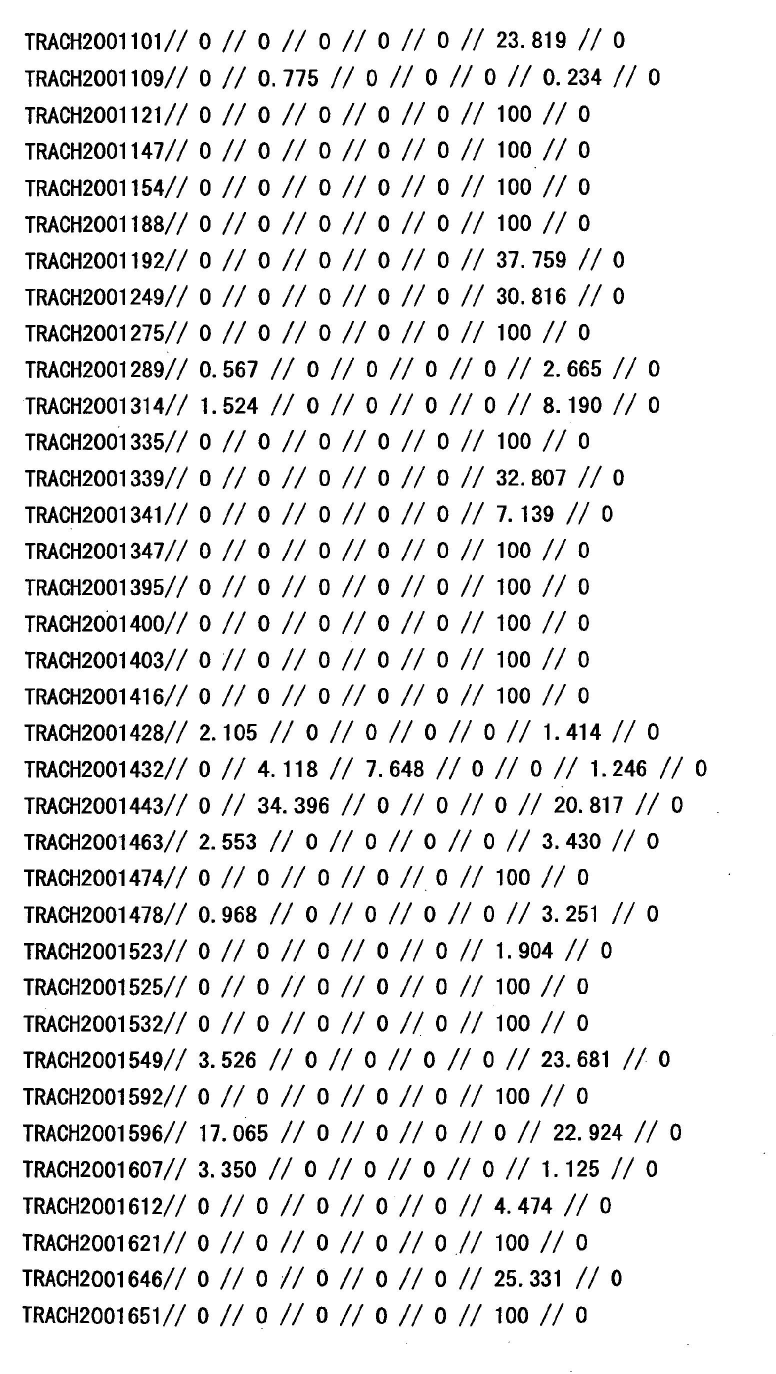

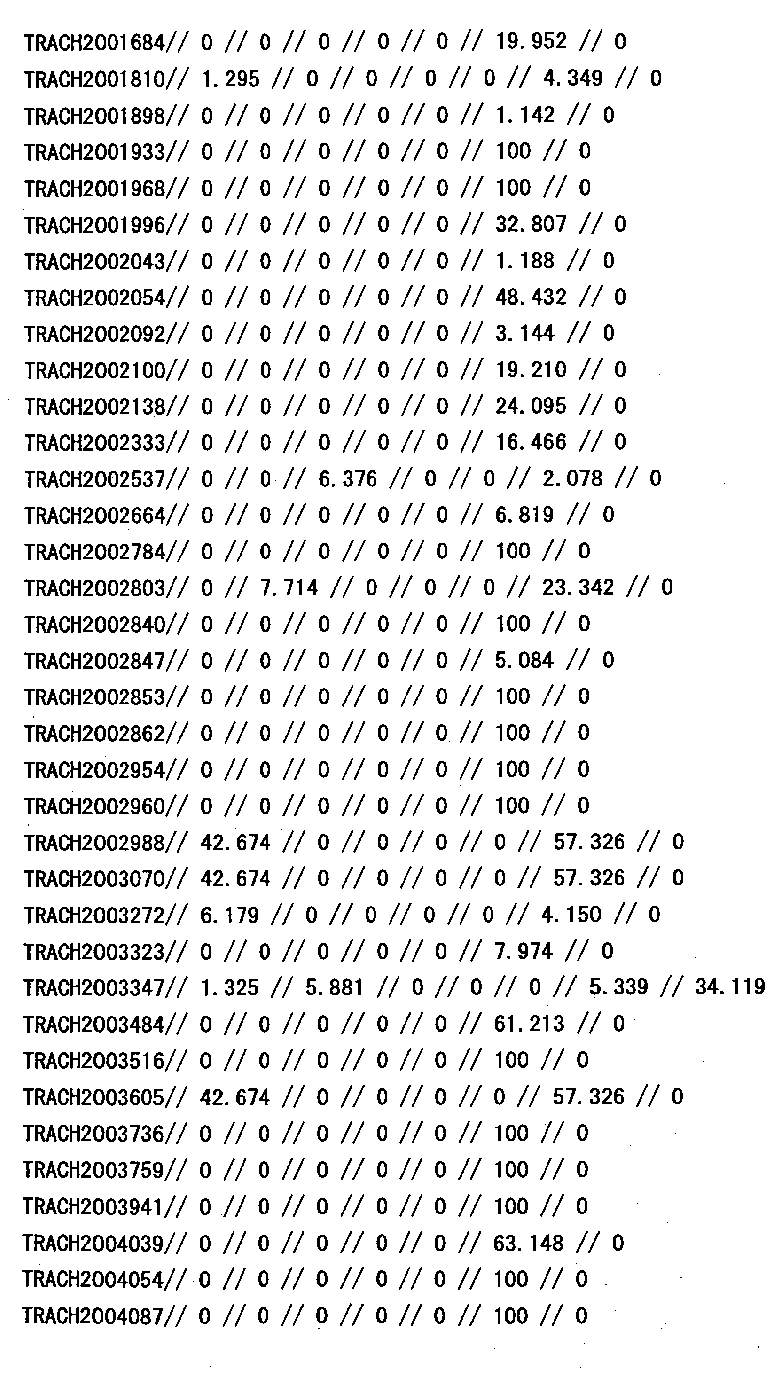

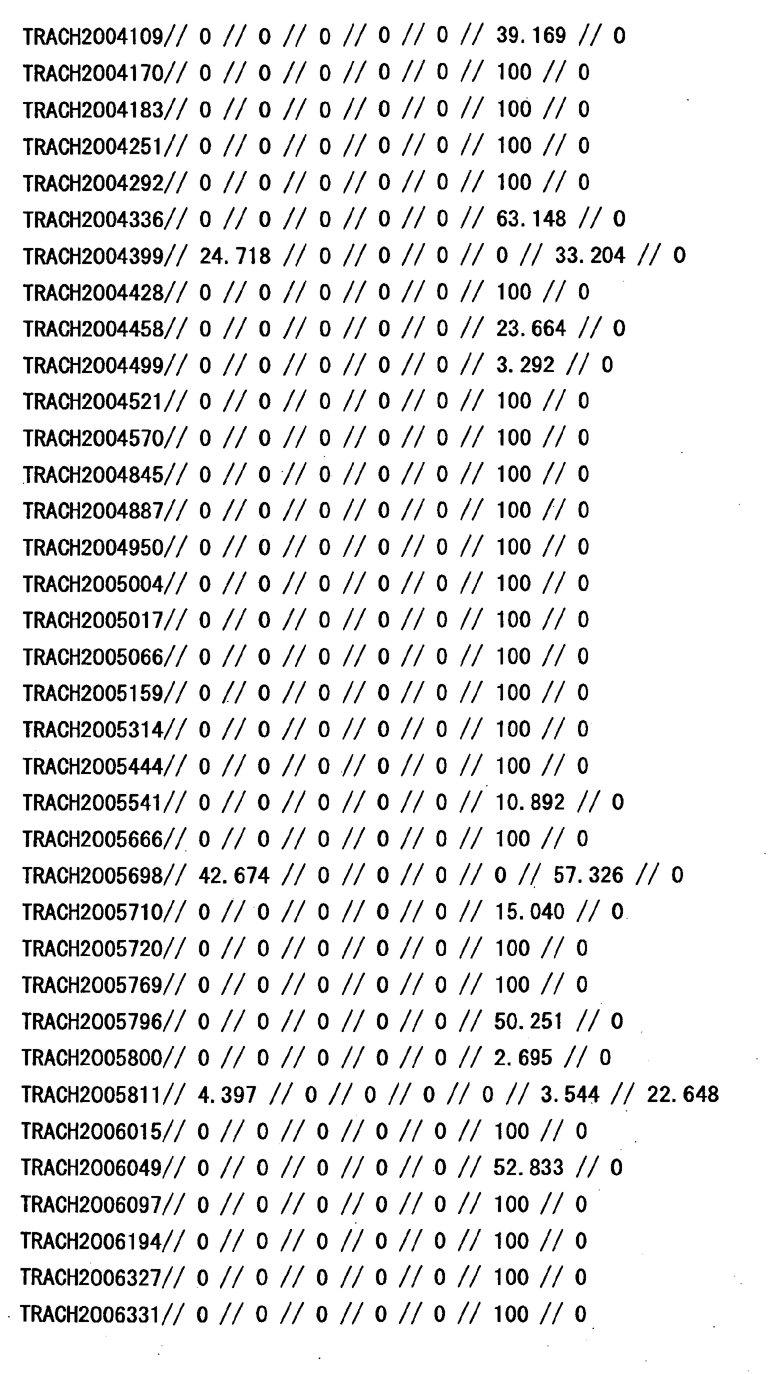

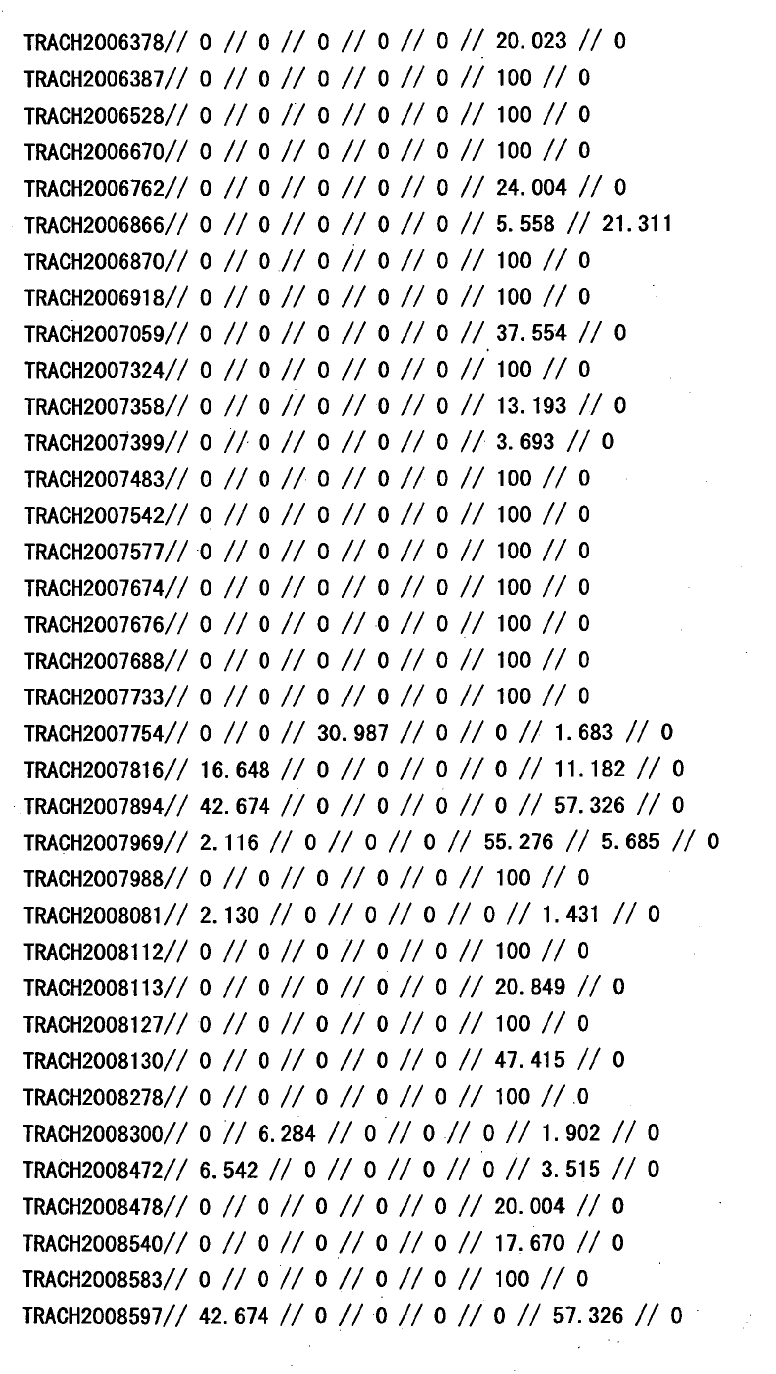

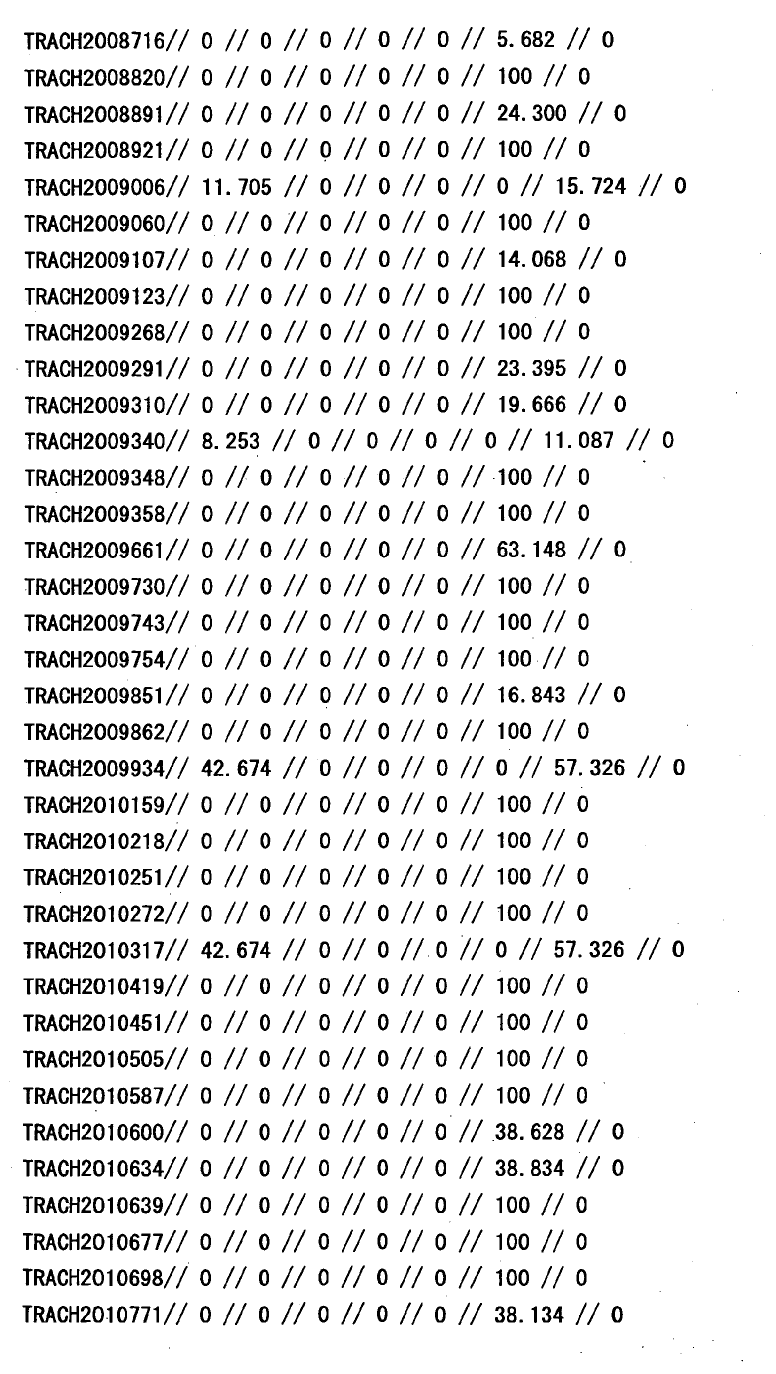

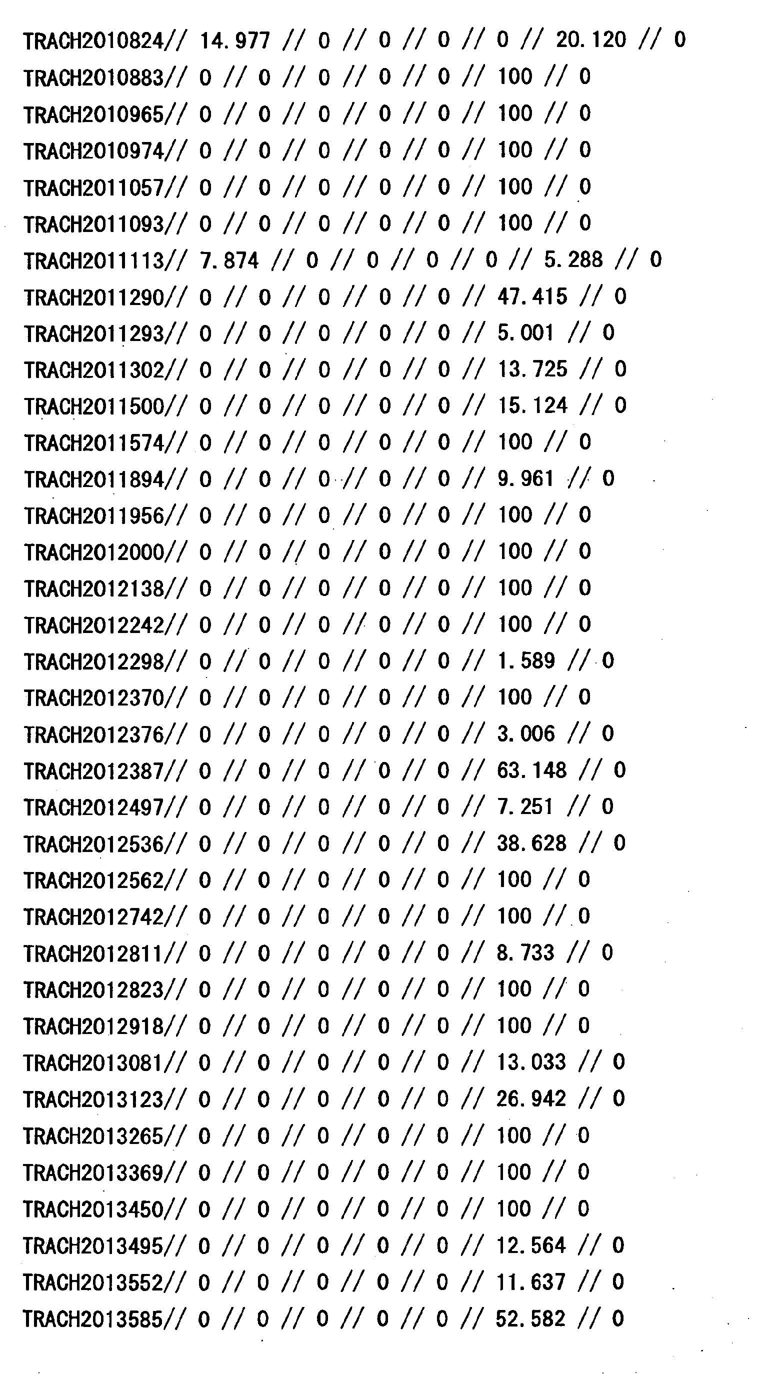

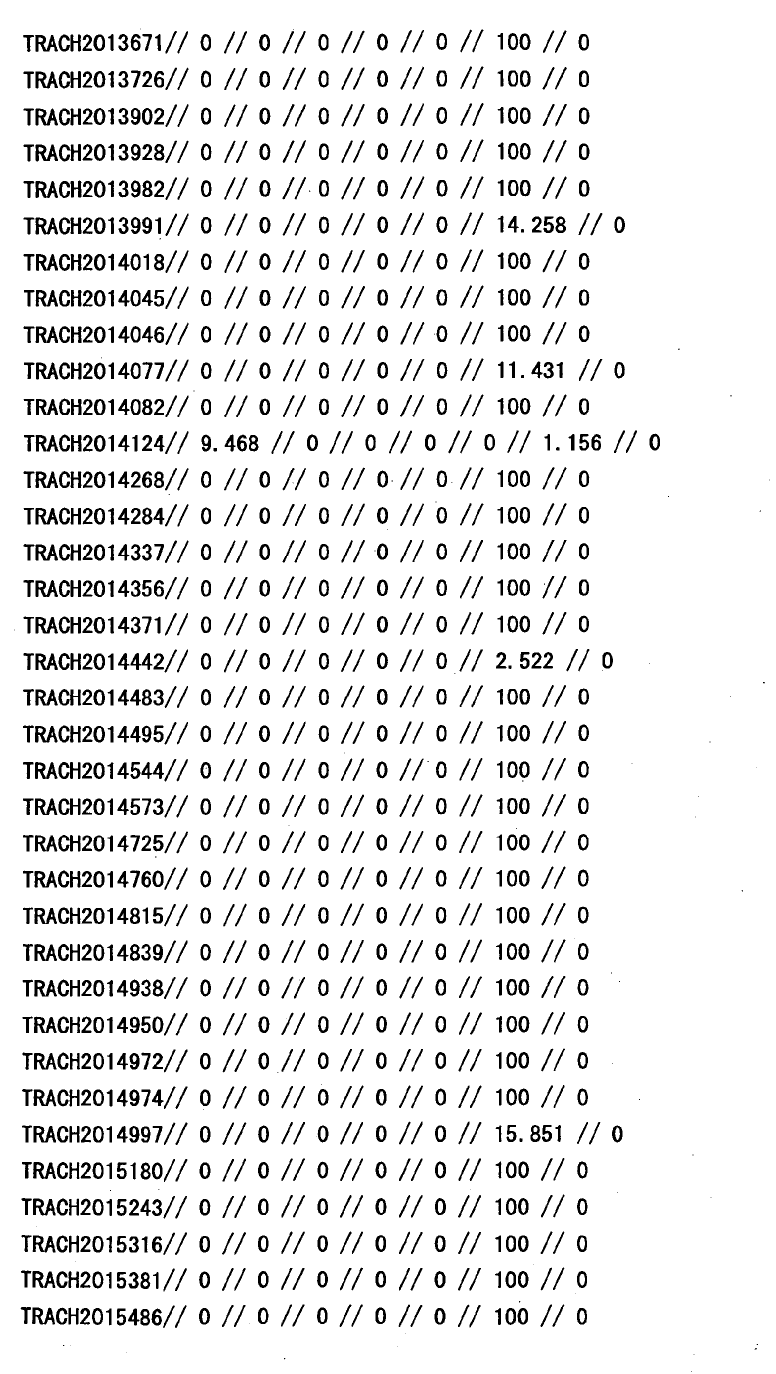

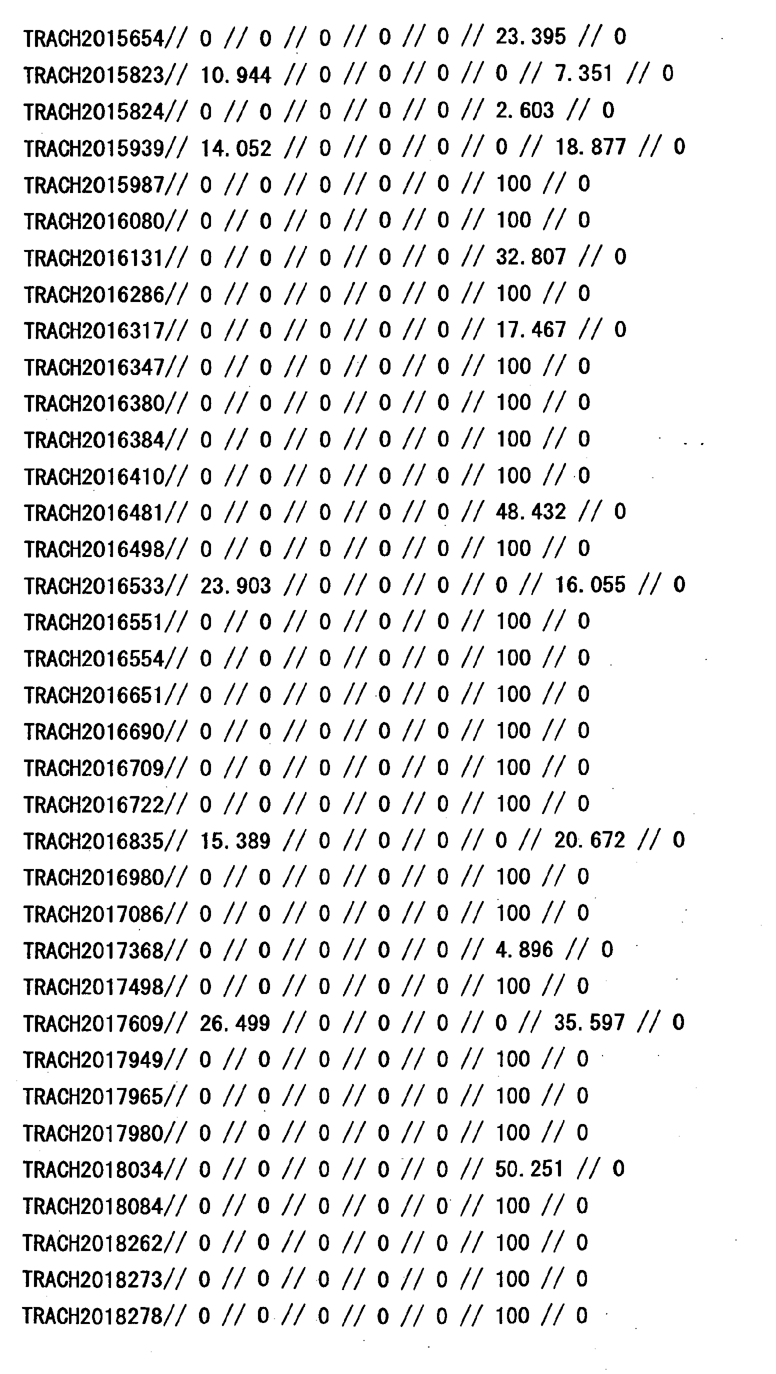

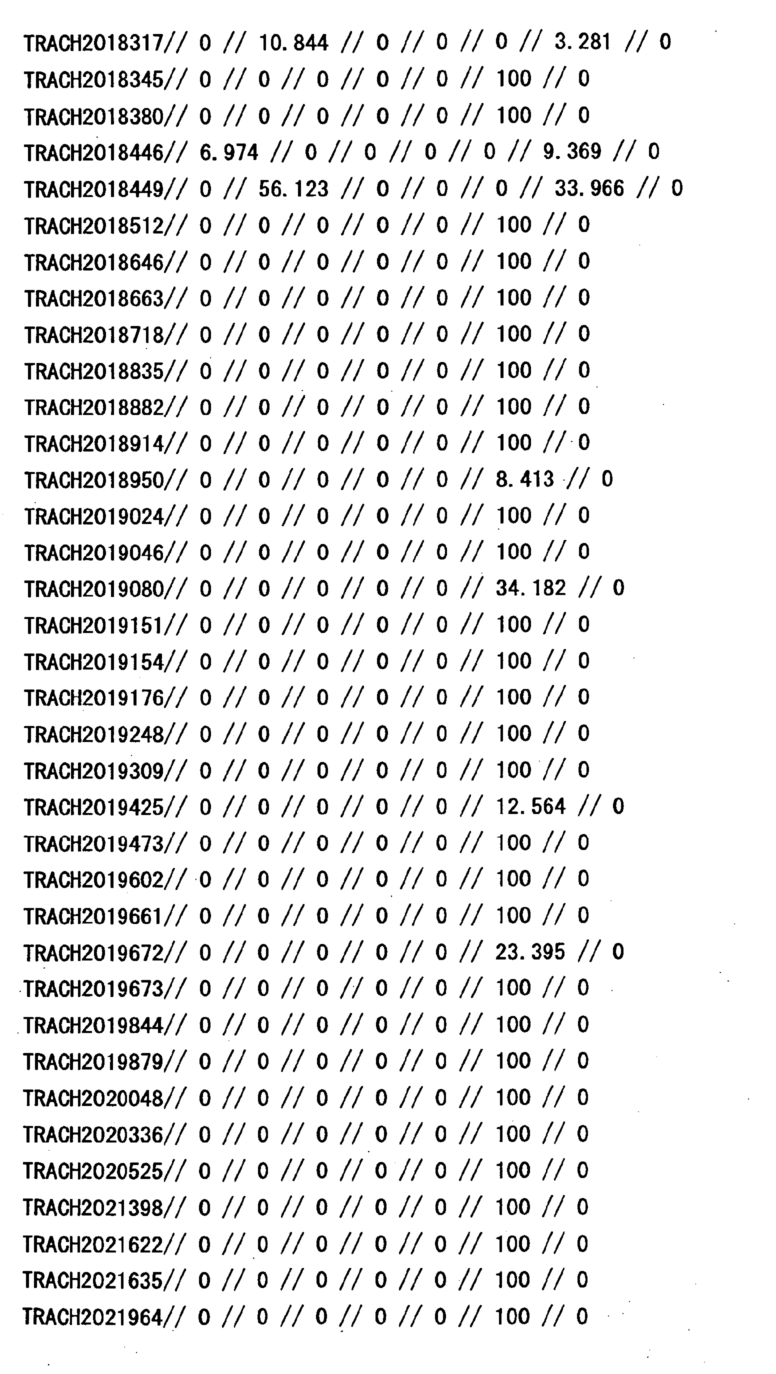

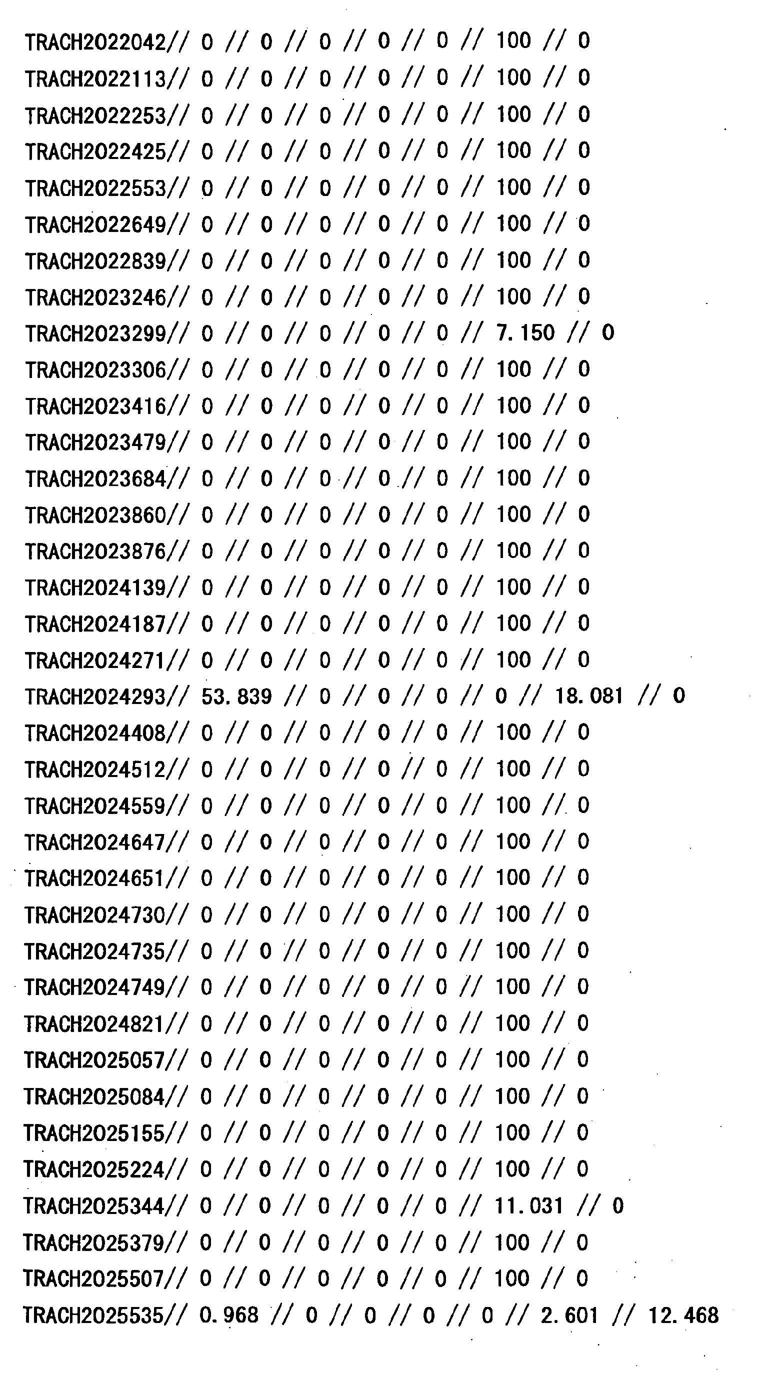

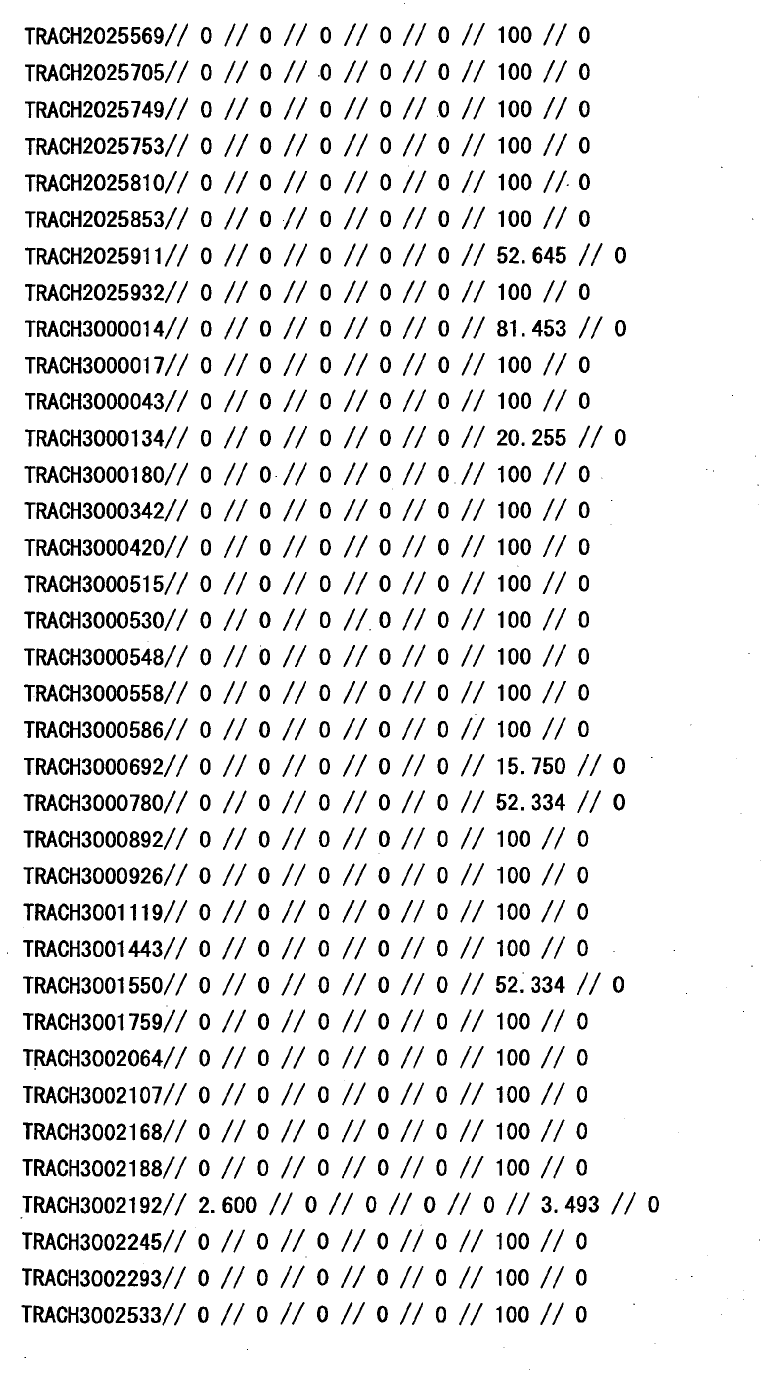

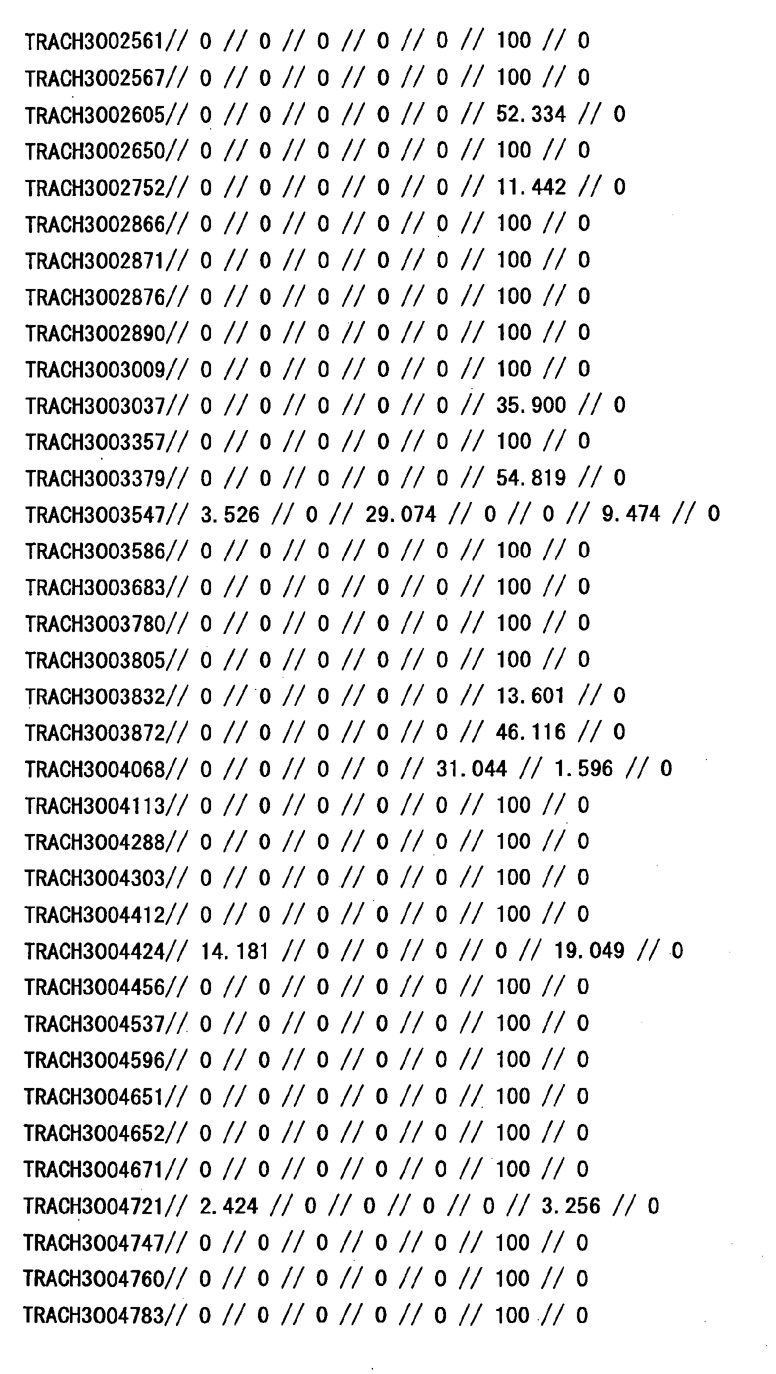

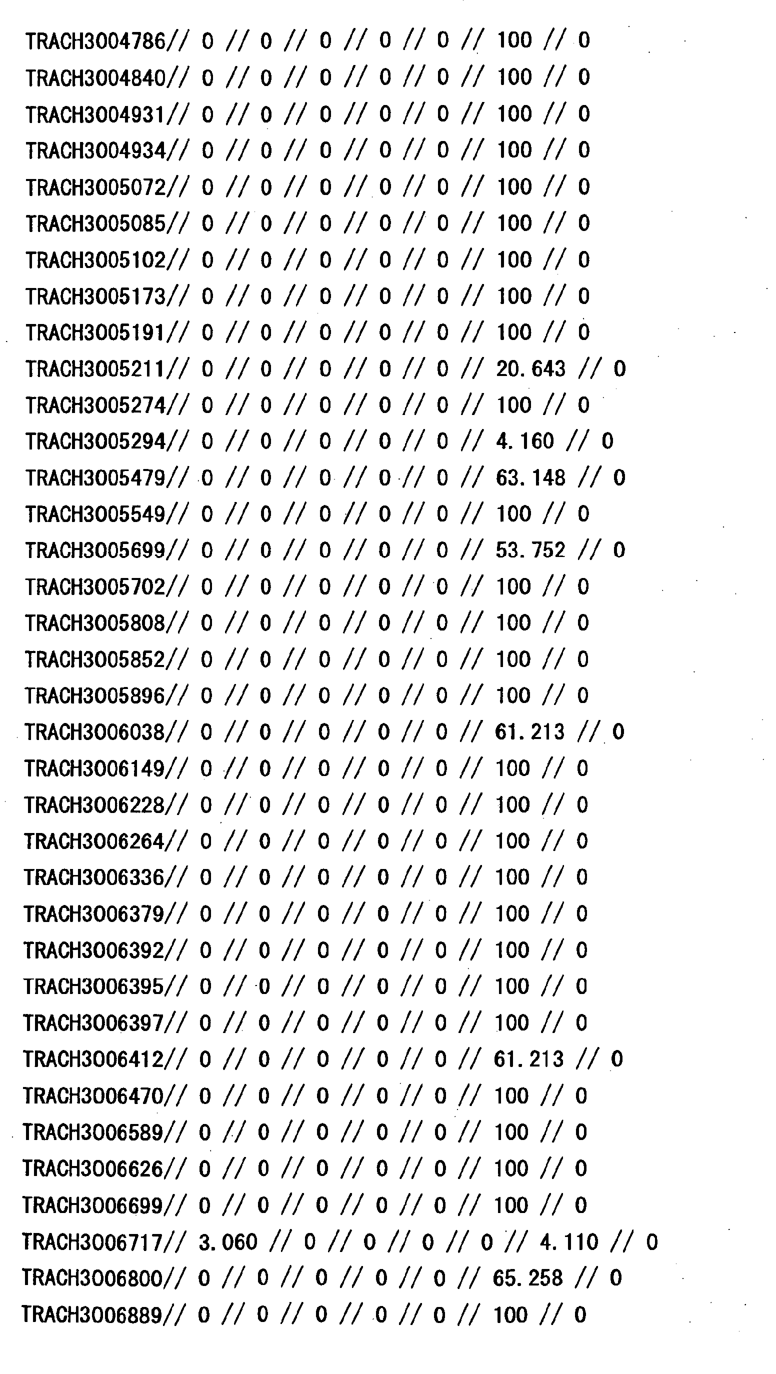

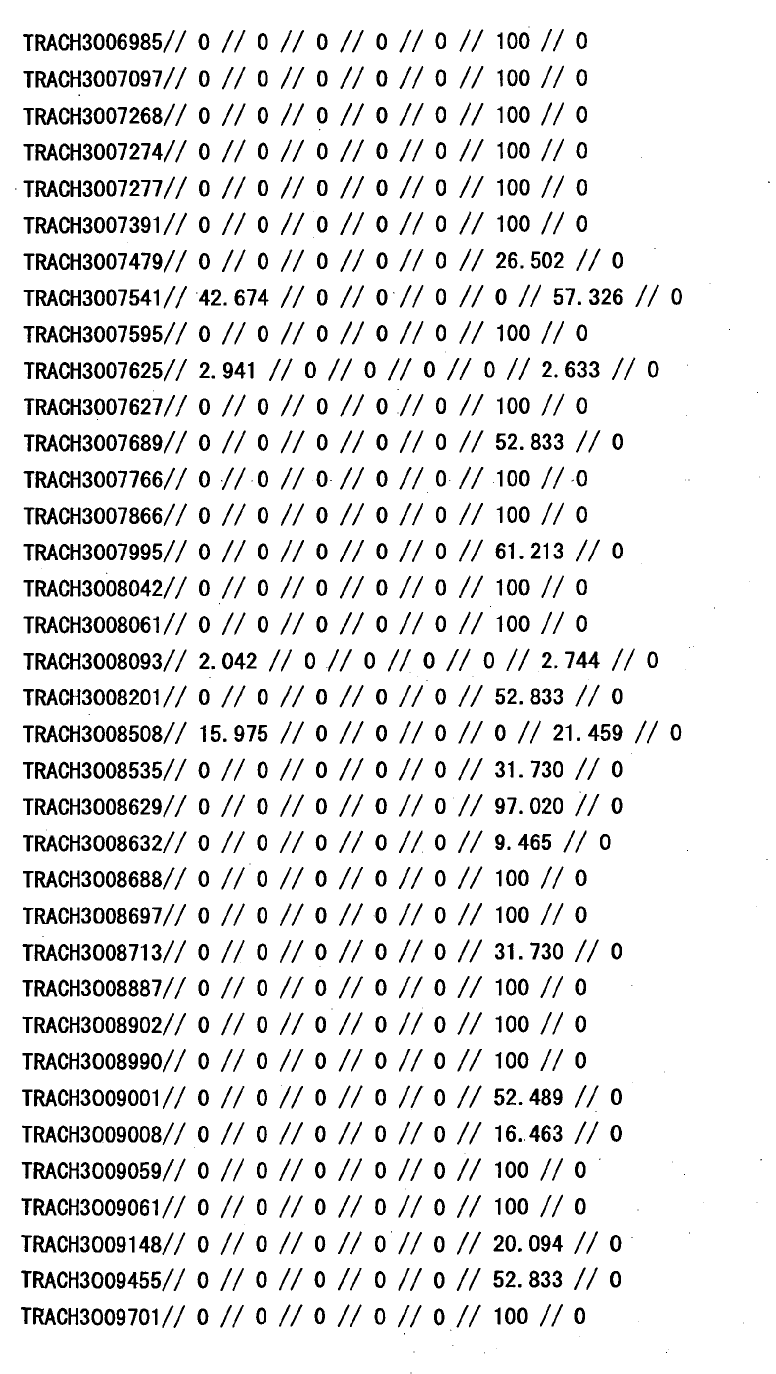

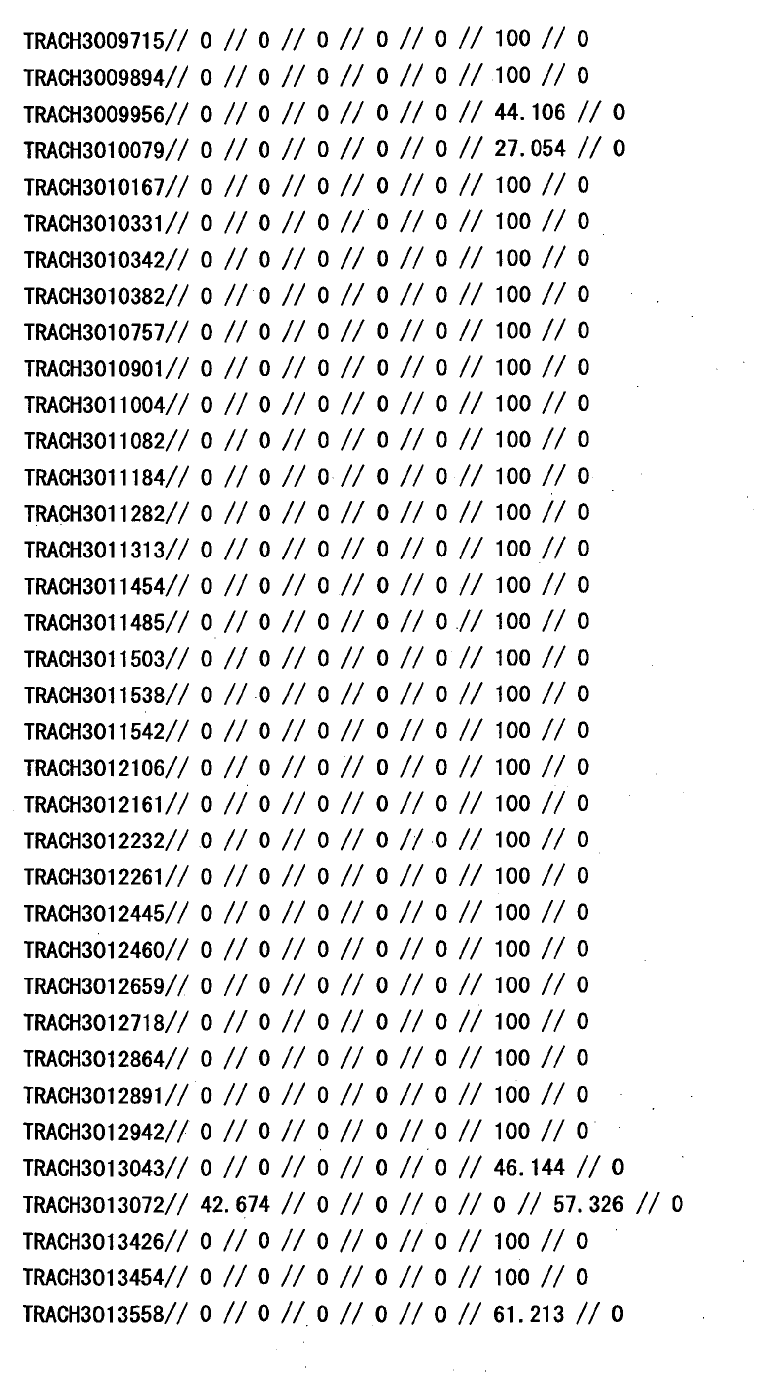

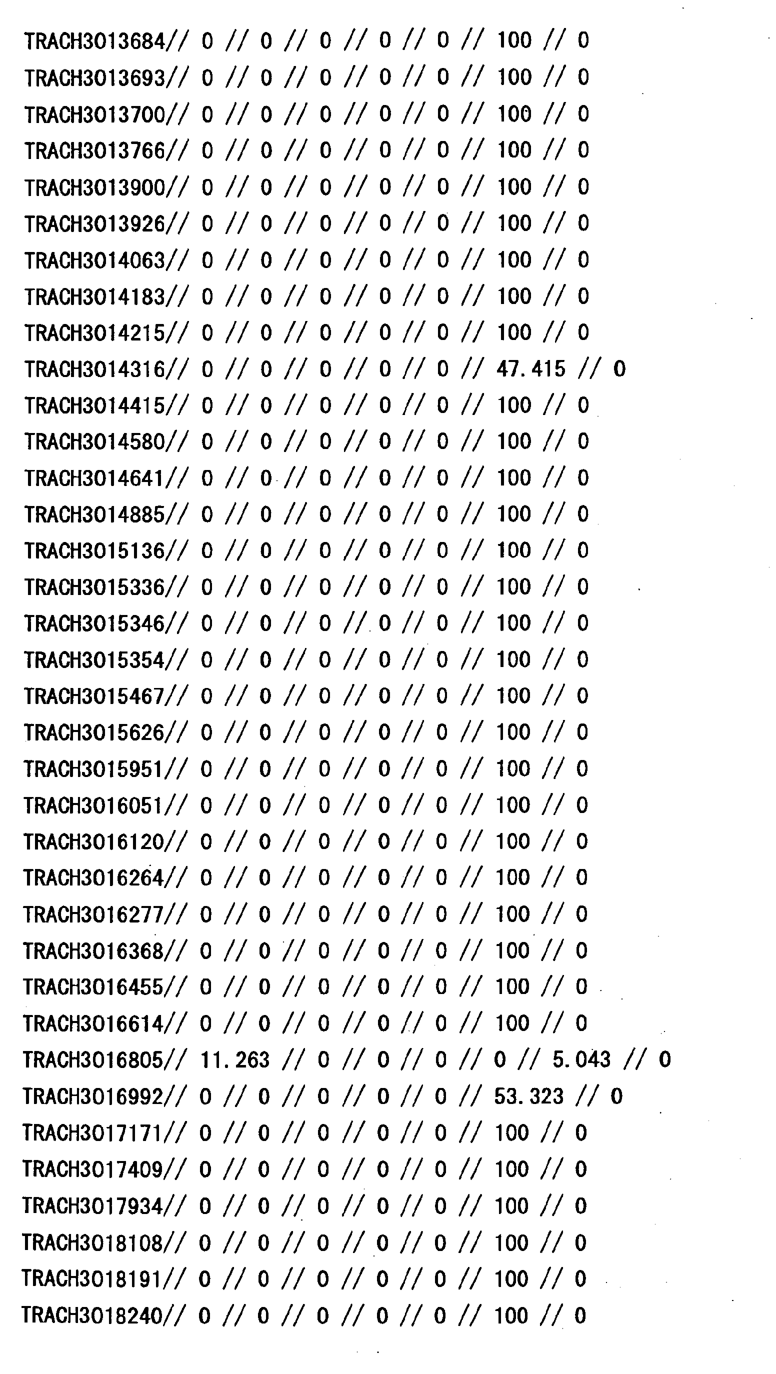

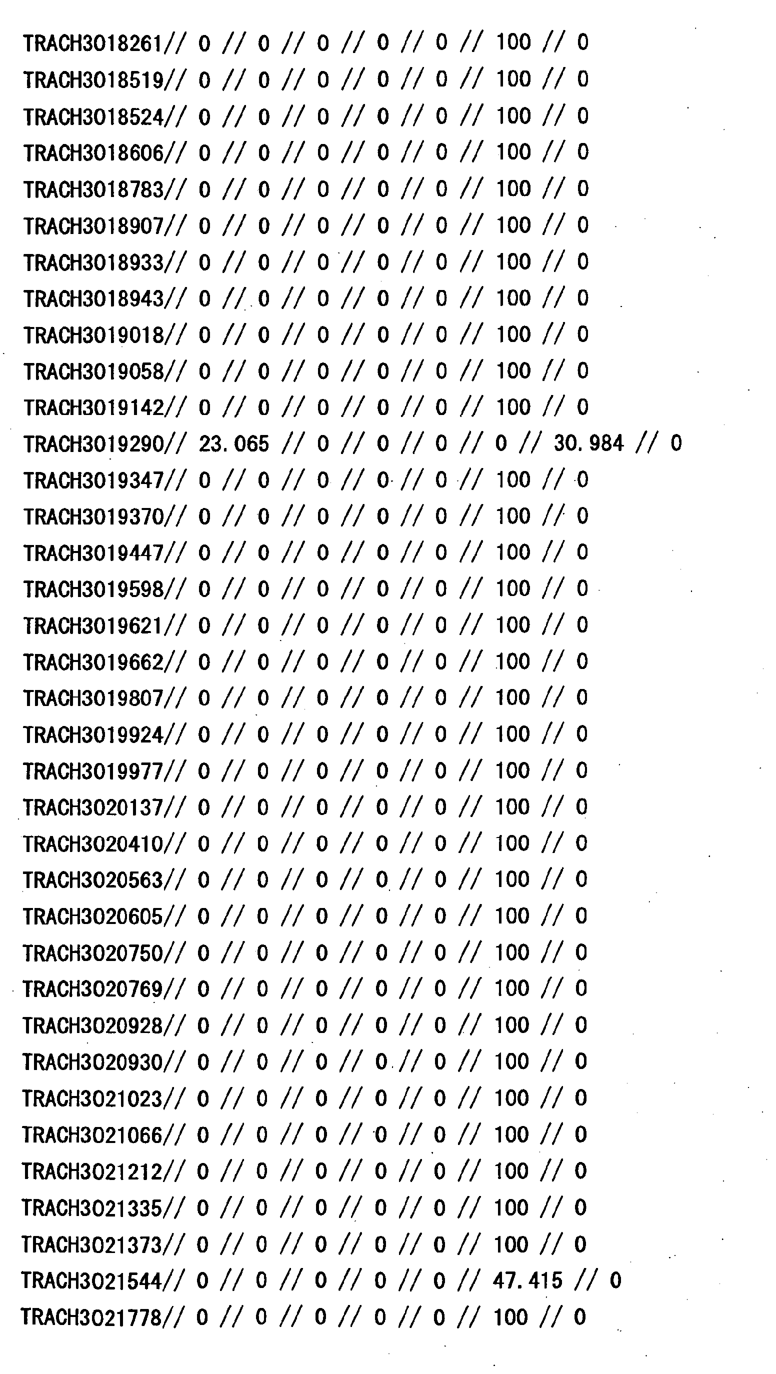

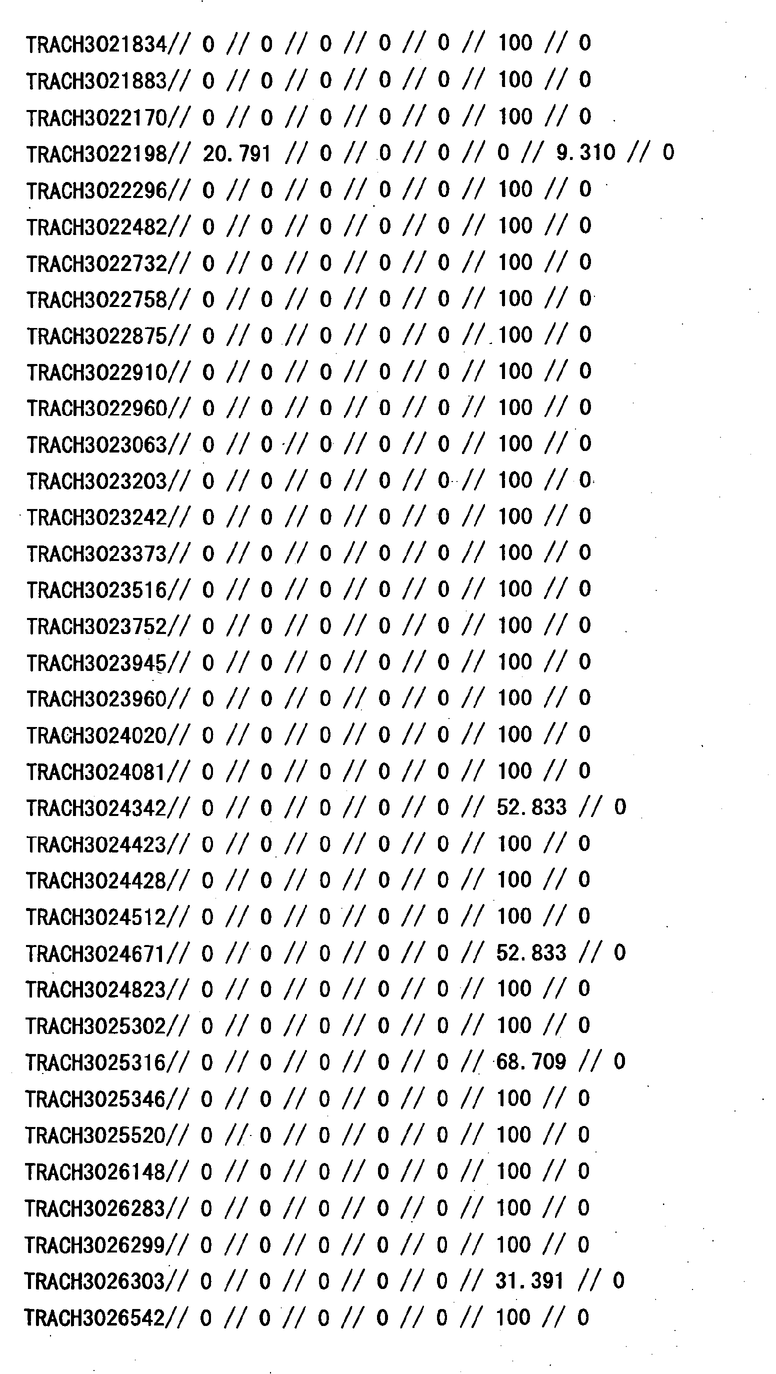

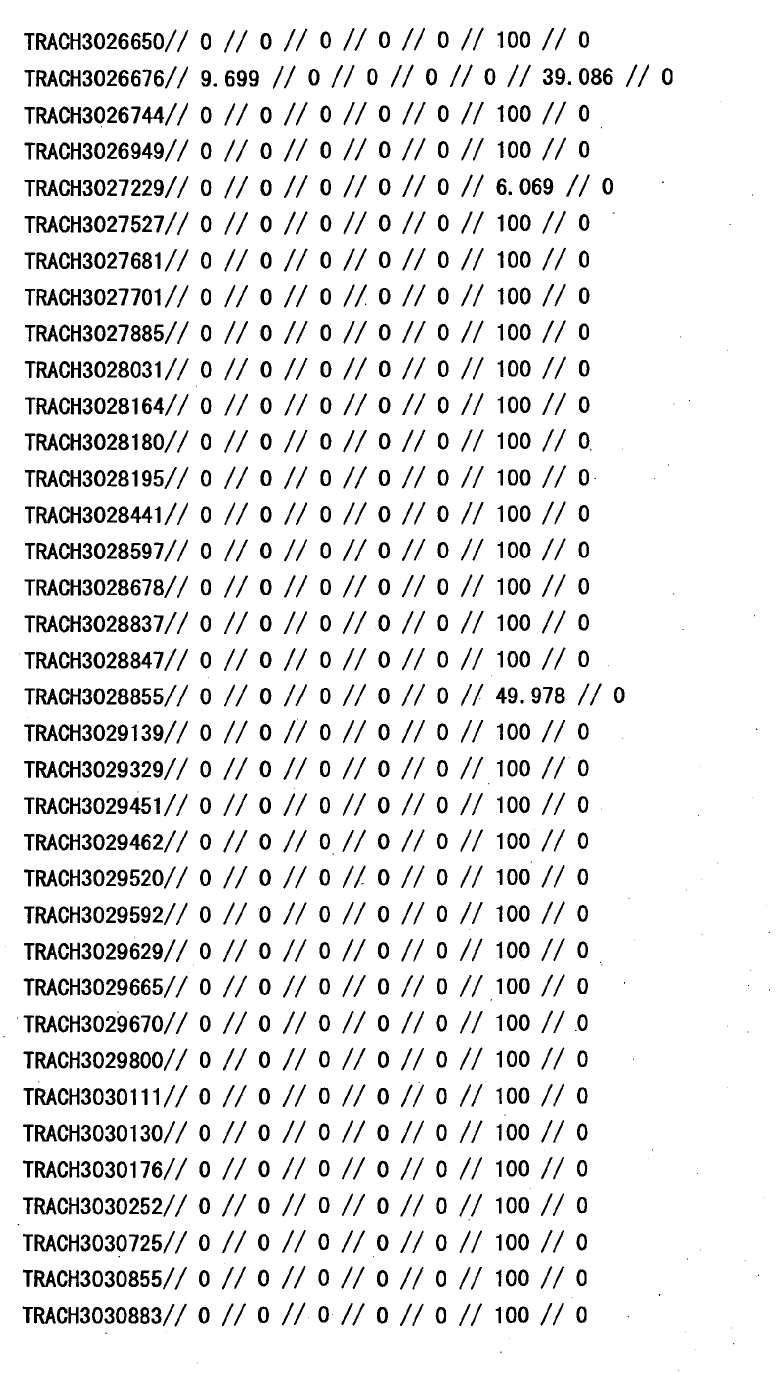

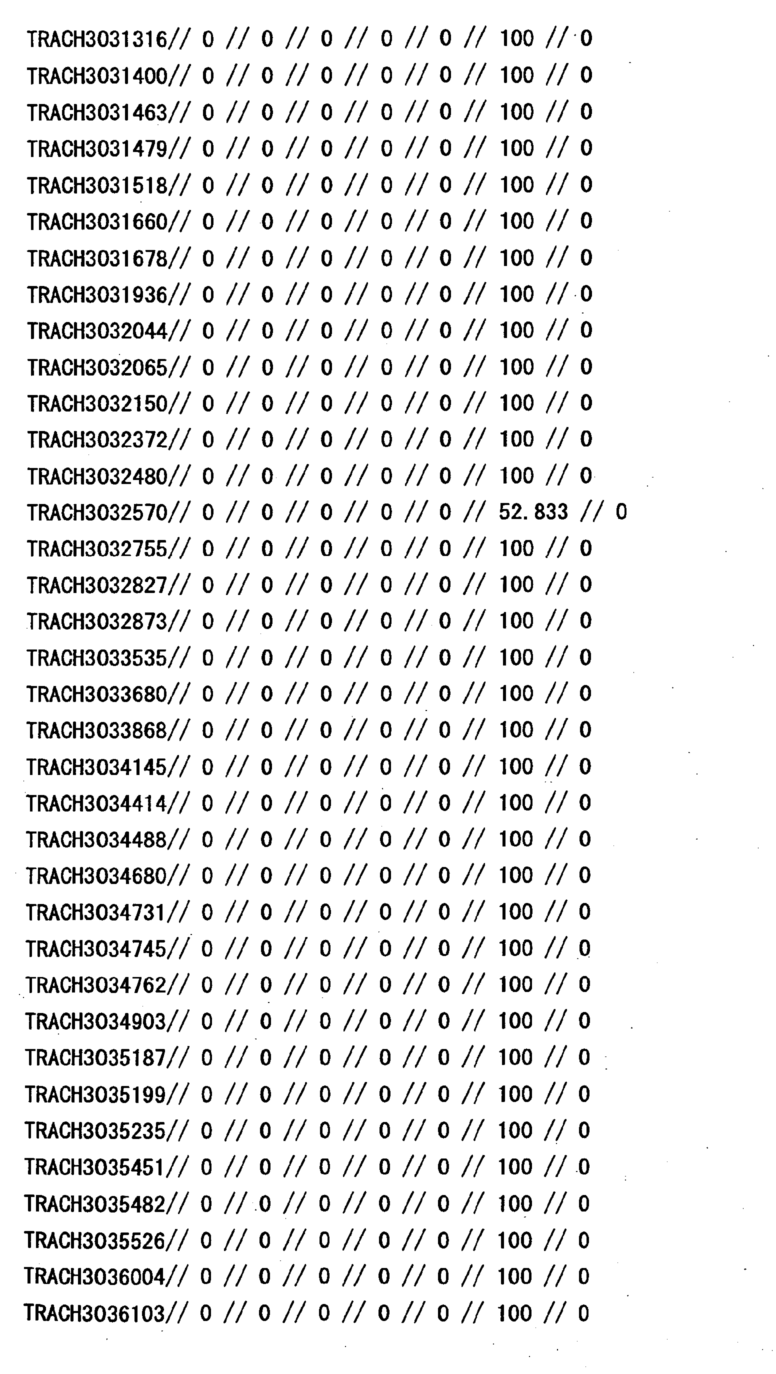

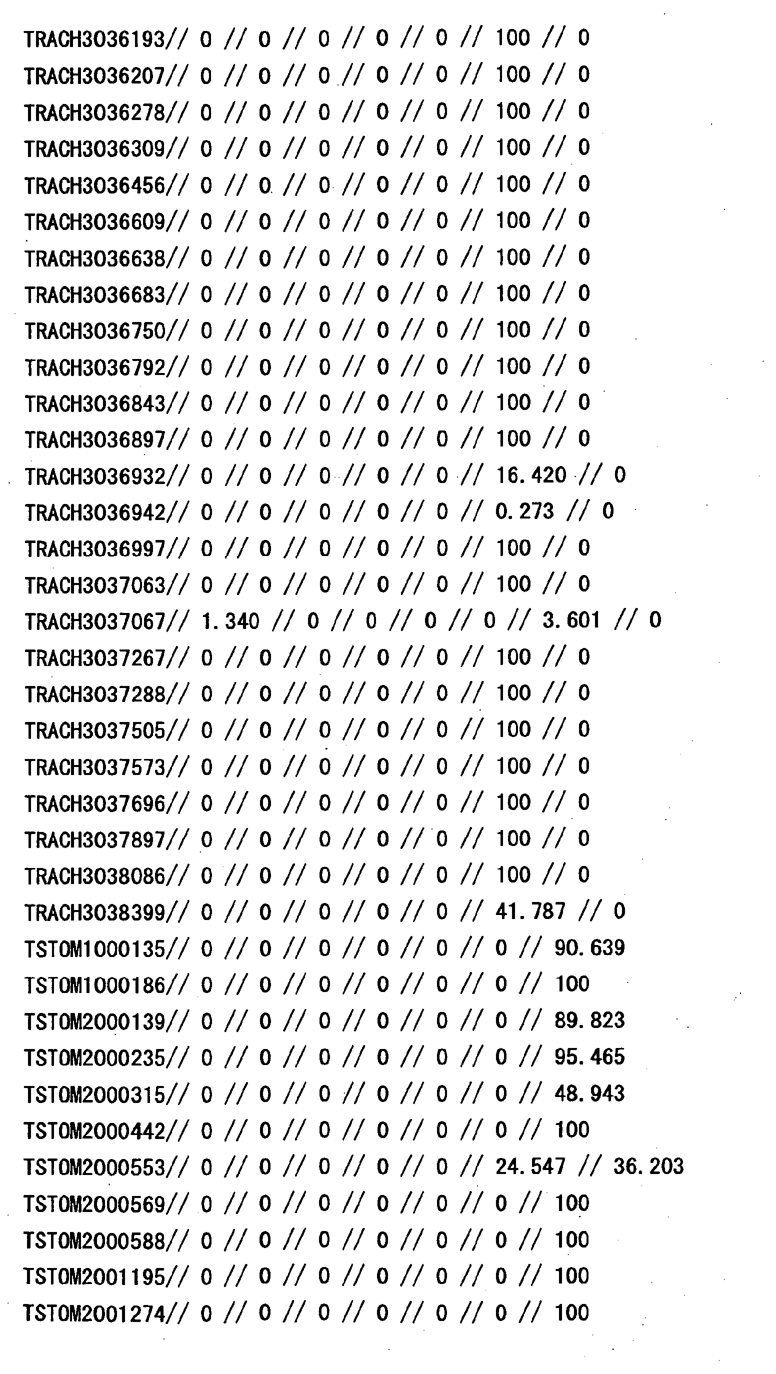

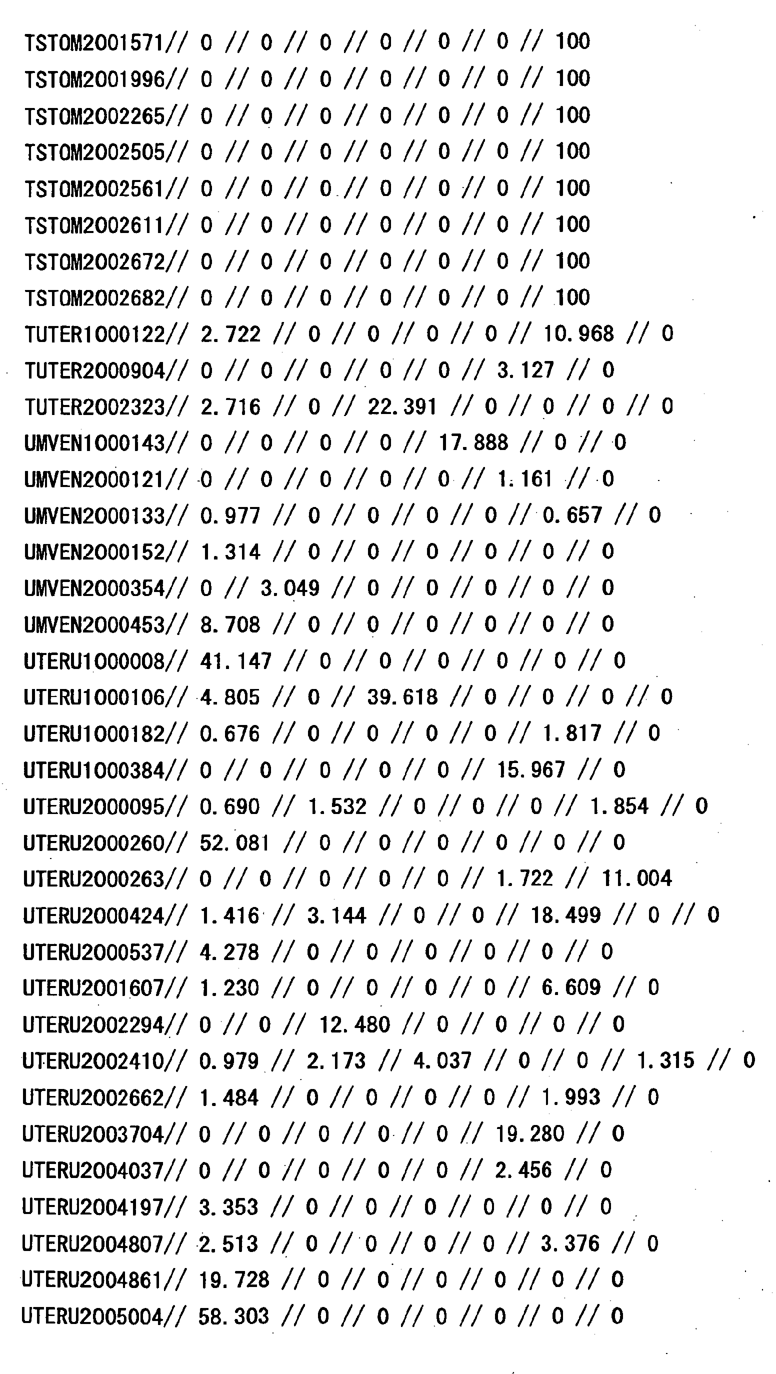

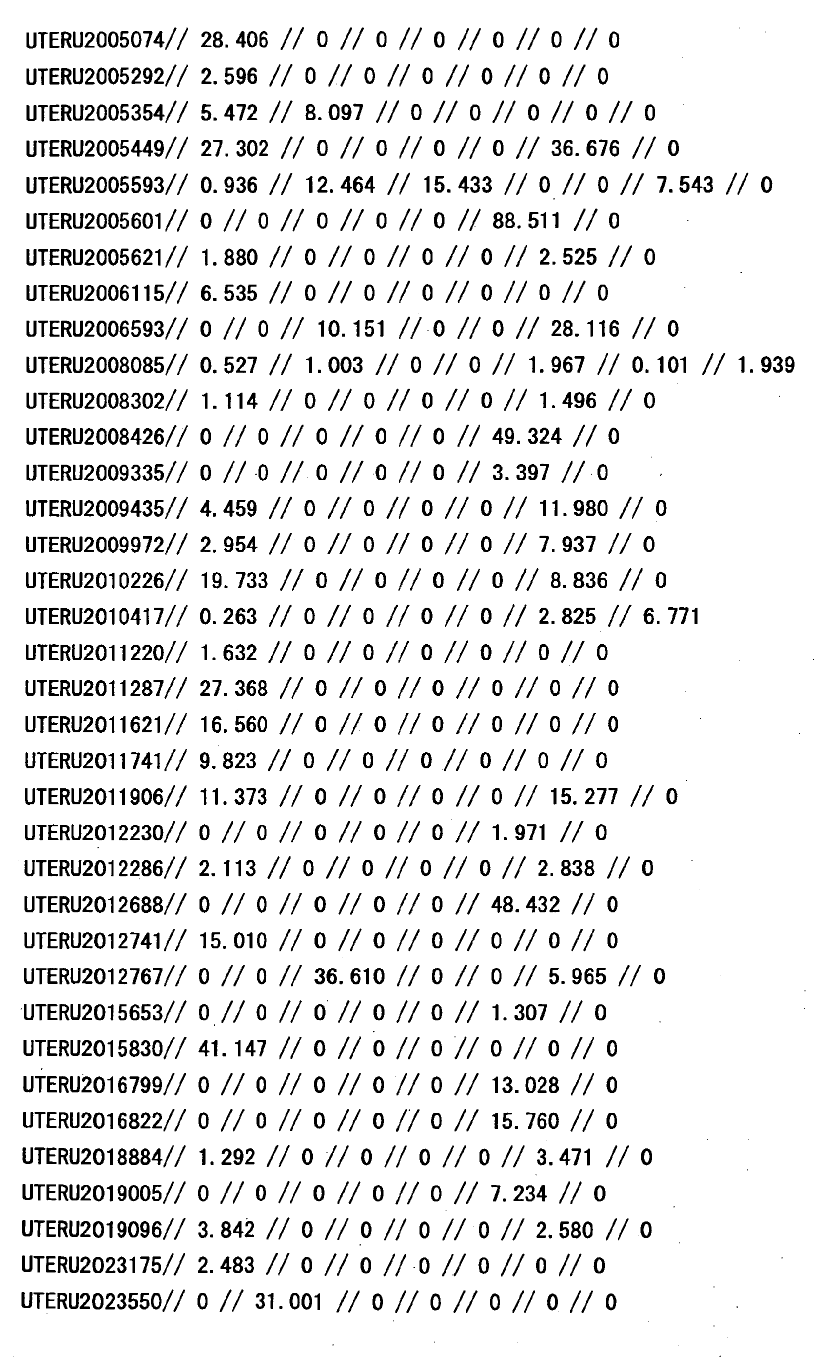

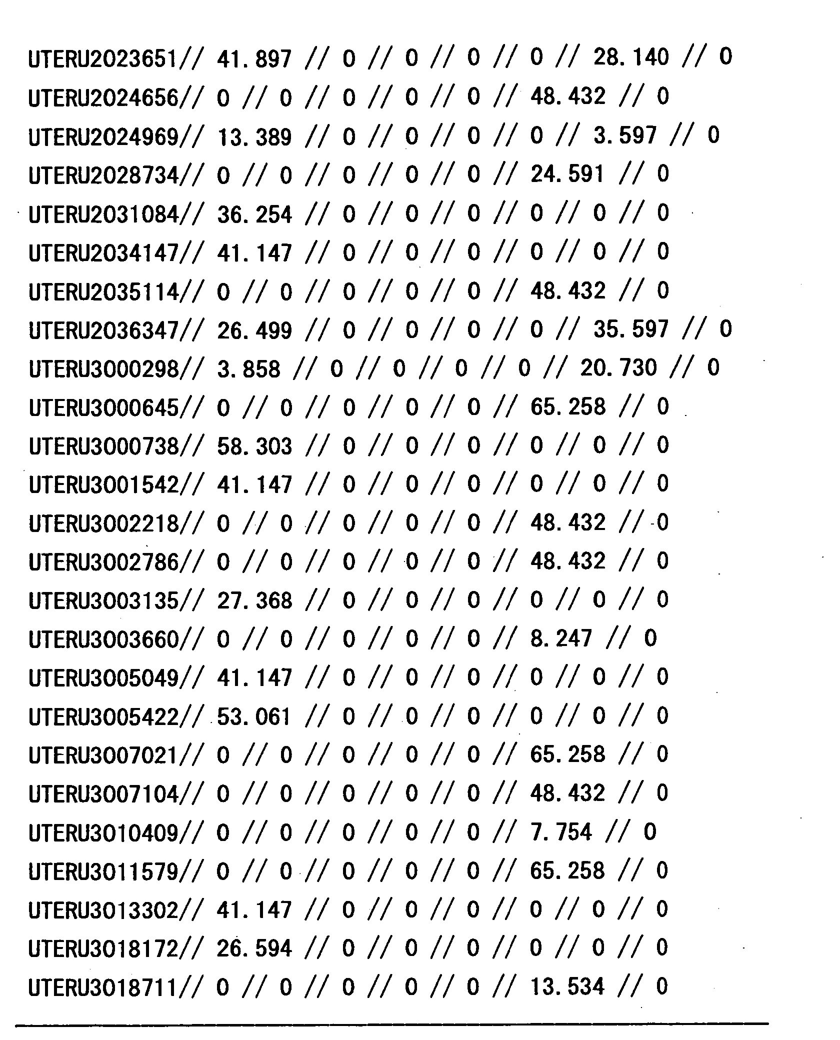

Genes whose expression levels were negligibly low in each of the above-described libraries are shown with their clone names below.
3NB691000018, 3NB691000085, 3NB691000113, 3NB691000129, 3NB691000191, 3NB692000012, 3NB692000029, 3NB692000102, 3NB692000154, 3NB692000276, 3NB692000305, 3NB692000429, 3NB692000484, 3NB692000529, 3NB692000973, 3NB692001002, 3NB692001034, 3NB692001040, 3NB692001123, 3NB692001267, 3NB692001339, 3NB692001349, 3NB692001366, 3NB692001408, 3NB692001433, 3NB692001459, 3NB692001471, 3NB692001496, 3NB692001501, 3NB692001507, 3NB692001511, 3NB692001519, 3NB692001528, 3NB692001548, 3NB692001557, 3NB692001637, 3NB692001719, 3NB692001861, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064,
ADIPS2000069, ADIPS2000088, ADIPS2000245, ADIPS2000425, ADRGL1000033, ADRGL1000038, ADRGL1000065, ADRGL1000067, ADRGL1000144, ADRGL1000147, ADRGL1000160, ADRGL1000165, ADRGL1000182, ADRGL2000006, ADRGL2000042, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002392, ADRGL2002679, ADRGL2002753, ADRGL2002857, ADRGL2003017,
ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008966, ADRGL2009146, ADRGL2009324,
ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2012179, AHMSC1000138, ASTRO1000009, ASTRO1000018, ASTRO1000029, ASTRO1000096, ASTRO1000165, ASTRO2000011, ASTRO2000014, ASTRO2000095, ASTRO2000191, ASTRO2000372, ASTRO2000381, ASTRO2000403, ASTRO2000417, ASTRO2000482, ASTRO2000533, ASTRO2000653, ASTRO2000662, ASTRO2000725, ASTRO2001001, ASTRO2001008, ASTRO2001088, ASTRO2001107, ASTRO2001122, ASTRO2001227, ASTRO2001249, ASTRO2001480, ASTRO2002035, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003574,
ASTRO2003581, ASTRO2003740, ASTRO2003925, ASTRO2003960, ASTRO2004032, ASTRO2004051, ASTRO2004177, ASTRO2004217, ASTRO2004297, ASTRO2004584, ASTRO2004751, ASTRO2004877, ASTRO2005008, ASTRO2005081, ASTRO2005096, ASTRO2005242, ASTRO2005273, ASTRO2005343, ASTRO2005413, ASTRO2005553, ASTRO2005557, ASTRO2005575, ASTRO2005726, ASTRO2005863, ASTRO2005896, ASTRO2005902, ASTRO2006356, ASTRO2006475, ASTRO2006732, ASTRO2006747, ASTRO2006952, ASTRO2007221, ASTRO2007377, ASTRO2007515, ASTRO2007578, ASTRO2007643, ASTRO2007666, ASTRO2007956, ASTRO2007962, ASTRO2008040, ASTRO2008239, ASTRO2008409, ASTRO2008425, ASTRO2008508, ASTRO2008777, ASTRO2008895, ASTRO2008960, ASTRO2008972, ASTRO2009177, ASTRO2009333,
ASTRO2009458, ASTRO2009526, ASTRO2009879, ASTRO2010072, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013802, ASTRO2014174, ASTRO2014211, ASTRO2014363, ASTRO2014392, ASTRO2014498, ASTRO2014576, ASTRO2014923, ASTRO2014961, ASTRO2015162, ASTRO2015185, ASTRO2015328, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000193, BGGI11000285,
BGGI12000022, BGGI12000067, BGGI12000161, BGGI12000544, BGGI12000616, BGGI12000684, BGGI12000693, BGGI12001097, BGGI12001241, BGGI12001247, BGGI12001714, BLADE1000176, BLADE2000181, BLADE2000256, BLADE2000340, BLADE2000389, BLADE2000463, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004345, BLADE2004389, BLADE2004462, BLADE2004628, BLADE2004670, BLADE2004722, BLADE2004849, BLADE2005036,
BLADE2005038, BLADE2005229, BLADE2005459, BLADE2005792, BLADE2006043, BLADE2006415, BLADE2006607, BLADE2006826, BLADE2006849, BLADE2006947, BLADE2007033, BLADE2007043, BLADE2007722, BLADE2007735, BLADE2007744, BLADE2007799, BLADE2007923, BLADE2007935, BLADE2007958, BLADE2008281, BLADE2008454, BLADE2008479, BLADE2008539, BLADE2008809, BLADE2008995, BLADE2009452, BLADE2009479, BLADE2009593, BNGH41000104, BNGH41000118, BNGH41000169, BNGH41000177, BNGH41000198, BNGH41000216, BNGH42000130, BNGH42000132, BNGH42000405, BNGH42000532, BNGH42000675, BNGH42000791, BNGH42000815, BNGH42000994, BNGH42001247, BNGH42001406, BNGH42001576, BNGH42001724, BNGH42002244, BNGH42002387, BNGH42002487, BNGH42002489,
BNGH42003529, BNGH42003570, BNGH42003641, BNGH42004076, BNGH42004291, BNGH42004538, BNGH42004679, BNGH42005017, BNGH42005235, BNGH42005504, BNGH42006226, BNGH42006234, BNGH42007037, BNGH42007594, BNGH42007798, BNGH42008199, BNGH42008510, BNGH42008649, BNGH42008832, BNGH42008850, BNGH42008943, BNGH42009021, BNGH42009025, BRACE1000047, BRACE1000051, BRACE1000065, BRACE1000084, BRACE1000087, BRACE1000092, BRACE1000093, BRACE1000101, BRACE1000114, BRACE1000124, BRACE1000132, BRACE1000169, BRACE1000187, BRACE1000239, BRACE1000258, BRACE1000451, BRACE1000475, BRACE1000572, BRACE2000077, BRACE2000078, BRACE2000100, BRACE2000109, BRACE2000141, BRACE2000148, BRACE2000183, BRACE2000229, BRACE2000232,
BRACE2000256, BRACE2000266, BRACE2000267, BRACE2000280, BRACE2000307, BRACE2000331, BRACE2000351, BRACE2000368, BRACE2000392, BRACE2000441, BRACE2000489, BRACE2000505, BRACE2000526, BRACE2000531, BRACE2000545, BRACE2000553, BRACE2000577, BRACE2000640, BRACE2000650, BRACE2000677, BRACE2000698, BRACE2000718, BRACE2000733, BRACE2000743, BRACE2000750, BRACE2000815, BRACE2000885, BRACE2000905, BRACE2000936, BRACE2000947, BRACE2000965, BRACE2000969, BRACE2000988, BRACE2001017, BRACE2001070, BRACE2001094, BRACE2001107, BRACE2001151, BRACE2001191, BRACE2001197, BRACE2001236, BRACE2001239, BRACE2001269, BRACE2001312, BRACE2001330, BRACE2001340, BRACE2001352, BRACE2001353, BRACE2001374, BRACE2001375,
BRACE2001423, BRACE2001424, BRACE2001445, BRACE2001453, BRACE2001455, BRACE2001492, BRACE2001508, BRACE2001534, BRACE2001543, BRACE2001564, BRACE2001588, BRACE2001607, BRACE2001626, BRACE2001677, BRACE2001705, BRACE2001726, BRACE2001733, BRACE2001737, BRACE2001742, BRACE2001777, BRACE2001779, BRACE2001859, BRACE2001865, BRACE2001872, BRACE2001881, BRACE2001898, BRACE2001923, BRACE2001954, BRACE2001972, BRACE2001973, BRACE2002026, BRACE2002034, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002394, BRACE2002409, BRACE2002431, BRACE2002441, BRACE2002444, BRACE2002458,
BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002676, BRACE2002682, BRACE2002695, BRACE2002707, BRACE2002736, BRACE2002752, BRACE2002762, BRACE2002772, BRACE2002792, BRACE2002796, . BRACE2002803, BRACE2002807, BRACE2002834, BRACE2002849, BRACE2002857, BRACE2002861, BRACE2002884, BRACE2002896, BRACE2002948, BRACE2003078, BRACE2003097, BRACE2003110, BRACE2003168, BRACE2003199, BRACE2003210, BRACE2003217, BRACE2003269, BRACE2003285, BRACE2003319, BRACE2003398, BRACE2003420, BRACE2003428, BRACE2003431, BRACE2003512, BRACE2003516, BRACE2003539,
BRACE2003540, BRACE2003598, BRACE2003609, BRACE2003614, BRACE2003639, BRACE2003700, BRACE2003713, BRACE2003766, BRACE2003802, BRACE2003825, BRACE2003847, BRACE2003873, BRACE2003885, BRACE2003887, BRACE2003904, BRACE2003905, BRACE2003928, BRACE2003954, BRACE2003987, BRACE2005034, BRACE2005047, BRACE2005063, BRACE2005083, BRACE2005090, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005243, BRACE2005251, BRACE2005253, BRACE2005290, BRACE2005328, BRACE2005329, BRACE2005337, BRACE2005348, BRACE2005363, BRACE2005434, BRACE2005448, BRACE2005450, BRACE2005453, BRACE2005457, BRACE2005460,
BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006072, BRACE2006075, BRACE2006089, BRACE2006122, BRACE2006137, BRACE2006143, BRACE2006167, BRACE2006174, BRACE2006240, BRACE2006245, BRACE2006274, BRACE2006281, BRACE2006298, BRACE2006344, BRACE2006354, BRACE2006380, BRACE2006393, BRACE2006413, BRACE2006417, BRACE2006453,
BRACE2006459, BRACE2006488, BRACE2006534, BRACE2006540, BRACE2006587, BRACE2006598, BRACE2006636, BRACE2006685, BRACE2006703, BRACE2006743, BRACE2006783, BRACE2006821, BRACE2006833, BRACE2006859, BRACE2006909, BRACE2006911, BRACE2006924, BRACE2006935, BRACE2006944, BRACE2006951, BRACE2006986, BRACE2007013, BRACE2007087, BRACE2007138, BRACE2007197, BRACE2007201, BRACE2007203, BRACE2007232, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563, BRACE2007577, BRACE2007621, BRACE2007640, BRACE2007641, BRACE2007646,
BRACE2007663, BRACE2007671, BRACE2007682, BRACE2007685, BRACE2007708, BRACE2007727, BRACE2007749, BRACE2007760, BRACE2007761, BRACE2007764, BRACE2007768, BRACE2007769, BRACE2007784, BRACE2007786, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008401, BRACE2008410, BRACE2008420, BRACE2008443, BRACE2008473, BRACE2008480, BRACE2008481, BRACE2008488, BRACE2008495,
BRACE2008500, BRACE2008553, BRACE2008594, BRACE2008604, BRACE2008615, BRACE2008622, BRACE2008629, BRACE2008655, BRACE2008656, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008999, BRACE2009014, BRACE2009016, BRACE2009031, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009307, BRACE2009311, BRACE2009318, BRACE2009323, BRACE2009366, BRACE2009421,
BRACE2009428, BRACE2009434, BRACE2009437, BRACE2009517, BRACE2009533, BRACE2009566, BRACE2009591, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010753, BRACE2010813, BRACE2010828, BRACE2010837, BRACE2010888, BRACE2010937, BRACE2010983, BRACE2011007, BRACE2011025, BRACE2011199, BRACE2011389, BRACE2011478, BRACE2011545, BRACE2011592, BRACE2011611, BRACE2011646, BRACE2011677, BRACE2011703, BRACE2011747, BRACE2011838,
BRACE2011892, BRACE2011904, BRACE2011947, BRACE2012061, BRACE2012062, BRACE2012185, BRACE2012222, BRACE2012232, BRACE2012291, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2014108, BRACE2014161, BRACE2014232, BRACE2014268, BRACE2014475, BRACE2014657, BRACE2014780, BRACE2014818, BRACE2014821, BRACE2014824, BRACE2014854, BRACE2015031, BRACE2015132, BRACE2015262, BRACE2015270, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315,
BRACE2016325, BRACE2016335, BRACE2016366, BRACE2016465, BRACE2016516, BRACE2016583, BRACE2016708, BRACE2016821, BRACE2016896, BRACE2017108, BRACE2017124, BRACE2017298, BRACE2017359, BRACE2017410, BRACE2017438, BRACE2017580, BRACE2017587, BRACE2017720, BRACE2017844, BRACE2017872, BRACE2017929, BRACE2017934, BRACE2017992, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019528, BRACE2019618, BRACE2019696, BRACE2019703, BRACE2019843, BRACE2019865, BRACE2019872, BRACE2020028,
BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404,
BRACE2023451, BRACE2023540, BRACE2023633, BRACE2023718, BRACE2023744, BRACE2023800, BRACE2024222, BRACE2024610, BRACE2024627, BRACE2024781, BRACE2024826, BRACE2025018, BRACE2025316, BRACE2025333, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026836, BRACE2026920, BRACE2026971, BRACE2027018, BRACE2027221, BRACE2027258, BRACE2027312, BRACE2027382, BRACE2027389, BRACE2027408, BRACE2027767, BRACE2027770, BRACE2027879, BRACE2027896, BRACE2028171, BRACE2028392, BRACE2028410, BRACE2028636, BRACE2028741, BRACE2028956, BRACE2028970, BRACE2029112, BRACE2029396,
BRACE2029581, BRACE2029849, BRACE2030039, BRACE2030096, BRACE2030326, BRACE2030341, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035441, BRACE2035485, BRACE2036005, BRACE2036096, BRACE2036743, BRACE2036830, BRACE2036834, BRACE2036900,
BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142,
BRACE2043248, BRACE2043349, BRACE2043381, BRACE2043774, BRACE2043942, BRACE2044057, BRACE2044105, BRACE2044263, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3001002, BRACE3001058, BRACE3001066, BRACE3001113, BRACE3001217, BRACE3001299, BRACE3001384, BRACE3001391, BRACE3001403, BRACE3001595,
BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003595, BRACE3003866, BRACE3003920, BRACE3004046, BRACE3004058, BRACE3004113, BRACE3004150, BRACE3004205, BRACE3004358, BRACE3004371, BRACE3004435, BRACE3004477, BRACE3004767, BRACE3004772, BRACE3004783, BRACE3004843, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005760, BRACE3005870, BRACE3005903, BRACE3006113, BRACE3006185, BRACE3006226, BRACE3006462, BRACE3006463, BRACE3006872,
BRACE3006917, BRACE3007058, BRACE3007084, BRACE3007085, BRACE3007322, BRACE3007472, BRACE3007480, BRACE3007559, BRACE3007625, BRACE3007642, BRACE3007649, BRACE3007767, BRACE3007869, BRACE3008066, BRACE3008092, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009539, BRACE3009574, BRACE3009701, BRACE3009708, BRACE3009724, BRACE3009993, BRACE3010076, BRACE3010079, BRACE3010086, BRACE3010221, BRACE3010315, BRACE3010397, BRACE3010416, BRACE3010435, BRACE3010702, BRACE3010862, BRACE3011271, BRACE3011447, BRACE3011505,
BRACE3011634, BRACE3011774, BRACE3012357, BRACE3012574, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3018083, BRACE3018308, BRACE3018963, BRACE3019055, BRACE3019071, BRACE3019084, BRACE3019570, BRACE3019611, BRACE3019817, BRACE3019828, BRACE3020151,
BRACE3020194, BRACE3020286, BRACE3020356, BRACE3020594, BRACE3020671, BRACE3020890, BRACE3021148, BRACE3021430, BRACE3021517, BRACE3021805, BRACE3022051, BRACE3022303, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027480, BRACE3027931, BRACE3028214, BRACE3028241, BRACE3028360,
BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032771, BRACE3032980, BRACE3032983, BRACE3033525, BRACE3034159, BRACE3034183, BRACE3034389, BRACE3034964, BRACE3034993, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239,
BRACE3040504, BRACE3040644, BRACE3040856, BRACE3041059, BRACE3041162, BRACE3041827, BRACE3042046, BRACE3042210, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2001279, BRALZ2001350, BRALZ2001445, BRALZ2001559, BRALZ2001743, BRALZ2001834, BRALZ2002174,
BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085,
BRALZ2016498, BRALZ2017359, BRALZ2017531, BRALZ2017607, BRALZ2017620, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000411, BRAMY2000653, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621,
BRAMY2002638, BRAMY2002739, BRAMY2002799, BRAMY2002853, BRAMY2002862, BRAMY2002956, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005648, BRAMY2005662, BRAMY2005708, BRAMY2005711, BRAMY2006092, BRAMY2006118, BRAMY2006149, BRAMY2006397,
BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145,
BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012691, BRAMY2012721, BRAMY2012731, BRAMY2012776, BRAMY2012880, BRAMY2012881, BRAMY2013200, BRAMY2013216, BRAMY2013405,
BRAMY2013414, BRAMY2013572, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2015251, BRAMY2015311, BRAMY2015420, BRAMY2015503, BRAMY2015516, BRAMY2015551, BRAMY2015559, BRAMY2015688, BRAMY2015705, BRAMY2015855, BRAMY2015890, BRAMY2015911, BRAMY2015925, BRAMY2016002, BRAMY2016020, BRAMY2016070, BRAMY2016251, BRAMY2016325, BRAMY2016327, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2018027, BRAMY2018106, BRAMY2018126, BRAMY2018272, BRAMY2018273, BRAMY2018279,
BRAMY2018295, BRAMY2018308, BRAMY2018396, BRAMY2018467, BRAMY2018635, BRAMY2019055, BRAMY2019111, BRAMY2019277, BRAMY2019300, BRAMY2019468, BRAMY2019509, BRAMY2019643, BRAMY2019710, BRAMY2019985, BRAMY2020050, BRAMY2020058, BRAMY2020106, BRAMY2020270, BRAMY2020354, BRAMY2020355, BRAMY2020445, BRAMY2020466, BRAMY2020520, BRAMY2020574, BRAMY2020634, BRAMY2020713, BRAMY2021040, BRAMY2021063, BRAMY2021098, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022263, BRAMY2022301, BRAMY2022320, BRAMY2022383, BRAMY2022454, BRAMY2022525, BRAMY2022532,
BRAMY2022723, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023482, BRAMY2023514, BRAMY2023719, BRAMY2023786, BRAMY2023852, BRAMY2023863, BRAMY2023939, BRAMY2024004, BRAMY2024073, BRAMY2024312, BRAMY2024374, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026091, BRAMY2026168,
BRAMY2026300, BRAMY2026347, BRAMY2026395, BRAMY2026405, BRAMY2026533, BRAMY2026538, BRAMY2026713, BRAMY2026778, BRAMY2026824, BRAMY2026899, BRAMY2026902, BRAMY2026904, BRAMY2026976, BRAMY2027073, BRAMY2027140, BRAMY2027242, BRAMY2027396, BRAMY2027451, BRAMY2027461, BRAMY2027467, BRAMY2027714, BRAMY2027717, BRAMY2027752, BRAMY2027782, BRAMY2028072, BRAMY2028183, BRAMY2028188, BRAMY2028269, BRAMY2028282, BRAMY2028472, BRAMY2028491, BRAMY2028565, BRAMY2028593, BRAMY2028783, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859,
BRAMY2030915, BRAMY2031317, BRAMY2031377, BRAMY2031442, BRAMY2032014, BRAMY2032231, BRAMY2032242, BRAMY2032290, BRAMY2032311, BRAMY2032317, BRAMY2032344, BRAMY2032589, BRAMY2032678, BRAMY2032777, BRAMY2032994, BRAMY2033003, BRAMY2033116, BRAMY2033240, BRAMY2033267, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035449, BRAMY2035545, BRAMY2035606, BRAMY2035718, BRAMY2035801, BRAMY2035869, BRAMY2035923, BRAMY2036079, BRAMY2036254, BRAMY2036266, BRAMY2036387, BRAMY2036396, BRAMY2036567, BRAMY2036615, BRAMY2036699, BRAMY2036913, BRAMY2036918, BRAMY2036968, BRAMY2037147,
BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038887, BRAMY2038904, BRAMY2039287, BRAMY2039300, BRAMY2039314, BRAMY2039341, BRAMY2039630, BRAMY2039695, BRAMY2039728, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918,
BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002312, BRAMY3002329, BRAMY3002508, BRAMY3002620, BRAMY3002803, BRAMY3002805, BRAMY3002886, BRAMY3002991, BRAMY3003026, BRAMY3003109,
BRAMY3003566, BRAMY3003723, BRAMY3004126, BRAMY3004224, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009556, BRAMY3009782, BRAMY3009811, BRAMY3009904, BRAMY3010264, BRAMY3010321, BRAMY3010411, BRAMY3010603, BRAMY3010654, BRAMY4000095, BRAMY4000229, BRAMY4000277, BRAMY4000915, BRAMY4000962, BRAMY4001173, BRAMY4001200, BRAMY4001449,
BRAMY4001652, BRAMY4001913, BRAMY4002575, BRASW1000053, BRASW1000125, BRAWH1000002, BRAWH1000094, BRAWH1000116, BRAWH1000130, BRAWH1000157, BRAWH1000164, BRAWH1000168, BRAWH1000180, BRAWH1000369, BRAWH2000034, BRAWH2000048, BRAWH2000071, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000443, BRAWH2000471, BRAWH2000476, BRAWH2000482, BRAWH2000500, BRAWH2000503, BRAWH2000522, BRAWH2000554, BRAWH2000595, BRAWH2000643, BRAWH2000651, BRAWH2000682, BRAWH2000752, BRAWH2000866, BRAWH2000892, BRAWH2000901, BRAWH2000926, BRAWH2000984,
BRAWH2001037, BRAWH2001052, BRAWH2001103, BRAWH2001137, BRAWH2001141, BRAWH2001159, BRAWH2001166, BRAWH2001203, BRAWH2001238, BRAWH2001255, BRAWH2001355, BRAWH2001378, BRAWH2001436, BRAWH2001438, BRAWH2001484, BRAWH2001492, BRAWH2001503, BRAWH2001519, BRAWH2001589, BRAWH2001662, BRAWH2001666, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002125, BRAWH2002191, BRAWH2002318, BRAWH2002333, BRAWH2002352, BRAWH2002383, BRAWH2002549, BRAWH2002560, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963,
BRAWH2002967, BRAWH2002976, BRAWH2003000, BRAWH2003025, BRAWH2003135, BRAWH2003307, BRAWH2003355, BRAWH2003366, BRAWH2003662, BRAWH2003832, BRAWH2003948, BRAWH2003964, BRAWH2004044, BRAWH2004068, BRAWH2004078, BRAWH2004580, BRAWH2004731, BRAWH2004779, BRAWH2004884, BRAWH2004923, BRAWH2005074, BRAWH2005315, BRAWH2005517, BRAWH2005524, BRAWH2005533, BRAWH2005812, BRAWH2005974, BRAWH2005998, BRAWH2006044, BRAWH2006083, BRAWH2006207, BRAWH2006301, BRAWH2006395, BRAWH2006405, BRAWH2006622, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007658, BRAWH2007664, BRAWH2007800, BRAWH2007825, BRAWH2007862,
BRAWH2007890, BRAWH2008128, BRAWH2008219, BRAWH2008255, BRAWH2008292, BRAWH2008297, BRAWH2008366, BRAWH2008380, BRAWH2008577, BRAWH2008790, BRAWH2008903, BRAWH2008993, BRAWH2009112, BRAWH2009227, BRAWH2009261, BRAWH2009263, BRAWH2009298, BRAWH2009393, BRAWH2009485, BRAWH2009490, BRAWH2009558, BRAWH2009590, BRAWH2009626, BRAWH2009695, BRAWH2009907, BRAWH2010000, BRAWH2010069, BRAWH2010084, BRAWH2010136, BRAWH2010318, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011155, BRAWH2011166, BRAWH2011204, BRAWH2011286, BRAWH2011294, BRAWH2011400, BRAWH2011762, BRAWH2011795, BRAWH2011796, BRAWH2011812,
BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013748, BRAWH2013866, BRAWH2013914, BRAWH2013941, BRAWH2013955, BRAWH2014234, BRAWH2014379, BRAWH2014414, BRAWH2014473, BRAWH2014511, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016221, BRAWH2016223,
BRAWH2016269, BRAWH2016305, BRAWH2016439, BRAWH2016446, BRAWH2016562, BRAWH2016655, BRAWH2016679, BRAWH2016702, BRAWH2016724, BRAWH2016785, BRAWH2016792, BRAWH2016800, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017913, BRAWH2018267, BRAWH2018282, BRAWH2018317, BRAWH2018506, BRAWH2018514, BRAWH2018526, BRAWH2018601, BRAWH2018729, BRAWH2018745, BRAWH2018875, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000884, BRAWH3001053, BRAWH3001326, BRAWH3001475,
BRAWH3001636, BRAWH3001638, BRAWH3001712, BRAWH3001760, BRAWH3001783, BRAWH3001833, BRAWH3001879, BRAWH3001891, BRAWH3002433, BRAWH3002467, BRAWH3002513, BRAWH3002574, BRAWH3002600, BRAWH3002819, BRAWH3003019, BRAWH3003136, BRAWH3003244, BRAWH3003343, BRAWH3003522, BRAWH3003598, BRAWH3003801, BRAWH3003975, BRAWH3004222, BRAWH3004335, BRAWH3004453, BRAWH3004666, BRAWH3005037, BRAWH3005047, BRAWH3005132, BRAWH3005292, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007783, BRAWH3008167, BRAWH3008341, BRAWH3008559,
BRAWH3008697, BRAWH3008867, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629,
BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024469, BRAWH3024506, BRAWH3024613, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538,
BRAWH3029556, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035904, BRAWH3035914, BRAWH3036077, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000105, BRCAN1000149, BRCAN1000168, BRCAN2000148, BRCAN2000149, BRCAN2000239, BRCAN2000346,
BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002416, BRCAN2002473, BRCAN2002562, BRCAN2002662, BRCAN2002826, BRCAN2002854, BRCAN2002856, BRCAN2002892, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119,
BRCAN2007144, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015757, BRCAN2015886, BRCAN2016025, BRCAN2016046, BRCAN2016174, BRCAN2016433, BRCAN2016619, BRCAN2016762, BRCAN2016814,
BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019773, BRCAN2019907, BRCAN2020234, BRCAN2020331, BRCAN2020467, BRCAN2020710, BRCAN2020880, BRCAN2021028, BRCAN2021325, BRCAN2021452, BRCAN2021669, BRCAN2021718, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028036,
BRCAN2028338, BRCAN2028378, BRCAN2028460, BRCAN2028545, BRCAN2028634, BRCAN2028702, BRCOC1000087, BRCOC2000047, BRCOC2000152, BRCOC2000184, BRCOC2000333, BRCOC2000360, BRCOC2000404, BRCOC2000637, BRCOC2000670, BRCOC2000816, BRCOC2000850, BRCOC2001355, BRCOC2002085, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003513, BRCOC2003740, BRCOC2004036, BRCOC2004175, BRCOC2004354, BRCOC2004455, BRCOC2004950, BRCOC2005542, BRCOC2005903, BRCOC2005951, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225,
BRCOC2008642, BRCOC2008993, BRCOC2009052, BRCOC2009196, BRCOC2009380, BRCOC2009638, BRCOC2009937, BRCOC2010115, BRCOC2010510, BRCOC2010980, BRCOC2010994, BRCOC2011010, BRCOC2011398, BRCOC2011418, BRCOC2011506, BRCOC2011769, BRCOC2011996, BRCOC2012229, BRCOC2012386, BRCOC2012813, BRCOC2013448, BRCOC2013573, BRCOC2013675, BRCOC2013780, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017856, BRCOC2018775, BRCOC2018877, BRCOC2019255, BRCOC2019549, BRCOC2019841, BRCOC2019896, BRCOC2019934, BRCOC2020122, BRCOC2020142, BRHIP1000058, BRHIP1000072, BRHIP1000129, BRHIP1000174, BRHIP1000258, BRHIP2000021,
BRHIP2000079, BRHIP2000163, BRHIP2000210, BRHIP2000312, BRHIP2000359, BRHIP2000445, BRHIP2000506, BRHIP2000532, BRHIP2000621, BRHIP2000691, BRHIP2000826, BRHIP2000920, BRHIP2001074, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003242, BRHIP2003272, BRHIP2003748, BRHIP2003786, BRHIP2003828, BRHIP2003917, BRHIP2004312, BRHIP2004355, BRHIP2004814, BRHIP2004883, BRHIP2004902, BRHIP2005010, BRHIP2005236, BRHIP2005354, BRHIP2005600, BRHIP2005719, BRHIP2005724, BRHIP2005752, BRHIP2005932, BRHIP2006617, BRHIP2006782,
BRHIP2006800, BRHIP2006819, BRHIP2006921, BRHIP2007307, BRHIP2007616, BRHIP2007690, BRHIP2007928, BRHIP2007969, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2010217, BRHIP2010309, BRHIP2010444, BRHIP2010487, BRHIP2010610, BRHIP2010723, BRHIP2010744, BRHIP2011080, BRHIP2011120, BRHIP2011491, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447,
BRHIP2013510, BRHIP2013699, BRHIP2013723, BRHIP2013958, BRHIP2013972, BRHIP2014063, BRHIP2014228, BRHIP2014285, BRHIP2014291, BRHIP2014387, BRHIP2014954, BRHIP2015224, BRHIP2015356, BRHIP2015360, BRHIP2016005, BRHIP2016125, BRHIP2016788, BRHIP2016968, BRHIP2016990, BRHIP2017315, BRHIP2017385, BRHIP2017404, BRHIP2017542, BRHIP2017553, BRHIP2017642, BRHIP2017714, BRHIP2017780, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019884, BRHIP2019972, BRHIP2020182, BRHIP2020622, BRHIP2020695, BRHIP2020743,
BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022298, BRHIP2022467, BRHIP2022708, BRHIP2023071, BRHIP2023229, BRHIP2023292, BRHIP2023695, BRHIP2023735, BRHIP2023762, BRHIP2023773, BRHIP2023860, BRHIP2023869, BRHIP2024165, BRHIP2024201, BRHIP2024347, BRHIP2024549, BRHIP2024789, BRHIP2024911, BRHIP2024941, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027054, BRHIP2027563,
BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000060, BRHIP3000070, BRHIP3000081, BRHIP3000240, BRHIP3000377, BRHIP3000457, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002124, BRHIP3002363, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193,
BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005768, BRHIP3005801, BRHIP3005944, BRHIP3006279, BRHIP3006390, BRHIP3006449, BRHIP3006683, BRHIP3006717, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008565, BRHIP3008598, BRHIP3008606, BRHIP3008691, BRHIP3008714, BRHIP3008997, BRHIP3009099, BRHIP3009140, BRHIP3009181,
BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013588, BRHIP3013698, BRHIP3013765, BRHIP3013897, BRHIP3014237, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3019643, BRHIP3019824, BRHIP3019880, BRHIP3019956, BRHIP3020155, BRHIP3020182, BRHIP3021019, BRHIP3021499, BRHIP3021534, BRHIP3021778,
BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000175, BRSSN2000293, BRSSN2000312, BRSSN2000518, BRSSN2000561, BRSSN2000566, BRSSN2000715, BRSSN2001213, BRSSN2001275, BRSSN2001342, BRSSN2001426, BRSSN2001503, BRSSN2001758, BRSSN2001810, BRSSN2002857, BRSSN2002859, BRSSN2003086, BRSSN2003093,
BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2006133, BRSSN2006390, BRSSN2006575, BRSSN2006611, BRSSN2006644, BRSSN2007076, BRSSN2007168., BRSSN2007202, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010110, BRSSN2010587, BRSSN2010596, BRSSN2010830, BRSSN2011653, BRSSN2012081, BRSSN2012103, BRSSN2012157, BRSSN2012198, BRSSN2012254, BRSSN2012622, BRSSN2012691, BRSSN2012838, BRSSN2013095, BRSSN2013544, BRSSN2013696, BRSSN2013733,
BRSSN2013753, BRSSN2013874, BRSSN2014112, BRSSN2014294, BRSSN2014424, BRSSN2014513, BRSSN2014556, BRSSN2014573, BRSSN2014636, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018731, BRSSN2018771, BRSSN2018925, BRSTN1000097, BRSTN2000070, BRSTN2000220, BRSTN2000312, BRSTN2000367, BRSTN2000536, BRSTN2000872, BRSTN2001613, BRSTN2001801, BRSTN2002105, BRSTN2002400, BRSTN2002532, BRSTN2002691, BRSTN2003590, BRSTN2003835, BRSTN2004863, BRSTN2004987, BRSTN2005721, BRSTN2006306,
BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012284, BRSTN2012320, BRSTN2012380, BRSTN2013171, BRSTN2013354, BRSTN2014633, BRSTN2014751, BRSTN2014770, BRSTN2015015, BRSTN2016066, BRSTN2016335, BRSTN2016678, BRSTN2016892, BRSTN2016918, BRSTN2017151, BRSTN2017237, BRSTN2017379, BRSTN2017771, BRSTN2017995, BRSTN2018077, BRSTN2018083, BRSTN2018596, BRSTN2018692,
BRSTN2018712, BRSTN2019079, BRSTN2019129, BRTHA1000021, BRTHA1000063, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005,
BRTHA2005448, BRTHA2005579, BRTHA2005831, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2012129, BRTHA2012183,
BRTHA2012189, BRTHA2012351, BRTHA2012392, BRTHA2012466, BRTHA2012555, BRTHA2012562, BRTHA2013262, BRTHA2013426, BRTHA2013460, BRTHA2013515, BRTHA2013595, BRTHA2013610, BRTHA2013707, BRTHA2013942, BRTHA2014287, BRTHA2014334, BRTHA2014381, BRTHA2014578, BRTHA2014647, BRTHA2014792, BRTHA2014828, BRTHA2014909, BRTHA2014975, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2017178, BRTHA2017353, BRTHA2017364, BRTHA2017972, BRTHA2018011, BRTHA2018129, BRTHA2018165, BRTHA2018304, BRTHA2018344, BRTHA2018420, BRTHA2018443, BRTHA2018558, BRTHA2018591,
BRTHA2018624, BRTHA2018706, BRTHA2019014, BRTHA2019022, BRTHA2019048, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874,
BRTHA2035448, BRTHA2036055, BRTHA2036295, BRTHA2036660, BRTHA2037247, BRTHA2038279, BRTHA2038345, BRTHA2038353, BRTHA2038396, BRTHA3000252, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002933, BRTHA3002955, BRTHA3003000, BRTHA3003023, BRTHA3003074, BRTHA3003225, BRTHA3003339, BRTHA3003343, BRTHA3003449, BRTHA3003474, BRTHA3003704, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685,
BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010540, BRTHA3010717, BRTHA3011149, BRTHA3011187, BRTHA3011229, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514,
BRTHA3018617, BRTHA3018623, BRTHA3018656, BRTHA3019048, BRTHA3019105, BRTHA3019183, BRTHA3020000, BRTHA3020369, BRTHA3021569, BRTHA3021708, BRTHA3021786, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058,
CHONS2000172, CHONS2001287, CHONS2001382, CHONS2001797, CHONS2001834, CHONS2002419, COLON1000030, COLON1000084, COLON1000135, COLON2000470, COLON2000557, COLON2000568, COLON2001721, COLON2001829, COLON2001866, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000020, CTONG1000022, CTONG1000040, CTONG1000056, CTONG1000062, CTONG1000086, CTONG1000088, CTONG1000093, CTONG1000097, CTONG1000113, CTONG1000137, CTONG1000165,
CTONG1000180, CTONG1000181, CTONG1000220, CTONG1000277, CTONG1000283, CTONG1000302, CTONG1000341, CTONG1000476, CTONG1000488, CTONG1000508, CTONG1000540, CTONG2000031, CTONG2000034, CTONG2000042, CTONG2000218, CTONG2000279, CTONG2000303, CTONG2000330, CTONG200.0395, CTONG2000452, CTONG2000480, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001413, CTONG2001513, CTONG2001524, CTONG2001533, CTONG2001598, CTONG2001681, CTONG2001724, CTONG2001749, CTONG2001800, CTONG2001820, CTONG2001877, CTONG2001911, CTONG2001932, CTONG2001955, CTONG2002066, CTONG2002095, CTONG2002143, CTONG2002417,
CTONG2002418, CTONG2002453, CTONG2002520, CTONG2002558, CTONG2002590, CTONG2002709, CTONG2002766, CTONG2002816, CTONG2002832, CTONG2002841, CTONG2002965, CTONG2002999, CTONG2003028, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004454, CTONG2004550, CTONG2004669, CTONG2004716, CTONG2004912, CTONG2004941, CTONG2004948, CTONG2005049, CTONG2005110, CTONG2005145, CTONG2005265,
CTONG2005290, CTONG2005374, CTONG2005399, CTONG2005567, CTONG2005585, CTONG2005635, CTONG2005775, CTONG2005795, CTONG2005890, CTONG2005904, CTONG2005913, CTONG2006129, CTONG2006223, CTONG2006273, CTONG2006310, CTONG2006393, CTONG2006412, CTONG2006449, CTONG2006515, CTONG2006524, CTONG2006568, CTONG2006611, CTONG2006620, CTONG2006691, CTONG2006836, CTONG2006942, CTONG2006964, CTONG2007009, CTONG2007091, CTONG2007104, CTONG2007175, CTONG2007259, CTONG2007272, CTONG2007293, CTONG2007297, CTONG2007399, CTONG2007474, CTONG2007613, CTONG2007623, CTONG2007681, CTONG2007779, CTONG2007898, CTONG2007959, CTONG2007970, CTONG2008014, CTONG2008184, CTONG2008262, CTONG2008269, CTONG2008321, CTONG2008343,
CTONG2008402, CTONG2008405, CTONG2008518, CTONG2008521, CTONG2008562, CTONG2008577, CTONG2008595, CTONG2008689, CTONG2008721, CTONG2008773, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009570, CTONG2009572, CTONG2009604, CTONG2009643, CTONG2009675, CTONG2009689, CTONG2009725, CTONG2009766, CTONG2009768, CTONG2009871, CTONG2009887, CTONG2009891, CTONG2009923, CTONG2009955, CTONG2009958, CTONG2010018, CTONG2010024, CTONG2010116, CTONG2010133, CTONG2010148, CTONG2010276, CTONG2010330, CTONG2010508, CTONG2010566,
CTONG2010623, CTONG2010803, CTONG2011188, CTONG2011429, CTONG2011474, CTONG2011676, CTONG2011716, CTONG2011765, CTONG2011779, CTONG2011815, CTONG2011822, CTONG2011920, CTONG2011985, CTONG2012029, CTONG2012077, CTONG2012101, CTONG2012123, CTONG2012158, CTONG2012230, CTONG2012425, CTONG2012447, CTONG2012473, CTONG2012476, CTONG2012533, CTONG2012554, CTONG2012564, CTONG2012724, CTONG2012738, CTONG2012758, CTONG2012843, CTONG2012879, CTONG2012901, CTONG2013128, CTONG2013149, CTONG2013156, CTONG2013178, CTONG2013222, CTONG2013284, CTONG2013339, CTONG2013352, CTONG2013381, CTONG2013393, CTONG2013511, CTONG2013630, CTONG2013803, CTONG2013985, CTONG2013986, CTONG2014032, CTONG2014058, CTONG2014191,
CTONG2014206, CTONG2014234, CTONG2014264, CTONG2014369, CTONG2014630, CTONG2014635, CTONG2014646, CTONG2014705, CTONG2014946, CTONG2014959, CTONG2014966, CTONG2014995, CTONG2015204, CTONG2015316, CTONG2015330, CTONG2015358, CTONG2015434, CTONG2015518, CTONG2015540, CTONG2015596, CTONG2015633, CTONG2015717, CTONG2015804, CTONG2015815, CTONG2015866, CTONG2015914, CTONG2015953, CTONG2016217, CTONG2016355, CTONG2016408, CTONG2016499, CTONG2016559, CTONG2016658, CTONG2016775, CTONG2016797, CTONG2016824, CTONG2016846, CTONG2016904, CTONG2016942, CTONG2016953, CTONG2017021, CTONG2017094, CTONG2017292, CTONG2017409, CTONG2017444, CTONG2017458, CTONG2017604, CTONG2017650, CTONG2017804, CTONG2017814,
CTONG2017910, CTONG2017939, CTONG2017989, CTONG2018062, CTONG2018069, CTONG2018147, CTONG2018217, CTONG2018279, CTONG2018343, CTONG2018379, CTONG2018383, CTONG2018413, CTONG2018483, CTONG2018632, CTONG2018652, CTONG2018653, CTONG2018655, CTONG2018808, CTONG2018900, CTONG2019063, CTONG2019144, CTONG2019232, CTONG2019400, CTONG2019652, CTONG2019833, CTONG2020031, CTONG2020127, CTONG2020171, CTONG2020374, CTONG2020411, CTONG2020445, CTONG2020522, CTONG2020806, CTONG2020831, CTONG2020974, CTONG2021132, CTONG2021168, CTONG2022153, CTONG2022601, CTONG2023021, CTONG2023449, CTONG2023512, CTONG2024206, CTONG2024614, CTONG2024749, CTONG2025496, CTONG2025513, CTONG2025516, CTONG2025610, CTONG2025900,
CTONG2026513, CTONG2026770, CTONG2026920, CTONG2026987, CTONG2027150, CTONG2027263, CTONG2027361, CTONG2027591, CTONG2027736, CTONG2027783, CTONG2027828, CTONG2027959, CTONG2028097, CTONG2028208, CTONG2028486, CTONG2028518, CTONG2028687, CTONG3000686, CTONG3000707, CTONG3000896, CTONG3001420, CTONG3001501, CTONG3001560, CTONG3001605, CTONG3002020, CTONG3002370, CTONG3002412, CTONG3002433, CTONG3002552, CTONG3002588, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003972, CTONG3004317, CTONG3004397, CTONG3004550, CTONG3004607, CTONG3004712, CTONG3004726,
CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009239, CTONG3009287, CTONG3009328, CTONG3009385, D3OST1000066, D3OST1000109, D3OST1000238, D3OST1000267, D3OST1000270, D3OST2000184, D3OST2000618, D3OST2000654, D3OST2001328, D3OST2001669, D3OST2002182, D3OST2002223, D3OST2002262, D3OST2002272, D3OST2002417, D3OST2002436, D3OST2002648, D3OST2003024, D3OST2003331,
D3OST2003607, D3OST2003797, D3OST2004637, D3OST3000169, D3OST3000195, D3OST3000388, D3OST3000493, D6OST2000127, D6OST2000169, D6OST2000245, D6OST2000507, D9OST2000031, D9OST2000278, D9OST2000440, D9OST2000869, D9OST2001319, D9OST2001433, D9OST2002397, D9OST2002673, D9OST2003106, D9OST2003108, D9OST2003137, D9OST2003397, D9OST2003580, D9OST2003594, D9OST2003762, D9OST2003989, D9OST2004018, D9OST2004073, D9OST2004417, DFNES1000003, DFNES1000107, DFNES1000185, DFNES2000143, DFNES2000146, DFNES2000153, DFNES2000212, DFNES2000268, DFNES2000292, DFNES2000335, DFNES2000432, DFNES2000457, DFNES2000471, DFNES2000913, DFNES2001091, DFNES2001096, DFNES2001108, DFNES2001177, DFNES2001564, DFNES2001565,
DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2004354, DFNES2004383, DFNES2004608, DFNES2004684, DFNES2005266, DFNES2005527, DFNES2005779, DFNES2005875, DFNES2006944, DFNES2007299, DFNES2007641, DFNES2007757, DFNES2008280, DFNES2008881, DFNES2010502, DFNES2011192, DFNES2011239, DFNES2011245, DFNES2011380, ERLTF1000021, ERLTF2000324, ERLTF2000465, FCBBF1000023, FCBBF1000069, FCBBF1000117, FCBBF1000118, FCBBF1000121, FCBBF1000125, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232,
FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000437, FCBBF1000449, FCBBF1000476, FCBBF1000506, FCBBF1000546, FCBBF1000550, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000691, FCBBF1000732, FCBBF1000748, FCBBF2000038, FCBBF2000094, FCBBF2000105, FCBBF2000123, FCBBF2000195, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566,
FCBBF2000576, FCBBF2000591, FCBBF2000677, FCBBF2000678, FCBBF2000685, FCBBF2000733, FCBBF2000808, FCBBF2000815, FCBBF2000825, FCBBF2000940, FCBBF2000951, FCBBF2001001, FCBBF2001105, FCBBF2001116, FCBBF2001188, FCBBF2001211, FCBBF2001238, FCBBF2001243, FCBBF2001299, FCBBF2001427, FCBBF2001480, FCBBF2001529, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370, FCBBF2002541, FCBBF2002626, FCBBF2002897, FCBBF2002898, FCBBF2002904, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636,
FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007610, FCBBF2007633, FCBBF2007840, FCBBF3000102, FCBBF3000120, FCBBF3000168, FCBBF3000175, FCBBF3000227, FCBBF3000229, FCBBF3000233, FCBBF3000269, FCBBF3000361, FCBBF3000367, FCBBF3000388, FCBBF3000462, FCBBF3000473, FCBBF3000501, FCBBF3000518,
FCBBF3000550, FCBBF3000847, FCBBF3001018, FCBBF3001020, FCBBF3001235, FCBBF3001281, FCBBF3001377, FCBBF3001594, FCBBF3001632, FCBBF3001657, FCBBF3001855, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004502, FCBBF3004842, FCBBF3004847, FCBBF3004955, FCBBF3005160, FCBBF3005218, FCBBF3005444, FCBBF3005698,
FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008159, FCBBF3008251, FCBBF3008311, FCBBF3008362, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012,
FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010729, FCBBF3010733, FCBBF3011003, FCBBF3011418, FCBBF3011485, FCBBF3011570, FCBBF3011592, FCBBF3011867, FCBBF3011889, FCBBF3012170, FCBBF3012182, FCBBF3012288, FCBBF3012332, FCBBF3012443, FCBBF3012588, FCBBF3012667, FCBBF3012842, FCBBF3012901, FCBBF3013041, FCBBF3013058, FCBBF3013205, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809,
FCBBF3015870, FCBBF3016018, FCBBF3016134, FCBBF3016178, FCBBF3016451, FCBBF3016618, FCBBF3016622, FCBBF3016644, FCBBF3016987, FCBBF3017059, FCBBF3017071, FCBBF3017123, FCBBF3017233, FCBBF3017255, FCBBF3017288, FCBBF3017396, FCBBF3017535, FCBBF3017681, FCBBF3017729, FCBBF3017873, FCBBF3017974, FCBBF3018067, FCBBF3018173, FCBBF3018453, FCBBF3018591, FCBBF3018753, FCBBF3018826, FCBBF3018949, FCBBF3019085, FCBBF3019437, FCBBF3019455, FCBBF3019564, FCBBF3019569, FCBBF3019570, FCBBF3019663, FCBBF3019714, FCBBF3019716, FCBBF3019784, FCBBF3019867, FCBBF3019961, FCBBF3020030, FCBBF3020138, FCBBF3020163, FCBBF3020232, FCBBF3020599, FCBBF3021026, FCBBF3021191, FCBBF3021221, FCBBF3021501, FCBBF3021506,
FCBBF3021524, FCBBF3021807, FCBBF3021940, FCBBF3021985, FCBBF3022005, FCBBF3022291, FCBBF3022311, FCBBF3022566, FCBBF3022765, FCBBF3022894, FCBBF3023061, FCBBF3023078, FCBBF3023372, FCBBF3023443, FCBBF3023599, FCBBF3023704, FCBBF3023887, FCBBF3023895, FCBBF3024096, FCBBF3024225, FCBBF3024266, FCBBF3024364, FCBBF3024623, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3026021, FCBBF3026048, FCBBF3026251, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102,
FCBBF3028143, FCBBF3028188, FCBBF3028472, FCBBF3028528, FCBBF3028582, FCBBF3028671, FCBBF3028794, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF5000041, FCBBF5000061, FCBBF5000165, FCBBF5000325, FCBBF5000353, FCBBF5000384, FCBBF5000483, FCBBF5000495, FEBRA1000005, FEBRA1000008, FEBRA1000022, FEBRA1000030, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000220, FEBRA2000282, FEBRA2000286, FEBRA2000297, FEBRA2000311, FEBRA2000321,
FEBRA2000333, FEBRA2000378, FEBRA2000391, FEBRA2000394, FEBRA2000397, FEBRA2000399, FEBRA2000404, FEBRA2000409, FEBRA2000415, FEBRA2000436, FEBRA2000462, FEBRA2000468, FEBRA2000491, FEBRA2000504, FEBRA2000538, FEBRA2000575, FEBRA2000581, FEBRA2000656, FEBRA2000659, FEBRA2000680, FEBRA2000690, FEBRA2000727, FEBRA2000734, FEBRA2000762, FEBRA2000771, FEBRA2000772, FEBRA2000782, FEBRA2000790, FEBRA2000793, FEBRA2000803, FEBRA2000805, FEBRA2000809, FEBRA2000856, FEBRA2000862, FEBRA2000877, FEBRA2000880, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133,
FEBRA2001161, FEBRA2001175, FEBRA2001197, FEBRA2001217, FEBRA2001245, FEBRA2001267, FEBRA2001334, FEBRA2001351, FEBRA2001383, FEBRA2001438, FEBRA2001440, FEBRA2001487, FEBRA2001561, FEBRA2001584, FEBRA2001590, FEBRA2001591, FEBRA2001610, FEBRA2001620, FEBRA2001659, FEBRA2001705, FEBRA2001706, FEBRA2001752, FEBRA2001772, FEBRA2001790, FEBRA2001914, FEBRA2002009, FEBRA2002027, FEBRA2002086, FEBRA2002109, FEBRA2002191, FEBRA2002260, FEBRA2002399, FEBRA2002410, FEBRA2002442, FEBRA2002508, FEBRA2002525, FEBRA2002527, FEBRA2002552, FEBRA2002611, FEBRA2002628, FEBRA2002662, FEBRA2002682, FEBRA2002727, FEBRA2002781, FEBRA2002783, FEBRA2002818, FEBRA2002858, FEBRA2002882, FEBRA2002897, FEBRA2002908,
FEBRA2002931, FEBRA2002962, FEBRA2002986, FEBRA2003054, FEBRA2003100, FEBRA2003128, FEBRA2003155, FEBRA2003163, FEBRA2003198, FEBRA2003253, FEBRA2003275, FEBRA2003308, FEBRA2003327, FEBRA2003429, FEBRA2003454, FEBRA2003468, FEBRA2003470, FEBRA2003520, FEBRA2003524, FEBRA2003541, FEBRA2003675, FEBRA2003707, FEBRA2003711, FEBRA2003822, FEBRA2003897, FEBRA2003907, FEBRA2004023, FEBRA2004026, FEBRA2004029, FEBRA2004053, FEBRA2004056, FEBRA2004091, FEBRA2004110, FEBRA2004191, FEBRA2004202, FEBRA2004219, FEBRA2004224, FEBRA2004237, FEBRA2004268, FEBRA2004325, FEBRA2004329, FEBRA2004412, FEBRA2004443, FEBRA2004490, FEBRA2004538, FEBRA2004592, FEBRA2004620, FEBRA2004651, FEBRA2004767, FEBRA2004813,
FEBRA2004840, FEBRA2004852, FEBRA2004867, FEBRA2004914, FEBRA2004933, FEBRA2004987, FEBRA2005014, FEBRA2005036, FEBRA2005079, FEBRA2005216, FEBRA2005377, FEBRA2005380, FEBRA2005427, FEBRA2005458, FEBRA2005701, FEBRA2005726, FEBRA2005752, FEBRA2005788, FEBRA2005998, FEBRA2006055, FEBRA2006061, FEBRA2006112, FEBRA2006150, FEBRA2006165, FEBRA2006315, FEBRA2006346, FEBRA2006354, FEBRA2006370, FEBRA2006379, FEBRA2006396, FEBRA2006409, FEBRA2006418, FEBRA2006427, FEBRA2006476, FEBRA2006519, FEBRA2006521, FEBRA2006627, FEBRA2006664, FEBRA2006667, FEBRA2006726, FEBRA2006736, FEBRA2006793, FEBRA2006817, FEBRA2006890, FEBRA2006942, FEBRA2006977, FEBRA2007017, FEBRA2007176, FEBRA2007200, FEBRA2007247,
FEBRA2007280, FEBRA2007414, FEBRA2007436, FEBRA2007449, FEBRA2007458, FEBRA2007534, FEBRA2007551, FEBRA2007566, FEBRA2007589, FEBRA2007620, FEBRA2007708, FEBRA2007714, FEBRA2007793, FEBRA2007818, FEBRA2007823, FEBRA2007931, FEBRA2007957, FEBRA2008009, FEBRA2008086, FEBRA2008134, FEBRA2008135, FEBRA2008159, FEBRA2008165, FEBRA2008172, FEBRA2008210, FEBRA2008255, FEBRA2008265, FEBRA2008282, FEBRA2008311, FEBRA2008360, FEBRA2008459, FEBRA2008468, FEBRA2008475, FEBRA2008481, FEBRA2008542, FEBRA2008660, FEBRA2008681, FEBRA2008741, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117,
FEBRA2009120, FEBRA2009162, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009541, FEBRA2009567, FEBRA2009588, FEBRA2009731, FEBRA2009754, FEBRA2009846, FEBRA2010141, FEBRA2010299, FEBRA2010484, FEBRA2010719, FEBRA2010746, FEBRA2010768, FEBRA2010802, FEBRA2011063, FEBRA2011356, FEBRA2011392, FEBRA2011414, FEBRA2011593, FEBRA2011665, FEBRA2012090, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013905, FEBRA2013951, FEBRA2014122, FEBRA2014198, FEBRA2014443,
FEBRA2014939, FEBRA2015076, FEBRA2015175, FEBRA2015292, FEBRA2015588, FEBRA2016112, FEBRA2016398, FEBRA2016583, FEBRA2016621, FEBRA2016654, FEBRA2016739, FEBRA2017098, FEBRA2017138, FEBRA2017154, FEBRA2017200, FEBRA2017223, FEBRA2017333, FEBRA2017502, FEBRA2017533, FEBRA2017736, FEBRA2017780, FEBRA2017811, FEBRA2017850, FEBRA2018051, FEBRA2018203, FEBRA2018407, FEBRA2018433, FEBRA2019172, FEBRA2019298, FEBRA2019389, FEBRA2019457, FEBRA2019519, FEBRA2019582, FEBRA2019637, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021134, FEBRA2021171, FEBRA2021339, FEBRA2021431, FEBRA2021497, FEBRA2021550,
FEBRA2021571, FEBRA2021636, FEBRA2021864, FEBRA2021892, FEBRA2021908, FEBRA2021966, FEBRA2022013, FEBRA2022055, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023285, FEBRA2023351, FEBRA2023377, FEBRA2023399, FEBRA2023498, FEBRA2023550, FEBRA2023721, FEBRA2023989, FEBRA2023990, FEBRA2024150, FEBRA2024343, FEBRA2024570, FEBRA2024693, FEBRA2024727, FEBRA2024744, FEBRA2024797, FEBRA2024987, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297,
FEBRA2027352, FEBRA2027364, FEBRA2027609, FEBRA2028222, FEBRA2028366, FEBRA2028457, FEBRA2028477, FEBRA2028503, FEHRT2000325, FEHRT2000364, FELNG2000241, HCASM1000021, HCASM1000050, HCASM1000061, HCASM1000130, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003076, HCASM2003099, HCASM2003212, HCASM2003237, HCASM2003357, HCASM2003415, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405,
HCASM2009424, HCASM2009463, HCASM2009588, HCHON1000015, HCHON1000131, HCHON1000142, HCHON1000176, HCHON1000191, HCHON2000038, HCHON2000100, HCHON2000156, HCHON2000199, HCHON2000212, HCHON2000244, HCHON2000271, HCHON2000315, HCHON2000322, HCHON2000344, HCHON2000373, HCHON2000395, HCHON2000417, HCHON2000418, HCHON2000475, HCHON2000503, HCHON2000660, HCHON2000663, HCHON2000673, HCHON2000676, HCHON2000705, HCHON2000815, HCHON2000817, HCHON2000818, HCHON2000819, HCHON2000851, HCHON2000898, HCHON2000935, HCHON2000942, HCHON2000956, HCHON2000994, HCHON2001050, HCHON2001099, HCHON2001108, HCHON2001110, HCHON2001116, HCHON2001161, HCHON2001214, HCHON2001233, HCHON2001269, HCHON2001407, HCHON2001450,
HCHON2001453, HCHON2001497, HCHON2001505, HCHON2001519, HCHON2001523, HCHON2001535, HCHON2001546, HCHON2001548, HCHON2001577, HCHON2001598, HCHON2001604, HCHON2001607, HCHON2001646, HCHON2001768, HCHON2001801, HCHON2002123, HCHON2002194, HCHON2002241, HCHON2002496, HCHON2002676, HCHON2002770, HCHON2002964, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005921, HCHON2006714, HCHON2006722, HCHON2006770, HCHON2006786, HCHON2006841, HCHON2006871, HCHON2007046, HCHON2007482, HCHON2007674,
HCHON2007774, HCHON2007881, HCHON2008112, HCHON2008239, HCHON2008444, HCHON2008582, HCHON2008672, HCHON2008871, HCHON2008989, HCHON2009191, HCHON2009384, HCHON2009749, HCHON2009766, HCHON2009795, HCHON2010074, HCHON2010543, HEART1000010, HEART1000088., HEART1000099, HEART1000102, HEART1000118, HEART1000132, HEART1000139, HEART1000142, HEART1000151, HEART1000173, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000520, HEART2000541, HEART2000568, HEART2000611, HEART2000959, HEART2001680, HEART2001756, HEART2002129, HEART2002184, HEART2002220, HEART2002432, HEART2002531,
HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006310, HEART2006334, HEART2006487, HEART2006557, HEART2006787, HEART2006789, HEART2006909, HEART2007031, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000114, HHDPC1000118, HHDPC2000102, HHDPC2000115, HHDPC2000258,
HHDPC2000315, HHDPC2000462, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005794, HHDPC2006460, HHDPC2006862, HHDPC2006927, HHDPC2007302, HHDPC2007775, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009528, HLUNG1000017, HLUNG1000024, HLUNG1000030, HLUNG1000034, HLUNG1000053, HLUNG1000064, HLUNG1000084, HLUNG1000091, HLUNG1000104, HLUNG1000105, HLUNG1000110, HLUNG1000151, HLUNG1000169, HLUNG2000014, HLUNG2000027, HLUNG2000068, HLUNG2000116, HLUNG2000130,
HLUNG2000142, HLUNG2000174, HLUNG2000217, HLUNG2000232, HLUNG2000255, HLUNG2000281, HLUNG2000314, HLUNG2000315, HLUNG2000340, HLUNG2000362, HLUNG2000363, HLUNG2000431, HLUNG2000480, HLUNG2000489, HLUNG2000501, HLUNG2000549, HLUNG2000557, HLUNG2000570, HLUNG2000761, HLUNG2000777, HLUNG2000870, HLUNG2000926, HLUNG2000955, HLUNG2000970, HLUNG2001013, HLUNG2001126, HLUNG2001144, HLUNG2001214, HLUNG2001459, HLUNG2001507, HLUNG2001814, HLUNG2001996, HLUNG2002006, HLUNG2002050, HLUNG2002085, HLUNG2002145, HLUNG2002295, HLUNG2002303, HLUNG2002334, HLUNG2002405, HLUNG2002429, HLUNG2002562, HLUNG2002659, HLUNG2002707, HLUNG2002811, HLUNG2002916, HLUNG2002942, HLUNG2002949, HLUNG2002958, HLUNG2002982,
HLUNG2003049, HLUNG2003061, HLUNG2003162, HLUNG2003246, HLUNG2003331, HLUNG2003335, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004170, HLUNG2004217, HLUNG2004273, HLUNG2004388, HLUNG2004416, HLUNG2004521, HLUNG2004684, HLUNG2004707, HLUNG2004774, HLUNG2004775, HLUNG2005012, HLUNG2005030, HLUNG2005045, HLUNG2005076, HLUNG2005133, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006397, HLUNG2006599, HLUNG2006614, HLUNG2006671, HLUNG2006781, HLUNG2006843,
HLUNG2006852, HLUNG2006935, HLUNG2007041, HLUNG2007163, HLUNG2007210, HLUNG2007245, HLUNG2007433, HLUNG2007931, HLUNG2008066, HLUNG2008139, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008833, HLUNG2008875, HLUNG2008885, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622,
HLUNG2013643, HLUNG2013784, HLUNG2013851, HLUNG2014262, HLUNG2014288, HLUNG2014449, HLUNG2015548, HLUNG2015578, HLUNG2015617, HLUNG2016022, HLUNG2016862, HLUNG2017286, HLUNG2017307, HLUNG2017350, HLUNG2017806, HLUNG2018029, HLUNG2018282, HLUNG2018626, HLUNG2019058, HSYRA1000131, HSYRA1000137, HSYRA1000148, HSYRA2000135, HSYRA2000137, HSYRA2000190, HSYRA2000221, HSYRA2000237, HSYRA2000332, HSYRA2000371, HSYRA2000507, HSYRA2000510, HSYRA2000605, HSYRA2000706, HSYRA2000760, HSYRA2000792, HSYRA2000832, HSYRA2000927, HSYRA2001003, HSYRA2001142, HSYRA2001153, HSYRA2001232, HSYRA2001255, HSYRA2001332, HSYRA2001484, HSYRA2001552, HSYRA2001574, HSYRA2001580, HSYRA2001615, HSYRA2001631, HSYRA2002103,
HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000158, IMR321000165, IMR321000193, IMR321000207, IMR321000230, IMR321000242, IMR322000010, IMR322000072, IMR322000107, IMR322000144, IMR322000152, IMR322000164, IMR322000302, IMR322000379, IMR322000383, IMR322000418, IMR322000518, IMR322000555, IMR322000604, IMR322000609, IMR322000656, IMR322000672, IMR322000742, IMR322000775, IMR322000791, IMR322000811, IMR322000819, IMR322000838, IMR322000859, IMR322000863, IMR322000917,
IMR322000919, IMR322000953, IMR322000984, IMR322001018, IMR322001049, IMR322001167, IMR322001185, IMR322001218, IMR322001245, IMR322001317, IMR322001318, IMR322001322, IMR322001332, IMR322001380, IMR322001438, IMR322001452, IMR322001491, IMR322001534, IMR322001665, IMR322001670, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731,
IMR322015523, IMR322016146, IMR322017049, IMR322018117, IMR322018192, KIDNE1000008, KIDNE1000012, KIDNE1000015, KIDNE1000028, KIDNE1000036, KIDNE1000064, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000375, KIDNE2000394, KIDNE2000422, KIDNE2000493, KIDNE2000497, KIDNE2000510, KIDNE2000513, KIDNE2000519, KIDNE2000555, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833,
KIDNE2000846, KIDNE2000867, KIDNE2000909, KIDNE2001117, KIDNE2001140, KIDNE2001269, KIDNE2001373, KIDNE2001802, KIDNE2001840, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002265, KIDNE2002438, KIDNE2002483, KIDNE2002768, KIDNE2002795, KIDNE2002798, KIDNE2002839, KIDNE2002845, KIDNE2002872, KIDNE2002882, KIDNE2002980, KIDNE2002991, KIDNE2003185, KIDNE2003188, KIDNE2003233, KIDNE2003335, KIDNE2003377, KIDNE2003507, KIDNE2003752, KIDNE2003764, KIDNE2003837, KIDNE2004034, KIDNE2004054, KIDNE2004084, KIDNE2004095, KIDNE2004115, KIDNE2004233, KIDNE2004262, KIDNE2004279, KIDNE2004294,
KIDNE2004305, KIDNE2004394, KIDNE2004519, KIDNE2004520, KIDNE2004562, KIDNE2004579, KIDNE2004626, KIDNE2004681, KIDNE2004828, KIDNE2004846, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006376, KIDNE2006378, KIDNE2006580, KIDNE2006687, KIDNE2006733, KIDNE2006760,
KIDNE2006820, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007910, KIDNE2007944, KIDNE2008022, KIDNE2008069, KIDNE2008072, KIDNE2008116, KIDNE2008117, KIDNE2008218, KIDNE2008315, KIDNE2008362, KIDNE2008378, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2009019, KIDNE2009109, KIDNE2009180, KIDNE2009191, KIDNE2009332, KIDNE2009426, KIDNE2009467, KIDNE2009490, KIDNE2009553, KIDNE2009628, KIDNE2009647, KIDNE2009676, KIDNE2009723,
KIDNE2010007, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012745, KIDNE2012775, KIDNE2012784, KIDNE2012945, KIDNE2013045, KIDNE2013095, KIDNE2013218, KIDNE2013263, KIDNE2013346, KIDNE2013388, KIDNE2013413, KIDNE2013489, KIDNE2013639, KIDNE2013731, KIDNE2013734,
KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014415, KIDNE2014489, KIDNE2014496, KIDNE2014661, KIDNE2014717, KIDNE2014789, KIDNE2014890, KIDNE2014978, KIDNE2015073, KIDNE2015221, KIDNE2015244, KIDNE2015313, KIDNE2015433, KIDNE2015483, KIDNE2015515, KIDNE2015556, KIDNE2015660, KIDNE2015710, KIDNE2015987, KIDNE2016000, KIDNE2016036, KIDNE2016096, KIDNE2016101, KIDNE2016142, KIDNE2016188, KIDNE2016388, KIDNE2016464, KIDNE2016539, KIDNE2016570, KIDNE2016918, KIDNE2017007, KIDNE2017151, KIDNE2017153, KIDNE2017315, KIDNE2017332, KIDNE2017343, KIDNE2017454, KIDNE2017603, KIDNE2017956,
KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018617, KIDNE2018678, KIDNE2018727, KIDNE2018863, KIDNE2018989, KIDNE2018996, KIDNE2019074, KIDNE2019187, LIVER1000050, LIVER1000055, LIVER1000058, LIVER1000063, LIVER1000073, LIVER1000082, LIVER1000099, LIVER1000126, LIVER1000139, LIVER1000155, LIVER1000165, LIVER1000175, LIVER1000224, LIVER1000278, LIVER1000397, LIVER1000421, LIVER1000433, LIVER1000479, LIVER1000482, LIVER1000542, LIVER2000003, LIVER2000033, LIVER2000158, LIVER2000216, LIVER2000237, LIVER2000247, LIVER2000360, LIVER2000416, LIVER2000446, LIVER2000515, LIVER2000592, LIVER2000626,
LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001835, LIVER2002638, LIVER2002644, LIVER2002660, LIVER2002702, LIVER2003008, LIVER2003234, LIVER2003511, LIVER2003524, LIVER2003568, LIVER2003581, LIVER2003800, LIVER2003854, LIVER2004074, LIVER2004392, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005973, LIVER2005981, LIVER2006251, LIVER2006410, LIVER2006469, LIVER2006644, LIVER2006764, LIVER2007415, LIVER2007548, LIVER2007568,
LIVER2007721, LIVER2008053, LIVER2008465, LIVER2008473, LIVER2008580, LIVER2008751, LIVER2008945, LIVER2008998, LIVER2009110, LIVER2009118, LIVER2009554, LYMPB1000141, LYMPB1000158, LYMPB2000083, MAMGL1000096, MAMGL1000100, MAMGL1000132, MAMGL1000151, MAMGL1000182, MAMGL1000184, MESAN1000032, MESAN1000035, MESAN1000080, MESAN1000121, MESAN1000147, MESAN1000180, MESAN2000023, MESAN2000026, MESAN2000067, MESAN2000167, MESAN2000264, MESAN2000291, MESAN2000337, MESAN2000434, MESAN2000457, MESAN2000462, MESAN2000501, MESAN2000620, MESAN2000711, MESAN2000894, MESAN2000909, MESAN2001154, MESAN2001770, MESAN2001979, MESAN2001980, MESAN2002113, MESAN2002122, MESAN2002147, MESAN2002186, MESAN2002424,
MESAN2002519, MESAN2002687, MESAN2002709, MESAN2002844, MESAN2002978, MESAN2003037, MESAN2003039, MESAN2003101, MESAN2003322, MESAN2003490, MESAN2003529, MESAN2003646, MESAN2003662, MESAN2003709, MESAN2003851, MESAN2004138, MESAN2004288, MESAN2004575, MESAN2005371, MESAN2005724, MESAN2005808, MESAN2005811, MESAN2006022, MESAN2006043, MESAN2006328, MESAN2006580, MESAN2006599, MESAN2006743, MESAN2006953, MESAN2006971, MESAN2007032, MESAN2007440, MESAN2007597, MESAN2008222, MESAN2008334, MESAN2008415, MESAN2008460, MESAN2008536, MESAN2008679, MESAN2008729, MESAN2008821, MESAN2009019, MESAN2009081, MESAN2009105, MESAN2009311, MESAN2009418, MESAN2009503, MESAN2009522, MESAN2010031, MESAN2010114,
MESAN2010312, MESAN2010321, MESAN2010681, MESAN2011545, MESAN2011597, MESAN2011627, MESAN2011632, MESAN2011977, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013936, MESAN2014295, MESAN2014298, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016965, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, NB9N41000011, NB9N41000047, NB9N41000135,
NB9N41000142, NB9N41000146, NB9N42000122, NB9N42000196, NB9N42000281, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000744, NESOP2001144, NESOP2001433, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000187, NHNPC2000212, NHNPC2000606, NHNPC2000877, NHNPC2001108, NHNPC2001223, NHNPC2001312, NHNPC2001816, NHNPC2001931, NHNPC2002565, NHNPC2002749, NOVAR1000102, NOVAR2000136,
NOVAR2000352, NOVAR2000412, NOVAR2000783, NOVAR2000962, NOVAR2001108, NOVAR2001620, NOVAR2001783, NOVAR2001876, NT2NE1000004, NT2NE1000018, NT2NE1000023, NT2NE1000024, NT2NE1000059, NT2NE1000063, NT2NE1000073, NT2NE1000083, NT2NE1000120, NT2NE1000175, NT2NE1000185, NT2NE2000038, NT2NE2000054, NT2NE2000057, NT2NE2000064, NT2NE2000109, NT2NE2000137, NT2NE2000156, NT2NE2000222, NT2NE2000259, NT2NE2000284, NT2NE2000299, NT2NE2000327, NT2NE2000353, NT2NE2000369, NT2NE2000374, NT2NE2000386, NT2NE2000390, NT2NE2000455, NT2NE2000488, NT2NE2000517, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000864,
NT2NE2000913, NT2NE2000951, NT2NE2000980, NT2NE2001005, NT2NE2001021, NT2NE2001040, NT2NE2001049, NT2NE2001142, NT2NE2001156, NT2NE2001176, NT2NE2001180, NT2NE2001181, NT2NE2001188, NT2NE2001191, NT2NE2001247, NT2NE2001252, NT2NE2001324, NT2NE2001337, NT2NE2001364, NT2NE2001372, NT2NE2001397, NT2NE2001428, NT2NE2001432, NT2NE2001442, NT2NE2001524, NT2NE2001530, NT2NE2001598, NT2NE2001623, NT2NE2001626, NT2NE2001634, NT2NE2001666, NT2NE2001677, NT2NE2001697, NT2NE2001698, NT2NE2001723, NT2NE2001725, NT2NE2001874, NT2NE2001889, NT2NE2001923, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150,
NT2NE2003185, NT2NE2003252, NT2NE2003280, NT2NE2003308, NT2NE2003309, NT2NE2003315, NT2NE2003393, NT2NE2003408, NT2NE2003457, NT2NE2003540, NT2NE2003571, NT2NE2003705, NT2NE2003752, NT2NE2003921, NT2NE2004378, NT2NE2004490, NT2NE2004519, NT2NE2004606, NT2NE2004716, NT2NE2004787, NT2NE2004916, NT2NE2005035, NT2NE2005223, NT2NE2005276, NT2NE2005358, NT2NE2005395, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006531,
NT2NE2006659, NT2NE2006784, NT2NE2006813, NT2NE2006894, NT2NE2006942, NT2NE2006958, NT2NE2007052, NT2NE2007220, NT2NE2007425, NT2NE2007725, NT2NE2007727, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011154, NT2NE2011221, NT2NE2011411, NT2NE2011485,
NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015275, NT2NE2015480, NT2NE2015511, NT2NE2015565, NT2NE2015626, NT2NE2015747, NT2NE2015860, NT2NE2015885, NT2NE2015904, NT2NE2016041, NT2NE2016387,
NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916, NT2NE2019056, NT2NE2019076, NT2NE2019099, NT2RI1000035, NT2RI1000164, NT2RI1000172, NT2RI2000004, NT2RI2000007, NT2RI2000117, NT2RI2000128, NT2RI2000133, NT2RI2000173, NT2RI2000187, NT2RI2000270, NT2RI2000276, NT2RI2000313, NT2RI2000341, NT2RI2000344, NT2RI2000421, NT2RI2000480, NT2RI2000575, NT2RI2000588,
NT2RI2000597, NT2RI2000669, NT2RI2000688, NT2RI2000689, NT2RI2000704, NT2RI2000738, NT2RI2000841, NT2RI2000865, NT2RI2000932, NT2RI2000987, NT2RI2001017, NT2RI2001030, NT2RI2001054, NT2RI2001083, NT2RI2001091, NT2RI2001167, NT2RI2001178, NT2RI2001230, NT2RI2001235, NT2RI2001244, NT2RI2001307, NT2RI2001385, NT2RI2001409, NT2RI2001410, NT2RI2001425, NT2RI2001449, NT2RI2001450, NT2RI2001474, NT2RI2001519, NT2RI2001595, NT2RI2001621, NT2RI2001624, NT2RI2001769, NT2RI2001782, NT2RI2001836, NT2RI2001952, NT2RI2002022, NT2RI2002041, NT2RI2002117, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002447,
NT2RI2002481, NT2RI2002507, NT2RI2002517, NT2RI2002530, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819, NT2RI2002822, NT2RI2002852, NT2RI2002923, NT2RI2002926, NT2RI2002970, NT2RI2003067, NT2RI2003184, NT2RI2003205, NT2RI2003222, NT2RI2003301, NT2RI2003360, NT2RI2003361, NT2RI2003383, NT2RI2003392, NT2RI2003399, NT2RI2003419, NT2RI2003481, NT2RI2003487, NT2RI2003556, NT2RI2003655, NT2RI2003667, NT2RI2003751, NT2RI2003884, NT2RI2003973, NT2RI2003993, NT2RI2004078, NT2RI2004079, NT2RI2004093, NT2RI2004136, NT2RI2004157, NT2RI2004188, NT2RI2004190, NT2RI2004209, NT2RI2004230, NT2RI2004284, NT2RI2004290,
NT2RI2004312, NT2RI2004381, NT2RI2004398, NT2RI2004410, NT2RI2004442, NT2RI2004468, NT2RI2004606, NT2RI2004618, NT2RI2004783, NT2RI2004818, NT2RI2004840, NT2RI2004916, NT2RI2004962, NT2RI2004984, NT2RI2004985, NT2RI2005048, NT2RI2005069, NT2RI2005087, NT2RI2005096, NT2RI2005115, NT2RI2005130, NT2RI2005166, NT2RI2005239, NT2RI2005246, NT2RI2005315, NT2RI2005358, NT2RI2005368, NT2RI2005382, NT2RI2005520, NT2RI2005564, NT2RI2005579, NT2RI2005621, NT2RI2005628, NT2RI2005670, NT2RI2005772, NT2RI2005816, NT2RI2005851, NT2RI2005966, NT2RI2006071, NT2RI2006081, NT2RI2006126, NT2RI2006127, NT2RI2006183, NT2RI2006257, NT2RI2006294, NT2RI2006345, NT2RI2006403, NT2RI2006445, NT2RI2006487, NT2RI2006506,
NT2RI2006553, NT2RI2006647, NT2RI2006659, NT2RI2006662, NT2RI2006667, NT2RI2006679, NT2RI2006686, NT2RI2006716, NT2RI2006735, NT2RI2006762, NT2RI2006788, NT2RI2006825, NT2RI2006838, NT2RI2006855, NT2RI2006973, NT2RI2007046, NT2RI2007048, NT2RI2007116, NT2RI2007148, NT2RI2007214, NT2RI2007303, NT2RI2007334, NT2RI2007386, NT2RI2007406, NT2RI2007439, NT2RI2007469, NT2RI2007498, NT2RI2007507, NT2RI2007593, NT2RI2007729, NT2RI2007751, NT2RI2007754, NT2RI2007827, NT2RI2007853, NT2RI2007877, NT2RI2007879, NT2RI2007891, NT2RI2007956, NT2RI2007965, NT2RI2008007, NT2RI2008050, NT2RI2008171, NT2RI2008188, NT2RI2008204, NT2RI2008221, NT2RI2008233, NT2RI2008455, NT2RI2008481, NT2RI2008502, NT2RI2008598,
NT2RI2008622, NT2RI2008656, NT2RI2008749, NT2RI2008801, NT2RI2008812, NT2RI2008841, NT2RI2008952, NT2RI2009005, NT2RI2009101, NT2RI2009103, NT2RI2009144, NT2RI2009151, NT2RI2009215, NT2RI2009233, NT2RI2009281, NT2RI2009289, NT2RI2009301, NT2RI2009402, NT2RI2009517, NT2RI2009585, NT2RI2009595, NT2RI2009855, NT2RI2010508, NT2RI2010795, NT2RI2011422, NT2RI2011683, NT2RI2011803, NT2RI2012350, NT2RI2012659, NT2RI2012990, NT2RI2013345, NT2RI2013357, NT2RI2013373, NT2RI2014247, NT2RI2014551, NT2RI2014683, NT2RI2014733, NT2RI2015239, NT2RI2016128, NT2RI2016134, NT2RI2017529, NT2RI2018311, NT2RI2018363, NT2RI2018883, NT2RI2018950, NT2RI2019751, NT2RI2019826, NT2RI2020390, NT2RI2020703, NT2RI2021463,
NT2RI2021625, NT2RI2022468, NT2RI2022584, NT2RI2023303, NT2RI2023671, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027323, NT2RI2027396, NT2RI2027484, NT2RI2027975, NT2RI2028213, NT2RI2028222, NT2RI2028537, NT2RI2028642, NT2RI3000213, NT2RI3000622, NT2RI3001132, NT2RI3001263, NT2RI3001279, NT2RI3001348, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003925, NT2RI3004133, NT2RI3004381,
NT2RI3004510, NT2RI3005202, NT2RI3005403, NT2RI3005416, NT2RI3005724, NT2RI3005861, NT2RI3005928, NT2RI3006132, NT2RI3006171, NT2RI3006340, NT2RI3006376, NT2RI3006666, NT2RI3006796, NT2RI3007065, NT2RI3007095, NT2RI3007158, NT2RI3007167, NT2RI3007213, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009524, NT2RP6000005, NT2RP6000008, NT2RP6000032, NT2RP6000033, NT2RP6000043, NT2RP6000059, NT2RP6000060, NT2RP6000077, NT2RP6000086, NT2RP6000100, NT2RP6000109, NT2RP6000119, NT2RP6000127, NT2RP6000130, NT2RP6000140, NT2RP6000150, NT2RP7000041,
NT2RP7000109, NT2RP7000311, NT2RP7000359, NT2RP7000364, NT2RP7000380, NT2RP7000391, NT2RP7000425, NT2RP7000579, NT2RP7000586, NT2RP7000624, NT2RP7000812, NT2RP7000926, NT2RP7001074, NT2RP7001080, NT2RP7001156, NT2RP7001306, NT2RP7001319, NT2RP7001364, NT2RP7001475, NT2RP7001532, NT2RP7001602, NT2RP7001678, NT2RP7001856, NT2RP7002064, NT2RP7002151, NT2RP7002163, NT2RP7002243, NT2RP7002478, NT2RP7002554, NT2RP7002594, NT2RP7002603, NT2RP7002650, NT2RP7002738, NT2RP7002779, NT2RP7002841, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003261, NT2RP7003439, NT2RP7003680, NT2RP7003724,
NT2RP7004027, NT2RP7004080, NT2RP7004233, NT2RP7004358, NT2RP7004396, NT2RP7004481, NT2RP7004541, NT2RP7004559, NT2RP7004722, NT2RP7004766, NT2RP7004790, NT2RP7004915, NT2RP7004964, NT2RP7004975, NT2RP7005118, NT2RP7005205, NT2RP7005219, NT2RP7005323, NT2RP7005468, NT2RP7005513, NT2RP7005629, NT2RP7005631, NT2RP7005669, NT2RP7005846, NT2RP7006033, NT2RP7006075, NT2RP7006160, NT2RP7006162, NT2RP7006263, NT2RP7006296, NT2RP7006304, NT2RP7006374, NT2RP7006395, NT2RP7006408, NT2RP7006457, NT2RP7006547, NT2RP7006621, NT2RP7006636, NT2RP7006853, NT2RP7006887, NT2RP7006980, NT2RP7007114, NT2RP7007221, NT2RP7007226, NT2RP7007269, NT2RP7007310, NT2RP7007359, NT2RP7007480, NT2RP7007504, NT2RP7007594,
NT2RP7007617, NT2RP7007643, NT2RP7007842, NT2RP7007925, NT2RP7007930, NT2RP7008013, NT2RP7008133, NT2RP7008137, NT2RP7008142, NT2RP7008144, NT2RP7008190, NT2RP7008360, NT2RP7008396, NT2RP7008406, NT2RP7008435, NT2RP7008441, NT2RP7008454, NT2RP7008487, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009168, NT2RP7009215, NT2RP7009225, NT2RP7009236, NT2RP7009253, NT2RP7009267, NT2RP7009322, NT2RP7009363, NT2RP7009370, NT2RP7009373, NT2RP7009397, NT2RP7009429, NT2RP7009439, NT2RP7009502, NT2RP7009507, NT2RP7009577, NT2RP7009743, NT2RP7009867, NT2RP7010128, NT2RP7010235,
NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013729, NT2RP7013999, NT2RP7014178, NT2RP7014348, NT2RP7014420, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197,
NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8006452, NT2RP8006521, NT2RP8007416, NT2RP8007503, NT2RP8007715, NT2RP8009248, NTONG1000006, NTONG1000047, NTONG1000052, NTONG1000087, NTONG1000123, NTONG1000161, NTONG1000172, NTONG1000213, NTONG1000247, NTONG1000257, NTONG2000040, NTONG2000107,
NTONG2000231, NTONG2000334, NTONG2000363, NTONG2000413, NTONG2000492, NTONG2000499, NTONG2000531, NTONG2000583, NTONG2000858, NTONG2000878, NTONG2000966, NTONG2001014, NTONG2001079, NTONG2001157, NTONG2001173, NTONG2001220, NTONG2001222, NTONG2001362, NTONG2001428, NTONG2001532, NTONG2001587, NTONG2001619, NTONG2001642, NTONG2001657, NTONG2001676, NTONG2002278, NTONG2002807, NTONG2002948, NTONG2002970, NTONG2002985, NTONG2003158, NTONG2003210, NTONG2003316, NTONG2003409, NTONG2003515, NTONG2003805, NTONG2003839, NTONG2003852, NTONG2003897, NTONG2004521, NTONG2004690, NTONG2004806, NTONG2004829, NTONG2004844, NTONG2004991, NTONG2005062, NTONG2005086, NTONG2005137, NTONG2005153, NTONG2005265,
NTONG2005277, NTONG2005363, NTONG2005480, NTONG2005497, NTONG2005577, NTONG2005657, NTONG2005822, NTONG2005897, NTONG2006099, NTONG2006187, NTONG2006301, NTONG2006324, NTONG2006354, NTONG2006440, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008365, NTONG2008672, NTONG2008862, NTONG2008939, NTONG2009068, NTONG2009229, NTONG2009233, NTONG2009468, OCBBF1000016, OCBBF1000030, OCBBF1000042, OCBBF1000049, OCBBF1000054, OCBBF1000067, OCBBF1000085, OCBBF1000091, OCBBF1000114, OCBBF1000118, OCBBF1000119, OCBBF1000122, OCBBF1000130,
OCBBF1000138, OCBBF1000145, OCBBF1000153, OCBBF1000175, OCBBF1000185, OCBBF1000188, OCBBF1000192, OCBBF1000254, OCBBF2000277, OCBBF2000287, OCBBF2000323, OCBBF2000452, OCBBF2000467, OCBBF2000703, OCBBF2000712, OCBBF2000719, OCBBF2000824, OCBBF2000904, OCBBF2000998, OCBBF2001075, OCBBF2001101, OCBBF2001124, OCBBF2001140, OCBBF2001176, OCBBF2001186, OCBBF2001210, OCBBF2001252, OCBBF2001323, OCBBF2001389, OCBBF2001402, OCBBF2001408, OCBBF2001416, OCBBF2001473, OCBBF2001494, OCBBF2001527, OCBBF2001586, OCBBF2001681, OCBBF2001749, OCBBF2001794, OCBBF2001910, OCBBF2001931, OCBBF2001961, OCBBF2002083, OCBBF2002124, OCBBF2002161, OCBBF2002357, OCBBF2002376, OCBBF2002615, OCBBF2002626, OCBBF2002663,
OCBBF2002805, OCBBF2002865, OCBBF2002920, OCBBF2002980, OCBBF2003028, OCBBF2003091, OCBBF2003246, OCBBF2003327, OCBBF2003593, OCBBF2004038, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151,
OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006764, OCBBF2006859, OCBBF2006972, OCBBF2006987, OCBBF2007039, OCBBF2007051, OCBBF2007068, OCBBF2007114, OCBBF2007184, OCBBF2007194, OCBBF2007196, OCBBF2007238, OCBBF2007428, OCBBF2007478, OCBBF2007485, OCBBF2007520, OCBBF2007555, OCBBF2007610, OCBBF2007622, OCBBF2007756, OCBBF2007762, OCBBF2007931, OCBBF2007946, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724,
OCBBF2008760, OCBBF2008768, OCBBF2008770, OCBBF2008775, OCBBF2008790, OCBBF2008814, OCBBF2009095, OCBBF2009115, OCBBF2009352, OCBBF2009391, OCBBF2009424, OCBBF2009536, OCBBF2009571, OCBBF2009583, OCBBF2009603, OCBBF2009788, OCBBF2009920, OCBBF2009926, OCBBF2010040, OCBBF2010313, OCBBF2010404, OCBBF2010416, OCBBF2010420, OCBBF2010604, OCBBF2010709, OCBBF2010792, OCBBF2010819, OCBBF2010843, OCBBF2010858, OCBBF2010863, OCBBF2010871, OCBBF2010931, OCBBF2010945, OCBBF2010978, OCBBF2010989, OCBBF2011073, OCBBF2011137, OCBBF2011160, OCBBF2011177, OCBBF2011228, OCBBF2011232, OCBBF2011283, OCBBF2011311, OCBBF2011536, OCBBF2011625, OCBBF2011669, OCBBF2011685, OCBBF2011706, OCBBF2011722, OCBBF2011855,
OCBBF2011872, OCBBF2011897, OCBBF2011914, OCBBF2011960, OCBBF2011981, OCBBF2012028, OCBBF2012039, OCBBF2012095, OCBBF2012139, OCBBF2012191, OCBBF2012262, OCBBF2012320, OCBBF2012525, OCBBF2012678, OCBBF2012704, OCBBF2012755, OCBBF2012812, OCBBF2012881, OCBBF2012908, OCBBF2012936, OCBBF2012990, OCBBF2013049, OCBBF2013091, OCBBF2013127, OCBBF2013208, OCBBF2013285, OCBBF2013319, OCBBF2013324, OCBBF2013481, OCBBF2013507, OCBBF2013721, OCBBF2013789, OCBBF2013883, OCBBF2013926, OCBBF2014089, OCBBF2014292, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232,
OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016689, OCBBF2016690, OCBBF2016841, OCBBF2016928, OCBBF2017069, OCBBF2017306, OCBBF2017325, OCBBF2017458, OCBBF2017489, OCBBF2017516, OCBBF2017665, OCBBF2017815, OCBBF2017882, OCBBF2017888, OCBBF2017920, OCBBF2018014, OCBBF2018084, OCBBF2018167, OCBBF2018206, OCBBF2018229, OCBBF2018234, OCBBF2018362, OCBBF2018380, OCBBF2018618, OCBBF2018663, OCBBF2018687, OCBBF2018707, OCBBF2018828, OCBBF2018873, OCBBF2018934, OCBBF2019195, OCBBF2019327,
OCBBF2019684, OCBBF2019761, OCBBF2019823, OCBBF2019950, OCBBF2020048, OCBBF2020343, OCBBF2020453, OCBBF2020741, OCBBF2020825, OCBBF2020838, OCBBF2021020, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481,
OCBBF2027511, OCBBF2027661, OCBBF2027728, OCBBF2027782, OCBBF2027868, OCBBF2028055, OCBBF2028173, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035885, OCBBF2035916, OCBBF2036019, OCBBF2036031, OCBBF2036225, OCBBF2036476, OCBBF2036752, OCBBF2037068, OCBBF2037340,
OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000185, PEBLM1000024, PEBLM1000029, PEBLM1000034, PEBLM1000062,
PEBLM1000068, PEBLM1000071, PEBLM1000083, PEBLM1000147, PEBLM1000174, PEBLM1000180, PEBLM2000112, PEBLM2000126, PEBLM2000170, PEBLM2000180, PEBLM2000213, PEBLM2000248, PEBLM2000267, PEBLM2000270, PEBLM2000321, PEBLM2000333, PEBLM2000479, PEBLM2000502, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003935, PEBLM2004015, PEBLM2004216, PEBLM2004290, PEBLM2004497, PEBLM2004733, PEBLM2005282, PEBLM2005615, PEBLM2005632, PEBLM2005697, PEBLM2006031, PEBLM2006049, PEBLM2006113, PEBLM2006135, PEBLM2006215, PEBLM2006283, PEBLM2006298, PEBLM2006366, PEBLM2006912, PEBLM2007112, PEBLM2007140,
PEBLM2007437, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117,
PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000058, PLACE5000066, PLACE5000067, PLACE5000070, PLACE5000080, PLACE5000105, PLACE5000115, PLACE5000116, PLACE5000129, PLACE5000139, PLACE5000153, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000263,
PLACE6000296, PLACE6000314, PLACE6000348, PLACE6000371, PLACE6000424, PLACE6000426, PLACE6000429, PLACE6000450, PLACE6000523, PLACE6000550, PLACE6000552, PLACE6000555, PLACE6000619, PLACE6000953, PLACE6001118, PLACE6001153, PLACE6001185, PLACE6001262, PLACE6001294, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002692, PLACE6002699, PLACE6002710, PLACE6002871, PLACE6002949, PLACE6003004, PLACE6003038, PLACE6003077, PLACE6003094, PLACE6003109, PLACE6003204, PLACE6003372,
PLACE6003393, PLACE6003399, PLACE6003539, PLACE6003621, PLACE6003705, PLACE6003740, PLACE6003771, PLACE6003796, PLACE6003839, PLACE6003850, PLACE6004101, PLACE6004219, PLACE6004336, PLACE6004368, PLACE6004380, PLACE6004396, PLACE6004454, PLACE6004464, PLACE6004491, PLACE6004578, PLACE6004663, PLACE6004687, PLACE6004738, PLACE6004931, PLACE6004966, PLACE6004993, PLACE6005007, PLACE6005029, PLACE6005108, PLACE6005234, PLACE6005283, PLACE6005328, PLACE6005351, PLACE6005423, PLACE6005487, PLACE6005535, PLACE6005546, PLACE6005559, PLACE6005579, PLACE6005691, PLACE6005786, PLACE6006077, PLACE6006158, PLACE6006217, PLACE6006222, PLACE6006266, PLACE6006287, PLACE6006394, PLACE6006418, PLACE6006474,
PLACE6006566, PLACE6006697, PLACE6006822, PLACE6006871, PLACE6006905, PLACE6006988, PLACE6007050, PLACE6007180, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008824, PLACE6008989, PLACE6009006, PLACE6009008, PLACE6009228, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010485, PLACE6010568, PLACE6010701, PLACE6010704,
PLACE6010732, PLACE6010765, PLACE6010925, PLACE6010936, PLACE6010991, PLACE6011041, PLACE6011102, PLACE6011156, PLACE6011211, PLACE6011260, PLACE6011334, PLACE6011627, PLACE6011774, PLACE6011881, PLACE6011907, PLACE6011975, PLACE6012108, PLACE6012168, PLACE6012297, PLACE6012308, PLACE6012574, PLACE6012908, PLACE6012942, PLACE6013288, PLACE6013386, PLACE6013400, PLACE6013650, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016383,
PLACE6017002, PLACE6017431, PLACE6017564, PLACE6017578, PLACE6017626, PLACE6017701, PLACE6017714, PLACE6017788, PLACE6017791, PLACE6018107, PLACE6018187, PLACE6018235, PLACE6018287, PLACE6018441, PLACE6018497, PLACE6018769, PLACE6018834, PLACE6018843, PLACE6018863, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7006051, PLACE7006090, PLACE7006240,
PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000123, PROST1000136, PROST1000144, PROST1000145, PROST1000152, PROST1000167, PROST1000184, PROST1000217, PROST1000262, PROST1000272, PROST1000298, PROST1000322, PROST1000334, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000559, PROST1000575, PROST2000053, PROST2000067, PROST2000079, PROST2000105,
PROST2000143, PROST2000162, PROST2000165, PROST2000176, PROST2000203, PROST2000206, PROST2000212, PROST2000267, PROST2000273, PROST2000325, PROST2000337, PROST2000432, PROST2000452, PROST2000463, PROST2000470, PROST2000505, PROST2000572, PROST2000717, PROST2000761, PROST2000893, PROST2000961, PROST2001138, PROST2001213, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001899, PROST2001998, PROST2002078, PROST2002101, PROST2002162, PROST2002212, PROST2002227, PROST2002338, PROST2002425, PROST2002488, PROST2002489, PROST2002527, PROST2002591,
PROST2002602, PROST2002633, PROST2002682, PROST2002685, PROST2002722, PROST2002736, PROST2002842, PROST2002906, PROST2002927, PROST2003102, PROST2003117, PROST2003210, PROST2003302, PROST2003303, PROST2003324, PROST2003340, PROST2003428, PROST2003472, PROST2003563, PROST2003577, PROST2003583, PROST2003586, PROST2003628, PROST2003635, PROST2003674, PROST2003676, PROST2003732, PROST2003775, PROST2003922, PROST2003930, PROST2004115, PROST2004142, PROST2004146, PROST2004161, PROST2004251, PROST2004258, PROST2004332, PROST2004481, PROST2004526, PROST2004570, PROST2004727, PROST2004742, PROST2004817, PROST2005039, PROST2005076, PROST2005093, PROST2005121, PROST2005131, PROST2005228, PROST2005426,
PROST2005466, PROST2005630, PROST2005683, PROST2005788, PROST2005880, PROST2005886, PROST2005904, PROST2005919, PROST2005943, PROST2006008, PROST2006020, PROST2006196, PROST2006201, PROST2006282, PROST2006450, PROST2006491, PROST2006510, PROST2006579, PROST2006688, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008268, PROST2008447, PROST2008468, PROST2008472, PROST2008516, PROST2008724,
PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010250, PROST2010318, PROST2010326, PROST2010382, PROST2010400, PROST2010606, PROST2010885, PROST2011012, PROST2011038, PROST2011105, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012353, PROST2012400, PROST2012542, PROST2012550, PROST2012740, PROST2012780,
PROST2012888, PROST2012890, PROST2013032, PROST2013053, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015332, PROST2015457, PROST2015464, PROST2015537, PROST2015545, PROST2015551, PROST2015636, PROST2015710, PROST2015756, PROST2015877, PROST2015900, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829,
PROST2016860, PROST2016918, PROST2016980, PROST2017098, PROST2017367, PROST2017413, PROST2017441, PROST2017529, PROST2017578, PROST2017617, PROST2017692, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019296, PROST2019398, PROST2019487, PROST2019781, PUAEN1000039, PUAEN1000081, PUAEN1000085, PUAEN1000087, PUAEN1000121, PUAEN1000175, PUAEN1000239, PUAEN1000275, PUAEN1000276, PUAEN1000288, PUAEN1000322, PUAEN2000080, PUAEN2000175, PUAEN2000247, PUAEN2000394, PUAEN2000497,
PUAEN2000594, PUAEN2000684, PUAEN2000942, PUAEN2001260, PUAEN2001345, PUAEN2001526, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004511, PUAEN2004525, PUAEN2004875, PUAEN2005110, PUAEN2005247, PUAEN2005502, PUAEN2005588, PUAEN2006029, PUAEN2006335, PUAEN2006578, PUAEN2006639, PUAEN2006761, PUAEN2007350, PUAEN2007785, PUAEN2007898, PUAEN2008123, PUAEN2008228, PUAEN2008314, PUAEN2008515, PUAEN2008815, PUAEN2008980, PUAEN2009348, PUAEN2009534, PUAEN2009655, PUAEN2009795, PUAEN2009852, PUAEN2010824, RECTM1000051, RECTM1000141,
RECTM2000220, RECTM2000349, RECTM2000433, RECTM2000510, RECTM2001307, RECTM2001347, RECTM2001519, RECTM2001666, RECTM2001691, RECTM2001749, RECTM2002602, RECTM2002632, SALGL1000005, SALGL1000024, SALGL1000028, SALGL1000065, SALGL1000107, SKMUS1000030, SKMUS1000064, SKMUS1000067, SKMUS1000102, SKMUS1000104, SKMUS1000118, SKMUS1000124, SKMUS1000129, SKMUS1000138, SKMUS1000176, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000361, SKMUS2000365, SKMUS2000380, SKMUS2000390, SKMUS2000416, SKMUS2000458, SKMUS2000467, SKMUS2000468, SKMUS2000515, SKMUS2000690,
SKMUS2000701, SKMUS2000705, SKMUS2000710, SKMUS2000724, SKMUS2000726, SKMUS2000774, SKMUS2000780, SKMUS2000821, SKMUS2000847, SKMUS2000863, SKMUS2000873, SKMUS2000894, SKMUS2000898, SKMUS2000902, SKMUS2000945, SKMUS2000961, SKMUS2000973, SKMUS2000980, SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695,
SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000159, SKNMC2000256,
SKNMC2000305, SKNMC2000322, SKNMC2000374, SKNMC2000583, SKNMC2000593, SKNMC2000612, SKNMC2000622, SKNMC2000635, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006327, SKNMC2006998, SKNMC2007134, SKNMC2007502, SKNMC2007504, SKNMC2007961, SKNMC2009450, SKNSH1000086, SKNSH1000174, SKNSH1000301, SKNSH1000308, SKNSH1000416, SKNSH2000051, SKNSH2000151, SKNSH2000163, SKNSH2000319, SKNSH2000482, SKNSH2000516, SKNSH2000550, SKNSH2000716, SKNSH2000971, SKNSH2001875, SKNSH2001931, SKNSH2002054, SKNSH2002866, SKNSH2003064, SKNSH2003174,
SKNSH2003422, SKNSH2003466, SKNSH2003483, SKNSH2003633, SKNSH2004039, SKNSH2005061, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000039, SMINT1000048, SMINT1000051, SMINT1000054, SMINT1000057, SMINT1000071, SMINT1000075, SMINT1000078, SMINT1000091, SMINT1000100, SMINT1000103, SMINT1000118, SMINT1000131, SMINT1000137, SMINT2000018, SMINT2000032, SMINT2000040, SMINT2000060, SMINT2000073, SMINT2000084, SMINT2000145, SMINT2000176,
SMINT2000185, SMINT2000227, SMINT2000229, SMINT2000267, SMINT2000400, SMINT2000518, SMINT2000529, SMINT2000530, SMINT2000537, SMINT2000538, SMINT2000541, SMINT2000558, SMINT2000562, SMINT2000568, SMINT2000609, SMINT2000811, SMINT2000824, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002202, SMINT2002210, SMINT2002281, SMINT2002311, SMINT2002314, SMINT2002328, SMINT2002414, SMINT2002457, SMINT2002743, SMINT2002778, SMINT2002880, SMINT2002884, SMINT2002899, SMINT2002974,
SMINT2003128, SMINT2003150, SMINT2003317, SMINT2003386, SMINT2003551, SMINT2003569, SMINT2003641, SMINT2003644, SMINT2003866, SMINT2004047, SMINT2004086, SMINT2004299, SMINT2004339, SMINT2004350, SMINT2004414, SMINT2004473, SMINT2004547, SMINT2004583, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005402, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716, SMINT2007101, SMINT2007140, SMINT2007219, SMINT2007294,
SMINT2007365, SMINT2007391, SMINT2007433, SMINT2007508, SMINT2007526, SMINT2007579, SMINT2007647, SMINT2007729, SMINT2007790, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009233, SMINT2009259, SMINT2009272, SMINT2009363, SMINT2009415, SMINT2009468, SMINT2009505, SMINT2009529, SMINT2009832, SMINT2009895, SMINT2009902, SMINT2009973, SMINT2010015, SMINT2010068,
SMINT2010076, SMINT2010144, SMINT2010200, SMINT2010278, SMINT2010324, SMINT2010326, SMINT2010426, SMINT2010500, SMINT2010533, SMINT2010629, SMINT2010639, SMINT2010672, SMINT2010753, SMINT2010853, SMINT2010897, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012122, SMINT2012195, SMINT2012285, SMINT2012501, SMINT2012734, SMINT2012793, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721,
SMINT2015006, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000049, SPLEN1000087, SPLEN1000089, SPLEN1000091, SPLEN1000139, SPLEN1000143, SPLEN1000144,
SPLEN1000145, SPLEN1000156, SPLEN1000163, SPLEN1000165, SPLEN1000178, SPLEN2000020, SPLEN2000064, SPLEN2000126, SPLEN2000134, SPLEN2000189, SPLEN2000197, SPLEN2000205, SPLEN2000210, SPLEN2000222, SPLEN2000242, SPLEN2000247, SPLEN2000258, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000537, SPLEN2000541, SPLEN2000607, SPLEN2000630, SPLEN2000695, SPLEN2000698, SPLEN2000839, SPLEN2001135, SPLEN2001176, SPLEN2001227, SPLEN2001245, SPLEN2001354, SPLEN2001367, SPLEN2001383, SPLEN2001503, SPLEN2001510,
SPLEN2001599, SPLEN2001626, SPLEN2001650, SPLEN2001689, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002272, SPLEN2002314, SPLEN2002343, SPLEN2002354, SPLEN2002385, SPLEN2002419, SPLEN2002462, SPLEN2002467, SPLEN2002477, SPLEN2002662, SPLEN2002695, SPLEN2002707, SPLEN2002917, SPLEN2002957, SPLEN2003160, SPLEN2003204, SPLEN2003219, SPLEN2003396, SPLEN2003678, SPLEN2003878, SPLEN2003918, SPLEN2003924, SPLEN2004060, SPLEN2004078, SPLEN2004124, SPLEN2004148, SPLEN2004343, SPLEN2004346, SPLEN2004368, SPLEN2004844, SPLEN2004880, SPLEN2005009, SPLEN2005227, SPLEN2005272, SPLEN2005450,
SPLEN2005474, SPLEN2005727, SPLEN2005838, SPLEN2005869, SPLEN2005927, SPLEN2005939, SPLEN2006268, SPLEN2006283, SPLEN2006325, SPLEN2006389, SPLEN2006425, SPLEN2006671, SPLEN2006701, SPLEN2006785, SPLEN2006875, SPLEN2007276, SPLEN2007314, SPLEN2007350, SPLEN2007619, SPLEN2007653, SPLEN2007689, SPLEN2007695, SPLEN2007750, SPLEN2007926, SPLEN2007951, SPLEN2008007, SPLEN2008135, SPLEN2008164, SPLEN2008317, SPLEN2008496, SPLEN2008591, SPLEN2008786, SPLEN2009088, SPLEN2009315, SPLEN2009340, SPLEN2009541, SPLEN2009548, SPLEN2009555, SPLEN2009581, SPLEN2009733, SPLEN2009803, SPLEN2009884, SPLEN2009918, SPLEN2010004, SPLEN2010112, SPLEN2010119, SPLEN2010146, SPLEN2010187, SPLEN2010350, SPLEN2010395,
SPLEN2010415, SPLEN2010447, SPLEN2010469, SPLEN2010510, SPLEN2010530, SPLEN2010534, SPLEN2010588, SPLEN2010800, SPLEN2010847, SPLEN2010862, SPLEN2010978, SPLEN2010986, SPLEN2011018, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012524, SPLEN2012525, SPLEN2012606, SPLEN2012611, SPLEN2012619, SPLEN2012725, SPLEN2012743, SPLEN2012756, SPLEN2012961, SPLEN2013195, SPLEN2013503, SPLEN2013644, SPLEN2013648, SPLEN2013673, SPLEN2013690, SPLEN2013714,
SPLEN2013821, SPLEN2013857, SPLEN2013860, SPLEN2013910, SPLEN2013936, SPLEN2014063, SPLEN2014073, SPLEN2014119, SPLEN2014136, SPLEN2014199, SPLEN2014210, SPLEN2014293, SPLEN2014318, SPLEN2014381, SPLEN2014454, SPLEN2014486, SPLEN2014543, SPLEN2014572, SPLEN2014645, SPLEN2014669, SPLEN2014711, SPLEN2014860, SPLEN2014865, SPLEN2014919, SPLEN2014920, SPLEN2014924, SPLEN2014937, SPLEN2014946, SPLEN2015031, SPLEN2015094, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015815, SPLEN2015890, SPLEN2015891, SPLEN2015899,
SPLEN2016045, SPLEN2016053, SPLEN2016069, SPLEN2016135, SPLEN2016241, SPLEN2016250, SPLEN2016304, SPLEN2016356, SPLEN2016480, SPLEN2016531, SPLEN2016533, SPLEN2016541, SPLEN2016552, SPLEN2016554, SPLEN2016602, SPLEN2016627, SPLEN2016773, SPLEN2016840, SPLEN2016863, SPLEN2016922, SPLEN2016932, SPLEN2016972, SPLEN2016973, SPLEN2017104, SPLEN2017121, SPLEN2017147, SPLEN2017187, SPLEN2017189, SPLEN2017212, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018159, SPLEN2018180, SPLEN2018181, SPLEN2018280, SPLEN2018285, SPLEN2018302, SPLEN2018364,
SPLEN2018395, SPLEN2018643, SPLEN2018732, SPLEN2018749, SPLEN2018933, SPLEN2019008, SPLEN2019043, SPLEN2019092, SPLEN2019102, SPLEN2019131, SPLEN2019257, SPLEN2019311, SPLEN2019323, SPLEN2019349, SPLEN2019375, SPLEN2019379, SPLEN2019471, SPLEN2019480, SPLEN2019571, SPLEN2019575, SPLEN2019709, SPLEN2019774, SPLEN2019793, SPLEN2019811, SPLEN2019839, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295, SPLEN2021298, SPLEN2021383, SPLEN2021417,
SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920, SPLEN2023024, SPLEN2023160, SPLEN2023423, SPLEN2023710, SPLEN2023791, SPLEN2023977, SPLEN2024070, SPLEN2024127, SPLEN2024273, SPLEN2024383, SPLEN2024530, SPLEN2024571, SPLEN2024577, SPLEN2024617, SPLEN2024737, SPLEN2024956, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138,
SPLEN2028365, SPLEN2028417, SPLEN2028424, SPLEN2028593, SPLEN2028844, SPLEN2028994, SPLEN2029051, SPLEN2029176, SPLEN2029380, SPLEN2029522, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031724, SPLEN2031780, SPLEN2032036, SPLEN2032112, SPLEN2032154, SPLEN2032321, SPLEN2032356, SPLEN2032677, SPLEN2032813, SPLEN2032924, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034864, SPLEN2034934, SPLEN2035318, SPLEN2035615,
SPLEN2036103, SPLEN2036326, SPLEN2036475, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037891, SPLEN2038055, SPLEN2038180, SPLEN2038345, SPLEN2038407, SPLEN2038473, SPLEN2039311, SPLEN2039697, SPLEN2039845, SPLEN2039936, SPLEN2040222, SPLEN2040237, SPLEN2040824, SPLEN2041304, SPLEN2041310, SPLEN2041585, SPLEN2041645, SPLEN2041700, SPLEN2041720, SPLEN2041977, SPLEN2042051, SPLEN2042521, SPLEN2042598, SPLEN2042714, SPLEN2042920, STOMA1000013, STOMA1000052, STOMA1000074, STOMA1000091, STOMA1000117, STOMA1000133, STOMA1000175, STOMA1000186,
STOMA1000190, STOMA1000191, STOMA2000032, STOMA2000072, STOMA2000088, STOMA2000104, STOMA2000148, STOMA2000168, STOMA2000183, STOMA2000188, STOMA2000279, STOMA2000289, STOMA2000383, STOMA2000386, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002141, STOMA2002367, STOMA2002688, STOMA2003289, STOMA2003444, STOMA2003477, STOMA2003646, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213,
STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, SYNOV1000045, SYNOV1000049, SYNOV1000118, SYNOV1000128, SYNOV1000190, SYNOV1000256, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001251, SYNOV2001300,
SYNOV2001356, SYNOV2001390, SYNOV2001451, SYNOV2001457, SYNOV2001468, SYNOV2001660, SYNOV2003326, SYNOV2005216, SYNOV2005448, SYNOV2005673, SYNOV2005817, SYNOV2005962, SYNOV2006430, SYNOV2006620, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981,
SYNOV4004184, SYNOV4004741, SYNOV4004914, SYNOV4005256, SYNOV4005570, SYNOV4005739, SYNOV4005889, SYNOV4005981, SYNOV4006256, SYNOV4007215, SYNOV4007360, SYNOV4007430, SYNOV4007521, SYNOV4007711, SYNOV4008336, SYNOV4009129, SYNOV4009139, SYNOV4009298, SYNOV4009575, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001492, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504,
TBAES2007548, TBAES2007697, TBAES2007862, TBAES2007964, TBAES2008133, TBAES2009387, TCERX2000171, TCERX2000431, TCERX2000613, TCERX2000998, TCERX2002644, TCOLN1000037, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2002005, TESOP2002110, TESOP2002150, TESOP2002273, TESOP2002489, TESOP2002539, TESOP2002545, TESOP2002804, TESOP2002950,
TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000055,
TESTI1000064, TESTI1000070, TESTI1000085, TESTI1000088, TESTI1000093, TESTI1000094, TESTI1000101, TESTI1000109, TESTI1000125, TESTI1000127, TESTI1000128, TESTI1000131, TESTI1000157, TESTI1000163, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000018, TESTI2000044, TESTI2000100, TESTI2000117, TESTI2000120, TESTI2000147, TESTI2000154, TESTI2000172, TESTI2000207, TESTI2000208, TESTI2000221, TESTI2000238, TESTI2000253, TESTI2000278, TESTI2000349, TESTI2000356, TESTI2000358, TESTI2000372, TESTI2000427, TESTI2000435, TESTI2000443, TESTI2000452,
TESTI2000462, TESTI2000489, TESTI2000571, TESTI2000591, TESTI2000600, TESTI2000616, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000849, TESTI2000850, TESTI2000874, TESTI2000883, TESTI2000909, TESTI2000951, TESTI2000981, TESTI2001008, TESTI2001049, TESTI2001082, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518, TESTI2001556,
TESTI2001593, TESTI2001613, TESTI2001621, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001869, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002149, TESTI2002216, TESTI2002245, TESTI2002251, TESTI2002264, TESTI2002282, TESTI2002293, TESTI2002294, TESTI2002326, TESTI2002351, TESTI2002361, TESTI2002406, TESTI2002415, TESTI2002423, TESTI2002453,
TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002910, TESTI2002912, TESTI2002928, TESTI2002937, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003117, TESTI2003127, TESTI2003130, TESTI2003131, TESTI2003139, TESTI2003141, TESTI2003152, TESTI2003181, TESTI2003193, TESTI2003196, TESTI2003228,
TESTI2003236, TESTI2003255, TESTI2003258, TESTI2003277, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998,
TESTI2004000, TESTI2004073, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004929, TESTI2004936,
TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005703, TESTI2005720, TESTI2005739, TESTI2005742, TESTI2005767, TESTI2005775, TESTI2005827,
TESTI2005835, TESTI2005860, TESTI2005892, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006097, TESTI2006109, TESTI2006111, TESTI2006125, TESTI2006179, TESTI2006209, TESTI2006210, TESTI2006212, TESTI2006218, TESTI2006238, TESTI2006255, TESTI2006278, TESTI2006333, TESTI2006341, TESTI2006360, TESTI2006409, TESTI2006425, TESTI2006437, TESTI2006457, TESTI2006460, TESTI2006465, TESTI2006483, TESTI2006499, TESTI2006523, TESTI2006543, TESTI2006565,
TESTI2006572, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006735, TESTI2006744, TESTI2006748, TESTI2006774, TESTI2006853, TESTI2006866, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007311, TESTI2007320, TESTI2007346, TESTI2007358, TESTI2007402, TESTI2007405, TESTI2007452, TESTI2007466, TESTI2007480, TESTI2007490, TESTI2007502, TESTI2007524, TESTI2007533, TESTI2007576, TESTI2007613, TESTI2007657,
TESTI2007685, TESTI2007692, TESTI2007749, TESTI2007750, TESTI2007808, TESTI2007814, TESTI2007829, TESTI2007867, TESTI2007872, TESTI2007906, TESTI2007922, TESTI2007951, TESTI2007972, TESTI2007973, TESTI2008014, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786,
TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009412, TESTI2009423, TESTI2009447, TESTI2009462, TESTI2009477, TESTI2009497, TESTI2009511, TESTI2009544, TESTI2009545, TESTI2009551, TESTI2009585, TESTI2009588, TESTI2009683, TESTI2009727, TESTI2009739, TESTI2009812, TESTI2009835, TESTI2009853, TESTI2009935, TESTI2009977, TESTI2010009, TESTI2010154, TESTI2010239, TESTI2010280, TESTI2010354, TESTI2010396, TESTI2010400, TESTI2010409, TESTI2010572,
TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244,
TESTI2012289, TESTI2012308, TESTI2012356, TESTI2012387, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889,
TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014577, TESTI2014578, TESTI2014716, TESTI2014780, TESTI2014800, TESTI2014843, TESTI2014979, TESTI2014988, TESTI2015042, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015987, TESTI2016046, TESTI2016101, TESTI2016162, TESTI2016197,
TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951,
TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018941, TESTI2018957, TESTI2018974, TESTI2018975, TESTI2019042, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019787, TESTI2019794, TESTI2019854, TESTI2019859, TESTI2019860, TESTI2019872,
TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020515, TESTI2020525, TESTI2020579, TESTI2020717, TESTI2020871, TESTI2020905, TESTI2020906, TESTI2020946, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021138, TESTI2021315, TESTI2021358, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895,
TESTI2021911, TESTI2021939, TESTI2021999, TESTI2022010, TESTI2022023, TESTI2022065, TESTI2022086, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267,
TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025791, TESTI2025846, TESTI2025872, TESTI2025879, TESTI2025911, TESTI2025920, TESTI2025924, TESTI2026014, TESTI2026024,
TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027296, TESTI2027378, TESTI2027411, TESTI2027450, TESTI2027496,
TESTI2027503, TESTI2027562, TESTI2027615, TESTI2027645, TESTI2027730, TESTI2027736, TESTI2027791, TESTI2027820, TESTI2027828, TESTI2027840, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029244, TESTI2029249, TESTI2029252, TESTI2029264, TESTI2029470, TESTI2029672, TESTI2029880, TESTI2030136, TESTI2030322, TESTI2030342, TESTI2030519, TESTI2030556, TESTI2030728, TESTI2030754,
TESTI2030770, TESTI2030860, TESTI2030901, TESTI2030917, TESTI2030930, TESTI2031006, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032681, TESTI2032761, TESTI2032768, TESTI2032774, TESTI2032828, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034357, TESTI2034401, TESTI2034407, TESTI2034506, TESTI2034520, TESTI2034718, TESTI2034730, TESTI2034749, TESTI2034767, TESTI2034774, TESTI2034777,
TESTI2034785, TESTI2034799, TESTI2034847, TESTI2034913, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375,
TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039342, TESTI2039353, TESTI2039544, TESTI2039613, TESTI2039732, TESTI2039738, TESTI2039776, TESTI2039867, TESTI2039908, TESTI2040018, TESTI2040094, TESTI2040102, TESTI2040128, TESTI2040143,
TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866,
TESTI2043869, TESTI2044064, TESTI2044118, TESTI2044194, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212,
TESTI2047342, TESTI2047383, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698,
TESTI2052799, TESTI2052822, TESTI2052985, TESTI2053151, TESTI2053242, TESTI2053399, TESTI2053526, TESTI2053561, TESTI2053621, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000370, TESTI4000394, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473,
TESTI4001517, TESTI4001527, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4003279, TESTI4003319, TESTI4003404, TESTI4003565, TESTI4003574, TESTI4003733, TESTI4003796, TESTI4003944, TESTI4004003, TESTI4004031, TESTI4004200, TESTI4004539, TESTI4004626, TESTI4004653, TESTI4004695, TESTI4004722, TESTI4004887, TESTI4004917, TESTI4005015, TESTI4005039, TESTI4005158, TESTI4005317, TESTI4005322, TESTI4005470, TESTI4005500,
TESTI4005534, TESTI4005543, TESTI4005628, TESTI4005801, TESTI4005805, TESTI4005857, TESTI4005961, TESTI4006053, TESTI4006079, TESTI4006112, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007965, TESTI4008007, TESTI4008018, TESTI4008050, TESTI4008058, TESTI4008086, TESTI4008097,
TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4011070, TESTI4011072, TESTI4011084,
TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392,.
TESTI4014415, TESTI4014445, TESTI4014553, TESTI4014661, TESTI4014694, TESTI4014801, TESTI4014818, TESTI4014891, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854,
TESTI4017901, TESTI4017961, TESTI4017984, TESTI4018101, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654,
TESTI4023722, TESTI4023762, TESTI4023942, TESTI4024096, TESTI4024240, TESTI4024294, TESTI4024344, TESTI4024387, TESTI4024420, TESTI4024494, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612,
TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4033146, TESTI4033177, TESTI4033433, TESTI4034172, TESTI4034212, TESTI4034432, TESTI4034495, TESTI4034632, TESTI4034633, TESTI4034912, TESTI4034973, TESTI4035063,
TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800,
TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330,
TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, TUTER1000014, TUTER1000137, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002356, TUTER2002729, UMVEN1000122, UMVEN1000156, UMVEN1000186,
UMVEN2000046, UMVEN2000069, UTERU1000015, UTERU1000024, UTERU1000031, UTERU1000032, UTERU1000057, UTERU1000065, UTERU1000077, UTERU1000093, UTERU1000096, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU2000023, UTERU2000047, UTERU2000074, UTERU2000099, UTERU2000154, UTERU2000197, UTERU2000218, UTERU2000238, UTERU2000243, UTERU2000300, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000539, UTERU2000541, UTERU2000542, UTERU2000546, UTERU2000550, UTERU2000569, UTERU2000607,
UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002332, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003411, UTERU2003456, UTERU2003577, UTERU2003926, UTERU2003973, UTERU2004015, UTERU2004039, UTERU2004061, UTERU2004073,
UTERU2004163, UTERU2004299, UTERU2004461, UTERU2004520, UTERU2004564, UTERU2004664, UTERU2004688, UTERU2004698, UTERU2004929, UTERU2005050, UTERU2005069, UTERU2005179, UTERU2005346, UTERU2005446, UTERU2005450, UTERU2005533, UTERU2005664, UTERU2005822, UTERU2005903, UTERU2005905, UTERU2006103, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007924, UTERU2007942, UTERU2008018, UTERU2008019, UTERU2008027, UTERU2008040,
UTERU2008077, UTERU2008130, UTERU2008347, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009414, UTERU2009483, UTERU2009510, UTERU2009538, UTERU2009540, UTERU2009776, UTERU2009904, UTERU2009951, UTERU2010115, UTERU2010124, UTERU2010164, UTERU2010231, UTERU2010304, UTERU2010320, UTERU2010431, UTERU2010525, UTERU2010651, UTERU2010724, UTERU2010747, UTERU2011195, UTERU2011199, UTERU2011261, UTERU2011410, UTERU2011574, UTERU2011657, UTERU2011806, UTERU2011811, UTERU2011897,
UTERU2011962, UTERU2011968, UTERU2012031, UTERU2012101, UTERU2012114, UTERU2012252, UTERU2012333, UTERU2012407, UTERU2012526, UTERU2012581, UTERU2012610, UTERU2012615, UTERU2012703, UTERU2012715, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015062, UTERU2015087, UTERU2015108, UTERU2015190, UTERU2015198,
UTERU2015202, UTERU2015405, UTERU2015640, UTERU2015880, UTERU2016147, UTERU2016157, UTERU2016426, UTERU2016464, UTERU2016669, UTERU2016757, UTERU2016761, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018955, UTERU2019038, UTERU2019163, UTERU2019257, UTERU2019453, UTERU2019491,
UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023687, UTERU2023712, UTERU2023941, UTERU2024002, UTERU2024042, UTERU2024141, UTERU2024481, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591,
UTERU2027616, UTERU2027941, UTERU2028377, UTERU2029503, UTERU2029660, UTERU2029742, UTERU2029953, UTERU2030213, UTERU2030270, UTERU2030280, UTERU2031060, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034695, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036507, UTERU2036512, UTERU2036530, UTERU2036623, UTERU2036690, UTERU2037224,
UTERU2037361, UTERU2037423, UTERU2037577, UTERU2037674, UTERU2037791, UTERU2037843, UTERU2038171, UTERU2038251, UTERU3000226, UTERU3000402, UTERU3000665, UTERU3000670, UTERU3000727, UTERU3000828, UTERU3000844, UTERU3000899, UTERU3000959, UTERU3001029, UTERU3001053, UTERU3001059, UTERU3001158, UTERU3001240, UTERU3001394, UTERU3001558, UTERU3001571, UTERU3001585, UTERU3001632, UTERU3001652, UTERU3001766, UTERU3001988, UTERU3002209, UTERU3002383, UTERU3002600, UTERU3002620, UTERU3002667, UTERU3002701, UTERU3002731, UTERU3002768, UTERU3002993, UTERU3003116, UTERU3003178, UTERU3003465, UTERU3003495, UTERU3003523, UTERU3003774, UTERU3003776, UTERU3004384, UTERU3004418, UTERU3004477, UTERU3004523,
UTERU3004616, UTERU3004635, UTERU3004709, UTERU3004938, UTERU3004992, UTERU3005159, UTERU3005205, UTERU3005230, UTERU3005264, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010526, UTERU3010604, UTERU3010892, UTERU3010919, UTERU3011063,
UTERU3011092, UTERU3011273, UTERU3011398, UTERU3011558, UTERU3011795, UTERU3011837, UTERU3012293, UTERU3012414, UTERU3012476, UTERU3012599, UTERU3012999, UTERU3013167, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018255, UTERU3018616, UTERU3019078

Of the 17,176 genes that include the cDNAs of the present invention, genes whose expression levels were high enough to detect in each of the TUTER, UMVEN, and UTERU libraries were analyzed for expression frequency. The analysis results are described below. Each is a relative value, where the expression level over all tissues is taken as 100. The larger this value, the greater the level of expression.

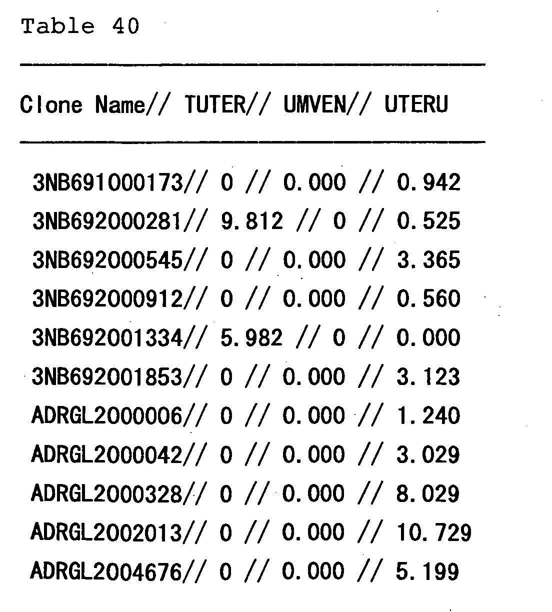

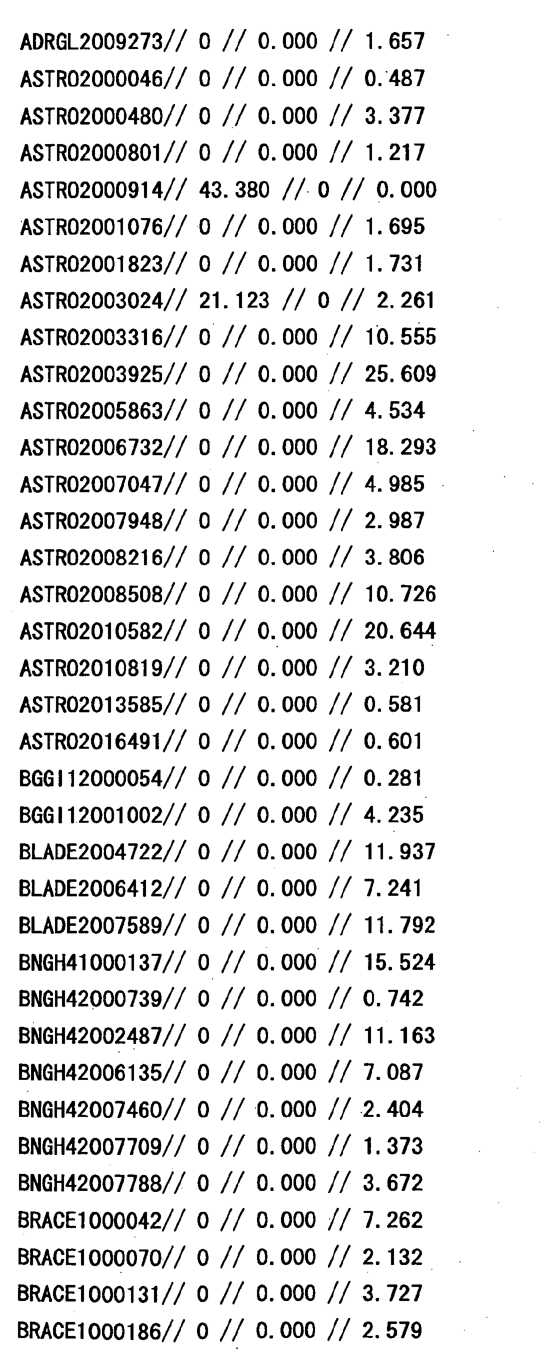

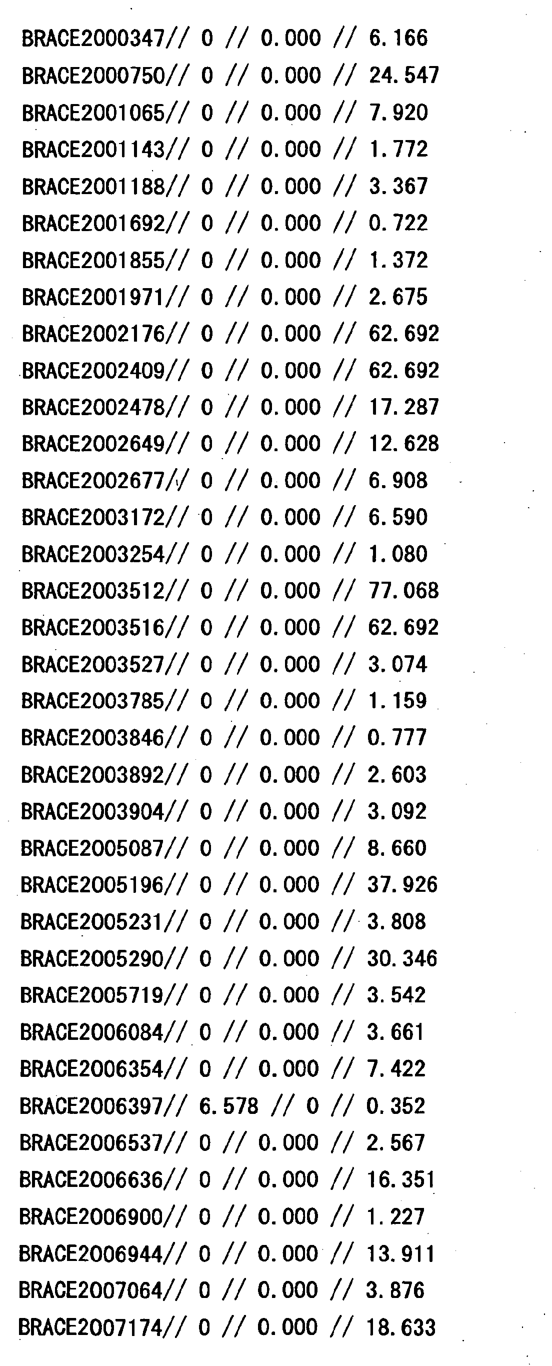

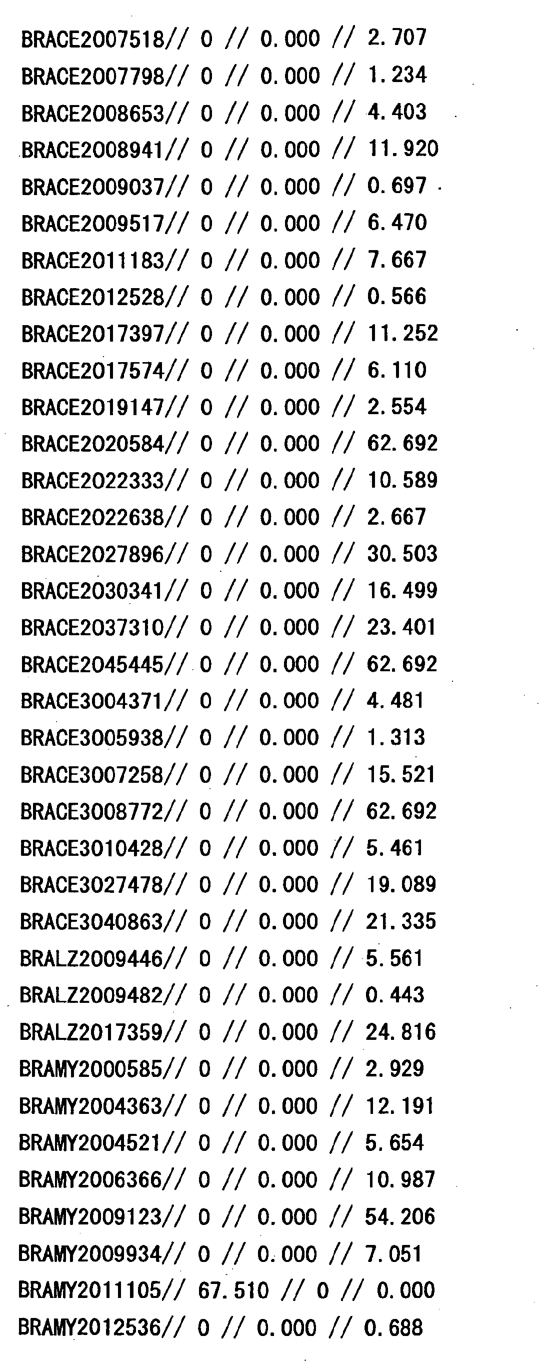

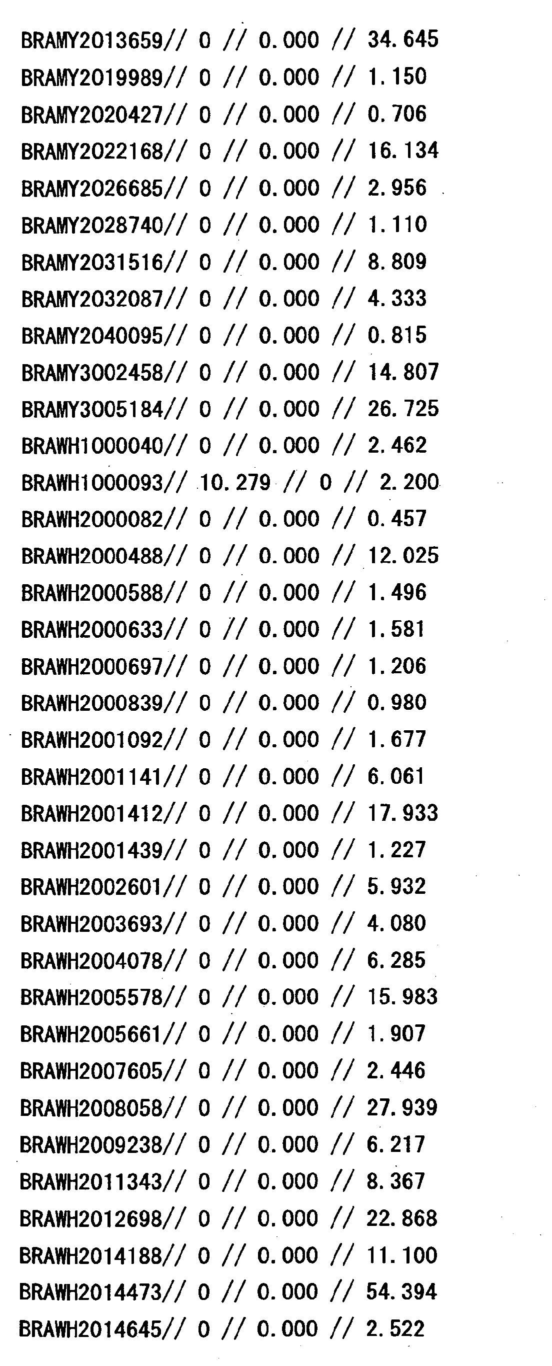

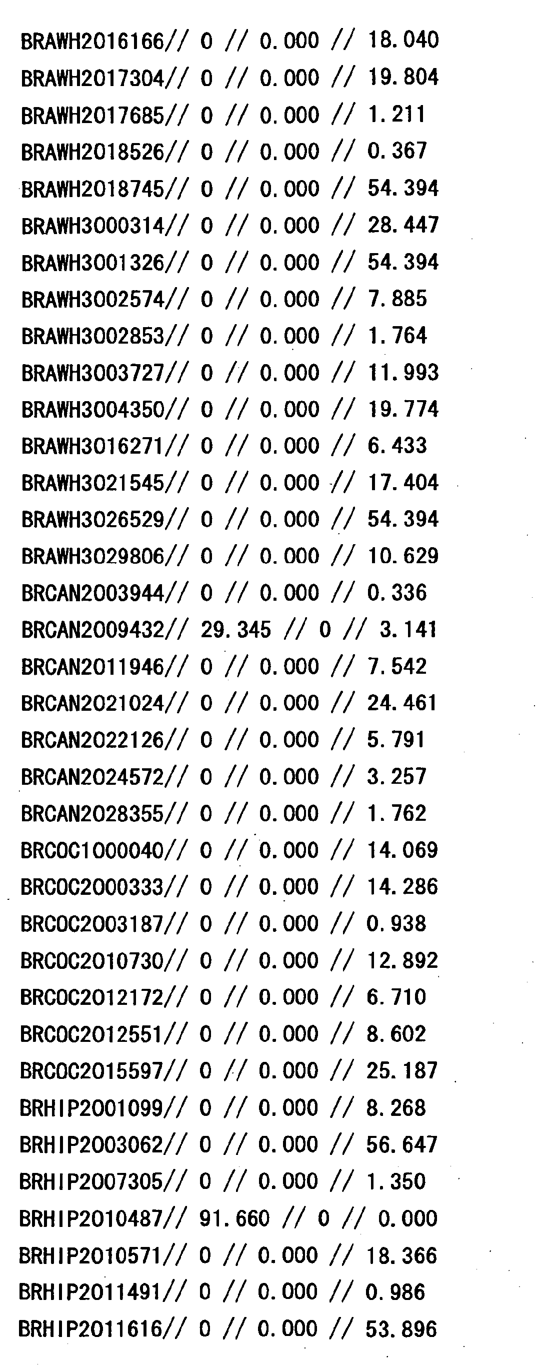

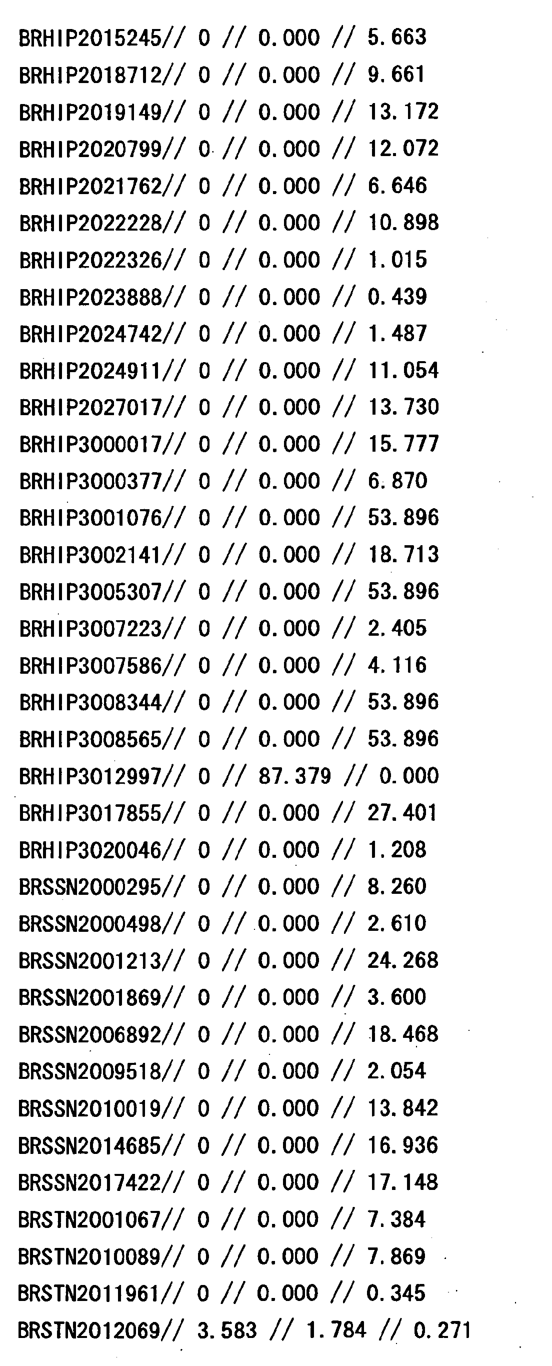

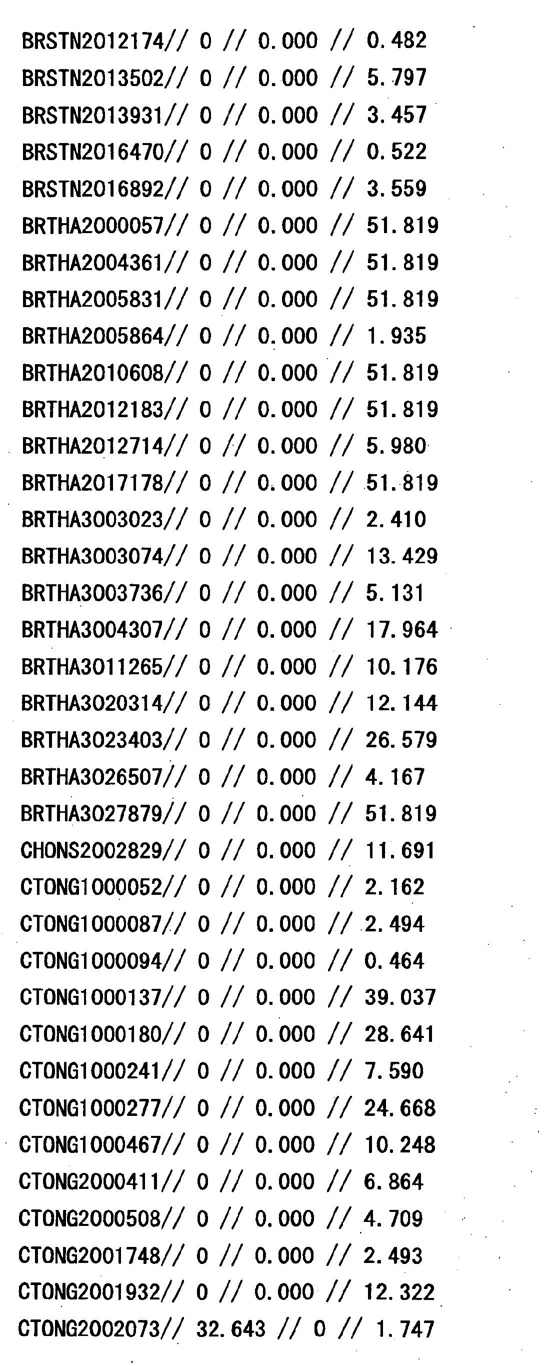

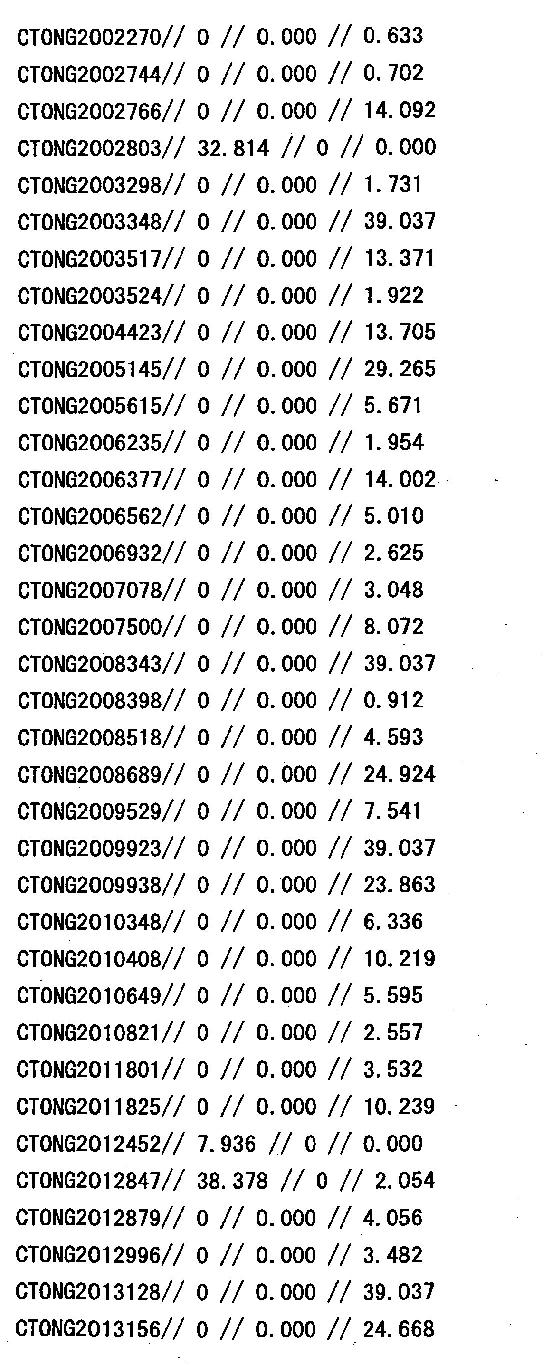

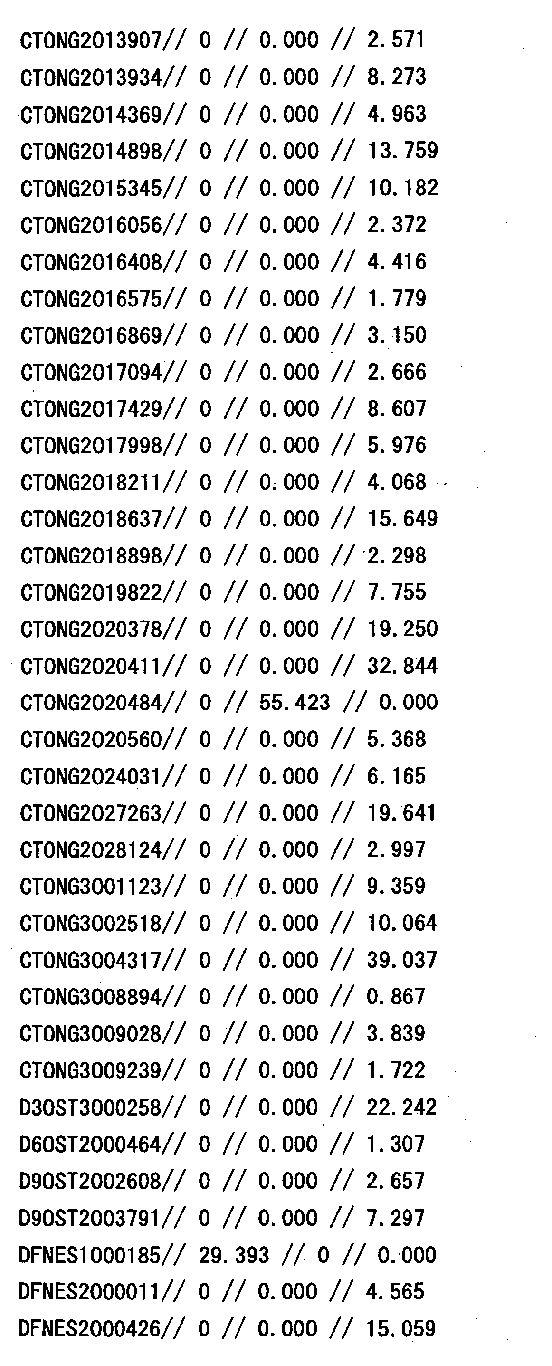

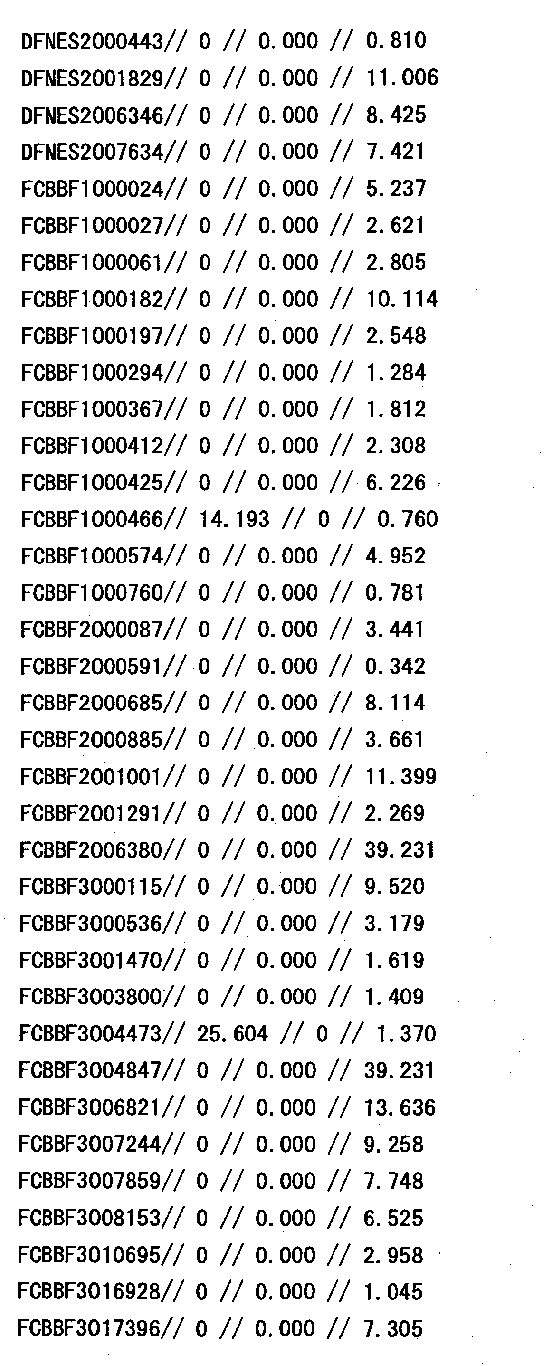

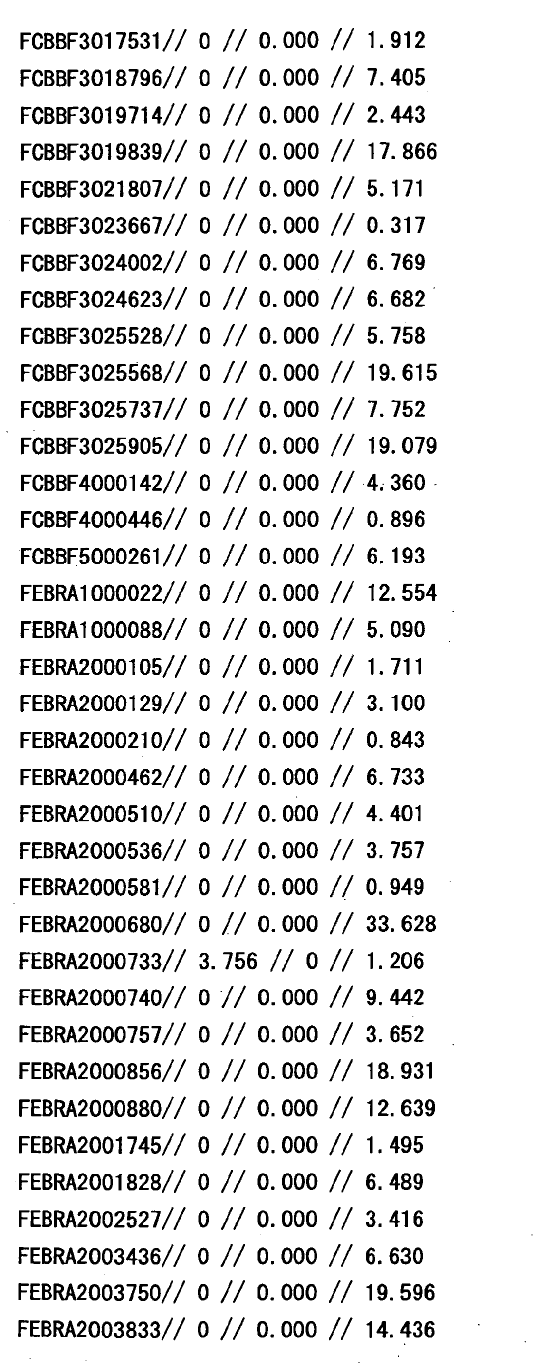

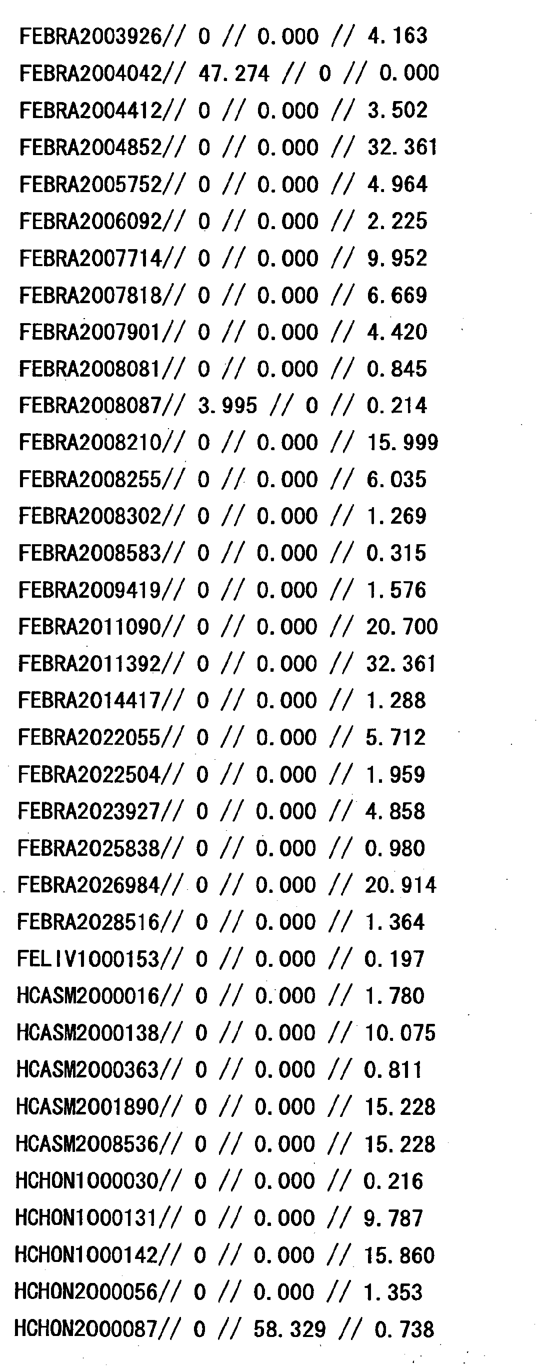

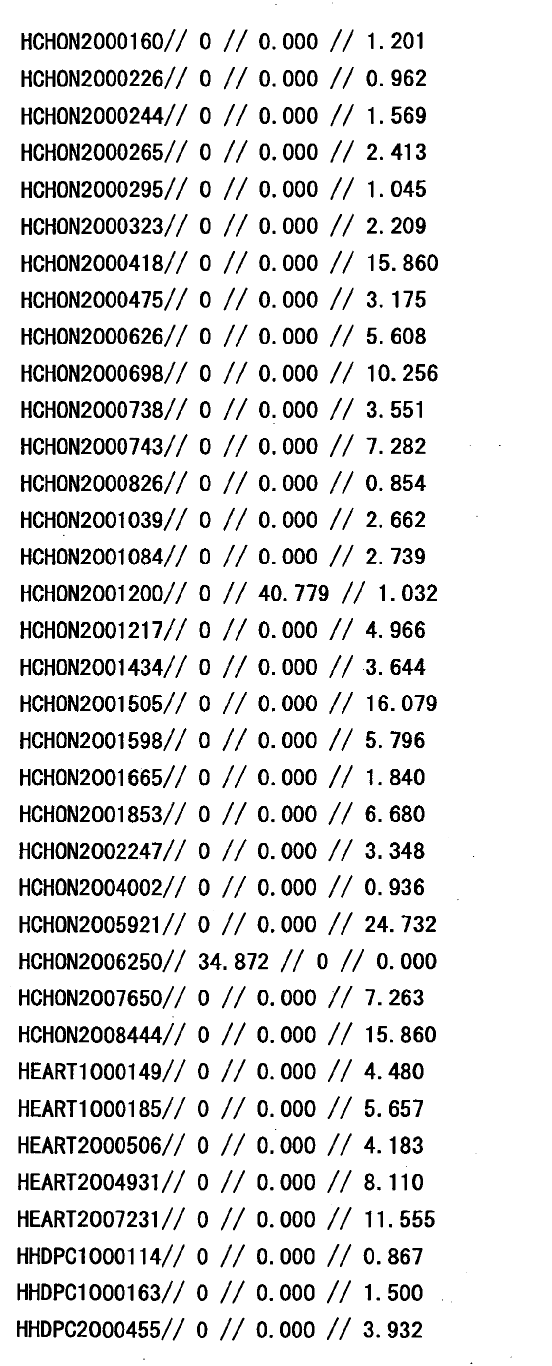

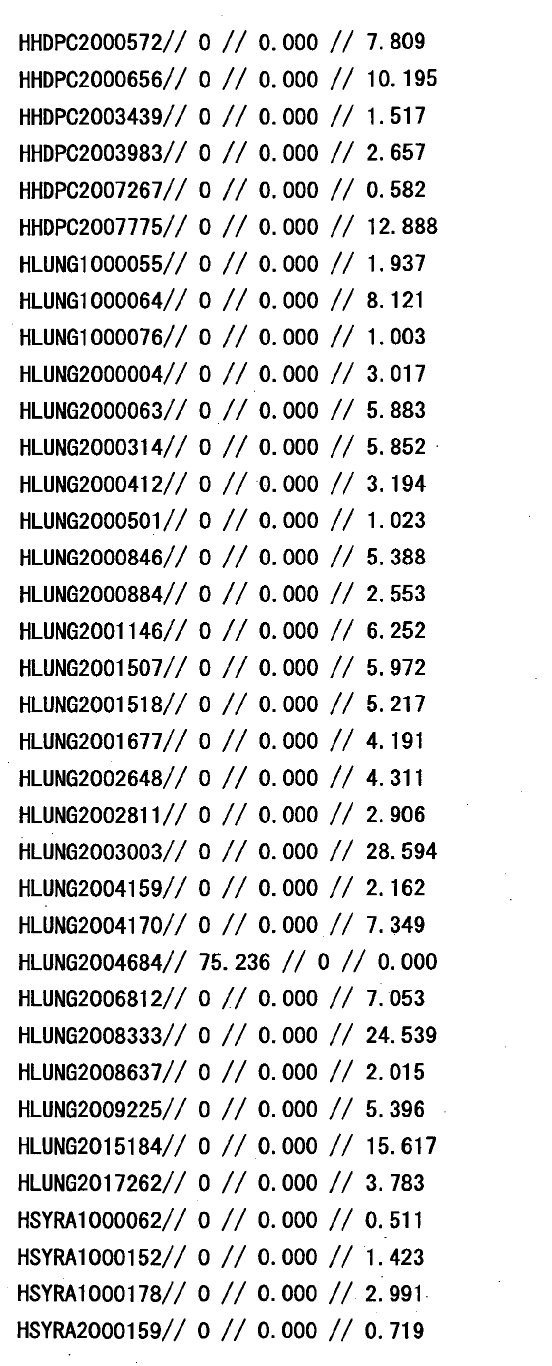

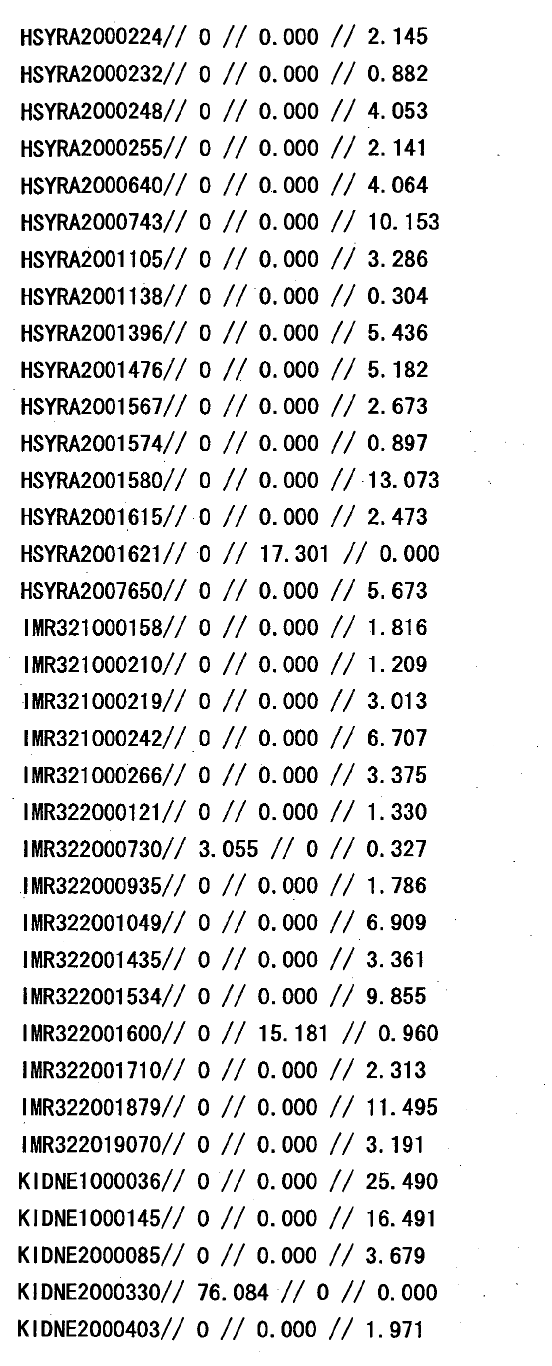

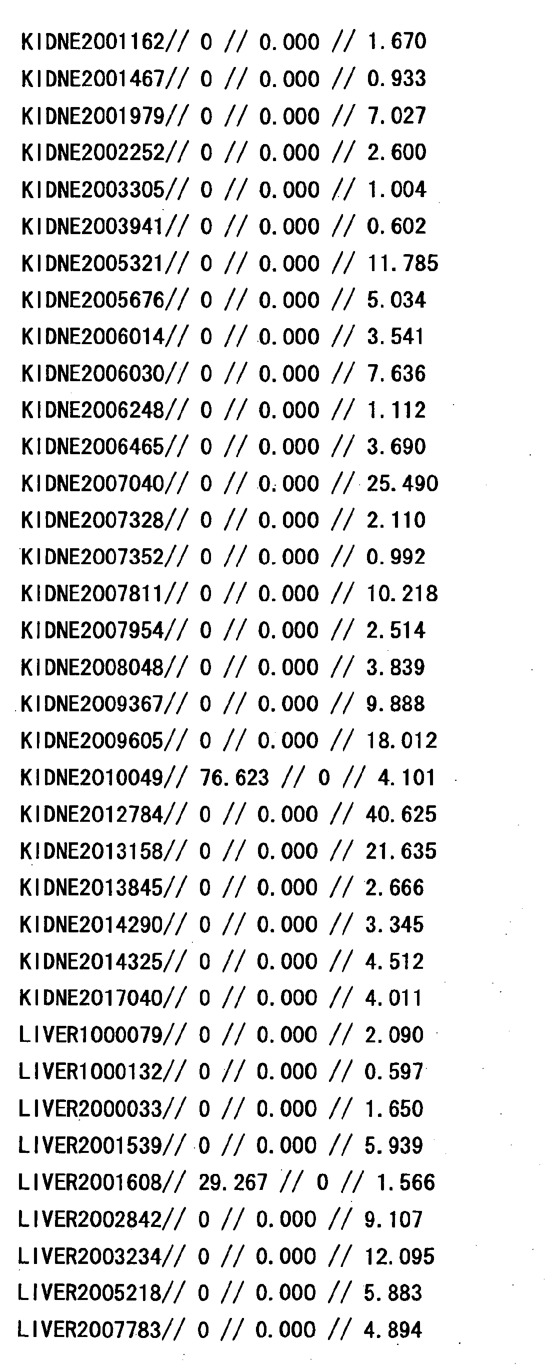

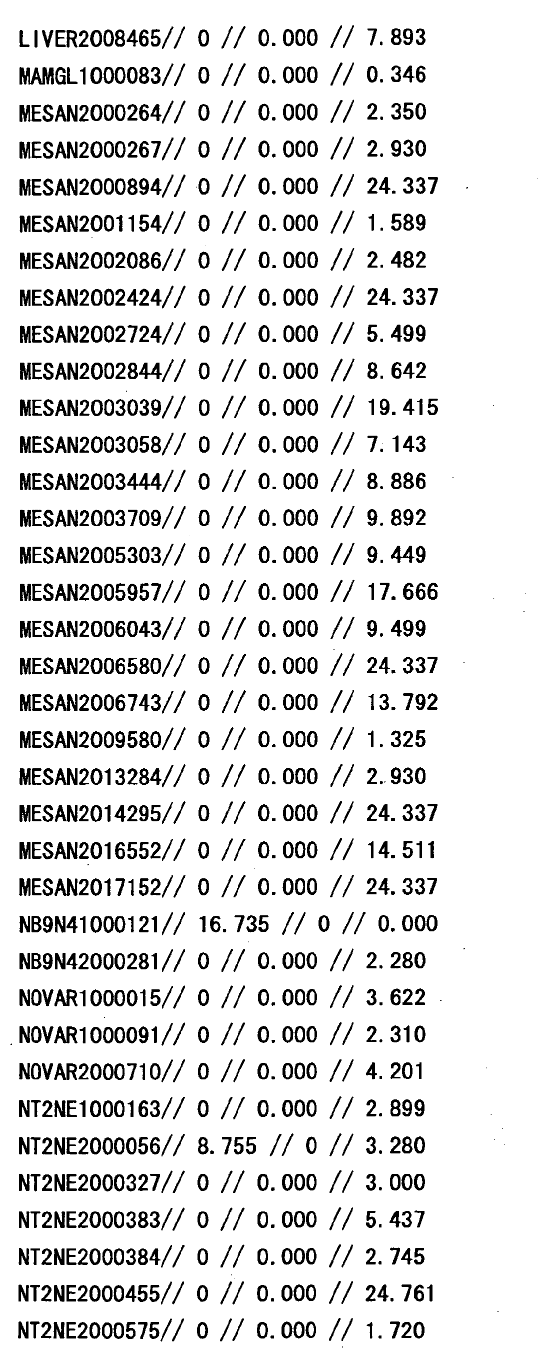

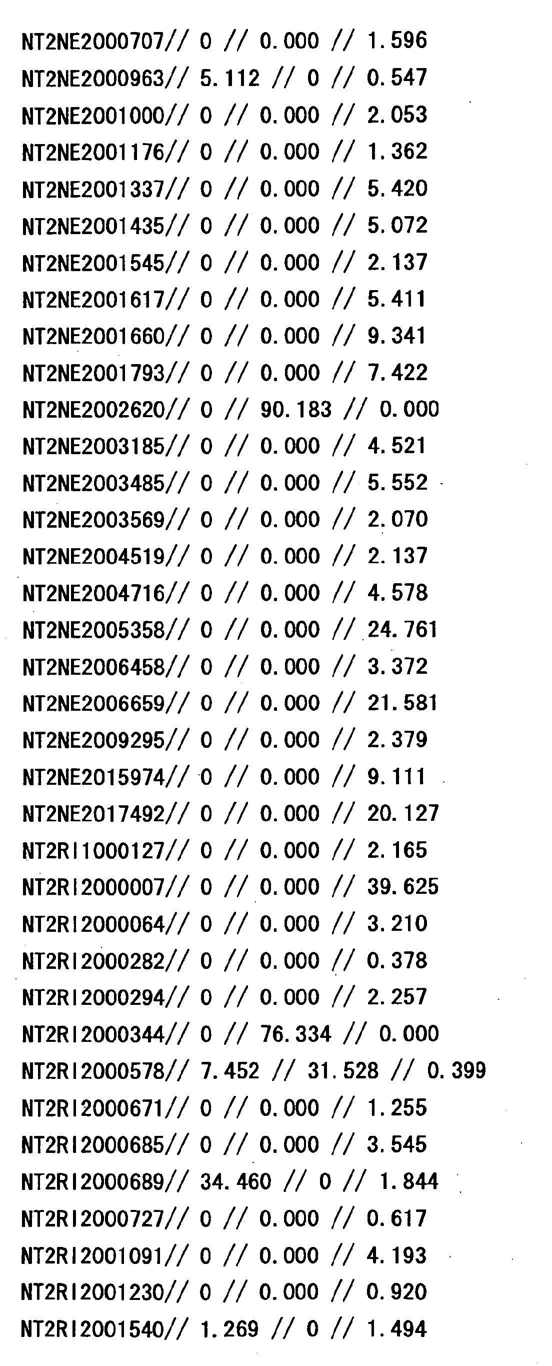

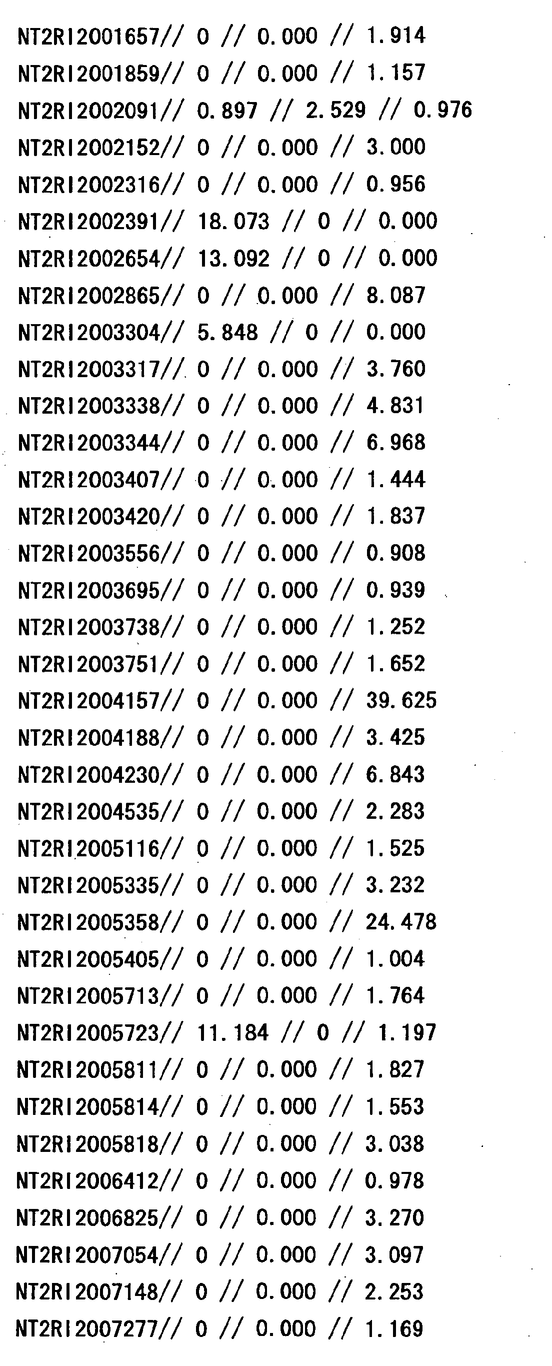

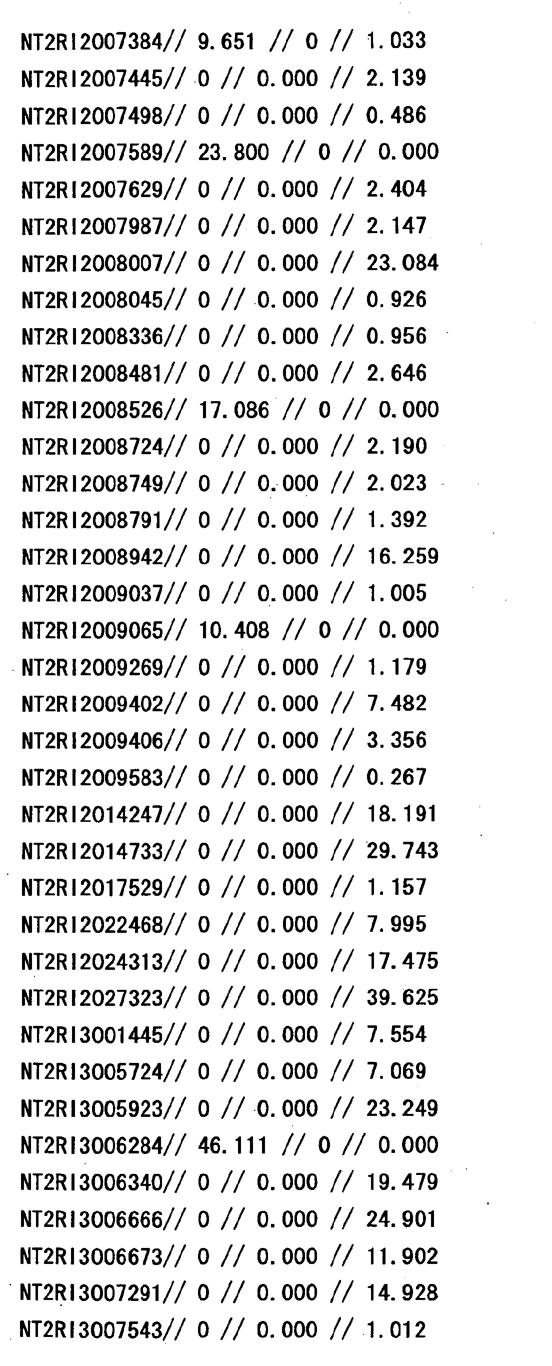

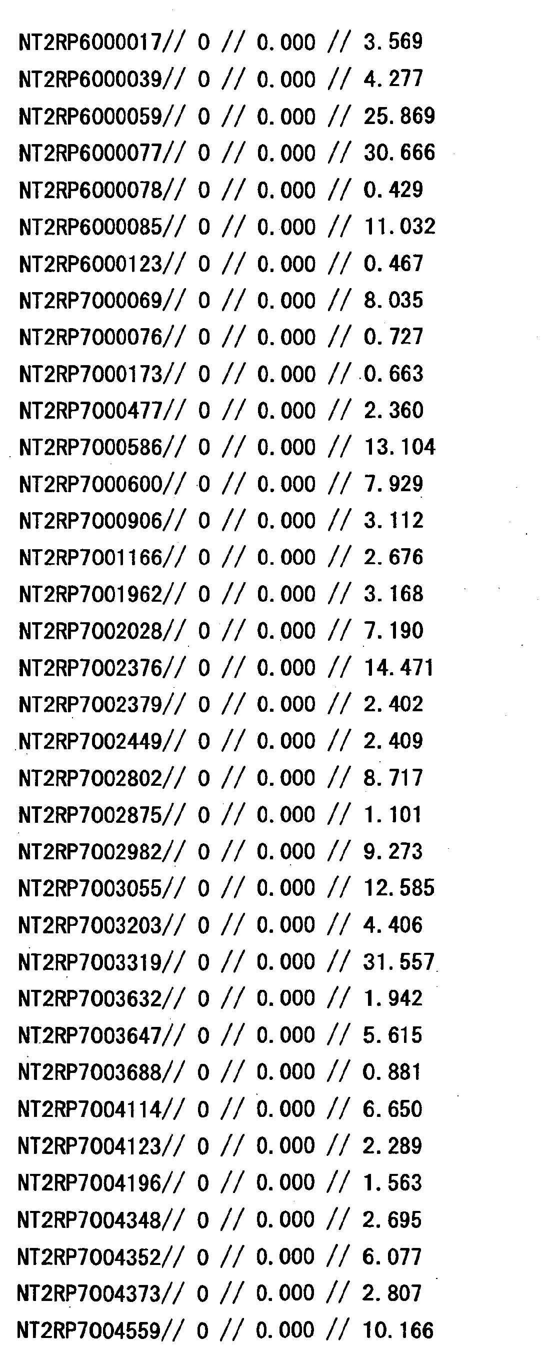

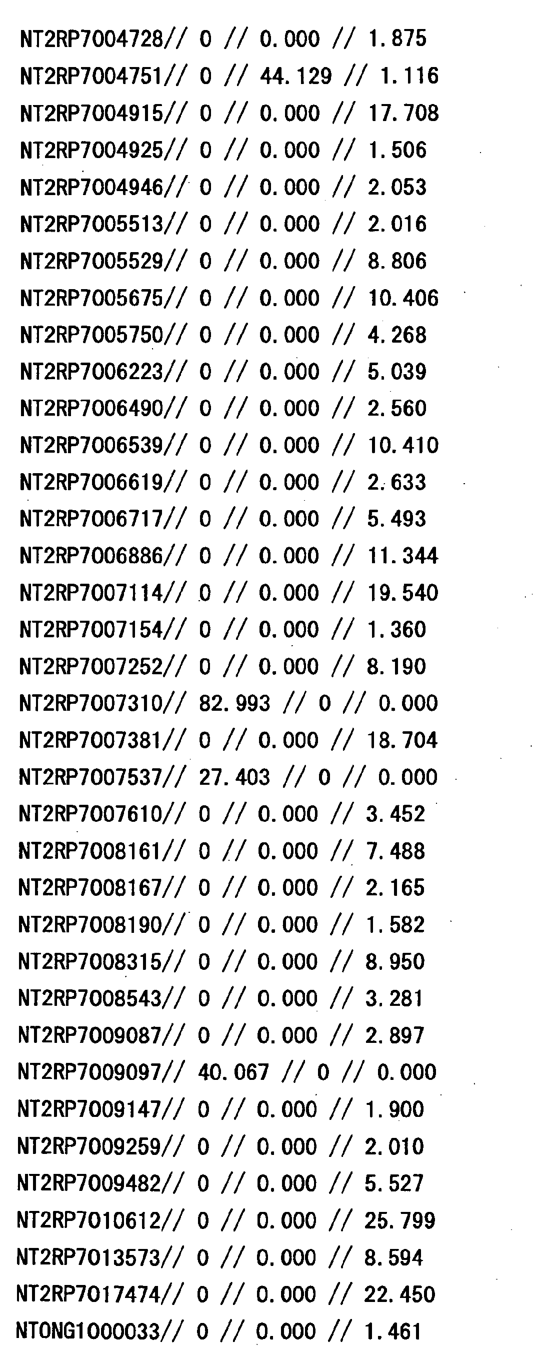

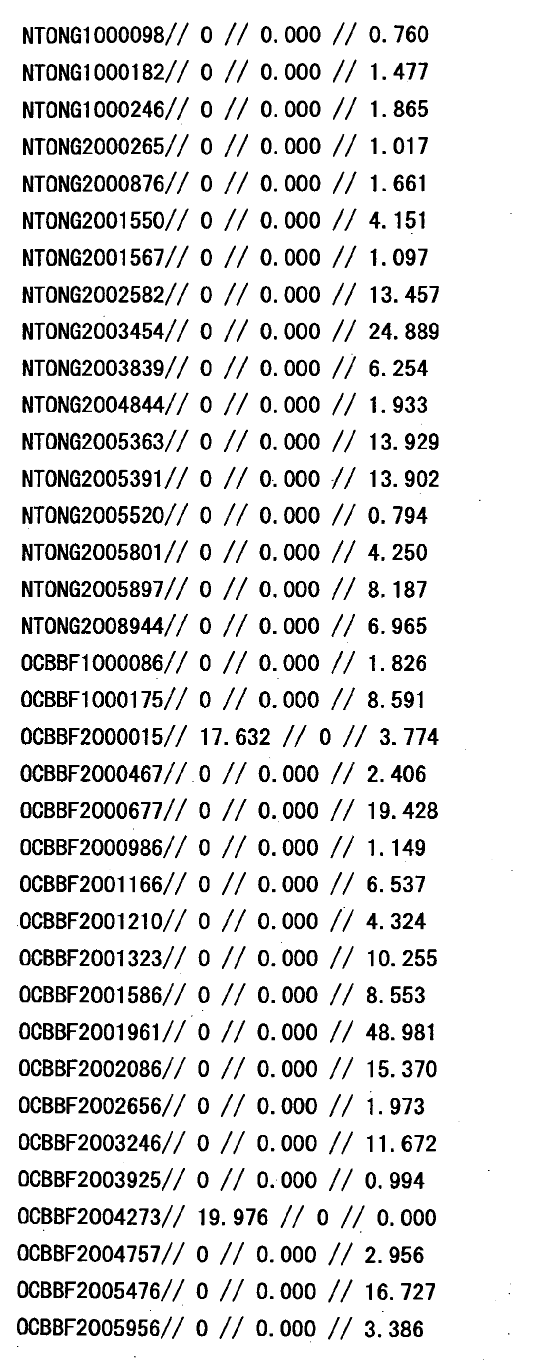

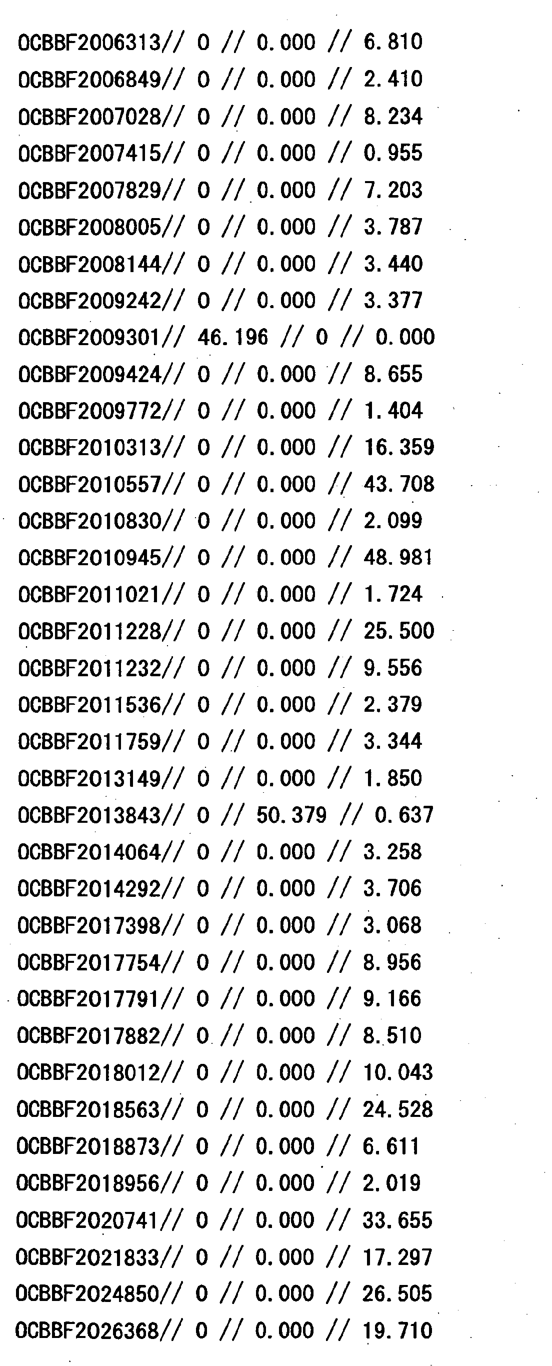

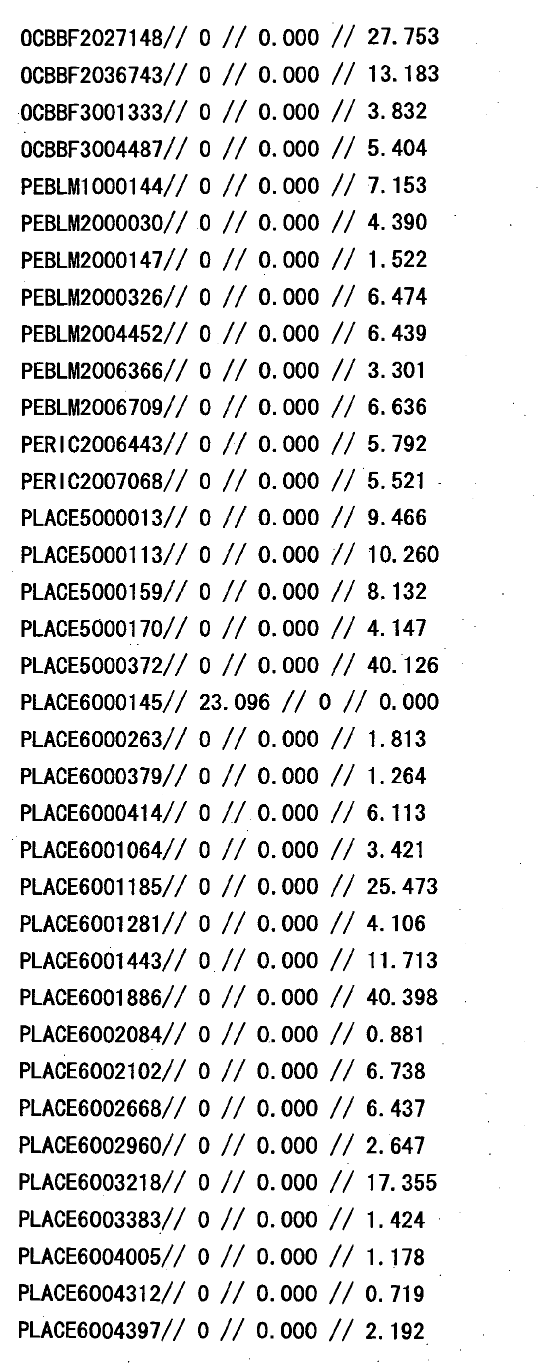

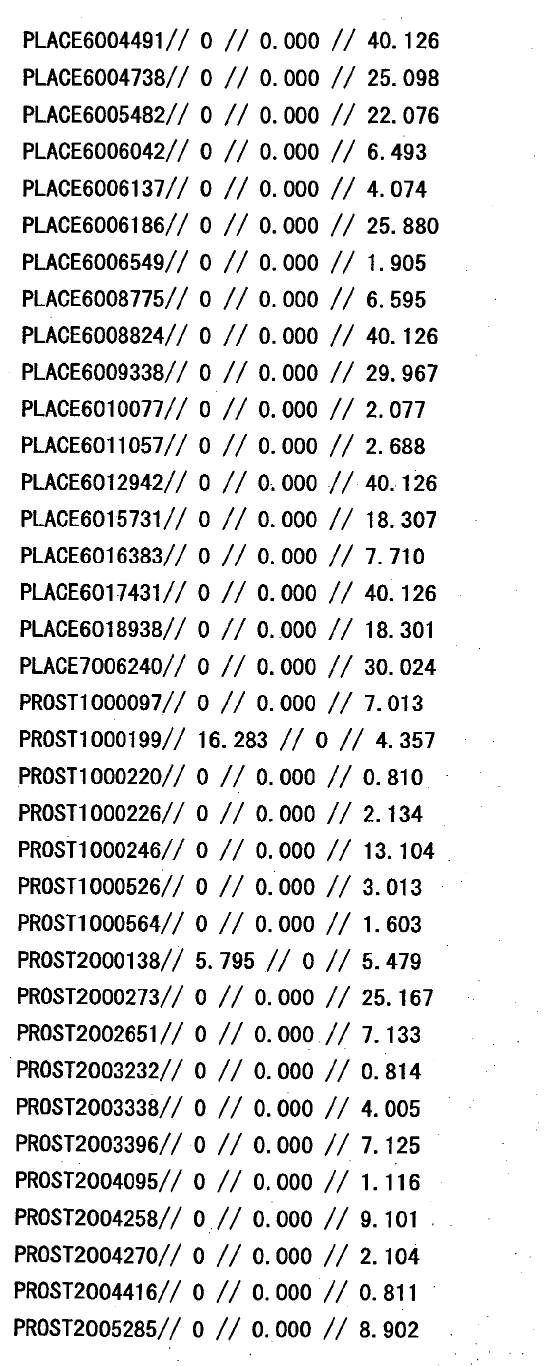

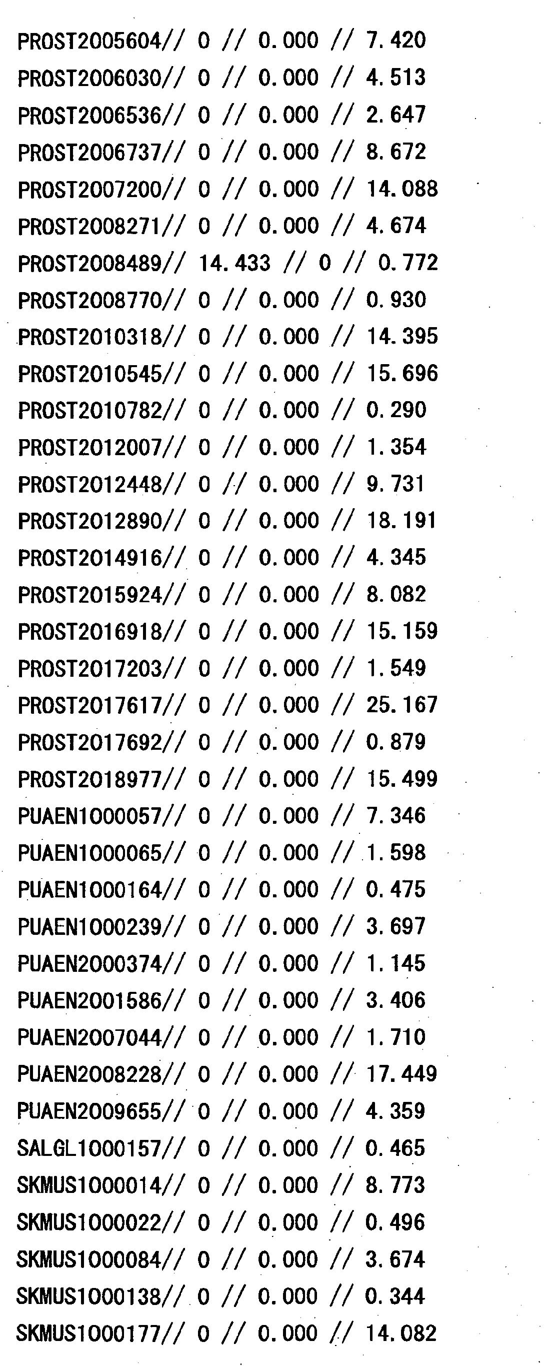

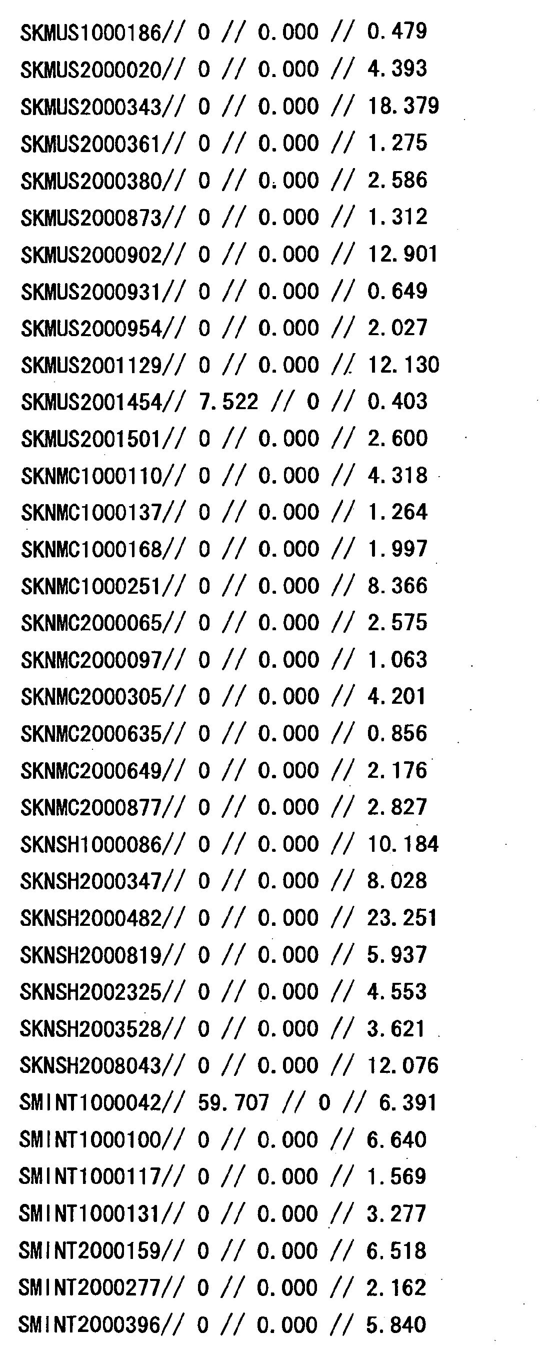

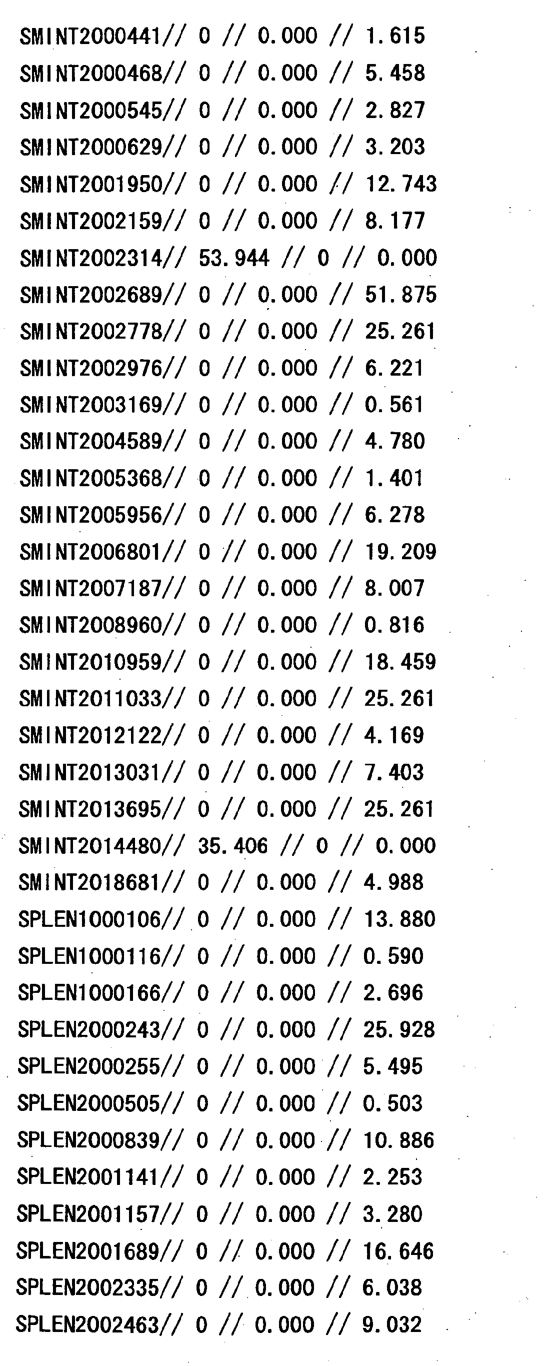

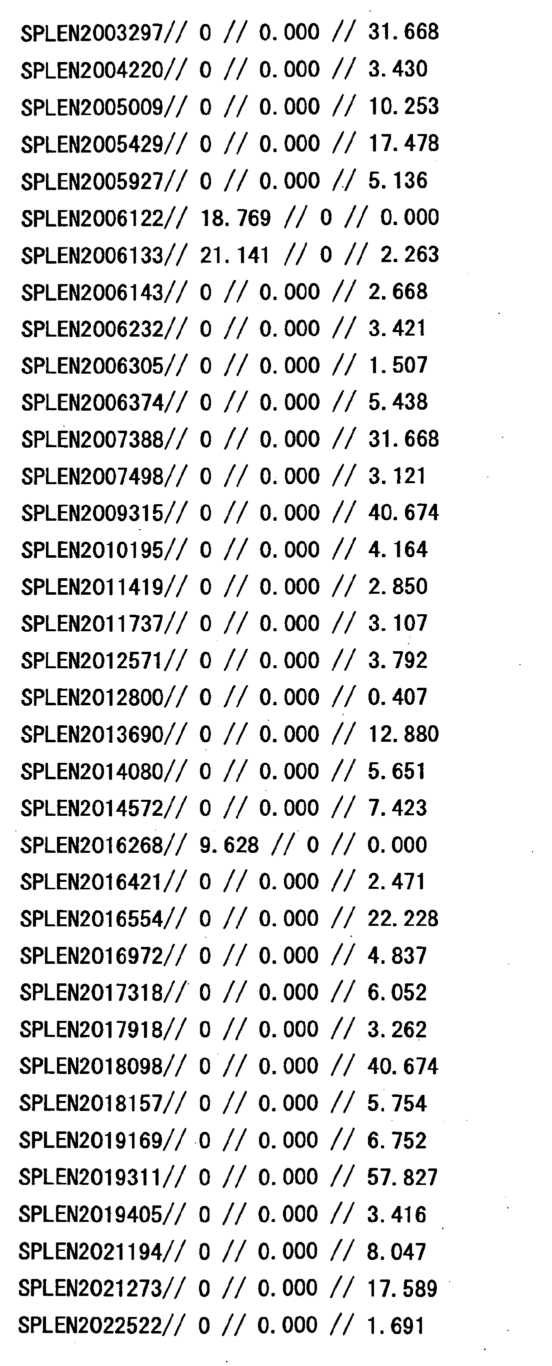

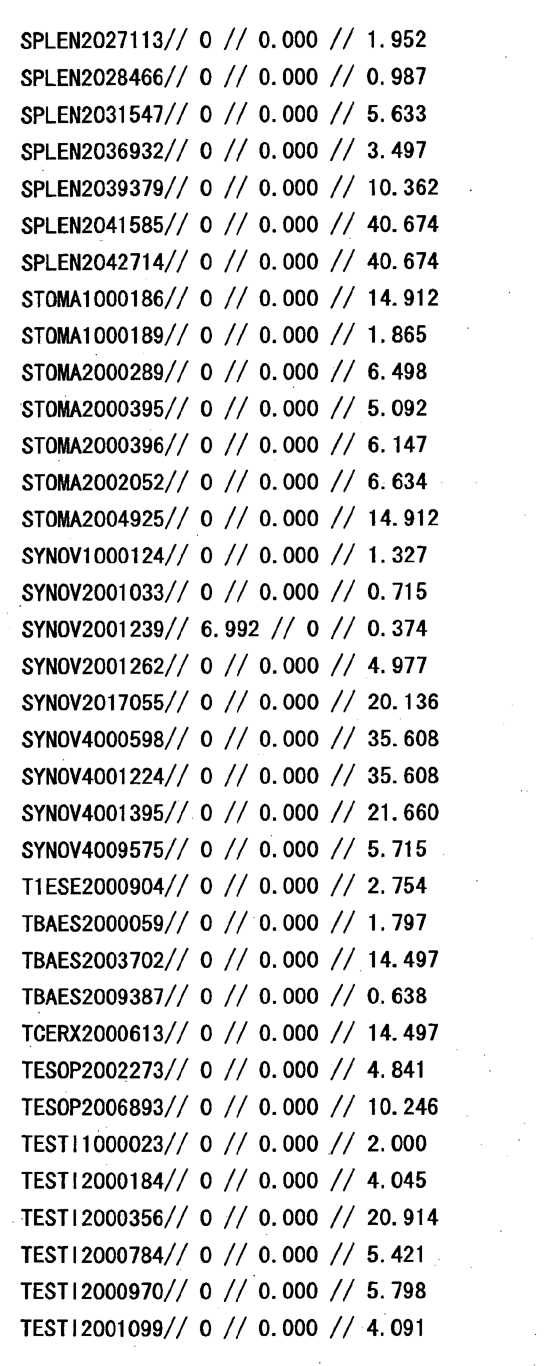

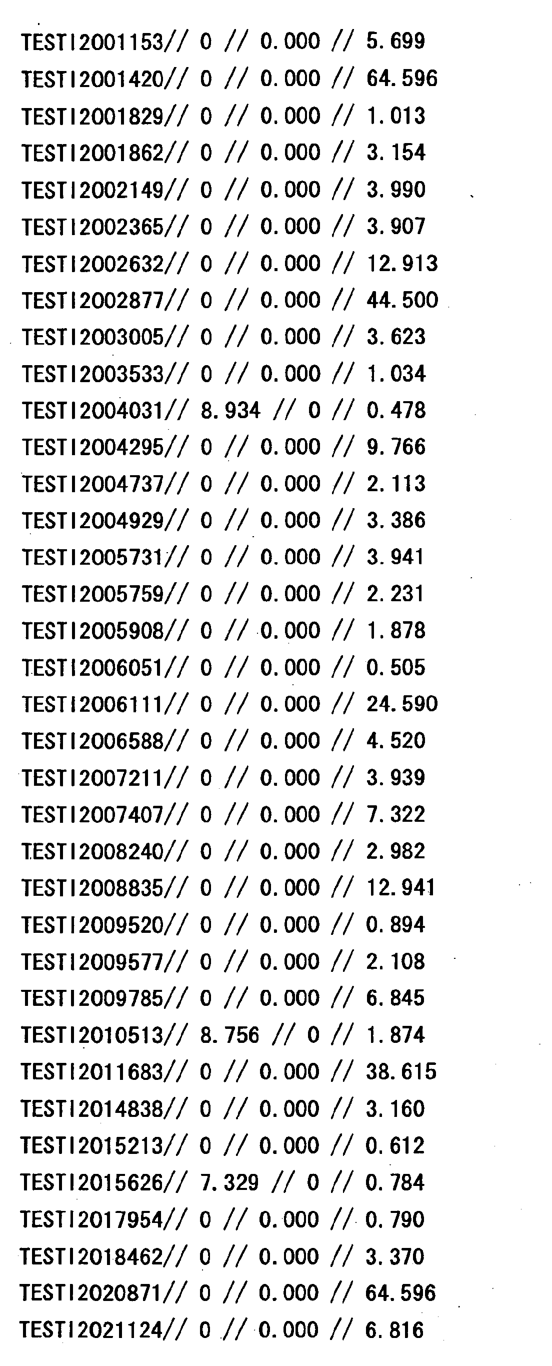

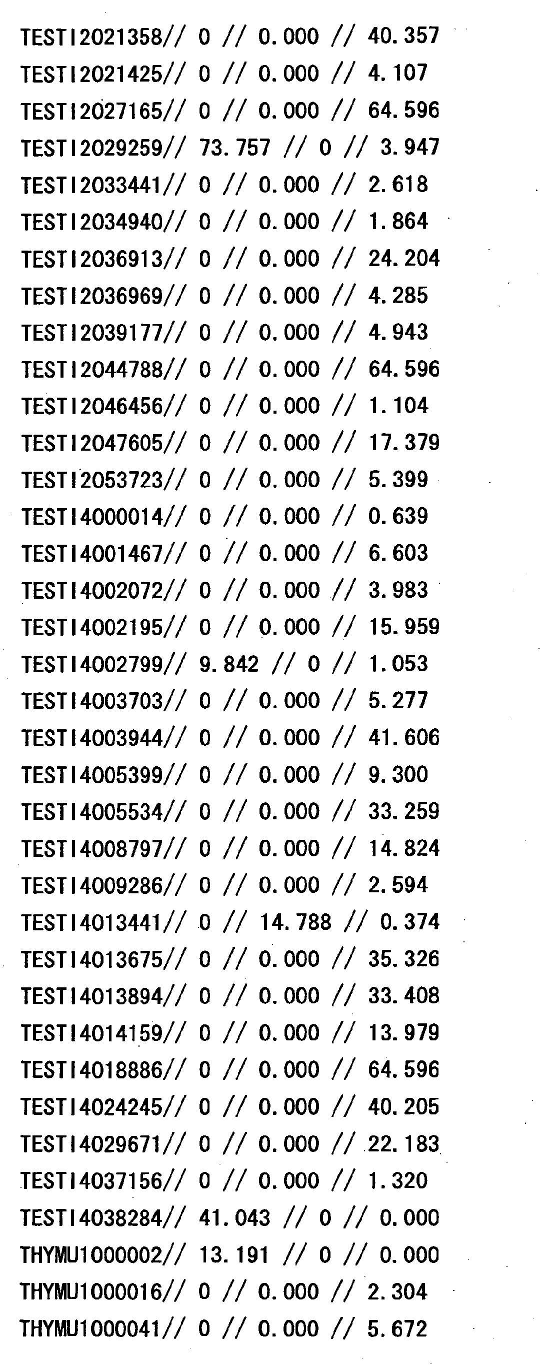

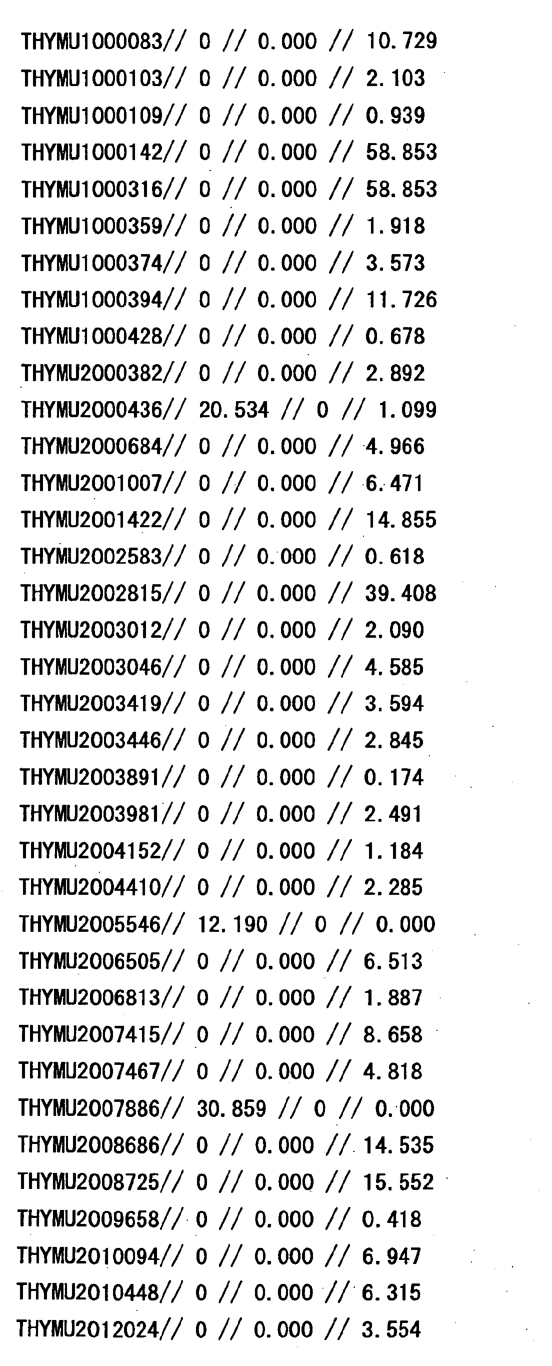

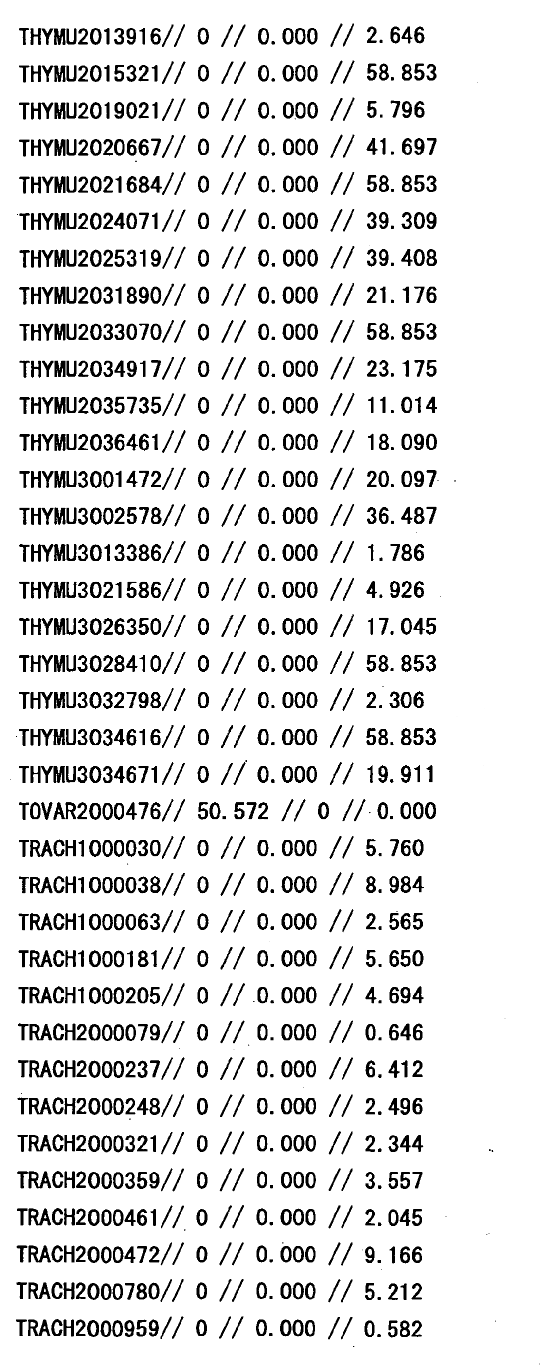

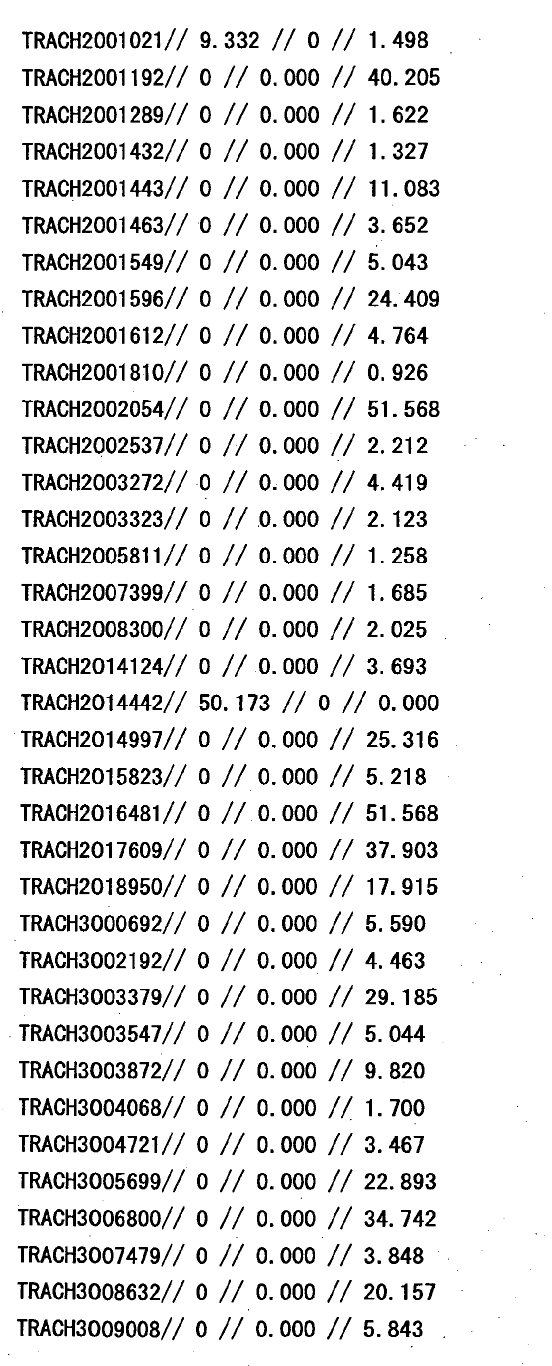

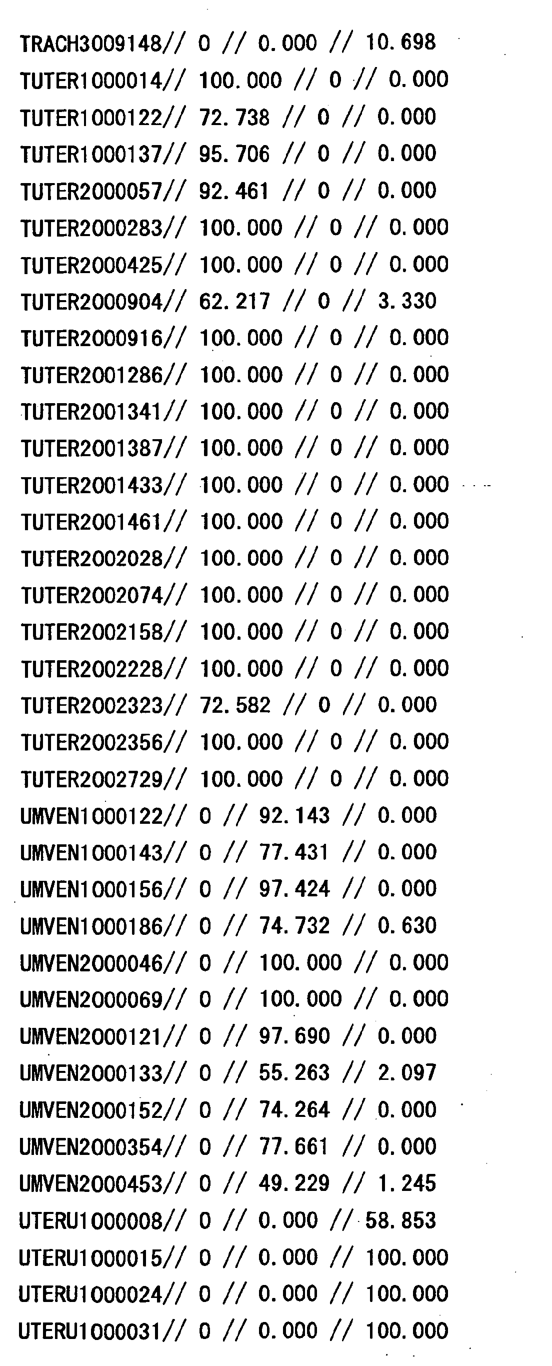

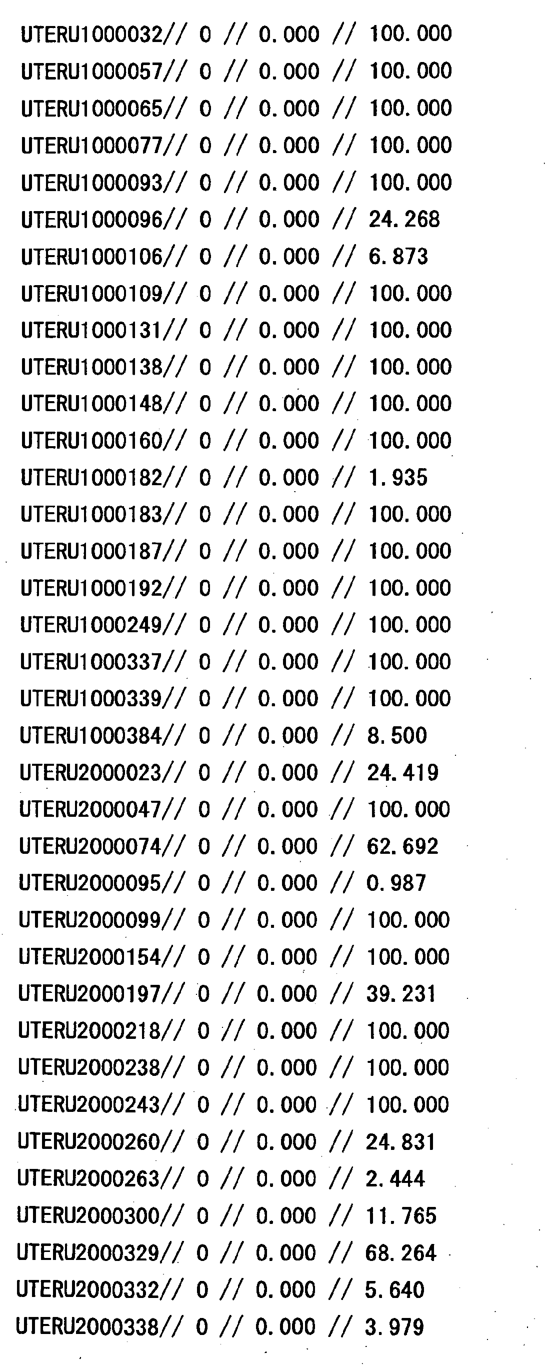

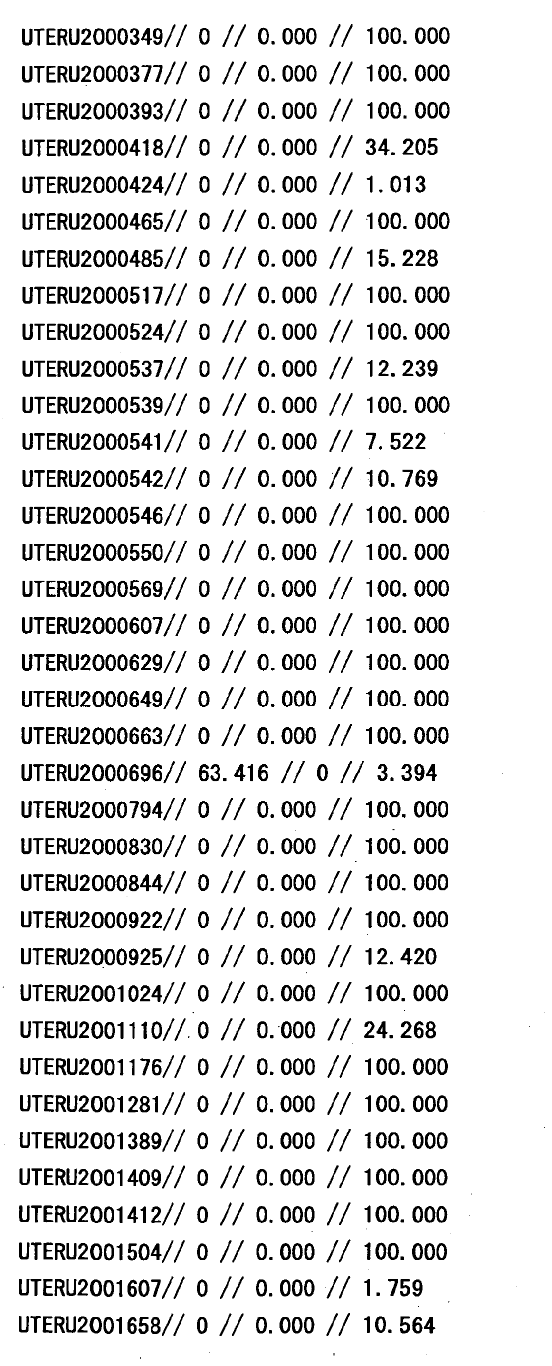

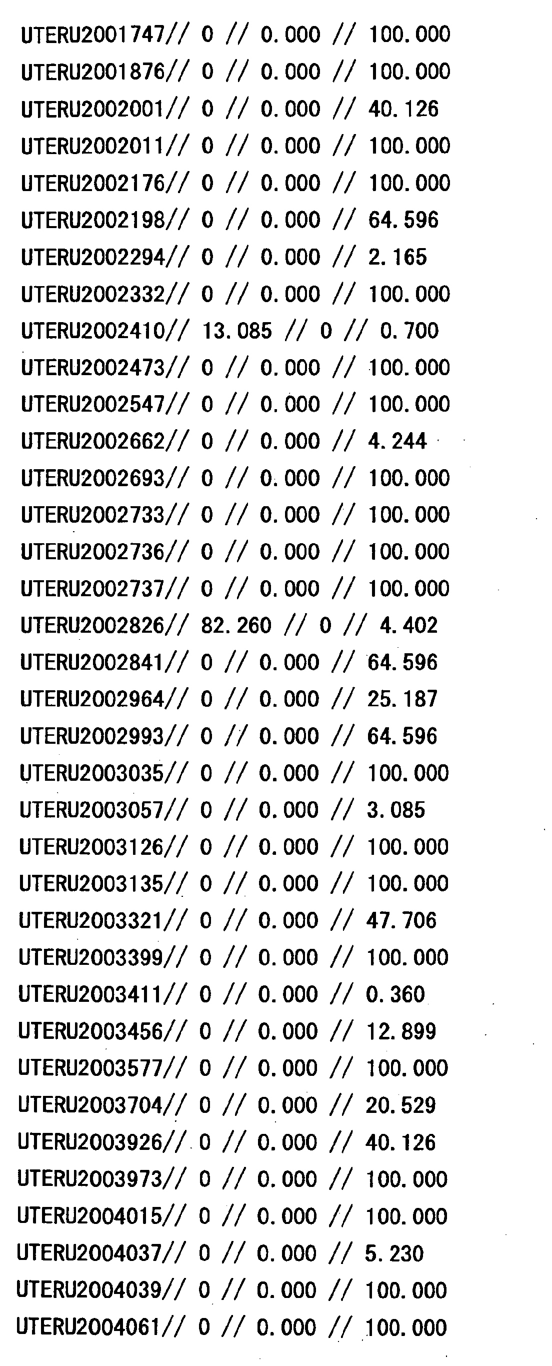

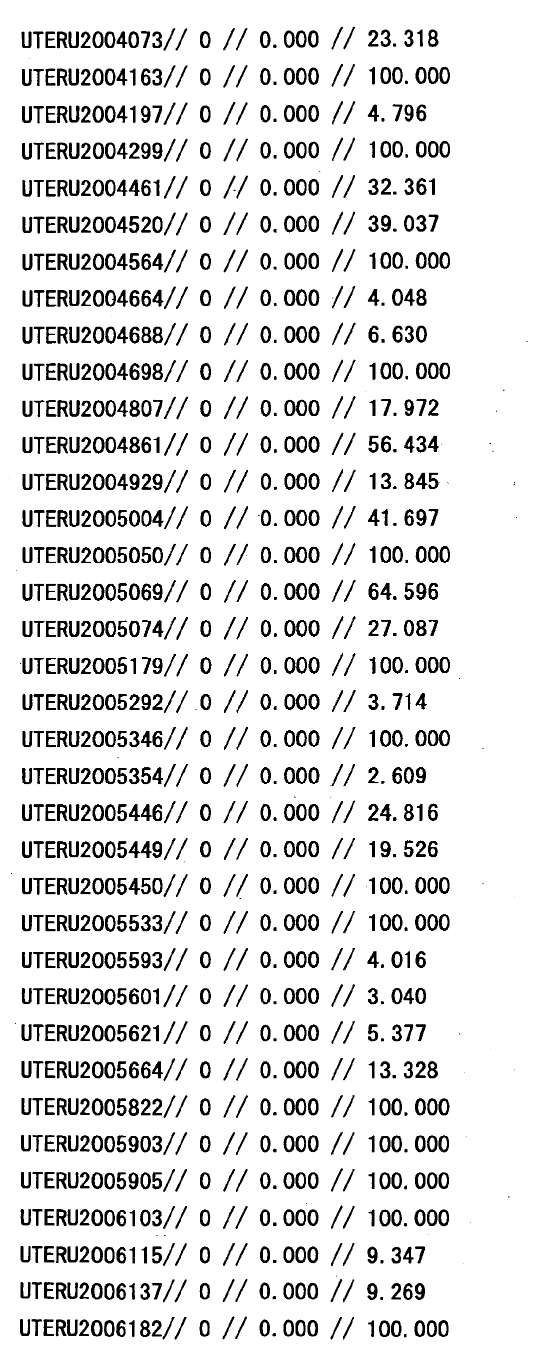

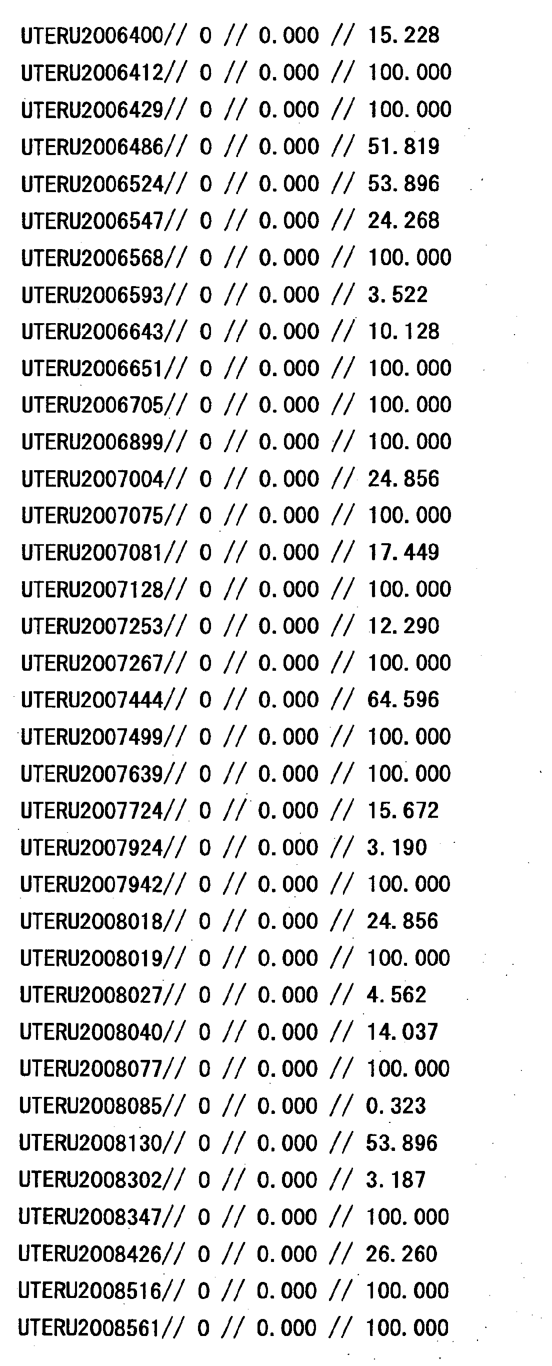

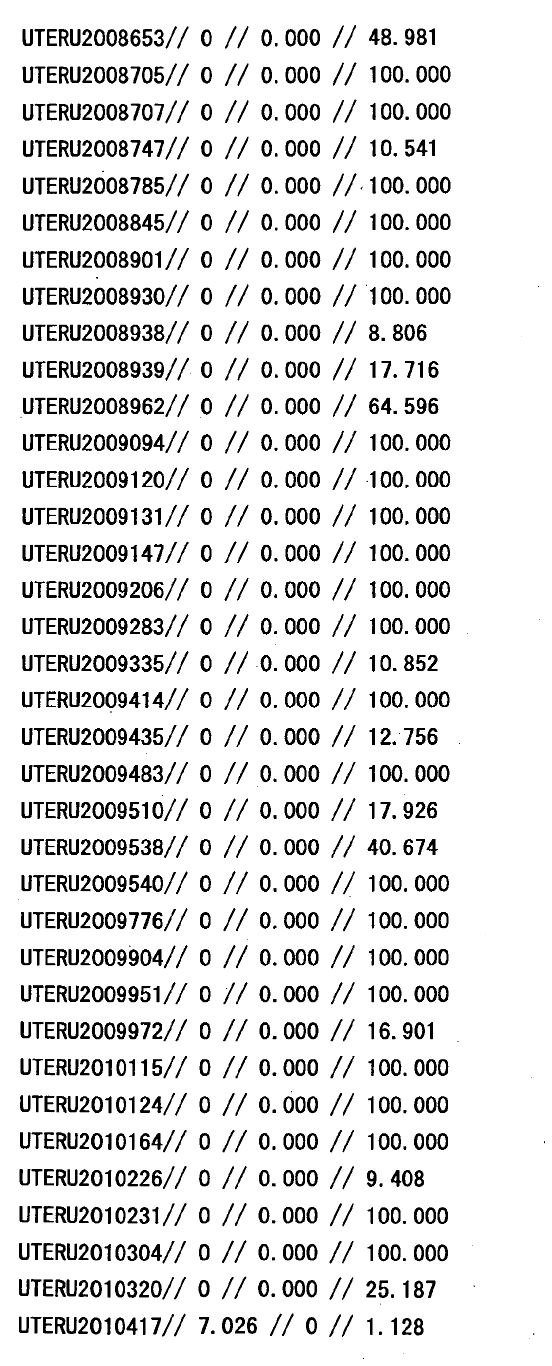

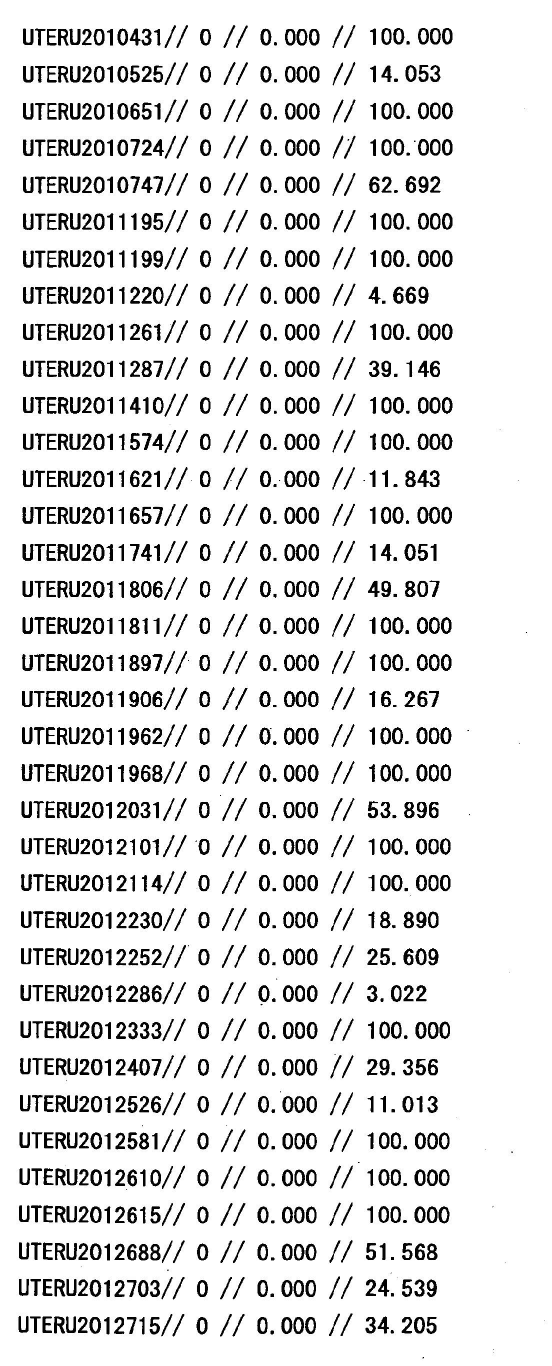

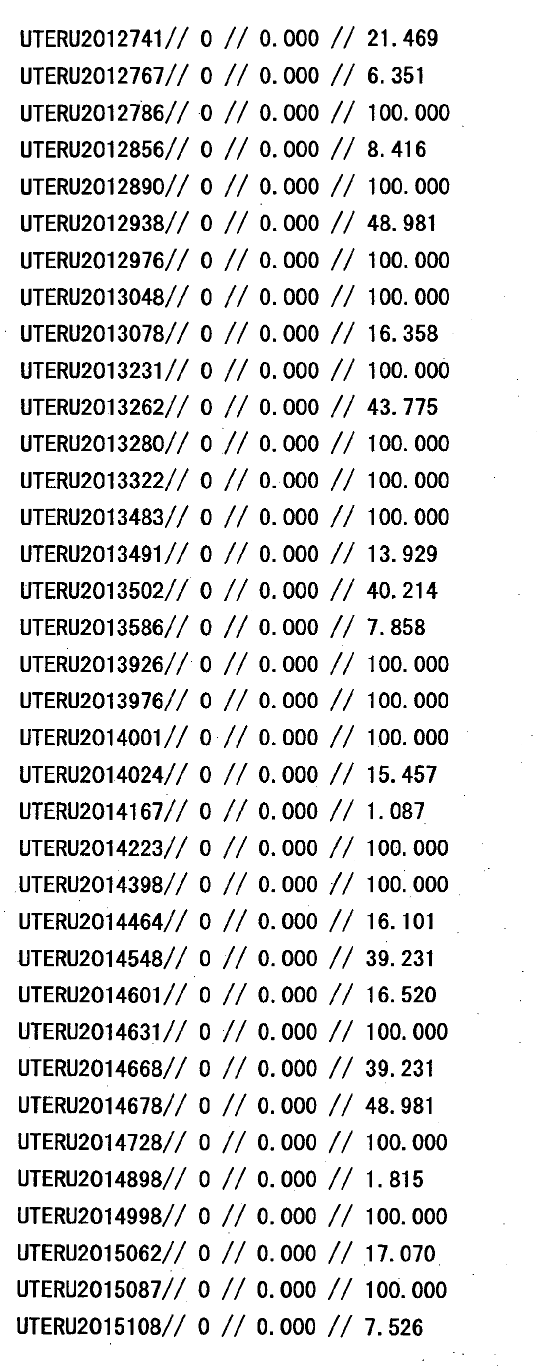

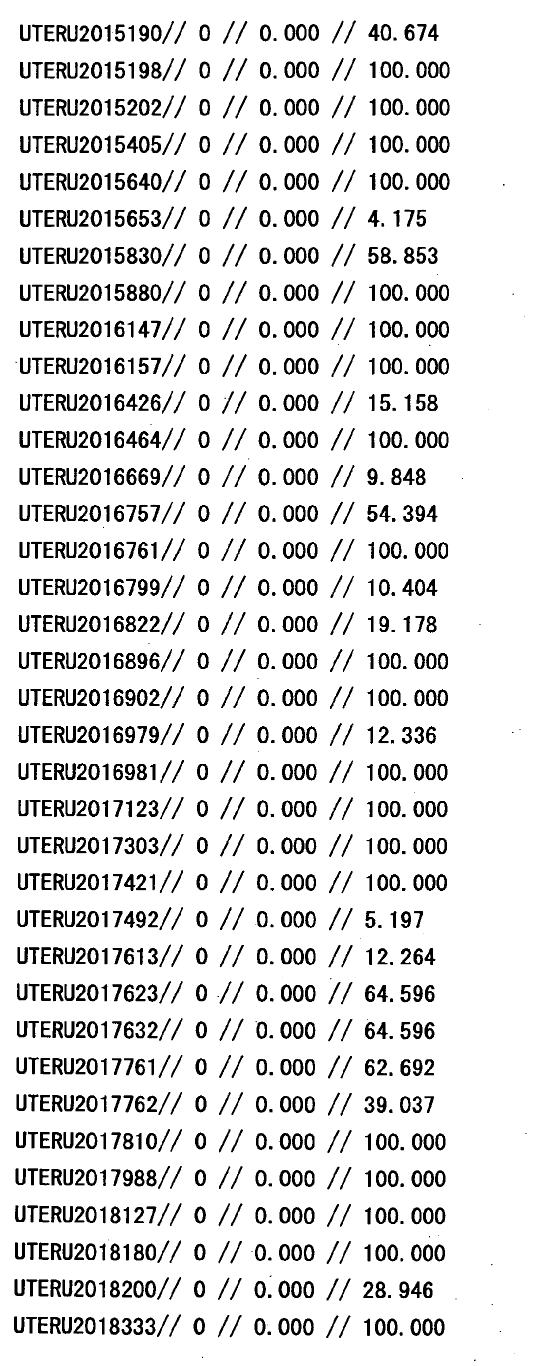

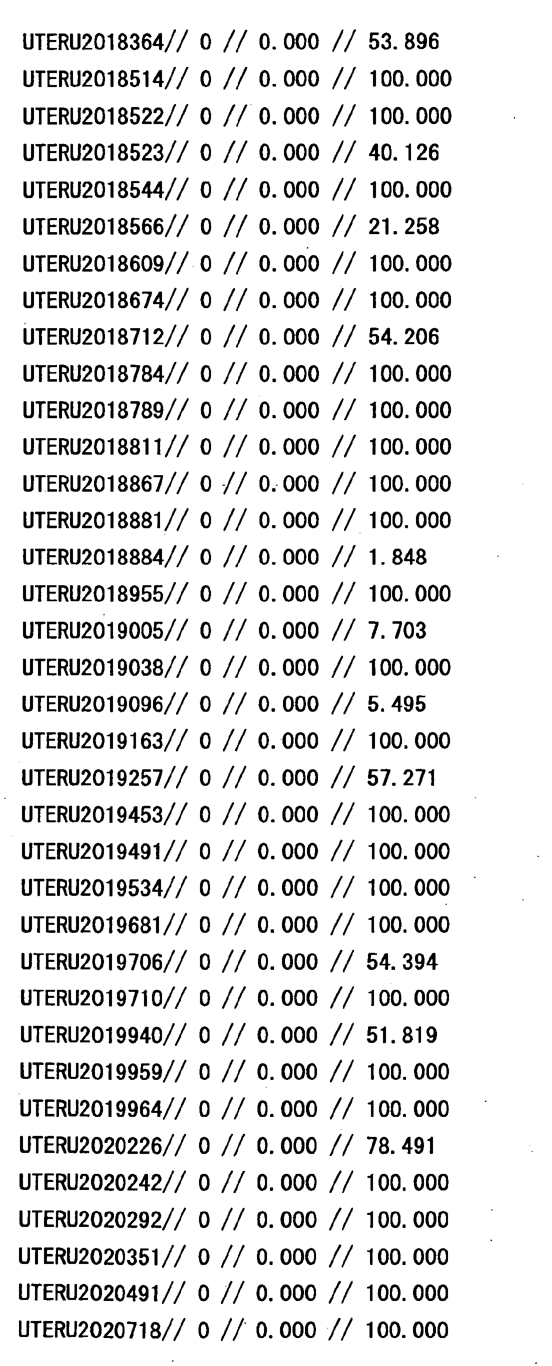

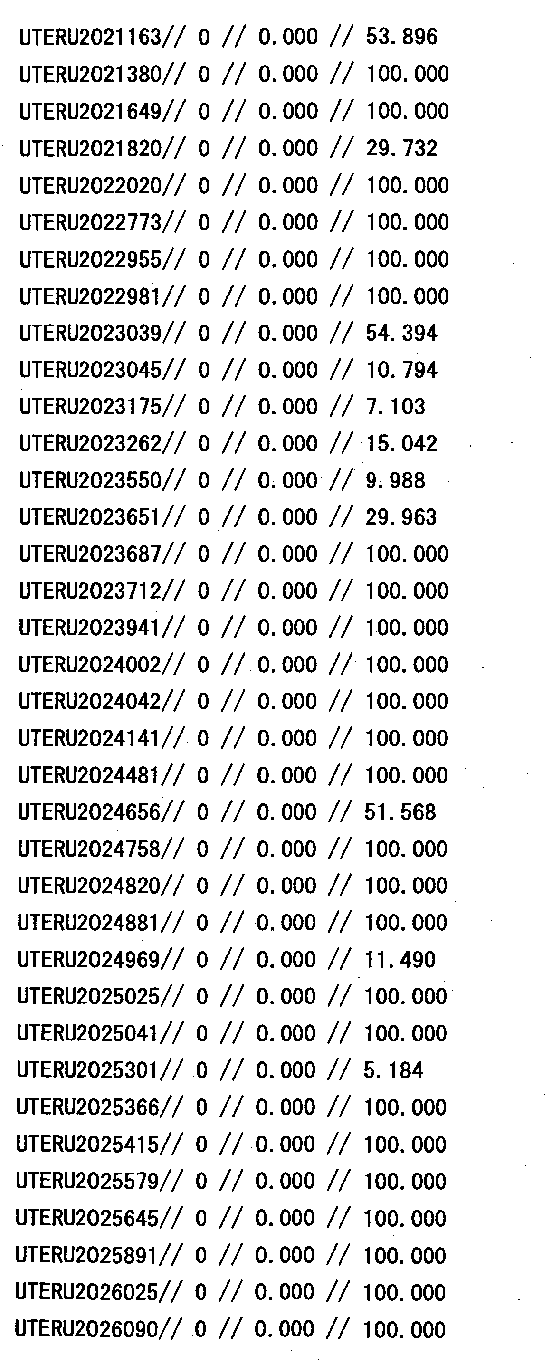

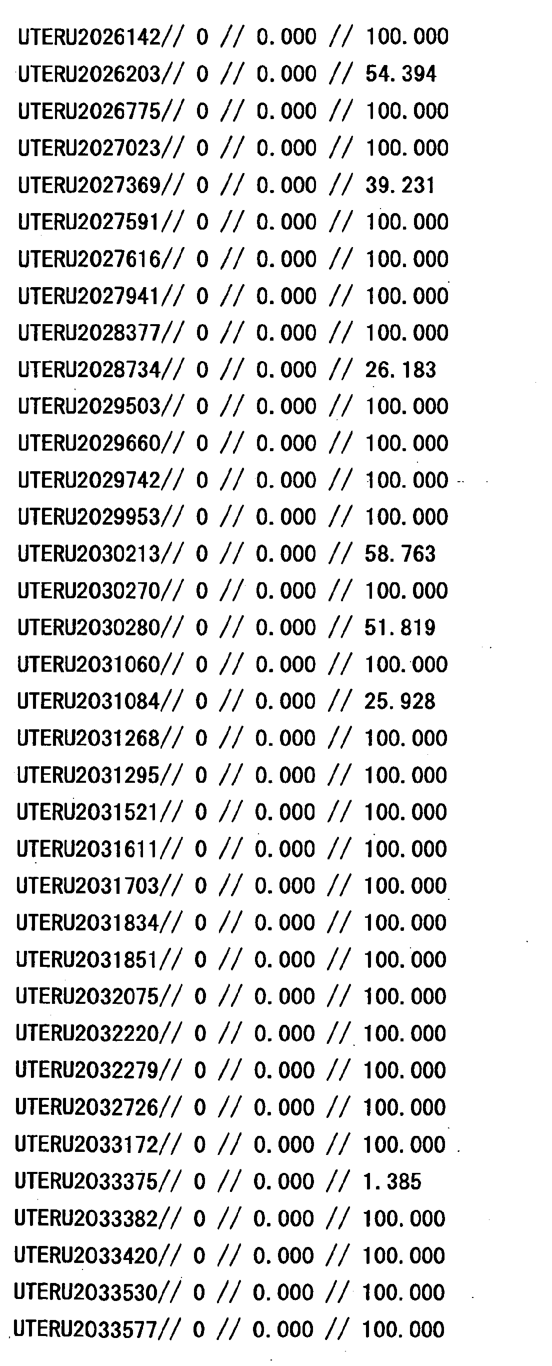

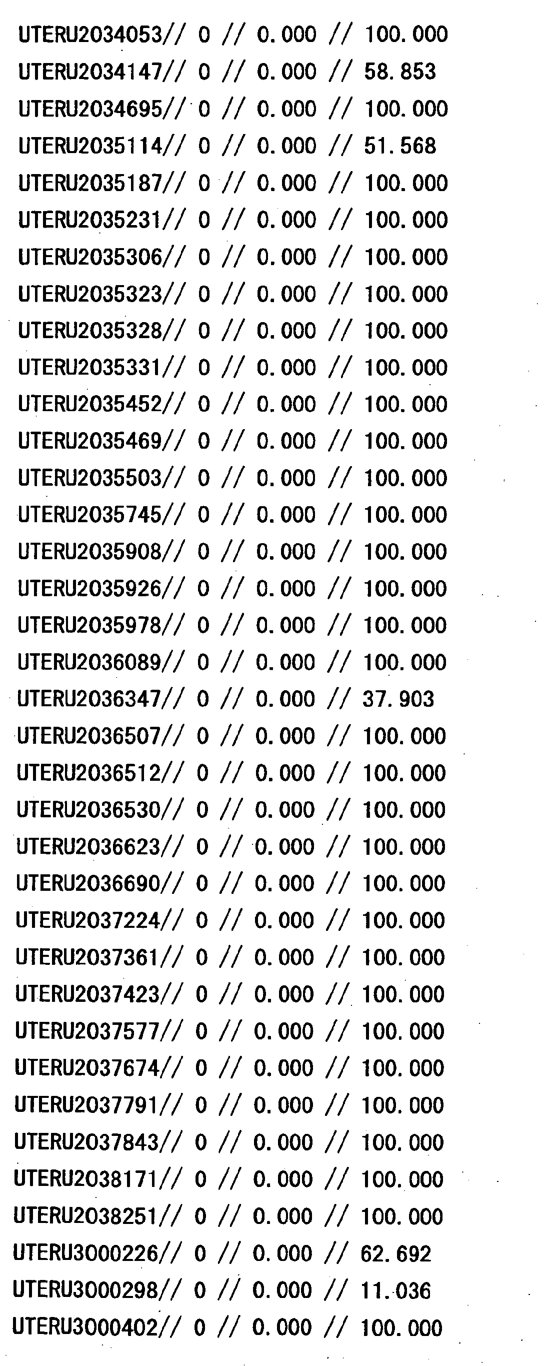

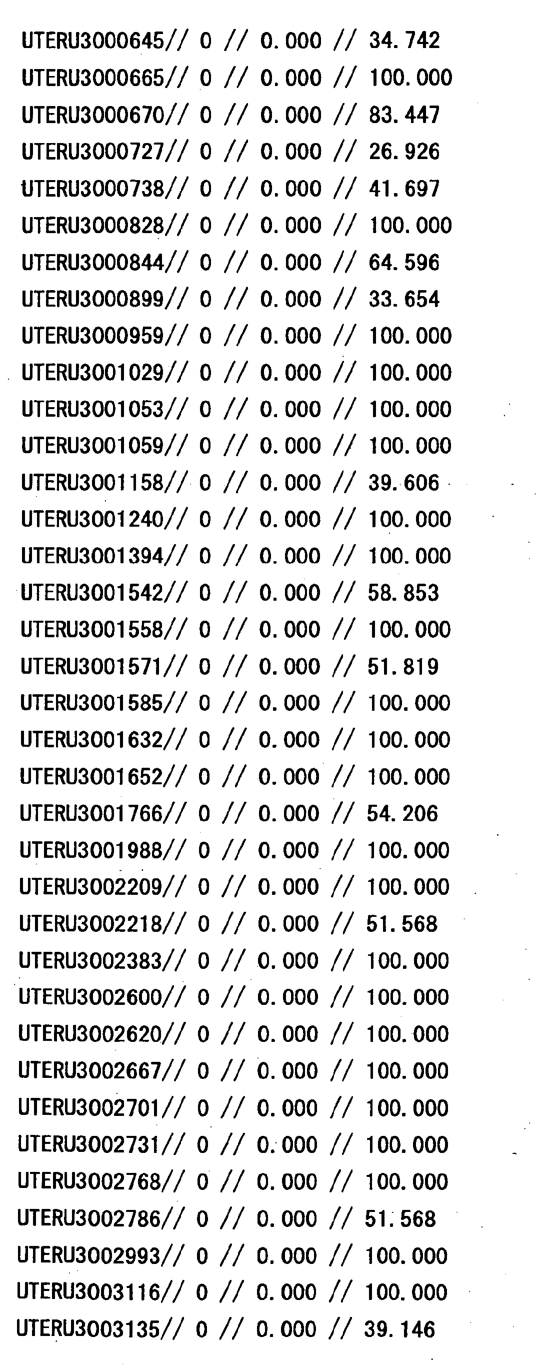

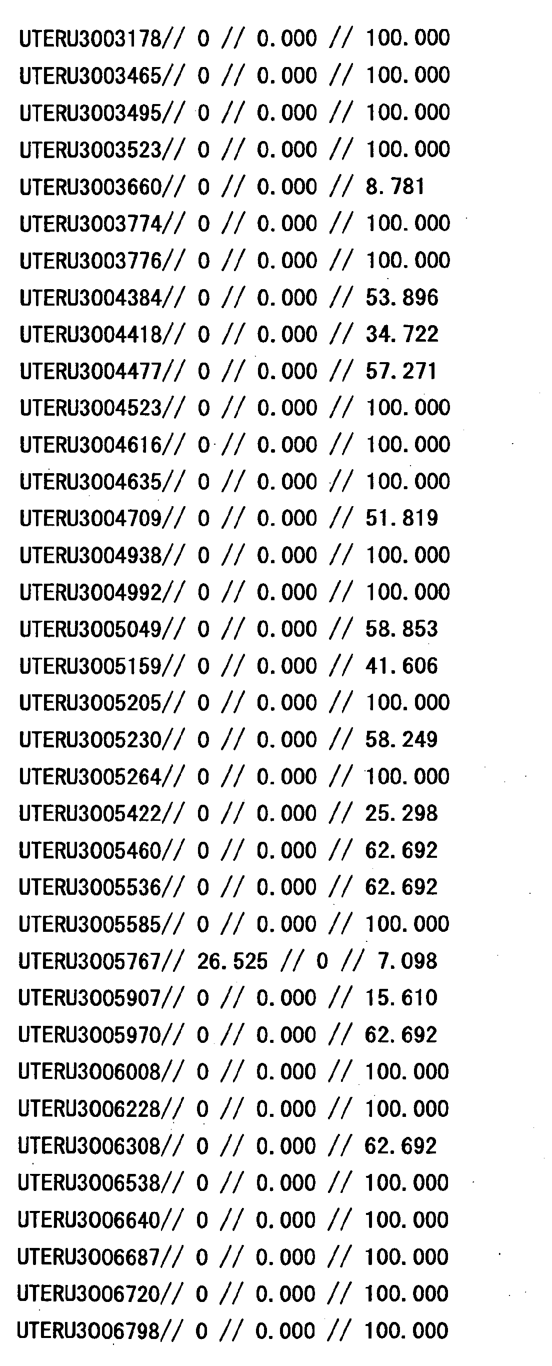

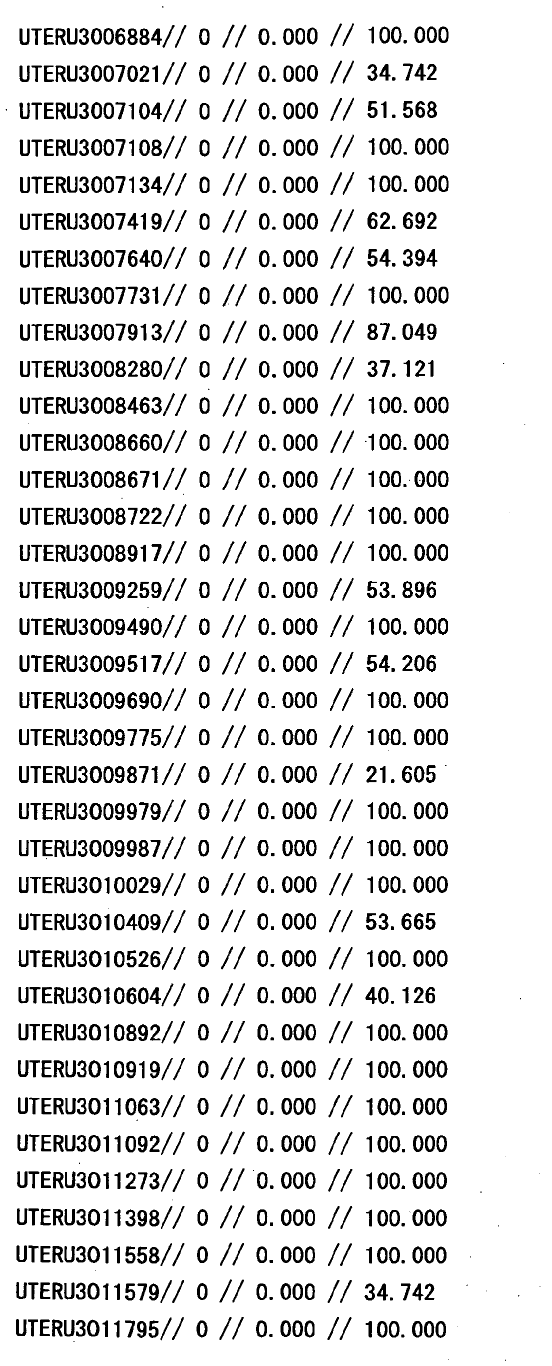

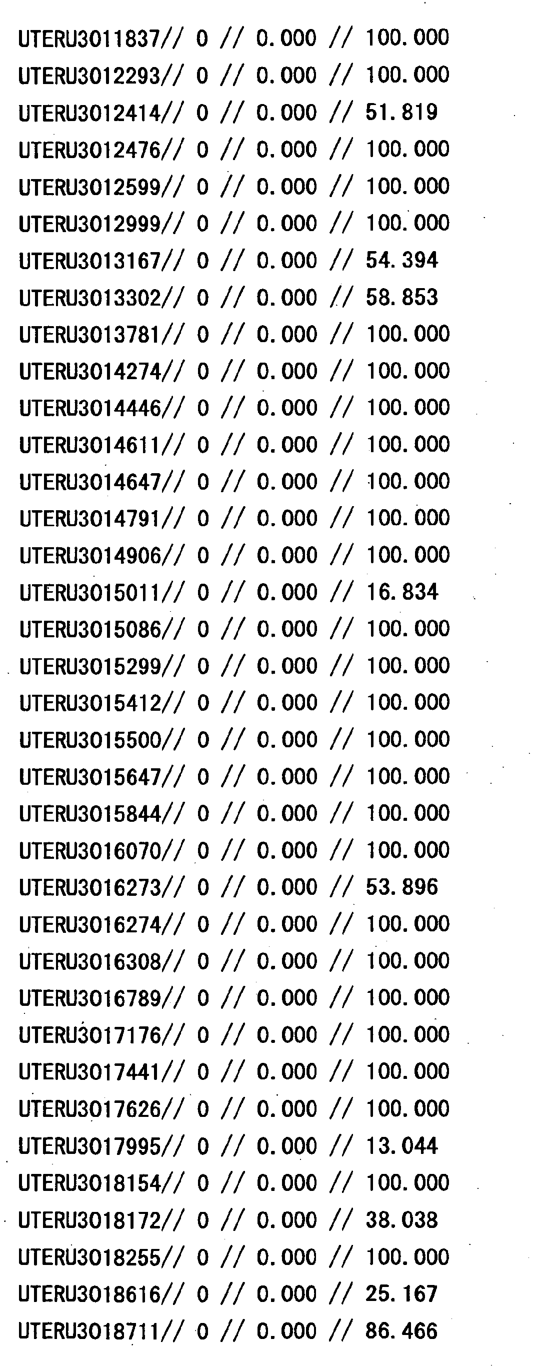

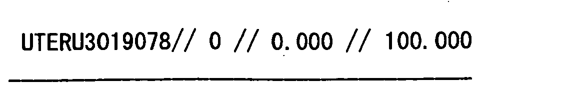

Genes whose expression levels were negligibly low in each of the above-described libraries are shown with their clone names below.
3NB691000018, 3NB691000085, 3NB691000113, 3NB691000116, 3NB691000129, 3NB691000191, 3NB692000012, 3NB692000029, 3NB692000102, 3NB692000154, 3NB692000276, 3NB692000305, 3NB692000330, 3NB692000374, 3NB692000429, 3NB692000484, 3NB692000529, 3NB692000973, 3NB692001002, 3NB692001022, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001861, 3NB692002051, 3NB692002365, 3NB692002685, 3NB692002806,
3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756,
ADRGL2001830, ADRGL2001836, ADRGL2001854, ADRGL2002029, ADRGL2002191, ADRGL2002260, ADRGL2002392, ADRGL2002477, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313,
ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO1000165, ASTRO2000011, ASTRO2000014, ASTRO2000095, ASTRO2000141, ASTRO2000191, ASTRO2000278, ASTRO2000372, ASTRO2000381, ASTRO2000403, ASTRO2000417, ASTRO2000482, ASTRO2000533, ASTRO2000653, ASTRO2000662, ASTRO2000725, ASTRO2001001, ASTRO2001008,
ASTRO2001029, ASTRO2001088, ASTRO2001107, ASTRO2001122, ASTRO2001227, ASTRO2001249, ASTRO2001480, ASTRO2001806, ASTRO2002024, ASTRO2002035, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003461, ASTRO2003574, ASTRO2003581, ASTRO2003632, ASTRO2003740, ASTRO2003840, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005242, ASTRO2005273, ASTRO2005343, ASTRO2005360, ASTRO2005413, ASTRO2005423, ASTRO2005553, ASTRO2005557,
ASTRO2005575, ASTRO2005593, ASTRO2005687, ASTRO2005726, ASTRO2005896, ASTRO2005902, ASTRO2006356, ASTRO2006475, ASTRO2006747, ASTRO2006920, ASTRO2006952, ASTRO2007221, ASTRO2007377, ASTRO2007515, ASTRO2007578, ASTRO2007643, ASTRO2007666, ASTRO2007956, ASTRO2007962, ASTRO2008040, ASTRO2008239, ASTRO2008409, ASTRO2008425, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2012552, ASTRO2013124, ASTRO2013671, ASTRO2013802,
ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015328, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000533, BGGI12000544, BGGI12000616, BGGI12000684, BGGI12000693, BGGI12000839, BGGI12001075,
BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004849, BLADE2005036, BLADE2005038, BLADE2005229, BLADE2005459, BLADE2005792, BLADE2006043,
BLADE2006415, BLADE2006607, BLADE2006826, BLADE2006830, BLADE2006849, BLADE2006947, BLADE2007033, BLADE2007043, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000153, BNGH41000169, BNGH41000177, BNGH41000190, BNGH41000198, BNGH41000216, BNGH41000235, BNGH42000130, BNGH42000132, BNGH42000360, BNGH42000405, BNGH42000532, BNGH42000675, BNGH42000791, BNGH42000815, BNGH42000994, BNGH42001247, BNGH42001406, BNGH42001576, BNGH42001724,
BNGH42002168, BNGH42002244, BNGH42002387, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006226, BNGH42006234, BNGH42007037, BNGH42007366, BNGH42007594, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020, BRACE1000047, BRACE1000051, BRACE1000065, BRACE1000073, BRACE1000084, BRACE1000087, BRACE1000092, BRACE1000093, BRACE1000101, BRACE1000114, BRACE1000115, BRACE1000124, BRACE1000132, BRACE1000159, BRACE1000166, BRACE1000169, BRACE1000187, BRACE1000239,
BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000307, BRACE2000331, BRACE2000332, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000753, BRACE2000815, BRACE2000864, BRACE2000885, BRACE2000893, BRACE2000905,
BRACE2000936, BRACE2000947, BRACE2000965, BRACE2000969, BRACE2000975, BRACE2000988, BRACE2001017, BRACE2001070, BRACE2001094, BRACE2001107, BRACE2001117, BRACE2001151, BRACE2001154, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737,
BRACE2001742, BRACE2001777, BRACE2001779, BRACE2001834, BRACE2001859, BRACE2001865, BRACE2001872, BRACE2001881, BRACE2001892, BRACE2001898, BRACE2001923, BRACE2001942, BRACE2001944, BRACE2001954, BRACE2001972, BRACE2001973, BRACE2002026, BRACE2002034, BRACE2002050, BRACE2002081, BRACE2002091, BRACE2002139, BRACE2002142, BRACE2002151, BRACE2002157, BRACE2002202, BRACE2002206, BRACE2002227, BRACE2002260, BRACE2002297, BRACE2002304, BRACE2002356, BRACE2002392, BRACE2002394, BRACE2002431, BRACE2002441, BRACE2002444, BRACE2002458, BRACE2002467, BRACE2002468, BRACE2002495, BRACE2002582, BRACE2002585, BRACE2002589, BRACE2002590, BRACE2002606, BRACE2002613, BRACE2002614, BRACE2002617, BRACE2002623,
BRACE2002635, BRACE2002661, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002860, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003269, BRACE2003285, BRACE2003304, BRACE2003319, BRACE2003398, BRACE2003420, BRACE2003428, BRACE2003431, BRACE2003539, BRACE2003540, BRACE2003594, BRACE2003598, BRACE2003609, BRACE2003614, BRACE2003628, BRACE2003639, BRACE2003700,
BRACE2003713, BRACE2003766, BRACE2003788, BRACE2003800, BRACE2003802, BRACE2003825, BRACE2003827, BRACE2003845, BRACE2003847, BRACE2003848, BRACE2003873, BRACE2003885, BRACE2003887, BRACE2003905, BRACE2003928, BRACE2003944, BRACE2003954, BRACE2003982, BRACE2003987, BRACE2005034, BRACE2005047, BRACE2005063, BRACE2005083, BRACE2005090, BRACE2005099, BRACE2005118, BRACE2005124, BRACE2005138, BRACE2005151, BRACE2005160, BRACE2005169, BRACE2005189, BRACE2005193, BRACE2005198, BRACE2005216, BRACE2005243, BRACE2005251, BRACE2005253, BRACE2005298, BRACE2005328, BRACE2005329, BRACE2005337, BRACE2005348, BRACE2005363, BRACE2005395, BRACE2005408, BRACE2005433, BRACE2005434, BRACE2005448, BRACE2005450,
BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006072, BRACE2006075, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274,
BRACE2006281, BRACE2006298, BRACE2006319, BRACE2006344, BRACE2006363, BRACE2006378, BRACE2006380, BRACE2006393, BRACE2006413, BRACE2006417, BRACE2006453, BRACE2006459, BRACE2006488, BRACE2006514, BRACE2006534, BRACE2006540, BRACE2006547, BRACE2006564, BRACE2006587, BRACE2006598, BRACE2006685, BRACE2006703, BRACE2006743, BRACE2006753, BRACE2006783, BRACE2006821, BRACE2006833, BRACE2006859, BRACE2006871, BRACE2006909, BRACE2006911, BRACE2006924, BRACE2006935, BRACE2006937, BRACE2006951, BRACE2006982, BRACE2006986, BRACE2007013, BRACE2007068, BRACE2007087, BRACE2007089, BRACE2007109, BRACE2007138, BRACE2007191, BRACE2007197, BRACE2007201, BRACE2007203, BRACE2007232, BRACE2007254, BRACE2007262,
BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563, BRACE2007567, BRACE2007577, BRACE2007621, BRACE2007640, BRACE2007641, BRACE2007646, BRACE2007663, BRACE2007671, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008,
BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE 2008380, BRACE2008385, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917,
BRACE2008919, BRACE2008948, BRACE2008960, BRACE2008968, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170,
BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012625,
BRACE2012653, BRACE2012814, BRACE2012833, BRACE2012838, BRACE2012936, BRACE2012947, BRACE2013009, BRACE2013010; BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335,
BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843,
BRACE2019865, BRACE2019872, BRACE2019877, BRACE2020028, BRACE2020077, BRACE2020097, BRACE2020157, BRACE2020278, BRACE2020358, BRACE2020467, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070,
BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879,
BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622,
BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039624, BRACE2039734, BRACE2039823, BRACE2039832, BRACE2039893, BRACE2039986, BRACE2040138, BRACE2040325, BRACE2040514, BRACE2040640,
BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835,
BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004435, BRACE3004477, BRACE3004767, BRACE3004772, BRACE3004783, BRACE3004843,
BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3009044, BRACE3009075, BRACE3009090, BRACE3009127,
BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714,
BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604,
BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184,
BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3041059, BRACE3041162, BRACE3041827, BRACE3042046, BRACE3042210, BRACE3042326, BRACE3042409,
BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397,
BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017531, BRALZ2017607, BRALZ2017620, BRALZ2017844, BRALZ2018342,
BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604,
BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711,
BRAMY2006092, BRAMY2006118, BRAMY2006149, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009192, BRAMY2009344, BRAMY2009349, BRAMY2009394, BRAMY2009489, BRAMY2009508, BRAMY2009528, BRAMY2009557,
BRAMY2009612, BRAMY2009693, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012581, BRAMY2012691, BRAMY2012721,
BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386,
BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019554, BRAMY2019600, BRAMY2019643, BRAMY2019710, BRAMY2019963, BRAMY2019985, BRAMY2020050, BRAMY2020058, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713,
BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863,
BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026713, BRAMY2026778, BRAMY2026824, BRAMY2026899, BRAMY2026902, BRAMY2026904, BRAMY2026976, BRAMY2027073, BRAMY2027114,
BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2032014, BRAMY2032231, BRAMY2032242, BRAMY2032290, BRAMY2032311,
BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629,
BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069,
BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002508, BRAMY3002620, BRAMY3002803, BRAMY3002805, BRAMY3002886,
BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004377, BRAMY3004672, BRAMY3004800, BRAMY3004900, BRAMY3004919, BRAMY3005091, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010008, BRAMY3010264, BRAMY3010321, BRAMY3010411,
BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000037, BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256,
BRAWH2000274, BRAWH2000323, BRAWH2000353, BRAWH2000370, BRAWH2000409, BRAWH2000417, BRAWH2000443, BRAWH2000460, BRAWH2000471, BRAWH2000476, BRAWH2000482, BRAWH2000494, BRAWH2000500, BRAWH2000503, BRAWH2000522, BRAWH2000554, BRAWH2000595, BRAWH2000636, BRAWH2000643, BRAWH2000651, BRAWH2000682, BRAWH2000686, BRAWH2000752, BRAWH2000859, BRAWH2000866, BRAWH2000892, BRAWH2000901, BRAWH2000924, BRAWH2000926, BRAWH2000944, BRAWH2000984, BRAWH2001019, BRAWH2001037, BRAWH2001052, BRAWH2001103, BRAWH2001129, BRAWH2001137, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406,
BRAWH2001418, BRAWH2001424, BRAWH2001436, BRAWH2001438, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025,
BRAWH2003135, BRAWH2003240, BRAWH2003307, BRAWH2003355, BRAWH2003366, BRAWH2003662, BRAWH2003689, BRAWH2003818, BRAWH2003832, BRAWH2003948, BRAWH2003964, BRAWH2004044, BRAWH2004068, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005225, BRAWH2005315, BRAWH2005517, BRAWH2005524, BRAWH2005533, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308,
BRAWH2007406, BRAWH2007550, BRAWH2007570, BRAWH2007591, BRAWH2007658, BRAWH2007664, BRAWH2007800, BRAWH2007808, BRAWH2007825, BRAWH2007862, BRAWH2007890, BRAWH2008128, BRAWH2008219, BRAWH2008255, BRAWH2008292, BRAWH2008297, BRAWH2008366, BRAWH2008380, BRAWH2008540, BRAWH2008577, BRAWH2008706, BRAWH2008790, BRAWH2008903, BRAWH2008956, BRAWH2008993, BRAWH2009112, BRAWH2009227, BRAWH2009261, BRAWH2009263, BRAWH2009298, BRAWH2009304, BRAWH2009307, BRAWH2009360, BRAWH2009393, BRAWH2009485, BRAWH2009.490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364,
BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234,
BRAWH2014379, BRAWH2014414, BRAWH2014511, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017305, BRAWH2017379, BRAWH2017407, BRAWH2017433, BRAWH2017523, BRAWH2017534, BRAWH2017542, BRAWH2017635, BRAWH2017913, BRAWH2018206,
BRAWH2018267, BRAWH2018282, BRAWH2018317, BRAWH2018506, BRAWH2018514, BRAWH2018527, BRAWH2018601, BRAWH2018729, BRAWH2018875, BRAWH2019053, BRAWH2019055, BRAWH2019198, BRAWH3000078, BRAWH3000088, BRAWH3000100, BRAWH3000166, BRAWH3000329, BRAWH3000410, BRAWH3000446, BRAWH3000491, BRAWH3000604, BRAWH3000884, BRAWH3001053, BRAWH3001319, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003801, BRAWH3003975, BRAWH3004222,
BRAWH3004335, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623,
BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH301350.8, BRAWH3014609, BRAWH3014639, BRAWH3015017, BRAWH3015175, BRAWH3015260, BRAWH3015610, BRAWH3015825, BRAWH3016123, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542,
BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620,
BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639,
BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494,
BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2010665, BRCAN2011254, BRCAN2011316, BRCAN2011602, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490,
BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021028, BRCAN2021325, BRCAN2021452, BRCAN2021669, BRCAN2021718, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026197, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028036, BRCAN2028040, BRCAN2028319, BRCAN2028338,
BRCAN2028378, BRCAN2028460, BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942,
BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010980, BRCOC2010994, BRCOC2011010, BRCOC2011398, BRCOC2011418, BRCOC2011506, BRCOC2011769, BRCOC2011996, BRCOC2012229, BRCOC2012386, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896,
BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000534, BRHIP2000553, BRHIP2000621, BRHIP2000691, BRHIP2000819, BRHIP2000826, BRHIP2000920, BRHIP2001074, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003242, BRHIP2003272,
BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010593, BRHIP2010610, BRHIP2010723, BRHIP2010744,
BRHIP2011080, BRHIP2011120, BRHIP2011199, BRHIP2011508, BRHIP2011576, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385,
BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020842, BRHIP2020859, BRHIP2020930, BRHIP2021102, BRHIP2021289, BRHIP2021495, BRHIP2021615, BRHIP2021744, BRHIP2021858, BRHIP2021870, BRHIP2021929, BRHIP2022006, BRHIP2022054, BRHIP2022221, BRHIP2022298, BRHIP2022467, BRHIP2022708, BRHIP2022986,
BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024789, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393,
BRHIP2029529, BRHIP2029643, BRHIP2029663, BRHIP3000060, BRHIP3000070, BRHIP3000081, BRHIP3000111, BRHIP3000131, BRHIP3000240, BRHIP3000457, BRHIP3000475, BRHIP3000488, BRHIP3000526, BRHIP3000626, BRHIP3000859, BRHIP3000907, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968,
BRHIP3005037, BRHIP3005137, BRHIP3005142, BRHIP3005231, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007609, BRHIP3007640, BRHIP3007960, BRHIP3008082, BRHIP3008183, BRHIP3008314, BRHIP3008320, BRHIP3008405, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082,
BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533,
BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000312, BRSSN2000518, BRSSN2000561, BRSSN2000566, BRSSN2000684, BRSSN2000715, BRSSN2000832, BRSSN2001275, BRSSN2001342, BRSSN2001426, BRSSN2001503, BRSSN2001579, BRSSN2001758, BRSSN2001810, BRSSN2002857, BRSSN2002859, BRSSN2003012, BRSSN2003086,
BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198,
BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070, BRSTN2000220,
BRSTN2000312, BRSTN2000367, BRSTN2000536, BRSTN2000872, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451,
BRSTN2013171, BRSTN2013354, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565,
BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598,
BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012189, BRTHA2012351, BRTHA2012392, BRTHA2012466, BRTHA2012555, BRTHA2012562, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663,
BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781,
BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252,
BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003225, BRTHA3003339, BRTHA3003343, BRTHA3003417, BRTHA3003449, BRTHA3003474, BRTHA3003490, BRTHA3003704, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386,
BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048,
BRTHA3019105, BRTHA3019183, BRTHA3020000, BRTHA3020369, BRTHA3021569, BRTHA3021708, BRTHA3021786, BRTHA3021971, BRTHA3022641, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026556, BRTHA3026916, BRTHA3027171, BRTHA3027318, BRTHA3027638, BRTHA3027820, BRTHA3027957, BRTHA3028339, BRTHA3028505, BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382,
CHONS2001797, CHONS2001834, CHONS2002419, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000056, CTONG1000062, CTONG1000086, CTONG1000088, CTONG1000093, CTONG1000097, CTONG1000113, CTONG1000165, CTONG1000175,
CTONG1000181, CTONG1000218, CTONG1000220, CTONG1000283, CTONG1000288, CTONG1000302, CTONG1000341, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000452, CTONG2000465, CTONG2000469, CTONG2000480, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001749, CTONG2001800, CTONG2001820, CTONG2001858,
CTONG2001877, CTONG2001911, CTONG2001955, CTONG2002066, CTONG2002095, CTONG2002143, CTONG2002395, CTONG2002417, CTONG2002418, CTONG2002453, CTONG2002520, CTONG2002558, CTONG2002590, CTONG2002709, CTONG2002721, CTONG2002816, CTONG2002820, CTONG2002832, CTONG2002841, CTONG2002903, CTONG2002965, CTONG2002999, CTONG2003028, CTONG2003094, CTONG2003100, CTONG2003115, CTONG2003189, CTONG2003245, CTONG2003293, CTONG2003350, CTONG2003361, CTONG2003372, CTONG2003375, CTONG2003427, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004454,
CTONG2004487, CTONG2004550, CTONG2004669, CTONG2004716, CTONG2004912, CTONG2004941, CTONG2004948, CTONG2005028, CTONG2005049, CTONG2005110, CTONG2005265, CTONG2005278, CTONG2005290, CTONG2005374, CTONG2005399, CTONG2005468, CTONG2005553, CTONG2005567, CTONG2005585, CTONG2005635, CTONG2005775, CTONG2005795, CTONG2005890, CTONG2005904, CTONG2005913, CTONG2006004, CTONG2006129, CTONG2006223, CTONG2006273, CTONG2006310, CTONG2006393, CTONG2006412, CTONG2006449, CTONG2006515, CTONG2006524, CTONG2006568, CTONG2006611, CTONG2006620, CTONG2006666, CTONG2006691, CTONG2006709, CTONG2006798, CTONG2006836, CTONG2006942, CTONG2006964, CTONG2007007, CTONG2007009, CTONG2007072, CTONG2007091, CTONG2007104,
CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321, CTONG2008402, CTONG2008405, CTONG2008466, CTONG2008506, CTONG2008521, CTONG2008562, CTONG2008577, CTONG2008595, CTONG2008621, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423,
CTONG2009440, CTONG2009527, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009955, CTONG2009958, CTONG2009963, CTONG2010018, CTONG2010024, CTONG2010116, CTONG2010133, CTONG2010148, CTONG2010276, CTONG2010330, CTONG2010508, CTONG2010566, CTONG2010623, CTONG2010652, CTONG2010803, CTONG2011188, CTONG2011429, CTONG2011474, CTONG2011676, CTONG2011716, CTONG2011765, CTONG2011779, CTONG2011815, CTONG2011822, CTONG2011920, CTONG2011985, CTONG2012001, CTONG2012029, CTONG2012050, CTONG2012077, CTONG2012101, CTONG2012123, CTONG2012158,
CTONG2012230, CTONG2012401, CTONG2012422, CTONG2012425, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012901, CTONG2013149, CTONG2013178, CTONG2013222, CTONG2013284, CTONG2013339, CTONG2013348, CTONG2013352, CTONG2013381, CTONG2013393, CTONG2013511, CTONG2013630, CTONG2013803, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014389, CTONG2014491, CTONG2014630, CTONG2014635, CTONG2014646, CTONG2014705, CTONG2014946, CTONG2014954,
CTONG2014959, CTONG2014966, CTONG2014995, CTONG2015091, CTONG2015204, CTONG2015316, CTONG2015330, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016499, CTONG2016505, CTONG2016559, CTONG2016658, CTONG2016775, CTONG2016797, CTONG2016824, CTONG2016846, CTONG2016904, CTONG2016931, CTONG2016942, CTONG2016953, CTONG2017021, CTONG2017292, CTONG2017409, CTONG2017444, CTONG2017458, CTONG2017500, CTONG2017604, CTONG2017650, CTONG2017798, CTONG2017804, CTONG2017814, CTONG2017910, CTONG2017939, CTONG2017989, CTONG2018062,
CTONG2018069, CTONG2018147, CTONG2018217, CTONG2018279, CTONG2018334, CTONG2018343, CTONG2018379, CTONG2018383, CTONG2018413, CTONG2018483, CTONG2018629, CTONG2018632, CTONG2018652, CTONG2018653, CTONG2018655, CTONG2018735, CTONG2018739, CTONG2018808, CTONG2018843, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019833, CTONG2020031, CTONG2020108, CTONG2020127, CTONG2020171, CTONG2020374, CTONG2020445, CTONG2020522, CTONG2020582, CTONG2020638, CTONG2020806, CTONG2020831, CTONG2020974, CTONG2021024, CTONG2021132, CTONG2021168, CTONG2021289, CTONG2022153, CTONG2022601,
CTONG2022835, CTONG2023021, CTONG2023449, CTONG2023512, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027327, CTONG2027361, CTONG2027591, CTONG2027736, CTONG2027783, CTONG2027828, CTONG2027959, CTONG2028097, CTONG2028208, CTONG2028309, CTONG2028486, CTONG2028518, CTONG2028687, CTONG2028758, CTONG3000657, CTONG3000686, CTONG3000707, CTONG3000896, CTONG3001420, CTONG3001501, CTONG3001560, CTONG3001605, CTONG3001746, CTONG3002020, CTONG3002127, CTONG3002370, CTONG3002412, CTONG3002433, CTONG3002552, CTONG3002588, CTONG3002674, CTONG3002728, CTONG3002835,
CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009287, CTONG3009328,
CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000291, D3OST3000388, D3OST3000493, D6OST2000127, D6OST2000169, D60ST2000245, D6OST2000358, D6OST2000507, D9OST2000031, D9OST2000278, D9OST2000440, D9OST2000869, D9OST2001319, D9OST2001433, D9OST2002397, D9OST2002673, D9OST2003106, D9OST2003108, D9OST2003137, D9OST2003397, D9OST2003580,
D9OST2003594, D9OST2003762, D9OST2003989, D9OST2004018, D9OST2004073, D9OST2004417, DFNES1000003, DFNES1000083, DFNES1000107, DFNES1000150, DFNES2000143, DFNES2000146, DFNES2000153, DFNES2000212, DFNES2000268, DFNES2000292, DFNES2000335, DFNES2000432, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004371, DFNES2004383, DFNES2004608, DFNES2004684, DFNES2005266, DFNES2005527, DFNES2005540,
DFNES2005690, DFNES2005766, DFNES2005779, DFNES2005875, DFNES2006944, DFNES2007113, DFNES2007299, DFNES2007332, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000025, FCBBF1000038, FCBBF1000053, FCBBF1000063, FCBBF1000069, FCBBF1000077, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000198, FCBBF1000206, FCBBF1000220, FCBBF1000232, FCBBF1000233, FCBBF1000243, FCBBF1000270,
FCBBF1000272, FCBBF1000280, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000437, FCBBF1000449, FCBBF1000476, FCBBF1000506, FCBBF1000509, FCBBF1000546, FCBBF1000550, FCBBF1000556, FCBBF1000563, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF2000038, FCBBF2000094, FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473,
FCBBF2000502, FCBBF2000566, FCBBF2000576, FCBBF2000677, FCBBF2000678, FCBBF2000733, FCBBF2000782, FCBBF2000808, FCBBF2000815, FCBBF2000825, FCBBF2000940, FCBBF2000951, FCBBF2001088, FCBBF2001105, FCBBF2001116, FCBBF2001183, FCBBF2001188, FCBBF2001211, FCBBF2001238, FCBBF2001243, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269,
FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000120, FCBBF3000168, FCBBF3000175, FCBBF3000227,
FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001632, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557,
FCBBF3003787, FCBBF3003902, FCBBF3004021, FCBBF3004041, FCBBF3004261, FCBBF3004323, FCBBF3004390, FCBBF3004409, FCBBF3004502, FCBBF3004715, FCBBF3004842, FCBBF3004955, FCBBF3005160, FCBBF3005218, FCBBF3005320, FCBBF3005330, FCBBF3005444, FCBBF3005497, FCBBF3005698, FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007248, FCBBF3007453, FCBBF3007462, FCBBF3007540, FCBBF3007597, FCBBF3007604, FCBBF3007631, FCBBF3007829, FCBBF3007855, FCBBF3007860, FCBBF3008073, FCBBF3008100, FCBBF3008159, FCBBF3008251, FCBBF3008311, FCBBF3008362, FCBBF3008382, FCBBF3008475, FCBBF3008556, FCBBF3008644, FCBBF3008689,
FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009541, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667,
FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017535, FCBBF3017681, FCBBF3017729, FCBBF3017873, FCBBF3017918, FCBBF3017974, FCBBF3018067, FCBBF3018173,
FCBBF3018453, FCBBF3018591, FCBBF3018753, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024096,
FCBBF3024225, FCBBF3024266, FCBBF3024364, FCBBF3024793, FCBBF3024911, FCBBF3025098, FCBBF3025252, FCBBF3025280, FCBBF3025285, FCBBF3025417, FCBBF3025650, FCBBF3025674, FCBBF3025730, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000017, FCBBF4000061, FCBBF4000076, FCBBF4000173, FCBBF4000192, FCBBF4000193, FCBBF4000268, FCBBF4000282, FCBBF4000399,
FCBBF4000415, FCBBF4000500, FCBBF4000529, FCBBF4000548, FCBBF5000041, FCBBF5000061, FCBBF5000165, FCBBF5000325, FCBBF5000353, FCBBF5000384, FCBBF5000483, FCBBF5000495, FEBRA1000005, FEBRA1000008, FEBRA1000030, FEBRA1000057, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452,
FEBRA2000454, FEBRA2000468, FEBRA2000491, FEBRA2000504, FEBRA2000532, FEBRA2000538, FEBRA2000575, FEBRA2000656, FEBRA2000659, FEBRA2000690, FEBRA2000727, FEBRA2000734, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000862, FEBRA2000877, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001294, FEBRA2001334,
FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001752, FEBRA2001772, FEBRA2001790, FEBRA2001814, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002552, FEBRA2002574, FEBRA2002601, FEBRA2002611, FEBRA2002628, FEBRA2002662,
FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002933, FEBRA2002962, FEBRA2002986, FEBRA2003054, FEBRA2003100, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003822, FEBRA2003897, FEBRA2003907, FEBRA2003930, FEBRA2004023, FEBRA2004026, FEBRA2004029, FEBRA2004053, FEBRA2004056, FEBRA2004091, FEBRA2004110,
FEBRA2004191, FEBRA2004202, FEBRA2004219, FEBRA2004224, FEBRA2004237, FEBRA2004268, FEBRA2004293, FEBRA2004325, FEBRA2004329, FEBRA2004331, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005572, FEBRA2005701, FEBRA2005726, FEBRA2005734, FEBRA2005778, FEBRA2005787, FEBRA2005788, FEBRA2005824, FEBRA2005995, FEBRA2005998, FEBRA2006055,
FEBRA2006061, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006370, FEBRA2006372, FEBRA2006379, FEBRA2006396, FEBRA2006401, FEBRA2006409, FEBRA2006418, FEBRA2006427, FEBRA2006476, FEBRA2006485, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551,
FEBRA2007566, FEBRA2007589, FEBRA2007613, FEBRA2007620, FEBRA2007622, FEBRA2007708, FEBRA2007793, FEBRA2007801, FEBRA2007823, FEBRA2007880, FEBRA2007900, FEBRA2007931, FEBRA2007957, FEBRA2008009, FEBRA2008036, FEBRA2008086, FEBRA2008134, FEBRA2008135, FEBRA2008159, FEBRA2008165, FEBRA2008172, FEBRA2008201, FEBRA2008265, FEBRA2008266, FEBRA2008282, FEBRA2008287, FEBRA2008311, FEBRA2008341, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029,
FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011356, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274,
FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018476, FEBRA2018746, FEBRA2019172, FEBRA2019242, FEBRA2019298, FEBRA2019389, FEBRA2019457,
FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023989,
FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2026474, FEBRA2026582, FEBRA2026629, FEBRA2026769, FEBRA2026879, FEBRA2026890, FEBRA2026977, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000156, HCASM2000202, HCASM2000214, HCASM2000266, HCASM2000307,
HCASM2000534, HCASM2000536, HCASM2000560, HCASM2000871, HCASM2001285, HCASM2001810, HCASM2001866, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2009405, HCASM2009424, HCASM2009463, HCASM2009588, HCHON1000015, HCHON1000166, HCHON1000176, HCHON1000191, HCHON2000028, HCHON2000038, HCHON2000062, HCHON2000100, HCHON2000156, HCHON2000199, HCHON2000212, HCHON2000271, HCHON2000315, HCHON2000322, HCHON2000344, HCHON2000364, HCHON2000373,
HCHON2000395, HCHON2000417, HCHON2000473, HCHON2000503, HCHON2000508, HCHON2000660, HCHON2000663, HCHON2000673, HCHON2000676, HCHON2000699, HCHON2000705, HCHON2000751, HCHON2000815, HCHON2000817, HCHON2000818, HCHON2000819, HCHON2000832, HCHON2000851, HCHON2000898, HCHON2000935, HCHON2000942, HCHON2000956, HCHON2000994, HCHON2001050, HCHON2001099, HCHON2001108, HCHON2001110, HCHON2001116, HCHON2001161, HCHON2001214, HCHON2001233, HCHON2001269, HCHON2001359, HCHON2001407, HCHON2001450, HCHON2001453, HCHON2001497, HCHON2001519, HCHON2001523, HCHON2001535, HCHON2001546, HCHON2001548, HCHON2001577, HCHON2001604, HCHON2001607, HCHON2001646, HCHON2001712, HCHON2001768, HCHON2001801, HCHON2002123,
HCHON2002194, HCHON2002241, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987, HCHON2006459, HCHON2006714, HCHON2006722, HCHON2006770, HCHON2006786, HCHON2006841, HCHON2006871, HCHON2007046, HCHON2007482, HCHON2007674, HCHON2007774, HCHON2007881, HCHON2008112, HCHON2008239, HCHON2008582, HCHON2008672, HCHON2008856, HCHON2008871, HCHON2008989, HCHON2009191, HCHON2009384, HCHON2009749, HCHON2009766,
HCHON2009795, HCHON2010074, HCHON2010543, HEART1000010, HEART1000074, HEART1000088, HEART1000099, HEART1000102, HEART1000118, HEART1000132, HEART1000139, HEART1000142, HEART1000151, HEART1000173, HEART2000024, HEART2000035, HEART2000099, HEART2000298, HEART2000306, HEART2000309, HEART2000411, HEART2000418, HEART2000444, HEART2000448, HEART2000492, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004940, HEART2004941,
HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000083, HHDPC1000118, HHDPC2000055, HHDPC2000070, HHDPC2000102, HHDPC2000104, HHDPC2000115, HHDPC2000149, HHDPC2000258, HHDPC2000315, HHDPC2000456, HHDPC2000462, HHDPC2000692, HHDPC2001337, HHDPC2001432, HHDPC2001896,
HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007302, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000084, HLUNG1000091, HLUNG1000099, HLUNG1000104, HLUNG1000105, HLUNG1000110, HLUNG1000151, HLUNG1000169, HLUNG2000014, HLUNG2000027, HLUNG2000068, HLUNG2000116, HLUNG2000125, HLUNG2000130, HLUNG2000142, HLUNG2000174, HLUNG2000176,
HLUNG2000217, HLUNG2000232, HLUNG2000255, HLUNG2000281, HLUNG2000315, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000870, HLUNG2000926, HLUNG2000950, HLUNG2000955, HLUNG2000970, HLUNG2001013, HLUNG2001126, HLUNG2001144, HLUNG2001214, HLUNG2001439, HLUNG2001459, HLUNG2001633, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002659, HLUNG2002707, HLUNG2002916, HLUNG2002942, HLUNG2002949, HLUNG2002958,
HLUNG2002982, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004154, HLUNG2004217, HLUNG2004273, HLUNG2004388, HLUNG2004416, HLUNG2004521, HLUNG2004534, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094,
HLUNG2006370, HLUNG2006397, HLUNG2006570, HLUNG2006599, HLUNG2006614, HLUNG2006671, HLUNG2006781, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008348, HLUNG2008384, HLUNG2008396, HLUNG2008439, HLUNG2008479, HLUNG2008480, HLUNG2008521, HLUNG2008833, HLUNG2008875, HLUNG2008885, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049,
HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000119, HSYRA1000131, HSYRA1000137, HSYRA1000148, HSYRA2000044, HSYRA2000135, HSYRA2000137, HSYRA2000190, HSYRA2000221, HSYRA2000237, HSYRA2000253, HSYRA2000332, HSYRA2000371, HSYRA2000424, HSYRA2000507, HSYRA2000510, HSYRA2000605, HSYRA2000629,
HSYRA2000706, HSYRA2000760, HSYRA2000787, HSYRA2000792, HSYRA2000828, HSYRA2000832, HSYRA2000925, HSYRA2000927, HSYRA2001003, HSYRA2001103, HSYRA2001142, HSYRA2001153, HSYRA2001225, HSYRA2001232, HSYRA2001255, HSYRA2001332, HSYRA2001353, HSYRA2001420, HSYRA2001484, HSYRA2001552, HSYRA2001595, HSYRA2001631, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007667, HSYRA2008154, HSYRA2008714, HSYRA2009102, IMR321000155, IMR321000161, IMR321000165, IMR321000193, IMR321000207, IMR321000230, IMR321000253, IMR322000010,
IMR322000072, IMR322000107, IMR322000127, IMR322000144, IMR322000152, IMR322000164, IMR322000178, IMR322000223, IMR322000243, IMR322000302, IMR322000316, IMR322000374, IMR322000379, IMR322000383, IMR322000418, IMR322000518, IMR322000555, IMR322000604, IMR322000609, IMR322000656, IMR322000672, IMR322000742, IMR322000775, IMR322000791, IMR322000811, IMR322000819, IMR322000838, IMR322000859, IMR322000863, IMR322000917, IMR322000918, IMR322000919, IMR322000953, IMR322000973, IMR322000984, IMR322001018, IMR322001167, IMR322001185, IMR322001218, IMR322001245, IMR322001317, IMR322001318, IMR322001322, IMR322001332, IMR322001380, IMR322001438, IMR322001452, IMR322001491, IMR322001541, IMR322001584,
IMR322001665, IMR322001670, IMR322001724, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000064, KIDNE1000104, KIDNE1000121, KIDNE1000143, KIDNE1000152, KIDNE2000026, KIDNE2000041, KIDNE2000046,
KIDNE2000051, KIDNE2000070, KIDNE2000167, KIDNE2000174, KIDNE2000192, KIDNE2000227, KIDNE2000244, KIDNE2000245, KIDNE2000252, KIDNE2000266, KIDNE2000287, KIDNE2000315, KIDNE2000317, KIDNE2000328, KIDNE2000335, KIDNE2000349, KIDNE2000375, KIDNE2000383, KIDNE2000394, KIDNE2000422, KIDNE2000493, KIDNE2000497, KIDNE2000510, KIDNE2000513, KIDNE2000517, KIDNE2000519, KIDNE2000555, KIDNE2000574, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2000909, KIDNE2000947, KIDNE2001117, KIDNE2001140, KIDNE2001269, KIDNE2001361, KIDNE2001373, KIDNE2001636, KIDNE2001713, KIDNE2001802,
KIDNE2001840, KIDNE2001847, KIDNE2001857, KIDNE2001873, KIDNE2001897, KIDNE2001931, KIDNE2002015, KIDNE2002162, KIDNE2002168, KIDNE2002191, KIDNE2002198, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882, KIDNE2002883, KIDNE2002980, KIDNE2002991, KIDNE2003185, KIDNE2003188, KIDNE2003233, KIDNE2003316, KIDNE2003335, KIDNE2003357, KIDNE2003373, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305,
KIDNE2004344, KIDNE2004394, KIDNE2004411, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376,
KIDNE2006378, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007077, KIDNE2007186, KIDNE2007222, KIDNE2007356, KIDNE2007422, KIDNE2007569, KIDNE2007810, KIDNE2007910, KIDNE2007944, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191,
KIDNE2009332, KIDNE2009426, KIDNE2009467, KIDNE2009490, KIDNE2009553, KIDNE2009628, KIDNE2009647, KIDNE2009676, KIDNE2009723, KIDNE2010007, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012945,
KIDNE2013045, KIDNE2013095, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142,
KIDNE2016188, KIDNE2016327, KIDNE2016371, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099, LIVER1000104, LIVER1000111,
LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000037, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001835, LIVER2002638, LIVER2002644, LIVER2002660, LIVER2002702, LIVER2003008, LIVER2003065, LIVER2003511, LIVER2003524, LIVER2003568,
LIVER2003581, LIVER2003800, LIVER2003854, LIVER2004074, LIVER2004392, LIVER2004514, LIVER2004565, LIVER2005028, LIVER2005198, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000032, MAMGL1000035, MAMGL1000096, MAMGL1000100, MAMGL1000132, MAMGL1000151,
MAMGL1000173, MAMGL1000178, MAMGL1000182, MAMGL1000184, MESAN1000032, MESAN1000035, MESAN1000050, MESAN1000079, MESAN1000080, MESAN1000101, MESAN1000121, MESAN1000126, MESAN1000147, MESAN1000180, MESAN2000023, MESAN2000026, MESAN2000067, MESAN2000092, MESAN2000149, MESAN2000167, MESAN2000291, MESAN2000337, MESAN2000434, MESAN2000457, MESAN2000462, MESAN2000501, MESAN2000572, MESAN2000608, MESAN2000620, MESAN2000711, MESAN2000815, MESAN2000909, MESAN2001450, MESAN2001627, MESAN2001770, MESAN2001979, MESAN2001980, MESAN2002113, MESAN2002122, MESAN2002147, MESAN2002186, MESAN2002519, MESAN2002687, MESAN2002709, MESAN2002790, MESAN2002940, MESAN2002978, MESAN2003035, MESAN2003037, MESAN2003101,
MESAN2003190, MESAN2003322, MESAN2003490, MESAN2003529, MESAN2003622, MESAN2003646, MESAN2003662, MESAN2003851, MESAN2004138, MESAN2004288, MESAN2004575, MESAN2005284, MESAN2005371, MESAN2005633, MESAN2005689, MESAN2005724, MESAN2005766, MESAN2005808, MESAN2005811, MESAN2005958, MESAN2006022, MESAN2006328, MESAN2006401, MESAN2006563, MESAN2006599, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926, MESAN2008936, MESAN2009019, MESAN2009081, MESAN2009105, MESAN2009311, MESAN2009418, MESAN2009503, MESAN2009522, MESAN2010031, MESAN2010114,
MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016965, MESAN2017133, MESAN2017373, MESAN2017417, MESAN2018209, MESAN2018576, MESAN2018670, MESAN2018699, MESTC1000042, MESTC1000147, MESTC2000150,
MESTC2000153, MESTC2000170, N1ESE2000698, NB9N41000011, NB9N41000047, NB9N41000135, NB9N41000142, NB9N41000146, NB9N41000340, NB9N42000104, NB9N42000122, NB9N42000196, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000187, NHNPC2000206, NHNPC2000212,
NHNPC2000606, NHNPC2000877, NHNPC2001108, NHNPC2001223, NHNPC2001312, NHNPC2001816, NHNPC2001931, NHNPC2002565, NHNPC2002749, NOVAR1000102, NOVAR2000038, NOVAR2000136, NOVAR2000352, NOVAR2000412, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000174, NT2NE2000195, NT2NE2000222, NT2NE2000259, NT2NE2000260, NT2NE2000284, NT2NE2000299, NT2NE2000353,
NT2NE2000356, NT2NE2000369, NT2NE2000374, NT2NE2000386, NT2NE2000390, NT2NE2000392, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000763, NT2NE2000802, NT2NE2000808, NT2NE2000809, NT2NE2000864, NT2NE2000909, NT2NE2000913, NT2NE2000950, NT2NE2000951, NT2NE2000980, NT2NE2001005, NT2NE2001021, NT2NE2001040, NT2NE2001048, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001364, NT2NE2001372, NT2NE2001397, NT2NE2001403, NT2NE2001428, NT2NE2001432, NT2NE2001442,
NT2NE2001524, NT2NE2001530, NT2NE2001598, NT2NE2001623, NT2NE2001626, NT2NE2001634, NT2NE2001648, NT2NE2001655, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002651, NT2NE2002732, NT2NE2002768, NT2NE2002791, NT2NE2002856, NT2NE2002870, NT2NE2003150, NT2NE2003252, NT2NE2003280, NT2NE2003308, NT2NE2003309, NT2NE2003315, NT2NE2003393, NT2NE2003408, NT2NE2003457, NT2NE2003540, NT2NE2003571, NT2NE2003705, NT2NE2003752, NT2NE2003887, NT2NE2003921, NT2NE2004255, NT2NE2004378, NT2NE2004490, NT2NE2004606, NT2NE2004740, NT2NE2004787, NT2NE2004916,
NT2NE2005035, NT2NE2005223, NT2NE2005276, NT2NE2005317, NT2NE2005323, NT2NE2005371, NT2NE2005395, NT2NE2005419, NT2NE2005441, NT2NE2005517, NT2NE2005537, NT2NE2005555, NT2NE2005588, NT2NE2005676, NT2NE2005688, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006478, NT2NE2006531, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060,
NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010842, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448,
NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397,
NT2NE2017480, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000027, NT2RI1000035, NT2RI1000048, NT2RI1000164, NT2RI1000172, NT2RI2000004, NT2RI2000107, NT2RI2000116, NT2RI2000117, NT2RI2000128, NT2RI2000133, NT2RI2000167, NT2RI2000173, NT2RI2000187, NT2RI2000255, NT2RI2000263, NT2RI2000270, NT2RI2000276, NT2RI2000284, NT2RI2000313, NT2RI2000341, NT2RI2000348, NT2RI2000412, NT2RI2000421, NT2RI2000480, NT2RI2000575,
NT2RI2000588, NT2RI2000597, NT2RI2000669, NT2RI2000688, NT2RI2000704, NT2RI2000738, NT2RI2000775, NT2RI2000822, NT2RI2000829, NT2RI2000841, NT2RI2000865, NT2RI2000932, NT2RI2000974, NT2RI2000987, NT2RI2001010, NT2RI2001017, NT2RI2001030, NT2RI2001054, NT2RI2001083, NT2RI2001167, NT2RI2001178, NT2RI2001235, NT2RI2001244, NT2RI2001307, NT2RI2001342, NT2RI2001385, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001726, NT2RI2001731, NT2RI2001769, NT2RI2001782, NT2RI2001836, NT2RI2001846, NT2RI2001866, NT2RI2001910, NT2RI2001952, NT2RI2002022, NT2RI2002041, NT2RI2002117, NT2RI2002120,
NT2RI2002153, NT2RI2002179, NT2RI2002243, NT2RI2002252, NT2RI2002260, NT2RI2002266, NT2RI2002270, NT2RI2002334, NT2RI2002359, NT2RI2002447, NT2RI2002481, NT2RI2002507, NT2RI2002517, NT2RI2002530, NT2RI2002540, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819, NT2RI2002822, NT2RI2002847, NT2RI2002852, NT2RI2002923, NT2RI2002926, NT2RI2002958, NT2RI2002970, NT2RI2003011, NT2RI2003019, NT2RI2003051, NT2RI2003067, NT2RI2003184, NT2RI2003205, NT2RI2003222, NT2RI2003301, NT2RI2003360, NT2RI2003361, NT2RI2003383, NT2RI2003392, NT2RI2003399, NT2RI2003419, NT2RI2003481, NT2RI2003487, NT2RI2003655, NT2RI2003667,
NT2RI2003678, NT2RI2003884, NT2RI2003921, NT2RI2003973, NT2RI2003993, NT2RI2004059, NT2RI2004078, NT2RI2004079, NT2RI2004093, NT2RI2004136, NT2RI2004190, NT2RI2004209, NT2RI2004284, NT2RI2004290, NT2RI2004304, NT2RI2004312, NT2RI2004381, NT2RI2004398, NT2RI2004410, NT2RI2004442, NT2RI2004468, NT2RI2004606, NT2RI2004608, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005130, NT2RI2005150, NT2RI2005166, NT2RI2005239, NT2RI2005246, NT2RI2005315, NT2RI2005353, NT2RI2005368, NT2RI2005382, NT2RI2005473, NT2RI2005520, NT2RI2005564,
NT2RI2005579, NT2RI2005621, NT2RI2005628, NT2RI2005647, NT2RI2005670, NT2RI2005710, NT2RI2005772, NT2RI2005816, NT2RI2005851, NT2RI2005966, NT2RI2006070, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006838, NT2RI2006853, NT2RI2006855, NT2RI2006856, NT2RI2006943, NT2RI2006973, NT2RI2007046, NT2RI2007048, NT2RI2007084, NT2RI2007096,
NT2RI2007116, NT2RI2007133, NT2RI2007214, NT2RI2007303, NT2RI2007334, NT2RI2007377, NT2RI2007386, NT2RI2007406, NT2RI2007439, NT2RI2007468, NT2RI2007469, NT2RI2007507, NT2RI2007572, NT2RI2007593, NT2RI2007723, NT2RI2007729, NT2RI2007751, NT2RI2007754, NT2RI2007827, NT2RI2007853, NT2RI2007877, NT2RI2007879, NT2RI2007884, NT2RI2007891, NT2RI2007956, NT2RI2007965, NT2RI2008050, NT2RI2008141, NT2RI2008171, NT2RI2008188, NT2RI2008204, NT2RI2008221, NT2RI2008233, NT2RI2008396, NT2RI2008455, NT2RI2008502, NT2RI2008566, NT2RI2008598, NT2RI2008622, NT2RI2008656, NT2RI2008714, NT2RI2008801, NT2RI2008808, NT2RI2008812, NT2RI2008841, NT2RI2008952, NT2RI2009005, NT2RI2009066, NT2RI2009083, NT2RI2009101,
NT2RI2009103, NT2RI2009144, NT2RI2009151, NT2RI2009173, NT2RI2009194, NT2RI2009215, NT2RI2009233, NT2RI2009239, NT2RI2009259, NT2RI2009281, NT2RI2009289, NT2RI2009301, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012350, NT2RI2012659, NT2RI2012726, NT2RI2012990, NT2RI2013345, NT2RI2013357, NT2RI2013373, NT2RI2014551, NT2RI2014683, NT2RI2015239, NT2RI2015342, NT2RI2015533, NT2RI2016128, NT2RI2016134, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022584, NT2RI2023303,
NT2RI2023671, NT2RI2024008, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381,
NT2RI3004510, NT2RI3005202, NT2RI3005403, NT2RI3005416, NT2RI3005861, NT2RI3005928, NT2RI3006132, NT2RI3006171, NT2RI3006376, NT2RI3006796, NT2RI3007065, NT2RI3007095, NT2RI3007158, NT2RI3007167, NT2RI3007213, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000032, NT2RP6000033, NT2RP6000043, NT2RP6000060, NT2RP6000072, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000086, NT2RP7000109,
NT2RP7000112, NT2RP7000238, NT2RP7000259, NT2RP7000271, NT2RP7000311, NT2RP7000359, NT2RP7000364, NT2RP7000380, NT2RP7000391, NT2RP7000425, NT2RP7000579, NT2RP7000624, NT2RP7000812, NT2RP7000926, NT2RP7001074, NT2RP7001080, NT2RP7001156, NT2RP7001231, NT2RP7001283, NT2RP7001306, NT2RP7001319, NT2RP7001335, NT2RP7001364, NT2RP7001475, NT2RP7001532, NT2RP7001591, NT2RP7001602, NT2RP7001678, NT2RP7001856, NT2RP7002064, NT2RP7002151, NT2RP7002163, NT2RP7002243, NT2RP7002282, NT2RP7002450, NT2RP7002478, NT2RP7002554, NT2RP7002594, NT2RP7002603, NT2RP7002619, NT2RP7002650, NT2RP7002738, NT2RP7002779, NT2RP7002829, NT2RP7002841, NT2RP7002906, NT2RP7002978, NT2RP7003025, NT2RP7003050, NT2RP7003084,
NT2RP7003091, NT2RP7003107, NT2RP7003134, NT2RP7003148, NT2RP7003261, NT2RP7003439, NT2RP7003511, NT2RP7003629, NT2RP7003680, NT2RP7003724, NT2RP7003960, NT2RP7004027, NT2RP7004080, NT2RP7004173, NT2RP7004204, NT2RP7004233, NT2RP7004260, NT2RP7004358, NT2RP7004396, NT2RP7004428, NT2RP7004481, NT2RP7004541, NT2RP7004641, NT2RP7004656, NT2RP7004687, NT2RP7004722, NT2RP7004766, NT2RP7004790, NT2RP7004884, NT2RP7004911, NT2RP7004961, NT2RP7004964, NT2RP7004975, NT2RP7005118, NT2RP7005205, NT2RP7005219, NT2RP7005323, NT2RP7005468, NT2RP7005493, NT2RP7005629, NT2RP7005631, NT2RP7005669, NT2RP7005846, NT2RP7006033, NT2RP7006075, NT2RP7006141, NT2RP7006160, NT2RP7006162, NT2RP7006188, NT2RP7006263,
NT2RP7006296, NT2RP7006304, NT2RP7006374, NT2RP7006395, NT2RP7006408, NT2RP7006457, NT2RP7006547, NT2RP7006601, NT2RP7006621, NT2RP7006636, NT2RP7006701, NT2RP7006853, NT2RP7006887, NT2RP7006980, NT2RP7006986, NT2RP7006995, NT2RP7007177, NT2RP7007221, NT2RP7007226, NT2RP7007269, NT2RP7007359, NT2RP7007387, NT2RP7007406, NT2RP7007480, NT2RP7007504, NT2RP7007530, NT2RP7007580, NT2RP7007594, NT2RP7007617, NT2RP7007643, NT2RP7007766, NT2RP7007842, NT2RP7007925, NT2RP7007930, NT2RP7007975, NT2RP7008013, NT2RP7008015, NT2RP7008133, NT2RP7008137, NT2RP7008142, NT2RP7008144, NT2RP7008360, NT2RP7008396, NT2RP7008406, NT2RP7008435, NT2RP7008441, NT2RP7008454, NT2RP7008487, NT2RP7008544, NT2RP7008550,
NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009108, NT2RP7009149, NT2RP7009168, NT2RP7009215, NT2RP7009225, NT2RP7009236, NT2RP7009253, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291,
NT2RP7012516, NT2RP7012776, NT2RP7013002, NT2RP7013374, NT2RP7013499, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7014910, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401,
NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000213, NTONG1000214, NTONG1000247, NTONG1000257, NTONG1000264, NTONG2000040, NTONG2000107, NTONG2000231, NTONG2000334,
NTONG2000363, NTONG2000413, NTONG2000492, NTONG2000499, NTONG2000531, NTONG2000583, NTONG2000800, NTONG2000858, NTONG2000878, NTONG2000966, NTONG2000977, NTONG2000985, NTONG2001014, NTONG2001079, NTONG2001137, NTONG2001157, NTONG2001173, NTONG2001220, NTONG2001222, NTONG2001362, NTONG2001428, NTONG2001532, NTONG2001587, NTONG2001612, NTONG2001619, NTONG2001642, NTONG2001657, NTONG2001676, NTONG2001762, NTONG2002278, NTONG2002807, NTONG2002948, NTONG2002970, NTONG2002985, NTONG2003158, NTONG2003210, NTONG2003316, NTONG2003409, NTONG2003515, NTONG2003805, NTONG2003852, NTONG2003897, NTONG2003928, NTONG2004095, NTONG2004308, NTONG2004521, NTONG2004614, NTONG2004690, NTONG2004806, NTONG2004829,
NTONG2004918, NTONG2004991, NTONG2005062, NTONG2005086, NTONG2005137, NTONG2005153, NTONG2005265, NTONG2005277, NTONG2005373, NTONG2005480, NTONG2005497, NTONG2005577, NTONG2005657, NTONG2005822, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008483, NTONG2008522, NTONG2008672, NTONG2008862, NTONG2008939, NTONG2009060, NTONG2009068, NTONG2009229, NTONG2009233, NTONG2009468, NTONG2009576,
OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000114, OCBBF1000118, OCBBF1000119, OCBBF1000122, OCBBF1000130, OCBBF1000138, OCBBF1000145, OCBBF1000146, OCBBF1000153, OCBBF1000185, OCBBF1000188, OCBBF1000192, OCBBF1000254, OCBBF1000315, OCBBF2000013, OCBBF2000126, OCBBF2000178, OCBBF2000231, OCBBF2000277, OCBBF2000287, OCBBF2000323, OCBBF2000452, OCBBF2000522, OCBBF2000703, OCBBF2000712, OCBBF2000719, OCBBF2000824, OCBBF2000831, OCBBF2000846, OCBBF2000904, OCBBF2000982, OCBBF2000998, OCBBF2001075, OCBBF2001101, OCBBF2001124, OCBBF2001140, OCBBF2001176, OCBBF2001186, OCBBF2001196, OCBBF2001252,
OCBBF2001307, OCBBF2001389, OCBBF2001402, OCBBF2001408, OCBBF2001416, OCBBF2001473, OCBBF2001492, OCBBF2001494, OCBBF2001527, OCBBF2001528, OCBBF2001639, OCBBF2001681, OCBBF2001687, OCBBF2001706, OCBBF2001749, OCBBF2001794, OCBBF2001910, OCBBF2001931, OCBBF2001983, OCBBF2002015, OCBBF2002083, OCBBF2002124, OCBBF2002161, OCBBF2002290, OCBBF2002357, OCBBF2002376, OCBBF2002615, OCBBF2002626, OCBBF2002663, OCBBF2002805, OCBBF2002865, OCBBF2002920, OCBBF2002980, OCBBF2003028, OCBBF2003052, OCBBF2003091, OCBBF2003327, OCBBF2003518, OCBBF2003543, OCBBF2003593, OCBBF2003744, OCBBF2003819, OCBBF2004038, OCBBF2004104, OCBBF2004147, OCBBF2004168, OCBBF2004235, OCBBF2004259, OCBBF2004385, OCBBF2004418,
OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005546, OCBBF2005843, OCBBF2005901, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006388, OCBBF2006438, OCBBF2006567, OCBBF2006624, OCBBF2006639, OCBBF2006660, OCBBF2006764, OCBBF2006859, OCBBF2006972, OCBBF2006987, OCBBF2007039, OCBBF2007051, OCBBF2007068, OCBBF2007114, OCBBF2007121,
OCBBF2007184, OCBBF2007194, OCBBF2007196, OCBBF2007224, OCBBF2007238, OCBBF2007354, OCBBF2007414, OCBBF2007428, OCBBF2007478, OCBBF2007485, OCBBF2007520, OCBBF2007555, OCBBF2007610, OCBBF2007622, OCBBF2007756, OCBBF2007762, OCBBF2007892, OCBBF2007931, OCBBF2007946, OCBBF2008041, OCBBF2008116, OCBBF2008138, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775, OCBBF2008790, OCBBF2008814, OCBBF2008822, OCBBF2009095, OCBBF2009115, OCBBF2009352, OCBBF2009391, OCBBF2009536, OCBBF2009571, OCBBF2009583,
OCBBF2009603, OCBBF2009788, OCBBF2009836, OCBBF2009920, OCBBF2009926, OCBBF2010040, OCBBF2010140, OCBBF2010281, OCBBF2010404, OCBBF2010416, OCBBF2010420, OCBBF2010604, OCBBF2010709, OCBBF2010792, OCBBF2010819, OCBBF2010841, OCBBF2010843, OCBBF2010858, OCBBF2010863, OCBBF2010871, OCBBF2010931, OCBBF2010978, OCBBF2010989, OCBBF2011073, OCBBF2011137, OCBBF2011160, OCBBF2011177, OCBBF2011283, OCBBF2011311, OCBBF2011625, OCBBF2011669, OCBBF2011685, OCBBF2011706, OCBBF2011722, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320,
OCBBF2012436, OCBBF2012525, OCBBF2012553, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013208, OCBBF2013285, OCBBF2013319, OCBBF2013324, OCBBF2013366, OCBBF2013481, OCBBF2013507, OCBBF2013721, OCBBF2013789, OCBBF2013883, OCBBF2013926, OCBBF2014052, OCBBF2014089, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503,
OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2016928, OCBBF2017035, OCBBF2017055, OCBBF2017069, OCBBF2017306, OCBBF2017325, OCBBF2017458, OCBBF2017489, OCBBF2017516, OCBBF2017665, OCBBF2017815, OCBBF2017888, OCBBF2017893, OCBBF2017920, OCBBF2018014, OCBBF2018084, OCBBF2018167, OCBBF2018206, OCBBF2018229, OCBBF2018234, OCBBF2018362, OCBBF2018380, OCBBF2018581, OCBBF2018618, OCBBF2018663, OCBBF2018687, OCBBF2018707, OCBBF2018827, OCBBF2018828, OCBBF2018934, OCBBF2019108, OCBBF2019195,
OCBBF2019327, OCBBF2019684, OCBBF2019761, OCBBF2019823, OCBBF2019950, OCBBF2020048, OCBBF2020318, OCBBF2020343, OCBBF2020453, OCBBF2020801, OCBBF2020825, OCBBF2020838, OCBBF2021020, OCBBF2021214, OCBBF2021286, OCBBF2021323, OCBBF2021518, OCBBF2021788, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026645, OCBBF2026649, OCBBF2026690, OCBBF2026981, OCBBF2027197, OCBBF2027354, OCBBF2027423, OCBBF2027478, OCBBF2027481, OCBBF2027511,
OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036752,
OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000323, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091,
PANCR1000185, PEBLM1000024, PEBLM1000029, PEBLM1000034, PEBLM1000062, PEBLM1000066, PEBLM1000068, PEBLM1000071, PEBLM1000083, PEBLM1000147, PEBLM1000174, PEBLM1000180, PEBLM2000112, PEBLM2000126, PEBLM2000170, PEBLM2000180, PEBLM2000213, PEBLM2000222, PEBLM2000248, PEBLM2000267, PEBLM2000270, PEBLM2000308, PEBLM2000321, PEBLM2000333, PEBLM2000338, PEBLM2000395, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004497, PEBLM2004666, PEBLM2004733, PEBLM2005282, PEBLM2005615,
PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006912, PEBLM2007112, PEBLM2007140, PEBLM2007188, PEBLM2007296, PEBLM2007437, PEBLM2007447, PEBLM2007598, PEBLM2007774, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287,
PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000058, PLACE5000066, PLACE5000067, PLACE5000068, PLACE5000070, PLACE5000080, PLACE5000105, PLACE5000115, PLACE5000116, PLACE5000129, PLACE5000139, PLACE5000153, PLACE5000171, PLACE5000172,
PLACE5000178, PLACE5000245, PLACE5000260, PLACE5000282, PLACE5000297, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000348, PLACE6000371, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001118, PLACE6001153, PLACE6001262, PLACE6001294, PLACE6001536, PLACE6001557, PLACE6001712, PLACE6001740, PLACE6001823, PLACE6001923, PLACE6001925, PLACE6001933, PLACE6002011, PLACE6002016, PLACE6002056, PLACE6002157, PLACE6002312,
PLACE6002323, PLACE6002345, PLACE6002419, PLACE6002462, PLACE6002647, PLACE6002692, PLACE6002699, PLACE6002710, PLACE6002871, PLACE6002949, PLACE6003004, PLACE6003038, PLACE6003077, PLACE6003094, PLACE6003109, PLACE6003204, PLACE6003372, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004101, PLACE6004219, PLACE6004336, PLACE6004368, PLACE6004380, PLACE6004396, PLACE6004454, PLACE6004464, PLACE6004578, PLACE6004663, PLACE6004687, PLACE6004931, PLACE6004966, PLACE6004993, PLACE6005007, PLACE6005029, PLACE6005108, PLACE6005231, PLACE6005234, PLACE6005283, PLACE6005294, PLACE6005328,
PLACE6005351, PLACE6005423, PLACE6005487, PLACE6005535, PLACE6005546, PLACE6005559, PLACE6005579, PLACE6005691, PLACE6005786, PLACE6006077, PLACE6006158, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008793, PLACE6008989,
PLACE6009006, PLACE6009008, PLACE6009228, PLACE6009237, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6013222, PLACE6013232, PLACE6013288, PLACE6013386,
PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015939, PLACE6016030, PLACE6016093, PLACE6016105, PLACE6016160, PLACE6016210, PLACE6016254, PLACE6016942, PLACE6016984, PLACE6017002, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6019195, PLACE6019385, PLACE6019542,
PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169, PLACE7005671, PLACE7005840, PLACE7006051, PLACE7006090, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110,
PROST1000120, PROST1000123, PROST1000136, PROST1000144, PROST1000145, PROST1000152, PROST1000167, PROST1000184, PROST1000215, PROST1000217, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000176, PROST2000203, PROST2000206, PROST2000212, PROST2000241, PROST2000267, PROST2000274, PROST2000277, PROST2000284, PROST2000325, PROST2000337,
PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001180, PROST2001213, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002682,
PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003324, PROST2003340, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004332, PROST2004481, PROST2004526, PROST2004570, PROST2004727, PROST2004739, PROST2004742, PROST2004744, PROST2004817, PROST2005039, PROST2005067, PROST2005076, PROST2005093,
PROST2005121, PROST2005131, PROST2005143, PROST2005228, PROST2005272, PROST2005426, PROST2005466, PROST2005630, PROST2005683, PROST2005788, PROST2005793, PROST2005880, PROST2005886, PROST2005904, PROST2005919, PROST2005943, PROST2006008, PROST2006020, PROST2006060, PROST2006196, PROST2006201, PROST2006260, PROST2006282, PROST2006343, PROST2006450, PROST2006491, PROST2006510, PROST2006579, PROST2006688, PROST2006766, PROST2006782, PROST2006933, PROST2006988, PROST2007122, PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988,
PROST2008079, PROST2008088, PROST2008243, PROST2008245, PROST2008268, PROST2008360, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010326, PROST2010382, PROST2010400, PROST2010579, PROST2010606, PROST2010885, PROST2011012, PROST2011038, PROST2011105, PROST2011297, PROST2011410, PROST2011439, PROST2011482, PROST2011577, PROST2011631, PROST2011660,
PROST2012005, PROST2012016, PROST2012088, PROST2012140, PROST2012157, PROST2012190, PROST2012249, PROST2012353, PROST2012400, PROST2012504, PROST2012542, PROST2012550, PROST2012740, PROST2012745, PROST2012780, PROST2012888, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900,
PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016980, PROST2017098, PROST2017128, PROST2017367, PROST2017413, PROST2017441, PROST2017529, PROST2017578, PROST2017612, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2019164, PROST2019253, PROST2019296, PROST2019398, PROST2019487, PROST2019781, PUAEN1000039,
PUAEN1000081, PUAEN1000085, PUAEN1000087, PUAEN1000121, PUAEN1000147, PUAEN1000161, PUAEN1000175, PUAEN1000275, PUAEN1000276, PUAEN1000288, PUAEN1000322, PUAEN2000080, PUAEN2000152, PUAEN2000175, PUAEN2000247, PUAEN2000312, PUAEN2000394, PUAEN2000497, PUAEN2000535, PUAEN2000594, PUAEN2000684, PUAEN2000942, PUAEN2001260, PUAEN2001345, PUAEN2001526, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029,
PUAEN2006328, PUAEN2006335, PUAEN2006578, PUAEN2006639, PUAEN2006761, PUAEN2007350, PUAEN2007785, PUAEN2007898, PUAEN2008123, PUAEN2008314, PUAEN2008515, PUAEN2008815, PUAEN2008939, PUAEN2008980, PUAEN2008985, PUAEN2009174, PUAEN2009348, PUAEN2009534, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000030, SKMUS1000064, SKMUS1000067, SKMUS1000102, SKMUS1000104, SKMUS1000110,
SKMUS1000118, SKMUS1000124, SKMUS1000129, SKMUS1000176, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000365, SKMUS2000390, SKMUS2000416, SKMUS2000458, SKMUS2000467, SKMUS2000468, SKMUS2000484, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000894, SKMUS2000898, SKMUS2000933, SKMUS2000945, SKMUS2000961, SKMUS2000973, SKMUS2000980, SKMUS2001008, SKMUS2001014, SKMUS2001147, SKMUS2001157, SKMUS2001164, SKMUS2001199,
SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001528, SKMUS2001543, SKMUS2001580, SKMUS2001585, SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667,
SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000159, SKNMC1000229, SKNMC1000264, SKNMC2000224, SKNMC2000256, SKNMC2000322, SKNMC2000356, SKNMC2000374, SKNMC2000583, SKNMC2000593, SKNMC2000612, SKNMC2000622, SKNMC2000698, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643,
SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000101, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000516, SKNSH2000550, SKNSH2000716, SKNSH2000971, SKNSH2001222, SKNSH2001875, SKNSH2001931, SKNSH2002054, SKNSH2002768, SKNSH2002866, SKNSH2003064, SKNSH2003174, SKNSH2003422, SKNSH2003466, SKNSH2003483, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822,
SKNSH2007142, SKNSH2007149, SKNSH2007429, SKNSH2007739, SKNSH2008940, SKNSH2008969, SKNSH2009197, SKNSH2009357, SKNSH2009435, SKNSH2009991, SKNSH2010015, SMINT1000012, SMINT1000016, SMINT1000039, SMINT1000048, SMINT1000051, SMINT1000054, SMINT1000057, SMINT1000071, SMINT1000075, SMINT1000078, SMINT1000091, SMINT1000103, SMINT1000118, SMINT1000137, SMINT1000192, SMINT2000007, SMINT2000018, SMINT2000032, SMINT2000040, SMINT2000060, SMINT2000073, SMINT2000084, SMINT2000145, SMINT2000176, SMINT2000185, SMINT2000227, SMINT2000229, SMINT2000232, SMINT2000239, SMINT2000267, SMINT2000400, SMINT2000454, SMINT2000518, SMINT2000529, SMINT2000530, SMINT2000537, SMINT2000538, SMINT2000541, SMINT2000558,
SMINT2000562, SMINT2000568, SMINT2000602, SMINT2000609, SMINT2000659, SMINT2000694, SMINT2000747, SMINT2000811, SMINT2000824, SMINT2000933, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2002126, SMINT2002202, SMINT2002210, SMINT2002224, SMINT2002281, SMINT2002311, SMINT2002328, SMINT2002414, SMINT2002457, SMINT2002620, SMINT2002743, SMINT2002880, SMINT2002882, SMINT2002884, SMINT2002899, SMINT2002974, SMINT2003003, SMINT2003074, SMINT2003128, SMINT2003150, SMINT2003317, SMINT2003340,
SMINT2003386, SMINT2003423, SMINT2003505, SMINT2003551, SMINT2003569, SMINT2003641, SMINT2003644, SMINT2003866, SMINT2003905, SMINT2004023, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004583, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716,
SMINT2007062, SMINT2007101, SMINT2007140, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921, SMINT2009119, SMINT2009121, SMINT2009212, SMINT2009216, SMINT2009233, SMINT2009259, SMINT2009272, SMINT2009292, SMINT2009363, SMINT2009415, SMINT2009468,
SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010997, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012179, SMINT2012195, SMINT2012285, SMINT2012291, SMINT2012501, SMINT2012734, SMINT2012793, SMINT2013032, SMINT2013129, SMINT2013170, SMINT2013181, SMINT2013228,
SMINT2013613, SMINT2013626, SMINT2013833, SMINT2013890, SMINT2014166, SMINT2014282, SMINT2014443, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2019017, SMINT2019105, SMINT2019142,
SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000247, SPLEN2000258, SPLEN2000267, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464,
SPLEN2000496, SPLEN2000515, SPLEN2000516, SPLEN2000537, SPLEN2000541, SPLEN2000607, SPLEN2000630, SPLEN2000695, SPLEN2000698, SPLEN2000874, SPLEN2000882, SPLEN2001135, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002467, SPLEN2002477, SPLEN2002493, SPLEN2002662,
SPLEN2002695, SPLEN2002707, SPLEN2002744, SPLEN2002917, SPLEN2002931, SPLEN2002957, SPLEN2003160, SPLEN2003204, SPLEN2003219, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004343, SPLEN2004346, SPLEN2004368, SPLEN2004555, SPLEN2004611, SPLEN2004662, SPLEN2004773, SPLEN2004844, SPLEN2004880, SPLEN2004984, SPLEN2005227, SPLEN2005272, SPLEN2005342, SPLEN2005416, SPLEN2005450, SPLEN2005474, SPLEN2005560, SPLEN2005727, SPLEN2005783, SPLEN2005790, SPLEN2005806, SPLEN2005818, SPLEN2005838, SPLEN2005869, SPLEN2005939, SPLEN2006268, SPLEN2006283, SPLEN2006325, SPLEN2006389, SPLEN2006425, SPLEN2006671,
SPLEN2006701, SPLEN2006785, SPLEN2006875, SPLEN2007182, SPLEN2007276, SPLEN2007314, SPLEN2007326, SPLEN2007350, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496, SPLEN2008591, SPLEN2008786, SPLEN2009081, SPLEN2009088, SPLEN2009163, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415,
SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012846, SPLEN2012889,
SPLEN2012961, SPLEN2013108, SPLEN2013195, SPLEN2013503, SPLEN2013644, SPLEN2013648, SPLEN2013670, SPLEN2013673, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258,
SPLEN2015261, SPLEN2015267, SPLEN2015276, SPLEN2015310, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016480, SPLEN2016531, SPLEN2016533, SPLEN2016541, SPLEN2016552, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212,
SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018112, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019257, SPLEN2019323, SPLEN2019349, SPLEN2019375, SPLEN2019379, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956,
SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273,
SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397,
SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036953,
SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041645, SPLEN2041700, SPLEN2041720, SPLEN2041977, SPLEN2042051, SPLEN2042303, SPLEN2042521, SPLEN2042535, SPLEN2042598, SPLEN2042920, STOMA1000013, STOMA1000047, STOMA1000052, STOMA1000074, STOMA1000091, STOMA1000117, STOMA1000133, STOMA1000175, STOMA1000190, STOMA1000191, STOMA2000032, STOMA2000072, STOMA2000088, STOMA2000104,
STOMA2000121, STOMA2000148, STOMA2000168, STOMA2000183, STOMA2000188, STOMA2000257, STOMA2000279, STOMA2000383, STOMA2000386, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002141, STOMA2002166, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2005120, STOMA2005664, STOMA2005667, STOMA2005746, STOMA2005782, STOMA2006181, STOMA2006213, STOMA2006229, STOMA2006325,
STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001251,
SYNOV2001300, SYNOV2001356, SYNOV2001374, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2009172, SYNOV2011233, SYNOV2012326, SYNOV2013365, SYNOV2013637, SYNOV2014157, SYNOV2014364, SYNOV2016124, SYNOV2016837, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000706, SYNOV4001153, SYNOV4001326, SYNOV4001342, SYNOV4002001, SYNOV4002392,
SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007671, SYNOV4007711, SYNOV4008245, SYNOV4008336, SYNOV4009129, SYNOV4009139, SYNOV4009298, T1ESE2000116, T1ESE2000609, T1ESE2002665, TBAES2000004, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435,
TBAES2003492, TBAES2003550, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474,
TESOP2001796, TESOP2001818, TESOP2001849, TESOP2001865, TESOP2001901, TESOP2001953, TESOP2002005, TESOP2002110, TESOP2002150, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552,
TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018,
TESTI2000044, TESTI2000100, TESTI2000117, TESTI2000120, TESTI2000147, TESTI2000154, TESTI2000172, TESTI2000177, TESTI2000179, TESTI2000207, TESTI2000208, TESTI2000221, TESTI2000238, TESTI2000253, TESTI2000272, TESTI2000278, TESTI2000349, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000849, TESTI2000850, TESTI2000874,
TESTI2000883, TESTI2000909, TESTI2000951, TESTI2000981, TESTI2001008, TESTI2001049, TESTI2001082, TESTI2001120, TESTI2001134, TESTI2001141, TESTI2001180, TESTI2001208, TESTI2001229, TESTI2001236, TESTI2001237, TESTI2001269, TESTI2001306, TESTI2001313, TESTI2001330, TESTI2001339, TESTI2001345, TESTI2001352, TESTI2001364, TESTI2001412, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001869, TESTI2001879,
TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002676, TESTI2002690, TESTI2002696, TESTI2002698, TESTI2002700, TESTI2002707, TESTI2002724, TESTI2002729,
TESTI2002752, TESTI2002753, TESTI2002789, TESTI2002802, TESTI2002806, TESTI2002840, TESTI2002866, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372,
TESTI2003376, TESTI2003413, TESTI2003418, TESTI2003419, TESTI2003432, TESTI2003454, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004153, TESTI2004163, TESTI2004169,
TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005017, TESTI2005025, TESTI2005040, TESTI2005060,
TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005739, TESTI2005742, TESTI2005743, TESTI2005767, TESTI2005775, TESTI2005784, TESTI2005788, TESTI2005827, TESTI2005835, TESTI2005860, TESTI2005892, TESTI2005911,
TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465, TESTI2006483, TESTI2006499, TESTI2006523, TESTI2006543, TESTI2006565, TESTI2006572, TESTI2006615,
TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007311, TESTI2007320, TESTI2007346, TESTI2007358, TESTI2007402, TESTI2007405, TESTI2007422, TESTI2007452, TESTI2007464, TESTI2007466, TESTI2007480, TESTI2007490, TESTI2007502, TESTI2007524,
TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2007998, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621,
TESTI2008636, TESTI2008657, TESTI2008684, TESTI2008699, TESTI2008715, TESTI2008762, TESTI2008774, TESTI2008786, TESTI2008822, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835, TESTI2009853, TESTI2009935,
TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011712, TESTI2011750, TESTI2011840, TESTI2011846, TESTI2011915, TESTI2011928, TESTI2011971,
TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012778, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013295, TESTI2013322, TESTI2013381, TESTI2013382, TESTI2013401, TESTI2013427,
TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015335,
TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102,
TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042,
TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020905, TESTI2020906, TESTI2020946,
TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021138, TESTI2021297, TESTI2021315, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874,
TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936,
TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491,
TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269,
TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418,
TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953,
TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657,
TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642,
TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276,
TESTI2044306, TESTI2044309, TESTI2044413, TESTI2044418, TESTI2044754, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047792, TESTI2047801, TESTI2047818,
TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TEST'12052525, TESTI2052670, TESTI2052693, TESTI2052698, TESTI2052799, TESTI2052822, TESTI2052985, TESTI2053151, TESTI2053242,
TESTI2053399, TESTI2053526, TESTI2053561, TESTI2053621, TESTI2053667, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527,
TESTI4001561, TESTI4001569, TESTI4001604, TESTI4001665, TESTI4001679, TESTI4001795, TESTI4001923, TESTI4001925, TESTI4001984, TESTI4002003, TESTI4002141, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003733, TESTI4003796, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158,
TESTI4005317, TESTI4005322, TESTI4005470, TESTI4005500, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965,
TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789,
TESTI4010817, TESTI4010831, TESTI4010851, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013685, TESTI4013735, TESTI4013742, TESTI4013774, TESTI4013817, TESTI4013830, TESTI4013852,
TESTI4013924, TESTI4013960, TESTI4013962, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254,
TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569,
TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139,
TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856,
TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TE-STI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339, TESTI4038492, TESTI4038721, TESTI4038758,
TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371, TESTI4043378, TESTI4043512, TESTI4043551,
TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017,
THYMU1000025, THYMU1000029, THYMU1000032, THYMU1000033, THYMU1000060, THYMU1000074, THYMU1000098, THYMU1000105, THYMU1000136, THYMU1000144, THYMU1000158, THYMU1000176, THYMU1000179, THYMU1000191, THYMU1000198, THYMU1000217, THYMU1000240, THYMU1000242, THYMU1000291, THYMU1000318, THYMU1000329, THYMU1000331, THYMU10003-36, THYMU1000366, THYMU1000382, THYMU1000393, THYMU1000399, THYMU1000426, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140,
THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000702, THYMU2000767, THYMU2000775, THYMU2000800, THYMU2000879, THYMU2000932, THYMU2000946, THYMU2000950, THYMU2000971, THYMU2000982, THYMU2000990, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001443,
THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002841, THYMU2002852, THYMU2002910, THYMU2002950, THYMU2002983, THYMU2003069, THYMU2003107, THYMU2003124, THYMU2003133, THYMU2003166, THYMU2003232, THYMU2003242,
THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004344, THYMU2004356, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003,
THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965,
THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452,
THYMU2008643, THYMU2008691, THYMU2008714, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010161, THYMU2010192, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072,
THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023,
THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015439, THYMU2015441, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360,
THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019364, THYMU2019436, THYMU2019442, THYMU2019587, THYMU2019599,
THYMU2019686, THYMU2019813, THYMU2020198, THYMU2020289, THYMU2020416, THYMU2020491, THYMU2020499, THYMU2020560, THYMU2020830, THYMU2020883, THYMU2020959, THYMU2021361, THYMU2021509, THYMU2021597, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024121, THYMU2024185, THYMU2024207, THYMU2024616, THYMU2024684, THYMU2024748, THYMU2025042, THYMU2025189, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053,
THYMU2027125, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031994, THYMU2032014, THYMU2032035, THYMU2032080, THYMU2032150,
THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036215, THYMU2036252, THYMU2036265, THYMU2036366, THYMU2036459, THYMU2036604, THYMU2036653, THYMU2037081, THYMU2037208,
THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390,
THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001593, THYMU3001883, THYMU3001939, THYMU3001991, THYMU3002452, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371,
THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701,
THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642,
THYMU3025683, THYMU3025772, THYMU3025865, THYMU3026000, THYMU3026479, THYMU3026532, THYMU3026783, THYMU3026869, THYMU3027251, THYMU3027540, THYMU3027655, THYMU3027671, THYMU3028075, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032867, THYMU3033402, THYMU3033626, THYMU3033630, THYMU3033649, THYMU3033754, THYMU3033759, THYMU3034078, THYMU3034099, THYMU3034453, THYMU3034853, THYMU3034867, THYMU3034983, THYMU3036200, THYMU3036310, THYMU3036934, THYMU3036953,
THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482,
THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078,
TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161,
TKIDN2015263, TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192,
TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000057, TRACH1000074, TRACH1000100, TRACH1000106, TRACH1000125, TRACH1000140, TRACH1000144, TRACH1000193, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235,
TRACH2000243, TRACH2000250, TRACH2000259, TRACH2000272, TRACH2000282, TRACH2000287, TRACH2000289, TRACH2000306, TRACH2000312, TRACH2000336, TRACH2000393, TRACH2000411, TRACH2000420, TRACH2000496, TRACH2000497, TRACH2000502, TRACH2000522, TRACH2000540, TRACH2000559, TRACH2000660, TRACH2000665, TRACH2000675, TRACH2000677, TRACH2000702, TRACH2000703, TRACH2000767, TRACH2000775, TRACH2000807, TRACH2000862, TRACH2000873, TRACH2000894, TRACH2000898, TRACH2000920, TRACH2000923, TRACH2000926, TRACH2000944, TRACH2000972, TRACH2000981, TRACH2000986, TRACH2001037, TRACH2001060, TRACH2001085, TRACH2001101, TRACH2001109, TRACH2001121, TRACH2001147, TRACH2001154, TRACH2001188, TRACH2001249, TRACH2001275,
TRACH2001314, TRACH2001335, TRACH2001339, TRACH2001341, TRACH2001347, TRACH2001395, TRACH2001400, TRACH2001403, TRACH2001416, TRACH2001428, TRACH2001474, TRACH2001478, TRACH2001523, TRACH2001525, TRACH2001532, TRACH2001592, TRACH2001607, TRACH2001621, TRACH2001646, TRACH2001651, TRACH2001684, TRACH2001898, TRACH2001933, TRACH2001968, TRACH2001996, TRACH2002043, TRACH2002092, TRACH2002100, TRACH2002138, TRACH2002333, TRACH2002664, TRACH2002784, TRACH2002803, TRACH2002840, TRACH2002847, TRACH2002853, TRACH2002862, TRACH2002954, TRACH2002960, TRACH2002988, TRACH2003070, TRACH2003347, TRACH2003484, TRACH2003516, TRACH2003605, TRACH2003736, TRACH2003759, TRACH2003941, TRACH2004039, TRACH2004054,
TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577,
TRACH2007674, TRACH2007676, TRACH2007688, TRACH2007733, TRACH2007754, TRACH2007816, TRACH2007894, TRACH2007969, TRACH2007988, TRACH2008081, TRACH2008112, TRACH2008113, TRACH2008127, TRACH2008130, TRACH2008278, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587,
TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045,
TRACH2014046, TRACH2014077, TRACH2014082, TRACH2014268, TRACH2014284, TRACH2014337, TRACH2014356, TRACH2014371, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498,
TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839,
TRACH2023246, TRACH2023299, TRACH2023306, TRACH2023416, TRACH2023479, TRACH2023684, TRACH2023860, TRACH2023876; TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000780,
TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003586, TRACH3003683, TRACH3003780, TRACH3003805, TRACH3003832, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085,
TRACH3005102, TRACH3005173, TRACH3005191, TRACH3005211, TRACH3005274, TRACH3005294, TRACH3005479, TRACH3005549, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006889, TRACH3006985, TRACH3007097, TRACH3007268, TRACH3007274, TRACH3007277, TRACH3007391, TRACH3007541, TRACH3007595, TRACH3007625, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008688,
TRACH3008697, TRACH3008713, TRACH3008887, TRACH3008902, TRACH3008990, TRACH3009001, TRACH3009059, TRACH3009061, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766,
TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662,
TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148,
TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065,
TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037067, TRACH3037267, TRACH3037288, TRACH3037505, TRACH3037573, TRACH3037696, TRACH3037897, TRACH3038086,
TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682

### Homology search data

Homology search data for full-length nucleotide sequences and the deduced amino acid sequences are shown below. The search results contain two types of length unit for the sequences compared: aa and bb.

Each piece of data is punctuated with a double slash mark (//) and shown in order: Sequence Name, Definition of hit data, P value, Length of sequence compared, Homology, and Accession Number of hit data. Sequences which did not show hit data in the homology search list only the Clone name.
3NB692002685// Homo sapiens sperm acrosomal protein mRNA, complete cds.// 7.3E-117// 227aa// 100%// AF047437
3NB692002806// Dihydropteridine reductase (EG 1.6.99.7) (HDHPR) (Quinoid dihydropteridine reductase).// 1.00E-105// 192aa// 78%// P09417
3NB692008729
ACTVT2000380
ADIPS2000088// Human (hybridoma H210) anti-hepatitis A IgG variable region, constant region, complementarity-determining regions mRNA, complete cds.// 3.5E-236// 477aa// 90%// M87789
ADRGL2000172
ADRGL2003329
ADRGL2009146
ADRGL2009691// Mus musculus D11lgp1 mRNA, complete cds.// 5.1E-17// 75aa// 60%// AF316996
ADRGL2009755// Homo sapiens brain and reproductive or gan-expressed protein (BRE) mRNA, complete cds.// 2.2E-156// 308aa// 93%// AF015767
ADRGL2012038
ADRGL2012179
ASTRO1000009// BAND 4.1-LIKE PROTEIN 4 (NBL4 PROTEIN).// 2.50E-45// 309aa// 38%// 057457
ASTRO2002842// sentrin/SUMO-specific protease [Mus musculus]// 2.00E-84// 148aa// 54%// NM_030702
ASTRO2003960
ASTRO2014923// hypothetical protein // 2.6E-66// 133aa// 100%// CAB70760 ASTRO2018373
ASTRO3000172
ASTRO3000177// Drosophila melanogaster BcDNA.GH03694 (BcDNA.GH03694) mRNA, complete cds.// 1.30E-89// 385aa// 47%// AF181626
ASTRO3000301// G2 [Homo sapiens]// 0// 1073aa// 96%// AAA21253
ASTRO3000482
BLADE1000176
BLADE2001371
BLADE2001987
BLADE2002073
BLADE2002782// Rattus norvegicus clone C53 CDK5 activator-binding protein mRNA, complete cds.// 1.90E-24// 70aa// 82%// AF177476
BLADE2002947
BLADE2003474
BLADE2004089// Mus musculus PDZ domain actin binding protein Shroom mRNA, complete cds. // 4.20E-244// 784aa// 64%// AF199421
BLADE2004462
BLADE2004670
BLADE2005036// CARBOXYPEPTIDASE A1 PRECURSOR (EC 3.4.17.1).// 1.20E-81// 168aa// 93%// P15085
BLADE2005459
BLADE2007666
BLADE2007958// Homo sapiens U5 snRNP-specific 40 kDa protein mRNA, complete cds// 6.0E-91// 190aa// 99%// NP_004805
BLADE2008281
BLADE2008398// Homo sapiens LRR FLI-I interacting protein 2 (LRRFIP2) mRNA, complete cds.// 2.00E-98// 200aa// 99%// AF115509
BLADE2008539// tumor antigen SLP-8p [Homo sapiens]// 0// 427aa// 95%//
NM_016516
BNGH42003570// Mus musculus mRNA for thrombospondin type 1 domain, complete cds.// 2.30E-44// 239aa// 39%// AB016768
BNGH42007788// VEGETATIBLE INCOMPATIBILITY PROTEIN HET-E-1.// 4.1E-40// 242aa// 37%// Q00808
BRACE1000186// g20 protein [Homo sapiens]// 3.00E-74// 138aa// 85%// NM_016210
BRACE1000258
BRACE1000533
BRACE1000572
BRACE2003639
BRACE2005457// PENDRIN.// 1.30E-61// 371aa// 35%// 043511
BRACE2006319// Homo sapiens mRNA for Fln29, complete cds.// 3E-157// 283aa// 99%// AB007447
BRACE2008594// Human protein serine/threonine kinase stk2 mRNA, complete cds.// 1.90E-39// 190aa// 44%// L20321
BRACE2010489// Mus musculus nucleolar protein C7 mRNA, complete cds.// 1.70E-149// 391aa// 76%// AF324899
BRACE2011747
BRACE2014306// Chromaffin granule amine transporter (Vesicular amine transporter 1) (VAT1).// 1.00E-15// 80aa// 22%// P54219
BRACE2014475
BRACE2014657
BRACE2015058
BRACE2015314
BRACE2016981// Sarcolemmal-associated protein [Homo sapiens]// 7.00E-18// 72aa// 29%// NM_007159
BRACE2018762// Homer, neuronal immediate early gene, 3 [Homo sapiens]// 1.00E-144// 267aa// 77%// NM_004838
BRACE2024627
BRACE2026836// Mus musculus mRNA for smoothelin L1, large isoform.// 8.00E-66// 220aa// 55%// AJ010305
BRACE2027258// Homo sapiens E2a-Pbx1-associated protein (EB-1) mRNA, partial cds.// 5.80E-163// 302aa// 99%// AF145204
BRACE2027970
BRACE2028970
BRACE2029112// Homo sapiens mRNA for plexin-B1 plasma membrane receptor, splice variant R (plexin-B1/SEP gene)// 4.0E-99// 119aa// 62%// AJ011415 BRACE2029849
BRACE2030326// OOCYTE ZINC FINGER PROTEIN XLCOF6 (FRAGMENT).// 1.40E-07// 106aa// 33%// P18749
BRACE2030341// Homo sapiens protein kinase STK10 mRNA, partial cds.// 2.60E-66// 129aa// 100%// AF170723
BRACE2030884
BRACE2031154
BRACE2031389
BRACE2031527
BRACE2031531
BRACE2031899
BRACE2032044
BRACE2032329
BRACE2032385
BRACE2032538
BRACE2032823
BRACE2033720
BRACE2035381// Homo sapiens phospholipase A2, group IVB (cytosolic) (PLA2G4B),mRNA// 0// 446aa// 96%// NM_005090
BRACE2035441
BRACE2036005
BRACE2036096
BRACE2036830
BRACE2036834
BRACE2037847
BRACE2038114
BRACE2038329// Rattus norvegicus CBL-B (Cbl-b) mRNA, partial cds.// 3.4E-96// 198aa// 92%// AF199504
BRACE2038551// PROTEIN ARGININE N-METHYLTRANSFERASE 2 (EC 2.1.1.-).// 6.40E-53// 102aa// 100%// P55345
BRACE2039249// POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N-ACETYLGALACTOSAMINYLTRANSFERASE) (GALNAC-T1).// 5.1E-99// 199aa// 88%// Q10472
BRACE2039327// hypothetical protein FLJ20241 // 6.3E-47// 97aa// 100%// XP_012789
BRACE2039475
BRACE2039734
BRACE2040138
BRACE2040325
BRACE2041009
BRACE2041200// PROTEIN-ARGININE DEIMINASE TYPE II (EC 3.5.3.15) (PEPTIDYLARGININE DEIMINASE II) (KIAA0994).// 5.9E-98// 181aa// 98%// Q9Y2J8 BRACE2041264
BRACE2042550// Bos taurus mRNA for SCO-spondin, partial.// 1.8E-154// 319aa// 80%// AJ133488
BRACE2043142
BRACE2043248// Human I (3) mbt protein homolog mRNA, complete cds.// 7.1E-71// 140aa// 98%// U89358
BRACE2043349
BRACE2043665
BRACE2044286// GUANINE NUCLEOTIDE EXCHANGE FACTOR DBS (DBL'S BIG SISTER) (MCF2 TRANSFORMING SEQUENCE-LIKE PROTEIN).// 7.20E-154// 578aa// 50%// Q64096
BRACE2044816
BRACE2044949
BRACE2045300// COFILIN, NON-MUSCLE ISOFORM.// 3.9E-85// 166aa// 100%// P23528
BRACE2045428
BRACE2045596// CTD-BINDING SR-LIKE PROTEIN RA4 (FRAGMENT).// 1.6E-08// 160aa// 32%// Q63627
BRACE2045772// 2-AMINO-3-KETOBUTYRATE COENZYME A LIGASE, MITOCHONDRIAL PRECURSOR (EG 2. 3. 1. 29) (AKB LIGASE) (GLYCINE ACETYLTRANSFERASE).// 1.3E-107// 142aa// 98%// 075600
BRACE2045947
BRACE2045954
BRACE2046251// Homo sapiens hucep-10 mRNA for cerebral protein-10, complete cds.// 4.3E-135// 264aa// 97%// AB000782
BRACE2046295// NTAK PROTEIN (NEURAL- AND THYMUS-DERIVED ACTIVATOR FOR ERBB KINASES).// 1.80E-165// 304aa// 99%// 014511
BRACE2047011
BRACE2047350
BRACE2047377// ras inhibitor [Homo sapiens]// 4.40E-11// 60aa// 58%// AAA36553
BRACE2047385
BRACE3000071
BRACE3000697
BRACE3000787
BRACE3000840// Baculoviral IAP repeat-containing protein 1 (Neuronal apoptosis inhibitory protein).// 0// 979aa// 96%// Q13075
BRACE3000973
BRACE3001002// Zinc finger X-linked protein ZXDB.// 1.00E-45// 97aa// 70%// P98169
BRACE3001217
BRACE3001391// Polycystin precursor (Autosomal dominant polycystic kidney disease protein 1).// 0// 388aa// 84%// P98161
BRACE3001595
BRACE3001754// Surfeit locus protein 5.// 2.00E-87// 166aa// 83%// Q15528
BRACE3002298
BRACE3002390
BRACE3002508// WD40 repeat domain 11 protein [Homo sapiens]// 1.00E-113// 200aa// 98%// NM_018117
BRACE3003004
BRACE3003192// latent transforming growth factor beta binding protein 3 [Homo sapiens]// 0// 670aa// 91%// NM_021070
BRACE3003595// CRK-associated substrate (p130Cas) (Breast cancer anti-estrogen resistance 1 protein).// 0// 712aa// 84%// P56945
BRACE3003698
BRACE3004058// NADH-cytochrome B5 reductase (EG 1.6.2.2) (B5R).// 1.00E-129// 220aa// 96%// P00387
BRACE3004113
BRACE3004150// Heterogeneous nuclear ribonucleoprotein H (hnRNP H).// 0// 390aa// 86%// P31943
BRACE3004358
BRACE3004435
BRACE3004772// protein tyrosine phosphatase, receptor type, f polypeptide (PTPRF), interacting protein (liprin), alpha 2 [Homo sapiens]// 1.00E-101// 168aa// 80%// NM_003625
BRACE3004783
BRACE3004843
BRACE3004880
BRACE3005145
BRACE3005225
BRACE3005430// DNA repair protein rad18.// 4.00E-21// 48aa// 38%// P53692
BRACE3005499
BRACE3006185
BRACE3006226
BRACE3006462
BRACE3006872// Echinoderm microtubule-associated protein-like 2 (EMAP-2) (HuEMAP-2).// 1.00E-46// 94aa// 34%// 095834
BRACE3007322
BRACE3007472
BRACE3007480
BRACE3007559
BRACE3007625// espin [Rattus norvegicus]// 1.00E-101// 178aa// 54%// NM_019622
BRACE3007642
BRACE3007767
BRACE3008036
BRACE3008092
BRACE3008137// Interleukin-16 precursor (IL-16) (Lymphocyte chemoattractant factor) (LCF).// 0// 530aa// 83%// Q14005
BRACE3008384// rhomboid (veinlet, Drosophila)-like; Rhomboid, drosophila, homolog of [Homo sapiens]// 7.00E-94// 158aa// 51%// NM_003961
BRACE3008720// SPOOB-associated GTP-binding protein.// 5.00E-33// 80aa// 43%// P20964
BRACE3008772
BRACE3009090
BRACE3009237
BRACE3009297// mdgl-1 [Mus musculus]// 6.00E-54// 110aa// 84%// AAG17143
BRACE3009377
BRACE3009574
BRACE3009701
BRACE3009708// Sodium/potassium-transporting ATPase alpha-2 chain precursor (EC 3.6.3.9) (Sodium pump 2) (Na+/K+ ATPase 2).// 0// 868aa// 98%// P50993
BRACE3009724
BRACE3009747// DAZ associated protein 1 [Homo sapiens]// 2.00E-18// 43aa// 57%// NM_018959
BRACE3010397// Glioma pathogenesis-related protein (RTVP-1 protein).// 4.00E-29// 65aa// 37%// P48060
BRACE3010428
BRACE3011271// Patched protein homolog 1 (PTC1) (PTC).// 5.00E-84// 147aa// 72%// Q13635
BRACE3011421// Diacylglycerol kinase, zeta (EC 2.7.1.107) (Diglyceride kinase) (DGK- zeta) (DAG kinase zeta).// 0// 839aa// 91%// Q13574
BRACE3011505
BRACE3012364
BRACE3012930
BRACE3013119
BRACE3013576
BRACE3013740
BRACE3013780
BRACE3014005// thymic stromal cotransporter; putative thymic stromal cotransporter TSCOT [Mus musculus]// 4.00E-21// 71aa// 23%// NM_021053
BRACE3014068
BRACE3014231
BRACE3014317
BRACE3014807// homolog 1; tweety homolog [Homo sapiens]// 3.00E-38// 75aa// 46%// NM_020659
BRACE3015027// Ras GTPase-activating protein nGAP.// 3.00E-75// 214aa// 35%// Q9UJF2
BRACE3015121
BRACE3015262// espin [Mus musculus]// 1.00E-10// 55aa// 40%// AAF98134
BRACE3015521// EH-domain containing protein 1 (Testilin) (hPASTI).// 1.00E-121// 215aa// 93%// Q9H4M9
BRACE3015894
BRACE3016884// Glucoamylase S1/S2 precursor (EC 3.2.1.3) (Glucan 1,4-alpha-glucosidase) (1,4-alpha-D-glucan glucohydrolase).// 8.00E-07// 87aa// 17%// P08640
BRACE3018308
BRACE3018963// Exostos in-like 3 (Putative tumour suppressor protein EXTL3) (Multiple exostosis-like protein 3) (Hereditary multiple exostoses gene isolog) (EXT-related protein 1).// 6.00E-39// 69aa// 98%// 043909
BRACE3019055
BRACE3019084// protein tyrosine phosphatase, receptor type, f polypeptide (PTPRF), interacting protein (liprin), alpha 1 [Homo sapiens]// 1.00E-130// 226aa// 93%// NM_003626
BRACE3020194
BRACE3020286
BRACE3020594// cytokine receptor-like factor 2: cytokine receptor CRL2 precusor [Homo sapiens]// 1.00E-27// 53aa// 73%// NM_022148
BRACE3022769
BRACE3023912
BRACE3024073// hypothetical protein similar to small G proteins, especially RAP-2A [Homo sapiens]// 8.00E-36// 77aa// 48%// NM_021183
BRACE3024659
BRACE3024662
BRACE3025153
BRACE3025457// testis-specific protein TSP-NY [Homo sapiens]// 0// 413aa// 97%// NM_032573
BRACE3025531
BRACE3025630// Alkaline serine exoprotease A precursor (EC 3.4.21.-).// 4.00E-18// 53aa// 33%// P16588
BRACE3026008
BRACE3026075
BRACE3026735
BRACE3027242
BRACE3027326// RAP1 GTPase activating protein 1 (RAP1GAP).// 1.00E-177// 331aa// 50%// P47736
BRACE3027478
BRACE3030103
BRACE3031838
BRACE3032983
BRACE3040856// Golgi Transport: Got1p [Saccharomyces cerevisiae]// 2.00E-13// 100aa// 45%// NP_014020
BRACE3045033// polyamine-modulated factor 1; polyamine modulated factor 1 [Homo sapiens]// 1.00E-41// 82aa// 100%// NM_007221
BRALZ2011796
BRALZ2012183
BRALZ2012848
BRALZ2014484
BRALZ2016085// MONOCARBOXYLATE TRANSPORTER 3 (MCT 3) (RETINAL EPITHELIAL MEMBRANE PROTEIN).// 4.2E-49// 244aa// 33%// Q90632
BRALZ2016498// Homo sapiens FKSG76 (FKSG76) mRNA, complete cds.// 4.8E-65// 127aa// 99%// AF345564
BRALZ2017359// Homo sapiens RNA helicase HDB/DICE1 mRNA, partial cds.// 4.4E-62// 318aa// 47%// AF141326
BRAMY2001473// Rattus norvegicus transmembrane receptor Unc5H2 mRNA, complete cds.// 3.00E-115// 501aa// 44%// U87306
BRAMY2003008
BRAMY2004771// CHONDROADHERIN PRECURSOR (CARTILAGE LEUCINE-RICH PROTEIN) (38 KDA BONE PROTEIN).// 2.30E-36// 293aa// 34%// Q27972
BRAMY2005052// COXSACKIEVIRUS AND ADENOVIRUS RECEPTOR PRECURSOR (COXSACKIEVIRUS B- ADENOVIRUS RECEPTOR) (HCAR) (CVB3 BINDING PROTEIN).// 3.10E-37// 200aa// 35%// P78310
BRAMY2017528
BRAMY2019300// CHONDROADHERIN PRECURSOR (CARTILAGE LEUCINE-RICH PROTEIN) (38 KDA BONE PROTEIN).// 8.10E-32// 350aa// 28%// Q27972
BRAMY2019963
BRAMY2019985
BRAMY2020058
BRAMY2020270
BRAMY2021498// Mus musculus papilin mRNA, complete cds.// 3E-89// 546aa// 37%// AF314171
BRAMY2028856
BRAMY2028914
BRAMY2029602
BRAMY2030098
BRAMY2030109// Homo sapiens hucep-4 mRNA for cerebral protein-4, complete cds.// 7.6E-83// 280aa// 55%// D88259
BRAMY2030702
BRAMY2030703
BRAMY2030799
BRAMY2031317// Mus musculus semaphorin cytoplasmic domain-associated protein 3A (Semcap3) mRNA, complete cds.// 2.6E-169// 682aa// 50%// AF127084
BRAMY2031377
BRAMY2031442
BRAMY2032014
BRAMY2032242
BRAMY2032317
BRAMY2033003// solute carrier family 22 (organic anion transporter), member 8 // 5.1E-25// 60aa// 100%// XP_006015
BRAMY2033116
BRAMY2033267// Rattus norvegicus mRNA for acetoacetyl-CoA synthetase, complete// 1.7E-42// 148aa// 66%// AB026291
BRAMY2033594
BRAMY2034185
BRAMY2034920
BRAMY2034993
BRAMY2036387
BRAMY2036396
BRAMY2036567// GUANINE NUCLEOTIDE EXCHANGE FACTOR DBS (DBL'S BIG SISTER) (MCF2 TRANSFORMING SEQUENCE-LIKE PROTEIN).// 5.3E-47// 113aa// 83%// Q64096
BRAMY2036699
BRAMY2036913
BRAMY2037823
BRAMY2038100
BRAMY2038484// Homo sapiens MDC-3.13 isoform 2 mRNA, complete cds.// 3.8E-65// 194aa// 60%// AF099935
BRAMY2038846
BRAMY2038904// Homo sapiens mRNA for X-like 1 protein.// 2.7E-199// 738aa// 55%// AJ005821
BRAMY2039872// Homo sapiens protein phosphatase methylesterase-1 (PME-1) mRNA, complete cds.// 4.1E-43// 84aa// 98%// AF157028
BRAMY2040478
BRAMY2040592// Homo sapiens hucep-11 mRNA for cerebral protein-11, complete cds.// 2.20E-101// 327aa// 64%// AB001596
BRAMY2041261
BRAMY2041378
BRAMY2041542
BRAMY2042612
BRAMY2042641
BRAMY2042760
BRAMY2042918
BRAMY2044078
BRAMY2044246
BRAMY2045036
BRAMY2046478
BRAMY2046742
BRAMY2046989// Protein bimA.// 4.00E-07// 60aa// 20%// P17885
BRAMY2047169
BRAMY2047420
BRAMY2047676
BRAMY2047746// nasopharyngeal carcinoma susceptibility protein [Homo sapiens]// 3.00E-18// 50aa// 42%// NM_013275
BRAMY2047751// REGULATOR OF G-PROTEIN SIGNALING 12 (RGS12).// 1.3E-178// 336aa// 100%// 014924
BRAMY2047765
BRAMY2047884
BRAMY3000206
BRAMY3000213// Homo sapiens RaP2 interacting protein 8 (RPIP8) mRNA, complete cds.// 5.7E-85// 167aa// 99%// U93871
BRAMY3001401
BRAMY3001794// Rattus norvegicus Circadian Oscillatory Protein (SCOP) (Scop)// 1.00E-117// 206aa// 59%// NM_021657
BRAMY3002312
BRAMY3002620
BRAMY3002803// p21-activated protein kinase 6 [Homo sapiens]// 0// 598aa// 87%// NM_020168
BRAMY3002805
BRAMY3004224
BRAMY3004672
BRAMY3004900
BRAMY3004919// Adenylate cyclase, type V (EC 4.6.1.1) (ATP pyrophosphatelyase) (Adenylyl cyclase).// 0// 831aa// 91%// Q04400
BRAMY3005091// Phosphatidylinositol 4-kinase alpha (EC 2.7.1.67) (P14-kinase) (Ptdlns-4-kinase) (P14K-alpha).// 1.00E-111// 192aa// 100%// P42356
BRAMY3005932// Diacylglycerol kinase, zeta (EC 2.7.1.107) (Diglyceride kinase) (DGK- zeta) (DAG kinase zeta) (DGK-IV) (104 kDa diacylglycerol kinase).// 1.00E-35// 71aa// 98%// 008560
BRAMY3006297
BRAMY3007206
BRAMY3007609// nuclear RNA export factor 2; TAP like protein 2 [Homo sapiens]// 2.00E-80// 155aa// 60%// NM_017809
BRAMY3008466// kinesin family member 21A [Mus musculus]// 1.00E-167// 277aa// 94%// NM_016705
BRAMY3008505
BRAMY3008650
BRAMY3009811
BRAMY3010411
BRAMY4000095// ERBB-3 receptor protein-tyrosine kinase precursor (EC 2.7.1.112) (Tyrosine kinase-type cell surface receptor HER3).// 0// 469aa// 80%// P21860
BRAMY4000229// CORONIN 2A (WD-REPEAT PROTEIN 2) (IR10).// 2.40E-87// 167aa// 71%// Q92828
BRAMY4000277// Alpha-1B-glycoprotein.// 1.00E-163// 276aa// 100%// P04217
BRASW1000053
BRASW1000125
BRAWH1000127// Mus musculus mRNA for thrombospondin type 1 domain, complete cds.// 8.30E-49// 204aa// 43%// AB016768
BRAWH2001395// MYELIN BASIC PROTEIN (MBP).// 7.90E-26// 64aa// 90%// P02686
BRAWH2001671
BRAWH2001940// H.sapiens gene from PAC 1026E2, partial.// 0// 640aa// 98%// AL035289
BRAWH2001973
BRAWH2002560// deleted in bladder cancer chromosome region candidate 1 [Homo sapiens].// 0// 770aa// 52%// NP_055433
BRAWH2002761
BRAWH2005315// neuronal-STOP protein // 5.5E-119// 441aa// 56%// CAA75930
BRAWH2007658// COXSACKIEVIRUS AND ADENOVIRUS RECEPTOR HOMOLOG PRECURSOR (MCAR).// 8.80E-14// 366aa// 24%// P97792
BRAWH2010000// golgi autoantigen, golgin subfamily a, 3; golgin-160 [Homo sapiens]// 5.00E-15// 148aa// 23%// NM_005895
BRAWH2010084
BRAWH2010536// paraneoplastic antigen MA1// 5.00E-18// 100aa// 42%// NP_006020
BRAWH2012162// KE03 protein [Homo sapiens]// 2.00E-97// 330aa// 55%// AAC17109
BRAWH2012326
BRAWH2013294
BRAWH2013871
BRAWH2014414// Cadherin-4 precursor (Retinal-cadherin) (R-cadherin) (R-CAD).// 0// 761aa// 90%// P55283
BRAWH2014645
BRAWH2014662
BRAWH2014876
BRAWH2014954// Mus musculus neuronal IL-16 mRNA, complete cds.// 4.40E-271// 604aa// 84%// AF175292
BRAWH2016221// T-CELL RECEPTOR BETA CHAIN ANA 11.// 2.90E-07// 80aa// 46%// P06333
BRAWH2016439
BRAWH2016702
BRAWH2016724// MAP2=HMW-MAP2 {alternatively spliced} [rats, brain, mRNA Partial, 267 nt].// 3.8E-24// 74aa// 77%// S81002
BRAWH3000078// Mus musculus adult male tongue cDNA, RIKEN full-length enriched library, clone:2310010M24, full insert sequence.// 1.20E-29// 111aa// 52%// AK009282
BRAWH3000100// dedicator of cyto-kinesis 1 [Homo sapiens]// 1.00E-10// 67aa// 20%// NM_001380
BRAWH3000314
BRAWH3000345
BRAWH3000491// 40S ribosomal protein S12.// 5.00E-42// 90aa// 84%// P46405
BRAWH3001326// 5-azacytidine resistance protein azr1.// 4.00E-27// 74aa// 31%// Q09189
BRAWH3001475
BRAWH3001891
BRAWH3002574// Calpain 2, large [catalytic] subunit precursor (EC 3.4.22.17) (Calcium-activated neutral proteinase) (CANP) (M-type) (M-calpain) (Millimolar-calpain).// 1.00E-84// 156aa// 91%// P17655
BRAWH3002600// cadherin 23: waltzer; otocadherin [Mus musculus]// 3.00E-30// 139aa// 28%// NM_023370
BRAWH3002819
BRAWH3002821// synaptotagmin-like 2 [Mus musculus]// 0// 358aa// 78%// NM_031394
BRAWH3003522
BRAWH3003555
BRAWH3003727
BRAWH3003801
BRAWH3003992
BRAWH3004453
BRAWH3004666
BRAWH3005132
BRAWH3005422
BRAWH3005912// MIC1 protein.// 1.00E-29// 71aa// 28%// P53258
BRAWH3005981
BRAWH3006548
BRAWH3006792
BRAWH3007221
BRAWH3007506
BRAWH3007592
BRAWH3007726
BRAWH3007783
BRAWH3008341// Ubiquitin carboxyl-terminal hydrolase 6 (EC 3.1.2.15) (Ubiquitin thiolesterase 6) (Ubiquitin-specific processing protease 6) (Deubiquitinating enzyme 6) (Proto-oncogene TRE-2).// 3.00E-63// 123aa// 67%// P35125
BRAWH3008634
BRAWH3008697
BRAWH3008931
BRAWH3009297
BRCAN2002562// Splicing factor, arginine/serine-rich 2 (Splicing factor SC35) (SC-35) (Splicing component, 35 kDa) (PR264 protein).// 6.00E-47// 88aa// 88%// Q62093
BRCAN2002856// AMYLOID BETA A4 PRECURSOR PROTEIN-BINDING FAMILY A MEMBER 2 (NEURON- SPECIFIC X11L PROTEIN) (NEURONAL MUNC18-1-INTERACTING PROTEIN 2) (MINT-2) (ADAPTER PROTEIN X11BETA).// 2.20E-89// 191aa// 93%// Q99767
BRCAN2002944// Mus musculus huntington yeast partner C (Hypc) mRNA, complete cds.// 2.1E-36// 83aa// 87%// AF135440
BRCAN2002948// Homo sapiens mRNA for gamma2-adaptin, complete cds.// 6.6E-53// 117aa// 97%// AB015318
BRCAN2003703// Homo sapiens mRNA for putative serine/threonine protein kinase, partial.// 1.4E-81// 187aa// 89%// AJ006701
BRCAN2003746// PHOSPHORYLASE B KINASE ALPHA REGULATORY CHAIN, SKELETAL MUSCLE ISOFORM (PHOSPHORYLASE KINASE ALPHA M SUBUNIT).// 5.9E-105// 213aa// 94%// P46020
BRCAN2003987
BRCAN2004355
BRCAN2005436
BRCAN2006063// COCHLIN PRECURSOR (COCH-5B2).// 5.7E-123// 230aa// 100%// 043405
BRCAN2006290
BRCAN2006297
BRCAN2006450
BRCAN2007144
BRCAN2007409
BRCAN2007426
BRCAN2008528
BRCAN2009203// Homo sapiens liprin-alpha2 mRNA, complete cds.// 1.2E-235// 273aa// 93%// AF034799
BRCAN2009432// hypothetical protein similar to small G proteins, especially RAP-2A [Homo sapiens]// 1.00E-93// 170aa// 92%// NM_021183
BRCAN2010376
BRCAN2011254
BRCAN2011602
BRCAN2012355
BRCAN2012481
BRCAN2013655
BRCAN2013660// Arabidopsis thaliana putative protein (F4F15.330) mRNA, complete cds.// 1.1E-24// 138aa// 44%// AF370547
BRCAN2014143
BRCAN2014602// DIACYLGLYCEROL KINASE, ZETA (EC 2.7.1.107) (DIGLYCERIDE KINASE) (DGK- ZETA) (DAG KINASE ZETA).// 6.5E-38// 74aa// 94%// Q13574
BRCAN2014881
BRCAN2015371
BRCAN2015464
BRCAN2016433
BRCAN2016619// CRK-associated substrate (p130Cas) (Breast cancer anti-estrogen resistance 1 protein).// 0// 735aa// 84%// P56945
BRCAN2017442// sarcosine dehydrogenase; dimethylglycine dehydrogenase-like 1 [Homo sapiens]// 5.00E-20// 51aa// 59%// NM_007101
BRCAN2017717
BRCAN2017905
BRCAN2018935
BRCAN2019387
BRCAN2020710
BRCAN2021028
BRCAN2024451// REGULATOR OF G-PROTEIN SIGNALING 14 (RGS14) (FRAGMENT).// 1.5E-131// 324aa// 83%// 043566
BRCAN2024563
BRCAN2025712
BRCAN2028355// Ca2+/calmodulin-dependent protein kinase (EC 2.7.1.123) II gamma-E// 3E-254// 479aa// 98%// JC5636
BRCOC2000670
BRCOC2001505// MYELIN BASIC PROTEIN (MBP).// 8.50E-26// 64aa// 90%// P02686 BRCOC2003213// Histidyl-tRNA synthetase (EC 6.1.1.21) (Histidine-tRNA ligase) (HisRS).// 0// 421aa// 95%// P12081
BRCOC2007034
BRCOC2014033
BRCOC2016525
BRCOC2019934
BRCOC2020142
BRHIP2000691
BRHIP2000819// Human mRNA for actin binding protein p57, complete cds.// 1.3E-48// 96aa// 100%// D44497
BRHIP2000826
BRHIP2000920
BRHIP2001074
BRHIP2001805// Homo sapiens TREK-1 potassium channel (KCNK2) mRNA, complete cds.// 1.8E-49// 134aa// 80%// AF129399
BRHIP2001927// Mus musculus mRNA for HS1 binding protein 3.// 2.7E-30// 102aa// 68%// AJ132192
BRHIP2002122// Homo sapiens B aggressive lymphoma long isoform (BAL) mRNA, complete cds.// 1.8E-97// 189aa// 100%// AF307338
BRHIP2002172// Mus musculus urea transporter isoform UTA-3 mRNA, complete cds.// 6.9E-208// 452aa// 82%// AF258602
BRHIP2002346
BRHIP2003242
BRHIP2003786// CCA3 [Rattus norvegicus]// 2.60E-199// 603aa// 61%// BAA19969
BRHIP2003917
BRHIP2004312
BRHIP2004359// ELAC PROTEIN.// 6.80E-20// 111aa// 37%// Q47012
BRHIP2004814// Homo sapiens gibbon ape leukemia virus receptor 1 (SLC20A1) gene, exon 11 and complete cds.// 1.8E-188// 346aa// 99%// AF102063
BRHIP2004883
BRHIP2005236// latrophilin 2 splice variant baaae // 1.3E-203// 250aa// 97%// AAD05305
BRHIP2005354
BRHIP2005600
BRHIP2005719
BRHIP2005752// NG5 [Homo sapiens]// 5.0E-61// 200aa// 100%// AAB47496
BRHIP2005932
BRHIP2006800
BRHIP2007616// plexin 2// 7.5E-137// 423aa// 59%// BAA13189
BRHIP2007741
BRHIP2009340
BRHIP2009414// Bax inhibitor-1 (BI-1) (Testis enhanced gene transcript).// 3.00E-97// 177aa// 77%// P55061
BRHIP2009474
BRHIP2013699
BRHIP2014228
BRHIP2021615// Homo sapiens CUG-BP and ETR-3 like factor 4 (CELF4) mRNA, complete cds.// 9.60E-115// 349aa// 65%// AF329265
BRHIP2022221
BRHIP2024146
BRHIP2024165// Synthase [Homo sapiens]// 5.00E-44// 83aa// 94%// NM_003896
BRHIP2026061
BRHIP2026288// Protein bem46.// 2.00E-47// 110aa// 41%// P54069
BRHIP2029176
BRHIP2029393// COBW-like protein [Homo sapiens]// 3.00E-89// 158aa// 98%// NM_018491
BRHIP3000339// MYELIN BASIC PROTEIN (MBP).// 8.5E-26// 64aa// 90%// P02686
BRHIP3000526
BRHIP3001283
BRHIP3006683
BRHIP3007483
BRHIP3007586
BRHIP3008183
BRHIP3008313// testis specific ankyrin-like protein 1 [Homo sapiens]// 1.00E-120// 210aa// 92%// NM_016552
BRHIP3008344
BRHIP3008405// Dynamin 2 (EC 3.6.1.50) (Dynamin UDNM).// 1.00E-56// 108aa// 90%// P39054
BRHIP3008565
BRHIP3008598
BRHIP3008997
BRHIP3009099
BRHIP3009448// 2-19 protein precursor.// 1.00E-102// 179aa// 99%// P98173
BRHIP3011241
BRHIP3013765
BRHIP3013897
BRHIP3015751
BRHIP3016213
BRHIP3018797
BRHIP3020182
BRHIP3024118// Monocarboxylate transporter 4 (MCT 4) (MCT 3).// 1.00E-36// 108aa// 30%// 035910
BRHIP3024533
BRHIP3024725
BRHIP3025161// Putative Rho/Rac guanine nucleotide exchange factor (Rho/Rac GEF) (Faciogenital dysplasia protein homolog).// 2.00E-75// 175aa// 30%// P52734
BRHIP3025702
BRHIP3026097
BRHIP3027137// 10-formyltetrahydrofolate dehydrogenase (EC 1.5.1.6) (10-FTHFDH).// 1.00E-119// 208aa// 93%// 075891
BRHIP3027854// Homo sapiens ectonucleotide pyrophosphatase/phosphodiesterase 2 (autotaxin) (ENPP2)// 1.00E-130// 222aa// 94%// NM_006209
BRSSN2000684// CDC14 homolog B, isoform 3 [Homo sapiens]// 3.00E-12// 52aa// 30%// NM_033332
BRSSN2003086
BRSSN2004496// TASP for testis-specific adriamycin sensitivity protein [Homo sapiens]// 5.00E-45// 101aa// 39%// NM_018697
BRSSN2004719// SHC transforming protein.// 4.00E-39// 89aa// 53%// P29353
BRSSN2006892
BRSSN2008549// oxysterol binding protein 2 [Mus musculus]// 1.00E-149// 252aa// 75%// NM_024289
BRSSN2008797
BRSSN2011262
BRSSN2011738
BRSSN2013874// TEMO [Rattus norvegicus]// 2.00E-53// 99aa// 98%// NM_023986
BRSSN2014299// TPA inducible gene-1; TPA inducible protein [Homo sapiens]// 2.00E-47// 93aa// 86%// NM_015889
BRSSN2014424// transporter-like protein [Homo sapiens]// 0// 413aa// 92%//
NM_022109
BRSSN2014556
BRSSN2018581
BRSSN2018925
BRSTN2000872// Protein disulfide isomerase A2 precursor (EC 5.3.4.1) (PDlp).// 0// 341aa// 92%// Q13087
BRSTN2001067
BRSTN2001613// HETEROGENEOUS NUCLEAR RIBONUCLEOPROTEINS C1/C2 (HNRNP C1 AND HNRNP C2).// 2.8E-34// 214aa// 43%// P07910
BRSTN2002400
BRSTN2003835
BRSTN2004863// Drosophila melanogaster polypeptide N-acetylgalactosaminyltransferase mRNA, complete cds.// 5.60E-126// 526aa// 47%// AF158747
BRSTN2004987// Homo sapiens mRNA for mitochondrial tryptophanyl-tRNA synthetase (WARS2 gene).// 1.20E-162// 360aa// 86%// AJ242739
BRSTN2005721
BRSTN2006865
BRSTN2007000
BRSTN2007284
BRSTN2008052
BRSTN2008283
BRSTN2008418// Breakpoint cluster region protein (EC 2.7.1.-).// 7.00E-33// 70aa// 75%// P11274
BRSTN2008457
BRSTN2009899
BRSTN2010363
BRSTN2010500
BRSTN2010750
BRSTN2012320
BRSTN2012380
BRSTN2013741// Ras-related protein M-Ras (Ras-related protein R-Ras3).// 1.00E-105// 189aa// 90%// 014807
BRSTN2015015
BRSTN2016470
BRSTN2016678
BRSTN2017084
BRSTN2017110
BRSTN2017237
BRSTN2017771// Homo sapiens putative BTK-binding protein mRNA, complete cds.// 1.0E-41// 90aa// 99%// AF235049
BRSTN2018083
BRSTN2019129
BRTHA1000311
BRTHA2000855
BRTHA2001462
BRTHA2002115
BRTHA2002281// Rho guanine nucleotide exchange factor 10 [Homo sapiens].// 5.0E-26// 123aa// 39%// NP_055444.1
BRTHA2002376
BRTHA2002442
BRTHA2002493
BRTHA2002608// aldehyde dehydrogenase 1A3// 2.00E-19// 46aa// 88%// NP_000684
BRTHA2002808// GAMMA-INTERFERON-INDUCIBLE PROTEIN IP-30 PRECURSOR.// 7.8E-65// 141aa// 90%// P13284
BRTHA2003030
BRTHA2003110// Protein Clorf8 precursor (Liver membrane-bound protein) (HSPC001).// 1.00E-98// 178aa// 92%// Q9BXS4
BRTHA2003116
BRTHA2003461
BRTHA2004821
BRTHA2004978
BRTHA2005579// Xenopus laevis mRNA for Kielin, complete cds.// 1.3E-190// 659aa// 47%// AB026192
BRTHA2005956
BRTHA2006075
BRTHA2006146
BRTHA2006194
BRTHA2007122// ANKYRIN 2 (BRAIN ANKYRIN) (ANKYRIN B) (ANKYRIN, NONERYTHROID).// 6.1E-18// 203aa// 32%// Q01484
BRTHA2007422
BRTHA2007603// H.sapiens mRNA for BCL7B protein.// 1.8E-56// 116aa// 98%// X89985
BRTHA2008316
BRTHA2008335
BRTHA2008527// LUTROPIN-CHORIOGONADOTROPIC HORMONE RECEPTOR PRECURSOR (LH/CG-R) (LSH-R) (LUTEINIZING HOROMINE RECEPTOR).// 7.5E-66// 189aa// 73%// P22888
BRTHA2008535
BRTHA2008955
BRTHA2009311// EOSINOPHIL LYSOPHOSPHOLIPASE// 1.0E-30// 64aa// 91%// P97400
BRTHA2009846
BRTHA2009972
BRTHA2010073
BRTHA2010608
BRTHA2010884
BRTHA2010907
BRTHA2011194
BRTHA2011351
BRTHA2011500
BRTHA2011641
BRTHA2012392// Homo sapiens HCDI (HCDI) mRNA, complete cds.// 8.0E-95// 194aa// 95%// AF226050
BRTHA2012562
BRTHA2012980// INTERLEUKIN-13 RECEPTOR ALPHA-1 CHAIN PRECURSOR (IL-13R-ALPHA-1) (IL- 13RA-1).// 1.5E-44// 91aa// 100%// P78552
BRTHA2013262
BRTHA2013460
BRTHA2013707
BRTHA2014792// ENHANCER OF ZESTE HOMOLOG 1 (ENX-2) (KIAA0388).// 7.1E-21// 184aa// 35%// Q92800
BRTHA2014828
BRTHA2015406// Homo sapiens mRNA for putative serine/threonine protein kinase, partial.// 1.90E-86// 268aa// 67%// AJ006701
BRTHA2015478
BRTHA2015696
BRTHA2015878
BRTHA2016215
BRTHA2016496// Vacuolar processing enzyme precursor (EC 3.4.22.-) (VPE).// 0// 370aa// 79%// P49043
BRTHA2016543
BRTHA2017353
BRTHA2017985
BRTHA2018165
BRTHA2018344
BRTHA2018591
BRTHA2018624// Oncorhynchus mykiss stl3 mRNA for rhamnose binding lectin STL3, complete cds.// 7.40E-21// 167aa// 34%// AB039024
BRTHA2018707
BRTHA2019014
BRTHA2019022
BRTHA2019048
BRTHA3000273
BRTHA3000297
BRTHA3000633// single-pass transmembrane protein [Rattus norvegicus]// 5.00E-48// 220aa// 54%// BAA90767
BRTHA3001721// TATA box binding protein (TBP)-associated factor, RNA polymerase III, GTF3B subunit 2; TATA box binding protein (TBP)-associated factor, RNA polymerase III, C, 90kD; general transcription factor IIIB, 90kD [Homo sapiens]// 4.00E-71// 135aa// 85%// NM_001519
BRTHA3002401
BRTHA3002427// Sodium- and chloride-dependent betaine transporter (Na+/Cl- betaine/GABA transporter) (BGT-1).// 0// 553aa// 96%// P48065
BRTHA3002933// uroplakin 3 [Homo sapiens]// 1.00E-158// 260aa// 99%// XP_001216
BRTHA3003074// putative prostate cancer susceptibility protein; hypothetical protein FLJ10530 [Homo sapiens]// 0// 435aa// 94%// NM_018127
BRTHA3003343// DAZ associated protein 1 [Homo sapiens]// 1.00E-95// 223aa// 92%// NP_061832
BRTHA3003449// MYOSIN HEAVY CHAIN, SMOOTH MUSCLE ISOFORM (SMMHC) (FRAGMENT).// 4.70E-215// 400aa// 100%// P35749
BRTHA3003474
BRTHA3003490
BRTHA3004475
BRTHA3005046
BRTHA3006856
BRTHA3007113
BRTHA3007148
BRTHA3007319
BRTHA3007769
BRTHA3008143
BRTHA3008310// Mus musculus mRNA for iroquois homeobox protein 6 (Irx6 gene).// 1.20E-176// 444aa// 76%// AJ271055
BRTHA3008386
BRTHA3008520// sporulation-induced transcript 4-associated protein: hypothetical protein FLJ11058 [Homo sapiens]// 1.00E-164// 287aa// 87%// NM_018312
BRTHA3008778// Acetyl-coenzyme A synthetase (EC 6.2.1.1) (Acetate―CoA ligase) (Acyl- activating enzyme).// 1.00E-168// 286aa// 51%// 068040
BRTHA3009037// Regulator of G-protein signaling 3 (RGS3) (RGP3).// 0// 479aa// 92%// P49796
BRTHA3009090// neuropathy target esterase [Homo sapiens]// 0// 784aa// 60%// NM_006702
BRTHA3009291
BRTHA3010366
BRTHA3013884// Sorting nexin 14 (Fragment).// 0// 359aa// 95%// Q9Y5W7
BRTHA3015815// Selenide, water dikinase 1 (EC 2.7.9.3) (Selenophosphate synthetase 1) (Selenium donor protein 1).// 1.00E-159// 275aa// 99%// P49903
BRTHA3015910
BRTHA3016845
BRTHA3016917// Valyl-tRNA synthetase 2 (EC 6.1.1.9) (Valine-tRNA ligase 2) (VALRS 2).// 4.00E-82// 169aa// 43%// P26640
BRTHA3017047
BRTHA3017589// junctional adhesion molecule 3 [Homo sapiens]// 1.00E-119// 213aa// 74%// NM_031470
BRTHA3017848// Organic cation/carnitine transporter 2 (Solute carrier family 22, member 5) (High-affinity sodium-dependent carnitine cotransporter).// 2.00E-42// 105aa// 35%// 076082
BRTHA3018514
BRTHA3018617
BRTHA3018656
BRTHA3019105
CERVX1000042
CERVX2002006
COLON1000030
COLON2000470// Rattus norvegicus nucleolar protein C7C mRNA, complete cds.// 5.9E-51// 187aa// 49%// AF333986
COLON2000568// Ig alpha-2 chain C region.// 0// 324aa// 95%// P01877
COLON2001721// GLUT4 vesicle protein [Mus musculus]// 8.00E-36// 160aa// 39%// AAD10190
COLON2002443
COLON2002520// Myosin heavy chain, nonmuscle type B (Cellular myosin heavy chain, type B) (Nonmuscle myosin heavy chain-B) (NNONHC-B).// 0// 447aa// 70%// Q27991
COLON2003043
COLON2004478// protein Tro alpha1 H, myeloma // 3.2E-233// 475aa// 88%// 0501254A
COLON2005126
COLON2005772// Homo sapiens candidate taste receptor T2R14 gene, complete cds.// 3.9E-54// 112aa// 97%// AF227138
COLON2006282
COLON2009499
CORDB1000140
CORDB2000061
CORDB2000541// F-actin capping protein beta subunit (CAPZ beta).// 1.00E-126// 217aa// 99%// P79136
CTONG1000087
CTONG1000088
CTONG1000288
CTONG1000302
CTONG1000341// THROMBOMODULIN PRECURSOR (FETOMODULIN) (TM) (CD141 ANTIGEN).// 1.0E-283// 488aa// 99%// P07204
CTONG1000467// Mus musculus mRNA for Deltex3, complete cds.// 5.00E-54// 203aa// 52%// AB015425
CTONG1000488
CTONG1000508
CTONG1000540
CTONG2000042// ALPHA-2-MACROGLOBULIN PRECURSOR (ALPHA-2-M).// 2E-132// 841aa// 35%// P01023
CTONG2001877
CTONG2004062// ATPase subunit 6 [Homo sapiens].// 3.00E-71// 226aa// 91%// BAA07295
CTONG2006798// putative serine/threonine protein kinase [Schizosaccharomyces pombe]// 5.80E-69// 581aa// 27%// CAB66438
CTONG2008233// Bos taurus DnaJ1 protein mRNA, complete cds.// 0// 1376bp// 85%// AF308815
CTONG2009423// 5-HYDROXYTRYPTAMINE 7 RECEPTOR (5-HT-7) (5-HT-X) (SEROTONIN RECEPTOR) (5HT7).// 2.40E-44// 113aa// 78%// P34969
CTONG2009531
CTONG2010803// Regulator of G-protein signaling 3 (RGS3) (RGP3).//.0// 323aa// 92%// P49796
CTONG2013178// Homo sapiens serine protease DESC1 (DESC1) mRNA, complete cds.// 2E-90// 421aa// 43%// AF064819
CTONG2017500// Homo sapiens muscle disease-related protein mRNA, complete cds.// 1.30E-59// 239aa// 47%// AF204674
CTONG2019248
CTONG2019652
CTONG2019704
CTONG2019788
CTONG2019833
CTONG2020026// Drosophila melanogaster BcDNA.GH09358 (BcDNA.GH09358) mRNA, complete cds.// 4.2E-187// 669aa// 45%// AF181639
CTONG2020127
CTONG2020522
CTONG2020638
CTONG2020806
CTONG2021132
CTONG2022153
CTONG2022601
CTONG2023021// H.sapiens mRNA for TFG protein.// 2.3E-88// 160aa// 100%// Y07968
CTONG2023512// Homo sapiens PIG-T mRNA for phosphatidyl inositol glycan class T, complete cds.// 7.1E-158// 289aa// 100%// AB057724
CTONG2024206
CTONG2024749// ALPHA-2-MACROGLOBULIN PRECURSOR (ALPHA-2-M).// 1.1E-174// 699aa// 46%// P06238
CTONG2025496// ALPHA-2-MACROGLOBULIN PRECURSOR (ALPHA-2-M).// 1.2E-218// 977aa// 45%// P01023
CTONG2025516// general transcription factor II, i, isoform 3; BTK-associated protein, 135kD; Williams-Beuren syndrome chromosome region 6; Bruton tyrosine kinase-associated protein 135; TFII-I protein; SPIN protein [Homo sapiens]// 2.00E-28// 57aa// 89%// NM_033001
CTONG2025900
CTONG2026920
CTONG2027327
CTONG2028124// very long-chain acyl-CoA synthetase homolog 1; VLCS-H1 protein [Homo sapiens]// 5.00E-86// 156aa// 48%// NM_014031
CTONG2028687
CTONG3000084// PROBABLE GUANINE NUCLEOTIDE REGULATORY PROTEIN TIM (ONCOGENE TIM) (P60 TIM) (TRANSFORMING IMMORTALIZED MAMMARY ONCOGENE).// 4.3E-276// 519aa// 100%// Q12774
CTONG3000657
CTONG3000686
CTONG3000707
CTONG3000896
CTONG3001123// Mus musculus Pax transcription activation domain interacting protein PTIP mRNA, complete cds.// 0// 965aa// 84%// AF104261
CTONG3001370// ALPHA-2-MACROGLOBULIN PRECURSOR (ALPHA2M).// 1.5E-267// 1008aa// 38%// Q61838
CTONG3001420
CTONG3001560
CTONG3002020
CTONG3002127// granuphilin [Mus musculus]// 1.00E-104// 204aa// 49%// NM_013757
CTONG3002412// Human DOCK180 protein mRNA, complete cds.// 4.5E-236// 678aa// 66%// D50857
CTONG3002674
CTONG3003179
CTONG3003483
CTONG3003652
CTONG3003654
CTONG3003737// PLATELET GLYCOPROTEIN V PRECURSOR (GPV) (CD42D).// 1.80E-73// 434aa// 37%// 008770
CTONG3003905
CTONG3003972
CTONG3004072// GL002 protein [Homo sapiens]// 3.00E-80// 152aa// 88%// NM_020193
CTONG3004712
CTONG3005325
CTONG3005648
CTONG3005713
CTONG3005813
CTONG3006067
CTONG3006186// syntaxin binding protein 4 [Mus musculus]// 0// 427aa// 76%// NM_011505
CTONG3006650
CTONG3007444
CTONG3007528
CTONG3007586
CTONG3007870
CTONG3008252
CTONG3008258// Homo sapiens GROS1-L protein mRNA, complete cds.// 7.70E-177// 680aa// 51%// AF097432
CTONG3008496
CTONG3008566
CTONG3008639// Human non-lens beta gamma-crystallin like protein (AIM1) mRNA, partial cds.// 0// 836aa// 99%// U83115
CTONG3008831// Rattus norvegicus PGC1 mRNA for PPAR gamma coactivator, complete cds.// 2.9E-69// 176aa// 46%// AB025784
CTONG3008894// Mus musculus SH3-domain binding protein 5// 3.00E-42// 89aa// 40%// NM_011894
CTONG3008951
CTONG3009028// sno gene product [Drosophila melanogaster]// 1.00E-148// 1000aa// 46%// AAF48240
CTONG3009227
CTONG3009239
CTONG3009328
CTONG3009385// Homo sapiens ARG99 mRNA, complete cds.// 2.4E-77// 153aa// 100%// AF319520
D3OST2002182// Homo sapiens mRNA for acetylglucosaminyltransferase-like protein.// 6.50E-11// 265aa// 23%// AJ007583
D3OST2002648// PUTATIVE G PROTEIN-COUPLED RECEPTOR GPR17 (R12).// 3E-24// 184aa// 28%// Q13304
D3OST3000169// Homo sapiens SH3-SAM adaptor protein (HACS1) mRNA, complete cds.// 2.40E-189// 354aa// 99%// AF218085
DFNES1000107
DFNES2000146// Mus musculus mRNA for thrombospondin type 1 domain, complete cds.// 4.10E-31// 135aa// 41%// AB016768
DFNES2001108// Homo sapiens nuclear dual-specificity phosphatase (SBF1) mRNA, partial cds.// 4.5E-41// 134aa// 63%// U93181
DFNES2005266// ADAM-TS 1 PRECURSOR (EC 3.4.24.-) (A DISINTEGRIN AND METALLOPROTEINASE WITH THROMBOSPONDIN MOTIFS 1) (ADAMTS-1) (ADAM-TS1).// 4.80E-15// 118aa// 30%// P97857
DFNES2010502
DFNES2011239
DFNES2011499
ERLTF2000324
FCBBF1000297// Human protein immuno-reactive with anti-PTH polyclonal antibodies mRNA, partial cds.// 7.5E-186// 359aa// 99%// U28831
FCBBF2001183
FCBBF2007510
FCBBF3001977
FCBBF3002163// chromosome condensation-related SMC-associated protein 1; chromosome condensation-related SMC-associated protein 1; KIAA0159 gene product [Homo sapiens]// 0// 840aa// 97%// NM_014865
FCBBF3003435
FCBBF3004502
FCBBF3004847
FCBBF3006171
FCBBF3007242
FCBBF3007540// GUANINE NUCLEOTIDE EXCHANGE FACTOR DBS (DBL'S BIG SISTER)// 5.00E-46// 300aa// 38%// 015068
FCBBF3008944
FCBBF3009888// Homo sapiens prostate stem cell antigen (PSCA) mRNA, complete cds.// 5.30E-06// 122aa// 32%// AF043498
FCBBF3012170// Mus musculus rostral cerebellar malformation protein (rcm) mRNA, complete cds.// 1.00E-80// 325aa// 51%// U72634
FCBBF3012288
FCBBF3013307// Homo sapiens RNA helicase-related protein mRNA, complete cds.// 0// 644aa// 99%// AF083255
FCBBF3013846
FCBBF3021576
FCBBF3021940// SYNAPSIN I (FRAGMENT).// 5.00E-06// 128aa// 35%// 062732
FCBBF3023443
FCBBF3023895// contains simiarity to tubulin-tyrosine ligase [Caenorhabditis elegans].// 1.00E-54// 220aa// 39%// AAF39893
FCBBF3025730
FCBBF3027717
FCBBF4000076
FEBRA1000030// T-CELL RECEPTOR BETA CHAIN ANA 11.// 2.7E-11// 131aa// 38%// P06333
FEBRA2000253
FEBRA2006396
FEBRA2007544// transcription factor [Homo sapiens]// 0// 400aa// 99%// AAG33674
FEBRA2007708// DRA PROTEIN (DOWN-REGULATED IN ADENOMA).// 2.60E-72// 511aa// 34%// P40879
FEBRA2007793
FEBRA2007801// Homo sapiens TRIAD3 mRNA, partial cds.// 2.3E-207// 358aa// 99%// AF228527
FEBRA2008287
FEBRA2008311// GALANIN RECEPTOR TYPE 1 (GAL1-R) (GALR1).// 1E-23// 299aa// 27%// P56479
FEBRA2008360
FEBRA2008468// LYSOSOMAL ACID LIPASE/CHOLESTERYL ESTER HYDROLASE PRECURSOR (EC 3.1.1.13) (LAL) (ACID CHOLESTERYL ESTER HYDROLASE) (STEROL ESTERASE) (LIPASE A) (CHOLESTERYL ESTERASE).// 1.20E-179// 330aa// 97%// P38571
FEBRA2010719 .
FEBRA2014213
FEBRA2015588
FEBRA2020484
FEBRA2020582
FEBRA2020668
FEBRA2020886
FEBRA2021339
FEBRA2021571
FEBRA2021908
FEBRA2021966
FEBRA2024136
FEBRA2024150
FEBRA2024343
FEBRA2024744// Homo sapiens Cat Eye Syndrome critical region protein isoform 1 mRNA, complete cds.// 1.3E-126// 252aa// 94%// AF273270
FEBRA2025427
FEBRA2026984// TYROSYL-TRNA SYNTHETASE (EC 6.1.1.1) (TYROSYL―TRNA LIGASE) (TYRRS) (FRAGMENT).// 7.80E-271// 528aa// 94%// Q29465
FEBRA2027082
FEBRA2027297
FEBRA2027352
FEBRA2028366
FEBRA2028477
FEBRA2028618
HCASM2001301// MITOGEN-ACTIVATED PROTEIN KINASE 12 (EC 2.7.1.-) (EXTRACELLULAR SIGNAL-REGULATED KINASE 6) (EC 2.7.1.-) (ERK6) (ERK5) (STRESS-ACTIVATED PROTEIN KINASE-3) (MITOGEN-ACTIVATED PROTEIN KINASE P38 GAMMA) (MAP KINASE P38 GAMMA).// 2.2E-52// 104aa// 100%// P53778
HCASM2002502
HCASM2002918
HCASM2003212
HCASM2003415
HCASM2007047
HCASM2007737// SEC14-LIKE PROTEIN 1.// 8.30E-09// 162aa// 24%// Q92503
HCHON2000028// Homo sapiens 7h3 protein mRNA, partial cds.// 2.1E-94// 228aa// 82%// AF209931
HCHON2000212
HCHON2000244
HCHON2000418
HCHON2000626// X-linked protein STS1769.// 2.00E-47// 89aa// 83%// Q99871
HCHON2001084// ARABINOSE-PROTON SYMPORTER (ARABINOSE TRANSPORTER).// 3E-66// 321aa// 36%// P09830
HCHON2001217// Homo sapiens cullin CUL4B (CUL4B) mRNA, complete cds.// 0// 782aa// 99%// AF212995
HCHON2001548
HCHON2001577// Human elastin gene, exon 1.// 1.5E-265// 585aa// 88%// M17282
HCHON2001712// Neutral amino acid transporter B(0) (ATB(0)).// 0// 369aa// 85%// Q15758
HCHON2002676// ALPHA-L-IDURONIDASE PRECURSOR (EC 3.2.1.76).// 3.20E-274// 330aa// 99%// P35475
HCHON2003532// PHOSPHORYLASE B KINASE BETA REGULATORY CHAIN (PHOSPHORYLASE KINASE BETA SUBUNIT).// 2.0E-159// 312aa// 95%// Q93100
HCHON2004007// Potential phospholipid-transporting ATPase IK (EC 3.6.3.13) (Fragment).// 1.00E-160// 273aa// 93%// 060423
HCHON2004531// UV excision repair protein RAD23 homolog B (HHR23B) (XP-C repair complementing complex 58 kDa protein) (P58).// 1.00E-142// 270aa// 66%// P54727
HCHON2004776// transmembrane protein (63kD), endoplasmic reticulum/Golgi intermediate compartment [Homo sapiens]// 0// 500aa// 86%// NP_006816
HCHON2005921// lipoma HMGIC fusion partner [Homo sapiens]// 1.00E-15// 52aa// 25%// NM_005780
HCHON2006250// Mus musculus SETA binding protein 1 (Sb1) mRNA, complete cds.// 3.9E-269// 544aa// 91%// AF246218
HCHON2006714
HCHON2007881
HCHON2008112// Homo sapiens HERC2 (HERC2) mRNA, complete cds.// 1.80E-24// 79aa// 70%// AF071172
HCHON2008444// 28S ribosomal protein S15, mitochondrial precursor (MPR-S15) (DC37).// 5.00E-39// 76aa// 76%// P82914
HEART1000010// Hepatocyte growth factor-like protein precursor (Macrophage stimulatory protein) (MSP) (Macrophage stimulating protein).// 5.00E-18// 40aa// 93%// P26927
HEART1000074// BANP homolog; putative transcription factor; Btg3 associated nulcear protein [Mus musculus]// 0// 420aa// 82%// NM_016812
HEART1000088
HEART1000139// TROPONIN T, CARDIAC MUSCLE ISOFORMS (TNTC).// 1.40E-112// 221aa// 98%// P45379
HEART2001680// Ig alpha-1 chain C region.// 0// 324aa// 91%// P01876
HEART2001756
HEART2006131// 2-hydroxyphytanoyl-CoA lyase [Mus musculus]// 1.00E-138// 263aa// 45%// NM_019975
HEART2006909// Hemolysin C.// 3.00E-40// 88aa// 33%// 054318
HEART2007031
HEART2010391
HEART2010492// GLYCEROL-3-PHOSPHATE ACYLTRANSFERASE, MITOCHONDRIAL PRECURSOR (EC 2. 3. 1. 15) (GPAT) (P90).// 3.6E-47// 462aa// 32%// Q61586
HEART2010495// MICROTUBULE-ASSOCIATED PROTEIN 4.// 2.00E-159// 579aa// 62%// P27816
HHDPC1000118// Threonine synthase (EC 4.2.99.2).// 3.00E-70// 178aa// 35%// Q9ZMX5
HHDPC2001337
HLUNG1000017
HLUNG2000014// Mus musculus strain BALB/c dectin-2 alpha isoform mRNA, complete cds.// 2.80E-55// 211aa// 50%// AF240357
HLUNG2001996
HLUNG2002465// Homo sapiens Asef mRNA for APC-stimulated guanine nucleotide exchange factor, complete cds.// 1.30E-183// 557aa// 62%// AB042199
HLUNG2002958
HLUNG2003003
HLUNG2003872
HLUNG2010464
HLUNG2011041// basic proline-rich peptide IB-8a - human (fragments)// 9.7E-07// 113aa// 35%// D38355
HLUNG2011298// Homo sapiens cytochrome b5 reductase 1 (B5R.1) mRNA, complete cds.// 1.6E-27// 79aa// 78%// AF169481
HLUNG2012049
HLUNG2012287
HLUNG2012727
HLUNG2013204// phytoene dehydrogenase-like [Arabidopsis thaliana]// 4.0E-53// 97aa// 55%// BAB10768
HLUNG2013304
HLUNG2013622
HLUNG2013851
HLUNG2014262
HLUNG2014288// Mus musculus RP42 mRNA, complete cds.// 2.4E-40// 189aa// 43%// AF198092
HLUNG2014449
HLUNG2015617
HLUNG2017350// GAP JUNCTION ALPHA-3 PROTEIN (CONNEXIN 44) (CX44).// 2.60E-53// 262aa// 41%// P41987
HLUNG2017546
HLUNG2017806
HLUNG2019058
HSYRA2004858
HSYRA2005456
HSYRA2005496// ENDOGLIN PRECURSOR (CD105 ANTIGEN).// 2.4E-117// 245aa// 92%// P17813
HSYRA2006873
HSYRA2007667
HSYRA2008376
HSYRA2008714// POTENTIAL PHOSPHOLIPID-TRANSPORTING ATPASE ID (EC 3.6.1.-) (FRAGMENT).// 6.2E-158// 412aa// 70%// P98198
HSYRA2009075
HSYRA2009102// UDP-galactose transporter related [Homo sapiens].// 3.0E-26// 280aa// 32%// NP_005818
IMR322000127// ZINC FINGER PROTEIN 135.// 3.30E-130// 426aa// 50%// P52742
IMR322000917// ZINC FINGER PROTEIN 29 (ZFP-29).// 1.50E-34// 197aa// 40%// Q07230
IMR322001380// Homo sapiens leucine-rich repeats containing F-box protein FBL3 mRNA, complete cds.// 7.00E-21// 216aa// 32%// AF186273
IMR322002035
IMR322002110
IMR322003675
IMR322006222
IMR322006495// Homo sapiens mRNA for kinetochore protein CENP-H, complete cds.// 3.1E-61// 183aa// 73%// AB035124
IMR322006886// Homo sapiens hepatocellular carcinoma-associated antigen 127 (HCA127) mRNA, complete cds.// 2.5E-107// 207aa// 99%// AF270491
IMR322007225
IMR322016146
IMR322018117
KIDNE1000064// Mus musculus mRNA for RST, complete cds.// 6.70E-219// 552aa// 73%// AB005451
KIDNE2000665
KIDNE2000722
KIDNE2000832
KIDNE2000846// Mus musculus orphan transporter isoform A12 (Xtrp2) mRNA, alternatively spliced, complete cds.// 1.2E-54// 203aa// 50%// AF075262 KIDNE2001361// Mus musculus catp mRNA for cation-transporting atpase, complete cds.// 4.1E-123// 273aa// 91%// AB035381
KIDNE2001847// H.sapiens graf gene.// 4. 10E-98// 300aa// 55%// Y10388 KIDNE2002252// Drosophila melanogaster BcDNA. GH09358 (BcDNA.GH09358) mRNA, complete cds. // 6.30E-145// 763aa// 42%// AF181639
KIDNE2002991
KIDNE2003837
KIDNE2005543
KIDNE2006580// CYTOCHROME P450 4C1 (EC 1.14.14.1) (CYPIVC1).// 1.10E-119// 496aa// 49%// P29981
KIDNE2010264
KIDNE2011314
KIDNE2011532// similar to melanoma-associated chondroitin sulfate proteoglycan 4// 7.00E-30// 54aa// 60%// XP_000655
KIDNE2011635// Rabbit mRNA for sodium-glucose cotransporter, complete cds.// 2. 1e-313// 670aa// 80%// D16226
KIDNE2012945// PROCOLLAGEN C-PROTEINASE ENHANCER PROTEIN PRECURSOR (PCPE) (TYPE I PROCOLLAGEN COOH-TERMINAL PROTEINASE ENHANCER) (TYPE 1 PROCOLLAGEN C-PROTEINASE ENHANCER PROTEIN).// 2.00E-14// 113aa// 41%// Q15113
KIDNE2013095
LIVER2007415
LYMPB1000141
LYMPB2000083// HLA CLASS I HISTOCOMPATIBILITY ANTIGEN, ALPHA CHAIN F PRECURSOR (HLA F ANTIGEN) (LEUKOCYTE ANTIGEN F).// 4.80E-131// 158aa// 93%// P33617
MESAN2001979
MESAN2006563
MESAN2012054
MESAN2014295
MESAN2015515
MESAN2018576
MESTC1000042
MESTC2000153
NB9N41000340
NCRRP1000129
NESOP2000744
NESOP2001433// ALC1_HUMAN lg alpha-1 chain C region// 0// 353aa// 100%// P01876
NESOP2001656
NESOP2001694// H.sapiens graf gene.// 7.4E-53// 162aa// 66%// Y10388
NESOP2001752
NESOP2002738
NHNPC2000606
NHNPC2000877
NHNPC2001223
NHNPC2001816
NHNPC2002565
NHNPC2002749
NOVAR2000136// Calsequestrin, skeletal muscle isoform precursor (Aspartactin) (Laminin-binding protein).// 1.00E-142// 235aa// 66%// P07221
NOVAR2000710
NOVAR2000962
NOVAR2001108// Human (hybridoma H210) anti-hepatitis A IgG variable region, constant region, complementarity-determining regions mRNA, complete cds.// 3.0E-230// 482aa// 88%// M87789
NOVAR2001783
NT2NE2003252// Human putative serine/threonine protein kinase PRK (prk) mRNA, complete cds.// 3.00E-44// 234aa// 38%// U56998
NT2NE2005890
NT2NE2006531// ZINC FINGER PROTEIN 184 (FRAGMENT).// 4.10E-113// 437aa// 47%// Q99676
NT2NE2006909// Methionine aminopeptidase 2 (EC 3.4.11.18) (MetAP 2) (Peptidase M 2) (Initiation factor 2 associated 67 kDa glycoprotein) (P67).// 1.00E-147// 258aa// 80%// P50579
NT2NE2008060
NT2RI2003993
NT2RI2004618// Cytosolic acyl coenzyme A thioester hydrolase (EC 3.1.2.2) (Long chain acyl-CoA thioester hydrolase) (CTE-II) (Brain acyl-CoA hydrolase) (BACH).// 1.00E-126// 222aa// 88%// 000154
NT2RI2005166// VEGETATIBLE INCOMPATIBILITY PROTEIN HET-E-1.// 7.70E-14// 300aa// 26%// Q00808
NT2RI2006686// E1A-ASSOCIATED PROTEIN P300.// 1.30E-18// 421aa// 26%// Q09472
NT2RI2008724
NT2RI2009855
NT2RI2011422// Homo sapiens partial mRNA for transport-secretion protein 2.1 (TTS-2.1 gene).// 6.4E-70// 428aa// 40%// AJ278475
NT2RI2011683
NT2RI2012659
NT2RI2012990// 76.5 KDA PROTEIN C210RF13.// 1.8E-73// 149aa// 100%// 095447
NT2RI2013357
NT2RI2014247
NT2RI2014551
NT2RI2014733
NT2RI2016128
NT2RI2018311
NT2RI2018883
NT2RI2019751
NT2RI2023303
NT2RI2025909// carnitine/acylcarnitine translocase// 3.0E-32// 260aa// 37%//
NP_000378
NT2RI2025957// LU1 protein [Homo sapiens]// 0// 630aa// 99%// AAF74512
NT2RI2027081
NT2RI2027396
NT2RI3000622
NT2RI3001263
NT2RI3001515// ALEX1 protein [Homo sapiens]// 2.0E-25// 220aa// 29%//
NP_057692
NT2RI3002303
NT2RI3002842
NT2R13002892
NT2RI3003031
NT2RI3003095
NT2RI3003162
NT2RI3003382
NT2RI3003409
NT2RI3004381
NT2RI3004510
NT2RI3005202
NT2RI3005403
NT2RI3005724
NT2RI3006132
NT2RI3006171// CARCINOEMBRYONIC ANTIGEN PRECURSOR (CEA) (MECONIUM ANTIGEN 100) (CD66E ANTIGEN).// 1.3E-54// 294aa// 39%// P06731
NT2RI3006284// Homo sapiens chorea-acanthocytosis (CHAC) mRNA, complete cds.// 1.2E-144// 538aa// 51%// AF337532
NT2RI3006340// Myomesin 1 (Skelemin).// 0// 1390aa// 81%// Q62234
NT2RI3006376
NT2RI3006673// LAR protein precursor (Leukocyte antigen related) (EC 3.1.3.48).// 0// 1151aa// 90%// P10586
NT2RI3006796
NT2RI3007065
NT2RI3007158
NT2RI3007291
NT2RI3007543
NT2RI3007757// breast cancer nuclear receptor-binding auxiliary protein // 1.00E-172// 295aa// 94%// AAD21311
NT2RI3007978// CTP synthase II; CTP synthetase type 2 [Homo sapiens]// 0// 536aa// 91%// NM_019857
NT2RI3008055
NT2RI3008162
NT2RI3008652// Homo sapiens mRNA for CDEP, complete cds.// 6.10E-113// 443aa// 52%// AB008430
NT2RI3008697// erythroblast macrophage protein [Mus musculus]// 2.00E-14// 70aa// 25%// NM_021500
NT2RI3008974// probable transposase - human transposon MER37// 1.20E-52// 165aa// 69%// S72481
NT2RI3009158// Iroquois-class homeodomain protein IRX-3.// 4.00E-16// 52aa// 36%// P81067
NT2RP7000359// PROTEIN-TYROSINE PHOSPHATASE D1 (EC 3.1.3.48).// 6.80E-25// 319aa// 28%// Q16825
NT2RP7000466// Cegp1 protein// 0// 482aa// 89%// NP_064436
NT2RP7004027// BONE MORPHOGENETIC PROTEIN 1 PRECURSOR (EC 3.4.24.-) (BMP-1).// 9.50E-33// 301aa// 30%// P98063
NT2RP7004123
NT2RP7005118// RAS GTPASE-ACTIVATING-LIKE PROTEIN IQGAP1 (P195) (KIAA0051).// 0// 1034aa// 58%// P46940
NT2RP7005529// PROBABLE GUANINE NUCLEOTIDE REGULATORY PROTEIN TIM (ONCOGENE TIM) (P60 TIM) (TRANSFORMING IMMORTALIZED MAMMARY ONCOGENE).// 5.40E-56// 364aa// 37%// Q12774
NT2RP7005846
NT2RP7009030
NT2RP7009147// CHE-2 protein [Caenorhabditis elegans]// 1.00E-177// 740aa// 41%// CAB38019
NT2RP7009867
NT2RP7010128
NT2RP7010599// Homo sapiens endothelial lipase mRNA, complete cds.// 5.60E-174// 321aa// 98%// AF118767
NT2RP7011570
NT2RP7013795// VEGETATIBLE INCOMPATIBILITY PROTEIN HET-E-1.// 4.90E-11// 129aa// 34%// Q00808
NT2RP7014005// CTP synthase II; CTP synthetase type 2 [Homo sapiens]// 0// 536aa// 91%// NM_019857
NT2RP7015512
NT2RP7017365
NT2RP7017474
NT2RP7017546
NT2RP8000137
NT2RP8000296// similar to Kelch proteins// 0// 600aa// 99%// AAF03529
NT2RP8000483// Rattus norvegicus mRNA for Nadrin E2, complete cds.// 2.00E-208// 548aa// 75%// AB060557
NTONG2000413// MATRIX METALLOPROTEINASE-16 PRECURSOR (EC 3.4.24.-) (MMP-16) (MEMBRANE-TYPE MATRIX METALLOPROTEINASE 3) (MT-MMP 3) (MTMMP3) (MMP-X2).// 5.60E-62// 290aa// 37%// P51512
NTONG2003852
NTONG2005277// ANKYRIN 1 (ERYTHROCYTE ANKYRIN).// 8.20E-31// 363aa// 31%// Q02357
NTONG2005969
NTONG2006354
NTONG2007249
NTONG2007517// RING CANAL PROTEIN (KELCH PROTEIN).// 9.10E-32// 295aa// 28%// Q04652
NTONG2008088
NTONG2008672// final exon in repeat region: similar to long tandem repeat region of sialidase (SP:TCNA_TRYCR, P23253) and neurofilament H protein // 1.9E-15// 559aa// 25%// AAC48204
OCBBF1000254
OCBBF2001794
OCBBF2002124// p40 [Homo sapiens]// 3.00E-63// 103aa// 88%// AAC51270
OCBBF2003819
OCBBF2004826// T-cell lymphoma invasion and metastasis 2 [Homo sapiens]// 0// 580aa// 99%// NP_036586
OCBBF2004883
OCBBF2005428
OCBBF2006005// Bos taurus phosphatidic acid-preferring phospholipase A1 mRNA, complete cds.// 0// 885aa// 90%// AF045022
OCBBF2006058// Homo sapiens acyl-Coenzyme A dehydrogenase-8 precursor, mRNA, complete cds.// 5.40E-57// 109aa// 100%// AF126245
OCBBF2006151// Mus musculus protein tyrosine phosphatase-like protein PTPLB (Ptplb) mRNA, complete cds.// 3.40E-126// 258aa// 93%// AF169286
OCBBF2006567
OCBBF2006764// seizure related gene 6 [Mus musculus]// 0// 780aa// 89%// NP_067261
OCBBF2007028// Homo sapiens mRNA for NESCA, complete cds.// 1.50E-169// 176aa// 98%// AB026894
OCBBF2007068// ankyrin 1 [Bos taurus].// 1.00E-68// 800aa// 32%// AAF61702
OCBBF2007114
OCBBF2007428
OCBBF2007478
OCBBF2007610// PSD-95/SAP90-associated protein-4 [Rattus norvegicus].// 1.00E-137// 226aa// 90%// AAB48590
OCBBF2008770
OCBBF2009788
OCBBF2009926
OCBBF2010140
OCBBF2010416
OCBBF2017516
OCBBF2019327
OCBBF2019823// lactate dehydrogenase A -like [Homo sapiens]// 1.00E-164// 273aa// 82%// NM_033195
OCBBF2020343
OCBBF2020453
OCBBF2020639
OCBBF2020741
OCBBF2020801// Ataxin 7 (Spinocerebellar ataxia type 7 protein).// 5.00E-67// 116aa// 100%// 015265
OCBBF2020838// FORKHEAD BOX PROTEIN D4 (FORKHEAD-RELATED PROTEIN FKHL9) (FORKHEAD- RELATED TRANSCRIPTION FACTOR 5) (FREAC-5) (TRANSCRIPTION FACTOR FKH- 2).// 1.70E-114// 371aa// 63%// Q60688
OCBBF2021020// Homo sapiens mRNA for vascular Rab-GAP/TBC-containing protein, complete cds.// 1.8E-24// 107aa// 47%// AB024057
OCBBF2021286
OCBBF2021323// Mus musculus GTRGE022 (Gtrgeo22) mRNA, complete cds.// 7.80E-49// 115aa// 88%// AF303106
OCBBF2021788// Homo sapiens mRNA for B-cell CLL/lymphoma 9 (BCL9 gene).// 1.30E-92// 600aa// 42%// Y13620
OCBBF2022351// TIPD PROTEIN.// 1.1E-54// 263aa// 40%// 015736
OCBBF2022574
OCBBF2023162
OCBBF2023643
OCBBF2024719
OCBBF2024781
OCBBF2024850
OCBBF2025028
OCBBF2025458
OCBBF2025527// GLYCEROL-3-PHOSPHATE DEHYDROGENASE [NAD+], CYTOPLASMIC (EC 1.1.1.8) (GPD-C) (GPDH-C).// 8.60E-49// 116aa// 78%// P13707
OCBBF2025730
OCBBF2026645
OCBBF2027423
OCBBF2027478
OCBBF2028173// JM11 protein [Homo sapiens]// 1.00E-131// 304aa// 97%// AAF05832
OCBBF2028935
OCBBF2029901
OCBBF2030354// Mus musculus pantothenate kinase 1 beta (panK1beta) mRNA, complete cds.// 9.50E-195// 372aa// 96%// AF200357
OCBBF2030517
OCBBF2030574
OCBBF2030708
OCBBF2031167// Homo sapiens mRNA for MDC2 alpha, MDC2 beta, complete cds.// 0// 813aa// 99%// AB009671
OCBBF2031366
OCBBF2032590// H.sapiens mRNA for melanoma-associated chondroitin sulfate proteoglycan (MCSP).// 1.80E-11// 151aa// 39%// X96753
OCBBF2032599
OCBBF2032611
OCBBF2032671
OCBBF2033869// PROCOLLAGEN C-PROTEINASE ENHANCER PROTEIN PRECURSOR (PCPE) (TYPE I PROCOLLAGEN COOH-TERMINAL PROTEINASE ENHANCER) (TYPE 1 PROCOLLAGEN C-PROTEINASE ENHANCER PROTEIN).// 6.6E-21// 151aa// 38%// Q15113
OCBBF2035110
OCBBF2035214
OCBBF2035564
OCBBF2035885
OCBBF2035916
OCBBF2036476
OCBBF2036743// ZINC FINGER PROTEIN 133.// 9.00E-157// 639aa// 48%// P52736
OCBBF2037068// BCL2/adenovirus E1B 19-kDa protein-interacting protein 2.// 3.00E-74// 122aa// 66%// 054940
OCBBF2037340// Sacsin.// 0// 356aa// 100%// Q9NZJ4
OCBBF2037398
OCBBF2037547// T-cell lymphoma invasion and metastasis 2 [Homo sapiens]// 0// 1024aa// 92%// NM_012454
OCBBF2037598// axonal-associated cell adhesion molecule [Mus musculus]// 0// 366aa// 89%// NP_031544
OCBBF2037638
OCBBF2038317// VPS10 domain receptor protein SORCS [Mus musculus]// 0// 986aa// 91%// NM_021377
OCBBF3000296
OCBBF3000483
OCBBF3002553
OCBBF3002600
OCBBF3003320// Potential phospholipid-transporting ATPase IS (EC 3.6.3.13) (Fragment).// 1.00E-110// 179aa// 62%// P98196
OCBBF3003592// Dynein beta chain, flagellar outer arm.// 2.00E-54// 222aa// 21%// Q39565
OCBBF3004314// Fas apoptotic inhibitory molecule [Mus musculus]// 8.00E-67// 117aa// 90%// NM_011810
OCBBF3006802
OCBBF3007516
OCBBF3008230
OCBBF3009279
PEBLM2000170// Sprouty homolog 3 (Spry-3).// 1.00E-31// 64aa// 100%// 043610
PEBLM2000338
PEBLM2001465// diphthamide biosynthesis; Dph5p [Saccharomyces cerevisiae]// 9.00E-65// 160aa// 57%// NP_013273
PEBLM2001488
PEBLM2002594// ATP-binding cassette, sub-family A member 8 [Homo sapiens]// 4.50E-156// 469aa// 64%// XP_016390
PEBLM2002749
PEBLM2002887// ZINC FINGER PROTEIN 195.// 1.50E-08// 62aa// 58%// 014628
PEBLM2004497
PEBLM2004666
PEBLM2005183// 5'-3' exonuclease // 0// 804aa// 92%// CAA62819
PEBLM2005697
PEBLM2006113
PEBLM2007112
PEBLM2007140
PEBLM2007834
PERIC1000147
PERIC2000889// Rattus norvegicus dynamin-like protein variant 4 mRNA, alternatively spliced, partial cds.// 3.1E-22// 51aa// 98%// AF107048
PERIC2000914
PERIC2001227
PERIC2001228
PERIC2002766
PERIC2003090
PERIC2003452
PERIC2003699
PERIC2003720// kinectin 1; CG-1 antigen [Homo sapiens].// 2.00E-92// 270aa// 90%// NP_004977
PERIC2003834
PERIC2004028// Mus musculus erythroblast macrophage protein EMP mRNA, complete cds.// 3.80E-33// 65aa// 100%// AF263247
PERIC2004259
PERIC2004379
PERIC2004429
PERIC2004909
PERIC2005347// alpha 1C adrenergic receptor isoform 2// 3.30E-22// 74aa// 70%// BAA06901
PERIC2005370
PERIC2006035
PERIC2007914// Ubiquitously transcribed TPR gene on Y chromosome [Homo sapiens]// 1.0E-22// 84aa// 67%// NP_009056
PERIC2008385// sarcosine dehydrogenase: dimethylglycine dehydrogenase-like 1 [Homo sapiens]// 4.00E-17// 47aa// 51%// NM_007101
PERIC2009086// Homo sapiens melanoma-associated antigen MG50 mRNA, partial cds.// 5.00E-189// 508aa// 66%// AF200348
PLACE5000001
PLACE5000171// E-SELECTIN PRECURSOR (ENDOTHELIAL LEUKOCYTE ADHESION MOLECULE 1) (ELAM-1) (LEUKOCYTE-ENDOTHELIAL CELL ADHESION MOLECULE 2) (LECAM2) (CD62E).// 1.50E-28// 242aa// 30%// P98110
PLACE5000260
PLACE5000282// elastin [Homo sapiens]// 8.00E-08// 420aa// 97%// NP_000492
PLACE6001185
PLACE6009006
PLACE6012574
PLACE6019385// MITOGEN-ACTIVATED PROTEIN KINASE KINASE KINASE 5 (EC 2.7.1.-) (MAPK/ERK KINASE KINASE 5) (MEK KINASE 5) (MEKK 5) (APOPTOSIS SIGNAL-REGULATING KINASE 1) (ASK-1).// 2E-57// 92aa// 63%// Q99683
PLACE6019932// Ictalurus punctatus NCC receptor protein 1 (NCCRP-1) mRNA, complete cds.// 1.2E-34// 124aa// 50%// AF208795
PLACE6020031// ANKYRIN HOMOLOG PRECURSOR.// 2.70E-06// 156aa// 35%// Q06527
PLACE7000514// Mus musculus mRNA for ER protein 58 (EP58 gene).// 3.80E-111// 366aa// 55%// AJ404004
PLACE7001022
PLACE7001936
PLACE7002641// Ring assembly protein 3.// 2.00E-13// 79aa// 26%// 074994
PLACE7006051// cytoplasmic dynein heavy chain 2 [Rattus norvegicus]// 0// 987aa// 90%// NM_023024
PLACE7008431// Phosphatidylinositol-4-phosphate 5-kinase type II alpha (EC 2.7.1.68) (PIP5KII-alpha) (1-phosphatidylinositol-4-phosphate kinase) (PtdIns(4)P-5-kinase B isoform) (Diphosphoinositide kinase).// 1.00E-109// 200aa// 56%// 070172
PLACE7008623
PROST1000184// VASOACTIVE INTESTINAL POLYPEPTIDE RECEPTOR 1 PRECURSOR (VIP-R-1) (PITUITARY ADENYLATE CYCLASE ACTIVATING POLYPEPTIDE TYPE II RECEPTOR) (PACAP TYPE II RECEPTOR) (PACAP-R-2).// 7.0E-63// 125aa// 98%// P32241
PROST1000528
PROST1000559// predicted osteoblast protein [Homo sapiens]// 6.00E-33// 227aa// 38%// NP_055703
PROST2003428// Protein pM5 precursor.// 9.00E-47// 91aa// 89%// Q15155
PROST2008993// Mus musculus Pax transcription activation domain interacting protein PTIP mRNA, complete cds.// 1.10E-211// 542aa// 77%// AF104261
PROST2015243
PROST2016462// N-chimaerin (NC) (N-chimerin) (Alpha chimerin) (A-chimaerin).// 6.00E-26// 65aa// 34%// P30337
PROST2017367// PROTEIN-GLUTAMINE GLUTAMYLTRANSFERASE 4 (EC 2.3.2.13)
(PROSTATE TRANSGLUTAMINASE) (PROSTATE TRANSGLUTAMINASE) (TGP).// 1.30E-52// 102aa// 99%// P49221
PROST2017413
PROST2017700
PROST2018030
PROST2018090// SUSHI REPEAT-CONTAINING PROTEIN SRPX PRECURSOR.// 9.50E-244// 414aa// 99%// P78539
PROST2018511// Growth factor receptor-bound protein 7 (GRB7 adapter protein) (Epidermal growth factor receptor GRB-7) (B47).// 0// 495aa// 99%// Q14451
PROST2018902
PROST2018922
PROST2019296
PROST2019781
PUAEN2002489// Homo sapiens putative seven pass transmembrane protein (TM7SF1) mRNA, complete cds.// 1.0E-48// 189aa// 53%// AF027826
PUAEN2002616
PUAEN2003079// nasopharyngeal carcinoma susceptibility protein [Homo sapiens]// 3.00E-36// 75aa// 96%// NP_037407
PUAEN2005588
PUAEN2005930
PUAEN2006328// vascular Rab-GAP/TBC-containing [Homo sapiens]// 8.0E-99// 360aa// 53%// NP_008994
PUAEN2006701
PUAEN2007044// TRNA PSEUDOURIDINE SYNTHASE B (EC 4.2.1.70) (TRNA PSEUDOURIDINE 55 SYNTHASE) (PS155 SYNTHASE) (PSEUDOURIDYLATE SYNTHASE) (URACIL HYDROLYASE).// 7.90E-15// 129aa// 34%// P45142
PUAEN2007785
PUAEN2009174
PUAEN2009655// Bos taurus phosphatidic acid-preferring phospholipase A1 mRNA, complete cds.// 0// 565aa// 96%// AF045022
PUAEN2009795// Endothelial cell multimerin precursor.// 1.00E-161// 296aa// 78%// Q13201
PUAEN2009852// serine/threonine protein kinase Kp78 splice variant CTAK75a // 3.00E-33// 86aa// 36%// AAD48007
RECTM2000433// ZG-16p [Rattus norvegicus] // 1.60E-64// 148aa// 85%// CAA83059
RECTM2001347// sphingosine kinase type 2 isoform [Homo sapiens]// 4.00E-46// 87aa// 80%// NM_020126
SKMUS2000757
SKMUS2003074
SKMUS2004047
SKMUS2006394// Mus musculus ankyrin repeat-containing protein Asb-4 mRNA, partial cds.// 6.40E-54// 405aa// 34%// AF155355
SKNMC1000124// putative nuclear protein [Homo sapiens].// 3.00E-12// 398aa// 37%// NP_057689
SKNMC2002402
SKNMC2004457
SKNMC2004643
SKNMC2005772
SKNMC2006998// PROTEIN PHOSPHATASE INHIBITOR 1 (IPP-1) (I-1).// 9.9E-32// 113aa// 64%// Q13522
SKNMC2007504// DNA-directed RNA polymerase II largest subunit (EC 2.7.7.6) (RPB1).// 1.00E-16// 76aa// 26%// P08775
SKNMC2007961
SKNMC2009450
SKNSH2000482
SKNSH2009991
SKNSH2010015
SMINT1000192// PUTATIVE ATP-DEPENDENT RNA HELICASE KIAA0134.// 4.00E-12// 37aa// 100%// Q14147
SMINT2001818
SMINT2002743
SMINT2006641
SMINT2007391
SMINT2009902
SMINT2010076// Ig alpha-1 chain C region.// 0// 319aa// 91%// P01876
SMINT2010897
SMINT2011311
SMINT2011888// protein Tro alpha1 H,myeloma// 8.9E-215// 481aa// 82%// 0501254A
SMINT2015787// immunoglobulin lambda light chain [Homo sapiens]// 1.40E-60// 164aa// 77%// CAA40954
SPLEN2001599// Homo sapiens sialic acid binding immunoglobulin-like lectin 8 long splice variant (Siglec8) gene, complete cds.// 4.00E-71// 294aa// 38%// AF287892
SPLEN2002147// Halocynthia roretzi mRNA for HrPET-3, complete cds.// 1.20E-09// 78aa// 41%// AB029335
SPLEN2002467// Homo sapiens leucine-rich repeats containing F-box protein FBL3 mRNA, complete cds.// 1.60E-187// 422aa// 77%// AF186273
SPLEN2002707
SPLEN2006122// Homo sapiens RNA-binding region (RNP1, RRM) containing 2 (RNPC2)// 2.00E-81// 147aa// 84%// NM_004902
SPLEN2009548
SPLEN2010912// putative nucleolar RNA helicase [Homo sapiens]// 0// 339aa// 90%// NM_019082
SPLEN2011422// CALDESMON (CDM).// 5.3E-12// 165aa// 37%// 005682
SPLEN2012624// BRCA1-associated RING domain protein 1 (BARD-1).// 6.00E-14// 48aa// 39%// Q9QZH2
SPLEN2012889// putative Na⁺-dependent inorganic phosphate cotransporter// 9.00E-19// 70aa// 32%// AAC35230
SPLEN2014946
SPLEN2015158
SPLEN2015267// Homo sapiens IGHG3 gene for immunoglobulin heavy chain gamma 3 constant region, 4-exon hinge, isolate Lib-A2.// 1.0E-213// 377aa// 100%// AJ390247
SPLEN2015679// Oryctolagus cuniculus sarcolemmal associated protein-3 mRNA, complete cds.// 4.90E-30// 266aa// 31%// U21157
SPLEN2016554
SPLEN2016863
SPLEN2017104
SPLEN2021701// HLA CLASS I HISTOCOMPATIBILITY ANTIGEN, A-2 ALPHA CHAIN PRECURSOR.// 4.40E-128// 173aa// 86%// P01892
SPLEN2023733
SPLEN2023791
SPLEN2024127
SPLEN2025491
SPLEN2027268
SPLEN2028844
SPLEN2028914
SPLEN2029051
SPLEN2029176
SPLEN2029522
SPLEN2029683
SPLEN2029727
SPLEN2029912
SPLEN2030335// Mus musculus fatty acid transport protein 3 mRNA, partial cds// 9.7E-251// 275aa// 81%// AF072758
SPLEN2030479
SPLEN2031125
SPLEN2031424
SPLEN2031547// Triose phosphate/phosphate translocator, non-green plastid precursor (CTPT).// 4.00E-20// 76aa// 25%// P52178
SPLEN2031724
SPLEN2031780
SPLEN2032154// NDRG1 PROTEIN (DIFFERENTIATION-RELATED GENE 1 PROTEIN) (DRG1) (REDUCING AGENTS AND TUNICAMYCIN-RESPONSIVE PROTEIN) (RTP) (NICKEL- SPECIFIC INDUCTION PROTEIN CAP43).// 1.0E-22// 80aa// 57%// Q92597
SPLEN2032321
SPLEN2032813
SPLEN2033098// tumor necrosis factor receptor superfamily, member 14// 1.7E-99// 183aa// 100%// NP_003811
SPLEN2033153
SPLEN2033539
SPLEN2033921
SPLEN2034021
SPLEN2034081
SPLEN2034678
SPLEN2034781
SPLEN2036103
SPLEN2036326// CLAUDIN-5 (TRANSMEMBRANE PROTEIN DELETED IN VCFS) (TMDVCF).// 2.6E-118// 218aa// 100%// 000501
SPLEN2036712
SPLEN2036821// MITOCHONDRIAL CARNITINE/ACYLCARNITINE CARRIER PROTEIN (CARNITINE/ACYLCARNITINE TRANSLOCASE) (CAC).// 6.5E-10// 104aa// 33%// 043772
SPLEN2036932// Homo sapiens calcium and DAG-regulated guanine nucleotide exchange factor I mRNA, complete cds.// 3.9E-63// 124aa// 100%// AF081194
SPLEN2037194// NORQ PROTEIN.// 5.5E-11// 127aa// 38%// Q51664
SPLEN2037580
SPLEN2037630
SPLEN2037722// lymphocyte antigen 108 [Mus musculus]// 3.00E-63// 137aa// 42%// NM_030710
SPLEN2038055
SPLEN2038180
SPLEN2038345
SPLEN2038407// basement membrane-induced gene // 2.1E-33// 283aa// 34%// XP_001646
SPLEN2039697
SPLEN2039936
SPLEN2040222
SPLEN2041304
SPLEN2041310
SPLEN2041645
SPLEN2041720
SPLEN2041977
SPLEN2042303
SPLEN2042598
STOMA1000189
STOMA2003444
STOMA2004294// lg lambda chain V-IV region Bau.// 1.00E-41// 79aa// 73%// P01715
STOMA2004925
STOMA2008546// CDM PROTEIN (6C6-AG TUMOR-ASSOCIATED ANTIGEN) (DXS1357E).// 5.00E-124// 246aa// 100%// P51572
SYNOV1000374 SYNOV2005216// Homo sapiens laryngeal carcinoma related protein 1 mRNA, complete cds.// 2.5E-36// 70aa// 98%// AF268387
SYNOV2005448
SYNOV2005817// CYTOKINE RECEPTOR CLASS-II CRF2-4 PRECURSOR.// 7.6E-176// 314aa// 98%// Q08334
SYNOV2006430
SYNOV2007965// Homo sapiens mRNA for H-I(3)mbt-like protein, alternative variant a.// 3.1E-118// 429aa// 54%// AJ305226
SYNOV2012326// PUTATIVE PROTEIN-TYROSINE PHOSPHATASE TPTE (EC 3.1.3.48).// 6.7E-24// 112aa// 58%// P56180
SYNOV2014400// FIBULIN-1, ISOFORM C PRECURSOR.// 4.0E-31// 198aa// 37%// P23144
SYNOV2016124
SYNOV2017055
SYNOV2018921
SYNOV2021320// SH3 DOMAIN-BINDING PROTEIN 3BP-2.// 2.3E-238// 429aa// 98%// P78314
SYNOV3000231// Ig gamma-1 chain C region.// 0// 315aa// 95%// P01857
SYNOV3000302// Ig gamma-1 chain C region.// 1.00E-173// 294aa// 89%// P01857 SYNOV4000472
SYNOV4000706// B cell phosphoinositide 3-kinase adaptor [Mus musculus]// 0// 633aa// 79%// NM_031376
SYNOV4001326
SYNOV4001395
SYNOV4002346
SYNOV4002392
SYNOV4002883// S-adenosylmethionine decarboxylase proenzyme (EC 4.1.1.50) (AdoMetDC) (SamDC) [Contains: S-adenosylmethionine decarboxylase alpha chain: S- adenosylmethionine decarboxylase beta chain].// 4.00E-72// 129aa// 99%// P17707
SYNOV4003322
SYNOV4004184
SYNOV4004741// BENE protein (Fragment).// 2.00E-77// 140aa// 94%// Q13021 SYNOV4004823
SYNOV4004914
SYNOV4006256
SYNOV4007012
SYNOV4007215
SYNOV4007360// SSXT protein (SYT protein).// 5.00E-24// 70aa// 36%// Q62280
SYNOV4007430
SYNOV4007521// fibroblast growth factor receptor-like 1 precursor [Homo sapiens]// 7.00E-11// 53aa// 29%// NM_021923
SYNOV4007553// toll-like receptor2 [Homo sapiens]// 0// 740aa// 94%// NM_003264
SYNOV4007671// Syntaxin 3.// 1.00E-144// 262aa// 99%// Q13277
SYNOV4008336
SYNOV4008440// Protein BAP28.// 0// 1119aa// 85%// Q9H583
T1ESE2000116
TBAES2001171
TBAES2001220
TBAES2001229// 60S ribosomal protein L23a.// 8.00E-48// 92aa// 82%// P29316
TBAES2001258// SERINE PROTEASE HEPSIN (EG 3.4.21.-) (TRANSMEMBRANE PROTEASE, SERINE 1).// 6.40E-19// 55aa// 87%// P05981
TBAES2001492
TBAES2001751
TBAES2002197
TBAES2003550
TBAES2004055// NY-REN-50 antigen// 1.00E-155// 290aa// 99%// AAD42878.
TBAES2005157
TBAES2005543
TBAES2006568
TBAES2007964
TCERX2000613
TCOLN2002278
TESOP1000127
TESOP2000801// PROTO-ONCOGENE TYROSINE-PROTEIN KINASE YES (EC 2.7.1.112) (P61-YES) (C-YES).// 3.9E-46// 159aa// 57%// Q04736
TESOP2001122// Caenorhabditis elegans LIN-9S (lin-9) mRNA, complete cds.// 5.60E-25// 222aa// 28%// AF269694
TESOP2001166// Mus musculus SOCS-5 mRNA, complete cds.// 1.2E-114// 439aa// 53%// AF033187
TESOP2001345
TESOP2001605// Homo sapiens laryngeal carcinoma related protein 1 mRNA, complete cds.// 2.5E-36// 70aa// 98%// AF268387
TESOP2001818
TESOP2001849
TESOP2001865
TESOP2001953// ooplasm [Mus musculus]// 7.00E-08// 58aa// 26%// NM_011860
TESOP2002273
TESOP2002451
TESOP2002489
TESOP2002539
TESOP2002950
TESOP2003273
TESOP2003753
TESOP2004114// PROCOLLAGEN-LYSINE,2-OXOGLUTARATE 5-DIOXYGENASE 2 PRECURSOR (EC 1.14.11.4) (LYSYL HYDROXYLASE 2) (LH2).// 1.70E-202// 237aa// 99%// 000469
TESOP2005285// Homo sapiens partial mRNA for chr2 synaptotagmin (CHR2SYT gene).// 1.1E-21// 54aa// 96%// AJ303365
TESOP2005485// Ig delta chain C region.// 2.00E-77// 136aa// 100%// P01880
TESOP2005579
TESOP2006041
TESOP2006060
TESOP2006068
TESOP2006670
TESOP2006746
TESOP2007052
TESOP2007262
TESOP2007636
TESOP2007688
TESOP2009121// Homo sapiens centromere protein E (312kD) (CENPE), mRNA// 2.00E-10// 155aa// 20%// NM_001813
TESOP2009555
TESTI1000257// GLUCOSE TRANSPORTER TYPE 3, BRAIN.// 7.4E-249// 493aa// 95%// P11169
TESTI1000319// Putative eukaryotic translation initiation factor 3 subunit (eIF-3) (Fragment).// 0// 683aa// 97%// 075153
TESTI1000330
TESTI1000348
TESTI1000390
TESTI1000491
TESTI1000545// Ring assembly protein 3.// 2.00E-14// 92aa// 26%// 074994
TESTI2000443
TESTI2000644// SMALL INDUCIBLE CYTOKINE A14 PRECURSOR (CHEMOKINE CC-1/CC-3) (HCC- 1/HCC-3) (NCC-2).// 2.80E-36// 69aa// 98%// 016627
TESTI2002036// DIHYDROPYRIDINE-SENSITIVE L-TYPE, SKELETAL MUSCLE CALCIUM CHANNEL ALPHA-1 SUBUNIT.// 1.70E-18// 398aa// 24%// P22316
TESTI2002618// ADAM 2 PRECURSOR (A DISINTEGRIN AND METALLOPROTEINASE DOMAIN 2) (FERTILIN BETA SUBUNIT) (PH-30) (PH30).// 1.10E-57// 253aa// 47%// Q99965
TESTI2002928
TESTI2003347// Homo sapiens connexin 59 (CX59) gene, complete cds.// 1.80E-243// 440aa// 100%// AF179597
TESTI2003573// Mus musculus cell cycle checkpoint control protein Mrad9 gene, complete cds.// 2.4E-38// 325aa// 30%// AF045662
TESTI2004215// Maackia amurensis early nodulin (ENOD2) mRNA, partial cds.// 1.3E-34// 390aa// 31%// AF039708
TESTI2004700
TESTI2005376
TESTI2005610// H.sapiens encoding CLA-1 mRNA.// 5.9E-234// 425aa// 99%// Z22555
TESTI2005739// Drosophila melanogaster Rho-kinase (Rhk) mRNA, complete cds.// 1.7E-09// 383aa// 24%// AF151375
TESTI2005986
TESTI2006041
TESTI2006643
TESTI2006648// ATP-binding cassette, sub-family C, member 5a// 9E-109// 452aa// 39%// NP_038818
TESTI2009474
TESTI2009477// TRICHOHYALIN.// 1.9E-18// 124aa// 39%// P37709
TESTI2009511
TESTI2009812
TESTI2010400
TESTI2013381
TESTI2013382
TESTI2014716// G-RICH SEQUENCE FACTOR-1 (GRSF-1).// 2.6E-228// 391aa// 99%// Q12849
TESTI2014843
TESTI2016046// Homo sapiens HOTTL protein mRNA, complete cds.// 2.8E-20// 242aa// 26%// AF078842
TESTI2017727
TESTI2018838
TESTI2019042
TESTI2019648
TESTI2023254
TESTI2023599
TESTI2024567// METABOTROPIC GLUTAMATE RECEPTOR 8 PRECURSOR.// 1.10E-130// 243aa// 99%// 000222
TESTI2026505// PUTATIVE RHO/RAC GUANINE NUCLEOTIDE EXCHANGE FACTOR (RHO/RAC GEF) (FACIOGENITAL DYSPLASIA PROTEIN).// 1.40E-50// 378aa// 29%// P98174
TESTI2027019// Homo sapiens leucine-rich repeat-containing G protein-coupled receptor 6 (LGR6) mRNA, partial cds.// 4.80E-125// 137aa// 100%// AF190501
TESTI2031529
TESTI2034520// Rattus norvegicus SMC (segregation of mitotic chromosomes 1)-like 1 (yeast) (Smc1I1), mRNA// 1.00E-145// 250aa// 53%// NM_031683
TESTI2034749
TESTI2034767// Homo sapiens collagen type IX alpha 1 chain (COL9A1) gene, long and short alternatively spliced forms, exon 38 and complete cds.// 1.40E-191// 484aa// 73%// AF036130
TESTI2034953// Homo sapiens 88-kDa Golgi protein (GM88) mRNA, complete cds.// 2.00E-27// 91aa// 64%// AF204231
TESTI2034997
TESTI2035107
TESTI2035997
TESTI2036513
TESTI2036684
TESTI2037643
TESTI2040018// Homo sapiens ZNF258 (ZNF258) mRNA, complete cds.// 7.80E-97// 461aa// 49%// AF055470
TESTI2042450
TESTI2044796// ring finger protein 3 [Homo sapiens]// 9.00E-41// 92aa// 38%// NM_006315
TESTI2044833
TESTI2045920
TESTI2045983
TESTI2046347
TESTI2047071
TESTI2048465
TESTI2048603
TESTI2048898
TESTI2049206
TESTI2049246
TESTI2049277
TESTI2049422
TESTI2049452
TESTI2049469
TESTI2049576
TESTI2049857// golgi stacking protein homolog GRASP55 [Rattus norvegicus]// 5.00E-163// 410aa// 89%// AAD55350
TESTI2050137// SHC transforming protein.// 1.00E-113// 232aa// 54%// P98083
TESTI2050681
TESTI2050987// RET finger protein-like 1.// 4.00E-35// 94aa// 34%// 075677
TESTI2051279
TESTI2051488
TESTI2051543
TESTI2051767
TESTI2051806
TESTI2051867// 60S ribosomal protein L4 (L1).// 1.00E-126// 222aa// 86%// P36578
TESTI2052211
TESTI2052693// brk kinase substrate [Homo sapiens].// 0// 341aa// 87%// CAB65105
TESTI2052698
TESTI2052822
TESTI2053242 TESTI2053399// Homo sapiens pescadillo homolog 1, containing BRCT domain (zebrafish) (PES1), mRNA// 9.00E-33// 63aa// 100%// NM_014303
TESTI2053526
TESTI2053621// Guanylyl cyclase activating protein 1 (GCAP 1) (Guanylate cyclase activator 1A).// 7.00E-96// 170aa// 92%// P43080
TESTI4000014// 130 kDa leucine-rich protein (LRP 130) (GP130).// 0// 1210aa// 96%// P42704
TESTI4000068
TESTI4000079// nuclear dual-specificity phosphatase [Homo sapiens]// 6.00E-07// 80aa// 36%// AAC39675
TESTI4000209// Homo sapiens F-BOX domain protein mRNA, complete cds.// 5.5E-103// 194aa// 99%// AF248640
TESTI4000215
TESTI4000250
TESTI4000288// Dynamin-1 (EC 3.6.1.50) (D100) (Dynamin, brain) (B-dynamin).// 2.00E-13// 38aa// 77%// P21575
TESTI4000349// thyroid hormone receptor interactor 12// 1.00E-39// 180aa// 40%// NP_004229
TESTI4000462
TESTI4000530
TESTI4000724// solute carrier family 16 (monocarboxylic acid transporters)// 5.00E-47// 490aa// 28%// NP_004687
TESTI4000970
TESTI4001100// protein tyrosine phosphatase, receptor type, f polypeptide (PTPRF), interacting protein (liprin), alpha 1 [Homo sapiens]// 3.00E-21// 50aa// 40%// NM_003626
TESTI4001106// ubiquitin-protein ligase e3 componen n-recognin [Mus musculus]// 1.00E-124// 228aa// 45%// NM_009461
TESTI4001148// Dynein beta chain, ciliary.// 1.00E-152// 282aa// 45%// P39057
TESTI4001176// Regulator of nonsense transcripts 1 (Nonsense mRNA reducing factor 1) (NORF1) (Up-frameshift suppressor 1 homolog).// 3.00E-46// 90aa// 92%// Q92900
TESTI4001201
TESTI4001206
TESTI4001527// UDP-glucuronosyltransferase 2C1 microsomal (EC 2.4.1.17) (UDPGT) (Fragment).// 9.00E-24// 64aa// 36%// P36514
TESTI4001561// 1-acyl-sn-glycerol-3-phosphate acyltransferase gamma (EC 2.3.1.51) (1- AGP acyltransferase 3) (1-AGPAT 3) (Lysophosphatidic acid acyltransferase-gamma) (LPAAT-gamma) (1-acylglycerol-3-phosphate 0-acyltransferase 3).// 0// 319aa// 93%// Q9NRZ7
TESTI4001665
TESTI4001923
TESTI4002290
TESTI4002491// Beta-soluble NSF attachment protein (SNAP-beta) (N-ethylmaleimide- sensitive factor attachment protein, beta) (Brain protein I47) (Fragment).// 1.00E-52// 99aa// 93%// P28663
TESTI4002552// Sodium/potassium-transporting ATPase alpha-4 chain (EC 3.6.3.9) (Sodium pump 4) (Na+/K+ ATPase 4) (Fragment).// 0// 505aa// 94%// Q13733
TESTI4002647
TESTI4002703
TESTI4002754
TESTI4002878
TESTI4004200
TESTI4005628
TESTI4005805
TESTI4005857
TESTI4005961
TESTI4006053
TESTI4006079// MUF1 protein: likely ortholog of mouse MUF1; elongin BC-interacting leucine-rich repeat protein [Homo sapiens]// 0// 365aa// 80%// NM_006369
TESTI4006112
TESTI4006137
TESTI4006148// putative NADH oxidoreductase complex I subunit// 2.00E-18// 40aa// 56%// AAD37863.
TESTI4006219
TESTI4006326
TESTI4006393// neural specific sr protein NSSR 2 [Mus musculus]// 7.00E-19// 70aa// 80%// BAA35093
TESTI4006412
TESTI4006420// SH3-domain binding protein 5 (BTK-associated); SH3 binding protein [Homo sapiens]// 8.00E-25// 61aa// 41%// NM_004844
TESTI4006546// colon cancer antigen NY-CO-45 [Homo sapiens].// 0// 723aa// 99%// AAC18034
TESTI4006802// mesothelin; megakaryocyte potentiating factor [Mus musculus]// 2.00E-06// 92aa// 23%// NM_018857
TESTI4006819// Alpha enolase (EC 4.2.1.11) (2-phospho-D-glycerate hydrolyase) (NON- neural enolase) (NNE) (Phosphopyruvate hydratase).// 1.00E-33// 72aa// 66%// P06733
TESTI4007064
TESTI4007163// Sodium- and chloride-dependent creatine transporter 2 (CT2) (Fragment).// 2.00E-92// 153aa// 84%// P53796
TESTI4007203
TESTI4007239
TESTI4007373
TESTI4007382
TESTI4007404
TESTI4007489
TESTI4007775
TESTI4007778// Alpha-actinin 3 (Alpha actinin skeletal muscle isoform 3) (F-actin cross linking protein).// 0// 853aa// 94%// Q08043
TESTI4007799
TESTI4007810// DNA ligase III (EG 6.5.1.1) (Polydeoxyribonucleotide synthase [ATP]).// 1.00E-112// 197aa// 86%// P49916
TESTI4008007
TESTI4008018// DAZ associated protein 2: KIAA0058 gene product [Homo sapiens]// 6.00E-41// 82aa// 75%// NM_014764
TESTI4008050// Translocation protein SEC63 homolog.// 1.00E-175// 314aa// 82%// Q9UGP8
TESTI4008219
TESTI4008401
TESTI4008429// Probable cation-transporting ATPase 2 (EC 3.6.3.-) (CGI-152).// 1.00E-136// 249aa// 94%// 09HD20
TESTI4008573
TESTI4008797
TESTI4008816
TESTI4008935
TESTI4008993
TESTI4009022
TESTI4009034
TESTI4009123
TESTI4009160// Kinesin-like protein KIF2.// 6.00E-06// 39aa// 37%// P28740
TESTI4009215
TESTI4009283
TESTI4009286// Homo sapiens HOTTL protein mRNA, complete cds// 2.00E-78// 180aa// 96%// AF078842
TESTI4009374// Apobec-1 complementation factor; APOBEC-1 stimulating protein; apobec-1 complementation factor [Homo sapiens]// 1.00E-120// 203aa// 68%// NM_014576
TESTI4009406
TESTI4009457// p53-inducible p53DINP1 [Homo sapiens]// 3.00E-80// 140aa// 88%// NM_033285
TESTI4009563// testis specific ankyrin-like protein 1 [Homo sapiens]// 1.00E-140// 239aa// 94%// NM_017844
TESTI4009608// putative T1/ST2 receptor binding protein [Homo sapiens]// 1.00E-41// 125aa// 57%// NP_006849
TESTI4009638
TESTI4009881// Dynein heavy chain, cytosolic (DYHC) (Cytoplasmic dynein heavy chain).// 5.00E-30// 176aa// 21%// Q9JHU4
TESTI4010211
TESTI4010377
TESTI4010713
TESTI4010789
TESTI4010817
TESTI4010831// yeast Sec31p homolog; ABP125 [Homo sapiens]// 0// 780aa// 81%// NM_016211
TESTI4010851// Probable ubiquitin carboxyl-terminal hydrolase FAF-X (EC 3.1.2.15) (Ubiquitin thiolesterase FAF-X) (Ubiquitin-specific processing protease FAF-X) (Deubiquitinating enzyme FAF-X) (Fat facets protein related, X-linked) (Ubiquitin-specific protease 9, X chromosome).// 2.00E-67// 213aa// 25%// Q93008
TESTI4010928
TESTI4011118
TESTI4011161
TESTI4011246
TESTI4011484// Sec23-interacting protein p125 [Homo sapiens]// 0// 387aa// 52%// NM_007190
TESTI4011505
TESTI4011745// WD-repeat protein 9 (Fragment).// 0// 674aa// 82%// Q9NSI6
TESTI4011956// Ciliary dynein heavy chain (Axonemal dynein heavy chain) (Dynein heavy chain 9).// 1.00E-170// 340aa// 39%// Q9NYC9
TESTI4012086
TESTI4012329
TESTI4012406// Apolipoprotein(A) (EC 3.4.21.-) (Apo(A)) (LP(A)) (Fragment).// 2.00E-25// 50aa// 79%// P14417
TESTI4012448// Stromelysin-3 precursor (EC 3.4.24.-) (Matrix metalloproteinase-11) (MMP-11) (ST3) (SL-3).// 0// 375aa// 99%// P24347
TESTI4012505// Tumor suppressor p53-binding protein 2 (p53-binding protein 2) (53BP2) (Bcl2-binding protein) (Bbp).// 1.00E-81// 220aa// 34%// Q13625
TESTI4012556
TESTI4012679// Homo sapiens cryptochrome 1 (photolyase-like) (CRY1), mRNA// 0// 330aa// 97%// NM_004075
TESTI4012702
TESTI4013369// ATP synthase lipid-binding protein, mitochondrial precursor (EC 3.6.1.34) (ATP synthase proteolipid P3) (ATPase protein 9) (ATPase subunit C).// 7.00E-60// 119aa// 83%// P48201
TESTI4013667
TESTI4013675
TESTI4013685
TESTI4013735
TESTI4013817// novel AMP-binding enzyme similar to acetyl-coenzyme A synthethase (acetate-coA ligase)// 8.00E-38// 99aa// 100%// CAB75500
TESTI4013830// Integral membrane glycoprotein gp210 precursor.// 0// 652aa// 41%// P11654
TESTI4013924// Intracellular protein transport protein US01.// 8.00E-20// 125aa// 20%// P25386
TESTI4014159
TESTI4014175// Chromodomain helicase-DNA-binding protein 3 (CHD-3) (Mi-2 autoantigen 240 kDa protein) (Mi2-alpha).// 0// 410aa// 75%// 012873
TESTI4014306
TESTI4014392
TEST 14014445
TESTI4014694
TESTI4014818// AD-012 protein [Homo sapiens]// 1.00E-123// 217aa// 70%// NM_018449
TESTI4014924// selective hybridizing clone [Mus musculus]// 0// 1153aa// 92%// NM_011370
TESTI4015263
TESTI4015293
TESTI4015471
TESTI4015600
TESTI4015646
TESTI4015681
TESTI4015688
TESTI4016110// DnaJ homolog subfamily B member 8 (mDJ6).// 1.00E-91// 165aa// 71%// Q9QYI7
TESTI4016238
TESTI4016551
TESTI4016812
TESTI4016822// Protein phosphatase inhibitor 2 (IPP-2).// 9.00E-72// 133aa// 83%// P41236
TESTI4016882
TESTI4016925// Dynein beta chain, ciliary.// 0// 533aa// 34%// P39057
TESTI4017001
TESTI4017137
TESTI4017254
TESTI4017543// ubinuclein 1 [Homo sapiens]// 1.00E-124// 286aa// 38%// NM_016936
TESTI4017575
TESTI4017848
TESTI4017901// alpha-1A-adrenergic receptor, isoform 2: adrenergic, alpha - 1A-, receptor: adrenergic, alpha-1C-, receptor: alpha 1A-adrenoceptor [Homo sapiens]// 9.00E-21// 51aa// 72%// NM_033303
TESTI4017961
TESTI4018152// protein tyrosine phosphatase, non-receptor type 13 [Mus musculus]// 3.00E-18// 130aa// 33%// NP_035334.
TESTI4018208// MYOSIN IC HEAVY CHAIN.// 6.10E-07// 112aa// 40%// P10569
TESTI4018382
TESTI4018555
TESTI4018806
TESTI4018835// Potential phospholipid-transporting ATPase IK (EC 3.6.3.13) (Fragment).// 0// 514aa// 88%// 060423
TESTI4018881// early endosome antigen 1, 162kD; early endosome-associated protein [Homo sapiens]// 2.00E-14// 101aa// 22%// NM_003566
TESTI4018886// M-protein, striated muscle.// 4.00E-81// 146aa// 46%// 002173
TESTI4019140// Mi-2 histone deacetylase complex protein 66 [Xenopus laevis]// 2.00E-98// 410aa// 71%// AAD55392
TESTI4019299
TESTI4019417
TESTI4019566// Dosage compensation regulator (Male-less protein) (No action potential protein).// 8.00E-49// 165aa// 29%// P24785
TESTI4019843// Rattus norvegicus huntingtin-associated protein interacting protein (duo) (Hapip), mRNA.// 0// 698aa// 91%// NM_032062
TESTI4020092// Laminin alpha-2 chain precursor (Laminin M chain) (Merosin heavy chain).// 3.00E-40// 74aa// 96%// P24043
TESTI4020102
TESTI4020806
TESTI4020920
TESTI4021294
TESTI4021456
TESTI4021478// Potential phospholipid-transporting ATPase IS (EC 3.6.3.13) (Fragment).// 0// 433aa// 54%// P98196
TESTI4021491
TESTI4022716// RNA helicase [Homo sapiens]// 0// 817aa// 95%// NM_014314
TESTI4022873// Dynein gamma chain, flagellar outer arm.// 3.00E-09// 106aa// 19%// Q39575
TESTI4022936
TESTI4023546// Sialidase (EG 3.2.1.18) (Neuraminidase) (NA) (Major surface antigen).// 6.00E-32// 134aa// 23%// P23253
TESTI4023555
TESTI4023722
TESTI4023762// Trichohyalin.// 5.00E-12// 94aa// 22%// P37709
TESTI4023942
TESTI4024344
TESTI4024420// multidomain presynaptic cytomatrix protein Piccolo [Rattus norvegicus]// 0// 789aa// 82%// NM_020098
TESTI4024874
TESTI4024890
TESTI4024907
TESTI4025731
TESTI4025797
TESTI4025920// B29 protein [Homo sapiens]// 2.00E-34// 73aa// 38%// NM_031939
TESTI4026079
TESTI4026192
TESTI4026295
TESTI4026456
TESTI4026510// RNA helicase [Homo sapiens]// 0// 445aa// 89%// NM_016130
TESTI4026524// Chromodomain helicase-DNA-binding protein 4 (CHD-4) (Mi-2 autoantigen 218 kDa protein) (Mi2-beta).// 0// 388aa// 59%// Q14839
TESTI4026700
TESTI4026762
TESTI4026785
TESTI4027516
TESTI4027557// Galectin-9 (HOM-HD-21) (Ecalectin).// 1.00E-176// 306aa// 86%// 000182
TESTI4027821
TESTI4028059// 6-phosphofructokinase, muscle type (EC 2.7.1.11) (Phosphofructokinase 1) (Phosphohexokinase) (Phosphofructo-1-kinase isozyme A) (PFK-A).// 0// 450aa// 96%// P08237
TESTI4028062
TESTI4028429// Eppin precursor.// 2.00E-32// 61aa// 76%// 095925
TESTI4028612
TESTI4028809
TESTI4028823// Niemann-Pick C1 protein precursor.// 6.00E-22// 127aa// 22%// P56941
TESTI4028880// Glucose transporter type 3, brain.// 0// 436aa// 88%// P11169
TESTI4028983
TESTI4029370
TESTI4029671
TESTI4029836// Potential phospholipid-transporting ATPase IB (EC 3.6.3.13).// 0// 888aa// 93%// P98200
TESTI4030069// fer-1 (C. elegans)-like 3 (myoferlin); fer-1 (C. elegans)-like 3 [Homo sapiens]// 4.00E-22// 64aa// 38%// NM_013451
TESTI4030159
TESTI4030505
TESTI4030603
TESTI4030669
TESTI4032895
TESTI4033433
TESTI4033690
TESTI4034172
TESTI4034212
TESTI4034432
TESTI4034632// polypeptide N-acetylgalactosaminyltransferase 9: UDP-GalNAc: polypeptide N-acetylgalactosaminyltransferase 9: GalNAc transferase 9; protein-UDP acetylgalactosaminyltransferase 9 [Homo sapiens]// 1.00E-113// 182aa// 60%// NM_021808
TESTI4034912// Intracellular protein transport protein US01.// 6.00E-38// 219aa// 21%// P25386
TESTI4035063// Restin (Cytoplasmic linker protein-170 alpha-2) (CLIP-170) (Reed- Sternberg intermediate filament associated protein).// 1.00E-17// 72aa// 27%// P30622
TESTI4035065
TESTI4035498// Septin-like protein KIAA0202 (Fragment).// 7.00E-58// 112aa// 49%// Q92599
TESTI4035602
TESTI4035637
TESTI4035649
TESTI4036042
TESTI4036909// Regulator of nonsense transcripts 1 homolog.// 9.00E-50// 140aa// 32%// Q9FJR0
TESTI4037066
TESTI4037156// WHSC2 protein [Homo sapiens]// 0// 425aa// 80%// NM_005663
TESTI4037188
TESTI4037244
TESTI4037727// Dynein beta chain, ciliary.// 0// 573aa// 73%// P39057
TESTI4038156
TESTI4038223
TESTI4038258
TESTI4038339
TESTI4038492
TESTI4038818
TESTI4039038
TESTI4039086
TESTI4039659// DnaJ homolog subfamily B member 8 (mDJ6).// 1.00E-91// 165aa// 71%// Q9QYI7
TESTI4040363// Surfeit locus protein 5.// 3.00E-62// 120aa// 100%// Q15528
TESTI4040800
TESTI4040939
TESTI4040956
TESTI4041053
TESTI4041099
TESTI4041143
TESTI4041519
TESTI4041624
TESTI4041903
TESTI4041954
TESTI4042098
TESTI4042444
TESTI4042711
TESTI4043129
TESTI4043203
TESTI4043551
TESTI4043947
TESTI4044035
TESTI4044084
TESTI4044123
TESTI4044186// leucine-rich, glioma inactivated 1 [Mus musculus]// 6.00E-65// 110aa// 60%// NM_020278
TESTI4044234
TESTI4044296
TESTI4044682
TESTI4045312
TESTI4046253
TESTI4046282
TESTI4046487// plexin 1 [Mus musculus]// 0// 433aa// 97%// NM_008881
TESTI4046819// Glucoamylase S1/S2 precursor (EC 3.2.1.3) (Glucan 1,4-alpha-glucosidase) (1,4-alpha-D-glucan glucohydrolase).// 4.00E-12// 134aa// 21%// P08640
TESTI4046884
TESTI4047069
THYMU1000496// KINESIN-LIKE PROTEIN KIF1C.// 6.40E-61// 210aa// 53%// 043896
THYMU1000600
THYMU2000932
THYMU2001053
THYMU2001090
THYMU2003397
THYMU2003632
THYMU2003760
THYMU2004693
THYMU2005003
THYMU2005190
THYMU2005303// T-CELL SURFACE GLYCOPROTEIN CD8 ALPHA CHAIN PRECURSOR (T-LYMPHOCYTE DIFFERENTIATION ANTIGEN T8/LEU-2).// 4.2E-56// 111aa// 100%// P01732
THYMU2005321
THYMU2006420// TRANSCRIPTION FACTOR-LIKE PROTEIN MRGX (KIAA0026).// 2.00E-129// 268aa// 92%// Q15014
THYMU2007060// Mus musculus Cdc42 GTPase-activating protein mRNA, complete cds.// 1.50E-37// 270aa// 40%// AF151363
THYMU2007179
THYMU2007658
THYMU2008282
THYMU2008725// PROTEIN-TYROSINE PHOSPHATASE BETA PRECURSOR (EC 3.1.3.48) (R-PTP- BETA).// 5.90E-192// 358aa// 98%// P23467
THYMU2009134
THYMU2009157// Mus musculus MRPS18b mRNA for mitochondrial ribosomal protein S18b, complete cds.// 5.00E-38// 97aa// 77%// AB049954
THYMU2009425// OLFACTORY RECEPTOR-LIKE PROTEIN HGMP07J.// 4.90E-46// 173aa// 53%// P30954
THYMU2011548// olfactory receptor 67 [Mus musculus]// 2.50E-56// 307aa// 39%// NP_038647
THYMU2011736// latent transforming growth factor beta binding protein 3// 0// 200aa// 99%// NP_066548
THYMU2013386// COTE1 PROTEIN.// 2.50E-25// 269aa// 28%// P81408
THYMU2014353
THYMU2016204
THYMU2016523
THYMU2019210// HLA CLASS I HISTOCOMPATIBILITY ANTIGEN, B-40 B*4002 ALPHA CHAIN PRECURSOR.// 2.1E-195// 248aa// 100%// Q04826
THYMU2019587
THYMU2023711// Homo sapiens mRNA for immunoglobulin lambda heavy chain.// 2.50E-233// 477aa// 89%// Y14737
THYMU2023967
THYMU2025707
THYMU2027497// 5-HYDROXYTRYPTAMINE 3 RECEPTOR PRECURSOR (5-HT-3) (SEROTONIN-GATED ION CHANNEL RECEPTOR) (5-HT3R).// 2E-10// 186aa// 24%// P46098
THYMU2027695// Ig gamma-1 chain C region.// 1.00E-169// 295aa// 78%// P01857
THYMU2027734// Homo sapiens SA hypertension-associated homolog (rat) (SAH), mRNA.// 2.00E-39// 72aa// 42%// NM_005622
THYMU2028978
THYMU2029676
THYMU2029688
THYMU2030068
THYMU2030226
THYMU2030264
THYMU2030637
THYMU2030796
THYMU2031046// Copine III.// 4.00E-28// 60aa// 75%// 075131
THYMU2031218
THYMU2031258// Homo sapiens oxysterol-binding protein-related protein (ORP1) mRNA, complete cds.// 4.0E-45// 125aa// 64%// AF274714
THYMU2031341
THYMU2031368
THYMU2031579
THYMU2031847
THYMU2031890
THYMU2032014// src homology 3 domain-containing protein HIP-55; HIP-55 protein [Homo sapiens]// 2.00E-84// 147aa// 90%// NM_014063
THYMU2032035
THYMU2032080
THYMU2032358
THYMU2032437
THYMU2032655
THYMU2032696
THYMU2032825// Mus musculus mRNA for Drctnnbla, complete cds.// 2.3E-74// 202aa// 71%// AB030242
THYMU2033070
THYMU2033079// ATP-binding cassette protein [Mus musculus].// 2.00E-53// 105aa// 91%// AAF31421
THYMU2033104// nuclear prelamin A recognition factor, isoform a [Homo sapiens]// 5.00E-34// 111aa// 47%// NP_036468
THYMU2033308
THYMU2033787
THYMU2033816
THYMU2034314
THYMU2034374// Homo sapiens MAID protein mRNA, complete cds.// 1.5E-75// 146aa// 100%// AF113535
THYMU2034647
THYMU2035064
THYMU2035101
THYMU2035319// Homo sapiens RNA-binding region (RNP1, RRM) containing 2 (RNPC2)// 0// 354aa// 81%// NM_004902
THYMU2035388
THYMU2035400
THYMU2035735// Oryctolagus cuniculus sarcolemmal associated protein-3 mRNA, complete cds.// 3.6E-154// 350aa// 90%// U21157
THYMU2036058
THYMU2036085
THYMU2036252
THYMU2036265
THYMU2036459// 240 KDA PROTEIN OF ROD PHOTORECEPTOR CNG-CHANNEL [CONTAINS: GLUTAMIC ACID-RICH PROTEIN (GARP); CYCLIC-NUCLEOTIDE-GATED CATION CHANNEL 4 (CNG CHANNEL 4) (CNG-4) (CYCLIC NUCLEOTIDE-GATED CATION CHANNEL MODULATORY SUBUNIT)].// 1.40E-13// 527aa// 24%// Q28181
THYMU2036653
THYMU2037081
THYMU2037208
THYMU2037226
THYMU2037233// RNA polymerase I transcription factor RRN3 [Homo sapiens]// 1.00E-71// 143aa// 95%// NP_060897
THYMU2037348
THYMU2037965
THYMU2038189
THYMU2038301// Homo sapiens mRNA for PRP8 protein, complete cds.// 3.90E-52// 112aa// 98%// AB007510
THYMU2038369// Mus musculus GTRGE022 (Gtrgeo22) mRNA, complete cds.// 1.10E-111// 262aa// 83%// AF303106
THYMU2038615
THYMU2038636
THYMU2038739
THYMU2038772
THYMU2038797// B locus C type Lectin [Gallus gallus]// 2.90E-15// 147aa// 34%// CAA18961
THYMU2039305// 70 KDA WD-REPEAT TUMOR-SPECIFIC ANTIGEN (FRAGMENT).// 6.90E-40// 98aa// 83%// 035828
THYMU2039315// Caenorhabditis elegans LIN-9L (lin-9) mRNA, complete cds.// 8.70E-66// 444aa// 34%// AF269693
THYMU2039350
THYMU2039411
THYMU2039780
THYMU2039989
THYMU2040140
THYMU2040412
THYMU2040824
THYMU2040975// PTB-ASSOCIATED SPLICING FACTOR (PSF).// 1.30E-08// 119aa// 36%// P23246
THYMU2041007
THYMU2041015// Monocarboxylate transporter 8 (MCT 8) (X-linked PEST-containing transporter) (MCT 7).// 1.00E-132// 230aa// 54%// P36021
THYMU2041252
THYMU3000028// Rat Tamm-Horsfall protein mRNA, complete cds.// 1.3E-21// 253aa// 28%// M63510
THYMU3000036
THYMU3000133
THYMU3000655
THYMU3000826
THYMU3001083// Tubulin epsilon chain (Epsilon tubulin).// 5.00E-26// 58aa// 98%// Q9UJT0
THYMU3001234// Dynamin 2 (EC 3.6.1.50) (Dynamin UDNM).// 1.00E-56// 108aa// 90%// P39054
THYMU3001379// 116 kDa U5 small nuclear ribonucleoprotein component (U5 snRNP- specific protein, 116 kDa) (U5-116 kDa).// 0// 492aa// 100%// Q15029
THYMU3001472
THYMU3001991// ART-4 protein [Homo sapiens]// 2.00E-46// 88aa// 97%// NM_014062
THYMU3002452
THYMU3002661
THYMU3003212// Saccharomyces cerevisiae TAD2 gene for tRNA-specific adenosine-34 deaminase subunit Tad2p.// 1.10E-21// 135aa// 40%// AJ242667
THYMU3003309// putative tumor antigen [Homo sapiens]// 2.00E-52// 105aa// 66%// NM_018666
THYMU3003763
THYMU3004157// peroxisomal acyl-CoA thioesterase [Homo sapiens]// 3.00E-44// 85aa// 82%// NM_005469
THYMU3004835// Probable beta-1,3-galactosyltransferase 8 (EC 2.4.1.-) (Beta-1,3- GalTase 8) (Beta3Gal-T8) (b3Gal-T8) (UDP-galactose:beta-N-acetylglucosamine beta-1,3-galactosyltransferase 8) (UDP-Gal:beta- GlcNAc beta-1,3-galactosyltransferase 8) (Beta-3-Gx-T8).// 2.00E-78// 146aa// 43%// Q9Y2A9
THYMU3004866// TPA inducible gene-1; TPA inducible protein [Homo sapiens]// 3.00E-47// 93aa// 86%// NM_015889
THYMU3005696
THYMU3006118// molybdenum cofactor synthesis 2 [Homo sapiens]// 3.00E-60// 112aa// 100%// NM_004531
THYMU3006132
THYMU3006168
THYMU3006172// membrane bound C2 domain containing protein [Rattus norvegicus]// 1.00E-145// 460aa// 52%// NP_058945
THYMU3006371
THYMU3006485
THYMU3006811// ATP-binding cassette, sub-family A, member 7, isoform a// 3.00E-11// 82aa// 41%// NP_061985
THYMU3006963
THYMU3007137// Interleukin-16 precursor (IL-16) (Lymphocyte chemoattractant factor) (LCF).// 0// 528aa// 83%// Q14005
THYMU3007368
THYMU3007845
THYMU3008171
THYMU3008436// 6-phosphofructokinase, muscle type (EC 2.7.1.11) (Phosphofructokinase 1) (Phosphohexokinase) (Phosphofructo-1-kinase isozyme A) (PFK-A).// 0// 764aa// 98%// P08237
THYMU3009255
TKIDN2000701// ankyrin G // 1.6E-90// 178aa// 100%// AAA64834
TKIDN2002424
TKIDN2002632
TKIDN2003044
TKIDN2004386
TKIDN2005934
TKIDN2005947
TKIDN2006525
TKIDN2006852// Homo sapiens cytosolic phospholipase A2 gamma (cPLA2 gamma) mRNA, complete cds.// 4.3E-103// 192aa// 100%// AF065214
TKIDN2007667
TKIDN2009092
TKIDN2009641
TKIDN2009889
TKIDN2010934
TKIDN2012824
TKIDN2013287
TKIDN2014757
TKIDN2014771
TKIDN2015263
TKIDN2015788
TKIDN2016309
TKIDN2019116
TLIVE2000023
TLIVE2001327// Human DOCK180 protein mRNA, complete cds.// 0// 961aa// 63%// D50857
TLIVE2001828
TLIVE2001927
TLIVE2002336// ectonucleotide pyrophosphatase/phosphodiesterase 5 [Mus musculus]// 7.00E-69// 144aa// 36%// NM_032003
TLIVE2002338
TLIVE2002690
TLIVE2003197
TLIVE2003225// CUB and Sushi multiple domains 1 [Homo sapiens]// 1.00E-129// 199aa// 58%// NM_033225
TLIVE2003381// taste receptor, type 1, member 3; saccharin preference [Mus musculus]// 8.00E-65// 112aa// 79%// NM_031872
TLIVE2003970
TLIVE2004110
TLIVE2004320// Homo sapiens PC2-glutamine-rich-associated protein (PCQAP) mRNA, complete cds.// 4.7E-201// 368aa// 99%// AF328769
TLIVE2004601
TLIVE2005180
TLIVE2006236
TLIVE2006529
TLIVE2007132
TLIVE2007528
TLIVE2007816
TLIVE2008083
TLIVE2008229// SIGNAL RECOGNITION PARTICLE 68 KDA PROTEIN (SRP68).// 1.00E-299// 506aa// 96%// Q00004
TLIVE2009541
TOVAR2000649
TOVAR2001281
TOVAR2001730
TOVAR2002247// Homo sapiens partial partial mRNA for NICE-4 protein, clone 3114f17.// 1.0E-117// 218aa// 100%// AJ243670
TOVAR2002549
TRACH1000205
TRACH2001443
TRACH2001549// Homo sapiens mRNA for neuropathy target esterase.// 1.10E-94// 295aa// 65%// AJ004832
TRACH2001684
TRACH2003070
TRACH2004170
TRACH2005066
TRACH2005811
TRACH2006049
TRACH2006387// P2Y PURINOCEPTOR 1 (ATP RECEPTOR) (P2Y1) (PURINERGIC RECEPTOR).// 2E-56// 307aa// 36%// P49650
TRACH2007059// Folate hydrolase (Prostate-specific membrane antigen 1).// 2.00E-37// 127aa// 26%// Q04609
TRACH2007834
TRACH2008300
TRACH2009310// PUTATIVE SERINE/THREONINE-PROTEIN KINASE D1044.3 IN CHROMOSOME III (EC 2.7.1.-).// 9.40E-85// 407aa// 38%// P41951
TRACH2019248
TRACH2019473
TRACH2020525
TRACH2021398
TRACH2021964
TRACH2022042
TRACH2022425// lg alpha-1 chain C region.// 0// 319aa// 91%// P01876
TRACH2022553// Human germline IgD-chain gene, C-region, second domain of membrane terminus.// 1.70E-234// 429aa// 99%// K02882
TRACH2022649// lg gamma-1 chain C region.// 0// 315aa// 95%// P01857
TRACH2023299// growth factor receptor bound protein 2-associated protein 2 [Mus musculus]// 5.00E-40// 77aa// 58%// NM_010248
TRACH2023306
TRACH2025344
TRACH2025507// tumor suppressing subtransferable candidate 1; tumor-supressing STF cDNA 1 [Homo sapiens]// 4.00E-48// 87aa// 74%// NM_003310
TRACH2025535// evectin-2 [Mus musculus]// 2.00E-75// 230aa// 90%// AAF01332
TRACH2025749
TRACH2025911
TRACH2025932
TRACH3000014
TRACH3000342
TRACH3000558// CREB-BINDING PROTEIN.// 1.9E-90// 120aa// 100%// Q92793
TRACH3000586
TRACH3000926// cardiac morphogenesis [Mus musculus]// 0// 417aa// 63%// NM_011724
TRACH3001427// p47 [Homo sapiens]// 2.00E-85// 167aa// 49%// NM_016143
TRACH3002064
TRACH3002168// Cell surface glycoprotein MUC18 precursor (Melanoma-associated antigen MUC18) (Melanoma-associated antigen A32) (S-endo 1 endothelial-associated antigen) (CD146 antigen) (Melanoma adhesion molecule).// 0// 341aa// 94%// P43121
TRACH3002192
TRACH3002650
TRACH3002866
TRACH3002871
TRACH3003379
TRACH3004068
TRACH3004537
TRACH3004721// 80 kda MCM3-associated protein (GANP protein).// 0// 474aa// 77%// 060318
TRACH3004786// Claudin-4 (Clostridium perfringens enterotoxin receptor) (CPE-receptor) (CPE-R).// 2.00E-90// 162aa// 77%// 014493
TRACH3004840
TRACH3005294
TRACH3005479
TRACH3005549// lg heavy chain V region IR2 precursor.// 4.00E-47// 89aa// 61%// P01805
TRACH3006038
TRACH3006149
TRACH3006228
TRACH3006412// Homo sapiens COP9 constitutive photomorphogenic homolog subunit 7B// 3.00E-57// 105aa// 99%// NM_022730
TRACH3006470
TRACH3006889
TRACH3007391
TRACH3007479// Nedd-4-like ubiquitin-protein ligase; WW domain-containing protein 2 [Homo sapiens]// 0// 320aa// 93%// NM_007014
TRACH3008093
TRACH3008535
TRACH3008629// Cadherin-related tumor suppressor homolog precursor (Fat protein homolog).// 6.00E-36// 143aa// 28%// Q14517
TRACH3008713// Beta-soluble NSF attachment protein (SNAP-beta) (N-ethylmaleimide- sensitive factor attachment protein, beta) (Brain protein 147) (Fragment).// 4.00E-52// 98aa// 92%// P28663
TRACH3009455// Phosphatidylinositol 3-kinase regulatory alpha subunit (PI3-kinase P85-alpha subunit) (Ptdlns-3-kinase P85-alpha) (PI3K).// 0// 386aa// 95%// P27986
TRACH3034731// Ras association (RaIGDS/AF-6) domain family 2// 7.00E-56// 320aa// 40%// NP_055552
TRACH3034762
TRACH3035199// antigen identified by monoclonal antibody MRC OX-2 receptor [Rattus norvegicus]// 1.00E-86// 170aa// 51%// NM_023953
TRACH3035235
TRACH3035482
TRACH3035526// Ig alpha-2 chain C region.// 0// 324aa// 95%// P01877
TRACH3036193// Genome polyprotein [Contains: Coat proteins VP1 TO VP4; Core proteins P2A TO P2C, P3A: Genome-linked protein VPG; Picornain 3C (EC 3.4.22.28) (Protease 3C) (P3C); RNA-directed RNA polymerase P3D (EC 2.7.7.48)].// 0// 1073aa// 69%// Q82122
TRACH3036207
TRACH3036309
TRACH3036456
TRACH3036609// J kappa-recombination signal binding protein (RBP-J kappa).// 1.00E-158// 271aa// 89%// P31266
TSTOM1000135
TSTOM2000442// Ig gamma-1 chain C region.// 1.00E-168// 292aa// 77%// P01857
TSTOM2000553// SYNAPTOTAGMIN IV.// 3.00E-08// 150aa// 28%// P40749
TSTOM2002672
TUTER1000122
TUTER2000425// zinc finger protein SBZF3 [Homo sapiens]// 4.00E-36// 74aa// 81%// NM_020394
TUTER2000904// Unc-119 protein homolog (Retinal protein 4) (RRG4).// 7.00E-72// 129aa// 70%// Q62885
TUTER2000916
TUTER2001387
TUTER2002729// D6MM5E protein [Mus musculus]// 1.00E-107// 191aa// 68%// NM_033079
UTERU1000024
UTERU1000031// G.gallus mRNA for tom-1B protein.// 2.1E-149// 535aa// 59%// Y08741
UTERU1000148
UTERU1000249
UTERU1000337// Putative protein phosphatase 2C (EC 3.1.3.16) (PP2C).// 1.00E-156// 271aa// 94%// P49593
UTERU1000339
UTERU2000649
UTERU2001409
UTERU2002410
UTERU2002841
UTERU2004688
UTERU2004929
UTERU2005004
UTERU2005621// CDC14 homolog B, isoform 2 [Homo sapiens]// 0// 423aa// 94%// NM_033331
UTERU2006115// ALPHA-ADAPTIN A (CLATHRIN ASSEMBLY PROTEIN COMPLEX 2 ALPHA-A LARGE CHAIN) (100 KDA COATED VESICLE PROTEIN A) (PLASMA MEMBRANE ADAPTOR HA2/AP2 ADAPTIN ALPHA A SUBUNIT).// 9.0E-141// 268aa// 99%// P17426
UTERU2006137
UTERU2006568
UTERU2007444
UTERU2007520
UTERU2007724// Calponin H2, smooth muscle (Neutral calponin).// 1.00E-144// 253aa// 86%// Q99439
UTERU2008347// Chlamydomonas reinhardtii vegetative cell wall protein gp1 (GP1) gene, complete cds.// 1.0E-19// 199aa// 30%// AF309494
UTERU2014678
UTERU2017762// plexin B1; KIAA0407 protein: plexin 5 [Homo sapiens]// 0// 383aa// 60%// NM_002673
UTERU2019491// Homo sapiens mRNA for 41-kDa phosphoribosylpyrophosphate synthetase-associated protein, complete cds.// 4.30E-48// 101aa// 100%// AB007851
UTERU2019681
UTERU2019706// T-COMPLEX PROTEIN 1, GAMMA SUBUNIT (TCP-1-GAMMA) (CCT-GAMMA).// 9.80E-273// 426aa// 99%// P49368
UTERU2019940// mitochondrial ribosomal protein L30 [Homo sapiens]// 2.00E-44// 82aa// 97%// NM_016503
UTERU2020491
UTERU2020718
UTERU2021163
UTERU2021380
UTERU2022020
UTERU2022981
UTERU2023039
UTERU2023175
UTERU2023651
UTERU2023712
UTERU2024002
UTERU2024656
UTERU2025025// High affinity nerve growth factor receptor precursor (EC 2.7.1.112) (TRK1 transforming tyrosine kinase protein) (p140-TrkA) (Trk-A).// 0// 479aa// 95%// P04629
UTERU2025645
UTERU2025891
UTERU2026025// SPLICING FACTOR, ARGININE/SERINE-RICH 2 (SPLICING FACTOR SC35) (SC-35) (SPLICING COMPONENT, 35 KDA) (PR264 PROTEIN).// 8.00E-30// 61aa// 100%// P30352
UTERU2026090// Cartilage-associated protein precursor.// 1.00E-180// 309aa// 87%// 075718
UTERU2026203// phosphoinositide phosphatase SAC1 [Rattus norvegicus].// 1.00E-107// 221aa// 95%// AAG29810
UTERU2027591// calcium-activated potassium channel // 7.8E-33// 79aa// 94%// AAA50216
UTERU2029953
UTERU2030213
UTERU2030280
UTERU2031084
UTERU2031268// NY-REN-25 antigen [Homo sapiens].// 1.00E-41// 330aa// 49%// AAD42869
UTERU2031521
UTERU2031703
UTERU2031851
UTERU2033375
UTERU2033382
UTERU2035114
UTERU2035323
UTERU2035328// Homo sapiens putative transcription factor CA150 mRNA, complete cds.// 1.80E-271// 796aa// 70%// AF017789
UTERU2035331
UTERU2035452// NG3 [Homo sapiens]// 1.00E-136// 150aa// 99%// AAB47494
UTERU2035469// Mus musculus microfibril-associated glycoprotein-2 (Magp2) mRNA, complete cds.// 1.2E-52// 164aa// 66%// AF180805
UTERU2035503
UTERU2035745// MYOSIN IA HEAVY CHAIN (MYOSIN-LIKE PROTEIN ABMA).// 1.30E-11// 101aa// 31%// P22467
UTERU2036089// SH3-BINDING PROTEIN 3BP-1.// 4.1E-168// 369aa// 86%// P55194
UTERU2037361
UTERU2037577
UTERU2038251
UTERU3000226
UTERU3000645// Claudin-4 (Clostridium perfringens enterotoxin receptor) (CPE-receptor) (CPE-R).// 3.00E-89// 161aa// 77%// 014493
UTERU3000665// Snf2-related CBP activator protein [Homo sapiens].// 7.00E-59// 500aa// 97%// NP_006653
UTERU3000828// 116 kDa U5 small nuclear ribonucleoprotein component (U5 snRNP- specific protein, 116 kDa) (U5-116 kDa).// 0// 931aa// 95%// Q15029
UTERU3000899// hTGN51 [Homo sapiens].// 1.00E-101// 281aa// 72%// AAC39542
UTERU3001059// ABC1 protein homolog, mitochondrial precursor.// 2.00E-99// 188aa// 48%// Q92338
UTERU3001240// Adenylate cyclase, type IV (EC 4.6.1.1) (ATP pyrophosphate-lyase) (Adenylyl cyclase).// 1.00E-176// 308aa// 81%// P26770
UTERU3001542
UTERU3001571
UTERU3001572// Neuroblast differentiation associated protein AHNAK (Desmoyokin) (Fragments).// 6.00E-19// 213aa// 21%// Q09666
UTERU3001585// Cytochrome P450 4c3 (EC 1.14.-.-) (CYPIVC3).// 1.00E-125// 230aa// 49%// 09VA27
UTERU3001652// 64 KDA AUTOANTIGEN D1 (THYROID-ASSOCIATED OPHTHALMOPATHY AUTOANTIGEN).// 1.00E-219// 416aa// 99%// P29536
UTERU3001766
UTERU3001988// COATOMER EPSILON SUBUNIT (EPSILON-COAT PROTEIN) (EPSILON-COP).// 1.70E-126// 159aa// 94%// Q28104
UTERU3002209
UTERU3002218
UTERU3002383
UTERU3002667
UTERU3002731
UTERU3002768
UTERU3002786
UTERU3002993
UTERU3003116// ADAM 12 precursor (EC 3.4.24.-) (A disintegrin and metalloproteinase domain 12) (Meltrin alpha).// 1.00E-20// 48aa// 44%// 043184
UTERU3003135// Splicing factor 3B subunit 2 (Spliceosome associated protein 145) (SAP 145) (SF3b150) (Pre-mRNA splicing factor SF3b 145 kDa subunit).// 6.00E-29// 62aa// 83%// Q13435
UTERU3003178// Kinesin light chain 2 (KLC 2).// 0// 312aa// 88%// Q9HOB6
UTERU3003465
UTERU3003523
UTERU3003776
UTERU3004523
UTERU3004616
UTERU3004709
UTERU3004992// Aortic preferentially expressed protein 1 (APEG-1).// 3.00E-61// 113aa// 100%// Q15772
UTERU3005049
UTERU3005205
UTERU3005230
UTERU3005460
UTERU3005585// rhophilin-like protein [Homo sapiens]// 0// 380aa// 91%// NM_033103
UTERU3005907// PROTEIN-GLUTAMINE GAMMA-GLUTAMYLTRANSFERASE (EC 2.3.2.13) (TISSUE TRANSGLUTAMINASE) (TGASE C) (TGC) (TGASE-H).// 1.30E-75// 152aa// 98%// P21980
UTERU3005970
UTERU3006008
UTERU3006308// SEMAPHORIN 4C PRECURSOR (SEMAPHORIN I) // 1.00E-128// 330aa// 86%// 064151
UTERU3007134
UTERU3007419// Rattus norvegicus Ca2+-dependent activator protein (CAPS) mRNA, complete cds.// 0// 1223aa// 78%// U16802
UTERU3007640// N-ethylmaleimide-sensitive factor attachment protein, alpha:// 5.00E-54// 110aa// 87%// NP_003818
UTERU3007913
UTERU3008660
UTERU3008671// SPARC precursor (Secreted protein acidic and rich in cysteine) (Osteonectin) (ON) (Basement membrane protein BM-40).// 4.00E-25// 49aa// 96%// P09486
UTERU3009259
UTERU3009490// LYSP100 protein (Lymphoid-restricted homolog of Sp100) (Nuclear autoantigen Sp-140) (Speckled 140 kDa) (Nuclear body protein Sp140).// 4.00E-33// 67aa// 61%// Q13342
UTERU3009517
UTERU3009690// alpha-1A-adrenergic receptor, isoform 2; adrenergic, alpha - 1A-, receptor; adrenergic, alpha-1C-, receptor; alpha 1A-adrenoceptor [Homo sapiens]// 5.00E-16// 41aa// 67%// NM_033303
UTERU3009871// feminization 1 homolog a (C. elegans)// 0// 588aa// 85%// NP_034322
UTERU3009979// growth arrest-specific 6; AXL stimulatory factor [Homo sapiens]// 0// 572aa// 97%// NM_000820
UTERU3011063// Transmembrane 9 superfamily protein member 4.// 0// 402aa// 89%// Q92544
UTERU3015086
UTERU3015500// G protein-coupled receptor 49// 1.00E-15// 350aa// 29%// NP_003658
UTERU3016789// SH3 domain-binding protein 3BP-2.// 1.00E-146// 261aa// 75%// P78314
UTERU3018081
UTERU3018154
UTERU3018616
UTERU3018711
3NB692004724
ADRGL2000042// Homo sapiens CTCL tumor antigen se20-4 mRNA, complete cds.// 6.20E-143// 269aa// 100%// AF273046
ADRGL2000056
BLADE2000579
BLADE2006830
BRACE2002589
BRACE2003609// endothelial zinc finger protein induced by tumor necrosis factor alpha [Homo sapiens]// 0// 310aa// 67%// NM_021216
BRACE2009318
BRACE2011677
BRACE2029396
BRACE2037299
BRACE2039823// CDP-DIACYLGLYCEROL―INOSITOL 3-PHOSPHATIDYLTRANSFERASE (EC 2.7.8.11) (PHOSPHATIDYLINOSITOL SYNTHASE) (PTDINS SYNTHASE) (PI SYNTHASE).// 2.10E-79// 154aa// 100%// 014735
BRACE2039832
BRACE2043105
BRACE3001058// zinc finger protein 347; zinc finger 1111 [Homo sapiens]// 0// 382aa// 52%// NM_032584
BRACE3001113// Zinc finger protein 91 (Zinc finger protein HTF10) (HPF7).// 6.00E-92// 226aa// 26%// Q05481
BRACE3003026
BRACE3003053
BRACE3005107// // // // //
BRACE3009127// oxysterol binding protein 2: oxysterol binding protein-like 1 [Homo sapiens]// 0// 670aa// 95%// NM_030758
BRACE3010076// Vigilin (High density lipoprotein-binding protein) (HDL-binding protein).// 0// 464aa// 92%// Q00341
BRACE3015829
BRACE3021148// DC12 protein [Homo sapiens]// 1.00E-16// 60aa// 30%// NM_020187
BRALZ2017844// HOMEOBOX PROTEIN CHOX-E (CHOX E) (FRAGMENT).// 2.80E-59// 157aa// 75%// Q91975
BRAMY2019111// POLYCYSTIN 2.// 6.30E-18// 204aa// 27%// 035245
BRAMY2035070// Homo sapiens zinc finger 1111 mRNA, complete cds.// 1.70E-213// 723aa// 53%// AY029765
BRAMY2035449// Mus musculus zinc finger protein ZFP113 mRNA, complete cds.// 2.70E-95// 356aa// 44%// AF167320
BRAMY2035718// NUCLEAR FACTOR 1-B (NFI-B) (CCAAT BOX-BINDING TRANSCRIPTION FACTOR) (CTF) (TGGCA-BINDING PROTEIN).// 5.60E-228// 418aa// 97%// P97863
BRAMY2038516// PROBABLE PROTEIN DISULFIDE ISOMERASE P5 PRECURSOR (EC 5.3.4.1).// 7.00E-237// 434aa// 99%// Q15084
BRAMY2039341// ZINC FINGER PROTEIN 135.// 7.30E-84// 227aa// 62%// P52742
BRAMY2040159// Homo sapiens MRIP-1 mRNA, complete cds.// 6.00E-234// 345aa// 91%// AF359283
BRAMY2041434
BRAMY2045471// Homo sapiens CGI-89 protein mRNA, complete cds.// 6.00E-45// 147aa// 44%// CAB82308
BRAMY3004800// Huntingtin-associated protein-interacting protein (Duo protein).// 0// 751aa// 91%// 060229
BRAWH1000369// Homo sapiens putative DNA polymerase mRNA, partial cds.// 5.40E-150// 199aa// 97%// AF044578
BRAWH2006207// ZINC FINGER PROTEIN 91 (ZINC FINGER PROTEIN HTF10) (HPF7).// 8.90E-32// 83aa// 81%// 005481
BRAWH2006395// transmembrane receptor UNC5H1// 1.00E-141// 292aa// 88%// AAB57678
BRAWH2008993
BRAWH2009393
BRAWH2010552
BRAWH3007441// CAT56 protein [Homo sapiens]// 1.00E-44// 78aa// 84%// NM_025263
BRAWH3009017// SEL-10 protein.// 6.00E-38// 73aa// 61%// Q93794
BRCAN2002473// Tropomyosin, fibroblast isoform 2 (TM-2).// 1.00E-114// 230aa// 70%// P19354
BRCAN2002854// Human Hsp27 ERE-TATA-binding protein (HET) mRNA, complete cds.// 2.60E-21// 240aa// 35%// U72355
BRCAN2003070// ubiquitin carrier protein E2-C [Homo sapiens]// 9.00E-51// 80aa// 78%// NP_008950
BRCAN2014229
BRCOC2019841
BRHIP2002722
BRHIP2003272
BRHIP2005271// protein tyrosine phosphatase// 7.00E-39// 190aa// 42%// NP_006255
BRHIP2005724// Homo sapiens mRNA for NDRG4-B, complete cds.// 3.10E-182// 301aa// 98%// AB044944
BRHIP2006617// NUCLEAR AUTOANTIGENIC SPERM PROTEIN (NASP).// 1.20E-156// 313aa// 97%// P49321
BRHIP2008389// Drosophila melanogaster furry protein short isoform mRNA, partial cds.// 1.70E-29// 179aa// 44%// AF351187
BRHIP2012360// XPG_XENLA// 4.00E-17// 70aa// 41%// P14629
BRHIP2017553// Zinc finger protein 184.// 1.00E-164// 272aa// 49%// Q99676
BRHIP2026877// Tyrosine-protein kinase receptor TYR03 precursor (EC 2.7.1.112) (Tyrosine-protein kinase RSE) (Tyrosine-protein kinase SKY) (Tyrosine- protein kinase DTK) (Protein-tyrosine kinase byk).// 3.00E-56// 104aa// 80%// Q06418
BRHIP3000017
BRHIP3000240// Homo sapiens potassium channel beta 2 subunit (HKvbeta2.2) mRNA, alternatively spliced, complete cds.// 1.70E-172// 347aa// 95%// AF044253
BRHIP3008314// sirtuin 2, isoform 2: silencing information regulator 2-like; sir2-like 2: silent mating type information regulation 2, S.cerevisiae, homolog 2; sir2-related protein type 2 [Homo sapiens]// 2.00E-48// 86aa// 98%// NM_030593
BRHIP3026052// Serine/threonine protein phosphatase 2A, 56 kDa regulatory subunit, gamma isoform (PP2A, B subunit, B' gamma isoform) (PP2A, B subunit, B56 gamma isoform) (PP2A, B subunit, PR61 gamma isoform) (PP2A, B subunit, R5 gamma isoform).// 0// 424aa// 83%// Q13362
BRSTN2013354// ETS-related protein PE-1 (ETS translocation variant 3) (Fragment).// 4.00E-61// 109aa// 84%// P41162
BRTHA2002133
BRTHA2002702
BRTHA2007060// EUKARYOTIC TRANSLATION INITIATION FACTOR 3 SUBUNIT 10 (EIF-3 THETA) (EUKARYOTIC TRANSLATION INITIATION FACTOR 3 LARGE SUBUNIT) (PNLA-35).// 0// 963aa// 74%// Q40554
BRTHA2010033
BRTHA2011321
BRTHA2013426
BRTHA2013610// deoxyguanosine kinase, putative [Arabidopsis thaliana]// 1.00E-142// 360aa// 78%// NP_565032*
BRTHA2016318// WTAP protein// 9.00E-87// 240aa// 100%// CAC10188
BRTHA2017364// PUTATIVE ATP-DEPENDENT RNA HELICASE T26G10.1 IN CHROMOSOME III.// 1.10E-33// 207aa// 34%// P34580
BRTHA2017972// Homo sapiens MAD-related gene SMAD7 (SMAD7) mRNA, complete cds.// 5.30E-114// 207aa// 99%// AF010193
BRTHA2018011// EPITHIN (EC 3.4.21.-).// 1.70E-53// 242aa// 45%// P56677
BRTHA2018443// POTENTIAL PHOSPHOLIPID-TRANSPORTING ATPASE IS (EC 3.6.1.-) (FRAGMENT).// 3.10E-195// 581aa// 61%// P98196
BRTHA3000296
BRTHA3003000// apoptosis-associated tyrosine kinase [Homo sapiens]// 1.00E-161// 300aa// 64%// NM_004920
BRTHA3008826
CERVX2002013// TRANSCRIPTION FACTOR BTEB2 (BASIC TRANSCRIPTION ELEMENT BINDING PROTEIN 2) (GC BOX BINDING PROTEIN 2).// 3.30E-33// 81aa// 81%// Q13887
CTONG1000113// ZINC FINGER PROTEIN 184 (FRAGMENT).// 0// 641aa// 85%// Q99676
CTONG2003348// Mus musculus mRNA for OASIS protein, complete cds.// 9.60E-87// 392aa// 51%// AB017614
CTONG2004000
CTONG2008721// Homo sapiens CAGH44 mRNA, partial cds.// 2.70E-94// 215aa// 91%// U80741
CTONG2015596
CTONG2015633
CTONG2016942// Homo sapiens serine protease DESC1 (DESC1) mRNA, complete cds.// 2.00E-95// 425aa// 43%// AF064819
CTONG2019822
CTONG2020374
CTONG2020378// ZINC FINGER PROTEIN 35 (ZFP-35).// 5.70E-100// 322aa// 55%// P15620
CTONG2020411// BASONUCLIN.// 1.30E-139// 616aa// 48%// Q01954
CTONG2020974// Homo sapiens mRNA for putative progesterone binding protein.// 8.10E-118// 223aa// 100%// AJ002030
CTONG2024031
CTONG2028758// Mus musculus zfh-4 mRNA for zinc-finger homeodomain protein 4, complete cds.// 0// 907aa// 91%// AB024499
CTONG3001501// Mus musculus glucocorticoid-induced gene 1 mRNA, complete cds.// 3.60E-202// 413aa// 89%// AF292939
CTONG3002552
CTONG3003598
CTONG3004550// SH3-domain binding protein 4 [Homo sapiens]// 1.00E-179// 351aa// 42%// NM_014521
CTONG3004726// ZINC FINGER PROTEIN 91 (ZINC FINGER PROTEIN HTF10) (HPF7).// 4.20E-230// 663aa// 55%// Q05481
CTONG3009287
DFNES2011192// Homo sapiens ZNF140-like transcription factor mRNA, complete cds.// 1.10E-221// 396aa// 99%// AF326206
FCBBF1000509// Homo sapiens mRNA for photolyase, complete cds.// 9.80E-280// 500aa// 99%// D83702
FCBBF3010361// Danio rerio mRNA for winged helix nude (whn gene).// 2.10E-107// 336aa// 64%// AJ252024
FCBBF3027854// contains similarity to Arabidopsis thaliana DNA-damage-repair/tolerance resistance protein DRT111 (SW:P42698// 9.60E-12// 87aa// 37%// AAC13593
FEBRA2000790
FEBRA2001990// SON OF SEVENLESS PROTEIN HOMOLOG 2 (SOS-2) (MSOS-2) (FRAGMENT).// 4.00E-09// 175aa// 20%// Q02384
FEBRA2006519// Mus musculus papilin mRNA, complete cds.// 4.90E-58// 327aa// 37%// AF314171
FEBRA2008692// Homo sapiens IRE1b mRNA for protein kinase/ribonuclease IRE1 beta, complete cds.// 0// 926aa// 96%// AB047079
FEBRA2014122// wizL [Mus musculus]// 0// 661aa// 88%// BAA32790
FEBRA2027609// ZINC FINGER PROTEIN 91 (ZINC FINGER PROTEIN HTF10) (HPF7).// 5.60E-196// 688aa// 51%// Q05481
FEBRA2028256// FIBRILLIN 2 PRECURSOR.// 2.90E-231// 527aa// 62%// Q61555
FEBRA2028516
HCASM2002754
HCASM2003018// Homo sapiens nuclear transcription factor NFX2 (NFX2) mRNA, complete cds.// 3.90E-70// 666aa// 31%// AF332009
HCASM2003099// HISTONE DEACETYLASE CLR3.// 5.00E-19// 108aa// 49%// P56523
HCASM2003357
HCASM2008536// DNA-REPAIR PROTEIN XRCC1.// 2.00E-12// 119aa// 36%// P18887
HCASM2009424// regulatory factor (trans-acting) 2 [Mus musculus]// 7.00E-24// 60aa// 48%// NP_033082
HCHON2000508// Homo sapiens prostate antigen PARIS-1 mRNA, complete cds.// 0// 686aa// 99%// AY026527
HCHON2000743
HCHON2004858// Zinc finger protein 29 (Zfp-29).// 1.00E-124// 208aa// 60%// Q07230
HEART2009680// Vasoactive intestinal polypeptide receptor 2 precursor (VIP-R-2) (Pituitary adenylate cyclase activating polypeptide type III receptor) (PACAP type III receptor) (PACAP-R-3) (Helodermin-preferring VIP receptor).// 0// 309aa// 96%// P41587
HLUNG2013350// Mus musculus mRNA for synaptotagmin VIII, complete cds.// 1.70E-39// 126aa// 67%// AB026805
HLUNG2015418// similar to cadherin and Drosophila Fat protein; similar to CAA60685 (PID:g1107687) [Homo sapiens].// 1.00E-139// 500aa// 91%// AAD28068
HLUNG2015548// INOSINE-5'-MONOPHOSPHATE DEHYDROGENASE 1 (EC 1.1.1.205) (IMP DEHYDROGENASE 1) (IMPDH-I) (IMPD 1).// 3.30E-253// 521aa// 94%// P20839
HLUNG2016862
HSYRA2005628// ZINC FINGER PROTEIN 195.// 1.30E-237// 426aa// 78%// 014628
IMR322001879
IMR322007078
IMR322008651// DNA REPAIR PROTEIN RAD51 HOMOLOG 1.// 8.40E-154// 340aa// 89%// Q06609
IMR322013396// Homo sapiens cone photoreceptor cGMP-gated channel alpha subunit (CNGA3) mRNA, complete cds.// 0// 638aa// 98%// AF065314
IMR322013731// TAT-BINDING HOMOLOG 7.// 1.90E-74// 187aa// 47%// P54816 LIVER2000247// RENAL SODIUM/DICARBOXYLATE COTRANSPORTER (NA(+)/DICARBOXYLATE COTRANSPORTER).// 2.70E-57// 243aa// 48%// Q13183
MESAN2001770// EUKARYOTIC TRANSLATION INITIATION FACTOR 4 GAMMA (EIF-4-GAMMA) (EIF- 4G) (EIF4G) (P220).// 4.70E-185// 420aa// 86%// Q04637
MESAN2005303// DNA BINDING PROTEIN URE-B1 (EC 6.3.2.-).// 2.80E-36// 171aa// 46%// P51593
MESAN2014412// Mus musculus zfh-4 mRNA for zinc-finger homeodomain protein 4, complete cds.// 2.8e-317// 606aa// 92%// AB024499
MESAN2015501// ZINC FINGER PROTEIN 91 (ZINC FINGER PROTEIN HTF10) (HPF7).// 7.80E-222// 753aa// 52%// Q05481
NT2RI2005772// Rattus norvegicus mRNA for DLG6 alpha, complete cds.// 4.30E-176// 273aa// 75%// AB030499
NT2RI2008952// ZINC FINGER PROTEIN 84 (ZINC FINGER PROTEIN HPF2).// 1.40E-131// 393aa// 55%// P51523
NT2RI2009583// Homo sapiens orphan G-protein coupled receptor (GPRC5C) mRNA, complete cds.// 3.70E-241// 441aa// 100%// AF207989
NT2RI2018448// AE-binding protein 2 [Mus musculus]// 1.00E-153// 258aa// 92%// NM_009637
NT2RI2027157// Mouse SDR2 mRNA, complete cds.// 3.20E-233// 539aa// 77%// D50464
NT2R13000174// Homo sapiens HepA-related protein HARP mRNA, complete cds.// 1.00E-38// 136aa// 46%// NP_054859.1
NT2RI3001132// Mus musculus Arkadia (Arkadia) mRNA, complete cds.// 0// 994aa// 90%// AF330197
NT2RI3002557
NT2RI3005928
NT2RI3007167
NT2RI3007443// MITOGEN-ACTIVATED PROTEIN KINASE KINASE KINASE 5 (EC 2.7.1.-) (MAPK/ERK KINASE KINASE 5) (MEK KINASE 5) (MEKK 5) (APOPTOSIS SIGNAL-REGULATING KINASE 1) (ASK-1).// 9.50E-244// 606aa// 66%// Q99683
NT2RP7008435// EPITHIN (EG 3.4.21.-).// 6.60E-79// 354aa// 43%// P56677
NT2RP8000521
NTONG2008093// 6PF-2-K/FRU-2,6-P2ASE BRAIN/PLACENTA-TYPE ISOZYME [INCLUDES: 6- PHOSPHOFRUCTO-2-KINASE (EG 2.7.1.105); FRUCTOSE-2,6-BISPHOSPHATASE (EC 3.1.3.46)].// 1.60E-72// 143aa// 97%// Q16875
OCBBF2003327// ADAM-TS 6 precursor (EC 3.4.24.-) (A disintegrin and metalloproteinase with thrombospondin motifs 6) (ADAMTS-6) (ADAM-TS6).// 2.00E-63// 108aa// 51%// Q9UKP5
OCBBF2005433// N-CHIMAERIN (NC) (N-CHIMERIN) (ALPHA CHIMERIN) (A-CHIMAERIN).// 7.70E-24// 213aa// 33%// P30337
OCBBF2006987
OCBBF2008144// ZINC FINGER PROTEIN 91. (ZINC FINGER PROTEIN HTF10) (HPF7).// 5.50E-244// 757aa// 56%// 005481
OCBBF2009583// ZINC FINGER PROTEIN 184 (FRAGMENT).// 1.20E-75// 345aa// 36%// Q99676
OCBBF2011669// Zinc finger protein 91 (Zinc finger protein HTF10) (HPF7).// 1.00E-90// 222aa// 26%// 005481
OCBBF2019684// ZINC FINGER PROTEIN 29 (ZFP-29).// 6.90E-246// 465aa// 90%// Q07230
OCBBF2020048// 95 kDa retinoblastoma protein binding protein: KIAA0661 gene product // 9.90E-97// 339aa// 63%// XP_010492
OCBBF2024284// GUANINE NUCLEOTIDE-BINDING PROTEIN BETA SUBUNIT-LIKE PROTEIN 12.3 (P205) (RECEPTOR OF ACTIVATED PROTEIN KINASE C 1) (RACK1).// 1.60E-72// 135aa// 99%// P25388
OCBBF2030116
OCBBF2032274// Zinc finger protein 93 (Zinc finger protein HTF34) (Fragment).// 1.00E-145// 247aa// 67%// P35789
OCBBF2034637// microtubule associated testis specific serine/threonine protein kinase [Mus musculus]// all 450aa// 79%// NP_032667
OCBBF3000167// ZINC FINGER PROTEIN 83 (ZINC FINGER PROTEIN HPF1).// 5.00E-156// 405aa// 62%// P51522
OCBBF3002654// Triple functional domain protein (PTPRF interacting protein).// 0// 510aa// 93%// 075962
OCBBF3003761
OCBBF3004972
PERIC2007068// Mus musculus mRNA for 1A13 protein.// 3.30E-121// 385aa// 62%// X83587
PLACE7000333
PLACE7000502// Human Notch4 (hNotch4) mRNA, complete cds.// 0// 666aa// 99%// U95299
PROST2000452// TRANSMEMBRANE PROTEASE, SERINE 2 (EC 3.4.21.-).// 1.60E-47// 186aa// 50%// 015393
PROST2009320// enigma protein: LIM domain protein [Homo sapiens]// 6.00E-42// 77aa// 71%// NM_005451
PROST2019487
PUAEN2006335
SKMUS2003194
SPLEN2004611// Homo sapiens transcription factor 7-like 2 (T-cell specific, HMG-box) (TCF7L2),// 1.00E-19// 50aa// 47%// NM_030756
SPLEN2016135// Zinc finger protein 2 (Zfp-2) (mKR2 protein).// 6.00E-27// 64aa// 39%// P08043
SPLEN2016781// zinc finger transcription factor REST protein // 5.00E-18// 80aa// 33%// AAB94893
SPLEN2016932
SPLEN2030847// Mus musculus Kif21b (Kif21b) mRNA, complete cds.// 1.00E-85// 110aa// 94%// AF202893
SPLEN2033490
SPLEN2036702// TENSIN.// 8.30E-175// 409aa// 54%// 004205
SPLEN2037319
SPLEN2039311// Pro-Pol-dUTPasepolyprotein.*// 2.70E-15// 93aa// 53%// 002711*
SPLEN2039379// Homo sapiens phosphatidylinositol 4-kinase 230 (pi4K230) mRNA, complete cds.// 0// 645aa// 99%// AF012872
STOMA2003158// DEOXYRIBONUCLEASE GAMMA PRECURSOR (EC 3.1.21.-) (DNASE GAMMA) (DEOXYRIBONUCLEASE I-LIKE 3) (DNASE I HOMOLOGOUS PROTEIN DHP2) (LIVER AND
SPLEEN DNASE) (LS-DNASE) (LSD).// 5.40E-143// 267aa// 99%// Q13609 STOMA2004893
SYNOV1000256// Human preferentially expressed antigen of melanoma (PRAME) mRNA, complete cds.// 2.80E-29// 275aa// 35%// U65011
SYNOV2001660
SYNOV2006620
SYNOV2013637// Homo sapiens putative renal organic anion transporter 1 (hROATI) mRNA, complete cds.// 2.40E-54// 213aa// 53%// AF057039
SYNOV2021953// hematopoietic zinc finger [Mus musculus]// 1.00E-63// 300aa// 51%// NP_038894
SYNOV4002744// Zinc finger protein 84 (Zinc finger protein HPF2).// 2.00E-26// 75aa// 30%// P51523
SYNOV4003981// megakaryocyte stimulating factor [Homo sapiens]// 0// 400aa// 74%// NM_005807
SYNOV4005739
SYNOV4005889
TBAES2000932// sirtuin 7; sirtuin type 7; silent mating type information regulation 2, S.cerevisiae, homolog 7; sir2-related protein type 7 [Homo sapiens]// 1.00E-69// 120aa// 93%// NM_016538
TESOP2000390// Mitogen-activated protein kinase kinase kinase 14 (EC 2.7.1.37) (NF- kappa beta-inducing kinase) (Serine/threonine protein kinase NIK) (HsNIK).// 0// 354aa// 81%// Q99558 TESOP2001796// RING finger protein 19 (Dorfin) (Double ring-finger protein). (p38 protein).// 0// 320aa// 69%// Q9NV58
TESOP2005199// ZINC FINGER PROTEIN 7 (ZINC FINGER PROTEIN KOX4) (ZINC FINGER PROTEIN HF.16).// 8.40E-104// 317aa// 49%// P17097
TESOP2006398// zinc finger-like; similar to P52742 (PID:g1731411) [Homo sapiens].// 4.00E-22// 100aa// 97%// AAC36300
TESOP2006865// ZINC FINGER PROTEIN MFG-3.// 5.30E-29// 116aa// 55%// P16374
TESOP2007384// Mus musculus putative purine nucleotide binding protein mRNA, complete cds.// 1.00E-78// 288aa// 55%// U44731
TESTI1000266
TESTI2008901
TESTI2015626// Human mRNA for phosphoribosypyrophosphate synthetase-associated protein 39, complete cds.// 1.00E-181// 356aa// 99%// D61391
TESTI2025924// CASEIN KINASE I, EPSILON ISOFORM (EC 2.7.1.-) (CKI-EPSILON).// 1.80E-42// 265aa// 36%// P49674
TESTI2026647// PROBABLE ATP-DEPENDENT RNA HELICASE DDX10 (DEAH BOX PROTEIN 10).// 6.40E-48// 226aa// 45%// Q13206
TESTI2029252// Homo sapiens mRNA for LAK-4p, complete cds.// 2.40E-79// 410aa// 38%// AB002405
TESTI2032643// ADENYLATE CYCLASE, TYPE IV (EC 4.6.1.1) (ATP PYROPHOSPHATE-LYASE) (ADENYLYL CYCLASE).// 1.00E-176// 155aa// 75%// P26770
TESTI2034251// ZINC FINGER PROTEIN 91 (ZINC FINGER PROTEIN HTF10) (HPF7).// 3.10E-186// 546aa// 57%// Q05481
TESTI2035981
TESTI2036288// PROSTAGLANDIN-F SYNTHASE 1 (EC 1.1.1.188) (PGF SYNTHASE 1) (PGF 1) (PROSTAGLANDIN-D2 11 REDUCTASE 1) (PGFSI).// 1.10E-41// 120aa// 69%// P05980
TESTI2037830
TESTI2039060// MALTASE-GLUCOAMYLASE, INTESTINAL [INCLUDES: MALTASE (EC 3.2.1.20) (ALPHA-GLUCOSIDASE); GLUCOAMYLASE (EC 3.2.1.3) (GLUCAN 1,4-ALPHA-GLUCOSIDASE)].// 8.60E-287// 641aa// 77%// 043451
TESTI2049956// Vegetatible incompatibility protein HET-E-1.// 5.00E-43// 107aa// 34%// Q00808
TESTI2050780// organic anion transporter OATP-E [Homo sapiens]// 4.00E-28// 71aa// 34%// NM_016354
TESTI4000137
TESTI4000155
TESTI4000183// ZINC FINGER PROTEIN 177.// 4.60E-66// 299aa// 43%// Q13360
TESTI4000214// similar to human transcription factor TFIIS (S34159)// 7.00E-40// 400aa// 32%// BAA13438
TESTI4000319// GUANINE NUCLEOTIDE DISSOCIATION STIMULATOR RALGDS FORM B (RALGEF).// 4.70E-50// 256aa// 48%// Q03386
TESTI4001984
TESTI4005317// Homo sapiens rab6 GTPase activating protein (GAP and centrosome-associated) (GAPCENA), mRNA// 0// 536aa// 95%// NM_012197
TESTI4006473// ATP-dependent RNA helicase A (Nuclear DNA helicase II) (NDH II) (DEAD-box protein 9).// 5.00E-72// 208aa// 33%// Q28141
TESTI4008058
TESTI4008302// zinc finger protein [Homo sapiens]// 0// 382aa// 89%// NM_014347
TESTI4010382// cytoplasmic dynein heavy chain 2 [Rattus norvegicus]// 0// 1171aa// 90%// NM_023024
TESTI4011070// Glucoamylase S1/S2 precursor (EC 3.2.1.3) (Glucan 1,4-alpha-glucosidase) (1,4-alpha-D-glucan glucohydrolase).// 5.00E-18// 148aa// 20%// P08640
TESTI4011072// tudor domain containing 1 [Mus musculus]// 1.00E-38// 201aa// 22%// NM_031387
TESTI4011829
TESTI4013365// S-antigen protein precursor.// 6.00E-07// 109aa// 20%// Q03400
TESTI4013602
TESTI4013894// mitsugumin 29 [Mus musculus]// 1.00E-117// 200aa// 92%// NM_008596
TESTI4014801// novel protein similar to archaeal, yeast and worm N2, N2-dimethylguanosine tRNA methyltransferase [Homo sapiens]// 0// 312aa// 89%// NM_030934
TESTI4015012
TESTI4015442// zinc finger homeodomain 4 [Mus musculus]// 0// 875aa// 79%// NM_030708
TESTI4017714// Protease II (EC 3.4.21.83) (Oligopeptidase B).// 4.00E-52// 175aa// 25%// Q59536
TESTI4019657// cyclin G associated kinase [Homo sapiens]// 1.00E-138// 311aa// 88%// NP_005246
TESTI4021482// RAC-alpha serine/threonine kinase (EC 2.7.1.-) (RAC-PK-alpha) (Protein kinase B) (PKB) (C-AKT).// 3.00E-28// 58aa// 98%// P31749
TESTI4024387// RAB GDP dissociation inhibitor alpha (RAB GDI alpha) (GDI-1).// 1.00E-102// 179aa// 98%// P50398
TESTI4025268// 77 kDa echinoderm microtubule-associated protein.// 1.00E-45// 119aa// 31%// Q26613
TESTI4025494// Zinc finger protein 33A (Zinc finger protein KOX31) (HA0946).// 0// 384aa// 49%// Q06730
TESTI4025547// double-stranded RNA-binding zinc finger protein JAZ [Homo sapiens]// 1.00E-161// 279aa// 87%// NM_012279
TESTI4025865
TESTI4026207// Kinesin-like protein KLP1.// 1.00E-83// 167aa// 43%// P46870
TESTI4028938// Zinc finger protein 85 (Zinc finger protein HPF4) (HTF1).// 0// 373aa// 66%// Q03923
TESTI4028958
TESTI4029348
TESTI4029528
TESTI4029690
TESTI4031745// Mitogen-activated protein kinase kinase kinase 5 (EC 2.7.1.-) (MAPK/ERK kinase kinase 5) (MEK kinase 5) (MEKK 5) (Apoptosis signal-regulating kinase 1) (ASK-1).// 0// 447aa// 58%// Q99683
TESTI4032090
TESTI4032112// Homo sapiens general transcription factor IIIC, polypeptide 1 (alpha subunit, 220kD ) (GTF3C1), mRNA// 1.00E-121// 213aa// 82%// NM_001520
TESTI4036767
TESTI4038721
TESTI4041086
TESTI4046240// sirtuin 7// 1.00E-115// 44aa// 98%// NP_057622
THYMU2004139// WEE1-LIKE PROTEIN KINASE (EG 2.7.1.112).// 2.80E-137// 534aa// 54%// P47817
THYMU2004284// UBIQUITIN-ACTIVATING ENZYME E1 (A1S9 PROTEIN).// 9.00E-63// 122aa// 100%// P22314
THYMU2006001// ZINC-BINDING PROTEIN A33.// 8.40E-51// 476aa// 27%// Q02084
THYMU2028739// ZINC FINGER PROTEIN 263 (ZINC FINGER PROTEIN FPM315).// 1.10E-39// 291aa// 40%// 014978
THYMU2030462
THYMU2031139// Homo sapiens zinc metalloprotease ADAMTS7 (ADAMTS7) mRNA, complete cds.// 3.30E-105// 504aa// 42%// AF140675
THYMU2031249// SERINE/THREONINE-PROTEIN KINASE PCTAIRE-1 (EC 2.7.1.-).// 1.20E-138// 291aa// 92%// Q00536
THYMU2032976
THYMU2033401
THYMU2034279
THYMU2035078// LRP16 protein [Homo sapiens]// 3.00E-50// 80aa// 55%// NP_054786
THYMU2035710// Tyrosine-protein kinase-like 7 [Precursor]// 1.00E-50// 121aa// 80%// Q13308
THYMU2040925// CDP-DIACYLGLYCEROL-INOSITOL 3-PHOSPHATIDYLTRANSFERASE (EC 2.7.8.11) (PHOSPHATIDYLINOSITOL SYNTHASE) (PTDINS SYNTHASE) (PI SYNTHASE).// 4.60E-50// 118aa// 86%// 014735
THYMU3000269// SUCCINATE DEHYDROGENASE [UBIQUINONE] FLAVOPROTEIN SUBUNIT, MITOCHONDRIAL PRECURSOR (EC 1.3.5.1) (FP) (FLAVOPROTEIN SUBUNIT OF COMPLEX II).// 2.80E-130// 245aa// 99%// P31040
THYMU3000360
THYMU3001428
TKIDN2008778
TKIDN2012771// DPY-19 PROTEIN.// 5.30E-49// 314aa// 35%// P34413
TKIDN2018926
TLIVE2001684// complement component 3 precursor [Homo sapiens]// 5.00E-59// 110aa// 32%// NP_000055
TLIVE2002046// DELTA-AMINOLEVULINIC ACID DEHYDRATASE (EC 4.2.1.24) (PORPHOBILINOGEN SYNTHASE) (ALADH).// 3.10E-156// 317aa// 93%// P13716
TLIVE2007607// CYTOCHROME P450 4A4 (EC 1.14.14.1) (CYPIVA4) (PROSTAGLANDIN OMEGA- HYDROXYLASE) (P450-P-2).// 6.90E-127// 448aa// 49%// P10611
TRACH1000212
TRACH2000862// Mus musculus putative purine nucleotide binding protein mRNA, complete cds.// 5.40E-224// 619aa// 68%// U44731
TRACH2007483// GASTRULA ZINC FINGER PROTEIN XLCGF7.1 (FRAGMENT).// 6.30E-12// 98aa// 37%// P18735
TRACH2019672// Rattus norvegicus mRNA for 45 kDa secretory protein, partial.// 5.80E-163// 303aa// 96%// AJ132352
TRACH2024408// ankyrin 2, isoform 2; ankyrin-2, nonerythrocytic; ankyrin-B; ankyrin, brain; ankyrin, neuronal; ankyrin, nonerythroid [Homo sapiens]// 1.00E-06// 119aa// 20%// NM_020977
TRACH2024559// Homo sapiens myosin 5c (MY05C) mRNA, complete cds.// 1.70E-59// 151aa// 86%// AF272390
TRACH3000134// ZINC FINGER PROTEIN 84 (ZINC FINGER PROTEIN HPF2).// 2.30E-215// 733aa// 53%// P51523
TRACH3000420// Homo sapiens ATP-binding cassette transporter family A member 12 (ABCA12) mRNA, complete cds.// 7.00E-253// 745aa// 41%// AY033486
TRACH3002561// UNR protein.// 2.00E-34// 66aa// 95%// P18395
TRACH3003683// Lactoperoxidase precursor (EC 1.11.1.7) (LPO) (Salivary peroxidase) (SPO).// 1.00E-39// 77aa// 86%// P22079
TRACH3003832// PHD finger protein 2 [Mus musculus]// 1.00E-153// 321aa// 39%// NM_011078
TRACH3007866// dipeptidyl peptidase 8: hypothetical protein FLJ20283 [Homo sapiens]// 1.00E-105// 172aa// 53%// NM_017743
TUTER2000057// Mus musculus AE-1 binding protein AEBP2 mRNA, complete cds.// 1.10E-26// 60aa// 93%// AF090326
UTERU2004299
UTERU2008040// Mus musculus mRNA for stac, complete cds.// 3.30E-82// 405aa// 47%// D86639
UTERU2011220
UTERU2019534// Golgi apparatus protein 1 [Homo sapiens]// 5.10E-61// 118aa// 100%// XP_012515
UTERU2021820// dipeptidylpeptidase VI [Homo sapiens]// 8.00E-23// 50aa// 89%// NP_001927
UTERU2028734// Mus musculus slp2-a mRNA for synaptotagmin-like protein 2-a delta 2S-III, complete cds.// 2.30E-216// 464aa// 86%// AB057762
UTERU2032279// 47 KDA HEAT SHOCK PROTEIN PRECURSOR (COLLAGEN-BINDING PROTEIN 1) (COLLIGIN 1).// 6.90E-95// 207aa// 93%// P29043
UTERU2033577// Homo sapiens mRNA for repressor protein, partial cds.// 1.80E-65// 209aa// 65%// D30612
UTERU2035978
UTERU3000402
UTERU3000738// Beta-adrenergic receptor kinase 1 (EC 2.7.1.126) (Beta-ARK-1) (G- protein coupled receptor kinase 2).// 5.00E-76// 139aa// 94%// P26817
UTERU3001053// zinc finger protein [Homo sapiens]// 0// 319aa// 91%// NM_018651
UTERU3014791
UTERU3015069
UTERU3015412
UTERU3017176
TESTI4038779// zinc finger protein RIN ZF [Rattus norvegicus]// 3.00E-73// 190aa// 36%// NM_024489

## Claims

1. A polynucleotide selected from the group consisting of:
(a) a polynucleotide comprising a protein-coding region of the nucleotide sequence according to any one of SEQ ID NOs: 1-2188 and SEQ ID NOs: 4377-4683;
(b) a polynucleotide comprising the nucleotide sequence encoding a polypeptide that comprises the amino acid sequence of any one of SEQ ID NOs: 2189-4376 and SEQ ID NOs: 4684-4990;
(c) a polynucleotide comprising a nucleotide sequence encoding a polypeptide, which comprises the amino acid sequence selected from SEQ ID NO: SEQ ID NOs: 2189-4376 and SEQ ID NOs: 4684-4990 wherein one or more amino acids have been substituted, deleted, inserted, and/or added, and which is functionally equivalent to the polypeptide comprising the selected amino acid sequence as described above;
(d) a polynucleotide which hybridizes to a polynucleotide comprising the nucleotide sequence selected from SEQ ID NOs: 1-2188 and SEQ ID NOs: 4377-4683, and which comprises the nucleotide sequence encoding a polypeptide functionally equivalent to a polypeptide encoded by the selected nucleotide sequence as described above;
(e) a polynucleotide comprising a nucleotide sequence encoding a partial amino acid sequence of a polypeptide encoded by the polynucleotides according to any one of (a)-(d);
(f) a polynucleotide comprising a nucleotide sequence having at least 70% identity to the nucleotide sequence of any one of SEQ ID NOs: 1-2188 and SEQ ID NOs: 4377-4683; and
(g) a polynucleotide comprising a nucleotide sequence having at least 90% identity to the nucleotide sequence of any one of SEQ ID NOs: 1-2188 and SEQ ID NOs: 4377-4683.

2. A polypeptide encoded by the polynucleotide according to claim 1, or a partial peptide thereof.

3. An antibody which binds to the polypeptide or the peptide according to claim 2.

4. An immunoassay method for the polypeptide or the peptide according to claim 2, which comprises the steps of:
(a) contacting the polypeptide or the peptide according to claim 2 with the antibody according to claim 3; and
(b) observing the binding between the two.

5. A vector comprising the polynucleotide according to claim 1.

6. A transformant comprising the polynucleotide according to claim 1 or the vector according to claim 5.

7. A transformant which comprises the polynucleotide according to claim 1 or vector according to claim 5 in an expressible manner.

8. A method for producing the polypeptide or the peptide according to claim 2, which comprises the steps of:
(a) culturing the transformant according to claim 7; and
(b) recovering the expression product.

9. An oligonucleotide comprising 15 or more nucleotides, which comprises the nucleotide sequence according to any one of SEQ ID NOs: 1-2188 and SEQ ID NOs: 4377-4683, or a nucleotide sequence complementary to the complementary strand thereof.

10. A primer for synthesizing a polynucleotide, which comprises the oligonucleotide according to claim 9.

11. A probe for detecting a polynucleotide, which comprises the oligonucleotide according to claim 9.

12. A polynucleotide according to any one of:
(a) an antisense polynucleotide comprising a nucleotide sequence complementary to the transcript of the polynucleotide according to claim 1;
(b) a polynucleotide with the ribozyme activity for specifically cleaving the transcript of the polynucleotide according to claim 1; and
(c) a polynucleotide which downregulates the expression of the polynucleotide of claim 1 due to RNAi activity in a host cell.

13. A method for detecting the polynucleotide according to claim 1, which comprises the steps of:
(a) incubating a target polynucleotide with the oligonucleotide according to claim 9 under conditions ensuring hybridization; and
(b) detecting the hybridization between the target polynucleotide and the oligonucleotide according to claim 9.

14. A database of polynucleotides and/or polypeptides, which comprises information on at least one of the nucleotide sequences of SEQ ID NOs: 1-2188 and SEQ ID NOs: 4377-4683 and/or on at least one of the amino acid sequences of SEQ ID NOs: 2189-4376 and SEQ ID NOs: 4684-4990.
